# EUROPEAN PATENT APPLICATION

(11) **EP 1 447 413 A2**
(43) Date of publication of application: **18.08.2004**
(21) Application number: 04003145.2
(22) Date of filing: 12.02.2004
(51) Int. Cl.: C07K 14/47, C07K 14/705, C07K 16/18, G01N 33/53, C12Q 1/68, C12N 15/11, C12N 15/12, C12N 9/00, C12P 21/00, G06F 19/00

(54) **Full-length human cDNA**

(30) Priority: 14.02.2003 JP 2003102207; 09.05.2003 JP 2003131452
(71) Applicant: Research Association for Biotechnology, Tokyo 105-0003 (JP)
(72) Inventor: Isogai, Takao, Inashiki-gun Ibaraki 300-0303 (JP); Yamamoto, Junichi, Kisarazu-shi Chiba 292-0014 (JP); Nishikawa, Tetsuo, Tokyo 173-0013 (JP); Isono, Yuko, Kisarazu-shi Chiba 292-0014 (JP); Sugiyama, Tomoyasu, Tokyo 130-0003 (JP); Otsuki, Tetsuji, Nishitokyo-shi Tokyo 188-0001 (JP); Wakamatsu, Ai, Kisarazu-shi Chiba 292-0014 (JP); Ishii, Shizuko, Shiraoi-gun Hokkaido 059-0914 (JP); Nagai, Keiichi, Higashiyamato-shi Tokyo 207-0022 (JP); Irie, Ryotaro, Saitama-shi Saitama 336-0936 (JP)
(74) Representative: VOSSIUS & PARTNER

(57) **Abstract**

Novel full-length cDNAs are provided.
1,995 cDNA derived from human have been isolated. The full-length nucleotide sequences of the cDNA and amino acid sequences encoded by the nucleotide sequences have been determined. Because the cDNA of the present invention are full-length and contain the translation start site, they provide information useful for analyzing the functions of the polypeptide.

SEQUENCE LISTING

<110> Research Association for Biotechnology

<120> Full length cDNA

<130> BTR-A0301Y1

<150> JP 2003-102207
<151> 2003-02-14

<150> JP 2003-131452
<151> 2003-05-09

<160> 3994

<170> PatentIn Ver. 3.1

<210> 1
<211> 1437
<212> DNA
<213> Homo sapiens

<400> 1
acccgggggg ccgcgctgcg cggagtgcca ggctgcgggc ggctgcagac ctgggagcgg 60
agaccggccc gccgcccccg acgccgccga gcacgtcagc ggcgcgcagc cggggctcgg 120
agaccgacgg gcagaacgac gggcggcgac tgcggcgacc gcgggacggc gagaggcacg 180
cggcgggagg ggaccggaat ccgcagctcc ggccgcgcca tggacggcaa cgacaacgtg 240
accctgctct tcgcccctct gctgcgggac aactacaccc tggcgcccaa tgccagcagc 300
ctgggccccg gcacggacct cgccctcgcc cctgcctcca gcgccggccc cggccctggg 360
ctcagcctcg ggccgggtcc gagcttcggc ttcagccccg gccccactcc gaccccggag 420
cccacgacca gcggcctcgc gggcggcgcg gcgagccacg gcccttcccc gttccctcgg 480
ccctgggcgc cccacgcgct cccgttctgg gacacgccgc tgaaccacgg gctgaacgtg 540
ttcgtgggcg ccgccctgtg catcaccatg ctgggcctgg gctgcacggt ggacgtgaac 600
cacttcgggg cgcacgtccg tcggcccgtg ggcgcgctgc tggcagcgct ctgccagttc 660
ggcctcctgc cgctgctggc cttcctgctg gccctcgcct tcaagctgga cgaggtggcc 720

gccgtggcgg tgctcctgtg tggctgctgt cccggcggca atctctccaa tcttatgtcc 780
ctgctggttg acggcgacat gaacctcagc atcatcatga ccatctcctc cacgcttctg 840
gccctcgtct tgatgcccct gtgcctgtgg atctacagct gggcttggat caacacccct 900
atcgtgcagt tactacccct agggaccgtg accctgactc tctgcagcac tctcatacct 960
atcgggttgg gcgtcttcat tcgctacaaa tacagccggg tggctgacta cattgtgaag 1020
gtttccctgt ggtctctgct agtgactctg gtggtccttt tcataatgac cggcactatg 1080
ttaggacctg aactgctggc aagtatccct gcagctgttt atgtgatagc aatttttatg 1140
cctttggcag gctacgcttc aggttatggt ttagctactc tcttccatct tccacccaac 1200
tgcaagagga ctgtatgtct ggaaacaggt agtcagaatg tgcagctctg tacagccatt 1260
ctaaaactgg cctttccacc gcaattcata ggaagcatgt acatgtttcc tttgctgtat 1320
gcacttttcc agtctgcaga agcggggatt tttgttttaa tctataaaat gtatggaagt 1380
gaaatgttgc acaagcgaga tcctctagat gaagatgaag atacagatat ttcttat 1437

<210> 2
<211> 1582
<212> DNA
<213> Homo sapiens

<400> 2
aacaaccaca tccctcctca gaagccccca gagcacaacg cctcaccatg gactgggcct 60
ggaggatcct ctttttggtg gcagcggcca caggtgtcca ctcccaggcc cagcttgtgc 120
agtctggggc tgaggcgaag aagcctgggg cctccgtgaa aatttcctgc aaggcttctg 180
gatacccctt cagtggctat gttttacact ggctgcgaca ggcccccgga caaggacttg 240
agtggctggg aagcatcacc gctggctacg atgccacaaa atattcacag aggttccagg 300
acagaatcac cattaccagg gacacatccg cgagcacagt ttacttggaa tggagcagcc 360
tgacatccga cgacacggct gtcctttact gtgcgaggga gggtgatgag gactacggtg 420
actctcttgg cgcttttgat gtctggggcc aagggacact ggtcaccgtc tctccagcat 480
ccccgaccag ccccaaggtc ttcccgctga gcctctgcag cacccagcca gatgggaacg 540
tggtcatcgc ctgcctggtc cagggcttct tcccccagga gccactcagt gtgacctgga 600
gcgaaagcgg acagggcgtg accgccagaa acttcccacc cagccaggat gcctccgggg 660
acctgtacac cacgagcagc cagctgaccc tgccggccac acagtgccta gccggcaagt 720
ccgtgacatg ccacgtgaag cactacacga atcccagcca ggatgtgact gtgccctgcc 780
cagttccctc aactccacct accccatctc cctcaactcc acctacccca tctccctcat 840
gctgccaccc ccgactgtca ctgcaccgac cggccctcga ggacctgctc ttaggttcag 900
aagcgaacct cacgtgcaca ctgaccggcc tgagagatgc ctcaggtgtc accttcacct 960

ggacgccctc aagtgggaag agcgctgttc aaggaccacc tgaccgtgac ctctgtggct 1020
gctacagcgt gtccagtgtc ctgccgggct gtgccgagcc atggaaccat gggaagacct 1080
tcacttgcac tgctgcctac cccgagtcca agaccccgct aaccgccacc ctctcaaaat 1140
ccggaaacac attccggccc gaggtccacc tgctgccgcc gccgtcggag gagctggccc 1200
tgaacgagct ggtgacgctg acgtgcctgg cacgtggctt cagccccaag gatgtgctgg 1260
ttcgctggct gcaggggtca caggagctgc cccgcgagaa gtacctgact tgggcatccc 1320
ggcaggagcc cagccagggc accaccacct tcgctgtgac cagcatactg cgcgtggcag 1380
ccgaggactg gaagaagggg gacaccttct cctgcatggt gggccacgag gccctgccgc 1440
tggccttcac acagaagacc atcgaccgct tggcgggtaa acccacccat gtcaatgtgt 1500
ctgttgtcat ggcggaggtg gacggcacct gctactgagc cgcccgcctg tccccacccc 1560
tgaataaact ccatgctccc cc 1582

<210> 3
<211> 2034
<212> DNA
<213> Homo sapiens

<400> 3
   cactcgctcc cagattaggt cgctctgggg ctatcaggcc agcatggacg ctggcaaagc 60
   ggggcagacc ctgaagactc actgctcagc ccaggtgaga gccttctgag ctgtgcctgg 120
   ggctgggggg acagggccct ggaaccccag cagccggcgg gtctgggagc tgctgtcctt 180
   gagttggttg ggccctggga agctgggttc tggcagcctg gtccctgaat gggtccagcc 240
   tgtacagctg ggtcccaggg tgggggtggg acaggagcca tgagatctga gtcccaagaa 300
   tgggggctgg gctgggtttc ggctcagtag ctccccactc cccaaagcgc ccagatgtct 360
   gcaggtggct gagccccttc atcctctcct gctgcgtgta cttctgcctc tggattcccg 420
   aggaccagct gtcctggttc gctgccctgg tcaagtgcct gcccgtcctc tgcctggctg 480
   ggttcctgtg ggtcatgtcc ccaagcgggg gctacaccca gctcctccag ggagcccttg 540
   tgtgctcggc tgtgggggac gcttgcctca tctggccggc agccttcgtc cctggtgagt 600
   ggaaggtgct gtgacgcttt ccagatgccc gtgtgagaga ggccccagca cttggatggg 660
   agcctgggtg cagagggctc ctgtgagagc tccagtggtc aaaggcacag tggggggcgc 720
   agccgtgacc gaggcagctc acgctccagc gaggggtggg ggcaggcggt gttagggcag 780
   tagctgaggt ggcgcagagg gcagtgagga cccgctggag gtactgcagt ttgaagcagg 840
   gttgctgagg aaggtctcac tgaggaagtc acatttgggc agagctgaag ggctgtatgc 900
   ctgtgtggag gaagggcgag tgtaaaggct ccggggcagg gacgtgtcag gtgcttgtag 960
   cccagcaggc cagggtggct ggcgcagagt ggctggagag ctggaggcca tggggctttg 1020
   gaggccaagg gaaggtgggg cctcctggca gttctgagca gagcaaggat ggacactgca 1080
   tgtggggaga cctgcaagga ggctcggcca gggtggggcc tggtgctctg ggatggtcct 1140
   tctcttaggc cctgaggggt gacctcgggg ccattcccag gatgggcctt cctcactggt 1200
   accccacccc tctcccgcca ggcatggccg cctttgccac cgcccacctc ctctacgtct 1260
   gggccttcgg cttctctccc ctgcagcccg gcctgctgct gctcatcatc ctggcccctg 1320
   gcccctacct cagccttgtg ctccagcacc tcgagccgga tatggtcctg ccggtggcag 1380
   cctatgggct gatcctgatg gccatgctgt ggcgcggcct ggcccagggc gggagtgccg 1440
   gctggggcgc gctgctcttc acgctctctg atggcgtgct ggcctgggac accttcgccc 1500
   agcccctgcc ccatgcccac ctggtgatca tgaccaccta ctatgctgcc cagctcctca 1560
   tcacactgtc agccctcagg agcccggtgc ccaagactga ctgactaggg agcttgaagg 1620
   gccggtgttc aggccctctc ctcctgcaag gacctgggcc tcccagccca gcccagcctg 1680
   agaaataccc tcagcagcga agcttcctga cgcctgtctg caggcgccgc tgccgccgtc 1740
   gcttctggct gaagacgttt gaggacgatt tgcggaattc caagtccact actgggttcc 1800
   agctgccttc ccccggttct gactccagat ccctggctcc tcagccaggc ccacatggag 1860
   ccctcccagc caccagcctg cctccatgtt cactgtcggc cccacagcct gcccgccccc 1920
   tgctgctgct ctgaatccgt ttccctgtgg ggtgtggaac cgtagatgtt gctgttaccg 1980
   taggagaggc ctcggggagg gtcatgattg tgataaacca tcgcggttaa tgac 2034

<210> 4
<211> 2312
<212> DNA
<213> Homo sapiens

<400> 4
tttacagaat tcttcctatc aaagattatt aaaatttagg cctaaaggga aaccatgccc 60
caaggaaatt ccaaaagaat caaaagaccc agaagtctta gtttgggaag aatgtgtggc 120
tgatactgca gtggtactac aaaacaataa atttggaact attatagact aggccccttg 180
aggccaatta tattatgact gtatgggcca gacccactca tgttcacagg ctccatctgt 240
ctggcccact aatccggcct atgttagtga tttaactaaa aggctagacc aggtttatag 300
aaggctagaa tcaccctatc catggaaatg gggtgaaaag aggatttcat caccccgacc 360
aaagttagtt agtcctgttg ttggtcctga acacccagaa ttatggaagc tcactgtggc 420
ctcataccac attagaattt ggtctggaaa tcaagttatg ggaacaagaa atcataagcc 480
atattatact attaacctaa attacaatct gaaaattcct ttgcaaagtt gtgtaaaacc 540
accttatatg ctagttgtag gaaacatagc tattaaacca gattcccaaa ctacaaccag 600
tgaaaattgt agattgttta cttgcattga ttcaactttt gattggcaga atggtactct 660

gtaagagcaa gagaaggcgt gtggatccct gtgtccatgg atcgaccgtg ggaggcttct 720
ccatccgtac atatcttaag tattaaaagg agttctaact agatctaaaa gattcatttt 780
tactttgatt gcagtgatta tgggtcttat tgcagtcaca gctactgctg cacctgctgg 840
aattgcttta cactcctctg ttcaaactgc agaatatgtg aataattggc aaaagaattc 900
ctcaaaattg tggaattctt agactcaaat agatcaaaaa ttgacaaatc aaattaatga 960
tcttagacaa actgttattt ggatgagaga taggctcatg agcttagaat atcttttca 1020
gttacagtgt gactggaata catcagattt ttgtattaaa cctcgagcct ataatgaatc 1080
tgaacatcac tgggacatgg ttagacgcca tctataagga aaagaagata atcttacctt 1140
agatatttct aaattgaaag aacaaatttt tgaaacatca aaagcccagt taaatctggt 1200
gtcagaaacg gaggcaatgg taaaagctgt tgacagcctc acaaatctta accctgtcac 1260
ttgggttaaa accattggaa attccactat tgcaaatttt gtattaattc ttgtatgtct 1320
gtcctctcta ttgttagtct acaggtatat ccagcagctc cggagagaca gcgaccagcg 1380
agaaggggcc atgatgacga tggcggtttt gtcaaaaaga aaagggggaa atgtagggaa 1440
aagcgagaga tcagactgtc actgtgtcta tgtagaaagg gaagacataa gagactccat 1500
tttgaaaaag atctgtactc taacaattgc tttgctgaga tgttgttcgt ttgtagcttt 1560
gccccagcca ctttgcctca gtcactttga cccaacttgg agttcacaaa aacatgtgtt 1620
gtataaaatc aaggtttaag ggatctaggg ctgtgcagga cgtgccttgt taaccaaatg 1680
tttacaagca gtatacttgg taaaagtcat tgccattctc tagtcacaat aaaccagggg 1740
cacaatgcac cgtggaaagc cgcagggagc cctgcccttg aaagcagtgt attgtccaag 1800
gtttctcccc atgtgatagt ctgaaatatg gcctcgtggg atgagaaaga cctgactgtc 1860
ccccagcctg acacccgtaa agcgtctgcg ctgaggtgga ttagtaaaag aggaaagcct 1920
cttgcagttg agatggagga aggccactgt ctcctgcttg cccctgggaa ttgaatgtct 1980
cggtgtaaac ctgattgtac atttgttcaa gtctgagctc ggagaaaagc tgccctgtgg 2040
cgggaggcga gacatgttgc agtaatgctg ccttgttatt ctttactcca ctgagatatt 2100
tgggtggaga gaaacataaa tctggcctac gtgcaagtcc agtcatagta ccttcccttg 2160
aacttaatta tgatatagat tcttttgctc acatgttttt tgttgacctt ctccttatta 2220
tcaccctgtt ctcctattac attccttttt gctgaaataa tgaaaatcat aatcaataaa 2280
aactgaggga actcagaggc cagttcccct gc 2312

<210> 5
<211> 2547
<212> DNA
<213> Homo sapiens

<400> 5

agaagctgaa attgtcaacc acaattatgt aaaagtttcc ctagggaaaa aaaaaaagaa 60

atatccaact cttcctggaa cagggaagaa ggaagggaag aaggaaagca gaaagagagg 120
gaatccacga aagcatgaag tcttgggaaa actttgcttt cagagtctcc tcgctctgtt 180
gccagtctgg agtacagtgg cacgatctcg gcccactgca acctccgcct accaggttca 240
agcgattatc ctgcctcagc ttcccaagta gctgggatta caagtgcaca ctaccacacc 300
cagatgttcc tgcaccattt gttgaagact gttctctccc tgttgaattg tcctggcact 360
cctgtcaaaa gcaatttacc agaattgtaa gggtttattt ttggactcta cccctctatc 420
ttgtctcaaa tgttactgat aaagtgaaag actgaaggga gaaaaaaatg aatccaggtg 480
gtggaaattc tccatgtgag ttagcacaag tgatgagctt tgcttcccat tccctgtagt 540
gtttggggag ctcttcattg acaggcatca gctaatggaa gtagtacttg aatgttacca 600
atgtcctcct acaagtcaaa ttaagttgtc ctttatttca gtaatgtccc ctgacatttg 660
gcattgctat ccacttctag aaacttggct ttgttctcct tttacctctg taattcttct 720
tcagtctact tcaccatctt actttttctt ctaactatcc ataccttaaa agttggtttt 780
ccacagagat ttatcttcat acttctcttt atatttaaag acttctggtt gccatacaat 840
atccattctt atgcattgtg cacaacagaa gatcaacttt tatctggaca cattgcttca 900
gaactaaatg atgacatgtc ccaacctccc ttgcatgttt ttattgccat gtgtcttaac 960
tctggatagt aatatggaag caaaagtgtt gtatgaatct ggaaaatgtc ttaattggtt 1020
agggaatgca ttcttgtttt gctcttcctg ttgctggtaa cctggaacac agtggggatg 1080
gctggagacc cagttaagtc atgatccttt tggaattttt ttaattatac gtagcaaacc 1140
taattataat tgagaaacca tcaaaggcta tgtattcatg tatcgttgaa ccaatattta 1200
ttaagcatct actacctatc atgtactgtg acaggtatca tggtgatgtg atgctgtcct 1260
caggaagtcc acagcctata gggaaataca taaatgtata cacacataaa acccttaatg 1320
gtaattttta taataatatt attatagaaa atatgtggtg tgccagggta atacaaggaa 1380
taaaacaatt aatcctgtca aggttttata aagctttgtg aggaagcaat atttgtattg 1440
agactcgata gactttgtaa ataaaagagg aaaaccatta tagataatgt agggtttaca 1500
aatgtatgga ttgtacagct gtgaatgttc atgattagtt taaaaatgca ttattagtta 1560
tgtctagaga aaaggctgtt atgtgtgtgt gacagagaga gagagagaga gagacggaga 1620
tgaaaagagg gagggaaggg agaagacaga gacatacagg agagagatcc tgggaagcag 1680
caagtgatca gagaggaaca gcaacagaga gtgttcccag gaaataggaa tttggggtat 1740
tgcatgtaga attgagaatg tatgtctcag cttatgaatc agagatagaa aatatgtttg 1800
aatccttatg tcaactctaa ttttgcctgc ctaaagtact acattgagaa gaatcctgaa 1860
tttacacctg agctcaggaa agagtgacat aaagtattag aaatgctggc atgacatcta 1920
cagccatatc tccctgtagg tggctgatct tatctgatct cagaaaccaa gtagagctgg 1980
gcctggttag tacttggatg aaatgtctgc atgagtttgg gacatgtggt ggttaatttt 2040
atgtgtcaac ttggctgagc cacagtgatc gggtatgtga tcaaacatta ttctagatgt 2100

ctctctgagg atatttttgg acgggattaa catttaaact tatggacttt gagtaaaact 2160
ggtagtcgat cacaatgtga gtgacctcac caatgagttg aagccccaag taaaataaaa 2220
gaatgacctc cctgaacaag gcagaattct gcatctcatg gcctttgaac ttgaactgca 2280
gtatcaaccc gtgtctccag ctgatgtcct ttgaatttga actgcagcat tggcttttct 2340
ccaagtctcc agcctattgg cccaacccac agattttgaa cttaccaccc ttcataacca 2400
catgacacaa tttcttaaaa taaatctctc tattgctctc tctctatata tatatcctat 2460
tggttctgtt tctctagaga accccaagta acaccaacgg attgaatatt cacatgtgag 2520
gaattaaaaa gagttttgaa ccttggc 2547

<210> 6
<211> 3601
<212> DNA
<213> Homo sapiens

<400> 6
tccagcacag aatggaattc agccaccaat cagtaactca aggacagatg aaagagaatt 60
ctttctcgcc tcttataaca gaaagaaaga ggatggagag ggcaacgttt ggattgcaaa 120
gtcatcagcc ggtgccaaag gtgaaggcat tctcatctcc tcagaggctt cagagcttct 180
cgatttcata gacaaccagg gccaagtgca cgtgatccag aaatatcttg agcaccctct 240
gctgcttgag ccaggtcatc gcaagtttga catccgaagc tgggtcttgg tggatcatca 300
gtataatatc tacctctata gagagggtgt gcttcggact gcttcagaac catatcatgt 360
tgataatttc caagacaaaa cctgccattt gaccaatcac tgcattcaaa aagagtattc 420
aaagaactac gggaagtatg aagaaggaaa tgaaatgttc ttcaaggagt tcaatcagta 480
cctaacaagt gctttgaaca ttaccctaga aagtagtatc ttactacaaa tcaaacatat 540
aataaggaac tgcctcctga gcgtggagcc tgccattagc accaagcacc tcccttacca 600
gagcttccag ctcttcggct ttgacttcat ggtcgatgag gagctgaagg tgtggctcat 660
tgaggtcaac ggtgcccctg catgtgctca gaagctctat gcagaactgt gccaaggcat 720
cgtggacata gccatttcca gtgtcttccc acccccagat gtggagcaac ctcagaccca 780
gccagctgcc ttcatcaagc tgtgacagag ggcactccct gctgccttgg aaaaagcacg 840
gggtcctgct ccagggaatg gtgaaatgac tggattgctc tttatccagc ccacagcagg 900
ggaaagaaag gcaactcgca aagatgagat ggaagaaggc acgtgagcag aggaggcagc 960
tcccaaagag agggctgctc agggggcttc ccaggtgtag ctctcagcag tgctgttgag 1020
acttttgaaa acaactttgg tacacaaagg cagctttgtg agcagagctc cttcccctct 1080
ccccgggaac ggcagggcac tgggacctct ggtcggtgcc tcccacccac tgcagcccta 1140
gtgccttagc tccatgcccg gctgcagccc cactgctctg gactatggat tggacgtcag 1200

agcatattgg aggttgcctg tgtgttcccc acccatccct tcggtaacac tctgccacac 1260
taagctctgt acaagcatgc accaacagtc cttagttttg tgctgtgcac tggcctctcg 1320
gcaaaggtgg tttccctcat caccttcctg atggtgtttg gtcagtcacc tgtcagggtt 1380
tgtgcgggtt gggccccaaa acagcatatg ctgctctaag tctgctctct gcatgtttta 1440
gaaacaaagt ggcaagtctg ccctgaacct gtaagcatca aataagcatg agagagaaaa 1500
aaacatgata tattgcttta cttaataggt tgaatatggt aggtctttga aaatatgatg 1560
attcaatttt ctcaatttc tttgctttaa ccaaaattct aaatgcagtt ttgcctagtt 1620
cccttttttt ttcttttttt actttttttt aaacgtttgt aaaaacctct ttgaggatga 1680
ggagtcagta aaattccact ccccaagtgg ccctgcccca gacaaaggtt gctttccccc 1740
tttttgttct ttttatgccc cgaagcactt tctgcagtag ctagagggac aggtttcctt 1800
ccaggaagga ttcgagttcc tgtgcctgtg ggtattagga gagtatatat cctgcctgaa 1860
tggggaagtc ttctaaaatg ggaaagaagt ggtttcatct ccacacagtg tcttgtaaat 1920
ctcaacaaat gtgtactgtt agaagtggct tccgcttact ggattaacta atactttata 1980
ggcttttcag gaggccacat cactagcagt agggagaaca agatgtcatt tgtgttcagt 2040
gtaagctgag taaacaggcc cttcctagag tgtcctggaa atcacagcaa cccattgaaa 2100
actgccctcc ccaccagaac gtgctacgtt ctttcttcat gcctatgtgt gctccattcc 2160
tcatttctac ttggctcaag aaaacatttc tgcagtcagg tgagactttt acaaaagagg 2220
agaaaatcaa tgcctccttg aacatgatga gatgtgagaa cttacaatga aaaaggcaat 2280
aatgatagaa attatttctt aggtacagca atagttgata ggatgtgagg gtgttacctt 2340
ggggtgaagt ggagaaggtc ccaggtgaat tggctctcat ggaaatttgg aattacgaaa 2400
taaacgtcct gggggttacc cagaatacag atttaaaagt ttgcctgtag agcaaaataa 2460
aacagtcagt tgtagtcatt aatccttgag gcccaacgca gccgatgggt tggtgtttgg 2520
gaaattctga gatgggagtg agatctgatc ggatcctggg aagatgtata cccagttaga 2580
acgtgtaggg ttctgggtcc ctggcaagtc taggtgggcg ggtgacaggg aaagcatggg 2640
catttttgta ttgctgtcac atgctaacag aggtttgtaa ttatcttttg gacccaaatt 2700
atagagacat tcacgagttt tctagccctc acagtaacag agctaagaat tcagatgtca 2760
ggaagtctgt gaatcttgat ggattttctg agaaacctga ctcaatggca tatataagag 2820
ggaagtaaga cttttaagaa aagaaaaagt tatgcctcat tcctcatgtg gcttccaata 2880
agtatcttag gaacttattt cctttttaaa aaatattttt taaattttta aaatttgatt 2940
ttaaatttca aataaattta aataaatttt aaataaattt taaataaaat tttacagaga 3000
cgtggtctca ctatgttgcc caggctggat tgcagtggct attcgcagtt gtaatcatag 3060
cacactgcag cctcgaattt ctgggcttga gcagtcctcc cgtctcagcc tcctgagtag 3120
ctgagactac aggtgcacac caccaagcct ggctttatgt atttatttct gttcatgcgg 3180
aatgattggt tcagaactgt tcctttccct tccatgatgt ccttgacaca gaaggttatg 3240
cctggctccc agtcaggctt catacttttg gtccatgtaa gtgctacccg ttgctggggg 3300
aggagtcatg gtttatttgg aaatgtcagt tgcaatcatg gttctgtcat ttgactgcac 3360

agtatcagag gagcctgtta acctctctgt gccttagttt cttagcccat gaaagagatc 3420
attgcctgac ccagggacta cctcaagggc ttttgatgag gacaagtgac agtaggaaga 3480
tgcaagagcc tttagtacca aggttctcaa cactgactac atgctgaaat gactgctgaa 3540
ataaggcgat tgtatgaata tttaaaatgc ctggaacact aaagtaaagt aatgatattt 3600
c 3601

<210> 7
<211> 2121
<212> DNA
<213> Homo sapiens

<400> 7
ctgtgcctct gcatcgccta ctgggccagc actgctgtct tcctgtccac ttccaacgaa 60
gcggtctata agatctttga tgacagcccc tgcccattta ctgcgaaaac ctgcaaccca 120
gagaccttcc cctcctccaa tgagtcccgc caatgcccca atgcccgttg ccagttcgcc 180
ttctacggtg gtgagtcggg ctaccaccgg gccctgctgg gcctgcagat cttcaatgcc 240
ttcatgttct tctggttggc caacttcgtg ctggcgctgg gccaggtcac gctggccggg 300
gcctttgcct cctactactg ggccctgcgc aagccggacg acctgccggc cttcccgctc 360
atcctggcca ttgtgcagat catccgtgtg atactcgagt acctggatca gcggctgaaa 420
gctgcagaga acaagtttgc caagtgcctc atgacctgtc tcaaatgctg cttctggtgc 480
ctggagaagt tcatcaaatt ccttaatagg aatgcctaca tcatgattgc catctacggc 540
accaatttct gcacctcggc caggaatgcc ttcttcctgc tcatgagaaa catcatcaga 600
gtggctgtcc tggataaagt tactgacttc ctcttcctgt tgggcaaact tctgatcgtt 660
ggtagtgtgg ggatcctggc tttcttcttc ttcacccacc gtatcaggat cgtgcaggat 720
acagcaccac ccctcaatta ttactgggtt cctatactga cggtgatcgt tggctcctac 780
ttgattgcac acggtttctt cagcgtctat ggcatgtgtg tggacacgct gttcctctgc 840
ttcttggagg acctggagag gaatgacggc tcggccgaga ggccttactt catgtcttcc 900
accctcaaga aactcttgaa caagaccaac aagaaggcag cggagtcctg aaggccccgt 960
gctccccacc tctcaaggag tctcatgccg cagggtgctc agtagctggg tctgttcccc 1020
cagccccttg ggctcacctg aagtcctatc actgccgctc tgcccctccc catgagccag 1080
atcccaccag tttctggacg tggagagtct ggggcatctc cttcttatgc caagggggcgc 1140
ttggagtttt catggctgcc cctccagact gcgagaaaca agtaaaaccc attggggcct 1200
cttgatgtct gggatggcac gtggcccgac ctccacaagc tccctcatgc ttcctgtccc 1260
ccgcttacac gacaacgggc cagaccacgg gaaggacggt gtttgtgtct gagggagctg 1320
ctggccacag tgaacaccca cgtttattcc tgcctgctcc ggccaggact gaaccccttc 1380

tccacacctg aacagttggc tcaagggcca ccagaagcat ttctttatta ttattatttt 1440
ttaacctgga catgcattaa agggtctatt agctttcttt ccgtctgtct caacagctga 1500
gatggggccg ccaaggagtg ccttcctttt gctccctcct agctgggagt gacgggtggg 1560
agtgtgtgtg cccaggtggg ggtgtctcct ggctgggaag gagggaaagg gagggagagt 1620
tttgcggggg ttggcagtgg agagcaggct ggagaggaga tggctaatag ctgtttaatg 1680
gaaacctgct gggctggagg gagttaggct gaatttcccg acttcctctg ccagttattg 1740
acacagctct ctttgtaaga gaggaaagaa actaaaccca cccaagggat gatttcaggg 1800
ggagaggtgg agggcagatg tcctgggcaa accggccccc tctgcccaca cacctcactt 1860
gatccttttg ccaaacttgt caaactcagg ggaactggct tcccagttgc ccctttgcca 1920
tattccaagt ccccctcaga cttcatgtct ctgctcatca gcactgtccc aggatcctgg 1980
agagggagaa cccctggccc caggggaaag aggggggggt ctcccgtttc ctgtgcctgc 2040
accagccctg cccccattgc gtctgcacac ccctgcgtgt aactgcattc caaccactaa 2100
taaagtgcct attgtacagg t 2121

<210> 8
<211> 2453
<212> DNA
<213> Homo sapiens

<400> 8
cttttgtctc tcgctgtagc cggagctcca ggttttgctc tcagttctct gtgtcttctg 60
ctcctagggg cctagcctgt gtggccctct gacctgcaga tattgggaga tccacagcta 120
agacgccagg accccctaga agcctagaaa tggatgaccc gaggtatgga atgtgtcctc 180
tcaagggagc aagtggatgc cctggggctg agaggagtct tctggtgcag tcttattttg 240
aaaaggggcc attgacgttt agggatgtgg ccatagaatt ctctctggag gagtggcaat 300
gcctggacag tgctcagcag ggtttgtata ggaaagtgat gttagagaac tacagaaacc 360
tggtcttctt ggcaggtatt gctctcacta agccagacct gatcacctgt ctggagcaag 420
gaaaagagcc ctggaatata aagagacatg agatggtagc caaaccccca gttatatgtt 480
ctcattttcc ccaagacctt tgggcagagc aggacattaa agattctttt caagaagcga 540
ttctgaaaaa atatggaaaa tatggacatg acaatttaca gttacaaaaa ggctgtaaaa 600
gtgtggatga gtgtaaagtg cacaaagaac atgataacaa attaaaccag tgtttgataa 660
ctacccagag caacatattt caatgtgatc catctgcaaa agtctttcat acattttcaa 720
attcaaacag acataagata agacatacta gaaagaaacc tttcaaatgt aaaaaaatgt 780
gaaaaatcat tttgcatgct tttacaccta actcaacata aaagatttca tattacagag 840
aattcctacc aatgtaaaga ttgtggcaaa gccttcaact ggttctcaac ccttactaca 900

cacaggagaa ttcatactgg agagaaaccc tacaaatgtg aagaatgtgg gaaagcattt 960
aaccggtcct cacaccttac tacacataag ataattcata ctggagagaa accatacaga 1020
tgtgaagaat gtgggaaagc ttttaaccgg tcttcacacc ttactacaca taaaagaatt 1080
catactggag tgaaacccta caaatgtaca gaatgtggca aagcttttaa ccggtcctca 1140
caccttacta cacacaggat aattcatact ggagagaaac cctacaaatg tgaagaatgt 1200
ggcaaagcct ttaaccagtc ctcaaccctt actacacata agataactca tgctggagag 1260
aaaccttaca aatgtgaaga atgtggcaaa gcttttaccc gattctcata ccttactaaa 1320
cataagacaa gtcatactgg agagaaattc tacaaatgtg aagaatgcgg caaaggcttt 1380
aactggtcct cagccctcac taaacataag agaattcata ctggagagaa accctacaaa 1440
tgtgaagaat gtggcaaagc ttttaatgag tcctcaaacc ttactaccca taagatgatt 1500
catactggag agaaacccta caaatgtgac gaatgtggca aagcctttaa ccggtcctca 1560
caactaactg cacataagat gattcatact ggagagaaac cctacaaatg tgaggaatgt 1620
ggcaaagctt ttaaccgatc ctcaacccat actaaacata agataactca tactggagag 1680
aaatcttaca aatgggaaga atgtggtaaa gactttaacc agtccctaag ccttattaaa 1740
caaaataact catactggag agaaacccta caaatgtgaa aaatgtggca aagcctttta 1800
ccagtcctca actcttacta aacataaaaa aattcatact ggagggaact cctgtgactg 1860
tgaagaatat ggcaaagcct ttaataaatt ctcaattcct aacagacata agataattca 1920
tactagagag aaattctaca aaccagaaag atgtgacagt gctttgaaaa cacctcaaac 1980
ttttcaaaac ataaatcata gtgttgagaa atcctagaaa tgtgaagaat gtgataaagt 2040
tttaaatggt tgtcacactt gattgtaggt aaggtaagtt atactggaga aaacttctac 2100
atgtgtgaac agtgtgacaa aacttttaac taatgctcac accttcacag gaaagcattt 2160
atacttgaga aatattgtac aaatataaag actgtgaaaa agccattaat acatgctcac 2220
atcttactca acatcagaga gttcatactc aataaaaaca taagtgcaac tactgtcaaa 2280
atatctttaa gaaaatataa gcttttaaag tgaagagtat tttgaagaag aacattgtag 2340
tagaattgta atatgtttac ttgtatcaca gatcttactg tacacgtttt gtattagagg 2400
aaacctctga agcagttgct caaactttgt tcaatatcag ggaatttata ttg 2453

<210> 9
<211> 4792
<212> DNA
<213> Homo sapiens

<400> 9
   tgcaggtgag cctggctcct tccccaactc tctgtgcatt tgtagagggc agggtttact 60
   gttcctgtct ggagggccga gggagccacg tattccttgt gaggacatta tcaggtgctg 120
   ctcctgcagt ggcctctgtt aacagaagga gggtgcagtg ctggagcctc agaagaggct 180
   cgtggaggcc gcagtgtgtc tggttctgat ttctaggggt ttgtgaacat ctgtttgtgg 240
   tttgaaggct ttttggctca ccaggtgggt cagatctcca taaaagatga gcatctcgca 300
   acgcggatgc actgtggttt ggctgtgtgc taggtggacg tggcactgct gccatgggaa 360
   atgggagcca gcagtgacca ggcacccagc caggccagtg cgccatccct gccggcgtgg 420
   agcagagcct ctcccatgtg tgctcgggtc cctgagggtg acgggggtgg cccagtacat 480
   gcaggaggcc cttgtcaagt ctctgcttgt ctcttgtgtc tctcaatgac ccaggtattg 540
   cattcctgct gaggaggaga acaagctgga agatgtggtc cacaccctgc tgcaagccaa 600
   tggcacccca gggctgcaga tgctggaaag caacgtcatg gtgcgtccac tcagccaccg 660
   cctccagcag gagctgtagg acctcctagg cacttgaaca tggtttccca tgaacccgcc 720
   tttcaaaggc aatgaggaca cagcaaaaca aatccccagg gtgcaaaggg aaaggtcctc 780
   tgacacaaaa gcgaaactga ccttccatca gattgcacag ggcgctgggg ttttgcacgg 840
   cctttgcatc tgttttgggg cttgtttgtg ttgtgggctg ctgtgggccc tgggatttct 900
   tgtgcccgcc gctcctacct tctgggcatg tgagaggaat gagcacaaag gcttcacctg 960
   ttttcaggaa gtcatggggc tggtgagttc ctgggaagtg ctgctttgag ctgagttctg 1020
   accagcttct ctggcctctg ctgaccctgc cggtcccgcc tgtccatctt agagaagaga 1080
   tggacggggg actcctttgt aagcccagcc tgtgggcctg tggcctactt ggctctggag 1140
   atgagcccgg cagcctcctg gttgtgtaac cttgtcctgt ttccagccct ggcattgcca 1200
   cgtggggaca gaggggcctg tgtggtcatc atgtcctcag ctgtgcatga acctgacatc 1260
   actcttggac cagtgcagtt tagaagctcc ttcttgtgtt atgaatgacg tctttttatt 1320
   ttccacataa aacaatctta tctccttgag agcaggcctc ttagggtgcg catacgtcac 1380
   ctgaagactg caggtgtccc tgcgcacagg gaggggtgcc tcagcctggc cctgtctccc 1440
   cagatctccc cggaggtgct gtgcaaagag gggatcaagg tgcacaggac cgtgcagcag 1500
   agtggccagt ttgtcgtctg cttcccggga tcctttgtgt ccaaagtgtg ctgtgggtac 1560
   agcgtgtctg agaccgtgca ctttgctacc acccagtgga caagtatggg ctttgagacc 1620
   gccaaggtga gcagagccgg cctcctcccg cttgctgccc ccgcatccct gtgagtgccg 1680
   tgcgtgagcg cacacagaag catccctgcg tgtgcgtgtc tatttgtcaa tagttccttt 1740
   tggaatatgt ctttgaaatt cttaagacgt ggttgaaagg tcttctagga atgaaaagtt 1800
   gttagggatt tgtttgtatc acaaagagtt ttgatcagac cgttactgac agacccctcc 1860
   agtattggaa aactgttgaa gatgctcaag gtaattgctt agaatggaca gagaacctcc 1920
   acccatcgtg agggagtggc agctgcctct gggtagcggc gaagtgctat gacttttcct 1980
   ggtatgtggt gcctttctca cagggaggca aagttttgaa gagtttttaa tctaaatgca 2040
   attcaagatt tagaaattca gacagcctgc ctgccccccc caccaagaag aaccttgaca 2100
   gctgcctagt aatgaaaatc caccctaaag ggatgtgact cctctttcag gaaatgaagc 2160
   gtcgccatat agctaagcca ttctccatgg agaagttact ctaccagatt gcacaagcag 2220
   aagcaaaaaa agaaaacggt cccactctca gtaccatctc agccctcctg gatgagctca 2280
   gggatacaga gctacggcag cgcaggcagc tgttcgaggc tggcctccac tcctccgcac 2340
   gctatggcag ccacgatggc agcagcacgg tggcggacgg gaagaaaaag cctcgaaagt 2400
   ggctgcagtt ggagacgtca gagaggaggt gtcagatctg ccagcacctg tgctacctgt 2460
   ccatggtgag cccgcctggc cctgccggcg ccctcgcatg tagtgcttgg cctgagagct 2520
   ccggggttgc ccccagaaga gggagggcgc tctctgccca ggagacctgc tgtgctccca 2580
   tctctggagc cggctgtggg acctcggcgg agcttctggc cgccggaggt ggctgcctca 2640
   cccacagtga ccaggccaca cagaggctgt ccctctgttc tgcccacgcg tggccctccc 2700
   tcggtcctgc agtgcgtcct tcctccctgg ggagcaggtg gcctccctct ccgccctact 2760
   ttgtcactcc agcccccacg cactctgctc ttacccattc tttctgaggc agtgaggggc 2820
   ggatttccag tactgggcag cgtctgccat tccctgtggc ctgtgcctgg tgcacctggg 2880
   cccatggcct ctgccctttg gctctgttct tggtttcctg gtcctgctca gctacatgat 2940
   ctgcctcagc ccccgaggac ttcagaatct atccttggtg aaagctgatg ttcctcctgt 3000
   cccatctggg atcagagatg cttttctggc acagccactc caggccacag caggctgcat 3060
   tcctcttgcc ttctcgactg tgccctgctg gcttcctcgt gcccacctgc ctgcctcagc 3120
   tttggggtta ataccaaaca cctcctcccc ctgctcaccc ctgttccagc ccagaggtgg 3180
   gctcagaaaa gcccttccat ggcgactgga tggctgtggc cctcgccatg cttcttccag 3240
   tctccccttg acctctcacc agagagctgc cctgacacac tctgggtagg gacgcccagc 3300
   tggcactgca gatggctcca cagcactaca caccttagcc tggaacagag agcatgcccg 3360
   aagggcctga cctgcctccc cgggcctcgt ccctttccac agtgcctcac atctccagta 3420
   cagaagccta gcttaggagg tgcccacccc atatgctgtg tggtgcctgt gtcccacagg 3480
   tgcccccttc ggctgcacac tcgctgagag gccagtgcct ctgcgtccct ctgcttgtcc 3540
   atggtgggcc aggactgaga cccgggtcga ctctccttgg cctcccagtg tgactgtgtc 3600
   atgccgtctc accccgagtc ctggcgtgcc ctgtagaaga gggaggacac agcctctccc 3660
   ggatgagtca gatcgtcaga tcgtcagatc agggctttgt aacatttgaa agagctaaaa 3720
   actcggtgtg tcctcatctc ctccttacct aaggacgcaa atcacattgt atcagatagg 3780
   cccaccctca tggcctcatt tgagctgatt tacctcttta gaggccctac ccccaactgc 3840
   agtcacagct gagatcctgg aggttcaacg tttgcatttg atgggggaca aatgcagtcc 3900
   gtgaccgccc tgcctcctgt cctgtagatt tccttctgtc tggttttttc ccccagactt 3960
   ctcaaaatgt attacctaga gccatttggt tcttaaaaga aaatgacgat aagcattttc 4020
   ccagatcttc aaagtatctt ataaacatca ctgataaaag ctgcatagct tggtgtgctc 4080
   tctctctgtt cttttttttt tgtttgtttg agacagtctt gctctgttgc ctaggatgga 4140
   atgcgtgctg tgatcctgtc tcactgtagc ctcaacctcc tggtcatcct cccacctcag 4200
   cctcctgagt agctgggaca gcaggtgtac caccacgcct ggttaacttt tttttttttg 4260
   tggtagagat ggggttttgc tgttacccat aggctgccta cttcagcctc ccagagcccc 4320
   aggattatag gcatgggcca ctgcgcccag ctgtccatcc tcttgctgga acacgctatt 4380
   catttccatg cactgagggc tgtatggtag ttacacctgg gggtgaagct ggtctgtccc 4440
   tgtgaggtgg caccaggtgg cctcattttc aagccatcct tcagaaactt cagagttgtc 4500
   acctggcatt tcctgtcagc ctgaaagtta ttttaagaag aatgtattgt gaggccgggc 4560
   atggtaggcc aatcccagtg cttcgggagg caacggtggg cagatcactc gaggccagga 4620
   gtgcgagacc agcctgggtg acagagggag actaatctct acaaagaata caaaaaatta 4680
   actgggtgtg gtggcggggg tatcacttga gcccagaagt tcaaggctac agtgagccat 4740
   gatcacacca ctgtactcca gcctgggtga cagagtgaca acctgtctct tt 4792

<210> 10
<211> 2630
<212> DNA
<213> Homo sapiens

<400> 10
agaggttatg ttgctagagg tgagatcagt tacctacgtg caactgaaat ttcaaacttc 60
tgttcagcag ggacgtgagt ggacaatggt gactgatagt tggaaatatc agcaaacatc 120
ttaaatttta tactcaaatg aatgagcaat gaaccaggag aataggtcca gtttttttg 180
gctccttgta atttttacct ttttacttaa aattacagca tctttttcaa tgagtgccta 240
tgtgactgtg acttattaca atgaaaccag caactacact gcaatagaga catgtgaatg 300
tggcgtttat ggattagctt caccagtggc taatgctatg ggagtggtag gcatccctaa 360
gaacaataac taccaagctt gtgaccacaa caccgagttt agtaatacta agaagccctg 420
gattgcgctg atagaaagag gtaattgtac attttcagaa aaaattcaaa cagcgggcag 480
aagaaatgct gatgctgttg tgatttacaa tgctccagag actggcaatc agacgataca 540
gatggcaaat tttggtgcag tagacattgt tgcaatcatg atcggcaatc tgaaaggcac 600
aaaaattctg caatctattc aaagaggcat acaagtgaca atggtcatag aagtagggaa 660
aaaacatggc ccttgggtga atcactattc aatttttttc gtttctgtgt ccttttttat 720
tattacggcg gcaactgtgg gctattttat cttttattct gctcgaaggc tacggaatgc 780
aagagctcaa agcaggaagc agaggcaatt aaaggcagat gctaaaaaag ctattggaag 840
gcttcaacta cgcacactga aacaaggaga caaggaaatt ggccctgatg gagatagttg 900
tgctgtgtgc attgaattgt ataaaccaaa tgatttggta cgcatcttaa cgtgcaacca 960
tattttccat aagacatgtg ttgacccatg gctgttagaa cacaggactt gccccatgtg 1020
caaatgtgac atactcaaag ctttgggaat tgaggtggat gttgaagatg gatcagtgtc 1080
tttacaagtc cctgtatcca atgaaatatc taatagtgcc tcctcccatg aagaggataa 1140
tcgcagcgag accgcatcat ctggatatgc ttcagtacag ggaacagatg aaccgcctct 1200
ggaggaacac gtgcagtcaa caaatgaaag tctacagctg gtaaaccatg aagcaaattc 1260
tgtggcagtg gatgttattc ctcatgttga caacccaacc tttgaagaag acgaaactcc 1320

taatcaagag actgctgttc gagaaattaa atcttaaaat ctgtgtaaat agaaaacttg 1380
aaccattagt aataacagaa ctgccaatca gggcctagtt tctattaata aattggataa 1440
atttaataaa ataagagtga tactgaaagt gctcagatga ctaatattat gctatagtta 1500
aatggcttaa aatatttaac ctgttaactt ttttccacaa actcattata atatttttca 1560
taggcaagtt tcctctcagt agtgataaca acatttttag acattcaaaa ctgtcttcaa 1620
gaagtcacgt ttttcattta taacaatttt cttataaaaa catgttgctt ttaaaatgtg 1680
gagtagctgt aatcacttta ttttatgata gtatcttaat gaaaaatacc acttctttag 1740
cttgggctac atgtgtcagg gtttttctcc aggtgcttat attgatctgg aattgtaatg 1800
taaaaagcaa tgcaaactta ggcgagtact tcttgaaatg tctatttaag ctgctttaag 1860
ttaatagaaa agattaaagc aaaatattca tttttacttt ttcttatttt taaaattagg 1920
ctgaatgtac ttcatgtgat ttgtcaacca tagtttatca gagattatgg acttaattga 1980
ttggtatatt agtgacatca acttgacaca agattagaca aaaaattcct tacaaaaata 2040
ctgtgtaact atttctcaaa cttgtgggat ttttcaaaag ctcagtatat gaatcatcat 2100
actgtttgaa attgctaatg acagagtaag taacactaat attggtcatt gatcttcgtt 2160
catgaattag tctacagaaa aaaaatgttc tgtaaaatta gtctgttgaa aatgttttcc 2220
aaacaatgtt actttgaaaa ttgagtttat gtttgaccta aatgggctaa aattacatta 2280
gataaactaa aattctgtcc gtgtaactat aaattttgtg aatgcatttt cctggtgttt 2340
gaaaaagaag ggggggagaa ttccaggtgc cttaatataa agtttgaagc ttcatccacc 2400
aaagttaaat agagctattt aaaaatgcac tttatttgta ctctgtgtgg cttttgtttt 2460
agaattttgt tcaaattata gcagaattta ggcaaaaata aaacagacat gtatttttgt 2520
ttgctgaatg gatgaaacca ttgcattctt gtacactgat ttgaaatgct gtaaatatgt 2580
cccaatttgt attgattctc tttaaatata aaatgtaaat aaaatattcc 2630

<210> 11
<211> 2255
<212> DNA
<213> Homo sapiens

<400> 11
tccagaccca gacaactctt ggtcggatgg tgaggagccc ggccgcagcc cggggcgcgg 60
atgcctgccc gccgcccgcc ctctctgcag gagcggctcc tcctccgggc cgcgcggctc 120
ccggcgagac cccatccagg cgccgcgccc ggcccggctg gggaacgcag agatttcaca 180
ccctttggag agtttctttc ttggataatt cagggtcttg ctgtgttgct cgggctgctc 240
ttgaactcct gggctcaaac agtcctcttg cgtcagcctc ccaacgtgct gggattacgg 300
gagcaagcca ccccactgtg tgcagcccag aagttgagcc tgagaggaag atgagagact 360

gcttaggcgc caccactagt accatgagtc cctgcactgg ttaaagccat cgccacaacc 420
tggacaggca gcaagggctc tgggtttgca gagagccgaa atgaccatga ctgccaacaa 480
gaattccagc atcacccacg gagctggtgg cactaaagcc cctcggggga ctctgagcag 540
gtctcagtca gtctctccac ctccagttct ctccccacca aggagtccca tctacccgct 600
cagtgatagt gaaacctcag cctgcaggta ccccagccac tccagctccc gggtgctcct 660
caaggaccgg cacccccag ctccttcacc ccagaatcct caagatccct ccccagatac 720
ttccccaccc acctgtccct tcaagaccgc cagcttcggt tatttggaca gaagcccttc 780
ggcgtgcaag agagacgccc aaaaggaaag tgtccaaggc gcagcccagg atgtagcagg 840
ggtcgctgcc tgcctccccc ttgcccagag cacgccattc ccggggccag cagctggccc 900
ccggggcgtc ttgctgaccc gtaccggtac ccgcgcccac agcctgggca tccgggagga 960
gatatcagca tgggaaggtc gccgagaggc gtcgcccagg atgagcatgt gtggagagaa 1020
gcgggagggc tctgggagcg agtgggcggc cagtgagggc tgccccagcc tgggctgtcc 1080
cagcgtggtg ccgtccccct gcagctctga aaagaccttt gatttcaagg gcctccggag 1140
gatgagcagg accttctccg agtgttccta cccagagact gaggaggagg gagaggcgct 1200
ccctgtccgg gactctttct accggctgga gaaacggctg ggccggagtg agcccagcgc 1260
cttcctcagg gggcgtggca gcaggaagga gagctcagca gtgctgagcc ggatccagaa 1320
aattgaacag gtcctgaagg agcagccggg ccgggggctc ccccagctcc ccagcagctg 1380
ctacagcgtg gaccggggga aaaggaacac tggaaccttg ggctccttgg aggagccggc 1440
agggggcgcg agtgtgagcg ctggcagccg ggcagtcgga gtggctggtg ttgcggggga 1500
ggcgggccca cccccagaga gggaaggcag tggttccact aagcccggga cccctggaaa 1560
tagccctagc tcccagcggc tgccatcgaa gagttccctc gatcccgctg tgaaccctat 1620
cctccaagcc agcccacctc tgccttcatc atatcccagg atactgtttg taaataatct 1680
gctgtaagct ttcttaactg tttttgtaa caagcaaaga gaatatggca aatatttgta 1740
tattcccaag gggccgggtg ctttcctgtc ctgccagagc atggatgaag tttcgctggg 1800
tgctcgtgac tggccagttt tgtgcagctg actgtctcag ccaaaccact gatcttccct 1860
ggaggccttc ggcctgcctg cctgcctgcc tgaggtcccc gctgccagtc ccgggccctg 1920
gagagcagat gctgtcttgt tatgtacagg aggacctttt aaaaaaatca agtttctatt 1980
ttttgctggt agtccgcata cccataccct ctgtttttga aaggcaaagg ccaatcagtc 2040
cccatttgta gcatggcacc agggtcttag gcctagtcct ctcattcctc ccaccctccg 2100
agatggtcag tgtgtcatgg gaagcccacc cccagctctg ccagtgctct ctgggcctgg 2160
ctcccagtca gtggtggcca cgatgcggta cagggcatcc ctccttccca tctacgggtg 2220
ttctcaataa acaatgtaca gttgtttggg cccag 2255

<210> 12
<211> 2078

<212> DNA
<213> Homo sapiens

<400> 12
atgggcacct tcccctgcct ctctccatgt gggtccccgc ccccagcctc gccctcagca 60
tctctctctc tcccactcgt gggcctcagt gctccaagag ctgcagctcg ggcactcgga 120
ggcgacaggt catctgtgcc attgggccgc ccagccactg cgggagcctg cagcactcca 180
agcctgtgga tgtggagcct tgtaacacgc agccctgtca tctcccccag ggtaaggaca 240
ggagggcagg gaggagtccg gcctctgacc tctctcccac tcgctacaaa cccagcaagc 300
atgtcctgcc tcggggcctc tgcctgcact gtgtcatccc tctggacccc acttctccca 360
tgtctccccc cgctgaaccc tttgccatct ctaaggccca cctggtgtgc agcccaccag 420
gcagccctgc tttcctccac tcagcggtag catgcccctg cccctctggc ccccatagca 480
tgctgtcatt tctgtgggac ccttagattc atttccttgt gctgctgtgg ttgtatacgg 540
cttgtcagac ctacccacta taagcttctt gaggactggg tccccaactt ccatattcct 600
ctttataccc ccatagaact tacctgacac agttaggtac tcagtgaatg tctgttgaat 660
gactgagtga ctgactgtgt gctgagggag ggaggcagtc caggaaggag tgtctccaca 720
tgccacttct gcctctgtct tcttcccttc atccccatcc cactgtgaaa ggagcctcac 780
aggcatgagg gcctgctgga gaaagggcac tgaggccagt gtttgcgtgg tgtgaggatg 840
ccacattgag gaagggtgtg ggttgtggtg agctggggat gggggtgggt gcgtagcctc 900
ccaatactgc agggaggcat gtttggcctt gactcactcg tgatggggac tctatgagct 960
gtagggacct tgagatgaag gaaggcagaa tttctatgag acatgtgaca ggtgtggcta 1020
agaaccagtg gcctcagagt caacagcagg cttgggttcc gatccactcc gttgctgacc 1080
agttgtgtgg ccttgggcaa gtgccccacc tctctgattc tcagtttgcc catctgcaca 1140
gtggggttaa aaagagcgac tttccagacc gtggtggtca tgcagcacaa cgcctgaccg 1200
catggggtgc ttgctagcta agcagagggt ggaaggggtg gtggtgaggc cttgtggggg 1260
agctcggcca tccggagggc ccctggtgcc tcagctgggg cctggtcctt ggggctcacc 1320
agcaggagag ccttggactc ccaggggtcc agctctccat ggagccagtc tgttccccag 1380
ggccttgttg gcttctaaaa gaggtgatct taagcctgtc ccagggacta gcaggaccca 1440
tatagaaagg aagaaaaagg gccaggtgag gattaatccc aaagtgctgg gattgcgggc 1500
gggagcccgt gctttgctca ttatctagtg tctttctatc cctttgtcta ttcatccaat 1560
tttttaaat atggaatttt tttatggagg tgaaatttac attcagttaa ccctttttaaa 1620
gtgtacaggt tggtggtatt tagcgcatta tttcaatctc tttgattata gacatcctag 1680
tgggtgggaa gcaggacctc attgtggttt tgatttgcat ttccctgatg actaatgatg 1740
ttgagtatct ttttacgtgt ttgttggcca tttgcatatc atctttggag aaatgcgcaa 1800
gttctttacc cactttttaa ttggattgtt tgcctttcat cagtccaatt ttttaaaaaa 1860
gtatgttctg ttttttgttt tatgtggctg ggatcataac acgtgcacat tttatagctg 1920

gtacttttg ctgaacatgt aagattagca tttttcatgc tgggtgcagt ggcttacacc 1980
cataatccca gcattttggg gggctgagtc aggaggatcg cttgagcccg ggagtttgag 2040
acaagcctgg gcaatataga aagaccctat ctctaaag 2078

<210> 13
<211> 2616
<212> DNA
<213> Homo sapiens

<400> 13
   aaactggact caggtggaaa tatccccagg tctcccagct cccaccccca gcctctgagc 60
   cggtggcacg gaggccggaa gtttggggag cctctcagag cctgtcttct ctgctcctct 120
   tcccccagcc tcaggagttc cccattctct gggtccaggg aggggactgg gtgtccagag 180
   acctggttct agtctcacac tggcacagat gcctacatag cctggacagt cagaccacct 240
   tgcagtgggc cttggcatcc cccatgtgtc cccaggtggg ggccaagggc ctgtgcaccc 300
   ctgcccacag ctgtagtcca gagtgctgtg tgactgtgcc taggagtctg agctcctgca 360
   cttcccgttc ttggggccag ctaatactcc tcatcccggg gtgggctgag gatttgggag 420
   tgggcagaaa agaggcttct gtgtcccaaa gctgggcccc gggtgggtct ctcagcctcc 480
   cctttagttt catctgtccg ccctctctca ggacacagcc ctgggtggag gaggaaaaac 540
   ccatgtatcc tattcccacc cccatggaat gtgctccgac tgggatgaag caactgggcc 600
   acccagggca ggcctcagct gggagggaca gtggaagcac tggggtccag gtgccctgct 660
   gcggggaagg gaagtagggg gcgttctaac ccaggcaggc tcagcagaag cactctcccc 720
   ccccccccca ccactccccg cagcaccccg gagaccgctg agttcctggg tgaggacctg 780
   ctgcaggtag aacagcggct ggagccggcc aagcgggcag cccacaacat ccacaagcgg 840
   ctgcaggcct gtctgcaggg ccagagcggg gcagacatgg acaagcgggt gaagaagctt 900
   cccctcatgg ctctgtccac cacgatggct gagagcttca aggagctgga ccctgattcc 960
   agcatggggt gagcacagac ggggcccagc cctcacctgg ggataccaag acgtgatctc 1020
   agctgggagg gggtccaggt ggtgagggca tccacatcag aagaatgacc aggctgggga 1080
   cagttcccag gaaggccttg gagatgagct gtgccatcca gaatcagctg gcccgcatcc 1140
   tggccgagtt tgagatgacc ctggagaggg acgtcctgca gccactcagc aggctgagtg 1200
   aggaggagct gccagccatc ctcaaacaca agaaaagcct ccagaagctc gtgtccgact 1260
   ggaacacact caagagcagg ctcagtcagg caaccaagaa ttcaggcagc agtcaaggcc 1320
   taggaggcag cccgggtagt cacagccata cgaccatggc caacaaggtg gagacgctga 1380
   aggaggagga ggaggagctg aagaggaaag tggagcaatg cagggacgag tacttggctg 1440
   acctgtacca ctttgttacc aaggaggact cctatgccaa ctacttcatt cgtctcctgg 1500
   agattcaggc cgattaccat cgcaggtcac tgagctcgct ggacacagcc ctggctgagc 1560
   tgagggagaa ccacggccaa gcagaccact ccccttcgat gacagccacc cacttcccca 1620
   gggtgtatgg ggtgtcgctg gcaacccacc tgcaagagct gggccgggag attgccctgc 1680
   ccatcgaggc ctgcgtcatg atgctgcttt ctgagggcat gaaggaagag ggtctcttcc 1740
   gtctggctgc tggggcctcg gtgctgaagc gtctcaagca gacaatggcc tcggaccccc 1800
   acagcctgga ggagttctgc tccgacccgc acgctgtggc aggtgccctc aagtcctatc 1860
   tgcgggagct gccagagcct ctgatgacct tcgacctcta tgatgactgg atgagggcag 1920
   ccagcctgaa ggagccaggg gcccggctgc aggccctcca agaggtgtgc agccgcctac 1980
   cccccgagaa cctcagcaac ctcaggtacc tgatgaagtt cctggcacgg ctggccgagg 2040
   agcaggaggt gaacaagatg acacccagca acatcgccat agtcctggga cccaacttgc 2100
   tgtggccacc tgagaaagaa ggggaccagg cccagctgga tgcagcctcc gtgtcttcca 2160
   tccaggtggt gggcgtcgtc gaggcgctga tccagagcgc agacaccctc ttccctggag 2220
   acatcaactt caacgtgtca ggcctcttct cagctgttac cctccaggac acagtcagtg 2280
   acaggctggc ctctgaggaa cttccgtcca ctgccgtgcc caccccagcc accaccccgg 2340
   ctccggctcc ggctccagct ccagctccgg ccccagcctt ggcttcagca gctaccaagg 2400
   aaaggacaga gtctgaggtg cctcccagac cagcctcccc caaggtcacc aggagtcccc 2460
   cggagacagc tgccccagtg gaggacatgg ctcggaggag tcctagggga gccaccggaa 2520
   ggaaggagag gtttgcctgc tcctacggga ctgattcttc tcttgtcgac atgttttttg 2580
   taaggctggt aaataaatta ttttggacaa aactgg 2616

<210> 14
<211> 2182
<212> DNA
<213> Homo sapiens

<400> 14
catattggat tttgttttct tttttgatta tttttatggc ttcattttta aaaatgtaaa 60
tctttaatcc tttgaaggtt aatgtggatg gacagaatga gatgtggacc ctcccttatt 120
tatatacatg tttatttgtt tatctctgga tgggctaagt tgtcccagtt gtcctgtgac 180
ctttcactga caagtctgtc tttccccatg acctgtgatg gcatctttag tgcgtcctgg 240
gtcccggctg tgctggggtc tggtagcagc ggctggttta gtgtgtcctg ggtcctggct 300
gtgctggggt ctgttagcag cggctggttt agtgtgtcct gggtcctaac tttgctggag 360
tctggtagcg acggctggtt tagtgtgtcc tgggtcccaa ctgtgctgga gtctagtagc 420
ggcggctggt ttagtgcgtc ctgggtcccg gctgtgctgg ggtctggtgg cagcagctgg 480
tttggtggtc ctctctgttg cccctgctga gctgtcacag agtgagtttg ctcctcagag 540

ctggagtcaa gctgcaggag taccccactt ggaggggtgg ccctagagtg gagaagggat 600
ggtggtctct gccagtgagc ctgtgggtgt tggacatccg tgatgtcaaa acaccagcag 660
gaattctctc tgtctccttg gtgccctggc cttatgggta gagactacct ggcccacctg 720
tttggccccc ctcagctcct gggcacaagg atacaaaagc cacactgcct tctccctggg 780
gtcccatgat gggccagcct gccccagggt tcccctggga gcctccacct cagacatagc 840
ctctcacctg tggcagccag gtcccaccca cccactgccc cagcctctgc ttgactcagg 900
cagtagtagc aggtcacagt tgggagcaga ggggaaacag ggcgtgttta agtggcctta 960
ttcccagaat cctggctcca gagctgtgtc tgaaagggag aggaactact tcgtctgtaa 1020
tacatcgggg gcagggattg ctggaagagc aagtctgttt ccaggtgaga aataatgaac 1080
aactttttct tatttctttt ttgagacaa ggtcttgctc tttcacccag gctggagtgc 1140
agtggtgcaa tcacggctca ctgcagcctc aacctcctgg gctcaagcaa tcctcccacc 1200
tcgtccttcc aagtagccga gagcacaggc ttatatggta gtgacagtga ggatggagaa 1260
tagtaaaaat gtttgaggaa tttttttttt aattttattt ttattatgaa aatgtccaat 1320
taggctgggc ctggtggctc accccctgtaa tcctagcact ttgggaggcc atagcgggag 1380
gattgtttga actcaggagt ttgagaccag cctgaacaac acagtgaaac cctacctcta 1440
tattaaaaat aagtaaataa atttaaaact taagaaaatg tccaacatgc agaaaagtaa 1500
aaataatttt tttatttttg agacagaatc ttgctctgtc accaggctgg agggctgtgg 1560
cacaatctca gctcaccgca gtctctacct tccaggttca agtgattctc atgtctcagc 1620
ctcctgagta gctggaatta caggcacatg cctggctaat ttttgtattt ttagtagaga 1680
tagggtttta ccatgttggc caggctggtc ttgaactcct gatctcaaga gatccaccta 1740
cctctgcctc ccaaggtgct gggattgcag gtgtgagcca ccatgcccgg ccaaaaagaa 1800

tttttttttt ttttttgaga ccaagtcttg ctctgtcacc caggctggag tgcaatggca 1860
tgatctcgtc ttactgcaac ctctgcctcc cgggctcaag tgatactcct gcctcagcct 1920
cccgagtagc tgggattaca ggcatatgcc accatgcccc gctaattttt gtatttttag 1980
tagaggaggg gttttgccgt gttggccagg ctggtctcga actcttgacc tcaggtgatc 2040
cgcctgcctc ggcctcccaa agtgctggga ttacaggcat gagccaccac actgagccaa 2100
aaataatatt tttgaaaaac gaatacaatt acctattgcc tagatttaac aatacttgct 2160
ccttgctggc tgctctacta gg 2182

<210> 15
<211> 1955
<212> DNA
<213> Homo sapiens

<400> 15
   atcttcacca ctgctctctc agagatccag gtccgggaga tgacagtggc tcccagaaag 60
   cccaggattc aatcgctgag agagtgctta ggcccgaatg ccggcccaaa tcgttctact 120
   caccgtgtcg gaggccgagg ccgaggccga gagcgatgag agtgcaggga agtggggaag 180
   agggggtggc cgccaggctc ctccgcttcc ctgggtccac ggcggatccc tcccgcttgt 240
   caggaggcgg ccagcgggta agctgactgg cggaaatgcg agagaggaga agggaaaggt 300
   ggaaggctaa agggggcaaa ctgaggggag gcgggtcccg caaccgagac tgggatcgtc 360
   tcccctccgc aaagcgaacc caaaatggcg gcgggagcgg cggcggcggc ggcggcagca 420
   gcagagtggc ggcggtggcg gcggcagctc ctccagaggg agggagcgaa gggcgcctag 480
   cgcccccctc aacctcccac tcctccctcc tcgcgttctt ccccaccgtc ccccgctccg 540
   cccgactccg tccgcgtagc gcgcacgccc gcccgcacgc gtacgagtgt ctacgggctc 600
   gtcgctggct gctcccacca accaccacct tcggccgccc cgcgcgccag ccagcccgta 660
   cgcgctcacc cacaggaacc ccctcgtcca gtccctcact acccctcagg ccctgtcaag 720
   ccggcgccgg cgcaggccct cacgcgtacc ttcaacggcg caagcccaag cctcctcctc 780
   ctcctcctcc acctcctctt ctctcctccc cccccttccc cgcccccacg gccaccaacc 840
   gccgccacgg ccgccgccgc cgccgccagc cccccctacc accctccacc cctcgcgcgt 900
   gcgcctccca caatcccccc cgcgggactg ttccattcct gtcggctgca ggggcaggag 960
   aggaagggac gggccgagcg ggtcggggct tgccgtttga ctggaattgc cagaatggcg 1020
   gaccgagccc cacgacaacc tacctccctg ggctcctcgc cgcagcgctg cggctcgcct 1080
   ccctctgctc ctcctcctcc gccggatcgc ggcgagcgga tcgaggactg cctagcgccc 1140
   ctctgcccac cggtggttgg aggccgcggc ggctgcgcgt tgagtcgttt cctgccggtt 1200
   gacctgagcc tacttcgcag tagcaggacc gctgctgtgg agctggtcgc aggcggtgtg 1260
   tgccggtcgc ctagtcagga gaactagtcc tcgactcacg gtgagggaat ggaccgacac 1320
   gggtattgta ccgctgaggg aaaggagcgg gactccggac ctccaggagg tagggagtga 1380
   ggccagtagg accggcgcgc ctccgggggg attcctcccg ggcgttgagt tgccaacctg 1440
   ggacccgagg aagatcggcg tggtggtgtg ctttttgttg ttgttaaccc tcctcggatt 1500
   tctcgaattt cacaccactg tccatatgcg atgatgtttg tttgcccttg acgcacttac 1560
   tcatggatgg tacttcttca gcctcgttag acagcctggt gatggaggat gaagaaacca 1620
   tgtgcttttc attcagttct ggacttagtc tcccttttct tccttcagca agttattttt 1680
   gttagttcct tatcaaaaag tgtacataaa aattaggcaa ctccaaacat gcctccaggg 1740
   ttaatgtgtg aaataataag ataatatatg taaagtggaa ttagctccta ggcataggga 1800
   aagtgcagaa tattgccgtg ttgtcattta cagttctgtt gatgtcgata acgtttgtgg 1860
   gtgtaattgg tagtgttctg tccctccaag gagttaataa aacaaagcaa acataggcct 1920
   gttaggtttc tgtggctact gaatatttgt ttctc 1955

<210> 16
<211> 2723
<212> DNA
<213> Homo sapiens

<400> 16
gtcccagaag cggagcatcc aggcaggtag gggcttccgc tacttaccta actcccacaa 60
ctcagtgggg gtgtaggcac tatatgaagt gtgctccacc acggtagggg gtccctgggc 120
ccacccttgg ggcccaagtg gctgttcatg ctctattttc tggcagacca cagggtgagc 180
ctgcagcagg ggttcttcct cctcggtatc agatggaatg ggagtctctg gccaggaagg 240
caggctcaag cctgcactta cggcagttac ccattctcat tccaagcttt gtatttgggc 300
ctccaggcac tcagctagca cctggaggtt ccttacctgt gctgtatccc gcagggactg 360
ggcatgcact ttccatagca tagtcaaaaa tgcccatcca actctgctgg caaaggcacc 420
ctccttctcg gtgttgtatg cttccaggtg cttcagggcc ttctccatgc tcatggggga 480
cctgtctacc tctgcccatg tttccaccag ggcccatctg gacagcacag ctgccaccaa 540
gtaccgcaac ccatgttggg gccacatggc caaccaggga tcatcaggtg ctgaggactc 600
actcacttca gaatcctgct aactacacca attgtccggt tccaacctaa gctggagtcc 660
gaggggagtt ggtggacggg tggcaggtag ttgaaagaac acttgggggt ccggaggcag 720
gggctttatt atgtgcccct ctcacagtgt cagtgataca tttttgcacc tcagagccag 780
ttgatgagct cacttataac atggttacat aactgtgatt atataatgca caggattgta 840
cacacgcact ccaatcctgc tgtgtcacgc tgcaccagat gtttaccttg gcctactctt 900
gactgccatg cagccttttt ccttacatat atataccatg gaatactatg cagccataaa 960
aaagaatgaa atcatgtcct ttgcagcaac atggatgaag ctggaggtca ctatctgaag 1020
tgaattaatg tgggaacaga aaaccaaaca ctacatgttc tcacttataa gtgggagtga 1080
aacattgggt actcatggaa ataatgatgg caacaataga aactgggaac tactgggggg 1140
aaggttgaga gactaaccat taggtactat gctcagtacc tgggtgatgg aaccattcat 1200
accccaaacc tcagcatcgt gcaatatacc caggtaagaa agctgcacat gttcccccaa 1260
tctaaagtaa aagttgagaa aaaaagaaaa tttgattttg tgcattctat cttatgctca 1320
gttttggtct gagttttgtt gttgttgttg ttgttgttgt tttatttttt atttttttcg 1380
agacagggtc tctaaaaaag gcccaggctg gtttcaaact cctgggttca agcaatcctg 1440
cctcaccctc ccaacatgct gggattatag gtgtgagcca ctgcccccag ccaagttgtt 1500
ttttatatga gacaatttgg taagggatca aaagatgaaa atacagaaat aaatggcata 1560
taaattttgc ataacagatg aagcttctag tactgatgat ggcagctcaa gaaatggact 1620
ttgctttgga aggaacagag tagtccacaa ctttccttcc tatacttgca ggaaactgct 1680
acttgaaaaa gatgaaacat ctttagatag gtttctcaaa aagtagtcaa gtgcccttaa 1740
gtgagtactt ccattaatct caagatgcct ttgaaaatta cccaaagaag ttctgatttc 1800

tttttttttt tttttttttt tttgagacgg agtctcgctc tgtcgcccag gctggagtgc 1860
agtggcgcga tctcggctca ctgcaagctc cgcctcccgg gttcacgcca ttctcctgcc 1920
tcagcctccc gagtagctgg gactgcaggc gcccgctacc acgcccggct aattttctgt 1980
atttttagta gagacggggt ttcaccgtgt tggccaggat ggtctcgatc tcctgacctc 2040
gtgatccgcc cgcctcggcc tcccaaagtg ctgggattac aggcatgagc caccgcgccc 2100
ggccagaagt tctgatttct atggcttcag tgctttttca cctgatttgt tgcaatacaa 2160
ataatacgag ggttaaaaag tagggatttt gcacctttct gaattccaga gtacataagt 2220
ggtctttgaa gagattttca aatacaggta ggttaactac tatttaaggc aaagtcttta 2280
gttacattgc aaacataaat taaatactta ggaggtaatg ttttggactc ttcctggcta 2340
tcactcatca gttaattagg gtcttttaga atttggtttg gctgggtgta gtggctcaca 2400
cctataatct cagcgctctg ggaggccaag gtggtaggat tgcttgaacc taggagtttg 2460
agagaccaac ctgggcaaca tagtgagact ccatctctac aacataaaaa attaaaataa 2520
ataaattagc caggcatggt ggcatgcaca tgtagttcca gctacttggg aggctgaggt 2580
agcaggatga tttgaccccg gggagttgag gctgcagtga gccatgatca tgccactgca 2640
ctccagcctg ggcaacagag caagaccctg tctcaaaaaa agaaaaatac aatgccaaat 2700
gttgcatggg acacacttat act 2723

<210> 17
<211> 2269
<212> DNA
<213> Homo sapiens

<400> 17
ttaaacatta tccaggtgtg gtggtgtcca cctgtgatcc cagctactta ggaggccgag 60
gcaggagaat cgcttgagcc caggagttcg agactgcagt gagccatggt ggtgccactg 120
tgctccagcc tgggtgacag atggagacct tgtctctaaa ttcatgtgtt gagcctggtt 180
gcagtttggg tggtgaagta gaaaggacac tggtcttgga gtcaggtgga ttggttggaa 240
tcccaggtct tactgcgtgt gactttagcc cagtgatttc acccccccga gccttcactt 300
tcccatggga tgatggccat gctaatgctt cctttgcagg gctttctgga ggataaagcc 360
aggagagcat gggaggccct gcctccaagg tgctcacaat gctccttcat tccttttctc 420
ccttgcttcc tcctacccag tgcgttcagc cccaggcatt gcctgccccc ctcccccgc 480
cccgccatcc agaccttgac cttggccttg agttccatga ggccttcgga aatggctttg 540
gctgctacct cggagggagc tgccccgatc cacttgcaga tcagaatctc cgggcacccc 600
ttgccagggc tttcatgttg ttgctttatt tcacaagaac cccggcattt gtttagcaac 660
tgggtggaat ttttagaact taaaagtaat tctgctcttc gaggaatgcg cctgtggatg 720

taacccaac tcaggccagg cctggaccca gagggagtgg gcactctgcc ctgggcctcc 780
ccacctccac atctttgctc cagttgaatg caggacaccc tcagtctgcc ctgtccggct 840
tgacttcttc ccgcctgctg gtcttgaccc cagggcaggg gctggtgggg tgggcacaac 900
agtgtcagcc tgtggttagc taaataatgc ccgcccccca agatgtccat gatataatcc 960
ctggaacctg tggatatatt atatggcaag agggactttg cagctgtgac ggaggatctt 1020
gagatgggaa gatgatctgg attgtctggg tgggccctgg gtaatcgtgg ggtacttata 1080
gaggaaggtt ggcagatcag agagagggaa ggagatgtga gactggaaaa aggtcagagt 1140
cagagagatc tgaaggtgcc ctgttgctga ctttcaggct agaggacagg gccagcagcc 1200
atgggatgca ggcggcctct aaaggctggg aaaggcaagg aaacaaattc tcccctatag 1260
cctccggtgg gagcctggcc ctgccaacac cctgattttg gcccagtgaa acctaatttg 1320
gacttctgat ccttaaaact ataaaataat acatcagtgt tgttttaagc cacaaagttg 1380
gtggtaattt gttacagcaa caataggatt ctaatgcaca acccagagcc cccgtccctc 1440
cttggagcac ctcattcaaa gtagggctga ggaggcacct cggggggtac agagggtgtc 1500
gtgagagagc cactctaacc tgtagccaaa tgctgcccgg ccactgtacc cctctgaacc 1560
tccagtaacc cctctggaaa gcaaggttca ttcctcctag aactgttggg aagaattttt 1620
gaggatggga aggacctagc ccagcccctg gcatggaata agcacaaaat tactcagccg 1680
tgtatattgt ttgtttctct gctgaaaaag gcagggaaag agggggtgtg cctggcacag 1740
tgcttgtacg tttggatcaa cattaggcaa cctggattca aattccagct ttggccgggt 1800
gcagtggctc acgcctgtaa tcccaacgct ttgggaggcc aaggcgggag aatcacttga 1860
gcccaaaagt tcaagaccag actgggcaac atagcaagac cctatctcta aaaaaattta 1920
aacattatcc aggtgtggtg gtgtccacct gtgatcccag ctacttagga ggctgaggca 1980
cttgagccca ggagttcgag actgcagtga gccatggtgg tgccactgcg ctccagcctg 2040
ggtgacagat ggagaccttg cctctaaaaa ataaataaaa atcccagctt ccccaaaccc 2100
agcaaaggtg gtggtgcatt gcaggtaaag ttctgcaaca tggctgggcg tggtggttca 2160
cgcctgtaat cccagcactt tgggaggctg aggcgggtgg atcacttgaa gtcaggagtt 2220
taagaccagc ctggccaaca gggcgaaacc ccatctctac taaaaatac 2269

<210> 18
<211> 1944
<212> DNA
<213> Homo sapiens

<400> 18
   tcatagtcca tcaagcaggg ctgtgactgg tcacatgtgg gctactggtg gcactgcaca 60
   tttggagaga agatgatgtg cttggtttag gaggtgctga gggtgatggc ttgggtaaca 120
   tcctggcaga gacacccagg tggctggaat gtgggtctgg ggctcaggaa tgacgtccga 180
   gttgagctga tgagggtgct gtctgccctc aagggtacag ggagcacatg gagatcagag 240
   ccctcacacg ctgtcctctg ctctgcctat gccaggcgcc ttcgccaagg tgaaggagag 300
   ccaacgcatg agtgacgagg gccgcatggt gcaggacgag gcagacggca ttcgcaggcg 360
   ctgccgcgtg gtgggtttcg ccctgcaggc cgagatgaac cacttccacc agcgccgtga 420
   gctcgacttc aagcacatga tgcagaacta cttgcgccag cagatcctct tctaccagcg 480
   ggtgggccag cagctggaga agaccctgcg catgtatgac aacctctgac cgcgtgtgcc 540
   tgggccccct ccttcccctg ggcctggtca ctgcagtgta ccccactttc ccgacctccc 600
   tataccagca gtgactgggg gaggggtcag cggtggggga gataagcggc ctgtcctgcc 660
   tcctgggaga aggagctttc aaggagtcat gggtgcccct gggaaattcc ccactcctta 720
   gaagtggggc acagcagggg tgagaataga gtcaggagcc ctcgaggcca aggcctgggc 780
   tgccggtcag ccagtgaagg tcaggccagg gtctcagcct cccctagagc ctattttgct 840
   tgctcacctg gccactgctg ccttatccat tcagcagaca ccgaggcctg ctgcaccctt 900
   gggtcggatg ctgggcaccc agggctgtga catgcctgct cttcaggagt cctcagtgaa 960
   ggtcggggtc agacacagac agagtcaatg cagtatgact gatgtttaag tgagggattt 1020
   ctggaagctc atagaaggga ccacagcatt ccactggtca gggaagactc catagagtag 1080
   gcaacatttg ggcagtgttt tgaagaatga caagggcctg ccagacagta catgggggag 1140
   aaggactttc aggggagagg aacagcatgg gcaaagttat ggaggcatgc aaacatctcc 1200
   ctcttctctc ccttactttc caagcaagtt aggtacgctt tccatgggga ttctggcctg 1260
   tgtggtagga agggatctcc cttgctccca tgttgctggc tgtccgtaca tcaccctgtc 1320
   ccctgcagga gggggctaca ggccatctcc ctcctgtagg cctctgactc ccctccactt 1380
   ttgggccctc agcttatctc gggcagggga ccattgcagc atcctcccct cctcggactc 1440
   aaggtgctga ggtataagcc ctgggcccca gatccctggt gacaccttcc tggagaagac 1500
   tctcaaaagt gactgtatat ttgagttcac cagcaataac tccccacact cgaagcaggt 1560
   ccaaacccag gatctcaggg tccttgggct ctgtggcact gtcttcccaa gatccttcct 1620
   gttgcacaat gggaaaccta agaggaaaaa gacaggggcc tgcttgccca gccatgcgag 1680
   ggattccatg cccacctgcc ctctgtctgc ctcgctggaa tgtgggcccc tgctccccgt 1740
   caggttgtgc tgtctctgac ctatgtttac atccccgagg ggtttctgcc tcctccccac 1800
   ccaggtcagg gtgtggtcca gcagcttgct gtggggtgct gacatgtgtc accactgccc 1860
   cccttgcccc cgggggggtc atggtctcct cctggatgct gctccttgaa tcttttttct 1920
   tgataaacct tttacaatta agat 1944

<210> 19
<211> 2343

<212> DNA
<213> Homo sapiens

<400> 19
ggcttctttg ccttgtcttc ctcttttctc catgttcttt ctgtctcctt ttcttgagat 60
agatacctgg tagtacttcc tttctctctt cggggactta ctcccttatg gatggtgcct 120
tagaacattt gagggccaag ctttagataa aatcccaaat aagagaaaga atgtattgga 180
agctgcactg ttttctgagt catttgtcag gataaaagag aaactgctat ttaattcttt 240
tttttttttt tttttttttg agatggagtc tcgctcttgt tgctcaggca ggagtgcaat 300
ggtgtgttct tggctcactg caacctccac gcccggggtt caaacgattc tcctgcctca 360
gcccccgaag tggctgggat tgtaggtgtg caccaccatg ccccgctaac ttttgtattt 420
ttggtggaga tggggtttta ccatgttggg caggctggtc tcgaactcct gacctcaagt 480
gatccgcccg cctggcctcc caaggtgctg ggattacagg catgagccac cgtgcccagc 540
ctgctattta attcttaaat gaaagttaag cctgggtgtg gtggctcatg cctttaatcc 600
cagcactttg ggaggctgag gcgggaggat cacttgaggt cagttgtttc aggccaggca 660
gcaacgtggc aggatcctgt ctctacaaaa aataaaagta aaaaaattat ctgagtgtga 720
tggaatgcac ctctaatcct agctactcag gaggctgagg caaggggatc acttgagtgc 780
aggaattcat ggccgaaatg agctgtcatc accagtgcac tccagcttgg gtgacagagc 840
gagaccctgt ctcaaaaaaa ttttaaaaag catctccaaa gattttcagt ttatttctct 900
tctgtagtac tttaggatgg agctgatgca tgctgaaaaa ctgaggaagg agaaggaaga 960
atttgaaaaa gccagtatgg atgtggagaa tcctgattat tctgaagaaa tccttaaggg 1020
cgagttggca tggatcatct acaaaaattc tgtaagcata attaaaggtg cagaatttca 1080
cgtgtcactg ctttcgattg cacagctatt tgactttgcc aaagatctac aaaaagagat 1140
ttatgatgac cttcaggctc tacacacaga tgatcctctc acttgggatt atgtggcaag 1200
gcgagaatta gagattgagt cacagacaga agagcagcct acaacgaaac aagccaaagc 1260
agtggaggtc ggccggaagg aggagaggtg ctgtgctgtg tatgaagagg cagtgaagac 1320
tctgccaaca gaggccatgt ggaagtgtta catcaccttt tgcttggaaa gatttactaa 1380
gaagtcaaat agtgggttcc ttagagggaa gaggttggaa agaaccatga ctgtattcag 1440
gaaggcacat gaactgaagc ttctgtcaga atgccaatac aagcagttga gtgtttcgtt 1500
gctgtgttat aacttcctga gggaagctct ggaagtggca gtagctggaa ctgaattgtt 1560
tagagactct gggacaatgt ggcagctgaa gctgcaggtg ctgatcgagt caaagagccc 1620
tgacatagcc atgctttttg aagaagcctt tgtgcacctg aaaccccagg tttgtctgcc 1680
attgtggatt tcctgggcag agtggagtga aggtgccaaa agccaagaag acactgaggc 1740
agtctttaag aaagctctct tagctgtcat aggtgccgac tcagtaaccc tgaagaataa 1800
gtacctggat tgggcttatc gaagtggtgg ctacaaaaag gccagagctg tgtttaaaag 1860
tttacaggag agccgaccat tttcagttga ctttttcagg aaaatgattc agtttgaaaa 1920

ggagcaagaa tcctgcaata tggcgaacat aagagaatat tatgagagag ctttgagaga 1980
gtttggatcc gcagattctg atctttggat ggattatatg aaagaagaat tgaaccaccc 2040
ccttggtaga cctgagaact gtggacagat ctactggcga gcgatgaaaa tgttgcaggg 2100
agagtcagca gaggcatttg tagctaaaca tgctatgcat cagactggcc atttatgaag 2160
atgaagaata cagtcagctt tgtgaaatag tattgcaagc aagccccgtg ggcaaatttg 2220
tattgagtcc atctgtaatt tgctcagtga tggcagacaa gatggctgtc tggttttgag 2280
acacacttta attttatgtt aacttgttaa atctttttaa aaattaaaaa atttttatga 2340
ttg 2343

<210> 20
<211> 2762
<212> DNA
<213> Homo sapiens

<400> 20
ttttgggtcg ccttccatgc tgtggaagct ttgttctttt gctctttgca ataaatcttg 60
ctgctgctca ctctttgggt ctgtgccgcc tttatgaggt gtaacactca ccgcgaaggt 120
ctgcagcttc attcctgaag tcaccgagac caggaaccca ccaggaggaa tgaacaactc 180
cagacatgcc gcctttatga actgtaacac tcaccgcgaa ggtctgcagc ttcactcttg 240
aagtcagcga gaccacgaac ccaccagaag gaagaaactc tggacatgtc cgaacatcag 300
aaggaacaaa ctctggacac accatcttta agaactgtaa cactcactgt gagggtccat 360
ggcttcattc ttgaagtcag caagaccaag aacccaccaa ttccagacac atttggccca 420
cccagatggg accatcgccc atcgccaggc ggtgagacta atgcctattg ccaaatgaat 480
catgtcaacc ctgccatgga cttcacgcag actccacctg ggatgttggc tctggacaac 540
atgctgtact ttgccaagca ccaccaagat gcctacatcc ggattgtgct tgagaacagt 600
agtcgagaag acaagcatga atgtcccttt ggccgcagta gtatagagct gaccaagatg 660
ctatgtgaga tcttgaaagt gggcgagttg cctagtgaga cctgcaacga cttccacccg 720
atgttcttca cccacgacag atcctttgag gagtttttct gcatctgtat ccagctcctg 780
aacaagacat ggaaggaaat gagggcaact tctgaagact tcaacaaggt aatgcaggtg 840
gtgaaggagc aggttatgag agcacttaca accaagccta gctccctgga ccagttcaag 900
agcaaactgc agaacctgag ctacactgag atcctgaaaa tccgccagtc cgagaggatg 960
aaccaggaag atttccagtc ccgcccgatt ttggaactaa aggagaagat tcagccagaa 1020
atcttagagc tgatcaaaca gcaacgcctg aaccgccttg tggaagggac ctgctttagg 1080
aaactcaatg cccggcggag gcaagacaag ttttggtatt gtcggctttc gccaaatcac 1140
aaagtcctgc attacggaga cttagaagag agtcctcagg gagaagtgcc ccacgattcc 1200

ttgcaggaca aactgccggt ggcagatatc aaagccgtgg tgacgggaaa ggactgccct 1260
catatgaaag agaaaggtgc ccttaaacaa aacaaggagg tgcttgaact cgctttctcc 1320
atcttgtatg actcaaactg ccaactgaac ttcatcgctc ctgacaagca tgagtactgt 1380
atctggacgg atggactgaa tgcgctactc gggaaggaca tgatgagcga cctgacgcgg 1440
aatgacctgg acaccctgct cagcatggaa atcaagctcc gcctcctgga cctggaaaac 1500
atccagatcc ctgacgcacc tccgccgatt cccaaggagc ccagcaacta tgacttcgtc 1560
tatgactgta actgaagtgg ccgggcccag acatgcccct tccaaaactg gaacacctag 1620
ctaacaggag agaggaatga aaacacaccc acgccttgga accgtccttt ggtaaaggga 1680
agctgtgggt ccacattccc ttcagcatca cctctagccc tggcaacttt cagcccctag 1740
ctggcatctt gctcaccgcc ctgattctgt tcctcggctc cactgcttca ggtcacttcc 1800
catggctgca gtccactggt gggacaagag caaagcccac tgccagtaag aaggccaaag 1860
ggcccttcca tcctagccct ctgcaggcat gcccttcctt cccttgggca ggaaagccag 1920
cagccccaga ctgcccaaaa acttgcccac cagaccaagg gcagtgcccc aaggcccctg 1980
tctggaggaa atggcctagc tatttgatga gaagaccaaa ccccacatcc tcctttcccc 2040
tctctctaga atcatctcgc accaccagtt acacttgaat taagatctgc gctcaaatct 2100
cctcccacct ctctccctgc ttttgccttg ctctgttcct ctttggtccc aagagcagca 2160
gccgcagcct cctcgtgatc ctccctagca taaatttccc aaacagtcca caggtcccat 2220
gcccactttg cgtctgcact gtgatcgtga caaatcttcc ctcctcacca gctagtctgg 2280
ggtttcctct ccctgcccca ggccagaact gccttcttca tttccaccca cgctcccagc 2340
ctcttagctg aaagcacaaa tggtgaaatc agtagtctcg ctccatctct aatagactaa 2400
acctaaatgc ctctaggacg gactgttgct atccaagcgt ttggtgttac cttctcctgg 2460
gaggtcctgc tgcaactcaa gttccacagg atggtcaagc tgtcagacat ccaagtttac 2520
atcattgtaa ttattactgg tatttacaat ttgcaagagt tttgggttag tttttttttt 2580
tttttgcttt gtttttgtac aaaagagtct aacatttttt gccaaacaga tatatattta 2640
atgaaaagaa gagatacata aatgtgtgaa tttccagttt ttttttaatt attttaatcc 2700
caaacatctt cctgaaaata acattccctt aaacatgctg tggaataaaa tggattgtga 2760
tg 2762

<210> 21
<211> 3660
<212> DNA
<213> Homo sapiens

<400> 21
tttacaatcc cttagctaga cataaacgtt ctccaagtcc ccaccagatt agctagatac 60

agagtgctga ttggtgcatt tacagtccct cagctagaca taaatgttct ccaaatcccc 120
actagactca ggagctcagc tggcttcacc cagtggatcc tgcaccaggg ccgcaggcgg 180
agcactcctc agcccttggg cagttgatgg gaccaggcgc cgccgagcag tgggtggcgc 240
tcctcgggga ggctcgggct gtgcaggagc ccacggcggc gggggaggc tcgggcatgg 300
caggctgcag gtcccaagcc ctgccccaca gggaggcagc tgaggcccgg caagtattcg 360
agtgcagcac cagcgggccg gcactgctgg gggacccggt gtaccctccg cagctgctgg 420
cccaggtgct aagcccctcg ctgctcaggg caggtggcgc cggcccacca ctccgagtgc 480
ggggtcgccg agctcacacc cacccggaac ttgcgctggc cggagagcac tgcgtgcagc 540
ctgggttccc gcccgcgcct ctccctccac acctccctgc aagcagaggg agccggctcc 600
gatcttggcc agcccagaga ggggctccca tagtgcagca gcaggctgaa gggctcctca 660
agcatggcca gagtgggctc tgaggccagc gagcaagggc tgccagcatg ctgtcacctc 720
tcacttcctt ctctctctct ttctttcatt tttgagacag ggtctccctc tgtcacccag 780
gctggagtgc agtggcgcga tcatagctga ccgcagcctg caactcctgg gctcaaacga 840
tccgcccacc tcagcctccg aaagtgctgg gattacaggt gaccatgccc agcctcatgt 900
gcagctaatt ttttcattgt ttgtagagaa gaggtcttgc aatgttgccc aggctgttct 960
caaaatctta gcctcaagcc atcctcctgc ctctgccttc caaagtgctg ggattgcagg 1020
tgtgagccac tacgcccagc cccaaaatat actttttat tcatttgttt gcctccccat 1080
gaggacaggg actgttgtct gtattgatca tttattgagc agcttgtgtg cgccaggccc 1140
tgtgctaggc ccaggggatg cagcagtgga cagaaagaca aaaactcctg tgttctcaga 1200
gctcacattc tagaaaggga gtcagaccct caagcagata aggacctaat tataggatag 1260
cagctgtgca gcccggaggt aaggcggggt caggatgcgg gggatggggc catcgttgag 1320
ccaggttggc cagggagggc ctctctagga ggtgaccttt gagctatagc tgaaggaagt 1380
gaaggagtca gtctgtggct gttgaagagg gagggatggt tgtcagccca aaggtccttg 1440
gctccctccc tctcctctgg gtctttactc aaggtcacct tctcaatgag aagattctgt 1500
accactgcac agccctcagg cagtgatctg gatcctattt ccacttgtct tttcacctct 1560
ctgtcctgcc attttctttt ctttttattt tttattttg agcctgggcg acaagagcga 1620
gactctgtct gaaataaaaa aaataaaata aaaaaaaaga ggatctgacc caagaaggtg 1680
tccactggct tcccctggct gcctgtaggg aatagactgg ggacacaggt ggagacaggg 1740
agagcagggt gggctcatgg agaacaactg atgcaggaaa ggtcagattc aggatcatct 1800
tgaagttgga gccacagaaa ttgctgcatc gtgagatgtt tctcatctca ggaaacatca 1860
tcaccattga ctgcattgct cacgctgcag accttgagtc ccgggctgtc tggcatcttg 1920
gggaaaagca tggcctctgg agccagctgc atggttccag attttaaaat cctacttctg 1980
atgcggcatg gtggctcaca cgtgtaatcc cagtgctttg ggatgccaag gtgggaggat 2040
cgcttgagcc caggagttca agagcagcct cggcacagtc ccagccactc aggaggctga 2100
ggtgggagga ttgcttgaac ctgggagttc gaggctgcag tgagccatga tcgtgccact 2160
gcactctagc tggtgtaaca gagtgagacc ctgtctctaa aaacattaat gagttaattt 2220

aaatagttca ttactcacta ctcaccagcc agtgtgaccc ccttgggcaa gtgcttaacc 2280
tctctgtgcc ttagtttacc cattggtaac acaaatagta ataggaccta gctggtacgg 2340
ttgctgtgag gattctgtga gtaatgactc cacagatatt agcttttatt tccttcccac 2400
ccaacatgca atcaatccct caccaagtcc tgtgtgagtt gtttttttc cgccttggtg 2460
gtttgggggt tcgaacctgc tgtgcctggg ttctgatccc tggttgagtc aatgggtggg 2520
gcctgggagc ctggatgagc tccatcatgc cttctcacca ggtattgacc tgatggacat 2580
ggcttcggac atcctgcagc ccaaaggaga tgatgtggcc cggatcagct ggtacctccg 2640
tgacatcatc actcgatacc aggagacctt caacgtcatc gagagggtga ctctaggggt 2700
ctggagggga tctttctcca tacgcgcctc ccctgggcca gccctgaccc cactcttcct 2760
gttccctctc agtgccccaa gcccgtgatt gctgccgtcc atgggggctg cattggcgga 2820
ggtgagtctg cggctatcct cctgctcggg tgctccccag gtggggctgc tgctccgatg 2880
ccgcggccac tggcatccag cctcagctct gtcatgggcc agactgtgtc ccaagaggca 2940
gccccacctc ccgggagcca gggttggttc tggtggtcat tcagcatccc tggcctctac 3000
ctcctaggtg tggaccttgt caccgcctgt gacatccggt actgtgccca ggatgctttc 3060
ttccaggtga aggaggtgga cgtgggtttg gctgccgatg taggaacact gcagcgcctg 3120
cccaaggtca tcaggaacca gagcctggtc aacgagctgg ccttcaccgc ccgcaagatg 3180
atggctgacg aggccctggg cagtgggctg gtcagccggg tgttcccaga caaagaggtc 3240
atgctggatg ctgccttagc gctggcggcc gagatttcca gcaagagccc cgtggcggtg 3300
cagagcacca aggtcaacct gctgtattcc cgcgaccatt cggtggccga gagcctcaac 3360
tacgtggcgt cctggaacat gagcatgctg cagacccaag acctcgtgaa gtcggtccag 3420
gccacgactg agaacaagga actgaaaacc gtcaccttct ccaagctctg agagccctcg 3480
cgtcccaggc cccagccagg gggccggcct tgtcccgcct catccacaga aagggaggat 3540
gggcgatgac agttgtttct atgccttctg acccagtttc ccagtttata actttatgac 3600
aatgagtttc tcaagcccaa ggccttatct tcaccccaca aacaataaag caaagtaaag 3660

<210> 22
<211> 1572
<212> DNA
<213> Homo sapiens

<400> 22
aagataaggc ggcgcgggaa gtggacacag ggtgggctgg agatctaact ggactctcgc 60
tcctgctggc tggacatgga ggatttggag gaagatgtaa ggtttattgt ggatgagacc 120
ttggactttg gggggctgtc accatctgac agccgtgagg aggaagacat aacagtgttg 180
gtgactccag agaaaccact tcgacggggc ctctcccacc gaagtgaccc aaatgcagtg 240

gcacctgccc cccagggtgt gaggctcagc ctaggccccc tcagtccaga gaagctggag 300
gagatcctcg atgaggccaa ccggctggcc gctcagctgg agcagtgtgc cctgcaggat 360
cgggagagcg caggcgaggg cctggggcct cgccgagtga agcccagtcc tcggcgggag 420
acctttgtgc tgaaggatag tcctgtccga gacctgctgc ccactgtgaa ctctttgacg 480
cggagcaccc cctccccaag cagcctgacg cctcgactcc ggagtaatga taggaagggg 540
tcagtcaggg ctctccgggc tacatctgga aagaggccct ccaacatgaa gagggagtca 600
cccacttgca atctgttccc tgcatccaaa agcccagcat cttctcctct tacccgatcg 660
actcccccag tccgggggag agccgggccc agtgggagag cagcagccag tgaggagacc 720
agagcagcca agttgcgggc ctgccagccg aatgccactc accagccgga gtgtgccacc 780
tggcagaggt gccctacctc cggattctct gtcaactcga aaagggcttc caagaccaag 840
cactgcagga cacagagtgc gggaaagtgg acacaaggtt cctgtttccc agcgactaaa 900
tcttcctgtc atgggtgcca ctcgcagcaa tctgcagccc cccaggaaag tggcagtccc 960
aggacctacc aggtaaagag atcaggacag caagcaagac ttcagtagca aaccactaca 1020
gtcagtacct ggactcgcct ctacccagca gaccctgact ccagcagatt ctggcccagg 1080
gacaggagga agagatgcca ccagggctgg tctcccagga gtagagacca tgggaaatgg 1140
ggtggattag gattgagctg gagaagactt aaactctctg ggttgaaaga agattagggg 1200
aaaagaggtc accttccagc agtgaaatga acaaatagaa gatgagaagt acaggcaagt 1260
ggtttgtctt tatccacccc cactgttgtg gtcagcccca gagaatttta tcttcttcct 1320
tggcattggt tcactggaca tttccacgtg agcggcctcc gtagctaacc tccctgccct 1380
ctgaggagcc atcttcctga atcgcattct ctactggact ctggcctgct tggagaggtg 1440
gcagcaggca cctggtcttc agaaattgtt tcctgtgaat tctgtgactc ctaataggcc 1500
agtttgtgat aagcttactc tatgagtctt catttttcta aaataaagtg aatgtatttt 1560
tatattctct gt 1572

<210> 23
<211> 2254
<212> DNA
<213> Homo sapiens

<400> 23
agttgcgcgc gtggctctgg ctgcgcagga acagctggtg cctccgaggg cggtcggcga 60
gcgcgcgggc gtggggcgct gggggccgg tcgggcagcg ctgcgggagc agccgccggc 120
accgccgcct tgcaccatcg catcatgtcc gggcagctgg agcgttgcga gcgcgaatgg 180
cacgagctgg agggagaatt tcaagaactg caggagacgc acaggatcta caagcagaag 240
ctggaggagc tggctgcgct gcagacgctg tgtagcagtt ccatcagtaa gcagaagaag 300

cacctcaagg acttgaagct tacactccag aggtgcaaac gccatgccag tcgggaggag 360
gcggagctcg ttcagcagat ggcagcgaac atcaaggagc ggcaggacgt cttcttcgac 420
atggaggcct acctgcccaa gaagaacggg ctctacttga acctggtcct cggcaatgtg 480
aacgtgaccc tcctcagcaa ccaggccaag ttcgcctaca aggacgaata tgagaagttc 540
aagctctacc tgaccatcat cctgctcctg ggtgccgtgg catgtcgatt tgtccttcac 600
tacagggtga ctgacgaagt cttcaacttc ctgctggtgt ggtattactg caccctgacc 660
attcgggaga gcattctcat cagcaacggc tcaagaatta aaggctggtg ggtgtctcac 720
cactacgtct ccacattcct gtccggagtg atgctgacct ggcctaatgg acccatttat 780
cagaagtttc gcaaccagtt cttagcattt tccatttttc agagctgcgt ccagttcctg 840
caatattatt accagagggg ctgcctctac cggctgcggg ccctggggga gaggaaccac 900
ctggatctca cagtggaagg gttccagtcc tggatgtggc ggggcctcac ctttctcctg 960
cccttcctct tctgtggcca tttctggcag ctctacaatg ccgtcacgct gtttgagctc 1020
tccagccacg aggaatgcag agaatggcag gtgttcgtac tggcgttcac cttcctcatc 1080
ctcttcctcg gcaacttcct gaccacgctc aaagtcgtgc atgccaagct ccagaagaac 1140
agaggcaaga caaagcagcc gtgagcctcg ggctcctgtg ccctcggccc ggacttcaga 1200
ctgcaggggg ctcccgggct ccttcccagc agccctctca ggcccgtggc atcgctggga 1260
gagggcccag gccctggtcc cccagtggac cccagtggtc tagaggaatg tgagccccgc 1320
ctgtccgcac agtgtccgcc cacctattta tgacatattt aatgctgggt cccccatcgt 1380
ccctggaacc cgaggcctca ctcctgtgct tgaaggtggc tgaggccggg ccagtcttcc 1440
tggggatggg gcctgaagcc tcagggagcc cctctgttcc cactcctgtc atttgaaccc 1500
ctctgggtgg ggtttggatg tgcctcgcgg ggttggattt atgctgacct gctacttacc 1560
aggcccaggc tggggtggtg tgaaccctca gtgtcctgtg gcgcccccac ccgggggcca 1620
ctctgcttct gctgtgagcc ccctcctcgc ccaccccgcc acgtggtgag ggttatttta 1680
agttctcaga gacccaccgc ctcgtgctct tcttgcccct ggagcgctgg gggcatcctg 1740
agtcaggctg tgagaagatt cgccaccaga gggcgccccg ggccctgggt cgtccaaggg 1800
gacaaggacg ttcccgtctg tgcttcgggg ttccctgacc cccccatcct gcagcccacc 1860
ttcctgcagt gtcgtggcag gcgacaccga ctcctaggcg ccctccagag ccaagcagct 1920
tgcgacttct ggtaggcacc ggaatcccct gggatgcttg ttttaaaagt cctgggccgg 1980
gcacggtggc tcgcgcctgt agtcccagca ctttgggggg ctggggcggg tggatcgcga 2040
ggtcgggagt tcgggaccgg cctggccagc atggtgaggc cctgtctcta ctggaagtac 2100
aaaaattagc cggacgtggt ggtgtgcgca tgtggtcccg gctgctcggg aggctgaggc 2160
gggggagtcg cttgggcccg gggggtggag cttgcagtgg gccgagatcg tgccgctgcg 2220
ctctagcctg agtgacagag tgagactctg tccc 2254

<210> 24

<211> 2977

<212> DNA

<213> Homo sapiens

<400> 24

aagcagaggc gttggcggcg tgaggctcag gactgtacat cagaggaagc caaaagccag 60

ctggaatact tcctgaatac agttttaatc ttgtttgcaa ttatttcgaa ggggagaaat 120

catctggatc agaattaaga tgctctggtt tcaaggaaat agcatgcaac ttgccagatc 180

ctcctttgga ctcttcttga gaaattgctc tgcctctaag acaactctgc ctgtgttgac 240

cttattcaca aaggagtttc tcagcacaag gtctctcttt gcctgctgcc atccatgtaa 300

gatgtgactt gctcctcctt gccttccgcc atcattatga ggcctcccca gccatgtgga 360

actgacccat gccccctttg tgatgaagcc aaggaagtac tcaagcctta tgaaaacggg 420

caaccctaca aggatcagaa gctgccagga acaaggagaa gacgctcccc ttcctcacct 480

tcccaccccc acatggcttc tcagtcgggg aaaagataca acttaacttt aaaccaagtc 540

ttaagttttg attatgacat gggattagat gcaccaaaaa caatttcaag tgactgtggg 600

gcattctatt gcctgagaat gttcaagagt cctgatatga cctgttgttt ttatcctaaa 660

cagtgaaaaa ttcatccacc aaatatatag gaatagaggg agacagaaag atgctttctg 720

aaggtatccc atatttagag tattggttac atatacctaa tgttggtttt aacctatagc 780

tcttcaataa acttactcat caattcttat ttcagagagg aatatggtca tgtgactagg 840

aatcctgcag atcttcagca aacccatggg tcccaacagc tggcaaaatg aaaggtagat 900

acctacaaac atggcatcta aaaggtggtt tgattatccc actgtcaagt atcagtgtta 960

tgtcacctat tactgtgatt ttcatgtgat gtttgtagca gcaattactt ttcctttctg 1020

aaattatgtt tgcaaagatc aatttgtttt tatataaaga ttaactttga tataagaagc 1080

tcatcattca cctccaaagg aaaaggtgat acaagcttcc ttttttttccc ccttcaagct 1140

aatgccttat gaaggagtgg aatagtttgg gacttcatat tgctgctcta gaatagtgcc 1200

tctttcttcc acatgtctct tcataacatt atgagcttac tggaacacat agctctttgg 1260

ccagtttatt gcacgaaaca agggtatact cagactgggg ttttttaaat aatgcagcaa 1320

tctcatggac atctgacagt aggaatcaca atacagccag gcttcagaag tacaattaaa 1380

gaaccaccca aaactgaaac atcacccttc tcacagtgcc ttggcttatt ttcttccttg 1440

ttctctcctg gctcacagct cccctgctta cacacactat ctgcttcctc atagctgcag 1500

cctgcttatg cccatcatta ctgctaacct ttctgttcag caatacagca ccctgagcct 1560

ctcaggcttc tctattctga aatcctaaaa ggaattttac tcagcttata ttttacaacc 1620

aggcacccat gacctaagtt ggctgaccaa atctatagaa ggagccacct ttaggacaga 1680

agcctatccc tgggccaact ggctatggaa gcggtatgca aatcacagtc actgcccact 1740

ggcagaggcc agaagcagga caggcaatga ccagcaactc tggcacgcct ccaaaaagct 1800

taagaatgtg acattctacc atactcaaga gaagtcagga gggacacatt ggtaagttca 1860

cttatatcct actccatagg agtaataagg acatgaacca aagcaatggg agtcagacag 1920
aaagagatat caggagatat ttaggagtga ggttgacaaa acttcatgac tgactggatg 1980
ggtcggtagt gatgtggaga ggggagtaga gagaaagcat ggattctggg ataactccca 2040
gttttcttgc ttacactact gggtgaattc tgatactttt tattccctga ggcagagaat 2100
gtaagaggaa ggaaaagtta gttttgggct gtgcttggtg gatgggaaag gtaagacatg 2160
gtgaattctc catgcttgtg ggatatcaag gtaaagataa aagttagaag tgcaaaacaa 2220
aggtctgggg ttaaggtata catttgagag cccactgagt atggttggaa atgtggccac 2280
ctggcatata aggagaaaag caagctattc ccaaaccatg gagaatacta atcttgaccc 2340
atgagaatag aggaagttgc tagtgaagga aaatgagaga gtggtggtgg aactaggaat 2400
gggagaagct catccaattt acattacgtg atgccttttg tgatttgata gtggcaggta 2460
ttattccaag cactggggat aaaacaacaa acaagatagc caggattcct actctcagag 2520
tcttgcattt taagggagag acagacaata aagatatcgt atcagaggaa aataatcatg 2580
aaagtcaggg aggagctttt cccagagtaa ctgacagtat caaatagcaa gagagttagg 2640
atgaggactg aacagaagac atcagatttg aaaattagaa aatcagtgct gctataggaa 2700
gaatagtccc caaagatacc caggtcctaa ttcctggaac ctgtcactta tatgcataaa 2760
ggactttgca gatttaagtt aaggatcttg agatgggaaa attatattgc attatctaag 2820
caggtcctaa tgtaatcaca ggtgtctcta taagagggag tcagaaggag atttgactag 2880
ggaaaagaga gtaggaaatt cgacaatgga agcaaaaggt tggagtgata caagaaagca 2940
gccatgagcc aaggaatgtg aatggcctcc agaagct 2977

<210> 25
<211> 2166
<212> DNA
<213> Homo sapiens

<400> 25
gtcagagtgc ctagaaagag ccatgaagtt tgcctttgag gaattccacc tgtggtacca 60
gtttgctctg tccctgatgg ctgctggaaa atctgcccgt gccgtgaagg tgctgaaaga 120
gtgtatccgc ctgaagccgg acgatgccac catccctctc ctcgctgcca agctctgcat 180
gggctccctg cactggttgg aagaggctga aaagtttgcc aaaactgtcg ttgatgtggg 240
agagaaaacg tcagagttca aggccaaagg ctacttagct ctggggctca cgtacagtct 300
gcaggccact gacgcttctt tgcgagggat gcaggaggtc ctacagagaa aggcgcttct 360
tgcatttcag agggcccaca gcctgtcacc cacagatcac caagcagctt tctacctggc 420
tctgcagctt gccatctcca gacagatccc agaggctctg gggtatgtcc gccaagctct 480
tcagcttcaa ggtgacgatg ccaactccct gcacctcctt gccctcctgc tgtcagcaca 540

gaagcattac catgacgctc tgaacatcat cgacatggcc ctgagtgaat acccagaaaa 600
tttcatacta ctgttttcca aagtgaagtt gcagtcactc tgccgaggcc cggacgaggc 660
actgctgact tgtaagcaca tgctgcagat atggaaatcc tgctacaacc tcaccaaccc 720
cagtgattct ggacgtggga gcagcctctt agatagaacc attgctgaca gacgacagct 780
taatacaatt actttgccag acttcagcga tcccgagaca ggctccgtcc atgccacatc 840
ggtagcagcc tcaagagtgg agcaggcact gtcggaagtg gcttcgtctc tgcagagcag 900
tgcccctaag cagggcccgc tgcacccctg gatgacgctg gcacagatct ggctccatgc 960
agctgaagtc tatatcggca tcgggaagcc tgcagaagcc acagcctgta cccaagaagc 1020
tgccaacctc ttcccaatgt cccacaatgt cctctacatg cgcggccaga ttgctgagct 1080
ccggggaagc atggacgagg cgcggcggtg gtatgaagag gccttagcca tcagccccac 1140
ccacgtgaag agcatgcagc gactggccct gatccttcac cagctaggcc gctacagtct 1200
ggcggagaag atcctccggg acgcggtgca ggtgaactcg acagcccacg aggtctggaa 1260
cgggctgggc gaggtcctcc aagctcaggg caacgatgcg gcggctacgg agtgcttcct 1320
gacagccttg gagctggagg ccagcagccc cgccgtgccc ttcaccatca tcccccgcgt 1380
gctctgagca ggcgcctgcc agcctcacct gccgctcagg cctcagaggc cctgccgggc 1440
accagggctt gtgccatcgc cccaagggga tgaatctgcc gcactgaggc cagggacgag 1500
tgttcagtgg gccacagtga accaaccaaa ccaaccccga atcatcgctc tcgccatgtg 1560
cgtttctctt gtttttttg ccagcccaat ggtagtttct gaacctattg acattgttca 1620
aaatggatca tgtgccatat tttgttagtt gacatctgag ttttcagtaa aatgattatg 1680
gaattaatca gcaaatgtag aagaatatat tcaaagttaa aattcagtgg cagcacagat 1740

tatttttatc agagctgtaa agaaaacaac tgtccttttc tccccaccac ccctcctgcc 1800
ccactttggc ccagaaacca aatgtgaact tcctgtctcc cacctcagca ctagtccatg 1860
ccaggacacc agctgacaat ttcttggttt tactgtcaat aattgtacca tgtgatcaat 1920
tactgtcctc acttagaaca aagcctgagt ccgagaatat ttatatttta ccaatatatg 1980
cctgttacaa gagaaggaaa tatgagttat ttaagtttaa ctttttatg tgaattcaga 2040
gtttatttat cgagggaaat atgtacaaag aagcttcaaa tggaatattt accgacattc 2100
cttatacatg acagacactt ggccacatgg gaagatgatg ttaataataa aatgattttt 2160
aaatgg 2166

<210> 26
<211> 3120
<212> DNA
<213> Homo sapiens

<400> 26
   gctccgcgct ctcctgccgc tccgctccgg gtctcccgcg ctcctctccc cggctcggcc 60
   gagcgcgctg ccccgacgcc gccacccaga gccgggccgc gccgggcgcc gagatgaagg 120
   tgctgggaca ccggctggag ctgctcacag gcctcctgct ccacgacgtg accatggccg 180
   ggctgcagga gctgcgattc cctgaggaga agccgctgct ccggggccag gacgccaccg 240
   agctggagag ctccgatgcc ttcctcttgg ctgcagacac agactggaag gaacatgaca 300
   tcgagacacc ctacggcctt ctgcatgtag tgatccgggg ctcccccaag gggaaccgcc 360
   cagccatcct cacctaccat gatgtgggcc tcaaccacaa actatgcttc aacaccttct 420
   tcaacttcga ggacatgcag gagatcacca agcactttgt ggtgtgtcac gtggatgccc 480
   ctggacaaca ggtgggggcg tcgcagtttc ctcaggggta ccagttcccc tccatggagc 540
   agctggctgc catgctcccc agcgtggtgc agcatttcgg gttcaagtat gtgattggca 600
   tcggagtggg cgccggagcc tatgtgctgg ccaagtttgc actcatcttc cccgacctgg 660
   tggaggggct ggtgctggtg aacatcgacc ccaatggcaa aggctggata gactgggctg 720
   ccaccaagct ctccggccta actagcactt tacccgacac ggtgctctcc cacctcttca 780
   gccaggagga gctggtgaac aacacagagt tggtgcagag ctaccggcag cagattggga 840
   acgtggtgaa ccaggccaac ctgcagctct tctggaacat gtacaacagc cgcagagacc 900
   tggacattaa ccggcctgga acggtgccca atgccaagac gctccgctgc cccgtgatgc 960
   tggtggttgg ggataatgca cccgctgagg acggggtggt ggagtgcaac tccaaactgg 1020
   acccgaccac tacgaccttc ctgaagatgg cagactctgg agggctgccc caggtcacac 1080
   agccagggaa gctgactgaa gccttcaaat acttcctgca aggcatgggc tacatgccct 1140
   cagccagcat gacccgcctg gcacgctccc gcactgcatc cctcaccagt gccagctcgg 1200
   tggatggcag ccgcccacag gcctgcaccc actcggagag cagcgagggg ctgggccagg 1260
   tcaaccacac catggaggtg tcctgttgaa gcccttgatc ccgctgacga cgcccacgtc 1320
   gaggccccac cgccatcctt gcgccggctc atgttccctt tagtttattt ttgtgagggc 1380
   aaaggggagg aaatggggtt ctgtttgaaa aaaatgaggg gatcttagat gctgcagcag 1440
   aacagtctcc aggtgtttta aggggctcag tcctcctcat cccatctcac tctccgtggt 1500
   aacttagcca acttgacccc tctcatccca ctcccggcgg cccaggcaca gaagggcagg 1560
   gccataggga gggagattcg ctacggatcc aggccattcc tgggtgagcc cttgggcagg 1620
   catgtttgga gatgagagag gcttcgagag ggtgggtgct gggccacagg ggtgcggggc 1680
   cagctcaggc actggcgtgg gagccctggg agaccccttc ccccaccctc caccaagcac 1740
   acctgtttct gtctcatagc acatgtgaca atcatctgga caacagccac aagggggcgc 1800
   tcggaccagg cagccacttt cctggtgctc tctgggccca gctggtgctg tagggccacg 1860
   caggcagggg cgtcaagggg tttctctgcc caaggaagac agaacatgga gaaccgtcag 1920
   ggcaggaacc ccacagactg tcccttccag cccacactct gccacctcct ggccctgtcc 1980
   caattctgag ccaaggcctc cccgaggcag aagttgcctg gtcctctgtc cccacagtga 2040
   cctgactggg ggtgagggag aaggaggaga gagcccatgt gtggtgtgtg tgcccctgag 2100
   aacttcgtgg tgactgcctt tgggagcccg caggtggcca gaggcagggg tagctgagtt 2160
   cctggagacc ccttttttgc ccccaggttc cccagagggc aacgccatca gtagcagtgt 2220
   ggtgtttcag gcagagctct ggccaggctg tgccagtgtg tcccggacgc atcactaagg 2280
   aagagagagt ttatttagtc aactggccca aggcagcgag gcttctacag tcccacaccc 2340
   catagccgcc tgggctgggg cttactgggg gctgaaggtt ctggacatga acaagggtca 2400
   ggtagaagag aaaggcttcc cctacacccc agcctcctgc tgtcccctga agcccaggac 2460
   tgcgttgtat gctttccatc cactcacctt accccatagc atcttgcggc ccagaaacca 2520
   gagccatttg tctcagaccc taaatcaata atcacaaacc ccaaaacggg agagagcagt 2580
   gaaaacatgc agggctgtgg acgggggaag ggttgtggcg ggtgttctga ggctgagagg 2640
   acacctatat gcgtatttcc tctacacaca tcacccccct tctataatct taagccatga 2700
   ctagcctggt ggcgtgttag tttctgccca gttctacccc ctcatgtgct tcttctgaat 2760
   actgaatgtg actgtttgaa agctggtaga attcatccct cttactgtag ataacactgc 2820
   aaatcttgga attttgtttt ttgctgtttc cagatgtatc tataaatatc tatacattat 2880
   atgtgtgtgt gtgtgtgtgt gtgtacatcg ggtcctccca tgtgtggtgt tcttctggag 2940
   gttgtctctt tggtcaaggt gaacttttaa tgtttattat tttcttctcc gcacaaagta 3000
   aagagcctaa ttttgtgtat tctggtggct gctgtcatga gatgataaaa tgtaaaacaa 3060
   aactctagtc aacgtagaaa gagttaactg tgctgaaaaa ctaataaaga acctaagaag 3120

<210> 27
<211> 1671
<212> DNA
<213> Homo sapiens

<400> 27
aaaggcttgg gtgtgagaca gcagcggtgg cagacaccgc agaagcaaag agcagtgagg 60
ctcctgcatt cgggtggagc accatggacg aagctggcag ctctgcgagc ggcgggggct 120
tccgcccggg cgtggacagc ctggacgaac cgcccaacag ccgcatcttc cttgtgatca 180
gcaagtacac acctgagtcg gtgctgaggg agcgcttctc gccttttggc gacatccagg 240
acatctgggt ggtgcgggac aagcacacca agcccatcaa ggttttcatt gctcagtccc 300
gatcatctgg aagtcaccga gatgttgaag atgaagaact tacaagaatc tttgttatga 360
taccaaagtc ctacacagaa gaagatctgc gggaaaaatt taaggtgtat ggagatatcg 420
agtattgcag cattattaag aataaagtga ctggagaaag taaaggtttg ggctacgtac 480
gatacttaaa accatcacaa gctgcccaag caatagaaaa ctgtgatcga agttttagag 540
caatcttggc tgaacctaaa aataaagcat ctgaatcctc tgaacaagat tattatagta 600
atatgaggca agaagctttg ggacatgaac ctagagtaaa tatgtttcca tttgaacaac 660

aatctgaatt ttcaagtttt gacaagaatg atagccgagg ccaggaagca atctccaaac 720
gcttgtcagt tgtatcaaga gttcctttca ctgaagaaca gcttttcagc atttttgata 780
tagtaccagg attggaatat tgtgaagttc aacgagatcc ttattcaaat tatggtcatg 840
gagtggttca gtattttaat gtagcatcag ctatttatgc aaaatacaaa ttacatggat 900
ttcagtaccc tcctgggaac cgaataggtg tttccttcat tgatgatgga agtaatgcaa 960
cagatctcct tagaaaaatg gcaacacaga tggtagctgc acagcttgca tcaatggtgt 1020
ggaataaccc aagtcagcaa caatttatgc aatttggagg aagctctgga tcacagttgc 1080
ctcaaatcca gacagatgtt gtacttccat catgcaaaaa aaaagctcct gctgaaactc 1140
ctgtgaaaga aagacttttt attgtgttta atcctcatcc tttaccttta gacgtattag 1200
aagatatatt ctgtcgtttt ggtaacctga tcgaagttta ccttgtgtca ggaaaaaatg 1260
tggggtatgc caagtatgcc gatagaataa gtgctaatga tgccattgcc actctacatg 1320
gaaagattct gaatggggtg agacttaaag ttatgctggc agattcgcca agagaagaat 1380
ctaacaaacg gcaaagaact tactgattct tgagaacaaa gactaaataa tgacataatc 1440
ctcagctgac tgactgaaaa tgtgactgga cgcattccct gtggacagtt gacagctttt 1500
tttttttcca tatacctgat agtctgtgta cagcattgtt ttgtctggga agcagggatt 1560
gctgacatgt atttttgaat ccatacatta atgctaaaac gaatatagta gttgttcctt 1620
agagcaatat gttgttacgt gtagcagaaa taaagttttc tttgcttaac t 1671

<210> 28
<211> 2148
<212> DNA
<213> Homo sapiens

<400> 28
acactcagtc tctgatttta tccctgccct ggcttaatta agtgctctga agacataaat 60
aggttttttg tttactcaag cttgggagaa gctggcagaa aaggcctttt cagctaaaag 120
ctccttacag ccggaaggtg tgaagcggca agtttccagc cgggaagaag cctctcagcc 180
gtaggcgtct ttgcccggag ctgtgagccc ccctcccaac tcccaaatcc cccggcgtcg 240
gagatgaggc cccggatgct gccagtgttc tttggggaga gcatcaaggt gagcccggaa 300
cccacgcatg agatccgctg caactctgag gtcaagtacg cctcggagaa gcatttccag 360
gacaaggtct tctatgcgcc cgtacccacc gtcacggcct acagcgagac catcgtggca 420
gcacccaact gcacgtggcg caactaccgc agccagctga ccctggagcc acgcccgcgc 480
gccctgcgct tccgcagcac caccatcatc ttccccaagc atgccaggag cactttccgg 540
accaccctgc actgcagcct gggccggccc agccgctggt tcaccgccag cgtgcagctg 600
cagctttgcc aggaccctgc ccccagcctc ctgggccctg ccacgctctg acggggctgg 660

ggccggcccg gggtgctgga ggagccggga gccctgggga gaagccggga ggatggacac 720
gatgagctcg gcctggcact cgggcaggag gcgggaaggg aggctgccag accaaggacc 780
cgtgtggaag gaggcggctc cccgctgcct gccctgacct acaggctagg tggtggtctc 840
cttgttttgg tgtcaaggac tcagtaaaag catctatttt tttagcactt aagctggcaa 900
ggcggtaggg gcatgcactg ttaggtggtg gccaccccca gggtcagggg aaaagaatgg 960
gtccatggag tgcccttgaa gcagagaaca gccccgagca gtgtgaggac agaggcatcc 1020
cagcctcagc cggtggggac ggctgccact cccccagagc cctgccaccc catgtgtcct 1080
aggctggtgg ccaaggccat gtgtgaggga agaggggtac ctctcttccc tgctgctggc 1140
ctggggtatt tccaaaacag ccttttcgca cacatgcagg ttgcaccgag aggtctggag 1200
ctgtggctga gccacctctg gcggatgttg agccaggagg ttgggcaaag gccggatgct 1260
gatgccagcg ctggaggtgg tgatactggg ggcggggaag gcctagaaat actttgagcc 1320
atggccttgc cagtgtcccg tgccctccag tgtcaaagat ttggggcact gcccgtcgaa 1380
atggaaaggt tggtgctcag cctctggagc ctcacctgca gggcgtcccc agctaacacc 1440
catccacgca ccacctccag gacgagaacc cttgatgtca aaaccaagtg cccagtggag 1500
gcggtgaagc tctcggaaat gctgccacct gtgtgaggcc gggtctgaac tcgagggagt 1560
cggagctcag ctgtcggttt aaagagacac tgaggggacc gggctgccgc cctcagcctg 1620
cattcctgtg cgcaatcgat tccgcaatga cagcacctta ctccttcctg cggcaggctc 1680
acccctgcct gtgggatgtt gtgagaggaa catgagccag acaaagactt ggctcagggc 1740
tccgtggaac aagccaggat gcacggggag ctgggggagc ccccagcctg ggcagcccca 1800
gcaggccgct gaacaaacac cccagaagcc agcactgtgg cagggtgctg ggagatgcc 1860
cctctgagcc ttcctccccc ctcagacctg aatgcactcc acagttgggg gctgcccctg 1920
ccactcccct ggtaatgcat aaaaggggag gggaaggttc cctggggctt gagctccctc 1980
tgtggaggtg aggaggggag attccgttca catccaggag gggcaaaatg actgatgtat 2040
ttttatgtat ctacacagag agtgcatttt ctctccagag atgctgtctg gttaacaaag 2100
gaataactta agaaattgat tgattatctt aataaactgt gcaaaccc 2148

<210> 29
<211> 2011
<212> DNA
<213> Homo sapiens

<400> 29
agccggcccg agcgcgcccc gccgctgacc gcccgcgccc cgggaaggaa gaacactcgc 60
tcccggccat acttgcgtgt gagttctgac ccctggagga gccactgtgg aagcagagca 120
atcgccatgg agtttgtgat gaagcaggct ctaggagggg ccaccaagga catggggaag 180

atgctggggg gtgacgagga gaaggaccca gacgccgcca agaaggagga ggagcggcag 240
gaggcgctgc gccagggcat ccgagacaag tacggcatca agaagaagga ggagcgcgag 300
gccgaggccc aggccgccat ggaggccaac tccgagggga gcttgacgcg gcccaagaag 360
gccatcccgc cgggctgcgg ggacgaggtg gaggaggagg acgagagcat cctggacacc 420
gtcatcaagt acctgcccgg gccgctgcag gacatgctca agaagtagcc gcgcgcggga 480
cagcggcccc gcggagcccc cgcccctccc cctacagatc ctccgcggag gcccctgagg 540
gacgagcaga gcgcagcccc cacgccgata taagccatag ccccaggccc gccctgcccg 600
cgcccgccgc ctccctcacc cggcaagggg cgcgcgcccc caggaccccc ccagtccgcc 660
ccgtgctcct ctgcccgcgg gtggctggag ccccatcgcc tgggacgccc ctttctcttg 720
ctgtttaccg cccacccagc agcgcccgag gccagggccg ccgaaggcca gccccgcgac 780
cacccctgtg cgcgcgttcg cggcggtggg cgcctgtcgg gcccgcactc aggagcacag 840
ccatagcaga cgggggggtg cgggcggggc gagggcggcg gggcctgggt ccccctcctg 900
cacgcggact ccggggttcg gatcgctccg ccgtgagtat ttgcgtccgc acgcggccgc 960

tctccgcccc ccggcctggc cccctcgcgc ccctgtccat ccgtctctgt ccgcgtcctt 1020
tcctctcctc tctggaaggt tttgcttctt acgaacgcca cggccgtgtt cacttctaaa 1080
ctaaaggaaa caaagcaata ggtttggggg acgcccagcc cccacccccg tcaccccgct 1140
cttcccaagt cctcgccccc cgcccggcct cctagcctct ccgcccacgc ggctgctgct 1200
tctccctggg gaggacccct gccctcggcc attgaacact gcaccctcca caggagccgc 1260
agaggcccga ggcaccggac gctggagacc ctgcgcccct gcccagcacc tcctccgtgg 1320
gcagctcctc gggtggggcc tgcggggttc cctgcgcgca gccggcgcgt gtgtggccta 1380
atccacctgg tggccctgcg gggcggcatc cgagcccctg tttctcctcc attcatgttt 1440
aatttgcatc acaatttgtt gaatctcagg taaatgaggt ctttgcattt aatgagtttt 1500
atcttgacag gcgccgcctc gcccccgggc cctttcgtcc acatcaaaaa tgcatcaagt 1560
ctccacgtgt ttcgggccag ggcgtggctt ggcattgacc ttcatgacct tacatagctt 1620
tagagaagcc ataacgttag actgcaatac taacgaccga cgcccctccg ggcagagacc 1680
accgcgcccc tctgcgcccc agcgacgcgg cccgcgggga cgtcgctgtc cgtcctgctc 1740
gccctgtgcc ctctcactga cttctcccgg gtcgtgtctt ttaaaaactc ctgttttcac 1800
accttacaaa gccagctctg agcagacagg gcgtcctctc gtagaacctg cgcaccccgt 1860
tcccagcgca tggcgccccg ggccgcgagc ttagcttaga ccgtggtgtc ctctgtccgt 1920
ctgtcctgcg cctgcgcctc ctcctgcatg tcggggcccc tgcgtgtgtt ctctccggat 1980
ggaatcacag ccaataaaca ccagtgattt c 2011

<210> 30
<211> 2024

<212> DNA
<213> Homo sapiens

<400> 30
tcgcagcccc atgatggacg ttctggaacc actcccgggc ctcatgttta caaagagacc 60
gagaggccca tcgtccaggg tcacagggat ggcaggtggc agagaggaca caaatgagtc 120
tgcaaccctc aatgtctgaa actcaatagc tggaagacgg ataccccag cccctgcccc 180
tccccgaggt cccacctatc cacctatgtt accgggggag gggagaagtc atagctggaa 240
ctctgggggt ttcccagaac cagccctgct gactgcgaac agcgtcccag catggagagg 300
ctgagaacag tacgtaccct aagacctgga aaagattggg gcacaaatcc acgctcggca 360
cagccatcac acacggccac acccagagag ccacccatgg cctgggaagc agggctgcca 420
cccgcacctc cctgggctgc ccagatgagg gcgggtgaac agaagggcag cccagggcca 480
tgtgtcagga ttcgggccac ctgcccaggc agcccagagc tccacccacc accccccacc 540
ctcaactgca cagcagggtc cctcagcagc cccctgcgtc acacggacca atgacctaca 600
cactgagccg gggctcatgc caactgaaaa ttgggacccg gcccagtctg ccggtttagt 660
tgctgagatg atgtggggag cgggacagag cacgtggatg cagagaggca gagctgccag 720
atgggcatgt ccccaagaca acagcttcct tgggatcaga gcgcccggga cagcacgcca 780
gccaagggaa gcgtgtcagc tccaaaagcg ggcctcgggc gttttgggcg actggctgcc 840
aggcttaggt caggagctgg gtacatgcac agacaaggga cgaaggaaca gcagccagga 900
aggggtccaa atccatacag gaatgtactt tgtgagaaag gtgggatttc acagctgtaa 960
gcaaaagaca ggtgctctcg acaggtgggg ctgagaagaa aactgagcca agatctaaag 1020
aaaaaattac atcagctcca tcccaccatg ttacataata acatgctcca gctggatcaa 1080
agcttcaacc atggaaacaa gcaagcagtc aaggagaagc cagaagaaaa gctctggagg 1140
ccgggcacgg tggctcatgc ctggaatccc ggcactttgg gaggccgagg cggtcggatc 1200
acgagattag gagagcgaga ccatcccggc caacatggtg aaacctcatc tctaaaatac 1260
aaaagttagc ccgggtgtgg tggcgtgcac ctgtagtccc agctacttgg gaggctgagg 1320
caggtgaatt gcttgaaccc gggaagcaga ggttgcagtg agctgagatt gcgccactgc 1380
actccagcct ggtgacagca agactctgtc tcaaaaaaaa aaaaaaaaaa agcactggag 1440
aggccacaaa agaattctct cagcatggaa atggggaagg catttctaag aatgacccca 1500
aacccaaaag ccatgaacta aaactgacac atttaattat acagagatca aaaaatccta 1560
cacagcaaag tgaccataag cagagccaac atagaagcaa ctagagggaa acatgtgcaa 1620
ctcatatcat aggcaaaagc ctaaattgcc taatatgtaa agagtgctgc cgggcacggt 1680
ggctcccgcc tgtaatccca gcactttggg aggccgaggc gggcggatca cgaggtcagg 1740
agatcgagac catcctggct aacatggtga ggccccgtct ctactaaaaa tacaaagaat 1800
cagctgggcg tggtggcggt tcgcctgtgg tcccagctac tcgggaggct gaggcgggag 1860
aatggtgtgg acctcgtagg cggagcttgc agtgagcaga gatggcgcca ctgcactcca 1920

gcctgggcga cagagtgaga ctctgtctca aaaaaaaaaa aaaaaaaaaa aaaagagtgc 1980
ctacaaatca ctaataaaaa gacaaactat ctactaaata atgt 2024

<210> 31
<211> 1725
<212> DNA
<213> Homo sapiens

<400> 31
ttttgtatct tgcagtttgg ctcgcactcc acagatgtct agggtcgccc gcctcttccc 60
ctgcccctct tcctctccgc atggccaagt ccagccaggg ttagcgctga ccccactggg 120
gagggaggag ggctctgcca gcgctcgggc cctcccacac agccgtggac cagacccagc 180
ctggccgggc atgagcgtcg ggcctgggcg gcagcagccg agacccctct gtggcggggg 240
ctcagtgtcg tcatggctcc cctggccacg ggcctgtggt cattgcctgt gacagggatg 300
acagggacag gcctccctgc tctggcacgg cctctggtgt tggcaggatc cagactgcgg 360
tgctgactgc acttcctgtc aggcagggtc tccagggcac ctctgggacc cagcctggct 420
gtccgtccca tagcccaggt gtgccctgtg gcctagggct ttcttggtca cagccaccag 480
accgtgggtc tcatccccac agcagcacca gggcagggac tgagccaccc acacactgag 540
cagagcccca cctgttttcc cagtgcaacg ggaaacctca ccaaccctga acactgaaat 600
ccctgatcct tgtgctggag ctgaatacgc cccttgtctc ctgggtccac tgccaccaag 660
gcccccaggc cctcatggca aagggatgcc cagcataggc ccagccgcac ccctgcaggg 720
cacagagcct ccactgcctt ccaggccgtc ctcgaatgcg ctgcccccctc cagcctgtgc 780
aggcgtacac gggggtgcag cctggggggt acagtcaagg ggagcccttc tcccacagga 840
gggcattggg gtgtggggcc tggggcactg gttcaggtac cgccttatcc cgggccagga 900
atgagctcag tgaccacttg gatgctatgg actccaacct ggataacctg cagaccatgc 960
tgagcagcca cggcttcagc gtggacacca gtgccctgct ggacctgttc agcccctcgg 1020
tgaccgtgcc cgacatgagc ctgcctgacc ttgacagcag cctggccagt gtgcgtaggc 1080
gggcgggggg tgagggggaa cgaggcagag aacagcagcc cggattcagg gaagcagctg 1140
gtgcactaca cagcgcagcc gctgttcctg ctggaccccg gctccgtgga caccgggagc 1200
aacgacctgc cggtgctgtt tgagctggga gagggctcct acttctccga aggggacggc 1260
ttcgccgagg accccaccat ctccctgctg acaggctcgg agcctcccaa agccaaggac 1320
cccactgtct cctagaggcc ccggaggagc tgggccagcc gcccaccccc acccccagtg 1380
cagggctggt cttggggagg cagggcagcc tcgcggcctt gggcactggt gggtcggccg 1440
ccatagcccc agtaggacaa acgggctcgg gtctgggcag cacctctggt caggagggtc 1500
accctggcct gccagtctgc cttcccccaa ccccgtgtcc tgtggtttgg ttggggcttc 1560

acagccacac ctggactgac cctgcaggtt gttcatagtc agaattgtat tttggatttt 1620
tacacaactg tcccgttccc cgctccacag agatacacag atatatacac acagtggatg 1680
gacggacaag acaggcagag atctataaac agacaggctc tatgc 1725

<210> 32
<211> 3083
<212> DNA
<213> Homo sapiens

<400> 32
   aaatccttcg tcatccgagc tgtgatgatg gagtggagcg tggcacgggc ccattggagt 60
   tctgtgggct ccatcggcac tcagcaggga tttggacgtg caggctggtg ggccctgcag 120
   gaagcctgct ccctgctctg ctgcgtggga gggggcagct cgggggaagg gggcttgcag 180
   agaaacagaa gaacatgggc tgtggggcac cttcagcagc ctggggttcc aatcccagct 240
   cctccatgtg ggggccaagc accccaggca gcctttccca gccttgctta gggccgggtt 300
   gggagaatcc caccccgcag ggctgtggtg agggttgagc ggagggtgtg tggtgagggt 360
   tgagcggagg gtgtgtgatg cgtggtgagg gttgagcgga gggtgtgtga tgcgtggtga 420
   gggttgaaca gagggtgtgt gatgtgccca gtggtacacg acaaattgcc ttctttgggt 480
   tgacagactg ggttttactc ttcctgaatc atcacaatga tccgtgcaag gccaaggctg 540
   ttgtcttctg tttcaagtgc gttttcctgt cctgtcctct gtcctgtggc agtggacagc 600
   tgtggctctt gccagattgt gtctgctcct aggactgtgg gagccggtgg tggtagcggc 660
   cttgagcttg acccatccct cctgcttccc tgttcctgag cgagcacctt ggagtatcct 720
   tggagtgtcc ttggaggctc tgctctcggg ggcagcctgg gccaagagag cgcctgatgc 780
   tcaccccgtc ctcacaggtt cacgcctaca tcatcagctc cctcaagaaa gagatgccca 840
   atgtctttgg taaagagagc aaaaagaaag agctggtgaa caacctggga gagatctacc 900
   agaagattga gcgcgagcac cagatctccc ctggggactt cccgagcctc cgcaagatgc 960
   aggaactcct gcagacccag gacttcagca agttccaggc gctgaagccc aagctgctgg 1020
   acacggtgga tgacatgctg gccaacgaca tcgcgcggct gatggtgatg gtgcggcagg 1080
   aggagtccct gatgccttcc caggtggtca agggcggcgc ctttgacggc accatgaacg 1140
   ggccgttcgg gcacggctac ggcgaggggg ccggcgaggg catcgacgac gtggagtggg 1200
   tggtgggcaa ggacaagccc acctacgacg agatcttcta cacgctgtcc cctgtcaacg 1260
   gcaagatcac gggcgccaac gccaagaagg agatggtgaa gtccaagctc cccaacaccg 1320
   tgctagggaa gatctggaag ctggccgacg tggacaagga cgggctgctg gacgacgagg 1380
   agttcgcgct ggccaaccac ctcatcaagg tcaagctgga gggccacgag ctgcccgccg 1440
   acccgccccc gcacctggtg ccgccctcca agcgcagaca tgagtgatgg cgcccggccc 1500
   cgcacctgcc atttgcacgc ccggccggga ggcagagacg gggggagggg aagcctcacc 1560
   atttctcaag gtccataaag actgagcgga tgtttcctcg cctctcgaaa aggaaaacca 1620
   ccatctttct tttaaggctg ttcctgggcc tggcggggga ggcaggggtg agaggatgga 1680
   attgtgtgca caagaactgt ggctatttta atatataatg ttagaggctg cgttctttgt 1740
   cgccgcctcc cctgtgtgcc agccctgtgt gcacggcctc tgccccccgg cctttgctgt 1800
   ggctggagct ggacagtgca gtgactgcga ccgtggggga gccaggtcgc ccttttggca 1860
   gctgctaggc tgaggctgca tggacaggaa caccaggcac cctccgtgtg cttctgagct 1920
   gaggttgctt cacgggaccg tggcttcctt cctcacctgg ctctgcctcc cccgtgctct 1980
   cgggcgaagt gggttcttgt gccttcccct cccgggccca ggctccccgt gcgcgggccc 2040
   tgccctttcc tcccgcgccc caccggctcc gacgcgcaac cccgctcagc agtcacagaa 2100
   gcagggccca gccaccttgg tctttttttg ggagttcagg ggagtaggag aatgtcttcc 2160
   agaaaaatac ataagctagt ttctgttctg taaagtgata cctttcatac ttgaccaaag 2220
   ttcccaataa cttcccaacc actgttcaaa agctgtgatt tttgtctccc cttcccaccc 2280
   tccagccaag gagcagccct gcccaggggg cattaggtgt gggtacccgg ggagcacccc 2340
   gttcctggac cccagtgttg catttcctgg ctgaggaagg gtggtcatcc cagctcctgc 2400
   cctaccctct cacttaactg gagctttggg acgcaccctc cacagtggga ggtggtggtg 2460
   ggtggcggtg gcggggcctc acgacagctt ggtgctggta agaggaagcc cgtggttctg 2520
   gctaggctct catgtccaga cagcggggac caggggaaaa cccagcccct tctgtaatcc 2580
   cccttcattt cctaccttcc ttcctcctct gtttagcaaa ggagggcagc tcacttggat 2640
   gtccttacaa cgcccctggc cccaggttga gcaataagaa accagaacct cgcggcccag 2700
   tggcccgggc cagttcaggc cgcctccccc tcctctgcct ggggccattg agcccagcct 2760
   ccagggcccg ggtgcgtttg caggccagtg gccactgtcc gggctgtgat ggcaccaagg 2820
   caggtggagc accaggtacc acacagctgg gcttcccacc aggctttccc gcgggggtct 2880
   cagggagctt ctccccagcg ctgctcggag tctgcaggaa ctggccttgt tctccttagc 2940
   ccgtcactcc atacagtatt aggtgaggat ggatgcgggc gctgtccttg ccgggaagtc 3000
   actgttgaag ttgcagtggc ttgttcacac ctgtgggaag agaagtgaag actttctcct 3060
   tgcattaaaa agtctgaact gtg 3083

<210> 33
<211> 1831
<212> DNA
<213> Homo sapiens

<400> 33
acccatcttc tgttatgttc cttcctgttc ctgagaccca gggtctcctc tttcttagtt 60

cacacctttg ttttggtgga gctttgtggg tgttctggtg tggaagataa aattctgaca 120
tgtcccatat ctgaaacaca ttgtctagcc ctcaacatta gattgaagtt tggctggaag 180
aattcttttt attagtacag tcacagacct tttttatca ctattgttgg tgagcttttt 240
cagtttgaaa ttcacatctc tcagttctgg gaaactgcct tgaaattttt ttttatttcc 300
ttcccttcta ttgctctgct ccctgtttct gggatgcctc ttacttggct attggcatct 360
gggatgggtc ctttaatgtt cttatctttt cactccaatt ttccattcct ttgtctttct 420

actctatttt cctggaaatt ttctcacttg tatctttcag accttctgtt aagattttca 480
tttctggtag cttttttttt ggtgggggga gggggtctca ctatttcaca cagactagag 540
cacagtggtg ccatcatagc ttactgtaac cttgaactcc tgggctcaag tgatcctctc 600
gcctcagcct cccaagtatc tgggactgca ggcatgtgtc actgtacctg actcattttt 660
aattttttta ttttttgtg gaaacaggtt ctcactatgt tgccagggct ggtcttaaat 720
tcctggcttc aagtgatgct cccgcctcag cctcccagag tgctgggatt acaggcatga 780
gctgcttcat ctggtctctt gtagcatatt tttactttcc aagagtgttt ttattctctg 840
aatgttcatc tttttagctg cctcctgttg ttctttcatt tacaatgtct ttccttattt 900
ctctgagaac agcagtaact ttttaaaaaa aaacagtgtg ttctctattc tctcctagtt 960
gttggcttat atgcaagaga ggggcaccac aaatctggct gacagctctg agcaggtaga 1020
tgtggggctt actggctgga agggacatag gggaacccca ctccccaagg agaccctttt 1080
cttagggtat gtgagcaagc agcttgagta tgagtggatg ctgcaaacag atccataggg 1140
caatgtggct aggccatttt gggggcactc ttcctccaag catgtttcgt tccatcgtct 1200
gaatcctgca agaaggtaac ttcagcctcc tgccagcatc cttgggaaag agattaacat 1260
ctcagcatcc aatgggactg tttacctaat aaccctgact ttctcagtac ggtttctcac 1320
ccttaacata gccttctgcc aggatgggag aagaaatcca gaagtaagtc atggcagtgc 1380
atactgttgg ctcagaattc tgtgcagaca gagcccacag cagcagccct gcccagcatc 1440
tgcttctgaa acagcctctc cagcctggct ggtgccctgc tcattcactt gcatggccag 1500
aagtaccagg tacctccaac tcctgagttt ttagaatctt cttagccagg cgcagccgct 1560
catgcctgta atcccggcac tttgggaggc ggaggcgggt gggttgtttg agtccaggag 1620
ttcgagacca tcctgggcaa catggcagaa ccctgtctct actaaaaaat aatacagaaa 1680
actagctggg catggtggca cgtacttgtg gtcccagctg cttgggaggc tgaggtggga 1740
gaatcaccca agccagggaa gtcaaggctg cagtgagctg agatggtgcc agtgcacacc 1800
agtctgggca atgggggtga gacccagtct c 1831

<210> 34
<211> 2742
<212> DNA

<213> Homo sapiens

<400> 34
aaagaatttg cagatgacgg cgtcaagtac ctggaactaa ggagcacacc cagaagagaa 60
aatgctaccg gaatgactaa agagacttat gtggaatcta tacttgaagg tataaaacag 120
tccaaacaag aaaacttgga cactgatgtt aggtatttga tagcagttga cagaagaggt 180
ggccctttag tagccaagga gactgtaaaa cttgccgagg agttcttcct ttctactgag 240
ggtacagttc ttggccttga cctcagtgga gaccctactg taggacaagc aaaagacttc 300
ttggaacctc ttttagaagc taagaaagca ggtctgaagt tagcattgca tctttcagag 360
attccaaacc aaaaaaaaga aacacaaata ctcctggatc tgcttcctga cagaatcggg 420
catggaacat ttctcaactc cggtgaggga ggatccctgg atctggtgga ctttgtgagg 480
caacatcgga taccactgga actctgtttg acctcaaacg tcaaaagtca gacagttcca 540
tcttatgacc agcaccattt cggattctgg tacagcattg cccatccttc tgtgatctgt 600
actgatgata agggtgtttt tgcaacacac ctttctcaag agtaccagct ggcagctgaa 660
acatttaatt tgacccagtc tcaggtgtgg gatctgtctt atgaatccat caactacatc 720
tttgcttctg acagcaccag atctgaactg aggaagaaat ggaatcacct gaagcccaga 780
gtgttacata tttaagctat aatgaggtga actacttctg agtatgtgtt tcaatcaagt 840
tcctgccata tcccacttag taaaacagtc caccactcct ttgaagcata gcaaccaagt 900
tccttgggct ctatcaccag caccttacac atggcaggta ctcagtaaac acgtgtcttc 960
aactgactca caagctctca ggtgcttact gggtgggact tgactgttgt tgctaattaa 1020
atccccattc caccagtgat tattgtgact cagcagtcct tccctattag tgatcataaa 1080
atttcaggga aatcgaagtt tctcatcagg aaatgttttg gaattactag tataaagtta 1140
ggaaagtggg gaaattaggt tactgccgag acctttaagc cttctaaaca gctttatatt 1200
ttattgtgca tactttaatc agactccctt cactcgcttt aagtttttaa aagtattccc 1260
cagccggatg tgatggctca tgcctgtaat cccagcactt tgggaagcca aagtgggcag 1320
attgcttgat cctaggagtt cagtagcagc ctaggcaaca tggagaaacc ctgtctctac 1380
aaaaacaaaa aaacaaaaaa ccggaaatta gccaggcacg gtggtacaca cctgtagtct 1440
cagccaccag ggaggctaag gtgggaggag acctgatccc agggatgttt gaggctgcag 1500
tgagctgtga tgctacagca ctccagcctg ggcaacagag tgaaaccctg tttcaagaaa 1560
aagaaaagta ttctccaaga tcccaagacc attgacacca ggccacattg tataccaggg 1620
atttatgtga atgcctaggg atgaccgtag tgacagataa agctaggtgc tgagatctgg 1680
ttttgccctc tttatgattg tcccatcggt actagaaagt gaagtcattg ctccaatttt 1740
ggaagaggag agaataactg gctgaggggt gagaaagaca gaaatagctg cagctctctt 1800
tttttttttt ttcaacaaag gctctcactc tgttccccag gctggagtgc agtgacatga 1860
tcacagatca ctgcagcttt cagttttaaa acagcttcta ttacattttc tttgtggaag 1920
ctgtatttct accttagtac tcactttctg cttggtctga aagatacctc tagtagtggc 1980

aacattgtgt taagtagagt tcattgggtc tcttgtaacc ctacccctac ataatggacc 2040
actggtgcag gcttacataa acacacatac tgtggcgact tcagctgaag acaagggaca 2100
aactaagata gttctgaaag gtaaaaaaac aaacaaacaa aaaacaaaaa caaaattatt 2160
agataccaag ttatgcttga attgctttgc tctttagaac gtgtgttttt ccctggtatc 2220
agaagaagtt aaggctgatg acacccctaa tttaaattac taaggatttc tgataggttt 2280
tctgtctttc tgtgatatcg taaagtattt atttggtctt tgaccctgtt tcctggcata 2340
ctgctcctaa aatccttaga gtctccagag tgatggcttt ttgtatctaa tgagttgact 2400
gctggctggc agcccctagg ggctggtcac tggaaagaca aatgcatgat tagagttggg 2460
actttcagtc ctcccccaaa ctcttgggag aagggctgaa ggttaagttg atcaccagtg 2520
accagtggta atcatacctt cataatgaag cctccataaa actccagaag aacagatttt 2580
ggagcatttc caggtaactg aacatatgga ggtccctaga gggtgctgta cctgaagagg 2640
gtagagggta taggagctcc ctgtcccttc ccacatgcct tgctctatcc atttcttcat 2700
ctctatcctt tgtactgtcc tttataataa accagtgaac ct 2742

<210> 35
<211> 1463
<212> DNA
<213> Homo sapiens

<400> 35
acttttgggt cgggccctcc gggaagatgg cggccgtgca ggcggccgag gtgaaagtgg 60
atggcagcga gccgaaactg agcaagaatg agctgaagag acgcctgaaa gctgagaaga 120
aagtagcaga gaaggaggcc aaacagaaag agctcagtga gaaacagcta agccaagcca 180
ctgctgctgc caccaaccac accactgata atggtgtggg tcctgaggaa gagagcgtgg 240
acccaaatca atactacaaa atccgcagtc aagcaattca tcttcacttt ggcctcaaag 300
acaaggaaac aaggtatcgc cagagatact tggacttgat cctgaatgac tttgtgaggc 360
agaaatttat catccgctct aagatcatca catatataag aagtttctta gatgagctgg 420
gattcctaga gattgaaact cccatgatga acatcatccc agagggagcc gtggccaagc 480
ctttcatcac ttatcacaac gagctggaca tgaacttata tatgagaatt gctccagaac 540
tctatcataa gatgcttgtg gttggtggca tcgaccgggt ttatgaaatt ggacgccagt 600
tccggaatga ggggattgat ttgacgcaca atcctgagtt caccacctgt gagttctaca 660
tggcctatgc agactatcac gatctcatgg aaatcacgga gaagatggtt tcagggatgg 720
tgaagctgcc agaaacgaac ctctttgaaa ctgaagaaac tcgcaaaatt cttgatgata 780
tctgtgtggc aaaagctgtt gaatgccctc cacctcggac cacagccagg ctccttgaca 840
agcttgttgg ggagttcctg gaagtgactt gcatcaatcc tacattcatc tgtgatcacc 900

cacagataat gagccctttg gctaaatggc accgctctaa agagggtctg actgagcgct 960
ttgagctgtt tgtcatgaag aaagagatat gcaatgcgta tactgagctg aatgatccca 1020
tgcggcagcg gcagcttttt gaagaacagg ccaaggccaa ggctgcaggt gatgatgagg 1080
ccatgttcat agatgaaaac ttctgtactg ccctggaata tgggctgccc cccacagctg 1140
gctggggcat gggcattgat cgagtcgcca tgtttctcac ggactccaac aacatcaagg 1200
aagtacttct gtttcctgcc atgaaacccg aagacaagaa ggagaatgta gcaaccactg 1260
atacactgga aagcacaaca gttggcactt ctgtctagaa aataataatt gcaagttgta 1320
taactcaggc gtctttgcat ttctgcgaaa gatcaaggtc tgcaaggaaa ttcttgtgtg 1380
ctgctttcca tttgacaccg cagttctgtt cagccatcag aagagagaca aggaattaaa 1440
aatttctttt taatcctgtt acc 1463

<210> 36
<211> 1969
<212> DNA
<213> Homo sapiens

<400> 36
ttgacctctt cctctccaga agttatcaac tcagtctttg gaggatcttt tccacgggca 60
catagctcct cattcagtaa tggaaatcag tggggaaata ggcttcaagt tctaaaactt 120
cattttccac tcagctttgc tgtaacactc cacgaataac ataggtccat gtgagtgcac 180
gggcaagacc agcctgggaa ggctttatgg gggaaaatag ctatcagcaa attttcaaag 240
gtttttttcc cctttcttga tttatcttat tatgagactc atataagttc atcatataaa 300
attcaacata tagaaatatt atgcagaaag tggaagtccc ccataatccc accccccaga 360
gataactacc ataacaattt tatgtatact acagtttttc taaattttat ttttgctatg 420
catttactaa ctacccattt actacaaaaa tgggatcata tggttcatag tgtttttgca 480
gtgcattttt cacgtatcaa tatattgtgg ccatctttcc atgtcagcac acatagatct 540
acctcattct tgttaatggc tgtatagtat tccattgtat ggaggtgccc taatttattt 600
aaccagttcc ccattggtgg acatttgggt tgttactaga agcttatcgt tctatatcct 660
gttccttttc tgcctttcct ccagcactga ctcatatatt gaagtccttg atggttcccg 720
tgtccaccct gagacttatg agtgggctag gaagatggca gtggatgccc tggaatacga 780
tgaatcagcc gaggatgcca atcctgcagg agcccttgaa gaaatcttgg aaaacccaga 840
gcgactgaaa gacctggacc ttgatgcctt tgcagaagag ctggagaggc agggctatgg 900
tgacaaacac atcacactct atgacatccg ggcagagctg agctgtcgat ataaggacct 960
ccggacagcc taccgctctc ccaacacaga ggagatcttc aatatgttaa ccaaagaaac 1020
accagagacc ttctacattg gaaagctcat catctgcaat gtcactggca ttgcccacag 1080

gcgtccccag ggtgagagct atgaccaggc gatccgcaat gatgagacag ggctgtggca 1140
gtgccccttc tgtcagcagg acaatttccc tgaactaagc gaggtgtgtg ctgcagcatt 1200
atcctgctca gtggatttcc ttggttgaat gatctcggtg cctagaaggg aatcagaaat 1260
gagggtgttc tagatcattc tctcaccaaa caagtacaca aagaaggaag acggacggga 1320
aacacacagg tttagcatga agtcccttct gagaaggctg acatagctgg gcacggtggc 1380
tcacgcctat aatcctagca ctttgggggc caaggtgggc ggttcatctg agatcagggg 1440
ttcgagacca gcctggccaa gatggtgaac cccgtctct actaaaaata caaaaattag 1500
ccagttgtgg tggcatacgc ctgtaatcct agctactcga gaggctgagg caggagaata 1560
acttgaaccg aggaggcgga gtttgcagtg agccgagatc ataccactgc actgcagcct 1620
gggtgacaga atgagacttc atctcagaaa aggaaaaaaa aaggctgact tagaaacttc 1680
ttatggtcgg ccgggcgcag tagctcacgc ctgtaatccc agcactttgg gaggccgagg 1740
cgggcggatc atgaggtcag gagatcgaga ccatcctggc taacacggtg aaaccccgtc 1800
tctactaaaa atacaaaaaa ttagccaggc acggtggcgg ccacctgtag tcccagccac 1860
tcaggaggct gagacaggag aatggcgtga acccaggaga tggagcttgc agtgagccaa 1920
gatagcagca ttgcactcca gcctgggcga aagagcgaga ctccgtctc 1969

<210> 37
<211> 2735
<212> DNA
<213> Homo sapiens

<400> 37
   ccccctggcg caacactcag ctggctgcga ccgcaacccc gagcctggac actgcgccag 60
   gaatcctaaa accaaaatat tagaacgaaa acagaaacat ggctcactat attacatttc 120
   tctgcatggt tttggtgctg cttcttcaga attctgtgtt agctgaagat ggggaagtaa 180
   gatcaagttg tcgtactgct ccgacagatt tagttttcat cttagatggc tcttatagtg 240
   ttggcccaga aaactttgaa atagtgaaaa agtggcttgt caatatcaca aaaaactttg 300
   acatagggcc gaagtttatt caagttggag tggttcaata tagtgactac cctgtgctgg 360
   agattcctct cggaagctat gattcaggag aacatttgac ggcagcagtg gaatccatac 420
   tctacttagg aggaaacaca aagacaggga aggccatcca gtttgcgctc gattaccttt 480
   ttgccaagtc ctcacgattt ctgactaaga tagcagtggt acttacggat ggcaagtccc 540
   aagatgacgt caaggatgca gctcaagcag caagagatag taagataaca ttatttgcta 600
   ttggtgttgg ttcagaaaca gaagatgccg aacttagagc tattgccaac aagccttcgt 660
   ctacttatgt gttttatgtg gaaggctata ttgcaatatc caaaataagg gaagtgatga 720
   agcagaaact ttgtgaagaa tctgtctgtc caacacgaat tccagtggca gctcgtgatg 780
   aaaggggatt tgatattctt ttaggtttag atgtaaataa aaaggttaag aaaagaatac 840
   agctttcacc aaaaaaagat aaaaggatat gaagtaacat caaaagttga tttatcagaa 900
   ctcacaagca atgttttccc agaaggtctt cctccatcat atgtatttgt gtctactcaa 960
   agatttaaag tcaagaaaat ttgggattta tggagaatat taactattga tggaaggcca 1020
   caaatagcag ttaccttaaa tggtgtggac aaaatcttat tatttacaac aaccagcgta 1080
   attaatggct cacaagtggt tacctttgct aaccctcaag ttaagacgtt gtttgatgaa 1140
   ggctggcacc aaattcgtct cttagtaaca gaacaagatg tgactttgta tattgatgac 1200
   caacaaattg aaaacaagcc cttacatcca gttttaggga tcttgatcaa tgggcaaacc 1260
   caaattggaa aatattctgg aaaagaagaa actgttcagt ttgatgtcca aaagttgcga 1320
   atctactgtg acccagaaca gaacaaccgg gagacagcat gtgagattcc tggatttaat 1380
   ggagagtgcc ttaatggtcc cagtgatgta ggttcaactc cagctccctg tatttgtcct 1440
   ccgggaaaac caggacttca aggccccaaa ggtgaccctg gactgcctgg gaaccctggc 1500
   taccctggac aacctggtca agatgggata tcaggaattg gcagggacac caggtgttcc 1560
   aggatctcca ggaatacaag gagctcgagg actaccaggt tacaaaggag aaccagggcg 1620
   agatggtgac aagggtgatc gtggacttcc tggttttcct gggcttcatg gcatgccagg 1680
   atcaaagggt gaaatgggtg ccaaaggaga caaaggatca cctggatttt atggcaaaaa 1740
   gggtgcaaaa ggtgaaaagg ggaatgctgg cttccctggc ctccctggac ctgctggaga 1800
   accaggaaga catggaaagg atggattaat gggtagtccc ggtttcaagg aatgttccct 1860
   gtgcatatga gaagggagaa gcaggatccc ctggtgctcc ggggcaggat ggaacacggg 1920
   gagagcctgg aatcccagga tttcctggaa accgaggatt aatgggccaa aagggagaaa 1980
   ttgggcctcc aggacagcaa ggaaaaaaaa ggagccccag ggatgcctgg tttaatggga 2040
   agcaatggct caccaggcca gcctggaaca ccgggatcta agggaagcaa aggtgaacct 2100
   ggaattcaag ggatgcctgg ggcttctggg ctcaagggag aaccaggagc aacgggttcc 2160
   ccaggagaac caggatacat gggtttaccc gggattcaag gaaaaaaggg ggacaaagga 2220
   aatcaaggtg aaaaaggtat tcagggtcaa aagggagaaa atggaagaca gggaattcca 2280
   gggcaacagg gaattcaagg ccatcatggt gcaaaaggag agagaggtga aaagggagaa 2340
   cctggtgtcc gaggtgccat tggatcaaaa ggagaatctg ggtggatggg cttgatgggg 2400
   cccgcaggtc ctaaggggca acctggggat ccaggtcctc agggaccccc aggtttggat 2460
   gggaagcccg gaagagagtt ttcagaacaa tttattcgac aagtttgcac agatgtaata 2520
   agagcccagc taccagtctt acttcagagt ggaagaatta gaaattgtga tcattgcctg 2580
   tcccaacatg gctccccggg tattcctggg ccacctggtc cgataggccc agagggtccc 2640
   agaggattac ctggtttgcc aggaagagat ggtgttcctg gattagtggg tgtccctgga 2700
   cgtccaggtg tcagaggatt aaaaggccta ccagg 2735

<210> 38
<211> 2493
<212> DNA
<213> Homo sapiens

<400> 38
cttggggtac atggaactga ttccagcagt gggaccagat tcacctgggc catcgcatgc 60
ctgattcatg ggcagcctca ctgagagcag tggcagatag agtcacctgc tccattgcag 120
tggctttttc ctctttgctc tttttttca atattcctgg ttgaatttgt gtagtggaat 180
gatcatatga tgcaactacc gtaatagaat ttatttgaca agctagtgca attagttatt 240
aatgaatgta aacttcacag gattagaagg gctatgtgtt tcactatttt tgtatctttg 300
ggttctacaa aatatactag agtgtcacat aattcgagaa gggatttcag gtacgtggat 360
gttaactact aatagtttga aaatgatact atctgggggt ttcctttttt ttccacttct 420
ctccaacata ttaagaaaac tattcggata ttattttgcc ttatgcagta gattgctagt 480
ggttgtgaat tgtgttttaa taagaacctg tttatgactt caggattttt tattggtgga 540
ccttctaata tgattagttc tgctatttct gcggacttgg gtcgccagga gctcatccaa 600
aggagcagtg aagctttggc cactgtcaca ggaattgtgg atggttcggg gagcattgga 660
gctgcagtgg gccagtattt agtgtctctg atccgggaca agctaggatg gatgtgggtt 720
ttctactttt tcattctcat gacaagttgt acaattgtgt ttatctcgcc attaatagtg 780
agggaaatat tctctctcgt gctaaggaga caggctcaca tattgaggga gtgaccggtg 840
cccgcgagac agaaagaaca atgtcagcca catcaggacc gctggggctt cgagtctgcc 900
ctaatttggg tttgtctagg agctccagcc tgatcttgga ttgtcaggcg tctctcaaca 960
ctgccagcca cctgagatgc tgaccagcag tgaaggctgc actagttttc tacactacag 1020
gaattactgt tgatgatttt tgctgttgtt tcatgaatgt gccgagtgac ttctgactct 1080
gccagcatct ttgacctctg actctgcccg cgtctttgtt tggcctattg aggtttagcg 1140
ttttaacctt aactgttgtt ttggacttga actttctcta gccttgaaaa tggagtgaac 1200
aggaggcaaa gccacgcgtg gtttgtgttg gtggtatgtt ccacaaatgg aagagatcat 1260
tgcattttgc tttggtaatg agtttacaga gagtgggtca tctttgaagc atcaaatcct 1320
gatcaaatgc tcaagctgga ccgagctgca agcctgcctt ggacaaagaa tccctctgtt 1380
cactgtggtg ggaaagatca cactacatgc ctgttgattg gctcagtcac tctgtgtctg 1440
atctaaacgt cattcagccc tagaagcatg aattgcttca aattattgtc aacttgttct 1500
cttccatttt cattcaaatt aactttgact cctggaagta ttgagtcttc ctttcaagga 1560
ccataaggta catcagttat cttgaacatt ctgacgtgta aaggaccata aggtacatca 1620
tttatcttga acattccgac gtgtaactca cagccgaagc actccatccc agatttgttg 1680
tctgggaact ttaggatcct ctaggaagct aatctgctta gtctattttt agaggattga 1740
tctctggcac aaccagcttc ctggagttac ctcagcagaa gactagaatt agagaaaagg 1800

gaaagacctt ttcttctagt aagtgtcaag tacagatgct ctttgactta tgatggggtt 1860
atgtcccagt aaacccattg gaagtcaaat gcatttaata cccctgactg gacaccacag 1920
cttagcctcg tccaccctaa atgtactcag aacacttaga attgcccaca gttgggcaga 1980
atcatctaac acaaactcta ttttataatc gagtgttgaa tatatcatgt aatttattga 2040
atattgtaca ttatgttgaa attgcaacca tttcacacca ttgtaaagtc caaaaatcag 2100
aagctgagga ctgtctgtac atgacattga atcacatgaa tggattacta gggcctgagg 2160
ggtgtccgtt ctttagaaaa atgtagagta tatatatctt cagggctgtg aattccatac 2220
tagttatcaa tattttaaga tcattctgta tatacatatt ttaaattatg caagcaaact 2280
aggaggagaa tgtgcaattt tgagggatac ctaatttgca ttcggttagg ggatattttt 2340
caacctcttg ctttatactc tgatatgggg tgtgtttatg aacttgttaa tgttatttaa 2400
tcatgcagat ttccagtcag tgtattgctc atttactgac aaaaagggaa aatacgaaac 2460
acagaaaggg aaaataaact gacttttata atg 2493

<210> 39
<211> 2015
<212> DNA
<213> Homo sapiens

<400> 39
cgagacaaaa caatccatat gtttagggaa ccagaaaagt cccctggtct gtcccttctt 60
tggggagcag ggcctcgaca gctccagctc ccttgaccta ccttcctccc cgcaccccgc 120
ccccaccttg tgcccctgtg tccagccccc caggggggcct gtgtctgtgt ctgtgcctgt 180
gtctgtgatg gggagccgcc tcgcacccct gttgtctgct tgtctctttg tgtctgttat 240
cctgggcagg atggtcattc tcaaaaaccc tggggtcctg ggccagagac aggcagggcc 300
cagtccaggg gccccaggcc tccccagtcc cagtgtgcga gccccacttg gacacaagtg 360
ttcagagagg tccccctctg ccacttgaca gggaccttca aacctcgaca gtgatgcaag 420
gacacagaga gtaccagata ggtagcagag accaaggcgc agggtgcttc agatgagcaa 480
gagaacccag tcgaaccaga taccccaggt gggccggagg gaccccagac cttcagaggg 540
ctgccctggt gttctccaca gtgcagtccc tctgtattcc cagggtggga tcggggcttt 600
cagccccacc ctgatgcctg ccctccagga tggctggttt agtctgggtc catgtcccag 660
acccctctat tctgctccag gacagcagga cttcaggtct tcctgggggt ggatatagga 720
gaaaatttct gcctggcaca cacctggctc caaccactgc caagtgatca ctcttaggcc 780
caggggaaca caatgactat cattactgat gcagacctgg ctgtggagag cagctaatgt 840
gtggcccaga gagcctgtct gtgtggagca cgtagtgcac agaatacgtg agagttgctc 900
tggcaggggc aggatcctca caggatcgcc tgggaggtga ggtgtgtgtg acccactgga 960

tgggagggca atgagtgtgc acatacaaat ggggcagtgt gcatgcaaca cacttagggg 1020
aggagtggcc ccagaattca gcacgcacac aacacacaag ggagagaacc cccagatgag 1080
aaaataggaa ggagcaatca tttgtagatg ggtgaaaaaa gaatgaggtt caagggagcg 1140
tgcaccaggt gaggtgagcg tgtgtgctct cagggaaggg cccaggctcc catgcctggg 1200
aggagctgcc agagagaagc aaaaaggcgg ctgtggatcg ccctgggctg ggcaccagtg 1260
acaggtcagg atctccaaac atggacgtcc tcccctccaa atccagaagc tcccagaagg 1320
tgtccttaac tgcaaagctg tgcagggtac tcctccagat ggaatcagga agtcgagaca 1380
ccatcccagg tgtgtgtaag agagagagag agaacatgga ggatacagaa gtattgcagc 1440
ccagatcccc tatcaggggg acagctggtg ggcaaagcag ccaccccaca gccttgtggc 1500
tagagtacag tggggtggac cctccagccc caatagccct agtacccagc tggcagggtt 1560
gcccacccct gctgtccacc tgctccatcc tctagggttc cacaggcccc tgaccgcaca 1620
gggaggctgg ggccagcctg gtctcccagg cctgaggaca tgcctcccac caaatgtccc 1680
ctgctccagt cccactcctg tcactccacg ctctgcactg ggagaaaaac gggaggtgct 1740
cgtgctggcc ctgggtggga gcggggagtc ctggtgagac cccggtgaga tggaccatcc 1800
tgcccgcgtg ggggatcccc tttcccacat ccgtgctgtg tcattgttgc tctgcttcct 1860
ttcaatgtgt cagtgcctgg ggggagggga ggagcacccc ctcagccccc ctgaacctga 1920
ccaaaagcca tggctgttgc tcccccccttt gtatgatgca aatgctgaaa tgtacaaaat 1980
caaccatgac aacaaagaaa aagaccttgt acagc 2015

<210> 40
<211> 1404
<212> DNA
<213> Homo sapiens

<400> 40
ctgctcagct tggtctgtgg tggtggtggt ggtggtggtg tgggtttggg gtgcggccgg 60
gtaggggggtt cgcctgcggc cgcgtctgct cggggcctga ggcctcgaag accccagccc 120
aagcccccaa gttgatgccg gcccaggatg gatcagacct gtgaactacc cagaagaaat 180
tgtctgctgc ccttttccaa tccagtgaat ttagatgccc ctgaagacaa ggacagccct 240
ttcggtaatg gtcaatccaa tttttctgag ccacttaatg ggtgtactat gcagttatcg 300
actgtcagtg gaacatccca aaatgcttat ggacaagatt ctccatcttg ttacattcca 360
ctgcggagac tacaggattt ggcctccatg atcaatgtag agtatttaaa tgggtctgct 420
gatggatcag aatcctttca agaccctgaa aaaagtgatt caagagctca gacgccaatt 480
gtttgcactt ccttgagtcc tggtggtcct acagcacttg ctatgaaaca ggaaccctct 540
tgtaataact cccctgaact ccaggtaaaa gtaacaaaga ctatcaagaa tggccttctg 600

cactttgaga attttacttg tgtggacgat gcagatgtag attctgaaat ggacccagaa 660
cagccagtca cagaggatga gagtatagag gagatctttg aggaaactca gaccaatgcc 720
acctgcaatt atgagactaa atcagagaat ggtgtaaaag tggccatggg aagtgaacaa 780
gacagcacac cagagagtag acacggtgca gtcaaatcgc cattcttgcc attagctcct 840
cagactgaaa cacagaaaaa taagcaaaga aatgaagtgg acggcagcaa tgaaaaagca 900
gcccttctcc cagccccctt ttcactagga gacacaaaca ttacaataga agagcaatta 960
aactcaataa atttatcttt tcaggatgat ccagattcca gtaccagtac attaggaaac 1020
atgctagaat tacctggaac ttcatcatca tctacttcac aggaattgcc attttgtcaa 1080
cctaagaaaa agtctacgcc actgaagtat gaagttggag atctcatctg ggcaaaattc 1140
aagagacgcc catggtggcc ctgcaggatt tgttctgatc cgttgattaa cacacattca 1200
aaaatgaaag tttccaaccg gaggccctat cggcagtact acgtggaggc ttttggagat 1260
ccttctgaga gagcctgggt ggctggaaaa gcaatcgtca tgtttgaagg cagacatcaa 1320
ttcgaagagc tacctgtcct taggagagag gaaagaaaga aagaaagaaa aagaaagagg 1380
aaagagaaag aaagaaagaa agag 1404

<210> 41
<211> 2052
<212> DNA
<213> Homo sapiens

<400> 41
tttcatagat aagaagactg aggttcagag agaagaggtc acttgcacag gtctaccctg 60
ctcacaaaag cagagggaac agcatgtgca aaggcccagg gtagggaggc agcctagagt 120
ttccaaagcc cagatcagag tttgactctc tctggattaa accatttgtg gctccctagt 180
gccatctgga caaagtccta actccttagt ttagcatcca tctgtccatc catcccctga 240
gcccctctgt gcccagccct gtgctgggta atgttgggga cacaacagtg accatgacag 300
cctcagccct gccctcatgg ggcacaaagt ctagtgaagg ggacagaccc atccccagat 360

ggtgatgccc cagaacgggc agggctggga tggtgaagcc aaggaactgg aggagctcac 420
agggggcggg tgttggacct gggatggggg gaggtgttta gagagggctt cctggaggag 480
ggggcatctg acccagacta gaggattcaa ggagggctgc ctggaggaga ggactttgga 540
ggtgagacca gcagaatggg aggagtgaag caggagagga gcatctgcag agggaatagc 600
acgtgccaag gccaggagag cttggaaatc tcgtggctgt gagacagccc cagcccccta 660
cacacaagga ggaagttctc tttggactgg gttgaggcag gcggccggac aggtggctaa 720
tgaattcagg gccagcagaa aagccctggg gatgagagtg gaggcctgca cagggcttat 780

gcatggaggc gggtgctgga ggccgagcct gggagagaga tgggcaggcg tatccaggcc 840
aggtagggca gtgggcgact cagctctttc atggccaagt gtgggtaggg atggtctggg 900
gtgagtgtca ggggaccttt ggggtcagta gtgatggagg ggtcttctca tcccactgcc 960
tggctgccca gtgggctctt ttctctgctc ctttctgtct ccttttctgt ctctccactc 1020
ctgatcatgg ctcactgtag cctcgacctc cccaggttaa gcgatcctct ggcctcagcc 1080
tcccaagtgg ctgagactac aggcgtgcgc caccatacct ggctaatttt tatttttatt 1140
tttgtagaga cgggtctcgc tttgttcccc aggctggtct cgaactcctg ggctcaagcg 1200
atcccccctgc ctctgcctcc caaagtgttg ggttacagg catgagtcac cgcgcccagc 1260
ctcctgtctc tctttctctc tctccatttc gtctatttct tcatgacttc ttctctctct 1320
tcccatgtcg ttctctctga ctccctccag gtctgtctct gtcctactct gcctgtctct 1380
ctccttgttc tctgtctctg tctgtaggtc tctccctccc tcacctccac tctgattctc 1440
tgtctccctc tttctcgtca ctgcgtctct tctctctgtg accctcccac atccctgtgc 1500
cattccccgc cagccactct ctctgtgtcc ccctcacatt ggggctgttg aggcctgcac 1560
agatgggctg gggtggggcc aggccccctg cccgctcctg acttggcctc tcctcccctc 1620
tccacagacc cagcctccgg ctgtgacccc cagcctcaca cctgcctctg gttcccgcct 1680
ggtcctccag cttcaggacc ccacctccaa aggcccctct gctcaatgcc tacctcccta 1740
gggccctgct gggacatggg ggcctgagtg cccatccaag ggctctcaag gacaccggca 1800
aggcctccag gccctgagcc ccacttctgc cttcacctct gcctgggacc cgagctgggc 1860
tcctgggcct tggtccccag aagatggcgg ctagggcctc gccgccagga cagagaaggg 1920
acggggtggc tgggcagtca gggaaggagg gtcgcccgga tccgacattt tggagagatt 1980
ccttcactct cctgtccccc ctacctccct tctctaattt cttctttttt ttaatgataa 2040
agtcttaaaa ac 2052

<210> 42
<211> 3317
<212> DNA
<213> Homo sapiens

<400> 42
atttattccg catctttcta tcactgtttt ataaaatggt gccagtgtcc cgcgaaatgg 60
caggtgccaa caccttccag actgttcagt gtactagctg tgctcctgta gggtgggaat 120
ccagcgacca ggtggtgccg gagacccggg ctcaggagcc gctgattgcg cagaagcgca 180
ggttgtgcgc aaacctctct gtccgagcct caacagggct cgggaattgg ggctttgttt 240
gttctaacca gccccaaagc gcctgttttt cctggatgcg gcgggagaag caactacaaa 300
ggcaacagtt ctccagctgc gcgtcttcct cctcagctag acgctaggta gccggtccct 360

taactcagag cgctgggagg cagccatttg tttaaagcgc ccagaaacct tcttccccca 420
tttccgcagc acttgcagct ccggcaggaa tctaggcttc cgggttctcg ggtcctttc 480
ctctcctaca cgtcagcgaa ttcagctggc cgtgagctcc ccgattgggg ctcggtccgg 540
ccaatattgt cactctaacg tgcactcgcg ccttccctcg gctctacagg gcatcgcagc 600
caccgatccc agcccctccc cggcctatgc cagccgcctg gtggccctcc gggctgcgga 660
gccaggcctg gcctgacagc agcagctgtt ttcttcccga agccgcagcg ctcaagggcg 720
tggttgcggt agacacctcg gatcccattt ggcctgtgcg gaccctaccg cgccaacccc 780
tcgtcttagg accgagaggc ccgacagcca ggtgggggtg gtctgggcag accggtcgac 840
atcgcaagtc agtgcggact gggagggcca gctgcccaca gcacggtctc caggaagcct 900
ctggagcagc gtaatgaaat attattactg gcgagcgtat ctggagggat cccggaaggt 960
ttaggcaaga tttgaagaat gctgctaaat gtttgccctc agggggtaga aggaaagagg 1020
gagggggctc cattcctgta aaaatctctt ctgaagtgaa tcattgctca gaatacctct 1080
ccctgcagca cgaatccact ttatgcacac acaatgtgca tcggctgcgc atccttaaat 1140
tatttcatcg ctttggcgtt tgcgcaaaac ctactttagg aaacaatttg ggccaggatg 1200
ggggtgggtg ggggggatga acggctgggc ggggtagcgg cagagaagtt agtctgaaac 1260
agcaacttta aatcgagcag atgtgagctt ccccgttaac tatattgtga gctcctccgt 1320
taatgtattt ccgagcagag gtgcttctgg ttccaagtgg agaaaatttg gaggaaacaa 1380
cccgagtgga tcaggatttt cgaggatgga aactcgacta agatggagtc actgtcgctc 1440
ctcctccata ccctccccat gagcccagag gaggaaggcg ggagggatgg aggcgttcaa 1500
gagagggcac cgggggcgct ctctgcacga gggaaagggg tgctcgacct gaggcgccgt 1560
ggaaagggat ttctgaaaat attttgtagt tcttttccag aaaatgagag acgaatgggg 1620
gagggtggca agcacctgac gggaacacga cccacaagca ctaatgtctc tgcgttgcct 1680
ccgccaggcg agaagccctt cagatgcgag ttcgagggct gcgagcggcg cttcgccaac 1740
agcagcgacc gtaagaagca ttcgcacgtg cacactagcg acaagccata cacgtgcaag 1800
gtgcggggct gcgacaagtg ctacacgcac cccagctcgc tgcgtaagca catgaaggtg 1860
cacgggcgct cgccgccgcc cagctctggc tacgattcgg ctacaccgtc tgccctcgtg 1920
tcgccctcgt cggactgcgg ccacaagtcc caggtggcct cctcggcggc ggtggcggcg 1980
cgtaccgccg acttgagcga atgatgtcca ccgcgttgct cgcaaggtaa tctcgctccg 2040
cgcagctgag cgccccgcat ctcgcgcctg ctacatcaaa gggcccgcgc acaaagcagt 2100
gtttcttcgc cacggtgcat cttcatggta agttaggatt tctatggcaa tgtgcaagtc 2160
gcactgaaat cctgaaaggc caagcctgga gcccgtccag gcttttcatt aaggacataa 2220
tatttacgtc taacagacct tttttcttgt gtatacaagt atatattttt gtttgacgcg 2280
gactaaatca ttttcattta atttccggta aacaaaaccc acgcgaatgg gcacttgtac 2340
ccgatcataa taaaaatgga taataatgtg aaggaagaaa agagccgctt gaatcgccgc 2400
tcagccccct ttgtttctgc tttctgcggt gatcagaggg cgcgtttggg tttgatggcg 2460
agtttctaaa ggcgaggaaa tggtttgtaa gaggggaaag aaaaggagaa aggtctaatc 2520

aagctcgggt tgttcaaaga gtcgggtttt ggggttgaaa gtgtgagttt gacggtgcat 2580
cagcatgccg cgttaggctc gccatggaaa tacgcgcggg gagcggccgc ttcaaaggcg 2640
gcacacttca ctacagacac tctattaaga tacatttgcg ctgacctttg ctttcacgcc 2700
atttaatact gtcactgcgc tctccagtat atacttcctt tctagaaccc ggcttgccca 2760
cgtttagggg ttcactctgc accctgatgt gggaggcttt ggcgcagggg acactttcag 2820
gaaagggagg agcacaagga ctctgtgcat cttgactgca ccccaaagag gctccaggat 2880
caggagtgaa agattttaaa gcagcctccg aagcttaaca aatgagcatt ccaagctcag 2940
ttttgtgcaa atcgcctttc tgactcttga gtaggatgga ggcttaaatt taatggcgac 3000
ttgggggaa gggagccacc ctgggggagt ctgaggagtt cagactgtgc ccttgggaat 3060
ttccactctg gctttccgtg ccactcttct tcctttccat cccaaaagtc tcttgcggcc 3120
cctgaaactt gtttctttct aaggcagggt gtgtggtacc cttaggcctg gactagtcct 3180
agatgcaaac tcaagagccc aaggccaagg ggatgtgggg aagatggcag gaaagttaga 3240
agtccatgtt cccttaattg tcttgttgtt tattttatcc aagtacccca gtgaataggg 3300
gaaaaataaa cacagtg 3317

<210> 43
<211> 1749
<212> DNA
<213> Homo sapiens

<400> 43
   gaaaggggga gtgggggagg gcagagcctg tggttggtat ttcagctacc ggcaaagccc 60
   tcctcaaatc aaaccctcca gggaaggcgg gtattagcag ggtggctgag gggcacctgc 120
   aatgcggaga ttaatcccct ttttgttttc tttcgggtct atctcagcac tgctgagtgg 180
   aagaatttat gttggcctcc ttcctagaag gaagctgcag gctcagaaaa gaggatgtgt 240
   catcagatgt cacctctcag ggtgcctctg ttgctatctg tctgtcgctc tgcccctccc 300
   taggtctctg cccctccctg ccaatcagtt ctaccttcct ttcactcctc agagttttat 360
   ttctgtggct cgaattctga tttcgtacca gaggaagaca acaacagctc tattaccagg 420
   tcccctgctc ctctccaatg tgccccagac aggtattcgg cgaccagagg agagctgagc 480
   tgggctttga tcacactctg cctctgcatc cgcggagctt gctgggccat gcagatgtct 540
   tctgcaagtg agctaaggcc atggaaaaga tccctcgggg gccatttgag atgtagctca 600
   cagtggcctt caacattttt atttaagctg tggagtcatt ttctggaaaa taaaatatca 660
   ttgacaacct cactatgtga aatggatagc gtgctcttat tgtacagggg tgagaagagg 720
   atagaggagg cctgaagtta gcccactgag ccctccactt cccaggaccc tgaggccact 780
   gagcacaggc ccttgtgcag ccctgctccc ggacccaccg tgactgtgat gtcctggagg 840
   aataagtgcc ctccttccat tcatacaaag gttcccaact ggctaccaaa ggctatggcc 900
   agtccctgga aggcatgctg tttccaccag gatcctgaaa tcccaggttc tcaaataggg 960
   tcctacattt tcaccaagga gctgcacact ttcccagtgt atatgtgact attcgtcgcc 1020
   agcatgtgca cgggaccatt ttggtgtgtg attttttcat atactttcca gggtgtttat 1080
   gactagggtg caattttaga aatgagagct ttggtagcaa ggtcctgacc atcccagaga 1140
   aggattttgg caaaaaatag tagttgctag ttatttactc ttcctctttt tgctagatca 1200
   ggtggcagaa aagatgagtt attgtcttac tatcttatct tccccagtat ttcatttccc 1260
   aatactgaag acagtggagg cattttggac ctaagaacca gtgtttcaga agatggcatt 1320
   taccctacaa tggccgtgcc caaggtaaac tctgggcttt gggctgcatg gccccaatcc 1380
   agagaacaga gggactattc ctggctcctt cctgagctgc tctaacatcc tgcccgactg 1440
   agcccagtta tgcttcattt acaagaagag gccgcgttag ctcctggctc atgtggagtt 1500
   aaatagagat gagatgtaac actttttttc taacagcaca aaactccttc ctgaagagtc 1560
   accttgaaga gagatggttt cttgtgcaga atcgcagtct gggcaattaa gtcagagaac 1620
   tctgagttta tatgctgtgc gttttcattg actgactctg tggctttttg gagaattaca 1680
   tttcctctct gatattgtta attgatgaag tctcacaact tcactaggga ataaacttat 1740
   gctgacaag 1749

<210> 44
<211> 2268
<212> DNA
<213> Homo sapiens

<400> 44
cttaatgcct tttcagttt gattgtctac atggagatca gggtgataag tttcaataaa 60
catatagacg cccccccacc cacccccatt agctagaaaa gaccaggact gggttgcttt 120
aaatgaacca agcttgacca acaaacaaga cagccatttg ttcactcaga tctagccgag 180
gtaactcaca agtgcacctt gagatcctcc atgtgtctgt caacagcgat aggcaggatc 240
tgcgcccagc tcatgctgca ttctaagaac tctcatttca tttgcatata aaattcatta 300
caggaagtaa ttaactgaag gaacagcatc atcaaaggct caaggataat ggaaaccgtc 360
atcatgatta cctactggga cctcatcagc cacagtgaga tgttctctga cagttacatg 420
agccaggaaa ttgcagacgg gctgcgcctg gaggtggaag ggaagatagt cagtaggaca 480
gaaggtaaca tttttgactc gctcattggt ggaaatgcct ctgctgaagg ccctgagggc 540
aaaggtaccg aaagcacagt aatcactggt gtcgatagtg tcatgaatca tcacctgcaa 600
gaaacaagct tcacaaaaga agcctacaat aagtgcatca aagattacat gaaatcaatc 660
aaaggcaaac tggaagaaca gagaccaaaa agagtaaaac cttttatgac aggagctgca 720

gaacagatca agcacatcct tgctaatttc aaaaactacc agaaaacatg aatccagatg 780
gcatggttgc tctgcggact actgtgagga tggtgtgacc tgatatatga ttttctttaa 840
ggatggttta gaaatggaaa aatgttaaca aatttggcaa ttactttgga tctatcacct 900
gtcatcataa ctggcttctg tttgtcatcc acataacacc aggacttaag acaaatggga 960
ctgatatcat cttgagttct tcattatttt gactgattta tttggagtgg aggcattgtt 1020
ttttagaaaa acatgtcatg taggttgtct aaaagtaaaa tgcatttaaa cttaaaaaaa 1080
aatacctgag actgggtaat ttagaaaaac aatagatttc tttatcttgt ggttctatag 1140
gctgagaagt tcaagggcat ggccctggtt tctggcaagg gcttttgtgc tgtgtcacaa 1200
catagtggag aaggtcaaag agaaagtgga cacatgcaaa gaggggaaaa cctgagtggc 1260
atcccggctt tataacaacc cactgtcaca ggaactaatc cattcctgag agaactaatc 1320
cagtcttgtg agactgagaa ttcactcact cctgcaagac tgccaccaag ccattcatga 1380
aggatccatc cccatgaccc agacacctcc cactaggacc caccttccaa catttgggga 1440
tcaaatttca acatgagttt tgatggagac aaataaacca catccaaacc atagcacagg 1500
gactgctaaa taccccaaca acagaaatag aaagctcccc taaacacctt ctttaactga 1560
gacctggctg tctcctgaga accccacttc gcctgtaggc ctctcctcag agattctttc 1620
cctcccccaa atacttctgc atctggaggt ggagtgggcc ccttctttgc tccccactgc 1680
accttccaga ccatgaccgc tgtacctctc aggcacttcc ctccattcta tccttttgcc 1740
caccccctag tcttgcccaa atccaaatca cttcaacatt catgtgaaag atctatccca 1800
cttcccacac ttcttgtttc ttcggcctac tcacctctgt tgccctccct ttctgctcca 1860
gtcacccact ctcagggccc caagtggatc ttgcgatggc ccagaactgc tgcaccactg 1920
aaatcttaac ttcaatcccc acaacctcac atctttctaa ctttcccatc tgattacttc 1980
attacactgt tcagctttgt tgagacttgt atgagctcat cacaaaagaa gaaatcccaa 2040
tgtccaataa acatggaaag gtgcttaacc tcatctactg tcagggaaac gaagaatgag 2100
ggagaatggg gcatgaggaa ggagcatgag aaaacttttt gaggtaatag atatgtgcat 2160
tatcttgatt gtggctctgg tttcaaggat gtttactaat caaattgtca cttaaaacgt 2220
gcagtttgtt gtacatcaat tatatatcaa taaagttgta aaatatac 2268

<210> 45
<211> 1884
<212> DNA
<213> Homo sapiens

<400> 45
attcagctgc ccggccagga gagacactca gctgcccagc agcccaagct ggacatgccc 60
ctcccaggaa ccccaggacc tgtcaccacc tccccacaga cccccacccc ccgccccctc 120

accaccgact ggcgcatctt gtccggcaag gggtccgggg gctcggcccg ggctgtctcc 180
aagctgcgct cctccagctc agggaacagc ttgctccgga tcagagacct gggggtgagg 240
aagagtcagg aggaggccgc ccctccttcc ccccggcccc agtcacgggc acatgcacag 300
accacaaacc cctactgggc agacacaaac acacgacccg gagcacaccc accagagcac 360
ggtggggtcg ctgctctgcc ggctcaggtg gtaggacagt gccaccagga ggccacagaa 420
gactgagaag aggacaggga cgtgctgctc tggccacgga gtctgcggag agaatccatg 480
ctggtcaggg gaggtgaggt aagcatgggg cccgggctgc ccccgtgtga gaagccactg 540
ctgagcccag ttcacagccc cgccccaacc acccttacgc actgcagcat gcccccacct 600
cgcaagccac ccctaccttg atggccccaa ggcagaaacc gtagagcagg gcagcagcca 660
gcaggctgcg ggagaggctg aagactgccg tgagcgggct ggtggcagct gtgtggaagg 720
gagaagtaca gcccagctct cacttgcagg taccccacct caaccctagc cgcgcttccc 780
ctcaactccc agaggctggc ctccctggaa aggcaccccc agcccattgt tctctgctgc 840
cccaagcccc aagcggcctt tagagcttcc aagattctca gatataaacc acttgcctgg 900
ggagggaagg agaggggaga aggcagaaac cttgagtctg cagtacagac gcaggagaag 960
gttccgcaag ggtggagcca aggacccaat ccgagggccc tcctgggcct ccacacgggc 1020
cccatccctg agccacctgt gggcatcata cctgtgcccc cgaagccgtg catatctatt 1080
tgctccagca ggtacatgag gcaggtgttg acctggggca ggaggcccag gaggaagacg 1140
aaggggaagc acagggtgaa cactggcagg gaggggacac gaaggcctgg tcagcctgct 1200
ggctttccgc ggcccaccct cggggactca ctctcctggg ccacctcatc cctgccccag 1260
cctcaccagt ggccacgtct cgggcacaga agaagaagga ggcagagaag agcgtgaggc 1320
cgtagaggga gacaggtggg aagggctgag ctgagcccag ggcgtccagc agccagatga 1380
gcagacagca gatgcagaag tagacaggac ggctgtacgc gatcacccag ttgtggccct 1440
gaggggtcag cccatgagga gctgggtctc cccgactggc cacacctctg acacatgcac 1500
acactcaagg gccccaggcc tggccctggg tccaagctgg agagcctggc caggtggggg 1560
tgatgggggt ggtgagggag ccccagccct ccatacccta ccctcctccc cacctggtct 1620
cagcccctcc cggggcctcc tggccagagg cgctgctcca tgggtactca cgtgcatggg 1680
ggacgccgca tcaggctgca cgctctggaa gagacagggc tacgttggca actagagccg 1740
atgcccacgg gatggtcaga ggccccagac ctgttcagct ccaccactag gcagttatga 1800
gtgaccaggt caggcccccg aactgtcctg agcctcagtt ttcttgcttg taaaagagct 1860
ggccagctct aggaaaccac acag 1884

<210> 46
<211> 1570
<212> DNA
<213> Homo sapiens

<400> 46
cattgacttg tcagggcccg ccagaggccc agagcgcccc cgcagcccgc agccagagct 60
gccgagatgt ctgttcttcc cgcccactcc tgccaccccc aactcccctc ctactccctt 120
ccgttgagaa caaggaattt ccagcctgta gcaaagggaa agaggcaaag acaaagacgg 180
agaccatggg aaactataga aaaacataaa gagacaggtg tgtgtgtgtg gggggggagt 240
ggtgacaaaa agggagtggg gacaaataca aaaattttca tttgtttcct gcatgggacc 300
tgttctcccc tgtgtcccct tggctcactc aaagaaggac cacttctgtg cctgactcac 360
ctgctctccc catccccagt gctctcaaag acagaccaga ggaactgatc tcgtgataaa 420
aaggattaat attcttttat ctgtgctggt ctcctccgtg aagggaaaca gtatcctcag 480
tcctagtttt ccaagaaaag gggaaacgat tgcgaggaaa gggaggaaag tgggcggaag 540
aggagtgcag agactgtcgt ccctacgcgc acgtgtagat tttcatctag cagattgctc 600
tgctcactcc tcccccaacg tctccactgg gccagatccg actcactcag caaggtttgg 660
ctcaccctct ccttcctgga ccccttccac tcctccaggc agaattaatt cctcccaccc 720
ctcggtggcc tcctcattgc tctttgtaca catttccaat tatagttgct tgtttcagcc 780
gaaatccagg acctctccag gataggcctg ttatctggtg gaccctctga aatgaataaa 840
ttgatcctat tcacaggcct gtgcaacttt gaaagggcac atgacacaga ttttactccc 900
aaaggggctg agcctgggca aaactgttta agtgaagaca tttgttttcc atatctcttt 960
cccttgtaaa gtttccagtg agtcccacaa atctggctcc ttggccttct aaaatgtcca 1020
ccattctggt aaattaattc acactcagat gctaaaggta gtaggccgaa gtcctggctg 1080
gtaatagaaa agtataaagt aatgatccct tttttctctt tcagtaacat ggcagaattt 1140
atcatctaag gaatgttgtc cttcaagtta gttattttac aagaaatgtt tcactcctga 1200
aagacagata atcctaacca ctgcctttaa ggagctcaca gactaatagg ggaaatagac 1260
ctataaacga ataattacaa catagtgcaa tgatatgggt acaacaatga cagaagtata 1320
agagggtgag tgatctgttc tgcttggagt tgtgaggggc cttggtttag acaggcatcc 1380
aaaggaggtg atgatgcagt cagtatgaga tttccaggag tggccagggc ggtggctcac 1440
acctgtaatc atagcacttc aggaggctga ggtgggagga acatttgagc ccagaagttg 1500
aaggctgcag tgagccttga tggcgccact gcatccctgc accacagagc aaaaagtgag 1560
tctcaaaaac 1570

<210> 47
<211> 1667
<212> DNA
<213> Homo sapiens

<400> 47
ctaacttttt cagatcatgt tcttttttga gaatattaat tttttctcta gaaaaaatac 60
acatacaggt aaaactatct atctattaac ttcctgggag gcataaagag cccctgaagt 120
ccaaccatgg atcacaggtt aatcaactcc caattgtaat ggtctgtgtt tacttttcac 180
attagtttta cttgaatagt atttgtccca aacctactta agagatcctg acattttttt 240
acattattgt ttgcatgttc ctccattcac ttaatgaaac caagagcaca tctctcacaa 300
agctcatgtg cagcccctct gacattaccc acagcacaac aggtggaatc agtgattcat 360
tgagacagct atcattccag ggagcttggg ctcagggatg gtggagcgac ccatctgctg 420
ccatctgctg ccatattctt ctcatgggct cccgtcccca gactgaaggt atgggggcaa 480
taaataaaca ctcatgtagg ggtttggcca tcaatgccca agctcccaga gtcctaggca 540
ggccagtgtc tagggtgagg cagtcccaaa agggaacaaa gacagaggac ctcagccaat 600
ggtggcagag gaaaggacac agcagcctgg gcaagcaaga ggggtggagg caagcgacct 660
ccatggtcgc ttggcctcct ggccacactg gtgggtgggg cacagtacaa atgtccctca 720
catgtgggct gaagctgctg ctgtgctccc agcttgatat ggtttggctg tatccccacc 780
caaatctcat cttgaattgt agttcccata attcccacgt gttatgggag ggacctggtg 840
ggagataatt gagtcatggg ggcagcttcc ccatactctt ctcatggtag tgaagaagtc 900
ttacaagatc tgatggtttt atatgaggaa acccctttcg tttggctctc attctctctt 960
gtctgctgcc atgtaagaca tgcctttcac cttccaccat gattgtcagg cctctccagc 1020
cacgtggaac tgtgagtcca ttaaacattt ttctttgtaa attacccagt ctctttaagt 1080
atgtcttcat cagtagcatg agaacagact aatacacagc tataggtaga agtggctgat 1140
gaactgggcc ggaaagggac atgggttagt tcagcttgaa gagctaggaa gaagaaaaga 1200
aaggagaaga ctgaaggtgg cccctccaga aacactgcag actgtcagcc ttgtcaaatg 1260
cttgggttct tgcatgaagc gctccctttt ccttgtggct tagtggcagc aagtatgaaa 1320
aatggaagta ttgatggaaa aaatatacat tggcagaaga tgggtagttg ggactctat 1380
tggttagaaa tgcacatgca aacggctggg cgtggtggct cacacctcta atcctagcac 1440
tttgggaggc cgaggcaggt ggatcacttc aggtcaggag tttgagacca gcctggcttc 1500
tactaaaact acaaaaatta gctgggcgtt gtgatgtacg cctatagtcc cgactactca 1560
ggctcctggg gcagaagaat cacttgagcc tggtaggtgg aggttgcggt gagccgagat 1620
catgccactg tgctccagcc tgggtgatag agtgaaactc cgtctcg 1667

<210> 48
<211> 2189
<212> DNA
<213> Homo sapiens

<400> 48
agattgaggt attttgaaag atatcaggaa aactcgtaaa ggatttagaa ttgaggtcta 60
aaatagattt tttaaaaagg catatggggg aaaaatgtca gaaaaataga aatttcatgg 120
ctgggagtct tagagagacc aaacagaata taactataat gttacaatgt attctactca 180
gaagagcctg tccatggcca cagttgcctt tactgtggat tgataatacc tttaacactt 240
cagtaatcca taaattgaaa agtttaggcc gggcatggtg gctcacacct gtaacctcag 300
cactttgcga ggctgaggcg ggcggatcac gaggtcaggc gatcgagacc atcttggcca 360
atatggtaaa accctgtcta ctaaaataca aaaaattagc cgggcgtggt ggcgtgtgcc 420
tgtaatctca gctacttggg aggctgaggc aaggaaatct cttgaacctg ggaggtagag 480
gttgcagtaa gctgagatcg caccaatgca ctccagcctg gcaacagagc aagactccat 540
ctcaaaaaaa aaaaaaaaaa aaaaaggttt agttcatagc tgcagagtgt ttcttccagt 600
tcctaatgtg gatctaccac ccctgtgttc atcgagattc cggtctggac ctcctggaga 660
agaggcccaa gtggctagcc aattcattgc agatgtcatt gaaaagtaag agcaccatga 720
taaaatttta ttacaattca ttttcagata gttttgggct tttcatttac tttgctcttt 780
gtcattaaat caagttcaca gataattcag aaagaggact tatgcatttc tccaggaaag 840
gtaagaggaa tagagaagct ataagttctt attaattctg aagggatttt ttttttgtgg 900
gggaaagtga acaaggaaat taaaaccacc aatcaaagta gctttaaata tggatgtctt 960
acagatttta gagattcagt aatttcttac tctataattc tctaaatgaa ccatattgtt 1020
tataaaaaaa caagtttgac taacttactg cctgtctggg agctaccttt taaactgaag 1080
taattttttt ccccccttaac ttttaaagtt caatcatttt ttattcctta tagcagatta 1140
taacattaat catgttaaca tatgtttatg gtgaatccac acagtagatt ccaaagtcta 1200
tttgaaaatg taaaactagg ttgattagaa cctagagagg ctttcctact agatcatata 1260
aactgataca ttagcactgt aaaacttaaa catttcatgt attatgagaa agtgcatttt 1320
gaatttagcc tagggctttc aaaaccatct cccttatctg aaacctccca atgagaatgg 1380
aaattccttc tacatctgta gctccattaa gggaaatgga catgtttgtc atttgtcagc 1440
ctggcaatca aaagccagct ttcacaaaac ctgtccactt tcttctggtt ttctatagaa 1500
ctttaaaaag gtgaagcatt ttcttaaggt ttttgagcct tgaaagttat cttgcttgtt 1560
taaccaaggg ttctgtggtt cttaagtcta agcgaatagc atgttctacc cattagaaga 1620
ctttaatttc cttttgtgtt taattctatt ttagcttgtc tgggttctat actgtgatct 1680
catttgcctc gactacgatg gaaacatttt ggatgcctgc acatttgctt tgctagcggc 1740
tttaaaaaat gttgcctgaa gttactataa atgaagaaac tgctttagca gaagttaatt 1800
taaagaagaa aagttatttg aatattagaa ctcatccagt tgcaacttcc tttgctgtgt 1860
ttgatgacac tttgcttata gttgacccta ctggagagga ggaacatctg gcaacaggaa 1920
ccttaacaat agtaatggat gaggaaggca aactctgttg tcttcacaaa ccaggtggta 1980
gtgggctaac tggagctaaa cttcaggact gtatgagccg agcagttaca agacacaaag 2040
aagttaaaaa actgatggat gaagtaatta agagtatgaa acccaaataa acagccacca 2100

cattttcaaa acagatttgt aaaaattgta tttgttaaca ctgtgcacaa acgttttata 2160
ctaaataaat atcaaactac attcttctg 2189

<210> 49
<211> 2223
<212> DNA
<213> Homo sapiens

<400> 49
catggtgcta ataaggacat acccgagact gggaaaattt acaaaagagg tttcattgga 60
ctcacagctc ctcgtagctg gggaagcctc acaatcatgg cataaggcag ggaggagcaa 120
gtcacatctt acatggatgg cggcaggcaa agagagaatg aggaagatgt aaaagcagaa 180

acctctgata atatcatcag atctcgtgag acttactgtc acaagaacaa cacgggaaag 240
acccgcctcc ataattcagt catctcccac tgggtccctc ccacaacacg tgggaattat 300
gggagctaca agatgagatt tgggtgggga tacagagcca aaccatatct ttctgcccct 360
ggcccctccc aaatctcata tcttcacatt tcaaaaccaa ttatgccttc ccaacagtct 420
cccaaagtct caactctttt cagcattaac ccaaaaatcc aagtccaaag tctcatctga 480
gacaaggcaa gtcccttctg cctaggagcc tgtaaaatca aaagcaagtt agttacttcc 540
tagatagaat gggggtacag gcattggata aatacagcca ttacaaatgg gagaaattgg 600
ctaaaacaaa gaggctactg gcaccagtag gaactcagtg tggtgggtct gaccccacat 660
ttcccttcca cactgcccta gcagaggttc tccatgaggg ccccacccct gcagcaaact 720
tttgcctggg catacaggca tttccataca tctgaaatct aggcagaggc tcccaaacct 780
cagttgttga cttctgtgta cccacaggct caacaccatg tggaagctgc ttccaccctc 840
tgaagcaaca gcccaagctg tacattggcc cctttagtca tggctggagc agctaggatg 900
tggggctcca agtccctagg ctgcacacag cacagggccc accccaaaaa gccacttttc 960
cctcctggcc cacaaaaata ctttttcctc ctaggcctct aggtctgtgg atgggagggg 1020
ctgccatgaa gacctctgac atgccctgga gacattttcc cccattgtct tggggattaa 1080
gattccgctc cttgttactt atgcaaattt ctccagccag aaactccccc ttatgatact 1140
atcagatctc atgagacttt ctttatcaca agaccagcat gggaaagacc catccccata 1200
attcaatcac ctcccaccgg gtccctccca caacacatgg gaattatggg agctacaaga 1260
taagatttgg gtggggaaac agagccaaac catatcactg cactctggat gaatttagag 1320
attcctgaaa aaattttgtt ttaagagctt tgtggaactc agcttcatta gagaaaggac 1380
agcaataagg aggttaagat gaatgagctc ctggaaggga gatgtgcttc cctctctgcc 1440
tccaccccac ttttccattt ggccttgctt cttttccttc ctctctcact atttctcctc 1500

ttccttgccc ttctcccctc ttgcctttaa aagcagtaga gatgccactt tgatgacctg 1560
aaagcttccc cagtttccca gtttaaacaa gagagtatca tatttactgg atgaacttat 1620
tattattttt gagatagagt ctcactctgt cacccaggct ggagtgcagt gatgcgatct 1680
cggctcactg caacccccgc cttccgggtt caagcgattc tatgcctcgg cctcctgagt 1740
agctgggatt acagacgccc gccacacgtc tggctaattt ttgtattttt ggtagagatg 1800
gggtttcgac atcttggcca ggccagcctt gaactcctga cctcgtgatc cacccacctt 1860
ggactcccaa ggtgctggga ttacaggcgt gagccaccat gcctggcctg gatgaactta 1920
ttaatgaaag aaaagttggc agggtgcggt ggctcacacc tataatccca gcactttgag 1980
aagccaaggt gggtggatta cttggggaca ggagttcaag accagcctgg ccaacatggt 2040
gaaaccccat ctctaccaaa aatacaaaaa aattagctgg gcgtggtggc acatgcctgt 2100
aatcccagct actagggagg ctgaggcagg agaatcgctt gaacccggga ggcagaggtt 2160
gcagtgagcg gagattatgc cactgcactc cagcctgggt gacacagaga gagactccgt 2220
ctc 2223

<210> 50
<211> 2206
<212> DNA
<213> Homo sapiens

<400> 50
atggtgtgtg cacgtgtgca ctgtgtatgc atggtgtgtg catgtgtgca ctgtatgcat 60
agtgtgcacg tgtgcactgt gtgtggatgc atggtaatgt gcacgtgtgc actgtgtgtg 120
gtgtgtatgc atggtgtgtg cacgtgtgca cggtgtgtgg tgtgtatgca tggtgtgtgc 180
acatgtgcac tgtgtatgca tggtgtgtgc acgtgtgcac tgtgtatgca tggtgtgtgc 240
acgtgtgcac tgtgtggtgt gtatgcatgg tgtgtgcaca tgtgcactgt gtatgcatgg 300
taatgtgcac gcgtgcactg tgtgtatgca tggtaatgtg cacgtgtgca ctgtgtgtgg 360
tgtgtatgca tggtgtgtgc acgtgtgcac tgtgtatgca tggtaatgtg cacgtgtgca 420
ctgtgtggtg tgtatgcatg gtgtgtgcac gtgtgcactg tgtatgcata gtgtgtgcac 480
gtgtgcactg tgtgtggatg catggtaatg tgcacgtgtg cactgtgtgt ggtgtgtatg 540
catggtgtgt gcacgtgtgc acggtgtgtg gtgtgtatgc atggtgtgtg cacgtgtgca 600
ctgtgtgcat gcgtgtgtgg tgtgtgtgca tgtatgcatg gtgtgtgcat acgtgtgcag 660
cagcacctgg tcccatctcc agtgcccagc agcatcacac gcactttggt gctttataaa 720
tgcatggtca gtgaggctga cagcaccaag ctgtcccttt accataacac ctggaatagt 780
cacctgtgat aagctatcac ataggaaaca tttttaaaat ttcattctca ttattttctg 840
taatcttgag aggttccaat caacatttat tgccttattc tttttatctc attccttttt 900

gaatgtgttt atctcctaag attttatctg tgatggagat gggatgcctg tgaatacaaa 960
agttgcagtg gtggcaccag ggtggggggg tgcggccggg gccaccatgg tctcccctga 1020
gagggggtgc tgtcttaggt gccccaagag gccctcgggc agcaagcgtg gggtgctgcc 1080
aaaatacagc tccccctggg tgggcaggac acacgtggcc tcctggcaga caggtgcctg 1140
ggtgagcccg ctgctcctga ttagtcatga atggcacctg gtctgggcga cagtcacccg 1200
caggaagccc tgagctggcc accatcaccc tgggcagtgg ctcccggggt gccaacaaga 1260
cctgggcccc tcgttctttg gtgctgagag ccccagctga ggctgtggag gaggccctgg 1320
acctggtctg gtgtctgtca gaggcaggtg cccagtcctt tgacttgctt ctctgaattg 1380
tcataattgt gctggaattg tgccagaaac tggtagtgat aacagctcct ggaaggcctg 1440
tggctgctga gggctgcctg gtccccctca ggacggccgg gggagcctct ccagaagcac 1500
cagctttgtc tgcaggtgga cgttgaaggg gggcagttgg gtcaggttca gactcacacc 1560
tgtggtcccc acgacgctgg ccaggacccg gtgtgtgtcc ctgtagaggg ccaggagccc 1620
aggggccgcc tccatgagga tgtgtgtgta tgccagcatg cctgtccccg gctgcacatc 1680
ctccctcttg tcgtacctgt gggatgagag ctggtggtcc tgcccagggc ccccacatcg 1740
ccctgtcccc cgcccccaag gggccctcac atggcctctg ggagggccgt gctgtgtgtg 1800
gatcctacct ccaggcgctg ttgacttgga gaaaccgaga cacacctgtc tgggcggctg 1860
ccacgtcaat gtgcagaagg acgtctagga aaaccaggcc tgccgtcaga gcccagccct 1920
gcagtcggag ccacagcagt ccccaggcag tgcccccagc acccacctgt ctgggggggc 1980
accagctggt gcagcctctg cattgcgacg ccacctgggt agttgaaatg ggacacatac 2040
agggccgtgg ctgagtaggc ggcattcacc acgaggtgtc cgatcacaag cagagacccc 2100
gctttgtaca gccaagactt tttatagtta ttcagcctga aaaaagaatg gttacacatc 2160
ataggcagaa cgtaatgaca ataaaattaa tctacctaca aaacat 2206

<210> 51
<211> 1847
<212> DNA
<213> Homo sapiens

<400> 51
ttcgtatgcg tgtgtgtgtt taacctcaat aaaaaatatt ttaaaaaatg agggtggaat 60
taaaacatcc tcagatgtct tagtcttttt gtgctgctat aacaaaatac cttaagactg 120
gataatttat gaagaacaga aatttgtttc ccacagttct gaaggttggg ggaagtccaa 180
aatcaaggct ccagcagata cagtgtttga taagttgccc tctgctttca agatggtccc 240
ttgtttctgt gtcctcactt ggcagaagga atgaacttgc tcccttgagt ccttatataa 300
agtcattaat cctatccata agggtggagc tagtatggcc taatcatctc atgaaagccc 360

cacctcttta ttttttattt ttggtagaga tggagtctca ctgtgttgcc caggctggtg 420
tcaaattcct aggctcaagt gatccttctg cttgggcctc aaaagtgctg tggttacagg 480
tgtgagccac acctcttaat cctgttgcat tggggattaa gtttcaaaat gaattttgga 540
ggaacacaaa cactaaaact acagcaccag atgattcaaa agcttaacga gtttgttacc 600
actagatctc ccttcatgaa ggaagtcatg aaggttgaaa taaaaggaca ttagacagga 660
aatcaaatct gtatgaagat ataatcatgt ctgataaagg taaatacatg aaaaattata 720
aaagctagta gtgtttctgt tttctacatg atttaagaga cttatgtatc aagaagttat 780
tagtctgtgt tttggacaca caacacattg agatgtagtt ttgtggcatt aatggctgaa 840
aggtgatggg gttggagctg tataggagca gtttttatat gttattgaag ttaacttggt 900
atacattcac atcagagtag tataacttca gggtgttaaa tatcagcccc atggtaacca 960
aaaagaaaat aactgtagga tatacacaaa aggaaatgaa aaggggatta aaatgttttt 1020
ctacaaagat caactaaaca caaaataaga taataatgca ggaaataggg gacaaaacaa 1080
ctgtgaggca tatatacaaa acaaacagca aaatgacaaa agttctgcct tatcagtgtt 1140
taaatgtaaa tagcttaaac cctgcagtcc aaagacagat tggcagaatg gatagaaaaa 1200
catccagctt ctagccataa gagacccact ttagatgaaa gacaaaaaaa tttaatgtga 1260
caggatggaa aaagatactc tcttcatata gtaaccaaaa gagatcgggg ttgctacact 1320
aatatcagac aaaatatact ttaaatcaaa aaagtttacg agacaaagca ggacattata 1380
tattaataaa aggtttaata cggcaaaaag acaaaacaca ctaataacag accactcaaa 1440
tatgtgaaac atgactgata atggaaggga gaaatagttc tacagcagta gttggggact 1500
ttaatgggta gaagaaccag gcagaaggta agtagggaaa tagaatatag tacttgaaca 1560
atataagaag ccaagtagat cttacagaca tacacggggc attctactca acaacagaat 1620
gcatcttctt cttaagtgga cttaggacct tctttaggat agaccataca ttaggccaga 1680
aattaagatt caagagattt ttaaaaatac gataccaagt attttctttg acaaaatggg 1740
atgaagtgag aaatcagtaa cagaaggaaa actggagact ttatgtaaat taagtaacac 1800
cctcttcaag aaccagtggt caaaggagaa atcacaaggg caattag 1847

<210> 52
<211> 1928
<212> DNA
<213> Homo sapiens

<400> 52
ttaatagaat tacaaagggg ttggcttaga tcagggggtc aggaaaggct tctcacagga 60
agaggtgggc aggagagttc aggcagaagg cctgaggaga atgggcttag ctgggcaccc 120
ttgaggaatg gacaggaggg tgtggtgctt tccctgaaag aaggttccat tctgtgcggc 180

tgaaaggacc agagcaaggg gcaagggttt tattctaagt gcagtgggag agcactgaag 240
gggtttaagc agcacaggat tcagtgtgca tttaagacgt gcactctgcc catctatgga 300
gaggctgaga gtggatccca gggatggtca cgaacctcca gagagccact ttatagtttc 360
caaagcttcc acaaacacca tctcaccact tgttttaaat gcagcccgag aagtcaaagg 420
aggtggggta tggggggagc atttctgtgt tcaccctttc tgtgactcag agaaggattc 480
taaggtggag ctacagagcc aggaagtggc agaggatttt aatcgcctcc ttggaggaga 540
ggaaggagcc tctgaggcca gaatgtatcc cagccagaag tcagaggacc attccctacc 600
ctcagtcatc agaagggctc ctgacctcag gggctgctgg gggcagcctg tgaggggcac 660
aaggctgggg ctgtgatgac gatgctcctt ggacatgcct ggaagggatg gaggtggggg 720
ctgactgccc accagggcct ctgctccctc actgctgcct cctcatcccc cagcctcctc 780
tgggattgtc cttctcaggg tcagccagct cccactgcag tgcagggacc aacatgccca 840
tctcctccca catcctggcc cccaccacag ccaacctggc cccgtgcctc atgccctgcc 900
ctcctgacca gcttcctaac tcactgatac tggcttccac tcggagagcc tacagctctc 960
tgccatctac ccacctaagc ttgggaatgt ctttctctgc ttgtgctctg tagccagctc 1020
acatgtcacc aggctgatgc acactcagct cagtggggct gcatttacct ttcctaggga 1080
ttccccagcc tgtggtgccc tccccagtgt gggtagagaa tagggtctgc tgcctcctct 1140
tgcaggtatc tcttctcgga tgggaatggg cgcccccagc tcccactggc cacctttaag 1200
agatcagagg gcaaccgggc actgtggctc atgcctgtaa tctcagcact ttgggaggcc 1260
aaggtgggag aagcgcttga gtccgggagt tcgagaccag cttggataac atagcaagac 1320
tatctctatt aaaataaata aaataaaaag atcagaaggc tggctgggtg ctaggcacag 1380
agggcatggc tgtaggcatg gggctctggc ctgaaggtag ctgtcagcct gacagcatga 1440
ggttcctgat gtgcccatac tggtagtggg tagggtgtgg gtagggtagt gggtaggttg 1500
ggggaagtcc tggggaggtg aggagcacag agaacccaac ccagccgagt ccaagacgtc 1560
caggatcttg ggtctctctt gacttcacgt ttgactaatt tcctcctccc atctgagtaa 1620
tcataatgat aaagatagca gcattgggcc aggtatggtg gctcacgcct gtaatcccag 1680
cactttggga ggctgaggcg ggcagatcac ttgaggtcag gaatttgaga ccagcctggc 1740
caatatggta aagcccagtc tttactaaaa atacaaaaag ttgccaggca tggtggcaca 1800
tacctgtaat cccagctact tgggaggctg aggcacgaga atcgcttgaa cctgggaggc 1860
aaaggttgca gtgagctgag atcacgctac tgcactccag cctgggcgac agagtgagac 1920
tgcgtctc 1928

<210> 53
<211> 2767
<212> DNA
<213> Homo sapiens

<400> 53
catacatttt acttctacat atgttattcc caaaattcat tgtcattatt tttgttttaa 60
actgttaatt aactttttaa aagatttaaa catgagagaa aattgtcttt taagtttatc 120
cacgtattca ctatttctgg caatgttctt tcctttgtgc agtttcagat ttccatatgg 180
tgtcattttc tttcttttaa agagctttct ttaatatctc tttagcatat atctgctagt 240

tatgagtttt ctcggctttt gattatctgg aagagtattt tgccttcatt tttgaaggtt 300
taaaaaattt taaagacggt gtcttgctct gtcacccagg ctcaatcatg gctcactgca 360
accttggcct cctgggctcg ggtggtcctc ctgcctcggc ctcccgggcg gctggagctg 420
ctggcgtgta ccaccacatc tggctttttt tttttttttt tttttgtaaa gacagggatt 480
tgggccgggc gtggtggctc acgcctgtgg tcccggctct tcgggaggcc gaggcaggtg 540
gattacctga ggtcaggagt tcgggaacag cctggtcaac atgttgaagc ctcgtcttta 600
ctaaaatgca aagattagcc aggtgtggtg gcgggtgcct gtggtcccgg ctgcccggga 660
ggctgaggcg ggagaatcgc ttgagcccgg gaggcggagg ttgcagtgag ccgggatcgc 720
tccgttgcac tccagcctga gtgacaagag cgagactttg tctcaaaaaa aaaaaaaaaa 780
agacgggttt gccatcttgc ctaggttggt ttctgaggta tttttgctag aaagagaatc 840
ctgcgttgat atttttctcc caggagttta agcatgtttt actgtgcctt atttggttac 900
tgatgagcat cctactgcca gtcccacctt cgtgtctctg tctgccttca tgatctcttt 960
atcacttttt tgtttgtttg ttttgttttg agacggagtt ttgctctgtc atcccaggct 1020
ggagtgcagt ggcgtgatct tggctcactg caacctctgc ctcccgggtt caagtgattc 1080
tcctgcctca tcctcctgag tagctgggat tgcaggcgtg cgccaccacg cccggctaat 1140
ttttgtattt ttggtagagt cggggtttcg ccatgttgtc cgggctggtc ttgagctcct 1200
gacctcaggt gatccacctg cctcggtctc ccgaagtgct gggattatag gtgtgagcta 1260
acacactcgg cctatcactg gtttttagca atttgattat gatgtatttg ggtgtggttc 1320
tcctcatgtt tcttctgctt aggtttcatt agaattgttg agtgtgttgg gttttagtta 1380
tcaaatttga aaaatcttct ggacctgtgg tgcagcctca gtgaggaggg tcaccctgca 1440
cccgtcgccg gccccggtct cccagggcct cacccgagtg atgccccgct attgcgcggc 1500
gatttgttgt aagaactgcc ggggatgaaa caataaagac cggaagctga gtttttatcc 1560
atttcctcta catgacaaag aaagactgga aaagtggtta aacaatatga agcgagattc 1620
atgggttccc ggtaaatacc agtttctgtg tagtgaccat tttactcctg actctcttga 1680
catcagatgg ggtattcgat atttaaaaca aagtgcagtt ccaacaatat tttctttgcc 1740
tgaagacaat cagggaaaag acccttctaa aaaaaaatcc cagaagaaaa acttggaaga 1800
tgagaagtat gcccaaaagc caagtcagaa gaatcagttg tattaaatga gacaaagaaa 1860
aatatagtta acacaaatgt gctccctcaa catccagaat tacttcattc atcttccttg 1920
gtaaagccac cagctcccaa aacaggaagt atacaaaata acatgttaac tcttaatcta 1980

gttaaacaac atactgggaa accagaatct accttggaaa catcagttaa ccaagataca 2040
ggtgtaggtg gttttcacac atgtttttat aatctaaatt ctacaactat tactttgaca 2100
atttcaaatt cagaaagtat tcatcaatct ttggaaaccc aagaagttct tgaagtaact 2160
tctagttatc ttgctaatcc aaactttaca agtaattcca tggaaataaa gtcagcacag 2220
gaaaatccat tcttattcag cacaattaag caaacagttg aagaattaaa cacaaataaa 2280
gaatctgtta ttgccatttt tgtacctgcc aaaaattcta aactttcagt taattctttt 2340
atatctgcac aaaaagaaac cacggaagtg gaagacatag acatcgaaga ctccttgtat 2400
aaggatgtag actatgggac agaagtttta caaatagaac attcttactg cagacaagat 2460
ataaataagg agcatctttg gcagaaagtc tctaagctac attcaaagat aactcttcta 2520
gagttcaaag agcaacaaac tctaggtaga ttgaagtctt tggaagctct tataaggcag 2580
ctaaagcagg aaaactggct atctgaagaa aatgtcaaga tcatagaaaa ccgttttaca 2640
acatatgaag tcactatgat atagaataac taggttttaa aactatggct gttaaataag 2700
ctttttccag ccaaaccaaa ctacatgtaa agtgaacttt ttcctgtata aagttctcat 2760
cttaatg 2767

<210> 54
<211> 2215
<212> DNA
<213> Homo sapiens

<400> 54
aggctgccgg gggctgcaga catggagggc cagagcagca ggggcagcag gaggccaggg 60
acccgggctg gcctgggttc cctgcccatg ccccagggtg ttgcccaaac tggggcaccc 120
tccaaggtgg actcaagttt tcagctccca gcaaagaaga acgcagccct aggaccctcg 180
gaaccaagga tcactgtggt cacatggaac gtgggcactg ccatgccccc agacgatgtc 240
acatccctcc tccacctggg cggtggtgac gacagcgacg gcgcagacat gatcgccata 300
gggttgcagg aagtgaactc catgctcaac aagcgactca aggacgccct cttcacggac 360
cagtggagtg agctgttcat ggatgcgcta gggcccttca acttcgtgct ggtgagttca 420
gtgaggatgc agggtgtcat cctgctgctg ttcgccaagt actaccacct gcccttcctg 480
cgagacgtgc agaccgactg cacgcgcact ggcctgggcg gctactgggg taacaagggt 540
ggcgtgagcg tgcgcctggc ggccttcggg cacatgctct gcttcctgaa ctgccacttg 600
cctgcgcata tggacaaggc ggagcagcgc aaagacaact tccagaccat cctcagcctc 660
cagcagttcc aagggccggg cgcacagggc atcctggatc atgacctcgt gttctggttc 720
ggggacctga acttccgcat tgagagctat gacctgcact ttgtcaagtt tgccatcgac 780
agtgaccagc tccatcagct ctgggagaag gaccagctca acatggccaa gaacacctgg 840

cccattctga agggctttca ggaggggccc ctcaacttcg ctcccacctt caagtttgat 900
gcgggtacca acaaatacga taccagtgcc aagaaacgga agccagcttg gacagaccgt 960
atcctatgga aggtcaaggc tccaggtggg ggtcccagcc cctcaggacg gaagagccac 1020
cgactccagg tgacgcagca cagctaccgc agccacatgg aatacacagt cagcgaccac 1080
aagcctgtgg ctgcccagtt cctcctgcag tttgccttca gggacgacat gccactggtg 1140
cggctggagg tggcagatga gtgggtgcgg cccgagcagg cggtggtgag gtaccgcatg 1200
gaaacagtgt tcgcccgcag ctcctgggac tggatcggct tataccgggt gggtttccgc 1260
cattgcaagg actatgtggc ttatgtctgg gccaaacatg aagatgtgga tgggaatacc 1320
taccaggtaa cattcagtga ggaatcactg cccaagggcc atggagactt catcctgggc 1380
tactatagtc acaaccacag catcctcatc ggcatcactg aacccttcca gatctcgctg 1440
ccttcctcgg agttggccag cagcagcaca gacagctcag gcaccagctc agagggagag 1500
gatgacagca cactggagct ccttgcaccc aagtcccgca gccccagtcc tggcaagtcc 1560
aagcgacacc gcagccgcag cccgggactg gccaggttcc ctgggcttgc cctacggccc 1620
tcatcccgtg aacgccgtgg tgccagccgt agcccctcac cccagagccg ccgcctgtcc 1680
cgagtggctc ctgacaggag cagtaatggc agcagccggg gcagtagtga agaggggccc 1740
tctgggttgc ctggcccctg ggccttccca ccagctgtgc ctcgaagcct gggcctgttg 1800
cccgccttgc gcctagagac tgtagaccct ggtggtggtg gctcctgggg acctgatcgg 1860
gaggccctgg cgcccaacag cctgtctcct agtccccagg gccatcgggg gctggaggaa 1920
gggggcctgg ggccctgagg gtggggtagg cagatgggcc aaggtgacca ccattctgcc 1980
tcaatctttt gcaagcccac ctgcctctct cctgctgctc ctccagctgt atctgcacct 2040
gcctctctgt cctggccagg ggtggacaac tggggtcccc caaaactcag tcctggcacc 2100
tcaactgtga caatcagcaa agccccaccc aggcccccat ctgggatgat gggagagctc 2160
tggcagatgt cccaatcctg gaggtcatcc attaggaatt aaattctcca gcctc 2215

<210> 55
<211> 2221
<212> DNA
<213> Homo sapiens

<400> 55
   actaatagga gaattttaaa tttccttgta catgttttta tttttggaaa ctaccctttt 60
   accaatatag tgaaaccctg gctctactaa aatacaaaaa aattagccag gtgtgatggc 120
   acacacctgt aatcccagct gctcgggagg ctgaggctgg agaatcgctt gagcctggta 180
   ggtggaggtt gcagtgagcc ggggtcatgc cactgcactc cagcctgggc acgcagagtg 240
   agacttgtct gaaaaaaaga gaaataaaga aagaaactac ccttttaatt catcagagcg 300
   ttttttgttt cataatacaa ggaagtaagt cctctctaac cctctttccg aaaaagctga 360
   ctagtatctc aaaaccactt aattttgtat tactttcttt gggactttgg aagttgcata 420
   agacatacag gttaacttgt aaagaatatc tttccagaat atgggttgcc aaatttttac 480
   tagattcatt tagggtattt tttgtgagga tactgtattc ttgggcattt tgttcttttt 540
   aattcctttt atgagtatac actctctgat tgtcttttct tatttcgcac ccagattttc 600
   tgcagtagct cagtggaata ttttgagctt agtgtaagat agaccgtttg taaataaatg 660
   taaagtattg catagaagaa tttatttgtc ttagaccctt aagcagcccc tatgctcccc 720
   cagtgaagtt gatttagatg tgtagacaac cttgaggaaa gagaagggct cttcagctgc 780
   tctcctgtct ccaagtttat ctaaatctag ggttctgtga aatgatttag tcttcagtgt 840
   ggattttgct ctgtgggata gcctctgagt gtaaatgctt cctgtcctaa gacccccgaa 900
   accagacgag ctacctaaga acagctaaaa gagcacaccc gtctatgtag caaaatagtg 960
   ggaagattta taggtagagg cgacaaacct accgagcctg gtgatagctg gttgtccaag 1020
   atagaatctt agttcaactt taaatttgcc cacagaaccc tctaaatccc cttgtaaatt 1080
   taactgttag tccaaagagg aacagctctt tggacactag gaaaaaacct tgtagagaga 1140
   gtaaaaaatt taacacccat agtaggccta aaagcagcca ccaattaaga aagcgttcaa 1200
   gctcaacacc cactacctaa aaaatcccaa acatataact gaactcctca cacccaattg 1260
   gaccaatcta tcaccctata gaagaactaa tgttagtata agtaacatga aaacattctc 1320
   ctccgcataa gcctgcgtca gattaaaaca ctgaactgac aattaacagc ccaatatcta 1380
   caatcaacca acaagtccat tattaccctc actgtcaacc caacacaggc atgctcataa 1440
   ggaaaggtta aaaaaagtaa aaggaactcg gcaaatctta ccccgcctgt ttaccaaaaa 1500
   catcacctct agcatcacca gtattagagg caccgcctgc ccagtgacac atgtttaacg 1560
   gccgcggtac cctaaccgtg caaaggtagc ataatcactt gttccttaat tagggacctg 1620
   tatgaatggc tccacgaggg ttcagctgtc tcttactttt aaccagtgaa attgacctgc 1680
   ccgtgaagag gcgggcatga cacagcaaga cgagaagacc ctatggagct ttaatttatt 1740
   aatgcaaaca gtacctaaca aacctacagg tcctaaacta ccaaacctgc attaaaaatt 1800
   tcggttgggg cgacctcgga gcagaaccca acctccgagc agtacatgct aagacttcac 1860
   cagtcaaagc gaactactat actcaattga tccaataact tgaccaacgg aacaagttac 1920
   cctagggata acagcgcaat cctattctag agtccatatc aacaataggg tttacgacct 1980
   cgatgttgga tcaggacatc ccgatggtgc agccgctatt aaaggttcgt ttgttcaacg 2040
   attaaagtcc tacgtgatct gagttcagac cggagtaatc caggtcggtt tctatctact 2100
   tcaaattcct ccctgtacga aaggacaaga gaaataaggc ctacttcaca aagcgccttc 2160
   ccccgtaaat gatatcatct caacttagta ttatacccac acccacccaa gaacagggtt 2220
   t 2221

<210> 56

<211> 2007
<212> DNA
<213> Homo sapiens

<400> 56
catgcacagg gcctgagagc cggtgggtga ggtagggctg ttcaagggag ggtgggctgg 60
ccttgccctg ccctgcccag atgttgacaa cgtcacccat tcccgccacc agcagtcccc 120
tctgggagaa ggccaggtgc cctgctccat ggggcagggt ccgagtgctc agaggctggt 180
acgtccctcg caagtcaaag atcttcagct ggtggtctag gccagaggtg gccatgtacc 240
tggtgagaga agagggatca attaatatgt cagtaaatgg gtttaccaag caagctgtgg 300
ccaagtccag gcatcaagtc tggctgggga gaaaaagatt aatagtaata accactgcca 360
tcaccctgaa cactccacag gcatcctctc agttaagctg cacacaactc atactatttt 420
tatttccctt taagaggtga ggaaactgaa gctcagggaa aggaaagcta ggtcagtgaa 480
tggtcaggcc tgtctcttta gcatctgcct ctaacctgct aacaccacac agccctctca 540
agacacgggc gtcaaaagga acgcccacac gacaggctgc acccaaatgt gatgtccccc 600
tgtacacaca tgcagcacac agcccagcaa ggggaaggag catgtgcagt ggtcagaaag 660
gcttcatggg aaaggtggga tttgagccat tctagataat tctcaaaaaa ttacaggaag 720
tagatacaca gcaggttcaa atgcattaac accagagtgt tgagactgag agggaagcag 780
aggtttgtta ggattggtgg gaaacatggt caggaaaatc aggagcagac aatttgtgag 840
gtttctttaa agtcagactg aggacccaca gctcatgatc ccaacattgc tctctggcag 900
tgacaaatca caaagtgaag gctccaagga cttgagaaga cctactcagg gaagtggtga 960
agtaatgcac tggaggccct tgccctgccc ctctgtgtgc tttccctgga aggaaggagg 1020
gaaggttggt gaccaaatcc tctccaggaa tcatgtactg cataagttgt ttactttcag 1080
aagttggagt tccttttctt tttttgagac ggggtctctc tgttgcccag gctggagtgc 1140
agtggcatga ccctggctca ctgcagcctc tgcctccctg gttcaggtga ttctcgtgcc 1200
tcggcctccc gggtagctgg gattacaggc atgcgccacc accgctaatt tttggtggag 1260
ccggggtttc gccatgttga ccacgctggt cttgaactcc tggcctcaaa tgacctgccc 1320
accttggcct cccagcatcc tggagacttt gaataagctg gtggccaagc tggatgtggt 1380
ggctcacagt aattactctg taatcccagt actttgggag gccgaggtgg gcggatcacc 1440
tgaggtcagg agtttgagac cagccaagcc aacatggcaa aaccccgtct ctactaaaaa 1500
tacaaaaatt agcgcatgca gtggcatgca cttgtactcc cagctacttg gaggctgagg 1560
cagaacaatc acttgagcct gggaggcgga ggttgtagtg agccgggatc gcgccactgc 1620
actccagcct gggtgataga tcaagactcc gtctcgaaaa atagtaataa aataaataaa 1680
tgcatcacct ggccaatcat tctcaaaaac catagccatg gccgggtgca gtggctcacg 1740
cctgtaatcc caacacttgc actttgggag gccgaagcag gtggatcacg atgtcaggag 1800
ttcaagacca gcctggccaa gatggtgaaa ccccgtctct actaaacatt aaaaaattaa 1860

ctgggcgtgg ttcgtgggcg cctgtagtcc cagctactca ggaggctgag gcaggagaat 1920
cgcttgaacc ccgggggca gaggttgtgg tgagctgaga ttgtgccact gcactccagc 1980
ctgggtgaca gatcaagact ctgtctc 2007

<210> 57
<211> 2886
<212> DNA
<213> Homo sapiens

<400> 57
ttgaagaatg taaaataaca tgtggacaca tggaaagatg caccatgatt tatggtgggg 60
aggccacaca aaaatgccag ttcactcaaa gaattcccat tcgaatctga acagggtttt 120
tatttatttt ttattttttt gagacagggt ctcgctctgt cacccaggct tgagtgagtg 180
cagtggcacg atcatggctc acttgcttgg ccttgacttc ccaggctcaa gcaatcctct 240
tgtctcagcc tcgcaggtag ctgggactat acatatgcac catcagtggt ggaccctatt 300
ccagccagag gcgttttccc tagggtgtaa taagaatttc acaccctgtg gaagaactct 360
gtgtacccaa atcctagcag gtgcctgaga tacacacagc aggcactcag taaatgtacc 420
attgcccagc tagcaaagac tgacctaata agcagggcaa acactgtcaa aactctagta 480
taaagttgcc tctgtgtgct ttatcggaat cctcttgttt taatcctctc ctggtacgtc 540
tccatcccat cttagagtgt taagtgcaga gtcccttcgg aagcttaagt ctttgtggct 600
gccagagtca gaatgacgtg acgagcagag ggaggctttg gaattgaagt tttcattcac 660
ctctcatcag ctttaccagt ataagcctgg ttatttcatc cttcggagct ctatctcttc 720
tataaaatgg ggatcgtctt taaagccctg gagtgctgtt gtcaagatgg agataaagta 780
tgccaagctg ctagaagaat attcagtaac tttccggtat tttgcttcct gtctgtgaac 840
tcttcttatc aggaatccag attactcggg aaagcataag tagttcacat aatagaggtg 900
tggattattt tgaaaatgcc tcctgcgtgt tgagtttgca ttggagaacc ccgaagacgc 960
gcaaactagt cctagcgtga gtgagcagca agcagtggta cttccttgtg aatcggggtg 1020
ggaacccttc agaaccgcga gctcccactt tctgtcgatc gctctgctca tgcggccctg 1080
agtctcatgg ttactgtggt ctcagtcaca gcaagtgctg ccaagctcag gcgacgctgc 1140
cagagccttc tgagagccgg gcggggctgg tgccactggt agcctcatcc ctggacatgt 1200
ttttcaactt gaagggagag cagactggct gtgtgtttgc atttcctgac aaaagcagcc 1260
attgacgtca agagttccct ctgttgttaa gaagtgacac ctgtgtgttt ttctttagct 1320
ttgcacgtta ttgggcttca gataatattt attttacca actttggccc aaattgtatc 1380
attactttga atctgtacag cacttttatg ttttcaaaac atttcatgta tttttcagtg 1440
ttacccttgc aacagccttg tgagttaacc atttcaagtg caacctgttg tttcgtgagg 1500

aaactgaggc tcctctgcta tgcatgtgac cagcccagca tcacatggtc ttgcagcagt 1560
ggaactgaga tgagctcctg gcctcctgac tgcccagggc tccctcccac tgcattgtgg 1620
agcctgggca cttggggagg agtggggcag gacccagtcc tgccactgtt tgcttcctca 1680
ccacctctgg gtttcaggtc atgagaagca ttgccttttc ccctgggaac accagttgct 1740
tgctttcgag ttttataatg agagccaagg accttctgca ggcgtaattg ttcatctttt 1800
accaagcctt tgcatttcaa gtatccaaag acctacgtgg aatgttagta tggagagaga 1860
agaggaggga gggactggga cagtcatcct gccatccagg aaatgcctga gctcgacatc 1920
gcccaagcta gcctcaggct gctgcccctg gcctagcagg atgcgtgctt gcccttgttg 1980
ggatgctggc atccagctcc ttgttctggt ctcaggaggg agatgtcggg aaggttcaca 2040
gggtctgttt atagacccta gaggtggtcg ttggtggtgc ccagtgacgg agcctctatt 2100
ggcaaaacct gtatctctgc agtcgcagct gttggcccag tggatagagc agccttactt 2160
aaagcatttt cacttttctg tcctcatatg gcatgttcct cctccctctc tccctccctc 2220
cctctctccc tccctccctt tctccctccc tccctttctc cctccctccc tctctccctt 2280
gcttaattcc aagaaatggg gaccaacatt cttcttacaa ttagaagatt ctttgttgtt 2340
cataacagtg gagtcacagg tgtcccaagg tttttcccag gcagttcagc aggcctgaag 2400
gcttctggat ggagtgcaga aaatcacttg taagcacaaa gagtgtggca agtgatgtga 2460
ggtagctaac tggaatccac attgaggacc agctgcgtgc ttggaacagc agctccaagt 2520
gtcactgcat catcgttgag agttattgac ggtgaaaaca caaacatatt accgaacact 2580
accttgctag ctttgtggcc ctagcgaagt cacttcaatt ctctgcctca gtgtccatat 2640
ttgttaatga ggaaaataat agtacccacc tcacaggttt gtggtgaaga ttaaacactt 2700
agagaaaaca taaaatgcct cttgtgaagt aagtattttt tgaatattag ccatgattat 2760
gttattgaat caatgttatg tatcaagctg ttaccgaatt atatcggtgt tagccgttgc 2820
tgtcatcatc tccattatcc ttgtatttca gacatgtgag ctttggaaat taaaatcttt 2880
gaaaat 2886

<210> 58
<211> 2289
<212> DNA
<213> Homo sapiens

<400> 58
   ttctgtcttt tggaaacttc cagctggtca aagagattgc cgatgaagac cccagccacg 60
   tgaacttggt caatggggac ggggcgacgc cactgatgct agcagctgtt acggggcagc 120
   tggctctggt gcagctgctg gtggagaggc acgcggatgt tgacaagcag gacagcgtgc 180
   atggctggac ggccctcatg caggcaacct accatggaaa taaggaaatt gtgaaatatc 240
   tgctaaacca aggggccgat gtcactcttc gtgcaaaaaa tggatacacg gcctttgacc 300
   tggtgatgct gctgaatgat cccgacacgg aacttgttcg actgctggca tctgtctgca 360
   tgcaggtgaa taaagacaaa ggccggccga gccaccagcc tcccctgccc cactcgaagg 420
   tccgacagcc ctggagcatc ccagtgctgc ccgatgacaa gggtggactg aagtcctggt 480
   ggaaccgaat gtccaatcgg ttccgaaagc tcaaactgat gcagacgctg ccccgtgggc 540
   tgtccagcaa ccagcctttg cctttctctg acgagcctga gccagctctg gactccacaa 600
   tgagggctgc cccccaggac aagacaagcc gctctgcact ccctgatgcg gcccctgtga 660
   ccaaagacaa tggtcctggg agcacaagag gagaaaagga agacacgtta ttgacaacca 720
   tgcttcgaaa cggagctccc ctcaccagac tcccgagtga caagctgaaa gcagtcatcc 780
   ccccattcct acccccttcc agttttgagc tgtggagctc tgatcggtcc cggacgcgtc 840
   acaacgggaa ggcagacccc atgaagactg cgctgcccca gagagccagc aggggccacc 900
   ccgtgggcgg cgggggcaca gacactacac ccgtcaggcc tgttaaattt ccaagcctcc 960
   ccagaagccc agcctcttct gccaattctg gaaacttcaa ccactcgcct cattcatcgg 1020
   gcggctccag tgggataggt gtgagccggc acggtgggga gctgcttaac cgctcaggtg 1080
   gcagcataga caatgtcttg tcccaaatcg ctgcccagag gaaaaaagca gccggattat 1140
   tggagcagaa acccagccat cggtcaagcc ctgtggggcc agcaccgggg tccagcccgt 1200
   ctgagcttcc agcctcccct gcaggtggca gcgctcctgt tggcaagaaa ttggagacca 1260
   gcaaaaggcc tccatctgga acttccacta cctccaagag cacctctcca accctcacgc 1320
   cctccccctc acccaaaggg cacactgcag agtcctcagt gtcttcctcg tcatcccatc 1380
   ggcagtccaa gagcagtggg ggctccagca gtggcaccat cacagatgag gatgaactga 1440
   ctggaatcct taagaaatta tcacttgaga aatatcagcc catttttgag gaacaagagt 1500
   ctgtctctgt ctcgtgcgtg gtgagggaga tgaggtggac atggaagcgt tcctcacact 1560
   gactgacggt gacttgaagg agctgggaat taagacagat gggtccaggc agcagattct 1620
   ggcagcgatt tctgaactga acgcaggcaa gggacgcgag agacaaattt tacaggaaac 1680
   cattcacaac tttcactctt cctttgagag cagtgccagc aacaccaggg cccctggcaa 1740
   cagcccctcc atggttgggt gggtgaggcc agaagaaact gcctccggca agaggtagca 1800
   gccgctcagg tggctctgct ggcatcggag cccacagaag tgaggagtgg ccgatggacc 1860
   tgccctccaa atgtgcctga ctctgggtct tgctgtcact ggatttcctg gcatggcaga 1920
   cagaaagaaa gatagtttga ccaagtcgta gaagctgatc cagcgggtaa aaagggggca 1980
   gggaactcgt cccttttatt cttgcctcag agctgcctga agacatgggc caggccggag 2040
   gctggacaac tttggataac gctgacctgt acttccaagt aaatgcctcc tgaagagccc 2100
   gggacccttc ctgggagaat tctgcagcca gaatgaaggt gccatcagca ggaggcactg 2160
   tgaagcacca tcctgtcgct gtccttgtcc attcctagca agttaatcgt gtcttgttaa 2220
   ccagcagttc ctgttcaacg tgtaaagaga cctgatgttt tccctaataa agctgataac 2280
   agattttgc 2289

<210> 59
<211> 2404
<212> DNA
<213> Homo sapiens

<400> 59
accggaggcg cggccgccct gggctgagaa gacgccgtcg gcccaggtgg catcagaacc 60
cacaggagga agacagccag cccaggagga gccatggggg gcctctgggg atcgcaaggg 120
tcgaccccat cctccctgaa acaaggccgg aggttgagga ggtctccgcc cagccccgca 180
gctgacaacc ggtggagaaa ccgcacctgc cacgggaggg gccgcggggt gggctcgagc 240
caccatgggg ctggtacaag caccctctcc gctcaggacc tcggccctca tcccagggca 300
ccagggctca caggggggatg ggcccgggtg gggacgaggg tgtggagggg caggaagcac 360
ggcgggtgga gcggggccgg gtgcgggggt cgcgggggac cgcggggacg gggccttgtg 420
atgcaggaga cggagggggc tggtgacgga gaacgggagg ccgcaaggat gggggacgcg 480
acatcctttt cccctcccac cggctcacca gggatgcgcc tccgcgactg cccgccccaa 540
tcccagggcg gaggcctcgc gcaaaaccca ggcgccgcgg ctccgcgctc cggctgaggg 600
tccgcgccgc cgcccgcccg ctccttcccg ctcggccgcg gcctcaggga cggatactcc 660
agcgcgcggt tccaaccgag gcccatggcg ggtttagccc catgaagatg aactgggcac 720
ccgcgaatgg ctgggctctg ctacaggagg ccgccttcct ctgcgtgcgg ccgcggaggg 780
ctccgcgggg gctcgagagg ggcctcgagg gtgtgtgtga ggccggcgct gtgctatggt 840
gccccgggct cctccctgcg cgtggctgcg gccggggcgg cgggctcagg ctggagaagg 900
gccgcgggac ccagggaacg cggcctggag gacgaggagg actgggttcc cggaggcaac 960
aggaccggct cggcatctac agctactgta cgtggacttc gggcgctgga cccacgggcg 1020
gcctgtgcct ctgtgtcggg ctgggtggcg ccctcggtgc cctggggctg ctgcggccct 1080
aggcctcgct gggctcggtg accacatctg gcccccgggc cgcaccgtcc cccaggatcc 1140
tccggcctgg gctccccctt cctcccttgc ccacagtctt ggagccccag tgggtgcagg 1200
agctgctggc tgcccctgtg gacccgccat ccaccgtcct gcccatgctg cctcgccatc 1260
cacctgcctg cccacgccgc ctcagcctcc cacctcccac ctagaggaga ccatgggccc 1320
tgccccacct gctccaggat gttagggtcc cctcagccaa aaaggcagtg gcctgtggct 1380
cctgtaccaa cagcccagcc acgctccaga gccgcccaaa gggaggtgcc aaggccagga 1440
acccaagcca ccccagctcc ccacgcctgc ccaggggccg tggtaaccca tggacgggga 1500
ggtgacctca ggctggttct gccactgagg ccctgaggaa tcaggccctc cccaaaagaa 1560
gtaatgaaat ggaccaaagg acttaagaat ttggggggaa gtaaggggaa aacgttaggt 1620
gctaaccacc tgcccagaag agtggatctc acagcccagg aacattccca agcaggaaaa 1680
ccatccgtcc aggaacccta accctaaaac taagcacaac ccctacccct aacccctgac 1740

cctaacgctg acccttaacc ataaccctta accctaacca ctaacgcaaa acactaaccc 1800
ttaaccctaa cccctaacac ctaaccccca cctcaagcac taaaccctaa gcctaaacct 1860
taaccctaac tgctaacact aacccataac cctaagccct agccccaacc ctaaacttga 1920
ccctgaaccc aagcctccaa cccaaccccg acacaaaccc ctaaccctaa cccctaattc 1980
tagccctaat ccctaaatcc aacaccaacc cttgaccgga cccctgaccc ctaaccctaa 2040
cctcaactca taacctcaac ccctactcct acacctaacc cctaccctaa tgctagccat 2100
aaacctaaca cccaaaccca aactcgaacc tatgcctacc cctaaaaccc tagcccgaac 2160
cgtaaccaca aatcctaacc cacaacgcta cccctaaacc taccactacc ccaccccaaa 2220
ccctgacacc gaaccctgaa gctaacccta acaacactaa gcctaacccc taacccttaa 2280
cccaactgta actcctaatg ctaaccctaa cccttaactc taacccctta ccctaaactg 2340
taacccctaa ccctaatccc aaccccaaca cgtaacccta accccctcta ccccctctag 2400
agcc 2404

<210> 60
<211> 2716
<212> DNA
<213> Homo sapiens

<400> 60
ttctcctttt cctctgggct ggtgctcaga tggtgagagc actgtgctga tgaggtgggc 60
cctggggtgg aacccatggc cccctccgac tcccaggaga cctggccagc tgcagcggcc 120
cccgtggacc agcagaggga caagagagga gctgacgtgg aggagccgag gcccactggg 180
ggctcctgga gagtccgtcc gcctgtggac agcagctcag gtgttcagct gcttccagag 240
aggaagcacc tcttcctaga ctgaggcatc gttttgatta aaaaaaaaaa agttgatttt 300
tttcaccttg gtgtaaattt ttgtttgtaa caatatgtta gttgtcacag ttaccactat 360
ttgtttttct ttctttcttt tttttttttt tttttacatt tttgaaatta tttacagaga 420
tgggctctcg ctacgttgtc caggctggtc tcagactcct ggcctcagtg gtcctcccac 480
cttggcctcc caaagtgctg ggactacagg tgtgagccgt ggcacctggc ctgtttttct 540
ttattactac agatattatc tgaagcttta aatcctcctg gagtgaaagc tagttcataa 600
ccacgctgtt tggagcgaag aaaactacct ggaattcttg ttctccaaag atatggaatt 660
atcccagcaa cagccatcct tcgctgctct gtcctgagga ccctcaggtg tggcccatcc 720
agcccctcag tgtttacctc tgtcctaagg actctcaggt gtgggcggtc cagcccctta 780
gtgtttccct ctgttttgga gatcctcagg tatggtcaat cctattagtg ttttcctttg 840
tactggggat gctcaggtgt gatctatcca tcttctcagt gttttacctc tttcctggag 900
accctcaggt tccatccatc catccaattg gtttttacct ctgtcctgga gaccctgagg 960

tgtggtctat ccatcccatt agtgtttacc tctgtcctgg ggaccctcag ttgtggtcaa 1020
tccatcccat tagtgttttt ccattgtcct cgagactctc aggtgtagtc tatccgtccc 1080
attagtgttt acatctatcc tgggaacgct taggtgtggt ctatccattt ctttagtgtt 1140
tacctctgcc ttgggcccct cagatgtggt cgatccccaa agtgttttat ctctattctg 1200
gggaatctca gttgtggttc atccatccgc ttagtgttta cctctgtcct ggggaccctg 1260
aggtgtggcc catcccctta ttgtttacct ttgtcctggg ggccctcaga tatggcccat 1320
ccatcccctg agtgttttac ctcggtcctc gagaccctca ggtgtggtct attaatttag 1380
tgtttaccgc accctgggga ctcacaggtt tggtctatcc caatagtttt tttatgtcgg 1440
ccctgggaac tctcagatgt ggcccatcca tcccgtcagt gtttccctct gtcctggggt 1500
cactcaggtg tggtccatcc accccctcag tgtttccctc ggtcctgagg atgctcaggt 1560
gtggcccatc cagcccctca gtgttttcct cggtcctggg gatgctcaag tgtggtccat 1620
ccatcccctc agtgtttccc tcggtcctga ggatgctcag gtgtggccca tccagcccct 1680

cagtgttttc ctcggtcctg gggatgctca ggtgtggtcc atccagcccc tcagtgtttc 1740
cctcggtcct gaggatgctc aggtgtggcc catccctccc ctcagtgttt tcctcggtcc 1800
tggggatgct cagttgtggc ccatccatcc cctcagtgtt ttcctcggtc ctgaggatgc 1860
tcaggtgtgg cccatccatc ccctcagtgt tttcctcggt cctgaggatg ctcaggtgtg 1920
gcccatccat cccctcagtg ttttcctctg tcctggggat gcccatcccc ttagtgtttt 1980
acctccaact ttctctattt tttgtttcca gtcttcccac acaggttgag agaggagggg 2040
atccaattgc ctgtgaggag gacacggctc ctgggtggac cctgcagatt gtgaagttca 2100
agtcacagct cctgggaagg tctctgtgtg taaagatcat gggggtgaga cagattcagg 2160
gaccacactc tgctctgctc tgtacctctg agtgtcgatc cagctgcctt gtgaccagga 2220
cacttagaag aagcatggac cctgcaagag gagacagggt atcaccagtc tcctgggacc 2280
tccgcctccc gggatggtct ccatctcctg acctcgtgat ccgcctgcct ctgtctccca 2340
aagtgctggg attacaggcc tgagccaccg cgcccggcca gatgggctca aggacagctt 2400
gcctgactgc agccataagg gtgaagcctc ttcagggtct tactctgtca cccaggctag 2460
agtgcagtca gtgtgccact gtggctcaac ctccgtctcc tgggctcaat caatcgttcc 2520
acctcggcct cccaaagtgc tgggactaca ggcgtgagcc actgcgcccg gcctccccaa 2580
ggagcacttt tacagccaaa cccacctctc tcagctctca ctcctgctta tatccctgca 2640
acccaggaga ctgttttcta tttgtaaaat tttgccattt caagacagtt gtattaatga 2700
aatcactgtg tgtgtc 2716

<210> 61
<211> 1900
<212> DNA

<213> Homo sapiens

<400> 61
tgaaacagtg ctctaaatgg catctttgca attgattatg gacaattaag tacttagaag 60
aaggaatatc aagccaatca gaaattaaga gaaagctgat ttgaaattat gattgaaatg 120
ggatatgtat gagtatgtgt gctttaagtt ttttattatg tagcagaaaa agctaatatc 180
ttgagttgta gggactcatg tgggcacagg tttcccggga cgtcccgacc acctgaatgg 240
ccgggtgccc tgatttcagc tgaatgcccc tccccgcatc cttctccata ggcccgtcat 300
ggaagtgccc tgtgacaagc ccttctcgga ggagcaagct cgcctctacc tgcgggacgt 360
catcctgggc ctcgagtact gtgagtgcgg ggcagcttgc ccactggggc tggggctagg 420
ggatctggca ggcggcagag cccaggctga gcagactctg agcagctccc gtcagtcaga 480
gctgacctgc caatcagctt cagtgggagt ggggcatgca cgtgtggcgg ggccaaaggc 540
ctttttgtgg ggtggggcgg gcggtggact ccactgggca tgtgccagat ccttcgtcgt 600
gtctggtcct gtgggtctga gtcctggctg ttctgtatct ttcttctgct gagttcttag 660
cctagcttag cgttgccacg gggcttcaag agatgcggga aggaagggat ttatgtccag 720
ctgctgggga gagtctgtcc tggcatgggg ccggggcatg gtggcagggt ggatttacct 780
gtgaggggcc ctagtctgat aagagctcag gagggtgatg tgagcttggc ctctgtctca 840
tttcattcat tagctacatt cacttgcctg ggggcatagg ggtgaaagac ccagacccga 900
gttcacggcc tagtgggagg gacaggaatc taggcaggca gataatacag cgtggtgcct 960
gccaaggctg gggagcctag aggctgtagg agtgccgggg ggctggggaa gtctccctga 1020
agaggctact tatgattcgg gtcctgaggg atgagtagac ttccctgctc aggttttgag 1080
ggatgggcgt ggaagacgat gtgcctggca taggcgtgta ctctgagtct ggggagaagt 1140
ggagtctggc tgaagcctcc agtgggcaga ggagggccgt ggttagtgaa agatgatgct 1200
ggaaacactg tccgggccac agcatgaggg ctgggaatcc ctcccctgag gtctttgctg 1260
actgcatcct gccagctctg tgaggccctg agagctttaa gcatggggag gggcgtgatg 1320
ggatttgtgc ctgagaaagc tctgtctggc agctgtgtgg tggctggatt ggagtgtgtc 1380
atcggagggt gagaggcagc cagctggcca gggaggaggc tgtttctgca gcccaagtga 1440
cagatggtga ggcctggatt aaggcagtgg cagcaggatg gggataggaa ggaggtgggg 1500
tggtcagcat ggagtgactt gccggtctgg ggagaggaga gcccctagac acctagggtc 1560
ctggcgtggg ttggggacca ggggagatgc ccatctctaa aatcttagct tgggccaggc 1620
gcaggggctc atgcctgtaa tcccagcact ttgggaggcc gaggtgggta gatcacctga 1680
ggtcaggggt ttgagaccag cctggccaac gtggcaaaag cctgtctcta ctacaaatac 1740
aaaaattagc cttgtgtggt ggtgggcacc tgtaatccca gctactcggg aggctgaggc 1800
aggagaatcg cttgaacctg ggaggtggag gttgcagtga gccgagatca cgccattgca 1860
ctccagcctg ggtgacaaga gtgaaactcc atctcaaaat 1900

<210> 62
<211> 4081
<212> DNA
<213> Homo sapiens

<400> 62
atacatgctc acccatacac ctgctcacac acacatgctc acccataaac atgctcacac 60
acacacatgc tcatacacgt ttacccatac acatgctcac acgattacat acacctgctc 120
acacacacac gtgctcacaa attacataca catgctcaca catacatgct cacaaacacg 180
actacataca cctactcaca cacacatgct cacacaatta catacaccga ctcacgcaca 240
catgctcaca caattacata cacctgctca cccatacaca catgctcaca tgattacaca 300
tgctcccaca tacatacatg ctcacacaca ttacatacac atgctcacac acgctcacac 360
atacacacga gtacatacac gctgacacat gctcacacac acgattacat acacatgctc 420
acacatacac atgctcaccc acacacacgc tcacacatac actcacacac acctgctcac 480
acatacccat acatgctcac ccatacacat gctcacacac acatgctcac ccatacacat 540
gctcacacac atgctcaccc atacacatgc tcacacacac atgctcatac acgtttaccc 600
atacacatgc tcacacacga ttacatacac ctcccacata cacgattaca tgcacctgct 660
cacacacaca catgctcaca aattacatac acatgctcac acatacacat acatgctcac 720
aaacatgatt acatacacct actcacacac acatgctcac acaattacat acgcctactc 780
acacacacat gctcacacaa attacataca cctgctcata cacacacaca tgctcacacg 840
attacataca catgctccca catgctcacc tatacacatg ctcacataca cacgagtaca 900
tacacatgct gacacatgct cacacacacg attacataca catgctcaca cacattcaca 960
ttcacatgtt cacgcacaca tgctcacatg ctcacccata catacacacg attacataca 1020
catgctcata catacacgat tacatacaca tgctcacaca ggctcataca catgctctta 1080
cccatacatg ctcacacaca cacatgctca cacacaagca cacatgatca cacaggcaca 1140
catgatcaca catacacagg cttgcacaag tttacacaca tactcataca tgctcacaca 1200
ggcatacaca catgctcaca tatgcacaca ctctcacatg tataggcaca cacaggatta 1260
cacatgctca cacatgtgct tatacacata cacccatgca catgttcaca cgtttataca 1320
cacatatcac acacacaccc ccatatactc acacacacgc tgccacatgc tctgtctctc 1380
acacatacct gacagttccg tgctgtcccg tttccctcgg ggtcttccct tcggaggctg 1440
cagctcgtct gagcatccag ccttgaaggc actgagcagg caaggaggta gggcttttct 1500
ctggggaggc ctgcgttgca gtacggcttc ctcatcccca ccaagggcag ggaagggcag 1560
gagtctagga cttacctgaa tacagaacac ctgggagcgt ctgtctcgtg tcggccttgt 1620
gctgagcatg tgcagaccag gacttgcgcc ctaagccact tgttggcccc tccctcagaa 1680
tcatgtgcag tggcttggcc tcacccacag acaagggagg cggtaagaaa tgccataaag 1740

agacgctgag cctgaaggac agcaccttac acctgtgcag tccacacttg cctttccaat 1800
tcgccgttgc atttcatcct cacgtccctt gacaccaagg cccagaagcg gagaaggcgc 1860
acgtcgcata atgctgagtt ggaagggagt ttgcttttca tctcttgtta ctgccccgtg 1920
aacaactttc caagtgacac attttgtaaa aggagagaac ctgcatggaa ctggctgctc 1980
tggggctgtc tccctgcccg aggctgcttc atgctgagtt actgccagga ctcctaactg 2040
tcttctctct gcgctttctc caggattacg gtggctacct gagcacctac atcctcccag 2100
caaagggaga aaatcaaggc cagacattca cctgcggctc tgctctctct ccaataacag 2160
acttcaaact ctatgcctct gcgttttccg agaggtactt gggcctccat ggacttgaca 2220
acagagcata cgagatgacc aaggtagccc atcgagtctc cgcgctggaa gaacagcagt 2280
tcctgatcat tcatcccact gccgatgaaa aaattcattt ccagcacaca gcagaactca 2340
ttacacaact aattagggga aaggctaatt acagcttaca ggtacagtac gcatgttact 2400
ctgttttgaa cctggagcaa gacattcctt tcatggagaa agacctgacg ggtgttcagg 2460
gccttctact gcagcagaca cgcctgtgct gtggtggtcg gtgttgagca aggtcccagc 2520
aggttggaaa atcagcacat gctcaacgtt ttcttataat taatgatagc tccgctctgt 2580
acttcagccc ggtttaaatc cctttaggac aaccatctcc ccgtactccc accccagccc 2640
tgctcccctc ctccgggcag tgagaagaac caagccagca cccaccctgc ccagcagtgt 2700
ggctggggct atcccgctgc ggagagtggg actgttgttc tgggttggtg agagtggtgc 2760
tggtgggccc tgagccttgc cccactgctt ccctctgacc aacaccacat gttactacgc 2820
actagcagga tgtcctgggg ccatctgtag ctaggcagta gtgaaagagg ggtacccttc 2880
ccagaactga actcactttc ccacaaccaa cccgtggccc gtccacaaca gcatcaagtg 2940
ctgacccagc agcctcaaac ctgggctctt cccagtaatg cacagggatg tctctggaaa 3000
cttctgccat ttccttcctg ccttctgctc ctttggtgga acaggtatga cccaatggtc 3060
tcttcaggtc caacatgaat gaatgggaga gctccttggg gacagatgac aggcaggcac 3120
cctgcctccc tcagagagct cttgccctgg gctgatcact gccttggcct ccagcatccc 3180
tgcctgtggg agggagggac cacctgtggg cttgatcagg cagggagagc tccagccaga 3240
agggagttgg accccagtgt ggatgaagag gggcagaagg acggggttac accacacttg 3300
agttcccttg tgaaagacgt tcagatttca gactttttca aagtaagtgt ggcagttgca 3360
tggcagtgtt aagcacactg accatggacg ctccaaaggt ccatgtgtat gtgttcatgt 3420
gggcaactcc ccaagatatg gggcaagctg aaccccccag gtacagagca gcctgcatcg 3480
ctgcaggctc catgggtatc agggagcagg acccacccag ggacacacat gtgggcacag 3540
caaaggcctt cacagacacc ccatgcatct ggagccaggc ccagccccag gtaagagcac 3600
gggagacggg ggcctgaggc aggaggagaa agcttcctgc ccatatcctc ttgtactact 3660
tgatgtttta atgacatatc atagcctaat gacagcatta ataaatactt caaaataaag 3720
atgaatttca ccctccaaaa acatacaaag aaggcaggaa gaggcagtct agtgtcatag 3780
aaagatgtag gttttgggcc agatgcagtg gctcacacct gtagtccctg cactttggga 3840
ggctgaggtg cggggattgc cttgagctca ggagtttgag accagcctgg gcaacagagc 3900

gagaccccat ctctacaaaa agtttttaga aaatagccgg acgtagtcat gtgtgcctgt 3960
ggtcccagct gcacgggagg ctgaggtggg agagtcactt gagcccagga ggttgaggct 4020
gcagtgagct atggtaacac catggcattc cagcctgggc gacagagtga gaccctgtct 4080
g 4081

<210> 63
<211> 3600
<212> DNA
<213> Homo sapiens

<400> 63
ctgaggactt cgtggggctg cacatgcatg ggttccggcg caccctgcgg aatgcagtcc 60
tcacccagaa gcaggacagc ctgcgcatca tcagcatcca gcccgtggca ggcaccaacc 120
aactggacat gctgtttgcg gtggagatgc acagcagcga gttctacaag ccagcctacc 180
tgatccagaa gctgtccaat gctagaagac acctggagaa tatcatgcgc atctcagcca 240
tcttggagaa gaactgctca gggctggact gtcaggaaca gcattgtgag caaggcttgt 300
cactcgattc ccacgcgctc atgacctaca gcacggctcg catcagcttt gtgtgtccgc 360
gtttctacag gaacgtgcgt tgcacctgca atggaggact gtgtccgggg tccaacgatc 420
cttgtgtgga gaagccgtgt ccaggggaca tgcagtgtgt cggttatgaa gccagcagga 480
gaccgttcct ctgccagtgt ccaccaggga agctcggaga gtgctcaggg cacacttctc 540
tcagctttgc tggaaacagt tacatcaaat atcggctttc tgaaaatagc aaagaagagg 600
atttcaaact agctctgcgt cttcgaacac tgcaaagcaa tgggattata atgtacacca 660
gagcaaatcc ctgcataatt ctgaagattg tggatggcaa gctgtggttc cagctggact 720
gcggcagcgg ccctggaatc ttgggcatct cgggccgtgc tgtcaacgac gggagctggc 780
actcggtctt cctggagctc aaccgcaact tcacgagcct gtccctggat gacagctacg 840
tggagcggcg ccgggcgccc ctctacttcc agacgctgag cactgagagt agcatctact 900
tcggcgccct ggtgcaagcg gataacatcc gcagcctgac tgacacgcgg gtcacgcagg 960
tgctcagcgg cttccagggc tgcctggact cggtgatact gaataacaat gagctgccgc 1020
tgcagaacaa gcgcagcagc ttcgcggagg tggtgggcct gacggagctg aagctgggct 1080
gcgtgctcta tcccgacgcc tgcaagcgca gcccgtgcca gcacgggggc agctgcactg 1140
gcctgccatc gggggctat cagtgtacct gtctctcaca gtttacgggg agaaactgtg 1200
aatctgagat tacagcctgc ttcccaaacc cctgccggaa tggaggatcc tgcgatccaa 1260
taggaaacac tttcatctgc aattgtaaag ctgggctcac tggagtcacg tgtgaggagg 1320
acatcaatga gtgcgaacga gaggagtgtg agaacggagg ctcctgcgtg aacgtgttcg 1380
gctccttcct ctgcaactgc acgccgggct acgtgggcca gtactgcggg ctgcgccccg 1440

tggtggtacc caatatccag gctggccact cctacgtggg gaaggaggag ctcatcggca 1500
tcgccgtggt cctcttcgtc atcttcatcc tggtggttct cttcatagtc ttccgcaaga 1560
aggtcttccg caagaactac tcccgcaaca acatcacgct agtgcaggac ccggccaccg 1620
ccgccctgct taacaagagc aatggcatcc cgttccggaa cctgcgcggc agtggggacg 1680
gccgcaacgt ctaccaggag gtggggcccc cgcaggtccc cgtgcgcccc atggcctaca 1740
caccctgctt ccagagtgac tccaggagca acctggataa gatcgtggac gggctgggag 1800
gcgagcacca ggaaatgacc acgtttcacc ctgagtcgcc ccgcatcctg acagcccggc 1860
ggggcgtggt cgtgtgcagt gtggccccca acctccccgc cgtgtcaccc tgccgctccg 1920
actgcgactc catccggaag aatggctggg acgcgggaac tgagaacaaa ggggttgatg 1980
acccgggaga agtgacctgc tttgcaggta gtaataaagg cagcaactct gaagttcagt 2040
ccctcagctc cttccagtca gattctggtg acgacaatgc ctatcactgg gacacctctg 2100
attggatgcc aggggcccgc ctgtcggaca tagaggaagt gcccaactat gagaaccagg 2160
atggagggtc tgcacaccag gggagcacac gggagctgga gagcgattac tacctgggtg 2220
gttatgacat tgacagtgaa tacccacccc ctcatgaaga ggagttcttg agtcaggacc 2280
agctgcctcc tcctctccca gaggacttcc cagaccaata tgaggccctg ccaccctccc 2340
agcctgtctc cctggccagc acactgagcc cagactgcag gagaaggccc cagtttcatc 2400
ctagccagta tctccctcct cacccattcc ccaacgaaac ggatttggtg ggcccgcctg 2460
ccagctgtga atttagtact tttgctgtga gcatgaacca gggcacagag cccacaggcc 2520
cagcagacag cgtgtctctg tccttgcaca attccagagg cacctcatcc tcggatgtgt 2580
ctgccaactg cggctttgac gattccgaag tagccatgag tgactacgag agcgtgggag 2640
agctcagcct cgccagcctt cacattccct ttgtggaggc tcagcatcag actcaagtgt 2700
agacatcaca tcttgggtac ttcaccctgt ttgttacaga aaagtggaag cagattggct 2760
gggcttctgt cccagtggag cattgtctgt ggaatgagaa gggaatactg tatttttca 2820
ctagaaactt cttcacaagt catactgtcc caacaagcaa gcttgattcc agttgggtga 2880
aaatgaaagg ctcagaaatt gtttttgaga ggtgactggt aatccttgat gtaggtacct 2940
atgttcacag ctaaaaatgc aaagagggaa aaattatttc acccactaag ttatacagcc 3000

agtcttgtat ggctttgtgc agtattgtgc cctggaaagt gctacagcat cagtccttgc 3060
agtattaaaa actggcaaca atcaaagagg cattgttgca tgtaattttg agccaatgaa 3120
atgaaaatag tagtaatgat tgttggaaaa gttagtctct taggcgaaag agaagagaaa 3180
caaatattat taaacaaacc agaaaatggg ctgaagcctt ttaaatcaac tctatttttt 3240
tgataagctg cccaattttc agctataaaa ttaggcttga ataacatgtt tagtatgctc 3300
agttatttct gtttgtttgt gttaagcatc caatctaata tagttgggtt ttatgatctt 3360
caagaaaggt atcaatgaag agcaacatga ggctttttgg gttccatttg gtgggtgggg 3420
gaggaagtta aagttgtttg aacattagaa agaatgtgat tatctggttg gttttgtgtt 3480
ttctggtaaa tattccagtt ggtaaatcta acattgctac agaagttggc tttgttcata 3540

tagcttctct acaattagat atttttagaa gtttaagcaa aactcacaaa ttcagggggg 3600

<210> 64
<211> 4387
<212> DNA
<213> Homo sapiens

<400> 64
aaactttgac tcaagatcat tccaatacag tgagatatag aaatgcacaa agagaagaca 60
gtgaaataaa gatgattcag gaaaaaaagg agcaagcaga gatgaaaagg aaagtgcaag 120
aagaggaact cagagagaac cacccatact tcgataagcc actgttcatt gtcgggcgag 180
aacacaggtt cagaaacttt tgccgggtgg tggtccgagc acgcttcaac gcatctaaaa 240
cagaccctgt cacaggagct gtgaaaaata caaagtacca tcaactttat gatttgctgg 300
gattggtcac ttacctggac tgggtcatga tcatcgtaac catctgctct tgcatttcca 360
tgatgtttga gtccccgttt cgaagagtca tgcatgcacc tactttgcag attgctgagt 420
atgtgtttgt gatattcatg agcattgagc ttaatctgaa gattatggca gatggcttat 480
ttttcactcc aactgctgtc atcagggact tcggtggagt aatggacata tttatatatc 540
ttgtgagctt gatatttctt tgttggatgc ctcaaaatgt acctgctgaa tcgggagctc 600
agcttctaat ggtccttcgg tgcctgagac ctctgcgcat attcaaactg gtgccccaga 660
tgaggaaagt tgttcgagaa cttttcagcg gcttcaagga aatttttttg gtctccattc 720
ttttgctgac attaatgctc gtttttgcaa gctttggagt tcagcttttt gctggaaaac 780
tggccaagtg caatgatccc aacattatta gaagggaaga ttgcaatggc atattcagaa 840
ttaatgtcag tgtgtcaaag aatttaaatt taaaattgag gcctggagag aaaaaacctg 900
gattttgggt gccccgtgtt tgggcgaatc ctcggaactt taatttcgac aatgtgggaa 960
acgctatgct ggcgttgttt gaagttctct ccttgaaagg ctgggtggaa gtgagagatg 1020
ttattattca tcgtgtgggg ccgatccatg gaatctatat tcatgttttt gtattcctgg 1080
gttgcatgat tggactgacc ctttttgttg gagtagttat tgctaatttc aatgaaaaca 1140
aggggacggc tttgctgacc gtcgatcaga gaagatggga agacctgaag agccgactga 1200
agatcgcaca gcctcttcat ctcccgcctc gcccggataa tgatggtttt agagctaaaa 1260
tgtatgacat aacccagcat ccatttttta agaggacaat cgcattactc gtcctggccc 1320
agtcggtgtt gctctctgtc aagtgggacg tcgaggaccc ggtgaccgta cctttggcaa 1380
caatgtcagt tgttttcacc ttcatctttg ttctggaggt taccatgaag atcatagcaa 1440
tgtcgcctgc tggcttctgg caaagcagaa gaaaccgata cgatctcctg gtgacgtcgc 1500
ttggcgttgt atgggtggtg cttcactttg ccctcctgaa tgcatatact tacatgatgg 1560
gcgcttgtgt gattgtattt aggtttttct ccatctgtgg aaaacatgta acgctaaaga 1620

tgctcctctt gacagtggtc gtcagcatgt acaagagctt ctttatcata gtaggcatgt 1680
ttctcttgct gctgtgttac gcttttgctg gagttgtttt atttggtact gtgaaatatg 1740
gggagaatat taacaggcat gcaaattttt cttcggctgg aaaagctatt accgtactgt 1800
tccgaattgt cacaggtgaa gactggaaca agattatgcg tgactgtatg gttcagcctc 1860
cgttttgtac tccagatgaa tttacatact gggcaacaga ctgtggaaat tatgctgggg 1920
cacttatgta tttctgttca ttttatgtca tcattgccta catcatgcta aatctgcttg 1980
tagccataat tgtggagaat ttctccttgt tttattccac tgaggaggac cagcttttaa 2040
gttacaatga tcttcgccac tttcaaatca tatggaacat ggtggatgat aaaagagagg 2100
gggtgatccc cacgttccgc gtcaagttcc tgctgcggct actgcgtggg aggctggagg 2160
tggacctgga caaggacaag ctcctgttta agcacatgtg ctacgaaatg gagaggctcc 2220
acaatggcgg cgacgtcacc ttccatgatg tcctgagcat gctttcatac cggtccgtgg 2280
acatccggaa gagcttgcag ctggaggaac tcctggcgag ggagcagctg gagtacacca 2340
tagaggagga ggtggccaag cagaccatcc gcatgtggct caagaagtgc ctgaagcgca 2400
tcagagctaa acagcagcag tcgtgcagta tcatccacag cctgagagag agtcagcagc 2460
aagagctgag ccggtttctg aacccgccca gcatcgagac cacccagccc agtgaggaca 2520
cgaatgccaa cagtcaggac aacagcatgc aacctgagac aagcagccag cagcagctcc 2580
tgagccccac gctgtcggat cgaggaggaa gtcggcaaga tgcagccgac gcagggaaac 2640
cccagaggaa atttgggcag tggcgtctgc cctcagcccc aaaaccaata agccattcag 2700
tgtcctcagt caacttacgg tttggaggaa ggacaaccat gaaatctgtc gtgtgcaaaa 2760
tgaaccccat gactgacgcg gcttcctgcg gttctgaagt taagaagtgg tggacccggc 2820
agctgactgt ggagagcgac gaaagtgggg atgaccttct ggatatttag gtggatgtca 2880
atgtagatga atttctagtg gtggaaaccg ttttctaata atgtccttga ttgtccagtg 2940
agcaatctgt aattgatcta taactgaatt ccagcttgtc acaagatgtt tataaattga 3000
ttttcatcct gccacagaaa ggcataagct gcatgtatga tgggttacta tcaatcattg 3060
ctcaaaaaaa tttttgtata atgacagtac tgataatatt agaaatgata ccgcaagcaa 3120
atgtatatca cttaaaaatg tcatatattc tgtctgcgta aactaaggta tatattcata 3180
tgtgctctaa tgcagtatta tcaccgcccc gcaaaagagt gctaagccca aagtggctga 3240
tatttagggt acaggggtta tagctttagt tcacatcttt cccatttcca ctagaaatat 3300
ttctcttgag agaatttatt atttatgatt gatctgaaag ggtcagcact gaacttatgc 3360
taaaatgata gtagttttac aaactacaga ttctgaattt taaaaagtat cttctttttc 3420
tcgtgttata tttttaaata tacacaagac atttggtgac cagaacaagt tgatttctgt 3480
cctcagttat gttaatgaaa ctgttgcctc cttctaagaa aattgtgtgt gcaagcacca 3540
ggcaaagaaa tggactcagg atgcttagcg gtttaaaaca aacctgtaga taaatcactt 3600
gagtgacata gttgcgcaaa gatgttaagt ttcttaagaa accttttaat aactgagttt 3660
agcaaaaaga ataaaactat atagctcaat ttatttaaaa aaatctttgc atgtgtgatg 3720
ttatcattgg cttcatttct tacccaaggt atgtctgttt tgccataaat cagcagagtc 3780

atttcattct gggtgatcct aacacaccat tgctacgtta gatttgaaat gacatctctg 3840
ttaaaagaat cttctatgga aataatggtg ccctgcaaaa tcttcctttg aactcacagg 3900
ttagggatca cacaacttac ttaatcgttt tttgtttttg ttttttttcc ttatatgtca 3960
atggcccatg tcctccggga aaattagaaa agcaaaatga ttacaaagtg ctgttagatt 4020
tcttgtgctg ggccagccaa gtagaagtgg acttgacttg gacctttaac tattttatta 4080
cagattggac atttgctgtt cagatgtttt ttaacagagg gattatctca gaatcctgtg 4140
acctccaggt tgttttataa tctatttttc tctatttaac attcctcaga tagataggca 4200
aataggacat tccttctgtg tcacagaagt atcgtggtag tggcagtcta cagtttatat 4260
gattcattgt aactatgaga taaagaacaa ccagtcatgt ggccaaaagg attagatttg 4320
atttgatgtt cacttggagt ttactttttg tacatacaag ataaaataaa tattggattt 4380
gtaaaat 4387

<210> 65
<211> 3593
<212> DNA
<213> Homo sapiens

<400> 65
gggcctcggt ggcgaggtgg tcgttctggc aaaggcgcac actgactttg acgtccaggc 60
cttccgggtc accttttttg atgaaaccag gtgatgcagc cgcccatcca cgatgttggg 120
agagggtctg ggcccggagt gggggccgag gcccgagcac ctccccctgg cttggctgtg 180
cgtctctgcg tctcccggtg ggtcagggcg ctgcgagctg cggcagtgct cagtcctcat 240
tccttgcagg atggcgtcgt gcgggcaggg cagtgtgcgg ctctggcggc tgcgtggcgg 300
ggtgctgcgt tcctgccccg tggacttagg ggagcaccac gcgctgcagt tcaccgacct 360
ggccttcaag caggcccggg acggctgccc ggagccctcg gctgccatgc tcttcgtgtg 420
cagccgcagt ggccacatct tggagattga ctgtcagcgc atggtcgtgc ggcatgcccg 480
ccgcctgctc cccacacgga ctccaggcgg tccccaccca cagaagcaga ccttcagctc 540
aggccccggc attgccatca gcagcctcag cgtctccccg gccatgtgtg ctgtgggctc 600
tgaggacggc ttcttgcggc tctggcccct ggacttctcc tcggtgctcc tggaggcaga 660
gcacgagggc cccgtcagct cagtctgtgt cagccccgat ggcctccgtg tgctgtctgc 720
cacctcctcg ggccacctgg gcttcctgga cacgctgtcc cgggtgtacc acatgctggc 780
tcgctcccac accgccccgg tgttggccct cgccatggag cagaggcggg gacagctggc 840
caccgtgtcc caggaccgta ccgtccgcat ctgggacctg gccaccctgc agcagctata 900
cgacttcaca tcatcagagg acgccccgtg cgctgtcacc ttccacccca caaggccaac 960
ctttttctgt ggctttagca gtggggccgt gcgctccttc agcctggagg ccgctgaggt 1020

cctggtggaa cacacgtgcc accgaggagc tgtcaccggc ctgaccgcca cccctgacgg 1080
ccgcctgctc ttcagctcct gctcccaggg ctccctggcc cagtacagct gtgcggaccc 1140
ccagtggcat gtcctccgag tggcagcgga catggtatgc ccggatgccc ccgcgagccc 1200
cagcgccctg gcagtcagca gggatggccg cctgctggcc tttgtgggac cctccaggtg 1260
cacagtgaca gtcatgggct cggcctccct tgatgagctg ctgcgagttg acatcggcac 1320
tctggacctg gccagcagcc gcctggactc agccatggct gtgtgctttg gccctgcagc 1380
tctgggccac ctgctggtgt ccacctcgtc caacagagtc gtggtgctgg atgctgtgtc 1440
gggccgcatc atccgggagc tgcccggtgt ccaccctgag ccctgcccct ccttgacgct 1500
cagtgaggac gcccgcttcc tgctgattgc cgccggccgg accatcaagg tgtgggacta 1560
cgccacacag gccagcccag gcccccaggt gtacatcggc cactcggaac ccgtgcaggc 1620
tgtggccttc tctcctgacc agcagcaggt cctcagcgca ggggacgccg tcttcctctg 1680
ggatgtcctg gcccctactg agagcgacca aagcttcccc ggggcccccc cagcctgcaa 1740
gacaggcccg ggcgcaggac cgctggagga cgcagcgtcc agggccagcg agctcccccg 1800
gcagcaggtc cccaagccat gtcaggcatc tccaccacgg ctgggcgtct gtgccaggcc 1860
tcccgaaggt ggcgatggcg ccagggacac caggaattcg ggggccccac gcaccaccta 1920
cctggcttcc tgcaaggcct tcacgcctgc cagggtcagc tgcagccccc actctgccaa 1980
gggcacttgc ccgcctcccg ccagcggtgg gtggctgcgt ctgaaggctg tcgtcggtta 2040
cagcgggaat gggcggccaa acatggtctg gaggccggac acaggcttct ttgcctacac 2100
gtgcggccgc ctggtggtgg tggaggacct gcactctggc gcccagcagc actggtccgg 2160
ccactctgcg gagatctcca cgctggccct cagccacagt gcccaggtcc tggcctctgc 2220
ctcgggccga agcagcacga ccgcccattg tcagatccgc gtctgggacg tgtctggcgg 2280
cctctgccag catctcattt tcccccatag caccaccgtg ctggccctgg ccttctcacc 2340
agatgacagg cttcttgtca cactggggga ccacgatggc cgcaccctcg ccctgtgggg 2400
cacggccacc tatgacctcg tgtcctccac ccgcctcccg gagccggtgc atggtgtggc 2460
cttcaacccc tgggacgccg gcgagctcac ctgtgtgggc cagggcactg tcaccttctg 2520
gctccttcag cagcgtgggg cagacatcag ccttcaggtg cgtcgagagc cagtcccaga 2580
ggcagtgggg gctggagagc tgacctcgct ctgctacggg gcacctcccc tgctctattg 2640
tggcaccagc tctggccagg tctgtgtctg ggacacgcgt gccggccgct gcttcttgtc 2700
ctgggaggcg gatgacggtg gcattgggct gttgctgttc tcgggttctc gattggtcag 2760
cggcagcagc acggggcggc tgcgcctgtg ggccgtgggg gctgtgtcgg agctgaggtg 2820
caaggctcag ggcgccagtt ctgtgttcat ggaacacgag ctggtgctgg acggggctgt 2880
ggtgagtgcc agcttcgatg acagcgtgga catgggcgtc gtgggcacca cggcgggcac 2940
gctgtggttt gtcagctggg ccgagggcac cagcacacgt ctcatcagtg gccacaggag 3000
caaggtgagg gacttccagc ctgggcagag gcggggcagc cgaacctggt gccctccctg 3060
cctgccggct ccatctccac cagcccagat gattccaagt cctgccgtca ctggctcgca 3120
gcggccgcct tggggttccc agcggggaag tcttgggtgt gcacgtcccc tcaaagccgt 3180

cccggttgtg tctgcacaag cgagccgcct ggcaggcctt gcaggtcttc tcaaactgtc 3240
ctttccctgc tattgctttg cgtttttttg tttgtttgtt ttttgttttt tgtaattgtc 3300
aaagaaatca catcagtccg ggcgtggtgg ttcatgcctg tgatcccagc actctgggag 3360
gccaaggcag gtggatcacc tggggtcagg agttcgagac cagcctggtc ggcatggcga 3420
agtcccgtct ctgctgaaaa tacaaaaatt agccgggcct cttgatgtat acctgtagtc 3480
ccagctactc tggaggctga gacaagagag tcgcttgaac ccgggaggcg gaggttgcag 3540
tgagccagga ttgcgccgct gcgctccagc ctgggcgaca gagactccat ctc 3593

<210> 66
<211> 4596
<212> DNA
<213> Homo sapiens

<400> 66
   agcggcgagg acccgggtct ggcgctgtgg gccgggagcc gtggggcggc atggaggggc 60
   tggttgtcgc cgccggcggg gacgtctccc tgcacaactt cagcgcgagg ctgtgggagc 120
   agctggtcca cttccacgtc atgcggctga cggactcgct gttcctgtgg gtgggggcca 180
   cgccgcacct gcgcaacctc gccgtggcca tgtgcagccg ctacgactcc atccccgtgt 240
   ctacctccct ccttggagac acttccgaca cgacctctac tggccttgcc cagcgcctag 300
   gtatgtaccc acagctggcg ctgcatggcc agccaggtgg ggcccactct ttaatggaac 360
   catgaagcaa gtcccttcgg aagtaaagca tctttatgct aagaaccgtc acctcattgc 420
   tgctgggaag cacagacctt tgcacgttgg gtcttattta aggggcacat tccatgctcg 480
   cctgtcatca ccaccgtccc agggctggcc tgttcccaaa ggactcccac ctcgtccttg 540
   ggtgtgtcct tgagtccatg agctgattta gttgccttcc gtaagtgcat cacctcctcc 600
   tgggaggaga gcatgagaag tgcccacagc ccctgtgggt ggtggctcaa ggtagcctcc 660
   cgggccttag gaggagtcag tgcagctggt ggagcaggtg tcacctctgt gcaggaagga 720
   ctgtcctcac tcagtgtgat accgttcagg gctcggaggg aagactggcc tggcctgtga 780
   gtgtggcgta gagcggggat taagcaaacg tcatgaggct gaatgctcca cttctcccac 840
   accccaacac acttcctgtg ggccaggtaa ggcttccatg tgctctgcaa cccccagccc 900
   cagtgcctgt ggccagtgtg cacaggatgc ctgtctcctg cccaggcctg gcgttctcca 960
   gtaggcccag ctgcttccgc tctggagtct tctctgtgtc tcaggcacag gacctggtac 1020
   ctgacaagca ggaacctctg tgtcagtcac actgaggcaa atgcacacga atagcatggc 1080
   acgtggccca gggggaaggc agggcagggt ggagtgtcac cagcaggccc atgacagtat 1140
   gtggatagga caggtcaggg agggcacagg tcagcatttc aggaactcgc agtattcttt 1200
   ttccctccct tggaggtggg gacctgactt gtaagttaat ttggtaattt ttaactagga 1260
   agtaaattta aaatttacag aaaagttgca aagaagatag aatttctgct tagcttttgc 1320
   cccaatttcc cacttgccac ccttccctct ttgtgtttgt atcttttttt ttctgagcca 1380
   catgaaagta aagccgcctt ttgtgtctgt gtcactctgt gtgtgtgact gtgtaagggg 1440
   tagcacactt acatggaaca ggttactgcg tgtgggagaa ggctcaggaa ggtattcata 1500
   tttatacaga cggaaagctg ggtggggttg cggacacact cccatagaga aggtggtgtg 1560
   agcttgtctg gcaggccgtg aataactttc atatcaataa ggtaaattag tctccagaat 1620
   atttgcgaga tggaggccta gtatctatat gcaaaaaaga ggggttccca gtatctcgac 1680
   tgtctcgaca agtcacgggt gtgccgtggc ttccagcgtc tcttaaagga ctggtgctgc 1740
   agatctcgga ccaagaaggt tttccagcaa agtcccatgc tttgccagtt gccttgagcc 1800
   tgggtctggg ccctcaagtt gaatattaat gcactataat gaaaagggag aggtgtcctg 1860
   aatcagtatc ttatatgaga gatgaaagca ggtagggaaa gagaaggcct ggggggttct 1920
   cagtagctct ggaagcatcg caggagttgg tgtggccaag gggacccaac ctcctatggc 1980
   cctggagggg cacagcttgg gtctgagggg ctcagtgcct ccagacacag cgttgttcct 2040
   cagaaatcct gtctctgcag ttagaacagt tggaatacca ggggggcccc cttggggtaa 2100
   agggggatgt tcctgcccct gggatatagg gcagggtgtg acttacaggc tcctcctagg 2160
   cagccttggt gagccccagc ttcttaccac actgcctgct ttagatcacc ctctacatga 2220
   gtggtcccag cctgcctaac acagctagga aaggatgagg ttggtcccta atggtggggg 2280
   aagcctaaga ggcttcctca ctccccgcag ggctgagcag ataactgggc acaactctcc 2340
   gcctcagggg cacactgcac ccagcacact ggtgggaggc tctgtgctaa gtactgtaag 2400
   ctgttcttta aacaccagct cttggaataa ccgaacagct gcctttgtcc agacacattc 2460
   cttttttttt tttaataaaa aaacagcttt accgagacgt aaacacatgt catacaattc 2520
   agcagctgtc ctcaggcaga ggctgtgctt gccgccgcct tgcttttcca taggtgcgtt 2580
   ggctgcctcg taaggacagt cagtgagtta atacccaaga cctttccagc cctaaaaatc 2640
   acgcagcctt gtacagcgga aaaacaggct tttggggtcc tgggctctcc ctttcctcaa 2700
   tttccaaaca aagctgggag aggtttccag gccacggtgg ggactgggcc tgctcctgca 2760
   cctgtgcccc tgagccacac tgcctctcag ggttcttggt gggaccatag ctcagatggc 2820
   agcaactgaa aggacagaac tgctgcccag atgagatccc atgatgtgtg ttctaaatcg 2880
   gcctttagta atggtgcagg tggcctggtg ccctcagcct caccgcacca cccttttgcc 2940
   ttttggtgat gaggctaagt acatgcttgc catatgttga ggtgggggcg ggtcagtgcc 3000
   tctccagggg tgagtagttc tgcactggcc ctgcgtatgg gtgaggctaa aggaggaggc 3060
   cgcacagtgg ggcaggcaac atccaactgc tttagccttt ttgcccacgt attttcagga 3120
   tattggcttt cagattgcag gtatatgact ctgcttgatt tttgcagttg gcccgatgtg 3180
   cacctcagcc attttctatt gattctgtag aaagggactc aaacttacat tcagtagaaa 3240
   attacgtgtg tgtccatatg cctatctgtg cgcacacaca cacacacaaa tatatttgtg 3300
   tgtatacaca tatatgcaaa tgcatggaaa tgtaggaggg agggaagtgg aactggttgg 3360
   ggtggggtgc tgaaggagaa ctttaatatt ttattttaaa tactttatta tttttttttt 3420
   gagacggagt cttgctctat cgcccaggct ggagtgcagt ggcgcgatct cggctcactg 3480
   caagctccgc ctcccgggtt cacgccattc tcctgcctca gcctcctgag tagctgggac 3540
   tacaggcgcc cgccaccacg cccggctaat tttttgtatt tttagtagag acggggtttc 3600
   aacgtgttag ccaggatggt ctcgatctcc tgaccttatg atccacccgt ctcggcctcc 3660
   caaagtgctg ggattatggg cgtgagccac cgctcccggc cattttttgt gtttaaattt 3720
   tttttttttt tttaaatcaa aacaccagaa acccctctgt gtagggcaga ggttcttaag 3780
   cagtgcgggg cacctgcagg atctgttcca gcagacagct ggccccaaat cctggctttc 3840
   tgggtcaaga gggctgggtg gggcccagga gctgcctctc gaacaagttc cagggaggct 3900
   gctgaggtgg ggcctgccct tcagaagccc gggtctggtg gagagactgg gagaggcctg 3960
   agtgactcgt ctgcatttgc agcacctgtc tgaagagagg catggagatt agagctttct 4020
   tctctcccta caaccccatc ctctttctga gcatttccca gctgcacgtg gctgtgagga 4080
   acacggggcc tgcagtattt caggagtggc tgtatcaatg tgttttctct ttttagccag 4140
   gaagaccaac aaacaggtgt ttgtcagcta taaccttcag aacacagaca gtaacttcgc 4200
   attacttgta gaaaacagga tcaaggaaga gatggaggct ttccccgaaa agttctagct 4260
   gagtggcaga agtgagaatt tgtaaactta tgtacaatgt acgtgtaaat aaatggattg 4320
   aatttcagtt tgtcatcagg ccgcgctccc gttttgtttt taaggggtta atcttttgag 4380
   cttccttctc agcagtgtgt gggccaaaag gctcatactg acccacctgg tgaaggagag 4440
   gcaaagtggg cagtatatac ttcctcattt ggctgtgagt gatagaggga tgaaatggga 4500
   tttttgttgg gattgaagac tgtaatctaa gagtttcaat cagacatgac tgtaacgtgc 4560
   atcctcaatt agaattaaag tgatatataa atatgc 4596

<210> 67
<211> 5046
<212> DNA
<213> Homo sapiens

<400> 67
   gattttctct ggcttgtgtt tctgacagtg ttagggcctc ctaaagcttc ctaggcatag 60
   gggtatgcct ggcttgaatt ttaaccaggg actcagtcca gattatgggg agacccgagg 120
   aaaagtgctg ggtagaagac caaagaacaa agaccctcag agtgaaaagg gcctctagac 180
   atcatccctt ccaacatctc ccagtgcctg gagccctcct gcgccacgtg tgtcagatgg 240
   ccggagagcc tctgctcgaa tacctctttg atggggagct caacttttta aaagccacca 300
   gttgcatcgt aagccaactc ttgccattag aagatccttc ttatgccaga cggaaaaccg 360
   ctctctggaa ctttctcccc tggctcattc tgacccctgg agcaccaggg catacgtctg 420
   ctctcttgct tacacggcag cccttcagac atttgtgaca gctctgtgtc ctgctaagat 480
   gtctcttctc tgagctgaag atcccttgac tctttgggcc cactgggtgt gagctggggg 540
   tctcagtcct cagggcctgt cttttctctc ccccatcacc gctcaaggtc gcacatgggc 600
   agtgcagcag acgtgcggtt ctccctgggc acaaccacac acgcaccccc aggcgtgcat 660
   cgccgctact cggcattgag ggaagagtct gccaaggact gggagacttc tccactgcct 720
   gggatgctgg ccccggcagc tggccctgcc tttgacagtg accctgagat ctccgacgtg 780
   gatgaggatg agccaggggg tctggtgggc tctgcggatg ttgtctcccc cagcggccac 840
   tcagatgccc agaccctggc catgatgctg caggagcagc tggatgccat caatgaggaa 900
   atcaggatga ttcaggaaga gaaggagtcc acggagctcc gcgcggagga gattgagacg 960
   cgtgtaacca gtggcagcat ggaagcccta aacctgaagc agctgcgcaa gcgtggttcc 1020
   atccccacct ctctgacggc cctgtccctg gccagcgcgt ccccaccact cagcggccgc 1080
   tccacaccta agctcacctc ccgcagtgct gcccaggacc tggaccgaat gggggtcatg 1140
   accctgccca gtgacttaag aaagcatagg aggaagctgc tgtcgccagt gtctcgggaa 1200
   gagaaccgag aggataaagc caccataaaa tgtgagactt ctcctccttc ctcacccagg 1260
   acgctgcggc tagagaagct tggccaccca gccctgagcc aggaagaagg caagagtgcc 1320
   ttggaggatc agggcagcaa ccccagcagc agcaacagca gccaggactc cctgcacaag 1380
   ggcgccaagc gcaagggcat caagtcgtcc attggccgcc tgtttgggaa gaaggagaag 1440
   ggcaggctga tccagctgag tcgggatgga gccacaggcc atgttctgct aacagactcc 1500
   gaattcagta tgcaggagcc tatggtgcct gccaagctgg ggacccaggc agagaaggac 1560
   cggcggctaa agaagaaaca ccagctgctt gaagatgccc gcaggaaagg aatgcccttt 1620
   gcccagtggg atggtcctac tgtggtctcc tggttggagc tctgggtggg gatgcctgcc 1680
   tggtatgtgg cagcctgccg ggccaacgtc aagagtggtg ccatcatgtc cgctctgtcg 1740
   gacacagaga tccagcggga gatcggcatc agcaatgccc tgcaccggct caagctccgc 1800
   ctggccattc aggagatggt gtcattgacc agcccctctg ccccacccac ctccaggact 1860
   tcttctggga atgtctgggt cacccatgaa gagatggaaa ctctggaaac atctactaaa 1920
   acagacagtg aggagggcag ctgggctcag accctggcct atggggacat gaaccatgag 1980
   tggattggga atgaatggct acccagcctg gggctcccgc agtaccgcag ctacttcatg 2040
   gagtgcctgg tggacgcccg catgctggac cacctcacca agaaggacct gcgggtccac 2100
   ctgaagatgg tggacagctt ccatcgaacc agtcttcagt atggcatcat gtgtctgaag 2160
   aggctgaatt atgaccggaa ggagctggag aagaggcgag aggagagcca gcatgagatc 2220
   aaggatgtgt tagtctggac caacgaccag gtggttcatt gggtccagtc tattggcctc 2280
   cgggactacg caggaaacct gcatgagagt ggtgtgcatg gagccttgct ggccctggac 2340
   gagaacttcg accacaacac actggccctg atcctccaga tccccacaca gaacacccag 2400
   gcacgccaag tgatggaaag agagttcaat aacctgttgg ccttgggcac agaccggaag 2460
   ctggatgacg gggatgacaa ggtgtttcgc cgcgcgccct cctggaggaa gcgcttccgg 2520
   ccgcgggagc accacggtcg cggcggcatg ctcagcgctt ccgcggagac cctcccggcg 2580
   ggcttccgtg tgtccaccct ggggaccctg cagcccccac cggccccgcc aaagaagatc 2640
   atgcctgaag ctcactccca ctatctctac ggacacatgc tctccgcctt ccgggactag 2700
   ccatggcccc cagggctggc ttcctccttc tgggtttcac aggctcctct ggccctgacc 2760
   cctcttgctc gttccccttc cttccgcagc tcctagtctc gtccgtgact ttccggttgc 2820
   cctggatctc agaatatatt cgtccacccc ctcggcaccc cattaccccg agtcccaccg 2880
   tgtgtccgtt gtaagtccgg tggatgtggc tggggtttcc tggtattgtg gaggcaccca 2940
   ggttgtccat gcttgggatt ctgggggaag gagagaaggg cagctcaggg tggatgtgaa 3000
   gccacccttc ctcttctgga cccagcctgg tctgcactgc aacctccacc aggaccagga 3060
   tcctgggcca caggctggga tgatccttcc aagaaagggt catttcagac gcagccctgc 3120
   ttgggctatt caatcttagg gtgtctatcc acgtctggct gtgccaaatg gtctggcagc 3180
   tggttttggc atccccagca tcaccactct cccaacccat caccgtgact gcagttcctg 3240
   cccccattct cttggggtca gggaggggct gggaagggct actgaaggcc ccattctccc 3300
   acaggatggt gaggctggga ggaggaagac tgaggtagag attccaggcc ctggcataag 3360
   ctgaatccca aatttgggtt tgggaagaac cagagagaaa tggatccctg agctctgagc 3420
   caagggtgag gatggggaaa ctctaagctc ccacctaata agaagcatag gcagaccagc 3480
   cagagggaga gccaatggcc tctggtagcc ttaagcccaa agggcagtgg gaatgtcccc 3540
   tgccccaacc atcgggtgga gctcctgctg ggctatgggg aagggaggtt gtgcggatct 3600
   tgactctagg gcagaacaga tctaaccatg cattgctagc tctgctccca gcatcccttc 3660
   cccttctctc ctcctctgcc tcacttcttt agtaatccca accctataaa aatgaaccta 3720
   atgggtggat tgaatataca ttgagcccaa agtcaagttt ggggaaaagg cagactaagg 3780
   cctcctttct ctgacctccc aggaagaaaa tagcttctcc tacagtgatt catgtcccag 3840
   gtccaggaaa tccaatgttg gtgaaggcag ccactctctt gcttgtcccc aaatcaccta 3900
   accctcatcc agggctattt tggtgggcag ggactgcctc ctcccggaat tcctaagatc 3960
   cgcccagctg ccaccatttt cattgctttc cccagcagca tgatgggaac ccaagctgag 4020
   ggatacaggt cctgatttgg taggaatatt attcccaaga aatacccgct cctcacctac 4080
   tccctcatcc taccaaggtg cctgaaaatg ttcaagactt atgttcaggg tgggatgatg 4140
   gaaccgaggg cttcatcaaa gtgagaggaa aggaaaagca tctggcatgt gtttcttgga 4200
   taggggccag tgcagtgcca tcctacaggt ggctggagca gctgctttgc aacctgatca 4260
   ccttgagttc tgagcaggga ctaggcttgc aggtgagata atgggccagg gcacccagtc 4320
   cagaaggagc aatggcacct gggcagtgcc agggcttaaa gcccgctgct ccttttcggt 4380
   agaggagagg cccatcactg gtgtggtggg gtgggctctc ccttaggctt gggcaaggca 4440
   gccacctgcc cttgctctcc cttagtgttc cctggcctcc ctgccatcag gttgctggga 4500
   gtggagatgg agggattatt gagcagaaaa tgagttggat ggagataaac agctcccatc 4560
   cctgggtaat ggatggtaag atgatggaga ttcctaagat tggtggagtt gggcaatgca 4620
   tagccatctg actccttcag ggtgctcttg atgggctggc tgtaagggag actcagtccc 4680
   agcctctccc ctctacaact cctgccactg ttggccatgt cgtaaggcag cagctgtgcc 4740
   aggatagctg ggtccattca gagcaccttg agaagtgttg cagggaggtg ttaagaagag 4800
   aaatctgtgc aaacagtgat ggaaggctgt tgtcttggtg tatcccttgc ctcatagtca 4860
   atatattttt ttttttggcg agtcaccagt gacccgagcc ctccacacca gcctcctgta 4920
   tctcatcagg tcccttctca gtactgtatt tgctcagtgc atcaggaatg ggtgtatggg 4980
   tgtgtgtggg tgggtgtgag tgtgggtgtg tacgtaccaa taaacaacct ggttttaaga 5040
   caatgt 5046

<210> 68
<211> 3777
<212> DNA
<213> Homo sapiens

<400> 68
cagtttgagt tattaacacg aggaaaataa aacacatatc catataaaga aatgaattca 60
agtatttaca gcagctttat ttgtaatata caaaaatcta taaataatat aaatgtccct 120
aaacacatgg ataaacactg acttatccat agaacagcat gatactcagc aatacaaaga 180
ttcatcattt ttgaatctca aaataattat acagcatgaa gaaaaccagt aagtatatat 240
atatacacac acacatatat aaacacatat gtatatatat acgtatacgt atatgcgtat 300
gcgtgtgtgt gtgtgtgtgt gtgtgtgtgt atgtatctca gtaaaaactc cagaaaatgc 360
aaactaattt actgtaaagg aaatcagttg aacctgaaga gctggggaag ggagaaagat 420
tataaaaaga tacaaggtaa atatatgggt gacacacaaa ttcattttta tggttgtaat 480
gatggtttca tggctttacc aaactgtaca ctttggatac gtgtactttg ttgcatgtca 540
gttatacaaa ttagaccttc aaaaagcttt aaaaagtggc tcaaaattaa gagcctagat 600
gttccttttt ttttaatcag ggtggcagcc tgatatggac ttcaccatct ctctctctcc 660
tcatgccctg agagaggcaa ccttgcctca ggggcagcaa attttggcaa gcagtgaagg 720
ctgttgccac ctgaattaat aggaatgttg gctgagtcac aaaagccaca tccctggacc 780
gcggaatgat ccttctctct attcttgttc tcctgcctgc atagcaatgg ccttgaggtg 840
aacagcccag acaggataca aggagccaca aaacctttat agactatttt cttttttttt 900
tttggagacg gagtctcact ctgttgcctg ggctggagtg cagtggtgca atctcagctc 960
actgcaactt ccgccttctg ggttcaagcg attctcctgc ctcggcctcc caagtggctg 1020
ggactatagg cgcatgctgc cacgcctggc taattttttg tattttagta gacatgggat 1080
ttcaccatgt tgcccaggct ggtcttgaac tcctgagctc aggcaatccg cccgccttgg 1140
cctcccaaag tgttaggatt acaccatgcc cggccagact attttctgat aatgatctca 1200
gcgatagtaa acagaagatc ctgagaattt ctgtatgcac tgatcctaca caaccatatt 1260
cactggaaaa tttgtagatg aacttttaga agagtcattc catctaattt actgcagtct 1320

tctccactcc ccatttacct ggagtttctg tcccaattac tgttgaacct gtaatttcac 1380
ctccacacct atagtctgat aacaaatggc accagtctac actgacttat atgccatggc 1440
acatggctcc tttgcaggac ctgggcaaca gattcccctc accagtgcta ggaattatgt 1500
ggatctcctg tggacaactg aacaaaattc acgttcacag aagcactggg gattgtcaca 1560
actgcctcct ttttatggtg acctctgacc tgacaaaaat ggcaccaggc cacaatacag 1620
gaacacaaag cctggcagtc tttactttga ggccttgggc agcaggccaa ttagagcaat 1680
acagaacaaa gagctgtctt ctgccatcac attgtccctt ctgagtacag aaagtttggg 1740
atctccacaa aatctattca ccccttcatg ctgacatcac tattctgctt acaagtcatt 1800
ctatgcaatg aatagaacac ccaagattaa aggtgcgtaa atttttttaa aacctttata 1860
aaaacattat aaattaaagc aatgtgcacc aagcagtttc caggctggtg aatcagaatg 1920
tggaaaaatt cattacaggc atcatgtgag accgacaatg ttccttaaaa aggtaatcct 1980
tagaaagctg acagcacaac tcagattctc tggaccacaa ctgggtcagg tgtatactct 2040
aaagtcagag aaaagacatg aagaccctac atgcttgggc tattaataat ttccccaagc 2100
aaatgggaga gtacagaggt ttgcttctct gagcactgtg ggggagtgac tcatgcacat 2160
ggtgtgcacc tgccaggtgg taggggtgaa atgtgaaatg gcttcagcct cagaaattgg 2220
cagtgacatg aatcaggcta taggcctcgg cagattagaa attacaatga acctcctcag 2280
agcttcccac atacctgaaa cttatgaaga ataccccact gtgggtgctc aaagtttaaa 2340
tttacgaaga tggctaccac agtctcctcc agtgctatta tactgaataa agaaatagca 2400
cattcccttc acatttaggg cctttcttca gtgtgaactc tccgatgtcg aatgaggtta 2460
gatttgcggc taaaggattt cccacattca ctgcattcat aaggcctttc tccagtgtga 2520
actctttggt gctgaataag gtctgatttg tgggtaaagg gtttcccaca ttgactgcac 2580
tcataaggtc tttctccagt gtgacctctc tggtgttgaa tgaggctaga gctctggcta 2640
aaggatttcc cacattcact gcattcatgg ggcctttctc cagtgtgaac tctctggtgt 2700
tgaatgaggc tagcactttg gctaaaagac tttccacatt cactacattc ataaggcctt 2760
tctccagtat gaagtctccg gtgctgaatg aggccagagc gctgtctaaa agatttgcca 2820
cattcactgc actcataagg tcttgtgcca gtatgaattc tccggtgttg aatgaggtca 2880
gatttgcggg taaaggattt tccacattca ctgcattcat agggtctttc tccagtgtga 2940
actctttggt gttgaatcag gataaatttg cggctaaagg atttctcaca ttcactgcac 3000
tcataaggcc tatcaccagt gtgaatgctg cggtgtcgaa tgaggttaga gaattgtcta 3060
aaggatttcc cacattctct gcattcataa ggtctttctc cactgtgaat tctctggtgt 3120
tgaatgagtt ccgatttgca gctaaaagat ttcccacaat cactgcactg ataaggcatt 3180
tctccagtgt gaactcttct atgtcgaatg agattgaaga tttgtctaaa ggatttccca 3240
cactcacagc actgataagg cctttctcca gagtgaactc tctggtgtcg aaaaaggcta 3300
gagctttgtc taaaggattt cccgcactcg ccacattcat aaggcctttc tccagtgtga 3360
actctctggt gttcaatgag gttagatttg tagctaaagg ttttcctaca ttcaccacac 3420
tcatgacgca ctcctccagt gtgacgtctc tggtggtgaa tgaattcaga tttatggata 3480

aaggctttct cacgatcact gcattcataa ggcctttctc cagtgtgaat tatcttgtgt 3540
aagattaggt ggcacttttt gctaaaggat tcctcacatt cactacactc aaacatcttt 3600
tgtccagtgt gaatttgctg gtgctgaaca agtgtgttct tgcaagtgaa ggctttctca 3660

catttgctac actcataaag ccctccttca gaacagacac tctgatgctg aacaagtgtg 3720
tgtttgtggc ttgaagcttt tctgtattca ccccacttgt aataactttt tgcattg 3777

<210> 69
<211> 3716
<212> DNA
<213> Homo sapiens

<400> 69
   acgtacttgc tgacccagaa tgctctttga catgctgcca aaaaataaac ctgtcctccc 60
   acaaaaacct caattgtcct ttcatgaaga atgaaaattt tgagcttaga attaccactt 120
   taatgcttga tttcaactag ttaagtaaat taataggtat gtatcaatat atttgccttc 180
   aagcagctta gattgtagct ggaacaaaag ctataacagg gatgaaatcg gtgcaagatt 240
   gtacaagccc ctggctctgc cttttaggga ggctaaagaa gagagaggca tgtatgggtt 300
   tgttgacttg ggaactcaga gtaggtttga gcccaccctt ttgtgtggtc agccttcctg 360
   tccagaggaa aagtgtagag tgggaatgag aagggtagga ggaaaagatc actggggagc 420
   aggaaaatgg gcagctgtgt tatatctcct catctctttc ctcccctcta ctggggatgc 480
   cagagaagaa atttcttttt tttttttttt tttttttgag acggagtctt gctctgttgc 540
   ccaggctgga gtgcagtggc accatctcag ctaactgcaa ccttcgcctc ctgggtttaa 600
   gtgatcctcc tgcctcagcc ccccagtagc tgtaattaca ggcatgcacc accacgccca 660
   gctaattttt ctatttttag tagagacggg gttttgccat gttggccagg ctgatctcga 720
   actcgtgacc tcaggtgatc cacccgcctc agcctcccaa agcactggga ttacaggcgt 780
   gagccaccgc gcccggccca ccagagaaga aatttctaaa ggcaggagtg gaaagagttt 840
   agaaagagaa gctagtcaga gaggcagtca gccttgtgga agaatgctgt gtaatcctgt 900
   gtcaccaaag gctgttcttt tctcaagaag ctgagatcca ggattagagt ggaaaacaag 960
   aaaaacagat atatcatttt gttggcactc aattgccttg gcgttttgtt ttatgtgatt 1020
   gcctgcaaga ccacactttc aatcccttcc ttcaataagc atttgttatg cacccaatga 1080
   aacacaaaac ataaaatcat ctttaacact ccttcttctc taatagacct aaagaattca 1140
   ttgagtgtgt ctcccatatc cgactgttgt cctggctgct gctgggttcc ctcactcaca 1200
   atgcagtgtg cccaaatgcc tcctctccct gcctgcccat tcctctggat gcaggctccc 1260
   acgttgcaga ccatcttatt gttatcctga ttggatttcc agagcaatca aaggtaagtg 1320
   atttctgcaa gattaagacc gtatgcatcg caattgctaa tggaaacctt atcagccaat 1380
   tatgtttctt ctgagaagaa aatacatctt atcatttgcc ctcctgtgga tgttagaaac 1440
   acaactttgt gctaatgtct tatttaaagt tgtttagatt cccagactgt ctttcccccc 1500
   gcccccaaat tgtttttcgg cttactttta taaagataaa tcatggagtc aagcaagagt 1560
   tggcttccca ggcatttctg tctcaaaata atgaagagaa ataaaaggct agaaatataa 1620
   tcaaaattct gtgatcagtt tttaaaacca gagacaaaac aaaggaaaat tcattctcta 1680
   tctaattctt catgtaccat cttttcaact gaaatctcta gagatacagg aaaatacaaa 1740
   atgaaaacaa gaaggccagt tgggaactca ctaaagcaca cattggcatg acctacacag 1800
   gggaactttg taaattgcag agacaggtga tgtgctaaag gaagaaagta gacccaaggc 1860
   aggcaccagt catgagcctg tgcaggcaaa ccggacacat tggtactaat cagctccgag 1920
   gagccagggg accttcacag agcttcggca agttctggga ggtcacattg gtcttgggga 1980
   cagctcgggt ttgggacagg gcaagctgac gatgtgataa aatggcgcac ccatcccaat 2040
   gatccaaatg atgttcacaa aaggaattag gcagtcgatt cctaatattt ctactcagat 2100
   ccgggtttga ttcctaacta tggtacttgc gattttgtga gcttgacaag tgtgtctaac 2160
   ctctctatta ttcagatttt catctgtgaa atagacgagg ggatggttgg agagtgatgg 2220
   aggtgatata ggaaaagcct ctggcgccat ctctggcgtg ttgtaggtgc tctctctgca 2280
   cgtgctttcc gctaggagga ggatctccgc tgctgctgct atgattatga acttctgggc 2340
   gtgtattcac gtatatcacc tgctttactc ccatgctttg aagagactac tattatcccc 2400
   attttacaga tgaggaaaca tcctaacatg gcagaatgat tattctccct aatctagttt 2460
   agggatggga atgtcagttt cattaaaggg caagggggtg ggtagatttc ttcagcaaat 2520
   taaggtgcct tctcttcaaa aatgtgcttt gttctctgag cacactccca ctggtggaga 2580
   agagagcaca gcagatagtt tcaaggtgat gaatcatgac tttgtaactc tgagataacg 2640
   ttatgccttt gtgcttcccc tgtgctccct ggcaagacca aagccatgcg ggaggactcc 2700
   gtggagggcc acagcctgct gggatgcaat gcactgatgc cgtcttcttc cttttcagac 2760
   ctccgtgctg cacatgtgct ccctcttcca cgcgttcatc tttgctcagc tgtggacagt 2820
   ttattgcgag caaagtgccg tcgctacaaa tctccaaaat cagaatgaat tcagcttcac 2880
   ggcgatactg acagcactag aattttggag tagggtgaca cccagcatcc ttcagctaat 2940
   ggcccataac aaagtgatgg tagaaatggt gtgtctccat gtgattagtt taatggaggc 3000
   attgcaggaa tgcaattcga ccatttttgt caagctgata cctatgtggt tgccaatgat 3060
   tcagtcaaat atcaagcact tatctgcggg actccagctt cgcctccagg ctattcagaa 3120
   ccacgtgaac caccacagcc taaggacgct gccgggctcg ggccagagca gtgctggcct 3180
   ggcagccctc cgaaagtggt tgcagtgcac tcagttcaaa atggcccagg tggagatcca 3240
   gtcctcggaa gcagcctctc aattttatcc tctatgagtg gactcctcgg cgctcagtgt 3300
   caacactctg gtttagcaat aatgggttta aaaacaaaca atttgatcca agcaggttgg 3360
   ggaacatatt ggtactgtac attctctttc tagtttagta aaagatgtgc aaaggccaga 3420
   gagggccgaa aatgaagctt tcttgctaca catatttctg atgactcctt gggctatctg 3480
   attaagtgtt tccttacatt attttttaaa aaccaaatca tttttcttta actaacttct 3540
   atttttttta agaaaaaaaa atagactggt gggtactcac agaaaagttg tataagtccc 3600
   cctgttgcta tttttgatga tagagaataa atagggtttt tgaaaccttt gtagtgtttt 3660
   ttcttaaaat ccactcttgg caatgcaata aaaaaaaccg tcaccataag ccagtg 3716

<210> 70
<211> 4050
<212> DNA
<213> Homo sapiens

<400> 70
atccacccgc cttggcctcc caaagtgctg ggattacagg cgtgagccac tgcgccgggc 60
cctcaaagga gccttttcac gtgggagggc tgtgccgact ctcaccacct tcctgttcaa 120
catctgtcct gtttccttgt tttccttcag atggagtttt gtcttgttta ctacccgaga 180
gcccataggt gtgcctccca aagggccctc ctgcagacca gagcaagctc ctctaggcca 240
agccggggcg ccgccagcac gtgcccttct gctggaaccg ggaggtctag agccaggccc 300
agaacaatct gtgatttctt gtgtttttac ttgacttggt ggtttctctt ttgccaaaag 360
gtccttactt gacttctgaa ggctgtttgt accaaatact tttcaaaagc ccaagcctgg 420
tctcctcaca gcttcctaag gcagtgctct cccccctccc agtgctatgc gctgggcaac 480
ttggggtgcc ccccatgagg ccaagggagg accctggggt cgagtttcct tcgggggcat 540
tgtgtggagg ggcaggcagc tcgctccaac tgctgggagt gggggaatcc cagctctgcc 600
catccctgcc aagttggggt tgtgtgtgga tcttggtgtt aactcttgct tccttctctg 660
cttctgccat gcttccaagc agcagctact ccatccgcca ctcaataagt atgccagcca 720
tgaggtaatg tatgcctgct cgctgcggct ccagagccgg gtgtctctgg tgggggttgg 780
ggcagctccc gccggttaca ccttgccctt cccatattcc tggattaatt gaggactcca 840
ttcccttggc aacctctgat gactaagggc cggtaaaaac cccctctagg ctgacgagga 900
agagctggta gggggtcagg ctcttcacct gcagcctcca gaggagatcc ttgtgtcaaa 960
ctgggccgtg tcctttccaa gtcctgaccc tggctgcctg cagtgggtgg ggtggggttc 1020
gaggggctcg cacacagatc ctggcacttg ccctggtggg ctgtcactca ggacacttca 1080
cggtctcagc cttcacggaa acacccagag ctgtggtcct tattctgtgc ctgtcaggca 1140
ccaccctgat tctcaggatt ctgtgcctgt gccgtgaggc actgccctga ttctcaggag 1200
agcccagaac tgtggtccct attctgtgcc tgcgccgtca ggcaccgccc tgattctcag 1260
gagagccctg ttctccttgt ggggactctg gctgggtgtc gcagacgtgc tggcctctag 1320
agtccccggg cctgggaggg gtatctgttt gtgtgggagg gactgagtgg gatggagctg 1380
gcctgctttc tcctcttgtc tccttatcac tctgctgtcc cagaagtgtc cttggtcttc 1440

cgtttgcttc tctgagagat gtgcatgaag aactgcacag acttttcata acttcagtac 1500
gagagcccag ggtgtccctg taggggacag cagagagggg ccgacagccc cccaactgcg 1560
cagacttttc ataacctcag tacgagagcc cagggtgtcc ctgtagggga tagcagagag 1620
gggccgacag ccccccaact gcacagactt ttcataacct caatacgaga gcccggggta 1680
tccctgtagg ggacagcaga gaggggccga cagcccccca actgcgcaga cttttcataa 1740
cctcagtacg agagcccagg gtgtccctgt aggggatagc agagaggggc cgacagcccc 1800
ccaactgcac agacttttca taacctcagt acgagagccc ggggtatccc tgtaggggac 1860
agcagagagg ggccgacagc cccccaactg cgcagacttt tcataacctc agtacgagag 1920
cccggggtgt ccctgtaggg gacagcagag aggggccgac agccccccaa ctgcacagac 1980
ttttcataac ctcagtacga gagcccggga tgtccctgta gggtatgaca gagaggggcc 2040
gacatctccc cagctgcaca gtcaaggggt ttccagttgc cttgtgtgac gtctggacct 2100
gtccctgtgg ttgttgggca tggccgacct cacttttgac ctggggagtc tccggaatga 2160
cctggctgtc tcacagtttt gccatgagat cttgggatgt tgttagggac cctttggagg 2220
ctggccctaa ccctgaggcc cttccgagtt aggagagcag cacaaccctc tcctcttgac 2280
accgacactc ttccctctct ctttaaagga actctgcgac cttcaagtcg tttgaggacc 2340
gagttgggac cataaagtct aaggttgtgg gtgacagaga gaacggcagt gacaacctcc 2400
cttcctcagc ggggagtagt gacaagcccc tgtcggatcc cgcacctttc taagcctgtg 2460
gttgcttcac ccgctgcaga gcacacgcaa cccagcctca gcatcacagc cgcagctctg 2520
ttcagcggag cagccagcca gggcggatga gcagagccgg ccctgaggac agtcctgccc 2580
atccacgcgg agatgtggct gccgcgtttg catgaatttg aagaacacag gcttgtacac 2640
agatgtttta cactcacgtt tgtagatgaa acagatcact gtgctgtcct tcctaggggt 2700
gcaggaagtg gacagggcgg agggtttgaa agaatattga gtcaaagccc aggctccctt 2760
tgggaatcat gttagcccat cagaatgttg aaggattgaa gagttctaag cataaaataa 2820
gtggcatttt ctgacttctt cctcctcctc cttccctgac tcacagaagg aatgcaatca 2880
cccagcaagt cctacctgtt acgcaatttt ttatctcaaa atgccgaacg agaaaactgt 2940
ccattttctg agacccccag aaaggaaact gaccctcagc agctgcctga ttgttacgcg 3000
aatctagctt taacggaagc aaattcatta tttttttaaa tgcagtggac ttttcaaaaa 3060
gtttaaatta ggcaaagcag ctttagcctc atagaatatt atttctttgg actcaagctg 3120
aaatacaagc cttacattgc cttatgcttt atttctttct aatttttata tgtatataga 3180
tgagggttcc ttaatggttg tgagcattgt gtggaatttt acacctggcc tgcgtggcag 3240
cctcttccag ttgaggtgtt ttatgtcacg cacactccat cccagtgtac aaaacctgct 3300
tctcttctca accgtggcag ctcccgctgg ctcctatgcc ctgccctaaa gggctcttga 3360
gcctctggga atgggagggg ccaagagaag gaaaaccctg tctttagcac cctttaaaag 3420
aactgtgccc cccttctcag tgctgccttt gcatgggcct ggcccggctc acattcgtca 3480
gtgactccaa ccctcctgct tgctgtactt gggatgaaac gaccccacag gtcaggtgga 3540
gggtggggcg tgggcatcag ccaggattgc cgttacagtc tttttctcag gagctacaaa 3600

gatctcttcc tgttactaaa tagtcgcacc ccagcagcct ctctcgcaca ccggggccct 3660
gcgtgtcaga tggcgtggtc tgcaggggga gctctgtgcc ttagtggctc ttggcaggac 3720
actgagggcc tgcctgtggt gtgcccggct ctgccactcc cgggagggga agggctgctc 3780
agctcaaggt gtcctgttcg gtagagcaag tgtcctctga cagccgtgtc cccggacagt 3840
tcagacaccc ttggggatgg cactccacac acgacagaga tgcaggggcc agggaagccc 3900
agcgctcggt gcccttcgtc cagggttaaa atcggcctgt ggggtgtggt gagaaggcag 3960
gttgtgcggg tgttgaccga tgtatctttt ccttaaagtt attataataa tgggtaattt 4020
gtcaataaag cattcctttg ggggaaagtc 4050

<210> 71
<211> 4741
<212> DNA
<213> Homo sapiens

<400> 71
tctctacagg agcagtcagg aacgccaaag aaatctttgc atacattgga aaaccccat 60
tttccaaagc caacctgtga cctggccaca taatttttga tcgcactggc atagcctctg 120
aatgtccctg ccaaggaatg tgggccaggc acccattaag aggaaacaga agaaaggtgg 180
gattaagttg ggagtcagtc ctggcaaaat ccatctctat tttcagagga cagttttcta 240
aaggaaggaa aatggaatgg aagtatagga agaaaatgta gacatggagg tctctagtaa 300
aaccaatccc aggagggcag aggctgggta agaggggcaa acatggctga gagggtctgg 360
caagcttccc tccctatatc tgccatggct gcttctctga gacccaaggg cagggagagc 420
actgatggta gcaggagcag cagtgcagcc catgtcccct cctctggaac ctactcccgc 480
ccctcttgga ggaaaagcag ggcctcccca gatgtatacc caagtcagag cgcaggatcc 540
acagaaagac cccaagccca ggcttccagt cccatccctg cctctcctgc tcttcctccc 600
tgctacggtg atccaggcag ggctttgggg cctccagatc cttgtggcca atgctggctc 660
attgcagaca ctctagggtc caaaggctgc cagatgcata gagaggagtg gcctggccac 720
aaccctcagg ggcgctaagg tgggaaaaga tgaacaccat aaagatggga taaaagctca 780
ggtctgtcca ttttgggagt gcatattccg aactgcctcc tatacctatt agctttgttg 840
tcaccaggaa atgcagagtc ctcaattctt agtctgtctt gggagaattc ggccaagaga 900
ccaagtagta atgtaagcaa aaggtttact gaaggaaaat aaagagcagg gattgtattt 960
atttatttat tttgtgacag agtctcactc tgtctcctag gctggagtgc agtggtgtga 1020
tctcggctca ctgcaacctc cacctcccgg gttcaagcga ttctcctgcc tcagcctccc 1080
aagtagctgg gactacaggc acgtgccacc acgcccagct aatttttgt attttagta 1140
gagacagggt ttcactgtgt tagccaggat ggtctcaatc tcctgatctc gtgatccacc 1200

tgcctcggcc tcccaaagtg ttgggattac aggcgtgagc taccatgccc ggcctgggag 1260
tttattgaag tagactccaa gagaggagcc agcaggtccg cctgggaagc agcagtattc 1320
acagcaagag tgaggtagca gcagagacaa tacactctga aagatgaggc agagcaggct 1380
gctcgaaaga tgagccagca actagagagt tctgccttgc ggtttttatt aggtcacact 1440
ctatttaggt tcctgcctct gtctcaagtc tccacctttg tctaatttcc tgcctctatc 1500
ttatgtctcc atcttttcgc cccacctagt tcccgcccca ggtttgtggg actctccctt 1560
actgtcagct gatgaacctg caggggcggg tgtcggatat gaatcctatc gaatggtgat 1620
gttgctaatt actgccactc caggaagggt gtatattggt atttattgca cgtgcatatc 1680
tcgtaggaat ttctcctttg ctttctttcc ctccttagca tgcagctggc tacattccca 1740
caggttaact gcagagtgat gactgggcat cttaaggggt gtcttggggt gttccttcct 1800
gcatagatat ttcccctcct ctctgctcat atctagggtg aatgttttgg atggtctctg 1860
gggtgtgaga ctttccagac ctccctttct cagaggcttt cccacctgct catatttatc 1920
tgcctactcc gacagtataa ccttgggcaa gtcacttgac ctctctgagc actggttttc 1980
tcattataga tcgaaagcat tagactaagt cagtggtttc caaactttcc cagccacata 2040
accctttctt taaatgaaat cttactggaa ccctaccatg taacctgttt actgaacagt 2100
cctaatttga tgagatacaa acatgaccag ttttatcttt cttgcaacca ctgccttcca 2160
acgtctccat tgagcccccc ttcccattcc taaggccacc tcaggcagac cctggtcact 2220

ttcagtggat aaaaagtctg cccagttgtt tcagtttaaa gcttcgatct gatgtccaac 2280
atggggccca catcctccca tcctccatcc tctgtttcgg gtttgcccca tagtctaaag 2340
gattcagacc agggaggaga acatggtctg ctctctcctg gctccttccc tctcctcccc 2400
agcactcttc tggacctctg tcttcagctg ttggggcagg gaggagggag cagaaaaaga 2460
acctttctg acctcctggc tgaagatgtc cttggtgctt caggcatcca gctgcttggg 2520
ggccttccta cctcttgctg aaaggtgttt agcagtcagg tctttgggac cgggtcctgg 2580
ccagatggct ccagtgaggc tgccttcctt ggtgctcact tggcacggag gagacccacc 2640
cattcttgcc ccacctggca atgggaggcc gcccgctgct gccccaatct gtcctgccct 2700
ctctttcttc tgccccaaca ggcaaagggg cagatcagca aggtctagca taaggagatg 2760
cccagccagg gaatggcagg tcagcttccc cttctcgtgg gcacctgtat gtcatacagt 2820
gagtggcacc attggaggcc cctcccttga cttgggcagg ggccaggcat ccccaggtct 2880
ccacagggag gtgcagggag gtaaagacca cagcagttcc ccaaggctgt gcctccttac 2940
gagggggatt ctcaactcct gctgaacatt agatcccatg aaatgctcag taagagatac 3000
caatgtgccg acccccgggg ccagttaact cagcatcttg agatggagcc tgagaatgtt 3060
tcttttgaaa gcacctgagg tgattgcgga aggcagccag ggttaggaac cactgccctg 3120
tccaaaaagt tcccatcaaa tgatggagaa aggacctgtt agacctgctg atggccaggg 3180
agggtctggg ttctcccttt gtaaggtggc aggggcgggc agacttagga cctggtaccc 3240
ttagttttag ggctttaggc acaatttgaa agtaccagaa gagttcccat tcaacattct 3300

actataagtg gttgccgaac ttgtcttagc agcagaactt tcctcatacg agtgtctggc 3360
tgcagcaagg ggaggggcca gagctttgcc agcagcattg gaacctgccc cccctcacac 3420
ccctttaatg aaataatcac tcaggcccct ttggctcact ctgagaacct tccgttgcta 3480
tgttacattg gagagagggg gccaccctgc agctctctga agggagaatg gggtggaaat 3540
ggagccagaa gcacaggcag agtagagaag caagggggca cccatagcag gttgtgggag 3600
agccagggtg ggccaggagg tgggagtaga aaggaagtag gcttgggggt gtggagaggg 3660
tggctgctga cctcccaatt ttgctgagaa cagccatctc taagccagag tacccagaaa 3720
ggtgtgctcc tccctatgcc ctgagcaggg ggaagaagag ggaacgtggc ctcagaacct 3780
gcagcagcca ccaccgccgg gtcagcacaa ggatgggcag aaatccactg gggatgagag 3840
gcccaccacg gagcccctct ttaaggtcct agatacgcct ctgagcgagg gtgatgagcc 3900
cgcaacgctg ccggccccgc gggaccatgg gcagagtgtg cagatggaag gctacctggg 3960
ccgcaagcat gacctggagg ggcccaacaa gaaggcttcc aacaggtcct ggaacaacct 4020
gtactgtgtg ctcaggaaca gtgagctaac cttctacaag gatgccaagg acctggccct 4080
ggggatgccc taccatgggg aggaacccct ggccctgaga catgccatct gtgagattgc 4140
tgccaactac aagaagaaga agcacgtctt taagctgagg ctgagtaatg gcagcgagtg 4200
gctcttccat ggcaaggatg aggaggagat gctgtcctgg ctgcagggcg tgagcaccgc 4260
catcaacgag tcccagagca tccgcgtcaa ggcgcagagc ctgcccctgc cttccctctc 4320
cggccccgac gccagcctcg gcaagaaaga caaggagaag agattcagct tcttccccaa 4380
aaagaagtag caggtggggc tggcgggcgc ggcggagtcg ggacgcgcag agaccgggca 4440
gcccaggcct tggcctcctc tcgccgccgc ccgccggtcg aatcgagatg agtcgagtcg 4500
cgggccgggc ccccgggccc cacgctgcac tgcaaaagct gccttcgccc gcgtgtcctg 4560
ggccgacgcc ccctccatgc cctcgcccgc gctggctccg ctccagggac aggggctccc 4620
agctcctctg gcgcctttgg ggcctccagg cctgggggca ggaaacggtt ctctgaggca 4680
cacgccctcc tctcccaccc tcctcctgag ccgagggggc acaggcccct tacccccaac 4740
c 4741

<210> 72
<211> 4321
<212> DNA
<213> Homo sapiens

<400> 72
ttttactact gttaaacagg gtcgcagtga attacatcct gatttttttgg ctaaattaca 60
agatgctgtt caaaatctgt ctctgatgat tacgctcaag gtattctcct tcatatgtta 120
gcttttgaga atgcgaacca tgagtgtaaa gtggtcatgc gttctgtcta atgacaaagt 180

ttacctgatc acgaggtgtg cctgcatata ttaaagcttg tgaaagcatt ggatcagaga 240
cccacaaagc tattctgtgg gcacgggcca tgaaggatgg caatcaaact ggcttgactg 300
aattcttttc ttggagcctg ctataattgt ggtcaacttc atcataccca aaaaaattgc 360
actgttaaaa gcggccaagc cagctcaaca aacatggcca aatgctcctg ctactgtttg 420
ccctcattgt cgtaaaggta aacactgggc aagtacttgc cactctaagt ctgatataga 480
tggcaatccc ttgccacaga accagggaaa tgggaagcag ggccagtccc aggccccagt 540
atcaaatggg acacctcaga ctcagaccaa tattgtgttt ctgcttcaag cggtcccaat 600
acagccccca gcacaggcaa atttacctac agccaaccca gatgggtccc agtcgcttct 660
tctgtctcag tacaatgctt gtctacctcc acagtagggg gcagggcggt caatctctgt 720
agtaccactc ctctaaattt actaccgaat tctttgcctt taattgtccc cacgggggcc 780
actggccctt tacctcaagg tttggtgggc ctggtgttag gtagggcatc cacctctgct 840
aaaggtatca tagttcatac tggtctcatt aattctgatt cctctgatga gattaaaata 900
atcgtgtctg ccaaggttcc tgtttccatt ccggccagtg agtcaattgc tcaactactt 960
ttactaccta atattgtttt aaacaaagga gataagacag gggccctgg gatgggctct 1020
ggcggtgaaa aagccgctta ttggattaaa gtaatttcta aacaacagcc cacctgcacc 1080
atacatatta aaggaaaaaa gtttgagggc ctagtagata ctggggctga tgtttctatt 1140
atttcctcta atttatggcc ttcttcctgg cttaaacatc ccactaacat gggactagta 1200
agggttggaa aagctgatga agttcaccaa agcacattta tcttgccttg cactgggcct 1260
gatggtcaaa agggaacaat tcagccttat atcatgccaa tccccattaa tctttggggt 1320
agacatttgc tggaacaatg gggggctgaa attaatattc cacataactc ttctcgtgct 1380
cccagtcaac atataatgga aaacgtgggg actgttcctg gactcagtct cggtccaaaa 1440
catgaaggaa ttactaaatt cctttatggt ttaaaagcat aatttaacca ctccccactc 1500
ccaattccat ttagcattgt ttactttaaa ctttctaaat gttcctaaag acaatactct 1560
gactgcagcc gaacaccatt atacaggcaa aaaattctcc ctaaatgaaa gcaagccagt 1620
gttatggaaa aactcccaga caaatacctg ggaacctgga acaattataa tgtggggaag 1680
agggtatgct tgtgtttcac caggagatca tcaatcccct gtctgggtgc ccactagaag 1740
actttaactt cgtgtgaata ctgacaatga aaaacacagg gaagagatgt ccacgtcaga 1800
gactgccctc atacctggtg agatctgtgc caactcctca aaaactggca caccaaatca 1860
aaatggatct cattcaatcc tccctaatgg caacagagac ccctctaact aatcccactt 1920
ctcctaatta cctttctttt tctccttaca aacctgaaaa tctcaccatt tctattagcc 1980
tgaaaataac atccctctgt tcttctcttc ctccttcagc actggatctc gcttacaata 2040
ggttttattt aataattcta ctccttatac tttctgtctc accagtttcc cctcacactg 2100
atttgcctgc tacacaaaat tattcttatt gggcttatgt gccttttcct ccacttattc 2160
gacctctcac ctggatgaat gctcctgcgg aaatctacac taacgatagt gtgtagatgc 2220
ctggagctat agatgaccat tgccctgctc aaccaggaga agaaggcact gcatttaatg 2280
ttactatggg ttatagatac cctcctctgt gcctcaggca tgcaactcgt tgtatccatc 2340

tagaaactca agtctgggct gcttatcttc tggagagatt agctacagga aaatggggac 2400
atttggtctc cggcctctcc ctttgtcctt taagacaaat gaaaagggga gtaataggag 2460
ataccccata ctttcaatat aaacctgtag gaaaactatg tcctaaaaat tttgagggcc 2520
catctaaaac tttaatttgg ggagattgtg ttaactcaca tgcagtagta ttaaaaaatg 2580
actcatatgc tttagtaata gactgggcac caaagggcta tttaaaaaac acttgctcct 2640
ctgggggagg ggaattcctg gaggctactt attttatttc ttattgggag gacgaggatc 2700
atcatcctac tttgcatagg tggttcggct cattctttac cttaaaatgg gaagataagg 2760
acattaccct ccacccccaa ggccttgtat gatattcccc attctgagcc cagaacaccc 2820
agaactttcg aaattggcta ttgtcatgtc tggactgcca gtatctgtga ttctgaaccc 2880
catgataaat ccccctttgaa ccttttttcct cttttgatg ccgatcctcc tttacgggac 2940
tccaattggc attacgataa ttcttatcga cccaggtatg cccctctact tcttcagcat 3000
ccccaggcac ctcggtttgc ttctttatgg tggagaacat cgggcattgc caccgccgct 3060
cctctccctc agtatcaaca tagattcaag cattctgctt tgtttacctc caacctgact 3120
attcctatac agagttgtgt taagcttcct tacatgctgt tagtggcaaa tatcaaaatt 3180
tggacaaaca atcaaactgt ccaatgcatt gtcatttata cacttgtgtt gactcccgtt 3240
ttgactccag gaaaagtgta atgttggttg gagctcgaga aggaatctgg atactgtgtg 3300
ccggaccagc caaagaatcc tgtgtcaaaa ccgagagaac gaataagcct tcatcaccat 3360
ggcacattta tataaaaaga aagggagaga tgttgcggga agtcagggac cccgaatgga 3420
gggactgact ggagccgcgg cagaggaaca taaattgcaa atatttcatt ttaatatgga 3480
catttatcag ttcccaaatt attacttttt acatttctta cgcctgtctt actttaatct 3540
cttaatcctg ttatcttcat aagctgagga tatatgtcac ctcaggacca ctgtgataat 3600
tgtgttaact gtacaaattg attgtaaaac atgtgtgttt gcacaatatg aaatcagtgc 3660
accttgaaga agaacagaat aacagtgatt tttaggcaac aagagaagac aaccataagg 3720
tctgactgcg tgcagggtca ggcaaaatag agccatattt ttcttcttgc agggagccta 3780
taaatggaca tggaagtagg gaagatattg ctaaattctt ttcctagcaa ggaatattac 3840
tattaatact ctgggaaagg aatgcattcc tggggggagg tctataaacg gccactctgg 3900
gaatgtctgt cctatgcagt tgagacaagg actgaagtac accctggtct cctgcagtac 3960
cctcaggctt actagggtgg ggaaaaacct ggccctggca aatctgtggt cagactggtt 4020
ctctgctctt gaaccctgtg ttctgttgtt taagatgttt atcaagacaa tacgtgcact 4080
gctgaacata gacccttatc aggagttcta cttttgccct tgtcctgttt cctcagaagc 4140
atgtgatctc tgttctgctt tttgcccctt aaagcatgtg atctttgtac ctaccccccg 4200
ttcgtacacc ccctcccctt ttgcaatcct taataaaaac ttgctggttt tgaggctcgg 4260
gccggcatca cggtcctact gatatgtgat gtcacccctg gcggcccagc tgtaaaattc 4320
c 4321

<210> 73
<211> 4234
<212> DNA
<213> Homo sapiens

<400> 73
ccacgaagct tggccaactc ctggtccttg ccttcctttt ccgtcagggc atgagccagt 60
gcctcttgca gggtagcgaa ctccacacgc tgctcgttga gggcgttctg cagccgcatc 120
tcaagctctg ctctggccgc cttctccagg gcaaggtcag cctgggcacg gccccgctcc 180
tgggtcagcc gcgccacctc cctttcctgc tgcccacgct cctcctgctg ctgcccctgg 240
ctctccatca gcgcggcccg cagccgttcc agctcattgc ccatctgctc tgcctcccgc 300
tccatagcct gcagcgctgc ctgtgtgctg cagaactggc gtccctgttg ctcttccagc 360
cactcgggct gtctgtctcc tgccctcgca ggctccttga ctaactcccg ggaggctgtt 420
tcctgctgct cacctgcctt gcgcaccaag gtctccaagc gggccacctc tttgctggtg 480
gcagccatct tttcctgcag agtggagagg tcatctgcaa gcttctgggc cctgacttcc 540
ttctcttgga cctgctggag tgctctggcc aggttggcat ggagctcagc tagttcgttc 600
tgctgccggc ttatctggag ctcgctgtgg gattctatgc ctgccacctt ctcctttgcc 660
tcctgcagct cctggcgggc cttctcacat tcctccttca aagtcatcag ctgttcctgg 720
aacatggcgc catactgtgc ctcctcttgc tggctatcct cataccgctc acgccgggca 780
gctacttctt tgacgagctg ctcacactca ctctcagctg tgtgctgggc agccatggcc 840
tctgccagct cccgccgcag gacttcagtc tcagcctgat gggcctcccc aagctgctgt 900
aatcgagcct ccagcccctt gcgcccagcc ctctcttctt ccagctcctt tcgttcccgc 960
ttatgctgct ccaccaggct tcgggtctct gccttcagct cagagataca acgctgctgc 1020
tcttccaggg catctgcagc cctgcgcttc tcctcttcaa ggctgccctt ggtgaccttc 1080
aaggactctt tgagggcctg gagctgctcc tggagctggt ccttctcctg ggccaccctt 1140
tctttctcag ttgctttttg ctgctcagac cgcagctgca actctagctc tgcaacctgg 1200
gcctgggcct catgctgttc ctggcgggct gtctcaacac aggcctggag ttcctccacc 1260
ttccggctca gctctgcctt ctcccgctgg gcctgtgtca ctgaggtctg ggcactgtcc 1320
cgggcttcat tagccacctg aagttgctgc tgcagaatct ccagcttggc agccttctcc 1380
ttctccagtg cctccagctg cttgagagcc gcatcccgct cccttaagga ggcctctcgc 1440
tcctctgcag cagtggccag ttgctgggca tggtcctgcc tagttgcctc ctgcttctcc 1500
gctacctcct tcaactgctg ctccttctgc ttcaggctac tgcttagctg ctccacctgg 1560
tggcggaggc cctgggaggc ctgttcttgc tgttggaggg tctgtgctag ctgggcctgc 1620
ttctctttgg cctgctgctt caggccagcc agttcttgat cctgttgctg gatggtggca 1680
ttgagtgtgg tgagctcaga ggtcagagag gccacctggg cagtcaaccg ggccccatga 1740
gcctgggagg cctgctccag ctcttccttg gcctggctga ggttggagat ggagctctgc 1800

aggtcagtga tcaggctaga cagctgctgc ttttcttttt cgaagtggcc ccgctcagca 1860
agcagcttgg cttcctgctg gcctcgctca gtctccagca tctctaccct ggcttggagc 1920
tgtgtgttgt ttgcagcaag agtggctgcc tcttgcttca aggtttccag ctggtggtat 1980
aaagagacaa actgggatca gcatgactcc tcagtcatca agactcctct ggaaatggag 2040
gaacaccagg aacccaaatg tactgaatgt gagtccacac caaccagcag cacacaggtc 2100
tttctgtgca ttcctacctg caagacatca cccagcacct cgcccttctc ctggggtggg 2160
ttatcctgca gctgggacaa gtgttcttcc agctgtgaaa gttttccctg aaggatttcg 2220
ttcttctctt caaggcattt ctgggagtaa atgagaccca tgtgaacaga gagggggaac 2280
aggcaccaga ttaccacaca agccctcata aacatcagca aacatggggc ccatcatctt 2340
gttaccaaga tctccttttg ccctggccta gaaaaaagca gaaaccatgg aactgggagc 2400
atgagggacc tgattctcta gacctagttc taaggcacaa taaagtcata atgtaacctg 2460
cttactactt ccccaaggct gctgagttta cttcaaattt ccttaccgta tacccttcct 2520
cagataagtc ttgagtccta gattatccaa atcaccacca gctgcccacc atcctctggg 2580
cagcaatacc actggcagca gctactcgtt actactccca ttttagggaa aggatgctta 2640
gaaaaacaga ctgattaact tgtccaagat ccacatcttg tgaacagcag agataggatt 2700
gaaaggtgta ctataaagtg agggcattga ctggggaaaa aaaaaaataa gttaaaaaaa 2760
aaaggtgtac tagactgaag cccacaatct ttcaattaca gcacactatt gaaccttgaa 2820
gagaaagcaa cttaataccc aaaggcccag aaaagcacag tggtaaactg gactcagctt 2880
tgcacatgtg gtcaagactg tggctgtgac cccagcagag ggttaccttg tcctgcaggg 2940
ctgcgctgag ctccttctcc agctgggcct gcttctctag ccactcctga gtggccttgc 3000
tgtgctcctc cgtcagctca ttgagggcat cctgtagctg ctgcagatga ctggcaaact 3060
cccgcagctg agacgggcaa gtgagaatcc ccatcaccca gagtcaacgc tgaccccacc 3120
actgctgtct gctcccagaa ccccaggggc tggatgcctc caattatcct gccaaccctc 3180
tacaaaccaa tcacagccat cacacctcta attcagaact acaaatcaac atttagggtc 3240
acagcccagt acccgggaag ccgccaatta tgggataagg aaaggggcaa aatatgcagg 3300
tctctggaga gactgaagca taagacaggt gctgggacat tcttctcaac cctctcttgg 3360
ggagccttcc agaccacctc agccctgagg ggcctccatg cacacaccac cttaacaccc 3420
accttaaagg aaaggtctcc attctcctcc gaaagctggt tgattttgcg atccatctgg 3480
ctcttctctg tcttcaggtc ctggcactgc ttcagggttt catgcagccg catgcaagct 3540
gcttcttcag ccgtctcatc tggaactgtg gggtctgcag gatatcaccc atgggagaag 3600
ctggagaacc tgagagaaag ctgaggaggg agaaggccca tatgcattgg tctcatgagg 3660
aacctgaata caaggaacat caaactcaac ggctagaact cgcctagacc tccttcttac 3720
attcctatca tccaccagct gacctcttca tcagaagacc agcagaacga tcactctttc 3780
tactgtagta ccagccgagg ccattgatat attccgtact tagattcctt atcattattt 3840
cctggtttat gtatctgcct ttccacctag aacatgcact acatgacggg aaaaacattt 3900
tttaaaagac tgtgggccgg gcgcagtggc tcatgcctgt attcctaaca ctttgggagg 3960

ccgaggcagg tggatcacct gatgtcagga gttcgagacc agcctgatca acatggtgaa 4020
accccgtctc tacaaaaaat acaaaaatta gccaggtgtg gtggcgtgcg cctgtagtcc 4080
cggctgctcg ggaggctgag caggagaatt gcttgaacac gggaagtgga gattgcagtg 4140
agctgagatc gcaccactac actccagcct gggcgacaga gagagactct gtctcaaaaa 4200
aaaataaaaa taaaaataaa aagactgtat tctc 4234

<210> 74
<211> 4136
<212> DNA
<213> Homo sapiens

<400> 74
aggtatggag gtgtcccggg ggcggcgtcg cggggcgcct catgcccaga cttcaggggg 60
ctgtgcgtgc ggctggcggc ggagctggcg acgctgggcg ccctcgagca gcagcgagag 120
gcgggcgcgg aggtgccgag cgccggcgac ggccctggcg cggaggagga ctttctgcgg 180
cagctcggca gcctgctgcg ggagctgcac tgcccggatc gcgcgctctg cggcggggat 240
ggcgcggctg cgcttcggga acccggtgcc ggactgcgcc tgctgcgctt tctctgctca 300
gagctccaag ccacccgcct cctgtgcctc cgctctctgc tggatccgag tcctaggcca 360
cccccttggtg aaggggtagt ggagggagcc ggcatggtcc aagaactgga ccttaccctc 420
caagccctgg ggctgcccag acctgcacca gggacccccg ccagccagct gctgcagggg 480
ttgcatgcta agatctcaga gctgcagcct tctctgcccc cagggtccct gcagcccctc 540
ctcagctgct cgctagatgc acccagatgg gaagcgttgg agtctctgtc ccaaagcctc 600
agagatcagt accgctgccg ccgctgcctc ctcctcaagc gccttgacct cactacatct 660
gctttccact ggagtgaccg ggcagaggtg tggtgggcag gggaccatgc agaattggag 720
aggcccagtt gtgggggctg cagttgcagg gaggaggaat gagggccagg agtatcaccc 780
tcttgcaggt tctcaggcca tggacatttt ataatttttt gaggtttggg gctcctagaa 840
tctcagggtc ccagggtatt gttgaattcc cagctctgag ctggcagtat attcagaggc 900
ataccccatc attttttttt ttttttttga gacggggtct cactctgttg ccaggctgga 960
gtgtggtggt gcgatctcag ctcactgcaa cctccgactc ccgggttcaa gtgattctcc 1020
tgcctcagcc tcctgagtag ctgggattac gggcacatgc caccatgccc agctaatttt 1080
tgtattttta gtagagacag ggtttcacca tgttggctag gatggtctcg atctcctgac 1140
cttgtgatct gcctgtcttg gcctcccaag gtgctgggat tgcgggcatg agccactgca 1200
cccagccttt tttttttatt ttttattttt tgagacggag ttttgctttt gttggccacg 1260
ctggagtgca atggtgcgat cttggctcac cgcagcctcc gcctcctggg ttcaagtgat 1320
tttcttgcct cagcctcccg agtggctggg atttcaggca tgcaccacca cgccgggcta 1380

attttgtatt ttttggtaca gacagggttt ctccatgttg gttaggctgg tctcaaactc 1440
ccaacctcag gtgatctgcc tgccttggcc ttccagggtt ctgggattgc aggcatgagc 1500
caccacgcct ggccaagtgt accccatctt taagagatac gagcagagtc aggcacagtg 1560
gctcatgcct gttatcccag cactttggga ggccaaggca ggaggatcac ttgagctcag 1620
aagtttgaga ccaccttggg caacatggca agacccccgt ctctactaaa aattaaaaaa 1680
aaaaaaatta gctgggcatg gtggcgcatg cttgtccatc tcagttactc gggaggctga 1740
ggtaggagga tcacttgagc ccaggagatt gaggctgcgg tgagccacca tcgcgccacc 1800
acactccagg ctgggtgaca gcgagaccct gtctcaaaaa ataaatattt atttgagctg 1860
tgggcagtga ggtcttgaat ggtaaagtaa tgcttccaag atgacaggtc ccacgtgggg 1920
aagggctcca gggtaccttc tcttacctcc tccccactcc aggcccaagg agaggccatg 1980
agggcagtgc tgatcccaat tcgagaggtt ctgaccccag aatcggacat ctccattgca 2040
cacgttctgg ctgcccgagc cgacctgtct tgtctcgtcc cagccaccag cgtggctgtc 2100
cgcagaggga cctgctgtgc catcaacaag gtgggcatct ggggtaggga agggccctgg 2160
catccttggc cttcagattg gcctttcagc ctctgctcca gtccaagttt cagacttacc 2220
acctctaatc cacccactag acatggcagc tagaaggacc tcgctcacac aaaaactgac 2280
tgtggactcc ccagctcaga atgcttccgt ggctccctaa tgatctgaaa acaaagtcta 2340
gctatttact gagtccctgg ctacttctcc agcttgggct taaaatcctt ccctatcacc 2400
aaacactacc tatttgttgt tttttaacct tttttttttt caagacagag tcttgctctg 2460
tcacccaggc tgcaatgcag tggcttgatc ttggctcact gcaacctcca cctcccgggt 2520
tcaagcgatt ctcctgcctc agcctaccga gtagctggga ttacaggcac ccaccaccat 2580
gtccggctaa tttttgtatc tttagtagtg acggggtttc gccgtgttgg tcaggctggt 2640
cttgaactcc tgaccttgtg atctacccgc cgtggcctcc taaagtgctg ggattacagg 2700
tgtgagaaac cgcacccttt taaccattta aaaaaaaaaa acaaagacag aaataggcca 2760
ggcacagtgg ctcacgcctg taatcccagc actttgggag gccgaggcgg gcggatcacg 2820
aggttaggag atcgagacca tcctggctaa cacggtggaa ccccatctct actaaaaata 2880
caaaaaaaaa attagccggg cgtggtggca gacacctgta gtcccggcta ctcgggaggc 2940
tgaggcagga gaatggcgtg aacccgggag gcggagcttg cagtgagcca ggatggcacc 3000
actgcactcc agcctgggcg acagagcaag actctgtctc aaacaaacaa acaaacaaaa 3060
aacagaaata ccctctgttt tagtgattag gtatctagcc gcatgcctct gtaagtagtc 3120
ttatgtaaaa tgagtattag ggaataaagt ctaacacagc aactattgag gcaaaattta 3180
agttaagaaa aaaactgagc ccgaggtggg tggatcactt gagcccagga gcagaggttg 3240
cagtgagccg ggttctcgcc actgagctcc agcctgggcg acagagccta ggcgactctg 3300
tctcaaaaaa aaaaaaagaa aagaaaagta ggctgggtgc agtggctcac gcttgtaatc 3360
ccggcacttt gggaggccaa ggtgggcgga tcacaggtc aggagatcga gaccatcctg 3420
gctagcacgg tgaaaccctg tctctactaa agatataaaa aattagccag gcgtggtggc 3480
gcgcacctgt ggtcccagct gcttgggagg ctgaggcagg agaatctctt gaacccggta 3540

ggtggaggtt gcagtgagct gagatcacac cactgcactc cagcctgggc aacagagtga 3600
gactccgtct caaaaaaaag aaaagaaaaa agaaagctca tgctagtatc ttccaccagt 3660
tcagtccccc ctcccccagc cactgtactc aaatagtcaa catctgctgt ttcttcttca 3720
gcctcatgct gttcccttcg ctctctggcc cccttgtcca agatactttt tttctctgca 3780
actcaaatct cataaactct caggtgctta tgggcaacgt tccagaccgg gggggccgcc 3840
caaatgagct ggagcctccc atgcccacct ggaggagccg aagagaggat ggaggccccc 3900
agtgttgggg tcgcaagaag aagaagaaga agtaaagggg gactggtggt cgggggcggg 3960
gggtcctcca tgagatgcta atgctatcgg taatctctgg ggagtccgtt taaggctttc 4020
cttggtattt ggcacccaag ccccccatct cctattcctg agtccccaaa tgccctgctc 4080
ccattcacgt caataccaag aaccatgttc tccagagaat aaatttaatt tatgac 4136

<210> 75
<211> 3703
<212> DNA
<213> Homo sapiens

<400> 75
   gatgtgtttt ctgcctttct ggactacttt gcctctaaga tgcagaaact gagtacaagg 60
   gaaaggactc caaatcccca gtatgtcccc tgcaggagca gagttgaagg aggtcccacc 120
   aggtctggtg ggtggccctg ctcaggctta cctccccctt gtaggggtca ctgggaactg 180
   tcaactgcga aggactgtga tttccagtct ggtatggtct gctctggggt ccaagtgagg 240
   aaagcataaa tcgggcaact ctctcccagt gagaagatag aaggagcttc tacagagaag 300
   gacgagattg aggagctgcg ggccgagatg ctggagatgc gggacgtcta tatggaggag 360
   gacgtgtatc agctgcagga gctgcgacag cagctggacc aggccagcaa gacctgccgc 420
   atcctgcagt atcggctgcg caaagccgag cgccgcagtc tccgtgccgc ccagaccggc 480
   caggtggacg gcgagcttat ccgtggtctg gagcaggatg tcaaggtctc taaggacatc 540
   tccatgcggc tgcataagga gctcgaggtg gtggagaaga aacgggcgcg gctggaggag 600
   gagaacgaag agcttcgtca gcggctcatc gagactgagc tggctaagca ggtgctgcag 660
   acggagctgg agcgaccgag agagcattcc ttgaagaaaa gaggaacccg ctccctgggg 720
   aaggccgata agaagacttt ggtgcaggag gacagtgcag acctgaagtg ccagttgcac 780
   tttgcaaagg aggagtcagc cctcatgtgc aagaagctca ctaagcttgc caaggagaat 840
   gacagcatga aggaggagct gctgaagtac cgctcgctct atggggacct ggacagcgcg 900
   ctgtcagccg aggagctggc cgatgccccc cactcgcggg agaccgagct gaaggtgcac 960
   ctgaagctgg tggaggagga agccaacctg ctgagccgcc gcatcgtgga gctggaggtg 1020
   gagaaccgag gcctgcgggc tgagatggac gacatgaagg atcatggagg tggctgtggg 1080
   ggtcctgagg cacgcctggc cttctccgcg ctgggtggcg gagagtgcgg ggagagcttg 1140
   gcagagctgc ggcgacacct gcagtttgtc gaagaggagg ccgagctgct gcggcgctcc 1200
   tctgccgagc tcgaggacca gaacaagctg ctgctgaacg agctggccaa gttccgctcg 1260
   gagcacgagc tggacgtggc gctgtcggag gacagttgtt ctgtgctcag cgaaccttca 1320
   caggaggagc tggcggccgc caagctgcag atcggcgagc tcagcggcaa ggtcaagaag 1380
   ctgcagtacg agaaccgcgt gctcctctcc aacctccagc gctgtgacct cgcctcctgc 1440
   cagagtacgc ggcccatgct ggagacggac gccgaggccg gggactctgc ccagtgtgtg 1500
   cctgctcccc tgggcgagac acacgagtcc catgcggtcc gactctgcag agccagggag 1560
   gccgaggtgc tgcctgggct gagagagcag gccgccctgg tcagtaaggc catcgatgtc 1620
   ctggtggctg atgccaatgg cttcacggct ggcctccggc tgtgtctgga caacgagtgt 1680
   gctgacttcc ggctgcatga ggcccccgac aacagcgagg gccccaggga caccaagctc 1740
   atccatgcca tcctggtgcg cctgagcgtg ctgcagcagg agctgaatgc cttcacgcgg 1800
   aaggcagatg cagtcctcgg gtgctctgtc aaggaacagc aggagtcctt ctcatcactg 1860
   ccccccttgg gctcccaggg gctctctaag gagattcttc tggcaaaaga ccttggctca 1920
   gactttcagc cacctgactt cagggacctg ccggaatggg agcccaggat ccgagaggct 1980
   ttccgcactg gtgacttgga ctctaagccc gaccccagcc ggagcttcag gccttaccga 2040
   gctgaagaca atgattccta tgcctctgag atcaaggagc tgcagctggt gctggctgag 2100
   gcccacgaca gcctccgggg cttgcaagag cagctctccc aggagcggca gctacgaaag 2160
   gaggaggccg acaatttcaa ccagaaaatg gtccagctga aggaggacca gcagagggcg 2220
   ctcctgaggc gggagtttga gctgcagagt ctgagcctcc agcggaggct ggagcagaaa 2280
   ttctggagcc aggagaagaa catgctggtg caggagtccc agcaattcaa gcacaacttc 2340
   ctgctgctct tcatgaagct caggtggttc ctcaagcgct ggcggcaggg caaggttttg 2400
   cccagcgaag gggatgactt cctcgaggtg aacagcatga aggagctgta cttgctgatg 2460
   gaggaagagg agataaacgc tcagcattct gataacaagg cctgcacggg ggacagctgg 2520
   acccagaaca cgcccaatga gtacatcaag acactggccg acatgaaggt gacgctgaag 2580
   gagctgtgct ggctgctccg ggatgaacgc cgtggtctga cggagcttca gcaacagttt 2640
   gccaaggcca aggctacctg ggagacagag cgggcagagc tcaagggcca tacctcccag 2700
   atggagctga agacagggaa gggggccggg gagcgggcag ggcccgactg gaaggcagcc 2760
   ctacagcggg agcgtgagga gcagcagcac ctcctagctg agtcctacag cgctgtcatg 2820
   gagctgactc ggcagctgca gatcagtgag cgcaactgga gccaggaaaa gctgcagctg 2880
   gtggagcggc tgcagggtga gaagcagcag gtggagcagc aggtgaagga gctgcagaac 2940
   cgcctaagcc agctgcagaa ggctgccgac ccctgggtcc tgaagcactc ggagctggag 3000
   aagcaggaca acagctggaa ggagacacgc agtgagaaga tccacgacaa ggaggctgtt 3060
   tccgaagttg agcttggagg aaatggttta aagagaacca aatctgtttc ttccatgtct 3120
   gagtttgaaa gtttgctcga ctgttcccct taccttgctg gcggagatgc ccggggcaag 3180
   aagctgccta acaaccctgc ctttggcttt gtgagctccg agccagggga tccagagaaa 3240
   gacaccaagg agaagcctgg gctctcgtcg agggactgca accacctggg tgccctggcc 3300
   tgccaggacc ccccagggag gcagaagctg cccttcctcc tcatcctggc ccctccccag 3360
   cccccgccaa tactgtgaac ccccttccca ctcagcctgg tttcctggtg agggtcctgc 3420
   agtcatgggc cctgggggac ccccagggca aggcccatgg gagggaaggg accaagggca 3480
   tccttgggcc aactgtccac ctctcttgtc cactattctc tcctttccac ttctgtcttc 3540
   aaaaggctcc ttcctaggat ggatcgggtg ctaggacaac tgcagtccaa tccaccagct 3600
   ctccctgccc ctgtgtctta tttcagacat gagaataact gtacagtgta aacttataaa 3660
   gcgtttttaa tggttgtaga ttggaaataa agtatgtcat atg 3703

<210> 76
<211> 3711
<212> DNA
<213> Homo sapiens

<400> 76
   cttccctggc cagattcctg cctgtctccc agcagcctag acaggcccag gtcttgcctc 60
   acactggcct ctctacatcc agctcatgcc tcacggtggc ctctccaggc tcaactcctg 120
   tcccaggacg tcatctccgg gcccaaaact taaagtcaga ctctctagtc ccaactgctg 180
   cctcctggtg gattatgaag gcccaaaatc tcctcaagtt gacctctaca ggcccagctc 240
   ctgcctcctg tcagcgtcta caggcccaac ctctgcctta tgggggcttc tccaggccca 300
   cctcttcctc ttggctgggt ctacaggcac aactgctgcc tcacaacagc cttttttggc 360
   ccagttcctg cccagctccc agcggccctg gtagacccac aacttcccga agccaagctc 420
   cccaggccca ggtcaggcct cacggtggcc tctccaagct cagctcctgc cctccaatgg 480
   catctgcagg ccccaaatgg tctccagtcg gtgggctcct ccacgccaag cttgggcctc 540
   ttggcgacct ctgcaggccc aagttgtcct gaagtcggcc tctcccggcc ctgcctccca 600
   gcaagtaagc aagctctttt ggctcaacta ctgccgagct cccaaccgcc tttctaggcc 660
   ccgaactttc tccagccaag ctcttcgggc ctacttcctg cctcccggtg gcctgtacag 720
   gcccagcact ggttggagaa cagcctctgc gggccccact cttgcctccc aggggcctct 780
   ccaggcccag ctcttgcccc cacggcggcc tcccggggcc aggtccctgc ctgcctccca 840
   gcagcccgcg tgcggcccag ctccttctca cggtggcctg ttgatgccca actcatgcct 900
   ctggcaccct gcccagagac atgagcccct gcctctcact ggctcctccc acgctgagac 960
   aggtcagcgt gagcccttgc ctcacaccgg cccctcccac gctgggaggt cagcgtgagc 1020
   cccttgcctc acgccggccc ctcccacgct gagagaggtc agcgtgagcc cttgcctcac 1080
   gccggccctc tcccacgctg agagaggtca gcgtgagccc ttgcctcacg ccggcccctc 1140
   ccacgctggg agaggtcagc gtgagccctt gcctcacacc ggcccctccc acgctgacag 1200
   aggtcagcct gagccccttg cctcacaccg gcccctccca cgctgacaga ggtcagcctg 1260
   agccccttgc gtcacaccgg cccctccctc gctgacagag gtcagcgtga gcccctagcc 1320
   tcacaccggc ccctcccagg ctgacagagg tcagcgtgag ccccttgcct cacaccggcc 1380
   cctcccacgc tgacagaggt cagcctgagc cccttgcctc acaccggccc ctcccacgct 1440
   gacagaagtc agcctgagcc ccttgcctca caccggcccc tccctcgctg acagaagtca 1500
   gcgtgagccc cttgcctcac accggcccct cccacgctga cagaggtcag cgggagcccc 1560
   ttgcctcaca ccggcccctc ccatgctgac agaggtcagc ctgagcccct tgcctcacac 1620
   cggcccctcc cacgctgaca gaggtcagcc tgagcccctt gcgtcacacc ggcccctccc 1680
   acgctgacat aggtcagcct gagccccttg cctcacaccg gcccctccca cgctgacaga 1740
   ggtcagcggg agccccttgc ctcacaccgg cccctcccag gctgacagag gtccgcggga 1800
   gcccctagcc tcacaccggc ccctccgaca ctgacagagg tccgcgggag ccccttgcct 1860
   cacaccggcc actcccatgc tgacagaggt cagcgtgagc cccttgcctc acaccggccc 1920
   ctcccacgct gacagaggtc agcctgagcc ccttgcctca caccggcccc tcccacgctg 1980
   acaggtcagc ctcagctcct agcctcacac cggcccctcc caggctgaca gaggtccgcg 2040
   ggagccccta gcctcacacc ggcccctccc acgctgagag aggtcaacgt gagccccttg 2100
   cctcacaccg gcccctccca cgctgacaga ggtcagcggg agcccctagc ctcacgccag 2160
   cccctcccac gctgacagag gtcagcggga gccccttgcc tcacaccggc cccttccacg 2220
   ctgagagacg tcagcgtgag cccttgcctc acatcggccc cttccacgct gagagaggtc 2280
   agcgtgagcc cctgcctcaa caggccaccg tgggggagga gcgggcttgc acgcgggctg 2340
   ctgggaggca ggcggggact tgggcctggg aggtcaccgt ggggcgagag ctgggcctgg 2400
   agacacccct gggaggcaac agcggggcct gcagacgctc ttctccagcc ggagctggga 2460
   ctgttcaggc tactggtggc gggatgtggg cctgagggct tggttgcaga aacttcgggg 2520
   tctgcaaagg ccggcgggag ctgagccggg ggagcttgtt tgctgggagg caggagctgg 2580
   gccgggagat gcagccggga ggaacagctg ggcctgaaga ggcccccatg cgggaggcag 2640
   aggccgggcc tcctcaagtt ggcctctcca gacccacttg cagcctccca gcgtcctctc 2700
   cgggcccagc tcttcctccc ggctgcgtct ccaggcccga ctctggcctc ccaacaacgt 2760
   ctttggactc agctcctgcc cagctcccag cggccctggt agacccacaa cttcctgaag 2820
   ccaagctccc caggcccagc tcaggcctca cggtggcctc tcccggctca gctcctgccc 2880
   tccgatggca tctgcaggcc ccaaatggcc tccggtcggt gggctcctct aggcccagct 2940
   tgggcctccc ggcggcctct gcaggcccaa aacgtcccga agtcggcctc tccaggccca 3000
   gctccggcct cccggcggcc tttgcaggcc caagtcgtcc tcaagtcggc ctggaattag 3060
   gcctggaaga gcagcaagtc agcctctccg ggcccagctc catcctctcg gcggcctctc 3120
   caggtgcaaa acttcctcga gtcagcctct ccaggcccag ctcctcctgc ctcccattgg 3180
   cctctttcag cccagcccag cccagctcat ggctctcggc ggccttccca ggccccgctt 3240
   ttgacttttg gcggcctctt caggcccaga acttgccctc cagtggacct ttgcaggccc 3300
   ggcctcggcc tcggcctcac agcggactct ccacgcccag ctagctctca cctcactgcg 3360
   gcctccccag tccaaagctc ctgcctttcg gccgcttcgg caggtccagc tcctgcctgc 3420
   cagtggcctc tttaggccca gctcattcct cacaatggtc ttcccaggcc ccgtttttcc 3480
   cttctggcag cctcttggcc tctaatttgt ttatcttttg tgtataaatc ccaaaatatg 3540
   gaattttgga atatttccac cattatatga atattttggt aggtaattta tttggagtta 3600
   gtttctgcac catgcccgaa tttttatttt tattttcctt attatttggt gttaaacaga 3660
   tttaatgacg gtcatggcaa ctttttggca caatgaaaaa tatcgcccat g 3711

<210> 77
<211> 5937
<212> DNA
<213> Homo sapiens

<400> 77
tgcaggtaac aagggcaaca gcctgagcat ctcagagccc agaggcagag cgttagccga 60
ttgcttccag catcatctgg ggcacagtgg ggtcttggtt cctcaatggg cctgagtgga 120
tctaactctg cgaagttaga tcccaacagc catcacagtt tgcagacaat gtcattaaga 180
ccatccagat aacttcctaa ctccagtttt gtgcccacca agcatccttc tgatttcaaa 240
ttggcctcgc atgccatgtg caactgggag agagtgtgtg gacagaaatg gggccaattg 300
actatttccc ttggctgtca tatttttcat taataaacta actctccagc cacaaataca 360
cactcagaat gcctcttgct actccagatc ctccattcac tgtgaaggca atcatgggga 420
ttatgaattc catctcccag gtgtggatta aactgcatgc caggggaggt ttctgtggtt 480
ccaatctacc ccgcttagta catcagggct caacaggatc aggtcaaagc tggaaggatc 540
ctgagagccc acagaaataa tgactcctgt gctgaggttc acaggagtag cactggggtc 600
tgtgaattct tggcaaaaat tcagaaaacc taagggaatc catgcattag ctgataatga 660
ggccatacag actaactaaa gcatcagcca cctcattaaa ctgggaagct taatactgtt 720
tttattgcac aatcatttct aaatgtcttt tattaataaa attggggaaa tgaatttgtt 780
attctttaat aagtgcagtg tgtttagctg acaaaatttt tacaaagatg gggatcaatg 840
ggttgcaaga atactaaaag atgttcttgt tctgcagggt tggaagcccc taagccacca 900
tgcactaccc atcattttac aaaagaagga gaaactgagg aacaaagaaa cacatatttt 960
ccctcaagct tcagattccc tttaaactct taggatatcc cataaccccc tgtagcttat 1020
ggcagctaga ttcatgacag acaatctctc tagagtcaat ttggtttttc tcttaactca 1080
ctcaagcctc tgggaatgaa aggtctagcc cttgaaggct acttttggta gaagacgagg 1140
ttcagtatta aaaaggagga cagaggatgg aaaagaacac aactacatca atagtttctc 1200
cacattattt gatgttcaga acagtcccat gaagaagata taatattccc tttctacaga 1260
ccaaaaaatt aatatttgga gaggtagaaa gaccacccaa gggaacacat atttagacgg 1320

aaagcccagt tctgtctagt gtttaagtcc tggcccattc agctactctg ctctgtaact 1380
atcacccatt tcagcaccgc ggacagaggc agagccctca gtcttccctg taggtgggat 1440
ggaggcagag ggtggtaaga tgggtgctaa gtcccaggga agatatgtat ccaccaaagt 1500
gcctgaatga tgagagggaa gtcagagcta aggaaggaca catcatggac atctctttac 1560
atgtgtatca aattgcgttc tgtttagaac cattttctag cctcccacca aggacgtaaa 1620
caggacaagc actgtcatct gtaaagtgcc actcccagac tgccacccag agttcataaa 1680
aggctcagat gaatcaatag gtgggaaagt tatctggaat ttataaaaat tcactgttaa 1740
ggagacgact atcacagaca aaccccaaaa tcagcggttt aacacaatag acattttttc 1800
tgacttgtgt aaagccccag acagggatcg ctgcctgtca aaaggcctga ctggcacttg 1860
tcttgaggca cactctggtg cccaggcccc ttgctccttg ttgctctgcc acctctgaag 1920
tggcttctaa ggtcactgta tttatctgtg tcaagacaga ggaaaaaatc atgaataaat 1980
aaacccaggg agatttgtat gagccaagcc tgaaattggc acacatcact ttggctcata 2040
cgtggaggac tgggagtgca gaaagatgag gaaatgggtc ttactgaaca cagaaccaca 2100
gaactctgtc tccctcctct ccaaagctga gaaattgcca caatcagaaa gtgtgattcc 2160
catctgagag tttaagagca ggaatagatt aaagacaaat catgtaaaat accttgactc 2220
ctagacttgc cgaagcattc agcctgagcc atctttacat gtggataatc ttggatttcc 2280
caactgggct tcttgcacac tccatggtag aacgtcagag gaaatttttt tcaagcaaga 2340
gctgttagat catgagattc cccagaaaga tacagataca ggtatatgtc atattactgg 2400
agattctaat tcagctacct tcacaggcct gggaatgtgt ttttgacaca ggggcattag 2460
gattgtttca gctgcaagtg acagaagtct agctcacacg gtcttaagca agaaaggaaa 2520
tgtattgatt cctataagtg caccaggatc tagatacact gtcaggcaca gctggatgca 2580
gactccaaca gtctcattgg gatacccacc ctctcctccc atctctgacc atcccaggtg 2640
gctcccttca tgggtcaaag tggccaccag aagctccaga tacatctttt tatcaacagc 2700
cccaggagaa cttctctttc tcagtaaaat cccacaaggt attttttggc actaattgtc 2760
ttggcttggt cccattctta tccctgaacc aaccactgtg accaaaaacg tggtattctc 2820
tgattatcca agcctgattc acgagcccac ccatgagtct gatatgaggt ccagtccata 2880
caatctacac aagctaggtc tggggcatgg tggtgcccca aggacagctg ggtgcctttt 2940
cctagaagaa agggggcagg gaatgggtgc tggaccagca tcagcagcag aattcttagg 3000
cactagactg tggggggctc agagaggcat gggagccctg aggtccccac aaggtgtggg 3060
atagtctttc agaccttcag ggggggtctc tgctcactaa cctgctcaaa gcaccctggc 3120
ccacaccgtg ggcagctgga aggtgccagt ccactgaaca tgtgtgattg gcatgaatct 3180
cctgctttcc tttgtcagaa ggctaggagt ggatttgacc cgtttactca gaccctctca 3240
gtggccctgc tcccacaggc tcaccccagc agggcccagt gcttggctgc caatgacgcc 3300
aaggatatta gctgacagtg acttaaaaca ggggttcatg acctcagggt aaccgaggaa 3360
cccctgaatc tgaattcatc agtctgtgta tatgaatgtg agtgcctctc ctttccccac 3420
aggaaaaaat ctacaacttt catcaaatta ctcagggaga ctttattcta aagagactga 3480

aaacgaccaa catcaatttt gactcctatg ggcatctgta aatagcttca aggttttaag 3540
gtgaaatgtc atgtaccaaa atacacattc tggagaagca ggaagctaca gaccaacttg 3600
agatgaaagt ctcatatcaa tgttttccca agtgtgctcc ttggaataca ggttgacatg 3660
atatgatgcc cagcaaggga aacaaaacat attcatgttc aaattagttg gggaaatgct 3720
ggactaaata aggtttgatg ggattctttt ttctgcagga cttctcagaa ggggctagag 3780
taggcaaagt ttcccagatt tacctaataa aagcattgtt tctgtgggag tttcatttgt 3840
tattacatgt ttcctgaatg cagattcata gactatcctt tggggaaccc tcgtcctcac 3900
gggatgtatg ttcatggtgg tgtcttcgag tttgtgccct tgtgaagcat tctggcagca 3960
agcgtctgaa cacttccaaa agggggcgat atttaggaga aatcgctcag cctgaattag 4020
aacaaatgca gctgctggtg tctcttggtg cctgggagcc ctagagtgtc agagggagga 4080
gcgtgcacac tggaaccagc agcctggtgc tgcgtctcag ctctgtcgct aactggctat 4140
gcacctctgg gcatggcact taacccttct gagccccagc ccgccatctg taaaaagggc 4200
ttgatgtgag gattgaatga gatcatgcag ggaacacaat gtctggcact tggaagcgtc 4260
caccataaag agccaaggca gtagatggcc cagctgggtt tgttccaggc agagtttacc 4320
ctctgccctg gaggctccag gaaatgctgc cacgtggctc ctattgcctt aaccacatcc 4380
gacctgttcc tgacagctcc ccacatctcc agctcctttg ctggtgctcc aggcacctcc 4440
aaacgtggcg agcccctctc ccctgccctc ttgtggcagc tgacctggca ggagtgggac 4500
caagacatcc aaggcagctc ctttccacct gcatgggcac tttcctcagg acatccttgc 4560
ccctggcacc accttgggcc agcaagccac atggaaatgg atgcagaggc accactgttt 4620
gctgacaatt atacactgtc cttaaggtca cccttggcga tctgtcacca ggagcagaca 4680
aacccacacc tcaaccatcc catcagagct tgtttctatc tgcatctgtc atcgctgatc 4740
gcatttgaat gggtttagtc tctattttaa ataaaagatt tatgccttag ctgtcagagc 4800
ctgcctttat ttgaaaattt aatcttgttt ctaggagtct agattaactt attagattta 4860
ggcgtccctc gtgggtctct gagagaggag gagtagattc tcctccctgc attcggccct 4920
gcacacccga cagtgagagc caagagctgg atgggcttct ccatccagca cgcccaggct 4980
ggacagaggc ccccaactca ggcaactttg tcgggtaacc gtgtgtccag ggagtgcttt 5040
cttgcacgct ccgtctccgg gccagcttcc aggacctgtg ctcactgcaa gggacaccca 5100
tcgagccggc cctttctcag aggttttggg gaggcttcag gaaggacccc cagtgggggc 5160
ccagcttgtc aacatgggct gtgccaagga gttctgagtt tccttcaggt ctgtattgta 5220
tcttccaccc cctcagagct ccctccctac tgcttagacc acacgaagct gtggggctgt 5280
gggcagccag ttcacttccc tgaccttgtc tgcaggtgga gacagtggca gtgcccctcc 5340
cgggctgctg cgtcactcag tgtaaaagca gggaggcact ggagagctgc tgtctgcaag 5400
tttgttgcta cttcaaaagg tgcaggtggg ccctcacctc ctttagaggt gaggatgagc 5460
tacccaaagt gaaaaggagc ttctcagcgt cgcaatggag tcacggccag gctgcccaca 5520
ccagccgtcc ggacctgcac cagtgccacg ggtctgcccc catcttctct tccctctcct 5580
tcccctctcc ctctctctcc ctgtctcttt ctcctctctc catgctattg actgaatgtt 5640

tgaattccct gcaaatgcat tcctaacccc caatgtgact gtgtttggag acagggtctt 5700
taggaggtaa ctgaggttaa atgaggttgt aaagatgggg ccctgaaccg atgggactgg 5760
ggtccttatg agaagaggaa aaggggtccc tccccatgga gggacgacca cagcgaggca 5820
gcagccgccc acacgccaga gaaggggact cagagggaag ccttgcttca ccggcacctt 5880
gatcttgact cctagcctcc agaattgtga taaataaatt tctgttgctt aagcccc 5937

<210> 78
<211> 3879
<212> DNA
<213> Homo sapiens

<400> 78
cttttttaaa atcacaaggc tgacacttgg gagatgctgg ataactgcct cgctgatcaa 60
taaggctgtg atgggcacct ttatcttcaa ccttaataac ctcctgattc aagtccaggt 120
ccctatagca aggaatgagg acctctcggt gtgaggctct gccctgccta atgtgcttct 180
ttgcccagca tctgcccagg ccctgtaccg agtgctactc tacagagtga ggagggcttg 240
ggcttgcagt cttttaggat tacctgcagc tgtgggcacc catcccagac catgggtgag 300
tgggtgctgc attgtggcag aggccacagg gagattccca aagctgtaga gcccctaaga 360
gggagatgct cctccacccc ttgctgcctg cctcgcttat gccaccccag gcggcatgcc 420
cacatgctgg ccccctcagc tcatcaggct tccaagctcc tgcagctctc aagttccctc 480
ccacctcttt gtcagcacag cgactgccct agctcagcat tttcccttgc tgggccactc 540
ggacttgccc tcttgcctgc tctcttgtgc aactcagtct gttctccccc agcagctctt 600
gcacacacag atctggctgc ttgaacccca tcaggggctc cctggtgtcc tcaggttgaa 660
gtccaacagg gtcctccaag acctgccccc atcccccagg cttcccatct caccataacc 720
tggcccttct ctccccgtca gcacctagct gacccttctt cagtctttcc ttaggtatcg 780
cctcctccag aaagccttcc tagactgtga ttggaatggt ctagctgcac agcctctgtg 840
ctgctttcca caactccctg agcctcgttt tctctgctcc tgtggatggt tttaacattt 900
tctgtttgtg tatttgacaa gctcctggag aatgaagcat ccccggcttg aggacgggga 960
ctggctcttt cacttagtaa ttccagcaca tagtgctgtg ctcagtcagc ttcgggtctc 1020
tccttctctg tatgtgggtc tcccatttct ccctttctgt ctgttttgtc acccagccgc 1080
tgtcccccac cacatgcctc agctcccacc ctgccctgct tctctgctgc attcaccttt 1140
ccatgctttg cacctcccca cacttcctca gggatgcccg tttccccttc ctgccctcta 1200
cccaccacaa cccttccctg ctctctggcc tggcagaggc gcacctctct gctgccatcc 1260
ccttgacctt catggttttg ctcttcctcc tgtctctcct ccagctctgc ccccaccaga 1320
tctgcttatt tccaccctcc tgcccttctc atctctctgt gtggttgtct ccatctcctg 1380

tctctgtgga cgtctctctc ttctctttgt cccactgcta gtctctttct ctctttgtct 1440
ctcttgcctt ttctccgtgg cctctgtaca atgcacatat gccgactttg gcatggggga 1500
tgctgggggt tggggggccc tgccctgggg tgtcgtcttt cacctctgag gggtgatcac 1560
tgagggagcc acctgaacac aggctggtct gaggcctcta cgggctgcat ccttggctgg 1620
ggggccctca cctgagcagc tgcctcttgg tcccgacccc ctctgcaagt gtgtgtgggt 1680
gtgtgtctga atgtgagaat gagtgtgtag gactgtatgt atctgtgcat gtgtgtgcat 1740
gtgtggaaat gaaccaggga gaaggagccg cagacctggg cacagtgagg aaaccccagg 1800
gcagcctcct cccagagggc tgctccccta gaatctcctt ggggccatga tggcaaagaa 1860
gcccagtccc ttgtatagag ccagctggga cttgggaacc agatttccag ccttttctgg 1920
aaccagattt tccagatcct tccagtcgtt taggatctaa attttgataa cacctaatca 1980
gcagttctca aagtgtgggc cccaaaccag cctcagtttc acctgggagc ttttttgaaa 2040
tgcaaattat ggggctccat accaggcaga ttgattcaga aacctggagc tggggggtgg 2100
tgagcagcaa ggaggtattc tgacacccct tctgccccag tttgagaacc acggatttac 2160
atgataaggt gtggtgagtg ccctgtcaga taaatcatgc aaaacagata tgaaggagtt 2220
gttttcacct tccatctctc aggcaacata gctgaacaac atagccaaat tttcagcctg 2280
agccagaggc ttcttagatc tttccaccga agacaaggag gttacaagca tggcagggag 2340
caaaagcaag gcaagcggca ggtaacgtga aacagctcca gcaaggaca gtagcattga 2400
catttcatgc tttgatttaa aatcctatgc agatttagtg gctcctggga ccatcctgga 2460
cttcctgcac tcttccctct aagtctgaac ctgcagctgc tgccagtgac tggaagggca 2520
agtccctgat tgcctcctgg aagagctgct cctcaatttg gagagcaaga gtggggcctc 2580
tggaactggg aggaaggcct tggagatcca ctgacagccc acagcccctt cccagatgcc 2640
cctgggccag accagtcctg ggcagtgccc cccaggtcct gcttatccct gttcttctgt 2700
gtctgccccc ctcaggagtt ctctccttca gagatcagca gagagccttc tcattacagt 2760
ggggacccca ccaccaccac cccggttaca tcatttcatt ccctgcactg agctcccagc 2820
ccttaccacc tccttggggc taaatacctc caggcattgc cattgctgca tttcagcccg 2880
ttatatttgt aaagagcagg ctttggttgc cagggtctct tcttgatcaa agatgtttca 2940
aactcctgtt cccaagaaca gggcttatct cccaggttac cggttacagc tgtactcagc 3000
ttgtgcagtg ccttgggcga atgttttttg tgtgtgggac ctgtgtctat ataaacaatt 3060
tgattttaaa atgtaattaa aaattcttgg cctatatgag gtattatgat gtattgatga 3120
aggtttacaa taatgaggag aaaagaggta gtaacatatt tgtttatttt ccaatctgtg 3180
catgtgaaga ttctttggca gtgtccaaga attatctttt cttgttcagg tccaggaacc 3240
atctctttgt gttctttatt gtcaaatgtc actcctggct aatttcttat ctcccaccat 3300
gaggaaaaga tgacaatgct gctgttattc acaatgccat ggctcttagg gatctatttg 3360
tattaaaata atatgaatct ccttgcctct gcagttccct atgtccccta ctactgtgta 3420
tttaatgctg gttgcatctg tatttacatg tactgagtac tgaatattga gtactgagta 3480
cagaacaggt ggctggggca ggatgggaaa ggaaggttac agggaaattc ctcagaaact 3540

aacaaagaaa ctcatttaaa tgtgcttggg cagctcttta aagagatatt taccatccag 3600
atcactatcc caaattcttc cacagcctgg ggcatggctt ttagatgtca atcatccatt 3660
ccctcctctc tgcttccttc gcactttctt ccaaaagaac aaaatcctga ttaagtgctt 3720
tctgccatca tcaaaaatag gaattcaaga ttaggaataa taacatagct gggcacagtg 3780
gctcgtgcct gtggtcccag cactgtgggc ggctgaggct ggaggattga gtccaggagt 3840
tggagaccaa cctgggcaat acagtgagac ccagtctct 3879

<210> 79
<211> 4647
<212> DNA
<213> Homo sapiens

<400> 79
atttgctgac cgtggccttt tcggtccaag gtgggaggcc acagggagca gagggtcgga 60
ggtacagagc ccagaggccc ccaggaccca ggcaagctga agccctcgga cagcccatat 120
ccagcctctt ctttgctttc agataagaac tttaaaaaga aaacatccta cagaaaagaa 180
gctcagcttt tccccgctgc tggctgagtg ggtttcctaa gagcgggtat ttcctccacc 240
ccagcagcgc ttttcagcga ggagcatctc cgtctccacg gagggcagcc tggaatgggg 300
gaacgcagtc aggctgacat tgctgtgacc tccgcggctc ggggtgggct gctgaccgtg 360
accaccggct ccgaggaaga aagagcccag atggggagtg tctgccaata cctgtggtgc 420
aaatgctccc accggggctg gcttcgagct gccaagggga catgctgcag gcaggggcgg 480
gcaccctgcg gtgacaccca cgtgacccga cctcagacag acacgaccca cgaccaacct 540
ctggatcctg gaggagcgca cagggccgca gcgtgagagg cagtggcgcg tgctgtgttc 600
agcgcttctg tttttagtga cttctttatt tgtaagttct tatgatttaa tttgtttttg 660
agatatggtt ttgctccatc actcaggctg gagggcagtg gcgcaaccac agctcactgc 720
agcctcaacc tcctgggctc aagtgatcct cctgtctcag cctcccgagg agctgggacc 780
gcaggtacac gccaccacat ccagcttatt tatttattta tttatttatt tatttattta 840
tttatttctg agacagagtc tcgctctgtc acccaggctg gagtgtagcc gcgcgatctc 900
ggctcactga aagctccgcc tcccgggctc acgccattct cctgcctcag cctcccaagt 960
agctgggact acgggcgcct gccaccacat ttggctaact tttttgtatt tttagttgag 1020
acgggttttc accgtgttag ccaggattgt ctgtctcctg acctcgtgat ccgcctgcct 1080
cagcctccca aagtgctggg attacaggtg tgagccaccg cgcccggcct atttattttt 1140
ttatctgcag agatgagatc ttgctgtgtt gcccaggctg gtctctcact cctgtcctca 1200
agccatcctc cctcctcgga ctcctggcgt gctggggtta cagcatgagc ccccacgcca 1260
ggctgattta attttaatag ttctgtgtat gacggccagc tcacccgatt ccaggacatg 1320

aaggctcagc tcttaggagc tggctctgca caccacctgt gcgacggctg caattcacac 1380
cagaccccaa caagaccgag gctttggtct gaccgtgaag gccccggcga ggacagcacc 1440
accctcagcc ccaggcaaga ggggacctgt agttcccctc cagccccgct gaggcctgag 1500
ctgccttcgc aatcccatcc tgagtacctc aaggccctgg ggttcccgag gctcctgccc 1560
caaaggtccc acgagcacca cgtgtcttcc tcttccaagt cctctgcctg cggtgctggg 1620
cctggtgtgg gggcagccaa gggcgctctg tgcaggaccc ggacccagca tggccactcg 1680
gagtgcgggc ccggcatccc gaagccccct tcccgtgaga gtgaagccga aggcgatgga 1740
gggaggcagg aggcatggct gtgtgtcctc gggaagctga gatttctaac cttgagtctg 1800
aacgtccagg ctggtgtgaa gtctgtcacg gcgggagcac ggaggctact ctgcgagttc 1860
cacggggcat cagccggggc ggtgttattt aacgccagga cgtgcagtgt atgggcctct 1920
cttggtctct gggccctgcc tgcgttaggg catcggccgg gtcggctgta tttaaatgcc 1980
catgtagtgc ctgggcctcc acagagctct aggccccgtc catgacggtg caggcgtgtt 2040
ctcggtggaa acacagccgg tgaaggcggg aggaggtaag agtgagctct ccctgcctgg 2100
gagtctccga ggggtccaga ctgagggttg ggggtgcagg aggggaggcc ccagggctca 2160
gcggatccca ggttgtgcag ggttcagtgg gtttcattga ggaatttgga ctctgcccca 2220
ggggtgacgg gagccaacag gcagcggcta cctcctgcct cctctccgtg atccaggtgc 2280
tcagaggagc caggcagcat ggctgaggcc acacagacgt gcaggaggga gtgcagagca 2340
gggccccgtc gtgcctgagg atagaaggtg cttccttcct ggggcagcag cctccctgcg 2400
agaaggcggc tctccagggc tgcctggaac gcataaccga cggcttgcaa tctgcaccct 2460
gtgatttatt tatatggggt tgaagtactg caggggtccc gtgctgctgt gtcaggattg 2520
ggcccacgta gcgcgcagac catttgcctt gcatgaaaaa tgcatgatgg cgtcgggaag 2580
ccgctgggaa gtttcgcgac gcccgtgcag gggtcctcgc acggccgact ggctgtcagc 2640
gccccggtcc tgccgcccat gttgccaatg gatgagccaa atgtccctcg tgcgaggggc 2700
cttgtagatc ctgcagacac cgcgcctctc accagggagt aagggaatgg tcttcccctg 2760
ggacccacga gttcatcctc agctccacct tcacccacag gccacgggga aaccgcccgg 2820
ttgcaggggg ctcagcacac caccaagagc cttttaaaaa ggctttgggt aacagggagg 2880
aagaaagccc agttttaaac accaagtgtg gtctgcggga acttcctagg gcagcctgtc 2940
cagtgggagt gtctgtggtg atgggaatgc ccgtgtctgt gtgagccacg tggtggtcac 3000
tggtcacatg tggagacggg aatgcccgtg tctgtgtgag ccacgcggtg ggtcactggt 3060
cacatgtgga gacgggaatc cttgtgtctg tgtgagccac gcggtggtca ctggtcacac 3120
gtggcgggaa tgcccgtgtc tgtgtgagcc acgtggtggt cactggtcac gtgtggctgc 3180
tgagcccttg aaatgtggct cgtacagttg aggaagtgtg gcctgtgcag ttgaggagcc 3240
ggcttttaaa ttttatttaa ttttaatgaa tttaagtgta aacataaata gctaccatat 3300
cggaccatgt ctaggttgat gacatattgt cttttttttt ttggcttttt aaagtggttg 3360
tatcgttatt attatttaat taaatcaatt aatttttgag acagggcctt gctgtgtcgc 3420
ccaggctgca gtgcaggggg gtgatcacag ctcactgcag cctcaacttc ccatgctcaa 3480

gccgtcctcc caccttagcc tcccgagtac ctgggactac aggcacgtgc caccacgcat 3540
ggctaatctt gtactttttg tagacatgag gtctcgctat gttgcccagg ccggtcttga 3600
actcctaggc tcaactcatc ttcctgtctt ggcttcttga agtgttggga ttacaggcgt 3660
gagccacctg tgcctggcct atttttattt tgtaaatata aggggtacaa gtgcagttac 3720
atggaaacac tgtgtagtgg tgaagtcggg gctgtcagtg tagccatcac cagagagcgt 3780
acatcaaacg caccgggaac tttctcatcc ctcgccccac cctcctgagc atccacgggt 3840
gagtgttccc cactctgtgt ccacgtgcac acatcattta gctcccacct gtaagtgagg 3900
acacgtgctc tttgtccttc tgtttctgaa ctgtttgact cagcactatg ttctcccgtt 3960
ccatccacgt tcctgcaaag ggcacaattg tacacttttt catggctgaa tagtactcca 4020
tcgtgtatat gcaccacatt ctctttctcc agccgtctgt tggtggagcc ctaggctgat 4080
ttcgtgtctt tgctgttgtg aacggtgctg tgatggacgt gcacgtgcag gtgtcttttt 4140
gatgtaatca tttccttcga gtagctgccc ggcagtggga ttgctggatg agttctgttt 4200
tcagttcttt gataaatctc tttactgggg cagtgggatt gctggatgag ttctgttttc 4260
agttctttga taaatctctt tactgtgctc cacaggggct gacaatattc ccggtgaggg 4320
tttgggagac gcaggtgtgt gcacgtctca gaactcatct cacggaactg ttaagatctg 4380
tgaattccac tgtaaatttt acctaaaaca agagaactgt tgacagatat tggattctag 4440
ttaatacttc tgaaatattt gtattgaaat atactgttgt ctacaacttc ctgtgaaatg 4500
catccaataa aataagatgg gttgacggag gggtgggggg acggagaggt gactgggtct 4560
atggtgaaga aatgagccag tgttgatcgt agaatccagg cagaggtgcc gggcttcgca 4620
ataaagtgct gaagaagctc caaatac 4647

<210> 80
<211> 3633
<212> DNA
<213> Homo sapiens

<400> 80
ctgtcctcag caggtgccct ggccctgacc ccgaccccag ggtgggcgac acagtggcag 60
agcccttcag cttcctggtg gcttgcacgc cccacccaac ccctgtggct cgagccacag 120
caccctcccc tgagccttgc aggcatgagg acacccagaa cttcctgagg gctgtggagt 180
ttggaaacca ggggccactg gcaccagggc cccagctgcc accctctgtg cactggccag 240
tcctgggcca ccgaccaaca tggacgacca ccggcctcca ccccacacca gcctctgcct 300
ctgtccacta ggagggagcc cactgtcccc acaggccgtc cctgcccatc cccctgaact 360
cgccctcctg cttttgcccc ggcctccccc gatcgtgggt tcctgggggc aaggtctttg 420
ctactttgtt ccctgggctc agaaggtccc aacaaagtgg gggcagggag cccgcaggcc 480

cccaggccat acctgcacca gcgccgtgca cagcccaaag atgcccacag ccagcaggtt 540
ctcctgaagc cacaccttca ccgtctcgta gcacggctgc agggagggat gtcatgcgtg 600
tcacggcagg gcctgctggt cccgccctct aggcccctac cagcccccag ggtgggggtc 660
tcactcaccg ccttccacca ggtgccgggg gcgtgcagcc cacagctctc actgaactcc 720
aagcagcagg agtcaggtac ccgcgtggcg ttgtacacct cgaaccagtc agtgtagttg 780
gagacgccac agcagcggaa ctggggggca agctcaggtc gggctgaggc aggaggggag 840
ggggcacccg ccgaccccgc ccacctgccc acgcctcacg tcggtctgga tgatgctcca 900
ggcgttggtg aggcccacgt tgccctgcgt gccgtacagg tgcaagcctt tcttcaggtc 960
ttgctgggca tacctgtcaa tctgggggtg tggggggggt cagcagggcc cctcccactg 1020
taccccgcct ccctcatcca gaccccttcg ccggccccta agctgggccc ctcctgcgcc 1080
tggtcttgtg tcccccgcgc ctcctagccc accaagctgc agacacaccg gccccatgtc 1140
ctcccagcag ccctgaggcc cagggccccc acctctgggc ttcctcccca ggctggggga 1200
ggaccatggg tcctgggggg acagaggaca gggacatagg tgcctccacg tatcgggaca 1260
cgtgcagcca tgctctgtga gctgaaagcc aggggctatg agcgctgagc ccacccagca 1320
ccctggcctc ggtccccaat attcagtgag gacccagagc accctggaga aggggctgat 1380
tcctgggcca gggcagggaa tgaaggagcc tggagcgccc tgcagtgcca ggaagtcagg 1440
acgtgcccca aaatgaaaag tctgttgatg ggggcacgtc aggggaccca gcagcagacg 1500
gcagaagctc cccagggcca aggacggaca gtgtgattca caaaacaaag tagtgttgtg 1560
ttctagctcg gagtgcaacc tcaatagcca ggacttcatc ccaatataaa caaatggctg 1620
aatatatgag tgagtgacca agaagaggcc gctctacaga gcagaacccc acggtgcgcg 1680
gcccggatgg ggcgctggga gcagacggct caggacgctg cgtcagaggg cgggggacaa 1740
cctggggcgc accctccggg tggggtagca gtgcccttgt gccccagtcg ctatagagcc 1800
acagcacagc ctcccagggt cgctggcgct gcccctccag gcctggtaaa tccacacccg 1860
ctgtcctggc cgcccgtgag ggcacatgca gggccactct gtccacaccg ggcccacagt 1920
gctccctggg agtgaggatg gagcccccag ccctgcagtt gggcctgcgg ccaaggcagc 1980
cgtaccttgt ccgtgtaggc gaagaagagg atggcgatgg tggcctccag caggaacacc 2040
agcagcagca gcaggaagaa ctgtggaggg ggcaggctca gacagggacc cgaaaggccg 2100
cacagcctcc gccccacagc atgccagggg ccagggccac gtacctcatg gatacctggt 2160
gctgccagga aacctctgcc aggcccccac tcccagagag cccagaacgc cgcccccacc 2220
tcagaaccct tggccccagg tccagggact gtggctgttg tgtcctgctc agcccctgag 2280
tgtggggccc tgggctggcc tgggctcccc tcccctgccc ccatccctgg ggcaacccag 2340
acacccccaa gtgggcacaa atccctgttc ctgaggcccc agcccctgtc agctgccttc 2400
aggacacctc catccagggg cctctgagtg tctggcaggt gcctcgctgg gtgcctgtct 2460
tcatggtggg aagatgtccc agcaggggcc acagctgggc tgcagaggcc ccttctgccc 2520
acacgtcaca gtcctcaggg cagccagcgt ggctgatgcc ccctccagca agaccacagg 2580
ctgcaagccc cacctctgac catgcaagct tcccccgcct ggcggcagcc ccaaagctca 2640

gcctgacccc agccctgctg ctcctccatg cgggggtcag tgacacccac aaaagaggag 2700
ggcaggcatg gacagcatgt gtccaggagc tgggggggccc aggcagagtg ggcgtgagtg 2760
gggcagacag ggtgctgggc taaggacaca ggagtccttc acccccagga ggtacccagg 2820
gttctggtcc caagtcaccc atgggaaagg gggttgggct tcggtccctg caggcctggt 2880
ggggcccaca cacagcagct tcctcaactg aggagctcca agccccagta gggctgccac 2940
agacccccac cgactgcctc caggggtctc tgggccccaa cctgatgcca gggaggctga 3000
tagacttggg gcttgccccc aggaaggggt acggagggga gtggactggc tgaggcgacc 3060
taagggaggc cccaacccat ccagggcttc ccagacaaag gagtgatcgg aagcctcgtg 3120
atttaggacc tcagcaggct gcacaccctc cgcagggccc acggcctcgg ccaggcatgt 3180
taatcagtag caagcctgtg tttccatcta ggagcccttt tcctttttaa ttatctgtga 3240
ttaacatttc taaggaggat aaacatatcc ctgcctgtaa gtccatcccc gaggcgaggt 3300
gggggctggg atcgtcccag cagccggtcc cagggagctg agagaccagg cccccaacaa 3360
gaagctgggg aaggggccac agtgggggtg gggtggtcag gaatgagtca cggagaccgc 3420
aagcccgtga aaggctgcgc atgcgtgtga gtgggggtgg gaggcgaggt gcatgcaagc 3480
gggggtgcat gtgagccggg gtgcgtgtga gcaggcgtga atgtgagcca ggtgcatggg 3540
agcctggggt gtgtgggccc cgaggcccca gcgtgctggc agacccacac agataaaatg 3600
cgtgcacgtg gttaataaaa ttacctgcaa ttt 3633

<210> 81
<211> 5789
<212> DNA
<213> Homo sapiens

<400> 81
atacattgct gcataacaaa ctactacaca tttaacagct taaacagctt aaaatctcac 60
atatttacta tcctacagtc tctctggggt caggaatctt gggacagttt agttaggtcc 120
tctgcttcag agtctcttac aaggctgcaa tcggaggtgt tggtcagggc taggttctta 180
cccacaggtc cacctgggga gagattcacc tctgaggtca tgttgttgtt ggcagaattc 240
agttccttga agtttggact gggagcctca gttggctctt ggctggagac cacctgcagt 300
ttctagtctc gtgggccttt ctaacatagc agcttgcttc atcaaagtat gcaagcagaa 360
gacaataaga agagtcagct agcacaatgg aagctacaac cttatgtaac caaatcgtgg 420
atgtgacatc ccatcacctt tgctgcattt gttcattaga agcaagtcat aggtccaccc 480
agactaaagg gattacccaa aggtgtgaat accaggaggc catcttagac tctgcctgcc 540
acagctgggt tgcagccaga cctgcctacc tctgaaaccc aatgcactgc aaatagttgc 600
ctggagatcc tggtgagcaa cccagcttct tacagaagta agaattagtg gataagtttg 660

acacaaattg ttaagaatga tccagagtct tttgctcttc tagacttact cttcttgcag 720
gaagtgagaa caaatggact gtcagctgga atagaatttc ttgtatatca agaccagcat 780
cctacacaga gccctcagta aaactatttg catgaacaga ttttgggctc catcagctcg 840
attgccctac ttttataaca aatgtttata atatccttaa ttctaagatg aaatccataa 900
ataaaatgtc cctgcccaca taatttcaaa atatcaatat gatgtaactg taatatacaa 960
aaggaaaata attataacaa aaacaatgtg tctcaatata tacatgctct aaaataaagt 1020
ggttgaatgc ttactcctga actcagaatc actgctaatg aaatagttat aaatgcagac 1080
tgttacttga cactgggtgt ggaattaggg actcagattc tacaatttgc actggtggtg 1140
tgattttcca ggaagacaaa taatatttgg aaacattctg aacaaagcaa aacaaaaacc 1200
accccataat ttacaaggag ttacacttct ggaaaattca gcgtatgttc aaattgtgaa 1260
aaacagaacc ttgttttgga attagtttag tcttgagtaa ttataggtaa ggtttttatc 1320
tacacgtatg tcatgcaaga cattggaggg ccttgagata attcattata caggactcta 1380
ctgcatttaa caggacaaat gtgtgggacc tggcatttct ccctacacac tccccccaat 1440
tggggaatgc ctcaattttg caacaataaa aacgcccttg gatttcacta gtgagcagtg 1500
acacttgaca ttgcaaacca ctgcaatggt gcctaacctc taggtgagag gaaagcaatg 1560
cagttataga cttggttgga caccattaag ttcagatatc aaggagtacc atgagagaga 1620
tggaaatgaa aatcagtggc ttcaaagtat ctactgtggc ccagcagcgc aggcacagtt 1680
agggtgctta aatcaagcaa aaggtgcagt gtcagctagg ggtggagaga gaacactgtg 1740
ttagggtttt agaagtggac atctcaaagc agatactttg cacactgaat tttaagaata 1800
ctgatgaaga caggtaccat ggctcacgcc tgtagtccca acactttggg aggctgaggc 1860
aggaggatca cttgagccca aaagttcaag gctgcagtga gctgcaatca caccactgca 1920
ctccagcctg ggcaacaggg acctcatctc taaaaaattg ttttaaaaga atactgacag 1980
aatgctttct tttccaacag aaacctgttt ccctccaatc ttgtggttgc agctttccgt 2040
acgtatgcaa ccgattataa agtcgtgacc cagaacagca gctctggaaa tgtaacccat 2100
gaaaagatcc ccataggcac tgagatagaa gggatgaaca ttttaggatt ggtcctgttt 2160
gctctggtgt taggagtggc cttaaagaaa ctaggctccg aaggagaaga cctcatccgt 2220
ttcttcaatt ccctcaacga ggcgacgatg gtgctggtgt cctggattat gtggtacgta 2280
cctgtgggca tcatgttcct tgttggaagc aagatcgtgg aaatgaaaga catcatcgtg 2340
ctggtgacca gcctggggaa atacatcttc gcatctatat tgggccatgt tattcatgga 2400
ggaattgttc tgccacttat ttattttgtt ttcacacgaa aaaacccatt cagattcctc 2460
ctgggcctcc tcgccccatt tgcgacagca tttgctacct gctccagctc agcgaccctt 2520
ccctctatga tgaagtgcat tgaagagaac aatggtgtgg acaagaggat cagcaggttt 2580
attctcccca tcggggccac cgtgaacatg gacggagcag ccatcttcca gtgtgtggcc 2640
gcggtgttca ttgcgcaact caacaacgta gagctcaacg caggacagat tttcaccatt 2700
ctagtgactg ccacagcgtc cagtgttgga gcagcaggcg tgccagctgg aggggtcctc 2760
accattgcca ttatcctgga ggccattggg ctgcctactc atgacctgcc tctgatcctg 2820

gctgtggact ggattgtgga ccggaccacc acggtggtga atgtggaagg ggatgccctg 2880
ggtgcaggca ttctccacca cctgaatcag aaggcaacaa agaaaggcga gcaggaactt 2940
gctgaggtga aagtggaagc catccccaac tgcaagtctg aggaggagac atcgcccctg 3000
gtgacacacc agaaccccgc tggccccgtg gccagtgccc cagaactgga atccaaggag 3060
tcggttctgt gatggggctg ggctttgggc ttgcctgcca gcagtgatgt cccaccctgt 3120
tcacccagcc gccagtcatg gacacagggc actgcccttg ccaactttta ccctcccaag 3180
caatgctttg gcccagtcgc tggcctgagg cttacctctc ggcactggca ttgggctccc 3240
cagccggaac tggttaccaa ggacaaggac actctgacat tcggcttgat ccatgtccag 3300
gtgcaactgt gtgtacacca gggatctgtt tggaaacaac cccttgagct gccaggctca 3360
agaaatcatg gactcacagg gtcctgtgtg gttacatctt ggaaaaaatg cagatgtatt 3420
tcactctccc cggtcagctc tgcatcaggt gttttctgag caaaccaagg gggtttatag 3480
tcatctgtcg cattgcctcg agttgcagta attgaaaaaa tgctcaaatt cttagccatg 3540
gctggccttt gctgagctgg gactcaggtg tttaaagagt ttgtgctata gctaggtgtg 3600
gatagcttct gatccctggg ttctgggaga ctgcaggtgc cgcacattgt caagttagaa 3660
atactccagg tgggtgttag cactgtggtg gtctctggtc cacagcctta ggtaaacaac 3720
ttagattctg aggtcaaaga aaaaaggaga gggaatgcag ccttgtgggg gagaagcggg 3780
gcagagggtt ctctaatcta atcaggacag gacaggtttc acatacaatt gtcccagttc 3840
gcatcccagc cctggggcac ttttctgctt ccttccagag gcctgggcct ctgataacac 3900
tttggctttt tctccattca cgctgatttg gcaaaaggcc agagatgggc ctccttccct 3960
ggggaggtgt gatgtagtta tcacattcag gacccttgtt gatttatcat ctattatttg 4020
aattcaactg gacactctgt agaatgctgc actgcagcaa aaacaaaacc accaccaccc 4080
cagagaaaac catgtactaa ttggagtggg gtacccccat tcacaggttc ccaggtcccc 4140
tggctttggc tgatttcaaa atatagagcc ctttcttgcc agtacatcca agtttaaaat 4200
tatcagcgaa atggtccatg tttttccaat tacctgctga cacggttcta agctaagtga 4260
aggggaagat ctgagagcgt gctgtttgtg gctgttgatg catattcgtg atgtaacagg 4320
tcctggggcc tcactttacc ccatttgtaa aatggggcta atgtcacctg cctcttacct 4380
acctcagagg gatttggtga agcaaactgt taatcttcga aaacgaccat ttcacttctt 4440
ggatatcaag tgctaaccca gtatgttctt ctttttatg taaggacag ctttctccac 4500
agagtccttt ctgctggtga ggacagcatt tctgagcagg gctttgttct ctatgtgcat 4560
taggactttt atcatgccct tgttctgtgt gtagttactt gacagcatca aatgccgcct 4620
cttcctaatg tccttcaagt tttcatgaac tagcaacccc accttccacc atggttctgg 4680
gcgcctgatt ttgctgtgac tcccagaccc agccactgtt tctgccaccc tgtaacaggc 4740
cattaaagct ccccagtgtt cagcctcctt cactcccttg ttttccctgt tgctatgtgt 4800
cacctgggcc ctacagacag gggcacacgc ttatggatgt gtgtaccatt gagatgagaa 4860
tgggtagatg gaacggagac catcaagcca cacccccttc ttaaaactgg ggacatgagc 4920
ctgagcagaa agggtgaaga agagccatgg gacacagagt tgacccagcc agggggaaag 4980

cccagctctc tttaaaccag ctaagccatt ccagtctcct gtgaagccaa aagggaccag 5040
gaaccgtgca aaggaaactg gaaacttttc cccgctgggt agagcatgtt gctgatactc 5100
ttctgttttc aagggaaaca atcacattgt ttgattccaa atggtaaatg aacactcact 5160
attcttcagg cttcagtaaa tctttttttc ttccttcata tatatataca caacacacac 5220
acacatatgt atatctatac acacatgtgt gttgtgtata tgcatgtgtg tgtgtgcgtg 5280
tgtgtatagt tttagctcca agccaagcaa gtttgtgttt ggatagaggg gaacttaact 5340
attaactaca agttgtatgt ctgtggtatc ttgattttcc catttctaaa gatgaatttc 5400
acaaagccat aaagcgtgaa attagagctg gacttaagac tcattggccg accatcctgt 5460
gtcctggcct ggccctgcag taagaagcgt gtctgggtct ggagaagggt gcttccgaga 5520
gtgtgcaggt ggcccttccc cttggaggcg agaagagaga atgtgctgtc tatcttcctg 5580
gttttcagtc cacagagtcg gtagaccagg ggttacgtga ctggggaaaa tctcacatct 5640
ccttgtctga aaacatttcc cctgctgttc tctttctaac atgttgtggt aaatctgttc 5700
agatactgct catccgactg ttttgtacat gtgacaattg ccttaaaacc tagcacagtc 5760
ctcagaaatg aataccgtgt ttccactgg 5789

<210> 82
<211> 4762
<212> DNA
<213> Homo sapiens

<400> 82
   atccattcat ctacccacct acctacccat tcatccactc acccgcccat ccagtcatcc 60
   atcaatccac ccatgccaca tccattcatc cagccatcca ttcatctatc cccctaccta 120
   cccattcacc cacccaccca tccattcatc catcaaccta ctcatgcaac attcatccac 180
   ccacccaccc atccattcat ccagccatcc acccacacac ctacctattc atctacccat 240
   ccagccatcc attcatccat caattcatgc atgcaatatc cattcatgca gccatccatt 300
   catctaccta cccacctacc catttgtcca cccacccacc cgtccagtca ttcatcagtc 360
   cacccactta acatccattt atccagccat ccactcatct acccacccac ctacccatta 420
   attcacccac tcacccacct ttccatccat tcatccatca gtatactcac gcaacattga 480
   tccacccacc aaccccttca tccatcagtc cacccatgga acatccattc atccagccat 540
   ccattcatct acccatctac ctactcattc atctacctac ccacccaccc atccattcat 600
   ccatcagtct acccatgcaa catccattca tccaaccatc cattcatcca accatccatt 660
   catctaccca cctacctacc cagtcatcca ctcacctacc catctatcca tccatcaatc 720
   tattcatgca tctttcatcc atccacccac ccatccattc atccatcaat ccaccaatgc 780
   ggcacccatt catccaccca cccacttatc catctattca tctacccacc cacttatcca 840
   tctattcatc cacccaccca tccatccatc ttcccaccca cccatccatt catccaccca 900
   cccatccatt catccatcaa ttcactcatg caacatacat ccacctaccc aaccatccat 960
   ccatccatca tgcagacatc aactgggctt gtaattgttg aagactgtta ggtacagaag 1020
   catctataat gcacaggttc tcgattgtga aaaggggttg tgtacacacc aggaggcatc 1080
   agtgttgtgt gatgagtaag ccatgagata atgcatgttg tctactcaga caaaaatgga 1140
   tgagcagagg gtggaatgtg ggtgttggtg ctgagactgg aaccacatgt atgttggtct 1200
   ccatcctacc cagggccttt gctgttacag cccatttctt agcaaacacc cagatgaatc 1260
   agagatgcat ggatgtactc gcagccagca cattcctgtc gggacagaca tatagcccaa 1320
   gcatcttgac ctccaggtgg catgcctgca ccaccgtgtg caacctagtg gatcgtgagc 1380
   agctgggggt gcagctgcca gcactcaggg tgcctgagga gtgaacagtg gggggctgag 1440
   ccacaagagg gagaggcatt ggagggaggt ggtccagctg gaccctttct cgtgggaggt 1500
   gcagaacctg gtctaggacc actgaaactt gttgtgttgc caggaacaag ccagctcaca 1560
   ccagctggaa catgggcgcc atcctggagg ggaagcgcag tggctttgca ccctgtgggc 1620
   ccaaagagca actttccatg gagatgatcc taaaggctga ggaagggaac cacgaatgga 1680
   tttgtgggat cctgaaggac aactttgcta gtgctgacgt ggcggacgca aagggctaca 1740
   ctgtgcttgc tgcggctgct gtaagcccca caccctccca gctggtgccc gcagcagctt 1800
   agctgtgagg gtcacacatg tgggtggccc tctgtggccc cctctgcagg agcagagctg 1860
   aggtacatgg ggacactgat tgtccacacc tccacctcgc cgttcagcag aaacccactc 1920
   agctgagtgt gacactcgtg gtccagtgca gaagggtttg gggcagagtg cctgttccat 1980
   tcctctgtcc cacacttgtc cctttgccaa gctcccgaat gagcactggt tcctgccctg 2040
   ccatggggtg gcctcatgag ggcatcagga cacccagtga cccttcccac ccctgagggc 2100
   caggtgcatc atcctgagtc ctgcctcatc tccctccaga ctcactgcca caacgacatt 2160
   gtcaaccctc tcctggactg tggggccgac gtgaacaagt gctcagatga gggtctcacg 2220
   gcactcagca tgtgtttcct cctccactac cccgcccagt ccttcaagcc caatgttgct 2280
   gaacggacca tacctgagcc ccaggaacct ccaaaattcc cagttgttcc aatcctttca 2340
   tcatcattta tggacacaaa cctggagtct ctgtactatg aggtgaacgt gccttcccag 2400
   ggtagctatg agctgaggcc accgccagca ccactgctcc tgccacgcgt ctcaggcagc 2460
   cacgagggcg gccacttcca ggacaccggg cagtgtgggg ggtccatgga ccacaggagc 2520
   agctctctga agggggactc cccgttggtg aagggcagcc ttggccatgt ggaaagcggg 2580
   cttgaggacg tgttgggaaa cacagaccgg ggcagtctgt gcagtgctga gacgaaattt 2640
   gagtccaacg tgtgtgtgtg cgacttctcc atcgagctct cgcaggccat gctggagaga 2700
   agcgcccagt cccacagctt gctgaagatg gcctcgccct caccgtgcac cagcagcttc 2760
   gacaaaggga ccatgcggag gatggcgctg tccatgatcg agtaggtcct ggcaccagct 2820
   ggtgggggtg gagggccacc atcagggctg aatcctatgc tcagcagacc cacgtctctt 2880
   ccctgtgcca gtgggaggcg ttgtgtctgg agatgtgtgt ctgaatgtgt gagcatccct 2940
   gtgtcggtgg ctccacgcca tggccagccc tgtgggggtg ccacggtgac gggctgtttt 3000
   cagtgccacc ccagccctgt gggggtgcca cggtgacggg ctgttttcag taccacgcca 3060
   gccctgcttt ggcctttggc actggcctga agtgtctctg tgggagcctc agcaggggcc 3120
   actgtcaggg gtcctatcct agccatagtg cacgtgagtg acacctgcct gggcagctct 3180
   cacacccctg ctgtccaccc tgtctatacc agtgtgtctc aaaatgtggt ctatgcaccc 3240
   ccgggggtcc aagacccttt cagggagtct gtggggtcaa aatgattctc ttgataaccc 3300
   tgagactctg ttagccttct ccttgtgttg atgttggtgg atggtatgaa gacagggccg 3360
   tgcagaccac cagcccccag cgtgcagggc agcagtgccc ggcctgcttg ggggcatggt 3420
   attccttcac cacggtgtgc acttgcgggg atgcctgtct cactgaagaa tgcctttgac 3480
   aaagcagaaa agcaatgaca aattgcatta aatcttgctc cttgcgtact cacccctcga 3540
   atattctggg tcggaaaaca tgggaaggac actgatgtgt gtctgccaca gaccaaggca 3600
   caccgcttcc ccgcaagaag cgcttccccc agggccagag tagcaacaga atgcggcatc 3660
   ttcccaacct cctgccccat ttttgattgg aagaatgacc actggtatgt ggctgttcat 3720
   tctcctgaac acagcctgcc actttaagga aaacatatga cactatttgt tgctggcgaa 3780
   atttacattt tcaagtgaat agcagaattc tggacacttg ccaccaccac caagaccttc 3840
   atagcttccc ttaactttga gacatgggtg ttcagaggtt tttcacgtga gatggcgtta 3900
   gcagcgcagt tttgtgatac tgcctgaaga catgccgaca gtgcccagat ctcttctatt 3960
   ggtgagccag cttttcccac acggccaagt tctgatgttg aaccattgcc aggtgggtga 4020
   agatccattg acagtgagag gtgggcccgt gggcttcagt gcagccaggc gcagaaggct 4080
   ggttcatgag tgtccagctc cgccaggtag ctagctcacc acccccagcc tgggttcatg 4140
   tagttcaaat aggaagacca cgatgatcag aaaggctgct caaatactcc ttcgtccagc 4200
   cgcgtacctg ggggaggctg aatctccact cacttccacc aaggctgtgc agagcagata 4260
   ggggaatcca gcaaaggtgg aaaacagtgc catccttctc cccaactggt tttgttttgt 4320
   aaaataactt tttgtgacag tgttacttat tagtaacatg cagtaggttt gttatggtta 4380
   acaagttggt gagcattatt gagaggtgaa gccagctgag cttctgggtt gggtggggac 4440
   ttggagaact tttgtgtcaa gctaaaggat tgtaaatgca ccaatcaatg ctcagtgtct 4500
   agctaaagga ttgtaaatgc accaatcagc actctgtaaa tcagcactct gtaaaatcga 4560
   ccaatcagcg ttctgtaaaa tggaccaatc agtggtctgt aaaatggacc agtcagcagg 4620
   atgtgggcgg ggccaaaaaa gggaataaaa gctggccacc gccaggctcc ccaccagcct 4680
   gcagcgacaa cctgcttagt ttcctttctg tgctgtggaa gctttgttct ttcagtcttc 4740
   acaataaatc ttgctgctgc tc 4762

<210> 83
<211> 2393
<212> DNA
<213> Homo sapiens

<400> 83
acacacaaaa atagaaagat gttccatgtt catggattgg tataatcaat gatgttaaac 60
tttctatact acccaaagaa ttctacagat tcagtgcaat ccctatcaga ataccaatga 120
cattcatcac agaaattgaa gaaataattc taaaattcat gtggagccac aaaagacaca 180
gaatagccaa agctatcctg agcaaaaaga acaaaactga aggaatcaca ttgcctttct 240
tcaaattata ctacagcgct atagtaactg aaaaagcatg gtactgccat aaaaacagac 300
acgtagacca atggaacaca atagagaacc cagaaacaaa tccatacacc tacagtgaac 360
tcatttttga caaaggtgcc aagagtatac attggagaaa ggaaggtctc ttcaataaat 420
cgtgctggga acactggata tctatatgaa taataatgaa acttgacccc tatctcttgc 480
catatacaaa aatcaaatta aaacggatta aagacctaaa tctaatacct aaatctatga 540
aactattaca agaaaacatt caagaaaccc cctgagacat tcatcttggc aacaatttct 600
tgagtaatac cccacacaca ggcaaacaaa gcaaaagtag acaaaagggc acagtggctc 660
gcgcctgtaa tcccagcact ttgggaggcc aaggcgggcg gatcacgagg tcaggagatc 720
gagaccatcc tggctaacac ggtgaaaccc cgtctctact aaaaatacaa aaaaaaatag 780
caggcgtggt ggcgggcacc tgtagtccca gctactcgga aggctgaggc aggagaatgg 840
cgtgaacctg ggaggcggag cttgcagtga gccgagatcg cgccactgca ctccagcctg 900
ggcgacagag cgagactcca tctcagaaca aaaaaggaaa aaaaaaagta gacaaaaggg 960
atcatatcaa gttaaaaagc ttctgcacag caaaggaaat aatgaagata caacccacag 1020
aatgggagga aatatttgca aagaaactat ccatctcaga agggattaat agccagaatg 1080
tattaggaac tcaaacaact gtataggaaa aaaatccaat aagccagtta aaaaataggt 1140
aaaagatttt aatagagatt tctcaaaaga agacatacaa atagcaaccg ggcatatcag 1200
aagattctca acatcattga tcatcagaga aatgcaaatc aaaactacaa tgacatgtta 1260
tctcactcca gtcaaaatgg cttatatcct aaaacaataa caaatgctgg taaatatgtg 1320
gagaaaaggg agcccttgta cactattggt tggaattaaa ttagtacacc ctctatggag 1380
aacagtttgg aggttcctca aaaaaactaa aaatagagct accatatgat ccactatccc 1440
actggtgggt atatacccaa aagaagggaa atcgttatat caaaggcata tttgccctgt 1500
catatttgtt gcagtactgt tcacaatagc caagatttgg aagcaaccta catgtccata 1560
atggatacag aaaatgtatt tatgcatggt ggagtactat tcagccagaa agaaatgaga 1620
tcctgtcact tgtaacaaca tggatggaac tggaggtcgt tatatcaagt gaaataagcc 1680
aggcacagaa acacaaacat tgcatgttgt cacttatttg tgggatataa aaatcaaaac 1740
aatttaacat atggagatgg agactagtag gatgattacc agaggctggg atgggtcgca 1800
gtgggtgaag ggatgtggc aatggttaac gggtacaaat aatagaaata ataaatagaa 1860
cctagtattt gatagcacaa cagggtgact atagtcaaaa taatttaatt gtgcatttaa 1920
aaataactaa aagagtataa tcggggccag gcacggtggc ttacgcctgt ggtcccggca 1980
ctttgggagg ccaaggcgga tggataatga ggtcaggagt tcgagaccag cctggccaat 2040

atggtgaaac cccgtctcta ctaaaaatac aaaaattggc cgagcatggt ggtgcgcgcc 2100
tgtagtccca gctacttgga aggctgagtc aggaaaatca cttgaatctg ggaggcggag 2160
gttgcagtga gccaggattg tgccactgca ctccagcctg ggcaacaaag caagactctg 2220
tctcaaaaaa aaaaaaaaaa aaaaaaaaaa aaaaagagta taattggatt ctgtgtaaca 2280
caaaagatga atgcttgaag ggatggattt gggattatta agtattttgt gcccttatca 2340
atatatctcc tgtactccat aaatacatac acctactatg tactcacaaa gtt 2393

<210> 84
<211> 3651
<212> DNA
<213> Homo sapiens

<400> 84
   gcgcctcacc ttcagcagcg cctaccaggg caacagcctc atgtaccatg acagcccctg 60
   ctccaacggc tatgtctaca tccccctggc cttcctgctc atgttgtacg ccgtctacct 120
   ggtggagtgt tggcactgcc aagcccgcca tgagctgcag caccgtgttg atgtgagcag 180
   tgtgcgggaa cgtgtgggcc gcatgcagca agccacgccc tgcatctggt ggaaggccat 240
   cagctaccac tatgtccgcc gcacccgcca ggtcaccaga taccgcaatg gagacgccta 300
   taccaccacc caggtaccag gctgcctagg aagccaggtg gtgcctttaa gatctccatg 360
   gagaaacgca cagctcgacg caggatccga cgaagaacca gcctaaaggg gttcagagcc 420
   cagacatgaa cccccagcgg ctcatgccct tccagactca cgggggacct gacatcccag 480
   ggaagacgcc atcagagatg cagacactga gtgtgcggat gtggtcagcc accacgcggt 540
   acgctgtgtc tgtgcgcccc tcgtctgcca cccctactcg gcccaagtaa gggggtgccc 600
   tgcagccctg ggaagcagaa gagtcagcca gtggccctgc ctgacctggc ccaggtgggt 660
   gctctttatc gcctctagag catctgccct cagccctaaa gccaaccaca tccagaatgg 720
   ccttgccagt ccagtcctgg gctgagaagc ctcacagatg agcacaagcc aggccctgcc 780
   ctcacggggc tcatatttgg tgctacaagt gaacatatct gtgaccattt actgaatttc 840
   tactatcaga actgggtgct aattgcagtg attttctgga atctaaggga tgttttgcca 900
   acacaacacc ctctggcaaa agtgtcagtt cctcatcttc agtggaaaca ggcccaagtg 960
   tcagtgagcc tcctggcctc cccagagtgt ctgcttacgt agacaccact gctgacttgg 1020
   atcggaaact ctccttctca cattctgatc actcctctga aatgtcgttg cctgaagtcc 1080
   aaaaggataa atatcctgag gaattcagcc tgcttaagtt gcagacgaaa gatgggcatc 1140
   gtcctgagtg gacattttac ccaaggttta gcagcaacat ccacacctac cacgtcggaa 1200
   agcagtgctt ctttaatgga gtcttcctcg gcaacaagag gtctctatca gagaggacgg 1260
   tggacaagtg ctttgggaga aagaaatacg atattgatcc caggaatgga atcccaaagt 1320
   taactccagg cgacaatcca tatatgtacc cagaacagag taaaggcttc cacaaagcag 1380
   gatcaatgct cccaccagtg aatttttcaa tagtgcctta tgaaaagaaa tttgatacat 1440
   ttattccact tgagcctctt ccacaaattc ccaacttgcc tttctgggtg aaggagaagg 1500
   ccaacagttt gaaaaatgag atacaagagg ttgaggagct tgacaactgg cagccagcag 1560
   tgcccttaat gcacatgcta cacctttctg gtgctttgga ctttccaaga caatcctgag 1620
   cataaacagg cccacaaaac atgtgctgac tgcactctgg cgaccctttt ccagttgatg 1680
   ttttttgtca tgtgactgtt ctaaatccag tgtttgaccc ttatgaggaa gtgttgtgct 1740
   ttgctttttt aaaactttat attttgaaaa ctttcacatt tacataaaag ttgcaagaat 1800
   tgtacaacaa acacctgcat atccttcaca tagattctat cattgttaat atttgttcaa 1860
   actttctctc tctcacaata ttacagttac cattttgctg aactctttga gagtgagtgg 1920
   cagatattgt aaccctttac acctgtatac ctacatacct cagcatgtat cttctaaggt 1980
   caaggatgtt gtctaacaac cacaacacaa ataccaaact cacagaattt aacactgata 2040
   aaatattatt aacatttagt ccacattcaa ttttagtcaa ttatatttag ttttctaagg 2100
   atgccctcaa atcaatcata aaatacagac atgatgtgag tgtcttagcc tgttcaggct 2160
   gcaataacaa aacgccataa acagaatggc ttataaacaa taggaattca tttcttacag 2220
   ttctggaggc tgggaagtcc aagcgcaggg tgctggcagg ttcagtgtct gctgagggcc 2280
   agccttctgg ctcatagata gtaccttctt gctgtgtcct catatgatag aaagggtgag 2340
   ctagttctct ggggtctctt ttacaagggc actaatctca ttcatgaggg ctctggtctc 2400
   atgacctaat cgcctcccaa tggccttacc tcctaatacc atcaccttgg ggattaggtt 2460
   tcgacatatg cattttgggg tggggaggac aaacatttag accatagcag tgaactttca 2520
   ttgcttttgg taaaactttt cattttatta ataccttttg gaatgtttct ctaatgttaa 2580
   cttggtacat gtcagaaaaa catgaaggct ggatctatac caggtaagta atactattgc 2640
   cctgtagctg cattccccag tttagtattg cctatctaca atagccaagc agggtgagag 2700
   ctcagtgacc accagtcatg agttttaggg cctcctgtca atcagacaag aagcaaacat 2760
   ttggaggaaa aggtgaaaac aaaagctgcc tcacaggctt tgacacagtc aaactagtga 2820
   ctcacggcat ttttacagcc aatctctttc tccactaggg aacccccttg gctggctaat 2880
   gaaggctgca gatctagtat ctccaaaacc ttaaagtgga aagagattgg ggtccatgct 2940
   gagactcaac atgtctatga catgagcatg atctatttcc caaaatgttt gttgtctcca 3000
   gtcacagcca aagtgaatga agcaggttta gaacacgata catttagcca ggtgtggtgg 3060
   ctcatgcctg taatcccagc acttttggag gccgaggcag gcgaatcact tgaggtcagg 3120
   agttcgagac cagcctggtc aacatggcga aacctgtctc tactaaaatc acaaaaatta 3180
   gccaggcaag atggtgggca cctgtaatcc ctggtactcg ggaggctgag gcaggagaat 3240
   ctcttgaacc caggaggcag aggttgcagt gagccaagac catgccattg cactccagcc 3300
   tgggcgacaa gagcgaaact ccatcccaaa aacaaagaag caaacaaaaa ccacaatgca 3360
   ttcatggcac cttcatatgt gcctctagac taactggcag agagcatggt gccgactgcc 3420
   agctgctgtg cctgaccact ccttgggtta acatcagcat acatccgtgc taatcctctg 3480
   gcccctgggt cacaggccca cccctgctcc caaagtgctg ggattacagg agtgagacac 3540
   cgcatcaaac tttttcacct aaagtgaatt gtccttcaag tctaggctcg gagcttccca 3600
   cataaattgc agagtctctg gaatctttga gaaaatatat acctttattt t 3651

<210> 85
<211> 5840
<212> DNA
<213> Homo sapiens

<400> 85
   gcgatcccca ccacaccacc aacccggccg cacggggcac tgagccgggt gctgagcacc 60
   ggaggccccg ccgaggccgg gactcaggta aagggctgga gcttgcgggg agggttccaa 120
   aggtgggcag cagcccctgc tgcccagcgt cctagaggca agaagtaact ccgactcgga 180
   gcagtagaag ggggcggtgg agcagcatgg aaccgggctt ttgtctgggg tgtatgccgg 240
   gggcttcgag tgtccacggt gcgcccgccc ctcgccgccc cctttttgat ggggtgcttc 300
   tacaaagtgg gcaagttttc ccgggaatcc gaaggcggca ttttgtacgg agttgaaaga 360
   gagggagggg gtcctggggg tggtacggtg gccccccaga gagctgcctc tggactccag 420
   agagcttcca gggttgctgg tggaagagca gggagaggtg catgtgatgc aggtgggcct 480
   ccatttccca gaggaccgaa aggcctccaa ctgccttggt ccggagcgcg agcaaccagg 540
   tgcccccgga gggttgggaa tggggcgccg cgggtactcc tgacctacgt ggagcgcagc 600
   gcgcagggcc ggcggattct gcacgttggc gccaggttca gcaccacccg gcagccctgg 660
   gcgagcgcgg gagcccagcc ttcgccaccg ccgcaggagg caccggcacc gcgcccacct 720
   ggaccctcag ccagaacttg ggaggacttg gtgcttaatg ccccatcttt ggttcgccct 780
   aggtaacctg ctctggcttc agcagagccc agaggctttc cagagagctg ataaccacga 840
   acaaacttgg ctgtcaaaag caaatcaatg ctttaccttg ttttctgggt gcctagtcta 900
   tccaacacaa actatccccg aagtttagtc aggatttacg ctaaatatat tacttatata 960
   gtaagttatt cgatataaca tttttggtca attttctagg ctacagtttt gatgtgtttt 1020
   ttaaaaatct tgaattaact gaataacagt tcatattgaa ggtggattct cccattccct 1080
   gcacccagga ttttccgttg ttccatcaaa ttctgtaaat cggtatgccc taacagtttc 1140
   ttatatttga gttggcttca gcttcgagaa attcttcagg caggtttagg cagagggctg 1200
   aggaagctag agaagattcc gaagagaaag gatgattatt tgttgcatga ttatagcttc 1260
   ttttaaaact tctaatagga aaacccttcc actttcctgg accaaatgca gagtaaagtc 1320
   attttctatt atctgttgca ataattaata ataaatatgt aaatgcagaa aacatggtta 1380
   gtacccatat cattgccaga aaccagtccc gctctccctc caccatcaaa aaaaagtcct 1440
   cgcaagtagg aattagataa aataatttaa cctgcaattt tgcaacaact cagactttca 1500
   gcaccaccga ccccctaata acatccctac tggtttcttc cattttttgt gtaggcaagt 1560
   ggaataacag gtatccttca tatccatagg aagagcattg ggaggagcca aagatatggg 1620
   gaggtagatc aagacattag gaaatctttt ctggcttctg actttgagtc tgaaaaggtg 1680
   gtgtgaattg gttggtgtct gaatctttat caggacacat tatggtggtc tggggtggat 1740
   ctggccatgc aactcaagct agaggatgac aactacagag atgtggtcac attttcaagt 1800
   tttacttcct tacctcaatc tttcaaaccc ctaattaatt aggtaataaa aattgtatat 1860
   atttatgatg cacacaacac agtattttga aacagtaata cattgtagaa tggcccaacc 1920
   aacataatta acctatatgt attacgtcac atttttatct tttgtttttt tttttttggt 1980
   gtggtgagaa cacttcaaat ctaccatctt aacaattttc aaaattgtga ctatagtgac 2040
   tatagtcact atgttgcaca atagatctct taaacttatt gctcctatct aactgaaatt 2100
   ttgtatcctt tgacaaacat ctcttcaccc caaccctaac tactggtaac cagggttcta 2160
   ctctattctc tgagttcaac aattttagat tcaagtctcc cttcccttcc cttcccctcc 2220
   cttcccctcc cttcccttcc cttcccttcc cttcccttcc cttcccttcc cttcccttcc 2280
   ctcctctccc ctcccctcct ctgccctccc ctcttttcct tccctctttt cttctctctc 2340
   ttctctctct ctctttcttc tcactgcagc ctcgacttca ccaggctcgg gtgatcctcg 2400
   cacctcagcc tcccgagtag ctgggactac aggcgtgtgc caccacaccc aggtaatttc 2460
   tgtgtttttg tagagatggg gtttcgccat ggttcccaag ctggattaca ggcatgagcc 2520
   actgtgccca gctgacttcc cttttttttt ttttttttga gatggagttt cactcttttt 2580
   tttgagatgg agttttgctg ttgtcaccca ggctggagtg cagtggctca atcttggctt 2640
   actgcaacct ctgcctcccg ggttcaagca gttcttctgc ctcagcctcc cgagtagctg 2700
   gcattacagg tgcgtgccac catgcctggc taattttttg tatctttagt agagatgggg 2760
   tttcaccatg ttggccaggc tggtctcgaa cttctgacct caggtgatca acccgcctca 2820
   gcctcccaaa gtgctgggat tacaggcgtg agccaccacg cctgactgat ttccttcttt 2880
   ttcaagtctg aattagtctc ctattgtgtg tgtgtgtgtg tgtgtgtgtg tgtgtgtgtg 2940
   tgtgtatcac atttcttcat ccattcatcc acttatagac atttaggttg attccatatc 3000
   ttggctatgg tgaataatgc tgcaaggaac atggaagtgc agctatctct tcaatataat 3060
   gctttccttt gggtatatag tgagattgct ggatcatatg gtagttccat ttttaatttt 3120
   tctgaggaac cactgtattg ttttccatca tggctttcca agatagtatc acttaatgac 3180
   ttcaatttta attaatgatt gcaaaaatgt atgtattttg agctacttag ccttttcttc 3240
   tggaggttct tggcagacac caaagtaacc cttatggatg atgagactga actataacaa 3300
   aacaccaagt tcaaaaaaaa gaacacatgg tccctgcctc caaatgttta gtgagaaaaa 3360
   tagatttaca aatatctgca ttacaagata gtataaatgg agtcataggg tagaacatca 3420
   atagtgtgct actgaatgac aaaggaaagg agaggttcat tcaaggctaa gtgtgaagaa 3480
   aagttgtggg gtgggcgtag caatttagga agaggatttc cttgatggaa aagaggcctg 3540
   ctggaacaag agaatagcac agagataagg aagtctagtg tgtttatagg aagcaaagca 3600
   gtttggagta gctgaaacct agaatttaag acagataagg ctgggaggtg ggatggggct 3660
   agattataga gactatgaca tgtcatgcta agaaattaaa atatgattga acattattgg 3720
   tatatttaca taaacagtat caacatcctt aactgtactt tttgccttta atttcagttt 3780
   tatgagtttc cttctaaaat aagtagttga ttaaaatctc aatattcact tcctatcagt 3840
   ttctttcaat cagtaaaaga ataatgtatt cctaatatcc ttcaatgctt tatctaaatc 3900
   caacagtgtt ttacccccaa acctgcattc ctatgatatt aatagctctt agcttgatta 3960
   aaacttccga tagaaaaaca cttccacttt cctggggcaa tatgcagtgt atagtcattt 4020
   tctattctct gttgcaatca ttaataataa taattaatgt ttattaaaca tgttatgtca 4080
   agggagccac atagccttat gttatctagg gaggcattat tttgatctta gactgagaat 4140
   ttttacaagg tcacacaact aagtaaaggg accaaggagt ctacccccag agtctgtgct 4200
   ttgaactatt gtactatcat gtaacggggc atagtgtatg ataattattg ttaatattag 4260
   atagaaactg ggcctgattc ctgtagcagg actgtttctc tgtttttttt tttctttgca 4320
   tcttaatttt catcatttgg attaatttta tcttacactt tcagtgacgg gacatatttt 4380
   aatgttttaa tacactttta aactactcat gggatctcgt atttttatga cccacccaaa 4440
   agaaactctt aaactctgag aagagacagg atgtgggacg aaaaaaaatg ttagcaacag 4500
   tgatggcaca agatgtgaag accaggcctc tggaagaatg ttgattacca atattctcat 4560
   gattcccaat aaactcccca aatcaccaga tctttttgca tttcagcatc ccatatattt 4620
   ttctgtgtgt cttgattttt ttattatgaa ggtaatacaa taaacatact actttgtaca 4680
   tgacatacta aagtatgtat ttagacaaca gatcaaacat cacatgatca tgtataaatt 4740
   gtgcaaaacg tttgctaact gaaagaacac gtcagtgtta ttatcataga tttcttcacc 4800
   agaacactat tctcaggcca agctctgcag gagagggttg tgtggagtag agagaagctg 4860
   aaatgagagc ctggcagtcc agtgaccctg gtaacagtgt gggacacagg gagatgatga 4920
   taccaatgta tctcagtgtc tggccctcac tctgcctaaa tttaactgtt acatctttat 4980
   ttttgtattt actttgttaa atatttggac ctttccatct tttctcaggc tctgagtggt 5040
   ttacagattc tgctattttc acttttgagt ttgagagtag gacttagaaa tagatgactc 5100
   ttcaatggat tgttgagtaa aggatcctga aactgttctc tcctttagca tgacgtctct 5160
   caacttcagt ttcctcatgg gtagattgag ggattgaata agataagtgg tggcaatcac 5220
   aaatcttcac aaggtttacc cggatgagac aggcatcata aatgagcaaa gaagactggg 5280
   ggcagtggtt gggtagtagg tgtgtggagg tatgggggat agcacaggtg aaactaaatg 5340
   caaggcaacg ttagtcctgc gtcagggaga tattagggag tagaaggacc tggagagaaa 5400
   atgcctcatc caatgagagc cagacttact cagttccagt taattgttat gtaagaatga 5460
   gggcccagtg ttatcagaac tttcaatgtt ttgaaagaat cctaaagttc agatttttat 5520
   gtgaaatcta ttctagtgtt aaaacatcag caatttggcc gggcgcagtg gctcacgcct 5580
   gtcatcccag cactttggag gcagaggcgg gcggatcacg aggtcaggat attgagacca 5640
   tcctggctaa cagggcgaaa ccccgtctct actaaaaaaa taccaaaaga attagccagg 5700
   catggtggcg ggcacctgta gtcccagcta cctgggaggc tgaagcagga gaatggcgtg 5760
   aacccgggag gtggagcttg cagtgagccg agatctcgcc actgcactcc ggcctgggcg 5820
   acacagcaag acttcgtctc 5840

<210> 86
<211> 3992
<212> DNA
<213> Homo sapiens

<400> 86
   atcctccgcg gacgcccgct gccatggcga ctctgctgcg ccctgtcctc cgtcggctct 60
   gcgggctccc gggcctacag cggcctgcgg cagaaatgcc cctccgggct aggagcgacg 120
   gcgccggccc gctatactcg caccacctcc ccacctcccc gctgcagaaa gcgctgttgg 180
   ccgccggctc cgcggcgatg gcgctctata acccctaccg ccacgtcttg cctttcaggg 240
   agcgtccccg gatttcgaca tccaccctcg acctgggcaa gctccagagc ctgccggaag 300
   gctccctcgg tcgcgagtat ctccgtttcc tggatgtgaa cgtgagtttt cagctcctgt 360
   gtatctggca gtcaccagac aggacagagg aatagcacag gcatgacacc ctgaggaaag 420
   aggagccctg agctgccacc attggcagga gtgctcttca ggctcatgcc aaggctttgt 480
   cattttctat gggataggca aatgggtggg aacatgcctg caattgataa tactggccag 540
   tgttcattaa cctcgcactg catgcctgat gccagcctca tccgtgatct tgctgaatcc 600
   ccccacaact ctgtcaagta tgttctatta ttatcctctt ttagtagata aggaaacaga 660
   ggctcagagc agtgaagcaa acttgctaat tccacataac tggtattaat ggagatagta 720
   atcaatccca gatcatgctc ttcactgctg tactgtatcc ctttattcca gggacattgc 780
   tttccctacg gtatggcagg tgtggcactc tccagcccct ccagccccag gcagggagga 840
   caaggagagg gggaaagggg gtgtcccgac tattggcaaa ctcattcact aactgaggct 900
   ctgctatgtg ccatcccttt ttttttttga gtcgctctgt cgcccaggct ggagtgcggt 960
   ggtgcgatct ctgctcactg caagctccgc ctcccgggta cacgccattc tcctgcctca 1020
   gcctcccgag tggctgggac tacgggcgcc cgccacgacg cccggctgat tttttgtatt 1080
   ttttagtaga gacggggttt caccgtgcta gccaggatgg tctcgatctc ctgaccacgt 1140
   ggtccgcccg catcagcctc cagagtgctg ggattacagg cgtgagccac tgcgctccgg 1200
   cctgggcgac agagtgggac tctgtctcca acaaaacaaa acaaaaaaat tcatataaaa 1260
   atacaggtaa aatgtttcaa gtaattagaa tattctggca ctggtccatg cgtgttggct 1320
   catgcctgta atcctaatac tttggggggc tgaagtgggc ggatcgcttg agcccgggag 1380
   tttgagaccg gcctggacag catggtgaaa ccctgtctct gcaagaagtg caaaagttgg 1440
   ccgggtgtgg tggtgtgtgc ccgtggtccc agctacctgg gagggagttt gaggtaggag 1500
   gatcagttga gcccaggagg tcgagggcgg tgagccgtaa tcacaccact gcactccagc 1560
   ttggacaaca gagcaagact ctgtctcaaa aataaaaaaa agtataccag cactattgtc 1620
   agcagttggg atacagtgga caaaaccagc aaaaagcctg cctttgtgga acttacattc 1680
   tagcagctat gaagagaaca agttagtgtg atagaagaca gctttggtat tgggaagggt 1740
   acagccactt ctgactgggt ggtcagggcc ggctcctctg aggagacttg gaaggtaagg 1800
   aggagccaac cctgtggaga gctgaggagg agcattctga gtagagggaa tggcaagggc 1860
   aaaggcccct tgtgtttagg aaaaggaagg ttgaccagtg tggccgcaga ggagggagta 1920
   gcagggagac agtgagtggg ctggaagggg agccagggct gcgccctgta ggccaccaga 1980
   aggaggagtc tgtattttgt aataggagca gtaggaaact tgtggaaggt tttgagcagg 2040
   acagtaaagt gacctgattt gtaaaggacc ttaggatcca gttggcccat cttccctgtc 2100
   cctgtcccat cttccctccc tcctgcaaca cagatacatc ccatcaaggc acccctgcca 2160
   aatggcgagc cagtctggct gatggaggtg aggggtctcc aaaggttgcc ctagaacagt 2220
   ttctcaagcc gggcatgtta atgtttaggc tggatcactc tttgttgtgg gggcggtcct 2280
   gagcctggta ggatgtttag tggcatccct ggcttctacc cactggatgc caggagcacc 2340
   cccccgtcaa gttgtgacaa ccacagatgt ctccagagat tacagatgtt ccctggtggg 2400
   cagttgagaa ccactgctct gaatggttat tgtccccgag acgatgttct gttcctcaca 2460
   gtctcctgca cagctacctt gacggggcga tcacctgcac tattttaaac catctctctc 2520
   agatttcacc aggatgcaag tcatttcatc agtgggaaac tcaccagggt gacctgcctc 2580
   aagtgctaca cagggaggga cagtctgaat ttcccccagt ctttagccct gtcgctgtgg 2640
   aggggagggg gtagcaggga caggactgct aggacacctt gctcagccac agccccactt 2700
   gtaaccgcgc tggcgattgt gacacttgta gagcaaggaa atcagcagct catggaatgt 2760
   gaaggagcac ctttgtcccc agagagattt agataaaggt tcaaggtgag gctaccctcc 2820
   cctccctgag gctgattttg gtgagaggtg tggtcatctt gtagtatcaa gactacaggc 2880
   atgggcttga gaattaggag gagcctgagt gagaatcctg gctctcttcc cagctttaca 2940
   agcctggaca aatcctttga cctcgctgag tctcaggttc ctcatcagaa gtaagaggac 3000
   ctcccatata tcttccaggg cacttatcgg ccttggtaat ggatattaag gccactggct 3060
   ttggaatcca gcagactttg tttcaaatgc tgactcggcc actttactag ctgtgtgatc 3120
   ttgggcaaat tactttatct ctctgagcct tgttttcctt atttttattt atttatttat 3180
   ttttttgagt ctcgctctgt cacccaggct ggagggcagt ggcacaatct tggctcactg 3240
   caacctccac ctcctggcaa ttctcctgcc tcggcctcct gagtagctgg gactacgggc 3300
   gcacgccacc aggcctggct attttttgta tttttagtat aggcgggatt tcatcatgtt 3360
   ggccaggctg gtctcgagct cctgacctca agtgatccgc tggccttggc ctcccaaagt 3420
   gttgggatta caggcatgag ccaccacgcc cggcccttgt tttccttatc tatatctgca 3480
   gtaataacag tatttacctc caagattgca aggatgaaag gataatatat gtaaaagaca 3540
   ccctaggtgc cggcacctgg tggatactct atgatcacct tcagcttcac acagcaggct 3600
   aaggccattc tttgggaaca ggaagaccag gctgttccca ctgtgtatgg gtgctgctga 3660
   gtactcagag gcagtgggca gttaagagga gaagagttct gggatcagaa aagtgcccag 3720
   gactgtcctt ttattcatat gttcaagagg tgttcattca tccaggccct ggatcaggct 3780
   actggaggta aaagaataag tgaaacagag ccagtcctgc ccttgtgtag cagacaggct 3840
   gtctggatac tgtccataaa aataaaatgt gatcggctgg gcacggtggc tcacaactgt 3900
   aatcccggct ctctgggagg cccgggcggg tggatcacga ggtcaggagt ttgagaccgg 3960
   cctgaccaac atggtgaaac cctgtctcta ct 3992

<210> 87
<211> 3588
<212> DNA
<213> Homo sapiens

<400> 87
gatcaaacac ggcgtcatgg tggacgccac cacccggatg ggctacactc ccctccatgt 60
ggccagtcac tatggaaaca tcaagctggt gaagtttctg ctgcagcacc aggcagatgt 120
caatgccaag accaagctag gatacagccc cctgcaccag gcagcccagc agggacacac 180
agacatcgtg actctgcttc tgaaaaacgg tgcttcccca aacgaggtca gctcggatgg 240
aaccacacct ctggccatag ccaagcgctt gggctacatt tctgtcaccg acgtgctcaa 300
ggtcgtcacg gatgaaacca gtttcgtgtt agtcagtgat aagcatcgaa tgagtttccc 360
tgagacagtt gatgagatcc tggatgtctc ggaagatgaa ggaactgctc atataactat 420
aatgggggaa gaactcatca gcttcaaggc tgagaggcgg gattccaggg atgttgatga 480
agagaaggag ctgctggatt ttgtgccgaa gctagaccaa gtggtggaat ctccagccat 540
ccccaggatt ccctgtgcca tgcctgagac agtggtgatc aggtcagaag agcaggagca 600
ggcatctaaa gagtatgatg aggactccct catccccagc agcccggcca ccgagacctc 660
agacaacatc agcccggtgg ccagcccggt gcatacaggg tttctggtga gcttcatggt 720
tgacgcccgg ggtggttcca tgagaggaag tcgccacaac ggcctgcgag tggtgatccc 780
gccacggacg tgcgcagcgc ccacccgcat cacctgccgc ctggtcaagc cccagaagct 840
cagcacgccg cccccactgg ccgaggagga gggcctcgcc agcaggatca tagcactggg 900
gcccacgggg gcacagttcc tgagccctgt aatcgtggag atcccgcact ttgcctccca 960
tggccgtgga gaccgcgagc tcgtggttct gaggagcgaa aacggctccg tgtggaagga 1020
gcacaggagc cgctatggag agagctacct ggatcagatc ctcaacggga tggacgaaga 1080
gctggggagc ctggaggagc tagagaagaa gagggtgtgc cgaatcatca ccaccgactt 1140
cccgctgtac ttcgtgatca tgtcacggct ctgccaggac tacgacacca tcggtcccga 1200
agggggctcc ctgaagagca agctggtgcc cctggtacag gcaacgttcc cggagaatgc 1260
cgtcaccaag agagtgaagc tggctctgca ggcccagcct gtcccggatg agcttgtcac 1320
taagctcctg ggcaaccagg ccacattcag ccccattgtc accgtggagc cccggcgccg 1380
gaagttccac cgccccattg ggcttcggat cccactacct ccttcctgga ccgacaaccc 1440

gagggacagc ggggagggag acaccaccag cctgcgcctg ctttgcagcg tcattggagg 1500
aacagaccaa gcccagtggg aagacataac aggaaccacc aaacttgtat atgccaacga 1560
gtgcgccaac ttcaccacca atgtctctgc caggttttgg ctgtcggact gtcctcggac 1620
tgctgaggct gtgaactttg ccaccctgct gtacaaagag ctcactgcag tgccctacat 1680
ggccaaattc gtcatctttg ccaagatgaa tgacccccga gaggggcgcc tgcgctgcta 1740
ctgcatgaca gatgataaag tggacaagac cctggagcag catgagaact tcgtggaggt 1800
ggcccggagc agggacatag aggtgttgga aggaatgtcc ctgtttgcag aactctctgg 1860
gaacctggtg cctgtgaaga aagctgccca gcagcggagc ttccacttcc agtcatttcg 1920
ggagaaccgt ctggccatgc ctgtaaaggt gagggacagc agtcgagagc cgggagggtc 1980
cctgtcgttt ctgcgcaagg cgatgaagta cgaggacacc cagcacattc tctgccacct 2040
gaacatcacc atgcccccct gcgccaaggg aagtggagcc gaagatagga gaaggacccc 2100
gacgcccctg gccctgcgat acagcattct cagtgagtcc acaccaggtt ctctcagtgg 2160
gacagagcag gcagagatga agatggctgt tatctcagag cacctcggtc tcagctgggc 2220
agagttggcc cgggagctgc agttcagtgt ggaagacatc aacaggatcc gagtggaaaa 2280
tcccaactcc ctgttggagc agagtgtggc cttgctgaac ctctgggtca tccgtgaagg 2340
ccaaaacgca aacatggaga atctgtacac agccctgcag agcattgacc gtggcgagat 2400
cgtgaacatg ctggagggtt ccggccgaca gagccgcaac ttgaagccag acaggcggca 2460
caccgaccgc gactactcgc tgtcaccctc ccagatgaat ggccatcaga gggggcaagc 2520
ccgaatcaca cattccccca ccgtgagtca ggtgacggag aggagtcagg acagactgca 2580
ggactgggat gcagacggct cgattgtctc atacctgcaa gatgctgcac aaggttcctg 2640
gcaagaggag gtcacgcaag gtccacactc attccaggga acaagtacca tgactgaagg 2700
gctagagccc ggtggatctc aggagtacga gaaggtcctg gtgtctgtaa gtgagcacac 2760
gtggacagaa cagcccgagg ctgagagctc ccaggccgac agggaccgga ggcagcaagg 2820
ccaagaagag caggtgcagg aggccaagaa caccttcacc caagtggtgc aggggaatga 2880
gtttcagaat attccagggg agcaggtgac agaggagcaa ttcacggatg agcagggcaa 2940
cattgtcacc aagaagatca ttcgcaaggt ggttcgacag atagacttgt ccagcgccga 3000
tgccgcccag gagcacgagg aggtggagct gagagggagt ggcctacagc cggacctgat 3060
agagggcagg aagggggcgc agatagtgaa gcgggccagc ctgaaaaggg ggaaacagtg 3120
accccgagcc gctctccttg gagtagcctc tcgggaggat cacacctcga cacccaaccc 3180
ctgaacccca cacactctgc catgcacaca ggaggagagc tggacctgag ggccaccgca 3240
gcggtgcaca cattcctctg ggctgacggc atgacctctg taaggactcc ctgctagtcc 3300
cctcttggca tgaatgactg actgtagacg catgacctcc aggcttcaat cctgcctctt 3360
gcaatgacag ctgatctgtc ggaaccagga cacaaaagca gcaagaagcg gggagagaga 3420
gggatagaaa acaagcgcag gagagcctgc gaacgcaaaa gtgaatgagg gctttttgtg 3480
gctggggatg ggttttggtt ttggggtttt tttttttaaa ttgttttgac ttcgtacagg 3540
gtactttttc ccaacctcat ctgtcagaaa tccatgtggg cttcctgg 3588

<210> 88
<211> 3781
<212> DNA
<213> Homo sapiens

<400> 88
cttagacctt ggttagattt gaaccaaggc cctcctcggc ctcccaaagt gctgggatta 60
caggcgtgag ccactgccca gctgaaaatt gatacatttt tcaaatgaga agtatttttt 120
tcaaatgggt gttggaaagt gagtttcctc acattgttca tgttgtgaca ctaggttggt 180
gctatgtaat gacatggata tgaacgagct gcctacgaac ctccccgtgg acactgtgaa 240
gcttcgcata gagaagactg tcatccgcag aatctctgcg gaggccttct attacctggt 300
ggagctccag tatctctggg tgacttacaa ttccgtggcc agcattgacc ccagcagctt 360
ttacaacctg aagcaactgc atgagttgcg cttggatggg aattctctgg ctgctttccc 420
ttgggcatct ctgctggaca tgccccttct gaggaccctg gacttgcaca ataacaaaat 480
aaccagtgtg ccaaatgagg cgctcaggta tctgaagaac cttgcctact tggatttatc 540
aagcaacaga ctcaccacat tgccaccaga tttcctggag aactggactc atttagtttc 600
aacaccttct ggagtcctgg acctttcccc aagcaggatt attcttggct gatagaacgt 660
tatggctgta aaaagctcta gcatttatct agtcaactct cccattttgc cgatgagaaa 720
acaggctcag caaggttgca tcacccaggt cacagaatta actaatggtt gagcctcatc 780
aggaacccag gcacagtaaa gttggtctac aggacaatcc ttggttctgt gactgtcata 840
tttccaaaat gattgagttg tcaaaggtcg ttgaccctgc tatagtgctt ctggatccac 900
tgatgacttg cagtgaacct gagcgcctca caggaatttt gtttcagcgg gctgaattgg 960
agcattgtct gaaaccatca gtgatgacct cagccaccaa aatcatgtct gctctgggca 1020
gtaatgttct actgcggtgt gatgccactg gcttccccac cccacagatc acatggacca 1080
gatctgacag ctcgccagtt aattatacag taatacaaga atctccagag gaaggagtca 1140
gatggtccat aatgagcttg acaggcattt cttccaaaga cgctggggat tacaaatgta 1200
aggccaaaaa tctggctggg ttgtcagaag ctgtggttac tgtgacagtg cttggcatta 1260
ccacaactcc aataccacca gacacttctg aaagaactgg agatcatcct gagtgggatg 1320
tccagccggg atctggaaga tctacatctg tatctagcgc atcatcatat ctttggtcct 1380
cttccttctc ccccacatct tctttttctg cttctacttt gtctcctccc tctactgctt 1440
ccttctcttt atctcctttc tcctcctcca ctgtttcttc aaccacaact ctgagcacaa 1500
gcatctcagc aagtaccacc atggccaaca agcgatcatt ccagctccac caaggtggga 1560
aaagaaattt aaaggtggca aagaatggaa gtaagcttcc tccagccagc acaagtaaga 1620
aagaagagct ggcattgttg gatcaaacaa tgcttacgga gacaaatgcc gcaatagaaa 1680

acctcagggt ggtcagtgag actaaagaga gtgtgacatt gacgtggaat atgatcaaca 1740
ccacacataa ctctgcagtg actgtgttgt attccaagta tggtgggaag gacctgctgc 1800
tgttgaatgc agactccagc aagaaccaag taaccataga tggcttggaa cccggtgggc 1860
aatacatggc ctgtgtctgt ccaaaaggag tgcctcccca gaaagaccaa tgcatcacct 1920
tttctactga aagagttgaa ggagatgatt ctcaatggtc tctccttctc gtggtgacca 1980
gtactgcctg tgttgttatc ttaccattga tttgtttctt gttgtacaaa gtttgcaaac 2040
tgcaatgtaa atcagaacct tttgggaag atgatttggc aaaggagact tatatccaat 2100
ttgagaccct gtttcccagg tctcaaagtg taggtgagct ctggacacga agccacaggg 2160
atgactcaga gaaattgctg ctttgttcta ggtcaagtgt ggaatctcag gtgactttta 2220
aaagtgaagg ttccagacca gagtattatt gctaaggttc tgcagctcag gtgcatgtga 2280
gctacaaaac tagcatctaa gggtataatt gaccctaggt ttggatgact tttggacaga 2340
ctttcacatt gtacatgaaa atcacaaatg gaatgctttt aagtatgttt aaaaaatacc 2400
atgagacctc tgaactgaaa agacaaataa tgttgatttt tttttcttgt gtggaaaagg 2460
tctacagaaa taatttttag agtggtgctt aataaattat taatactttt tagggtaatg 2520
ttttagttct taaaaataag ttcatgtgaa agtagcaagt gaactctgta tttaaaaata 2580
tagtttttg agtcatgagg aaacattagc aaaaccagct aagccttgat gtttattcat 2640
gttcgaagtg cattatagca tttgctgtgg atgttttctg acattgccta atggagagcg 2700
tgggatcata aaggattttg tgttcactaa ggttaaatct aactcacggg agagagaggg 2760
ctctgtctct tggtaccatt atgcattgtg atactataga gttgtgttca tatttcttgt 2820
aagaaaaatt tttgcttttc tttagaaata taggtattag caaaagtaag tatacaaaga 2880
ggattatgac aaatagacca tttctttaaa aatggtaact gttgcattta aacacatcgg 2940
ctgacagatt atatggatat ttaaaaataa ctttaaatca atcttttaat attttgctgt 3000
ttgctcttat gttctccctt ctttcttcct tccttgtctt ccctcccttt ccttccttca 3060
ttctttcttt cttctgctct gtcagaagat atacaagtag ccaaagcatc acgagccaag 3120
ctgtttgtct tttgccatat tgtcttttga ttctcttgat aaagagaaag cttttgaaat 3180
tacttactgc cctttgaaag tctgttttac aaccccaaag gatattttag gactttaaaa 3240
gggaagcttc agtggattca tttggttaga ttcttttcct aggttaaaaa catcggggaa 3300
cttaaataca taggaaagga gttcacaaat aaaatttagt ttttttagaa agagaatatt 3360
ttaataactg tagtatacca caagaaacat ctcatggctt taagattaac gatagcgata 3420
aggcccactc aagcatctca cagtggtgat attacatgtc acttagcagg atgcaacctg 3480
gcctagggat ttccagaaga caaggccacc tcagcacaga tgcaactttc ataaatttta 3540
gaacaaagcc taaccttact agattaaact tgctctatga aagaaacact tggtaattga 3600
cccagactga atacatgtat aagaaatggg aaagatctcc caaactctga gaacggtcct 3660
caaatagaag ctctcctagt cagttggtca tctgacctct gactatatcc ggcctatgac 3720
accagccagc tcctgctttc tgtcttgtaa gagcactgct ggaataaact acttgagcat 3780
t 3781

<210> 89
<211> 3848
<212> DNA
<213> Homo sapiens

<400> 89
gatacttaaa acgaatacaa tcttgtacag taaatgttta acaccccaaa ataacgaaaa 60
gtaattatta gcattcattt atatctaaat ctcagtcaat atgatctaaa tatttgtgtt 120
taaagactcc ccctttccat atagataatg aagggctttg aaataagtgt aggaaaccct 180
aagtttggtc tcaggcttcc tattcattca ctatgctttt tcgtgtgctg tttacagttt 240
cctttttctg gaatgccttt ccctgctttt ttcccccgcc aacatttcag cttcccacag 300
acacacattt gtgccctcct ggttgtcctg caggatgacc tttgtttttg tttttgtctt 360
ttgtttttcg agacagggtc tcgctctgtc gtccgggatg gagtgcagtg gtgtgatctc 420
tgctcactgc ggcttccgcc atccggtctc aagtgaccct cccaccgcaa cctcccaagt 480
ggctgggagg agcggcgcgc gccaccatgt ctggctagtt tttgtatttt ttgtagagac 540
ggagtttccc catgttgccc aggctgctct cagactcctg agctcaagcg atctgccgcc 600
tgcctggccc tcccaaagtt ctgggattac aggtgtgagc cactgtgcct ggccaggatg 660
acctttttga agtcacctag tcattgaagc tttccctctc agccttttcc tctgtggccc 720
catgcttata gagcctccca cattgatttg cagtgccttc gttcgttcac tttatttcca 780
cctaccaaac agtaagatct tgaagacaga aacggcatta ttcgtttata tatccccggt 840
atctagtaca atttttctgt ttgcaataat aaatgtctta aaacttaaga aaggagtaag 900
tcttctcaaa gacttttttt aaaaaagacc cagattcagc acttattgta tgtaagacag 960
accttccttt tacattacat ttttcatcag taaaagtact gggccaggcg cggtggctca 1020
cgcataatct cagcactttg ggaggctgaa gcgggtggat cgcttcaggt catgagttaa 1080
agacccacct ggccaacatg gtaaagcccc atctctacta aaagtggaag gattggccga 1140
atgtggtggc tcgcgcctgt aatcccagct actcgggagg ctgagacaga ggagtcgctt 1200
gaacccggga ggcggaggtt gcagtgagcc gaggtccgcc actgcgctcc agcccggatg 1260
acagagcaag actccatccc caaaaaaagg tactagtatt tcatgttaat aaaatggaat 1320
tcttatataa acatatataa attaaaaatt attttaagag ttgaacagat ttctctgcaa 1380
atccccctat atttttatat tttttaaact aactgtattc agcaaagctt tttattattt 1440
aatttattcc aaaccacatt aatcagtatt tctctgaact cctacagcac atttaaaaca 1500
gtactgtttc tctaggtgtg ggtgagtaaa aaaatattta atatatgtta attaacttcc 1560
gcatctcctg accccactaa ggtttgaata ggagcctccc cctttataaa ccctttttat 1620
atacccacac tacctagcct aattggggca catattagct gcttcataca tacttttcaa 1680

ctgcctaatt tgctgattag gcataaaagt atatacttct ggaaaagtct ccacaaggtg 1740
aaaatgatta tccaaataat aaaggcccac aatgccagtg agtacattat ctgcattatg 1800
ctgatatttg actcctttca actctctttg gttgtagaaa catgaccgat gattatatta 1860
cacttttaac tctttaaata ttattagctt gctttggact gtggaaaact aactacaatc 1920
atttttattc ctcagaatat attttgcggc cgggcacggt ggctcatgcc tgtataccca 1980
gcactttggg aggccgaggt gggcagatca cgaggccagg agttcgagac cagcctggcc 2040
gatatggtgt aaccccgtct ctgctaagag tacaaacatt gggtgggcat ggtgctgcaa 2100
gcctgtagtc ctagctactc gggaggctga ggcggaagaa tcacttgaac ccgggaggca 2160
gaggttgcag tgagccaaga tcatggcatt gcactccagc ctgggtgaca gagcaagact 2220
ccatcttaaa aaatatatat atatagatag atagatagat agatagatgt atatatacat 2280
atacgtatac atatacgtat atgtatacgt atacatatat gtggagtata tatacatcta 2340
cgtatatgta tactctacat atatgtgtat atatacatat atacatctac atatatacac 2400
atatatatgt atatggcttc tgagctatag cttgagtaac ttgttaactt gtaatacttg 2460
agtaacttgt aatattgtat gctgccaaag catttttca cttaaacatc tcatcgtgat 2520
tttcagaaat ttagaatcgg aaaatcgttt ttgatcccac tcatggatat ataaacttag 2580
ggctaaatca gaaaagcaag cacatagttt tagacaatga cacttatttg cttaattact 2640
ctatggagcc aggtggatat gtacaggtta tgtccttttg catgcttgaa tttatagtga 2700
gttgaatctg ttcagacagc catcagtgat gtgtccgtga ggaacacaga aaatgtttct 2760
ttttgtttaa aggaatttat ttatttactt atttttactt ttttttttaa tcatacttta 2820
agttctagga tacatgtgca caacgtgcag gtttgttacg tatgtatata tgtgccatgt 2880
tggtgtgctg gacccattaa ctcgtcgttt acattcggta tatctcctaa tgctatccct 2940
cccctctccc cccaaaaatg tttcttttga atgcagttga ctcagttacc taagatgtga 3000
gtgaagtgga aagggtaagt ggtgggaata atgcaaaatg atagtttaaa aatccttcat 3060
gcccgtttac tttcctaata tggagataat tgaaatctgg actgggaata attttttttt 3120
aaaaaaactg gattttcctt gggcattgcc atttactaat ctcatgatcc tgggcgagtc 3180
atgtaatctc tttaaaactt attaactcat aaaaatagaa gtaacaatgt ccaaacttcc 3240
tattttagag ttttcacgag gcgcaataga tgtaactgaa agcttcgaga agtttaaagg 3300
acttgcaagg aaatctactg aaataagccc cattccacct gggcagaagc actcagacgc 3360
caaaccccac cttctgtcct gaaattcacc ttccactagg agagtttgct tttctaatgc 3420
ttcaaatgca caacgggaga tgttctgact tcagggtttc taccettcac tgctgtaatc 3480
tgctgcctgt atgacatgcg atctggttta caccgtacag cttatcatct tcagcttgtg 3540
gccccttcct tgtttccacc ttgctattca ggagggcctg taagggtatc tggattcctt 3600
gagctgtaga tgcaggacag tgagagggag ggaggtagtt ctctggtcta atttcagttt 3660
cctaactgag ggtacatgcc ttccctgaat gatcatcatc aaaaatatcc agcccttcat 3720
ggaagacacc acccctcctc atagatgagt ccctgagctt atgatctcaa aaggaaatcc 3780
ttatcagaat acaacagaat aaggacatag tttcattaca gttataataa taaaagtagt 3840

tcttcttt                                                          3848

<210> 90
<211> 3546
<212> DNA
<213> Homo sapiens

<400> 90
   tgccctttag agcctggaga gggcatgcag agggcctgtg gcttgtccca gccggaggtt      60
   ccccttaagg ggaccagctg ccttctctgg ggagctgtct atggggtccc cgcagccctc     120
   cagtggaggc agaggccctg gctcctgacc gcacttgttt tgcataggac tgggctaagc     180
   ctgggcccta tgaccagcct ctggtgaaca ccctgcagcg ccgcaaagag aagcgagaac     240
   cggaccccaa cgggggagga cccactaccg ccagcggccc acctgcagca gctgaggagg     300
   ctcagagacc acggagcatg actgtatcgg ctgccaccag ggtgatgctc ttgttctcag     360
   tagctggttg gggccacctc agcctggctg gggatcacat tctggccagc aactgggggg     420
   ccaaggcagc cttgcagctg gagggacctg ggctccattc cctgacttcc tgctctacag     480
   caaagtccct gggaaaaaaa ccttgtgtgg gaatgagaga ggcagaactt tctagggtca     540
   gagtgggacc caagcaagtc aatagccccc tggccccaca gacatgtagt gctcacgggg     600
   gcagaacagg agtgctgttg agcccccccc ctaaagcagc ctgtgtattt ctctcctcca     660
   acctgcagcc tggtgaggag atggaggctt gtgaggagct ggccctggcc ctgtctcggg     720
   gcctgcagct ggacacccag aggagcagcc gggactcgct tcagtgctcc agcggctaca     780
   gcacccagac aaccaccccc tgctgctctg aggacaccat cccttcccaa gtttcagatt     840
   atgattattt ctctgtaagt ggtgaccagg aggcagatca gcaggagttc gacaagtcct     900
   ccaccattcc aagaaacagc gacatcagcc agtcctaccg acggatgttc caagccaagc     960
   gtccagcctc aactgctggc ctccccacca ccctgggacc tgctatggtc actccagggg    1020
   ttgcaactat ccgacggacc ccttccacca agccttctgt ccgccgggga accattggag    1080
   ctggtcccat ccccatcaag acacccgtga tccctgtcaa gaccccaacc gtcccagacc    1140
   tcccaggggt gttgccagcc cctccagatg ggccagaaga gcggggggag cacagccctg    1200
   agtcgccatc tgtgggtgag ggcccccaag gtgtcaccag catgccctcc tcaatgtgga    1260
   gcggccaagc ttccgttaac cctccacttc caggcccgaa gcccagtatc cctgaggagc    1320
   acagacaggc aattccagaa agtgaagctg aagaccagga acgggaaccc ccaagtgcca    1380
   ctgtctcccc aggccagatt ccagagagtg accctgcaga cctgagccca agggatgctc    1440
   cacaaggaga agacatgctg aacgccatcc gaaggggcgt gaaactgaag aagaccacga    1500
   caaacgatcg ctcagcccct cgcttttctt aggttcacaa gaaatgcgcc ggtggggaat    1560
   gaactgtttc attaataaaa cctaatttgt cttgatccat tccactctat aataaaacaa    1620
   aagattttgt aggcaactcg gaatatagct cttttgaaag tactcgacac ctttagataa 1680
   gaattaaaac caacctatgt aactgacata atcttgatct tttaatttgt aaatattgac 1740
   aattttcttt ctgcacattt taatcttagt ttcccttttg atttttctga aggtgccaaa 1800
   ttccatttaa cttttttaca agtctttgta aaattttaaa tgcataaagg gggttggggc 1860
   aggggaacca cgaagtagtt aattttagaa aaggatttac tatacttcac tcttcttttt 1920
   tttccccaca agcttttgta gatgcattgt agtagtctag cttagaagca aatgcaagtt 1980
   attttaatgt acaaactaaa tgggtaagag gtaaaatctt catttaaata tactatgttc 2040
   tggatgaaaa gagcaggagt aacaattgat gagcaatatt cagagtgaag taaatctgga 2100
   aatggtagac tgtgttggga taggggggag ggccatggga ggggtacatc gtcaacatag 2160
   ccgatcctgt tacatttaag agtagcctcg taggttgaat ttcttctggt agcttcatgg 2220
   taaatgcatc cgaataagcc atactggatt gcaatgtttg tttctgtagg gtgtttaagg 2280
   acttgacttc ctttctccca tgattcctct ggactgcaca cagcacccac aaccagcccc 2340
   atgcatgctg ctgcctctgg gcagtcgtag aatctcccac ttcagtttct cgttgattgt 2400
   actcaccttt atggaatcca aatacatcca aaagggtaag gcagttttaa aaatgtgaaa 2460
   acatttaaaa atgataatag cagggaattc ttagattata gtaaatgcct tttacttaac 2520
   tgtgcccagc aggctgggtg cgttaaaaag cccaagtatt ttgaaaaaac tcgaacagat 2580
   ttgacaaggg tagccagctt ggagtctagc aacttgccaa tgtgtttacc aatctggggg 2640
   cttgtttttc ttttcttctt tcaaataaat ggcagttaac tggctttaca gtaaacattg 2700
   aagagaggag gatttgttta ttgtcactgg gaatctgacc actatactgt cctttttttg 2760
   tattctgggt aatgtttttt ggaaaagatt tgtcttttct aagtggaagt taaatttgtt 2820
   atactgccca tcccctaaag ccaacagaga tttgtagatt taaagggatc acatttgaag 2880
   acaatagtgt ttaagaaagc aagcaagtcc cttagcagtc aggtcataac agggcacatt 2940
   tctgaccgaa ccctctcaag gcagaggagg agtttggtgg gtttcataca ccctgcagat 3000
   tcctgttggc tctaaccctc aattacctaa tcttatgctt taacacataa ctgcattgga 3060
   tgtgagagta acgtaccgta tggtcattgt tctatatatt aacattgaac actgctgcga 3120
   ttgctcaagg acattttatg ttacggcttt aaagcaaagg catgattatt agaaactatt 3180
   taagcttttt tctttgaaaa acaagctcct tttacagaat ataaacaaca gtagtgcctg 3240
   tggtttagcc caccaatctt gatgactaaa agtagctgat gcattgtgca tatgatgctt 3300
   gagatggttt ttgcaaaagc agaaatcgct gcaaggtaat cacaatagat aaaagtggta 3360
   ttttaaacct ttaaaataaa tggatgtaac tgtaccttgg tacagctttt ctcttgttta 3420
   gtttttaaac gttagtacaa tctgaataaa taaaatgttg ccaaattcaa tgtagaaaga 3480
   atgtgacaac acaccttggg tagttctgct tgtgtttttg catattgtaa aagcagtgtc 3540
   acagct 3546

<210> 91

<211> 3675
<212> DNA
<213> Homo sapiens

<400> 91
ctgatttctg gtcacaggag taatgcccac tttcccaccc ttgcctcccc atccccatga 60
gcacagtcag gcctgaatgc aaagcctgat gttgccaccg ctagtgctat gaccttggga 120
acattactca catttcctga gcctcaaggc cttggtctat aacacgagca taataaaatg 180
tgagcggttg ttatgagatt taaataagat taaggaagtg ttacggttgt gttcagcatg 240
ttgcctggca gatactaaag atttaacaaa tggtagctaa tcttgtcagt cctggctttt 300
aaaaacatct ttcccagcca tttcttaggc tgtctagttt cttatcataa atacacagcc 360
agttgagaat tgagttacat cttaagagga aatttgggac agtaagtaat cttgccaata 420
gtgcctctct gctatcttaa atctgtgtct cttcctcaat gcccttatcc tcccaaaaaa 480
aaaaaaagag aatatttttg ctctgccatg tctcctattc atccttctca gagggatggg 540
gtagaacatg gtttggagat gagactacct catctttctc tagctcctca tagagctcat 600
gtccttgctg tcaaggaaga tggcggggag tagtacttcc ccggagcata gatgtggttg 660
aagctgcacc ctgtcccctc ttccagggcc ccctgatgtg ccacactact aagatgtaca 720
gcacagatga tggagtccag tttcacgcct ttggccgggt gctgagtggc accattcatg 780
ctgggcagcc tgtgaaggta ctgggggaga actacaccct ggaggatgag gaagactccc 840
agatatgcac cgtgggccgc ctttggatct ctgtggccag gtaccacatc gaggtgaacc 900
gtgttcctgc tggcaactgg gttctgattg aaggtgttga tcaaccaatt gtgaagacag 960
caaccataac cgaaccccga ggcaatgagg aggtagagtt tctgaagaga ccaactgggt 1020
tggcttaagc tagatggtcc cacggacaca catgagcaca aacggacta atccacagtc 1080
attgccatgt cctggtttca ggcttagcct tctagatatg accctgctgg attctccctg 1140
tactcagttc agatgcgagg aattaggacg ggtgcctttt attattttcc tcctctcttt 1200
gttctgccag aatacctggg aagaaatggc ccttcaggtt acccccactc agcaccaggc 1260
tcctggttgg ggaatacata gtctgatcct aagcaagtcc tcttctcttc tcatacctct 1320
gcactttctg tgcctgctca tgccagttgc cctcagtcct ttgtgctttg gatctcaggc 1380
tcagattttc cgacccttga agttcaatac cacatctgtt atcaagattg ctgtggagcc 1440
agtcaacccc tcagagctgc ccaagatgct tgatggcctg cgcaaggtca acaagagcta 1500
tccatccctc accaccaagg tggaggagtc tggcgagcat gtgatcctgg gcactgggga 1560
gctctacctg gactgtgtga tgcatgattt gcggaagatg tactcagaga tagacatcaa 1620
ggtggctgac ccagttgtca cgttttgtga gacggtggtg gaaacatcct ccctcaagtg 1680
ctttgctgaa acgcctaata agaagaacaa gatcaccatg attgctgagc ctcttgagaa 1740
gggcctggca gaggacatag agaatgaggt ggtccagatt acgtggaaca ggaagaagct 1800
gggagagttc ttccagacca agtacgattg ggatctgctg gctgcccgtt ccatctgggc 1860

ttttggccct gatgcgactg gccccaacat tctggtggat gatactctgc cctctgaggt 1920
ggacaaggct cttcttggtt cagtgaagga cagcatcgtt caaggtttcc agtggggaac 1980
cagggagggc cccctctgtg atgaatgtaa gtccaccagc actcccccac cccagtcctc 2040
gagggtcctt gcagccaggc atatgagtgg gatgggctca ccatctttag gattcggcag 2100
gagaagcagc ttggggtaca caggaccatc ccaagtcctg ggccagcttc ttccctttc 2160
cttccttatc ctggtggtgt agcctggaaa tggaaattta agtcatttct aaactgtcat 2220
ttgctcctca tttctgagaa gggtttggcg ttggacgtat ttgagaagag atatcaagag 2280
gatgatgaga ttggaatggt ttatagaccc tgattgggct tcatggacca aatgtacaat 2340
tctggaattt attctacatc cacaaaaatg taaatatgtg cagaagaagg aaataaactt 2400
ctaggaaagc tctaagtctg agcatggcct gaagcaaaca ctaagaacat atgcttaact 2460
tctgacctct gccatgggcc ttgcttattc agttagaacg cccacctccc atttgatttc 2520
tgtaccatgt ctttcatgac tgcaagacag ctgcagtgtt gcaggagact gctactctgc 2580
catggcccca tgacaggccc agaacctctc cccagtcact ccctccacct cctttacagt 2640
gattcggaat gtcaagttta agatcctgga tgcggtggtt gcccaggagc ccctgcaccg 2700
gggcgggggc cagatcatcc ccacagccag gagagtcgtc tactctgcct tcctcatggc 2760
tactcctcgt ctgatggagc cttactactt tgtagaggtc caggcccctg cagattgcgt 2820
ctctgcagtt tataccgtcc tggccaggcg caggggggcac gtgactcagg atgcacccat 2880
cccaggctcc cctctgtaca ccatcaaagc ttttatcccg gccatcgact cttttggctt 2940
tgagactgat ctccggactc acacccaggg acaagccttt tctctgtctg tcttccacca 3000
ctggcagatt gtgcctggtg atcccctgga caagagcatt gtcatccgcc ccttggagcc 3060
acagccagct cctcacctgg cccgggaatt catgatcaaa acccgccgta ggaagggcct 3120
cagtgaagat gtgagcatca gcaaattctt cgatgatcct atgttgctgg aacttgccaa 3180
acaggatgtt gtgctcaatt accccatgtg agtgcgtgga ctcctgggag ctcctgctcc 3240
ctacagtggg ctgcaactcc tgtacttgaa gctgagacct catatgacgt ggccttcgtg 3300
ttgtcagaga gtgtctggaa gctgctgttg ccatcttgaa caactcacca acctccaacc 3360
cagagcccca gtgagagagg agcatttggc ctcctgcttc cttctgtggc ctctgccggg 3420
ctccattccc aaggaaaaga gaggagcttg ggctcacaga aagagaaggg gatgaaaccc 3480
caaggggccc tatctttggg atttacatgg aattttattt tctacaagtt tgaccttagc 3540
catggtttgc aagtgaacag aacattctga cctctgtctt gctctgctcc tttcatcctc 3600
gtctcccctg ccccgtctgg tgcttacatt ctgaatatat gtcatctccc aagaggcttc 3660
actgcctctg cttcc 3675

<210> 92
<211> 3700
<212> DNA

<213> Homo sapiens

<400> 92
catatagacc aatggaacag gttagagaac ccggaaataa ggctgcatac ctacagctat 60
ctgatctttg acaaacctga taaacacaag caatggggaa aggactccct atttagtaaa 120
tggtgctggg atagctggct agccatatgc agaagattga aactataccc cttccttata 180
ccatatacaa aaattagctg aagatggatt aaagacttaa atgtaaaacc caaaaactgt 240
aacaaccctg gaagaaaacc tagaaaatac tattctggac aaaggaatgg gtgaagattt 300
catgatgaag atgccgaaag caattgcaac caaagaaaaa attgacaaac ggaatctaat 360
taaactaaat aacttttgca cagcaaaaga aactagcaat agagtgaaaa gacaacctag 420
agaatgggag caaatttttg taagctatcc atctgacaaa ggtctaatat ataatatcta 480
taaggaactt aagcaaattt ataagaacaa aacaagcaac cccattaaaa aatgggcaaa 540
ggacatgaac agacacatct caaaagaagg catacatatg gctaacaatc atatgaaact 600
attttcatat cactgatcat tagagaaatg caaatcaaaa ccacaatgac atatcatctc 660
acaccagtca gaatggctat taaaaagtca aaacataaca ggtgctggtg aacttgtgga 720
gaaaaaggaa tgctcttata ctgttgttgg gagtgtaaat tagttcatcc attgtgaaag 780
gcagtgtgac aattcctcaa agacctaaaa acagaaatac taacagctat tactaaaaag 840
tcaaaaatta agagatgctg gtgaacttgt ggagaaaaag gaacactttt acactgttgg 900
tgggagtgga tattaatgca tccattgtgg aaggcaatgt gatgattcct caaagaccta 960
aaaacagaaa taccattgga cccagcaatc ccaatactgg gtgtataccc aaaggaatat 1020
aagtagtttt atcataaaga cacatgcaca tgtatgttca ttgcagcact attcacaata 1080
gcaaagacat agaatcagcc taaatgccca tcaagagtag actggataaa gaaaatgtgg 1140
tacatataca ccatggcata ctacgcagcc ataaaagaga atgagatcat ttcccttgca 1200
gccacatgga tggagctgga tgccattatc cttagcaaac taacacagga acagaaaacc 1260
aaataccaca tattctcgct tgtatgtagg agctaaatga tgagaacaca cggatacata 1320
gaggggaaca acagacactg gggcctatca gaggatgggg tggagggtag gggcaggaat 1380
aggttcaaga aaaaggtcat ctaatgggta ttaggcttaa tacctgggtg atgaaataat 1440
ctgtacaata aacctccatg acacaagttt acctatataa aaaacctgca catgtacccc 1500
tgaacttaaa ataaaagtta aatttaaaat aagaaaaaac acaaaaaatc aataaaataa 1560
aaagttggtt ttttgaaaag ataaaaacat tgaaaaacat tttagtcaga ctaagaaaaa 1620
acgtgaagac ccaaataagt aaaatcagag atgaaaaaga catcattaca gctgatactg 1680
cagaaattca aaggatcatt agagactact gtgagcaatt atctgccaat aagctggaaa 1740
acctagaata aatggagaaa ttcctatata tgtttaactt atcaagattg aactgtgaag 1800
aaatccaaaa cttgaacaga ccaataacaa gtaatgagat tgaagctgta ataaaatttc 1860
tcccagcaaa gaaaaaccag ggacctgatg gcttcattcc tgaagtttac caaatattta 1920
aagaagagct aataccaaac tattccaaga aatagagaag gagggaaccc ttccaaactc 1980

attctgtgaa gtcaatatta ccctgatgcc ataaccagac attgtattag tctgttttca 2040
cactgctgta aagaactacc tgagactggg taatttatga agaaaagagg tttcattgac 2100
tcacagttca gcaggcttaa caggaagcat gactggaagg cctcaggaaa cttacaatca 2160
tagtggaagg caaaggggaa gcaagcatgt cttaccatgg cggcagaaga gagagagaga 2220
gaacaaaggg ggaaatgcca cacactttca aacaaccagc tttcttgaga actcactcac 2280
taccacaaga acagcaaggg agaagtctgc ctcatgattc aatcacctct caccaggccc 2340
tgcccctgac atgtgagtat tccaatttga gataatattt ggttggggac acagagccaa 2400
accatatcac acatcaaaaa gacaaaatta taggccaata tcccttataa cattggtgga 2460
aaaatcttca acaaaagcat agtaaactga atttgacaac aattaaaaaa ttattcttta 2520
taaccaaatg gaatttatct cagtaataaa aagatggttc aacatatgct aattaatcaa 2580
tgtgctatat catatcagca gagtgaagga caaaaaccat gtgattattt cgattgatgc 2640
tgaaaaacat ttggtaaaat tcaacatccc ttcatgataa aaactctaaa gaactgggta 2700
tagagggaac atacctcagc gccataaaaa ccatatatga cagaccaaca gctagtatca 2760
tatgaatagg aaaaagctga aagccttttt tctaagatgt ggaacatgac aaggatgaca 2820
actttcatca ctgttattca acatagtact ggaagtccta gctagagcaa tcagacaaga 2880
ggaaaaaata aagggcttcc aaattggaaa ggaagagtca aattatcctt gtttgcagaa 2940
gatatgatct tatattcaga aaatcctaaa gactccacca aaacgctatt agaactgata 3000
aacaaattca gcaaagttgc aggatacaaa atcagcatac aaaaatcagt agcatttcta 3060
tatgccaata gtgaacaatc tgaaaaagaa atcatgaaag taatcccatt tgcaatagct 3120
gcaaataaaa taagatactt aggaataaac ttaaacaaat aagtgaaaga tctctgcaat 3180
gaaaactaga gaacattgat gcaactaatt gaagagagca taaaatggaa agatattcca 3240
tatttatgga ttggaagaat caagattgtt aaaatgtcca taggacccaa agcactctac 3300
agattaaatg caatccccat cagtgtacca atgacattct ttatagaaat agaaaaaaca 3360
atcctaaaat ttatatggaa ccacaaatga cccagaatat tcaaagccat cctgaacaaa 3420
aagactaaaa ctggaggaat cacattactt gacttcaaat tatgctacag agatattgta 3480
accaaaacag catggaattg acatgaaaac agacacatag accaatgaaa cagaatagag 3540
aacccagaaa taaatccata catctgcagt gaactcattt ttaacaaagg tgccaaaaca 3600
tacattggga aaaggatagt gtctttaata aatggtgcta gagaaactag atatctatat 3660
gcagaagaat gaaactagac ccctatctct cactatatac 3700

<210> 93
<211> 4906
<212> DNA
<213> Homo sapiens

<400> 93
atattcttct tgccctattc cttgactctc tttttctcct tactgccacc cctactactc 60
cattatcccc ttcggctcca gctcccccgg cgtggatgct cctggcttgc cttctgggat 120
gatacccagg gcttccctaa ttgttgccac agcctaccca gcccgctcct ccctctggac 180
tgactcactt cgccaggcac agagggccca tagcctgcaa tcctccccag tcagtgaatc 240
cctccccctt ccacagagat cttcagaaga ggtgtcgcca caccctttgc ccaggcgcca 300
ccctcccgcg tcagtgtgcg tcaccgtggg gatggtcttt gttgtttccc tctcaagcag 360
ggccctcctt ttcctcttgt tctgtacaca ctggagaggg agaacagatg gtcctcctgc 420
cgggggccag ccctcctggc agaggtgacc aaaccacccg gctttcccat ctcaggggca 480
tcatggacac ccctcagcta taggttctgt ttctggggat cccttgggtt ttctgtgtgt 540
ccccccccttc cccttccaa ggtctctgag acctgctgac ctatgcagct caagcccaga 600
gagaagacac caggcaggac tggtggtgct aaaagatggt agaaatcagt ttgtccctcc 660
tccgtgctgt ttgtgatttt gatttcaacc tgggacgagt tgaaaacagt cccccgcgca 720
gctcacccgg cccacgttac ccactatctt cccggactca cagctgtctc cacccatgct 780
tgcctactgg caggcctttc cggctttatt ttgcctagtg gtgttaccaa aatgtctcct 840
ggttgaactt gactcttgtt atatcttcca atttctcccg tgtccagatt tccagacctt 900
acccactgtg gaaggcatca cactgaagcc cagacatgag ttctgggtct gctcttaccc 960
agtccagtga ccctgggaaa gtcaacaaac ctctctgagc cagtttcctc atctgcagga 1020
tcaggatgtt accagcctgt tctgaggatc aaatgagcga ataaatgcaa agattctttg 1080
taaactgtaa accaacattc tccaaactgc attctgcaga ccctgctctc aggagaggtg 1140
ttcataaact atttaggaaa acaaaaaagt tttaggtcaa gtttgaggaa ggctagccta 1200
aacaaagcaa actagatttc ctcactctag aatttctcag aaactaactt gccaatgtgc 1260
atagcaaatc tccaagagca gggtagaaaa catggcttta tgtcaaaact gctttttca 1320
tggactactt attaacttct ttaggttagt ggtttccaca gagcatagca tatattaata 1380
ttatttctcc tacaaataat aatgtgaatc tttccacaag atttattcca tcatccaagt 1440
cactaatcct gggtaatacc agttaatcag tgtctgaaaa aagtattgct ggagtccctg 1500
cctttaatct tctgggcatt gtgctctgtg ctgtggggga gaggggacct gctattgaag 1560
gcctctgcca ttggactcca gccctaactc ccgccatccc catgccgcac tctgcccctg 1620
cagcttagtg aacccgctgc cctgtgtatg catcttttgt cttcctgttc cctgcctttg 1680
ttccatcctt ctccctgcca aggtgcctcc ctcccatttc taccagtaca gttgtcaccc 1740
attcctcaag tcttagccca aatcccaact tctccatgga aatttttcta actaaagcga 1800
tgtgttctac acaggactac tgaccccttg aactcaactc taagccccta aaggcagaga 1860
gcattactta tacttcctga ttttctccaa aaaccagcat tcatttgtgg gttgattgat 1920
tgctcccttc caccccaccc cccaagaacc ataaggcaca cagttgtacc ttgggaactt 1980
caacccagag gtatgggtcc ttcagagctg gggagtcagt ggtgagttta cttatttcat 2040
tcagtagaca cttattgctg gggaactgct gggaactcaa agttgaacaa gacaaggtct 2100

ctgcctttaa gaagctcttg gtctagacaa aagcataaaa acgtcattgc aagtcaatgt 2160
gctgagttta ataattgaaa aaatactgga gcacaaaatt gggttcgtgg tgagagtaag 2220
tggaaatcga ggcaggcttg tggagaggat gtcacctgga gcagatgtgg ccgggttatg 2280
gggaagaagg acctctgaga ggggattcag atctgttccc tgggtcacag ggaggaatgt 2340
tgtaatggag ccagtaggtt acagcagttg ctgaaagagg gtgtgagatg ggacctttgc 2400
atcatacaca cacaaacaca cacacacaca cacacacaca cacgtctgtg ggaatcccac 2460
ttccctgctt ttccatcata ccctctcacc ccttcttcct tctcaccttg actcattctt 2520
cctctcctcg tctctcacct ccgggtcctg gggccatcca tcgttacctc atcgggacag 2580
ccccgtgtct gctgtctgtt ggtagctaga ggcctgcttt ggtgacaggc tggtcagtgc 2640
ggtgcccact ccccatgggg ggccctcagg agggagagca gcctcaactc accactttct 2700
cttttcttcc cacagaaaac caaaagagac gtcaataatt ttgaccaaga ctttacccgg 2760
gaagagccgg tactcaccct tgtggacgaa gcaattgtaa agcagatcaa ccaggaggaa 2820
ttcaaaggtt tctcctactt tggtgaagac ctgatgccct gagagcccac tgcagttgga 2880
ctttgccgat gctgcaagaa ggggtgcaga gaagactcct gtgttggaga cactcagcag 2940
gtcttgaact acttctcctc ctcggagccc cagtcccatg tccactgtct atttattgca 3000
ttcccttgcc ccaggccacc tcctccccct cccacctggt gaccagaagg cgctctcggt 3060
tcttgtctca ccagtaatgc agactcattg ggtcagcaat tagctgtata cactgccgtg 3120
tttggaccat tggcaagcct ggttccactc ctcaggggct cctggcagtg aagcaacttc 3180
agttctttta ctgcaaagaa cagaaaaaag aaagaaagca aacaagaaga ctccggctct 3240
gctatcggac acagatcctg atccctcttg cttcttttcc ctcctgcacc gcagcttgcc 3300
atccctgccc ttctgtcctg gagaagagac tggtgcttct ccgcacacac gagggagggc 3360
gcccttgagg catgccctct gagggaggga gaccagagat gcagggattg gccagctggg 3420
ttggtttgct ctggaatggc taactcttac ctgctttggt tttagctttt cagcatgcca 3480
aagtcatgta agtttgtgtc ttgtggaaga aatcctcttt gtggaaaaag aaacagggtt 3540
ttgaactctg ttaacatttg aaaaatatat tttcaaattc actttctaat tggccaaaag 3600
agatgagttc cagtctgaat acaggtagat attaaagggc taataaaaaa tgagaaaccg 3660
gtcgtccaag gtggatgctg tcaatgcccg agtgacacat gagagctgta tgaattgaga 3720
gaaaaggcaa caagtagcat tcttcatcat tcaagttcta cctggacaca aaggcgagga 3780
ccctggggtt ccaacaaagc tcagctccca gattctcttt ccagtttcat cctaagttcc 3840
tagcataaac actatttatt ttctgcagca gtgtgttatt tttgcgcact tatacaaaat 3900
ggtagtacta ctgtgttgtg gtttttaaac attaaacatg taaagttata tacgaaatat 3960
ctgcttttgg aataagcaga atgaggctaa acatgggtta tacaaagggt atctggaaac 4020
tgaagagcaa cttgttagaa aactgacaat gtcgcaagat gtactcagtt ttgtttctgt 4080
gtgacatgca atggcaactc atgtggacac tattgaaggg atgtgacatt acctcctgta 4140
gatatgctaa cagtgttatt ctttcatttc caagggttct ctgtggcttt gtgtatatgt 4200
ttcccagagg tcatttgatt acctaattta ctgaactgat ttagcaggga atggaatcca 4260

ttccaactat tgcacgtgga tttcccagct gcccctaaat atatatactt gtgagtggca 4320
aagtggcact aatgaagctt ttgccttttg tacatttgag atttttgtat atagtgtttg 4380
ctgcaaggcc tgtggaatta attcgttgca tatagaggta tcaactgctg catgttcagg 4440
catattataa aactttagtc tatgaaagaa taattataat aatgtccagg tgcaatactc 4500
tgtaagtcta ttggttcaag ttaccgagag ataggtgtgt tcctttatgg gggatggggg 4560
ggtgtgttgg ggattctttg tattgtttat ttcattttgg tttattttaa aagatgtaaa 4620
catatattaa gctatattaa atctcacata cagttcttct gtgctctatt ataccctgat 4680
agagatgggg gagagaaagg aatgtttttg atggtggttt caaagctcgg acagtaacta 4740
tcttgagccc attagagagt ctgtgtccat atttgcatct ggctggtcat agcctttgtt 4800
actaatgatg acattcagtt ctcttttgtt tttatttttt aaaaactcag gtgtaattat 4860
tatctgttct taagataatt gcaaatatta aatattatga tatatc 4906

<210> 94
<211> 3713
<212> DNA
<213> Homo sapiens

<400> 94
ccgctccggg agagttaggg ctccgagccg agcgcgcgga gcagctgggg ccggggcgcg 60
gatgctggaa gttcacatcc cgtcggtggg gcccgaggcc gaggggccca ggcagagccc 120
ggagaaaagc cacatgatca agaagctgta caaagtgccc gacttcccct cgaaacgcct 180
gcccaactgg aggaccagag ggttggaaca gcgccggcag ggcttggagg cttacatcca 240
gggcatcctg tacctgaacc aggaggtgcc caaggagtta ctggaattcc tgagacttcg 300
gcacttcccc acagacccca aggctagcaa ctggggcacc ctgagggagt tcctgcctgg 360
cgacagcagc tcccagcagc accagcggcc tgtcctgagc ttccatgtgg atccctatgt 420
ttgcaacccc tccccaggtg aggaggtgcc tagatatggg gctacagggc tgggttgtgg 480
gctttgcatt tctcggctcc tgggaccctc agacagcatc tccttcctgc tgcccacctc 540
tggagccact acctcctgct cctgtcccct ggagcctgct ttgtagacac aaggaaaaca 600
agtttggctt ccctggtctc catttcttca gcagcctgac ttctttacca agctgatgtg 660
aaaagaatgt gacctgggaa tgcggaggct tcatttgggg tggacagctg ctgtctgctg 720
ccttggcagg ggctcctact cccaagtggg ggctgagccc atgagcagga gctcaaggag 780
ctgcaggtct gaggccagac ctgtttaatt ctatcccaca gagtcgctgc ccaacgtggt 840
ggtgaatggt gtgctccagg gcctctacag cttcagcatc agcccagata aagcccagcc 900
aaaggcggcc tgtcaccctg ctcctctgcc accgatgccc tgatcagtcc agaggccttt 960
ggctgcctcc taagaaagtc atgtgcctct gtcctatgaa ctccatataa ggctgggtcc 1020

tcctttggcc tggacccagg acttaattac ccagtgccca gttgtgccac attcccactc 1080
aaggctcaga acttggctcg cattggtagc tggaggtggt agaatttgta tgctcttaga 1140
gcccaacagc caaggcaggg tcaagaagat aagtaataaa agaggaagtc agccaggcgc 1200
ggtggctcgt gcctgtaatc ccagcacttt gggaggccaa gatgggtgga tcgcctgaag 1260
tcaggagttc gagaccagcc tgaccaccat ggagaaaccc cgtctctact ggaaatgcaa 1320
aattagctgg gattgcaggc atccgccacc atgcccggct aatttttgta tttttaatgg 1380
agatgggttt caccacgttg gccaggctgg tcttgtactc ctgacgtcag gtgatccacc 1440
cgcctcggct tcccaaagtg ctgggattac gggcgtgagc caccgtgctc ggcccaaggc 1500
aggagaatct cttgaagcca ggaggcggaa gttgtggtga gccgagatcg cgccactgca 1560
ctccagcctg ggcaacaaga gtgaaactcc gtctcaaaat aaataaataa taaaggaaga 1620
ggtcagctgg gtgcagaggc tcatgcctgt aatcccaata ctttaggagg ccaaggcagg 1680
aggatgcttg ggtctggaag tttgagacca gccagcgcaa catagtgaga cctgtctcta 1740
ccaaaaaaaa aaaaaaaaaa aaaaaatcaa tttagaggta ggctatgaaa aagtacagat 1800
acatccctta acatagactg gagggtggtg gcaaggggag caaatgtgga ggcccagtag 1860
ctggcaaagt ttagaagcag gggcaaaagc ctcctgcctg acttttcctg ggtagctcct 1920
gggtcaaaat ttcaggcagg atcccaggga aggggcaggc acccattgcc acaggagagt 1980
tgcaggcatg ttgggatgta ggcctagggg aggtgctgca ggctcaagaa ccaggcccac 2040
acattatata ttaaaaaaaa aaaaacccac attttttttt attggtcagt gttggtagga 2100
gtttgttaca aaagtgagtc catgggcctg tggaatgtga ggggagtggg tccgctccac 2160
cagatgccag caccggggcc agtgcagctc agagccctgt ggcggactac agggcctgca 2220
cagacggtca ctcaaagaaa gatgtccctg tgccctactc cttggcgatg gcaaagggct 2280
tctccacctc gatcttgccg cagtctgcga tgatcacatc cttcaggggt ttatcccggc 2340
tgtctgtctt ggtgctctcc accttccgca ccacctcctg gaaaagaaag gtggaagcag 2400
gagggcatgg tggatcagga ggtccaccgc tcaggagaaa ggccccagcg tatggctcag 2460
gagggctaag acccacaagt gatcaacagc acacaaaact ggaggcacca aaattctaac 2520
agactcctgg ccagagcagg gagaatgcag atttgacgag gggtacaggg aattttgttc 2580
ctttgaagta agacccaggt tgggccaagg gtgaggagga ggaagagggt gaccagggca 2640
tgtggcttct cagggacatt gcgttcagct gcactctgta tacctcaggg gtgggaccag 2700
cacgtcactg agtgaaggag gggagggagg ctctggcagt tgtgcagcct tcctggctgg 2760
gctctgaggg ggctggaaga atttagaacc ttggaggcat ggaggtacag ggtttattct 2820
ggacaggagc actgggctgc atctgtgggt tgggtccttt tgggaaaggg atggacacat 2880
ggagctcctg ccctggggtc tgtgttgaat ccccggtgag gattgcccag tagtagccct 2940
tgcttccaca actcaccatg ccctctagaa ctttgccaaa caccacatgc ttgccatcta 3000
gccaggctgt cttgactgtc gtgatgaaga actgggagcc gttggtgtct ttgcctgcgt 3060
tggccatgct cacccagcca ggcccgtagt gcttcagttt gaagttctca tcggggaagc 3120
gctcaccgta gatgctcttt cctgggaaaa aagacagagc aggtcagggg cgctggattg 3180

cgccaaacca agcagacatt cggggccagg actgaggggg cttaacctgt cctctgtgcc 3240
aggctaggct ggagtggact acaaggacat aaaagcagcg tccttcctat cttctggcct 3300
cagagccaag ccatgctgac tgaggccaag tggggcatca ggccaggctg atgtggtgaa 3360
cagctcacag aaggattact ttgagaagat agttttgtgg cttttaaata aggcgcatgt 3420
tatcagctcc ccttagctat ggcccaggcc tgccacggag ggactttggt ggagggaagt 3480
gccgtgcagg atgcagggga gtcaacaggg tcggaactct ccagaaaagg aggactgctg 3540
tggctgacca gcctgttttc ctatgagcac aaaagacaca ggcatagctg cagaggagaa 3600
agcagccaga gatgtgtggg gaggggcaga aaagaggggc tgccccttgg attctggaga 3660
gtcaaagcct cttaacaagg aatgaacagg aataaaagac agctgtgagt gtg 3713

<210> 95
<211> 3226
<212> DNA
<213> Homo sapiens

<400> 95
aatagaactg ggagctgagc ctggagcggt tctgggcttt tggttctctg catcaacaca 60
gccagcatgc ctatgatttc tgtgctgggc aaaatgtttc tgtggcagcg tgaagggcct 120
ggaggacgat ggacttgtca gacaagtcgc agagtgtcct cggaccccgc ttgggctgtg 180
gagtggatcg aacttcctcg gggtctctct ctatcctctt tgggatctgc tcgaaccctc 240
cgaggctgga gcaggtcctc ccgcccttcc tcggtggaca gtcaggactt gccagaggtg 300
ctgggcccct ggtggtgggg ggaaggagga cgtcatccat ataaagggga tctgcagccc 360
ccacccacgc ctggccagcc agcttctggc tcccttttcc ggcgggcgga ggcgctatcc 420
ggcggcgggc cgggaggccg cccccgtgcc ggtctgctct gctcggcgct gtgccagcag 480
gcggagagct cgcgccttcc gcgctgacgt cagcgcatcc cgggccgtat cccgggagac 540
cctgttgcgt ggtgatgggt tgccagggag acatacacct tttctctggg cctgggccgc 600
agctgcgcgg agcgccgggc acggatggcg gcggctgagg ggagcgaagc gagggaggga 660
gagcaagcta agaaacaccc agcaggtgct cccccgccta ggcctggctg gaggctactg 720
gcgccaccct gggggccctg tcagccaggt acccaagggg agggatcgag ggtgggcctc 780
aggtcaaggg gcagtgttgg ctgcccttgt gagggacggg aacgtgatag aagagagctg 840
ggcaatgccg gggagggatg tgtgcctcca acttcattaa gtgagggaaa catttgctgg 900
ggcttgtcag ggagccctga gccaggtaca gggtggagtt aggaacttac gtgcatcaga 960
cttaggcctt gccatcctga gctccctcgg gagacaggca agggcaatga tgggggcggg 1020
gtgtgtcaga gaaaagaaga ggctgtcggc tgaaagcatt atttgcaggt gacacttgag 1080
atgggcctta agtgatggca agcatttttt cacgtaagga tggcattctt ggcccagagg 1140

aaagctgagt tcctttcct ggaggcaggc gggctgcttg ctgacaggga ttggtggaga 1200
agggtttttg tttttgtta tttattttta tgtatttatt tatttgagac ggagtttcgc 1260
tcttgttgcc caggctggaa tgcaatggtg cgatctcggc tcaccgcagc ctctgcctcc 1320
ggggttcaag cgattatcct gcctcagcct ccagagtagc tgggattaca ggcatgcgct 1380
gccacgcccg gctgattttg tatttttagt agaggcgggg tttcaccatg ttggccaggc 1440
tggtctccaa ctcccgacct caggtgatcc gcctgcttcg gcctcccaaa atgctgggat 1500
tacaggcgtg agccactgcg cccggcctga gaaggggggt tttattgggc agaggagatc 1560
agcagtggat tcaaaggagg cttggaagga ggcaagggtg tcacagagtg ggatccttca 1620
gggcctgggt atgatgcctg cactaacctc actggacagt agcgtaggct agacaagatt 1680
ttagagatgt gttgtgacca gctgcactcc aggaaaactg tttacattat atcttacctc 1740
attcatccag cctttgcatt tttgtttgct tgtttttgag acagagtctt tttctgtcgc 1800
ccaggctgga gtgcagtggc acaatcttgg ctcactgcaa tctccgcctc ctgggttcaa 1860
gcaattctcc tgcctcagcc tcctgagtag ctgggataac aggcacccgc caccatgccc 1920
tgcccatttt ttaaattatt tttagtggag atggggtttc accatgttgg cctggctggt 1980
ctcaaactcc tgacctcaga tgatctaccc accttggccc cttggcctcc caaagtgctg 2040
gaattacagg cgtgagccac cacgcctggc ccagcctatg catttttttt tttttttttt 2100
ttttgagatg gagtcttgca ccgtagccta agctggagtg cagtggtgcg atctcggctc 2160
accgcaacct ccgcctcccg ggtcctggtt caagcaattt tcttgcctca gcctcctgag 2220
tagctgggat tacaggaacg tgccaccatg cccagctaat tttgtatttt ttagtagaga 2280
cggggtttca ccatgttggc caggctggtc ttgaactcgt gacctcatga tccgctcacc 2340
tcggcctccc aaagtgctgg gattacaggc atgagccact gacgcctggc cagcctatgc 2400
atttttaaga aattattctg tattaggtgc tgtgctaaac attgggcact acagtgacca 2460
aaacagactg aattccccaa gagccaaaga ccagtgaggg agaccaacaa ggaacaggaa 2520
atgcaaaaga gaccattatt actcactatg actaagggcc acaaatgggg tacgttgatg 2580
gagagtgatt tgttaagaga ctacagaggg aggacagact accaagaggg gggccaggaa 2640
agctcctctg acgaggtggt atttcagccc aaactggaag aatgagaaag agctagccag 2700
ccatcagaat agtccagaag agatggggag cactacactc actacacttt ggcctgagaa 2760
aatagcatgg gattggagga ggctggggga acaccacttc tgccgacctg ggcaggaggc 2820
attgagggct tgagaaaggg caatggcagt agcagtagaa aggacagggt aggagcaggg 2880
actttgcagg tggaatcatt aggtcttatc aacagatatg ggcaagcaaa gccaggggag 2940
aattgatggt aatgctgagg tttggagcca ggctagatgg gacagtggtg ggtgatgcaa 3000
aggaaagagg tcaggaagca gggccagacg tggggagaag gtgtgggggt ttggtttcca 3060
tcttgccgag tctgccggaa tgtggatggg aagaccaaga ggaggagcaa ggggcagagg 3120
ggaagggaat cttaaagaag tcctggatgc cacactcttc ttccttcctc ctcttccctc 3180
tcctcagagg tctcactcgt ggttcttcat ttcctgccca ccctcc 3226

<210> 96
<211> 3804
<212> DNA
<213> Homo sapiens

<400> 96
cagaagagtc taacataaga gaaatggtca gataggaggg cagaatggat cactgttaat 60
tatgaggact ttggaggcaa actgcccaga ttcaaatccc tgttccaccc cttgctagca 120
gtgtgacccc aagcaagtca tttaacttct ctgtacttca tctgtcaaat tcactcatca 180
caatacttaa ctcacagggc tgccgttaat tagttaatgt ccacatgtat ctgattagaa 240
gagcacactg tgtaacacag cccattacga ctgctctaat tccagcccac agggccagct 300
ggccggggcc cctttccaca gcagtggtga gtccagatgg gccacacctg ctgctgctcc 360
agggctgcat ccccttgtgc tgtcctccca aggctacatc tgcaagtgga gaggatttgg 420
ctatcactca ctgggggaga gataagtgtg gaagcaggag ggggaattgg ggaaggaggg 480
aggctgcgtg tgggacacct tcggaagccc ttgtcttctt cgtagtcccc agggaaagga 540
acgcctgcct gctctgtaga gggcaggctg agctctgagt gtgggcttcc tattctcaca 600
gcagctagtt tcatacttaa aagtctgaag ccagatatat tctgccacca gattaatttt 660
tttctgaagc tcccatttgg tctgttggag gaattcacat tcctccggct gtcataactt 720
tgctctgtct ttcaagagct cacacccctg gccttgtttt gcaaatagag cattctttct 780
ctcctgcctg tcctttacct tacaaagctt ccccatactg aacaaaaaca ttttcaactg 840
ctctgtagtg gaaggtaggg aggccggggt ggaagtaaag ggtaattaaa taatttctgc 900
tgcagcacga aaagctgaac agcatgaagt attattaata cgatgtgctc ttcctaatga 960
attaacacag gcaatccctc tctcctgtct ggtgttcagt aaatatttgt tgaatgaatg 1020
aataacagct tatgattctg tctttcctgc tctgatgaat tatgaagtcc atgtagccaa 1080
atcagcatca aacccatttt atatgcagtc tgtgttttga gccccaggtc aaagtgaata 1140
aaatatgtgt gaaaatgaag taataatgtc cattggagac cactggcttc ctgtatttgt 1200
aggggatagg gtctacctgg cttggtattg gtgacttgag agtagccttc cagctgcttt 1260
tctctggttc ccaggtaaac cagggtcagg ctgtcccttc taggtggaac tctgttgaga 1320
ccccacaggc agctctggtg aatgaccagc atgtgcaaac tcattgctct ccagctgacc 1380
ccaaaaccat gcaggaagaa gggcaattcc tgcaggactg agtctgcccc cggctgagcc 1440
ttgctttgct cattgcagat atttaagtct ctccatagtg ctgtgactca cctgtctgtc 1500
cttggttgga attccacagg gaaatcacat gcagaacctg gtcactatct gtacacaaag 1560
cacatggaga gattctaggg acaatcattt ctaagtaaat tacctaaggg cagaagagtc 1620
tgcccgggag ctgggggaga atggagcagg gacaggagat gggaataagt gacttgtaca 1680
ttaatcacaa agcaatcagc agatgacaag ggccttggaa accaccacgt ctgtctagct 1740

ggacgaggtg aaagaaaagg tcacccttgt ctgggaactc tcatccatct tcaagacaca 1800
cacttacctg gcttgcatgt gcctgagctt cctgtttttc ccgcccagca gagatgctgc 1860
cctggcctgg ttcattgtcc attatttgcc ctctgggcca tcccagcatc cacctggcct 1920
gccccacctg gcccaggcca cagaggaggg catggctttg gctagttgac ctttaggaag 1980
tcatttactg ggtgtaaccc acctcagttc ctgagacctg taaactcctc tttctctgcc 2040
ctgaatcttc cccagttatc agggcccaag caggagagtg ggcgtgaact ctcagcgctc 2100
ttcccctaac tttttccctt gatcctcccc agcttcttcc ctctcctggc aacccacccc 2160
ctgcccactg tcccctgaac acagaggctc ctgatggatg gagctgtctt cttacttccg 2220
ctggccctcc ctgggctgta cactagccct tggatcaaaa cctgttccca agttcagatc 2280
aatttctttc tgacatattt tcacaaaagt gggttttgtc tcccaggtca tttgaatttg 2340
aactggagca atgtccttgc ccaggtgtaa cccttggcag tggattttgg cagtggtgat 2400
gagtttggag tagaaaaaaa gagtcctgat tagaatggct actccatggc tcagatcaca 2460
gatagagcat gagatttgga tttaggcctt ggatttattt tattgattga ttgattgatt 2520
gattgattga gacggagttt catgctttcg cccgggctgg agtgaagtgg tgcaatcttg 2580
gcttactgca acctccacca cccctccccc ggccctcacc aggttcaagt gattctcctg 2640
cctcagcctc ccgagtagct gggattattg gcacctccca gcactttggg aggccgaggt 2700
gggcagatca cctgaggtca ggaatttgag accggcctgg ctaacatggc aaaaccctgt 2760
ctctactaaa aatacaaaaa ttagtgaggt gtggtggcac atgcctgtaa tcccagctac 2820
tcgggaggtt gaggcacaag aatcacttga accctagagg cagaggttgc agtgagccga 2880
gatcgcacca ctgcattcca gtctggacga caaagtgaga ctccatctca aaaaaaaaaa 2940
aaaaaaaaaa aaaaggacaa tgcctgagca acattacctg ggtcccctag cccagcaggg 3000
ttctccttgt ttcagccaaa gatgggaagc tgacttggtt aggctgggca tccagacacc 3060
gaggctgaat agaaaagaga taagaaaatt acaacttaca gactgaccct ggggatgcca 3120
gagggtggtc cagatggagt aaaccccaaa aatgaccttc tctggtgtta gcctccacat 3180
gcattctgtc tattgtgctg catggcttgt gctatggtct gaacactggt gtctccccaa 3240
aattcatatg ttgaaactta attcccgagg tgatagtatt aagaggtggg gactctggga 3300
gatgatgaga tcatgaaagc agagccctca tgaatgggac aagtgccttt ataaaagagg 3360
ctagaggaaa gctgtttgcc cttctgccac gtgaggacac atagagagca ccacccataa 3420
agaatgggct ctcaccagac accgaatctg ccagcacctt gatcttggac ttcccagcct 3480
ctagaattgt gagcaataaa caactcataa agatatttat gaggccaggc acggtgactc 3540
atgcctgtaa tcccagcact ttgggaggcc aaggcaggtg gatcacttga ggtcaggagt 3600
ttgagaacag actggccaac atggtgaagc cccatctcta ctaaaaatac aaaaattagc 3660
tgagtgtggt ggtgcacacc tgtaatccca gttactaggg aggctgaagc acgagaattg 3720
cttgagcccg ggaagtggat gttgcaatga gtggagattg tgctactgta ctccagcatg 3780
ggcaacagag tgagaccctg tctc 3804

<210> 97
<211> 4865
<212> DNA
<213> Homo sapiens

<400> 97
ggcctttttt tttttttttt tgtcatttat ttatttatgt gcagacttca agtgctcagg 60
cttagataac ttttggtaaa agtacactct catctttcgc ccactgggtc ctctttcttt 120
ctttgctatt ctttgaactc gttctctgtg gtttattttt ctatgccagg agcatatatt 180
atttgtatca gtcctaatgc ttagcttaca atagtctgac atggtcctcc ttacagagtt 240
actcattact catcctctag tctgtttttt tttttaacct ttctttgttg ttttggtttt 300
ttaaaaaaaa cctttccctt ctccctttttg atcacttgtt ccctaacttg catcttttca 360
ttttgacggg cctcaaaatc tatgtctcat tctgcacttc actactcata catccattct 420
ttctttcatt taggttcttt ttcattgtca aatgtcagag gcatatattt gagttacaca 480
taccccttag tccataatca cctgatatcg ctttgtgtgt tgccctatta cttagtgatt 540
cactggtttt tttctattta tatattgatt gcctagccat caaagcggat gaaccatggt 600
cttgtgtaca cacactctca tcctggcttg cttacttctt tacctattta cttgtttatt 660
cattctctct tgcttcactc attcattcat ctggtcattt attatacaat gcaatagata 720
ccagtgaaac aacctctcaa caacaacagc aacaaaactc agaacattca cagtatcttt 780
ggcattcctg cttcttctca ctgagtcacc aaccaaggta cacacagccc acagtccaga 840
aacacacaca aatggcggat gcagagcaga gacacagtga atgttaagtt tactcccaga 900
tccttctggg gctcgcgtcc taaacatact gggggcagat ctgagatgag ggcccacgtt 960
ccccaggccc cctgggcaag gcaagcgccg cctgttctgc ccctctggac cgtggtgaat 1020
gaccatcccc atgagaaacc tgtttcaaga ccccagaata catagagaca cacacatggt 1080
acatagtctc ctggtgggaa acgacggaca tggagttcat ttttaaatag gtggctttgt 1140
ctgtggcaat ataaaaatat tttggccttt caaggcaaat acaaaagaaa ctacccatta 1200
acatggctga gtgtctcagt cccaaacaat aaagaacttg aagaggttta gccggaaact 1260
ttgaagacag aaaatctgcc cattatgttt tccagacatt tcgagggagc gagcggaggt 1320
cattggagct tgaagcccat ttggaaggct ggtccctcgg cctgcgtttt ctccttggcc 1380
ctttgaaggg ccccccagct cacagggcac ttccatccta gtctgccctg cggcacaaga 1440
gccaaaggga gactcaataa tgaaagagcc attcatttag aagagtgaaa aagaagaaat 1500
tcttttgggg tccgacattt gaagcccgcc tatagaattc ttttgccaca gagacaacac 1560
aaggaggcag gggctcctg tcttagaaaa caggagcccc caggtctgca cttggcttga 1620
ctcgatgggc acccacctcc cggtgcacag acccccggcc agtgcccgcc agcccctcca 1680
cgaggtccgc ctggcggtgt gctccgggca gctggcccca ccctcagcag caaggccaca 1740

tgtgtgttct ttacactcca gccacttacg atttcgagct ggagaggggc tggggtccca 1800
cattcgaggt cccttcccac gtaggcatcc aggtgatggc ttccgcccaa accaggaagg 1860
agacgagagg cccgccagga agaagacttg ggtccgggat ggtgggcccc atcagggcct 1920
cttccgcagc ttccatccgc agttcttcag ccgacccagc cgggccacgg cccacgtgcc 1980
ttggggaagg gcagagagtt agaatcatgg caaggcaagg gccccggaaa agccaaacac 2040
accgaagaaa gactcggggc acggcctcac ctgcagttta cttttcggtg gagtggggtg 2100
actactgggg gatgcccaat gacaggaaaa ggtgcaaagc agcaacgggc tgggttaggg 2160
gcagccccaa atccagaact gagtggggag caaggaccgg ggcggggcag aggccaggac 2220
tctcagggcc acggcggagg caaaggcagg cgggggcaac acccaccagg gcacgatcac 2280
gaggggtctc tgggatggga caggagtctg tccatagccc tgaggccagg cccacaggag 2340
gaggcactgg aggatgcttg gcaggagatg gagacaccag caggggcttg tcagggcac 2400
tgggaacctg ctggaaacag gcagctggag tgggagtggc tcctggggca ggggagtggt 2460
actcagacca caggagggcc aggtgaccac aggtatgggc gtggcaggga gaaccagcct 2520
gctaggcaga gggctgtgga gctgagccct ggagcccagg gactgaggca gagaagggga 2580
ggttggtcag gcccacctca ggagacagag caaagcggag tgccgggtgg gtgaggaagc 2640
tggccgctgg agagccacca gggagagcaa gccagcagcc gggcagaagc agagcctgca 2700
gatgctccct ggaggtttcc cctgctggtc ctcggcatgg gaggtgggcc ccagcagctg 2760
ctagagagtg aaggagctgg accaagcccc gggggaggaa ggcatcacgg caagggaagg 2820
gtcgcagtca caaccacacc tcgagaggga gacctgtggc caaagcccgg gggggcacac 2880
actacagctg ttcccgtgcc cactttggtc ctgagagact cagggccagg ggtccagaac 2940
agttcagcat caggaatggg gcggggaagg cagagaggga gaaggggctg gctccgacca 3000
gagcagagcc ctggggatag cttgaatctg ccctcacacc ctccggccta cctctatgga 3060
gccacagtcc actgctccag cccctgggca accccccggc ccgccctcct ggggacacag 3120
aggccatcaa gggatgggct acggtggccg gtgcccaggc cagaccaggg ccgtgggcgt 3180
gctcagttag gatcaactgt tggatgacgg gatggccgcg cgggtgaatg cagggacgcg 3240
accagcaagg cccccgttca aatggggcag gggtcggaag ctgctcacac acctctcaaa 3300
aatgaattcc accctccagc ctttgcacct agaactcttc gcatggccca gctagtggct 3360
cagctgtggt ggtcgagtcc tttcattcac tcacctggcg aaaccaacat cccacatact 3420
ctaactgagc cccactctgt gccaggctgg tgctgggaca ccctgaggag acacggggct 3480
ggtcaagggc accccgggat ggccaggtca ggaactgggg agggacaaag agagggagat 3540
attgagagag aggaagacga ggaagaaggc aacagaagca gaaaaagtag ggactcaagg 3600
agccaggtta aagggctgcc cctacactca agggagcaac gagacccctc tgccggtgcc 3660
tcagaaaagt ccagaaaccc aagcaggatg gggacgtggg gagttaactt ttgaaagttt 3720
ttagggctcc aggagccttg ggcacaaaac cccagagaac ctgccggggg ccagctgatt 3780
tcttccacgc cctctccggg ctttccaatg tccacaggag ctactgcagc actaacagtg 3840
gctttggggc ctgtatgtga atgactgcca tgtgtttgag ggtgaggaga gtgccatgga 3900

ttcttcatta ctattgaaag tcttctcatt tagaaacatc aggcgtgctc tatttttcta 3960
ctctccaaca gctacatggc ggggaggcca ggttttcgtg tgatctcagc ttcagaggtc 4020
tacacccgag tccacccata gtcagagtgg ccaactacat caccgtcact ggaaagtctc 4080
gccttggcaa aggggatgaa aagattgcct caggcttttg ccccaaccgg ccaatgggag 4140
atccttccac attcttcctc gtggggtgct ggggtagagt tggttttgct aacttggaaa 4200
cagtatccga ttctaattca cgaagccaga gaacctttag ccaacagact cactaaggga 4260
ggaaggtccc gtaaggtttc aactaataac attttcttgc tccaactacc tcccttatcc 4320
cctagttttt ttttccattt ggctgaaggt ctgactggag cctaaagttt aattggagtg 4380
atttaatcat gattgatccg ccttggggtg agaaatgtcc agagcttggg acaaataagg 4440
gcatgtttca gactcgggac atacctgctc taaagaatta tgggctctag ggagctcaaa 4500
gatcagaaca aaaagaggag gagggttcag gcagggtaca ggacatgtgg gtgaggatat 4560
taaggggcaa aagaagaact actatagggc tgggcatggt ggctcacgcc tgtaatccca 4620
gcactttggg aggccaaggc aggtggatca cctgaggtcg ggagttcaag accagcctga 4680
ccaacatgga gaaaccccat ctctactaga aatacaaaat tagccaggca tggtggtgca 4740
tgcctgtaat cccagctact cgggaggctg aagcaggaga atcgcttaaa tccgggaggc 4800
ggaggttgct gtgagccgag atcgcgccat ttccagcctg ggcaacaaga gggaaactcc 4860
gtctc 4865

<210> 98
<211> 4762
<212> DNA
<213> Homo sapiens

<400> 98
tgcccaacaa aactaaatag aattcctacc tattgtagag ccccctttgt acacttcaat 60
atgaatttat ttgtaggttt actccttact ttagtatagc aaagtacaac caggatggat 120
tccaacctcc aggcccacat ctcccataaa tactcctgta gaaagggaag gtaggttctt 180
taatggaaac cacaagtatc tctttgaggc agacatctga agtcatgacc aactttttag 240
aaaatgtact caaagggctg ggcgcggtgg ctcatgcctg taatcccagc actttgggag 300
gccgaggtgg gtggatcatg aggtcgggag ttcgagaccg gcctggccaa tatggtgaag 360
ccccgtctct actagtaatg caagaattag ccgggcgtgg tggcgcgcac ctgtggtccc 420
agctgcttgg gaggctgagg cgggggaatc gcttgaaccc aggaggcgga ggttgcagtg 480
ggctgagatc gtgccactgc actctagcct gggcaacaga gcgagactcc atctcaaaaa 540
aaagaaaatg tattcaagga aggaaatatc agtaatatca gtaactgcct tgctttttaa 600
tgatttgctt cttcagttgt agataatcat gtcattcttg cttggagcta ttttaggcag 660

cctttactga ctccttcctt ctactacata gtaccaggaa ttgggagagg tgtggtttaa 720
gtcattcttt ttgtggttgg tatatttagg gaaagatttg gtataggaga aggtcaaact 780
aatatttcaa tgatggtttt agactgtgca tggctttcaa tcatctgagc aacttctact 840
tcaccatccc tggcccctaa catatataac tagttgacca ggattactga gtcacttggc 900
cttggatttt tttttttat gcctcccaag ttccatatga atacatttaa agcagggagt 960
ttagagcagg tggaaagttg gaagtctgtt tgattttgtc gaccgtagga aatatggata 1020
aagatacatt tgctagaact aaggagaaat aagagtagct ttaatgctgt gtagctctcc 1080
ctaaggccca aacaggagac gcaaactaat gtgatgatgt aagaattttt ctatttctca 1140
caccatccct tgcttccact ccaagcatgg tagtatctgg gtatttaggt gattggtgaa 1200
acacaccaat tgagtagcca aaatgaatgt gtctgaatga ctccccatga catggtttct 1260
ataccttgag gagagacaac atgtttcttc aggagacaag aagttgcttt ccctgtgtgc 1320
tccactgtct tagtcctttt tgtgctgcta taacagtgta cctgagcctg ggtaattcat 1380
aaggacctga gattgatttc tcactgttct gaaggtcagg aagtccaaga gcaaggcgcc 1440
ataggtttgg tgtctctact tccaggatgg ctccttgaac actacatcct ctaggggggtt 1500
ggagaacgct ggtactcatg tggcagaaga atggaagggg caagagaagg agagagggcc 1560
aacttgcctt ttataacaag cctagtgcca ctctaacaac ctactcctga aaaaaaacaa 1620
aaaacaaaca aacaaacaaa aacagtatta atccattcat aagggtgaag acctgatggt 1680
ctaatcacct cttaaagact ccacctgtta acactgttac tgttaaagca gcaattacat 1740
ttcaatgtga gttttggggg ggataaacat tccagccata gcacccaccc taccccctaac 1800
tctctctgtt gggcgccacc ccaggagcta catccctctt gacccacccc catttctcag 1860
ccacctgccc tggccatcca tccttggatc ccaggctacc tgctgccctc ctgggtgcat 1920
gaaaggaatg ttggccagta ccaacatttt tttaaagtgg ctactttgta aaagtaggaa 1980
ctcttggcat acactagagc atgggcacca cctgcaccat tccacgggta cctgcctctg 2040
ccagataccc ctgagggaag aggatgttct ataaccaggc cgacaggtta gcatttgtga 2100
acacagttct gacgttgttg ggagggtttg tttgccagaa acatccccat gcgctactct 2160
ttcaaccaga ggtcaagaag tcctttactt ttgtgtcttt tttgtttgtt tgtttgagac 2220
ggagtttcac tcttgttgcc caggctggag tgcaatggcg caatctcggc ttaccacaac 2280
ctctgcctcc caggttcaag caattctcct gcctcagcct cccgagtagc tgggatcaca 2340
ggtgcccacc accacgcctg ctaatttttg tacttttcgt agagacaggg tttcactgtg 2400
ttagtcaggc tggtcttgaa ctcctgacct caggtgatct gcccgcttcg gccccccaaa 2460
atgctgggat tacaggtgtg agccaccgcg cccagccttc gtgtctttta agctgcactc 2520
ggagcacagg caaggtgctt cctcagaggg aggttgctta acatggggag acatccacac 2580
aggagaccac agctgatcca tcccttgact gtcagcagca agtcccccctc tctgcatttt 2640
ccagctcatg acatagtttg gaaatagccc tgccctgggg tctgctctgt ggttgtaaca 2700
gtggagccct ccggtgacat ctgtgggcca agtcatttca ccatgacctt tggccatgcc 2760
acatgcagtc atctgaaagc tgcaccttct ggggtctgag gggtttcttg ggtatttttt 2820

cctgattgag acacatctgc tgggcccctg agagaatggg ggatacctgg cgggaccagc 2880
attcttcacg gagctattcc acgagctact atgagaacat ggtgactcag agaggctcac 2940
gtgtctgcac taaattcaga cctaaagtaa taagcagatt cttgggaact ttttttttaa 3000
ctgtacttga tgtccttttt gtttttgttt taaccagacc caagttcagg acctctgtgt 3060
ttatcacaag caggacttgt tccatttggg aacgaggacc tagagaacag cagcaaaaag 3120
ctgagccttc ccaggacctg gaggagggtg ttctgtgagc ttccacagag cagatgcctt 3180
aaaaacgaag gagctgtctg gaaaaaatca ggagagagag agagattgag atttgcagga 3240
aagcctttac tgccaatgct caacagaaag tctgagtgtt ccactaagaa aactgctccc 3300
cgagggagaa gtgggtccca tcctcttgag tttgatgagg gtgggaatca gctttgtaat 3360
ttttattttt tggcctgtgg atggttcgta agctggaaag ggtttgggtc cccaagagct 3420
ccccgatgca cagctctcct gtgtcctgtg tggggctatt taaggatgaa aggccaggac 3480
tgtgaatcct gatggtcaca gctctctcag cttaaagcta ggggaccccg ctcgaagggg 3540
accctgaagt accctgcacc ccaatatctg aaagtcctct gacctcgctt gcaccccctg 3600
gagcctcggc actcagtgcc tttgctgaaa gtagcagccg ccgcaccagt agtggtggat 3660
cagaggttag ttttcccagg agcctggcac tattggatca ggtttctttt caggagaaag 3720
agcagggaga cagggagcta acccaatcac ccacgtctat gctctttggc cttagacaca 3780
gcttggccag gtggggaggc gtccttccct ttgcagagcc cagagcagcc caggttcggg 3840
tctggcagtc actgtgagca gttttcaagc ctgtcccttt tctaccctct ccttctagaa 3900
ggtgctggac gaatgccaga accagcgggc ctgccacctc ctggtcaata gccgtgtttt 3960
tggacctgac ctttgtccag gaagcagtaa atacctcctg gtctccttta aatgccaacc 4020
tagtaagtaa cttcggaggg ggacagtgtg tttggggtgt ggttctcgtt tccttcacac 4080
tcctggtcaa aattctctgc ccgttggcat ttgtccaact ttgaaactca ttaacagaca 4140
cattgggctc catcctaccc agttttttag agacagagag agaaagaaag acagagaaag 4200
aaagagagag agattttgtg tgtgtgagtc tgcacatgcc ttcattttct tacaaatgcc 4260
tgaattttaa aaatcctttc caatatgtaa aactcagttg tactgatgct acaactctca 4320
ggggtggtga gagactgcaa ttgctgttcc tcaaagtgag gagctggttc tgcgtttcag 4380
aggtgtcttg tgctgagatt gagaggctgc ctgtttttcc ttgaggccca gtggcccact 4440
tgccacctgc gcccactgct tcagatcttt aagaaccagc tcctcccgcc ctcacccacg 4500
gcaggggctc cttgttcccc aggagaatga gaggggatat ggctggagcc atctctttta 4560
ttctgattct tgttgttttc tccattgact tttgccattt ctaatacgca tcagctttgt 4620
gatgggcaaa aataatccat cttccgtggg tgggtcacac ttccaggagc caagagcctg 4680
cctgagataa atcataggtc actgtggcat tagaggttct tgcacacttt gttgcaaaga 4740
aacgatagca gtgtgtttaa cc 4762

<210> 99

<211> 4312
<212> DNA
<213> Homo sapiens

<400> 99
tcatttttta ggtgacatta gaaatccgga tcatgttaaa tctagttttt aaatattggc 60
aacaaatcca cttgttaaaa actgcaatga tcaaacatgt ccataggtca aatctggcca 120
taggccaaca gtccatgctc tccagctgga catgtcaagg accaaatctt ctcaggcaac 180
tgctgatcga acctccatca agcctcaggg actggggcaa aatagggtgt ctacctagtg 240
gcccacaggc cacacaggct attccagaat ctggccctca acaacgactt gagaaggtca 300
cctcaattcc tttcactgaa ccttttttgt tttgagacag ggttttgccc tgtcacccag 360
gctggagtgc agcagcagga tctcggctca cttcaacctc cacctcccag gttcaagcaa 420
ttcccctgcc tcagcctcct gggtagctgg gattacaggc atacaccacc acacccagct 480
aatttttgta tttttagtag agacggggtt tcaccatgtt ggccaggttg gtcttgaact 540
actgacctca agtgatctgc ccgcctcagc ctcccaaagt gctgggatga taagcatgag 600
ccaccaagcc cagccctttc actgaactct taaatggccc tttccaggcc aaatagcgtt 660
gcctggaacc atttagtacc atgtaccatg gttctcagat ccagaaacag gatccgatca 720
cctatgcatt ttctaataca taagccctaa gttattttag gtctataaaa actatacgct 780
aaggtggggg taaaaaatct cacatcaaat ataaaaaatt taagtcatta aaggagatga 840
ggcatttgta aagaactttt aaaaatatct taagagctgg aggggtcttt ttgttggttg 900
gtttgggttg ggttttattg gtttgtgttt agagtctgga gtatttgctt tttaaaaagg 960
cagtgaaaga aaaaaaacga acccccttcca aacacatttg tcatttttat atactatatc 1020
atgcatgttt atgctggcta cattaggaac aagcttgaat tacaaccccc ggtttaccta 1080
atggagttct gaaaagccgt ccaatcttcc tggaagaggg agttgggagg aatgtggtcc 1140
agtttgcact tctttcgtat ggactggaga gtgctggtga aatcagacac atacctgccg 1200
ggaaagggaa gaaaatggta gggaggtcac cactgattcc ccaactctaa agagacctgg 1260
aattctacag ctctggacaa attgctaatg gccttgagag catctttaga ggctcccacc 1320
gacgagtgag actgcttgta aacctctgaa cacaaaaagt tcttctctat gtggcaagac 1380
tgtcttcacc acctggttga gcagtttcta aaagagatgc ccaagaggag aaggaaggaa 1440
taatcgtgaa gttgactcca gggagcaacc aggtgagaga tgatgctgat aaatcccccc 1500
tcatccagat aagtacattt ctgtgaaagc aggtttgaag aactgtttaa acatcacggt 1560
cccatggtaa taactgactg tgacctggga taacgtaagg aaggtcatga catccaaggt 1620
acctctgcat cattctcatc ctctaaattt agattaaata cccgtggcaa aaagattaaa 1680
gtttcacggc tggcaagtgt ctgactttaa tgggacagac gcatgaaagt ggattcaaag 1740
ccaggagatc ctccctcaca ggctgcgttg tttgctctca ctgtacacac atacttttcc 1800
ctcacagcat ctaaaacact gtaattaaac agcttgttta gacagggcat ggtggctcac 1860

gcctgtaatc ccagcacctt gggaggctga ggaaggagga tcgcttgagc tcaaggagtt 1920
cgagacctgc ctgggcaaca gggtgagatc ccatctctaa aaaaaacaaa aaaattaggc 1980
gggtgtggtg gctcatgcct gtaatcacag cgctttggga ggccgaggta ggaggaccac 2040
ttgtgcatgg aagttcggga ccacactggg tgacgtggcg gagctttgtc tctgcaaaga 2100
atgcaaggat tagcctggcg tggtggcgca tgcctgtggt cccagctgct caggaggctg 2160
gggtggaaag atggcttgag cctaggaggt cagggctgca gtgggccatg agcacgccac 2220
tgcactccag cctgggtggc agagcaagac cctgtctcca gataaagaaa tagcttgtat 2280
gatgcttatt gctgctgcta gactttaagc tctgggagaa cagagtctgt gcctcatact 2340
tatatcccca ggaactagca cagagctgga cataaaggag ggagtcatgt cctctgaaag 2400
ctacgaaacc tgtgcctcat acttatatcc ccaggaacta gcacagagct ggacataaag 2460
gagggagtca tgtcctctga aagctacgaa acctgtgaca tttgcatgca tcacaagagg 2520
ccttgccgct tgcccctccc aaaacgaagc tctaaatgag gaaagtgaaa tgtgaggacc 2580
cagggcacag ccccaaaagg tcagtccatg cagtggagac acaggccact caatgaacca 2640
actggaagaa acggcttcag gtcactccac tgactgcaaa tgatcctgct gctcccttta 2700
cagagagctc gatgctttca ggtggaaagc tgcaaaaccc agagtaccac tgacaacacc 2760
ttccccttgc accaagagag gtgacagctt tctagggtta taagtcccag gaaggagagg 2820
ggctggcctc aagcacaaac tgacagggga atgttaagga acatgattgt gctttagcca 2880
tgggaagtta aacaaccatg tcaccacctc tgcaactatg gcaaatatcc atgtgctctg 2940
cagaaagagc aggtacacac tgcaggagaa ggagttttgt ttagccacta ttctcaggta 3000
tggttataag caaatgcttt ctctatttca ttagctgtca atccagaatt ctaaatggtt 3060
tgtcatgaaa tctcagagga aaagtgtagc tttcatcaca tcatttttgt tcccaaaccc 3120
tccagctgtt tctcacaata cacatttcca attgtcaacc atgtcagagt caccgtgtta 3180
ggggctttaa tgatatgctt ttggttgata ttcacaacct ccttggaaag gagatattgt 3240
catcatcagt ttactcagag aggaacacta ccatctctaa ggtccccact agtaagtgtc 3300
cagagctccc atgcccatca cagtcttaat acccctcctt cttgaaccac cggttggcca 3360
ctatactcta tgcttcatcc tagttcgagt taggccaacc tgcacctgcc tttcctgcgt 3420
accctgttct ccttgcccct gcctccctta atacccttcc ccaaaccctc ctcactactg 3480
aacaatgtgc catttgaacc ctgctcaagt tagcttcaac ctgatttccc tgattttata 3540
agcatcagct attttggtaa cttcaaaatc ttccctttta gagaagatgg cctgggtttt 3600
ccagatatac ttttgtacag tttgaatgct taatatggaa atgtattatt ttgatttttt 3660
tttatttttt atttttgag acagagtttt gttcttgttg cacaggctgg agtgccatgg 3720
cacagtcttg gctcactgca agctctgcct cttggattca gcaattttta tgtctcagcc 3780
tcctgagcgg ctgggattac aggcatccac catcatgcct acctgatttt tgcgtttttg 3840
gtagagatga gcttcgccgc gttgaccagg gtggtttcga actcctgacc tcaagtgatc 3900
cacctgcctc ggccacccaa ggtgctggga ttgcaggcgt gagccaccac acccagcctg 3960
atgtttcttt ttctgcagga acacattcag gctcatccgg ccacaccaga tgatgtgttc 4020

ctatagaaat gcctacgtgg aactgccctg ggaggtgctg gatgcccgat ggcaatgcca 4080
gggagctctg ggtccaagga gcctccacag ttcacagagg catgctcagt atttctggaa 4140
caatcagaga cagggcttcg ccatgttggc caggctggtc tcgagctcct gagtttaagc 4200
ggtccacctg tcttggcctc ccaaggtgct gggattacag gcgtgattca ccgcaccagg 4260
ccaaaggtcc tttttcttgt tcaaataaac tttgttaaat ttaacttgtc ag 4312

<210> 100
<211> 4599
<212> DNA
<213> Homo sapiens

<400> 100
acttccgtgg cttcgggcgg ggaggtcaca atcacattga gccaaaacgc atccagtgtt 60
ttctccagtt acaaataaaa cgaatatgcc catgctgcta ccacatcctc accagcattt 120
cctaaaaggc cttttaagag cacctttccg atgttaccac ttcatctttc actcaagtac 180
tcatctcgga tcaggaatcc catgtgctca gccgtttaat tctcttggac tccattgtac 240
aaagtggatg ctgctgtcag atggcttaaa gagaaaatta tgtgtacaaa caaccttaaa 300
ggaccacaca gaaggacttt ctgataaaga gcaaagattt gtggataaac tttatactgg 360
tttaatccaa gggcaaaggg cctgtttagc agaggccata actcttgtag aatcaactca 420
cagcaggaaa aaggagttag cccaggtgct tcttcagaaa gtattacttt accacagaga 480
acaagaacaa tcaaataaag gaaaaccact agcatttcga gtaggattgt ctgggccccc 540
tggtgctgga aaatcaacat ttatagaata ttttggaaaa atgcttactg agagagggca 600
caaattatct gtgctagctg tggacccttc ttcttgtact agtggtggat cactcttagg 660
tgataaaacc cgaatgactg agttatcaag agatatgaat gcatacatca ggccatctcc 720
tactagagga actttaggag gcgtgacaag gaccacaaat gaagctattc tgttgtgtga 780
aggagcggga tatgacataa ttcttattga aaccgttggt gtgggtcagt cggagtttgc 840
tgttgctgac atggttgaca tgtttgtttt actactgcca ccagcaggag gagatgagct 900
gcagggtatc aaaaggggta taatcgagat ggcagatctg gtagctgtaa ctaaatctga 960
tggagacttg attgtgccag ctcgaaggat acaagcggaa tatgtgagtg cactgaaatt 1020
actccgcaaa cgttcacaag tctggaaacc aaaggtaatt cgtatttctg cccgaagtgg 1080
agaggggatc tctgaaatgt gggataaaat gaaagatttc caggacctaa tgcttgccag 1140
tggggagctg actgccaaac gacggaagca acagaaagtt tggatgtgga atctcattca 1200
ggaaagtgtg ttagagcatt tcaggaccca ccccacagtc cgggaacaga ttccacttct 1260
ggaacaaaag gttctcattg ggccctgtc cccaggacta gcagcagact tcttgttaaa 1320
agcttttaaa agcagagact aataaaattc atcctgtata ataattttac atatcatttc 1380

ataaagtatt ttaatagaaa aatcacttgt atgcttatat tttcagtaat tattgtatgg 1440
tgctcttgtc ttctttgttt gtgacccatg cttgaaaact tgaaggaagt tagatatgaa 1500
tggcaaaagt taggcagtat ttataaggta cctgttttat gttactgata tctgtttcct 1560
tctcttctta taccctggca tggtggcctg tagggtagtt tcttcttaat agtcaagaac 1620
agagaaagct gaggagagca gaaaatatct tgcactttaa aatgcttatt ttgattccat 1680
attatctgaa tttccagaga ggatgtaaac aagagtgacc atatttttgg agtccttctt 1740
gaagccagaa caacacacag tgggttgcta agagccgaca atcataattg acatttagtg 1800
taccagatta ttctaagaat ttctgaaatt accatgtaca tgagtaatga aacaactgga 1860
ataatgtgat gatgtgaaca caattttaaa gtctgcttgt ctggatttgc aggtaccctt 1920
ccgccacccc ccaacatgat aggtaagagg gcaaaaggga accgggaaaa atcagctaca 1980
aataaaatgg gtgactaatc cccagttctt ggacccatta tatttgtagc caattggagt 2040
aaacccctaa aagacaaagt gaggtcagaa tgattcaagg attattcagt ttgaagacta 2100
ccaaaggctt ttgcttctct tcttccttgg ccatttcact tcattttagt attttaaaaa 2160
tctttttctt tgatggtgaa caagttttgt cctatgctat ggaggaggca tagcacagtg 2220
attaagagct tgggctctgt agttggacta tgtgggttta aactttaaac ccagatcaat 2280
tgtatattag ctacatgagc taatctcttt aagcttccat ttcctccctg gtaaaatgaa 2340
attggcaaat aactttctaa ggttagtgaa gataggaaat actcgaaggc tgcttactat 2400
ccttattaca ttaaagttat gggtatcttg aaactacaaa cctgatgtat aatttcagtt 2460
ttctctcatg gtcttgttac taccatcata ctcttgatat tttaggatac tttaggatta 2520
cctttgaaga accctttcta gttaattctt tgaatatgtt ttatacttat ttgatggtag 2580
ttgtaactgt gtacatttga ataaaatttc taaccataaa atttcgccat tgcacaaaaa 2640
gcctttatag aagtttctaa catattctat tctgaaccct gtagaatatt ttatgtagta 2700
tagcagcttg cattgttact tactgtactg gaatcaaacc tcttgagcat tattttaaca 2760
aacatccctt tacatttttt ggaaagattt ttagtactcc attggcatag ctatttcata 2820
caaggattag gagaaacttt gaaaggttat ccacttgact tttctgattt tagaaaggac 2880
taccattaaa ccaccctatc agacaaaaaa aactggtttt tagaaacctg agagaaggaa 2940
ttttataatt tccctgagta agctgttaca gggaataaca aatctcctgc cacatgtggc 3000
acagtgtaag taatgctgga gcacacatac tgcattgtaa agaaggtagg aaaggtgggg 3060
ggacttttgt tagctttaat tttaaggcta aaagcatttc ttctcagttt gaactacttt 3120
taaatgttat aaatgtcatc ataaaaatca ttagaaaact catttctcct tatgttgtcg 3180
ttgtaccatt gggtattgta tagagaccaa tttctaagag atttttcac ctctgtttct 3240
gttatttgct atactggagg taatataata ataatgaaat atagcttttt ttttcaactc 3300
agaaatccta ttccctctcc ccatctgtga ttatattgta ctggacttct gacttggtca 3360
aaagcatagg ttttgactta gatacatgtg ggggcagatc tcagctctat cactaactag 3420
ctatatgata tttggcaagt tttttctcta tgtgcagtag tttctttatc tgtaaaatta 3480
agataatagc tactttacag ggttggaaga attaatttag aaattgtaca gtgcttagcc 3540

tcaataattg gtaggtggtg gtgtaaatat tttacataga tacttgtgac agttctttgt 3600
tctacaaaac agactaatat taatttcaga ttggccaagc tacttaattt ctctgtgcct 3660
cagtttcctc actattaaat ggggatgaca atagtgtcca cctagagcag gactatttta 3720
gtattacata taatccttgg atataaatgc tgatgaagtt tggatatttg tccccaccca 3780
agtctcatgt tgaaatgtaa tccccaatct tggaggtgga gcctggtggg aggtgtatgg 3840
attagatcag atctctcatg aatgacttgg gccatccttt gggtggtaag tgagctcttg 3900
ctccgagttc acacgaggtc tagatctggt catttaaaag tgtgtggcac ctccccatcc 3960
ctcactctat cacttgctcc tactttcgtc atatgacatg cctgctcccc tttcaccttc 4020
tgccatgatt gtaagcttcc tgaggcctcc ctagaagctg agcagatgcc agcaccatgc 4080
ttcctgtagt ctgtagaacc ataagccaat taaacatctt ttctttataa atttataaat 4140
tacccagtcg cagatattta tagaaagaac agcctaatac aggtgctaaa tacatatctg 4200
acaataagtg gtagttatct cttagaactg ttatgaggat taaacgagtt acagtgtcca 4260
tcttaaaaag ataattctga aaaaaatctc ataggttcac tttgccttgt caagaatatg 4320
attaccatta acttctgtag ataggaaaaa aatgtgctga gctccaaaac tgacagacat 4380
tttgctaaaa caatatcaaa tctcattaaa atgatgacag ttatttcaat agttgacagt 4440
agctatccca atacaaccta acaaggcatt ttctcttcag aatcactcga tcatatggcc 4500
atttccctaa gcttttatag ctattgccca gtgtacactg aagacaacag ctaacgaaca 4560
acatacgtgt gataatttat gtagttgaac ctctaaggc 4599

<210> 101
<211> 4023
<212> DNA
<213> Homo sapiens

<400> 101
   tttaaaattt atttctcatt aaaattataa cacagcctcg taaaaatact aaaacagcga 60
   gacagatgtg tcaggggtta tgtatatgaa ggaggtagca cagttctcat cacgggtgtg 120
   cttagtgtcc ccctaaagtt catgccagtg aatgtgatcg tgactgtaat tggaaatagg 180
   gtcgttgcag atataattaa gatgaggtcc ttctggagtc gagtgggact ggtgtcctta 240
   gaggagaaga gacacggaca cacagaatgc cacgtgggac agaggcctgc tgtctacacc 300
   cgtgcagagg agacaacaag gagagtgggg catgacccct gcccacgaca ctcgcctcca 360
   ggaaggcagc cagctgccca tctttggtgc ggtcatctct gcaggcaaca gaatggcaga 420
   gtggcagtct gatcactcta ctccatcgtg tgagagtctc caggatgaag accaggctcc 480
   ccagcaggac gtgcggccct catggagttc tggccttgtt ttcggctgtt gcctggctga 540
   tattctgcac atctgcccac cgtcctctgg gcggtccacg aatacattgt gccttcctct 600
   ttgcatatgc tggtccctct gctggcaatg cccttccttc ttggtgagct ccaatgcatc 660
   cctcaagact cagttcagat gcgcctctct ggactcatcc ccaactccca gacggggttg 720
   gctgctccct tttttcagat cctgaaacac ttacttttcc ccctttatgt gagcatttat 780
   ctgatagtag agtttttctg tactgttgga atggtgagtt ccctgaagct aggtcagctt 840
   ccttctcagc attcagcagg cggtggggga ggagggaagg agaggaactt cctaataaag 900
   atgaacacct ctttgtggtt aggtcagacc agcctgtgcc tgtgcgacct ggtgcagacc 960
   ttcacctctc agagcctcgg tttcctcatc tgagcctcag gcacgacaat ctgctagact 1020
   ctgcttcccg gtggcagctg gggaatggga tgggaaggag ccctgggatg ctcaggtcat 1080
   ccctgccacc agaatgtccc cagagtcatg accatcagca cctcagcctc gctgcttttt 1140
   ccttggggtc cgcaggggca ggacgcaaac ccgacacgct cgctgcgcag taagctgcag 1200
   gggcttctcc agcagcacct ctgaggcctg cctaccacgt ggagatgctg ctcccgtggg 1260
   ctctgaccac cgctctgccc tcttctcaca ggaaccagcg accactcggc ccggcgtaac 1320
   cacgcatgtc cactggtcac ctggtggcct tcgacaggaa cgtgtgataa atccctgccg 1380
   gagtgcggct ccgggaaaac actgtcaccg caaaccagct cctctttgtt gcagtccccc 1440
   gggggcatgg cccggacctc ccactctcgg gcaaaggccg aggcagccct cacagcagct 1500
   cagaaagccc aggaggaggc gcggatcgcc aggatcactg ccaaagagtt ctccccttcc 1560
   ttccagcacc gggaaaacgg gctggagtac cagaggccga agcgtcagac ctcctgtgac 1620
   gacatcgagg tgctgtccac cgggacaccc ctgcagcagg agagccccga gctgtaccgc 1680
   aagggcacca ctccctccga cctgaccccc gacgacagcc ccctgcagag cttccccacc 1740
   agccccgcgg ccaccccgcc gcccgcgccc gccgccagga acaaggtcgc ccacttctcg 1800
   aggcaggtgt cggtggacga ggagcggggc ggggacatcc agatgctcct ggagggccgg 1860
   gccggggact gcgcccgcag cagctggggc gaggagcagg ccgggggctc caggggtgtc 1920
   cgcagcggtg ccctgcgcgg cggcctgctc gtggatgact tccgcacccg aggttcgggc 1980
   cgcaagcagc ccgggaaccc caagccgcgg gagcggcgga cggagtcacc ccccgtgttc 2040
   acgtggactt cccaccaccg ggccagcaac cacagccccg gaggctccag gctgctggag 2100
   ctgcaggagg agaagctgag caactaccgg atggagatga aacccttgct gaggatggag 2160
   acgcatcccc agaaaagacg ctacagcaag ggcggcgcct gccggggctt gggggacgac 2220
   caccgccccg aggaccgggg cttcggggtg cagagactgc ggtccaaggc ccagaacaag 2280
   gagaacttca ggccggcctc ctccgcggag cccgccgtgc agaaactggc gagcctgcgg 2340
   ctgggcgggg ccgagccccg gttgctgcgt tgggacttga ccttctcccc gccccagaaa 2400
   tccttgcctg tcgctctaga gtccgacgag gagaatgggg atgagctcaa gtccagtacg 2460
   ggctcagcgc ctatcctggt ggtcatggtg atcttgctca acatcggagt cgccattctg 2520
   tttattaact ttttcatctg atgagatgtc gcggtagcaa aaatagagaa agggtagaaa 2580
   aaagggacat taaaattaaa agcaaaacca caagaaggga aagaccgcaa ctcggacagc 2640
   ccagcgactt ccaagtcctc tcacagaaga accacacgat tgggtatcac tcacagtttg 2700
   cctttttttc tgggtaatgt tttttggatt ttagccaaaa ttctttgctt gtataacact 2760
   ctgctgtgtg gcatggcaga tggaggccag cacgcagccc ctccagctcc acgtggagac 2820
   agaagggatc ccggcacatc agtggtaaca gcggacgttg tcctcgtggt cacacgtccc 2880
   gtcttgggtg tggatggagg gcagcccggg gcagagcctc agccccgcgg cccctgagtg 2940
   gcagggctga ctcccgtcga cacgagctta gaaagtggat tcactgcttt ctctgtctag 3000
   aacagacggg tgacaagtat gggcaggagg catggggcag ggtggcccac cccagtgggc 3060
   agtagcctgg cctttttctg tgtgagatct gtgctgcaca cctgagggag gggggaggat 3120
   cggccacctc ctccctgtga gacggatgca ggtccttccc tcttctcggc actgcccccg 3180
   gccttccatg agaagccgac tccccacacc gagttttaaa gcaaagccct tttcttctgc 3240
   tgcccactca ctgtgggtcc cattcggctg tttcccccac cagaccccag ggaagccggg 3300
   gcccactccg atccgcctgg gctcagctaa gcacggaagc caagggggct gtgccgtgga 3360
   gctgggctcg cgccggggct ctgggtgtgt gcgcttggcg tgcagggtgg acgcgtgggg 3420
   ttccgtgtcc ccagcagtga gggccctaga ggacgccttc tcccatggtt actgatctcc 3480
   acgggttttc acatctctgt actgtgcctg cctcaacttc ccctaacaaa tatgcatatt 3540
   ccttccagat gcctcagtgc tacaccacag tgggcctggt cccaggacag gaatgcggtt 3600
   caaacccagt ggcttgaaac ttcctgagaa actgtagcat atccagcccc ctaaaatgta 3660
   caatgtaact tgttcagtcc aacaaaaaca ggttccttat gtttctgcct tctccaccag 3720
   ggtcgctcca tcacccaaac aaaagaacaa ggtttgccag gatgtccgag tgccccctgg 3780
   ccctggctct cgtgtgcatg gacgtgcctg aggggtccgg gcacggccat acgcaggacc 3840
   cctgtgcccg gggaggcgct gcagggattc cccatccggt cgtcttgggg ccagcccgtc 3900
   ttatggactc tgccttgctt tgcttatgtt tagctgtttc tctgctacct ttcgagcaga 3960
   cttctttact acactgcact ggattgctat atttttaacc agaaataaac taaagattag 4020
   agc 4023

<210> 102
<211> 5205
<212> DNA
<213> Homo sapiens

<400> 102
tcagcccctt ctccccaagc aaccccagct ctctccatct ctctctcgcc ctcagcatcc 60
ccacccgacc cctttccctc caagcctcct ctctccctct tgcttccttt tctgtccctt 120
tctttcatcc ctcccttctc tcccatctcc cttctctaac tctctccaac ctctctctcc 180
ctcagctcac ttcccctcag tttccgcccc acctgctctg cccccacttc catttccttg 240
tccaagttcc ttcaactgcc tggagtccag tgggtgcctt gagaggctgg agagccggcc 300
tggcgccccc gccggcagca tgagaggtgc ctgggtgcat ctgcactcgg gggcggcgtc 360

tagcctcaga ccctgccgct gcggggctgg cgcagctccg aaaagctccc cgcgctctcc 420
tggagggcgg cgtggagacg gcagcagtga cagcgagggc ggcgtctcct tcgcaggggt 480
gctgttcctg cagttcgggg aggagactcg gcgcgtgcac atcacgcacg aggtcagcag 540
cctggacacg ctgcacgcac tcatcgcgca catgttcccg cagaagctca ccatgggcat 600
gcttaagtcg cccaataccg ccatcctcat caaagacgag gctcgcaacg tcttctacga 660
gctggaggac gtccgggaca tccaggaccg cagtattatc aagatctaca gaaaggagcc 720
cctctacgct gccttccctg gctcacatct caccaacggg gacctccgga gagagatggt 780
gtacgcatcg cgggagtcct cgcccacgcg gcgcctcaac aacctgtcac cagcgccgca 840
cctggcatcc ggctcgccgc cgcccgggct gccgtcgggg ctgccgtccg ggctgcagtc 900
cggttcgccg tcgcgttcgc gcctatcgta cgccgggggg cgcccgcctt cgtacgccgg 960
cagcccggtg caccacgcgg ccgagaggct gggaggcgcc ccggccgccc agggcgtcag 1020
ccccagcccc agcgccatcc tggagcggcg cgacgtgaag ccggacgagg acctggcgag 1080
caaggcgggc ggcatggtgc tggtgaaagg cgagggcctc tatgctgacc cctacgggct 1140
gctgcacgag ggccgtctga gcctggccgc ggccgccggc gacccgttcg cctacccggg 1200
cgccggcggc ctctacaagc gcggctcggt gcgctcgctc agcacctact cggccgccgc 1260
gctgcagtcc gatctggagg actccctgta caaggcggcg ggcggcggcg gcccgctgta 1320
cggcgacggc tacggcttcc gcctgccgcc ttcgtcaccg cagaagctgg ccgacgtggc 1380
agcacccccc ggaggtcccc cgccaccgca cagcccctac tcgggccgcc ccagccgcgg 1440
ctcgccagtg cgccagtcct tccgcaagga ctcgggctcc tcgtccgtct ttgccgagag 1500
tcctggaggg aagacccgca gcgcggggag cgcctcgacg gccggagctc cccttcgga 1560
gctcttccct gggcctgggg aacgctcgct ggttgggttc gggccgccag tgccagccaa 1620
ggacacggag accagggagc gcatggaggc catggagaag cagattgcca gcctcacagg 1680
cctggtgcag agcgccttac tgcgaggctc tgagcctgag acccccagcg agaagattga 1740
aggctccaat ggagcagcca tcccctcagc accctgtggg tcaggcggcc ggagcagcgg 1800
ggccaccccg gtgtccggcc cgcccccgcc ctcggccagc agcacccccg caggtcagcc 1860
taccgccgtt agccggctgc agatgcagct tcacctgcga ggcctgcaga acagcgccag 1920
tgacttgcgc ggccagctcc agcagttgcg caagctccag ctacagaacc aggagtcggt 1980
gcgcgcgctg ctgaagcgca cggaggcgga gctgagcatg cgcgtgtcgg aggcggcgcg 2040
gcggcaggag gacccgctgc agcggcagcg caccctggtg gaagaggaac ggctgcgcta 2100
tctcaacgac gaggagctta ttacccagca gctcaatgac ctggagaaat cggtggagaa 2160
gatccagaga gacgtgtccc acaaccaccg gctggtgccc ggccctgagc tggaggagaa 2220
ggcactggtg ctgaagcagc tcggggagac gctgacagag ctcaaggctc acttcccggg 2280
cctgcagagc aagatgcggg tggtgctgcg cgtggaggtg gaggcggtga agttcctgaa 2340
ggaggagccc cagcgcctgg atgggctcct caagcgctgc cgcggggtca cggacacgct 2400
ggcccagatc cgaaggcaag tggatgaggg tgtgtggcca ccccccaaca atctcctgag 2460
tcagtccccc aagaaggtga cggcagagac tgacttcaac aagagcgtgg acttcgaaat 2520

gccacccccc agccccccgc tgaacctgca tgagctgagc gggccagctg aaggagcctc 2580
tcttaccccc aagggggggca accccaccaa aggcctggac actcctggca agagaagcgt 2640
ggacaaagct gtgtctgttg aggctgcaga gcgagactgg gaggagaagc gggcagccct 2700
gacccagtac agtgccaagg acatcaaccg gctgctggaa gagacacagg cagagctgct 2760
caaggccatc cctgacctgg actgtgccag caaggcccat ccaggcccgg cccccactcc 2820
agatcacaag cccccccaagg cccccccacgg ccagaaggca gcccccccgaa cggagcccag 2880
tgggaggagg ggctcagatg agttgaccgt gccccgatac cgcacagaga agccctccaa 2940
gtcgccccca ccgcccccctc cccgccggag cttcccctcc tcccatggcc tgaccaccac 3000
acgtaccgga gaggtggtgg tcaccagcaa gaaggactcg gccttcatca agaaggctga 3060
gtccgaggag ctggaggtgc agaagcccca ggtgaagctg cgccgggctg tgtctgaggt 3120
ggcccgccca gcctccacac cacccatcat ggcctcggcc atcaaggacg aggatgacga 3180
ggatcgcatc atcgcagagc tagagagtgg cggaggcagt gtaccaccca tgaaggtggt 3240
gactccgggg gcctctcggc tgaaggcggc ccagggccag gcgggcagcc ccgacaaaag 3300
caaacatggc aagcagaggg ccgagtacat gcggatccag gcccagcagc aggtctaatg 3360
agcaggcgcg gaggctgtgt ggagagtgga catacctcac ctgtgggtgt ttactgccct 3420
tgtggcctgg tcagaggctg cagggtggct cctggaccca gatgttgtga gagaccctta 3480
gtgcactcgt ttgatttagt taatatttat gagcagctcc tgtatgacag gcccattctg 3540
ggtgtgaggg tgggggctgt gatatgtcca gatgctctca cttaaagaag gttctagact 3600
cagaggcaag acgttagaga aggttctaga ctcagaggca agaggttaga gatgggtgag 3660
tccatctctt tttcagatga gggggcagct cagaggagga gaggggcttc ccaaaggtgc 3720
acagcaaggt ggcatcacag ccaggatcgg gacctaggaa tcttggaacc cttccctgca 3780
ctggccctgg gacagccctg ggcacccaag gccctgcctc cctactggct cccaggagaa 3840
tttctcaaaa agcagccctc cagccccccg cagcttctcc acagctccca cctgcctcct 3900
catggccccc agacaccctg cgagccccac ctccagatct ttttcctgcc actgccaggg 3960
atgagttccc cttgtcctgt ccgggctgga ccctcctggg tgcccagcag agcagctgac 4020
actgacagag ctttcgctgg ggccaggcat gcgcattatc tcatctcaca ctcaccacac 4080
tgaggaaggt ctattacccc catgtgacgg gggcggaaac tgaggcttaa tccaaggcca 4140
cacggctagc aagtgggggg caggacacaa gtccagactg cctccctccc caccacaaag 4200
tgcttcagcc tctccagcag ccccttccct cagctctaat gatgttgcac tgtttcccac 4260
cctagtccct ccctccccag agcccagctc ctccgctgtg gtgtcagcct catgccttta 4320
tggcccagcc tccaccccat ctccggggtt gcctgggtct ccagtgacat cgagatgggc 4380
tcttttcacc cccctccccc actgctctgc agcatgtggc cttgtagacc ccccccacac 4440
ctgcataggg ctgtgcagac cctctgttcc tcagcatcct ctccccagcc tccacctgga 4500
gcctctgggc tcctttctgg gtgactccct ctatttttg agacggagtc ttgctctgtc 4560
tcccaggctg gagtccagtg gtgtgatctc agctcactgc agcctccgcc tcctgggttc 4620
aagtgattct cgtgcctcag cctcccgagc agctgagact acaggctaat taccacactg 4680

gctaattttt gtatttttag tagagacggg gtttcaccat gttggccagg ctggtctcaa 4740
actcctgagc tcaggtgatc ctcccacctc agcctcccaa agtgctggga ttacaggcgt 4800
gagccattgc accctgcctg ggcgactctc ctttgcctgc ctcttccctt cctgtgcccc 4860
actcctcagc ctcttctcct cacaccatgc cttcctctcc tcaggcacca ggtgcactgc 4920
ttagtccctg cccccaccca ggtctccatt ctgagctcca gtcacccaca cacgcttccc 4980
cgggcatccc caggctcctc gaatgcgtgt gtccagacat aatgctcccc cgaccacgct 5040
gccccagccg gtacgaggct gccccagctg ctgtctccct caggaagggt cgcccacccc 5100
cagccttccc aagcccctcc gtgcccctgt ctcacttcca gtgctccagt agcctttctt 5160
ataaacatgg cttctttctt gtaaagtaat aaatgtttac agtgg 5205

<210> 103
<211> 3723
<212> DNA
<213> Homo sapiens

<400> 103
taaactataa ctagatgagc tctatttttt agggcaatgg aagaacatgg gctttggatc 60
cacacacaga gagccaggtt caatccttgc tccatcactc actagctgtg caactctggg 120
aaattacgta ttgagcctta gtctccttat ctgggaaaat gggaataatg atattttcct 180
tactcagttg tgaagaccag ataggtaacg tgtaaagtgc atttgctggg tgttcaatga 240
gggtagttct tttccttctc tctttcctgt ctcccgtcac agagtggagc atggtgtttg 300
cacagaggct gacagctttc cttctgttca cccctttttc ttttttctct cttttttttt 360
tgagacagag tctcgctctg tcacccaggg tggagtgcaa tggtgtgatc tcagctcact 420
gcaacctcca cctcccaggt tcaagtgatt ctccctgcca tagcctcccg agtagctggg 480
attacaggtg catgccacca ggcctggcta atttttgtat ttcagtagag acggggtttc 540
accatgttgg ccaggctggt ctcgaactcc tgacctcagg tgatccacct gccttggcct 600
ccccaagtgc tgggattaca ggtgtgagcc actgcgcccg gcctctgttc acccctttct 660
tgaccaagac tcatcctcta atctctgccc ttttcaactg agggcagaat cttagatgga 720
caggattttt tttttttttt ttttgagacg gagtttcgct cttgttgccc aggctggagt 780
acaatggtgc gatcttggct cactgcaaac tccacccctg ggttcaagcg attctcctgc 840
ctcagcctcc caagtagctg agactatagg caggctccac cacgcccagc taattttgta 900
tttttagtac agacagagct tctccatgtt ggtcaggctg gtctcaaact cccaacctca 960
ggtgatctgc ccacctcgcc ctcccaaagt gctgggatta caggcctgag ccaccacacc 1020
cggccaggat ttaaatctag tctactcagc catagagttc acttcttagc cctcttgatt 1080
gctgggtcta gagagaccca ttattccaga gcaaagagta gaaattgtgc ccaggctcac 1140

atttggacag aagcagctaa taagtggatc aaaactattc tggcctttcc ctgcatgtct 1200
tcttttttc tttttccga agatcctttc caagtcatcc ctgcatttaa aactatcatt 1260
ctatcatctc taaatcttac cttctatatt taaaaagaaa acaaacagta ctttgattct 1320
tccctttaa ttctacctat ctcttctaga cccttcccct tttataaata tttaaggaga 1380
gcttactttg tgcaaataag ttttgtccaa aagagagaga aagaacgagt atggcaggga 1440
atgagacaag caggaaaagg agtgttgtaa agggcaaaga aagatcgcta aggcagagaa 1500
gaggagggag gaagaaggga aggagagagg gagaggtaag gaagtgaaga ggaagatgaa 1560
gctaggtctt gatttgcata tattgaatct tttccaggcc tcccagctct ggcaccagat 1620
tgggatggtg gcctgtcccc agtgagaagg aggtggcaag gaggctgtct cttgcacttt 1680
gcaagatgct taaggtgggc atgagtagat tgggtgaggt gtgaggagaa aagggtaggc 1740
tcaggaagaa gatgggggtg gtagggagca gacaggagga gaagggaagc ggtaggctga 1800
aaccacacac gttcacattt gcatggattt gctgaggtgt gctgcagagt gtctgctact 1860
gatgggggtg ggtgtccagg agaggaggca tctcccttga tgacttggaa agtgttggat 1920
gtgttaggac agcagtgtgg agctgctctg ctgcacagat ctatgtgatt ttatagtaca 1980
ctggagagac tcacatcctg ctagatgtgg ttctctcgtt ctacatacca caggcttaga 2040
caaccacgaa ccagctcgga acccttcttc tgtatgagca ctcagggagt tgtcccctta 2100
aattcagaat caggatccta tctgatcctt gcatggggga ccgctgaaga atttggtctc 2160
ccagaacttg gtaggaaaac attctttttt tttttttgga gatggtgtct ctgttgccag 2220
gctgaagtgc agtggtgtga tctcggctca cggcaatctc tgtctcctgg gttcaagcaa 2280
ttcccctgcc tcagcctccc gagtagctgg gattacaggc gcgtaccacc acaagcagct 2340
aatttttgt attttggtag agacagggtt tcaccgtgtt gtccgggatg gtctctatct 2400
cctgactacc tgacccgccc accttggcct gggattacag gcgtgagcca ctgcgcccgg 2460
ctggaaggaa aacattctga gtgaccgtgt atgtaaataa gtctttctgg ttattttatt 2520
ctgtgaacct gtgtgatgta atgactgcat ttaaatagta ttacaacaaa aactaattgt 2580
aataatagta aatagctaac atctttaaat gtttgccatg ggccaggcac cgtgctaggc 2640
cctcggcaag tatcacatca cctaatcctc cacttcaatc tgatgcaata gatgtagcta 2700
ttcattccta ttttacaaac atggagactg atttagatgg gttaagtaat ttttaagatc 2760
tcctgcttaa aaagaagtag agttgagatt ggaactcagg tagcctgatg tcagtacctc 2820
tgctcttggg ttaatttgta agcttctgaa ctctggctgg tatgcatttt gaatgaggtc 2880
ttttgagtct gattgtatta ctctgtgtgt caccttgagc atatgtgcta tcatgttccg 2940
cctgaaggtg ctagatggct gtcttccatt tggtttgttt cttagtataa gatggaccag 3000
aagaggagca ctgctttgcg caactccagg gtgcgtcatt cacactgcag tccatgcagg 3060
ggcactggcc atgccctgtg tgaaggaagg gaaggagcgg gagggaggga gtgggaacag 3120
gagggaaggg atagataatg gcgggtagtg ttctttgaat gatatgtgta cccatttgtg 3180
acaacatctc ttaaagttga aggtctacac atgtgtatcc atattagcat atgaacttca 3240
tcagacaagc actctccctc caacagaggg agaccctgtc tcaaaaaaca aaaaaaaata 3300

gcctttagag tcagacagac ctggaatcaa attctagttc tgtttcttaa tatgtaaatg 3360
acctcaggta agttgcgtaa ggtctcaatt tagtctcttc atatagagaa tggggcaggc 3420
cgggcacgcg cctataattc cagcactttg ggaggccgag gtgggtggat cattggaggt 3480
caggagcttg agaccagcct ggccaacacg gtgaagccct gtctctacta aaaatacaaa 3540
aaaaatgagc cgggcgtggt ggcatgcgcc tgtaatccca gctactcagg aggctgaggc 3600
agaagaatcg cttgaacccg ggaagcagag gttgtagtga gccgagattg caccactgca 3660
ctccagcctg ggtgacagag tgagaccctg tctaaaaaca aacacatcag aaacagaaaa 3720
cag 3723

<210> 104
<211> 3635
<212> DNA
<213> Homo sapiens

<400> 104
taaaaaatga aagtaaataa aaaaataaaa ttggagcccc agtgtctaga atttttttca 60
gacagggtct ctctctttgt cacccaggcc ggagtgcagt ggtgcaaaca cagctcactg 120
cagcctcaac atccttggct caagcaatcc tccaacctca atttccagag tagcttggac 180
tataggtgca tgccacaatg gccggctaat tttcgtattt tttgtagaga cgagatttca 240
ccgtgttgcc caggccggtc tcgaactcct gagctcaagc aatccaccag cctcaacttc 300
ccagagtgct gcgattacag gcatgggcca tcgtgtctgg cctagatttt tttttttaag 360
ccagcgtttc tgttgctcac ctaggcagag acccctcagg atgcaacatc taaacaccat 420
tgccatctct ccagtcccca acagcctggt ggagatgggc ctgcagctgc acccatcaca 480
gcccctgctc acagtgtgca gactgtaccc tgcacaggag atcccactga ggagatgtgg 540
ggcctgaaat ccagtcatac cctacaacca aggcacacag tacattccct catcttccca 600
ggaacaggca ccacttccta atttaccatg ttacccattc aatacaaata cagggagact 660
tgtgctctcc tctgctgtgg gacacagata ggggctcctc aggatgcagg cccaggatga 720
attcctgtag gagataatgc ttgggtcaag tttggtttag ggaatagaag caaagaagtc 780
acctctctac tttttacctc cccaatccag gctaccccac cccatgactc cctgtggtta 840
tcagggacat caccattctc ctggccactg acagggacac tttaataaca gtacacattg 900
gccaggcgcg gtggcccacc tcggcctccc gaagtgctgg gattacaggc gtgagccaca 960
gcacctggcc taatttattt ttaattttaa tttttaaatt tttctcagac tttagtgagc 1020
ataagaatgg gcagagattt ttagagtcac acagaatagt attccggttc tggctctaag 1080
agcttttcag gggggcttggg cacccatacc ctcatctgta agctgcagag acccccctcgc 1140
aggctgcatg agtattgagc ccagtgctga aatgcccagc acagggctgg cccctccctg 1200

cccagtgttc ctcctgccag cttcatgggt ctccccagcc tgccatttcc ccctcattgt 1260
gtgtgttggt ggtgggggt ggcggggtgc agatgaaggc agatcctctt ccccagaatg 1320
aggagagcag gggctcaca gcctgctgca ctggcccacc tccacagccc tgtccccact 1380
ggccgcctct ctgtacttgc ctctgccaac tctctgggtc tttggccttt ctttccaaga 1440
tgcagaacgt ttgtaaaacg aaggggttgg accagaagaa ctcaaaagtc cattttgaga 1500
aggggaagct aaagttcaga tgccttaaag ttctgtgtct tttctaggag gtccaaactc 1560
ccagcatatc tgactgggct gcccccaggg gactgggaca tctgcttttg tgtcacctgg 1620
ctccatcatt tctccctcct ctcttttctt tctcttacat gactaaagtt gtttgagctc 1680
ttaaacaatt tgccgtgcat tgtataggca ttttacataa tccatctgaa tttttttttt 1740
ttttaagata gagttttgct cttgttgccc aagctggagt gcaatggcat gatctcggct 1800
cactgcaacc tctgcctcct gggttcaagc aattatcctg ccttagcctc ctgcagctgg 1860
gattacaggt gcctgccacc atgcccggca aatttttgta tttttagtag agatggggtt 1920
tcaccatgtt ggtcaggctg gtcttgaact cctgacctca ggtgatccag ccacctcggc 1980
ctcccaaagt gctgggatta cagacatgag ctgctgtgcc cggccccgat tgaatcctta 2040
aaacaacctt gtgaagtaaa tactattctt ttttatccca ttttatagat gaggaaactg 2100
aggttcagag agggtcaggt cactcatcca ctgctaatta gaggcagagc cagcaggctg 2160
cttgctctct caatgccatt ttccagatgg cgggggtccc attctggctt gaattcatgg 2220
ggctcaatat ggcagtgcct gtgctcacct cttctttcca gtttttcca aatggtctct 2280
gactcagtcc ctggatacgc tcgcctagca ggatctcctg ggctgctggg tgtgtcccgt 2340
agaccctggc cgacctacgg atgaaatgag tagtcagaca ggtatgcagt gtaacagcag 2400
ctagatgact acctggctct agtggccaga gagcagcccc gagaagctgg ggctgcttgc 2460
ttttattccg tgcaggcaca atgccggaaa cctggagcca acacaacctg taggtaatta 2520
acatttattc ttcccctttc agggaacttc atgtgcagat aatcaaaggt cagtttctgg 2580
tcaacataag taaacaagcc tgtttaagat aaattccccc acactccctt ggggagctga 2640
gtgaaggggg gtgccatcta ctcaaacaag cctggagaag gagtaggtgt ggggggatgg 2700
atggagggga acaatggttt catttggcca tcatggcaga ccttgctggc tgctctcccc 2760
aagctccttc ttctgctttc tctttagatc ttcaagagct agaaggttcc ctaagagacc 2820
acccgaaaca agcctctcca tccacgtatt cattgaatga atacacctcc agtcctccta 2880
cttaccaggc agtgttaggt cctgaggata cagcgatgga caaggcagac aaggtctctg 2940
cccttgttta catcctgggg ggagaagaca gactataaaa gagattagga tgagtgctgt 3000
gaggatgaaa agggtgatat aacagacaat gactgtgggc aggtgcagtg tgttcctggc 3060
aaagggaact gctagtgcca aggcctgatg gcaggaacta gcaggtgtga ggaaggagcc 3120
taagtgaggc cctcttggct ggatgaggtg ggcaagggga ccccatacaa ggaggtgctc 3180
agggtccagc tgtgtagcct ggggccaggg cgagggtttg gttatagtct gcatgatgca 3240
gttttgaagg gctttaaggg gggagtgatg tgatcagatt acatgtgtaa agaattcttc 3300
agccaccagg aggagaatgg acggtaagga gagccggagt gcacaaggga gagagatgag 3360

agtggcttgg acagtgaggt ggagggtcat ggttagattc aggatctagg tcatggtgaa 3420
gataacagga gctgttggac tggatgtggg catgcagtga gggctgactc ttgggctttt 3480
cccttataac agaaacctga aaagtgtttt attttgttgt ttttgttgtt tgaaatgaaa 3540
ggtctcacat atttattact gaacccagcc aaccaacgca ttcataacag attcagagag 3600
gaaaaatgta ttcccaataa aacatgtcta actgc 3635

<210> 105
<211> 3932
<212> DNA
<213> Homo sapiens

<400> 105
aaataaaacg gacaccttaa gcgctacaat caaggtgcgc acggggtgct acagaacaat 60
acgcagacag gaggaggtgg ggggaaaggt gagacttccc aggcaggtgt catcccagaa 120
taacttttac acagggtgac taagcgagtt agtgactgcg cggaaaacgg gcttccaagg 180
ttcacaaggc tcgtgcagcg gctccgggag ttatgtcaca gtaagcttac tatcatcctt 240
tgggcatctg ctttacggat gagttcatca ggatttaaag gatcttggtt ccatatcctt 300
ccccttcctc acagaggccg ccagcccgga gcccctctag gccctcctcc ctcctgcatc 360
tactggccgc gagcctttcc ctccccgccc ccttcacaca ggccgccccc agcctcccaa 420
ccccccggtt ccgttccacg ggaggccccg gcctccctgc cctctcctcc accgttctac 480
ccgcatcgcc cggcttcccg caagccgctg gcaccgtccc ctcaacaccc tccgcccacc 540
gccactccct tccactgagg gggaccgggc tgccttctct ctgacccgcc gttctccgcc 600
cccacccact tccccaggcc tcccgcacca cccccttccc gccgcgctct ctcccgcccc 660
cgcgcttccg gccccgcccg cccccgcgcg gaggactgtg ggagcggctt ccttggattc 720
cgcgcttggc aacggctcgg cgtcgcgctt tggccaaccg ctgcgtcgtc cctgggcccg 780
aataactgtc gcccgcttcc ctcagcgtga ggtgaagcac ttcaaattgc ctgatgaaag 840
ttgaatcagg aatgaagata aaagtacaca acaatgcaga aagcggtact tagcctcaca 900
aagatggaaa cattccaaaa acgtgaagta ctgttgtccc aagattcttt ctaagcctaa 960
aattacactt ttactagtcc gattttctgg ttgtgagttt tttttgtttg tttttttttt 1020
tttcgagaaa ctgcagtagg cttcttggaa gtagaccaag ggtgtccaat ctttggcttc 1080
cctgggccac atcggaagaa gaattgtctt gggccacaca tgaaattcac aaacactaag 1140
gtacaaatga tgcaaaatgt gcacctggct ccagagacag atgaagatga tctttattcc 1200
ggctataatg actacaatcc aatctatgat atcgaggaat tggagaatga tgcagctttt 1260
cagcaagctg tgaggactag tcatggcaga agacctccaa taactgctaa aatatcaagc 1320
acggcagtta ctagacctat agctactgga tatgggtcca agacatctct ggcatcatca 1380

ataggaagac caatgacagg ggctattcag gatggagtta ctagacccat gacagcagtg 1440
agagcagctg gttttaccaa agcagctttg agaggctctg catttgaccc ccttagtcag 1500
tcaaggggcc ctgcttcccc tttggaagcc aagaaaaaag atagcccaga ggaaaaaata 1560
aagcaattag agaaggaagt aaatgagttg gtagaagaaa gctgtattgc caatagttgt 1620
ggagacttaa aattggcctt agaaaaggca aaagatgcag gaagaaaaga gagagtcctg 1680
gtgagacagc gagaacaagt tacaactcca gaaaatatca atttggattt aacttactca 1740
gttcttttca atttggccag tcagtattca gttaatgaaa tgtatgccga agcacttaac 1800
acttatcaag ttatagtcaa aaataagatg tttagcaatg caggaatatt gaaaatgaat 1860
atgggaaata tctatttaaa gcaaagaaat tattccaaag ccattaaatt ctaccgaatg 1920
gcattagacc aagttccaag tgtcaataag caaatgagga ttaaaataat gcagaatatt 1980
ggagttacat ttattcaggc tggtcagtat tcagatgcta ttaattcata tgagcacata 2040
atgagcatgg caccaaatct gaaggcaggc tacaacctaa ctatctgtta ttttgctatt 2100
ggagaccgag aaaaaatgaa gaaggcattc caaaaattga ttactgttcc attagaaatt 2160
gatgaagata aatatatttc accaagtgat gatcctcata ctaacttagt aactgaagct 2220
ataaaaaatg atcacctcag gcaaatggaa cgtgaaagga aagccatggc agaaaaatat 2280
attatgacat ctgcaaaact cattgctcct gtaattgaaa catcttttgc tgcaggttat 2340
gattggtgcg tggaagtggt gaaagcttct caatatgtag agctagccaa tgatctggaa 2400
ataaacaaag cagttacata cttgagacaa aaagactata accaagctgt agagttctta 2460
aaagtgttgg aaaaaaagga caatagagtg aaaagtgcag ctgcaaccaa tctctcagcc 2520
ctgtattata tgggaaagga ttttgcacaa gccagcagct atgcagatat agctgtgaac 2580
tctgatagat ataatctagc agctcttact aataaaggga atacagtttt tgcaaatggt 2640
gattatgaga aggccgctga attctataaa gaggctctaa gaaatgattc ttcttgtact 2700
gaagcacttt ataatattgg ccttacctat gagaaactaa atcggctaga tgaggctttg 2760
gactgtttcc tgaaacttca cgcaatccta cgaaacagtg ccgaagttct ttaccagata 2820
gcaaatatat atgaattaat ggaaaatccc agtcaagcta ttgaatggct aatgcaggtg 2880
gtcagtgtta ttccaaccga tcctcaagtt ttatctaagc taggagaatt atatgatcgt 2940
gaaggagata aatctcaagc atttcaatat tactatgagt catataggta ttttccttgt 3000
aatattgaag tcattgagtg gcttggagcc tattacattg acacccaatt ttgggaaaaa 3060
gctattcagt actttgaaag agcttctctt atacagccta cacaagtgaa atggcagctg 3120
atggtagcta gttgtttcag aagaagtggt aactaccaaa aagcattaga tacttacaaa 3180
gatactcaca gaaaatttcc ggaaaatgtc gaatgttctg gcagcgtgag gacaggacac 3240
atggagagag accctttaaa cctccttcca aggaagtcag ttctccctgg tccagagatt 3300
gcaaagcgta attatttttc aggagaaaaa tactatttgt ctgcgtttct tagttcgtct 3360
ctgcacagat cttggattaa aagatgctca agaatatgcc agaaaactga agaggttgga 3420
aaaaatgaaa gaaataaggg aacagcgcat aaagtcaggc agagatggca gtgggggctc 3480
ccgtggcaaa agagaaggaa gtgctagcgg tgatagtggc cagaactata gtgccagtag 3540

taaaggtgaa cgactaagtg ccagactcag agctttacct gggacaaatg aaccttatga 3600
aagtagcagt aacaaagaaa tagatgcctc ctatgtggac ccacttggcc ctcaaataga 3660
acgaccaaaa actgcagcca agaaaaggat cgatgaggat gattttgctg atgaagaatt 3720
aggagatgat ttgcttccag aataatattc actttaatat ttattaaagg aaagaaattg 3780
ccttatgaga tcatcctcat gttaaacctt ggattaaata tctaacctgt aattattttt 3840
tttcactgtc aaaacttaag taagtgtatt ctattctgta tgtatgcatt taagttgttt 3900
ttttctttta aggaataaaa acaggtaaaa ct 3932

<210> 106
<211> 3820
<212> DNA
<213> Homo sapiens

<400> 106
   gagtcaagcc tcaagcactg ccggggacag ctgggactgg acaccaccca gaagccagct 60
   ggctggccct gccctgccct gctggaggca ccatctctgg tgagtggggg catgtctgac 120
   tcccctgcgg ctctcccacc attacctggg gccccagctg agtcaggggt ggcctcaaat 180
   cccaggcaag agggtggcat cagtgagtgg gtctcctgaa gtctttggtg gcagtggctg 240
   gagaaggtgt taaagggctg aatggccctg caggtagggg aggctgcagt gtgtgccagg 300
   cggatgtgac tttaggaggc cagtggccac cagcagaata gggatgggaa atgaagatgg 360
   gcgggcaggg agacacagcc ctgccacagg ccaggcaatt gccccgtgca gtgcggtctt 420
   gaagctggcg tctgaaggtg aagggattgt tggaagaagg gagggtggcc aagcccgttt 480
   ccagcttgac aacagttatg tgttttccag tatcctcctt cattcattta ttcccttaca 540
   agtattaatt gaggcagggc cccaggggaa acacaaagat gggtcaggca cacatcttcc 600
   cttcccaaga gcttatacct tggctgggga cataaagcat gacagaaata accatcgata 660
   aggcagaaaa tgtcagggct atataagaga taagagaagt gtactgggga gcacggtaca 720
   gtgaggtgca agtccagcag gctaaaggaa gatagttcta atctacagct agccactgag 780
   tctctatgaa cctcagtctt taaatatata agatggagac aataacatct gccttatgga 840
   ggcttatagg gttgtaagca tcaaatatat ataaaaggtg cttgaaattg tacagcaata 900
   tacagaggta agctattatg ggtatcgtat tatccaacta gccagcatct ctctgaactt 960
   ctccgcctgg aattctggga gccacaggga gcccaggatc tgagtactgg gttcttgtca 1020
   gcagcaaagc aaagtctaga ccctgaggcc tcctttcccc ttatagaagg ctgacagcct 1080
   ctctcagctc ttggccaatc aatccaattg atccacagat atgtagtgcc cattttttgg 1140
   atgtataaag cactgtgcta gggaccatgg gcgtggagag gcatgaaatc cagagtccat 1200
   tccctacaga cctatcatga gccagggagg caagatctct gtttataaga tacacctggc 1260
   cgggcacggt ggctcatgcc tataacccca gcactttggg aggccaacat gggcggatca 1320
   gcagaggtga ggagtttgag acgagcctgg ccaacatagt gaaaccctgt ctctactaaa 1380
   aatacaaaaa aaaaaaaaac tagctgggtg tggtggtggg cgcctgtaat cccagttact 1440
   tgggaggctg aggcaggaga atcgcttgaa cccaggaggc agaggttgca gtgagccaag 1500
   attgcaccac tgtgctccag cctgggcaac agagtaagaa tccgtctcaa aaaaaaacga 1560
   aaaagataca tctaaggcag agccatgtaa gagtagcaaa tgcatgccag aggtgggatc 1620
   ccaggagagc agagggagga agaggactac agggtggggg gtaccagcaa aagcctctgg 1680
   gaggagccaa gggctttaaa gggggatgga atcaagaggt cgagaggagg agagaggcat 1740
   cctggattga gaatacggga catgtgagcg aactcacagg aaggcaagtg cgggggcgag 1800
   gcagactcag agcaggcttc tcaggctgga gtgaccattt cctgtggggg aacaggaagg 1860
   agaagaggct ggagtcaagg ctaggtacag gttgcagagg ccttgaatgc caggcaaggg 1920
   agcctggact ttcacataaa ggcaccagga gccagtgaag gtgttaggaa tgaaaggtgt 1980
   tggggtgaag tcagtgaagg tcctgccccg gatgggggtt tgcagggtga ctggagaaga 2040
   ctgccaccag gggccatcac tctgggggct ggtgtagcca ctgcagcaag gtagaaggaa 2100
   tgctcccccc gaaatgctca cagattgggg tgagacgctt cctgaaggta ggggaacaaa 2160
   ttgcagtgag ggctacacgt ttccattttg attttgatag aggcaggctg actgagtggc 2220
   ctggatccct ggacccttgg ccttgatgtc tggttctcag gatttgcgca gtctgtggat 2280
   ggaggctgac cagatcagag cagctcaggg gaccccgttc tctccttcca cagggagttg 2340
   gttagcggcc actgagccct gggcagggag ggggatgagg gaggagcaga ttttgcagcc 2400
   atgggggagg gtttagcatc aggtgacatt ccagtggact ctgcccaagg cctgcttccg 2460
   ggtacacttc cagagcgatc acccatgggc agccagttgt ggtgaatgag ggtttcactc 2520
   gcctgctccc tggccctggc ttcctccttc ttaaggggcc acgatgggac ccgagtcctt 2580
   tcaaaaaact aggcagttcc cgctgctcca agcagaaagt cctatgagcc taagagaaaa 2640
   agagatttgg ccagacactg cagccagggc aggaaccagt taatatgagc ctgggagcaa 2700
   ggagaggtga aatgctgttt accagcagcc ggctgtgggt tgcagggcac ccagccacct 2760
   agcacaggat tccaggcccc aacttgtcag gaagcaggag ccccaccctc agctcagtga 2820
   gggactattc ctccccatct ggggcctggc tgcaagacct agggctctcc actccccagg 2880
   tagagggagc aggccttcta ctctgtggag gaggaaatgg ctcctgttgg agattctgca 2940
   gggtgagact gcaatccaga actcctgctg cccaggcaaa ctgggaagtc cctctaaaca 3000
   acacaactga cctctggctc tccctgggta ggcctctcca tgcccaaagc cggggtgcca 3060
   gggcctcctg ccctcatctc tgatgcaggg cattgcagcc ccatggcagg tgacttctcc 3120
   agtaggcact ggggcttgcc tgcttctcag aaagtggggt acgggaggca gggcaggggt 3180
   ggtgctgttt gcaaggctgc tggtgtgagg gggaagatcg gccgtggcca gccacagaat 3240
   aacggatatt ccacaagcat ttactgaaaa catttaccca cacccgtctg agtgccagat 3300
   gccagggaaa acaagatgag tgagacatgt ccctggctct ctagaagggc acagtgtaat 3360
   tggagaaaaa acaatttaga aggctaatta tgatacacta ggataaatga taaataaatg 3420
   aatgtgtaac aggctgtggg taaatagggc aactcacaat gggtctttcg tgggaaggtg 3480
   aggatcgctt cacagaggaa ggaacattgg agctgagctt tggggcaggg cagatttttt 3540
   aaaacttttt ggtcacaaag aaaaaggatg gtaagaggca taaaatgttc gaggtggaag 3600
   gaacatcaca cagagcatgg catggcaaag ctattccaat catggtgaaa cagaactgaa 3660
   agaatgccag cttctgcaag agtttgggtt aaaaaccatt aggccaagca cggtggctca 3720
   tgcctgtaat cccagcactt tgggaggaca aggagggagg attgcttgag cctaggagtt 3780
   ccagaccagc ctgggcaaca tagtgagacc ccatctctac 3820

<210> 107
<211> 3745
<212> DNA
<213> Homo sapiens

<400> 107
ggacatactc ctgccagtcc ccagtctggg tcaggtgcct ttgaccaggg tgcgcagagc 60
tgttgcacac agctgtactc tgcccccgtg cacccggttc caggggtgcc acactgcacc 120
cacctggggc tcgctcctca cagtggtgcg tgggccctcg cctgtctcac ggcacctgct 180
gcctgtgccc tcagctcctc acttcatcct ggggtctctg ctcagcacag agcccggtgc 240
ccgcggggat tgaatgagcc attgcttgtt gcttcatagg cgtggggtgt gtgtgtgtgg 300
tagccaggag gtggcgatgg gctgtgttcc tcacacagga ctctggctgc cagctgccct 360
cctgcagagc catgggtttg gccctgtacc aggaaggggt tggaatctcc tggccgcacc 420
tttcgcagca tgattctgtg aggagtttag ttttcacttt gctagagctt ggcagggtgc 480
gtggtgccct cagttggcag tggattacaa gattggctgt ttcattaatt ctggttgata 540
gaaacatatg ggcaattgca gactgtccag agacttttgt aagtcttcta ggtcataaaa 600
cataggcaag cagaggcacc ttgtaccttc caaacatgct gcgtgacacg cctctcctga 660
ggtttggcat ggacgctggg gccaccgcat gggctccatc ccatgcccgt ggctgttggg 720
gttttcgctt gtgcaagtct gagacccctg cccctgccag ggtgcactgc cctcccacat 780
ctcctcccct cctccccacg tcagcctgcc ccatgcctct tttttggatg taccacaggc 840
tgaggggaga gaggccacag gttgtcagct ctgtttgaga tgcagggagc atatgctctg 900
agggggactt gagccagccc cagctggcct tcacttctca ctgttccagc ggctctgccc 960
gagatccatt ccggaagtaa gagttagtac ctgcaaagat gatgactgta gtcagtttca 1020
acaagcatac agcttatcca aagacgcctt gtcgctagga gagtggcttt ggtcttttgg 1080
ggtcacacgt ctctgacatc tctctgttgg ggtccctgca gcagtgaaca tttgctgtaa 1140
agggaattct ttgtccagtt acctcaggaa ataagatagt attgttttga aatactcctg 1200
tcgcatcttc ttttttttcc catttaaagt atggatcatg acttaggggg tacttggcga 1260

tggactttgt aaaattgttt tccgaggtgg tccagcatta ccagctctgc cctcatgggc 1320
agcatgagca cggccaagtc ctccagcctt ggctgccccg gcactgtgga agccctcgca 1380
gctcgtgacg ggcgcgctct ttcctttcag agatgttcga caagcagcag gcgaacacca 1440
tcttctggag cccccaagga cagttcgtgg tgttggcggg cctgaggagg taggtgtctg 1500
cgctctgagc ctgtccgccc tgtgagtagc gctctgctgt ggcagagacc cctcaggcgc 1560
ctgcacccgg cttcttggta gagagagtat tgtgcgctga aagggttgcc ccggtgtcag 1620
tggataggac ttcattgaac ctctgcagac ccacggtact ccaggactca ggccaagccc 1680
gcacagagtg aaataagcgc tcccagatgc tttcgtgaag tgaccaggca gacccgtctg 1740
cgggttgtgg ttctctgtgg ttaaacaagt gccttccagc catccagtaa cctccccccg 1800
ggggtcagtc ccttgtcttc tagtgggaag ggtattcgtt acacagctgt gaggctgcct 1860
tacctttgtg gttggtgtca cggtgtcggt ctcagctgtg ttccagcagt gtgtcgaaat 1920
catggagaag cttccagctt tctgctctga gttaaaagaa tacccttttg ttgctgctct 1980
cccttgcttc attgtccaca cttgctactg tatttttgga ggcttactta gcctcagata 2040
acatttccag taaaaataat atgatggcag tcgtatgtaa tactcactgc acactctgca 2100
tctgtgattt ggtaagtctc acagcggcca ccagaggctg ttgctgtgct tgtttccatg 2160
ttaacgccga ggctggctcc ctggggaccc catgccgcag cacctctcag gagtgggatg 2220
ccaggaggta tgcgtctcca tgaagcctga cggtcctgtc tgtctttgca gtatgaacgg 2280
tgccttagcg tttgtggaca cttcggactg cacggtcatg aacatcgcag agcactacat 2340
ggcttccgac gtcgaatggg atcctactgg gcgctacgtc gtcacctctg tgtcctggtg 2400
gagccataag gtggacaacg cgtactggct gtggactttc cagggacgcc tcctgcagaa 2460
gaacaacaag gaccgcttct gccagctgct gtggcggccc cggcctccca cactcctgag 2520
ccaggaacag atcaagcaaa ttaaaaagga tctgaagaaa tactctaaaa tctttgaaca 2580
gaaggatcgt ttgagtcagt ccaaagcctc aaaggtgagc ctcattccca aaatgagggc 2640
tgtgctgtga catccgccat catggcaaag gcggggctgc ggggagctgc tgtgtatgtg 2700
ctcagagttg cctctgctcc gaagacactg gttctgttcc cccccaggag ggatgcactg 2760
gggaactggg gttggcacgg ggacttggag ttggtgacct cactgctgcc ttgtgggtgg 2820
ggggcagcgt tgggggaaaa gtgagaatga atggactggc cattcctgct tgagtgtctc 2880
tgtatgctgt ccttctggca ccgtccgcct gggtgtgggc tcttcgcgat ggggaggcac 2940
ttgtcttacc agttctgtgc tttccccaac ctcatgcata ggaattggtg gagagaaggc 3000
gcaccatgat ggaagatttc cggaagtacc ggaaaatggc ccaggagctc tatatggagc 3060
agaaaaacga gcgcctggag ttgcgaggag gggtggacac tgacgagctg gacagcaacg 3120
tggacgactg ggaagaggag accattgagt tcttcgtcac tgaagaaatc attcccctcg 3180
ggaatcagga gtgacctgga gcactgtggg gacggactcc gcctgctgtt cccgcgctga 3240
gctacaggac tcccgagtgt gagccgcggt tcctctgttg cagcgcagcc gtgtgtgctg 3300
tggagccgag gccgtcctgc aggaagccgc gtgactcccg cctcctccct gtgctctctg 3360
gctctggact gtgactgcgc ctggattctg ccattgcgac acatttttgt gcctttcagc 3420

ccctggtgtc tgcagtgggg gatttaaggc acccgcttcc acttctttct tgtttggagt 3480
tttctgttgg aaccgccggc gttggctccg aagacttagc gacgccactg gcggcacctt 3540
ctcctgcgcc cagtgatgtt tccacggtgc ctgtacacag ccgagcagca tttccgttga 3600
aggacttgca tccccattgc gggcagtgct ggacgtgtcc cggagaccca ccgggagggc 3660
gccgccatgc cttgtacccc caccgtgcag gttgtggccg gttttctccg caggttgaac 3720
atggaaataa aagcaaactt gtatg 3745

<210> 108
<211> 3599
<212> DNA
<213> Homo sapiens

<400> 108
   aatccgagtg gcgactgaag gtggaggtaa gattgaagag gcggatgccc gggccgtggc 60
   tcagcaagaa gttgttgagg ggcgactcct cgtggaggag cgcccagctc tggtgcgcca 120
   ggcgcggcag cggggcggcc gacgcgcgga agtctgtgcc gtagaagagc agcgcgcgcg 180
   tccgcgagtc cctcagcgct cggcggttcc gggacgccac gcacgcgccg cgcgcacact 240
   cgatgcgctc cgagtctccc gggaagtggg ggaatagccc tgggctccac cacagcagta 300
   ccggcaagtc ccccgcctcc tccctccctg gccgcggcgt cccagagctg cgcgtcaccc 360
   ccactgcgcc cagcgccgag ggcggccgga aaaccgcgcc atcccacggt tccgcccact 420
   ccgcctcccc gccggcctcc ctctccgcta cggacccatg gccgctggct gcacagacac 480
   tgagcacccc tagaaggacc aacaccaccc taatggggcc ggccgccatg tccactccgg 540
   cagccggcgc cccctcggcc agcaccgacc ccgcccagcg gatagtcgtg accggacgag 600
   gccctacgcc acgggggcac gtggcgcatg cgcagctcgc tcagccgacg gccaggacta 660
   aaagcaaagt gagctttcgg gagtaaagat gaggctaatg gttacatctt aatttatttt 720
   atttctttac aaattaaaga tgcattgaaa aataaaccaa tgaagagaaa tgagaggtcc 780
   agagcagggg catcccaggt ggggagcagg caagcctggc caccatcaag cctagccctt 840
   gtcctgcccc caagccagtt ggggttggaa gataactgtc agccactcag tcaataccaa 900
   agcccaagcg gtgtcccagc aggagagaac ggcgctccag ctgcctctgc ctctctcccc 960
   aacaaccccg ggcctggaga agcagtgttt ctcgtgggag ctcagagtgg gatgtgaact 1020
   ggctctgtga ggtattgcaa gttgatgacc aggctcccta ggctccctac agaaccatcc 1080
   agtgttgggg cctctccagc tggaaaggaa gctggtacct ctaccagatc ttaatatatt 1140
   ttgcaactca gttctccggt cacctctgac agtgtgcctc tctatccacg gagtgatcat 1200
   caatcattgc aggagagagc gagcgagcag tgaaagcaga gaaacttgac tgctaacctc 1260
   caggcccaag aaggactgac cacttttctc tccaccacct atgggctata ccccagctct 1320
   tgtgcaccta tatgttttat ccctaatata ggaagttgtg gatgacactg agccccctgt 1380
   agtaacctta ggatccccac tctttcctgc ctgcccctct cctcttggga gactggtcca 1440
   aagggacaca taatattgcc agggtctggg tgcctccaaa cccccaatac cgaatcatat 1500
   acagtgaact gagaagctac attcattatg acaggattag caggaaaaag agggtagaat 1560
   gagggaacag gggcaccttc catcagcagg acatgacaga gtggcaggat acttgagccc 1620
   agaaagaagg tgtctcctta tatctccccc tccccccaga gagactgagg tcagcttaca 1680
   taagatatgt tactgtccca atttctctga catcctgggg tgctttctgg aagctagcct 1740
   gggccctatg ccatttaccc acttgcttta gctcagtagc tgccgaatct ccttgtgcat 1800
   atgacagaga aagtccacat aagatgctcc cccactcaga ctcttgtctt ccaccaggaa 1860
   gtgcttgaac agcatctcca tcttgtcttc ctgtttcacc acggtaagct acggccagaa 1920
   ttcagggttc ataaggcaag gccaaaggaa attaaacatg cccagaaatc tcattaggca 1980
   gtcacttact cattccccac ccaaccccca cttctaggtg aggacttgac aaggtaagaa 2040
   atccagggtt cagatcagtg ttaccttcat gtaccgggat ctctgtgccc gtaagctatc 2100
   aatgaggcct cgaaccttct tggacagtgg attatccaga actggcagaa cactctagaa 2160
   cacaaaattc atttatcctt atggtaaggc actctgagtt cagtcaaggt ggggcagcat 2220
   acatgtatgc atcatctgaa acaggcaaac ctgggaagaa tgacagcaac tgcccttgtc 2280
   cccaagtgtt ccctgatatt tgagggtact agcacactaa ctatattggc tcccaattgt 2340
   ttcacaaagt atacttccac acccatattc tccttgactc cctgccctca ccaaaccact 2400
   ggtgatctga ctgaaggagg agacgctgaa aaggctctgg acaacaccct gttggacgct 2460
   tgctcccacc cagaggaaga ggttgagccc attctccagt aaatatatat ccccattgct 2520
   tagacgctct tcagaggctc gaactgctgg tggttcggta gtactctcaa cgggagactt 2580
   tgtctagaca aaaggaaggg agcaaaaggt taccaaggac cataagacaa gtctgccccc 2640
   ccgcaccaca cacaaaattt tgaaagctaa gtcctcagac accacccctt ctggggtctc 2700
   agtaactctc tcagtgtgaa aattacatca gctaaaatac ataaaaacaa attccactag 2760
   tgtctgactg atactagcct atcctttcct cctcctaaga ggccctttgc tccgtgcaag 2820
   atctcatact ccaaccctca atcgcaccaa aggtaagagc cgagggtaga agaagacatt 2880
   ggtctcagtc acatccatgg aggtaactag ctgtcggaca taggcacggt catcagtagt 2940
   gacttcagct ccaggctgca ggacatcact cttcaacaca cagttcaggt aaactgggag 3000
   tagcttcatg cactcaggaa ggatcaactg aggggattat caaaataaag tcaatgatca 3060
   tgagaaatcc ctcccaaatg caaacatttg tcccaacaac cccacctccc actaatatac 3120
   ttgccccacc tgtcctgcag aggaggggct agcacagttc tttctgtaac aggccaggat 3180
   ctgggcacac tgggtgatga gcgtgtcacg aacagccttc acagggctat tcaggactcc 3240
   ccgatatgct ggatgggaaa gcaaagatag tcactggtca gctgcccatc atgtgtgcat 3300
   tcccaccatc acaactaccc actcaccatc ctggacctct cagccccaat cccaccctgc 3360
   cctgcttcta ccctcaccaa acttggccat gtagttgatg agcgtgtcag tctcacagtt 3420
   tcgatataga tcagccagct gggtgcagca gttcagggcc agattatgga tgcggagccg 3480
   acgctgccct gcacagctgg tgtaaagcag ggcacactag gggaaaggag aaagggggaa 3540
   ctcacacccc tccctcacaa aggccctttg ctacactatt aaactggaag agagaaatg 3599

<210> 109
<211> 3693
<212> DNA
<213> Homo sapiens

<400> 109
cacatatagc tgtcaaagtg ctcgtgtcca gaatatataa gtaactttta caaattacta 60
agttaatcca atagaaaaat gggcaaaaga cacagaagca cttcaaaaat gacatacaaa 120
agtccaataa acaaaggaaa agttgcttca ttagtgatca tcagggaaat aaataatatt 180
aaaacgacaa taatagctct atacattggc aaaaaggatg tggcaaagtg ggaggtttca 240
tgcattgctg tgggaggagt ataaatgggt acaaccactt tggagaatag ttttgcattt 300
tctcctaaag ttgaagataa cattccctac caaagtcacc actctgtaca cctccagaag 360
cttcattcat gttgttatct atagaaatgc tactgcctca agttgtacag tagtacatgg 420
cctgtgtggc tatacacatt tcccctttgt ttcccctatg acccagttgt atatatctca 480
gttctggata tatactctag aaaacctgta tacaagtgta tgacgatgca cgttgatagc 540
agtactattc acagtagcca agaactgaaa atgacccaaa cgccaacttc tagtagaaca 600
aatgaattgt ggaatattca tagatggaat actatagagc aatgagaaca aatgacctga 660
acctaacaaa aacaacatgg atgcacagta caaacgtaat actgaacgaa atacataatt 720
ttaatataca caatatgatt ccagctttat aaaaacagtc aaaactacat tggtttaggc 780
atatacagac acacaaaagt ggcagaggta tttggaaaaa aaaaccaagg aagtgataaa 840
agtcaggaaa ataggaggga ggggagaagg tggggaagga ttaccagggg caaagaaaac 900
ttttggcatg atgagtatgt cttgttagat tgtggtgatg atttcacagg tgtatagtat 960
atatcagaac ttatcaaatt gtgtgtatac tttgaatatg tacagtttat tatatgtcaa 1020
ggatacatca ataaaacttt taaaatcaac attttacatt ttaaatattg ttaataactg 1080
cactattctt atttatattt ttaataataa aaattggata aaagtggatc cgatagggaa 1140
agcctaaagc tatttctatt agtggcacta tccttctcag tggcccttga ggcaccttca 1200
ataaagccct agccctctga agaagagttt gaaaatttct gttttaaact aagtcttaac 1260
cacaggctct taagccagct gacaaaattt atattaattt ttaaattaca tttctttcca 1320
aatagtctca cagcaatttc ctgtaataat ttttttggag atacatgcaa actttgatac 1380
tgccaataat ttttaatttt caatttttgt gggtacatag gtatatatat ttatggagta 1440
catgagatat ttttatacag gtctgcaatg tgtaacaatc acatcatgga aaatgggggt 1500
agccatctcc tcaagcattt atcctttgtt ttacaaacaa tcgaattata ctttattata 1560

ttaaaatgta caactgaatt attttgacta tagtcaccct gttgggctat caaacagtag 1620
gtcttattca ttctattttt ttaataccca ttaaccatcc ccacctcccc tccaccctca 1680
cccccactat cctttccagc ctctggtaac catccatgaa tttaatcatt ttgattttgg 1740
atcctactaa taaatgagaa catgtgatgt ttgtctttct gtgcctgact tgtttcactt 1800
aacataatga tcttcgagtt ccatccatgt tgcaaatgac aagatctcat tctctttaag 1860
gctgaatagt attccattgt gtatgagtac cacattttct ttatctactc atctgttgat 1920
ggatacttaa gttgtttcca aatattggct attgtggaca aacatgggag tgcaggttat 1980
ctctttgata tactgatttc ctttcttttg ggtagatatg tagcagtggg attgctggat 2040
cttacagcag ctctattttc agtttcttga ggaacctcta aactgttctc catagtggtt 2100
gtactaattt aaatttccac cagcagtgta caaggttctc cttttctcca catcctcacc 2160
agcatttaat attgcctgtc ttttggataa aatccattct aactagggta agatgatatc 2220
tcattgtagt tttgatctgc atttctctga tgatcaatga tgttgagcac attttcatat 2280
gcctgtttgc catttatcac ttcttttttg acaaatgtct attcaaatcc tttgcccatt 2340
aattggatta gattttcttc ctatagaggt gtttggacat acagcttata tattctggtt 2400
attaatccct ttgagaggtg acagcatgct ggcagtcctc acagccctcg ctcgctctca 2460
gcgcctcctc tgcctgggct cccactttgg cagcacttga ggagcccttc ggcccaccac 2520
tgcactgtgg gagccccttt cagggctggc caaggccgga gccggctccc tcagcttgca 2580
gagaggtgtg gagggagagg cgcgagcagg aactgggccg gtgcttgcgg gccggctgga 2640
gttctgggtg ggtgtgggct tggcgggccc cacactcgga gcagccggcc ggccctgcca 2700
gccccaggta atgacgggct tagcacctgg gccagtggct gcagagggta tactgggtcc 2760
ccctgcagtg ccagcccacc ggcgctgcac tcaatttctc accaggcctt agctgccttc 2820
ccacagggca gggctcggga cctgcagccc accatgcctg agcctcccac cccctccatg 2880
ggctcctgtg tggcccgagc ctccccgacg agcgccgccc cctgctccac agcacccagt 2940
cccatcgacc acccaagggc tgaggagtgc aggcacacgg cgtgggactg gcaggcagct 3000
ccacctgcag ccctggtgtg ggacccactg ggtgaagcca gctgtgctcc tgagtctggt 3060
ggggccttag agaagcttta tgtctagctc aaggattgta aatacaccaa tcgcactctg 3120
tatctagctc aaggtttgta aacacaccaa tcagcaccct gtgtctagct caggattgt 3180
aaatacatca atcagcacac tgtgtctagc tcagggtttg tgaatgcacc aattgacact 3240
gtatctagct actctggtgg ggccttggag aacctttatg tctagctcag ggattgtaaa 3300
tacaccaatt ggcactctgt atctagctca aggtttgtaa acacaccaat cagcagcctg 3360
tgtctagctc agggtttgtg aatgcaccaa ttgacactct gtatctagtt actctggtgg 3420
ggccttggag aacctttgtg tcgacactct gtatctagct aatctggagg ggatgtggag 3480
aaactttgtg tctagctcag ggattgtaaa cgcaccaatc agcgccctgt caaaacagac 3540
cactcagctc taccaatcag caggatgtgg gtggggccag ataagagaat aaaagcaggc 3600
tgcctgagcc agcagtggca accggctcgg gtccccttcc acactgtgga agctttgttc 3660
ttttgctctt tgcaataaat cttgctacta ctc 3693

<210> 110
<211> 3696
<212> DNA
<213> Homo sapiens

<400> 110
cttatttgaa gtcattgccg gatgtgagga aaaaatcgct tcccttgcct gaaaagccac 60
acaaagaaga aaattcagaa atcgtggttt ggagagaatt tgacaagcaa gtgttcctct 120
tgaactgaag cccccggaga cagtcgaagc tgtacacggt ggacctggag tcagggctac 180
actacctcct gcgggtggag ctggcagccc acaagtccct ggccggagca gagctgaaga 240
cgctcaagga ctttgtgact gtcttggcca agctgttccc tggacggccg ccagtcaaga 300
agctgttgga gatgctgcag gagtggctgg ccagccttcc cctggacagg atcccctaca 360
acgccgtgct tgacctggtc aacaacaaga tgcggatttc tggaatattc cttactaatc 420
acataaagtg ggttggatgt caaggaagcc gatctgagtt gaggggttac ccgtgttctc 480
tctggaaact gttccacact ttgactgttg aagcctcgac ccacccagat gcactggttg 540
gcacaggctt tgaagacgac ccccaggctg tgctgcagac aatgaggagg tacgttcaca 600
ccttctttgg gtgtaaggaa tgtggtgagc actttgagga aatggctaaa gaatccatgg 660
actcggtgaa aaccccagac caagccatcc tctggctgtg gaagaagcat aatatggtga 720
acggccgcct ggcaggccat ctgagtgagg atccccggtt tccaaagctt cagtggccca 780
ctccggacct ctgcccagcc tgccatgagg aaattaaggg cctggccagc tgggatgaag 840
gccacgtgct cacattcttg aagcagcact atggccgcga caacctctta gacacgtatt 900
ccgcagacca ggggggttcc agtgaaggag gaaccctggc caggggtgag gaagaggaga 960
aaagactcac tcccccagag gtgtcccatg gagaccgaga cacccagagc gtccgtccac 1020
ctggtgcact gggccccagg cctgcccttc cagagagctt gcatcacagc ttggacggga 1080
aactccagag tctggatggg cccggggccc acaaggaggt gggcggggcc gcacccttcc 1140
tcggggttga cttctccagc ctggacatga gtctctgtgt cgtgctgtac gtggcttcat 1200
ccctgttcct catggtgatg tacttcttct tccaggtgag gtccaggcgg tggaaggtca 1260
agcaccacca cccggccgtg tgagtgcccg ggtgctgcca gccacggcgg aagctccctt 1320
ggaggcagcc ctgccccgtg ccacctgcag ctttaatatt tatgatcagg gattttataa 1380
acatgcgggc ctggtttcac atcggatggc accttttggc ttcaaagtcc tggttttaca 1440
aacgctcttc taacaaggga agaacacggg gtaattttgt ggggatattt gcattcctgg 1500
cgtactcaag tctgttcatg ctccttttgc aggtcttaca gcaaaaagac ttctgtattt 1560
ttactcttct agatgtgaaa agagggtgca gagtccaggc cagatagtct tccccacaca 1620
ctttcatctc gtttcctcca ctccgcccca tcctgcaggg ccttgttttt gtatttggag 1680

aacctcgccc atcccccccg cggctcctgg ctcccccccc cccaccttgg ccctccctgc 1740
accctccact cccctcgctc cccctccccc cgctcccccc acgcccccctg ctccaggctg 1800
ccaagtgttt tcctttagcc gggcggggac agacagagcc ggaagcgcag tcggcctctg 1860
cagcccctcc aagcaagtgc tccagggact atcctgtgtt tgtagctgct tccctagggc 1920
aggttcctga gggctctcct tgttcctccg ggtgttcgac accagacgtg ggatttcaa 1980
caggggagga gccaaggaat tctgtggctg tgctgcgttt cagaaaataa cccccagagg 2040
ccttgggctg tggacctggg ggttggaagg atgggggctc atttaaccct cagaggcagc 2100
gcctttgtct gtctatctgg tgacaagaga gagacaagta aatgggggcc gttgggacgg 2160
cgggtgcctg gagggcagct ctgggctcag cgggcagtgc ttagagcaca ggcccctctg 2220
ttgggggatg gggaggagag cagtctgccc ttgggagcgt aggccccagg gagacttcta 2280
aagccccccc tgtcgtctgc tcttcaccca gcaccacaga ggcacctgct gcacacacaa 2340
gcatctcact cggcccacgg agggggccag gcttcctttg cctgaagctg ttttgggaag 2400
ggtctccaca caggcactga tctcccaagc tttggtcatg atgtctttta ccatttgata 2460
attttaaaca ttgtttttaa acccaaaaca tttagtggtc cgttgcctct gaagatgtaa 2520
acaaacaaat acactatttc tgggaacatt tatattgaga ttctttgtgg ctattggtgt 2580
gtctcacagg caaaatttga tttggctaaa ataggctcag atgtatttgt gtgcccgtgt 2640
gtgtgtgtgt gtgtgtgtgt gtgtgtgtgt atgagagaga gagagacttt gacggttgta 2700
gatatttttt ccgctttgcc tactctatgt tgtataatca tgtgtttact aacaagttga 2760
tgacatggat gtattcataa gaccatgtaa tattgatgtg attgttgtcg cttgagaaaa 2820
aaaggcaaca gctgattctt tcaacaactg tcacagaatg gctgggctga gaacgctgcc 2880
cagggccctg cagctggcag gagaggtgtc tggtgggagc ggtgtctggt gcgtcagcct 2940
gctgcttcgt gtctcactcg agagttgctt ctggtttcac actttttaac ccctctgtgc 3000
tttagcagcc gtgaccttgc cttcattgct tcatccagtg caggcctggg ttattgaaga 3060
caccaaagtg tttctcttcc agtttgaaaa ccaggcaggt ttacacgtgg gtttcagtgt 3120
atttgccttt gaacccttca aactaaactt tagccttttg gctggtgtta aatgtcttta 3180
gctggggtga cctggagtga cctgggacgg tgttgtggtt taccgtctcc agcttcagcc 3240
tttccagaaa cccttgtgga gggcagtgtt ggctgcaggt ttcatcatat tgcagtttga 3300
ggtcaccact atttggggaa caagcttgcg tcctgctgag ggcaggattt ttcagcagag 3360
cgtcggtggg gctgggccgt cgtggaggtg gccccaggag tcatatggcc atactcagac 3420
acaccttgtg tggcctgctc agagctggat gccaccttta ggcaatgttt agagtctatt 3480
ttctaaagtt ttaagtattt taagaggtat tgagactaat gaatataata gttcagtaat 3540
ttaaatgctt atttattttc agtggaagga tttttattaa aaagaagcta attgacatgg 3600
aaatgtcagt gaaatttctt acctgcaagg aaagtgaaca ttttgtattt aagtaaacta 3660
taatgtgcac attttaataa agaaatctga catttg 3696

<210> 111
<211> 3520
<212> DNA
<213> Homo sapiens

<400> 111
aactaatgat taatgatatt catatataat catatctaag atctatatct ggtataacta 60
ttcttgtttt atattttatt atactggaac agcccgtgtc ctctgtctct tgcttcggcg 120
cctggatggc ttgccgccca cacaagagca ccttaaaacc agaactgctt aacctatttc 180
tgagactcca aaaagctgtt cgctctcatg ctgccccttt tcatatttct catatccgat 240
ctcacacaca acttcctggg gccactatct ctaggtaatg atagagcaga taaattcatc 300
agttctatat ttcaacaagc ccaagcttcc catgctttac tgcatcagaa caacttgccc 360
ttactcgtat gtttcatctg cctcgcagcc aggcttgagt tattgtgcaa gcctgcccca 420
cttgccagca tgtccctggt gttgcacctg tggaaggatg caacccacga ggcttggctc 480
caaatgaaat ctggcagatg gatgttacat atatatagca gcctttggct aactccgcta 540
tgttcatgta atatagacac ttactcccat atgttacatg ccacatgcca aactggggaa 600
acagctggtc atgtctgacg atactgtctg tcgtcatttg cccatatggg ggtccctaaa 660
caatttaaaa ctgacagtgg actcgcttat gttagtcatg ctttcccaat tttttacaat 720
tgtgggcaat caatcataaa acaggaattc catacaatcc ctgagggcca ggcattattg 780
agcgggtaca tcaaacacta caacgcatgc tgaaaaaaca aaaagggaga ataggggacc 840
aattaccact tcaaacaaaa ttacatttag ccttgtttac tttaaattgt ttgactcctg 900
gtacggatgg caagacccca gcagaacgac actggcaact gctagaggaa aagaggcaag 960
tttatccaaa agtgttatgg aaatccccag aagaaggaca atggaaaggt ccggtggatt 1020
tgctgacgtg ggacgagggg tatgcttgtg tttttacagg agatggacaa accgtgtggg 1080
tgccctccag gtgtgtgtga ccatggaacg ggagaccgga gggatccatg gtattcaacc 1140
gtgggcctgt tacctccagt acgagccatg agccagcgga atctgaatgc aaagacagaa 1200
caagggccga ccggagtcac aatgacatcc aaccccataa catggggaca gatcaagaaa 1260
acgacacaag aagctgagaa actactggag cgccagggtc aggcaaaaac ccctgactcc 1320
atgttcttgg ccatgctagc tgtagtgtcc tgtgcggtat gtttcccctg tgtagaggca 1380
aaaacatatt gggcatatgt tcctaaccca ctggtagtac gactggtact ctggagcgac 1440
actcctcctg agatatatta tgatcaggga gcgtgggcac caggacccct aactccgtct 1500
gacacagagc aatagcatca attacaccgg cccattggaa ggactccctt tatgtatcac 1560
catggatacg tcactcaact gcagctgtct tgcagtccaa tctcaggcat ggttaagtca 1620
ccatggaaaa attatgtatt tattaggcct cagctttatt aatgttactg gcgtgttcac 1680
caatcactcc tagccccatc actcaaattg tatggattat atggaatggg ctccctttga 1740
tatttctcac ccccctcctt ggacccagtg tcttggcccc ttggccagac aacagtctat 1800

gttaatggga aacattgact ggggtccccg tggccattta gaggggagag atgaaaatca 1860
gacctcttgg cacaaacttc actggcactg gtggtggacc ttcaacacct cttcatcaca 1920
tcacactggg attcagtccc ccagttggct gcacaacttg cttggtatag gacaggcttt 1980
aacccgccct tacctcagtg acgtcatcta ggaaacagag gcccaattca ggaatcgata 2040
tggaaggcaa cactcccatt tatgaataac agcatctggg tcagaacatt atccaacaat 2100
agtaatatta ctcaacacag ttttaatatc acctttgtaa aaaatattac cactcaattt 2160
acggtttgtg ggttttttgt tgttgttgtt aactgtttga gatggagtct cactctgtcg 2220
cccaggctgg agtgcagtgg cgcagtctcg ggtcgctgca acctttgcct cccgggttca 2280
agcaattgtc tgcctcagcc tcccgagtgg ctgggattac aggcgcccgc caccgtgcca 2340
ggctattttt tgttttttgt atttttaata gaaacgggat ttcaccttct tggccaggct 2400
gctcttgaat tcctgacctc gtgatccacc cgccttggct tcccaaagtg ctgggattac 2460
aggcgtgagc caccatgccc ggccagtttg tgtttttaat ccctatgttt ttctagcatc 2520
taagaaggac caactccacg taaacatcac ccagttgact tgtgactctt gtcaactgta 2580
tcactgcctc aatcatagca caatacaaac acacaacata tccactctaa taattctggg 2640
tcgtatccct ggattatgga tccctgtcaa tttgtgcaaa ccttgggctg ccacccctgc 2700
tttacatttt gtaaaactcc ttcttaccca gctcactcat cgtgcacgta aagccttaaa 2760
catagtaatt tttactgtaa tctccttagt cacactaata acctcggttg tagtgtcctc 2820
agtagcacta catagttcta ttcaaacagc ccaatatgta gaaaattgga catgtacagc 2880
caatcaggca tggatgcttc aaaataaaat taataccgaa ttacaaacaa aggtagcaat 2940
gttaaaggct actgttctgt ggctgggaaa gcaggtacaa agtttgcaat tgtagcagca 3000
attgcactgt catttcaatc atattcatat ttgtgtgacc aatttggaat agaaccaaag 3060
tgaatatcca tggaaccttg taaaagctca tttgcaggga gcttttacat ccaatattac 3120
ttttgatatc agtaacttac agagtaaaat tcttaacttg aataagcaaa ctcaaaaata 3180
acagcccttt ttagaagctt aaacagaatt ccagcagggc ttagaaagcc tcaaccctgg 3240
ccgggtgcgg tggctcacgc ctataatccc agcactttgg gaggccgagg cgggcagatc 3300
acgaggtcag aagatcgaga ccatcctggc taaaacagtg aaaccccatc tctactaaaa 3360
atacaaaaaa attagctggg tgtggtggcg ggcgcctgtg gtcccagcta atcgggaggc 3420
tgaggtggga gaatgacgtg aacccgggag gcagagcttg cggtgagccg agatggcgcc 3480
actgcactcc agcctgggtg acagagcgag actccatctc 3520

<210> 112
<211> 3792
<212> DNA
<213> Homo sapiens

<400> 112
   caataaagta agctaaagaa aagaaaatgt tattaagaaa atcataagga agagaaaata 60
   tatgtactat tcattaaggg gaagtatatc atcataaagg tcttcatcct cgtcgttttc 120
   acgatgagtg ggcctaggag gaggagggac aggaggggct ggtcttactc cctcaggggt 180
   ggcagaggtg gaagaaaatc cagctatcag tggacccaca cagttcacac ctgtgttgct 240
   taagtatagg tgatactaca ttcattttct cttgctgtgt aacagattat ctcaaaaccc 300
   agtggcttaa aacaataaac attacctctc agtttggtcg tgggtcagaa atcctgcaac 360
   agcttggcag ggcggttgtg gctcagctat ctcctgaggt tgcagtcagg atgccagcag 420
   aggctgcagg agccccttcc aggatggttc cgtcacatgg ctgtgggctg gaggcctcag 480
   ctcctcacca cagaggctct ccagagggct ggttgtgtgt cttcacagca tgacagctgg 540
   ctcccttccc cagggggtgt gacccaagat agagggaaaa cccgcagcgt tatggtgtct 600
   tttgtgactg agttatggga gactcccact gtcaattcca ccatattaag tgactaagtc 660
   tagaccatat ttaaggctag gggagtaaag ctccaactct tagagggagg agtatacagt 720
   aatttgcaga catattatag aaccaccata tgtacatttt tcatagagtt tgggaatgaa 780
   atagtaaaag gtatatagaa aactaagaaa agggaaaatt ccggggggct gggatgagga 840
   agtgattagc accagggaaa accaaagttt ataccagaaa ggaactctaa tcttaggctg 900
   ccatattaag ccatgtggct gggctacatt gtgttaagtc actgatgaat gatctaaaca 960
   agagtctgga tataaccaaa tcaggaaggc ttgaagaatg tgtgtgttat tggggggaac 1020
   ggtgtccaca gtaggaggta gatggttatg taaaactaga gaaaaggaac taatataaag 1080
   tgttagttgg aatatattaa taattgtcag tggaaacagc ttaaagtgct aaatgtgctt 1140
   caggggagtg aaaagcaggg atgggtgagg aacctgtggt tttgtcctgt gcctcattaa 1200
   actatattta tgcttagcca taggaaaaat aaacattcac atcttaaaag tagtaagtga 1260
   aagaatcggt tttcttaggg ggcaggtctt tttaaagttt atatttcacc taataggccg 1320
   tcagattctc ctttttccat gtacaatatt ttgagttctg aagaatgtcc agggtcgtgc 1380
   agctgtcaca gcccggacac agagcaattc cttcccccag aaaaccagat gtgttttgag 1440
   aagaaactgt tgggatttac taatttcatt ctgatttgct ttttatttcc ctttttaagg 1500
   gaggtggatc tgcccaccat agacctttgg cagtatctga agatctttag tagtcgcggc 1560
   tagggggctg ctgctggcat agggtgggta gaagttgggg acactgccaa acattctcct 1620
   ctgtacagga cagcccccac ttcccagaac tccctggtcc agagtggcag ttacattgag 1680
   cttgagaagc cctggagtgc attccattgt tcttctcttg tttcctggga attcagatta 1740
   aatttagagt attggagttc attttcattg tatggtgctg ggtagaaaag tccgtaaata 1800
   gaactgttac atgtccttga tactttgagg aaatacatga tataaatata tgatagagtc 1860
   ttttaatagc tgccttattt gaaattggaa ttctcctcta ttctgagtga ttgatgactg 1920
   cattggtcgg gtctttgtgt gtagataatc caggcacatg gcggcactgt tgaccccacc 1980
   ttcacgagtc gatgcacgca ccttctctgt gagagtcaag tcagcagcgc gtatgcacag 2040
   gcaataagag aaagaaagag atgtgttact gcacactggt taaacacagt cttaaagaag 2100
   aagaaaatgg taccgccgca ccgagccctt cacttcccag tggccttccc accaggagga 2160
   aagccatgtt cacagcatat tatttctgtg actggatttg ttgatagtga cagagatgac 2220
   ctaaaattaa tggcttattt ggcaggtgcc aaatatacgg gttatctatg ccgcagcaac 2280
   acagtcctca tctgtaaaga accaactggt ttaaagtatg aaaaagccaa agagtggagg 2340
   ataccctgtg tcaacgccca gtggcttggc gacattcttc tgggaaactt tgaggcactg 2400
   aggcagattc agtatagtcg ctacacggca ttcagtctgc aggatccatt tgcccctacc 2460
   cagcatttag ttttaaatct tttagatgct tggagagttc ccttaaaagt gtctgcagag 2520
   ttgttgatga gtataagact acctcccaaa ctgaaacaga atgaagtagc taatgtccag 2580
   ccttcttcca aaagagccag aattgaagac gtaccacctc ccactaaaaa gctaactcca 2640
   gaattgaccc cttttgtgct tttcactgga ttcgagcctg tccaggttca acagtatatt 2700
   aagaagctct acattcttgg tggagaggtt gcggagtctg cacagaagtg cacacacctc 2760
   attgccagca aagtgactcg caccgtgaag ttcctgacgg cgatttctgt cgtgaagcac 2820
   atagtgacgc cagagtggct ggaagaatgc ttcaggtgtc agaagttcat tgatgagcag 2880
   aactacattc tccgagatgc tgaggcagaa gtacttttct ctttcagctt ggaagaatcc 2940
   ttaaaacggg cacacgtttc tccactcttt aaggtacact ttaagggaaa agtaaatcag 3000
   tccaagtaag cataacacac ggacctgctc aagggaacta ccaggcaaaa tatttttaca 3060
   tcacacctgg aatctgccca agtctttcca ctatgaaggc aatcgtagag tgtgcaggag 3120
   gaaaggtgtt atccaagcag ccatctttcc ggaagctcgt ggagcacaag cagaactcga 3180
   gtttgtcgga aataatttta atatcctgtg aaaatgacct tcatttatgc cgagaatatt 3240
   ttgccagagg catagatgtt cacaatgcag agttcgttct gactggagtg ctcactcaaa 3300
   cgctggacta tgaatcatat aagtttaact gatggcgtct aggctgccgt gcatgtcgac 3360
   tcctgcggtg cggggctggc tgtctggctg gcgaggagct gctgcgcttc cttcacatgc 3420
   tcttgttttc cagctgcttt cctgggggat cagactgtga agcaggaaga cagatataat 3480
   aaatatactg catcttttta agatgtgcaa ttttattctg aggaaacata aattatgttt 3540
   tgtattatat gactttaaga gcccacatta ggttttatga ttcatttgcc aggtttttaa 3600
   atgttttcac aaaactgtta cgggacttca actagaaata aaatggtgta aataaagacc 3660
   ttgctatctc taaattatgg atgttaaaga tttgaaatgt tttgtacttt gattattttt 3720
   atttcttata ctctgttttc ttttatattg atatcttgcc cacattttaa ataaatgtac 3780
   ttttgaactt ag 3792

<210> 113
<211> 3972
<212> DNA
<213> Homo sapiens

<400> 113
cttattcgac gaatatttta atgatgattc aatcgaaacc aggactattg atgatgttcc 60
atttaaaaca agtacagata gtaaagacca ttgctgtatg ttagagtttt caaaaatatt 120
gtgtacaaaa aataacaagc agaacaatga attttgtaaa tgtatagaaa cagttcccca 180
agattcatgt aaaacctgct ttcctcagca ggaccaaaga gatacactct ccattcttgt 240
cccccatggg gataaagaga gttcagataa aaaaattgct gtaggaactg aatgggacat 300
tccaagaaat gaaagttcag acagtgccct tggggatagt gaaagtgaag atacaggtca 360
tgatatgact agacaagtta gcagttatta tggaggagag caagaagatt gggcagaaga 420
ggatgagata ccttttcctg ggtcaaagtt aatcgaagtg agtgctgttc agcccaacat 480
tgccaacttc gggaggtcct tgctgggtgg ctactgctca tcttatgtgc ctgactttgt 540
tcttcaagga attgggagtg atgagaggtt ccgtcagtgt ctgatgtcag atttatctca 600
tgctgtgcag catccagttt tggatgaacc aatagcagaa gctgtctgta ttatagctga 660
catggataaa tggactgttc aagtggccag tagccagaga cgagtgacag ataataaatt 720
gggaaaggaa gtattggttt ccagtcttgt ttccaatctg cttcattcca cacttcagct 780
ttataagcat aacttgtctc caaatttttg tgtaatgcat cttgaagacc ggttgcagga 840
gctatacttc aaaagtaaaa tgctgtctga atacctgagg gggcagatgc gtgttcatgt 900
caaggagctg ggagtggttc tggggattga atccagtgat cttccacttc tggctgctgt 960
agcaagcact cactctccat atgttgcaca aatactcctt taatatacct aaaaattgtt 1020
agaaattggt gggaaaatag gtagaaacca aggaagcaga cacaacatgc atttatggag 1080
attctttttc ccttttagac ttccatctga atgagtcagt caccagggta ttctgcatag 1140
cattgtatat tctgtgtatg tcagatggct ttttcttttt gactggactt ttgggtggtg 1200
gtagattttt aaacaaatga aattaaagca acaataattt tgaagcattt gaaaaagcca 1260
aagtgtacgg tagaaatttc tacaaaatga atattatcaa gagtttcatg tgatcactgc 1320
agtgttgtca cagctcataa atagcaacag tgtttcatga tttaatggct cagaaatagt 1380
tattcattag tttttaattt ttaatttcta aggtacagag atctataaaa ccttgattat 1440
ttgttagttt tgcaattcaa aacagctaat gtctggttat ttctcaaagt aagtatttta 1500
aacagcctgt taattataag aaactcagaa taatgagtgt aaatgtgtta tgttatccac 1560
ccaagtgtac atatgtacct attttttttt aaaaagcaga aatagaaata caagactggt 1620
aaacatgcct ttaaaaatat atatattttc aactagtatt gtctataatg ctgaaatatt 1680
acttattggt gatttttctg tttcacacac tctaaaatat aagtaaagcc aacctttttt 1740
ttaaggctga gattcccaaa atgagaatac tactttatac catttgttta taagtatgaa 1800
ctgttcttat aaatattaat atttacatat tcactaattt aacataaatg aaaattagga 1860
ttaaaaattg caccaaagca tcggcaaaaa caatactata ttctttaaaa gtgctcaggt 1920
agccaaggcc cttgcttttg gtatcaaccc tcatgaaccc ataggagctg aatatttgtt 1980
tcactgctta ataatcctca atttacacta ttcataactc ttaaaattat tctctttttt 2040
ttctaagagc ccctcccttc caaaagtgta tttttttcaa agattttcac ttctcaattg 2100

ttgcctttgt acatactata gagtgttgct tgtaagaaag gctaatatgg aaccaaactc 2160
ttgtaagtaa tgtaaataga aaggtgggtg gataaagttt tcaatacttt ctactacctc 2220
agtttacttg agtactacat tatagtttat tctttgctta tctggtctaa gagactttta 2280
atgctagtag taaagttggt ttctgctttc attgactatt ttcatcataa tttcatcatt 2340
gattaaaaaa agaaaaccac ttgtttattc agttattaaa tatatttact atataacaca 2400
tccattcttg ctgtttaaat tttcaatagt taatggaaag ttgtctttga ccttgaattt 2460
acagcattgg gtcacatttt gccttgctgt gtatgtattc aagagacttc caactagaca 2520
aagaaaaaat tgttgtttta atggaatgta aacctgaaat tggtgtgtct gcaatctgtt 2580
tggcccatga ccttttacct agtcccagtt attacctgag tctcccatgg atgacttgct 2640
gccaaggagt gtttgtggat atattttctt tggcttaatt ttcttattct gtgcattaac 2700
aaaattatcc agttgtctga ttttggaatt ctatgagtca atctttttgg cagaattcag 2760
aatattaaaa agttcataca tttgcgggcc attgtacctt tttttttttt tttttttttg 2820
acggagtttc actcttgtta cccaggctgg agtgcaatgg tgcgatctca gctcactgca 2880
acctccgcct cccagttcaa gtgattctcc tgcctcagcc cccaagtagc tgggattaca 2940
agtgtgcgcc accacaccca gctaattttt tatttttagt agagatgaga tttcaccatg 3000
tgttggtcag gctggtcttg aactcctgaa ctcaagtgat ccacctgcct cgacctccca 3060
aagtactggg attacaggcg tgagccactg tgcccagcct tgtacttttt tttttttta 3120
ttgtagctct gtatagcact tggataatgt ctgggtgtct ccttaaccat caaactgttc 3180
tttatttaaa atgtttaatt accgtttaga aattctagtc ctcaataagt gggtggctca 3240
tgctattgtt gttcctaaag ctaagctttg ctgggaagga aatgacctat agtttcttaa 3300
agaatcatta atccttatag gattcctgag ttactgtttt gttcctcccc actgcttccc 3360
attcctgagt tttgtaattc ctaatccttc tataatttct attaccttca ccatagtcat 3420
tctttcctta cgcaaagccc aaggaatgag ctgctgctac tttaaagtgt ggtcattatg 3480
atgaatgtga aaagagtttt ggcttgttct aaataatttt tacaagttat ggtacagaca 3540
gttgttacat atcaaaaaaa acctgtttat tgaaacagga aataaaaaag ggatctttta 3600
cattatagaa ttaaaactaa ttttttcttg tatataaact aattggtttg attttaaata 3660
tttctggctt ttattaatat gtcttaattt tgagtttgaa aatgttaagt gcaataaaaa 3720
catactagta cagattttgt tttgttccaa ttggcatact ctggggatga tcacttaaaa 3780
aaacagattt tacataatgc ctacttctgg tagatgtctt atgagattgt tctgcttttt 3840
ctaagttact tagatgttgg atatgtacat agctgtttct tgttctgtat acatttctca 3900
aatgtacact tgtattataa taacctccca gttctagggg atatttgtgc aataaataca 3960
catgtcaact tg 3972

<210> 114
<211> 4632

<212> DNA
<213> Homo sapiens

<400> 114
gttcccgcag aagctcacca tgggcatgct taagtcgccc aataccgcca tcctcatcaa 60
agacgaggct cgcaacgtct tctacgagct ggaggacgtc cgggacatcc aggaccgcag 120
tattatcaag atctacagaa aggagcccct ctacgctgcc ttccctggct cacatctcac 180
caacggggac ctccggagag agatggtgta cgcatcgcgg gagtcctcgc ccacgcggcg 240
cctcaacaac ctgtcaccag cgccgcacct ggcatccggc tcgccgccgc ccgggctgcc 300
gtcggggctg ccgtccgggc tgcagtccgg ttcgccgtcg cgttcgcgcc tatcgtacgc 360
cggggggcgc ccgccttcgt acgccggcag cccggtgcac cacgcggccg agaggctggg 420
aggcgccccg gccgcccagg gcgtcagccc cagccccagc gccatcctgg agcggcgcga 480
cgtgaagccg gacgaggacc tggcgagcaa ggcgggcggc atggtgctgg tgaaaggcga 540
gggcctctat gctgacccct acgggctgct gcacgagggc cgtctgagcc tggccgcggc 600
cgccggcgac ccgttcgcct acccgggcgc cggcggcctc tacaagcgcg gctcggtgcg 660
ctcgctcagc acctactcgg ccgccgcgct gcagtccgat ctggaggact ccctgtacaa 720
ggcggcgggc ggcggcggcc cgctgtacgg cgacggctac ggcttccgcc tgccgccttc 780
gtcaccgcag aagctggccg acgtggcagc acccccccgga ggtcccccgc caccgcacag 840
cccctactcg ggccgcccca gccgcggctc gccagtgcgc cagtccttcc gcaaggactc 900
gggctcctcg tccgtctttg ccgagagtcc tggagggaag acccccagcg cggggagcgc 960
ctcgacggcc ggagctcccc cttcggagct cttccctggg cctggggaac gctcgctggt 1020
tgggttcggg ccgccagtgc cagccaagga cacggagacc agggagcgca tggaggccat 1080
ggagaagcag attgccagcc tcacaggcct ggtgcagagc gccttactgc gaggctctga 1140
gcctgagacc cccagcgaga agattgaagg ctccaatgga gcagccaccc cctcagcacc 1200
ctgtgggtca ggcggccgga gcagcggggc caccccggtg tccggcccgc ccccgccctc 1260
ggccagcagc acccccgcag gtcagcctac cgccgttagc cggctgcaga tgcagcttca 1320
cctgcgaggc ctgcagaaca gcgccagtga cttgcgcggc cagctccagc agttgcgcaa 1380
gctccagcta cagaaccagg agtcggtgcg cgcgctgctg aagcgcacgg aggcagagct 1440
gagcatgcgc gtgtcggagg cggcgcggcg gcaggaggac ccgctgcagc ggcagcgcac 1500
cctggtggaa gaggaacggc tgcgctatct caacgacgag gagcttatta cccagcagct 1560
caatgacctg gagaaatcgg tggagaagat ccagagagac gtgtcccaca accaccggct 1620
ggtgcccggc cctgagctgg aggagaaggc actggtgctg aagcagctcg gggagacgct 1680
gacagagctc aaggctcact tcccgggcct gcagagcaag atgcgggtgg tgctgcgcgt 1740
ggaggtggag gcggtgaagt tcctgaagga ggagccccag cgcctggatg ggctcctcaa 1800
gcgctgccgc ggggtcacgg acacgctggc ccagatccga aggcaagtgg atgagggtgt 1860
gtggccaccc cccaacaatc tcctgagtca gtcccccaag aaggtgacgg cagagactga 1920

cttcaacaag agcgtggact tcgaaatgcc accccccagc cccccgctga acctgcatga 1980
gctgagcggg ccagctgaag gagcctctct tacccccaag gggggcaacc ccaccaaagg 2040
cctggacact cctggcaaga gaagcgtgga caaagctgtg tctgttgagg ctgcagagcg 2100
agactgggag gagaagcggg cagccctgac ccagtacagt gccaaggaca tcaaccggct 2160
gctggaagag acacaggcag agctgctcaa ggccatccct gacctggact gtgccagcaa 2220
ggcccatcca ggcccggccc ccactccaga tcacaagccc cccaaggccc cccacggcca 2280
gaaggcagcc ccccgaacgg agcccagtgg gaggaggggc tcagatgagt tgaccgtgcc 2340
ccgataccgc acagagaagc cctccaagtc gcccccaccg ccccctcccc gccggagctt 2400
cccctcctcc catggcctga ccaccacacg taccggagag gtggtggtca ccagcaagaa 2460
ggactcggcc ttcatcaaga aggctgagtc cgaggagctg gaggtgcaga agccccaggt 2520
gaagctgcgc cgggctgtgt ctgaggtggc ccgcccagcc tccacaccac ccatcatggc 2580
ctcggccatc aaggacgagg atgacgagga tcgcatcatc gcagagctag agagtggcgg 2640
aggcagtgta ccacccatga aggtggtgac tccgggggcc tctcggctga aggcggccca 2700
gggccaggcg ggcagccccg acaaaagcaa acatggcaag cagagggccg agtacatgcg 2760
gatccaggcc cagcagcagg tctaatgagc aggcgcggag gctgtgtgga gagtggacat 2820
acctcacctg tgggtgttta ctgcccttgt ggcctggtca gaggctgcag ggtggctcct 2880
ggacccagat gttgtgagag acccttagtg cactcgtttg atttagttaa tatttatgag 2940
cagctcctgt atgacaggcc cattctgggt gtgagggtgg gggctgtgat atgtccagat 3000
gctctcactt aaagaaggtt ctagactcag aggcaagacg ttagagaagg ttctagactc 3060
agaggcaaga ggttagagat gggtgagtcc atctcttttt cagatgaggg ggcagctcag 3120
aggaggagag ggcttcccca aaggtgcaca gcaaggtggc atcacagcca ggatcgggac 3180
ctaggaatct tggaaccctt ccctgcactg gccctgggac agccctgggc acccaaggcc 3240
ctgcctccct actggctccc aggagaattt ctcaaaaagc agccctccag ccccccgcag 3300
cctctccaca gctcccacct gcctcctcat ggcccccaga caccctgcga gccccacctc 3360
cagatctttt tcctgccact gccagggatg agttcccctt gtcctgtccg ggctggaccc 3420
tcctgggtgc ccagcagagc agctgacact gacagagctt tcgctggggc caggcatgcg 3480
cattatctca tctcacactc accacactga ggaaggtcta ttacccccat gtgacggggg 3540
cggaaactga ggcttaatcc aaggccacac ggctagcaag tgggggggcag gacacaagtc 3600
cagactgcct ccctccccac cacaaagtgc ttcagcctct ccagcagccc cttccctcag 3660
ctctaatgat gttgcactgt ttcccaccct agtccctccc tccccagagc ccagctcctc 3720
cgctgtggtg tcagcctcat gcctttatgg cccagcctcc accccatctc cggggttgcc 3780
tgggtctcca gtgacatcga gatgggctct tttcaccccc ctcccccact gctctgcagc 3840
atgtggcctt gtagaccccc cccacacctg catagggctg tgcagaccct ctgttcctca 3900
gcatcctctc cccagcctcc acctggagcc tctgggctcc tttctgggtg actccctcta 3960
ttttttgaga cggagtcttg ctctgtctcc caggctggag tccagtggtg tgatctcagc 4020
tcactgcagc ctccgcctcc tgggttcaag tgattctctt gcctcagcct cccgagcagc 4080

tgagactaca ggctaattac cacactggct aatttttgta tttttagtag agacggggtt 4140
tcaccatgtt ggccaggctg gtctcgaact cctgagctca ggtgatcctc ccacctcggc 4200
ctcccaaagt gctgggatta caggcgtgag ccattgcacc ctgcctgggc gactctcctt 4260
tgcctgcctc ttcccttcct gtgccccact cctcagcctc ttctcctcac accatgcctt 4320
cctctcctca ggcaccaggt gcactgctta gtccctgccc ccacccaggt ctccattctg 4380
agctccagtc acccacacac gcttccccgg gcatccccag gctcctcgaa tgcgtgtgtc 4440
cagacataat gctcccccga ccacgctgcc ccagccggta cgaggctgcc ccagctgctg 4500
tctccctcag gaagggtcgc ccacccccag ccttcccaag cccctccgtg cccctgtctc 4560
acttccagtg ctccagtagc ctttcttata aacatggctt ctttcttgta aagtaataaa 4620
tgtttacagt gg 4632

<210> 115
<211> 3625
<212> DNA
<213> Homo sapiens

<400> 115
acggcctcag tgaaagggaa ccgctggtga cagggcctac agaccccgac caaaaagcgc 60
atataactca cgtgcaggct tcgatgcaca aagaccccgg gggccggcgg agcagggtcg 120
gagagcgcga cagcccctgc cctgagaccc cggctttatg tgctacgcat ccacgcgtgg 180
cactgcccct tccatgcagt ctccttccgt ggataaagtg acacattttg ttttgttttt 240
tttttttaag atgcagtctc gctctgtcgc caggctggag tgcagtggcg ctgcctcggc 300
tcgctgcaac ctccgcctcc cggagtcggg caattctcct gcctcagcct cccgagtagc 360
tgggactaca agcacgcgcc accatgcccc cctagttttg gtatttttag tagagacggg 420
gtttccccag gttggccagg atagtctcca tctcttatcc tcgtgatctg cccgccttgg 480
cctcccaaag tgccgggatt acaggcgtga gccaccgcac ctggccgagt gacacacttt 540
gtaagacaaa agccatctca tgaacttcta cacccatgaa gtgtgtctgg gaggccccct 600
cctctgggca ccactgccct acgatggctc catctgtagc ctccttttcc aagaggactt 660
aagaccgaca ataaatggat cccagataca gattcccctg caagcggcaa acgtccatcc 720
ccattaccgg aaacctccag atacttcaca cttactggca gcccaggaca cggggaccca 780
aatccttgcc tgccctgagc agtggctctc gaggccagga aggggggctc gtgctcagag 840
ccaggctggc ctgcctgctc acttctgttt gccagggcac catcatctcc caccaaggat 900
gaacctgaag cttcagggca acgaagagaa acccagaagc gaagggactt gcaaccaagg 960
ctgcccaaag tggcccctgt ccaggcccat ctctaaatac aacccacacc gaggatgcct 1020
ggtggggcag aagtccctgg gtctcgttcc cgtcaggggc gagtgaacct tcacaacctc 1080

ccggggcttt ggaatttgac ttaatgatga agggcaacat ggaccactgg acaaagacct 1140
ggagttccca ctacctgcac cgctctggcc aatcccattt ggaaatcagt cagcaagatt 1200
cactctcctc tggactctga gcccccggga ggagaggatg ggagaggtca agcgtgtgca 1260
attctgttgc agcctcacaa ccaacaagca gccgtgttcc gacggctctg cgggaagccc 1320
agagggactc ccgtggctca aacgggggca gagacgtgca gggccccggg gaacgtgaag 1380
gtgagagaca gaacataccg tgaagaagcc actgagagtg ggagacagag gcaggaacag 1440
ggatgacact ggaggacagc aggcctgcct ggaggccagc attctctaca accttccaca 1500
aaccaacagc aaagcccgct ccgggccacg tgcctggcag ctgctcggcc actgccccgc 1560
tcctccctag gcaaaatccc agggaagcac cttgcgtcgt ttccatttcc tcacctctta 1620
ctcttccttg aacagtcccc ccaagaaact gcctacccac catcaacaac tggcacaggg 1680
cagatccacg ggtcaggctg tgtgcacctg accgcttcat aacccctgcg tgggcagcca 1740
gcaccctcca tcagaaatcg tttgatcccg tggcctctgg gtctccatca ttcgagctcg 1800
ggagcaacat cccatcacca tctcctctcc tcggtgggcc cctcctcgtg ttcacccctg 1860
cactgggggg aacccaggct ccactcacag aggagccaac ctctgggcag cctgccagct 1920
cgctgtgaaa gtcctcacgg ccctgactcc tcctggagct ctgctggcag cacctaagtg 1980
cccactcaga cctgaatggt ggcaccagcg gatgcatgaa atgccagccc agcacccgcc 2040
ccggtctctc ccagctcagc agcagacacc gctgtgcact aggcttgagg gccacctccc 2100
aggagccgcc cctgactcca ttctcttgac cggtctgtca tcagacctcg accacggccc 2160
ctgcccctgc tctcctgccc gttctcccgc ctggcctagg agaagccaca gcaaacccca 2220
cgttccccgc cacaaagaga aggaagtcca gagtcagtgc caggctgcca cggctcaggg 2280
gcccagccca ccacagcctt tcatgccccc ccacacactc ctgcccagga gctgaaagag 2340
ccccacactg ccgccagccc ctacccagcc ctaagactct tggcagcgca tcttgctgcc 2400
gggaagcctc tgacacggat cgtcagtgca cgtccagctc ctccaccaaa atcgaagctt 2460
ctcgtgggca gagacgccac ccggcatagc agcgcatccc catcacccat caacctgcac 2520
ttggcaagca cctccaaaca gagagagcac acacactccg ccggcagccg aaggagctgc 2580
aggatggtgc tgagagtggg agcaggccag aacgaagctc taacacagaa gagccgggtg 2640
ctggggagag acggggagga caggtgggag gactcaggcc cctccccagg caggatgggg 2700
aggccacgac acttgggcca gcttggaggg tggcggggga ggagaagagc agatgcagac 2760
tgcacctgct gggggtgacg acggtgcggc gtggccagcc cagccactgg caggcccaca 2820
ggtcagctgg atggggcaga ggtggggccc accccaactt ccaccgggcc ttgcctccca 2880
gattcctgag ccaaggttta ataacagaaa agatggagct ctaggggagc aagggacgcc 2940
gaccaagcaa gccgcagcag agaggactgt gctggagcca catcggtggc ttctccggga 3000
ggtaacgtcc tgtgcagact cccagccaca ccctggcgct gcctcggctg cctccctgaa 3060
tgtcagcggc ctgagggacc ccactcggca gggagcgggg gctgcttgtg ggaacacaca 3120
gggtctgatt ccaagtgaga ggggtgactg gtgtggcttc agacggcacc aaccacgcaa 3180
aggatacaca gcttctcgtc gtcctgaaat gtgaagtaaa gcttaacaaa gaaggggtga 3240

tccaggcgcg acatgacatc ccgctctctg gttacatagg ggaccttgtt ctcttttatg 3300
atatgtcgct tctccagaat tttaacttca ggtgagagag aagtgagtta ctatcagaaa 3360
caacaaaaaa cactaaagac atgactcaca aaggtaactg gtacaaatta aagtctttca 3420
aacattgtac acaacagcct ggtggtctct aaagccaaca gtgtcctgta ccctgaaatc 3480
agcacagaaa caccggccct gccaccccag ccgccctgca cggagccgct tgccctgctc 3540
ccggacgcac agctccctgc agcccatact cactcgcata ttctctggag gttgccagtt 3600
ctcgagccag gacaacctgg ttggg 3625

<210> 116
<211> 1057
<212> DNA
<213> Homo sapiens

<400> 116
aatcccagca ctctgggagg ccaaggtgta aggctcactt taggccagga gtttgagact 60
ggactaggca atgtggtgaa atcctgttac tacaaaaaat acaaaaatta gttaggcatg 120
gtggcatgca tctgtagtcc cagctactgg gaggctgagg ttggaggatc gcttgagctg 180
aagaggtgta ggctgcagtg agcaatgttc agtgcactgc acttcagcca ggacactgga 240
gttgagagaa aggaaggaaa taaggaagga aggaaggaaa gaaggaagga aggaaagaaa 300
gaaggaagga aggaaggaaa gaaggaagga aggaaagaaa gaaggaagga aggaatgaag 360
ggaaggaagg aaggaaggga aggaagggga agaaagaaag atgaaagaaa gagagaaaga 420
gaaagaaaga aagagagaga aaaagaaaga aagaaagaaa gagaaagaaa gaaagataaa 480
aagaaagaat tttgtcccag gaagagggcc ggcctgaaaa gaaagagcaa tcacataaaa 540
acataaaaca cagatctcca acatgctgtg ttgagacgcc tcttctgggt taaaacaaaa 600
tgaaaacagt cacagacgag gcaggggaaa gtgctggcaa agcgtgggct cctcgcagac 660
agctccaggt cctccggccg caggcgacga aggcagagcg gctggagagc gcggagcccc 720
ggcggagagc ggagcgcagc ggctgcggac tcaccccgcc gcccggcccc gcggcagcag 780
ctgccgctgc cgcctccgcc tcccggctct cccgctcgcg gctcacctcg gccgcggtcc 840
ccgcgcagta cccacctctg cgccgccgtg ttggcgtcca gcaccccggc gccctgcatc 900
cacgtccgca ccgcgttcat gccgctgccc agcggctctt ccttcatgct gcttccactc 960
cgttggatgc tttcctgaat cccaaacatg aaaacaacct tttcttgtgg aaaatctcct 1020
ccccttgaa aaaaattaaa aaaaaaaaa aggaaag 1057

<210> 117

<211> 3903
<212> DNA
<213> Homo sapiens

<400> 117
atctaaagat catgtcatca acagacagac aatttaactt tttcctttgc gatttggaca 60
aattttattt ctttctcttg actaattgtt ctggctagga tttccaatac tgttttcaat 120
agaagtgtct ggaataagca ttcttgtctt attactaatc ttacgaaaaa aactttcaat 180
tttcactatt caatatgatg ttagctgtgg gcttatcgta catgaccctt actgtgttga 240
ggtccattcc ttctgtgcct aatttattga gagtttttaa tcatgaaagg atgtttaatt 300
ttgttgaata cctttctcca tcaattgaga tgatcaggtt gggcgtggtg gctcacgcct 360
gtaatcccgg cactttggga ggctgaggtg ggcggatcac gggatcagga gatggagacc 420
gtcctggcta gtttttgtat tttcagtaga gatgggtcct cgccatgttg gccaggctgc 480
tctcaaactc ctgacctcaa gtctgcctgc cttcgcctcc caaagtgctg ggattacaga 540
catgagcctg gcctggatga tctttttaat gtgttgttga atttggtttg ctggtcttgc 600
tttgtcaccc aggctggagt gcagtggcat aatcttggtt tactgcaggc cttaaactcc 660
tgggctcaag taatcctcct gtctcagtct tttaaagtgc tggtattaca ggtgtgagcc 720
acattgcacc tggccttatt gaggattttt gtatctatgc tgatgtagtc ccattggtct 780
ataattttct tttcttgtag tgtccttgtc tggctattgt tcacgagcat gttgttcaat 840
ttctttgtat ttgtgaaatt ttccaaaatt ctttttatta tttctagttt cataccattg 900
tggtcagaaa agatacttgg tatgatttca gtcttctaaa gtttattaag actcgttttg 960
tggcctaaca tgtgagttgt cctcaagaat gttccatgtg cacttgggaa gaatgtattt 1020
tctgctgctg ttggatggaa tgttctctat gtctgttagt ttcctttggt ctaaagtgta 1080
gttcaagttt gatgtttcct tttgattttt ctggctttat tgaaagtgga ctattgaagt 1140
ctcctactat tattattatt atggaaatgg agtcctgctt tgtcacccag gctggagtgc 1200
agtggcggga tctcggctca ctgaaacctc ctcctcccgg gttcgagtga ttctcctccc 1260
tcagcctcct gagtagctga gattacaggt gggagccacc atgtccggct gatttttgta 1320
tttttggtag agatgtgatt tcgccatgtt ggccaggctg gtcttgaacc cttgagttcc 1380
ggtgatctgc ccacgtcggc ctcccaaagt gctggggtta caggtgtgag ccaccacacc 1440
tggcctaaag tcccctacta ttattgtatt ataatctctc tctctctaga tgtattgata 1500
tttgccttat gtatctagaa gctttgatgt tggatgtatt tacagttgtc ccttggtgtg 1560
ggattgcttc cagtacctct gtgtgtaaca aaagctgcac cattcaagtc ccacagttgc 1620
cctgcgaaac ctctgtatat gaaaagttgg ccctccatgt acatgggttt cccatcctgt 1680
gagtactgta tttttgatcc tcatttggtt ggaaaaaatc tgcatataag tggacctgtg 1740
cagttcaaac ccgtgttgtt caagggtcag ctgtatattt acagttgtta tattgtcttg 1800
ataaattgat cctctgtcat tatgtaatga tgttctttgt cttgttttac agtttttact 1860

tagtctgttt taagtatagc taccccctgct ctctttggtt tccatttgcc tgaaatgtct 1920
ttttctagcc tttcactttc attctatgtg tgttcttaaa tgtgaagtta atcttcatag 1980
gccacatata gttgggtctg tttttaaatt tttgatagta tccaacctaa tgggtgtgag 2040
gtgataattc tttgtggttt tgatttgcat ttctctaatg attagtgatg ttgagcatct 2100
ttacatatga ttgttggcca tttgtgtccc ttctttggag actattcaaa gtcctttacc 2160
cattttaaaa atgaaggcat ttgccctttg ttgttgagtt gtaggaattt taaaaatata 2220
ttctggatag taaatccttt tcagatataa gatttgcaaa tgtttttttcc cattctgtgg 2280
gttgtctttt cactcttttt ctttttcttt tttttttttt tttttttttg agacaaggtc 2340
ttgctctgtt atcactggag tacagtggcg tgaatgtggc tcactgcagt ctcgaccttc 2400
ctggctcaag ggatgctccc gcctcggcct cccaaatagc tgggactata gacgcatgtc 2460
accacatctg gctaattttt tatttttttgt agagatgaga tttcaccata ttacccaggc 2520
tgttcttgaa ctcctaggct caagtgattt gtctgcctca gcctcccaaa gtgttgggat 2580
tgcaggtgtg agccactgca cccggctgcc ctttcactcc taattgtatc ctctgttgca 2640
cagaaatttt taagtttgat gtagtcccat atgtctcctt ttttttcccc atgcttttta 2700
tgatttctag catctttttg tgggcttatt gtgcttgtat atcttctttg gagaaatgtc 2760
tgttcaattc ctttgccctt ttttgaatgg ggttgtttgg tttttctgtt gtggaatatt 2820
taaatttctc tatgtatcct caatgttaag ccatactaga gatatgcttt tcaaatattt 2880
tccccccattc tgtgcatcac ctttttttact ctgctgaaag tgctgtttga tgcaaaaaag 2940
tgtttaattt tcatgaggtc caatatatct atttttttctt ttgttgcctg tgccttgggt 3000
gttatattca agaaatcatt gacaaatcca atgatatgct cttctacact cttaaaaatt 3060
atagacaacc ccaaataact tttatttagt ggttttaaca atatttacca tgtctgaaat 3120
atgataaaca ttaaaattag tattttggaa aaatgccata ttagaaactg atgatttaaa 3180
agtaacaaca atgaatccat tacatgtgaa catactgttt ttttgtttgt ttgtttgttt 3240
gttttgagac ggagtttcac tcttttgccc aggctggagt gcagtggtgc gattgcggct 3300
cactgtagtc ttcgcctccc aggctcaagt gattctcatg cctcggcctc ctgagtagct 3360
gggattacag gtgctcacca ccacacccgg ctaatttttg tagagatggg gtttcaccgt 3420
attggccagg ctggtcttga actccagact tcaagtgatc cacccacctt ggcctcccaa 3480
agtgctggga ttacgggcat gagccactgc accaggccaa catacttttt ataaaaacag 3540
ctgtcttctc taaaacaaca aaaaaatgta gataatagta gtatcatttt atagttttgc 3600
aactctcttt aatgtttggc ttaatagaag atagttggat tctcgtatct gtttttgtat 3660
tcagtctgtt gtgtatgttg ttttgattga agtagatgaa ggaaatccag ctacatacag 3720
atttggagtt ggaaaaaata gtattttaat aacctttttta gatcatggtg gatactcttc 3780
tttgttatgt catcaaaatt agacaaatgg cagtttctga aaaattagtt gtaatgtgtg 3840
taaaaaatta gttgaatcca tatcagtgaa ctcatacttt tctatattaa acttcattgc 3900
tct 3903

<210> 118
<211> 3650
<212> DNA
<213> Homo sapiens

<400> 118
taatggcctc ttttgtatgt gggagaccat ggtgttgagg ggaggcagtt acacaaaaag 60
cccagatacc gagtgttagt tccatgggtg tggctggtca tggtgcgcac tcacgaagga 120
tgtaggtgat gccctccact ccgcagagcc agtcccacag gggtacccat tctgactcct 180
accaccacag ctccctacct cccgcctgcc atgggttcca cgtgcatggc tctgggcagt 240
ctgtgtcttt gcgtgcgtct gtgtatgtct gcgtgtatct gcttacattc gctcagcatc 300
tgagattcac ccaggctttt gcgtggatca gcggcagact cctgattgcc gcgtggcggc 360
ctgttgtgtg ggtgtgtgcc gcgtgtgtat ccagtcccta ctcaggagtg tacgtttcca 420
gtgtgggttg cagtcaggat ccatttctcc cttaactttc agattatttg ctgcataggc 480
ttttttctcc taatttggcg cctttgtcag aaatcaactg acctacctgt gtgggtctgt 540
ttctgtttgt ccttacgctc ttgccattgc cacactttag acaaggtctt gctctgtccc 600
tcaggctgga gtgcagtagc gtgatctcag ctcactgcac ccttgacctc ccatactcca 660
gcaatcccct cgcctcagtc tcctgagtag tagctgcgac cacaggcgtg tgtcaccaca 720
cccggctgat tttatttttt ttatttttat tttttttgta gagacggggt tttactatgt 780
tgcccaggca ggtcttgatc tgggctcaag tgatgctcct gtctcagcct cccaaactac 840
tgggattaca ggttgtaggt gtgagccatg gtgcccggcc gccacactgt cttggctaca 900
gttgggttta taataaaccc tgaatcgagg tgatgtgcat tttccaactc ttctttttaa 960
agattcttgt ggctgtttat gtccttggtg tttccatata tgttttacaa tcagtgtctc 1020
aatttctacc aaaaaacatg ccttctggaa tttcaatagc aattcccttg aacgcatttt 1080
tgaatacctt ttctgaatgt cttgaataca tgttgggaaa aatcggtgtc ttaacagtct 1140
ccagtgtcca cgcgttgact gccgtgtgtg actccagtcg gcgtctcaac agtctccagt 1200
gtccacgcgt tgactgccgt gtgtgactcc agtcggcgtc tcagcagtct ccagtgtcca 1260
cgcgttgact gccgtgtgtg actccagtcg gcgtctcagc agtctccggt gtccacgcgt 1320
tgactgccgt gtgtgactcc agtcggcgtc tcagcagtct ccggtgtcca cgcgttgact 1380
gccgtgtgtg actccagtcg gcgtctcagc agtctccggt gtccacgcgt tgactgccgt 1440
gtgtgactcc agtcggcgtc tcagcagtct ccggtgtcca cgcgttgact gccgtgtgtg 1500
actccagtcg gcgtctcagc agtctccggt gtccacgcgt tgactgccgt gtgtgactcc 1560
agtcggcgtc tcagcagtct ccggtgtcca cgcgttgact gccgtgtgtg actccagtcg 1620
gcgtctcagc agtctccggt gtccacgcgt tgactgccgt gtgtgactcc agtcggcgtc 1680
tcagcagtct ccggtgtcca cgcgttgact gccgtgtgtg actccagtcg gcgtctcagc 1740

agtctccggt gtccacgcgt tgactgccgt gtgtgactcc agtcggcgtc tcaatagtct 1800
ccggtgtcca tgcgttgact gccatgtatg actccaggta ttgagttcat cttcaacttc 1860
tcccaacaat gaactggagt tttctgtgca tgtgtctggc atgttttcct ttagatctag 1920
tcctaggtgt tcgatatgct attgaggact gtgtattttt atttcatttg tcacctgtgc 1980
gtggtgcttt aatcctgtgc cctaaactcc cgccttggcc atggcggtgt ttctgtggac 2040
tcgtggggtt tccgtgtgca cacgcgtgtc ctctgtctga cagtggcttt gcttcccctt 2100
gttagtctgt gcctcggctt ctcactgcac tggcgaggtg agccggcgct tgctaatctt 2160
attcccagtc tcggtgaaca tgggctcagt ctctcccggc tcagtgttgg gtttgcactg 2220
gtgcacttac aggcggaaga gcttcctcat ttgctgaggg cttttcctga atccgtgttg 2280
aatgtggtca gctgcctttc ctgcacctag tgagatgctc atgaggtcac ttccttaccg 2340
cattactgta gtgaattacg ctgactttca tatgccaaga tgacctggca tttccaggta 2400
agtccctgtg gccatggtgc attggccttt tctctgcatg gccagatgag atttgctcat 2460
accgggctaa ggacctcctg tgtaggaatg ctcttgaggg acactgggct tggatgttca 2520
ttgtcttgtc atgtgatgtg tctgtcatgg tttgatattg cattaagtgg gcctcctgaa 2580
acaagttctg ccatagtttg tataggattg gcattatttg tttcatagag atgtgatggg 2640
tgaggccatc tatgcctgcc attttctttt tgcttaggtt tttcattaag aacttgattt 2700
ttaaaaatag acatgaggcc tggcatggtg gctcacgctt gtaatcccgg cactttggga 2760
ggccaacgtg gatggatcac ctgagggcag gagtttgaga ccagcctggt caacatggtg 2820
aaaccccgtc tccattaaaa atataaaaat tggcagggtg tggtggtgca tgcctgtggt 2880
cccagctgct ctggaggctg gggcgggaga atcgcttgag ctcaggaggt ggaggttgcg 2940
gtgagctggg gtcgcaccac tgcactccag cctgggcaac agagcgaggc tctgtctcaa 3000
aaaaaaagac atgaggcttt tggattttct gtttcttcct ttgtcatttt tttaaagata 3060
ggtttgtcgc agaatttcat ctaagttatc aaaacaattt acataaatca ttcacagtat 3120
cctctgtgca gtgatgtcca ttctcattcc tgctctgggc catttgtgtt ttcttttccc 3180
cctgatcaga ctagctaaga gtttatcatt tttacagata ttttccccaa agaaacctac 3240
ttttgggtcc attgattttt tttttctatt gttttctctt tcattgactt ttgctctttt 3300
cttatgaatt tcctttcttc tacctatttt gtgttcaatt tgctgtcctt gttttaggtt 3360
cttttgatag aggcttagat cattgatgaa gaatatgaaa gaatgggata atacagttaa 3420
aaacttggtc tacttatgcc cattagagaa aacatcctta agcacgcatg gaatctttta 3480
aaaatggaca aggtactagg acccaaagca aatgtcagta attcgcagat gataatgtaa 3540
tgtataaata tatgatatgt aggtaaataa tacaaatcac attctttggc cagagtgcaa 3600
ttgaggagga aatcagtaac aaaaagacaa ttaaatccat ttgattcttt 3650

<210> 119
<211> 3588

<212> DNA
<213> Homo sapiens

<400> 119
caattgcatg ctgccactcc tctgcttagc atccttccag ggtttcccac agcattcaga 60
gtagtgccca aactcgtagc caagcttccg agacaatgaa gacctggtcc ctgcagattt 120
ctctaggcaa atctcaggac tgtatcccct tcacttatta atttccagcc cctcctctct 180
gcacattcac atgccatgct cttagttcaa tctgccctat ggcccatagc attcctttta 240
ctgggctgca cttcccttct gctctttgca tgttgtccaa atcctcattt ctcaggcctc 300
agccttacct tttaaagccc ttctctaaca acaactacaa aatggtgttt ctatccctta 360
accttatgct aatatcccct tcagctattt tgccctttga agttcttttc ttgtttgttg 420
actgtcttcc ccactggact gaaagctact taaagcctgg agccatgttg gttttcttta 480
ctgttgtcac ttcagcacct ggaatgatgc ctggcaatgg aaggcattca ataaatatcc 540
attgaataga caagagaaaa agaaaaagaa aaagaaaaac aacagcagtc cttcaagcta 600
caactcagta gggaaaataa taaataaata tataaataaa taaataaacc tagtaacaca 660
ttaaatgaat agtttccatg tactgactag ttagtatata ccacacacat agtgtgccca 720
acattttatt ttattgccca caattctata agcaagatat tactctccct gtatattttt 780
ctttcccttt ctccctccct ttactttctt tctttcttcc ttctttcctt cttttctttc 840
tttctctttc tttctttctt tccttccttc tcttctcttc ttctttcttt tctttctttc 900
cttttcttct ttctttctta atttctttct cctttctttc tttcttcctt cctccctttc 960
tttactgctt gccttctttc tttctttctt tctttctttc tttctttctt tctttctttc 1020
tttctttctt tcttctctct ctctctctct ctctcttctt ttcacaagat ttcactctgt 1080
cacccaggct ggagtacagt ggcacaatta tgatcatggc tcactgtagc catgacttcc 1140
caagctcaag caatcccccg acttcagcct ccagagtagc taggactata ggcatacacc 1200
accatgccca gctaatttaa aaaaaaaaaa agtttcccaa agtgctagga ttacaggcat 1260
gagccacagt gtctggccaa tatttttcaa actaactaag ataaagaaaa catggtactt 1320
atatacaatg gagtactatt cagccataaa aaagaatgag attctgtcct ttgcaacatg 1380
gatggaactg gtcatcatta tgttgggtga aatatgccag gcacagaagg aaaaacatca 1440
cattttctca cttatttgtg ggttctaaag atcaaaaaca attgaactca tggagataga 1500
aaatagaaca atggttacca gaggctgtga aaaatagtgg gggagtggag ggaaggtagg 1560
aatgattaat gcatacaaaa catacatagt tagaagaatg aataacagct agtatttgct 1620
agcacagcag aggggctata ggcaataata atttaattgt acatttacag taactgaaag 1680
agtataatta atataattgt ttgaaacaca aaggataaat gattgagggg atagatacac 1740
aattttccat gctgattatt acatattgca tgcctgtatc caaatatctc aggtacccca 1800
taaatatata tatctactag gtacccacaa aaacttaaaa aaacaactat gaaatgataa 1860
atgataaatg attgagggga tagatacaca attttccatg ctgattgtta tgtattgcat 1920

gactgtatcc aaatgtctca ggtaccccat aaatatatat acctactatg tacccacaaa 1980
accttaaaaa acaaacaact aagaaaccag aagctcagac aaaaaaagta ccttggccaa 2040
agtcatagtc ggtaagtggt agaaccaggt gttggttgta gaagggtttg aataccatac 2100
tgagaatgaa gactttattt aattgacatc taggaagcac tgagggtgtt ttgagcaagg 2160
taaaataatt cttaaaatat ctgattagta ggagtattca tgccctgaat tataatagcc 2220
aattggtagt tttcaccttt gaaacctaaa tggagctgat attcaatttc tctctcaggc 2280
tttgcccaag gactcaattt ctgattctac tccctcaatt aaagagaatt gggttaactt 2340
tttgaatgta aaattctcct ctgacacaca aataatcaca aggacaatta aagtgataaa 2400
atgtcgagtt aaatgggaaa gaaagtcaaa cataatttct tctgagtgtt tataattatt 2460
ttgtggcata aaattgtgtt ctgtgcgaaa attcagaaac tttaattttt aagagagaaa 2520
aatagcataa tcactatact cccttcctaa atgtgttttt aacataacac attataattt 2580
atgccacatt tttaatatga aataatttta atgtgtctgg cctggttcag tcctgttaaa 2640
caacaaaaaa gactgcaaaa tgtatttccc catgtggcta gatacattct aacgtcgtga 2700
agaaacacat tatgtatata tgtttttcta tattctcaat tatagataga aatccaggca 2760
caaagagatg tatctttggg caacacgata atttgtgaaa aggctacaca gttaaacaag 2820
cattttccaa gatttgaaaa actaatgagg gagtgtgaaa gcacaaagaa attaaattgt 2880
aagctgtaag gactgacttg aaaatattaa agagaggtat aattcacata tagttgtgtg 2940
actcttttc cttaaaaatc cattttgatc attttcttat gtcattaaat attattcatg 3000
ctgtacaata aatatataat acttcaacat ggggctatgc cattattgct ttaacaaagc 3060
tttcttttat atagcaaaac tcctttcaa cttttatttt ttgcttttc agtttcaata 3120
atgtaaaaaa attccactgg gatgaaaaaa tattttttat taatcagctt tatttagcta 3180
taacttgctt atgatataat acacactttt taagttttat ttcaaggagt ttgaattata 3240
tatataccca tgcaaccatg attatagtta gaactcagag tactccaatc agacaagtgt 3300
cccttagtgt cccccacttc cctggcctag ggaaccattg atctgtcact aaatgaccta 3360
tgcctattct agaatttcct gtcagtgaaa tcacacagtg tatatgcttt tgggtttggc 3420
ttttctcatt cagaataatg actttgctat ccatccatgt tgttattgta acaagttcat 3480
tgttgctgag tggcagattc acaatttgtt tattctttct ccctttggaa tatttgtgtt 3540
gctttaggtt tgggttgttt tgaataaggc cactatgaac attcgctt 3588

<210> 120
<211> 4032
<212> DNA
<213> Homo sapiens

<400> 120
   agacaatgac aaatatgagc ctgaaggaag atgagctgat ggcattccca gcttattacc 60
   actccttggg ggccttatct tacatacatg gattcaattc gtagattcag ctgggattta 120
   ctgcctcaag atggttatgt tggaggattc caatagttct actggatgtg gagccagaaa 180
   ttgtgtggaa tgcctggtgt ttctttcagt tcttggatgc caatctgagg cccctggcag 240
   ctcgtggttg gcaagagcac gaacccgtgg tcagatgcaa cgtcctgcct catgcatttt 300
   cctcttggtg ctttggtcag aacttcccca agtggagtga aactcaggag ctgagaaacc 360
   gagtcactgt gaaaagatgg gaaattatct cctgcgaaaa ctcaggcagg aaatgactac 420
   atttgaaaga aaacttcaag atcaagataa gaaaagccaa gaagtttcat ccacttctaa 480
   tcaggaaaac gagaatggca gtggttctga agaagtgtgc tacactgtca ttaatcacat 540
   cccccatcag agatcctccc tgagctccaa tgatgatggc tatgagaaca ttgactccct 600
   cacaaggaaa gtgagacagt ttagagaaag gtcagagaca gaatatgccc ttcttaggac 660
   ttctgttagt aggccttgtt cctgcaccca tgagcatgat tatgaagttg tgtttccaca 720
   ctaaaatcct caagctgctt tatcaccttc cagcaatgaa gacaatgcag aatagcagac 780
   tctggcgaag ttgttcaccc tgagcagtgc atgaaacatt cctttctggc taaagtttag 840
   aaatattatc ttattatata tccttaggca actctgatat gtggcatctc tgtggcttag 900
   gtgaaatcat agaaattgac acaatgacct aaaatattct atgtgttttt gcttgtaaag 960
   tttgaggaca tggaggtgat aaaaaaaact ttcttaggac aataatgtaa aatgaaaata 1020
   aatttctaat ccccctgact aactgaatgg accctcttct aggccaaaga gacctcagat 1080
   gaacctgaaa gactgaattc tggccatgat aggaagggag gtgagacaca ccttgttata 1140
   ccccttccct tttggagttt atgcacaagt gaccaggatg agtcataaga ctgatggaat 1200
   agactgattg tggcaataag agtcccaatt ccaacctgac tctggtgtag atcacacact 1260
   gtctgaggga ttccatctat gagactttgt ctacataaca gagaccttgg tttccacaac 1320
   ccctttattt tagctaaagc attcttttct actgacttct taagtcttta gacaaagctt 1380
   aactctttca accaattgcc aatcagacaa actttgaatc tacctatgac ctgtaagctc 1440
   tctcctgctt caagatcttg cctctttaag ctgaaccgat gtgcactttc catttaatga 1500
   tttatgtctt tgcttgtaac tcctgtctcc ctaaaatgta taaaagtaaa cggtgacctg 1560
   accacctcag gcacactttc tcaggacctc ctgagagtgt atcccaggcc atggtaagtc 1620
   atgttggctc agaatcaacc tctttaaata ttttacagaa tttgggtttt ggttaccaat 1680
   aagtctccac aaatatatgt ccaagaatct tcaattccaa gcctgctcac caaatttcaa 1740
   atgccaacat ctccccatcc aattacctat ttcacctttg aggtgtaatc tactcaataa 1800
   actgtgtaag accagtgacc agaccctttg ctaacctgac atttacttca atttttcttt 1860
   ttctatgtac tggatatttt tgcatataaa cttgcagtaa tagttcaaaa attaatagtt 1920
   tttgacattg gcttttctga gaagagaaat tgaaagtgtc acaaaataaa aaaagatgaa 1980
   atgaagcata tataattgtc aattttttca attttctagc caacagagaa tcgaaggatt 2040
   ctgttcaaat attagtaaaa attgaaaata aacttgtgct tatattttgt ttgcaacaca 2100
   ctagttaatt taacctgtga ctagttatct ctaccgaagg tggatgtgta gtttctggtt 2160
   ttaaaattca agcaaactgg aaaataatcc atctaattat gctttctttc ccaagaagtt 2220
   ttttaatgat atgccagctt cctaatttgg agacaaaagc cttaattgac aatgcattca 2280
   ttatatattt ttttgtatag ttacagtata cgagttgagt atcccttaga tgagatgctt 2340
   gggaccagaa gtgttttgga tttcagattt atttttggat tttggaatat ttccatacat 2400
   ataatgagag agttggaaaa tgggattcaa gtctaatcat aaaattcact tatgtttgat 2460
   atacacctta tctgaatagc ctgaaggtaa ttttatacaa tattttaaat aattttatgc 2520
   ctgaaacaga gtttgcgcac attggaccat cagaaagcag aagtgtcact atttcaagtc 2580
   agtgctcaaa aagtttcaga tgttaagctg gtgatgcagt tcatgccagt gatccgagta 2640
   ctttgggaag ccaagacagg tggatctctt gagcccagga gtttgaggcc agactgcaca 2700
   acacagtgag acctcgtttc tacaaataat taaaaaatta gccaggtgtg gtggtgcaca 2760
   cctgtagtcc caggtactca ggaggctgag gtagtaggat tgtttgagac tgggaggttg 2820
   aggctgaact gagccaggat cttgccacca cattccagct tgggcaacag agtgagaccc 2880
   tgtctcaaaa aaaaaaaaaa agtttcagat tttggagcat ttcagatctt cagattaggg 2940
   attttcaacc tgtactgacc ttttagtcat tgacaagcat taatcaatag gtggactcca 3000
   gataactcat ttgctgtata cacattttgc ctctctattc aacgaattct tatgccctct 3060
   tgtggtgatt ttaatgtgcg gaagggaaac aatagaaatt ttgcaattct agaaaagtca 3120
   ttctgtcaaa atatgtcagt cctgtagata ttagccaatt ttaggaaaat gacaaaattt 3180
   tttacttttc gtctgccttt gtagctgttt tatgatataa ataccttatt tgtaataaaa 3240
   ttaattttaa tttgagtaac aatctggaat tatcagagaa ggggcaagca ataggttaat 3300
   aaacagtatt gattggtaga agggacgttg aaatccaaga gcatcaatgt cttctggtgg 3360
   ttcaccataa gccacagcag atgtcttaat ctttccgaga tctagttttt cagcaaagca 3420
   ggatttaaga aatgtaacta tcttatgtgg ttatgaagaa caatagaatc attgctgtat 3480
   aagtgctttt taacctgtaa attttgtgaa gcttatcttt tatgcatata aatatttgaa 3540
   cattttacat tgtttatatt tttaatcagt tttactcaag tgtgattata tacaagaaaa 3600
   tgtaaccact gtaagggtag agttataaga attttgtcaa atgtattcac ccatgtagtc 3660
   acctccttat gaagagacag aacacgtaca tcctcccaga aagttccaca gtgctccttt 3720
   tccctgagtt tcaccagtcc tggcaaccaa tgatctgctt cgtataatta taactgttct 3780
   agatatttgt agcaatgtac cctttccata tttattttgt gtgtgtaagg cttcttttag 3840
   tcattataat atttttgaga ttcatctatg tttaatgttc tatcagtagt tgtacatctt 3900
   acttgtctca gcatatcacc atatagatat actataattt gttaatctaa tcactgatgg 3960
   atatgtagga tatttaagtt tttgacatta tgaataaagt ggctataaat gaataaagcg 4020
   actacaaatt tg 4032

<210> 121
<211> 3701

<212> DNA
<213> Homo sapiens

<400> 121
acatatgtag gcctgggcat ttctatttgc agcctgatcc tttgcttgtc cgttgaggtc 60
ctagtctgga gccaagtgac aaagacagag atcacctatt tacgccatgt gtgcattgtt 120
aacattgcag ccactttgct gatggcagat gtgtggttca ttgtggcttc ctttcttagt 180
ggcccaataa cacaccacaa gggatgtgtg gcagccacat tttttgttca tttcttttac 240
ctttctgtat ttttctggat gcttgccaag gcactcctta tcctctatgg aatcatgatt 300
gttttccata ccttgcccaa gtcagtcctg gtggcatctc tgttttcagt gggctatgga 360
tgccctttgg ccattgctgc catcactgtt gctgccactg aacctggcaa aggctatcta 420
cgacctgaga tctgctggct caactgggac atgaccaaag ccctcctggc cttcgtgatc 480
ccagctttgg ccatcgtggt agtaaacctg atcacagtca cactggtgat tgtcaagacc 540
cagcgagctg ccattggcaa ttccatgttc caggaagtga gagccattgt gagaatcagc 600
aagaacatcg ccatcctcac accacttctg ggactgacct ggggatttgg agtagccact 660
gtcatcgatg acagatccct ggccttccac attatcttct ccctgctcaa tgcattccag 720
ggtttcttca tccttgtgtt tggaaccatc ctggatccaa agataagaga agccttaaag 780
ggttgagtag cctctgcaaa aaggagctcc agaatatcag agaatgcttc ttctgaatta 840
tcttgtcatc ccatcaaagg cccaagttaa caaaccaggc tacatcccta acctaggagg 900
aatggcagag cccaaaggag aaatgtgctc attcctgtct tcttgcttct ggagaatatg 960
taggaagctc acacatgtgg tgctgatgga ggagggcgtg atgtgcagaa aggatgaaag 1020
gctcctctgt ctaagctgtt ctgtgctact aaacatttct cgtccatcaa ctgtacgtag 1080
ctacatatat gtcatgctgg gcagccacag caaacaaaaa gacaaagggg gccacaaccg 1140
tggtttttgt tggggcttcc atctctatgc tgttgggtca catttgtctt cagaaagcaa 1200
cctctgtctc atcttggaaa aggcggcctg agatttcacc tggctggatt ctgtgtgttg 1260
ctgagggagg agcctcatga atagtcacaa agagttgggc tgactctaag taggacattg 1320
cagattattt tttttttttc aaaaggaatc cactgccagt atattccact ttttcaaatt 1380
taaatagatt ggattgtcaa cttggtttag gcttagatac aacttccttg gccatgaacc 1440
aaggggaaaa gcatagagct tttgagacag aaagatgacc attttcagtt aaggagatgg 1500
aagcctggag ttgtgagtca atgctaatcg aggactgagc cagaggtaga aacccaagtg 1560
tgctggctcc cagccctgta cttcctcttc aagaccatat agacactgaa agatcttctg 1620
cctacactga caatgtaagg tcaatcctca ccaacaatat gctttttgga tacctgggat 1680
tggagagagg aaatatcaca tttccaattc agagctgctc tttttcttaa cctgtatgta 1740
aacaaattca acattcaaac caggtgttac ctgatagaac agtattgtag gtagaagctc 1800
tttgaggaaa aaaaaagcct ctttacaaag caaggacata gatgactgca gcagcatcta 1860
actagtggat gaaggcaaag gggcaatggg cagcctttaa ttatcccaac aagaagtcag 1920

tattgatgac gtttcattac aaaggagaaa gtggggctca ctttaggtat gagtgcagtc 1980
ccttccttcc ttccttcctt ccttccttcc ttccttcctt ccttccttcc ttccttcctt 2040
cttccctccc tccctccctc tctctctctt tctccctttc tctttctttc tttttttctc 2100
cctctctctc tttctttctt tctttgtttc ttattacaaa taatcttcct ataagtagga 2160
aagtttgagc agacaaaaag catcaatctg aagttaaaaa aaatgcatca acctggagaa 2220
atagtcattt ttcaaatggc attgcttatc ttgggacaac aaaagcatcc ttcacaaata 2280
aagtcagaga gtggttgtaa tttaccattt accaatagat ttgtatgttc tttccaagag 2340
aaggggtgag atagacatag tcctcgtttt cctcaatgca ggcttaggta caaagagatg 2400
tcacttaccc agagtccata tattgaagtg aaaatggaat ttcatgaagc aaatatagtt 2460
ataggatggt aactctggac aaattttata aaagatgaac tccaattgcc tcatttcaca 2520
gataacagct ctgagcacta gggagagagg gagggattgg cctagcattt gctttgtgtc 2580
ttgagcagtc acatctatta tggaagtact cactactcag ctatactcac tccctgagac 2640
aggatggttg gaaagaaacc ccaccttagc ttcatcatta accaagaaac cccaaggatg 2700
cttagagaaa aactctggaa ttatttggga attcctggaa gaagctatgc cctctctcct 2760
gcatcccaaa gcccactcta caccatcttg aaaaatgtca aggaaccgca tggattaggt 2820
tgactctgga tgaccctggg tagagacaca ttcagtttcc tgctgacacc gcaccaagaa 2880
agtgatttgg tcaggtgact ctaggagaaa ttggtactga tagtatccaa ggcagatttt 2940
taagtatcag acttaataca taatgattct tgggaaagcc atttaaatcc ctgagacttt 3000
aagtggataa atgtaggtaa agtagtatag tacttggctc ataataattg ctcattcaaa 3060
ttcacatgtt agttttagtt gtttttttta aattagagtc ctgtgaatac atgaacccac 3120
ccacccacat gcaaacacaa gcaaacacac agccatgacc ccatgcataa gtttggctga 3180
agacaccctc atgggaacta gttcactgac ttccaaatta aagagttaag catttgattt 3240
ggtctgttca acattagggg tatcagccag caaaatctgt tgagcttacc attgaaaaat 3300
cctttcattc attactgtgg tcataataca cagttacctg gccaggaaag agtacacatc 3360
acagggatct gctcactgca cgtgtgcctg agctctccct cactttctgc cttcttcaag 3420
aattcttccc tgattggtcc aaccagaagc gctgattctc cccacctgat ttcctgtaat 3480
tttataatct atgtccttca cttttgtgtt atattgttta tttccttttg gtctttttgt 3540
gtgcatatgt atatgttttg gggaatgggg tattcacttt tgttactcac tgtgttactc 3600
acttttgtat gcccatagtg cagagcatgg tgccttgtac atagagtatg ttcggtaaat 3660
atgtgcaata aaaagtcctt tgattacaca aaaaaaaaaa g 3701

<210> 122
<211> 4818
<212> DNA
<213> Homo sapiens

<400> 122
   taacatcagc tgccgaacgg acgggcagat gccttgcaca tgttgctggt tctgccaagg 60
   tcagcgtctc ttcctctgga agtcctttgt cttctccatc ctttgtccat cctactgttc 120
   caccctttat tctgcagcag gcccccagcc tgtctttgtc tggtgggaca gtcacatttt 180
   tgtagcaagc actgctgtta ctttacagaa cgctcctgag tttgagtttg tccgctgttt 240
   cctggtcatg ggatttgaag tgtgtcttag gctgtcacat tatacagagg acatggtgtc 300
   cctcttaggg tactgtgtcc tgaggcagcc agtgcctgtc tcgtctgcat gaccttgttt 360
   gttcttttaa atttaagtga aattcacaaa acaacaattt tttgtttttt ttattttcat 420
   aataatatag aggcagggtc tcactttgtt gcccaggctg gtctcccaac tcctgggctc 480
   aagctgtcct cctaccttgg cctcccaaag tgctcagatt acaggcgtga gccagcacac 540
   cgggcccaaa ataactactt taaagttact ttaaactact cagtgacatt tggtacatcc 600
   acagagatgc agtcaccacc tctttctcat tcagaaacat ttccactcta gctgggcacc 660
   acggttcacg tgtgtagtac cagctactag tgaagctgag gtggaaggat ggcctgagga 720
   gttcaaggcc agcctgggca gtgtagtgag atcttgtctc taaaacataa taaaaaagac 780
   attttcatca tttcaaaata aaccccctgc cctttaagca ctctctcccc gtccctccct 840
   cccctcagca cctgggcagc cagtagtttc ctgtgatttc cctgtctgga catttcgtgg 900
   aaatggaatg cccagcctct cgtatggcag tgtgtttgac cttttgtgcc tagctctcac 960
   ctggcatact gttttgtggc tcacccccgt tgtgtagcat gcatcagtgc tggcgtttct 1020
   attgctaaat aatattctct tgtatggcta gaccacggtt tgttttccca ttcttcccct 1080
   gaggcacatt tgggctgttt ctgtccttca gctgttgtga gtggtgctgc tgtgaacacg 1140
   tgtgtgcaca tatgtgtttg agtgcctgtt ttcacattcg ggtttatgcc tgtgagtggg 1200
   attgctggat ttatgtggta attctatgtt taactttttg aggaaccacc aaattgtttt 1260
   ctgcagcagt tgtaccattc tatattccac cagcaatgtg agggttctca tatctcgaca 1320
   tcctcaccaa tacttgttct tgtttttctt ctttttaata gtcgtcctgg tggctatgaa 1380
   gtagtacctg attgtggttt tgatttgcat tctctaaaga cttatgttta gcatcttttc 1440
   atatgcttgt tggccatttc cgtaccttct ttagagaaat gtctactcaa ggcctttacc 1500
   catttttgaa atgggtggtt tgtccttttg tggctgggtt gtaggagctc ctggcatgtt 1560
   ctgagtacca ggctcttgtg agagatgtga tgtgcagatg tcttctcttg ttctatgggt 1620
   tgtcgttatt tacattcctc ataattaatg tctttttttt tttttttttt ttttgagaca 1680
   gagtgcagtg gcacgatctt ggctcactgc aacctttgtt tcctgggatc aagtgattct 1740
   catgcctcag actcccaagt agctgggact acaggtgccc gccaccatac ctggctgatt 1800
   tttgtatttt tagtagaggc ggggtttcac catgttggcc agactggtct caaactcctg 1860
   acctcaagtg atctgcccgc cttggcctcc caaagtgctg ggattacagg cgtgaggcac 1920
   tatacccggc ctagtacagc catcttaaca atatttttac tccacgaaca tgagatgtgt 1980
   tccatttatt taagctattt tttcagcaat gttttgtaat ttatagtgta cacatttttc 2040
   ccttctctgg ttaaactatt cctagctatt cttttggatg ctgttgtaaa ttgaattttt 2100
   tttttcagtt tccttttcag tgtgttcatt tctggtgtat agaaacacaa ctgacttgtg 2160
   tgtgttgatc ttatacccta taactttgct gagcttatta gtttttgtgg gattcttttc 2220
   tttgggattt tctacgtgta taatcatgtc atctgcaaag agagatagct ttacttcctt 2280
   cctaacttgg aatccttttc atttctgttt cttccctgat cgctctgcct agaacctcta 2340
   gccccatgtt gaatagcagt ggtgaaagca ggcatccttg ccttgttgct gatctttttt 2400
   ctttttttct tttttttttt ttttgagaca gagtctcact ctacatgtag cccaggctgg 2460
   aatgcagtga tgcgatctcg gctcactgca agctccgcct cctgggttca cgccattctc 2520
   ctgcctcagc ctcccgagta gctgggacta caggtgccca ccaccatgcc aggctaattt 2580
   tttttgtatt tttagcagag acagggtttc actgtgttag gatggtctcg atctcctgac 2640
   ctcatgatct gcccaccttg gcctcccaaa gtgctgggat tacaggtgtg agccaccgca 2700
   cccggcccgt ggctgatctt aggggaaaat tttgtctctc accattgggt atggttagct 2760
   gttttatttt gttgaggagg ttcccctcta ttcctagtgc ttttctcagg aaagagtgtc 2820
   gcgtttgtga ctttgactgc ttggttgcag gtgcgcacca ctgtgcccgg ctgatttttg 2880
   ttttttttgt tttgttttgt tttgtgacgg agtctcgctc tgtcgcccag gctggagtgc 2940
   agtggtgcag tctcggctca ctgcaagctc cgcctcccgg gttcacgcca ttctcctgcc 3000
   tcagcctccc cagcagttgg gactacaggt gcatgctgcc acgcccggct aattttttgt 3060
   atttttagta gagacggggt ttcaccatgt tagccaggat ggtcccaatc tcctgacttt 3120
   gtgatccgcc tgccttggcc tcccaaagtg ctgggattac aggcgtaagc caccacgccc 3180
   ggcctgattt ttgtgttttc agtagagatg gggtgttacc acattggcca ggctggtctc 3240
   aaactcctga cctcaagtgt gatccaccca ccttggcctc ccaaagtgct gggattacag 3300
   gcaggagcca ctgcgtccgg ccccatctga tttcttcact acagagttgc tgtcttttgt 3360
   aatgataatt accatgttat ttttgagtgg gattagacat caatttgtga ctctcaccag 3420
   cacgcctaag ggccccacga atcgctggtg tagagcgtgt gtgttttcat aacatgaagg 3480
   gagaagagca gttgcgacag ttgtccctga gcaaacattt gcatccagct gggcacgcgt 3540
   gcctccccag gctagggcag cgggtgctca ccctgccctc ttcctgttca cagcctgatt 3600
   cggactccac caaacactca actccatcga atagctccaa ccccagcggc ccaccgagcc 3660
   ccaactcccc ccacaggagc cagctccccc tcgaaggcct ggagcagccg gcctgtgaca 3720
   cctgaagccg ccagctcgcc acaggggcca gggagctgga gatggcctcc agcgtcagtg 3780
   ccaagactga gcgggccctc cagtgttgtc caaggaaatg tagaatcact ttgtagatat 3840
   ggagatgaag aagacaaatc tttattataa tattgatcag ttttatgccg cattgttcgt 3900
   ggcagtagac cacatctgtt cgtctgcaca gctgtgaggc gatgctgttc catctgcaca 3960
   tgaaggaccc ccatacagcc tgtctcccac ccctgacaac ccgagagggc atatggggcc 4020
   ctgccaacac cacttcctca gcagaaaccc gtcatgacgc ggctgcttcg gaagcagaca 4080
   tctggggaca cagcctcagt acccagtctt ttccctagtt cctgaaactt tcctaggacc 4140
   ttaagagaat agtaggaggt cctatagcat tcccagtgtc actagaattt tgaagacagg 4200
   aaagtggagg ttagtctgtg gccttttttt catttagcca ttgcacagtc agctgcagaa 4260
   gtcctgctga ccacctagtc atggacaaag gcccaggacc agtgacaccc tgcgtccctg 4320
   tgtgcattaa gttcattctg ggtcgcagcc atgaagtgtc accagtatct actactgtga 4380
   agtcagctgt gctgttttcc attcgcttcc acggcttctg cctcctgcca taaaaccagc 4440
   gagtgtcgtg gtgcaggcag gccctgtggc ctgctgggct gagggaagtc agagccccag 4500
   ggcgccacga agtagcccgt atgatcacgt agacccaccc aacacactcc tgcacactgg 4560
   ccccggccca cggcacagca atcccctgcg cgtggatttc acctcaccct ttgtaccaga 4620
   tgttgagtga ccagctctgt ggccctgtgt cgtcagaggc ttgtgattaa ctgtggcggc 4680
   agacacagct tgtccacagc ttgggccagg cttcccctgt cctcccaccg gtcggctgct 4740
   tggcaaggct gttcaggacg tgcacttccc caagtcggca ctgagtggcc cagcaccgcc 4800
   tagccctgcc accccact 4818

<210> 123
<211> 3377
<212> DNA
<213> Homo sapiens

<400> 123
   agcagtagca gcgccgcggg cccttcagag tggccgcagt ccgagctttg ccctcgcatc 60
   ctgttccaac gcgaggctgg tgtgagtggc gggagccatc tgtgggcgcc atggcaaaga 120
   gggaggacag ccctggccca gaggtccagc caatggacaa gcagttcctg gtatgcagca 180
   tctgcctgga tcggtaccag tgccccaagg ttcttccttg cctgcacacc ttctgtgaga 240
   gatgtctcca aaactatatc cctgcccaga tcagtgcagc gttcgaggac ctggagacca 300
   tttgtggggc caaacagaag gtgttgcaaa gccagctgga cacactgcgc cagggtcagg 360
   aacacatcgg cagtagctgc agctttgcag agcaggcact gcgcctgggc tcggccccgg 420
   aggtgttgct ggtgcgcaag cacatgcgag agcggctggc tgcattggcg gcacaggcct 480
   tcccggagcg gccacatgag aatgcacagc tggaactggt ccttgaggtg gacggtctgc 540
   ggcgatcggt gctcaatctg ggcgcactgc tcaccacgag cgccactgca cacgaaacgg 600
   tggccacggg agagggcctg cgccaggcgc tagtgggcca gcctgcctcg ctcactgtca 660
   ctaccaaaga caaggacggg cggttggtgc gcacaggcag cgctgagctg cgtgcagaga 720
   tcaccggccc ggacggcacg cgccttccgg tgccagtggt ggaccacaag aatggcacat 780
   atgagctagt gtacacagcg cgcacggaag gcgagctgct cctctcggtg ctgctctacg 840
   gacagccagt gcgcggcagc cccttccgcg tgcgtgccct gcgtccgggg gacctgccac 900
   cttccccgga cgatgtgaag cgccgtgtca agtcccctgg cggccccggc agccatgtgc 960
   gccagaaggc agtgcgtagg cccagctcca tgtacagcac aggcggcaaa cgaaaggaca 1020
   acccaattga ggatgagctc gtcttccgtg ttggcagtcg tggaagggag aaaggtgaat 1080
   tcaccaattt acaaggtgtg tccgcagcca gcagcggccg catcgtggta gcagacagca 1140
   acaaccagtg tattcaggtt ttctccaatg agggccagtt caagttccgt tttggggtcc 1200
   gaggacgctc acctgggcag ctgcagcgcc ccacaggtgt ggcagtggac accaatggag 1260
   acataattgt ggcagactat gacaaccgtt gggtcagcat cttctcccct gagggcaagt 1320
   tcaagaccaa gattggagct ggccgcctca tgggccccaa gggagtggcc gtagaccgga 1380
   atggacatat cattgtggtc gacaacaagt cttgctgcgt ctttaccttc cagcccaatg 1440
   gcaaactggt tggccgtttt gggggccgtg gggccactga ccgccacttt gcagggcccc 1500
   attttgtggc tgtgagcaac aagaatgaaa ttgtagtaac ggacttccat aaccattcag 1560
   tgaaggtcag tgtcttccct ccctccgtga ccactgtccc aacatccttt cctcttccac 1620
   aagcctcctc tctattttac ctcctccttt tcccctttga gatcattcac tcaaacaata 1680
   cacatttaca catttatgca tggctgcttt gtcccaggca gtatgctaga agctggagat 1740
   acagtgatgc acaaagcaaa tgtattctgt gtcttcttga ttatagtcta gtagggaaga 1800
   gaaaaaggta accaaaaaat tacaatatga tgtaatagat tctgtgatag gaaggtgcag 1860
   ggaactgtgg gaagggatac agaagtgtat gattcaggaa agactatatg aggaggacct 1920
   caagcacaag tgggaattaa atggaaggca gaggaggacg agagtgtttg agacaaaaga 1980
   cacaggaaga gccaagaatg caaggtggga accttaatag tgtgttcgag aaatcacaag 2040
   tagttcagac tcagcttgag gaggggaccg tgaaagctga gagtgaggct tgtgtgcccc 2100
   cttaaggggc ttgctctcct cccaagagct atggggagtc acaggattgc aggcagggga 2160
   gtgatgcagt cctgtgtgtc ttagatcact caggctgagg caagactgag acaggaagat 2220
   caatcttttg cagtgatcca gatgagaaat gatgtgagcc tggaccgtga gaaagacgag 2280
   acagtgtatg catagggtat agacttgaga gagggatttg agatggaatc aagagttgtg 2340
   ttaattcatt ggcctatcaa acagagaaga aattggagtc aagagtgact catggccagc 2400
   tgggtggcac cagtcattga gatagagaac taagagaact gatttcccaa agaaaatgag 2460
   ctgttagatt tgatattccc aatggacatc agcaagagat gtctactagg cagtagggca 2520
   catggatgtg aagcctatga agcagatttg ggctgtagct gtagatctgg gtgggggttc 2580
   tcagccctgg ggcttcaccc tatcctcctc caaagcccta ccctctgcct gaggccaggt 2640
   ccccagtccc tagccccttc cccgccaggg tgtacagtgc cgatggagag ttcctcttca 2700
   agtttggctc ccatggcgag ggcaatgggc agttcaatgc ccccacagga gtagctgtgg 2760
   actccaatgg aaacatcatt gtggctgact ggggcaacag ccgcatccag gtattcgaca 2820
   gctctggctc cttcctgtcc tatatcaaca catctgcaga accactgtat ggtccacagg 2880
   gcctggcact gacctcggac ggccatgtgg tggtggctga tgctggcaac cactgcttta 2940
   aagcctatcg ctacctccag tagctgtaca gaggccctgc ctggcttgtg gagggacaga 3000
   cattggggtg attggacaag agggtctggc tgggaggtgg gccagacctg gcagcactga 3060
   atgtgggctg tgggcatggg tgcacccggt gccctccctc tcctaccccc acccccacgg 3120
   ttgcacttta tttattcggt tcttgctttg gtgactgggt gagcctggac tgtggtccca 3180
   aggatgtgtg cagagcttca ccctaccctt cttacacacc tccccacccc tgtcagtctg 3240
   ctccccatcc cccagcctgg ggccagaaca gcctacccca ggacaggagt ccctctagtt 3300
   gtctccctac caccctatac acactgacag agacagcaat accccacccc ccatattaaa 3360
   taaatgtctt caccaag 3377

<210> 124
<211> 3649
<212> DNA
<213> Homo sapiens

<400> 124
gtctcaagga gagagggatg tgggaacagc tggttaggag agcagtccga acacacactt 60
tttttattta ctttcactgc cttacatggg tatggttcat gctgcctcca aatacttaca 120
aaaacatcaa agatcactga ttacagatca ttaaaatagg tttaataata atggaaacat 180
ttgaaatatt gcaaaaatta ccaaaatgcg acagagacat gaaatgagaa catgctgttg 240
aaaaaaatgg cactcataga cttgttggac acaggattga aagaaaccaa ttcatcaaaa 300
ccacaatatc tgcaaaacgc aataaggtga tgcacaataa aaggaagtat atctgcactt 360
ccttcttttt tactgaaaaa agcccaagtt cttactctgg tgttgagaac ctttcattag 420
ctggctacaa tctcagttca caatcatttc ttattgccct tcaacaatgt cctgaccaaa 480
tcgagttctt tctcattggg aacagttcat tcctttatcc cttcgctcat tctgttcctt 540
ttttcttaga aaaattattt gcccatgttt tttatccccc aattctatct ttttcaatgc 600
ccacttcgtg tccaacccct ctctactttc cctctaaact tactcaaaca cttggtgaac 660
tttttctgaa tgtttttttct gaatgtttac ttttaaagga gttgcccagt cacaaagttt 720
gagccaagtt tttttgtttt aaacttgttt taaacttttg ttttcatcaa ctttttttcaa 780
acaatttttg tcagcttaga ggttgtgttt taattactat ttttaaaatc ttgtatgagg 840
tagtatgatt tgaaattaga tggaatgtgc ccaaaattat ctctgtagaa taatatggaa 900
aaacgaaaat gaaaagatgt attacctgaa tgtgctcctc ctagccagtt tctcaaggga 960
gaagaatcat taataagttt gtgaggcttt tttttttttt tctggtagat ttctgtttcc 1020
ttgatcagtg gtttttcaga attttttaaa gcttgtgaaa tacttatttg taaatagcat 1080
cttatgagga accttgatat gtgacaaaac agatgctttg atttgagagc agaagacctg 1140
cagcccctga ctgctcacct ttccagggac ccctgaacat tggcctagag ggtagtgtac 1200
agtcacttct tcagtgataa cttactgagt gtgattcaac aaaactgctt tggtttatgg 1260
gtgaaacata atttataact aggcaaatgt cagcttaaat ttatattttc taaatagcac 1320
tgaaaatgac aatagacaaa tgtaatttct gatttttaag aaatgatttt ccattttaac 1380
atacatttta aatgttttgt cttcaaaggg aatccttaaa aatgatatat tggaaaccac 1440

tgatttctta ttttcatttc atgaatttca gattcatgaa tggaatatat tgataatagc 1500
atttttttaag ttgccccaaa aagatgaaat acaaataatt ttaatcccaa atctatttca 1560
gacacctaac ttttttttta ttttttatac tttaagttct aggatacacg tgccatgttg 1620
gtttgctgca cccatcaacc cgtcatctac attagatatt tctactaatg ctatccctcc 1680
cctagccccc taccccacaa aaggccccag tgtgtgatgt tcccctccct gtgtccatct 1740
gttctcattg ttcaactccc acttatgagt ggcaacatgt gctgtttggt tttctgttcc 1800
tgtgtgtgtt tgctgagaat gatggtttcc agcttcatcc atatccctga aaaggacatg 1860
aactcatcct tttttatggc tgcatagtat tccatggtgt atatatgcca cattttcttt 1920
atccagtcta tcattgatgg acatttgggt tggttccaag tctttactat tgtgaacagt 1980
gctgcaataa acatatgtgt gtatgtgtct ttacagtaaa atgatttata atcttttggg 2040
tatacaccca gtaatgggat tgctgggtca aatggtattt ctggttctag atccttgagg 2100
aatcaccaca ctgtcttcca caatggttgc aatttacact cccaccaaca gtgtgtaaaa 2160
gcttcctatt tctctacatt ctctccagca tctgttgttt cctgacttaa tgattgccat 2220
tctaactggc gtgagatggt ttctcactgt ggttttgatt tgcatttctg taatgacccg 2280
tgatgatgag ctttttttca tgtttgttgg ctgcataaat gtcttctttt gagaagtgta 2340
aggtacttaa ttcttaaatt aaaaaaaaat acccggccgg gcacggtggc tcacgcctgt 2400
aatcccagca ctttgggagg ccgaggcggg tggatcatga ggtcaagaga tcgagaccat 2460
cctggctaac aaggtgaaac cccgtctcta ctaaaaatac aaaaaattag ccgggcgcgg 2520
tggcgggcgc ctgtagtccc agctactcgg gaggctgagg caggagaatg gcgtgaaccc 2580
gggaagcggg gcttgcagcg agccgagatt gcgccactgc agtccgcagt ccggcctggg 2640
cgacagagcg agactccgtc tcaaaaaaaa aaaaaaaaaa aaaaaaaaat acccttcttt 2700
aacaaaatca agtttcaagt tttctgcctt gttgataact tgttaacatt tttttatttt 2760
agaataaatc taaacagaga ccacactgat atctgttaac aacctatgta aagttaagat 2820
taaggttaaa tcacattttt cacagagagt gttttactga aatataaact tttatgcatc 2880
tgatattatc taattgtgtc cttactgggt aacttgtgta tttttcaagt taaaggattt 2940
cctaaaaatg cttatttgaa tatttatgtc atttgtcaca catactttca aaacataacc 3000
ttcttcaacg agtatcttct tttccttcct aaaatactgt taatagctgg ctattttgta 3060
agcctgtatc tggtttaaaa aaaagaagtg aatattttca atacttatga accatagaat 3120
tctgtattct tttcataact tttaactttt ccataggacc tatcactatg taaaatatta 3180
tacatatcta ctgatttatt tattgcctat gtacctatac tagaacatag taaacatgag 3240
aacagggaat cttggttatt aattcctgca ggactcaact acttatcact caaacatttg 3300
ttcttgaaaa gaacttcgta taacatcatt agatttttac agaatcttta aaaaagcttt 3360
aatattttca aggtttttta tttatataaa tattgttact ttaccactag tgatttcaat 3420
agtggcattg tcacatgatt caagaagagg aggccattac attactgttg tattaaaaag 3480
tcagttcagg cttaatatat atgactacta taagtcaacc tcacatttta atgtatgaag 3540
aagattgtat gaacgatcac attaattcat ttaaatagta tgtattttga tactaatttt 3600

taagaagaaa tgtatcattc tcatggaaat aaaagataat ttgaaaagc 3649

<210> 125
<211> 3527
<212> DNA
<213> Homo sapiens

<400> 125
gtgttttttc acaggtgcta ccaagcgaca gccaacatgt ctcttgcagc tcaggatcag 60
ggtggcagcc tgacagttgc cagggtaggg cttcgccttt ctatggagtt gctcccaccg 120
gcctgactgg gaaaaatctc ccagggatca ggtcactggc ctggtgcccc agggccaggg 180
ctgctgtgtc tcctccccac cctaggctcc atgcatgggt cctgctgcct cgggggaggg 240
cagcccctct ctgtgtttgc atcattgcac atgggccccg aggccctggc actcaaggca 300
ggcaggggat ggtgtccgac cttccagagc tttcctgagc ctactcctag ctacggagcc 360
ccaggcgtgg agttcatggg gctgcaccag gagaacaacg ctgtgacgca gatccacctc 420
ctgcccggcc agtgccagct ggtcaccctg ctggatgaca acagcctgca cctttggagc 480
ctgaaggtca agggcggggc atcggagctg caggaggatg agagcttcac actgcgtgga 540
cccccagggg ctgcccccag tgccacacag atcaccgtgg tcctgccaca ttcctcctgc 600
gagctgctct acctgggcac cgagagtggc aacgtgtttg tggtgcagct gccagctttt 660
cgtgcgctgg aggaccggac catcagctcg gacgcggtgc tgcagcggtt gccagaggag 720
gcccgccacc ggcgtgtgtt cgagatggtg gaggcactgc aggagcaccc tcgagacccc 780
aaccagatcc tgatcggcta cagccgaggc ctcgttgtca tctgggacct acagggcagc 840
cgcgtgctct accacttcct cagcagccag caactggaga acatctggtg gcagcgggac 900
ggccgcctgc tcgtcagctg tcactctgac ggcagctact gccagtggcc cgtgtccagc 960
gaagcccagc aaccagagcc cctccgcagc ctcgtgcctt acggtccctt tccttgcaaa 1020
gcgattacca gaatcctctg gctgaccact aggcaggggt tgcccttcac catcttccag 1080
ggtggcatgc cacgggccag ctacggggac cgccactgca tctcagtgat ccacgatggc 1140
cagcagacgg ccttcgactt cacctcccgt gtcatcggct tcactgtcct cacagaggca 1200
gaccctgcag ccacctttga cgacccctat gccctggtgg tgctggctga ggaggagctg 1260
gtggtgattg acctgcagac agcaggctgg ccaccggtcc agctgcccta cctggcttct 1320
ctgcactgtt ccgccatcac ctgctctcac cacgtctcca acatcccgct gaagctgtgg 1380
gagcggatca ttgccgccgg cagccggcag aacgcacact tctccaccat ggagtggcca 1440
attgatggtg gcaccagcct gaccccagcc ccaccccaga gggacctgct gctcacaggg 1500
cacgaggacg gcacggtgcg gttctgggat gcctcgggtg tctgcctgcg gctgctctac 1560
aaactcagca ctgtgcgcgt gttcctcacc gacacggacc ccaacgagaa cttcagtgcc 1620

cagggcgagg acgagtggcc cccactccgc aaggtgggct cctttgaccc ctacagtgat 1680
gacccccggc tgggcatcca gaagatcttc ctctgcaagt acagcggcta cctggctgtg 1740
gcaggcacgg cagggcaggt gctggtactg gaactgaatg acgaggcagc ggagcaggct 1800
gtggagcagg tggaggccga cctgctgcag gaccaagagg gctaccgctg gaaggggcac 1860
gagcgcctgg cagcccgctc agggcccgtg cgctttgagc ctggctttca gcccttcgtg 1920
ttggtgcagt gtcagccccc ggctgtggtc acctccttgg ccctgcactc tgagtggcgg 1980
ctcgtggcct tcggcaccag ccatggcttt ggcctctttg accaccagca gcggcggcag 2040
gtctttgtta agtgcacact gcaccccagt gaccagctgg ccttggaggg cccactctcc 2100
cgcgtcaagt ccctcaagaa gtccttgcgt cagtcattcc gccggatgcg tcggagccgg 2160
gtgtccagcc ggaagcggca cccggctggc cccccaggag aggcacagga ggggagtgcc 2220
aaggctgagc ggccaggcct ccagaacatg gagctggcgc ctgtgcagcg caagatcgag 2280
gctcgctcgg cagaggactc cttcacaggc ttcgtccgga ccctgtactt tgctgacacc 2340
tacctgaagg acagctcccg gcactgcccc tcgctgtggg ctggcaccaa tgggggcacc 2400
atctatgcct tctccctgcg tgtgcctccc gccgagcgga gaatggatga gcctgtgcgg 2460
gcagagcagg ccaaggagat ccagctgatg caccgggcgc cggtggtggg catcctggtg 2520
ctcgacggac acagcgtacc ccttcccgag cccctcgaag tggcccatga tctgtcgaag 2580
agccctgaca tgcagggaag ccaccagctg ctcgtcgtat cagaggagca gttcaaggtg 2640
ttcacgctgc ccaaggtgag tgccaagctg aagttgaagc tgacggccct ggagggctca 2700
agagtgcggc gggtcagcgt ggcccacttc ggcagtcgtc gagccgagga ctacggggag 2760
caccacctgg cagtccttac caacctgggc gacatccagg tggtctcgct gcccctgctc 2820
aagccccagg tgcgctacag ctgcatccgc cgggaggacg tcagtggcat cgcctcctgc 2880
gtcttcacca aatatggcca aggcttctac ctgatctcac cctcggagtt tgagcgcttc 2940
tctctctcca ccaagtggct ggtggagccc cggtgtctgg tggattcagc agaaaccaag 3000
aaccaccgcc ctggtaacgg tgcgggcccc aagaaggccc cgagccgagc caggaactca 3060
gggactcaga gtgatggcga ggagaagcag cccggcctgg tgatggagcg cgctctgctc 3120
agtgatgaga gagcggcaac tggcgttcac atcgagccgc cgtggggtgc agcctcagca 3180
atggcggagc agagtgagtg gctgagcgtc caggctgcgc gatgagcaca cactactact 3240
gatggccttt cgggggtccc tgccccaacc ggagaggccg gtgcacaggg ccccgccagg 3300
ggctgggggc atcccggctt ccacaatgca gctgctctgg gcctcgggag aggagagacc 3360
ccagtcccct gggctgccct tcccgggcct cgtctgtctg ggtcctttgg tcaatgttgc 3420
acagttttta ttgctcccat ccctttttgt agtgggctgg gttttaagtt ataaatgtta 3480
actgcctctg ggtgaaaaag tttttaataa acacctatta cctcttg 3527

<210> 126
<211> 3574

<212> DNA
<213> Homo sapiens

<400> 126
   tttttcttat ctgggtactt ttcagaattt atctttgtct ttgcttttat gaagttttat 60
   tatggtgttt ctaggtacca tttaatttat tctgcttggg attttgtgac tcgatgttct 120
   tttaaggaaa ttcttggtaa ttgttttttt cacagagtgc ttttactctg ttctttcttt 180
   attcctcttc taggacacca gtcagacaga cacagacttt cccaccaggt ctccatgcct 240
   cttatgttct ttttcatatt tatctgcggt tttctccttg ttgctgtctg gatgttgtct 300
   tctaacctat cattcatttt actaattcta tttgtgtttt atatgctgtt aaacttatct 360
   attgagttct taattccagt tactatgttt ttttggtttt agattaattt cgctttgcta 420
   aaattgtcca catggttatc tatttcacag ttattttaaa ggttatattt gacaattcta 480
   gtatttatat cttctgtgga tttattttga cacttggtcc tgcttcttgg tgtgcctggt 540
   aatttacggt tgaatttcag gccatgggtg gttgtttcct tctacaaaga gtattcactc 600
   agcccatctc ctgagaaaat ttatcctgtc ctccagtagg caaagaagag gcagatcact 660
   ttcagttcaa tcagagacgg aagccggttt tcagtctttg taaggttcag ctatctctga 720
   ttatccctct cttcctaagt gaaagacttg agtatttact tttgcctggg caggtcctga 780
   gttccaaaat tcatttcctc agcactttga accttctgaa aagtaagctc agctctcctg 840
   cttctttctg ctcaacactt gagcctatta tccttctttg actaagaatc attgagcact 900
   ttgaaggaaa aacacgtgca tagtgtctta gttctctgtt tctcttctgt cctagctttt 960
   ccatcctcaa gtcttgtttg ccttgatagg ctcttgctac aacttttgtc ttctcagctc 1020
   tgtaaaactg ttgaaagctc tccttagctg ccacgtgctg cttgcctttc tgtttatgtt 1080
   tctgccaggc ttttcagtat ctacccctat actgctcaag agctggcaaa taacccaagg 1140
   gagaagacca gaatgctaag tgtcaggtta cctccatgaa gtccttcctc cggatctttg 1200
   gcacttcaag ttctggctgc caaggcagct cttgaatgtc ttagaacaga tgaagtaact 1260
   taccagcttt tctcctcctc ttggcaggga cattggtttc ctaaaagttg ctcagttttg 1320
   gccggaagtg gaggtctaaa gtttttggtt ttgttttcct gaattatctg tgttgatttt 1380
   ggaatcagag tttggcaagc cacaaaatag attgatagat tcctatattt ttctataatg 1440
   taaaatgagt tgagcagctt ttacattatc ttttgaaagt ttgaagtaac tttttttttt 1500
   aagatggaat ctcactctgt cgcccaggct ggagtgcagt ggtgcgatct tggctcactg 1560
   caggctccac ctcccgggtt cacgccattc tcctgcctca gcctccccag tagctgggag 1620
   tgcaggcccc caccaccacg cctggctagt tttttgtatt tttttagtag agacggggtt 1680
   tcactgttta gccacgatgg tctcgatctc ctgagctcgt gatccgcctg cctcggcctc 1740
   ccaaagtgct gggattacgg gcgtgagcca ctgcgcccag cctgaaatag tttattaata 1800
   aaatcatttg gacatagtac tctctctccc ttcatagttg ttctttggca actttttcta 1860
   atttctgatt attagcttag tcagattttt ctccctcctc tctaataatt ttgataatat 1920
   atttttgggt gggttgtttt aatgcaatca ttaatttctt ctgtatttgc caagtttatt 1980
   tgttgtggag aatacataat attttaagat ctctttgtgt ttatagcctc tttttcacag 2040
   ataatgtttt taagtttttt atttctctct ctgggtttgt tttttaatca gtgcttttaa 2100
   agaactgatt tttttttttt tttggatcca gtctactgtt tttatttttt atttctgtta 2160
   attattttgt gcttttaaaa ttaatcattt tatcctactt ggaacaagat gatatttgtg 2220
   ggtgtgtttt ttgctttttg tactttgtgc tttacatatt tttcttcttt ttaacttttt 2280
   ttaaaatata tttttattat attttaagtt ctagggtaca tgtgcaggtt tgttacatat 2340
   gtatacatgt gccatgttgg tgtgctgcac ccattaactt gtcatttaca ttaggtatat 2400
   atcctaatgt tgtccctccc ccatcccccc ccccccaaaa aaaagccatc cccccgctct 2460
   gccccgtcgc acattcggcc cccgcgactc ggccagagcg gcgctggcag aggagtgtcc 2520
   ggcaggaggg ccaacgcccg ctgttcggtt tgcgacacgc agcagggagg tgggcggcag 2580
   cgtcgccggc ttccagacac caatgggaat cccaatgggg aagtcgatgc tggtgcttct 2640
   caccttcttg gccttcgcct cgtgctgcat tgctgcttac cgccccagtg agaccctgtg 2700
   cggcggggag ctggtggaca ccctccagtt cgtctgtggg gaccgcggct tctacttcag 2760
   caggcccgca agccgtgtga gccgtcgcag ccgtggcatc gttgaggagt gctgtttccg 2820
   cagctgtgac ctggccctcc tggagacgta ctgtgctacc cccgccaagt ccgagaggga 2880
   cgtgtcgacc cctccgaccg tgcttccgga caacttcccc agataccccg tgggcaagtt 2940
   cttccaatat gacacctgga agcagtccac ccagcgcctg cgcaggggcc tgcctgccct 3000
   cctgcgtgcc cgccggggtc acgtgctcgc caaggagctc gaggcgttca gggaggccaa 3060
   acgtcaccgt cccctgattg ctctacccac ccaagacccc gcccacgggg gcgccccccc 3120
   agagatggcc agcaatcgga agtgagcaaa actgccgcaa gtctgcagcc cggcgccacc 3180
   atcctgcagc ctcctcctga ccacggacgt ttccatcagg ttccatcccg aaaatctctc 3240
   ggttccacgt ccccctgggg cttctcctga cccagtcccc gtgccccgcc tccccgaaac 3300
   aggctactct cctcggcccc ctccatcggg ctgaggaagc acagcagcat cttcaaacat 3360
   gtacaaaatc gattggcttt aaacaccctt cacataccct ccccccaaat tatccccaat 3420
   tatccccaca cataaaaaat caaaacatta aactaacccc cttccccccc ccccacaaca 3480
   accctcttaa aactaattgg ctttttagaa acaccccaca aaagctcaga aattggcttt 3540
   aaaaaaaaca accaccaaaa aaaatcaatt ggcc 3574

<210> 127
<211> 5126
<212> DNA
<213> Homo sapiens

<400> 127

attaccacag ttgagcacca aatgttgata taagccttct agaggcaaaa cagaaagcat 60
aatgcataat gtgtcaggtt acagagggct tttttttttt tttttaaatc aaaagagaag 120
ttgggctttt cttagtggct gttgtatttc cagtaccagg taattccatg tatttcttaa 180
ggataaggct ctacctccct cttgtcatcc ctgtccccat cccctagagt accatgagct 240
ctgcacaaaa cctatagtaa gatctcagag ctagggcctc cagccggtga caggaaacag 300
agcctttcag tgtcttctga aagttgagct tactgatgtt gggtgcaggc tgacagaggc 360
cattcaggct ggtctagacg ttcatcctgg agatgcgccc agcaccagcc gaacttctgg 420
tgggaagtgg aatgaaatgg acaggggtgg tcctttggag aatcttatta tccgatggag 480
acgaagcata cctttgggaa ctgggaacct ctttctacag agagtctggg ttaggggcct 540
tgttgcacct gcttgctatc ctaagtgcag gcaaatgaga aatgttttaa tttcccccta 600
ggaatgagaa aatgcattat gttactctga atatccatag cctcaacaga taccgactta 660
tggaagagaa tgttgagact gtcaggacta aatacttaaa gcaaaatcag gaaatcggag 720
gttgtgctgt tcatgtgcca ctatccacat gtgtcttttg agctctcaaa atgtggctaa 780
gtccaaattg agatgggctg aaagtgtaaa atatacacca gattgcaaac actattgtgt 840
gaaaaaaaaa aatctcagta gtaatattgt aatgtaaacg atctcaacat gtaataccag 900
ttacatgttg aaatactatt taggctatat aaaatatatt acaattaatc ttacctattt 960
cttttcactt tttaatgtag ccactagaga atttaaaatt aaatatgtgg cttggccggg 1020
cacggtggct tatacctgta atcccagcac tttgggaggc cgaggtgggt ggatcacctg 1080
aggtcaggaa tttgagacca gcctggacaa catggtgaaa caccgtctct actaaaaata 1140
caaaaattag ccgggtgtgg tggcaaaccc ttgtaatccc agctacttgg gaggctaagg 1200
cagaagaatc acttgaaccc gagaggcaga ggttgcagtg agtcgatatc gcgccactgc 1260
accccagcct gggtgacaag agtgaaactc catctcaaaa acaaacaaac aaaaagtatg 1320
tggcttgtgt tatatttgta tctgacagct ctggtctaaa catctagaat taaacaaaac 1380
ttttttgagg agtaaatttc tactgtgttt tctaattcct atgttctctc tttggttctc 1440
tttcaacaga tatgactcca cgtagcatgt caaggactac attaatcacc aattccttta 1500
tttttccccc cctacagttt ccattttttt ttatacttgc ttactccagc catctgcagt 1560
acaccagttt caggtctttt gagctgtgta gagtttctgt gtgtacagat gtgtgctcgg 1620
acttttctct ttttgagaaa tctgaaggag atggttgcag aagatccact tactactgag 1680
aaccattacc accgactcgg cctccggggt gttgggtggt ttctgggtgg ctcctggagc 1740
ctcctctggg cagtgcactg tcccatctgt acgccctaat gtgccattcc ctagagggga 1800
acaaccaagt gccgtggagg cagatgatca tggtctgcct caactgtctg gtttcctgta 1860
aaataaacac attgttttat atttttaggg aacaaaaagt gctgctatag ggttcaaagt 1920
tttccttctg aacacttttc cgaaacaaat taccccaaag acacattttg aatatcctgg 1980
tcacatcttt ggatctgtaa aatatacctt ttagtatggc acctgttaaa atgcaaagca 2040
aatttctttg gggcagaaaa acaatctgac agtagcagtg tagaatttgt tcattcaaat 2100
acatctgtgt aaatgcaaaa agtcataaaa ttcacctccg agctgcttgc ttttgaacct 2160

gcagcaacta gtcttagccg gcccggtttg aacatcgttc tttcagaagt gctgaaaatg 2220
ctgcaaagtt ggataagtgg aaatgtggct gcccctctcc tcactacttc ctctctgatc 2280
gttctgaagc ttgcattggg aatggctgct ttctctaacc attttcagct tgagtgggta 2340
ttgctgaaga aatccaacat cattccagca gttgaaaaag gaagccttcg ggagaaagtg 2400
cttgtcaaaa ttttgttctt tgtgcttgtg tatgagtaag ttgccatgaa taagttatta 2460
ttttaaccca taattggcga ctgtttatat gaattctttc tttggcacca aataggtttc 2520
atcttcttag gcacaattag aaaaaatcca catagatgga tattttacat ttagttattg 2580
ctttatccaa atacatgaat ctaaagctga atcaaccctt acttccagtt gtgcttatta 2640
agaagatcaa tttccaagta gtaaagtttt cagggaaact gactgtgctg ctatttgttt 2700
tgacaaattt gggggtaagt caatgacaac caaaccaatc tcggtggaaa ctcctatcct 2760
atcatgttgt gtgcccaaga tgagtgagct ggcactgtgc cctgaagctt tcaccactgt 2820
aatgaaatat atgccagggg agactttggg cttttctcat gactgtgtgg gtcgaaggta 2880
gctcaagtgt gtgtgtgtgt gtgtgtgtgt gtgtgtgtgt gtgtgtgtat gtgtgtaaag 2940
tgctaagaac tgtgcattga catccaaaca tttcttgtac aaaatttccc tagcaaagca 3000
aacctgcttt gacttaattt atttgttaaa tgttgcactt tgtttatgta tgttttgttt 3060
ttggtgggga ataaggagag agaggacgac aaattctatt gaagtattta ttttgtgaag 3120
atggcaattt tgcatttgtt taaatttttt tcattcttta attttgttat cagtgccagc 3180
ccaatatacc tgctctacca ttatttgcgg tctgataaaa gggtccttgt ggggcaggtt 3240
ttgcaaagct tatcaggtaa taacatatgc cacataacct tgttgatatg tttgcttctg 3300
atttgggaag ctaaacattg gtgtttgaga ggattgccaa ttattaattg tcattaccac 3360
tactctccat tactttttgt ttggaaattg aacaaaggtc agtaatggtt tttggctctt 3420
gttaatatcc atcataaaat agattgtttt agattctttc cagggtgatt tttccctggg 3480
taccccgttt caacttctaa agaattgctt ggcactttca tgtttcaaag ggaaacattc 3540
gcttgtagtt ccattttact tgatctctac aagggactga caacatttgc tttactttta 3600
ttcacagaga aagttggctt tgatgtctct taaagataat tctgctagtt gctgatcagc 3660
cagtcagttc acctagcttc aatctttata ggacttctaa tctaattttc ctatagtgtg 3720
actaaaaggg aggcaaatta ttggaacgga ttattcaaat ggatccttaa atattgctat 3780
gtataataag ccagttatta tatcaggacc atgttctctg taggccactt tctaaaaaag 3840
ccacatatgt gcaattttca ggtttttaga ctattgctcc ctgtacttta aatgtaaaaa 3900
ccacacttct gaacaactaa gctcatgaat atgattttgg ttatatgcag cttttgacta 3960
gcatgtattg tgtctttttc tcctctatga ataattttat atttcatgct acttcttgaa 4020
agtttactct ttgatgctct aagagaacag ccagatggtt tatatgaata atctttatct 4080
gcaggatggt ggattggtaa attaggagaa tgttgtttga gatatcaaga tttatgtctg 4140
ggaactaaaa tatataatgc caaatgtgtt tttgtcaatt actagagaat tctgtgcaaa 4200
catatcatct cttcaaatgc tgcacacttt gcttttgtta aacagcaggt agtagacaga 4260
acaataacag tttcgcgtta agacttttaa aggaaataga atcgtgatta agaaatcaga 4320

atttatagat atattgggat aaatgaagaa ataaaaatgt ttgtctagaa tgtagcatct 4380
agtgactttt taaagcccta acgtttacat aaagaagctc tagttcttat agaaataaca 4440
aagcaaataa aagttcttaa caatcccctc tttcgaagtg cattttttta aagcagggca 4500
ggagacattt ggactctagc tatatgacat actgggaaag gcagagggtg gagggaagat 4560
ttcacttcat tgtctagccc agaatcttga gcaagctaaa gaaaccatca taatctaaaa 4620
ttgcttcatt taacactaac aatttagact ttttaaacca agcattgaat aatggctgga 4680
taactgccga agtaagcgcc gctccatgaa gtctgcttac ttatttaaaa attgtgtatc 4740
agttttaaat actgttcatt gtgtgcagat ataaggggaa tagggcattc tgtagaatta 4800
tacatgtcta gtttgtaaag tgtgtcctgt gtactgcaga tgtgtgttct ctgggcttta 4860
tgtatctgta cagtagcttt cacattaaaa aaattgtgga caaacttgtc cggggggttt 4920
gaggggagaa tggtggttta tatcaataac gatgctgtac tatagtccat gtaacaaaag 4980
atctggaagt caccctcctc tggcccacgg aaaattttgg taatcttcta ggttctaaaa 5040
tgaagatgta tgggtactct ggcagactgc atgttgtata atttgaaaaa tactaaaagt 5100
ggaagataaa attgaattaa actttg 5126

<210> 128
<211> 3677
<212> DNA
<213> Homo sapiens

<400> 128
cctcatccgc ttctgctgct gccggccccc cgagccccac gggtccaaga tcccctcgcc 60
cgggggaggc tgcgtcacct ggagctgcat tgtcgccctt ctcgccggct ggtaatgggg 120
ccccagggtg ggtgggcggt ggggacaggg ctccccaagc tctttgctgg ccttcctggg 180
ggtgtcctcc ggggacatgg aggaagcaga caggaaggag gaaactccct cgtccctgtc 240
cctgccattt gcaagcccac ttcagcgcac agcaggaagg actgtcccat ttgatggatg 300
gagaaagtga ggctctggag aggaagtgaa tcaagggcag ccatctgcac tggcattggc 360
atcggtttct atggcaacag tgagaccagt gatggggtgt cccagctcag ctctgcgctg 420
ctgcacgcca accacacact cagcaccatt gaccacctga cggtggagag gctgggcgag 480
gcggtgagga cagagctgac caccctggag gaggtgctcg agccgcgcac ggagctggtg 540
gctgccgccc gaggggctcg acggcaggcg gaggctgcgg cccagcagct gcaggggctg 600
gccttctggc agggggtgcc cctgagcccc ctgcaggtgg ctgaaaatgt gtcctttgtg 660
gaggagtaca ggtggctggc ctacgtcctc ctgctgctcc tggagctgct ggtctgcctc 720
ttcaccctcc tgggcctggc gaagcagagc aagtggctgg tgatcgtgat gacagtcatg 780
agtctcctgg ttctcgtcct gagctggggc tccatgggcc tggaggcagc cacggccgtg 840

ggcctcagtg acttctgctc caatccagac ccttatgttc tgaacctgac ccaggaggag 900
acagggctca gctcagacat cctgagctat tatctcctct gcaaccgggc cgtctccaac 960
cccttccaac agaggctgac tctgtcccag cgagctctgg ccaacatcca ctcccagctg 1020
ctgggcctgg agcgagaagc tgtgcctcag ttcccttcag cgcagaagcc tctgctgtcc 1080
ttggaggaga ctctgaatgt gacagaagga aatttccacc agttggtggc actgctacac 1140
tgccgcagcc tgcacaaggt gaagcccctc ccctcccaat ttcttctccc acggggggcc 1200
tctgtctcga cccacagaac tacctcctcc ttctccttgg acccctgcca ttgcgcctga 1260
ggatatctct gtatcctctg tttatatgat ttatctgtcc tatatctatt ctctacctat 1320
ttataacctg tcgtctacct acctatcaag cattatccat attccttccc tcctccctcc 1380
ctttcccccc aactcccgca ctccccgctg ggtccccatc ccaccctccc cgtccattcc 1440
tgccaccttt tccttccttg tcttctctcc tctctccgtc ctcctgcctc tccctccctc 1500
ctagaggctg ccgcttagtg agttctggag caagactgcc tgggttccag tcctacctcc 1560
tgaccaaggg caagtcacct aacttctctg tacctcagtt agttccctca cttataaacc 1620
tgggattgca agagtgggca cctgtccagc tcccctgcgg cttgtgctgt tcatacactg 1680
gacaggcagg ggtgggcagg gtgcccagga ggaaagatat ctggtgtcct gcaggctcca 1740
gtttgggctc tgccgcaggc tgcacggcca taggcaggtg agcgtggctg actctgcttc 1800
ccacccgtac cgtgaggaag gacgagtttt ttttgtttgt tttttttca ctggatggct 1860
gtgaggattg aaaaaaaaaa tccccttata aaacagtagg agctggccgg gcgcggaggc 1920
tcacacctgt aatcccagca ctttgggagg cccaggcggg tggatcacct gaggtcagga 1980
gttcaagacc agtctggcca acatggtgat accccgtctc tactaaaaat agaaaaagtt 2040
agccgggcat ggtggtgggc gcctataatc ccaactactc gggaggctga ggcaggagaa 2100
tcgcttgaat ccacgaggca gaggttgcag tgagccaaga ttgtgccact gcactccagc 2160
ctgggcaaca agagcgaaac tctgtcacaa acaaacaaac aaaaccagta agagctgttc 2220
taaaacacac acccgaggat gcctttcccc ggccagtctt cccatcaaag acctcacatg 2280
tgcatagctt caccaagtac ctgaccctgt tgcatttcac aggtggtaac tcatcgatgc 2340
ctcataacaa tgctatgaag aaggaactat gattatccca cctaacaggt tagaagatga 2400
ggagcagagt tgagggacct tcccaaggtc acacggccag ccagcccgtg cacactgagg 2460
agagagggac cttccggagg gccccgccct ctgcccccca cagccaggca gttgcccagt 2520
tttgtccttt ctgcttctgg aatgtcttca tccatctact ctcaatgccc aatgcccctg 2580
ctgcagctga ggcctcgttc tctcccctgc agaaggaagc atggccccct ccccatccgt 2640
ccccccatgg accctagaat agggctgcca gattcagcaa acagcaaacc actctgctat 2700
aagtacgccc ggtgcaatgc tggggagata cttatactag aaaattatgt attgggcatc 2760
cgatactcaa atgtaactgg aagtcctgaa tttgatctgg caaccctacc caagagggat 2820
ttgtctacgc catcgcatgg atctgagctt tccctggtct ggctcagaca gaccccttct 2880
gtggctccca ctgcctccca cccttctgga actggcattc caaaaaggga gtggggagta 2940
agcactgaag ccatgctcag aattgctgac tattgagctg agaaaggctg ggcccccacc 3000

cagcccaggg ctgccctgat cttttgcata aggataaact ggggttcagg cgcttttctg 3060
tggcctaggc agaaactggg ctggaataca ggcccccaga ctcctttgtg gagggaggga 3120
agggaaggaa gaggacagat cactctaccc ttccctacca ctgcaccatg agaccctggg 3180
tctccatctc ccctgtcagt gtgccagggt cctcacagat taaaatcaag aataagcggc 3240
ggggccgggc actgtggctc acgcctgtaa tcccagcact ttgggaaacc aaggtgggcg 3300
gatcaccaga ggtcaggagt ttgagaccag cctggccagc atggcgaaat cccatctcta 3360
ctaaaaatac aaaaaaacta gccgggcgtg gtggcgcacc cctgtattcc tagctactca 3420
ggaggctgag gcacgagaat tgcttgaacc tgggcagtgg aggttgcagt gagctgagat 3480
cgagccactg cactccagcc ggggtgacag agcgagaatc cgtctcaaaa aaaaaaaaaa 3540
aataggaggg ggctagggat gggtgcctcg tgcctatcaa cccagcactt tgggaggccc 3600
agtggggcag atcagaactc cttgagctca ggagttcaag accagcctgg ccaacatggt 3660
gaaaccccat ctctatt 3677

<210> 129
<211> 3734
<212> DNA
<213> Homo sapiens

<400> 129
tttgtatttt tagtagagac agggtcttgc catgttagct gggctggttt tgaactcctg 60
acctcagatg agccaccacg cccagcctca agctctcgtt tctgtcctcc cgtcaccttt 120
gaggaagtaa gtggtggttt tatgccatga gagctgtggg gtttcaggta ccgtgatgca 180
aaaacagcca caaaattagc agttcttgta cattgcagtt gctatccttc aaggataagc 240
tcttttccag tttccgcctg ctttggttgc tttccagtgc tattagttgt ttattgtatg 300
gcatgtgcac attttataat tattatctga gggaaagtgt ccctgactgt tttactctgt 360
taatgctgca tgctgaaaaa tctcaccatg cacatacaat attcctcctt agtgaagcct 420
gagaaattgt tctacctttg tgatacatgc tgtatcttgt acatacttct ttgttgtacc 480
tgtgacacct tcttgcaggt ttgtgtttac ttacattttc ttgcaagtat gtgttacatg 540
cctctggtaa cttctgagca gaaacagtgt ttgtcttggc acttccataa ataaatacag 600
tttttggcat taagtggctt cacagtaaat attcttagaa tgtgttattt aaaaactaac 660
tgatggatat ttttaccttg atttttgttt tccctacaat ataaatcata aaacagggaa 720
aattgtagtc tttctatatt aggattcgta tgactgccac aaggaagcag ggtcaaaagt 780
agttcccaat atccagtcca cctgttcttt ttcttttcct tttttttgac agagtcctgc 840
tctgtcgccc aggctggagt gcagtggcat gctctgggct cactgcgtcc tccgcctcct 900
ggtttcaagc tattctcctg cctcagcctc ccatgtaact gggattacag gcgcacacca 960

ctatgcccag ctaatttttt gtatttttag tagacatggg gtttcaccat cttggccagg 1020
gtggttttta actcatgacc tcaagtgatc cacctacctc agcctcccaa agtgctggga 1080
ttacaggcat gaaccactgt acctggccaa gtccacctgt tctgattaca gttccatcca 1140
ttctagggat cagtgtcttt caatgatgtg actgtggact tcactcagga ggagtggcag 1200
cacctggatc atgctcagaa gactctatat atggatgtga tgttggaaaa ctattgccac 1260
ctcatctctg tggggtgtca catgaccaaa cctgatgtga tcctcaagtt ggaacgagga 1320
gaagagccat ggacatcatt tgcaggtcat acctgcttgg aagaaaactg gaaagctgaa 1380
gactttttag tgaaattcaa ggaacaccaa gagaagtatt ctagatcagt tgtaagcatc 1440
aaccacaaaa aactggtgaa ggagaagagt aaaatatatg aaaagacatt tactctaggc 1500
aaaaaccctg tgaattcaaa aaatctacct cctgaatatg atactcatgg aaggattttg 1560
aaaaatgttt cagaattaat catcagtaat ctaaatcctg caagaaagag acttagtgag 1620
tataatggat atgggaaatc actcctgagt actaaacaag agactactca tcctgaagtc 1680
aaatcccata atcaaagtgc cagagctttc agtcataatg aagttcttat gcagtatcag 1740
aaaacggaaa ctccagcaca gtcatttgga tataatgact gtgagaaatc attccttcaa 1800
aggggaggcc tgattacaca tagtagacct tacaaaggag aaaacccatc tgtatataat 1860
aaaaaaagaa gagcaaccaa tattgaaaaa aaacatacat gcaatgaatg tgggaaatct 1920
ttctgcagga aatcagtatt gattctgcat cagggaattc actcagaaga aaaaccctat 1980
caatgtcatc aatgtggaaa tgcatttaga aggaaatcat atctcattga tcatcagaga 2040
actcacacag gagagaaacc ctttgtttgc aatgaatgtg gtaagtcctt ccgcctcaag 2100
acagccctca ctgatcatca gagaacacac acaggggaga aatcgtatga atgtctgcaa 2160
tgtaggaatg ccttcagatt gaagtcacac ctcattcgtc atcagagaac tcacacggga 2220
gagaaaccat atgagtgtaa tgactgtggg aagtccttcc gccagaagac aacactctct 2280
ctacatcaga gaatccatac aggtgagaaa ccctatattt gtaaagaatg tgggaagtcc 2340
tttcaccaga aggcaaatct tactgtacat cagagaactc atacagggga aaagccctat 2400
atttgtaatg aatgtgggaa atccttctcc cagaagacaa cccttgctct tcatgagaaa 2460
actcataatg aggagaaacc ctatatttgt agtgaatgtg gaaagtcctt ccgccagaag 2520
acaacccttg tagcacatca gagaacacat acaggggaga aatcttatga atgtcctcac 2580
tgtgggaagg cctttagaat gaagtcatac ctcattgatc atcaccgaac tcacacagga 2640
gagaaaccat atgaatgtaa tgaatgtggt aaatcattca gtcaaaagac aaatctcaat 2700
ctacatcaga gaattcatac aggggagaaa ccctatgttt gtaatgaatg tgggaagtcc 2760
tttcgccaga aagcaaccct cactgtacat cagaaaatac ataccggcca gaaatcctat 2820
gaatgtcctc agtgtgggaa agcctttagc aggaagtcat atctcattca tcatcaaaga 2880
actcatacgg gagagaaacc atataaatgt agtgaatgtg gaaagtgctt ccgccagaag 2940
acaaatctta ttgtacatca gagaactcac acaggtgaga aaccctatgt ttgtaatgag 3000
tgtggtaagt ctttcagtta taagagaaac ctcattgtcc atcaaagaac tcacaaggga 3060
gaaaacattg aaatgcaata aatgatgtgg tttcttatat gaattcttta caagctgttg 3120

taaacattta gttttaaaaa gaaaagcatg ctgaaacatg ttaatgtaat tttaaatcac 3180
aagtctaata attattaaag taccatacgg aataactgtc tactgtttac tagcatataa 3240
aataagtatg atcattatta ttgaactcta tcagctatga agctaaattt taaagtcaac 3300
tgctcttcct actgactcaa atagtttatt ttttaaaaat acttatataa tacatgcaga 3360
gacaagatac acaatgatta taagtattaa tctccataag agaaaatatt tatgaactat 3420
atttctcatt gcactctgta ataaaaagca gttagttgtt acttacctaa gagttaccac 3480
ttccgtagcc tataacatca aaacgtagtt tttgcatgtt ttcaatttta taaatatata 3540
ttttatcaga catcatgaat tattttgtgt cttgcttatt tcactcaatt ttttaagatc 3600
tgtacattat tgcatgtggc agtagtgtat ttttcatttg catactattc cattttaaga 3660
atatattgct gtttatccta ttgatggata tttgtatttt ttatactttt gtgtaataat 3720
aaaaatactg ctgt 3734

<210> 130
<211> 4977
<212> DNA
<213> Homo sapiens

<400> 130
catgtggttt ttatgaggtt cacaggcccc cagacagcca ggacctaaaa gtggttttta 60
atggcagtaa tgcccacatg tggcaatagc ctgtcatctt catcttcatc tcccaatcag 120
gaaactgagg caccgagagg aaggtgccct tcaaggtggc gggaacagtc tcccccaggc 180
ccccaccatg cggactcccc agcctcctcc ccttgccgtg ctcccgagcc tgacctcagc 240
cccgacaccg ccaccctctc tattgtgccg tccccatcct gcccgcctcc cctttctagg 300
aatccctggg gtgcggagtc ttcatcccac gctgccccgt ccctgtgtcc accatgccca 360
ccccaccttc tgagccacct ggttggggtg tcctctgccg aggcttgggt tagttacagg 420
ggcccgcggg caggttctcc ctcctcccca gcctcagttt ccacctccgt ggagtgggtc 480
ctggggcagt catgagatgg agcgtgttct gtgcccggct ggcctggcag gggcatgcat 540
gcacaggggt gggcacagct ccttgtgtgg tcctggtgcc ccaagctctg tgcccacctg 600
cagcttgggc ctgggcgatc agcgccgtct cactctgtca cctgggaaat ggcttcttgt 660
cctgcatcac ttgatcatcc ctgaggggac agcaggtgga ttggttctat cctaaacctg 720
gcgaggcccc ggcctctaga caagcctccc tgtgacaaca ggtctaccag ggcctggact 780
cagcccctgt gccagagggc agcttggtga aattcccagt tgctgagcgc acgctgggga 840
gctgcctcgc agcacccagg gcagacatgg gtggatttgt gggtgcggag gagccctcct 900
tacctgccca ggagcccagg gactctcaca ctgatgagtc tgcgttccgg ggaaggttgc 960
gtttctctgg cagtgagcaa cttgacaggt ggtgtctgtg gctccctaga taggctgggg 1020

acctcgggcc cggatcagcc cggctgcttc atccctggga gcacagacct gattgtggag 1080
cctggaagct ccaagctcct tgaacttcat ccctgtggcc tggacgggga gcggaaaaga 1140
gtcacatgac cagggttcct cctgcaggga gcccccagac ccaagggcct cagtccacct 1200
tcccctcccc tcccctcccc tgccctcccc tcccctgccc ttacctgccc tgccatgccc 1260
tgccctccct gctcctgggg ccctgtgagg accccgtggg tcacattggg ggacatcggt 1320
tatcgccctc acgattgctc tgaatgtaag aaaaacctcc tgcggggacc gggaacacga 1380
gctggcctgt gctgtggggt gagaagaggg gccccgaggt gcgcagtcag gttccccggg 1440
tcggcgaccc ctgagagcct ggccctcacc cgggcccctc cctgagtgtg tcccctcccg 1500
cagtgcctgg ccgccggctc catcatcatg aaccgggagc tggagagcat ggccatgcgc 1560
ccgctggcca aggagctgac gcgcagcctg gaggacgtgc ggggcgccct ccgtgaccag 1620
gcgctgcggg acctgaacac ctacacagag aagattaggg aagcgctgag gcacttcgac 1680
gtcctgttcg cagagtttga gctcagctac gtctcggcca tggtgcctgt gaagtccccc 1740
agggagtact acgtgcagca ggaggtcatc gtgctcttct gcgagacggt ggagagggcc 1800
ctggacttcg ggtacctgac tcaggacatg attgatgact acgagccggc cctcatgttc 1860
agcatcccca ggctggccat cgtgtgtggc ctcgtggtct atgcggacgg acctctgaac 1920
ttggaccgca aggtggaaga catgtccgag ctgttccggc ccttccacac gttgctgcgg 1980
aaaataaggg atttgctgca gacgctgacg gaggaggagc tgcacacgct ggaacggaac 2040
ctctgcattt cccaagacgt ggagttcccc atccgcgcag acgtgcaggg acccgctgcc 2100
ctggcgcctg ccctctctgc ccctctcccc cctgaggggc cactctcagc taaggccaaa 2160
gacccggatg cagagctggc ctgctccatg cagtacgacg accaggggct ggagcagctc 2220
agccgcatgg tccacagggc gggggacgag atgtcctctt tgctttcacc gcccattgcc 2280
tgccagtccc cagctcacag gccaggagcg gagggcagcc caggcgggga ggcctctcca 2340
ggtagaccgc gcctgcggtc aggcagtgac gaggaggagc gcgtgttctt catggatgac 2400
gtggagggga cggcagaagc cctggccagg cccgagtccc cagctggccc atttgggtgg 2460
gcaggcagta cctgggccga cccccaggag aaagggcagg gtgggccagg cggagcggcg 2520
gggatcagct tgcccgcctc ggaaaaggag gaggacttga gcaacaacaa tctcgaggcc 2580
gagggcacag atggggccag cctcgcgggc accagctcct gcagctgcct ggactcgcgg 2640
ctgcacctgg acggctggga ggtgggtgcg gatgacgcag agacggctga gatgatcgcc 2700
caccggacag gggcatgaa gctctcagcc acggtcatct tcaaccccaa atcgcccact 2760
tccctggact ctgcggtcgc cacccaggag gccgcctcgg agcccgtggc cgaggggatg 2820
gatggcggcc cccacaagct tagcactggg gccaccaact gccttctgca ttcctgcgtg 2880
tgctgtggga gctgcgggga cagcagggag gacgtggtgg agcgtctgcg ggagaagtgc 2940
agcccgggag gcgtcattgg tgcctcgtac gctgccggct tagccaaggc cagcgacagg 3000
gcccctgaga gacaggagga ggcgccccca ccctcagaag atgcctccaa cgggcgggag 3060
cccaaagccc ccacttccga caagtgcctg cctcacacct caggttccca ggtggacaca 3120
gcgagtgggc tgcaaggaga ggctggggtt gcaggtcagc aggagccaga ggccagagag 3180

ctgcatgctg ggagcccccc ggctcacgag gcgcctcagg gcctgtcggg ctccagcagc 3240
tccacagctg ggtcctgctc ctcagacaag atggggccag aggcggcccc agcagccacg 3300
catgctgccc cacaggccac aagagagaag atccggtcca ggtttcacgg cagccacgac 3360
ctcatccacc gcctgttcgt ctgcatttca ggtgtggctg accagctgca gacgaactat 3420
gccagtgacc tgagaagtat tcttaaaaca ctgtttgagg tcatggccac caagcctgaa 3480
acagacgaca aggaaaagtt aaggaaggtc acccagactc tgcggagtgc ggccttggag 3540
gactgtgcac tgtgccagga gaccctgtca tcctctgaac tggcagccaa gacccgcgat 3600
ggggactttg aagacccccc ggagtgggtg ccagacgagg cctgtggctt ctgcacggcg 3660
tgcaaagcac ccttcaccgt catccgccgg aagcaccact gccgcagctg tgggaagatc 3720
ttctgctcgc gctgctcctc gcactcagca ccgctgcccc gctacggcca ggtgaagccg 3780
gtccgagtgt gcacccactg ctacatgttc catgtcacgc ccttctacag cgacaaggcc 3840
ggcctgtgac gtggtgccag gggcagcccc aacccacccg ggccaggaac ccccaggaag 3900
gtggggccac gctgcaggca ggtctcactg cgtctcatga ggccgctgcc gctgctgcag 3960
gggcacccag acctccagag catccagagc ctctgtcctg cccacgctag cctccctgca 4020
gggaccccgg gccggctgca ggggccaaca agaggtcagc tttgcccagc aggggacccg 4080
gaggagcctg gtggctgcct cagggcaggg tgcctggcgg agaaggcctg ggccctccca 4140
ctggtccctc ggagatctgc tggggctatg ggaatcgggg cgggtgatgg aggtggacaa 4200
gagtggggcc actctgccgc ctctgctgtt gcggtgaatc tggaagccgg agcaagggcc 4260
actccacagg ctctttggca cagcagggct ccctatgtgc ctcccaaacg tcaaatgcag 4320
gctacccatt ctccccatca gctgcccacc tcgttcccca gccctcaccc ctcacccctc 4380
accatccttc ctcaggactt ggctaaacaa catgcatctc atttcttctt ggagtgggga 4440
gatgcgggaa gctgacccag tggtcctcta ggcaggcagg gtgctcctgg tgtctgccca 4500
ccactgtccc cttgctgatg gcatgggggt ccgaccaggc actgacaccg caggcagagg 4560
ttgctcgggg ccttgggtcg ggtttaccaa tcagccagga cgcccggggc caccagctca 4620
gctaccactt ccccttccct ctgcaccctc tggaaccctg gcccaattcc tcagacccca 4680
gagtggcctg ggacccaagc tgccgcagct gagtggcccc agcggacacc tcctcaggct 4740
taaatctcag gcttcacatt gcaatgacga ttgcagtttt atttttggag atgacacacc 4800
tactgcttct tttctaggat aaatctaatt actgtttact ggtggagtac atatgcatag 4860
aaacggcttc tgtcgggggg agagtatata atggaggaaa tattatgtat tgagattata 4920
tttattttct aaatgctgta atttgaagcc atttccataa atctatttaa ggattgc 4977

<210> 131
<211> 3571
<212> DNA
<213> Homo sapiens

<400> 131
   ctgagagaag gccggctcct gtgctctgca ctctggccca gggctgctgt tccgctggct 60
   gacgccctgt ccccacccca gcatgtcctg cttccaggct gcttgacccg gctgggctgg 120
   gtgcctcttc cccacccccg ccccagtctc ccgggcccca tcccagcctg gcccgtcata 180
   actgccgatc cccagaactg tccctggtgt gcagcaggca tgcagggtgg gcctgcagag 240
   cgaggtccca gcaccggcgg catctggcgc tgaacacagg aagatgggac gtttgctgaa 300
   tgaatgcacg tccctgatga ggcctggact tcgacggatg aaggggctgg tggggtggag 360
   tcagggttcc ccgaaagccc tgggtggaga gggtccccaa agtggggtca gacagcctgt 420
   gaggctcaca gtcctgccct gcagcccttt ggtttggacc aggtgccacc tccccatctc 480
   cctggcccca gccctcagct aaccagccca tgccaccgcc tggaaatggg ccagagggca 540
   gtggcgacct gcccaagtcc acacgcaggc cacaggccac actctgaacc ccggggcagg 600
   ctcagacggg ctttgctcag cctgccttag ggcatagagg gagactgagt cagggccctc 660
   ccaggaagga tgggtgtgcg gccttccacg ctcgggactt tggccacgga tgtccacccc 720
   agggtcttcg cagagcagcg ggaaaggacc ctgctggcag gcacagtgcc cgtctctgcc 780
   cccggctctg ctccaaccgc ccccagccca agggcggcac gccgcagggg gtctgtgatc 840
   cccccacgtc cccggctcct ttcacccaca caccacactg ggaggcggca gcccgagggc 900
   aggtttattg acaacctcac gggacacaag caggctgggg acaggacggc gacaggctcc 960
   ggcggcggcg gcggcggcgc tacctgcggt accagatctg cagcctccgc tcccgcttga 1020
   tcttcctctg cagctgcagg atgccgtaga gcagggcctc ggccgtaggt gggcagcctg 1080
   cagggacctt gcctcagtct cgcttcccgc agccggagcc cgcgtgaccg cgtgcacagc 1140
   gcagccggct cggctttgga agggtttccc gggccggcct ctgaaggtgg aggctgacag 1200
   ccctgggtgg gcgcagtgcc agggactggg agccggcgca ggggaggtcc ctgcaggaag 1260
   agtggggtcc tcccctgggc aaggccccac tgccctccac ccgtggctca tcccgggctg 1320
   tcacagggac ctctcggaca agacgctgac ctcactaacc cggcccccgg ccccagccct 1380
   caggactcaa tgggggccag cgctcagcag acacacttag cagtggggag cagggcccgg 1440
   ggctgccctg gacggagatg gaggcaggga ggctgggcgg agggagggga ggtggcccag 1500
   agcccgggca tcccacgagg acccgatgac cctctttctg agccccaagc cagagctctt 1560
   tgctggggat cccccagtcc cggctgccac gcaggttcag aacaaagggc ttgagcaaga 1620
   ggacgaagcc cagggcccaa cggggacagc aggagtggaa acaggggaag acgccagggg 1680
   catgggcaga gctcctgacc ccaggatgga cctctctgtg ctgtcaagtc acagggaggc 1740
   ccaggctgcc ctctccactg cccccggggt gacctgaacc gtgcagaacg ctgaacaaat 1800
   caacgccctt tccagggaag cggaatccaa agtcagagcc tgttcctcca cttttgagag 1860
   gcaccaggat gtgcctctcg cttgcccaaa cccccagact cgggactcag ggctgggctc 1920
   tctgcgcatg agctaatgcc gcacgcagca cagggtggcc aagaacggga ctctgtcccc 1980
   tgtcccacat gagcgccacc tccaagcctg cctaccgtgg accactgtca aggtgaacgc 2040
   ccggggggat ggccctcgtg ggaccacgtg aggtgcaaga gtcccacggg atcaccagat 2100
   ataaagaatg ttcccggtgc gatgtgaatt tcaggtagga ccatatgaaa ctgctgtaaa 2160
   ccaggcgtgt gtcaggaatt ccactcagct tcccctgctc cctctgatcc ctctgatgcc 2220
   tgaatacatc gccgacttct cacaggggac aggctccact aagaggcagc gctccgagtc 2280
   ggcccatcca taggatgggg gagcccgccg tctgctgggg gtgtgggact ccctcacagg 2340
   ggccgccggg ctccgtgtgg cgctggccct gtctccagct ccctcgtggg cggtgcggtc 2400
   ccggccctac ctgggatgta gatgtccacg ggcacgatgc ggtcgcagcc cctcaccacc 2460
   gagtaggaat agtggtagta gcctcctccg ttggcgcagc tgttggggag gcaccgtgag 2520
   gggagtgtgc cgaagacccg cctgcccacc ctccctccac gaggcctcgc cccatcccca 2580
   cccgccttca actgcaggcc aggcacctac gaggctggaa cttgtcacgg agaagcaatg 2640
   agcaaacacg cacaggctcc cagagcctct gtgggaccag tggggcgcaa gctcacacgt 2700
   ggctgataac tcggggaaaa gtgctcccct cccgaggggg ctcctgacca ccgattcccg 2760
   gggctgggat gtgtcagatc acatgtgtca acaaaaagcc acttcagaca gccacagtgc 2820
   caacacccag cccctgtcta tggggcggct gagcctcacc ctcgcctgat tccggctctg 2880
   tcagtgaggc gctggggtcc acccggggca gggagctgga aaccccagga gaccacacag 2940
   actccactcc cagcactgct tcccacagcc gtaaaaatgg acataccctg aatgcccagc 3000
   agtgggagac ggttatgaaa actctggaat attctaatta actgtttaga attagattag 3060
   taatatgtcc acacagtggg atattattca gccaagaaaa ggcacgaggc tcggacacag 3120
   gccacagcac ggatgcacct tgaggacgtc gcgctcagtg agagacgccg gacacagaag 3180
   gccatgcggc gtgtgatcct gtttctgtga aatgtccagg acgggcccat ccacagagac 3240
   agggagggga ggtgtgggca ccggggctcg gaaggggatg cagagtgact gctgatgggg 3300
   acagcatttc atttgggggt gatgaggatg ttctggaatt agaggtggtg gcctaaaaac 3360
   cactgaactg ctcgctttaa aagggtaaac ttatcaagac ctgatctcta cagaaatttt 3420
   aaaaagtagg cacagtggag tacatctgta atcccagcac tctgagaggc taaggttggg 3480
   ggaccgcttg agcccaggag gtcaaggctg cagtgagcta tgactgcacc actgcactcc 3540
   agcctgggca actcagtgag actctgtctc t 3571

<210> 132
<211> 4233
<212> DNA
<213> Homo sapiens

<400> 132
ctcaactcac accgtgtatg caaatcaact cgcactgcgt atgcaactca actcgcactg 60
cgtatgcaac tcaactcgca ctgcgtatgc aactcaactc gcactgcgta tgcgaatcca 120

ctcactgcct atgcaactca gctcgctctg cgtatgcaac tcagctcgct ctgcgtatgc 180
aacccagctc gctctgcgta tgcaactcaa gtcgcactgc gtatgcaact cagctcgctc 240
tgcgtatgca actcagctcg cactgcgtat gcaactcagc tcgcactgcg tatgcaagtc 300
agctcgctct gcgtatgcaa ctcagctcgc tctgcgtatg caactcagct cgcactgcgt 360
atgcaactca gctcgcactg cgtatgcaac tcagctcgct ctgcgtatgc aactcagctc 420
gctctgcgta tgcaactcag ctcgcactgc gtatgcaact cagctcgctc tgcgtatgca 480
actcagctcg ctctgcgtat gcaactcagc tcgcactgcg tatgcaactc gcactgcgta 540
tgcaactcaa ctcgcactgc gtatgcaact caactcgcac tgcgtatgca actcaactcg 600
cactgcatat gcaactcaac tcgcactgcg tgtgcaactc aactcacact gcgtatgcaa 660
ctcagcttgc tctgtgtatg caactcaact cgcactgcgt atgcaaatca actcactgca 720
tatgcaactc agctcgcact gcatatgcaa cgcaactcaa ctcatactgc gtatgcaact 780
caactcacac tgtatgcaac tcagctcgct ctgcatatgc aattcaactc gcactgcgta 840
tgcaaatcaa cttactgcat atgcaactca actcactgcg tatgcaactc aactcgcact 900
gcgtatgcaa ctcaactcgc actgcgtatg caactcaact cgcactgcgt atgcaactca 960
gctcgcactg cgtatgcaac tcaactgttg caagtactta tttccggcca cttccttttt 1020
ctaactacca caccaagcca gtatttctcc tccctgaagt cagcccagga tgaggcacta 1080
gacagcagga catgctgtat gcccttgggc ctgctggaag tatgcagact agccagcccc 1140
agacttcatc ctgccctgtc ctgcctttcc tgtgaaaacc ctgtggcctc tgcctcccct 1200
ggctctgact tctgcctcct gcccagctct gcagctcccc ttgggccctg cctggagtga 1260
tgtgccgcct tctcttgaca ctgtgagtga taaactttcc atgtcaggaa cctgtgtgtg 1320
tcactcactc accttgacga gtccgcgtct aggccccacc agtggtgtgg ttttcctcat 1380
agtctctcta cctaagcaca tgtctgtgac aaggtcttac ccagcccagg gattcttgaa 1440
ctatctgtag gaactgccat gttgactcct gggcagtttt attctttctc tctactcgtt 1500
caaccttatt agggagtgac gtttttttcca aagtggttgt gaaacagcat ctccttgggg 1560
tgttggtttg cacttctctt cattactgat agtatgaaca tggttttgct tacgctggct 1620
attttccttt ttggatgaaa tacctggtct tgttttttcc tggtttgttt gatctttgtt 1680
ctttttttaga aatagtgagg ctgttgtgag gtgagtcacc acttgtgact tagaagattg 1740
cagtggtttc cacttcacct gtttccactt ttgtgatgat gccttaagga aaaagattgt 1800
gccaggtgtg gtggctcagg cctgtaatcc caacactttg ggaggccgag gtgggtggat 1860
cacaaggtca ggagatcaag accatcctgg ccaacatggt gaaacgccat ctctactaaa 1920
aatagaaaaa ttggctagat gtggtggtgc acgcctgtaa tcccagctcc ttgggaggct 1980
gaggcaggag aattgcttga agatgggaag cacagattgc agtgaaccga gatcgcgcca 2040
ctgcactcca gcctggcaac agagtgagac tctgtctcaa aaaaaaagga aaaatatttc 2100
ttaattttgg catactcaaa tttatacaac ctttccttta tcttcatcag aaatttcttt 2160
tctcctaagg ccttaaatat gctgtactat tttggcttcc acagtttcta tgttttatcc 2220
caagttgctt aatccatttg gagttgattt ttgttgaccg tatctgtaga gtgtaatgtc 2280

attttttccca ctatggacat aaaaattttt gcaaggcctc tttttttttaa tgaagaaaag 2340
aggtttattt tgttcacaat tctgaaggcc tggagggtcc cagaagcttg gcaccagcac 2400
ccactaggct ccagatcggg gcctcatgct gacttccgtt ttcacacaac gagtgggaaa 2460
caggtgtcct aacactgtga gagaaaaaat ccgaaagttt agtttttccc attcgcttgc 2520
cctggcctcc aggcagtcct gcagcagcaa acacatcatc agccttagga gctggcctgt 2580
gaggttgacc actctgtagg tggacgagcc tcgcccgaga agagacatga gcagttctgc 2640
gtaggcttta agacagaatc aacatggaaa gcacgaccca catggtttca atagaacttg 2700
cctcctgaac ctgactaggt tactcgcctg atgaaccagt atctccatca cccgtgggac 2760
tgacagaccc agagtgtcat aacctaaaat tcagaagtgt agactataat cttaaattat 2820
tcagcatatg tgcaacagcc ttggggagca gtgttatgat accaaatggc taacattcat 2880
gacctcagta tcctggaaga aaaggagaaa gtgtgatgta gtaaaaccac ttgaaaaaat 2940
aatgatgaaa catttctcat atttggtgta agataaaaac ttaagtttga agaggttcca 3000
tgagccccag acaggataaa cctaaagaaa accaagccca ggccgggggc ggtggctcac 3060
acctgtaatc ccagcacttt gggaggccga ggtgggcgga tcacaaggtc aggagatcat 3120
gaccatcctg gccaacacag tgaaacctcg tctctagtaa aaaaaaaaaa aaaagaaaaa 3180
tacaaaaatt tagttgggcg tggtggcggg cgactgcagt cccagctact ggggaggctg 3240
aggcaggaga atggcgtgaa cccgggaggc agagcatgca gtgagctgag attgcaccac 3300
tgcactccag cctgggcaac agagcgagac tctgtctcaa aaaagaagaa agaaaagaaa 3360
accaagcccc tgccaccacg aagctgccga aagctacaga gtgaaagtct tggaaggagg 3420
ctgagaaagt ggacacagtg cttccagaag aacaattcaa ttgaccgggt ttctcgtcgg 3480
aagccataga ggccaacagg aaatgtagga gcatttttca agttctgaaa taaaaagaaa 3540
tgccaaccca gattgctata tccaacaaaa tatcctttag gaatgaaaca gaagtaacga 3600
tgaggcaata caaagaattg cctgcagacc tggtttaaat aaatggttac agaaaactct 3660
taaggaaacc tggaacaaag gaaaagcaat agaagagtaa atacctgagt aaatagatca 3720
gatcattcta ctcttgagtt ctttaaaata tatattattg tcaaaagcaa aaattatcac 3780
atggggtttc aactgatgta tatttaatac ataagacaac tacaacatga aggtgagggg 3840
caacgtggct ggccggcgtg gaggttccgt gctccatttc agtggcaaaa taccattcca 3900
gaaggactgg aaaagctaag tctgtatatt tcaattacta aagaagtgta cagctgggtg 3960
tggtggctca cgcctgtaat cccaccactt tgggagagtg aggtgggtgg accacttgag 4020
gtcaggagtt tgagaccagc ttggccaaca tggtgaaacc cgatctctac taagaaattc 4080
aaaaattagc tgtgtgtggt tgggggcacc tgtaatctca gcttctcagg aggctgaggc 4140
aggagaatca cttgaaccca ggaggtggag gttgcagtca gtcgagattg tgccacggca 4200
ctccagcctg ggtgacagag caagactctg tct 4233

<210> 133

<211> 4976
<212> DNA
<213> Homo sapiens

<400> 133
tgcaagctct tctcacttca tcattcttcc tacttcaggg tttggtaggt gtctggggcc 60
cagcctgtgg tgaatgtgtg cacagttgaa tgagtcagtt gcttctgttt gttcctccag 120
gtatttcatc gctgatttgc cccacttgca ggacagcttt gtggacaaac tccttgacct 180
tatgccccga ctcatgacat ccaaacctgc agaagtggtc aaaattctac agaccatgct 240
gcgacagagt gcctttctgc atctcccact tccagagcag gtcagggtct atttggcccc 300
tgtaggccaa atctgtccag acggtcaaaa atctttacct tggtgatctt ctttgaccaa 360
tggagcagct gggctaggct cttcctctga gtggctccca catgcccact cattagataa 420
ggagacagaa aaaagatgtg ttttcttgtc caggaatgac ctggctttag aatcatagga 480
ttttcctctg gcttacagat ccacaaagcc tcagccacca tcattgagcc agcgggcgag 540
tcagacaacc ctttgcagtt taactctggg ttggtggttg ccctggatgt tgatgcaacc 600
ctggagcatg tgcaggatcc tcagaacact gttaaggtcc aggtctgtcg ggtttgggtc 660
ctcgtggagc ttctagactg accttgtgtt gaagtattta atgttagtac ccctatagtc 720
tctacttgag ggacagaaac tgggacagga gaagaaatga gcctctgtgg gaaagaacct 780
aggcttgaat ctcaactact gctgagtgtg gtttgaagca agttacttta aactctgagt 840
ctcagattct acaactgaca aataggagga gttgacataa agagataatg aatttaaagc 900
ccccaggacc cagtaagggt tcagtaaatg ataagacctt ttctaatctt gtaatgtgac 960
gttaagattt ggtcttttat tacagaaaac tataataaac aagttccttt caggctgtga 1020
gtcagtcagc tggcagataa aatttatgga tgggaaaaag cgattactag gaaatattct 1080
ttcccaagtt acatagatgt aatagagaca catgagaaat gtagtagaga cactaatcaa 1140
ttcagacagt gcagttaagc cccacaaaga gtactactcc ttcattcatc catctgtcca 1200
ctcattcatc cttccttcca tccatttgtg caattgtgtt tactctattg catgcactaa 1260
gtgctgtggt tgccaaggta aatatatcac agttcttcct ctcggggtgt acacagtttc 1320
attcagagac atacaaatgg gcaggcaatt ataaaacagg gtttggtgag tagacatgca 1380
caggatgggt catctcctag actgtctcct aaaaaaccct ttcaaagcac ctctaccaca 1440
tgcagggcta aaccttttac agtattcaat ttggagtgtg gtggaggctc aggaaaggaa 1500
aggagaaggg cacaagggat atgagagaag ggccaaaggg atatgagtca aagcagggat 1560
gcaagcactg gcaggaagta caataagggg acagaaaaat aggctggaac aggcagagaa 1620
ggcttcctgg aggaggtaag gatgaatcag gccttgaaga ttacgctttt tggctatggt 1680
gacaataaag gaacgggcct tctggcttcc aagcagaaat tattataaat tatgagacct 1740
gcagagaact agttctgagc tagctgtgaa gttgtattgc atattggagt gtttttgttt 1800
tgttttgttt ttttccacat tagtggttgt ccaccttcac tgcactttag gattactcag 1860

aaagcattaa gaacagatga ataggtgtca gcctacatta attaaatcaa aatcctgaga 1920
atagggccta tgggcactag tgtttaagag ttcataggtg attctaatac gtagtcagtg 1980
ataccagcag aactactgtt ttacattgtc cctgaaaaaa aacttcactg atcacctgag 2040
tgtagaggag attgtaggca ggttacttaa gggtcatctc ctagactgtc tcctaaaccc 2100
ccttcaaagc acatctacca cacgcaaggc taaacctttt aggtacctta taggtagagg 2160
gacgggaaac tcactgcccc atcgtgggac aggtccagtg ctttgaaagt gccatccagt 2220
ccagtcacca caccctgagt cattcctctg ttccaggcac tacttacttc aggtgctcga 2280
tagagatata gcagtaaaca tgcctcagag atgacactga gattacattc cccaataaaa 2340
tactgtagat ctgttcttta aagcaaagcc tgcagaactc cattcattaa ctatttattt 2400
tattttattt tattattatt atactttaag ttttagggta catgtgtaca atgtgcaggt 2460
ttgttacata tgtatacatg tgccatgttg gtgtgctgca cccattaact cgtcacttag 2520
cattgggtat atctccaaat gctatccctc ccccctcccc ccaccccaca acagtccccg 2580
gggtgtgatg ttccccttcc tgtgtccatg tgttctcatt gttcaattcc cacctatgag 2640
tgagaacatg cggtgtttgg tttttttgtcc ttgtgatagt ttgctgagaa tggtggtttc 2700
cagtttcatc atgttttatg gctgcatagt attccatggt gtataagtgc cacattttct 2760
taatccagtc tatcgttgtt gggcatttgg gttggttcca agtctttgct attgtgaatg 2820
gtgccgcaat aaacatacgt gtgcatgtgt ctttatagca gcatgattta tagtcctttg 2880
ggtatatacc cagtaatggg atggctgggt caaatggtat ttctagttct agatccctga 2940
ggaatcgcca cactgacttc cacaatggtt gaactagttt acagtcccaa caacagtgta 3000
aaagtgttcc tatttctcca catcctctct agcacctgtt gtttcctgac tttttaatga 3060
tcgccattct aactggtgtg agatggtatc tcattgtggt tttgatttgc atttctctga 3120
tggccagtga tgatgagcat tttttcatgt gtttttggc tgcataaatg tcttcttttg 3180
agaagtgtct gttcatatcc tccgcccact ttttgatggg gttgtttgtt tttttcttgt 3240
aaatttattt gagttcattg tagattctgg atatttgccc tttgttggat gagtaggttg 3300
cgaaaatttt ctcccatttt gtaggttgcc tgttcagtct gatggtagtt tcttttgctg 3360
tgcagaagct ctttagttta gttagatccc gtttgtcaat tttggctttt gttgccattg 3420
cttttggtgt tttagacatg aagtccttgc ccatgcctat gtcctgaatg gtattgccta 3480
ggttttcttc tagggttttt atggttttag ttctaacatg taagtcttta atccatcttg 3540
aattaatttt tgtctaaggt gtaaggaagg gatccagttt cagctttcta catatgacta 3600
gccagttttc ccagcaccat ttattaaata gggaatcctt tccccactgc ttgtttttgt 3660
caggtttgtc aaagatcaga tggttgtagg tatgtggtgt tatttctgag gactctgttc 3720
tgttccattg atctatatct ctgttttggt accagtacca tgctgttttg gttattgtag 3780
ccttgtagta tagtttgaag tcaggtagca tgatgcctct ggctttgttc ttttggctta 3840
ggattgactt ggcgatgcgg gctctttttt tgttccatat gaactttaaa gtagtttttt 3900
ccaattctgt gaagaaagtc attggtagct tgatggggat ggcattgaat ctataaatta 3960
ccttgggcag tatggccatt ttcacgatat tgattcttcc aacccatgag catgggatgt 4020

tgttccattt gtttgtattc tcttttattt cattgaacag tggtttgcag ttctccttaa 4080
catctgttaa ggagattaat taacatctgt taatgagaac tttcctcgat taaatattct 4140
gtcattggag attggtacat ttctttgtca agggcaacag tttttaagcc attcacatac 4200
acacgttcct gggagaagtg acactgggga gatttttttt ttaacccctg aaaataaaca 4260
tatcggagaa tacattcatg atctctccac aggttctaat aaagggggga gacctgggtt 4320
ctgcacctgg ccttgttgtc gtggttacgg ttgctttcag gcgatggcac ctgttctcca 4380
ggtgtgtctg gacttctact tgctttagcc atatatggcc accaggggga gcagtcaccc 4440
cacagttgcc tggagcgccc tcgctctcca aattcacagc ttgaaatgtc acctgtggcc 4500
gtctgcggtc atccacggtt ttctttctta ccttttcttt ctttctttcc ttccttcctt 4560
cctccttccc tttttccttc gttccttcct tccttttttt ttctttcttt ctcttttctt 4620
tctttctttc cccctctctc tctcccttcc ttctttgtct ttctttcgat ccttccttcc 4680
ttctcccctc ccctcccctc ctctcccctt cccgacggag tttcgctctt gttgcccggg 4740
ctggagtgca gtgagccgag aatgcaccat tgcactccgg cctgggtgac agagcgagac 4800
tgtgtctcaa aaataaaaat aaaagttagc aaggcatggt ggtgtgcacc tgtggtccca 4860
gctactcagg aggctgagct gggaggatca cttgagctca cgagttcaag gctgcagtga 4920
gccatgattg ggtcactgca caccagcctg ggtgatggag tgagacctta tctctc 4976

<210> 134
<211> 3384
<212> DNA
<213> Homo sapiens

<400> 134
acagtccttc tgaggaaggg catgtgaggt gttcgttgtc tgtggtagac ctgggtctgt 60
gggaggctca ggattctggt ccggactgga gtagaacctg gggagctatt ggctggcctc 120
tgttatttaa agccacagaa tggatgtaaa accaaggact agcagtcata ctgagacggt 180
gaaatggtgg aaattggaac cttagggagc agcactgggc tggatggaag aggaagagcc 240
tgcaagagaa aagagctgcg gaggtcagag ggagcggctg tgccttgggc caggggagca 300
gtgagccacg ttccttatcc caagcagact ccttggagat ctggagaggc cggagctgag 360
ggccgggacc ccgagggcct cggaaggagt tgtttcagcc tcttgggcca acagccagat 420
cccagtggct ctggaagtaa tctgtctaga tgtgagatga tgaaggaagg aaggatggct 480
gtacttgagg agggagccgg gtcacagatg ggcaagaggg cacgatcgtg gtgttgagtg 540
aagagaaagg actgaagaca aaggaagtgc cggagcctgt cctggagaaa ggagaaggga 600
cctcttgtct gtggagtcag aaaagcagga aaggttgaag ggagaaggtg ctacttactc 660
actaccaggg gcctgggaca caagcctgtt tgagggtttc tgaagaacag tggagatgct 720

gagattctgt gcaggccttt ctgactttgc accagtggca gttcgcagcc tccaggactt 780
ggctcgcatc gccatccggg gcaccattaa aaagattatt catcaggaaa ctgtgagcaa 840
aaacggaaac ggactaaaga acacccccag gtttaaacga aggagagttc gccgccgtcg 900
aatggaaacg attgtctttt tggacaaaga agtctttgcc agtcggattt ccaacccctc 960
agatgacaac agctgtgaag acttggaaga ggaacggagg gaagaagaag agaagacccc 1020
gccggaaaca aagccagacc ccccagtgaa cttcctacgc cagaaggtcc tgagcctccc 1080
tctgccagat cccctgaaat actacttgct ttattacaga gaaaaataag tctcctgttt 1140
gaaaggggga aataggaaga gcagattgct gagtgtgaag ttcgtgctgc ctgtgtgctg 1200
ttgaagggtc acctggaggc agacgttgtg gggaagggaa ctgctgggct catccacacc 1260
atggttttct tctagttcct gattgacctc taaaattcta ttcagttgta tgatttgttt 1320
acatagttcc acaagacctt cattgcatag aagattgttt tcccaaagtg gagagaatct 1380
tcatagagaa aaagagaagg ctgtttcttt ttcggctctg acgaaacact gaagtctgcg 1440
taagagagac tgtttgatga ccgtccctca tgcaacatgc acggtactca ctaaaaatga 1500
aaactgaagt ggaaactaac ctgtgttgct tataaagtgt gaaagcacaa gcttataaat 1560
gtataaaatc ttttctgggt gtgacgcacc tgcgtccaag tttgaatttt tatgatatgt 1620
accacttaat tactggcact gagtatcact gaatttctta gttttctagt ggggaaacat 1680
tattgagaag ccctccctta ttttaagtaa gttgattaaa tcttatgtga gttgccagtt 1740
gtaatttttc aaaggaaaaa ttttgatggg gtggaggaat gaattgccag ataatctttc 1800
tggaattccg agagaattcc aaagagggtt tttttttttt tttttaggac atcttttgat 1860
acctttaaaa gaaccactgt caagtaatcc ttaaaagaat atcttggaaa aggaaacaga 1920
ttttttcctg tgtgtaagca ataagtgaag ttacatttgc cctaacccta gggatgattc 1980
tttacccagt tttaaagccc atcatggtat tctaaggtgt tgacaccctc catcctcaga 2040
gcaggtcgaa aatattaaat agactgggga ctctatgatg ggcagcctgt gctttttgac 2100
ttcagtttgc tatttttctg tgatcacatt agtactgatt catagattct atcttttata 2160
attctggaga aaaagatttg ttagttttgt aatttttttg taagaccaaa tgtatgtatt 2220
ttagtagctc cattgcatga gaagagtgta actcacactg acttgtgata tcagccttct 2280
ctgggccttg tgtgtggaga gctttctatc ttaccaagtg gtagggctaa aagaacaaca 2340
gcctttttgg tagtcacata gcagaatgat cagagttaca ttgcttattc caaaacattg 2400
gttcttttta aaacattttt ttttacccaa agaaaagaat aatagaaatt actaacaata 2460
aatataaatt cagagtgttg atataggatt cagtatccag agtttatttt taatcttaat 2520
cctcagcttc ttgggagttg ctgggcttca gtgtctctgt ggtttcacca gcttagcttg 2580
agctctggtt attttggatc ttttctgctt tttttaagta actgagtcat ttttaccaca 2640
cagtccagtt tgcatgtata gctaggaaac atgtattgct ctagattggg cagtttaagt 2700
cattttaaag aaagttagtt catagttgtt gccttttaac tcatagtcaa gcttcagtct 2760
ttcaaagaga aatgtgtgat tttcatttac ttgctgatat tttgtagttt ggagatcctt 2820
gtgggcatta ttctaactga tacgtagaca cttacttgga aatttttgga cattatatta 2880

aatgagtgct atctgtgaaa ttggttatat taggtggctt gactaatgtt ttttctataa 2940
ttgtatatgg actgcatttt taaaaaaacc gcatttgcct ttatgctaga ttgtaaaaaa 3000
ttatattaga atgcataaga catgtttttc cttcatatgc tagacttttc ctagcatttc 3060
gtatttctgt gttgtcagtg tgtgattttt aaaccggaat ttggtttaaa aaaaatctgg 3120
tggtaatata tgtgagaaat actttggtgt ttaccttatg aaaataaagg attgtaagta 3180
aagtttcctg cgcaccttat accagaattc agtataatac actactttct gttttcaaac 3240
agataaatca taatatagtc tgtattatct gtaagatctg tcttgtaaac cacattcttg 3300
acaactattt gcttttgagt agtttgtatt ttaatatgtg acttttgtct tgaaaagtag 3360
taaagccata gacttgtgca aaac 3384

<210> 135
<211> 4110
<212> DNA
<213> Homo sapiens

<400> 135
cagtatcctg gttgtgatat ggtaatacag ttttgcaaga tactaccctt aggggaaatg 60
aggtaagacc tggcatctct ctgtattatt tcttaattgc atgtgaatct acaattattt 120
caaaataaaa agtatgattg aagtaactat caggaagctt agcctactgt ggattaagac 180
atcaaaattg cagcaggctt taacaaagct atactactac aaggatagtt cacacttgaa 240
ttagcttgaa acattaccta tccatgtcat cttggaaagc agaaaatgga actttattgg 300
agtgggacac tggatggcca gggtttagca gagcctgccc aacaaaagta gcttcagaat 360
tggctttatt ctcaggaagg ctcttcctgg acagtggcaa acatagccat agcaaatact 420
ggctcacttt ctatcagcca gcaaccctgg tggaaagagg gcctcttact cagttgtacc 480
agcaagctgt ctccaggagc cggggctgga gagacaccca gtgctgctct aggccagtgt 540
ggctgagggt ctgtctagtc tgtccttgta ctcacggctt ccttttctct tgcagacgcc 600
cactattgct tggttctact tcctttatcc cgattggccg tcggacttat gccagtgcgg 660
cggagccggt tggcagcaaa gctgtcctgg tcacaggctg tgactctgga tttgggttct 720
cattggccaa gcatctgcat tcaaaaggct tccttgtgtt tgctggctgc ttgatgaagc 780
tttgaagcac ccactgagtg ggcggatcat aatttacttc atcaaaatct cctgtaaatg 840
agcatttagg tgttttcaat attttgcaat tacaaataat gccacaatga ataactttgt 900
atttttgtta ttattggggg gttatctttt ttttttttct tttttttgag atggagtttt 960
gctcttgttg cccaggctgg agtgcaatgg cccagtgtcg gctcactgca acctccacct 1020
cccgggttca agaaattctc ctgcctcagc ctcccaagta gccgggatta taggtgccta 1080
ccaccacatc tggctaattt ttgtattttt agtagagagg gggcttcacc atgttggtca 1140

gactggttat ctttagagta aattccttgg gagtgagatt gctcttagat cagagtgtaa 1200
ataaaatata ttgtcatttt aaagatattc ccaaattttc catcctaggg gtcatgtaat 1260
tttacattcc ctccaggagt gcatgagtgc ctatttccac acaatttcac caacagagta 1320
tgcatcaagc ttttggtttt tgccagtctg ataggtggga aatgtacctc agtgttgttt 1380
ttattgtatt tctcttctga gtgcataccc ctgttcttta aggacatgac ccagaagctt 1440
cctctgcaga tatcacctgt gctctcattc tcttggccag aacttagtca catggcctta 1500
cctggctgca aggaaggctg gaaaatgtag tgagtattct gagacaatat gtacccggcc 1560
taaaatgtca ggcgttctac tgctatagaa gaaggatgaa gggaagagtg ggcattcagg 1620
acaacttgct ttctctgctc ggtgggtgag acatttttct ttattttcaa ggcagtagga 1680
acccagggga ggaatttccc atactatgtt gatatctttg taatatttga tacccaacgt 1740
aaaatttcaa taatataaac aagtaaaaga aatcatttat attgccatgc tctaaacatc 1800
attccatctg gatcagagaa ggttttgttt gtccttttaa tctttacttc tttgaattgt 1860
gaaagtaata ctacatattc ataataaatt caaataatac agaagtatat tatgtaaaaa 1920
agtgaagtct tcccaatctc ttgctccaag gcagcttaaa tgcaggtgta tacattattt 1980
gtgagttttg ctattacagg tcgagtatct cttatctgaa atgcttggga ccagatgtgt 2040
tttggatttt ggatttttcc agatttggga atatttgcat tatacttgct cagcatctct 2100
gatctgaaaa tccaaaatcc aaagtgctcc aatgagtatt tccctggagc atcatgtcag 2160
cactcaaaat tcagattttg gaacatttca gatttgggat tattggatta gggatgctca 2220
accggtatta acaaagcctg ggtgagccat cttgcacata tatctttgcc tccttccact 2280
ggcgattctt aggaacattt cgtagatgtg aaagtgcaag ttgcaaaggt gtgcaggttt 2340
taggttttgt tcaaatcccc aaactgcttt cctgaaaggc tgtcgctctc ctgacagtgc 2400
tcaagagaac ccgtctgggg agggttctgg gaaatgggtg tggcatccct ggtgctggat 2460
tcttctgcag aatgtggacc tacctcagcg ggtcccagca agaaccatga tgccataaaa 2520
atgttgtaaa gtcccttgtc ctatgttagg cttctgtgcc attgtgctgg cttatccagt 2580
gacacaggga gccacagtca gattccacag cacagggcag aggctgctcg atgctgcaga 2640
agactcttca aaagggcctt cggttctagt cctagactga ccactaaccc cggaagggga 2700
caaacatgtc ccctccctgg gcctcagttt cctcttactt accctaatag gattggactg 2760
gtgatcttag gtccttccac tcctgcaaca gtgattctag aattggttga ctggtatttt 2820
gaagaaagta ccagacaagc tcattggcag gttatatctg ttggtatcta ccccttgacc 2880
cattccatct gctatctgag atcagtcagg caagtcagaa agcagttagc accaccagag 2940
gctttggata aggcaccgtg ggggttgaaa gggggaattt gcttctagcc cgattctgta 3000
gtgttagccc catgatggat tccggctaca tctgtaccta aacactctct ctgctcttgt 3060
tcagaattag tgttcctctt atctctcata tctcattgcc tttggccaac tcagggactt 3120
agctccagca gatgtctgcc acctcctacg tcactggctc cacgctctct cctgggtcat 3180
ttcagcacat acacacacac acacacacac acacacacac acacacacac acacacacac 3240
acacacacac acacacacac acacacacac acacacacac acacacccct gttctgcagg 3300

gcttcagcgt ggccttcatt cccgcctcgc ctcggcctct tttcaattaa gaagtgatgc 3360
cacttgaggc agggtctgca tgatcacttc ttgggtttga gtttgaaagc tgccatccct 3420
gcgactgccc agggcctgta tgttgggacc gaagccaaac accactgtcc tccagggggc 3480
tttcggcccg gggcagggtc cccagtgccc gccttcgaat cagactccgc gtttccgcct 3540
ctccctgggc ggacctgccg ctagagggcg ctcctcctcc gcagctggcg ccggcctcca 3600
gcgccccaga ctcccgcgct gccgcccgag cctgaggttt ctcacggcgt tcgggattcg 3660
cgcttcggaa accgggaaca aagaagcaag cagcaccctt cagaaacagt gttctcgctc 3720
tcctagcagc tggcccgggc ccggaggctg gtgccaggag aaggcagaag agcccgggca 3780
gccggagtgg gctacggggt tcaggcagtg ccaaggaaga agggcctcac aatgggcaat 3840
tcagctgcct cccgggccgt tgtccccgct gtcttcaaac aggggtcccc tgtcccagct 3900
gccccaggag gagcggggga ggcaggcagg gccgaaaccc tccagagccc agagggggac 3960
gcttggtggc cgctgcagtt acataaccat aggatgcttt tgtaccgtgg accttgttct 4020
agcaggatcc tttcggaatt gcacttttac atgttgggcg aatttgtgtc cgtgctgaag 4080
tttattaaag gaaaatagat ggagccttgg 4110

<210> 136
<211> 4058
<212> DNA
<213> Homo sapiens

<400> 136
ctggtcttac agataagttc acaagcagct ggttctctct tgtggataca tgggaccaca 60
gtggctgtag ctagtttcct aagactccat ctgccttatc tgccagatcc ttcctttgcc 120
acacacacac acacagtgtc tctctctctc tccctccctc tctcatacac acacaaccaa 180
atgcttcatt cttcacctag tcagaaacct ggggagcccc caggagagct ctgtccctga 240
ttgccacctg ccaacctgcc agtcttcttg gcttgaggca tctcagtgct ccagtgctca 300
gctcaccttg ccagctttcc tgtagcctga ttgggtcctg gtcatactca tctgttgcca 360
ctatttcatc agtttcggcc tccatgcccc aaaatgtggg atcctttttt ttttttagat 420
ggagtttcac tcttgttgcc caggctggag tgcaatggtg tgatctcggc tcactgcaac 480
ctctgcctcc tggattcaag cgattctcct gcctcagcct cccgggtagc tgggattaca 540
ggggcctacc accgtgcctg gctaattttt tcatttttag tagagatggg gtttcaccat 600
gttggccagg ctggtctcaa actcccgacc tcaggtgatc cacccgcctc agcctcccaa 660
agtgctggga ttacaggcgt gagccaccat acctggctgg gttcctctga attgtatttt 720
ttttttttaa gtaaagatgg ggtttcacca tgttggccag gctggtcttg aactccggac 780
ctcaagtgat ccaccttgct cagcctccca gaatgctggg attacaggca tgagccactg 840

cacctgctca gtccccttaa ttgtgattta ctttaatata gtttcattta tagtggtggc 900
ccctttaata tagtttatca tttcttacag ttgaagagtc cagtgttaac tgaacaggca 960
gaaaactaca catttctcta aatcagcacc ttaatttata aagtctcatg ttggaaatga 1020
agagaattgt gttgtttctt ggcttttaag acttggaaaa tatcttaaat ggcctctctc 1080
catttaggac aaacaataaa aacttgcctg gaataatctt cgaacagaag gtatgagata 1140
agtagtaagt aaacgtccac aggactccaa atagctctaa agatctgatt cccctaggag 1200
acttgctgag ggaaaatcta ggaactaaga tactgcaaaa tggaaataga ccttcttggt 1260
tgaccgaggc tctagggtga agcctgtgag tcagtgtctg cacctttgga aatagaggaa 1320
attagctcaa cagggcttgg ggcatgtagg tatctgccaa atattttata tgaattgaat 1380
ttggactcaa tttttggtat gtataatatg taacatgaag agaaaattta ccaggggaac 1440
tggaaaatgc acttaacttt tatggaggtg agaacaaaaa ctgaattaaa atctttccct 1500
gacttggtgt ttcctgtgta tgctcatgca tgctagctat aattttgtca tttctgtact 1560
gtgataattc ataattcata tttacatgct taaggtgggg cctaatacag ggcagggcgt 1620
tatttacact gtagggtagg cattgagcag aagtggtgtg atattttttc tgttcatttt 1680
tctagattgg ggaaagttta aattgaaatt attggcatca ctgatgataa ttaagaactg 1740
tttaaattcc atttctggcg aattcctgaa ggatatataa tcaggaactc tagggaaacc 1800
tgaagggctg agctggaggg tctagaattg gggccttaac ccgggagtcc aaaaactttg 1860
ccaaacactt atttccacct tccactcctc ctcccgtgcc caccctcaca gagcagtact 1920
tggcacccta gacaaatact gccagatcta cagacatcta atgtctgcag atacccactc 1980
cctgcctagc ctccctaatc acataagact gggacatctt cgaaggcaag gatacccctt 2040
gttcttactc atccttgttt ctgcaaagcc cacctacctc agggcctgac tcattgtggg 2100
tcattcagta gatgtttgct gaatgaataa acattgtttg tcttttgttc ttttaatgag 2160
taactgctgt ttcctttgca cccctcccca atacattttt ctcttggctg gcagctcctt 2220
gtctgcttgc tgaagataga accgcagacc acctgagcac cccctgcctc ctcagctgct 2280
cactgttctt ctgcatgtgg ccttcaggac ctctgcccag cagttgtggc ccatgtcacc 2340
ctctctgctg ttgtcaccag aaatttaccc gcactgtacc ctctggtcct tcacagctca 2400
tttctcataa tctattttaa caacaaagtt tgagaaggtt aagaatacag gatggtagga 2460
ggtggaaaaa acagtgggcg tgagcagtaa gtcgatatga ctaagctgag actcttctga 2520
gtcttctaga tggaagggga atttctccag agttacgggc agttaccacg ttgcctcatg 2580
gtcttgagcc cctcttttaa aacccctgtt gtctacatta agcggaagtc tccccttggc 2640
tgcattttga tgtcacttaa aatgttgctg gggcccatgc ttcctagagg aaggcaagca 2700
cagctagcat tcccaggaaa gccctcaact caggggtttc attttgtttt tatgatggga 2760
aataactttc atttcgcggt cactgcccac cccactactt cctctttgac cccttcatct 2820
aagatgaaca catctgttat tttaccctgt atagcaggtg caaaagagaa ttaaaatggt 2880
gtctgaatcc ataaacggaa tatattgcat agagaatcaa ggccacagta acaagtgaca 2940
ggcgatctta aaatattgga ataatctatc tttcaagtta cagaagctta ctagagatag 3000

gggcaggtca ttgttacaag catctggcag tacattaatc atgagaaatt aattaggcct 3060
taatgtgggt ctaatgagaa atccttatta tccatgtttg cttatgggct ttgttggtta 3120
attatcaaat ggaacactag agaccctaaa atgtgattat aaaatgattt ttaaaagtcc 3180
tttcttggag tgtaatattc tattaatgaa tcagcagaaa ctgaagatta tctgctttgg 3240
agatgtgtaa caatccaatg taattacaaa cctgtttaga gaggaaccat ttagctatat 3300
atgatcaaaa tagcccttat aactgttctc tgtgtatgtg tcttttatct tagttttctg 3360
tgttcctggg aattattttc ttcctggagc tcactgccgg agttctagca tttgttttca 3420
aagactggat caaagaccag ctgtatttct ttataaacaa caacatcaga gcatatcggg 3480
atgacattga tttgcaaaac ctcatagact tcacccagga atattggcag tgctgtgggg 3540
cttttggagc tgatgattgg aacctaaata tttacttcaa ttgcacagat tccaatgcaa 3600
gtcgagagcg atgtggcgtt ccattctcct gctgcactaa agatcccgca gaagatgtca 3660
tcaacactca gtgtggctat gatgccaggc aaaaaccaga agttgaccag cagattgtaa 3720
tctacacgaa aggctgtgtg ccccagtttg agaagtggtt gcaggacaat ttaaccatcg 3780
ttgctggtat tttcataggc attgcattgc tgcagatatt tgggatatgc ctggcccaga 3840
atttggttag cgatatcgaa gctgtcaggg cgagctggta gacccccctgc aaccgctgct 3900
gcaagacact ggacagaccc agctttcggg accctcccgc gtgccgaact gatcttcgag 3960
ctgcatggac ctaatcacag atgcagcctg cagtctcgcc taatggagct gccattaggg 4020
gagtgtaaaa ctgggaaatg ctgctcactg acagaatt 4058

<210> 137
<211> 4417
<212> DNA
<213> Homo sapiens

<400> 137
atttccagtg atacagctga tggaaaatgt gtccaggaag ggaataagtc ttcagtccag 60
aaacaatata gatgtgatgt gtgtgattat acaagtacaa catatgttgg tgtcagaaac 120
cacaggcgaa tccataactc tgataagccg tacagatgct ctctgtgtgg gtatgtgtgt 180
agccatcctc cttctttgaa gtctcatatg tggaaacatg caagtgacca aaattacaac 240
tacgaacaag taaacaaggc tattaacgac gcgatttcac aaagtggcag agttctgggg 300
aaatcccctg gaaagactca attaaagagc agtgaagaga gtgcagatcc cgtcactgga 360
agttcggaaa atgcagtgtc atcttcagaa ctgatgtccc agactcccag tgaagttctg 420
ggtaccaacg agaatgagaa actgagccct acaagtaata cctcatatag tttagaaaaa 480
atctccagtc tggcccctcc tagcatggag tactgcgttt tactcttctg ctgttgtatt 540
tgtggttttg aatcaaccag caaagaaaac ctcttggatc atatgaaaga gcacgagggt 600

gaaattgtaa acatcatcct gaataaggac cacaatacag ctctaaacac aaattaggtg 660
gaataatgac tcgagcagga aagcagtaga agaggattcc ttcaccacag tttcaccttt 720
acgctgtcag acaacttcct gccacagaag aagtcgttga tgtgattttt gaggaaatga 780
cagatgtgac tttggaacca aacttgtaat aaaaggaatt ccaaatggac aagcagtaat 840
gatatttaaa tattttgagt gagggggagt gggtcaaaga ggaagtagag gtgtaatcct 900
gtattaaccc tctgtcaccc cttcttagtg tcgagtgtat ttattaataa aagcttatta 960
gagtgtagaa atgccagcaa gagttaagaa agggcttttc aggaactatt ctaaaagtca 1020
taaaagggtc acagtcttaa gcagagctta gttttcttct cacttgtcaa atgcagactg 1080
tgaggcctcc agtgagaaat gggaggcagt tctgggaggg ggtttttca gtgtcaccaa 1140
cagagtcaac aaggaaacat aattagaggg ctttgaaatg atctactgaa atacctaaat 1200
tgatagaaat taatcatact caatctagga catgttcctt tactccttaa aaaagaaagg 1260
aaagatctct aaattcaaag ctagattgta aataggcatt caagaagtat tgtcttaaat 1320
ttacgatgaa tttcttgtga gcagaggacg aaacaggtga attctcacca gactcaaacc 1380
agatctcaaa tcatagtaat gctaaatcgt cttccgtgtt ctcagaggtt cttttcagtg 1440
tctgtcagaa cttggcatac tttctccata gtacgtagac cttctaatac tctgctaaat 1500
gaaaccacac ttgttatctg aaagtgtgtg aaagaaacaa tgtatgaaaa tttacatttg 1560
atcatccggc attggaaata gtttgaacta tttccaaaaa tcccttaggg gatgaggggt 1620
gaaatttaaa agctcctgaa aatgagtact gctgtgttga gcttttcttc cctgggtata 1680
ttaatttagt tatatttagc agaagaggaa tataaccaaa tgtgactaaa atatagcaga 1740
aactcaggtt gtttgtaaaa attttaataa caaatagcat ttggcaagtc tataggattc 1800
ttcctatctg gaaattctat tttgttagag tgcgatagga catatggaaa accaacacaa 1860
agtgctttag cattaaaact caccatcaaa tagatgtaat cttattaatt acacattttg 1920
tattttaatt atttcaagga gtgaagaaaa cagggtgttt ttgtttttgt agttctgatt 1980
actcatttat gcattgtttt gtttaagtaa attctgccta caagtgaaaa ggtcggtgcg 2040
ctcttctgtg caccatcctc acggtgctat ttccgaatat ctgaatattg atttctaaca 2100
cctggatgag gctgtgaccg atcaacttgg aacactgtaa aggttttcga atgggatacg 2160
ctgtaggcac gttttaagaa gtattctgtt cctaaagtcc aggtatgatt ggattaatat 2220
ttataaaatt attattagga attatttaag tggggccagg catcttgcaa cattttctga 2280
tttttttttc tttctttcta atctcatttt ggtgatattt aaacaaacat aaatgattgt 2340
aactttgcag ctttttttatt taggtagctt taatttattt atgaaagtta atccatttct 2400
gatacgtggt ttttaaaaat atgaaatgga tttatatata ctatattcct caaatccact 2460
gtatgtggac ttatattcat tttctccttt tttcggaatt gaaacatttt aatttcaaat 2520
tcaaatagaa catttaaaat gatttcatta ttattaccca tactgttgcc actcatattg 2580
taagtcagtt ttttcatcgc tggtacaatg actcagtatt tctttataaa aatctgttgt 2640
tctgaaaatc aacaacctta gaaggatttt gtcttagaaa atttccttgg ctttagtttt 2700
atatcatatt tagaaaataa taatgaaaag catccccaaa atttcaatga tgtgaatttt 2760

taaattacct ttatttcttt aaactaattc cattgattgt tacttaaatt ttccacctgg 2820
aatcatggat ttaaaatttc cccacctcat ggggaaaaaa ctaacccaga agttcaaagc 2880
ctttaaagtt tcatctgatt ctggccttag aattgtcctt aaaagctttc tcctggccga 2940
taggaggaag catatatcaa ggaggttctt tcccttccca ttatcatttt gtggcaagcc 3000
ttaagttaac aatgtgtcta ccaagaacaa ttgagttttc taaaagtaat aatgaagatt 3060
atgcaattct aactgtagaa gagatagcta ttaaaataat cagtagcctg ggcatagtgg 3120
ctcacacctg taatcccaat actttagaag gtcaaggctg gaggactgct tgagtccaga 3180
agtttaagac cagcctgggc aacacgggga gaccctgtct ctacaagaaa ttaaaaatta 3240
gccaggcata gttggcacgt gcctgtagca ccagctactt gggaggttaa ggtgggagga 3300
tcacttgagc ctgggagata gaggcttgca gtgagccgtg atggcaccat tgcactccaa 3360
ccggggtgac agatgaggtc ctctgtttac ctcccccccc aaaaaaaaaa ttcagtagga 3420
attccttcat agaataacat gttatttatt ggtattgact tcttattaga cagagctcat 3480
agtatttgtt ttctgctccg aacacttaaa aaatactaag acgtgatagt gaaaatagct 3540
ttgaaaagaa actacatatg gcaaaggtgg ggagggggga agatgatgca ttctgatcat 3600
tataaagaat acttttcagg gctctatcat tttctccctt tctctaatca tccagaatat 3660
ggcgggtgtc ccagtgttca acagtatcat gctaatattc catttgatcg gtagtccaag 3720
ttctttggcc agatagtgca atgttgtgac accgaccgaa gcctttgttc tggatctttc 3780
ttcctattga aagtggctgc tggtggcttc ataattttct gttttttttc tcacaaagta 3840
aatggtgggc atccatgttt acattgcacc ttcccgtgct gtaattggct tgacaaagac 3900
aagcaggctt ctctgttgag acttattgtg tttttagttt tcatagacac ttatttaatc 3960
tttttaaatt gtacagcaag gtgctctaag taatattcga taaaatatat ttaatagaaa 4020
tttgcgtttg atattcatga acatgaatac atgtattttt ttaagaaata agtattgtgt 4080
aacactatgg cattgcttct atagccaaag tataaaaatt tctggaatac tgacatgtaa 4140
agactacagt taattctgac actgtatctt attaaaatag gatgatttgc attttgtaaa 4200
attatcctgc acatcaagct gcatgcctta aagcggaaac tctaggactg tgttcatggg 4260
agagcagttc atctgttcag aacagtgagg caaggtctgt agtgcttctt taactacctt 4320
tggaaatact gtacaatgtt agaataattt attttgcttt acaggagttt gtcatgtatt 4380
gactttaata ttgtattttg gtaataaatt ttttgtt 4417

<210> 138
<211> 4815
<212> DNA
<213> Homo sapiens

<400> 138

attgccacgt ctgtctgcct gtcagcacag cgttggagcc ccttggtaga cttacttgat 60
gccaagtacc cccctggtgt ctgtggtcat tgtgcataga tcttggtcta aaaggctgcc 120
tgaacttcct ccatacgtgt caccactgcc accctccctc accccacccc aaacacacac 180
acaggcctta tctctaaact aaaagcctgt ctagttccac ctttcccact gcagtcaacc 240
ctcccataat tataataatt gtggccatca ctgaatggcc acctctgtgt accaggccct 300
gggtcagagc atgtcataag cgttatgtct tggaaggggc tcttcatttc tccttttttt 360
tttttttttt tgagacagag tttcgctctt gtaccccagg ctggagtgca gggcgcgatc 420
ttggctcact gcgacctcca cctcccaggt tcaagcgatt ctcctgcctc agcctcccga 480
gtggctggga ttgggtgccc accaccacac ccgactaatt tttttttttt ttttgtattt 540
ttagtagaga cggggttttg ccgtgctggc cgggcttgtc ctgaactcct gacttcaggc 600
ccacctcggc ctcccaaagt gctgggatta caggcgtgag ccaccatgcc tggcacattt 660
ctccattttt tattgaaaag gatactgaag tcagaaagaa gaagtcactt acttacccca 720
gagcacacag ctagtaagtg gcagaggcag gacttggacc ccagctggct gactccagag 780
accatgttcc cacagctggt ggggatagca ggtccagcca caaagctggt agcactcggc 840
ccctcccgtg gagaagctcc cagtctggca gagcagacac ggtgcagcac aaagcctcta 900
gctgagatag tgaagtaaaa ctaacatgta cagggacatc tgaggaggaa gggattttaa 960
gtcaaagtgg tggggccagg aaggctcctt tctggggtca gaggagactc caggaccatc 1020
cttcatgtaa tatacccatt ggtgctggtt ccttatctaa ataaaggtat cagccaacat 1080
tgagcattta ccgagctcca ggccctgtgc taggtgctta gataagttac ctcctggagt 1140
cctcaagcca gtaggcctag atactcttat tttctaccat cttacaaacg aagaaactga 1200
aggtccgaga ggggaaacaa ctggcgtgag tgcgcatgcc tctgaagtga tggagctggg 1260
atttcattta ctcacttgcc catccgatga ttttattcta tccctgccac ccgccaggcc 1320
ctgatctgga tgatgagggg ccagcagtga gcagcacaga caggcctttg cgtcatgagc 1380
cttagatctc atggggaggg gagccacagg gaacaaacag acaagagagc accagagggt 1440
gagggacact ctgcagagag cctaaaacac agggcatgat aggagccatg ggattatttt 1500
tggatttgac aacccccggg tctgtccaat gccagtgctg gagctcttgg ccactggtcc 1560
tcactgtcac tccctgctga gctcctgcaa cttttggttg aggcatttgc cccattatac 1620
cttgttttat gtacttatgt gcttttctgg gtttttttct taaggggtca acctcacctt 1680
attcttttct gtatcctagt ggcactgaat acagtgctag ataatgacag atgggagaga 1740
gctggggatt ccattggaag gggcacccaa gcccaggcag tggcactggc tgaggatggc 1800
cagttggggc cttggcacca tgatgagacc ttttagaggt gaaggctgtg ttctggtgac 1860
accagtgaag agaagggtca gccagtgcag ggccagtggg aggagcacag tgtggacgga 1920
agggctggga caggggagcc agcaggagcc cacaacatgg tgctcctgtg aggaggtcag 1980
ggcttagcct agaacagcag caggggcaca aaaaaacacc tgagaaactg tatgaagagg 2040
ctgctgctgg acttgggttg gaggtggccc tgagactttg agaatagtta tgctaacaaa 2100
aaggaggagt tttagaagga aagctggttg tgtgggagat aaaaagccaa tagccttcac 2160

tgagtgctca tcatgcatga ggccctgtgc taaaatctct gccttgtctc actgaattcc 2220
cattacaatc cagcagggtc agtgttctca tgatgcctgc tttacagacg aagacctaag 2280
gccagaggct aagccatttg ccacacacca cagaggcagt aagtggccta gacagggctt 2340
gatctcccat ctgtctgccc agtctgtgcc cctccaacca ccccaccccc aatccactac 2400
ctcctgcttt taatgacttg gatgtggaat cagttacatc tgagaacaaa cgacatagtc 2460
taggggctgc agccctgggg agaatgtgtg cctgagcccg tgaatttagg gttttgggca 2520
caaagcaaaa tttggagcta tagaagtggg gttttactaa ggagagaaga gccaagaaat 2580
tgaagaataa tctgaggtgg gagagagggc aggaggcaga agatgtcacc catcagaggc 2640
tgcagagggc aggatggata gatgagcaga ggccaaggcc ttcggttggg gagaaggaga 2700
tcttggctga gctcaaaggg tggtttcctg gaagcacagg aggccaggct ctaagggttg 2760
agaatacaag tgaggaagtg aaaggagcag gaggctctta gagggctgga gttttgaaaa 2820
tcaagaagga ccaaggacac catcaggttg agtgccctcc tctcaaatgc agaaactgag 2880
gcccagagag aggagtttgc cccagaccac ggaactaggt gttctgacac ccttttcatt 2940
gcaccatgct gcctctcttg acatgaagaa cagagaaaga ataaagtgtg aagacatgcc 3000
gtggcgattt taaggtgcac agactttatc tgttggaggc agaaggatgc ctcgctggag 3060
gtacgagagc ggtaaggttg aaagagaacc agaacattag ttcagatgtt ggctgtggcg 3120
tgtaccagtc aagggactta agtgacttca ccctgagcct cactttcatc ttctgtaaaa 3180
tgataattat accataatgt caataatatt aggttgatgc aaaagtaatt gcagtttttg 3240
ccattggaaa taatggcata taaacctgag gcatttcaat agagagagga gacataccaa 3300
acacagagct tctcaacggc tatgaaaaga tgatgagaaa gtcccagaga agaaagaatg 3360
gagagacttt tctttctttt ttttcttttc ttttctttct cacttccatt gtattataat 3420
cccagccccc aatgtgcatt gtgggtgctg gtggggcaga catgacccta tggcacctgc 3480
atgacagagg gcctcggcct ccacaaatgc tcacaacttg taaaagtctt ttcacagggt 3540
cagcatccag cctgcccact accgttgccc gttatcattg caggttaatt ttgttgcagc 3600
tattgatgtg tacgaagatg gagaagctgg tttgctgttg tgttacaact acagttgcat 3660
ctataaaaag gtttgcccct ttaatggtgg ctcttttttg gttcaacctt ctgcgtcaga 3720
tttccagttc tgttggaacc aggctcccta tgcaattgtc tgtgctttcc cgtatctcct 3780
ggccttcacc accgactcca tggagatccg cctggtggtg aacgggaacc tggtccacac 3840
tgcagtcgtg ccgcagctgc agctggtggc ctccaggtcg gatatatact tcacagcaac 3900
tgcagctgtg aatgaggtct catctggagg cagctccaag ggggccagtg cccgaaattc 3960
tcctcagaca cccccgggcc gagatactcc agtatttcct tcttccctgg gggaaggtga 4020
aattcaatca aaaaatctgt acaagattcc acttagaaac ctcgtgggca gaagcatcga 4080
acgacctctg aagtcaccct tagtctccaa ggtcatcacc ccacccactc ccatcagtgt 4140
gggccttgct gccattccag tcacgcactc cttgtccctg tctcgcatgg agatcaaaga 4200
aatagcaagc aggacccgca gggaactact gggcctctcg gatgaaggtg gacccaagtc 4260
agaaggagcg ccaaaggcca aatcaaaacc ccggaagcgg ttagaagaaa gccaaggagg 4320

ccccaagcca ggggcagtga ggtcatctag cagtgacagg atcccatcag gctccttgga 4380
aagtgcttct acttccgaag ccaaccctga ggggcactca gccagctctg accaggaccc 4440
tgtggcagac agagagggca gcccggtctc cggcagcagc cccttccagc tcacggcttt 4500
ctccgatgaa gacattatag acttgaagta acagagttga atctcatttg ccatctttag 4560
ttttcttatg gaggtttata ctctttaaac agttctgatg taatttctca acaaaatgtg 4620
gcttttagcc tgtcagtgat ctattggacc aaaccttctg cacactcggc cagttccctc 4680
tccaatgtcc ggtgccatct ttcctgacct ttgtttcttt ctgttcagga accatcagtc 4740
cccttgtaat aaaggtggta gatttcattg aggttttaga ttgaaacttt gaataaatca 4800
aaaatactca ttctt 4815

<210> 139
<211> 3867
<212> DNA
<213> Homo sapiens

<400> 139
aaggggccct ccgttctgac cactctgttg caagctgagg aagcagttct gggaggttgg 60
gtgacttgcc tcaaggcctc cggtcagcca gagtcagatg cagaaccctg ctcagtctgc 120
aaaggctcag ggccccagag ggaggatggt ggaaggcaca cggccccagg gacggcgctc 180
agaggcacag cgtggtgtgt tccagggctc cagggacaga ggggcagctt cgctggggag 240
ttcctttggg gctctcatgg aaactaccgg aggaagagga aactgagggc cagtggtgag 300
gcgcctgggc cgccgagctc agctctctct gggcacagcc tggagtctgc agggaagtgg 360
gagaacccag gcaggtgctg gaggaccagc caggcagtgc agaaccagag ggcggggaga 420
ccggccgggc agtacaggac ccgggggccg aagggatggc atggctgggc agtgcagaac 480
cagagggcgg ggagactggc cgggcagtac aggacccggg ggccgagggg atggcatggc 540
tgggcagtgc agaaccagat ggctggggga ccagccaggc agtgcgggac taagagggcc 600
agggaatgga gctggaactg aagaacaggc cagctcagcc acggggccca ggagagggtt 660
gagccagaca gagccacaca tttgggcgca ggcaccaaga gaaggaggtg caggtgcatg 720
aggctcagcc ttctgcagat gagacttagg gggacgcgag ggctgggcag aattgggaaa 780
gctgagcagg agacgtctcg cttttatgtt tggctgaaac atgaaaacac gaagggccag 840
tgggaaaggg acctcacaga cacgttgggc ccgcagtgtc tcttgtctcg ctcaggcaag 900
tttcaggctg aaaaagtgcc cagaattggg tgaggtggga ggagggaagg ggagagggag 960
cctgtctgca cctttctctt caagggagaa gttgccttcg gtcgggcagg agcagggcct 1020
cctctgctgt gtgttgttta ttttctgtgc ctttccttcc cctgagctat tctggtgcaa 1080
gagaaacagt gagggatgtg gggcgtgagg tgtccccagg ttcccgggcc ggtctgcagt 1140

gccctgtccc gtgtaagggg ctgacgacga acgctggttg gagcagtggt ccgggggcgt 1200
ggctgacccc acccaggcag ggcacaggcg gaccctggtg tcgatcgcct ggtgggagcg 1260
tgtggacagc atgagacccg ctggtcttgg ggaacacgct ctgggcatcc gtgtgggcag 1320
cactgcccac catccttcga tggtctcctc gggggtcttc gtccctgtgc atggagccgt 1380
cctcactgag tgtaccagct cacaggtctc ccaccttcaa ggggtctccc accttcaagg 1440
ggtcaggctg accctgctga gccagggttc acatagcctg gctgcagggg ctcctcaccc 1500
actcctgggt ctgatgtcct cacctgggct gtcctccgct tctcctcatg gtcacgcaga 1560
cacctcaggc ccattggatg ccgcaaaatg tcgttcaggt cccacctggc cgagggctgt 1620
cccttctgca agctccctcc ttggtcccgg gtgctggcac cagcccatcc aggcccatcc 1680
aggccctccc ctccctccaa cacaggcctt gctggcacac atttaggggt cagaccacgc 1740
cagcaggagc tggatgaggc cacagcgagg gagctggcag caggcggccc ctgagaatgt 1800
gggagagaac acaggagaga gacgggggcgg gggggccac agccctgtcg ggcagctgt 1860
gtcgctgtca ggatgtgccc gaagcactgg tccctgagtc cacatgtttc tgttcaaact 1920
cctgtttgga aattcgggag gggatcaaag tcctgccctc caaggttccc gttgcagaca 1980
cagctccacg gtccaggctg gcccggcctg cgcacttgcc tgcctgattc tgctccctgc 2040
tgccgggcag tcactccttc atctcagtcc ctgggtcctc tccgtcctct gtgcgcatct 2100
tgctcacagc ctggttgctc catttcctgc ggcacagctg ccctccccac acagtctgtg 2160
gtccccccctc catctcctct gctctcgctc ccacccactc tgtccttccc tcattcctct 2220
gctcgtccag cccccgccag tgtgttctgc cactccctat cctgggtttc caggggccca 2280
tgtggagggc tgcaggggtc tccctgttgt cctggaggca cctcccagag catcatccct 2340
gcccagcccg caggcccaac atcctccgtc cagaccctga ggggctgtcc ccagaggcag 2400
caggtcccac gcgtccgttc ctagcgcctg aagtccaggc acatgcagcc tggccttttg 2460
gcagtggggc tccctcgtag tgttgagtag gatgctgggg gtcccccgat ggagtcaggg 2520
tgctgcaggg ggtcacacgc tgtcccgtgg aatcagtaat cctcatgcaa gattcttcca 2580
aatgcacagc aactgcgtga gacagaacgt gcgtgtggga agggcagcga tcccgtgcag 2640
tgtcacgcgg cccctcggct ccctccttgg cctgtgcttc cgtttctcct gtttccgagg 2700
tcaaatgatg caaagtgtcc ttgaccatga gcccacgggc atgggacaag gtctctgtgt 2760
ttgcactgta agccacggaa ctgagtcaat gcgtaactgg ccctgtcccc agcccaggca 2820
ctttctgaac ccaaaataga ctcagaggga ccgaggcagc tgccggccct acaggagcaa 2880
gggggaggag agcctgggct gtgcacctgc aggagggatc ctgccagacc cccgtaaagg 2940
ccaggccccc ggaagcagca gtaccagcag tcagtgcggc ctgggagggg agctggctgc 3000
atgcttcacg catccccgct ggaccagtgc atgagcacct tttgggtaaa aggtgcaagt 3060
ggtgtgtgcg tgttccaggc agcatgagaa ggtttgccct gggtgagccc agctcctgtt 3120
ctcctccacc tgttcaatat tcacaatttg gtgtgattgt tactcagctg cagacggcct 3180
cctgaacgcc attgaccgca catgcttgtt gattctgaca taggtgttga ggacctagcc 3240
acatcgtgga ggcccccatg tgggtgtgtg tgctctgcac tgatctggga atgactgcgt 3300

gtgccccatc acacggatgc tccaggatgt acctcagcag tcccctgcct tggggctctc 3360
gtgctgctcc caaacctctt ctgtcagcac agacgctgcg tttgcatctc ggtcctgccc 3420
cggtgccctg gcactggttg tgcagggcag gtaggtggga tggcagcaaa ctgcggtggg 3480
caccgcgtcg caccaatgtg cacgtgttca cacacagata ggactcggaa ttgtggagcc 3540
atcgtattcg tctttccctt ttaacaatcc ccccttgaag gccgggcgcg gtggctcacg 3600
cctgtaatcc cagcactttg ggaggccgag gcgggtggat catgaggtca ggagatcgag 3660
accatcctgg ctaacaaggt gaaaccccgt ctctactaaa aatacaaaaa attagccggg 3720
cgcggtggcg ggcgcctgtg gtcccagcta ctcgggaggc tgaggcagga gaatggcgtg 3780
aacccgggaa gcggagcttg cagtgagccg agattgcgcc actgcagtcc gcagtccggc 3840
ctgggcgaca gagcgagact ccatctc 3867

<210> 140
<211> 3419
<212> DNA
<213> Homo sapiens

<400> 140
gatcgcaaac ccggaagacg tgttcgggca gctggagtgt acgggcccgc gggccacggc 60
catgcagccc ctggaggtag gtctggttcc cgctccagct ggggagccga gactgacccg 120
ctggctgcgg agaggcagtg ggatcttggc gcacctggta gctttgggct tcaccatctt 180
tctgacagcg ctgtcccggc caggaaccag tcttttctcc tggcaccctg tattcatggc 240
cttggcggat gtatgagagc acctcctttt tctcaggcct ccaagccaga catgaggctt 300
actggctctc tcctatgttc acagttctgc ctctgcatgg ctgaagccat cctactcttc 360
tcacctgaac actccctgtt cttcttctgc tcccgaaaag cacggatccg gctccactgg 420
gcagggcaga ccctagccat cctctgtgca gctctgggcc tgggcttcat catctccagc 480
aggacccgca gtgagctgcc tcatctggtg tcctggcaca gctgggtggg agccctgaca 540
ctgctggcca ctgctgtcca ggcactgtgt gggctctgcc tcctttgtcc ccgggcagcc 600
agggtctcaa gggtggctcg cctcaagctc taccatctga catgtggact ggtggtctac 660
ctgatggcta cagtaacggt gcttctgggc atgtactcag tatggttcca ggcccagatc 720
aaaggtgcgg cctggtacct gtgcctggca ctgcccgtct atccagccct ggtgatcatg 780
caccagattt ccagatccta cttgccgagg aagaaaatgg aaatgtgagt tcctgcgaac 840
gctgaatcta ggtgggacgc ttgccttgaa catcatggtt cctttggtga tctataaggg 900
atctatttaa gaagtggtca ggttttcgca cttcttggct ggtccaggga ctgcagaaac 960
caaagctgct attgttgagg aataattcag tgggtcaaaa tggggagatg tactgggtat 1020
gagtggaagg tgatggagag cctgatcctg aagcctctac ttgatgagag acagagtttt 1080

gggtggtgat agtgatgtgc tggtggtcat ttcttgcttg tgtgcctgat gaaaaactgg 1140
gttcctgtaa gttatgaatg gcatccaggg atatttgggt tacttttaag aaagcagtgt 1200
gatgtagtgg agagagccca tgggtcttat ttatgggata tggtcctctt aggctctgtt 1260
gtacaacctt aggtacattc catatcttaa gaccactgtt tcttcatctg tgaaatgttt 1320
ctaaacaatc tctaagtccc ttccttctct aatatacagc gtctgtcagg tcgatgtctc 1380
agaacactct cccagctgtg gaccacgtgg accacttagc agactcaggg ggtagttctt 1440
tactctccct ttactaccct ggagggacag ctctgccctt gaggcccttc agaaatttgt 1500
gctgatttgg tccctgtgcc agggcacagg gggtcaggca ctgtggaaag aagggactca 1560
ggtgagggtc ttctggactc taagacggta aaggcactaa ggtcacttta aagcttttgg 1620
agaagcagga gggcattggc ttaaccaagc cgaaggctgc tggctgggct gcctagccca 1680
gctaagatct tccttcagcc cacctcagga tccgggcttg agggctgcag ggcctgcgtg 1740
cgctccctcc cccgaaatca cttctcaggg caaaggagcc ccaggcatct catgctttgc 1800
ccttcttagg gctcaggttc ttgccttagc acatagcttc tgggagcttt tttgaagcat 1860
ttcataaggg ccaaggaagt ggggcagggc ttttctgatc caaagaaaac aaagtttctc 1920
tggttacccc tcttcccttg ttatctaagc tttcctcagt tgtcatctct tcccagctct 1980
ttggtccaag agggggctga gtttggtgcc agctggcaaa tgagggctgg actctcttcc 2040
ctccagagac caccccgctc cttgctgcag ctgaaagtag ttccccaggc tgccctcggt 2100
ggtgaggact ggatgctaga ctgctgagct gtggtctggg ctcagttgag aagataggat 2160
ctcccttgat catggcaagg cctgacaaca gctgagccag gaaagtgctg ctgaggcaag 2220
tcgacatagc tcacagggaa ctctgggaag cctggggtgt aggcgctgga gctccagttc 2280
ccaggagcag gggcaggtgt tcctagatgt tagtgttgtg gatgtccttg gtcttctgaa 2340
gattcaggtc ctcctctccg ggtagcttag aagtaaggag gttggtttga ttcaatagta 2400
caagaatggg gacagcaagg gcagaagtgt cctctccacg taactcatta tgcccctcct 2460
gggggagatt gcatctccag gacaagcatc taaaggaggc ccctgctgtg cagaggggta 2520
ttgttcctgt ctctttcatt gtcctccctc ctctcaaact ctccagtgtg gtgtgaactc 2580
agaagaaacg gttactgggg ctgcatggag aatttcacct gcggtgattt tgatccaggg 2640
actgcatctc ctctttcctc atcacagcca gtgctgagag gctccttcta cctgccgcag 2700
ggtaggaggg ccaggcaaag ttcaccagct tgctctaaga gcaagcaggc aatgccgtaa 2760
agcctgagcc tgcctgaggt gctgcctcct cccaggtggt gcgggggctg gtgggcgggc 2820
gggcaggcgt gctgacagcc ggcagtttgc gtgggctgtg ccatctgatg tctattccca 2880
gccctgggag gaagggggag tcatttatat tctgcaggag gaagggcccc cagctgtcgc 2940
ctttctgacc agcaggcctg gagggcaggg gcacagagca gagaggaggg cactggtggt 3000
ctcctgctta gcctggtctg actgcagtgt agggaatagg tcaccaggag gagcccttca 3060
tcctggcagg ccggattgtg gagggacttc cctccccctc ttttccattt ctccctccat 3120
caccacctgc cctcacatcc tggggcagca gctggacagc cattagacct cagtgccagg 3180
gcactcttcc tccagctggg atctcagtgg ctcccagctg catgggctcc tgcttgtgtg 3240

ttccctcctc cgccatcctc tgttccccgc acctccctta ttctgctcat gtctggggta 3300
ttcacttggc tcctcagcaa ggaagcaaac gccttggagg agaagcacat ggttgccctt 3360
ttgtccttgc tccctggcta gggaaagctg ctagtggtca gcctgttttg cctttttt 3419

<210> 141
<211> 3691
<212> DNA
<213> Homo sapiens

<400> 141
   cagaacagag ccgcatgttt tctgattgtg aaggtcttca taggtgggtg ggtgagtatg 60
   ggaagcctga aaggagacct aggctatggg actaaactag ctgccccagg cagaattgtt 120
   aagtgagtgc cttccttatg agggcccttc acttaccctg cctcttgcct tcccttcctc 180
   tcctctctgt gcccaaagcc ttcctctcag atactccctc atacctttcc tatcctgctc 240
   tctgcctcaa aattacccca tatttaaatt tcctgcttta ttgtaccatg gtctctagtg 300
   atgatcaaca gaaaggcaac catatggttt tggttaaagt aattcagact aagatgttag 360
   tgactgaaaa gctggggtca gtagcttggc agcttgcttc tgaaaagtta cacacctctg 420
   ctctctacag agtcaccaac ctgtctgtga ttgccaaaaa cagcagactc ggtgctactc 480
   ccatacaatt atgaagaaga gattgccaac agcatggttt cctaattttc attttcacac 540
   tgaactgcta gactacattt gacatacact ggtgacattc aaaggtatag ttctggtaaa 600
   ataaaattga acatatggtg gcaccagcac tgagagcttg gttcttttcc tgatcagcag 660
   tttggctctt catcagttaa ctgcctgggc ctcagtttct cagctgttaa attgaaggag 720
   gtggatgagt tataacgttc tttctagttc ttacacagaa tgagtttctt gagttccaat 780
   atgctggaga agaaaaatag aagagtttga ccactaattt ataacagaag tagtatatac 840
   caggacacgt gataaattat agacattttc tgttagggag acttgtctga agactagttt 900
   tattactttc atttcttcct caaagatcct ttcataaaaa acaaacaaac aaaaaacaaa 960
   aaacaaaaac aaaaaaaaac caaggctggg cgcggtggct cacgcctgcg atcccagcac 1020
   tttgggaggc tgaggtggac ggatcacaag ttcaggaaat caagaccatc ctggccaaca 1080
   tggtgaaact ctgtccctac tgaaatacaa aaaactagcc aggtgtgttg gcgggtgcct 1140
   gtagtcccag ctactcggga ggctgaggca ggggaatcac ttgaacccgg gaggtggaga 1200
   ttgcagtgag gcaagatcac accactgtac tccagcctgg caacagagca agactccgtc 1260
   tcaaaaaaaa caaaacaaag caaaacaaaa aacctttcac taatttcttc ttggcttgtt 1320
   cagttcctgg tatagtgtcc gtgcttctct ctccccactg tgaatctcaa atgtagtcct 1380
   gtgagtcatt aaatatattt ttattattag aattacagct tcatgtataa aatctgaatc 1440
   cttttatata acagttgtaa tgacattctg agtaacaaag tcaacatctt gtacagattc 1500
   ctttctcatt agttatcaca ggctggtggg ggaaaacttg tttcctctgt tatatattgg 1560
   aacaattcaa gacagaaggg aattgatttt attgagggtg tcaccgtact gggacatttc 1620
   tcagaaaacc ctgagactgc tttgggtttg ggggagggag aagaaagagg attttgtttt 1680
   aaagtcattt ctttggggta ccgaacacca catcccatca ctcatcatta atagatgttt 1740
   tgcccttttg gattctggct tttaactgtt ttggattctg gatacttaac tgttcccagg 1800
   caagtaaatt gatctcaata ttctggttat ttattgctac ataacctcca agcttagttg 1860
   tttaaaacac agttgtttat tattatagtt cacagttctg tgatttggcc aggctcatct 1920
   ggacagttct cacttggggt ctcatacagt tgtggtgaga tgtagttgga gttggactta 1980
   ccggaaggcc ctctgggcag gatgcctgag atggtatact catatgactg gaagctgatg 2040
   cttgctggga gcttagccag agttgtcact ggagcaccta cctacacgtg gcttcttcct 2100
   gtctctaagt cttaaagaag cttccagcca gttaaggttc cacttagaac tggcagtgcc 2160
   acttttatct attccattgg ttagagcact catgcagact gctgaatttc aaagggtgaa 2220
   gaattagaag ccaccttttg atggagaagt gtcaaggtca cattgcagaa agtcatgtag 2280
   gttgtggcca tctttgaaaa atatagtcta ctgtattaag gcactgagcc ttaatttctt 2340
   gtttataaag gaaagataac attaatggtg ttgtagtttt aaagattcag tgagataatg 2400
   ttttattcgg cataatatct caatatttaa caaatagtga atatcattac tgttgttgtc 2460
   tccagatttt atactggttc catacagggc tatagaactt ctagaatgcc ttagtctagt 2520
   actttggcat cattactact aggtaatgat aataccctta agatctgcca agaagatgag 2580
   gcaaatggat tcaatagatt ttatagtgac ttgttaggaa agaactcaat ggaaacggtg 2640
   gaatgatttg ggagaaccaa gtcatttttc tgttttcttt gctacaaagg attagaaaag 2700
   cctcagttac ttgaggccaa ctttctcttt tgatatgctt tcagatatta ccttgtgtct 2760
   ttaagtgcaa gtttatccag ggttgatggc aggatcttgc ctctaatttg cttccacagg 2820
   gcttaattca gagaggaggg agactcagga aaccttgggg cggtggggga gggggggcat 2880
   caagtaggaa gctttatctt atgattcact ataaattgca agatgttctg gaatgatgat 2940
   acatcagcag tttataagtc ataatttttg ttttaatttg ctagacatct gttagcttcg 3000
   atggcaacca tatgggaatc taaattgcgt tttgaattac agggagatgg taggaagaaa 3060
   gctaaatatg gttatgaaag tgtcatatgg gaaaagtgta agaaaggtat aaagtgggga 3120
   aagtaattga atataggctt gcttagtgtg gccactccct ctgtggggca gtatgaaggc 3180
   gcagaagcat tctcacttga tatactccgc agattgaaat gaagccaagt ggggagaagt 3240
   tggaaaacca aatcatgttt caaccaccat aaacttgcca aaatgttgca ctcatttaga 3300
   tgggtcactt tgttttaatg tccacagtaa taggtagtcc ctggcaaaag gtgaaagcaa 3360
   tctgcatttt taacagaact tttcactaat gatgtttttc ttgtaagcac cccaaagagt 3420
   cttccaagaa tattatatct ttgtgacaga tgaagaaatt ggagtacaga gatgtggagt 3480
   aacttttgag gtgttgaaga gcatgtcaag gttcagtttc agagtgttaa gtctcttccg 3540
   taatgatagc cccagctttt tgggtggaga cttattttag aagatgcttg ttattctaaa 3600
   ataaaacatc aagtaagtca tttacatctg ttcataatcg aaaagtgata aagtttttgc 3660
   atatgccaca attaacacca cccacaagca c 3691

<210> 142
<211> 3727
<212> DNA
<213> Homo sapiens

<400> 142
aggagtgatt tctgaaattg tttagtgcag tgaggaaaag acagtgggat attacaatgg 60
agagacgctg ggcattgtgc agcaagacgg agaagtcagg gagggctgag ctggatggaa 120
gtggtgggaa ggagccagcc tgcagagaag cagagcgtgt gctctggcag agggagtggc 180
agggcaaagg ccctgggcag gagcagggtc agggtcacag ctgtgtggct ggagttcagt 240
gggccagggg gagagtgatg gaaggcaagg tcaaggtggg ctggggccaa accctgcagt 300
gctttgtcag ccatggcaag gatttggggt tttattctaa gtgcatctca gagtgccggc 360
atctttatct tggggtgatg ttggcatatt cccaccagga taaattaaca ttctcacctt 420
aacagatcat tgggtagaga ctagaaatgt accgagcagt gcgcaaagaa gatgaggagg 480
gttctacagt ttctccagat acataccatg acaatttgtt tttttaaaaa agaagaaagc 540
ctgcaggtaa gtacagccga aagtaaagag ggccacagta gaggagggaa caatgccaca 600
caacatgaac agcaaggagg gagcattcca ttctgagcag agggacgcga ggaggtgggg 660
cctgaaagga cacaggaggc actgaccccc cgaggcaagg gagatgtgcc ttctctggga 720
gacagcggct tgggatggtg gtggggcagg ttccccgggg aagacctgga ccaggggctg 780
ggggtgggag ttggagagga gagtgcagtc ttgggagcag atttaaagtt aagtggcctc 840
catggacccc acatggccca gtcagtttgg accctcctgg gagatcctga gttctgagcc 900
taggcggtct tgtcctgcct ctgctgagca cccgggccat ggcagccatc aggatgcagg 960
aaggtggggt cccctcccag cagctgggaa agcatgaagg aaattcattt ctctgcagca 1020
gctatagtga tcgtgaggag aagtaactga aaaggtcaga cacacacttc acttacacag 1080
ctcataagtc agtaaagaag caaagcctga gacttcctca ggaggtgagt aaatcctctg 1140
ccttgccaag gcctgcaaag ctaggcttgg ggccggccag cctaggggag tggcttctcc 1200
tgctctagct cccagtgccc tctggcttgg ctttctgaag agagggctag aggagctgtg 1260
cctctagagt tcaaaagaaa agtaccttag ggagggagta tagggttgca gtcaaaagaa 1320
tgggctctgg actcataaaa acatcagttc aaattcagac tcataccact tatttgctgt 1380
gtgattttgg gccactcaca caacctctct gagctgtcgt ttcccctcca tactgtaggg 1440
ctgataatag tacccaaacc ataagtcatt gagaggattc aatgaagatc atacaagtaa 1500
aagagttaga tcaatgccta ggttcccact gcatattggc tgtggttgtt atctttatca 1560
tttaaatcat ttattattat taacacttta attgtacagc ctccaaaatg acccctgtga 1620

tcccttcccc ctggtattca tgcctgctat ttaaccagaa tagggttgat ctgtgtacta 1680
ataggagatt gtggggacag cagaatgtga cttccaaggc taggtcctga aagatgctgt 1740
gactttcgcc tgagtctctt ggatcactcg ctctggggga ggactgctgt catgtcatga 1800
ggacactcag gcatccccat ggaaaagtct gcatggtgag cacccgagac ctcctaccca 1860
cagccagcat taacttacct gctctgtgag tgaggcagct tggaggaaga tccccccacc 1920
ccagataacc cctaaatgac agcagctccc accaacatct tctctccaag ctcatgaggg 1980
agtgtgagtc agaaccacct agctaagctc gtgaattcct gacctacaaa aaccgtgcga 2040
gatcacaaat gttcattttt tttattgaag ccattaagtt ttggggtgat ttgtaatgca 2100
taaataatta atacaattat tatactgaaa atttctcact aggaagtggc cactcattag 2160
gcagccccag ccttcaggct ctgtccagcc cagtaaaagt cagtcatttt tgtgccaaag 2220
atccctgaaa gagacagcat catgtttggg tatcacctcc tccagagagc cttcttggcc 2280
aagtcaagag cttttctcca tgtgcccaca gaaaggctct ttgtaatcct ctaccacact 2340
gcattgtcat tctggaccca atcatgtttt catctaggct ttggctacta ggaggctcag 2400
acccaaattt tcaggccact gcgaagactc tcaatttatt cttgccctag tttactcatc 2460
tatttgcttt gtcctgtttt gtgtgtccct tgtgtaaatt atcacacatc ctttgtgaaa 2520
tgaggcggca tgaagcaagt ccttgctgaa ggtccagggc atggcaagag aacctgagtt 2580
taagaagcag acatcacctg ctggcagcag gcagtatcct ctcagtcagc cttgatgtcc 2640
ccttccctgg atcctcactt ctcatggcaa tctccccgtc tcctcaggtg gggatgggat 2700
gaacaggttt gattagccct tcactgtaaa tgcctgtcct ctgtcactgc accaggactg 2760
atatggttta gttgtccatg gctatcatcc aaacttgaag gtggcacttg taaggaaaaa 2820
acaaaccaac aagctatcct aaaaggaggc tggcagcatg aggaaggggc ttgccatccc 2880
ctgtgccagt gcgggaagac cagcccaagt gcccacccca ctgcgggagc agactcagct 2940
gtccccaaac ctgaatgcag gtaacaaggg cagcagcctg agcatctcag agcccagagg 3000
cagagcgtta gccgattgct tccagcatca tctggggcac agtggggtct tggttcctca 3060
atgggcctga gtggatctaa ctctgcgaag ttagatccca acagccatca cagtttgcag 3120
acaatgtcat taagaccatc cagataactt cctaactcca gttttgtgcc caccaagcat 3180
ccttctgatt tcaaattggc ctcgcatgcc atgtgcaact gggagagagt gtgtggacag 3240
aaatggggcc aattgactat ttcccttggc tgtcatattt ttcattaata aactaactct 3300
ccagccacaa atacacactc agaatgcctc ttgctactcc agatcctcca ttcactgtga 3360
aggcaatcat ggggattatg aattccatct cccaggtgtg gattaaactg catgccaggg 3420
gaggtttctg tggttccaat ctaccccgct tagtacatca gagctcaaca ggatcaggtc 3480
aaagctggaa ggatcctgag agcccacaga aataatgact cctgtgctga ggttcacagg 3540
agtagcactg ggtctgtga attcttggca aaaattcaga aaacctaagg gaatccatgc 3600
attagctgat aatgaggcca tacagactaa ctaaagcatc agccacctca ttaaactggg 3660
aagcttaata ctgtttttat tgcacaatca tttctaaatg tcttttatta ataaaattgg 3720
ggaaatg 3727

<210> 143
<211> 4118
<212> DNA
<213> Homo sapiens

<400> 143
ttagcagaag atgaatttat taaagggacc ctggattgct catggaaatt ccaggggagc 60
tacagagcca ttgtggaagc ttcccagcag ggacaatggt cagaacctgg ctgccgctgc 120
tgccacttct ttgaccatgg cctggatgct tcggcttgca atgcttaccc actggggctg 180
gatgctgggt gctgctgaga actctacctc ctctgtgatc ttgctgtggc caccactgtc 240
cctacaggct ctagggtccc tgcttcctgg cattactggc tctggattga aagcctgggc 300
ctctgatgaa tggaacgtgt gtgcatgtga aactgccttt gcaaagatga tgacagtgag 360
ggaagtctag catgggtgac tccatcttgc ctctaccctc acaggctggc tgtcctcact 420
cattcctggg cataggccaa gctaaccatg ggaggaattt agtttacagt ttaactctga 480
agcaaagatg atattagtcc ctccctaaaa tgaaccccct cctagctcag ggacctgcct 540
ttgtaaaact aatgaaaggc cacaagatta ggattatggg aggggcttga attttgctaa 600
gatgtgggtg tagctaaccg atgaccagcc attgacccct agcttgcctt tctataatcc 660
tttgctgctc aggagtcatg cggccagagg tcacaagatc tgtgacttcc ccaattgctc 720
ctatagataa catcactatt gtaaaaccaa atattggtct tctgagatat ttttcagact 780
tttgcattct gaaatgattc atgactcaac tggtcctgtg acccccaccc agaggtggac 840
ttagtgcaca aggtctgttt tctacactcc tatgatttta tccccaacga gtcagtagca 900
cccattctct gctcttaccg cccaccaaat tctccttaag aaccctagcc tctgagttct 960
tgtggggact gatttgagaa ataaactccc atcctttcac ttggctagct ctgtgttgtt 1020
aaactctttc tctactgcaa taccatggtc tcagtgaatt ggttttgtct acgcagcagg 1080
caggaagaac ccttcgggtg attacaaatt agcctgctcc agagctgctg gaggtggggg 1140
tgacacactt cttgtgcctg tgtcttcttt cttgggaggt cggctctgtt cccaagactg 1200
atatcatggg gaactcctct aatgtggcaa agcagtttga atgctgggca gccaggggag 1260
tcacagatgg ttgcatacac atctgtgttc tgcatgacac tgctagacat ggcggacaat 1320
accaagacgg atcagaccca gcacctgtct tccgagacct tacgggctca tgattctctt 1380
ccttttaaca tgcacattta ttaggtacca tagtgtgcaa agcttgttca ctcgaattat 1440
ttcattaggt ccatctgata gtcatgtagg gtaggcggaa ctgataggat ttctgtttta 1500
tatttgaaga aactaggtct gtcaatagtg agccaggttt aaaaaaaaaa agaaactagg 1560
aagttaggtg atgagcctaa gacatcacca ctggtgagga gcagagctgg gacatgagcc 1620
tatcttctga ctccaggccc agtgctaagc caactaaagg ggcataaagg ggagctgaga 1680

tcagggaagg ctctgtggag tggcactgcc cctcagtccc ttgtctgcaa ttctaaaatc 1740
caaaggactc tgaaaacaaa gtctctgtct tttttttgta atgggatctc attctgttgc 1800
ccaggctgga gtgcagtggt gccctctgca gcctcgacct ccctggcaca agtgatcctc 1860
ttgcctcagc ctcctgagta gctgggacta caggcgtgtg tcacaatacc tggctattgt 1920
tttttgtttg tttgttttca agtttgggac taaaactcaa tttggcagca aaacctgacc 1980
cgaactgatg tgatttgtaa tctttagtta tccatttacc gtaagatcaa tgtgtttagc 2040
tgcagaaatc ttaatgtttg ttaatagggg gttatcctgg tccataaagg gagggagagg 2100
aggaggtgtt acctaatatt gggtccatgg actacatagc ctaaaaaaat ctataaaatt 2160
tcaaaacaca cctggcttca agggttatgg ataagggagt ctgggttgga tctgaaaggg 2220
ggcttggttg tggggtagaa tttcgatgtt tggaagggat gtggagggat gttatgaaga 2280
ggaagcctca cgccatacca ccttgggtct tggtgttaag tcattatcaa ccattacatt 2340
tgctttaaag cagagatgac ttacatttct gataactact ttcccttaat tcatgattca 2400
cacacatttg gagggctgga ctttctgggt ttggttattt cccctggtac caatccccgc 2460
cttttcccag aaatcacttg actttcccga aaactcagaa acacaccctt tggctgaaaa 2520
atagcattaa gcaatcggtg aaattcccca ttcctccttt ccccgctgga gccctggata 2580
gagggctctc tggcctggga ggaggacgct gtccttgttt caaaagcaga gggagctcca 2640
agatcaaggc tgagtttccc taagtcatgg tctcccagcc ccctctgttt agatcaggga 2700
atttcagaca tgcacactcg ggtagggaat cttatgaaca gaaccaggac agggaggctg 2760
gccggaggtt cctgcagagg gagcgtcaag gccctgtgct gctgtccctg ggggccagag 2820
gggttgccca gcatgcccac tggcaggaga gagggaactg acccacttgc tcctaccagc 2880
ttctgaaggt aaaatcctta caaacgagga gcagagcttt gatggaggaa aaaggaaagg 2940
cagaaaactt cacgtaggag ggcttggggc aatgatagag ggctgacaaa ggcagcataa 3000
aaaaaggcag aatatagaca acctatttct cttaggctca ccccatccac ccctacttca 3060
ccccccttacc taccactgtt ttcagggtta aagcaatttt agcaaccctt tactaggaac 3120
tgtggggaga tagcttgtat tttcagtaag ttggcggtgg ggggcaggga caaactccaa 3180
acaaactctg gaaaacgaat gtaggaagtt tttatgttcc ttgtcatttg gctgtccctt 3240
ttctccttcc tcgcacaacc ctgtatcagt ggggcagcac ttccttctca gaccgcttgt 3300
cccagggtcc ctgccttccc ctagtgaaag aagctcctgc ctccatcggt gggcgttggg 3360
agcatcagcg gagatgcacg caatgataaa cagcagacag ggctgcttga ggagatgagg 3420
tgatcacggt taaagaagag gggctgtcct acatggatga tcatgatgcg ccaagactgt 3480
tgaaaacctg agtggaaatg ggagcatcgt tcttgagggc aggagagaga ctgataccgc 3540
gggggtgggg ctggtggagg acaatagagg aagaagaaaa ctgggagaaa gtactcgatt 3600
cctttggaga aaaattagga gaaagtctag agaagcaatg agggaaggat acaatttagc 3660
tatagtctag aaagttagtc gaagttaact agggagggtg tgaggggcac ggtgccatgt 3720
ggggatgttg gtgatatggc cctgtgtgtt atacctttga aggtgacact gagccccagg 3780
tgacgccgca ccaccaaaga aggtgcttgt gtttgtcaga caaatacagc caggcctgcc 3840

accccttagg ctccaaagtc cggaggtgca gaaagccagg accaagagac aggcagctca 3900
ccagggtgga caaatcgcca gagatgtggt aagtgatcaa gggtccctga gatgacacag 3960
actccattcc cttcatcttc tcaagaaaag cgctcaaggg ccaggcatga tggctcacac 4020
ctgtaacccc agcactttgg gaggctgagg cgggaggatt gcttgagccc aggactttga 4080
gaccagcctg ggcaacataa ggagaccctg tctctatt 4118

<210> 144
<211> 3379
<212> DNA
<213> Homo sapiens

<400> 144
cgtttgctgg gggcttgtac attcactcgc ccccattcac ttctctccct tcgtctctct 60
ctacactctg ccctctcgtc cggccgtctc cctcctttgt cctctgtcct gctccgtctc 120
tctccccctg tccgcatggc ctttcttgca ggggagcctc cacctcgccc acctcagcct 180
gccttcttca ctcagagtaa gtggggctgc ttccgatgcc ttggcccccg cccccgcccc 240
ctctctctca ctcctctcct ggaaggtaca gagcctgggc ggctgggccg gaggggaggt 300
gggtgggtgt gtgctgtgag cttccgtggc tgccctggcc tagcatgcct gccccccctc 360
gagcccgccc cccggagcct gggcttctgc tcacgagtgt ggccctggct tccgcctcgg 420
cctcactccc tcccagacct gtctgagctt ctggagcacg agtgtggccc tggcttctgc 480
cttggcctcg ctgcctccca gacctgcagg cgctgtctgg gctttgtggc cgttggtcag 540
gagtactgtc ctctccaagg atagtcctta tagcccctga tggcaggagg cggctccgtg 600
cctgcactaa tggtgtgccg tgacaggggc ggggatgagg gctcactaac tcgccagggc 660
gcaggagcag ggccgccggg gtcttgtgct ggtggaggca gcgtcaggca ggagccagtg 720
ttgcttgctg cagcttagac ggagcgaggc ggggctcacc ggcccacatg gggctgccgg 780
gcagagcagg ttccgccctc ctgatggagt ggtcggaggt gcgctgagaa cctggccgcc 840
ggtggctgac ctcagacctc ggagggagtt gggagtggag ggttgctggg ggacacttgg 900
ctttctttcc cacgtgccac ctcccctcgt gtcccagacc ctgcctgact gccttcctgt 960
tgggctctcc ctttccatca cagaaccaac ctatgaccct gtgagcgagg accaagaccc 1020
cttgtccagc gacttcaaga ggctgggcct gcggaagcca ggcctgcccc gagggctgtg 1080
gctggcgaag ccctcggcgc gggtgccggg caccaaggcc agccgaggca gcggggctga 1140
ggtcacgctc atcgacttcg gtgaggagcc cgtggtcccg gccctacggc cctgcgcgcc 1200
ctccctggcg cagctggcca tggacgcctg ctccctgctg gacgagaccc cgcctcagag 1260
ccccacgcgg gcactgcccc ggcccctgca ccccacgcct gtggtggact gggacgcacg 1320
cccgctgccc cccccgcccg cctatgacga cgtggcccag gatgaggatg actttgagat 1380

ctgctccatc aacagcaccc tcgtgggcgc gggggtccct gccgggccca gccagggcca 1440
gaccaactac gcctttgtgc ctgagcaggc gcggccgccc cctcccctgg aggacaacct 1500
gttcctcccg ccccagggtg ggggcaagcc gcccagctcc gcacagaccg cagagatctt 1560
ccaggcgcta cagcaggagt gcatgaggca actgcaggct ccggccggct ccccggcccc 1620
ctctcccagc ccggggggtg acgacaagcc ccaggtgcct cctcgggtac ccatcccccc 1680
tcggcccacg cgcccacacg tccagctgtc tccagccccc ccgggcgagg aggagaccag 1740
ccagtggcct ggacctgctt cccctccccg ggtgcctccg cgggagcccc tgtcccctca 1800
aggctcgagg acacccagcc ccctggtacc acctggcagc tccccgctgc caccccggct 1860
ctcaagctca cctgggaaga ccatgcccac cacccagagc tttgcctcag accccaagta 1920
cgccaccccc caggtgatcc aggcccctgg cccgcgggct ggtccctgca tcctgcccat 1980
cgtccgggat ggcaaaaagg tcagcagcac ccactattac ttgctgcccg agcgaccatc 2040
ctacctggag cgctaccagc gcttcctgcg tgaggcccag agccccgagg agcctacccc 2100
cctgcctgtg cctctgctgc tgcccccacc cagcacccca gcccccgccg cccccacggc 2160
caccgtgcgg ccgatgcccc aggctgcctt ggaccccaag gccaacttct ccaccaacaa 2220
cagcaaccca ggggcccggc caccaccccc gagggccact gctcggctgc cacagagggg 2280
ctgccctggc gatgggccag aggcgggccg gccagcagac aagatccaga tgctgcaggc 2340
catggtgcat ggggtgacca cagaggagtg ccgggcggcc ctgcagtgcc acggctggag 2400
cgtgcagagg gctgcccagt atctgaaggt ggagcagctc ttcgggctgg gtctgcggcc 2460
cagaggggag tgccacaaag tgctggagat gttcgactgg aacctggagc aggccggctg 2520
ccaccttctg ggctcctggg gccctgccca ccacaagcgc tgagatgcgt ctggagagcc 2580
agagggcctg cctgaaggaa tcacctgagc ctgtccgtcc accaggagtg gggagatgcc 2640
cccatccagt cctggaggac ccgctgctcc tgctgccccc ggggatggag caaggccaag 2700
gctgcgggag gctgggagcc ctgccctgcc catccctccc gcaccagcgc tgtccctgca 2760
cactttggtt cagtcccggt gcccctgcca agatgtggaa ggggccgggt gaagacaggc 2820
ttgagggctg ccccagcagg ctctgggtat gacctgcctc tggccctggt cctgggcggg 2880
gcctgtgggt ggagtagtac ccccaggccc tgccctgggt gacagactgg gaggaaacca 2940
ggctggacct gggcaggcgg gatgtgttgg ccacagggag aggcggaccg gcacccggtg 3000
ggacctccta ggactgggcc ttcttccagg gggcccctgg cagcagctgg ggtgtcgggc 3060
agaatgtgac ttgtggcctt accatggact tgaatgggac ttggctggcc tcaggatctt 3120
gtgcctggaa atagcctgag gtggctcagg aagcggagaa agggtgccag accattctct 3180
ggcggggacc agggcccaag gccccagggc tggaaggaga ccaaggggca gccgccctgg 3240
agggacatca gtgcttcctc ttccacccaa ttcccccacg cggttccatg ttttcccacc 3300
agcctgttgg cgaagttgct gctccggcat tcagtacctg cttcttccag agaaataaag 3360
ttagtttcta ttttatgtt 3379

<210> 145
<211> 3453
<212> DNA
<213> Homo sapiens

<400> 145
tttccacatc ttggctattg tcaatagtgc tgcggtgaac ctgggcgtgc agtgaacctg 60
ggtgctgcag tgaacctggg cgtgcagtga acctgggcgt gcagtgaacc tgggtgctgc 120
agtgaacctg ggcgtgcagt gaacctgggc gtgcagatgt ttctgctggg ctgatttcat 180
ttcctctggg tctatacaca gcagtgggat tgctggattg tgtagtagct ctagtttaaa 240
ttttctgaag aagctctata ctgtttctat aactgatctc agtgctggga ccccttgagc 300
ttagccccct ccttctgaga ctgcccatgg tgcccaggca gggcactccc tggcctgact 360
gtgcttgcct tgcagagtcg gtggctctga ggctgcgagg tgggacctgc tgctgtgctg 420
ggtggctgga cgtgttctac aatgggacct ggggcgccat gtgcagcaat gccctgaagg 480
acctctcctt gtccatcatc tgcaagcagc tggggtgtgg ggtgtgggga gtggggctgg 540
ctggagaaca ggcccttccc ctctgcgggc accgggaccg cctgggtgga caacatcgag 600
tgccgcaggc tgcccaactc cactctgtgg caatgccctt cccacccatg gcacccgcac 660
tcttgcgacc ttcgagagca ggtctggatt acctgtgcag gtgggcatta gaggtctctg 720
ggggtgcctg ggaagaggtt tatctgtcct gacaggtcac ttacagggga tgccattcaa 780
tcagagcctg ggcctggggg tcactaggat ccacagcctt ccttgtgaag cttctctggg 840
caccgaagct ctcagagctg ctgtttaacc cgcaggattg tcagaggaca ggccacaggc 900
tgctggggag cccctcaact gctcctcctg gctcggctgc ccaggtaccc ctctgtcctt 960
gtcgctgtcc tccttcccat cccggtccag cccccgcagt gaccgcagcc ccttttgcag 1020
aggagggcgc actgcgcgtg cgcgggggcg aggaccgctg ctccgggcgc gtggagctct 1080
ggcacgcggg ctcctggggc accgtgtgcg acgatggctg ggacctggcg gacgcggagg 1140
tcgtgtgccg ccagctgggc tgtggtcggg ccgtcgccgc cctgggggcc gccgcctttg 1200
gccctggctc cgggcccgtg tggctggacg aggtggggtg ccggggcagc gaggcgtccc 1260
tgtggggctg ccctgcggag cggtggggac gcggagaccg cgcgcacgag gaggacgcgg 1320
gcgtgcgctg ctggggtgag tggggggcgg tgggaagtcg gtcatggggc cggcagaggg 1380
cgctgggatg gagtcagtct tgagtgtcac ttcggtgggc aggagagctc gcccaggtgt 1440
tagtgggttg gtttccaccc tgggattttt ctgggatagg ctgatttgct gtggaggcct 1500
gtttgggaca tgcactccct ggggtttccc cctaatcttt tttgggaatt cccttttgtt 1560
tattccaatc cttgttcatt tcgggtatgt gtcacctggg tgttggcagg ttcagttgtt 1620
ccctgcacct cattctcttt gcagaaaaca aacagttaac cacagtgatg accttagcaa 1680
aagcagcagc agtggtaact ttccctgagc cactcccatt ctctgtgctc tggtcacctc 1740
ttgtgacttg ccctggcatc tgcctggggg ccatgtttta ccaaggaagg gggctgtccc 1800

ttgaaagcag acacccccag tggacactag ccattttggg gtgctctgca cagctgaaac 1860
cagcagatgc ccccccagca cttccgactc gcaggtcctc catgagggag agggtgtggc 1920
tgggcccttg gagccaccct gtcctgctga gggtgtgcag ttccccccca ccacccagct 1980
ggctcaagac ctctcctttc tcagagcctg gcccaggccc cccactgcct gcagctccct 2040
tccggacatt ctgggtggtc agtgtcgtcc tgggatccct tcttggtctt ctccttccgg 2100
gcctcatggc ttttctgatt ctgcctcgag tcacacaagc catgcagagg ggtctgggaa 2160
gatccgaggt atctcctgga gaagccatct atgatgtcat tggggaaatg ccgccagcag 2220
gactgtacga ggaaatcatg gaggccgagg ctgtgctcca agatgaggag gacggaagtg 2280
tggtgaaggt ggacacagaa gccgcagttt caggggaggt gtctaacctc ctggagggac 2340
agtctatacg tgcggaggga ggacacagca gacctgtttc tcagggatat gacgaggctg 2400
cgtttcctct ggaggagatg acgttgtaaa gcaacctgag gatgagatac accagctggc 2460
tgtcgaaatc acagctcttc attttcttgt acaattgtag tggatttcgt gagaacacct 2520
tggatgcctt tctcttgcaa tgtcctccat gtccatgtaa aatccagtcc ttccaggccc 2580
tgcctggctc taaccctcat ccccttcgag ggccatctgc tgtggacagt tgtgctgtgt 2640
aaccttcaga tttcccacac attacagcaa atgcaaatac acatagaaat cagtggttcc 2700
ttttgtggtt tagagacaca tggtgccatc ttcatcttcc gctccacagc tcgcttctgg 2760
cacccagcag tgggttgcag agctccccat gccagaacct tcctcttttt ttcttaaaaa 2820
ctcttcttaa ttgaatccaa agtatctttt aaatgttcta cttgtgtaat catgtcatct 2880
gtgaatattc agatttatct tctccttcca atccgtgtac atttaatctc tttttctgtg 2940
ccttatttcg gggctggga cccttcagtc cagtgttgaa gagaggcagc cagtggaggt 3000
cttgtctcat tcaaggactc agagcaaatg tgttccacat ttaatttcac tatgaaatat 3060
aatatttgat gttcagtttt gtagatgcta tttatcagat caaggaaagc ccagtctata 3120
cctaatttgt taagggtttt gctttttatc ataagtgtcg acttttatca aattcttttt 3180
tgtatctatt aagatgatag atgattgatt ttcatatgtt aaattaacca tgggttaaac 3240
aaacttacct ttatcatgat atattattct ttttgtattt cacaggaatt agtttggtaa 3300
tatgttgggt caatgtttaa aaaagaaaat gatgtgtaat ttttttcttt tattgtagta 3360
tttctgttta atttttggta tgaggattat tcaggtctca taagagttag gagtatattc 3420
tcttttaaaa aatatttgct aatttacact ccc 3453

<210> 146
<211> 4024
<212> DNA
<213> Homo sapiens

<400> 146

taaaatacta tcatctggca ggtataagag agaaatatgg gcccctgaga gttgggtgat 60
ttcactgtga ccaaatctaa aatacattaa ttcatgggca ccagtcagca agaagctttg 120
tgtgtcttgt atggtttcga tgccacagct ttgctgttaa cccgtcttcc agtcagcagt 180
tttttcagta actttcataa aggtctaaaa catgtttatc aagccaccat ttgtcagaaa 240
gctgagaggg ttaactaatt tgacacgatg caaagtcaca cattaggatt atctcaacag 300
atgagagact tccaggccca agccagcaag aagaggttta agagggcaaa atgtaacgtt 360
atgtactggg ctttggataa tcagctgcct gagtacaggg tgagagtgat gtggtttcat 420
agcagtcatt gtgaacaatg tctgaggatt tcggttggcc acagattgga gatgagagat 480
gagagtgatg caacagccag ccacaccagc attctcttga gacctctgca taggaaacat 540
gaggtccagt cacaggaact acggcctgtg ctcctgcagc ctcagggagc atccaaaggt 600
ggtgtgcagc cctggggcca cattttaaaa gggacctcga ccaacccaga gccaatccag 660
agaaattgta accaaatgga gagccattaa aaccaagaag taattgtttc aggagtctga 720
gaggtattaa aagaggatgg cagtctgcag aactgtgacc agtgaataga agcaaccagg 780
aagcagcttt atctcagtgt caagtcaaac attcaaaaac cagagtgggc agaccagctg 840
ccagcacacc aaatccagcc cacagatggg ctttgtttta actgcagttg tcctcctcac 900
attaccaaaa aactaaacta aaaattgagt ttgtcagcca tgctgaaaaa ttgggagagt 960
ttgcattttt aaaaaatcta gacttttgac ttctcctgaa gaatcagaat atctgaccct 1020
atggggcctt ccttctactg acagcagtct ctccacagaa tatgtgctgt cacggtcacc 1080
acagtccctc ctggtgtctc cccagtgctg aggcctggtg ccaggcacca ccaccatggc 1140
acccaccatt tggtctctct gagttggtga gagggctaat gggccaagca ggtaaaacag 1200
tgcctgttcc ttggtgaaag cccagcaaac gttacctgca attgttgtat attatcagcc 1260
tggcccttgc agatactgga gtttcaaacc tctgttgtaa aaatggaacc acctaactgt 1320
ggagttaggc ctcaggaaat atttactttt ttaccattgt gggtattaaa ataaaggctg 1380
gtagaatgct gttcattcag tgccttgcag aaggtcctct tctgtgtgaa aggctggttc 1440
cctggggtag gagctgccct ccactcttgg tgcatggaaa tgaggcccgt gagaagtgcg 1500
aaggtgcagg agaaatctcg agagaaggtg gtacactctg ctatttttgc tatagagtga 1560
catctcactg ttagaaaaaa gaccatccct tcagaagtta ttctcaacct cacagcctgt 1620
ggagaaacca ggctgatgct ccccctaggg gaatatcagg aacagcctca ttcttccttc 1680
tttctctctt tcccccctct ctcagctcca tccccagtgg gtcactgcat ctcactacca 1740
accatcatag ggagcgtgct gcttcccact gggatgaccc ctaacctggg agtctcctct 1800
cagccactct gctatacccc acccttcaca ctgcccttga ggccctcctg tgccaaggac 1860
ccctcaccct cccatagtcg tctctgctct gactcactcc ttgccagcct gaacctcctc 1920
tatcctctgt ctcagatgtg gcactcttgc cacagtgctg gctttcccag tggggccctt 1980
gtgtccattc tgctgctgtc ccctagtatg agagagtgtg tgtgtgtgtg tgtgtgtgtg 2040
tgtgtgtgtg tgtgtgtgtg tgtgtgtgtg tgtgtgtgaa ggcctgcggg gggagggaag 2100
aagctggaga gtaatttggt aagaaacatt ttggaaaaca gaagccaaaa gtaaatcata 2160

ttttataaaa tgatactttt tcaaaattga aggccttaat tgcctaaggt gaaatatagc 2220
cttaggaata gaatatgtat gcatatgtat gtatataaat atacattaac tctctctttt 2280
ccatttttga atgtttagtt tacagctgga tcctgaacac ttaaaatagc acctgacaca 2340
tagtaggaac tcaaaaatag ataggattca agaggaagca aataattttt accgaaaaat 2400
tggtttttgt gtttttttct tcttactggg tgttttgtgc atatttttat tgtgctctaa 2460
ataatcattc taacattatc acccctaagt tgttgaagca catttatatg gaaatcttgg 2520
caattagaat ggtaacttca tatttgattt taaattaaca gaatcttatt gagatcacta 2580
taaaggaagt gatcccattt tcttagagcc attagtttat ttagttggtt tttgaaagag 2640
agattgaatt caaacattgt taaagtcaga aaccttgcag acaagaagga agtgggaggg 2700
aaatgtacca tcaaagcctt ggcctcctga gaaaggagtg actctctaga cctgggtcag 2760
aaagaagtgt ctgaaaaggc ctaatttcat tgtcagagtc catttataaa tcctagggac 2820
ctagttgtgg ttttttgttt ttaactctta attgtatgta aattgtatac acataaataa 2880
aacaggcttg taatgcctaa aggaagctct ccatggctct gagactggga gcgggcgggc 2940
cttggctgag catgcaaata ctgacagctg aagtcacttg ttggaaagtc cgcaaactct 3000
ggttacagct gcatttgtca gtagctttaa gggaagtgta atcagccaga gagattaatt 3060
tgcatctcaa ttagaaaaga gtctggagga agcactaggg tctgttgcgg gaaggttgtg 3120
gaagaaatct caccaagcac aaaagcctcc tggcgcttcg tcctccagct gagtggagga 3180
caatatctga ggtttcgttt ccatagggac cagaagcctg ctgggcccca gaagaatctc 3240
gggtttgcac cgtaatcatt tggtcatgat gacacaaatt ccactgttgt tcccaaatac 3300
atttttgcta ttagaaagct gttagaaaag tggaaatggt catcatatca aaagcatgca 3360
agagggtatt tgctgaccca acttaccttt ttcatagtgc tgctaggatt gctaagcatt 3420
taacatttat taatgtctga gtattcaaag agcaaaaggt cattccataa atgacaatat 3480
ttctttagca ttcagatggt tactttggga atgacagagc ttcaaaggga gccattattc 3540
ctgattcttc cacacttttc agcagtaggc aaacaagatt ataaatttta aaacacaggc 3600
aaggaagaga catggaatct tctggccatg aagcatagaa aaaagtctgg ccattacaag 3660
actttttata atcctaatga atcctaataa aaatcataat ttaaaaaagc acaaagtagt 3720
ctaaatttat taattttccc tttaaatatt atctagtcat tttcataggc tttgatactt 3780
atatcacacg gctcctttgt tttcagagga gttcttaata ttatcaaaag cgggtacaaa 3840
gcctggtgag atctaaagct gtctgaaccg tttatccatg ccaaatcact tactcaacca 3900
ggaaggccca cccttggcat ttttggaaga agacttttat ttcaggttta ttttgttctc 3960
tcttaaatag ttaattcatt ggccccagct tcaaagtgag aaaacaaaca aaaaacagtg 4020
agac 4024

<210> 147
<211> 3810

<212> DNA
<213> Homo sapiens

<400> 147
ttattcaatg aggttgtgca gatgaacttt gaaatagcca gtttcagcag cctttcaggg 60
actcagccca tcacgtggca ggtggagtac ccacggaagg ggaccacaga catcgccttg 120
tccgagatct ttgtcagcca gaaggacctg gtgggcatcg ttcccttggc tatggacact 180
gaaattctga acaccgccgt actcacagga aagacagttg ccatgcctat caaggtggtc 240
tctgtggagg agaacagtgc cgtgatggac atctcagagt cggtggagtg caagtccaca 300
gacgaggacg ttatcaaagt gtctgagcgc tgtgactaca tctttgtcaa tggcaaagag 360
atcaaaggaa agatggatgc ggtggtgaac ttcacatacc agtacctgag cgcccccctg 420
tgtgtcaccg tgtgggtgcc ccggctgccc ctgcagatcg aggtctctga cacggagctc 480
agccagataa agggctggag ggtccccatt gtgaccaata agaggcccac tcgtgagagc 540
gaggatgagg acgaggagga gcggcggggc cggggctgcg cactgcaata ccagcacgcc 600
accgtgcggg tcctcaccca gtttgtgtct gagggcgccg gtccatgggg ccagccgaac 660
tacctgctta gtcctaactg gcagttcgac atcactcacc tggtggcaga cttcatgaag 720
ctggaggaac ctcacgtggc caccctccag gacagccggg tcctggttgg gcgagaggtt 780
gggatgacga ccatccaggt gttgtctcca ctgtctgact ccatcctggc agagaagacg 840
ataaccgtgc tagatgacaa agtgtcggtg acagacttgg ccatccagct cgtggctggg 900
ctgtctgtcg ccctttaccc caacgcagaa aacagcaagg ccgtaacagc tgtggtcaca 960
gctgaggagg tgctgcggac ccccaaacag gaggctgtat tcagcacgtg gctgcagttc 1020
agtgatggct ctgtgacgcc cctggacatc tacgacacca aggacttctc cctggcagcc 1080
atctcccagg acggggctgt cgtgtcagtc ccccagcccc gctctcccag gtggcccgtt 1140
gtggtggccg aaggggaagg ccagggccca ctgatccgag tggacatgac gatcgccgag 1200
gcctgccaga aatctaaacg caagagcatc ctggctgtgg gcgtcggcaa cgtcagggtc 1260
aagttcggac agaacgatgc tgactccagc cccggcaggg actatgagga agatgagatc 1320
aagaaccacg ccagcgaccg ccggcagaag ggccagcacc atgagcgcac aggccaggat 1380
gggcacctct atggcagctc tcccgtggag cgtgaggaag gggctctccg aagagccact 1440
accacggcca ggtccctgct ggacaacaaa gtggtgaaga acagtcgggc agacgggggc 1500
aggctggcag gagaggggca gctgcagaac atccccattg acttcaccaa cttccctgcc 1560
cacgtggacc tccccaaggc cgggagtggg ctggaggaaa acgacctggt gcagactccg 1620
cggggcctga gtgatctgga gatagggatg tacgccctcc tgggggtgtt ctgcctggcc 1680
atcctcgtct tcctgatcaa ctgcgccacc tttgccctga agtacaggca caagcaagtg 1740
cccctggaag gtcaggcctc catgacccac tctcacgact gggtgtggct tggcaatgag 1800
gccgaactcc tggagagcat gggggatgca ccgccgcccc aggacgagca caccaccatc 1860
atagaccgcg gaccgggggc ctgcgaggag agcaaccatc tcctgctcaa tggtggctcc 1920

cacaagcacg tgcagagcca gattcacagg tcagccgact ccggggggcg gcagggcaga 1980
gaacagaagc aggaccccct gcactcgccc acctccaaga ggaagaaggt gaaatttacc 2040
acctttacca ccatcccccc ggacgacagc tgccccacgg tgaactccat cgtcagcagc 2100
aatgatgagg acatcaaatg ggtgtgtcaa gacgtggctg tgggtgcccc caaggaactt 2160
agaaactatc tggagaaact caaagataag gcttaggccc ctctagccaa agggccctgc 2220
ccagatgcct tccttgtact ggaaactggc ccaagtgggg cagaaggcgt tgtcagtggg 2280
gttaagaagg gacggtccca gggtccatgc tagaccagtt ggaaagtttt gaagtcagga 2340
aaagacgttt ttgtatcaag ggatttttag cagttaatgg tggtggattt ttaaaggtca 2400
ggggaataaa gtctggggca tggggagtgc agaccaagtt actgaactgc acaggcaaaa 2460
ttaggaaggt tattttatga gtcaaaacat actacagaca agctaccaaa aattatttgt 2520
taaaaaatgc aacaagacaa ataaaaagag aaataatcat ctgtttatat ttctaataaa 2580
ggagcaaaat ataaaaatag gacctgctaa gagacatttt ccattctaat tcacgattca 2640
cttttccaag gacagccttc aactgtcacc acacagctgg gggggagtca tttcttaaca 2700
agggatgcct cttgggatag aactagggag ttttaaatct ttacttgatc atcttttatt 2760
ttcttttcca ctttttcctt ttctctctct ctctgtgtcc tagacttcca ttgcatttat 2820
atttaatgtt tatttctgag aatcaagcag tatatttttc ctaaatgaaa cataaattat 2880
attcctattc attagatagg ttcctaggaa caatgccaat taatccattg tttaagtagt 2940
aacttgaatg tttttctata tccctccagc tttgttgata gtggcgggtt ttgtacaatt 3000
ggagggagcc ctcagagcct tctgggggag gagaggaact gtccttaatc catcaccact 3060
accatagggc aaagccagca ggtgtggccc tgtgaggggc tgtacagacg ggatgtggcc 3120
aggagaacag agccccacct ggaccacctg acccctcggg attccacccc tgtcatcgtg 3180
gggatgttcc tatataggag aaagttgggt taaatcaaaa aagaggccac gcccaggtgt 3240
aatcagagcc aacctggtgg gctgggtcta tcacaagaca taactgatgc tgaacatgaa 3300
caaagataaa aactgtttgg agggtttttg agttgttttt cttatgttgt tgggtggggt 3360
ataccagcat aaactctaaa gataaaatct atgttagatt gtcaatcaac tgtgtttttg 3420
aacagcataa ttgtgtagca gcacattgca aaaatgcatt catccaaagc gacacatgtg 3480
gcaacgtaga ccacgccagt gaaataagcc ccttcgtgat cacctgactc cagttctccg 3540
tgtgctccat tggctgcggc tgcaggagga agatgcctga cagccctcat gctctccgca 3600
ggggggcgct cacaaagatg ccaggggtgt ttattgtgtt tattttttta attactaaaa 3660
tcagtagcta agaaagggtc cttgaagcct cctaacctgg gttggacctt tgaaaaatat 3720
atttgtagca catattatag atggaaagaa gaagatattt atttatacct gtgatgccaa 3780
ttgtcattaa aaggcttttc atggcttgac 3810

<210> 148
<211> 3274

<212> DNA
<213> Homo sapiens

<400> 148
gtcagcgctg aaggcccaga gcgcgctgca cgagcagaag actctgcccg ggatgaaccg 60
gccgatccag gtgaagcctg cggacagcga gagccgagga gatagaaaac tcttcgtggg 120
catgctcaac aagcaacagt ccgaggacga cgtgcgccgc cttttcgagg cctttgggaa 180
catcgaggag tgcaccatcc tgcgcgggcc cgacggcaac agcaaggggt gcgcctttgt 240
gaagtactcc tcccacgccg aggcgcaggc cgccatcaac gcgctacacg gcagccagac 300
catgccggtg agtgctggcc ccttggggcg gggcgaggg cagcggcggg ccgagacccc 360
agcacccgcc acgccccgcc ggctgtccag tctccccaag agacaggaaa gcatgaccct 420
tattccgggc ctcagacaag gacgcggaag cccagggatg ttaaggaact ggcctgaggt 480
tacccaggtg gaaaatgccc gcggtggggt tcacacctca ttcccctggg cgagcgccga 540
tgccgcctcc tccaaagcgc cccgaggagc gggcggggtg ggcgcaggcc agaggcaccg 600
acagctgcgg gcggaggctc tggagcaggt gggtctcacg cgccgccccg gccgcaggga 660
gcctcgtcca gtctggtggt caagttcgcc gacaccgaca aggagcgcac gatgcggcga 720
atgcagcaga tggctggcca gatgggcatg ttcaacccca tggccatccc tttcggggcc 780
tacggcgcct acgctcaggc agtaagtggc agcgcgcacg ggcagcggtt ctcaagctca 840
ctcccctggg agcctcagag agcgggacat gaaaccgaaa atactactct atcccttgcc 900
ctctccctcc agagtgagga ggggccggga atgaacccat gaacccgggg ggtgtaactt 960
cagaagggtt tgaaagaagg ctggcccagt tggggtggct gccaggagga ggtgggctgc 1020
tgtcttagtt ctaaggggca gagctgagag aggccccaca gaaaagtggg ttaccctgag 1080
ccttttcctc tcagccagat caacgttgaa ccccaggaag cacagatgag agggtgaggt 1140
tcaggtcatt ggttgcaaac cacccctact ccatggaacg gtgacttcag gccttctggt 1200
ctctcctggc agggctcctg tgaggtgccc taaagcccta cacacccaca cttctgatat 1260
cccctcaggc ctctggccca cctacttctt tcttccacat gggggcccac tctgtcagcc 1320
tctcccggag ctgcctgcag actggtaacc acagctccca tgtgggtggc actgaggcag 1380
gccacggttc tgggtctgaa aatgcaagct tacctcactt ctgcagtact gtctgccttg 1440
gccacattcc gcagcctccc ctgtccgcac tcacctttgt gacaacacta ggggtgcctg 1500
gggttctatt cataattctc ctgtcacttc taccaacaga ctccaaatta aaaccattct 1560
ggacagaaac ctgtgctaag tgagttctgc agaggctcat ccagcatgtc tctagtgctt 1620
tggatgtcac ttccccacga atgacaggct gtacacaggg ctgactagca cccacagagc 1680
cagtctcctc ctctgcccct agcaacaact ttggagttcc aaagacccta gcattacttt 1740
taaatacagt catctctcag ggtggtggac tctaatagct gttgaggtac agggcaagaa 1800
tgggagaggc atcagggctt tctggagtgg tagttgggag cttctggggc tcagagcctg 1860
ggagattggt ctccaggagc tgaggcccct gtcggggagg gatgccccct ggaagcacgt 1920

gatattttt ccactccctc tgccccagct gatgcagcag caagcggccc tgatggcatc 1980
agtcgcgcag ggcggctacc tgaaccccat ggctgccttc gctgccgccc agatgcagca 2040
gatggcggcc ctcaacatga atggcctggc ggccgcacct atgaccccaa cctcaggtgg 2100
cagcacccct ccgggcatca ctgcaccagc tgtgcctagc atcccatccc ccattggggt 2160
gaatggcttc accggcctcc ccccacaggc caatgggcaa cctgctgcgg aagctgtgtt 2220
cgccaatggc atccacccct acccagcaca gagccccacc gccgcggacc ccctgcagca 2280
ggcctacgcc ggagtgcagc agtatgcagg tccagctgcc taccctgctg cctatggtca 2340
gataagccag gcctttcctc agccgcctcc aatgatcccc cagcagcaga gagaagggcc 2400
cgagggctgt aacctgttca tctaccatct gccccaggag tttggggacg ctgagctgat 2460
gcagatgttc ctccctttcg gcttcgtgag cttcgacaac ccggccagcg cgcagaccgc 2520
catccaggcc atgaacggct tccagatcgg catgaagagg ctcaaggtgc agctgaagcg 2580
gcccaaagac gccaatcgcc cgtactgagc gccggcggga gcgtcccccg ggggagacca 2640
ggactcgcac agggcaggat gctgaacggg ctacattaaa aaacaaacct ctctctatat 2700
atatttataa atgagaactg ttggatgaca cctttgacat atcagccaat atcaatcaag 2760
ctgaagactc cagacactgt ctgtgtgact gtaacatttc ttcaaggaaa gtatagcgtc 2820
tatggagttc agagggcacg tgtttggggg aaaatatata tgacatgaag aagaagatga 2880
agaaaaatga gaaaaaaaca cacaaaaggc aactttaaaa caaaatatca cgagcagacg 2940
gggaggctga agggctggga gctgggagga gacgctgctt accgatcccg gggcttttcc 3000
agcccacggg cgcctgacgc aggctggggc aagtggtgcg tggggcctgg tccccaaggg 3060
gcggctgaga ggccgccact gagcatctct atctgtcatt cctttagcta tttaggacc 3120
aaaggaccaa actttttatt gcagatgtgt agctctatgt caaatagagg gggaatggag 3180
gacccctcc ttcctgcctc atggctgttc ttgaaacagc ttagagcgat tctatgaaaa 3240
aatgtaataa aaaattaaaa aaaaaacaaa aaac 3274

<210> 149
<211> 3349
<212> DNA
<213> Homo sapiens

<400> 149
atatgaaaac atttccaatc agattttctg ctgaatatat tggcaaagga atttcatatg 60
gggctggaga tctagaagag atattgcaga gaatccgaga ggaaacttaa gagaatgaaa 120
ggaagtgtga taaacagcac tgagaccagt aaaaatctgg gaagtcagaa gtattgcagt 180
tgtccaggca aaatttataa caagctatgg caacagcaga gtggactgaa agagaagata 240
gacttcatag ggtttgtctc caaagcactt ggtgattgcc tgaatgtaga agacaggcaa 300

gaatgtaaaa gaggactctc acaaacacag ctttcgaaca gtagacacat ttgcaaaaga 360
gaaaaagttc tcaccactcc aggaatccaa gcagaaagtc aacacccctc ttatatgtgt 420
ttaccataag aatagtttct tctctagagg gtaagtcaaa ctgggtgtcc tctttttccc 480
atctgcgtcc aggattgtgc ctttctatgt ggtagagtac gtctgtgtgt gtatgtgaaa 540
ttgtgccaat tatcaactta ttgtttgagc ttcaaattct tgtgttgcct ggtctgtgaa 600
aatggatctg ggccctttac gtgtttcctt tgccagctgg cataaggttt atcagtagag 660
ggcaccagag caacactgca gggagaaggg tcctctttct tgggtcaggt ggctctttca 720
ccaggtcctg gagagtacct gtttttttc ttttctattt ttttttttt taagatagag 780
ttttgctctt gttgcccagg ctggagtgca atggcgtgat cttggctcac tgcaacctct 840
gtgagagtac ccatttttac tctactccat ggccagtgaa gttttctccc gtgctaaatg 900
cctgcaggac acagcttttt ctaccaattg gccaccatag gagtatgttt tgtccagctc 960
tccactcagg cactggtggc tcagttggga gcctaaacag cacaggcctg tgccccagcc 1020
caagccactg ccataccctc tctgcagcct cactggtaca aatgctgggg gccccacggg 1080
actggccgtc tagcaagcag accacctgac ctcctcctgt gcggaggctt gagcctcccc 1140
agcccacact gaccctctcc ccagctctag tgcatgcaca ctagggcccc agtagccact 1200
gttgggtctc ccagcccaag ctgtgcacag aagagtacta ggttcttgca gctctccagc 1260
ctccatttcc agcagtgccc acagcagcgc cttggccgcc tccgctcatc cgcccattaa 1320
ccttggctcc tgttgtttcc ttaggccttc cccacacaga caccatgtga tccaggcctt 1380
gtgtcaacat caacacccca gctctctctg tattcccaat tccctctgtg tactatttgc 1440
cagctttggc ttgcttgtgc cccagagggt tatttctgct tgcttagtga ctgtggacca 1500
actctggcct gcacaacctt ccccctcaaa cttttctgct atccagtagg ctgcaaccac 1560
aactccaaga aggactgaac cctagccttg gggagggaac cctccttcca agtttatcct 1620
tgggtaatcc ccctcagctg taggattttc tttttgtatt tacatttatc tttggaaaat 1680
aattttttttt gggggtggg gatcgagtct tgctttgttg cccaggctgg agtgcaatgg 1740
cacgatctca gctcacggca acatccacct ccctggttca agcatttctc cctgcctcag 1800
cctcctgagt agctgggatt ataggcaccc gccaccacag ccggctaatt tttgtatttt 1860
tagagatggg gtttcaccat gttgccaggc tggcctcgaa ctcctgacct caggtgatcc 1920
gcctgcctca gcctcccaaa gtgctgggat taaaggcatg agccaccatc cctggcaaaa 1980
taattcttta atttaaatta ctgtatggtt tctgtctccc tgtcgaacgt agattgatac 2040
aagtgtgaag gtatatgtgt gtgtttttaa aatgagggaa aagacatgaa ttttgactgc 2100
atatatttgg tgagcccaga gataagccaa ataaataatg caagtcaaaa ctattaatta 2160
tgaatactca gctggggctg tgaccctgaa tctagacaca gaaatctaat ggtctacatc 2220
agaggttggc aaactacagc ctaaatgcca atccttgctg atcacatgtt tttgtgtggc 2280
cttgagccac acaaaatgta aatggtcttt acatttacat ttacacaaaa tgtaaatggt 2340
ctttacattt acatttacac aaaatgtaaa tggtctttac attttaaatg gtcgcaaaga 2400
atttaaagga taataatatt ttgtgacatc tatttgtatc cataaataac attttattgg 2460

aacccagcca cactgatttg ctaggtattg agtatagctg cttccatggt acatggtaaa 2520
gttgagtaat tgcaacagag accaaatggc tcgatagccc taaaatgctc tctgccctta 2580
acaaaaaaaa tattggaaac taaccagtct tatacatatt ttaaaaaagt ttgcccgccc 2640
ctgatctaga ctgaaacttg ggaccatttt atcaagtaaa taccagatga attgcctgtt 2700
taatccccgg ctctgaattc atgacctctt ttcccttatt tgtagattgg caaactaatt 2760
gagttgcaag gcatctagaa tagccaaaac aaccttgaaa aagaaaaagg aggaggattc 2820
acactttttc tgacttcata tagatcaaag gaatagaatt ggaagtccag aaataaaacc 2880
atgtgtcaat ggtcaaatga ttttccacta gggtgccaag attattcagt gggggataga 2940
agagtctttt taacaaatga tgctgagcta actgtatatc tacttagaaa agaatacagt 3000
ttgaccctac cttataccat atataaaata tgaactaaaa atatatcaaa aatctaaatg 3060
taagagctaa aactataaaa ctattagaag aaaacaaagg ggtaaatctt cataatctta 3120
gatttggcag aaaagatttt tcttattgca ccaaaaacat gagcaacaac aacaacaaaa 3180
agataaatta gacttcatcc aaattaaaaa acttttgtac aggatattac caagaaagtg 3240
aaaaggccag gcatggtggc tcatgcccgt gatcccggca gtttgggagg ctgaggtggg 3300
tggattgctt gaatccagct tgggcaacgt ggtgaaaccc tgtctctac 3349

<210> 150
<211> 3519
<212> DNA
<213> Homo sapiens

<400> 150
ttaaatttaa aattgaggcc tggagagaaa aaacctggat tttgggtgcc ccgtgtttgg 60
gcgaatcctc ggaactttaa tttcgacaat gtgggaaacg ctatgctggc gttgtttgaa 120
gttctctcct tgaaaggctg ggtggaagtg agagatgtta ttattcatcg tgtggggccg 180
atccatggaa tctatattca tgtttttgta ttcctgggtt gcatgattgg actgactctt 240
tttgttggag tagttattgc taatttcaat gaaaacaagg ggacggcttt gctgaccgtc 300
gatcagagaa gatgggaaga cctgaagagc cgactgaaga tcgcacagcc tcttcatctc 360
ccgcctcgcc cggataatga tggttttaga gctaaaatgt atgacataac ccagcatcca 420
ttttttaaga ggacaatcgc attactcgtc ctggcccagt cggtgttgct ctctgtcaag 480
tgggacgtcg aggacccggt gaccgtacct ttggcaacaa tgtcagttgt tttcaccttc 540
atctttgttc tggaggttac catgaagatc atagcaatgt cgcctgctgg cttctggcaa 600
agcagaagaa accgatacga tctcctggtg acgtcgcttg gcgttgtatg ggtggtgctt 660
cactttgccc tcctgaatgc atatacttac atgatgggcg cttgtgtgat tgtatttagg 720
tttttctcca tctgtggaaa acatgtaacg ctaaagatgc tcctcttgac agtggtcgtc 780

agcatgtaca agagcttctt tatcatagta ggcatgtttc tcttgctgct gtgttacgct 840
tttgctggag ttgttttatt tggtactgtg aaatatgggg agaatattaa caggcatgca 900
aatttttctt cggctggaaa agctattacc gtactgttcc gaattgtcac aggtgaagac 960
tggaacaaga ttatgcatga ctgtatggtt cagcctccgt tttgtactcc agatgaattt 1020
acatactggg caacagactg tggaaattat gctggggcac ttatgtattt ctgttcattt 1080
tatgtcatca ttgcctacat catgctaaat ctgcttgtag ccataattgt ggagaatttc 1140
tccttgtttt attccactga ggaggaccag cttttaagtt acaatgatct tcgccacttt 1200
caaatcatat ggaacatggt ggatgataaa agagaggggg tgatccccac gttccgcgtc 1260
aagttcctgc tgcggctact gcgtgggagg ctggaggtgg acctggacaa ggacaagctc 1320
ctgtttaagc acatgtgcta cgaaatggag aggctccaca atggcggcga cgtcaccttc 1380
catgatgtcc tgagcatgct ttcataccgg tccgtggaca tccggaagag cttgcagctg 1440
gaggaactcc tggcgaggga gcagctggag tacaccatag aggaggaggt ggccaagcag 1500
accatccgca tgtggctcaa gaagtgcctg aagcgcatca gagctaaaca gcagcagtcg 1560
tgcagtatca tccacagcct gagagagagt cagcagcaag agctgagccg gtttctgaac 1620
ccgcccagca tcgagaccac ccagcccagt gaggacacga atgccaacag tcaggacaac 1680
agcatgcaac ctgagacaag cagccagcag cagctcctga gccccacgct gtcggatcga 1740
ggaggaagtc ggcaagatgc agccgacgca gggaaacccc agaggaaatt tgggcagtgg 1800
cgtctgccct cagccccaaa accaataagc cattcagtgt cctcagtcaa cttacggttt 1860
ggaggaagga caaccatgaa atctgtcgtg tgcaaaatga accccatgac tgacgcggct 1920
tcctgcggtt ctgaagttaa gaagtggtgg acccggcagc tgactgtgga gagcgacgaa 1980
agtggggatg accttctgga tatttaggtg gatgtcaatg tagatgaatt tctagtggtg 2040
gaaaccgttt tctaataatg tccttgattg tccagtgagc aatctgtaat tgatctataa 2100
ctgaattcca gcttgtcaca agatgtttat aaattgattt tcatcctgcc acagaaaggc 2160
ataagctgca tgtatgatgg gttactatca atcattgctc aaaagaattt ttgtataatg 2220
acagtactga taatattaga aatgataccg caagcaaatg tatatcactt aaaaatgtca 2280
tatattctgt ctgcgtaaac taaggtatat attcatatgt gctctaatgc agtattatca 2340
ccgccccgca aaagagtgct aagcccaaag tggctgatat ttagggtaca gggtttatag 2400
ctttagttca catctttccc atttccacta gaaatatttc tcttgagaga atttattatt 2460
tatgattgat ctgaaaaggt cagcactgaa cttatgctaa aatgatagta gttttacaaa 2520
ctacagattc tgaattttaa aaagtatctt ctttttctcg tgttatattt ttaaatatac 2580
acaagacatt tggtgaccag aacaagttga tttctgtcct cagttatgtt aatgaaactg 2640
ttgcctcctt ctaagaaaat tgtgtgtgca agcaccaggc aaagaaatgg actcaggatg 2700
cttagcggtt taaaacaaac ctgtagataa atcacttgag tgacatagtt gcgcaaagat 2760
gttaagtttc ttaagaaacc ttttaataac tgagtttagc aaaaagaata aaactatata 2820
gctcaattta tttaaaaaaa tctttgcatg tgtgatgtta tcattggctt catttcttac 2880
ccaaggtatg tctgttttgc cataaatcag cagagtcatt tcattctggg tgatcctaac 2940

acaccattgc tacgttagat ttgaaatgac atctctgtta aaagaatctt ctatggaaat 3000
aatggtgccc tgcaaaatct tcctttgaac tcacaggtta gggatcacac aacttactta 3060
atcgtttttt gtttttgttt tttttcctta tatgtcaatg gcccatgtcc tccgggaaaa 3120
ttagaaaagc aaaatgatta caaagtgctg ttagatttct tgtgctgggc cagccaagta 3180
gaagtggact tgacttggac ctttaactat tttattacag attggacatt tgctgttcag 3240
atgtttttta acagagggat tatctcagaa tcctgtgacc tccaggttgt tttataatct 3300
atttttctct atttaacatt cctcagatag ataggcaaat aggacattcc ttctgtgtca 3360
cagaagtatc gtggtagtgg cagtctacag tttatatgat tcattgtaac tatgagataa 3420
agaacaacca gtcatgtggc caaaaggatt agatttgatt tgatgttcac ttggagttta 3480
ctttttgtac atacaagata aaataaatat tggatttgt 3519

<210> 151
<211> 4247
<212> DNA
<213> Homo sapiens

<400> 151
tgatgcctgg cagcccacgg gccgaaggaa gaatcggaaa aacaaagaga ccgtccggca 60
catcacacag caggtggaag atgacagccg ggccagctcc ccggtgccgt ccttcctgcc 120
gacgtcgggg tggccgttca gcagccgaga ctcaaatgcg gacctgtccg tcaccgaagc 180
cagcagctcc gacagccgag gagagagggc cgagctctat gcacaggtgg aggagggtct 240
cctgggagga gaagccagct atctgggccc tcccctcacc ccagagaagg acgacgccct 300
gcatcaggcc accgcggtgg ccaacctgcg cgcggcgctc atgagtaaga acagcctgct 360
gtcgctgaag gccgacgtgc tgggggatga cggctccctg ctgttcgagt acctgcccag 420
aggggcccac tcgctgtccc tgaatgagtt cacggtgatc aggaagaagt tcaagtgtcc 480
gtactgcagc ttctcggcca tgcaccagtg catcctcaag cgacacatgc gctcgcacac 540
gggagagcgg ccctacccct gcgagatctg cgggaagaag ttcacgcggc gcgagcacat 600
gaagcgccac acgctggtcc acagctagga caagaagtat gtgtgcaagg tgtgcagccg 660
cgtcttcatg tccgccgcca gcgtgggcat caggcatggc tccaggcgcc acggtgtgtg 720
caccgactgt gctggccgcg gcatggccgg gcccctggac catggcggcg gaggcggcga 780
gggctctcca gaggcgctgt tcccaggcga cgggccctat ctggaggacc ctgaggaccc 840
acgaggggag gcggaggagc tgggcgagga cgacgagggc ctggcccctg aggatgcgct 900
gttggcagac gacaaggatg aagaagactc gccgcggccg cgcagccccc caggaggccc 960
tgacaaggac ttcgcctggc tctcctaggc ccgcccgccg gcagggtcgg tggctgcctc 1020
gctccgtcca cccgtgtgtg tgtccggtgg gtctccactg cggggccagg gccacgctca 1080

cccctctcgc ggcccctcct ctgcttcccc ctgaacccac cccccacgga aaccagccct 1140
gcgggctaag caggtgcgac cccagcaaga ggggtgctct gggaccagac atgaagtgag 1200
ttgggggagg gcacagggtg ggtttgagtg aagggagagc acggtcctaa gtccccagca 1260
ggtggtgcgg gtgtgtgagt ggcccctgtg atggccagcc tggcttggac acgtgatggg 1320
cctgtggccg ggtccagtgg gcactgggcg gggtggtgtg ttcggcccag aggcccctgg 1380
cctgagcagg tgcagagttt tacagacacc cggtcagccc ggctggagcc cgccctcccc 1440
tcccctagca gccaggtcac tgcctgtggc tgcagccgtg gcccgtggtg cagccgtggc 1500
ccatggcagc ctgtagaatc caggtgcaca gagagccctg ccaccgtctt acctctgggc 1560
tttggtgctt aacacacaac acagctgcag accctgctgg aggccgaggg ctccaggtgc 1620
tatcttaggt ggacacagcc ctgggggctc cttccaggag gggaccctca gccctgtgcc 1680
ccccaccact tcaggccaca ccaggttccc tctgcaaggg cctcggctca gtcgtgtgca 1740
cttcctcgga gagccttggg ctgccacgtc caccccgggc tctgcccgtc ctgttctgcc 1800
catggcccag cccggccgct cctgctgacc cctcctggac gggctggagc tgggctcctg 1860
cctttgctgc taacactgga ggcggtgttc ctaactgcag tgtgctgctt acacctcccc 1920
gcgtgggtaa ccaaattttt aagtagtcag agacatatcg aggtagttac ataaaattat 1980
tttgtttggc attatttttc tcactcgaag aaactatata gggttgtttt tcctttagct 2040
tgtgctcaag tcctcttgct gtgttttcag aagcactcac atgttctttc ttttcctgag 2100
tgaaaagcaa aggtcccacg gtgtgtgctg tggtgcaccg cctggctttg ggggtcccgg 2160
aggcaggctg cctagactca cagcctcggg accgttgcca cggcctgtct tctcgttcag 2220
gcctgcctct gacagcactc accatgagga cattccatcc ttcaccccct cctctggcac 2280
aagccaccac tgcggtgctg tgccttcaga tgggaggtgg gcgcggtggc cgcctccttc 2340
cctccaggac ctgcccgtgt gaagaccccc cggagtgctg agcttcaggg ctgcgtggaa 2400
agagttttta ctctcttttt ctagcctgta taccaggctt ttccccacat tgtcaggtag 2460
agcaccagct tccctgaccg ctgctgctcg gggagggctg gggctggccg ggggtcctgt 2520
ggaggagtac atggaggact ccaggtacag cgcaggagtc acggcttttg ttttttgaca 2580
ttggcccccg gttctaccaa tgacagggtg ccctggctgg agctgtcatc acacacaccc 2640
ctcagctcgg aggctgtggg ctcctcaaag ctggagaaag aggccaagat ttttctgcac 2700
acggagtgtg gggataggag ccgggccaag cgctggcccc tcagcggtga gccctgccca 2760
ctcttaccga gcaaggtggg tggctctggc acgagtcccc cagggggaga gcatggctac 2820
cagggagctg cagcggagcc ctccagccct caccccaggc cagccccacc ccggcctctt 2880
tgagaattct cagaactttg tacctttccc ctgattttta aacccttttt ctaaacagac 2940
tgactttctt acaaaatgca tttggaaacc agacctttgc tacccaccaa tgtctctggg 3000
ttttgtacca gtccctgctc tcaggccacc ctgcccagga cccgggcccg cctccccctc 3060
cacactcagg atgtcctgct ccatctggcc ggctcactcc gtgtggcctg cctttgctga 3120
ccgttttggg gttccccgcc ggagctacag ggcatttttc ttccctaaaa ccaacagtgt 3180
cccactgacc tccccaagtg tttgctgcgt ggcagatttc ctgttcttgt tcgcagtttg 3240

ccgactgaag agtgtgggat ttccgaggcc caggtgagca cgtccatctc aggaggcgtg 3300
gagggaaaag acatgtcatg aagggttttt tttatgtgac tgattttttt ttaaatcgat 3360
gttcaaacta ataaatattt ttttatgaag aggaaaaatg tgtagattac atttcacatt 3420
ttgtattttt gtttgtgtct gtttgtattt tggtgtttac aacaccaaag tgggaaatac 3480
agtccattgg ggatggtgtt atttgggggc ggggaggggg cagggacacc acgatttttc 3540
tgtcaagctc tggatcctga ccaggttgta cactggggct ctctgagctt tgggacacag 3600
gacactgcca gggctacgta gggaactgac tcagaagacg cagcttactg cttccaactt 3660
tgcacatctt cctctttaaa aaactgagaa aatgcaaaaa ctggaacttt ttgcaatatt 3720
ataaaagaag taatcttatt ttagctcatt ctgtgacatg tgcgactctt aagaaagcca 3780
tacttaatgg tggtggtttt ttttagatct tatattgtgt tttgtatgca gcccttttag 3840
aactacttgt agtgagggtg ctgtgtgtgc ttttcttaaa tatttatttt tttcaacatg 3900
ctttcaacct gtcaacaaaa acaaaacaca caaaaaaagg gcagtgtttg aagattgttg 3960
atttttttct ggggataatc tatattatat tgacttccta ttacttatta taaacctgtg 4020
tttgtattgg agatgtgtct actattgggg gaagaggttc tcgtaatcgc tcggtgggaa 4080
atcatggctc tgccgtcctg cctctctgtg gccgtgggtt cacgtggcct ctgcggtgag 4140
tctccaagtt tctgcctagg cgcctgtgcg tttcctttct gtgacgggat tagcttagac 4200
atccttgcaa agcgatcact ttcaataaat tgggaaattg ctgctcc 4247

<210> 152
<211> 4268
<212> DNA
<213> Homo sapiens

<400> 152
attacagccc aggaatgagg cctgaacaga aggaggctct ggcaaagcga ctgctggccc 60
ctgaactgtt tggggaagtg cctgcctggc cccaggagct gctgtgggca gtgctgcccc 120
tgctccccca cctccctctg gagaactttt tgcagctcag ccctcaccag atccaggccc 180
tggaggatag ctggccagca gcaggtctgg ggccagggca tgcccgccat gtgctgcgca 240
gcctggtaaa ccagagtgtc caggatggtg aggagcaggt acgcaggtgg catatgaact 300
tctgggtgtg ttgcgctcat ctggaggagc ggtgctgagc cccgggagc tgcgggtctg 360
ggcccctctc ttccctcagc tgggcctccg cttccttcag gagctgtcag agccccagct 420
tagagccatg cttcctgtcc tgcagggaac tagtgttaca cctgctcagg ctgtcctgct 480
gcttggacgg ctccttccta ggcacgatct atccctggag gaactctgct ccttgcacct 540
tctgctacca ggcctcagcc cccagacact ccaggccatc cctaggcgag tcctggtcgg 600
ggcttgttcc tgcctggccc ctgaactgtc acgcctctca gcctgccaga ccgcagcact 660

gctgcagacc tttcgggtat gagagtggca aggaggatga gataatcagg gataccggct 720
ctttctggtt gggaggaagg catcttccct gaggccaggg aaggcctttc atacctcccc 780
acttacacac acacacagac acacacacac acacacacac acacacacac acacacaacc 840
aattctcatg caggttaaag atggtgttaa aaatatgggt acaacaggtg ctggtccagc 900
tgtgtgtatc cctggtcagg taagtgtgag atctcccaac tgagctcctc tccccattct 960
ggggcagttt catatggctg gtgctacctc ccacactacc ctgcagtggc cctgagagtt 1020
ctggttagct ctgtgcccat tagcagccct ccccagtgcc agatgcagga cagcatgatc 1080
cactcacatt gtcctagact aatgtcaaag ctggaagggc ctgagaaatc ttccaggcca 1140
cccaccctgc tttcagatga aaagaccaag gctgggagaa gctaagggac tttgtttgcc 1200
tggtgcctaa ctagcagcaa cacttgacca cagcagcctg cagtgtgagg ctcttaggcg 1260
tttattgcta cagtggcaaa tgccattcca cttctgtcct agctttggtc cctttccacc 1320
cccatggttc cttttctctg agtgctaagt acagactctc tcacctatca ctacactgct 1380
atacccatca ccgccagcag cctattccca ccacctggcc agactgcctg cttcccctgc 1440
tcccattaaa gctgctacaa ctggattcct tggctcttct ggcaaatcga agacgctact 1500
gggagctgcc ctggtctgag cagcaggtaa ttctccccac ttaatttcag aacttcctcc 1560
ctcaatgtag tctaccttct ttacctatcc cttagcccta tttggccagc ttatccctac 1620
tatcctttat ttgattgttt gagatacagt ctcactctgt tgcccaggct gcagtgcagt 1680
ggcatgatca gagttcgctg taacctcaaa ctcctgagct caggcaatct ttctgcctca 1740
gcctcctgaa tagctaggac gacaggtggt taccaccatg cctggctaat tttaaatttt 1800
ttttttttgt tttttgagat gaagtcttgc tctgtcaccc aggcttgagt acagtggcac 1860
aagcttggct cactgcaacc tctgtctccc gggttcaagc gattctcctg cctcagcctc 1920
ccgagtaact gggactacag gcactcccca caatgcctgg ctaatttttt ttttgtttta 1980
gtagagacag ggtttcacca tattggccag gctggtctcg aactgctgac cttgtgatct 2040
gcctgcctct gcctctcaaa gtgctgggat tacaggtgtg agccaccatg cccggccaat 2100
ttttaaattt tttgtagaga cagacaatac aaaaatgtgg acactatgtg gagacactat 2160
gttgaggtac tatgctgtcc agattggtct tgaactcctg gcctcaagca atcctcctgc 2220
cttggcctcc caaagtgctg ggattacaga cctgagccac tgcacccagc cccctagtat 2280
ctcttataat gtgacttgct ttttcttttc tttctccttc ccttttcttt catttctttc 2340
tcactctcga gagaagagtg ggcatctggg agagtgggag gctggtgggt cccacagagt 2400
gaggaggcag gactgggtcc aaggcagtcc tgcctctcca ctctaggggg tatccttgga 2460
cagtgtctct tctgggaagg ggctcgtctt tctttctctt gtaggcacag tttctctgga 2520
agaagatgca agtacccacc aaccttaccc tcaggaatct gcaggctctg ggcaccctgg 2580
caggaggcat gtcctgtgag tttctgcagc agatcaactc catggtagac ttccttgaag 2640
tggtgcacat gatctatcag ctgcccacta gagttcgagg gagcctgagg gcctgtatct 2700
gggcagagct acagcggagg atggcaatgc cagaaccaga atggacaact gtagggccag 2760
aactgaacgg gctggatagc aagctactcc tggacttacc gctccaaagg agactccagt 2820

ctcaggggaa gtgctggaga ccttaggccc tttggttgga ttcctgggga cagagagcac 2880
acgacagatc cccctacaga tcctgctgtc ccatctcagt cagctgcaag gcttctgcct 2940
aggagagaca tttgccacag agctgggatg gctgctattg caggagtctg ttcttgggaa 3000
accagagttg tggagccagg atgaagtaga gcaagctgga cgcctagtat tcactctgtc 3060
tactgaggca atttccttga tccccaggga ggccttgggt ccagagaccc tggagcggct 3120
tctagaaaag cagcagagct gggagcagag cagagttgga cagctgtgta gggagccaca 3180
gcttgctgcc aagaaagcag ccctggtagc aggggtggtg cgaccagctg ctgaggatct 3240
tccagaacct gtgccaaatt gtgcagatgt acgagggaca ttcccagcag cctggtctgc 3300
aacccagatt gcagagatgg agctctcaga cttcgaggac tgcctgacat tatttgcagg 3360
agacccagga cttgggcctg aggaactgcg ggcagccatg ggcaaagcaa aacagttgtg 3420
gggtcccccc cggggatttc gtcctgagca gatcctgcag ctcggtaggc tcttaatagg 3480
tctaggagat cgggaactac aggagctgat cctagtggac tggggagtgc tgagcaccct 3540
ggggcagata gatggctgga gcaccactca gctccgcatt gtggtctcca gtttcctacg 3600
gcagagtggt cggcatgtga gccacctgga cttcgttcat ctgacagcgc tgggttatac 3660
tctctgtgga ctgcggccag aggagctcca gcacatcagc agttgggagt ttagccaagc 3720
agctctcttc ctcggcaccc tgcatctgca gtgctctgag gaacaactgg aggttctggc 3780
ccacctactt gtactgcctg gtgggtttgg cccaatcagt aactgggggc ctgagatctt 3840
cactgaaatt ggcaccatag cagctgggat cccagacctg gctctttcag cactgctgcg 3900
gggacagatc cagggcgtta ctcctcttgc catttctgtc atccctcctc ctaaatttgc 3960
tgtggtgttt agtcccatcc aactatctag tctcaccagt gctcaggctg tggctgtcac 4020
tcctgagcaa atggcctttc tgagtcctga gcagcgacga gcagttgcat gggcccaaca 4080
tgagggaaag gagagcccag aacagcaagg tcgaagtaca gcctggggcc tccaggactg 4140
gtcacgacct tcctggtccc tggtattgac tatcagcttc cttggccacc tgctatgagc 4200
ctgtctctac agtagaagga gattgtgggg agagaaatct taagtcataa tgaataaagt 4260
gcaaacag 4268

<210> 153
<211> 3673
<212> DNA
<213> Homo sapiens

<400> 153
ctcctcctct cagcacctgg cgcggcgcta cgcggccctg gccgccgagg actgcgccgc 60
tgctgcccgc cgcttcctgc tatcctcggc cgccgccgcc gccgccgctg ccgcctcggc 120
ttcgtcgccc gcctcctgct gcaaagagtt ggggctggct gcggccgccg cctgggagca 180

gcagggccga agtctcttcc tggccagctt ggggccggtg cgcttcctgg ggccgcccgc 240
cgccgtgcag ctcttccggg ggccgacacc gtcaccggcc gagctcccta cgccccccga 300
aatggtgtgc aagcggaagg gggccggggt ccccgcctgc accccctgca agcagccccg 360
cagccgctct gccctccgcc ctcttctccc acctccgaag gtgcccccac cgaagctggc 420
ggggacgctg tccgagccgg gggcaccgcc cccttgtccg cccagcagca gcatgaatgt 480
ggcgacgcgg actgtcggga gtcccccgaa aacccctgcg actgtcacag ggagccgccc 540
cccgaaaccc cagacatcaa ccagctgccg ccgtccatcc tgctcaagat attttccaat 600
ttgtcactgg atgagcgttg cctttccgca tcattggttt gcaagtactg gcgtgacctt 660
tgtttagact tccagttttg gaagcagctg gatcttagta gtcgtcagca ggtcactgat 720
gaattgttgg aaaaaattgc atcaagaagt cagaatataa ttgaaatcaa catttctgat 780
tgtcgcagta tgtctgataa tggcgtatgt gttttagcat ttaaatgtcc tggacttctt 840
aggtatacag cctacaggtg taaacagctt tctgacacct ctattattgc ggttgcctct 900
cactgtcctt tacttcagaa agtgcatgta ggcaaccagg acaaactcac tgatgaagga 960
ctcaagcagc tgggctcaaa atgcagagaa ctcaaagata ttcatttcgg ccagtgttac 1020
aagatctcag atgaaggcat gatcgtcata gctaagggct gtctgaaatt acaaaggata 1080
tacatgcagg aaaacaaatt agtgacagat cagtcagtga aagcatttgc tgaacactgt 1140
cctgagcttc aatatgtagg cttcatgggt tgttcagtca cttctaaagg agtcattcac 1200
ctaaccaagc taagaaacct ttccagcttg gacctacgtc atatcactga actggataat 1260
gaaaccgtga tggaaattgt caagaggtgc aaaaatctta gctctctcaa tctctgtctg 1320
aactggatca taaatgacag gtgtgtggag gtcattgcaa aggaaggaca aaacctgaaa 1380
gagctatatt tggtgtcctg taaaatcaca gattatgcac tgatagccat tgggcgatac 1440
agcatgacaa tagagactgt ggatgtcgga tggtgtaaag aaatcacaga ccaaggagcc 1500
accctgattg cacagagcag caagtctctg agatatttgg ggctgatgag atgtgataaa 1560
gtcaacgaag tgacggtgga acagctggtg cagcagtacc cccacatcac cttcagcacc 1620
gtcctgcagg actgcaagag gaccttggag agagcctatc agatgggctg gacccccaac 1680
atgtctgccg cctcctccta gcgctcctgc ctcgcctagt ccactgggat cattcagcag 1740
agcagaggag aattgtacat ttggggagct gatctctcgg aagggtttta actgtcacct 1800
gtctgtgtgt gttcaagtat gtgtatttgt gtcttgtttg catactgcat accaaaagtg 1860
cagttgtcat ttgttcccaa gtgagctggc tttttttctt agaaattaaa gattatgaaa 1920
gccacaaact ttttagtttt taacttcaaa ggcttctttc tctttaaaaa aaaattattt 1980
ttaatataga gtcaaaaaat tggatgtatt attttgagct tctaattgct gccacttgga 2040
gatgtccaag taagaaggcc ttctttttac atgggatgaa ttgtgcactt ctactgatga 2100
tgactacagc agaagtgatg ttatacgtac aggcacttac actctctcac atattcacac 2160
gtgtgcacat ctacaccagg gctctgatct actctagatc gtttcctgac agagaagcct 2220
tcttccaggc cactaaggta gcatgttatc cactgtggca ctgcctaact ccgtgtatcc 2280
tcgggtggcc aacttgctat ctggtgggtg acttggggac tagaaagaat aaagcctttt 2340

ctaaatttgt tatatagagg caacagtctg aactctcctg gccagctcac gaaagttaat 2400
tatacattat tctacaaatg cattctgttg agcactaaat attttagttg gttttatgtt 2460
aacattgtgt tagcttttat ttcctggata gtaactggtc aggatatatt ttaatgatat 2520
ttgaaactaa tcttagcatt gaagccacca atgtaattaa tggtgtgcag ttattttaaa 2580
aactacagat catttcatta ttttcttaat tttggtaatt attaaagtct atttctgaga 2640
tacgagaggg tagtcacatt aatagaagat tcatgagtga gatataagta gacaatcttt 2700
ctactgttgt tgctcttttt gctttgcttt gggttcttga acaggttttt tggatatatg 2760
catgttgtca ttttggacta gttctcatgc tactgcaaag atctattgct agctaatata 2820
tgagttaaga aagaaaatta gaaaatctgt tcattgaaat tctgtttaca cttgtcagaa 2880
ataagcattt ctttgtttaa ttaaggattg ccagcttcag ttaagacaag acctatcagt 2940
aaatgaatat atgttttgat ttaacacatc tttacaatta attcataata gggactcagg 3000
ctcaaataac tgttcctgtg tctagattta taatgaggac atcagggagc ctttttaagc 3060
taaagagtga ttatctttca cagtagaact agaaacaaaa ccctaacttg tgaagttgct 3120
atttattttt ccctgagtga atataactgt tatttggtct ttaacagata tactgaggtt 3180
tgagtttcac tttctttaag tctcacattg ctaacatgac attagctgta agaaacaaga 3240
tagctttaca tttgaatgcc atttactgat ttctaatgat tctctttttt tgtaaacaac 3300
tgtttccttt gattatgtca acatgtttct gatgtggcat ttttgtttta atcccaatcg 3360
gttaggaaaa tctagtcagt aagcgccatg agtattcttc ccataactaa atgggtttct 3420
aatttaattt gaagggaata aaatttgtgt aagaaaaaag ctgtttaaca ttaataaact 3480
atatttcttc ttacacattg aactgtatca ttttcatatc cacttcccca ccactgaaat 3540
atcttatttc acaatatttt aactgatttt taatccaaaa ctaatttata agacagtatg 3600
tatagtaata gtagcttgag tgaatatgca aaagtttaat ttttatatat gtcatactat 3660
attttaaata gtt 3673

<210> 154
<211> 3920
<212> DNA
<213> Homo sapiens

<400> 154
aatcagtgca tcttagaggt attacaaaat gtttgagctc aaaagattct taccctcggt 60
caggaaagct tcaagaggaa ttgctactta ctcagccttc cagagagtat gaggcacaaa 120
tagagggcac aggtgagcat agggtgtatg aaagcagcag cttgtgacca gggtaaggat 180
gtgtgagcaa gtgcagtgtt tcactcctct taggttaaag aaatgccttc aacttttgtt 240
cataatctgt atttctattt cttttgtcca cctctgctgg tcataatggt ccttaaaaac 300

tatatatata gacataaaat gcattatttc ttttcttttc tttttttttt tttttttgag 360
acggagtctt gctctgtcac ccaggctgga gtgcagtggc gcgatcttgg ctcactgcaa 420
cctctgcctc ccgggttcac gcagttctcc tgcctcagcc ttccgagtag ctgggattac 480
aggcacatgc caccatgccc agctaatttt ttgtattttt ttagtagaga cagggtttcg 540
ccatgttggc caggctggtc tcgaactcct gacctcgtga tccacccgcc tcagccttcc 600
aaagtgctgg gattataggt gtgagccact gtgcccagct gaggtttgta gactttctaa 660
agctctggta cttaacacat acacacacac acacacaccc cctcacacac atgctccaat 720
tcctgatacc tttgtgcaca actccttttt tatagaaatg gacccagaga tcttccatgg 780
caggaagtat gtgataaaga tactggaatt tctttgtctc caaatcttgt cagaaataat 840
gtgagccctc acccccatag aaaacctcat gtcattgttt ttatttggtt ttgtttgttt 900
ttgttttcta aattgagatg cagtctttgg gaggcctagg tgggaggagg attgcatgat 960
gccagtagtt tgagcccagc ctggtcagta gagcaagacc ttgtctctac caaaaatata 1020
aaaattagct gagcgtggtg gtgtgtgcct gtggtcccag ctaattgggg ggctgaggtg 1080
ggaggattgc ttaagccaga agtttgaggc tgctgtgagc cacagttata ccaccacacc 1140
ctagcctggg ggacagagtg agaccctatg tgaaataaat aataaatagg tgagatagat 1200
aaatagatag atagagagat atggagctgg ctgtgttgcc cagattggtc atgagctcca 1260
gcaatcttct tgcctcagtc tcctgaatgg ctgggactac aagtgctttc tgctatggcc 1320
tgctatttag gatttttatt agggggcact atggttagat ttgctagggt aagtatattc 1380
tgtataactt aagtcatttg ctggaaaaga cagttaggcc gggtaatatg ggaatagtct 1440
ttttatcgac caataatgct ttttcaagaa agaaaaatag taggtgatta atttttgttc 1500
aaagagatgg aacataaaaa cgtgacattt tcttaaagtt gtgtgtaact aatgatgcta 1560
aagagccata tgcacttttt atcgattgac tgtgcgggat cctgctttgt tgcctaggct 1620
ggagagtagt ggcatgatca tagctcactg cagccttaaa ctcctgggct caagcgatcc 1680
tcccacctca gctactctgg aggctgaggc agaagaatca tttgaacctg ggaggtgaag 1740
gttgcggtga gccaagatcg cgctactgta ctccagcctg ggcaacaaga gagactcttg 1800
tctcaaaaaa taaataaata aataaataat gaaaaagaag ttacgtggcc agtcttaaga 1860
ctcttgccag aacaagaatc tgacaaatgg ttttaaaaat gtatctaaaa gacacaagta 1920
tatttttaat ttactctcct gatagcttga aatctgttta taagtaacgt tggtttcttt 1980
taacagattg cctggaatgg aaaaactagt gatgtgtttt ttgtcttcca taaggactga 2040
gagagtatat taaaatataa ttatcaagac acttctttgt tgaacataga aattacatag 2100
tctacatgat cgtttagtta ctgttgaaca catttcctga gtatattcta gcttagggag 2160
cttaataagt gcttgataat tgagacaagg gacctagctt ataaattcag tgagtttacc 2220
gggtggtaat tcctttgaat tatgatgaag agacatagac atttggggct ttttgttttt 2280
ttccaaattg aggaggaata gggcagagcc gagttttctc attttagctt tgtttagcca 2340
tcatttttaa acagctgaat ggggttaggg gcagtccctg gccaaggcat ggagctgtgc 2400
taaccagccc acatgctgat gtagaacaag tgcacattgg agtggtcttg acattttgct 2460

aatgttgaag tcatgcctta tctttttggc tttaaaagga gaaaattatc aaagccccat 2520
cgttctttgg cagcaaagag ggtaatttgg tgaatactga tgttttactc tcagggacac 2580
tatcaagtgc ccagaatttc cttggtgctc tttggaacat agaagaaaac ctgactgagg 2640
cattcttgag ttgcttttta gtctgtgaac tataatattc atgagacgct taacacttcc 2700
caaagtggca gggaggaaac tctttgtttc ttctgattcc accggaagag agagtggaca 2760
ggacccagga cttttcggag tagagttagt gattcatctc ttgtttgtga gatagatggg 2820
gcttgagtag tgagtatccc tttgggacat ttttttgagg ggaagtgaga ggcagcatgc 2880
ttcatttatg cacagtgatt gaggtcgctg aataatagat tatatgttgc aaacattatt 2940
aagcactata agccttgtta tgtcagctgc tagatatcat atgtatttat tttactatat 3000
tcagttccaa tatgtgtgta tgacttaata tcttcattaa tagattttct ttttttctga 3060
gacggagttt tgctcttgct gcccagactg gagtgcagtg gcgcgatctt ggctcaccga 3120
aacctctgct tcccggtggt aggtgattct cttgcctcag cctcccgagt agctgggatt 3180
gcaggcaagt gccagcacac ccggctaatt ttgtattttt agtacagacg gggtttctcc 3240
atgttggtca ggctagtctg aaactcccga gctcaggtga tctacccgcc tcagtctccc 3300
aaagtgctgg gattacaggc gtgagccact gcgcccagcc tgatagattt tcttactaca 3360
aatattcaat aacttttaaa tttgtatcat ctgacttaca aatatctttt cattgtgcat 3420
tcattcataa gtagactgaa aatttgtaaa tttgagatgg gcaacacaac tcctcacaat 3480
tatgtagcta tttcttttat ctcatctcta aacctaccct tccattatct gctttgtgat 3540
gctcgggctg ttgtactcat tatattaata tataacccag tcttatatat aactcgtgta 3600
atatagtgaa ttcggcttta aaatattcta atattcagct gggtgcagtg gctcatgcct 3660
gtaatcctag gactttggga ggccggggtg ggaggattac atgaaccttg tagttaggga 3720
ccagcctggg caacattggg acaccctgtc tctataaata ataaaaagat tggcatggcg 3780
tggcggcgta tgcctgtgtt cccagaagtg tttgggaggt tgaggtggga ggattgcttg 3840
agcccgggag ttcggggctg tagtgagcca tgattgtgac actgtacttt ggcctgggtg 3900
acagagcggg actctgtctt 3920

<210> 155
<211> 3168
<212> DNA
<213> Homo sapiens

<400> 155
cctgcaccct ccctagctgc cctttgcatc acctctgctc ccagctccct ccaggctcc 60
atcatccccc atcccattgg atgcccagct tctgtggagc ttgggaaggg ggtggcagta 120
ttccctgggc agggggatgg aagagaccac agggtcatag gccacagtcc tagtccccct 180

tcttaccccc acccctgggg aagggagtcc atgtggtgac tcagattaga tcactcgacc 240
tccctgagcc tcagtttccc ttgctgtatg atgaggagaa tcagcctgct ccagaccctt 300
ctccatggtg gctttataga tcagggctgg gaggtggagg aggggacaac aggtgtatca 360
cctgccaaga accacctgac acctcttgcc ttgtccatct ccccccgccc ccatccacta 420
cagtgcccag ggagtgggca gggcccacgc tggggggatg gcctcgatcc tccctggcct 480
cgaccctctt ctctgtcctc cttgtcaccc ttggtgggga ggggagctgg cattccttga 540
agacaacttg cctggccttg ccccatgtcc ctagtgccct gccctagtca ccaggagtct 600
ttattttcta gccatctcct ccatgccttc aactcagctc cttctcctcc caggccctgt 660
ctcaggcctt ctgtccagtg ggctcccttc ccacttcctc tctgtcttcc ctcctagtcc 720
aggcagactc tcctgaagtg gagcctgaca ttcagggggt ggggctgggc tggcagggag 780
tggggatcac cgtggggagg tgcagagttt ctggcccccg ctctgtgcca ggagctaggc 840
aggagccccc tgtctccaat cctcagtcca gctgcctcct tccccccatc gcccttcccc 900
catcacacgc cctcatctcc gaggctttag ttggagtgac agggagactc aacagggcgc 960
tgggtattag caatttgggg ggatattaca caggaatgtt tacagaaagg caattgcccg 1020
gaaaatgact ttggttgctg tggtatgggg cggaaaagat ggttttagaa aacagacaca 1080
gatgggcttc tgatccaagg acccacagcc agccagcaga aactgaggag tatgggcaca 1140
caaggaaggc agagccaggg gcacccaggg acagggaggc cgattccttc actagcaggg 1200
gaagcaaagg atcccctctc ctaattcggg ttcccagagc ctctgcctag tttgatacat 1260
atctggtccc ggttgacctt tgtcctctgg cctccaacct ccaagcacac cgtgtgcttc 1320
ccgactgcag caggcaccca gcaggatgga gtcatcatgc acaatcgaag gtggcagtgc 1380
ctcggggctc ctcctggctg acttgggctc ctgtagcatt ggctgcagag gagggactgg 1440
gggagcctca tgctgtgtgt ttgtgtgtgt gtgtgggtgt ggcctaaggc aggcaggtcc 1500
tccacccttc cagctgtctc catgctcctc tcctgctgcc ctgcctagcc ccatgtcctg 1560
tgggcttgtg agttggtgtc ttcaccctga gtgcctcctg ccctcgcttc ctgccagggc 1620
tataacccag ctcctacccc tgcccaggca gtgggtcggg gcacacatgt gacagcctct 1680
caggtcccca agggaggggt tttgtccagc ctccttatct ccacagagag ggttacaggg 1740
acctgatttc cagatcttga gtttggggct ggggaggact cacatcaggg gccccagaga 1800
gttctcttct ccctttcctg agctggtggc ttcgttcttc ctctactttg tccatttgcc 1860
tctctccttc cttctccctc cctctcctct cctctccccg ttctcctcct ttgcagatga 1920
catcatgtgt gaatgttgcc caggggcagc cagaggacag agtagagggt tacatccggg 1980
gtaaagatgc cagttgggga ccggcttggt ggcttatgag atggtgtcac taagatgttg 2040
ggatcttaat tctcataatc agttacagtg atctcatgta aaagaatgtg gtggaggata 2100
aaaataaatg aggctgataa ataaacgtag aatttggaat tgttcggaca aatgacaacg 2160
tcactcttcc caagcacctg tgccgtcctc ctagccggcc actggggggc gcccgccact 2220
tcccggccac cccagcctca ggccactttc accttccgcg tttcccaggc ggggcaggct 2280
gagctgaggg tggcagaacc cgccttcctc accctgccct tcccctcatt cggattctgg 2340

atttcgccag ttcttggcat cttccctgtg gcttgaatct cggactcaaa aaatatagtc 2400
agccttgcac actgaggttc cttggagaaa agagaagccc tgagccccct cccttatgtc 2460
tctggtttcc ccaaccccat ccctgggttg gggggaacct tcaggcccaa cgcccctcca 2520
ccagggtatc ctagctttgc ttcccttccc ccagagttcc ctgcctccgt ctctctctat 2580
cgcaatgccc ctatccctct cccagcagaa cccagccatg cttaagaccc cagcccgggt 2640
ctctcatgcc ctccctgcct ggaacaggac gcattgatct gtgcagggct ggcaggtgcc 2700
tggatccgct tgctagattg gaatcgatcc ctgtgtcttt tccctgggct gctcaggtcc 2760
gagggggggtc agggccactc cgaggacttg ctgatggctg gaacaggcgt aggggtaaca 2820
gggaggtcag gctgctacag gcccatcgct gctcaggaat gtctttcatt gaaagcaacc 2880
tactttggcc tggcgcggtg gctcacgcct gtaatcccag cactttggga ggccgaggcg 2940
ggcggatcac ctgaggtcag gcgttggaga ccagcctggc caacatggtg aaactccgtc 3000
tctactaaaa atacaaaaat tagctgggta tggttgtggg ctcctgtaat cccagctact 3060
cagaaggctg aagcaggaga gttgcttgag cccgggtggc agaggttgcg gtgagccgag 3120
atcgcgccat tgcactccag cctgggccat agagtgaaac tctgtctc 3168

<210> 156
<211> 3466
<212> DNA
<213> Homo sapiens

<400> 156
   accattccac gcggctcgag cccgcgtgcg ggcctctttc aggccgctcc tagtggacgc 60
   agaggcgggc cgaggacggt gccagccccc aggccccaag aggagctggt ccccaggaca 120
   gaggagggag aggagcaaga ggctcccctg ggccccttcc aggccccacc tccaggtcac 180
   aggcgcgaga tggagtcccc aagagggtgg accctgcagg tggccccaga ggaaggccag 240
   gtcctctgca atgtgaagac tgccacgagg ggcctctctg agggggctgt gtctggaggc 300
   tggggggcct gggaaaactc cacggaggtt ccgagggagg caggggacgg ccagcggcag 360
   caagccacac tgggggcggc ggacgaacag ggaggccccg gcagggagct gggccccgca 420
   gacggtgggc gggacggggc tgggcccagg agcgagcctg cagaccgggc gttgcgccct 480
   tcgcctctcc cagaggagcc gggctgccgg tgcggggagt gcggcaaggc gttcagccag 540
   ggctcttact tgctgcagca tcggcgcgtg cacacaggcg agaaaccgta cacgtgcccc 600
   gagtgcggca aggccttcgc ctggagctcc aacctcagcc agcaccagcg catccacagc 660
   ggcgagaagc cctacgcttg cagggagtgc ggcaaggcct tccgcgcgca atcgcagctc 720
   atccaccacc aggagacaca cagcggcctg aagcccttcc gctgcccgga ctgcggcaag 780
   tccttcggcc gaagcaccac gctggtgcag caccgacgca cgcacacggg cgagaagccc 840
   tacgagtgcc cggagtgcgg caaggccttc agctggaact ccaatttcct ggagcaccgg 900
   cgcgtgcaca cgggcgcgcg gccgcacgcc tgccgggact gtggcaaggc cttcagccag 960
   agctccaacc tggccgagca cctgaagatc cacgcgggcg cacggccaca cgcctgtccc 1020
   gactgcggca aggccttcgt gcgtgtggcg gggctgcggc agcaccggcg cacgcacagc 1080
   agcgagaagc ccttcccctg cgccgagtgc ggaaaggctt tccgcgagag ctcgcagctc 1140
   ctgcagcacc agcgcacgca cactggtgag cggcccttcg agtgcgccga gtgcggccag 1200
   gctttcgtca tgggctccta cctggcggag caccggcgcg tgcacacggg cgagaagcct 1260
   catgcgtgcg cccagtgcgg caaggcctcc agccagcgct ccaacctact gagccaccgg 1320
   cgcacgcact cgggcgccaa gcccttcgcc tgcgccgact gcggcaaggc cttccgcggc 1380
   agttccggcc tggcgcacca ccggctttcg cacacgggag agcgaccctt cgcctgcgca 1440
   gaatgcggca aggccttccg cggcagctcc gagctgcgcc agcaccagcg cctgcactct 1500
   ggcgagaggc cgttcgtctg cgcccactgc agcaaggcct tcgtgcgcaa gtcggagctc 1560
   ttaagccacc ggcgcacgca cacgggcgag aggccctacg cttgcggcga gtgcgggaag 1620
   cctttcagcc accgttgcaa cctcaacgag caccagaagc ggcacggggg ccgcgctgcg 1680
   ccctgacccg aggacgccct gagcgggagg tcgcggacac acggcattgc ggggtctcgg 1740
   gcgtgagtgc gctgtctgct ggcccagact ttttcgggcc gccggtgcgg gcgccctcct 1800
   gctgggagtg caggggcggc cttgggtgtg gagaaccctg gccgcacagt ccctttgacg 1860
   atagtccacc ggccacccag gcctgtctgg ggacatgtag gatgggctct taccccaggg 1920
   agggcggcag gctccacttc ggcgagaggt tcgtccatgc agaggtgggc aagaactggg 1980
   gtctccgaca ggtgtggcta tttctttgag ttctctggca ctgtcaaaag cagccaaccc 2040
   accccccagt ccacatggtc accactgctg ctaccagctg ctcagtgcag tggccactgt 2100
   gtctcctaag gtgctcgctt cagtcagcac ttcatctcag gcaaccacag gtgacagtta 2160
   aacatgatga aaccgcatgc tatggctttc tagtgtctca tattctgttg gcaagaagct 2220
   cagcactgca ttcctgaccg aggtcagaac cagatcaatc tcagaatctc acctgtgtag 2280
   gtctgtttca tgggactttt cttttttggg ggagggggca gggtttcact ctgtcaccca 2340
   ggctggagtg cagtggtgca atcactgttt attgcagccg cgacttctta ggctcaggtg 2400
   atcctcccac ctcagcctcc caagcagctg ggatgacaga cttgcgtcac tacacctggc 2460
   taattttaaa attttttgta aggacagtgt ctcaccatgt tgcctaggcg ggtctcaaaa 2520
   ctcctgggct caagtgatcc tcctgcctca gcctcccaaa gcgttgggat tataggcgtg 2580
   agccaccgca cctggccagg gaaccttctt tataccaagt accacaccag tcagagtcaa 2640
   gtcaggaaat agaaaccaca ctaggtattt caaacagagg ggatataagt tagggactga 2700
   ttgtgcaggt tttgcaaggc tcagagagca aaagtggatg ttgcagaatc tcagagctgg 2760
   atacttgcag gaagctgcta ccacccttag ggctggagaa cccagggaag ctaagaggag 2820
   ggtgcaacga ggctggtgtt gggactgcca aaggaaacac agaatgacag ctccatctct 2880
   gcagacctca gatttgacca ggcctctggc tgcctgggca gcctcattgc caatgggctg 2940
   aaaggttctg tcactgttca gtggtctgac ttctgagttc tgtgcacacc ctgagctctg 3000
   ctcctctggc ctggccacca gtccttgtct gagtgcccca ggtctggtta tgacttcagg 3060
   cctcagcacc tggttgtctc ttctgcaaag aatgcattct cccccagtcc acccagaaca 3120
   cacagcctcc atccaggtat tggctctgag caggacagag agcatgcagc aggtgctcag 3180
   caatatggaa atgggaccaa acagagggag tctccacagc tccccgtccc tcacagcaga 3240
   agccagagcc gctgcagtgc ccagctggtc ttcatcacgt ctatgagctc tgcaatcgct 3300
   ctgcagtcgc ctcaactcct ggtctcctct gctccttctc actgcacccg catctcccat 3360
   gtttgcactg gctgttccct ctgcctggaa tgctccttac ccagttatcc cactgtcttc 3420
   tttgcatctg gcacacagta gatgctcaat aaatgcctgt ggaatg 3466

<210> 157
<211> 3100
<212> DNA
<213> Homo sapiens

<400> 157
aggtgccgcc cctgccacag cctctagcag ctctcagtgg ccccatcctg aaagtgggtt 60
gggggtgtgg ggtcctgcct ggcctcccct ccctggcata acttctctgg ccagcactcg 120
gactggggac ccaggtccct tggagagcag ctctccttgc ctcccaaggg ctcagatgct 180
gctggcagag ttgtctcggg agcggggtga gctgcagggt gaacgcgggg agctgcgggg 240
ccggctggcg cggctggagc tggagcgggc acagctggag atgcagagcc agcagctgcg 300
cgagtccaac cagcagctgg acctgagcgc ctgccggctg accacgcagt gtgagctatt 360
gacacagctg cgaagtgccc aggaagagga gaaccggcag ctgctggctg aagttcaggc 420
cctgagccgg gagaacaggg agctcctgga gcgcagcctg gagagtcggg accacctgca 480
ccgcgaacag cgggagtacc tggaccagct taatgccctg cgccgcgaga agcagaagct 540
cgtggagaag atcatggacc aataccgcgt gctggagcct gtgcccctgc cccggaccaa 600
gaagggcagc tggctggcag acaaggtgaa gaggctgatg cggccccggc gggagggggg 660
ccccccctggg gggctgcgcc tgggggccga tggggctggc agcaccgaga gcctgggggg 720
ccccccggag acggagcttc ctgagggcag ggaggcagat gggacagggt ccccttcccc 780
ggcacccatg cgccgggccc agagctccct ctgcctgcgg gatgagacct tggcaggcgg 840
gcagcggcgg aaactcagct caaggttccc ggtggggcga agctctgagt cattcagccc 900
ctgggacacc cctaggcaac gattccgaca gcgccatcca ggccccctgg gggcgcccgt 960
ctcccacagc aaaggtgagg gacaggggtc actgtaccag ccagcccccc aactctttgt 1020
ggacccacca gctccttggg ggaggaggct tccttcttgt cccctgtgtc tcctgcaagc 1080
tctgtccact tccgcactgg ccctgggccc agtggttgcc ttgtgcctcc caggacctgg 1140
tgtgggatgg gagaactccg ctgagaccct gcaggaacac gaaacagatg ccaaccgaga 1200

gggtgagtgg gggactgtgg aaggagtagt attctttgtc ctgcctgggg cccctggcag 1260
aacctcattc atccattctt tcattcgaca atgatccttt accaagtgcc aggtgacgtg 1320
ctggcagggg acacagcagg gaacgttctg gtgtgaggag gcagatagta aacaatttgc 1380
ctaaaaataa ttattatcta caacagccct gtccactaga aatatggtgt gaaccacatg 1440
ataattttaa cttttccagt aggcttacca gaaaaagtga aaagagacag gtacaatgga 1500
ttttaataac atttaactca atatgcctac gctattacca tttcagcatg tagccagtgt 1560
aaaaatcatg aatgagctat tagacgtgat tttttttttt tttttgagat ggaatttcac 1620
tctttggctc actgcgacct cctcctccca ggttcaagcg attctcctgg ctcagcctcc 1680
cgagtggctg ggattgcggg catgggccac cacgccaggc tagttttgta taattattat 1740
tatttttttt ggtagagacg gggtttcacc atgttggtcg ggctggtctc ggactcctga 1800
cctcgggtga tccgcccgct tcagcctccc aaagtgctgg gattataggt gtgagccacc 1860
cagccagacg tgattttttt catagtaagt ctttggcagt ttcggaggcc gaggcgggcg 1920
gatcgcttga ggccagaggt tggagaccgg cctggccagg gtggtgaaat cccgtctcta 1980
ctaaaaatgc aaaaattggc caggcgtggt gtcccatgcc tgtggtccca cctgctcggg 2040
gggctgaggc gggagagtcg cttgaacctg ggaggcggag ggtgcagtga gccaagatca 2100
cgccattgca ctccagcgtt ggtgacagag tgagactgtc tcaaaaacaa aaaacaaaaa 2160
acaaaaaaac atagtaggtc tttgaagcac gtcacaggcc aaccccatct cacctactta 2220
gctccgccct gggcagctca gccctagacc cggctggacc atcaggaggc gctaatgctc 2280
tgggaagaaa ggagagcagg ggaggggtca ggagctcagg agctctgggc aggctggttg 2340
gagttttagt aactggggtg ctggggtggg cctcagagag gatgtgaaat tggagcaaag 2400
acttggaggg gagagtttgt cgtgtggaca tacctgagaa cagcattctg ggcagaggga 2460
accgctgagg gaacagcagg cgtggtgcag ctggaagcgg ggggtattgg caggtcgggg 2520
gtgggcagaa ggtgctcgag cctgtgggcc tcctgggact ctgggagtcc tcctgaggga 2580
agcagatctc agctgagcct ttccctctag gccccgaggt acaggaaccg gagaaacgtc 2640
ccctcacccc atccctcagc cagtgacacc gtgggaacag caggcttggg agtgcagcct 2700
tctcggcact ggagtgtcag cagaggcccc aggcagccca agagctcagg gagccaggga 2760
ccccaagggg agtccttgga caaggaggcc tgggccctga gatcctccac ggtcagcgcc 2820
ggggcccgga gatggagctg ggacgagtgt gtggacaggg gggatggctg gcccccacga 2880
gcagctccag gctggagttc tggttcttcc aggtggctcc cgttgaggca gcggtctctg 2940
ggggatcccc cagctgaagg aggctggcag gagttggcaa gagaaccccc tgccctgtcc 3000
aggtgggaag ctgagtccca gtgctggggg actgtggccg gggctgatct tgagccttaa 3060
ctggacatga ggggcatgaa aataaagctg aactgcagcc 3100

<210> 158
<211> 5109

<212> DNA
<213> Homo sapiens

<400> 158
gctctcctgc cgcttcgctc cgcgctctcc tgccgctccg ctccgggtct cccgcgctcc 60
tctccccggc tcggccgagc gcgctgcccc gacgccgcca cccagagccg ggccgcgccg 120
ggcgccgaga tgaaggtgct gggacaccgg ctggagctgc tcacaggcct cctgctccac 180
gacgtgacca tggccgggct gcaggagctg cgattccctg aggagaagcc gctgctccgg 240
ggccaggacg ccaccgagct ggagagctcc gatgccttcc tcttggctgc agacacagac 300
tggaaggcct cgggcgagct cgccacccag atccttgctg cccaggagaa cagggcacgg 360
agcctgagat ggacctggtg ttctgctccc cagagacccg agagccgggc cccaggctgg 420
ttgcctctgg gaacgagggc cgggtgggtc tgcagggggt gggcatgtca atgtcagcaa 480
gctgcctcct ggccccgctt cctctccctg gtggcctgga gcccctggca gcagggtgag 540
ggtggggagg gcagagctgg tgtgtgcctg accatacccc tggccctgct atctgcccag 600
ggcccctggc ccccggcccc caaactccat tttcagcatg tggggaaacc ccctgtatct 660
gcgtgggcaa ggtgggattt tcatccctgg tgggtggaga aggagcagcc ccttgaagca 720
gggtgataac ccaagttgcc ccagaaagca gcagctcccc aggcctccac aaggttaagc 780
gatggaacct tcctaatggg aaggctgggc gtgggggtct ggtccccaag atgttgggct 840
tggggccctc tcgggccctc catactcccc aaggtgtggg tcaagggctc tagctgtctg 900
ggcagtgccc atgcaccctg ccctgccccg cattggtaag ctgtgcatgt ctgtgacgac 960
cctgtgttta cctgacatgg gtgagtctgt acgtgtggtc tcggcttttg tattcggagg 1020
atcccgtgtg agctgcagct gttggcgtgt gcctatgtgc tgggggcgga tgcaggggct 1080
ggggaccatc aaggatgatg tgtggctggg gtgggaggag cagtgacagg gctggggcaa 1140
ggaggacttc agaagccaat tggagccagg cttgtctccc agcagccaat ctgggagctg 1200
gggctcctgg taagcgagtg gctgctcaga aaccctgcgg ggaggagggg ctagctaggc 1260
tgggccagga tccctgtata aatggcccag gagctgtgcc ccatcacaga gccgaccatc 1320
tcccactcga gctgcccccg ccctctggac ccgagtgact caggcctttg tttgtccttc 1380
ctggtagagg cgggttccct ccctcggcaa gatgccggag tgctgggatg gggtgagtga 1440
gggcgctgcg ggcatcaagg tgggccggga ggatggtgca tccttatgac ccaccgcagg 1500
gaggaagtgc ccccctcaac ccacatctgg tggcagcccg gccttcaaat agcctcaccc 1560
tggggtcact gagacacggc cagagtgctc caggttccct ggtgatcctg tttgcaggag 1620
tggctggggg cagctgctag tcagagccca tagcaggggc tggggggagc gtgtggttgg 1680
ggggtgggtg acatatctgc aggaaatgga acgtggaagg cactgtctga cttggctgca 1740
ttgcccgtgt ggacccgggc gctagtgcct gcatgtccct ctgaccgtgt gccctctggt 1800
gtgcgcttgg atggtgtctg ccttggtgat gcctggggcc atcaggccag gggcgggggg 1860
tgtccttggt gagcccccga ggctgtgtgc ccagtgtgcg tgccgggtct gtgcgtgcag 1920

gggctgccgt cccagtgggc ggccgtctag cctgtttgga tggctgcgcc agtgtgatgg 1980
gaaggacagg cggcctcttc cttaggagcc acctcacagt tcccagggct ctgctgcgcc 2040
agccgggcag aggcaaggag gtctgggcca cacaggaatg acagcctttc aggcaggggt 2100
ccctgctggg gaggaacgag gaggggttgc ctgcctgcct tattttgtag gtgggggaag 2160
gcaacgctgg agccgtgaag ctggcagggc taggggaccc caggtggagc ccagggcacc 2220
tcctctcgcc ggggcatcag gaacatgaca tcgagacacc ctacggcctt ctgcatgtag 2280
tgatccgggg ctcccccaag gggaaccgcc cagccatcct cacctaccat gatgtgggcc 2340
tcaaccacaa actatgcttc aacaccttct tcaacttcga ggacatgcag gagatcacca 2400
agcactttgt ggtgtgtcac gtggatgccc ctggacaaca ggtgggggcg tcgcagtttc 2460
ctcaggggta ccagttcccc tccatggagc agctggctgc catgctcccc agcgtggtgc 2520
agcatttcgg gttcaagtat gtgattggca tcggagtggg cgccggagcc tatgtgctgg 2580
ccaagtttgc actcatcttc cccgacctgg tggaggggct ggtgctggtg aacatcgacc 2640
ccaatggcaa aggctggata gactgggctg ccaccaagct ctccggccta actagcactt 2700
tacccgacac ggtgctctcc cacctcttca gccaggagga gctggtgaac aacacagagt 2760
tggtgcagag ctaccggcag cagattggga acgtggtgaa ccaggccaac ctgcagctct 2820
tctggaacat gtacaacagc cgcagagacc tggacattaa ccggcctgga acggtgccca 2880
atgccaagac gctccgctgc cccgtgatgc tggtggttgg ggataatgca cccgctgagg 2940
acggggtggt ggagtgcaac tccaaactgg acccgaccac tacgaccttc ctgaagatgg 3000
cagactctgg agggctgccc caggtcacac agccaggaa gctgactgaa gccttcaaat 3060
acttcctgca aggcatgggc tacattgcgt acttgaagga ccgaaggctg agtggaggag 3120
cagtgccctc agccagcatg acccgcctgg cacgctcccg cactgcatcc ctcaccagtg 3180
ccagctcggt ggatggcagc cgcccacagg cctgcaccca ctcggagagc agcgaggggc 3240
tgggccaggt caaccacacc atggaggtgt cctgttgaag cccttgatcc cgctgacgac 3300
gcccacgtcg aggccccacc gccatccttg cgccggctca tgttcccttt agtttatttt 3360
tgtgagggca aaggggagga aatggggttc tgtttgaaaa aatgaggggg atcttagatg 3420
ctgcagcaga acagtctcca ggtgttttaa ggggctcagt cctcctcatc ccatctcact 3480
ctccgtggta acttagccaa cttgacccct ctcatcccac tcccggcggc ccaggcacag 3540
aagggcaggg ccatagggag ggagattcgc tacggatcca ggccattcct gggtgagccc 3600
ttgggcaggc atgtttggag atgagagagg cttcgagagg gtgggtgctg ggccacaggg 3660
gtgcggggcc agctcaggca ctggcgtggg agccctggga gaccccttcc cccaccctcc 3720
accaagcaca cctgtttctg tctcatagca catgtgacaa tcatctggac aacagccaca 3780
agggggcgct cggaccaggc agccactttc ctggtgctct ctgggcccag ctggtgctgt 3840
agggccacgc aggcaggggc gtcaaggggt ttctctgccc aaggaagaca gaacatggag 3900
aaccgtcagg gcaggaaccc cacagactgt cccttccagc ccacactctg ccacctcctg 3960
gccctgtccc aattctgagc caaggcctcc ccgaggcaga agttgcctgg tcctctgtcc 4020
ccacagtgac ctgactgggg gtgagggaga aggaggagag agcccatgtg tggtgtgtgt 4080

gcccctgaga acttcgtggt gactgccttt gggagcccgc aggtggccag aggcaggggt 4140
agctgagttc ctggagaccc cttttttgcc cccaggttcc ccagagggca acgccatcag 4200
tagcagtgtg gtgtttcagg cagagctctg gccaggctgt gccagtgtgt cccggacgca 4260
tcactaagga agagagagtt tatttagtca actggcccaa ggcagcgagg cttctacagt 4320
cccacacccc atagccgcct gggctggggc ttactggggg ctgaaggttc tggacatgaa 4380
caagggtcag gtagaagaga aaggcttccc ctacacccca gcctcctgct gtcccctgaa 4440
gcccaggact gcgttgtatg ctttccatcc actcacctta ccccatagca tcttgcggcc 4500
cagaaaccag agccatttgt ctcagaccct aaatcaataa tcacaaaccc caaaacggga 4560
gagagcagtg aaaacatgca gggctgtgga cgggggaagg gttgtggcgg gtgttctgag 4620
gctgagagga cacctatatg cgtatttcct ctacacacat cacccccctt ctataatctt 4680
aagccatgac tagcctggtg gcgtgttagt ttctgcccag ttctaccccc tcatgtgctt 4740
cttctgaata ctgaatgtga ctgtttgaaa gctggtagaa ttcatccctc ttacggtaga 4800
taacactgca aatcttggaa ttttgttttt tgctgtttcc agatgtatct ataaatatct 4860
atacattata tgtgtgtgtg tgtgtgtgtg tgtacatcgg gtcctcccat gtgtggtgtt 4920
cttctggagg ttgtctcttt ggtcaaggtg aacttttaat gtttattatt ttcttctccg 4980
cacaaagtaa agagcctaat tttgtgtatt ctggtggctg ctgtcatgag atgataaaat 5040
gtaaaacaaa actctagtca acgtagaaag agttaactgt gctgaaaaac taataaagaa 5100
cctaagaag 5109

<210> 159
<211> 3292
<212> DNA
<213> Homo sapiens

<400> 159
gttttttata acctacccag tttatggtat tttgttactg cagccagaag gaactaagat 60
gctggtttct atcagcataa agcctctgag attcatcgaa gttgttgggt gttctgatac 120
ctgttgttgt tgttttcctg agtagcattc tgttgtatgg atgtaccata atttgtttat 180
tcattcaccc attgaaggat attttagttg tcattttttg tcatcacaaa taatgctact 240
ataactgtgt atagaatttt gtaaatagca gtttttattt ctctagagga aatacccagg 300
agagggattg catggttgga tggtcattgt atgttacaca tttttaagaa actatgaaag 360
tgttttctct agtagtgtct gtactatttt gcattccact aggaaggtat gagggtttta 420
gttgctctgc atccctgcca ccccttggta ttgtcagtat ttattttttt tctgaggtgg 480
ggtctcactc tgtcacccag gctggagtgc agtggtgtaa tactggcttg cttcaacttc 540
cacctcccag gctcaagtga tcctcccacc tcagcctact gtgtaggtgg gaccgcagcc 600

atgcaccacc atcgctgcct aatttttttt tttgtatttt tttggtagag actgggtttt 660
gccatgttgc ccaggctgtg tcagtatgtt ttattttggc cattctaata ggtttgagtg 720
atatctcatg gtggttttaa tttgcatttc tctaatgatt gaacaatgtt gatcaactat 780
tcatgtgctt atttgccacc acgtcaaaaa ctgcaaatgt tttttttgta gtagccatcc 840
taatgggtac aaagagatat ctcattgtgg ttttgatttg catttccctg atttaatgac 900
cttgaacact ttttcacgtg attattggct gttagtatgt ctttggagaa ttgtctattg 960
cagtcctttt cccatttgta aaccggatta tcttgttttt tttgttactg agtggtaaga 1020
gttcctttat gttctggata ttaactcctt attagatgtg tggtttgcag atatgttcta 1080
caattctgta tattgttcct tcttactctg tcatttgctt cctatgttgt gcagagatat 1140
taaaatttga tgtaatgtat ttgtctgatt ttgcctttgt tgcctgtgct tttggtgtca 1200
tatcctagaa gtcattgcaa aatccagtgt tgggaagctt ttcccctaag ttttcctcta 1260
ggagttttct agtttcaagc ctatacattt aggtctttaa tccaatgtta gccctttctt 1320
aatgagttct ttgtattttg ggcagggaat aagtctttct ttaatgtaac tgtcaggaat 1380
ttactatatt taactttttc cttcttcctt cctcccctct ttccctccct ccttcccttc 1440
ctcactcatt ccctccttcc ctctcttacg gcttgaagat aatggaatca ctcaggtttt 1500
gttcgtaact ggtaaggttt tagtttagta ggatgataat tcattttcaa cagcactttc 1560
ttgtttgttc attggttagc aggaagaaga tgagccttag gtctgaacgc cgaggaattc 1620
atgtggatcc tgtgcaagaa aggatgtggt tactgtggca accctacctg gcagggtttc 1680
tgctccaagt gctggagggg agagtagcac aaagccaggc agaagcagat tcaggagtac 1740
tgggagctgg tggaacgact ccagcgggag gaagaagagg cctttgccag cagtcagagc 1800
agccaagggg cccaatccct catattctcc aagtttgaag gaaagaaaac caacaagaag 1860
acccgcaagg ttaccacagt gaagaaatct tcagtacgtc ttccagcgtc ggatcaaaga 1920
agggctggag tgcaatggcg cgatttcggc tcactgcaac ctccacctat cgggttcaag 1980
tgattctcct gcctcagcct cccaatagct gggattacag atattgaaat ggattccagg 2040
cgtgtgcctc gagacaagct ggcctgcatc accaagtgca gcaagcacat cttcgatgcc 2100
atcaagatca cctagaacga gctggcgtca gcagatgact tcctccccac cctcatctgc 2160
attgttttga agggcaaccc ccatgccttc agtctaatat ccagtatatc acgcgcttct 2220
gcaatccaag ccgactgatg actggagagg atggctacta tttcaccaat ctggtgagta 2280
agtgagttct tggcgttgtg gagaaggact aggaaggtgg tggttttggg gatgtgatag 2340
gtcactcagg cccatgacag gtgaatgctt ctgtgtgaga aggcagcacg gctgaggaag 2400
ctcactttgc atcagggagc acaaggacca ggccgtacag acactccgcc tcccagcact 2460
tgatcagaga ttgtgtttat cctacagaaa cagatgacat gtgttgggca tcactcccca 2520
cggtcctggg tagaagagtc cttcacttgg cagggctttt tcaaccaatg aataaggcaa 2580
attatatata agttaataat gccatttcga accgagacag atggcagcta aatgaagttt 2640
aattaaagaa tgagtgctgg ggcccttttt attgggtact gcatctactt cgaccacaaa 2700
agacgaagtg accccaactt caagaacagg ctttgagatg gaagaaagaa acagaagctt 2760

gccaaggaga gagctgggct ttccaagtta cctgacctta aagatgctga agctgttcag 2820
aagttcttcc ttgaagaaat acagcttggt gaagagttac tagctcaagg tgaatatgag 2880
aagggcgtag accatctgac aaatggaatt gctgtgtgtg gacagccaca gcagttactg 2940
caggccttac agcaaactct tccaccacta gtgttccaga tgcttttgac taagctccca 3000
acaattagag aattctaagt gctcagagct tggctgaaga tgatgtggaa tgagaaacaa 3060
atgttaacat aataaaatct cagttaaaaa tatttaaaaa attcttggta gttgagcagc 3120
tctgggggaa taagggcaaa tatccttgtt atgaactaca ctgaaatcta ccaaagttaa 3180
tgtttacttt gtgtaggtcc atttgtctat tttatttatt tttcccagtg aaaagtgtat 3240
tttgatagag aacttttcat tttataaata cactatgagt tactgaaaat tc 3292

<210> 160
<211> 3941
<212> DNA
<213> Homo sapiens

<400> 160
gtccactgca gcagaatctc tgatgccact taattagctg tgccactgaa ctataggttc 60
ctcatttgtg aaaacaaaac aatggaccag tgagctctga ggtaccctca agaccaaaat 120
atgtggctct atgatgatgg tttccttttt ttccttcttt ttgctactga gaacaaagta 180
ttcaaactaa ggaagattat gagtagtcct acttagttgg atactgagtt agtagaattt 240
gatatccaaa ttcttctaat attagaattc ttttgagtta cattaataaa ctctattgag 300
tactaataga ataaacctct ttattagaaa ttttggataa tttgcaaagg aaatagaaac 360
tatggttctg ttcataacct ctaaaaatta aactaggaag gaatgaacaa acagaaaaat 420
ctgtacctgt gaatatgagg aatatgagat ttcacttttt cttacaagca agtcaaagtg 480
attttttttt tagctttgtt aattctttca gcaaatgttt actgaatatc tgccatgtgc 540
caggagtagt gccaggcaca ggactcccgc tgtccttgaa gagttttgtg ggatgtgctg 600
acctacaggt acatacagtg agctatggaa tttttaaggc aaacaatttg atttaaaaga 660
aatgtttagt tcaataaagg taacatacgc agaaattagc ttaagacaca agcttggtct 720
ttagcctttg aagaataaac tgggtcagta gtggtggaat atgaaatcaa gggaaatact 780
atgtgctttc tatgtagatg aatatatgca ccagcatttg gatgtttaat ggtattcctc 840
ttacagccgt tggacatacc agatggtcga agagctccac ttcctgctca ttaccggagc 900
agtagtactc gcagcattga cactcagact ccttctgtcc aggagcgcag cagtagctgc 960
agcagtcatt caccctgtgt ctcccctttt tgtcccccgg aatcccagga tggtagccct 1020
tgctcaacag aagatttgct ctatgatcgt gataaaggtc tcgtcagcct atctcggccc 1080
ctctcttttc atgtcctgac aaaaacaagg ttaatttcat cccaaccgga tcagctttct 1140

gtcctgtaaa acttctaggc cccctcttac ctgcttctga ccttatgctc aagaactccc 1200
ctaactctgg ccagagctca gctttggcaa ctctgaccgt tgagcagctc tcatcccggg 1260
tttcctttac gtctctttct gatgacacca gcacagcggg ctccatggag gcctctgtcc 1320
agcagccatc ccagcagcag cagctcctgc aggaactgca gggtgaggac cacatctctg 1380
ctcagaacta tgtgatcatc taaaaaaggg ggagctggcc tccaccctat gttccatgga 1440
ttcggaacaa gatttcagac atctgcatga gtgacaaact ttctgaacac caccaccacc 1500
aataatactt atcagcatca taaagtatct cttaaacact gatcttggca gggacggaac 1560
tcctattcag cagtttttgt ggaaagcagt aatgcttgca aaacgtgtgt gtcattcagc 1620
attttaagtg gagactatgc atttcatagt atatttgaca gattagtact gtgtcctgtg 1680
ttttgttcca gattcttcag tataaataag ctctatatca aaaagttgcc tgtctaaata 1740
gaaaatgtct tgctgtgttt tgtcctatgg aaaatactgt aattcaggat tatgtttaca 1800
attgatccag gtgtttgttt ctaacttctg taatacatac aatgcaaaaa aaaaaaaaaa 1860
aatggccaca acagttgcac agtgcccacc ctatggccta gcttcaggta cttcagttga 1920
agtctaaact caggtaactt ggaatgtata tcatattggg atattaaata tttcacagct 1980
aaaaagttaa agagggaaca tcactctttt gcctttcctt attttatgca tttccctttc 2040
ctcattacat tccacattct tagaataaga agtgcattca atcctaggag aatgataatc 2100
ctggacatgg gtgaacatga ggagaaccag caaaatctgt ggtgtttgac atcactttgt 2160
catgtggtta caagtaaaac aactgttgca ttcactgttt caacatgtgt acatgtggct 2220
tttttaaaag ttcaggtgtt gctcagtaaa ggactgtgac aatgttgcaa ataaagtgtt 2280
cagtactgga ctgtacataa acattccaca ttgtgtgtga tgaaatttaa agacaagaat 2340
gtctagagtt aatttcaaaa taagtgaagt gtttgacgga atggttgaga tttttttgtt 2400
tatgttagcc atcagggtca taactgttac cattttatct aaagacatat ttatatttag 2460
tttctccctt ggaaattctt tattttgcag gtgaaaaagt gacatacttt ttgttattgt 2520
cttcctcaag cagtttaggt gcatgatctt catttacata gaatacttgg gtctcagaat 2580
tgatgcaaca taagcaggtt tttttggtga cttacaagag caatagtttg aagctatctc 2640
atttaagcct ctcataatgc ataatcatga gtagttttga aatttgcaac ctgtgaggta 2700
gagcataaac tcaagaaaat agccttgaac ttgcagactt ttgacacaag ttctccacaa 2760
agtgtgaaga gagccccagg cattcctgat tggtcaatgg gagagcctaa ctttcattgt 2820
tttcttcagt acaaagagta tccaaaagct aagtttttgt attccactac tttcagttca 2880
ataaaaccta gagttgtttc atctgcgcct aaagtgtatg gcacaatttt cttaagaatt 2940
aggggaacca ggtgcctaca gttaaaggaa cgtttcagtt cctttcattc attcctgggt 3000
ttttctttta ttttctaaga aggttgaaga aggatgagtg atagagaaga aagcaacacc 3060
attgattttt ttttttttta agaaatgata tatatatgta tatgtttgtg tgtgtgtgtg 3120
tgtgtgtgtg tgtgtattct gtgcattatt ttgtcatgat ctcaattctc ttctttccac 3180
caaagtttgt cgtaatattt tctcctgaag gtgcattctg gctcctttaa attagtcagt 3240
gttatattgt aggagactgt catggaaaaa aggactcagt ttactttcgt cattttcaca 3300

ggggaacctt ttaaaacaat cttttcagca gcagatacct ttaaccctaa taatctcagg 3360
ccttgatgaa aatactatat tttgtagatt atggttaaag ggggaaaatt actagttccg 3420
taagataaat atgagctcca tttgacttct gatgtctggt ttagcattac ataatatgtt 3480
gatcttacac tctgcttttg tccaaataaa atgcaatagt atcaatatca atttcagaaa 3540
aatggactga atatgctttt ttggtgatga aatctcatgt acgatattta tagtgatgtg 3600
cttttatttt ctcatgagat actaaatatt aattgtgttg tacatttgtt cttagcatat 3660
attaaagttt tgaaccaaat gtgttaaagc ttacgctttg ccatgtaaat ttcccagaag 3720
ttgttgagct caaatgtatc ctacatccag ctgtagaaat ttgtcagaaa ttgtttaaat 3780
tttgtatata attgtactgt ttaattctag ccattgcgct gaacagtatt tgagttacca 3840
tataatatgg ctctacacaa ggaaatgtgt ggcttttgtt ttgtattttt tcagtataga 3900
agttcctgtg tcttatttaa ataaagttat tagtaaaact g 3941

<210> 161
<211> 3196
<212> DNA
<213> Homo sapiens

<400> 161
tgatgcggct gacaggccga accacagcac caccagcacc aactcccaca ccggcgcaaa 60
gcccggctca aagccccact cccctccagt gctcaaggtc acaggcgagg ggagacggac 120
cgaccgagag gagccgccac agccccctcc cctgctgccg cagtgcttcc cgcctgcgcc 180
tacctggagg cggggccggc tctgacgtca cccagagcca atgggagtgc tcggccctga 240
gggttggggg cctcagagtg caccgcgctg tggcccttgt ggggctgccc ttgcgcagcg 300
ctccaaggga cggggatgtt tggcttcgat caggctgaac tgagtcctga gctctccgca 360
gagggtgaga gaaggcgtta gggaggttcg cagcagggtt cagcgaaggt cactggactt 420
cgtaggacag gtagcccggt gacgcccagg cccagcccca gcccttccca tcctgggaga 480
tgagccctag tagagcctga tcacgtcacc tcagggggat ggggacaggg gcggccacac 540
cagggctggg agaagacagt ggggcctcct cagcagcaga gagcagacac ccctcacacc 600
cctcaggcgg acccgcatgc tgcgggtctg ggtttggaag gcaccgccct gggctctcga 660
cgtctgaccc caggaggata actgtccttt atgcagcagg aggcgcattg cagttctttt 720
gcagccccac cctccagagc tgagagaact cacaggtggc tgtgaaaggg ctgcctggtc 780
aggagctccc tcgggtggaa cctgatctcc tgtgaacatg cagccttcag tgcaccgccc 840
tggcccctga gactggcacc tggcagggcc tgccctccct actctgggat tgaccttggg 900
caagtcattt ccctttccta gcctcaattt cctcctctat ggcttgggcc tatgtgggaa 960
gtaaatgaga aaactgtggg tcagtggtca gtggcgggga agtgttcctg gatttgatgg 1020

gccccgagaa aggataggcc tgggatggga ggggctgtgt ggtctgtccc ctctcagccc 1080
tggtgtggcc tcctcatccc ctgggtggtg caatcagccg catggctcac cctaaggctc 1140
attacctggg ctctcctgga ggacaacagg gaaataaaaa gggcctgcaa gtcaaagagt 1200
gttctccagg gtggagcctt tggccaccag gttggtgcca gcttgtcact ccacactgga 1260
gacacaggcc tggacagtgg tcctctgaga ccagacccac taatgctggc ccctgagcag 1320
ctgtcttctg ctgtctaaat ctcctttccc atttggaggc aggaactcag gttctttcta 1380
aaccaaagtg tcctcacctg caaaacaggg actagggttc ctgtctcttg ggtaacatgc 1440
aggaagcctg gcacacagta ggtgctcagt cacttgatgt ttctttcatg tccttgaccc 1500
cagccccacc tccaactcac tgggccactc acgtggttcc cagggctcac aatgatgcag 1560
agaggatcgg tttatttagc tcagatggcc cctcttgaag tttgcccatc cgagtggcta 1620
agaatgccca gtttgcaaag gaacagcgag aagggcattc tcccaaggct caggatgccc 1680
ctagcataac agcccccttt ctggggcttt ctccaagtgc taaatgctcc acaaacattg 1740
gcttgtctag ttcccgcaaa gtccctgtga ggtggttctg gatggagagg ctgaggcaca 1800
gcagagaagt aacatgacat gctcagtcac aagtaagggg tagaaccagg tcttcctgag 1860
gaagtaatga ccttgagcag caaaaccaaa ggtgtgaact caagaggatc ccagagcagg 1920
ctctgcagaa ggcctctaga ggggatgggg aggggggtct gacccgggcc tgcctgaagg 1980
gagcccttga ggatgctggg ctcacacaga agggcgttca aggtggaagg aaccacatgc 2040
gcaagagcct tagagtggga gccaggggca gtgccggggt cggggctagg cttggctggc 2100
acggggtgtt ttctgaggat tccaggcagg accactgagg ccgttcccct gcagcagcag 2160
gaagtgcctg agagttctga gtgtgagagg gacttgggct ggggaggcag agctgtccag 2220
gaggggctgc tgcaagatgg agaaaaacag cctccctgga ccctcaaggg cggaggacag 2280
tgacagcaca aaccagtggc tggtttgcag agaggaccag accttcccca tgcagatcag 2340
ttcacattgt ctctgtactg aacagccctg agaaggggtg ttccatccat tcagtcctct 2400
ctgggggctt cttggggtga tgagaacagg atgccccaag taggggctct accacaggcc 2460
taacaggtac caggctgcgt cacctcattc cccagaggag ccaagagcgg aggctgccca 2520
gagtgtccag cacggtctct ccccttcagg aacatgacat cgagacaccc tacggccttc 2580
tgcatgtagt gatccggggc tcccccaagg ggaaccgccc agccatcctc acctaccatg 2640
atgtgggcct caaccgtaag tgcagcccag cctcagtcag ccctcctctg cctcccatca 2700
gccagagcgg tgaggccccc caccctcccc acagggccct gtcagcccca ctcacactca 2760
cctctgttgc cttcgccctc cgggcctccc atgtgtggtg ttcttctgga ggttgtctct 2820
ttggtcaagg tgaactttta atgtttatta ttttcttctc cgcacaaagt aaagagccta 2880
attttgtgta ttctggtggc tgctgtcatg agatgataaa atgtaaaaca aaactctagt 2940
caacgtagaa agagttaact gtgctgaaaa actaataaag aacctaagaa gaattccagt 3000
gtggtgatgc catgcccatc atgggaggct tttggagaaa cagaatgttt gggcaggggc 3060
tgctggtgct gcttgggttt tgggttgagg gtgctaggag aggatggtct ccacccatct 3120
ttctatttcc agtacacgtc acattatttt accggtgaga tgagaatgtc acaaacatta 3180

aaagccttat gtgctc                                                    3196

<210> 162
<211> 4747
<212> DNA
<213> Homo sapiens

<400> 162
   gtgtcttcca gttcctcctc ctcatcatct tcgtcttctc cttctttatc atccatattg      60
   ggaaccaagg agcactgtga tgaatttggc caagtatggt ctttgctgac ctctcctaac     120
   tcatctgcac ctgcgtcaga atggccagta aaccaggtga ggaacctgtc tggttcctca     180
   ggatgcctct tcctactggc tttattctgc atttgacttg aagagtttcc tttccagatt     240
   tccatttgat ttccgtggac tttgaagatg gatcatgact ctcattcaga tgaaattctt     300
   tgagagagct ttatttttga agtaaggatt ttcgtcaaaa taaaaaccta ttctgtaacc     360
   tgatttaata tcttcaaatt ctgtcacttc agcactggtc aaacaatgca gtgcctcttc     420
   atcctcctcc caagcagtgc agacgcttgt ggatggctga caaatgttat tacccagaaa     480
   ttggggattt tgggaatcaa ttctcatctg ccctgaagaa cgtggttggc cgaatttgtc     540
   tatgttcgct ctcccagtcc gcttgccgct cacttgctaa gcctgtttct gaagcctgtt     600
   ttgtgctgtg cttcctcgtg ggcttggttg ccgcttgttc tgttcctctc ttcctctggc     660
   ctctgtcagg ggctggcgcc atgtcttcct ttcaggcagg ggcagagacg ggtgtttggg     720
   ggtcattccg ggaggagagt ctggacccaa gggcctcctt gctcatcagg aagaagctca     780
   taggactaag atcacatttt tttttttttg acatggagtc tcgctctgtc acccaggctg     840
   gagtgcagtg gcatgacctt ggctcatggc aacctctgcc cccatgccct ccaactccat     900
   ggttcaagct gttctcctgc ctcagcctcc tgagtagctg ggactacagg cacccaccac     960
   catgcctggc taatttttgt atttttagta gagacggagt ttcgtcaagt tggccaagct    1020
   ggtcctgaac tcctgacctc aggtgatcca cccgcctcgg cctcccaaag tgctgggatt    1080
   acaggtgtga gccactgcgc ccggccccaa attttttaat ctattccccc agtctctact    1140
   tttcatgtgg gacaggtaat gcgtttcatc taatgtaatt actgaaaggt agggtttata    1200
   cttgtcgatt ttgtatttgc tttctgcgtc ttctgtcttt tttgttactt tctgcctcag    1260
   ttagtttttg ttcttcatgg agatggagtt tcactatgtt gcccaggctg gagcgcagtg    1320
   agtattcata ggcatgatca tagtgcaccg tggccttgaa ctcccgggct cacaggatcc    1380
   tcccacctca gcctcctgag tagctgggac tgcaggcgta ccacaacgcc tgactcattg    1440
   ctttcttctt ttgtgttaga tattttctag tgtataccat tttaattccc ttgctgttta    1500
   tttgactgta tttttatttt gttttcttgg ttgtcctggg gattacaatt tagtatatta    1560
   atttttgata atgtagtttg tattaatact atattacaat actatatgaa atatttattt    1620
   gggcatagct taatttcttt tgcccttctt gtactgttat tgtcatgcaa attacattac 1680
   actgtgtgcc catctacgca gatttatagt tgctgcttta cgcaattgtc tttcaaatca 1740
   tataggagaa aaaacaaatt ataaattaac aaatacattt atactgtctt atgtagctgc 1800
   ttttatactt tacctgtgta gctgctttta ctggcgctct tgattgcatt cccctcccct 1860
   cccctcttga cagggtctcg ctctgtcacc caggtgggag tacagtggca caatcatagc 1920
   actgcagcct tgacctcctg ggctaaagag atcctcctgc ctcagccccc caggagtcct 1980
   gggatgaagg ttattcgact ctctcaattc ctcctgaaat gttggcctcg taccagtctt 2040
   acagcagcac atttcactca ttagaggaac agcaagtctg catggctgtt gacataggca 2100
   gacatcggtg ggatcaagtg aaaaaggagg accaagaggc aacaggtccc aggctcagca 2160
   gggagctgct ggatgagaaa gagcctgaag tcttgcagga ctcactggat agatgttatt 2220
   caactccttc aggttgtctt gaactgactg actcatgcca gccctacaga agtgcctttt 2280
   acgtattgga gcaacagcgt gttggcttgg ctattgacat ggatgaaatt gaaaagtacc 2340
   aagaagtgga agaagaccaa gacccatcat gccccaggct cagcagggag ctgctggatg 2400
   agaaagagcc tgaagtcttg caggactcac tggatagatg ttattcaact ccttcagatt 2460
   atcttgaact gcctgactta ggccagccct acagcagtgc tgtttactca ttggaggaac 2520
   agtaccttgg cttggctctt gacgtggaca gaattaaaaa ggaccaagaa gaggaagaag 2580
   accaaggccc accatgcccc aggctcagca gggagctgct ggaggtagta gagcctgaag 2640
   tcttgcagga ctcactagat agatgttatt caactccttc cagttgtctt gaacagcctg 2700
   actcctgcca gccctatgga agttcctttt atgcattgga ggaaaaacat gttggctttt 2760
   ctcttgacgt gggagaaatt gaaaagaagg ggaaggggaa gaaaagaagg ggaagaagat 2820
   caaagaagaa aagaagaagg ggaagaaaag aaggggaaga tgacaaccca ccatgcccca 2880
   ggctctacgg cgtgctgctg gaagtggaag agcctgaagt cttacaggac tcactggata 2940
   gatgttattc gactccgtca atgtactttg aacaacctga ctcattccag cactacagaa 3000
   gtgtgtttta ctcatttgag gaagagcata tcagcttcgc cctttacgtg gacaataggt 3060
   tttttacttt gacggtgaca agtctccacc tggtgttcca gatgggagtc atattcccac 3120
   aataagcagc tcttactaag ccgagagatg tcattcctgc aggcaggacc tataggcacg 3180
   agaagatttg aatgaaagta cagttccatt tggaagccca gacataggat gggtcagtga 3240
   gcatggctct attcctattc tcaaaccatg ccattggcaa cctgtgctca atctgaagac 3300
   aatggaccca tgttaggtgt gacacgttca cataactgtg cagaacatgc cgggagtgat 3360
   cagtcagaca ttttaatttg aaccacgtat ctctgggtag ctacaaaatt cctcagggat 3420
   ttcattttgc aggcatgtct ctgagcttct atacctgctc aaggtcattg tcatctttgt 3480
   gtttagctca tccaaaggtg ttaccctggt ttcaatgaac ctaacctcat tctttgtgtc 3540
   ttcagtgttg gcttgtttta gctgatccat ctgtaacaca ggagggatcc ttggctgagg 3600
   attgtatttc agaaccacca actgctcttg acaattgtta acccgctagg ctcctttggt 3660
   tagagaagcc acagtccttc agcctccaat tggtgtcagt acttaggaag accacagcta 3720
   gatggacaaa cagcattggg aggccttagc cctgctcctc tcaattccat cctgtagaga 3780
   acaggagtca ggaaccgctg gcaggagaca gcatgtcacc caggactctg ccggtgcaga 3840
   atatgaacaa tgccatgttc ttgcagaaaa cgcttagcct gagtttcata ggaggtaatc 3900
   accagacaac tgcggagtgt ggggcactga gcgggacagc tgacctgtct ccttcacatg 3960
   gtccatgtca ccacaaatca cacaacggaa gggaggggag atattttggg ttcaaaaaaa 4020
   gtaaaaagat aatgtagctg catttcttta gttattttga gccccaaata tttcctcatc 4080
   tttttgttgt tgtcatggat ggtggtgaca tggacttgtt tatagaggac aggtcggctg 4140
   tctggctcgg tgatctacat tctgaagttg tctgaaaatg tcttcatgat taaattcagc 4200
   ctaaacattt tgccgggaac actgcagaga caatgctgtg agtttccaac ctcagcccat 4260
   ctgcgggcag agaaggtcta gtttgtccat caccattatg atatcaggac tggttacttg 4320
   gttaaggagg ggtctaggag atctgtccct tttagagaca ccttacttat aatgaagtac 4380
   ttgggaaagc ggttttcaag agtataaata tcctgtattc taatgatcat cctctaaaca 4440
   ttttatcatt tattaatcct ccctgcctgt gtctattatt atattcatat ctctacactg 4500
   caaattttgg gtctcaattt ttactgtgcc tttgttttta ctagtgtctg ctgttgcaaa 4560
   aagaagaaaa cattctctgc ctgagtttta atttttgtcc aaagttaatc ttaatctata 4620
   caattaaaac cttttgccta tcactctgga cttctggatt gttttttaca ttcagtgtta 4680
   taatatttga ttatgctgat tggttttggt gggtactgat gtgaattaat aaaaacattt 4740
   catttcc 4747

<210> 163
<211> 3581
<212> DNA
<213> Homo sapiens

<400> 163
actttgagag taattgagac tctgtctcaa aaaaaaaaaa gaaaatgggc aagagacatg 60
aagagacctc cagcacattg acatccgatg gccaggaagc acaggctgag acacccagca 120
tcaccgaatg ctggcgcgga ggaggccgga gcgcggagga ggtccgaacg cggaggaggc 180
tggagcgtgt gtctcacgca ttgcccgggg ggcgagccgc gtgcctcatg ccttgccggg 240
gggacttgcc gggggacgag ccgcgtgcct cacgccttgc cggggggacg agccgcgtgt 300
ctcgcgcatt gccctggggg cgagctgtgt ctcatgcatt gccagggttg agccgtgtct 360
ctcaggcatt cccagggagt gacaagtggc agggccgctc tggagaacag gtggtcggtt 420
tcctacgaag ctaaacacac agctactgag ctaccgtggg acatttatcc cagagaaacg 480
gaaactctca ttcacacaca agtccgtaac aggcgttcag agacgtgtct gttgtagcca 540
aacagtggca tcaacccagg tgtcctacag gtgaacggtt caataggtgg tctgcacacc 600
acgagatggc ccttggcagg gagagggagt ggcggatgcc ccgagcccta gtgaagagtg 660

ccagtccaga ggctgcagct gttggtcccc ttctagaaca taactaagat gacagtctgg 720
gcatggtggc tcacacctac catcccagcc ctgtgggagg tgaggccagc gggtctctta 780
agcctaggag tttgagacca gccatagtga gaccatctcc aaaaaaaaaa aaagatgaca 840
gaactgtagt gaagcattca ggtcgtgatt gctgtggtgc tgagaggaat ccgtggtgcg 900
gtcagtggcg cggccccgca ctcacacaca cgagtccagc tggccacgtc tggtgcatct 960
gtaggtggcg cggctgcccg ggctcagtgc ttctctgagg ttatgagaga cgccctccct 1020
gggggatgct gggcgcaggt gctcagggta tttacagctt cctgctattc tgattattcc 1080
aaaataaaaa gttaaaggat cgggccaggc acggtggctc acgcctgtaa tcccagcact 1140
ttgggaggcc gaggtgggca gatcacgagg tcaggagatg gagaccatcc tggctaacat 1200
ggtgaaatcc cacctctact aaaaaataca aaaaaaatta accgtgcgtg gtggtgggcg 1260
cctgtagtcc cagctacttg ggaggctgag gcaggagaat ggtgagaatc cgggaggtgg 1320
agcttgaagt gagctgagat cacaccactg cactccagcc tgggtgacag agcgagattc 1380
cgtctcaaaa aaaaaagtta cgggatcagc cctgatttga tgtgctgcga gaggcagtgg 1440
ccacgtgaca gatggtggtg gccctctagg acgctacaga cgtggggaca ctgccccaac 1500
ccccgtggac aagccccggg tctgccttgg ctcctggggg tgacgtgggg acactgcccc 1560
aacccccgtg gacaagccct gggtctgcct tggctcctgg gggtggggtg ctgcctccac 1620
agctgccctg tcctggtgtc tgccctccat ccagagtgtt cccgtcgccc cagcacctgg 1680
cctgtgctcc agcccctgga gaccttcaca cgtgtgggtg gagccggcac ctctggacgg 1740
cctcgagtga tcggtgttct attttgcaag cgcatgtttt ccaatatacg tggtgctctg 1800
cgttatagaa acagtagttg cccctcatgg acaaccagat gccgagtttc cgatctccgt 1860
ctccacaggc tcaccagctg gattccctcc tgggggcttc attcccaaaa cggccaccac 1920
gcccaaaggc agcagctcct ggcagacaag tcggccgcca gcccagggcg cctcatggcc 1980
ccctcaggcc aagccgcccc ccaaagcctg cacacagcca aggcctaact atgcctcgaa 2040
cttcagtgtg atcggggcgc gggaggagcg gggggtccgc gcacccagct ttggtgagtc 2100
ccccactctc tgttgctgtg gagtttgcag agacctccct gaattggcta aaggtagaac 2160
ctatgttgtt ttcttcatct cagagcccag tcctgggaca ggcacggcca agccactcag 2220
agaacgacgc ggtttccgat gtgtgttcac tggtctctct ctgggtcagg cctctgggtc 2280
cctcttgttc cagtgacacc ttcttgacct gtttttaaca aatggccttt gcaaccctgt 2340
cttgtcttcc ctcttcttta ctctggaggc atcaggatgg tagccctgtt cccgggggca 2400
gcaggtggaa ttgggcctgt gtgtctctgc agaaagtttc tgttcttaac tgggctttgt 2460
ggctcacgcc tataatccca gcactttggg aggctgaggc ggacagatcg ctggagccca 2520
ggagttcaag accagcttgg gcaacagtga tacctcctct ctacaaaaaa tgtaaaaatt 2580
agccaggcat ggtggcgcat gcctgtggtc ccagctactt gggaggctga ggcaagagga 2640
tcgcttgagg ccgggaggca aaggctgcag agagctgaga tcacgccatt gcactctggc 2700
ctgggtgaca gagtggagac cctgtctcaa acaaaaaaaa gtttctgttc ttttctagct 2760
caaaagccaa aagtctctga gaacgacttt gaagatctgt tgtccaatca aggcttctcc 2820

tccaggtctg acaagaaagg gccaaagacc attgcagaga tgaggaagca ggacctggct 2880
aaagacacgg acccactcaa gctgaagctc ctggactgga ttgagggcaa ggagcggaac 2940
atccgggccc tgctgtccac gctgcacaca gtgctgtggg acggggagag ccgctggacg 3000
cccgtgggca tggccgacct ggtggctccg gagcaagtga agaagcacta tcgccgcgcg 3060
gtgctggccg tgcaccccga caaggctgcg gggcagccgt acgagcagca cgccaagatg 3120
atcttcatgg agctgaatga cgcctggtcg gagtttgaga accagggctc ccggcccctc 3180
ttctgaggcc gcagtggtgg tggctgcgca cacagctcca caggttggga gccgtcgtgg 3240
gacctgggtc cccaccgtga ggaccccgtg ggcgacagca ggtgtggcca gggtggggct 3300
ccgagccccg ggtcaccgcc cgcccagcgt tccaggcaca tgaagagaaa gcattccaaa 3360
gcctctgatt gttgtttcct ttttctcctc ccgaaggaac agctgattca tgctcctccc 3420
gcaattgtca cgtctgtgat ttatttggtg tttcgggcgt ggcctctgga gccccggcac 3480
gtggtgggcc acgctgctgg cgctcatggg ccctggtgtt tgcaccgcac tttgtaatca 3540
gtcccgtggt tgtctgtaca gaattaaact attttccgat g 3581

<210> 164
<211> 3600
<212> DNA
<213> Homo sapiens

<400> 164
ataccatatg aggatgctat gaggaaaagc tttagacttc ttgggagaaa actaagtaaa 60
tctgaggtgc atgtattatt tcatacagtt ttaatcagga gcaagtgagc ttaagaaact 120
ttgttgaata ataaaaatat gaaaaaatgt gttcatttag aacaaaagtt gttagcatct 180
actgtgggag gctgcagaag caaacaaaac ttgtcccctc atcaaggagt ttaccatctg 240
ctaaggtgga tggaatgtac ctggggatgt caaactcaga tgtccccagg ggccagcagg 300
tgatgtcaag aggtgagcaa gagagaatac taacagttcc aaatgttaat aagagcccag 360
tttgccagat cttccttttt attctccaag aaagactgga aatctggatt tttgcaagaa 420
atatcctacc acctgtaaat gttggcaatg atttgattta aaaaaaaaaa aatactttgc 480
atgcccaaca aaatttgagc atgggctata tacaccccta aggccatcag ccagcacttg 540
ggaccttctc tgcatacatt atttacacag agtgaagtgt gctgcaggct gtgacatctg 600
aattcaaagg agggcaacat ggctttaggt gtgcttaaac tttaaaatca gatgggccct 660
ggttaaaact ctgtcttcta tttagagcca cactgacctt gagcaaatct caacttccaa 720
agccttggct tctgcattta tataatggga ataataatgt ctcctgagta tattgattat 780
gaagcttaaa tgaggtaatg ttgatgaaat gtttaacaat gcttgctgca cagtgatcac 840
aataaatgct gttgctgtta atactaataa cttcctttcc acttaaagga taccttagga 900

aagccagctg ctaaggatgt ccatcttgtg gatcactgca aatacaagta agatttgcag 960
gactcctcac tttcttggtt tccatactgc ttttgaaata gcttcacttt gggtgagaac 1020
tttaatataa aggcccagct ggagtaaatg aaagttgcat taattcattt cactggcttt 1080
tcctcagggc ctaaatgcac tgacctgagt tctgtggcga ctgtagagaa gaatggaaca 1140
tggttcctgc ttccaaacag ctgcatgcta gctagtaatc acagtaatgg aattacctct 1200
ccataagccc cacccaccca atcattgaag ttagaataaa tatttctcag ttaataaata 1260
ttagaattat tatcctggtg tgctggcatg ctcctgtagt cttagctact cgggaggctg 1320
aggtaggagg atcctttgag cccaagagtt caagaccagc ctgggctaca tagtgagacc 1380
ctgtctctaa gagaaataaa ataaaattct gggactaaga attagatgaa caggtaaaac 1440
caggtctaga ggagggcagt ggtggtgggg gtggctcaga gttaggagtg ctaatgacac 1500
gcagagtagg agctatatgg tgaaagcaac tctggttggc gacaggtgcg tgcataagaa 1560
atgctgagaa gaagacagct tttttgtttt tttttttttc ccctgtgggc aatctctgaa 1620
aattgattag gttttcctca ttctcctctc tacaccaggg gtccccaacc ccctgccatg 1680
gtactggtct attaggaacc aggctgcatg gcaggaggtg agcagtgggg aagcgagcat 1740
taccgcctga gctctgcctt ctgtcagatc agccttggca ttagattctc ataggagcac 1800
aaactgtatt gtgaactgtg tatgcaaggg atcaaggttg cgtgctcctt atgagaatct 1860
aactaatgcc tgatgatctg aggtggaaca gtttcatcca aaaccatctc ccccacacta 1920
cccatccatg gaaaaattgt ctcccatgaa actggtccct ggtgccaaag aggttgggga 1980
ccgctgctct atactttaag agaagagggt gatgtccagg aagcagtgga gaccttcgac 2040
cagatggggt acactagagg aaaaacaagg ctttgggagg ccatgactag ttagagacat 2100
taggcctaga aaagcttgtg aaattttttt cactcgttat attttatgtt taccattgaa 2160
catttatttt ctcagatatg ttccaaatct acaagctgtc atttccttct gagccctgta 2220
tacactctac ccggcaccca gctcctgacc ctatctagtg ctgtctggag aaaatatccc 2280
ctatttttcc tcaccctgcc attcccagaa caacctgtag tcctagccct ggtaaagtgg 2340
ctgatagaaa ccacttagga acatattaat gtgtccttga aaactcaaag tggaaagaga 2400
atggagagga atcataaaag gaaagaaggg gaggggagaa taagcataaa aggtcatatt 2460
tgtggcagca ggcatatagt atcaaatgta ttttgttctt ttttccccag gtatctgttt 2520
aattttcgag gcgtagctgc aagtttccgg tttaaacacc tcttcctgtg tggctcactt 2580
gttttccatg ttggtgatga gtggctagaa ttcttctatc cacagctgaa gccatgggtt 2640
cactatatcc cagtcaaaac agatctctcc aatgtccaag agctgttaca atttgtaaaa 2700
gcaaatgatg atgtagctca agagattgct gaaaggggaa gccagtttat taggaaccat 2760
ttgcagatgg atgacatcac ctgttactgg gagaacctct tgagtgaata ctctaaattc 2820
ctgtcttata atgtaacgag aaggaaaggt tatgatcaaa ttattcccaa aatgttgaaa 2880
actgaactat agtagtcatc ataggaccat agtcctcttt gtggcaacag atctcagata 2940
tcctacggtg agaagcttac cataagcttg gcacctatac cttgaatatc tgctatcaag 3000
ccaaatacct ggttttcctt atcatgctgc acccagagca actcttgaga aagatttaaa 3060

atgtgtctaa tacactgata tgaagcagtt caactttttg gatgaataag gaccagaaat 3120
cgtgagatgt ggattttgaa cccaactcta cctttcattt tcttaagacc aatcacagct 3180
tgtgcctcag atcatccacc tgtgtgagtc catcactgtg aaattgactg tgtccatgtg 3240
atgatgccct ttgtcccatt atttggagca gaaaattcgt catttggaag tagtacaact 3300
cattgctgga attgtgaaat tattcaaggc gtgatctctg tcactttatt ttaatgtagg 3360
aaaccctatg gggtttatga aaaatacttg gggatcattc tctgaatggt ctaaggaagc 3420
ggtagccatg ccatgcaatg atgtaggagt tctcttttgt aaaaccataa actctgttac 3480
tcaggaggtt tctataatgc cacatagaaa gaggccaatt gcatgagtaa ttattgcaat 3540
tggatttcag gttccctttt tgtgccttca tgccctactt cttaatgcct ctctaaagcc 3600

<210> 165
<211> 3266
<212> DNA
<213> Homo sapiens

<400> 165
agtagagatg gggtttctcc atgttggtcc ggctggtctc gaactcctga cctcaggtga 60
tctacacacc tcagcctccc aaagtgctgg gattacaggg gtgagcaacc ctgactggcc 120
aggacagtgc ttattaattc ctgagatgca tccaggagca catgacctgg ctgtgactgt 180
tctaacagag ttccccaaat gggtggctca ggacaacaga aagtcattct ctccagttcc 240
agaagcttga tgtctgaaac ccgcagggcc atgctccctc tgaaggctct aggggtgaat 300
ccttccttgc ctcttctggc ttctggtggt tgctggcatt ctttggcttg tgtccacatc 360
attccattct cttccttcat tctcatgtgg ccttctcccc tgtgtgtctc tgtctcttct 420
tctcttccca tgaggatgcc attattactc gatttaaggt tcacgctatt ccaatatgac 480
ctctttgtaa tgagatctgc agtgacccta ttttcttttc ttttttttgt gatggagtct 540
tgctctgttg cccaggctgg agttcagtga cacaatctcg gctcgcttca actctgcctc 600
ctgggttcaa gtgattcttc agcctcggcc tctgaagtgg ctgggattac gggtgcacgc 660
caccatgcct agctaatttt tgtgtttttg gtagagacag ggtttgccat gctggccagg 720
ctggtctcga actcctgacc tcaagtgatc ctcctgcctc agcctcccaa agtgctaaga 780
ttacaagcat gagccaccat gccctgcccc tattttctaa taaggtcaca ttctgggatt 840
cctggtgaat gtgaattttt ggaagacagt attcagtcta gcaaaaggca gaacatcctc 900
atttcttccc ctacctcaga aataaagaag ttaacttcaa cccctctgag agagagaggc 960
ttcctgagct tccaacaatc aattatccaa atattagtca cagaaggcca ctaaggttg 1020
tgcacagcac gtggccagcc cattctctga gtctgtcaag tttaaggtga acgctaatcc 1080
tgaatgagtc ttaaaatgta cttggcatat cctgttcatt gtaaaatgtt ctcacattgt 1140

gatggctggg gcttccctct caggtgtaat ctgcgaagtc agacgtgaca cagcctgggt 1200
gaggtgggcc aagccgggaa ctgggttagg agggaagctg gggaatgatc tccaaggtct 1260
cagatcccaa actggcttta gccagattca cccagaggga tctcataaaa aatgcacatt 1320
ccggggccca ccccagacct aatgaatcag aattacctgg gaaggagcct ggggagctct 1380
gttttcagaa gcagcccagc cgaatcctac ggtcagacag ggctaggaat cgaaactcag 1440
tctagcgcgg tagttcccaa actcgtctgt gcttcaaaaa atacagatgc tgatgtccag 1500
acatggtggc tcatgcctgt aatcccagca gtttgggacg gtgaggcggg agtatcactt 1560
gagcccagga gtttgagact agcctggaga acataaggag atactgtctc tataaaaaat 1620
ttaaaaattg gccgggcgtg gtggtgcccg cctgtgatcc cagctaccgt ggaggttgaa 1680
gtgggagggt ttcttgagcc caggagttgg agcctgtggt gagctatgat tgtgccactg 1740
cactccagcc tgggtaacag agcgaggccc tgtctcaaaa acaaaacaaa acaaaaaacg 1800
gagtctatgt cccattccag aggttgaggt ttaattgttc ttgggtgtgg cctgggtttt 1860
ggaagattta aaaaaaaaat ctcaggtgac cctaaagtgt agatgagttt ggaaaccaca 1920
catttaaggc acacttgaat gggggagcag tgaggtggcg cgggctagcc ggccagaacc 1980
caggggtggg ccagtaggaa ccagcattgc agaggccatt aaggctggga agcatagtgt 2040
ctggggccca taacaatgct tcggcatgaa tgctttagac ctaagacaat tggctcctaa 2100
atgtgaaaac tgcaaggctg aaatgaatgc atgtttaatg ccttacaaca ttgtcaagtg 2160
atcagctgca actcctttct gagggcatga tgcctgagat atgcctgtaa tgcgggttga 2220
ttttaattaa tttaatatgg tgtggagtgg ggccttcaaa agtaaagacg tcagttctaa 2280
gttggttgca gggttctggg caaaggtctt aaaaccccat ggtgagcaga tggccaatcc 2340
tgaacacccc aattttaaaa cagggctttt ttttccaaga gactttttga aaatagctcc 2400
tattttgaga ggaggaaccc tggcaggaga gagccagagt taagcccagc tgagaggggg 2460
ttggtaggca ggggcctgcc tgatcctcac tgaagcttga tactcagggt gagcttccta 2520
aaccagtgca gatttgccgg cccactgagc ctcccagatg agaacctgca tttcaacaag 2580
gtccttgatg cagcaaagtt tgagatatac tgggctagaa cactcagggg acacaaaggt 2640
tctctgaaaa ctaaggaaaa taggcagggt gtggtggctc atgcctgtaa tccttgtatt 2700
ttgggatgcc aaggcgggcg gatcacctga ggtcaggagt ttgagaccag cctggatcaa 2760
catggtgaaa caccatctct acaaaaaata caaaaattag ctgggtgcgg ccgggcacag 2820
tggctcatgc ctgtaatcct agcactttgg gagtccgacg cgggcagatc acgaggtcag 2880
gagatcgaga ccatcctggc taacacgatg aaaccctgtc tttactaaaa acacaaaaaa 2940
attagccggg cgtggtggca ggttcctgta gtcccaacta ctcgggaggc tgagggagga 3000
gaatggcatg aacctcgagg aggagcttgc agtgagccga gattgcacca ctgcactcca 3060
gcctgggcaa cagagccaga ctctgtctca aaaaaaaaaa aaaaaaaaaa aaaaaaaaat 3120
tatctgggtg aagtggcagg tacctgtggt cccggctact tggggggctg aaacaggaga 3180
atagattgaa cctgggaggc ggaagttgca gtggcccgag atcgcaccac tgcactccag 3240
tctggtggca gagtgagact ccatct 3266

<210> 166
<211> 4001
<212> DNA
<213> Homo sapiens

<400> 166
atatattgca aatctccatg ggagggtata ccgtatgata catatcaatt ctcaagttta 60
tttgtctaaa gactcttgtg tatttcacaa aacatctata atttctcatg gacattagca 120
ttctgcaaaa acattactaa gtcaaaccca atagcttgag atacaaggcc ttctatgaga 180
taaactgctt aattctccca tctcacttac cttctatcaa acacgcatcc cattttccca 240
tgctacaaaa ctggttacag ttttctaaca tatcatcaat tttttcatag ttatatgttg 300
ctaataatgc ttccccttct gcctgaaatt cgacttcctt tcatctgcct tataaaatta 360
tattcattct tcaaggcact gctcaaatgt ttctccctct ttggaccttt ctgtgtccta 420
tcccaaaaaa attatgaact cttttctgtg gttctcatcc ctctcctctc tttctctctc 480
tgtctctctc tttctctctc tctttctctt gatctgccta tatacacaca tacacaatac 540
atatacttta atatatatgc agttttatat ataatctata taaaatatgg tatttaatat 600
tatattaaga tgagtgagat agaaataaac acataaagag acaaacataa atgaaaacat 660
gttatatatg gatgttcatc ataatatttt tcacttatgt gtttacttat gctttcttca 720
ctgaacaatg gattccttaa aggcagtgac tatgtctaat ttctctctta acctctctat 780
tcacataatc taatgaaatg catagcattc aatgtctgct aagggcatgt tcaaatgaat 840
tcactcatct cgcttatagc tgaagtccag gccatcccac cactcaagat gaacttgtgt 900
gcttttgcac aaatgcagtt ctgcctgctg atctccctct ggtgggagct tcactttgcc 960
aaggacactg gtccagaacc actatgtaag attcaaaata gatatcatgg taatggatat 1020
agtctggctc ttgctctact ctccaagcca tgattctggc atggactgtg ctatcctgga 1080
agaagaagct tctttttgtg ttctcacaaa gcatgcatga atgatgacag ccatgcacca 1140
ggccatccag aaactattgg ttccctgtta ctcctgttag tgtatttggt actgccagtg 1200
tactctgcta ctctcaccta aataaatctc cccaggatct gtgaataaga tgctgatagg 1260
attttccaac aggaacattt ccccaagatg ttcatgctag agcaggtctc agatatgggt 1320
tattattcaa tgacacattt gggcaaggga gaataacaaa acctaatcac aaatgttcat 1380
cactgatctc tatggtaatt gtcaaattta aagatggagg caagggtgaa ctgcctttaa 1440
tgctgaaagc tgcctgtgcc acactaaagt tcttgtagtg ccagaatgga tacatcacca 1500
cagaacctga tatatcacag aattgtagtc caatgcaatc atacaatatg gagacagaga 1560
cctgattacc aggtgacata gcatttgggc tggttggcct aagaaggtag agattgtcct 1620
caataaggtg gagaaatgta aatacactag tatataagat gataggcttc cactacagaa 1680

agtgaactta tttagagaat agctctcctt gctgagttgt gaaaccagct atcttcaaag 1740
ttcctgggtc aacaaccgtg aatgcctagg cactgagtaa gatgatggag gattagccac 1800
tggaccaggg gagtgataac taaaaccact ttcaggattg atcccatcca ctggcctaca 1860
gtaaaaagct tcaggcttga tataagtgaa gtgtagaaaa atatctttaa aacaaataat 1920
ggtttcccat aatatgccat tattttaaaa cttttaatat tttgacatct tttatttatt 1980
atcattattg tttacatgga ataaaaaatt cttattttta tgaaaataga ttcaaactgt 2040
atataattat gtgaattatg tgtcttgact tttaatttaa aatatataat gaacaccttt 2100
ctaggccagt gtgtgtatgc atatgagagt ggggcaggtg tatacattag cacatatata 2160
tatacaatta tacataaaat ttgtttttta tataattaat ccaatttatt ttcacttact 2220
ttttaaacag atttattgag gctcaattaa caaacagtag actgaaccta ttgaatgcat 2280
acatttgtga gttttgacat gtatgcacac tcacacttaa tgtgaaacca taaccacact 2340
acacactaca ataaatgtgt tcattatcct gtttccttat gcccttctta atcttcctct 2400
cctattccag cccctgccat tctcaaacaa ccactggtct gcttgatgtc attataagta 2460
catttttatt tcctagaatt gttgtaagta gaatagtata atacttatgc ttttctgttt 2520
cattttgttc actcatcata aacgctgttt ttttgtttgt ttgtttttgt tttgttttgt 2580
tttgtttttt gagattgaga gttttgctct catcacccag gctggagtgc agtggcatga 2640
tctcagctca ttgcaacctc cacctcccgg gttcaagaaa ctctcctgcc tcagcctcct 2700
gagctgggat tacaggcatg ggacactacg ccactacgcc tggctaattt ttgtattttt 2760
agtagagatg gtgttccacc atgttggcca ggctggtctc aaactcctga cctcaagtga 2820
tctgccagcc tcagccttcc aaagtgctgg gattacaggc gtgagccact gcacccggat 2880
atgaacgctt ttaaatttat ccatgttgtt gcatgtatca ataggttgtt cctttttatt 2940
gttgactgtt attctattgc atgcttgtaa cacagttttt ttacacattc atacatttaa 3000
attatttggc tatcacaaat taagatatta taaacatcca tttgtaagtt ttatatggac 3060
atatgctcta tttacttagg agtactgtgg ctggaccata tggcaaaaat atttaaagtt 3120
cttgaaaaac tataaaattg cttttcaaaa tgactgtatc attttaaatt cccacaagca 3180
gtgtatgagt gcttcagttg ccgacaaagc ctcaccaaca attggtataa ccagcctttt 3240
taaatttagc ctttctaata agtaggagta atatcttatt gtggttttaa tttgcatttc 3300
gctgaagact aatggtgttt agagtatatt catgtacttt atttgctatt catatgtctt 3360
gtttgtttgt ttggtaaaga gtctgttcgt atcttctgct tttgttattg tcttcattgt 3420
tttcttatta ttgggttttg agacttattt atgtgtaatg aatacaagtc ctttatcaga 3480
tacaagattt tcaaatattt tctcatagtc tgtggttgtc atttcattat ctttacagta 3540
gctttagaaa atcagtactt gttgattctg atgaagtcca atttatcaac tctttttctc 3600
ttctattgca cttttggagt tgtatctaaa aaattctgtc taacccaatg tcacaaacat 3660
aatctcctaa gttttcttct aaaagattta acgtttcagg ttttacattt aggtccataa 3720
tcaatttgtg ttagtgtttg tctatttgtg tctatggtgt tagggatggt ttgaatttca 3780
ttatttgcat aaggattcca gtagttctta tgtaactgct gaagactgtc ctttctccac 3840

tgaatggcct ttgcatcttt gtcaaatata aattgactgt atatgtgtgg atctaattcc 3900
agatcctagt tagttccatt gatctgtata acttaataac ttagcgtaaa tactacactg 3960
tcttgattaa tgtagcttga taataaatct tgaactcaga t 4001

<210> 167
<211> 3163
<212> DNA
<213> Homo sapiens

<400> 167
   atcgagtgca ttgcgtgtac agctcagcgg gtctctgcag agtgggtctc atgcgtattc 60
   atgtaacccc catgggtcct gagacagggc atctctcgct ggggtccagg ctgtcccagg 120
   aatggctggc cctcacaggt ttcataaatg cacagctggg atgttaccat gttccccaaa 180
   cctcagctct gtaaacatga gaagaagctt tataacaaca gaggcgttta gcaatcagct 240
   gctattttcc tctatcatgt ggcaccttct acaacaggct aagagaagac caggaaaagg 300
   gagtgcccgc ccaggtattg aggagtctgg agaggactct gctcctgaag gcccagggtc 360
   ccaggaaggc atggtgggac tcctgcccct gctggccaag ctgctttttt ttttggagac 420
   agagtctcgc tctgtcgccc aggctggagt gcagtggcac gatctcggct cactgcaagc 480
   tccgcctcct gggttcacac cattctcccg cctcagcccc ccgagtagct gggactacag 540
   gtgcccacca ccacgcctgg caaatttttt gtatttttag tagagacggg gtttcaccat 600
   gttagtcagg atggtctcga tctcctgacc tttgatccgc ccgcctcagc ctcccaaagt 660
   gctgggatta caggcatgag ccaccgcgcc tggctggcca aagcttcttg cagccctgcc 720
   tgggtcagct gtcacctcct gcagagactg acccaacccc ttctcctgcc agggtctgac 780
   tgcgtaccat gatatctccc tggacaagtg ctatgtcatc gaactcaaca ccaccattgt 840
   gctgccccct cgcaacttct gggagctcct catgaacgtg aaggtgcgca gggggttggg 900
   gggatgtctg cagcatcctg tccctccctt gccccctgtc tcatggaggc taggtctgag 960
   gtacaggaag ttcctgtgtc aggggttggg ggagcaagag acattgctgg cacctgggcc 1020
   ctgtacccag attggtctcg cctctgctat ccccctgctt ccacatctcc agagtcaatg 1080
   ccccgagacc ggcttcctta gtgttcaagg ttgtaccctg ggtacggact cgtgcgcatg 1140
   ccattgctcc tggcagcaca ggtgcaggca ccgggcacag gcaggaagtg cctgaggaca 1200
   tcagaagggc cagcccaggg gcctctggag gagggaggtg ggctggcatt tcgggcgagg 1260
   ggtttgacga aagcctgagg ggccgactca ctgtggcggc caccttgttt tgcagagggg 1320
   gacctacctg ccgcagacgt acatcatcca ggaggagatg gtggtcacgg agcatgtcag 1380
   tgacaaggag gccctggggt ccttcatcta ccacctgtgc aacgggaaag acacctaccg 1440
   gctccggcgc cgggcaacgc ggaggcgtga gtggctggct tcacccacag tagcccctgt 1500
   cccgtgcccc agaccacagt tatcttcacg cctagcccag ctgtcagaga gctcagatag 1560
   cagcagcaat aacagctagc attagcagag cacgtccgtg tgccgggcaa tgctgtgtgc 1620
   tctttatgac ctcttaagag tgtaagtcat tctcatcctc attattattt ttgctgccca 1680
   ttttaaagat gaggaaacag gtgcagagag gttaacaccc tggaggtcac acagcaggga 1740
   ggtggcagac atgggcaggc tgactccagc tttcgagctc tccccatttc tcactgtgct 1800
   ctttgggtga atctgggtga atggtgtcac ttcctggccc tcctgtggct caggctgagg 1860
   ctggtggtct ttgtgactca gccaggatgc agggctcact ggggtttttc tttttcctcc 1920
   cagggatcaa caagcgtggg gccaagaact gcaatgccat ccgccacttc gagaacacct 1980
   tcgtggtgga gacgctcatc tgcggggtgg tgtgaggccc tcctccccca gaaccccctg 2040
   ccgtgttcct cttttcttct ttccggctgc tctctggccc tcctccttcc ccctgcttag 2100
   cttgtacttt ggacgcgttt ctatagaggt gacatgtctc tccattcctc tccaaccctg 2160
   cccacctccc tgtaccagag ctgtgatctc tcggtggggg gcccatctct gctgacctgg 2220
   gtgtggcgga gggagaggcg atgctgcaaa gtgttctctg tgtcccactg tcttgaagct 2280
   gggcctgcca aagcctgggc ccacagctgc accggcagcc caaggggaag gaccggttgg 2340
   gggagccggg catgtgaggc cctgggcaag ggcatggggc tgtgggggcg gggcggcatg 2400
   ggcttcagaa gtatctgcac aattagaaaa gtcctcagaa gctttttctt ggagggtaca 2460
   ctttcttcac tgtccctatt cctagacctg gggcttgagc tgaggatggg acgatgtgcc 2520
   cagggaggga cccaccagag cacaagagaa ggtggctacc tgggggtgtc ccagggactc 2580
   tgtcagtgcc ttcagcccac cagcaggagc ttggagtttg gggagtgggg atgagtccgt 2640
   caagcacaac tgttctctga gtggaaccaa agaagcaagg agctaggacc cccagtcctg 2700
   ccccccagga gcacaagcag ggtcccctca gtcaaggcag tgggatgggc ggctgaggaa 2760
   cggggcaggc aaggtcactg ctcagtcacg tccacggggg acgagccgtg ggttctgctg 2820
   agtaggtgga gctcattgct ttctccaagc ttggaactgt tttgaaagat aacacagagg 2880
   gaaagggaga gccacctggt acttgtccac cctgcctcct ctgttctgaa attccatccc 2940
   cctcagctta ggggaatgca cctttttccc tttccttctc acttttgcat gtttttactg 3000
   atcattcgat atgctaaccg ttctcagccc tgagccttgg agaggagggc tgtaacgcct 3060
   tcagtcagtc tctggggatg aaactcttaa atgctttgta tattttctca attagatctc 3120
   ttttcagaag tgtctataga acaataaaaa tcttttactt ctg 3163

<210> 168
<211> 3459
<212> DNA
<213> Homo sapiens

<400> 168

ttttgagccc atgctctggg ccaggcactg ttaggcactg gggatactgt atggggtggg 60
tatattgacc aatattgaca tcatctctgc ctagtgaaac ttatgctgta gtgagagaga 120
ctgacacgaa acatgaatgc aggtaaaaag acaatagaat tacccgttat cataagtatt 180
aaaacggcaa cagaatggag cattcatgta ataaaagagt gagtgaccct gttcggtagg 240
aacatcaggg aaatgtctcc aaggagaggt attgggccac atttaagcta cggcctgaag 300
actgagaagg gccaagctgt gaccagtggg gagaagagaa gagaggagac catggtgagc 360
agaggtggca gtgtgttcag aagcccgggg gaggaaggag agggctctgc acatcctgag 420
aacagcaagg tggccagcgt gcctggggtg gtgagagcaa gggaagggat gtggctggca 480
aagcctgcag aggccagaac aggcagcctt gctctcagga gcctgtgccc tgtgttattt 540
ttgagatggg gaagatctta actagtacca tcctttataa gcatattcaa gataggtgcg 600
agaataaggt acaaggagca ccatgccaga aaagcaatgt gtgtgcctct gtgtgtgcat 660
gtgtagatgt gtacacttgt atgtgcacat gagtggatgt gcacacacat ctctgtatgt 720
gtgcaaacgt gcatttttgt gtacatttct gcacgtgtgt gtatgtatgt acatgcatgt 780
atgtggcatg cgcatacgtg catgtattgc agcaggaaga gaaggagcca cgaacagtat 840
gacagtgggg agattgctga cttcagagcc cagcagaatg agggtcagtg ttgctgggca 900
cgctgcttac agcatgtgca gagcatagct ctgttttgcc tcacatccct cctggactga 960
tccctttctg tttgtatggt acattcagtg agtgacagca tcacaggagg cagaccccag 1020
ccttatgaac atatcttcat aacaccatac agtctcacga gaaaggggag gtcatttgtt 1080
cactgtagtc cacccctgaa ttgttccctc actggctctg tgtcactttc agagattttt 1140
ttaaattctg ggaaatgctt tggggacttc ggcattttgg agtgtgtttt caaggattgt 1200
attagcacca cagagaccct tagcctcttt cttaaaggtt ctgtttttag acatcctcct 1260
cttttatccg tgctttcaat tctgctgctt ccttccttct gagattcctt cttccttgct 1320
ggtcttggaa caagaaacat ttagctgtat tttcctcgtt ctcgcctagg tagggtgtgg 1380
tccccctgtg tcctcatttg ttcaacattt tgagacaaag attggtggaa ttgccactta 1440
aaaaaataaa accagggaaa gcagctcaga gaatttaatt tgcctctaac ccaactcttc 1500
tacttattaa aaaaatatac aattattcat tccgtagcac tccagattgc ttcactgaaa 1560
gaaagaaaga aagggatcca ctccagggag aaagaggctc ttgagctttt tgttccttga 1620
tgatcctgtc tttacgtaat ttggtggagc ctactgggga atcatcattt tgggagacta 1680
tggatgtgcc tcattgcaga agaataagta gagagcagtg tctggaacaa atgcttgtct 1740
taaaaaaagt ttatgaaatg ccagcagatg tcagtgtgcc cttgccattt gatttttttt 1800
ttaaagcaac attaattatt ttcggtagta aaaacaacac aagtatattc tgctacagtt 1860
ataaatgaaa agatcatgag gcaacgtttt ttaggtttgg agaaggacca gcagaattta 1920
tgtcataagg taaaatcctc taaattatct gtgtaatata atttgtcttt tcccagaaaa 1980
gtggtttcaa aatgcaaaac ctagtgtaag tttttttttt ttttttccct ctctcccagc 2040
cattgttcag gtgccaataa tagcttagga tccagcttag cttctgagaa gctactggct 2100
ctgctaattg ataacagctc cttatttcaa agctggagtt tgcaaaggaa ggtagtgtag 2160

## Description

### FIELD OF THE INVENTION

The present invention relates to polynucleotides encoding novel polypeptides, the polypeptides encoded by these polynucleotides, and new uses of these.

### BACKGROUND OF THE INVENTION

The genomic DNAs of various living organisms are currently being sequenced and analyzed all over the world. The entire genomic sequences of more than 40 species of prokaryotes, a lower eukaryote, yeast, a multicellular eukaryote, *C. elegans,* and a higher plant, *arabidopsis,* and such have already been determined. Analysis of the human genome, presumed to have three billion base pairs, was advanced under global cooperative organization, and a draft sequence was disclosed in 2001. In 2003 the complete structure had been elucidated and publically disclosed. A genome is a blueprint for highly complicated living organisms. The aim in determining a genomic sequence is to reveal the function and regulation of all genes, and to understand living organisms as a network of interactions between genes, proteins, cells or individuals. Understanding living organisms through the genomic information of various species is not only academically important, but also socially significant from the viewpoint of industrial application.

However, simply determining a genomic sequence will not reveal the function of all genes. For example, in the case of yeast, the function of only approximately half of the 6000 genes predicted on the basis of genomic sequence have been deduced. The human genome has been estimated to contain about 30,000 to 40,000 genes. Further, 100,000 or more types of mRNAs are said to exist when variants produced by alternative splicing are taken into consideration. Therefore, it is desirable to establish "a high throughput system for analysis of gene functions" which allows rapid and efficient identification of the functions of vast amounts of , genes obtained by genomic sequencing.

Many genes in the eukaryotic genome are split by introns into multiple exons. Thus, it is difficult to correctly predict the structure of an encoded protein based solely on genomic information. In contrast, cDNA, which is produced from mRNA that lacks introns, encodes a protein as a single continuous amino acid sequence and allows easy identification of the protein's primary structure. In human cDNA research to date, more than three million ESTs (Expression Sequence Tags) are publicly available, which presumably covers no less than 80% of all human genes.

EST information is utilized in a variety of ways, for example in analyzing the structure of the human genome, or in predicting exon regions of genomic sequences or their expression profiles. However, most human ESTs have been derived from regions proximal to the cDNA 3'-end, and little information is available from around the mRNA 5'-end. In human cDNAs, the full-length protein sequence of approximately 15,000 corresponding mRNAs have been deduced.

It is possible to identify the mRNA transcription start site on the genomic sequence based on the 5'-end sequence of a full-length cDNA, and to analyze factors involved in the stability of mRNA contained in that sequence, or in the regulation of its expression at the translation stage. Also, since the atg codon, or translation start site, is contained in the 5'-region of a full-length cDNA, translation to proteins will occur in the correct frame. Therefore, it is possible to produce a large amount of the protein encoded by the cDNA, or to analyze the biological activity of the expressed protein by utilizing an appropriate expression system. Thus, analysis of full-length cDNA provides valuable information which complements genome sequencing information. Also, full-length cDNA clones that can be expressed are extremely valuable in empirical analysis of gene function and in industrial application.

Therefore, if a novel human full-length cDNA can be isolated, it can be used for developing medicines for diseases in which its gene is involved. A protein encoded by such a gene can be used as a drug by itself. Thus, obtaining full-length cDNAs encoding novel human proteins is of great significance.

In particular, human secretory proteins or membrane proteins would be useful used as medicines in the same manner as tissue plasminogen activator (TPA), or as target proteins for medicines like membrane receptors. In addition, genes for signal transduction-related proteins (protein kinases, etc.), glycoprotein-related proteins, transcription-related proteins, and such, are genes whose relationships to human diseases have been elucidated. Moreover, genes for disease-related proteins form a gene group rich in genes whose relationships to human diseases have been elucidated.

Isolating novel full-length human cDNA clones, only a few of which have been isolated, is of great significance. The isolation of novel cDNA clones encoding secretory proteins or membrane proteins is especially desired since such proteins would be useful in themselves as medicines, and also their clones would potentially include genes involved in disease. In addition, genes encoding proteins involved in signal transduction, glycoprotein, transcription, or disease, are expected to be useful as target molecules for therapy, or as medicines in themselves. These genes form a gene group predicted to be strongly involved in disease. Thus, identification of full-length cDNA clones encoding these proteins has great significance..

### SUMMARY OF THE INVENTION

An objective of the present invention is to provide polynucleotides encoding novel polypeptides, polypeptides encoded by the polynucleotides, and novel usages of these.

The inventors have developed a method for efficiently cloning, from a CDNA library having a very high fullness-ratio, human full-length cDNAs predicted to be full-length cDNA clones, where that cDNA library is synthesized by an improved method (WO 01/04286) of oligo-capping (K. Maruyama and S. Sugano, Gene, 138: 171-174 (1994); Y. Suzuki et al., Gene, 200: 149-156 (1997)). The nucleotide sequences of cDNA clones whose fullness ratio is high, obtained by this method, were determined mainly from their 5'-end, and, if required, from their 3'-end.

Among the clones obtained, representative clones estimated to be novel and full-length were analyzed for their full-length nucleotide sequence. The determined full-length nucleotide sequences were analyzed using a BLAST homology search of the databases shown below. Homology searches of the present invention were carried out based on full-length cDNA information, including the entire coding region, and thus homology to every part of a polypeptide could be analyzed. Therefore, in the present invention, the reliability of homology searches has been greatly improved.
[1] SwissProt
   (http://www.ebi.ac.uk/ebi docsSwissProt db/swisshome.html),
[2] GenBank (http://www.ncbi.nlm.nih.gov/web/GenBank),
[3] nr (a protein database, which has been constructed by combining data of coding sequences (CDS) in nucleotide sequences deposited in GenBank, and data of SwissProt, PDB (http://www.rcsb.org/pdb/index.html), PIR (http://pir.georgetown.edu/pirwww/pirhome.shtml), and PRF (http://www.prf.or.jp/en/); overlapping sequences have been removed.), and
[4] RefSeq (http://www.ncbi.nlm.nih.gov/LocusLink/refseq.html).

The gene expression profiles of cDNA clones whose full-length nucleotide sequence had been determined were studied by analyzing the large-scale cDNA database constructed based on the 5'-end nucleotide sequences of cDNAs obtained. The present inventors revealed the usefulness of the genes of the present invention based on these analysis results.

In the present invention, gene functions were revealed by analysis of expression profiles in silico, based on full-length nucleotide sequence information. The expression profiles used in the expression frequency analysis were studied based on databases containing a sufficient amount of fragment sequence data. Expression frequency analysis was carried out by referring, for these expression profiles, to the full-length nucleotide sequences of the many cDNA clones obtained in the present invention. Thus, highly reliable analysis was achieved by referring to the full-length nucleotide sequences of a wide variety of genes in a sufficiently large population for analysis (expression profiles). Namely, the results of expression frequency analysis using the full-length sequences of the present invention more precisely reflect gene expression frequency in tissues and cells from which a certain cDNA library was derived. Thus, the full-length cDNA nucleotide sequence information of the present invention made it possible to achieve highly reliable expression frequency analysis.

The full-length cDNA clones of the present invention were obtained by a method comprising the steps of [1] preparing libraries containing cDNAs with a high fullness ratio using oligo-capping, and [2] assembling 5'-end sequences and selecting those with the highest probability of completeness in length in the cluster formed (there are many clones longer in the 5'-end direction). The use of primers designed based on the 5'- and 3'-end sequences of polynucleotides provided by the present invention enable full-length cDNAs to be readily obtained without using such special techniques. Primers, which are designed for use in obtaining cDNAs capable of being expressed, are not limited to the 5'- and 3'-end sequences of a polynucleotide.

Specifically, the present invention relates to polynucleotides and proteins encoded by the polypeptides as follows.
[1] A polynucleotide selected from the group consisting of:
   (a) a polynucleotide comprising a protein-coding region of the nucleotide sequence according to any one of SEQ ID NOs: 1-1956 and SEQ ID NOs: 3913-3951;
   (b) a polynucleotide comprising the nucleotide sequence encoding a polypeptide that comprises the amino acid sequence of any one of SEQ ID NOs: 1957-3912 and SEQ ID NOs: 3952-3990;
   (c) a polynucleotide comprising a nucleotide sequence encoding a polypeptide, which comprises the amino acid sequence selected from SEQ ID NO: SEQ ID NOs: 1957-3912 and SEQ ID NOs: 3952-3990 wherein one or more amino acids have been substituted, deleted, inserted, and/or added, and which is functionally equivalent to the polypeptide comprising the selected amino acid sequence as described above;
   (d) a polynucleotide which hybridizes to a polynucleotide comprising the nucleotide sequence selected from SEQ ID NOs: 1-1956 and SEQ ID NOs: 3913-3951, and which comprises the nucleotide sequence encoding a polypeptide functionally equivalent to a polypeptide encoded by the selected nucleotide sequence as described above;
   (e) a polynucleotide comprising a nucleotide sequence encoding a partial amino acid sequence of a polypeptide encoded by the polynucleotides according to any one of (a)-(d);
   (f) a polynucleotide comprising a nucleotide sequence having at least 70% identity to the nucleotide sequence of any one of SEQ ID NOs: 1-1956 and SEQ ID NOs: 3913-3951; and
   (g) a polynucleotide comprising a nucleotide sequence having at least 90% identity to the nucleotide sequence of any one of SEQ ID NOs: 1-1956 and SEQ ID NOs: 3913-3951.
[2] A polypeptide encoded by the polynucleotide according to [1], or a partial peptide thereof.
[3] An antibody which binds to the polypeptide or the peptide according to [2].
[4] An immunoassay method for the polypeptide or the peptide according to [2], which comprises the steps of:
   (a) contacting the polypeptide or the peptide according to [2] with the antibody according to [3]; and
   (b) observing the binding between the two.
[5] A vector comprising the polynucleotide according to [1].
[6] A transformant comprising the polynucleotide according to [1] or the vector according to [5].
[7] A transformant which comprises the polynucleotide according to [1] or vector according to [5] in an expressible manner.
[8] A method for producing the polypeptide or the peptide according to [2], which comprises the steps of:
   (a) culturing the transformant according to [7]; and
   (b) recovering the expression product.
[9] An oligonucleotide comprising 15 or more nucleotides, which comprises the nucleotide sequence according to any one of SEQ ID NOs: 1-1956 and SEQ ID NOs: 3913-3951, or a nucleotide sequence complementary to the complementary strand thereof.
[10] A primer for synthesizing a polynucleotide, which comprises the oligonucleotide according to [9].
[11] A probe for detecting a polynucleotide, which comprises the oligonucleotide according to [9].
[12] A polynucleotide according to any one of:
   (a) an antisense polynucleotide comprising a nucleotide sequence complementary to the transcript of the polynucleotide according to [1];
   (b) a polynucleotide with the ribozyme activity for specifically cleaving the transcript of the polynucleotide according to [1]; and
   (c) a polynucleotide which downregulates the expression of the polynucleotide of [1] due to RNAi activity in a host cell.
[13] A method for detecting the polynucleotide according to [1], which comprises the steps of:
   (a) incubating a target polynucleotide with the oligonucleotide according to [9] under conditions ensuring hybridization; and
   (b) detecting the hybridization between the target polynucleotide and the oligonucleotide according to [9].
[14] A database of polynucleotides and/or polypeptides, which comprises information on at least one of the nucleotide sequences of SEQ ID NOs: 1-1956 and SEQ ID NOs: 3913-3951 and/or on at least one of the amino acid sequences of SEQ ID NOs: 1957-3912 and SEQ ID NOs: 3952-3990.

Herein, "polynucleotide" is defined as a molecule, such as a DNA or RNA, in which multiple nucleotides are polymerized. There is no limitation as to the number of polymerized nucleotides. If a polymer contains a relatively low number of nucleotides, it is also described as an "oligonucleotide", which is included in the "polynucleotide" of the present invention. The polynucleotides or oligonucleotides of the present invention can be natural or chemically synthesized. Alternatively, they can be synthesized using a template polynucleotide in an enzymatic reaction such as PCR. Furthermore, the polynucleotides of the present invention may be modified chemically. Single-stranded and double-stranded polynucleotides are included in the present invention. In this specification, especially in the claims, when the polynucleotides are described merely as "polynucleotide", it means not only single-stranded polynucleotides but also double-stranded polynucleotides. When it means a double-stranded polynucleotide, the nucleotide sequence of only one chain is indicated. However, based on the nucleotide sequence of a sense chain, the nucleotide sequence of the corresponding complementary strand is essentially determined.

All the cDNAs provided by the present invention are full-length cDNAs. "Full-length cDNAs" herein means cDNAs containing the ATG codon, which is the start point of translation therein. Untranslated regions upstream and downstream of the protein-coding region are both naturally contained in natural mRNAs and are not essential. It is preferable that the full-length cDNAs of the present invention contain a stop codon.

### BRIEF DESCRIPTION OF THE DRAWINGS

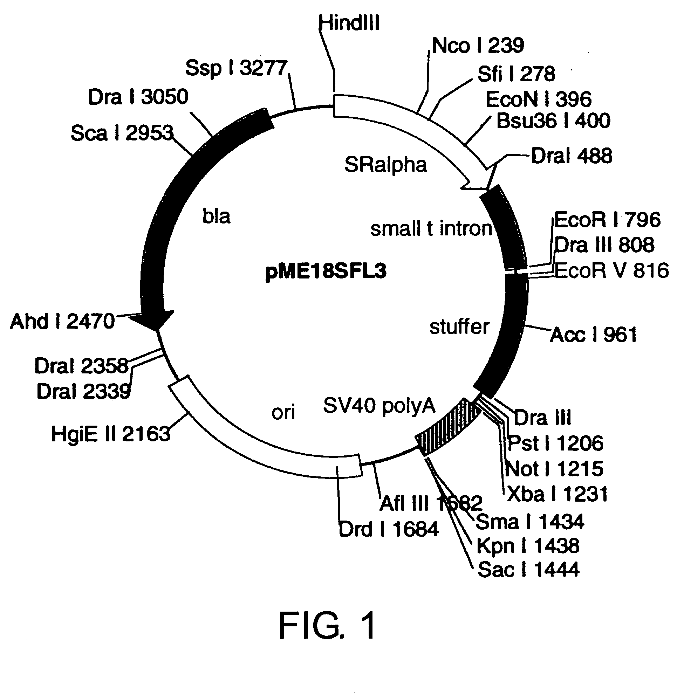

Fig. 1 shows the restriction map of the vector pME18SFL3.

### DETAILED DESCRIPTION OF THE INVENTION

All of the clones (1,995 clones) of the present invention are novel and encode full-length polypeptides. Further, all of the clones are cDNAs with a high fullness ratio, and which were obtained by oligo-capping method. None of the clones are identical to any known human mRNAs (namely, they are novel clones) selected by searching 5'-end sequences and mRNA sequences with the annotation of "complete cds" in the GenBank and UniGene (Human) (http://www.ncbi.nlm.gov/UniGene) databases using BLAST homology [S. F. Altschul, W. Gish, W. Miller, E. W. Myers & D. J. Lipman, J. Mol. Biol., 215: 403-410 (1990); W. Gish & D. J. States, Nature Genet., 3: 266-272 (1993)]. They are also clones that were assumed to have a higher fullness ratio among members in assembled clusters. Most of the clones with a high fullness ratio in a cluster had nucleotide sequences longer in the 5'-end direction.

All of the full-length cDNAs of the present invention can be synthesized by a method such as PCR (Current protocols in Molecular Biology edit. Ausubel et al. (1987) Publish. John Wiley & Sons Section 6.1-6.4) using primer sets designed based on 5'-end and 3'-end sequences, or using primer sets of primers designed based on 5'-end sequences and a primer of oligo dT sequence corresponding to poly A sequence. Table 1 contains the clone names of 1,995 full-length cDNA clones of the present invention, SEQ ID NOs of the full-length nucleotide sequences, CDS portions deduced from the full-length nucleotide sequences, and SEQ ID NOs of the translated amino acids. The positions of CDS are shown according to the rules set out in "DDBJ/EMBL/GenBank Feature Table Definition" (http://www.ncbi.nlm.nih.gov/collab/FT/index.html). The start position number corresponds to the first letter of "ATG", the nucleotide triplet encoding methionine; the termination position number corresponds to the third letter of the stop codon. These are indicated by flanking with the mark "..". However, in clones without a stop codon, the termination position is indicated by the mark ">", according to the above rules.

**Table 1**

| Clone Name | SEQ ID NO of Nucleotide | CDS Portion | SEQ ID NO of Amino Acid |
|---|---|---|---|
| 3NB692004045 | 1 | 220..>1437 | 1957 |
| ADIPS2000069 | 2 | 48..1538 | 1958 |
| ADRGL2010315 | 3 | 1182..1604 | 1959 |
| ADRGL2010594 | 4 | 203.. 664 | 1960 |
| AHMSC1000138 | 5 | 135.. 455 | 1961 |
| ASTRO2008972 | 6 | 455.. 805 | 1962 |
| ASTRO2015162 | 7 | 244.. 951 | 1963 |
| ASTRO2016114 | 8 | 796..1779 | 1964 |
| ASTRO3000154 | 9 | 2154..2807 | 1965 |
| BEAST2000981 | 10 | 149..1357 | 1966 |
| BLADE2000256 | 11 | 461..1687 | 1967 |
| BLADE2001031 | 12 | 27.. 506 | 1968 |
| BLADE2002310 | 13 | 1065..>2616 | 1969 |
| BLADE2002744 | 14 | 82.. 510 | 1970 |
| BLADE2004849 | 15 | 276..1202 | 1971 |
| BLADE2006043 | 16 | 218.. 622 | 1972 |
| BLADE2007735 | 17 | 310.. 723 | 1973 |
| BLADE2007744 | 18 | 1629..>1944 | 1974 |
| BLADE2007799 | 19 | 917..2158 | 1975 |
| BLADE2008809 | 20 | 286..1575 | 1976 |
| BRACE1000475 | 21 | 2878..3471 | 1977 |
| BRACE2002392 | 22 | 76..1008 | 1978 |
| BRACE2003628 | 23 | 145..1164 | 1979 |
| BRACE2005991 | 24 | 337.. 666 | 1980 |
| BRACE2010336 | 25 | 23..1387 | 1981 |
| BRACE2012528 | 26 | 114..1289 | 1982 |
| BRACE2012625 | 27 | 84..1406 | 1983 |
| BRACE2012833 | 28 | 244.. 651 | 1984 |
| BRACE2012838 | 29 | 127.. 468 | 1985 |
| BRACE2012936 | 30 | 617.. 994 | 1986 |
| BRACE2012947 | 31 | 756..1223 | 1987 |
| BRACE2013009 | 32 | 777..1487 | 1988 |
| BRACE2013126 | 33 | 334.. 651 | 1989 |
| BRACE2013132 | 34 | 73.. 795 | 1990 |
| BRACE2016896 | 35 | 27..1298 | 1991 |
| BRACE2017359 | 36 | 755..1228 | 1992 |
| BRACE2017397 | 37 | 99.. 872 | 1993 |
| BRACE2017580 | 38 | 514.. 834 | 1994 |
| BRACE2017844 | 39 | 1674..>2015 | 1995 |
| BRACE2017872 | 40 | 148..>1404 | 1996 |
| BRACE2017992 | 41 | 934..1245 | 1997 |
| BRACE2019348 | 42 | 1423..2004 | 1998 |
| BRACE2023633 | 43 | 122.. 478 | 1999 |
| BRACE2023744 | 44 | 349.. 771 | 2000 |
| BRACE2025452 | 45 | 55.. 426 | 2001 |
| BRACE2026404 | 46 | 67.. 408 | 2002 |
| BRACE2027312 | 47 | 255.. 848 | 2003 |
| BRACE2027382 | 48 | 1749..2090 | 2004 |
| BRACE2028956 | 49 | 133.. 480 | 2005 |
| BRACE2030039 | 50 | 87.. 719 | 2006 |
| BRACE2032584 | 51 | 129.. 485 | 2007 |
| BRACE2033128 | 52 | 296.. 676 | 2008 |
| BRACE2034434 | 53 | 1963..2664 | 2009 |
| BRACE2035120 | 54 | 22..1938 | 2010 |
| BRACE2035191 | 55 | 207.. 542 | 2011 |
| BRACE2039362 | 56 | 81.. 440 | 2012 |
| BRACE2039607 | 57 | 1850..2398 | 2013 |
| BRACE2042541 | 58 | 96..1562 | 2014 |
| BRACE2046976 | 59 | 836..1345 | 2015 |
| BRACE2047232 | 60 | 1434..2363 | 2016 |
| BRACE2047975 | 61 | 299.. 652 | 2017 |
| BRACE3001403 | 62 | 1965..2507 | 2018 |
| BRACE3001973 | 63 | 24..2702 | 2019 |
| BRACE3002264 | 64 | 72..2870 | 2020 |
| BRACE3002344 | 65 | 113..3295 | 2021 |
| BRACE3002541 | 66 | 2255..2593 | 2022 |
| BRACE3002756 | 67 | 595..2700 | 2023 |
| BRACE3003866 | 68 | 2667..3542 | 2024 |
| BRACE3004046 | 69 | 2774..3277 | 2025 |
| BRACE3004371 | 70 | 467.. 832 | 2026 |
| BRACE3004767 | 71 | 3944..4390 | 2027 |
| BRACE3004887 | 72 | 2217..2792 | 2028 |
| BRACE3004981 | 73 | 3154..3624 | 2029 |
| BRACE3005870 | 74 | 5.. 379 | 2030 |
| BRACE3005903 | 75 | 328..3378 | 2031 |
| BRACE3006553 | 76 | 2589..3344 | 2032 |
| BRACE3007649 | 77 | 4951..5472 | 2033 |
| BRACE3007869 | 78 | 932..1549 | 2034 |
| BRACE3009075 | 79 | 1318..2025 | 2035 |
| BRACE3009265 | 80 | 1886..2374 | 2036 |
| BRACE3009392 | 81 | 2134..3072 | 2037 |
| BRACE3009416 | 82 | 2056..2805 | 2038 |
| BRACE3009539 | 83 | 117.. 449 | 2039 |
| BRACE3010702 | 84 | 1062..1619 | 2040 |
| BRACE3011447 | 85 | 289.. 786 | 2041 |
| BRACE3011774 | 86 | 906..1331 | 2042 |
| BRACE3013418 | 87 | 17..3121 | 2043 |
| BRACE3013874 | 88 | 765..2255 | 2044 |
| BRACE3013986 | 89 | 335.. 661 | 2045 |
| BRACE3014523 | 90 | 318..1532 | 2046 |
| BRACE3014714 | 91 | 1034..2182 | 2047 |
| BRACE3015090 | 92 | 287.. 616 | 2048 |
| BRACE3015898 | 93 | 119.. 448 | 2049 |
| BRACE3016020 | 94 | 62.. 586 | 2050 |
| BRACE3016167 | 95 | 67.. 507 | 2051 |
| BRACE3016580 | 96 | 225.. 578 | 2052 |
| BRACE3016788 | 97 | 1740..2486 | 2053 |
| BRACE3016810 | 98 | 2064..2561 | 2054 |
| BRACE3016862 | 99 | 33.. 335 | 2055 |
| BRACE3017253 | 100 | 86..1342 | 2056 |
| BRACE3018083 | 101 | 1447..2541 | 2057 |
| BRACE3019570 | 102 | 320..3358 | 2058 |
| BRACE3019611 | 103 | 2005..2484 | 2059 |
| BRACE3019817 | 104 | 7.. 345 | 2060 |
| BRACE3019941 | 105 | 1120..3507 | 2061 |
| BRACE3020356 | 106 | 344.. 661 | 2062 |
| BRACE3020669 | 107 | 2192..2650 | 2063 |
| BRACE3021430 | 108 | 243.. 686 | 2064 |
| BRACE3021517 | 109 | 218.. 523 | 2065 |
| BRACE3021805 | 110 | 312..1283 | 2066 |
| BRACE3022051 | 111 | 1042..1515 | 2067 |
| BRACE3022303 | 112 | 2107..3009 | 2068 |
| BRACE3022312 | 113 | 98..1003 | 2069 |
| BRACE3022340 | 114 | 20..2785 | 2070 |
| BRACE3022847 | 115 | 560..1066 | 2071 |
| BRACE3023604 | 116 | 600..>1057 | 2072 |
| BRACE3024379 | 117 | 412.. 717 | 2073 |
| BRACE3024444 | 118 | 129.. 455 | 2074 |
| BRACE3024497 | 119 | 219.. 545 | 2075 |
| BRACE3024537 | 120 | 377.. 724 | 2076 |
| BRACE3024879 | 121 | 142.. 786 | 2077 |
| BRACE3025627 | 122 | 1648..2019 | 2078 |
| BRACE3025719 | 123 | 111..1811 | 2079 |
| BRACE3026161 | 124 | 330.. 722 | 2080 |
| BRACE3026290 | 125 | 211..3225 | 2081 |
| BRACE3026345 | 126 | 2603..3145 | 2082 |
| BRACE3026456 | 127 | 2113..2439 | 2083 |
| BRACE3026802 | 128 | 298..1260 | 2084 |
| BRACE3026844 | 129 | 1231..3081 | 2085 |
| BRACE3026947 | 130 | 1528..3849 | 2086 |
| BRACE3027256 | 131 | 670..1020 | 2087 |
| BRACE3027931 | 132 | 64..1260 | 2088 |
| BRACE3028360 | 133 | 4486..4920 | 2089 |
| BRACE3028895 | 134 | 716..1129 | 2090 |
| BRACE3028998 | 135 | 264.. 785 | 2091 |
| BRACE3029005 | 136 | 394.. 771 | 2092 |
| BRACE3029021 | 137 | 208.. 657 | 2093 |
| BRACE3029205 | 138 | 3800..4531 | 2094 |
| BRACE3029447 | 139 | 1873..2424 | 2095 |
| BRACE3030538 | 140 | 253.. 828 | 2096 |
| BRACE3031161 | 141 | 138.. 494 | 2097 |
| BRACE3031184 | 142 | 57.. 422 | 2098 |
| BRACE3031185 | 143 | 11.. 370 | 2099 |
| BRACE3031315 | 144 | 1220..2563 | 2100 |
| BRACE3031372 | 145 | 1587..2429 | 2101 |
| BRACE3031579 | 146 | 1636..2079 | 2102 |
| BRACE3031728 | 147 | 22..2196 | 2103 |
| BRACE3031743 | 148 | 53.. 805 | 2104 |
| BRACE3031843 | 149 | 115.. 429 | 2105 |
| BRACE3032385 | 150 | 103..2007 | 2106 |
| BRACE3032537 | 151 | 639..1199 | 2107 |
| BRACE3032538 | 152 | 3317..4198 | 2108 |
| BRACE3032631 | 153 | 790..1701 | 2109 |
| BRACE3032980 | 154 | 223.. 618 | 2110 |
| BRACE3033525 | 155 | 1346..1672 | 2111 |
| BRACE3034183 | 156 | 190..1686 | 2112 |
| BRACE3034389 | 157 | 176..1294 | 2113 |
| BRACE3034964 | 158 | 2385..3278 | 2114 |
| BRACE3034993 | 159 | 1533..1838 | 2115 |
| BRACE3035168 | 160 | 829..1149 | 2116 |
| BRACE3036156 | 161 | 3.. 338 | 2117 |
| BRACE3036271 | 162 | 2019..3125 | 2118 |
| BRACE3036283 | 163 | 2599..3186 | 2119 |
| BRACE3037612 | 164 | 2401..2892 | 2120 |
| BRACE3037637 | 165 | 199.. 570 | 2121 |
| BRACE3037803 | 166 | 2507..2884 | 2122 |
| BRACE3038012 | 167 | 2134..2634 | 2123 |
| BRACE3038030 | 168 | 976..1302 | 2124 |
| BRACE3038570 | 169 | 223.. 621 | 2125 |
| BRACE3038760 | 170 | 2145..2906 | 2126 |
| BRACE3039288 | 171 | 876..1511 | 2127 |
| BRACE3039358 | 172 | 385..2664 | 2128 |
| BRACE3039378 | 173 | 479..2254 | 2129 |
| BRACE3039454 | 174 | 351..1073 | 2130 |
| BRACE3040012 | 175 | 145.. 465 | 2131 |
| BRACE3040239 | 176 | 1505..2548 | 2132 |
| BRACE3040504 | 177 | 307.. 738 | 2133 |
| BRACE3040644 | 178 | 1280..3214 | 2134 |
| BRACE3040863 | 179 | 12.. 626 | 2135 |
| BRACE3041059 | 180 | 99..2558 | 2136 |
| BRACE3041162 | 181 | 2041..2604 | 2137 |
| BRACE3041827 | 182 | 3169..3561 | 2138 |
| BRACE3042046 | 183 | 1488..2879 | 2139 |
| BRACE3042210 | 184 | 174.. 533 | 2140 |
| BRACE3042326 | 185 | 216..2366 | 2141 |
| BRACE3042409 | 186 | 238..3258 | 2142 |
| BRACE3042432 | 187 | 2084..2473 | 2143 |
| BRACE3042594 | 188 | 179.. 517 | 2144 |
| BRACE3043597 | 189 | 23.. 460 | 2145 |
| BRACE3044090 | 190 | 1198..1602 | 2146 |
| BRACE3044172 | 191 | 803..3067 | 2147 |
| BRACE3044247 | 192 | 107.. 451 | 2148 |
| BRACE3044377 | 193 | 223.. 900 | 2149 |
| BRACE3044495 | 194 | 893..1510 | 2150 |
| BRACE3045078 | 195 | 90.. 593 | 2151 |
| BRACE3045145 | 196 | 187.. 501 | 2152 |
| BRACE3045424 | 197 | 275.. 619 | 2153 |
| BRACE3045708 | 198 | 219.. 587 | 2154 |
| BRACE3045981 | 199 | 302.. 724 | 2155 |
| BRACE3046049 | 200 | 849..1298 | 2156 |
| BRACE3046152 | 201 | 2241..3440 | 2157 |
| BRACE3046294 | 202 | 189.. 578 | 2158 |
| BRACE3046466 | 203 | 111..2969 | 2159 |
| BRACE3046491 | 204 | 26..2275 | 2160 |
| BRACE3046609 | 205 | 1563..2477 | 2161 |
| BRACE3046837 | 206 | 25.. 558 | 2162 |
| BRACE3046855 | 207 | 3735..4247 | 2163 |
| BRACE3046966 | 208 | 12.. 452 | 2164 |
| BRACE3047018 | 209 | 2886..3425 | 2165 |
| BRACE3047482 | 210 | 43..2313 | 2166 |
| BRACE3047801 | 211 | 17..3757 | 2167 |
| BRACE3048483 | 212 | 2340..2672 | 2168 |
| BRACE3048565 | 213 | 264.. 626 | 2169 |
| BRACE3048615 | 214 | 352.. 729 | 2170 |
| BRACE3048677 | 215 | 238.. 732 | 2171 |
| BRACE3048756 | 216 | 2483..3052 | 2172 |
| BRACE3048904 | 217 | 229.. 759 | 2173 |
| BRACE3048905 | 218 | 1733..2038 | 2174 |
| BRACE3049186 | 219 | 106.. 519 | 2175 |
| BRACE3049714 | 220 | 294..2957 | 2176 |
| BRACE3050270 | 221 | 2725..3291 | 2177 |
| BRACE3050504 | 222 | 287..1306 | 2178 |
| BRACE3051144 | 223 | 176.. 763 | 2179 |
| BRACE3051621 | 224 | 1748..3418 | 2180 |
| BRACE3051627 | 225 | 1216..2235 | 2181 |
| BRACE3051722 | 226 | 816..1190 | 2182 |
| BRACE3051819 | 227 | 3..2801 | 2183 |
| BRACE3051879 | 228 | 193.. 519 | 2184 |
| BRACE3052321 | 229 | 124..1614 | 2185 |
| BRACE3052410 | 230 | 17..2926 | 2186 |
| BRACE3052486 | 231 | 1622..1942 | 2187 |
| BRACE3052595 | 232 | 40.. 780 | 2188 |
| BRALZ2003119 | 233 | 363..1844 | 2189 |
| BRALZ2007661 | 234 | 1159..1746 | 2190 |
| BRALZ2008930 | 235 | 61.. 825 | 2191 |
| BRALZ2010842 | 236 | 173..1048 | 2192 |
| BRALZ2011337 | 237 | 3.. 305 | 2193 |
| BRALZ2013621 | 238 | 214.. 576 | 2194 |
| BRALZ2013690 | 239 | 283.. 603 | 2195 |
| BRALZ2014054 | 240 | 213.. 920 | 2196 |
| BRAMY2015516 | 241 | 747..1292 | 2197 |
| BRAMY2021098 | 242 | 1405..1785 | 2198 |
| BRAMY2022320 | 243 | 320..1975 | 2199 |
| BRAMY2023939 | 244 | 39..>2128 | 2200 |
| BRAMY2025495 | 245 | 552..1031 | 2201 |
| BRAMY2031516 | 246 | 1227..>2798 | 2202 |
| BRAMY2033895 | 247 | 175.. 531 | 2203 |
| BRAMY2035801 | 248 | 1487..1963 | 2204 |
| BRAMY2036254 | 249 | 156.. 548 | 2205 |
| BRAMY2036266 | 250 | 294.. 776 | 2206 |
| BRAMY2037609 | 251 | 107.. 787 | 2207 |
| BRAMY2039630 | 252 | 1281..1703 | 2208 |
| BRAMY2040915 | 253 | 488..2380 | 2209 |
| BRAMY2041347 | 254 | 332..2059 | 2210 |
| BRAMY2041384 | 255 | 682..2211 | 2211 |
| BRAMY2041507 | 256 | 1331..2029 | 2212 |
| BRAMY2044686 | 257 | 1418..1990 | 2213 |
| BRAMY2046489 | 258 | 375.. 698 | 2214 |
| BRAMY2046537 | 259 | 293.. 952 | 2215 |
| BRAMY3000692 | 260 | 1427..1960 | 2216 |
| BRAMY3001409 | 261 | 1952..2428 | 2217 |
| BRAMY3002329 | 262 | 2751..3269 | 2218 |
| BRAMY3002508 | 263 | 1949..2374 | 2219 |
| BRAMY3002886 | 264 | 25..2148 | 2220 |
| BRAMY3004126 | 265 | 1657..2676 | 2221 |
| BRAMY3004364 | 266 | 1056..1466 | 2222 |
| BRAMY3005184 | 267 | 430..3735 | 2223 |
| BRAMY3005656 | 268 | 919..1632 | 2224 |
| BRAMY3005912 | 269 | 3863..4513 | 2225 |
| BRAMY3007078 | 270 | 493..3057 | 2226 |
| BRAMY3007449 | 271 | 229.. 726 | 2227 |
| BRAMY3007471 | 272 | 3.. 344 | 2228 |
| BRAMY3008436 | 273 | 3093..3557 | 2229 |
| BRAMY3009158 | 274 | 116.. 511 | 2230 |
| BRAMY3009491 | 275 | 2262..3581 | 2231 |
| BRAMY3009556 | 276 | 3108..3455 | 2232 |
| BRAMY3009904 | 277 | 286.. 705 | 2233 |
| BRAMY3010321 | 278 | 332..2785 | 2234 |
| BRAMY3010603 | 279 | 311.. 706 | 2235 |
| BRAMY3010654 | 280 | 1357..1947 | 2236 |
| BRAMY3010902 | 281 | 182.. 487 | 2237 |
| BRAMY3011501 | 282 | 185..2437 | 2238 |
| BRAMY3011581 | 283 | 1000..2361 | 2239 |
| BRAMY3011865 | 284 | 2563..3414 | 2240 |
| BRAMY3014027 | 285 | 362..2593 | 2241 |
| BRAMY3014555 | 286 | 100..1563 | 2242 |
| BRAMY3014613 | 287 | 437..2254 | 2243 |
| BRAMY3015086 | 288 | 179..2284 | 2244 |
| BRAMY3015547 | 289 | 983..4105 | 2245 |
| BRAMY3015549 | 290 | 622..>3960 | 2246 |
| BRAMY3016829 | 291 | 99.. 428 | 2247 |
| BRAMY3017827 | 292 | 24..1400 | 2248 |
| BRAMY3017920 | 293 | 398..1486 | 2249 |
| BRAMY3017965 | 294 | 2468..3796 | 2250 |
| BRAMY3018121 | 295 | 78.. 605 | 2251 |
| BRAMY3018248 | 296 | 2236..2946 | 2252 |
| BRAMY3018340 | 297 | 161..>3646 | 2253 |
| BRAMY3018754 | 298 | 101..1555 | 2254 |
| BRAMY4000915 | 299 | 307..2370 | 2255 |
| BRAMY4000962 | 300 | 1695..3305 | 2256 |
| BRAMY4001234 | 301 | 578..1183 | 2257 |
| BRAMY4001652 | 302 | 184..2169 | 2258 |
| BRAMY4001863 | 303 | 30.. 923 | 2259 |
| BRAMY4001913 | 304 | 1048..1440 | 2260 |
| BRAMY4002575 | 305 | 41.. 886 | 2261 |
| BRAMY4002628 | 306 | 10..2097 | 2262 |
| BRAWH2000256 | 307 | 898..1617 | 2263 |
| BRAWH2002333 | 308 | 387..1385 | 2264 |
| BRAWH2004078 | 309 | 536..1525 | 2265 |
| BRAWH2010364 | 310 | 1383..2126 | 2266 |
| BRAWH2010619 | 311 | 291.. 896 | 2267 |
| BRAWH2011796 | 312 | 2109..2423 | 2268 |
| BRAWH2011812 | 313 | 9..3053 | 2269 |
| BRAWH2011958 | 314 | 1316..1732 | 2270 |
| BRAWH2012054 | 315 | 1195..1809 | 2271 |
| BRAWH2012866 | 316 | 658..1926 | 2272 |
| BRAWH2013955 | 317 | 325.. 642 | 2273 |
| BRAWH2014053 | 318 | 363.. 962 | 2274 |
| BRAWH2016209 | 319 | 96..1985 | 2275 |
| BRAWH2016223 | 320 | 1144..2187 | 2276 |
| BRAWH2016305 | 321 | 727..1320 | 2277 |
| BRAWH2016514 | 322 | 325..1236 | 2278 |
| BRAWH2016562 | 323 | 1832..2242 | 2279 |
| BRAWH2016785 | 324 | 695..1201 | 2280 |
| BRAWH3000446 | 325 | 822..2396 | 2281 |
| BRAWH3000884 | 326 | 2966..4306 | 2282 |
| BRAWH3001053 | 327 | 1956..3152 | 2283 |
| BRAWH3001638 | 328 | 1017..4334 | 2284 |
| BRAWH3001783 | 329 | 363..2540 | 2285 |
| BRAWH3001833 | 330 | 209..1012 | 2286 |
| BRAWH3003244 | 331 | 892..1248 | 2287 |
| BRAWH3003573 | 332 | 2544..2936 | 2288 |
| BRAWH3003975 | 333 | 40.. 342 | 2289 |
| BRAWH3004335 | 334 | 1017..1733 | 2290 |
| BRAWH3004350 | 335 | 498..1247 | 2291 |
| BRAWH3005037 | 336 | 1815..2180 | 2292 |
| BRAWH3005886 | 337 | 307..1545 | 2293 |
| BRAWH3005892 | 338 | 668.. 970 | 2294 |
| BRAWH3005896 | 339 | 328.. 636 | 2295 |
| BRAWH3008167 | 340 | 212..2563 | 2296 |
| BRAWH3008559 | 341 | 244..3627 | 2297 |
| BRAWH3008867 | 342 | 249.. 686 | 2298 |
| BRAWH3009961 | 343 | 140..4189 | 2299 |
| BRAWH3010461 | 344 | 188.. 895 | 2300 |
| BRAWH3010602 | 345 | 642..1319 | 2301 |
| BRAWH3010657 | 346 | 363..3491 | 2302 |
| BRAWH3010726 | 347 | 1866..3446 | 2303 |
| BRAWH3010833 | 348 | 2255..2632 | 2304 |
| BRAWH3011101 | 349 | 188.. 502 | 2305 |
| BRAWH3011331 | 350 | 193.. 942 | 2306 |
| BRAWH3011402 | 351 | 173.. 538 | 2307 |
| BRAWH3011577 | 352 | 369.. 737 | 2308 |
| BRAWH3011623 | 353 | 2953..3834 | 2309 |
| BRAWH3011685 | 354 | 1342..1935 | 2310 |
| BRAWH3011907 | 355 | 140.. 472 | 2311 |
| BRAWH3011929 | 356 | 447.. 800 | 2312 |
| BRAWH3012005 | 357 | 1816..2289 | 2313 |
| BRAWH3012662 | 358 | 1213..1854 | 2314 |
| BRAWH3012779 | 359 | 460.. 999 | 2315 |
| BRAWH3013009 | 360 | 2236..3414 | 2316 |
| BRAWH3013049 | 361 | 17.. 343 | 2317 |
| BRAWH3013264 | 362 | 68..2716 | 2318 |
| BRAWH3013508 | 363 | 1159..1539 | 2319 |
| BRAWH3014609 | 364 | 416..1972 | 2320 |
| BRAWH3014639 | 365 | 84.. 515 | 2321 |
| BRAWH3015017 | 366 | 1097..1402 | 2322 |
| BRAWH3015175 | 367 | 38..1054 | 2323 |
| BRAWH3015610 | 368 | 450.. 896 | 2324 |
| BRAWH3015825 | 369 | 180..1181 | 2325 |
| BRAWH3016123 | 370 | 3057..3677 | 2326 |
| BRAWH3016715 | 371 | 953..1690 | 2327 |
| BRAWH3017180 | 372 | 88..2925 | 2328 |
| BRAWH3017259 | 373 | 259..3957 | 2329 |
| BRAWH3017260 | 374 | 549..2114 | 2330 |
| BRAWH3017477 | 375 | 11..1324 | 2331 |
| BRAWH3017980 | 376 | 23.. 358 | 2332 |
| BRAWH3018063 | 377 | 67..2175 | 2333 |
| BRAWH3018369 | 378 | 271.. 846 | 2334 |
| BRAWH3018548 | 379 | 569..3589 | 2335 |
| BRAWH3018969 | 380 | 192..1553 | 2336 |
| BRAWH3019026 | 381 | 26..2530 | 2337 |
| BRAWH3019529 | 382 | 12.. 464 | 2338 |
| BRAWH3019594 | 383 | 657..1079 | 2339 |
| BRAWH3019820 | 384 | 1802..2194 | 2340 |
| BRAWH3020200 | 385 | 412.. 822 | 2341 |
| BRAWH3020318 | 386 | 23.. 775 | 2342 |
| BRAWH3020884 | 387 | 265..1347 | 2343 |
| BRAWH3020928 | 388 | 1114..1422 | 2344 |
| BRAWH3021012 | 389 | 410..2296 | 2345 |
| BRAWH3021574 | 390 | 17..1576 | 2346 |
| BRAWH3021580 | 391 | 710..2128 | 2347 |
| BRAWH3021641 | 392 | 222.. 524 | 2348 |
| BRAWH3021643 | 393 | 64.. 408 | 2349 |
| BRAWH3021724 | 394 | 63..1172 | 2350 |
| BRAWH3022347 | 395 | 1572..3788 | 2351 |
| BRAWH3022431 | 396 | 100.. 414 | 2352 |
| BRAWH3022459 | 397 | 256.. 621 | 2353 |
| BRAWH3022542 | 398 | 395.. 772 | 2354 |
| BRAWH3022651 | 399 | 171..2396 | 2355 |
| BRAWH3022719 | 400 | 209.. 565 | 2356 |
| BRAWH3022900 | 401 | 47.. 391 | 2357 |
| BRAWH3023156 | 402 | 2880..3281 | 2358 |
| BRAWH3023168 | 403 | 2271..2606 | 2359 |
| BRAWH3023172 | 404 | 968..2017 | 2360 |
| BRAWH3023274 | 405 | 3302..3706 | 2361 |
| BRAWH3023415 | 406 | 2179..2850 | 2362 |
| BRAWH3023421 | 407 | 1713..2354 | 2363 |
| BRAWH3024186 | 408 | 58..2940 | 2364 |
| BRAWH3024231 | 409 | 1815..2843 | 2365 |
| BRAWH3024242 | 410 | 1094..1741 | 2366 |
| BRAWH3024506 | 411 | 2506..3510 | 2367 |
| BRAWH3024989 | 412 | 1445..2161 | 2368 |
| BRAWH3025157 | 413 | 1..1746 | 2369 |
| BRAWH3026349 | 414 | 165..1166 | 2370 |
| BRAWH3026938 | 415 | 270..2579 | 2371 |
| BRAWH3027420 | 416 | 67.. 483 | 2372 |
| BRAWH3027440 | 417 | 1644..2651 | 2373 |
| BRAWH3027533 | 418 | 328..2253 | 2374 |
| BRAWH3027574 | 419 | 20..3067 | 2375 |
| BRAWH3027607 | 420 | 3857..4312 | 2376 |
| BRAWH3027616 | 421 | 233.. 682 | 2377 |
| BRAWH3027675 | 422 | 1315..1779 | 2378 |
| BRAWH3027806 | 423 | 359..2653 | 2379 |
| BRAWH3027880 | 424 | 244..1482 | 2380 |
| BRAWH3028202 | 425 | 1707..2297 | 2381 |
| BRAWH3028223 | 426 | 26.. 352 | 2382 |
| BRAWH3028461 | 427 | 194.. 526 | 2383 |
| BRAWH3028754 | 428 | 173.. 811 | 2384 |
| BRAWH3028796 | 429 | 1035..2291 | 2385 |
| BRAWH3029313 | 430 | 171.. 563 | 2386 |
| BRAWH3029385 | 431 | 3368..4567 | 2387 |
| BRAWH3029538 | 432 | 153.. 773 | 2388 |
| BRAWH3029806 | 433 | 16..2613 | 2389 |
| BRAWH3030772 | 434 | 470.. 865 | 2390 |
| BRAWH3030810 | 435 | 2278..2976 | 2391 |
| BRAWH3030910 | 436 | 872..1864 | 2392 |
| BRAWH3031054 | 437 | 374.. 769 | 2393 |
| BRAWH3031342 | 438 | 170..1699 | 2394 |
| BRAWH3031710 | 439 | 1326..4424 | 2395 |
| BRAWH3032298 | 440 | 114..4013 | 2396 |
| BRAWH3032340 | 441 | 254..3775 | 2397 |
| BRAWH3032571 | 442 | 1..3678 | 2398 |
| BRAWH3033117 | 443 | 479..1165 | 2399 |
| BRAWH3033293 | 444 | 308..3097 | 2400 |
| BRAWH3033448 | 445 | 352..1584 | 2401 |
| BRAWH3033513 | 446 | 660..1061 | 2402 |
| BRAWH3034097 | 447 | 1331..1711 | 2403 |
| BRAWH3034114 | 448 | 230.. 727 | 2404 |
| BRAWH3034134 | 449 | 211.. 555 | 2405 |
| BRAWH3034668 | 450 | 1326..3407 | 2406 |
| BRAWH3034743 | 451 | 2..1855 | 2407 |
| BRAWH3034775 | 452 | 133..2391 | 2408 |
| BRAWH3034890 | 453 | 123..1124 | 2409 |
| BRAWH3035403 | 454 | 77..1249 | 2410 |
| BRAWH3035904 | 455 | 49..2673 | 2411 |
| BRAWH3035914 | 456 | 54..1340 | 2412 |
| BRAWH3035936 | 457 | 703..2760 | 2413 |
| BRAWH3036077 | 458 | 350.. 973 | 2414 |
| BRAWH3036247 | 459 | 282..2219 | 2415 |
| BRAWH3036270 | 460 | 1844..3085 | 2416 |
| BRAWH3036334 | 461 | 456..2537 | 2417 |
| BRAWH3036561 | 462 | 136.. 579 | 2418 |
| BRAWH3037265 | 463 | 1658..>4355 | 2419 |
| BRAWH3037394 | 464 | 15.. 485 | 2420 |
| BRAWH3037428 | 465 | 1010..1558 | 2421 |
| BRAWH3037533 | 466 | 282.. 593 | 2422 |
| BRAWH3037979 | 467 | 249..>4245 | 2423 |
| BRAWH3038055 | 468 | 1554..5042 | 2424 |
| BRAWH3038230 | 469 | 29.. 364 | 2425 |
| BRAWH3038252 | 470 | 1..2535 | 2426 |
| BRAWH3038324 | 471 | 925..2925 | 2427 |
| BRAWH3038827 | 472 | 270..2669 | 2428 |
| BRAWH3039258 | 473 | 1933..4083 | 2429 |
| BRAWH3039623 | 474 | 2197..2607 | 2430 |
| BRAWH3040297 | 475 | 198.. 686 | 2431 |
| BRAWH3040695 | 476 | 3041..3466 | 2432 |
| BRAWH3040711 | 477 | 165.. 746 | 2433 |
| BRAWH3040900 | 478 | 567..1043 | 2434 |
| BRAWH3041492 | 479 | 2501..3106 | 2435 |
| BRAWH3041556 | 480 | 780..3560 | 2436 |
| BRAWH3041928 | 481 | 144.. 464 | 2437 |
| BRAWH3042132 | 482 | 160.. 621 | 2438 |
| BRAWH3042438 | 483 | 27.. 974 | 2439 |
| BRAWH3042447 | 484 | 303..2771 | 2440 |
| BRAWH3042568 | 485 | 243.. 803 | 2441 |
| BRAWH3042772 | 486 | 470.. 979 | 2442 |
| BRAWH3042787 | 487 | 67.. 405 | 2443 |
| BRAWH3042820 | 488 | 1311..1778 | 2444 |
| BRAWH3042996 | 489 | 349.. 744 | 2445 |
| BRAWH3043034 | 490 | 84..3068 | 2446 |
| BRAWH3043295 | 491 | 547.. 945 | 2447 |
| BRAWH3043498 | 492 | 28.. 375 | 2448 |
| BRAWH3043623 | 493 | 236.. 832 | 2449 |
| BRAWH3043944 | 494 | 128.. 796 | 2450 |
| BRAWH3044122 | 495 | 106..2874 | 2451 |
| BRAWH3044151 | 496 | 1625..2767 | 2452 |
| BRAWH3044487 | 497 | 80..1294 | 2453 |
| BRAWH3044585 | 498 | 276.. 857 | 2454 |
| BRAWH3044676 | 499 | 249.. 611 | 2455 |
| BRAWH3044985 | 500 | 3188..3493 | 2456 |
| BRAWH3045118 | 501 | 5..3106 | 2457 |
| BRAWH3045229 | 502 | 315..1136 | 2458 |
| BRAWH3045625 | 503 | 71.. 388 | 2459 |
| BRAWH3046196 | 504 | 45.. 584 | 2460 |
| BRAWH3046209 | 505 | 4437..4874 | 2461 |
| BRAWH3046424 | 506 | 1763..3661 | 2462 |
| BRAWH3046802 | 507 | 155.. 478 | 2463 |
| BRAWH3046959 | 508 | 1722..2213 | 2464 |
| BRAWH3047063 | 509 | 94.. 483 | 2465 |
| BRAWH3047539 | 510 | 126.. 455 | 2466 |
| BRAWH3047565 | 511 | 335.. 952 | 2467 |
| BRAWH3047644 | 512 | 1314..4229 | 2468 |
| BRAWH3047692 | 513 | 668..2413 | 2469 |
| BRAWH3047946 | 514 | 115.. 609 | 2470 |
| BRAWH3048374 | 515 | 869..3058 | 2471 |
| BRAWH3048548 | 516 | 124.. 924 | 2472 |
| BRAWH3048724 | 517 | 1708..2232 | 2473 |
| BRAWH3049068 | 518 | 234.. 626 | 2474 |
| BRAWH3049544 | 519 | 102..1913 | 2475 |
| BRAWH3049726 | 520 | 49.. 468 | 2476 |
| BRAWH3049858 | 521 | 161.. 643 | 2477 |
| BRCAN2000923 | 522 | 125.. 685 | 2478 |
| BRCAN2002662 | 523 | 1502..1897 | 2479 |
| BRCAN2002892 | 524 | 289.. 888 | 2480 |
| BRCAN2003269 | 525 | 10.. 945 | 2481 |
| BRCAN2003814 | 526 | 76..1044 | 2482 |
| BRCAN2006051 | 527 | 1.. 570 | 2483 |
| BRCAN2006955 | 528 | 123.. 485 | 2484 |
| BRCAN2007525 | 529 | 188.. 559 | 2485 |
| BRCAN2008701 | 530 | 435.. 944 | 2486 |
| BRCAN2009168 | 531 | 2594..>3177 | 2487 |
| BRCAN2010547 | 532 | 405.. 737 | 2488 |
| BRCAN2010581 | 533 | 1674..2045 | 2489 |
| BRCAN2010665 | 534 | 193..1851 | 2490 |
| BRCAN2015402 | 535 | 1718..2158 | 2491 |
| BRCAN2015757 | 536 | 192..1175 | 2492 |
| BRCAN2018269 | 537 | 131.. 751 | 2493 |
| BRCAN2018667 | 538 | 217.. 582 | 2494 |
| BRCAN2019653 | 539 | 165.. 779 | 2495 |
| BRCAN2019907 | 540 | 88.. 516 | 2496 |
| BRCAN2019953 | 541 | 56.. 589 | 2497 |
| BRCAN2020234 | 542 | 595..1089 | 2498 |
| BRCAN2020331 | 543 | 58..1401 | 2499 |
| BRCAN2020412 | 544 | 989..1609 | 2500 |
| BRCAN2020467 | 545 | 269.. 673 | 2501 |
| BRCAN2020880 | 546 | 1036..1623 | 2502 |
| BRCAN2020972 | 547 | 651..1016 | 2503 |
| BRCAN2021325 | 548 | 163..1428 | 2504 |
| BRCAN2021452 | 549 | 1107..1502 | 2505 |
| BRCAN2021718 | 550 | 876..1691 | 2506 |
| BRCAN2022126 | 551 | 87..3116 | 2507 |
| BRCAN2025093 | 552 | 62.. 862 | 2508 |
| BRCAN2027593 | 553 | 749..1108 | 2509 |
| BRCAN2028702 | 554 | 135..1082 | 2510 |
| BRCOC2001355 | 555 | 67.. 936 | 2511 |
| BRCOC2002777 | 556 | 2206..2814 | 2512 |
| BRCOC2006164 | 557 | 325..2700 | 2513 |
| BRCOC2006639 | 558 | 188..1027 | 2514 |
| BRCOC2006942 | 559 | 116.. 643 | 2515 |
| BRCOC2009638 | 560 | 244.. 726 | 2516 |
| BRCOC2010115 | 561 | 204.. 569 | 2517 |
| BRCOC2012386 | 562 | 224..1543 | 2518 |
| BRHIP2006819 | 563 | 21.. 557 | 2519 |
| BRHIP2006921 | 564 | 80.. 517 | 2520 |
| BRHIP2008756 | 565 | 924..2273 | 2521 |
| BRHIP2009177 | 566 | 101.. 541 | 2522 |
| BRHIP2011199 | 567 | 44.. 694 | 2523 |
| BRHIP2013958 | 568 | 78..3164 | 2524 |
| BRHIP2015153 | 569 | 1959..2474 | 2525 |
| BRHIP2016125 | 570 | 930..1538 | 2526 |
| BRHIP2017714 | 571 | 130.. 438 | 2527 |
| BRHIP2020930 | 572 | 285..>602 | 2528 |
| BRHIP2021929 | 573 | 253..1413 | 2529 |
| BRHIP2023735 | 574 | 865..1191 | 2530 |
| BRHIP2024941 | 575 | 110.. 574 | 2531 |
| BRHIP2026346 | 576 | 10..1791 | 2532 |
| BRHIP2027077 | 577 | 194.. 541 | 2533 |
| BRHIP2027563 | 578 | 14..1234 | 2534 |
| BRHIP2029529 | 579 | 345..1643 | 2535 |
| BRHIP2029643 | 580 | 1799..2104 | 2536 |
| BRHIP2029663 | 581 | 72..1607 | 2537 |
| BRHIP3000626 | 582 | 558..1139 | 2538 |
| BRHIP3000859 | 583 | 30.. 536 | 2539 |
| BRHIP3001076 | 584 | 974..2767 | 2540 |
| BRHIP3001141 | 585 | 402.. 893 | 2541 |
| BRHIP3001338 | 586 | 3819..>4142 | 2542 |
| BRHIP3001360 | 587 | 689..2866 | 2543 |
| BRHIP3001481 | 588 | 775..2172 | 2544 |
| BRHIP3001573 | 589 | 1429..3393 | 2545 |
| BRHIP3001878 | 590 | 3977..4435 | 2546 |
| BRHIP3002000 | 591 | 44..2758 | 2547 |
| BRHIP3002114 | 592 | 2654..3442 | 2548 |
| BRHIP3002124 | 593 | 194.. 790 | 2549 |
| BRHIP3002141 | 594 | 1668..2993 | 2550 |
| BRHIP3002363 | 595 | 1196..1591 | 2551 |
| BRHIP3002691 | 596 | 56.. 757 | 2552 |
| BRHIP3002920 | 597 | 265.. 906 | 2553 |
| BRHIP3002931 | 598 | 2360..2896 | 2554 |
| BRHIP3003063 | 599 | 726..3443 | 2555 |
| BRHIP3003126 | 600 | 1121..2608 | 2556 |
| BRHIP3003306 | 601 | 1972..3834 | 2557 |
| BRHIP3003340 | 602 | 768..1412 | 2558 |
| BRHIP3003395 | 603 | 214.. 540 | 2559 |
| BRHIP3003688 | 604 | 247.. 636 | 2560 |
| BRHIP3003795 | 605 | 1693..2085 | 2561 |
| BRHIP3003845 | 606 | 1649..2635 | 2562 |
| BRHIP3003961 | 607 | 1163..1738 | 2563 |
| BRHIP3003984 | 608 | 987..3938 | 2564 |
| BRHIP3004215 | 609 | 1217..3940 | 2565 |
| BRHIP3004710 | 610 | 889..3069 | 2566 |
| BRHIP3004725 | 611 | 1741..4032 | 2567 |
| BRHIP3004774 | 612 | 23.. 412 | 2568 |
| BRHIP3004786 | 613 | 18.. 434 | 2569 |
| BRHIP3005037 | 614 | 671..2284 | 2570 |
| BRHIP3005142 | 615 | 1051..4995 | 2571 |
| BRHIP3005231 | 616 | 1519..3309 | 2572 |
| BRHIP3005307 | 617 | 2128..3567 | 2573 |
| BRHIP3005673 | 618 | 907..1515 | 2574 |
| BRHIP3005801 | 619 | 483.. 926 | 2575 |
| BRHIP3005944 | 620 | 1826..2575 | 2576 |
| BRHIP3006279 | 621 | 98.. 448 | 2577 |
| BRHIP3006294 | 622 | 455..3478 | 2578 |
| BRHIP3006449 | 623 | 385..2763 | 2579 |
| BRHIP3006786 | 624 | 209..1057 | 2580 |
| BRHIP3006950 | 625 | 139.. 546 | 2581 |
| BRHIP3007172 | 626 | 1426..1815 | 2582 |
| BRHIP3007195 | 627 | 143..3709 | 2583 |
| BRHIP3007223 | 628 | 764..4627 | 2584 |
| BRHIP3007291 | 629 | 115.. 837 | 2585 |
| BRHIP3007409 | 630 | 2136..4439 | 2586 |
| BRHIP3007424 | 631 | 93..1148 | 2587 |
| BRHIP3007609 | 632 | 2063..4171 | 2588 |
| BRHIP3007960 | 633 | 3709..4917 | 2589 |
| BRHIP3008082 | 634 | 254..>3526 | 2590 |
| BRHIP3008320 | 635 | 75..3710 | 2591 |
| BRHIP3008714 | 636 | 2704..3252 | 2592 |
| BRHIP3009672 | 637 | 343.. 705 | 2593 |
| BRHIP3009753 | 638 | 188..1006 | 2594 |
| BRHIP3010289 | 639 | 303..4004 | 2595 |
| BRHIP3010916 | 640 | 602..1054 | 2596 |
| BRHIP3011082 | 641 | 2495..3835 | 2597 |
| BRHIP3011269 | 642 | 2043..3173 | 2598 |
| BRHIP3011460 | 643 | 285..2900 | 2599 |
| BRHIP3011567 | 644 | 1572..2684 | 2600 |
| BRHIP3011831 | 645 | 2815..3318 | 2601 |
| BRHIP3012185 | 646 | 409.. 792 | 2602 |
| BRHIP3012289 | 647 | 2691..3719 | 2603 |
| BRHIP3012357 | 648 | 1095..1799 | 2604 |
| BRHIP3012736 | 649 | 134.. 625 | 2605 |
| BRHIP3012997 | 650 | 243.. 617 | 2606 |
| BRHIP3013078 | 651 | 926..1357 | 2607 |
| BRHIP3013588 | 652 | 2846..3625 | 2608 |
| BRHIP3013698 | 653 | 271.. 801 | 2609 |
| BRHIP3014675 | 654 | 51..1976 | 2610 |
| BRHIP3015854 | 655 | 227.. 616 | 2611 |
| BRHIP3016032 | 656 | 260..1984 | 2612 |
| BRHIP3016421 | 657 | 335..3670 | 2613 |
| BRHIP3017109 | 658 | 185.. 637 | 2614 |
| BRHIP3017146 | 659 | 306.. 929 | 2615 |
| BRHIP3017256 | 660 | 3558..3971 | 2616 |
| BRHIP3017558 | 661 | 393..1853 | 2617 |
| BRHIP3017855 | 662 | 1719..4223 | 2618 |
| BRHIP3018784 | 663 | 37.. 345 | 2619 |
| BRHIP3019643 | 664 | 1575..2339 | 2620 |
| BRHIP3019824 | 665 | 25..4392 | 2621 |
| BRHIP3019880 | 666 | 3145..>3494 | 2622 |
| BRHIP3019956 | 667 | 66.. 386 | 2623 |
| BRHIP3020046 | 668 | 705..1736 | 2624 |
| BRHIP3020155 | 669 | 2852..3238 | 2625 |
| BRHIP3020733 | 670 | 728..1399 | 2626 |
| BRHIP3021019 | 671 | 1266..2867 | 2627 |
| BRHIP3021499 | 672 | 1425..1760 | 2628 |
| BRHIP3021987 | 673 | 30.. 401 | 2629 |
| BRHIP3022656 | 674 | 403.. 735 | 2630 |
| BRHIP3023922 | 675 | 993..1409 | 2631 |
| BRHIP3024703 | 676 | 1929..2840 | 2632 |
| BRHIP3024820 | 677 | 653..1183 | 2633 |
| BRHIP3025795 | 678 | 2311..3708 | 2634 |
| BRHIP3025844 | 679 | 172.. 702 | 2635 |
| BRHIP3026231 | 680 | 50.. 652 | 2636 |
| BRHIP3026651 | 681 | 30.. 341 | 2637 |
| BRHIP3027160 | 682 | 569..1048 | 2638 |
| BRHIP3027191 | 683 | 131..3106 | 2639 |
| BRHIP3027651 | 684 | 90..2582 | 2640 |
| BRHIP3027947 | 685 | 140.. 550 | 2641 |
| BRHIP3028246 | 686 | 408..4205 | 2642 |
| BRHIP3028570 | 687 | 112..4293 | 2643 |
| BRHIP3028742 | 688 | 5.. 694 | 2644 |
| BRHIP3029409 | 689 | 134.. 670 | 2645 |
| BRHIP3029530 | 690 | 187.. 501 | 2646 |
| BRHIP3029670 | 691 | 339.. 812 | 2647 |
| BRHIP3029866 | 692 | 73.. 630 | 2648 |
| BRHIP3030230 | 693 | 159..1457 | 2649 |
| BRHIP3031733 | 694 | 53.. 361 | 2650 |
| BRHIP3031890 | 695 | 2921..3229 | 2651 |
| BRHIP3032148 | 696 | 1862..3133 | 2652 |
| BRHIP3032311 | 697 | 27.. 887 | 2653 |
| BRHIP3032374 | 698 | 2973..4667 | 2654 |
| BRHIP3033481 | 699 | 71.. 484 | 2655 |
| BRHIP3033557 | 700 | 22..2580 | 2656 |
| BRHIP3033734 | 701 | 718..1986 | 2657 |
| BRHIP3033806 | 702 | 1241..2221 | 2658 |
| BRHIP3035006 | 703 | 2734..3606 | 2659 |
| BRHIP3035222 | 704 | 1658..2143 | 2660 |
| BRHIP3035754 | 705 | 301.. 615 | 2661 |
| BRHIP3036371 | 706 | 3591..4817 | 2662 |
| BRHIP3036715 | 707 | 416..1063 | 2663 |
| BRHIP3036936 | 708 | 235..3189 | 2664 |
| BRHIP3037543 | 709 | 960..1286 | 2665 |
| BRHIP3037810 | 710 | 2158..2556 | 2666 |
| BRHIP3038030 | 711 | 1970..3091 | 2667 |
| BRHIP3038735 | 712 | 571..2007 | 2668 |
| BRHIP3039430 | 713 | 190.. 702 | 2669 |
| BRHIP3039509 | 714 | 1145..3061 | 2670 |
| BRHIP3039592 | 715 | 1..1914 | 2671 |
| BRHIP3040878 | 716 | 612..1046 | 2672 |
| BRHIP3041587 | 717 | 54.. 365 | 2673 |
| BRHIP3042817 | 718 | 117.. 473 | 2674 |
| BRHIP3043012 | 719 | 2292..2714 | 2675 |
| BRSSN2004303 | 720 | 193.. 714 | 2676 |
| BRSSN2004710 | 721 | 1102..1491 | 2677 |
| BRSSN2008464 | 722 | 774..1076 | 2678 |
| BRSSN2011843 | 723 | 61.. 825 | 2679 |
| BRSSN2012157 | 724 | 28.. 546 | 2680 |
| BRSSN2012198 | 725 | 1877..2182 | 2681 |
| BRSSN2013696 | 726 | 572..1351 | 2682 |
| BRSSN2015497 | 727 | 251..3196 | 2683 |
| BRSSN2018218 | 728 | 126.. 485 | 2684 |
| BRSTN2000312 | 729 | 270..1166 | 2685 |
| BRSTN2006466 | 730 | 954..>1515 | 2686 ' |
| BRSTN2006638 | 731 | 50..1099 | 2687 |
| BRSTN2008475 | 732 | 2167..2511 | 2688 |
| BRSTN2009247 | 733 | 319..1197 | 2689 |
| BRSTN2010089 | 734 | 103..1464 | 2690 |
| BRSTN2010416 | 735 | 114.. 584 | 2691 |
| BRSTN2011688 | 736 | 288.. 719 | 2692 |
| BRSTN2011899 | 737 | 218.. 553 | 2693 |
| BRSTN2011961 | 738 | 415.. 972 | 2694 |
| BRSTN2012069 | 739 | 64..1452 | 2695 |
| BRSTN2015699 | 740 | 720..1250 | 2696 |
| BRSTN2015788 | 741 | 45.. 401 | 2697 |
| BRSTN2016892 | 742 | 10.. 351 | 2698 |
| BRSTN2016918 | 743 | 9..1304 | 2699 |
| BRSTN2016992 | 744 | 89.. 523 | 2700 |
| BRSTN2017104 | 745 | 237.. 632 | 2701 |
| BRSTN2017151 | 746 | 160.. 486 | 2702 |
| BRSTN2017184 | 747 | 157.. 660 | 2703 |
| BRSTN2018712 | 748 | 226.. 744 | 2704 |
| BRTHA2000969 | 749 | 199.. 528 | 2705 |
| BRTHA2001304 | 750 | 47.. 886 | 2706 |
| BRTHA2001953 | 751 | 175.. 537 | 2707 |
| BRTHA2002091 | 752 | 785..1684 | 2708 |
| BRTHA2003759 | 753 | 209.. 601 | 2709 |
| BRTHA2005448 | 754 | 310..2469 | 2710 |
| BRTHA2006720 | 755 | 278.. 874 | 2711 |
| BRTHA2008502 | 756 | 902..1711 | 2712 |
| BRTHA2008598 | 757 | 653.. 976 | 2713 |
| BRTHA2010672 | 758 | 2012..2770 | 2714 |
| BRTHA2012189 | 759 | 447.. 818 | 2715 |
| BRTHA2014647 | 760 | 1952..2290 | 2716 |
| BRTHA2018304 | 761 | 987..1526 | 2717 |
| BRTHA2019726 | 762 | 81..1511 | 2718 |
| BRTHA2019743 | 763 | 260.. 616 | 2719 |
| BRTHA2020400 | 764 | 38.. 562 | 2720 |
| BRTHA2020566 | 765 | 268.. 615 | 2721 |
| BRTHA2020642 | 766 | 235..1155 | 2722 |
| BRTHA2020695 | 767 | 83.. 466 | 2723 |
| BRTHA2020721 | 768 | 80..1495 | 2724 |
| BRTHA2020781 | 769 | 1138..1563 | 2725 |
| BRTHA2020910 | 770 | 1901..2815 | 2726 |
| BRTHA2021212 | 771 | 209.. 583 | 2727 |
| BRTHA2021440 | 772 | 216.. 911 | 2728 |
| BRTHA2021450 | 773 | 158.. 550 | 2729 |
| BRTHA2022074 | 774 | 248.. 556 | 2730 |
| BRTHA2022914 | 775 | 315.. 704 | 2731 |
| BRTHA2022968 | 776 | 621..1133 | 2732 |
| BRTHA2023402 | 777 | 69.. 578 | 2733 |
| BRTHA2023437 | 778 | 106.. 438 | 2734 |
| BRTHA2024177 | 779 | 448.. 813 | 2735 |
| BRTHA2024354 | 780 | 123..1502 | 2736 |
| BRTHA2024712 | 781 | 2765..3208 | 2737 |
| BRTHA2025869 | 782 | 98..1429 | 2738 |
| BRTHA2026071 | 783 | 1069..1449 | 2739 |
| BRTHA2026290 | 784 | 2508..3953 | 2740 |
| BRTHA2026311 | 785 | 339..1817 | 2741 |
| BRTHA2027227 | 786 | 7.. 885 | 2742 |
| BRTHA2027229 | 787 | 698..1135 | 2743 |
| BRTHA2027250 | 788 | 589..2013 | 2744 |
| BRTHA2028297 | 789 | 2669..3112 | 2745 |
| BRTHA2029969 | 790 | 238.. 543 | 2746 |
| BRTHA2030036 | 791 | 254..2149 | 2747 |
| BRTHA2030213 | 792 | 27.. 389 | 2748 |
| BRTHA2031517 | 793 | 94.. 426 | 2749 |
| BRTHA2031917 | 794 | 1729..2394 | 2750 |
| BRTHA2032763 | 795 | 88.. 453 | 2751 |
| BRTHA2033122 | 796 | 165.. 557 | 2752 |
| BRTHA2033155 | 797 | 198.. 665 | 2753 |
| BRTHA2033320 | 798 | 186.. 590 | 2754 |
| BRTHA2033469 | 799 | 140.. 514 | 2755 |
| BRTHA2033683 | 800 | 301..1146 | 2756 |
| BRTHA2034281 | 801 | 9.. 533 | 2757 |
| BRTHA2034576 | 802 | 148.. 483 | 2758 |
| BRTHA2035743 | 803 | 127..1824 | 2759 |
| BRTHA2036055 | 804 | 2274..3482 | 2760 |
| BRTHA2036295 | 805 | 514..2751 | 2761 |
| BRTHA2037247 | 806 | 2592..3308 | 2762 |
| BRTHA2038279 | 807 | 1530..2024 | 2763 |
| BRTHA2038345 | 808 | 1236..2291 | 2764 |
| BRTHA2038353 | 809 | 397.. 798 | 2765 |
| BRTHA3000456 | 810 | 488..1603 | 2766 |
| BRTHA3002411 | 811 | 557..1996 | 2767 |
| BRTHA3003225 | 812 | 52..1092 | 2768 |
| BRTHA3003417 | 813 | 3420..3806 | 2769 |
| BRTHA3003736 | 814 | 2707..4320 | 2770 |
| BRTHA3005988 | 815 | 220..>3773 | 2771 |
| BRTHA3006593 | 816 | 4.. 516 | 2772 |
| BRTHA3007469 | 817 | 1400..1732 | 2773 |
| BRTHA3007662 | 818 | 2834..3355 | 2774 |
| BRTHA3009858 | 819 | 934..1293 | 2775 |
| BRTHA3010135 | 820 | 121..1947 | 2776 |
| BRTHA3010212 | 821 | 81..1829 | 2777 |
| BRTHA3010469 | 822 | 2653..3081 | 2778 |
| BRTHA3010530 | 823 | 251..1171 | 2779 |
| BRTHA3010540 | 824 | 122.. 574 | 2780 |
| BRTHA3010717 | 825 | 208.. 915 | 2781 |
| BRTHA3011187 | 826 | 126..3383 | 2782 |
| BRTHA3011194 | 827 | 309..>2485 | 2783 |
| BRTHA3011229 | 828 | 205.. 633 | 2784 |
| BRTHA3011265 | 829 | 174.. 569 | 2785 |
| BRTHA3011306 | 830 | 356.. 769 | 2786 |
| BRTHA3011361 | 831 | 1184..2929 | 2787 |
| BRTHA3011510 | 832 | 702..1079 | 2788 |
| BRTHA3011892 | 833 | 416.. 784 | 2789 |
| BRTHA3011998 | 834 | 53.. 388 | 2790 |
| BRTHA3012265 | 835 | 381..1760 | 2791 |
| BRTHA3013860 | 836 | 60.. 527 | 2792 |
| BRTHA3013882 | 837 | 34.. 639 | 2793 |
| BRTHA3014000 | 838 | 116..3646 | 2794 |
| BRTHA3014105 | 839 | 378.. 752 | 2795 |
| BRTHA3014507 | 840 | 1901..2767 | 2796 |
| BRTHA3014547 | 841 | 52..4179 | 2797 |
| BRTHA3014835 | 842 | 300.. 743 | 2798 |
| BRTHA3014854 | 843 | 185.. 664 | 2799 |
| BRTHA3014920 | 844 | 336.. 662 | 2800 |
| BRTHA3016616 | 845 | 293.. 616 | 2801 |
| BRTHA3017791 | 846 | 12..1109 | 2802 |
| BRTHA3018409 | 847 | 13..2601 | 2803 |
| BRTHA3018623 | 848 | 207.. 701 | 2804 |
| BRTHA3019183 | 849 | 2860..3315 | 2805 |
| BRTHA3020369 | 850 | 182.. 574 | 2806 |
| BRTHA3020771 | 851 | 218..1810 | 2807 |
| BRTHA3021569 | 852 | 2914..>3417 | 2808 |
| BRTHA3021708 | 853 | 1925..2560 | 2809 |
| BRTHA3021786 | 854 | 48.. 566 | 2810 |
| BRTHA3021971 | 855 | 180..2975 | 2811 |
| BRTHA3022641 | 856 | 219.. 590 | 2812 |
| BRTHA3023403 | 857 | 1461..3743 | 2813 |
| BRTHA3023590 | 858 | 3479..3856 | 2814 |
| BRTHA3023929 | 859 | 292.. 606 | 2815 |
| BRTHA3024600 | 860 | 2490..3026 | 2816 |
| BRTHA3025073 | 861 | 234..3422 | 2817 |
| BRTHA3026161 | 862 | 290.. 601 | 2818 |
| BRTHA3026180 | 863 | 35.. 475 | 2819 |
| BRTHA3026556 | 864 | 1128..1478 | 2820 |
| BRTHA3026916 | 865 | 84..2825 | 2821 |
| BRTHA3027171 | 866 | 412.. 864 | 2822 |
| BRTHA3027318 | 867 | 166.. 489 | 2823 |
| BRTHA3027638 | 868 | 1777..2181 | 2824 |
| BRTHA3027820 | 869 | 3477..3821 | 2825 |
| BRTHA3027879 | 870 | 123.. 701 | 2826 |
| BRTHA3027957 | 871 | 20..1756 | 2827 |
| BRTHA3028339 | 872 | 69.. 422 | 2828 |
| BRTHA3028505 | 873 | 60.. 527 | 2829 |
| CERVX2000812 | 874 | 186.. 686 | 2830 |
| CERVX2000968 | 875 | 652..1602 | 2831 |
| CHONS2000797 | 876 | 316..1806 | 2832 |
| CHONS2001287 | 877 | 3.. 836 | 2833 |
| CHONS2001797 | 878 | 621..1019 | 2834 |
| CHONS2001834 | 879 | 51..1106 | 2835 |
| CHONS2002419 | 880 | 20..1141 | 2836 |
| CHONS2002829 | 881 | 69..2270 | 2837 |
| COLON2001829 | 882 | 334.. 948 | 2838 |
| COLON2001866 | 883 | 268.. 924 | 2839 |
| COLON2004351 | 884 | 82.. 876 | 2840 |
| COLON2004911 | 885 | 251.. 919 | 2841 |
| COLON2005623 | 886 | 293.. 967 | 2842 |
| COLON2005735 | 887 | 390..1595 | 2843 |
| CTONG2001932 | 888 | 405..2174 | 2844 |
| CTONG2003517 | 889 | 138..1211 | 2845 |
| CTONG2006235 | 890 | 21..1673 | 2846 |
| CTONG2008989 | 891 | 172.. 843 | 2847 |
| CTONG2009033 | 892 | 412.. 720 | 2848 |
| CTONG2009570 | 893 | 1762..3051 | 2849 |
| CTONG2010330 | 894 | 418..1896 | 2850 |
| CTONG2010633 | 895 | 394.. 711 | 2851 |
| CTONG2011801 | 896 | 122..2419 | 2852 |
| CTONG2012123 | 897 | 728..1360 | 2853 |
| CTONG2014206 | 898 | 826..1710 | 2854 |
| CTONG2014959 | 899 | 154..1101 | 2855 |
| CTONG2020582 | 900 | 27..1754 | 2856 |
| CTONG2026987 | 901 | 384..1031 | 2857 |
| CTON62027150 | 902 | 300.. 731 | 2858 |
| CTONG2027591 | 903 | 1451..2728 | 2859 |
| CTONG2027783 | 904 | 300..2561 | 2860 |
| CTONG2027959 | 905 | 65.. 412 | 2861 |
| CTONG3001605 | 906 | 321.. 896 | 2862 |
| CTONG3002518 | 907 | 1943..2269 | 2863 |
| CTONG3002588 | 908 | 107.. 454 | 2864 |
| CTONG3003669 | 909 | 258..2015 | 2865 |
| CTONG3008223 | 910 | 159.. 464 | 2866 |
| D9OST2003106 | 911 | 737..>2505 | 2867 |
| D9OST2003989 | 912 | 239.. 658 | 2868 |
| D9OST2004417 | 913 | 54.. 689 | 2869 |
| DFNES2001829 | 914 | 105.. 758 | 2870 |
| DFNES2011221 | 915 | 196..3138 | 2871 |
| ERLTF2001452 | 916 | 836..1321 | 2872 |
| ERLTF2001835 | 917 | 300..1388 | 2873 |
| ERLTF2002178 | 918 | 127..1683 | 2874 |
| ERLTF2002369 | 919 | 160..1986 | 2875 |
| FCBBF3001018 | 920 | 133..1026 | 2876 |
| FCBBF3002188 | 921 | 300.. 611 | 2877 |
| FCBBF3005160 | 922 | 263.. 658 | 2878 |
| FCBBF3012443 | 923 | 148..1173 | 2879 |
| FCBBF3020030 | 924 | 1..1650 | 2880 |
| FCBBF3021191 | 925 | 179..1768 | 2881 |
| FCBBF3024911 | 926 | 1228..>2188 | 2882 |
| FCBBF5000384 | 927 | 782..2089 | 2883 |
| FEBRA2000805 | 928 | 65.. 568 | 2884 |
| FEBRA2002260 | 929 | 303.. 734 | 2885 |
| FEBRA2012625 | 930 | 393.. 821 | 2886 |
| FEBRA2013069 | 931 | 353.. 667 | 2887 |
| FEBRA2013570 | 932 | 128..1567 | 2888 |
| FEBRA2017736 | 933 | 98.. 406 | 2889 |
| FEBRA2017811 | 934 | 458..1705 | 2890 |
| FEBRA2023498 | 935 | 295.. 708 | 2891 |
| FEBRA2026582 | 936 | 22.. 348 | 2892 |
| FEBRA2026977 | 937 | 1950..2378 | 2893 |
| FEBRA2028222 | 938 | 538..2613 | 2894 |
| FEBRA2028457 | 939 | 149..1960 | 2895 |
| FEHRT2001482 | 940 | 1257..1589 | 2896 |
| FEHRT2002708 | 941 | 332..1078 | 2897 |
| FEKID2001001 | 942 | 206..>2002 | 2898 |
| FEKID2001201 | 943 | 41..>2361 | 2899 |
| FEKID2002231 | 944 | 259.. 672 | 2900 |
| FEKID2002493 | 945 | 54..1043 | 2901 |
| FEKID2002637 | 946 | 50..1291 | 2902 |
| FELNG2000720 | 947 | 120..1037 | 2903 |
| FELNG2001613 | 948 | 194.. 907 | 2904 |
| FELNG2001706 | 949 | 306.. 821 | 2905 |
| FELNG2001953 | 950 | 723..1358 | 2906 |
| HCASM2007773 | 951 | 1930..2421 | 2907 |
| HCASM2008154 | 952 | 38..1852 | 2908 |
| HCHON2009766 | 953 | 264..>2250 | 2909 |
| HEART2002531 | 954 | 342.. 695 | 2910 |
| HHDPC2008185 | 955 | 435..2105 | 2911 |
| HLUNG2012600 | 956 | 30.. 380 | 2912 |
| HSYRA2004550 | 957 | 35..1408 | 2913 |
| HSYRA2007338 | 958 | 91..1491 | 2914 |
| JCMLC1000159 | 959 | 1312..1782 | 2915 |
| JCMLC2000273 | 960 | 522..2198 | 2916 |
| JCMLC2002095 | 961 | 159..1178 | 2917 |
| JCMLC2002751 | 962 | 291..1475 | 2918 |
| KIDNE2004531 | 963 | 315..1430 | 2919 |
| KIDNE2010049 | 964 | 121..1710 | 2920 |
| KIDNE2014496 | 965 | 292.. 948 | 2921 |
| KIDNE2015987 | 966 | 125..1648 | 2922 |
| KIDNE2016464 | 967 | 725..1168 | 2923 |
| KIDNE2017153 | 968 | 415..1122 | 2924 |
| KIDNE2018268 | 969 | 925..2145 | 2925 |
| LIVER2008465 | 970 | 71.. 898 | 2926 |
| LYMPB1000158 | 971 | 87..3215 | 2927 |
| LYMPB2001387 | 972 | 302.. 622 | 2928 |
| LYMPB2002236 | 973 | 91..1584 | 2929 |
| LYMPB2002344 | 974 | 389..1801 | 2930 |
| LYMPB2002458 | 975 | 108..1433 | 2931 |
| LYMPB2002478 | 976 | 172.. 546 | 2932 |
| MESAN2007032 | 977 | 122.. 922 | 2933 |
| MESAN2009156 | 978 | 295..2175 | 2934 |
| MESAN2014624 | 979 | 957..1658 | 2935 |
| MESAN2016304 | 980 | 1045..1353 | 2936 |
| MESAN2017133 | 981 | 35.. 733 | 2937 |
| MESTC2000170 | 982 | 147.. 467 | 2938 |
| N1ESE2000698 | 983 | 129..1844 | 2939 |
| NETRP2000439 | 984 | 70.. 537 | 2940 |
| NETRP2000961 | 985 | 644..1870 | 2941 |
| NETRP2002082 | 986 | 132.. 479 | 2942 |
| NETRP2003103 | 987 | 84.. 566 | 2943 |
| NETRP2003268 | 988 | 8.. 391 | 2944 |
| NETRP2003448 | 989 | 176.. 859 | 2945 |
| NETRP2003539 | 990 | 67.. 372 | 2946 |
| NETRP2004017 | 991 | 679..1584 | 2947 |
| NETRP2004090 | 992 | 68.. 454 | 2948 |
| NETRP2004434 | 993 | 95..1516 | 2949 |
| NETRP2005282 | 994 | 409.. 789 | 2950. |
| NETRP2005849 | 995 | 254.. 580 | 2951 |
| NETRP2005972 | 996 | 1023..1370 | 2952 |
| NETRP2006468 | 997 | 1740..>2944 | 2953 |
| NETRP2007945 | 998 | 225.. 554 | 2954 |
| NETRP2008488 | 999 | 646..1035 | 2955 |
| NETRP2008582 | 1000 | 108.. 524 | 2956 |
| NOVAR2000783 | 1001 | 387..1364 | 2957 |
| NT2NE2011107 | 1002 | 100..1353 | 2958 |
| NT2NE2016041 | 1003 | 594..1358 | 2959 |
| NT2RI2004818 | 1004 | 106..>2988 | 2960 |
| NT2RI2009233 | 1005 | 307..1119 | 2961 |
| NT2RI2010795 | 1006 | 275.. 685 | 2962 |
| NT2RI2012542 | 1007 | 689..1072 | 2963 |
| NT2RI2015533 | 1008 | 1023..2843 | 2964 |
| NT2RI2023671 | 1009 | 1124..1486 | 2965 |
| NT2RI2028537 | 1010 | 3.. 734 | 2966 |
| NT2RI3001573 | 1011 | 947..2095 | 2967 |
| NT2RI3001967 | 1012 | 224..2254 | 2968 |
| NT2RI3005861 | 1013 | 135.. 509 | 2969 |
| NT2RI3005923 | 1014 | 363..4463 | 2970 |
| NT2RI3007095 | 1015 | 115..2856 | 2971 |
| NT2RI3008179 | 1016 | 184..>3991 | 2972 |
| NT2RI3009480 | 1017 | 333..1463 | 2973 |
| NT2RI3009524 | 1018 | 85..3615 | 2974 |
| NT2RP7003439 | 1019 | 63.. 548 | 2975 |
| NT2RP7007387 | 1020 | 12..3557 | 2976 |
| NT2RP7014178 | 1021 | 922..1752 | 2977 |
| NT2RP7014778 | 1022 | 302.. 700 | 2978 |
| NT2RP7016508 | 1023 | 587..1954 | 2979 |
| NT2RP7017139 | 1024 | 50..1780 | 2980 |
| NT2RP7019682 | 1025 | 386..1180 | 2981 |
| NT2RP7020343 | 1026 | 256.. 741 | 2982 |
| NT2RP8000633 | 1027 | 31.. 348 | 2983 |
| NT2RP8001363 | 1028 | 711..2567 | 2984 |
| NT2RP8001407 | 1029 | 162.. 854 | 2985 |
| NT2RP8001584 | 1030 | 711..2912 | 2986 |
| NT2RP8001604 | 1031 | 223..3726 | 2987 |
| NT2RP8001605 | 1032 | 1254..1751 | 2988 |
| NT2RP8003490 | 1033 | 384..1532 | 2989 |
| NT2RP8003657 | 1034 | 325.. 804 | 2990 |
| NT2RP8003787 | 1035 | 118..1647 | 2991 |
| NT2RP8005546 | 1036 | 1104..3023 | 2992 |
| NT2RP8006452 | 1037 | 55..2667 | 2993 |
| NT2RP8006521 | 1038 | 2089..2691 | 2994 |
| NT2RP8007416 | 1039 | 45.. 410 | 2995 |
| NT2RP8007503 | 1040 | 199.. 564 | 2996 |
| NT2RP8007920 | 1041 | 25..3366 | 2997 |
| NT2RP8008057 | 1042 | 103..3591 | 2998 |
| NT2RP8009119 | 1043 | 591..1307 | 2999 |
| NT2RP8009248 | 1044 | 501..1025 | 3000 |
| NTONG2002278 | 1045 | 536.. 886 | 3001 |
| NTONG2003805 | 1046 | 1092..2708 | 3002 |
| NTONG2004829 | 1047 | 756..1412 | 3003 |
| NTONG2008483 | 1048 | 145..2553 | 3004 |
| NTONG2009468 | 1049 | 13.. 717 | 3005 |
| OCBBF2000831 | 1050 | 355..2085 | 3006 |
| OCBBF2003518 | 1051 | 271..2073 | 3007 |
| OCBBF2004478 | 1052 | 238..1593 | 3008 |
| OCBBF2007039 | 1053 | 139..2925 | 3009 |
| OCBBF2009536 | 1054 | 3..1481 | 3010 |
| OCBBF2014745 | 1055 | 6..1388 | 3011 |
| OCBBF2016928 | 1056 | 108..1742 | 3012 |
| OCBBF2018229 | 1057 | 223.. 711 | 3013 |
| OCBBF2018618 | 1058 | 922..1902 | 3014 |
| OCBBF2019761 | 1059 | 332.. 691 | 3015 |
| OCBBF2024589 | 1060 | 103..1821 | 3016 |
| OCBBF2024779 | 1061 | 1734..2081 | 3017 |
| OCBBF2025631 | 1062 | 53.. 868 | 3018 |
| OCBBF2030927 | 1063 | 158..2335 | 3019 |
| OCBBF2036019 | 1064 | 24.. 908 | 3020 |
| OCBBF3000743 | 1065 | 8.. 355 | 3021 |
| OCBBF3000830 | 1066 | 94.. 468 | 3022 |
| OCBBF3001076 | 1067 | 861..3401 | 3023 |
| OCBBF3001202 | 1068 | 826..2655 | 3024 |
| OCBBF3001333 | 1069 | 36..1466 | 3025 |
| OCBBF3001616 | 1070 | 1795..2349 | 3026 |
| OCBBF3003745 | 1071 | 2.. 412 | 3027 |
| OCBBF3004487 | 1072 | 1472..2344 | 3028 |
| OCBBF3004908 | 1073 | 134.. 532 | 3029 |
| OCBBF3005330 | 1074 | 1168..>4215 | 3030 |
| OCBBF3005843 | 1075 | 241.. 750 | 3031. |
| OCBBF3006986 | 1076 | 72..4016 | 3032 |
| OCBBF3007078 | 1077 | 1883..2401 | 3033 |
| OCBBF3007704 | 1078 | 2601..2999 | 3034 |
| OCBBF3008392 | 1079 | 83..1657 | 3035 |
| OCBBF3008835 | 1080 | 58..2874 | 3036 |
| OCBBF3009244 | 1081 | 89.. 988 | 3037 |
| OCBBF3019269 | 1082 | 763..1359 | 3038 |
| OCBBF3020263 | 1083 | 353..1633 | 3039 |
| OCBBF3020414 | 1084 | 20..1339 | 3040 |
| OCBBF3021086 | 1085 | 137..>3623 | 3041 |
| OCBBF3021166 | 1086 | 2350..2985 | 3042 |
| OCBBF3021361 | 1087 | 783..>3838 | 3043 |
| OCBBF3021502 | 1088 | 1345..1851 | 3044 |
| OCBBF3021515 | 1089 | 243.. 641 | 3045 |
| OCBBF3022123 | 1090 | 276.. 677 | 3046 |
| OCBBF3022166 | 1091 | 1848..2273 | 3047 |
| OCBBF3022576 | 1092 | 1966..2424 | 3048 |
| OCBBF3022827 | 1093 | 108..2234 | 3049 |
| OCBBF3023175 | 1094 | 50..4726 | 3050 |
| OCBBF3023543 | 1095 | 122..3793 | 3051 |
| OCBBF3023913 | 1096 | 230..3364 | 3052 |
| OCBBF3023993 | 1097 | 74.. 379 | 3053 |
| OCBBF3025127 | 1098 | 1867..2325 | 3054 |
| OCBBF3025131 | 1099 | 585.. 944 | 3055 |
| OCBBF3025475 | 1100 | 37..1692 | 3056 |
| OCBBF3025503 | 1101 | 425..1837 | 3057 |
| OCBBF3025630 | 1102 | 2380..3138 | 3058 |
| OCBBF3025887 | 1103 | 264.. 653 | 3059 |
| OCBBF3025901 | 1104 | 273.. 605 | 3060 |
| OCBBF3026088 | 1105 | 3651..4004 | 3061 |
| OCBBF3026361 | 1106 | 286..>3475 | 3062 |
| OCBBF3026979 | 1107 | 154..2670 | 3063 |
| OCBBF3027969 | 1108 | 623..3430 | 3064 |
| OCBBF3028001 | 1109 | 10..1581 | 3065 |
| PEBLM2001803 | 1110 | 389..1747 | 3066 |
| PEBLM2003935 | 1111 | 78.. 998 | 3067 |
| PEBLM2005615 | 1112 | 64..1200 | 3068 |
| PEBLM2006298 | 1113 | 208..>2264 | 3069 |
| PERIC2003349 | 1114 | 340..2499 | 3070 |
| PLACE5000492 | 1115 | 653..2305 | 3071 |
| PLACE5000522 | 1116 | 245.. 688 | 3072 |
| PLACE5000527 | 1117 | 2193..2498 | 3073 |
| PLACE6000012 | 1118 | 111.. 527 | 3074 |
| PLACE6000055 | 1119 | 76..>1552 | 3075 |
| PLACE6001933 | 1120 | 875..1192 | 3076 |
| PLACE6003004 | 1121 | 39..1463 | 3077 |
| PLACE6008315 | 1122 | 161.. 691 | 3078 |
| PLACE6010925 | 1123 | 828..2033 | 3079 |
| PLACE6010936 | 1124 | 494..1585 | 3080 |
| PLACE6016030 | 1125 | 224.. 679 | 3081 |
| PLACE6019542 | 1126 | 257.. 559 | 3082 |
| PLACE6019600 | 1127 | 307.. 633 | 3083 |
| PLACE6019674 | 1128 | 333.. 956 | 3084 |
| PLACE7000266 | 1129 | 153..3767 | 3085 |
| PLACE7000707 | 1130 | 728..1117 | 3086 |
| PLACE7001759 | 1131 | 227..1069 | 3087 |
| PLACE7002303 | 1132 | 1662..2615 | 3088 |
| PLACE7003639 | 1133 | 1309..1881 | 3089 |
| PLACE7003684 | 1134 | 16.. 579 | 3090 |
| PLACE7003985 | 1135 | 3721..4707 | 3091 |
| PLACE7004103 | 1136 | 284..2827 | 3092 |
| PLACE7004961 | 1137 | 173.. 835 | 3093 |
| PLACE7005169 | 1138 | 15..>4050 | 3094 |
| PLACE7005671 | 1139 | 796..1185 | 3095 |
| PLACE7005840 | 1140 | 912..1418 | 3096 |
| PLACE7006090 | 1141 | 3573..3947 | 3097 |
| PLACE7006240 | 1142 | 383.. 784 | 3098 |
| PLACE7006268 | 1143 | 1746..2198 | 3099 |
| PLACE7006498 | 1144 | 330..1298 | 3100 |
| PLACE7006540 | 1145 | 204.. 536 | 3101 |
| PLACE7007379 | 1146 | 2794..3576 | 3102 |
| PLACE7007973 | 1147 | 227..1678 | 3103 |
| PLACE7008136 | 1148 | 277.. 666 | 3104 |
| PLACE7008766 | 1149 | 296.. 772 | 3105 |
| PLACE7009563 | 1150 | 1696..2880 | 3106 |
| PLACE7009757 | 1151 | 279..1409 | 3107 |
| PLACE7009936 | 1152 | 33..1223 | 3108 |
| PLACE7010567 | 1153 | 3473..3841 | 3109 |
| PLACE7011269 | 1154 | 70.. 678 | 3110 |
| PLACE7011559 | 1155 | 1965..2978 | 3111 |
| PLACE7012111 | 1156 | 283..2532 | 3112 |
| PLACE7012127 | 1157 | 2243..2848 | 3113 |
| PLACE7013060 | 1158 | 189.. 539 | 3114 |
| PLACE7014247 | 1159 | 3149..3544 | 3115 |
| PLACE7014396 | 1160 | 126.. 716 | 3116 |
| PLACE7015238 | 1161 | 82.. 408 | 3117 |
| PLACE7015647 | 1162 | 1172..1882 | 3118 |
| PLACE7016214 | 1163 | 1250..1696 | 3119 |
| PLACE7016321 | 1164 | 203.. 622 | 3120 |
| PLACE7016454 | 1165 | 761..1375 | 3121 |
| PLACE7016526 | 1166 | 15..2786 | 3122 |
| PLACE7018304 | 1167 | 148..4749 | 3123 |
| PLACE7018349 | 1168 | 93.. 875 | 3124 |
| PLACE7018452 | 1169 | 9.. 533 | 3125 |
| PLACE7018479 | 1170 | 90..3317 | 3126 |
| PLACE7018512 | 1171 | 471..3329 | 3127 |
| PROST2002078 | 1172 | 269.. 898 | 3128 |
| PROST2007444 | 1173 | 2286..3500 | 3129 |
| PROST2016566 | 1174 | 1009..1482 | 3130 |
| PROST2017578 | 1175 | 81.. 386 | 3131 |
| PROST2017729 | 1176 | 1759..2088 | 3132 |
| PROST2017749 | 1177 | 1422..1799 | 3133 |
| PROST2017910 | 1178 | 269..2098 | 3134 |
| PUAEN2000594 | 1179 | 1864..2304 | 3135 |
| PUAEN2000684 | 1180 | 180..2591 | 3136 |
| PUAEN2006639 | 1181 | 637..1746 | 3137 |
| RECTM2001519 | 1182 | 58.. 402 | 3138 |
| SKMUS2008585 | 1183 | 444..1319 | 3139 |
| SKMUS2009479 | 1184 | 324.. 647 | 3140 |
| SKMUS2009557 | 1185 | 323.. 661 | 3141 |
| SKNMC2003639 | 1186 | 207.. 671 | 3142 |
| SKNSH2007306 | 1187 | 164..1570 | 3143 |
| SMINT2003641 | 1188 | 87.. 578 | 3144 |
| SMINT2009292 | 1189 | 72..2024 | 3145 |
| SMINT2009895 | 1190 | 914..1420 | 3146 |
| SMINT2010753 | 1191 | 73..1950 | 3147 |
| SMINT2011406 | 1192 | 932..1501 | 3148 |
| SMINT2011509 | 1193 | 692..1495 | 3149 |
| SMINT2012040 | 1194 | 1068..1541 | 3150 |
| SMINT2012179 | 1195 | 1021..1833 | 3151 |
| SMINT2014166 | 1196 | 1300..1740 | 3152 |
| SMINT2014721 | 1197 | 202..2124 | 3153 |
| SMINT2017964 | 1198 | 328..1419 | 3154 |
| SMINT2019105 | 1199 | 179.. 733 | 3155 |
| SPLEN2001227 | 1200 | 278.. 649 | 3156 |
| SPLEN2007689 | 1201 | 263.. 931 | 3157 |
| SPLEN2011252 | 1202 | 750..1199 | 3158 |
| SPLEN2012571 | 1203 | 121..1770 | 3159 |
| SPLEN2017999 | 1204 | 2270..2677 | 3160 |
| SPLEN2019092 | 1205 | 505..1098 | 3161 |
| SPLEN2019480 | 1206 | 1113..1487 | 3162 |
| SPLEN2021231 | 1207 | 15.. 578 | 3163 |
| SPLEN2021991 | 1208 | 332.. 715 | 3164 |
| SPLEN2022785 | 1209 | 104.. 511 | 3165 |
| SPLEN2022920 | 1210 | 660..1001 | 3166 |
| SPLEN2024571 | 1211 | 1.. 690 | 3167 |
| SPLEN2025012 | 1212 | 18..1520 | 3168 |
| SPLEN2027852 | 1213 | 129.. 482 | 3169 |
| SPLEN2028417 | 1214 | 348..1814 | 3170 |
| SPLEN2028593 | 1215 | 323.. 655 | 3171 |
| SPLEN2031004 | 1216 | 292.. 876 | 3172 |
| SPLEN2032677 | 1217 | 46..1452 | 3173 |
| SPLEN2033996 | 1218 | 2306..3064 | 3174 |
| SPLEN2034551 | 1219 | 669..1649 | 3175 |
| SPLEN2034601 | 1220 | 6.. 317 | 3176 |
| SPLEN2034934 | 1221 | 331.. 891 | 3177 |
| SPLEN2035615 | 1222 | 170.. 475 | 3178 |
| SPLEN2036608 | 1223 | 153..1118 | 3179 |
| SPLEN2037077 | 1224 | 243.. 545 | 3180 |
| SPLEN2042051 | 1225 | 343.. 807 | 3181 |
| STOMA2003894 | 1226 | 311.. 862 | 3182 |
| STOMA2004663 | 1227 | 106.. 516 | 3183 |
| SYNOV2003326 | 1228 | 191.. 595 | 3184 |
| SYNOV2017179 | 1229 | 21..1568 | 3185 |
| SYNOV3000345 | 1230 | 148..1356 | 3186 |
| SYNOV4000598 | 1231 | 457..2010 | 3187 |
| SYNOV4003174 | 1232 | 273..2282 | 3188 |
| SYNOV4004210 | 1233 | 104..2215 | 3189 |
| SYNOV4009139 | 1234 | 2294..3715 | 3190 |
| SYNOV4009575 | 1235. | 262..2406 | 3191 |
| T1ESE2000609 | 1236 | 58..1422 | 3192 |
| T1ESE2000904 | 1237 | 265..2259 | 3193 |
| T1ESE2002665 | 1238 | 66..2210 | 3194 |
| TBAES2003917 | 1239 | 67..2532 | 3195 |
| TBAES2005361 | 1240 | 217.. 582 | 3196 |
| TBAES2007428 | 1241 | 22..1569 | 3197 |
| TBAES2007548 | 1242 | 1037..1381 | 3198 |
| TBAES2007862 | 1243 | 195.. 641 | 3199 |
| TESOP2002005 | 1244 | 315.. 665 | 3200 |
| TESOP2003308 | 1245 | 1105..1536 | 3201 |
| TESOP2004110 | 1246 | 1562..2053 | 3202 |
| TESOP2008556 | 1247 | 344.. 685 | 3203 |
| TESTI1000459 | 1248 | 60..2720 | 3204 |
| TESTI2001364 | 1249 | 119.. 850 | 3205 |
| TESTI2001915 | 1250 | 199..1440 | 3206 |
| TESTI2003768 | 1251 | 621.. 932 | 3207 |
| TESTI2004452 | 1252 | 252.. 770 | 3208 |
| TESTI2004601 | 1253 | 265..1644 | 3209 |
| TESTI2004971 | 1254 | 1095..1637 | 3210 |
| TESTI2005112 | 1255 | 958..1689 | 3211 |
| TESTI2005153 | 1256 | 49..1011 | 3212 |
| TESTI2005564 | 1257 | 149..1867 | 3213 |
| TESTI2006543 | 1258 | 653..1057 | 3214 |
| TESTI2007490 | 1259 | 506..2131 | 3215 |
| TESTI2008636 | 1260 | 136.. 618 | 3216 |
| TESTI2009497 | 1261 | 233..2119 | 3217 |
| TESTI2009739 | 1262 | 172..>1871 | 3218 |
| TESTI2011020 | 1263 | 986..1432 | 3219 |
| TESTI2011033 | 1264 | 265.. 567 | 3220 |
| TESTI2018335 | 1265 | 413..1825 | 3221 |
| TESTI2018687 | 1266 | 1233..2936 | 3222 |
| TESTI2018867 | 1267 | 53..2755 | 3223 |
| TESTI2021112 | 1268 | 310.. 732 | 3224 |
| TESTI2021654 | 1269 | 212..1123 | 3225 |
| TESTI2022323 | 1270 | 710..1660 | 3226 |
| TESTI2023053 | 1271 | 333.. 647 | 3227 |
| TESTI2023903 | 1272 | 7..1899 | 3228 |
| TESTI2024267 | 1273 | 177..1673 | 3229 |
| TESTI2026024 | 1274 | 321..1124 | 3230 |
| TESTI2026284 | 1275 | 867..1322 | 3231 |
| TESTI2028613 | 1276 | 683..1114 | 3232 |
| TESTI2030519 | 1277 | 16..1683 | 3233 |
| TESTI2030901 | 1278 | 1407..1751 | 3234 |
| TESTI2033905 | 1279 | 60..2384 | 3235 |
| TESTI2034913 | 1280 | 733..1326 | 3236 |
| TESTI2035962 | 1281 | 774..1466 | 3237 |
| TESTI2036285 | 1282 | 398.. 958 | 3238 |
| TESTI2036822 | 1283 | 263..1474 | 3239 |
| TESTI2037085 | 1284 | 325.. 771 | 3240 |
| TESTI2037209 | 1285 | 266..1063 | 3241 |
| TESTI2037572 | 1286 | 285.. 983 | 3242 |
| TESTI2037657 | 1287 | 73..>1778 | 3243 |
| TESTI2037877 | 1288 | 352..1500 | 3244 |
| TESTI2038733 | 1289 | 945..1352 | 3245 |
| TESTI2039342 | 1290 | 26.. 433 | 3246 |
| TESTI2039732 | 1291 | 307.. 999 | 3247 |
| TESTI2039738 | 1292 | 346.. 714 | 3248 |
| TESTI2040372 | 1293 | 322.. 981 | 3249 |
| TESTI2040377 | 1294 | 41..1942 | 3250 |
| TESTI2041362 | 1295 | 1041..1484 | 3251 |
| TESTI2041976 | 1296 | 849..1208 | 3252 |
| TESTI2046188 | 1297 | 42.. 569 | 3253 |
| TESTI2049041 | 1298 | 175..1302 | 3254 |
| TESTI2049062 | 1299 | 72.. 707 | 3255 |
| TESTI2051742 | 1300 | 206..1120 | 3256 |
| TESTI2052110 | 1301 | 446..1354 | 3257 |
| TESTI2052202 | 1302 | 273.. 602 | 3258 |
| TESTI2052670 | 1303 | 172..1959 | 3259 |
| TESTI2052799 | 1304 | 526..1260 | 3260 |
| TESTI4000370 | 1305 | 994..3684 | 3261 |
| TESTI4000534 | 1306 | 5562..5957 | 3262 |
| TESTI4000600 | 1307 | 1693..2196 | 3263 |
| TESTI4000621 | 1308 | 386..>7666 | 3264 |
| TESTI4000703 | 1309 | 251..4408 | 3265 |
| TESTI4000957 | 1310 | 2650..3012 | 3266 |
| TESTI4001037 | 1311 | 1856..2248 | 3267 |
| TESTI4001348 | 1312 | 1443..3488 | 3268 |
| TESTI4001517 | 1313 | 800..1201 | 3269 |
| TESTI4001569 | 1314 | 205..3540 | 3270 |
| TESTI4001679 | 1315 | 1512..2084 | 3271 |
| TESTI4002003 | 1316 | 2189..2623 | 3272 |
| TESTI4002072 | 1317 | 246.. 983 | 3273 |
| TESTI4002141 | 1318 | 1400..3520 | 3274 |
| TESTI4002195 | 1319 | 599.. 1111 | 3275 |
| TESTI4002520 | 1320 | 484.. 921 | 3276 |
| TESTI4002774 | 1321 | 447..2672 | 3277 |
| TESTI4002799 | 1322 | 581..3988 | 3278 |
| TESTI4002868 | 1323 | 229..4074 | 3279 |
| TESTI4002889 | 1324 | 68.. 457 | 3280 |
| TESTI4003179 | 1325 | 18.. 335 | 3281 |
| TESTI4003279 | 1326 | 360.. 956 | 3282 |
| TESTI4003319 | 1327 | 187.. 495 | 3283 |
| TESTI4003404 | 1328 | 1290..2780 | 3284 |
| TESTI4003565 | 1329 | 283..5193 | 3285 |
| TESTI4003574 | 1330 | 312.. 638 | 3286 |
| TESTI4003579 | 1331 | 120..2084 | 3287 |
| TESTI4003602 | 1332 | 2867..3784 | 3288 |
| TESTI4003703 | 1333 | 68..3613 | 3289 |
| TESTI4003733 | 1334 | 2180..2773 | 3290 |
| TESTI4003796 | 1335 | 1947..2933 | 3291 |
| TESTI4003944 | 1336 | 779..4684 | 3292 |
| TESTI4004031 | 1337 | 267..3998 | 3293 |
| TESTI4004210 | 1338 | 219.. 599 | 3294 |
| TESTI4004539 | 1339 | 591..1664 | 3295 |
| TESTI4004653 | 1340 | 588..2351 | 3296 |
| TESTI4004695 | 1341 | 1400..4468 | 3297 |
| TESTI4004917 | 1342 | 2286..4751 | 3298 |
| TESTI4005013 | 1343 | 9..1967 | 3299 |
| TESTI4005322 | 1344 | 113..1600 | 3300 |
| TESTI4005399 | 1345 | 289..1830 | 3301 |
| TESTI4005470 | 1346 | 194..2185 | 3302 |
| TESTI4005653 | 1347 | 65..>4403 | 3303 |
| TESTI4006441 | 1348 | 324.. 752 | 3304 |
| TESTI4006539 | 1349 | 1689..2123 | 3305 |
| TESTI4007565 | 1350 | 1253..1675 | 3306 |
| TESTI4007671 | 1351 | 70..1800 | 3307 |
| TESTI4007965 | 1352 | 801..3338 | 3308 |
| TESTI4008086 | 1353 | 24.. 329 | 3309 |
| TESTI4008305 | 1354 | 1977..2687 | 3310 |
| TESTI4009501 | 1355 | 53.. 730 | 3311 |
| TESTI4010544 | 1356 | 254..3787 | 3312 |
| TESTI4010721 | 1357 | 380..1459 | 3313 |
| TESTI4010902 | 1358 | 1372..3834 | 3314 |
| TESTI4010979 | 1359 | 69.. 488 | 3315 |
| TESTI4011616 | 1360 | 1096..2172 | 3316 |
| TESTI4011744 | 1361 | 270..1058 | 3317 |
| TESTI4011926 | 1362 | 2465..2962 | 3318 |
| TESTI4012258 | 1363 | 419.. 847 | 3319 |
| TESTI4012382 | 1364 | 34.. 360 | 3320 |
| TESTI4012623 | 1365 | 1027..1653 | 3321 |
| TESTI4012956 | 1366 | 185.. 544 | 3322 |
| TESTI4012960 | 1367 | 1588..>4231 | 3323 |
| TESTI4013474 | 1368 | 69..3125 | 3324 |
| TESTI4013742 | 1369 | 47..>3602 | 3325 |
| TESTI4013774 | 1370 | 1239..2138 | 3326 |
| TESTI4013960 | 1371 | 56.. 451 | 3327 |
| TESTI4013962 | 1372 | 1170..1946 | 3328 |
| TESTI4014262 | 1373 | 189.. 623 | 3329 |
| TESTI4014415 | 1374 | 424..3798 | 3330 |
| TESTI4014891 | 1375 | 318..1235 | 3331 |
| TESTI4014908 | 1376 | 1459..4134 | 3332 |
| TESTI4014932 | 1377 | 47..4318 | 3333 |
| TESTI4014977 | 1378 | 240.. 659 | 3334 |
| TESTI4015129 | 1379 | 2023..2352 | 3335 |
| TESTI4015339 | 1380 | 2521..2853 | 3336 |
| TESTI4016848 | 1381 | 32.. 679 | 3337 |
| TESTI4017229 | 1382 | 8.. 352 | 3338 |
| TESTI4017382 | 1383 | 2725..3801 | 3339 |
| TESTI4017647 | 1384 | 861..1187 | 3340 |
| TESTI4017854 | 1385 | 1918..2277 | 3341 |
| TESTI4018436 | 1386 | 2379..2828 | 3342 |
| TESTI4018506 | 1387 | 930..3350 | 3343 |
| TESTI4019149 | 1388 | 69.. 374 | 3344 |
| TESTI4020342 | 1389 | 116..1918 | 3345 |
| TESTI4020596 | 1390 | 187..2229 | 3346 |
| TESTI4020819 | 1391 | 3802..4266 | 3347 |
| TESTI4021129 | 1392 | 109..3558 | 3348 |
| TESTI4021197 | 1393 | 210..3806 | 3349 |
| TESTI4021377 | 1394 | 86.. 754 | 3350 |
| TESTI4021569 | 1395 | 94..1497 | 3351 |
| TESTI4021713 | 1396 | 297..1172 | 3352 |
| TESTI4021821 | 1397 | 274.. 606 | 3353 |
| TESTI4022158 | 1398 | 452..3289 | 3354 |
| TESTI4023096 | 1399 | 237..3125 | 3355 |
| TESTI4023172 | 1400 | 607..1740 | 3356 |
| TESTI4023654 | 1401 | 335.. 778 | 3357 |
| TESTI4024240 | 1402 | 2136..2660 | 3358 |
| TESTI4024245 | 1403 | 96..2003 | 3359 |
| TESTI4024294 | 1404 | 95..1024 | 3360 |
| TESTI4024494 | 1405 | 162..1736 | 3361 |
| TESTI4025062 | 1406 | 1503..2345 | 3362 |
| TESTI4025401 | 1407 | 322.. 633 | 3363 |
| TESTI4025908 | 1408 | 1629..2258 | 3364 |
| TESTI4026080 | 1409 | 106..3897 | 3365 |
| TESTI4026680 | 1410 | 321..1382 | 3366 |
| TESTI4027139 | 1411 | 1.. 537 | 3367 |
| TESTI4027170 | 1412 | 77.. 466 | 3368 |
| TESTI4027262 | 1413 | 144.. 836 | 3369 |
| TESTI4027660 | 1414 | 335..1504 | 3370 |
| TESTI4028042 | 1415 | 226..>4276 | 3371 |
| TESTI4028182 | 1416 | 399..2429 | 3372 |
| TESTI4029023 | 1417 | 2005..2856 | 3373 |
| TESTI4029297 | 1418 | 262..3138 | 3374 |
| TESTI4029651 | 1419 | 79..1287 | 3375 |
| TESTI4029676 | 1420 | 271.. 651 | 3376 |
| TESTI4029731 | 1421 | 894..2738 | 3377 |
| TESTI4029743 | 1422 | 137..2536 | 3378 |
| TESTI4030319 | 1423 | 372..1022 | 3379 |
| TESTI4030673 | 1424 | 1273..1584 | 3380 |
| TESTI4030864 | 1425 | 5420..6574 | 3381 |
| TESTI4031066 | 1426 | 52..2520 | 3382 |
| TESTI4031173 | 1427 | 170..2068 | 3383 |
| TESTI4031818 | 1428 | 107.. 430 | 3384 |
| TESTI4032128 | 1429 | 354..2324 | 3385 |
| TESTI4032270 | 1430 | 376.. 720 | 3386 |
| TESTI4032375 | 1431 | 489.. 800 | 3387 |
| TESTI4032834 | 1432 | 1268..>3008 | 3388 |
| TESTI4032856 | 1433 | 126.. 428 | 3389 |
| TESTI4032913 | 1434 | 326..2587 | 3390 |
| TESTI4033177 | 1435 | 1606..3528 | 3391 |
| TESTI4034633 | 1436 | 293.. 643 | 3392 |
| TESTI4034973 | 1437 | 195.. 572 | 3393 |
| TESTI4035770 | 1438 | 1828..2241 | 3394 |
| TESTI4035872 | 1439 | 258.. 599 | 3395 |
| TESTI4035898 | 1440 | 141.. 737 | 3396 |
| TESTI4035989 | 1441 | 590..1066 | 3397 |
| TESTI4036012 | 1442 | 2397..2801 | 3398 |
| TESTI4036048 | 1443 | 23..1696 | 3399 |
| TESTI4037228 | 1444 | 906..2576 | 3400 |
| TESTI4037949 | 1445 | 386..2248 | 3401 |
| TESTI4038047 | 1446 | 177.. 590 | 3402 |
| TESTI4038758 | 1447 | 279.. 785 | 3403 |
| TESTI4039451 | 1448 | 292..3714 | 3404 |
| TESTI4039575 | 1449 | 61.. 441 | 3405 |
| TESTI4039744 | 1450 | 88..1245 | 3406 |
| TESTI4039904 | 1451 | 350..1588 | 3407 |
| TESTI4040197 | 1452 | 40.. 624 | 3408 |
| TESTI4040559 | 1453 | 225..2219 | 3409 |
| TESTI4040598 | 1454 | 277..1899 | 3410 |
| TESTI4040804 | 1455 | 1878..2429 | 3411 |
| TESTI4041049 | 1456 | 3669..4301 | 3412 |
| TESTI4041482 | 1457 | 141..2540 | 3413 |
| TESTI4041832 | 1458 | 1266..2633 | 3414 |
| TESTI4041984 | 1459 | 339..2873 | 3415 |
| TESTI4042420 | 1460 | 315.. 674 | 3416 |
| TESTI4042846 | 1461 | 160.. 477 | 3417 |
| TESTI4043067 | 1462 | 48..4778 | 3418 |
| TESTI4043166 | 1463 | 215..3145 | 3419 |
| TESTI4043223 | 1464 | 104.. 553 | 3420 |
| TESTI4043371 | 1465 | 127.. 612 | 3421 |
| TESTI4043378 | 1466 | 286.. 687 | 3422 |
| TESTI4044076 | 1467 | 1421..1996 | 3423 |
| TESTI4044291 | 1468 | 98.. 958 | 3424 |
| TESTI4044770 | 1469 | 1000..1689 | 3425 |
| TESTI4045168 | 1470 | 363.. 674 | 3426 |
| TESTI4045330 | 1471 | 13.. 561 | 3427 |
| TESTI4045470 | 1472 | 772..1218 | 3428 |
| TESTI4046073 | 1473 | 346..1989 | 3429 |
| TESTI4046090 | 1474 | 1776..2138 | 3430 |
| TESTI4046245 | 1475 | 321..3464 | 3431 |
| TESTI4046328 | 1476 | 144.. 521 | 3432 |
| TESTI4046450 | 1477 | 173.. 544 | 3433 |
| TESTI4046873 | 1478 | 347..2647 | 3434 |
| TESTI4046962 | 1479 | 18.. 887 | 3435 |
| TESTI4047119 | 1480 | 309..2705 | 3436 |
| TESTI4047305 | 1481 | 2524..3351 | 3437 |
| TESTI4047328 | 1482 | 285..4811 | 3438 |
| TESTI4047437 | 1483 | 144..1694 | 3439 |
| TESTI4047569 | 1484 | 1785..2396 | 3440 |
| TESTI4047746 | 1485 | 406.. 744 | 3441 |
| TESTI4047808 | 1486 | 4..>3552 | 3442 |
| TESTI4048232 | 1487 | 926..1276 | 3443 |
| TESTI4048296 | 1488 | 137.. 505 | 3444 |
| TESTI4048545 | 1489 | 951..3350 | 3445 |
| TESTI4048619 | 1490 | 2964..4142 | 3446 |
| TESTI4049110 | 1491 | 62.. 373 | 3447 |
| TESTI4049552 | 1492 | 307.. 624 | 3448 |
| TESTI4049562 | 1493 | 257.. 697 | 3449 |
| TESTI4049677 | 1494 | 313..1092 | 3450 |
| TESTI4049786 | 1495 | 390..3248 | 3451 |
| TESTI4049863 | 1496 | 2520..2981 | 3452 |
| TESTI4049899 | 1497 | 1599..2156 | 3453 |
| TESTI4050293 | 1498 | 2539..4578 | 3454 |
| TESTI4050954 | 1499 | 219.. 581 | 3455 |
| TESTI4051015 | 1500 | 335.. 787 | 3456 |
| TESTI4051054 | 1501 | 149.. 721 | 3457 |
| TESTI4051388 | 1502 | 364..1224 | 3458 |
| TESTI4051424 | 1503 | 19..3537 | 3459 |
| TESTI4051504 | 1504 | 1199..1702 | 3460 |
| TESTI4051747 | 1505 | 156.. 821 | 3461 |
| TESTI4051858 | 1506 | 151.. 612 | 3462 |
| TESTI4051865 | 1507 | 157..2943 | 3463 |
| TESTI4051952 | 1508 | 3884..>4234 | 3464 |
| TESTI4052132 | 1509 | 253.. 861 | 3465 |
| TESTI4052217 | 1510 | 1058..1606 | 3466 |
| TESTI4052219 | 1511 | 324..2528 | 3467 |
| TESTI4052430 | 1512 | 88..1038 | 3468 |
| TESTI4052598 | 1513 | 129.. 827 | 3469 |
| TESTI4052775 | 1514 | 187..1566 | 3470 |
| THYMU2008207 | 1515 | 735..1469 | 3471 |
| THYMU2038199 | 1516 | 12.. 422 | 3472 |
| THYMU3000390 | 1517 | 3.. 587 | 3473 |
| THYMU3000776 | 1518 | 2139..2495 | 3474 |
| THYMU3001082 | 1519 | 563.. 967 | 3475 |
| THYMU3001593 | 1520 | 1895..2224 | 3476 |
| THYMU3001776 | 1521 | 603..1142 | 3477 |
| THYMU3002825 | 1522 | 2232..3350 | 3478 |
| THYMU3002887 | 1523 | 1159..2085 | 3479 |
| THYMU3003007 | 1524 | 3153..3662 | 3480 |
| THYMU3003350 | 1525 | 1815..2138 | 3481 |
| THYMU3003958 | 1526 | 1970..2416 | 3482 |
| THYMU3004628 | 1527 | 221.. 562 | 3483 |
| THYMU3004632 | 1528 | 303.. 899 | 3484 |
| THYMU3007308 | 1529 | 793..1413 | 3485 |
| THYMU3007559 | 1530 | 1215..1532 | 3486 |
| THYMU3008105 | 1531 | 1829..3454 | 3487 |
| THYMU3008935 | 1532 | 274.. 645 | 3488 |
| THYMU3009643 | 1533 | 583.. 948 | 3489 |
| THYMU3009755 | 1534 | 147.. 512 | 3490 |
| THYMU3011012 | 1535 | 763..1230 | 3491 |
| THYMU3011244 | 1536 | 44.. 352 | 3492 |
| THYMU3011360 | 1537 | 168.. 572 | 3493 |
| THYMU3011534 | 1538 | 318..3047 | 3494 |
| THYMU3011556 | 1539 | 65.. 430 | 3495 |
| THYMU3011717 | 1540 | 39.. 386 | 3496 |
| THYMU3012402 | 1541 | 1422..2435 | 3497 |
| THYMU3012907 | 1542 | 304.. 669 | 3498 |
| THYMU3012983 | 1543 | 4..1542 | 3499 |
| THYMU3013114 | 1544 | 1724..2092 | 3500 |
| THYMU3013197 | 1545 | 64.. 486 | 3501 |
| THYMU3013241 | 1546 | 294..1394 | 3502 |
| THYMU3013470 | 1547 | 212.. 736 | 3503 |
| THYMU3013785 | 1548 | 1136..2656 | 3504 |
| THYMU3013897 | 1549 | 1550..1981 | 3505 |
| THYMU3014038 | 1550 | 140.. 571 | 3506 |
| THYMU3014173 | 1551 | 20.. 448 | 3507 |
| THYMU3014372 | 1552 | 58..2484 | 3508 |
| THYMU3014620 | 1553 | 217..1077 | 3509 |
| THYMU3014701 | 1554 | 165.. 650 | 3510 |
| THYMU3015042 | 1555 | 731..>1237 | 3511 |
| THYMU3015457 | 1556 | 492.. 854 | 3512 |
| THYMU3015571 | 1557 | 593.. 895 | 3513 |
| THYMU3015647 | 1558 | 255..1841 | 3514 |
| THYMU3016518 | 1559 | 38..1657 | 3515 |
| THYMU3016822 | 1560 | 183.. 611 | 3516 |
| THYMU3017761 | 1561 | 711..1121 | 3517 |
| THYMU3018151 | 1562 | 186.. 518 | 3518 |
| THYMU3018896 | 1563 | 49.. 429 | 3519 |
| THYMU3019095 | 1564 | 2331..2666 | 3520 |
| THYMU3019476 | 1565 | 115.. 732 | 3521 |
| THYMU3019605 | 1566 | 243.. 716 | 3522 |
| THYMU3019916 | 1567 | 1539..1937 | 3523 |
| THYMU3020221 | 1568 | 80..1513 | 3524 |
| THYMU3020869 | 1569 | 29.. 415 | 3525 |
| THYMU3020970 | 1570 | 1659..2924 | 3526 |
| THYMU3021404 | 1571 | 314..2083 | 3527 |
| THYMU3021586 | 1572 | 466..3147 | 3528 |
| THYMU3021755 | 1573 | 838..1827 | 3529 |
| THYMU3021900 | 1574 | 2284..2676 | 3530 |
| THYMU3022211 | 1575 | 36.. 431 | 3531 |
| THYMU3022434 | 1576 | 1094..>3280 | 3532 |
| THYMU3022528 | 1577 | 165.. 518 | 3533 |
| THYMU3022668 | 1578 | 2098..2616 | 3534 |
| THYMU3022982 | 1579 | 282.. 626 | 3535 |
| THYMU3023107 | 1580 | 91..1113 | 3536 |
| THYMU3023394 | 1581 | 3.. 521 | 3537 |
| THYMU3023400 | 1582 | 849..2909 | 3538 |
| THYMU3023797 | 1583 | 93.. 443 | 3539 |
| THYMU3024164 | 1584 | 188.. 652 | 3540 |
| THYMU3024339 | 1585 | 1334..1924 | 3541 |
| THYMU3025118 | 1586 | 30..1625 | 3542 |
| THYMU3025313 | 1587 | 103.. 546 | 3543 |
| THYMU3025642 | 1588 | 120..1124 | 3544 |
| THYMU3025683 | 1589 | 125..2854 | 3545 |
| THYMU3025772 | 1590 | 91.. 921 | 3546 |
| THYMU3026000 | 1591 | 218..1642 | 3547 |
| THYMU3026306 | 1592 | 2902..4020 | 3548 |
| THYMU3026350 | 1593 | 162.. 488 | 3549 |
| THYMU3026479 | 1594 | 1530..2747 | 3550 |
| THYMU3026532 | 1595 | 83..2368 | 3551 |
| THYMU3026783 | 1596 | 3025..3552 | 3552 |
| THYMU3026869 | 1597 | 1884..2414 | 3553 |
| THYMU3027251 | 1598 | 2631..3344 | 3554 |
| THYMU3027540 | 1599 | 244.. 633 | 3555 |
| THYMU3027655 | 1600 | 2018..2356 | 3556 |
| THYMU3027671 | 1601 | 2909..3913 | 3557 |
| THYMU3028075 | 1602 | 339.. 680 | 3558 |
| THYMU3028461 | 1603 | 15.. 584 | 3559 |
| THYMU3028702 | 1604 | 76..4164 | 3560 |
| THYMU3029188 | 1605 | 652..1104 | 3561 |
| THYMU3029318 | 1606 | 81.. 509 | 3562 |
| THYMU3029421 | 1607 | 2123..3187 | 3563 |
| THYMU3029719 | 1608 | 2067..2567 | 3564 |
| THYMU3029774 | 1609 | 13.. 954 | 3565 |
| THYMU3029795 | 1610 | 2128..3012 | 3566 |
| THYMU3030072 | 1611 | 96..2144 | 3567 |
| THYMU3030706 | 1612 | 237.. 581 | 3568 |
| THYMU3030752 | 1613 | 261..1238 | 3569 |
| THYMU3031146 | 1614 | 182.. 688 | 3570 |
| THYMU3031402 | 1615 | 78.. 395 | 3571 |
| THYMU3031612 | 1616 | 836..1360 | 3572 |
| THYMU3031868 | 1617 | 6..1733 | 3573 |
| THYMU3031878 | 1618 | 43.. 930 | 3574 |
| THYMU3032032 | 1619 | 159.. 536 | 3575 |
| THYMU3032798 | 1620 | 349..2139 | 3576 |
| THYMU3032867 | 1621 | 146.. 565 | 3577 |
| THYMU3033626 | 1622 | 2310..2663 | 3578 |
| THYMU3033630 | 1623 | 6.. 428 | 3579 |
| THYMU3033649 | 1624 | 79..1047 | 3580 |
| THYMU3033754 | 1625 | 439..2073 | 3581 |
| THYMU3033759 | 1626 | 198..>2907 | 3582 |
| THYMU3034099 | 1627 | 2025..2519 | 3583 |
| THYMU3034453 | 1628 | 652..1053 | 3584 |
| THYMU3034616 | 1629 | 1533..1922 | 3585 |
| THYMU3034671 | 1630 | 737..2014 | 3586 |
| THYMU3034853 | 1631 | 118.. 510 | 3587 |
| THYMU3034867 | 1632 | 287.. 619 | 3588 |
| THYMU3034983 | 1633 | 1151..1942 | 3589 |
| THYMU3036200 | 1634 | 59..3331 | 3590 |
| THYMU3036310 | 1635 | 262.. 702 | 3591 |
| THYMU3036934 | 1636 | 141.. 482 | 3592 |
| THYMU3036953 | 1637 | 77.. 502 | 3593 |
| THYMU3037052 | 1638 | 274..1278 | 3594 |
| THYMU3037192 | 1639 | 1952..2524 | 3595 |
| THYMU3037617 | 1640 | 1845..3041 | 3596 |
| THYMU3037772 | 1641 | 56..1489 | 3597 |
| THYMU3037827 | 1642 | 175.. 543 | 3598 |
| THYMU3037856 | 1643 | 1766..2281 | 3599 |
| THYMU3037867 | 1644 | 85.. 606 | 3600 |
| THYMU3037909 | 1645 | 115.. 645 | 3601 |
| THYMU3037980 | 1646 | 2802..3170 | 3602 |
| THYMU3038158 | 1647 | 286..1623 | 3603 |
| THYMU3038167 | 1648 | 2621..3073 | 3604 |
| THYMU3038214 | 1649 | 1458..2123 | 3605 |
| THYMU3038266 | 1650 | 47.. 358 | 3606 |
| THYMU3038347 | 1651 | 2641..3315 | 3607 |
| THYMU3038375 | 1652 | 1644..>3418 | 3608 |
| THYMU3038603 | 1653 | 568..1041 | 3609 |
| THYMU3038687 | 1654 | 1496..2065 | 3610 |
| THYMU3038759 | 1655 | 2993..>3473 | 3611 |
| THYMU3038879 | 1656 | 543.. 860 | 3612 |
| THYMU3038970 | 1657 | 253..1536 | 3613 |
| THYMU3039807 | 1658 | 107.. 445 | 3614 |
| THYMU3039846 | 1659 | 153.. 632 | 3615 |
| THYMU3040068 | 1660 | 111.. 683 | 3616 |
| THYMU3040126 | 1661 | 32.. 943 | 3617 |
| THYMU3040146 | 1662 | 249.. 707 | 3618 |
| THYMU3040168 | 1663 | 1088..1630 | 3619 |
| THYMU3040172 | 1664 | 187..2136 | 3620 |
| THYMU3040725 | 1665 | 33.. 359 | 3621 |
| THYMU3040746 | 1666 | 41..1666 | 3622 |
| THYMU3040816 | 1667 | 1824..2267 | 3623 |
| THYMU3040829 | 1668 | 85.. 492 | 3624 |
| THYMU3040830 | 1669 | 1770..2087 | 3625 |
| THYMU3041354 | 1670 | 108.. 419 | 3626 |
| THYMU3041386 | 1671 | 109.. 420 | 3627 |
| THYMU3041428 | 1672 | 81..2483 | 3628 |
| THYMU3041573 | 1673 | 88.. 951 | 3629 |
| THYMU3041603 | 1674 | 2167..2631 | 3630 |
| THYMU3041736 | 1675 | 3246..3650 | 3631 |
| THYMU3041918 | 1676 | 2664..2990 | 3632 |
| THYMU3042075 | 1677 | 93.. 401 | 3633 |
| THYMU3042321 | 1678 | 1382..2146 | 3634 |
| THYMU3042758 | 1679 | 201.. 557 | 3635 |
| THYMU3043200 | 1680 | 955..2460 | 3636 |
| THYMU3043327 | 1681 | 1405..2043 | 3637 |
| THYMU3043482 | 1682 | 944..1321 | 3638 |
| THYMU3043688 | 1683 | 48.. 461 | 3639 |
| THYMU3043779 | 1684 | 159.. 833 | 3640 |
| THYMU3043883 | 1685 | 67.. 417 | 3641 |
| THYMU3043993 | 1686 | 87.. 572 | 3642 |
| THYMU3044075 | 1687 | 55..1215 | 3643 |
| THYMU3044188 | 1688 | 670..1035 | 3644 |
| THYMU3044441 | 1689 | 1014..1415 | 3645 |
| THYMU3044445 | 1690 | 153.. 500 | 3646 |
| THYMU3045510 | 1691 | 1095..2255 | 3647 |
| THYMU3045673 | 1692 | 337.. 810 | 3648 |
| THYMU3045692 | 1693 | 105..1211 | 3649 |
| THYMU3045704 | 1694 | 1040.. 1627 | 3650 |
| THYMU3046140 | 1695 | 2273..2683 | 3651 |
| THYMU3046360 | 1696 | 22..1254 | 3652 |
| THYMU3047115 | 1697 | 199..2808 | 3653 |
| THYMU3047144 | 1698 | 84.. 701 | 3654 |
| THYMU3047156 | 1699 | 152.. 544 | 3655 |
| THYMU3047513 | 1700 | 1574..2215 | 3656 |
| THYMU3047542 | 1701 | 1008..1358 | 3657 |
| THYMU3047760 | 1702 | 2623..3039 | 3658 |
| THYMU3047891 | 1703 | 804..1325 | 3659 |
| TKIDN2000319 | 1704 | 13.. 720 | 3660 |
| TKIDN2003396 | 1705 | 442..1374 | 3661 |
| TKIDN2010602 | 1706 | 121.. 684 | 3662 |
| TKIDN2011051 | 1707 | 274..1995 | 3663 |
| TKIDN2011160 | 1708 | 322..1305 | 3664 |
| TLIVE2000142 | 1709 | 2934..3287 | 3665 |
| TLIVE2001616 | 1710 | 521..2425 | 3666 |
| TLIVE2007736 | 1711 | 653..1261 | 3667 |
| TLIVE2008797 | 1712 | 237.. 560 | 3668 |
| TLUNG2000654 | 1713 | 55..1392 | 3669 |
| TLUNG2001445 | 1714 | 80..1558 | 3670 |
| TLUNG2001600 | 1715 | 80..1507 | 3671 |
| TLUNG2001810 | 1716 | 190.. 567 | 3672 |
| TLUNG2002055 | 1717 | 245.. 676 | 3673 |
| TRACH2011057 | 1718 | 1660..2277 | 3674 |
| TRACH2013585 | 1719 | 395..1864 | 3675 |
| TRACH2019080 | 1720 | 294.. 599 | 3676 |
| TRACH2022113 | 1721 | 1206..1520 | 3677 |
| TRACH2024730 | 1722 | 8..2029 | 3678 |
| TRACH3002188 | 1723 | 210.. 584 | 3679 |
| TRACH3002293 | 1724 | 1633..2253 | 3680 |
| TRACH3002752 | 1725 | 1018..3147 | 3681 |
| TRACH3002890 | 1726 | 197.. 781 | 3682 |
| TRACH3003037 | 1727 | 233..3394 | 3683 |
| TRACH3003357 | 1728 | 597..2234 | 3684 |
| TRACH3003458 | 1729 | 267..2207 | 3685 |
| TRACH3003872 | 1730 | 70.. 543 | 3686 |
| TRACH3004113 | 1731 | 692..2758 | 3687 |
| TRACH3004288 | 1732 | 271.. 759 | 3688 |
| TRACH3004412 | 1733 | 1696..3171 | 3689 |
| TRACH3004424 | 1734 | 75..2810 | 3690 |
| TRACH3004596 | 1735 | 153.. 536 | 3691 |
| TRACH3004747 | 1736 | 16..2190 | 3692 |
| TRACH3005173 | 1737 | 796..1419 | 3693 |
| TRACH3005191 | 1738 | 2363..>4128 | 3694 |
| TRACH3005274 | 1739 | 3430..4416 | 3695 |
| TRACH3005699 | 1740 | 174..4097 | 3696 |
| TRACH3006379 | 1741 | 52.. 810 | 3697 |
| TRACH3006397 | 1742 | 1332..1634 | 3698 |
| TRACH3006717 | 1743 | 114..1691 | 3699 |
| TRACH3006800 | 1744 | 112..5055 | 3700 |
| TRACH3007274 | 1745 | 1276..1635 | 3701 |
| TRACH3007625 | 1746 | 252..1412 | 3702 |
| TRACH3007689 | 1747 | 1626..3743 | 3703 |
| TRACH3007995 | 1748 | 2775..3248 | 3704 |
| TRACH3008042 | 1749 | 543..1694 | 3705 |
| TRACH3008508 | 1750 | 1594..2829 | 3706 |
| TRACH3008632 | 1751 | 590.. 919 | 3707 |
| TRACH3009008 | 1752 | 63..1808 | 3708 |
| TRACH3009061 | 1753 | 130..2496 | 3709 |
| TRACH3009701 | 1754 | 34..2739 | 3710 |
| TRACH3010079 | 1755 | 2516..3328 | 3711 |
| TRACH3010167 | 1756 | 285..1580 | 3712 |
| TRACH3010342 | 1757 | 3307..3627 | 3713 |
| TRACH3010382 | 1758 | 222.. 608 | 3714 |
| TRACH3011082 | 1759 | 35.. 613 | 3715 |
| TRACH3011184 | 1760 | 33.. 425 | 3716 |
| TRACH3011282 | 1761 | 100.. 630 | 3717 |
| TRACH3011313 | 1762 | 155..1132 | 3718 |
| TRACH3011454 | 1763 | 285.. 839 | 3719 |
| TRACH3011503 | 1764 | 237..1391 | 3720 |
| TRACH3011538 | 1765 | 185..1324 | 3721 |
| TRACH3012106 | 1766 | 405..2675 | 3722 |
| TRACH3012460 | 1767 | 3.. 605 | 3723 |
| TRACH3012659 | 1768 | 80.. 709 | 3724 |
| TRACH3012718 | 1769 | 2096..2527 | 3725 |
| TRACH3012864 | 1770 | 224.. 850 | 3726 |
| TRACH3012891 | 1771 | 236.. 580 | 3727 |
| TRACH3013043 | 1772 | 810..3005 | 3728 |
| TRACH3013072 | 1773 | 92..1135 | 3729 |
| TRACH3013684 | 1774 | 188.. 565 | 3730 |
| TRACH3013900 | 1775 | 296.. 673 | 3731 |
| TRACH3014063 | 1776 | 3143..3523 | 3732 |
| TRACH3014183 | 1777 | 119..4420 | 3733 |
| TRACH3014580 | 1778 | 1813..2325 | 3734 |
| TRACH3015136 | 1779 | 832..1560 | 3735 |
| TRACH3015346 | 1780 | 265..1728 | 3736 |
| TRACH3015354 | 1781 | 776..2002 | 3737 |
| TRACH3015467 | 1782 | 283.. 678 | 3738 |
| TRACH3015951 | 1783 | 49..1932 | 3739 |
| TRACH3016264 | 1784 | 734..1672 | 3740 |
| TRACH3016368 | 1785 | 384.. 806 | 3741 |
| TRACH3016455 | 1786 | 2059..2943 | 3742 |
| TRACH3016805 | 1787 | 2373..4172 | 3743 |
| TRACH3016885 | 1788 | 1228..1644 | 3744 |
| TRACH3016992 | 1789 | 20.. 352 | 3745 |
| TRACH3017409 | 1790 | 22..3924 | 3746 |
| TRACH3018108 | 1791 | 168..1538 | 3747 |
| TRACH3018191 | 1792 | 2795..3268 | 3748 |
| TRACH3018240 | 1793 | 5.. 337 | 3749 |
| TRACH3018261 | 1794 | 29.. 586 | 3750 |
| TRACH3018519 | 1795 | 98.. 406 | 3751 |
| TRACH3018524 | 1796 | 17..>4237 | 3752 |
| TRACH3018606 | 1797 | 672..1229 | 3753 |
| TRACH3018783 | 1798 | 273.. 617 | 3754 |
| TRACH3018907 | 1799 | 81..1559 | 3755 |
| TRACH3018943 | 1800 | 113.. 451 | 3756 |
| TRACH3019058 | 1801 | 41..1054 | 3757 |
| TRACH3019370 | 1802 | 206..2761 | 3758 |
| TRACH3019621 | 1803 | 1553..2779 | 3759 |
| TRACH3019807 | 1804 | 3915..4550 | 3760 |
| TRACH3020137 | 1805 | 186.. 710 | 3761 |
| TRACH3020563 | 1806 | 2232..3158 | 3762 |
| TRACH3020605 | 1807 | 1391..3862 | 3763 |
| TRACH3020769 | 1808 | 62..>3300 | 3764 |
| TRACH3020930 | 1809 | 40..1773 | 3765 |
| TRACH3021023 | 1810 | 38..1618 | 3766 |
| TRACH3021335 | 1811 | 346..2559 | 3767 |
| TRACH3021373 | 1812 | 1857..4919 | 3768 |
| TRACH3021544 | 1813 | 1471..3357 | 3769 |
| TRACH3021778 | 1814 | 989..2113 | 3770 |
| TRACH3021834 | 1815 | 204.. 860 | 3771 |
| TRACH3021883 | 1816 | 1935..2594 | 3772 |
| TRACH3022109 | 1817 | 14..1000 | 3773 |
| TRACH3022198 | 1818 | 422.. 991 | 3774 |
| TRACH3022296 | 1819 | 45.. 431 | 3775 |
| TRACH3022732 | 1820 | 115.. 426 | 3776 |
| TRACH3022758 | 1821 | 274..2112 | 3777 |
| TRACH3022960 | 1822 | 221..3502 | 3778 |
| TRACH3023063 | 1823 | 300.. 671 | 3779 |
| TRACH3023203 | 1824 | 37.. 429 | 3780 |
| TRACH3023242 | 1825 | 830..1333 | 3781 |
| TRACH3023373 | 1826 | 417..>3334 | 3782 |
| TRACH3023516 | 1827 | 112..1824 | 3783 |
| TRACH3023752 | 1828 | 90.. 998 | 3784 |
| TRACH3023945 | 1829 | 223.. 564 | 3785 |
| TRACH3023960 | 1830 | 1542..3920 | 3786 |
| TRACH3024020 | 1831 | 709..1401 | 3787 |
| TRACH3024081 | 1832 | 463..1197 | 3788 |
| TRACH3024342 | 1833 | 97.. 726 | 3789 |
| TRACH3024423 | 1834 | 964..1926 | 3790 |
| TRACH3024428 | 1835 | 96.. 488 | 3791 |
| TRACH3024512 | 1836 | 97..2523 | 3792 |
| TRACH3024671 | 1837 | 903..2780 | 3793 |
| TRACH3024823 | 1838 | 1468..2226 | 3794 |
| TRACH3025316 | 1839 | 727..1662 | 3795 |
| TRACH3025346 | 1840 | 1687..2352 | 3796 |
| TRACH3025520 | 1841 | 3112..3417 | 3797 |
| TRACH3026283 | 1842 | 45.. 821 | 3798 |
| TRACH3026299 | 1843 | 80..1675 | 3799 |
| TRACH3026303 | 1844 | 155..1705 | 3800 |
| TRACH3026542 | 1845 | 144.. 545 | 3801 |
| TRACH3026650 | 1846 | 56..4174 | 3802 |
| TRACH3026676 | 1847 | 119.. 754 | 3803 |
| TRACH3026949 | 1848 | 410.. 766 | 3804 |
| TRACH3027229 | 1849 | 774..1532 | 3805 |
| TRACH3027681 | 1850 | 64.. 474 | 3806 |
| TRACH3027701 | 1851 | 132..1484 | 3807 |
| TRACH3028164 | 1852 | 63.. 467 | 3808 |
| TRACH3028180 | 1853 | 293..2080 | 3809 |
| TRACH3028441 | 1854 | 81..1685 | 3810 |
| TRACH3028597 | 1855 | 110.. 415 | 3811 |
| TRACH3028837 | 1856 | 1811..4627 | 3812 |
| TRACH3028855 | 1857 | 202..2187 | 3813 |
| TRACH3029139 | 1858 | 186.. 506 | 3814 |
| TRACH3029329 | 1859 | 108..1742 | 3815 |
| TRACH3029462 | 1860 | 262..>4112 | 3816 |
| TRACH3029520 | 1861 | 839..1219 | 3817 |
| TRACH3029592 | 1862 | 2928..3299 | 3818 |
| TRACH3029670 | 1863 | 80..1696 | 3819 |
| TRACH3030176 | 1864 | 17..3145 | 3820 |
| TRACH3030855 | 1865 | 2600..3463 | 3821 |
| TRACH3031316 | 1866 | 26.. 463 | 3822 |
| TRACH3031660 | 1867 | 25..1242 | 3823 |
| TRACH3031678 | 1868 | 491..1723 | 3824 |
| TRACH3032044 | 1869 | 2.. 472 | 3825 |
| TRACH3032150 | 1870 | 29..1042 | 3826 |
| TRACH3032480 | 1871 | 97.. 420 | 3827 |
| TRACH3032570 | 1872 | 1515..2471 | 3828 |
| TRACH3033535 | 1873 | 7.. 492 | 3829 |
| TRACH3034680 | 1874 | 17..1813 | 3830 |
| TRACH3035451 | 1875 | 17..>5003 | 3831 |
| TRACH3036004 | 1876 | 996..4160 | 3832 |
| TRACH3036103 | 1877 | 80.. 556 | 3833 |
| TRACH3036278 | 1878 | 40.. 492 | 3834 |
| TRACH3036750 | 1879 | 209..1915 | 3835 |
| TRACH3036792 | 1880 | 110.. 457 | 3836 |
| TRACH3036897 | 1881 | 2016..2777 | 3837 |
| TRACH3036932 | 1882 | 87.. 662 | 3838 |
| TRACH3037505 | 1883 | 81..1694 | 3839 |
| TRACH3038399 | 1884 | 509..3406 | 3840 |
| TSTOM2000235 | 1885 | 17..1378 | 3841 |
| TSTOM2001571 | 1886 | 1784..2608 | 3842 |
| TSTOM2002611 | 1887 | 72.. 641 | 3843 |
| TSTOM2002682 | 1888 | 97.. 480 | 3844 |
| TUTER1000014 | 1889 | 98.. 937 | 3845 |
| TUTER2001433 | 1890 | 80..1579 | 3846 |
| UTERU2000300 | 1891 | 608..1351 | 3847 |
| UTERU2014998 | 1892 | 876..1379 | 3848 |
| UTERU2016464 | 1893 | 662..1540 | 3849 |
| UTERU2016669 | 1894 | 111.. 758 | 3850 |
| UTERU2020226 | 1895 | 1256..1747 | 3851 |
| UTERU2022955 | 1896 | 139.. 543 | 3852 |
| UTERU2023941 | 1897 | 121.. 687 | 3853 |
| UTERU2024042 | 1898 | 192.. 632 | 3854 |
| UTERU2027369 | 1899 | 161.. 697 | 3855 |
| UTERU2028377 | 1900 | 348.. 773 | 3856 |
| UTERU2029660 | 1901 | 1766..2335 | 3857 |
| UTERU2035926 | 1902 | 187.. 552 | 3858 |
| UTERU2037423 | 1903 | 1228..2184 | 3859 |
| UTERU3000670 | 1904 | 122..5446 | 3860 |
| UTERU3001029 | 1905 | 1074..3416 | 3861 |
| UTERU3001394 | 1906 | 76.. 651 | 3862 |
| UTERU3001946 | 1907 | 133..2736 | 3863 |
| UTERU3004635 | 1908 | 3..>5144 | 3864 |
| UTERU3005264 | 1909 | 2519..4000 | 3865 |
| UTERU3005422 | 1910 | 51.. 962 | 3866 |
| UTERU3006538 | 1911 | 2549..3055 | 3867 |
| UTERU3006720 | 1912 | 1643..2821 | 3868 |
| UTERU3007108 | 1913 | 343.. 783 | 3869 |
| UTERU3009775 | 1914 | 1679..2092 | 3870 |
| UTERU3010029 | 1915 | 1769..2287 | 3871 |
| UTERU3010409 | 1916 | 2375..2902 | 3872 |
| UTERU3010604 | 1917 | 243.. 761 | 3873 |
| UTERU3010892 | 1918 | 2018..2425 | 3874 |
| UTERU3010919 | 1919 | 125..1669 | 3875 |
| UTERU3011092 | 1920 | 1978..2853 | 3876 |
| UTERU3011398 | 1921 | 4299..4982 | 3877 |
| UTERU3011558 | 1922 | 1239..3335 | 3878 |
| UTERU3011579 | 1923 | 160.. 795 | 3879 |
| UTERU3011837 | 1924 | 836..1627 | 3880 |
| UTERU3012293 | 1925 | 638..2485 | 3881 |
| UTERU3012414 | 1926 | 469..1170 | 3882 |
| UTERU3012476 | 1927 | 66.. 425 | 3883 |
| UTERU3012599 | 1928 | 207.. 632 | 3884 |
| UTERU3012999 | 1929 | 1.. 501 | 3885 |
| UTERU3013167 | 1930 | 2919..3398 | 3886 |
| UTERU3013302 | 1931 | 1461..3221 | 3887 |
| UTERU3014274 | 1932 | 58.. 540 | 3888 |
| UTERU3014647 | 1933 | 199.. 558 | 3889 |
| UTERU3014906 | 1934 | 5.. 625 | 3890 |
| UTERU3015011 | 1935 | 1055..2266 | 3891 |
| UTERU3015299 | 1936 | 470.. 937 | 3892 |
| UTERU3015647 | 1937 | 771..1136 | 3893 |
| UTERU3015844 | 1938 | 1871..2188 | 3894 |
| UTERU3016070 | 1939 | 138..1613 | 3895 |
| UTERU3016273 | 1940 | 135.. 776 | 3896 |
| UTERU3016274 | 1941 | 226..1458 | 3897 |
| UTERU3016308 | 1942 | 220..1974 | 3898 |
| UTERU3017441 | 1943 | 616..3621 | 3899 |
| UTERU3017626 | 1944 | 2588..3340 | 3900 |
| UTERU3017995 | 1945 | 1796..2227 | 3901 |
| UTERU3018172 | 1946 | 2006..2551 | 3902 |
| UTERU3018255 | 1947 | 259.. 741 | 3903 |
| UTERU3019708 | 1948 | 993..2114 | 3904 |
| UTERU3020090 | 1949 | 1828..3153 | 3905 |
| UTERU3021231 | 1950 | 172.. 576 | 3906 |
| UTERU3021850 | 1951 | 665..1906 | 3907 |
| UTERU3022168 | 1952 | 61..3237 | 3908 |
| UTERU3022588 | 1953 | 269..1717 | 3909 |
| UTERU3022922 | 1954 | 66.. 368 | 3910 |
| UTERU3023141 | 1955 | 93.. 947 | 3911 |
| UTERU3023413 | 1956 | 164.. 490 | 3912 |
| ADRGL2011190 | 3913 | 56..2113 | 3952 |
| BRACE3002184 | 3914 | 199..1890 | 3953 |
| BRACE3026993 | 3915 | 143..4903 | 3954 |
| BRACE3046450 | 3916 | 361..4356 | 3955 |
| BRAMY3008096 | 3917 | 602..1396 | 3956 |
| BRAMY3016953 | 3918 | 4547..5188 | 3957 |
| BRAWH3013197 | 3919 | 233..2623 | 3958 |
| BRAWH3028645 | 3920 | 256..1938 | 3959 |
| BRAWH3046240 | 3921 | 2257..3441 | 3960 |
| BRCAN2019772 | 3922 | 5..1273 | 3961 |
| BRHIP3030064 | 3923 | 3039..3347 | 3962 |
| BRHIP3038037 | 3924 | 122.. 739 | 3963 |
| BRTHA3004432 | 3925 | 1988..2629 | 3964 |
| BRTHA3024233 | 3926 | 1440..2039 | 3965 |
| CTONG2002832 | 3927 | 346..2148 | 3966 |
| CTONG2003764 | 3928 | 1438..2169 | 3967 |
| PLACE7013963 | 3929 | 2326..>5202 | 3968 |
| PROST2010326 | 3930 | 298..1869 | 3969 |
| TBAES2004105 | 3931 | 135..>1864 | 3970 |
| TBAES2007379 | 3932 | 70..2118 | 3971 |
| TBAES2007481 | 3933 | 53..1162 | 3972 |
| TBAES2008133 | 3934 | 389..2719 | 3973 |
| TESTI2043585 | 3935 | 310..1296 | 3974 |
| TESTI2046536 | 3936 | 111.. 533 | 3975 |
| TESTI4002988 | 3937 | 597..2687 | 3976 |
| TESTI4005158 | 3938 | 952..2961 | 3977 |
| TESTI4005500 | 3939 | 249..2336 | 3978 |
| TESTI4052089 | 3940 | 1517..2038 | 3979 |
| THYMU3024602 | 3941 | 31..2634 | 3980 |
| THYMU3044175 | 3942 | 1101..2054 | 3981 |
| THYMU3046350 | 3943 | 282..1787 | 3982 |
| TLIVE2007192 | 3944 | 350.. 730 | 3983 |
| TRACH1000193 | 3945 | 998..2956 | 3984 |
| TRACH3019290 | 3946 | 152..1738 | 3985 |
| TRACH3021066 | 3947 | 2485..3792 | 3986 |
| UTERU2017492 | 3948 | 80.. 808 | 3987 |
| UTERU2025415 | 3949 | 47.. 379 | 3988 |
| UTERU2036507 | 3950 | 276..1691 | 3989 |
| UTERU3020583 | 3951 | 126..575 | 3990 |

Primers used to synthesize polynucleotides can be designed based on the nucleotide sequences of polynucleotides of the present invention, shown in SEQ ID NOs in Table 1 above. When synthesizing full-length cDNAs, an oligo dT primer can be used as the 3'-end primer. The length of the primer is usually 15-100 bp, and favorably between 15-35 bp. In the case of LA PCR, described below, a primer length of 25-35 bp provides a good result.

Methods for designing a primer that enables specific amplification based on a target nucleotide sequence are known to those skilled in the art (Current Protocols in Molecular Biology, Ausubel et al. edit, (1987) John Wiley & Sons, Section 6.1-6.4). In principle, primers based on 5'-end sequences are designed such that amplification products will include the translation start site. Accordingly, for example, when the 5'-end primer is designed based on the nucleotide sequence of the 5' untranslated region (5'UTR), any part of the 5'-end, which ensures specificity to the cDNA of interest, can be selected as the primer.

When synthesizing a full-length cDNA, the target nucleotide sequence to be amplified can extend to several thousand bp in some cDNA. Such long nucleotides can be amplified using methods such as LA PCR (Long and Accurate PCR). The use of LAPCR is advantageous when synthesizing long DNA. In LA PCR, in which a special DNA polymerase having 3' -> 5' exonuclease activity is used, misincorporated nucleotides can be removed. Accordingly, accurate synthesis of the complementary strand can be achieved even with a long nucleotide sequence. By using LA PCR, amplification of nucleotides 20 kb or longer can be achieved under desirable conditions (Takeshi Hayashi (1996) Jikken-Igaku Bessatsu, "Advanced Technologies in PCR" Youdo-sha).

Template DNAs for synthesizing the full-length cDNAs of the present invention can be obtained by using cDNA libraries prepared by various methods. The full-length cDNA clones of the present invention are clones with a high probability of completeness in length, obtained by a method comprising the steps of [1] preparing libraries containing cDNAs with a very high fullness ratio using oligo-capping, and [2] assembling 5'-end sequences and selecting those with the highest probability of completeness in length in clusters formed (there are many clones longer in the 5'-end direction).

However, the use of primers designed based on the full-length nucleotide sequences provided by the present invention enable full-length cDNAs to be easily obtained without using such a special technique.

The problem with commercially available cDNA libraries or those prepared by known methods is that mRNA contained in these libraries has a very low fullness ratio. Thus, it is difficult to screen full-length cDNA clones directly from the library using ordinary cloning methods. The present invention has revealed nucleotide sequences of novel full-length cDNA. If such a full-length nucleotide sequence is provided, it is possible to synthesize a target full-length cDNA by using enzymatic reactions such as PCR. In particular, a full-length-enriched cDNA library, synthesized by methods such as oligo-capping, is desirable to more reliably synthesize a full-length cDNA.

The present invention provides isolated polynucleotides comprising the nucleotide sequences of SEQ ID NO: 1 as shown in Table 1, or homologs thereof. As used herein, an "isolated polynucleotide" is a polynucleotide whose structure is not identical to that of any naturally occurring polynucleotide or to that of any fragment of a naturally occurring genomic polynucleotide spanning more than three separate genes. The term therefore includes, for example, (a) a DNA which comprises the sequence of part of a naturally occurring genomic DNA molecule in the genome of the organism in which it naturally occurs; (b) a polynucleotide incorporated into a vector or into the genomic DNA of a prokaryote or eukaryote such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion polypeptide. Specifically excluded from this definition are polynucleotides of DNA molecules present in mixtures of different (i) DNA molecules, (ii) transfected cells, or (iii) cell clones; e.g., as these occur in DNA libraries such as cDNA or genomic DNA libraries.

The 5'-end sequence of the full-length cDNA clones of this invention can be used to isolate the regulatory elements of transcription, including the promoter on the genome. A rough draft of the human genome (an analysis of the human genomic sequence with lower accuracy), which covers 90% of the genome, has been reported (Nature, Vol.409, 814-823, 2001), and by the year 2003, analysis of the entire human genomic sequence will be finished. However, using software to analyze transcription start sites on the human genome, in which long introns exist, is difficult. In contrast, it is easy to specify transcription start sites in the genomic sequence using nucleotide sequences which include the 5'-end of the full-length cDNA clones of the present invention, and thus it is easy to obtain genomic regions involved in transcription regulation, which include promoters contained upstream of the transcription start site.

The polypeptides encoded by the full-length cDNAs of the invention can be prepared as recombinant polypeptides or as natural polypeptides. For example, a recombinant polypeptide can be prepared by inserting a polynucleotide encoding a polypeptide of the present invention into a vector, introducing the vector into an appropriate host cell, and purifying the polypeptide expressed within that transformed host cell, as described below. In contrast, a natural polypeptide can be prepared, for example, by utilizing an affinity column to which is attached an antibody against a polypeptide of the present invention (Current Protocols in Molecular Biology (1987) Ausubel et al. edit, John Wiley & Sons, Section 16.1-16.19). The antibody used for affinity purification may be either a polyclonal antibody, or a monoclonal antibody. Alternatively, *in vitro* translation (see, for example, "On the fidelity of mRNA translation in the nuclease-treated rabbit reticulocyte lysate system. " Dasso M.C., and Jackson R.J. (1989) Nucleic Acids Res. 17: 3129-3144) may be used for preparing a polypeptide of the invention.

The present invention provides substantially pure proteins encoded by the full-length cDNAs of the present invention. The term "substantially pure" herein used in reference to a given protein or polypeptide means that the protein or polypeptide is substantially free from other biological macromolecules. For example, the substantially pure protein or polypeptide is at least 75%, 80%, 85%, 95%, or 99% pure by dry weight. Purity can be measured by any appropriate standard method known in the art, for example, by column chromatography, polyacrylamide gel electrophoresis, or HPLC analysis.

Polypeptides functionally equivalent to the polypeptides of the present invention can be prepared based on the activities of the polypeptides of the present invention, clarified in the above-mentioned manner. Whether or not a particular polypeptide is functionally equivalent to a polypeptide of the present invention can be verified by using the biological activity of the polypeptide of the present invention as an index to examine whether that polypeptide has the said activity.

Polypeptides functionally equivalent to polypeptides of the present invention can be prepared by those skilled in the art, for example, by using a method for introducing mutations into a polypeptide amino acid sequence (for example, site-directed mutagenesis (Current Protocols in Molecular Biology, edit, Ausubel et al., (1987) John Wiley & Sons, Section 8.1-8.5). Such polypeptides can also be generated by spontaneous mutations. The present invention also includes polypeptides comprising the amino acid sequences shown in Table 1 in which one or more amino acids are substituted, deleted, inserted, and/or added, as long as the polypeptides have a function equivalent to that of a polypeptide identified in the Examples of the present invention, described later.

There are no limitations as to the number and site of amino acid mutation, as long as polypeptide function is maintained. The number of mutations typically corresponds to 30% or less, or 20% or less, or 10% or less, preferably 5% or less or 3% or less of the total amino acids, more preferably 2% or less or 1% or less of the total amino acids. Alternatively, herein, substitution of one or more amino acids includes substitution of several amino acids. As used herein, the term "several amino acids" means, for example, five amino acids, preferably four or three amino acids, more preferably two amino acids, and further preferably one amino acid.

Herein "conservative amino acid substitution" refers to substitution of an amino acid residue belonging to a group comprising a chemically similar side chain, with another amino acid in the same group. Groups of amino acid residues having similar side chains have been defined in the art. These groups include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

In addition, polypeptides functionally equivalent to the polypeptides of the present invention can be isolated by using techniques of hybridization or gene amplification known to those skilled in the art. Specifically, using hybridization (Current Protocols in Molecular Biology, edit, Ausubel et al., (1987) John Wiley & Sons, Section 6.3-6.4)), those skilled in the art can usually isolate a polynucleotide highly homologous to a polynucleotide encoding the polypeptide identified in the present Example, based on the identified nucleotide sequence (Table 1) or a portion thereof, and obtain a functionally equivalent polypeptide from the isolated polynucleotide. The present invention includes polypeptides encoded by polynucleotides which hybridize with the polynucleotides encoding the polypeptides identified in the present Example, as long as the polypeptides are functionally equivalent to those identified in the present Example. Organisms from which the functionally equivalent polypeptides are isolated include but are not limited to vertebrates such as humans, mice, rats, rabbits, pigs and cows.

Washing conditions for hybridization for polynucleotide isolation encoding functionally equivalent polypeptides are usually "1x SSC, 0.1% SDS, 37°C"; more stringent conditions are "0.5x SSC, 0.1% SDS, 42°C"; and still more stringent conditions are "0.1x SSC, 0.1% SDS, 65°C". Alternatively, the following conditions can be given as hybridization conditions of the present invention. Namely, conditions in which hybridization is performed at "6x SSC, 40% Formamide, 25°C", and washing at "1x SSC, 55°C" can be given. More preferable conditions are those in which hybridization is performed at "6x SSC, 40% Formamide, 37°C", and washing at "0.2x SSC, 55°C". Even more preferable are those in which hybridization is performed at "6x SSC, 50% Formamide, 37°C", and washing at "0.1x SSC, 62°C". The more stringent the hybridization conditions, the more frequently polynucleotides highly homologous to the probe sequence are isolated. Therefore, it is preferable to conduct hybridization under stringent conditions. Examples of stringent conditions in the present invention are, washing conditions of "0.5x SSC, 0.1% SDS, 42°C", or alternatively, hybridization conditions of "6x SSC, 40% Formamide, 37°C", and washing at "0.2x SSC, 55°C".

One skilled in the art can suitably select various conditions, such as SSC dilution ratio, formamide concentration, and temperature, to accomplish a similar stringency.

The above-mentioned combinations of SSC, SDS, and temperature conditions are indicated just as examples. Those skilled in the art can select hybridization conditions with similar stringency to those mentioned above by properly combining the above-mentioned or other factors that determine hybridization stringency (for example, probe concentration, probe length, and duration of hybridization reaction).

The amino acid sequences of polypeptides isolated by using hybridization techniques usually have high identity to those of the polypeptides of the present invention, shown in Table 1. The present invention encompasses polynucleotides comprising a nucleotide sequence with high identity to the nucleotide sequence of claim 1 (a). Furthermore, the present invention encompasses peptides, or polypeptides comprising an amino acid sequence with high identity to the amino acid sequence encoded by the polynucleotide of claim 1 (b). The term "high identity" indicates sequence identity of at least 40% or more; preferably 60% or more; and more preferably 70% or more. Even more preferable is identity of 90% or more, 93% or more, or 95% or more. Further more preferable is 97% or more, or 99% or more. Identity can be determined using the BLAST search algorithm.

As used herein, "percent identity" of amino acid sequences or nucleic acids is determined using the BLAST algorithm of Karlin and Altschul (Proc. Natl. Acad. Sci. USA 90:5873-5877, 1993). Such an algorithm is incorporated into the BLASTN and BLASTX programs of Altschul et al. (J. Mol. Biol.215:403-410, 1990). BLAST nucleotide searches are performed with the BLASTN program, using for example, score = 100, wordlength = 12. BLAST protein searches are performed with the BLASTX program, using for example, score = 50, wordlength = 3. When utilizing the BLAST and Gapped BLAST programs, the default parameters of each program are used. See http://www.ncbi.nlm.nih.gov.

Using the gene amplification technique (PCR) (Current Protocols in Molecular Biology, edit, Ausubel et al., (1987) John Wiley & Sons, Section 6.1-6.4)) and primers designed based on the nucleotide sequences (Table 1) or portions thereof as identified in the present Example, it is possible to isolate polynucleotide fragments highly homologous to the polynucleotide sequences or portions thereof, and to obtain polypeptides functionally equivalent to a particular polypeptide identified in the present Example, based on the isolated polynucleotide fragment.

The present invention also provides polynucleotides containing at least 15 nucleotides complementary to a polynucleotide comprising a nucleotide sequence of SEQ ID NOs shown in Table 1, or the complementary strand thereof. Herein, the term "complementary strand" is defined as the other strand to one strand of a double stranded DNA composed of A:T and G:C base pairs. In addition, "complementary" is not only defined as sequences completely matching a continuous region of at least 15 nucleotides, but is also defined to include sequences comprising identity of at least 70%, favorably 80% or higher, more favorably 90% or higher, and most favorably 95% or higher within that region. Identity may be determined using the algorithm described herein.

Such polynucleotides includes probes and primers used for the detection and amplification of polynucleotides encoding the inventive polypeptides. When used as a primer, such a polynucleotide usually comprises 15 to 100 bp, and preferably of 15 to 35 bp. When used as a probe, such a polynucleotide comprises the whole or a part of the sequence of a polynucleotide of the present invention, and comprises at least 15 bp. When used as a primer, such a polynucleotide is complementary at the 3'-end, and restriction enzyme recognition sequences or tags can be added to the 5'-end.

Furthermore, polynucleotides of the present invention include antisense polynucleotides for suppressing the expression of a polypeptide of the present invention, which comprises an amino acid sequence of SEQ ID NOs shown in Table 1. To exert an antisense effect, an antisense polynucleotide has at least 15 bp or more, for example 50 bp or more, preferably 100 bp or more, and more preferably 500 bp or more, and usually has 3000 bp or less, and preferably 2000 bp or less. Antisense polynucleotides can be used in gene therapy of diseases caused by abnormalities of the polypeptides of the invention (abnormal function or abnormal expression). An antisense polynucleotide can be prepared, for example, using the phosphorothioate method ("Physicochemical properties of phosphorothioate oligodeoxynucleotides." Stein (1988) Nucleic Acids Res. 16: 3209-3221) based on the sequence information of polynucleotides encoding a polypeptide of this invention (for example, the nucleotide sequences of SEQ ID NOs: 1 to 1956 and SEQ ID NOs: 3913 to 3951).

The present invention also includes polynucleotides that can use ribozyme or RNA interference (RNAi) activity to downregulate expression of a polynucleotide of the present invention, where such a polynucleotide can be designed based on the nucleotide sequence of the polynucleotide of the present invention.

A ribozyme is a polynucleotide that comprises 1) an antisense sequence of a polynucleotide of the present invention, and 2) the nucleotide sequence of a catalytic unit required for catalytic action. The antisense sequence constituting the ribozyme can be appropriately selected to be compatible with the structure of the ribozyme's catalytic unit. The ribozome's catalytic unit is well known in the art. For example, the hammer-head ribozyme (Rossi et al. (1991) Pharmac. Ther. 50: 245-254) and hairpin ribozyme (Hampel et al. (1990) Nucl. Acids Res. 18: 299-304, and U.S. Pat. No. 5,254,678) are known to have nucleotide sequence-specific cleaving activity. These ribozymes use this catalytic activity to cleave, at a specific position, polynucleotides which hybridize to the antisense sequence.

For example, the autolytic domain of a hammer-head ribozyme cleaves on the 3' side of C15 in the sequence G13U14C15. Base pairing between U14 and A9 plays an important role in this activity, and A15 or U15 can be cleaved instead of C15 (Koizumi M, et al: FEBS Lett 228: 228, 1988). A restriction enzyme-like RNA-cleaving ribozyme that recognizes the target RNA sequences UC, UU, or UA can be produced by designing the ribozyme such that the substrate binding site complements the RNA sequence near the target site (Koizumi, M. et al., FEBS Lett, 239:285, 1988; Koizumi, M. and Otsuka, E., Protein, Nucleic acid, and Enzyme, 35:2191, 1990; Koizumi, M. et al., Nucl Acids Res, 17:7059, 1989). For example, the polynucleotides of the present invention (SEQ ID NOs: 1-1956 and SEQ ID NOs: 3913-3951) contain a number of potential target sites. The polynucleotides of the present invention can be cleaved at desired positions with a ribozyme which contains an appropriately selected antisense sequence.

The ribozyme preferably comprises RNA and may be synthesized chemically or produced by enzymatic reaction. Methods of chemical synthesizing RNA are known in the art. Alternatively, the ribozyme can be produced by using RNA polymerase to transcribe a polynucleotide encoding the ribozyme. To produce a ribozyme by transcription, a polynucleotide encoding the ribozyme is arranged downstream of a promotor recognized by an RNA polymerase. Such RNA polymerases include T7 RNA polymerase and SP6 RNA polymerase. Alternatively, a ribozyme can be expressed in a host cell by inserting a polynucleotide encoding the ribozyme into an appropriate vector, and then introducing the vector into a host cell. The vector contains a promotor that can direct the expression of the gene in the host cell.

The present invention also provides siRNA (small interfering RNA) that downregulates the expression of a polynucleotide of the present invention. siRNA is a technique for controlling gene expression that inhibits protein synthesis from an mRNA by using a double-stranded RNA which comprises the same nucleotide sequence as that mRNA (Fire et al. (1998) Nature 391: 806-811). The effect of downregulating gene expression using double-stranded RNA is called "the RNAi effect". siRNA has been reported to effectively control gene expression in mice (Zamore et al. (2000) Cell 101:25-33; Gura (2000) Nature 404: 804-808). Thus the introduction of such a double-stranded RNA into cells can result in selective downregulation of target gene expression.

There is no limitation as to the length of the siRNAs. The double-stranded RNAs introduced into cells are enzymatically digested inside these cells, starting from their original 3' end and forming fragments of 21 bp to 23 bp. The enzyme that digests the double-stranded RNA is called 'dicer'. The resulting double-stranded RNA fragments recognize and bind to target nucleotide sequences which comprise the same sequence. The nucleotide sequence is then cleaved by the activity of RNase III-like nuclease (Hammond et al. (2000) Nature, 404: 293-298; Zamore et al. (2000) Cell 101: 25-33).

siRNA is introduced into cells to downregulate gene expression using RNAi activity. siRNA can be introduced into cells using the same methods as for ribozymes. Specifically, chemically-synthesized, double-stranded RNA can be introduced into cells. When synthesized RNA, including antisense RNA and siRNA, is intended for introduction into cells, it can be pre-modified to prevent degradation by nuclease. For example, thiolated RNA is protected from nuclease degradation.

Alternatively, siRNA can be expressed in cells. For example, siRNA can be expressed in cells by inserting a sense sequence and its corresponding antisense sequence into a vector, and then transforming cells with that vector. When the two strands are adjacent, the expressed double-stranded RNA will have a hairpin-loop structure. When the two strands are expressed under the control of different promotors, the resulting double-stranded RNA will comprise two separate strands. Promotors generally used for the expression of siRNA include the U6 promotor.

The nucleotide sequence of an antisense polynucleotide, ribozyme, or siRNA of the present invention may be completely identical or complementary to any one of the nucleotide sequences of SEQ ID NOs: 1-1956 and SEQ ID NOs: 3913-3951, or may have high homology to these nucleotide sequences. The phrase "high homology" to an antisense polynucleotide, ribozyme, or siRNA nucleotide sequence typically means 90% or higher homology, preferably 95% or higher homology, more preferably 98% or higher homology, and still more preferably 99% or higher homology. The homology of a nucleotide sequence can be estimated, for example, by a method described herein.

One skilled in the art can design siRNA based on the nucleotide sequence of a gene whose expression is to be downregulated. The typical methods for designing siRNA include, for example, those described below. To begin with, it is advantageous to avoid using as target sequences: 1) 5'- or 3'-untranslated regions, and 2) regions adjacent to the start codon.

These regions often serve as binding regions for transcriptional regulatory proteins. In addition, these regions may also contain nucleotide sequences conserved among various mRNAs, and thus they may act to inhibit the expression of genes other than the gene of interest.

Thus, it may be advantageous to arrange the target sequence, for example, within the ORF downstream of the start codon. It is preferable to adjust the number of nucleotides between the start codon and the target sequence, for example, to 50 nucleotides or more. Typically, the nucleotide sequence of an siRNA is designed so that it starts from an aa sequence and comprises 19-21 consecutive nucleotides. A dinucleotide overhang is added to one end of siRNA. The nucleotide sequence of such an overhang may include doublets, dTdT or UU sequences. The GC content of a nucleotide sequence constituting siRNA is preferably about 50%. G and C nucleotide residues are preferably uniformly distributed throughout the siRNA.

The action of siRNA is based on sequence-specific mRNA hybridization. Thus, to achieve downregulation specific to a particular gene, it is essential to make the target nucleotide sequence as specific as possible to that gene. It is thus preferable to use homology searches to confirm that the proposed target nucleotide sequence exhibits negligible homology with other genes. Nucleotide sequence homology can be determined using established algorithms.

As long as an siRNA of the present invention downregulates the expression of a polynucleotide of the present invention, it is not limited to nucleotide sequences obtained using the typical design method described above. For example, even if the target sequence is not specific to the nucleotide sequence of a particular gene, it can specifically downregulate the expression of a gene of interest in cells which do not express genes comprising homologous nucleotide sequences. Furthermore, double-stranded RNA having RNAi activity can be obtained without using the above-described methods typically used to select a target sequence.

The polynucleotides or antisense polynucleotides, ribozymes, and siRNAs of the present invention can be used in, for example, gene therapy. Preferable target diseases may be, for example, cancers or various inflammatory diseases. Such molecules can be used for gene therapy, for example, by administrating them to patients *in vivo* or *ex vivo* using viral vectors such as retroviral vectors, adenoviral vectors, and adeno-related viral vectors, or non-viral vectors such as liposomes.

The present invention also includes partial peptides of the polypeptides of the invention. Such a partial peptide comprises a polypeptide generated as a result of removing a signal peptide from a secretory protein. If a polypeptide of the present invention has activity as a receptor or ligand, the partial peptide may function as a competitive inhibitor of the polypeptide, and may bind to the receptor (or ligand). In addition, the present invention includes an antigen peptide for raising antibodies. For the peptides to be specific to a polypeptide of the present invention, the peptides comprise at least seven amino acids, preferably eight amino acids or more, more preferably nine amino acids or more, and even more preferably ten amino acids or more. The peptide can be used to prepare an antibody against or competitive inhibitor of a polypeptide of the present invention, and can also be used to screen for a receptor that binds to the polypeptide of this invention. The partial peptides of this invention can be produced, for example, by genetic engineering methods, known methods for synthesizing peptides, or by digesting a polypeptide of the invention with an appropriate peptidase.

The present invention also relates to a vector into which a polynucleotide of the invention is inserted. Vectors of the present invention are not limited as long as they can contain the inserted polynucleotide stably. For example, if *E. coli* is used as a host, vectors such as pBluescript vector (Stratagene) are preferred cloning vectors. To produce a polypeptide of the invention, expression vectors are especially useful. Any expression vector can be used as long as it is capable of expressing the polypeptide *in vitro*, in *E. coli,* in cultured cells, or *in vivo*. For example, pBEST vector (Promega) is preferable for *in vitro* expression, pET vector (Invitrogen) for *E. coli*, pME18S-FL3 vector (GenBank Accession No. AB009864) for cultured cells, and pME18S vector (Mol. Cell. Biol. (1988) 8: 466-472) for *in vivo* expression. To insert a polynucleotide of the present invention, ligation utilizing restriction sites can be performed according to standard methods (Current Protocols in Molecular Biology (1987) Ausubel et al. edit, John Wiley & Sons, Section 11.4-11.11).

The GATEWAY™ system (Invitrogen), which is an expression vector construction system for polypeptide expression, can also be used (Experimental Medicine, Vol. 18, No. 19 (December), p2716-2717, 2000). This system includes two types of site-specific recombinases (BP CLONASE™ and LR CLONASE™) derived from lambda phage and uses BP CLONASE™-specific recombination sites for the Entry Vector, and LR CLONASE™-specific recombination sites for the Destination Vector, which may comprise a tag useful for polypeptide purification. With this system, an expression vector can be obtained by using homologous recombination.

First, a polynucleotide fragment of interest is inserted into the entry vector using the first recombination. Then, a second recombination is allowed to take place between the entry vector, where the polynucleotide fragment of interest has been inserted, and the destination vector. Thus, the expression vector can be prepared rapidly and efficiently. Using the above-mentioned typical restriction enzyme/ligase reaction method, expression vector construction and expression of a polypeptide of interest takes about seven to ten days. However, using the GATEWAY™ system, the polypeptide of interest can be expressed and prepared in only three to four days. Thus, the system ensures a high-throughput functional analysis for expressed polypeptides (http://biotech.nikkeibp.co.jp/netlink/lto/gateway/).

The present invention also relates to a transformant carrying a vector of the present invention. Any cell can be used as a host into which a vector of this invention is inserted, and various kinds of host cells can be used depending on the purpose. For example, COS cells or CHO cells can be used for strong expression of the polypeptide in eukaryotic cells.

Introduction of such a vector into host cells can be performed, for example, by calcium phosphate precipitation, electroporation (Current Protocols in Molecular Biology (1987) Ausubel et al. edit, John Wiley & Sons, Section 9.1-9.9), lipofectamine method (GIBCO-BRL), or microinjection, etc.

Further, a polynucleotide containing at least 15 nucleotides comprising a nucleotide sequence of any one of the polynucleotides comprising the nucleotide sequences of SEQ ID NOs shown in Table 1, or the complementary strand thereof, can be used not only as a primer for synthesizing full-length cDNAs, but also for testing and diagnosing abnormalities of the polypeptide encoded by the full-length cDNA of the present invention. For example, by utilizing polymerase chain reaction (genomic DNA-PCR, or RT-PCR) using a polynucleotide of this invention as a primer, a polynucleotide encoding a polypeptide of the invention can be amplified. It is also possible to obtain the regulatory region of expression in the 5'-upstream by using PCR or hybridization, since the transcription start site within the genomic sequence can be easily specified based on the 5'-end sequence of the full-length cDNA. The obtained genomic region can be used for detection and/or diagnosis of sequence abnormality using RFLP analysis, SSCP, or sequencing. Where expression of an mRNA of the present invention varies according to a specific disease, analysis of the amount of mRNA expression using a polynucleotide of the present invention as a probe or a primer enables detection and diagnosis of that disease.

The present invention also relates to antibodies that bind to a polypeptide of the present invention. There are no limitations as to the form of the antibodies of this invention. They include polyclonal antibodies, monoclonal antibodies, or portions thereof that can bind to an antigen. They also include antibodies of all classes. Furthermore, special antibodies such as humanized antibodies and chimeric antibodies are also included.

A polyclonal antibody of this invention can be obtained according to the standard method of synthesizing an oligopeptide corresponding to an amino acid sequence, and immunizing rabbits with that peptide (Current Protocols in Molecular Biology (1987) Ausubel et al. edit, John Wiley & Sons, Section 11.12-11.13). A monoclonal antibody of the present invention can be obtained according to the standard method of purifying a polypeptide expressed in *E. coli*, immunizing mice with that polypeptide, and producing a hybridoma cell by fusing spleen cells and myeloma cells (Current Protocols in Molecular Biology (1987) Ausubel et al. edit, John Wiley & Sons, Section 11.4-11.11).

An antibody binding to a polypeptide of the present invention can be used for purification of the polypeptide of the invention, and also for detection and/or diagnosis of abnormalities of the expression and structure of that polypeptide. Specifically, polypeptides can be extracted, for example, from tissues, blood, or cells, and a polypeptide of this invention can then be detected for the above purpose using Western blotting, immunoprecipitation, or ELISA.

Furthermore, an antibody binding to a polypeptide of the present invention can be utilized for treating a disease associated with that polypeptide. If the antibody is used to treat patients, human antibodies, humanized antibodies, or chimeric antibodies are preferred due to their low antigenicity. Human antibodies can be prepared by immunizing a mouse whose immune system is replaced with that of a human (e.g., see "Functional transplant of megabase human immunoglobulin loci recapitulates human antibody response in mice" Mendez, M.J. et al. (1997) Nat. Genet. 15: 146-156). These humanized antibodies can be prepared by recombination of the hypervariable region of a monoclonal antibody (Methods in Enzymology (1991) 203: 99-121).

The cDNAs (clones) of the present invention include polypeptide sequences encoding proteins whose function can be predicted, such as, for example, secretory and/or membrane proteins, glycoprotein-related proteins, signal transduction-related proteins, transcription-related proteins, disease-related proteins, enzyme and/or metabolism-related proteins, cell division- and/or cell proliferation-related proteins, cytoskeleton-related proteins, nuclear protein and/or RNA synthesis-related proteins, protein synthesis and/or transport-related proteins, cellular defense-related proteins, development and/or differentiation-related proteins, DNA- and/or RNA-binding proteins, ATP- and/or GTP-binding proteins. The results of cDNA homology searches can be used to estimate whether a cDNA sequence comprises the function of an above-described protein. Specifically, the function of a polypeptide encoded by a cDNA of the present invention can be inferred by (a) searching for a known gene or protein which is homologous to the complete or partial nucleotide sequence of the full-length cDNA of the present invention, and (b) comparing the function of the gene and that of the protein encoded by the gene.

Alternatively, the function of a polypeptide encoded by a cDNA of the present invention can be predicted when a signal sequence, transmembrane domain, nuclear translocation signal, glycosylation signal, phosphorylation site, zinc-finger motif, SH3 domain, or such is found in the amino acid sequence. In particular, partial sequence structures such as motif and domain structures are commonly found in a number of proteins, and comprise a minimal functional protein structure. The Pfam database identifies a total of 4,832 types of motifs and domains, including both those whose functions have been clarified and those whose functions remain unclear (http://www.sanger.ac.uk/Software/Pfam/index.shtml) Version 7.7 (the latest version as of December 2002).

A specific example of motif/domain function is shown below. The ITAM motif (immunoreceptor tyrosine-based activation motif) is found in the intracellular region of the T cell receptor which is expressed on the cell membrane of T cells participating in an immune response (Flaswinkel, H. et al. Semin Immunol 1995 Feb,7(1):21-7). The ITAM motif has a tandem YXXL structure (tyrosine-arbitrary amino acid-arbitrary amino acid-leucine). On extracellular stimulus by an antigen or antibody, the tyrosine in the motif is phosphorylated by an enzyme (LCK) with a kinase domain. Then, ZAP70 binds to the phosphorylated tyrosine via the SH2 domain, resulting in downstream signal transduction (Bu, J.Y. et al., Proc Natl Acad Sci U S A 1995 May 23, 92 (11) :5106-10; Neumeister, E.N. et al., Mol Cell Biol 1995 June, 15(6):3171-8).

A similar phenomenon has been found in mast cells as well as in T cells (Chen, T. et al., J Biol Chem 1996 Oct 11, 271(41):25308-15). Thus, at the molecular level, such a phenomenon is the first step in the activation of immune cells in immunologic diseases such as allergies, atopic dermatitis, and asthma.

Even in a simple exemplary scheme such as the one described above, there are three major motif/domain structures - ITAM, the SH2 domain, and the protein kinase domain - each of which play an important role. The mechanism of this scheme can be interpreted using these three structures. Thus, collecting, categorizing and elucidating the function of molecules that comprise common motif/domain structures is exceedingly important in understanding the molecular-based mechanisms of various cellular functions, including and in addition to the immune response described herein. Searching for motif/domain structures is highly important as the first step in elucidating the functions of unknown polypeptides. It is also understood that an entire polypeptide structure is comprised by minimal structures such as motifs and domains, thus providing the overall function of an entire polypeptide.

The overall function of a polypeptide in cells can be accurately predicted at the molecular level using data obtained by domain and motif structure analysis. In addition, a fusion polypeptide comprising a partial amino acid sequence and a GFP protein or the like may be prepared, and then introduced into cultured cells. For example, if a polypeptide is localized on the cell membrane, it may function as a receptor or ion channel. Alternatively, if a polypeptide is localized in the nucleus, it can be predicted to serve as a polynucleotide-binding protein or to participate in transcription. Thus, the function of a polypeptide can also be predicted by determining its localization.

The function of a full-length cDNA obtained in the present invention can be predicted by carrying out the above-described analysis using its entire nucleotide sequence and the amino acid sequence it encodes. Even when the full-length cDNA nucleotide sequence is not available, however, a partial sequence thereof (preferably 300 nucleotides or more) often enables function to be predicted. However, function predicted based on information yielded in a partial sequence homology search will not necessarily be the same as that based on a full-length sequence. Functional prediction based on a full-length nucleotide sequence is obviously preferable.

A more specific method for predicting function involves homology searches of databases such as GenBank, Swiss-Prot, UniGene, nr and RefSeq, using BLAST or FASTA. The functions of polypeptides encoded by the cDNAs of the present invention can be predicted based on hit genes and the function of polypeptides encoded by these genes. Polypeptide functions can be predicted from the amino acid sequences deduced from the structure of the full-length nucleotide sequences. In this way, signal sequences and transmembrane domains can be predicted from amino acid sequences using PSORT [K. Nakai & M. Kanehisa, Genomics, 14: 897-911 (1992)], SOSUI [T. Hirokawa et al., Bioinformatics, 14, 378-379 (1998)] (Mitsui Knowledge Industry Co., Ltd.), MEMSAT [D. T. Jones, W. R. Taylor & J. M. Thornton, Biochemistry, 33, 3038-3049 (1994)], and the like. Alternatively, motifs and domains can be predicted from amino acid sequences by carrying out searches using Pfam, PROSITE (http://www.expasy.ch/prosite/), or such. The above-described procedures facilitate more accurate prediction of polypeptide function.

The databases GenBank, Swiss-Prot, nr and RefSeq were searched as described above for homology to the 1,995 full-length clone sequences of the present invention whose full-length nucleotide had been determined (see Example 4 and the results of homology searches). In addition, the amino acid sequences deduced from the full-length nucleotide sequences were analyzed by database searches for signal sequences and transmembrane domains using PSORT and SOSUI (see Example 5). The clones were categorized into the fourteen functional categories shown below, based on 1) the results of annotation-based functional prediction (by referring to keywords in the hit data of Swiss-Prot, or to Definitions and Reference information in the hit data of nr or RefSeq), 2) the results of PSORT searches for signal sequences using the deduced ORFs and 3) the results of SOSUI searches for transmembrane domains using the deduced ORFs. As a result, 1,006 clones were estimated to encode proteins belonging to the categories described below.
Secretory and/or membrane protein (570 clones)
Glycoprotein-related protein (124 clones)
Signal transduction-related protein (75 clones)
Transcription-related protein (113 clones)
Disease-related protein (414 clones)
Enzyme and/or metabolism-related protein (172 clones)
Cell division- and/or cell proliferation-related protein (29 clones)
Cytoskeleton-related protein (61 clones)
Nuclear protein and/or RNA synthesis-related protein (41 clones)
Protein synthesis- and/or transport-related protein (52 clones)
Cellular defense-related protein (five clones)
Development and/or differentiation-related protein (17 clones)
DNA- and/or RNA-binding protein (127 clones)
ATP- and/or GTP-binding protein (70 clones)

The clones predicted to belong to the category of secretory protein and/or membrane protein are the following 551 clones.
3NB692004045, ADIPS2000069, ADRGL2010315, ASTRO2015162, BLADE2001031, BLADE2002744, BLADE2007744, BRACE2003628, BRACE2012528, BRACE2013126, BRACE2017397, BRACE2017580, BRACE2017992, BRACE2023633, BRACE2030039, BRACE2035191, BRACE3001403, BRACE3001973, BRACE3002264, BRACE3002756, BRACE3004767, BRACE3004981, BRACE3007869, BRACE3009392, BRACE3013874, BRACE3013986, BRACE3014523, BRACE3015898, BRACE3018083, BRACE3021517, BRACE3021805, BRACE3022051, BRACE3024379, BRACE3024444, BRACE3024497, BRACE3024879, BRACE3026345, BRACE3026456, BRACE3026802, BRACE3028360, BRACE3029021, BRACE3030538, BRACE3031372, BRACE3031579, BRACE3031728, BRACE3032385, BRACE3032537, BRACE3032980, BRACE3033525, BRACE3034964, BRACE3034993, BRACE3037637, BRACE3037803, BRACE3038570, BRACE3039358, BRACE3039378, BRACE3040644, BRACE3040863, BRACE3042326, BRACE3042409, BRACE3042432, BRACE3044090, BRACE3046049, BRACE3046466, BRACE3048565, BRACE3050504, BRACE3051144, BRACE3051621, BRACE3052486, BRALZ2010842, BRALZ2011337, BRALZ2013690, BRAMY2015516, BRAMY2021098, BRAMY2025495, BRAMY2037609, BRAMY2041507, BRAMY2044686, BRAMY2046537, BRAMY3002886, BRAMY3004126, BRAMY3007449, BRAMY3009556, BRAMY3009904, BRAMY3010654, BRAMY3010902, BRAMY3015549, BRAMY3016829, BRAMY3018248, BRAWH2000256, BRAWH2010364, BRAWH2011812, BRAWH2011958, BRAWH2012866, BRAWH2014053, BRAWH2016209, BRAWH2016305, BRAWH3001053, BRAWH3001783, BRAWH3001833, BRAWH3003573, BRAWH3005892, BRAWH3008867, BRAWH3010461, BRAWH3010657, BRAWH3011907, BRAWH3012005, BRAWH3012662, BRAWH3012779, BRAWH3013049, BRAWH3014609, BRAWH3015175, BRAWH3016123, BRAWH3017259, BRAWH3018063, BRAWH3018548, BRAWH3018969, BRAWH3019529, BRAWH3019820, BRAWH3020200, BRAWH3020884, BRAWH3021012, BRAWH3021641, BRAWH3022347, BRAWH3023156, BRAWH3023274, BRAWH3023415, BRAWH3023421, BRAWH3024186, BRAWH3024242, BRAWH3027574, BRAWH3027880, BRAWH3028223, BRAWH3028754, BRAWH3029806, BRAWH3030810, BRAWH3032298, BRAWH3034114, BRAWH3034134, BRAWH3035914, BRAWH3036270, BRAWH3038055, BRAWH3038324, BRAWH3040711, BRAWH3040900, BRAWH3042132, BRAWH3042772, BRAWH3042996, BRAWH3043498, BRAWH3043623, BRAWH3044151, BRAWH3044676, BRAWH3046196, BRAWH3047063, BRAWH3048374, BRAWH3048724, BRAWH3049068, BRAWH3049544, BRCAN2002662, BRCAN2003269, BRCAN2003814, BRCAN2006051, BRCAN2006955, BRCAN2015402, BRCAN2018269, BRCAN2019653, BRCAN2019907, BRCAN2020234, BRCAN2020412, BRCAN2020972, BRCAN2021325, BRCAN2022126, BRCOC2006164, BRCOC2006639, BRCOC2009638, BRHIP2006921, BRHIP2020930, BRHIP2021929, BRHIP3000859, BRHIP3001878, BRHIP3002000, BRHIP3002124, BRHIP3003063, BRHIP3003306, BRHIP3003395, BRHIP3004774, BRHIP3005801, BRHIP3005944, BRHIP3006950, BRHIP3007195, BRHIP3007424, BRHIP3007960, BRHIP3008320, BRHIP3010289, BRHIP3011269, BRHIP3011831, BRHIP3012185, BRHIP3012357, BRHIP3012997, BRHIP3013078, BRHIP3016032, BRHIP3017146, BRHIP3017558, BRHIP3019956, BRHIP3020733, BRHIP3021019, BRHIP3025795, BRHIP3025844, BRHIP3027160, BRHIP3027191, BRHIP3028742, BRHIP3029530, BRHIP3030230, BRHIP3031733, BRHIP3035222, BRHIP3035754, BRHIP3036715, BRHIP3036936, BRHIP3037810, BRHIP3039430, BRHIP3039509, BRSSN2004710, BRSSN2018218, BRSTN2010089, BRSTN2011688, BRSTN2011899, BRSTN2011961, BRTHA2000969, BRTHA2003759, BRTHA2012189, BRTHA2014647, BRTHA2018304, BRTHA2019726, BRTHA2019743, BRTHA2020400, BRTHA2020566, BRTHA2020721, BRTHA2020781, BRTHA2021212, BRTHA2021440, BRTHA2021450, BRTHA2022914, BRTHA2022968, BRTHA2023437, BRTHA2026311, BRTHA2027250, BRTHA2030036, BRTHA2031917, BRTHA2033155, BRTHA2033320, BRTHA2033469, BRTHA2033683, BRTHA2036055, BRTHA2036295, BRTHA3003225, BRTHA3006593, BRTHA3010135, BRTHA3010540, BRTHA3010717, BRTHA3011194, BRTHA3011998, BRTHA3012265, BRTHA3013882, BRTHA3014835, BRTHA3016616, BRTHA3018623, BRTHA3026161, BRTHA3027820, BRTHA3028505, CHONS2001287, CHONS2001797, CHONS2002419, COLON2004351, COLON2005623, COLON2005735, CTONG2008989, CTONG2020582, CTONG2027150, CTONG3001605, CTONG3002588, CTONG3008223, FCBBF3012443, FEBRA2023498, FEBRA2026977, FEHRT2002708, FEKID2002231, FEKID2002493, FELNG2000720, FELNG2001706, HCHON2009766, HSYRA2004550, JCMLC1000159, JCMLC2000273, JCMLC2002095, JCMLC2002751, KIDNE2004531, KIDNE2015987, KIDNE2017153, LYMPB1000158, LYMPB2002236, LYMPB2002458, LYMPB2002478, MESAN2014624, NETRP2004090, NETRP2004434, NETRP2005282, NETRP2005849, NETRP2008582, NT2RI3001967, NT2RI3005861, NT2RI3005923, NT2RI3009524, NT2RP7019682, NT2RP8001605, NT2RP8003787, NT2RP8008057, OCBBF2000831, OCBBF2004478, OCBBF2007039, OCBBF2009536, OCBBF2018229, OCBBF2018618, OCBBF2036019, OCBBF3003745, OCBBF3007704, OCBBF3021502, OCBBF3022123, OCBBF3022576, OCBBF3023175, OCBBF3023993, OCBBF3025475, OCBBF3025887, OCBBF3026979, OCBBF3028001, PEBLM2003935, PEBLM2005615, PLACE5000522, PLACE6000012, PLACE6010936, PLACE6019674, PLACE7000266, PLACE7000707, PLACE7001759, PLACE7003639, PLACE7006090, PLACE7006498, PLACE7008136, PLACE7011269, PLACE7012111, PLACE7016321, PLACE7016454, PUAEN2000684, SMINT2003641, SPLEN2011252, SPLEN2025012, SPLEN2031004, SPLEN2034551, SPLEN2035615, SPLEN2042051, STOMA2004663, SYNOV4009139, T1ESE2002665, TBAES2005361, TBAES2007428, TESOP2008556, TESTI2003768, TESTI2007490, TESTI2018335, TESTI2022323, TESTI2024267, TESTI2028613, TESTI2036822, TESTI2037085, TESTI2037657, TESTI2037877, TESTI2046188, TESTI2049041, TESTI2052670, TESTI4001037, TESTI4002072, TESTI4002889, TESTI4003602, TESTI4004539, TESTI4004653, TESTI4005399, TESTI4007671, TESTI4010544, TESTI4010721, TESTI4013774, TESTI4014415, TESTI4014932, TESTI4014977, TESTI4017647, TESTI4017854, TESTI4019149, TESTI4021197, TESTI4021377, TESTI4021569, TESTI4022158, TESTI4023096, TESTI4023654, TESTI4024494, TESTI4026680, TESTI4027170, TESTI4028042, TESTI4031173, TESTI4031818, TESTI4032128, TESTI4034973, TESTI4035872, TESTI4035989, TESTI4036012, TESTI4037949, TESTI4038047, TESTI4040559, TESTI4041049, TESTI4043067, TESTI4043371, TESTI4045168, TESTI4046450, TESTI4047119, TESTI4048296, TESTI4048545, TESTI4051015, TESTI4051858, TESTI4052219, TESTI4052430, TESTI4052598, THYMU3002825, THYMU3003007, THYMU3003350, THYMU3008935, THYMU3009755, THYMU3011360, THYMU3013197, THYMU3014173, THYMU3015457, THYMU3015647, THYMU3016518, THYMU3018151, THYMU3019605, THYMU3021404, THYMU3022211, THYMU3022528, THYMU3022668, THYMU3023107, THYMU3023400, THYMU3025118, THYMU3025313, THYMU3025642, THYMU3026306, THYMU3026532, THYMU3026869, THYMU3027540, THYMU3028461, THYMU3029795, THYMU3031878, THYMU3032032, THYMU3033649, THYMU3033754, THYMU3034099, THYMU3034616, THYMU3036310, THYMU3036934, THYMU3036953, THYMU3037192, THYMU3037772, THYMU3038158, THYMU3038167, THYMU3040068, THYMU3040126, THYMU3040146, THYMU3040172, THYMU3040746, THYMU3040816, THYMU3041918, THYMU3042321, THYMU3043688, THYMU3043779, THYMU3044188, THYMU3045510, THYMU3047115, THYMU3047156, THYMU3047542, THYMU3047760, TKIDN2011160, TLIVE2008797, TRACH3003872, TRACH3004747, TRACH3005274, TRACH3005699, TRACH3007274, TRACH3007625, TRACH3009008, TRACH3009061, TRACH3010382, TRACH3011082, TRACH3011184, TRACH3012659, TRACH3012891, TRACH3013900, TRACH3014063, TRACH3014580, TRACH3015136, TRACH3015346, TRACH3016368, TRACH3016885, TRACH3016992, TRACH3017409, TRACH3018191, TRACH3018240, TRACH3018524, TRACH3018943, TRACH3019058, TRACH3019370, TRACH3019621, TRACH3019807, TRACH3020930, TRACH3021023, TRACH3021544, TRACH3022758, TRACH3023063, TRACH3023203, TRACH3023516, TRACH3023945, TRACH3024081, TRACH3024671, TRACH3025346, TRACH3026542, TRACH3027681, TRACH3029670, TRACH3031316, TRACH3031678, TRACH3032480, TRACH3034680, TRACH3036103, TRACH3036278, TSTOM2002682, UTERU3005422, UTERU3010029, UTERU3011092, UTERU3011398, UTERU3011837, UTERU3012414, UTERU3015647, UTERU3016273, UTERU3017626, UTERU3021850, UTERU3022168, UTERU3022922, UTERU3023413

The following 19 clones are also predicted to belong to the category of secretory protein and/or membrane protein.
ADRGL2011190, BRACE3046450, BRAWH3013197, BRAWH3028645, BRAWH3046240, BRTHA3024233, PROST2010326, TBAES2004105, TBAES2007379, TBAES2007481, TBAES2008133, TESTI2043585, TESTI4005158, THYMU3024602, THYMU3044175, THYMU3046350, TLIVE2007192, UTERU2025415, UTERU2036507

The clones predicted to belong to the category of glycoprotein-related protein are the following 114 clones.
3NB692004045, ADIPS2000069, BRACE2017397, BRACE3001403, BRACE3001973, BRACE3002264, BRACE3009392, BRACE3026345, BRACE3032385, BRACE3039358, BRACE3039378, BRACE3042432, BRACE3046466, BRACE3051621, BRAMY3004126, BRAWH2012866, BRAWH3001783, BRAWH3003573, BRAWH3014609, BRAWH3023156, BRAWH3024186, BRAWH3029806, BRAWH3040900, BRAWH3043623, BRAWH3044151, BRAWH3049544, BRCAN2003269, BRCAN2021325, BRHIP3005944, BRHIP3007424, BRHIP3010289, BRHIP3011269, BRHIP3011567, BRHIP3030230, BRHIP3036715, BRHIP3036936, BRHIP3039509, BRTHA2019726, BRTHA2020721, BRTHA2022968, BRTHA2025869, BRTHA2027250, BRTHA2031917, BRTHA2033155, BRTHA2033683, BRTHA3010135, CHONS2001287, COLON2004351, FEKID2002493, FELNG2000720, JCMLC1000159, JCMLC2000273, JCMLC2002095, JCMLC2002751, KIDNE2015987, LYMPB2002458, NT2RI3005923, NT2RI3009524, OCBBF2000831, OCBBF2004478, OCBBF2007039, OCBBF2018618, OCBBF3026979, PEBLM2005615, PLACE6001933, PLACE6010936, PLACE7006090, PLACE7012111, SPLEN2025012, STOMA2004663, T1ESE2002665, TESTI2007490, TESTI2022323, TESTI2037657, TESTI2052670, TESTI4001517, TESTI4014932, TESTI4031173, THYMU3014173, THYMU3015647, THYMU3016518, THYMU3020221, THYMU3025118, THYMU3026532, THYMU3032032, THYMU3037772, THYMU3040172, TKIDN2011160, TLUNG2001445, TRACH3005274, TRACH3009061, TRACH3015136, TRACH3018524, TRACH3018907, TRACH3019058, TRACH3019370, TRACH3019621, TRACH3019807, TRACH3020930, TRACH3021023, TRACH3023516, TRACH3025346, TRACH3026299, TRACH3028441, TRACH3029670, TRACH3031678, TRACH3034680, TRACH3037505, TRACH3038399, TUTER2001433, UTERU3011398, UTERU3011837, UTERU3015647, UTERU3021850

The following ten clones are also predicted to belong to the category of glycoprotein-related protein.
BRAWH3013197, BRAWH3046240, PROST2010326, TBAES2004105, TESTI2043585, TESTI4005158, THYMU3024602, THYMU3046350, TRACH3021066, UTERU2036507

The clones predicted to belong to the category of signal transduction-related protein are the following 71 clones.
BRACE3002344, BRACE3017253, BRACE3031315, BRACE3036283, BRACE3042046, BRACE3044172, BRACE3046491, BRACE3046609, BRAMY3009491, BRAMY3015547, BRAMY3017920, BRAWH3017180, BRAWH3019026, BRAWH3027806, BRAWH3032340, BRAWH3042438, BRAWH3047644, BRCAN2010665, BRHIP3006294, BRHIP3011460, BRHIP3011567, BRHIP3033557, BRHIP3037543, BRHIP3041587, BRTHA2026290, BRTHA2035743, BRTHA3011187, BRTHA3021708, BRTHA3025073, BRTHA3026916, KIDNE2010049, N1ESE2000698, OCBBF3005330, OCBBF3006986, OCBBF3009244, OCBBF3025630, PLACE6000055, PLACE6001933, PLACE7009936, PLACE7011559, PLACE7014247, PUAEN2006639, SKMUS2008585, SPLEN2007689, TESTI2021654, TESTI2040377, TESTI4010902, TESTI4013474, TESTI4046073, TESTI4049786, TESTI4051865, THYMU3013785, THYMU3025683, THYMU3032798, TRACH3003037, TRACH3003357, TRACH3005173, TRACH3018519, TRACH3018606, TRACH3024020, TRACH3026650, TRACH3027701, TRACH3029462, TRACH3030176, TRACH3038399, TSTOM2001571, TSTOM2002611, UTERU2024042, UTERU3001029, UTERU3010919, UTERU3021231

The following four clones are also predicted to belong to the category of signal transduction-related protein.
BRHIP3030064, CTONG2003764, PLACE7013963, TRACH3021066

The clones predicted to belong to the category of transcription-related protein are the following 106 clones.
ASTRO2016114, BEAST2000981, BRACE2012947, BRACE2019348, BRACE3025719, BRACE3026844, BRACE3034183, BRACE3041162, BRACE3046152, BRAMY2040915, BRAMY3000692, BRAMY3007078, BRAMY3011581, BRAMY3014027, BRAMY3018754, BRAWH3000446, BRAWH3005886, BRAWH3011577, BRAWH3013009, BRAWH3013264, BRAWH3017477, BRAWH3023172, BRAWH3028796, BRAWH3031342, BRAWH3032571, BRAWH3035936, BRAWH3036247, BRAWH3036334, BRAWH3038827, BRCOC2012386, BRHIP2023735, BRHIP2027077, BRHIP2029529, BRHIP3004725, BRHIP3027651, BRHIP3028246, BRTHA2024712, BRTHA3000456, BRTHA3003736, BRTHA3010212, BRTHA3014000, BRTHA3028339, CHONS2000797, CHONS2002829, CTONG2001932, CTONG2011801, D9OST2003106, FCBBF3020030, FCBBF5000384, HCASM2008154, NETRP2004017, NT2RI3008179, NT2RI3009480, NT2RP8003490, NTONG2003805, NTONG2008483, OCBBF2016928, OCBBF3005330, OCBBF3008392, OCBBF3020263, OCBBF3021361, OCBBF3022166, PLACE7002303, PLACE7005169, PLACE7009757, SPLEN2012571, SPLEN2028417, SYNOV2003326, T1ESE2000904, TESTI2040377, TESTI4001679, TESTI4002799, TESTI4002868, TESTI4003796, TESTI4003944, TESTI4005322, TESTI4005470, TESTI4039904, TESTI4052775, THYMU3008105, THYMU3012983, THYMU3014372, THYMU3020869, THYMU3021586, THYMU3022434, THYMU3026000, THYMU3030072, THYMU3037052, THYMU3043200, TLIVE2001616, TRACH3003037, TRACH3003458, TRACH3004424, TRACH3010079, TRACH3010167, TRACH3010342, TRACH3015951, TRACH3021883, TRACH3022109, TRACH3028180, TRACH3036750, UTERU2037423, UTERU3012293, UTERU3016070, UTERU3019708, UTERU3022588

The following seven clones are also predicted to belong to the category of transcription-related protein.
BRACE3002184, BRCAN2019772, CTONG2002832, TESTI4002988, TESTI4005500, TRACH1000193, TRACH3019290

The clones predicted to belong to the category of disease-related protein are the following 391 clones.
ADIPS2000069, ASTRO2015162, ASTRO2016114, ASTRO3000154, BLADE2000256, BRACE1000475, BRACE2012838, BRACE2012947, BRACE2013009, BRACE2016896, BRACE2017397, BRACE2023744, BRACE2027382, BRACE3001403, BRACE3001973, BRACE3002756, BRACE3004767, BRACE3009392, BRACE3013418, BRACE3018083, BRACE3019941, BRACE3020669, BRACE3025719, BRACE3026345, BRACE3026802, BRACE3028998, BRACE3036283, BRACE3039378, BRACE3040644, BRACE3041059, BRACE3041162, BRACE3042046, BRACE3042432, BRACE3043597, BRACE3044172, BRACE3046152, BRACE3046466, BRACE3046609, BRACE3051621, BRACE3052321, BRALZ2010842, BRALZ2013621, BRAMY2041384, BRAMY3000692, BRAMY3004126, BRAMY3007078, BRAMY3009491, BRAMY3011501, BRAMY3011581, BRAMY3014027, BRAMY3015086, BRAMY3017920, BRAMY3018248, BRAWH2002333, BRAWH2012866, BRAWH2014053, BRAWH3001638, BRAWH3001783, BRAWH3004335, BRAWH3010602, BRAWH3011577, BRAWH3011623, BRAWH3017180, BRAWH3017259, BRAWH3018548, BRAWH3019026, BRAWH3021580, BRAWH3023156, BRAWH3023172, BRAWH3023415, BRAWH3024186, BRAWH3029385, BRAWH3029538, BRAWH3031342, BRAWH3032298, BRAWH3032571, BRAWH3033513, BRAWH3034668, BRAWH3034775, BRAWH3034890, BRAWH3036334, BRAWH3038324, BRAWH3038827, BRAWH3040900, BRAWH3041492, BRAWH3041556, BRAWH3042438, BRAWH3042447, BRAWH3042772, BRAWH3043295, BRAWH3043623, BRAWH3044151, BRAWH3046424, BRAWH3047565, BRAWH3047644, BRAWH3049544, BRCAN2003269, BRCAN2006051, BRCAN2010665, BRCAN2020331, BRCAN2021325, BRCOC2012386, BRHIP2008756, BRHIP2023735, BRHIP2029529, BRHIP3001076, BRHIP3001481, BRHIP3003984, BRHIP3004215, BRHIP3004725, BRHIP3005037, BRHIP3005307, BRHIP3005673, BRHIP3005801, BRHIP3006449, BRHIP3007609, BRHIP3010289, BRHIP3011567, BRHIP3017146, BRHIP3017855, BRHIP3021019, BRHIP3023922, BRHIP3025795, BRHIP3027191, BRHIP3027651, BRHIP3028742, BRHIP3029409, BRHIP3030230, BRHIP3032374, BRHIP3035006, BRHIP3036715, BRHIP3037543, BRHIP3039509, BRSSN2004710, BRSTN2006466, BRSTN2008475, BRSTN2011961, BRSTN2012069, BRSTN2016918, BRTHA2019726, BRTHA2020721, BRTHA2020910, BRTHA2024712, BRTHA2025869, BRTHA2026071, BRTHA2026290, BRTHA2031917, BRTHA2033155, BRTHA2033683, BRTHA3003736, BRTHA3010135, BRTHA3010212, BRTHA3011187, BRTHA3011998, BRTHA3012265, BRTHA3014547, BRTHA3021708, BRTHA3021971, BRTHA3023403, BRTHA3026916, BRTHA3027957, CHONS2001287, CHONS2002829, COLON2001829, COLON2004911, COLON2005735, CTONG2001932, CTONG2010330, CTONG2011801, CTONG2014206, D9OST2004417 FCBBF3020030, FCBBF3021191, FCBBF3024911, FCBBF5000384, FEBRA2013570, FEBRA2026582, FEBRA2028457, FEKID2002637, FELNG2000720, FELNG2001953, HCASM2008154, JCMLC1000159, JCMLC2000273, JCMLC2002095, KIDNE2015987, N1ESE2000698, NETRP2000961, NETRP2003448, NETRP2004017, NETRP2008582, NT2RI3008179, NT2RI3009480, NT2RI3009524, NT2RP8003787, NT2RP8008057, NTONG2003805, OCBBF2004478, OCBBF2007039, OCBBF2018618, OCBBF2024589, OCBBF2030927, OCBBF2036019, OCBBF3001202, OCBBF3004487, OCBBF3008392, OCBBF3020263, OCBBF3022166, OCBBF3025475, OCBBF3025503, OCBBF3025630, OCBBF3026979, PEBLM2005615, PLACE5000492, PLACE6001933, PLACE6016030, PLACE7000266, PLACE7001759, PLACE7002303, PLACE7003985, PLACE7004103, PLACE7006090, PLACE7006268, PLACE7006498, PLACE7007379, PLACE7009563, PLACE7009757, PLACE7009936, PLACE7011559, PLACE7012111, PLACE7014247, PLACE7016526, PUAEN2000594, SKNSH2007306, SMINT2011406, SMINT2011509, SMINT2014721, SPLEN2007689, SPLEN2012571, SPLEN2025012, SPLEN2028417, SPLEN2033996, SYNOV2003326, SYNOV4009139, T1ESE2000609, TESTI2005112, TESTI2007490, TESTI2009739, TESTI2023903, TESTI2030901, TESTI2034913, TESTI2052670, TESTI4001517, TESTI4001679, TESTI4002868, TESTI4003796, TESTI4003944, TESTI4004653, TESTI4005322, TESTI4005653, TESTI4007965, TESTI4017382, TESTI4017647, TESTI4018436, TESTI4020596, TESTI4021197, TESTI4021569, TESTI4021713, TESTI4023096, TESTI4026080, TESTI4028182, TESTI4031173, TESTI4032128, TESTI4032834, TESTI4032913, TESTI4033177, TESTI4036048, TESTI4039575, TESTI4039904, TESTI4041984, TESTI4046073, TESTI4047119, TESTI4049786, TESTI4049899, TESTI4051015, TESTI4052775, THYMU3002825, THYMU3008105, THYMU3012402, THYMU3012983, THYMU3013785, THYMU3014173, THYMU3014372, THYMU3014620, THYMU3016518, THYMU3020221, THYMU3020869, THYMU3021586, THYMU3021755, THYMU3022434, THYMU3023400, THYMU3025118, THYMU3026306, THYMU3026532, THYMU3027671, THYMU3032032, THYMU3032798, THYMU3033649, THYMU3033759, THYMU3037052, THYMU3037772, THYMU3038158, THYMU3038375, THYMU3040172, THYMU3040746, THYMU3040816, THYMU3040829, THYMU3043200, THYMU3047115, THYMU3047760, TKIDN2011160, TLIVE2007736, TLUNG2000654, TLUNG2001445, TLUNG2001600, TRACH2024730, TRACH3004424, TRACH3005173, TRACH3005191, TRACH3005699, TRACH3006379, TRACH3006800, TRACH3008042, TRACH3009008, TRACH3009701, TRACH3010079, TRACH3010167, TRACH3010342, TRACH3011282, TRACH3011313, TRACH3011503, TRACH3012891, TRACH3015951, TRACH3016455, TRACH3016805, TRACH3018524, TRACH3018907, TRACH3019058, TRACH3019621, TRACH3020769, TRACH3020930, TRACH3021023, TRACH3021373, TRACH3021778, TRACH3021883, TRACH3023373, TRACH3023960, TRACH3024081, TRACH3024671, TRACH3025346, TRACH3026283, TRACH3026299, TRACH3028441, TRACH3028597, TRACH3028837, TRACH3029670, TRACH3030855, TRACH3031660, TRACH3031678, TRACH3032570, TRACH3034680, TRACH3036750, TRACH3037505, TRACH3038399, TUTER2001433, UTERU2024042, UTERU2037423, UTERU3001946, UTERU3004635, UTERU3011398, UTERU3012293, UTERU3012414, UTERU3012999, UTERU3015011, UTERU3015299, UTERU3016308, UTERU3017441, UTERU3017626, UTERU3019708, UTERU3021850, UTERU3022588

The following 23 clones are also predicted to belong to the category of disease-related protein.
ADRGL2011190, BRACE3002184, BRACE3026993, BRACE3046450, BRAWH3013197, BRAWH3046240, BRCAN2019772, BRHIP3030064, BRTHA3024233, CTONG2002832, CTONG2003764, PROST2010326, TBAES2004105, TESTI2046536, TESTI4005158, TESTI4005500, THYMU3024602, THYMU3046350, TRACH1000193, TRACH3019290, TRACH3021066, UTERU2017492, UTERU2036507

In particular, hit data of the following 390 clones for Swiss-Prot, or nr or RefSeq corresponded to genes or proteins which had been deposited in the Online Mendelian Inheritance in Man (OMIM), which is the human gene and disease database (the OMIM Number is shown in the parenthesis after the Clone Name).
ADIPS2000069(146900), ASTRO2015162(606106), ASTRO2016114(603899), ASTRO3000154(601594), BLADE2000256(140750), BRACE1000475(600696), BRACE2012838 (605032) , BRACE2012947 (140580) , BRACE2013009(605888), BRACE2016896(601421), BRACE2017397(115437), BRACE2023744(600763), BRACE2027382 (606019) , BRACE3001403(126141), BRACE3001973(600976), BRACE3002756 (603143) , BRACE3004767 (182790) , BRACE3009392 (600229) , BRACE3013418(182900), BRACE3018083 (605268) ,
BRACE3019941(600595), BRACE3020669(603917), BRACE3025719 (605493) , BRACE3026345(147470), BRACE3026802(605784), BRACE3028998 (603063) , BRACE3036283 (602052) , BRACE3039378 (604100) , BRACE3040644 (603159) , BRACE3041059 (603486) , BRACE3041162 (194556) , BRACE3042046 (311030) , BRACE3042432(192321), BRACE3043597(603704), BRACE3044172(601231), BRACE3046152(604950), BRACE3046466(604210;600105),
BRACE3046609(606457), BRACE3051621(601313;173900),
BRACE3052321(603050),
BRALZ2010842 (212138), BRALZ2013621 (600712) , BRAMY2041384 (114070) , BRAMY3000692(603971), BRAMY3004126(603071), BRAMY3007078(602410),
BRAMY3009491 (600286) , BRAMY3011501(602869), BRAMY3011581 (601243), BRAMY3014027(194542), BRAMY3015086(602879), BRAMY3017920(600365), BRAMY3018248(605464), BRAWH2002333(171891), BRAWH2012866(185605), BRAWH2014053(604581), BRAWH3001638(605003) , BRAWH3001783(605514), BRAWH3004335 (603244) , BRAWH3010602(603216),
BRAWH3011577(601139), BRAWH3011623(164020), BRAWH3017180(601441), BRAWH3017259(603143), BRAWH3018548(193065), BRAWH3019026(602033), BRAWH3021580(179838), BRAWH3023156(137190), BRAWH3023172(603755), BRAWH3023415(604346), BRAWH3024186(179590), BRAWH3029385(602378), BRAWH3029538(600948), BRAWH3031342(603971) , BRAWH3032298(601995), BRAWH3032571 (603277) , BRAWH3033513 (604054;261510) ,
BRAWH3034668 (603486) , BRAWH3034775(605800), BRAWH3034890 (606265) , BRAWH3036334 (603971) , BRAWH3038324 (604249) , BRAWH3038827 (600574) , BRAWH3040900 (604265) , BRAWH3041492 (130500) , BRAWH3041556 (172460) , BRAWH3042438 (125855) , BRAWH3042447 (606323) , BRAWH3042772 (602878) , BRAWH3043295 (179030) , BRAWH3043623 (600976) , BRAWH3044151 (605421) , BRAWH3046424 (300272) , BRAWH3047565 (606277) , BRAWH3047644 (605216) , BRAWH3049544 (602273) , BRCAN2003269 (171060;602347) ,
BRCAN2006051 (604581) , BRCAN2010665 (603583) , BRCAN2020331 (604851) , BRCAN2021325 (114855) , BRCOC2012386 (602277) , BRHIP2008756 (605819) , BRHIP2023735 (601670) , BRHIP2029529 (189972) , BRHIP3001076 (604673) , BRHIP3001481(176889), BRHIP3003984(603722;223900),
BRHIP3004215 (603294) , BRHIP3004725 (602075) , BRHIP3005037 (603526) , BRHIP3005307(603197), BRHIP3005673 (138385) , BRHIP3005801 (605704) , BRHIP3006449 (604275) , BRHIP3007609 (426000) , BRHIP3010289 (603130), BRHIP3011567 (114207) , BRHIP3017146 (602878) , BRHIP3017855 (606406) , BRHIP3021019 (176879) , BRHIP3023922(156570;250940),
BRHIP3025795 (603877) , BRHIP3027191 (601746) , BRHIP3027651 (604589) , BRHIP3028742 (602076) , BRHIP3029409 (604156) , BRHIP3030230 (602367) , BRHIP3032374 (603197) , BRHIP3035006 (604402) , BRHIP3036715 (142800) , BRHIP3037543 (602052), BRHIP3039509(601328), BRSSN2004710(600127), BRSTN2006466 (138275), BRSTN2008475 (605178) , BRSTN2011961 (176790) , BRSTN2012069 (130590), BRSTN2016918 (137780) , BRTHA2019726 (147100), BRTHA2020721 (147100) , BRTHA2020910 (602661), BRTHA2024712 (600747), BRTHA2025869 (162280) , BRTHA2026071 (605297) , BRTHA2026290 (602306), BRTHA2031917 (118946) , BRTHA2033155 (601873) , BRTHA2033683 (111000), BRTHA3003736(133510;234050), BRTHA3010135(179590),
BRTHA3010212(603971), BRTHA3011187(605837), BRTHA3011998(603264), BRTHA3012265(605646), BRTHA3014547(182900), BRTHA3021708(602654), BRTHA3021971(605609), BRTHA3023403(600597), BRTHA3026916 (601619) , BRTHA3027957(606078), CHONS2001287(146732), CHONS2002829(602981), COLON2001829(604399), COLON2004911(603937;180100),
COLON2005735(111690;111700), CTONG2001932(605683),
CTONG2010330(606088), CTONG2011801(603971), CTONG2014206(605609), D9OST2004417(113703), FCBBF3020030(603406), FCBBF3021191 (605119) , FCBBF5000384(601737), FEBRA2013570(248600), FEBRA2026582(300252), FEBRA2028457(164035), FEKID2002637(176875), FELNG2000720(601662), FELNG2001953(603597),
HCASM2008154(133450), JCMLC1000159 (107470;209950),
JCMLC2000273(120980), JCMLC2002095 (600738) , KIDNE2015987 (191845) , N1ESE2000698(604734), NETRP2000961(600417), NETRP2003448(179551), NETRP2004017(605344), NETRP2008582(103195), NT2RI3008179(603808), NT2RI3009480(601804), NT2RI3009524(604210;600105),
NT2RP8003787(605427), NT2RP8008057(603489), NTONG2003805(601781), OCBBF2004478 (604265) , OCBBF2007039(605009), OCBBF2018618(102775), OCBBF2024589(602462),
OCBBF2030927(603897), OCBBF2036019(601825;256000),
OCBBF3001202(140750), OCBBF3004487(142560), OCBBF3008392(605682), OCBBF3020263(604077), OCBBF3022166(600848), OCBBF3025475(604148), OCBBF3025503(601653;113650), OCBBF3025630(604141),
OCBBF3026979(602319), PEBLM2005615 (600242) , PLACE5000492(602142), PLACE6001933(131550), PLACE6016030(605442), PLACE7000266(188840), PLACE7001759(600338), PLACE7002303(601542;180500;137600;604229),
PLACE7003985(109684), PLACE7004103(142695),
PLACE7006090(154360), PLACE7006268(603053), PLACE7006498(604394), PLACE7007379(603105), PLACE7009563(300344), PLACE7009757(601804), PLACE7009936(600365), PLACE7011559(600831), PLACE7012111(602714), PLACE7014247(601232), PLACE7016526(605490), PUAEN2000594(604679), SKNSH2007306(118990), SMINT2011406(147890), SMINT2011509(606343), SMINT2014721(606090), SPLEN2007689(233700), SPLEN2012571(603430), SPLEN2025012(146900), SPLEN2028417(142995),
SPLEN2033996(603853), SYNOV2003326(602960), SYNOV4009139(603551),
T1ESE2000609(182465), TESTI2005112(603846), TESTI2007490(601291), TESTI2009739(160745), TESTI2023903 (605046) , TESTI2030901(600436), TESTI2034913(148060), TESTI2052670(142461), TESTI4001517(148070), TESTI4001679(602850), TESTI4002868(601863;209920),
TESTI4003796(603132), TESTI4003944(603971), TESTI4004653(606106), TESTI4005322(603899), TESTI4005653(182465), TESTI4007965(603533), TESTI4017382(605689), TESTI4017647(603211), TESTI4018436(601754), TESTI4020596(602537), TESTI4021197(602189), TESTI4021569(605464), TESTI4021713(604105), TESTI4023096(604878), TESTI4026080(605575), TESTI4028182(603892), TESTI4031173(190197), TESTI4032128(104776), TESTI4032834 (300188) , TESTI4032913 (106410) , TESTI4033177 (602038) , TESTI4036048(601272), TESTI4039575(600951), TESTI4039904(603899), TESTI4041984(604710), TESTI4046073 (300118;309801) ,
TESTI4047119(606202), TESTI4049786 (142600;235700) ,
TESTI4049899(601969), TESTI4051015(602974), TESTI4052775(165250), THYMU3002825(604346), THYMU3008105(194548), THYMU3012402(600686), THYMU3012983(194556), THYMU3013785(604722), THYMU3014173(143010), THYMU3014372(116945), THYMU3014620(605657), THYMU3016518(147100), THYMU3020221 (147100) , THYMU3020869(602550), THYMU3021586(184756), THYMU3021755(605024), THYMU3022434(601408), THYMU3023400(605180), THYMU3025118(155735), THYMU3026306(604346),
THYMU3026532 (600065;116920) , THYMU3027671(604143),
THYMU3032032(604463), THYMU3032798(601212), THYMU3033649(186780), THYMU3033759(600495), THYMU3037052(300346), THYMU3037772(147100), THYMU3038158(603033;603034), THYMU3038375(181590),
THYMU3040172(186720), THYMU3040746(147110), THYMU3040816(605704), THYMU3040829(602649), THYMU3043200(605596), THYMU3047115(108730), THYMU3047760(604783), TKIDN2011160(605011),
TLIVE2007736(604990), TLUNG2000654(148059), TLUNG2001445(146900), TLUNG2001600(147130), TRACH2024730(605611), TRACH3004424(603971), TRACH3005173(151410), TRACH3005191(605333), TRACH3005699(606154), TRACH3006379(148059), TRACH3006800(606154), TRACH3008042 (166945) , TRACH3009008 (601112) , TRACH3009701 (603330) , TRACH3010079(604850), TRACH3010167(601804), TRACH3010342(602943), TRACH3011282(601833), TRACH3011313(113520), TRACH3011503(602862),
TRACH3012891(602397), TRACH3015951(604084), TRACH3016455(605286),
TRACH3016805(106410), TRACH3018524(176882), TRACH3018907(146900), TRACH3019058(147170), TRACH3019621(191350), TRACH3020769(160776), TRACH3020930(147100), TRACH3021023(147170), TRACH3021373(606030), TRACH3021778(164035), TRACH3021883(603347), TRACH3023373(159350), TRACH3023960(603337), TRACH3024081(605867), TRACH3024671(605942), TRACH3025346(603377;212140), TRACH3026283(601517),
TRACH3026299(147170), TRACH3028441(147170), TRACH3028597(604310), TRACH3028837(602127), TRACH3029670(147170), TRACH3030855(173321), TRACH3031660(176912), TRACH3031678(600523), TRACH3032570(602217), TRACH3034680(147170), TRACH3036750(604077), TRACH3037505(147170), TRACH3038399(604032;226980), TUTER2001433(146900),
UTERU2024042(602214), UTERU2037423(604077), UTERU3001946(606154), UTERU3004635(103390), UTERU3011398(120240;158810;254090),
UTERU3012293(194556),
UTERU3012414(604394), UTERU3012999(605567), UTERU3015011(602505), UTERU3015299(601825;256000), UTERU3016308(602127),
UTERU3017441 (604276) , UTERU3017626 (603788) , UTERU3019708(601430), UTERU3021850(605009), UTERU3022588(123811)

Additionally, hit data of the following 23 clones for Swiss-Prot, nr or RefSeq corresponded to genes or proteins which had been deposited in the Online Mendelian Inheritance in Man (OMIM), which is the human gene and disease database (the OMIM Number is shown in the parenthesis after the Clone Name).
ADRGL2011190 (602658), BRACE3002184 (603899), BRACE3026993 (601282), BRACE3046450 (603035), BRAWH3013197 (604100), BRAWH3046240 (602646), BRCAN2019772 (603849), BRHIP3030064 (605762), BRTHA3024233 (603088), CTONG2002832 (604668), CTONG2003764 (176977), PROST2010326 (604110), TBAES2004105 (605008), TESTI2046536 (182890; 182882), TESTI4005158 (601328), TESTI4005500 (602277), THYMU3024602 (600493), THYMU3046350 (603080), TRACH1000193 (139139), TRACH3019290 (603975), TRACH3021066 (164870), UTERU2017492 (602917), UTERU2036507 (605008)

The clones predicted to belong to the category of enzyme and/or metabolism-related protein are the following 164 clones. ASTRO2008972, BRACE1000475, BRACE2013132, BRACE2016896, BRACE2035120, BRACE3017253, BRACE3021805, BRACE3028998, BRACE3031315, BRACE3036283, BRACE3041059, BRACE3042409, BRACE3044172, BRACE3046609, BRAMY3009491, BRAMY3011581, BRAWH2002333, BRAWH2014053, BRAWH3001638, BRAWH3004335, BRAWH3011331, BRAWH3017180, BRAWH3020928, BRAWH3023415, BRAWH3023421, BRAWH3024186, BRAWH3024506, BRAWH3029385, BRAWH3029806, BRAWH3032571, BRAWH3033513, BRAWH3034668, BRAWH3037428, BRAWH3037979, BRAWH3041556, BRAWH3042438, BRAWH3043295, BRAWH3044151, BRAWH3046424, BRAWH3047692, BRAWH3049544, BRCAN2003814, BRCAN2006051, BRCAN2015402, BRCAN2021325, BRHIP3001481, BRHIP3003126, BRHIP3005307, BRHIP3005673, BRHIP3007195, BRHIP3007223, BRHIP3011082, BRHIP3011269, BRHIP3021019, BRHIP3023922, BRHIP3032374, BRHIP3035006, BRHIP3037543, BRHIP3041587, BRSSN2004710, BRSTN2006466, BRSTN2011961, BRTHA2005448, BRTHA2010672, BRTHA2025869, BRTHA2026311, BRTHA2033155, BRTHA2035743, BRTHA3003736, BRTHA3010135, BRTHA3010469, BRTHA3023403, CHONS2002829, COLON2004351, CTONG2010330, CTONG2020582, CTONG3001605, FCBBF3001018, FCBBF3021191, FCBBF5000384, FEBRA2013570, FEBRA2026582, FEKID2002637, HSYRA2004550, KIDNE2010049, NETRP2000961, NT2RI2004818, NT2RP7016508, OCBBF2007039, OCBBF2024589, OCBBF2036019, OCBBF3004487, OCBBF3005330, OCBBF3009244, PLACE5000492, PLACE6001933, PLACE7001759, PLACE7003985, PLACE7004103, PLACE7004961, PLACE7006090, PLACE7006268, PLACE7007379, PLACE7011559, PLACE7012111, PLACE7014247, PLACE7016526, SKMUS2008585, SMINT2011509, SMINT2012179, SMINT2014721, SPLEN2007689, SYNOV4009139, TBAES2007428, TESTI2005112, TESTI2007490, TESTI2021654, TESTI2040377, TESTI2049062, TESTI4000621, TESTI4002799, TESTI4007671, TESTI4020596, TESTI4033177, TESTI4049786, TESTI4052219, THYMU3002825, THYMU3026306, THYMU3032798, THYMU3034671, THYMU3036953, THYMU3041428, THYMU3047115, THYMU3047891, TKIDN2011160, TRACH3005173, TRACH3005274, TRACH3009008, TRACH3011313, TRACH3011503, TRACH3012891, TRACH3015136, TRACH3016455, TRACH3018108, TRACH3018261, TRACH3018524, TRACH3019621, TRACH3021544, TRACH3022758, TRACH3023516, TRACH3024020, TRACH3024081, TRACH3027229, TRACH3027701, TRACH3032150, TRACH3038399, TSTOM2001571, TSTOM2002611, UTERU2024042, UTERU3010604, UTERU3010919, UTERU3015299, UTERU3019708, UTERU3021850

The following eight clones are also predicted to belong to the category of enzyme and/or metabolism-related protein.
ADRGL2011190, BRHIP3030064, CTONG2003764, PLACE7013963, TBAES2004105, THYMU3044175, TRACH3021066, UTERU2036507

The clones predicted to belong to the category of cell division and/or cell proliferation-related protein are the following 27 clones.
BLADE2000256, BRACE2002392, BRACE3030538, BRACE3036283, BRACE3044495, BRAMY3002886, BRAMY3009556, BRAWH2016209, BRAWH3004350, BRAWH3027574, BRCAN2019907, BRHIP3001076, BRHIP3029409, BRSTN2008475, BRTHA3011265, FEKID2002637, NT2RP8005546, OCBBF3001202, PLACE7011559, SPLEN2033996, TESTI2023903, TESTI4020819, TESTI4049899, THYMU3014372, THYMU3021586, UTERU3010919, UTERU3012999

The following two clones are also predicted to belong to the category of cell division and/or cell proliferation-related protein.
TBAES2007481, TESTI2046536

The clones predicted to belong to the category of cytoskeleton-related protein are the following 60 clones.
BRACE3004767, BRACE3013418, BRACE3051819, BRAMY3005184, BRAMY3015086, BRAMY4000915, BRAMY4001652, BRAWH3001783, BRAWH3015175, BRAWH3018548, BRAWH3019026, BRAWH3021580, BRAWH3021724, BRAWH3027440, BRAWH3027806, BRAWH3029385, BRAWH3040900, BRAWH3041492, BRCAN2020467, BRHIP3003063, BRHIP3003340, BRSTN2016918, BRTHA2020910, BRTHA2025869, BRTHA3014547, BRTHA3025073, CERVX2000968, JCMLC2000273, N1ESE2000698, NT2RI3005923, OCBBF2003518, OCBBF2004478, OCBBF3027969, PLACE7000266, PLACE7004961, SMINT2010753, SPLEN2034934, SYNOV4003174, TESTI2001915, TESTI2009739, TESTI2034913, TESTI4001517, TESTI4004917, TESTI4010902, TESTI4032913, TESTI4051424, THYMU3026532, TLUNG2000654, TRACH3006379, TRACH3016805, TRACH3020769, TRACH3022960, TRACH3026650, TRACH3028837, TRACH3029462, TRACH3032570, UTERU3000670, UTERU3001029, UTERU3015011, UTERU3016308

The following one clone is also predicted to belong to the category of cytoskeleton-related protein.
BRACE3026993

The clones predicted to belong to the category of nuclear protein and/or RNA synthesis-related protein are the following 40 clones.
ASTRO3000154, BRACE3014714, BRACE3036283, BRALZ2013621, BRAMY3009556, BRAMY3011501, BRAWH3011623, BRAWH3017180, BRAWH3022651, BRAWH3038252, BRAWH3040695, BRAWH3046424, BRHIP3004215, BRHIP3007223, BRHIP3020046, BRTHA3010530, CTONG2006235, FEBRA2028457, NT2RP7016508, OCBBF3001333, OCBBF3004487, PLACE7004103, PLACE7006268, TESTI2036285, TESTI2037657, TESTI4014932, TESTI4028182, TESTI4032128, TESTI4033177, TESTI4039575, THYMU3012402, THYMU3040829, THYMU3041428, TRACH3002752, TRACH3018108, TRACH3021778, UTERU3004635, UTERU3010409, UTERU3010919, UTERU3013302

The following one clone is also predicted to belong to the category of nuclear protein and/or RNA synthesis-related protein.
TESTI2046536

The clones predicted to belong to the category of protein synthesis and/or transport-related protein are the following 50 clones.
BRACE2016896, BRACE2023744, BRACE3020669, BRACE3030538, BRACE3041059, BRACE3043597, BRAWH2014053, BRAWH3001638, BRAWH3010602, BRAWH3024506, BRAWH3026349, BRAWH3034668, BRAWH3037979, BRAWH3041556, BRAWH3044151, BRAWH3047692, BRCAN2000923, BRCAN2002892, BRCAN2006051, BRCAN2021325, BRHIP3007223, BRSTN2012069, BRTHA2005448, BRTHA2010672, CHONS2002829, CTONG3001605, D9OST2004417 OCBBF2000831, OCBBF2007039, PLACE6019600, PLACE7007379, PLACE7012111, PLACE7016526, TESTI4018436, TESTI4020596, TESTI4032128, TESTI4036048, THYMU3012402, THYMU3033759, THYMU3036953, THYMU3046360, TKIDN2011160, TRACH3016455, TRACH3018519, TRACH3021544, TRACH3025316, TRACH3030855, TRACH3038399, UTERU3014647, UTERU3021850

The following two clones are also predicted to belong to the category of protein synthesis and/or transport-related protein.
TBAES2004105, UTERU2036507

The clones predicted to belong to the category of cellular defense-related protein are the following five clones.
BRACE2012947, BRHIP2029529, BRTHA3003736, THYMU3015571, TRACH3022296

The clones predicted to belong to the category of development and/or differentiation-related protein are the following 16 clones.
ASTRO3000154, BRACE3034964, BRAWH3004350, BRAWH3029538, BRAWH3038252, BRHIP3007424, BRTHA2024712, BRTHA3011265, FEKID2002493, NT2RP8003490, NT2RP8006452, OCBBF3025503, PLACE7002303, TESTI2026024, TRACH3028180, UTERU3016070

The following one clone is also predicted to belong to the category of development and/or differentiation-related protein.
BRCAN2019772

The clones predicted to belong to the category of DNA-binding and/or RNA-binding protein are the following 119 clones.
ASTRO2016114, BEAST2000981, BRACE2012947, BRACE2019348, BRACE3020669, BRACE3025719, BRACE3026844, BRACE3031743, BRACE3034183, BRACE3041162, BRACE3046152, BRALZ2013621, BRAMY2040915, BRAMY2046537, BRAMY3000692, BRAMY3007078, BRAMY3011501, BRAMY3011581, BRAMY3014027, BRAMY3018754, BRAMY4000962, BRAWH3000446, BRAWH3011577, BRAWH3011623, BRAWH3013009, BRAWH3013264, BRAWH3017477, BRAWH3028796, BRAWH3031342, BRAWH3032571, BRAWH3034775, BRAWH3035936, BRAWH3036247, BRAWH3036334, BRAWH3038827, BRCAN2020331, BRCOC2012386, BRHIP2027077, BRHIP3004725, BRHIP3028246, BRSSN2015497, BRTHA2024712, BRTHA3000456, BRTHA3003736, BRTHA3010212, BRTHA3014000, BRTHA3028339, CHONS2000797, CHONS2002829, CTONG2001932, CTONG2011801, D9OST2003106, FCBBF3020030, FCBBF5000384, FEBRA2028457, HCASM2008154, NETRP2004017, NT2RI3008179, NT2RI3009480, NT2RP7016508, NT2RP8003490, NTONG2003805, NTONG2008483, OCBBF2016928, OCBBF3004487, OCBBF3008392, OCBBF3020263, OCBBF3021361, OCBBF3022166, PLACE7002303, PLACE7004103, PLACE7005169, PLACE7009757, PROST2002078, PUAEN2000594, SMINT2011509, SPLEN2012571, SPLEN2028417, T1ESE2000609, T1ESE2000904, TESTI4002868, TESTI4003796, TESTI4003944, TESTI4005322, TESTI4005470, TESTI4005653, TESTI4032128, TESTI4039575, TESTI4039904, TESTI4052775, THYMU3008105, THYMU3012983, THYMU3014372, THYMU3020869, THYMU3021586, THYMU3026000, THYMU3030072, THYMU3033759, THYMU3037052, THYMU3040829, TLIVE2001616, TRACH3003458, TRACH3004424, TRACH3005191, TRACH3008508, TRACH3010079, TRACH3010167, TRACH3010342, TRACH3015951, TRACH3021778, TRACH3021883, TRACH3022109, TRACH3028180, TRACH3036750, UTERU2037423, UTERU3012293, UTERU3013302, UTERU3016070, UTERU3022588

The following eight clones are also predicted to belong to the category of DNA-binding and/or RNA-binding protein.
BRACE3002184, BRCAN2019772, CTONG2002832, TESTI2046536, TESTI4002988, TESTI4005500, TRACH1000193, TRACH3019290

The clones predicted to belong to the category of ATP binding and/or GTP-binding protein are the following 68 clones.
BRACE2013009, BRACE2016896, BRACE3002344, BRACE3014714, BRACE3017253, BRACE3036283, BRACE3051819, BRAMY3011501, BRAMY3018248, BRAWH2014053, BRAWH3015175, BRAWH3024506, BRAWH3029385, BRAWH3032571, BRAWH3037428, BRAWH3041556, BRAWH3047692, BRCAN2000923, BRCAN2002892, BRCAN2003269, BRCAN2006051, BRHIP3007195, BRHIP3027191, BRHIP3041587, BRSTN2012069, BRTHA2020910, BRTHA3003736, HSYRA2004550, KIDNE2010049, NETRP2003448, NT2RP7016508, OCBBF2003518, OCBBF3004487, PLACE6001933, PLACE6019600, PLACE7004961, PLACE7006498, PLACE7011559, PLACE7016526, PUAEN2006639, SMINT2011406, TESTI2009739, TESTI2040377, TESTI4004917, TESTI4013474, TESTI4021569, TESTI4028182, TESTI4049786, TESTI4052219, THYMU3014372, THYMU3032798, THYMU3041428, THYMU3047115, TRACH3005191, TRACH3009061, TRACH3009701, TRACH3012891, TRACH3019370, TRACH3020769, TRACH3022960, TRACH3023960, TRACH3024081, TRACH3038399, TSTOM2001571, UTERU2024042, UTERU3010919, UTERU3012414, UTERU3019708

The following two clones are also predicted to belong to the category of ATP binding and/or GTP-binding protein.
CTONG2003764, TRACH3021066

The 104 clones shown below are clones which were unassignable to any of the above-mentioned categories, but have been predicted to have some function based on homology search using their full-length nucleotide sequences. Clone Name and Definition in the result of homology search, demarcated by a double slash mark (//), are shown below.
BLADE2002310//SH3-domain binding protein 1 [Homo sapiens]
BLADE2007799//Hepatocellular carcinoma-associated antigen 66.
BRACE2017359//Mus musculus suppressor of Ty 6 homolog (S. cerevisiae) (Supt6h)
BRACE2017872//nuclear receptor-binding SET-domain protein 1 [Mus musculus]
BRACE3009416//testis specific ankyrin-like protein 1 [Homo sapiens]
BRACE3016020//SBBI31 protein [Homo sapiens]
BRACE3019570//SNAP-25-interacting protein [Rattus norvegicus]
BRACE3022303//Pax transcription activation domain interacting protein [Mus musculus]
BRACE3022340//SNAP-25-interacting protein [Rattus norvegicus]
BRACE3026290//Homo sapiens lethal giant larvae homolog 2 [Homo sapiens]
BRACE3032631//F-box protein FBX13 [Mus musculus].
BRACE3040239//Deltex3 [Mus musculus]
BRACE3047482//tripartite motif-containing 9 [Homo sapiens]
BRACE3049714//NYD-TSPG protein [Homo sapiens]
BRACE3052410//IDN3 protein [Homo sapiens]
BRACE3052595//Nim2 [Rattus norvegicus]
BRALZ2014054//cenexin 2 [Rattus norvegicus].
BRAMY3007471//gene trap locus F3b; transcript expressed during hematopoiesis 2 [Mus musculus]
BRAMY3010321//MRIP-1 protein [Homo sapiens]
BRAMY3014613//SH3-domain binding protein 1 [Homo sapiens]
BRAMY4001863//Mus musculus enabled homolog (Drosophila) (Enah), mRNA
BRAWH2011796//S-100 protein, alpha chain.
BRAWH3008167//CUB and Sushi multiple domains 1 [Homo sapiens]
BRAWH3009961//Nim2 [Rattus norvegicus]
BRAWH3010726//phosphatidylinositol transfer protein, membrane-associated; Drosophila retinal degeneration B [Homo sapiens]
BRAWH3015017//axonemal dynein light chain p33.
BRAWH3024231//Tetratricopeptide repeat protein 4.
BRAWH3026938//semaF cytoplasmic domain associated protein 3; semaphorin cytoplasmic domain-associated protein 3A [Mus musculus]
BRAWH3027533//rap2 interacting protein x [Homo sapiens].
BRAWH3030910//Sec23-interacting protein p125 [Homo sapiens]
BRAWH3031710//serologically defined colon cancer antigen 33 [Homo sapiens]
BRAWH3033293//synaptopodin [Homo sapiens]
BRAWH3042568//ventral anterior homeobox containing gene 1 [Mus musculus]
BRAWH3043034//Mus musculus neuregulin 1 (Nrg1)
BRAWH3044122//Munc13-1 [Rattus norvegicus]
BRHIP2026346//lymphocyte specific formin related protein; formin-related gene in leukocytes [Mus musculus]
BRHIP2027563//host cell factor homolog [Homo sapiens]
BRHIP3002114//rTS beta protein [Homo sapiens]
BRHIP3003795//cytochrome P450 retinoid metabolizing protein [Homo sapiens]
BRHIP3006786//peptidylprolyl isomerase (cyclophilin)-like 2; cyclophilin-like protein CyP-60 [Homo sapiens]
BRHIP3017109//Socs-5 [Mus musculus]
BRHIP3019643//Homo sapiens gamma tubulin ring complex protein (76p gene) (76P), mRNA
BRHIP3032148//brain-enriched guanylate kinase-associated [Rattus norvegicus]
BRSTN2006638//synaptotagmin interacting protein 1 [Rattus norvegicus]
BRSTN2016892//BUP protein [Homo sapiens]
BRSTN2016992//DRR1 protein (TU3A protein).
BRSTN2017151//COP9 (constitutive photomorphogenic), subunit 7a (Arabidopsis); COP9 complex S7a [Mus musculus]
BRTHA2020642//DRR1 protein (TU3A protein).
BRTHA3018409//synaptotagmin-like 4; granuphilin-a; granuphilin-b; granuphilin
BRTHA3019183//<Ca2+>dependent activator protein for secretion; Ca2+-dependent activator protein for secretion [Mus musculus] CHONS2001834//tumor endothelial marker 7 precursor [Homo sapiens]
CTONG2009570//rab11 binding protein [Bos taurus].
CTONG2012123//Mus musculus enabled homolog (Drosophila) (Enah), mRNA
CTONG2027591//Mus musculus pecanex homolog (Drosophila) (Pcnx), mRNA.
CTONG3003669//high-glucose-regulated protein 8 [Homo sapiens]
ERLTF2002178//Kelch-like protein X.
HHDPC2008185//jerky [Mus musculus]
NT2RI3001573//F-box protein FBL10 [Mus musculus].
NT2RI3007095//Mus musculus neuregulin 1. (Nrg1), mRNA.
NT2RP8001363//signal peptide, CUB domain, EGF-like 1 [Mus musculus]
NT2RP8001584//alpha integrin binding protein 63 [Homo sapiens]
NT2RP8001604//CUB and Sushi multiple domains 1 [Homo sapiens]
OCBBF3019269//Homo sapiens Dvl-binding protein IDAX (inhibition of the Dvl and Axin complex) (IDAX)
OCBBF3022827//putative Rab5 GDP/GTP exchange factor homologue [Homo sapiens]
OCBBF3023913//Mus musculus protein phosphatase 1, regulatory (inhibitor) subunit 1C (Ppp1r1c)
PLACE6003004//rTS beta protein [Homo sapiens]
PLACE6008315//similar to ALPHA-ACTININ, SARCOMERIC (F-ACTIN CROSS LINKING PROTEIN) (D. melanogaster) [Homo sapiens].
PLACE6010925//NY-REN-50 antigen [Homo sapiens]
PLACE7012127//AAA-ATPase TOB3 [Homo sapiens]
PROST2016566//erythroblast macrophage protein [Mus musculus]
SYNOV2017179//EBP50-PDZ interactor of 64 kD [Homo sapiens] SYNOV3000345//upregulated by 1,25-dihydroxyvitamin D-3 [Homo sapiens]
TBAES2003917//NG28 protein [Mus musculus]
TESTI2001364//lactate dehydrogenase A -like [Homo sapiens]
TESTI2004601//NYD-TSPG protein [Homo sapiens]
TESTI2009497//GPI-anchored protein p137 (p137GPI).
TESTI4002774//oxysterol binding protein 2 [Mus musculus]
TESTI4003579//FH1/FH2 domains-containing protein (Formin homolog overexpressed in spleen) (FHOS).
TESTI4003703//retinoblastoma-associated protein RAP140 [Homo sapiens]
TESTI4013742//antigen identified by monoclonal antibody 2A8 [Mus musculus]
TESTI4014908//dedicator of cyto-kinesis 2 [Mus musculus]
TESTI4018506//tomosyn [Rattus norvegicus]
TESTI4020342//H326 [Homo sapiens]
TESTI4024294//WW domain binding protein 2 [Mus musculus]
TESTI4039451//B29 protein [Homo sapiens]
TESTI4041482//Rattus norvegicus SEC15 homolog (S. cerevisiae) (Sec15), mRNA
TESTI4043166//lymphocyte specific formin related protein; formin-related gene in leukocytes [Mus musculus]
TESTI4047328//otogelin [Mus musculus]
THYMU3011717//exocyst component protein 70 kDa homolog (S. cerevisiae)
THYMU3016822//erythroblast macrophage protein [Mus musculus]
THYMU3026479//secretory pathway component Sec31B-1 [Homo sapiens]
THYMU3028702//chromosome condensation-related SMC-associated protein 1; chromosome condensation-related SMC-associated protein 1; KIAA0159 gene product [Homo sapiens]
THYMU3029719//AAA-ATPase TOB3 [Homo sapiens]
THYMU3038347//tumor stroma and activated macrophage protein DLM-1 [Homo sapiens]
THYMU3038603//WW domain binding protein 2 [Mus musculus]
THYMU3040830//AD-012 protein [Homo sapiens]
THYMU3041603//gamma-tubulin complex protein 2 [Homo sapiens]
TKIDN2003396//Homo sapiens paternally expressed 10 (PEG10), mRNA
TRACH2011057//D-type cyclin-interacting protein 1; MAID protein [Homo sapiens]
TRACH3004412//clusterin-like 1 (retinal); unknown prepropeptide specific to rod photoreceptor [Homo sapiens]
TRACH3012106//erythrocyte protein band 4.1-like 3 [Mus musculus]
UTERU3009775//PAPIN [Rattus norvegicus]
UTERU3010892//adaptor-related protein complex 3, delta 1. subunit; adaptin, delta [Homo sapiens]
UTERU3017995//p47 [Homo sapiens]

So far no information suggesting the function of the following six clones has been provided by the homology search. The functions of these clones may be clarified when an updated database becomes available in future. Clone names are shown below.
BRAMY3008096, BRAMY3016953, BRHIP3038037, BRTHA3004432,
TESTI4052089, UTERU3020583

Further, a polypeptide will not always belong solely to a single category of the above-described functional categories, and therefore, a polypeptide may belong to any of the predicted functional categories. Further analyses may yield additional functions for clones classified into these functional categories.

Detailed descriptions concerning each domain or motif can be found in websites linked from the websites of Pfam, InterPro (http://www.ebi.ac.uk/interpro/), PROSITE (http://www.expasy.ch/cgi-bin/prosite-list.pl), or such. This information can be found based on domain/motif names, and accession numbers of hit data obtained through domain searches of Pfam (http://www.sanger.ac.uk/Software/Pfam/index.shtml) (see Example 5) for amino acid sequences deduced from the 2,495 full-length clones of the present invention whose full-length nucleotide sequences have been determined. PROSITE in particular enables comparison of unique functional categories. The functions of polypeptides encoded by the 825 clones with hit data in Pfam were predicted and classified into the 14 functional categories described below. As a result, 646 clones were estimated to encode proteins belonging to these categories.
Secretory and/or membrane protein (102 clones)
Glycoprotein-related protein (83 clones)
Signal transduction-related protein (128 clones)
Transcription-related protein (149 clones)
Disease-related protein (four clones)
Enzyme and/or metabolism-related protein (271 clones)
Cell division- and/or cell proliferation-related protein (14 clones)
Cytoskeleton-related protein (51 clones)
Nuclear protein and/or RNA synthesis-related protein (29 clones)
Protein synthesis- and/or transport-related protein (51 clones)
Cellular defense-related protein (four clones)
Development and/or differentiation-related protein (one clone)
DNA- and/or RNA-binding protein (192 clones)
ATP- and/or GTP-binding protein (42 clones)

The clones predicted to belong to the category of secretory protein and/or membrane protein are the following 92 clones.
3NB692004045, BRACE3002264, BRACE3009392, BRACE3013418, BRACE3024879, BRACE3032385, BRACE3039378, BRACE3042432, BRACE3050504, BRACE3051621, BRAMY2046537, BRAMY3004126, BRAWH2000256, BRAWH2011812, BRAWH3023156, BRAWH3025157, BRAWH3027880, BRAWH3036270, BRAWH3037265, BRAWH3042772, BRCAN2003269, BRCAN2022126, BRCOC2006164, BRHIP3002000, BRHIP3005944, BRHIP3008320, BRHIP3011567, BRHIP3014675, BRHIP3016032, BRHIP3017558, BRHIP3025795, BRHIP3033557, BRHIP3039509, BRSTN2010089, BRTHA2031917, BRTHA3011194, BRTHA3012265, BRTHA3014547, COLON2005735, JCMLC2000273, KIDNE2004531, LYMPB2002236, NT2RP7019682, NT2RP8001363, NT2RP8003787, OCBBF2003518, OCBBF2004478, OCBBF2009536, OCBBF2018618, OCBBF3004487, OCBBF3025475, OCBBF3028001, PEBLM2005615, PLACE6010936, PLACE7004103, PLACE7011559, PLACE7018304, TESTI2018335, TESTI2022323, TESTI2024267, TESTI2036822, TESTI4003602, TESTI4004539, TESTI4005399, TESTI4008305, TESTI4010544, TESTI4014415, TESTI4021569, TESTI4023096, TESTI4026080, TESTI4040559, TESTI4049899, THYMU3015647, THYMU3021404, THYMU3023400, THYMU3026532, THYMU3030752, THYMU3040172, THYMU3044075, TRACH3003357, TRACH3004113, TRACH3004747, TRACH3005699, TRACH3006800, TRACH3009061, TRACH3019370, TRACH3023373, TRACH3031678, TRACH3032150, UTERU3001946, UTERU3016273, UTERU3017626

The following ten clones are also predicted to belong to the category of secretory protein and/or membrane protein.
BRAWH3013197, BRAWH3028645, BRAWH3046240, BRCAN2019772, PROST2010326, TESTI2043585, TESTI4005158, THYMU3024602, THYMU3046350, TRACH1000193

The clones predicted to belong to the category of glycoprotein-related protein are the following 81 clones.
3NB692004045, ADIPS2000069, BRACE2017397, BRACE3013874, BRACE3017253, BRACE3039358, BRAMY2040915, BRAMY3015549, BRAWH3009961, BRAWH3023415, BRAWH3049544, BRHIP3017558, BRHIP3025795, BRHIP3036371, BRHIP3036715, BRHIP3038735, BRTHA2019726, BRTHA2020400, BRTHA2020721, BRTHA3017791, CERVX2000968, FELNG2000720, JCMLC2000273, KIDNE2004531, NT2RP8008057, OCBBF2000831, OCBBF2004478, OCBBF2030927, PEBLM2005615, PLACE7006090, SPLEN2025012, STOMA2004663, TESTI2021654, TESTI2052670, TESTI4008305, TESTI4022158, TESTI4031173, TESTI4032128, TESTI4037949, TESTI4051424, THYMU3002825, THYMU3014173, THYMU3016518, THYMU3020221, THYMU3025118, THYMU3026306, THYMU3026532, THYMU3037772, THYMU3040746, TLUNG2001445, TLUNG2001600, TRACH3003357, TRACH3004113, TRACH3004412, TRACH3005274, TRACH3005699, TRACH3006800, TRACH3011082, TRACH3011184, TRACH3012659, TRACH3015354, TRACH3018261, TRACH3018907, TRACH3019058, TRACH3019621, TRACH3019807, TRACH3020930, TRACH3021023, TRACH3024512, TRACH3026299, TRACH3028441, TRACH3029670, TRACH3031316, TRACH3034680, TRACH3036103, TRACH3037505, TUTER2001433, UTERU3001946, UTERU3010409, UTERU3011398, UTERU3015647

The following two clones are also predicted to belong to the category of glycoprotein-related protein.
BRAWH3028645, TESTI4005158

The clones predicted to belong to the category of signal transduction-related protein are the following 125 clones.
BLADE2002310, BLADE2008809, BRACE2047975, BRACE3002344, BRACE3003866, BRACE3004767, BRACE3013418, BRACE3015898, BRACE3017253, BRACE3042046, BRACE3044172, BRACE3045424, BRACE3046491, BRACE3051621, BRACE3052321, BRACE3052595, BRAMY3005184, BRAMY3009491, BRAMY3010321, BRAMY3014613, BRAMY3015547, BRAMY3017920, BRAWH2012866, BRAWH3009961, BRAWH3017180, BRAWH3018063, BRAWH3019026, BRAWH3022431, BRAWH3024186, BRAWH3026349, BRAWH3027574, BRAWH3027806, BRAWH3029385, BRAWH3031342, BRAWH3032340, BRAWH3035914, BRAWH3037428, BRAWH3044122, BRAWH3047692, BRCAN2000923, BRCAN2002892, BRCOC2001355, BRHIP3003306, BRHIP3006294, BRHIP3006786, BRHIP3011460, BRHIP3017109, BRHIP3021019, BRHIP3028570, BRHIP3037543, BRHIP3041587, BRTHA2026290, BRTHA2027250, BRTHA3014547, BRTHA3020771, BRTHA3021708, BRTHA3023403, BRTHA3026916, CTONG2009570, ERLTF2002369, FEKID2001001, FEKID2002637, FELNG2001953, KIDNE2010049, LYMPB2002344, N1ESE2000698, NETRP2003448, NT2RI2004818, NTONG2008483, OCBBF3006986, OCBBF3021086, OCBBF3021502, OCBBF3023175, PLACE5000492, PLACE6000055, PLACE6019600, PLACE7009936, PLACE7014247, PLACE7016526, PUAEN2006639, SKMUS2008585, SKMUS2009557, SMINT2017964, SPLEN2007689, SYNOV2017179, SYNOV4009575, TESTI4002774, TESTI4004695, TESTI4010902, TESTI4012960, TESTI4013474, TESTI4020342, TESTI4020596, TESTI4021197, TESTI4022158, TESTI4028042, TESTI4029731, TESTI4033177, TESTI4036048, TESTI4046073, TESTI4047808, TESTI4049786, TESTI4051865, THYMU3013785, THYMU3025683, THYMU3032798, TRACH2024730, TRACH3003037, TRACH3003357, TRACH3005173, TRACH3011538, TRACH3018519, TRACH3020605, TRACH3024020, TRACH3030176, TRACH3031660, TRACH3036750, TRACH3038399, TSTOM2001571, UTERU2024042, UTERU3001029, UTERU3006720, UTERU3010919, UTERU3021231, UTERU3022168

The following three clones are also predicted to belong to the category of signal transduction-related protein.
BRAMY3008096, TESTI4052089, TRACH3021066

The clones predicted to belong to the category of transcription-related protein are the following 141 clones.
ASTRO2016114, BEAST2000981, BRACE2019348, BRACE3025719, BRACE3026844, BRACE3026947, BRACE3029021, BRACE3034183, BRACE3040239, BRACE3041162, BRACE3047482, BRAMY2041347, BRAMY3000692, BRAMY3007078, BRAMY3011581, BRAMY3014027, BRAMY4002575, BRAWH2000256, BRAWH2014053, BRAWH2016209, BRAWH3000446, BRAWH3005886, BRAWH3009961, BRAWH3013009, BRAWH3013264, BRAWH3015175, BRAWH3015610, BRAWH3017477, BRAWH3021580, BRAWH3022651, BRAWH3027533, BRAWH3027880, BRAWH3028796, BRAWH3031342, BRAWH3031710, BRAWH3032571, BRAWH3035936, BRAWH3036247, BRAWH3036334, BRAWH3042787, BRAWH3044122, BRAWH3046424, BRCAN2021452, BRCOC2012386, BRHIP2027077, BRHIP2029663, BRHIP3005037, BRHIP3007609, BRHIP3017256, BRHIP3019824, BRHIP3027651; BRHIP3028246, BRHIP3039592, BRSSN2011843, BRSTN2012069, BRTHA3000456, BRTHA3003225, BRTHA3010212, BRTHA3014000, BRTHA3028339, CHONS2000797, CTONG2006235, CTONG2011801, FCBBF3020030, FEBRA2002260, HCASM2008154, KIDNE2018268, NETRP2003103, NETRP2004017, NT2RI3009480, NTONG2003805, NTONG2008483, OCBBF2014745, OCBBF2016928, OCBBF3001333, OCBBF3008392, OCBBF3019269, OCBBF3020263, OCBBF3022827, OCBBF3025503, OCBBF3026361, PLACE7005169, PLACE7007973, PLACE7009757, PLACE7018512, SMINT2014721, SPLEN2012571, SPLEN2036608, T1ESE2000904, TESTI2036822, TESTI2040377, TESTI4000370, TESTI4000621, TESTI4001679, TESTI4002799, TESTI4003796, TESTI4003944, TESTI4005322, TESTI4005470, TESTI4024494, TESTI4028182, TESTI4032913, TESTI4039904, TESTI4051054, TESTI4052775, THYMU2008207, THYMU2038199, THYMU3008105, THYMU3012983, THYMU3014372, THYMU3021586, THYMU3022434, THYMU3026000, THYMU3030072, THYMU3034671, THYMU3037617, THYMU3043200, THYMU3045704, TKIDN2003396, TLIVE2001616, TLUNG2000654, TRACH3002752, TRACH3003037, TRACH3003458, TRACH3004113, TRACH3004412, TRACH3004424, TRACH3005274, TRACH3010079, TRACH3010167, TRACH3015951, TRACH3022109, TRACH3026303, TRACH3028180, TRACH3036750, UTERU2037423, UTERU3012293, UTERU3015011, UTERU3016070, UTERU3018172, UTERU3022588

The following eight clones are also predicted to belong to the category of transcription-related protein.
BRACE3002184, BRCAN2019772, CTONG2002832, TESTI2046536, TESTI4002988, TESTI4005500, TRACH1000193, TRACH3019290

The clones predicted to belong to the category of disease-related protein are the following four clones.
BRAWH3041928, BRHIP3000859, BRTHA3018409, THYMU3025642

The clones predicted to belong to the category of enzyme and/or metabolism-related protein are the following 264 clones.
BRACE1000475, BRACE2003628, BRACE2012528, BRACE2013132, BRACE2016896, BRACE2035120, BRACE2042541, BRACE2047975, BRACE3002344, BRACE3009392, BRACE3013418, BRACE3015898, BRACE3017253, BRACE3019941, BRACE3024444, BRACE3031315, BRACE3031372, BRACE3032537, BRACE3033525, BRACE3034183, BRACE3034964, BRACE3039288, BRACE3039454, BRACE3041059, BRACE3042409, BRACE3044172, BRACE3046491, BRACE3049714, BRACE3050270, BRACE3051819, BRACE3052410, BRACE3052595, BRAMY3007078, BRAMY3009491, BRAMY3011581, BRAMY3014613, BRAMY4000915, BRAWH2000256, BRAWH2002333, BRAWH2012866, BRAWH2014053, BRAWH2016785, BRAWH3009961, BRAWH3010657, BRAWH3013264, BRAWH3015175, BRAWH3017180, BRAWH3017259, BRAWH3019026, BRAWH3021724, BRAWH3022431, BRAWH3023415, BRAWH3024186, BRAWH3028796, BRAWH3029385, BRAWH3029806, BRAWH3032571, BRAWH3033513, BRAWH3034668, BRAWH3034743, BRAWH3037979, BRAWH3041556, BRAWH3043295, BRAWH3044122, BRAWH3044985, BRAWH3046424, BRAWH3047692, BRAWH3048724, BRAWH3049544, BRCAN2003814, BRCAN2006051, BRCAN2015402, BRCAN2021325, BRCOC2001355, BRCOC2006164, BRHIP2029663, BRHIP3001481, BRHIP3002000, BRHIP3002114, BRHIP3002141, BRHIP3003063, BRHIP3003126, BRHIP3003795, BRHIP3004725, BRHIP3005307, BRHIP3005673, BRHIP3007195, BRHIP3007223, BRHIP3011082, BRHIP3012289, BRHIP3016032, BRHIP3019643, BRHIP3021019, BRHIP3032374, BRHIP3033557, BRHIP3035006, BRHIP3037543, BRHIP3038030, BRHIP3041587, BRSSN2004710, BRSSN2011843, BRSTN2011961, BRSTN2016918, BRTHA2001304, BRTHA2005448, BRTHA2026290, BRTHA2026311, BRTHA2027250, BRTHA2030036, BRTHA2033683, BRTHA2035743, BRTHA2036295, BRTHA2037247, BRTHA3003736, BRTHA3010135, BRTHA3014547, BRTHA3021786, BRTHA3023403, BRTHA3026916, CHONS2002829, COLON2004351, CTONG2010330, CTONG2020582, CTONG2026987, FCBBF3001018, FCBBF3021191, FEBRA2013570, FEBRA2026582, FEHRT2002708, FEKID2002637, HHDPC2008185, HSYRA2004550, KIDNE2004531, KIDNE2010049, LYMPB2002236, NT2RI2004818, NT2RI3001967, NT2RP7016508, NT2RP8003490, NT2RP8003787, NT2RP8005546, OCBBF2000831, OCBBF2007039, OCBBF2024589, OCBBF3001616, OCBBF3004487, OCBBF3021086, OCBBF3023175, OCBBF3025503, OCBBF3026088, OCBBF3026361, PLACE5000492, PLACE6003004, PLACE7003985, PLACE7004103, PLACE7004961, PLACE7006090, PLACE7007379, PLACE7008136, PLACE7012111, PLACE7014247, PLACE7016526, SKMUS2008585, SKMUS2009557, SMINT2011406, SMINT2011509, SYNOV2017179, SYNOV4003174, SYNOV4009139, T1ESE2000609, T1ESE2002665, TESTI2001364, TESTI2005112, TESTI2007490, TESTI2018335, TESTI2021112,
TESTI2021654, TESTI2030901, TESTI2037877, TESTI2049062, TESTI4000621, TESTI4002774, TESTI4002799, TESTI4003404, TESTI4003565, TESTI4003602, TESTI4003703, TESTI4005399, TESTI4007671, TESTI4010544, TESTI4010721, TESTI4012960, TESTI4017854, TESTI4020342, TESTI4020596, TESTI4020819, TESTI4021129, TESTI4021197, TESTI4023096, TESTI4024494, TESTI4026080, TESTI4028182, TESTI4031066, TESTI4033177, TESTI4040598, TESTI4041482, TESTI4046073, TESTI4047808, TESTI4049786, TESTI4051424, TESTI4051865, TESTI4052219, THYMU3000390, THYMU3002825, THYMU3014372, THYMU3023400, THYMU3025683, THYMU3026306, THYMU3026479, THYMU3031878, THYMU3032798, THYMU3034671, THYMU3036953, THYMU3041428, THYMU3047115, THYMU3047891, TRACH2022113, TRACH2024730, TRACH3003037, TRACH3005274, TRACH3006800, TRACH3009008, TRACH3009061, TRACH3011313, TRACH3016455, TRACH3017409, TRACH3018108, TRACH3018261, TRACH3019621, TRACH3021544, TRACH3023516, TRACH3024020, TRACH3024081, TRACH3024342, TRACH3024512, TRACH3025316, TRACH3026303, TRACH3026650, TRACH3027229, TRACH3027701, TRACH3029329, TRACH3032150, TRACH3036750, TRACH3038399, TSTOM2001571, UTERU2024042, UTERU3001946, UTERU3010604, UTERU3010919, UTERU3015299, UTERU3017441, UTERU3019708, UTERU3020090, UTERU3022168

The following seven clones are also predicted to belong to the category of enzyme and/or metabolism-related protein.
ADRGL2011190, BRAMY3008096, CTONG2002832, TESTI4052089, THYMU3024602, THYMU3044175, TRACH3021066

The clones predicted to belong to the category of cell division and/or cell proliferation-related protein are the following 13 clones.
BRACE3022303, BRAWH3017260, BRHIP2008756, BRHIP3028570, BRSTN2006638, NT2RI2004818, PLACE7009563, PLACE7016526, SMINT2014721, THYMU3025642, THYMU3033626, TRACH3029329, UTERU3010919

The following one clone is also predicted to belong to the category of cell division and/or cell proliferation-related protein.
TBAES2007481

The clones predicted to belong to the category of cytoskeleton-related protein are the following 51 clones.
BRACE2046976, BRACE3013874, BRACE3047482, BRACE3051819, BRAMY3015549, BRAWH3015175, BRAWH3018548, BRAWH3021580, BRAWH3024186, BRAWH3024506, BRAWH3029385, BRAWH3032298, BRAWH3049544, BRHIP3003340, BRHIP3012736, BRHIP3036936, BRSTN2016918, BRTHA2020910, BRTHA2025869, BRTHA2031917, BRTHA3011361, BRTHA3025073, CTONG2008989, HSYRA2007338, LYMPB2002458, OCBBF3008835, OCBBF3027969, PEBLM2006298, PLACE7000266, PLACE7004103, PLACE7004961, SMINT2011406, SYNOV4003174, TESTI2006543, TESTI2034913, TESTI4001517, TESTI4005653, TESTI4008305, TESTI4041049, TESTI4051424, TESTI4051865, THYMU3020221, THYMU3038158, TLUNG2000654, TRACH3002890, TRACH3006379, TRACH3012460, TRACH3018524, TRACH3020769, TRACH3028837, UTERU3011837

The clones predicted to belong to the category of nuclear protein and/or RNA synthesis-related protein are the following 29 clones.
BRACE2016896, BRACE3032537, BRACE3034183, BRACE3039288, BRAWH3013264, BRAWH3032571, BRCOC2006164, BRHIP3004725, BRSSN2011843, BRTHA2026290, BRTHA3003736, BRTHA3014547, NT2RP7016508, NT2RP8005546, OCBBF3004487, OCBBF3021086, OCBBF3026361, PLACE5000492, TESTI4000621, TESTI4002799, TESTI4010721, TESTI4012960, THYMU3014372, THYMU3033626, THYMU3041428, TRACH3017409, TRACH3029462, UTERU3010919, UTERU3019708

The clones predicted to belong to the category of protein synthesis and/or transport-related protein are the following 50 clones.
BRACE3033525, BRACE3041059, BRAMY4001652, BRAWH3010657, BRAWH3013264, BRAWH3034668, BRAWH3036247, BRAWH3037428, BRAWH3037979, BRCAN2000923, BRCAN2002892, BRCAN2006051, BRCAN2021325, BRCAN2021718, BRHIP2029663, BRHIP3002000, BRHIP3003126, BRHIP3007223, BRHIP3011082, BRSTN2010089, BRTHA2036295, BRTHA3012265, CHONS2002829, D9OST2004417 HHDPC2008185, NETRP2003448, OCBBF2007039, OCBBF3021086, PLACE6003004, PLACE6010925, PLACE7006498, PLACE7007379, PLACE7012111, PLACE7016526, TESTI2023903, TESTI2036285, TESTI4003602, TESTI4012960, TESTI4014415, TESTI4030864, TESTI4051865, THYMU3036953, THYMU3047891, TRACH3004113, TRACH3006800, TRACH3009061, TRACH3021544, TRACH3026650, UTERU3001946, UTERU3012414

The following one clone is also predicted to belong to the category of protein synthesis and/or transport-related protein.
CTONG2002832

The clones predicted to belong to the category of cellular defense-related protein are the following four clones.
BRHIP3027191, SYNOV4009575, TESTI2023903, TRACH3029462

The clones predicted to belong to the category of development and/or differentiation-related protein are the following one clone.
CHONS2000797

The clones predicted to belong to the category of DNA-binding and/or RNA-binding protein are the following 185 clones.
ASTRO2016114, BEAST2000981, BRACE2012625, BRACE2016896, BRACE2019348, BRACE3019941, BRACE3025719, BRACE3026844, BRACE3026947, BRACE3029021, BRACE3031743, BRACE3032537, BRACE3034183, BRACE3039288, BRACE3040239, BRACE3041162, BRACE3047482, BRACE3050270, BRALZ2013621, BRAMY2041347, BRAMY3000692, BRAMY3007078, BRAMY3011581, BRAMY3014027, BRAMY4002575, BRAWH2000256, BRAWH2014053, BRAWH2016209, BRAWH3000446, BRAWH3005886, BRAWH3009961, BRAWH3011623, BRAWH3013009, BRAWH3013264, BRAWH3015175, BRAWH3015610, BRAWH3017477, BRAWH3021580, BRAWH3022651, BRAWH3027533, BRAWH3027607, BRAWH3027880, BRAWH3028796, BRAWH3031342, BRAWH3031710, BRAWH3032571, BRAWH3035403, BRAWH3035936, BRAWH3036247, BRAWH3036334, BRAWH3038055, BRAWH3042787, BRAWH3044122, BRCAN2020331, BRCAN2021452, BRCOC2006164, BRCOC2012386, BRHIP2027077, BRHIP2029663, BRHIP3002141, BRHIP3004725, BRHIP3005037, BRHIP3007609, BRHIP3017256, BRHIP3019824, BRHIP3027651, BRHIP3028246, BRHIP3039592, BRSSN2011843, BRSTN2012069, BRTHA2026290, BRTHA2037247, BRTHA3000456, BRTHA3003225, BRTHA3003736, BRTHA3010212, BRTHA3014000, BRTHA3014547, BRTHA3028339, CHONS2000797, CTONG2003517, CTONG2006235, CTONG2011801, CTONG2026987, D9OST2003106, FCBBF3020030, FEBRA2002260, FEBRA2028457, FEHRT2002708, HCASM2008154, KIDNE2018268, NETRP2003103, NETRP2004017, NT2RI3009480, NT2RP7016508, NT2RP8005546, NTONG2003805, NTONG2008483, OCBBF2014745, OCBBF2016928, OCBBF3001333, OCBBF3001616, OCBBF3004487, OCBBF3008392, OCBBF3019269, OCBBF3020263, OCBBF3021086, OCBBF3021361, OCBBF3022827, OCBBF3025503, OCBBF3026361, PLACE5000492, PLACE7004103, PLACE7005169, PLACE7007973, PLACE7008136, PLACE7009757, PLACE7018512, PROST2002078, SMINT2011509, SMINT2014721, SPLEN2012571, SPLEN2036608, T1ESE2000609, T1ESE2000904, TESTI2036822, TESTI2040377, TESTI4000370, TESTI4000621, TESTI4001679, TESTI4002799, TESTI4003796, TESTI4003944, TESTI4005322, TESTI4005470, TESTI4012960, TESTI4024494, TESTI4028182, TESTI4032913, TESTI4039904, TESTI4051054, TESTI4052775, THYMU2008207, THYMU2038199, THYMU3008105, THYMU3012983, THYMU3014372, THYMU3021586, THYMU3022434, THYMU3023400, THYMU3026000, THYMU3030072, THYMU3037617, THYMU3040829, THYMU3041428, THYMU3043200, THYMU3045704, TKIDN2003396, TLIVE2001616, TLUNG2000654, TRACH3002752, TRACH3003037, TRACH3003458, TRACH3004113, TRACH3004412, TRACH3004424, TRACH3005274, TRACH3010079, TRACH3010167, TRACH3015951, TRACH3017409, TRACH3021778, TRACH3022109, TRACH3026303, TRACH3028180, TRACH3036750, UTERU2037423, UTERU3012293, UTERU3015011, UTERU3016070, UTERU3019708, UTERU3020090, UTERU3022168, UTERU3022588, UTERU3023141

The following seven clones are also predicted to belong to the category of DNA-binding and/or RNA-binding protein.
BRACE3002184, BRCAN2019772, CTONG2002832, TESTI4002988, TESTI4005500, TRACH1000193, TRACH3019290

The clones predicted to belong to the category of ATP binding and/or GTP-binding protein are the following 41 clones.
BRACE3042409, BRAWH2002333, BRAWH2014053, BRAWH3015175, BRAWH3029385, BRAWH3029806, BRAWH3034743, BRAWH3037428, BRAWH3047692, BRCAN2000923, BRCAN2002892, BRCAN2022126, BRCOC2001355, BRCOC2006164, BRHIP3007195, BRSSN2004710, BRTHA2026290, BRTHA2033683, BRTHA3026916, CTONG2020582, HSYRA2004550, NETRP2003448, PLACE6019600, PLACE7004961, PLACE7006498, PLACE7016526, SMINT2011406, TESTI4010544, TESTI4014415, TESTI4028182, TESTI4029731, TESTI4040559, TESTI4041482, TESTI4052219, THYMU3013785, THYMU3047115, TRACH2024730, TRACH3024081, TRACH3029329, TRACH3031660, UTERU3012414

The following one clone is also predicted to belong to the category of ATP binding and/or GTP-binding protein.
ADRGL2011190

The following 172 clones had hit data in Pfam (see Example 5), although it remains unclear as to which of the above-described categories each clone belongs. If data on polypeptides with a similar domain or motif can be accumulated, and their functions clarified in more detail, they may be classified into any of the above-described categories.
BLADE2001031, BRACE2010336, BRACE2013009, BRACE2017872, BRACE2023744, BRACE2034434, BRACE3001973, BRACE3002756, BRACE3005903, BRACE3014523, BRACE3019570, BRACE3022340, BRACE3026345, BRACE3036283, BRACE3040644, BRACE3043597, BRACE3046466, BRACE3048615, BRALZ2010842, BRAMY2031516, BRAMY2041384, BRAMY3002886, BRAMY3011501, BRAMY3015086, BRAMY3018754, BRAMY4000962, BRAWH2011796, BRAWH2016223, BRAWH3001783, BRAWH3003573, BRAWH3008167, BRAWH3011331, BRAWH3011577, BRAWH3014609, BRAWH3021574, BRAWH3022347, BRAWH3022719, BRAWH3024231, BRAWH3026938, BRAWH3027440, BRAWH3030772, BRAWH3030910, BRAWH3033448, BRAWH3034775, BRAWH3038252, BRAWH3038324, BRAWH3038827, BRAWH3042438, BRAWH3042568, BRAWH3044151, BRAWH3045118, BRAWH3048374, BRCAN2010665, BRCAN2019907, BRCAN2020234, BRCAN2025093, BRCOC2006639, BRHIP2013958, BRHIP2026346, BRHIP2027563, BRHIP3001878, BRHIP3004710, BRHIP3005142, BRHIP3005231, BRHIP3006449, BRHIP3007424, BRHIP3009753, BRHIP3010289, BRHIP3020733, BRHIP3029409, BRHIP3030230, BRHIP3033734, BRSSN2015497, BRTHA2038345, BRTHA3011187, BRTHA3021971, BRTHA3026161, BRTHA3027171, BRTHA3027638, CHONS2001287, CHONS2001834, DFNES2011221, ERLTF2002178, FCBBF3012443, FCBBF3024911, FCBBF5000384, FEBRA2000805, FEBRA2023498, FEKID2002493, HCHON2009766, JCMLC2002751, KIDNE2015987, NT2RI3001573, NT2RI3005923, NT2RI3009524, NT2RP7007387, NT2RP7020343, NT2RP8000633, NT2RP8001604, NT2RP8006452, NT2RP8007920, NT2RP8009119, OCBBF3001202, OCBBF3005330, OCBBF3023913, OCBBF3026979, PEBLM2001803, PLACE6001933, PLACE7002303, PUAEN2000594, PUAEN2000684, SMINT2010753, SPLEN2022785, SPLEN2028417, SYNOV2003326, TBAES2003917, TBAES2007428, TESOP2002005, TESTI2005564, TESTI2009739, TESTI2011020, TESTI2018867, TESTI2049041, TESTI4001569, TESTI4002141, TESTI4002868, TESTI4004031, TESTI4007965, TESTI4011926, TESTI4013742, TESTI4024294, TESTI4035898, TESTI4039451, TESTI4041984, TESTI4043166, TESTI4046873, TESTI4047328, TESTI4047569, TESTI4051015, TESTI4052598, THYMU3003007, THYMU3012402, THYMU3015042, THYMU3015571, THYMU3017761, THYMU3019476, THYMU3021755, THYMU3033649, THYMU3040126, THYMU3046360, TKIDN2011051, TKIDN2011160, TLIVE2007736, TRACH3007689, TRACH3012106, TRACH3015346, TRACH3016805, TRACH3018606, TRACH3022296, TRACH3022758, TRACH3023203, TRACH3028855, TRACH3030855, TRACH3032570, UTERU2016669, UTERU3001394, UTERU3009775, UTERU3011558, UTERU3011579, UTERU3017995, UTERU3018255, UTERU3021850

Likewise, the following seven clones also had hit data in Pfam (see Example 5), although it remains unclear as to which of the above-described categories each clone belongs. When data on polypeptides with a similar domain or motif are accumulated, and their functions are clarified in more detail, these clones may also be classified into any of the above-described categories.
BRACE3026993// TSC-22/dip/bun family
BRACE3046450// PDZ domain (Also known as DHR or GLGF).//PDZ domain (Also known as DHR or GLGF).//PDZ domain (Also known as
DHR or GLGF). 1//PDZ domain (Also known as DHR or GLGF). CTONG2003764// Phorbol esters/diacylglycerol binding domain (C1 domain)
TBAES2004105// Thrombospondin type 1 domain
TBAES2007379// EGF-like domain//EGF-like domain//Trypsin
Inhibitor like cysteine rich domain//EGF-like domain//EGF-like domain//Keratin, high sulfur B2 protein//EGF-like domain//EGF-like domain//Granulins//EGF-like domain//EGF-like domain//EGF-like domain
TBAES2008133// PDZ domain (Also known as DHR or GLGF).
UTERU2036507// Thrombospondin type 1 domain//NTR/C345C module

The function of a motif or domain may sometimes belong to more than one of the above-described functional categories, and there is also the possibility that such a motif or domain may be predicted to belong to every functional category. As new polypeptide data are accumulated and novel domains and motifs are found, a new functional domain or motif may be identified by re-analyzing deduced amino acid sequences in homology searches using updated databases. Thus in the future, the remaining clones, for which there are currently no hit data, may be classified into any of the above-described categories.

Since the polypeptides encoded by clones of the present invention contain full-length amino acid sequences, it is possible to analyze their biological activity and effect on cellular conditions such as cell proliferation and differentiation, by expressing the polypeptides as recombinant polypeptides using an appropriate expression system, injecting the recombinant into a cell, or raising a specific antibody against that polypeptide.

The biological activities of respective polypeptides can be analyzed by the methods as shown below.
Secretory protein, transmembrane protein:
"Ion Channels" (Ed., R. H. Ashley, 1995) of "The Practical Approach Series" (IRL PRESS),
"Growth Factors" (Eds., I. McKay, I. Leigh, 1993),
"Extracellular Matrix" (Eds., M. A. Haralson, J. R. Hassell, 1995) ;

### Glycoprotein-related protein:

"Glycobiology" (Eds., M. Fukuda, A. Kobata, 1993) of "The Practical Approach Series" (IRL PRESS),
"Glycoprotein Analysis in Biomedicine" (Ed., Elizabeth F. Hounsell, 1993) of "Method in Molecular Biology" (Humana Press) series;

### Signal transduction-related protein:

"Signal Transduction" (Ed., G. Milligan, 1992) of "The Practical Approach Series" (IRL PRESS),
"Protein Phosphorylation" (Ed., D. G. Hardie, 1993), or
"Signal Transduction Protocols" (Eds., David A. Kendall, Stephen J. Hill, 1995) of "Method in Molecular Biology" (Humana Press) series;

### Transcription-related protein:

"Gene Transcription" (Eds., B. D. Hames, S. J. Higgins, 1993) of "The Practical Approach Series" (IRL PRESS),
"Transcription Factors" (Ed., D. S. Latchman, 1993); Enzyme and/or metabolism-related protein:
   "Enzyme Assays" (Eds., ROBERT EISENTHAL and MICHAEL J. DANSON, 1992) of "The Practical Approach Series" (IRL PRESS); Cell division and/or cell proliferation-related protein:
   "Cell Growth, Differentiation and Senescence" (Ed., GEORGE STUDZINSKI, 2000) of "The Practical Approach Series" (IRL PRESS) ;

### Cytoskeleton-related protein:

"Cytoskeleton: Signalling and Cell Regulation" (Eds., KERMIT L. CARRAWAY and CAROLIE A. CAROTHERS CARRAWAY, 2000) of "The Practical Approach Series" (IRL PRESS),
"Cytoskeleton Methods and Protocols" (Ed., Gavin, Ray H., 2000) of "Method in Molecular Biology" (Humana Press) series; Nuclear protein and/or RNA synthesis-related protein:
   "Nuclear Receptors" (Ed., DIDIER PICARD, 1999) of "The Practical Approach Series" (IRL PRESS),
   "RNA Processing" (Eds., STEPHEN J. HIGGINS and B. DAVID HAMES, 1994);

### Protein synthesis and/or transport-related protein:

"Membrane Transport" (Ed., STEPHEN A. BALDWIN, 2000) of "The Practical Approach Series" (IRL PRESS),
"Protein Synthesis Methods and Protocols" (Eds., Martin, Robin, 1998) of "Method in Molecular Biology" (Humana Press) series;

### Cellular defense-related protein:

"DNA Repair Protocols" (Henderson, Daryl S., 1999) of "Method in Molecular Biology" (Humana Press) series,
"Chaperonin Protocols" (Eds., Schneider, Christine, 2000); Development and/or differentiation-related protein:
   "Developmental Biology Protocols" (Eds., ROBERT EISENTHAL and MICHAEL J. DANSON, 1992) of "Method in Molecular Biology" (Humana Press) series; DNA- and/or RNA-binding protein:
      "DNA-Protein Interactions Principles and Protocols" (Eds., Kneale, G. Geoff, 1994) of "Method in Molecular Biology" (Humana Press) series,
      "RNA-Protein Interaction Protocols" (Eds., Haynes, Susan R., 1999) ;

### ATP- and/or GTP-binding protein:

"Signal Transduction Protocols" (Eds., David A. Kendall, Stephen J. Hill, 1995) of "Method in Molecular Biology" (Humana Press) series.

When other techniques are used, the activity of a polypeptide can be analyzed according to the description in Methods in Enzymology (Academic Press).

In the above-described categorization, a clone predicted to belong to the secretory and/or membrane protein category refers to a clone having hit data in a homology search with some annotation to suggest that the clone encodes a secretory and/or membrane protein, such as a growth factor, cytokine, hormone, signal, transmembrane, membrane, extracellular matrix, receptor, G-protein coupled receptor, ionic channel, voltage-gated channel, calcium channel, cell adhesion, collagen, and connective tissue protein; or a clone in which the results of PSORT and SOSUI analyses for deduced ORF suggest the presence of a nucleotide sequence encoding a signal sequence or transmembrane region; or a clone in which the results of a domain/motif search with Pfam suggest the presence of domains/motifs that suggest receptors, ion channels, hormones, or growth factors, for example, seven-transmembrane receptors, pancreatic hormone peptides, ion transport proteins, or fibroblast growth factors.

A clone predicted to belong to the glycoprotein-related protein category means a clone having hit data in a homology search with some annotation, such as glycoprotein, suggesting that the clone encodes a glycoprotein-related protein; or a clone in which the results of a domain/motif search with Pfam indicate the presence of domains and motifs such as a glycoprotein or glycosyltransferase that suggest the involvement of glycobiology, for example, immunoglobulin domain or glycosyl transferases group 1.

A clone predicted to belong to the signal transduction-related protein category means a clone having hit data in a homology search with some annotation, such as serine/threonine-protein kinase, tyrosine-protein kinase, SH3 domain, and SH2 domain, suggesting that the clone encodes a signal transduction-related protein; or a clone in which the results of a domain/motif search with Pfam suggest the presence of domains and motifs that suggest a protein kinase, dephosphoenzyme, SH2 domain, or small G protein, for example, eukaryotic protein kinase domain, protein phosphatase 2C, or Ras family.

A clone predicted to belong to the transcription-related protein category means a clone having hit data in a homology search with some annotation, such as transcription regulation, zinc finger, and homeobox, suggesting that the clone encodes a transcription-related protein; or a clone in which the results of a domain/motif search with Pfam suggest the presence of domains and motifs that suggest a transcription factor or transcription-controlling protein, for example, bZIP transcription factor, Zinc finger, or C2H2 type.

A clone predicted to belong to the category of disease-related protein means a clone having hit data in a homology search with some annotation, such as disease mutation and syndrome, suggesting that the clone encodes a disease-related protein; or a clone whose full-length nucleotide sequence has hit data in Swiss-Prot, nr, or RefSeq, where that hit data corresponds to genes or polypeptides which have been deposited in the Online Mendelian Inheritance in Man (OMIM) (http://www.ncbi.nlm.nih.gov/Omim/), the human gene and disease database described later; or a clone in which the results of a domain/motif search with Pfam suggest the presence of domains and motifs, that suggest proteins with disease-specific expression or proteins involved in increasing or decreasing expression (depending on the disease), for example, Wilm's tumor protein or von Hippel-Lindau disease tumor suppressor protein.

A clone predicted to belong to the category of enzyme and/or metabolism-related protein means a clone having hit data in a homology search with some annotation, such as metabolism, oxidoreductase, and E. C. No. (Enzyme commission number), suggesting that the clone encodes an enzyme and/or metabolism-related protein; or a clone in which the results of a domain/motif search with Pfam suggests the presence of domains and motifs that suggest transferase, synthase, or hydrolase, for example, aldehyde dehydrogenase family, chitin synthase, or glucose-6-phosphate dehydrogenase.

A clone predicted to belong to the category of cell division and/or cell proliferation-related protein means a clone having hit data in a homology search with some annotation, such as cell division, cell cycle, mitosis, chromosomal protein, cell growth, and apoptosis, suggesting that the clone encodes a cell division and/or cell proliferation-related protein; or a clone in which the results of a domain/motif search with Pfam suggest the presence of domains and motifs that suggest cyclin or cell proliferation-controlling protein, for example, cyclin or cell division protein.

A clone predicted to belong to the category of cytoskeleton-related protein means a clone having hit data in a homology search with some annotation, such as structural protein, cytoskeleton, actin-binding, and microtubles, suggesting that the clone encodes a cytoskeleton-related protein; or a clone in which the results of a domain/motif search with Pfam suggest the presence of domains and motifs that suggest actin, kinesin, or fibronectin, for example, actin, fibronectin type I domain, or kinesin motor domain.

A clone predicted to belong to the category of nuclear protein and/or RNA synthesis-related protein means a clone having hit data in a homology search with some annotation, such as nuclear protein, RNA splicing, RNA processing, RNA helicase, and polyadenylation, suggesting that the clone encodes a nuclear protein and/or RNA synthesis-related protein; or a clone in which the results of a domain/motif search with Pfam suggest the presence of domains and motifs that suggest splicing factor, RNA synthase, or helicase, for example, hepatitis C virus RNA dependent RNA polymerase or DEAD/DEAR box helicase.

A clone predicted to belong to the category of protein synthesis and/or transport-related protein means a clone having hit data in a homology search with some annotation, such as translation regulation, protein biosynthesis, amino-acid biosynthesis, ribosomal protein, protein transport, and signal recognition particle, suggesting that the clone encodes a protein synthesis and/or transport-related protein; or a clone in which the results of a domain/motif search with Pfam suggest the presence of domains and motifs that suggest translation-relating protein, ubiquitin-relating protein, or ribosomal protein, for example, translation initiation factor SUI1, ubiquitin family, or ribosomal protein L16.

A clone predicted to belong to the category of cellular defense-related protein means a clone having hit data in a homology search with some annotation, such as heat shock, DNA repair, and DNA damage, suggesting that the clone encodes a cellular defense-related protein; or a clone in which the results of a domain/motif search with Pfam suggest the presence of domains and motifs that suggest chaperonin or DNA repair protein, for example, HSP90 protein or DNA mismatch repair protein.

A clone predicted to belong to the category of development and/or differentiation-related proteins means a clone having hit data in a homology search with some annotation, such as developmental protein, suggesting that the clone encodes a development and/or differentiation-related protein; or a clone in which the results of a domain/motif search with Pfam suggest the presence of domains and motifs that suggest organogenesis-relating protein, for example, floricaula/leafy protein.

A clone predicted to belong to the category of DNA- and/or RNA-binding protein means a clone having hit data in a homology search with some annotation, such as DNA-binding, RNA-binding, and such; or a clone in which the results of a domain/motif search with Pfam suggest the presence of domains and motifs that suggest DNA/RNA-relating enzyme group including transcription factor and DNA ligase or Zinc-finger relating protein, for example, transcription factor WhiB, B-box zinc finger, or tRNA synthetases class I (C).

A clone predicted to belong to the category of ATP- and/or GTP-binding protein means a clone having hit data in a homology search with some annotation, such as ATP-binding, GTP-binding, and such; or a clone in which the results of a domain/motif search with Pfam suggest the presence of domains and motifs that suggest ATP/GTP-relating enzyme group including ATPase or G protein, for example, E1-E2 ATPase or Ras family.

It is possible to perform functional analysis of a protein involved in a disease as described above. It is also possible to analyze correlation between a protein's expression or activity and a certain disease by using a specific antibody obtained by using the expressed protein. Alternatively, it is possible to utilize the OMIM database, which is a database of human genes and diseases, to analyze the protein. New information is constantly being deposited into this database. Therefore, it is possible that one skilled in the art will find a new relationship between a particular disease and a gene of the present invention by using the most up-to-date database. Proteins involved in diseases are useful for developing diagnostic markers or medicines for regulation of their expression and activity, or as gene therapy targets.

The proteins may have a variety of functions, including but not limited to the above 14 categories, such as secretory proteins, membrane proteins, signal transduction-related proteins, glycoprotein-related proteins, or transcription-related proteins. When searching OMIM using these keywords, the proteins are revealed to be involved in a great number of diseases (the results of the OMIM search for secretory and membrane proteins are shown below as an Example). Associations between proteins related to signal transduction or transcription and diseases are reported in "Transcription Factor Research-1999" (Fujii, Tamura, Morohashi, Kageyama, and Satake edit, (1999) Jikken-Igaku Zoukan, Vol.17, No.3), and "Gene Medicine" (1999) Vol.3, No.2). As another example and as described in "Biology of Cancer", many proteins are involved in cancers, including enzymes and/or metabolism-related proteins, cytoskeleton-related proteins, cell division and/or cell proliferation-related proteins as well as secretory proteins, membrane proteins, signal transduction-related proteins, glycoprotein-related proteins, transcription-related proteins, (S. Matsubara, 1992) of Life Science series (Shokabo). As the above example clearly demonstrates, not only disease-related proteins but also secretory proteins, membrane proteins, signal transduction-related proteins, glycoprotein-related proteins, and transcription-related proteins are often involved in diseases, and thus such proteins can be useful targets in the field of medical industry.

The results of the OMIM search for secretory and membrane proteins are shown below. The keywords used were:
(1) secretion protein,
(2) membrane protein,
(3) channel, and
(4) extracellular matrix.

Only the OMIM accession numbers are shown in the search results. The first 50 accession numbers displayed in the search results are provided. Using this number, data showing the relationship between a disease and a gene or protein can be seen. OMIM data is renewed daily.
1) Secretion protein
   When searching under these keywords, 436 genes were registered as being associated with disease. The OMIM numbers for 50 of these genes are as follows.
   *604667, *104760, *176860, *139320, *118910, *151675, *107400, *604029, #200100, *177061, *600946, *601693, *139250, *176880, *600998, *603850, *605083, *147572, *179513, *606055, *604028, *125950, *157147, *246700, *602926, *600560, *602421, *603215, 185860, *600174, *179512, *109270, *179511, *179510, *179509, *601146, *604710, *177020, *138120, *170280, *600626, *164160, *168470, *154545, *603831, *601652, *104311, *601489, *603062, *102720
2) Membrane protein
   When searching under these keywords, 1873 genes were registered as being associated with disease. The OMIM numbers for 50 of these genes are as follows.
   *130500, *605704, *305360, *153330, *109270, *173610, *170995, *120920, *170993, *309060, *104776, *602333, *605703, *602690, *605943, *159430, *600897, *606867, *133090, *601178, *602413, *602003, *604405, *605940, *603237, *109280, *606958, *600378, *606959, *602173, *107776, *602334, *125305, *602335, *309845, *601134, *605731, *606795, *185881, *607178, *603177, *154045, *603214, *603718, *606909, *600594, *603241, *606629, *603657, *600182
3) Channel (member of membrane proteins)
   When searching under these keywords, 449 genes were registered as being associated with disease. The OMIM numbers for 50 of these genes are as follows.
   *176266, *600724, *605427, *182390, *123825, *114208, *114206, *114205, *176267, *600053, *601784, *603749, *182392, *600937, *603415, *114204, *114209, *114207, *607370, *604528, *604527, *601011, *600760, *192500, *118425, *600228, *600359, *176261, *602235, *600761, *182389, *300008, *600877, *605692, *300338, *602232, *603537, *182391, *176263, *602343, *601328, *605874, *604385, *603939, *602208, *601534, *601958, *603220, *600504, *607368
4) Extracellular matrix
   When searching under these keywords, 267 genes were registered as being associated with disease. The OMIM numbers for 50 of these genes are as follows.
   *605912, *602201, *603479, *604633, *601418, *601548, *115437, *154870, *120361, *602285, *600754, *602262, *134797, *602261, *603320, *603321, *604871, *604629, *601807, #154700, *128239, *600310, *605470, *185250, *178990, *603767, *120360, *185261, *116935, *607056, *253700, *190180, *600985, *188826, *193300, *276901, *308700, *120150, *602109, *120324, *600514, #177170, #247100, #116920, #200610, *605127, *601313, *601652, *120180, *154790

In addition to these, various keywords shown in the above-mentioned categorizations or others can be used in an OMIM search to reveal involvement in disease.

Further, the use of nucleotide sequences of cDNAs of the present invention enables the expression frequency of genes corresponding to those cDNAs to be analyzed. Gene function can be predicted based on information obtained by expression frequency analysis.

There are several methods for analyzing the expression level of genes involved in disease. Differences in gene expression levels between diseased and normal tissues can be studied by analytical methods using, for example, Northern blotting, RT-PCR, DNA microarrays, etc. (Experimental Medicine, Vol.17, No. 8, 980-1056 (1999); Cell Engineering (additional volume) DNA Microarray and Advanced PCR Methods, Muramatsu & Nawa (eds.), Shujunsya (2000)). In addition to these analysis methods, computer analysis can be used to compare the nucleotide sequences of expressed genes, and hence to analyze expression frequency. For example, in the "BODYMAP" database, gene clones are randomly extracted from cDNA libraries of various tissues and/or cells, clones homologous to each other are assigned to a single cluster based on 3'-end nucleotide sequence homology information, genes are then classified into clusters, and the number of clones in each cluster is compared to gain information on expression frequency (http://bodymap.ims.u-tokyo.ac.jp/).

When these analytical methods result in observation of an explicit difference between gene expression levels in diseased tissues and normal tissues, it can be concluded that the gene is closely involved in the disease or disorder. When gene expression is explicitly different between normal cells and cells reproducing specific disease-associated features, even if they are not diseased tissues, it can be concluded that the gene is closely involved in a disease or disorder.

Of the 1,995 clones whose full-length nucleotide sequences were revealed, genes involved in a particular pathology or function were selected using the database shown below (see Example 8; "Expression frequency analysis *in silico*"). The database used in the analyses of the present invention contains the nucleotide sequences of 1,402,069 clones, a sufficiently large population for analysis. Sequence information in the database was obtained by randomly selecting cDNA clones from cDNA libraries derived from the various tissues and cells shown in Example 1, and determining the 5'-end sequences thereof.

The nucleotide sequence of each clone in this database was then categorized (clustered) based on nucleotide sequence homology determined with a search program. The number of clones belonging to each cluster of each library was determined and normalized; and the ratio of a certain gene in a cDNA library was determined. This analysis provided information on the expression frequency of a gene in the tissue or cell that was the source of the cDNA library.

In order to analyze the expression of genes corresponding to the nucleotide sequences of cDNAs of the present invention in tissues and cells, the libraries from the tissues or cells, which had been used in large-scale cDNA analyses, were taken as subjects for comparison of expression levels between different tissues or cells. Namely, expression frequency was analyzed by comparing the previously normalized values between tissues or cells from which were derived the 600 or more cDNA clones whose nucleotide sequences had been analyzed. This analysis showed that the cDNA clones corresponded to the genes involved in the pathologies and functions indicated below. Each value in Tables 2 to 24 indicated below represents a relative expression frequency; a higher value indicates a higher expression level. Genes included in these Tables do not indicate such a big difference between compared libraries, but when compared with other tissue- or gene-derived libraries based on Example 9, they indicate a significant difference. Thus, these genes are specific to a tissue or cell, and can be considered useful diagnostic markers for disease, as well as useful for analyzing molecular mechanisms.

### Osteoporosis-related genes

Osteoporosis is a pathology in which bones are easily broken owing to an overall decrease in bone components. The onset involves the balance between the functions of osteoblast producing bone and osteoclast absorbing bone, namely bone metabolism. Genes involved in the increase of osteoclasts differentiating from precursor cells of monocyte/macrophage line (Molecular Medicine 38. 642-648. (2001)) are thus genes involved in osteoporosis relevant to bone metabolism.

Nucleotide sequence-based analysis was carried out to identify genes whose expression frequencies were higher or lower in CD34+ cells (cells expressing glycoprotein CD34) treated with osteoclast differentiation factor (Molecular Medicine 38. 642-648. (2001)), compared to untreated CD34+ cells, which are precursor cells in monocyte/macrophage lines. The result of comparative analysis of frequency between the cDNA libraries prepared from the RNA of CD34+ cells (CD34C), and from the RNA of CD34+ cells treated with the osteoclast differentiation factor (D3OST, D6OST or D9OST) (Table 2), showed that the genes whose expression levels differed between the two were twelve clones indicated below.
BRAWH3018063, BRHIP3020046, BRSSN2013696, BRSTN2012069, BRTHA2027229, D9OST2003106 D9OST2003989 D9OST2004417 OCBBF2016928, TESTI4005653, TESTI4013474, THYMU3032798

These clones are involved in osteoporosis.

### Genes involved in neural cell differentiation

Genes involved in neural cell differentiation are useful for treating neurological disease. Genes with varying expression levels in response to induction of cellular differentiation in neural cells are thought to be involved in neurological disease.

A survey was performed for genes whose expression levels varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and cells subjected to differentiation treatment (NT2RP, NT2RI or NT2NE) (Table 3) showed that the genes whose expression levels differed between the two were 102 clones indicated below.
BLADE2004849, BRACE2003628, BRACE2012528, BRAMY2023939, BRAMY2031516, BRAMY4002628, BRAWH3010461, BRAWH3017259, BRAWH3018063, BRAWH3022651, BRAWH3024186, BRCAN2019653, BRCAN2022126, BRCOC2012386, BRHIP3002000, BRHIP3007223, BRHIP3021019, BRSTN2011961, BRSTN2012069, BRTHA2033155, BRTHA3010212, BRTHA3011194, BRTHA3011265, BRTHA3017791, BRTHA3021971, CHONS2002829, CTONG2006235, FCBBF3012443, FEBRA2026582, LIVER2008465, NT2NE2011107, NT2NE2016041, NT2RI2004818, NT2RI2009233, NT2RI2010795, NT2RI2015533, NT2RI2023671, NT2RI2028537, NT2RI3001573, NT2RI3001967, NT2RI3005861, NT2RI3005923, NT2RI3007095, NT2RI3008179, NT2RI3009480, NT2RI3009524, NT2RP7003439, NT2RP7007387, NT2RP7014178, NT2RP7014778, NT2RP7016508, NT2RP7017139, NT2RP7019682, NT2RP7020343, NT2RP8000633, NT2RP8001363, NT2RP8001407, NT2RP8001584, NT2RP8001604, NT2RP8001605, NT2RP8003490, NT2RP8003657, NT2RP8003787, NT2RP8005546, NT2RP8006452, NT2RP8006521, NT2RP8007416, NT2RP8007503, NT2RP8007920, NT2RP8008057, NT2RP8009119, NT2RP8009248, NTONG2008483, OCBBF2003518, OCBBF3001333, OCBBF3004908, PLACE7004103, PROST2017910, SMINT2009292, SPLEN2012571, T1ESE2000904, TESTI4002072, TESTI4002774, TESTI4002799, TESTI4005653, TESTI4007965, TESTI4012960, TESTI4018436, THYMU3001776, THYMU3002887, THYMU3029795, THYMU3041428, THYMU3047115, TRACH3003872, TRACH3004424, TRACH3006717, TRACH3007625, TRACH3009008, TRACH3016805, TRACH3016885, TRACH3026303, UTERU2016669

These genes are neurological disease-related genes.

### Genes involved in Alzheimer's disease

Alzheimer's disease is a cranial neurological disease characterized by memory loss. As the disease advances, patients can no longer support themselves and require nursing. Alzheimer's disease eventually leads to brain atrophication. Environmental factors such as stress, and vascular factors such as hypertension and cholesterolemia, are assumed but not confirmed to contribute to the onset of Alzheimer's disease. Genes whose expression levels differ between normal brain tissues and tissues affected with Alzheimer's disease are expected to be involved in Alzheimer's disease. Such genes can be used to elucidate the disease's onset mechanism and in genetic diagnosis. cDNA libraries derived from the cerebral cortex of Alzheimer patients (BRALZ and BRASW) and a library derived from the whole tissues of a normal brain (BRAWH) were analyzed and compared (Table 4). Genes whose expression levels differed between the two were the 298 clones and five clones listed below.
ASTRO2016114, BRACE2002392, BRACE2012528, BRACE3004371, BRACE3004767, BRACE3022340, BRACE3031185, BRACE3031743, BRACE3032385, BRACE3032631, BRACE3039358, BRACE3040863, BRACE3042432, BRACE3045981, BRALZ2003119, BRALZ2007661, BRALZ2008930, BRALZ2010842, BRALZ2011337, BRALZ2013621, BRALZ2013690, BRALZ2014054, BRAMY2031516, BRAMY3002329, BRAMY3004126, BRAMY3005912, BRAMY3008436, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019653, BRCAN2022126, BRCAN2025093, BRCOC2012386, BRHIP2015153, BRHIP2027077, BRHIP3001573, BRHIP3002000, BRHIP3003063, BRHIP3003984, BRHIP3004774, BRHIP3007223, BRHIP3007409, BRHIP3008320, BRHIP3014675, BRHIP3017855, BRHIP3018784, BRHIP3021019, BRHIP3028246, BRHIP3028570, BRSTN2010089, BRSTN2012069, BRSTN2016992, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, CTONG2006235, CTONG2009033, CTONG2020582, D9OST2003106, DFNES2001829, KIDNE2010049, MESAN2017133, NT2RI2009233, NT2RI2015533, NT2RI3005923, NT2RI3009524, NT2RP7007387, NT2RP8001605, NT2RP8007920, NTONG2008483, OCBBF2003518, OCBBF2018618, OCBBF3001333, OCBBF3004487, PLACE7004103, PLACE7006240, PROST2007444, PROST2017910, TBAES2007428, TESTI2005112, TESTI2021654, TESTI4002072, TESTI4002774, TESTI4002799, TESTI4003602, TESTI4004210, TESTI4005399, TESTI4005653, TESTI4006441, TESTI4013474, TESTI4014908, TESTI4022158, THYMU3000776, THYMU3002887,
THYMU3003350, THYMU3021586, THYMU3026000, THYMU3026306, THYMU3026350, THYMU3032798, THYMU3032867, THYMU3037827, THYMU3038214, THYMU3041428, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAWH3013197, BRAWH3028645, BRTHA3004432, TRACH1000193

These genes are involved in Alzheimer's disease.

### Genes involved in Parkinson's disease

Parkinson's disease is a cranial neurological disease characterized by impaired production of the neurotransmitter dopamine in the substantia nigra in the brain. This results in dyskinesia, such as hand tremors, and impaired body movement due to muscular rigidity. Normally, the number of brain neurons gradually decreases with age. However, compared to healthy people, patients with Parkinson's disease experience a rapid and marked decrease in the number of neurons in their substantia nigra. Genes whose expression levels differ between tissues of the whole brain and the nigra are expected to be involved in Parkinson's disease. These genes exhibit nigra-specific alterations in their expression levels, and can be used to elucidate the disease onset mechanism and in gene diagnosis. cDNA libraries derived from the substantia nigra (BRSSN) and a library derived from whole tissues of a normal brain (BRAWH) were analyzed and compared (Table 5). Genes whose expression levels differed between the two were the 305 clones and five clones listed below.
ASTRO2016114, BRACE2012528, BRACE2017844, BRACE3004371, BRACE3004767, BRACE3022340, BRACE3025719, BRACE3026802, BRACE3031185, BRACE3031743, BRACE3032385, BRACE3032631, BRACE3039358, BRACE3040863, BRACE3042432, BRACE3045981, BRAMY2031516, BRAMY3002329, BRAMY3004126, BRAMY3005912, BRAMY3008436, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2022126, BRCAN2025093, BRCOC2012386, BRHIP2015153, BRHIP2027077, BRHIP2029643, BRHIP3001573, BRHIP3002000, BRHIP3003063, BRHIP3003984, BRHIP3004774, BRHIP3007223, BRHIP3007409, BRHIP3008320, BRHIP3014675, BRHIP3017855, BRHIP3018784, BRHIP3020046, BRHIP3021019, BRHIP3028246, BRHIP3028570, BRSSN2004303, BRSSN2004710, BRSSN2008464, BRSSN2011843, BRSSN2012157, BRSSN2012198, BRSSN2013696, BRSSN2015497, BRSSN2018218, BRSTN2010089, BRSTN2011961, BRSTN2012069, BRSTN2016992, BRSTN2017104, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, CTONG2006235, CTONG2009033, CTONG2011801, CTONG2020582, D9OST2003106 DFNES2001829, KIDNE2010049, MESAN2017133, NT2RI2009233, NT2RI2015533, NT2RI3005923, NT2RI3009524, NT2RP7007387, NT2RP8001605, NT2RP8007920, NTONG2008483, OCBBF2003518, OCBBF2018618, OCBBF3001333, OCBBF3004487, PLACE7004103, PLACE7006240, PROST2007444, SMINT2009292, T1ESE2000904, TBAES2007428, TESTI2005112, TESTI2021654, TESTI4002072, TESTI4002774, TESTI4002799, TESTI4003602, TESTI4004210, TESTI4005399, TESTI4005653, TESTI4006441, TESTI4014908, TESTI4022158, THYMU3000776, THYMU3002887, THYMU3003350, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3022198, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAWH3013197, BRAWH3028645, BRTHA3004432, TRACH1000193

These genes are involved in Parkinson's disease.

### Genes involved in short-term memory and dementia

In the brain, the hippocampus is a highly important memory-related area. The hippocampus functions to establish a memory by judging whether acquired information is necessary, and then accumulating the memory in another area of the brain. According to clinical findings, patients can retain a new memory for only about five minutes with an abnormal, or at worst without a hippocampus. Some dementia patients are presumed to have hippocampus abnormalities. Thus, genes whose expression levels differ between tissues of the whole brain and the hippocampus are expected to be involved in memory or dementia. Such genes can be used to elucidate the mechanism underlying memory, and in gene diagnosis. cDNA libraries derived from the hippocampus (BRHIP) and from the whole tissues of a normal brain (BRAWH) were analyzed and compared (Table 6). Genes whose expression levels differed between the two were the 438 clones and five clones listed below.
ASTRO2016114, BRACE2002392, BRACE2012528, BRACE2017359, BRACE2017397, BRACE2017844, BRACE3004046, BRACE3004371, BRACE3004767, BRACE3009416, BRACE3022340, BRACE3027931, BRACE3029021, BRACE3031185, BRACE3031743, BRACE3032385, BRACE3032631, BRACE3036156, BRACE3039358, BRACE3040863, BRACE3042326, BRACE3042432, BRACE3045078, BRACE3045981, BRAMY2031516, BRAMY3002329, BRAMY3004126, BRAMY3005184, BRAMY3005912, BRAMY3007078, BRAMY3008436, BRAMY4000915, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019953, BRCAN2022126, BRCAN2025093, BRCOC2012386, BRHIP2006819, BRHIP2006921, BRHIP2008756, BRHIP2009177, BRHIP2011199, BRHIP2013958, BRHIP2015153, BRHIP2016125, BRHIP2017714, BRHIP2020930, BRHIP2021929, BRHIP2023735, BRHIP2024941, BRHIP2026346, BRHIP2027077, BRHIP2027563, BRHIP2029529, BRHIP2029643, BRHIP2029663, BRHIP3000626, BRHIP3000859, BRHIP3001076, BRHIP3001141, BRHIP3001338, BRHIP3001360, BRHIP3001481, BRHIP3001573, BRHIP3001878, BRHIP3002000, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003688, BRHIP3003795, BRHIP3003845, BRHIP3003961, BRHIP3003984, BRHIP3004215, BRHIP3004710, BRHIP3004725, BRHIP3004774, BRHIP3004786, BRHIP3005037, BRHIP3005142, BRHIP3005231, BRHIP3005307, BRHIP3005673, BRHIP3005801, BRHIP3005944, BRHIP3006279, BRHIP3006294, BRHIP3006449, BRHIP3006786, BRHIP3006950, BRHIP3007172, BRHIP3007195, BRHIP3007223, BRHIP3007291, BRHIP3007409, BRHIP3007424, BRHIP3007609, BRHIP3007960, BRHIP3008082, BRHIP3008320, BRHIP3008714, BRHIP3009672, BRHIP3009753, BRHIP3010289, BRHIP3010916, BRHIP3011082, BRHIP3011269, BRHIP3011460, BRHIP3011567, BRHIP3011831, BRHIP3012185, BRHIP3012289, BRHIP3012357, BRHIP3012736, BRHIP3012997, BRHIP3013078, BRHIP3013588, BRHIP3013698, BRHIP3014675, BRHIP3015854, BRHIP3016032, BRHIP3016421, BRHIP3017109, BRHIP3017146, BRHIP3017256, BRHIP3017558, BRHIP3017855, BRHIP3018784, BRHIP3019643, BRHIP3019824, BRHIP3019880, BRHIP3019956, BRHIP3020046, BRHIP3020155, BRHIP3020733, BRHIP3021019, BRHIP3021499, BRHIP3021987, BRHIP3022656, BRHIP3023922, BRHIP3024703, BRHIP3024820, BRHIP3025795, BRHIP3025844, BRHIP3026231, BRHIP3026651, BRHIP3027160, BRHIP3027191, BRHIP3027651, BRHIP3027947, BRHIP3028246, BRHIP3028570, BRHIP3028742, BRSTN2010089, BRSTN2012069, BRSTN2016992, BRTHA2001953, BRTHA2008502, BRTHA2031517, BRTHA2033155, BRTHA2035743, BRTHA3003417, BRTHA3003736, BRTHA3005988, BRTHA3007662, BRTHA3010135, BRTHA3010212, BRTHA3011194, BRTHA3011265, BRTHA3012265, BRTHA3017791, BRTHA3020771, BRTHA3021971, BRTHA3023403, CHONS2002829, CTONG2006235, CTONG2009033, CTONG2020582, D9OST2003106, DFNES2001829, KIDNE2010049, LIVER2008465, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2000831, OCBBF2003518, OCBBF2018618, OCBBF3001333, OCBBF3004487, PLACE7004103, PLACE7006240, PROST2007444, SMINT2012179, SYNOV4004210, TBAES2007428, TESTI2005112, TESTI2005564, TESTI2021654, TESTI4001569, TESTI4002072, TESTI4002774, TESTI4002799, TESTI4003602, TESTI4003703, TESTI4003944, TESTI4004210, TESTI4005399, TESTI4005653, TESTI4006441, TESTI4014908, TESTI4022158, TESTI4029297, THYMU3000776, THYMU3002887, THYMU3003007, THYMU3003350, THYMU3007308, THYMU3008105, THYMU3021586, THYMU3026000, THYMU3026306, THYMU3026350, THYMU3032798, THYMU3032867, THYMU3034671, THYMU3037827, THYMU3038214, THYMU3044075, TKIDN2000319, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3022198, TRACH3024342, TRACH3024671, TRACH3025316, TRACH3026303, TRACH3026676, TRACH3028855, TRACH3032570, TRACH3036932, TRACH3038399, TSTOM2000235, UTERU3005422, UTERU3010409, UTERU3013167, UTERU3016273
BRACE3046450, BRAWH3013197, BRAWH3028645, BRTHA3004432, TRACH1000193

These genes are involved in memory and dementia.

### Genes involved in equilibrium sense and movement function

The cerebellum is the center of equilibrium sense, muscular movement, and motor learning. This area is thought to be involved in motor control, and smooth movements are achieved unconsciously due to cerebellum action. Recent studies have elucidated that the cerebellum participates in not only simple movements but also in establishing higher-order movements such as reading and writing. Thus, genes whose expression levels differ between tissues of the whole brain and the cerebellum are expected to be involved in equilibrium sense or motor function, which can be useful for elucidating the molecular mechanism controlled by the brain. cDNA libraries derived from the cerebellum (BRACE) and from the whole tissues of a normal brain (BRAWH) were analyzed and compared (Table 7). Genes whose expression levels differed between the two were the 502 clones and nine clones listed below.
ASTRO2016114, BRACE1000475, BRACE2002392, BRACE2003628, BRACE2005991, BRACE2010336, BRACE2012528, BRACE2012625, BRACE2012833, BRACE2012838, BRACE2012936, BRACE2012947, BRACE2013009, BRACE2013126, BRACE2013132, BRACE2016896, BRACE2017359, BRACE2017397, BRACE2017580, BRACE2017844, BRACE2017872, BRACE2017992, BRACE2019348, BRACE2023633, BRACE2023744, BRACE2025452, BRACE2026404, BRACE2027312, BRACE2027382, BRACE2028956, BRACE2030039, BRACE2032584, BRACE2033128, BRACE2034434, BRACE2035120, BRACE2035191, BRACE2039362, BRACE2039607, BRACE2042541, BRACE2046976, BRACE2047232, BRACE2047975, BRACE3001403, BRACE3001973, BRACE3002344, BRACE3002541, BRACE3002756, BRACE3003866, BRACE3004046, BRACE3004371, BRACE3004767, BRACE3004887, BRACE3004981, BRACE3005870, BRACE3005903, BRACE3006553, BRACE3007649, BRACE3007869, BRACE3009075, BRACE3009265, BRACE3009392, BRACE3009416, BRACE3009539, BRACE3010702, BRACE3011447, BRACE3011774, BRACE3013418, BRACE3013874, BRACE3013986, BRACE3014523, BRACE3014714, BRACE3015090, BRACE3015898, BRACE3016020, BRACE3016167, BRACE3016580, BRACE3016788, BRACE3016810, BRACE3016862, BRACE3017253, BRACE3018083, BRACE3019570, BRACE3019611, BRACE3019817, BRACE3019941, BRACE3020356, BRACE3020669, BRACE3021430, BRACE3021517, BRACE3021805, BRACE3022051, BRACE3022303, BRACE3022312, BRACE3022340, BRACE3022847, BRACE3023604, BRACE3024379, BRACE3024444, BRACE3024497, BRACE3024537, BRACE3024879, BRACE3025627, BRACE3025719, BRACE3026161, BRACE3026290, BRACE3026345, BRACE3026456, BRACE3026802, BRACE3026844, BRACE3026947, BRACE3027256, BRACE3027931, BRACE3028360, BRACE3028895, BRACE3028998, BRACE3029005, BRACE3029021, BRACE3029205, BRACE3029447, BRACE3030538, BRACE3031161, BRACE3031184, BRACE3031185, BRACE3031315, BRACE3031372, BRACE3031579, BRACE3031728, BRACE3031743, BRACE3031843, BRACE3032385, BRACE3032537, BRACE3032538, BRACE3032631, BRACE3032980, BRACE3033525, BRACE3034183, BRACE3034389, BRACE3034964, BRACE3034993, BRACE3035168, BRACE3036156, BRACE3036271, BRACE3036283, BRACE3037612, BRACE3037637, BRACE3037803, BRACE3038012, BRACE3038030, BRACE3038570, BRACE3038760, BRACE3039288, BRACE3039358, BRACE3039378, BRACE3039454, BRACE3040012, BRACE3040239, BRACE3040504, BRACE3040644, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044495, BRACE3045078, BRACE3045145, BRACE3045424, BRACE3045708, BRACE3045981, BRACE3046049, BRACE3046152, BRACE3046294, BRACE3046466, BRACE3046491, BRACE3046609, BRACE3046837, BRACE3046855, BRACE3046966, BRACE3047018, BRACE3047482, BRACE3047801, BRAMY2031516, BRAMY3002329, BRAMY3004126, BRAMY3004364, BRAMY3005912, BRAMY3008436, BRAMY3009491, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019953, BRCAN2022126, BRCAN2025093, BRCOC2012386, BRHIP2015153, BRHIP2027077, BRHIP2029643, BRHIP3001360, BRHIP3001573, BRHIP3002000, BRHIP3002114, BRHIP3003063, BRHIP3003126, BRHIP3003961, BRHIP3003984, BRHIP3004215, BRHIP3004774, BRHIP3005801, BRHIP3007223, BRHIP3007409, BRHIP3008320, BRHIP3014675, BRHIP3017855, BRHIP3018784, BRHIP3021019, BRHIP3028246, BRHIP3028570, BRSSN2011843, BRSSN2013696, BRSTN2010089, BRSTN2011961, BRSTN2012069, BRSTN2016992, BRTHA2033155, BRTHA2035743, BRTHA3003736, BRTHA3005988, BRTHA3009858, BRTHA3010135, BRTHA3010212, BRTHA3010530, BRTHA3011194, BRTHA3011265, BRTHA3011998, BRTHA3017791, BRTHA3020771, BRTHA3021708, BRTHA3021971, BRTHA3023403, CHONS2002829, CTONG2006235, CTONG2009033, CTONG2020582, CTONG2027959, D9OST2003106, DFNES2001829, KIDNE2010049, KIDNE2017153, LIVER2008465, MESAN2017133, NOVAR2000783, NT2RI2009233, NT2RI2015533, NT2RI3005923, NT2RI3009524, NT2RP7007387, NT2RP8001605, NT2RP8007920, NT2RP8009119, NTONG2008483, NTONG2009468, OCBBF2003518, OCBBF2014745, OCBBF2018618, OCBBF3001333, OCBBF3004487, PLACE7004103, PLACE7006240, PROST2007444, TBAES2007428, TESTI2005112, TESTI2018867, TESTI2021654, TESTI4002072, TESTI4002774, TESTI4002799, TESTI4003602, TESTI4003703, TESTI4004210, TESTI4005013, TESTI4005399, TESTI4005653, TESTI4006441, TESTI4014908, TESTI4022158, TESTI4029297, TESTI4032913, TESTI4043223, THYMU3000776, THYMU3002887, THYMU3003350, THYMU3007308, THYMU3021586, THYMU3026000, THYMU3026306, THYMU3026350, THYMU3032798, THYMU3032867, THYMU3033626, THYMU3034671, THYMU3037827, THYMU3038214, THYMU3044075, TLIVE2007736, TRACH2013585, TRACH3002752, TRACH3003037, TRACH3003872, TRACH3004424, TRACH3006717, TRACH3007625, TRACH3007689, TRACH3007995, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3000670, UTERU3010409, UTERU3013167, UTERU3015011
BRACE3002184, BRACE3026993, BRACE3046450, BRAMY3008096, BRAWH3013197, BRAWH3028645, BRTHA3004432, TESTI4002988, TRACH1000193

These genes are involved in equilibrium sense or motor function.

### Genes involved in signaling from sensory organs

The thalamus is an area which comprises many neurons strongly connected to the cerebrum, and which transmits sensory information from the spinal cord or such to the responsible area of the cerebrum. The thalamus also controls the direction of movement from the cerebrum. For example, the thalamus resolves vision into the elements of size, shape, and color, and resolves sound into volume and sweetness or harshness to the ear, and then transmits this information to the sensory area of the cerebral cortex. Thus, genes whose expression levels differ between tissues of the whole brain and the thalamus are expected to be involved in signaling from sensory organs. These genes can be used to elucidate the molecular mechanism underlying signaling controlled by the brain. cDNA libraries derived from the thalamus (BRTHA) and from whole tissues of a normal brain (BRAWH) were analyzed and compared (Table 8). Genes whose expression levels differed between the two were the 440 clones and eight clones listed below.
ASTRO2008972, ASTRO2016114, BLADE2004849, BRACE2002392, BRACE2012528, BRACE2019348, BRACE3004371, BRACE3004767, BRACE3019941, BRACE3022312, BRACE3022340, BRACE3031185, BRACE3031743, BRACE3032385, BRACE3032631, BRACE3036156, BRACE3039358, BRACE3040863, BRACE3042432, BRACE3045981, BRAMY2031516, BRAMY3002329, BRAMY3004126, BRAMY3005912, BRAMY3008436, BRAMY3009556, BRAMY3010654, BRAMY4001863, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2002892, BRCAN2010665, BRCAN2020234, BRCAN2022126, BRCAN2025093, BRCOC2006164, BRCOC2012386, BRHIP2013958, BRHIP2015153, BRHIP2027077, BRHIP3001573, BRHIP3002000, BRHIP3002691, BRHIP3002920, BRHIP3003063, BRHIP3003961, BRHIP3003984, BRHIP3004215, BRHIP3004774, BRHIP3007223, BRHIP3007409, BRHIP3008320, BRHIP3014675, BRHIP3017855, BRHIP3018784, BRHIP3020046, BRHIP3021019, BRHIP3028246, BRHIP3028570, BRSSN2015497, BRSTN2010089, BRSTN2011961, BRSTN2012069, BRSTN2016992, BRTHA2000969, BRTHA2001304, BRTHA2001953, BRTHA2002091, BRTHA2003759, BRTHA2005448, BRTHA2006720, BRTHA2008502, BRTHA2008598, BRTHA2010672, BRTHA2012189, BRTHA2014647, BRTHA2018304, BRTHA2019726, BRTHA2019743, BRTHA2020400, BRTHA2020566, BRTHA2020642, BRTHA2020695, BRTHA2020721, BRTHA2020781, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2028297, BRTHA2029969, BRTHA2030036, BRTHA2030213, BRTHA2031517, BRTHA2031917, BRTHA2032763, BRTHA2033122, BRTHA2033155, BRTHA2033320, BRTHA2033469, BRTHA2033683, BRTHA2034281, BRTHA2034576, BRTHA2035743, BRTHA2036055, BRTHA2036295, BRTHA2037247, BRTHA2038279, BRTHA2038345, BRTHA2038353, BRTHA3000456, BRTHA3002411, BRTHA3003225, BRTHA3003417, BRTHA3003736, BRTHA3005988, BRTHA3006593, BRTHA3007469, BRTHA3007662, BRTHA3009858, BRTHA3010135, BRTHA3010212, BRTHA3010469, BRTHA3010530, BRTHA3010540, BRTHA3010717, BRTHA3011187, BRTHA3011194, BRTHA3011229, BRTHA3011265, BRTHA3011306, BRTHA3011361, BRTHA3011510, BRTHA3011892, BRTHA3011998, BRTHA3012265, BRTHA3013860, BRTHA3013882, BRTHA3014000, BRTHA3014105, BRTHA3014507, BRTHA3014547, BRTHA3014835, BRTHA3014854, BRTHA3014920, BRTHA3016616, BRTHA3017791, BRTHA3018409, BRTHA3018623, BRTHA3019183, BRTHA3020369, BRTHA3020771, BRTHA3021569, BRTHA3021708, BRTHA3021786, BRTHA3021971, BRTHA3022641, BRTHA3023403, BRTHA3023590, BRTHA3023929, BRTHA3024600, BRTHA3025073, BRTHA3026161, BRTHA3026180, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, CHONS2002829, CTONG2006235, CTONG2009033, CTONG2011801, CTONG2020582, D9OST2003106 DFNES2001829, KIDNE2010049, LIVER2008465, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2003518, OCBBF2009536, OCBBF2018618, OCBBF3001333, OCBBF3004487, OCBBF3008835, PLACE6003004, PLACE7004103, PLACE7006240, PROST2007444, SMINT2009292, TBAES2007428, TESTI2005112, TESTI2021654, TESTI2039342, TESTI4002072, TESTI4002774, TESTI4002799, TESTI4003602, TESTI4003703, TESTI4004210, TESTI4004695, TESTI4005399, TESTI4005653, TESTI4006441, TESTI4007965, TESTI4010979, TESTI4013474, TESTI4014908, TESTI4022158, TESTI4029297, TESTI4032913, THYMU3000776, THYMU3002887, THYMU3003350, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008508, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3012414, UTERU3013167, UTERU3017995, UTERU3018172 BRACE3046450, BRAWH3013197, BRAWH3028645, BRTHA3004432, BRTHA3024233, CTONG2002832, THYMU3046350, TRACH1000193

These genes are involved in signaling from sensory organs.

### Genes involved in emotional reaction

The amygdala is the center of emotion in the brain. Information passing through the amygdala induces an emotional reaction, for example, panic or fear. When a strong fear reaction is produced due to the emotional evaluation of stimulus in the amygdala, the amygdala transmits an alert signal to each area of the brain. This results in various reactions such as sweating palms, palpitation, elevated blood pressure, and rapid secretion of adrenaline. In other words, the amygdala transmits signals which cause the body to be on the alert and is a tissue involved in a kind of defense instinct. Thus, genes whose expression levels differ between tissues of the whole brain and the amygdala are expected to be involved in emotional reaction. Such genes can be used to elucidate the molecular mechanism underlying emotional reaction, fear, or panic. cDNA libraries derived from the amygdale (BRAMY) and from whole tissues of a normal brain (BRAWH) were analyzed and compared (Table 9). Genes whose expression levels differed between the two were the 357 clones and nine clones listed below.
ASTRO2016114, BRACE2002392, BRACE2012528, BRACE2017397, BRACE2017844, BRACE3004371, BRACE3004767, BRACE3022340, BRACE3031185, BRACE3031315, BRACE3031743, BRACE3032385, BRACE3032631, BRACE3039358, BRACE3040863, BRACE3042432, BRACE3045981, BRAMY2015516, BRAMY2021098, BRAMY2022320, BRAMY2023939, BRAMY2025495, BRAMY2031516, BRAMY2033895, BRAMY2035801, BRAMY2036254, BRAMY2036266, BRAMY2037609, BRAMY2039630, BRAMY2040915, BRAMY2041347, BRAMY2041384, BRAMY2041507, BRAMY2044686, BRAMY2046489, BRAMY2046537, BRAMY3000692, BRAMY3001409, BRAMY3002329, BRAMY3002508, BRAMY3002886, BRAMY3004126, BRAMY3004364, BRAMY3005184, BRAMY3005656, BRAMY3005912, BRAMY3007078, BRAMY3007449, BRAMY3007471, BRAMY3008436, BRAMY3009158, BRAMY3009491, BRAMY3009556, BRAMY3009904, BRAMY3010321, BRAMY3010603, BRAMY3010654, BRAMY4000915, BRAMY4000962, BRAMY4001234, BRAMY4001652, BRAMY4001863, BRAMY4001913, BRAMY4002575, BRAMY4002628, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2022126, BRCAN2025093, BRCOC2012386, BRHIP2015153, BRHIP2027077, BRHIP3001573, BRHIP3002000, BRHIP3002691, BRHIP3003063, BRHIP3003984, BRHIP3004215, BRHIP3004774, BRHIP3005673, BRHIP3007223, BRHIP3007409, BRHIP3008320, BRHIP3012736, BRHIP3014675, BRHIP3017146, BRHIP3017855, BRHIP3018784, BRHIP3020046, BRHIP3021019, BRHIP3028246, BRHIP3028570, BRSTN2010089, BRSTN2012069, BRSTN2016992, BRTHA2026071, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, BRTHA3026916, CHONS2002829, CTONG2006235, CTONG2009033, CTONG2020582, D9OST2003106, DFNES2001829, KIDNE2010049, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2000831, OCBBF2003518, OCBBF2018618, OCBBF2030927, OCBBF3001333, OCBBF3004487, OCBBF3009244, PLACE6008315, PLACE6010936, PLACE7004103, PLACE7006240, PROST2007444, SPLEN2012571, SYNOV4004210, SYNOV4009575, TBAES2007428, TESTI2005112, TESTI2021654, TESTI4002072, TESTI4002774, TESTI4002799, TESTI4003602, TESTI4003703, TESTI4004210, TESTI4005399, TESTI4005653, TESTI4006441, TESTI4014908, TESTI4022158, TESTI4029297, TESTI4032913, TESTI4043223, TESTI4046073, THYMU3000776, THYMU3002887, THYMU3003350, THYMU3008105, THYMU3019476, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAMY3008096, BRAWH3013197, BRAWH3028645, BRTHA3004432, TESTI4002988, THYMU3046350, TRACH1000193, TRACH3019290

These genes are involved in emotional reaction.

### Cancer-related genes

Cancer tissues are assumed to express a distinct set of genes distinct from normal tissues, and thus expression of these genes can contribute to carcinogenesis in tissues and cells. Thus, genes whose expression patterns in cancer tissues differ from those in normal tissues are cancer-related genes. A search was carried out for genes whose expression levels in cancer tissues differed from those in normal tissues.

The result of comparative analysis of cDNA libraries derived from breast tumor (TBAES) and normal breast (BEAST) (Table 10) showed that the genes whose expression levels differed between the two were the ten and four clones listed below.
BRSTN2011961, BRSTN2012069, TBAES2003917, TBAES2005361, TBAES2007428, TBAES2007548, TBAES2007862, TESTI2005564, TESTI4017854, TRACH3016805
TBAES2004105, TBAES2007379, TBAES2007481, TBAES2008133

The result of comparative analysis of cDNA libraries derived from cervical tumor (TCERX) and normal cervical duct (CERVX) (Table 11) showed that the genes whose expression levels differed between the two were six clones listed below.
BRACE2017397, BRHIP2027077, BRSTN2011961, BRSTN2012069, CERVX2000812, CERVX2000968

The result of comparative analysis of cDNA libraries derived from colon tumor (TCOLN) and normal colon (COLON) (Table 12) showed that the genes whose expression levels differed between the two were ten clones listed below.
BRSTN2011961, BRSTN2012069, COLON2001829, COLON2001866, COLON2004351, COLON2004911, COLON2005623, COLON2005735, OCBBF3001333, SMINT2017964

The result of comparative analysis of cDNA libraries derived from esophageal tumor (TESOP) and normal esophagus (NESOP) (Table 13) showed that the genes whose expression levels differed between the two were the 14 clones listed below.
BRAMY3004364, BRAWH3027533, BRHIP3007223, BRSTN2011961, BRSTN2012069, CTONG2011801, CTONG3002518, SMINT2009292, TESOP2002005, TESOP2003308, TESOP2004110, TESOP2008556, UTERU3015011, UTERU3017995

The result of comparative analysis of cDNA libraries derived from kidney tumor (TKIDN) and normal kidney (KIDNE) (Table 14) showed that the genes whose expression levels differed between the two were the 43 clones listed below.
BRACE2002392, BRACE2012528, BRACE3004371, BRAMY2039630, BRAMY3004364, BRAMY3008436, BRAWH2004078, BRAWH3012662, BRAWH3021574, BRAWH3022651, BRAWH3037428, BRCAN2019953, BRCAN2022126, BRHIP3002000, BRHIP3002691, BRHIP3012997, BRHIP3020046, BRSTN2012069, BRSTN2016992, BRTHA3010212, CTONG2006235, KIDNE2004531, KIDNE2010049, KIDNE2014496, KIDNE2015987, KIDNE2016464, KIDNE2017153, KIDNE2018268, NT2RI2015533, NT2RP7007387, OCBBF3004487, PLACE6008315, SYNOV4004210, TESTI2005112, THYMU3001776, THYMU3002887, THYMU3029795, THYMU3032867, TKIDN2000319, TKIDN2003396, TKIDN2010602, TKIDN2011051, TKIDN2011160

The result of comparative analysis of cDNA libraries derived from liver tumor (TLIVE) and normal liver (LIVER) (Table 15) showed that the genes whose expression levels differed between the two were the 14 and two clones listed below.
BRAWH3022651, BRCAN2020412, BRSTN2012069, BRTHA3003736, CTONG2006235, LIVER2008465, TESTI4013474, THYMU3002887, THYMU3038158, TLIVE2000142, TLIVE2001616, TLIVE2007736, TLIVE2008797, TRACH3027229
THYMU3046350, TLIVE2007192

The result of comparative analysis of cDNA libraries derived from lung tumor (TLUNG) and normal lung (HLUNG) (Table 16) showed that the genes whose expression levels differed between the two were the 17 clones listed below.
BRACE3036283, BRAMY2031516, BRSTN2011961, BRSTN2012069, HLUNG2012600, MESAN2009156, NTONG2008483, PROST2007444, TESTI4003703, TESTI4005653, TESTI4013474, TESTI4029297, THYMU3001776, THYMU3033626, THYMU3034671, THYMU3041428, TRACH3022198

The result of comparative analysis of cDNA libraries derived from ovary tumor (TOVER) and normal ovary (NOVER) (Table 17A) showed the genes whose expression levels differed between the two were the three clones listed below.
BRSTN2012069, NOVAR2000783, THYMU3002887

The result of comparative analysis of cDNA libraries derived from stomach tumor (TSTOM) and normal stomach (STOMA) (Table 18) showed that the genes whose expression levels differed between the two were the nine clones listed below.
BRSTN2012069, CHONS2002829, STOMA2003894, STOMA2004663, THYMU3001776, TSTOM2000235, TSTOM2001571, TSTOM2002611, TSTOM2002682

The result of comparative analysis of cDNA libraries derived from uterine tumor (TUTER) and normal uterus (UTERU) (Table 19) showed that the genes whose expression levels differed between the two were the 128 and three clones listed below.
BRACE2012528, BRACE2017397, BRACE3004371, BRACE3036283, BRACE3040863, BRAMY2031516, BRAMY3005184, BRAWH2004078, BRAWH3004350, BRAWH3022651, BRAWH3024186, BRAWH3029806, BRAWH3031342, BRCAN2022126, BRHIP3001076, BRHIP3002000, BRHIP3002141, BRHIP3005307, BRHIP3007223, BRHIP3017855, BRHIP3020046, BRSTN2010089, BRSTN2011961, BRSTN2012069, BRSTN2016892, BRTHA3003736, BRTHA3011265, BRTHA3023403, BRTHA3027879, CHONS2002829, CTONG2001932, CTONG2003517, CTONG2006235, CTONG2011801, CTONG3002518, DFNES2001829, KIDNE2010049, LIVER2008465, NT2RI3005923, OCBBF3001333, OCBBF3004487, PLACE6008315, PLACE7006240, PROST2007444, SPLEN2012571, SYNOV4000598, SYNOV4009575, T1ESE2000904, TESTI4002072, TESTI4002195, TESTI4002774, TESTI4002799, TESTI4003703, TESTI4003944, TESTI4005399, TESTI4005653, TESTI4024245, TESTI4029297, THYMU3002887, THYMU3021586, THYMU3026350, THYMU3032798, THYMU3034616, THYMU3034671, TRACH3003872, TRACH3005699, TRACH3006800, TRACH3008632, TRACH3009008, TUTER1000014, TUTER2001433, UTERU2000300, UTERU2014998, UTERU2016464, UTERU2016669, UTERU2020226, UTERU2022955, UTERU2023941, UTERU2024042, UTERU2027369, UTERU2028377, UTERU2029660, UTERU2035926, UTERU2037423, UTERU3000670, UTERU3001029, UTERU3001394, UTERU3001946, UTERU3004635, UTERU3005264, UTERU3005422, UTERU3006538, UTERU3006720, UTERU3007108, UTERU3009775, UTERU3010029, UTERU3010409, UTERU3010604, UTERU3010892, UTERU3010919, UTERU3011092, UTERU3011398, UTERU3011558, UTERU3011579, UTERU3011837, UTERU3012293, UTERU3012414, UTERU3012476, UTERU3012599, UTERU3012999, UTERU3013167, UTERU3013302, UTERU3014274, UTERU3014647, UTERU3014906, UTERU3015011, UTERU3015299, UTERU3015647, UTERU3015844, UTERU3016070, UTERU3016273, UTERU3016274, UTERU3016308, UTERU3017441, UTERU3017626, UTERU3017995, UTERU3018172, UTERU3018255 UTERU2017492, UTERU2025415, UTERU2036507

The result of comparative analysis of cDNA libraries derived from tongue cancer (CTONG) and normal tongue (NTONG) (Table 20) showed that the genes whose expression levels differed between the two were the 67 and two clones listed below.
BRACE2012528, BRAMY4001863, BRAWH3021574, BRAWH3022651, BRAWH3024186, BRHIP2027077, BRHIP3001573, BRHIP3002000, BRHIP3007223, BRHIP3012997, BRHIP3020046, BRSSN2013696, BRSTN2011961, BRSTN2012069, BRTHA2027229, BRTHA2033155, BRTHA3011194, BRTHA3022641, CTONG2001932, CTONG2003517, CTONG2006235, CTONG2008989, CTONG2009033, CTONG2009570, CTONG2010330, CTONG2011801, CTONG2012123, CTONG2014206, CTONG2014959, CTONG2020582, CTONG2026987, CTONG2027150, CTONG2027591, CTONG2027783, CTONG2027959, CTONG3001605, CTONG3002518, CTONG3002588, CTONG3003669, CTONG3008223, NT2RI2009233, NTONG2002278, NTONG2003805, NTONG2004829, NTONG2008483, NTONG2009468, OCBBF3004487, PLACE6008315, PLACE7004103, SKNMC2003639, SPLEN2012571, SPLEN2019092, SYNOV4009575, T1ESE2000904, TESTI2005564, TESTI2018867, TESTI4002799, TESTI4005653, TESTI4032913, THYMU3021586, THYMU3047115, TRACH3006717, TRACH3007625, TRACH3016805, TRACH3036932, TRACH3038399, UTERU2000300
CTONG2002832, CTONG2003764

These genes are involved in cancer.

Expression frequency analysis is used when searching for genes involved in development and differentiation, where the expression levels of genes in developing and/or differentiating tissues and/or cells are compared with those in adult tissues and/or cells. Genes involved in tissue development and/or differentiation are genes which participate in tissue construction and function expression. These genes are thus useful genes available for medicine aiming at regeneration of injured tissues.

By using information on gene expression frequency gained from the database of the nucleotide sequences of the 1,402,069 clones as described above, genes whose expression frequencies differed between developing and/or differentiating tissues and/or cells, and adult tissues and/or cells, were analyzed.

The result of comparative analysis of cDNA libraries derived from fetal brain (FCBBF, FEBRA or OCBBF) and adult brain (BRACE, BRALZ, BRAMY, BRAWH, BRCAN, BRCOC, BRHIP, BRSSN, BRSTN or BRTHA) (Table 21) showed that the genes whose expression levels differed between the two were the 916 and 13 clones listed below.
ASTRO2008972, ASTRO2016114, BLADE2004849, BRACE1000475, BRACE2002392, BRACE2003628, BRACE2005991, BRACE2010336, BRACE2012528, BRACE2012625, BRACE2012833, BRACE2012838, BRACE2012936, BRACE2012947, BRACE2013009, BRACE2013126, BRACE2013132, BRACE2016896, BRACE2017359, BRACE2017397, BRACE2017580, BRACE2017844, BRACE2017872, BRACE2017992, BRACE2019348, BRACE2023633, BRACE2023744, BRACE2025452, BRACE2026404, BRACE2027312, BRACE2027382, BRACE2028956, BRACE2030039, BRACE2032584, BRACE2033128, BRACE2034434, BRACE2035120, BRACE2035191, BRACE2039362, BRACE2039607, BRACE2042541, BRACE2046976, BRACE2047232, BRACE2047975, BRACE3001403, BRACE3001973, BRACE3002344, BRACE3002541, BRACE3002756, BRACE3003866, BRACE3004046, BRACE3004371, BRACE3004767, BRACE3004887, BRACE3004981, BRACE3005870, BRACE3005903, BRACE3006553, BRACE3007649, BRACE3007869, BRACE3009075, BRACE3009265, BRACE3009392, BRACE3009416, BRACE3009539, BRACE3010702, BRACE3011447, BRACE3011774, BRACE3013418, BRACE3013874, BRACE3013986, BRACE3014523, BRACE3014714, BRACE3015090, BRACE3015898, BRACE3016020, BRACE3016167, BRACE3016580, BRACE3016788, BRACE3016810, BRACE3016862, BRACE3017253, BRACE3018083, BRACE3019570, BRACE3019611, BRACE3019817, BRACE3019941, BRACE3020356, BRACE3020669, BRACE3021430, BRACE3021517, BRACE3021805, BRACE3022051, BRACE3022303, BRACE3022312, BRACE3022340, BRACE3022847, BRACE3023604, BRACE3024379, BRACE3024444, BRACE3024497, BRACE3024537, BRACE3024879, BRACE3025627, BRACE3025719, BRACE3026161, BRACE3026290, BRACE3026345, BRACE3026456, BRACE3026802, BRACE3026844, BRACE3026947, BRACE3027256, BRACE3027931, BRACE3028360, BRACE3028895, BRACE3028998, BRACE3029005, BRACE3029021, BRACE3029205, BRACE3029447, BRACE3030538, BRACE3031161, BRACE3031184, BRACE3031185, BRACE3031315, BRACE3031372, BRACE3031579, BRACE3031728, BRACE3031743, BRACE3031843, BRACE3032385, BRACE3032537, BRACE3032538, BRACE3032631, BRACE3032980, BRACE3033525, BRACE3034183, BRACE3034389, BRACE3034964, BRACE3034993, BRACE3035168, BRACE3036156, BRACE3036271, BRACE3036283, BRACE3037612, BRACE3037637, BRACE3037803, BRACE3038012, BRACE3038030, BRACE3038570, BRACE3038760, BRACE3039288, BRACE3039358, BRACE3039378, BRACE3039454, BRACE3040012, BRACE3040239, BRACE3040504, BRACE3040644, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044495, BRACE3045078, BRACE3045145, BRACE3045424, BRACE3045708, BRACE3045981, BRACE3046049, BRACE3046152, BRACE3046294, BRACE3046466, BRACE3046491, BRACE3046609, BRACE3046837, BRACE3046855, BRACE3046966, BRACE3047018, BRACE3047482, BRACE3047801, BRALZ2003119, BRALZ2007661, BRALZ2008930, BRALZ2010842, BRALZ2011337, BRALZ2013621, BRALZ2013690, BRALZ2014054, BRAMY2015516, BRAMY2021098, BRAMY2022320, BRAMY2023939, BRAMY2025495, BRAMY2031516, BRAMY2033895, BRAMY2035801, BRAMY2036254, BRAMY2036266, BRAMY2037609, BRAMY2039630, BRAMY2040915, BRAMY2041347, BRAMY2041384, BRAMY2041507, BRAMY2044686, BRAMY2046489, BRAMY2046537, BRAMY3000692, BRAMY3001409, BRAMY3002329, BRAMY3002508, BRAMY3002886, BRAMY3004126, BRAMY3004364, BRAMY3005184, BRAMY3005656, BRAMY3005912, BRAMY3007078, BRAMY3007449, BRAMY3007471, BRAMY3008436, BRAMY3009158, BRAMY3009491, BRAMY3009556, BRAMY3009904, BRAMY3010321, BRAMY3010603, BRAMY3010654, BRAMY4000915, BRAMY4000962, BRAMY4001234, BRAMY4001652, BRAMY4001863, BRAMY4001913, BRAMY4002575, BRAMY4002628, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2000923, BRCAN2002662, BRCAN2002892, BRCAN2003269, BRCAN2003814, BRCAN2006051, BRCAN2006955, BRCAN2007525, BRCAN2008701, BRCAN2009168, BRCAN2010547, BRCAN2010581, BRCAN2010665, BRCAN2015402, BRCAN2015757, BRCAN2018269, BRCAN2018667, BRCAN2019653, BRCAN2019907, BRCAN2019953, BRCAN2020234, BRCAN2020331, BRCAN2020412, BRCAN2020467, BRCAN2020880, BRCAN2021325, BRCAN2021452, BRCAN2021718, BRCAN2022126, BRCAN2025093, BRCAN2027593, BRCAN2028702, BRCOC2001355, BRCOC2002777, BRCOC2006164, BRCOC2006639, BRCOC2006942, BRCOC2009638, BRCOC2010115, BRCOC2012386, BRHIP2006819, BRHIP2006921, BRHIP2008756, BRHIP2009177, BRHIP2011199, BRHIP2013958, BRHIP2015153, BRHIP2016125, BRHIP2017714, BRHIP2020930, BRHIP2021929, BRHIP2023735, BRHIP2024941, BRHIP2026346, BRHIP2027077, BRHIP2027563, BRHIP2029529, BRHIP2029643, BRHIP2029663, BRHIP3000626, BRHIP3000859, BRHIP3001076, BRHIP3001141, BRHIP3001338, BRHIP3001360, BRHIP3001481, BRHIP3001573, BRHIP3001878, BRHIP3002000, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003688, BRHIP3003795, BRHIP3003845, BRHIP3003961, BRHIP3003984, BRHIP3004215, BRHIP3004710, BRHIP3004725, BRHIP3004774, BRHIP3004786, BRHIP3005037, BRHIP3005142, BRHIP3005231, BRHIP3005307, BRHIP3005673, BRHIP3005801, BRHIP3005944, BRHIP3006279, BRHIP3006294, BRHIP3006449, BRHIP3006786, BRHIP3006950, BRHIP3007172, BRHIP3007195, BRHIP3007223, BRHIP3007291, BRHIP3007409, BRHIP3007424, BRHIP3007609, BRHIP3007960, BRHIP3008082, BRHIP3008320, BRHIP3008714, BRHIP3009672, BRHIP3009753, BRHIP3010289, BRHIP3010916, BRHIP3011082, BRHIP3011269, BRHIP3011460, BRHIP3011567, BRHIP3011831, BRHIP3012185, BRHIP3012289, BRHIP3012357, BRHIP3012736, BRHIP3012997, BRHIP3013078, BRHIP3013588, BRHIP3013698, BRHIP3014675, BRHIP3015854, BRHIP3016032, BRHIP3016421, BRHIP3017109, BRHIP3017146, BRHIP3017256, BRHIP3017558, BRHIP3017855, BRHIP3018784, BRHIP3019643, BRHIP3019824, BRHIP3019880, BRHIP3019956, BRHIP3020046, BRHIP3020155, BRHIP3020733, BRHIP3021019, BRHIP3021499, BRHIP3021987, BRHIP3022656, BRHIP3023922, BRHIP3024703, BRHIP3024820, BRHIP3025795, BRHIP3025844, BRHIP3026231, BRHIP3026651, BRHIP3027160, BRHIP3027191, BRHIP3027651, BRHIP3027947, BRHIP3028246, BRHIP3028570, BRHIP3028742, BRSSN2004303, BRSSN2004710, BRSSN2008464, BRSSN2011843, BRSSN2012157, BRSSN2012198, BRSSN2013696, BRSSN2015497, BRSSN2018218, BRSTN2000312, BRSTN2006466, BRSTN2006638, BRSTN2008475, BRSTN2009247, BRSTN2010089, BRSTN2010416, BRSTN2011688, BRSTN2011961, BRSTN2012069, BRSTN2016892, BRSTN2016918, BRSTN2016992, BRSTN2017104, BRSTN2017151, BRSTN2018712, BRTHA2000969,
BRTHA2001304, BRTHA2001953, BRTHA2002091, BRTHA2003759, , BRTHA2005448, BRTHA2006720, BRTHA2008502, BRTHA2008598, BRTHA2010672, BRTHA2012189, BRTHA2014647, BRTHA2018304, BRTHA2019726, BRTHA2019743, BRTHA2020400, BRTHA2020566, BRTHA2020642, BRTHA2020695, BRTHA2020721, BRTHA2020781, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2028297, BRTHA2029969, BRTHA2030036, BRTHA2030213, BRTHA2031517, BRTHA2031917, BRTHA2032763, BRTHA2033122, BRTHA2033155, BRTHA2033320, BRTHA2033469, BRTHA2033683, BRTHA2034281, BRTHA2034576, BRTHA2035743, BRTHA2036055, BRTHA2036295, BRTHA2037247, BRTHA2038279, BRTHA2038345, BRTHA2038353, BRTHA3000456, BRTHA3002411, BRTHA3003225, BRTHA3003417, BRTHA3003736, BRTHA3005988, BRTHA3006593, BRTHA3007469, BRTHA3007662, BRTHA3009858, BRTHA3010135, BRTHA3010212, BRTHA3010469, BRTHA3010530, BRTHA3010540, BRTHA3010717, BRTHA3011187, BRTHA3011194, BRTHA3011229, BRTHA3011265, BRTHA3011306, BRTHA3011361, BRTHA3011510, BRTHA3011892, BRTHA3011998, BRTHA3012265, BRTHA3013860, BRTHA3013882, BRTHA3014000, BRTHA3014105, BRTHA3014507, BRTHA3014547, BRTHA3014835, BRTHA3014854, BRTHA3014920, BRTHA3016616, BRTHA3017791, BRTHA3018409, BRTHA3018623, BRTHA3019183, BRTHA3020369, BRTHA3020771, BRTHA3021569, BRTHA3021708, BRTHA3021786, BRTHA3021971, BRTHA3022641, BRTHA3023403, BRTHA3023590, BRTHA3023929, BRTHA3024600, BRTHA3025073, BRTHA3026161, BRTHA3026180, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, CHONS2002829, CTONG2001932, CTONG2006235, CTONG2009033, CTONG2011801, CTONG2020582, CTONG2027959, D9OST2003106 DFNES2001829, FCBBF3001018, FCBBF3002188, FCBBF3005160, FCBBF3012443, FCBBF3020030, FCBBF3021191, FCBBF3024911, FCBBF5000384, FEBRA2000805, FEBRA2002260, FEBRA2012625, FEBRA2013069, FEBRA2013570, FEBRA2017736, FEBRA2017811, FEBRA2023498, FEBRA2026582, FEBRA2026977, FEBRA2028222, FEBRA2028457, JCMLC2000273, KIDNE2010049, KIDNE2017153, LIVER2008465, MESAN2017133, NOVAR2000783, NT2NE2011107, NT2RI2009233, NT2RI2010795, NT2RI2015533, NT2RI3005923, NT2RI3009524, NT2RP7007387, NT2RP8001604, NT2RP8001605, NT2RP8007920, NT2RP8009119, NTONG2008483, NTONG2009468, OCBBF2000831, OCBBF2003518, OCBBF2004478, OCBBF2007039, OCBBF2009536, OCBBF2014745, OCBBF2016928, OCBBF2018229, OCBBF2018618, OCBBF2019761, OCBBF2024589, OCBBF2024779, OCBBF2025631, OCBBF2030927, OCBBF2036019, OCBBF3000743, OCBBF3000830, OCBBF3001076, OCBBF3001202, OCBBF3001333, OCBBF3001616, OCBBF3003745, OCBBF3004487, OCBBF3004908, OCBBF3005330, OCBBF3005843, OCBBF3006986, OCBBF3007078, OCBBF3007704, OCBBF3008392, OCBBF3008835, OCBBF3009244, PLACE5000492, PLACE6003004, PLACE6008315, PLACE6010936, PLACE7004103, PLACE7006240, PROST2007444, PROST2017910, SMINT2009292, SMINT2012179, SPLEN2012571, SYNOV4004210, SYNOV4009575, T1ESE2000609, T1ESE2000904, TBAES2007428, TESTI2005112, TESTI2005564, TESTI2009497, TESTI2018867, TESTI2021654, TESTI2039342, TESTI4001569, TESTI4002072, TESTI4002195, TESTI4002774, TESTI4002799, TESTI4003602, TESTI4003703, TESTI4003944, TESTI4004210, TESTI4004695, TESTI4005013, TESTI4005399, TESTI4005653, TESTI4006441, TESTI4007965, TESTI4010979, TESTI4012960, TESTI4013474, TESTI4014908, TESTI4020596, TESTI4022158, TESTI4029297, TESTI4032913, TESTI4035770, TESTI4043223, TESTI4046073, THYMU3000776, THYMU3002887, THYMU3003007, THYMU3003350, THYMU3007308, THYMU3008105, THYMU3019476, THYMU3021586, THYMU3026000, THYMU3026306, THYMU3026350, THYMU3032798, THYMU3032867, THYMU3033626, THYMU3034671, THYMU3037827, THYMU3038214, THYMU3041428, THYMU3044075, TKIDN2000319, TLIVE2007736, TRACH2013585, TRACH3002752, TRACH3003037, TRACH3003872, TRACH3004424, TRACH3006717, TRACH3007625, TRACH3007689, TRACH3007995, TRACH3008508, TRACH3008632, TRACH3009008, TRACH3010079, TRACH3016805, TRACH3016885, TRACH3021544, TRACH3022109, TRACH3022198, TRACH3024342, TRACH3024671, TRACH3025316, TRACH3026303, TRACH3026676, TRACH3028855, TRACH3032570, TRACH3036932, TRACH3038399, TSTOM2000235, UTERU2000300, UTERU2027369, UTERU3000670, UTERU3005422, UTERU3010409, UTERU3012414, UTERU3013167, UTERU3015011, UTERU3016273, UTERU3017995, UTERU3018172 BRACE3002184, BRACE3026993, BRACE3046450, BRAMY3008096, BRAWH3013197, BRAWH3028645, BRTHA3004432, BRTHA3024233, CTONG2002832, TESTI4002988, THYMU3046350, TRACH1000193, TRACH3019290

The result of comparative analysis of cDNA libraries derived from fetal heart (FEHRT) and adult heart (HEART) (Table 22) showed that the genes whose expression levels differed between the two were the ten clones listed below.
BRACE2012528, BRACE3004371, BRCAN2003814, BRSTN2011961, BRSTN2012069, BRSTN2016992, HEART2002531, NTONG2008483, PROST2002078, T1ESE2000609

The result of comparative analysis of cDNA libraries derived from fetal kidney (FEKID) and adult kidney (KIDNE) (Table 23) showed that the genes whose expression levels differed between the two were the 21 clones listed below. BRACE3004371, BRAMY2039630, BRAMY3004364, BRAWH2004078, BRHIP3002000, BRSTN2011961, BRSTN2012069, BRTHA2027229, KIDNE2004531, KIDNE2010049, KIDNE2014496, KIDNE2015987, KIDNE2016464, KIDNE2017153, KIDNE2018268, NT2RP7007387, TESTI2005112, TESTI4002799, THYMU3001776, THYMU3029795, THYMU3032867

The result of comparative analysis of cDNA libraries derived from fetal lung (FELNG) and adult lung (HLUNG) (Table 24) showed that the genes whose expression levels differed between the two were the 18 clones listed below.
BRACE3036283, BRAMY2031516, BRSTN2011961, BRSTN2012069, HLUNG2012600, MESAN2009156, NTONG2008483, PROST2007444, TESTI4003703, TESTI4005653, TESTI4013474, TESTI4029297, THYMU3001776, THYMU3033626, THYMU3034671, THYMU3041428, THYMU3044188, TRACH3022198

These genes are involved in regeneration of tissues and/or cells.

For example, if a polypeptide encoded by a cDNA of the present invention is a regulatory factor for cellular conditions such as growth and differentiation, it can be used for developing medicines as follows. The polypeptide or antibody provided by the present invention is injected into cells using microinjection, and then low molecular weight compounds and such can be screened by using, as an index, change in cellular conditions (such as growth and differentiation), or activation/inhibition of a particular gene in the cell. The screening can be performed as follows.

A polypeptide of the present invention is first expressed and purified as a recombinant. This purified polypeptide is then microinjected into cells such as various cell lines, or primary culture cells, and cellular changes such as growth and differentiation are examined. Alternatively, induction of a gene whose expression is known to be involved in the change of a particular cellular condition may be detected using mRNA or polypeptide amounts. Alternatively, the amount of an intracellular molecule (low molecular weight compounds, etc.) may be detected, where that amount is changed by the function of a gene product (polypeptide) known to influence change in a particular cellular condition. The compounds to be screened (including both low and high molecular compounds) can be added to culture media and screened using, as an index, their ability to change a cellular condition.

Cell lines introduced with a gene of the present invention can be used for screening, even without microinjection. If the product of a gene of the present invention is revealed to be involved in a particular change in cellular conditions, the change in that product can be used as an index for screening. Once a compound which can activate or inhibit the function of a polypeptide of the present invention has been developed using this screening, such a compound can be practically applied in medicines.

If a polypeptide encoded by a cDNA of the present invention is a secretory or membrane protein, or a protein involved in signal transduction, glycoproteins, transcription, or disease, it can be used in functional assays for developing medicines as described below.

In case of a membrane protein, the polypeptide is very likely to function as a receptor or ligand on the cell surface. Therefore, it is possible to reveal new ligand-receptor relationships by screening membrane proteins of the present invention, based on binding activity with known or new ligands or receptors. Screening can be performed according to known methods.

For example, a ligand against a polypeptide of the present invention can be screened in the following manner. Namely, a ligand that binds to a specific polypeptide can be screened using a method comprising the steps of: (a) contacting a test sample with a polypeptide of the present invention, or partial peptide thereof, or cells expressing such, and (b) selecting a test sample that binds to said polypeptide, said partial peptide, or said cells.

Screening using cells expressing receptor polypeptides of the present invention can also be performed, for example, as follows. Receptors capable of binding to a specific polypeptide can be screened by (a) attaching sample cells to a polypeptide of the present invention or its partial peptide, and (b) selecting cells that can bind to the said polypeptide or its partial peptide.

For example, screening can be carried out as follows: a polypeptide of the present invention is first expressed, the recombinant polypeptide is purified and labeled, a binding assay is performed using various cell lines or primary cultured cells, and cells that express the receptor are selected (Growth and differentiation factors and their receptors, Shin-Seikagaku Jikken Kouza Vol.7 (1991) Honjyo, Arai, Taniguchi, and Muramatsu edit, p203-236, Tokyo-Kagaku-Doujin). A polypeptide of the present invention can be labeled with RI such as ¹²⁵I, and enzyme (alkaline phosphatase etc.).

Alternatively, a polypeptide of the present invention may be used without labeling and then detected by using a labeled antibody against that polypeptide. Cells expressing a receptor polypeptide of the present invention, and selected using the above screening methods, can be applied as mentioned below to screen agonists or antagonists of that receptor.

Once a ligand binding to a polypeptide of the present invention, a receptor of that polypeptide, or cells expressing that receptor have been obtained by screening as described above, a compound binding to that ligand or receptor can be screened. It is also possible to screen a compound that can inhibit both bindings (agonists or antagonists of the receptor, for example) by using this binding activity as an index.

When a polypeptide of the present invention is a receptor, the screening method comprises the steps of (a) contacting the ligand with a polypeptide of the present invention, or cells expressing that polypeptide, in the presence of a test sample, (b) detecting binding activity between that polypeptide or cells expressing that polypeptide and the ligand, and (c) selecting a compound that can reduce that binding activity when compared to activity in the absence of the test sample. Furthermore, when a polypeptide of the present invention is a ligand, the screening method comprises the steps of (a) contacting the polypeptide of the present invention with its receptor or cells expressing that receptor, in the presence of a test sample, (b) detecting binding activity between the polypeptide and its receptor, or cells expressing that receptor, and (c) selecting a compound that can reduce that binding activity compared to activity in the absence of the test sample.

Examples of test samples to screen include, but are not limited to, cell extracts, expressed gene library products, synthesized low molecular compounds, synthesized peptides, and natural compounds. A compound that is isolated by the above screening can also be used as a test sample, using binding activity with a polypeptide of the present invention as an index.

A compound isolated using this screening may be a candidate agonist or antagonist of a polypeptide or polypeptide receptor of the present invention. By monitoring changes in intracellular signals, such as phosphorylation due to reduced binding between a polypeptide and its receptor, it is possible to identify whether the obtained compound is an agonist or antagonist of the polypeptide receptor of the present invention. Also, the screened compound may be a candidate for a compound that can inhibit interaction between a polypeptide and its associated molecules (including receptors) *in vivo*. The polypeptides of this invention, recepters that bind to those polypeptides or ligands, and compounds thereof, can be applied in the development of preventative, therapeutic or testing agents for diseases in which the polypeptides of the present invention are involved.

Secretory proteins may regulate cellular conditions such as growth and differentiation. A novel factor that regulates cellular conditions can be found by i) adding a secretory protein of the present invention to a certain kind of cell, and ii) screening using as an index cellular changes in growth or differentiation, or activation of a particular gene.

Screening can be performed, for example, as follows. First, a polypeptide of the present invention is expressed and purified in a recombinant form. Then, the purified polypeptide is added to various cell lines or primary cultured cells, and changes in cell growth and differentiation are monitored. The induction of a particular gene known to be involved in a certain cellular change is detected using mRNA and polypeptide amounts. Alternatively, detection can be carried out using the amount of an intracellular molecule (low-molecular-weight compounds, etc.) changed by the action of a gene product (polypeptide) which influences a certain cellular change.

If screening reveals that a polypeptide of the present invention can regulate cellular conditions or functions, it is possible to apply that polypeptide as a pharmaceutical and diagnostic medicine for related diseases, either directly or by altering a part of it into an appropriate composition.

As for membrane proteins as described above, a secretory protein provided by the present invention may be used to explore a novel ligand-receptor interaction by screening based on binding activity to a known or new ligand or receptor. A similar method can be used to identify an agonist or antagonist. Compounds obtained by these methods are candidate compounds for inhibiting the interaction between the polypeptide of the present invention and an interacting molecule (including receptors). These compounds may be applied as preventive, therapeutic, or testing agents for diseases in which the polypeptide plays a role.

Proteins involved in signal transduction or transcription may be factors that affect a certain polypeptide or gene in response to intra- or extra-cellular stimuli. A novel factor able to affect a polypeptide or gene can be found by expressing a polypeptide provided by the present invention in a certain type of cell, and screening using as an index the activation of a certain intracellular polypeptide or gene.

Screening may be performed as follows. First, a transformed cell line which expresses a polypeptide of the present invention is obtained. Then, changes in a certain gene between the transformed and the original untransformed cell lines are detected using mRNA or polypeptide amounts. Alternatively, detection may be carried out using the amount of an intracellular molecule (low molecular weight compounds, etc.) changed by the function of a certain gene product (polypeptide). Furthermore, change in expression of a certain gene can be detected by estimating the activity of a marker gene product (polypeptide), where the polypeptide of the present invention is expressed in a cell that has been introduced with a fusion gene comprising a regulatory region of the certain gene and a marker gene (luciferase, β-galactosidase, etc.).

If the polypeptide or gene influenced by a protein of the present invention is involved in disease, it is possible to screen a gene or compound that can regulate that polypeptide or gene's expression and/or activity, either directly or indirectly, by utilizing a polypeptide of the present invention.

For example, a polypeptide of the present invention is expressed and purified as a recombinant polypeptide, and a polypeptide or gene that interacts with that polypeptide is purified and screened based on binding. Alternatively, changes in binding activity can be monitored after adding a candidate inhibitor compound. In another method, the 5'-upstream transcription regulatory region of a gene that encodes a polypeptide of the present invention and that can regulate the expression of another gene, is obtained. The gene is fused with a marker gene and then introduced into a cell, compounds and the like are added, and a factor which can regulate expression of that gene can be discovered.

A compound obtained by this screening can be used for developing pharmaceutical medicines for a disease in which a polypeptide of the present invention is involved. Similarly, if a regulatory factor obtained by screening turns out to be a polypeptide, compounds that can newly affect the expression or activity of this polypeptide may be used as a medicine for diseases in which the polypeptide of the present invention is involved.

If a polypeptide of the present invention has enzymatic activity, regardless of whether it is a secretory protein, membrane protein, or a protein involved in signal transduction, glycoprotein, transcription, or disease, screening may be performed by adding a compound to the polypeptide of the present invention under suitable conditions, and monitoring that compound's change. This enzymatic activity may also be used as an index to screen a compound that inhibits the polypeptide activity.

In an example of this screening, a polypeptide of the present invention is expressed and the recombinant polypeptide is purified. Then, compounds are contacted with the purified polypeptide, and the amount of compound and reaction product is examined. Alternatively, inhibitor candidate compounds are added, then a compound (substrate) that reacts with the purified polypeptide is added, and change in the amount of substrate and reaction product is examined.

A compound obtained by screening may be used as a medicine for diseases in which a polypeptide of the present invention is involved. Also such a compound can be applied in tests that examine, for example, whether a polypeptide of the invention functions normally *in vivo*.

Whether a secretory protein, membrane protein, signal transduction-related protein, glycoprotein-related protein, or transcription-related protein of the present invention is a novel protein involved in disease or not is determined using different method to that described above. In this method, a specific antibody against a polypeptide of the present invention is obtained, and the relationship between the expression or activity of the polypeptide and a certain disease is examined. Alternatively, analysis can use the methods in "Molecular Diagnosis of Genetic Diseases" as a reference (Elles R. edit, (1996) in the series of "Method in Molecular Biology" (Humana Press).

Proteins involved in disease are very useful in developing drugs which regulate their expression and activity, and become targets of the above-mentioned screenings. They are also useful in the medicinal industry as diagnostic markers for their related disease, or as gene therapy targets.

A compound isolated as mentioned above can be administered to patients as is, or after being formulated into a pharmaceutical composition according to known methods. Specific examples of pharmaceutically acceptable carriers or vehicles include sterilized water, saline, plant oils, emulsifiers, suspending agents and the like, where they are mixed with the compound appropriately. The pharmaceutical compositions can be administered to patients by a method known to those skilled in the art, such as intraarterial, intravenous, or subcutaneous injection. Dosage may vary depending on the weight or age of a patient, or the method of administration, but those skilled in the art can properly choose an appropriate dosage. If a compound is encoded by a polynucleotide, the polynucleotide can be cloned into a gene therapy vector, and used for gene therapy. Those skilled in the art can properly choose the dosage of the polynucleotide and the method of its administration, and these may vary depending on the weight, age or symptoms of a patient.

The present invention further relates to databases comprising at least one sequence of a polynucleotide and/or polypeptide, or a medium recorded in such databases, selected from the sequence data of the nucleotides and/or amino acids indicated in Table 1. The term "database" means a set of accumulated, machine-searchable and readable nucleotide sequence information. The databases of the present invention comprise at least one of the novel nucleotide sequences of the polynucleotides provided by the present invention. The databases of the present invention can consist only of the sequence data of the novel polynucleotides provided by the present invention, or can comprise other information on known nucleotide sequences of full-length cDNAs or ESTs. The databases of the present invention can be comprised of not only information on nucleotide sequences, but also information on gene functions as revealed by the present invention. Additional information such as the names of DNA clones carrying the full-length cDNAs can be recorded or linked together with the sequence data in the databases.

The database of the present invention is useful for gaining complete gene sequence information from the partial sequence information of a gene of interest. The database of the present invention comprises nucleotide sequence information of full-length cDNAs. Consequently, by comparing information in this database with the nucleotide sequence of a partial gene fragment yielded by differential display method or subtraction method, information on the full-length nucleotide sequence of interest can be gained from the sequence of the partial fragment as a starting clue.

Sequence information of the full-length cDNAs constituting the database of the present invention contains information on not only complete sequences, but also on gene expression frequency and gene homology to known genes and polypeptides. This extra information facilitates rapid functional analyses of partial gene fragments. Further, information on human genes is accumulated in the database of the present invention, and therefore, the database is useful for isolating a human homologue of a gene originating from another species. The human homologue can be isolated based on the nucleotide sequence of the gene from the original species.

At present, information on a wide variety of gene fragments can be obtained by differential display method and subtraction method. In general, these gene fragments are utilized as tools for isolating the full-length sequences thereof. When the gene fragment corresponds to a known gene, the full-length sequence is easily obtained by comparing the partial sequence with the information in known databases. However, when there is no information corresponding to the partial sequence of interest in known databases, cDNA cloning should be carried out for the full-length cDNA. It is often difficult to obtain the full-length nucleotide sequence using partial sequence information as an initial clue. If the full-length gene is not available, the amino acid sequence of the polypeptide encoded by that gene remains unidentified. Thus the database of the present invention can contribute to the identification of full-length cDNAs corresponding to gene fragments, which cannot be revealed using databases of known genes.

The present invention has provided 1,995 polynucleotides. In humans, where isolation of full-length cDNA has not progressed, the provision of new full-length cDNAs has great significance. Secretory proteins, membrane proteins, signal transduction-related proteins, glycoprotein-related proteins, transcription-related proteins, and so on are known to be involved in many diseases. The genes and proteins involved in diseases are useful for developing diagnostic markers or medicines for regulation of their expression and activity, or as a gene therapy target.

In particular, cDNA encoding secretory proteins of the present invention are extremely important to the industry since these proteins are expected to be useful as pharmaceutical agents, and many disease-related genes can be included with them. In addition, membrane proteins, signal transduction-related proteins, transcription-related proteins, disease-related proteins, and genes encoding these proteins can be used as disease indexes, etc. These cDNAs are also very important to the industry, and are expected to be effective in treating diseases and the like by regulating the activity or expression of the proteins they encode.

### [Industrial Applicability]

Human cDNAs involved in various diseases, morbid states, or functions were isolated in the present invention. The cDNAs of the present invention include, for example, genes that can be useful as diagnostic markers or therapeutic targets for the diseases or morbid states shown below. These diseases receive widespread attention, and require the development of new technology for treatment and diagnosis.
- osteoporosis
- neurologic diseases
- Alzheimer's disease
- Parkinson's disease
- dementia
- various cancers

In addition, human cDNAs involved in the functions shown below were isolated in the present invention. These genes can be used to elucidate the mechanisms of various functions, and in therapeutic methods which enhance or repress those functions. For example, genes participating in tissue generation and functional expression can be used in regenerative medicines.
- emotional reaction
- tissue generation and functional expression
- motor function controlled by the brain, signaling function controlled by the brain
- emotional reaction, fear response, and panic

Furthermore, information on the nucleotide sequence of the full-length cDNAs or their full-length amino acid sequences as provided by the present invention can be used to isolate human genes based on partial sequence information obtained by functional analysis of various genes, or from sequence information of genes from non-human organisms.

Any patents, patent applications, and publications cited herein are incorporated by reference.

The present invention is illustrated more specifically with reference to the following examples, but is not to be construed as being limited thereto.

### EXAMPLE 1

### Preparation of cDNA library by oligo-capping

### (1) Extraction and purchase of mRNA

Total RNAs as mRNA sources were extracted from human tissues (shown below) by the method described in the reference (J. Sambrook, E. F. Fritsch & T. Maniatis, Molecular Cloning Second edition, Cold Spring harbor Laboratory Press, 1989). Further, by the method described in the reference (J. Sambrook, E. F. Fritsch & T. Maniatis, Molecular Cloning Second edition, Cold Spring harbor Laboratory Press, 1989), total RNAs as mRNA sources were extracted from human culture cells and human primary culture cells (shown below) cultivated by the methods described in the catalogs.

The library names and origins are indicated below in the order of "Library name: Origin". When a library was prepared by the subtraction method, the item is followed by a description of how to prepare the subtracted library.

### <Extraction of mRNA from human tissues>

NTONG: Normal tongue;
CTONG: Tongue cancer;
FCBBF: Fetal brain;
OCBBF: Fetal brain;
PLACE: Placenta;
SYNOV: Synovial membrane tissue (from rheumatioid arthritis);
CORDB: Cord blood.
<Extraction of mRNA from culture cells>
BNGH4: H4 cells (ATCC #HTB-148);
IMR32: IMR32 cells (ATCC #CCL-127);
SKNMC: SK-N-MC cells (ATCC #HTB-10);
3NB69: NB69 cells (RCB #RCB0480);
BGGI1: GI1 cells (RCB #RCB0763);
NB9N4: NB9 cells (RCB #RCB0477);
SKNSH: SK-N-SH cells (RCB #RCB0426);
AHMSC: Human mesenchymal cells (HMSC);
CHONS: Chondrocytes;
ERLTF: TF-1 cells (Erythroleukemia);
HELAC: HeLa cells;
JCMLC: Myelogenous leukemia cells;
MESTC: Mesenchyme stem cells;
N1ESE: Mesenchymal stem cells;
NCRRM: Embryonal carcinomas;
NCRRP: Embryonal carcinomas treated with retinoic acid (RA) to induce the differentiation;
T1ESE: Mesenchymal stem cells treated with trichostatin and 5-azacytidine to induce the differentiation;
NT2RM: NT2 cells (STARATAGENE #204101);
NT2RP: NT2 cells treated with RA for 5 weeks to induce the differentiation;
NT2RI: NT2 cells treated with RA for 5 weeks to induce the differentiation, followed by the treatment with the growth inhibitor for 2 weeks;
NT2NE: NT2 cells were treated with RA and the growth inhibitor for the neuronal differentiation, and the resultant neurons were concentrated and harvested (NT2 Neuron);
NTISM: NT2 cells (STARATAGENE #204101) were treated with RA for five weeks to induce the differentiation, and then treated with the growth inhibitor for two weeks; mRNA was prepared from the cells and a cDNA library was constructed from the mRNA; the cDNA libraries whose nucleotide sequences were shared by those of mRNAs from undifferentiated NT2 cells were subtracted by using a Subtract Kit (Invitrogen #K4320-01); the subtracted library (NT2RI-NT2RM) was provided by this procedure.

RCB indicates that the cell was provided by the Cell Bank, RIKEN GENE BANK, The Institute of Physical and Chemical Research; ATCC indicates that the cell was provided by American Type Culture Collection.

### <Extraction of mRNA from primary culture cells>

ASTRO: Normal human astrocyte NHA5732, Takara Shuzo #CC2565;
DFNES: Normal human dermal fibroblast (neonatal skin); NHDF-Neo NHDF2564, Takara Shuzo #CC2509;
MESAN: Normal human mesangial cell NHMC56046-2, Takara Shuzo #CC2559;
NHNPC: Normal human neural progenitor cell NHNP5958, Takara Shuzo #CC2599;
PEBLM: Normal human peripheral blood mononuclear cell HPBMC5939, Takara Shuzo #CC2702;
HSYRA: Human synoviocyte HS-RA (from rheumatoid arthritis), Toyobo #T404K-05;
PUAEN: Normal human pulmonary artery endothelial cells, Toyobo #T302K-05;
UMVEN: Normal human umbilical vein endothelial cell HUVEC, Toyobo #T200K-05;
HCASM: Normal human coronary artery smooth muscle cell HCASMC, Toyobo #T305K-05;
HCHON: Normal human chondrocyte HC, Toyobo #T402K-05;
HHDPC: Normal human dermal papilla cell HDPC, Toyobo #THPCK-001; CD34C: CD34+ cells (AllCells, LLC #CB14435M);
D3OST: CD34+ cells treated with the osteoclast differentiation factor (ODF) for three days to induce the differentiation;
D6OST: CD34+ cells treated with ODF for six days to induce the differentiation;
D9OST: CD34+ cells treated with ODF for nine days to induce the differentiation;
ACTVT: Activated T-cells;
LYMPB: Lymphoblasts (EB virus transferred B cells);
NETRP: Neutrophils.

Total RNAs extracted from the following human tissues were then purchased and used as mRNA sources. Library names and the origins are indicated below in the order of "Library name: Origin". When a library was prepared by the subtraction method, the item is followed by a description of how to prepare the subtracted library.

### <Purchase of total RNA containing mRNA extracted from human tissues>

ADRGL: Adrenal gland, CLONTECH #64016-1;
BRACE: Brain (cerebellum), CLONTECH #64035-1;
BRAWH: Whole brain, CLONTECH #64020-1;
FEBRA: Fetal brain, CLONTECH #64019-1;
FELIV: Fetal liver, CLONTECH #64018-1;
HEART: Heart, CLONTECH #64025-1;
HLUNG: Lung, CLONTECH #64023-1;
KIDNE: Kidney, CLONTECH #64030-1;
LIVER: Liver, CLONTECH #64022-1;
MAMGL: Mammary Gland, CLONTECH #64037-1;
PANCR: Pancreas, CLONTECH #64031-1;
PROST: Prostate, CLONTECH #64038-1;
SALGL: Salivary Gland, CLONTECH #64026-1;
SKMUS: Skeletal Muscle, CLONTECH #64033-1;
SMINT: Small Intestine, CLONTECH #64039-1;
SPLEN: Spleen, CLONTECH #64034-1;
STOMA: Stomach, CLONTECH #64090-1;
TBAES: Breast (Tumor), CLONTECH #64015-1;
TCERX: Cervix (Tumor), CLONTECH #64010-1;
TCOLN: Colon (Tumor), CLONTECH #64014-1;
TESTI: Testis, CLONTECH #64027-1;
THYMU: Thymus, CLONTECH #64028-1;
TLUNG: Lung (Tumor), CLONTECH #64013-1;
TOVAR: Ovary (Tumor), CLONTECH #64011-1;
TRACH: Trachea, CLONTECH #64091-1;
TUTER: Uterus (Tumor), CLONTECH #64008-1;
UTERU: Uterus, CLONTECH #64029-1;
ADIPS: Adipose, Invitrogen #D6005-01;
BLADE: Bladder, Invitrogen #D6020-01;
BRALZ: Cerebral cortex from an Alzheimer patient (Brain, cortex, Alzheimer), Invitrogen #D6830-01;
CERVX: Cervix, Invitrogen #D6047-01;
COLON: Colon, Invitrogen #D6050-0;
NESOP: Esophagus, Invitrogen #D6060-01;
PERIC: Pericardium, Invitrogen #D6105-01;
RECTM: Rectum, Invitrogen #D6110-01;
TESOP: Esophageal (Tumor), Invitrogen #D6860-01;
TKIDN: Kidney (Tumor), Invitrogen #D6870-01;
TLIVE: Liver (Tumor), Invitrogen #D6880-01;
TSTOM: Stomach (Tumor), Invitrogen #D6920-01;
BEAST: Adult breast, STARATAGENE #735044;
FEHRT: Fetal heart, STARATAGENE #738012;
FEKID: Fetal kidney, STARATAGENE #738014;
FELNG: Fetal lung, STARATAGENE #738020;
NOVAR: Adult ovary, STARATAGENE #735260;
BRASW: subtracted library (BRALZ-BRAWH) A cDNA library was constructed from mRNA prepared from tissues of cerebral cortex obtained from an Alzheimer patient [BRALZ: Cerebral cortex from an Alzheimer patient (Brain, cortex, Alzheimer), Invitrogen #D6830-01]; the cDNA libraries whose nucleotide sequences were shared by those of mRNAs from whole brain tissue [BRAWH: Whole brain, CLONTECH #64020-1] were subtracted using a Subtract Kit (Invitrogen #K4320-01).

Further, mRNAs extracted and purified as poly A(+) RNAs from the human tissues shown below were purchased. A cDNA library was prepared from an RNA mixture in which the poly A(+) RNA from each tissue was combined with poly A(-) RNA. The poly A(-) RNA was prepared by removing poly A(+) RNA from the total RNA of whole brain tissue (CLONTECH #64020-1) by using oligo dT cellulose. The library names and origins are indicated below in the order of "Library name: Origin".
<Purchase of mRNAs of human tissues as poly A(+) RNAs>
BRAMY: Brain (amygdala), CLONTECH #6574-1;
BRCAN: Brain (caudate nucleus), CLONTECH #6575-1;
BRCOC: Brain (corpus callosum), CLONTECH #6577-1;
BRHIP: Brain (hippocampus), CLONTECH #6578-1;
BRSSN: Brain (substantia nigra), CLONTECH #6580-1;
BRSTN: Brain (subthalamic nucleus), CLONTECH #6581-1;
BRTHA: Brain (thalamus), CLONTECH #6582-1.

### (2) Preparation of cDNA libraries

A cDNA library was prepared from each RNA using the improved method (WO 01/04286) of oligo capping [M. Maruyama and S. Sugano, Gene, 138: 171-174 (1994)]. A series of procedures, BAP (Bacterial Alkaline Phosphatase) treatment, TAP (Tobacco Acid Pyrophosphatase) treatment, RNA ligation, first strand cDNA synthesis and RNA removal, were carried out using the oligo-cap linker (agcaucgagu cggccuuguu ggccuacugg/ SEQ ID NO: 3991) and oligo dT primer (gcggctgaag acggcctatg tggccttttt tttttttttt tt/ SEQ ID NO: 3992), as described in WO 01/04286. The single-stranded cDNA was then converted to a double-stranded cDNA by PCR (polymerase chain reaction) using 5' (agcatcgagt cggccttgtt g/ SEQ ID NO: 3993) and 3' (gcggctgaag acggcctatg t/ SEQ ID NO: 3994) PCR primers, and then digested with SfiI. Then, a fraction of cDNA fragments, typically 2-kb or longer (3-kb or longer in some cases), was unidirectionally cloned into a DraIII-digested pME18SFL3 vector (Figure 1) (GenBank AB009864, Expression vector); and the cDNA library was thus prepared.

Shown below are the names of cDNA libraries used in the analysis of full-length cDNA sequences, and their origins. The Library Name is provided with the Type, Origin, and such of the library source, demarcated by a slash mark (/) within parentheses.
3NB69 (culture cells / NB69 cells (RCB #RCB0480))
ADIPS (Tissues / Adipose (Invitrogen #D6005-01))
ADRGL (Tissues / Adrenal gland (CLONTECH #64016-1))
AHMSC (culture cells / HMSC cells (Human mesenchymal cell))
ASTRO (primary culture cells / Normal Human Astrocyte NHA5732 (Takara Shuzo #CC2565))
BEAST (Tissues / Adult Breast (STARATAGENE #735044))
BLADE (Tissues / Bladder (Invitrogen #D6020-01))
BRACE (Tissues / Brain (cerebellum) (CLONTECH #64035-1))
BRALZ (Tissues / Cerebral cortex from an Alzheimer patient (Brain, cortex, Alzheimer) (Invitrogen #D6830-01))
BRAMY (Tissues / Brain (amygdala) (CLONTECH #6574-1 ))
BRAWH (Tissues / Whole brain (CLONTECH #64020-1))
BRCAN (Tissues / Brain (caudate nucleus) (CLONTECH #6575-1))
BRCOC (Tissues / Brain (corpus callosum) (CLONTECH #6577-1))
BRHIP (Tissues / Brain (hippocampus) (CLONTECH #6578-1))
BRSSN (Tissues / Brain (substantia nigra) (CLONTECH #6580-1))
BRSTN (Tissues / Brain (subthalamic nucleus) (CLONTECH #6581-1))
BRTHA (Tissues / Brain (thalamus) (CLONTECH #6582-1))
CERVX (Tissues / Cervix (Invitrogen #D6047-01))
CHONS (culture cells / Chondrocyte)
COLON (Tissues / Colon (Invitrogen #D6050-0))
CTONG (Tissues / Tongue Cancer)
D9OST (primary culture cells / CD34+ cells (treated with ODF for nine days to induce the differentiation))
DFNES (primary culture cells / Normal human dermal fibroblasts (neonatal skin); NHDF-Neo NHDF2564 (Takara Shuzo #CC2509))
ERLTF (culture cells / TF-1 cells (Erythroleukemia))
FCBBF (Tissues / Fetal brain)
FEBRA (Tissues / Fetal brain (CLONTECH #64019-1))
FEHRT (Tissues / Fetal heart (STARATAGENE #738012))
FEKID (Tissues / Fetal kidney)
FELNG (Tissues / Fetal lung (STARATAGENE #738020))
HCASM (primary culture cells / Normal human coronary artery smooth muscle cell HCASMC (Toyobo #T305K-05))
HCHON (primary culture cells / Normal human chondrocyte HC (Toyobo #T402K-05))
HEART (Tissues / Heart (CLONTECH #64025-1))
HHDPC (primary culture cells / Normal human dermal papilla cell HDPC (Toyobo #THPCK-001))
HLUNG (Tissues / Lung (CLONTECH #64023-1))
HSYRA (primary culture cells / Human synoviocyte HS-RA (from rheumatoid arthritis)(Toyobo #T404K-05))
JCMLC (culture cells / myelogenous leukemias)
KIDNE (Tissues / Kidney (CLONTECH #64030-1))
LIVER (Tissues / Liver (CLONTECH #64022-1))
LYMPB (primary culture cells / Lymphoblasts (EB virus transferred B cells))
MESAN (primary culture cells / Normal human mesangial cell NHMC56046-2 (Takara Shuzo #CC2559))
MESTC (culture cells / Mesenchyme stem cells)
N1ESE (culture cells / Mesenchyme stem cells)
NETRP (primary culture cells / Neutrophil)
NOVAR (Tissues / Adult ovary (STARATAGENE #735260))
NT2NE (culture cells / NT2 cells were treated with RA and the growth inhibitor for the neuronal differentiation, and the resultant neurons were concentrated and harvested (NT2 Neuron))
NT2RI (culture cells / NT2 cells treated with RA for five weeks to induce the differentiation, followed by the treatment with the growth inhibitor for 2 weeks)
NT2RP (culture cells / NT2 cells treated with RA for five weeks to induce the differentiation)
NTONG (Tissues / Normal tongue)
OCBBF (Tissues /Fetal brain)
PEBLM (primary culture cells / Normal human peripheral blood mononuclear cell HPBMC5939 (Takara Shuzo #CC2702))
PERIC (Tissues / Pericardium (Invitrogen #D6105-01))
PLACE (Tissues / Placenta)
PROST (Tissues / Prostate (CLONTECH #64038-1))
PUAEN (primary culture cells / Normal human pulmonary artery endothelial cells (Toyobo #T302K-05))
RECTM (Tissues / Rectum (Invitrogen #D6110-01))
SKMUS (Tissues / Skeletal Muscle (CLONTECH #64033-1))
SKNMC (culture cells / SK-N-MC cells (ATCC #HTB-10))
SKNSH (culture cells / SK-N-SH cells (RCB #RCB0426))
SMINT (Tissues / Small Intestine (CLONTECH #64039-1))
SPLEN (Tissues / Spleen (CLONTECH #64034-1))
STOMA (Tissues / Stomach (CLONTECH #64090-1))
SYNOV (Tissues / Synovial membrane tissue (from rheumatoid arthritis))
T1ESE (culture cells / Mesenchymal stem cell (treated with trichostatin and 5-azacytidine to induce the differentiation))
TBAES (Tissues / Breast (Tumor) (CLONTECH #64015-1))
TESOP (Tissues / Esophageal (Tumor) (Invitrogen #D6860-01))
TESTI (Tissues / Testis (CLONTECH #64027-1))
THYMU (Tissues / Thymus (CLONTECH #64028-1))
TKIDN (Tissues / Kidney (Tumor) (Invitrogen #D6870-01))
TLIVE (Tissues / Liver (Tumor) (Invitrogen #D6880-01))
TLUNG (Tissues / Lung (Tumor))
TRACH (Tissues / Trachea (CLONTECH #64091-1))
TSTOM (Tissues / Stomach (Tumor) (Invitrogen #D6920-01))
TUTER (Tissues / Uterus (Tumor) (CLONTECH #64008-1))
UTERU (Tissues / Uterus (CLONTECH #64029-1))

cDNA libraries with a high fullness ratio (the fullness ratio of 5'-end was 90% on average, calculated for each cDNA library using the protein coding region found in known mRNA species as an index) prepared by the improved oligo-capping method were constructed using a eukaryotic expression vector pME18SFL3. The vector contained SR α promoter and SV40 small t intron upstream of the cloning site, and SV40 polyA added signal sequence site downstream. As the cloning site of pME18SFL3 has asymmetrical DraIII sites, and the ends of cDNA fragments contain SfiI sites complementary to the DraIII sites, the cloned cDNA fragments can be inserted downstream of the SR α promoter unidirectionally. Therefore, clones containing full-length cDNA can be expressed transiently by introducing the obtained plasmid directly into COS cells, etc. Thus, clones can be analyzed very easily using the clone gene product proteins, or those proteins' biological activities.

### (3) Assessment of 5'-end completeness of clones derived from a cDNA library prepared by oligo-capping

With respect to the plasmid DNAs of clones derived from the libraries, the nucleotide sequences of cDNA 5'-ends (3'-ends as well in some cases) were determined in a DNA sequencer (ABI PRISM 3700, PE Biosystems), after sequencing reaction were conducted using a DNA sequencing reagent (BigDye Terminator Cycle Sequencing FS Ready Reaction Kit, PE Biosystems) according to the manual. A database was constructed based on the obtained data.

The 5'-end completeness of about 1,110,000 clones derived from the human cDNA libraries prepared by the improved oligo-capping method was determined using the following method. The clones whose 5'-end sequences were consistent with those of known human mRNA in the public database were judged to be "full-length" if they had a longer 5'-end sequence than that of the known human mRNA. Even if the 5'-end sequence was shorter, clones containing the translation initiation codon were judged to comprise the "full-length" sequence. Clones which did not comprise the translation initiation codon were judged to be "not-full-length". The fullness ratio ((the number of full-length clones)/(the number of full-length and not-full-length clones)) at the 5'-end of the cDNA clones was determined by comparison with known human mRNA. The fullness ratio of the 5'-ends was found to be 90%. The result indicates that the fullness ratio at the 5'-end sequence was extremely high in human cDNA clones obtained by the oligo-capping method.

### EXAMPLE 2

### Sequencing analysis of cDNA ends and selection of full-length clones

With respect to the plasmid DNAs of clones obtained from each cDNA library, the 5'-end nucleotide sequences of the cDNAs were determined in a DNA sequencer (ABI PRISM 3700, PE Biosystems), after sequencing reaction was conducted by using a DNA sequencing reagent (Dye Terminator Cycle Sequencing FS Ready Reaction Kit, dRhodamine Terminator Cycle Sequencing FS Ready Reaction Kit or BigDye Terminator Cycle Sequencing FS Ready Reaction Kit, PE Biosystems) according to the manual. A database was constructed using the data obtained.

For the analyzed 5'-end sequences of cDNA clones, the data with the annotation of "complete cds" in the GenBank and UniGene were searched by BLAST homology search. When identical to certain human mRNA sequences, such cDNA clones were excluded. Then, clustering was carried out. When the identity was 90% or higher, and the length of consensus sequence was 50 base pairs or longer, the cDNA clones were assumed to belong to an identical cluster, and thus clustered. cDNA clones longer in the 5' direction were selected from the members belonging to a cluster; if required, the 3'-end sequences of the selected clones were determined by the same analysis method as used to determine the 5'-end sequences. The data of the end sequences obtained were analyzed, and then the clones forming a sequence contig at 5'- and 3'-ends were excluded. Further, as mentioned above, the data was analyzed again by BLAST homology search; when identical to certain human mRNA sequences (including sequences patented and applied for), the cDNA clones were excluded. Thus, the cDNAs clones to be analyzed for their nucleotide sequence were obtained.

### EXAMPLE 3

### Analysis of the full-length nucleotide sequences

The full-length nucleotide sequences of the selected clones were determined. The nucleotide sequence determination was mainly performed by primer walking method comprising the dideoxy terminator method using custom-made synthetic DNA primers. Namely, the nucleotide sequences of the DNAs were determined in a sequencer from PE Biosystems, after sequencing reaction was carried out with a DNA sequencing reagent from the same supplier using the custom-made synthetic DNA primers according to the manual. A part of the clones were analyzed with a DNA sequencer from Licor.

Further, the nucleotide sequences of a part of the clones were determined by the shotgun method where the plasmids containing the cDNAs were digested at random were used, instead of the use of custom-made primers, by the same method in the DNA sequencer. The full-length nucleotide sequences were finally determined by completely assembling the partial nucleotide sequences obtained by the above method.

Then, the regions translatable to proteins were deduced from the determined full-length nucleotide sequences, and thereby the amino acid sequences were determined. SEQ ID NOs corresponding to the respective sequences are shown in Table 1.

### EXAMPLE 4

### Functional prediction by homology search

SwissProt, RefSeq, and nr were searched using BLAST for the determined nucleotide sequences and the ORF amino acid sequences deduced to encode the polypeptides. Of the hit data which met the criteria described below, representative hit data were selected. This representative data is hit data with a higher homology and that enables relatively easy functional prediction for nucleotide sequences and deduced amino acid sequences. The results of the homology search are shown at the end of this specification.
˙ Hit data whose P value or E value is 10⁻⁴ or less
˙ For analysis using an amino acid database, hit data whose P value or E value is 10⁻⁴ or less, where the length of consensus sequence x homology = 30 or higher,

Thus, only representative data are indicated and molecules exhibiting homology to each clone are not limited thereto. For some clones, hit data that do not meet the above-described criteria in BLAST search are not shown.

### EXAMPLE 5

### Search for signal sequence, transmembrane domain and other functional domains in the deduced amino acid sequences

With respect to the amino acid sequences deduced from the full-length nucleotide sequences, the prediction was made for the presence of signal sequence at the amino terminus, the presence of transmembrane domain, and the presence of functional protein domains (motifs). The signal sequence at the amino terminus was searched for by PSORT [K. Nakai & M. Kanehisa, Genomics, 14: 897-911 (1992)]; the transmembrane domain, by SOSUI [T. Hirokawa et al., Bioinformatics, 14: 378-379 (1998)] (Mitsui Knowledge Industry); the function domain, by Pfam (Version 5.5) (http://www.sanger.ac.uk/Software/Pfam/index.shtml). The amino acid sequence in which the signal sequence at the amino terminus or transmembrane domain had been predicted to be present by PSORT or SOSUI were assumed to be a secretory or membrane protein. Further, when the amino acid sequence hit a certain functional domain by the Pfam functional domain search, the protein function can be predicted based on the hit data, for example, by referring to the function categories on the PROSITE (http://www.expasy.ch/cgi-bin/prosite-list.pl). In addition, the functional domain search can also be carried out on the PROSITE.

The search results obtained with the respective programs are shown below.

The 130 clones whose deduced amino acid sequences were detected to have the signal sequences by PSORT are as follows.
ADIPS2000069, ASTRO2015162, BEAST2000981, BLADE2001031, BRACE2017397, BRACE2023633, BRACE3009392, BRACE3013986, BRACE3014523, BRACE3026345, BRACE3032537, BRACE3037803, BRACE3051144, BRAMY2015516, BRAMY3002886, BRAMY3009556, BRAMY3016829, BRAWH2011958, BRAWH2016209, BRAWH3005886, BRAWH3012005, BRAWH3014609, BRAWH3016123, BRAWH3018063, BRAWH3020200, BRAWH3023421, BRAWH3028223, BRAWH3032298, BRAWH3034134, BRAWH3040695, BRAWH3042438, BRAWH3046196, BRAWH3049068, BRCAN2003269, BRCAN2006955, BRCAN2018269, BRHIP2006921, BRHIP2020930, BRHIP3000859, BRHIP3012997, BRHIP3016032, BRHIP3020046, BRHIP3020733, BRHIP3028742, BRHIP3030230, BRHIP3035754, BRSSN2018218, BRSTN2010089, BRSTN2011688, BRTHA2000969, BRTHA2014647, BRTHA2020400, BRTHA2020721, BRTHA2026290, BRTHA2030036, BRTHA2033320, BRTHA2033683, BRTHA3003225, BRTHA3014835, BRTHA3018623, BRTHA3026161, BRTHA3027820, COLON2005623, CTONG3002588, FEBRA2023498, FEKID2002493, HCHON2009766, JCMLC2002095, NETRP2004090, NETRP2005849, NT2RI3009524, NT2RP8008057, OCBBF3007704, OCBBF3021502, OCBBF3022123, OCBBF3028001, PEBLM2005615, SKMUS2008585, SPLEN2025012, SPLEN2035615, SPLEN2042051, STOMA2004663, SYNOV4009139, TESTI2037657, TESTI2049041, TESTI4002072, TESTI4022158, TESTI4024494, TESTI4035872, TESTI4043371, TESTI4045168, THYMU3003350, THYMU3014173, THYMU3015457, THYMU3023107, THYMU3025118, THYMU3025313, THYMU3025642, THYMU3026532, THYMU3026869, THYMU3028461, THYMU3029795, THYMU3034099, THYMU3036310, THYMU3036953, THYMU3037772, THYMU3038158, THYMU3038167, THYMU3040068, THYMU3040746, THYMU3044188, THYMU3045510, THYMU3047542, TRACH3003872, TRACH3014580, TRACH3016368, TRACH3019058, TRACH3020930, TRACH3021023, TRACH3021544, TRACH3022758, TRACH3023203, TRACH3023516, TRACH3025346, TRACH3026542, TRACH3026650, TRACH3029670, TRACH3031316, TRACH3034680, TRACH3036278

Deduced amino acid sequences of following nine clones were also detected to have the signal sequences by PSORT.
ADRGL2011190, PROST2010326, TBAES2004105, TBAES2007379, TBAES2008133, THYMU3044175, TLIVE2007192, UTERU2025415, UTERU2036507

The 455 clones whose deduced amino acid sequences were detected to have the transmembrane domains by SOSUI are as follows. Numerals indicate the numbers of transmembrane domains detected in the deduced amino acid sequences. Of the search result, the Clone Name and the Number of transmembrane domains are demarcated by a double slash mark (//).
3NB692004045//5, ADRGL2010315//3, ASTRO2015162//5, BEAST2000981//4, BLADE2002744//2, BLADE2007744//1, BRACE2003628//4, BRACE2012528//1, BRACE2013126//3, BRACE2017580//2, BRACE2017992//2, BRACE2023633//2, BRACE2030039//6, BRACE2035191//1, BRACE3001973//2, BRACE3002264//11, BRACE3002344//1, BRACE3004981//1, BRACE3007869//3, BRACE3009392//6,
BRACE3013874//2, BRACE3015898//1, BRACE3018083//1, BRACE3021517//1, BRACE3021805//1, BRACE3022051//1, BRACE3024379//2, BRACE3024444//2, BRACE3024497//2, BRACE3024879//6, BRACE3026345//1, BRACE3026456//1, BRACE3026802//2, BRACE3028360//2, BRACE3029021//1, BRACE3030538//5, BRACE3031372//3, BRACE3031579//3, BRACE3031728//1, BRACE3032385//8,
BRACE3032980//1, BRACE3033525//1, BRACE3034964//1, BRACE3034993//2, BRACE3037637//3, BRACE3037803//2, BRACE3038570//3, BRACE3039358//1, BRACE3039378//6, BRACE3040644//1, BRACE3040863//2, BRACE3042326//11, BRACE3042409//4, BRACE3042432//2, BRACE3044090//2, BRACE3046049//1, BRACE3046152//3, BRACE3046466//2, BRACE3048565//2, BRACE3050504//7,
BRACE3051144//3, BRACE3051621//3, BRACE3052486//1, BRALZ2011337//1, BRALZ2013690//2, BRAMY2015516//4, BRAMY2021098//1, BRAMY2025495//2, BRAMY2037609//2, BRAMY2040915//1, BRAMY2041507//2, BRAMY2044686//4, BRAMY2046537//5, BRAMY3002886//5, BRAMY3004126//6, BRAMY3007449//2, BRAMY3009904//2, BRAMY3010654//2, BRAMY3010902//2, BRAMY3015547//2,
BRAMY3015549//2, BRAMY3016829//3, BRAWH2000256//5, BRAWH2010364//2, BRAWH2011812//1, BRAWH2012866//1, BRAWH2016209//5, BRAWH2016305//1, BRAWH3001053//1, BRAWH3001783//1, BRAWH3001833//7, BRAWH3005892//1, BRAWH3008867//2, BRAWH3010461//4, BRAWH3010657//1, BRAWH3011907//1, BRAWH3012662//1, BRAWH3012779//3, BRAWH3013049//1, BRAWH3014609//2,
BRAWH3015175//1, BRAWH3018063//1, BRAWH3018969//5, BRAWH3019529//1, BRAWH3019820//3, BRAWH3020200//3, BRAWH3020884//1, BRAWH3021012//2, BRAWH3021641//2, BRAWH3022347//1, BRAWH3023156//1, BRAWH3023274//2, BRAWH3023415//1, BRAWH3024186//1, BRAWH3024242//3, BRAWH3027574//1, BRAWH3027880//5, BRAWH3028223//2, BRAWH3028754//2, BRAWH3029806//10,
BRAWH3030810//1, BRAWH3034114//1, BRAWH3034134//1, BRAWH3035914//1, BRAWH3036270//1, BRAWH3038055//1, BRAWH3038324//3, BRAWH3040695//1, BRAWH3040711//2, BRAWH3042132//1, BRAWH3042438//1, BRAWH3042772//2, BRAWH3042996//2, BRAWH3043498//2, BRAWH3044676//1, BRAWH3046196//3, BRAWH3047063//1, BRAWH3048374//2, BRAWH3048724//2, BRAWH3049068//2,
BRAWH3049544//1, BRCAN2002662//1, BRCAN2003269//3, BRCAN2018269//2, BRCAN2019653//2, BRCAN2020412//2, BRCAN2020972//1, BRCAN2022126//5, BRCOC2006164//8, BRCOC2006639//1, BRCOC2009638//3, BRHIP2021929//7, BRHIP3001878//3, BRHIP3002000//10, BRHIP3002124//3, BRHIP3003306//2, BRHIP3003395//1, BRHIP3004774//1, BRHIP3005801//1, BRHIP3006950//2,
BRHIP3007195//8, BRHIP3007960//1, BRHIP3008320//6, BRHIP3010289//2, BRHIP3011831//1, BRHIP3012185//2, BRHIP3013078//1, BRHIP3016032//10, BRHIP3017146//3, BRHIP3017558//11, BRHIP3019956//1, BRHIP3021019//1, BRHIP3025795//11, BRHIP3025844//1, BRHIP3027160//1, BRHIP3027191//1, BRHIP3028742//2, BRHIP3029530//2, BRHIP3031733//1, BRHIP3033557//5,
BRHIP3035222//2, BRHIP3036715//3, BRHIP3036936//1, BRHIP3037810//3, BRHIP3039430//4, BRHIP3041587//1, BRSSN2004710//1, BRSSN2018218//2, BRSTN2010089//11, BRSTN2011688//2, BRSTN2011899//1, BRTHA2000969//2, BRTHA2003759//3, BRTHA2012189//1, BRTHA2014647//2, BRTHA2018304//2, BRTHA2019726//1, BRTHA2019743//2, BRTHA2020566//1, BRTHA2020781//1,
BRTHA2021212//1, BRTHA2021440//1, BRTHA2021450//1, BRTHA2022914//2, BRTHA2022968//1, BRTHA2023437//1, BRTHA2030036//3, BRTHA2031917//1, BRTHA2033469//2, BRTHA2033683//7, BRTHA2036055//1, BRTHA2036295//1, BRTHA3006593//1, BRTHA3010540//2, BRTHA3010717//4, BRTHA3011194//11, BRTHA3011998//3, BRTHA3012265//4, BRTHA3013882//3, BRTHA3014835//1,
BRTHA3016616//2, BRTHA3018623//2, BRTHA3028505//1, CHONS2001797//1, CHONS2002419//2, COLON2005623//1, COLON2005735//10, CTONG2008989//4, CTONG2020582//1, CTONG2027150//1, CTONG3001605//5, CTONG3002588//1, CTONG3008223//2, FCBBF3012443//1, FEBRA2023498//1, FEBRA2026977//1, FEHRT2002708//4, FEKID2002231//1, FELNG2000720//1, FELNG2001706//1,
HCHON2009766//12, HSYRA2004550//6, JCMLC2000273//1, KIDNE2004531//6, KIDNE2015987//2, KIDNE2017153//1, LYMPB1000158//1, LYMPB2002236//12, LYMPB2002478//2, MESAN2014624//5, N1ESE2000698//1, NETRP2004090//3, NETRP2004434//1, NETRP2005282//2, NETRP2005849//2, NT2RI3005861//1, NT2RI3009524//1, NT2RP7019682//2, NT2RP8001605//2, NT2RP8003787//6,
NT2RP8008057//1, OCBBF2004478//4, OCBBF2018229//2, OCBBF2018618//6, OCBBF2036019//2, OCBBF3003745//2, OCBBF3007704//1, OCBBF3022123//1, OCBBF3022576//2, OCBBF3023175//3, OCBBF3023993//3, OCBBF3025475//13, OCBBF3025887//1, OCBBF3028001//2, PEBLM2003935//1, PEBLM2005615//9, PLACE5000522//2, PLACE6000012//3, PLACE6010936//6, PLACE6019674//1,
PLACE7000266//1, PLACE7000707//2, PLACE7003639//3, PLACE7008136//2, PLACE7011269//2, PLACE7014247//3, PLACE7016321//3, PLACE7016454//2, PUAEN2000684//4, SKMUS2008585//2, SMINT2003641//1, SPLEN2007689//2, SPLEN2011252//3, SPLEN2031004//1, SPLEN2034551//1, SPLEN2035615//1, STOMA2004663//1, T1ESE2002665//12, TBAES2005361//1, TBAES2007428//2,
TESOP2008556//1, TESTI2007490//2, TESTI2018335//10, TESTI2022323//6, TESTI2024267//9, TESTI2028613//3, TESTI2036822//5, TESTI2037085//1, TESTI2037877//1, TESTI2046188//1, TESTI4001037//2, TESTI4002072//6, TESTI4002889//2, TESTI4003602//6, TESTI4004539//8, TESTI4004653//9, TESTI4005399//11, TESTI4007671//1, TESTI4010544//13, TESTI4010721//6,
TESTI4013774//2, TESTI4014415//14, TESTI4014932//2, TESTI4014977//1, TESTI4017647//1, TESTI4017854//2, TESTI4019149//1, TESTI4021377//4, TESTI4021569//4, TESTI4022158//2, TESTI4023096//9, TESTI4023654//1, TESTI4026680//1, TESTI4027170//1, TESTI4028042//2, TESTI4031818//1, TESTI4032128//1, TESTI4033177//2, TESTI4034973//3, TESTI4035989//1,
TESTI4036012//3, TESTI4037949//2, TESTI4038047//2, TESTI4039575//1, TESTI4040559//4, TESTI4041049//2, TESTI4043067//1, TESTI4043371//3, TESTI4046073//1, TESTI4046450//2, TESTI4047119//11, TESTI4048296//2, TESTI4048545//1, TESTI4051015//3, TESTI4051858//1, TESTI4052219//2, TESTI4052430//1, TESTI4052598//1, THYMU3002825//1, THYMU3003007//1,
THYMU3008935//1, THYMU3009755//2, THYMU3011360//2, THYMU3013197//1, THYMU3014173//1, THYMU3015647//9, THYMU3016518//2, THYMU3018151//2, THYMU3019605//1, THYMU3021404//7, THYMU3021586//3, THYMU3022211//2, THYMU3022528//3, THYMU3022668//1, THYMU3023107//5, THYMU3023400//9, THYMU3025313//1, THYMU3025642//2, THYMU3026306//1, THYMU3026532//2,
THYMU3026869//1, THYMU3027540//2, THYMU3031878//4, THYMU3032032//2, THYMU3033649//2, THYMU3034616//2, THYMU3036310//1, THYMU3036934//2, THYMU3038158//1, THYMU3040126//3, THYMU3040146//2, THYMU3040172//2, THYMU3040746//2, THYMU3040816//2, THYMU3041918//1, THYMU3042321//1, THYMU3043688//4, THYMU3043779//2, THYMU3044188//2, THYMU3047115//7,
THYMU3047156//3, THYMU3047542//2, THYMU3047760//2, TLIVE2008797//1, TRACH3003872//2, TRACH3004747//12, TRACH3005274//1, TRACH3005699//1, TRACH3007274//2, TRACH3007625//7, TRACH3009008//2, TRACH3009061//6, TRACH3010382//1, TRACH3011184//1, TRACH3012891//2, TRACH3013900//1, TRACH3014063//3, TRACH3015346//1, TRACH3016368//1, TRACH3016885//1,
TRACH3016992//1, TRACH3017409//1, TRACH3018191//2, TRACH3018240//1, TRACH3018943//1, TRACH3019370//6, TRACH3019621//10, TRACH3020930//2, TRACH3022109//3, TRACH3023063//3, TRACH3023203//3, TRACH3023945//1, TRACH3024081//2, TRACH3024671//2, TRACH3025346//5, TRACH3026542//2, TRACH3026650//1, TRACH3027681//2, TRACH3029670//2, TRACH3031316//1,
TRACH3031678//8, TRACH3032480//1, TRACH3038399//1, TSTOM2002682//1, UTERU3005422//3, UTERU3010029//3, UTERU3011092//8, UTERU3011837//1, UTERU3012414//1, UTERU3015647//1, UTERU3016273//5, UTERU3017626//6, UTERU3022168//1, UTERU3022922//2, UTERU3023413//1

Deduced amino acid sequences of following 14 clones were also detected to have the transmembrane domains by SOSUI.
BRAWH3013197//6, BRAWH3028645//7, BRAWH3046240//7, BRTHA3024233//3, PROST2010326//8, TBAES2004105//1, TBAES2007379//1, TBAES2007481//1, TESTI2043585//8, THYMU3024602//8, THYMU3044175//2, THYMU3046350//9, TLIVE2007192//2, TRACH3021066//2

The 796 clones whose deduced amino acid sequences were detected to have functional domains with Pfam are as follows. The search result is indicated as "Clone Name//Functional Domain Name". When the clone has Multiple Hit Functional Domains, they are listed and demarcated by a double slash mark (//). When the clone has multiple hits of an identical functional domain, each is listed without abridgment.
3NB692004045//Sodium Bile acid symporter family// FecCD transport family
ADIPS2000069//Immunoglobulin domain// Immunoglobulin domain//
Immunoglobulin domain// Immunoglobulin domain// Immunoglobulin domain
ASTRO2016114//Zinc finger, C2H2 type// Zinc finger, C2H2 type//
PHD-finger// Zinc finger, C2H2 type// Zinc finger, C2H2 type//
Transcription factor S-II (TFIIS)// Zinc finger, C2H2 type//
Zinc finger, C2H2 type// Zinc finger, C2H2 type// Zinc finger,
C2H2 type// Zinc finger, C2H2 type// Zinc finger, C2H2 type
BEAST2000981//PA domain// Zinc finger, C3HC4 type (RING finger)// PHD-finger
BLADE2001031//Thrombospondin type 1 domain
BLADE2002310//RhoGAP domain
BLADE2008809//PH domain
BRACE1000475//Enoyl-CoA hydratase/isomerase family
BRACE2003628//NADH-Ubiquinone oxidoreductase (complex I), chain 5 C-terminus
BRACE2010336//TPR Domain// TPR Domain// TPR Domain// TPR
Domain// TPR Domain// TPR Domain// TPR Domain
BRACE2012528//alpha/beta hydrolase fold
BRACE2012625//RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
BRACE2013009//EF hand
BRACE2013132//Adenosine/AMP deaminase
BRACE2016896//tRNA synthetases class II (F)// tRNA synthetases class II (F)// tRNA synthetases class II (D, K and N)
BRACE2017397//von Willebrand factor type A domain
BRACE2017872//PWWP domain
BRACE2019348//Zinc finger, C2H2 type// Zinc finger, C2H2 type
BRACE2023744//Translationally controlled tumor protein
BRACE2034434//Protein of unknown function
BRACE2035120//Inositol polyphosphate phosphatase family, catalytic domain
BRACE2042541//Ank repeat// Ank repeat// Glutathione S-transferases.
BRACE2046976//Collagen triple helix repeat (20 copies)
BRACE2047975//Eukaryotic protein kinase domain
BRACE3001973//EGF-like domain// Laminin G domain// EGF-like domain// EGF-like domain// EGF-like domain
BRACE3002264//GNS1/SUR4 family// Ion transport protein// Ion transport protein
BRACE3002344//WD domain, G-beta repeat// WD domain, G-beta repeat// WD domain, G-beta repeat// WD domain, G-beta repeat//
PQQ enzyme repeat// WD domain, G-beta repeat// WD domain, G-beta repeat// WD domain, G-beta repeat// WD domain, G-beta repeat
BRACE3002756//SAM domain (Sterile alpha motif)
BRACE3003866//Src homology domain 2
BRACE3004767//PH domain
BRACE3005903//K-box region// TSC-22/dip/bun family
BRACE3009392//Aminotransferases class-III pyridoxal-phosphate// Sodium:dicarboxylate symporter family
BRACE3013418//Ank repeat// Ank repeat// ZU5 domain// Death domain// TGF-beta propeptide// Fatty acid desaturase// Isocitrate lyase
BRACE3013874//Leucine rich repeat C-terminal domain// Immunoglobulin domain// Fibronectin type III domain
BRACE3014523//Wiskott Aldrich syndrome homology region 2
BRACE3015898//PI3-kinase family, ras-binding domain
BRACE3017253//Adenylate kinase// Transferrin
BRACE3019570//Troponin
BRACE3019941//TPR Domain// TPR Domain// DNA-dependent RNA polymerase// TPR Domain// TPR Domain// TPR Domain// 7-fold repeat in Clathrin and VPS// TPR Domain// TPR Domain// TPR Domain// TPR Domain
BRACE3022303//BRCA1 C Terminus (BRCT) domain// BRCA1 C Terminus (BRCT) domain
BRACE3022340//Troponin
BRACE3024444//Copper/zinc superoxide dismutase (SODC)
BRACE3024879//7 transmembrane receptor (Secretin family)
BRACE3025719//Zinc finger, C3HC4 type (RING finger)// Filamin/ABP280 repeat.// NHL repeat// NHL repeat// NHL repeat// NHL repeat// Squash family of serine protease inhibitors
BRACE3026345//Insulin/IGF/Relaxin family
BRACE3026844//KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// TRAF-type zinc finger// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type
BRACE3026947//FYVE zinc finger
BRACE3029021//Zinc finger, C2H2 type
BRACE3031315//Serine hydroxymethyltransferase// UBA domain
BRACE3031372//D-isomer specific 2-hydroxyacid dehydrogenases
BRACE3031743//RNA recognition motif. (a.k.a. RRM, RBD, or RNP domain)
BRACE3032385//Ion transport protein
BRACE3032537//Luteovirus (ORF3) RNA-directed RNA-polymerase
BRACE3033525//Prolyl oligopeptidase family
BRACE3034183//Zinc finger, C2H2 type// PHD-finger// Zinc finger, C2H2 type// Plant PEC family metallothionein// Transcription factor S-II (TFIIS)// Zinc finger, C2H2 type// Zinc finger, C2H2 type// Zinc finger, C2H2 type// FYVE zinc finger// Zinc finger, C2H2 type// RNA polymerases M/15 Kd subunits// Zinc finger, C2H2 type// Zinc finger, C2H2 type// TRAF-type zinc finger// Zinc finger, C2H2 type// Zinc finger, C2H2 type// Zinc finger, C2H2 type// Zinc finger, C2H2 type// Zinc finger, C2H2 type// Zinc finger, C2H2 type
BRACE3034964//alpha/beta hydrolase fold
BRACE3036283//DnaJ domain
BRACE3039288//Viral RNA dependent RNA polymerase
BRACE3039358//Leucine Rich Repeat// Leucine Rich Repeat// Leucine rich repeat C-terminal domain// Immunoglobulin domain// Immunoglobulin domain// Immunoglobulin domain
BRACE3039378//Pentaxin family// Receptor family ligand binding region// 7 transmembrane receptor (metabotropic glutamate family)
BRACE3039454//Aminotransferases class-III pyridoxal-phosphate
BRACE3040239//Zinc finger, C3HC4 type (RING finger)
BRACE3040644//Low-density lipoprotein receptor domain class A// EB module// Low-density lipoprotein receptor domain class A// CUB domain// Low-density lipoprotein receptor domain class A// Low-density lipoprotein receptor domain class A
BRACE3041059//Ubiquitin carboxyl-terminal hydrolases family 2// Ubiquitin carboxyl-terminal hydrolase family 2
BRACE3041162//Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Putative zinc finger in N-recognin
BRACE3042046//RhoGEF domain// PH domain
BRACE3042409//AMP-binding enzyme
BRACE3042432//7 transmembrane receptor (Secretin family)
BRACE3043597//KOW motif
BRACE3044172//Phosphatidylinositol 3- and 4-kinases
BRACE3045424//PH domain
BRACE3046466//EGF-like domain// EGF-like domain// Laminin G domain// Laminin G domain// EGF-like domain// Laminin G domain// EGF-like domain// Laminin G domain// Laminin G domain// EGF-like domain// EGF-like domain// Laminin EGF-like (Domains III and V)// EB module// EGF-like domain// EGF-like domain
BRACE3046491//PDZ domain (Also known as DHR or GLGF).// PDZ domain (Also known as DHR or GLGF).// PDZ domain (Also known as DHR or GLGF).// SH3 domain// Guanylate kinase
BRACE3047482//Zinc finger, C3HC4 type (RING finger)// Zinc finger, C3HC4 type (RING finger)// B-box zinc finger.// CONSTANS family zinc finger// B-box zinc finger.// Fibronectin type III domain// SPRY domain
BRACE3048615//Leucine Rich Repeat// Leucine Rich Repeat
BRACE3049714//Dihydroneopterin aldolase
BRACE3050270//Reverse transcriptase (RNA-dependent DNA polymerase)
BRACE3050504//Ion transport protein
BRACE3051621//Latrophilin/CL-1-like GPS domain// PLAT/LH2 domain// Regulator of G protein signaling domain BRACE3051819//Myosin head (motor domain)// IQ calmodulin-binding motif// IQ calmodulin-binding motif// Myosin tail// KE2 family protein// Myosin tail// lactate/malate dehydrogenase// Troponin// Myosin tail
BRACE3052321//SH3 domain
BRACE3052410//Viral methyltransferase
BRACE3052595//C2 domain
BRALZ2010842//Mitochondrial carrier proteins
BRALZ2013621//KH domain
BRAMY2031516//wnt family of developmental signaling proteins
BRAMY2040915//Immunoglobulin domain
BRAMY2041347//Mov34/MPN/PAD-1 family
BRAMY2041384//Annexin// Annexin// Annexin// Annexin// Annexin// Annexin
BRAMY2046537//PMP-22/EMP/MP20/Claudin family
BRAMY3000692//Zinc finger, C2H2 type// Zinc finger, C2H2 type
BRAMY3002886//Domain of unknown function// CBS domain// CBS domain
BRAMY3004126//Transmembrane 4 family// 7 transmembrane receptor (rhodopsin family)
BRAMY3005184//ZU5 domain// Death domain
BRAMY3007078//Inositol monophosphatase family// PHD-finger// PHD-finger
BRAMY3009491//Phosphatidylinositol 3- and 4-kinases
BRAMY3010321//SAM domain (Sterile alpha motif)// PH domain// PH domain// Putative GTP-ase activating protein for Arf
BRAMY3011501//SAP domain// SPRY domain
BRAMY3011581//Prokaryotic DNA topoisomerase// Topoisomerase DNA binding C4 zinc finger// PHD-finger// Zinc finger, CCHC class
BRAMY3014027//KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type
BRAMY3014613//RhoGAP domain// haloacid dehalogenase-like hydrolase
BRAMY3015086//FERM domain (Band 4.1 family)
BRAMY3015547//RhoGEF domain
BRAMY3015549//Immunoglobulin domain// Immunoglobulin domain// Immunoglobulin domain// Immunoglobulin domain// Immunoglobulin domain// Fibronectin type III domain// Fibronectin type III domain// Fibronectin type III domain// Fibronectin type III domain
BRAMY3017920//RhoGEF domain// PH domain// RhoGAP domain
BRAMY3018754//Protein of unknown function// Domain of unknown function
BRAMY4000915//Ank repeat// Ank repeat// Ank repeat// Ank repeat// Ank repeat// Ank repeat// Ank repeat// Ank repeat// Ank repeat// Ank repeat// Glutamine amidotransferases class-II// Ank repeat// Ank repeat// Ank repeat// Ank repeat// Ank repeat// Ank repeat// Hantavirus nucleocapsid protein// Ank repeat// Ank repeat
BRAMY4000962//Tudor domain
BRAMY4001652//Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ank repeat// Ribosomal protein L34// Ank repeat// Ank repeat// Ank repeat// Uncharacterized protein family UPF0028
BRAMY4002575//Zinc finger, C2H2 type// Transcription factor S-II (TFIIS)// Zinc finger, C2H2 type// Zinc finger, C2H2 type
BRAWH2000256//Progesterone receptor// Cytochrome c oxidase subunit III// Sulfate transporter family
BRAWH2002333//3'5'-cyclic nucleotide phosphodiesterase
BRAWH2011796//S-100/ICaBP type calcium binding domain// EF hand
BRAWH2011812//Syndecan domain// BNR repeat// BNR repeat// BNR repeat// BNR repeat// BNR repeat// PKD domain
BRAWH2012866//C2 domain// C2 domain
BRAWH2014053//Sigma-54 transcription factors// ATPases associated with various cellular activities (AAA)
BRAWH2016209//Zinc finger, C3HC4 type (RING finger)// PHD-finger
BRAWH2016223//TPR Domain
BRAWH2016785//Pyridine nucleotide-disulphide oxidoreductase
BRAWH3000446//Zinc finger, C2H2 type// Zinc finger, C2H2 type// Zinc finger, C2H2 type// Zinc finger, C2H2 type// Zinc finger, C2H2 type
BRAWH3001783//Cadherin domain// Cadherin domain// Cadherin domain// Cadherin domain// Cadherin domain
BRAWH3003573//EF hand// EF hand// EF hand
BRAWH3005886//Rubredoxin// PHD-finger
BRAWH3008167//Sushi domain (SCR repeat)// CUB domain// Sushi domain (SCR repeat)// CUB domain// Sushi domain (SCR repeat)// CUB domain// Sushi domain (SCR repeat)// CUB domain
BRAWH3009961//PHD-finger// Glycophorin A// PDZ domain (Also known as DHR or GLGF).// C2 domain// C2 domain BRAWH3010657//Ubiquitin carboxyl-terminal hydrolases family 2// Ubiquitin carboxyl-terminal hydrolase family 2
BRAWH3011331//Disintegrin
BRAWH3011577//KRAB box
BRAWH3011623//RNA recognition motif. (a.k.a. RRM, RBD, or RNP domain)
BRAWH3013009//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// DM DNA binding domain// Zinc finger, C2H2 type
BRAWH3013264//SNF2 and others N-terminal domain// SNF2 and others N-terminal domain// TPR Domain// 3C cysteine protease (picornain 3C)// Zinc finger, C3HC4 type (RING finger)// Plant PEC family metallothionein// Helicases conserved C-terminal domain
BRAWH3014609//Leucine rich repeat N-terminal domain// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine rich repeat C-terminal domain
BRAWH3015175//Xylose isomerase// Intermediate filament proteins// Helix-loop-helix DNA-binding domain// ATP synthase Alpha chain, C terminal
BRAWH3015610//TFIIE beta subunit core domain
BRAWH3017180//Phorbol esters/diacylglycerol binding domain (C1 domain)// Diacylglycerol kinase catalytic domain (presumed)// Diacylglycerol kinase accessory domain (presumed)// Ank repeat// Ank repeat
BRAWH3017259//Carboxylesterases// Syntaxin// SAM domain (Sterile alpha motif)
BRAWH3017260//Pancreatic hormone peptides
BRAWH3017477//Zinc finger, C2H2 type// Zinc finger, C2H2 type// Zinc finger, C2H2 type
BRAWH3018063//Rap/ran-GAP
BRAWH3018548//Intermediate filament proteins// Vinculin family
BRAWH3019026//WD domain, G-beta repeat// WD domain, G-beta repeat// WD domain, G-beta repeat// Protein phosphatase 2A regulatory subunit PR55// WD domain, G-beta repeat// WD domain, G-beta repeat// WD domain, G-beta repeat
BRAWH3021574//Wiskott Aldrich syndrome homology region 2
BRAWH3021580//Intermediate filament proteins// Zinc finger, CCHC class
BRAWH3021724//Transglutaminase-like superfamily
BRAWH3022347//Leucine Rich Repeat
BRAWH3022431//FGGY family of carbohydrate kinases
BRAWH3022651//PHD-finger// Zn-finger in ubiquitin-hydrolases and other proteins
BRAWH3022719//Eukaryotic initiation factor 4E
BRAWH3023156//Neurotransmitter-gated ion-channel
BRAWH3023415//Glycosyl hydrolase family 47
BRAWH3024186//Fibronectin type III domain// Protein-tyrosine phosphatase// Dual specificity phosphatase, catalytic domain// Protein-tyrosine phosphatase
BRAWH3024231//TPR Domain// TPR Domain// TPR Domain
BRAWH3024506//I/LWEQ domain
BRAWH3025157//Ank repeat// Ank repeat// Ank repeat// BTB/POZ domain// K+ channel tetramerisation domain
BRAWH3026349//PX domain
BRAWH3026938//PDZ domain (Also known as DHR or GLGF).
BRAWH3027440//TPR Domain// TPR Domain// TPR Domain// TPR Domain// PPR repeat
BRAWH3027533//AN1-like Zinc finger// PHD-finger// FYVE zinc finger
BRAWH3027574//TBC domain// EF hand
BRAWH3027607//DNA binding domain with preference for A/T rich regions
BRAWH3027806//WD domain, G-beta repeat// WD domain, G-beta repeat// WD domain, G-beta repeat// WD domain, G-beta repeat// Arenavirus nucleocapsid protein// WD domain, G-beta repeat// WD domain, G-beta repeat
BRAWH3027880//Integral membrane protein// AN1-like Zinc finger// DHHC zinc finger domain
BRAWH3028796//Zinc finger C-x8-C-x5-C-x3-H type (and similar).// Zinc finger C-x8-C-x5-C-x3-H type (and similar).// Zinc finger C-x8-C-x5-C-x3-H type (and similar).// Zinc finger, C3HC4 type (RING finger)// Integrase Zinc binding domain// Zinc finger C-x8-C-x5-C-x3-H type (and similar).// Protein phosphatase 2A regulatory B subunit (B56 family)
BRAWH3029385//PH domain// Dynamin GTPase effector domain
BRAWH3029806//Copper/zinc superoxide dismutase (SODC)// Adenylate and Guanylate cyclase catalytic domain// NADH-ubiquinone oxidoreductase chain 4, amino terminus// Endothelin family
BRAWH3030772//Ank repeat// Ank repeat// Ank repeat
BRAWH3030910//SAM domain (Sterile alpha motif)
BRAWH3031342//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Putative zinc finger in N-recognin// Zinc finger, C2H2 type// Zinc finger, C2H2 type// Transcription factor S-II (TFIIS)// Src homology domain 2// Zinc finger, C2H2 type// Zinc finger, C2H2 type// Src homology domain 2// Zinc finger, C2H2 type
BRAWH3031710//Zinc finger, C2H2 type// Zinc finger, C2H2 type// Zinc finger, C2H2 type// Zinc finger, C2H2 type// Zinc finger, C2H2 type
BRAWH3032298//EGF-like domain// EGF-like domain// EGF-like domain// EB module// EGF-like domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibrinogen beta and gamma chains, C-terminal globular domain
BRAWH3032340//RhoGEF domain// PH domain// Ezrin/radixin/moesin family
BRAWH3032571//PHD-finger// von Willebrand factor type C domain// PHD-finger// 'chromo' (CHRromatin Organization MOdifier) domain// 'chromo' (CHRromatin Organization MOdifier) domain// DEAD/DEAH box helicase// SNF2 and others N-terminal domain// Helicases conserved C-terminal domain
BRAWH3033448//TPR Domain// TPR Domain
BRAWH3033513//Thiolase
BRAWH3034668//Ubiquitin carboxyl-terminal hydrolases family 2// E7 protein, Early protein// Ubiquitin carboxyl-terminal hydrolase family 2
BRAWH3034743//ATP synthase (E/31 kDa) subunit// Ezrin/radixin/moesin family
BRAWH3034775//SAP domain// SPRY domain
BRAWH3035403//RNA recognition motif. (a.k.a. RRM, RED, or RNP domain)// RNA recognition motif. (a.k.a. RRM, RBD, or RNP domain)
BRAWH3035914//TBC domain
BRAWH3035936//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRAWH3036247//BTB/POZ domain// Zinc finger, C2H2 type// Zinc finger, C2H2 type// Zinc finger, C2H2 type// Zinc finger, C2H2 type// Phorbol esters/diacylglycerol binding domain (C1 domain)// Zinc finger, C2H2 type// Ribosomal protein L31// Zinc finger, C2H2 type// Zinc finger, C2H2 type// Zinc finger, C2H2 type// Zinc finger, C2H2 type
BRAWH3036270//Atrial natriuretic peptide
BRAWH3036334//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRAWH3037265//SURF4 family
BRAWH3037428//ADP-ribosylation factor family// Ras family BRAWH3037979//wnt family of developmental signaling proteins// Ubiquitin carboxyl-terminal hydrolases family 2// Ubiquitin carboxyl-terminal hydrolase family 2
BRAWH3038055//Spectrin repeat// Myb-like DNA-binding domain// Spectrin repeat// 7-fold repeat in Clathrin and VPS// Spectrin repeat// Spectrin repeat// Spectrin repeat// Spectrin repeat// Spectrin repeat
BRAWH3038252//Formin Homology 2 Domain
BRAWH3038324//Dehydrins
BRAWH3038827//Kelch motif// Kelch motif// Kelch motif// Kelch motif// BTB/POZ domain// BTB/POZ domain BRAWH3041556//Tetrahydrofolate dehydrogenase/cyclohydrolase// Formate--tetrahydrofolate ligase
BRAWH3041928//Wilm's tumour protein
BRAWH3042438//'Paired box' domain// EF hand// Phorbol esters/diacylglycerol binding domain (C1 domain)
BRAWH3042568//Homeobox domain
BRAWH3042772//Cation efflux family
BRAWH3042787//Gag P30 core shell protein// Zinc finger, CCHC class
BRAWH3043295//Inorganic pyrophosphatase
BRAWH3044122//C2 domain// Sigma-70 factor
BRAWH3044151//Thrombospondiri type 1 domain// Thrombospondin type 1 domain// Thrombospondin type 1 domain// Keratin, high sulfur B2 protein// Thrombospondin type 1 domain
BRAWH3044985//Phosphotriesterase family
BRAWH3045118//DnaJ domain
BRAWH3046424//Histone deacetylase family
BRAWH3047692//Shikimate kinase// ATPases associated with various cellular activities (AAA)
BRAWH3048374//Sushi domain (SCR repeat)// Sushi domain (SCR repeat)// Sushi domain (SCR repeat)// Sushi domain (SCR repeat)// Sushi domain (SCR repeat)// Sushi domain (SCR repeat)// Sushi domain (SCR repeat)// Keratin, high sulfur B2 protein// Sushi domain (SCR repeat)
BRAWH3048724//Subtilase family
BRAWH3049544//Glycosyl transferases// Similarity to lectin domain of ricin beta-chain, 3 copies.
BRCAN2000923//ADP-ribosylation factor family// Ras family
BRCAN2002892//ADP-ribosylation factor family// Ras family
BRCAN2003269//ABC transporter
BRCAN2003814//Phosphatidylinositol-specific phospholipase C, X domain
BRCAN2006051//Peptidase family M41
BRCAN2010665//PDZ domain (Also known as DHR or GLGF).// PDZ domain (Also known as DHR or GLGF).// PDZ domain (Also known as DHR or GLGF).
BRCAN2015402//Cytochrome P450
BRCAN2019907//EF hand
BRCAN2020234//Lipocalin / cytosolic fatty-acid binding protein family
BRCAN2020331//RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
BRCAN2021325//Zinc carboxypeptidase// Zinc carboxypeptidase
BRCAN2021452//Zinc finger, CCHC class// Zinc finger, CCHC class
BRCAN2021718//Ribosomal protein L10
BRCAN2022126//IstB-like ATP binding protein// Receptor family ligand binding region// Bacterial extracellular solute-binding proteins, family 3// Ligand-gated ion channel
BRCAN2025093//Ank repeat// Flagellar FliJ protein// Ank repeat BRCOC2001355//GTP1/OBG family// Phosphoribulokinase// Adenylylsulfate kinase
BRCOC2006164//ATP synthase subunit C// Nucleoside transporter// Sugar (and other) transporter// Influenza RNA-dependent RNA polymerase subunit PA
BRCOC2006639//Leucine Rich Repeat// Leucine Rich Repeat// Leucine Rich Repeat// Leucine Rich Repeat
BRCOC2012386//KRAB box// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type
BRHIP2008756//MAS20 protein import receptor// BRCA1 C Terminus (BRCT) domain
BRHIP2013958//Domain of unknown function// MSP (Major sperm protein) domain
BRHIP2026346//Formin Homology 2 Domain
BRHIP2027077//Zinc finger, C2H2 type// Zinc finger, C2H2 type
BRHIP2027563//Kelch motif
BRHIP2029663//TPR Domain// Zinc finger, C3HC4 type (RING finger)// ATP-dependent protease La (LON) domain
BRHIP3000859//Wilm's tumour protein
BRHIP3001481//Protein-tyrosine phosphatase
BRHIP3001878//POT family
BRHIP3002000//Peptidase family M20/M25/M40// Sugar (and other) transporter
BRHIP3002114//Mandelate racemase / muconate lactonizing enzyme family
BRHIP3002141//DNA polymerase (viral) C-terminal domain
BRHIP3003063//cAMP phosphodiesterases class-II// Vinculin family
BRHIP3003126//HECT-domain (ubiquitin-transferase).
BRHIP3003306//Uncharacterized protein family// Rap/ran-GAP
BRHIP3003340//Actin
BRHIP3003795//Cytochrome P450
BRHIP3004710//TPR Domain// TPR Domain// TPR Domain
BRHIP3004725//tRNA synthetases class I (C)
BRHIP3005037//BAH domain// ELM2 domain// Myb-like DNA-binding domain// GATA zinc finger
BRHIP3005142//Adaptin N terminal region
BRHIP3005231//TPR Domain// TPR Domain
BRHIP3005307//Glutathione S-transferases.// Uncharacterized protein family UPF0028
BRHIP3005673//Glutathione S-transferases.
BRHIP3005944//Scavenger receptor cysteine-rich domain// Scavenger receptor cysteine-rich domain
BRHIP3006294//SH3 domain// RhoGAP domain
BRHIP3006449//Armadillo/beta-catenin-like repeats// Armadillo/beta-catenin-like repeats// Armadillo/beta-catenin-like repeats// Armadillo/beta-catenin-like repeats// Armadillo/beta-catenin-like repeats
BRHIP3006786//Sec7 domain
BRHIP3007195//Type I restriction modification DNA specificity domain// E1-E2 ATPase// E1-E2 ATPase// Neuraxin and MAP1B proteins// E1-E2 ATPase// Cof family
BRHIP3007223//Ubiquitin carboxyl-terminal hydrolases family 2// Ubiquitin carboxyl-terminal hydrolase family 2
BRHIP3007424//wnt family of developmental signaling proteins
BRHIP3007609//PHD-finger
BRHIP3008320//Transmembrane region cyclic Nucleotide Gated Channel
BRHIP3009753//CUB domain// Low-density lipoprotein receptor domain class A
BRHIP3010289//Delta serrate ligand// Kelch motif// Kelch motif// Plexin repeat// Plexin repeat// Lectin C-type domain// Plexin repeat// Plexin repeat// Laminin EGF-like (Domains III and V)// Keratin, high sulfur B2 protein// Laminin EGF-like (Domains III and V)
BRHIP3011082//HECT-domain (ubiquitin-transferase).
BRHIP3011460//RhoGEF domain// PH domain// SH3 domain
BRHIP3011567//Dihydropyridine sensitive L-type calcium channel (Beta subunit)// Dihydropyridine sensitive L-type calcium channel (Beta subunit)
BRHIP3012289//Flavin containing amine oxidase
BRHIP3012736//Collagen triple helix repeat (20 copies)
BRHIP3014675//Ank repeat// Ank repeat// Ank repeat// BTB/POZ domain// K+ channel tetramerisation domain
BRHIP3016032//Integral membrane protein// Divalent cation transporter// TPR Domain// TPR Domain// TPR Domain
BRHIP3017109//Src homology domain 2
BRHIP3017256//LIM domain containing proteins// AN1-like Zinc finger
BRHIP3017558//eubacterial secY protein// FecCD transport family// Domain of unknown function// Sugar (and other) transporter// Sodium:galactoside symporter family// Monocarboxylate transporter
BRHIP3019643//Biopterin-dependent aromatic amino acid hydroxylase BRHIP3019824//Prokaryotic transcription elongation factor, GreA/GreB
BRHIP3020733//Keratin, high sulfur B2 protein
BRHIP3021019//Protein-tyrosine phosphatase// Dual specificity phosphatase, catalytic domain
BRHIP3025795//Sugar (and other) transporter// Protein of unknown function// Sodium:galactoside symporter family// Herpesvirus glycoprotein M// Monocarboxylate transporter
BRHIP3027191//Hsp70 protein
BRHIP3027651//PHD-finger// PHD-finger
BRHIP3028246//CXXC zinc finger// PHD-finger// Beta type Zein
BRHIP3028570//PH domain// Fibroblast growth factor
BRHIP3029409//NTR/C345C module
BRHIP3030230//Pentaxin family
BRHIP3032374//Glutathione S-transferases.// Uncharacterized protein family UPF0028
BRHIP3033557//Pyridoxamine 5'-phosphate oxidase// Ligand-gated ion channel
BRHIP3033734//Keratin, high sulfur B2 protein
BRHIP3035006//Sialyltransferase family
BRHIP3036371//Immunoglobulin domain// Kunitz/Bovine pancreatic trypsin inhibitor domain// Kunitz/Bovine pancreatic trypsin inhibitor domain
BRHIP3036715//Class I Histocompatibility antigen, domains alpha 1 and 2// Immunoglobulin domain
BRHIP3036936//Fibronectin type III domain
BRHIP3037543//Eukaryotic protein kinase domain
BRHIP3038030//Pyridine nucleotide-disulphide oxidoreductase// Phytoene dehydrogenase related enzyme
BRHIP3038735//Immunoglobulin domain// Immunoglobulin domain// Immunoglobulin domain
BRHIP3039509//Notch (DSL) domain// Amiloride-sensitive sodium channel
BRHIP3039592//Zinc finger, C3HC4 type (RING finger)
BRHIP3041587//Eukaryotic protein kinase domain
BRSSN2004710//3'5'-cyclic nucleotide phosphodiesterase
BRSSN2011843//bZIP transcription factor// RNA polymerase alpha subunit
BRSSN2015497//Tudor domain
BRSTN2006638//EF hand// EF hand// Pancreatic hormone peptides
BRSTN2010089//Cell cycle protein// 7 transmembrane receptor (Secretin family)
BRSTN2011961//Thioredoxin
BRSTN2012069//Elongation factor Tu family
BRSTN2016918//Transposase// Ezrin/radixin/moesin family// Intermediate filament proteins
BRTHA2001304//Exonuclease
BRTHA2005448//Calpain family cysteine protease// Calpain large subunit, domain III// EF hand
BRTHA2019726//Immunoglobulin domain// Immunoglobulin domain// Immunoglobulin domain// Immunoglobulin domain
BRTHA2020400//Immunoglobulin domain
BRTHA2020721//Immunoglobulin domain// Immunoglobulin domain// Immunoglobulin domain// Immunoglobulin domain
BRTHA2020910//Tubulin/FtsZ family
BRTHA2025869//Intermediate filament proteins// Intermediate filament proteins
BRTHA2026290//Guanine nucleotide exchange factor for Ras-like GTPases; N-terminal motif// Initiator RepB protein// RasGEF domain
BRTHA2026311//Thioredoxin// Thioredoxin
BRTHA2027250//C2 domain// C2 domain
BRTHA2030036//Lipase
BRTHA2031917//Fibronectin type III domain// Photosynthetic reaction center protein
BRTHA2033683//E1-E2 ATPase
BRTHA2035743//Serine hydroxymethyltransferase// UBA domain
BRTHA2036295//Helper component proteinase
BRTHA2037247//Reverse transcriptase (RNA-dependent DNA polymerase)
BRTHA2038345//Ank repeat// Ank repeat// Ank repeat
BRTHA3000456//Zinc finger, C2H2 type// DM DNA binding domain// Zinc finger, C2H2 type// Transcription factor S-II (TFIIS)// Zinc finger, C2H2 type// Putative zinc finger in N-recognin// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRTHA3003225//Zinc finger, C3HC4 type (RING finger)// PHD-finger
BRTHA3003736//DEAD/DEAH box helicase// Helicases conserved C-terminal domain
BRTHA3010135//Protein-tyrosine phosphatase// Protein-tyrosine phosphatase
BRTHA3010212//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRTHA3011187//EF hand
BRTHA3011194//Voltage gated chloride channels// CBS domain
BRTHA3011361//Calponin homology (CH) domain// LIM domain containing proteins
BRTHA3012265//Sulfate transporter family// Ribosomal protein S3, C-terminal domain.
BRTHA3014000//Birnavirus VP3 protein// Zinc finger, C2H2 type// ELM2 domain// Myb-like DNA-binding domain// Zinc finger, C2H2 type// Zinc finger, C2H2 type
BRTHA3014547//Adenylate kinase// Viral (Superfamily 1) RNA helicase// Arsenical pump membrane protein// Ank repeat// Ank repeat// Ank repeat// Ank repeat// Ank repeat// Dehydrogenase E1 component// Ank repeat// Shikimate / quinate 5-dehydrogenase// Ank repeat// Ank repeat// Ank repeat// Ank repeat// Ank repeat// Ank repeat// Ank repeat// Ank repeat// Ank repeat// Ank repeat// Ank repeat
BRTHA3017791//Lectin (probable mannose binding)
BRTHA3018409//Wilm's tumour protein
BRTHA3020771//PH domain
BRTHA3021708//PH domain
BRTHA3021786//Protein prenyltransferase alpha subunit repeat
BRTHA3021971//Putative peptidoglycan binding domain
BRTHA3023403//Phosphatidylinositol-specific phospholipase C, X domain// Phosphatidylinositol-specific phospholipase C, X domain// C2 domain
BRTHA3025073//Calponin homology (CH) domain
BRTHA3026161//Adenosine-deaminase (editase) domain
BRTHA3026916//Guanine nucleotide exchange factor for Ras-like GTPases; N-terminal motif// RasGEF domain// Ras association (RalGDS/AF-6) domain
BRTHA3027171//Scorpion short toxins
BRTHA3027638//Matrix protein (MA), p15
BRTHA3028339//Zn-finger in Ran binding protein and others.
CERVX2000968//Immunoglobulin domain
CHONS2000797//T-box
CHONS2001287//Insulin-like growth factor binding proteins// Thyroglobulin type-1 repeat
CHONS2001834//Plexin repeat
CHONS2002829//F5/8 type C domain// Zinc carboxypeptidase// Zinc carboxypeptidase// Zinc carboxypeptidase
COLON2004351//Galactosyltransferase
COLON2005735//CbiM// Ammonium Transporter Family
CTONG2003517//RNA recognition motif. (a.k.a. RRM, RBD, or RNP domain)
CTONG2006235//Zn-finger in ubiquitin-hydrolases and other proteins
CTONG2008989//Connexin
CTONG2009570//WD domain, G-beta repeat// WD domain, G-beta repeat// WD domain, G-beta repeat// WD domain, G-beta repeat//
WD domain, G-beta repeat
CTONG2010330//Lysophospholipase catalytic domain
CTONG2011801//BTB/POZ domain// Zinc finger, C2H2 type// Zinc finger, C2H2 type// Transcription factor S-II (TFIIS)// Zinc finger, C2H2 type// Zinc finger, C2H2 type// Zinc finger, C2H2 type// PHD-finger// Zinc finger, C2H2 type// Zinc finger, C2H2 type
CTONG2020582//AMP-binding enzyme
CTONG2026987//Reverse transcriptase (RNA-dependent DNA polymerase)
D9OST2003106//RN recognition motif. (a.k.a. RRM, RED, or RNP domain)// RNA recognition motif. (a.k.a. RRM, RBD, or RNP domain)
D9OST2004417//Ribosomal protein L13e
DFNES2011221//Rotavirus NS26
ERLTF2002178//Kelch motif// Kelch motif// Kelch motif// Kelch motif// Kelch motif// Kelch motif
ERLTF2002369//PH domain// RhoGAP domain// Tropomyosins
FCBBF3001018//HMGL-like
FCBBF3012443//Leucine rich repeat N-terminal domain// Leucine Rich Repeat// Leucine Rich Repeat// Leucine Rich Repeat// Leucine Rich Repeat// Leucine Rich Repeat// Leucine rich repeat C-terminal domain
FCBBF3020030//B-box zinc finger.// B-box zinc finger.// Putative zinc finger in N-recognin
FCBBF3021191//Protein phosphatase 2C// Protein phosphatase 2C
FCBBF3024911//PWWP domain
FCBBF5000384//BAF60b domain of the SWIB complex
FEBRA2000805//Uncharacterized protein family UPF0054
FEBRA2002260//CXXC zinc finger
FEBRA2013570//Dehydrogenase E1 component
FEBRA2023498//Leucine rich repeat N-terminal domain// Leucine Rich Repeat// Leucine Rich Repeat// Leucine Rich Repeat
FEBRA2026582//PPIC-type PPIASE domain.
FEBRA2028457//RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)// RNA recognition motif. (a.k.a. RRM, RBD, or RNP domain)
FEHRT2002708//DNA gyrase/topoisomerase IV, subunit A
FEKID2001001//SH3 domain// WW domain// WW domain// PH domain
FEKID2002493//wnt family of developmental signaling proteins
FEKID2002637//Ser/Thr protein phosphatase
FELNG2000720//Immunoglobulin domain// Immunoglobulin domain
FELNG2001953//Src homology domain 2
HCASM2008154//RNA recognition motif. (a.k.a. RRM, RBD, or RNP domain)// Zn-finger in Ran binding protein and others.
HCHON2009766//eIF4-gamma/eIF5/eIF2-epsilon
HHDPC2008185//Helper component proteinase// Site-specific recombinases
HSYRA2004550//E1-E2 ATPase// E1-E2 ATPase
HSYRA2007338//Fibronectin type III domain
JCMLC2000273//Lysosome-associated membrane glycoprotein (Lamp)// Integrin alpha cytoplasmic region
JCMLC2002751//von Willebrand factor type D domain// Plant PEC family metallothionein// Trypsin Inhibitor like cysteine rich domain// von Willebrand factor type C domain// von Willebrand factor type D domain
KIDNE2004531//Prion protein// Integral membrane protein// Cytochrome c oxidase subunit III// Uncharacterized protein family
KIDNE2010049//FGGY family of carbohydrate kinases// FGGY family of carbohydrate kinases
KIDNE2015987//EGF-like domain// Keratin, high sulfur B2 protein// Zona pellucida-like domain
KIDNE2018268//Zinc finger, C2H2 type
LYMPB2002236//ABC 3 transport family// CbiM// NADH-ubiquinone oxidoreductase chain 4, amino terminus// UDP-glucoronosyl and UDP-glucosyl transferases
LYMPB2002344//TBC domain
LYMPB2002458//Fibronectin type III domain// Fibronectin type III domain// Fibrinogen beta and gamma chains, C-terminal globular domain
N1ESE2000698//WD domain, G-beta repeat// WD domain, G-beta repeat// WD domain, G-beta repeat// WD domain, G-beta repeat// WD domain, G-beta repeat// WD domain, G-beta repeat// WD domain, G-beta repeat// WD domain, G-beta repeat
NETRP2003103//Zinc finger, C3HC4 type (RING finger)// PHD-finger
NETRP2003448//ADP-ribosylation factor family// Ras family
NETRP2004017//Histone-like transcription factor (CBF/NF-Y) and archaeal histone// 2S seed storage family
NT2RI2004818//Phosphatidylinositol-specific phospholipase C, X domain// Phosphatidylinositol-specific phospholipase C, X domain// C2 domain// Fes/CIP4 homology domain
NT2RI3001573//Leucine Rich Repeat// Leucine Rich Repeat// Leucine Rich Repeat// Leucine Rich Repeat
NT2RI3001967//Ank repeat// Ank repeat// Ank repeat// Ank repeat// Glutathione S-transferases.// SAM domain (Sterile alpha motif)
NT2RI3005923//Cadherin domain// Cadherin domain// Cadherin domain// Cadherin domain// Cadherin domain// Cadherin domain// Cadherin domain// Cadherin domain// Cadherin domain// Cadherin domain// Cadherin domain
NT2RI3009480//Zinc finger, C2H2 type// Zinc finger, C2H2 type// Zinc finger, C2H2 type
NT2RI3009524//EGF-like domain// EGF-like domain// EGF-like domain// EGF-like domain// EGF-like domain// EGF-like domain// EGF-like domain// EGF-like domain// Metallothionein// EGF-like domain// Laminin G domain// Laminin G domain// EGF-like domain// Laminin G domain// EGF-like domain// Laminin G domain// Laminin G domain// EGF-like domain// EGF-like domain
NT2RP7007387//Armadillo/beta-catenin-like repeats// picornavirus capsid protein
NT2RP7016508//DEAD/DEAH box helicase// Helicases conserved C-terminal domain
NT2RP7019682//Cation efflux family
NT2RP7020343//Transforming growth factor beta like domain// Keratin, high sulfur B2 protein
NT2RP8000633//VPR/VPX protein
NT2RP8001363//TNFR/NGFR cysteine-rich region// CUB domain
NT2RP8001604//CUB domain// Sushi domain (SCR repeat)// CUB domain// Sushi domain (SCR repeat)// CUB domain// Sushi domain (SCR repeat)// CUB domain// Sushi domain (SCR repeat)// CUB domain// Sushi domain (SCR repeat)// CUB domain// Sushi domain (SCR repeat)// CUB domain
NT2RP8003490//Hemagglutinin-neuraminidase// LIM domain containing proteins// LIM domain containing proteins// Homeobox domain
NT2RP8003787//Thermophilic metalloprotease (M29)// Ank repeat// Ion transport protein// Sodium:galactoside symporter family
NT2RP8005546//Viral (Superfamily 1) RNA helicase
NT2RP8006452//African swine fever virus multigene family 360 protein// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat
NT2RP8007920//PPR repeat// LIM domain containing proteins
NT2RP8008057//Thrombospondin type 1 domain// Immunoglobulin domain// Transforming growth factor beta like domain
NT2RP8009119//Picornavirus 2B protein
NTONG2003805//KRAB box// Zinc finger, C2H2 type// Zinc finger, C2H2 type// Zinc finger, C2H2 type// TRAF-type zinc finger// Zinc finger, C2H2 type// Transcription factor S-II (TFIIS)// Zinc finger, C2H2 type// Zinc finger, C2H2 type// Zinc finger, C2H2 type// Zinc finger, C2H2 type
NTONG2008483//WD domain, G-beta repeat// WD domain, G-beta repeat// WD domain, G-beta repeat// WD domain, G-beta repeat// WD domain, G-beta repeat// WD domain, G-beta repeat// Zinc finger, C2H2 type
OCBBF2000831//WAP-type (Whey Acidic Protein) 'four-disulfide core'// Kazal-type serine protease inhibitor domain// Immunoglobulin domain// Kunitz/Bovine pancreatic trypsin inhibitor domain// Kunitz/Bovine pancreatic trypsin inhibitor domain// Furin-like cysteine rich region// Respiratory-chain NADH dehydrogenase, 49 Kd subunit// NTR/C345C module
OCBBF2003518//Transient receptor// Transient receptor
OCBBF2004478//Trypanosome variant surface glycoprotein// 7 transmembrane receptor (Secretin family)
OCBBF2007039//Reprolysin family propeptide// Reprolysin (M12B) family zinc metalloprotease// Thrombospondin type 1 domain// EB module
OCBBF2009536//Amiloride-sensitive sodium channel
OCBBF2014745//Zinc finger, C2H2 type// Zinc finger, C2H2 type
OCBBF2016928//KRAB box// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type
OCBBF2018618//7 transmembrane receptor (Secretin family)// 7 transmembrane receptor (rhodopsin family)
OCBBF2024589//Glutamine amidotransferases class-II// Dihydroorotase-like
OCBBF2030927//Neuregulin family// von Willebrand factor type A domain// EGF-like domain// Response regulator receiver domain// von Willebrand factor type A domain// von Willebrand factor type A domain
OCBBF3001202//DENN (AEX-3) domain
OCBBF3001333//Zinc finger, C2H2 type// Zinc finger, C2H2 type// Zinc finger, C2H2 type// Zinc finger, C2H2 type// Zinc finger// Transcription factor S-II (TFIIS)// Zinc finger, C2H2 type// Zinc finger, C2H2 type// Zinc finger, C2H2 type// Zinc finger, C2H2 type// Zinc finger, C2H2 type
OCBBF3001616//Reverse transcriptase (RNA-dependent DNA polymerase)
OCBBF3004487//Platelet-derived growth factor (PDGF)// DEAD/DEAH box helicase
OCBBF3005330//Domain found in Dishevelled, Egl-10, and Pleckstrin// TCP-1/cpn60 chaperonin family// TCP-1/cpn60 chaperonin family
OCBBF3006986//Beige/BEACH domain// WD domain, G-beta repeat// WD domain, G-beta repeat
OCBBF3008392//DNA binding domain with preference for A/T rich regions// PHD-finger// Bromodomain
OCBBF3008835//Collagen triple helix repeat (20 copies)
OCBBF3019269//CXXC zinc finger
OCBBF3020263//KRAB box// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type
OCBBF3021086//Influenza RNA-dependent RNA polymerase subunit PA// Reprolysin family propeptide// Leptin
OCBBF3021361//ELM2 domain// Myb-like DNA-binding domain
OCBBF3021502//Leptin
OCBBF3022827//A20-like zinc finger// Vacuolar sorting protein 9 (VPS9) domain
OCBBF3023175//Protein phosphatase 2C// Beige/BEACH domain// WD domain, G-beta repeat// WD domain, G-beta repeat// WD domain, G-beta repeat// WD domain, G-beta repeat
OCBBF3023913//R3H domain// Retroviral Vif (Viral infectivity) protein
OCBBF3025475//Sodium:sulfate symporter transmembrane region// Sodium:sulfate symporter transmembrane region
OCBBF3025503//Vesiculovirus phosphoprotein// haloacid dehalogenase-like hydrolase
OCBBF3026088//Fatty acid desaturase
OCBBF3026361//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// RNA polymerases M/15 Kd subunits
OCBBF3026979//Laminin G domain// Thrombospondin N-terminal -like domains// von Willebrand factor type C domain// von Willebrand factor type C domain// EGF-like domain// EB module// EGF-like domain// Plant PEC family metallothionein// EGF-like domain// Trypsin Inhibitor like cysteine rich domain// Metallothionein// EGF-like domain// EGF-like domain// EGF-like domain// EGF-like domain// von Willebrand factor type C domain// von Willebrand factor type C domain
OCBBF3027969//Ank repeat// Ank repeat// Ank repeat// Ank repeat// Myosin head (motor domain)
OCBBF3028001//Transmembrane region cyclic Nucleotide Gated Channel
PEBLM2001803//Vacuolar sorting protein 9 (VPS9) domain
PEBLM2005615//7 transmembrane receptor (rhodopsin family)// Herpesvirus glycoprotein M
PEBLM2006298//Kinesin motor domain
PLACE5000492//Viral RNA dependent RNA polymerase//
Phosphatidylinositol-specific phospholipase C, X domain//
Phosphatidylinositol-specific phospholipase C, X domain// C2 domain
PLACE6000055//WD domain, G-beta repeat// WD domain, G-beta repeat// WD domain, G-beta repeat
PLACE6001933//Receptor L domain
PLACE6003004//Signal peptidase I// Mandelate racemase / muconate lactonizing enzyme family
PLACE6010925//Ribosomal protein S11
PLACE6010936//7 transmembrane receptor (rhodopsin family)
PLACE6019600//Ras family
PLACE7000266//Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain
PLACE7002303//Homeobox domain
PLACE7003985//Prokaryotic molybdopterin oxidoreductases// short chain dehydrogenase
PLACE7004103//KH domain// KH domain// KH domain// KH domain// Dynamin central region// Domain of unknown function// KH domain// KH domain// KH domain// Small cytokines (intecrine/chemokine), interleukin-8 like// Fanconi anaemia group C protein// KH domain// KH domain// AIR carboxylase// KH domain
PLACE7004961//Dynamin GTPase effector domain
PLACE7005169//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
PLACE7006090//Glycosyl hydrolases family 31
PLACE7006498//ADP-ribosylation factor family// G-protein alpha subunit
PLACE7007379//Zinc carboxypeptidase
PLACE7007973//Matrix protein (MA), p15// Matrix protein (MA), p15// Gag P30 core shell protein// Zinc finger, CCHC class
PLACE7008136//Reverse transcriptase (RNA-dependent DNA polymerase)
PLACE7009563//Arginase family// MAGE family
PLACE7009757//Zinc finger, C2H2 type// Zinc finger, C2H2 type// Zinc finger, C2H2 type
PLACE7009936//RhoGAP domain
PLACE7011559//Ank repeat// Ank repeat// Ank repeat// Ank repeat// Ank repeat// Ank repeat// Integrin alpha cytoplasmic region
PLACE7012111//Reprolysin family propeptide// Reprolysin (M12B) family zinc metalloprotease// Keratin, high sulfur B2 protein// Disintegrin// EGF-like domain
PLACE7014247//Phosphatidylinositol 3- and 4-kinases
PLACE7016526//ATP-dependent protease La (LON) domain// NB-ARC domain// Adenylylsulfate kinase// IstB-like ATP binding protein// Shikimate kinase// Isopentenyl transferase// ATPases associated with various cellular activities (AAA)
PLACE7018304//PLAT/LH2 domain// PLAT/LH2 domain// PLAT/LH2 domain// PLAT/LH2 domain// PLAT/LH2 domain// PLAT/LH2 domain// PLAT/LH2 domain// PLAT/LH2 domain// PLAT/LH2 domain// PLAT/LH2 domain
PLACE7018512//Zinc finger, C2H2 type// Zinc finger, C2H2 type PROST2002078//RNA recognition motif. (a.k.a. RRM, RBD, or RNP domain)
PUAEN2000594//Poly-adenylate binding protein, unique domain.
PUAEN2000684//Geminivirus AL2 protein// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat
PUAEN2006639//WD domain, G-beta repeat// WD domain, G-beta repeat// WD domain, G-beta repeat// WD domain, G-beta repeat// WD domain, G-beta repeat// WD domain, G-beta repeat
SKMUS2008585//Dual specificity phosphatase, catalytic domain
SKMUS2009557//Hydroxyethylthiazole kinase family
SMINT2010753//TPR Domain// TPR Domain// TPR Domain// TPR Domain// PPR repeat// TPR Domain
SMINT2011406//Dynamin GTPase effector domain
SMINT2011509//DNA polymerase X family
SMINT2014721//E2 (early) protein, N terminal// Carnitate acyltransferase
SMINT2017964//PH domain
SPLEN2007689//PX domain// SH3 domain
SPLEN2012571//SCAN domain// KRAB box// Zinc finger, C2H2 type// Zinc finger, C2H2 type// Zinc finger, C2H2 type// Zinc finger, C2H2 type// TRAF-type zinc finger// Zinc finger, C2H2 type
SPLEN2022785//Polyomavirus coat protein
SPLEN2025012//Immunoglobulin domain// Immunoglobulin domain// Immunoglobulin domain// Immunoglobulin domain// Immunoglobulin domain
SPLEN2028417//Homeobox domain
SPLEN2036608//Zinc finger C-x8-C-x5-C-x3-H type (and similar).
STOMA2004663//Immunoglobulin domain
SYNOV2003326//TSC-22/dip/bun family
SYNOV2017179//Hepatitis C virus non-structural protein NS4a// TBC domain
SYNOV4003174//Phosphoribulokinase// Myosin head (motor domain)
SYNOV4009139//Hyaluronidase
SYNOV4009575//WD domain, G-beta repeat// Gram-negative pili assembly chaperone// WD domain, G-beta repeat// WD domain, G-beta repeat
T1ESE2000609//DNA polymerase (viral) C-terminal domain// G-patch domain// Double-stranded RNA binding motif
T1ESE2000904//KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
T1ESE2002665//Amino acid permease
TBAES2003917//Ank repeat// Ank repeat// Ank repeat// Ank repeat TBAES2007428//Scorpion short toxins// EGF-like domain// EGF-like domain
TESOP2002005//E7 protein, Early protein
TESTI2001364//Pyridine nucleotide-disulphide oxidoreductase// lactate/malate dehydrogenase
TESTI2005112//Respiratory-chain NADH dehydrogenase, 30 Kd subunit
TESTI2005564//EF hand
TESTI2006543//Collagen triple helix repeat (20 copies)
TESTI2007490//UDP-glucoronosyl and UDP-glucosyl transferases
TESTI2009739//Tropomyosins// Domain of unknown function
TESTI2011020//Keratin, high sulfur B2 protein
TESTI2018335//NADH-ubiquinone/plastoquinone oxidoreductase chain 4L// Transmembrane amino acid transporter protein// Amino acid permease
TESTI2018867//FF domain
TESTI2021112//Carbamoyl-phosphate synthase (CPSase)
TESTI2021654//Glycosyl hydrolases family 11
TESTI2022323//7 transmembrane receptor (rhodopsin family)
TESTI2023903//Ubiquitin family// Gram-negative pili assembly chaperone
TESTI2024267//Transmembrane amino acid transporter protein// Ion transport protein
TESTI2030901//Glutathione S-transferases.
TESTI2034913//Intermediate filament proteins// Intermediate filament proteins
TESTI2036285//Ubiquitin family// Ubiquitin family// Ubiquitin family
TESTI2036822//Integral membrane protein// AN1-like Zinc finger// DHHC zinc finger domain
TESTI2037877//Flavin containing amine oxidase
TESTI2040377//Phorbol esters/diacylglycerol binding domain (C1 domain)// Zinc finger, C3HC4 type (RING finger)// PHD-finger// Zinc finger present in dystrophin, CBP/p300// Zinc finger, C3HC4 type (RING finger)
TESTI2049041//TPR Domain// TPR Domain
TESTI2049062//short chain dehydrogenase
TESTI2052670//Immunoglobulin domain// Immunoglobulin domain// Immunoglobulin domain// Immunoglobulin domain// Immunoglobulin domain
TESTI4000370//Keratin, high sulfur B2 protein// Bacterial regulatory proteins, gntR family
TESTI4000621//SNF2 and others N-terminal domain// 6-O-methylguanine DNA methyltransferase// Rel homology domain (RHD).// Helicases conserved C-terminal domain
TESTI4001517//Intermediate filament proteins
TESTI4001569//Leucine Rich Repeat// Leucine Rich Repeat// Leucine Rich Repeat// Leucine Rich Repeat// Leucine Rich Repeat// KE2 family protein// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat// Leucine Rich Repeat
TESTI4001679//FYVE zinc finger// Zinc finger, C3HC4 type (RING finger)// PHD-finger
TESTI4002141//Keratin, high sulfur B2 protein
TESTI4002774//PH domain// Phosphoglycerate mutase family// Oxysterol-binding protein
TESTI4002799//RNA polymerase beta subunit// PHD-finger
TESTI4002868//Metallothionein
TESTI4003404//Ank repeat// Ank repeat// Ank repeat// PEP-utilizing enzymes
TESTI4003565//Inositol monophosphatase family
TESTI4003602//Translation initiation factor IF-3// Divalent cation transporter
TESTI4003703//Inositol monophosphatase family
TESTI4003796//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
TESTI4003944//Zinc finger, C2H2 type// Zinc finger, C2H2 type// Zinc finger, C2H2 type// Zinc finger, C2H2 type// Domain of unknown function// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type
TESTI4004031//Domain of unknown function
TESTI4004539//Integral membrane protein// Sodium Bile acid symporter family
TESTI4004695//Leptin
TESTI4005322//KRAB box// Fungal cellulose binding domain// Zinc finger, C2H2 type// Zinc finger, C2H2 type// Zinc finger, C2H2 type// Zinc finger, C2H2 type// Zinc finger, C2H2 type// TRAF-type zinc finger// Zinc finger, C2H2 type// Zinc finger, C2H2 type// Zinc finger, C2H2 type// Zinc finger, C2H2 type// Zinc finger, C2H2 type// Zinc finger, C2H2 type
TESTI4005399//Divalent cation transporter// Divalent cation transporter
TESTI4005470//Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
TESTI4005653//Collagen triple helix repeat (20 copies)
TESTI4007671//Thioredoxin
TESTI4007965//Adaptin N terminal region// Gamma-adaptin, C-terminus
TESTI4008305//Collagen triple helix repeat (20 copies)// Herpesvirus Glycoprotein B
TESTI4010544//Cytochrome C and Quinol oxidase polypeptide I// Sodium/hydrogen exchanger family// Ion transport protein// Cyclic nucleotide-binding domain
TESTI4010721//Ribonucleotide reductase
TESTI4010902//WD domain, G-beta repeat// WD domain, G-beta repeat// WD domain, G-beta repeat// WD domain, G-beta repeat// WD domain, G-beta repeat// WD domain, G-beta repeat
TESTI4011926//Gag P30 core shell protein
TESTI4012960//Influenza RNA-dependent RNA polymerase subunit PA// Reprolysin family propeptide// Leptin
TESTI4013474//Nuclear transition protein 2// Phorbol esters/diacylglycerol binding domain (C1 domain)// RhoGAP domain
TESTI4013742//Leucine Rich Repeat// Leucine Rich Repeat// Hantavirus nucleocapsid protein// Troponin// Formin Homology 2 Domain// Apolipoprotein A1/A4/E family
TESTI4014415//Ribosomal protein S8// Uncharacterized protein family// Domain of unknown function// Sodium/hydrogen exchanger family// Ion transport protein// Cyclic nucleotide-binding domain
TESTI4017854//Hepatitis C virus non-structural protein E2/NS1
TESTI4020342//WD domain, G-beta repeat// WD domain, G-beta repeat// WD domain, G-beta repeat// WD domain, G-beta repeat// WD domain, G-beta repeat// WD domain, G-beta repeat// Protein phosphatase 2A regulatory subunit PR55// WD domain, G-beta repeat
TESTI4020596//Calpain family cysteine protease// Calpain large subunit, domain III// C2 domain
TESTI4020819//C1q domain
TESTI4021129//GAF domain
TESTI4021197//C2 domain// PDZ domain (Also known as DHR or GLGF).// Regulator of G protein signaling domain// Regulator of G protein signaling domain
TESTI4021569//ABC transporter transmembrane region.
TESTI4022158//Immunoglobulin domain// Thrombospondin type 1 domain// Thrombospondin type 1 domain// ZU5 domain// Death domain
TESTI4023096//ABC 3 transport family// Amino acid permease// Cystatin domain
TESTI4024294//Chorion protein
TESTI4024494//Zinc finger, C3HC4 type (RING finger)// Peroxidase// Zinc finger, C3HC4 type (RING finger)// B-box zinc finger.// Scorpion short toxins
TESTI4026080//Nucleosome assembly protein (NAP)// Lipoate-protein ligase B// Asparaginase// ABC transporter
TESTI4028042//WD domain, G-beta repeat// WD domain, G-beta repeat// WD domain, G-beta repeat// Fibrillar collagen C-terminal domain
TESTI4028182//3'5'-cyclic nucleotide phosphodiesterase// Elongation factor Tu family
TESTI4029731//Ras family
TESTI4030864//Ribosomal protein L36
TESTI4031066//Lipoate-protein ligase B// KE2 family protein
TESTI4031173//Immunoglobulin domain// Immunoglobulin domain// Immunoglobulin domain// Immunoglobulin domain
TESTI4032128//Amyloid A4 extracellular domain// Kunitz/Bovine pancreatic trypsin inhibitor domain
TESTI4032913//Zinc finger present in dystrophin, CBP/p300// Ank repeat// Ank repeat// Ank repeat// Ank repeat// Ank repeat// Ank repeat// Ank repeat
TESTI4033177//K-box region// Dual specificity phosphatase, catalytic domain// Penicillin amidase
TESTI4035898//Kelch motif// Kelch motif// Kelch motif
TESTI4036048//PX domain
TESTI4037949//BTB/POZ domain// Kelch motif// Kelch motif// Kelch motif// Kelch motif// Glycophorin A// Kelch motif// Kelch motif
TESTI4039451//Adaptin N terminal region
TESTI4039904//Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type
TESTI4040559//Transmembrane region cyclic Nucleotide Gated Channel// Cyclic nucleotide-binding domain
TESTI4040598//Cytochrome P450
TESTI4041049//Calponin homology (CH) domain
TESTI4041482//Archaeal ATPase
TESTI4041984//EGF-like domain// EGF-like domain// EGF-like domain// EGF-like domain// EB module// EGF-like domain// EGF-like domain// EGF-like domain// EGF-like domain// TB domain// EGF-like domain// EGF-like domain// TB domain// EGF-like domain// EGF-like domain
TESTI4043166//Formin Homology 2 Domain
TESTI4046073//Dockerin domain type I// RhoGAP domain
TESTI4046873//TPR Domain// TPR Domain// TPR Domain// TPR Domain
TESTI4047328//von Willebrand factor type D domain// Trypsin Inhibitor like cysteine rich domain// Chitin binding domain// von Willebrand factor type D domain// Trypsin Inhibitor like cysteine rich domain// Metallothionein
TESTI4047569//Keratin, high sulfur B2 protein
TESTI4047808//Eukaryotic protein kinase domain// Eukaryotic protein kinase domain
TESTI4049786//Mur ligase family// Hexokinase// Hexokinase
TESTI4049899//Scavenger receptor cysteine-rich domain// CUB domain
TESTI4051015//Major intrinsic protein// Major intrinsic protein
TESTI4051054//B-box zinc finger.
TESTI4051424//Immunoglobulin domain// Fibronectin type III domain// Fibronectin type III domain// Fibronectin type III domain// Fibronectin type III domain// Fibronectin type III domain// Aldo/keto reductase family// Immunoglobulin domain// Thioredoxin// Immunoglobulin domain// Immunoglobulin domain
TESTI4051865//PDZ domain (Also known as DHR or GLGF).// Collagen triple helix repeat (20 copies)// PDZ domain (Also known as DHR or GLGF).// PDZ domain (Also known as DHR or GLGF).// SH3 domain// Ribosomal protein L15 amino terminal region// Guanylate kinase
TESTI4052219//E1-E2 ATPase// E1-E2 ATPase// GTP cyclohydrolase II
TESTI4052598//Lectin C-type domain
TESTI4052775//Zinc finger, C2H2 type// Zinc finger, C2H2 type// Zinc finger, C2H2 type// TRAF-type zinc finger// Zinc finger, C2H2 type
THYMU2008207//Zinc finger, C3HC4 type (RING finger)
THYMU2038199//Zinc finger, C2H2 type// Zinc finger, C2H2 type
THYMU3000390//Ribonuclease T2 family
THYMU3002825//FliP family// Glycosyl hydrolase family 47
THYMU3003007//TPR Domain
THYMU3008105//Zinc finger, C2H2 type// FYVE zinc finger// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type
THYMU3012402//Armadillo/beta-catenin-like repeats// Armadillo/beta-catenin-like repeats// Armadillo/beta-catenin-like repeats// Armadillo/beta-catenin-like repeats// Armadillo/beta-catenin-like repeats// Armadillo/beta-catenin-like repeats
THYMU3012983//Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type
THYMU3013785//RasGEF domain
THYMU3014173//Class I Histocompatibility antigen, domains alpha 1 and 2
THYMU3014372//Integrase Zinc binding domain// Aldo/keto reductase family// MCM2/3/5 family
THYMU3015042//Polyomavirus coat protein
THYMU3015571//Chaperonins 10 Kd subunit
THYMU3015647//Domain of unknown function// Latrophilin/CL-1-like GPS domain// CbiM// 7 transmembrane receptor (Secretin family)
THYMU3016518//Immunoglobulin domain// Immunoglobulin domain// Immunoglobulin domain// Immunoglobulin domain
THYMU3017761//Gag P30 core shell protein
THYMU3019476//Matrix protein (MA), p15
THYMU3020221//Immunoglobulin domain// Fibronectin type II domain// Immunoglobulin domain// Immunoglobulin domain// Immunoglobulin domain
THYMU3021404//Sugar (and other) transporter
THYMU3021586//Helix-loop-helix DNA-binding domain
THYMU3021755//HCO3- transporter family
THYMU3022434//Zinc finger, C2H2 type// MOZ/SAS family
THYMU3023400//Transmembrane amino acid transporter protein// Large-conductance mechanosensitive channel, MscL// CbiM// DNA gyrase/topoisomerase IV, subunit A
THYMU3025118//Immunoglobulin domain// Immunoglobulin domain// Immunoglobulin domain// Immunoglobulin domain// Immunoglobulin domain
THYMU3025642//von Hippel-Lindau disease tumor suppressor protein
THYMU3025683//Heavy-metal-associated domain// Vacuolar sorting protein 9 (VPS9) domain// chorismate binding enzyme// Ras association (RalGDS/AF-6) domain
THYMU3026000//KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type
THYMU3026306//FliP family// Glycosyl hydrolase family 47
THYMU3026479//Glutathione S-transferases.
THYMU3026532//Plexin repeat// Integrins, beta chain
THYMU3030072//KRAB box// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// DnaJ central domain (4 repeats)// Zinc finger, C2H2 type// Zinc finger, C2H2 type// Zinc finger, C2H2 type// Zinc finger, C2H2 type// Zinc finger, C2H2 type
THYMU3030752//K+ channel tetramerisation domain
THYMU3031878//PAP2 superfamily
THYMU3032798//Eukaryotic protein kinase domain
THYMU3033626//NOL1/NOP2/sun family
THYMU3033649//Immunoreceptor tyrosine-based activation motif
THYMU3034671//Histone deacetylase family
THYMU3036953//Trypsin
THYMU3037617//FYVE zinc finger// AN1-like Zinc finger
THYMU3037772//Immunoglobulin domain// Immunoglobulin domain// Immunoglobulin domain// Immunoglobulin domain
THYMU3038158//Collagen triple helix repeat (20 copies)// Collagen triple helix repeat (20 copies)// Collagen triple helix repeat (20 copies)
THYMU3040126//Metallothionein
THYMU3040172//Scavenger receptor cysteine-rich domain// Scavenger receptor cysteine-rich domain// Scavenger receptor cysteine-rich domain
THYMU3040746//Immunoglobulin domain// Immunoglobulin domain// Immunoglobulin domain// Immunoglobulin domain
THYMU3040829//RNA recognition motif. (a.k.a. RRM, RBD, or RNP domain)
THYMU3041428//DEAD/DEAH box helicase// Helicases conserved C-terminal domain
THYMU3043200//Zinc finger, C2H2 type
THYMU3044075//BTB/POZ domain// K+ channel tetramerisation domain
THYMU3045704//Prokaryotic transcription elongation factor, GreA/GreB
THYMU3046360//F-box domain.
THYMU3047115//Site-specific recombinases// E1-E2 ATPase// Na+/K+ ATPase C-terminus
THYMU3047891//ThiF family// short chain dehydrogenase
TKIDN2003396//Zinc finger, CCHC class
TKIDN2011051//Keratin, high sulfur B2 protein
TKIDN2011160//Thrombospondin type 1 domain
TLIVE2001616//Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type
TLIVE2007736//PDZ domain (Also known as DHR or GLGF).
TLUNG2000654//bZIP transcription factor// Intermediate filament proteins
TLUNG2001445//Immunoglobulin domain// Immunoglobulin domain// Immunoglobulin domain// Immunoglobulin domain// Immunoglobulin domain
TLUNG2001600//Immunoglobulin domain// Immunoglobulin domain// Immunoglobulin domain// Immunoglobulin domain
TRACH2022113//dUTPase
TRACH2024730//Phosphoribosyl-ATP pyrophosphohydrolase// Death domain
TRACH3002752//PHD-finger// Zn-finger in ubiquitin-hydrolases and other proteins
TRACH3002890//Collagen triple helix repeat (20 copies)
TRACH3003037//Phosphotriesterase family// RhoGEF domain// PH domain// Thaumatin family// GATA zinc finger// FYVE zinc finger// PH domain
TRACH3003357//Interleukin-6/G-CSF/MGF family// SH3 domain// SH3 domain// SH3 domain// SH3 domain
TRACH3003458//Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type
TRACH3004113//Ribosomal protein L37e// FYVE zinc finger// Alpha-2-macroglobulin family// Interleukin 4
TRACH3004412//Zinc finger, CCHC class// Clusterin
TRACH3004424//Fungal cellulose binding domain// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type
TRACH3004747//Sugar (and other) transporter
TRACH3005173//PH domain
TRACH3005274//Trans-activation protein X// Glycosyl transferases group 1
TRACH3005699//SEA domain// SEA domain
TRACH3006379//Intermediate filament proteins
TRACH3006800//Fungalysin metallopeptidase (M36)// SEA domain// SEA domain// SEA domain
TRACH3007689//Ank repeat// Ank repeat// Ank repeat// TPR Domain// TPR Domain// TPR Domain
TRACH3009008//Pyridine nucleotide-disulphide oxidoreductase
TRACH3009061//Papain family cysteine protease// ABC transporter
TRACH3010079//Mov34/MPN/PAD-1 family
TRACH3010167//Zinc finger, C2H2 type// Zinc finger, C2H2 type// Zinc finger, C2H2 type
TRACH3011082//Immunoglobulin domain
TRACH3011184//Immunoglobulin domain
TRACH3011313//Aminotransferase class IV
TRACH3011538//RhoGEF domain// PH domain
TRACH3012106//FERM domain (Band 4.1 family)
TRACH3012460//Collagen triple helix repeat (20 copies)
TRACH3012659//Immunoglobulin domain
TRACH3015346//Uncharacterized protein family UPF0004// Uncharacterized protein family UPF0004
TRACH3015354//Lectin (probable mannose binding)
TRACH3015951//BTB/POZ domain// Zinc finger, C2H2 type// Zinc finger, C3HC4 type (RING finger)// Zinc finger, C2H2 type// Zinc finger, C2H2 type// Zinc finger, C2H2 type// PHD-finger// Zinc finger, C2H2 type// Zinc finger, C2H2 type// Zinc finger, C2H2 type// Zinc finger, C2H2 type// Zinc finger, C2H2 type// Zinc finger, C2H2 type
TRACH3016455//Calpain family cysteine protease// Calpain large subunit, domain III
TRACH3016805//Ank repeat// Ank repeat// Ank repeat// Ank repeat// Ank repeat// Ank repeat// Ank repeat
TRACH3017409//RNA dependent RNA polymerase
TRACH3018108//FKBP-type peptidyl-prolyl cis-trans isomerases// TPR Domain// TPR Domain
TRACH3018261//Glycosyl hydrolase family 47
TRACH3018519//WD domain, G-beta repeat
TRACH3018524//Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain// Fibronectin type III domain
TRACH3018606//SAM domain (Sterile alpha motif)
TRACH3018907//Immunoglobulin domain// Immunoglobulin domain// Immunoglobulin domain// Immunoglobulin domain// Immunoglobulin domain
TRACH3019058//Immunoglobulin domain// Immunoglobulin domain
TRACH3019370//ABC transporter
TRACH3019621//Glycosyl transferase
TRACH3019807//Immunoglobulin domain
TRACH3020605//RhoGEF domain// SH3 domain// SH3 domain
TRACH3020769//Ezrin/radixin/moesin family// Myosin tail
TRACH3020930//Immunoglobulin domain// Immunoglobulin domain// Immunoglobulin domain// Immunoglobulin domain
TRACH3021023//Immunoglobulin domain// Immunoglobulin domain// Immunoglobulin domain// Immunoglobulin domain
TRACH3021544//Ubiquitin carboxyl-terminal hydrolases family 2// Ubiquitin carboxyl-terminal hydrolase family 2
TRACH3021778//RNA recognition motif. (a.k.a. RRM, RBD, or RNP domain)
TRACH3022109//DHHC zinc finger domain
TRACH3022296//DnaJ domain
TRACH3022758//EF hand// EF hand
TRACH3023203//Flavivirus polyprotein propeptide
TRACH3023373//EF hand// EF hand// Reovirus viral attachment protein sigma 1// Peptide hormone
TRACH3023516//FKBP-type peptidyl-prolyl cis-trans isomerases// FKBP-type peptidyl-prolyl cis-trans isomerases// FKBP-type peptidyl-prolyl cis-trans isomerases// FKBP-type peptidyl-prolyl cis-trans isomerases// EF hand// EF hand
TRACH3024020//Eukaryotic protein kinase domain
TRACH3024081//E1-E2 ATPase
TRACH3024342//Metallo-beta-lactamase superfamily
TRACH3024512//Chitin synthase// von Willebrand factor type A domain
TRACH3025316//Armadillo/beta-catenin-like repeats// Armadillo/beta-catenin-like repeats// Armadillo/beta-catenin-like repeats// Armadillo/beta-catenin-like repeats// Beta-lactamase// Armadillo/beta-catenin-like repeats
TRACH3026299//Immunoglobulin domain// Immunoglobulin domain// Immunoglobulin domain// Immunoglobulin domain
TRACH3026303//Rhodanese-like domain// Integrase Zinc binding domain// Integrase Zinc binding domain
TRACH3026650//IPT/TIG domain// IPT/TIG domain// IPT/TIG domain// C1q domain// EF hand// EF hand// Growth-Arrest-Specific Protein 2 Domain// Potyvirus P1 protease
TRACH3027229//Acyltransferase
TRACH3027701//Thermophilic metalloprotease (M29)
TRACH3028180//SCAN domain// Zinc finger, C2H2 type// Zinc finger, C2H2 type// Transcription factor S-II (TFIIS)// Zinc finger, C2H2 type// Zinc finger, C2H2 type// PHD-finger// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type
TRACH3028441//Immunoglobulin domain// Immunoglobulin domain// Immunoglobulin domain// Immunoglobulin domain
TRACH3028837//Calponin homology (CH) domain
TRACH3028855//R3H domain// Uncharacterized protein family UPF0024
TRACH3029329//Fes/CIP4 homology domain// Hr1 repeat motif// ENV polyprotein (coat polyprotein)// ATP synthase Alpha chain, C terminal
TRACH3029462//Spectrin repeat// Spectrin repeat// Protein of unknown function// Spectrin repeat// Bacterial flagellin N-terminus// Spectrin repeat// Spectrin repeat// Spectrin repeat// Caulimovirus movement protein// Spectrin repeat// Spectrin repeat// Spectrin repeat// UvrB/uvrC motif// Spectrin repeat// Spectrin repeat// Spectrin repeat// KE2 family protein
TRACH3029670//Immunoglobulin domain// Immunoglobulin domain// Immunoglobulin domain// Immunoglobulin domain
TRACH3030176//Beige/BEACH domain// WD domain, G-beta repeat// WD domain, G-beta repeat
TRACH3030855//Serpins (serine protease inhibitors)
TRACH3031316//Immunoglobulin domain
TRACH3031660//Regulatory subunit of type II PKA R-subunit// Cyclic nucleotide-binding domain// Cyclic nucleotide-binding domain
TRACH3031678//Natural resistance-associated macrophage protein
TRACH3032150//Calcium channel extracellular region// Pyridoxal-dependent decarboxylase conserved domain
TRACH3032570//PDZ domain (Also known as DHR or GLGF).// PDZ domain (Also known as DHR or GLGF).
TRACH3034680//Immunoglobulin domain// Immunoglobulin domain// Immunoglobulin domain// Immunoglobulin domain
TRACH3036103//Immunoglobulin domain
TRACH3036750//BTK motif// Zinc finger, C2H2 type// Zinc finger, C2H2 type// Zinc finger, C2H2 type// Zinc finger, C2H2 type// Zinc finger, C2H2 type// Zinc finger, C2H2 type// GATA zinc finger// Zinc finger, C2H2 type// Zinc finger, C2H2 type// Zinc finger, C2H2 type// Zinc finger, C2H2 type// Ribonuclease T2 family
TRACH3037505//Immunoglobulin domain// Immunoglobulin domain// Immunoglobulin domain// Immunoglobulin domain
TRACH3038399//Eukaryotic protein kinase domain// Eukaryotic protein kinase domain
TSTOM2001571//Eukaryotic protein kinase domain
TUTER2001433//Immunoglobulin domain// Immunoglobulin domain// Immunoglobulin domain// Immunoglobulin domain// Immunoglobulin domain
UTERU2016669//Helix-hairpin-helix motif.// Helix-hairpin-helix motif.
UTERU2024042//Eukaryotic protein kinase domain
UTERU2037423//Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type
UTERU3001029//WD domain, G-beta repeat// WD domain, G-beta repeat// WD domain, G-beta repeat// WD domain, G-beta repeat// WD domain, G-beta repeat// WD domain, G-beta repeat
UTERU3001394//EGF-like domain
UTERU3001946//Fungalysin metallopeptidase (M36)// SEA domain// SEA domain
UTERU3006720//RhoGAP domain
UTERU3009775//PDZ domain (Also known as DHR or GLGF).
UTERU3010409//Immunoglobulin domain
UTERU3010604//Indoleamine 2,3-dioxygenase
UTERU3010919//Eukaryotic protein kinase domain// Regulator of chromosome condensation (RCC1)// Regulator of chromosome condensation (RCC1)// Regulator of chromosome condensation (RCC1)
UTERU3011398//von Willebrand factor type A domain
UTERU3011558//GTPase of unknown function
UTERU3011579//Plant PEC family metallothionein
UTERU3011837//Fibronectin type III domain
UTERU3012293//Zinc finger, C2H2 type// Zinc finger, C2H2 type//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type
UTERU3012414//ADP-ribosylation factor family// G-protein alpha subunit
UTERU3015011//LIM domain containing proteins// LIM domain containing proteins// LIM domain containing proteins// Phorbol esters/diacylglycerol binding domain (C1 domain)// PHD-finger//
LIM domain containing proteins
UTERU3015299//NADH ubiquinone oxidoreductase, 20 Kd subunit
UTERU3015647//Immunoglobulin domain
UTERU3016070//SCAN domain// Zinc finger, C2H2 type// Zinc finger, C2H2 type// Zinc finger, C2H2 type// Zinc finger, C2H2 type// Transcription factor S-II (TFIIS)// Zinc finger, C2H2 type//
Zinc finger, C2H2 type// Zinc finger, C2H2 type// Zinc finger, C2H2 type
UTERU3016273//Integral membrane protein
UTERU3017441//Armadillo/beta-catenin-like repeats// Armadillo/beta-catenin-like repeats// Armadillo/beta-catenin-like repeats// D-isomer specific 2-hydroxyacid dehydrogenases// Armadillo/beta-catenin-like repeats// Armadillo/beta-catenin-like repeats
UTERU3017626//Ion transport protein// Transmembrane region cyclic Nucleotide Gated Channel
UTERU3017995//UBX domain
UTERU3018172//Bacterial regulatory proteins, crp family
UTERU3018255//Thrombospondin type 1 domain
UTERU3019708//Viral (Superfamily 1) RNA helicase
UTERU3020090//DNA polymerase (viral) C-terminal domain
UTERU3021231//PX domain
UTERU3021850//Thrombospondin type 1 domain// DnaJ central domain (4 repeats)
UTERU3022168//DNA polymerase family B// C2 domain// C2 domain// C2 domain// C2 domain// C2 domain
UTERU3022588//bZIP transcription factor
UTERU3023141//Double-stranded RNA binding motif

Deduced amino acid sequences of following 29 clones were also detected to have functional domains with Pfam.
ADRGL2011190// GAF domain// Transposase, Mutator family// 3'5'-cyclic nucleotide phosphodiesterase
BRACE3002184// KRAB box// Zinc finger, C2H2 type// Zinc finger, C2H2 type// Zinc finger, C2H2 type// Zinc finger, C2H2 type//
Zinc finger, C2H2 type// Zinc finger, C2H2 type// Transcription factor S-II (TFIIS)// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
BRACE3026993// TSC-22/dip/bun family
BRACE3046450// PDZ domain (Also known as DHR or GLGF).// PDZ domain (Also known as DHR or GLGF).// PDZ domain (Also known as DHR or GLGF). 1// PDZ domain (Also known as DHR or GLGF).
BRAMY3008096// Eukaryotic protein kinase domain
BRAWH3013197// Receptor family ligand binding region// 7 transmembrane receptor (metabotropic glutamate family)
BRAWH3028645// Latrophilin/CL-1-like GPS domain// FecCD transport family// 7 transmembrane receptor (Secretin family)
BRAWH3046240// 7 transmembrane receptor (rhodopsin family)
BRCAN2019772// Zinc finger, C4 type (two domains)// Ligand-binding domain of nuclear hormone receptor
CTONG2002832// KRAB box// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc carboxypeptidase// Zinc finger, C2H2 type// Zinc finger, C2H2 type// Zinc finger, C2H2 type// Zinc finger, C2H2 type// Zinc finger, C2H2 type// Zinc finger, C2H2 type// Zinc finger, C2H2 type
CTONG2003764// Phorbol esters/diacylglycerol binding domain (C1 domain)
PROST2010326// Latrophilin/CL-1-like GPS domain// Cytochrome C assembly protein// 7 transmembrane receptor (Secretin family)
TBAES2004105// Thrombospondin type 1 domain
TBAES2007379// EGF-like domain// EGF-like domain// Trypsin Inhibitor like cysteine rich domain// EGF-like domain// EGF-like domain// Keratin, high sulfur B2 protein// EGF-like domain// EGF-like domain// Granulins// EGF-like domain// EGF-like domain// EGF-like domain
TBAES2007481// Cyclin
TBAES2008133// PDZ domain (Also known as DHR or GLGF).
TESTI2043585// 7 transmembrane receptor (rhodopsin family)
TESTI2046536// Sperm histone P2
TESTI4002988// KRAB box// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// DM DNA binding domain//
Zinc finger, C2H2 type// Transcription factor S-II (TFIIS)//
Zinc finger, C2H2 type// Putative zinc finger in N-recognin//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type
TESTI4005158// Integrins, beta chain// Notch (DSL) domain// Amiloride-sensitive sodium channel
TESTI4005500// KRAB box// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// DM DNA binding domain//
Zinc finger, C2H2 type// Transcription factor S-II (TFIIS)//
Zinc finger, C2H2 type// Putative zinc finger in N-recognin//
Zinc finger, C2H2 type// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type
TESTI4052089// Eukaryotic protein kinase domain
THYMU3024602// EGF-like domain// EB module// EGF-like domain// Metallothionein// EGF-like domain// EGF-like domain// Keratin, high sulfur B2 protein// EGF-like domain// Latrophilin/CL-1-like GPS domain// Leishmanolysin//7 transmembrane receptor (Secretin family)
THYMU3044175// S-adenosyl-L-homocysteine hydrolase
THYMU3046350// Sodium:neurotransmitter symporter family// Sulfate transporter family// Sodium:neurotransmitter symporter family// Sodium:neurotransmitter symporter family
TRACH1000193// Metallothionein family 5// Zinc finger, C4 type (two domains)// Ligand-binding domain of nuclear hormone receptor
TRACH3019290// KRAB box// Zinc finger, C2H2 type// Zinc finger, C2H2 type// Zinc finger// Zinc finger, C2H2 type// Zinc finger, C2H2 type// Zinc finger, C2H2 type// Zinc finger, C2H2 type//
Zinc finger, C2H2 type// Zinc finger, C2H2 type// Zinc finger, C2H2 type// Zinc finger, C2H2 type// Transcription factor S-II (TFIIS)// Zinc finger, C2H2 type// PHD-finger// Zinc finger, C2H2 type
TRACH3021066// Furin-like cysteine rich region// Eukaryotic protein kinase domain
UTERU2036507// Thrombospondin type 1 domain// NTR/C345C module

### EXAMPLE 6

### Functional categorization based on homology search of the full-length nucleotide sequences and deduced amino acid sequences

The functional prediction and categorization of the proteins encoded by the clones were carried out based on the result of homology search of the databases of Swiss-Prot, nr, and RefSeq (see the Homology Search Result Data) for the full-length nucleotide sequences and deduced amino acid sequences.

A clone predicted to belong to the category of secretory protein/membrane protein means a clone having hit data with some annotation, such as growth factor, cytokine, hormone, signal, transmembrane, membrane, extracellular matrix, receptor, G-protein coupled receptor, ionic channel, voltage-gated channel, calcium channel, cell adhesion, collagen, and connective tissue, suggesting that it is a secretory or membrane protein, or means a clone in which the presence of nucleotide sequence encoding a signal sequence or transmembrane domain was suggested by the results of PSORT and SOSUI analyses for deduced ORF.

A clone predicted to belong to the category of glycoprotein-related protein means a clone having hit data with some annotation, such as glycoprotein, suggesting that the clone encodes a glycoprotein-related protein.

A clone predicted to belong to the category of signal transduction-related protein means a clone having hit data with some annotation, such as serine/threonine-protein kinase, tyrosine-protein kinase, SH3 domain, and SH2 domain, suggesting that the clone encodes a signal transduction-related protein.

A clone predicted to belong to the category of transcription-related protein means a clone having hit data with some annotation, such as transcription regulation, zinc finger, and homeobox, suggesting that the clone encodes a transcription-related protein.

A clone predicted to belong to the category of disease-related protein means a clone having hit data with some annotation, such as disease mutation and syndrome, suggesting that the clone encodes a disease-related protein, or means a clone whose full-length nucleotide sequence has hit data for Swiss-Prot, nr, or RefSeq, where the hit data corresponds to genes or proteins which have been deposited in the Online Mendelian Inheritance in Man (OMIM) (http://www.ncbi.nlm.nih.gov/Omim/), which is the human gene and disease database.

A clone predicted to belong to the category of enzyme and/or metabolism-related protein means a clone having hit data with some annotation, such as metabolism, oxidoreductase, and E. C. No. (Enzyme commission number), suggesting that the clone encodes an enzyme and/or metabolism-related protein.

A clone predicted to belong to the category of cell division and/or cell proliferation-related protein means a clone having hit data with some annotation, such as cell division, cell cycle, mitosis, chromosomal protein, cell growth, and apoptosis, suggesting that the clone encodes a cell division and/or cell proliferation-related protein.

A clone predicted to belong to the category of cytoskeleton-related protein means a clone having hit data with some annotation, such as structural protein, cytoskeleton, actin-binding, and microtubles, suggesting that the clone encodes a cytoskeleton-related protein.

A clone which is predicted to belong to the category of nuclear protein and/or RNA synthesis-related protein means a clone having hit data with some annotation, such as nuclear protein, RNA splicing, RNA processing, RNA helicase, and polyadenylation, suggesting that the clone encodes a nuclear protein and/or RNA synthesis-related protein.

A clone predicted to belong to the category of protein synthesis and/or transport-related protein means a clone having hit data with some annotation, such as translation regulation, protein biosynthesis, amino-acid biosynthesis, ribosomal protein, protein transport, and signal recognition particle, suggesting that the clone encodes a protein synthesis and/or transport-related protein.

A clone predicted to belong to the category of cellular defense-related protein means a clone having hit data with some annotation, such as heat shock, DNA repair, and DNA damage, suggesting that the clone encodes a cellular defense-related protein.

A clone predicted to belong to the category of development and/or differentiation-related proteins means a clone having hit data with some annotation, such as developmental protein, suggesting that the clone encodes a development and/or differentiation-related protein.

A clone predicted to belong to the category of DNA-binding and/or RNA-binding protein means a clone having hit data with some annotation, such as DNA-binding, RNA-binding, etc.

A clone predicted to belong to the category of ATP-binding and/or GTP-binding protein means a clone having hit data with some annotation, such as ATP-binding, GTP-binding, etc.

In this functional categorization, when a single clone corresponded to multiple categories of those shown above, the clone was assigned to the multiple categories. However, the function of a protein is not restricted to the functional categories in this classification, and there is the possibility that other functions are newly assigned to the protein.

The clones predicted to belong to the category of secretory protein and/or membrane protein are the following 551 clones.
3NB692004045, ADIPS2000069, ADRGL2010315, ASTRO2015162, BLADE2001031, BLADE2002744, BLADE2007744, BRACE2003628, BRACE2012528, BRACE2013126, BRACE2017397, BRACE2017580, BRACE2017992, BRACE2023633, BRACE2030039, BRACE2035191, BRACE3001403_{;} BRACE3001973, BRACE3002264, BRACE3002756, BRACE3004767, BRACE3004981, BRACE3007869, BRACE3009392, BRACE3013874, BRACE3013986, BRACE3014523, BRACE3015898, BRACE3018083, BRACE3021517, BRACE3021805, BRACE3022051, BRACE3024379, BRACE3024444, BRACE3024497, BRACE3024879, BRACE3026345, BRACE3026456, BRACE3026802, BRACE3028360, BRACE3029021, BRACE3030538, BRACE3031372, BRACE3031579, BRACE3031728, BRACE3032385, BRACE3032537, BRACE3032980, BRACE3033525, BRACE3034964, BRACE3034993, BRACE3037637, BRACE3037803, BRACE3038570, BRACE3039358, BRACE3039378, BRACE3040644, BRACE3040863, BRACE3042326, BRACE3042409, BRACE3042432, BRACE3044090, BRACE3046049, BRACE3046466, BRACE3048565, BRACE3050504, BRACE3051144, BRACE3051621, BRACE3052486, BRALZ2010842, BRALZ2011337, BRALZ2013690, BRAMY2015516, BRAMY2021098, BRAMY2025495, BRAMY2037609, BRAMY2041507, BRAMY2044686, BRAMY2046537, BRAMY3002886, BRAMY3004126, BRAMY3007449, BRAMY3009556, BRAMY3009904, BRAMY3010654, BRAMY3010902, BRAMY3015549, BRAMY3016829, BRAMY3018248, BRAWH2000256, BRAWH2010364, BRAWH2011812, BRAWH2011958, BRAWH2012866, BRAWH2014053, BRAWH2016209, BRAWH2016305, BRAWH3001053, BRAWH3001783, BRAWH3001833, BRAWH3003573, BRAWH3005892, BRAWH3008867, BRAWH3010461, BRAWH3010657, BRAWH3011907, BRAWH3012005, BRAWH3012662, BRAWH3012779, BRAWH3013049, BRAWH3014609, BRAWH3015175, BRAWH3016123, BRAWH3017259, BRAWH3018063, BRAWH3018548, BRAWH3018969, BRAWH3019529, BRAWH3019820, BRAWH3020200, BRAWH3020884, BRAWH3021012, BRAWH3021641, BRAWH3022347, BRAWH3023156, BRAWH3023274, BRAWH3023415, BRAWH3023421, BRAWH3024186, BRAWH3024242, BRAWH3027574, BRAWH3027880, BRAWH3028223, BRAWH3028754, BRAWH3029806, BRAWH3030810, BRAWH3032298, BRAWH3034114, BRAWH3034134, BRAWH3035914, BRAWH3036270, BRAWH3038055, BRAWH3038324, BRAWH3040711, BRAWH3040900, BRAWH3042132, BRAWH3042772, BRAWH3042996, BRAWH3043498, BRAWH3043623, BRAWH3044151, BRAWH3044676, BRAWH3046196, BRAWH3047063, BRAWH3048374, BRAWH3048724, BRAWH3049068, BRAWH3049544, BRCAN2002662, BRCAN2003269, BRCAN2003814, BRCAN2006051, BRCAN2006955, BRCAN2015402, BRCAN2018269, BRCAN2019653, BRCAN2019907, BRCAN2020234, BRCAN2020412, BRCAN2020972, BRCAN2021325, BRCAN2022126, BRCOC2006164, BRCOC2006639, BRCOC2009638, BRHIP2006921, BRHIP2020930, BRHIP2021929, BRHIP3000859, BRHIP3001878, BRHIP3002000, BRHIP3002124, BRHIP3003063, BRHIP3003306, BRHIP3003395, BRHIP3004774, BRHIP3005801, BRHIP3005944, BRHIP3006950, BRHIP3007195, BRHIP3007424, BRHIP3007960, BRHIP3008320, BRHIP3010289, BRHIP3011269, BRHIP3011831, BRHIP3012185, BRHIP3012357, BRHIP3012997, BRHIP3013078, BRHIP3016032, BRHIP3017146, BRHIP3017558, BRHIP3019956, BRHIP3020733, BRHIP3021019, BRHIP3025795, BRHIP3025844, BRHIP3027160, BRHIP3027191, BRHIP3028742, BRHIP3029530, BRHIP3030230, BRHIP3031733, BRHIP3035222, BRHIP3035754, BRHIP3036715, BRHIP3036936, BRHIP3037810, BRHIP3039430, BRHIP3039509, BRSSN2004710, BRSSN2018218, BRSTN2010089, BRSTN2011688, BRSTN2011899, BRSTN2011961, BRTHA2000969, BRTHA2003759, BRTHA2012189, BRTHA2014647, BRTHA2018304, BRTHA2019726, BRTHA2019743, BRTHA2020400, BRTHA2020566, BRTHA2020721, BRTHA2020781, BRTHA2021212, BRTHA2021440, BRTHA2021450, BRTHA2022914, BRTHA2022968, BRTHA2023437, BRTHA2026311, BRTHA2027250, BRTHA2030036, BRTHA2031917, BRTHA2033155, BRTHA2033320, BRTHA2033469, BRTHA2033683, BRTHA2036055, BRTHA2036295, BRTHA3003225, BRTHA3006593, BRTHA3010135, BRTHA3010540, BRTHA3010717, BRTHA3011194, BRTHA3011998, BRTHA3012265, BRTHA3013882, BRTHA3014835, BRTHA3016616, BRTHA3018623, BRTHA3026161, BRTHA3027820, BRTHA3028505, CHONS2001287, CHONS2001797, CHONS2002419, COLON2004351, COLON2005623, COLON2005735, CTONG2008989, CTONG2020582, CTONG2027150, CTONG3001605, CTONG3002588, CTONG3008223, FCBBF3012443, FEBRA2023498, FEBRA2026977, FEHRT2002708, FEKID2002231, FEKID2002493, FELNG2000720, FELNG2001706, HCHON2009766, HSYRA2004550, JCMLC1000159, JCMLC2000273, JCMLC2002095, JCMLC2002751, KIDNE2004531, KIDNE2015987, KIDNE2017153, LYMPB1000158, LYMPB2002236, LYMPB2002458, LYMPB2002478, MESAN2014624, NETRP2004090, NETRP2004434, NETRP2005282, NETRP2005849, NETRP2008582, NT2RI3001967, NT2RI3005861, NT2RI3005923, NT2RI3009524, NT2RP7019682, NT2RP8001605, NT2RP8003787, NT2RP8008057, OCBBF2000831, OCBBF2004478, OCBBF2007039, OCBBF2009536, OCBBF2018229, OCBBF2018618, OCBBF2036019, OCBBF3003745, OCBBF3007704, OCBBF3021502, OCBBF3022123, OCBBF3022576, OCBBF3023175, OCBBF3023993, OCBBF3025475, OCBBF3025887, OCBBF3026979, OCBBF3028001, PEBLM2003935, PEBLM2005615, PLACE5000522, PLACE6000012, PLACE6010936, PLACE6019674, PLACE7000266, PLACE7000707, PLACE7001759, PLACE7003639, PLACE7006090, PLACE7006498, PLACE7008136, PLACE7011269, PLACE7012111, PLACE7016321, PLACE7016454, PUAEN2000684, SMINT2003641, SPLEN2011252, SPLEN2025012, SPLEN2031004, SPLEN2034551, SPLEN2035615, SPLEN2042051, STOMA2004663, SYNOV4009139, T1ESE2002665, TBAES2005361, TBAES2007428, TESOP2008556, TESTI2003768, TESTI2007490, TESTI2018335, TESTI2022323, TESTI2024267, TESTI2028613, TESTI2036822, TESTI2037085, TESTI2037657, TESTI2037877, TESTI2046188, TESTI2049041, TESTI2052670, TESTI4001037, TESTI4002072, TESTI4002889, TESTI4003602, TESTI4004539, TESTI4004653, TESTI4005399, TESTI4007671, TESTI4010544, TESTI4010721, TESTI4013774, TESTI4014415, TESTI4014932, TESTI4014977, TESTI4017647, TESTI4017854, TESTI4019149, TESTI4021197, TESTI4021377, TESTI4021569, TESTI4022158, TESTI4023096, TESTI4023654, TESTI4024494, TESTI4026680, TESTI4027170, TESTI4028042, TESTI4031173, TESTI4031818, TESTI4032128, TESTI4034973, TESTI4035872, TESTI4035989, TESTI4036012, TESTI4037949, TESTI4038047, TESTI4040559, TESTI4041049, TESTI4043067, TESTI4043371, TESTI4045168, TESTI4046450, TESTI4047119, TESTI4048296, TESTI4048545, TESTI4051015, TESTI4051858, TESTI4052219, TESTI4052430, TESTI4052598, THYMU3002825, THYMU3003007, THYMU3003350, THYMU3008935, THYMU3009755, THYMU3011360, THYMU3013197, THYMU3014173, THYMU3015457, THYMU3015647, THYMU3016518, THYMU3018151, THYMU3019605, THYMU3021404, THYMU3022211, THYMU3022528, THYMU3022668, THYMU3023107, THYMU3023400, THYMU3025118, THYMU3025313, THYMU3025642, THYMU3026306, THYMU3026532, THYMU3026869, THYMU3027540, THYMU3028461, THYMU3029795, THYMU3031878, THYMU3032032, THYMU3033649, THYMU3033754, THYMU3034099, THYMU3034616, THYMU3036310, THYMU3036934, THYMU3036953, THYMU3037192, THYMU3037772, THYMU3038158, THYMU3038167, THYMU3040068, THYMU3040126, THYMU3040146, THYMU3040172, THYMU3040746, THYMU3040816, THYMU3041918, THYMU3042321, THYMU3043688, THYMU3043779, THYMU3044188, THYMU3045510, THYMU3047115, THYMU3047156, THYMU3047542, THYMU3047760, TKIDN2011160, TLIVE2008797, TRACH3003872, TRACH3004747, TRACH3005274, TRACH3005699, TRACH3007274, TRACH3007625, TRACH3009008, TRACH3009061, TRACH3010382, TRACH3011082, TRACH3011184, TRACH3012659, TRACH3012891, TRACH3013900, TRACH3014063, TRACH3014580, TRACH3015136, TRACH3015346, TRACH3016368, TRACH3016885, TRACH3016992, TRACH3017409, TRACH3018191, TRACH3018240, TRACH3018524, TRACH3018943, TRACH3019058, TRACH3019370, TRACH3019621, TRACH3019807, TRACH3020930, TRACH3021023, TRACH3021544, TRACH3022758, TRACH3023063, TRACH3023203, TRACH3023516, TRACH3023945, TRACH3024081, TRACH3024671, TRACH3025346, TRACH3026542, TRACH3027681, TRACH3029670, TRACH3031316, TRACH3031678, TRACH3032480, TRACH3034680, TRACH3036103, TRACH3036278, TSTOM2002682, UTERU3005422, UTERU3010029, UTERU3011092, UTERU3011398, UTERU3011837, UTERU3012414, UTERU3015647, UTERU3016273, UTERU3017626, UTERU3021850, UTERU3022168, UTERU3022922, UTERU3023413

The following 19 clones are also predicted to belong to the category of secretory protein and/or membrane protein.
ADRGL2011190, BRACE3046450, BRAWH3013197, BRAWH3028645, BRAWH3046240, BRTHA3024233, PROST2010326, TBAES2004105, TBAES2007379, TBAES2007481,TBAES2008133, TESTI2043585, TESTI4005158, THYMU3024602, THYMU3044175, THYMU3046350, TLIVE2007192, UTERU2025415, UTERU2036507

The clones predicted to belong to the category of glycoprotein-related protein are the following 114 clones.
3NB692004045, ADIPS2000069, BRACE2017397, BRACE3001403, BRACE3001973, BRACE3002264, BRACE3009392, BRACE3026345, BRACE3032385, BRACE3039358, BRACE3039378, BRACE3042432, BRACE3046466, BRACE3051621, BRAMY3004126, BRAWH2012866, BRAWH3001783, BRAWH3003573, BRAWH3014609, BRAWH3023156, BRAWH3024186, BRAWH3029806, BRAWH3040900, BRAWH3043623, BRAWH3044151, BRAWH3049544, BRCAN2003269, BRCAN2021325, BRHIP3005944, BRHIP3007424, BRHIP3010289, BRHIP3011269, BRHIP3011567, BRHIP3030230, BRHIP3036715, BRHIP3036936, BRHIP3039509, BRTHA2019726, BRTHA2020721, BRTHA2022968, BRTHA2025869, BRTHA2027250, BRTHA2031917, BRTHA2033155, BRTHA2033683, BRTHA3010135, CHONS2001287, COLON2004351, FEKID2002493, FELNG2000720, JCMLC1000159, JCMLC2000273, JCMLC2002095, JCMLC2002751, KIDNE2015987, LYMPB2002458, NT2RI3005923, NT2RI3009524, OCBBF2000831, OCBBF2004478, OCBBF2007039, OCBBF2018618, OCBBF3026979, PEBLM2005615, PLACE6001933, PLACE6010936, PLACE7006090, PLACE7012111, SPLEN2025012, STOMA2004663, T1ESE2002665, TESTI2007490, TESTI2022323, TESTI2037657, TESTI2052670, TESTI4001517, TESTI4014932, TESTI4031173, THYMU3014173, THYMU3015647, THYMU3016518, THYMU3020221, THYMU3025118, THYMU3026532, THYMU3032032, THYMU3037772, THYMU3040172, TKIDN2011160, TLUNG2001445, TRACH3005274, TRACH3009061, TRACH3015136, TRACH3018524, TRACH3018907, TRACH3019058, TRACH3019370, TRACH3019621, TRACH3019807, TRACH3020930, TRACH3021023, TRACH3023516, TRACH3025346, TRACH3026299, TRACH3028441, TRACH3029670, TRACH3031678, TRACH3034680, TRACH3037505, TRACH3038399, TUTER2001433, UTERU3011398, UTERU3011837, UTERU3015647, UTERU3021850

The following ten clones are also predicted to belong to the category of glycoprotein-related protein.
BRAWH3013197, BRAWH3046240, PROST2010326, TBAES2004105, TESTI2043585, TESTI4005158, THYMU3024602, THYMU3046350, TRACH3021066, UTERU2036507

The clones predicted to belong to the category of signal transduction-related protein are the following 71 clones.
BRACE3002344, BRACE3017253, BRACE3031315, BRACE3036283, BRACE3042046, BRACE3044172, BRACE3046491, BRACE3046609, BRAMY3009491, BRAMY3015547, BRAMY3017920, BRAWH3017180, BRAWH3019026, BRAWH3027806, BRAWH3032340, BRAWH3042438, BRAWH3047644, BRCAN2010665, BRHIP3006294, BRHIP3011460, BRHIP3011567, BRHIP3033557, BRHIP3037543, BRHIP3041587, BRTHA2026290, BRTHA2035743, BRTHA3011187, BRTHA3021708, BRTHA3025073, BRTHA3026916, KIDNE2010049, NlESE2000698, OCBBF3005330, OCBBF3006986, OCBBF3009244, OCBBF3025630, PLACE6000055, PLACE6001933, PLACE7009936, PLACE7011559, PLACE7014247, PUAEN2006639, SKMUS2008585, SPLEN2007689, TESTI2021654, TESTI2040377, TESTI4010902, TESTI4013474, TESTI4046073, TESTI4049786, TESTI4051865, THYMU3013785, THYMU3025683, THYMU3032798, TRACH3003037, TRACH3003357, TRACH3005173, TRACH3018519, TRACH3018606, TRACH3024020, TRACH3026650, TRACH3027701, TRACH3029462, TRACH3030176, TRACH3038399, TSTOM2001571, TSTOM2002611, UTERU2024042, UTERU3001029, UTERU3010919, UTERU3021231

The following four clones are also predicted to belong to the category of signal transduction-related protein.
BRHIP3030064, CTONG2003764, PLACE7013963, TRACH3021066

The clones predicted to belong to the category of transcription-related protein are the following 106 clones.
ASTRO2016114, BEAST2000981, BRACE2012947, BRACE2019348, BRACE3025719, BRACE3026844, BRACE3034183, BRACE3041162, BRACE3046152, BRAMY2040915, BRAMY3000692, BRAMY3007078, BRAMY3011581, BRAMY3014027, BRAMY3018754, BRAWH3000446, BRAWH3005886, BRAWH3011577, BRAWH3013009, BRAWH3013264, BRAWH3017477, BRAWH3023172, BRAWH3028796, BRAWH3031342, BRAWH3032571, BRAWH3035936, BRAWH3036247, BRAWH3036334, BRAWH3038827, BRCOC2012386, BRHIP2023735, BRHIP2027077, BRHIP2029529, BRHIP3004725, BRHIP3027651, BRHIP3028246, BRTHA2024712, BRTHA3000456, BRTHA3003736, BRTHA3010212, BRTHA3014000, BRTHA3028339, CHONS2000797, CHONS2002829, CTONG2001932, CTONG2011801, D9OST2003106, FCBBF3020030, FCBBF5000384, HCASM2008154, NETRP2004017, NT2RI3008179, NT2RI3009480, NT2RP8003490, NTONG2003805, NTONG2008483, OCBBF2016928, OCBBF3005330, OCBBF3008392, OCBBF3020263, OCBBF3021361, OCBBF3022166, PLACE7002303, PLACE7005169, PLACE7009757, SPLEN2012571, SPLEN2028417, SYNOV2003326, T1ESE2000904, TESTI2040377, TESTI4001679, TESTI4002799, TESTI4002868, TESTI4003796, TESTI4003944, TESTI4005322, TESTI4005470, TESTI4039904, TESTI4052775, THYMU3008105, THYMU3012983, THYMU3014372, THYMU3020869, THYMU3021586, THYMU3022434, THYMU3026000, THYMU3030072, THYMU3037052, THYMU3043200, TLIVE2001616, TRACH3003037, TRACH3003458, TRACH3004424, TRACH3010079, TRACH3010167, TRACH3010342, TRACH3015951, TRACH3021883, TRACH3022109, TRACH3028180, TRACH3036750, UTERU2037423, UTERU3012293, UTERU3016070, UTERU3019708, UTERU3022588

The following seven clones are also predicted to belong to the category of transcription-related protein.
BRACE3002184, BRCAN2019772, CTONG2002832, TESTI4002988, TESTI4005500, TRACH1000193, TRACH3019290

The clones predicted to belong to the category of disease-related protein are the following 391 clones.
ADIPS2000069, ASTRO2015162, ASTRO2016114, ASTRO3000154, BLADE2000256, BRACE1000475, BRACE2012838, BRACE2012947, BRACE2013009, BRACE2016896, BRACE2017397, BRACE2023744, BRACE2027382, BRACE3001403, BRACE3001973, BRACE3002756, BRACE3004767, BRACE3009392, BRACE3013418, BRACE3018083, BRACE3019941, BRACE3020669, BRACE3025719, BRACE3026345, BRACE3026802, BRACE3028998, BRACE3036283, BRACE3039378, BRACE3040644, BRACE3041059, BRACE3041162, BRACE3042046, BRACE3042432, BRACE3043597, BRACE3044172, BRACE3046152, BRACE3046466, BRACE3046609, BRACE3051621, BRACE3052321, BRALZ2010842, BRALZ2013621, BRAMY2041384, BRAMY3000692, BRAMY3004126, BRAMY3007078, BRAMY3009491, BRAMY3011501, BRAMY3011581, BRAMY3014027, BRAMY3015086, BRAMY3017920, BRAMY3018248, BRAWH2002333, BRAWH2012866, BRAWH2014053, BRAWH3001638, BRAWH3001783, BRAWH3004335, BRAWH3010602, BRAWH3011577, BRAWH3011623, BRAWH3017180, BRAWH3017259, BRAWH3018548, BRAWH3019026, BRAWH3021580, BRAWH3023156, BRAWH3023172, BRAWH3023415, BRAWH3024186, BRAWH3029385, BRAWH3029538, BRAWH3031342, BRAWH3032298, BRAWH3032571, BRAWH3033513, BRAWH3034668, BRAWH3034775, BRAWH3034890, BRAWH3036334, BRAWH3038324, BRAWH3038827, BRAWH3040900, BRAWH3041492, BRAWH3041556, BRAWH3042438, BRAWH3042447, BRAWH3042772, BRAWH3043295, BRAWH3043623, BRAWH3044151, BRAWH3046424, BRAWH3047565, BRAWH3047644, BRAWH3049544, BRCAN2003269, BRCAN2006051, BRCAN2010665, BRCAN2020331, BRCAN2021325, BRCOC2012386, BRHIP2008756, BRHIP2023735, BRHIP2029529, BRHIP3001076, BRHIP3001481, BRHIP3003984, BRHIP3004215, BRHIP3004725, BRHIP3005037, BRHIP3005307, BRHIP3005673, BRHIP3005801, BRHIP3006449, BRHIP3007609, BRHIP3010289, BRHIP3011567, BRHIP3017146, BRHIP3017855, BRHIP3021019, BRHIP3023922, BRHIP3025795, BRHIP3027191, BRHIP3027651, BRHIP3028742, BRHIP3029409, BRHIP3030230, BRHIP3032374, BRHIP3035006, BRHIP3036715, BRHIP3037543, BRHIP3039509, BRSSN2004710, BRSTN2006466, BRSTN2008475, BRSTN2011961, BRSTN2012069, BRSTN2016918, BRTHA2019726, BRTHA2020721, BRTHA2020910, BRTHA2024712, BRTHA2025869, BRTHA2026071, BRTHA2026290, BRTHA2031917, BRTHA2033155, BRTHA2033683, BRTHA3003736, BRTHA3010135, BRTHA3010212, BRTHA3011187, BRTHA3011998, BRTHA3012265, BRTHA3014547, BRTHA3021708, BRTHA3021971, BRTHA3023403, BRTHA3026916, BRTHA3027957, CHONS2001287, CHONS2002829, COLON2001829, COLON2004911, COLON2005735, CTONG2001932, CTONG2010330, CTONG2011801, CTONG2014206, D9OST2004417, FCBBF3020030, FCBBF3021191, FCBBF3024911, FCBBF5000384, FEBRA2013570, FEBRA2026582, FEBRA2028457, FEKID2002637, FELNG2000720, FELNG2001953, HCASM2008154, JCMLC1000159, JCMLC2000273, JCMLC2002095, KIDNE2015987, N1ESE2000698, NETRP2000961, NETRP2003448, NETRP2004017, NETRP2008582, NT2RI3008179, NT2RI3009480, NT2RI3009524, NT2RP8003787, NT2RP8008057, NTONG2003805, OCBBF2004478, OCBBF2007039, OCBBF2018618, OCBBF2024589, OCBBF2030927, OCBBF2036019, OCBBF3001202, OCBBF3004487, OCBBF3008392, OCBBF3020263, OCBBF3022166, OCBBF3025475, OCBBF3025503, OCBBF3025630, OCBBF3026979, PEBLM2005615, PLACE5000492, PLACE6001933, PLACE6016030, PLACE7000266, PLACE7001759, PLACE7002303, PLACE7003985, PLACE7004103, PLACE7006090, PLACE7006268, PLACE7006498, PLACE7007379, PLACE7009563, PLACE7009757, PLACE7009936, PLACE7011559, PLACE7012111, PLACE7014247, PLACE7016526, PUAEN2000594, SKNSH2007306, SMINT2011406, SMINT2011509, SMINT2014721, SPLEN2007689, SPLEN2012571, SPLEN2025012, SPLEN2028417, SPLEN2033996, SYNOV2003326, SYNOV4009139, TlESE2000609, TESTI2005112, TESTI2007490, TESTI2009739, TESTI2023903, TESTI2030901, TESTI2034913, TESTI2052670, TESTI4001517, TESTI4001679, TESTI4002868, TESTI4003796, TESTI4003944, TESTI4004653, TESTI4005322, TESTI4005653, TESTI4007965, TESTI4017382, TESTI4017647, TESTI4018436, TESTI4020596, TESTI4021197, TESTI4021569, TESTI4021713, TESTI4023096, TESTI4026080, TESTI4028182, TESTI4031173, TESTI4032128, TESTI4032834, TESTI4032913, TESTI4033177, TESTI4036048, TESTI4039575, TESTI4039904, TESTI4041984, TESTI4046073, TESTI4047119, TESTI4049786, TESTI4049899, TESTI4051015, TESTI4052775, THYMU3002825, THYMU3008105, THYMU3012402, THYMU3012983, THYMU3013785, THYMU3014173, THYMU3014372, THYMU3014620, THYMU3016518, THYMU3020221, THYMU3020869, THYMU3021586, THYMU3021755, THYMU3022434, THYMU3023400, THYMU3025118, THYMU3026306, THYMU3026532, THYMU3027671, THYMU3032032, THYMU3032798, THYMU3033649, THYMU3033759, THYMU3037052, THYMU3037772, THYMU3038158, THYMU3038375, THYMU3040172, THYMU3040746, THYMU3040816, THYMU3040829, THYMU3043200, THYMU3047115, THYMU3047760, TKIDN2011160, TLIVE2007736, TLUNG2000654, TLUNG2001445, TLUNG2001600, TRACH2024730, TRACH3004424, TRACH3005173, TRACH3005191, TRACH3005699, TRACH3006379, TRACH3006800, TRACH3008042, TRACH3009008, TRACH3009701, TRACH3010079, TRACH3010167, TRACH3010342, TRACH3011282, TRACH3011313, TRACH3011503, TRACH3012891, TRACH3015951, TRACH3016455, TRACH3016805, TRACH3018524, TRACH3018907, TRACH3019058, TRACH3019621, TRACH3020769, TRACH3020930, TRACH3021023, TRACH3021373, TRACH3021778, TRACH3021883, TRACH3023373, TRACH3023960, TRACH3024081, TRACH3024671, TRACH3025346, TRACH3026283, TRACH3026299, TRACH3028441, TRACH3028597, TRACH3028837, TRACH3029670, TRACH3030855, TRACH3031660, TRACH3031678, TRACH3032570, TRACH3034680, TRACH3036750, TRACH3037505, TRACH3038399, TUTER2001433, UTERU2024042, UTERU2037423, UTERU3001946, UTERU3004635, UTERU3011398, UTERU3012293, UTERU3012414, UTERU3012999, UTERU3015011, UTERU3015299, UTERU3016308, UTERU3017441, UTERU3017626, UTERU3019708, UTERU3021850, UTERU3022588

The following 23 clones are also predicted to belong to the category of disease-related protein.
ADRGL2011190, BRACE3002184, BRACE3026993, BRACE3046450, BRAWH3013197, BRAWH3046240, BRCAN2019772, BRHIP3030064, BRTHA3024233, CTONG2002832, CTONG2003764, PROST2010326, TBAES2004105, TESTI2046536, TESTI4005158, TESTI4005500, THYMU3024602, THYMU3046350, TRACH1000193, TRACH3019290, TRACH3021066, UTERU2017492, UTERU2036507

In particular, hit data of the following 390 clones for Swiss-Prot, or nr or RefSeq corresponded to genes or proteins which had been deposited in the Online Mendelian Inheritance in Man (OMIM), which is the human gene and disease database (the OMIM Number is shown in the parenthesis after the Clone Name).
ADIPS2000069 (146900) , ASTRO2015162 (606106) , ASTRO2016114 (603899) , ASTRO3000154(601594) , BLADE2000256 (140750) , BRACE1000475 (600696) , BRACE2012838 (605032) , BRACE2012947(140580) , BRACE2013009 (605888) , BRACE2016896 (601421) , BRACE2017397 (115437) , BRACE2023744 (600763) , BRACE2027382(606019), BRACE3001403(126141), BRACE3001973 (600976) , BRACE3002756(603143), BRACE3004767(182790), BRACE3009392(600229), BRACE3013418(182900), BRACE3018083(605268),
BRACE3019941 (600595) , BRACE3020669 (603917) , BRACE3025719 (605493) , BRACE3026345 (147470) , BRACE3026802(605784), BRACE3028998 (603063) , BRACE3036283(602052), BRACE3039378(604100), BRACE3040644(603159), BRACE3041059(603486), BRACE3041162(194556), BRACE3042046(311030), BRACE3042432 (192321) , BRACE3043597 (603704) , BRACE3044172 (601231) , BRACE3046152 (604950) , BRACE3046466 (604210;600105) ,
BRACE3046609(606457), BRACE3051621(601313;173900),
BRACE3052321 (603050) ,
BRALZ2010842(212138), BRALZ2013621(600712), BRAMY2041384(114070), BRAMY3000692(603971), BRAMY3004126(603071), BRAMY3007078 (602410) , BRAMY3009491(600286), BRAMY3011501(602869), BRAMY3011581(601243), BRAMY3014027 (194542) , BRAMY3015086(602879), BRAMY3017920(600365),
BRAMY3018248 (605464) , BRAWH2002333 (171891) , BRAWH2012866 (185605) , BRAWH2014053 (604581) , BRAWH3001638 (605003) , BRAWH3001783 (605514) , BRAWH3004335 (603244) , BRAWH3010602(603216),
BRAWH3011577(601139), BRAWH3011623(164020), BRAWH3017180 (601441) , BRAWH3017259(603143), BRAWH3018548(193065), BRAWH3019026(602033), BRAWH3021580(179838), BRAWH3023156 (137190) , BRAWH3023172 (603755) , BRAWH3023415 (604346) , BRAWH3024186 (179590) , BRAWH3029385 (602378) , BRAWH3029538 (600948) , BRAWH3031342 (603971) , BRAWH3032298 (601995) , BRAWH3032571 (603277) , BRAWH3033513 (604054;261510) ,
BRAWH3034668 (603486) , BRAWH3034775 (605800) , BRAWH3034890(606265), BRAWH3036334 (603971) , BRAWH3038324 (604249) , BRAWH3038827(600574) , BRAWH3040900(604265), BRAWH3041492 (130500) , BRAWH3041556 (172460) , BRAWH3042438(125855), BRAWH3042447(606323), BRAWH3042772(602878), BRAWH3043295(179030), BRAWH3043623(600976), BRAWH3044151(605421), BRAWH3046424(300272), BRAWH3047565(606277), BRAWH3047644(605216), BRAWH3049544(602273), BRCAN2003269(171060;602347),
BRCAN2006051(604581), BRCAN2010665(603583), BRCAN2020331(604851), BRCAN2021325(114855), BRCOC2012386(602277), BRHIP2008756(605819), BRHIP2023735(601670), BRHIP2029529(189972), BRHIP3001076(604673), BRHIP3001481(176889), BRHIP3003984(603722;223900),
BRHIP3004215(603294), BRHIP3004725(602075), BRHIP3005037(603526), BRHIP3005307(603197), BRHIP3005673(138385), BRHIP3005801(605704), BRHIP3006449(604275), BRHIP3007609(426000), BRHIP3010289(603130), BRHIP3011567(114207), BRHIP3017146(602878), BRHIP3017855(606406), BRHIP3021019(176879), BRHIP3023922(156570;250940),
BRHIP3025795(603877), BRHIP3027191(601746), BRHIP3027651(604589), BRHIP3028742(602076), BRHIP3029409(604156), BRHIP3030230 (602367) , BRHIP3032374 (603197) , BRHIP3035006(604402), BRHIP3036715 (142800) , BRHIP3037543(602052), BRHIP3039509(601328), BRSSN2004710 (600127) , BRSTN2006466 (138275) , BRSTN2008475 (605178) , BRSTN2011961 (176790) , BRSTN2012069(130590), BRSTN2016918(137780), BRTHA2019726(147100), BRTHA2020721(147100), BRTHA2020910(602661), BRTHA2024712(600747), BRTHA2025869(162280), BRTHA2026071(605297), BRTHA2026290(602306), BRTHA2031917(118946), BRTHA2033155(601873), BRTHA2033683(111000), BRTHA3003736(133510;234050), BRTHA3010135(179590),
BRTHA3010212 (603971) , BRTHA3011187 (605837) , BRTHA3011998 (603264) , BRTHA3012265 (605646) , BRTHA3014547 (182900) , BRTHA3021708(602654), BRTHA3021971 (605609) , BRTHA3023403 (600597) , BRTHA3026916(601619), BRTHA3027957 (606078) , CHONS2001287 (146732) , CHONS2002829 (602981) , COLON2001829(604399), COLON2004911(603937;180100), COLON2005735(111690;111700), CTONG2001932(605683),
CTONG2010330(606088), CTONG2011801(603971), CTONG2014206(605609), D9OST2004417 (113703), FCBBF3020030 (603406) , FCBBF3021191 (605119) , FCBBF5000384(601737), FEBRA2013570 (248600) , FEBRA2026582 (300252) , FEBRA2028457(164035), FEKID2002637(176875), FELNG2000720(601662), FELNG2001953(603597),
HCASM2008154(133450), JCMLC1000159 (107470;209950) ,
JCMLC2000273(120980), JCMLC2002095(600738), KIDNE2015987(191845), N1ESE2000698 (604734) , NETRP2000961 (600417) , NETRP2003448 (179551) , NETRP2004017(605344), NETRP2008582(103195), NT2RI3008179(603808), NT2RI3009480(601804), NT2RI3009524(604210;600105),
NT2RP8003787(605427), NT2RP8008057(603489), NTONG2003805(601781), OCBBF2004478(604265), OCBBF2007039(605009), OCBBF2018618(102775), OCBBF2024589(602462),
OCBBF2030927(603897), OCBBF2036019(601825;256000),
OCBBF3001202(140750), OCBBF3004487(142560), OCBBF3008392(605682), OCBBF3020263(604077), OCBBF3022166(600848), OCBBF3025475(604148), OCBBF3025503(601653;113650), OCBBF3025630(604141),
OCBBF3026979(602319), PEBLM2005615(600242), PLACE5000492(602142), PLACE6001933(131550), PLACE6016030(605442), PLACE7000266(188840), PLACE7001759(600338), PLACE7002303(601542;180500;137600;604229), PLACE7003985(109684), PLACE7004103(142695),
PLACE7006090 (154360) , PLACE7006268(603053), PLACE7006498(604394), PLACE7007379 (603105) , PLACE7009563 (300344) , PLACE7009757 (601804) , PLACE7009936(600365), PLACE7011559(600831), PLACE7012111(602714), PLACE7014247(601232), PLACE7016526(605490), PUAEN2000594(604679), SKNSH2007306(118990), SMINT2011406(147890), SMINT2011509(606343), SMINT2014721 (606090) , SPLEN2007689(233700), SPLEN2012571(603430), SPLEN2025012(146900), SPLEN2028417(142995),
SPLEN2033996(603853), SYNOV2003326(602960), SYNOV4009139(603551), T1ESE2000609(182465), TESTI2005112(603846), TESTI2007490(601291), TESTI2009739(160745), TESTI2023903(605046), TESTI2030901(600436),
TESTI2034913(148060), TESTI2052670(142461), TESTI4001517(148070), TESTI4001679(602850), TESTI4002868(601863;209920),
TESTI4003796(603132), TESTI4003944(603971), TESTI4004653(606106), TESTI4005322(603899), TESTI4005653(182465), TESTI4007965(603533), TESTI4017382(605689), TESTI4017647(603211), TESTI4018436(601754), TESTI4020596(602537), TESTI4021197(602189), TESTI4021569(605464), TESTI4021713(604105), TESTI4023096(604878), TESTI4026080(605575), TESTI4028182(603892), TESTI4031173(190197), TESTI4032128(104776),. TESTI4032834(300188), TESTI4032913(106410), TESTI4033177(602038), TESTI4036048(601272), TESTI4039575(600951), TESTI4039904(603899), TESTI4041984(604710), TESTI4046073(300118;309801),
TESTI4047119(606202), TESTI4049786(142600;235700),
TESTI4049899(601969), TESTI4051015(602974), TESTI4052775(165250), THYMU3002825 (604346) , THYMU3008105(194548), THYMU3012402(600686), THYMU3012983 (194556) , THYMU3013785(604722), THYMU3014173(143010), THYMU3014372(116945), THYMU3014620(605657), THYMU3016518 (147100) , THYMU3020221(147100), THYMU3020869(602550), THYMU3021586 (184756) , THYMU3021755 (605024) , THYMU3022434(601408), THYMU3023400 (605180) , THYMU3025118(155735), THYMU3026306(604346) ,
THYMU3026532(600065;116920), THYMU3027671(604143),
THYMU3032032(604463), THYMU3032798(601212), THYMU3033649(186780), THYMU3033759(600495), THYMU3037052(300346), THYMU3037772(147100), THYMU3038158(603033;603034), THYMU3038375(181590),
THYMU3040172(186720), THYMU3040746 (147110) , THYMU3040816(605704), THYMU3040829(602649), THYMU3043200(605596), THYMU3047115(108730), THYMU3047760(604783), TKIDN2011160(605011),
TLIVE2007736 (604990) , TLUNG2000654(148059), TLUNG2001445(146900), TLUNG2001600 (147130) , TRACH2024730(605611), TRACH3004424(603971), TRACH3005173(151410), TRACH3005191(605333), TRACH3005699(606154), TRACH3006379(148059), TRACH3006800(606154), TRACH3008042(166945), TRACH3009008(601112), TRACH3009701(603330), TRACH3010079(604850), TRACH3010167(601804), TRACH3010342(602943), TRACH3011282(601833), TRACH3011313(113520), TRACH3011503(602862),
TRACH3012891(602397), TRACH3015951(604084), TRACH3016455(605286), TRACH3016805(106410), TRACH3018524 (176882), TRACH3018907(146900), TRACH3019058(147170), TRACH3019621 (191350), TRACH3020769 (160776), TRACH3020930(147100), TRACH3021023 (147170) , TRACH3021373(606030), TRACH3021778 (164035) , TRACH3021883 (603347) , TRACH3023373(159350), TRACH3023960(603337), TRACH3024081 (605867) , TRACH3024671(605942), TRACH3025346(603377;212140), TRACH3026283(601517),
TRACH3026299(147170), TRACH3028441(147170), TRACH3028597 (604310), TRACH3028837(602127), TRACH3029670(147170), TRACH3030855(173321), TRACH3031660(176912), TRACH3031678(600523), TRACH3032570(602217), TRACH3034680(147170) , TRACH3036750(604077), TRACH3037505(147170), TRACH3038399(604032;226980), TUTER2001433(146900),
UTERU2024042(602214), UTERU2037423(604077), UTERU3001946(606154), UTERU3004635(103390), UTERU3011398(120240;158810;254090),
UTERU3012293(194556),
UTERU3012414(604394), UTERU3012999(605567), UTERU3015011(602505), UTERU3015299(601825;256000), UTERU3016308(602127),
UTERU3017441 (604276) , UTERU3017626 (603788) , UTERU3019708 (601430) , UTERU3021850(605009), UTERU3022588 (123811)

Additionally, hit data of the following 23 clones for Swiss-Prot, or nr or RefSeq corresponded to genes or proteins which had been deposited in the Online Mendelian Inheritance in Man (OMIM), which is the human gene and disease database (the OMIM Number is shown in the parenthesis after the Clone Name).
ADRGL2011190 (602658), BRACE3002184 (603899), BRACE3026993 (601282), BRACE3046450 (603035), BRAWH3013197 (604100), BRAWH3046240 (602646), BRCAN2019772 (603849), BRHIP3030064 (605762), BRTHA3024233 (603088), CTONG2002832 (604668), CTONG2003764 (176977), PROST2010326 (604110), TBAES2004105 (605008), TESTI2046536 (182890; 182882), TESTI4005158 (601328), TESTI4005500 (602277), THYMU3024602 (600493), THYMU3046350 (603080), TRACH1000193 (139139), TRACH3019290 (603975), TRACH3021066 (164870), UTERU2017492 (602917), UTERU2036507 (605008)

The clones predicted to belong to the category of enzyme and/or metabolism-related protein are the following 164 clones.
ASTRO2008972, BRACE1000475, BRACE2013132, BRACE2016896, BRACE2035120, BRACE3017253, BRACE3021805, BRACE3028998, BRACE3031315, BRACE3036283, BRACE3041059, BRACE3042409, BRACE3044172, BRACE3046609, BRAMY3009491, BRAMY3011581, BRAWH2002333, BRAWH2014053, BRAWH3001638, BRAWH3004335, BRAWH3011331, BRAWH3017180, BRAWH3020928, BRAWH3023415, BRAWH3023421, BRAWH3024186, BRAWH3024506, BRAWH3029385, BRAWH3029806, BRAWH3032571, BRAWH3033513, BRAWH3034668, BRAWH3037428, BRAWH3037979, BRAWH3041556, BRAWH3042438, BRAWH3043295, BRAWH3044151, BRAWH3046424, BRAWH3047692, BRAWH3049544, BRCAN2003814, BRCAN2006051, BRCAN2015402, BRCAN2021325, BRHIP3001481, BRHIP3003126, BRHIP3005307, BRHIP3005673, BRHIP3007195, BRHIP3007223, BRHIP3011082, BRHIP3011269, BRHIP3021019, BRHIP3023922, BRHIP3032374, BRHIP3035006, BRHIP3037543, BRHIP3041587, BRSSN2004710, BRSTN2006466, BRSTN2011961, BRTHA2005448, BRTHA2010672, BRTHA2025869, BRTHA2026311, BRTHA2033155, BRTHA2035743, BRTHA3003736, BRTHA3010135, BRTHA3010469, BRTHA3023403, CHONS2002829, COLON2004351, CTONG2010330, CTONG2020582, CTONG3001605, FCBBF3001018, FCBBF3021191, FCBBF5000384, FEBRA2013570, FEBRA2026582, FEKID2002637, HSYRA2004550, KIDNE2010049, NETRP2000961, NT2RI2004818, NT2RP7016508, OCBBF2007039, OCBBF2024589, OCBBF2036019, OCBBF3004487, OCBBF3005330, OCBBF3009244, PLACE5000492, PLACE6001933, PLACE7001759, PLACE7003985, PLACE7004103, PLACE7004961, PLACE7006090, PLACE7006268, PLACE7007379, PLACE7011559, PLACE7012111, PLACE7014247, PLACE7016526, SKMUS2008585, SMINT2011509, SMINT2012179, SMINT2014721, SPLEN2007689, SYNOV4009139, TBAES2007428, TESTI2005112, TESTI2007490, TESTI2021654, TESTI2040377, TESTI2049062, TESTI4000621, TESTI4002799, TESTI4007671, TESTI4020596, TESTI4033177, TESTI4049786, TESTI4052219, THYMU3002825, THYMU3026306, THYMU3032798, THYMU3034671, THYMU3036953, THYMU3041428, THYMU3047115, THYMU3047891, TKIDN2011160, TRACH3005173, TRACH3005274, TRACH3009008, TRACH3011313, TRACH3011503, TRACH3012891, TRACH3015136, TRACH3016455, TRACH3018108, TRACH3018261, TRACH3018524, TRACH3019621, TRACH3021544, TRACH3022758, TRACH3023516, TRACH3024020, TRACH3024081, TRACH3027229, TRACH3027701, TRACH3032150, TRACH3038399, TSTOM2001571, TSTOM2002611, UTERU2024042, UTERU3010604, UTERU3010919, UTERU3015299, UTERU3019708, UTERU3021850

The following eight clones are also predicted to belong to the category of enzyme and/or metabolism-related protein.
ADRGL2011190, BRHIP3030064, CTONG2003764, PLACE7013963, TBAES2004105, THYMU3044175, TRACH3021066, UTERU2036507

The clones predicted to belong to the category of cell division and/or cell proliferation-related protein are the following 27 clones.
BLADE2000256, BRACE2002392, BRACE3030538, BRACE3036283, BRACE3044495, BRAMY3002886, BRAMY3009556, BRAWH2016209, BRAWH3004350, BRAWH3027574, BRCAN2019907, BRHIP3001076, BRHIP3029409, BRSTN2008475, BRTHA3011265, FEKID2002637, NT2RP8005546, OCBBF3001202, PLACE7011559, SPLEN2033996, TESTI2023903, TESTI4020819, TESTI4049899, THYMU3014372, THYMU3021586, UTERU3010919, UTERU3012999

The following two clones are also predicted to belong to the category of cell division and/or cell proliferation-related protein.
TBAES2007481, TESTI2046536

The clones predicted to belong to the category of cytoskeleton-related protein are the following 60 clones.
BRACE3004767, BRACE3013418, BRACE3051819, BRAMY3005184, BRAMY3015086, BRAMY4000915, BRAMY4001652, BRAWH3001783, BRAWH3015175, BRAWH3018548, BRAWH3019026, BRAWH3021580, BRAWH3021724, BRAWH3027440, BRAWH3027806, BRAWH3029385, BRAWH3040900, BRAWH3041492, BRCAN2020467, BRHIP3003063, BRHIP3003340, BRSTN2016918, BRTHA2020910, BRTHA2025869, BRTHA3014547, BRTHA3025073, CERVX2000968, JCMLC2000273, N1ESE2000698, NT2RI3005923, OCBBF2003518, OCBBF2004478, OCBBF3027969, PLACE7000266, PLACE7004961, SMINT2010753, SPLEN2034934, SYNOV4003174, TESTI2001915, TESTI2009739, TESTI2034913, TESTI4001517, TESTI4004917, TESTI4010902, TESTI4032913, TESTI4051424, THYMU3026532, TLUNG2000654, TRACH3006379, TRACH3016805, TRACH3020769, TRACH3022960, TRACH3026650, TRACH3028837, TRACH3029462, TRACH3032570, UTERU3000670, UTERU3001029, UTERU3015011, UTERU3016308

The following one clone is also predicted to belong to the category of cytoskeleton-related protein.
BRACE3026993

The clones predicted to belong to the category of nuclear protein and/or RNA synthesis-related protein are the following 40 clones.
ASTRO3000154, BRACE3014714, BRACE3036283, BRALZ2013621, BRAMY3009556, BRAMY3011501, BRAWH3011623, BRAWH3017180, BRAWH3022651, BRAWH3038252, BRAWH3040695, BRAWH3046424, BRHIP3004215, BRHIP3007223, BRHIP3020046, BRTHA3010530, CTONG2006235, FEBRA2028457, NT2RP7016508, OCBBF3001333, OCBBF3004487, PLACE7004103, PLACE7006268, TESTI2036285, TESTI2037657, TESTI4014932, TESTI4028182, TESTI4032128, TESTI4033177, TESTI4039575, THYMU3012402, THYMU3040829, THYMU3041428, TRACH3002752, TRACH3018108, TRACH3021778, UTERU3004635, UTERU3010409, UTERU3010919, UTERU3013302

The following one clone is also predicted to belong to the category of nuclear protein and/or RNA synthesis-related protein.
TESTI2046536

The clones predicted to belong to the category of protein synthesis and/or transport-related protein are the following 50 clones.
BRACE2016896, BRACE2023744, BRACE3020669, BRACE3030538, BRACE3041059, BRACE3043597, BRAWH2014053, BRAWH3001638, BRAWH3010602, BRAWH3024506, BRAWH3026349, BRAWH3034668, BRAWH3037979, BRAWH3041556, BRAWH3044151, BRAWH3047692, BRCAN2000923, BRCAN2002892, BRCAN2006051, BRCAN2021325, BRHIP3007223, BRSTN2012069, BRTHA2005448, BRTHA2010672, CHONS2002829, CTONG3001605, D9OST2004417, OCBBF2000831, OCBBF2007039, PLACE6019600, PLACE7007379, PLACE7012111, PLACE7016526, TESTI4018436, TESTI4020596, TESTI4032128, TESTI4036048, THYMU3012402, THYMU3033759, THYMU3036953, THYMU3046360, TKIDN2011160, TRACH3016455, TRACH3018519, TRACH3021544, TRACH3025316, TRACH3030855, TRACH3038399, UTERU3014647, UTERU3021850

The following two clones are also predicted to belong to the category of protein synthesis and/or transport-related protein.
TBAES2004105, UTERU2036507

The clones predicted to belong to the category of cellular defense-related protein are the following five clones.
BRACE2012947, BRHIP2029529, BRTHA3003736, THYMU3015571,
TRACH3022296

The clones predicted to belong to the category of development and/or differentiation-related protein are the following 16 clones.
ASTRO3000154, BRACE3034964, BRAWH3004350, BRAWH3029538, BRAWH3038252, BRHIP3007424, BRTHA2024712, BRTHA3011265, FEKID2002493, NT2RP8003490, NT2RP8006452, OCBBF3025503, PLACE7002303, TESTI2026024, TRACH3028180, UTERU3016070

The following one clone is also predicted to belong to the category of development and/or differentiation-related protein.
BRCAN2019772

The clones predicted to belong to the category of DNA-binding and/or RNA-binding protein are the following 119 clones.
ASTRO2016114, BEAST2000981, BRACE2012947, BRACE2019348, BRACE3020669, BRACE3025719, BRACE3026844, BRACE3031743, BRACE3034183, BRACE3041162, BRACE3046152, BRALZ2013621, BRAMY2040915, BRAMY2046537, BRAMY3000692, BRAMY3007078, BRAMY3011501, BRAMY3011581, BRAMY3014027, BRAMY3018754, BRAMY4000962, BRAWH3000446, BRAWH3011577, BRAWH3011623, BRAWH3013009, BRAWH3013264, BRAWH3017477, BRAWH3028796, BRAWH3031342, BRAWH3032571, BRAWH3034775, BRAWH3035936, BRAWH3036247, BRAWH3036334, BRAWH3038827, BRCAN2020331, BRCOC2012386, BRHIP2027077, BRHIP3004725, BRHIP3028246, BRSSN2015497, BRTHA2024712, BRTHA3000456, BRTHA3003736, BRTHA3010212, BRTHA3014000, BRTHA3028339, CHONS2000797, CHONS2002829, CTONG2001932, CTONG2011801, D9OST2003106, FCBBF3020030, FCBBF5000384, FEBRA2028457, HCASM2008154, NETRP2004017, NT2RI3008179, NT2RI3009480, NT2RP7016508, NT2RP8003490, NTONG2003805, NTONG2008483, OCBBF2016928, OCBBF3004487, OCBBF3008392, OCBBF3020263, OCBBF3021361, OCBBF3022166, PLACE7002303, PLACE7004103, PLACE7005169, PLACE7009757, PROST2002078, PUAEN2000594, SMINT2011509, SPLEN2012571, SPLEN2028417, T1ESE2000609, T1ESE2000904, TESTI4002868, TESTI4003796, TESTI4003944, TESTI4005322, TESTI4005470, TESTI4005653, TESTI4032128, TESTI4039575, TESTI4039904, TESTI4052775, THYMU3008105, THYMU3012983, THYMU3014372, THYMU3020869, THYMU3021586, THYMU3026000, THYMU3030072, THYMU3033759, THYMU3037052, THYMU3040829, TLIVE2001616, TRACH3003458, TRACH3004424, TRACH3005191, TRACH3008508, TRACH3010079, TRACH3010167, TRACH3010342, TRACH3015951, TRACH3021778, TRACH3021883, TRACH3022109, TRACH3028180, TRACH3036750, UTERU2037423, UTERU3012293, UTERU3013302, UTERU3016070, UTERU3022588

The following eight clones are also predicted to belong to the category of DNA-binding and/or RNA-binding protein.
BRACE3002184, BRCAN2019772, CTONG2002832, TESTI2046536, TESTI4002988, TESTI4005500, TRACH1000193, TRACH3019290

The clones predicted to belong to the category of ATP binding and/or GTP-binding protein are the following 68 clones.
BRACE2013009, BRACE2016896, BRACE3002344, BRACE3014714, BRACE3017253, BRACE3036283, BRACE3051819, BRAMY3011501, BRAMY3018248, BRAWH2014053, BRAWH3015175, BRAWH3024506, BRAWH3029385, BRAWH3032571, BRAWH3037428, BRAWH3041556, BRAWH3047692, BRCAN2000923, BRCAN2002892, BRCAN2003269, BRCAN2006051, BRHIP3007195, BRHIP3027191, BRHIP3041587, BRSTN2012069, BRTHA2020910, BRTHA3003736, HSYRA2004550, KIDNE2010049, NETRP2003448, NT2RP7016508, OCBBF2003518, OCBBF3004487, PLACE6001933, PLACE6019600, PLACE7004961, PLACE7006498, PLACE7011559, PLACE7016526, PUAEN2006639, SMINT2011406, TESTI2009739, TESTI2040377, TESTI4004917, TESTI4013474, TESTI4021569, TESTI4028182, TESTI4049786, TESTI4052219, THYMU3014372, THYMU3032798, THYMU3041428, THYMU3047115, TRACH3005191, TRACH3009061, TRACH3009701, TRACH3012891, TRACH3019370, TRACH3020769, TRACH3022960, TRACH3023960, TRACH3024081, TRACH3038399, TSTOM2001571, UTERU2024042, UTERU3010919, UTERU3012414, UTERU3019708

The following two clones are also predicted to belong to the category of ATP binding and/or GTP-binding protein.
CTONG2003764, TRACH3021066

The 104 clones shown below are clones which were unassignable to any of the above-mentioned categories, but have been predicted to have some functions based on homology search using their full-length nucleotide sequences. Clone Name and Definition in the result of homology search, demarcated by a double slash mark (//), are shown below.
BLADE2002310//SH3-domain binding protein 1 [Homo sapiens]
BLADE2007799//Hepatocellular carcinoma-associated antigen 66.
BRACE2017359//Mus musculus suppressor of Ty 6 homolog (S. cerevisiae) (Supt6h)
BRACE2017872//nuclear receptor-binding SET-domain protein 1 [Mus musculus]
BRACE3009416//testis specific ankyrin-like protein 1 [Homo sapiens]
BRACE3016020//SBBI31 protein [Homo sapiens]
BRACE3019570//SNAP-25-interacting protein [Rattus norvegicus]
BRACE3022303//Pax transcription activation domain interacting protein [Mus musculus]
BRACE3022340//SNAP-25-interacting protein [Rattus norvegicus]
BRACE3026290//Homo sapiens lethal giant larvae homolog 2 [Homo sapiens]
BRACE3032631//F-box protein FBX13 [Mus musculus].
BRACE3040239//Deltex3 [Mus musculus]
BRACE3047482//tripartite motif-containing 9 [Homo sapiens]
BRACE3049714//NYD-TSPG protein [Homo sapiens]
BRACE3052410//IDN3 protein [Homo sapiens]
BRACE3052595//Nim2 [Rattus norvegicus]
BRALZ2014054//cenexin 2 [Rattus norvegicus].
BRAMY3007471//gene trap locus F3b; transcript expressed during hematopoiesis 2 [Mus musculus]
BRAMY3010321//MRIP-1 protein [Homo sapiens]
BRAMY3014613//SH3-domain binding protein 1 [Homo sapiens]
BRAMY4001863//Mus musculus enabled homolog (Drosophila) (Enah), mRNA
BRAWH2011796//S-100 protein; alpha chain.
BRAWH3008167//CUB and Sushi multiple domains 1 [Homo sapiens]
BRAWH3009961//Nim2 [Rattus norvegicus]
BRAWH3010726//phosphatidylinositol transfer protein, membrane-associated; Drosophila retinal degeneration B [Homo sapiens]
BRAWH3015017//axonemal dynein light chain p33.
BRAWH3024231//Tetratricopeptide repeat protein 4.
BRAWH3026938//semaF cytoplasmic domain associated protein 3; semaphorin cytoplasmic domain-associated protein 3A [Mus musculus]
BRAWH3027533//rap2 interacting protein x [Homo sapiens].
BRAWH3030910//Sec23-interacting protein p125 [Homo sapiens]
BRAWH3031710//serologically defined colon cancer antigen 33 [Homo sapiens]
BRAWH3033293//synaptopodin [Homo sapiens]
BRAWH3042568//ventral anterior homeobox containing gene 1 [Mus musculus]
BRAWH3043034//Mus musculus neuregulin 1 (Nrg1)
BRAWH3044122//Munc13-1 [Rattus norvegicus]
BRHIP2026346//lymphocyte specific formin related protein; formin-related gene in leukocytes [Mus musculus]
BRHIP2027563//host cell factor homolog [Homo sapiens]
BRHIP3002114//rTS beta protein [Homo sapiens]
BRHIP3003795//cytochrome P450 retinoid metabolizing protein [Homo sapiens]
BRHIP3006786//peptidylprolyl isomerase (cyclophilin)-like 2; cyclophilin-like protein CyP-60 [Homo sapiens]
BRHIP3017109//Socs-5 [Mus musculus]
BRHIP3019643//Homo sapiens gamma tubulin ring complex protein (76p gene) (76P), mRNA
BRHIP3032148//brain-enriched guanylate kinase-associated [Rattus norvegicus]
BRSTN2006638//synaptotagmin interacting protein 1 [Rattus norvegicus]
BRSTN2016892//BUP protein [Homo sapiens]
BRSTN2016992//DRR1 protein (TU3A protein).
BRSTN2017151//COP9 (constitutive photomorphogenic), subunit 7a (Arabidopsis); COP9 complex S7a [Mus musculus]
BRTHA2020642//DRR1 protein (TU3A protein).
BRTHA3018409//synaptotagmin-like 4; granuphilin-a; granuphilin-b; granuphilin
BRTHA3019183//Ca<2+>dependent activator protein for secretion; Ca2+-dependent activator protein for secretion [Mus musculus]
CHONS2001834//tumor endothelial marker 7 precursor [Homo sapiens]
CTONG2009570//rab11 binding protein [Bos taurus].
CTONG2012123//Mus musculus enabled homolog (Drosophila) (Enah), mRNA
CTONG2027591//Mus musculus pecanex homolog (Drosophila) (Pcnx), mRNA.
CTONG3003669//high-glucose-regulated protein 8 [Homo sapiens]
ERLTF2002178//Kelch-like protein X.
HHDPC2008185//jerky [Mus musculus]
NT2RI3001573//F-box protein FBL10 [Mus musculus].
NT2RI3007095//Mus musculus neuregulin 1 (Nrg1), mRNA.
NT2RP8001363//signal peptide, CUB domain, EGF-like 1 [Mus musculus]
NT2RP8001584//alpha integrin binding protein 63 [Homo sapiens]
NT2RP8001604//CUB and Sushi multiple domains 1 [Homo sapiens]
OCBBF3019269//Homo sapiens Dvl-binding protein IDAX (inhibition of the Dvl and Axin complex) (IDAX)
OCBBF3022827//putative Rab5 GDP/GTP exchange factor homologue [Homo sapiens]
OCBBF3023913//Mus musculus protein phosphatase 1, regulatory (inhibitor) subunit 1C (Ppp1r1c)
PLACE6003004//rTS beta protein [Homo sapiens]
PLACE6008315//similar to ALPHA-ACTININ, SARCOMERIC (F-ACTIN CROSS LINKING PROTEIN) (D. melanogaster) [Homo sapiens].
PLACE6010925//NY-REN-50 antigen [Homo sapiens]
PLACE7012127//AAA-ATPase TOB3 [Homo sapiens]
PROST2016566//erythroblast macrophage protein [Mus musculus]
SYNOV2017179//EBP50-PDZ interactor of 64 kD [Homo sapiens]
SYNOV3000345//upregulated by 1,25-dihydroxyvitamin D-3 [Homo sapiens]
TBAES2003917//NG28 protein [Mus musculus]
TESTI2001364//lactate dehydrogenase A -like [Homo sapiens]
TESTI2004601//NYD-TSPG protein [Homo sapiens]
TESTI2009497//GPI-anchored protein p137 (p137GPI).
TESTI4002774//oxysterol binding protein 2 [Mus musculus]
TESTI4003579//FH1/FH2 domains-containing protein (Formin homolog overexpressed in spleen) (FHOS).
TESTI4003703//retinoblastoma-associated protein RAP140 [Homo sapiens]
TESTI4013742//antigen identified by monoclonal antibody 2A8 [Mus musculus]
TESTI4014908//dedicator of cyto-kinesis 2 [Mus musculus]
TESTI4018506//tomosyn [Rattus norvegicus]
TESTI4020342//H326 [Homo sapiens]
TESTI4024294//WW domain binding protein 2 [Mus musculus]
TESTI4039451//B29 protein [Homo sapiens]
TESTI4041482//Rattus norvegicus SEC15 homolog (S. cerevisiae) (Sec15), mRNA
TESTI4043166//lymphocyte specific formin related protein; formin-related gene in leukocytes [Mus musculus]
TESTI4047328//otogelin [Mus musculus]
THYMU3011717//exocyst component protein 70 kDa homolog (S. cerevisiae)
THYMU3016822//erythroblast macrophage protein [Mus musculus]
THYMU3026479//secretory pathway component Sec31B-1 [Homo sapiens]
THYMU3028702//chromosome condensation-related SMC-associated protein 1; chromosome condensation-related SMC-associated protein 1; KIAA0159 gene product [Homo sapiens]
THYMU3029719//AAA-ATPase TOB3 [Homo sapiens]
THYMU3038347//tumor stroma and activated macrophage protein DLM-1 [Homo sapiens]
THYMU3038603//WW domain binding protein 2 [Mus musculus]
THYMU3040830//AD-012 protein [Homo sapiens]
THYMU3041603//gamma-tubulin complex protein 2 [Homo sapiens]
TKIDN2003396//Homo sapiens paternally expressed 10 (PEG10), mRNA TRACH2011057//D-type cyclin-interacting protein 1; MAID protein [Homo sapiens]
TRACH3004412//clusterin-like 1 (retinal); unknown prepropeptide specific to rod photoreceptor [Homo sapiens]
TRACH3012106//erythrocyte protein band 4.1-like 3 [Mus musculus]
UTERU3009775//PAPIN [Rattus norvegicus]
UTERU3010892//adaptor-related protein complex 3, delta 1 subunit; adaptin, delta [Homo sapiens]
UTERU3017995//p47 [Homo sapiens]

So far no information suggesting the function of the remaining 879 clones has been provided by the homology search. The functions of these clones may be clarified when an updated database becomes available in future. Clone names are shown below.
ADRGL2010594, AHMSC1000138, BLADE2004849, BLADE2006043, BLADE2007735, BLADE2008809, BRACE2005991, BRACE2010336, BRACE2012625, BRACE2012833, BRACE2012936, BRACE2017844, BRACE2025452, BRACE2026404, BRACE2027312, BRACE2028956, BRACE2032584, BRACE2033128, BRACE2034434, BRACE2039362, BRACE2039607, BRACE2042541, BRACE2046976, BRACE2047232, BRACE2047975, BRACE3002541, BRACE3003866, BRACE3004046, BRACE3004371, BRACE3004887, BRACE3005870, BRACE3005903,. BRACE3006553, BRACE3007649, BRACE3009075, BRACE3009265, BRACE3009539, BRACE3010702, BRACE3011447, BRACE3011774, BRACE3015090, BRACE3016167, BRACE3016580, BRACE3016788, BRACE3016810, BRACE3016862, BRACE3019611, BRACE3019817, BRACE3020356, BRACE3021430, BRACE3022312, BRACE3022847, BRACE3023604, BRACE3024537, BRACE3025627, BRACE3026161, BRACE3026947, BRACE3027256, BRACE3027931, BRACE3028895, BRACE3029005, BRACE3029205, BRACE3029447, BRACE3031161, BRACE3031184, BRACE3031185, BRACE3031843, BRACE3032538, BRACE3034389, BRACE3035168, BRACE3036156, BRACE3036271, BRACE3037612, BRACE3038012, BRACE3038030, BRACE3038760, BRACE3039288, BRACE3039454, BRACE3040012, BRACE3040504, BRACE3041827, BRACE3042210, BRACE3042594, BRACE3044247, BRACE3044377, BRACE3045078, BRACE3045145, BRACE3045424, BRACE3045708, BRACE3045981, BRACE3046294, BRACE3046837, BRACE3046855, BRACE3046966, BRACE3047018, BRACE3047801, BRACE3048483, BRACE3048615, BRACE3048677, BRACE3048756, BRACE3048904, BRACE3048905, BRACE3049186, BRACE3050270, BRACE3051627, BRACE3051722, BRACE3051879, BRALZ2003119, BRALZ2007661, BRALZ2008930, BRAMY2022320, BRAMY2023939, BRAMY2031516, BRAMY2033895, BRAMY2035801, BRAMY2036254, BRAMY2036266, BRAMY2039630, BRAMY2041347, BRAMY2046489, BRAMY3001409, BRAMY3002329, BRAMY3002508, BRAMY3004364, BRAMY3005656, BRAMY3005912, BRAMY3008436, BRAMY3009158, BRAMY3010603, BRAMY3011865, BRAMY3014555, BRAMY3017827, BRAMY3017965, BRAMY3018121, BRAMY3018340, BRAMY4001234, BRAMY4001913, BRAMY4002575, BRAMY4002628, BRAWH2004078, BRAWH2010619, BRAWH2012054, BRAWH2013955, BRAWH2016223, BRAWH2016514, BRAWH2016562, BRAWH2016785, BRAWH3000884, BRAWH3003244, BRAWH3003975, BRAWH3005037, BRAWH3005896, BRAWH3008559, BRAWH3010833, BRAWH3011101, BRAWH3011402, BRAWH3011685, BRAWH3011929, BRAWH3013508, BRAWH3014639, BRAWH3015610, BRAWH3015825, BRAWH3016715, BRAWH3017260, BRAWH3017980, BRAWH3018369, BRAWH3019594, BRAWH3020318, BRAWH3021574, BRAWH3021643, BRAWH3022431, BRAWH3022459, BRAWH3022542, BRAWH3022719, BRAWH3022900, BRAWH3023168, BRAWH3024989, BRAWH3025157, BRAWH3027420, BRAWH3027607, BRAWH3027616, BRAWH3027675, BRAWH3028202, BRAWH3028461, BRAWH3029313, BRAWH3030772, BRAWH3031054, BRAWH3033117, BRAWH3033448, BRAWH3034097, BRAWH3034743, BRAWH3035403, BRAWH3035904, BRAWH3036077, BRAWH3036561, BRAWH3037265, BRAWH3037394, BRAWH3037533, BRAWH3038230, BRAWH3039258, BRAWH3039623, BRAWH3040297, BRAWH3041928, BRAWH3042787, BRAWH3042820, BRAWH3043944, BRAWH3044487, BRAWH3044585, BRAWH3044985, BRAWH3045118, BRAWH3045229, BRAWH3045625, BRAWH3046209, BRAWH3046802, BRAWH3046959, BRAWH3047539, BRAWH3047946, BRAWH3048548, BRAWH3049726, BRAWH3049858, BRCAN2007525, BRCAN2008701, BRCAN2009168, BRCAN2010547, BRCAN2010581, BRCAN2015757, BRCAN2018667, BRCAN2019953, BRCAN2020880, BRCAN2021452, BRCAN2021718, BRCAN2025093, BRCAN2027593, BRCAN2028702, BRCOC2001355, BRCOC2002777, BRCOC2006942, BRCOC2010115, BRHIP2006819, BRHIP2009177, BRHIP2011199, BRHIP2013958, BRHIP2015153, BRHIP2016125, BRHIP2017714, BRHIP2024941, BRHIP2029643, BRHIP2029663, BRHIP3000626, BRHIP3001141, BRHIP3001338, BRHIP3001360, BRHIP3001573, BRHIP3002141, BRHIP3002363, BRHIP3002691, BRHIP3002920, BRHIP3002931, BRHIP3003688, BRHIP3003845, BRHIP3003961, BRHIP3004710, BRHIP3004786, BRHIP3005142, BRHIP3005231, BRHIP3006279, BRHIP3007172, BRHIP3007291, BRHIP3007409, BRHIP3008082, BRHIP3008714, BRHIP3009672, BRHIP3009753, BRHIP3010916, BRHIP3012289, BRHIP3012736, BRHIP3013588, BRHIP3013698, BRHIP3014675, BRHIP3015854, BRHIP3016421, BRHIP3017256, BRHIP3018784, BRHIP3019824, BRHIP3019880, BRHIP3020155, BRHIP3021499, BRHIP3021987, BRHIP3022656, BRHIP3024703, BRHIP3024820, BRHIP3026231, BRHIP3026651, BRHIP3027947, BRHIP3028570, BRHIP3029670, BRHIP3029866, BRHIP3031890, BRHIP3032311, BRHIP3033481, BRHIP3033734, BRHIP3033806, BRHIP3036371, BRHIP3038030, BRHIP3038735, BRHIP3039592, BRHIP3040878, BRHIP3042817, BRHIP3043012, BRSSN2004303, BRSSN2008464, BRSSN2011843, BRSSN2012157, BRSSN2012198, BRSSN2013696, BRSTN2000312, BRSTN2009247, BRSTN2010416, BRSTN2015699, BRSTN2015788, BRSTN2017104, BRSTN2017184, BRSTN2018712, BRTHA2001304, BRTHA2001953, BRTHA2002091, BRTHA2006720, BRTHA2008502, BRTHA2008598, BRTHA2020695, BRTHA2022074, BRTHA2023402, BRTHA2024177, BRTHA2024354, BRTHA2027227, BRTHA2027229, BRTHA2028297, BRTHA2029969, BRTHA2030213, BRTHA2031517, BRTHA2032763, BRTHA2033122, BRTHA2034281, BRTHA2034576, BRTHA2037247, BRTHA2038279, BRTHA2038345, BRTHA2038353, BRTHA3002411, BRTHA3003417, BRTHA3005988, BRTHA3007469, BRTHA3007662, BRTHA3009858, BRTHA3011229, BRTHA3011306, BRTHA3011361, BRTHA3011510, BRTHA3011892, BRTHA3013860, BRTHA3014105, BRTHA3014507, BRTHA3014854, BRTHA3014920, BRTHA3017791, BRTHA3020369, BRTHA3020771, BRTHA3021569, BRTHA3021786, BRTHA3022641, BRTHA3023590, BRTHA3023929, BRTHA3024600, BRTHA3026180, BRTHA3026556, BRTHA3027171, BRTHA3027318, BRTHA3027638, BRTHA3027879, CERVX2000812, COLON2001866, CTONG2003517, CTONG2009033, CTONG2010633, CTONG2014959, CTONG2026987, CTONG2027783, CTONG2027959, CTONG3002518, D9OST2003989, DFNES2001829, DFNES2011221, ERLTF2001452, ERLTF2001835, ERLTF2002369, FCBBF3002188, FCBBF3005160, FEBRA2000805, FEBRA2002260, FEBRA2012625, FEBRA2013069, FEBRA2017736, FEBRA2017811, FEBRA2028222, FEHRT2001482, FEKID2001001, FEKID2001201, FELNG2001613, HCASM2007773, HEART2002531, HLUNG2012600, HSYRA2007338, KIDNE2014496, KIDNE2016464, KIDNE2018268, LIVER2008465, LYMPB2001387, LYMPB2002344, MESAN2007032, MESAN2009156, MESAN2016304, MESAN2017133, MESTC2000170, NETRP2000439, NETRP2002082, NETRP2003103, NETRP2003268, NETRP2003539, NETRP2005972, NETRP2006468, NETRP2007945, NETRP2008488, NOVAR2000783, NT2NE2011107, NT2NE2016041, NT2RI2009233, NT2RI2010795, NT2RI2012542, NT2RI2015533, NT2RI2023671, NT2RI2028537, NT2RP7003439, NT2RP7007387, NT2RP7014178, NT2RP7014778, NT2RP7017139, NT2RP7020343, NT2RP8000633, NT2RP8001407, NT2RP8003657, NT2RP8006521, NT2RP8007416, NT2RP8007503, NT2RP8007920, NT2RP8009119, NT2RP8009248, NTONG2002278, NTONG2004829, NTONG2009468, OCBBF2014745, OCBBF2019761, OCBBF2024779, OCBBF2025631, OCBBF3000743, OCBBF3000830, OCBBF3001076, OCBBF3001616, OCBBF3004908, OCBBF3005843, OCBBF3007078, OCBBF3008835, OCBBF3020414, OCBBF3021086, OCBBF3021166, OCBBF3021515, OCBBF3023543, OCBBF3025127, OCBBF3025131, OCBBF3025901, OCBBF3026088, OCBBF3026361, PEBLM2001803, PEBLM2006298, PERIC2003349, PLACE5000527, PLACE6019542, PLACE7003684, PLACE7005671, PLACE7005840, PLACE7006240, PLACE7006540, PLACE7007973, PLACE7008766, PLACE7010567, PLACE7013060, PLACE7014396, PLACE7015238, PLACE7015647, PLACE7016214, PLACE7018304, PLACE7018349, PLACE7018452, PLACE7018479, PLACE7018512, PROST2007444, PROST2017578, PROST2017729, PROST2017749, PROST2017910, RECTM2001519, SKMUS2009479, SKMUS2009557, SKNMC2003639, SMINT2009292, SMINT2009895, SMINT2012040, SMINT2014166, SMINT2017964, SMINT2019105, SPLEN2001227, SPLEN2017999, SPLEN2019092, SPLEN2019480, SPLEN2021231, SPLEN2021991, SPLEN2022785, SPLEN2022920, SPLEN2024571, SPLEN2027852, SPLEN2028593, SPLEN2032677, SPLEN2034601, SPLEN2036608, SPLEN2037077, STOMA2003894, SYNOV4000598, SYNOV4004210, SYNOV4009575, TBAES2007548, TBAES2007862, TESOP2002005, TESOP2003308, TESOP2004110, TESTI1000459, TESTI2004452, TESTI2004971, TESTI2005153, TESTI2005564, TESTI2006543, TESTI2008636, TESTI2011020, TESTI2011033, TESTI2018687, TESTI2018867, TESTI2021112, TESTI2023053, TESTI2026284, TESTI2030519, TESTI2033905, TESTI2035962, TESTI2037209, TESTI2037572, TESTI2038733, TESTI2039342, TESTI2039732, TESTI2039738, TESTI2040372, TESTI2041362, TESTI2041976, TESTI2051742, TESTI2052110, TESTI2052202, TESTI2052799, TESTI4000370, TESTI4000534, TESTI4000600, TESTI4000703, TESTI4000957, TESTI4001348, TESTI4001569, TESTI4002003, TESTI4002141, TESTI4002195, TESTI4002520, TESTI4003179, TESTI4003279, TESTI4003319, TESTI4003404, TESTI4003565, TESTI4003574, TESTI4003733, TESTI4004031, TESTI4004210, TESTI4004695, TESTI4005013, TESTI4006441, TESTI4006539, TESTI4007565, TESTI4008086, TESTI4008305, TESTI4009501, TESTI4010979, TESTI4011616, TESTI4011744, TESTI4011926, TESTI4012258, TESTI4012382, TESTI4012623, TESTI4012956, TESTI4012960, TESTI4013960, TESTI4013962, TESTI4014262, TESTI4014891, TESTI4015129, TESTI4015339, TESTI4016848, TESTI4017229, TESTI4021129, TESTI4021821, TESTI4023172, TESTI4024240, TESTI4024245, TESTI4025062, TESTI4025401, TESTI4025908, TESTI4027139, TESTI4027262, TESTI4027660, TESTI4029023, TESTI4029297, TESTI4029651, TESTI4029676, TESTI4029731, TESTI4029743, TESTI4030319, TESTI4030673, TESTI4030864, TESTI4031066, TESTI4032270, TESTI4032375, TESTI4032856, TESTI4034633, TESTI4035770, TESTI4035898, TESTI4037228, TESTI4038758, TESTI4039744, TESTI4040197, TESTI4040598, TESTI4040804, TESTI4041832, TESTI4042420, TESTI4042846, TESTI4043223, TESTI4043378, TESTI4044076, TESTI4044291, TESTI4044770, TESTI4045330, TESTI4045470, TESTI4046090, TESTI4046245, TESTI4046328, TESTI4046873, TESTI4046962, TESTI4047305, TESTI4047437, TESTI4047569, TESTI4047746, TESTI4047808, TESTI4048232, TESTI4048619, TESTI4049110, TESTI4049552, TESTI4049562, TESTI4049677, TESTI4049863, TESTI4050293, TESTI4050954, TESTI4051054, TESTI4051388, TESTI4051504, TESTI4051747, TESTI4051952, TESTI4052132, TESTI4052217, THYMU2008207, THYMU2038199, THYMU3000390, THYMU3000776, THYMU3001082, THYMU3001593, THYMU3001776, THYMU3002887, THYMU3003958, THYMU3004628, THYMU3004632, THYMU3007308, THYMU3007559, THYMU3009643, THYMU3011012, THYMU3011244, THYMU3011534, THYMU3011556, THYMU3012907, THYMU3013114, THYMU3013241, THYMU3013470, THYMU3013897, THYMU3014038, THYMU3014701, THYMU3015042, THYMU3017761, THYMU3018896, THYMU3019095, THYMU3019476, THYMU3019916, THYMU3020970, THYMU3021900, THYMU3022982, THYMU3023394, THYMU3023797, THYMU3024164, THYMU3024339, THYMU3025772, THYMU3026350, THYMU3026783, THYMU3027251, THYMU3027655, THYMU3028075, THYMU3029188, THYMU3029318, THYMU3029421, THYMU3029774, THYMU3030706, THYMU3030752, THYMU3031146, THYMU3031402, THYMU3031612, THYMU3031868, THYMU3032867, THYMU3033626, THYMU3033630, THYMU3034453, THYMU3034853, THYMU3034867, THYMU3034983, THYMU3036200, THYMU3037617, THYMU3037827, THYMU3037856, THYMU3037867, THYMU3037909, THYMU3037980, THYMU3038214, THYMU3038266, THYMU3038687, THYMU3038759, THYMU3038879, THYMU3038970, THYMU3039807, THYMU3039846, THYMU3040168, THYMU3040725, THYMU3041354, THYMU3041386, THYMU3041573, THYMU3041736, THYMU3042075, THYMU3042758, THYMU3043327, THYMU3043482, THYMU3043883, THYMU3043993, THYMU3044075, THYMU3044441, THYMU3044445, THYMU3045673, THYMU3045692, THYMU3045704, THYMU3046140, THYMU3047144, THYMU3047513, TKIDN2000319, TKIDN2010602, TKIDN2011051, TLIVE2000142, TLUNG2001810, TLUNG2002055, TRACH2013585, TRACH2019080, TRACH2022113, TRACH3002188, TRACH3002293, TRACH3002890, TRACH3004113, TRACH3004288, TRACH3004596, TRACH3006397, TRACH3006717, TRACH3007689, TRACH3007995, TRACH3008632, TRACH3011454, TRACH3011538, TRACH3012460, TRACH3012718, TRACH3012864, TRACH3013043, TRACH3013072, TRACH3013684, TRACH3014183, TRACH3015354, TRACH3015467, TRACH3016264, TRACH3018783, TRACH3020137, TRACH3020563, TRACH3020605, TRACH3021335, TRACH3021834, TRACH3022198, TRACH3022732, TRACH3023242, TRACH3023752, TRACH3024342, TRACH3024423, TRACH3024428, TRACH3024512, TRACH3024823, TRACH3025520, TRACH3026303, TRACH3026676, TRACH3026949, TRACH3028164, TRACH3028855, TRACH3029139, TRACH3029329, TRACH3029520, TRACH3029592, TRACH3032044, TRACH3033535, TRACH3035451, TRACH3036004, TRACH3036792, TRACH3036897, TRACH3036932, TSTOM2000235, TUTER1000014, UTERU2000300, UTERU2014998, UTERU2016464, UTERU2016669, UTERU2020226, UTERU2022955, UTERU2023941, UTERU2027369, UTERU2028377, UTERU2029660, UTERU2035926, UTERU3001394, UTERU3005264, UTERU3006538, UTERU3006720, UTERU3007108, UTERU3011558, UTERU3011579, UTERU3012476, UTERU3012599, UTERU3013167, UTERU3014274, UTERU3014906, UTERU3015844, UTERU3016274, UTERU3018172, UTERU3018255, UTERU3020090, UTERU3023141

Likewise, so far no information suggesting the function of six clones shown below has been provided by the homology search. The functions of these clones may be clarified when an updated database becomes available in future. Clone names are shown below.
BRAMY3008096, BRAMY3016953, BRHIP3038037, BRTHA3004432,
TESTI4052089, UTERU3020583

### EXAMPLE 7

### Functional categorization based on a functional domain search for deduced amino acid sequences

Domains and motifs are minimal functional structures of polypeptides. The structure of a polypeptide is constituted by a collection of such minimal structures, and thus the overall function of a polypeptide is ensured by the resulting structure. Thus, the overall function of a polypeptide can be predicted relatively accurately using data obtained by analysis of domain and motif structures. Furthermore, classifying these results into functional categories in a database allows clones comprising specific functions to be easily selected. Thus, such databases are highly useful in the functional analysis of each clone.

Pfam was used to undertake a domain search for the amino acid sequences deduced from the full-length nucleotide sequences (see Example 5). Based on these results, the proteins encoded by clones 664 and 29 were categorized and their functions predicted. This was performed by referring to domain and motif names, accession numbers for hit data, and detailed descriptions in Pfam (http://www.sanger.ac.uk/Software/Pfam/index.shtml) as well as functional categorizations in PROSITE (http://www.expasy.ch/cgi-bin/prosite-list.pl).

A clone predicted to belong to the category of secretory and/or membrane protein means a clone having domains and motifs, for example, seven-transmembrane receptor, pancreatic hormone peptides, ion transport protein, or fibroblast growth factor, which suggest receptor, ion channel., hormone, or growth factor.

A clone predicted to belong to the category of glycoprotein-related protein means a clone having domains and motifs, for example, immunoglobulin domain or glycosyl transferases group 1, which suggest involvement in glycobiology, such as glycoprotein or glycosyltransferase.

A clone predicted to belong to the category of signal transduction-related protein means a clone having domains and motifs, for example, eukaryotic protein kinase domain, protein phosphatase 2C, or Ras family, which suggest protein kinase, dephosphoenzyme, SH2 domain, or small G protein.

A clone predicted to belong to the category of transcription-related protein means a clone having domains and motifs, for example, bZIP transcription factor, Zinc finger, or C2H2 type, which suggest transcription factor or transcription-controlling protein.

A clone predicted to belong to the category of disease-related protein means a clone having domains and motifs, for example, Wilm's tumor protein or von Hippel-Lindau disease tumor suppressor protein, which suggest proteins with disease-specific expression or that promote or suppress expression, depending on the disease.

A clone predicted to belong to the category of enzyme and/or metabolism-related protein means a clone having domains and motifs, for example, aldehyde dehydrogenase family, chitin synthase, or glucose-6-phosphate dehydrogenase, which suggest transferase, synthase, or hydrolase.

A clone predicted to belong to the category of cell division- and/or cell proliferation-related protein means a clone having domains and motifs, for example, cyclin or cell division protein, which suggest cyclin or cell proliferation-controlling protein.

A clone predicted to belong to the category of cytoskeleton-related protein means a clone having domains and motifs, for example, actin, fibronectin type I domain, or kinesin motor domain, which suggest actin, kinesin, or fibronectin.

A clone predicted to belong to the category of nuclear protein and/or RNA synthesis-related protein means a clone having domains and motifs, for example, hepatitis C virus RNA dependent RNA polymerase or DEAD/DEAH box helicase, which suggest splicing factor, RNA synthase, or helicase.

A clone predicted to belong to the category of protein synthesis and/or transport-related protein means a clone having domains and motifs, for example, translation initiation factor SUI1, ubiquitin family, or ribosomal protein L16, which suggest translation-related protein, ubiquitin-related protein, or ribosomal protein.

A clone predicted to belong to the category of cellular defense-related protein means a clone having domains and motifs, for example, HSP90 protein or DNA mismatch repair protein, which suggest chaperonin or DNA repair protein.

A clone predicted to belong to the category of development-and/or differentiation-related proteins means a clone having domains and motifs, for example, floricaula / leafy protein, which suggest organogenesis-related protein.

A clone predicted to belong to the category of DNA- and/or RNA-binding protein means a clone having domains and motifs, for example, transcription factor WhiB, B-box zinc finger, or tRNA synthetases class I (C), which suggest DNA/RNA-relating enzyme group including transcription factor and DNA ligase or Zinc-finger related protein.

A clone predicted to belong to the category of ATP- and/or GTP-binding protein means a clone having domains and motifs, for example, E1-E2 ATPase or Ras family, which suggest ATP/GTP-related enzyme group including ATPase or G protein.

During this functional categorization, if a clone met every criterion of multiple categories as described above, it was grouped into multiple categories. However, the function of a polypeptide is not limited to these functional categories.

The clones predicted to belong to the category of secretory protein and/or membrane protein are the following 92 clones.
3NB692004045, BRACE3002264, BRACE3009392, BRACE3013418, BRACE3024879, BRACE3032385, BRACE3039378, BRACE3042432, BRACE3050504, BRACE3051621, BRAMY2046537, BRAMY3004126, BRAWH2000256, BRAWH2011812, BRAWH3023156, BRAWH3025157, BRAWH3027880, BRAWH3036270, BRAWH3037265, BRAWH3042772, BRCAN2003269, BRCAN2022126, BRCOC2006164, BRHIP3002000, BRHIP3005944, BRHIP3008320, BRHIP3011567, BRHIP3014675, BRHIP3016032, BRHIP3017558, BRHIP3025795, BRHIP3033557, BRHIP3039509, BRSTN2010089, BRTHA2031917, BRTHA3011194, BRTHA3012265, BRTHA3014547, COLON2005735, JCMLC2000273, KIDNE2004531, LYMPB2002236, NT2RP7019682, NT2RP8001363, NT2RP8003787, OCBBF2003518, OCBBF2004478, OCBBF2009536, OCBBF2018618, OCBBF3004487, OCBBF3025475, OCBBF3028001, PEBLM2005615, PLACE6010936, PLACE7004103, PLACE7011559, PLACE7018304, TESTI2018335, TESTI2022323, TESTI2024267, TESTI2036822, TESTI4003602, TESTI4004539, TESTI4005399, TESTI4008305, TESTI4010544, TESTI4014415, TESTI4021569, TESTI4023096, TESTI4026080, TESTI4040559, TESTI4049899, THYMU3015647, THYMU3021404, THYMU3023400, THYMU3026532, THYMU3030752, THYMU3040172, THYMU3044075, TRACH3003357, TRACH3004113, TRACH3004747, TRACH3005699, TRACH3006800, TRACH3009061, TRACH3019370, TRACH3023373, TRACH3031678, TRACH3032150, UTERU3001946, UTERU3016273, UTERU3017626

The following ten clones are also predicted to belong to the category of secretory protein and/or membrane protein.
BRAWH3013197, BRAWH3028645, BRAWH3046240, BRCAN2019772, PROST2010326, TESTI2043585, TESTI4005158, THYMU3024602, THYMU3046350, TRACH1000193

The clones predicted to belong to the category of glycoprotein-related protein are the following 81 clones.
3NB692004045, ADIPS2000069, BRACE2017397, BRACE3013874, BRACE3017253, BRACE3039358, BRAMY2040915, BRAMY3015549, BRAWH3009961, BRAWH3023415, BRAWH3049544, BRHIP3017558, BRHIP3025795, BRHIP3036371, BRHIP3036715, BRHIP3038735, BRTHA2019726, BRTHA2020400, BRTHA2020721, BRTHA3017791, CERVX2000968, FELNG2000720, JCMLC2000273, KIDNE2004531, NT2RP8008057, OCBBF2000831, OCBBF2004478, OCBBF2030927, PEBLM2005615, PLACE7006090, SPLEN2025012, STOMA2004663, TESTI2021654, TESTI2052670, TESTI4008305, TESTI4022158, TESTI4031173, TESTI4032128, TESTI4037949, TESTI4051424, THYMU3002825, THYMU3014173, THYMU3016518, THYMU3020221, THYMU3025118, THYMU3026306, THYMU3026532, THYMU3037772, THYMU3040746, TLUNG2001445, TLUNG2001600, TRACH3003357, TRACH3004113, TRACH3004412, TRACH3005274, TRACH3005699, TRACH3006800, TRACH3011082, TRACH3011184, TRACH3012659, TRACH3015354, TRACH3018261, TRACH3018907, TRACH3019058, TRACH3019621, TRACH3019807, TRACH3020930, TRACH3021023, TRACH3024512, TRACH3026299, TRACH3028441, TRACH3029670, TRACH3031316, TRACH3034680, TRACH3036103, TRACH3037505, TUTER2001433, UTERU3001946, UTERU3010409, UTERU3011398, UTERU3015647

The following two clones are also predicted to belong to the category of glycoprotein-related protein.
BRAWH3028645, TESTI4005158

The clones predicted to belong to the category of signal transduction-related protein are the following 125 clones.
BLADE2002310, BLADE2008809, BRACE2047975, BRACE3002344, BRACE3003866, BRACE3004767, BRACE3013418, BRACE3015898, BRACE3017253, BRACE3042046, BRACE3044172, BRACE3045424, BRACE3046491, BRACE3051621, BRACE3052321, BRACE3052595, BRAMY3005184, BRAMY3009491, BRAMY3010321, BRAMY3014613, BRAMY3015547, BRAMY3017920, BRAWH2012866, BRAWH3009961, BRAWH3017180, BRAWH3018063, BRAWH3019026, BRAWH3022431, BRAWH3024186, BRAWH3026349, BRAWH3027574, BRAWH3027806, BRAWH3029385, BRAWH3031342, BRAWH3032340, BRAWH3035914, BRAWH3037428, BRAWH3044122, BRAWH3047692, BRCAN2000923, BRCAN2002892, BRCOC2001355, BRHIP3003306, BRHIP3006294, BRHIP3006786, BRHIP3011460, BRHIP3017109, BRHIP3021019, BRHIP3028570, BRHIP3037543, BRHIP3041587, BRTHA2026290, BRTHA2027250, BRTHA3014547, BRTHA3020771, BRTHA3021708, BRTHA3023403, BRTHA3026916, CTONG2009570, ERLTF2002369, FEKID2001001, FEKID2002637, FELNG2001953, KIDNE2010049, LYMPB2002344, N1ESE2000698, NETRP2003448, NT2RI2004818, NTONG2008483, OCBBF3006986, OCBBF3021086, OCBBF3021502, OCBBF3023175, PLACE5000492, PLACE6000055, PLACE6019600, PLACE7009936, PLACE7014247, PLACE7016526, PUAEN2006639, SKMUS2008585, SKMUS2009557, SMINT2017964, SPLEN2007689, SYNOV2017179, SYNOV4009575, TESTI4002774, TESTI4004695, TESTI4010902, TESTI4012960, TESTI4013474, TESTI4020342, TESTI4020596, TESTI4021197, TESTI4022158, TESTI4028042, TESTI4029731, TESTI4033177, TESTI4036048, TESTI4046073, TESTI4047808, TESTI4049786, TESTI4051865, THYMU3013785, THYMU3025683, THYMU3032798, TRACH2024730, TRACH3003037, TRACH3003357, TRACH3005173, TRACH3011538, TRACH3018519, TRACH3020605, TRACH3024020, TRACH3030176, TRACH3031660, TRACH3036750, TRACH3038399, TSTOM2001571, UTERU2024042, UTERU3001029, UTERU3006720, UTERU3010919, UTERU3021231, UTERU3022168

The following three clones are also predicted to belong to the category of signal transduction-related protein.
BRAMY3008096, TESTI4052089, TRACH3021066

The clones predicted to belong to the category of transcription-related protein are the following 141 clones.
ASTRO2016114, BEAST2000981, BRACE2019348, BRACE3025719, BRACE3026844, BRACE3026947, BRACE3029021, BRACE3034183, BRACE3040239, BRACE3041162, BRACE3047482, BRAMY2041347, BRAMY3000692, BRAMY3007078, BRAMY3011581, BRAMY3014027, BRAMY4002575, BRAWH2000256, BRAWH2014053, BRAWH2016209,. BRAWH3000446, BRAWH3005886, BRAWH3009961, BRAWH3013009, BRAWH3013264, BRAWH3015175, BRAWH3015610, BRAWH3017477, BRAWH3021580, BRAWH3022651, BRAWH3027533, BRAWH3027880, BRAWH3028796, BRAWH3031342, BRAWH3031710, BRAWH3032571, BRAWH3035936, BRAWH3036247, BRAWH3036334, BRAWH3042787, BRAWH3044122, BRAWH3046424, BRCAN2021452, BRCOC2012386, BRHIP2027077, BRHIP2029663, BRHIP3005037, BRHIP3007609, BRHIP3017256, BRHIP3019824, BRHIP3027651, BRHIP3028246, BRHIP3039592, BRSSN2011843, BRSTN2012069, BRTHA3000456, BRTHA3003225, BRTHA3010212, BRTHA3014000, BRTHA3028339, CHONS2000797, CTONG2006235, CTONG2011801, FCBBF3020030, FEBRA2002260, HCASM2008154, KIDNE2018268, NETRP2003103, NETRP2004017, NT2RI3009480, NTONG2003805, NTONG2008483, OCBBF2014745, OCBBF2016928, OCBBF3001333, OCBBF3008392, OCBBF3019269, OCBBF3020263, OCBBF3022827, OCBBF3025503, OCBBF3026361, PLACE7005169, PLACE7007973, PLACE7009757, PLACE7018512, SMINT2014721, SPLEN2012571, SPLEN2036608, T1ESE2000904, TESTI2036822, TESTI2040377, TESTI4000370, TESTI4000621, TESTI4001679, TESTI4002799, TESTI4003796, TESTI4003944, TESTI4005322, TESTI4005470, TESTI4024494, TESTI4028182, TESTI4032913, TESTI4039904, TESTI4051054, TESTI4052775, THYMU2008207, THYMU2038199, THYMU3008105, THYMU3012983, THYMU3014372, THYMU3021586, THYMU3022434, THYMU3026000, THYMU3030072, THYMU3034671, THYMU3037617, THYMU3043200, THYMU3045704, TKIDN2003396, TLIVE2001616, TLUNG2000654, TRACH3002752, TRACH3003037, TRACH3003458, TRACH3004113, TRACH3004412, TRACH3004424, TRACH3005274, TRACH3010079, TRACH3010167, TRACH3015951, TRACH3022109, TRACH3026303, TRACH3028180, TRACH3036750, UTERU2037423, UTERU3012293, UTERU3015011, UTERU3016070, UTERU3018172, UTERU3022588

The following eight clones are also predicted to belong to the category of transcription-related protein.
BRACE3002184, BRCAN2019772, CTONG2002832, TESTI2046536, TESTI4002988, TESTI4005500, TRACH1000193, TRACH3019290

The clones predicted to belong to the category of disease-related protein are the following four clones.
BRAWH3041928, BRHIP3000859, BRTHA3018409, THYMU3025642

The clones predicted to belong to the category of enzyme and/or metabolism-related protein are the following 264 clones.
BRACE1000475, BRACE2003628, BRACE2012528, BRACE2013132, BRACE2016896, BRACE2035120, BRACE2042541, BRACE2047975, BRACE3002344, BRACE3009392, BRACE3013418, BRACE3015898, BRACE3017253, BRACE3019941, BRACE3024444, BRACE3031315, BRACE3031372, BRACE3032537, BRACE3033525, BRACE3034183, BRACE3034964, BRACE3039288, BRACE3039454, BRACE3041059, BRACE3042409, BRACE3044172, BRACE3046491, BRACE3049714, BRACE3050270, BRACE3051819, BRACE3052410, BRACE3052595, BRAMY3007078, BRAMY3009491, BRAMY3011581, BRAMY3014613, BRAMY4000915, BRAWH2000256, BRAWH2002333, BRAWH2012866, BRAWH2014053, BRAWH2016785, BRAWH3009961, BRAWH3010657, BRAWH3013264, BRAWH3015175, BRAWH3017180, BRAWH3017259, BRAWH3019026, BRAWH3021724, BRAWH3022431, BRAWH3023415, BRAWH3024186, BRAWH3028796, BRAWH3029385, BRAWH3029806, BRAWH3032571, BRAWH3033513, BRAWH3034668, BRAWH3034743, BRAWH3037979, BRAWH3041556, BRAWH3043295, BRAWH3044122, BRAWH3044985, BRAWH3046424, BRAWH3047692, BRAWH3048724, BRAWH3049544, BRCAN2003814, BRCAN2006051, BRCAN2015402, BRCAN2021325, BRCOC2001355, BRCOC2006164, BRHIP2029663, BRHIP3001481, BRHIP3002000, BRHIP3002114, BRHIP3002141, BRHIP3003063, BRHIP3003126, BRHIP3003795, BRHIP3004725, BRHIP3005307, BRHIP3005673, BRHIP3007195, BRHIP3007223, BRHIP3011082, BRHIP3012289, BRHIP3016032, BRHIP3019643, BRHIP3021019, BRHIP3032374, BRHIP3033557, BRHIP3035006, BRHIP3037543, BRHIP3038030, BRHIP3041587, BRSSN2004710, BRSSN2011843, BRSTN2011961, BRSTN2016918, BRTHA2001304, BRTHA2005448, BRTHA2026290, BRTHA2026311, BRTHA2027250, BRTHA2030036, BRTHA2033683, BRTHA2035743, BRTHA2036295, BRTHA2037247, BRTHA3003736, BRTHA3010135, BRTHA3014547, BRTHA3021786, BRTHA3023403, BRTHA3026916, CHONS2002829, COLON2004351, CTONG2010330, CTONG2020582, CTONG2026987, FCBBF3001018, FCBBF3021191, FEBRA2013570, FEBRA2026582, FEHRT2002708, FEKID2002637, HHDPC2008185, HSYRA2004550, KIDNE2004531, KIDNE2010049, LYMPB2002236, NT2RI2004818, NT2RI3001967, NT2RP7016508, NT2RP8003490, NT2RP8003787, NT2RP8005546, OCBBF2000831, OCBBF2007039, OCBBF2024589, OCBBF3001616, OCBBF3004487, OCBBF3021086, OCBBF3023175, OCBBF3025503, OCBBF3026088, OCBBF3026361, PLACE5000492, PLACE6003004, PLACE7003985, PLACE7004103, PLACE7004961, PLACE7006090, PLACE7007379, PLACE7008136, PLACE7012111, PLACE7014247, PLACE7016526, SKMUS2008585, SKMUS2009557, SMINT2011406, SMINT2011509, SYNOV2017179, SYNOV4003174, SYNOV4009139, T1ESE2000609, T1ESE2002665, TESTI2001364, TESTI2005112, TESTI2007490, TESTI2018335, TESTI2021112, TESTI2021654, TESTI2030901, TESTI2037877, TESTI2049062, TESTI4000621, TESTI4002774, TESTI4002799, TESTI4003404, TESTI4003565, TESTI4003602, TESTI4003703, TESTI4005399, TESTI4007671, TESTI4010544, TESTI4010721, TESTI4012960, TESTI4017854, TESTI4020342, TESTI4020596, TESTI4020819, TESTI4021129, TESTI4021197, TESTI4023096, TESTI4024494, TESTI4026080, TESTI4028182, TESTI4031066, TESTI4033177, TESTI4040598, TESTI4041482, TESTI4046073, TESTI4047808, TESTI4049786, TESTI4051424, TESTI4051865, TESTI4052219, THYMU3000390, THYMU3002825, THYMU3014372, THYMU3023400, THYMU3025683, THYMU3026306, THYMU3026479, THYMU3031878, THYMU3032798, THYMU3034671, THYMU3036953, THYMU3041428, THYMU3047115, THYMU3047891, TRACH2022113, TRACH2024730, TRACH3003037, TRACH3005274, TRACH3006800, TRACH3009008, TRACH3009061, TRACH3011313, TRACH3016455, TRACH3017409, TRACH3018108, TRACH3018261, TRACH3019621, TRACH3021544, TRACH3023516, TRACH3024020, TRACH3024081, TRACH3024342, TRACH3024512, TRACH3025316, TRACH3026303, TRACH3026650, TRACH3027229, TRACH3027701, TRACH3029329, TRACH3032150, TRACH3036750, TRACH3038399, TSTOM2001571, UTERU2024042, UTERU3001946, UTERU3010604, UTERU3010919, UTERU3015299, UTERU3017441, UTERU3019708, UTERU3020090, UTERU3022168

The following seven clones are also predicted to belong to the category of enzyme and/or metabolism-related protein.
ADRGL2011190, BRAMY3008096, CTONG2002832, TESTI4052089,
THYMU3024602, THYMU3044175, TRACH3021066

The clones predicted to belong to the category of cell division and/or cell proliferation-related protein are the following 13 clones.
BRACE3022303, BRAWH3017260, BRHIP2008756, BRHIP3028570, BRSTN2006638, NT2RI2004818, PLACE7009563, PLACE7016526, SMINT2014721, THYMU3025642, THYMU3033626, TRACH3029329, UTERU3010919

The following one clone is also predicted to belong to the category of cell division and/or cell proliferation-related protein.
TBAES2007481

The clones predicted to belong to the category of cytoskeleton-related protein are the following 51 clones.
BRACE2046976, BRACE3013874, BRACE3047482, BRACE3051819, BRAMY3015549, BRAWH3015175, BRAWH3018548, BRAWH3021580, BRAWH3024186, BRAWH3024506, BRAWH3029385, BRAWH3032298, BRAWH3049544, BRHIP3003340, BRHIP3012736, BRHIP3036936, BRSTN2016918, BRTHA2020910, BRTHA2025869, BRTHA2031917, BRTHA3011361, BRTHA3025073, CTONG2008989, HSYRA2007338, LYMPB2002458, OCBBF3008835, OCBBF3027969, PEBLM2006298, PLACE7000266, PLACE7004103, PLACE7004961, SMINT2011406, SYNOV4003174, TESTI2006543, TESTI2034913, TESTI4001517, TESTI4005653, TESTI4008305, TESTI4041049, TESTI4051424, TESTI4051865, THYMU3020221, THYMU3038158, TLUNG2000654, TRACH3002890, TRACH3006379, TRACH3012460, TRACH3018524, TRACH3020769, TRACH3028837, UTERU3011837

The clones predicted to belong to the category of nuclear protein and/or RNA synthesis-related protein are the following 29 clones.
BRACE2016896, BRACE3032537, BRACE3034183, BRACE3039288, BRAWH3013264, BRAWH3032571, BRCOC2006164, BRHIP3004725, BRSSN2011843, BRTHA2026290, BRTHA3003736, BRTHA3014547, NT2RP7016508, NT2RP8005546, OCBBF3004487, OCBBF3021086, OCBBF3026361, PLACE5000492, TESTI4000621, TESTI4002799, TESTI4010721, TESTI4012960, THYMU3014372, THYMU3033626, THYMU3041428, TRACH3017409, TRACH3029462, UTERU3010919, UTERU3019708

The clones predicted to belong to the category of protein synthesis and/or transport-related protein are the following 50 clones.
BRACE3033525, BRACE3041059, BRAMY4001652, BRAWH3010657, BRAWH3013264, BRAWH3034668, BRAWH3036247, BRAWH3037428, BRAWH3037979, BRCAN2000923, BRCAN2002892, BRCAN2006051, BRCAN2021325, BRCAN2021718, BRHIP2029663, BRHIP3002000, BRHIP3003126, BRHIP3007223, BRHIP3011082, BRSTN2010089, BRTHA2036295, BRTHA3012265, CHONS2002829, D9OST2004417, HHDPC2008185, NETRP2003448, OCBBF2007039, OCBBF3021086, PLACE6003004, PLACE6010925, PLACE7006498, PLACE7007379, PLACE7012111, PLACE7016526, TESTI2023903, TESTI2036285, TESTI4003602, TESTI4012960, TESTI4014415, TESTI4030864, TESTI4051865, THYMU3036953, THYMU3047891, TRACH3004113, TRACH3006800, TRACH3009061, TRACH3021544, TRACH3026650, UTERU3001946, UTERU3012414

The following one clone is also predicted to belong to the category of protein synthesis and/or transport-related protein.
CTONG2002832

The clones predicted to belong to the category of cellular defense-related protein are the following four clones.
BRHIP3027191, SYNOV4009575, TESTI2023903, TRACH3029462

The clone predicted to belong to the category of development and/or differentiation-related protein is the following one clone.
CHONS2000797

The clones predicted to belong to the category of DNA-binding and/or RNA-binding protein are the following 185 clones.
ASTRO2016114, BEAST2000981, BRACE2012625, BRACE2016896, BRACE2019348, BRACE3019941, BRACE3025719, BRACE3026844, BRACE3026947, BRACE3029021, BRACE3031743, BRACE3032537, BRACE3034183, BRACE3039288, BRACE3040239, BRACE3041162, BRACE3047482, BRACE3050270, BRALZ2013621, BRAMY2041347, BRAMY3000692, BRAMY3007078, BRAMY3011581, BRAMY3014027, BRAMY4002575, BRAWH2000256, BRAWH2014053, BRAWH2016209, BRAWH3000446, BRAWH3005886, BRAWH3009961, BRAWH3011623, BRAWH3013009, BRAWH3013264, BRAWH3015175, BRAWH3015610, BRAWH3017477, BRAWH3021580, BRAWH3022651, BRAWH3027533, BRAWH3027607, BRAWH3027880, BRAWH3028796, BRAWH3031342, BRAWH3031710, BRAWH3032571, BRAWH3035403, BRAWH3035936, BRAWH3036247, BRAWH3036334, BRAWH3038055, BRAWH3042787, BRAWH3044122, BRCAN2020331, BRCAN2021452, BRCOC2006164, BRCOC2012386, BRHIP2027077, BRHIP2029663, BRHIP3002141, BRHIP3004725, BRHIP3005037, BRHIP3007609, BRHIP3017256, BRHIP3019824, BRHIP3027651, BRHIP3028246, BRHIP3039592, BRSSN2011843, BRSTN2012069, BRTHA2026290, BRTHA2037247, BRTHA3000456, BRTHA3003225, BRTHA3003736, BRTHA3010212, BRTHA3014000, BRTHA3014547, BRTHA3028339, CHONS2000797, CTONG2003517, CTONG2006235, CTONG2011801, CTONG2026987, D9OST2003106, FCBBF3020030, FEBRA2002260, FEBRA2028457, FEHRT2002708, HCASM2008154, KIDNE2018268, NETRP2003103, NETRP2004017, NT2RI3009480, NT2RP7016508, NT2RP8005546, NTONG2003805, NTONG2008483, OCBBF2014745, OCBBF2016928, OCBBF3001333, OCBBF3001616, OCBBF3004487, OCBBF3008392, OCBBF3019269, OCBBF3020263, OCBBF3021086, OCBBF3021361, OCBBF3022827, OCBBF3025503, OCBBF3026361, PLACE5000492, PLACE7004103, PLACE7005169, PLACE7007973, PLACE7008136, PLACE7009757, PLACE7018512, PROST2002078, SMINT2011509, SMINT2014721, SPLEN2012571, SPLEN2036608, T1ESE2000609, T1ESE2000904, TESTI2036822, TESTI2040377, TESTI4000370, TESTI4000621, TESTI4001679, TESTI4002799, TESTI4003796, TESTI4003944, TESTI4005322, TESTI4005470, TESTI4012960, TESTI4024494, TESTI4028182, TESTI4032913, TESTI4039904, TESTI4051054, TESTI4052775, THYMU2008207, THYMU2038199, THYMU3008105, THYMU3012983, THYMU3014372, THYMU3021586, THYMU3022434, THYMU3023400, THYMU3026000, THYMU3030072, THYMU3037617, THYMU3040829, THYMU3041428, THYMU3043200, THYMU3045704, TKIDN2003396, TLIVE2001616, TLUNG2000654, TRACH3002752, TRACH3003037, TRACH3003458, TRACH3004113, TRACH3004412, TRACH3004424, TRACH3005274, TRACH3010079, TRACH3010167, TRACH3015951, TRACH3017409, TRACH3021778, TRACH3022109, TRACH3026303, TRACH3028180, TRACH3036750, UTERU2037423, UTERU3012293, UTERU3015011, UTERU3016070, UTERU3019708, UTERU3020090, UTERU3022168, UTERU3022588, UTERU3023141

The following seven clones are also predicted to belong to the category of DNA-binding and/or RNA-binding protein.
BRACE3002184, BRCAN2019772, CTONG2002832, TESTI4002988, TESTI4005500, TRACH1000193, TRACH3019290

The clones predicted to belong to the category of ATP binding and/or GTP-binding protein are the following 41 clones.
BRACE3042409, BRAWH2002333, BRAWH2014053, BRAWH3015175, BRAWH3029385, BRAWH3029806, BRAWH3034743, BRAWH3037428, BRAWH3047692, BRCAN2000923, BRCAN2002892, BRCAN2022126, BRCOC2001355, BRCOC2006164, BRHIP3007195, BRSSN2004710, BRTHA2026290, BRTHA2033683, BRTHA3026916, CTONG2020582, HSYRA2004550, NETRP2003448, PLACE6019600, PLACE7004961, PLACE7006498, PLACE7016526, SMINT2011406, TESTI4010544, TESTI4014415, TESTI4028182, TESTI4029731, TESTI4040559, TESTI4041482, TESTI4052219, THYMU3013785, THYMU3047115, TRACH2024730, TRACH3024081, TRACH3029329, TRACH3031660, UTERU3012414

The following one clone is also predicted to belong to the category of ATP binding and/or GTP-binding protein.
ADRGL2011190

Although the 172 clones described below have hit data in Pfam, it remains unclear as to which of the above-described categories each of these clones belong. However, if data for proteins having a similar domain or motif are accumulated and their functions clarified in more detail, in the future these clones can be classified into any of the above-described categories. The Clone Name and Functional Domain Name are indicated as "Clone Name//Functional Domain Name". When a clone had hit data in multiple functional domains, all data were represented, with each marked with a double slash (//).

In addition, even when a clone had multiple hit data in an identical functional domain, these data are fully represented without abridgment.
BLADE2001031// Thrombospondin type 1 domain
BRACE2010336// TPR Domain
BRACE2013009// EF hand
BRACE2017872// PWWP domain
BRACE2023744// Translationally controlled tumor protein
BRACE2034434// Protein of unknown function
BRACE3001973// EGF-like domain//Laminin G domain
BRACE3002756// SAM domain (Sterile alpha motif)
BRACE3005903// K-box region//TSC-22/dip/bun family
BRACE3014523// Wiskott Aldrich syndrome homology region 2
BRACE3019570// Troponin
BRACE3022340// Troponin
BRACE3026345// Insulin/IGF/Relaxin family
BRACE3036283// DnaJ domain
BRACE3040644// Low-density lipoprotein receptor domain class A//EB module//CUB domain
BRACE3043597// KOW motif
BRACE3046466// EGF-like domain//Laminin G domain//Laminin EGF-like (Domains III and V)//EB module
BRACE3048615// Leucine Rich Repeat
BRALZ2010842// Mitochondrial carrier proteins
BRAMY2031516// wnt family of developmental signaling proteins
BRAMY2041384// Annexin
BRAMY3002886// Domain of unknown function//CBS domain
BRAMY3011501// SAP domain//SPRY domain
BRAMY3015086// FERM domain (Band 4.1 family)
BRAMY3018754// Protein of unknown function//Domain of unknown function
BRAMY4000962// Tudor domain
BRAWH2011796// S-100/ICaBP type calcium binding domain//EF hand
BRAWH2016223// TPR Domain
BRAWH3001783// Cadherin domain
BRAWH3003573// EF hand
BRAWH3008167// Sushi domain (SCR repeat)//CUB domain
BRAWH3011331// Disintegrin
BRAWH3011577// KRAB box
BRAWH3014609// Leucine rich repeat N-terminal domain//Leucine
Rich Repeat//Leucine rich repeat C-terminal domain
BRAWH3021574// Wiskott Aldrich syndrome homology region 2
BRAWH3022347// Leucine Rich Repeat
BRAWH3022719// Eukaryotic initiation factor 4E
BRAWH3024231// TPR Domain
BRAWH3026938// PDZ domain (Also known as DHR or GLGF).
BRAWH3027440// TPR Domain//PPR repeat
BRAWH3030772// Ank repeat
BRAWH3030910// SAM domain (Sterile alpha motif)
BRAWH3033448// TPR Domain
BRAWH3034775// SAP domain//SPRY domain
BRAWH3038252// Formin Homology 2 Domain
BRAWH3038324// Dehydrins
BRAWH3038827// Kelch motif//BTB/POZ domain
BRAWH3042438// 'Paired box' domain//EF hand//Phorbol esters/diacylglycerol binding domain (C1 domain)
BRAWH3042568// Homeobox domain
BRAWH3044151// Thrombospondin type 1 domain//Keratin, high sulfur B2 protein
BRAWH3045118// DnaJ domain
BRAWH3048374// Sushi domain (SCR repeat)//Keratin, high sulfur B2 protein
BRCAN2010665// PDZ domain (Also known as DHR or GLGF).
BRCAN2019907// EF hand
BRCAN2020234// Lipocalin / cytosolic fatty-acid binding protein family
BRCAN2025093// Ank repeat//Flagellar FliJ protein
BRCOC2006639// Leucine Rich Repeat
BRHIP2013958// Domain of unknown function//MSP (Major sperm protein) domain
BRHIP2026346// Formin Homology 2 Domain
BRHIP2027563// Kelch motif
BRHIP3001878// POT family
BRHIP3004710// TPR Domain
BRHIP3005142// Adaptin N terminal region
BRHIP3005231// TPR Domain
BRHIP3006449// Armadillo/beta-catenin-like repeats
BRHIP3007424// wnt family of developmental signaling proteins
BRHIP3009753// CUB domain//Low-density lipoprotein receptor domain class A
BRHIP3010289// Delta serrate ligand//Kelch motif//Plexin repeat//Lectin C-type domain//Laminin EGF-like (Domains III and V)//Keratin, high sulfur B2 protein
BRHIP3020733// Keratin, high sulfur B2 protein
BRHIP3029409// NTR/C345C module
BRHIP3030230// Pentaxin family
BRHIP3033734// Keratin, high sulfur B2 protein
BRSSN2015497// Tudor domain
BRTHA2038345// Ank repeat
BRTHA3011187// EF hand
BRTHA3021971// Putative peptidoglycan binding domain
BRTHA3026161// Adenosine-deaminase (editase) domain
BRTHA3027171// Scorpion short toxins
BRTHA3027638// Matrix protein (MA), p15
CHONS2001287// Insulin-like growth factor binding proteins//Thyroglobulin type-1 repeat
CHONS2001834// Plexin repeat
DFNES2011221// Rotavirus NS26
ERLTF2002178// Kelch motif
FCBBF3012443// Leucine rich repeat N-terminal domain//Leucine
Rich Repeat//Leucine rich repeat C-terminal domain
FCBBF3024911// PWWP domain
FCBBF5000384// BAF60b domain of the SWIB complex
FEBRA2000805// Uncharacterized protein family UPF0054
FEBRA2023498// Leucine rich repeat N-terminal domain//Leucine Rich Repeat
FEKID2002493// wnt family of developmental signaling proteins
HCHON2009766// eIF4-gamma/eIF5/eIF2-epsilon
JCMLC2002751// von Willebrand factor type D domain//Plant PEC family metallothionein//Trypsin Inhibitor like cysteine rich domain//von Willebrand factor type C domain
KIDNE2015987// EGF-like domain//Keratin, high sulfur B2 protein//Zona pellucida-like domain
NT2RI3001573// Leucine Rich Repeat
NT2RI3005923// Cadherin domain
NT2RI3009524// EGF-like domain//Metallothionein//Laminin G domain
NT2RP7007387// Armadillo/beta-catenin-like repeats//picornavirus capsid protein
NT2RP7020343// Transforming growth factor beta like domain//Keratin, high sulfur B2 protein
NT2RP8000633// VPR/VPX protein
NT2RP8001604// CUB domain//Sushi domain (SCR repeat)
NT2RP8006452// African swine fever virus multigene family 360 protein//Leucine Rich Repeat
NT2RP8007920// PPR repeat//LIM domain containing proteins
NT2RP8009119// Picornavirus 2B protein
OCBBF3001202// DENN (AEX-3) domain
OCBBF3005330// Domain found in Dishevelled, Egl-10, and Pleckstrin//TCP-1/cpn60 chaperonin family
OCBBF3023913// R3H domain//Retroviral Vif (Viral infectivity) protein
OCBBF3026979// Laminin G domain//Thrombospondin N-terminal -like domains//von Willebrand factor type C domain//EGF-like domain//EB module//Plant PEC family metallothionein//Trypsin
Inhibitor like cysteine rich domain//Metallothionein
PEBLM2001803// Vacuolar sorting protein 9 (VPS9) domain
PLACE6001933// Receptor L domain
PLACE7002303// Homeobox domain
PUAEN2000594// Poly-adenylate binding protein, unique domain.
PUAEN2000684// Geminivirus AL2 protein//Leucine Rich Repeat
SMINT2010753// TPR Domain//PPR repeat
SPLEN2022785// Polyomavirus coat protein
SPLEN2028417// Homeobox domain
SYNOV2003326// TSC-22/dip/bun family
TBAES2003917// Ank repeat
TBAES2007428// Scorpion short toxins//EGF-like domain
TESOP2002005// E7 protein, Early protein
TESTI2005564// EF hand
TESTI2009739// Tropomyosins//Domain of unknown function
TESTI2011020// Keratin, high sulfur B2 protein
TESTI2018867// FF domain
TESTI2049041// TPR Domain
TESTI4001569// Leucine Rich Repeat//KE2 family protein
TESTI4002141// Keratin, high sulfur B2 protein
TESTI4002868// Metallothionein
TESTI4004031// Domain of unknown function
TESTI4007965// Adaptin N terminal region//Gamma-adaptin, C-terminus
TESTI4011926// Gag P30 core shell protein
TESTI4013742// Leucine Rich Repeat//Hantavirus nucleocapsid protein//Troponin//Formin Homology 2 Domain//Apolipoprotein A1/A4/E family
TESTI4024294// Chorion protein
TESTI4035898// Kelch motif
TESTI4039451// Adaptin N terminal region
TESTI4041984// EGF-like domain//EB module//TB domain
TESTI4043166// Formin Homology 2 Domain
TESTI4046873// TPR Domain
TESTI4047328// von Willebrand factor type D domain//Trypsin
Inhibitor like cysteine rich domain//Chitin binding domain//Metallothionein
TESTI4047569// Keratin, high sulfur B2 protein
TESTI4051015// Major intrinsic protein
TESTI4052598// Lectin C-type domain
THYMU3003007// TPR Domain
THYMU3012402// Armadillo/beta-catenin-like repeats
THYMU3015042// Polyomavirus coat protein
THYMU3015571// Chaperonins 10 Kd subunit
THYMU3017761// Gag P30 core shell protein
THYMU3019476// Matrix protein (MA), p15
THYMU3021755// HCO3- transporter family
THYMU3033649// Immunoreceptor tyrosine-based activation motif
THYMU3040126// Metallothionein
THYMU3046360// F-box domain.
TKIDN2011051// Keratin, high sulfur B2 protein
TKIDN2011160// Thrombospondin type 1 domain
TLIVE2007736// PDZ domain (Also known as DHR or GLGF).
TRACH3007689// Ank repeat//TPR Domain
TRACH3012106// FERM domain (Band 4:1 family)
TRACH3015346// Uncharacterized protein family UPF0004
TRACH3016805// Ank repeat
TRACH3018606// SAM domain (Sterile alpha motif)
TRACH3022296// DnaJ domain
TRACH3022758// EF hand
TRACH3023203// Flavivirus polyprotein propeptide
TRACH3028855// R3H domain//Uncharacterized protein family UPF0024
TRACH3030855// Serpins (serine protease inhibitors)
TRACH3032570// PDZ domain (Also known as DHR or GLGF).
UTERU2016669// Helix-hairpin-helix motif.
UTERU3001394// EGF-like domain
UTERU3009775// PDZ domain (Also known as DHR or GLGF).
UTERU3011558// GTPase of unknown function
UTERU3011579// Plant PEC family metallothionein
UTERU3017995// UBX domain
UTERU3018255// Thrombospondin type 1 domain
UTERU3021850// Thrombospondin type 1 domain//DnaJ central domain (4 repeats)

Likewise, although seven clones described below have hit data in Pfam (see Example 5), it remains unclear as to which of the above-described categories each of the clones belong. However, if data for proteins comprising a similar domain or motif are accumulated and their functions are clarified in more detail, in the future these clones can be classified into any of the categories described above.
BRACE3026993// TSC-22/dip/bun family
BRACE3046450// PDZ domain (Also known as DHR or GLGF).//PDZ domain (Also known as DHR or GLGF).//PDZ domain (Also known as
DHR or GLGF). 1//PDZ domain (Also known as DHR or GLGF).
CTONG2003764// Phorbol esters/diacylglycerol binding domain (C1 domain)
TBAES2004105// Thrombospondin type 1 domain
TBAES2007379// EGF-like domain//EGF-like domain//Trypsin Inhibitor like cysteine rich domain//EGF-like domain//EGF-like domain//Keratin, high sulfur B2 protein//EGF-like domain//EGF-like domain//Granulins//EGF-like domain//EGF-like domain//EGF-like domain
TBAES2008133// PDZ domain (Also known as DHR or GLGF).
UTERU2036507// Thrombospondin type 1 domain//NTR/C345C module

In addition, when data for proteins are accumulated and novel domains and motifs are found, in the future the remaining clones, which had no hit data in the search with Pfam, can be classified into any of the above-described categories if a new functional domain or motif is identified by re-analyzing the deduced amino acid sequences of the clones using a homology search against an updated database.

### EXAMPLE 8

### Expression frequency analysis in silico

The cDNA libraries derived from various tissues and cells as indicated in Example 1 were prepared, and cDNA clones were selected from each library at random. The 5'-end sequences were determined and the database was constructed based on the data. The database was constructed based on the nucleotide sequences of 1,402,069 clones, and thus the population of the database is large enough for the analysis.

Then, clones having a homologous sequence are categorized into a single cluster (clustering) by searching the nucleotide sequences of respective clones in this database with the program of nucleotide sequence homology search; the number of clones belonging to each cluster was determined and normalized for every library; thus, the ratio of a certain gene in each cDNA library was determined. This analysis gave the information of the expression frequency of genes in tissues and cells which were sources of the cDNA libraries.

Then, in order to analyze the expression of a gene containing the nucleotide sequence of the cDNA of the present invention in tissues and cells, the library derived from a tissue or a cell used in the large-scale cDNA analysis was subjected to the comparison of the expression levels between tissues or cells. Namely, the expression frequency was analyzed by comparing the previously normalized values between tissues and/or cells for which the nucleotide sequences of 600 or more cDNA clones had been analyzed. By this analysis, some of the genes were revealed to be involved in the pathology and functions indicated below. Each value in Tables 2 to 24 shown below represents a relative expression frequency; the higher the value, the higher the expression level. The genes which are included a part of the Tables indicate not so big difference between compared libraries, but when compared with other libraries from another tissue or cell based on Example 9, they indicate significant difference. Thus, the genes are specific in each tissue or cell, and can be considered to be useful as diagnosing markers for the disease as well as useful for analyzing molecular mechanisms.

### Osteoporosis-related genes

Osteoporosis is a pathology in which bones are easily broken owing to overall decrease in components of bone. The onset involves the balance between the functions of osteoblast producing bone and osteoclast absorbing bone, namely bone metabolism. Thus, the genes involved in the increase of osteoclasts differentiating from precursor cells of monocyte/macrophage line (Molecular Medicine 38. 642-648. (2001)) are genes involved in osteoporosis relevant to bone metabolism.

A nucleotide sequence information-based analysis was carried out to identify the genes whose expression frequencies are higher or lower in CD34+ cell (cell expressing a glycoprotein CD34) treated with the osteoclast differentiation factor (Molecular Medicine 38. 642-648. (2001)) than in the untreated CD34+ cell, which is the precursor cell of monocyte/macrophage line. The result of comparative analysis for the frequency between the two cDNA libraries prepared from the RNA of CD34+ cells (CD34C) and from the RNA of CD34+ cells treated with the osteoclast differentiation factor (D30ST, D60ST or D9OST) showed that the genes whose expression levels were different between the two were the following twelve clones (Table 2).
BRAWH3018063, BRHIP3020046, BRSSN2013696, BRSTN2012069, BRTHA2027229, D9OST2003106, D9OST2003989, D9OST2004417 OCBBF2016928, TESTI4005653, TESTI4013474, THYMU3032798

These genes are involved in osteoporosis.

### Genes involved in neural cell differentiation

Genes involved in neural cell differentiation are useful for treating neurological diseases. Genes with varying expression levels in response to induction of cellular differentiation in neural cells are thought to be involved in neurological diseases.

A survey was performed for genes whose expression levels are varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and the cells subjected to the differentiation treatment (NT2RP, NT2RI or NT2NE) showed that the genes whose expression levels were different between the two were the following 102 clones (Table 3).
BLADE2004849, BRACE2003628, BRACE2012528, BRAMY2023939, BRAMY2031516, BRAMY4002628, BRAWH3010461, BRAWH3017259, BRAWH3018063, BRAWH3022651, BRAWH3024186, BRCAN2019653, BRCAN2022126, BRCOC2012386, BRHIP3002000, BRHIP3007223, BRHIP3021019, BRSTN2011961, BRSTN2012069, BRTHA2033155, BRTHA3010212, BRTHA3011194, BRTHA3011265, BRTHA3017791, BRTHA3021971, CHONS2002829, CTONG2006235, FCBBF3012443, FEBRA2026582, LIVER2008465, NT2NE2011107, NT2NE2016041, NT2RI2004818, NT2RI2009233, NT2RI2010795, NT2RI2015533, NT2RI2023671, NT2RI2028537, NT2RI3001573, NT2RI3001967, NT2RI3005861, NT2RI3005923, NT2RI3007095, NT2RI3008179, NT2RI3009480, NT2RI3009524, NT2RP7003439, NT2RP7007387, NT2RP7014178, NT2RP7014778, NT2RP7016508, NT2RP7017139, NT2RP7019682, NT2RP7020343, NT2RP8000633, NT2RP8001363, NT2RP8001407, NT2RP8001584, NT2RP8001604, NT2RP8001605, NT2RP8003490, NT2RP8003657, NT2RP8003787, NT2RP8005546, NT2RP8006452, NT2RP8006521, NT2RP8007416, NT2RP8007503, NT2RP8007920, NT2RP8008057, NT2RP8009119, NT2RP8009248, NTONG2008483, OCBBF2003518, OCBBF3001333, OCBBF3004908, PLACE7004103, PROST2017910, SMINT2009292, SPLEN2012571, T1ESE2000904, TESTI4002072, TESTI4002774, TESTI4002799, TESTI4005653, TESTI4007965, TESTI4012960, TESTI4018436, THYMU3001776, THYMU3002887, THYMU3029795, THYMU3041428, THYMU3047115, TRACH3003872, TRACH3004424, TRACH3006717, TRACH3007625, TRACH3009008, TRACH3016805, TRACH3016885, TRACH3026303, UTERU2016669

These genes are neurological disease-related genes.

### Genes involved in Alzheimer's disease

Alzheimer's disease is a cranial neurological disease that is characterized by memory loss. As the disease advances, patients can no longer support themselves and require nursing. Alzheimer's disease eventually leads to atrophication of the brain itself. Environmental factors such as stress, and vascular factors such as hypertension and cholesterolemia, are assumed, but not confirmed, to contribute to the onset of Alzheimer's disease. Genes whose expression levels differ between normal brain tissues and tissues affected with Alzheimer's disease are expected to be involved in Alzheimer's disease. Such genes can be used to elucidate the disease's onset mechanism and in genetic diagnosis. cDNA libraries derived from the cerebral cortex of Alzheimer patients (BRALZ and BRASW), and a library derived from whole tissues of a normal brain (BRAWH) were analyzed and compared (Table 4). The results showed that genes whose expression levels differed between the two are the following 298 and five clones listed below.
ASTRO2016114, BRACE2002392, BRACE2012528, BRACE3004371, BRACE3004767, BRACE3022340, BRACE3031185, BRACE3031743, BRACE3032385, BRACE3032631, BRACE3039358, BRACE3040863, BRACE3042432, BRACE3045981, BRALZ2003119, BRALZ2007661, BRALZ2008930, BRALZ2010842, BRALZ2011337, BRALZ2013621, BRALZ2013690, BRALZ2014054, BRAMY2031516, BRAMY3002329, BRAMY3004126, BRAMY3005912, BRAMY3008436, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019653, BRCAN2022126, BRCAN2025093, BRCOC2012386, BRHIP2015153, BRHIP2027077, BRHIP3001573, BRHIP3002000, BRHIP3003063, BRHIP3003984, BRHIP3004774, BRHIP3007223, BRHIP3007409, BRHIP3008320, BRHIP3014675, BRHIP3017855, BRHIP3018784, BRHIP3021019, BRHIP3028246, BRHIP3028570, BRSTN2010089, BRSTN2012069, BRSTN2016992, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, CTONG2006235, CTONG2009033, CTONG2020582, D9OST2003106 DFNES2001829, KIDNE2010049, MESAN2017133, NT2RI2009233, NT2RI2015533, NT2RI3005923, NT2RI3009524, NT2RP7007387, NT2RP8001605, NT2RP8007920, NTONG2008483, OCBBF2003518, OCBBF2018618, OCBBF3001333, OCBBF3004487, PLACE7004103, PLACE7006240, PROST2007444, PROST2017910, TBAES2007428, TESTI2005112, TESTI2021654, TESTI4002072, TESTI4002774, TESTI4002799, TESTI4003602, TESTI4004210, TESTI4005399, TESTI4005653, TESTI4006441, TESTI4013474, TESTI4014908, TESTI4022158, THYMU3000776, THYMU3002887, THYMU3003350, THYMU3021586, THYMU3026000, THYMU3026306, THYMU3026350, THYMU3032798, THYMU3032867, THYMU3037827, THYMU3038214, THYMU3041428, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAWH3013197, BRAWH3028645, BRTHA3004432, TRACH1000193

These genes are involved in Alzheimer's disease. Genes involved in Parkinson's disease

Parkinson's disease is a cranial neurological disease characterized by impaired production of the neurotransmitter dopamine in the substantia nigra in the brain. This results in dyskinesia, such as hand tremors, and impaired body movement due to muscular rigidity. Normally, the number of brain neurons gradually decreases with age. However, compared to healthy people, patients with Parkinson's disease experience a rapid and marked decrease in the number of neurons in their substantia nigra. Genes whose expression levels differ between tissues of the whole brain and the nigra are expected to be involved in Parkinson's disease. These genes exhibit nigra-specific alterations in their expression levels, and can be used to elucidate the disease onset mechanism and in gene diagnosis. cDNA libraries derived from the substantia nigra (BRSSN) and a library derived from whole tissues of a normal brain (BRAWH) were analyzed and compared (Table 5). Genes whose expression levels differed between the two were the 305 clones and five clones listed below.
ASTRO2016114, BRACE2012528, BRACE2017844, BRACE3004371, BRACE3004767, BRACE3022340, BRACE3025719, BRACE3026802, BRACE3031185, BRACE3031743, BRACE3032385, BRACE3032631, BRACE3039358, BRACE3040863, BRACE3042432, BRACE3045981, BRAMY2031516, BRAMY3002329, BRAMY3004126, BRAMY3005912, BRAMY3008436, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2022126, BRCAN2025093, BRCOC2012386, BRHIP2015153, BRHIP2027077, BRHIP2029643, BRHIP3001573, BRHIP3002000, BRHIP3003063, BRHIP3003984, BRHIP3004774, BRHIP3007223, BRHIP3007409, BRHIP3008320, BRHIP3014675, BRHIP3017855, BRHIP3018784, BRHIP3020046, BRHIP3021019, BRHIP3028246, BRHIP3028570, BRSSN2004303, BRSSN2004710, BRSSN2008464, BRSSN2011843, BRSSN2012157, BRSSN2012198, BRSSN2013696, BRSSN2015497, BRSSN2018218, BRSTN2010089, BRSTN2011961, BRSTN2012069, BRSTN2016992, BRSTN2017104, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, CTONG2006235, CTONG2009033, CTONG2011801, CTONG2020582, D9OST2003106 DFNES2001829, KIDNE2010049, MESAN2017133, NT2RI2009233, NT2RI2015533, NT2RI3005923, NT2RI3009524, NT2RP7007387, NT2RP8001605, NT2RP8007920, NTONG2008483, OCBBF2003518, OCBBF2018618, OCBBF3001333, OCBBF3004487, PLACE7004103, PLACE7006240, PROST2007444, SMINT2009292, T1ESE2000904, TBAES2007428, TESTI2005112, TESTI2021654, TESTI4002072, TESTI4002774, TESTI4002799, TESTI4003602, TESTI4004210, TESTI4005399, TESTI4005653, TESTI4006441, TESTI4014908, TESTI4022158, THYMU3000776, THYMU3002887, THYMU3003350, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3022198, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAWH3013197, BRAWH3028645, BRTHA3004432, TRACH1000193

These genes are involved in Parkinson's disease.

### Genes involved in short-term memory and dementia

In the brain, the hippocampus is a highly important memory-related area. The hippocampus functions to establish a memory by judging whether acquired information is necessary, and then accumulating the memory in another area of the brain. According to clinical findings, patients can retain a new memory for only about five minutes with an abnormal, or at the worst without a hippocampus. Some dementia patients are presumed to have hippocampus abnormalities. Thus, genes whose expression levels differ between tissues of the whole brain and the hippocampus are expected to be involved in memory or dementia. Such genes can be used to elucidate the mechanism underlying the memory and in gene diagnosis. cDNA libraries derived from the hippocampus (BRHIP) and from the whole tissues of a normal brain (BRAWH) were analyzed and compared (Table 6). Genes whose expression levels differed between the two were the 438 clones and five clones listed below.
ASTRO2016114, BRACE2002392, BRACE2012528, BRACE2017359, BRACE2017397, BRACE2017844, BRACE3004046, BRACE3004371, BRACE3004767, BRACE3009416, BRACE3022340, BRACE3027931, BRACE3029021, BRACE3031185, BRACE3031743, BRACE3032385, BRACE3032631, BRACE3036156, BRACE3039358, BRACE3040863, BRACE3042326, BRACE3042432, BRACE3045078, BRACE3045981, BRAMY2031516, BRAMY3002329, BRAMY3004126, BRAMY3005184, BRAMY3005912, BRAMY3007078, BRAMY3008436, BRAMY4000915, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019953, BRCAN2022126, BRCAN2025093, BRCOC2012386, BRHIP2006819, BRHIP2006921, BRHIP2008756, BRHIP2009177, BRHIP2011199, BRHIP2013958, BRHIP2015153, BRHIP2016125, BRHIP2017714, BRHIP2020930, BRHIP2021929, BRHIP2023735, BRHIP2024941, BRHIP2026346, BRHIP2027077, BRHIP2027563, BRHIP2029529, BRHIP2029643, BRHIP2029663, BRHIP3000626, BRHIP3000859, BRHIP3001076, BRHIP3001141, BRHIP3001338, BRHIP3001360, BRHIP3001481, BRHIP3001573, BRHIP3001878, BRHIP3002000, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003688, BRHIP3003795, BRHIP3003845, BRHIP3003961, BRHIP3003984, BRHIP3004215, BRHIP3004710, BRHIP3004725, BRHIP3004774, BRHIP3004786, BRHIP3005037, BRHIP3005142, BRHIP3005231, BRHIP3005307, BRHIP3005673, BRHIP3005801, BRHIP3005944, BRHIP3006279, BRHIP3006294, BRHIP3006449, BRHIP3006786, BRHIP3006950, BRHIP3007172, BRHIP3007195, BRHIP3007223, BRHIP3007291, BRHIP3007409, BRHIP3007424, BRHIP3007609, BRHIP3007960, BRHIP3008082, BRHIP3008320, BRHIP3008714, BRHIP3009672, BRHIP3009753, BRHIP3010289, BRHIP3010916, BRHIP3011082, BRHIP3011269, BRHIP3011460, BRHIP3011567, BRHIP3011831, BRHIP3012185, BRHIP3012289, BRHIP3012357, BRHIP3012736, BRHIP3012997, BRHIP3013078, BRHIP3013588, BRHIP3013698, BRHIP3014675, BRHIP3015854, BRHIP3016032, BRHIP3016421, BRHIP3017109, BRHIP3017146, BRHIP3017256, BRHIP3017558, BRHIP3017855, BRHIP3018784, BRHIP3019643, BRHIP3019824, BRHIP3019880, BRHIP3019956, BRHIP3020046, BRHIP3020155, BRHIP3020733, BRHIP3021019, BRHIP3021499, BRHIP3021987, BRHIP3022656, BRHIP3023922, BRHIP3024703, BRHIP3024820, BRHIP3025795, BRHIP3025844, BRHIP3026231, BRHIP3026651, BRHIP3027160, BRHIP3027191, BRHIP3027651, BRHIP3027947, BRHIP3028246, BRHIP3028570, BRHIP3028742, BRSTN2010089, BRSTN2012069, BRSTN2016992, BRTHA2001953, BRTHA2008502, BRTHA2031517, BRTHA2033155, BRTHA2035743, BRTHA3003417, BRTHA3003736, BRTHA3005988, BRTHA3007662, BRTHA3010135, BRTHA3010212, BRTHA3011194, BRTHA3011265, BRTHA3012265, BRTHA3017791, BRTHA3020771, BRTHA3021971, BRTHA3023403, CHONS2002829, CTONG2006235, CTONG2009033, CTONG2020582, D9OST2003106 DFNES2001829, KIDNE2010049, LIVER2008465, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2000831, OCBBF2003518, OCBBF2018618, OCBBF3001333, OCBBF3004487, PLACE7004103, PLACE7006240, PROST2007444, SMINT2012179, SYNOV4004210, TBAES2007428, TESTI2005112, TESTI2005564, TESTI2021654, TESTI4001569, TESTI4002072, TESTI4002774, TESTI4002799, TESTI4003602, TESTI4003703, TESTI4003944, TESTI4004210, TESTI4005399, TESTI4005653, TESTI4006441, TESTI4014908, TESTI4022158, TESTI4029297, THYMU3000776, THYMU3002887, THYMU3003007, THYMU3003350, THYMU3007308, THYMU3008105, THYMU3021586, THYMU3026000, THYMU3026306, THYMU3026350, THYMU3032798, THYMU3032867, THYMU3034671, THYMU3037827, THYMU3038214, THYMU3044075, TKIDN2000319, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3022198, TRACH3024342, TRACH3024671, TRACH3025316, TRACH3026303, TRACH3026676, TRACH3028855, TRACH3032570, TRACH3036932, TRACH3038399, TSTOM2000235, UTERU3005422, UTERU3010409, UTERU3013167, UTERU3016273
BRACE3046450, BRAWH3013197, BRAWH3028645, BRTHA3004432, TRACH1000193

These genes are involved in memory and dementia.

### Genes involved in equilibrium sense and movement function

The cerebellum is the center of equilibrium sense, muscular movement, and motor learning. This area is thought to be involved in motor control, and smooth movements are achieved unconsciously due to cerebellum action. Recent studies have elucidated that the cerebellum participates in not only simple movements but also in establishing higher-order movements such as reading and writing. Thus, genes whose expression levels differ between tissues of the whole brain and the cerebellum are expected to be involved in equilibrium sense or motor function, which can be useful for elucidating the molecular mechanism controlled by the brain. cDNA libraries derived from the cerebellum (BRACE) and from the whole tissues of a normal brain (BRAWH) were analyzed and compared (Table 7). Genes whose expression levels differed between the two were the 502 clones and nine clones listed below.
ASTRO2016114, BRACE1000475, BRACE2002392, BRACE2003628, BRACE2005991, BRACE2010336, BRACE2012528, BRACE2012625, BRACE2012833, BRACE2012838, BRACE2012936, BRACE2012947, BRACE2013009, BRACE2013126, BRACE2013132, BRACE2016896, BRACE2017359, BRACE2017397, BRACE2017580, BRACE2017844, BRACE2017872, BRACE2017992, BRACE2019348, BRACE2023633, BRACE2023744, BRACE2025452, BRACE2026404, BRACE2027312, BRACE2027382, BRACE2028956, BRACE2030039, BRACE2032584, BRACE2033128, BRACE2034434, BRACE2035120, BRACE2035191, BRACE2039362, BRACE2039607, BRACE2042541, BRACE2046976, BRACE2047232, BRACE2047975, BRACE3001403, BRACE3001973, BRACE3002344, BRACE3002541, BRACE3002756, BRACE3003866, BRACE3004046, BRACE3004371, BRACE3004767, BRACE3004887, BRACE3004981, BRACE3005870, BRACE3005903, BRACE3006553, BRACE3007649, BRACE3007869, BRACE3009075, BRACE3009265, BRACE3009392, BRACE3009416, BRACE3009539, BRACE3010702, BRACE3011447, BRACE3011774, BRACE3013418, BRACE3013874, BRACE3013986, BRACE3014523, BRACE3014714, BRACE3015090, BRACE3015898, BRACE3016020, BRACE3016167, BRACE3016580, BRACE3016788, BRACE3016810, BRACE3016862, BRACE3017253, BRACE3018083, BRACE3019570, BRACE3019611, BRACE3019817, BRACE3019941, BRACE3020356, BRACE3020669, BRACE3021430, BRACE3021517, BRACE3021805, BRACE3022051, BRACE3022303, BRACE3022312, BRACE3022340, BRACE3022847, BRACE3023604, BRACE3024379, BRACE3024444, BRACE3024497, BRACE3024537, BRACE3024879, BRACE3025627, BRACE3025719, BRACE3026161, BRACE3026290, BRACE3026345, BRACE3026456, BRACE3026802, BRACE3026844, BRACE3026947, BRACE3027256, BRACE3027931, BRACE3028360, BRACE3028895, BRACE3028998, BRACE3029005, BRACE3029021, BRACE3029205, BRACE3029447, BRACE3030538, BRACE3031161, BRACE3031184, BRACE3031185, BRACE3031315, BRACE3031372, BRACE3031579, BRACE3031728, BRACE3031743, BRACE3031843, BRACE3032385, BRACE3032537, BRACE3032538, BRACE3032631, BRACE3032980, BRACE3033525, BRACE3034183, BRACE3034389, BRACE3034964, BRACE3034993, BRACE3035168, BRACE3036156, BRACE3036271, BRACE3036283, BRACE3037612, BRACE3037637, BRACE3037803, BRACE3038012, BRACE3038030, BRACE3038570, BRACE3038760, BRACE3039288, BRACE3039358, BRACE3039378, BRACE3039454, BRACE3040012, BRACE3040239, BRACE3040504, BRACE3040644, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044495, BRACE3045078, BRACE3045145, BRACE3045424, BRACE3045708, BRACE3045981, BRACE3046049, BRACE3046152, BRACE3046294, BRACE3046466, BRACE3046491, BRACE3046609, BRACE3046837, BRACE3046855, BRACE3046966, BRACE3047018, BRACE3047482, BRACE3047801, BRAMY2031516, BRAMY3002329, BRAMY3004126, BRAMY3004364, BRAMY3005912, BRAMY3008436, BRAMY3009491, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH30274.40, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2019953, BRCAN2022126, BRCAN2025093, BRCOC2012386, BRHIP2015153, BRHIP2027077, BRHIP2029643, BRHIP3001360, BRHIP3001573, BRHIP3002000, BRHIP3002114, BRHIP3003063, BRHIP3003126, BRHIP3003961, BRHIP3003984, BRHIP3004215, BRHIP3004774, BRHIP3005801, BRHIP3007223, BRHIP3007409, BRHIP3008320, BRHIP3014675, BRHIP3017855, BRHIP3018784, BRHIP3021019, BRHIP3028246, BRHIP3028570, BRSSN2011843, BRSSN2013696, BRSTN2010089, BRSTN2011961, BRSTN2012069, BRSTN2016992, BRTHA2033155, BRTHA2035743, BRTHA3003736, BRTHA3005988, BRTHA3009858, BRTHA3010135, BRTHA3010212, BRTHA3010530, BRTHA3011194, BRTHA3011265, BRTHA3011998, BRTHA3017791, BRTHA3020771, BRTHA3021708, BRTHA3021971, BRTHA3023403, CHONS2002829, CTONG2006235, CTONG2009033, CTONG2020582, CTONG2027959, D9OST2003106, DFNES2001829, KIDNE2010049, KIDNE2017153, LIVER2008465, MESAN2017133, NOVAR2000783, NT2RI2009233, NT2RI2015533, NT2RI3005923, NT2RI3009524, NT2RP7007387, NT2RP8001605, NT2RP8007920, NT2RP8009119, NTONG2008483, NTONG2009468, OCBBF2003518, OCBBF2014745, OCBBF2018618, OCBBF3001333, OCBBF3004487, PLACE7004103, PLACE7006240, PROST2007444, TBAES2007428, TESTI2005112, TESTI2018867, TESTI2021654, TESTI4002072, TESTI4002774, TESTI4002799, TESTI4003602, TESTI4003703, TESTI4004210, TESTI4005013, TESTI4005399, TESTI4005653, TESTI4006441, TESTI4014908, TESTI4022158, TESTI4029297, TESTI4032913, TESTI4043223, THYMU3000776, THYMU3002887, THYMU3003350, THYMU3007308, THYMU3021586, THYMU3026000, THYMU3026306, THYMU3026350, THYMU3032798, THYMU3032867, THYMU3033626, THYMU3034671, THYMU3037827, THYMU3038214, THYMU3044075, TLIVE2007736, TRACH2013585, TRACH3002752, TRACH3003037, TRACH3003872, TRACH3004424, TRACH3006717, TRACH3007625, TRACH3007689, TRACH3007995, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3000670, UTERU3010409, UTERU3013167, UTERU3015011
BRACE3002184, BRACE3026993, BRACE3046450, BRAMY3008096, BRAWH3013197, BRAWH3028645, BRTHA3004432, TESTI4002988, TRACH1000193

These genes are involved in equilibrium sense or motor function.

### Genes involved in signaling from sensory organs

The thalamus is an area which comprises many neurons strongly connected to the cerebrum, and which transmits sensory information from the spinal cord or such to the responsible area of the cerebrum. The thalamus also controls the direction of movement from the cerebrum. For example, the thalamus resolves vision into the elements of size, shape, and color, and resolves sound into volume and sweetness or harshness to the ear, and then transmits this information to the sensory area of the cerebral cortex. Thus, genes whose expression levels differ between tissues of the whole brain and the thalamus are expected to be involved in signaling from sensory organs. These genes can be used to elucidate the molecular mechanism underlying signaling controlled by the brain. cDNA libraries derived from the thalamus (BRTHA) and from whole tissues of a normal brain (BRAWH) were analyzed and compared (Table 8). Genes whose expression levels differed between the two were the 440 clones and eight clones listed below.
ASTRO2008972, ASTRO2016114, BLADE2004849, BRACE2002392, BRACE2012528, BRACE2019348, BRACE3004371, BRACE3004767, BRACE3019941, BRACE3022312, BRACE3022340, BRACE3031185, BRACE3031743, BRACE3032385, BRACE3032631, BRACE3036156, BRACE3039358, BRACE3040863, BRACE3042432, BRACE3045981, BRAMY2031516, BRAMY3002329, BRAMY3004126, BRAMY3005912, BRAMY3008436, BRAMY3009556, BRAMY3010654, BRAMY4001863, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2002892, BRCAN2010665, BRCAN2020234, BRCAN2022126, BRCAN2025093, BRCOC2006164, BRCOC2012386, BRHIP2013958, BRHIP2015153, BRHIP2027077, BRHIP3001573, BRHIP3002000, BRHIP3002691, BRHIP3002920, BRHIP3003063, BRHIP3003961, BRHIP3003984, BRHIP3004215, BRHIP3004774, BRHIP3007223, BRHIP3007409, BRHIP3008320, BRHIP3014675, BRHIP3017855, BRHIP3018784, BRHIP3020046, BRHIP3021019, BRHIP3028246, BRHIP3028570, BRSSN2015497, BRSTN2010089, BRSTN2011961, BRSTN2012069, BRSTN2016992, BRTHA2000969, BRTHA2001304, BRTHA2001953, BRTHA2002091, BRTHA2003759, BRTHA2005448, BRTHA2006720, BRTHA2008502, BRTHA2008598, BRTHA2010672, BRTHA2012189, BRTHA2014647, BRTHA2018304, BRTHA2019726, BRTHA2019743, BRTHA2020400, BRTHA2020566, BRTHA2020642, BRTHA2020695, BRTHA2020721, BRTHA2020781, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2028297, BRTHA2029969, BRTHA2030036, BRTHA2030213, BRTHA2031517, BRTHA2031917, BRTHA2032763, BRTHA2033122, BRTHA2033155, BRTHA2033320, BRTHA2033469, BRTHA2033683, BRTHA2034281, BRTHA2034576, BRTHA2035743, BRTHA2036055, BRTHA2036295, BRTHA2037247, BRTHA2038279, BRTHA2038345, BRTHA2038353, BRTHA3000456, BRTHA3002411, BRTHA3003225, BRTHA3003417, BRTHA3003736, BRTHA3005988, BRTHA3006593, BRTHA3007469, BRTHA3007662, BRTHA3009858, BRTHA3010135, BRTHA3010212, BRTHA3010469, BRTHA3010530, BRTHA3010540, BRTHA3010717, BRTHA3011187, BRTHA3011194, BRTHA3011229, BRTHA3011265, BRTHA3011306, BRTHA3011361, BRTHA3011510, BRTHA3011892, BRTHA3011998, BRTHA3012265, BRTHA3013860, BRTHA3013882, BRTHA3014000, BRTHA3014105, BRTHA3014507, BRTHA3014547, BRTHA3014835, BRTHA3014854, BRTHA3014920, BRTHA3016616, BRTHA3017791, BRTHA3018409, BRTHA3018623, BRTHA3019183, BRTHA3020369, BRTHA3020771, BRTHA3021569, BRTHA3021708, BRTHA3021786, BRTHA3021971, BRTHA3022641, BRTHA3023403, BRTHA3023590, BRTHA3023929, BRTHA3024600, BRTHA3025073, BRTHA3026161, BRTHA3026180, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, CHONS2002829, CTONG2006235, CTONG2009033, CTONG2011801, CTONG2020582, D9OST2003106, DFNES2001829, KIDNE2010049, LIVER2008465, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2003518, OCBBF2009536, OCBBF2018618, OCBBF3001333, OCBBF3004487, OCBBF3008835, PLACE6003004, PLACE7004103, PLACE7006240, PROST2007444, SMINT2009292, TBAES2007428, TESTI2005112, TESTI2021654, TESTI2039342, TESTI4002072, TESTI4002774, TESTI4002799, TESTI4003602, TESTI4003703, TESTI4004210, TESTI4004695, TESTI4005399, TESTI4005653, TESTI4006441, TESTI4007965, TESTI4010979, TESTI4013474, TESTI4014908, TESTI4022158, TESTI4029297, TESTI4032913, THYMU3000776, THYMU3002887, THYMU3003350, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008508, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3012414, UTERU3013167, UTERU3017995, UTERU3018172 BRACE3046450, BRAWH3013197, BRAWH3028645, BRTHA3004432, BRTHA3024233, CTONG2002832, THYMU3046350, TRACH1000193

These genes are involved in signaling from sensory organs.

### Genes involved in emotional reaction

The amygdala is the center of emotion in the brain. Information passing through the amygdala induces an emotional reaction, for example, panic or fear. When a strong fear reaction is produced due to the emotional evaluation of stimulus in the amygdala, the amygdala transmits an alert signal to each area of the brain. This results in various reactions such as sweating palms, palpitation, elevated blood pressure, and rapid secretion of adrenaline. In other words, the amygdala transmits signals which cause the body to be on the alert and is a tissue involved in a kind of defense instinct. Thus, genes whose expression levels differ between tissues of the whole brain and the amygdala are expected to be involved in emotional reaction. Such genes can be used to elucidate the molecular mechanism underlying emotional reaction, fear, or panic. cDNA libraries derived from the amygdale (BRAMY) and from whole tissues of a normal brain (BRAWH) were analyzed and compared (Table 9). Genes whose expression levels differed between the two were the 357 clones and nine clones listed below.
ASTRO2016114, BRACE2002392, BRACE2012528, BRACE2017397, BRACE2017844, BRACE3004371, BRACE3004767, BRACE3022340, BRACE3031185, BRACE3031315, BRACE3031743, BRACE3032385, BRACE3032631, BRACE3039358, BRACE3040863, BRACE3042432, BRACE3045981, BRAMY2015516, BRAMY2021098, BRAMY2022320, BRAMY2023939, BRAMY2025495, BRAMY2031516, BRAMY2033895, BRAMY2035801, BRAMY2036254, BRAMY2036266, BRAMY2037609, BRAMY2039630, BRAMY2040915, BRAMY2041347, BRAMY2041384, BRAMY2041507, BRAMY2044686, BRAMY2046489, BRAMY2046537, BRAMY3000692, BRAMY3001409, BRAMY3002329, BRAMY3002508, BRAMY3002886, BRAMY3004126, BRAMY3004364, BRAMY3005184, BRAMY3005656, BRAMY3005912,. BRAMY3007078, BRAMY3007449, BRAMY3007471, BRAMY3008436, BRAMY3009158, BRAMY3009491, BRAMY3009556, BRAMY3009904, BRAMY3010321, BRAMY3010603, BRAMY3010654, BRAMY4000915, BRAMY4000962, BRAMY4001234, BRAMY4001652, BRAMY4001863, BRAMY4001913, BRAMY4002575,. BRAMY4002628, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259,
BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2010665, BRCAN2022126, BRCAN2025093, BRCOC2012386, BRHIP2015153, BRHIP2027077, BRHIP3001573, BRHIP3002000, BRHIP3002691, BRHIP3003063, BRHIP3003984, BRHIP3004215, BRHIP3004774, BRHIP3005673, BRHIP3007223, BRHIP3007409, BRHIP3008320, BRHIP3012736, BRHIP3014675, BRHIP3017146, BRHIP3017855, BRHIP3018784, BRHIP3020046, BRHIP3021019, BRHIP3028246, BRHIP3028570, BRSTN2010089, BRSTN2012069, BRSTN2016992, BRTHA2026071, BRTHA2033155, BRTHA3003736, BRTHA3005988, BRTHA3010135, BRTHA3010212, BRTHA3011194, BRTHA3011265, BRTHA3017791, BRTHA3020771, BRTHA3021971, BRTHA3023403, BRTHA3026916, CHONS2002829, CTONG2006235, CTONG2009033, CTONG2020582, D9OST2003106, DFNES2001829, KIDNE2010049, MESAN2017133, NT2RI2009233, NT2RI2010795, NT2RI2015533, NT2RI3005923, NT2RI3009524, NT2RP7007387, NT2RP8001605, NT2RP8007920, NTONG2008483, OCBBF2000831, OCBBF2003518, OCBBF2018618, OCBBF2030927, OCBBF3001333, OCBBF3004487, OCBBF3009244, PLACE6008315, PLACE6010936, PLACE7004103, PLACE7006240, PROST2007444, SPLEN2012571, SYNOV4004210, SYNOV4009575, TBAES2007428, TESTI2005112, TESTI2021654, TESTI4002072, TESTI4002774, TESTI4002799, TESTI4003602, TESTI4003703, TESTI4004210, TESTI4005399, TESTI4005653, TESTI4006441, TESTI4014908, TESTI4022158, TESTI4029297, TESTI4032913, TESTI4043223, TESTI4046073, THYMU3000776, THYMU3002887, THYMU3003350, THYMU3008105, THYMU3019476, THYMU3021586, THYMU3026000, THYMU3026306, THYMU3026350, THYMU3032798, THYMU3032867, THYMU3037827, THYMU3038214, THYMU3044075, TRACH2013585, TRACH3002752, TRACH3003037, TRACH3003872, TRACH3004424, TRACH3006717, TRACH3007625, TRACH3007689, TRACH3008632, TRACH3009008, TRACH3010079, TRACH3016805, TRACH3016885, TRACH3024342, TRACH3024671, TRACH3026303, TRACH3026676, TRACH3028855, TRACH3032570, TRACH3036932, TRACH3038399, UTERU3010409, UTERU3013167
BRACE3046450, BRAMY3008096, BRAWH3013197, BRAWH3028645, BRTHA3004432, TESTI4002988, THYMU3046350, TRACH1000193, TRACH3019290

These genes are involved in emotional reaction.

### Cancer-related genes

Cancer tissues are assumed to express a distinct set of genes distinct from normal tissues, and thus expression of these genes can contribute to carcinogenesis in tissues and cells. Thus, genes whose expression patterns in cancer tissues differ from those in normal tissues are cancer-related genes. A search was carried out for genes whose expression levels in cancer tissues differed from those in normal tissues.

The result of comparative analysis of cDNA libraries derived from breast tumor (TBAES) and normal breast (BEAST) (Table 10) showed that the genes whose expression levels differed between the two were ten and four clones as described below.
BRSTN2011961, BRSTN2012069, TBAES2003917, TBAES2005361, TBAES2007428, TBAES2007548, TBAES2007862, TESTI2005564, TESTI4017854, TRACH3016805
TBAES2004105, TBAES2007379, TBAES2007481, TBAES2008133

The result of comparative analysis of cDNA libraries derived from cervical tumor (TCERX) and normal cervical duct (CERVX) (Table 11) showed that the genes whose expression levels differed between the two were six clones as described below.
BRACE2017397, BRHIP2027077, BRSTN2011961, BRSTN2012069,
CERVX2000812, CERVX2000968

The result of comparative analysis of cDNA libraries derived from colon tumor (TCOLN) and normal colon (COLON) (Table 12) showed that the genes whose expression levels were different between the two were ten clones as described below.
BRSTN2011961, BRSTN2012069, COLON2001829, COLON2001866, COLON2004351, COLON2004911, COLON2005623, COLON2005735, OCBBF3001333, SMINT2017964

The result of comparative analysis of cDNA libraries derived from esophageal tumor (TESOP) and normal esophagus (NESOP) (Table 13) showed that the genes whose expression levels were different between the two were 14 clones as described below.
BRAMY3004364, BRAWH3027533, BRHIP3007223, BRSTN2011961, BRSTN2012069, CTONG2011801, CTONG3002518, SMINT2009292, TESOP2002005, TESOP2003308, TESOP2004110, TESOP2008556, UTERU3015011, UTERU3017995

The result of comparative analysis of cDNA libraries derived from kidney tumor (TKIDN) and normal kidney (KIDNE) (Table 14) showed that the genes whose expression levels were different between the two were 43 clones as described below.
BRACE2002392, BRACE2012528, BRACE3004371, BRAMY2039630, BRAMY3004364, BRAMY3008436, BRAWH2004078, BRAWH3012662, BRAWH3021574, BRAWH3022651, BRAWH3037428, BRCAN2019953, BRCAN2022126, BRHIP3002000, BRHIP3002691, BRHIP3012997, BRHIP3020046, BRSTN2012069, BRSTN2016992, BRTHA3010212, CTONG2006235, KIDNE2004531, KIDNE2010049, KIDNE2014496, KIDNE2015987, KIDNE2016464, KIDNE2017153, KIDNE2018268, NT2RI2015533, NT2RP7007387, OCBBF3004487, PLACE6008315, SYNOV4004210, TESTI2005112, THYMU3001776, THYMU3002887, THYMU3029795, THYMU3032867, TKIDN2000319, TKIDN2003396, TKIDN2010602, TKIDN2011051, TKIDN2011160

The result of comparative analysis of cDNA libraries derived from liver tumor (TLIVE) and normal liver (LIVER) (Table 15) showed that the genes whose expression levels were different between the two were 14 and two clones as described below.
BRAWH3022651, BRCAN2020412, BRSTN2012069, BRTHA3003736, CTONG2006235, LIVER2008465, TESTI4013474, THYMU3002887, THYMU3038158, TLIVE2000142, TLIVE2001616, TLIVE2007736, TLIVE2008797, TRACH3027229
THYMU3046350, TLIVE2007192

The result of comparative analysis of cDNA libraries derived from lung tumor (TLUNG) and normal lung (HLUNG) (Table 16) showed that the genes whose expression levels were different between the two were 17 clones as described below.
BRACE3036283, BRAMY2031516, BRSTN2011961, BRSTN2012069, HLUNG2012600, MESAN2009156, NTONG2008483, PROST2007444, TESTI4003703, TESTI4005653, TESTI4013474, TESTI4029297, THYMU3001776, THYMU3033626, THYMU3034671, THYMU3041428, TRACH3022198

The result of comparative analysis of cDNA libraries derived from ovary tumor (TOVER) and normal ovary (NOVER) (Table 17) showed the genes whose expression levels were different between the two were three clones as described below.
BRSTN2012069, NOVAR2000783, THYMU3002887

The result of comparative analysis of cDNA libraries derived from stomach tumor (TSTOM) and normal stomach (STOMA) (Table 18) showed that the genes whose expression levels were different between the two were 9 clones as described below.
BRSTN2012069, CHONS2002829, STOMA2003894, STOMA2004663, THYMU3001776, TSTOM2000235, TSTOM2001571, TSTOM2002611, TSTOM2002682

The result of comparative analysis of cDNA libraries derived from uterine tumor (TUTER) and normal uterus (UTERU) (Table 19) showed that the genes whose expression levels were different between the two were 128 and three clones as described below.
BRACE2012528, BRACE2017397, BRACE3004371, BRACE3036283, BRACE3040863, BRAMY2031516, BRAMY3005184, BRAWH2004078, BRAWH3004350, BRAWH3022651, BRAWH3024186, BRAWH3029806, BRAWH3031342, BRCAN2022126, BRHIP3001076, BRHIP3002000, BRHIP3002141, BRHIP3005307, BRHIP3007223, BRHIP3017855, BRHIP3020046, BRSTN2010089, BRSTN2011961, BRSTN2012069, BRSTN2016892, BRTHA3003736, BRTHA3011265, BRTHA3023403, BRTHA3027879, CHONS2002829, CTONG2001932, CTONG2003517, CTONG2006235, CTONG2011801, CTONG3002518, DFNES2001829, KIDNE2010049, LIVER2008465, NT2RI3005923, OCBBF3001333, OCBBF3004487, PLACE6008315, PLACE7006240, PROST2007444, SPLEN2012571, SYNOV4000598, SYNOV4009575, T1ESE2000904, TESTI4002072, TESTI4002195, TESTI4002774, TESTI4002799, TESTI4003703, TESTI4003944, TESTI4005399, TESTI4005653, TESTI4024245, TESTI4029297, THYMU3002887, THYMU3021586, THYMU3026350, THYMU3032798, THYMU3034616, THYMU3034671, TRACH3003872, TRACH3005699, TRACH3006800, TRACH3008632, TRACH3009008, TUTER1000014, TUTER2001433, UTERU2000300, UTERU2014998, UTERU2016464, UTERU2016669, UTERU2020226, UTERU2022955, UTERU2023941, UTERU2024042, UTERU2027369, UTERU2028377, UTERU2029660, UTERU2035926, UTERU2037423, UTERU3000670, UTERU3001029, UTERU3001394, UTERU3001946, UTERU3004635, UTERU3005264, UTERU3005422, UTERU3006538, UTERU3006720, UTERU3007108, UTERU3009775, UTERU3010029, UTERU3010409, UTERU3010604, UTERU3010892, UTERU3010919, UTERU3011092, UTERU3011398, UTERU3011558, UTERU3011579, UTERU3011837, UTERU3012293, UTERU3012414, UTERU3012476, UTERU3012599, UTERU3012999, UTERU3013167, UTERU3013302, UTERU3014274, UTERU3014647, UTERU3014906, UTERU3015011, UTERU3015299, UTERU3015647, UTERU3015844, UTERU3016070, UTERU3016273, UTERU3016274, UTERU3016308, UTERU3017441, UTERU3017626, UTERU3017995, UTERU3018172, UTERU3018255 UTERU2017492, UTERU2025415, UTERU2036507

The result of comparative analysis of cDNA libraries derived from tongue cancer (CTONG) and normal tongue (NTONG) (Table 20) showed that the genes whose expression levels were different between the two were 67 and two clones as described below.
BRACE2012528, BRAMY4001863, BRAWH3021574, BRAWH3022651, BRAWH3024186, BRHIP2027077, BRHIP3001573, BRHIP3002000, BRHIP3007223, BRHIP3012997, BRHIP3020046, BRSSN2013696, BRSTN2011961, BRSTN2012069, BRTHA2027229, BRTHA2033155, BRTHA3011194, BRTHA3022641, CTONG2001932, CTONG2003517, CTONG2006235, CTONG2008989, CTONG2009033, CTONG2009570, CTONG2010330, CTONG2011801, CTONG2012123, CTONG2014206, CTONG2014959, CTONG2020582, CTONG2026987, CTONG2027150, CTONG2027591, CTONG2027783, CTONG2027959, CTONG3001605, CTONG3002518, CTONG3002588, CTONG3003669, CTONG3008223, NT2RI2009233, NTONG2002278, NTONG2003805, NTONG2004829, NTONG2008483, NTONG2009468, OCBBF3004487, PLACE6008315, PLACE7004103, SKNMC2003639, SPLEN2012571, SPLEN2019092, SYNOV4009575, T1ESE2000904, TESTI2005564, TESTI2018867, TESTI4002799, TESTI4005653, TESTI4032913, THYMU3021586, THYMU3047115, TRACH3006717, TRACH3007625, TRACH3016805, TRACH3036932, TRACH3038399, UTERU2000300
CTONG2002832, CTONG2003764

These genes are involved in cancer.

Further, there is a method to search for genes involved in development and differentiation, which is the expression frequency analysis in which the expression levels of genes are compared between developing and/or differentiating tissues and/or cells and adult tissues and/or cells. The genes involved in tissue development and/or differentiation are genes participating in tissue construction and expression of function, and thus are useful genes, which are available for regenerative medicine aiming at convenient regeneration of injured tissues.

By using the information of gene expression frequency gained from the database of nucleotide sequences of 1,402,069 clones as described above, genes whose expression frequencies were different between developing and/or differentiating tissues and/or cells and adult tissues and/or cells were analyzed.

The result of comparative analysis of cDNA libraries derived from fetal brain (FCBBF, FEBRA or OCBBF) and adult brain (BRACE, BRALZ, BRAMY, BRAWH, BRCAN, BRCOC, BRHIP, BRSSN, BRSTN or BRTHA) (Table 21) showed that the genes whose expression levels were different between the two were 916 and 13 clones as described below.
ASTRO2008972, ASTRO2016114, BLADE2004849, BRACE1000475, BRACE2002392, BRACE2003628, BRACE2005991, BRACE2010336, BRACE2012528, BRACE2012625, BRACE2012833, BRACE2012838, BRACE2012936, BRACE2012947, BRACE2013009, BRACE2013126, BRACE2013132, BRACE2016896, BRACE2017359, BRACE2017397, BRACE2017580, BRACE2017844, BRACE2017872, BRACE2017992, BRACE2019348, BRACE2023633, BRACE2023744, BRACE2025452, BRACE2026404, BRACE2027312, BRACE2027382, BRACE2028956, BRACE2030039, BRACE2032584, BRACE2033128, BRACE2034434, BRACE2035120, BRACE2035191, BRACE2039362, BRACE2039607, BRACE2042541, BRACE2046976, BRACE2047232, BRACE2047975, BRACE3001403, BRACE3001973, BRACE3002344, BRACE3002541, BRACE3002756, BRACE3003866, BRACE3004046, BRACE3004371, BRACE3004767, BRACE3004887, BRACE3004981, BRACE3005870, BRACE3005903, BRACE3006553, BRACE3007649, BRACE3007869, BRACE3009075, BRACE3009265, BRACE3009392, BRACE3009416, BRACE3009539, BRACE3010702, BRACE3011447, BRACE3011774, BRACE3013418, BRACE3013874, BRACE3013986, BRACE3014523, BRACE3014714, BRACE3015090, BRACE3015898, BRACE3016020, BRACE3016167, BRACE3016580, BRACE3016788, BRACE3016810, BRACE3016862, BRACE3017253, BRACE3018083, BRACE3019570, BRACE3019611, BRACE3019817, BRACE3019941, BRACE3020356, BRACE3020669, BRACE3021430, BRACE3021517, BRACE3021805, BRACE3022051, BRACE3022303, BRACE3022312, BRACE3022340, BRACE3022847, BRACE3023604, BRACE3024379, BRACE3024444, BRACE3024497, BRACE3024537, BRACE3024879, BRACE3025627, BRACE3025719, BRACE3026161, BRACE3026290, BRACE3026345, BRACE3026456, BRACE3026802, BRACE3026844, BRACE3026947, BRACE3027256, BRACE3027931, BRACE3028360, BRACE3028895, BRACE3028998, BRACE3029005, BRACE3029021, BRACE3029205, BRACE3029447, BRACE3030538, BRACE3031161, BRACE3031184, BRACE3031185, BRACE3031315, BRACE3031372, BRACE3031579, BRACE3031728, BRACE3031743, BRACE3031843, BRACE3032385, BRACE3032537, BRACE3032538, BRACE3032631, BRACE3032980, BRACE3033525, BRACE3034183, BRACE3034389, BRACE3034964, BRACE3034993, BRACE3035168, BRACE3036156, BRACE3036271, BRACE3036283, BRACE3037612, BRACE3037637, BRACE3037803, BRACE3038012, BRACE3038030, BRACE3038570, BRACE3038760, BRACE3039288, BRACE3039358, BRACE3039378, BRACE3039454, BRACE3040012, BRACE3040239, BRACE3040504, BRACE3040644, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044495, BRACE3045078, BRACE3045145, BRACE3045424, BRACE3045708, BRACE3045981, BRACE3046049, BRACE3046152, BRACE3046294, BRACE3046466, BRACE3046491, BRACE3046609, BRACE3046837, BRACE3046855, BRACE3046966, BRACE3047018, BRACE3047482, BRACE3047801, BRALZ2003119, BRALZ2007661, BRALZ2008930, BRALZ2010842, BRALZ2011337, BRALZ2013621, BRALZ2013690, BRALZ2014054, BRAMY2015516, BRAMY2021098, BRAMY2022320, BRAMY2023939, BRAMY2025495, BRAMY2031516, BRAMY2033895, BRAMY2035801, BRAMY2036254, BRAMY2036266, BRAMY2037609, BRAMY2039630, BRAMY2040915, BRAMY2041347, BRAMY2041384, BRAMY2041507, BRAMY2044686, BRAMY2046489, BRAMY2046537, BRAMY3000692, BRAMY3001409, BRAMY3002329, BRAMY3002508, BRAMY3002886, BRAMY3004126, BRAMY3004364, BRAMY3005184, BRAMY3005656, BRAMY3005912, BRAMY3007078, BRAMY3007449, BRAMY3007471, BRAMY3008436, BRAMY3009158, BRAMY3009491, BRAMY3009556, BRAMY3009904, BRAMY3010321, BRAMY3010603, BRAMY3010654, BRAMY4000915, BRAMY4000962, BRAMY4001234, BRAMY4001652, BRAMY4001863, BRAMY4001913, BRAMY4002575, BRAMY4002628, BRAWH2000256, BRAWH2002333, BRAWH2004078, BRAWH2010364, BRAWH2010619, BRAWH2011796, BRAWH2011812, BRAWH2011958, BRAWH2012054, BRAWH2012866, BRAWH2013955, BRAWH2014053, BRAWH2016209, BRAWH2016223, BRAWH2016305, BRAWH2016514, BRAWH2016562, BRAWH2016785, BRAWH3000446, BRAWH3000884, BRAWH3001053, BRAWH3001638, BRAWH3001783, BRAWH3001833, BRAWH3003244, BRAWH3003573, BRAWH3003975, BRAWH3004335, BRAWH3004350, BRAWH3005037, BRAWH3005886, BRAWH3005892, BRAWH3005896, BRAWH3008167, BRAWH3008559, BRAWH3008867, BRAWH3009961, BRAWH3010461, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005,. BRAWH3012662, BRAWH3012779, BRAWH3013009, BRAWH3013049, BRAWH3013264, BRAWH3013508, BRAWH3014609, BRAWH3014639, BRAWH3015017, BRAWH3015175, BRAWH3015610, BRAWH3015825, BRAWH3016123, BRAWH3016715, BRAWH3017180, BRAWH3017259, BRAWH3017260, BRAWH3017477, BRAWH3017980, BRAWH3018063, BRAWH3018369, BRAWH3018548, BRAWH3018969, BRAWH3019026, BRAWH3019529, BRAWH3019594, BRAWH3019820, BRAWH3020200, BRAWH3020318, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542, BRAWH3022651, BRAWH3022719, BRAWH3022900, BRAWH3023156, BRAWH3023168, BRAWH3023172, BRAWH3023274, BRAWH3023421, BRAWH3024186, BRAWH3024231, BRAWH3024242, BRAWH3024506, BRAWH3024989, BRAWH3026349, BRAWH3026938, BRAWH3027420, BRAWH3027440, BRAWH3027533, BRAWH3027574, BRAWH3027607, BRAWH3027616, BRAWH3027675, BRAWH3027806, BRAWH3027880, BRAWH3028202, BRAWH3028223, BRAWH3028461, BRAWH3028754, BRAWH3028796, BRAWH3029313, BRAWH3029385, BRAWH3029538, BRAWH3029806, BRAWH3030772, BRAWH3030810, BRAWH3030910, BRAWH3031054, BRAWH3031342, BRAWH3031710, BRAWH3032298, BRAWH3032340, BRAWH3032571, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035403, BRAWH3035904, BRAWH3035914, BRAWH3035936, BRAWH3036077, BRAWH3036247, BRAWH3036270, BRAWH3036334, BRAWH3036561, BRAWH3037265, BRAWH3037394, BRAWH3037428, BRAWH3037533, BRAWH3037979, BRAWH3038055, BRAWH3038230, BRAWH3038252, BRAWH3038324, BRCAN2000923, BRCAN2002662, BRCAN2002892, BRCAN2003269, BRCAN2003814, BRCAN2006051, BRCAN2006955, BRCAN2007525, BRCAN2008701, BRCAN2009168, BRCAN2010547, BRCAN2010581, BRCAN2010665, BRCAN2015402, BRCAN2015757, BRCAN2018269, BRCAN2018667, BRCAN2019653, BRCAN2019907, BRCAN2019953, BRCAN2020234, BRCAN2020331, BRCAN2020412, BRCAN2020467, BRCAN2020880, BRCAN2021325, BRCAN2021452, BRCAN2021718, BRCAN2022126, BRCAN2025093, BRCAN2027593, BRCAN2028702, BRCOC2001355, BRCOC2002777, BRCOC2006164, BRCOC2006639, BRCOC2006942, BRCOC2009638, BRCOC2010115, BRCOC2012386, BRHIP2006819, BRHIP2006921, BRHIP2008756, BRHIP2009177, BRHIP2011199, BRHIP2013958, BRHIP2015153, BRHIP2016125, BRHIP2017714, BRHIP2020930, BRHIP2021929, BRHIP2023735, BRHIP2024941, BRHIP2026346, BRHIP2027077, BRHIP2027563, BRHIP2029529, BRHIP2029643, BRHIP2029663, BRHIP3000626, BRHIP3000859, BRHIP3001076, BRHIP3001141, BRHIP3001338, BRHIP3001360, BRHIP3001481, BRHIP3001573, BRHIP3001878, BRHIP3002000, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003688, BRHIP3003795, BRHIP3003845, BRHIP3003961, BRHIP3003984, BRHIP3004215, BRHIP3004710, BRHIP3004725, BRHIP3004774, BRHIP3004786, BRHIP3005037, BRHIP3005142, BRHIP3005231, BRHIP3005307, BRHIP3005673, BRHIP3005801, BRHIP3005944, BRHIP3006279, BRHIP3006294, BRHIP3006449, BRHIP3006786, BRHIP3006950, BRHIP3007172, BRHIP3007195, BRHIP3007223, BRHIP3007291, BRHIP3007409, BRHIP3007424, BRHIP3007609, BRHIP3007960, BRHIP3008082, BRHIP3008320, BRHIP3008714, BRHIP3009672, BRHIP3009753, BRHIP3010289, BRHIP3010916, BRHIP3011082, BRHIP3011269, BRHIP3011460, BRHIP3011567, BRHIP3011831, BRHIP3012185, BRHIP3012289, BRHIP3012357, BRHIP3012736, BRHIP3012997, BRHIP3013078, BRHIP3013588, BRHIP3013698, BRHIP3014675, BRHIP3015854, BRHIP3016032, BRHIP3016421, BRHIP3017109, BRHIP3017146, BRHIP3017256, BRHIP3017558, BRHIP3017855, BRHIP3018784, BRHIP3019643, BRHIP3019824, BRHIP3019880, BRHIP3019956, BRHIP3020046, BRHIP3020155, BRHIP3020733, BRHIP3021019, BRHIP3021499, BRHIP3021987, BRHIP3022656, BRHIP3023922, BRHIP3024703, BRHIP3024820, BRHIP3025795, BRHIP3025844, BRHIP3026231, BRHIP3026651, BRHIP3027160, BRHIP3027191, BRHIP3027651, BRHIP3027947, BRHIP3028246, BRHIP3028570, BRHIP3028742, BRSSN2004303, BRSSN2004710, BRSSN2008464, BRSSN2011843, BRSSN2012157, BRSSN2012198, BRSSN2013696, BRSSN2015497, BRSSN2018218, BRSTN2000312, BRSTN2006466, BRSTN2006638, BRSTN2008475, BRSTN2009247, BRSTN2010089, BRSTN2010416, BRSTN2011688, BRSTN2011961, BRSTN2012069, BRSTN2016892, BRSTN2016918, BRSTN2016992, BRSTN2017104, BRSTN2017151, BRSTN2018712, BRTHA2000969, BRTHA2001304, BRTHA2001953, BRTHA2002091, BRTHA2003759, BRTHA2005448, BRTHA2006720, BRTHA2008502, BRTHA2008598, BRTHA2010672, BRTHA2012189, BRTHA2014647, BRTHA2018304, BRTHA2019726, BRTHA2019743, BRTHA2020400, BRTHA2020566, BRTHA2020642, BRTHA2020695, BRTHA2020721, BRTHA2020781, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2028297, BRTHA2029969, BRTHA2030036, BRTHA2030213, BRTHA2031517, BRTHA2031917, BRTHA2032763, BRTHA2033122, BRTHA2033155, BRTHA2033320, BRTHA2033469, BRTHA2033683, BRTHA2034281, BRTHA2034576, BRTHA2035743, BRTHA2036055, BRTHA2036295, BRTHA2037247, BRTHA2038279, BRTHA2038345, BRTHA2038353, BRTHA3000456, BRTHA3002411, BRTHA3003225, BRTHA3003417, BRTHA3003736, BRTHA3005988, BRTHA3006593, BRTHA3007469, BRTHA3007662, BRTHA3009858, BRTHA3010135, BRTHA3010212, BRTHA3010469, BRTHA3010530, BRTHA3010540, BRTHA3010717, BRTHA3011187, BRTHA3011194, BRTHA3011229, BRTHA3011265, BRTHA3011306, BRTHA3011361, BRTHA3011510, BRTHA3011892, BRTHA3011998, BRTHA3012265, BRTHA3013860, BRTHA3013882, BRTHA3014000, BRTHA3014105, BRTHA3014507, BRTHA3014547, BRTHA3014835, BRTHA3014854, BRTHA3014920, BRTHA3016616, BRTHA3017791, BRTHA3018409, BRTHA3018623, BRTHA3019183, BRTHA3020369, BRTHA3020771, BRTHA3021569, BRTHA3021708, BRTHA3021786, BRTHA3021971, BRTHA3022641, BRTHA3023403, BRTHA3023590, BRTHA3023929, BRTHA3024600, BRTHA3025073, BRTHA3026161, BRTHA3026180, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, CHONS2002829, CTONG2001932, CTONG2006235, CTONG2009033, CTONG2011801, CTONG2020582, CTONG2027959, D9OST2003106 DFNES2001829, FCBBF3001018, FCBBF3002188, FCBBF3005160, FCBBF3012443, FCBBF3020030, FCBBF3021191, FCBBF3024911, FCBBF5000384, FEBRA2000805, FEBRA2002260, FEBRA2012625, FEBRA2013069, FEBRA2013570, FEBRA2017736, FEBRA2017811, FEBRA2023498, FEBRA2026582, FEBRA2026977, FEBRA2028222, FEBRA2028457, JCMLC2000273, KIDNE2010049, KIDNE2017153, LIVER2008465, MESAN2017133, NOVAR2000783, NT2NE2011107, NT2RI2009233, NT2RI2010795, NT2RI2015533, NT2RI3005923, NT2RI3009524, NT2RP7007387, NT2RP8001604, NT2RP8001605, NT2RP8007920, NT2RP8009119, NTONG2008483, NTONG2009468, OCBBF2000831, OCBBF2003518, OCBBF2004478, OCBBF2007039, OCBBF2009536, OCBBF2014745, OCBBF2016928, OCBBF2018229, OCBBF2018618, OCBBF2019761, OCBBF2024589, OCBBF2024779, OCBBF2025631, OCBBF2030927, OCBBF2036019, OCBBF3000743, OCBBF3000830, OCBBF3001076, OCBBF3001202, OCBBF3001333, OCBBF3001616, OCBBF3003745, OCBBF3004487, OCBBF3004908, OCBBF3005330, OCBBF3005843, OCBBF3006986, OCBBF3007078, OCBBF3007704, OCBBF3008392, OCBBF3008835, OCBBF3009244, PLACE5000492, PLACE6003004, PLACE6008315, PLACE6010936, PLACE7004103, PLACE7006240, PROST2007444, PROST2017910, SMINT2009292, SMINT2012179, SPLEN2012571, SYNOV4004210, SYNOV4009575, T1ESE2000609,T1ESE2000904, TBAES2007428, TESTI2005112, TESTI2005564, TESTI2009497, TESTI2018867, TESTI2021654, TESTI2039342, TESTI4001569, TESTI4002072, TESTI4002195, TESTI4002774, TESTI4002799, TESTI4003602, TESTI4003703, TESTI4003944, TESTI4004210, TESTI4004695, TESTI4005013, TESTI4005399, TESTI4005653, TESTI4006441, TESTI4007965, TESTI4010979, TESTI4012960, TESTI4013474, TESTI4014908, TESTI4020596, TESTI4022158, TESTI4029297, TESTI4032913, TESTI4035770, TESTI4043223, TESTI4046073, THYMU3000776, THYMU3002887, THYMU3003007, THYMU3003350, THYMU3007308, THYMU3008105, THYMU3019476, THYMU3021586, THYMU3026000, THYMU3026306, THYMU3026350, THYMU3032798, THYMU3032867, THYMU3033626, THYMU3034671, THYMU3037827, THYMU3038214, THYMU3041428, THYMU3044075, TKIDN2000319, TLIVE2007736, TRACH2013585, TRACH3002752, TRACH3003037, TRACH3003872, TRACH3004424, TRACH3006717, TRACH3007625, TRACH3007689, TRACH3007995, TRACH3008508, TRACH3008632, TRACH3009008, TRACH3010079, TRACH3016805, TRACH3016885, TRACH3021544, TRACH3022109, TRACH3022198, TRACH3024342, TRACH3024671, TRACH3025316, TRACH3026303, TRACH3026676, TRACH3028855, TRACH3032570, TRACH3036932, TRACH3038399, TSTOM2000235, UTERU2000300, UTERU2027369, UTERU3000670, UTERU3005422, UTERU3010409, UTERU3012414, UTERU3013167, UTERU3015011, UTERU3016273, UTERU3017995, UTERU3018172 BRACE3002184, BRACE3026993, BRACE3046450, BRAMY3008096, BRAWH3013197, BRAWH3028645, BRTHA3004432, BRTHA3024233, CTONG2002832, TESTI4002988, THYMU3046350, TRACH1000193, TRACH3019290

The result of comparative analysis of cDNA libraries derived from fetal heart (FEHRT) and adult heart (HEART) (Table 22) showed that the genes whose expression levels were different between the two were ten clones as described below.
BRACE2012528, BRACE3004371, BRCAN2003814, BRSTN2011961, BRSTN2012069, BRSTN2016992, HEART2002531, NTONG2008483, PROST2002078, T1ESE2000609

The result of comparative analysis of cDNA libraries derived from fetal kidney (FEKID) and adult kidney (KIDNE) (Table 23) showed that the genes whose expression levels were different between the two were 21 clones as described below.
BRACE3004371, BRAMY2039630, BRAMY3004364, BRAWH2004078, BRHIP3002000, BRSTN2011961, BRSTN2012069, BRTHA2027229, KIDNE2004531, KIDNE2010049, KIDNE2014496, KIDNE2015987, KIDNE2016464, KIDNE2017153, KIDNE2018268, NT2RP7007387, TESTI2005112, TESTI4002799, THYMU3001776, THYMU3029795, THYMU3032867

The result of comparative analysis of cDNA libraries derived from fetal lung (FELNG) and adult lung (HLUNG) (Table 24) showed that the genes whose expression levels were different between the two were 18 clones as described below.
BRACE3036283, BRAMY2031516, BRSTN2011961, BRSTN2012069, HLUNG2012600, MESAN2009156, NTONG2008483, PROST2007444, TESTI4003703, TESTI4005653, TESTI4013474, TESTI4029297, THYMU3001776, THYMU3033626, THYMU3034671, THYMU3041428, THYMU3044188, TRACH3022198

These genes are involved in regeneration of tissues and/or cells.

A nucleotide sequence information-based analysis was carried out to identify the genes whose expression frequencies are higher or lower in CD34+ cell (cell expressing a glycoprotein CD34) treated with the osteoclast differentiation factor (Molecular Medicine 38. 642-648. (2001)) than in the untreated CD34+ cell, which is the precursor cell of monocyte/macrophage line. The result of comparative analysis for the frequency between the two cDNA libraries prepared from the RNA of CD34+ cells (CD34C) and from the RNA of CD34+ cells treated with the osteoclast differentiation factor (D3OST, D6OST or D9OST) showed following genes whose expression levels were different between the two.

**Table 2**

| Clone ID | CD34C | D30ST | D60ST | D90ST |
|---|---|---|---|---|
| BRAWH3018063 | 0.000 | 30.813 | 0.000 | 0.000 |
| BRHIP3020046 | 0.000 | 0.000 | 68.001 | 0.000 |
| BRSSN2013696 | 0.000 | 41.698 | 0.000 | 0.000 |
| BRSTN2012069 | 2.383 | 0.442 | 0.000 | 0.128 |
| BRTHA2027229 | 0.000 | 0.000 | 61.446 | 0.000 |
| D9OST2003106 | 0.000 | 0.000 | 0.000 | 93.096 |
| D9OST2003989 | 0.000 | 0.000 | 0.000 | 100.000 |
| D9OST2004417 | 0.000 | 0.000 | 0.000 | 100.000 |
| OCBBF2016928 | 0.000 | 31.972 | 0.000 | 0.000 |
| TESTI4005653 | 0. 000 | 0. 000 | 0.000 | 6.704 |
| TESTI4013474 | 43.396 | 0.000 | 0.000 | 0.000 |
| THYMU3032798 | 0.000 | 0.000 | 0.000 | 26.074 |

A survey was performed for genes whose expression levels are varied in response to induction of differentiation (stimulation by retinoic acid (RA) or growth inhibitor treatment after RA stimulation) in cultured cells of a neural strain, NT2. The result of comparative analysis of cDNA libraries derived from undifferentiated NT2 cells (NT2RM) and the cells subjected to the differentiation treatment (NT2RP, NT2RI or NT2NE) showed following genes whose expression levels were different between the two.

**Table 3**

| Clone ID | NT2RM | NT2RP | NT2RI | NT2NE |
|---|---|---|---|---|
| BLADE2004849 | 0.000 | 0.000 | 0.000 | 26.862 |
| BRACE2003628 | 0.000 | 16.442 | 0.000 | 0.000 |
| BRACE2012528 | 0.000 | 1.143 | 0.862 | 0.000 |
| BRAMY2023939 | 0.000 | 0.000 | 11.107 | 22.151 |
| BRAMY2031516 | 0.000 | 17.802 | 0.000 | 0.000 |
| BRAMY4002628 | 0.000 | 17.119 | 12.908 | 0.000 |
| BRAWH3010461 | 0.000 | 0.000 | 3.733 | 0.000 |
| BRAWH3017259 | 0.000 | 0.000 | 0.000 | 50.722 |
| BRAWH3018063 | 0.000 | 0.000 | 14.389 | 0.000 |
| BRAWH3022651 | 0.000 | 3.948 | 0.000 | 0.000 |
| BRAWH3024186 | 0.000 | 0.000 | 18.135 | 0.000 |
| BRCAN2019653 | 0.000 | 15.031 | 0.000 | 0.000 |
| BRCAN2022126 | 0.000 | 0.000 | 8.824 | 0.000 |
| BRCOC2012386 | 0.000 | 27.482 | 20.721 | 0.000 |
| BRHIP3002000 | 0.000 | 3.615 | 0.000 | 0.000 |
| BRHIP3007223 | 0.000 | 4.859 | 3.664 | 0.000 |
| BRHIP3021019 | 0.000 | 0.000 | 14.074 | 0.000 |
| BRSTN2011961 | 8.334 | 0.000 | 0.526 | 3.149 |
| BRSTN2012069 | 0.000 | 0.479 | 0.052 | 0.788 |
| BRTHA2033155 | 0.000 | 0.000 | 7.729 | 0.000 |
| BRTHA3010212 | 0.000 | 13.315 | 0.000 | 0.000 |
| BRTHA3011194 | 0.000 | 0.000 | 0.000 | 12.809 |
| BRTHA3011265 | 0.000 | 20.563 | 0.000 | 0.000 |
| BRTHA3017791 | 0.000 | 5.698 | 0.000 | 0.000 |
| BRTHA3021971 | 55.855 | 0.000 | 0.000 | 0.000 |
| CHONS2002829 | 0.000 | 0.000 | 2.969 | 0.000 |
| CTONG2006235 | 0.000 | 3.948 | 0.000 | 0.000 |
| FCBBF3012443 | 0.000 | 0.000 | 49.588 | 0.000 |
| FEBRA2026582 | 0.000 | 0.000 | 42.162 | 0.000 |
| LIVER2008465 | 0.000 | 15.950 | 0.000 | 0.000 |
| NT2NE2011107 | 0.000 | 0.000 | 0.000 | 43.585 |
| NT2NE2016041 | 0.000 | 0.000 | 0.000 | 100.000 |
| NT2RI2004818 | 0.000 | 0.000 | 100.000 | 0.000 |
| NT2RI2009233 | 0.000 | 5.436 | 4.099 | 0.000 |
| NT2RI2010795 | 0.000 | 0.000 | 29.973 | 0.000 |
| NT2RI2015533 | 0.000 | 0.000 | 2.824 | 0.000 |
| NT2RI2023671 | 0.000 | 0.000 | 100.000 | 0.000 |
| NT2RI2028537 | 0.000 | 0.000 | 100.000 | 0.000 |
| NT2RI3001573 | 0.000 | 0.000 | 100.000 | 0.000 |
| NT2RI3001967 | 0.000 | 0.000 | 100.000 | 0.000 |
| NT2RI3005861 | 0.000 | 0.000 | 100.000 | 0.000 |
| NT2RI3005923 | 0.000 | 0.000 | 35.423 | 0.000 |
| NT2RI3007095 | 0.000 | 0.000 | 100.000 | 0.000 |
| NT2RI3008179 | 0.000 | 0.000 | 100.000 | 0.000 |
| NT2RI3009480 | 0.000 | 0.000 | 68.547 | 0.000 |
| NT2RI3009524 | 0.000 | 0.000 | 64.504 | 0.000 |
| NT2RP7003439 | 0.000 | 100.000 | 0.000 | 0.000 |
| NT2RP7007387 | 0.000 | 9.894 | 3.730 | 0.000 |
| NT2RP7014178 | 0.000 | 100.000 | 0.000 | 0.000 |
| NT2RP7014778 | 0.000 | 100.000 | 0.000 | 0.000 |
| NT2RP7016508 | 0.000 | 100.000 | 0.000 | 0.000 |
| NT2RP7017139 | 0.000 | 100.000 | 0.000 | 0.000 |
| NT2RP7019682 | 0.000 | 100.000 | 0.000 | 0.000 |
| NT2RP7020343 | 0.000 | 100.000 | 0.000 | 0.000 |
| NT2RP8000633 | 0.000 | 100.000 | 0.000 | 0.000 |
| NT2RP8001363 | 0.000 | 100.000 | 0.000 | 0.000 |
| NT2RP8001407 | 0.000 | 100.000 | 0.000 | 0.000 |
| NT2RP8001584 | 0.000 | 100.000 | 0.000 | 0.000 |
| NT2RP8001604 | 0.000 | 33.445 | 25.218 | 0.000 |
| NT2RP8001605 | 0.000 | 70.676 | 0.000 | 0.000 |
| NT2RP8003490 | 0.000 | 100.000 | 0.000 | 0.000 |
| NT2RP8003657 | 0.000 | 100.000 | 0.000 | 0.000 |
| NT2RP8003787 | 0.000 | 100.000 | 0.000 | 0.000 |
| NT2RP8005546 | 0.000 | 59.103 | 0.000 | 0.000 |
| NT2RP8006452 | 0.000 | 100.000 | 0.000 | 0.000 |
| NT2RP8006521 | 0.000 | 100.000 | 0.000 | 0.000 |
| NT2RP8007416 | 0.000 | 100.000 | 0.000 | 0.000 |
| NT2RP8007503 | 0.000 | 100.000 | 0.000 | 0.000 |
| NT2RP8007920 | 0.000 | 9.535 | 0.000 | 0.000 |
| NT2RP8008057 | 0.000 | 68.270 | 0.000 | 0.000 |
| NT2RP8009119 | 0.000 | 11.123 | 0.000 | 0.000 |
| NT2RP8009248 | 0.000 | 100.000 | 0.000 | 0.000 |
| NTONG2008483 | 0.000 | 0.000 | 4.356 | 0.000 |
| OCBBF2003518 | 0.000 | 0.000 | 1.873 | 0.000 |
| OCBBF3001333 | 0.000 | 7.743 | 5.839 | 0.000 |
| OCBBF3004908 | 0.000 | 23.051 | 17.381 | 0.000 |
| PLACE7004103 | 0.000 | 6.234 | 9.400 | 0.000 |
| PROST2017910 | 0.000 | 25.185 | 0.000 | 0.000 |
| SMINT2009292 | 0.000 | 0.000 | 6.446 | 0.000 |
| SPLEN2012571 | 0.000 | 15.323 | 5.777 | 0.000 |
| T1ESE2000904 | 0.000 | 11.128 | 0.000 | 0.000 |
| TESTI4002072 | 0.000 | 8.048 | 6.068 | 0.000 |
| TESTI4002774 | 0.000 | 0.000 | 7.456 | 0.000 |
| TESTI4002799 | 0.000 | 4.258 | 1.605 | 0.000 |
| TESTI4005653 | 0.000 | 1.198 | 7.228 | 1.802 |
| TESTI4007965 | 0.000 | 0.000 | 32.797 | 0.000 |
| TESTI4012960 | 0.000 | 71.770 | 0.000 | 0.000 |
| TESTI4018436 | 0.000 | 0. 000 | 9.457 | 0.000 |
| THYMU3001776 | 0.000 | 7.637 | 0.000 | 0.000 |
| THYMU3002887 | 0.000 | 1.880 | 0.000 | 14.135 |
| THYMU3029795 | 0.000 | 0.000 | 19.335 | 0.000 |
| THYMU3041428 | 0.000 | 0.614 | 0.463 | 0.000 |
| THYMU3047115 | 0.000 | 0.000 | 0.747 | 0.000 |
| TRACH3003872 | 0.000 | 13.230 | 0.000 | 0.000 |
| TRACH3004424 | 0.000 | 0.000 | 30.905 | 0.000 |
| TRACH3006717 | 0.000 | 0.000 | 6.668 | 0.000 |
| TRACH3007625 | 0.000 | 2.833 | 2.136 | 4.260 |
| TRACH3009008 | 0.000 | 11.807 | 0.000 | 0.000 |
| TRACH3016805 | 0.000 | 10.851 | 0.000 | 0.000 |
| TRACH3016885 | 0.000 | 0.000 | 14.459 | 0.000 |
| TRACH3026303 | 0.000 | 27.016 | 0.000 | 0.000 |
| UTERU2016669 | 0.000 | 0.000 | 0.000 | 29.924 |

The result of comparative analysis of cDNA libraries derived from the cerebral cortex of Alzheimer patients (BRALZ and BRASW), and from whole tissues of a normal brain (BRAWH) showed the following genes whose expression levels differed between the two.

**Table 4**

| Clone ID | BRAWH | BRALZ | BRASW |
|---|---|---|---|
| ASTRO2016114 | 14.497 | 0.000 | 0.000 |
| BRACE2002392 | 0.000 | 18.684 | 0.000 |
| BRACE2012528 | 3.794 | 1.714 | 0.000 |
| BRACE3004371 | 11.270 | 0.000 | 0.000 |
| BRACE3004767 | 58.488 | 0.000 | 0.000 |
| BRACE3022340 | 58.488 | 0.000 | 0.000 |
| BRACE3031185 | 58.488 | 0.000 | 0.000 |
| BRACE3031743 | 5.455 | 0.000 | 0.000 |
| BRACE3032385 | 13.674 | 0.000 | 0.000 |
| BRACE3032631 | 12.612 | 0.000 | 0.000 |
| BRACE3039358 | 41.331 | 0.000 | 0.000 |
| BRACE3040863 | 17.888 | 0.000 | 0.000 |
| BRACE3042432 | 58.488 | 0.000 | 0.000 |
| BRACE3045981 | 26.434 | 0.000 | 0.000 |
| BRALZ2003119 | 0.000 | 100.000 | 0.000 |
| BRALZ2007661 | 0.000 | 100.000 | 0.000 |
| BRALZ2008930 | 0.000 | 100.000 | 0.000 |
| BRALZ2010842 | 0.000 | 100.000 | 0.000 |
| BRALZ2011337 | 0.000 | 100.000 | 0.000 |
| BRALZ2013621 | 0.000 | 100.000 | 0.000 |
| BRALZ2013690 | 0.000 | 100.000 | 0.000 |
| BRALZ2014054 | 0.000 | 38.408 | 0.000 |
| BRAMY2031516 | 14.772 | 0.000 | 0.000 |
| BRAMY3002329 | 20.323 | 0.000 | 0.000 |
| BRAMY3004126 | 49.811 | 0.000 | 0.000 |
| BRAMY3005912 | 21.416 | 0.000 | 0.000 |
| BRAMY3008436 | 5.603 | 5.061 | 0.000 |
| BRAWH2000256 | 100.000 | 0.000 | 0.000 |
| BRAWH2002333 | 100.000 | 0.000 | 0.000 |
| BRAWH2004078 | 10.539 | 0.000 | 0.000 |
| BRAWH2010364 | 34.464 | 0.000 | 0.000 |
| BRAWH2010619 | 100.000 | 0.000 | 0.000 |
| BRAWH2011796 | 100.000 | 0.000 | 0.000 |
| BRAWH2011812 | 13.544 | 0.000 | 0.000 |
| BRAWH2011958 | 100.000 | 0.000 | 0.000 |
| BRAWH2012054 | 100.000 | 0.000 | 0.000 |
| BRAWH2012866 | 8.503 | 0.000 | 0.000 |
| BRAWH2013955 | 100.000 | 0.000 | 0.000 |
| BRAWH2014053 | 23.222 | 0.000 | 0.000 |
| BRAWH2016209 | 8.306 | 0.000 | 0.000 |
| BRAWH2016223 | 100.000 | 0.000 | 0.000 |
| BRAWH2016305 | 4.308 | 0.000 | 0.000 |
| BRAWH2016514 | 35.037 | 0.000 | 0.000 |
| BRAWH2016562 | 49.499 | 0.000 | 0.000 |
| BRAWH2016785 | 100.000 | 0.000 | 0.000 |
| BRAWH3000446 | 100.000 | 0.000 | 0.000 |
| BRAWH3000884 | 100.000 | 0.000 | 0.000 |
| BRAWH3001053 | 100.000 | 0.000 | 0.000 |
| BRAWH3001638 | 100.000 | 0.000 | 0.000 |
| BRAWH3001783 | 100.000 | 0.000 | 0.000 |
| BRAWH3001833 | 100.000 | 0.000 | 0.000 |
| BRAWH3003244 | 100.000 | 0.000 | 0.000 |
| BRAWH3003573 | 20.162 | 0.000 | 0.000 |
| BRAWH3003975 | 100.000 | 0.000 | 0.000 |
| BRAWH3004335 | 100.000 | 0.000 | 0.000 |
| BRAWH3004350 | 16.580 | 0.000 | 0.000 |
| BRAWH3005037 | 100.000 | 0.000 | 0.000 |
| BRAWH3005886 | 100.000 | 0.000 | 0.000 |
| BRAWH3005892 | 100.000 | 0.000 | 0.000 |
| BRAWH3005896 | 100.000 | 0.000 | 0.000 |
| BRAWH3008167 | 100.000 | 0.000 | 0.000 |
| BRAWH3008559 | 100.000 | 0.000 | 0.000 |
| BRAWH3008867 | 100.000 | 0.000 | 0.000 |
| BRAWH3009961 | 47.417 | 0.000 | 0.000 |
| BRAWH3010461 | 16.435 | 0.000 | 0.000 |
| BRAWH3010602 | 100.000 | 0.000 | 0.000 |
| BRAWH3010657 | 100.000 | 0.000 | 0.000 |
| BRAWH3010726 | 100.000 | 0.000 | 0.000 |
| BRAWH3010833 | 100.000 | 0.000 | 0.000 |
| BRAWH3011101 | 100.000 | 0.000 | 0.000 |
| BRAWH3011331 | 100.000 | 0.000 | 0.000 |
| BRAWH3011402 | 100.000 | 0.000 | 0.000 |
| BRAWH3011577 | 100.000 | 0.000 | 0.000 |
| BRAWH3011623 | 100.000 | 0.000 | 0.000 |
| BRAWH3011685 | 100.000 | 0.000 | 0.000 |
| BRAWH3011907 | 47.417 | 0.000 | 0.000 |
| BRAWH3011929 | 100.000 | 0.000 | 0.000 |
| BRAWH3012005 | 100.000 | 0.000 | 0.000 |
| BRAWH3012662 | 33.972 | 0.000 | 0.000 |
| BRAWH3012779 | 100.000 | 0.000 | 0.000 |
| BRAWH3013009 | 100.000 | 0.000 | 0.000 |
| BRAWH3013049 | 100.000 | 0.000 | 0.000 |
| BRAWH3013264 | 100.000 | 0.000 | 0.000 |
| BRAWH3013508 | 100.000 | 0.000 | 0.000 |
| BRAWH3014609 | 44.597 | 0.000 | 0.000 |
| BRAWH3014639 | 100.000 | 0.000 | 0.000 |
| BRAWH3015017 | 100.000 | 0.000 | 0.000 |
| BRAWH3015175 | 100.000 | 0.000 | 0.000 |
| BRAWH3015610 | 100.000 | 0.000 | 0.000 |
| BRAWH3015825 | 100.000 | 0.000 | 0.000 |
| BRAWH3016123 | 100.000 | 0.000 | 0.000 |
| BRAWH3016715 | 100.000 | 0.000 | 0.000 |
| BRAWH3017180 | 28.251 | 0.000 | 0.000 |
| BRAWH3017259 | 13.996 | 0.000 | 0.000 |
| BRAWH3017260 | 100.000 | 0.000 | 0.000 |
| BRAWH3017477 | 100.000 | 0.000 | 0.000 |
| BRAWH3017980 | 49.811 | 0.000 | 0.000 |
| BRAWH3018063 | 15.836 | 0.000 | 0.000 |
| BRAWH3018369 | 100.000 | 0.000 | 0.000 |
| BRAWH3018548 | 100.000 | 0.000 | 0.000 |
| BRAWH3018969 | 100.000 | 0.000 | 0.000 |
| BRAWH3019026 | 100.000 | 0.000 | 0.000 |
| BRAWH3019529 | 100.000 | 0.000 | 0.000 |
| BRAWH3019594 | 100.000 | 0.000 | 0.000 |
| BRAWH3019820 | 100.000 | 0.000 | 0.000 |
| BRAWH3020200 | 100.000 | 0.000 | 0.000 |
| BRAWH3020318 | 100.000 | 0.000 | 0.000 |
| BRAWH3020884 | 100.000 | 0.000 | 0.000 |
| BRAWH3020928 | 49.499 | 0.000 | 0.000 |
| BRAWH3021012 | 100.000 | 0.000 | 0.000 |
| BRAWH3021574 | 9.400 | 0.000 | 0.000 |
| BRAWH3021580 | 100.000 | 0.000 | 0.000 |
| BRAWH3021641 | 100.000 | 0.000 | 0.000 |
| BRAWH3021643 | 100.000 | 0.000 | 0.000 |
| BRAWH3021724 | 100.000 | 0.000 | 0.000 |
| BRAWH3022347 | 100.000 | 0.000 | 0.000 |
| BRAWH3022431 | 100.000 | 0.000 | 0.000 |
| BRAWH3022459 | 100.000 | 0.000 | 0.000 |
| BRAWH3022542 | 100.000 | 0.000 | 0.000 |
| BRAWH3022651 | 9.828 | 5.919 | 0.000 |
| BRAWH3022719 | 100.000 | 0.000 | 0.000 |
| BRAWH3022900 | 100.000 | 0.000 | 0.000 |
| BRAWH3023156 | 100.000 | 0.000 | 0.000 |
| BRAWH3023168 | 100.000 | 0.000 | 0.000 |
| BRAWH3023172 | 0.916 | 0.000 | 0.000 |
| BRAWH3023274 | 100.000 | 0.000 | 0.000 |
| BRAWH3023421 | 100.000 | 0.000 | 0.000 |
| BRAWH3024186 | 19.959 | 0.000 | 0.000 |
| BRAWH3024231 | 100.000 | 0.000 | 0.000 |
| BRAWH3024242 | 31.843 | 0.000 | 0.000 |
| BRAWH3024506 | 100.000 | 0.000 | 0.000 |
| BRAWH3024989 | 2.369 | 0.000 | 0.000 |
| BRAWH3026349 | 100.000 | 0.000 | 0.000 |
| BRAWH3026938 | 6.894 | 8.303 | 0.000 |
| BRAWH3027420 | 100.000 | 0.000 | 0.000 |
| BRAWH3027440 | 58.488 | 0.000 | 0.000 |
| BRAWH3027533 | 11.008 | 0.000 | 0.000 |
| BRAWH3027574 | 47.417 | 0.000 | 0.000 |
| BRAWH3027607 | 100.000 | 0.000 | 0.000 |
| BRAWH3027616 | 100.000 | 0.000 | 0.000 |
| BRAWH3027675 | 100.000 | 0.000 | 0.000 |
| BRAWH3027806 | 100.000 | 0.000 | 0.000 |
| BRAWH3027880 | 100.000 | 0.000 | 0.000 |
| BRAWH3028202 | 100.000 | 0.000 | 0.000 |
| BRAWH3028223 | 100.000 | 0.000 | 0.000 |
| BRAWH3028461 | 100.000 | 0.000 | 0.000 |
| BRAWH3028754 | 100.000 | 0.000 | 0.000 |
| BRAWH3028796 | 100.000 | 0.000 | 0.000 |
| BRAWH3029313 | 100.000 | 0.000 | 0.000 |
| BRAWH3029385 | 35.187 | 0.000 | 0.000 |
| BRAWH3029538 | 100.000 | 0.000 | 0.000 |
| BRAWH3029806 | 26.735 | 0.000 | 0.000 |
| BRAWH3030772 | 100.000 | 0.000 | 0.000 |
| BRAWH3030810 | 21.287 | 0.000 | 0.000 |
| BRAWH3030910 | 100.000 | 0.000 | 0.000 |
| BRAWH3031054 | 100.000 | 0.000 | 0.000 |
| BRAWH3031342 | 10.856 | 0.000 | 0.000 |
| BRAWH3031710 | 100.000 | 0.000 | 0.000 |
| BRAWH3032298 | 100.000 | 0.000 | 0.000 |
| BRAWH3032340 | 100.000 | 0.000 | 0.000 |
| BRAWH3032571 | 100.000 | 0.000 | 0.000 |
| BRAWH3033117 | 100.000 | 0.000 | 0.000 |
| BRAWH3033293 | 100.000 | 0.000 | 0.000 |
| BRAWH3033448 | 100.000 | 0.000 | 0.000 |
| BRAWH3033513 | 100.000 | 0.000 | 0.000 |
| BRAWH3034097 | 100.000 | 0.000 | 0.000 |
| BRAWH3034114 | 100.000 | 0.000 | 0.000 |
| BRAWH3034134 | 100.000 | 0.000 | 0.000 |
| BRAWH3034668 | 100.000 | 0.000 | 0.000 |
| BRAWH3034743 | 16.662 | 0.000 | 0.000 |
| BRAWH3034775 | 100.000 | 0.000 | 0.000 |
| BRAWH3034890 | 100.000 | 0.000 | 0.000 |
| BRAWH3035403 | 35.337 | 0.000 | 0.000 |
| BRAWH3035904 | 100.000 | 0.000 | 0.000 |
| BRAWH3035914 | 100.000 | 0.000 | 0.000 |
| BRAWH3035936 | 70.575 | 0.000 | 0.000 |
| BRAWH3036077 | 100.000 | 0.000 | 0.000 |
| BRAWH3036247 | 54.530 | 0.000 | 0.000 |
| BRAWH3036270 | 100.000 | 0.000 | 0.000 |
| BRAWH3036334 | 6.241 | 0.000 | 0.000 |
| BRAWH3036561 | 100.000 | 0.000 | 0.000 |
| BRAWH3037265 | 44.597 | 0.000 | 0.000 |
| BRAWH3037394 | 100.000 | 0.000 | 0.000 |
| BRAWH3037428 | 5.877 | 0.000 | 0.000 |
| BRAWH3037533 | 100.000 | 0.000 | 0.000 |
| BRAWH3037979 | 100.000 | 0.000 | 0.000 |
| BRAWH3038055 | 100.000 | 0.000 | 0.000 |
| BRAWH3038230 | 100.000 | 0.000 | 0.000 |
| BRAWH3038252 | 100.000 | 0.000 | 0.000 |
| BRAWH3038324 | 100.000 | 0.000 | 0.000 |
| BRCAN2010665 | 19.610 | 0.000 | 0.000 |
| BRCAN2019653 | 0.000 | 22.535 | 0.000 |
| BRCAN2022126 | 4.855 | 0.000 | 0.000 |
| BRCAN2025093 | 21.732 | 0.000 | 0.000 |
| BRCOC2012386 | 11.403 | 0.000 | 0.000 |
| BRHIP2015153 | 20.038 | 0.000 | 0.000 |
| BRHIP2027077 | 2.020 | 0.000 | 0.000 |
| BRHIP3001573 | 24.474 | 0.000 | 0.000 |
| BRHIP3002000 | 4.500 | 2.710 | 0.000 |
| BRHIP3003063 | 10.960 | 0.000 | 0.000 |
| BRHIP3003984 | 27.477 | 0.000 | 0.000 |
| BRHIP3004774 | 39.520 | 0.000 | 0.000 |
| BRHIP3007223 | 2.016 | 0.000 | 0.000 |
| BRHIP3007409 | 31.957 | 0.000 | 0.000 |
| BRHIP3008320 | 23.349 | 0.000 | 0.000 |
| BRHIP3014675 | 37.925 | 0.000 | 0.000 |
| BRHIP3017855 | 15.316 | 0.000 | 0.000 |
| BRHIP3018784 | 6.930 | 0.000 | 0.000 |
| BRHIP3021019 | 7.745 | 0.000 | 0.000 |
| BRHIP3028246 | 22.147 | 0.000 | 0.000 |
| BRHIP3028570 | 31.957 | 0.000 | 0.000 |
| BRSTN2010089 | 6.598 | 0.000 | 0.000 |
| BRSTN2012069 | 0.047 | 0.718 | 7.145 |
| BRSTN2016992 | 1.476 | 40.898 | 0.000 |
| BRTHA2033155 | 4.253 | 0.000 | 0.000 |
| BRTHA3003736 | 4.302 | 0.000 | 0.000 |
| BRTHA3005988 | 31.076 | 0.000 | 0.000 |
| BRTHA3010135 | 20.010 | 0.000 | 0.000 |
| BRTHA3010212 | 5.525 | 0.000 | 0.000 |
| BRTHA3011194 | 3.534 | 0.000 | 0.000 |
| BRTHA3011265 | 25.596 | 0.000 | 0.000 |
| BRTHA3017791 | 11.821 | 4. 271 | 0.000 |
| BRTHA3020771 | 31.957 | 0.000 | 0.000 |
| BRTHA3021971 | 1.941 | 0.000 | 0.000 |
| BRTHA3023403 | 14.857 | 0.000 | 0.000 |
| CTONG2006235 | 9.828 | 5.919 | 0.000 |
| CTONG2009033 | 25.755 | 0.000 | 0.000 |
| CTONG2020582 | 9.594 | 0.000 | 0.000 |
| D9OST2003106 | 6.904 | 0.000 | 0.000 |
| DFNES2001829 | 9.228 | 0.000 | 0.000 |
| KIDNE2010049 | 3.438 | 0.000 | 0.000 |
| MESAN2017133 | 17.548 | 31.703 | 0.000 |
| NT2RI2009233 | 6.767 | 0.000 | 0.000 |
| NT2RI2015533 | 6.216 | 5.616 | 0.000 |
| NT2RI3005923 | 19.493 | 0.000 | 0.000 |
| NT2RI3009524 | 35.496 | 0.000 | 0.000 |
| NT2RP7007387 | 6.158 | 0.000 | 0.000 |
| NT2RP8001605 | 29.324 | 0.000 | 0.000 |
| NT2RP8007920 | 1.978 | 0.000 | 0.000 |
| NTONG2008483 | 2.397 | 0.000 | 0.000 |
| OCBBF2003518 | 2.062 | 0.000 | 0.000 |
| OCBBF2018618 | 16.218 | 0.000 | 0.000 |
| OCBBF3001333 | 9.639 | 0.000 | 0.000 |
| OCBBF3004487 | 4.531 | 0.000 | 0.000 |
| PLACE7004103 | 3.880 | 0.000 | 0.000 |
| PLACE7006240 | 25.174 | 0.000 | 0.000 |
| PROST2007444 | 4.374 | 0.000 | 0.000 |
| PROST2017910 | 0.000 | 37.758 | 0.000 |
| TBAES2007428 | 5.371 | 0.000 | 0.000 |
| TESTI2005112 | 19.455 | 0.000 | 0.000 |
| TESTI2021654 | 9.773 | 0.000 | 0.000 |
| TESTI4002072 | 16.696 | 0.000 | 0.000 |
| TESTI4002774 | 4.103 | 0.000 | 0.000 |
| TESTI4002799 | 1.767 | 0.000 | 0.000 |
| TESTI4003602 | 25. 967 | 0.000 | 0.000 |
| TESTI4004210 | 36.051 | 0.000 | 0.000 |
| TESTI4005399 | 7.797 | 0.000 | 0.000 |
| TESTI4005653 | 1.989 | 0.000 | 0.000 |
| TESTI4006441 | 60.471 | 0.000 | 0.000 |
| TESTI4013474 | 0.000 | 3.733 | 0.000 |
| TESTI4014908 | 36.051 | 0.000 | 0.000 |
| TESTI4022158 | 53.386 | 0. 000 | 0.000 |
| THYMU3000776 | 15.604 | 0.000 | 0.000 |
| THYMU3002887 | 4.680 | 0.000 | 0.000 |
| THYMU3003350 | 54.530 | 0.000 | 0.000 |
| THYMU3021586 | 20.651 | 0.000 | 0.000 |
| THYMU3026000 | 49.066 | 0.000 | 0.000 |
| THYMU3026306 | 46.281 | 0.000 | 0.000 |
| THYMU3026350 | 14.291 | 0.000 | 0.000 |
| THYMU3032798 | 9.668 | 0.000 | 0.000 |
| THYMU3032867 | 12.871 | 0.000 | 0.000 |
| THYMU3037827 | 54.530 | 0.000 | 0.000 |
| THYMU3038214 | 39.314 | 0.000 | 0.000 |
| THYMU3041428 | 0.000 | 0.000 | 96.263 |
| THYMU3044075 | 37.925 | 0.000 | 0.000 |
| TRACH2013585 | 23.471 | 0.000 | 0.000 |
| TRACH3002752 | 10.215 | 0.000 | 0.000 |
| TRACH3003037 | 64.100 | 0.000 | 0.000 |
| TRACH3003872 | 2.745 | 0.000 | 0.000 |
| TRACH3004424 | 17.006 | 0.000 | 0.000 |
| TRACH3006717 | 18.347 | 0.000 | 0.000 |
| TRACH3007625 | 1.175 | 0.000 | 0.000 |
| TRACH3007689 | 47.167 | 0.000 | 0.000 |
| TRACH3008632 | 25.351 | 0.000 | 0.000 |
| TRACH3009008 | 24.495 | 0.000 | 0.000 |
| TRACH3010079 | 48.305 | 0.000 | 0.000 |
| TRACH3016805 | 4.502 | 0.000 | 0.000 |
| TRACH3016885 | 3.978 | 0.000 | 0.000 |
| TRACH3024342 | 47.167 | 0.000 | 0.000 |
| TRACH3024671 | 47.167 | 0.000 | 0.000 |
| TRACH3026303 | 5.605 | 0.000 | 0.000 |
| TRACH3026676 | 11.631 | 0.000 | 0.000 |
| TRACH3028855 | 22.309 | 0.000 | 0.000 |
| TRACH3032570 | 47.167 | 0.000 | 0.000 |
| TRACH3036932 | 14.659 | 0.000 | 0.000 |
| TRACH3038399 | 12.435 | 0.000 | 0.000 |
| UTERU3010409 | 3.461 | 0.000 | 0.000 |
| UTERU3013167 | 45.606 | 0.000 | 0.000 |
| BRACE3046450 | 21.984 | 0.000 | 0.000 |
| BRAWH3013197 | 100.000 | 0.000 | 0.000 |
| BRAWH3028645 | 100.000 | 0.000 | 0.000 |
| BRTHA3004432 | 32.087 | 0.000 | 0.000 |
| TRACH1000193 | 21.722 | 0.000 | 0.000 |

The result of comparative analysis of cDNA libraries derived from the substantia nigra (BRSSN), and from whole tissues of a normal brain (BRAWH) showed following genes whose expression levels differed between the two.

**Table 5**

| Clone ID | BRAWH | BRSSN |
|---|---|---|
| ASTRO2016114 | 14.497 | 0.000 |
| BRACE2012528 | 3.794 | 0.000 |
| BRACE2017844 | 0.000 | 38.254 |
| BRACE3004371 | 11.270 | 0.000 |
| BRACE3004767 | 58.488 | 0.000 |
| BRACE3022340 | 58.488 | 0.000 |
| BRACE3025719 | 0.000 | 67.040 |
| BRACE3026802 | 0.000 | 63.610 |
| BRACE3031185 | 58.488 | 0.000 |
| BRACE3031743 | 5.455 | 0.000 |
| BRACE3032385 | 13.674 | 0.000 |
| BRACE3032631 | 12.612 | 0.000 |
| BRACE3039358 | 41.331 | 0.000 |
| BRACE3040863 | 17.888 | 0.000 |
| BRACE3042432 | 58.488 | 0.000 |
| BRACE3045981 | 26.434 | 0.000 |
| BRAMY2031516 | 14.772 | 0.000 |
| BRAMY3002329 | 20.323 | 0.000 |
| BRAMY3004126 | 49.811 | 0.000 |
| BRAMY3005912 | 21.416 | 0.000 |
| BRAMY3008436 | 5.603 | 10.427 |
| BRAWH2000256 | 100.000 | 0.000 |
| BRAWH2002333 | 100.000 | 0.000 |
| BRAWH2004078 | 10.539 | 0.000 |
| BRAWH2010364 | 34.464 | 0.000 |
| BRAWH2010619 | 100.000 | 0.000 |
| BRAWH2011796 | 100.000 | 0.000 |
| BRAWH2011812 | 13.544 | 0.000 |
| BRAWH2011958 | 100.000 | 0.000 |
| BRAWH2012054 | 100.000 | 0.000 |
| BRAWH2012866 | 8.503 | 0.000 |
| BRAWH2013955 | 100.000 | 0.000 |
| BRAWH2014053 | 23.222 | 0.000 |
| BRAWH2016209 | 8.306 | 0.000 |
| BRAWH2016223 | 100.000 | 0.000 |
| BRAWH2016305 | 4.308 | 0.000 |
| BRAWH2016514 | 35.037 | 0.000 |
| BRAWH2016562 | 49.499 | 0.000 |
| BRAWH2016785 | 100.000 | 0.000 |
| BRAWH3000446 | 100.000 | 0.000 |
| BRAWH3000884 | 100.000 | 0.000 |
| BRAWH3001053 | 100.000 | 0.000 |
| BRAWH3001638 | 100.000 | 0.000 |
| BRAWH3001783 | 100.000 | 0.000 |
| BRAWH3001833 | 100.000 | 0.000 |
| BRAWH3003244 | 100.000 | 0.000 |
| BRAWH3003573 | 20.162 | 0.000 |
| BRAWH3003975 | 100.000 | 0.000 |
| BRAWH3004335 | 100.000 | 0.000 |
| BRAWH3004350 | 16.580 | 0.000 |
| BRAWH3005037 | 100.000 | 0.000 |
| BRAWH3005886 | 100.000 | 0.000 |
| BRAWH3005892 | 100.000 | 0.000 |
| BRAWH3005896 | 100.000 | 0.000 |
| BRAWH3008167 | 100.000 | 0.000 |
| BRAWH3008559 | 100.000 | 0.000 |
| BRAWH3008867 | 100.000 | 0.000 |
| BRAWH3009961 | 47.417 | 0.000 |
| BRAWH3010461 | 16.435 | 7.646 |
| BRAWH3010602 | 100.000 | 0.000 |
| BRAWH3010657 | 100.000 | 0.000 |
| BRAWH3010726 | 100.000 | 0.000 |
| BRAWH3010833 | 100.000 | 0.000 |
| BRAWH3011101 | 100.000 | 0.000 |
| BRAWH3011331 | 100.000 | 0.000 |
| BRAWH3011402 | 100.000 | 0.000 |
| BRAWH3011577 | 100.000 | 0.000 |
| BRAWH3011623 | 100.000 | 0.000 |
| BRAWH3011685 | 100.000 | 0.000 |
| BRAWH3011907 | 47.417 | 0.000 |
| BRAWH3011929 | 100.000 | 0.000 |
| BRAWH3012005 | 100.000 | 0.000 |
| BRAWH3012662 | 33.972 | 0.000 |
| BRAWH3012779 | 100.000 | 0.000 |
| BRAWH3013009 | 100.000 | 0.000 |
| BRAWH3013049 | 100.000 | 0.000 |
| BRAWH3013264 | 100.000 | 0.000 |
| BRAWH3013508 | 100.000 | 0.000 |
| BRAWH3014609 | 44.597 | 0.000 |
| BRAWH3014639 | 100.000 | 0.000 |
| BRAWH3015017 | 100.000 | 0.000 |
| BRAWH3015175 | 100.000 | 0.000 |
| BRAWH3015610 | 100.000 | 0.000 |
| BRAWH3015825 | 100.000 | 0.000 |
| BRAWH3016123 | 100.000 | 0.000 |
| BRAWH3016715 | 100.000 | 0.000 |
| BRAWH3017180 | 28.251 | 0.000 |
| BRAWH3017259 | 13.996 | 0.000 |
| BRAWH3017260 | 100.000 | 0.000 |
| BRAWH3017477 | 100.000 | 0.000 |
| BRAWH3017980 | 49.811 | 0.000 |
| BRAWH3018063 | 15.836 | 0.000 |
| BRAWH3018369 | 100.000 | 0.000 |
| BRAWH3018548 | 100.000 | 0.000 |
| BRAWH3018969 | 100.000 | 0.000 |
| BRAWH3019026 | 100.000 | 0.000 |
| BRAWH3019529 | 100.000 | 0.000 |
| BRAWH3019594 | 100.000 | 0.000 |
| BRAWH3019820 | 100.000 | 0.000 |
| BRAWH3020200 | 100.000 | 0.000 |
| BRAWH3020318 | 100.000 | 0.000 |
| BRAWH3020884 | 100.000 | 0.000 |
| BRAWH3020928 | 49.499 | 0.000 |
| BRAWH3021012 | 100.000 | 0.000 |
| BRAWH3021574 | 9.400 | 17.492 |
| BRAWH3021580 | 100.000 | 0.000 |
| BRAWH3021641 | 100.000 | 0.000 |
| BRAWH3021643 | 100.000 | 0.000 |
| BRAWH3021724 | 100.000 | 0.000 |
| BRAWH3022347 | 100.000 | 0.000 |
| BRAWH3022431 | 100.000 | 0.000 |
| BRAWH3022459 | 100.000 | 0.000 |
| BRAWH3022542 | 100.000 | 0.000 |
| BRAWH3022651 | 9.828 | 6.097 |
| BRAWH3022719 | 100.000 | 0.000 |
| BRAWH3022900 | 100.000 | 0.000 |
| BRAWH3023156 | 100.000 | 0.000 |
| BRAWH3023168 | 100.000 | 0.000 |
| BRAWH3023172 | 0.916 | 0.000 |
| BRAWH3023274 | 100.000 | 0.000 |
| BRAWH3023421 | 100.000 | 0.000 |
| BRAWH3024186 | 19.959 | 0.000 |
| BRAWH3024231 | 100.000 | 0.000 |
| BRAWH3024242 | 31.843 | 0.000 |
| BRAWH3024506 | 100.000 | 0.000 |
| BRAWH3024989 | 2.369 | 0.000 |
| BRAWH3026349 | 100.000 | 0.000 |
| BRAWH3026938 | 6.894 | 0.000 |
| BRAWH3027420 | 100.000 | 0.000 |
| BRAWH3027440 | 58.488 | 0.000 |
| BRAWH3027533 | 11.008 | 0.000 |
| BRAWH3027574 | 47.417 | 0.000 |
| BRAWH3027607 | 100.000 | 0.000 |
| BRAWH3027616 | 100.000 | 0.000 |
| BRAWH3027675 | 100.000 | 0.000 |
| BRAWH3027806 | 100.000 | 0.000 |
| BRAWH3027880 | 100.000 | 0.000 |
| BRAWH3028202 | 100.000 | 0.000 |
| BRAWH3028223 | 100.000 | 0.000 |
| BRAWH3028461 | 100.000 | 0.000 |
| BRAWH3028754 | 100.000 | 0.000 |
| BRAWH3028796 | 100.000 | 0.000 |
| BRAWH3029313 | 100.000 | 0.000 |
| BRAWH3029385 | 35.187 | 0.000 |
| BRAWH3029538 | 100.000 | 0.000 |
| BRAWH3029806 | 26.735 | 0.000 |
| BRAWH3030772 | 100.000 | 0.000 |
| BRAWH3030810 | 21.287 | 0.000 |
| BRAWH3030910 | 100.000 | 0.000 |
| BRAWH3031054 | 100.000 | 0.000 |
| BRAWH3031342 | 10.856 | 0.000 |
| BRAWH3031710 | 100.000 | 0.000 |
| BRAWH3032298 | 100.000 | 0.000 |
| BRAWH3032340 | 100.000 | 0.000 |
| BRAWH3032571 | 100.000 | 0.000 |
| BRAWH3033117 | 100.000 | 0.000 |
| BRAWH3033293 | 100.000 | 0.000 |
| BRAWH3033448 | 100.000 | 0.000 |
| BRAWH3033513 | 100.000 | 0.000 |
| BRAWH3034097 | 100.000 | 0.000 |
| BRAWH3034114 | 100.000 | 0.000 |
| BRAWH3034134 | 100.000 | 0.000 |
| BRAWH3034668 | 100.000 | 0.000 |
| BRAWH3034743 | 16.662 | 0.000 |
| BRAWH3034775 | 100.000 | 0.000 |
| BRAWH3034890 | 100.000 | 0.000 |
| BRAWH3035403 | 35.337 | 0.000 |
| BRAWH3035904 | 100.000 | 0.000 |
| BRAWH3035914 | 100.000 | 0.000 |
| BRAWH3035936 | 70.575 | 0.000 |
| BRAWH3036077 | 100.000 | 0.000 |
| BRAWH3036247 | 54.530 | 0.000 |
| BRAWH3036270 | 100.000 | 0.000 |
| BRAWH3036334 | 6.241 | 0.000 |
| BRAWH3036561 | 100.000 | 0.000 |
| BRAWH3037265 | 44.597 | 0.000 |
| BRAWH3037394 | 100.000 | 0.000 |
| BRAWH3037428 | 5.877 | 21.873 |
| BRAWH3037533 | 100.000 | 0.000 |
| BRAWH3037979 | 100.000 | 0.000 |
| BRAWH3038055 | 100.000 | 0.000 |
| BRAWH3038230 | 100.000 | 0.000 |
| BRAWH3038252 | 100.000 | 0.000 |
| BRAWH3038324 | 100.000 | 0.000 |
| BRCAN2010665 | 19.610 | 0.000 |
| BRCAN2022126 | 4.855 | 0.000 |
| BRCAN2025093 | 21.732 | 0.000 |
| BRCOC2012386 | 11.403 | 0.000 |
| BRHIP2015153 | 20.038 | 0.000 |
| BRHIP2027077 | 2. 020 | 0. 000 |
| BRHIP2029643 | 0.000 | 55.599 |
| BRHIP3001573 | 24.474 | 0.000 |
| BRHIP3002000 | 4.500 | 5.583 |
| BRHIP3003063 | 10.960 | 0.000 |
| BRHIP3003984 | 27.477 | 0.000 |
| BRHIP3004774 | 39.520 | 0.000 |
| BRHIP3007223 | 2.016 | 0.000 |
| BRHIP3007409 | 31.957 | 0.000 |
| BRHIP3008320 | 23.349 | 0.000 |
| BRHIP3014675 | 37.925 | 0.000 |
| BRHIP3017855 | 15.316 | 0.000 |
| BRHIP3018784 | 6.930 | 0.000 |
| BRHIP3020046 | 0.000 | 3.770 |
| BRHIP3021019 | 7.745 | 0.000 |
| BRHIP3028246 | 22.147 | 0.000 |
| BRHIP3028570 | 31.957 | 0.000 |
| BRSSN2004303 | 0.000 | 100.000 |
| BRSSN2004710 | 0.000 | 100.000 |
| BRSSN2008464 | 0.000 | 100.000 |
| BRSSN2011843 | 0.000 | 44.546 |
| BRSSN2012157 | 0.000 | 100.000 |
| BRSSN2012198 | 0.000 | 100.000 |
| BRSSN2013696 | 0.000 | 13.293 |
| BRSSN2015497 | 0.000 | 26.619 |
| BRSSN2018218 | 0.000 | 100.000 |
| BRSTN2010089 | 6.598 | 0.000 |
| BRSTN2011961 | 0.000 | 1.078 |
| BRSTN2012069 | 0.047 | 0.000 |
| BRSTN2016992 | 1.476 | 5.495 |
| BRSTN2017104 | 0.000 | 7.257 |
| BRTHA2033155 | 4.253 | 15.830 |
| BRTHA3003736 | 4.302 | 0.000 |
| BRTHA3005988 | 31.076 | 0.000 |
| BRTHA3010135 | 20.010 | 0.000 |
| BRTHA3010212 | 5.525 | 0.000 |
| BRTHA3011194 | 3.534 | 0.000 |
| BRTHA3011265 | 25.596 | 0.000 |
| BRTHA3017791 | 11.821 | 0.000 |
| BRTHA3020771 | 31.957 | 0.000 |
| BRTHA3021971 | 1.941 | 0.000 |
| BRTHA3023403 | 14.857 | 0.000 |
| CTONG2006235 | 9.828 | 6.097 |
| CTONG2009033 | 25.755 | 0.000 |
| CTONG2011801 | 0.000 | 11.021 |
| CTONG2020582 | 9.594 | 0.000 |
| D9OST2003106 | 6.904 | 0.000 |
| DFNES2001829 | 9.228 | 0.000 |
| KIDNE2010049 | 3.438 | 0.000 |
| MESAN2017133 | 17.548 | 0.000 |
| NT2RI2009233 | 6.767 | 0.000 |
| NT2RI2015533 | 6.216 | 0.000 |
| NT2R13005923 | 19.493 | 0.000 |
| NT2RI3009524 | 35.496 | 0.000 |
| NT2RP7007387 | 6.158 | 0.000 |
| NT2RP8001605 | 29.324 | 0.000 |
| NT2RP8007920 | 1.978 | 7.363 |
| NTONG2008483 | 2.397 | 0.000 |
| OCBBF2003518 | 2.062 | 0.000 |
| OCBBF2018618 | 16.218 | 0.000 |
| OCBBF3001333 | 9.639 | 0.000 |
| OCBBF3004487 | 4.531 | 0.000 |
| PLACE7004103 | 3.880 | 0.000 |
| PLACE7006240 | 25.174 | 0.000 |
| PROST2007444 | 4.374 | 8.140 |
| SMINT2009292 | 0.000 | 13.202 |
| T1ESE2000904 | 0.000 | 8.593 |
| TBAES2007428 | 5.371 | 0.000 |
| TESTI2005112 | 19.455 | 0.000 |
| TESTI2021654 | 9.773 | 0.000 |
| TESTI4002072 | 16.696 | 0.000 |
| TESTI4002774 | 4.103 | 0.000 |
| TESTI4002799 | 1.767 | 0.000 |
| TESTI4003602 | 25.967 | 0.000 |
| TESTI4004210 | 36.051 | 0.000 |
| TESTI4005399 | 7.797 | 0.000 |
| TESTI4005653 | 1.989 | 0.000 |
| TESTI4006441 | 60.471 | 0.000 |
| TESTI4014908 | 36.051 | 0.000 |
| TESTI4022158 | 53.386 | 0.000 |
| THYMU3000776 | 15.604 | 0.000 |
| THYMU3002887 | 4.680 | 2.903 |
| THYMU3003350 | 54.530 | 0.000 |
| THYMU3021586 | 20.651 | 0.000 |
| THYMU3026000 | 49.066 | 0.000 |
| THYMU3026306 | 46.281 | 0.000 |
| THYMU3026350 | 14.291 | 0.000 |
| THYMU3032798 | 9.668 | 0.000 |
| THYMU3032867 | 12.871 | 0.000 |
| THYMU3037827 | 54.530 | 0.000 |
| THYMU3038214 | 39.314 | 0.000 |
| THYMU3044075 | 37.925 | 0.000 |
| TRACH2013585 | 23.471 | 0.000 |
| TRACH3002752 | 10.215 | 19.010 |
| TRACH3003037 | 64.100 | 0.000 |
| TRACH3003872 | 2.745 | 10.216 |
| TRACH3004424 | 17.006 | 0.000 |
| TRACH3006717 | 18.347 | 0.000 |
| TRACH3007625 | 1.175 | 0.000 |
| TRACH3007689 | 47.167 | 0.000 |
| TRACH3008632 | 25.351 | 0.000 |
| TRACH3009008 | 24.495 | 0.000 |
| TRACH3010079 | 48.305 | 0.000 |
| TRACH3016805 | 4.502 | 0.000 |
| TRACH3016885 | 3.978 | 0.000 |
| TRACH3022198 | 0.000 | 30.935 |
| TRACH3024342 | 47.167 | 0.000 |
| TRACH3024671 | 47.167 | 0.000 |
| TRACH3026303 | 5.605 | 0.000 |
| TRACH3026676 | 11.631 | 0.000 |
| TRACH3028855 | 22.309 | 0.000 |
| TRACH3032570 | 47.167 | 0.000 |
| TRACH3036932 | 14.659 | 0.000 |
| TRACH3038399 | 12.435 | 0.000 |
| UTERU3010409 | 3.461 | 0.000 |
| UTERU3013167 | 45.606 | 0.000 |
| BRACE3046450 | 21.984 | 0.000 |
| BRAWH3013197 | 100.000 | 0.000 |
| BRAWH3028645 | 100.000 | 0.000 |
| BRTHA3004432 | 32.087 | 0.000 |
| TRACH1000193 | 21.722 | 0.000 |

The result of comparative analysis of cDNA libraries derived from the hippocampus (BRHIP), and from whole tissues of a normal brain (BRAWH) showed following genes whose expression levels differed between the two.

**Table 6**

| Clone ID | BRAWH | BRHIP |
|---|---|---|
| ASTRO2016114 | 14.497 | 0.000 |
| BRACE2002392 | 0.000 | 5.275 |
| BRACE2012528 | 3.794 | 4.354 |
| BRACE2017359 | 0.000 | 41.817 |
| BRACE2017397 | 0.000 | 4.813 |
| BRACE2017844 | 0.000 | 5.243 |
| BRACE3004046 | 0.000 | 58.973 |
| BRACE3004371 | 11.270 | 7.666 |
| BRACE3004767 | 58.488 | 0.000 |
| BRACE3009416 | 0.000 | 29.118 |
| BRACE3022340 | 58.488 | 0.000 |
| BRACE3027931 | 0.000 | 34.325 |
| BRACE3029021 | 0.000 | 74.193 |
| BRACE3031185 | 58.488 | 0.000 |
| BRACE3031743 | 5.455 | 5.566 |
| BRACE3032385 | 13.674 | 20.927 |
| BRACE3032631 | 12.612 | 0.000 |
| BRACE3036156 | 0.000 | 44.660 |
| BRACE3039358 | 41.331 | 21.084 |
| BRACE3040863 | 17. 888 | 18.250 |
| BRACE3042326 | 0.000 | 35.796 |
| BRACE3042432 | 58.488 | 0.000 |
| BRACE3045078 | 0.000 | 41.817 |
| BRACE3045981 | 26.434 | 0.000 |
| BRAMY2031516 | 14.772 | 7.536 |
| BRAMY3002329 | 20.323 | 34.558 |
| BRAMY3004126 | 49.811 | 0.000 |
| BRAMY3005184 | 0.000 | 22.861 |
| BRAMY3005912 | 21.416 | 0.000 |
| BRAMY3007078 | 0.000 | 50.312 |
| BRAMY3008436 | 5.603 | 27.152 |
| BRAMY4000915 | 0.000 | 50.312 |
| BRAWH2000256 | 100.000 | 0.000 |
| BRAWH2002333 | 100.000 | 0.000 |
| BRAWH2004078 | 10.539 | 0.000 |
| BRAWH2010364 | 34.464 | 17.581 |
| BRAWH2010619 | 100.000 | 0.000 |
| BRAWH2011796 | 100.000 | 0.000 |
| BRAWH2011812 | 13.544 | 0.000 |
| BRAWH2011958 | 100.000 | 0.000 |
| BRAWH2012054 | 100.000 | 0.000 |
| BRAWH2012866 | 8.503 | 4.337 |
| BRAWH2013955 | 100.000 | 0.000 |
| BRAWH2014053 | 23.222 | 0.000 |
| BRAWH2016209 | 8.306 | 0.000 |
| BRAWH2016223 | 100.000 | 0.000 |
| BRAWH2016305 | 4.308 | 0.000 |
| BRAWH2016514 | 35.037 | 35.747 |
| BRAWH2016562 | 49.499 | 50.501 |
| BRAWH2016785 | 100.000 | 0.000 |
| BRAWH3000446 | 100.000 | 0.000 |
| BRAWH3000884 | 100.000 | 0.000 |
| BRAWH3001053 | 100.000 | 0.000 |
| BRAWH3001638 | 100.000 | 0.000 |
| BRAWH3001783 | 100.000 | 0.000 |
| BRAWH3001833 | 100.000 | 0.000 |
| BRAWH3003244 | 100.000 | 0.000 |
| BRAWH3003573 | 20.162 | 0.000 |
| BRAWH3003975 | 100.000 | 0.000 |
| BRAWH3004335 | 100.000 | 0.000 |
| BRAWH3004350 | 16.580 | 0.000 |
| BRAWH3005037 | 100.000 | 0.000 |
| BRAWH3005886 | 100.000 | 0.000 |
| BRAWH3005892 | 100.000 | 0.000 |
| BRAWH3005896 | 100.000 | 0.000 |
| BRAWH3008167 | 100.000 | 0.000 |
| BRAWH3008559 | 100.000 | 0.000 |
| BRAWH3008867 | 100.000 | 0.000 |
| BRAWH3009961 | 47.417 | 0.000 |
| BRAWH3010461 | 16.435 | 8.384 |
| BRAWH3010602 | 100.000 | 0.000 |
| BRAWH3010657 | 100.000 | 0.000 |
| BRAWH3010726 | 100.000 | 0.000 |
| BRAWH3010833 | 100.000 | 0.000 |
| BRAWH3011101 | 100.000 | 0.000 |
| BRAWH3011331 | 100.000 | 0.000 |
| BRAWH3011402 | 100.000 | 0.000 |
| BRAWH3011577 | 100.000 | 0.000 |
| BRAWH3011623 | 100.000 | 0.000 |
| BRAWH3011685 | 100.000 | 0.000 |
| BRAWH3011907 | 47.417 | 0.000 |
| BRAWH3011929 | 100.000 | 0.000 |
| BRAWH3012005 | 100.000 | 0.000 |
| BRAWH3012662 | 33.972 | 11.553 |
| BRAWH3012779 | 100.000 | 0.000 |
| BRAWH3013009 | 100.000 | 0.000 |
| BRAWH3013049 | 100.000 | 0.000 |
| BRAWH3013264 | 100.000 | 0.000 |
| BRAWH3013508 | 100.000 | 0.000 |
| BRAWH3014609 | 44.597 | 0.000 |
| BRAWH3014639 | 100.000 | 0.000 |
| BRAWH3015017 | 100.000 | 0.000 |
| BRAWH3015175 | 100.000 | 0.000 |
| BRAWH3015610 | 100.000 | 0.000 |
| BRAWH3015825 | 100.000 | 0.000 |
| BRAWH3016123 | 100.000 | 0.000 |
| BRAWH3016715 | 100.000 | 0.000 |
| BRAWH3017180 | 28.251 | 0.000 |
| BRAWH3017259 | 13.996 | 0.000 |
| BRAWH3017260 | 100.000 | 0.000 |
| BRAWH3017477 | 100.000 | 0.000 |
| BRAWH3017980 | 49.811 | 0.000 |
| BRAWH3018063 | 15.836 | 0.000 |
| BRAWH3018369 | 100.000 | 0.000 |
| BRAWH3018548 | 100.000 | 0.000 |
| BRAWH3018969 | 100.000 | 0.000 |
| BRAWH3019026 | 100.000 | 0.000 |
| BRAWH3019529 | 100.000 | 0.000 |
| BRAWH3019594 | 100.000 | 0.000 |
| BRAWH3019820 | 100.000 | 0.000 |
| BRAWH3020200 | 100.000 | 0.000 |
| BRAWH3020318 | 100.000 | 0.000 |
| BRAWH3020884 | 100.000 | 0.000 |
| BRAWH3020928 | 49.499 | 50.501 |
| BRAWH3021012 | 100.000 | 0.000 |
| BRAWH3021574 | 9.400 | 0.000 |
| BRAWH3021580 | 100.000 | 0.000 |
| BRAWH3021641 | 100.000 | 0.000 |
| BRAWH3021643 | 100.000 | 0.000 |
| BRAWH3021724 | 100.000 | 0.000 |
| BRAWH3022347 | 100.000 | 0.000 |
| BRAWH3022431 | 100.000 | 0.000 |
| BRAWH3022459 | 100.000 | 0.000 |
| BRAWH3022542 | 100.000 | 0.000 |
| BRAWH3022651 | 9.828 | 5.014 |
| BRAWH3022719 | 100.000 | 0.000 |
| BRAWH3022900 | 100.000 | 0.000 |
| BRAWH3023156 | 100.000 | 0.000 |
| BRAWH3023168 | 100.000 | 0.000 |
| BRAWH3023172 | 0.916 | 1.869 |
| BRAWH3023274 | 100.000 | 0.000 |
| BRAWH3023421 | 100.000 | 0.000 |
| BRAWH3024186 | 19.959 | 10.182 |
| BRAWH3024231 | 100.000 | 0.000 |
| BRAWH3024242 | 31.843 | 32.488 |
| BRAWH3024506 | 100.000 | 0.000 |
| BRAWH3024989 | 2.369 | 4.834 |
| BRAWH3026349 | 100.000 | 0.000 |
| BRAWH3026938 | 6.894 | 2.344 |
| BRAWH3027420 | 100.000 | 0.000 |
| BRAWH3027440 | 58.488 | 0.000 |
| BRAWH3027533 | 11.008 | 0.000 |
| BRAWH3027574 | 47.417 | 0.000 |
| BRAWH3027607 | 100.000 | 0.000 |
| BRAWH3027616 | 100.000 | 0.000 |
| BRAWH3027675 | 100.000 | 0.000 |
| BRAWH3027806 | 100.000 | 0.000 |
| BRAWH3027880 | 100.000 | 0.000 |
| BRAWH3028202 | 100.000 | 0.000 |
| BRAWH3028223 | 100.000 | 0.000 |
| BRAWH3028461 | 100.000 | 0.000 |
| BRAWH3028754 | 100.000 | 0.000 |
| BRAWH3028796 | 100.000 | 0.000 |
| BRAWH3029313 | 100.000 | 0.000 |
| BRAWH3029385 | 35.187 | 17.949 |
| BRAWH3029538 | 100.000 | 0.000 |
| BRAWH3029806 | 26.735 | 0.000 |
| BRAWH3030772 | 100.000 | 0.000 |
| BRAWH3030810 | 21.287 | 0.000 |
| BRAWH3030910 | 100.000 | 0.000 |
| BRAWH3031054 | 100.000 | 0.000 |
| BRAWH3031342 | 10.856 | 0.000 |
| BRAWH3031710 | 100.000 | 0.000 |
| BRAWH3032298 | 100.000 | 0.000 |
| BRAWH3032340 | 100.000 | 0.000 |
| BRAWH3032571 | 100.000 | 0.000 |
| BRAWH3033117 | 100.000 | 0.000 |
| BRAWH3033293 | 100.000 | 0.000 |
| BRAWH3033448 | 100.000 | 0.000 |
| BRAWH3033513 | 100.000 | 0.000 |
| BRAWH3034097 | 100.000 | 0.000 |
| BRAWH3034114 | 100.000 | 0.000 |
| BRAWH3034134 | 100.000 | 0.000 |
| BRAWH3034668 | 100.000 | 0.000 |
| BRAWH3034743 | 16.662 | 21.249 |
| BRAWH3034775 | 100.000 | 0.000 |
| BRAWH3034890 | 100.000 | 0.000 |
| BRAWH3035403 | 35.337 | 0.000 |
| BRAWH3035904 | 100.000 | 0.000 |
| BRAWH3035914 | 100.000 | 0.000 |
| BRAWH3035936 | 70.575 | 0.000 |
| BRAWH3036077 | 100.000 | 0.000 |
| BRAWH3036247 | 54.530 | 0.000 |
| BRAWH3036270 | 100.000 | 0.000 |
| BRAWH3036334 | 6.241 | 19.102 |
| BRAWH3036561 | 100.000 | 0.000 |
| BRAWH3037265 | 44.597 | 0.000 |
| BRAWH3037394 | 100.000 | 0.000 |
| BRAWH3037428 | 5.877 | 0.000 |
| BRAWH3037533 | 100.000 | 0.000 |
| BRAWH3037979 | 100.000 | 0.000 |
| BRAWH3038055 | 100.000 | 0.000 |
| BRAWH3038230 | 100.000 | 0.000 |
| BRAWH3038252 | 100.000 | 0.000 |
| BRAWH3038324 | 100.000 | 0.000 |
| BRCAN2010665 | 19.610 | 13.338 |
| BRCAN2019953 | 0.000 | 9.704 |
| BRCAN2022126 | 4.855 | 4.954 |
| BRCAN2025093 | 21.732 | 0.000 |
| BRCOC2012386 | 11.403 | 0.000 |
| BRHIP2006819 | 0.000 | 100.000 |
| BRHIP2006921 | 0.000 | 100.000 |
| BRHIP2008756 | 0.000 | 100.000 |
| BRHIP2009177 | 0.000 | 100.000 |
| BRHIP2011199 | 0.000 | 55.026 |
| BRHIP2013958 | 0.000 | 36.661 |
| BRHIP2015153 | 20.038 | 20.443 |
| BRHIP2016125 | 0.000 | 100.000 |
| BRHIP2017714 | 0.000 | 100.000 |
| BRHIP2020930 | 0.000 | 100.000 |
| BRHIP2021929 | 0.000 | 100.000 |
| BRHIP2023735 | 0.000 | 100.000 |
| BRHIP2024941 | 0.000 | 100.000 |
| BRHIP2026346 | 0.000 | 100.000 |
| BRHIP2027077 | 2.020 | 2.061 |
| BRHIP2027563 | 0.000 | 100.000 |
| BRH)P2029529 | 0.000 | 100.000 |
| BRHIP2029643 | 0.000 | 15.240 |
| BRHIP2029663 | 0.000 | 45.093 |
| BRHIP3000626 | 0.000 | 100.000 |
| BRHIP3000859 | 0.000 | 100.000 |
| BRHIP3001076 | 0.000 | 46.104 |
| BRHIP3001141 | 0.000 | 100.000 |
| BRHIP3001338 | 0.000 | 100.000 |
| BRHIP3001360 | 0.000 | 58.973 |
| BRHIP3001481 | 0.000 | 38.435 |
| BRHIP3001573 | 24.474 | 24.970 |
| BRHIP3001878 | 0.000 | 100.000 |
| BRHIP3002000 | 4.500 | 3.826 |
| BRHIP3002114 | 0.000 | 39.793 |
| BRHIP3002124 | 0.000 | 100.000 |
| BRHIP3002141 | 0.000 | 16.008 |
| BRHIP3002363 | 0.000 | 100.000 |
| BRHIP3002691 | 0.000 | 16.923 |
| BRHIP3002920 | 0.000 | 64.789 |
| BRHIP3002931 | 0.000 | 100.000 |
| BRHIP3003063 | 10.960 | 55.910 |
| BRHIP3003126 | 0.000 | 74.193 |
| BRHIP3003306 | 0.000 | 60.949 |
| BRHIP3003340 | 0.000 | 100.000 |
| BRHIP3003395 | 0.000 | 100.000 |
| BRHIP3003688 | 0.000 | 100.000 |
| BRHIP3003795 | 0.000 | 45.093 |
| BRHIP3003845 | 0.000 | 10.774 |
| BRHIP3003961 | 0.000 | 19.351 |
| BRHIP3003984 | 27.477 | 28.033 |
| BRHIP3004215 | 0.000 | 13.851 |
| BRHIP3004710 | 0.000 | 100.000 |
| BRHIP3004725 | 0.000 | 100.000 |
| BRHIP3004774 | 39.520 | 60.480 |
| BRHIP3004786 | 0.000 | 100.000 |
| BRHIP3005037 | 0.000 | 82.401 |
| BRHIP3005142 | 0.000 | 60.949 |
| BRHIP3005231 | 0.000 | 100.000 |
| BRHIP3005307 | 0.000 | 46.104 |
| BRHIP3005673 | 0.000 | 52.564 |
| BRHIP3005801 | 0.000 | 19.121 |
| BRHIP3005944 | 0.000 | 100.000 |
| BRHIP3006279 | 0.000 | 100.000 |
| BRHIP3006294 | 0.000 | 40.683 |
| BRHIP3006449 | 0.000 | 100.000 |
| BRHIP3006786 | 0.000 | 100.000 |
| BRHIP3006950 | 0.000 | 100.000 |
| BRHIP3007172 | 0.000 | 100.000 |
| BRHIP3007195 | 0. 000 | 75.737 |
| BRHIP3007223 | 2.016 | 2.057 |
| BRHIP3007291 | 0.000 | 100.000 |
| BRHIP3007409 | 31.957 | 32.604 |
| BRHIP3007424 | 0. 000 | 100.000 |
| BRHIP3007609 | 0.000 | 100.000 |
| BRHIP3007960 | 0.000 | 100.000 |
| BRHIP3008082 | 0.000 | 45.093 |
| BRHIP3008320 | 23. 349 | 47.644 |
| BRHIP3008714 | 0.000 | 100.000 |
| BRHIP3009672 | 0.000 | 100.000 |
| BRHIP3009753 | 0.000 | 1.187 |
| BRHIP3010289 | 0.000 | 100.000 |
| BRHIP3010916 | 0.000 | 45.093 |
| BRHIP3011082 | 0.000 | 100.000 |
| BRHIP3011269 | 0.000 | 100.000 |
| BRHIP3011460 | 0.000 | 100.000 |
| BRHIP3011567 | 0.000 | 100.000 |
| BRHIP3011831 | 0.000 | 100.000 |
| BRHIP3012185 | 0.000 | 100.000 |
| BRHIP3012289 | 0.000 | 100.000 |
| BRHIP3012357 | 0.000 | 100.000 |
| BRHIP3012736 | 0.000 | 33.611 |
| BRHIP3012997 | 0.000 | 2.837 |
| BRHIP3013078 | 0.000 | 100.000 |
| BRHIP3013588 | 0.000 | 60.949 |
| BRHIP3013698 | 0.000 | 100.000 |
| BRHIP3014675 | 37.925 | 19.346 |
| BRHIP3015854 | 0.000 | 100.000 |
| BRHIP3016032 | 0.000 | 100.000 |
| BRHIP3016421 | 0.000 | 100.000 |
| BRHIP3017109 | 0.000 | 100.000 |
| BRHIP3017146 | 0.000 | 66.943 |
| BRHIP3017256 | 0.000 | 100.000 |
| BRHIP3017558 | 0.000 | 100.000 |
| BRHIP3017855 | 15.316 | 11.720 |
| BRHIP3018784 | 6.930 | 3.535 |
| BRHIP3019643 | 0.000 | 100.000 |
| BRHIP3019824 | 0.000 | 100.000 |
| BRHIP3019880 | 0.000 | 100.000 |
| BRHIP3019956 | 0.000 | 100.000 |
| BRHIP3020046 | 0.000 | 2.067 |
| BRHIP3020155 | 0.000 | 100.000 |
| BRHIP3020733 | 0.000 | 55.026 |
| BRHIP3021019 | 7.745 | 7.901 |
| BRHIP3021499 | 0.000 | 100.000 |
| BRHIP3021987 | 0.000 | 36.438 |
| BRHIP3022656 | 0.000 | 100.000 |
| BRHIP3023922 | 0.000 | 100.000 |
| BRHIP3024703 | 0.000 | 100.000 |
| BRHIP3024820 | 0.000 | 100.000 |
| BRHIP3025795 | 0.000 | 100.000 |
| BRHIP3025844 | 0.000 | 100.000 |
| BRHIP3026231 | 0.000 | 100.000 |
| BRHIP3026651 | 0.000 | 100.000 |
| BRHIP3027160 | 0.000 | 100.000 |
| BRHIP3027191 | 0.000 | 100.000 |
| BRHIP3027651 | 0.000 | 100.000 |
| BRHIP3027947 | 0.000 | 100.000 |
| BRHIP3028246 | 22.147 | 22.595 |
| BRHIP3028570 | 31.957 | 32.604 |
| BRHIP3028742 | 0.000 | 100.000 |
| BRSTN2010089 | 6.598 | 0.000 |
| BRSTN2012069 | 0.047 | 0.000 |
| BRSTN2016992 | 1.476 | 2.510 |
| BRTHA2001953 | 0.000 | 9.766 |
| BRTHA2008502 | 0.000 | 22.073 |
| BRTHA2031517 | 0.000 | 47.917 |
| BRTHA2033155 | 4.253 | 0.000 |
| BRTHA2035743 | 0.000 | 34.617 |
| BRTHA3003417 | 0.000 | 31.398 |
| BRTHA3003736 | 4.302 | 6.584 |
| BRTHA3005988 | 31.076 | 0.000 |
| BRTHA3007662 | 0.000 | 64.789 |
| BRTHA3010135 | 20.010 | 0.000 |
| BRTHA3010212 | 5.525 | 0.000 |
| BRTHA3011194 | 3.534 | 10.818 |
| BRTHA3011265 | 25.596 | 0.000 |
| BRTHA3012265 | 0.000 | 64.789 |
| BRTHA3017791 | 11.821 | 4.824 |
| BRTHA3020771 | 31.957 | 32.604 |
| BRTHA3021971 | 1.941 | 1.980 |
| BRTHA3023403 | 14.857 | 15.157 |
| CHONS2002829 | 0.000 | 11.668 |
| CTONG2006235 | 9.828 | 5.014 |
| CTONG2009033 | 25.755 | 26.276 |
| CTONG2020582 | 9.594 | 1.958 |
| D9OST2003106 | 6.904 | 0.000 |
| DFNES2001829 | 9.228 | 18.830 |
| KIDNE2010049 | 3.438 | 0.000 |
| LIVER2008465 | 0.000 | 6.752 |
| MESAN2017133 | 17.548 | 0.000 |
| NT2RI2009233 | 6.767 | 0.000 |
| NT2RI2010795 | 0.000 | 16.827 |
| NT2RI2015533 | 6.216 | 1.586 |
| NT2RI3005923 | 19.493 | 0. 000 |
| NT2RI3009524 | 35.496 | 0. 000 |
| NT2RP7007387 | 6.158 | 2.094 |
| NT2RP8001605 | 29.324 | 0.000 |
| NT2RP8007920 | 1.978 | 0.000 |
| NTONG2008483 | 2.397 | 0.815 |
| OCBBF2000831 | 0.000 | 9.824 |
| OCBBF2003518 | 2.062 | 0.000 |
| OCBBF2018618 | 16.218 | 16.546 |
| OCBBF3001333 | 9.639 | 0.000 |
| OCBBF3004487 | 4.531 | 4.622 |
| PLACE7004103 | 3.880 | 5.278 |
| PLACE7006240 | 25.174 | 0.000 |
| PROST200744 | 4.374 | 5.578 |
| SMINT2012179 | 0.000 | 18.954 |
| SYNOV4004210 | 0.000 | 1.927 |
| TBAES2007428 | 5.371 | 0.000 |
| TESTI2005112 | 19.455 | 0.000 |
| TESTI2005564 | 0. 000 | 3.736 |
| TESTI2021654 | 9. 773 | 0. 000 |
| TESTI4001569 | 0.000 | 43.832 |
| TESTI4002072 | 16. 696 | 20.440 |
| TESTI4002774 | 4. 103 | 4.186 |
| TESTI4002799 | 1.767 | 2.703 |
| TESTI4003602 | 25.967 | 0.000 |
| TESTI4003703 | 0.000 | 4.514 |
| TESTI4003944 | 0.000 | 35.590 |
| TESTI4004210 | 36. 051 | 0: 000 |
| TESTI4005399 | 7.797 | 15.910 |
| TESTI4005653 | 1.989 | 3.551 |
| TESTI4006441 | 60.471 | 0. 000 |
| TESTI4014908 | 36. 051 | 0. 000 |
| TESTI4022158 | 53.386 | 13.617 |
| TESTI4029297 | 0. 000 | 4.514 |
| THYMU3000776 | 15.604 | 0.000 |
| THYMU3002887 | 4.680 | 6.367 |
| THYMU3003007 | 0.000 | 19.391 |
| THYMU3003350 | 54.530 | 0.000 |
| THYMU3007308 | 0.000 | 39.793 |
| THYMU3008105 | 0.000 | 20.662 |
| THYMU3021586 | 20.651 | 16.855 |
| THYMU3026000 | 49.066 | 0.000 |
| THYMU3026306 | 46.281 | 0.000 |
| THYMU3026350 | 14.291 | 14.581 |
| THYMU3032798 | 9.668 | 0.000 |
| THYMU3032867 | 12.871 | 0.000 |
| THYMU3034671 | 0.000 | 4.866 |
| THYMU3037827 | 54.530 | 0.000 |
| THYMU3038214 | 39.314 | 0.000 |
| THYMU3044075 | 37.925 | 19.346 |
| TKIDN2000319 | 0.000 | 21.607 |
| TRACH2013585 | 23.471 | 23.946 |
| TRACH3002752 | 10.215 | 10.422 |
| TRACH3003037 | 64.100 | 0.000 |
| TRACH3003872 | 2.745 | 5.600 |
| TRACH3004424 | 17.006 | 0.000 |
| TRACH3006717 | 18. 347 | 11. 231 |
| TRACH3007625 | 1.175 | 10.794 |
| TRACH3007689 | 47.167 | 0.000 |
| TRACH3008632 | 25.351 | 8.621 |
| TRACH3009008 | 24.495 | 0.000 |
| TRACH3010079 | 48.305 | 24.641 |
| TRACH3016805 | 4.502 | 0.000 |
| TRACH3016885 | 3.978 | 4.059 |
| TRACH3022198 | 0.000 | 8.480 |
| TRACH3024342 | 47.167 | 0.000 |
| TRACH3024671 | 47.167 | 0.000 |
| TRACH3025316 | 0.000 | 31.291 |
| TRACH3026303 | 5.605 | 11.436 |
| TRACH3026676 | 11.631 | 0.000 |
| TRACH3028855 | 22.309 | 0.000 |
| TRACH3032570 | 47.167 | 0.000 |
| TRACH3036932 | 14.659 | 14.956 |
| TRACH3038399 | 12.435 | 0.000 |
| TSTOM2000235 | 0.000 | 4.535 |
| UTERU3005422 | 0.000 | 21.641 |
| UTERU3010409 | 3.461 | 7.062 |
| UTERU3013167 | 45.606 | 0.000 |
| UTERU3016273 | 0.000 | 46.104 |
| BRACE3046450 | 21.984 | 0.000 |
| BRAWH3013197 | 100.000 | 0.000 |
| BRAWH3028645 | 100.000 | 0.000 |
| BRTHA3004432 | 32.087 | 0.000 |
| TRACH1000193 | 21.722 | 0.000 |

The result of comparative analysis of cDNA libraries derived from the cerebellum (BRACE), and from whole tissues of a normal brain (BRAWH) showed following genes whose expression levels differed between the two.

**Table 7**

| Clone ID | BRAWH | BRACE |
|---|---|---|
| ASTRO2016114 | 14.497 | 0.000 |
| BRACE1000475 | 0.000 | 100.000 |
| BRACE2002392 | 0.000 | 3.670 |
| BRACE2003628 | 0.000 | 9.684 |
| BRACE2005991 | 0.000 | 100.000 |
| BRACE2010336 | 0.000 | 9.658 |
| BRACE2012528 | 3.794 | 15.820 |
| BRACE2012625 | 0.000 | 100.000 |
| BRACE2012833 | 0.000 | 100.000 |
| BRACE2012838 | 0.000 | 100.000 |
| BRACE2012936 | 0.000 | 100.000 |
| BRACE2012947 | 0.000 | 100.000 |
| BRACE2013009 | 0.000 | 100.000 |
| BRACE2013126 | 0.000 | 100.000 |
| BRACE2013132 | 0.000 | 100.000 |
| BRACE2016896 | 0.000 | 100.000 |
| BRACE2017359 | 0.000 | 58.183 |
| BRACE2017397 | 0.000 | 3.348 |
| BRACE2017580 | 0.000 | 100.000 |
| BRACE2017844 | 0.000 | 29.179 |
| BRACE2017872 | 0.000 | 100.000 |
| BRACE2017992 | 0.000 | 100.000 |
| BRACE2019348 | 0.000 | 74.228 |
| BRACE2023633 | 0.000 | 100.000 |
| BRACE2023744 | 0.000 | 100.000 |
| BRACE2025452 | 0.000 | 100.000 |
| BRACE2026404 | 0.000 | 100.000 |
| BRACE2027312 | 0.000 | 100.000 |
| BRACE2027382 | 0.000 | 100.000 |
| BRACE2028956 | 0.000 | 100.000 |
| BRACE2030039 | 0.000 | 100.000 |
| BRACE2032584 | 0.000 | 100.000 |
| BRACE2033128 | 0.000 | 100.000 |
| BRACE2034434 | 0.000 | 100.000 |
| BRACE2035120 | 0.000 | 100.000 |
| BRACE2035191 | 0.000 | 100.000 |
| BRACE2039362 | 0.000 | 100.000 |
| BRACE2039607 | 0.000 | 100.000 |
| BRACE2042541 | 0.000 | 100.000 |
| BRACE2046976 | 0.000 | 100.000 |
| BRACE2047232 | 0.000 | 100.000 |
| BRACE2047975 | 0.000 | 100.000 |
| BRACE3001403 | 0.000 | 100.000 |
| BRACE3001973 | 0.000 | 36.360 |
| BRACE3002344 | 0.000 | 100.000 |
| BRACE3002541 | 0.000 | 100.000 |
| BRACE3002756 | 0.000 | 100.000 |
| BRACE3003866 | 0.000 | 100.000 |
| BRACE3004046 | 0.000 | 41.027 |
| BRACE3004371 | 11.270 | 10.666 |
| BRACE3004767 | 58.488 | 41.512 |
| BRACE3004887 | 0.000 | 100.000 |
| BRACE3004981 | 0.000 | 100.000 |
| BRACE3005870 | 0.000 | 100.000 |
| BRACE3005903 | 0.000 | 100.000 |
| BRACE3006553 | 0.000 | 18.271 |
| BRACE3007649 | 0.000 | 100.000 |
| BRACE3007869 | 0.000 | 100.000 |
| BRACE3009075 | 0.000 | 100.000 |
| BRACE3009265 | 0.000 | 100.000 |
| BRACE3009392 | 0.000 | 100.000 |
| BRACE3009416 | 0.000 | 20.257 |
| BRACE3009539 | 0.000 | 100.000 |
| BRACE3010702 | 0.000 | 100.000 |
| BRACE3011447 | 0.000 | 100.000 |
| BRACE3011774 | 0.000 | 100.000 |
| BRACE3013418 | 0.000 | 100.000 |
| BRACE3013874 | 0.000 | 100.000 |
| BRACE3013986 | 0.000 | 100.000 |
| BRACE3014523 | 0.000 | 100.000 |
| BRACE3014714 | 0.000 | 100.000 |
| BRACE3015090 | 0.000 | 100.000 |
| BRACE3015898 | 0.000 | 100.000 |
| BRACE3016020 | 0.000 | 100.000 |
| BRACE3016167 | 0.000 | 100.000 |
| BRACE3016580 | 0.000 | 100.000 |
| BRACE3016788 | 0.000 | 100.000 |
| BRACE3016810 | 0.000 | 100.000 |
| BRACE3016862 | 0.000 | 100.000 |
| BRACE3017253 | 0.000 | 55.894 |
| BRACE3018083 | 0.000 | 100.000 |
| BRACE3019570 | 0.000 | 100.000 |
| BRACE3019611 | 0.000 | 100.000 |
| BRACE3019817 | 0.000 | 100.000 |
| BRACE3019941 | 0.000 | 2.074 |
| BRACE3020356 | 0.000 | 100.000 |
| BRACE3020669 | 0.000 | 38.787 |
| BRACE3021430 | 0.000 | 8.467 |
| BRACE3021517 | 0.000 | 100.000 |
| BRACE3021805 | 0.000 | 100.000 |
| BRACE3022051 | 0.000 | 100.000 |
| BRACE3022303 | 0.000 | 100.000 |
| BRACE3022312 | 0.000 | 24.151 |
| BRACE3022340 | 58.488 | 41.512 |
| BRACE3022847 | 0.000 | 100.000 |
| BRACE3023604 | 0.000 | 100.000 |
| BRACE3024379 | 0.000 | 100.000 |
| BRACE3024444 | 0.000 | 100.000 |
| BRACE3024497 | 0.000 | 100.000 |
| BRACE3024537 | 0.000 | 100.000 |
| BRACE3024879 | 0.000 | 100.000 |
| BRACE3025627 | 0.000 | 100.000 |
| BRACE3025719 | 0.000 | 12.784 |
| BRACE3026161 | 0.000 | 100.000 |
| BRACE3026290 | 0.000 | 100.000 |
| BRACE3026345 | 0.000 | 100.000 |
| BRACE3026456 | 0.000 | 100.000 |
| BRACE3026802 | 0.000 | 36.390 |
| BRACE3026844 | 0.000 | 100.000 |
| BRACE3026947 | 0.000 | 100.000 |
| BRACE3027256 | 0.000 | 100.000 |
| BRACE3027931 | 0.000 | 23.879 |
| BRACE3028360 | 0.000 | 100.000 |
| BRACE3028895 | 0.000 | 32.302 |
| BRACE3028998 | 0.000 | 100.000 |
| BRACE3029005 | 0.000 | 100.000 |
| BRACE3029021 | 0.000 | 25.807 |
| BRACE3029205 | 0.000 | 100.000 |
| BRACE3029447 | 0.000 | 100.000 |
| BRACE3030538 | 0.000 | 36.360 |
| BRACE3031161 | 0.000 | 100.000 |
| BRACE3031184 | 0.000 | 100.000 |
| BRACE3031185 | 58.488 | 41.512 |
| BRACE3031315 | 0.000 | 41.329 |
| BRACE3031372 | 0.000 | 100.000 |
| BRACE3031579 | 0.000 | 100.000 |
| BRACE3031728 | 0.000 | 100.000 |
| BRACE3031743 | 5.455 | 15.488 |
| BRACE3031843 | 0.000 | 100.000 |
| BRACE3032385 | 13.674 | 19.411 |
| BRACE3032537 | 0.000 | 100.000 |
| BRACE3032538 | 0.000 | 100.000 |
| BRACE3032631 | 12.612 | 8.951 |
| BRACE3032980 | 0.000 | 100.000 |
| BRACE3033525 | 0.000 | 100.000 |
| BRACE3034183 | 0.000 | 100.000 |
| BRACE3034389 | 0.000 | 100.000 |
| BRACE3034964 | 0.000 | 100.000 |
| BRACE3034993 | 0.000 | 100.000 |
| BRACE3035168 | 0.000 | 29.854 |
| BRACE3036156 | 0.000 | 31.069 |
| BRACE3036271 | 0.000 | 100.000 |
| BRACE3036283 | 0.000 | 5.298 |
| BRACE3037612 | 0.000 | 100.000 |
| BRACE3037637 | 0.000 | 100.000 |
| BRACE3037803 | 0.000 | 100.000 |
| BRACE3038012 | 0.000 | 100.000 |
| BRACE3038030 | 0.000 | 100.000 |
| BRACE3038570 | 0.000 | 100.000 |
| BRACE3038760 | 0.000 | 100.000 |
| BRACE3039288 | 0.000 | 100.000 |
| BRACE3039358 | 41.331 | 14.668 |
| BRACE3039378 | 0.000 | 100.000 |
| BRACE3039454 | 0.000 | 100.000 |
| BRACE3040012 | 0.000 | 100.000 |
| BRACE3040239 | 0.000 | 100.000 |
| BRACE3040504 | 0.000 | 100.000 |
| BRACE3040644 | 0.000 | 100.000 |
| BRACE3040863 | 17.888 | 12.696 |
| BRACE3041059 | 0.000 | 100.000 |
| BRACE3041162 | 0.000 | 100.000 |
| BRACE3041827 | 0.000 | 100.000 |
| BRACE3042046 | 0.000 | 100.000 |
| BRACE3042210 | 0.000 | 100.000 |
| BRACE3042326 | 0.000 | 24.903 |
| BRACE3042409 | 0.000 | 100.000 |
| BRACE3042432 | 58.488 | 41.512 |
| BRACE3042594 | 0.000 | 100.000 |
| BRACE3043597 | 0.000 | 100.000 |
| BRACE3044090 | 0.000 | 100.000 |
| BRACE3044172 | 0.000 | 100.000 |
| BRACE3044247 | 0.000 | 100.000 |
| BRACE3044377 | 0.000 | 100.000 |
| BRACE3044495 | 0.000 | 100.000 |
| BRACE3045078 | 0.000 | 58.183 |
| BRACE3045145 | 0.000 | 100.000 |
| BRACE3045424 | 0.000 | 100.000 |
| BRACE3045708 | 0.000 | 100.000 |
| BRACE3045981 | 26.434 | 56.286 |
| BRACE3046049 | 0.000 | 100.000 |
| BRACE3046152 | 0.000 | 100.000 |
| BRACE3046294 | 0.000 | 100.000 |
| BRACE3046466 | 0.000 | 100.000 |
| BRACE3046491 | 0.000 | 100.000 |
| BRACE3046609 | 0.000 | 100.000 |
| BRACE3046837 | 0.000 | 100.000 |
| BRACE3046855 | 0.000 | 100.000 |
| BRACE3046966 | 0.000 | 100.000 |
| BRACE3047018 | 0.000 | 100.000 |
| BRACE3047482 | 0.000 | 100.000 |
| BRACE3047801 | 0.000 | 100.000 |
| BRAMY2031516 | 14.772 | 0.000 |
| BRAMY3002329 | 20.323 | 9.616 |
| BRAMY3004126 | 49.811 | 0.000 |
| BRAMY3004364 | 0.000 | 4.821 |
| BRAMY3005912 | 21.416 | 30.401 |
| BRAMY3008436 | 5.603 | 5.965 |
| BRAMY3009491 | 0.000 | 22.146 |
| BRAWH2000256 | 100.000 | 0.000 |
| BRAWH2002333 | 100.000 | 0.000 |
| BRAWH2004078 | 10.539 | 3.740 |
| BRAWH2010364 | 34.464 | 36.692 |
| BRAWH2010619 | 100.000 | 0.000 |
| BRAWH2011796 | 100.000 | 0.000 |
| BRAWH2011812 | 13.544 | 0.000 |
| BRAWH2011958 | 100.000 | 0.000 |
| BRAWH2012054 | 100.000 | 0.000 |
| BRAWH2012866 | 8.503 | 3.017 |
| BRAWH2013955 | 100.000 | 0.000 |
| BRAWH2014053 | 23.222 | 16.482 |
| BRAWH2016209 | 8.306 | 11.791 |
| BRAWH2016223 | 100.000 | 0.000 |
| BRAWH2016305 | 4.308 | 0.000 |
| BRAWH2016514 | 35.037 | 0.000 |
| BRAWH2016562 | 49.499 | 0.000 |
| BRAWH2016785 | 100.000 | 0.000 |
| BRAWH3000446 | 100.000 | 0.000 |
| BRAWH3000884 | 100.000 | 0.000 |
| BRAWH3001053 | 100.000 | 0.000 |
| BRAWH3001638 | 100.000 | 0.000 |
| BRAWH3001783 | 100.000 | 0.000 |
| BRAWH3001833 | 100.000 | 0.000 |
| BRAWH3003244 | 100.000 | 0.000 |
| BRAWH3003573 | 20.162 | 14.310 |
| BRAWH3003975 | 100.000 | 0.000 |
| BRAWH3004335 | 100.000 | 0.000 |
| BRAWH3004350 | 16.580 | 0.000 |
| BRAWH3005037 | 100.000 | 0.000 |
| BRAWH3005886 | 100.000 | 0.000 |
| BRAWH3005892 | 100.000 | 0.000 |
| BRAWH3005896 | 100.000 | 0.000 |
| BRAWH3008167 | 100.000 | 0.000 |
| BRAWH3008559 | 100.000 | 0.000 |
| BRAWH3008867 | 100.000 | 0.000 |
| BRAWH3009961 | 47.417 | 0.000 |
| BRAWH3010461 | 16.435 | 10.207 |
| BRAWH3010602 | 100.000 | 0.000 |
| BRAWH3010657 | 100.000 | 0.000 |
| BRAWH3010726 | 100.000 | 0.000 |
| BRAWH3010833 | 100.000 | 0.000 |
| BRAWH3011101 | 100.000 | 0.000 |
| BRAWH3011331 | 100.000 | 0.000 |
| BRAWH3011402 | 100.000 | 0.000 |
| BRAWH3011577 | 100.000 | 0.000 |
| BRAWH3011623 | 100.000 | 0.000 |
| BRAWH3011685 | 100.000 | 0.000 |
| BRAWH3011907 | 47.417 | 0.000 |
| BRAWH3011929 | 100.000 | 0.000 |
| BRAWH3012005 | 100.000 | 0.000 |
| BRAWH3012662 | 33.972 | 0.000 |
| BRAWH3012779 | 100.000 | 0.000 |
| BRAWH3013009 | 100.000 | 0.000 |
| BRAWH3013049 | 100.000 | 0.000 |
| BRAWH3013264 | 100.000 | 0.000 |
| BRAWH3013508 | 100.000 | 0.000 |
| BRAWH3014609 | 44.597 | 0.000 |
| BRAWH3014639 | 100.000 | 0.000 |
| BRAWH3015017 | 100.000 | 0.000 |
| BRAWH3015175 | 100.000 | 0.000 |
| BRAWH3015610 | 100.000 | 0.000 |
| BRAWH3015825 | 100.000 | 0.000 |
| BRAWH3016123 | 100.000 | 0.000 |
| BRAWH3016715 | 100.000 | 0.000 |
| BRAWH3017180 | 28.251 | 40.103 |
| BRAWH3017259 | 13.996 | 4.967 |
| BRAWH3017260 | 100.000 | 0.000 |
| BRAWH3017477 | 100.000 | 0.000 |
| BRAWH3017980 | 49.811 | 0.000 |
| BRAWH3018063 | 15.836 | 0.000 |
| BRAWH3018369 | 100.000 | 0.000 |
| BRAWH3018548 | 100.000 | 0.000 |
| BRAWH3018969 | 100.000 | 0.000 |
| BRAWH3019026 | 100.000 | 0.000 |
| BRAWH3019529 | 100.000 | 0.000 |
| BRAWH3019594 | 100.000 | 0.000 |
| BRAWH3019820 | 100.000 | 0.000 |
| BRAWH3020200 | 100.000 | 0.000 |
| BRAWH3020318 | 100.000 | 0.000 |
| BRAWH3020884 | 100.000 | 0.000 |
| BRAWH3020928 | 49.499 | 0.000 |
| BRAWH3021012 | 100.000 | 0.000 |
| BRAWH3021574 | 9.400 | 3.336 |
| BRAWH3021580 | 100.000 | 0.000 |
| BRAWH3021641 | 100.000 | 0.000 |
| BRAWH3021643 | 100.000 | 0.000 |
| BRAWH3021724 | 100.000 | 0.000 |
| BRAWH3022347 | 100.000 | 0.000 |
| BRAWH3022431 | 100.000 | 0.000 |
| BRAWH3022459 | 100.000 | 0.000 |
| BRAWH3022542 | 100.000 | 0.000 |
| BRAWH3022651 | 9.828 | 1.163 |
| BRAWH3022719 | 100.000 | 0.000 |
| BRAWH3022900 | 100.000 | 0.000 |
| BRAWH3023156 | 100.000 | 0.000 |
| BRAWH3023168 | 100.000 | 0.000 |
| BRAWH3023172 | 0.916 | 0.000 |
| BRAWH3023274 | 100.000 | 0.000 |
| BRAWH3023421 | 100.000 | 0.000 |
| BRAWH3024186 | 19.959 | 0.000 |
| BRAWH3024231 | 100.000 | 0.000 |
| BRAWH3024242 | 31.843 | 0.000 |
| BRAWH3024506 | 100.000 | 0.000 |
| BRAWH3024989 | 2.369 | 0.000 |
| BRAWH3026349 | 100.000 | 0.000 |
| BRAWH3026938 | 6.894 | 4.893 |
| BRAWH3027420 | 100.000 | 0.000 |
| BRAWH3027440 | 58.488 | 41.512 |
| BRAWH3027533 | 11.008 | 0.000 |
| BRAWH3027574 | 47.417 | 0.000 |
| BRAWH3027607 | 100.000 | 0.000 |
| BRAWH3027616 | 100.000 | 0.000 |
| BRAWH3027675 | 100.000 | 0.000 |
| BRAWH3027806 | 100.000 | 0.000 |
| BRAWH3027880 | 100.000 | 0.000 |
| BRAWH3028202 | 100.000 | 0.000 |
| BRAWH3028223 | 100.000 | 0.000 |
| BRAWH3028461 | 100.000 | 0.000 |
| BRAWH3028754 | 100.000 | 0.000 |
| BRAWH3028796 | 100.000 | 0.000 |
| BRAWH3029313 | 100.000 | 0.000 |
| BRAWH3029385 | 35.187 | 12.487 |
| BRAWH3029538 | 100.000 | 0.000 |
| BRAWH3029806 | 26.735 | 0.000 |
| BRAWH3030772 | 100.000 | 0.000 |
| BRAWH3030810 | 21.287 | 67.989 |
| BRAWH3030910 | 100.000 | 0.000 |
| BRAWH3031054 | 100.000 | 0.000 |
| BRAWH3031342 | 10.856 | 7.705 |
| BRAWH3031710 | 100.000 | 0.000 |
| BRAWH3032298 | 100.000 | 0.000 |
| BRAWH3032340 | 100.000 | 0.000 |
| BRAWH3032571 | 100.000 | 0.000 |
| BRAWH3033117 | 100.000 | 0.000 |
| BRAWH3033293 | 100.000 | 0.000 |
| BRAWH3033448 | 100.000 | 0.000 |
| BRAWH3033513 | 100.000 | 0.000 |
| BRAWH3034097 | 100.000 | 0.000 |
| BRAWH3034114 | 100.000 | 0.000 |
| BRAWH3034134 | 100.000 | 0.000 |
| BRAWH3034668 | 100.000 | 0.000 |
| BRAWH3034743 | 16.662 | 5.913 |
| BRAWH3034775 | 100.000 | 0.000 |
| BRAWH3034890 | 100.000 | 0.000 |
| BRAWH3035403 | 35.337 | 25.081 |
| BRAWH3035904 | 100.000 | 0.000 |
| BRAWH3035914 | 100.000 | 0.000 |
| BRAWH3035936 | 70.575 | 0.000 |
| BRAWH3036077 | 100.000 | 0.000 |
| BRAWH3036247 | 54.530 | 0.000 |
| BRAWH3036270 | 100.000 | 0.000 |
| BRAWH3036334 | 6.241 | 4.430 |
| BRAWH3036561 | 100.000 | 0.000 |
| BRAWH3037265 | 44.597 | 0.000 |
| BRAWH3037394 | 100.000 | 0.000 |
| BRAWH3037428 | 5.877 | 25.026 |
| BRAWH3037533 | 100.000 | 0.000 |
| BRAWH3037979 | 100.000 | 0.000 |
| BRAWH3038055 | 100.000 | 0.000 |
| BRAWH3038230 | 100.000 | 0.000 |
| BRAWH3038252 | 100.000 | 0.000 |
| BRAWH3038324 | 100.000 | 0.000 |
| BRCAN2010665 | 19.610 | 0.000 |
| BRCAN2019953 | 0.000 | 6.751 |
| BRCAN2022126 | 4.855 | 3.446 |
| BRCAN2025093 | 21.732 | 0.000 |
| BRCOC2012386 | 11.403 | 0.000 |
| BRHIP2015153 | 20.038 | 14.222 |
| BRHIP2027077 | 2.020 | 0.000 |
| BRHIP2029643 | 0.000 | 10.603 |
| BRHIP3001360 | 0.000 | 41.027 |
| BRHIP3001573 | 24.474 | 0.000 |
| BRHIP3002000 | 4.500 | 0.532 |
| BRHIP3002114 | 0.000 | 27.684 |
| BRHIP3003063 | 10.960 | 0.000 |
| BRHIP3003126 | 0.000 | 25.807 |
| BRHIP3003961 | 0.000 | 13.462 |
| BRHIP3003984 | 27.477 | 29.254 |
| BRHIP3004215 | 0.000 | 9.636 |
| BRHIP3004774 | 39.520 | 0.000 |
| BRHIP3005801 | 0.000 | 13.302 |
| BRHIP3007223 | 2.016 | 1.431 |
| BRHIP3007409 | 31.957 | 0.000 |
| BRHIP3008320 | 23.349 | 0.000 |
| BRHIP3014675 | 37.925 | 26.918 |
| BRHIP3017855 | 15.316 | 13.588 |
| BRHIP3018784 | 6.930 | 0.000 |
| BRHIP3021019 | 7.745 | 0.000 |
| BRHIP3028246 | 22.147 | 0.000 |
| BRHIP3028570 | 31.957 | 0.000 |
| BRSSN2011843 | 0.000 | 8.495 |
| BRSSN2013696 | 0.000 | 12.675 |
| BRSTN2010089 | 6.598 | 4.683 |
| BRSTN2011961 | 0.000 | 3.083 |
| BRSTN2012069 | 0.047 | 0.517 |
| BRSTN2016992 | 1.476 | 5.588 |
| BRTHA2033155 | 4.253 | 9.056 |
| BRTHA2035743 | 0.000 | 4.816 |
| BRTHA3003736 | 4.302 | 1.527 |
| BRTHA3005988 | 31.076 | 0.000 |
| BRTHA3009858 | 0.000 | 16.462 |
| BRTHA3010135 | 20.010 | 0.000 |
| BRTHA3010212 | 5.525 | 0.000 |
| BRTHA3010530 | 0.000 | 19.846 |
| BRTHA3011194 | 3.534 | 0.000 |
| BRTHA3011265 | 25.596 | 0.000 |
| BRTHA3011998 | 0.000 | 56.141 |
| BRTHA3017791 | 11.821 | 9.229 |
| BRTHA3020771 | 31.957 | 0.000 |
| BRTHA3021708 | 0.000 | 39.025 |
| BRTHA3021971 | 1.941 | 0.000 |
| BRTHA3023403 | 14.857 | 0.000 |
| CHONS2002829 | 0.000 | 1.160 |
| CTONG2006235 | 9.828 | 1.163 |
| CTONG2009033 | 25.755 | 0.000 |
| CTONG2020582 | 9.594 | 1.362 |
| CTONG2027959 | 0.000 | 27.592 |
| D9OST2003106 | 6.904 | 0.000 |
| DFNES2001829 | 9.228 | 6.550 |
| KIDNE2010049 | 3.438 | 0.000 |
| KIDNE2017153 | 0.000 | 16.915 |
| LIVER2008465 | 0.000 | 4.697 |
| MESAN2017133 | 17.548 | 0.000 |
| NOVAR2000783 | 0.000 | 2.684 |
| NT2RI2009233 | 6.767 | 1. 601 |
| NT2RI2015533 | 6.216 | 3.309 |
| NT2RI3005923 | 19.493 | 0.000 |
| NT2RI3009524 | 35.496 | 0.000 |
| NT2RP7007387 | 6.158 | 0.000 |
| NT2RP8001605 | 29.324 | 0.000 |
| NT2RP8007920 | 1.978 | 1.404 |
| NT2RP8009119 | 0.000 | 3.276 |
| NTONG2008483 | 2.397 | 0.000 |
| NTONG2009468 | 0.000 | 13.482 |
| OCBBF2003518 | 2.062 | 0.000 |
| OCBBF2014745 | 0.000 | 36.360 |
| OCBBF2018618 | 16.218 | 0.000 |
| OCBBF3001333 | 9.639 | 4.561 |
| OCBBF3004487 | 4.531 | 6.432 |
| PLACE7004103 | 3.880 | 0.918 |
| PLACE7006240 | 25.174 | 0.000 |
| PROST2007444 | 4.374 | 5.433 |
| TBAES2007428 | 5.371 | 0.000 |
| TESTI2005112 | 19.455 | 0.000 |
| TESTI018867 | 0.000 | 4.042 |
| TESTI2021654 | 9.773 | 0.000 |
| TESTI4002072 | 16.696 | 11.850 |
| TESTI4002774 | 4.103 | 0.000 |
| TESTI4002799 | 1.767 | 0. 627 |
| TESTI4003602 | 25.967 | 18.431 |
| TESTI4003703 | 0. 000 | 6. 281 |
| TESTI4004210 | 36. 051 | 0. 000 |
| TESTI4005013 | 0.000 | 11.599 |
| TESTI4005399 | 7.797 | 11. 068 |
| TESTI4005653 | 1.989 | 1.059 |
| TESTI4006441 | 60.471 | 0. 000 |
| TESTI4014908 | 36. 051 | 0. 000 |
| TESTI4022158 | 53.386 | 9.473 |
| TESTI4029297 | 0. 000 | 6. 281 |
| TESTI4032913 | 0. 000 | 5.753 |
| TESTI4043223 | 0.000 | 19.643 |
| THYMU3000776 | 15.604 | 22.150 |
| THYMU3002887 | 4.680 | 7.197 |
| THYMU3003350 | 54.530 | 0.000 |
| THYMU3007308 | 0.000 | 27.684 |
| THYMU3021586 | 20.651 | 0.000 |
| THYMU3026000 | 49.066 | 0.000 |
| THYMU3026306 | 46.281 | 0.000 |
| THYMU3026350 | 14.291 | 0.000 |
| THYMU3032798 | 9.668 | 10.979 |
| THYMU3032867 | 12.871 | 9.135 |
| THYMU3033626 | 0.000 | 7.979 |
| THYMU3034671 | 0.000 | 5.078 |
| THYMU3037827 | 54.530 | 0.000 |
| THYMU3038214 | 39.314 | 27.904 |
| THYMU3044075 | 37.925 | 26.918 |
| TLIVE2007736 | 0.000 | 9.358 |
| TRACH2013585 | 23.471 | 0.000 |
| TRACH3002752 | 10.215 | 3.625 |
| TRACH3003037 | 64.100 | 0.000 |
| TRACH3003872 | 2.745 | 0.000 |
| TRACH3004424 | 17.006 | 0.000 |
| TRACH3006717 | 18.347 | 10.418 |
| TRACH3007625 | 1.175 | 2.503 |
| TRACH3007689 | 47.167 | 0.000 |
| TRACH3007995 | 0.000 | 38.787 |
| TRACH3008632 | 25.351 | 11.995 |
| TRACH3009008 | 24.495 | 0.000 |
| TRACH3010079 | 48.305 | 0.000 |
| TRACH3016805 | 4.502 | 3.196 |
| TRACH3016885 | 3.978 | 5.647 |
| TRACH3024342 | 47.167 | 0.000 |
| TRACH3024671 | 47.167 | 0.000 |
| TRACH3026303 | 5.605 | 0.000 |
| TRACH3026676 | 11.631 | 0.000 |
| TRACH3028855 | 22.309 | 0.000 |
| TRACH3032570 | 47.167 | 0.000 |
| TRACH3036932 | 14.659 | 10.405 |
| TRACH3038399 | 12.435 | 8.826 |
| UTERU3000670 | 0.000 | 16.553 |
| UTERU3010409 | 3.461 | 17.196 |
| UTERU3013167 | 45.606 | 0.000 |
| UTERU3015011 | 0.000 | 5.009 |
| BRACE3002184 | 0.000 | 19.020 |
| BRACE3026993 | 0.000 | 100.000 |
| BRACE3046450 | 21.984 | 78.016 |
| BRAMY3008096 | 0.000 | 41.329 |
| BRAWH3013197 | 100.000 | 0.000 |
| BRAWH3028645 | 100.000 | 0.000 |
| BRTHA3004432 | 32.087 | 0.000 |
| TESTI4002988 | 0.000 | 15.392 |
| TRACH1000193 | 21.722 | 15.417 |

The result of comparative analysis of cDNA libraries derived from the thalamus (BRTHA), and from whole tissues of a normal brain (BRAWH) showed following genes whose expression levels differed between the two.

**Table 8**

| Clone ID | BRAWH | BRTHA |
|---|---|---|
| ASTRO2008972 | 0.000 | 17.271 |
| ASTRO2016114 | 14.497 | 0.000 |
| BLADE2004849 | 0.000 | 8.220 |
| BRACE2002392 | 0.000 | 1.911 |
| BRACE2012528 | 3.794 | 13.673 |
| BRACE2019348 | 0.000 | 25.772 |
| BRACE3004371 | 11.270 | 4.166 |
| BRACE3004767 | 58.488 | 0.000 |
| BRACE3019941 | 0.000 | 6.480 |
| BRACE3022312 | 0.000 | 37.734 |
| BRACE3022340 | 58.488 | 0.000 |
| BRACE3031185 | 58.488 | 0.000 |
| BRACE3031743 | 5.455 | 0.000 |
| BRACE3032385 | 13.674 | 15.164 |
| BRACE3032631 | 12.612 | 0.000 |
| BRACE3036156 | 0.000 | 24.272 |
| BRACE3039358 | 41.331 | 22.917 |
| BRACE3040863 | 17.888 | 0.000 |
| BRACE3042432 | 58.488 | 0.000 |
| BRACE3045981 | 26.434 | 0.000 |
| BRAMY2031516 | 14.772 | 0.000 |
| BRAMY3002329 | 20.323 | 15.025 |
| BRAMY3004126 | 49.811 | 0.000 |
| BRAMY3005912 | 21.416 | 0.000 |
| BRAMY3008436 | 5.603 | 12.427 |
| BRAMY3009556 | 0.000 | 52.395 |
| BRAMY3010654 | 0.000 | 52.395 |
| BRAMY4001863 | 0.000 | 16.612 |
| BRAWH2000256 | 100.000 | 0.000 |
| BRAWH2002333 | 100.000 | 0.000 |
| BRAWH2004078 | 10.539 | 0.000 |
| BRAWH2010364 | 34.464 | 0.000 |
| BRAWH2010619 | 100.000 | 0.000 |
| BRAWH2011796 | 100.000 | 0.000 |
| BRAWH2011812 | 13.544 | 0.000 |
| BRAWH2011958 | 100.000 | 0.000 |
| BRAWH2012054 | 100.000 | 0.000 |
| BRAWH2012866 | 8.503 | 11.786 |
| BRAWH2013955 | 100.000 | 0.000 |
| BRAWH2014053 | 23.222 | 25.752 |
| BRAWH2016209 | 8.306 | 0.000 |
| BRAWH2016223 | 100.000 | 0.000 |
| BRAWH2016305 | 4.308 | 0.000 |
| BRAWH2016514 | 35.037 | 0.000 |
| BRAWH2016562 | 49.499 | 0.000 |
| BRAWH2016785 | 100.000 | 0.000 |
| BRAWH3000446 | 100.000 | 0.000 |
| BRAWH3000884 | 100.000 | 0.000 |
| BRAWH3001053 | 100.000 | 0.000 |
| BRAWH3001638 | 100.000 | 0.000 |
| BRAWH3001783 | 100.000 | 0.000 |
| BRAWH3001833 | 100.000 | 0.000 |
| BRAWH3003244 | 100.000 | 0.000 |
| BRAWH3003573 | 20.162 | 22.358 |
| BRAWH3003975 | 100.000 | 0.000 |
| BRAWH3004335 | 100.000 | 0.000 |
| BRAWH3004350 | 16.580 | 18.386 |
| BRAWH3005037 | 100.000 | 0.000 |
| BRAWH3005886 | 100.000 | 0.000 |
| BRAWH3005892 | 100.000 | 0.000 |
| BRAWH3005896 | 100.000 | 0.000 |
| BRAWH3008167 | 100.000 | 0.000 |
| BRAWH3008559 | 100.000 | 0.000 |
| BRAWH3008867 | 100.000 | 0.000 |
| BRAWH3009961 | 47.417 | 52.583 |
| BRAWH3010461 | 16.435 | 11.391 |
| BRAWH3010602 | 100.000 | 0.000 |
| BRAWH3010657 | 100.000 | 0.000 |
| BRAWH3010726 | 100.000 | 0.000 |
| BRAWH3010833 | 100.000 | 0.000 |
| BRAWH3011101 | 100.000 | 0.000 |
| BRAWH3011331 | 100.000 | 0.000 |
| BRAWH3011402 | 100.000 | 0.000 |
| BRAWH3011577 | 100.000 | 0.000 |
| BRAWH3011623 | 100.000 | 0.000 |
| BRAWH3011685 | 100.000 | 0.000 |
| BRAWH3011907 | 47.417 | 52.583 |
| BRAWH3011929 | 100.000 | 0.000 |
| BRAWH3012005 | 100.000 | 0.000 |
| BRAWH3012662 | 33.972 | 12.558 |
| BRAWH3012779 | 100.000 | 0.000 |
| BRAWH3013009 | 100.000 | 0.000 |
| BRAWH3013049 | 100.000 | 0.000 |
| BRAWH3013264 | 100.000 | 0.000 |
| BRAWH3013508 | 100.000 | 0.000 |
| BRAWH3014609 | 44.597 | 0.000 |
| BRAWH3014639 | 100.000 | 0.000 |
| BRAWH3015017 | 100.000 | 0.000 |
| BRAWH3015175 | 100.000 | 0.000 |
| BRAWH3015610 | 100.000 | 0.000 |
| BRAWH3015825 | 100.000 | 0.000 |
| BRAWH3016123 | 100.000 | 0.000 |
| BRAWH3016715 | 100.000 | 0.000 |
| BRAWH3017180 | 28.251 | 0.000 |
| BRAWH3017259 | 13.996 | 0.000 |
| BRAWH3017260 | 100.000 | 0.000 |
| BRAWH3017477 | 100.000 | 0.000 |
| BRAWH3017980 | 49.811 | 0.000 |
| BRAWH3018063 | 15.836 | 0.000 |
| BRAWH3018369 | 100.000 | 0.000 |
| BRAWH3018548 | 100.000 | 0.000 |
| BRAWH3018969 | 100.000 | 0.000 |
| BRAWH3019026 | 100.000 | 0.000 |
| BRAWH3019529 | 100.000 | 0.000 |
| BRAWH3019594 | 100.000 | 0.000 |
| BRAWH3019820 | 100.000 | 0.000 |
| BRAWH3020200 | 100.000 | 0.000 |
| BRAWH3020318 | 100.000 | 0.000 |
| BRAWH3020884 | 100.000 | 0.000 |
| BRAWH3020928 | 49.499 | 0.000 |
| BRAWH3021012 | 100.000 | 0.000 |
| BRAWH3021574 | 9.400 | 0.000 |
| BRAWH3021580 | 100.000 | 0.000 |
| BRAWH3021641 | 100.000 | 0.000 |
| BRAWH3021643 | 100.000 | 0.000 |
| BRAWH3021724 | 100.000 | 0.000 |
| BRAWH3022347 | 100.000 | 0.000 |
| BRAWH3022431 | 100.000 | 0.000 |
| BRAWH3022459 | 100.000 | 0.000 |
| BRAWH3022542 | 100.000 | 0.000 |
| BRAWH3022651 | 9.828 | 5.450 |
| BRAWH3022719 | 100.000 | 0.000 |
| BRAWH3022900 | 100.000 | 0.000 |
| BRAWH3023156 | 100.000 | 0.000 |
| BRAWH3023168 | 100.000 | 0.000 |
| BRAWH3023172 | 0.916 | 2.031 |
| BRAWH3023274 | 100.000 | 0.000 |
| BRAWH3023421 | 100.000 | 0.000 |
| BRAWH3024186 | 19.959 | 0.000 |
| BRAWH3024231 | 100.000 | 0.000 |
| BRAWH3024242 | 31.843 | 0.000 |
| BRAWH3024506 | 100.000 | 0.000 |
| BRAWH3024989 | 2.369 | 0.000 |
| BRAWH3026349 | 100.000 | 0.000 |
| BRAWH3026938 | 6.894 | 0.000 |
| BRAWH3027420 | 100.000 | 0.000 |
| BRAWH3027440 | 58.488 | 0.000 |
| BRAWH3027533 | 11.008 | 0.000 |
| BRAWH3027574 | 47.417 | 52.583 |
| BRAWH3027607 | 100.000 | 0.000 |
| BRAWH3027616 | 100.000 | 0.000 |
| BRAWH3027675 | 100.000 | 0.000 |
| BRAWH3027806 | 100.000 | 0.000 |
| BRAWH3027880 | 100.000 | 0.000 |
| BRAWH3028202 | 100.000 | 0.000 |
| BRAWH3028223 | 100.000 | 0.000 |
| BRAWH3028461 | 100.000 | 0.000 |
| BRAWH3028754 | 100.000 | 0.000 |
| BRAWH3028796 | 100.000 | 0.000 |
| BRAWH3029313 | 100.000 | 0.000 |
| BRAWH3029385 | 35.187 | 0.000 |
| BRAWH3029538 | 100.000 | 0.000 |
| BRAWH3029806 | 26.735 | 9.883 |
| BRAWH3030772 | 100.000 | 0.000 |
| BRAWH3030810 | 21.287 | 0.000 |
| BRAWH3030910 | 100.000 | 0.000 |
| BRAWH3031054 | 100.000 | 0.000 |
| BRAWH3031342 | 10.856 | 0.000 |
| BRAWH3031710 | 100.000 | 0.000 |
| BRAWH3032298 | 100.000 | 0.000 |
| BRAWH3032340 | 100.000 | 0.000 |
| BRAWH3032571 | 100.000 | 0.000 |
| BRAWH3033117 | 100.000 | 0.000 |
| BRAWH3033293 | 100.000 | 0.000 |
| BRAWH3033448 | 100.000 | 0.000 |
| BRAWH3033513 | 100.000 | 0.000 |
| BRAWH3034097 | 100.000 | 0.000 |
| BRAWH3034114 | 100.000 | 0.000 |
| BRAWH3034134 | 100.000 | 0.000 |
| BRAWH3034668 | 100.000 | 0.000 |
| BRAWH3034743 | 16.662 | 18.478 |
| BRAWH3034775 | 100.000 | 0.000 |
| BRAWH3034890 | 100.000 | 0.000 |
| BRAWH3035403 | 35.337 | 0.000 |
| BRAWH3035904 | 100.000 | 0.000 |
| BRAWH3035914 | 100.000 | 0.000 |
| BRAWH3035936 | 70.575 | 0.000 |
| BRAWH3036077 | 100.000 | 0.000 |
| BRAWH3036247 | 54.530 | 0.000 |
| BRAWH3036270 | 100.000 | 0.000 |
| BRAWH3036334 | 6.241 | 6.921 |
| BRAWH3036561 | 100.000 | 0.000 |
| BRAWH3037265 | 44.597 | 0.000 |
| BRAWH3037394 | 100.000 | 0.000 |
| BRAWH3037428 | 5.877 | 19.551 |
| BRAWH3037533 | 100.000 | 0.000 |
| BRAWH3037979 | 100.000 | 0.000 |
| BRAWH3038055 | 100.000 | 0.000 |
| BRAWH3038230 | 100.000 | 0.000 |
| BRAWH3038252 | 100.000 | 0.000 |
| BRAWH3038324 | 100.000 | 0.000 |
| BRCAN2002892 | 0.000 | 32.586 |
| BRCAN2010665 | 19.610 | 7.249 |
| BRCAN2020234 | 0.000 | 27.335 |
| BRCAN2022126 | 4.855 | 0.000 |
| BRCAN2025093 | 21.732 | 0.000 |
| BRCOC2006164 | 0.000 | 32.234 |
| BRCOC2012386 | 11.403 | 0.000 |
| BRHIP2013958 | 0.000 | 39.849 |
| BRHIP2015153 | 20.038 | 0.000 |
| BRHIP2027077 | 2.020 | 0.000 |
| BRHIP3001573 | 24.474 | 27.141 |
| BRHIP3002000 | 4.500 | 0.832 |
| BRHIP3002691 | 0.000 | 18.395 |
| BRHIP3002920 | 0.000 | 35.211 |
| BRHIP3003063 | 10.960 | 0.000 |
| BRHIP3003961 | 0.000 | 21.034 |
| BRHIP3003984 | 27.477 | 15.236 |
| BRHIP3004215 | 0.000 | 15.055 |
| BRHIP3004774 | 39.520 | 0.000 |
| BRHIP3007223 | 2.016 | 4.472 |
| BRHIP3007409 | 31.957 | 35.439 |
| BRHIP3008320 | 23.349 | 0.000 |
| BRHIP3014675 | 37.925 | 0.000 |
| BRHIP3017855 | 15.316 | 4.246 |
| BRHIP3018784 | 6.930 | 0.000 |
| BRHIP3020046 | 0.000 | 1.123 |
| BRHIP3021019 | 7.745 | 0.000 |
| BRHIP3028246 | 22.147 | 0.000 |
| BRHIP3028570 | 31.957 | 35.439 |
| BRSSN2015497 | 0.000 | 7.931 |
| BRSTN2010089 | 6.598 | 0.000 |
| BRSTN2011961 | 0.000 | 1.285 |
| BRSTN2012069 | 0.047 | 0.000 |
| BRSTN2016992 | 1.476 | 1.091 |
| BRTHA2000969 | 0.000 | 100.000 |
| BRTHA2001304 | 0.000 | 100.000 |
| BRTHA2001953 | 0.000 | 10.615 |
| BRTHA2002091 | 0.000 | 100.000 |
| BRTHA2003759 | 0.000 | 100.000 |
| BRTHA2005448 | 0.000 | 100.000 |
| BRTHA2006720 | 0.000 | 100.000 |
| BRTHA2008502 | 0.000 | 23.993 |
| BRTHA2008598 | 0.000 | 100.000 |
| BRTHA2010672 | 0.000 | 100.000 |
| BRTHA2012189 | 0.000 | 100.000 |
| BRTHA2014647 | 0.000 | 100.000 |
| BRTHA2018304 | 0.000 | 100.000 |
| BRTHA2019726 | 0.000 | 100.000 |
| BRTHA2019743 | 0.000 | 100.000 |
| BRTHA2020400. | 0.000 | 100.000 |
| BRTHA2020566 | 0.000 | 100.000 |
| BRTHA2020642 | 0.000 | 100.000 |
| BRTHA2020695 | 0.000 | 100.000 |
| BRTHA2020721 | 0.000 | 100.000 |
| BRTHA2020781 | 0.000 | 100.000 |
| BRTHA2020910 | 0.000 | 100.000 |
| BRTHA2021212 | 0.000 | 100.000 |
| BRTHA2021440 | 0.000 | 57.080 |
| BRTHA2021450 | 0.000 | 100.000 |
| BRTHA2022074 | 0.000 | 100.000 |
| BRTHA2022914 | 0.000 | 100.000 |
| BRTHA2022968 | 0.000 | 100.000 |
| BRTHA2023402 | 0.000 | 100.000 |
| BRTHA2023437 | 0.000 | 100.000 |
| BRTHA2024177 | 0.000 | 100.000 |
| BRTHA2024354 | 0.000 | 100.000 |
| BRTHA2024712 | 0.000 | 100.000 |
| BRTHA2025869 | 0.000 | 100.000 |
| BRTHA2026071 | 0.000 | 13.612 |
| BRTHA2026290 | 0.000 | 100.000 |
| BRTHA2026311 | 0.000 | 100.000 |
| BRTHA2027227 | 0.000 | 100.000 |
| BRTHA2027229 | 0.000 | 2.030 |
| BRTHA2027250 | 0.000 | 100.000 |
| BRTHA2028297 | 0.000 | 100.000 |
| BRTHA2029969 | 0.000 | 100.000 |
| BRTHA2030036 | 0.000 | 100.000 |
| BRTHA2030213 | 0.000 | 100.000 |
| BRTHA2031517 | 0.000 | 52.083 |
| BRTHA2031917 | 0.000 | 100.000 |
| BRTHA2032763 | 0.000 | 100.000 |
| BRTHA2033122 | 0.000 | 100.000 |
| BRTHA2033155 | 4.253 | 9.433 |
| BRTHA2033320 | 0.000 | 100.000 |
| BRTHA2033469 | 0.000 | 38.930 |
| BRTHA2033683 | 0.000 | 100.000 |
| BRTHA2034281 | 0.000 | 100.000 |
| BRTHA2034576 | 0.000 | 100.000 |
| BRTHA2035743 | 0.000 | 22.576 |
| BRTHA2036055 | 0.000 | 100.000 |
| BRTHA2036295 | 0.000 | 100.000 |
| BRTHA2037247 | 0.000 | 100.000 |
| BRTHA2038279 | 0.000 | 100.000 |
| BRTHA2038345 | 0.000 | 100.000 |
| BRTHA2038353 | 0.000 | 100.000 |
| BRTHA3000456 | 0.000 | 62.915 |
| BRTHA3002411 | 0.000 | 100.000 |
| BRTHA3003225 | 0.000 | 47.165 |
| BRTHA3003417 | 0.000 | 34.129 |
| BRTHA3003736 | 4.302 | 2.385 |
| BRTHA3005988 | 31.076 | 68.924 |
| BRTHA3006593 | 0.000 | 100.000 |
| BRTHA3007469 | 0.000 | 100.000 |
| BRTHA3007662 | 0.000 | 35.211 |
| BRTHA3009858 | 0.000 | 25.720 |
| BRTHA3010135 | 20.010 | 44.380 |
| BRTHA3010212 | 5.525 | 6.127 |
| BRTHA3010469 | 0.000 | 100.000 |
| BRTHA3010530 | 0.000 | 31.009 |
| BRTHA3010540 | 0.000 | 7.466 |
| BRTHA3010717 | 0.000 | 100.000 |
| BRTHA3011187 | 0.000 | 100.000 |
| BRTHA3011194 | 3.534 | 3.920 |
| BRTHA3011229 | 0.000 | 100.000 |
| BRTHA3011265 | 25.596 | 18.923 |
| BRTHA3011306 | 0.000 | 13.657 |
| BRTHA3011361 | 0.000 | 62.915 |
| BRTHA3011510 | 0.000 | 100.000 |
| BRTHA3011892 | 0.000 | 100.000 |
| BRTHA3011998 | 0.000 | 43.859 |
| BRTHA3012265 | 0.000 | 35.211 |
| BRTHA3013860 | 0.000 | 100.000 |
| BRTHA3013882 | 0.000 | 100.000 |
| BRTHA3014000 | 0.000 | 100.000 |
| BRTHA3014105 | 0.000 | 100.000 |
| BRTHA3014507 | 0.000 | 100.000 |
| BRTHA3014547 | 0.000 | 100.000 |
| BRTHA3014835 | 0.000 | 100.000 |
| BRTHA3014854 | 0.000 | 100.000 |
| BRTHA3014920 | 0.000 | 100.000 |
| BRTHA3016616 | 0.000 | 100.000 |
| BRTHA3017791 | 11.821 | 5.244 |
| BRTHA3018409 | 0.000 | 100.000 |
| BRTHA3018623 | 0.000 | 100.000 |
| BRTHA3019183 | 0.000 | 100.000 |
| BRTHA3020369 | 0.000 | 100.000 |
| BRTHA3020771 | 31.957 | 35.439 |
| BRTHA3021569 | 0.000 | 100.000 |
| BRTHA3021708 | 0.000 | 60.975 |
| BRTHA3021786 | 0.000 | 100.000 |
| BRTHA3021971 | 1.941 | 4.305 |
| BRTHA3022641 | 0.000 | 19.618 |
| BRTHA3023403 | 14.857 | 8.238 |
| BRTHA3023590 | 0.000 | 100.000 |
| BRTHA3023929 | 0.000 | 100.000 |
| BRTHA3024600 | 0.000 | 100.000 |
| BRTHA3025073 | 0.000 | 100.000 |
| BRTHA3026161 | 0.000 | 100.000 |
| BRTHA3026180 | 0.000 | 100.000 |
| BRTHA3026556 | 0.000 | 100.000 |
| BRTHA3026916 | 0.000 | 52.395 |
| BRTHA3027171 | 0.000 | 100.000 |
| BRTHA3027318 | 0.000 | 100.000 |
| BRTHA3027638 | 0.000 | 100.000 |
| BRTHA3027820 | 0.000 | 100.000 |
| BRTHA3027879 | 0.000 | 48.181 |
| BRTHA3027957 | 0.000 | 100.000 |
| BRTHA3028339 | 0.000 | 100.000 |
| BRTHA3028505 | 0.000 | 100.000 |
| CHONS2002829 | 0.000 | 1.812 |
| CTONG2006235 | 9.828 | 5.450 |
| CTONG2009033 | 25.755 | 0.000 |
| CTONG2011801 | 0.000 | 3.284 |
| CTONG2020582 | 9.594 | 4.256 |
| D9OST2003106 | 6.904 | 0.000 |
| DFNES2001829 | 9.228 | 0.000 |
| KIDNE2010049 | 3.438 | 0.000 |
| LIVER2008465 | 0.000 | 7.339 |
| MESAN2017133 | 17.548 | 0.000 |
| NT2RI2009233 | 6.767 | 2.501 |
| NT2RI2010795 | 0.000 | 36.581 |
| NT2RI2015533 | 6.216 | 12.064 |
| NT2RI3005923 | 19.493 | 0.000 |
| NT2RI3009524 | 35.496 | 0.000 |
| NT2RP7007387 | 6.158 | 2.276 |
| NT2RP8001605 | 29.324 | 0.000 |
| NT2RP8007920 | 1.978 | 0.000 |
| NTONG2008483 | 2.397 | 1.772 |
| OCBBF2003518 | 2.062 | 0.000 |
| OCBBF2009536 | 0.000 | 47.165 |
| OCBBF2018618 | 16.218 | 0.000 |
| OCBBF3001333 | 9.639 | 0.000 |
| OCBBF3004487 | 4.531 | 0.000 |
| OCBBF3008835 | 0.000 | 47.165 |
| PLACE6003004 | 0.000 | 38.390 |
| PLACE7004103 | 3.880 | 4.302 |
| PLACE7006240 | 25.174 | 0.000 |
| PROST200744 | 4.374 | 3.638 |
| SMINT2009292 | 0.000 | 3.933 |
| TBAES2007428 | 5.371 | 0.000 |
| TESTI2005112 | 19.455 | 0.000 |
| TESTI2021654 | 9.773 | 10.838 |
| TESTI2039342 | 0.000 | 77.236 |
| TESTI4002072 | 16.696 | 11.109 |
| TESTI4002774 | 4.103 | 0. 000 |
| TESTI4002799 | 1.767 | 1.469 |
| TESTI4003602 | 25.967 | 0. 000 |
| TESTI4003703 | 0. 000 | 4.907 |
| TESTI4004210 | 36. 051 | 0. 000 |
| TESTI4004695 | 0. 000 | 36.122 |
| TESTI4005399 | 7.797 | 0. 000 |
| TESTI4005653 | 1.989 | 0.551 |
| TESTI4006441 | 60.471 | 0. 000 |
| TESTI4007965 | 0. 000 | 20. 014 |
| TESTI4010979 | 0.000 | 21.790 |
| TESTI4013474 | 0. 000 | 2. 292 |
| TESTI4014908 | 36.051 | 0.000 |
| TESTI4022158 | 53.386 | 14.801 |
| TESTI4029297 | 0. 000 | 4.907 |
| TESTI4032913 | 0. 000 | 8.989 |
| THYMU3000776 | 15.604 | 0.000 |
| THYMU3002887 | 4.680 | 3.460 |
| THYMU3003350 | 54.530 | 0.000 |
| THYMU3021586 | 20.651 | 4.580 |
| THYMU3026000 | 49.066 | 0.000 |
| THYMU3026306 | 46.281 | 0.000 |
| THYMU3026350 | 14.291 | 15.849 |
| THYMU3032798 | 9.668 | 4.289 |
| THYMU3032867 | 12.871 | 0.000 |
| THYMU3037827 | 54.530 | 0.000 |
| THYMU3038214 | 39.314 | 0.000 |
| THYMU3044075 | 37.925 | 0.000 |
| TRACH2013585 | 23.471 | 0.000 |
| TRACH3002752 | 10.215 | 0.000 |
| TRACH3003037 | 64.100 | 0.000 |
| TRACH3003872 | 2.745 | 0.000 |
| TRACH3004424 | 17.006 | 18.859 |
| TRACH3006717 | 18.347 | 0.000 |
| TRACH3007625 | 1.175 | 3.911 |
| TRACH3007689 | 47.167 | 0.000 |
| TRACH3008508 | 0.000 | 21.245 |
| TRACH3008632 | 25.351 | 9.371 |
| TRACH3009008 | 24.495 | 0.000 |
| TRACH3010079 | 48.305 | 0.000 |
| TRACH3016805 | 4.502 | 0.000 |
| TRACH3016885 | 3.978 | 0.000 |
| TRACH3024342 | 47.167 | 0.000 |
| TRACH3024671 | 47.167 | 0.000 |
| TRACH3026303 | 5.605 | 0.000 |
| TRACH3026676 | 11.631 | 12.899 |
| TRACH3028855 | 22.309 | 0.000 |
| TRACH3032570 | 47.167 | 0.000 |
| TRACH3036932 | 14.659 | 16.256 |
| TRACH3038399 | 12.435 | 13.790 |
| UTERU3010409 | 3.461 | 0.000 |
| UTERU3012414 | 0.000 | 48.181 |
| UTERU3013167 | 45.606 | 0.000 |
| UTERU3017995 | 0.000 | 12.128 |
| UTERU3018172 | 0.000 | 35.368 |
| BRACE3046450 | 21.984 | 0.000 |
| BRAWH3013197 | 100.000 | 0.000 |
| BRAWH3028645 | 100.000 | 0.000 |
| BRTHA3004432 | 32.087 | 35.583 |
| BRTHA3024233 | 0.000 | 100.000 |
| CTONG2002832 | 0.000 | 12.432 |
| THYMU3046350 | 0.000 | 15.320 |
| TRACH1000193 | 21.722 | 0.000 |

The result of comparative analysis of cDNA libraries derived from the amygdale (BRAMY), and from whole tissues of a normal brain (BRAWH) showed following genes whose expression levels differed between the two.

**Table 9**

| Clone ID | BRAWH | BRAMY |
|---|---|---|
| ASTRO2016114 | 14.497 | 0.000 |
| BRACE2002392 | 0.000 | 5.210 |
| BRACE2012528 | 3.794 | 10.512 |
| BRACE2017397 | 0.000 | 2.376 |
| BRACE2017844 | 0.000 | 15.534 |
| BRACE3004371 | 11.270 | 7.571 |
| BRACE3004767 | 58.488 | 0.000 |
| BRACE3022340 | 58.488 | 0.000 |
| BRACE3031185 | 58.488 | 0.000 |
| BRACE3031315 | 0.000 | 58.671 |
| BRACE3031743 | 5.455 | 5.497 |
| BRACE3032385 | 13.674 | 13.778 |
| BRACE3032631 | 12.612 | 0.000 |
| BRACE3039358 | 41.331 | 0.000 |
| BRACE3040863 | 17.888 | 0.000 |
| BRACE3042432 | 58.488 | 0.000 |
| BRACE3045981 | 26.434 | 0.000 |
| BRAMY2015516 | 0.000 | 100.000 |
| BRAMY2021098 | 0.000 | 100.000 |
| BRAMY2022320 | 0.000 | 100.000 |
| BRAMY2023939 | 0.000 | 6.159 |
| BRAMY2025495 | 0.000 | 100.000 |
| BRAMY2031516 | 14.772 | 7.442 |
| BRAMY2033895 | 0.000 | 100.000 |
| BRAMY2035801 | 0.000 | 100.000 |
| BRAMY2036254 | 0.000 | 100.000 |
| BRAMY2036266 | 0.000 | 100.000 |
| BRAMY2037609 | 0.000 | 100.000 |
| BRAMY2039630 | 0.000 | 8.744 |
| BRAMY2040915 | 0.000 | 100.000 |
| BRAMY2041347 | 0.000 | 100.000 |
| BRAMY2041384 | 0.000 | 100.000 |
| BRAMY2041507 | 0.000 | 100.000 |
| BRAMY2044686 | 0.000 | 100.000 |
| BRAMY2046489 | 0.000 | 100.000 |
| BRAMY2046537 | 0.000 | 100.000 |
| BRAMY3000692 | 0.000 | 100.000 |
| BRAMY3001409 | 0.000 | 100.000 |
| BRAMY3002329 | 20.323 | 20.478 |
| BRAMY3002508 | 0.000 | 100.000 |
| BRAMY3002886 | 0.000 | 100.000 |
| BRAMY3004126 | 49.811 | 50.189 |
| BRAMY3004364 | 0.000 | 13.689 |
| BRAMY3005184 | 0.000 | 22.578 |
| BRAMY3005656 | 0.000 | 100.000 |
| BRAMY3005912 | 21.416 | 21.579 |
| BRAMY3007078 | 0.000 | 49.688 |
| BRAMY3007449 | 0.000 | 100.000 |
| BRAMY3007471 | 0.000 | 100.000 |
| BRAMY3008436 | 5.603 | 4.234 |
| BRAMY3009158 | 0.000 | 100.000 |
| BRAMY3009491 | 0.000 | 31.439 |
| BRAMY3009556 | 0.000 | 47.605 |
| BRAMY3009904 | 0.000 | 100.000 |
| BRAMY3010321 | 0.000 | 100.000 |
| BRAMY3010603 | 0.000 | 100.000 |
| BRAMY3010654 | 0.000 | 47.605 |
| BRAMY4000915 | 0.000 | 49.688 |
| BRAMY4000962 | 0.000 | 100.000 |
| BRAMY4001234 | 0.000 | 54.718 |
| BRAMY4001652 | 0.000 | 100.000 |
| BRAMY4001863 | 0.000 | 15.094 |
| BRAMY4001913 | 0.000 | 100.000 |
| BRAMY4002575 | 0.000 | 100.000 |
| BRAMY4002628 | 0.000 | 7.157 |
| BRAWH2000256 | 100.000 | 0.000 |
| BRAWH2002333 | 100.000 | 0.000 |
| BRAWH2004078 | 10.539 | 0.000 |
| BRAWH2010364 | 34.464 | 0.000 |
| BRAWH2010619 | 100.000 | 0.000 |
| BRAWH2011796 | 100.000 | 0.000 |
| BRAWH2011812 | 13.544 | 13.646 |
| BRAWH2011958 | 100.000 | 0.000 |
| BRAWH2012054 | 100.000 | 0.000 |
| BRAWH2012866 | 8.503 | 14.993 |
| BRAWH2013955 | 100.000 | 0.000 |
| BRAWH2014053 | 23.222 | 0.000 |
| BRAWH2016209 | 8.306 | 0.000 |
| BRAWH2016223 | 100.000 | 0.000 |
| BRAWH2016305 | 4.308 | 0.000 |
| BRAWH2016514 | 35.037 | 0.000 |
| BRAWH2016562 | 49.499 | 0.000 |
| BRAWH2016785 | 100.000 | 0.000 |
| BRAWH3000446 | 100.000 | 0.000 |
| BRAWH3000884 | 100.000 | 0.000 |
| BRAWH3001053 | 100.000 | 0.000 |
| BRAWH3001638 | 100.000 | 0.000 |
| BRAWH3001783 | 100.000 | 0.000 |
| BRAWH3001833 | 100.000 | 0.000 |
| BRAWH3003244 | 100.000 | 0.000 |
| BRAWH3003573 | 20.162 | 0.000 |
| BRAWH3003975 | 100.000 | 0.000 |
| BRAWH3004335 | 100.000 | 0.000 |
| BRAWH3004350 | 16.580 | 0.000 |
| BRAWH3005037 | 100.000 | 0.000 |
| BRAWH3005886 | 100.000 | 0.000 |
| BRAWH3005892 | 100.000 | 0.000 |
| BRAWH3005896 | 100.000 | 0.000 |
| BRAWH3008167 | 100.000 | 0.000 |
| BRAWH3008559 | 100.000 | 0.000 |
| BRAWH3008867 | 100.000 | 0.000 |
| BRAWH3009961 | 47.417 | 0.000 |
| BRAWH3010461 | 16.435 | 14.490 |
| BRAWH3010602 | 100.000 | 0.000 |
| BRAWH3010657 | 100.000 | 0.000 |
| BRAWH3010726 | 100.000 | 0.000 |
| BRAWH3010833 | 100.000 | 0.000 |
| BRAWH3011101 | 100.000 | 0.000 |
| BRAWH3011331 | 100.000 | 0.000 |
| BRAWH3011402 | 100.000 | 0.000 |
| BRAWH3011577 | 100.000 | 0.000 |
| BRAWH3011623 | 100.000 | 0.000 |
| BRAWH3011685 | 100.000 | 0.000 |
| BRAWH3011907 | 47.417 | 0.000 |
| BRAWH3011929 | 100.000 | 0.000 |
| BRAWH3012005 | 100.000 | 0.000 |
| BRAWH3012662 | 33.972 | 0.000 |
| BRAWH3012779 | 100.000 | 0.000 |
| BRAWH3013009 | 100.000 | 0.000 |
| BRAWH3013049 | 100.000 | 0.000 |
| BRAWH3013264 | 100.000 | 0.000 |
| BRAWH3013508 | 100.000 | 0.000 |
| BRAWH3014609 | 44.597 | 0.000 |
| BRAWH3014639 | 100.000 | 0.000 |
| BRAWH3015017 | 100.000 | 0.000 |
| BRAWH3015175 | 100.000 | 0.000 |
| BRAWH3015610 | 100.000 | 0.000 |
| BRAWH3015825 | 100.000 | 0.000 |
| BRAWH3016123 | 100.000 | 0.000 |
| BRAWH3016715 | 100.000 | 0.000 |
| BRAWH3017180 | 28.251 | 0.000 |
| BRAWH3017259 | 13.996 | 0.000 |
| BRAWH3017260 | 100.000 | 0.000 |
| BRAWH3017477 | 100.000 | 0.000 |
| BRAWH3017980 | 49.811 | 50.189 |
| BRAWH3018063 | 15.836 | 7.978 |
| BRAWH3018369 | 100.000 | 0.000 |
| BRAWH3018548 | 100.000 | 0.000 |
| BRAWH3018969 | 100.000 | 0.000 |
| BRAWH3019026 | 100.000 | 0.000 |
| BRAWH3019529 | 100.000 | 0.000 |
| BRAWH3019594 | 100.000 | 0.000 |
| BRAWH3019820 | 100.000 | 0.000 |
| BRAWH3020200 | 100.000 | 0.000 |
| BRAWH3020318 | 100.000 | 0.000 |
| BRAWH3020884 | 100.000 | 0.000 |
| BRAWH3020928 | 49.499 | 0.000 |
| BRAWH3021012 | 100.000 | 0.000 |
| BRAWH3021574 | 9.400 | 0.000 |
| BRAWH3021580 | 100.000 | 0.000 |
| BRAWH3021641 | 100.000 | 0.000 |
| BRAWH3021643 | 100.000 | 0.000 |
| BRAWH3021724 | 100.000 | 0.000 |
| BRAWH3022347 | 100.000 | 0.000 |
| BRAWH3022431 | 100.000 | 0.000 |
| BRAWH3022459 | 100.000 | 0.000 |
| BRAWH3022542 | 100.000 | 0.000 |
| BRAWH3022651 | 9.828 | 4.952 |
| BRAWH3022719 | 100.000 | 0.000 |
| BRAWH3022900 | 100.000 | 0.000 |
| BRAWH3023156 | 100.000 | 0.000 |
| BRAWH3023168 | 100.000 | 0.000 |
| BRAWH3023172 | 0.916 | 0.000 |
| BRAWH3023274 | 100.000 | 0.000 |
| BRAWH3023421 | 100.000 | 0.000 |
| BRAWH3024186 | 19.959 | 10.055 |
| BRAWH3024231 | 100.000 | 0.000 |
| BRAWH3024242 | 31.843 | 0.000 |
| BRAWH3024506 | 100.000 | 0.000 |
| BRAWH3024989 | 2.369 | 0.000 |
| BRAWH3026349 | 100.000 | 0.000 |
| BRAWH3026938 | 6.894 | 0.000 |
| BRAWH3027420 | 100.000 | 0.000 |
| BRAWH3027440 | 58.488 | 0.000 |
| BRAWH3027533 | 11.008 | 0.000 |
| BRAWH3027574 | 47.417 | 0.000 |
| BRAWH3027607 | 100.000 | 0.000 |
| BRAWH3027616 | 100.000 | 0.000 |
| BRAWH3027675 | 100.000 | 0.000 |
| BRAWH3027806 | 100.000 | 0.000 |
| BRAWH3027880 | 100.000 | 0.000 |
| BRAWH3028202 | 100.000 | 0.000 |
| BRAWH3028223 | 100.000 | 0.000 |
| BRAWH3028461 | 100.000 | 0.000 |
| BRAWH3028754 | 100.000 | 0.000 |
| BRAWH3028796 | 100.000 | 0.000 |
| BRAWH3029313 | 100.000 | 0.000 |
| BRAWH3029385 | 35.187 | 0.000 |
| BRAWH3029538 | 100.000 | 0.000 |
| BRAWH3029806 | 26.735 | 8.979 |
| BRAWH3030772 | 100.000 | 0.000 |
| BRAWH3030810 | 21.287 | 10.724 |
| BRAWH3030910 | 100.000 | 0.000 |
| BRAWH3031054 | 100.000 | 0.000 |
| BRAWH3031342 | 10.856 | 0.000 |
| BRAWH3031710 | 100.000 | 0.000 |
| BRAWH3032298 | 100.000 | 0.000 |
| BRAWH3032340 | 100.000 | 0.000 |
| BRAWH3032571 | 100.000 | 0.000 |
| BRAWH3033117 | 100.000 | 0.000 |
| BRAWH3033293 | 100.000 | 0.000 |
| BRAWH3033448 | 100.000 | 0.000 |
| BRAWH3033513 | 100.000 | 0.000 |
| BRAWH3034097 | 100.000 | 0.000 |
| BRAWH3034114 | 100.000 | 0.000 |
| BRAWH3034134 | 100.000 | 0.000 |
| BRAWH3034668 | 100.000 | 0.000 |
| BRAWH3034743 | 16.662 | 12.592 |
| BRAWH3034775 | 100.000 | 0.000 |
| BRAWH3034890 | 100.000 | 0.000 |
| BRAWH3035403 | 35.337 | 0.000 |
| BRAWH3035904 | 100.000 | 0.000 |
| BRAWH3035914 | 100.000 | 0.000 |
| BRAWH3035936 | 70.575 | 0.000 |
| BRAWH3036077 | 100.000 | 0.000 |
| BRAWH3036247 | 54.530 | 0.000 |
| BRAWH3036270 | 100.000 | 0.000 |
| BRAWH3036334 | 6.241 | 0.000 |
| BRAWH3036561 | 100.000 | 0.000 |
| BRAWH3037265 | 44.597 | 0.000 |
| BRAWH3037394 | 100.000 | 0.000 |
| BRAWH3037428 | 5.877 | 5.921 |
| BRAWH3037533 | 100.000 | 0.000 |
| BRAWH3037979 | 100.000 | 0.000 |
| BRAWH3038055 | 100.000 | 0.000 |
| BRAWH3038230 | 100.000 | 0.000 |
| BRAWH3038252 | 100.000 | 0.000 |
| BRAWH3038324 | 100.000 | 0.000 |
| BRCAN2010665 | 19.610 | 9.879 |
| BRCAN2022126 | 4.855 | 14.677 |
| BRCAN2025093 | 21.732 | 0.000 |
| BRCOC2012386 | 11.403 | 0.000 |
| BRHIP2015153 | 20.038 | 10.095 |
| BRHIP2027077 | 2.020 | 2.035 |
| BRHIP3001573 | 24.474 | 8.220 |
| BRHIP3002000 | 4.500 | 0.756 |
| BRHIP3002691 | 0.000 | 8.357 |
| BRHIP3003063 | 10.960 | 33.130 |
| BRHIP3003984 | 27.477 | 0.000 |
| BRHIP3004215 | 0.000 | 13.679 |
| BRHIP3004774 | 39.520 | 0.000 |
| BRHIP3005673 | 0.000 | 25.956 |
| BRHIP3007223 | 2.016 | 0.000 |
| BRHIP3007409 | 31.957 | 0.000 |
| BRHIP3008320 | 23.349 | 0.000 |
| BRHIP3012736 | 0.000 | 66.389 |
| BRHIP3014675 | 37.925 | 0.000 |
| BRHIP3017146 | 0.000 | 33.057 |
| BRHIP3017855 | 15.316 | 0.000 |
| BRHIP3018784 | 6.930 | 0.000 |
| BRHIP3020046 | 0.000 | 1.021 |
| BRHIP3021019 | 7.745 | 7.803 |
| BRHIP3028246 | 22.147 | 22.315 |
| BRHIP3028570 | 31.957 | 0.000 |
| BRSTN2010089 | 6.598 | 0.000 |
| BRSTN2012069 | 0.047 | 0.029 |
| BRSTN2016992 | 1.476 | 4.958 |
| BRTHA2026071 | 0.000 | 12.368 |
| BRTHA2033155 | 4.253 | 4.285 |
| BRTHA3003736 | 4.302 | 0.000 |
| BRTHA3005988 | 31.076 | 0.000 |
| BRTHA3010135 | 20.010 | 0.000 |
| BRTHA3010212 | 5.525 | 5.567 |
| BRTHA3011194 | 3.534 | 0.000 |
| BRTHA3011265 | 25.596 | 0.000 |
| BRTHA3017791 | 11.821 | 5.956 |
| BRTHA3020771 | 31.957 | 0.000 |
| BRTHA3021971 | 1.941 | 1.956 |
| BRTHA3023403 | 14.857 | 7.485 |
| BRTHA3026916 | 0.000 | 47.605 |
| CHONS2002829 | 0.000 | 1.646 |
| CTONG2006235 | 9.828 | 4.952 |
| CTONG2009033 | 25.755 | 0.000 |
| CTONG2020582 | 9.594 | 3.867 |
| D9OST2003106 | 6.904 | 0.000 |
| DFNES2001829 | 9.228 | 0.000 |
| KIDNE2010049 | 3.438 | 0.000 |
| MESAN2017133 | 17.548 | 0.000 |
| NT2RI2009233 | 6.767 | 4.545 |
| NT2RI2010795 | 0.000 | 16.619 |
| NT2RI2015533 | 6.216 | 12.527 |
| NT2RI3005923 | 19.493 | 0. 000 |
| NT2RI3009524 | 35.496 | 0. 000 |
| NT2RP7007387 | 6.158 | 0.000 |
| NT2RP8001605 | 29.324 | 0.000 |
| NT2RP8007920 | 1.978 | 0.000 |
| NTONG2008483 | 2.397 | 0.000 |
| OCBBF2000831 | 0.000 | 9.702 |
| OCBBF2003518 | 2.062 | 1.039 |
| OCBBF2018618 | 16.218 | 16.341 |
| OCBBF2030927 | 0.000 | 44.784 |
| OCBBF3001333 | 9.639 | 6.475 |
| OCBBF3004487 | 4.531 | 4.565 |
| OCBBF3009244 | 0.000 | 34.702 |
| PLACE6008315 | 0.000 | 1.403 |
| PLACE6010936 | 0.000 | 12.399 |
| PLACE7004103 | 3.880 | 1.303 |
| PLACE7006240 | 25.174 | 0.000 |
| PROST2007444 | 4.374 | 1.102 |
| SPLEN2012571 | 0.000 | 3.203 |
| SYNOV4004210 | 0.000 | 0.952 |
| SYNOV4009575 | 0.000 | 4.828 |
| TBAES2007428 | 5.371 | 0.000 |
| TESTI2005112 | 19.455 | 0.000 |
| TESTI2021654 | 9.773 | 9.847 |
| TESTI4002072 | 16. 696 | 3.364 |
| TESTI4002774 | 4.103 | 0. 000 |
| TESTI4002799 | 1.767 | 0. 000 |
| TESTI4003602 | 25.967 | 0.000 |
| TESTI4003703 | 0. 000 | 4.458 |
| TESTI4004210 | 36.051 | 0.000 |
| TESTI4005399 | 7.797 | 15. 713 |
| TESTI4005653 | 1.989 | 3.507 |
| TESTI4006441 | 60.471 | 0.000 |
| TESTI4014908 | 36. 051 | 0. 000 |
| TESTI4022158 | 53.386 | 0. 000 |
| TESTI4029297 | 0. 000 | 4.458 |
| TESTI4032913 | 0. 000 | 8.167 |
| TESTI4043223 | 0. 000 | 27.886 |
| TESTI4046073 | 0. 000 | 19.130 |
| THYMU3000776 | 15.604 | 0.000 |
| THYMU3002887 | 4.680 | 3.930 |
| THYMU3003350 | 54.530 | 0.000 |
| THYMU3008105 | 0.000 | 20.406 |
| THYMU3019476 | 0.000 | 54.718 |
| THYMU3021586 | 20.651 | 4.162 |
| THYMU3026000 | 49.066 | 0.000 |
| THYMU3026306 | 46.281 | 0.000 |
| THYMU3026350 | 14.291 | 14.400 |
| THYMU3032798 | 9.668 | 1.948 |
| THYMU3032867 | 12.871 | 0.000 |
| THYMU3037827 | 54.530 | 0.000 |
| THYMU3038214 | 39.314 | 0.000 |
| THYMU3044075 | 37.925 | 0.000 |
| TRACH2013585 | 23.471 | 0.000 |
| TRACH3002752 | 10.215 | 0.000 |
| TRACH3003037 | 64.100 | 0.000 |
| TRACH3003872 | 2.745 | 2.765 |
| TRACH3004424 | 17.006 | 0.000 |
| TRACH3006717 | 18.347 | 3.697 |
| TRACH3007625 | 1.175 | 3.553 |
| TRACH3007689 | 47.167 | 0.000 |
| TRACH3008632 | 25.351 | 0.000 |
| TRACH3009008 | 24.495 | 0.000 |
| TRACH3010079 | 48.305 | 0.000 |
| TRACH3016805 | 4.502 | 0.000 |
| TRACH3016885 | 3.978 | 12.025 |
| TRACH3024342 | 47.167 | 0.000 |
| TRACH3024671 | 47.167 | 0.000 |
| TRACH3026303 | 5.605 | 0.000 |
| TRACH3026676 | 11.631 | 0.000 |
| TRACH3028855 | 22.309 | 0.000 |
| TRACH3032570 | 47.167 | 0.000 |
| TRACH3036932 | 14.659 | 0.000 |
| TRACH3038399 | 12.435 | 0.000 |
| UTERU3010409 | 3.461 | 0.000 |
| UTERU3013167 | 45.606 | 0.000 |
| BRACE3046450 | 21.984 | 0.000 |
| BRAMY3008096 | 0.000 | 58.671 |
| BRAWH3013197 | 100.000 | 0.000 |
| BRAWH3028645 | 100.000 | 0.000 |
| BRTHA3004432 | 32.087 | 32.330 |
| TESTI4002988 | 0. 000 | 21.851 |
| THYMU3046350 | 0.000 | 6.960 |
| TRACH1000193 | 21.722 | 0.000 |
| TRACH3019290 | 0.000 | 27.871 |

The result of comparative analysis of cDNA libraries derived from breast tumor (TBAES), and normal breast (BEAST) showed the following genes whose expression levels differed between the two.

**Table 10**

| Clone ID | BEAST | TBAES |
|---|---|---|
| BRSTN2011961 | 6.317 | 4.075 |
| BRSTN2012069 | 0.000 | 0.067 |
| TBAES2003917 | 0.000 | 100.000 |
| TBAES2005361 | 0.000 | 100.000 |
| TBAES2007428 | 0.000 | 37.783 |
| TBAES2007548 | 0.000 | 100.000 |
| TBAES2007862 | 0.000 | 100.000 |
| TESTI2005564 | 0. 000 | 25.762 |
| TESTI4017854 | 0.000 | 91.498 |
| TRACH3016805 | 0.000 | 31.673 |
| TBAES2004105 | 0.000 | 100.000 |
| TBAES2007379 | 0.000 | 100.000 |
| TBAES2007481 | 0.000 | 100.000 |
| TBAES2008133 | 0.000 | 100.000 |

The result of comparative analysis of cDNA libraries derived cervical tumor (TCERX), and normal cervical duct (CERVX) showed the following genes whose expression levels differed between the two.

**Table 11**

| Clone ID | CERVX | TCERX |
|---|---|---|
| BRACE2017397 | 48.741 | 0.000 |
| BRHIP2027077 | 41.741 | 0.000 |
| BRSTN2011961 | 5.985 | 0.000 |
| BRSTN2012069 | 5.668 | 7.585 |
| CERVX2000812 | 100.000 | 0.000 |
| CERVX2000968 | 100.000 | 0.000 |

The result of comparative analysis of cDNA libraries derived from colon tumor (TCOLN), and normal colon (COLON) showed the following genes whose expression levels differed between the two.

**Table 12**

| Clone ID | COLON | TCOLN |
|---|---|---|
| BRSTN2011961 | 0.000 | 6.140 |
| BRSTN2012069 | 1.726 | 1.805 |
| COLON2001829 | 100.000 | 0.000 |
| COLON2001866 | 100.000 | 0.000 |
| COLON2004351 | 100.000 | 0.000 |
| COLON2004911 | 100.000 | 0.000 |
| COLON2005623 | 100.000 | 0.000 |
| COLON2005735 | 100.000 | 0.000 |
| OCBBF3001333 | 22.556 | 0.000 |
| SMINT2017964 | 34.030 | 0.000 |

The result of comparative analysis of cDNA libraries derived from esophageal tumor (TESOP), and normal esophagus (NESOP) showed the following genes whose expression levels differed between the two.

**Table 13**

| Clone ID | NESOP | TESOP |
|---|---|---|
| BRAMY3004364 | 0.000 | 46.478 |
| BRAWH3027533 | 0.000 | 75.317 |
| BRHIP3007223 | 42.899 | 0.000 |
| BRSTN2011961 | 0.000 | 1.981 |
| BRSTN2012069 | 1.409 | 2.071 |
| CTONG2011801 | 0.000 | 20.259 |
| CTONG3002518 | 0.000 | 57.732 |
| SMINT2009292 | 0.000 | 24.268 |
| TESOP2002005 | 0.000 | 100.000 |
| TESOP2003308 | 0.000 | 100.000 |
| TESOP2004110 | 0.000 | 100.000 |
| TESOP2008556 | 0.000 | 100.000 |
| UTERU3015011 | 0.000 | 48.286 |
| UTERU3017995 | 0.000 | 74.828 |

The result of comparative analysis of cDNA libraries derived from kidney tumor (TKIDN), and normal kidney (KIDNE) showed the following genes whose expression levels differed between the two.

**Table 14**

| Clone ID | KIDNE | TKIDN |
|---|---|---|
| BRACE2002392 | 0.000 | 6.380 |
| BRACE2012528 | 0.000 | 14.044 |
| BRACE3004371 | 13.097 | 0.000 |
| BRAMY2039630 | 60.511 | 0.000 |
| BRAMY3004364 | 23.683 | 0.000 |
| BRAMY3008436 | 0.000 | 5.185 |
| BRAWH2004078 | 18.371 | 0.000 |
| BRAWH3012662 | 0.000 | 41.917 |
| BRAWH3021574 | 0.000 | 17.397 |
| BRAWH3022651 | 0.000 | 6.064 |
| BRAWH3037428 | 0.000 | 21.753 |
| BRCAN2019953 | 0.000 | 35.208 |
| BRCAN2022126 | 0.000 | 17.973 |
| BRHIP3002000 | 10.458 | 0.000 |
| BRHIP3002691 | 0.000 | 30.700 |
| BRHIP3012997 | 0.000 | 3. 431 |
| BRHIP3020046 | 0.000 | 3.749 |
| BRSTN2012069 | 0.429 | 0.140 |
| BRSTN2016992 | 0.000 | 1.822 |
| BRTHA3010212 | 0.000 | 20.450 |
| CTONG2006235 | 0.000 | 6.064 |
| KIDNE2004531 | 80.699 | 0.000 |
| KIDNE2010049 | 11.987 | 0.000 |
| KIDNE2014496 | 100.000 | 0.000 |
| KIDNE2015987 | 100.000 | 0.000 |
| KIDNE2016464 | 100.000 | 0.000 |
| KIDNE2017153 | 83. 085 | 0.000 |
| KIDNE2018268 | 100.000 | 0.000 |
| NT2RI2015533 | 0.000 | 11.506 |
| NT2RP7007387 | 7.156 | 0.000 |
| OCBBF3004487 | 0.000 | 16.771 |
| PLACE6008315 | 0.000 | 5.154 |
| SYNOV4004210 | 0.000 | 3.496 |
| TESTI2005112 | 67.827 | 0.000 |
| THYMU3001776 | 22.094 | 0.000 |
| THYMU3002887 | 0.000 | 2.887 |
| THYMU3029795 | 37.093 | 0.000 |
| THYMU3032867 | 44.871 | 0.000 |
| TKIDN2000319 | 0.000 | 78.393 |
| TKIDN2003396 | 0.000 | 100.000 |
| TKIDN2010602 | 0.000 | 100.000 |
| TKIDN2011051 | 0.000 | 100.000 |
| TKIDN2011160 | 0.000 | 100.000 |

The result of comparative analysis of cDNA libraries derived from liver tumor (TLIVE), and normal liver (LIVER) showed the following genes whose expression levels differed between the two.

**Table 15**

| Clone ID | LIVER | TLIVE |
|---|---|---|
| BRAWH3022651 | 14.200 | 0.000 |
| BRCAN2020412 | 0.000 | 74.979 |
| BRSTN2012069 | 0.000 | 0.390 |
| BRTHA3003736 | 18.647 | 0.000 |
| CTONG2006235 | 14.200 | 0.000 |
| LIVER2008465 | 57.368 | 0.000 |
| TESTI4013474 | 0. 000 | 7.104 |
| THYMU3002887 | 6.762 | 0.000 |
| THYMU3038158 | 53.382 | 0.000 |
| TLIVE2000142 | 0.000 | 100.000 |
| TLIVE2001616 | 0.000 | 100.000 |
| TLIVE2007736 | 0.000 | 90.642 |
| TLIVE2008797 | 0.000 | 100.000 |
| TRACH3027229 | 93.931 | 0.000 |
| THYMU3046350 | 0.000 | 47.489 |
| TLIVE2007192 | 0.000 | 100. 000 |

The result of comparative analysis of cDNA libraries derived from lung tumor (TLUNG), and normal lung (HLUNG) showed the following genes whose expression levels differed between the two.

**Table 16**

| Clone ID | HLUNG | TLUNG |
|---|---|---|
| BRACE3036283 | 27.376 | 0.000 |
| BRAMY2031516 | 27.091 | 0.000 |
| BRSTN2011961 | 2.124 | 0.000 |
| BRSTN2012069 | 0.763 | 0.000 |
| HLUNG2012600 | 100.000 | 0.000 |
| MESAN2009156 | 38.142 | 0.000 |
| NTONG2008483 | 2.930 | 0.000 |
| PROST2007444 | 4.011 | 0.000 |
| TESTI4003703 | 16.229 | 0. 000 |
| TESTI4005653 | 1.824 | 0. 000 |
| TESTI4013474 | 3.790 | 0. 000 |
| TESTI4029297 | 16.229 | 0. 000 |
| THYMU3001776 | 11.622 | 0.000 |
| THYMU3033626 | 20.615 | 0.000 |
| THYMU3034671 | 8.747 | 0.000 |
| THYMU3041428 | 0.935 | 0.000 |
| TRACH3022198 | 30.484 | 0.000 |

The result of comparative analysis of cDNA libraries derived from ovary tumor (TOVER), and normal ovary (NOVER) showed the following genes whose expression levels differed between the two.

**Table 17**

| Clone ID | NOVAR | TOVAR |
|---|---|---|
| BRSTN2012069 | 5.861 | 6.386 |
| NOVAR2000783 | 90.145 | 0.000 |
| THYMU3002887 | 0.000 | 16.991 |

The result of comparative analysis of cDNA libraries derived from stomach tumor (TSTOM) and normal stomach (STOMA) showed the following genes whose expression levels differed between the two.

**Table 18**

| Clone ID | STOMA | TSTOM |
|---|---|---|
| BRSTN2012069 | 1.995 | 4.062 |
| CHONS2002829 | 11.118 | 0.000 |
| STOMA2003894 | 100.000 | 0.000 |
| STOMA2004663 | 47.516 | 0.000 |
| THYMU3001776 | 21.564 | 0.000 |
| TSTOM2000235 | 0.000 | 95.465 |
| TSTOM2001571 | 0.000 | 100.000 |
| TSTOM2002611 | 0. 000 | 100.000 |
| TSTOM2002682 | 0.000 | 100.000 |

The result of comparative analysis of cDNA libraries derived from uterine tumor (TUTER) and normal uterus (UTERU) showed the following genes whose expression levels differed between the two.

**Table 19**

| Clone ID | UTERU | TUTER |
|---|---|---|
| BRACE2012528 | 0.566 | 0.000 |
| BRACE2017397 | 11.252 | 0.000 |
| BRACE3004371 | 4.481 | 0.000 |
| BRACE3036283 | 8.902 | 0.000 |
| BRACE3040863 | 21.335 | 0.000 |
| BRAMY2031516 | 8.809 | 0.000 |
| BRAMY3005184 | 26.725 | 0.000 |
| BRAWH2004078 | 6.285 | 0.000 |
| BRAWH3004350 | 19.774 | 0.000 |
| BRAWH3022651 | 1.954 | 0.000 |
| BRAWH3024186 | 11.902 | 0.000 |
| BRAWH3029806 | 10.629 | 0.000 |
| BRAWH3031342 | 6.474 | 0.000 |
| BRCAN2022126 | 5.791 | 0.000 |
| BRHIP3001076 | 53.896 | 0.000 |
| BRHIP3002000 | 0.894 | 0.000 |
| BRHIP3002141 | 18.713 | 0.000 |
| BRHIP3005307 | 53.896 | 0.000 |
| BRHIP3007223 | 2.405 | 0.000 |
| BRHIP3017855 | 27.401 | 0.000 |
| BRHIP3020046 | 1.208 | 0.000 |
| BRSTN2010089 | 7.869 | 0.000 |
| BRSTN2011961 | 0.345 | 0.000 |
| BRSTN2012069 | 0.271 | 3.583 |
| BRSTN2016892 | 3.559 | 0.000 |
| BRTHA3003736 | 5.131 | 0.000 |
| BRTHA3011265 | 10.176 | 0.000 |
| BRTHA3023403 | 26.579 | 0.000 |
| BRTHA3027879 | 51.819 | 0.000 |
| CHONS2002829 | 11.691 | 0.000 |
| CTONG2001932 | 12.322 | 0.000 |
| CTONG2003517 | 13.371 | 0.000 |
| CTONG2006235 | 1.954 | 0.000 |
| CTONG2011801 | 3.532 | 0.000 |
| CTONG3002518 | 10.064 | 0.000 |
| DFNES2001829 | 11.006 | 0.000 |
| KIDNE2010049 | 4.101 | 76.623 |
| LIVER2008465 | 7.893 | 0.000 |
| NT2RI3005923 | 23.249 | 0.000 |
| OCBBF3001333 | 3.832 | 0.000 |
| OCBBF3004487 | 5.404 | 0.000 |
| PLACE6008315 | 1.661 | 0.000 |
| PLACE7006240 | 30.024 | 0.000 |
| PROST2007444 | 5.217 | 0.000 |
| SPLEN2012571 | 3.792 | 0.000 |
| SYNOV4000598 | 35.608 | 0.000 |
| SYNOV4009575 | 5.715 | 0.000 |
| TIESE2000904 | 2.754 | 0.000 |
| TESTI4002072 | 3.983 | 0.000 |
| TESTI4002195 | 15.959 | 0.000 |
| TESTI4002774 | 9.787 | 0.000 |
| TESTI4002799 | 1. 053 | 9. 842 |
| TESTI4003703 | 5.277 | 0.000 |
| TESTI4003944 | 41.606 | 0.000 |
| TESTI4005399 | 9.300 | 0.000 |
| TESTI4005653 | 1.779 | 0.000 |
| TESTI4024245 | 40.205 | 0.000 |
| TESTI4029297 | 5.277 | 0.000 |
| THYMU3002887 | 0.930 | 0.000 |
| THYMU3021586 | 4.926 | 0.000 |
| THYMU3026350 | 17.045 | 0.000 |
| THYMU3032798 | 2.306 | 0.000 |
| THYMU3034616 | 58.853 | 0.000 |
| THYMU3034671 | 19.911 | 0.000 |
| TRACH3003872 | 9.820 | 0.000 |
| TRACH3005699 | 22.893 | 0.000 |
| TRACH3006800 | 34.742 | 0.000 |
| TRACH3008632 | 20.157 | 0.000 |
| TRACH3009008 | 5.843 | 0.000 |
| TUTER1000014 | 0.000 | 100.000 |
| TUTER2001433 | 0.000 | 100.000 |
| UTERU2000300 | 11.765 | 0.000 |
| UTERU2014998 | 100.000 | 0.000 |
| UTERU2016464 | 100.000 | 0.000 |
| UTERU2016669 | 9.848 | 0.000 |
| UTERU2020226 | 78.491 | 0.000 |
| UTERU2022955 | 100.000 | 0.000 |
| UTERU2023941 | 100.000 | 0.000 |
| UTERU2024042 | 100.000 | 0.000 |
| UTERU2027369 | 39.231 | 0.000 |
| UTERU2028377 | 100.000 | 0.000 |
| UTERU2029660 | 100.000 | 0.000 |
| UTERU2035926 | 100.000 | 0.000 |
| UTERU2037423 | 100.000 | 0.000 |
| UTERU3000670 | 83.447 | 0.000 |
| UTERU3001029 | 100.000 | 0.000 |
| UTERU3001394 | 100.000 | 0.000 |
| UTERU3001946 | 34.742 | 0.000 |
| UTERU3004635 | 100.000 | 0.000 |
| UTERU3005264 | 100.000 | 0.000 |
| UTERU3005422 | 25.298 | 0.000 |
| UTERU3006538 | 100.000 | 0.000 |
| UTERU3006720 | 100.000 | 0.000 |
| UTERU3007108 | 100.000 | 0.000 |
| UTERU3009775 | 100.000 | 0.000 |
| UTERU3010029 | 100.000 | 0.000 |
| UTERU3010409 | 53.665 | 0.000 |
| UTERU3010604 | 40.126 | 0.000 |
| UTERU3010892 | 100.000 | 0.000 |
| UTERU3010919 | 100.000 | 0.000 |
| UTERU3011092 | 100.000 | 0.000 |
| UTERU3011398 | 100.000 | 0.000 |
| UTERU3011558 | 100.000 | 0.000 |
| UTERU3011579 | 34.742 | 0.000 |
| UTERU3011837 | 100.000 | 0.000 |
| UTERU3012293 | 100.000 | 0.000 |
| UTERU3012414 | 51.819 | 0.000 |
| UTERU3012476 | 100.000 | 0.000 |
| UTERU3012599 | 100.000 | 0.000 |
| UTERU3012999 | 100.000 | 0.000 |
| UTERU3013167 | 54.394 | 0.000 |
| UTERU3013302 | 58.853 | 0.000 |
| UTERU3014274 | 100.000 | 0.000 |
| UTERU3014647 | 100.000 | 0.000 |
| UTERU3014906 | 100.000 | 0.000 |
| UTERU3015011 | 16.834 | 0.000 |
| UTERU3015299 | 100.000 | 0.000 |
| UTERU3015647 | 100.000 | 0.000 |
| UTERU3015844 | 100.000 | 0.000 |
| UTERU3016070 | 100.000 | 0.000 |
| UTERU3016273 | 53.896 | 0.000 |
| UTERU3016274 | 100.000 | 0.000 |
| UTERU3016308 | 100.000 | 0.000 |
| UTERU3017441 | 100.000 | 0.000 |
| UTERU3017626 | 100.000 | 0.000 |
| UTERU3017995 | 13.044 | 0.000 |
| UTERU3018172 | 38.038 | 0.000 |
| UTERU3018255 | 100.000 | 0.000 |
| UTERU2017492 | 5.197 | 0.000 |
| UTERU2025415 | 100.000 | 0.000 |
| UTERU2036507 | 100.000 | 0.000 |

The result of comparative analysis of cDNA libraries derived from tongue cancer (CTONG) and normal tongue (NTONG) showed the following genes whose expression levels differed between the two.

**Table 20**

| Clone ID | NTONG | CTONG |
|---|---|---|
| BRACE2012528 | 0.000 | 0.883 |
| BRAMY4001863 | 0.000 | 55.803 |
| BRAWH3021574 | 0.000 | 17.507 |
| BRAWH3022651 | 0.000 | 3.051 |
| BRAWH3024186 | 0.000 | 18.588 |
| BRHIP2027077 | 0.000 | 3.762 |
| BRHIP3001573 | 0.000 | 15.195 |
| BRHIP3002000 | 0.000 | 12.571 |
| BRHIP3007223 | 14.859 | 3.755 |
| BRHIP3012997 | 0.000 | 1.726 |
| BRHIP3020046 | 0.000 | 3.773 |
| BRSSN2013696 | 0.000 | 6.652 |
| BRSTN2011961 | 2.134 | 0.539 |
| BRSTN2012069 | 0.000 | 0.018 |
| BRTHA2027229 | 0.000 | 3.410 |
| BRTHA2033155 | 0.000 | 7.921 |
| BRTHA3011194 | 0.000 | 6.583 |
| BRTHA3022641 | 0.000 | 32.949 |
| CTONG2001932 | 0.000 | 38.485 |
| CTONG2003517 | 0.000 | 20.880 |
| CTONG2006235 | 0.000 | 3.051 |
| CTONG2008989 | 0.000 | 100.000 |
| CTONG2009033 | 0.000 | 47.970 |
| CTONG2009570 | 0.000 | 100.000 |
| CTONG2010330 | 0.000 | 100.000 |
| CTONG2011801 | 0.000 | 5.515 |
| CTONG2012123 | 0.000 | 100.000 |
| CTONG2014206 | 0.000 | 100.000 |
| CTONG2014959 | 0.000 | 100.000 |
| CTONG2020582 | 0.000 | 21.442 |
| CTONG2026987 | 0.000 | 100.000 |
| CTONG2027150 | 0.000 | 100.000 |
| CTONG2027591 | 0.000 | 100.000 |
| CTONG2027783 | 0.000 | 100.000 |
| CTONG2027959 | 0.000 | 72.408 |
| CTONG3001605 | 0.000 | 100.000 |
| CTONG3002518 | 0.000 | 15.716 |
| CTONG3002588 | 0.000 | 100.000 |
| CTONG3003669 | 0.000 | 100.000 |
| CTONG3008223 | 0.000 | 100.000 |
| NT2RI2009233 | 0. 000 | 8.402 |
| NTONG2002278 | 100.000 | 0.000 |
| NTONG2003805 | 100.000 | 0.000 |
| NTONG2004829 | 100.000 | 0.000 |
| NTONG2008483 | 17.664 | 2.976 |
| NTONG2009468 | 69.996 | 0.000 |
| OCBBF3004487 | 0.000 | 8.439 |
| PLACE6008315 | 10.262 | 0.000 |
| PLACE7004103 | 9.531 | 12.044 |
| SKNMC2003639 | 0.000 | 19.285 |
| SPLEN2012571 | 0.000 | 5.921 |
| SPLEN2019092 | 0.000 | 51.706 |
| SYNOV4009575 | 0.000 | 8.924 |
| T1ESE2000904 | 0.000 | 4.300 |
| TESTI2005564 | 0.000 | 6.821 |
| TESTI2018867 | 20.987 | 0.000 |
| TESTI4002799 | 0. 000 | 1. 645 |
| TESTI4005653 | 0. 000 | 7.408 |
| TESTI4032913 | 59.736 | 0.000 |
| THYMU3021586 | 0.000 | 7.693 |
| THYMU3047115 | 57.541 | 0.000 |
| TRACH3006717 | 0.000 | 13.669 |
| TRACH3007625 | 0.000 | 4.379 |
| TRACH3016805 | 0.000 | 8.386 |
| TRACH3036932 | 0.000 | 27.304 |
| TRACH3038399 | 0.000 | 23.161 |
| UTERU2000300 | 0.000 | 9.187 |
| CTONG2002832 | 0.000 | 20.880 |
| CTONG2003764 | 0.000 | 24.325 |

The result of comparative analysis of cDNA libraries derived from fetal brain (FCBBF, FEBRA, or OCBBF) and adult brain (BRACE, BRALZ, BRAMY, BRAWH, BRCAN, BRCOC, BRHIP, BRSSN, BRSTN, or BRTHA) showed the following genes whose expression levels differed between the two.

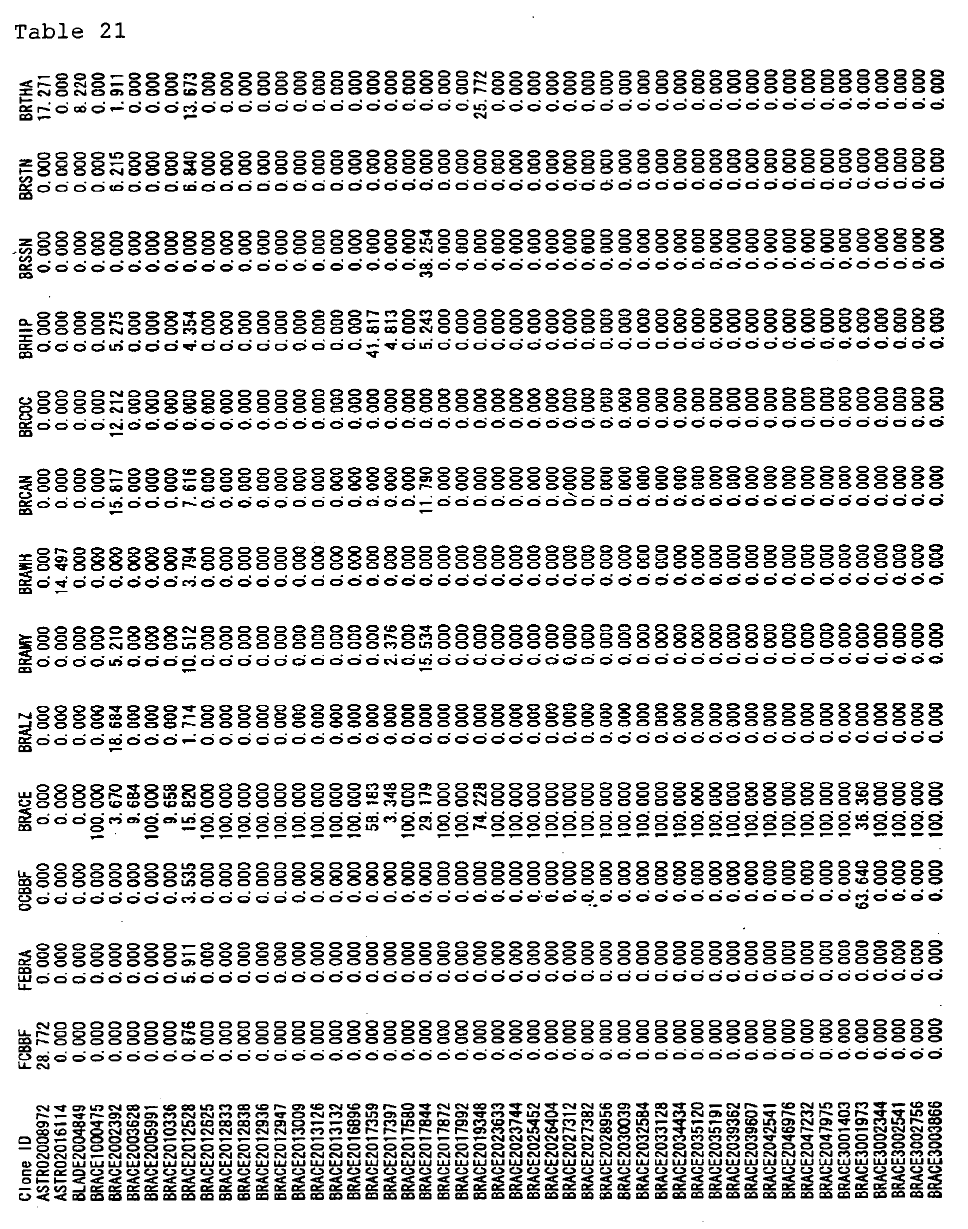

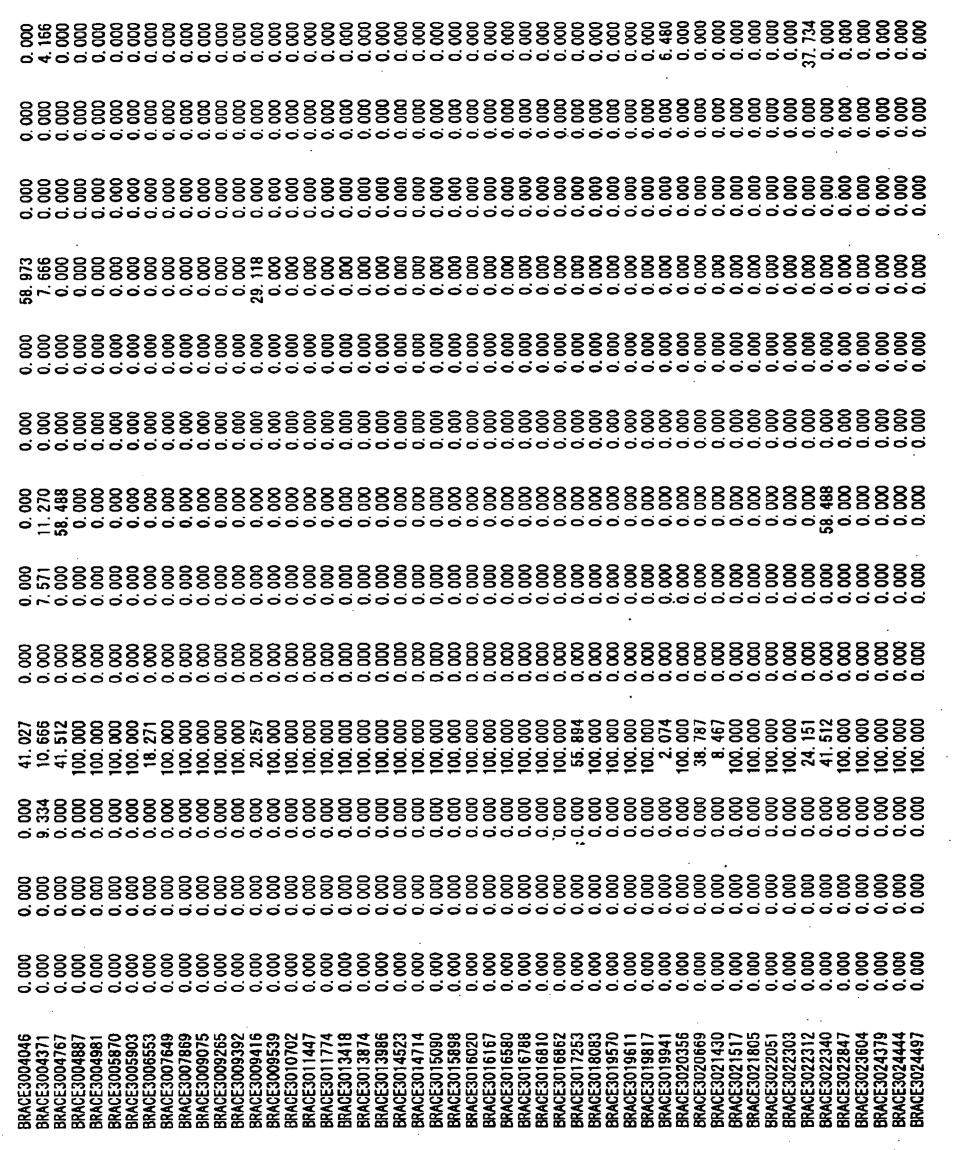

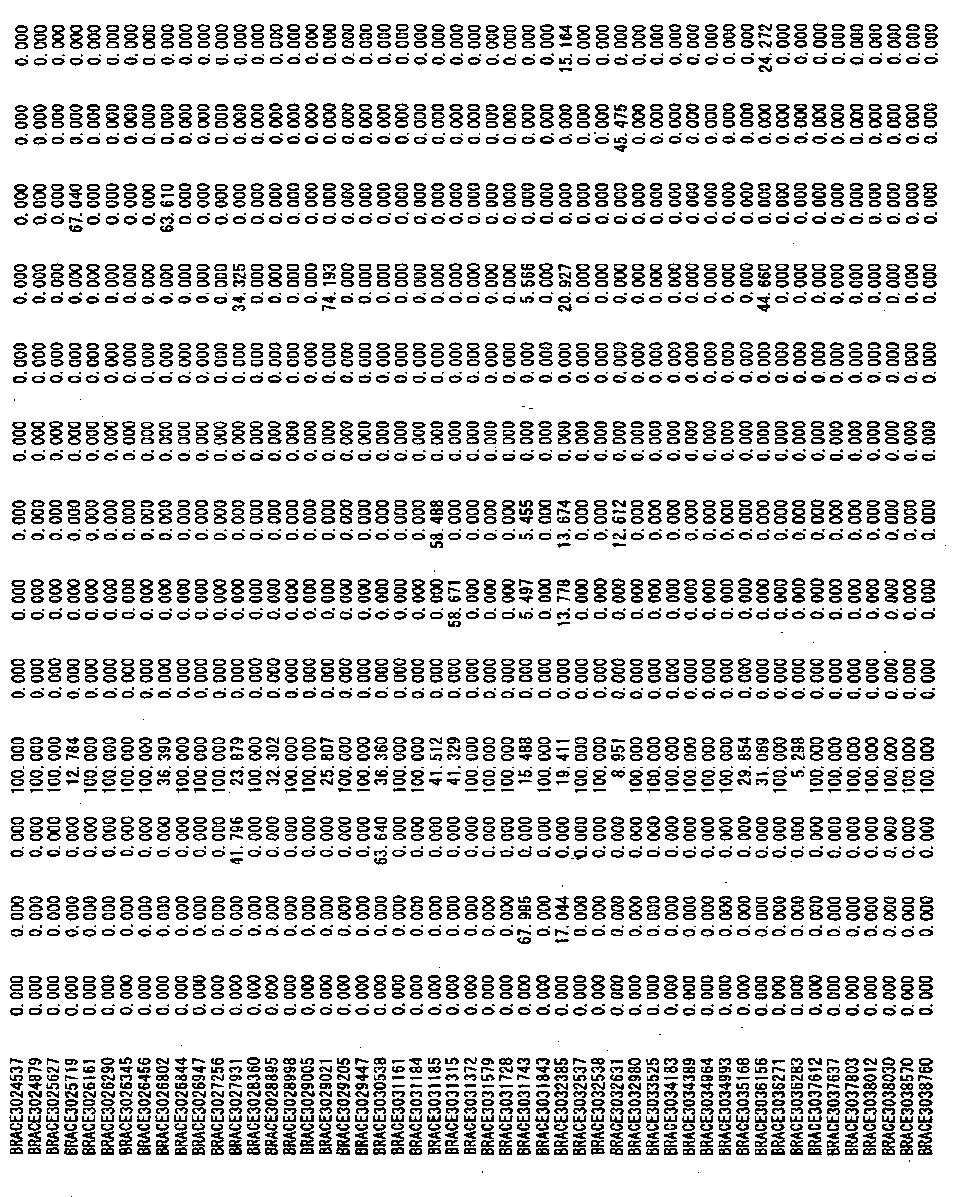

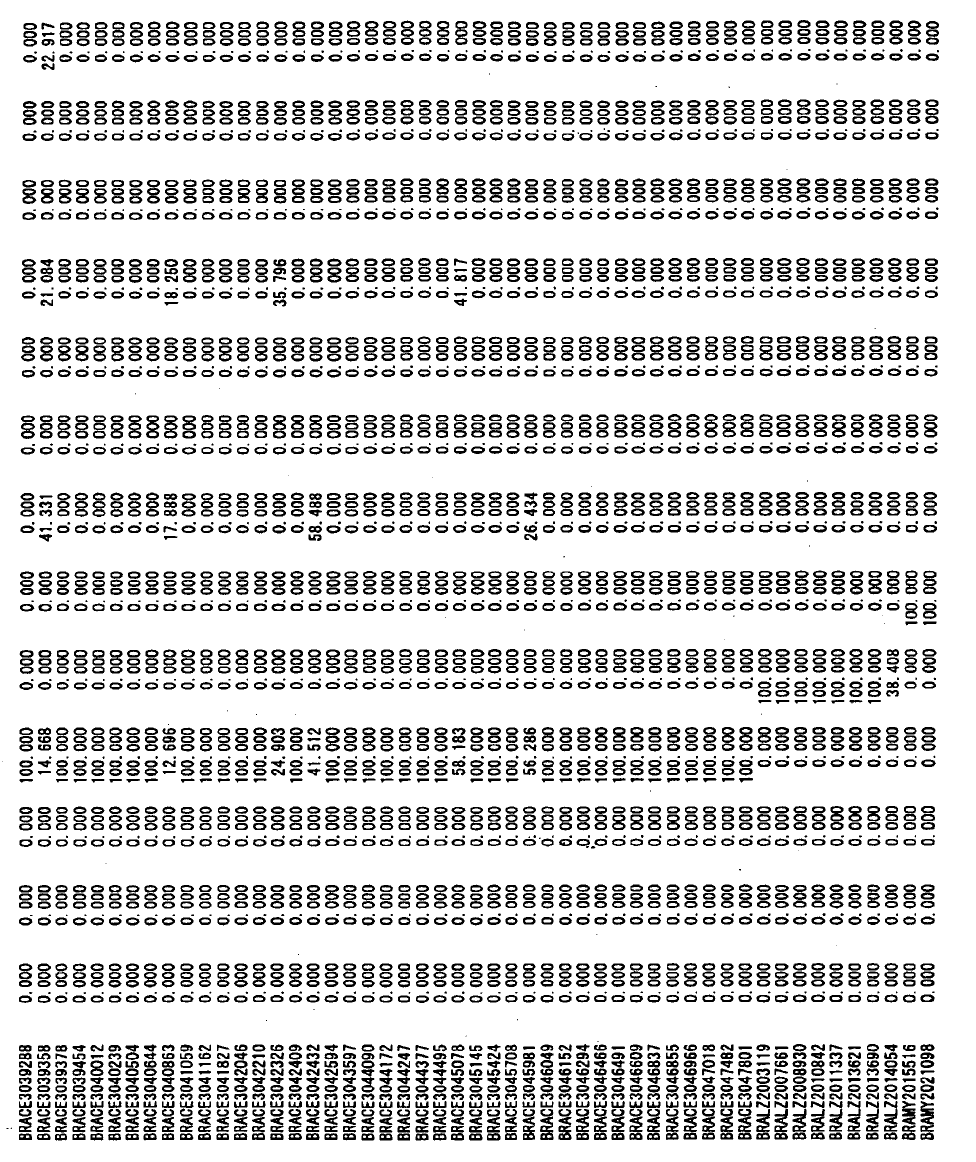

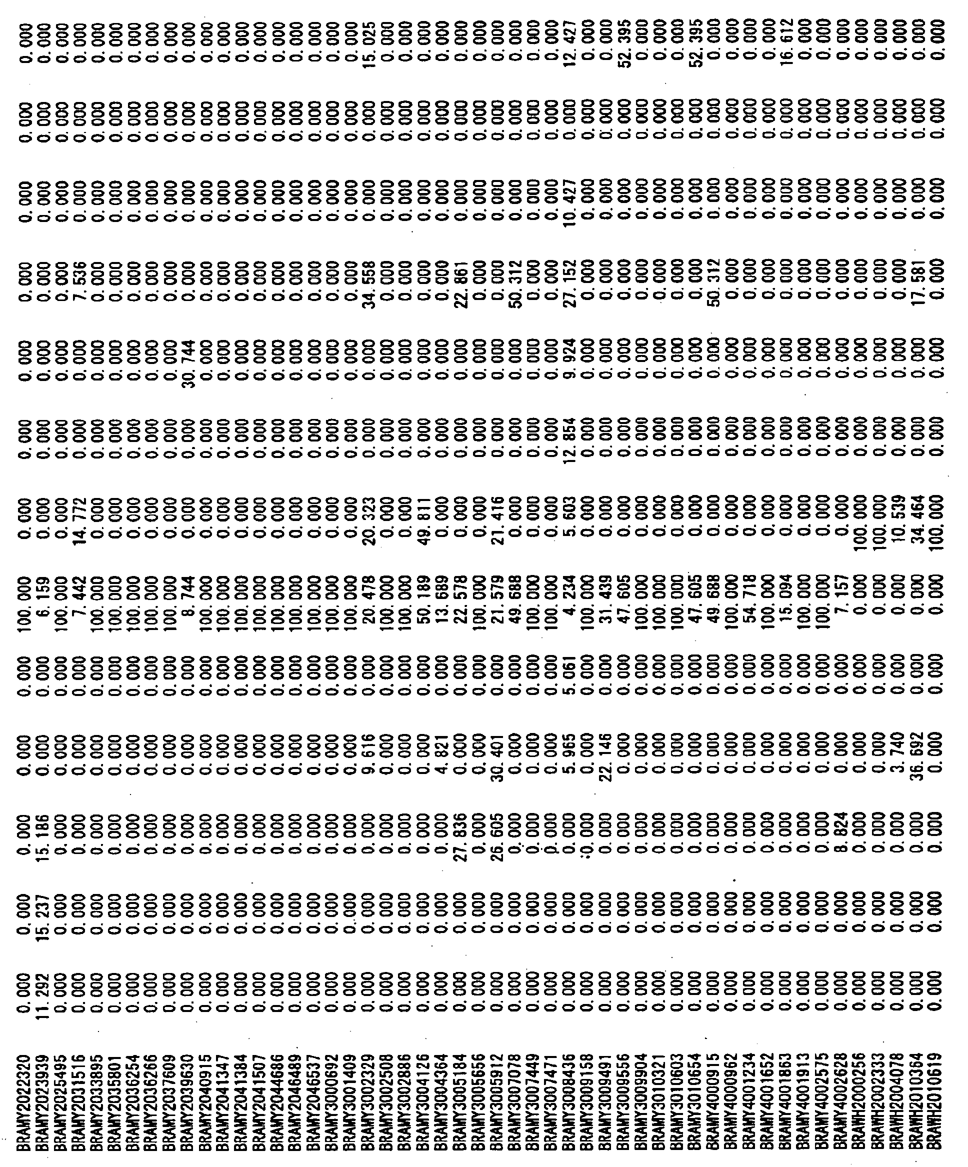

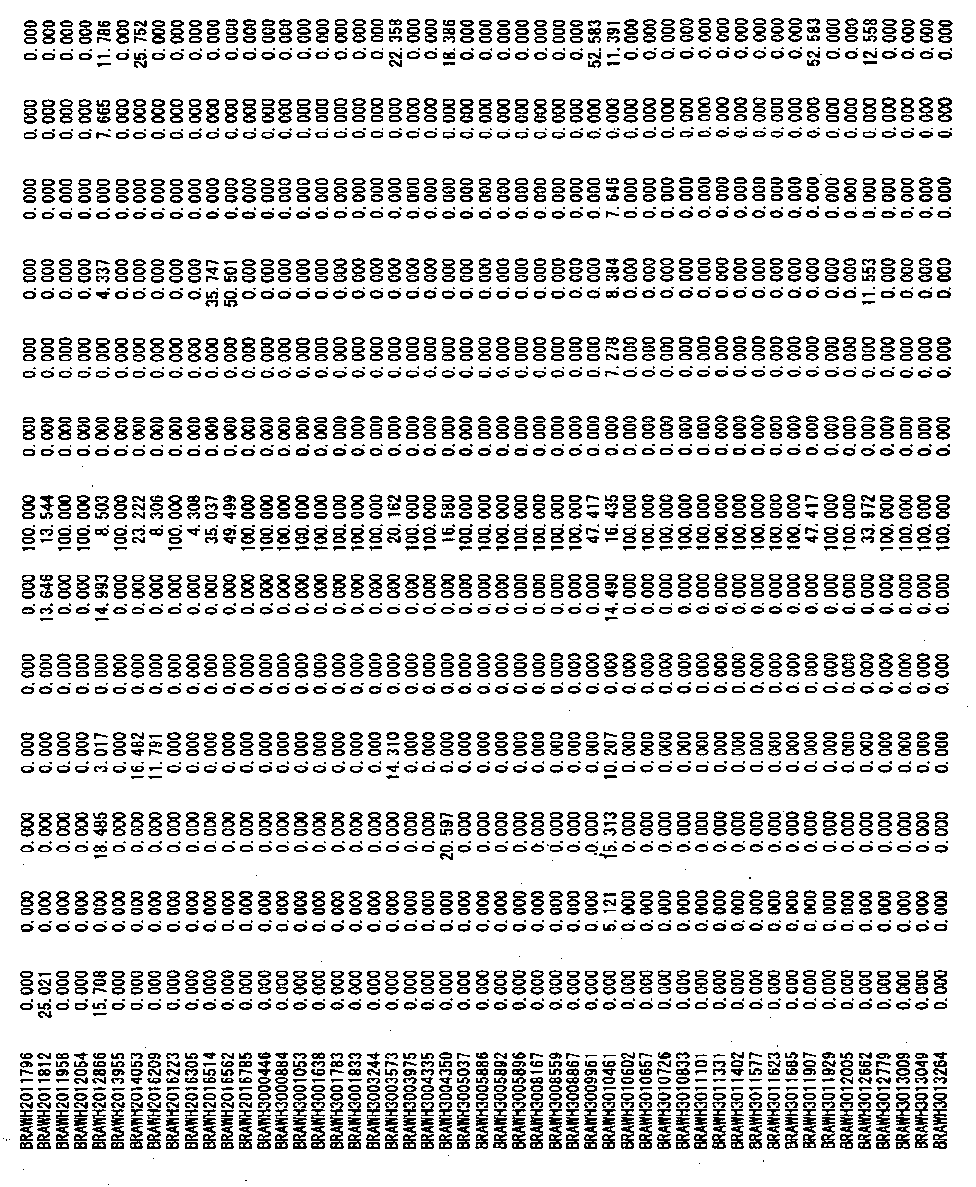

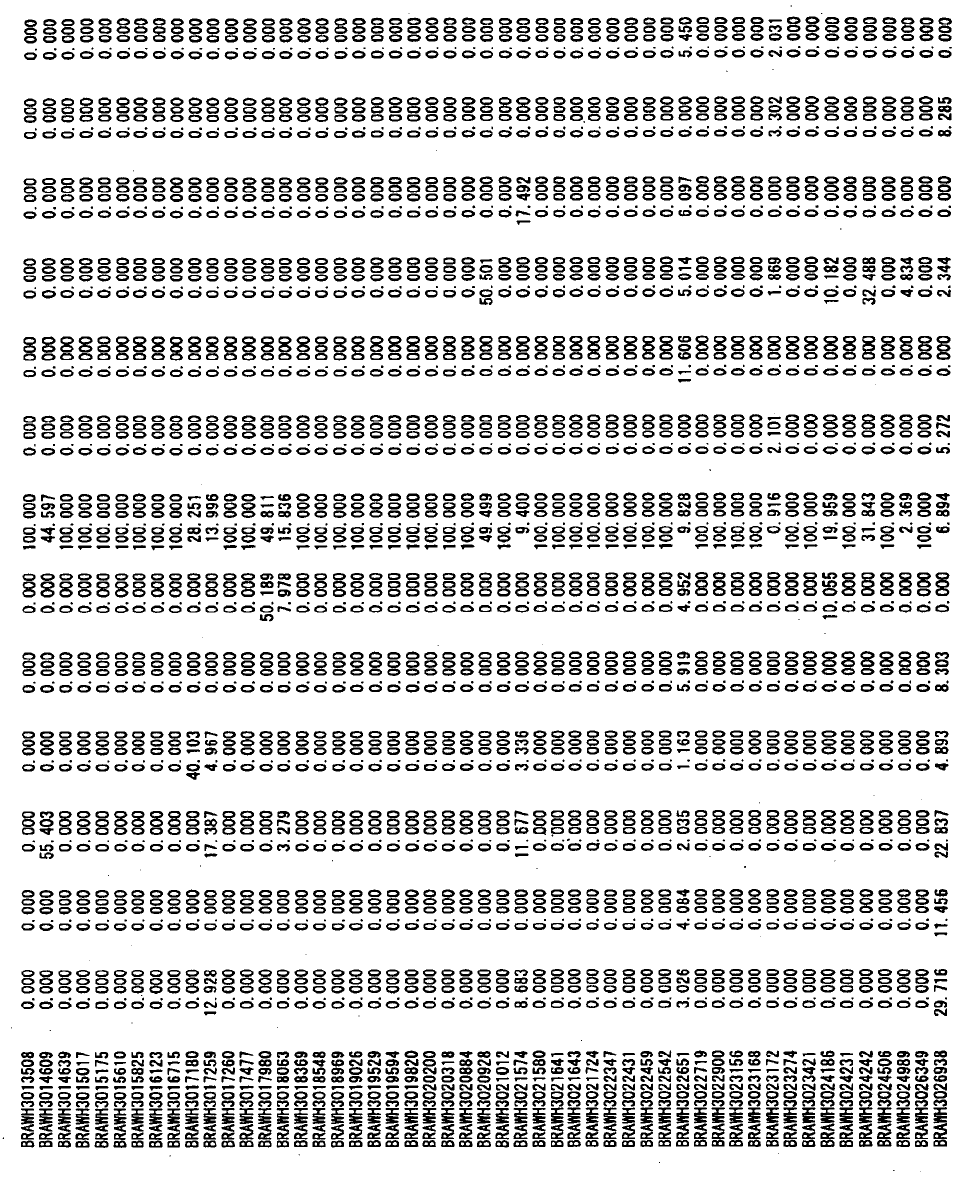

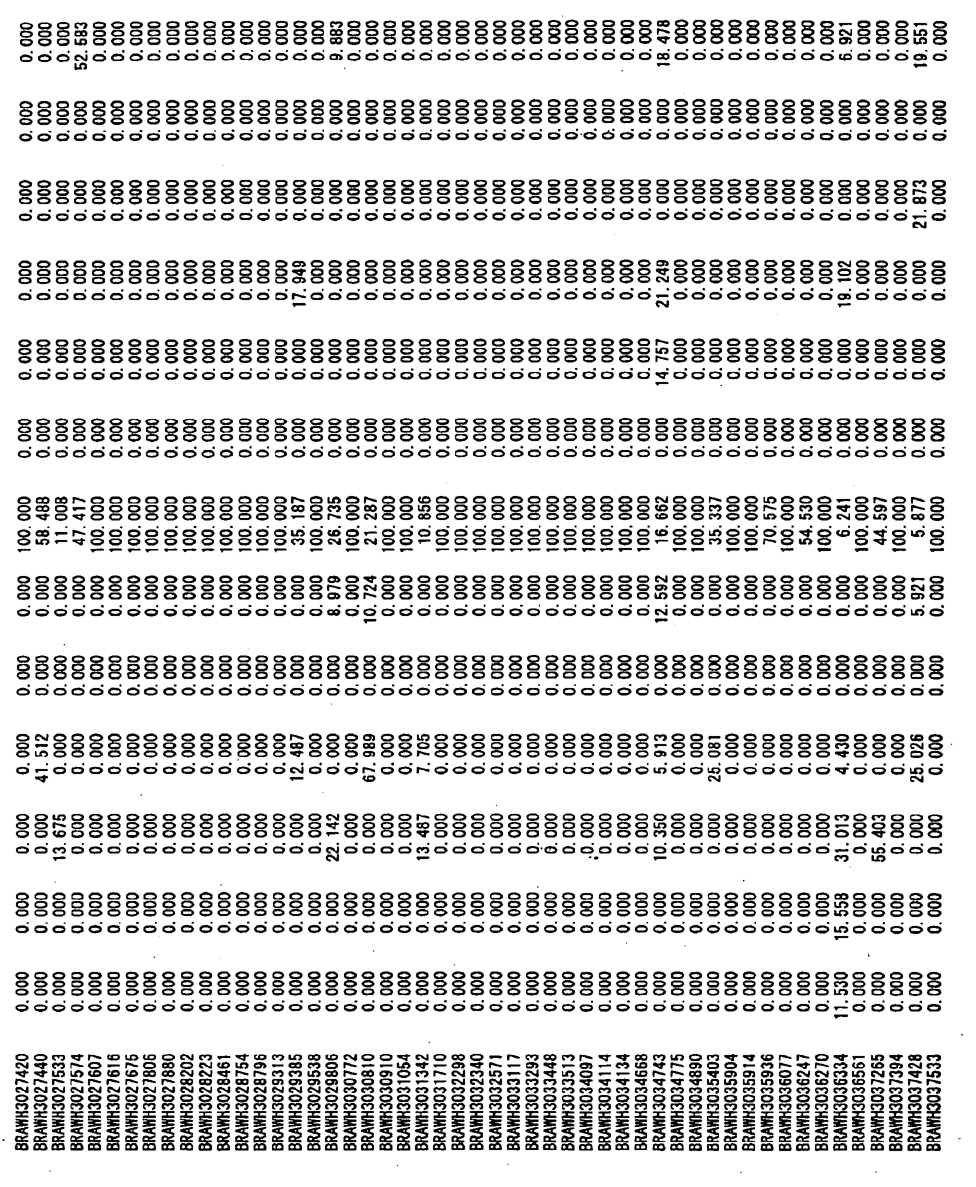

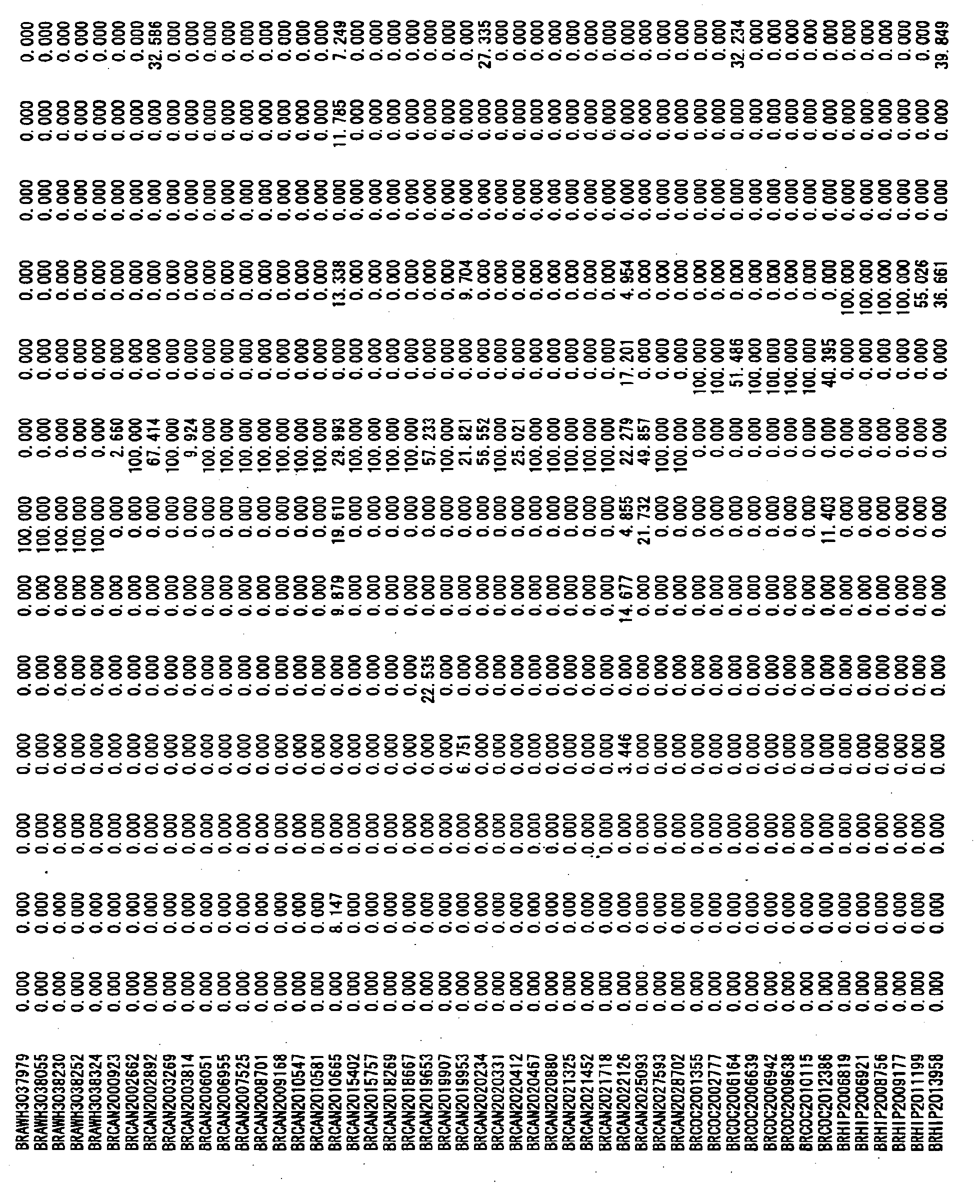

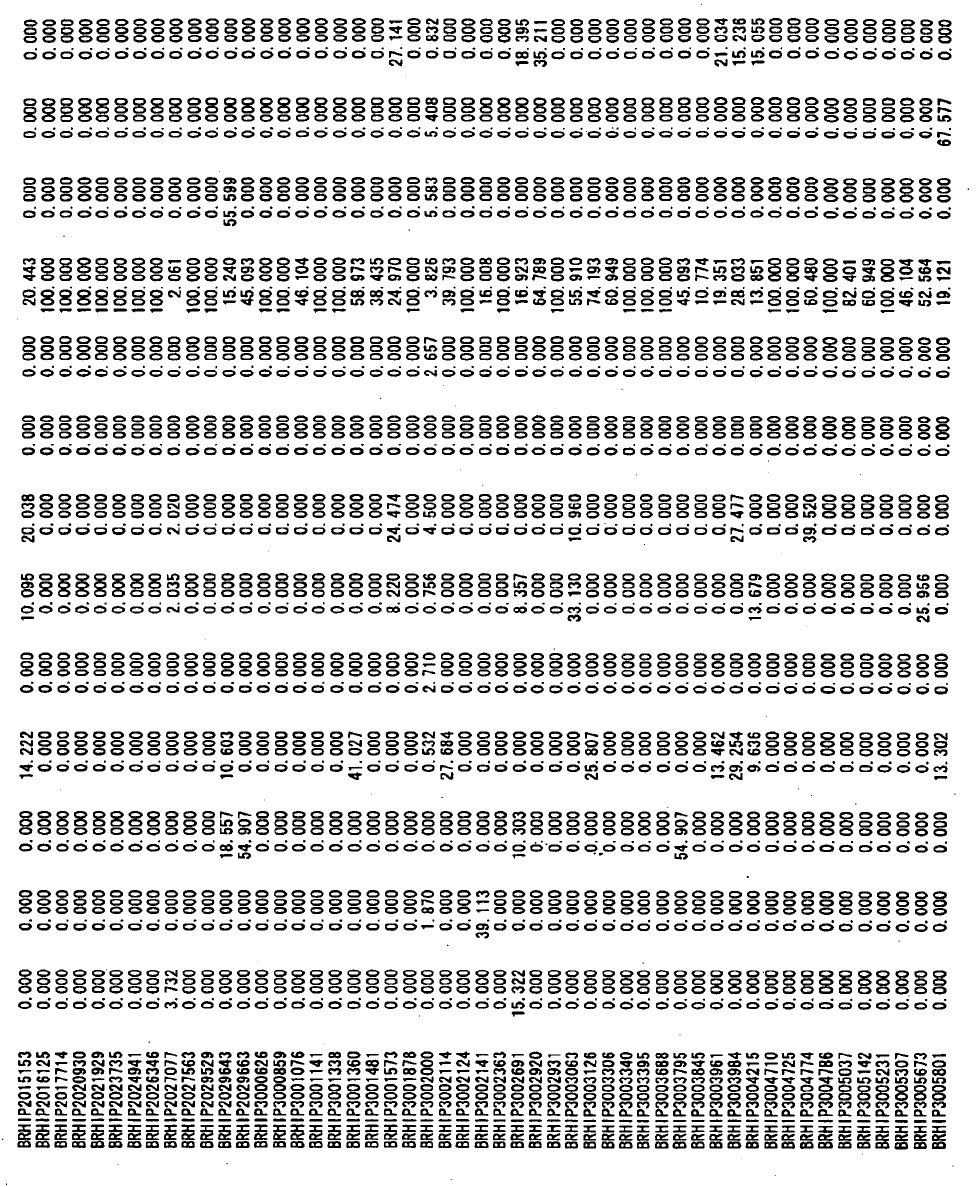

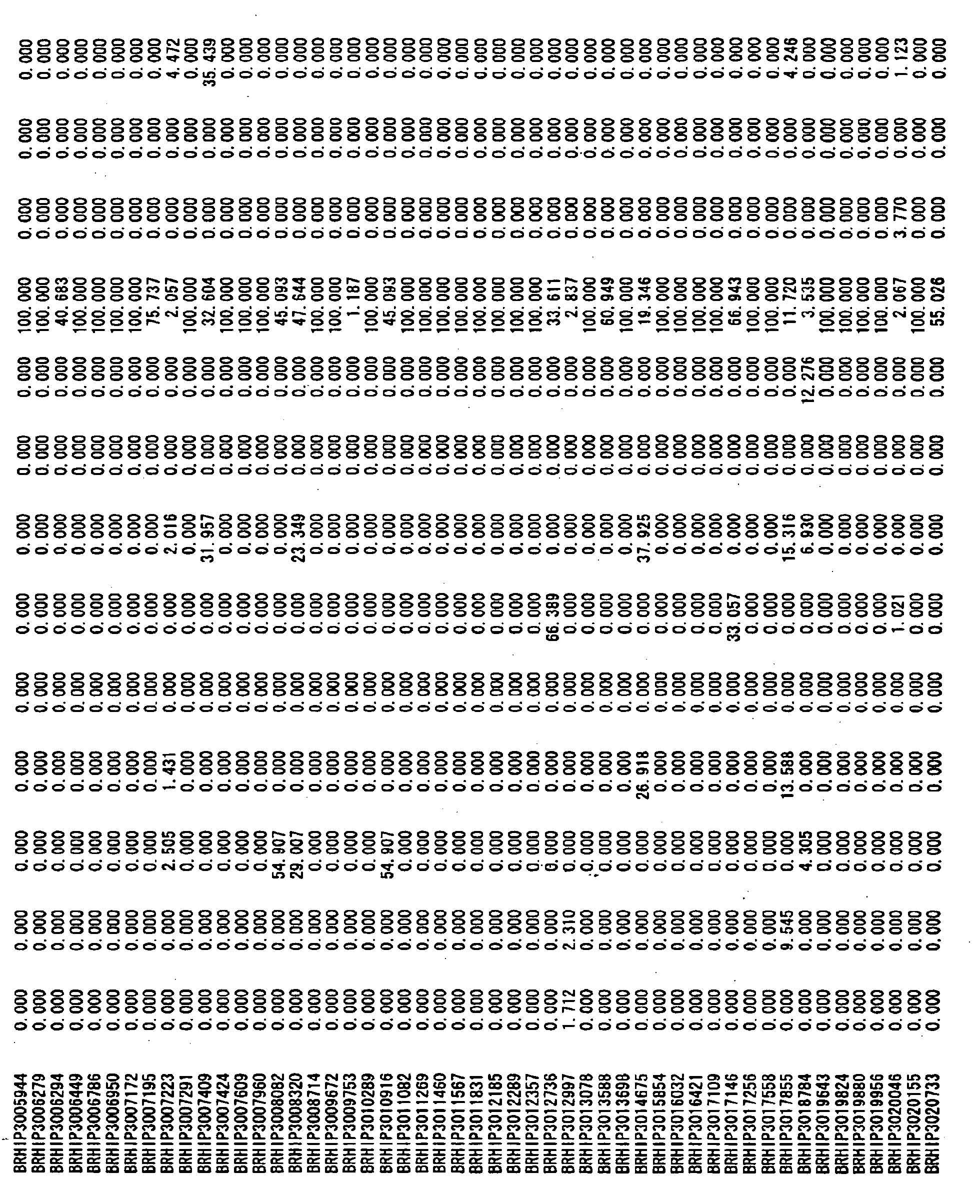

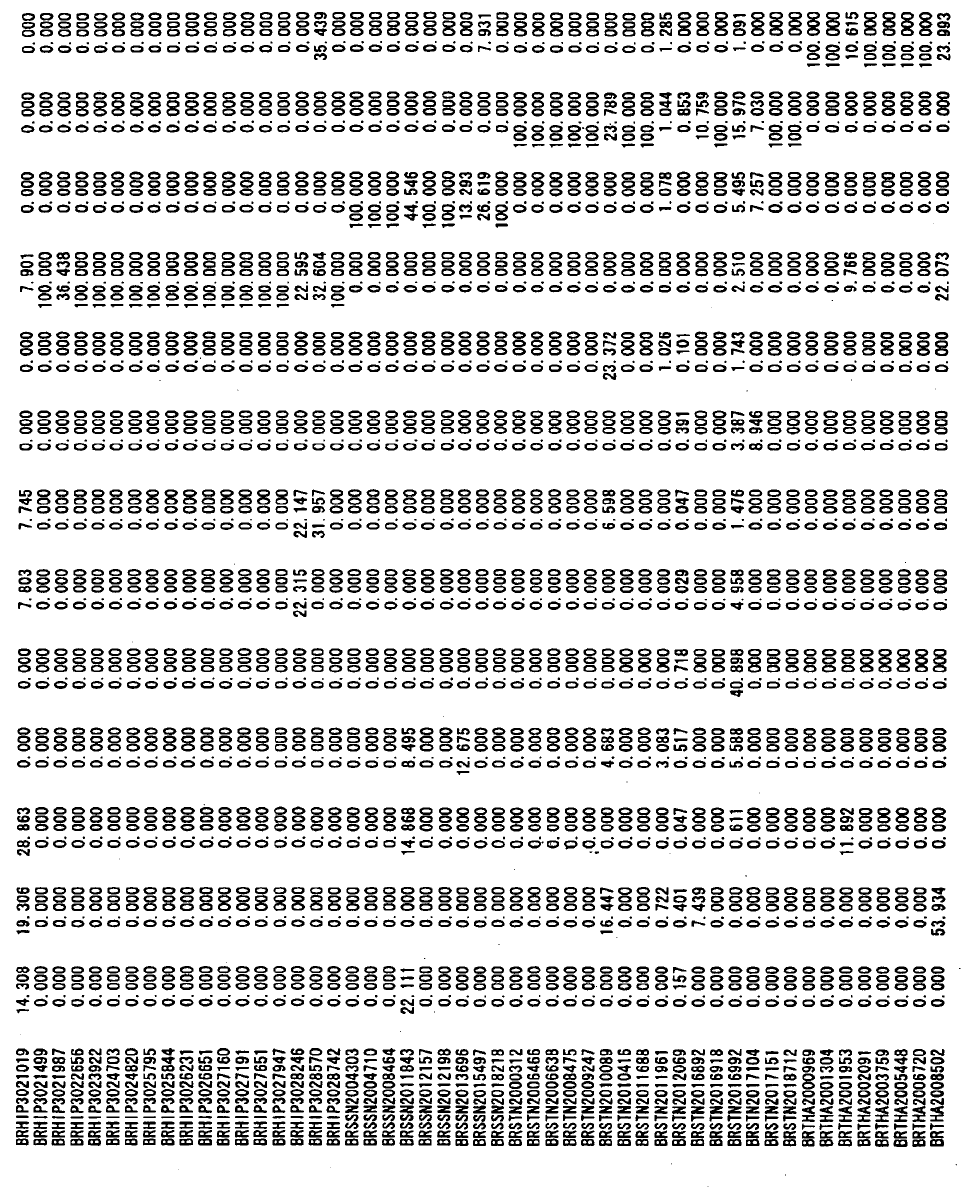

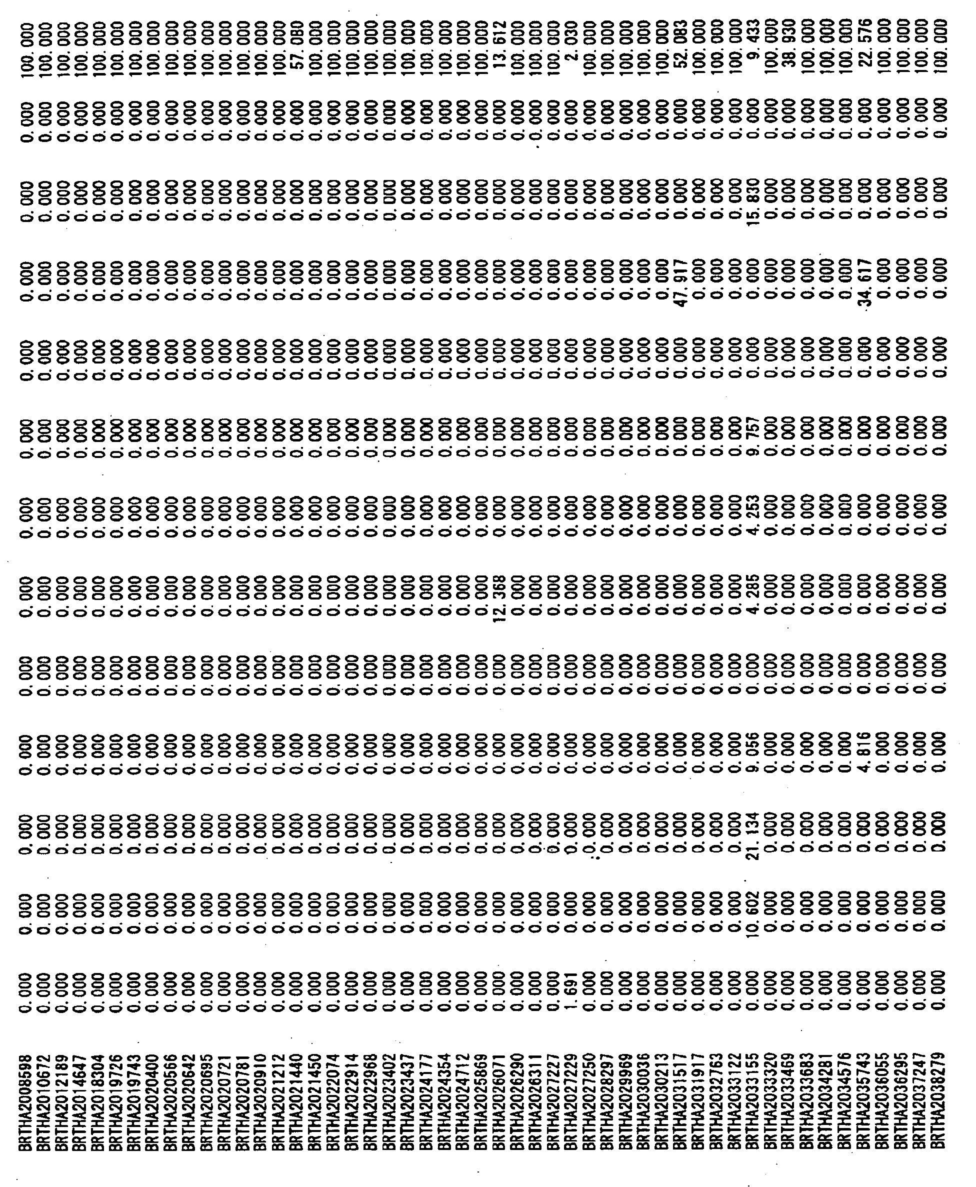

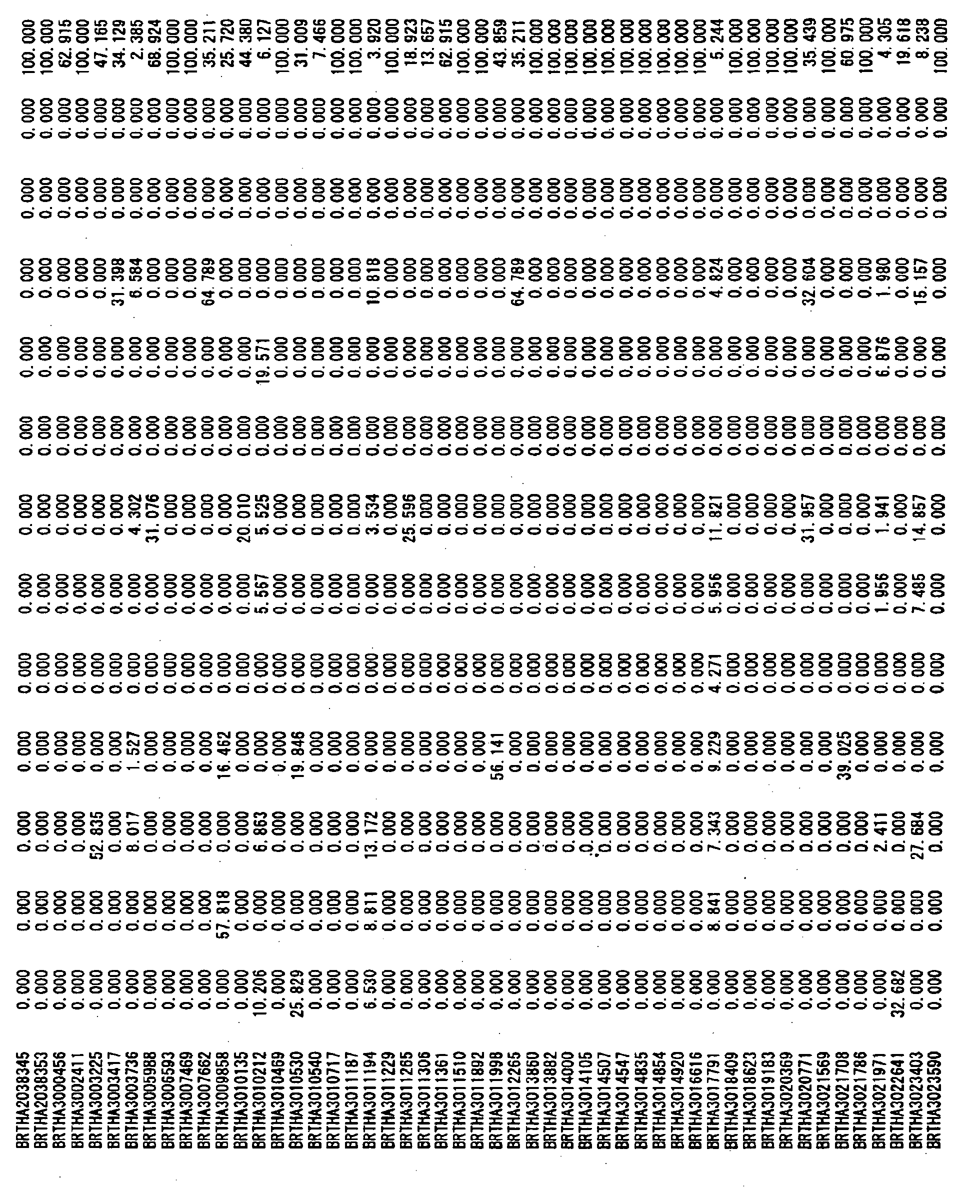

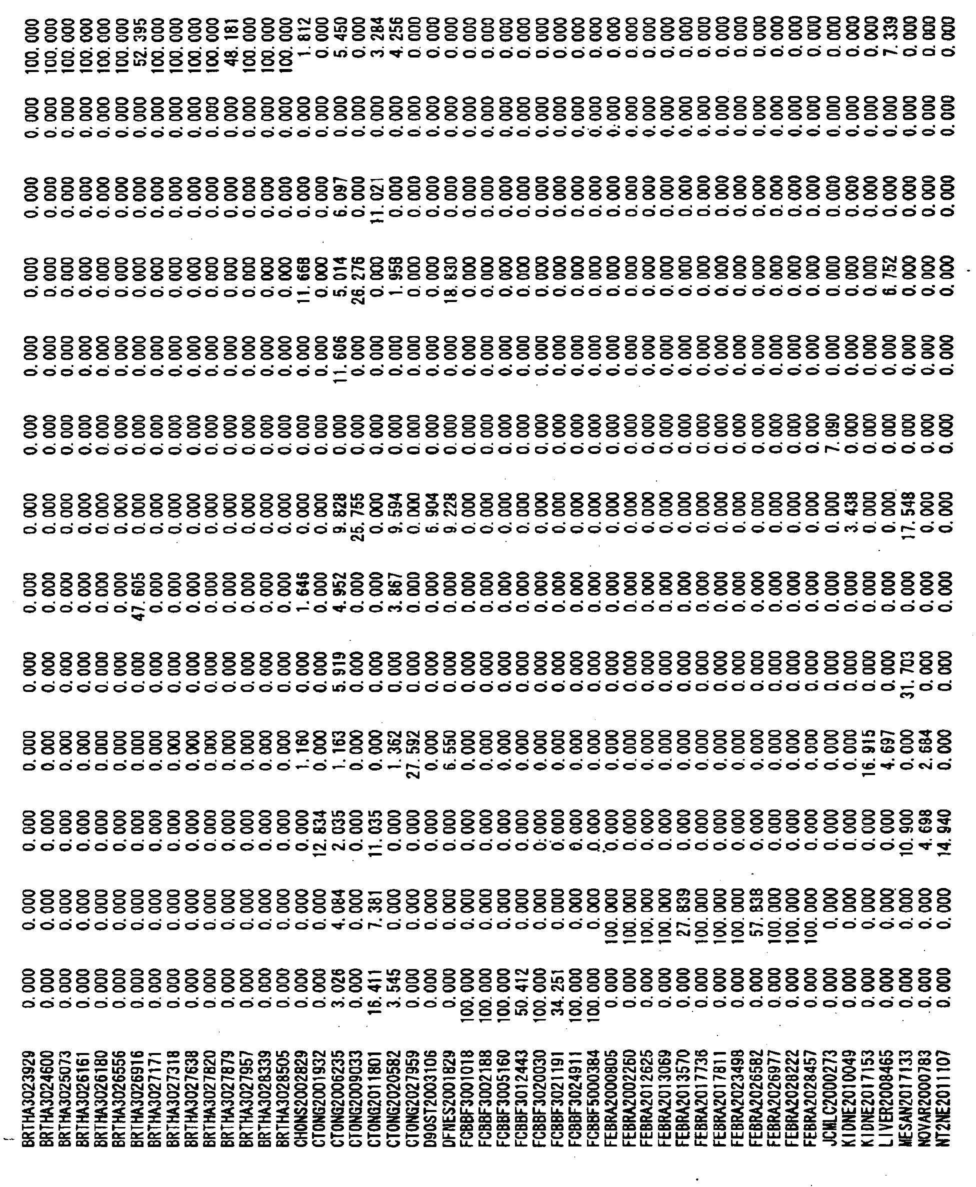

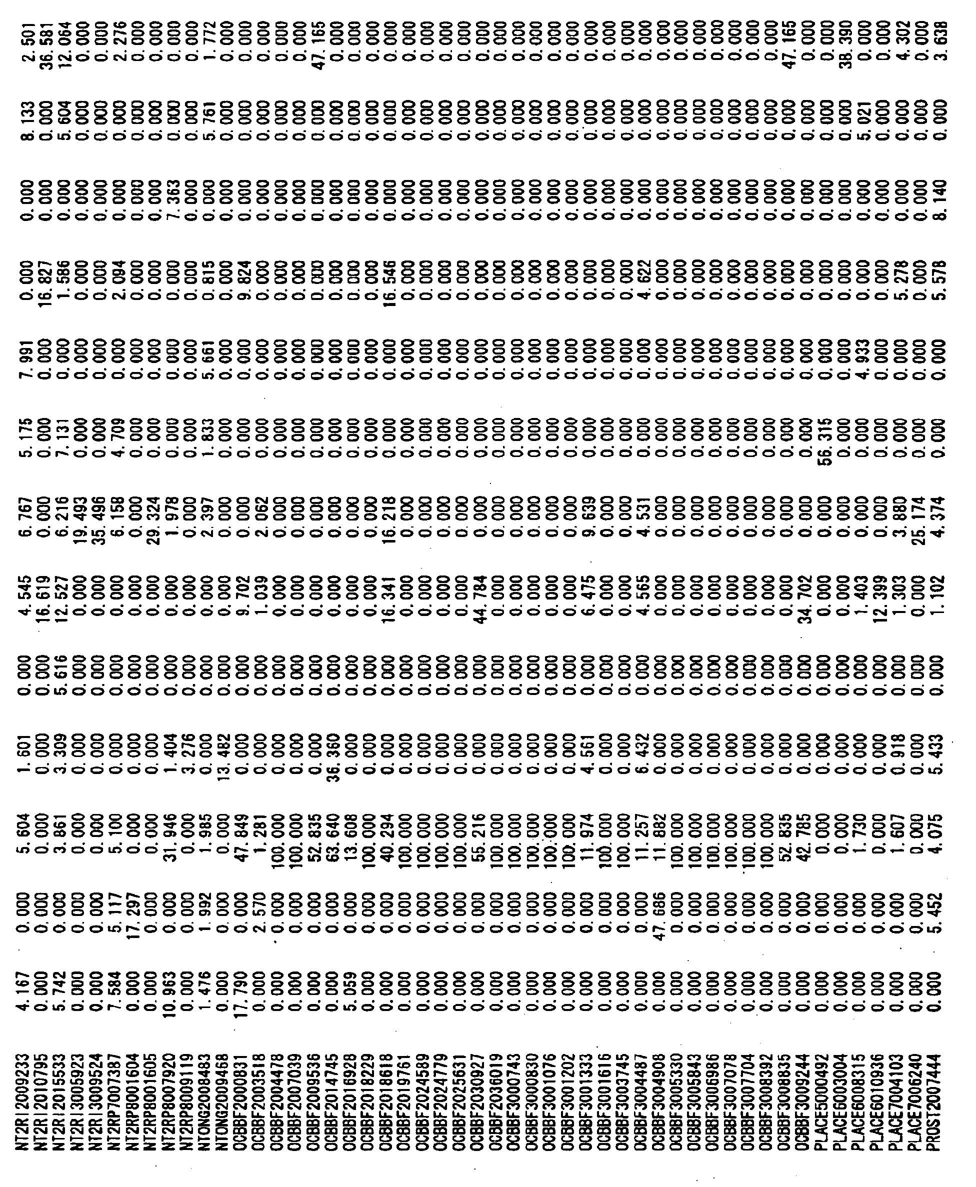

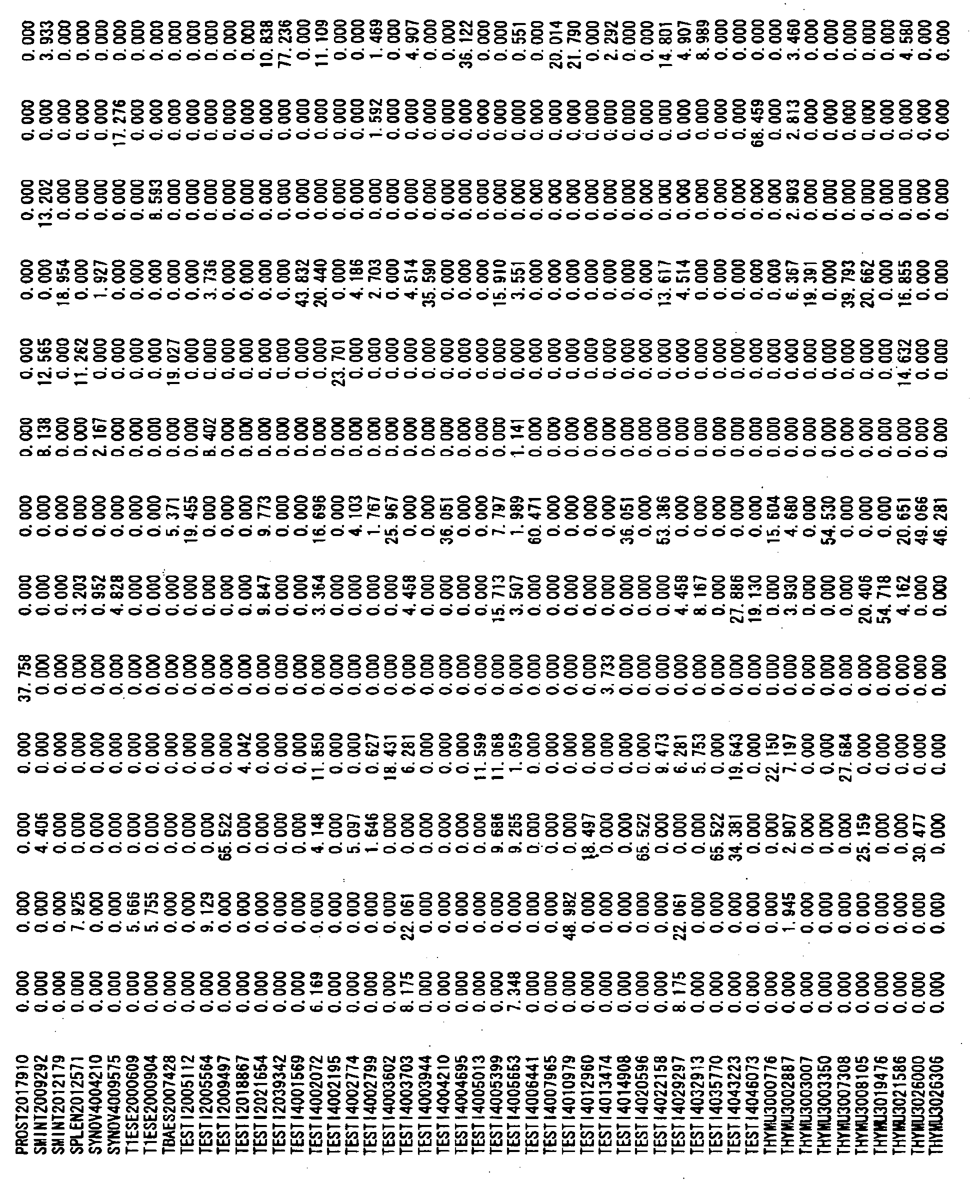

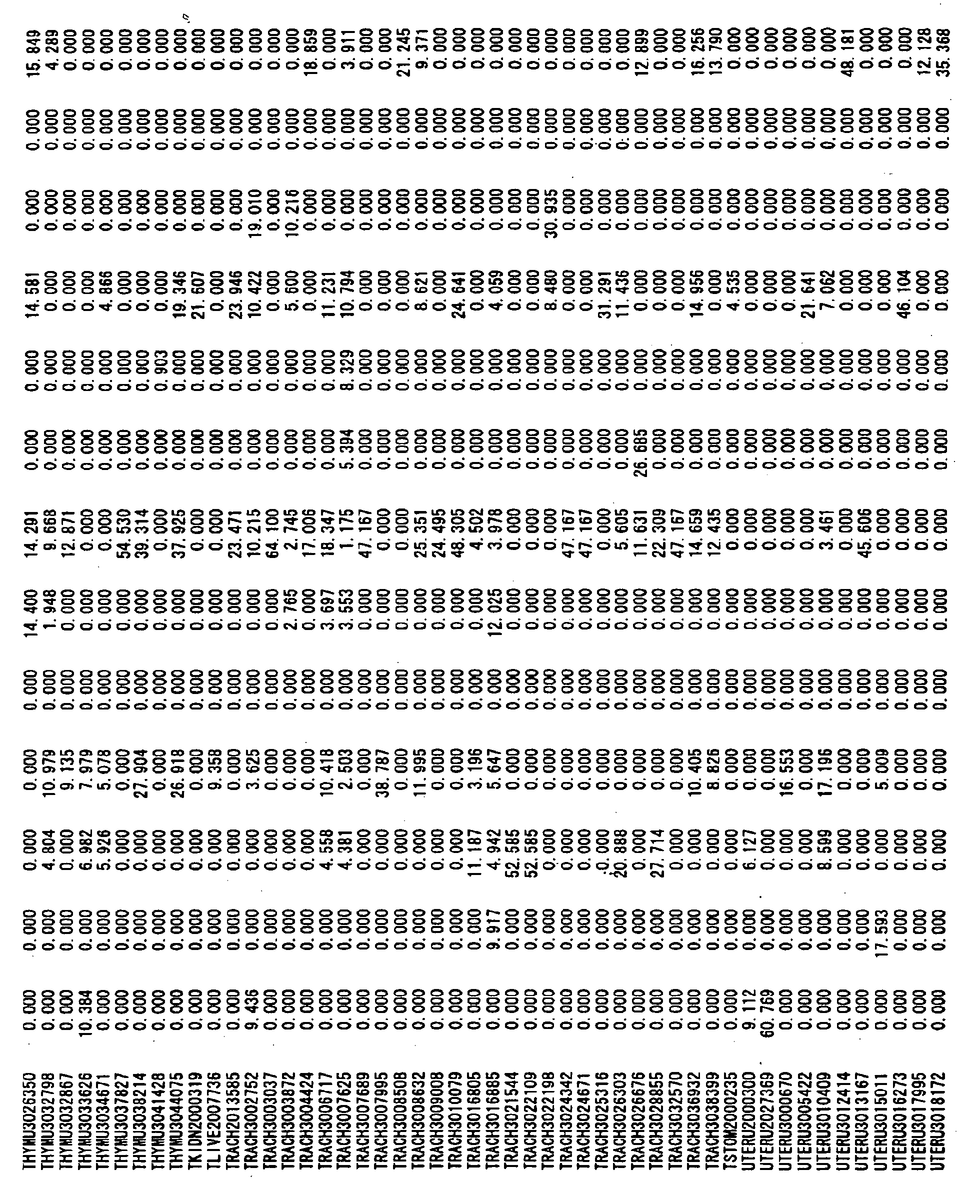

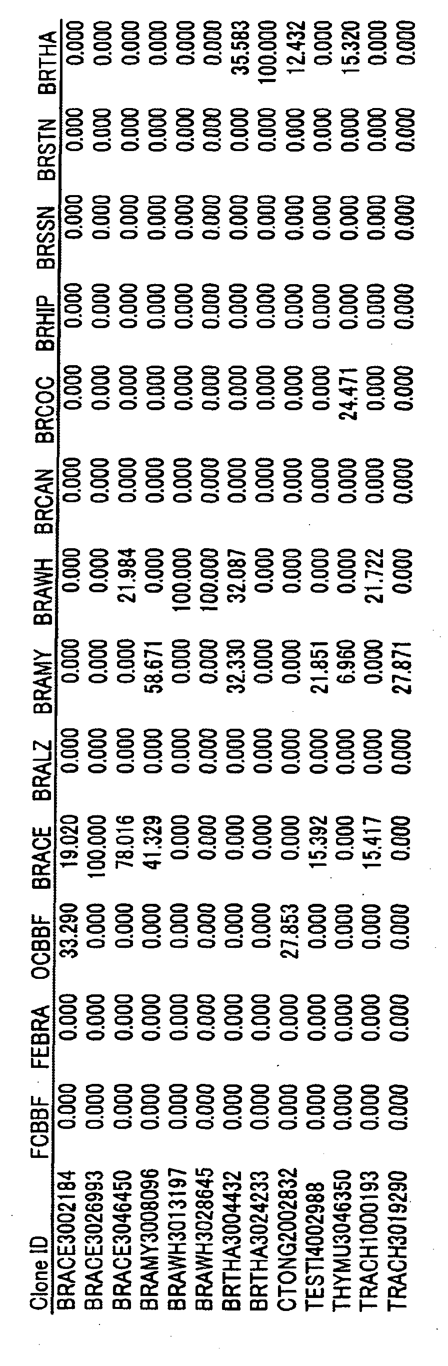

The result of comparative analysis of cDNA libraries derived from fetal heart (FEHRT) and adult heart (HEART) showed the following genes whose expression levels differed between the two.

**Table 22**

| Clone ID | HEART | FEHRT |
|---|---|---|
| BRACE2012528 | 6.328 | 0.000 |
| BRACE3004371 | 25.063 | 0.000 |
| BRCAN2003814 | 0.000 | 90.076 |
| BRSTN2011961 | 1.932 | 0.000 |
| BRSTN2012069 | 1.641 | 0.394 |
| BRSTN2016992 | 6.566 | 0.000 |
| HEART2002531 | 100.000 | 0.000 |
| NTONG2008483 | 5.330 | 0.000 |
| PROST2002078 | 16.213 | 0.000 |
| T1ESE2000609 | 15.163 | 0.000 |

The result of comparative analysis of cDNA libraries derived from fetal kidney (FEKID) and adult kidney (KIDNE) showed the following genes whose expression levels differed between the two.

**Table 23**

| Clone ID | KIDNE | FEKID |
|---|---|---|
| BRACE3004371 | 13.097 | 0.000 |
| BRAMY2039630 | 60.511 | 0.000 |
| BRAMY3004364 | 23.683 | 0.000 |
| BRAWH2004078 | 18.371 | 0.000 |
| BRHIP3002000 | 10.458 | 0.000 |
| BRSTN2011961 | 0.000 | 6.265 |
| BRSTN2012069 | 0.429 | 1.841 |
| BRTHA2027229 | 0.000 | 19.799 |
| KIDNE2004531 | 80.699 | 0.000 |
| KIDNE2010049 | 11.987 | 0.000 |
| KIDNE2014496 | 100.000 | 0.000 |
| KIDNE2015987 | 100.000 | 0.000 |
| KIDNE2016464 | 100.000 | 0.000 |
| KIDNE2017153 | 83.085 | 0.000 |
| KIDNE2018268 | 100.000 | 0.000 |
| NT2RP7007387 | 7.156 | 0.000 |
| TESTI2005112 | 67.827 | 0.000 |
| TESTI4002799 | 0.000 | 9.553 |
| THYMU3001776 | 22.094 | 0.000 |
| THYMU3029795 | 37.093 | 0.000 |
| THYMU3032867 | 44.871 | 0.000 |

The result of comparative analysis of cDNA libraries derived from fetal lung (FELNG) and adult lung (HLUNG) showed the following genes whose expression levels differed between the two.

**Table 24**

| Clone ID | HLUNG | FELNG |
|---|---|---|
| BRACE3036283 | 27.376 | 0.000 |
| BRAMY2031516 | 27.091 | 0.000 |
| BRSTN2011961 | 2.124 | 0.000 |
| BRSTN2012069 | 0.763 | 2.238 |
| HLUNG2012600 | 100.000 | 0.000 |
| MESAN2009156 | 38.142 | 0.000 |
| NTONG2008483 | 2.930 | 0.000 |
| PROST2007444 | 4.011 | 0.000 |
| TESTI4003703 | 16.229 | 0. 000 |
| TESTI4005653 | 1.824 | 0. 000 |
| TESTI4013474 | 3.790 | 0.000 |
| TESTI4029297 | 16.229 | 0.000 |
| THYMU3001776 | 11.622 | 0.000 |
| THYMU3033626 | 20.615 | 0.000 |
| THYMU3034671 | 8.747 | 0.000 |
| THYMU3041428 | 0.935 | 0.000 |
| THYMU3044188 | 0.000 | 96.268 |
| TRACH3022198 | 30.484 | 0.000 |

### EXAMPLE 9

### In-silico expression frequency analysis based on large-scale analysis data for 5'-end sequences

Analyzing the expression level of a gene in various organs, tissues or cells is an exceedingly important step in clarifying the function of that organ, tissue, or cell, as well as in clarifying gene networks and in studying a causative gene of a morbid state. For example, if the expression level of a gene differs between a normal tissue and a malignant tissue, there is a possibility that that gene is involved in cancer. If tissue canceration can be suppressed by downregulating the expression of that gene, a compound comprising such downregulating activity can be used as an anticancer drug.

Various methods for analyzing gene expression frequency have been developed. For example, wet-type experiment-based methods include Northern blotting and RT-PCR, and of the use of gene chips and microarrays, where target samples synthesized from tissue or cell-derived RNA are hybridized to polynucleotides that comprise partial gene sequences synthesized on a base, or cDNA clones attached as plasmids directly to a base, and then signals are detected (Experiment Medicine, Vol. 17, No. 8, 980-1056 (1999), Eds., Muramatsu and Nawa, Cell Technology, Suppl. "DNA Microarray and New PCR Methods" (Shujunsha, 2000)). A method called "ATAC-PCR" is also available (Kato. K (1997) Nucleic Acids Res. 25, 4694-6), which comprises the steps of cleaving cDNA synthesized from tissue or cell-derived RNA, attaching adapters of different length depending on the type of tissue or cell, carrying out competitive PCR using a primer which contains a fluorescent dye and a sequence complementary to the adapter, and a primer specific to the gene, and then analyzing the expression level of the gene. In addition, an in-silico analysis-based method using sequence data is available. A database called BODYMAP (http://bodymap.ims.u-tokyo.ac.jp/) has been constructed by randomly extracting gene clones from cDNA libraries of various tissues and cells, combining clones homologous to one another as a cluster, classifying the genes in each cluster unit based on homology information on the nucleotide sequences of cDNA 3' ends, and then obtaining information on gene expression frequency by comparing the number of clones in respective clusters.

Both experiment-based and in-silico analysis-based methods are widely accepted methods of gene expression analysis. These methods, and almost all other established methods for analyzing the frequency of gene expression, are based on analysis of 3'-end gene sequences. In human genes the 3'-untranslated regions (3'-UTR) are usually long, and thus there is controversy as to whether the results of such methods directly correspond to gene expression frequency and polypeptide expression pattern. However, the analysis method for gene expression frequency of the present invention is based on 5'-end gene sequences, and thus the expression pattern of each gene is a more accurate and faithful reproduction of the original *in vivo* pattern.

cDNA libraries in which the probability of cDNA completeness is exceedingly high were prepared from various tissues and cells as described in Example 1. cDNA clones were randomly selected from each library, the cDNA 5'-end sequences were determined, and a sequence database was constructed using this data. This database contains the nucleotide sequences of 1,402,069 clones, a sufficiently large population for analysis. The libraries used in the analysis, the number of cDNA clones whose 5'-end sequences were determined, and the weighted value for a single sequence in each library (100 divided by the number of sequences analyzed in each library) are described below.

**Table 25**

| Library Names | Number of clones whose 5' ends were analyzed | Weighted values for single sequences (100/Number of sequences) |
|---|---|---|
| 3NB69 | 8182 | 0.012221951 |
| ACTVT | 684 | 0.14619883 |
| ADIPS | 614 | 0.16286645 |
| ADRGL | 10300 | 0.009708738 |
| AHMSC | 671 | 0.149031297 |
| ASTRO | 17226 | 0.005805178 |
| BEAST | 2736 | 0.036549708 |
| BGGI1 | 1904 | 0.052521008 |
| BLADE | 8503 | 0.011760555 |
| BNGH4 | 7739 | 0.012921566 |
| BRACE | 84087 | 0.001189244 |
| BRALZ | 16517 | 0.006054368 |
| BRAMY | 59232 | 0.001688277 |
| BRASW | 158 | 0.632911392 |
| BRAWH | 59682 | 0.001675547 |
| BRCAN | 26014 | 0.003844084 |
| BRCOC | 16847 | 0.005935775 |
| BRHIP | 58498 | 0.00170946 |
| BRSSN | 16035 | 0.006236358 |
| BRSTN | 16552 | 0.006041566 |
| BRTHA | 53818 | 0.001858114 |
| CD34C | 1421 | 0.070372977 |
| CERVX | 2888 | 0.034626039 |
| CHONS | 2694 | 0.037119525 |
| COLON | 8501 | 0.011763322 |
| CORDB | 711 | 0.140646976 |
| CTONG | 32043 | 0.003120806 |
| D30ST | 5112 | 0.019561815 |
| D60ST | 889 | 0.112485939 |
| D90ST | 4426 | 0.022593764 |
| DFNES | 10126 | 0.009874593 |
| ERLTF | 2178 | 0.045913682 |
| FCBBF | 32305 | 0.003095496 |
| FEBRA | 23941 | 0.004176935 |
| FEHRT | 2866 | 0.034891835 |
| FEKID | 2759 | 0.036245016 |
| FELIV | 186 | 0.537634409 |
| FELNG | 2775 | 0.036036036 |
| HCASM | 8989 | 0.011124708 |
| HCHON | 9432 | 0.010602205 |
| HEART | 8946 | 0.01117818 |
| HELAC | 680 | 0.147058824 |
| HHDPC | 8476 | 0.011798018 |
| HLUNG | 16272 | 0.006145526 |
| HSYRA | 7985 | 0.012523482 |
| IMR32 | 16914 | 0.005912262 |
| JCMLC | 2171 | 0.046061723 |
| KIDNE | 17119 | 0.005841463 |
| LIVER | 6885 | 0.014524328 |
| LYMPB | 2630 | 0.038022814 |
| MAMGL | 184 | 0.543478261 |
| MESAN | 16095 | 0.00621311 |
| MESTC | 691 | 0.1447178 |
| N1ESE | 2628 | 0.03805175 |
| NB9N4 | 1764 | 0.056689342 |
| NCRRM | 703 | 0.142247511 |
| NCRRP | 699 | 0.143061516 |
| NESOP | 2805 | 0.035650624 |
| NETRP | 9236 | 0.010827198 |
| NHNPC | 2392 | 0.04180602 |
| NOVAR | 2504 | 0.039936102 |
| NT2NE | 16468 | 0.006072383 |
| NT2RI | 32842 | 0.003044882 |
| NT2RM | 2074 | 0.048216008 |
| NT2RP | 24763 | 0.004038283 |
| NTISM | 181 | 0.552486188 |
| NTONG | 8098 | 0.012348728 |
| OCBBF | 48042 | 0.002081512 |
| PANCR | 182 | 0.549450549 |
| PEBLM | 7940 | 0.012594458 |
| PERIC | 8860 | 0.011286682 |
| PLACE | 33535 | 0.002981959 |
| PROST | 16829 | 0.005942124 |
| PUAEN | 10577 | 0.009454477 |
| RECTM | 2743 | 0.036456435 |
| SALGL | 185 | 0.540540541 |
| SKMUS | 8470 | 0.011806375 |
| SKNMC | 7656 | 0.013061651 |
| SKNSH | 8692 | 0.011504832 |
| SMINT | 16913 | 0.005912612 |
| SPLEN | 34307 | 0.002914857 |
| STOMA | 8770 | 0.011402509 |
| SYNOV | 27671 | 0.003613892 |
| T1ESE | 2687 | 0.037216226 |
| TBAES | 8484 | 0.011786893 |
| TCERX | 2828 | 0.035360679 |
| TCOLN | 2815 | 0.035523979 |
| TESOP | 8723 | 0.011463946 |
| TESTI | 91301 | 0.001095278 |
| THYMU | 71574 | 0.001397155 |
| TKIDN | 16123 | 0.00620232 |
| TLIVE | 8681 | 0.01151941 |
| TLUNG | 2884 | 0.034674064 |
| TOVAR | 2740 | 0.03649635 |
| TRACH | 53281 | 0.001876842 |
| TSTOM | 2779 | 0.035984167 |
| TUTER | 2678 | 0.037341299 |
| UMVEN | 633 | 0.157977883 |
| UTERU | 50040 | 0.001998401 |

Using a nucleotide sequence homology search program, the nucleotide sequences of respective clones in this database were categorized (clustered) into groups, each of which contained homologous sequences. Cluster members (the number of clones whose 5' ends were analyzed) were totalized and normalized for each library, resulting in an abundance ratio of genes in the cDNA library. In addition, 17,547 genes, including full-length cDNAs of the present invention, were assigned to a cluster using a nucleotide sequence homology search program.

In addition to the abundance ratio of the assigned genes in cDNA libraries, the sums of the weighted values for single sequences in each library were also calculated. For example, the expression of the clone BRACE2000753 was detectable in the BRACE, SPLEN, and THYMU libraries, based on expression analysis results. The single-sequence weighted values for these libraries are 0.001189244, 0.002914857, and 0.001397155 respectively, as seen in Table 1. Thus, their sum is 0.005501257. This parameter serves as an index for gene expression frequency in entire libraries. The higher this value, the greater the gene expression level. Listed below are the clone names of the 17,176 genes assigned to clusters, the number of cDNA clones whose 5' end sequences were determined, and the sum of the weighted values for single sequences, cluster names, and the number of clones whose 5' ends were analyzed.

**Table 26**

| Names of clones assigned to clusters | Sums of weighted values for single sequences | Cluster names | Number of clones constituting clusters |
|---|---|---|---|
| 3NB691000018 | 0.018027129 | 76695 | 2 |
| 3NB691000085 | 0.019353319 | 56523 | 3 |
| 3NB691000113 | 0.024745432 | 48667 | 2 |
| 3NB691000116 | 0.103029787 | 73878 | 17 |
| 3NB691000129 | 0.066363755 | 74681 | 5 |
| 3NB691000173 | 0.212194846 | 38917 | 27 |
| 3NB691000191 | 0.02737028 | 77141 | 4 |
| 3NB692000012 | 0.012221951 | 168462 | 1 |
| 3NB692000029 | 0.012221951 | 271188 | 1 |
| 3NB692000102 | 0.012221951 | 110443 | 1 |
| 3NB692000154 | 0.012221951 | 201888 | 1 |
| 3NB692000276 | 0.012221951 | 268979 | 1 |
| 3NB692000281 | 0.761173312 | 35400 | 45 |
| 3NB692000305 | 0.012221951 | 265781 | 1 |
| 3NB692000330 | 0.379866613 | 53361 | 33 |
| 3NB692000374 | 0.154501611 | 57823 | 29 |
| 3NB692000429 | 0.012221951 | 249303 | 1 |
| 3NB692000484 | 0.016398886 | 123962 | 2 |
| 3NB692000529 | 0.015026689 | 223096 | 3 |
| 3NB692000545 | 0.059391668 | 134996 | 14 |
| 3NB692000912 | 0.356619986 | 48215 | 43 |
| 3NB692000973 | 0.023726783 | 201722 | 2 |
| 3NB692001002 | 0.085645793 | 67876 | 7 |
| 3NB692001022 | 0.134669151 | 41083 | 19 |
| 3NB692001034 | 0.012221951 | 221723 | 1 |
| 3NB692001040 | 0.012221951 | 115756 | 1 |
| 3NB692001123 | 0.012221951 | 266961 | 1 |
| 3NB692001267 | 0.012221951 | 265274 | 1 |
| 3NB692001288 | 0.1118352 | 117265 | 23 |
| 3NB692001334 | 0.624221602 | 49741 | 81 |
| 3NB692001339 | 0.071442272 | 47481 | 4 |
| 3NB692001349 | 0.012221951 | 175232 | 1 |
| 3NB692001366 | 0.013317229 | 3969 | 2 |
| 3NB692001408 | 0.012221951 | 126880 | 1 |
| 3NB692001433 | 0.048706104 | 130520 | 3 |
| 3NB692001442 | 0.138711778 | 127766 | 17 |
| 3NB692001459 | 0.029639045 | 200852 | 3 |
| 3NB692001471 | 0.012221951 | 75788 | 1 |
| 3NB692001496 | 0.021768101 | 84831 | 5 |
| 3NB692001501 | 0.012221951 | 268316 | 1 |
| 3NB692001507 | 0.012221951 | 268061 | 1 |
| 3NB692001511 | 0.019462755 | 167015 | 3 |
| 3NB692001519 | 0.012221951 | 271957 | 1 |
| 3NB692001520 | 0.013619106 | 104334 | 2 |
| 3NB692001528 | 0.071227801 | 139135 | 6 |
| 3NB692001538 | 0.084340651 | 122218 | 7 |
| 3NB692001548 | 0.012221951 | 96322 | 1 |
| 3NB692001557 | 0.027621147 | 91468 | 4 |
| 3NB692001637 | 0.036967383 | 168210 | 3 |
| 3NB692001719 | 0.024327187 | 94876 | 6 |
| 3NB692001853 | 0.063988222 | 34169 | 5 |
| 3NB692001861 | 0.012221951 | 193198 | 1 |
| 3NB692002051 | 0.019613515 | 178660 | 3 |
| 3NB692002365 | 0.012221951 | 4137 | 1 |
| 3NB692002685 | 0.012221951 | 217529 | 1 |
| 3NB692002806 | 0.012221951 | 154266 | 1 |
| 3NB692003538 | 0.012221951 | 208889 | 1 |
| 3NB692004045 | 0.012221951 | 284307 | 1 |
| 3NB692004724 | 0.012221951 | 196462 | 1 |
| 3NB692005439 | 0.012221951 | 185399 | 1 |
| 3NB692006952 | 0.028620836 | 68822 | 3 |
| 3NB692008178 | 0.013317229 | 46324 | 2 |
| 3NB692008729 | 0.012221951 | 135639 | 1 |
| ACTVT2000380 | 0.14619883 | 240854 | 1 |
| ADIPS1000064 | 0.16286645 | 41464 | 1 |
| ADIPS2000069 | 0.16286645 | 278077 | 1 |
| ADIPS2000088 | 0.325732899 | 273369 | 2 |
| ADIPS2000245 | 0.16286645 | 260321 | 1 |
| ADIPS2000425 | 0.215847182 | 124522 | 9 |
| ADRGL1000002 | 0.101756095 | 64755 | 44 |
| ADRGL1000018 | 0.034647887 | 46754 | 15 |
| ADRGL1000033 | 0.043883526 | 34724 | 5 |
| ADRGL1000038 | 0.009708738 | 66512 | 1 |
| ADRGL1000065 | 0.02971239 | 34887 | 11 |
| ADRGL1000067 | 0.009708738 | 8956 | 1 |
| ADRGL1000111 | 0.011105893 | 55238 | 2 |
| ADRGL1000144 | 0.05013397 | 52060 | 8 |
| ADRGL1000147 | 0.019417476 | 53719 | 2 |
| ADRGL1000160 | 0.077669903 | 10013 | 8 |
| ADRGL1000165 | 0.009708738 | 73605 | 1 |
| ADRGL1000182 | 0.009708738 | 65035 | 1 |
| ADRGL2000006 | 0.161178621 | 129178 | 41 |
| ADRGL2000042 | 0.065977455 | 94764 | 17 |
| ADRGL2000056 | 0.011105893 | 208582 | 2 |
| ADRGL2000064 | 0.009708738 | 184566 | 1 |
| ADRGL2000074 | 0.011397014 | 157554 | 2 |
| ADRGL2000085 | 0.009708738 | 184572 | 1 |
| ADRGL2000097 | 0.075366581 | 171199 | 10 |
| ADRGL2000117 | 0.019631485 | 114323 | 4 |
| ADRGL2000142 | 0.009708738 | 141891 | 1 |
| ADRGL2000172 | 0.009708738 | 182717 | 1 |
| ADRGL2000248 | 0.009708738 | 223982 | 1 |
| ADRGL2000261 | 0.009708738 | 207317 | 1 |
| ADRGL2000323 | 0.012623595 | 236322 | 2 |
| ADRGL2000328 | 0.024889644 | 175865 | 6 |
| ADRGL2000353 | 0.009708738 | 215172 | 1 |
| ADRGL2000384 | 0.067961165 | 12648 | 7 |
| ADRGL2000428 | 0.009708738 | 234148 | 1 |
| ADRGL2000636 | 0.009708738 | 75522 | 1 |
| ADRGL2000644 | 0.015763106 | 214709 | 2 |
| ADRGL2000968 | 0.009708738 | 196722 | 1 |
| ADRGL2001119 | 0.009708738 | 112417 | 1 |
| ADRGL2001229 | 0.009708738 | 215864 | 1 |
| ADRGL2001287 | 0.009708738 | 197023 | 1 |
| ADRGL2001301 | 0.009708738 | 241888 | 1 |
| ADRGL2001352 | 0.019587871 | 192434 | 4 |
| ADRGL2001354 | 0.009708738 | 223064 | 1 |
| ADRGL2001554 | 0.009708738 | 224065 | 1 |
| ADRGL2001651 | 0.009708738 | 222162 | 1 |
| ADRGL2001756 | 0.009708738 | 85903 | 1 |
| ADRGL2001830 | 0.014018789 | 65424 | 3 |
| ADRGL2001836 | 0.009708738 | 245756 | 1 |
| ADRGL2001854 | 0.009708738 | 243277 | 1 |
| ADRGL2002013 | 0.018625967 | 119719 | 7 |
| ADRGL2002029 | 0.009708738 | 78836 | 1 |
| ADRGL2002191 | 0.013106475 | 63260 | 3 |
| ADRGL2002260 | 0.011105893 | 188977 | 2 |
| ADRGL2002392 | 0.009708738 | 94613 | 1 |
| ADRGL2002477 | 0.098491284 | 97270 | 26 |
| ADRGL2002679 | 0.013465797 | 130441 | 3 |
| ADRGL2002753 | 0.019417476 | 231793 | 2 |
| ADRGL2002857 | 0.009708738 | 127516 | 1 |
| ADRGL2003017 | 0.009708738 | 46472 | 1 |
| ADRGL2003329 | 0.009708738 | 152935 | 1 |
| ADRGL2003552 | 0.009708738 | 283156 | 1 |
| ADRGL2003585 | 0.009708738 | 15181 | 1 |
| ADRGL2003638 | 0.009708738 | 134901 | 1 |
| ADRGL2003684 | 0.009708738 | 155321 | 1 |
| ADRGL2003773 | 0.009708738 | 223161 | 1 |
| ADRGL2003785 | 0.011418198 | 69967 | 2 |
| ADRGL2004031 | 0.009708738 | 164853 | 1 |
| ADRGL2004077 | 0.009708738 | 22835 | 1 |
| ADRGL2004451 | 0.019417476 | 72403 | 2 |
| ADRGL2004459 | 0.019417476 | 189693 | 2 |
| ADRGL2004676 | 0.038439333 | 112371 | 8 |
| ADRGL2004708 | 0.009708738 | 163374 | 1 |
| ADRGL2004724 | 0.009708738 | 174384 | 1 |
| ADRGL2004749 | 0.009708738 | 192682 | 1 |
| ADRGL2004770 | 0.009708738 | 200652 | 1 |
| ADRGL2004777 | 0.009708738 | 131595 | 1 |
| ADRGL2004833 | 0.009708738 | 80679 | 1 |
| ADRGL2005336 | 0.009708738 | 17560 | 1 |
| ADRGL2005564 | 0.009708738 | 24035 | 1 |
| ADRGL2005756 | 0.009708738 | 187567 | 1 |
| ADRGL2005838 | 0.015854264 | 5613 | 2 |
| ADRGL2005926 | 0.038834951 | 145927 | 4 |
| ADRGL2005961 | 0.009708738 | 3196 | 1 |
| ADRGL2006034 | 0.009708738 | 101275 | 1 |
| ADRGL2006124 | 0.009708738 | 32168 | 1 |
| ADRGL2006177 | 0.009708738 | 149214 | 1 |
| ADRGL2006193 | 0.009708738 | 146749 | 1 |
| ADRGL2006233 | 0.016534781 | 47690 | 3 |
| ADRGL2006377 | 0.009708738 | 77853 | 1 |
| ADRGL2006677 | 0.009708738 | 186902 | 1 |
| ADRGL2006732 | 0.009708738 | 101903 | 1 |
| ADRGL2006767 | 0.009708738 | 275340 | 1 |
| ADRGL2006817 | 0.009708738 | 213653 | 1 |
| ADRGL2006846 | 0.009708738 | 187510 | 1 |
| ADRGL2006886 | 0.009708738 | 176480 | 1 |
| ADRGL2007080 | 0.009708738 | 190702 | 1 |
| ADRGL2007125 | 0.009708738 | 45966 | 1 |
| ADRGL2007283 | 0.009708738 | 167805 | 1 |
| ADRGL2007313 | 0.017100302 | 168347 | 3 |
| ADRGL2007357 | 0.009708738 | 186500 | 1 |
| ADRGL2007636 | 0.009708738 | 155823 | 1 |
| ADRGL2007651 | 0.009708738 | 144684 | 1 |
| ADRGL2007810 | 0.009708738 | 99151 | 1 |
| ADRGL2007877 | 0.009708738 | 197625 | 1 |
| ADRGL2007906 | 0.018592193 | 81944 | 5 |
| ADRGL2007974 | 0.011397014 | 174372 | 2 |
| ADRGL2008023 | 0.009708738 | 122769 | 1 |
| ADRGL2008331 | 0.009708738 | 223423 | 1 |
| ADRGL2008337 | 0.009708738 | 261136 | 1 |
| ADRGL2008535 | 0.011105893 | 189976 | 2 |
| ADRGL2008966 | 0.011397014 | 148609 | 2 |
| ADRGL2009146 | 0.010897982 | 200721 | 2 |
| ADRGL2009273 | 0.120619855 | 138546 | 30 |
| ADRGL2009324 | 0.009708738 | 195085 | 1 |
| ADRGL2009489 | 0.009708738 | 149020 | 1 |
| ADRGL2009533 | 0.009708738 | 42576 | 1 |
| ADRGL2009691 | 0.009708738 | 89392 | 1 |
| ADRGL2009755 | 0.009708738 | 30025 | 1 |
| ADRGL2010152 | 0.02892579 | 15777 | 5 |
| ADRGL2010315 | 0.009708738 | 199715 | 1 |
| ADRGL2010594 | 0.009708738 | 242848 | 1 |
| ADRGL2010860 | 0.009708738 | 235358 | 1 |
| ADRGL2010974 | 0.009708738 | 202322 | 1 |
| ADRGL2011190 | 0.009708738 | 98377 | 1 |
| ADRGL2011330 | 0.011105893 | 203020 | 2 |
| ADRGL2012038 | 0.081507962 | 90763 | 5 |
| ADRGL2012179 | 0.01179025 | 104156 | 2 |
| AHMSC1000138 | 0.149031297 | 255416 | 1 |
| ASTRO1000009 | 0.064170068 | 77684 | 3 |
| ASTRO1000018 | 0.005805178 | 111744 | 1 |
| ASTRO1000029 | 0.005805178 | 31508 | 1 |
| ASTRO1000096 | 0.016632376 | 110634 | 2 |
| ASTRO1000165 | 0.075597651 | 69043 | 13 |
| ASTRO2000011 | 0.005805178 | 260808 | 1 |
| ASTRO2000014 | 0.408013258 | 131485 | 22 |
| ASTRO2000046 | 0.410629945 | 81809 | 138 |
| ASTRO2000095 | 0.005805178 | 228929 | 1 |
| ASTRO2000141 | 0.11032761 | 28048 | 10 |
| ASTRO2000191 | 0.005805178 | 205634 | 1 |
| ASTRO2000278 | 0.008599489 | 146123 | 3 |
| ASTRO2000372 | 0.007514638 | 33799 | 2 |
| ASTRO2000381 | 0.02668074 | 125948 | 6 |
| ASTRO2000403 | 0.005805178 | 235134 | 1 |
| ASTRO2000417 | 0.005805178 | 32092 | 1 |
| ASTRO2000480 | 0.059182171 | 97447 | 15 |
| ASTRO2000482 | 0.007480725 | 103504 | 2 |
| ASTRO2000533 | 0.005805178 | 109282 | 1 |
| ASTRO2000653 | 0.005805178 | 77531 | 1 |
| ASTRO2000662 | 0.02077397 | 164888 | 3 |
| ASTRO2000725 | 0.005805178 | 224690 | 1 |
| ASTRO2000801 | 0.164247184 | 18747 | 6 |
| ASTRO2000914 | 0.086079069 | 36093 | 9 |
| ASTRO2001001 | 0.005805178 | 137036 | 1 |
| ASTRO2001008 | 0.005805178 | 135634 | 1 |
| ASTRO2001029 | 0.133620252 | 162823 | 25 |
| ASTRO2001076 | 0.117885646 | 168112 | 17 |
| ASTRO2001088 | 0.005805178 | 168622 | 1 |
| ASTRO2001107 | 0.022938731 | 113427 | 11 |
| ASTRO2001122 | 0.005805178 | 139390 | 1 |
| ASTRO2001227 | 0.005805178 | 194451 | 1 |
| ASTRO2001249 | 0.017611554 | 142448 | 2 |
| ASTRO2001458 | 0.055081272 | 130929 | 13 |
| ASTRO2001480 | 0.005805178 | 273731 | 1 |
| ASTRO2001806 | 0.117118444 | 51065 | 21 |
| ASTRO2001823 | 0.115432411 | 148627 | 22 |
| ASTRO2002024 | 0.012780199 | 136960 | 6 |
| ASTRO2002035 | 0.015437208 | 14714 | 4 |
| ASTRO2002064 | 0.012007498 | 161710 | 2 |
| ASTRO2002202 | 0.072708957 | 107156 | 11 |
| ASTRO2002459 | 0.191410161 | 160202 | 7 |
| ASTRO2002571 | 0.005805178 | 153931 | 1 |
| ASTRO2002632 | 0.005805178 | 184466 | 1 |
| ASTRO2002659 | 0.011610356 | 128564 | 2 |
| ASTRO2002693 | 0.023638116 | 58270 | 3 |
| ASTRO2002720 | 0.006900456 | 45986 | 2 |
| ASTRO2002733 | 0.010891192 | 52101 | 4 |
| ASTRO2002743 | 0.005805178 | 152270 | 1 |
| ASTRO2002842 | 0.017592071 | 190999 | 2 |
| ASTRO2003024 | 0.176782467 | 101801 | 29 |
| ASTRO2003212 | 0.011185197 | 183619 | 4 |
| ASTRO2003316 | 0.056801159 | 119269 | 19 |
| ASTRO2003461 | 0.028128472 | 97123 | 10 |
| ASTRO2003574 | 0.011230867 | 93166 | 4 |
| ASTRO2003581 | 0.005805178 | 152157 | 1 |
| ASTRO2003632 | 0.403324214 | 58701 | 38 |
| ASTRO2003740 | 0.009649262 | 146059 | 2 |
| ASTRO2003840 | 0.027050092 | 133906 | 8 |
| ASTRO2003925 | 0.007803579 | 74962 | 2 |
| ASTRO2003960 | 0.006994423 | 206956 | 2 |
| ASTRO2004032 | 0.027492808 | 76671 | 8 |
| ASTRO2004051 | 0.005805178 | 115862 | 1 |
| ASTRO2004177 | 0.006994423 | 174394 | 2 |
| ASTRO2004217 | 0.005805178 | 200913 | 1 |
| ASTRO2004297 | 0.005805178 | 118848 | 1 |
| ASTRO2004584 | 0.005805178 | 576 | 1 |
| ASTRO2004628 | 0.018824826 | 147998 | 7 |
| ASTRO2004751 | 0.005805178 | 210722 | 1 |
| ASTRO2004877 | 0.005805178 | 128553 | 1 |
| ASTRO2004974 | 0.086235482 | 104154 | 13 |
| ASTRO2005008 | 0.005805178 | 104924 | 1 |
| ASTRO2005081 | 0.005805178 | 145748 | 1 |
| ASTRO2005096 | 0.005805178 | 198881 | 1 |
| ASTRO2005242 | 0.018149557 | 123226 | 5 |
| ASTRO2005273 | 0.005805178 | 168450 | 1 |
| ASTRO2005343 | 0.005805178 | 77687 | 1 |
| ASTRO2005360 | 0.028086918 | 149445 | 8 |
| ASTRO2005413 | 0.005805178 | 281753 | 1 |
| ASTRO2005423 | 0.048662157 | 122393 | 22 |
| ASTRO2005553 | 0.064661953 | 106837 | 7 |
| ASTRO2005557 | 0.005805178 | 20738 | 1 |
| ASTRO2005575 | 0.005805178 | 37609 | 1 |
| ASTRO2005593 | 0.012007498 | 90378 | 2 |
| ASTRO2005687 | 0.150323004 | 93161 | 20 |
| ASTRO2005726 | 0.005805178 | 168471 | 1 |
| ASTRO2005863 | 0.044075179 | 110378 | 14 |
| ASTRO2005896 | 0.018866829 | 189722 | 2 |
| ASTRO2005902 | 0.005805178 | 104669 | 1 |
| ASTRO2006356 | 0.005805178 | 187764 | 1 |
| ASTRO2006475 | 0.022496365 | 133653 | 5 |
| ASTRO2006732 | 0.010924386 | 144077 | 3 |
| ASTRO2006747 | 0.005805178 | 214900 | 1 |
| ASTRO2006920 | 0.027928558 | 206896 | 5 |
| ASTRO2006952 | 0.005805178 | 190210 | 1 |
| ASTRO2007047 | 0.080176463 | 113453 | 14 |
| ASTRO2007221 | 0.008925985 | 143785 | 2 |
| ASTRO2007377 | 0.005805178 | 165596 | 1 |
| ASTRO2007515 | 0.005805178 | 215382 | 1 |
| ASTRO2007578 | 0.005805178 | 165593 | 1 |
| ASTRO2007643 | 0.005805178 | 224456 | 1 |
| ASTRO2007666 | 0.005805178 | 217428 | 1 |
| ASTRO2007948 | 0.066903155 | 139726 | 18 |
| ASTRO2007956 | 0.005805178 | 74035 | 1 |
| ASTRO2007962 | 0.005805178 | 156422 | 1 |
| ASTRO2008040 | 0.013986539 | 149402 | 5 |
| ASTRO2008216 | 0.052509128 | 88347 | 10 |
| ASTRO2008239 | 0.005805178 | 217321 | 1 |
| ASTRO2008409 | 0.012888343 | 127528 | 3 |
| ASTRO2008425 | 0.005805178 | 150223 | 1 |
| ASTRO2008508 | 0.018630777 | 138166 | 3 |
| ASTRO2008777 | 0.005805178 | 141331 | 1 |
| ASTRO2008895 | 0.005805178 | 46303 | 1 |
| ASTRO2008960 | 0.005805178 | 177732 | 1 |
| ASTRO2008972 | 0.010758789 | 160000 | 3 |
| ASTRO2009068 | 0.02961118 | 128613 | 7 |
| ASTRO2009118 | 0.010726901 | 149916 | 3 |
| ASTRO2009177 | 0.005805178 | 190477 | 1 |
| ASTRO2009333 | 0.005805178 | 75248 | 1 |
| ASTRO2009458 | 0.005805178 | 159002 | 1 |
| ASTRO2009526 | 0.005805178 | 116040 | 1 |
| ASTRO2009879 | 0.005805178 | 280536 | 1 |
| ASTRO2010072 | 0.009649262 | 145536 | 2 |
| ASTRO2010582 | 0.009680421 | 81044 | 3 |
| ASTRO2010615 | 0.005805178 | 125076 | 1 |
| ASTRO2010799 | 0.011740953 | 63502 | 2 |
| ASTRO2010819 | 0.249018954 | 17947 | 96 |
| ASTRO2010962 | 0.005805178 | 206950 | 1 |
| ASTRO2010996 | 0.00885006 | 164323 | 2 |
| ASTRO2011149 | 0.005805178 | 161025 | 1 |
| ASTRO2011422 | 0.023096182 | 101511 | 8 |
| ASTRO2011426 | 0.005805178 | 241244 | 1 |
| ASTRO2011437 | 0.005805178 | 60299 | 1 |
| ASTRO2011461 | 0.005805178 | 253416 | 1 |
| ASTRO2011834 | 0.005805178 | 130341 | 1 |
| ASTRO2011893 | 0.005805178 | 190191 | 1 |
| ASTRO2012552 | 0.113357236 | 70566 | 17 |
| ASTRO2013050 | 0.065204083 | 112505 | 24 |
| ASTRO2013124 | 0.005805178 | 147155 | 1 |
| ASTRO2013585 | 0.343711979 | 31391 | 53 |
| ASTRO2013671 | 0.007202334 | 46333 | 2 |
| ASTRO2013802 | 0.005805178 | 168328 | 1 |
| ASTRO2014135 | 0.048237303 | 45846 | 3 |
| ASTRO2014174 | 0.007514638 | 135847 | 2 |
| ASTRO2014211 | 0.005805178 | 255616 | 1 |
| ASTRO2014363 | 0.005805178 | 75297 | 1 |
| ASTRO2014392 | 0.011337539 | 167372 | 3 |
| ASTRO2014498 | 0.009372912 | 129794 | 4 |
| ASTRO2014561 | 0.019012865 | 58366 | 3 |
| ASTRO2014576 | 0.014825091 | 115245 | 4 |
| ASTRO2014863 | 0.04959715 | 53993 | 9 |
| ASTRO2014923 | 0.005805178 | 166494 | 1 |
| ASTRO2014961 | 0.005805178 | 157854 | 1 |
| ASTRO2015162 | 0.005805178 | 20020 | 1 |
| ASTRO2015185 | 0.005805178 | 95607 | 1 |
| ASTRO2015214 | 0.057020452 | 185848 | 4 |
| ASTRO2015295 | 0.327013605 | 137956 | 20 |
| ASTRO2015328 | 0.044027039 | 113878 | 11 |
| ASTRO2015529 | 0.090436131 | 165708 | 13 |
| ASTRO2015987 | 0.063798417 | 36693 | 9 |
| ASTRO2016044 | 0.005805178 | 146106 | 1 |
| ASTRO2016114 | 0.011557963 | 18679 | 4 |
| ASTRO2016148 | 0.00788669 | 106373 | 2 |
| ASTRO2016313 | 0.050855316 | 164367 | 12 |
| ASTRO2016491 | 0.33272971 | 33255 | 78 |
| ASTRO2016681 | 0.005805178 | 236880 | 1 |
| ASTRO2016819 | 0.025966357 | 59150 | 6 |
| ASTRO2016847 | 0.005805178 | 156242 | 1 |
| ASTRO2016892 | 0.005805178 | 50889 | 1 |
| ASTRO2017348 | 0.005805178 | 241546 | 1 |
| ASTRO2017627 | 0.005805178 | 163656 | 1 |
| ASTRO2018169 | 0.177397925 | 117098 | 12 |
| ASTRO2018373 | 0.012007498 | 163438 | 2 |
| ASTRO2018540 | 0.107746655 | 28370 | 9 |
| ASTRO2019039 | 0.005805178 | 183074. | 1 |
| ASTRO2019171 | 0.009372912 | 130262 | 4 |
| ASTRO3000154 | 0.005805178 | 270258 | 1 |
| ASTRO3000172 | 0.005805178 | 257814 | 1 |
| ASTRO3000177 | 0.005805178 | 247423 | 1 |
| ASTRO3000301 | 0.005805178 | 266756 | 1 |
| ASTRO3000474 | 0.005805178 | 252434 | 1 |
| ASTRO3000482 | 0.007663293 | 260423 | 2 |
| BEAST2000454 | 0.036549708 | 29585 | 1 |
| BGGI11000123 | 0.239085915 | 62672 | 18 |
| BGGI11000193 | 0.0561349 | 78986 | 2 |
| BGGI11000285 | 0.052521008 | 35193 | 1 |
| BGGI12000022 | 0.052521008 | 263509 | 1 |
| BGGI12000054 | 0.711429859 | 113109 | 92 |
| BGGI12000067 | 0.052521008 | 73635 | 1 |
| BGGI12000161 | 0.052521008 | 23642 | 1 |
| BGGI12000533 | 0.074219504 | 8036 | 5 |
| BGGI12000544 | 0.052521008 | 120857 | 1 |
| BGGI12000616 | 0.187405138 | 102543 | 15 |
| BGGI12000684 | 0.067914618 | 51818 | 3 |
| BGGI12000693 | 0.088766025 | 145987 | 2 |
| BGGI12000839 | 0.227220843 | 27656 | 23 |
| BGGI12001002 | 0.094386136 | 131716 | 8 |
| BGGI12001075 | 0.08464501 | 17107 | 12 |
| BGGI12001097 | 0.149100363 | 142097 | 13 |
| BGGI12001241 | 0.052521008 | 184267 | 1 |
| BGGI12001247 | 0.052521008 | 150339 | 1 |
| BGGI12001682 | 0.162245467 | 139773 | 32 |
| BGGI12001714 | 0.065701387 | 38725 | 3 |
| BLADE1000176 | 0.014139044 | 51068 | 3 |
| BLADE2000181 | 0.011760555 | 185326 | 1 |
| BLADE2000256 | 0.014675412 | 12223 | 2 |
| BLADE2000340 | 0.011760555 | 130145 | 1 |
| BLADE2000389 | 0.011760555 | 108104 | 1 |
| BLADE2000463 | 0.011760555 | 56291 | 1 |
| BLADE2000492 | 0.047744722 | 136666 | 2 |
| BLADE2000579 | 0.011760555 | 121609 | 1 |
| BLADE2000640 | 0.011760555 | 280554 | 1 |
| BLADE2000866 | 0.011760555 | 234026 | 1 |
| BLADE2000938 | 0.011760555 | 192101 | 1 |
| BLADE2001031 | 0.011760555 | 257809 | 1 |
| BLADE2001133 | 0.011760555 | 245054 | 1 |
| BLADE2001138 | 0.011760555 | 177041 | 1 |
| BLADE2001141 | 0.011760555 | 76703 | 1 |
| BLADE2001371 | 0.013470015 | 52824 | 2 |
| BLADE2001458 | 0.011760555 | 177047 | 1 |
| BLADE2001575 | 0.017673167 | 183403 | 2 |
| BLADE2001753 | 0.011760555 | 205923 | 1 |
| BLADE2001935 | 0.011760555 | 248845 | 1 |
| BLADE2001940 | 0.011760555 | 238112 | 1 |
| BLADE2001987 | 0.011760555 | 188797 | 1 |
| BLADE2002073 | 0.011760555 | 153257 | 1 |
| BLADE2002310 | 0.011760555 | 238186 | 1 |
| BLADE2002730 | 0.012855833 | 211742 | 2 |
| BLADE2002738 | 0.013618669 | 202912 | 2 |
| BLADE2002744 | 0.068649476 | 194471 | 3 |
| BLADE2002782 | 0.011760555 | 273692 | 1 |
| BLADE2002947 | 0.011760555 | 276859 | 1 |
| BLADE2002972 | 0.011760555 | 50579 | 1 |
| BLADE2003474 | 0.011760555 | 182842 | 1 |
| BLADE2003916 | 0.011760555 | 276589 | 1 |
| BLADE2004089 | 0.016908153 | 73797 | 4 |
| BLADE2004345 | 0.011760555 | 278121 | 1 |
| BLADE2004389 | 0.011760555 | 209756 | 1 |
| BLADE2004462 | 0.011760555 | 155020 | 1 |
| BLADE2004628 | 0.011760555 | 188402 | 1 |
| BLADE2004670 | 0.011760555 | 218339 | 1 |
| BLADE2004722 | 0.016740916 | 114161 | 3 |
| BLADE2004849 | 0.022605909 | 85424 | 4 |
| BLADE2005036 | 0.011760555 | 170294 | 1 |
| BLADE2005038 | 0.011760555 | 278268 | 1 |
| BLADE2005229 | 0.011760555 | 168257 | 1 |
| BLADE2005459 | 0.011760555 | 172713 | 1 |
| BLADE2005792 | 0.011760555 | 192229 | 1 |
| BLADE2006043 | 0.011760555 | 161191 | 1 |
| BLADE2006412 | 0.027596903 | 160606 | 9 |
| BLADE2006415 | 0.011760555 | 184028 | 1 |
| BLADE2006607 | 0.011760555 | 241968 | 1 |
| BLADE2006826 | 0.011760555 | 233655 | 1 |
| BLADE2006830 | 0.099676546 | 87087 | 10 |
| BLADE2006849 | 0.011760555 | 277868 | 1 |
| BLADE2006947 | 0.02352111 | 148312 | 2 |
| BLADE2007033 | 0.011760555 | 273120 | 1 |
| BLADE2007043 | 0.013436102 | 149106 | 2 |
| BLADE2007589 | 0.033894588 | 45920 | 9 |
| BLADE2007666 | 0.049180905 | 212513 | 4 |
| BLADE2007722 | 0.011760555 | 212999 | 1 |
| BLADE2007735 | 0.011760555 | 231094 | 1 |
| BLADE2007744 | 0.011760555 | 235449 | 1 |
| BLADE2007799 | 0.011760555 | 270841 | 1 |
| BLADE2007923 | 0.011760555 | 160628 | 1 |
| BLADE2007935 | 0.013470015 | 226601 | 2 |
| BLADE2007958 | 0.011760555 | 149323 | 1 |
| BLADE2008281 | 0.011760555 | 197770 | 1 |
| BLADE2008398 | 0.027022345 | 110886 | 10 |
| BLADE2008454 | 0.011760555 | 279749 | 1 |
| BLADE2008461 | 0.067592263 | 20287 | 7 |
| BLADE2008479 | 0.011760555 | 210912 | 1 |
| BLADE2008539 | 0.011760555 | 162493 | 1 |
| BLADE2008809 | 0.011760555 | 192230 | 1 |
| BLADE2008995 | 0.011760555 | 141691 | 1 |
| BLADE2009452 | 0.011760555 | 198602 | 1 |
| BLADE2009479 | 0.011760555 | 211142 | 1 |
| BLADE2009593 | 0.011760555 | 265180 | 1 |
| BNGH41000098 | 0.066102669 | 52545 | 19 |
| BNGH41000104 | 0.022376043 | 48987 | 2 |
| BNGH41000118 | 0.012921566 | 29364 | 1 |
| BNGH41000137 | 0.038618962 | 76227 | 15 |
| BNGH41000153 | 0.035657499 | 54613 | 7 |
| BNGH41000169 | 0.012921566 | 66533 | 1 |
| BNGH41000177 | 0.024099746 | 8028 | 2 |
| BNGH41000190 | 0.066215467 | 66513 | 18 |
| BNGH41000198 | 0.080650037 | 72493 | 16 |
| BNGH41000216 | 0.012921566 | 87914 | 1 |
| BNGH41000235 | 0.025707724 | 10945 | 8 |
| BNGH42000130 | 0.15998039 | 123106 | 2 |
| BNGH42000132 | 0.012921566 | 194610 | 1 |
| BNGH42000360 | 1.065255609 | 56156 | 47 |
| BNGH42000405 | 0.012921566 | 203887 | 1 |
| BNGH42000532 | 0.01771792 | 97272 | 3 |
| BNGH42000675 | 0.012921566 | 35195 | 1 |
| BNGH42000739 | 0.269301405 | 83698 | 129 |
| BNGH42000791 | 0.012921566 | 261436 | 1 |
| BNGH42000815 | 0.019134676 | 87815 | 2 |
| BNGH42000994 | 0.012921566 | 135858 | 1 |
| BNGH42001247 | 0.03184986 | 78838 | 5 |
| BNGH42001406 | 0.024385512 | 260626 | 2 |
| BNGH42001576 | 0.034339999 | 189891 | 7 |
| BNGH42001724 | 0.012921566 | 39677 | 1 |
| BNGH42002168 | 0.125933709 | 68433 | 13 |
| BNGH42002244 | 0.012921566 | 146543 | 1 |
| BNGH42002387 | 0.015874959 | 99741 | 3 |
| BNGH42002487 | 0.017901927 | 123500 | 3 |
| BNGH42002489 | 0.012921566 | 245750 | 1 |
| BNGH42003529 | 0.012921566 | 151167 | 1 |
| BNGH42003570 | 0.012921566 | 239401 | 1 |
| BNGH42003641 | 0.012921566 | 236561 | 1 |
| BNGH42004076 | 0.012921566 | 223271 | 1 |
| BNGH42004291 | 0.012921566 | 92653 | 1 |
| BNGH42004538 | 0.012921566 | 171161 | 1 |
| BNGH42004679 | 0.014016844 | 35257 | 2 |
| BNGH42005017 | 0.019134676 | 159594 | 2 |
| BNGH42005235 | 0.012921566 | 165878 | 1 |
| BNGH42005504 | 0.012921566 | 34753 | 1 |
| BNGH42005968 | 0.072950516 | 131341 | 13 |
| BNGH42006135 | 0.253789888 | 84562 | 87 |
| BNGH42006226 | 0.014609843 | 134037 | 2 |
| BNGH42006234 | 0.012921566 | 139293 | 1 |
| BNGH42007037 | 0.02693841 | 109167 | 3 |
| BNGH42007366 | 0.026200916 | 125945 | 3 |
| BNGH42007460 | 0.166281879 | 99772 | 37 |
| BNGH42007594 | 0.012921566 | 250143 | 1 |
| BNGH42007709 | 0.727928246 | 66128 | 75 |
| BNGH42007788 | 0.054428793 | 120895 | 11 |
| BNGH42007798 | 0.017061726 | 100817 | 3 |
| BNGH42008199 | 0.012921566 | 226838 | 1 |
| BNGH42008510 | 0.012921566 | 161476 | 1 |
| BNGH42008603 | 0.11584507 | 133073 | 35 |
| BNGH42008649 | 0.012921566 | 278044 | 1 |
| BNGH42008743 | 0.10884126 | 126361 | 14 |
| BNGH42008832 | 0.012921566 | 33390 | 1 |
| BNGH42008850 | 0.015903525 | 160114 | 2 |
| BNGH42008943 | 0.020165179 | 29169 | 3 |
| BNGH42009021 | 0.012921566 | 129251 | 1 |
| BNGH42009025 | 0.120186589 | 150229 | 17 |
| BRACE1000020 | 0.014886212 | 54636 | 5 |
| BRACE1000042 | 0.110072803 | 53847 | 30 |
| BRACE1000047 | 0.001189244 | 64934 | 1 |
| BRACE1000051 | 0.009347382 | 75149 | 6 |
| BRACE1000065 | 0.004236603 | 52286 | 3 |
| BRACE1000070 | 0.093726205 | 39812 | 17 |
| BRACE1000073 | 0.0397401 | 49943 | 9 |
| BRACE100084 | 0.001189244 | 52494 | 1 |
| BRACE1000087 | 0.01141852 | 73907 | 4 |
| BRACE1000092 | 0.001189244 | 66811 | 1 |
| BRACE100093 | 0.004284741 | 36501 | 2 |
| BRACE1000101 | 0.014973455 | 73673 | 4 |
| BRACE1000114 | 0.001189244 | 69287 | 1 |
| BRACE1000115 | 0.895124541 | 5165 | 65 |
| BRACE1000124 | 0.001189244 | 64539 | 1 |
| BRACE1000131 | 0.16086513 | 62338 | 32 |
| BRACE 1 000132 | 0.011664658 | 76367 | 4 |
| BRACE1000159 | 0.090748029 | 52820 | 15 |
| BRACE1000166 | 0.0025864 | 87629 | 2 |
| BRACE1000169 | 0.008264884 | 41745 | 4 |
| BRACE1000186 | 0.077496146 | 73042 | 14 |
| BRACE1000187 | 0.062081235 | 57824 | 29 |
| BRACE1000239 | 0.008455944 | 143594 | 4 |
| BRACE1000258 | 0.00524328 | 88300 | 4 |
| BRACE1000451 | 0.001189244 | 251199 | 1 |
| BRACE1000475 | 0.001189244 | 258814 | 1 |
| BRACE1000533 | 0.071027019 | 89830 | 30 |
| BRACE1000572 | 0.001189244 | 278488 | 1 |
| BRACE2000061. | 0.004274676 | 172422 | 3 |
| BRACE2000077 | 0.003473767 | 158305 | 3 |
| BRACE2000078 | 0.037434261 | 199344 | 2 |
| BRACE2000100 | 0.001189244 | 215188 | 1 |
| BRACE2000109 | 0.003047359 | 166740 | 2 |
| BRACE2000141 | 0.009647536 | 85923 | 6 |
| BRACE2000148 | 0.046934018 | 190688 | 3 |
| BRACE2000183 | 0.036549923 | 167435 | 2 |
| BRACE2000229 | 0.012976137 | 130666 | 2 |
| BRACE2000232 | 0.001189244 | 204443 | 1 |
| BRACE2000245 | 0.018945752 | 97358 | 9 |
| BRACE2000256 | 0.001189244 | 207459 | 1 |
| BRACE2000266 | 0.001189244 | 251079 | 1 |
| BRACE2000267 | 0.012536959 | 102199 | 5 |
| BRACE2000280 | 0.008940271 | 112431 | 3 |
| BRACE2000300 | 0.031521611 | 95671 | 11 |
| BRACE2000307 | 0.142252985 | 132485 | 49 |
| BRACE2000331 | 0.002864792 | 18972 | 2 |
| BRACE2000332 | 0.061728861 | 126335 | 12 |
| BRACE2000347 | 0.064814986 | 182381 | 17 |
| BRACE2000351 | 0.001189244 | 281897 | 1 |
| BRACE2000368 | 0.002378489 | 206430 | 2 |
| BRACE2000392 | 0.001189244 | 214545 | 1 |
| BRACE2000421 | 0.043236157 | 82742 | 25 |
| BRACE2000441 | 0.001189244 | 146945 | 1 |
| BRACE2000487 | 0.020419338 | 165327 | 4 |
| BRACE2000489 | 0.001189244 | 64699 | 1 |
| BRACE2000505 | 0.013712726 | 184139 | 2 |
| BRACE2000525 | 0.103126365 | 93675 | 35 |
| BRACE2000526 | 0.001189244 | 216121 | 1 |
| BRACE2000531 | 0.001189244 | 142381 | 1 |
| BRACE2000545 | 0.001189244 | 106138 | 1 |
| BRACE2000553 | 0.001189244 | 91501 | 1 |
| BRACE2000565 | 0.023362848 | 153227 | 8 |
| BRACE2000577 | 0.002284523 | 71681 | 2 |
| BRACE2000640 | 0.001189244 | 270201 | 1 |
| BRACE2000650 | 0.001189244 | 238334 | 1 |
| BRACE2000676 | 0.155624815 | 48302 | 44 |
| BRACE2000677 | 0.001189244 | 266766 | 1 |
| BRACE2000698 | 0.019473747 | 144048 | 3 |
| BRACE2000718 | 0.001189244 | 208548 | 1 |
| BRACE2000733 | 0.009507114 | 176333 | 3 |
| BRACE2000743 | 0.001189244 | 249263 | 1 |
| BRACE2000750 | 0.008141256 | 57101 | 4 |
| BRACE2000753 | 0.005501257 | 154521 | 3 |
| BRACE2000815 | 0.001189244 | 233427 | 1 |
| BRACE2000864 | 0.31749202 | 137308 | 41 |
| BRACE2000885 | 0.002284523 | 127717 | 2 |
| BRACE2000893 | 0.037076472 | 150848 | 17 |
| BRACE2000905 | 0.001189244 | 227967 | 1 |
| BRACE2000936 | 0.001189244 | 273068 | 1 |
| BRACE2000947 | 0.001189244 | 237436 | 1 |
| BRACE2000965 | 0.001189244 | 158015 | 1 |
| BRACE2000969 | 0.007334771 | 149537 | 2 |
| BRACE2000975 | 0.261569543 | 75529 | 133 |
| BRACE2000988 | 0.001189244 | 279611 | 1 |
| BRACE2001017 | 0.001189244 | 247496 | 1 |
| BRACE2001065 | 0.025233658 | 88814 | 7 |
| BRACE2001070 | 0.007425602 | 100127 | 2 |
| BRACE2001094 | 0.001189244 | 281360 | 1 |
| BRACE2001107 | 0.011383773 | 230024 | 4 |
| BRACE2001117 | 0.060920807 | 55893 | 10 |
| BRACE2001143 | 0.112794231 | 98802 | 15 |
| BRACE2001151 | 0.025957605 | 49097 | 5 |
| BRACE2001154 | 0.015770393 | 72571 | 6 |
| BRACE2001188 | 0.059357034 | 94051 | 6 |
| BRACE2001191 | 0.001189244 | 273710 | 1 |
| BRACE2001197 | 0.002898705 | 44154 | 2 |
| BRACE2001236 | 0.009312322 | 181678 | 3 |
| BRACE2001239 | 0.001189244 | 227737 | 1 |
| BRACE2001269 | 0.001189244 | 230267 | 1 |
| BRACE2001312 | 0.002378489 | 212099 | 2 |
| BRACE2001313 | 0.005501257 | 166260 | 3 |
| BRACE2001330 | 0.001189244 | 41898 | 1 |
| BRACE2001340 | 0.001189244 | 134905 | 1 |
| BRACE2001352 | 0.001189244 | 185672 | 1 |
| BRACE2001353 | 0.001189244 | 197676 | 1 |
| BRACE2001374 | 0.002877521 | 109292 | 2 |
| BRACE2001375 | 0.001189244 | 192284 | 1 |
| BRACE2001423 | 0.001189244 | 189404 | 1 |
| BRACE2001424 | 0.001189244 | 29307 | 1 |
| BRACE2001445 | 0.001189244 | 20354 | 1 |
| BRACE2001453 | 0.001189244 | 220137 | 1 |
| BRACE2001455 | 0.001189244 | 30719 | 1 |
| BRACE2001477 | 0.048719392 | 115129 | 20 |
| BRACE2001478 | 0.094622909 | 106856 | 32 |
| BRACE2001492 | 0.001189244 | 95633 | 1 |
| BRACE2001496 | 0.034537152 | 11508 | 8 |
| BRACE2001497 | 0.089919331 | 130162 | 27 |
| BRACE2001508 | 0.01901123 | 220066 | 4 |
| BRACE2001534 | 0.054321394 | 173923 | 6 |
| BRACE2001543 | 0.001189244 | 244558 | 1 |
| BRACE2001564 | 0.003473767 | 111429 | 3 |
| BRACE2001588 | 0.001189244 | 232704 | 1 |
| BRACE2001607 | 0.001189244 | 200443 | 1 |
| BRACE2001626 | 0.001189244 | 245180 | 1 |
| BRACE2001673 | 0.714683906 | 90925 | 23 |
| BRACE2001677 | 0.001189244 | 235166 | 1 |
| BRACE2001692 | 0.276719989 | 87056 | 17 |
| BRACE2001705 | 0.001189244 | 247141 | 1 |
| BRACE2001709 | 0.05162969 | 96776 | 14 |
| BRACE2001711 | 0.043073912 | 41456 | 7 |
| BRACE2001726 | 0.004735635 | 64631 | 3 |
| BRACE2001733 | 0.001189244 | 247168 | 1 |
| BRACE2001737 | 0.001189244 | 229602 | 1 |
| BRACE2001742 | 0.002378489 | 223664 | 2 |
| BRACE2001777 | 0.001189244 | 243124 | 1 |
| BRACE2001779 | 0.001189244 | 239647 | 1 |
| BRACE2001785 | 0.162822998 | 105745 | 26 |
| BRACE2001834 | 0.044147361 | 108730 | 17 |
| BRACE2001855 | 0.145620135 | 74488 | 15 |
| BRACE2001859 | 0.012313952 | 69588 | 2 |
| BRACE2001865 | 0.001189244 | 159266 | 1 |
| BRACE2001872 | 0.001189244 | 235994 | 1 |
| BRACE2001881 | 0.007261627 | 174173 | 2 |
| BRACE2001892 | 0.553136526 | 79977 | 240 |
| BRACE2001898 | 0.001189244 | 245875 | 1 |
| BRACE2001923 | 0.001189244 | 90201 | 1 |
| BRACE2001942 | 0.0025864 | 165068 | 2 |
| BRACE2001944 | 0.026710106 | 152439 | 8 |
| BRACE2001954 | 0.001189244 | 155522 | 1 |
| BRACE2001971 | 0.074703155 | 15916 | 17 |
| BRACE2001972 | 0.001189244 | 170824 | 1 |
| BRACE2001973 | 0.005946222 | 208320 | 5 |
| BRACE2002026 | 0.001189244 | 221386 | 1 |
| BRACE2002034 | 0.001189244 | 220242 | 1 |
| BRACE2002050 | 0.057781435 | 128655 | 26 |
| BRACE2002081 | 0.008731926 | 180908 | 3 |
| BRACE2002091 | 0.079344087 | 128918 | 19 |
| BRACE2002139 | 0.001189244 | 114525 | 1 |
| BRACE2002142 | 0.001189244 | 150126 | 1 |
| BRACE2002151 | 0.001189244 | 155744 | 1 |
| BRACE2002157 | 0.001189244 | 143410 | 1 |
| BRACE2002176 | 0.003187646 | 180777 | 2 |
| BRACE2002202 | 0.004663012 | 181084 | 4 |
| BRACE2002206 | 0.001189244 | 114072 | 1 |
| BRACE2002227 | 0.001189244 | 121343 | 1 |
| BRACE2002260 | 0.001189244 | 180781 | 1 |
| BRACE2002297 | 0.001189244 | 208054 | 1 |
| BRACE2002304 | 0.001189244 | 154933 | 1 |
| BRACE2002356 | 0.00922151 | 42663 | 5 |
| BRACE2002392 | 0.097211361 | 103454 | 25 |
| BRACE2002394 | 0.001189244 | 285151 | 1 |
| BRACE2002409 | 0.003187646 | 130594 | 2 |
| BRACE2002431 | 0.001189244 | 54577 | 1 |
| BRACE2002441 | 0.148775007 | 111346 | 21 |
| BRACE2002444 | 0.001189244 | 41749 | 1 |
| BRACE2002458 | 0.001189244 | 175336 | 1 |
| BRACE2002467 | 0.025633146 | 155379 | 3 |
| BRACE2002468 | 0.001189244 | 99366 | 1 |
| BRACE2002478 | 0.011560335 | 175363 | 7 |
| BRACE2002495 | 0.00723081 | 168524 | 2 |
| BRACE2002582 | 0.025645851 | 101505 | 9 |
| BRACE2002585 | 0.006721605 | 110818 | 3 |
| BRACE2002589 | 0.001189244 | 272237 | 1 |
| BRACE2002590 | 0.001189244 | 140923 | 1 |
| BRACE2002606 | 0.031262135 | 114772 | 5 |
| BRACE2002613 | 0.001189244 | 108603 | 1 |
| BRACE2002614 | 0.001189244 | 107029 | 1 |
| BRACE2002617 | 0.001189244 | 265212 | 1 |
| BRACE2002623 | 0.001189244 | 114765 | 1 |
| BRACE2002635 | 0.002877521 | 140061 | 2 |
| BRACE2002649 | 0.015825326 | 131524 | 9 |
| BRACE2002661 | 0.058181693 | 124597 | 9 |
| BRACE2002676 | 0.001189244 | 49196 | 1 |
| BRACE2002677 | 0.086792471 | 103900 | 15 |
| BRACE2002682 | 0.008751187 | 132202 | 4 |
| BRACE2002685 | 0.048883651 | 201820 | 4 |
| BRACE2002695 | 0.002378489 | 139887 | 2 |
| BRACE2002707 | 0.001189244 | 111285 | 1 |
| BRACE2002736 | 0.001189244 | 128665 | 1 |
| BRACE2002752 | 0.010897982 | 242273 | 2 |
| BRACE2002755 | 0.003066086 | 171219 | 2 |
| BRACE2002762 | 0.001189244 | 238001 | 1 |
| BRACE2002772 | 0.001189244 | 173638 | 1 |
| BRACE2002792 | 0.001189244 | 145752 | 1 |
| BRACE2002796 | 0.003270756 | 132488 | 2 |
| BRACE2002803 | 0.009195636 | 88035 | 6 |
| BRACE2002807 | 0.00519938 | 123075 | 3 |
| BRACE2002812 | 0.028512311 | 60834 | 13 |
| BRACE2002834 | 0.002378489 | 175252 | 2 |
| BRACE2002843 | 0.22660084 | 33605 | 58 |
| BRACE2002849 | 0.001189244 | 264019 | 1 |
| BRACE2002857 | 0.001189244 | 160288 | 1 |
| BRACE2002860 | 0.066552329 | 5635 | 16 |
| BRACE2002861 | 0.064566514 | 117326 | 12 |
| BRACE2002884 | 0.001189244 | 111155 | 1 |
| BRACE2002896 | 0.011179055 | 23341 | 5 |
| BRACE2002948 | 0.001189244 | 245402 | 1 |
| BRACE2003037 | 0.077599497 | 71466 | 25 |
| BRACE2003078 | 0.010351617 | 105424 | 3 |
| BRACE2003097 | 0.002898705 | 164178 | 2 |
| BRACE2003110 | 0.012438635 | 186504 | 4 |
| BRACE2003168 | 0.001189244 | 259248 | 1 |
| BRACE2003172 | 0.515557623 | 94268 | 154 |
| BRACE2003199 | 0.001189244 | 106524 | 1 |
| BRACE2003210 | 0.002378489 | 169936 | 2 |
| BRACE2003217 | 0.038871874 | 68900 | 9 |
| BRACE2003254 | 0.185073124 | 86751 | 35 |
| BRACE2003269 | 0.001189244 | 117238 | 1 |
| BRACE2003285 | 0.001189244 | 68395 | 1 |
| BRACE2003304 | 0.076642407 | 8482 | 16 |
| BRACE2003319 | 0.073132035 | 10304 | 4 |
| BRACE2003398 | 0.007243613 | 267224 | 2 |
| BRACE2003420 | 0.001189244 | 198564 | 1 |
| BRACE2003428 | 0.001189244 | 15191 | 1 |
| BRACE2003431 | 0.001189244 | 171176 | 1 |
| BRACE2003449 | 0.067608748 | 117850 | 22 |
| BRACE2003512 | 0.005186047 | 62441 | 3 |
| BRACE2003516 | 0.003187646 | 89609 | 2 |
| BRACE2003527 | 0.065002078 | 47270 | 13 |
| BRACE2003539 | 0.003047359 | 100355 | 2 |
| BRACE2003540 | 0.001189244 | 104535 | 1 |
| BRACE2003594 | 0.014349722 | 226521 | 3 |
| BRACE2003598 | 0.006094718 | 115732 | 4 |
| BRACE2003609 | 0.018433522 | 97813 | 7 |
| BRACE2003614 | 0.001189244 | 113504 | 1 |
| BRACE2003628 | 0.024560067 | 154466 | 7 |
| BRACE2003639 | 0.001189244 | 165240 | 1 |
| BRACE2003700 | 0.012200138 | 113523 | 8 |
| BRACE2003713 | 0.007243613 | 253583 | 2 |
| BRACE2003766 | 0.001189244 | 185696 | 1 |
| BRACE2003785 | 0.17242849 | 108323 | 45 |
| BRACE2003788 | 0.00429586 | 175370 | 3 |
| BRACE2003800 | 0.006113445 | 136299 | 4 |
| BRACE2003802 | 0.002378489 | 165262 | 2 |
| BRACE2003825 | 0.012313952 | 118486 | 2 |
| BRACE2003827 | 0.191244224 | 63595 | 42 |
| BRACE2003845 | 0.036775553 | 112087 | 12 |
| BRACE2003846 | 0.257097629 | 111157 | 31 |
| BRACE2003847 | 0.001189244 | 283219 | 1 |
| BRACE2003848 | 0.003066086 | 39370 | 2 |
| BRACE2003873 | 0.001189244 | 199346 | 1 |
| BRACE2003885 | 0.001189244 | 107600 | 1 |
| BRACE2003887 | 0.001189244 | 257294 | 1 |
| BRACE2003892 | 0.076773409 | 62724 | 27 |
| BRACE2003904 | 0.06463287 | 64974 | 14 |
| BRACE2003905 | 0.010595549 | 157872 | 7 |
| BRACE2003928 | 0.001189244 | 124346 | 1 |
| BRACE2003944 | 0.323677952 | 108195 | 40 |
| BRACE2003954 | 0.007101856 | 55642 | 2 |
| BRACE2003982 | 0.040716444 | 89008 | 10 |
| BRACE2003987 | 0.001189244 | 43206 | 1 |
| BRACE2005034 | 0.001189244 | 270218 | 1 |
| BRACE2005047 | 0.001189244 | 228652 | 1 |
| BRACE2005063 | 0.001189244 | 191153 | 1 |
| BRACE2005083 | 0.00696547 | 65336 | 5 |
| BRACE2005087 | 0.161528991 | 34376 | 69 |
| BRACE2005090 | 0.002284523 | 165711 | 2 |
| BRACE2005099 | 0.126048051 | 118147 | 40 |
| BRACE2005118 | 0.004756978 | 92217 | 4 |
| BRACE2005124 | 0.001189244 | 151453 | 1 |
| BRACE2005138 | 0.001189244 | 278805 | 1 |
| BRACE2005151 | 0.079742189 | 90872 | 13 |
| BRACE2005160 | 0.001189244 | 194376 | 1 |
| BRACE2005169 | 0.001189244 | 192616 | 1 |
| BRACE2005189 | 0.006612615 | 159288 | 4 |
| BRACE2005193 | 0.002898705 | 110026 | 2 |
| BRACE2005196 | 0.005269158 | 180812 | 3 |
| BRACE2005198 | 0.001189244 | 179517 | 1 |
| BRACE2005216 | 0.005946222 | 149522 | 5 |
| BRACE2005231 | 0.052483553 | 53758 | 20 |
| BRACE2005243 | 0.001189244 | 94059 | 1 |
| BRACE2005251 | 0.01585274 | 129793 | 10 |
| BRACE2005253 | 0.003047359 | 192462 | 2 |
| BRACE2005290 | 0.006585383 | 133765 | 4 |
| BRACE2005298 | 0.005501257 | 238111 | 3 |
| BRACE2005328 | 0.001189244 | 157538 | 1 |
| BRACE2005329 | 0.001189244 | 250935 | 1 |
| BRACE2005337 | 0.001189244 | 263896 | 1 |
| BRACE2005348 | 0.001189244 | 14925 | 1 |
| BRACE2005363 | 0.01179145 | 69313 | 2 |
| BRACE2005395 | 0.017771209 | 136896 | 9 |
| BRACE2005408 | 0.017616261 | 126124 | 7 |
| BRACE2005433 | 0.014508876 | 33267 | 4 |
| BRACE2005434 | 0.243038258 | 159387 | 22 |
| BRACE2005448 | 0.02464078 | 114059 | 6 |
| BRACE2005450 | 0.001189244 | 196801 | 1 |
| BRACE2005453 | 0.012952566 | 179221 | 2 |
| BRACE2005457 | 0.002864792 | 148694 | 2 |
| BRACE2005460 | 0.001189244 | 191165 | 1 |
| BRACE2005489 | 0.001189244 | 251214 | 1 |
| BRACE2005518 | 0.001189244 | 64745 | 1 |
| BRACE2005556 | 0.001189244 | 145382 | 1 |
| BRACE2005562 | 0.001189244 | 179357 | 1 |
| BRACE2005624 | 0.001189244 | 168474 | 1 |
| BRACE2005642 | 0.022931482 | 74158 | 9 |
| BRACE2005661 | 0.001189244 | 171453 | 1 |
| BRACE2005667 | 0.001189244 | 148123 | 1 |
| BRACE2005681 | 0.167842894 | 40549 | 87 |
| BRACE2005719 | 0.056417443 | 115048 | 16 |
| BRACE2005731 | 0.002877521 | 169088 | 2 |
| BRACE2005742 | 0.001189244 | 139089 | 1 |
| BRACE2005762 | 0.001189244 | 147495 | 1 |
| BRACE2005773 | 0.001189244 | 168467 | 1 |
| BRACE2005787 | 0.001189244 | 187705 | 1 |
| BRACE2005795 | 0.001189244 | 252383 | 1 |
| BRACE2005800 | 0.001189244 | 150044 | 1 |
| BRACE2005858 | 0.016215826 | 84377 | 10 |
| BRACE2005869 | 0.001189244 | 216537 | 1 |
| BRACE2005875 | 0.03892138 | 153328 | 3 |
| BRACE2005881 | 0.001189244 | 165965 | 1 |
| BRACE2005898 | 0.002378489 | 165487 | 2 |
| BRACE2005904 | 0.001189244 | 161930 | 1 |
| BRACE2005906 | 0.001189244 | 142179 | 1 |
| BRACE2005907 | 0.001189244 | 171454 | 1 |
| BRACE2005911 | 0.001189244 | 160120 | 1 |
| BRACE2005937 | 0.027739619 | 175663 | 8 |
| BRACE2005949 | 0.001189244 | 144908 | 1 |
| BRACE2005981 | 0.001189244 | 111483 | 1 |
| BRACE2005991 | 0.001189244 | 155307 | 1 |
| BRACE2006055 | 0.083760892 | 155424 | 18 |
| BRACE2006072 | 0.008500825 | 213122 | 4 |
| BRACE2006075 | 0.010326307 | 217884 | 3 |
| BRACE2006084 | 0.054589823 | 184305. | 7 |
| BRACE2006089 | 0.041095851 | 30416 | 24 |
| BRACE2006102 | 0.003983555 | 209616 | 3 |
| BRACE2006105 | 0.007897763 | 77099 | 5 |
| BRACE2006122 | 0.001189244 | 234826 | 1 |
| BRACE2006137 | 0.002877521 | 204780 | 2 |
| BRACE2006143 | 0.001189244 | 244251 | 1 |
| BRACE2006162 | 0.108285945 | 160645 | 9 |
| BRACE2006167 | 0.079483894 | 34093 | 31 |
| BRACE2006174 | 0.001189244 | 262976 | 1 |
| BRACE2006240 | 0.001189244 | 271255 | 1 |
| BRACE2006245 | 0.001189244 | 252264 | 1 |
| BRACE2006249 | 0.0025864 | 203093 | 2 |
| BRACE2006258 | 0.204556781 | 67510 | 34 |
| BRACE2006264 | 0.019852288 | 188999 | 7 |
| BRACE2006274 | 0.005755042 | 99934 | 4 |
| BRACE2006281 | 0.055446818 | 70046 | 3 |
| BRACE2006298 | 0.001189244 | 255370 | 1 |
| BRACE2006319 | 0.047792784 | 49721 | 19 |
| BRACE2006344 | 0.046874944 | 145391 | 5 |
| BRACE2006354 | 0.026926489 | 99305 | 10 |
| BRACE2006363 | 0.018078792 | 126430 | 8 |
| BRACE2006378 | 0.003066086 | 201430 | 2 |
| BRACE2006380 | 0.004087949 | 159761 | 3 |
| BRACE2006393 | 0.001189244 | 257566 | 1 |
| BRACE2006397 | 0.567652044 | 94125 | 50 |
| BRACE2006413 | 0.001189244 | 250113 | 1 |
| BRACE2006417 | 0.011892445 | 90652 | 10 |
| BRACE2006453 | 0.001189244 | 171125 | 1 |
| BRACE2006459 | 0.004104101 | 251803 | 2 |
| BRACE2006488 | 0.003567733 | 1822 | 3 |
| BRACE2006514 | 0.192867899 | 11048 | 61 |
| BRACE2006534 | 0.001189244 | 99015 | 1 |
| BRACE2006537 | 0.155686962 | 95917 | 33 |
| BRACE2006540 | 0.001189244 | 255246 | 1 |
| BRACE2006547 | 0.011198766 | 152427 | 5 |
| BRACE2006564 | 0.003983555 | 160946 | 3 |
| BRACE2006587 | 0.001189244 | 222032 | 1 |
| BRACE2006598 | 0.005792378 | 110900 | 3 |
| BRACE2006636 | 0.01222174 | 118499 | 7 |
| BRACE2006685 | 0.001189244 | 261600 | 1 |
| BRACE2006703 | 0.001189244 | 262684 | 1 |
| BRACE2006743 | 0.037684663 | 103447 | 15 |
| BRACE2006753 | 0.027249027 | 128172 | 6 |
| BRACE2006783 | 0.001189244 | 154308 | 1 |
| BRACE2006821 | 0.090743342 | 104649 | 22 |
| BRACE2006833 | 0.004460001 | 171316 | 3 |
| BRACE2006859 | 0.016491365 | 153508 | 4 |
| BRACE2006871 | 0.167574939 | 116102 | 9 |
| BRACE2006900 | 0.325644347 | 29047 | 36 |
| BRACE2006909 | 0.001189244 | 230630 | 1 |
| BRACE2006911 | 0.009023047 | 155213 | 3 |
| BRACE2006924 | 0.001189244 | 236896 | 1 |
| BRACE2006935 | 0.007243613 | 155118 | 2 |
| BRACE2006937 | 0.125196352 | 121760 | 31 |
| BRACE2006944 | 0.014365826 | 42390 | 3 |
| BRACE2006951 | 0.001189244 | 153398 | 1 |
| BRACE2006982 | 0.12916629 | 6982 | 26 |
| BRACE2006986 | 0.001189244 | 234274 | 1 |
| BRACE2007013 | 0.016341915 | 78490 | 8 |
| BRACE2007064 | 0.103117687 | 50384 | 38 |
| BRACE2007068 | 0.006690501 | 172630 | 4 |
| BRACE2007087 | 0.001189244 | 246196 | 1 |
| BRACE2007089 | 0.197885429 | 69728 | 14 |
| BRACE2007109 | 0.17553833 | 108689 | 65 |
| BRACE2007138 | 0.001189244 | 230550 | 1 |
| BRACE2007153 | 0.224251358 | 125514 | 49 |
| BRACE2007174 | 0.010725179 | 118448 | 5 |
| BRACE2007191 | 0.243938443 | 131102 | 38 |
| BRACE2007197 | 0.001189244 | 285656 | 1 |
| BRACE2007201 | 0.002898705 | 247830 | 2 |
| BRACE2007203 | 0.001189244 | 234245 | 1 |
| BRACE2007232 | 0.001189244 | 180360 | 1 |
| BRACE2007254 | 0.022508864 | 153140 | 6 |
| BRACE2007262 | 0.001189244 | 126993 | 1 |
| BRACE2007274 | 0.001189244 | 241721 | 1 |
| BRACE2007281 | 0.001189244 | 99385 | 1 |
| BRACE2007295 | 0.017818629 | 175248 | 5 |
| BRACE2007358 | 0.001189244 | 242533 | 1 |
| BRACE2007396 | 0.001189244 | 248147 | 1 |
| BRACE2007398 | 0.001189244 | 52678 | 1 |
| BRACE2007401 | 0.091683993 | 88738 | 26 |
| BRACE2007411 | 0.003567733 | 176323 | 3 |
| BRACE2007447 | 0.001189244 | 247769 | 1 |
| BRACE2007477 | 0.001189244 | 242604 | 1 |
| BRACE2007502 | 0.002378489 | 134480 | 2 |
| BRACE2007503 | 0.001189244 | 217246 | 1 |
| BRACE2007518 | 0.073827438 | 102551 | 32 |
| BRACE2007522 | 0.002898705 | 154500 | 2 |
| BRACE2007527 | 0.001189244 | 113455 | 1 |
| BRACE2007538 | 0.001189244 | 40271 | 1 |
| BRACE2007563 | 0.001189244 | 243885 | 1 |
| BRACE2007567 | 0.120753346 | 124417 | 16 |
| BRACE2007577 | 0.171121036 | 72310 | 13 |
| BRACE2007621 | 0.001189244 | 244840 | 1 |
| BRACE2007640 | 0.003567733 | 88485 | 3 |
| BRACE2007641 | 0.106885695 | 21293 | 46 |
| BRACE2007646 | 0.002877521 | 110136 | 2 |
| BRACE2007663 | 0.005277194 | 149782 | 4 |
| BRACE2007671 | 0.108374936 | 90530 | 18 |
| BRACE2007682 | 0.001189244 | 230535 | 1 |
| BRACE2007685 | 0.001189244 | 64181 | 1 |
| BRACE2007708 | 0.078848286 | 154775 | 15 |
| BRACE2007727 | 0.001189244 | 244060 | 1 |
| BRACE2007749 | 0.001189244 | 128479 | 1 |
| BRACE2007760 | 0.012784712 | 74217 | 10 |
| BRACE2007761 | 0.001189244 | 267719 | 1 |
| BRACE2007764 | 0.002877521 | 4420 | 2 |
| BRACE2007767 | 0.05381986 | 144915 | 5 |
| BRACE2007768 | 0.017511022 | 146332 | 6 |
| BRACE2007769 | 0.001189244 | 221669 | 1 |
| BRACE2007784 | 0.001189244 | 86887 | 1 |
| BRACE2007786 | 0.001189244 | 180262 | 1 |
| BRACE2007798 | 0.161988061 | 32099 | 36 |
| BRACE2007799 | 0.001189244 | 173702 | 1 |
| BRACE2007801 | 0.002284523 | 102202 | 2 |
| BRACE2007832 | 0.013350897 | 175572 | 7 |
| BRACE2007836 | 0.001189244 | 217903 | 1 |
| BRACE2007868 | 0.001189244 | 39294 | 1 |
| BRACE2007882 | 0.025441469 | 161035 | 14 |
| BRACE2007888 | 0.001189244 | 133348 | 1 |
| BRACE2007902 | 0.005227527 | 168485 | 2 |
| BRACE2007920 | 0.001189244 | 191167 | 1 |
| BRACE2007937 | 0.065970832 | 84509 | 29 |
| BRACE2007944 | 0.001189244 | 218029 | 1 |
| BRACE2007952 | 0.069106987 | 108548 | 31 |
| BRACE2007953 | 0.104236124 | 154196 | 6 |
| BRACE2008008 | 0.001189244 | 54881 | 1 |
| BRACE2008015 | 0.001189244 | 56521 | 1 |
| BRACE2008021 | 0.001189244 | 129663 | 1 |
| BRACE2008097 | 0.034899573 | 159484 | 6 |
| BRACE2008102 | 0.007054456 | 146187 | 3 |
| BRACE2008114 | 0.001189244 | 213249 | 1 |
| BRACE2008144 | 0.001189244 | 199209 | 1 |
| BRACE2008172 | 0.001189244 | 133693 | 1 |
| BRACE2008216 | 0.0126554 | 156162 | 4 |
| BRACE2008218 | 0.001189244 | 191398 | 1 |
| BRACE2008251 | 0.012368099 | 160350 | 4 |
| BRACE2008276 | 0.001189244 | 139106 | 1 |
| BRACE2008295 | 0.001189244 | 159895 | 1 |
| BRACE2008308 | 0.001189244 | 168480 | 1 |
| BRACE2008336 | 0.001189244 | 183863 | 1 |
| BRACE2008358 | 0.073941797 | 92379 | 20 |
| BRACE2008361 | 0.002378489 | 56309 | 2 |
| BRACE2008380 | 0.085760918 | 142497 | 19 |
| BRACE2008385 | 0.080224922 | 121227 | 13 |
| BRACE2008399 | 0.030273708 | 68382 | 5 |
| BRACE2008401 | 0.001189244 | 189130 | 1 |
| BRACE2008402 | 0.039956833 | 72031 | 8 |
| BRACE2008410 | 0.001189244 | 206913 | 1 |
| BRACE2008420 | 0.001189244 | 259891 | 1 |
| BRACE2008443 | 0.001189244 | 147720 | 1 |
| BRACE2008473 | 0.001189244 | 239082 | 1 |
| BRACE2008480 | 0.001189244 | 210791 | 1 |
| BRACE2008481 | 0.060022429 | 52249 | 8 |
| BRACE2008488 | 0.001189244 | 90930 | 1 |
| BRACE2008495 | 0.007334771 | 42391 | 2 |
| BRACE2008500 | 0.001189244 | 41129 | 1 |
| BRACE2008553 | 0.040287278 | 185004 | 5 |
| BRACE2008594 | 0.001189244 | 75334 | 1 |
| BRACE2008604 | 0.001189244 | 139728 | 1 |
| BRACE2008615 | 0.001189244 | 235013 | 1 |
| BRACE2008622 | 0.001189244 | 222015 | 1 |
| BRACE2008629 | 0.001189244 | 210096 | 1 |
| BRACE2008653 | 0.317700554 | 22887 | 122 |
| BRACE2008655 | 0.001189244 | 82033 | 1 |
| BRACE2008656 | 0.001189244 | 90862 | 1 |
| BRACE2008706 | 0.050994595 | 2457 | 12 |
| BRACE2008708 | 0.011987788 | 154887 | 6 |
| BRACE2008710 | 0.001189244 | 244142 | 1 |
| BRACE2008754 | 0.001189244 | 110483 | 1 |
| BRACE2008797 | 0.013499711 | 113154 | 8 |
| BRACE2008847 | 0.001189244 | 212662 | 1 |
| BRACE2008857 | 0.002864792 | 166051 | 2 |
| BRACE2008861 | 0.001189244 | 196343 | 1 |
| BRACE2008867 | 0.002378489 | 47837 | 2 |
| BRACE2008881 | 0.012087227 | 120538 | 3 |
| BRACE2008883 | 0.001189244 | 232501 | 1 |
| BRACE2008917 | 0.001189244 | 271030 | 1 |
| BRACE2008919 | 0. 001189244 | 218058 | 1 |
| BRACE2008941 | 0.016765604 | 130467 | 7 |
| BRACE2008948 | 0.001189244 | 20474 | 1 |
| BRACE2008960 | 0.003567733 | 209967 | 3 |
| BRACE2008968 | 0.288163224 | 121926 | 34 |
| BRACE2008999 | 0.023806966 | 143537 | 7 |
| BRACE2009014 | 0.001189244 | 95156 | 1 |
| BRACE2009016 | 0.001189244 | 242650 | 1 |
| BRACE2009031 | 0.001189244 | 100079 | 1 |
| BRACE2009037 | 0.573817419 | 4525 | 63 |
| BRACE2009044 | 0.016699921 | 166211 | 7 |
| BRACE2009045 | 0.027828217 | 83640 | 14 |
| BRACE2009053 | 0.001189244 | 229251 | 1 |
| BRACE2009122 | 0.001189244 | 81982 | 1 |
| BRACE2009131 | 0.001189244 | 15612 | 1 |
| BRACE2009185 | 0.001189244 | 257741 | 1 |
| BRACE2009188 | 0.001189244 | 66571 | 1 |
| BRACE2009212 | 0.001189244 | 103113 | 1 |
| BRACE2009221 | 0.001189244 | 262082 | 1 |
| BRACE2009235 | 0.001189244 | 37733 | 1 |
| BRACE2009238 | 0.001189244 | 244505 | 1 |
| BRACE2009255 | 0.007054456 | 221652 | 3 |
| BRACE2009270 | 0.001189244 | 262765 | 1 |
| BRACE2009272 | 0.001189244 | 256547 | 1 |
| BRACE2009274 | 0.001189244 | 259886 | 1 |
| BRACE2009275 | 0.001189244 | 246818 | 1 |
| BRACE2009291 | 0.126668675 | 116526 | 14 |
| BRACE2009293 | 0.001189244 | 261750 | 1 |
| BRACE2009307 | 0.01131341 | 72313 | 8 |
| BRACE2009311 | 0.002877521 | 236956 | 2 |
| BRACE2009318 | 0.001189244 | 254503 | 1 |
| BRACE2009323 | 0.001189244 | 228794 | 1 |
| BRACE2009361 | 0.034447498 | 106221 | 13 |
| BRACE2009366 | 0.046375375 | 168064 | 5 |
| BRACE2009421 | 0.001189244 | 256680 | 1 |
| BRACE2009428 | 0.001189244 | 234105 | 1 |
| BRACE2009434 | 0.001189244 | 239220 | 1 |
| BRACE2009437 | 0.001189244 | 244925 | 1 |
| BRACE2009483 | 0.019069861 | 93738 | 6 |
| BRACE2009517 | 0.061776874 | 106292 | 16 |
| BRACE2009533 | 0.001189244 | 262712 | 1 |
| BRACE2009558 | 0.058274451 | 117558 | 12 |
| BRACE2009566 | 0.00883448 | 199057 | 3 |
| BRACE2009591 | 0.001189244 | 233334 | 1 |
| BRACE2009620 | 0.008731926 | 79065 | 3 |
| BRACE2009654 | 0.001189244 | 216624 | 1 |
| BRACE2009721 | 0.006994423 | 182629 | 2 |
| BRACE2009732 | 0.001189244 | 197086 | 1 |
| BRACE2009754 | 0.001189244 | 74772 | 1 |
| BRACE2009828 | 0.004565798 | 141115 | 3 |
| BRACE2009886 | 0.001189244 | 189096 | 1 |
| BRACE2009907 | 0.001189244 | 125581 | 1 |
| BRACE2009957 | 0.004284741 | 37521 | 2 |
| BRACE2010170 | 0.001189244 | 168616 | 1 |
| BRACE2010171 | 0.003567733 | 173067 | 3 |
| BRACE2010203 | 0.001189244 | 14948 | 1 |
| BRACE2010208 | 0.001189244 | 168617 | 1 |
| BRACE2010336 | 0.012313952 | 155347 | 2 |
| BRACE2010348 | 0.001189244 | 168611 | 1 |
| BRACE2010440 | 0.001189244 | 14807 | 1 |
| BRACE2010444 | 0.001189244 | 100861 | 1 |
| BRACE2010477 | 0.001189244 | 163301 | 1 |
| BRACE2010489 | 0.015829508 | 14377 | 10 |
| BRACE2010535 | 0.001189244 | 158388 | 1 |
| BRACE2010550 | 0.001189244 | 143937 | 1 |
| BRACE2010598 | 0.001189244 | 95651 | 1 |
| BRACE2010632 | 0.001189244 | 133217 | 1 |
| BRACE2010642 | 0.010897982 | 169237 | 2 |
| BRACE2010651 | 0.009067111 | 94884 | 3 |
| BRACE2010669 | 0.026283838 | 134883 | 15 |
| BRACE2010684 | 0.003066086 | 82176 | 2 |
| BRACE2010753 | 0.008983134 | 239625 | 3 |
| BRACE2010813 | 0.001189244 | 113253 | 1 |
| BRACE2010828 | 0.004284741 | 128309 | 2 |
| BRACE2010837 | 0.001189244 | 78307 | 1 |
| BRACE2010888 | 0.001189244 | 160783 | 1 |
| BRACE2010937 | 0.001189244 | 207883 | 1 |
| BRACE2010983 | 0.001189244 | 49965 | 1 |
| BRACE2011007 | 0.001189244 | 205132 | 1 |
| BRACE2011025 | 0.001189244 | 191252 | 1 |
| BRACE2011183 | 0.026065865 | 132980 | 13 |
| BRACE2011199 | 0.001189244 | 200222 | 1 |
| BRACE2011265 | 0.045167185 | 131887 | 4 |
| BRACE2011389 | 0.001189244 | 166287 | 1 |
| BRACE2011478 | 0.001189244 | 220741 | 1 |
| BRACE2011545 | 0.001189244 | 183842 | 1 |
| BRACE2011592 | 0.001189244 | 131526 | 1 |
| BRACE2011611 | 0.001189244 | 211435 | 1 |
| BRACE2011646 | 0.001189244 | 199613 | 1 |
| BRACE2011677 | 0.001189244 | 199638 | 1 |
| BRACE2011703 | 0.004104101 | 67945 | 2 |
| BRACE2011747 | 0.002378489 | 92459 | 2 |
| BRACE2011838 | 0.001189244 | 42924 | 1 |
| BRACE2011892 | 0.001189244 | 114304 | 1 |
| BRACE2011904 | 0.001189244 | 284895 | 1 |
| BRACE2011947 | 0.001189244 | 191309 | 1 |
| BRACE2012061 | 0.009945564 | 61436 | 3 |
| BRACE2012062 | 0.001189244 | 32297 | 1 |
| BRACE2012185 | 0.001189244 | 189272 | 1 |
| BRACE2012222 | 0.007852474 | 198 | 5 |
| BRACE2012232 | 0.002877521 | 122011 | 2 |
| BRACE2012291 | 0.004284741 | 161196 | 2 |
| BRACE2012317 | 0.244180507 | 78598 | 143 |
| BRACE2012510 | 0.001189244 | 199134 | 1 |
| BRACE2012528 | 0.353320111 | 30663 | 153 |
| BRACE2012625 | 0.001189244 | 194923 | 1 |
| BRACE2012653 | 0.002877521 | 172764 | 2 |
| BRACE2012814 | 0.109265947 | 50905 | 26 |
| BRACE2012833 | 0.001189244 | 93506 | 1 |
| BRACE2012838 | 0.001189244 | 129303 | 1 |
| BRACE2012936 | 0.001189244 | 232255 | 1 |
| BRACE2012947 | 0.001189244 | 161055 | 1 |
| BRACE2013009 | 0.001189244 | 206931 | 1 |
| BRACE2013010 | 0.001189244 | 206754 | 1 |
| BRACE2013126 | 0.001189244 | 259480 | 1 |
| BRACE2013132 | 0.001189244 | 229262 | 1 |
| BRACE2013149 | 0.001189244 | 217457 | 1 |
| BRACE2013369 | 0.005366179 | 161049 | 2 |
| BRACE2013624 | 0.00931331 | 115882 | 5 |
| BRACE2013806 | 0.001189244 | 165551 | 1 |
| BRACE2013855 | 0.020889599 | 169975 | 5 |
| BRACE2013911 | 0.022290047 | 2207 | 8 |
| BRACE2014108 | 0.002378489 | 165542 | 2 |
| BRACE2014161 | 0.001189244 | 33385 | 1 |
| BRACE2014232 | 0.001189244 | 209561 | 1 |
| BRACE2014257 | 0.013511576 | 133947 | 9 |
| BRACE2014268 | 0.001189244 | 76843 | 1 |
| BRACE2014306 | 0.034424422 | 49096 | 8 |
| BRACE2014475 | 0.001189244 | 271557 | 1 |
| BRACE2014657 | 0.021688793 | 138948 | 17 |
| BRACE2014746 | 0.024011621 | 146076 | 7 |
| BRACE2014780 | 0.001189244 | 219837 | 1 |
| BRACE2014818 | 0.004104101 | 187936 | 2 |
| BRACE2014821 | 0.001189244 | 120300 | 1 |
| BRACE2014824 | 0.001189244 | 114075 | 1 |
| BRACE2014850 | 0.022340617 | 48270 | 8 |
| BRACE2014854 | 0.001189244 | 78522 | 1 |
| BRACE2014917 | 0.011278791 | 80941 | 3 |
| BRACE2015031 | 0.001189244 | 83466 | 1 |
| BRACE2015058 | 0.038215023 | 161719 | 16 |
| BRACE2015132 | 0.010169901 | 148690 | 3 |
| BRACE2015262 | 0.001189244 | 45481 | 1 |
| BRACE2015270 | 0.001189244 | 173298 | 1 |
| BRACE2015287 | 0.072963499 | 46157 | 10 |
| BRACE2015314 | 0.001189244 | 276534 | 1 |
| BRACE2015352 | 0.001189244 | 260718 | 1 |
| BRACE2015368 | 0.002864792 | 133626 | 2 |
| BRACE2015412 | 0.013762774 | 126249 | 4 |
| BRACE2015436 | 0.001189244 | 220738 | 1 |
| BRACE2015741 | 0.001189244 | 62074 | 1 |
| BRACE2015756 | 0.001189244 | 268259 | 1 |
| BRACE2015969 | 0.004104101 | 158181 | 2 |
| BRACE2015995 | 0.001189244 | 21734 | 1 |
| BRACE2016058 | 0.001189244 | 73899 | 1 |
| BRACE2016194 | 0.005033328 | 135278 | 2 |
| BRACE2016315 | 0.001189244 | 169518 | 1 |
| BRACE2016325 | 0.001189244 | 129040 | 1 |
| BRACE2016335 | 0.024032885 | 46661 | 4 |
| BRACE2016362 | 0.01086925 | 70860 | 7 |
| BRACE2016366 | 0.019719539 | 146884 | 4 |
| BRACE2016465 | 0.002378489 | 154306 | 2 |
| BRACE2016516 | 0.001189244 | 92554 | 1 |
| BRACE2016583 | 0.001189244 | 193615 | 1 |
| BRACE2016708 | 0.001189244 | 202712 | 1 |
| BRACE2016821 | 0.001189244 | 169580 | 1 |
| BRACE2016896 | 0.001189244 | 97035 | 1 |
| BRACE2016981 | 0.007391564 | 238972 | 2 |
| BRACE2017108 | 0.001189244 | 27536 | 1 |
| BRACE2017124 | 0.001189244 | 160394 | 1 |
| BRACE2017298 | 0.001189244 | 162794 | 1 |
| BRACE2017359 | 0.004087949 | 119629 | 3 |
| BRACE2017397 | 0.071040729 | 131411 | 16 |
| BRACE2017410 | 0.002378489 | 124055 | 2 |
| BRACE2017438 | 0.004066766 | 41203 | 3 |
| BRACE2017574 | 0.032704414 | 87099 | 11 |
| BRACE2017580 | 0.001189244 | 160421 | 1 |
| BRACE2017587 | 0.001189244 | 163031 | 1 |
| BRACE2017720 | 0.001189244 | 141397 | 1 |
| BRACE2017844 | 0.032605046 | 40595 | 15 |
| BRACE2017872 | 0.001189244 | 278301 | 1 |
| BRACE2017929 | 0.001189244 | 160272 | 1 |
| BRACE2017934 | 0.018128793 | 152472 | 4 |
| BRACE2017985 | 0.003066086 | 135671 | 2 |
| BRACE2017992 | 0.001189244 | 201597 | 1 |
| BRACE2018337 | 0.070348372 | 127875 | 16 |
| BRACE2018443 | 0.0025864 | 135620 | 2 |
| BRACE2018448 | 0.001189244 | 90106 | 1 |
| BRACE2018568 | 0.001189244 | 84752 | 1 |
| BRACE2018700 | 0.001189244 | 56190 | 1 |
| BRACE2018727 | 0.001189244 | 212341 | 1 |
| BRACE2018736 | 0.004142637 | 96058 | 3 |
| BRACE2018759 | 0.001189244 | 159274 | 1 |
| BRACE2018762 | 0.002898705 | 146432 | 2 |
| BRACE2018782 | 0.001189244 | 160483 | 1 |
| BRACE2018847 | 0.00858901 | 154407 | 6 |
| BRACE2019004 | 0.003047359 | 151363 | 2 |
| BRACE2019044 | 0.001189244 | 187459 | 1 |
| BRACE2019147 | 0.078238458 | 113092 | 11 |
| BRACE2019244 | 0.004104101 | 168591 | 2 |
| BRACE2019258 | 0.001189244 | 228132 | 1 |
| BRACE2019327 | 0.018697818 | 102356 | 4 |
| BRACE2019348 | 0.014419429 | 30414 | 11 |
| BRACE2019371 | 0.011205958 | 90760 | 4 |
| BRACE2019467 | 0.001189244 | 64827 | 1 |
| BRACE2019510 | 0.001189244 | 193968 | 1 |
| BRACE2019519 | 0.009001861 | 28846 | 3 |
| BRACE2019528 | 0.001189244 | 45078 | 1 |
| BRACE2019618 | 0.001189244 | 119997 | 1 |
| BRACE2019696 | 0.002378489 | 160119 | 2 |
| BRACE2019703 | 0.001189244 | 127804 | 1 |
| BRACE2019843 | 0.001189244 | 201979 | 1 |
| BRACE2019865 | 0.001189244 | 164406 | 1 |
| BRACE2019872 | 0.001189244 | 262882 | 1 |
| BRACE2019877 | 0.02758694 | 92970 | 9 |
| BRACE2020028 | 0.001189244 | 223748 | 1 |
| BRACE2020077 | 0.001189244 | 134143 | 1 |
| BRACE2020097 | 0.001189244 | 226284 | 1 |
| BRACE2020157 | 0.001189244 | 163688 | 1 |
| BRACE2020278 | 0.001189244 | 148215 | 1 |
| BRACE2020358 | 0.001189244 | 182740 | 1 |
| BRACE2020467 | 0.002378489 | 58401 | 2 |
| BRACE2020584 | 0.003187646 | 161548 | 2 |
| BRACE2020652 | 0.001189244 | 210120 | 1 |
| BRACE2020709 | 0.001189244 | 275578 | 1 |
| BRACE2020742 | 0.001189244 | 91497 | 1 |
| BRACE2020756 | 0.001189244 | 156306 | 1 |
| BRACE2020863 | 0.001189244 | 210160 | 1 |
| BRACE2020982 | 0.001189244 | 186808 | 1 |
| BRACE2021014 | 0.028283424 | 118744 | 16 |
| BRACE2021143 | 0.021173272 | 72230 | 5 |
| BRACE2021148 | 0.001189244 | 220825 | 1 |
| BRACE2021245 | 0.001189244 | 122693 | 1 |
| BRACE2021254 | 0.001189244 | 92219 | 1 |
| BRACE2021264 | 0.001189244 | 235318 | 1 |
| BRACE2021344 | 0.005366179 | 199345 | 2 |
| BRACE2021433 | 0.001189244 | 114004 | 1 |
| BRACE2021478 | 0.012943319 | 74993 | 3 |
| BRACE2021541 | 0.010525536 | 179192 | 5 |
| BRACE2021670 | 0.001189244 | 164138 | 1 |
| BRACE2021695 | 0.001189244 | 6298 | 1 |
| BRACE2021708 | 0.005659102 | 115797 | 4 |
| BRACE2021721 | 0.001189244 | 198280 | 1 |
| BRACE2021932 | 0.002898705 | 110700 | 2 |
| BRACE2021936 | 0.001189244 | 238478 | 1 |
| BRACE2022030 | 0.001189244 | 191358 | 1 |
| BRACE2022158 | 0.001189244 | 269452 | 1 |
| BRACE2022196 | 0.001189244 | 163330 | 1 |
| BRACE2022270 | 0.001189244 | 179030 | 1 |
| BRACE2022328 | 0.001189244 | 158372 | 1 |
| BRACE2022333 | 0.018873269 | 103103 | 11 |
| BRACE2022389 | 0.015768543 | 204228 | 4 |
| BRACE2022394 | 0.001189244 | 279838 | 1 |
| BRACE2022435 | 0.001189244 | 179090 | 1 |
| BRACE2022448 | 0.002378489 | 168411 | 2 |
| BRACE2022450 | 0.001189244 | 163304 | 1 |
| BRACE2022549 | 0.003270756 | 186844 | 2 |
| BRACE2022633 | 0.001189244 | 169407 | 1 |
| BRACE2022638 | 0.224805206 | 93790 | 48 |
| BRACE2022693 | 0.001189244 | 192635 | 1 |
| BRACE2022848 | 0.001189244 | 254317 | 1 |
| BRACE2022928 | 0.001189244 | 131721 | 1 |
| BRACE2022990 | 0.002877521 | 123595 | 2 |
| BRACE2023069 | 0.001189244 | 23405 | 1 |
| BRACE2023070 | 0.001189244 | 159145 | 1 |
| BRACE2023223 | 0.004586981 | 79703 | 3 |
| BRACE2023268 | 0.001189244 | 172956 | 1 |
| BRACE2023284 | 0.001189244 | 200569 | 1 |
| BRACE2023404 | 0.001189244 | 182641 | 1 |
| BRACE2023451 | 0.001189244 | 204095 | 1 |
| BRACE2023540 | 0.004756978 | 73500 | 4 |
| BRACE2023633 | 0.001189244 | 214818 | 1 |
| BRACE2023718 | 0.010440573 | 133875 | 6 |
| BRACE2023727 | 0.008486842 | 10631 | 3 |
| BRACE2023744 | 0.001189244 | 143952 | 1 |
| BRACE2023800 | 0.001189244 | 143338 | 1 |
| BRACE2023801 | 0.004463242 | 123312 | 3 |
| BRACE2024074 | 0.0025864 | 122064 | 2 |
| BRACE2024222 | 0.001189244 | 209929 | 1 |
| BRACE2024610 | 0.002378489 | 158064 | 2 |
| BRACE2024627 | 0.013687031 | 143575 | 3 |
| BRACE2024781 | 0.002864792 | 122515 | 2 |
| BRACE2024826 | 0.001189244 | 113971 | 1 |
| BRACE2025018 | 0.001189244 | 215208 | 1 |
| BRACE2025316 | 0.001189244 | 196890 | 1 |
| BRACE2025333 | 0.002378489 | 34335 | 2 |
| BRACE2025437 | 0.054881851 | 34042 | 8 |
| BRACE2025452 | 0.002378489 | 140862 | 2 |
| BRACE2025492 | 0.006366253 | 117709 | 3 |
| BRACE2025710 | 0.001189244 | 71332 | 1 |
| BRACE2025796 | 0.005366179 | 129806 | 2 |
| BRACE2025992 | 0.002378489 | 120107 | 2 |
| BRACE2026054 | 0.007329622 | 44424 | 3 |
| BRACE2026131 | 0.002284523 | 139515 | 2 |
| BRACE2026141 | 0.004284741 | 173483 | 2 |
| BRACE2026142 | 0.007425602 | 150672 | 2 |
| BRACE2026293 | 0.001189244 | 210903 | 1 |
| BRACE2026294 | 0.001189244 | 53157 | 1 |
| BRACE2026404 | 0.001189244 | 197793 | 1 |
| BRACE2026675 | 0.001189244 | 165634 | 1 |
| BRACE2026725 | 0.009138469 | 167647 | 3 |
| BRACE2026836 | 0.024282302 | 192680 | 3 |
| BRACE2026920 | 0.001189244 | 168423 | 1 |
| BRACE2026971 | 0.001189244 | 59027 | 1 |
| BRACE2027018 | 0.007125019 | 216761 | 2 |
| BRACE2027221 | 0.001189244 | 84704 | 1 |
| BRACE2027258 | 0.004959033 | 194107 | 3 |
| BRACE2027312 | 0.001189244 | 247720 | 1 |
| BRACE2027382 | 0.001189244 | 232038 | 1 |
| BRACE2027389 | 0.001189244 | 227058 | 1 |
| BRACE2027408 | 0.001189244 | 106881 | 1 |
| BRACE2027643 | 0.011632802 | 98024 | 5 |
| BRACE2027767 | 0.005729583 | 149324 | 4 |
| BRACE2027770 | 0.001189244 | 158066 | 1 |
| BRACE2027879 | 0.001189244 | 231993 | 1 |
| BRACE2027896 | 0.006551469 | 32851 | 4 |
| BRACE2027915 | 0.004460001 | 171316 | 3 |
| BRACE2027970 | 0.0025864 | 166086 | 2 |
| BRACE2028171 | 0.010897982 | 203768 | 2 |
| BRACE2028392 | 0.032870112 | 155946 | 7 |
| BRACE2028410 | 0.001189244 | 35686 | 1 |
| BRACE2028636 | 0.012520806 | 18826 | 6 |
| BRACE2028741 | 0.001189244 | 180711 | 1 |
| BRACE2028956 | 0.001189244 | 156858 | 1 |
| BRACE2028970 | 0.001189244 | 170565 | 1 |
| BRACE2029112 | 0.001189244 | 73421 | 1 |
| BRACE2029356 | 0.0025864 | 215157 | 2 |
| BRACE2029396 | 0.001189244 | 203977 | 1 |
| BRACE2029581 | 0.001189244 | 143923 | 1 |
| BRACE2029644 | 0.0025864 | 177295 | 2 |
| BRACE2029849 | 0.001189244 | 270322 | 1 |
| BRACE2030039 | 0.001189244 | 262525 | 1 |
| BRACE2030096 | 0.001189244 | 234442 | 1 |
| BRACE2030323 | 0.01069797 | 109078 | 7 |
| BRACE2030326 | 0.001189244 | 170455 | 1 |
| BRACE2030341 | 0.024224665 | 150448 | 7 |
| BRACE2030345 | 0.0025864 | 152729 | 2 |
| BRACE2030697 | 0.079484867 | 8648 | 23 |
| BRACE2030736 | 0.001189244 | 223746 | 1 |
| BRACE2030883 | 0.001189244 | 282592 | 1 |
| BRACE2030884 | 0.001189244 | 219424 | 1 |
| BRACE2031154 | 0.003047359 | 181094 | 2 |
| BRACE2031232 | 0.001189244 | 163185 | 1 |
| BRACE2031389 | 0.016472575 | 153391 | 6 |
| BRACE2031527 | 0.001189244 | 173342 | 1 |
| BRACE2031531 | 0.001189244 | 173341 | 1 |
| BRACE2031553 | 0.001189244 | 273894 | 1 |
| BRACE2031899 | 0.001189244 | 169575 | 1 |
| BRACE2032044 | 0.001189244 | 143019 | 1 |
| BRACE2032090 | 0.001189244 | 61887 | 1 |
| BRACE2032134 | 0.001189244 | 197812 | 1 |
| BRACE2032148 | 0.001189244 | 183268 | 1 |
| BRACE2032182 | 0.001189244 | 193781 | 1 |
| BRACE2032329 | 0.002378489 | 147651 | 2 |
| BRACE2032385 | 0.001189244 | 145368 | 1 |
| BRACE2032436 | 0.001189244 | 186471 | 1 |
| BRACE2032477 | 0.030068076 | 139630 | 11 |
| BRACE2032502 | 0.003047359 | 158563 | 2 |
| BRACE2032538 | 0.001189244 | 193647 | 1 |
| BRACE2032584 | 0.001189244 | 173439 | 1 |
| BRACE2032823 | 0.001189244 | 159076 | 1 |
| BRACE2033128 | 0.001189244 | 191725 | 1 |
| BRACE2033394 | 0.001189244 | 173417 | 1 |
| BRACE2033478 | 0.001189244 | 189015 | 1 |
| BRACE2033720 | 0.001189244 | 200989 | 1 |
| BRACE2033786 | 0.001189244 | 173409 | 1 |
| BRACE2034434 | 0.001189244 | 270663 | 1 |
| BRACE2034523 | 0.001189244 | 277470 | 1 |
| BRACE2034616 | 0.001189244 | 221896 | 1 |
| BRACE2034622 | 0.001189244 | 203209 | 1 |
| BRACE2034870 | 0.001189244 | 255002 | 1 |
| BRACE2035003 | 0.001189244 | 237866 | 1 |
| BRACE2035120 | 0.001189244 | 97700 | 1 |
| BRACE2035124 | 0.001189244 | 135999 | 1 |
| BRACE2035191 | 0.001189244 | 181140 | 1 |
| BRACE2035278 | 0.004460001 | 193209 | 3 |
| BRACE2035381 | 0.016463922 | 140683 | 11 |
| BRACE2035441 | 0.001189244 | 196939 | 1 |
| BRACE2035485 | 0.001189244 | 156997 | 1 |
| BRACE2036005 | 0.001189244 | 279947 | 1 |
| BRACE2036096 | 0.001189244 | 283323 | 1 |
| BRACE2036743 | 0.001189244 | 55962 | 1 |
| BRACE2036830 | 0.001189244 | 154543 | 1 |
| BRACE2036834 | 0.001189244 | 160118 | 1 |
| BRACE2036900 | 0.001189244 | 173343 | 1 |
| BRACE2037132 | 0.002864792 | 161142 | 2 |
| BRACE2037294 | 0.001189244 | 275703 | 1 |
| BRACE2037295 | 0.05107896 | 138332 | 8 |
| BRACE2037299 | 0.001189244 | 255744 | 1 |
| BRACE2037310 | 0.008539914 | 116022 | 5 |
| BRACE2037431 | 0.001189244 | 280273 | 1 |
| BRACE2037692 | 0.001189244 | 278363 | 1 |
| BRACE2037847 | 0.001189244 | 261549 | 1 |
| BRACE2038114 | 0.001189244 | 187272 | 1 |
| BRACE2038269 | 0.002284523 | 185857 | 2 |
| BRACE2038271 | 0.002378489 | 233254 | 2 |
| BRACE2038329 | 0.003567733 | 106366 | 3 |
| BRACE2038492 | 0.001189244 | 276491 | 1 |
| BRACE2038551 | 0.001189244 | 186443 | 1 |
| BRACE2038618 | 0.001189244 | 253910 | 1 |
| BRACE2039009 | 0.001189244 | 197781 | 1 |
| BRACE2039128 | 0.001189244 | 206396 | 1 |
| BRACE2039170 | 0.001189244 | 176476 | 1 |
| BRACE2039249 | 0.001189244 | 217790 | 1 |
| BRACE2039286 | 0.001189244 | 159894 | 1 |
| BRACE2039297 | 0.001189244 | 271657 | 1 |
| BRACE2039327 | 0.001189244 | 120710 | 1 |
| BRACE2039362 | 0.001189244 | 266478 | 1 |
| BRACE2039475 | 0.001189244 | 162948 | 1 |
| BRACE2039600 | 0.001189244 | 156205 | 1 |
| BRACE2039607 | 0.001189244 | 248814 | 1 |
| BRACE2039624 | 0.17318819 | 110639 | 34 |
| BRACE2039734 | 0.001189244 | 180966 | 1 |
| BRACE2039823 | 0.002877521 | 34063 | 2 |
| BRACE2039832 | 0.001189244 | 160250 | 1 |
| BRACE2039893 | 0.001189244 | 90770 | 1 |
| BRACE2039986 | 0.015799087 | 95628 | 3 |
| BRACE2040138 | 0.001189244 | 256291 | 1 |
| BRACE2040325 | 0.001189244 | 264272 | 1 |
| BRACE2040514 | 0.001189244 | 69404 | 1 |
| BRACE2040640 | 0.001189244 | 178480 | 1 |
| BRACE2040987 | 0.001189244 | 151322 | 1 |
| BRACE2041009 | 0.001189244 | 194083 | 1 |
| BRACE2041095 | 0.001189244 | 240601 | 1 |
| BRACE2041200 | 0.003066086 | 127048 | 2 |
| BRACE2041264 | 0.001189244 | 194076 | 1 |
| BRACE2041890 | 0.001189244 | 165880 | 1 |
| BRACE2041898 | 0.001189244 | 162801 | 1 |
| BRACE2042394 | 0.001189244 | 207506 | 1 |
| BRACE2042398 | 0.001189244 | 176238 | 1 |
| BRACE2042541 | 0.005946222 | 149522 | 5 |
| BRACE2042550 | 0.001189244 | 280151 | 1 |
| BRACE2042620 | 0.001189244 | 173528 | 1 |
| BRACE2042628 | 0.001189244 | 208427 | 1 |
| BRACE2042873 | 0.001189244 | 221630 | 1 |
| BRACE2043036 | 0.001189244 | 200931 | 1 |
| BRACE2043105 | 0.001189244 | 202711 | 1 |
| BRACE2043142 | 0.002284523 | 217179 | 2 |
| BRACE2043248 | 0.001189244 | 215841 | 1 |
| BRACE2043349 | 0.001189244 | 132431 | 1 |
| BRACE2043381 | 0.015568047 | 32764 | 3 |
| BRACE2043665 | 0.007030707 | 218663 | 2 |
| BRACE2043774 | 0.001189244 | 281174 | 1 |
| BRACE2043942 | 0.001189244 | 269821 | 1 |
| BRACE2044057 | 0.001189244 | 233553 | 1 |
| BRACE2044105 | 0.008326089 | 182136 | 3 |
| BRACE2044263 | 0.001189244 | 148866 | 1 |
| BRACE2044286 | 0.008975759 | 107828 | 5 |
| BRACE2044304 | 0.001189244 | 272371 | 1 |
| BRACE2044473 | 0.001189244 | 276278 | 1 |
| BRACE2044510 | 0.001189244 | 275695 | 1 |
| BRACE2044640 | 0.001189244 | 228568 | 1 |
| BRACE2044816 | 0.001189244 | 187746 | 1 |
| BRACE2044946 | 0.001189244 | 184533 | 1 |
| BRACE2044949 | 0.042995265 | 275421 | 2 |
| BRACE2045300 | 0.001189244 | 162489 | 1 |
| BRACE2045328 | 0.007852474 | 198 | 5 |
| BRACE2045428 | 0.001189244 | 228314 | 1 |
| BRACE2045445 | 0.003187646 | 53580 | 2 |
| BRACE2045596 | 0.001189244 | 246679 | 1 |
| BRACE2045772 | 0.057878587 | 178675 | 2 |
| BRACE2045947 | 0.001189244 | 205141 | 1 |
| BRACE2045954 | 0.006135548 | 152963 | 4 |
| BRACE2046251 | 0.001189244 | 28876 | 1 |
| BRACE2046295 | 0.001189244 | 205392 | 1 |
| BRACE2046976 | 0.001189244 | 274104 | 1 |
| BRACE2047011 | 0.001189244 | 181440 | 1 |
| BRACE2047232 | 0.001189244 | 151494 | 1 |
| BRACE2047350 | 0.0025864 | 262844 | 2 |
| BRACE2047377 | 0.001189244 | 156767 | 1 |
| BRACE2047385 | 0.001189244 | 192935 | 1 |
| BRACE2047492 | 0.002877521 | 226506 | 2 |
| BRACE2047558 | 0.001189244 | 134717 | 1 |
| BRACE2047573 | 0.001189244 | 46721 | 1 |
| BRACE2047835 | 0.001189244 | 181494 | 1 |
| BRACE2047893 | 0.001189244 | 211781 | 1 |
| BRACE2047975 | 0.001189244 | 225885 | 1 |
| BRACE3000071 | 0.012475926 | 220026 | 2 |
| BRACE3000404 | 0.001189244 | 194754 | 1 |
| BRACE3000520 | 0.001189244 | 233671 | 1 |
| BRACE3000697 | 0.001189244 | 281964 | 1 |
| BRACE3000787 | 0.015304811 | 163440 | 7 |
| BRACE3000840 | 0.001189244 | 271497 | 1 |
| BRACE3000973 | 0.012431434 | 42734 | 10 |
| BRACE3001002 | 0.001189244 | 152370 | 1 |
| BRACE3001058 | 0.001189244 | 239739 | 1 |
| BRACE3001066 | 0.004310051 | 131463 | 2 |
| BRACE3001113 | 0.006366253 | 70834 | 3 |
| BRACE3001217 | 0.001189244 | 226468 | 1 |
| BRACE3001299 | 0.001189244 | 281001 | 1 |
| BRACE3001384 | 0.001189244 | 278808 | 1 |
| BRACE3001391 | 0.001189244 | 3627 | 1 |
| BRACE3001403 | 0.001189244 | 276971 | 1 |
| BRACE3001595 | 0.001189244 | 280871 | 1 |
| BRACE3001754 | 0.003066086 | 106962 | 2 |
| BRACE3001948 | 0.001189244 | 144921 | 1 |
| BRACE3001973 | 0.003270756 | 270727 | 2 |
| BRACE3002184 | 0.006252716 | 18869 | 3 |
| BRACE3002298 | 0.001189244 | 171941 | 1 |
| BRACE3002344 | 0.001189244 | 98555 | 1 |
| BRACE3002390 | 0.001189244 | 79450 | 1 |
| BRACE3002420 | 0.001189244 | 233592 | 1 |
| BRACE3002508 | 0.001189244 | 274398 | 1 |
| BRACE3002541 | 0.001189244 | 223843 | 1 |
| BRACE3002756 | 0.001189244 | 237062 | 1 |
| BRACE3003004 | 0.001189244 | 241547 | 1 |
| BRACE3003026 | 0.010260967 | 44231 | 4 |
| BRACE3003053 | 0.003270756 | 212426 | 2 |
| BRACE3003192 | 0.002864792 | 125573 | 2 |
| BRACE3003413 | 0.0025864 | 224239 | 2 |
| BRACE3003595 | 0.007135467 | 109650 | 6 |
| BRACE3003698 | 0.0025864 | 234028 | 2 |
| BRACE3003866 | 0.001189244 | 211465 | 1 |
| BRACE3003920 | 0.001189244 | 176605 | 1 |
| BRACE3004046 | 0.002898705 | 279350 | 2 |
| BRACE3004058 | 0.001189244 | 159838 | 1 |
| BRACE3004113 | 0.001189244 | 164089 | 1 |
| BRACE3004150 | 0.043140332 | 22395 | 7 |
| BRACE3004205 | 0.003473767 | 226065 | 3 |
| BRACE3004358 | 0.001189244 | 228022 | 1 |
| BRACE3004371 | 0.044600234 | 39521 | 18 |
| BRACE3004435 | 0.001189244 | 201680 | 1 |
| BRACE3004477 | 0.001189244 | 176719 | 1 |
| BRACE3004767 | 0.002864792 | 236073 | 2 |
| BRACE3004772 | 0.001189244 | 229092 | 1 |
| BRACE3004783 | 0.002378489 | 233556 | 2 |
| BRACE3004843 | 0.005266482 | 170248 | 3 |
| BRACE3004862 | 0.003066086 | 83676 | 2 |
| BRACE3004880 | 0.001189244 | 222589 | 1 |
| BRACE3004887 | 0.001189244 | 190917 | 1 |
| BRACE3004981 | 0.001189244 | 187202 | 1 |
| BRACE3004996 | 0.001189244 | 232376 | 1 |
| BRACE3004998 | 0.001189244 | 129051 | 1 |
| BRACE3005103 | 0.001189244 | 50996 | 1 |
| BRACE3005107 | 0.006416772 | 199019 | 3 |
| BRACE3005127 | 0.001189244 | 241318 | 1 |
| BRACE3005145 | 0.001189244 | 223316 | 1 |
| BRACE3005217 | 0.001189244 | 274015 | 1 |
| BRACE3005225 | 0.001189244 | 150216 | 1 |
| BRACE3005430 | 0.001189244 | 229360 | 1 |
| BRACE3005499 | 0.005357225 | 161806 | 4 |
| BRACE3005760 | 0.007439043 | 207400 | 5 |
| BRACE3005870 | 0.001189244 | 208337 | 1 |
| BRACE3005903 | 0.001189244 | 232736 | 1 |
| BRACE3005938 | 0.152151783 | 127032 | 36 |
| BRACE3005989 | 0.716731118 | 91573 | 19 |
| BRACE3006113 | 0.001189244 | 208623 | 1 |
| BRACE3006185 | 0.001189244 | 187232 | 1 |
| BRACE3006226 | 0.001189244 | 142659 | 1 |
| BRACE3006462 | 0.001189244 | 252312 | 1 |
| BRACE3006463 | 0.001189244 | 234664 | 1 |
| BRACE3006553 | 0.013017557 | 50414 | 11 |
| BRACE3006872 | 0.001189244 | 255765 | 1 |
| BRACE3006917 | 0.001189244 | 235972 | 1 |
| BRACE3007058 | 0.002284523 | 135281 | 2 |
| BRACE3007084 | 0.001189244 | 160259 | 1 |
| BRACE3007085 | 0.001189244 | 172323 | 1 |
| BRACE3007258 | 0.012875282 | 6581 | 8 |
| BRACE3007322 | 0.001189244 | 275240 | 1 |
| BRACE3007472 | 0.001189244 | 258709 | 1 |
| BRACE3007480 | 0.025816447 | 128624 | 16 |
| BRACE3007559 | 0.001189244 | 229380 | 1 |
| BRACE3007625 | 0.008324711 | 131927 | 7 |
| BRACE3007642 | 0.001189244 | 161691 | 1 |
| BRACE3007649 | 0.002378489 | 249540 | 2 |
| BRACE3007767 | 0.001189244 | 247436 | 1 |
| BRACE3007869 | 0.001189244 | 199684 | 1 |
| BRACE3008036 | 0.009079841 | 191467 | 3 |
| BRACE3008066 | 0.001189244 | 2300 | 1 |
| BRACE3008092 | 0.001189244 | 194640 | 1 |
| BRACE3008095 | 0.0025864 | 101779 | 2 |
| BRACE3008137 | 0.001189244 | 64407 | 1 |
| BRACE3008238 | 0.036215441 | 100290 | 21 |
| BRACE3008255 | 0.001189244 | 174065 | 1 |
| BRACE3008298 | 0.038198657 | 124033 | 19 |
| BRACE3008337 | 0.001189244 | 228948 | 1 |
| BRACE3008384 | 0.004236603 | 188789 | 3 |
| BRACE3008491 | 0.107321308 | 43537 | 34 |
| BRACE3008720 | 0.001189244 | 180099 | 1 |
| BRACE3008772 | 0.003187646 | 207047 | 2 |
| BRACE3009044 | 0.001189244 | 153369 | 1 |
| BRACE3009075 | 0.001189244 | 238861 | 1 |
| BRACE3009090 | 0.001189244 | 218553 | 1 |
| BRACE3009127 | 0.001189244 | 91612 | 1 |
| BRACE3009197 | 0.001189244 | 238873 | 1 |
| BRACE3009237 | 0.009237104 | 182663 | 6 |
| BRACE3009265 | 0.001189244 | 277243 | 1 |
| BRACE3009297 | 0.001189244 | 116554 | 1 |
| BRACE3009377 | 0.001189244 | 244308 | 1 |
| BRACE3009392 | 0.001189244 | 251948 | 1 |
| BRACE3009416 | 0.005870825 | 229667 | 4 |
| BRACE3009539 | 0.001189244 | 184910 | 1 |
| BRACE3009574 | 0.001189244 | 199561 | 1 |
| BRACE3009701 | 0.001189244 | 252788 | 1 |
| BRACE3009708 | 0.001189244 | 78624 | 1 |
| BRACE3009724 | 0.002864792 | 164137 | 2 |
| BRACE3009747 | 0.074889021 | 89002 | 14 |
| BRACE3009993 | 0.001189244 | 242139 | 1 |
| BRACE3010076 | 0.001189244 | 251989 | 1 |
| BRACE3010079 | 0.001189244 | 240749 | 1 |
| BRACE3010086 | 0.001189244 | 232264 | 1 |
| BRACE3010221 | 0.002378489 | 234204 | 2 |
| BRACE3010283 | 0.003066086 | 122361 | 2 |
| BRACE3010315 | 0.001189244 | 246165 | 1 |
| BRACE3010397 | 0.004905473 | 206862 | 3 |
| BRACE3010416 | 0.001189244 | 277833 | 1 |
| BRACE3010428 | 0.146373937 | 116230 | 44 |
| BRACE3010435 | 0.001189244 | 236289 | 1 |
| BRACE3010438 | 0.093572816 | 44400 | 21 |
| BRACE3010702 | 0.001189244 | 244257 | 1 |
| BRACE3010862 | 0.001189244 | 250450 | 1 |
| BRACE3011271 | 0.01518253 | 35201 | 10 |
| BRACE3011421 | 0.005930878 | 82580 | 4 |
| BRACE3011447 | 0.001189244 | 226423 | 1 |
| BRACE3011502 | 0.064024495 | 110806 | 23 |
| BRACE3011505 | 0.001189244 | 206641 | 1 |
| BRACE3011634 | 0.001189244 | 250490 | 1 |
| BRACE3011774 | 0.001189244 | 167245 | 1 |
| BRACE3012043 | 0.009338148 | 133794 | 5 |
| BRACE3012357 | 0.002378489 | 143987 | 2 |
| BRACE3012364 | 0.039395015 | 121642 | 19 |
| BRACE3012574 | 0.001189244 | 152013 | 1 |
| BRACE3012806 | 0.0025864 | 139436 | 2 |
| BRACE3012930 | 0.003066086 | 158250 | 2 |
| BRACE3013119 | 0.001189244 | 274622 | 1 |
| BRACE3013418 | 0.002378489 | 118255 | 2 |
| BRACE3013576 | 0.001189244 | 283925 | 1 |
| BRACE3013740 | 0.001189244 | 139616 | 1 |
| BRACE3013780 | 0.034934141 | 107332 | 6 |
| BRACE3013874 | 0.001189244 | 279648 | 1 |
| BRACE3013936 | 0.005499295 | 105973 | 3 |
| BRACE3013986 | 0.001189244 | 258980 | 1 |
| BRACE3014005 | 0.001189244 | 258982 | 1 |
| BRACE3014068 | 0.001189244 | 240010 | 1 |
| BRACE3014231 | 0.001189244 | 250592 | 1 |
| BRACE3014290 | 0.002378489 | 197547 | 2 |
| BRACE3014317 | 0.001189244 | 102335 | 1 |
| BRACE3014523 | 0.001189244 | 61325 | 1 |
| BRACE3014714 | 0.001189244 | 140386 | 1 |
| BRACE3014807 | 0.001189244 | 175809 | 1 |
| BRACE3014942 | 0.001189244 | 148478 | 1 |
| BRACE3015027 | 0.064566514 | 117326 | 12 |
| BRACE3015090 | 0.001189244 | 129069 | 1 |
| BRACE3015121 | 0.001189244 | 138155 | 1 |
| BRACE3015262 | 0.002378489 | 257963 | 2 |
| BRACE3015521 | 0.001189244 | 258322 | 1 |
| BRACE3015808 | 0.001189244 | 228723 | 1 |
| BRACE3015829 | 0.001189244 | 237508 | 1 |
| BRACE3015894 | 0.001189244 | 148524 | 1 |
| BRACE3015898 | 0.001189244 | 228760 | 1 |
| BRACE3016020 | 0.001189244 | 144653 | 1 |
| BRACE3016122 | 0.002864792 | 118093 | 2 |
| BRACE3016167 | 0.001189244 | 244536 | 1 |
| BRACE3016170 | 0.001189244 | 244270 | 1 |
| BRACE3016580 | 0.001189244 | 227829 | 1 |
| BRACE3016788 | 0.001189244 | 248860 | 1 |
| BRACE3016810 | 0.001189244 | 249727 | 1 |
| BRACE3016862 | 0.001189244 | 229470 | 1 |
| BRACE3016884 | 0.025952275 | 211347 | 4 |
| BRACE3017083 | 0.001189244 | 235213 | 1 |
| BRACE3017253 | 0.004255331 | 234499 | 3 |
| BRACE3018083 | 0.001189244 | 235175 | 1 |
| BRACE3018308 | 0.001189244 | 222009 | 1 |
| BRACE3018963 | 0.002898705 | 136132 | 2 |
| BRACE3019055 | 0.002378489 | 166429 | 2 |
| BRACE3019071 | 0.00591215 | 205684 | 4 |
| BRACE3019084 | 0.001189244 | 199688 | 1 |
| BRACE3019570 | 0.001189244 | 124762 | 1 |
| BRACE3019611 | 0.001189244 | 258177 | 1 |
| BRACE3019817 | 0.001189244 | 135429 | 1 |
| BRACE3019828 | 0.001189244 | 136479 | 1 |
| BRACE3019941 | 0.057350561 | 101871 | 13 |
| BRACE3020151 | 0.001189244 | 164354 | 1 |
| BRACE3020194 | 0.001189244 | 118031 | 1 |
| BRACE3020286 | 0.001189244 | 276135 | 1 |
| BRACE3020356 | 0.001189244 | 241825 | 1 |
| BRACE3020594 | 0.001189244 | 227580 | 1 |
| BRACE3020669 | 0.003066086 | 117372 | 2 |
| BRACE3020671 | 0.001189244 | 273251 | 1 |
| BRACE3020890 | 0.001189244 | 246702 | 1 |
| BRACE3021148 | 0.001189244 | 229888 | 1 |
| BRACE3021430 | 0.014045078 | 121125 | 3 |
| BRACE3021517 | 0.001189244 | 183991 | 1 |
| BRACE3021805 | 0.001189244 | 258470 | 1 |
| BRACE3022051 | 0.001189244 | 238809 | 1 |
| BRACE3022303 | 0.001189244 | 247697 | 1 |
| BRACE3022312 | 0.004924201 | 137816 | 3 |
| BRACE3022340 | 0.002864792 | 124763 | 2 |
| BRACE3022769 | 0.117309152 | 99020 | 46 |
| BRACE3022847 | 0.001189244 | 214850 | 1 |
| BRACE3023604 | 0.001189244 | 269992 | 1 |
| BRACE3023912 | 0.001189244 | 189694 | 1 |
| BRACE3024073 | 0.001189244 | 82623 | 1 |
| BRACE3024359 | 0.001189244 | 175638 | 1 |
| BRACE3024379 | 0.001189244 | 283649 | 1 |
| BRACE3024444 | 0.001189244 | 217297 | 1 |
| BRACE3024497 | 0.001189244 | 235096 | 1 |
| BRACE3024537 | 0.001189244 | 79454 | 1 |
| BRACE3024659 | 0.001189244 | 74718 | 1 |
| BRACE3024662 | 0.001189244 | 198906 | 1 |
| BRACE3024879 | 0.001189244 | 164449 | 1 |
| BRACE3025153 | 0.001189244 | 154384 | 1 |
| BRACE3025302 | 0.001189244 | 137650 | 1 |
| BRACE3025457 | 0.001189244 | 145581 | 1 |
| BRACE3025531 | 0.001189244 | 126933 | 1 |
| BRACE3025627 | 0.001189244 | 149321 | 1 |
| BRACE3025630 | 0.003567733 | 148647 | 3 |
| BRACE3025719 | 0.009302444 | 53572 | 3 |
| BRACE3026008 | 0.001189244 | 122419 | 1 |
| BRACE3026075 | 0.001189244 | 126932 | 1 |
| BRACE3026161 | 0.001189244 | 171340 | 1 |
| BRACE3026290 | 0.001189244 | 124589 | 1 |
| BRACE3026345 | 0.001189244 | 246787 | 1 |
| BRACE3026456 | 0.002378489 | 214257 | 2 |
| BRACE3026735 | 0.006931557 | 163414 | 5 |
| BRACE3026802 | 0.009804091 | 64897 | 4 |
| BRACE3026844 | 0.001189244 | 246661 | 1 |
| BRACE3026947 | 0.001189244 | 256532 | 1 |
| BRACE3026993 | 0.002378489 | 158252 | 2 |
| BRACE3027242 | 0.001189244 | 214578 | 1 |
| BRACE3027256 | 0.001189244 | 205914 | 1 |
| BRACE3027326 | 0.001189244 | 171329 | 1 |
| BRACE3027478 | 0.010468993 | 119584 | 6 |
| BRACE3027480 | 0.001189244 | 211484 | 1 |
| BRACE3027931 | 0.004980217 | 110747 | 3 |
| BRACE3027976 | 0.0025864 | 108599 | 2 |
| BRACE3028214 | 0.001189244 | 115909 | 1 |
| BRACE3028241 | 0.001189244 | 161249 | 1 |
| BRACE3028360 | 0.001189244 | 181059 | 1 |
| BRACE3028895 | 0.003681678 | 28758 | 3 |
| BRACE3028998 | 0.001189244 | 134522 | 1 |
| BRACE3029005 | 0.001189244 | 141654 | 1 |
| BRACE3029021 | 0.004608165 | 91068 | 3 |
| BRACE3029205 | 0.001189244 | 134452 | 1 |
| BRACE3029447 | 0.001189244 | 148048 | 1 |
| BRACE3030057 | 0.001189244 | 69400 | 1 |
| BRACE3030103 | 0.001189244 | 180187 | 1 |
| BRACE3030538 | 0.003270756 | 102592 | 2 |
| BRACE3030748 | 0.001189244 | 145766 | 1 |
| BRACE3030866 | 0.002864792 | 82596 | 2 |
| BRACE3031161 | 0.001189244 | 145480 | 1 |
| BRACE3031184 | 0.001189244 | 132373 | 1 |
| BRACE3031185 | 0.002864792 | 125436 | 2 |
| BRACE3031315 | 0.002877521 | 244358 | 2 |
| BRACE3031372 | 0.001189244 | 249159 | 1 |
| BRACE3031579 | 0.001189244 | 109237 | 1 |
| BRACE3031728 | 0.001189244 | 241878 | 1 |
| BRACE3031743 | 0.030714937 | 71298 | 12 |
| BRACE3031838 | 0.002864792 | 252119 | 2 |
| BRACE3031843 | 0.001189244 | 221115 | 1 |
| BRACE3032385 | 0.024506169 | 132139 | 14 |
| BRACE3032427 | 0.002864792 | 195440 | 2 |
| BRACE3032455 | 0.001189244 | 261202 | 1 |
| BRACE3032537 | 0.001189244 | 240808 | 1 |
| BRACE3032538 | 0.001189244 | 164141 | 1 |
| BRACE3032631 | 0.013285472 | 129386 | 5 |
| BRACE3032771 | 0.001189244 | 283486 | 1 |
| BRACE3032980 | 0.001189244 | 260415 | 1 |
| BRACE3032983 | 0.001189244 | 187643 | 1 |
| BRACE3033525 | 0.001189244 | 266837 | 1 |
| BRACE3034159 | 0.001189244 | 175485 | 1 |
| BRACE3034183 | 0.001189244 | 184775 | 1 |
| BRACE3034389 | 0.001189244 | 6570 | 1 |
| BRACE3034964 | 0.001189244 | 35141 | 1 |
| BRACE3034993 | 0.001189244 | 250584 | 1 |
| BRACE3035168 | 0.003983555 | 139342 | 3 |
| BRACE3035227 | 0.001189244 | 123021 | 1 |
| BRACE3036156 | 0.007655524 | 30705 | 5 |
| BRACE3036271 | 0.001189244 | 113895 | 1 |
| BRACE3036283 | 0.022448721 | 104123 | 8 |
| BRACE3037612 | 0.001189244 | 187772 | 1 |
| BRACE3037637 | 0.001189244 | 222659 | 1 |
| BRACE3037803 | 0.001189244 | 177702 | 1 |
| BRACE3038012 | 0.001189244 | 214713 | 1 |
| BRACE3038030 | 0.001189244 | 248786 | 1 |
| BRACE3038569 | 0.001189244 | 264527 | 1 |
| BRACE3038570 | 0.001189244 | 263137 | 1 |
| BRACE3038687 | 0.001189244 | 279931 | 1 |
| BRACE3038760 | 0.001189244 | 269558 | 1 |
| BRACE3039288 | 0.001189244 | 220816 | 1 |
| BRACE3039358 | 0.008107913 | 118354 | 5 |
| BRACE3039378 | 0.002378489 | 90659 | 2 |
| BRACE3039454 | 0.001189244 | 284195 | 1 |
| BRACE3040012 | 0.001189244 | 260452 | 1 |
| BRACE3040239 | 0.001189244 | 279874 | 1 |
| BRACE3040504 | 0.001189244 | 200637 | 1 |
| BRACE3040644 | 0.001189244 | 262790 | 1 |
| BRACE3040856 | 0.001189244 | 264498 | 1 |
| BRACE3040863 | 0.009366964 | 155117 | 6 |
| BRACE3041059 | 0.001189244 | 273044 | 1 |
| BRACE3041162 | 0.001189244 | 231432 | 1 |
| BRACE3041827 | 0.001189244 | 256379 | 1 |
| BRACE3042046 | 0.001189244 | 165416 | 1 |
| BRACE3042210 | 0.001189244 | 260938 | 1 |
| BRACE3042326 | 0.004775546 | 63088 | 3 |
| BRACE3042409 | 0.001189244 | 227217 | 1 |
| BRACE3042432 | 0.002864792 | 260685 | 2 |
| BRACE3042594 | 0.001189244 | 263963 | 1 |
| BRACE3043597 | 0.001189244 | 225982 | 1 |
| BRACE3044090 | 0.001189244 | 282897 | 1 |
| BRACE3044172 | 0.001189244 | 272396 | 1 |
| BRACE3044247 | 0.001189244 | 262335 | 1 |
| BRACE3044377 | 0.001189244 | 256842 | 1 |
| BRACE3044389 | 0.001189244 | 252401 | 1 |
| BRACE3044495 | 0.001189244 | 280557 | 1 |
| BRACE3045011 | 0.001189244 | 231521 | 1 |
| BRACE3045033 | 0.001189244 | 127483 | 1 |
| BRACE3045078 | 0.004087949 | 192835 | 3 |
| BRACE3045145 | 0.001189244 | 284077 | 1 |
| BRACE3045424 | 0.001189244 | 269869 | 1 |
| BRACE3045708 | 0.001189244 | 241816 | 1 |
| BRACE3045981 | 0.006338559 | 71048 | 5 |
| BRACE3046049 | 0.001189244 | 278286 | 1 |
| BRACE3046152 | 0.001189244 | 214332 | 1 |
| BRACE3046294 | 0.001189244 | 203297 | 1 |
| BRACE3046450 | 0.007621769 | 64401 | 6 |
| BRACE3046466 | 0.001189244 | 270869 | 1 |
| BRACE3046491 | 0.001189244 | 79387 | 1 |
| BRACE3046609 | 0.001189244 | 250690 | 1 |
| BRACE3046762 | 0.001189244 | 278219 | 1 |
| BRACE3046837 | 0.001189244 | 123388 | 1 |
| BRACE3046855 | 0.001189244 | 223302 | 1 |
| BRACE3046966 | 0.001189244 | 283072 | 1 |
| BRACE3047018 | 0.001189244 | 262974 | 1 |
| BRACE3047482 | 0.001189244 | 284339 | 1 |
| BRACE3047801 | 0.001189244 | 91325 | 1 |
| BRALZ1000047 | 0.006054368 | 209425 | 1 |
| BRALZ1000103 | 0.006054368 | 208787 | 1 |
| BRALZ2000189 | 0.006054368 | 37983 | 1 |
| BRALZ2000263 | 0.007243613 | 128577 | 2 |
| BRALZ2000988 | 0.03228223 | 116246 | 16 |
| BRALZ2001038 | 0.010309699 | 78090 | 4 |
| BRALZ2001279 | 0.012108736 | 99816 | 2 |
| BRALZ2001350 | 0.012108736 | 58272 | 2 |
| BRALZ2001445 | 0.006054368 | 101234 | 1 |
| BRALZ2001559 | 0.006054368 | 181345 | 1 |
| BRALZ2001743 | 0.006054368 | 65342 | 1 |
| BRALZ2001834 | 0.007912483 | 100135 | 2 |
| BRALZ2001966 | 0.012215794 | 183591 | 4 |
| BRALZ2002174 | 0.006054368 | 84725 | 1 |
| BRALZ2002418 | 0.007742645 | 124647 | 2 |
| BRALZ2002477 | 0.006054368 | 200027 | 1 |
| BRALZ2003119 | 0.006054368 | 97588 | 1 |
| BRALZ2003330 | 0.006054368 | 262294 | 1 |
| BRALZ2003453 | 0.006054368 | 236852 | 1 |
| BRALZ2003525 | 0.006054368 | 152942 | 1 |
| BRALZ2004397 | 0.006054368 | 172185 | 1 |
| BRALZ2005467 | 0.006054368 | 121905 | 1 |
| BRALZ2005471 | 0.006054368 | 79624 | 1 |
| BRALZ2005888 | 0.006054368 | 45044 | 1 |
| BRALZ2005950 | 0.006054368 | 205321 | 1 |
| BRALZ2005992 | 0.006054368 | 237256 | 1 |
| BRALZ2006288 | 0.015364717 | 130927 | 4 |
| BRALZ2006474 | 0.006054368 | 225106 | 1 |
| BRALZ2006560 | 0.006054368 | 227174 | 1 |
| BRALZ2006734 | 0.006054368 | 227194 | 1 |
| BRALZ2006800 | 0.012108736 | 249789 | 2 |
| BRALZ2006976 | 0.006054368 | 252208 | 1 |
| BRALZ2007376 | 0.006054368 | 244791 | 1 |
| BRALZ2007545 | 0.006054368 | 248296 | 1 |
| BRALZ2007576 | 0.006054368 | 136757 | 1 |
| BRALZ2007661 | 0.006054368 | 232125 | 1 |
| BRALZ2007790 | 0.006054368 | 7312 | 1 |
| BRALZ2007793 | 0.006054368 | 132826 | 1 |
| BRALZ2007952 | 0.006054368 | 203925 | 1 |
| BRALZ2008031 | 0.006054368 | 263450 | 1 |
| BRALZ2008166 | 0.009149864 | 100033 | 2 |
| BRALZ2008462 | 0.006054368 | 116596 | 1 |
| BRALZ2008484 | 0.015675457 | 144794 | 6 |
| BRALZ2008617 | 0.020679715 | 78975 | 4 |
| BRALZ2008696 | 0.007729915 | 116307 | 2 |
| BRALZ2008763 | 0.006054368 | 248650 | 1 |
| BRALZ2008869 | 0.006054368 | 251314 | 1 |
| BRALZ2008930 | 0.006054368 | 253441 | 1 |
| BRALZ2009446 | 0.035935332 | 95658 | 6 |
| BRALZ2009482 | 0.450889643 | 88477 | 55 |
| BRALZ2009486 | 0.01595282 | 90054 | 3 |
| BRALZ2010186 | 0.006054368 | 224258 | 1 |
| BRALZ2010842 | 0.006054368 | 202363 | 1 |
| BRALZ2011337 | 0.006054368 | 215681 | 1 |
| BRALZ2011796 | 0.030400931 | 42915 | 8 |
| BRALZ2011880 | 0.054955421 | 135349 | 10 |
| BRALZ2012050 | 0.006054368 | 256724 | 1 |
| BRALZ2012183 | 0.006054368 | 239539 | 1 |
| BRALZ2012848 | 0.006054368 | 244734 | 1 |
| BRALZ2013621 | 0.006054368 | 95352 | 1 |
| BRALZ2013690 | 0.006054368 | 112266 | 1 |
| BRALZ2014054 | 0.015763106 | 65654 | 2 |
| BRALZ2014316 | 0.006054368 | 233292 | 1 |
| BRALZ2014484 | 0.006054368 | 235211 | 1 |
| BRALZ2014951 | 0.006054368 | 202173 | 1 |
| BRALZ2015168 | 0.006054368 | 235217 | 1 |
| BRALZ2015811 | 0.006054368 | 224629 | 1 |
| BRALZ2016085 | 0.006054368 | 118047 | 1 |
| BRALZ2016498 | 0.006054368 | 224685 | 1 |
| BRALZ2017359 | 0.00805277 | 101670 | 2 |
| BRALZ2017531 | 0.006054368 | 76811 | 1 |
| BRALZ2017607 | 0.006054368 | 251769 | 1 |
| BRALZ2017620 | 0.006054368 | 256462 | 1 |
| BRALZ2017844 | 0.012256688 | 163079 | 2 |
| BRALZ2018342 | 0.006054368 | 214159 | 1 |
| BRALZ2018492 | 0.007243613 | 133939 | 2 |
| BRALZ2018834 | 0.026851826 | 172207 | 5 |
| BRALZ2019047 | 0.007912483 | 237762 | 2 |
| BRAMY1000095 | 0.001688277 | 111454 | 1 |
| BRAMY1000098 | 0.001688277 | 278339 | 1 |
| BRAMY1000130 | 0.013910227 | 174420 | 2 |
| BRAMY1000157 | 0.001688277 | 148531 | 1 |
| BRAMY1000173 | 0.015844282 | 102816 | 8 |
| BRAMY2000021 | 0.001688277 | 262947 | 1 |
| BRAMY2000025 | 0.010618374 | 142852 | 5 |
| BRAMY2000052 | 0.015472487 | 148977 | 4 |
| BRAMY2000076 | 0.003376553 | 157164 | 2 |
| BRAMY2000086 | 0.001688277 | 276108 | 1 |
| BRAMY2000151 | 0.007600539 | 66132 | 2 |
| BRAMY2000181 | 0.004783773 | 101609 | 2 |
| BRAMY2000196 | 0.001688277 | 95538 | 1 |
| BRAMY2000231 | 0.004603134 | 273607 | 2 |
| BRAMY2000277 | 0.001688277 | 205638 | 1 |
| BRAMY2000354 | 0.121936009 | 52892 | 18 |
| BRAMY2000388 | 0.027386151 | 127133 | 9 |
| BRAMY2000411 | 0.027034061 | 124231 | 8 |
| BRAMY2000508 | 0.004760979 | 2414 | 3 |
| BRAMY2000562 | 0.087191067 | 97390 | 26 |
| BRAMY2000585 | 0.068220966 | 148164 | 7 |
| BRAMY2000653 | 0.001688277 | 151864 | 1 |
| BRAMY2000814 | 0.007890596 | 131263 | 2 |
| BRAMY2000893 | 0.110106451 | 110629 | 9 |
| BRAMY2000946 | 0.001688277 | 153309 | 1 |
| BRAMY2000981 | 0.001688277 | 226304 | 1 |
| BRAMY2000987 | 0.009871944 | 8481 | 4 |
| BRAMY2001068 | 0.001688277 | 77225 | 1 |
| BRAMY2001114 | 0.001688277 | 94481 | 1 |
| BRAMY2001203 | 0.001688277 | 174295 | 1 |
| BRAMY2001282 | 0.001688277 | 85700 | 1 |
| BRAMY2001367 | 0.001688277 | 91656 | 1 |
| BRAMY2001427 | 0.001688277 | 149801 | 1 |
| BRAMY2001473 | 0.001688277 | 158269 | 1 |
| BRAMY2001678 | 0.004482587 | 91076 | 3 |
| BRAMY2002005 | 0.001688277 | 184946 | 1 |
| BRAMY2002044 | 0.003376553 | 101735 | 2 |
| BRAMY2002072 | 0.001688277 | 120931 | 1 |
| BRAMY2002158 | 0.001688277 | 162620 | 1 |
| BRAMY2002311 | 0.001688277 | 169939 | 1 |
| BRAMY2002339 | 0.001688277 | 274751 | 1 |
| BRAMY2002364 | 0.001688277 | 79012 | 1 |
| BRAMY2002369 | 0.020538448 | 66030 | 11 |
| BRAMY2002479 | 0.007729843 | 159751 | 2 |
| BRAMY2002493 | 0.001688277 | 166922 | 1 |
| BRAMY2002525 | 0.002877521 | 132126 | 2 |
| BRAMY2002584 | 0.001688277 | 168278 | 1 |
| BRAMY2002604 | 0.001688277 | 129321 | 1 |
| BRAMY2002621 | 0.004735635 | 153424 | 3 |
| BRAMY2002638 | 0.001688277 | 48990 | 1 |
| BRAMY2002739 | 0.001688277 | 276200 | 1 |
| BRAMY2002799 | 0.003363824 | 157940 | 2 |
| BRAMY2002853 | 0.001688277 | 138334 | 1 |
| BRAMY2002862 | 0.001688277 | 176836 | 1 |
| BRAMY2002956 | 0.001688277 | 181661 | 1 |
| BRAMY2003008 | 0.006335944 | 102377 | 4 |
| BRAMY2003037 | 0.006753106 | 147423 | 4 |
| BRAMY2003117 | 0.001688277 | 21320 | 1 |
| BRAMY2003287 | 0.001688277 | 181687 | 1 |
| BRAMY2003325 | 0.013494652 | 174414 | 2 |
| BRAMY2003529 | 0.001688277 | 188521 | 1 |
| BRAMY2003538 | 0.004565798 | 161463 | 3 |
| BRAMY2003583 | 0.130254954 | 84775 | 43 |
| BRAMY2003585 | 0.001688277 | 180079 | 1 |
| BRAMY2003634 | 0.001688277 | 40418 | 1 |
| BRAMY2003653 | 0.003376553 | 71471 | 2 |
| BRAMY2003681 | 0.001688277 | 278791 | 1 |
| BRAMY2003893 | 0.001688277 | 154086 | 1 |
| BRAMY2003897 | 0.001688277 | 39871 | 1 |
| BRAMY2003898 | 0.001688277 | 7501 | 1 |
| BRAMY2003904 | 0.001688277 | 273224 | 1 |
| BRAMY2003926 | 0.001688277 | 207241 | 1 |
| BRAMY2003929 | 0.007879269 | 175730 | 3 |
| BRAMY2004031 | 0.013177596 | 22981 | 4 |
| BRAMY2004058 | 0.001688277 | 235378 | 1 |
| BRAMY2004183 | 0.001688277 | 30938 | 1 |
| BRAMY2004335 | 0.001688277 | 194915 | 1 |
| BRAMY2004351 | 0.001688277 | 128355 | 1 |
| BRAMY2004352 | 0.001688277 | 188782 | 1 |
| BRAMY2004363 | 0.081959587 | 57294 | 38 |
| BRAMY2004387 | 0.005532361 | 213336 | 2 |
| BRAMY2004492 | 0.001688277 | 117969 | 1 |
| BRAMY2004521 | 0.070690827 | 118887 | 21 |
| BRAMY2004524 | 0.007729843 | 104031 | 2 |
| BRAMY2004537 | 0.001688277 | 190262 | 1 |
| BRAMY2004542 | 0.001688277 | 181836 | 1 |
| BRAMY2004756 | 0.001688277 | 86339 | 1 |
| BRAMY2004771 | 0.001688277 | 114280 | 1 |
| BRAMY2005052 | 0.014429405 | 122494 | 9 |
| BRAMY2005064 | 0.001688277 | 252478 | 1 |
| BRAMY2005094 | 0.001688277 | 171186 | 1 |
| BRAMY2005151 | 0.001688277 | 195123 | 1 |
| BRAMY2005182 | 0.001688277 | 126136 | 1 |
| BRAMY2005244 | 0.001688277 | 145878 | 1 |
| BRAMY2005391 | 0.001688277 | 89031 | 1 |
| BRAMY2005488 | 0.014703466 | 153630 | 7 |
| BRAMY2005576 | 0.007890596 | 131572 | 2 |
| BRAMY2005648 | 0.001688277 | 203292 | 1 |
| BRAMY2005662 | 0.001688277 | 76598 | 1 |
| BRAMY2005684 | 0.022570023 | 133371 | 10 |
| BRAMY2005708 | 0.001688277 | 185498 | 1 |
| BRAMY2005711 | 0.001688277 | 203299 | 1 |
| BRAMY2006092 | 0.001688277 | 229856 | 1 |
| BRAMY2006118 | 0.001688277 | 168650 | 1 |
| BRAMY2006149 | 0.001688277 | 181621 | 1 |
| BRAMY2006366 | 0.018188047 | 129951 | 4 |
| BRAMY2006375 | 0.007068987 | 72288 | 5 |
| BRAMY2006397 | 0.059666056 | 125572 | 6 |
| BRAMY2006411 | 0.001688277 | 125831 | 1 |
| BRAMY2006459 | 0.001688277 | 95693 | 1 |
| BRAMY2006466 | 0.001688277 | 146568 | 1 |
| BRAMY2006659 | 0.001688277 | 237010 | 1 |
| BRAMY2006779 | 0.001688277 | 19868 | 1 |
| BRAMY2006809 | 0.001688277 | 36409 | 1 |
| BRAMY2006879 | 0.001688277 | 87546 | 1 |
| BRAMY2007043 | 0.001688277 | 126414 | 1 |
| BRAMY2007078 | 0. 044062731 | 126642 | 8 |
| BRAMY2007110 | 0.001688277 | 208844 | 1 |
| BRAMY2007185 | 0.001688277 | 223397 | 1 |
| BRAMY2007244 | 0.014296277 | 135445 | 6 |
| BRAMY2007249 | 0.001688277 | 198536 | 1 |
| BRAMY2007255 | 0.00848375 | 83492 | 5 |
| BRAMY2007287 | 0.015637282 | 84744 | 4 |
| BRAMY2007308 | 0.001688277 | 204726 | 1 |
| BRAMY2007411 | 0.001688277 | 118549 | 1 |
| BRAMY2007458 | 0.001688277 | 149654 | 1 |
| BRAMY2007486 | 0.001688277 | 266661 | 1 |
| BRAMY2007546 | 0.001688277 | 49131 | 1 |
| BRAMY2007567 | 0.021593198 | 22941 | 12 |
| BRAMY2007594 | 0.005404505 | 38055 | 3 |
| BRAMY2007610 | 0.001688277 | 114780 | 1 |
| BRAMY2007613 | 0.125082366 | 84438 | 62 |
| BRAMY2007653 | 0.001688277 | 149732 | 1 |
| BRAMY2007753 | 0.001688277 | 132915 | 1 |
| BRAMY2007859 | 0.001688277 | 263143 | 1 |
| BRAMY2008333 | 0.001688277 | 213557 | 1 |
| BRAMY2008382 | 0.010805541 | 134302 | 3 |
| BRAMY2008563 | 0.003397737 | 88603 | 2 |
| BRAMY2008583 | 0.001688277 | 11191 | 1 |
| BRAMY2008977 | 0.033139054 | 126239 | 6 |
| BRAMY2008979 | 0.001688277 | 99011 | 1 |
| BRAMY2009013 | 0.027510006 | 107963 | 13 |
| BRAMY2009023 | 0.012638846 | 49221 | 5 |
| BRAMY2009034 | 0.001688277 | 241671 | 1 |
| BRAMY2009105 | 0.001688277 | 222178 | 1 |
| BRAMY2009123 | 0.003686678 | 43124 | 2 |
| BRAMY2009192 | 0.001688277 | 213779 | 1 |
| BRAMY2009344 | 0. 001688277 | 82133 | 1 |
| BRAMY2009349 | 0.001688277 | 124273 | 1 |
| BRAMY2009394 | 0.001688277 | 18397 | 1 |
| BRAMY2009489 | 0.009322617 | 129119 | 6 |
| BRAMY2009508 | 0.022346414 | 74704 | 14 |
| BRAMY2009528 | 0.001688277 | 63123 | 1 |
| BRAMY2009557 | 0.003397737 | 153062 | 2 |
| BRAMY2009612 | 0.012379628 | 55151 | 5 |
| BRAMY2009693 | 0.001688277 | 14706 | 1 |
| BRAMY2009934 | 0.028343935 | 165950 | 7 |
| BRAMY2010068 | 0.001688277 | 207845 | 1 |
| BRAMY2010135 | 0.001688277 | 149065 | 1 |
| BRAMY2010145 | 0.001688277 | 159445 | 1 |
| BRAMY2010171 | 0.001688277 | 149965 | 1 |
| BRAMY2010208 | 0.009218016 | 174692 | 3 |
| BRAMY2010239 | 0.001688277 | 159075 | 1 |
| BRAMY2010272 | 0.003363824 | 18745 | 2 |
| BRAMY2010290 | 0.001688277 | 185818 | 1 |
| BRAMY2010317 | 0.142290854 | 110977 | 54 |
| BRAMY2010357 | 0.011221561 | 105629 | 7 |
| BRAMY2010366 | 0.001688277 | 15618 | 1 |
| BRAMY2010395 | 0.001688277 | 222421 | 1 |
| BRAMY2010441 | 0.001688277 | 91575 | 1 |
| BRAMY2010442 | 0.003546391 | 215612 | 2 |
| BRAMY2010464 | 0.015208629 | 123638 | 8 |
| BRAMY2010581 | 0.001688277 | 179317 | 1 |
| BRAMY2010798 | 0.001688277 | 181265 | 1 |
| BRAMY2010808 | 0.001688277 | 165804 | 1 |
| BRAMY2010919 | 0.001688277 | 179619 | 1 |
| BRAMY2011064 | 0.001688277 | 147688 | 1 |
| BRAMY2011105 | 0.055312338 | 155765 | 5 |
| BRAMY2011178 | 0.001688277 | 126580 | 1 |
| BRAMY2011196 | 0.001688277 | 270166 | 1 |
| BRAMY2011216 | 0.001688277 | 78961 | 1 |
| BRAMY2011253 | 0.001688277 | 185381 | 1 |
| BRAMY2011280 | 0.10132157 | 79371 | 45 |
| BRAMY2011297 | 0.408013258 | 131485 | 22 |
| BRAMY2011446 | 0.004735635 | 99446 | 3 |
| BRAMY2011480 | 0.001688277 | 86064 | 1 |
| BRAMY2011639 | 0.001688277 | 231626 | 1 |
| BRAMY2011679 | 0.001688277 | 70054 | 1 |
| BRAMY2011767 | 0.080407312 | 97605 | 30 |
| BRAMY2011811 | 0.001688277 | 207642 | 1 |
| BRAMY2011841 | 0.01518463 | 154555 | 3 |
| BRAMY2011846 | 0.001688277 | 207572 | 1 |
| BRAMY2011849 | 0.003397737 | 207406 | 2 |
| BRAMY2011872 | 0.001688277 | 146966 | 1 |
| BRAMY2011961 | 0.001688277 | 159663 | 1 |
| BRAMY2012017 | 0.001688277 | 182088 | 1 |
| BRAMY2012091 | 0.028216948 | 159513 | 5 |
| BRAMY2012119 | 0.001688277 | 183683 | 1 |
| BRAMY2012162 | 0.001688277 | 134104 | 1 |
| BRAMY2012163 | 0.021192046 | 162715 | 5 |
| BRAMY2012277 | 0.001688277 | 70995 | 1 |
| BRAMY2012340 | 0.003397737 | 84398 | 2 |
| BRAMY2012426 | 0.02710763 | 61961 | 4 |
| BRAMY2012497 | 0.001688277 | 204093 | 1 |
| BRAMY2012517 | 0.001688277 | 112434 | 1 |
| BRAMY2012536 | 0.290288676 | 36444 | 21 |
| BRAMY2012555 | 0.003376553 | 185592 | 2 |
| BRAMY2012568 | 0.001688277 | 198471 | 1 |
| BRAMY2012581 | 0.007334907 | 39769 | 4 |
| BRAMY2012691 | 0.001688277 | 179817 | 1 |
| BRAMY2012721 | 0.001688277 | 263161 | 1 |
| BRAMY2012731 | 0.001688277 | 266298 | 1 |
| BRAMY2012776 | 0.001688277 | 143687 | 1 |
| BRAMY2012880 | 0.003376553 | 113376 | 2 |
| BRAMY2012881 | 0.003546391 | 65904 | 2 |
| BRAMY2013169 | 0.014735555 | 53312 | 6 |
| BRAMY2013200 | 0.001688277 | 203810 | 1 |
| BRAMY2013216 | 0.001688277 | 210436 | 1 |
| BRAMY2013405 | 0.001688277 | 216963 | 1 |
| BRAMY2013414 | 0.003376553 | 61615 | 2 |
| BRAMY2013572 | 0.001688277 | 254396 | 1 |
| BRAMY2013590 | 0.007755668 | 35084 | 4 |
| BRAMY2013621 | 0.00572656 | 182111 | 2 |
| BRAMY2013659 | 0.00576819 | 155141 | 3 |
| BRAMY2013722 | 0.001688277 | 143226 | 1 |
| BRAMY2013736. | 0.003546391 | 111232 | 2 |
| BRAMY2013756 | 0.008873318 | 149120 | 4 |
| BRAMY2013944 | 0.001688277 | 181579 | 1 |
| BRAMY2013975 | 0.001688277 | 268746 | 1 |
| BRAMY2014019 | 0.001688277 | 177887 | 1 |
| BRAMY2014205 | 0.011694423 | 126460 | 4 |
| BRAMY2014387 | 0.001688277 | 172312 | 1 |
| BRAMY2014462 | 0.003397737 | 108055 | 2 |
| BRAMY2014469 | 0.003376553 | 145453 | 2 |
| BRAMY2014541 | 0.001688277 | 210401 | 1 |
| BRAMY2014754 | 0.119621588 | 6885 | 51 |
| BRAMY2014813 | 0.017368167 | 87665 | 4 |
| BRAMY2015211 | 0.012899034 | 152308 | 4 |
| BRAMY2015251 | 0.05472127 | 94033 | 18 |
| BRAMY2015311 | 0.001688277 | 189178 | 1 |
| BRAMY2015420 | 0.007904162 | 131835 | 5 |
| BRAMY2015503 | 0.001688277 | 92331 | 1 |
| BRAMY2015516 | 0.001688277 | 279553 | 1 |
| BRAMY2015550 | 0.004943546 | 163106 | 3 |
| BRAMY2015551 | 0.001688277 | 69419 | 1 |
| BRAMY2015559 | 0.001688277 | 106643 | 1 |
| BRAMY2015688 | 0.001688277 | 272346 | 1 |
| BRAMY2015705 | 0.00572656 | 128885 | 2 |
| BRAMY2015782 | 0.042787761 | 206855 | 4 |
| BRAMY2015833 | 0.036348251 | 109722 | 12 |
| BRAMY2015855 | 0.002877521 | 148057 | 2 |
| BRAMY2015890 | 0.001688277 | 242770 | 1 |
| BRAMY2015911 | 0.011245968 | 6281 | 4 |
| BRAMY2015925 | 0.001688277 | 230468 | 1 |
| BRAMY2016002 | 0.001688277 | 105854 | 1 |
| BRAMY2016009 | 0.032040691 | 133660 | 16 |
| BRAMY2016020 | 0.003376553 | 225348 | 2 |
| BRAMY2016070 | 0.001688277 | 277068 | 1 |
| BRAMY2016251 | 0.023298868 | 152393 | 10 |
| BRAMY2016325 | 0.044469609 | 82943 | 9 |
| BRAMY2016327 | 0.001688277 | 180047 | 1 |
| BRAMY2016386 | 0.003085432 | 155444 | 2 |
| BRAMY2016611 | 0.261569543 | 75529 | 133 |
| BRAMY2016706 | 0.033805049 | 173354 | 6 |
| BRAMY2016771 | 0.001688277 | 212914 | 1 |
| BRAMY2016892 | 0.011539563 | 97158 | 7 |
| BRAMY2016938 | 0.001688277 | 159919 | 1 |
| BRAMY2016953 | 0.001688277 | 102459 | 1 |
| BRAMY2017014 | 0.001688277 | 150224 | 1 |
| BRAMY2017249 | 0.003376553 | 149613 | 2 |
| BRAMY2017348 | 0.001688277 | 171936 | 1 |
| BRAMY2017450 | 0.001688277 | 205652 | 1 |
| BRAMY2017455 | 0.001688277 | 42527 | 1 |
| BRAMY2017528 | 0.001688277 | 7948 | 1 |
| BRAMY2017864 | 0.012126528 | 118454 | 5 |
| BRAMY2018027 | 0.003376553 | 174496 | 2 |
| BRAMY2018106 | 0.001688277 | 134667 | 1 |
| BRAMY2018122 | 0.013207687 | 17774 | 2 |
| BRAMY2018126 | 0.001688277 | 30015 | 1 |
| BRAMY2018272 | 0.001688277 | 36167 | 1 |
| BRAMY2018273 | 0.001688277 | 190282 | 1 |
| BRAMY2018279 | 0.001688277 | 172766 | 1 |
| BRAMY2018295 | 0.009333512 | 127568 | 3 |
| BRAMY2018308 | 0.001688277 | 36470 | 1 |
| BRAMY2018396 | 0.001688277 | 181631 | 1 |
| BRAMY2018467 | 0.001688277 | 39335 | 1 |
| BRAMY2018635 | 0.006168562 | 124266 | 4 |
| BRAMY2018785 | 0.024638944 | 145238 | 8 |
| BRAMY2019055 | 0.001688277 | 85000 | 1 |
| BRAMY2019111 | 0.005532361 | 186675 | 2 |
| BRAMY2019277 | 0.001688277 | 213756 | 1 |
| BRAMY2019300 | 0.003363824 | 160704 | 2 |
| BRAMY2019468 | 0.044112636 | 116019 | 28 |
| BRAMY2019509 | 0.001688277 | 210006 | 1 |
| BRAMY2019546 | 0.027828217 | 83640 | 14 |
| BRAMY2019554 | 0.209966321 | 34085 | 59 |
| BRAMY2019600 | 0.020752286 | 149067 | 9 |
| BRAMY2019643 | 0.002783555 | 94113 | 2 |
| BRAMY2019710 | 0.003546391 | 164923 | 2 |
| BRAMY2019963 | 0.02460619 | 120171 | 11 |
| BRAMY2019985 | 0.001688277 | 186853 | 1 |
| BRAMY2019989 | 0.173767891 | 97829 | 61 |
| BRAMY2020050 | 0.001688277 | 158682 | 1 |
| BRAMY2020058 | 0.005998327 | 155162 | 3 |
| BRAMY2020106 | 0.014988363 | 171974 | 5 |
| BRAMY2020270 | 0.001688277 | 271061 | 1 |
| BRAMY2020354 | 0.002783555 | 140088 | 2 |
| BRAMY2020355 | 0.001688277 | 222464 | 1 |
| BRAMY2020412 | 0.015608914 | 141693 | 3 |
| BRAMY2020427 | 0.282979705 | 33258 | 37 |
| BRAMY2020445 | 0.001688277 | 145725 | 1 |
| BRAMY2020466 | 0.001688277 | 176243 | 1 |
| BRAMY2020520 | 0.001688277 | 88406 | 1 |
| BRAMY2020574 | 0.001688277 | 188203 | 1 |
| BRAMY2020634 | 0.001688277 | 135463 | 1 |
| BRAMY2020713 | 0.006491413 | 105032 | 3 |
| BRAMY2021040 | 0.001688277 | 164848 | 1 |
| BRAMY2021063 | 0.001688277 | 194124 | 1 |
| BRAMY2021098 | 0.001688277 | 237169 | 1 |
| BRAMY2021139 | 0.006547077 | 126566 | 3 |
| BRAMY2021142 | 0.006257322 | 133382 | 5 |
| BRAMY2021276 | 0.003546391 | 119252 | 2 |
| BRAMY2021310 | 0.013486295 | 164889 | 2 |
| BRAMY2021388 | 0.001688277 | 142648 | 1 |
| BRAMY2021498 | 0.001688277 | 184636 | 1 |
| BRAMY2021523 | 0.007901386 | 202729 | 2 |
| BRAMY2021710 | 0.0222763 | 70935 | 4 |
| BRAMY2021732 | 0.001688277 | 258058 | 1 |
| BRAMY2021746 | 0.001688277 | 4424 | 1 |
| BRAMY2021825 | 0.005086013 | 133624 | 3 |
| BRAMY2021867 | 0.004735635 | 97376 | 3 |
| BRAMY2021962 | 0.001688277 | 67422 | 1 |
| BRAMY2021982 | 0.001688277 | 275972 | 1 |
| BRAMY2022052 | 0.001688277 | 234255 | 1 |
| BRAMY2022160 | 0.013299826 | 45362 | 8 |
| BRAMY2022168 | 0.012386364 | 105685 | 7 |
| BRAMY2022263 | 0.001688277 | 269301 | 1 |
| BRAMY2022301 | 0.001688277 | 239395 | 1 |
| BRAMY2022320 | 0.001688277 | 100685 | 1 |
| BRAMY2022383 | 0.004565798 | 159773 | 3 |
| BRAMY2022445 | 0.003565118 | 176556 | 2 |
| BRAMY2022454 | 0.001688277 | 64167 | 1 |
| BRAMY2022493 | 0.063159019 | 122241 | 13 |
| BRAMY2022525 | 0.00759795 | 122571 | 4 |
| BRAMY2022532 | 0.001688277 | 176617 | 1 |
| BRAMY2022723 | 0.004735635 | 196520 | 3 |
| BRAMY2022786 | 0.134278042 | 6170 | 34 |
| BRAMY2022796 | 0.001688277 | 197503 | 1 |
| BRAMY2022929 | 0.001688277 | 213309 | 1 |
| BRAMY2022980 | 0.010975146 | 148033 | 4 |
| BRAMY2022984 | 0.001688277 | 145187 | 1 |
| BRAMY2023060 | 0.001688277 | 171657 | 1 |
| BRAMY2023110 | 0.001688277 | 109918 | 1 |
| BRAMY2023115 | 0.003397737 | 149720 | 2 |
| BRAMY2023161 | 0.001688277 | 91707 | 1 |
| BRAMY2023172 | 0.001688277 | 279892 | 1 |
| BRAMY2023238 | 0.001688277 | 128693 | 1 |
| BRAMY2023327 | 0.001688277 | 151849 | 1 |
| BRAMY2023358 | 0.001688277 | 143766 | 1 |
| BRAMY2023406 | 0.02537982 | 196749 | 4 |
| BRAMY2023482 | 0.001688277 | 55187 | 1 |
| BRAMY2023514 | 0.001688277 | 130981 | 1 |
| BRAMY2023719 | 0.02802479 | 175044 | 4 |
| BRAMY2023729 | 0.003565118 | 127398 | 2 |
| BRAMY2023786 | 0.001688277 | 18496 | 1 |
| BRAMY2023852 | 0.001688277 | 65047 | 1 |
| BRAMY2023863 | 0.004733158 | 38792 | 2 |
| BRAMY2023939 | 0.027413512 | 149393 | 10 |
| BRAMY2024004 | 0.060577217 | 89481 | 6 |
| BRAMY2024073 | 0.001688277 | 165041 | 1 |
| BRAMY2024247 | 0.015634486 | 152461 | 9 |
| BRAMY2024312 | 0.003397737 | 67516 | 2 |
| BRAMY2024374 | 0.001688277 | 160278 | 1 |
| BRAMY2024390 | 0.004794892 | 121402 | 3 |
| BRAMY2024416 | 0.037029711 | 83172 | 18 |
| BRAMY2024449 | 0.001688277 | 160352 | 1 |
| BRAMY2024489 | 0.003376553 | 169551 | 2 |
| BRAMY2024497 | 0.001688277 | 131172 | 1 |
| BRAMY2024514 | 0.001688277 | 164794 | 1 |
| BRAMY2024530 | 0.001688277 | 14950 | 1 |
| BRAMY2024535 | 0.001688277 | 273806 | 1 |
| BRAMY2024545 | 0.001688277 | 147641 | 1 |
| BRAMY2024576 | 0.001688277 | 144663 | 1 |
| BRAMY2024593 | 0.012997918 | 65991 | 5 |
| BRAMY2024711 | 0.013665617 | 139275 | 3 |
| BRAMY2024715 | 0.013152222 | 153107 | 2 |
| BRAMY2024728 | 0.001688277 | 60144 | 1 |
| BRAMY2024827 | 0.083989576 | 158591 | 8 |
| BRAMY2024849 | 0.001688277 | 175106 | 1 |
| BRAMY2024923 | 0.020081166 | 141389 | 9 |
| BRAMY2025024 | 0.001688277 | 212856 | 1 |
| BRAMY2025032 | 0.001688277 | 190156 | 1 |
| BRAMY2025121 | 0.010119342 | 165766 | 5 |
| BRAMY2025175 | 0.001688277 | 12504 | 1 |
| BRAMY2025218 | 0.001688277 | 152965 | 1 |
| BRAMY2025230 | 0.017089279 | 117181 | 4 |
| BRAMY2025272 | 0.003546391 | 115562 | 2 |
| BRAMY2025277 | 0.001688277 | 112572 | 1 |
| BRAMY2025495 | 0.001688277 | 280148 | 1 |
| BRAMY2025812 | 0.001688277 | 162669 | 1 |
| BRAMY2025996 | 0.001688277 | 230575 | 1 |
| BRAMY2026009 | 0.011267149 | 192537 | 4 |
| BRAMY2026091 | 0.001688277 | 113738 | 1 |
| BRAMY2026168 | 0.005221938 | 76858 | 3 |
| BRAMY2026300 | 0.001688277 | 104670 | 1 |
| BRAMY2026347 | 0.001688277 | 147417 | 1 |
| BRAMY2026395 | 0.001688277 | 157733 | 1 |
| BRAMY2026405 | 0.001688277 | 88728 | 1 |
| BRAMY2026533 | 0.008951281 | 154491 | 4 |
| BRAMY2026538 | 0.003376553 | 91404 | 2 |
| BRAMY2026685 | 0.067607046 | 116703 | 18 |
| BRAMY2026713 | 0.001688277 | 264346 | 1 |
| BRAMY2026778 | 0.034583959 | 148047 | 6 |
| BRAMY2026824 | 0.001688277 | 160595 | 1 |
| BRAMY2026899 | 0.001688277 | 193505 | 1. |
| BRAMY2026902 | 0.001688277 | 87919 | 1 |
| BRAMY2026904 | 0.001688277 | 73642 | 1 |
| BRAMY2026976 | 0.001688277 | 150599 | 1 |
| BRAMY2027073 | 0.001688277 | 229549. | 1 |
| BRAMY2027114 | 0.009748711 | 145186 | 3 |
| BRAMY2027140 | 0.00649736 | 171273 | 3 |
| BRAMY2027242 | 0.001688277 | 16413 | 1 |
| BRAMY2027396 | 0.001688277 | 131400 | 1 |
| BRAMY2027451 | 0.001688277 | 45694 | 1 |
| BRAMY2027461 | 0.001688277 | 28464 | 1 |
| BRAMY2027467 | 0.001688277 | 133720 | 1 |
| BRAMY2027587 | 0.012548236 | 118907 | 8 |
| BRAMY2027714 | 0.001688277 | 278718 | 1 |
| BRAMY2027717 | 0.04069848 | 105997 | 23 |
| BRAMY2027739 | 0.12450995 | 188594 | 7 |
| BRAMY2027752 | 0.001688277 | 164376 | 1 |
| BRAMY2027782 | 0.001688277 | 39225 | 1 |
| BRAMY2028072 | 0.001688277 | 274286 | 1 |
| BRAMY2028183 | 0.008919087 | 228892 | 3 |
| BRAMY2028188 | 0.001688277 | 175384 | 1 |
| BRAMY2028269 | 0.010265519 | 204213 | 4 |
| BRAMY2028282 | 0.006865285 | 48781 | 3 |
| BRAMY2028472 | 0.001688277 | 172264 | 1 |
| BRAMY2028491 | 0.004670236 | 206029 | 2 |
| BRAMY2028516 | 0.042482865 | 144522 | 5 |
| BRAMY2028565 | 0.001688277 | 273002 | 1 |
| BRAMY2028593 | 0.001688277 | 141401 | 1 |
| BRAMY2028682 | 0.007890596 | 167525 | 2 |
| BRAMY2028740 | 0.179971651 | 87730 | 48 |
| BRAMY2028783 | 0.001688277 | 255679 | 1 |
| BRAMY2028796 | 0.112720242 | 130292 | 13 |
| BRAMY2028856 | 0.001688277 | 203646 | 1 |
| BRAMY2028914 | 0.009458653 | 119461 | 3 |
| BRAMY2029204 | 0.001688277 | 225248 | 1 |
| BRAMY2029602 | 0.001688277 | 200985 | 1 |
| BRAMY2029988 | 0.01422502 | 117928 | 8 |
| BRAMY2030098 | 0.003972799 | 126180 | 3 |
| BRAMY2030109 | 0.001688277 | 143999 | 1 |
| BRAMY2030206 | 0.001688277 | 169340 | 1 |
| BRAMY2030477 | 0.001688277 | 281314 | 1 |
| BRAMY2030545 | 0.007493455 | 107263 | 2 |
| BRAMY2030607 | 0.001688277 | 155855 | 1 |
| BRAMY2030702 | 0.001688277 | 171192 | 1 |
| BRAMY2030703 | 0.001688277 | 171190 | 1 |
| BRAMY2030768 | 0.001688277 | 223983 | 1 |
| BRAMY2030799 | 0.001688277 | 171201 | 1 |
| BRAMY2030859 | 0.001688277 | 221030 | 1 |
| BRAMY2030915 | 0.001688277 | 226837 | 1 |
| BRAMY2031317 | 0.010681699 | 109314 | 7 |
| BRAMY2031377 | 0.001688277 | 163712 | 1 |
| BRAMY2031442 | 0.001688277 | 256075 | 1 |
| BRAMY2031516 | 0.022684724 | 104298 | 9 |
| BRAMY2032014 | 0.001688277 | 213503 | 1 |
| BRAMY2032087 | 0.138356563 | 5993 | 40 |
| BRAMY2032231 | 0.001688277 | 260203 | 1 |
| BRAMY2032242 | 0.001688277 | 235481 | 1 |
| BRAMY2032290 | 0.001688277 | 263255 | 1 |
| BRAMY2032311 | 0.001688277 | 37344 | 1 |
| BRAMY2032317 | 0.002783555 | 264191 | 2 |
| BRAMY2032344 | 0.001688277 | 264072 | 1 |
| BRAMY2032589 | 0.001688277 | 178171 | 1 |
| BRAMY2032678 | 0.001688277 | 195279 | 1 |
| BRAMY2032696 | 0.007890596 | 49991 | 2 |
| BRAMY2032777 | 0.001688277 | 235150 | 1 |
| BRAMY2032994 | 0.001688277 | 179152 | 1 |
| BRAMY2033003 | 0.001688277 | 184119 | 1 |
| BRAMY2033116 | 0.001688277 | 228101 | 1 |
| BRAMY2033240 | 0.001688277 | 261444 | 1 |
| BRAMY2033267 | 0.001688277 | 262038 | 1 |
| BRAMY2033316 | 0.003085432 | 32684 | 2 |
| BRAMY2033332 | 0.001688277 | 177678 | 1 |
| BRAMY2033506 | 0.001688277 | 230160 | 1 |
| BRAMY2033594 | 0.001688277 | 212078 | 1 |
| BRAMY2033895 | 0.001688277 | 212849 | 1 |
| BRAMY2033914 | 0.003363824 | 145115 | 2 |
| BRAMY2034072 | 0.001688277 | 206061 | 1 |
| BRAMY2034185 | 0.001688277 | 230205 | 1 |
| BRAMY2034187 | 0.01347517 | 202857 | 2 |
| BRAMY2034305 | 0.001688277 | 202786 | 1 |
| BRAMY2034561 | 0.001688277 | 284412 | 1 |
| BRAMY2034920 | 0.001688277 | 171583 | 1 |
| BRAMY2034993 | 0.001688277 | 171654 | 1 |
| BRAMY2035070 | 0.003085432 | 18903 | 2 |
| BRAMY2035449 | 0.001688277 | 206535. | 1 |
| BRAMY2035545 | 0.002877521 | 221477 | 2 |
| BRAMY2035606 | 0.001688277 | 281517 | 1 |
| BRAMY2035718 | 0.001688277 | 282089 | 1 |
| BRAMY2035719 | 0.01103125 | 62268 | 4 |
| BRAMY2035801 | 0.001688277 | 276513 | 1 |
| BRAMY2035869 | 0.002783555 | 1045 | 2 |
| BRAMY2035923 | 0.001688277 | 164296 | 1 |
| BRAMY2036076 | 0.015491023 | 86250 | 7 |
| BRAMY2036079 | 0.001688277 | 257310 | 1 |
| BRAMY2036254 | 0.001688277 | 142325 | 1 |
| BRAMY2036266 | 0.001688277 | 148962 | 1 |
| BRAMY2036387 | 0.012515475 | 139077 | 2 |
| BRAMY2036396 | 0.001688277 | 227369 | 1 |
| BRAMY2036459 | 0.088906521 | 164382 | 11 |
| BRAMY2036567 | 0.022417078 | 84976 | 12 |
| BRAMY2036615 | 0.001688277 | 224550 | 1 |
| BRAMY2036699 | 0.001688277 | 213126 | 1 |
| BRAMY2036913 | 0.001688277 | 168297 | 1 |
| BRAMY2036918 | 0.001688277 | 271232 | 1 |
| BRAMY2036968 | 0.001688277 | 279454 | 1 |
| BRAMY2037147 | 0.004670236 | 166258 | 2 |
| BRAMY2037328 | 0.001688277 | 188389 | 1 |
| BRAMY2037609 | 0.001688277 | 158193 | 1 |
| BRAMY2037629 | 0.001688277 | 168259 | 1 |
| BRAMY2037800 | 0.001688277 | 265412 | 1 |
| BRAMY2037823 | 0.005648346 | 84476 | 4 |
| BRAMY2037971 | 0.001688277 | 266427 | 1 |
| BRAMY2038100 | 0.001688277 | 259330 | 1 |
| BRAMY2038128 | 0.001688277 | 255693 | 1 |
| BRAMY2038317 | 0.001688277 | 220493 | 1 |
| BRAMY2038475 | 0.001688277 | 62040 | 1 |
| BRAMY2038484 | 0.003376553 | 220482 | 2 |
| BRAMY2038516 | 0.013220528 | 42714 | 4 |
| BRAMY2038832 | 0.013746414 | 28808 | 3 |
| BRAMY2038846 | 0.003565118 | 248903 | 2 |
| BRAMY2038887 | 0.001688277 | 90556 | 1 |
| BRAMY2038904 | 0.002783555 | 182255 | 2 |
| BRAMY2039287 | 0.001688277 | 210992 | 1 |
| BRAMY2039300 | 0.001688277 | 192922 | 1 |
| BRAMY2039314 | 0.001688277 | 184107 | 1 |
| BRAMY2039341 | 0.001688277 | 182222 | 1 |
| BRAMY2039630 | 0.019306977 | 126235 | 4 |
| BRAMY2039695 | 0.001688277 | 192889 | 1 |
| BRAMY2039728 | 0.004586981 | 222030 | 3 |
| BRAMY2039872 | 0.008451611 | 29553 | 4 |
| BRAMY2040051 | 0.001688277 | 277330 | 1 |
| BRAMY2040095 | 0.490173259 | 23879 | 83 |
| BRAMY2040159 | 0.001688277 | 56644 | 1 |
| BRAMY2040214 | 0.011552293 | 91006 | 7 |
| BRAMY2040478 | 0.001688277 | 278794 | 1 |
| BRAMY2040592 | 0.048118185 | 125029 | 11 |
| BRAMY2040915 | 0.001688277 | 284131 | 1 |
| BRAMY2041261 | 0.001688277 | 277177 | 1 |
| BRAMY2041347 | 0.001688277 | 210516 | 1 |
| BRAMY2041378 | 0.001688277 | 258600 | 1 |
| BRAMY2041384 | 0.001688277 | 27916 | 1 |
| BRAMY2041434 | 0.001688277 | 215608 | 1 |
| BRAMY2041507 | 0.001688277 | 200277 | 1 |
| BRAMY2041542 | 0.001688277 | 282740 | 1 |
| BRAMY2041738 | 0.001688277 | 283696 | 1 |
| BRAMY2042122 | 0.001688277 | 172570 | 1 |
| BRAMY2042131 | 0.001688277 | 146223 | 1 |
| BRAMY2042178 | 0.001688277 | 271806 | 1 |
| BRAMY2042195 | 0.005532361 | 195676 | 2 |
| BRAMY2042244 | 0.001688277 | 230001 | 1 |
| BRAMY2042549 | 0.001688277 | 211224 | 1 |
| BRAMY2042596 | 0.001688277 | 279119 | 1 |
| BRAMY2042612 | 0.001688277 | 280056 | 1 |
| BRAMY2042641 | 0.001688277 | 219803 | 1 |
| BRAMY2042711 | 0.001688277 | 199781 | 1 |
| BRAMY2042760 | 0.001688277 | 241025 | 1 |
| BRAMY2042804 | 0.002877521 | 144442 | 2 |
| BRAMY2042899 | 0.001688277 | 211083 | 1 |
| BRAMY2042918 | 0.001688277 | 178328 | 1 |
| BRAMY2043069 | 0.007890596 | 158421 | 2 |
| BRAMY2043103 | 0.001688277 | 115759 | 1 |
| BRAMY2043314 | 0.001688277 | 264884 | 1 |
| BRAMY2043345 | 0.001688277 | 163385 | 1 |
| BRAMY2044078 | 0.038215023 | 161719 | 16 |
| BRAMY2044246 | 0.001688277 | 263050 | 1 |
| BRAMY2044317 | 0.001688277 | 261445 | 1 |
| BRAMY2044349 | 0.001688277 | 259554 | 1 |
| BRAMY2044686 | 0.001688277 | 276005 | 1 |
| BRAMY2044838 | 0.005404505 | 252610 | 3 |
| BRAMY2045036 | 0.001688277 | 281392 | 1 |
| BRAMY2045299 | 0.001688277 | 148569 | 1 |
| BRAMY2045471 | 0.003376553 | 218376 | 2 |
| BRAMY2045530 | 0.017760844 | 5408 | 4 |
| BRAMY2046109 | 0.001688277 | 98014 | 1 |
| BRAMY2046478 | 0.001688277 | 276394 | 1 |
| BRAMY2046489 | 0.001688277 | 215808 | 1 |
| BRAMY2046537 | 0.001688277 | 240114 | 1 |
| BRAMY2046667 | 0.001688277 | 220865 | 1 |
| BRAMY2046742 | 0.001688277 | 195740 | 1 |
| BRAMY2046871 | 0.0050521 | 213129 | 3 |
| BRAMY2046984 | 0.001688277 | 238975 | 1 |
| BRAMY2046989 | 0.001688277 | 117879 | 1 |
| BRAMY2047169 | 0.001688277 | 251188 | 1 |
| BRAMY2047280 | 0.001688277 | 179986 | 1 |
| BRAMY2047420 | 0.165408115 | 117426 | 10 |
| BRAMY2047676 | 0.001688277 | 170182 | 1 |
| BRAMY2047692 | 0.001688277 | 150911 | 1 |
| BRAMY2047746 | 0.001688277 | 160869 | 1 |
| BRAMY2047751 | 0.001688277 | 240602 | 1 |
| BRAMY2047765 | 0.001688277 | 204252 | 1 |
| BRAMY2047884 | 0.001688277 | 145301 | 1 |
| BRAMY2047948 | 0.001688277 | 260960 | 1 |
| BRAMY3000206 | 0.001688277 | 191074 | 1 |
| BRAMY3000210 | 0.001688277 | 243176 | 1 |
| BRAMY3000213 | 0.001688277 | 208662 | 1 |
| BRAMY3000224 | 0.005865212 | 214564 | 2 |
| BRAMY3000254 | 0.001688277 | 274173 | 1 |
| BRAMY3000307 | 0.001688277 | 14805 | 1 |
| BRAMY3000692 | 0.001688277 | 238277 | 1 |
| BRAMY3001356 | 0.001688277 | 240026 | 1 |
| BRAMY3001401 | 0.001688277 | 282047 | 1 |
| BRAMY3001409 | 0.001688277 | 122846 | 1 |
| BRAMY3001794 | 0.001688277 | 278756 | 1 |
| BRAMY3002120 | 0.003085432 | 274920 | 2 |
| BRAMY3002312 | 0.001688277 | 212407 | 1 |
| BRAMY3002329 | 0.024733489 | 88376 | 15 |
| BRAMY3002458 | 0.013496638 | 50360 | 8 |
| BRAMY3002508 | 0.001688277 | 187663 | 1 |
| BRAMY3002620 | 0.002877521 | 263532 | 2 |
| BRAMY3002803 | 0.011613011 | 6275 | 6 |
| BRAMY3002805 | 0.001688277 | 187027 | 1 |
| BRAMY3002886 | 0.001688277 | 283709 | 1 |
| BRAMY3002991 | 0.042733411 | 156256 | 6 |
| BRAMY3003026 | 0.003769789 | 171950 | 2 |
| BRAMY3003109 | 0.001688277 | 235868 | 1 |
| BRAMY3003205 | 0.006964265 | 22122 | 5 |
| BRAMY3003310 | 0.003085432 | 29786 | 2 |
| BRAMY3003566 | 0.001688277 | 1038 | 1 |
| BRAMY3003723 | 0.001688277 | 273171 | 1 |
| BRAMY3003935 | 0.01069797 | 109078 | 7 |
| BRAMY3004126 | 0.003363824 | 269938 | 2 |
| BRAMY3004224 | 0.005073284 | 90383 | 3 |
| BRAMY3004364 | 0.024665517 | 167502 | 7 |
| BRAMY3004377 | 0.202027897 | 108797 | 44 |
| BRAMY3004672 | 0.001688277 | 198821 | 1 |
| BRAMY3004800 | 0.022011049 | 46315 | 13 |
| BRAMY3004900 | 0.001688277 | 195490 | 1 |
| BRAMY3004919 | 0.001688277 | 110243 | 1 |
| BRAMY3005091 | 0.008730003 | 70985 | 4 |
| BRAMY3005184 | 0.00747765 | 83059 | 4 |
| BRAMY3005656 | 0.001688277 | 260511 | 1 |
| BRAMY3005912 | 0.007823825 | 177921 | 5 |
| BRAMY3005932 | 0.002877521 | 82603 | 2 |
| BRAMY3006032 | 0.001688277 | 186399 | 1 |
| BRAMY3006297 | 0.001688277 | 253799 | 1 |
| BRAMY3006761 | 0.015262219 | 60379 | 9 |
| BRAMY3007078 | 0.003397737 | 211502 | 2 |
| BRAMY3007206 | 0.001688277 | 283003 | 1 |
| BRAMY3007311 | 0.021327119 | 144415 | 9 |
| BRAMY3007350 | 0.001688277 | 27494 | 1 |
| BRAMY3007449 | 0.001688277 | 266907 | 1 |
| BRAMY3007471 | 0.001688277 | 261690 | 1 |
| BRAMY3007609 | 0.001688277 | 256744 | 1 |
| BRAMY3007723 | 0.001688277 | 257850 | 1 |
| BRAMY3007968 | 0.001688277 | 138565 | 1 |
| BRAMY3008044 | 0.093406814 | 60946 | 38 |
| BRAMY3008088 | 0.001688277 | 277370 | 1 |
| BRAMY3008096 | 0.002877521 | 128660 | 2 |
| BRAMY3008335 | 0.009432945 | 77458 | 5 |
| BRAMY3008436 | 0.119621588 | 6885 | 51 |
| BRAMY3008466 | 0.001688277 | 249700 | 1 |
| BRAMY3008505 | 0.001688277 | 259721 | 1 |
| BRAMY3008650 | 0.001688277 | 255619 | 1 |
| BRAMY3008937 | 0.001688277 | 265634 | 1 |
| BRAMY3009158 | 0.001688277 | 265210 | 1 |
| BRAMY3009207 | 0.001688277 | 260172 | 1 |
| BRAMY3009491 | 0.005369955 | 117745 | 4 |
| BRAMY3009556 | 0.003546391 | 251503 | 2 |
| BRAMY3009782 | 0.003546391 | 185685 | 2 |
| BRAMY3009811 | 0.005107197 | 87425 | 3 |
| BRAMY3009904 | 0.001688277 | 259738 | 1 |
| BRAMY3010008 | 0.287891807 | 117489 | 14 |
| BRAMY3010264 | 0.003363824 | 234713 | 2 |
| BRAMY3010321 | 0.001688277 | 232211 | 1 |
| BRAMY3010411 | 0.001688277 | 211338 | 1 |
| BRAMY3010492 | 0.02909303 | 367 | 10 |
| BRAMY3010603 | 0.001688277 | 220417 | 1 |
| BRAMY3010654 | 0.003546391 | 87638 | 2 |
| BRAMY4000089 | 0.032925039 | 111406 | 17 |
| BRAMY4000095 | 0.011468135 | 70851 | 3 |
| BRAMY4000229 | 0.004735635 | 261921 | 3 |
| BRAMY4000277 | 0.001688277 | 260995 | 1 |
| BRAMY4000915 | 0.003397737 | 122639 | 2 |
| BRAMY4000962 | 0.001688277 | 283461 | 1 |
| BRAMY4001173 | 0.001688277 | 210285 | 1 |
| BRAMY4001200 | 0.001688277 | 201028 | 1 |
| BRAMY4001234 | 0.003085432 | 177802 | 2 |
| BRAMY4001449 | 0.001688277 | 221870 | 1 |
| BRAMY4001652 | 0.001688277 | 210095 | 1 |
| BRAMY4001863 | 0.011185159 | 109043 | 5 |
| BRAMY4001913 | 0.001688277 | 125997 | 1 |
| BRAMY4002575 | 0.001688277 | 238638 | 1 |
| BRAMY4002628 | 0.023589801 | 141596 | 11 |
| BRASW1000053 . | 0.632911392 | 218746 | 1 |
| BRASW1000125 | 0.638952958 | 187197 | 2 |
| BRAWH1000001 | 0.050352377 | 61329 | 21 |
| BRAWH1000002 | 0.001675547 | 66770 | 1 |
| BRAWH1000007 | 0.018646974 | 6612 | 5 |
| BRAWH1000037 | 0.091889673 | 54702 | 24 |
| BRAWH1000040 | 0.24351653 | 11924 | 49 |
| BRAWH1000045 | 0.167388547 | 79272 | 64 |
| BRAWH1000083 | 0.036981447 | 9715 | 10 |
| BRAWH1000093 | 0.36328603 | 48128 | 52 |
| BRAWH1000094 | 0.039797585 | 39402 | 14 |
| BRAWH1000116 | 0.001675547 | 43181 | 1 |
| BRAWH1000127 | 0.052416584 | 37367 | 19 |
| BRAWH1000130 | 0.001675547 | 67297 | 1 |
| BRAWH1000157 | 0.001675547 | 64749 | 1 |
| BRAWH1000162 | 0.091810869 | 30660 | 32 |
| BRAWH1000164 | 0.001675547 | 75232 | 1 |
| BRAWH1000167 | 0.092619948 | 51003 | 33 |
| BRAWH1000168 | 0.001675547 | 128265 | 1 |
| BRAWH1000174 | 0.058484446 | 75043 | 25 |
| BRAWH1000180 | 0.001675547 | 40597 | 1 |
| BRAWH1000369 | 0.001675547 | 165088 | 1 |
| BRAWH2000034 | 0.001675547 | 273204 | 1 |
| BRAWH2000048 | 0.001675547 | 259623 | 1 |
| BRAWH2000071 | 0.001675547 | 69516 | 1 |
| BRAWH2000082 | 0.436810495 | 55088 | 158 |
| BRAWH2000093 | 0.001675547 | 228500 | 1 |
| BRAWH2000109 | 0.001675547 | 136811 | 1 |
| BRAWH2000131 | 0.007729915 | 206593 | 2 |
| BRAWH2000142 | 0.010996329 | 117660 | 4 |
| BRAWH2000169 | 0.015895751 | 114160 | 3 |
| BRAWH2000177 | 0.020956106 | 23588 | 7 |
| BRAWH2000218 | 0.003533661 | 45764 | 2 |
| BRAWH2000232 | 0.001675547 | 122780 | 1 |
| BRAWH2000248 | 0.001675547 | 169569 | 1 |
| BRAWH2000256 | 0.001675547 | 245732 | 1 |
| BRAWH2000274 | 0.08487909 | 126191 | 44 |
| BRAWH2000323 | 0.001675547 | 106322 | 1 |
| BRAWH2000353 | 0.002864792 | 87260 | 2 |
| BRAWH2000370 | 0.001675547 | 195714 | 1 |
| BRAWH2000409 | 0.001675547 | 221617 | 1 |
| BRAWH2000417 | 0.271182045 | 85514 | 92 |
| BRAWH2000443 | 0.029573251 | 105194 | 11 |
| BRAWH2000460 | 0.274541089 | 10111 | 76 |
| BRAWH2000471 | 0.001675547 | 267212 | 1 |
| BRAWH2000476 | 0.006952582 | 93782 | 4 |
| BRAWH2000482 | 0.110393093 | 13399 | 26 |
| BRAWH2000488 | 0.016619307 | 133749 | 6 |
| BRAWH2000494 | 0.018430826 | 140072 | 6 |
| BRAWH2000500 | 0.002864792 | 192393 | 2 |
| BRAWH2000503 | 0.008076427 | 131331 | 4 |
| BRAWH2000522 | 0.09244356 | 39525 | 18 |
| BRAWH2000554 | 0.001675547 | 265277 | 1 |
| BRAWH2000588 | 0.133555816 | 79239 | 29 |
| BRAWH2000595 | 0.013436102 | 119870 | 2 |
| BRAWH2000633 | 0.126392578 | 31367 | 18 |
| BRAWH2000636 | 0.317081402 | 80819 | 122 |
| BRAWH2000643 | 0.001675547 | 231904 | 1 |
| BRAWH2000651 | 0.001675547 | 144148 | 1 |
| BRAWH2000682 | 0.001675547 | 95684 | 1 |
| BRAWH2000686 | 0.137074917 | 38929 | 51 |
| BRAWH2000697 | 0.165643883 | 125094 | 44 |
| BRAWH2000752 | 0.001675547 | 130443 | 1 |
| BRAWH2000839 | 0.407966855 | 38528 | 119 |
| BRAWH2000859 | 0.003552389 | 214221 | 2 |
| BRAWH2000866 | 0.001675547 | 255369 | 1 |
| BRAWH2000892 | 0.001675547 | 219414 | 1 |
| BRAWH2000901 | 0.001675547 | 280013 | 1 |
| BRAWH2000924 | 0.583840525 | 60800 | 159 |
| BRAWH2000926 | 0.039727297 | 109375 | 2 |
| BRAWH2000944 | 0.032118304 | 103162 | 11 |
| BRAWH2000984 | 0.001675547 | 122613 | 1 |
| BRAWH2001019 | 0.073467058 | 4310 | 21 |
| BRAWH2001037 | 0.041779669 | 170115 | 15 |
| BRAWH2001052 | 0.001675547 | 270537 | 1 |
| BRAWH2001092 | 0.953353157 | 65081 | 148 |
| BRAWH2001103 | 0.025497494 | 217133 | 5 |
| BRAWH2001129 | 0.003552389 | 143882 | 2 |
| BRAWH2001137 | 0.001675547 | 178414 | 1 |
| BRAWH2001141 | 0.032970596 | 75233 | 13 |
| BRAWH2001159 | 0.001675547 | 80868 | 1 |
| BRAWH2001166 | 0.05308009 | 96586 | 15 |
| BRAWH2001171 | 0.271297828 | 45061 | 101 |
| BRAWH2001186 | 0.058806701 | 67320 | 6 |
| BRAWH2001203 | 0.001675547 | 44253 | 1 |
| BRAWH2001238 | 0.013858127 | 121814 | 7 |
| BRAWH2001239 | 0.022965193 | 151246 | 5 |
| BRAWH2001255 | 0.001675547 | 239935 | 1 |
| BRAWH2001321 | 0.195185305 | 40551 | 78 |
| BRAWH2001355 | 0.001675547 | 104096 | 1 |
| BRAWH2001378 | 0.001675547 | 128073 | 1 |
| BRAWH2001395 | 0.398667125 | 6827 | 161 |
| BRAWH2001406 | 0.049495121 | 106201 | 7 |
| BRAWH2001412 | 0.011143487 | 197652 | 4 |
| BRAWH2001418 | 0.064965414 | 99926 | 17 |
| BRAWH2001424 | 0.0329093 | 72850 | 7 |
| BRAWH2001436 | 0.001675547 | 260418 | 1 |
| BRAWH2001438 | 0.001675547 | 260234 | 1 |
| BRAWH2001439 | 0.162847305 | 86450 | 59 |
| BRAWH2001459 | 0.068509233 | 76563 | 29 |
| BRAWH2001461 | 0.047136806 | 76709 | 6 |
| BRAWH2001484 | 0.015770289 | 125985 | 5 |
| BRAWH2001492 | 0.001675547 | 139006 | 1 |
| BRAWH2001502 | 1.267697555 | 27207 | 213 |
| BRAWH2001503 | 0.001675547 | 285113 | 1 |
| BRAWH2001519 | 0.001675547 | 275735 | 1 |
| BRAWH2001530 | 0.021950701 | 131534 | 6 |
| BRAWH2001535 | 0.339353143 | 83177 | 102 |
| BRAWH2001589 | 0.003351094 | 222414 | 2 |
| BRAWH2001662 | 0.03644306 | 104160 | 11 |
| BRAWH2001666 | 0.003351094 | 145143 | 2 |
| BRAWH2001671 | 0.044064981 | 135772 | 15 |
| BRAWH2001679 | 0.001675547 | 43070 | 1 |
| BRAWH2001686 | 0.001675547 | 208551 | 1 |
| BRAWH2001701 | 0.003351094 | 185782 | 2 |
| BRAWH2001842 | 0.001675547 | 177855 | 1 |
| BRAWH2001862 | 0.049891555 | 50559 | 2 |
| BRAWH2001873 | 0.001675547 | 199204 | 1 |
| BRAWH2001940 | 0.004480285 | 104286 | 3 |
| BRAWH2001973 | 0.004480285 | 84575 | 3 |
| BRAWH2002047 | 0.002864792 | 163894 | 2 |
| BRAWH2002060 | 0.002864792 | 110855 | 2 |
| BRAWH2002062 | 0.003072702 | 168588 | 2 |
| BRAWH2002125 | 0.001675547 | 33389 | 1 |
| BRAWH2002191 | 0.001675547 | 155312 | 1 |
| BRAWH2002318 | 0.006278681 | 6005 | 3 |
| BRAWH2002333 | 0.001675547 | 90149 | 1 |
| BRAWH2002352 | 0.001675547 | 182411 | 1 |
| BRAWH2002383 | 0.001675547 | 203207 | 1 |
| BRAWH2002549 | 0.009299599 | 168333 | 3 |
| BRAWH2002560 | 0.051925901 | 24106 | 18 |
| BRAWH2002601 | 0.033688345 | 132020 | 13 |
| BRAWH2002623 | 0.001675547 | 195435 | 1 |
| BRAWH2002630 | 0.001675547 | 205437 | 1 |
| BRAWH2002635 | 0.001675547 | 167440 | 1 |
| BRAWH2002662 | 0.001675547 | 198736 | 1 |
| BRAWH2002679 | 0.017698113 | 38968 | 4 |
| BRAWH2002725 | 0.001675547 | 262605 | 1 |
| BRAWH2002761 | 0.001675547 | 276467 | 1 |
| BRAWH2002811 | 0.019428385 | 140433 | 8 |
| BRAWH2002861 | 0.001675547 | 144857 | 1 |
| BRAWH2002911 | 0.007041727 | 109926 | 3 |
| BRAWH2002963 | 0.014042972 | 145002 | 3 |
| BRAWH2002967 | 0.003757059 | 143402 | 2 |
| BRAWH2002976 | 0.001675547 | 34620 | 1 |
| BRAWH2003000 | 0.002864792 | 121508 | 2 |
| BRAWH2003025 | 0.055260238 | 40082 | 12 |
| BRAWH2003135 | 0.004796353 | 75904 | 2 |
| BRAWH2003240 | 0.021350649 | 80779 | 4 |
| BRAWH2003307 | 0.001675547 | 227712 | 1 |
| BRAWH2003355 | 0.007729915 | 232894 | 2 |
| BRAWH2003366 | 0.001675547 | 173285 | 1 |
| BRAWH2003662 | 0.027035576 | 142685 | 13 |
| BRAWH2003689 | 0.003552389 | 129639 | 2 |
| BRAWH2003693 | 0.048975235 | 55559 | 16 |
| BRAWH2003818 | 0.05536172 | 39487 | 11 |
| BRAWH2003832 | 0.001675547 | 168336 | 1 |
| BRAWH2003948 | 0.001675547 | 46769 | 1 |
| BRAWH2003964 | 0.003385007 | 48760 | 2 |
| BRAWH2004044 | 0.001675547 | 161936 | 1 |
| BRAWH2004068 | 0.001675547 | 60350 | 1 |
| BRAWH2004078 | 0.031797678 | 215344 | 7 |
| BRAWH2004095 | 0.141739371 | 91975 | 53 |
| BRAWH2004136 | 0.010530678 | 204838 | 4 |
| BRAWH2004217 | 0.003072702 | 79530 | 2 |
| BRAWH2004580 | 0.009214721 | 149244 | 4 |
| BRAWH2004731 | 0.001675547 | 172111 | 1 |
| BRAWH2004779 | 0.003363824 | 172110 | 2 |
| BRAWH2004842 | 0.042036641 | 125533 | 22 |
| BRAWH2004884 | 0.001675547 | 148802 | 1 |
| BRAWH2004923 | 0.001675547 | 251058 | 1 |
| BRAWH2005008 | 0.010148342 | 92454 | 5 |
| BRAWH2005068 | 0.416566647 | 145643 | 23 |
| BRAWH2005074 | 0.004771043 | 99951 | 2 |
| BRAWH2005225 | 0.028905305 | 140646 | 10 |
| BRAWH2005315 | 0.001675547 | 117854 | 1 |
| BRAWH2005517 | 0.003757059 | 47957 | 2 |
| BRAWH2005524 | 0.001675547 | 150027 | 1 |
| BRAWH2005533 | 0.001675547 | 275384 | 1 |
| BRAWH2005578 | 0.012503372 | 158656 | 7 |
| BRAWH2005661 | 0.104812185 | 103899 | 29 |
| BRAWH2005812 | 0.001675547 | 92953 | 1 |
| BRAWH2005974 | 0.001675547 | 154422 | 1 |
| BRAWH2005998 | 0.005519631 | 165543 | 2 |
| BRAWH2006044 | 0.009523565 | 68176 | 4 |
| BRAWH2006083 | 0.001675547 | 144284 | 1 |
| BRAWH2006207 | 0.012945233 | 150670 | 4 |
| BRAWH2006301 | 0.002864792 | 102326 | 2 |
| BRAWH2006395 | 0.01346244 | 245071 | 2 |
| BRAWH2006405 | 0.012911511 | 98799 | 4 |
| BRAWH2006450 | 0.033369032 | 128986 | 13 |
| BRAWH2006493 | 0.032942226 | 142646 | 13 |
| BRAWH2006526 | 0.003072702 | 192799 | 2 |
| BRAWH2006543 | 0.038198657 | 124033 | 19 |
| BRAWH2006622 | 0.010126419 | 112961 | 4 |
| BRAWH2006760 | 0.001675547 | 70390 | 1 |
| BRAWH2006960 | 0.001675547 | 42476 | 1 |
| BRAWH2006989 | 0.006708875 | 161487 | 3 |
| BRAWH2006996 | 0.049260868 | 113942 | 11 |
| BRAWH2007069 | 0.001675547 | 173997 | 1 |
| BRAWH2007308 | 0.003351094 | 108276 | 2 |
| BRAWH2007406 | 0.001675547 | 196415 | 1 |
| BRAWH2007550 | 0.001675547 | 161434 | 1 |
| BRAWH2007570 | 0.001675547 | 78177 | 1 |
| BRAWH2007591 | 0.0533923 | 82609 | 32 |
| BRAWH2007605 | 0.081686424 | 109624 | 37 |
| BRAWH2007658 | 0.014419301 | 158117 | 6 |
| BRAWH2007664 | 0.016375786 | 30706 | 5 |
| BRAWH2007800 | 0.071331673 | 110609 | 8 |
| BRAWH2007808 | 0.004469858 | 158139 | 3 |
| BRAWH2007825 | 0.002864792 | 90359 | 2 |
| BRAWH2007862 | 0.001675547 | 156457 | 1 |
| BRAWH2007890 | 0.001675547 | 33980 | 1 |
| BRAWH2008058 | 0.007152616 | 157433 | 4 |
| BRAWH2008128 | 0.007588159 | 141840 | 2 |
| BRAWH2008219 | 0.011627806 | 2127 | 4 |
| BRAWH2008255 | 0.039660178 | 118528 | 7 |
| BRAWH2008292 | 0.004590404 | 23747 | 2 |
| BRAWH2008297 | 0.001675547 | 116427 | 1 |
| BRAWH2008366 | 0.013139493 | 32578 | 2 |
| BRAWH2008380 | 0.001675547 | 221636 | 1 |
| BRAWH2008540 | 0.003072702 | 168580 | 2 |
| BRAWH2008577 | 0.047128784 | 119324 | 3 |
| BRAWH2008706 | 0.058270128 | 83391 | 25 |
| BRAWH2008790 | 0.001675547 | 169218 | 1 |
| BRAWH2008903 | 0.039628131 | 86047 | 18 |
| BRAWH2008956 | 0.010761144 | 116317 | 6 |
| BRAWH2008993 | 0.003363824 | 110730 | 2 |
| BRAWH2009112 | 0.001675547 | 195764 | 1 |
| BRAWH2009227 | 0.010601532 | 157980 | 3 |
| BRAWH2009238 | 0.03214183 | 66051 | 20 |
| BRAWH2009261 | 0.001675547 | 134582 | 1 |
| BRAWH2009263 | 0.001675547 | 190993 | 1 |
| BRAWH2009298 | 0.001675547 | 180316 | 1 |
| BRAWH2009304 | 0.042719991 | 71017 | 11 |
| BRAWH2009307 | 0.084153786 | 134573 | 13 |
| BRAWH2009360 | 0.019362756 | 149798 | 4 |
| BRAWH2009393 | 0.001675547 | 126256 | 1 |
| BRAWH2009485 | 0.001675547 | 72162 | 1 |
| BRAWH2009490 | 0.049523133 | 160072 | 3 |
| BRAWH2009558 | 0.001675547 | 152234 | 1 |
| BRAWH2009590 | 0.001675547 | 103984 | 1 |
| BRAWH2009626 | 0.003757059 | 157183 | 2 |
| BRAWH2009678 | 0.007877867 | 206807 | 2 |
| BRAWH2009695 | 0.006262528 | 94573 | 4 |
| BRAWH2009907 | 0.001675547 | 143767 | 1 |
| BRAWH2010000 | 0.008938551 | 110619 | 4 |
| BRAWH2010069 | 0.001675547 | 133838 | 1 |
| BRAWH2010084 | 0.001675547 | 169125 | 1 |
| BRAWH2010136 | 0.006345783 | 148720 | 3 |
| BRAWH2010318 | 0.001675547 | 161941 | 1 |
| BRAWH2010329 | 0.066964303 | 84969 | 40 |
| BRAWH2010354 | 0.012911195 | 134398 | 4 |
| BRAWH2010364 | 0.009723566 | 75150 | 7 |
| BRAWH2010536 | 0.011384285 | 150103 | 2 |
| BRAWH2010552 | 0.002864792 | 161059 | 2 |
| BRAWH2010584 | 0.005519631 | 174573 | 2 |
| BRAWH2010618 | 0.001675547 | 162035 | 1 |
| BRAWH2010619 | 0.001675547 | 160987 | 1 |
| BRAWH2010754 | 0.01497874 | 146640 | 4 |
| BRAWH2010980 | 0.023711758 | 8032 | 4 |
| BRAWH2011066 | 0.007911905 | 97161 | 2 |
| BRAWH2011079 | 0.001675547 | 34587 | 1 |
| BRAWH2011096 | 0.003072702 | 41070 | 2 |
| BRAWH2011155 | 0.001675547 | 53735 | 1 |
| BRAWH2011166 | 0.001675547 | 169015 | 1 |
| BRAWH2011204 | 0.003363824 | 133840 | 2 |
| BRAWH2011286 | 0.018775481 | 169769 | 3 |
| BRAWH2011294 | 0.001675547 | 84560 | 1 |
| BRAWH2011343 | 0.023885229 | 3696 | 12 |
| BRAWH2011400 | 0.001675547 | 76793 | 1 |
| BRAWH2011762 | 0.001675547 | 160760 | 1 |
| BRAWH2011795 | 0.001675547 | 159903 | 1 |
| BRAWH2011796 | 0.001675547 | 125184 | 1 |
| BRAWH2011812 | 0.012371582 | 138479 | 4 |
| BRAWH2011823 | 0.00774793 | 81130 | 2 |
| BRAWH2011860 | 0.001675547 | 15493 | 1 |
| BRAWH2011920 | 0.008843072 | 102818 | 5 |
| BRAWH2011958 | 0.001675547 | 145201 | 1 |
| BRAWH2012054 | 0.001675547 | 274598 | 1 |
| BRAWH2012077 | 0.013373031 | 108446 | 3 |
| BRAWH2012161 | 0.043517163 | 109813 | 4 |
| BRAWH2012162 | 0.009293218 | 134272 | 3 |
| BRAWH2012164 | 0.0080418 | 127327 | 4 |
| BRAWH2012258 | 0.001675547 | 149606 | 1 |
| BRAWH2012277 | 0.005852482 | 162426 | 2 |
| BRAWH2012326 | 0.001675547 | 132676 | 1 |
| BRAWH2012538 | 0.033030933 | 156117 | 6 |
| BRAWH2012540 | 0.001675547 | 190796 | 1 |
| BRAWH2012619 | 0.001675547 | 206260 | 1 |
| BRAWH2012698 | 0.008738778 | 81784 | 5 |
| BRAWH2012749 | 0.001675547 | 150056 | 1 |
| BRAWH2012827 | 0.018631453 | 156309 | 5 |
| BRAWH2012866 | 0.078824191 | 92308 | 33 |
| BRAWH2012963 | 0.001675547 | 156447 | 1 |
| BRAWH2013047 | 0.00891916 | 168647 | 3 |
| BRAWH2013219 | 0.017296059 | 150185 | 5 |
| BRAWH2013294 | 0.016992457 | 82020 | 8 |
| BRAWH2013748 | 0.062780603 | 140432 | 10 |
| BRAWH2013866 | 0.001675547 | 184419 | 1 |
| BRAWH2013871 | 0.004469858 | 121957 | 3 |
| BRAWH2013914 | 0.003533661 | 169064 | 2 |
| BRAWH2013941 | 0.001675547 | 117299 | 1 |
| BRAWH2013955 | 0.001675547 | 200899 | 1 |
| BRAWH2014053 | 0.00721534 | 49117 | 5 |
| BRAWH2014188 | 0.234037348 | 89493 | 75 |
| BRAWH2014234 | 0.001675547 | 169657 | 1 |
| BRAWH2014379 | 0.008071523 | 156404 | 4 |
| BRAWH2014414 | 0.018757788 | 144394 | 11 |
| BRAWH2014473 | 0.003673948 | 107630 | 2 |
| BRAWH2014511 | 0.038995427 | 74990 | 11 |
| BRAWH2014645 | 0.158504425 | 5893 | 47 |
| BRAWH2014662 | 0.010795289 | 130749 | 4 |
| BRAWH2014717 | 0.001675547 | 130227 | 1 |
| BRAWH2014729 | 0.001675547 | 135718 | 1 |
| BRAWH2014876 | 0.015999447 | 79596 | 8 |
| BRAWH2014934 | 0.004657506 | 196562 | 2 |
| BRAWH2014954 | 0.008594375 | 64398 | 6 |
| BRAWH2015247 | 0.016049487 | 51796 | 6 |
| BRAWH2015349 | 0.015234123 | 133657 | 4 |
| BRAWH2015375 | 0.001675547 | 73565 | 1 |
| BRAWH2015385 | 0.002864792 | 145887 | 2 |
| BRAWH2015595 | 0.001675547 | 168501 | 1 |
| BRAWH2015728 | 0.003363824 | 232459 | 2 |
| BRAWH2015853 | 0.001675547 | 191340 | 1 |
| BRAWH2015866 | 0.001675547 | 281697 | 1 |
| BRAWH2016028 | 0.003363824 | 162496 | 2 |
| BRAWH2016106 | 0.004590404 | 55835 | 2 |
| BRAWH2016160 | 0.001675547 | 80028 | 1 |
| BRAWH2016166 | 0.022155807 | 94358 | 12 |
| BRAWH2016209 | 0.020172424 | 184569 | 6 |
| BRAWH2016221 | 0.003533661 | 213257 | 2 |
| BRAWH2016223 | 0.001675547 | 201440 | 1 |
| BRAWH2016269 | 0.127095772 | 109168 | 15 |
| BRAWH2016305 | 0.038891773 | 244464 | 2 |
| BRAWH2016439 | 0.001675547 | 88084 | 1 |
| BRAWH2016446 | 0.019259288 | 204380 | 4 |
| BRAWH2016514 | 0.004782163 | 234421 | 3 |
| BRAWH2016562 | 0.003385007 | 132251 | 2 |
| BRAWH2016655 | 0.001675547 | 22329 | 1 |
| BRAWH2016679 | 0.01738912 | 187261 | 3 |
| BRAWH2016702 | 0.004540339 | 145426 | 3 |
| BRAWH2016724 | 0.004771043 | 155420 | 2 |
| BRAWH2016785 | 0.003351094 | 81447 | 2 |
| BRAWH2016792 | 0.003351094 | 86017 | 2 |
| BRAWH2016800 | 0.001675547 | 274840 | 1 |
| BRAWH2017002 | 0.0673329 | 57826 | 23 |
| BRAWH2017103 | 0.001675547 | 224531 | 1 |
| BRAWH2017250 | 0.001675547 | 127741 | 1 |
| BRAWH2017294 | 0.003533661 | 106107 | 2 |
| BRAWH2017304 | 0.01009072 | 137379 | 5 |
| BRAWH2017305 | 0.001675547 | 198548 | 1 |
| BRAWH2017379 | 0.011027116 | 161551 | 4 |
| BRAWH2017407 | 0.001675547 | 10980 | 1 |
| BRAWH2017433 | 0.003351094 | 138889 | 2 |
| BRAWH2017523 | 0.001675547 | 152193 | 1 |
| BRAWH2017534 | 0.001675547 | 185738 | 1 |
| BRAWH2017542 | 0.004771043 | 161709 | 2 |
| BRAWH2017635 | 0.001675547 | 15543 | 1 |
| BRAWH2017685 | 0.330158685 | 120529 | 61 |
| BRAWH2017913 | 0.001675547 | 155897 | 1 |
| BRAWH2018206 | 0.01516167 | 101278 | 6 |
| BRAWH2018267 | 0.008107913 | 143028 | 5 |
| BRAWH2018282 | 0.001675547 | 75552 | 1 |
| BRAWH2018317 | 0.001675547 | 195763 | 1 |
| BRAWH2018506 | 0.001675547 | 231106 | 1 |
| BRAWH2018514 | 0.001675547 | 221793 | 1 |
| BRAWH2018526 | 0.544470089 | 129133 | 44 |
| BRAWH2018527 | 0.019684242 | 185060 | 3 |
| BRAWH2018601 | 0.001675547 | 196414 | 1 |
| BRAWH2018729 | 0.056231978 | 123237 | 5 |
| BRAWH2018745 | 0.003673948 | 48669 | 2 |
| BRAWH2018875 | 0.012648252 | 84089 | 8 |
| BRAWH2019053 | 0.001675547 | 89882 | 1 |
| BRAWH2019055 | 0.001675547 | 98551 | 1 |
| BRAWH2019198 | 0.001675547 | 77683 | 1 |
| BRAWH3000078 | 0.001675547 | 88230 | 1 |
| BRAWH3000088 | 0.144845796 | 124777 | 54 |
| BRAWH3000100 | 0.001675547 | 279359 | 1 |
| BRAWH3000166 | 0.001675547 | 242460 | 1 |
| BRAWH3000314 | 0.007025042 | 166457 | 4 |
| BRAWH3000329 | 0.001675547 | 216881 | 1 |
| BRAWH3000410 | 0.001675547 | 272484 | 1 |
| BRAWH3000446 | 0.001675547 | 205765 | 1 |
| BRAWH3000491 | 0.001675547 | 281787 | 1 |
| BRAWH3000604 | 0.003072702 | 233470 | 2 |
| BRAWH3000884 | 0.001675547 | 269906 | 1 |
| BRAWH3001053 | 0.003351094 | 179568 | 2 |
| BRAWH3001319 | 0.006138789 | 183216 | 4 |
| BRAWH3001326 | 0.003673948 | 232595 | 2 |
| BRAWH3001475 | 0.001675547 | 257569 | 1 |
| BRAWH3001636 | 0.001675547 | 262464 | 1 |
| BRAWH3001638 | 0.001675547 | 282938 | 1 |
| BRAWH3001712 | 0.003385007 | 270855 | 2 |
| BRAWH3001760 | 0.001675547 | 99303 | 1 |
| BRAWH3001783 | 0.001675547 | 143864 | 1 |
| BRAWH3001833 | 0.001675547 | 261258 | 1 |
| BRAWH3001879 | 0.001675547 | 255948 | 1 |
| BRAWH3001891 | 0.009702382 | 179526 | 7 |
| BRAWH3001918 | 0.012066426 | 89585 | 8 |
| BRAWH3002433 | 0.003533661 | 234665 | 2 |
| BRAWH3002467 | 0.009180808 | 191510 | 4 |
| BRAWH3002513 | 0.001675547 | 517 | 1 |
| BRAWH3002574 | 0.025343997 | 69483 | 8 |
| BRAWH3002600 | 0.004553068 | 135831 | 3 |
| BRAWH3002819 | 0.001675547 | 259427 | 1 |
| BRAWH3002821 | 0.007632302 | 166780 | 4 |
| BRAWH3002853 | 0.226526584 | 32608 | 60 |
| BRAWH3003019 | 0.001675547 | 238681 | 1 |
| BRAWH3003136 | 0.001675547 | 228170 | 1 |
| BRAWH3003244 | 0.001675547 | 238643 | 1 |
| BRAWH3003343 | 0.001675547 | 247864 | 1 |
| BRAWH3003411 | 0.007240683 | 190039 | 5 |
| BRAWH3003522 | 0.001675547 | 248808 | 1 |
| BRAWH3003555 | 0.022004007 | 42729 | 7 |
| BRAWH3003573 | 0.008310618 | 65784 | 6 |
| BRAWH3003598 | 0.001675547 | 242182 | 1 |
| BRAWH3003727 | 0.016663179 | 116935 | 10 |
| BRAWH3003801 | 0.003351094 | 131635 | 2 |
| BRAWH3003975 | 0.003351094 | 199152 | 2 |
| BRAWH3003992 | 0.011552401 | 131464 | 5 |
| BRAWH3004222 | 0.003757059 | 194775 | 2 |
| BRAWH3004335 | 0.001675547 | 263186 | 1 |
| BRAWH3004350 | 0.010106008 | 114768 | 6 |
| BRAWH3004453 | 0.001675547 | 261011 | 1 |
| BRAWH3004530 | 0.009498452 | 54899 | 6 |
| BRAWH3004666 | 0.003385007 | 244416 | 2 |
| BRAWH3005037 | 0.001675547 | 248294 | 1 |
| BRAWH3005047 | 0.001675547 | 258477 | 1 |
| BRAWH3005132 | 0.003363824 | 188326 | 2 |
| BRAWH3005146 | 0.033092275 | 69861 | 13 |
| BRAWH3005292 | 0.001675547 | 282073 | 1 |
| BRAWH3005300 | 0.003552389 | 82196 | 2 |
| BRAWH3005422 | 0.001675547 | 279719 | 1 |
| BRAWH3005534 | 0.001675547 | 280748 | 1 |
| BRAWH3005886 | 0.001675547 | 261047 | 1 |
| BRAWH3005892 | 0.001675547 | 280937 | 1 |
| BRAWH3005896 | 0.001675547 | 256349 | 1 |
| BRAWH3005912 | 0.001675547 | 271411 | 1 |
| BRAWH3005981 | 0.00571383 | 277327 | 2 |
| BRAWH3006547 | 0.001675547 | 276488 | 1 |
| BRAWH3006548 | 0.011796656 | 148518 | 7 |
| BRAWH3006761 | 0.007878022 | 166828 | 5 |
| BRAWH3006792 | 0.003385007 | 246720 | 2 |
| BRAWH3007129 | 0.017027073 | 160039 | 8 |
| BRAWH3007221 | 0.003351094 | 235246 | 2 |
| BRAWH3007441 | 0.001675547 | 264171 | 1 |
| BRAWH3007455 | 0.001675547 | 251894 | 1 |
| BRAWH3007506 | 0.001675547 | 233101 | 1 |
| BRAWH3007592 | 0.093440803 | 64972 | 30 |
| BRAWH3007726 | 0.006145405 | 217388 | 4 |
| BRAWH3007783 | 0.001675547 | 279927 | 1 |
| BRAWH3008167 | 0.001675547 | 138566 | 1 |
| BRAWH3008341 | 0.001675547 | 200684 | 1 |
| BRAWH3008559 | 0.001675547 | 283607 | 1 |
| BRAWH3008697 | 0.003351094 | 86255 | 2 |
| BRAWH3008867 | 0.001675547 | 254090 | 1 |
| BRAWH3008931 | 0.048377823 | 106919 | 17 |
| BRAWH3009013 | 0.02559117 | 50260 | 11 |
| BRAWH3009017 | 0.001675547 | 223932 | 1 |
| BRAWH3009285 | 0.004540339 | 119388 | 3 |
| BRAWH3009297 | 0.002864792 | 227623 | 2 |
| BRAWH3009546 | 0.007108153 | 133866 | 4 |
| BRAWH3009961 | 0.003533661 | 81921 | 2 |
| BRAWH3010461 | 0.081558458 | 71514 | 41 |
| BRAWH3010560 | 0.001675547 | 164700 | 1 |
| BRAWH3010602 | 0.001675547 | 158258 | 1 |
| BRAWH3010657 | 0.001675547 | 114193 | 1 |
| BRAWH3010726 | 0.001675547 | 112837 | 1 |
| BRAWH3010833 | 0.001675547 | 237791 | 1 |
| BRAWH3011101 | 0.001675547 | 184506 | 1 |
| BRAWH3011331 | 0.001675547 | 207193 | 1 |
| BRAWH3011402 | 0.001675547 | 145263 | 1 |
| BRAWH3011577 | 0.001675547 | 248955 | 1 |
| BRAWH3011623 | 0.001675547 | 251784 | 1 |
| BRAWH3011637 | 0.00542923 | 122745 | 3 |
| BRAWH3011685 | 0.001675547 | 237702 | 1 |
| BRAWH3011907 | 0.003533661 | 207282 | 2 |
| BRAWH3011929 | 0.001675547 | 277704 | 1 |
| BRAWH3012005 | 0.001675547 | 231751 | 1 |
| BRAWH3012662 | 0.014796535 | 99528 | 6 |
| BRAWH3012779 | 0.001675547 | 282146 | 1 |
| BRAWH3013009 | 0.001675547 | 238671 | 1 |
| BRAWH3013049 | 0.001675547 | 228700 | 1 |
| BRAWH3013197 | 0.001675547 | 265568 | 1 |
| BRAWH3013264 | 0.003351094 | 150921 | 2 |
| BRAWH3013508 | 0.001675547 | 283882 | 1 |
| BRAWH3014609 | 0.003757059 | 160705 | 2 |
| BRAWH3014639 | 0.001675547 | 274081 | 1 |
| BRAWH3015017 | 0.001675547 | 102597 | 1 |
| BRAWH3015175 | 0.001675547 | 256586 | 1 |
| BRAWH3015260 | 0.001675547 | 177813 | 1 |
| BRAWH3015610 | 0.001675547 | 262371 | 1 |
| BRAWH3015825 | 0.001675547 | 48227 | 1 |
| BRAWH3016123 | 0.001675547 | 267069 | 1 |
| BRAWH3016271 | 0.031064887 | 120891 | 8 |
| BRAWH3016715 | 0.001675547 | 84691 | 1 |
| BRAWH3017180 | 0.005930878 | 82580 | 4 |
| BRAWH3017259 | 0.023943624 | 34784 | 8 |
| BRAWH3017260 | 0.003351094 | 83107 | 2 |
| BRAWH3017477 | 0.001675547 | 268033 | 1 |
| BRAWH3017537 | 0.001675547 | 267063 | 1 |
| BRAWH3017980 | 0.003363824 | 160032 | 2 |
| BRAWH3018063 | 0.06348498 | 144822 | 20 |
| BRAWH3018326 | 0.013764262 | 145370 | 4 |
| BRAWH3018369 | 0.001675547 | 217040 | 1 |
| BRAWH3018548 | 0.001675547 | 164946 | 1 |
| BRAWH3018969 | 0.001675547 | 60794 | 1 |
| BRAWH3019026 | 0.001675547 | 97288 | 1 |
| BRAWH3019529 | 0.001675547 | 257347 | 1 |
| BRAWH3019594 | 0.001675547 | 210334 | 1 |
| BRAWH3019629 | 0.001675547 | 276435 | 1 |
| BRAWH3019820 | 0.001675547 | 268523 | 1 |
| BRAWH3020200 | 0.001675547 | 81634 | 1 |
| BRAWH3020318 | 0.001675547 | 84995 | 1 |
| BRAWH3020486 | 0.001675547 | 213436 | 1 |
| BRAWH3020884 | 0.001675547 | 23422 | 1 |
| BRAWH3020928 | 0.003385007 | 152654 | 2 |
| BRAWH3021012 | 0.001675547 | 207349 | 1 |
| BRAWH3021545 | 0.04592879 | 50396 | 22 |
| BRAWH3021574 | 0.035651665 | 61311 | 13 |
| BRAWH3021580 | 0.003351094 | 74031 | 2 |
| BRAWH3021641 | 0.001675547 | 110504 | 1 |
| BRAWH3021643 | 0.001675547 | 89504 | 1 |
| BRAWH3021724 | 0.003351094 | 81943 | 2 |
| BRAWH3022347 | 0.001675547 | 213075 | 1 |
| BRAWH3022431 | 0.001675547 | 195705 | 1 |
| BRAWH3022459 | 0.001675547 | 218788 | 1 |
| BRAWH3022542 | 0.001675547 | 258254 | 1 |
| BRAWH3022651 | 0.10228708 | 119632 | 35 |
| BRAWH3022719 | 0.001675547 | 106275 | 1 |
| BRAWH3022866 | 0.001675547 | 276578 | 1 |
| BRAWH3022900 | 0.001675547 | 111018 | 1 |
| BRAWH3023156 | 0.001675547 | 266869 | 1 |
| BRAWH3023168 | 0.001675547 | 226668 | 1 |
| BRAWH3023172 | 0.182954113 | 86062 | 10 |
| BRAWH3023274 | 0.001675547 | 109669 | 1 |
| BRAWH3023421 | 0.001675547 | 264367 | 1 |
| BRAWH3024186 | 0.016789762 | 65471 | 8 |
| BRAWH3024231 | 0.001675547 | 267850 | 1 |
| BRAWH3024242 | 0.005261849 | 157813 | 3 |
| BRAWH3024469 | 0.001675547 | 19232 | 1 |
| BRAWH3024506 | 0.001675547 | 156888 | 1 |
| BRAWH3024613 | 0.001675547 | 197765 | 1 |
| BRAWH3024989 | 0.07072506 | 120140 | 29 |
| BRAWH3026349 | 0.001675547 | 251374 | 1 |
| BRAWH3026394 | 0.001675547 | 259578 | 1 |
| BRAWH3026529 | 0.003673948 | 138720 | 2 |
| BRAWH3026938 | 0.072918291 | 109313 | 27 |
| BRAWH3027420 | 0.001675547 | 212385 | 1 |
| BRAWH3027440 | 0.002864792 | 178730 | 2 |
| BRAWH3027533 | 0.015221005 | 130150 | 3 |
| BRAWH3027574 | 0.003533661 | 147319 | 2 |
| BRAWH3027607 | 0.001675547 | 132187 | 1 |
| BRAWH3027616 | 0.001675547 | 184622 | 1 |
| BRAWH3027675 | 0.001675547 | 244944 | 1 |
| BRAWH3027806 | 0.001675547 | 201378 | 1 |
| BRAWH3027880 | 0.001675547 | 183033 | 1 |
| BRAWH3027927 | 0.001675547 | 3799 | 1 |
| BRAWH3028202 | 0.001675547 | 186937 | 1 |
| BRAWH3028223 | 0.001675547 | 234808 | 1 |
| BRAWH3028306 | 0.001675547 | 61779 | 1 |
| BRAWH3028461 | 0.001675547 | 201279 | 1 |
| BRAWH3028645 | 0.001675547 | 213562 | 1 |
| BRAWH3028754 | 0.001675547 | 262333 | 1 |
| BRAWH3028796 | 0.001675547 | 210470 | 1 |
| BRAWH3029254 | 0.001675547 | 204569 | 1 |
| BRAWH3029313 | 0.001675547 | 195640 | 1 |
| BRAWH3029385 | 0.009523796 | 157043 | 6 |
| BRAWH3029538 | 0.003351094 | 83210 | 2 |
| BRAWH3029556 | 0.008606945 | 116664 | 5 |
| BRAWH3029806 | 0.018801856 | 83334 | 11 |
| BRAWH3030613 | 0.001675547 | 156978 | 1 |
| BRAWH3030772 | 0.001675547 | 125907 | 1 |
| BRAWH3030810 | 0.015742571 | 125856 | 12 |
| BRAWH3030892 | 0.001675547 | 147829 | 1 |
| BRAWH3030910 | 0.001675547 | 125981 | 1 |
| BRAWH3030950 | 0.001675547 | 174483 | 1 |
| BRAWH3031054 | 0.001675547 | 146636 | 1 |
| BRAWH3031342 | 0.03086749 | 18696 | 13 |
| BRAWH3031710 | 0.001675547 | 152867 | 1 |
| BRAWH3031902 | 0.001675547 | 132138 | 1 |
| BRAWH3032298 | 0.001675547 | 159392 | 1 |
| BRAWH3032340 | 0.001675547 | 147921 | 1 |
| BRAWH3032571 | 0.001675547 | 159231 | 1 |
| BRAWH3032620 | 0.001675547 | 94379 | 1 |
| BRAWH3032930 | 0.001675547 | 211300 | 1 |
| BRAWH3033117 | 0.001675547 | 169507 | 1 |
| BRAWH3033293 | 0.001675547 | 177964 | 1 |
| BRAWH3033448 | 0.001675547 | 214377 | 1 |
| BRAWH3033513 | 0.001675547 | 216441 | 1 |
| BRAWH3034097 | 0.001675547 | 139494 | 1 |
| BRAWH3034114 | 0.001675547 | 134289 | 1 |
| BRAWH3034134 | 0.001675547 | 120881 | 1 |
| BRAWH3034668 | 0.001675547 | 237898 | 1 |
| BRAWH3034743 | 0.040224064 | 56904 | 21 |
| BRAWH3034775 | 0.001675547 | 73162 | 1 |
| BRAWH3034890 | 0.001675547 | 73172 | 1 |
| BRAWH3035154 | 0.001675547 | 156807 | 1 |
| BRAWH3035403 | 0.004741633 | 124099 | 3 |
| BRAWH3035904 | 0.001675547 | 166356 | 1 |
| BRAWH3035914 | 0.001675547 | 103310 | 1 |
| BRAWH3035936 | 0.00474825 | 134375 | 3 |
| BRAWH3036077 | 0.001675547 | 153135 | 1 |
| BRAWH3036247 | 0.003072702 | 156760 | 2 |
| BRAWH3036270 | 0.001675547 | 174326 | 1 |
| BRAWH3036334 | 0.026846921 | 18694 | 13 |
| BRAWH3036561 | 0.001675547 | 165613 | 1 |
| BRAWH3036738 | 0.001675547 | 202007 | 1 |
| BRAWH3036951 | 0.001675547 | 140590 | 1 |
| BRAWH3037265 | 0.003757059 | 166320 | 2 |
| BRAWH3037394 | 0.001675547 | 128028 | 1 |
| BRAWH3037428 | 0.028512311 | 60834 | 13 |
| BRAWH3037533 | 0.001675547 | 118159 | 1 |
| BRAWH3037979 | 0.001675547 | 116724 | 1 |
| BRAWH3038055 | 0.003351094 | 149091 | 2 |
| BRAWH3038230 | 0.001675547 | 239909 | 1 |
| BRAWH3038252 | 0.001675547 | 113692 | 1 |
| BRAWH3038324 | 0.003351094 | 13645 | 2 |
| BRCAN1000065 | 0.003844084 | 135287 | 1 |
| BRCAN1000076 | 0.056927504 | 71942 | 6 |
| BRCAN1000105 | 0.003844084 | 91601 | 1 |
| BRCAN1000149 | 0.006758941 | 122109 | 2 |
| BRCAN1000168 | 0.118776685 | 82827 | 42 |
| BRCAN2000148 | 0.003844084 | 244740 | 1 |
| BRCAN2000149 | 0.003844084 | 248714 | 1 |
| BRCAN2000209 | 0.010046404 | 163728 | 2 |
| BRCAN2000239 | 0.003844084 | 137382 | 1 |
| BRCAN2000346 | 0.003844084 | 147935 | 1 |
| BRCAN2000364 | 0.003844084 | 54885 | 1 |
| BRCAN2000383 | 0.003844084 | 261105 | 1 |
| BRCAN2000418 | 0.003844084 | 276541 | 1 |
| BRCAN2000523 | 0.003844084 | 220782 | 1 |
| BRCAN2000541 | 0.003844084 | 99442 | 1 |
| BRCAN2000563 | 0.012741448 | 80080 | 4 |
| BRCAN2000577 | 0.003844084 | 187454 | 1 |
| BRCAN2000620 | 0.003844084 | 112571 | 1 |
| BRCAN2000639 | 0.00998961 | 132003 | 2 |
| BRCAN2000763 | 0.003844084 | 189621 | 1 |
| BRCAN2000832 | 0.003844084 | 85128 | 1 |
| BRCAN2000923 | 0.14449106 | 143876 | 2 |
| BRCAN2001223 | 0.007241821 | 50291 | 3 |
| BRCAN2001866 | 0.003844084 | 99717 | 1 |
| BRCAN2002173 | 0.084263573 | 78105 | 12 |
| BRCAN2002416 | 0.003844084 | 98836 | 1 |
| BRCAN2002473 | 0.003844084 | 54001 | 1 |
| BRCAN2002562 | 0.003844084 | 3398 | 1 |
| BRCAN2002662 | 0.003844084 | 161777 | 1 |
| BRCAN2002826 | 0.003844084 | 249654 | 1 |
| BRCAN2002854 | 0.003844084 | 63625 | 1 |
| BRCAN2002856 | 0.019559718 | 8075 | 4 |
| BRCAN2002892 | 0.005702198 | 102492 | 2 |
| BRCAN2002944 | 0.005241239 | 187798 | 2 |
| BRCAN2002948 | 0.003844084 | 139985 | 1 |
| BRCAN2003070 | 0.003844084 | 275253 | 1 |
| BRCAN2003269 | 0.003844084 | 177054 | 1 |
| BRCAN2003389 | 0.055104973 | 42048 | 3 |
| BRCAN2003628 | 0.003844084 | 161812 | 1 |
| BRCAN2003703 | 0.003844084 | 141346 | 1 |
| BRCAN2003746 | 0.003844084 | 190406 | 1 |
| BRCAN2003814 | 0.038735919 | 137987 | 2 |
| BRCAN2003944 | 0.594971919 | 95673 | 58 |
| BRCAN2003987 | 0.003844084 | 151203 | 1 |
| BRCAN2004173 | 0.003844084 | 160496 | 1 |
| BRCAN2004355 | 0.003844084 | 84673 | 1 |
| BRCAN2004371 | 0.003844084 | 152864 | 1 |
| BRCAN2004653 | 0.004939362 | 138924 | 2 |
| BRCAN2004699 | 0.003844084 | 179615 | 1 |
| BRCAN2005436 | 0.003844084 | 190248 | 1 |
| BRCAN2005449 | 0.003844084 | 28712 | 1 |
| BRCAN2006019 | 0.04460076 | 104549 | 10 |
| BRCAN2006051 | 0.003844084 | 163958 | 1 |
| BRCAN2006063 | 0.003844084 | 58312 | 1 |
| BRCAN2006117 | 0.006721605 | 169303 | 3 |
| BRCAN2006174 | 0.003844084 | 160469 | 1 |
| BRCAN2006290 | 0.003844084 | 212382 | 1 |
| BRCAN2006297 | 0.011637973 | 170416 | 3 |
| BRCAN2006323 | 0.003844084 | 42504 | 1 |
| BRCAN2006401 | 0.007390475 | 110847 | 3 |
| BRCAN2006411 | 0.003844084 | 41795 | 1 |
| BRCAN2006450 | 0.003844084 | 263743 | 1 |
| BRCAN2006955 | 0.003844084 | 268784 | 1 |
| BRCAN2007119 | 0.00988565 | 36558 | 2 |
| BRCAN2007144 | 0.003844084 | 36656 | 1 |
| BRCAN2007409 | 0.055846075 | 59873 | 8 |
| BRCAN2007426 | 0.003844084 | 146743 | 1 |
| BRCAN2007525 | 0.003844084 | 195523 | 1 |
| BRCAN2007700 | 0.0137779 | 188624 | 5 |
| BRCAN2008494 | 0.003844084 | 149957 | 1 |
| BRCAN2008528 | 0.003844084 | 144220 | 1 |
| BRCAN2008701 | 0.003844084 | 161671 | 1 |
| BRCAN2008894 | 0.022007189 | 165687 | 4 |
| BRCAN2009156 | 0.003844084 | 39419 | 1 |
| BRCAN2009168 | 0.003844084 | 225031 | 1 |
| BRCAN2009203 | 0.003844084 | 34820 | 1 |
| BRCAN2009432 | 0.127250354 | 23822 | 14 |
| BRCAN2010374 | 0.005532361 | 103405 | 2 |
| BRCAN2010376 | 0.005033328 | 141036 | 2 |
| BRCAN2010547 | 0.003844084 | 230039 | 1 |
| BRCAN2010581 | 0.003844084 | 195474 | 1 |
| BRCAN2010665 | 0.051267018 | 79370 | 21 |
| BRCAN2011254 | 0.003844084 | 174425 | 1 |
| BRCAN2011316 | 0.003844084 | 225201 | 1 |
| BRCAN2011602 | 0.007688168 | 167458 | 2 |
| BRCAN2011946 | 0.026496751 | 28461 | 6 |
| BRCAN2012110 | 0.003844084 | 108708 | 1 |
| BRCAN2012355 | 0.003844084 | 195653 | 1 |
| BRCAN2012402 | 0.003844084 | 275590 | 1 |
| BRCAN2012408 | 0.003844084 | 146031 | 1 |
| BRCAN2012481 | 0.003844084 | 206235 | 1 |
| BRCAN2012613 | 0.003844084 | 198686 | 1 |
| BRCAN2012713 | 0.003844084 | 201852 | 1 |
| BRCAN2012929 | 0.013729734 | 169420 | 3 |
| BRCAN2013655 | 0.003844084 | 209395 | 1 |
| BRCAN2013660 | 0.003844084 | 271438 | 1 |
| BRCAN2014143 | 0.003844084 | 160591 | 1 |
| BRCAN2014229 | 0.003844084 | 160592 | 1 |
| BRCAN2014370 | 0.003844084 | 202940 | 1 |
| BRCAN2014602 | 0.003844084 | 82595 | 1 |
| BRCAN2014652 | 0.003844084 | 107243 | 1 |
| BRCAN2014788 | 0.003844084 | 220642 | 1 |
| BRCAN2014881 | 0.005532361 | 8661 | 2 |
| BRCAN2014952 | 0.003844084 | 174930 | 1 |
| BRCAN2015371 | 0.015285503 | 152047 | 5 |
| BRCAN2015402 | 0.003844084 | 267367 | 1 |
| BRCAN2015464 | 0.003844084 | 241390 | 1 |
| BRCAN2015719 | 0.055495027 | 109738 | 12 |
| BRCAN2015757 | 0.003844084 | 232947 | 1 |
| BRCAN2015886 | 0.003844084 | 232952 | 1 |
| BRCAN2016025 | 0.003844084 | 209149 | 1 |
| BRCAN2016046 | 0.005532361 | 154590 | 2 |
| BRCAN2016174 | 0.015410936 | 168562 | 3 |
| BRCAN2016433 | 0.003844084 | 54131 | 1 |
| BRCAN2016619 | 0.01271868 | 109646 | 4 |
| BRCAN2016762 | 0.003844084 | 138231 | 1 |
| BRCAN2016814 | 0.005702198 | 149212 | 2 |
| BRCAN2017442 | 0.003844084 | 195360 | 1 |
| BRCAN2017717 | 0.015750862 | 131671 | 4 |
| BRCAN2017905 | 0.003844084 | 213650 | 1 |
| BRCAN2018240 | 0.003844084 | 114116 | 1 |
| BRCAN2018269 | 0.003844084 | 147416 | 1 |
| BRCAN2018490 | 0.003844084 | 218477 | 1 |
| BRCAN2018566 | 0.006758941 | 221911 | 2 |
| BRCAN2018667 | 0.003844084 | 272519 | 1 |
| BRCAN2018748 | 0.003844084 | 21173 | 1 |
| BRCAN2018935 | 0.018368412 | 275378 | 2 |
| BRCAN2019002 | 0.003844084 | 231098 | 1 |
| BRCAN2019387 | 0.003844084 | 158055 | 1 |
| BRCAN2019653 | 0.026866142 | 137400 | 7 |
| BRCAN2019773 | 0.003844084 | 261413 | 1 |
| BRCAN2019907 | 0.003844084 | 61114 | 1 |
| BRCAN2019953 | 0.017616261 | 126124 | 7 |
| BRCAN2020234 | 0.006797477 | 151391 | 3 |
| BRCAN2020331 | 0.003844084 | 74998 | 1 |
| BRCAN2020412 | 0.015363494 | 19743 | 2 |
| BRCAN2020467 | 0.003844084 | 7574 | 1 |
| BRCAN2020710 | 0.015417139 | 126295 | 6 |
| BRCAN2020880 | 0.003844084 | 209759 | 1 |
| BRCAN2021024 | 0.016339278 | 57384 | 8 |
| BRCAN2021028 | 0.015925385 | 219211 | 3 |
| BRCAN2021325 | 0.003844084 | 18495 | 1 |
| BRCAN2021452 | 0.003844084 | 262828 | 1 |
| BRCAN2021669 | 0.003844084 | 252453 | 1 |
| BRCAN2021718 | 0.003844084 | 209683 | 1 |
| BRCAN2022126 | 0.034508627 | 126374 | 12 |
| BRCAN2022191 | 0.003844084 | 252624 | 1 |
| BRCAN2022472 | 0.003844084 | 121747 | 1 |
| BRCAN2022474 | 0.003844084 | 172625 | 1 |
| BRCAN2023347 | 0.003844084 | 216974 | 1 |
| BRCAN2023724 | 0.003844084 | 283958 | 1 |
| BRCAN2024259 | 0.095037005 | 31692 | 41 |
| BRCAN2024451 | 0.003844084 | 179715 | 1 |
| BRCAN2024563 | 0.003844084 | 276224 | 1 |
| BRCAN2024572 | 0.061349936 | 69558 | 17 |
| BRCAN2025009 | 0.003844084 | 276509 | 1 |
| BRCAN2025093 | 0.007710188 | 139096 | 4 |
| BRCAN2025349 | 0.003844084 | 242581 | 1 |
| BRCAN2025368 | 0.019796904 | 136756 | 4 |
| BRCAN2025712 | 0.003844084 | 172914 | 1 |
| BRCAN2025982 | 0.005553544 | 67879 | 2 |
| BRCAN2026117 | 0.003844084 | 280910 | 1 |
| BRCAN2026197 | 0.107321308 | 43537 | 34 |
| BRCAN2026340 | 0.011087697 | 198787 | 3 |
| BRCAN2026711 | 0.003844084 | 86354 | 1 |
| BRCAN2027150 | 0.016438542 | 32203 | 2 |
| BRCAN2027310 | 0.003844084 | 172371 | 1 |
| BRCAN2027334 | 0.005532361 | 131917 | 2 |
| BRCAN2027355 | 0.003844084 | 74301 | 1 |
| BRCAN2027364 | 0.056419347 | 28163 | 18 |
| BRCAN2027513 | 0.003844084 | 150618 | 1 |
| BRCAN2027593 | 0.003844084 | 281663 | 1 |
| BRCAN2027970 | 0.003844084 | 197634 | 1 |
| BRCAN2028021 | 0.003844084 | 142751 | 1 |
| BRCAN2028036 | 0.069399133 | 37105 | 14 |
| BRCAN2028040 | 0.006336518 | 179367 | 3 |
| BRCAN2028319 | 0.012874753 | 118570 | 4 |
| BRCAN2028338 | 0.003844084 | 202521 | 1 |
| BRCAN2028355 | 0.113416185 | 34589 | 7 |
| BRCAN2028378 | 0.003844084 | 190610 | 1 |
| BRCAN2028460 | 0.003844084 | 221046 | 1 |
| BRCAN2028545 | 0.00988565 | 249974 | 2 |
| BRCAN2028634 | 0.005702198 | 230114 | 2 |
| BRCAN2028702 | 0.003844084 | 222760 | 1 |
| BRCOC1000040 | 0.056815748 | 91819 | 23 |
| BRCOC1000087 | 0.005935775 | 98530 | 1 |
| BRCOC1000094 | 0.175974203 | 90967 | 41 |
| BRCOC2000004 | 0.093083084 | 117659 | 26 |
| BRCOC2000047 | 0.011990143 | 186127 | 2 |
| BRCOC2000152 | 0.008017287 | 126617 | 2 |
| BRCOC2000184 | 0.019993963 | 153536 | 3 |
| BRCOC2000186 | 0.056330024 | 61789 | 5 |
| BRCOC2000333 | 0.013988544 | 57918 | 3 |
| BRCOC2000360 | 0.009320782 | 13758 | 3 |
| BRCOC2000404 | 0.005935775 | 71087 | 1 |
| BRCOC2000487 | 0.578350283 | 183430 | 8 |
| BRCOC2000637 | 0.005935775 | 33321 | 1 |
| BRCOC2000670 | 0.007624052 | 157803 | 2 |
| BRCOC2000816 | 0.007611322 | 47698 | 2 |
| BRCOC2000850 | 0.005935775 | 251661 | 1 |
| BRCOC2001355 | 0.005935775 | 168470 | 1 |
| BRCOC2002085 | 0.005935775 | 227864 | 1 |
| BRCOC2002155 | 0.007812617 | 85141 | 2 |
| BRCOC2002323 | 0.005935775 | 208816 | 1 |
| BRCOC2002659 | 0.005935775 | 60687 | 1 |
| BRCOC2002664 | 0.005935775 | 223483 | 1 |
| BRCOC2002686 | 0.005935775 | 227218 | 1 |
| BRCOC2002751 | 0.005935775 | 152273 | 1 |
| BRCOC2002756 | 0.005935775 | 236262 | 1 |
| BRCOC2002777 | 0.005935775 | 236888 | 1 |
| BRCOC2002835 | 0.005935775 | 271611 | 1 |
| BRCOC2003100 | 0.007125019 | 189182 | 2 |
| BRCOC2003125 | 0.02948599 | 170652 | 3 |
| BRCOC2003187 | 0.213061185 | 136280 | 21 |
| BRCOC2003213 | 0.016139846 | 176666 | 5 |
| BRCOC2003513 | 0.005935775 | 222059 | 1 |
| BRCOC2003732 | 0.007812617 | 72397 | 2 |
| BRCOC2003740 | 0.005935775 | 118759 | 1 |
| BRCOC2004036 . | 0.011990143 | 84666 | 2 |
| BRCOC2004175 | 0.017222457 | 14318 | 2 |
| BRCOC2004354 | 0.005935775 | 71847 | 1 |
| BRCOC2004455 | 0.005935775 | 187920 | 1 |
| BRCOC2004950 | 0.005935775 | 222129 | 1 |
| BRCOC2005542 | 0.009482166 | 137824 | 3 |
| BRCOC2005903 | 0.019087997 | 182454 | 3 |
| BRCOC2005951 | 0.005935775 | 113101 | 1 |
| BRCOC2006164 | 0.011528845 | 67154 | 4 |
| BRCOC2006189 | 0.05047861 | 87356 | 17 |
| BRCOC2006243 | 0.005935775 | 245025 | 1 |
| BRCOC2006270 | 0.011990143 | 5012 | 2 |
| BRCOC2006639 | 0.005935775 | 83473 | 1 |
| BRCOC2006942 | 0.005935775 | 161969 | 1 |
| BRCOC2007034 | 0.007125019 | 197343 | 2 |
| BRCOC2007476 | 0.020811331 | 129777 | 7 |
| BRCOC2007593 | 0.005935775 | 200862 | 1 |
| BRCOC2007733 | 0.005935775 | 143874 | 1 |
| BRCOC2007769 | 0.005935775 | 46881 | 1 |
| BRCOC2007864 | 0.165643883 | 125094 | 44 |
| BRCOC2008064 | 0.005935775 | 272829 | 1 |
| BRCOC2008225 | 0.005935775 | 160323 | 1 |
| BRCOC2008642 | 0.005935775 | 174077 | 1 |
| BRCOC2008993 | 0.005935775 | 128520 | 1 |
| BRCOC2009052 | 0.005935775 | 156579 | 1 |
| BRCOC2009196 | 0.005935775 | 130823 | 1 |
| BRCOC2009380 | 0.005935775 | 208446 | 1 |
| BRCOC2009638 | 0.005935775 | 160277 | 1 |
| BRCOC2009937 | 0.01042879 | 49779 | 4 |
| BRCOC2010115 | 0.005935775 | 274106 | 1 |
| BRCOC2010123 | 0.152952001 | 135859 | 14 |
| BRCOC2010510 | 0.013953062 | 173796 | 3 |
| BRCOC2010730 | 0.015501458 | 52063 | 6 |
| BRCOC2010980 | 0.005935775 | 115520 | 1 |
| BRCOC2010994 | 0.005935775 | 239211 | 1 |
| BRCOC2011010 | 0.005935775 | 149962 | 1 |
| BRCOC2011398 | 0.005935775 | 193068 | 1 |
| BRCOC2011418 | 0.005935775 | 167478 | 1 |
| BRCOC2011506 | 0.176080525 | 152205 | 14 |
| BRCOC2011544 | 0.117885646 | 168112 | 17 |
| BRCOC2011769 | 0.005935775 | 215804 | 1 |
| BRCOC2011996 | 0.005935775 | 208943 | 1 |
| BRCOC2012172 | 0.059567813 | 141939 | 29 |
| BRCOC2012229 | 0.005935775 | 86581 | 1 |
| BRCOC2012386 | 0.014694486 | 136577 | 4 |
| BRCOC2012551 | 0.023231153 | 123831 | 11 |
| BRCOC2012813 | 0.009779859 | 42927 | 2 |
| BRCOC2013448 | 0.005935775 | 17689 | 1 |
| BRCOC2013573 | 0.005935775 | 258775 | 1 |
| BRCOC2013675 | 0.005935775 | 125334 | 1 |
| BRCOC2013780 | 0.011990143 | 168066 | 2 |
| BRCOC2014013 | 0.030896123 | 156531 | 5 |
| BRCOC2014033 | 0.010717938 | 192261 | 4 |
| BRCOC2014178 | 0.083115518 | 88587 | 28 |
| BRCOC2014400 | 0.009974058 | 37726 | 2 |
| BRCOC2014748 | 0.005935775 | 153159 | 1 |
| BRCOC2014833 | 0.005935775 | 159685 | 1 |
| BRCOC2015597 | 0.007934176 | 248379 | 2 |
| BRCOC2015824 | 0.005935775 | 133223 | 1 |
| BRCOC2015908 | 0.008850632 | 128500 | 2 |
| BRCOC2015944 | 0.005935775 | 271718 | 1 |
| BRCOC2016525 | 0.005935775 | 199258 | 1 |
| BRCOC2016661 | 0.005935775 | 273678 | 1 |
| BRCOC2017061 | 0.005935775 | 78265 | 1 |
| BRCOC2017652 | 0.012138095 | 18378 | 2 |
| BRCOC2017856 | 0.005935775 | 96918 | 1 |
| BRCOC2018775 | 0.01653798 | 142231 | 2 |
| BRCOC2018877 | 0.008850632 | 49368 | 2 |
| BRCOC2019255 | 0.019587861 | 128892 | 5 |
| BRCOC2019549 | 0.005935775 | 175751 | 1 |
| BRCOC2019841 | 0.007125019 | 171607 | 2 |
| BRCOC2019896 | 0.007624052 | 66373 | 2 |
| BRCOC2019903 | 0.010453737 | 104768 | 3 |
| BRCOC2019910 | 0.268110198 | 82318 | 140 |
| BRCOC2019934 | 0.005935775 | 197632 | 1 |
| BRCOC2020122 | 0.005935775 | 137297 | 1 |
| BRCOC2020142 | 0.007611322 | 152952 | 2 |
| BRHIP1000058 | 0.00170946 | 230471 | 1 |
| BRHIP1000072 | 0.00170946 | 146589 | 1 |
| BRHIP1000104 | 0.25633749 | 99527 | 75 |
| BRHIP1000108 | 0.038879618 | 85252 | 15 |
| BRHIP1000129 | 0.003397737 | 93924 | 2 |
| BRHIP1000174 | 0.033048593 | 122269 | 11 |
| BRHIP1000258 | 0.006480503 | 87401 | 3 |
| BRHIP2000021 | 0.014771111 | 83122 | 2 |
| BRHIP2000079 | 0.00170946 | 60877 | 1 |
| BRHIP2000087 | 0.025458357 | 141764 | 7 |
| BRHIP2000163 | 0.004480285 | 107084 | 3 |
| BRHIP2000210 | 0.002898705 | 180589 | 2 |
| BRHIP2000239 | 0.003586302 | 219053 | 2 |
| BRHIP2000245 | 0.003106616 | 36877 | 2 |
| BRHIP2000312 | 0.025838097 | 13600 | 14 |
| BRHIP2000359 | 0.00170946 | 110633 | 1 |
| BRHIP2000445 | 0.00170946 | 74655 | 1 |
| BRHIP2000506 | 0.00170946 | 265273 | 1 |
| BRHIP2000532 | 0.00170946 | 242287 | 1 |
| BRHIP2000534 | 0.489558345 | 54252 | 52 |
| BRHIP2000553 | 0.065204083 | 112505 | 24 |
| BRHIP2000621 | 0.00170946 | 135060 | 1 |
| BRHIP2000691 | 0.00170946 | 91747 | 1 |
| BRHIP2000819 | 0.077565447 | 63996 | 15 |
| BRHIP2000826 | 0.00170946 | 195964 | 1 |
| BRHIP2000920 | 0.004574252 | 1721 | 3 |
| BRHIP2001074 | 0.008552481 | 141443 | 4 |
| BRHIP2001099 | 0.024171057 | 18466 | 9 |
| BRHIP2001686 | 0.00170946 | 168052 | 1 |
| BRHIP2001782 | 0.00170946 | 147120 | 1 |
| BRHIP2001802 | 0.00170946 | 13741 | 1 |
| BRHIP2001805 | 0.011584053 | 130721 | 2 |
| BRHIP2001886 | 0.003397737 | 156599 | 2 |
| BRHIP2001927 | 0.00170946 | 103096 | 1 |
| BRHIP2002122 | 0.00170946 | 202996 | 1 |
| BRHIP2002143 | 0.002898705 | 152747 | 2 |
| BRHIP2002172 | 0.00170946 | 156585 | 1 |
| BRHIP2002339 | 0.021350394 | 130120 | 10 |
| BRHIP2002346 | 0.00170946 | 158808 | 1 |
| BRHIP2002510 | 0.00170946 | 283979 | 1 |
| BRHIP2002664 | 0.00170946 | 161947 | 1 |
| BRHIP2002722 | 0.00170946 | 154342 | 1 |
| BRHIP2002929 | 0.00170946 | 96456 | 1 |
| BRHIP2003048 | 0.00170946 | 147259 | 1 |
| BRHIP2003062 | 0.017638961 | 67501 | 10 |
| BRHIP2003242 | 0.00170946 | 160902 | 1 |
| BRHIP2003272 | 0.00170946 | 216050 | 1 |
| BRHIP2003748 | 0.00170946 | 207793 | 1 |
| BRHIP2003786 | 0.00170946 | 113029 | 1 |
| BRHIP2003828 | 0.00170946. | 219454 | 1 |
| BRHIP2003917 | 0.014268684 | 147330 | 3 |
| BRHIP2004312 | 0.00170946 | 40614 | 1 |
| BRHIP2004355 | 0.00170946 | 33474 | 1 |
| BRHIP2004359 | 0.00688226 | 14940 | 5 |
| BRHIP2004814 | 0.004830267 | 206824 | 2 |
| BRHIP2004883 | 0.00170946 | 203046 | 1 |
| BRHIP2004902 | 0.00170946 | 196516 | 1 |
| BRHIP2005010 | 0.01062862 | 144510 | 4 |
| BRHIP2005236 | 0.00170946 | 234942 | 1 |
| BRHIP2005271 | 0.023655592 | 131024 | 13 |
| BRHIP2005354. | 0.00170946 | 183924 | 1 |
| BRHIP2005583 | 0.048140878 | 101969 | 13 |
| BRHIP2005600 | 0.00170946 | 168223 | 1 |
| BRHIP2005719 | 0.003385007 | 191287 | 2 |
| BRHIP2005724 | 0.00170946 | 30696 | 1 |
| BRHIP2005752 | 0.002898705 | 108690 | 2 |
| BRHIP2005932 | 0.00170946 | 50190 | 1 |
| BRHIP2006617 | 0.00170946 | 161005 | 1 |
| BRHIP2006782 | 0.00170946 | 163891 | 1 |
| BRHIP2006800 | 0.00170946 | 163869 | 1 |
| BRHIP2006819 | 0.00170946 | 242608 | 1 |
| BRHIP2006921 | 0.00170946 | 260663 | 1 |
| BRHIP2007305 | 0.148012957 | 130789 | 29 |
| BRHIP2007307 | 0.005886395 | 143844 | 2 |
| BRHIP2007616 | 0.00170946 | 163973 | 1 |
| BRHIP2007690 | 0.00170946 | 213796 | 1 |
| BRHIP2007741 | 0.096527316 | 158769 | 27 |
| BRHIP2007928 | 0.00170946 | 154976 | 1 |
| BRHIP2007969 | 0.00170946 | 179793 | 1 |
| BRHIP2008269 | 0.006021473 | 40089 | 3 |
| BRHIP2008389 | 0.00170946 | 189559 | 1 |
| BRHIP2008607 | 0.009331071 | 109657 | 6 |
| BRHIP2008756 | 0.00170946 | 21599 | 1 |
| BRHIP2008909 | 0.00170946 | 211728 | 1 |
| BRHIP2009019 | 0.002898705 | 232461 | 2 |
| BRHIP2009177 | 0.00170946 | 228601 | 1 |
| BRHIP2009340 | 0.00170946 | 217726 | 1 |
| BRHIP2009351 | 0.00170946 | 239323 | 1 |
| BRHIP2009414 | 0.00170946 | 30608 | 1 |
| BRHIP2009474 | 0.003567575 | 170450 | 2 |
| BRHIP2009617 | 0.00170946 | 149177 | 1 |
| BRHIP2009685 | 0.047037149 | 151845 | 8 |
| BRHIP2010217 | 0.030001638 | 126118 | 9 |
| BRHIP2010309 | 0.00170946 | 222788 | 1 |
| BRHIP2010444 | 0.003567575 | 102257 | 2 |
| BRHIP2010487 | 0.040739036 | 105303 | 3 |
| BRHIP2010571 | 0.010881243 | 132250 | 7 |
| BRHIP2010593 | 0.047847882 | 118687 | 3 |
| BRHIP2010610 | 0.00170946 | 111747 | 1 |
| BRHIP2010723 | 0.003397737 | 140170 | 2 |
| BRHIP2010744 | 0.00170946 | 143992 | 1 |
| BRHIP2011080 | 0.004804956 | 108959 | 2 |
| BRHIP2011120 | 0.021830116 | 129498 | 12 |
| BRHIP2011199 | 0.003106616 | 165737 | 2 |
| BRHIP2011491 | 0.202710934 | 146691 | 17 |
| BRHIP2011508 | 0.007759041 | 88948 | 5 |
| BRHIP2011576 | 0.008740167 | 182175 | 3 |
| BRHIP2011616 | 0.003707861 | 155037 | 2 |
| BRHIP2011783 | 0.00170946 | 236020 | 1 |
| BRHIP2011838 | 0.00170946 | 175556 | 1 |
| BRHIP2011891 | 0.002804738 | 148563 | 2 |
| BRHIP2011933 | 0.00170946 | 27376 | 1 |
| BRHIP2012054 | 0.005886395 | 72314 | 2 |
| BRHIP2012059 | 0.00170946 | 156034 | 1 |
| BRHIP2012141 | 0.003567575 | 159032 | 2 |
| BRHIP2012314 | 0.00170946 | 179267 | 1 |
| BRHIP2012360 | 0.00341892 | 162676 | 2 |
| BRHIP2012545 | 0.00170946 | 183243 | 1 |
| BRHIP2012580 | 0.00170946 | 122018 | 1 |
| BRHIP2012627 | 0.010666604 | 175420 | 3 |
| BRHIP2012642 | 0.00170946 | 189468 | 1 |
| BRHIP2012750 | 0.00170946 | 133293 | 1 |
| BRHIP2012922 | 0.00341892 | 173974 | 2 |
| BRHIP2012923 | 0.005553544 | 70357 | 2 |
| BRHIP2012972 | 0.003567575 | 143538 | 2 |
| BRHIP2013286 | 0.009786026 | 132197 | 3 |
| BRHIP2013447 | 0.00170946 | 153478 | 1 |
| BRHIP2013510 | 0.00170946 | 89582 | 1 |
| BRHIP2013699 | 0.007852315 | 137064 | 4 |
| BRHIP2013723 | 0.00170946 | 38026 | 1 |
| BRHIP2013958 | 0.004662853 | 122647 | 3 |
| BRHIP2013972 | 0.00170946 | 34651 | 1 |
| BRHIP2014063 | 0.004624317 | 29547 | 2 |
| BRHIP2014228 | 0.01138336 | 151704 | 6 |
| BRHIP2014285 | 0.00170946 | 153370 | 1 |
| BRHIP2014291 | 0.00170946 | 54956 | 1 |
| BRHIP2014373 | 0.00496473 | 112679 | 3 |
| BRHIP2014386 | 0.003106616 | 102740 | 2 |
| BRHIP2014387 | 0.00170946 | 84258 | 1 |
| BRHIP2014747 | 0.146698192 | 36550 | 40 |
| BRHIP2014954 | 0.00170946 | 157461 | 1 |
| BRHIP2015153 | 0.016723757 | 125355 | 10 |
| BRHIP2015224 | 0.03683202 | 86319 | 18 |
| BRHIP2015245 | 0.070578263 | 8049 | 39 |
| BRHIP2015356 | 0.00170946 | 165697 | 1 |
| BRHIP2015360 | 0.00170946 | 157497 | 1 |
| BRHIP2015679 | 0.066794406 | 86476 | 14 |
| BRHIP2016005 | 0.00170946 | 189760 | 1 |
| BRHIP2016125 | 0.00170946 | 180928 | 1 |
| BRHIP2016788 | 0.00170946 | 150426 | 1 |
| BRHIP2016968 | 0.00170946 | 180531 | 1 |
| BRHIP2016990 | 0.00170946 | 165673 | 1 |
| BRHIP2017010 | 0.012188947 | 166776 | 7 |
| BRHIP2017315 | 0.00170946 | 140634 | 1 |
| BRHIP2017385 | 0.00170946 | 277604 | 1 |
| BRHIP2017404 | 0.00170946 | 98389 | 1 |
| BRHIP2017542 | 0.00170946 | 236099 | 1 |
| BRHIP2017553 | 0.00170946 | 241426 | 1 |
| BRHIP2017642 | 0.012973736 | 148973 | 6 |
| BRHIP2017714 | 0.00170946 | 127630 | 1 |
| BRHIP2017780 | 0.00170946 | 140992 | 1 |
| BRHIP2017945 | 0.051986696 | 163768 | 4 |
| BRHIP2018014 | 0.00170946 | 5665 | 1 |
| BRHIP2018369 | 0.00170946 | 198515 | 1 |
| BRHIP2018612 | 0.00170946 | 218737 | 1 |
| BRHIP2018635 | 0.002804738 | 18726 | 2 |
| BRHIP2018650 | 0.00341892 | 158261 | 2 |
| BRHIP2018712 | 0.08273766 | 112751 | 28 |
| BRHIP2018719 | 0.003397737 | 139645 | 2 |
| BRHIP2018805 | 0.00170946 | 149646 | 1 |
| BRHIP2018998 | 0.00170946 | 134678 | 1 |
| BRHIP2019007 | 0.00170946 | 146177 | 1 |
| BRHIP2019047 | 0.00170946 | 139731 | 1 |
| BRHIP2019149 | 0.015171807 | 124148 | 3 |
| BRHIP2019177 | 0.00170946 | 110480 | 1 |
| BRHIP2019186 | 0.084900897 | 33675 | 11 |
| BRHIP2019291 | 0.00170946 | 281799 | 1 |
| BRHIP2019494 | 0.004608165 | 129121 | 3 |
| BRHIP2019589 | 0.00170946 | 68568 | 1 |
| BRHIP2019641 | 0.00341892 | 187186 | 2 |
| BRHIP2019819 | 0.019324521 | 134898 | 10 |
| BRHIP2019884 | 0.00170946 | 249651 | 1 |
| BRHIP2019972 | 0.00341892 | 192726 | 2 |
| BRHIP2020182 | 0.00170946 | 23948 | 1 |
| BRHIP2020509 | 0.008948078 | 59863 | 3 |
| BRHIP2020573 | 0.01851457 | 94284 | 9 |
| BRHIP2020622 | 0.007241821 | 151154 | 3 |
| BRHIP2020695 | 0.003385007 | 166153 | 2 |
| BRHIP2020743 | 0.00170946 | 187389 | 1 |
| BRHIP2020799 | 0.033107653 | 44218 | 18 |
| BRHIP2020827 | 0.026065865 | 132980 | 13 |
| BRHIP2020842 | 0.00341892 | 143639 | 2 |
| BRHIP2020859 | 0.00170946 | 216464 | 1 |
| BRHIP2020930 | 0.00170946 | 190847 | 1 |
| BRHIP2021102 | 0.00170946 | 166796 | 1 |
| BRHIP2021289 | 0.00170946 | 173214 | 1 |
| BRHIP2021495 | 0.026123956 | 118824 | 7 |
| BRHIP2021615 | 0.00341892 | 189293 | 2 |
| BRHIP2021744 | 0.00170946 | 187775 | 1 |
| BRHIP2021762 | 0.060142531 | 89523 | 21 |
| BRHIP2021858 | 0.050070972 | 99622 | 9 |
| BRHIP2021870 | 0.037738375 | 57100 | 11 |
| BRHIP2021929 | 0.00170946 | 115180 | 1 |
| BRHIP2022006 | 0.00170946 | 116182 | 1 |
| BRHIP2022054 | 0.068041549 | 117359 | 10 |
| BRHIP2022221 | 0.003567575 | 205217 | 2 |
| BRHIP2022228 | 0.018336685 | 18904 | 9 |
| BRHIP2022298 | 0.00170946 | 146534 | 1 |
| BRHIP2022326 | 0.196957662 | 123190 | 39 |
| BRHIP2022467 | 0.011789902 | 157907 | 3 |
| BRHIP2022708 | 0.00170946 | 136370 | 1 |
| BRHIP2022986 | 0.058886507 | 33672 | 16 |
| BRHIP2023071 | 0.003397737 | 172433 | 2 |
| BRHIP2023229 | 0.011163937 | 101098 | 2 |
| BRHIP2023272 | 0.049644331 | 52563 | 4 |
| BRHIP2023292 | 0.004804956 | 182910 | 2 |
| BRHIP2023309 | 0.014362873 | 168908 | 6 |
| BRHIP2023369 | 0.009587327 | 96335 | 3 |
| BRHIP2023438 | 0.006568261 | 86980 | 3 |
| BRHIP2023695 | 0.004691419 | 152755 | 2 |
| BRHIP2023735 | 0.00170946 | 210654 | 1 |
| BRHIP2023762 | 0.070211287 | 67922 | 10 |
| BRHIP2023773 | 0.005886395 | 86378 | 2 |
| BRHIP2023860 | 0.00170946 | 206783 | 1 |
| BRHIP2023869 | 0.004662853 | 228725 | 3 |
| BRHIP2023888 | 0.455384502 | 77486 | 118 |
| BRHIP2024046 | 0.005463143 | 150099 | 3 |
| BRHIP2024146 | 0.550803718 | 46185 | 133 |
| BRHIP2024165 | 0.00170946 | 279107 | 1 |
| BRHIP2024201 | 0.00170946 | 137328 | 1 |
| BRHIP2024347 | 0.00170946 | 241176 | 1 |
| BRHIP2024398 | 0.019900997 | 98223 | 9 |
| BRHIP2024549 | 0.00170946 | 118391 | 1 |
| BRHIP2024742 | 0.134347487 | 50167 | 50 |
| BRHIP2024789 | 0.00341892 | 189370 | 2 |
| BRHIP2024911 | 0.018077944 | 114397 | 8 |
| BRHIP2024941 | 0.00170946 | 282810 | 1 |
| BRHIP2025245 | 0.003586302 | 51623 | 2 |
| BRHIP2025366 | 0.00170946 | 149074 | 1 |
| BRHIP2025448 | 0.005479249 | 203737 | 3 |
| BRHIP2025679 | 0.002898705 | 119403 | 2 |
| BRHIP2025739 | 0.00170946 | 227691 | 1 |
| BRHIP2025797 | 0.00170946 | 78509 | 1 |
| BRHIP2026021 | 0.00170946 | 62680 | 1 |
| BRHIP2026061 | 0.005479249 | 205812 | 3 |
| BRHIP2026155 | 0.00170946 | 174215 | 1 |
| BRHIP2026214 | 0.004804956 | 157216 | 2 |
| BRHIP2026274 | 0.018249654 | 218809 | 5 |
| BRHIP2026288 | 0.007763828 | 116278 | 2 |
| BRHIP2026346 | 0.00170946 | 144555 | 1 |
| BRHIP2026557 | 0.00170946 | 173254 | 1 |
| BRHIP2026723 | 0.00170946 | 229414 | 1 |
| BRHIP2026877 | 0.005553544 | 162037. | 2 |
| BRHIP2027017 | 0.014555181 | 134600 | 6 |
| BRHIP2027054 | 0.00170946 | 278982 | 1 |
| BRHIP2027077 | 0.082954585 | 126331 | 13 |
| BRHIP2027563 | 0.00170946 | 221371 | 1 |
| BRHIP2027685 | 0.003397737 | 272625 | 2 |
| BRHIP2027762 | 0.00170946 | 187405 | 1 |
| BRHIP2028187 | 0.004608165 | 156486 | 3 |
| BRHIP2028303 | 0.00170946 | 15203 | 1 |
| BRHIP2028330 | 0.00170946 | 189077 | 1 |
| BRHIP2028480 | 0.00170946 | 165857 | 1 |
| BRHIP2028583 | 0.013470015 | 142951 | 2 |
| BRHIP2028593 | 0.00170946 | 150512 | 1 |
| BRHIP2029176 | 0.00170946 | 195499 | 1 |
| BRHIP2029283 | 0.00170946 | 249330 | 1 |
| BRHIP2029393 | 0.00170946 | 224694 | 1 |
| BRHIP2029529 | 0.00170946 | 27646 | 1 |
| BRHIP2029643 | 0.011216575 | 155782 | 4 |
| BRHIP2029663 | 0.003790972 | 175923 | 2 |
| BRHIP3000017 | 0.038000524 | 75906 | 14 |
| BRHIP3000060 | 0.003567575 | 148628 | 2 |
| BRHIP3000070 | 0.00170946 | 210344 | 1 |
| BRHIP3000081 | 0.00512838 | 118437 | 3 |
| BRHIP3000111 | 0.163667808 | 24064 | 26 |
| BRHIP3000131 | 0.045627167 | 103307 | 19 |
| BRHIP3000240 | 0.005277194 | 60392 | 4 |
| BRHIP3000339 | 0.398667125 | 6827 | 161 |
| BRHIP3000377 | 0.029087086 | 174714 | 5 |
| BRHIP3000457 | 0.006169461 | 204613 | 4 |
| BRHIP3000473 | 0.034647887 | 46754 | 15 |
| BRHIP3000475 | 0.007749326 | 252971 | 4 |
| BRHIP3000488 | 0.00341892 | 149552 | 2 |
| BRHIP3000526 | 0.00170946 | 240924 | 1 |
| BRHIP3000626 | 0.00170946 | 251484 | 1 |
| BRHIP3000859 | 0.00170946 | 224748 | 1 |
| BRHIP3000907 | 0.00170946 | 235965 | 1 |
| BRHIP3001076 | 0.003707861 | 85300 | 2 |
| BRHIP3001079 | 0.00170946 | 276480 | 1 |
| BRHIP3001141 | 0.00170946 | 260374 | 1 |
| BRHIP3001283 | 0.003397737 | 197725 | 2 |
| BRHIP3001338 | 0.00170946 | 245641 | 1 |
| BRHIP3001360 | 0.002898705 | 118453 | 2 |
| BRHIP3001481 | 0.008895312 | 125404 | 7 |
| BRHIP3001573 | 0.020538448 | 66030 | 11 |
| BRHIP3001878 | 0.00170946 | 278106 | 1 |
| BRHIP3001964 | 0.00170946 | 260235 | 1 |
| BRHIP3002000 | 0.223425027 | 89444 | 65 |
| BRHIP3002114 | 0.00429586 | 177731 | 3 |
| BRHIP3002124 | 0.00170946 | 210949 | 1 |
| BRHIP3002141 | 0.010679107 | 124253 | 5 |
| BRHIP3002363 | 0.00170946 | 253318 | 1 |
| BRHIP3002691 | 0.020202753 | 122270 | 8 |
| BRHIP3002920 | 0.005277035 | 151611 | 3 |
| BRHIP3002931 | 0.00170946 | 223015 | 1 |
| BRHIP3003063 | 0.015287678 | 50029 | 9 |
| BRHIP3003126 | 0.004608165 | 80595 | 3 |
| BRHIP3003306 | 0.002804738 | 259995 | 2 |
| BRHIP3003340 | 0.00170946 | 247561 | 1 |
| BRHIP3003395 | 0.00170946 | 254955 | 1 |
| BRHIP3003484 | 0.00170946 | 264212 | 1 |
| BRHIP3003644 | 0.00170946 | 242845 | 1 |
| BRHIP3003688 | 0.00170946 | 229131 | 1 |
| BRHIP3003795 | 0.003790972 | 266307 | 2 |
| BRHIP3003845 | 0.015866389 | 91566 | 3 |
| BRHIP3003916 | 0.027035576 | 142685 | 13 |
| BRHIP3003961 | 0.008834056 | 155207 | 5 |
| BRHIP3003984 | 0.012195862 | 118711 | 8 |
| BRHIP3004193 | 0.00170946 | 165881 | 1 |
| BRHIP3004215 | 0.012341912 | 70616 | 6 |
| BRHIP3004416 | 0.00170946 | 75491 | 1 |
| BRHIP3004710 | 0.00170946 | 104512 | 1 |
| BRHIP3004725 | 0.00170946 | 197711 | 1 |
| BRHIP3004774 | 0.008479475 | 136828 | 5 |
| BRHIP3004786 | 0.00170946 | 212343 | 1 |
| BRHIP3004863 | 0.00170946 | 228898 | 1 |
| BRHIP3004968 | 0.004980217 | 168537 | 3 |
| BRHIP3005037 | 0.006223659 | 40997 | 4 |
| BRHIP3005137 | 0.00170946 | 260046 | 1 |
| BRHIP3005142 | 0.002804738 | 252493 | 2 |
| BRHIP3005231 | 0.00170946 | 104514 | 1 |
| BRHIP3005307 | 0.003707861 | 186763 | 2 |
| BRHIP3005574 | 0.011891968 | 152925 | 7 |
| BRHIP3005673 | 0.006504352 | 116820 | 4 |
| BRHIP3005768 | 0.003567575 | 205685 | 2 |
| BRHIP3005801 | 0.008940271 | 43547 | 3 |
| BRHIP3005944 | 0.008547301 | 124239 | 5 |
| BRHIP3006279 | 0.00170946 | 208068 | 1 |
| BRHIP3006294 | 0.004201894 | 94675 | 3 |
| BRHIP3006390 | 0.003385007 | 159603 | 2 |
| BRHIP3006449 | 0.00170946 | 116740 | 1 |
| BRHIP3006683 | 0.006952582 | 92246 | 4 |
| BRHIP3006717 | 0.098443203 | 87795 | 35 |
| BRHIP3006786 | 0.00170946 | 49040 | 1 |
| BRHIP3006950 | 0.00170946 | 265026 | 1 |
| BRHIP3007172 | 0.00170946 | 246962 | 1 |
| BRHIP3007183 | 0.012223166 | 17467 | 6 |
| BRHIP3007195 | 0.004514199 | 140090 | 3 |
| BRHIP3007223 | 0.083103968 | 39599 | 19 |
| BRHIP3007239 | 0.00170946 | 217039 | 1 |
| BRHIP3007291 | 0.00170946 | 202817 | 1 |
| BRHIP3007409 | 0.005243122 | 118506 | 3 |
| BRHIP3007424 | 0.00170946 | 140000 | 1 |
| BRHIP3007483 | 0.00170946 | 217103 | 1 |
| BRHIP3007586 | 0.048553699 | 136122 | 23 |
| BRHIP3007609 | 0.00170946 | 280652 | 1 |
| BRHIP3007640 | 0.007506869 | 48333 | 5 |
| BRHIP3007834 | 0.048662157 | 122393 | 22 |
| BRHIP3007960 | 0.00170946 | 240423 | 1 |
| BRHIP3008082 | 0.003790972 | 189488 | 2 |
| BRHIP3008183 | 0.00170946 | 82140 | 1 |
| BRHIP3008313 | 0.005870825 | 229667 | 4 |
| BRHIP3008314 | 0.00170946 | 51827 | 1 |
| BRHIP3008320 | 0.007175979 | 114378 | 4 |
| BRHIP3008344 | 0.003707861 | 221258 | 2 |
| BRHIP3008405 | 0.009523796 | 157043 | 6 |
| BRHIP3008565 | 0.003707861 | 174931 | 2 |
| BRHIP3008598 | 0.00170946 | 133153 | 1 |
| BRHIP3008606 | 0.00170946 | 143870 | 1 |
| BRHIP3008691 | 0.003385007 | 150203 | 2 |
| BRHIP3008714 | 0.00170946 | 221000 | 1 |
| BRHIP3008997 | 0.00170946 | 78492 | 1 |
| BRHIP3009099 | 0.004804956 | 212905 | 2 |
| BRHIP3009140 | 0.00170946 | 258695 | 1 |
| BRHIP3009181 | 0.00170946 | 258703 | 1 |
| BRHIP3009318 | 0.00170946 | 264633 | 1 |
| BRHIP3009427 | 0.00170946 | 240851 | 1 |
| BRHIP3009448 | 0.00170946 | 269727 | 1 |
| BRHIP3009672 | 0.00170946 | 273146 | 1 |
| BRHIP3009753 | 0.143956971 | 259646 | 2 |
| BRHIP3010135 | 0.00170946 | 232047 | 1 |
| BRHIP3010289 | 0.00170946 | 186967 | 1 |
| BRHIP3010529 | 0.00170946 | 232037 | 1 |
| BRHIP3010916 | 0.003790972 | 94696 | 2 |
| BRHIP3011082 | 0.00170946 | 83359 | 1 |
| BRHIP3011241 | 0.00170946 | 212394 | 1 |
| BRHIP3011269 | 0.00341892 | 93536 | 2 |
| BRHIP3011460 | 0.00170946 | 141687 | 1 |
| BRHIP3011567 | 0.00170946 | 269883 | 1 |
| BRHIP3011831 | 0.00170946 | 271999 | 1 |
| BRHIP3012185 | 0.00170946 | 107345 | 1 |
| BRHIP3012289 | 0.00170946 | 266237 | 1 |
| BRHIP3012357 | 0.00170946 | 271523 | 1 |
| BRHIP3012736 | 0.005086013 | 132938 | 3 |
| BRHIP3012745 | 0.00170946 | 210226 | 1 |
| BRHIP3012868 | 0.00170946 | 272345 | 1 |
| BRHIP3012997 | 0.180797099 | 37543 | 9 |
| BRHIP3013078 | 0.00170946 | 245492 | 1 |
| BRHIP3013271 | 0.00170946 | 276773 | 1 |
| BRHIP3013588 | 0.002804738 | 107102 | 2 |
| BRHIP3013698 | 0.00170946 | 245591 | 1 |
| BRHIP3013765 | 0.00170946 | 94917 | 1 |
| BRHIP3013897 | 0.00170946 | 222369 | 1 |
| BRHIP3014237 | 0.00170946 | 195548 | 1 |
| BRHIP3014675 | 0.008836199 | 131462 | 6 |
| BRHIP3015620 | 0.00170946 | 101894 | 1 |
| BRHIP3015751 | 0.00170946 | 270376 | 1 |
| BRHIP3015854 | 0.00170946 | 186322 | 1 |
| BRHIP3016032 | 0.00170946 | 173390 | 1 |
| BRHIP3016213 | 0.00170946 | 149338 | 1 |
| BRHIP3016302 | 0.00170946 | 157213 | 1 |
| BRHIP3016421 | 0.00170946 | 105308 | 1 |
| BRHIP3017109 | 0.00170946 | 208100 | 1 |
| BRHIP3017146 | 0.005107197 | 149132 | 3 |
| BRHIP3017256 | 0.00170946 | 206318 | 1 |
| BRHIP3017325 | 0.00170946 | 282488 | 1 |
| BRHIP3017558 | 0.00170946 | 110149 | 1 |
| BRHIP3017637 | 0.00170946 | 213396 | 1 |
| BRHIP3017855 | 0.043759235 | 14626 | 26 |
| BRHIP3018278 | 0.00429586 | 171897 | 3 |
| BRHIP3018784 | 0.048353746 | 138992 | 10 |
| BRHIP3018797 | 0.125082366 | 84438 | 62 |
| BRHIP3019643 | 0.00170946 | 247653 | 1 |
| BRHIP3019824 | 0.00170946 | 279665 | 1 |
| BRHIP3019880 | 0.00170946 | 237470 | 1 |
| BRHIP3019956 | 0.00170946 | 210038 | 1 |
| BRHIP3020046 | 0.165417468 | 124270 | 15 |
| BRHIP3020155 | 0.00170946 | 150487 | 1 |
| BRHIP3020182 | 0.00170946 | 258319 | 1 |
| BRHIP3020733 | 0.003106616 | 274810 | 2 |
| BRHIP3021019 | 0.021635132 | 94036 | 9 |
| BRHIP3021499 | 0.00170946 | 228855 | 1 |
| BRHIP3021534 | 0.00170946 | 275436 | 1 |
| BRHIP3021778 | 0.00170946 | 243641 | 1 |
| BRHIP3021987 | 0.004691419 | 102623 | 2 |
| BRHIP3022656 | 0.00170946 | 87459 | 1 |
| BRHIP3023922 | 0.00170946 | 110544 | 1 |
| BRHIP3024118 - | 0.00170946 | 269579 | 1 |
| BRHIP3024533 | 0.00170946 | 277394 | 1 |
| BRHIP3024703 | 0.00170946 | 271798 | 1 |
| BRHIP3024725 | 0.00170946 | 224006 | 1 |
| BRHIP3024820 | 0.00170946 | 214661 | 1 |
| BRHIP3025161 | 0.00170946 | 226124 | 1 |
| BRHIP3025702 | 0.00170946 | 231785 | 1 |
| BRHIP3025795 | 0.00170946 | 94107 | 1 |
| BRHIP3025844 | 0.00170946 | 220907 | 1 |
| BRHIP3026052 | 0.011182749 | 90953 | 4 |
| BRHIP3026097 | 0.00170946 | 267330 | 1 |
| BRHIP3026231 | 0.00170946 | 278214 | 1 |
| BRHIP3026335 | 0.00170946 | 80649 | 1 |
| BRHIP3026651 | 0.00170946 | 228916 | 1 |
| BRHIP3027105 | 0.003385007 | 190048 | 2 |
| BRHIP3027137 | 0.00170946 | 210699 | 1 |
| BRHIP3027160 | 0.00341892 | 213769 | 2 |
| BRHIP3027191 | 0.00170946 | 72949 | 1 |
| BRHIP3027501 | 0.00170946 | 269793 | 1 |
| BRHIP3027594 | 0.008619675 | 235362 | 5 |
| BRHIP3027651 | 0.00170946 | 80535 | 1 |
| BRHIP3027854 | 0.00170946 | 243843 | 1 |
| BRHIP3027947 | 0.00341892 | 251125 | 2 |
| BRHIP3028246 | 0.007565717 | 154250 | 5 |
| BRHIP3028570 | 0.005243122 | 139192 | 3 |
| BRHIP3028742 | 0.00170946 | 236522 | 1 |
| BRSSN1000056 | 0.027656288 | 186429 | 3 |
| BRSSN1000059 | 0.012449468 | 88729 | 2 |
| BRSSN1000092 | 0.006236358 | 192844 | 1 |
| BRSSN1000153 | 0.006236358 | 187396 | 1 |
| BRSSN2000097 | 0.024199233 | 125506 | 3 |
| BRSSN2000175 | 0.006236358 | 181614 | 1 |
| BRSSN2000197 | 0.007633513 | 146613 | 2 |
| BRSSN2000244 | 0.012438678 | 131125 | 2 |
| BRSSN2000293 | 0.051094821 | 134194 | 11 |
| BRSSN2000295 | 0.024193313 | 145070 | 8 |
| BRSSN2000312 | 0.015573199 | 150566 | 6 |
| BRSSN2000498 | 0.153123455 | 87722 | 34 |
| BRSSN2000518 | 0.006236358 | 145645 | 1 |
| BRSSN2000561 | 0.026721366 | 139273 | 9 |
| BRSSN2000566 | 0.034447498 | 106221 | 13 |
| BRSSN2000684 | 0.035751595 | 97160 | 17 |
| BRSSN2000715 | 0.006236358 | 43038 | 1 |
| BRSSN2000832 | 0.013917225 | 159332 | 6 |
| BRSSN2001213 | 0.008234759 | 116356 | 2 |
| BRSSN2001275 | 0.010080442 | 164175 | 2 |
| BRSSN2001342 | 0.006236358 | 64260 | 1 |
| BRSSN2001426 | 0.006236358 | 137157 | 1 |
| BRSSN2001503 | 0.006236358 | 275065 | 1 |
| BRSSN2001579 | 0.012438678 | 74274 | 2 |
| BRSSN2001758 | 0.017823087 | 101268 | 4 |
| BRSSN2001810 | 0.006236358 | 197379 | 1 |
| BRSSN2001869 | 0.055510128 | 115027 | 20 |
| BRSSN2002160 | 0.009507114 | 176333 | 3 |
| BRSSN2002857 | 0.006236358 | 272943 | 1 |
| BRSSN2002859 | 0.006236358 | 273660 | 1 |
| BRSSN2003012 | 0.038733392 | 71520 | 7 |
| BRSSN2003086 | 0.006236358 | 144135 | 1 |
| BRSSN2003093 | 0.006236358 | 144275 | 1 |
| BRSSN2003820 | 0.006236358 | 148131 | 1 |
| BRSSN2003841 | 0.006236358 | 246032 | 1 |
| BRSSN2003937 | 0.006236358 | 184655 | 1 |
| BRSSN2004142 | 0.006236358 | 159642 | 1 |
| BRSSN2004303 | 0.006236358 | 167085 | 1 |
| BRSSN2004304 | 0.006236358 | 69822 | 1 |
| BRSSN2004496 | 0.007924635 | 165569 | 2 |
| BRSSN2004657 | 0.007331636 | 92659 | 2 |
| BRSSN2004679 | 0.011009329 | 123585 | 3 |
| BRSSN2004686 | 0.006236358 | 49979 | 1 |
| BRSSN2004710 | 0.006236358 | 187801 | 1 |
| BRSSN2004719 | 0.031979606 | 141965 | 7 |
| BRSSN2005847 | 0.007633513 | 151978 | 2 |
| BRSSN2006133 | 0.006236358 | 154482 | 1 |
| BRSSN2006390 | 0.006236358 | 203026 | 1 |
| BRSSN2006575 | 0.006236358 | 136185 | 1 |
| BRSSN2006611 | 0.007924635 | 131281 | 2 |
| BRSSN2006644 | 0.006236358 | 148457 | 1 |
| BRSSN2006892 | 0.010821159 | 143769 | 4 |
| BRSSN2007076 | 0.006236358 | 142064 | 1 |
| BRSSN2007168 | 0.006236358 | 155527 | 1 |
| BRSSN2007202 | 0.010080442 | 150100 | 2 |
| BRSSN2007338 | 0.022669981 | 140230 | 4 |
| BRSSN2007464 | 0.040944704 | 65759 | 4 |
| BRSSN2007504 | 0.006236358 | 243470 | 1 |
| BRSSN2007819 | 0.006236358 | 201862 | 1 |
| BRSSN2008125 | 0.006236358 | 50333 | 1 |
| BRSSN2008419 | 0.006236358 | 175644 | 1 |
| BRSSN2008464 | 0.006236358 | 246347 | 1 |
| BRSSN2008549 | 0.009803933 | 130361 | 3 |
| BRSSN2008797 | 0.008094472 | 176121 | 2 |
| BRSSN2009119 | 0.009151215 | 49353 | 2 |
| BRSSN2009244 | 0.194213688 | 79808 | 4 |
| BRSSN2009378 | 0.006236358 | 191776 | 1 |
| BRSSN2009389 | 0.006236358 | 186636 | 1 |
| BRSSN2009395 | 0.006236358 | 211859 | 1 |
| BRSSN2009518 | 0.09727873 | 61927 | 23 |
| BRSSN2009627 | 0.006236358 | 112261 | 1 |
| BRSSN2009630 | 0.006236358 | 132259 | 1 |
| BRSSN2009702 | 0.006236358 | 158373 | 1 |
| BRSSN2010019 | 0.014437079 | 132968 | 3 |
| BRSSN2010110 | 0.006236358 | 159520 | 1 |
| BRSSN2010587 | 0.007425602 | 131033 | 2 |
| BRSSN2010596 | 0.013979003 | 217318 | 3 |
| BRSSN2010830 | 0.006236358 | 199624 | 1 |
| BRSSN2011262 | 0.059453458 | 36357 | 7 |
| BRSSN2011653 | 0.033815049 | 52320 | 8 |
| BRSSN2011738 | 0.015930911 | 134521 | 7 |
| BRSSN2011799 | 0.060054739 | 141469 | 10 |
| BRSSN2011843 | 0.013999766 | 135118 | 5 |
| BRSSN2012081 | 0.006236358 | 98673 | 1 |
| BRSSN2012103 | 0.006236358 | 123195 | 1 |
| BRSSN2012157 | 0.006236358 | 206979 | 1 |
| BRSSN2012198 | 0.006236358 | 155352 | 1 |
| BRSSN2012254 | 0.006236358 | 31976 | 1 |
| BRSSN2012439 | 0.007633513 | 76028 | 2 |
| BRSSN2012622 | 0.006236358 | 183629 | 1 |
| BRSSN2012691 | 0.006236358 | 168893 | 1 |
| BRSSN2012838 | 0.006236358 | 228888 | 1 |
| BRSSN2013095 | 0.007945818 | 47694 | 2 |
| BRSSN2013544 | 0.006236358 | 281374 | 1 |
| BRSSN2013696 | 0.046913263 | 50403 | 19 |
| BRSSN2013702 | 0.017662866 | 224348 | 4 |
| BRSSN2013733 | 0.007924635 | 125854 | 2 |
| BRSSN2013753 | 0.011362752 | 169855 | 3 |
| BRSSN2013874 | 0.006236358 | 133218 | 1 |
| BRSSN2014112 | 0.006236358 | 121880 | 1 |
| BRSSN2014294 | 0.017996913 | 137087 | 2 |
| BRSSN2014299 | 0.045344114 | 51217 | 10 |
| BRSSN2014424 | 0.010080442 | 43088 | 2 |
| BRSSN2014513 | 0.006236358 | 245942 | 1 |
| BRSSN2014556 | 0.006236358 | 162834 | 1 |
| BRSSN2014573 | 0.006236358 | 262723 | 1 |
| BRSSN2014610 | 0.046931868 | 114219 | 19 |
| BRSSN2014636 | 0.006236358 | 227598 | 1 |
| BRSSN2014685 | 0.011799878 | 117723 | 4 |
| BRSSN2015199 | 0.007425602 | 128489 | 2 |
| BRSSN2015238 | 0.048945168 | 137301 | 17 |
| BRSSN2015369 | 0.006236358 | 129318 | 1 |
| BRSSN2015493 | 0.045840127 | 58109 | 5 |
| BRSSN2015497 | 0.023428368 | 104848 | 16 |
| BRSSN2015554 | 0.012172133 | 83197 | 2 |
| BRSSN2015707 | 0.006236358 | 70611 | 1 |
| BRSSN2015773 | 0.006236358 | 145073 | 1 |
| BRSSN2015907 | 0.006236358 | 159863 | 1 |
| BRSSN2015982 | 0.010313595 | 35466 | 3 |
| BRSSN2016589 | 0.006236358 | 143329 | 1 |
| BRSSN2016768 | 0.012472716 | 208455 | 2 |
| BRSSN2016905 | 0.006236358 | 167075 | 1 |
| BRSSN2017297 | 0.006236358 | 210890 | 1 |
| BRSSN2017422 | 0.01165368 | 214642 | 4 |
| BRSSN2017530 | 0.006236358 | 176547 | 1 |
| BRSSN2017682 | 0.006236358 | 176557 | 1 |
| BRSSN2017757 | 0.006236358 | 220412 | 1 |
| BRSSN2018218 | 0.006236358 | 130807 | 1 |
| BRSSN2018440 | 0.00831787 | 72053 | 2 |
| BRSSN2018447 | 0.025638602 | 119395 | 10 |
| BRSSN2018581 | 0.012438678 | 147007 | 2 |
| BRSSN2018731 | 0.006236358 | 125747 | 1 |
| BRSSN2018771 | 0.006236358 | 176447 | 1 |
| BRSSN2018925 | 0.006236358 | 181854 | 1 |
| BRSTN1000083 | 0.262421535 | 94790 | 41 |
| BRSTN1000097 | 0.006041566 | 65953 | 1 |
| BRSTN2000058 | 0.069460404 | 49960 | 16 |
| BRSTN2000070 | 0.007751026 | 163797 | 2 |
| BRSTN2000220 | 0.048836214 | 143433 | 18 |
| BRSTN2000312 | 0.006041566 | 238650 | 1 |
| BRSTN2000367 | 0.007136844 | 171047 | 2 |
| BRSTN2000536 | 0.007751026 | 132193 | 2 |
| BRSTN2000872 | 0.006041566 | 51603 | 1 |
| BRSTN2001067 | 0.027064268 | 29260 | 6 |
| BRSTN2001613 | 0.006041566 | 148979 | 1 |
| BRSTN2001801 | 0.006041566 | 244595 | 1 |
| BRSTN2002105 | 0.006041566 | 190995 | 1 |
| BRSTN2002400 | 0.006041566 | 169593 | 1 |
| BRSTN2002532 | 0.006041566 | 2576 | 1 |
| BRSTN2002691 | 0.006041566 | 242370 | 1 |
| BRSTN2003590 | 0.006041566 | 195725 | 1 |
| BRSTN2003835 | 0.006041566 | 152669 | 1 |
| BRSTN2004863 | 0.016817414 | 91234 | 4 |
| BRSTN2004869 | 0.007918408 | 79994 | 2 |
| BRSTN2004987 | 0.006041566 | 155975 | 1 |
| BRSTN2005721 | 0.006041566 | 53315 | 1 |
| BRSTN2006134 | 0.008956423 | 208265 | 2 |
| BRSTN2006306 | 0.006041566 | 214754 | 1 |
| BRSTN2006466 | 0.006041566 | 271293 | 1 |
| BRSTN2006580 | 0.00723081 | 189831 | 2 |
| BRSTN2006583 | 0.00789968 | 81364 | 2 |
| BRSTN2006638 | 0.006041566 | 284114 | 1 |
| BRSTN2006865 | 0.006041566 | 147851 | 1 |
| BRSTN2007000 | 0.006041566 | 219546 | 1 |
| BRSTN2007118 | 0.006041566 | 240062 | 1 |
| BRSTN2007284 | 0.00988565 | 102381 | 2 |
| BRSTN2007765 | 0.006041566 | 252330 | 1 |
| BRSTN2008052 | 0.015304811 | 163440 | 7 |
| BRSTN2008283 | 0.006041566 | 237711 | 1 |
| BRSTN2008367 | 0.006041566 | 189295 | 1 |
| BRSTN2008418 | 0.006041566 | 81302 | 1 |
| BRSTN2008457 | 0.006041566 | 232356 | 1 |
| BRSTN2008475 | 0.006041566 | 232370 | 1 |
| BRSTN2008930 | 0.006041566 | 174103 | 1 |
| BRSTN2009247 | 0.006041566 | 166679 | 1 |
| BRSTN2010089 | 0.025396583 | 4319 | 8 |
| BRSTN2010107 | 0.500891046 | 1671 | 135 |
| BRSTN2010363 | 0.006041566 | 51123 | 1 |
| BRSTN2010416 | 0.006041566 | 283711 | 1 |
| BRSTN2010500 | 0.006041566 | 275957 | 1 |
| BRSTN2010569 | 0.006041566 | 27001 | 1 |
| BRSTN2010750 | 0.016472575 | 153391 | 6 |
| BRSTN2010923 | 0.006041566 | 215119 | 1 |
| BRSTN2011211 | 0.006041566 | 204863 | 1 |
| BRSTN2011597 | 0.00723081 | 189486 | 2 |
| BRSTN2011688 | 0.006041566 | 159393 | 1 |
| BRSTN2011799 | 0.006041566 | 167121 | 1 |
| BRSTN2011961 | 0.57856361 | 4537 | 64 |
| BRSTN2012069 | 17.71546276 | 31469 | 1260 |
| BRSTN2012089 | 0.028886371 | 83043 | 8 |
| BRSTN2012174 | 0.828439861 | 34274 | 85 |
| BRSTN2012284 | 0.006041566 | 219502 | 1 |
| BRSTN2012320 | 0.006041566 | 79763 | 1 |
| BRSTN2012380 | 0.006041566 | 268752 | 1 |
| BRSTN2012451 | 0.20286663 | 163701 | 7 |
| BRSTN2013171 | 0.048353119 | 99506 | 8 |
| BRSTN2013354 | 0.012083132 | 62736 | 2 |
| BRSTN2013502 | 0.068941636 | 116848 | 14 |
| BRSTN2013931 | 0.17342111 | 87169 | 14 |
| BRSTN2014490 | 0.018186009 | 234542 | 3 |
| BRSTN2014633 | 0.006041566 | 169243 | 1 |
| BRSTN2014751 | 0.006041566 | 201389 | 1 |
| BRSTN2014770 | 0.006041566 | 144964 | 1 |
| BRSTN2015015 | 0.011953716 | 139496 | 5 |
| BRSTN2015889 | 0.043256602 | 142464 | 15 |
| BRSTN2015978 | 5.522814481 | 6913 | 1008 |
| BRSTN2016066 | 0.006041566 | 229573 | 1 |
| BRSTN2016335 | 0.006041566 | 208565 | 1 |
| BRSTN2016470 | 0.765521402 | 78918 | 124 |
| BRSTN2016678 | 0.018261552 | 6374 | 3 |
| BRSTN2016892 | 0.056151978 | 99323 | 5 |
| BRSTN2016918 | 0.006041566 | 137234 | 1 |
| BRSTN2016992 | 0.340483632 | 93672 | 83 |
| BRSTN2017104 | 0.085935761 | 146119 | 12 |
| BRSTN2017151 | 0.006041566 | 133409 | 1 |
| BRSTN2017237 | 0.006041566 | 173095 | 1 |
| BRSTN2017379 | 0.006041566 | 1843 | 1 |
| BRSTN2017761 | 0.007918408 | 120371 | 2 |
| BRSTN2017771 | 0.006041566 | 173745 | 1 |
| BRSTN2017995 | 0.006041566 | 269660 | 1 |
| BRSTN2018077 | 0.00988565 | 128207 | 2 |
| BRSTN2018083 | 0.007751026 | 186991 | 2 |
| BRSTN2018596 | 0.006041566 | 173603 | 1 |
| BRSTN2018692 | 0.006041566 | 136281 | 1 |
| BRSTN2018712 | 0.006041566 | 275897 | 1 |
| BRSTN2019079 | 0.006041566 | 243556 | 1 |
| BRSTN2019129 | 0.006041566 | 235661 | 1 |
| BRTHA1000021 | 0.009756234 | 154304 | 5 |
| BRTHA1000063 | 0.001858114 | 142993 | 1 |
| BRTHA1000311 | 0.056784799 | 131833 | 27 |
| BRTHA2000057 | 0.003856516 | 191151 | 2 |
| BRTHA2000474 | 0.001858114 | 132639 | 1 |
| BRTHA2000514 | 0.001858114 | 231066 | 1 |
| BRTHA2000574 | 0.001858114 | 198864 | 1 |
| BRTHA2000855 | 0.001858114 | 195311 | 1 |
| BRTHA2000969 | 0.001858114 | 253804 | 1 |
| BRTHA2000972 | 0.001858114 | 253807 | 1 |
| BRTHA2001304 | 0.001858114 | 133019 | 1 |
| BRTHA2001370 | 0.001858114 | 103288 | 1 |
| BRTHA2001462 | 0.001858114 | 103371 | 1 |
| BRTHA2001717 | 0.001858114 | 237101 | 1 |
| BRTHA2001812 | 0.019660235 | 157418 | 3 |
| BRTHA2001953 | 0.017503822 | 174353 | 5 |
| BRTHA2002091 | 0.001858114 | 114392 | 1 |
| BRTHA2002115 | 0.001858114 | 231102 | 1 |
| BRTHA2002133 | 0.001858114 | 231132 | 1 |
| BRTHA2002281 | 0.001858114 | 240089 | 1 |
| BRTHA2002376 | 0.001858114 | 201758 | 1 |
| BRTHA2002442 | 0.005425689 | 209697 | 3 |
| BRTHA2002493 | 0.001858114 | 218275 | 1 |
| BRTHA2002565 | 0.001858114 | 225816 | 1 |
| BRTHA2002608 | 0.001858114 | 196607 | 1 |
| BRTHA2002702 | 0.001858114 | 103255 | 1 |
| BRTHA2002721 | 0.001858114 | 109357 | 1 |
| BRTHA2002726 | 0.007800238 | 101738 | 2 |
| BRTHA2002741 | 0.001858114 | 279940 | 1 |
| BRTHA2002808 | 0.014452573 | 103836 | 2 |
| BRTHA2002896 | 0.001858114 | 264368 | 1 |
| BRTHA2002903 | 0.001858114 | 264384 | 1 |
| BRTHA2003030 | 0.001858114 | 163926 | 1 |
| BRTHA2003110 | 0.001858114 | 249079 | 1 |
| BRTHA2003116 | 0.001858114 | 254352 | 1 |
| BRTHA2003436 | 0.001858114 | 95937 | 1 |
| BRTHA2003461 | 0.076340879 | 104645 | 11 |
| BRTHA2003759 | 0.001858114 | 189664 | 1 |
| BRTHA2003794 | 0.001858114 | 62315 | 1 |
| BRTHA2004350 | 0.041138741 | 14663 | 17 |
| BRTHA2004361 | 0.003856516 | 244165 | 2 |
| BRTHA2004371 | 0.001858114 | 241049 | 1 |
| BRTHA2004582 | 0.001858114 | 257605 | 1 |
| BRTHA2004629 | 0.001858114 | 69587 | 1 |
| BRTHA2004639 | 0.001858114 | 195374 | 1 |
| BRTHA2004642 | 0.001858114 | 211464 | 1 |
| BRTHA2004812 | 0.001858114 | 226051 | 1 |
| BRTHA2004821 | 0.001858114 | 175218 | 1 |
| BRTHA2004978 | 0.001858114 | 267648 | 1 |
| BRTHA2004992 | 0.001858114 | 252121 | 1 |
| BRTHA2005005 | 0.007432458 | 102370 | 4 |
| BRTHA2005013 | 0.015015825 | 102359 | 3 |
| BRTHA2005448 | 0.007432458 | 99099 | 4 |
| BRTHA2005579 | 0.001858114 | 259845 | 1 |
| BRTHA2005831 | 0.003856516 | 245772 | 2 |
| BRTHA2005864 | 0.103266523 | 134399 | 27 |
| BRTHA2005942 | 0.001858114 | 175483 | 1 |
| BRTHA2005956 | 0.001858114 | 219073 | 1 |
| BRTHA2006075 | 0.001858114 | 216815 | 1 |
| BRTHA2006146 | 0.001858114 | 228679 | 1 |
| BRTHA2006194 | 0.001858114 | 225960 | 1 |
| BRTHA2006232 | 0.001858114 | 211410 | 1 |
| BRTHA2006720 | 0.001858114 | 228104 | 1 |
| BRTHA2006735 | 0.001858114 | 250988 | 1 |
| BRTHA2006974 | 0.001858114 | 181051 | 1 |
| BRTHA2006975 | 0.073700379 | 181050 | 3 |
| BRTHA2007060 | 0.001858114 | 261826 | 1 |
| BRTHA2007122 | 0.001858114 | 144211 | 1 |
| BRTHA2007422 | 0.001858114 | 271107 | 1 |
| BRTHA2007603 | 0.001858114 | 207172 | 1 |
| BRTHA2007665 | 0.001858114 | 229738 | 1 |
| BRTHA2007998 | 0.194298765 | 63084 | 15 |
| BRTHA2008316 | 0.001858114 | 211650 | 1 |
| BRTHA2008335 | 0.020582403 | 94910 | 6 |
| BRTHA2008502 | 0.00774451 | 92286 | 3 |
| BRTHA2008513 | 0.003567575 | 177419 | 2 |
| BRTHA2008527 | 0.001858114 | 229687 | 1 |
| BRTHA2008535 | 0.001858114 | 185415 | 1 |
| BRTHA2008598 | 0.001858114 | 255151 | 1 |
| BRTHA2008669 | 0.001858114 | 267658 | 1 |
| BRTHA2008955 | 0.003047359 | 144589 | 2 |
| BRTHA2009311 | 0.001858114 | 166999 | 1 |
| BRTHA2009349 | 0.001858114 | 211638 | 1 |
| BRTHA2009486 | 0.01532813 | 157623 | 3 |
| BRTHA2009803 | 0.001858114 | 280740 | 1 |
| BRTHA2009846 | 0.001858114 | 200423 | 1 |
| BRTHA2009886 | 0.008060434 | 185690 | 2 |
| BRTHA2009972 | 0.001858114 | 93029 | 1 |
| BRTHA2010008 | 0.001858114 | 98357 | 1 |
| BRTHA2010033 | 0.001858114 | 96595 | 1 |
| BRTHA2010073 | 0.001858114 | 282860 | 1 |
| BRTHA2010097 | 0.039909865 | 105637 | 2 |
| BRTHA2010191 | 0.001858114 | 102568 | 1 |
| BRTHA2010397 | 0.001858114 | 266931 | 1 |
| BRTHA2010608 | 0.003856516 | 164610 | 2 |
| BRTHA2010672 | 0.001858114 | 193092 | 1 |
| BRTHA2010884 | 0.001858114 | 264909 | 1 |
| BRTHA2010907 | 0.001858114 | 555 | 1 |
| BRTHA2011062 | 0.001858114 | 192653 | 1 |
| BRTHA2011138 | 0.603047235 | 155783 | 12 |
| BRTHA2011194 | 0.003047359 | 217167 | 2 |
| BRTHA2011321 | 0.001858114 | 217136 | 1 |
| BRTHA2011351 | 0.001858114 | 102058 | 1 |
| BRTHA2011500 | 0.003716229 | 179680 | 2 |
| BRTHA2011641 | 0.003546391 | 195266 | 2 |
| BRTHA2011691 | 0.00325527 | 145364 | 2 |
| BRTHA2012129 | 0.001858114 | 142810 | 1 |
| BRTHA2012183 | 0.003856516 | 203312 | 2 |
| BRTHA2012189 | 0.001858114 | 214683 | 1 |
| BRTHA2012351 | 0.001858114 | 177092 | 1 |
| BRTHA2012392 | 0.004840074 | 112107 | 2 |
| BRTHA2012466 | 0.003716229 | 93930 | 2 |
| BRTHA2012555 | 0.001858114 | 252381 | 1 |
| BRTHA2012562 | 0.001858114 | 243552 | 1 |
| BRTHA2012714 | 0.133679029 | 5064 | 22 |
| BRTHA2012980 | 0.013377525 | 185533 | 2 |
| BRTHA2013054 | 0.027893359 | 48870 | 19 |
| BRTHA2013262 | 0.001858114 | 266515 | 1 |
| BRTHA2013275 | 0.003734956 | 241994 | 2 |
| BRTHA2013426 | 0.001858114 | 247578 | 1 |
| BRTHA2013460 | 0.001858114 | 252305 | 1 |
| BRTHA2013515 | 0.003533661 | 119126 | 2 |
| BRTHA2013540 | 0.004943546 | 197960 | 3 |
| BRTHA2013595 | 0.001858114 | 247598 | 1 |
| BRTHA2013610 | 0.001858114 | 243614 | 1 |
| BRTHA2013707 | 0.001858114 | 247505 | 1 |
| BRTHA2013942 | 0.001858114 | 77541 | 1 |
| BRTHA2014287 | 0.001858114 | 95391 | 1 |
| BRTHA2014334 | 0.001858114 | 242123 | 1 |
| BRTHA2014381 | 0.001858114 | 254585 | 1 |
| BRTHA2014578 | 0.001858114 | 246118 | 1 |
| BRTHA2014647 | 0.001858114 | 272576 | 1 |
| BRTHA2014663 | 0.009457589 | 167559 | 3 |
| BRTHA2014792 | 0.001858114 | 258150 | 1 |
| BRTHA2014828 | 0.001858114 | 258121 | 1 |
| BRTHA2014909 | 0.001858114 | 102542 | 1 |
| BRTHA2014975 | 0.001858114 | 97542 | 1 |
| BRTHA2014997 | 0.00325527 | 101663 | 2 |
| BRTHA2015406 | 0.001858114 | 230120 | 1 |
| BRTHA2015478 | 0.001858114 | 170434 | 1 |
| BRTHA2015696 | 0.001858114 | 218651 | 1 |
| BRTHA2015700 | 0.001858114 | 236623 | 1 |
| BRTHA2015853 | 0.001858114 | 214472 | 1 |
| BRTHA2015878 | 0.001858114 | 138376 | 1 |
| BRTHA2016179 | 0.001858114 | 214927 | 1 |
| BRTHA2016215 | 0.004772971 | 151443 | 2 |
| BRTHA2016267 | 0.001858114 | 202409 | 1 |
| BRTHA2016318 | 0.001858114 | 182204 | 1 |
| BRTHA2016496 | 0.001858114 | 247417 | 1 |
| BRTHA2016543 | 0.001858114 | 99657 | 1 |
| BRTHA2016552 | 0.001858114 | 271066 | 1 |
| BRTHA2016577 | 0.071012737 | 94909 | 12 |
| BRTHA2016765 | 0.014240074 | 231452 | 3 |
| BRTHA2016918 | 0.017093753 | 102338 | 4 |
| BRTHA2017178 | 0.003856516 | 202308 | 2 |
| BRTHA2017353 | 0.001858114 | 171429. | 1 |
| BRTHA2017364 | 0.001858114 | 197914 | 1 |
| BRTHA2017972 | 0.009929338 | 177613 | 3 |
| BRTHA2017985 | 0.003734956 | 92516 | 2 |
| BRTHA2018011 | 0.003716229 | 138317 | 2 |
| BRTHA2018129 | 0.001858114 | 50830 | 1 |
| BRTHA2018165 | 0.001858114 | 100289 | 1 |
| BRTHA2018304 | 0.001858114 | 96986 | 1 |
| BRTHA2018344 | 0.001858114 | 202403 | 1 |
| BRTHA2018420 | 0.001858114 | 231424 | 1 |
| BRTHA2018443 | 0.007582332 | 144708 | 5 |
| BRTHA2018504 | 0.004930817 | 220141 | 3 |
| BRTHA2018558 | 0.001858114 | 270957 | 1 |
| BRTHA2018591 | 0.001858114 | 59186 | 1 |
| BRTHA2018624 | 0.001858114 | 180293 | 1 |
| BRTHA2018706 | 0.001858114 | 209675 | 1 |
| BRTHA2018707 | 0.00325527 | 136620 | 2 |
| BRTHA2019014 | 0.001858114 | 101362 | 1 |
| BRTHA2019022 | 0.001858114 | 103155 | 1 |
| BRTHA2019048 | 0.001858114 | 97789 | 1 |
| BRTHA2019073 | 0.00325527 | 101754 | 2 |
| BRTHA2019726 | 0.001858114 | 266333 | 1 |
| BRTHA2019743 | 0.001858114 | 201797 | 1 |
| BRTHA2020400 | 0.001858114 | 164964 | 1 |
| BRTHA2020566 | 0.003716229 | 164957 | 2 |
| BRTHA2020642 | 0.001858114 | 128505 | 1 |
| BRTHA2020695 | 0.001858114 | 150695 | 1 |
| BRTHA2020721 | 0.001858114 | 270933 | 1 |
| BRTHA2020781 | 0.001858114 | 147171 | 1 |
| BRTHA2020811 | 0.001858114 | 280153 | 1 |
| BRTHA2020910 | 0.001858114 | 105888 | 1 |
| BRTHA2021212 | 0.001858114 | 196532 | 1 |
| BRTHA2021440 | 0.00325527 | 254163 | 2 |
| BRTHA2021450 | 0.001858114 | 201953 | 1 |
| BRTHA2022074 | 0.001858114 | 150658 | 1 |
| BRTHA2022914 | 0.001858114 | 130738 | 1 |
| BRTHA2022968 | 0.001858114 | 33602 | 1 |
| BRTHA2023402 | 0.001858114 | 136956 | 1 |
| BRTHA2023437 | 0.001858114 | 140780 | 1 |
| BRTHA2024177 | 0.001858114 | 130641 | 1 |
| BRTHA2024354 | 0.001858114 | 126649 | 1 |
| BRTHA2024712 | 0.001858114 | 124242 | 1 |
| BRTHA2025869 | 0.001858114 | 10842 | 1 |
| BRTHA2026071 | 0.013650469 | 31734 | 6 |
| BRTHA2026290 | 0.001858114 | 269857 | 1 |
| BRTHA2026311 | 0.001858114 | 42711 | 1 |
| BRTHA2027227 | 0.001858114 | 163735 | 1 |
| BRTHA2027229 | 0.183063644 | 135880 | 10 |
| BRTHA2027250 | 0.001858114 | 227498 | 1 |
| BRTHA2027546 | 0.001858114 | 274421 | 1 |
| BRTHA2028297 | 0.001858114 | 155839 | 1 |
| BRTHA2029969 | 0.001858114 | 118071 | 1 |
| BRTHA2030036 | 0.001858114 | 67404 | 1 |
| BRTHA2030213 | 0.001858114 | 192840 | 1 |
| BRTHA2031517 | 0.003567575 | 152024 | 2 |
| BRTHA2031800 | 0.001858114 | 151996 | 1 |
| BRTHA2031917 | 0.001858114 | 148553 | 1 |
| BRTHA2032763 | 0.001858114 | 188359 | 1 |
| BRTHA2032845 | 0.001858114 | 130948 | 1 |
| BRTHA2033122 | 0.001858114 | 119146 | 1 |
| BRTHA2033155 | 0.039396899 | 67281 | 16 |
| BRTHA2033264 | 0.005702198 | 146347 | 2 |
| BRTHA2033320 | 0.001858114 | 122715 | 1 |
| BRTHA2033469 | 0.004772971 | 149299 | 2 |
| BRTHA2033683 | 0.001858114 | 71947 | 1 |
| BRTHA2034281 | 0.001858114 | 165261 | 1 |
| BRTHA2034576 | 0.001858114 | 69618 | 1 |
| BRTHA2034874 | 0.001858114 | 48881 | 1 |
| BRTHA2035448 | 0.001858114 | 129756 | 1 |
| BRTHA2035743 | 0.024691211 | 92447 | 14 |
| BRTHA2036055 | 0.001858114 | 155229 | 1 |
| BRTHA2036295 | 0.001858114 | 255162 | 1 |
| BRTHA2036660 | 0.001858114 | 118512 | 1 |
| BRTHA2037247 | 0.001858114 | 150668 | 1 |
| BRTHA2038279 | 0.001858114 | 129561 | 1 |
| BRTHA2038345 | 0.001858114 | 126099 | 1 |
| BRTHA2038353 | 0.001858114 | 278798 | 1 |
| BRTHA2038396 | 0.001858114 | 169118 | 1 |
| BRTHA3000252 | 0.003716229 | 23295 | 2 |
| BRTHA3000273 | 0.00325527 | 195619 | 2 |
| BRTHA3000296 | 0.001858114 | 164811 | 1 |
| BRTHA3000297 | 0.007135149 | 152696 | 4 |
| BRTHA3000325 | 0.001858114 | 206242 | 1 |
| BRTHA3000456 | 0.002953393 | 78216 | 2 |
| BRTHA3000633 | 0.004902996 | 30816 | 2 |
| BRTHA3000789 | 0.004756819 | 123818 | 3 |
| BRTHA3001221 | 0.001858114 | 167348 | 1 |
| BRTHA3001623 | 0.001858114 | 163468 | 1 |
| BRTHA3001721 | 0.01422502 | 117928 | 8 |
| BRTHA3002265 | 0.001858114 | 246225 | 1 |
| BRTHA3002401 | 0.001858114 | 190010 | 1 |
| BRTHA3002411 | 0.001858114 | 255497 | 1 |
| BRTHA3002427 | 0.058609412 | 82080 | 18 |
| BRTHA3002864 | 0.00325527 | 181358 | 2 |
| BRTHA3002933 | 0.001858114 | 248080 | 1 |
| BRTHA3002955 | 0.001858114 | 210602 | 1 |
| BRTHA3003000 | 0.009770019 | 152207 | 3 |
| BRTHA3003023 | 0.082905216 | 124881 | 13 |
| BRTHA3003074 | 0.014881132 | 14957 | 6 |
| BRTHA3003225 | 0.003939626 | 227824 | 2 |
| BRTHA3003339 | 0.001858114 | 215609 | 1 |
| BRTHA3003343 | 0.001858114 | 256394 | 1 |
| BRTHA3003417 | 0.005444416 | 161511 | 3 |
| BRTHA3003449 | 0.001858114 | 249570 | 1 |
| BRTHA3003474 | 0.001858114 | 276685 | 1 |
| BRTHA3003490 | 0.103213241 | 113322 | 22 |
| BRTHA3003704 | 0.003047359 | 130304 | 2 |
| BRTHA3003736 | 0.07789303 | 98288 | 21 |
| BRTHA3004307 | 0.055621142 | 108282 | 33 |
| BRTHA3004432 | 0.005221938 | 131871 | 3 |
| BRTHA3004475 | 0.003546391 | 224738 | 2 |
| BRTHA3004502 | 0.001858114 | 273993 | 1 |
| BRTHA3004610 | 0.001858114 | 273198 | 1 |
| BRTHA3005046 | 0.003567575 | 173422 | 2 |
| BRTHA3005735 | 0.001858114 | 23404 | 1 |
| BRTHA3005988 | 0.005391776 | 145838 | 3 |
| BRTHA3006318 | 0.001858114 | 179015 | 1 |
| BRTHA3006593 | 0.001858114 | 257082 | 1 |
| BRTHA3006856 | 0.001858114 | 129740 | 1 |
| BRTHA3007113 | 0.001858114 | 262617 | 1 |
| BRTHA3007130 | 0.001858114 | 234521 | 1 |
| BRTHA3007148 | 0.001858114 | 243016 | 1 |
| BRTHA3007319 | 0.001858114 | 239823 | 1 |
| BRTHA3007469 | 0.001858114 | 263316 | 1 |
| BRTHA3007472 | 0.042940532 | 130274 | 12 |
| BRTHA3007532 | 0.001858114 | 89120 | 1 |
| BRTHA3007662 | 0.005277035 | 176725 | 3 |
| BRTHA3007685 | 0.003047359 | 237059 | 2 |
| BRTHA3007769 | 0.001858114 | 270404 | 1 |
| BRTHA3007922 | 0.010736471 | 253856 | 4 |
| BRTHA3008143 | 0.001858114 | 249767 | 1 |
| BRTHA3008293 | 0.001858114 | 264217 | 1 |
| BRTHA3008310 | 0.001858114 | 158621 | 1 |
| BRTHA3008386 | 0.001858114 | 8934 | 1 |
| BRTHA3008520 | 0.003533661 | 239799 | 2 |
| BRTHA3008608 | 0.001858114 | 165175 | 1 |
| BRTHA3008694 | 0.001858114 | 205546 | 1 |
| BRTHA3008778 | 0.006744365 | 84754 | 4 |
| BRTHA3008826 | 0.001858114 | 29110 | 1 |
| BRTHA3009037 | 0.001858114 | 145094 | 1 |
| BRTHA3009090 | 0.014235922 | 111161 | 6 |
| BRTHA3009291 | 0.001858114 | 271541 | 1 |
| BRTHA3009858 | 0.007224294 | 107617 | 3 |
| BRTHA3010135 | 0.008373735 | 65474 | 4 |
| BRTHA3010212 | 0.030329007 | 18697 | 10 |
| BRTHA3010366 | 0.001858114 | 218438 | 1 |
| BRTHA3010469 | 0.003716229 | 175354 | 2 |
| BRTHA3010530 | 0.011984525 | 115899 | 7 |
| BRTHA3010540 | 0.049777951 | 123221 | 3 |
| BRTHA3010717 | 0.001858114 | 71531 | 1 |
| BRTHA3011149 | 0.001858114 | 138502 | 1 |
| BRTHA3011187 | 0.001858114 | 141701 | 1 |
| BRTHA3011194 | 0.047405969 | 38798 | 15 |
| BRTHA3011229 | 0.001858114 | 124896 | 1 |
| BRTHA3011265 | 0.019638355 | 87098 | 9 |
| BRTHA3011306 | 0.013605416 | 187293 | 3 |
| BRTHA3011361 | 0.002953393 | 109699 | 2 |
| BRTHA3011510 | 0.001858114 | 276094 | 1 |
| BRTHA3011892 | 0.001858114 | 188733 | 1 |
| BRTHA3011998 | 0.004236603 | 188789 | 3 |
| BRTHA3012265 | 0.005277035 | 188376 | 3 |
| BRTHA3013860 | 0.001858114 | 256911 | 1 |
| BRTHA3013882 | 0.001858114 | 281554 | 1 |
| BRTHA3013884 | 0.001858114 | 252473 | 1 |
| BRTHA3013981 | 0.001858114 | 258272 | 1 |
| BRTHA3014000 | 0.001858114 | 242794 | 1 |
| BRTHA3014105 | 0.001858114 | 280016 | 1 |
| BRTHA3014507 | 0.001858114 | 128803 | 1 |
| BRTHA3014547 | 0.001858114 | 145850 | 1 |
| BRTHA3014691 | 0.001858114 | 186262 | 1 |
| BRTHA3014835 | 0.001858114 | 203108 | 1 |
| BRTHA3014854 | 0.001858114 | 226205 | 1 |
| BRTHA3014908 | 0.001858114 | 80719 | 1 |
| BRTHA3014920 | 0.001858114 | 79620 | 1 |
| BRTHA3015815 | 0.001858114 | 195542 | 1 |
| BRTHA3015910 | 0.011230867 | 93166 | 4 |
| BRTHA3016616 | 0.001858114 | 240126 | 1 |
| BRTHA3016835 | 0.001858114 | 231788 | 1 |
| BRTHA3016845 | 0.001858114 | 240138 | 1 |
| BRTHA3016917 | 0.001858114 | 84707 | 1 |
| BRTHA3017047 | 0.001858114 | 224085 | 1 |
| BRTHA3017589 | 0.001858114 | 60549 | 1 |
| BRTHA3017791 | 0.141739371 | 91975 | 53 |
| BRTHA3017848 | 0.003533661 | 148953 | 2 |
| BRTHA3018409 | 0.001858114 | 97049 | 1 |
| BRTHA3018514 | 0.001858114 | 84924 | 1 |
| BRTHA3018617 | 0.001858114 | 281297 | 1 |
| BRTHA3018623 | 0.001858114 | 228742 | 1 |
| BRTHA3018656 | 0.001858114 | 154137 | 1 |
| BRTHA3019048 | 0.001858114 | 207399 | 1 |
| BRTHA3019105 | 0.001858114 | 222076 | 1 |
| BRTHA3019183 | 0.001858114 | 228721 | 1 |
| BRTHA3020000 | 0.001858114 | 174228 | 1 |
| BRTHA3020314 | 0.016455223 | 108610 | 9 |
| BRTHA3020369 | 0.001858114 | 238482 | 1 |
| BRTHA3020771 | 0.005243122 | 139192 | 3 |
| BRTHA3021569 | 0.001858114 | 226080 | 1 |
| BRTHA3021708 | 0.003047359 | 259965 | 2 |
| BRTHA3021786 | 0.001858114 | 247601 | 1 |
| BRTHA3021971 | 0.086324188 | 118853 | 12 |
| BRTHA3022641 | 0.009471572 | 132297 | 4 |
| BRTHA3023403 | 0.022556145 | 103660 | 12 |
| BRTHA3023523 | 0.001858114 | 161537 | 1 |
| BRTHA3023590 | 0.001858114 | 236396 | 1 |
| BRTHA3023929 | 0.001858114 | 216919 | 1 |
| BRTHA3024233 | 0.001858114 | 167329 | 1 |
| BRTHA3024600 | 0.001858114 | 216463 | 1 |
| BRTHA3025073 | 0.001858114 | 258890 | 1 |
| BRTHA3025789 | 0.001858114 | 221043 | 1 |
| BRTHA3025826 | 0.001858114 | 240471 | 1 |
| BRTHA3026161 | 0.001858114 | 262193 | 1 |
| BRTHA3026180 | 0.001858114 | 222750 | 1 |
| BRTHA3026507 | 0.047956841 | 76565 | 24 |
| BRTHA3026556 | 0.001858114 | 234235 | 1 |
| BRTHA3026916 | 0.003546391 | 104037 | 2 |
| BRTHA3027171 | 0.001858114 | 263889 | 1 |
| BRTHA3027318 | 0.001858114 | 197580 | 1 |
| BRTHA3027638 | 0.001858114 | 234184 | 1 |
| BRTHA3027820 | 0.001858114 | 258849 | 1 |
| BRTHA3027879 | 0.003856516 | 19874 | 2 |
| BRTHA3027957 | 0.001858114 | 218841 | 1 |
| BRTHA3028339 | 0.001858114 | 77129 | 1 |
| BRTHA3028505 | 0.001858114 | 60728 | 1 |
| BRTHA3028782 | 0.001858114 | 253394 | 1 |
| CD34C2000051 | 0.070372977 | 2976 | 1 |
| CD34C2000152 | 0.070372977 | 147255 | 1 |
| CD34C2000175 | 0.070372977 | 113756 | 1 |
| CD34C2000443 | 0.070372977 | 53395 | 1 |
| CD34C3000125 | 0.072082437 | 158107 | 2 |
| CD34C3000252 | 0.204145114 | 118638 | 22 |
| CD34C3000314 | 0.070372977 | 154623 | 1 |
| CD34C3000411 | 0.219156642 | 71428 | 31 |
| CD34C3000424 | 0.073177715 | 77507 | 3 |
| CD34C3000494 | 0.070372977 | 167343 | 1 |
| CERVX1000042 | 0.034626039 | 67344 | 1 |
| CERVX2000812 | 0.034626039 | 276282 | 1 |
| CERVX2000968 | 0.034626039 | 195678 | 1 |
| CERVX2002006 | 0.034626039 | 174407 | 1 |
| CERVX2002013 | 0.034626039 | 169904 | 1 |
| CERVX2002430 | 0.034626039 | 189997 | 1 |
| CERVX2002770 | 0.071082473 | 181132 | 2 |
| CHONS1000058 | 0.037119525 | 260326 | 1 |
| CHONS2000172 | 0.04484944 | 146038 | 3 |
| CHONS2000797 | 0.03851668 | 213245 | 2 |
| CHONS2001287 | 0.037119525 | 12902 | 1 |
| CHONS2001382 | 0.037119525 | 214482 | 1 |
| CHONS2001797 | 0.037119525 | 195567 | 1 |
| CHONS2001834 | 0.037119525 | 100947 | 1 |
| CHONS2002419 | 0.037119525 | 36130 | 1 |
| CHONS2002829 | 0.102559495 | 102447 | 32 |
| COLON1000030 | 0.011763322 | 9531 | 1 |
| COLON1000084 | 0.011763322 | 196656 | 1 |
| COLON1000135 | 0.011763322 | 271740 | 1 |
| COLON2000470 | 0.023523877 | 172195 | 2 |
| COLON2000557 | 0.011763322 | 204534 | 1 |
| COLON2000568 | 0.011763322 | 274367 | 1 |
| COLON2001721 | 0.030286812 | 187376 | 13 |
| COLON2001829 | 0.011763322 | 104654 | 1 |
| COLON2001866 | 0.011763322 | 203953 | 1 |
| COLON2002443 | 0.037157444 | 90793 | 4 |
| COLON2002520 | 0.011763322 | 195730 | 1 |
| COLON2003043 | 0.011763322 | 247917 | 1 |
| COLON2003529 | 0.011763322 | 241903 | 1 |
| COLON2004318 | 0.011763322 | 176203 | 1 |
| COLON2004351 | 0.011763322 | 124325 | 1 |
| COLON2004478 | 0.011763322 | 274317 | 1 |
| COLON2004911 | 0.011763322 | 231909 | 1 |
| COLON2005126 | 0.023526644 | 176286 | 2 |
| COLON2005623 | 0.011763322 | 231849 | 1 |
| COLON2005735 | 0.011763322 | 188221 | 1 |
| COLON2005772 | 0.011763322 | 28843 | 1 |
| COLON2006263 | 0.011763322 | 226701 | 1 |
| COLON2006282 | 0.011763322 | 219407 | 1 |
| COLON2006417 | 0.011763322 | 44351 | 1 |
| COLON2007274 | 0.011763322 | 212155 | 1 |
| COLON2007733 | 0.013438869 | 216968 | 2 |
| COLON2008105 | 0.011763322 | 178748 | 1 |
| COLON2009337 | 0.023526644 | 176434 | 2 |
| COLON2009499 | 0.011763322 | 208506 | 1 |
| CORDB1000140 | 0.140646976 | 215444 | 1 |
| CORDB2000061 | 0.140646976 | 246593 | 1 |
| CORDB2000127 | 0.140646976 | 249465 | 1 |
| CORDB2000541 | 0.140646976 | 249151 | 1 |
| CTONG1000009 | 0.034496405 | 62385 | 13 |
| CTONG1000010 | 0.025844691 | 77899 | 7 |
| CTONG1000020 | 0.003120806 | 77966 | 1 |
| CTONG1000022 | 0.003120806 | 72470 | 1 |
| CTONG1000040 | 0.003120806 | 7962 | 1 |
| CTONG1000052 | 0.277307012 | 16876 | 89 |
| CTONG1000056 | 0.003120806 | 43501 | 1 |
| CTONG1000062 | 0.003120806 | 59249 | 1 |
| CTONG1000086 | 0.003120806 | 62390 | 1 |
| CTONG1000087 | 0.160228428 | 21020 | 44 |
| CTONG1000088 | 0.057351275 | 79448 | 3 |
| CTONG1000093 | 0.004216085 | 70893 | 2 |
| CTONG1000094 | 0.430230536 | 55221 | 85 |
| CTONG1000097 | 0.003120806 | 141332 | 1 |
| CTONG1000113 | 0.014884128 | 77814 | 2 |
| CTONG1000137 | 0.005119208 | 74841 | 2 |
| CTONG1000165 | 0.003120806 | 6634 | 1 |
| CTONG1000175 | 0.099518905 | 67270 | 21 |
| CTONG100018 | 0.006977322 | 40124 | 3 |
| CTONG1000181 | 0.003120806 | 22626 | 1 |
| CTONG1000218 | 0.347974452 | 40191 | 42 |
| CTONG1000220 | 0.012575283 | 62125 | 2 |
| CTONG1000241 | 0.078984935 | 69883 | 31 |
| CTONG1000277 | 0.008101167 | 71091 | 3 |
| CTONG1000283 | 0.003120806 | 14595 | 1 |
| CTONG1000288 | 0.110164387 | 63549 | 34 |
| CTONG1000302 | 0.003120806 | 180451 | 1 |
| CTONG1000341 | 0.006035663 | 94998 | 2 |
| CTONG1000467 | 0.019500085 | 169148 | 7 |
| CTONG1000476 | 0.003120806 | 167184 | 1 |
| CTONG1000488 | 0.003120806 | 271982 | 1 |
| CTONG1000508 | 0.003120806 | 186524 | 1 |
| CTONG1000540 | 0.003120806 | 175577 | 1 |
| CTONG2000031 | 0.004796353 | 86537 | 2 |
| CTONG2000034 | 0.018590341 | 162325 | 3 |
| CTONG2000042 | 0.04782732 | 121139 | 16 |
| CTONG2000063 | 0.078268289 | 105397 | 28 |
| CTONG2000201 | 0.086441422 | 112247 | 21 |
| CTONG2000218 | 0.003120806 | 255618 | 1 |
| CTONG2000279 | 0.003120806 | 278930 | 1 |
| CTONG2000303 | 0.006241613 | 197275 | 2 |
| CTONG2000330 | 0.003120806 | 90705 | 1 |
| CTONG2000395 | 0.003120806 | 265176 | 1 |
| CTONG2000411 | 0.029115702 | 150428 | 11 |
| CTONG2000452 | 0.057530151 | 35838 | 10 |
| CTONG2000465 | 0.004517962 | 35886 | 2 |
| CTONG2000469 | 0.05737883 | 103841 | 12 |
| CTONG2000480 | 0.003120806 | 263293 | 1 |
| CTONG2000508 | 0.212174642 | 84844 | 73 |
| CTONG2000561 | 0.046175455 | 124179 | 15 |
| CTONG2000589 | 0.015026848 | 22042 | 4 |
| CTONG2000766 | 0.003120806 | 106143 | 1 |
| CTONG2000771 | 0.003120806 | 267049 | 1 |
| CTONG2000819 | 0.003120806 | 167101 | 1 |
| CTONG2000827 | 0.004978921 | 92558 | 2 |
| CTONG2000846 | 0.003120806 | 85683 | 1 |
| CTONG2000855 | 0.003120806 | 112142 | 1 |
| CTONG2000977 | 0.003120806 | 13589 | 1 |
| CTONG2001139 | 0.006216302 | 181502 | 2 |
| CTONG2001226 | 0.022074238 | 13354 | 4 |
| CTONG2001320 | 0.03178173 | 130660 | 5 |
| CTONG2001366 | 0.021616218 | 126438 | 6 |
| CTONG2001413 | 0.003120806 | 124385 | 1 |
| CTONG2001428 | 0.004997648 | 240236 | 2 |
| CTONG2001513 | 0.006102766 | 39666 | 2 |
| CTONG2001524 | 0.003120806 | 258587 | 1 |
| CTONG2001533 | 0.004830267 | 204421 | 2 |
| CTONG2001598 | 0.004310051 | 168941 | 2 |
| CTONG2001681 | 0.003120806 | 33244 | 1 |
| CTONG2001724 | 0.003120806 | 183725 | 1 |
| CTONG2001748 | 0.080158054 | 3135 | 26 |
| CTONG2001749 | 0.003120806 | 108180 | 1 |
| CTONG2001800 | 0.005925545 | 124246 | 3 |
| CTONG2001820 | 0.006165688 | 109341 | 2 |
| CTONG2001858 | 0.022703207 | 3778 | 5 |
| CTONG2001877 | 0.003120806 | 281661 | 1 |
| CTONG2001911 | 0.011088714 | 117968 | 4 |
| CTONG2001932 | 0.016218342 | 62274 | 6 |
| CTONG2001955 | 0.003120806 | 47686 | 1 |
| CTONG2002066 | 0.014884128 | 155537 | 2 |
| CTONG2002073 | 0.114392808 | 119881 | 20 |
| CTONG2002095 | 0.023924858 | 76669 | 9 |
| CTONG2002143 | 0.004216085 | 106850 | 2 |
| CTONG2002270 | 0.315884954 | 80131 | 174 |
| CTONG2002395 | 0.004517962 | 133635 | 2 |
| CTONG2002417 | 0.003120806 | 187183 | 1 |
| CTONG2002418 | 0.003120806 | 63629 | 1 |
| CTONG2002453 | 0.021583041 | 179222 | 4 |
| CTONG2002520 | 0.056918342 | 53994 | 7 |
| CTONG2002558 | 0.006035663 | 99482 | 2 |
| CTONG2002590 | 0.003120806 | 152778 | 1 |
| CTONG2002709 | 0.077331872 | 77951 | 6 |
| CTONG2002721 | 0.004517962 | 189962 | 2 |
| CTONG2002744 | 0.284516459 | 79890 | 72 |
| CTONG2002766 | 0.014181268 | 69565 | 9 |
| CTONG2002803 | 0.113796531 | 77877 | 37 |
| CTONG2002816 | 0.003120806 | 119481 | 1 |
| CTONG2002820 | 0.014897288 | 103972 | 6 |
| CTONG2002832 | 0.014946393 | 179335 | 7 |
| CTONG2002841 | 0.015818152 | 126198 | 9 |
| CTONG2002903 | 0.054395942 | 138939 | 13 |
| CTONG2002965 | 0.018724838 | 149389 | 6 |
| CTONG2002999 | 0.003120806 | 206341 | 1 |
| CTONG2003028 | 0.003120806 | 95144 | 1 |
| CTONG2003094 | 0.09145026 | 126067 | 29 |
| CTONG2003100 | 0.039104973 | 187360 | 2 |
| CTONG2003115 | 0.003120806 | 199174 | 1 |
| CTONG2003189 | 0.004216085 | 162326 | 2 |
| CTONG2003245 | 0.003120806 | 204581 | 1 |
| CTONG2003293 | 0.018126706 | 108714 | 6 |
| CTONG2003298 | 0.230892881 | 121073 | 48 |
| CTONG2003348 | 0.005119208 | 137769 | 2 |
| CTONG2003350 | 0.003120806 | 141148 | 1 |
| CTONG2003361 | 0.007130942 | 87892 | 3 |
| CTONG2003372 | 0.003120806 | 181168 | 1 |
| CTONG2003375 | 0.003120806 | 101229 | 1 |
| CTONG2003427 | 0.003120806 | 157347 | 1 |
| CTONG2003517 | 0.014946209 | 155548 | 4 |
| CTONG2003524 | 0.103986045 | 75516 | 28 |
| CTONG2003599 | 0.003120806 | 412 | 1 |
| CTONG2003680 | 0.016182457 | 83753 | 2 |
| CTONG2003699 | 0.004809083 | 110024 | 2 |
| CTONG2003764 | 0.012829544 | 152578 | 2 |
| CTONG2003782 | 0.003120806 | 115291 | 1 |
| CTONG2003889 | 0.003120806 | 160486 | 1 |
| CTONG2003937 | 0.008962269 | 179141 | 2 |
| CTONG2003945 | 0.015680031 | 41700 | 3 |
| CTONG2004000 | 0.003120806 | 87224 | 1 |
| CTONG2004062 | 0.068657741 | 140112 | 22 |
| CTONG2004115 | 0.014018789 | 138170 | 3 |
| CTONG2004126 | 0.003120806 | 61376 | 1 |
| CTONG2004264 | 0.003120806 | 112602 | 1 |
| CTONG2004305 | 0.004796353 | 183331 | 2 |
| CTONG2004397 | 0.006035663 | 126844 | 2 |
| CTONG2004423 | 0.01458127 | 38348 | 7 |
| CTONG2004454 | 0.003120806 | 115355 | 1 |
| CTONG2004487 | 0.177140902 | 118959 | 79 |
| CTONG2004550 | 0.008962269 | 122165 | 2 |
| CTONG2004669 | 0.003120806 | 144841 | 1 |
| CTONG2004716 | 0.003120806 | 18409 | 1 |
| CTONG2004912 | 0.003120806 | 253234 | 1 |
| CTONG2004941 | 0.003120806 | 106992 | 1 |
| CTONG2004948 | 0.003120806 | 93041 | 1 |
| CTONG2005028 | 0.024477841 | 67324 | 10 |
| CTONG2005049 | 0.006241613 | 155344 | 2 |
| CTONG2005110 | 0.003120806 | 50728 | 1 |
| CTONG2005145 | 0.006828668 | 96127 | 3 |
| CTONG2005265 | 0.008962269 | 167518 | 2 |
| CTONG2005278 | 0.058139526 | 54224 | 17 |
| CTONG2005290 | 0.007297741 | 30737 | 2 |
| CTONG2005374 | 0.003120806 | 167274 | 1 |
| CTONG2005399 | 0.003120806 | 76298 | 1 |
| CTONG2005468 | 0.011239261 | 141850 | 4 |
| CTONG2005553 | 0.23397385 | 86681 | 40 |
| CTONG2005567 | 0.003120806 | 222692 | 1 |
| CTONG2005585 | 0.059810149 | 32632 | 2 |
| CTONG2005615 | 0.035237193 | 99667 | 19 |
| CTONG2005635 | 0.003120806 | 103368 | 1 |
| CTONG2005775 | 0.003120806 | 213226 | 1 |
| CTONG2005795 | 0.004830267 | 125171 | 2 |
| CTONG2005890 | 0.003120806 | 251063 | 1 |
| CTONG2005904 | 0.003120806 | 246498 | 1 |
| CTONG2005913 | 0.037549133 | 138354 | 9 |
| CTONG2006004 | 0.158747049 | 60248 | 22 |
| CTONG2006129 | 0.003120806 | 237474 | 1 |
| CTONG2006223 | 0.003120806 | 257390 | 1 |
| CTONG2006235 | 0.10228708 | 119632 | 35 |
| CTONG2006273 | 0.003120806 | 142600 | 1 |
| CTONG2006310 | 0.003120806 | 155452 | 1 |
| CTONG2006377 | 0.014272056 | 131941 | 9 |
| CTONG2006393 | 0.004796353 | 82547 | 2 |
| CTONG2006412 | 0.026992367 | 145607 | 10 |
| CTONG2006449 | 0.019060663 | 57104 | 5 |
| CTONG2006515 | 0.003120806 | 185387 | 1 |
| CTONG2006524 | 0.003120806 | 160618 | 1 |
| CTONG2006562 | 0.119676012 | 115921 | 29 |
| CTONG2006568 | 0.003120806 | 1118 | 1 |
| CTONG2006611 | 0.023203382 | 114381 | 6 |
| CTONG2006620 | 0.003120806 | 141233 | 1 |
| CTONG2006666 | 0.006394803 | 148234 | 3 |
| CTONG2006691 | 0.003120806 | 94651 | 1 |
| CTONG2006709 | 0.064792528 | 66027 | 25 |
| CTONG2006798 | 0.017364674 | 116923 | 5 |
| CTONG2006836 | 0.003120806 | 112541 | 1 |
| CTONG2006932 | 0.152263897 | 113947 | 16 |
| CTONG2006942 | 0.012483226 | 116396 | 4 |
| CTONG2006964 | 0.003120806 | 199107 | 1 |
| CTONG2007007 | 0.179986723 | 3690 | 50 |
| CTONG2007009 | 0.003120806 | 152526 | 1 |
| CTONG2007072 | 0.077886267 | 17742 | 18 |
| CTONG2007078 | 0.131115217 | 119962 | 31 |
| CTONG2007091 | 0.003120806 | 92658 | 1 |
| CTONG2007104 | 0.026939526 | 114315 | 9 |
| CTONG2007168 | 0.027717208 | 30085 | 16 |
| CTONG2007175 | 0.003120806 | 35654 | 1 |
| CTONG2007259 | 0.003120806 | 241536 | 1 |
| CTONG2007272 | 0.003120806 | 96165 | 1 |
| CTONG2007293 | 0.004978921 | 148996 | 2 |
| CTONG2007297 | 0.003120806 | 241542 | 1 |
| CTONG2007399 | 0.010272745 | 150737 | 7 |
| CTONG2007400 | 0.014561073 | 152008 | 6 |
| CTONG2007417 | 0.007773232 | 142891 | 4 |
| CTONG2007441 | 0.008118454 | 137131 | 3 |
| CTONG2007474 | 0.015773997 | 75185 | 3 |
| CTONG2007500 | 0.222801919 | 93241 | 51 |
| CTONG2007586 | 0.027878218 | 146137 | 8 |
| CTONG2007613 | 0.019142568 | 106401 | 5 |
| CTONG2007623 | 0.003120806 | 174951 | 1 |
| CTONG2007681 | 0.00906687 | 72577 | 5 |
| CTONG2007776 | 0.039395015 | 121642 | 19 |
| CTONG2007779 | 0.004978921 | 160578 | 2 |
| CTONG2007834 | 0.177140902 | 118959 | 79 |
| CTONG2007898 | 0.003120806 | 187129 | 1 |
| CTONG2007959 | 0.00921057 | 117951 | 3 |
| CTONG2007970 | 0.003120806 | 67829 | 1 |
| CTONG2008014 | 0.011066625 | 155226 | 3 |
| CTONG2008184 | 0.003120806 | 143548 | 1 |
| CTONG2008233 | 0.243998186 | 121013 | 62 |
| CTONG2008262 | 0.003120806 | 6595 | 1 |
| CTONG2008269 | 0.024627562 | 274521 | 3 |
| CTONG2008321 | 0.011138093 | 189416 | 3 |
| CTONG2008343 | 0.005119208 | 87922 | 2 |
| CTONG2008398 | 0.219156642 | 71428 | 31 |
| CTONG2008402 | 0.003120806 | 134022 | 1 |
| CTONG2008405 | 0.007613821 | 164132 | 4 |
| CTONG2008466 | 0.021431248 | 125662 | 5 |
| CTONG2008506 | 0.178058455 | 124982 | 51 |
| CTONG2008518 | 0.043505258 | 80331 | 15 |
| CTONG2008521 | 0.011021259 | 67399 | 4 |
| CTONG2008562 | 0.003120806 | 122276 | 1 |
| CTONG2008577 | 0.003120806 | 132456 | 1 |
| CTONG2008595 | 0.003120806 | 62016 | 1 |
| CTONG2008621 | 0.004997648 | 122242 | 2 |
| CTONG2008689 | 0.008017912 | 171541 | 4 |
| CTONG2008720 | 0.006206238 | 104094 | 3 |
| CTONG2008721 | 0.004978921 | 174128 | 2 |
| CTONG2008773 | 0.003120806 | 190009 | 1 |
| CTONG2008792 | 0.02280339 | 59837 | 6 |
| CTONG2008989 | 0.003120806 | 189099 | 1 |
| CTONG2009033 | 0.006505814 | 140048 | 3 |
| CTONG2009108 | 0.007060433 | 109378 | 3 |
| CTONG2009132 | 0.003120806 | 82136 | 1 |
| CTONG2009156 | 0.006241613 | 155851 | 2 |
| CTONG2009181 | 0.006241613 | 63192 | 2 |
| CTONG2009257 | 0.003120806 | 39686 | 1 |
| CTONG2009258 | 0.003120806 | 113070 | 1 |
| CTONG2009268 | 0.003120806 | 121502 | 1 |
| CTONG2009270 | 0.003120806 | 99946 | 1 |
| CTONG2009395 | 0.003120806 | 102049 | 1 |
| CTONG2009423 | 0.004830267 | 167720 | 2 |
| CTONG2009440 | 0.003120806 | 266855 | 1 |
| CTONG2009527 | 0.003120806 | 104916 | 1 |
| CTONG2009529 | 0.02649962 | 45436 | 5 |
| CTONG2009531 | 0.003120806 | 100842 | 1 |
| CTONG2009534 | 0.021788234 | 92756 | 10 |
| CTONG2009570 | 0.003120806 | 60631 | 1 |
| CTONG2009572 | 0.006241613 | 20586 | 2 |
| CTONG2009604 | 0.003120806 | 82835 | 1 |
| CTONG2009643 | 0.003120806 | 66463 | 1 |
| CTONG2009675 | 0.003120806 | 112952 | 1 |
| CTONG2009689 | 0.003120806 | 237559 | 1 |
| CTONG2009725 | 0.003120806 | 231913 | 1 |
| CTONG2009766 | 0.003120806 | 217517 | 1 |
| CTONG2009768 | 0.003120806 | 265018 | 1 |
| CTONG2009844 | 0.009323126 | 198981 | 2 |
| CTONG2009871 | 0.003120806 | 255787 | 1 |
| CTONG2009887 | 0.003120806 | 235099 | 1 |
| CTONG2009891 | 0.003120806 | 113214 | 1 |
| CTONG2009923 | 0.005119208 | 143566 | 2 |
| CTONG2009938 | 0.008374477 | 107882 | 4 |
| CTONG2009955 | 0.003120806 | 6572 | 1 |
| CTONG2009958 | 0.003120806 | 83771 | 1 |
| CTONG2009963 | 0.008105674 | 95735 | 5 |
| CTONG2010018 | 0.003120806 | 160358 | 1 |
| CTONG2010024 | 0.006241613 | 112185 | 2 |
| CTONG2010116 | 0.003120806 | 175358 | 1 |
| CTONG2010133 | 0.003120806 | 47501 | 1 |
| CTONG2010148 | 0.003120806 | 147658 | 1 |
| CTONG2010276 | 0.003120806 | 144021 | 1 |
| CTONG2010330 | 0.003120806 | 178863 | 1 |
| CTONG2010348 | 0.094622909 | 106856 | 32 |
| CTONG2010408 | 0.019555713 | 10440 | 9 |
| CTONG2010508 | 0.040867652 | 142924 | 3 |
| CTONG2010566 | 0.01235668 | 89826 | 4 |
| CTONG2010623 | 0.003120806 | 200489 | 1 |
| CTONG2010649 | 0.107152979 | 147960 | 24 |
| CTONG2010652 | 0.010424747 | 60713 | 6 |
| CTONG2010803 | 0.014881362 | 67891 | 2 |
| CTONG2010821 | 0.234479482 | 47615 | 51 |
| CTONG2011188 | 0.007951073 | 124623 | 3 |
| CTONG2011429 | 0.003120806 | 72408 | 1 |
| CTONG2011474 | 0.003120806 | 217588 | 1 |
| CTONG2011676 | 0.003120806 | 274344 | 1 |
| CTONG2011716 | 0.006035663 | 35776 | 2 |
| CTONG2011765 | 0.003120806 | 273347 | 1 |
| CTONG2011779 | 0.003120806 | 120967 | 1 |
| CTONG2011801 | 0.056586757 | 105981 | 15 |
| CTONG2011815 | 0.003120806 | 74005 | 1 |
| CTONG2011822 | 0.003120806 | 145602 | 1 |
| CTONG2011825 | 0.019516612 | 14977 | 6 |
| CTONG2011920 | 0.003120806 | 79613 | 1 |
| CTONG2011985 | 0.003120806 | 150354 | 1 |
| CTONG2012001 | 0.023432238 | 139309 | 10 |
| CTONG2012029 | 0.008962269 | 196248 | 2 |
| CTONG2012050 | 0.023587306 | 121537 | 9 |
| CTONG2012077 | 0.003120806 | 21210 | 1 |
| CTONG2012101 | 0.010613531 | 176766 | 5 |
| CTONG2012123 | 0.003120806 | 153183 | 1 |
| CTONG2012158 | 0.018366985 | 83115 | 4 |
| CTONG2012230 | 0.003120806 | 164971 | 1 |
| CTONG2012401 | 0.039831185 | 150153 | 8 |
| CTONG2012422 | 0.004517962 | 80146 | 2 |
| CTONG2012425 | 0.006035663 | 176359 | 2 |
| CTONG2012447 | 0.006241613 | 100680 | 2 |
| CTONG2012452 | 0.470539281 | 86556 | 72 |
| CTONG2012453 | 0.022248538 | 114970 | 7 |
| CTONG2012473 | 0.003120806 | 140865 | 1 |
| CTONG2012476 | 0.003120806 | 93696 | 1 |
| CTONG2012533 | 0.003120806 | 266275 | 1 |
| CTONG2012554 | 0.003120806 | 176800 | 1 |
| CTONG2012564 | 0.042410704 | 123974 | 3 |
| CTONG2012607 | 0.096882531 | 121319 | 10 |
| CTONG2012661 | 0.007996629 | 150181 | 4 |
| CTONG2012724 | 0.006241613 | 167247 | 2 |
| CTONG2012738 | 0.003120806 | 153127 | 1 |
| CTONG2012745 | 0.030207963 | 53232 | 6 |
| CTONG2012758 | 0.003120806 | 143773 | 1 |
| CTONG2012843 | 0.018590341 | 113537 | 3 |
| CTONG2012847 | 0.097299276 | 64724 | 18 |
| CTONG2012879 | 0.098543947 | 60159 | 24 |
| CTONG2012901 | 0.003120806 | 53288 | 1 |
| CTONG2012996 | 0.057385388 | 69872 | 19 |
| CTONG2013128 | 0.005119208 | 69307 | 2 |
| CTONG2013149 | 0.012575283 | 144804 | 2 |
| CTONG2013156 | 0.008101167 | 135776 | 3 |
| CTONG2013178 | 0.017705559 | 115940 | 3 |
| CTONG2013222 | 0.044586909 | 123152 | 3 |
| CTONG2013284 | 0.012483226 | 18407 | 4 |
| CTONG2013339 | 0.003120806 | 126572 | 1 |
| CTONG2013348 | 0.03045737 | 21056 | 11 |
| CTONG2013352 | 0.006165688 | 141074 | 2 |
| CTONG2013381 | 0.053410589 | 132949 | 16 |
| CTONG2013393 | 0.003120806 | 213337 | 1 |
| CTONG2013511 | 0.003120806 | 164929 | 1 |
| CTONG2013630 | 0.003120806 | 144965 | 1 |
| CTONG2013803 | 0.003120806 | 186489 | 1 |
| CTONG2013907 | 0.077719633 | 126397 | 20 |
| CTONG2013934 | 0.024156494 | 136205 | 15 |
| CTONG2013985 | 0.02861187 | 105398 | 5 |
| CTONG2013986 | 0.003120806 | 127193 | 1 |
| CTONG2014032 | 0.003120806 | 60741 | 1 |
| CTONG2014058 | 0.003120806 | 136818 | 1 |
| CTONG2014119 | 0.020674126 | 31757 | 12 |
| CTONG2014165 | 0.034799554 | 150747 | 6 |
| CTONG2014191 | 0.003120806 | 160438 | 1 |
| CTONG2014206 | 0.003120806 | 14385 | 1 |
| CTONG2014234 | 0.003120806 | 52670 | 1 |
| CTONG2014264 | 0.003120806 | 92254 | 1 |
| CTONG2014280 | 0.013329953 | 74214 | 6 |
| CTONG2014369 | 0.040262756 | 149684 | 10 |
| CTONG2014389 | 0.06343209 | 135781 | 8 |
| CTONG2014491 | 0.004997648 | 81374 | 2 |
| CTONG2014630 | 0.003120806 | 254496 | 1 |
| CTONG2014635 | 0.003120806 | 230514 | 1 |
| CTONG2014646 | 0.003120806 | 258279 | 1 |
| CTONG2014697 | 0.10290126 | 128010 | 24 |
| CTONG2014705 | 0.008925985 | 244218 | 2 |
| CTONG2014898 | 0.029049444 | 146811 | 13 |
| CTONG2014946 | 0.003120806 | 163825 | 1 |
| CTONG2014954 | 0.01370457 | 142266 | 5 |
| CTONG2014959 | 0.003120806 | 188300 | 1 |
| CTONG2014966 | 0.003120806 | 145508 | 1 |
| CTONG2014995 | 0.003120806 | 206100 | 1 |
| CTONG2015091 | 0.206327196 | 56768 | 24 |
| CTONG2015204 | 0.011046569 | 165000 | 4 |
| CTONG2015316 | 0.003120806 | 133962 | 1 |
| CTONG2015330 | 0.005891632 | 22110 | 3 |
| CTONG2015345 | 0.019627211 | 31512 | 7 |
| CTONG2015358 | 0.004216085 | 132408 | 2 |
| CTONG2015434 | 0.003120806 | 134117 | 1 |
| CTONG2015518 | 0.012617427 | 84426 | 4 |
| CTONG2015540 | 0.003120806 | 153039 | 1 |
| CTONG2015596 | 0.003120806 | 145511 | 1 |
| CTONG2015633 | 0.003120806 | 184709 | 1 |
| CTONG2015678 | 0.004997648 | 176156 | 2 |
| CTONG2015717 | 0.003120806 | 230523 | 1 |
| CTONG2015804 | 0.003120806 | 131923 | 1 |
| CTONG2015815 | 0.019047249 | 236266 | 4 |
| CTONG2015866 | 0.003120806 | 110172 | 1 |
| CTONG2015914 | 0.006241613 | 171694 | 2 |
| CTONG2015953 | 0.003120806 | 173186 | 1 |
| CTONG2016056 | 0.084263661 | 50258 | 9 |
| CTONG2016185 | 0.064452745 | 112993 | 20 |
| CTONG2016217 | 0.003120806 | 123191 | 1 |
| CTONG2016355 | 0.003120806 | 159411 | 1 |
| CTONG2016408 | 0.04525627 | 48099 | 11 |
| CTONG2016499 | 0.004796353 | 149894 | 2 |
| CTONG2016505 | 0.009323126 | 183221 | 2 |
| CTONG2016559 | 0.003120806 | 181025 | 1 |
| CTONG2016575 | 0.337008866 | 34785 | 103 |
| CTONG2016658 | 0.003120806 | 132148 | 1 |
| CTONG2016775 | 0.003120806 | 176517 | 1 |
| CTONG2016797 | 0.003120806 | 206052 | 1 |
| CTONG2016824 | 0.039364238 | 79287 | 7 |
| CTONG2016846 | 0.003120806 | 145385 | 1 |
| CTONG2016869 | 0.063438942 | 17960 | 33 |
| CTONG2016904 | 0.003120806 | 116043 | 1 |
| CTONG2016931 | 0.05490659 | 88544 | 16 |
| CTONG2016942 | 0.014584752 | 155457 | 2 |
| CTONG2016953 | 0.003120806 | 243892 | 1 |
| CTONG2017021 | 0.003120806 | 188857 | 1 |
| CTONG2017094 | 0.074946426 | 130588 | 33 |
| CTONG2017292 | 0.003120806 | 175116 | 1 |
| CTONG2017409 | 0.004978921 | 112030 | 2 |
| CTONG2017429 | 0.023217322 | 123615 | 9 |
| CTONG2017444 | 0.045500795 | 132817 | 8 |
| CTONG2017458 | 0.003120806 | 162142 | 1 |
| CTONG2017500 | 0.06325171 | 153121 | 16 |
| CTONG2017604 | 0.013948004 | 185642 | 2 |
| CTONG2017650 | 0.003120806 | 164997 | 1 |
| CTONG2017798 | 0.008407551 | 47881 | 5 |
| CTONG2017804 | 0.009362419 | 137779 | 3 |
| CTONG2017814 | 0.003120806 | 133713 | 1 |
| CTONG2017910 | 0.003120806 | 144540 | 1 |
| CTONG2017939 | 0.003120806 | 171580 | 1 |
| CTONG2017989 | 0.003120806 | 8532 | 1 |
| CTONG2017998 | 0.066885703 | 65289 | 22 |
| CTONG2018062 | 0.003120806 | 204619 | 1 |
| CTONG2018069 | 0.003120806 | 230565 | 1 |
| CTONG2018135 | 0.010772981 | 117859 | 3 |
| CTONG2018147 | 0.003120806 | 199576 | 1 |
| CTONG2018211 | 0.049128914 | 143252 | 23 |
| CTONG2018217 | 0.003120806 | 178742 | 1 |
| CTONG2018279 | 0.003120806 | 198774 | 1 |
| CTONG2018334 | 0.022906496 | 175097 | 4 |
| CTONG2018343 | 0.011000075 | 27839 | 4 |
| CTONG2018379 | 0.003120806 | 149546 | 1 |
| CTONG2018383 | 0.037066396 | 140531 | 8 |
| CTONG2018413 | 0.006654468 | 445 | 3 |
| CTONG2018483 | 0.003120806 | 145199 | 1 |
| CTONG2018614 | 0.107152979 | 147960 | 24 |
| CTONG2018629 | 0.008118454 | 61273 | 3 |
| CTONG2018632 | 0.020949569 | 146791 | 6 |
| CTONG2018637 | 0.0255403 | 114676 | 13 |
| CTONG2018652 | 0.004216085 | 134374 | 2 |
| CTONG2018653 | 0.003120806 | 154748 | 1 |
| CTONG2018655 | 0.003120806 | 160945 | 1 |
| CTONG2018735 | 0.004517962 | 158753 | 2 |
| CTONG2018739 | 0.043236157 | 82742 | 25 |
| CTONG2018808 | 0.021273983 | 18560 | 5 |
| CTONG2018843 | 0.015092343 | 162030 | 6 |
| CTONG2018898 | 0.347911398 | 85618 | 143 |
| CTONG2018900 | 0.039156842 | 185001 | 2 |
| CTONG2018976 | 0.004517962 | 59482 | 2 |
| CTONG2019029 | 0.02070585 | 86408 | 8 |
| CTONG2019063 | 0.00789185 | 177974 | 3 |
| CTONG2019144 | 0.003120806 | 230543 | 1 |
| CTONG2019226 | 0.126812117 | 121445 | 44 |
| CTONG2019232 | 0.003120806 | 177074 | 1 |
| CTONG2019248 | 0.011075103 | 109409 | 4 |
| CTONG2019400 | 0.008488153 | 99779 | 5 |
| CTONG2019579 | 0.246421275 | 60996 | 32 |
| CTONG2019590 | 0.015571145 | 177140 | 5 |
| CTONG2019652 | 0.003120806 | 275526 | 1 |
| CTONG2019704 | 0.004997648 | 151418 | 2 |
| CTONG2019788 | 0.054798922 | 112077 | 8 |
| CTONG2019822 | 0.051535226 | 140011 | 7 |
| CTONG2019833 | 0.003120806 | 249441 | 1 |
| CTONG2020031 | 0.003120806 | 239538 | 1 |
| CTONG2020108 | 0.004517962 | 108592 | 2 |
| CTONG2020127 | 0.003120806 | 235025 | 1 |
| CTONG2020171 | 0.003120806 | 273578 | 1 |
| CTONG2020374 | 0.010781951 | 130107 | 5 |
| CTONG2020378 | 0.010381057 | 159348 | 5 |
| CTONG2020411 | 0.024337954 | 142074 | 9 |
| CTONG2020445 | 0.00796088 | 193716 | 3 |
| CTONG2020484 | 0.285038793 | 155470 | 28 |
| CTONG2020522 | 0.007311581 | 94151 | 3 |
| CTONG2020560 | 0.111693548 | 94701 | 39 |
| CTONG2020582 | 0.087326661 | 84741 | 38 |
| CTONG2020638 | 0.013533263 | 190625 | 5 |
| CTONG2020806 | 0.003120806 | 176801 | 1 |
| CTONG2020831 | 0.004978921 | 12747 | 2 |
| CTONG2020974 | 0.004216085 | 188736 | 2 |
| CTONG2021024 | 0.004997648 | 16554 | 2 |
| CTONG2021132 | 0.003120806 | 173441 | 1 |
| CTONG2021168 | 0.003120806 | 275834 | 1 |
| CTONG2021289 | 0.016284682 | 32489 | 9 |
| CTONG2022153 | 0.003120806 | 107309 | 1 |
| CTONG2022601 | 0.003120806 | 155433 | 1 |
| CTONG2022835 | 0.097666493 | 113041 | 7 |
| CTONG2023021 | 0.004978921 | 212634 | 2 |
| CTONG2023449 | 0.014584752 | 216175 | 2 |
| CTONG2023512 | 0.003120806 | 63452 | 1 |
| CTONG2024031 | 0.064831259 | 130106 | 13 |
| CTONG2024206 | 0.003120806 | 152314 | 1 |
| CTONG2024614 | 0.003120806 | 114796 | 1 |
| CTONG2024749 | 0.003120806 | 121142 | 1 |
| CTONG2025496 | 0.003120806 | 121138 | 1 |
| CTONG2025513 | 0.003120806 | 105636 | 1 |
| CTONG2025516 | 0.003120806 | 50392 | 1 |
| CTONG2025610 | 0.003120806 | 190580 | 1 |
| CTONG2025823 | 0.104812185 | 103899 | 29 |
| CTONG2025900 | 0.006241613 | 141228 | 2 |
| CTONG2026513 | 0.003120806 | 279437 | 1 |
| CTONG2026770 | 0.003120806 | 171895 | 1 |
| CTONG2026920 | 0.003120806 | 126518 | 1 |
| CTONG2026987 | 0.003120806 | 193881 | 1 |
| CTONG2027150 | 0.003120806 | 135723 | 1 |
| CTONG2027263 | 0.010174556 | 115873 | 6 |
| CTONG2027327 | 0.005915117 | 130565 | 3 |
| CTONG2027361 | 0.003120806 | 167504 | 1 |
| CTONG2027591 | 0.003120806 | 176258 | 1 |
| CTONG2027736 | 0.003120806 | 161070 | 1 |
| CTONG2027783 | 0.003120806 | 207067 | 1 |
| CTONG2027828 | 0.003120806 | 201528 | 1 |
| CTONG2027959 | 0.004310051 | 223767 | 2 |
| CTONG2028097 | 0.003120806 | 84216 | 1 |
| CTONG2028124 | 0.333389741 | 74106 | 72 |
| CTONG2028208 | 0.047316758 | 103024 | 7 |
| CTONG2028309 | 0.022827717 | 91737 | 13 |
| CTONG2028486 | 0.004830267 | 126502 | 2 |
| CTONG2028518 | 0.003120806 | 79299 | 1 |
| CTONG2028687 | 0.003120806 | 65547 | 1 |
| CTONG2028758 | 0.010418404 | 232620 | 3 |
| CTONG3000084 | 0.018149557 | 123226 | 5 |
| CTONG3000657 | 0.043186159 | 54434 | 13 |
| CTONG3000686 | 0.003120806 | 255501 | 1 |
| CTONG3000707 | 0.003120806 | 223771 | 1 |
| CTONG3000896 | 0.003120806 | 258070 | 1 |
| CTONG3001123 | 0.021352338 | 6973 | 8 |
| CTONG3001370 | 0.04782732 | 121139 | 16 |
| CTONG3001420 | 0.006102766 | 125067 | 2 |
| CTONG3001501 | 0.003120806 | 182219 | 1 |
| CTONG3001560 | 0.003120806 | 219139 | 1 |
| CTONG3001605 | 0.003120806 | 102594 | 1 |
| CTONG3001746 | 0.011712566 | 125383 | 6 |
| CTONG3002020 | 0.003120806 | 261829 | 1 |
| CTONG3002127 | 0.004997648 | 138614 | 2 |
| CTONG3002370 | 0.003120806 | 155186 | 1 |
| CTONG3002412 | 0.015657464 | 132482 | 3 |
| CTONG3002433 | 0.003120806 | 160967 | 1 |
| CTONG3002518 | 0.019857151 | 115778 | 5 |
| CTONG3002552 | 0.003120806 | 217505 | 1 |
| CTONG3002588 | 0.003120806 | 257921 | 1 |
| CTONG3002674 | 0.007499921 | 145272 | 3 |
| CTONG3002728 | 0.003120806 | 162301 | 1 |
| CTONG3002835 | 0.152511253 | 70898 | 51 |
| CTONG3002909 | 0.003120806 | 231263 | 1 |
| CTONG3002947 | 0.003120806 | 265335 | 1 |
| CTONG3002983 | 0.003120806 | 213712 | 1 |
| CTONG3003165 | 0.003120806 | 206650 | 1 |
| CTONG3003179 | 0.003120806 | 155199 | 1 |
| CTONG3003483 | 0.003120806 | 190414 | 1 |
| CTONG3003553 | 0.003120806 | 174419 | 1 |
| CTONG3003598 | 0.003120806 | 158758 | 1 |
| CTONG3003652 | 0.003120806 | 79140 | 1 |
| CTONG3003654 | 0.003120806 | 186697 | 1 |
| CTONG3003669 | 0.003120806 | 83233 | 1 |
| CTONG3003737 | 0.006241613 | 169505 | 2 |
| CTONG3003905 | 0.008556245 | 144878 | 3 |
| CTONG3003972 | 0.006035663 | 114203 | 2 |
| CTONG3004072 | 0.00858395 | 169053 | 4 |
| CTONG3004317 | 0.005119208 | 164909 | 2 |
| CTONG3004397 | 0.003120806 | 186737 | 1 |
| CTONG3004550 | 0.003120806 | 193373 | 1 |
| CTONG3004576 | 0.004997648 | 264550 | 2 |
| CTONG3004607 | 0.003120806 | 193312 | 1 |
| CTONG3004712 | 0.003120806 | 189679 | 1 |
| CTONG3004726 | 0.009893738 | 186753 | 4 |
| CTONG3005325 | 0.003120806 | 223681 | 1 |
| CTONG3005648 | 0.003120806 | 144963 | 1 |
| CTONG3005713 | 0.003120806 | 166765 | 1 |
| CTONG3005803 | 0.003120806 | 262194 | 1 |
| CTONG3005813 | 0.004310051 | 148009 | 2 |
| CTONG3006067 | 0.004216085 | 250341 | 2 |
| CTONG3006073 | 0.003120806 | 275889 | 1 |
| CTONG3006186 | 0.003120806 | 193154 | 1 |
| CTONG3006629 | 0.005202318 | 210397 | 2 |
| CTONG3006650 | 0.003120806 | 263678 | 1 |
| CTONG3006659 | 0.003120806 | 213612 | 1 |
| CTONG3007156 | 0.030068076 | 139630 | 11 |
| CTONG3007244 | 0.003120806 | 134002 | 1 |
| CTONG3007444 | 0.003120806 | 206812 | 1 |
| CTONG3007528 | 0.003120806 | 189773 | 1 |
| CTONG3007586 | 0.003120806 | 218484 | 1 |
| CTONG3007870 | 0.003120806 | 155788 | 1 |
| CTONG3008223 | 0.003120806 | 166729 | 1 |
| CTONG3008252 | 0.003120806 | 138752 | 1 |
| CTONG3008258 | 0.003120806 | 130160 | 1 |
| CTONG3008457 | 0.003120806 | 193532 | 1 |
| CTONG3008496 | 0.003120806 | 210268 | 1 |
| CTONG3008566 | 0.003120806 | 159106 | 1 |
| CTONG3008639 | 0.003120806 | 142559 | 1 |
| CTONG3008831 | 0.003120806 | 176161 | 1 |
| CTONG3008894 | 0.23050447 | 54222 | 65 |
| CTONG3008951 | 0.003120806 | 189767 | 1 |
| CTONG3008959 | 0.003120806 | 280325 | 1 |
| CTONG3009028 | 0.104117944 | 101576 | 17 |
| CTONG3009227 | 0.003120806 | 212143 | 1 |
| CTONG3009239 | 0.116049925 | 122198 | 20 |
| CTONG3009287 | 0.003120806 | 266067 | 1 |
| CTONG3009328 | 0.013971528 | 166252 | 4 |
| CTONG3009385 | 0.003120806 | 283717 | 1 |
| D3OST1000066 | 0.019561815 | 106133 | 1 |
| D3OST1000109 | 0.093873669 | 65300 | 5 |
| D3OST1000238 | 0.019561815 | 70264 | 1 |
| D3OST1000267 | 0.038801408 | 61539 | 6 |
| D3OST1000270 | 0.06484856 | 165871 | 10 |
| D3OST2000184 | 0.019561815 | 115674 | 1 |
| D3OST2000618 | 0.019561815 | 233011 | 1 |
| D3OST2000654 | 0.019561815 | 240422 | 1 |
| D3OST2000734 | 0.020958971 | 165082 | 2 |
| D3OST2001328 | 0.019561815 | 54994 | 1 |
| D3OST2001598 | 0.133620252 | 162823 | 25 |
| D3OST2001669 | 0.019561815 | 160748 | 1 |
| D3OST2001670 | 0.044906326 | 32003 | 16 |
| D3OST2001788 | 0.057455321 | 207448 | 3 |
| D3OST2002068 | 0.779927307 | 13188 | 36 |
| D3OST2002182 | 0.022543774 | 181875 | 2 |
| D3OST2002223 | 0.019561815 | 196249 | 1 |
| D3OST2002262 | 0.019561815 | 10981 | 1 |
| D3OST2002272 | 0.019561815 | 184492 | 1 |
| D3OST2002417 | 0.019561815 | 181802 | 1 |
| D3OST2002436 | 0.019561815 | 59507 | 1 |
| D3OST2002648 | 0.111685261 | 205579 | 3 |
| D3OST2003024 | 0.020657094 | 172212 | 2 |
| D3OST2003331 | 0.019561815 | 102138 | 1 |
| D3OST2003607 | 0.042155579 | 172299 | 2 |
| D3OST2003797 | 0.019561815 | 209937 | 1 |
| D3OST2003856 | 0.028522523 | 139468 | 4 |
| D3OST2004637 | 0.019561815 | 83434 | 1 |
| D3OST3000169 | 0.342393646 | 107384 | 16 |
| D3OST3000195 | 0.019561815 | 101848 | 1 |
| D3OST3000258 | 0.026954175 | 166297 | 5 |
| D3OST3000291 | 0.020958971 | 225614 | 2 |
| D3OST3000388 | 0.039123631 | 129867 | 2 |
| D3OST3000493 | 0.019561815 | 13211 | 1 |
| D6OST2000127 | 0.112485939 | 22207 | 1 |
| D6OST2000169 | 0.112485939 | 165157 | 1 |
| D6OST2000245 | 0.112485939 | 34544 | 1 |
| D6OST2000358 | 0.117909172 | 14321 | 4 |
| D6OST2000445 | 0.13116108 | 21045 | 7 |
| D6OST2000464 | 0.152933569 | 68417 | 10 |
| D6OST2000507 | 0.112485939 | 234407 | 1 |
| D9OST2000031 | 0.03546628 | 127947 | 4 |
| D9OST2000278 | 0.022593764 | 240111 | 1 |
| D9OST2000440 | 0.022593764 | 260948 | 1 |
| D9OST2000869 | 0.022593764 | 116154 | 1 |
| D9OST2001319 | 0.022593764 | 13197 | 1 |
| D9OST2001433 | 0.022593764 | 94735 | 1 |
| D9OST2001547 | 0.320836355 | 113852 | 56 |
| D9OST2002397 | 0.059713289 | 27384 | 2 |
| D9OST2002608 | 0.075207697 | 5633 | 24 |
| D9OST2002673 | 0.11471721 | 140952 | 3 |
| D9OST2003106 | 0.024269311 | 163466 | 2 |
| D9OST2003108 | 0.034354319 | 116037 | 2 |
| D9OST2003137 | 0.022593764 | 149663 | 1 |
| D9OST2003397 | 0.0586298 | 110749 | 2 |
| D9OST2003580 | 0.024282041 | 205287 | 2 |
| D9OST2003594 | 0.022593764 | 119273 | 1 |
| D9OST2003762 | 0.076873546 | 88189 | 4 |
| D9OST2003791 | 0.027386476 | 190681 | 4 |
| D9OST2003989 | 0.022593764 | 186515 | 1 |
| D9OST2004018 | 0.022593764 | 196472 | 1 |
| D9OST2004073 | 0.022593764 | 174322 | 1 |
| D9OST2004417 | 0.022593764 | 194901 | 1 |
| DFNES1000003 | 0.009874593 | 70308 | 1 |
| DFNES1000059 | 0.36328603 | 48128 | 52 |
| DFNES1000083 | 0.074487441 | 46150 | 11 |
| DFNES1000107 | 0.009874593 | 91562 | 1 |
| DFNES1000150 | 0.150038079 | 72887 | 26 |
| DFNES1000185 | 0.127043346 | 54022 | 4 |
| DFNES2000011 | 0.306413222 | 134223 | 35 |
| DFNES2000143 | 0.009874593 | 155119 | 1 |
| DFNES2000146 | 0.015051601 | 37368 | 3 |
| DFNES2000153 | 0.052333198 | 124873 | 7 |
| DFNES2000212 | 0.011562869 | 255753 | 2 |
| DFNES2000268 | 0.151739919 | 101980 | 12 |
| DFNES2000292 | 0.044645918 | 170876 | 6 |
| DFNES2000335 | 0.035367106 | 159474 | 10 |
| DFNES2000426 | 0.013270149 | 121257 | 3 |
| DFNES2000432 | 0.022469051 | 189201 | 2 |
| DFNES2000443 | 0.246835777 | 102486 | 13 |
| DFNES2000457 | 0.018864075 | 200670 | 4 |
| DFNES2000471 | 0.009874593 | 240352 | 1 |
| DFNES2000526 | 0.060630942 | 105554 | 14 |
| DFNES2000913 | 0.009874593 | 122514 | 1 |
| DFNES2001091 | 0.019523855 | 146980 | 3 |
| DFNES2001096 | 0.021864736 | 244363 | 3 |
| DFNES2001108 | 0.009874593 | 143613 | 1 |
| DFNES2001177 | 0.009874593 | 115344 | 1 |
| DFNES2001404 | 0.016076912 | 206200 | 2 |
| DFNES2001564 | 0.009874593 | 225231 | 1 |
| DFNES2001565 | 0.009874593 | 226983 | 1 |
| DFNES2001647 | 0.009874593 | 235448 | 1 |
| DFNES2001800 | 0.009874593 | 128303 | 1 |
| DFNES2001829 | 0.018156706 | 142272 | 6 |
| DFNES2002220 | 0.009874593 | 273079 | 1 |
| DFNES2002509 | 0.033000279 | 166587 | 3 |
| DFNES2002550 | 0.009874593 | 49135 | 1 |
| DFNES2002588 | 0.009874593 | 134598 | 1 |
| DFNES2002817 | 0.009874593 | 55052 | 1 |
| DFNES2002966 | 0.010969871 | 153176 | 2 |
| DFNES2003081 | 0.021277101 | 109172 | 2 |
| DFNES2003192 | 0.05593799 | 169863 | 5 |
| DFNES2003255 | 0.009874593 | 194431 | 1 |
| DFNES2003742 | 0.016076912 | 92268 | 2 |
| DFNES2004346 | 0.053538604 | 189798 | 4 |
| DFNES2004354 | 0.009874593 | 21250 | 1 |
| DFNES2004371 | 0.106203986 | 143173 | 24 |
| DFNES2004383 | 0.009874593 | 244456 | 1 |
| DFNES2004608 | 0.022223321 | 167623 | 2 |
| DFNES2004676 | 0.523525708 | 65148 | 65 |
| DFNES2004684 | 0.009874593 | 226924 | 1 |
| DFNES2005266 | 0.009874593 | 162205 | 1 |
| DFNES2005527 | 0.009874593 | 174374 | 1 |
| DFNES2005540 | 0.055428176 | 51301 | 6 |
| DFNES2005690 | 0.033003076 | 102213 | 13 |
| DFNES2005766 | 0.091393352 | 69311 | 7 |
| DFNES2005779 | 0.009874593 | 189676 | 1 |
| DFNES2005875 | 0.009874593 | 86363 | 1 |
| DFNES2006346 | 0.023718542 | 162010 | 7 |
| DFNES2006944 | 0.009874593 | 172325 | 1 |
| DFNES2007113 | 0.025073495 | 83012 | 4 |
| DFNES2007299 | 0.051018213 | 155151 | 3 |
| DFNES2007332 | 0.045147519 | 177409 | 5 |
| DFNES2007634 | 0.188510464 | 49606 | 58 |
| DFNES2007641 | 0.015679771 | 180029 | 2 |
| DFNES2007757 | 0.044500631 | 187106 | 2 |
| DFNES2008088 | 0.063735892 | 58501 | 10 |
| DFNES2008160 | 0.037401317 | 85928 | 16 |
| DFNES2008280 | 0.009874593 | 181779 | 1 |
| DFNES2008881 | 0.009874593 | 176061 | 1 |
| DFNES2009482 | 0.118139575 | 122834 | 10 |
| DFNES2010502 | 0.009874593 | 142265 | 1 |
| DFNES2011192 | 0.009874593 | 93746 | 1 |
| DFNES2011221 | 0.011751434 | 207725 | 2 |
| DFNES2011239 | 0.022424565 | 73183 | 3 |
| DFNES2011245 | 0.011584053 | 10966 | 2 |
| DFNES2011380 | 0.014333695 | 84318 | 4 |
| DFNES2011499 | 0.027007696 | 120899 | 10 |
| ERLTF1000021 | 0.045913682 | 244656 | 1 |
| ERLTF2000324 | 0.045913682 | 250347 | 1 |
| ERLTF2000465 | 0.045913682 | 142255 | 1 |
| FCBBF1000023 | 0.023819499 | 60177 | 4 |
| FCBBF1000024 | 0.076316914 | 49417 | 29 |
| FCBBF1000025 | 0.052359894 | 36915 | 11 |
| FCBBF1000027 | 0.45742453 | 62524 | 90 |
| FCBBF1000038 | 0.014914122 | 68397 | 6 |
| FCBBF1000053 | 0.061912034 | 35621 | 13 |
| FCBBF1000061 | 0.071243067 | 22766 | 26 |
| FCBBF1000063 | 0.028015417 | 14993 | 12 |
| FCBBF1000069 | 0.003095496 | 38028 | 1 |
| FCBBF1000077 | 0.206886551 | 62771 | 45 |
| FCBBF1000115 | 0.008243094 | 44497 | 4 |
| FCBBF1000117 | 0.003095496 | 102986 | 1 |
| FCBBF1000118 | 0.011049492 | 48994 | 5 |
| FCBBF1000121 | 0.016865074 | 9752 | 4 |
| FCBBF1000125 | 0.01075679 | 67675 | 5 |
| FCBBF1000131 | 0.003095496 | 52379 | 1 |
| FCBBF1000139 | 0.095848531 | 39474 | 37 |
| FCBBF1000155 | 0.003095496 | 21603 | 1 |
| FCBBF1000157 | 0.003095496 | 62763 | 1 |
| FCBBF1000171 | 0.003095496 | 60036 | 1 |
| FCBBF1000174 | 0.003095496 | 29641 | 1 |
| FCBBF1000182 | 0.019758782 | 11390 | 5 |
| FCBBF1000197 | 0.15685083 | 2587 | 55 |
| FCBBF1000198 | 0.003095496 | 33978 | 1 |
| FCBBF1000206 | 0.003095496 | 63982 | 1 |
| FCBBF1000220 | 0.003095496 | 22880 | 1 |
| FCBBF1000232 | 0.003095496 | 16449 | 1 |
| FCBBF1000233 | 0.003095496 | 68393 | 1 |
| FCBBF1000243 | 0.003095496 | 29523 | 1 |
| FCBBF1000270 | 0.003095496 | 48993 | 1 |
| FCBBF1000272 | 0.004804956 | 56068 | 2 |
| FCBBF1000280 | 0.029664149 | 41382 | 4 |
| FCBBF1000294 | 0.155624815 | 48302 | 44 |
| FCBBF1000297 | 0.003095496 | 74266 | 1 |
| FCBBF1000305 | 0.009340032 | 65758 | 4. |
| FCBBF1000314 | 0.003095496 | 1 | 1 |
| FCBBF1000322 | 0.003095496 | 32180 | 1 |
| FCBBF1000349 | 0.003095496 | 20926 | 1 |
| FCBBF1000367 | 0.110295219 | 4791 | 14 |
| FCBBF1000374 | 0.004190774 | 572 | 2 |
| FCBBF1000376 | 0.007866539 | 66556 | 3 |
| FCBBF1000377 | 0.015494321 | 46845 | 5 |
| FCBBF1000395 | 0.003095496 | 28170 | 1 |
| FCBBF1000397 | 0.003095496 | 2853 | 1 |
| FCBBF1000403 | 0.019977624 | 55364 | 5 |
| FCBBF1000412 | 0.086580986 | 58258 | 18 |
| FCBBF1000425 | 0.032095449 | 73402 | 11 |
| FCBBF1000437 | 0.013102145 | 55983 | 4 |
| FCBBF1000449 | 0.025062187 | 47334 | 7 |
| FCBBF1000466 | 0.263102133 | 55886 | 74 |
| FCBBF1000476 | 0.019293346 | 82143 | 9 |
| FCBBF1000506 | 0.009007758 | 68182 | 2 |
| FCBBF1000509 | 0.089799033 | 35365 | 28 |
| FCBBF1000526 | 0.080158054 | 3135 | 26 |
| FCBBF1000546 | 0.003095496 | 78873 | 1 |
| FCBBF1000550 | 0.003095496 | 76601 | 1 |
| FCBBF1000556 | 0.157181137 | 846 | 63 |
| FCBBF1000563 | 0.111559045 | 77935 | 35 |
| FCBBF1000574 | 0.080717809 | 69258 | 39 |
| FCBBF1000581 | 0.003095496 | 28339 | 1 |
| FCBBF1000582 | 0.003095496 | 83783 | 1 |
| FCBBF1000598 | 0.003095496 | 162089 | 1 |
| FCBBF1000604 | 0.003095496 | 100093 | 1 |
| FCBBF1000618 | 0.003095496 | 63094 | 1 |
| FCBBF1000627 | 0.003095496 | 74410 | 1 |
| FCBBF1000671 | 0.012238737 | 51017 | 6 |
| FCBBF1000675 | 0.013194834 | 69613 | 5 |
| FCBBF1000684 | 0.009331854 | 51019 | 2 |
| FCBBF1000686 | 0.007272431 | 70185 | 2 |
| FCBBF1000687 | 0.019396227 | 132528 | 8 |
| FCBBF1000691 | 0.003095496 | 69392 | 1 |
| FCBBF1000732 | 0.059837989 | 86412 | 9 |
| FCBBF1000748 | 0.00495361 | 11819 | 2 |
| FCBBF1000760 | 0.255865764 | 32709 | 29 |
| FCBBF2000038 | 0.124736983 | 28224 | 39 |
| FCBBF2000087 | 0.058081999 | 76337 | 7 |
| FCBBF2000094 | 0.003095496 | 117702 | 1 |
| FCBBF2000105 | 0.003095496 | 263895 | 1 |
| FCBBF2000123 | 0.003095496 | 265963 | 1 |
| FCBBF2000195 | 0.040311722 | 163377 | 2 |
| FCBBF2000199 | 0.059891866 | 79334 | 27 |
| FCBBF2000214 | 0.003095496 | 183947 | 1 |
| FCBBF2000232 | 0.003095496 | 54920 | 1 |
| FCBBF2000259 | 0.028019685 | 97114 | 9 |
| FCBBF2000276 | 0.003095496 | 129052 | 1 |
| FCBBF2000291 | 0.003095496 | 229153 | 1 |
| FCBBF2000362 | 0.020017388 | 101639 | 9 |
| FCBBF2000379 | 0.003095496 | 275965 | 1 |
| FCBBF2000473 | 0.003095496 | 132162 | 1 |
| FCBBF2000502 | 0.025398692 | 135290 | 3 |
| FCBBF2000566 | 0.045079387 | 136406 | 14 |
| FCBBF2000576 | 0.014882389 | 242007 | 2 |
| FCBBF2000591 | 0.584454109 | 94457 | 13 |
| FCBBF2000677 | 0.003095496 | 261367 | 1 |
| FCBBF2000678 | 0.003095496 | 283276 | 1 |
| FCBBF2000685 | 0.024628816 | 100828 | 5 |
| FCBBF2000733 | 0.021141239 | 108905 | 9 |
| FCBBF2000782 | 0.004492651 | 82708 | 2 |
| FCBBF2000808 | 0.003095496 | 263938 | 1 |
| FCBBF2000815 | 0.003095496 | 284071 | 1 |
| FCBBF2000825 | 0.022657311 | 238945 | 2 |
| FCBBF2000885 | 0.10915845 | 111627 | 42 |
| FCBBF2000940 | 0.00495361 | 201747 | 2 |
| FCBBF2000951 | 0.003095496 | 99499 | 1 |
| FCBBF2001001 | 0.017531439 | 111026 | 6 |
| FCBBF2001088 | 0.010334114 | 22044 | 3 |
| FCBBF2001105 | 0.055198885 | 22492 | 12 |
| FCBBF2001116 | 0.003095496 | 263718 | 1 |
| FCBBF2001183 | 0.10132157 | 79371 | 45 |
| FCBBF2001188 | 0.003095496 | 225291 | 1 |
| FCBBF2001211 | 0.026145652 | 134566 | 10 |
| FCBBF2001238 | 0.003095496 | 96304 | 1 |
| FCBBF2001243 | 0.003095496 | 225292 | 1 |
| FCBBF2001291 | 0.088074524 | 137034 | 18 |
| FCBBF2001299 | 0.007380237 | 189283 | 3 |
| FCBBF2001309 | 0.038511283 | 94283 | 13 |
| FCBBF2001400 | 0.008227607 | 82606 | 4 |
| FCBBF2001427 | 0.003095496 | 113787 | 1 |
| FCBBF2001480 | 0.00693958 | 183077 | 2 |
| FCBBF2001529 | 0.003095496 | 230424 | 1 |
| FCBBF2001538 | 0.21990932 | 118326 | 82 |
| FCBBF2001594 | 0.044059453 | 94350 | 25 |
| FCBBF2001672 | 0.003095496 | 44484 | 1 |
| FCBBF2001675 | 0.003095496 | 268010 | 1 |
| FCBBF2001718 | 0.003095496 | 259602 | 1 |
| FCBBF2001720 | 0.003095496 | 179421 | 1 |
| FCBBF2001817 | 0.00693958 | 18763 | 2 |
| FCBBF2001868 | 0.006190992 | 118121 | 2 |
| FCBBF2002044 | 0.003095496 | 183700 | 1 |
| FCBBF2002111 | 0.014559442 | 143975 | 2 |
| FCBBF2002281 | 0.010367927 | 110123 | 3 |
| FCBBF2002349 | 0.042442686 | 103204 | 13 |
| FCBBF2002370 | 0.003095496 | 109478 | 1 |
| FCBBF2002541 | 0.003095496 | 197892 | 1 |
| FCBBF2002626 | 0.004284741 | 3985 | 2 |
| FCBBF2002801 | 0.064558893 | 111089 | 19 |
| FCBBF2002897 | 0.003095496 | 141060 | 1 |
| FCBBF2002898 | 0.003095496 | 160955 | 1 |
| FCBBF2002904 | 0.003095496 | 22851 | 1 |
| FCBBF2002928 | 0.053850774 | 199720 | 4 |
| FCBBF2002953 | 0.003095496 | 43724 | 1 |
| FCBBF2003083 | 0.003095496 | 151747 | 1 |
| FCBBF2003102 | 0.008367709 | 80220 | 3 |
| FCBBF2003269 | 0.003095496 | 207147 | 1 |
| FCBBF2003293 | 0.009137062 | 167120 | 2 |
| FCBBF2003297 | 0.003095496 | 207159 | 1 |
| FCBBF2003336 | 0.262421535 | 94790 | 41 |
| FCBBF2003395 | 0.003095496 | 211024 | 1 |
| FCBBF2003528 | 0.003095496 | 52583 | 1 |
| FCBBF2003543 | 0.004771043 | 150811 | 2 |
| FCBBF2003549 | 0.014600328 | 45661 | 2 |
| FCBBF2003567 | 0.025703904 | 63614 | 10 |
| FCBBF2003636 | 0.003095496 | 190114 | 1 |
| FCBBF2003823 | 0.003095496 | 172921 | 1 |
| FCBBF2003895 | 0.003095496 | 199750 | 1 |
| FCBBF2004066 | 0.083115518 | 88587 | 28 |
| FCBBF2004090 | 0.003095496 | 49898 | 1 |
| FCBBF2004138 | 0.003095496 | 96860 | 1 |
| FCBBF2004217 | 0.003095496 | 2580 | 1 |
| FCBBF2004256 | 0.003095496 | 183624 | 1 |
| FCBBF2004373 | 0.007133779 | 24091 | 2 |
| FCBBF2004448 | 0.003095496 | 104546 | 1 |
| FCBBF2004671 | 0.003095496 | 230442 | 1 |
| FCBBF2004707 | 0.003095496 | 203865 | 1 |
| FCBBF2004788 | 0.003095496 | 139777 | 1 |
| FCBBF2004930 | 0.105565309 | 110213 | 33 |
| FCBBF2005069 | 0.003095496 | 132134 | 1 |
| FCBBF2005122 | 0.003095496 | 112227 | 1 |
| FCBBF2005245 | 0.23923172 | 104456 | 77 |
| FCBBF2005428 | 0.004284741 | 204446 | 2 |
| FCBBF2005439 | 0.006190992 | 96429 | 2 |
| FCBBF2005538 | 0.003095496 | 163134 | 1 |
| FCBBF2005637 | 0.06029586 | 155045 | 3 |
| FCBBF2005645 | 0.003095496 | 190701 | 1 |
| FCBBF2005658 | 0.021528592 | 109338 | 4 |
| FCBBF2005733 | 0.005177008 | 98294 | 2 |
| FCBBF2005909 | 0.026846921 | 18694 | 13 |
| FCBBF2005966 | 0.017691894 | 112189 | 5 |
| FCBBF2006131 | 0.003095496 | 106381 | 1 |
| FCBBF2006380 | 0.005093897 | 151005 | 2 |
| FCBBF2006418 | 0.003095496 | 121988 | 1 |
| FCBBF2006452 | 0.003095496 | 148978 | 1 |
| FCBBF2006634 | 0.035497262 | 143610 | 5 |
| FCBBF2006781 | 0.026479008 | 163248 | 6 |
| FCBBF2006882 | 0.048078306 | 18948 | 4 |
| FCBBF2007012 | 0.003095496 | 181784 | 1 |
| FCBBF2007080 | 0.039628131 | 86047 | 18 |
| FCBBF2007095 | 0.084263661 | 50258 | 9 |
| FCBBF2007186 | 0.003095496 | 175519 | 1 |
| FCBBF2007265 | 0.003095496 | 90437 | 1 |
| FCBBF2007302 | 0.007133779 | 155784 | 2 |
| FCBBF2007556 | 0.018334719 | 127067 | 4 |
| FCBBF2007610 | 0.006190992 | 98276 | 2 |
| FCBBF2007633 | 0.003095496 | 162796 | 1 |
| FCBBF2007840 | 0.013452619 | 42083 | 4 |
| FCBBF3000001 | 0.016389065 | 111079 | 6 |
| FCBBF3000102 | 0.013503056 | 105120 | 6 |
| FCBBF3000110 | 0.025944427 | 140529 | 8 |
| FCBBF3000115 | 0.041984388 | 80345 | 22 |
| FCBBF3000120 | 0.003095496 | 220253 | 1 |
| FCBBF3000168 | 0.020579852 | 95687 | 9 |
| FCBBF3000175 | 0.039903954 | 33268 | 7 |
| FCBBF3000184 | 0.011185197 | 183619 | 4 |
| FCBBF3000227 | 0.014901871 | 65144 | 2 |
| FCBBF3000228 | 0.018696377 | 107760 | 8 |
| FCBBF3000229 | 0.017352873 | 97121 | 4 |
| FCBBF3000233 | 0.003095496 | 234177 | 1 |
| FCBBF3000269 | 0.005473985 | 213191 | 3 |
| FCBBF3000361 | 0.003095496 | 128820 | 1 |
| FCBBF3000367 | 0.003095496 | 231682 | 1 |
| FCBBF3000388 | 0.067835682 | 50807 | 21 |
| FCBBF3000434 | 0.090504275 | 93919 | 35 |
| FCBBF3000462 | 0.003095496 | 196256 | 1 |
| FCBBF3000473 | 0.003095496 | 61548 | 1 |
| FCBBF3000501 | 0.003095496 | 264017 | 1 |
| FCBBF3000518 | 0.003095496 | 203242 | 1 |
| FCBBF3000536 | 0.062857826 | 119699 | 8 |
| FCBBF3000550 | 0.003095496 | 108008 | 1 |
| FCBBF3000768 | 0.027878218 | 146137 | 8 |
| FCBBF3000847 | 0.003095496 | 160555 | 1 |
| FCBBF3001018 | 0.003095496 | 144149 | 1 |
| FCBBF3001020 | 0.003095496 | 198381 | 1 |
| FCBBF3001081 | 0.063813822 | 18682 | 17 |
| FCBBF3001235 | 0.003095496 | 34590 | 1 |
| FCBBF3001281 | 0.008229057 | 155647 | 3 |
| FCBBF3001302 | 0.050406334 | 73062 | 3 |
| FCBBF3001377 | 0.016272096 | 186082 | 7 |
| FCBBF3001470 | 0.12343259 | 2956 | 15 |
| FCBBF3001594 | 0.003095496 | 277262 | 1 |
| FCBBF3001632 | 0.108539818 | 157195 | 21 |
| FCBBF3001657 | 0.071027019 | 89830 | 30 |
| FCBBF3001818 | 0.018394886 | 155304 | 8 |
| FCBBF3001855 | 0.059831107 | 52248 | 11 |
| FCBBF3001890 | 0.060915562 | 156540 | 14 |
| FCBBF3001912 | 0.003095496 | 33343 | 1 |
| FCBBF3001914 | 0.003095496 | 131677 | 1 |
| FCBBF3001918 | 0.003095496 | 12687 | 1 |
| FCBBF3001924 | 0.003095496 | 143587 | 1 |
| FCBBF3001977 | 0.0224955 | 161197 | 11 |
| FCBBF3002163 | 0.003095496 | 106152 | 1 |
| FCBBF3002188 | 0.003095496 | 197370 | 1 |
| FCBBF3002190 | 0.003095496 | 18929 | 1 |
| FCBBF3002268 | 0.003095496 | 103104 | 1 |
| FCBBF3002334 | 0.209966321 | 34085 | 59 |
| FCBBF3002475 | 0.003095496 | 71492 | 1 |
| FCBBF3002556 | 0.005177008 | 102832 | 2 |
| FCBBF3002592 | 0.003095496 | 166891 | 1 |
| FCBBF3002658 | 0.003095496 | 118928 | 1 |
| FCBBF3002782 | 0.003095496 | 142376 | 1 |
| FCBBF3002818 | 0.004771043 | 107319 | 2 |
| FCBBF3002925 | 0.003095496 | 105298 | 1 |
| FCBBF3003216 | 0.003095496 | 50836 | 1 |
| FCBBF3003305 | 0.003095496 | 96192 | 1 |
| FCBBF3003373 | 0.003095496 | 179753 | 1 |
| FCBBF3003435 | 0.018714474 | 207259 | 3 |
| FCBBF3003475 | 0.003095496 | 166878 | 1 |
| FCBBF3003481 | 0.003095496 | 84909 | 1 |
| FCBBF3003557 | 0.004771043 | 195270 | 2 |
| FCBBF3003787 | 0.017119771 | 128336 | 3 |
| FCBBF3003800 | 0.425362467 | 115654 | 125 |
| FCBBF3003902 | 0.007133779 | 179937 | 2 |
| FCBBF3004021 | 0.015689954 | 67962 | 2 |
| FCBBF3004041 | 0.044901516 | 165850 | 2 |
| FCBBF3004261 | 0.018522597 | 13249 | 7 |
| FCBBF3004323 | 0.030411669 | 80988 | 11 |
| FCBBF3004390 | 0.003095496 | 3900 | 1 |
| FCBBF3004409 | 0.005177008 | 71434 | 2 |
| FCBBF3004473 | 0.145841376 | 31452 | 30 |
| FCBBF3004502 | 0.003095496 | 81209 | 1 |
| FCBBF3004715 | 0.044746061 | 36441 | 5 |
| FCBBF3004842 | 0.003095496 | 144487 | 1 |
| FCBBF3004847 | 0.005093897 | 216184 | 2 |
| FCBBF3004955 | 0.021740844 | 80574 | 9 |
| FCBBF3005160 | 0.003095496 | 151590 | 1 |
| FCBBF3005218 | 0.003095496 | 138158 | 1 |
| FCBBF3005320 | 0.061422066 | 31211 | 16 |
| FCBBF3005330 | 0.241870702 | 56356 | 77 |
| FCBBF3005444 | 0.006190992 | 109183 | 2 |
| FCBBF3005497 | 0.213136262 | 78006 | 56 |
| FCBBF3005698 | 0.003095496 | 183684 | 1 |
| FCBBF3005729 | 0.003095496 | 110961 | 1 |
| FCBBF3006171 | 0.003095496 | 262531 | 1 |
| FCBBF3006249 | 0.003095496 | 138355 | 1 |
| FCBBF3006288 | 0.003095496 | 135436 | 1 |
| FCBBF3006399 | 0.003095496 | 162729 | 1 |
| FCBBF3006513 | 0.030648354 | 61532 | 12 |
| FCBBF3006628 | 0.003095496 | 9180 | 1 |
| FCBBF3006821 | 0.029311461 | 118609 | 18 |
| FCBBF3007077 | 0.003095496 | 5710 | 1 |
| FCBBF3007150 | 0.006190992 | 189818 | 2 |
| FCBBF3007152 | 0.003095496 | 180493 | 1 |
| FCBBF3007242 | 0.003095496 | 231705 | 1 |
| FCBBF3007244 | 0.021585909 | 157755 | 6 |
| FCBBF3007248 | 0.003095496 | 62467 | 1 |
| FCBBF3007453 | 0.003095496 | 194201 | 1 |
| FCBBF3007462 | 0.004804956 | 118823 | 2 |
| FCBBF3007540 | 0.003095496 | 230414 | 1 |
| FCBBF3007597 | 0.006709388 | 196496 | 2 |
| FCBBF3007604 | 0.003095496 | 220987 | 1 |
| FCBBF3007631 | 0.007669748 | 28265 | 4 |
| FCBBF3007829 | 0.014893514 | 105631 | 2 |
| FCBBF3007855 | 0.003095496 | 108031 | 1 |
| FCBBF3007859 | 0.025792706 | 69538 | 14 |
| FCBBF3007860 | 0.016559245 | 105628 | 9 |
| FCBBF3007977 | 0.044113724 | 132884 | 19 |
| FCBBF3008073 | 0.003095496 | 28057 | 1 |
| FCBBF3008100 | 0.011231376 | 120283 | 3 |
| FCBBF3008153 | 0.030625467 | 8804 | 15 |
| FCBBF3008159 | 0.008090791 | 8205 | 5 |
| FCBBF3008251 | 0.003095496 | 10075 | 1 |
| FCBBF3008311 | 0.006190992 | 155600 | 2 |
| FCBBF3008362 | 0.003095496 | 111566 | 1 |
| FCBBF3008382 | 0.015556263 | 207555 | 4 |
| FCBBF3008475 | 0.003095496 | 146780 | 1 |
| FCBBF3008556 | 0.004190774 | 155227 | 2 |
| FCBBF3008644 | 0.019447014 | 139467 | 4 |
| FCBBF3008689 | 0.017951547 | 143439 | 3 |
| FCBBF3008748 | 0.003095496 | 65274 | 1 |
| FCBBF3008870 | 0.003095496 | 215271 | 1 |
| FCBBF3008938 | 0.006010353 | 118747 | 2 |
| FCBBF3008944 | 0.003095496 | 285350 | 1 |
| FCBBF3009069 | 0.037415329 | 90386 | 21 |
| FCBBF3009101 | 0.004771043 | 120883 | 2 |
| FCBBF3009152 | 0.008809326 | 154077 | 3 |
| FCBBF3009256 | 0.004492651 | 254894 | 2 |
| FCBBF3009317 | 0.006190992 | 45274 | 2 |
| FCBBF3009428 | 0.003095496 | 80029 | 1 |
| FCBBF3009464 | 0.003095496 | 108257 | 1 |
| FCBBF3009479 | 0.016938605 | 147290 | 4 |
| FCBBF3009526 | 0.014182646 | 146947 | 6 |
| FCBBF3009541 | 0.406452685 | 87045 | 44 |
| FCBBF3009703 | 0.005177008 | 96427 | 2 |
| FCBBF3009717 | 0.003095496 | 250383 | 1 |
| FCBBF3009888 | 0.003095496 | 58275 | 1 |
| FCBBF3009949 | 0.003095496 | 102466 | 1 |
| FCBBF3009978 | 0.009031271 | 140827 | 2 |
| FCBBF3010008 | 0.003095496 | 133334 | 1 |
| FCBBF3010012 | 0.072918291 | 109313 | 27 |
| FCBBF3010041 | 0.092918664 | 130311 | 35 |
| FCBBF3010124 | 0.003095496 | 181016 | 1 |
| FCBBF3010130 | 0.003095496 | 180997 | 1 |
| FCBBF3010142 | 0.011609374 | 117093 | 6 |
| FCBBF3010149 | 0.003095496 | 155978 | 1 |
| FCBBF3010211 | 0.003095496 | 196702 | 1 |
| FCBBF3010361 | 0.003095496 | 101835 | 1 |
| FCBBF3010508 | 0.014856051 | 91710 | 2 |
| FCBBF3010544 | 0.003095496 | 91163 | 1 |
| FCBBF3010586 | 0.003095496 | 84520 | 1 |
| FCBBF3010601 | 0.003095496 | 200384 | 1 |
| FCBBF3010665 | 0.006369493 | 59284 | 3 |
| FCBBF3010695 | 0.067566706 | 15347 | 10 |
| FCBBF3010729 | 0.003095496 | 74635 | 1 |
| FCBBF3010733 | 0.003095496 | 174591 | 1 |
| FCBBF3011003 | 0.003095496 | 268668 | 1 |
| FCBBF3011183 | 0.013295058 | 99956 | 5 |
| FCBBF3011418 | 0.003095496 | 200677 | 1 |
| FCBBF3011485 | 0.008353798 | 19003 | 4 |
| FCBBF3011523 | 0.009297816 | 82316 | 2 |
| FCBBF3011570 | 0.003095496 | 126373 | 1 |
| FCBBF3011592 | 0.006190992 | 49168 | 2 |
| FCBBF3011867 | 0.003095496 | 85770 | 1 |
| FCBBF3011889 | 0.003095496 | 174970 | 1 |
| FCBBF3012170 | 0.003095496 | 158268 | 1 |
| FCBBF3012182 | 0.636006888 | 179857 | 2 |
| FCBBF3012288 | 0.004190774 | 226685 | 2 |
| FCBBF3012332 | 0.007866539 | 137006 | 3 |
| FCBBF3012347 | 0.076340879 | 104645 | 11 |
| FCBBF3012443 | 0.006140378 | 157133 | 2 |
| FCBBF3012546 | 0.067381772 | 51526 | 23 |
| FCBBF3012588 | 0.003095496 | 110940 | 1 |
| FCBBF3012667 | 0.006865285 | 75229 | 3 |
| FCBBF3012842 | 0.003095496 | 158323 | 1 |
| FCBBF3012901 | 0.003095496 | 97274 | 1 |
| FCBBF3013041 | 0.003095496 | 104712 | 1 |
| FCBBF3013058 | 0.003095496 | 237839 | 1 |
| FCBBF3013205 | 0.003095496 | 113182 | 1 |
| FCBBF3013266 | 0.00792573 | 6824 | 4 |
| FCBBF3013307 | 0.003095496 | 119687 | 1 |
| FCBBF3013341 | 0.003095496 | 275987 | 1 |
| FCBBF3013506 | 0.007055566 | 70754 | 4 |
| FCBBF3013589 | 0.02019543 | 57995 | 3 |
| FCBBF3013623 | 0.003095496 | 16629 | 1 |
| FCBBF3013800 | 0.004804956 | 69448 | 2 |
| FCBBF3013846 | 0.003095496 | 175736 | 1 |
| FCBBF3014229 | 0.003095496 | 190802 | 1 |
| FCBBF3014260 | 0.074209592 | 112722 | 23 |
| FCBBF3014355 | 0.003095496 | 1229 | 1 |
| FCBBF3014567 | 0.003095496 | 139758 | 1 |
| FCBBF3014770 | 0.017558968 | 120640 | 4 |
| FCBBF3015100 | 0.033580771 | 117707 | 16 |
| FCBBF3015119 | 0.003095496 | 217028 | 1 |
| FCBBF3015131 | 0.003095496 | 126166 | 1 |
| FCBBF3015317 | 0.003095496 | 222451 | 1 |
| FCBBF3015396 | 0.079742189 | 90872 | 13 |
| FCBBF3015727 | 0.004190774 | 175082 | 2 |
| FCBBF3015758 | 0.003095496 | 113156 | 1 |
| FCBBF3015809 | 0.003095496 | 124899 | 1 |
| FCBBF3015870 | 0.003095496 | 192464 | 1 |
| FCBBF3016018 | 0.003095496 | 237852 | 1 |
| FCBBF3016134 | 0.016096048 | 104871 | 4 |
| FCBBF3016178 | 0.003095496 | 239329 | 1 |
| FCBBF3016451 | 0.007133779 | 144397 | 2 |
| FCBBF3016618 | 0.003095496 | 120202 | 1 |
| FCBBF3016622 | 0.004771043 | 30459 | 2 |
| FCBBF3016644 | 0.003095496 | 192744 | 1 |
| FCBBF3016667 | 0.013088788 | 98301 | 4 |
| FCBBF3016928 | 0.191224014 | 78908 | 32 |
| FCBBF3016987 | 0.003095496 | 188489 | 1 |
| FCBBF3017024 | 0.032691387 | 91639 | 9 |
| FCBBF3017059 | 0.003095496 | 163553 | 1 |
| FCBBF3017071 | 0.003095496 | 1166 | 1 |
| FCBBF3017123 | 0.003095496 | 27872 | 1 |
| FCBBF3017233 | 0.003095496 | 100605 | 1 |
| FCBBF3017255 | 0.004804956 | 254544 | 2 |
| FCBBF3017288 | 0.005177008 | 105123 | 2 |
| FCBBF3017396 | 0.082074724 | 123824 | 20 |
| FCBBF3017531 | 0.104503607 | 80975 | 13 |
| FCBBF3017535 | 0.003095496 | 245940 | 1 |
| FCBBF3017681 | 0.003095496 | 278172 | 1 |
| FCBBF3017729 | 0.003095496 | 224411 | 1 |
| FCBBF3017873 | 0.003095496 | 142688 | 1 |
| FCBBF3017918 | 0.02559117 | 50260 | 11 |
| FCBBF3017974 | 0.003095496 | 180463 | 1 |
| FCBBF3018067 | 0.009137062 | 199895 | 2 |
| FCBBF3018173 | 0.004804956 | 102819 | 2 |
| FCBBF3018453 | 0.036272915 | 11172 | 12 |
| FCBBF3018591 | 0.003095496 | 87725 | 1 |
| FCBBF3018753 | 0.007272431 | 78046 | 2 |
| FCBBF3018796 | 0.05397134 | 134299 | 13 |
| FCBBF3018826 | 0.017175926 | 103781 | 5 |
| FCBBF3018949 | 0.011840079 | 142681 | 5 |
| FCBBF3019085 | 0.003095496 | 116314 | 1 |
| FCBBF3019320 | 0.008476207 | 53626 | 5 |
| FCBBF3019437 | 0.005177008 | 163552 | 2 |
| FCBBF3019455 | 0.020573785 | 162262 | 6 |
| FCBBF3019564 | 0.003095496 | 107210 | 1 |
| FCBBF3019569 | 0.004284741 | 180656 | 2 |
| FCBBF3019570 | 0.003095496 | 202160 | 1 |
| FCBBF3019663 | 0.003095496 | 189005 | 1 |
| FCBBF3019714 | 0.081803803 | 3716 | 8 |
| FCBBF3019716 | 0.003095496 | 154090 | 1 |
| FCBBF3019784 | 0.006140378 | 21322 | 2 |
| FCBBF3019839 | 0.044741637 | 132857 | 21 |
| FCBBF3019867 | 0.003095496 | 17746 | 1 |
| FCBBF3019961 | 0.003095496 | 249984 | 1 |
| FCBBF3020030 | 0.003095496 | 285116 | 1 |
| FCBBF3020138 | 0.003095496 | 126595 | 1 |
| FCBBF3020140 | 0.005889807 | 108327 | 3 |
| FCBBF3020163 | 0.003095496 | 201387 | 1 |
| FCBBF3020232 | 0.003095496 | 148608 | 1 |
| FCBBF3020599 | 0.006010353 | 247156 | 2 |
| FCBBF3021026 | 0.00693958 | 165540 | 2 |
| FCBBF3021191 | 0.00903762 | 57594 | 2 |
| FCBBF3021221 | 0.003095496 | 123739 | 1 |
| FCBBF3021501 | 0.003095496 | 83948 | 1 |
| FCBBF3021506 | 0.003095496 | 121039 | 1 |
| FCBBF3021524 | 0.003095496 | 157510 | 1 |
| FCBBF3021576 | 0.019495442 | 57915 | 5 |
| FCBBF3021807 | 0.038644985 | 161093 | 7 |
| FCBBF3021940 | 0.006709388 | 190587 | 2 |
| FCBBF3021985 | 0.003095496 | 187397 | 1 |
| FCBBF3022005 | 0.003095496 | 67389 | 1 |
| FCBBF3022291 | 0.004284741 | 132527 | 2 |
| FCBBF3022311 | 0.007380237 | 147872 | 3 |
| FCBBF3022321 | 0.022327869 | 111563 | 8 |
| FCBBF3022504 | 0.010751098 | 134416 | 4 |
| FCBBF3022566 | 0.004771043 | 162304 | 2 |
| FCBBF3022593 | 0.02418371 | 35373 | 7 |
| FCBBF3022765 | 0.004783773 | 283481 | 2 |
| FCBBF3022767 | 0.008227607 | 152022 | 4 |
| FCBBF3022894 | 0.037987331 | 121092 | 2 |
| FCBBF3023061 | 0.003095496 | 279182 | 1 |
| FCBBF3023078 | 0.039131532 | 193305 | 2 |
| FCBBF3023368 | 0.068291868 | 72083 | 14 |
| FCBBF3023372 | 0.003095496 | 266581 | 1 |
| FCBBF3023443 | 0.003095496 | 243362 | 1 |
| FCBBF3023599 | 0.003095496 | 185654 | 1 |
| FCBBF3023667 | 1.26175789 | 6796 | 104 |
| FCBBF3023704 | 0.003095496 | 173913 | 1 |
| FCBBF3023887 | 0.017679991 | 35952 | 7 |
| FCBBF3023895 | 0.003095496 | 255290 | 1 |
| FCBBF3024002 | 0.029523914 | 28589 | 16 |
| FCBBF3024096 | 0.003095496 | 93587 | 1 |
| FCBBF3024225 | 0.003095496 | 149394 | 1 |
| FCBBF3024266 | 0.017129488 | 3024 | 7 |
| FCBBF3024364 | 0.003095496 | 98834 | 1 |
| FCBBF3024623 | 0.059813931 | 45257 | 18 |
| FCBBF3024663 | 0.022806825 | 83294 | 11 |
| FCBBF3024793 | 0.01048706 | 40320 | 3 |
| FCBBF3024911 | 0.003095496 | 246119 | 1 |
| FCBBF3024935 | 0.105889362 | 102938 | 37 |
| FCBBF3025073 | 0.023163784 | 148824 | 8 |
| FCBBF3025098 | 0.003095496 | 1019 | 1 |
| FCBBF3025142 | 0.034580253 | 128442 | 15 |
| FCBBF3025252 | 0.003095496 | 50012 | 1 |
| FCBBF3025280 | 0.003095496 | 452 | 1 |
| FCBBF3025285 | 0.00693958 | 69856 | 2 |
| FCBBF3025417 | 0.019520132 | 119643 | 7 |
| FCBBF3025528 | 0.034705132 | 17097 | 7 |
| FCBBF3025568 | 0.010188365 | 82534 | 5 |
| FCBBF3025650 | 0.006663071 | 161974 | 3 |
| FCBBF3025674 | 0.003095496 | 122763 | 1 |
| FCBBF3025730 | 0.008462843 | 179377 | 5 |
| FCBBF3025737 | 0.051555398 | 106162 | 15 |
| FCBBF3025905 | 0.010474608 | 128816 | 6 |
| FCBBF3026021 | 0.003095496 | 147857 | 1 |
| FCBBF3026048 | 0.005177008 | 148775 | 2 |
| FCBBF3026236 | 0.01851457 | 94284 | 9 |
| FCBBF3026251 | 0.003095496 | 160066 | 1 |
| FCBBF3026308 | 0.013109707 | 18907 | 5 |
| FCBBF3026651 | 0.022961796 | 109189 | 14 |
| FCBBF3026678 | 0.003095496 | 223298 | 1 |
| FCBBF3026692 | 0.010326307 | 27253 | 3 |
| FCBBF3027104 | 0.003095496 | 83160 | 1 |
| FCBBF3027199 | 0.003095496 | 151981 | 1 |
| FCBBF3027328 | 0.003095496 | 141045 | 1 |
| FCBBF3027559 | 0.004771043 | 75587 | 2 |
| FCBBF3027717 | 0.003095496 | 165913 | 1 |
| FCBBF3027755 | 0.003095496 | 1220 | 1 |
| FCBBF3027768 | 0.003095496 | 91135 | 1 |
| FCBBF3027854 | 0.02140721 | 223399 | 4 |
| FCBBF3027863 | 0.003095496 | 177020 | 1 |
| FCBBF3027903 | 0.006190992 | 159232 | 2 |
| FCBBF3028102 | 0.003095496 | 265666 | 1 |
| FCBBF3028143 | 0.003095496 | 141680 | 1 |
| FCBBF3028188 | 0.003095496 | 158195 | 1 |
| FCBBF3028202 | 0.014583868 | 162303 | 5 |
| FCBBF3028244 | 0.180469591 | 11446 | 57 |
| FCBBF3028472 | 0.003095496 | 210384 | 1 |
| FCBBF3028528 | 0.003095496 | 121994 | 1 |
| FCBBF3028582 | 0.003095496 | 19310 | 1 |
| FCBBF3028593 | 0.041498893 | 78176 | 16 |
| FCBBF3028671 | 0.003095496 | 128279 | 1 |
| FCBBF3028794 | 0.003095496 | 161858 | 1 |
| FCBBF4000017 | 0.018864645 | 207011 | 5 |
| FCBBF4000061 | 0.003095496 | 85356 | 1 |
| FCBBF4000076 | 0.003095496 | 151467 | 1 |
| FCBBF4000142 | 0.045830797 | 66559 | 22 |
| FCBBF4000173 | 0.003095496 | 201938 | 1 |
| FCBBF4000192 | 0.003095496 | 260340 | 1 |
| FCBBF4000193 | 0.005177008 | 102821 | 2 |
| FCBBF4000268 | 0.004804956 | 148806 | 2 |
| FCBBF4000282 | 0.003095496 | 216578 | 1 |
| FCBBF4000399 | 0.003095496 | 226291 | 1 |
| FCBBF4000415 | 0.003095496 | 96302 | 1 |
| FCBBF4000446 | 0.223074046 | 129912 | 60 |
| FCBBF4000500 | 0.019117956 | 178642 | 4 |
| FCBBF4000529 | 0.003095496 | 264684 | 1 |
| FCBBF4000548 | 0.011463751 | 168269 | 5 |
| FCBBF5000041 | 0.003095496 | 84551 | 1 |
| FCBBF5000061 | 0.007998492 | 106949 | 3 |
| FCBBF5000165 | 0.050902363 | 103810 | 26 |
| FCBBF5000261 | 0.032271118 | 116453 | 10 |
| FCBBF5000325 | 0.016573675 | 157539 | 5 |
| FCBBF5000353 | 0.004783773 | 110781 | 2 |
| FCBBF5000384 | 0.009286488 | 250110 | 3 |
| FCBBF5000483 | 0.003095496 | 250092 | 1 |
| FCBBF5000495 | 0.003095496 | 190473 | 1 |
| FEBRA1000005 | 0.004176935 | 283841 | 1 |
| FEBRA1000008 | 0.007221817 | 51948 | 2 |
| FEBRA1000022 | 0.031835934 | 51520 | 10 |
| FEBRA1000030 | 0.010249318 | 41539 | 2 |
| FEBRA1000057 | 0.096516255 | 80034 | 46 |
| FEBRA1000088 | 0.039262423 | 76752 | 8 |
| FEBRA1000138 | 0.004176935 | 11952 | 1 |
| FEBRA1000148 | 0.004176935 | 69685 | 1 |
| FEBRA1000149 | 0.004176935 | 41492 | 1 |
| FEBRA1000183 | 0.004176935 | 10974 | 1 |
| FEBRA1000188 | 0.080062953 | 39146 | 30 |
| FEBRA1000189 | 0.008264884 | 73547 | 4 |
| FEBRA1000190 | 0.02108267 | 81248 | 3 |
| FEBRA2000007 | 0.00707564 | 1252 | 3 |
| FEBRA2000021 | 0.004176935 | 272023 | 1 |
| FEBRA2000035 | 0.004176935 | 165814 | 1 |
| FEBRA2000053 | 0.02375981 | 88848 | 10 |
| FEBRA2000105 | 0.233617833 | 65409 | 45 |
| FEBRA2000126 | 0.004176935 | 260702 | 1 |
| FEBRA2000129 | 0.064454281 | 29212 | 15 |
| FEBRA2000210 | 0.237030397 | 137341 | 17 |
| FEBRA2000220 | 0.004176935 | 69451 | 1 |
| FEBRA2000228 | 0.042419201 | 96185 | 12 |
| FEBRA2000253 | 0.011446575 | 112721 | 5 |
| FEBRA2000282 | 0.005272213 | 59705 | 2 |
| FEBRA2000286 | 0.004176935 | 151674 | 1 |
| FEBRA2000297 | 0.021128524 | 140830 | 6 |
| FEBRA2000311 | 0.016736159 | 79540 | 3 |
| FEBRA2000319 | 0.122339577 | 104010 | 39 |
| FEBRA2000321 | 0.004176935 | 139252 | 1 |
| FEBRA2000330 | 0.017511022 | 146332 | 6 |
| FEBRA2000333 | 0.006035049 | 173257 | 2 |
| FEBRA2000377 | 0.016749426 | 69207 | 7 |
| FEBRA2000378 | 0.004176935 | 174863 | 1 |
| FEBRA2000391 | 0.004176935 | 214772 | 1 |
| FEBRA2000394 | 0.004176935 | 212154 | 1 |
| FEBRA2000397 | 0.014412342 | 65918 | 4 |
| FEBRA2000399 | 0.004176935 | 141078 | 1 |
| FEBRA2000402 | 0.030231398 | 97450 | 7 |
| FEBRA2000404 | 0.016652861 | 10099 | 3 |
| FEBRA2000409 | 0.004176935 | 61638 | 1 |
| FEBRA2000415 | 0.004176935 | 115096 | 1 |
| FEBRA2000436 | 0.004176935 | 223385 | 1 |
| FEBRA2000438 | 0.018964506 | 121726 | 6 |
| FEBRA2000452 | 0.16706337 | 118037 | 51 |
| FEBRA2000454 | 0.00557409 | 18672 | 2 |
| FEBRA2000462 | 0.059357307 | 69079 | 36 |
| FEBRA2000468 | 0.004176935 | 182097 | 1 |
| FEBRA2000491 | 0.004176935 | 207236 | 1 |
| FEBRA2000504 | 0.004176935 | 61545 | 1 |
| FEBRA2000510 | 0.045411177 | 65267 | 10 |
| FEBRA2000532 | 0.052396457 | 151351 | 4 |
| FEBRA2000536 | 0.053194835 | 154038 | 20 |
| FEBRA2000538 | 0.010413293 | 133436 | 2 |
| FEBRA2000575 | 0.004176935 | 172903 | 1 |
| FEBRA2000581 | 0.210626844 | 82854 | 102 |
| FEBRA2000656 | 0.004176935 | 81282 | 1 |
| FEBRA2000659 | 0.004176935 | 62321 | 1 |
| FEBRA2000680 | 0.017827821 | 73496 | 10 |
| FEBRA2000690 | 0.004176935 | 41374 | 1 |
| FEBRA2000727 | 0.004176935 | 73716 | 1 |
| FEBRA2000733 | 0.994262346 | 65778 | 106 |
| FEBRA2000734 | 0.004176935 | 204792 | 1 |
| FEBRA2000740 | 0.148155923 | 126429 | 39 |
| FEBRA2000757 | 0.054716008 | 85619 | 27 |
| FEBRA2000762 | 0.026935129 | 103207 | 5 |
| FEBRA2000771 | 0.007723326 | 204285 | 3 |
| FEBRA2000772 | 0.019587871 | 192434 | 4 |
| FEBRA2000782 | 0.006555424 | 126095 | 3 |
| FEBRA2000787 | 0.084829388 | 125809 | 20 |
| FEBRA2000790 | 0.004176935 | 54713 | 1 |
| FEBRA2000793 | 0.043693537 | 146325 | 15 |
| FEBRA2000803 | 0.004176935 | 185525 | 1 |
| FEBRA2000805 | 0.004176935 | 122630 | 1 |
| FEBRA2000809 | 0.004176935 | 180145 | 1 |
| FEBRA2000815 | 0.046348201 | 65761 | 15 |
| FEBRA2000856 | 0.010556449 | 9963 | 6 |
| FEBRA2000862 | 0.004176935 | 101614 | 1 |
| FEBRA2000874 | 0.045830797 | 66559 | 22 |
| FEBRA2000877 | 0.011777362 | 100172 | 6 |
| FEBRA2000880 | 0.015811798 | 119896 | 6 |
| FEBRA2000881 | 0.052934384 | 156622 | 13 |
| FEBRA2000901 | 0.016130763 | 106445 | 3 |
| FEBRA2000909 | 0.004176935 | 223842 | 1 |
| FEBRA2000917 | 0.004176935 | 212227 | 1 |
| FEBRA2000928 | 0.004176935 | 117080 | 1 |
| FEBRA2000936 | 0.004176935 | 192445 | 1 |
| FEBRA2000937 | 0.004176935 | 181738 | 1 |
| FEBRA2000959 | 0.004176935 | 177077 | 1 |
| FEBRA2000972 | 0.011061583 | 118979 | 4 |
| FEBRA2001004 | 0.007574672 | 73453 | 3 |
| FEBRA2001012 | 0.004176935 | 471 | 1 |
| FEBRA2001020 | 0.004176935 | 214167 | 1 |
| FEBRA2001093 | 0.004176935 | 118043 | 1 |
| FEBRA2001124 | 0.004176935 | 32473 | 1 |
| FEBRA2001133 | 0.005272213 | 34757 | 2 |
| FEBRA2001134 | 0.212174642 | 84844 | 73 |
| FEBRA2001146 | 0.014983635 | 66459 | 8 |
| FEBRA2001161 | 0.004176935 | 214147 | 1 |
| FEBRA2001175 | 0.004176935 | 249403 | 1 |
| FEBRA2001197 | 0.01188635 | 165277 | 5 |
| FEBRA2001217 | 0.004176935 | 96908 | 1 |
| FEBRA2001227 | 0.045319674 | 88377 | 15 |
| FEBRA2001245 | 0.066428826 | 75508 | 28 |
| FEBRA2001267 | 0.004176935 | 41876 | 1 |
| FEBRA2001294 | 0.069335056 | 108604 | 12 |
| FEBRA2001334 | 0.029136712 | 58548 | 13 |
| FEBRA2001351 | 0.004176935 | 277232 | 1 |
| FEBRA2001383 | 0.016062214 | 141059 | 5 |
| FEBRA2001438 | 0.004176935 | 192423 | 1 |
| FEBRA2001440 | 0.014395436 | 96434 | 3 |
| FEBRA2001487 | 0.004176935 | 223847 | 1 |
| FEBRA2001492 | 0.051769295 | 91638 | 26 |
| FEBRA2001523 | 0.01970648 | 186423 | 4 |
| FEBRA2001529 | 0.049104411 | 110021 | 25 |
| FEBRA2001561 | 0.004176935 | 214799 | 1 |
| FEBRA2001571 | 0.079898853 | 12275 | 26 |
| FEBRA2001581 | 0.040864724 | 22680 | 11 |
| FEBRA2001584 | 0.004176935 | 149220 | 1 |
| FEBRA2001590 | 0.004176935 | 223518 | 1 |
| FEBRA2001591 | 0.016402976 | 148591 | 5 |
| FEBRA2001593 | 0.106885695 | 21293 | 46 |
| FEBRA2001610 | 0.004176935 | 77006 | 1 |
| FEBRA2001620 | 0.004176935 | 146214 | 1 |
| FEBRA2001659 | 0.004176935 | 35993 | 1 |
| FEBRA2001669 | 0.156407021 | 83699 | 77 |
| FEBRA2001698 | 0.013764262 | 145370 | 4 |
| FEBRA2001705 | 0.004176935 | 21251 | 1 |
| FEBRA2001706 | 0.005852482 | 139638 | 2 |
| FEBRA2001715 | 0.168037623 | 64622 | 68 |
| FEBRA2001745 | 0.133701175 | 71102 | 22 |
| FEBRA2001752 | 0.010638393 | 90140 | 4 |
| FEBRA2001772 | 0.004176935 | 242141 | 1 |
| FEBRA2001790 | 0.004176935 | 157502 | 1 |
| FEBRA2001814 | 0.12664823 | 95070 | 44 |
| FEBRA2001828 | 0.030797704 | 151754 | 10 |
| FEBRA2001867 | 0.045209688 | 119453 | 14 |
| FEBRA2001914 | 0.004176935 | 151811 | 1 |
| FEBRA2001974 | 0.014380448 | 108212 | 6 |
| FEBRA2001989 | 0.00557409 | 137054 | 2 |
| FEBRA2001990 | 0.046173839 | 118218 | 22 |
| FEBRA2002009 | 0.004176935 | 267335 | 1 |
| FEBRA2002027 | 0.066135695 | 58584 | 21 |
| FEBRA2002086 | 0.004176935 | 172253 | 1 |
| FEBRA2002109 | 0.004176935 | 280861 | 1 |
| FEBRA2002191 | 0.028522523 | 139468 | 4 |
| FEBRA2002194 | 0.138211456 | 118822 | 32 |
| FEBRA2002260 | 0.004176935 | 242171 | 1 |
| FEBRA2002352 | 0.048268514 | 117002 | 14 |
| FEBRA2002399 | 0.004176935 | 136412 | 1 |
| FEBRA2002410 | 0.006258447 | 185295 | 2 |
| FEBRA2002429 | 0.042790317 | 218135 | 3 |
| FEBRA2002442 | 0.008021019 | 177512 | 2 |
| FEBRA2002456 | 0.153620295 | 72543 | 66 |
| FEBRA2002508 | 0.004176935 | 64653 | 1 |
| FEBRA2002525 | 0.006258447 | 85737 | 2 |
| FEBRA2002527 | 0.058506177 | 111123 | 9 |
| FEBRA2002552 | 0.004176935 | 88046 | 1 |
| FEBRA2002574 | 0.00557409 | 103876 | 2 |
| FEBRA2002601 | 0.015696345 | 5562 | 2 |
| FEBRA2002611 | 0.007710596 | 92071 | 3 |
| FEBRA2002628 | 0.004176935 | 148620 | 1 |
| FEBRA2002662 | 0.004176935 | 238882 | 1 |
| FEBRA2002682 | 0.006258447 | 110880 | 2 |
| FEBRA2002707 | 0.046501045 | 27368 | 8 |
| FEBRA2002727 | 0.004176935 | 175605 | 1 |
| FEBRA2002781 | 0.024488495 | 93575 | 15 |
| FEBRA2002783 | 0.004176935 | 173916 | 1 |
| FEBRA2002792 | 0.125180907 | 83171 | 50 |
| FEBRA2002818 | 0.004176935 | 269467 | 1 |
| FEBRA2002858 | 0.004176935 | 264199 | 1 |
| FEBRA2002882 | 0.004176935 | 254902 | 1 |
| FEBRA2002897 | 0.004176935 | 212093 | 1 |
| FEBRA2002908 | 0.004176935 | 163856 | 1 |
| FEBRA2002931 | 0.153107618 | 104282 | 74 |
| FEBRA2002933 | 0.452774471 | 47338 | 38 |
| FEBRA2002962 | 0.004176935 | 83476 | 1 |
| FEBRA2002986 | 0.004176935 | 240045 | 1 |
| FEBRA2003054 | 0.004176935 | 62330 | 1 |
| FEBRA2003100 | 0.254417087 | 88710 | 39 |
| FEBRA2003115 | 0.007729324 | 75428 | 3 |
| FEBRA2003128 | 0.057602096 | 35368 | 29 |
| FEBRA2003155 | 0.004176935 | 235304 | 1 |
| FEBRA2003163 | 0.00835387 | 226188 | 2 |
| FEBRA2003181 | 0.00557409 | 259109 | 2 |
| FEBRA2003198 | 0.006258447 | 176644 | 2 |
| FEBRA2003253 | 0.004176935 | 94104 | 1 |
| FEBRA2003258 | 0.059223674 | 129836 | 13 |
| FEBRA2003275 | 0.004176935 | 261389 | 1 |
| FEBRA2003308 | 0.004176935 | 254043 | 1 |
| FEBRA2003327 | 0.004176935 | 126193 | 1 |
| FEBRA2003429 | 0.004176935 | 45084 | 1 |
| FEBRA2003436 | 0.030140426 | 59024 | 8 |
| FEBRA2003454 | 0.004176935 | 143803 | 1 |
| FEBRA2003468 | 0.00835387 | 213833 | 2 |
| FEBRA2003470 | 0.011480385 | 129650 | 5 |
| FEBRA2003520 | 0.004176935 | 42392 | 1 |
| FEBRA2003524 | 0.016398886 | 105446 | 2 |
| FEBRA2003541 | 0.109806822 | 105115 | 27 |
| FEBRA2003675 | 0.004176935 | 84330 | 1 |
| FEBRA2003707 | 0.008021019 | 175985 | 2 |
| FEBRA2003711 | 0.004176935 | 222118 | 1 |
| FEBRA2003726 | 0.168260867 | 52967 | 9 |
| FEBRA2003733 | 0.119522789 | 61527 | 44 |
| FEBRA2003750 | 0.091784057 | 141213 | 31 |
| FEBRA2003822 | 0.030464585 | 161581 | 3 |
| FEBRA2003833 | 0.096900985 | 70230 | 41 |
| FEBRA2003897 | 0.004176935 | 181699 | 1 |
| FEBRA2003907 | 0.005366179 | 174415 | 2 |
| FEBRA2003926 | 0.24002786 | 128692 | 35 |
| FEBRA2003930 | 0.093229135 | 120142 | 14 |
| FEBRA2004023 | 0.005272213 | 158238 | 2 |
| FEBRA2004026 | 0.004176935 | 74912 | 1 |
| FEBRA2004029 | 0.004176935 | 42428 | 1 |
| FEBRA2004042 | 0.078988859 | 44190 | 16 |
| FEBRA2004053 | 0.004176935 | 118450 | 1 |
| FEBRA2004056 | 0.004176935 | 139290 | 1 |
| FEBRA2004091 | 0.015855022 | 79489 | 6 |
| FEBRA2004099 | 0.066903155 | 139726 | 18 |
| FEBRA2004110 | 0.006258447 | 132401 | 2 |
| FEBRA2004191 | 0.008449003 | 190594 | 4 |
| FEBRA2004202 | 0.004176935 | 250783 | 1 |
| FEBRA2004219 | 0.005886395 | 128740 | 2 |
| FEBRA2004224 | 0.14472965 | 126539 | 32 |
| FEBRA2004237 | 0.008933913 | 202823 | 5 |
| FEBRA2004268 | 0.009543114 | 209948 | 3 |
| FEBRA2004293 | 0.063029631 | 75155 | 21 |
| FEBRA2004325 | 0.047571095 | 89569 | 10 |
| FEBRA2004329 | 0.004176935 | 244185 | 1 |
| FEBRA2004331 | 0.00557409 | 159910 | 2 |
| FEBRA2004412 | 0.057069716 | 154207 | 16 |
| FEBRA2004443 | 0.004176935 | 250767 | 1 |
| FEBRA2004485 | 0.024143038 | 164816 | 7 |
| FEBRA2004490 | 0.011887531 | 112414 | 4 |
| FEBRA2004538 | 0.004176935 | 152600 | 1 |
| FEBRA2004592 | 0.010413293 | 81949 | 2 |
| FEBRA2004620 | 0.017098501 | 91748 | 2 |
| FEBRA2004628 | 0.058739771 | 122287 | 14 |
| FEBRA2004651 | 0.004176935 | 216779 | 1 |
| FEBRA2004767 | 0.006555424 | 109708 | 3 |
| FEBRA2004813 | 0.004176935 | 246503 | 1 |
| FEBRA2004818 | 0.123048402 | 93677 | 31 |
| FEBRA2004840 | 0.004176935 | 242895 | 1 |
| FEBRA2004852 | 0.006175336 | 251770 | 2 |
| FEBRA2004867 | 0.004176935 | 161383 | 1 |
| FEBRA2004914 | 0.004176935 | 127069 | 1 |
| FEBRA2004933 | 0.004176935 | 213438 | 1 |
| FEBRA2004987 | 0.004176935 | 222662 | 1 |
| FEBRA2005014 | 0.004176935 | 57040 | 1 |
| FEBRA2005036 | 0.004176935 | 223872 | 1 |
| FEBRA2005079 | 0.010042147 | 94042 | 3 |
| FEBRA2005114 | 0.024970504 | 42183 | 7 |
| FEBRA2005216 | 0.004176935 | 272863 | 1 |
| FEBRA2005291 | 0.046202668 | 100772 | 3 |
| FEBRA2005361 | 0.23923172 | 104456 | 77 |
| FEBRA2005377 | 0.004176935 | 254136 | 1 |
| FEBRA2005380 | 0.004176935 | 255991 | 1 |
| FEBRA2005416 | 0.156027624 | 123623 | 20 |
| FEBRA2005427 | 0.005852482 | 208219 | 2 |
| FEBRA2005458 | 0.004176935 | 182098 | 1 |
| FEBRA2005476 | 0.064330012 | 161302 | 10 |
| FEBRA2005572 | 0.094039945 | 100147 | 23 |
| FEBRA2005701 | 0.004176935 | 262739 | 1 |
| FEBRA2005726 | 0.004176935 | 109196 | 1 |
| FEBRA2005734 | 0.198445894 | 40243 | 19 |
| FEBRA2005752 | 0.040255197 | 143138 | 9 |
| FEBRA2005778 | 0.055859713 | 156866 | 12 |
| FEBRA2005787 | 0.00557409 | 170802 | 2 |
| FEBRA2005788 | 0.033949775 | 120367 | 11 |
| FEBRA2005824 | 0.449223397 | 100968 | 136 |
| FEBRA2005995 | 0.119522789 | 61527 | 44 |
| FEBRA2005998 | 0.004176935 | 60173 | 1 |
| FEBRA2006055 | 0.063434366 | 179859 | 6 |
| FEBRA2006061 | 0.004176935 | 56171 | 1 |
| FEBRA2006092 | 0.269467869 | 37856 | 45 |
| FEBRA2006112 | 0.025385361 | 34360 | 13 |
| FEBRA2006150 | 0.004176935 | 270568 | 1 |
| FEBRA2006165 | 0.004176935 | 81741 | 1 |
| FEBRA2006255 | 0.128587489 | 91402 | 52 |
| FEBRA2006270 | 0.14333092 | 114889 | 24 |
| FEBRA2006315 | 0.004176935 | 266752 | 1 |
| FEBRA2006346 | 0.011278791 | 80941 | 3 |
| FEBRA2006354 | 0.004176935 | 167128 | 1 |
| FEBRA2006357 | 0.252967967 | 78596 | 135 |
| FEBRA2006368 | 0.053922974 | 99624 | 20 |
| FEBRA2006370 | 0.011727858 | 41237 | 3 |
| FEBRA2006372 | 0.124762606 | 18616 | 22 |
| FEBRA2006379 | 0.004176935 | 196938 | 1 |
| FEBRA2006396 | 0.020846589 | 175672 | 8 |
| FEBRA2006401 | 0.667575146 | 98766 | 29 |
| FEBRA2006409 | 0.004176935 | 202608 | 1 |
| FEBRA2006418 | 0.004176935 | 29177 | 1 |
| FEBRA2006427 | 0.004176935 | 181899 | 1 |
| FEBRA2006476 | 0.004176935 | 164491 | 1 |
| FEBRA2006485 | 0.055081272 | 130929 | 13 |
| FEBRA2006491 | 0.011283258 | 91401 | 5 |
| FEBRA2006519 | 0.014090451 | 81576 | 7 |
| FEBRA2006521 | 0.02190798 | 138761 | 10 |
| FEBRA2006553 | 0.042352188 | 117092 | 10 |
| FEBRA2006627 | 0.016240411 | 111564 | 6 |
| FEBRA2006664 | 0.005272213 | 18921 | 2 |
| FEBRA2006667 | 0.004176935 | 103319 | 1 |
| FEBRA2006726 | 0.004176935 | 274740 | 1 |
| FEBRA2006736 | 0.011278791 | 148018 | 3 |
| FEBRA2006793 | 0.004176935 | 196322 | 1 |
| FEBRA2006808 | 0.040818316 | 7695 | 12 |
| FEBRA2006817 | 0.010218501 | 110819 | 2 |
| FEBRA2006873 | 0.037313918 | 69447 | 8 |
| FEBRA2006890 | 0.007528029 | 152681 | 3 |
| FEBRA2006942 | 0.004176935 | 55987 | 1 |
| FEBRA2006977 | 0.004176935 | 213028 | 1 |
| FEBRA2007017 | 0.010231303 | 114664 | 2 |
| FEBRA2007176 | 0.005852482 | 161700 | 2 |
| FEBRA2007200 | 0.005272213 | 211510 | 2 |
| FEBRA2007212 | 0.042684601 | 94310 | 17 |
| FEBRA2007247 | 0.014051528 | 74992 | 2 |
| FEBRA2007280 | 0.004176935 | 177111 | 1 |
| FEBRA2007414 | 0.004176935 | 236821 | 1 |
| FEBRA2007436 | 0.004176935 | 64921 | 1 |
| FEBRA2007449 | 0.004176935 | 212459 | 1 |
| FEBRA2007458 | 0.020552719 | 126626 | 6 |
| FEBRA2007534 | 0.009284132 | 60262 | 4 |
| FEBRA2007544 | 0.068440576 | 18116 | 42 |
| FEBRA2007551 | 0.006035049 | 50243 | 2 |
| FEBRA2007566 | 0.004176935 | 229682 | 1 |
| FEBRA2007589 | 0.024506169 | 132139 | 14 |
| FEBRA2007613 | 0.050493173 | 87471 | 17 |
| FEBRA2007620 | 0.005272213 | 274280 | 2 |
| FEBRA2007622 | 0.162411089 | 113012 | 26 |
| FEBRA2007708 | 0.019604797 | 115093 | 15 |
| FEBRA2007714 | 0.020081166 | 141389 | 9 |
| FEBRA2007793 | 0.008339959 | 148844 | 3 |
| FEBRA2007801 | 0.067873593 | 40240 | 21 |
| FEBRA2007818 | 0.029964139 | 103573 | 5 |
| FEBRA2007823 | 0.005852482 | 163986 | 2 |
| FEBRA2007880 | 0.044518678 | 102314 | 15 |
| FEBRA2007900 | 0.02161745 | 108630 | 10 |
| FEBRA2007901 | 0.045209184 | 84151 | 11 |
| FEBRA2007931 | 0.004176935 | 131059 | 1 |
| FEBRA2007957 | 0.009203576 | 126442 | 4 |
| FEBRA2008009 | 0.004176935 | 223799 | 1 |
| FEBRA2008036 | 0.117309152 | 99020 | 46 |
| FEBRA2008081 | 0.23661529 | 56755 | 57 |
| FEBRA2008086 | 0.006035049 | 42736 | 2 |
| FEBRA2008087 | 0.934772397 | 43032 | 73 |
| FEBRA2008134 | 0.005865212 | 157061 | 2 |
| FEBRA2008135 | 0.004176935 | 212501 | 1 |
| FEBRA2008159 | 0.005366179 | 35865 | 2 |
| FEBRA2008165 | 0.004176935 | 163062 | 1 |
| FEBRA2008172 | 0.004176935 | 158014 | 1 |
| FEBRA2008201 | 0.069870416 | 108703 | 12 |
| FEBRA2008210 | 0.024981157 | 167548 | 8 |
| FEBRA2008255 | 0.033112123 | 89656 | 10 |
| FEBRA2008265 | 0.010231303 | 236535 | 2 |
| FEBRA2008266 | 0.018439689 | 138368 | 4 |
| FEBRA2008282 | 0.004176935 | 54789 | 1 |
| FEBRA2008287 | 0.056845056 | 24108 | 10 |
| FEBRA2008302 | 0.157446218 | 2665 | 33 |
| FEBRA2008311 | 0.004176935 | 53554 | 1 |
| FEBRA2008341 | 0.025507452 | 91475 | 6 |
| FEBRA2008360 | 0.016389065 | 111079 | 6 |
| FEBRA2008459 | 0.005865212 | 156347 | 2 |
| FEBRA2008468 | 0.004176935 | 1224 | 1 |
| FEBRA2008475 | 0.019858702 | 128443 | 3 |
| FEBRA2008481 | 0.004176935 | 164501 | 1 |
| FEBRA2008542 | 0.004176935 | 230774 | 1 |
| FEBRA2008583 | 1.267697555 | 27207 | 213 |
| FEBRA2008660 | 0.004176935 | 278426 | 1 |
| FEBRA2008662 | 0.105565309 | 110213 | 33 |
| FEBRA2008681 | 0.004176935 | 264225 | 1 |
| FEBRA2008692 | 0.068291938 | 134261 | 15 |
| FEBRA2008741 | 0.004176935 | 231739 | 1 |
| FEBRA2008755 | 0.102006309 | 66392 | 9 |
| FEBRA2008836 | 0.005272213 | 19098 | 2 |
| FEBRA2008859 | 0.005366179 | 253884 | 2 |
| FEBRA2008861 | 0.086031312 | 95688 | 37 |
| FEBRA2008866 | 0.004176935 | 226965 | 1 |
| FEBRA2008881 | 0.004176935 | 256972 | 1 |
| FEBRA2008928 | 0.005366179 | 63023 | 2 |
| FEBRA2008983 | 0.004176935 | 241847 | 1 |
| FEBRA2009016 | 0.004176935 | 111575 | 1 |
| FEBRA2009022 | 0.050172916 | 76242 | 12 |
| FEBRA2009029 | 0.004176935 | 196323 | 1 |
| FEBRA2009074 | 0.004176935 | 145066 | 1 |
| FEBRA2009117 | 0.004176935 | 210754 | 1 |
| FEBRA2009120 | 0.004176935 | 186096 | 1 |
| FEBRA2009162 | 0.004176935 | 110029 | 1 |
| FEBRA2009185 | 0.184420408 | 89752 | 80 |
| FEBRA2009240 | 0.004176935 | 238850 | 1 |
| FEBRA2009265 | 0.004176935 | 112418 | 1 |
| FEBRA2009276 | 0.007158894 | 96904 | 2 |
| FEBRA2009289 | 0.011940763 | 139044 | 3 |
| FEBRA2009327 | 0.004176935 | 222085 | 1 |
| FEBRA2009328 | 0.033813702 | 107117 | 6 |
| FEBRA2009352 | 0.005886395 | 216904 | 2 |
| FEBRA2009362 | 0.022006758 | 198442 | 5 |
| FEBRA2009385 | 0.004176935 | 173199 | 1 |
| FEBRA2009416 | 0.004176935 | 78048 | 1 |
| FEBRA2009419 | 0.126812117 | 121445 | 44 |
| FEBRA2009437 | 0.045982955 | 105449 | 2 |
| FEBRA2009478 | 0.004176935 | 192483 | 1 |
| FEBRA2009507 | 0.004176935 | 274534 | 1 |
| FEBRA2009514 | 0.050584741 | 114032 | 12 |
| FEBRA2009541 | 0.010073751 | 110374 | 3 |
| FEBRA2009567 | 0.004176935 | 101574 | 1 |
| FEBRA2009588 | 0.034580253 | 128442 | 15 |
| FEBRA2009731 | 0.004176935 | 32693 | 1 |
| FEBRA2009754 | 0.004176935 | 21279 | 1 |
| FEBRA2009780 | 0.006053777 | 66242 | 2 |
| FEBRA2009804 | 0.033048593 | 122269 | 11 |
| FEBRA2009846 | 0.008116561 | 23934 | 3 |
| FEBRA2009986 | 0.026068598 | 143534 | 13 |
| FEBRA2010141 | 0.004176935 | 179626 | 1 |
| FEBRA2010299 | 0.004176935 | 100899 | 1 |
| FEBRA2010484 | 0.004176935 | 216383 | 1 |
| FEBRA2010719 | 0.009656184 | 189228 | 4 |
| FEBRA2010746 | 0.019530799 | 109453 | 9 |
| FEBRA2010768 | 0.010218501 | 82636 | 2 |
| FEBRA2010802 | 0.004176935 | 263426 | 1 |
| FEBRA2010858 | 0.038439333 | 112371 | 8 |
| FEBRA2011063 | 0.004176935 | 158095 | 1 |
| FEBRA2011090 | 0.009654004 | 53618 | 4 |
| FEBRA2011356 | 0.004176935 | 66635 | 1 |
| FEBRA2011392 | 0.012350672 | 179765 | 4 |
| FEBRA2011414 | 0.004176935 | 54879 | 1 |
| FEBRA2011593 | 0.004176935 | 41044 | 1 |
| FEBRA2011665 | 0.004176935 | 182229 | 1 |
| FEBRA2012090 | 0.004176935 | 152453 | 1 |
| FEBRA2012120 | 0.010379255 | 75673 | 2 |
| FEBRA2012181 | 0.010148342 | 88797 | 5 |
| FEBRA2012195 | 0.004176935 | 210665 | 1 |
| FEBRA2012397 | 0.007158894 | 66332 | 2 |
| FEBRA2012418 | 0.004176935 | 117254 | 1 |
| FEBRA2012507 | 0.022453254 | 197553 | 3 |
| FEBRA2012625 | 0.004176935 | 219988 | 1 |
| FEBRA2013019 | 0.007272431 | 207383 | 2 |
| FEBRA2013069 | 0.004176935 | 276428 | 1 |
| FEBRA2013274 | 0.004176935 | 100846 | 1 |
| FEBRA2013465 | 0.004176935 | 98695 | 1 |
| FEBRA2013570 | 0.015004133 | 58831 | 2 |
| FEBRA2013699 | 0.023412536 | 106733 | 10 |
| FEBRA2013905 | 0.004176935 | 188051 | 1 |
| FEBRA2013951 | 0.010218501 | 48778 | 2 |
| FEBRA2014010 | 0.010379255 | 52372 | 2 |
| FEBRA2014122 | 0.006258447 | 211231 | 2 |
| FEBRA2014198 | 0.004176935 | 121977 | 1 |
| FEBRA2014213 | 0.042550127 | 198616 | 3 |
| FEBRA2014297 | 0.078398788 | 22094 | 14 |
| FEBRA2014417 | 0.310323648 | 123452 | 71 |
| FEBRA2014443 | 0.004176935 | 181650 | 1 |
| FEBRA2014578 | 0.007655602 | 199666 | 3 |
| FEBRA2014939 | 0.02005848 | 135477 | 9 |
| FEBRA2015042 | 0.162544289 | 118129 | 10 |
| FEBRA2015076 | 0.004176935 | 8597 | 1 |
| FEBRA2015085 | 0.109895262 | 46839 | 43 |
| FEBRA2015175 | 0.004176935 | 153648 | 1 |
| FEBRA2015292 | 0.004176935 | 223223 | 1 |
| FEBRA2015588 | 0.004176935 | 166658 | 1 |
| FEBRA2016112 | 0.004176935 | 108652 | 1 |
| FEBRA2016398 | 0.009696566 | 8638 | 3 |
| FEBRA2016572 | 0.008527483 | 213901 | 4 |
| FEBRA2016583 | 0.005886395 | 180408 | 2 |
| FEBRA2016621 | 0.004176935 | 168142 | 1 |
| FEBRA2016654 | 0.004176935 | 203372 | 1 |
| FEBRA2016739 | 0.004176935 | 17832 | 1 |
| FEBRA2017024 | 0.01736624 | 132873 | 6 |
| FEBRA2017098 | 0.007272431 | 145411 | 2 |
| FEBRA2017138 | 0.004176935 | 162604 | 1 |
| FEBRA2017154 | 0.004176935 | 210959 | 1 |
| FEBRA2017200 | 0.004176935 | 165288 | 1 |
| FEBRA2017223 | 0.004176935 | 134801 | 1 |
| FEBRA2017333 | 0.004176935 | 108289 | 1 |
| FEBRA2017441 | 0.330158685 | 120529 | 61 |
| FEBRA2017502 | 0.004176935 | 219556 | 1 |
| FEBRA2017533 | 0.030714937 | 71298 | 12 |
| FEBRA2017680 | 0.017621075 | 129041 | 5 |
| FEBRA2017736 | 0.004176935 | 190247 | 1 |
| FEBRA2017780 | 0.010367927 | 115664 | 3 |
| FEBRA2017811 | 0.004176935 | 199751 | 1 |
| FEBRA2017850 | 0.009353943 | 48773 | 3 |
| FEBRA2018051 | 0.004176935 | 223975 | 1 |
| FEBRA2018203 | 0.01104222 | 199198 | 4 |
| FEBRA2018407 | 0.010342623 | 181890 | 3 |
| FEBRA2018433 | 0.005366179 | 111538 | 2 |
| FEBRA2018476 | 0.153349683 | 103898 | 23 |
| FEBRA2018746 | 0.022404216 | 117454 | 5 |
| FEBRA2018868 | 0.109806822 | 105115 | 27 |
| FEBRA2019172 | 0.004176935 | 140282 | 1 |
| FEBRA2019242 | 0.020652321 | 110291 | 8 |
| FEBRA2019298 | 0.069220147 | 146588 | 4 |
| FEBRA2019378 | 0.028343935 | 165950 | 7 |
| FEBRA2019389 | 0.010231303 | 235384 | 2 |
| FEBRA2019457 | 0.004176935 | 127687 | 1 |
| FEBRA2019519 | 0.004176935 | 140063 | 1 |
| FEBRA2019582 | 0.004176935 | 152928 | 1 |
| FEBRA2019637 | 0.010249318 | 110294 | 2 |
| FEBRA2019663 | 0.019237825 | 119678 | 4 |
| FEBRA2019690 | 0.010379255 | 214054 | 2 |
| FEBRA2019711 | 0.07923718 | 28801 | 11 |
| FEBRA2019867 | 0.006035049 | 93839 | 2 |
| FEBRA2020400 | 0.045223526 | 103242 | 18 |
| FEBRA2020406 | 0.007272431 | 190803 | 2 |
| FEBRA2020484 | 0.045837097 | 22079 | 14 |
| FEBRA2020566 | 0.040212971 | 83643 | 2 |
| FEBRA2020582 | 0.007272431 | 115348 | 2 |
| FEBRA2020668 | 0.004176935 | 49133 | 1 |
| FEBRA2020801 | 0.004176935 | 137452 | 1 |
| FEBRA2020845 | 0.005852482 | 253224 | 2 |
| FEBRA2020886 | 0.013820389 | 43862 | 5 |
| FEBRA2020977 | 0.031797766 | 48839 | 7 |
| FEBRA2021032 | 0.031253286 | 3688 | 5 |
| FEBRA2021134 | 0.004176935 | 126153 | 1 |
| FEBRA2021171 | 0.015750862 | 131671 | 4 |
| FEBRA2021339 | 0.005272213 | 133147 | 2 |
| FEBRA2021431 | 0.004176935 | 123477 | 1 |
| FEBRA2021497 | 0.004176935 | 112425 | 1 |
| FEBRA2021550 | 0.005366179 | 112254 | 2 |
| FEBRA2021571 | 0.004176935 | 61967 | 1 |
| FEBRA2021636 | 0.008281036 | 72901 | 3 |
| FEBRA2021864 | 0.005366179 | 116323 | 2 |
| FEBRA2021892 | 0.004176935 | 107158 | 1 |
| FEBRA2021908 | 0.004176935 | 272994 | 1 |
| FEBRA2021966 | 0.038843984 | 110533 | 7 |
| FEBRA2022013 | 0.017821942 | 21906 | 3 |
| FEBRA2022055 | 0.314892511 | 96889 | 33 |
| FEBRA2022148 | 0.004176935 | 108629 | 1 |
| FEBRA2022204 | 0.004176935 | 104762 | 1 |
| FEBRA2022255 | 0.004176935 | 66090 | 1 |
| FEBRA2022322 | 0.004176935 | 49854 | 1 |
| FEBRA2022388 | 0.219398702 | 86661 | 51 |
| FEBRA2022504 | 0.102020016 | 144180 | 19 |
| FEBRA2022601 | 0.005366179 | 108100 | 2 |
| FEBRA2022696 | 0.008021019 | 255161 | 2 |
| FEBRA2022729 | 0.004176935 | 107507 | 1 |
| FEBRA2022787 | 0.004176935 | 29149 | 1 |
| FEBRA2022911 | 0.004176935 | 74986 | 1 |
| FEBRA2022956 | 0.004176935 | 184652 | 1 |
| FEBRA2022963 | 0.004176935 | 104928 | 1 |
| FEBRA2022972 | 0.004176935 | 48074 | 1 |
| FEBRA2023227 | 0.032715806 | 42324 | 5 |
| FEBRA2023285 | 0.004176935 | 269499 | 1 |
| FEBRA2023351 | 0.004176935 | 109927 | 1 |
| FEBRA2023377 | 0.021599684 | 82634 | 6 |
| FEBRA2023399 | 0.019089191 | 105396 | 4 |
| FEBRA2023498 | 0.004176935 | 250642 | 1 |
| FEBRA2023550 | 0.010231303 | 108011 | 2 |
| FEBRA2023721 | 0.004176935 | 108781 | 1 |
| FEBRA2023764 | 0.142252985 | 132485 | 49 |
| FEBRA2023927 | 0.041138741 | 14663 | 17 |
| FEBRA2023989 | 0.004176935 | 109770 | 1 |
| FEBRA2023990 | 0.004176935 | 106902 | 1 |
| FEBRA2024019 | 0.124941952 | 90732 | 35 |
| FEBRA2024136 | 0.014604759 | 33488 | 5 |
| FEBRA2024150 | 0.00746277 | 103517 | 4 |
| FEBRA2024343 | 0.004176935 | 104950 | 1 |
| FEBRA2024570 | 0.016649651 | 109698 | 3 |
| FEBRA2024693 | 0.004176935 | 108953 | 1 |
| FEBRA2024727 | 0.006258447 | 89739 | 2 |
| FEBRA2024744 | 0.004176935 | 108970 | 1 |
| FEBRA2024797 | 0.004176935 | 162537 | 1 |
| FEBRA2024987 | 0.017032768 | 112421 | 3 |
| FEBRA2025249 | 0.004176935 | 108593 | 1 |
| FEBRA2025427 | 0.067594876 | 113139 | 15 |
| FEBRA2025463 | 0.004176935 | 150904 | 1 |
| FEBRA2025477 | 0.004176935 | 107718 | 1 |
| FEBRA2025838 | 0.203971855 | 98825 | 23 |
| FEBRA2026474 | 0.004176935 | 169879 | 1 |
| FEBRA2026582 | 0.007221817 | 151672 | 2 |
| FEBRA2026629 | 0.004176935 | 261073 | 1 |
| FEBRA2026769 | 0.004176935 | 181728 | 1 |
| FEBRA2026879 | 0.004176935 | 221496 | 1 |
| FEBRA2026890 | 0.004176935 | 178116 | 1 |
| FEBRA2026977 | 0.004176935 | 229822 | 1 |
| FEBRA2026984 | 0.009555137 | 161905 | 5 |
| FEBRA2027082 | 0.004176935 | 195860 | 1 |
| FEBRA2027297 | 0.004176935 | 252510 | 1 |
| FEBRA2027352 | 0.004176935 | 126254 | 1 |
| FEBRA2027364 | 0.004176935 | 184588 | 1 |
| FEBRA2027609 | 0.004176935 | 18770 | 1 |
| FEBRA2027742 | 0.025838097 | 13600 | 14 |
| FEBRA2028158 | 0.006053777 | 246879 | 2 |
| FEBRA2028222 | 0.004176935 | 284042 | 1 |
| FEBRA2028256 | 0.02236498 | 181783 | 4 |
| FEBRA2028366 | 0.004176935 | 280117 | 1 |
| FEBRA2028457 | 0.004176935 | 14163 | 1 |
| FEBRA2028477 | 0.004176935 | 166823 | 1 |
| FEBRA2028503 | 0.023965831 | 148429 | 7 |
| FEBRA2028516 | 0.146491235 | 88510 | 41 |
| FEBRA2028618 | 0.032970596 | 75233 | 13 |
| FEHRT2000325 | 0.034891835 | 70992 | 1 |
| FEHRT2000364 | 0.037806692 | 57020 | 2 |
| FEKID1000018 | 0.042447336 | 117026 | 2 |
| FELIV1000101 | 0.539031564 | 44356 | 2 |
| FELIV1000137 | 0.540700495 | 56709 | 3 |
| FELIV1000153 | 1.015909407 | 54819 | 42 |
| FELIV1000164 | 0.576279927 | 46875 | 4 |
| FELNG2000241 | 0.036036036 | 282691 | 1 |
| HCASM1000021 | 0.011124708 | 126275 | 1 |
| HCASM1000050 | 0.011124708 | 98126 | 1 |
| HCASM1000061 | 0.011124708 | 19737 | 1 |
| HCASM1000115 | 0.16165611 | 52993 | 20 |
| HCASM1000130 | 0.011124708 | 56971 | 1 |
| HCASM2000016 | 0.224501738 | 98352 | 38 |
| HCASM2000138 | 0.039671728 | 19852 | 19 |
| HCASM2000156 | 0.012812985 | 119815 | 2 |
| HCASM2000202 | 0.011124708 | 232040 | 1 |
| HCASM2000214 | 0.017715981 | 67601 | 4 |
| HCASM2000266 | 0.011124708 | 156085 | 1 |
| HCASM2000307 | 0.011124708 | 114209 | 1 |
| HCASM2000363 | 0.246392643 | 31143 | 25 |
| HCASM2000534 | 0.011124708 | 179022 | 1 |
| HCASM2000536 | 0.011124708 | 111511 | 1 |
| HCASM2000560 | 0.011124708 | 253520 | 1 |
| HCASM2000871 | 0.011124708 | 205937 | 1 |
| HCASM2001285 | 0.011124708 | 201665 | 1 |
| HCASM2001301 | 0.017327028 | 113838 | 2 |
| HCASM2001810 | 0.011124708 | 246145 | 1 |
| HCASM2001866 | 0.011124708 | 14388 | 1 |
| HCASM2001890 | 0.013123109 | 245441 | 2 |
| HCASM2002148 | 0.011124708 | 224626 | 1 |
| HCASM2002502 | 0.011124708 | 55010 | 1 |
| HCASM2002754 | 0.011124708 | 270319 | 1 |
| HCASM2002918 | 0.011124708 | 210349 | 1 |
| HCASM2003018 | 0.018984488 | 193278 | 7 |
| HCASM2003076 | 0.02942649 | 23765 | 4 |
| HCASM2003099 | 0.011124708 | 227979 | 1 |
| HCASM2003212 | 0.011124708 | 280389 | 1 |
| HCASM2003237 | 0.011124708 | 112291 | 1 |
| HCASM2003357 | 0.011124708 | 274475 | 1 |
| HCASM2003415 | 0.099890733 | 140729 | 3 |
| HCASM2005388 | 0.047371899 | 122277 | 8 |
| HCASM2005484 | 0.012982822 | 111937 | 2 |
| HCASM2006131 | 0.011124708 | 205625 | 1 |
| HCASM2006338 | 0.011124708 | 172892 | 1 |
| HCASM2006359 | 0.011124708 | 185574 | 1 |
| HCASM2006632 | 0.011124708 | 245983 | 1 |
| HCASM2007047 | 0.048836291 | 170371 | 3 |
| HCASM2007317 | 0.011124708 | 220628 | 1 |
| HCASM2007737 | 0.011124708 | 148618 | 1 |
| HCASM2007773 | 0.011124708 | 120553 | 1 |
| HCASM2008154 | 0.011124708 | 15455 | 1 |
| HCASM2008536 | 0.013123109 | 130406 | 2 |
| HCASM2009405 | 0.011124708 | 273620 | 1 |
| HCASM2009424 | 0.011124708 | 185414 | 1 |
| HCASM2009463 | 0.011124708 | 220610 | 1 |
| HCASM2009588 | 0.160156005 | 136063 | 2 |
| HCHON1000015 | 0.107497299 | 5029 | 14 |
| HCHON1000030 | 0.923793667 | 28724 | 139 |
| HCHON1000131 | 0.040839417 | 65581 | 12 |
| HCHON1000142 | 0.012600607 | 35194 | 2 |
| HCHON1000166 | 0.055699267 | 64723 | 11 |
| HCHON1000176 | 0.010602205 | 67842 | 1 |
| HCHON1000191 | 0.346899111 | 12702 | 20 |
| HCHON2000028 | 0.105609731 | 88009 | 24 |
| HCHON2000038 | 0.010602205 | 98996 | 1 |
| HCHON2000056 | 0.590768361 | 33092 | 92 |
| HCHON2000062 | 0.039977658 | 35045 | 7 |
| HCHON2000087 | 0.270837225 | 71643 | 19 |
| HCHON2000100 | 0.010602205 | 153476 | 1 |
| HCHON2000156 | 0.010602205 | 58975 | 1 |
| HCHON2000160 | 0.166457755 | 90503 | 37 |
| HCHON2000199 | 0.010602205 | 193073 | 1 |
| HCHON2000212 | 0.019495442 | 57915 | 5 |
| HCHON2000226 | 0.415523562 | 76543 | 41 |
| HCHON2000244 | 0.254716517 | 126260 | 33 |
| HCHON2000265 | 0.082815681 | 145896 | 9 |
| HCHON2000271 | 0.010602205 | 114194 | 1 |
| HCHON2000295 | 0.382534307 | 44066 | 23 |
| HCHON2000315 | 0.010602205 | 271872 | 1 |
| HCHON2000322 | 0.015259711 | 220834 | 3 |
| HCHON2000323 | 0.090453209 | 142866 | 12 |
| HCHON2000344 | 0.010602205 | 62319 | 1 |
| HCHON2000364 | 0.37689031 | 12853 | 19 |
| HCHON2000373 | 0.165324137 | 60351 | 23 |
| HCHON2000395 | 0.010602205 | 175810 | 1 |
| HCHON2000417 | 0.010602205 | 30763 | 1 |
| HCHON2000418 | 0.012600607 | 86945 | 2 |
| HCHON2000473 | 0.028625077 | 107435 | 10 |
| HCHON2000475 | 0.125879405 | 106653 | 17 |
| HCHON2000503 | 0.010602205 | 115620 | 1 |
| HCHON2000508 | 0.448707369 | 126548 | 48 |
| HCHON2000626 | 0.142540221 | 92209 | 23 |
| HCHON2000660 | 0.010602205 | 110230 | 1 |
| HCHON2000663 | 0.019641786 | 225426 | 5 |
| HCHON2000673 | 0.010602205 | 81829 | 1 |
| HCHON2000676 | 0.056199156 | 11960 | 9 |
| HCHON2000698 | 0.058457997 | 141006 | 20 |
| HCHON2000699 | 0.011999361 | 126073 | 2 |
| HCHON2000705 | 0.016815315 | 99834 | 2 |
| HCHON2000738 | 0.056273021 | 122890 | 13 |
| HCHON2000743 | 0.027443448 | 36390 | 10 |
| HCHON2000751 | 0.046931294 | 15705 | 12 |
| HCHON2000781 | 0.062218274 | 17625 | 20 |
| HCHON2000815 | 0.059998228 | 101772 | 11 |
| HCHON2000817 | 0.010602205 | 269196 | 1 |
| HCHON2000818 | 0.010602205 | 267907 | 1 |
| HCHON2000819 | 0.022869683 | 56616 | 3 |
| HCHON2000826 | 0.23411552 | 87016 | 57 |
| HCHON2000832 | 0.047360549 | 159261 | 10 |
| HCHON2000851 | 0.010602205 | 267889 | 1 |
| HCHON2000898 | 0.016443668 | 199625 | 2 |
| HCHON2000935 | 0.010602205 | 159966 | 1 |
| HCHON2000942 | 0.01477914 | 58469 | 2 |
| HCHON2000956 | 0.010602205 | 98638 | 1 |
| HCHON2000994 | 0.021204411 | 12791 | 2 |
| HCHON2001039 | 0.150161876 | 78146 | 60 |
| HCHON2001050 | 0.11560155 | 145954 | 8 |
| HCHON2001084 | 0.072972628 | 87760 | 12 |
| HCHON2001099 | 0.061433165 | 112187 | 9 |
| HCHON2001108 | 0.078470402 | 20531 | 5 |
| HCHON2001110 | 0.020310943 | 125628 | 2 |
| HCHON2001116 | 0.010602205 | 129287 | 1 |
| HCHON2001161 | 0.015561238 | 105445 | 4 |
| HCHON2001200 | 0.387403789 | 34429 | 51 |
| HCHON2001214 | 0.010602205 | 269459 | 1 |
| HCHON2001217 | 0.160978591 | 5378 | 50 |
| HCHON2001233 | 0.010602205 | 45213 | 1 |
| HCHON2001269 | 0.010602205 | 67140 | 1 |
| HCHON2001359 | 0.1968093 | 76309 | 30 |
| HCHON2001407 | 0.212188264 | 117940 | 34 |
| HCHON2001434 | 0.10968658 | 48012 | 37 |
| HCHON2001450 | 0.010602205 | 47009 | 1 |
| HCHON2001453 | 0.010602205 | 268556 | 1 |
| HCHON2001497 | 0.02236276 | 154626 | 2 |
| HCHON2001505 | 0.037286861 | 12745 | 11 |
| HCHON2001519 | 0.011697484 | 257017 | 2 |
| HCHON2001523 | 0.010602205 | 83523 | 1 |
| HCHON2001535 | 0.018169384 | 66915 | 5 |
| HCHON2001546 | 0.023125687 | 52241 | 2 |
| HCHON2001548 | 0.014640488 | 101326 | 2 |
| HCHON2001577 | 0.010602205 | 37624 | 1 |
| HCHON2001598 | 0.068959654 | 132427 | 8 |
| HCHON2001604 | 0.010602205 | 12950 | 1 |
| HCHON2001607 | 0.010602205 | 285606 | 1 |
| HCHON2001646 | 0.010602205 | 269204 | 1 |
| HCHON2001665 | 0.108581659 | 101140 | 27 |
| HCHON2001712 | 0.011999361 | 77424 | 2 |
| HCHON2001768 | 0.010602205 | 89904 | 1 |
| HCHON2001801 | 0.01653798 | 180355 | 2 |
| HCHON2001853 | 0.029917426 | 117972 | 7 |
| HCHON2002123 | 0.010602205 | 12763 | 1 |
| HCHON2002194 | 0.010602205 | 138024 | 1 |
| HCHON2002241 | 0.010602205 | 173487 | 1 |
| HCHON2002247 | 0.059683268 | 59169 | 15 |
| HCHON2002354 | 0.013188605 | 82745 | 3 |
| HCHON2002496 | 0.021572076 | 46490 | 3 |
| HCHON2002676 | 0.012277752 | 212131 | 2 |
| HCHON2002770 | 0.010602205 | 176302 | 1 |
| HCHON2002964 | 0.021204411 | 12872 | 2 |
| HCHON2002971 | 0.011999361 | 178400 | 2 |
| HCHON2003131 | 0.010602205 | 92613 | 1 |
| HCHON2003134 | 0.010602205 | 28465 | 1 |
| HCHON2003327 | 0.010602205 | 22149 | 1 |
| HCHON2003513 | 0.010602205 | 105982 | 1 |
| HCHON2003532 | 0.010602205 | 207938 | 1 |
| HCHON2003642 | 0.013517062 | 93307 | 2 |
| HCHON2003659 | 0.010602205 | 965 | 1 |
| HCHON2003676 | 0.010602205 | 243165 | 1 |
| HCHON2004002 | 0.213419707 | 130575 | 37 |
| HCHON2004007 | 0.010602205 | 135584 | 1 |
| HCHON2004279 | 0.010602205 | 136665 | 1 |
| HCHON2004359 | 0.010602205 | 104086 | 1 |
| HCHON2004531 | 0.010602205 | 36374 | 1 |
| HCHON2004776 | 0.010602205 | 127547 | 1 |
| HCHON2004858 | 0.012683717 | 116759 | 2 |
| HCHON2005048 | 0.010602205 | 111303 | 1 |
| HCHON2005118 | 0.010602205 | 172354 | 1 |
| HCHON2005166 | 0.013697701 | 146621 | 2 |
| HCHON2005802 | 0.010602205 | 217253 | 1 |
| HCHON2005921 | 0.040400707 | 154965 | 16 |
| HCHON2005987 | 0.016804525 | 176993 | 2 |
| HCHON2006250 | 0.107081819 | 27447 | 10 |
| HCHON2006459 | 0.015523928 | 158722 | 3 |
| HCHON2006714 | 0.010602205 | 151914 | 1 |
| HCHON2006722 | 0.01653798 | 126183 | 2 |
| HCHON2006770 | 0.059368571 | 66629 | 4 |
| HCHON2006786 | 0.012311665 | 52239 | 2 |
| HCHON2006841 | 0.010602205 | 138680 | 1 |
| HCHON2006871 | 0.010602205 | 195906 | 1 |
| HCHON2007046 | 0.046638241 | 71519 | 2 |
| HCHON2007482 | 0.010602205 | 200988 | 1 |
| HCHON2007650 | 0.027514824 | 76325 | 5 |
| HCHON2007674 | 0.010602205 | 14156 | 1 |
| HCHON2007774 | 0.010602205 | 103138 | 1 |
| HCHON2007881 | 0.012683717 | 184857 | 2 |
| HCHON2008112 | 0.012290482 | 184362 | 2 |
| HCHON2008239 | 0.010602205 | 163399 | 1 |
| HCHON2008444 | 0.012600607 | 130366 | 2 |
| HCHON2008582 | 0.010602205 | 162830 | 1 |
| HCHON2008672 | 0.010602205 | 13028 | 1 |
| HCHON2008856 | 0.016804525 | 206860 | 2 |
| HCHON2008871 | 0.010602205 | 150290 | 1 |
| HCHON2008989 | 0.010602205 | 110588 | 1 |
| HCHON2009191 | 0.010602205 | 169572 | 1 |
| HCHON2009384 | 0.010602205 | 143708 | 1 |
| HCHON2009749 | 0.010602205 | 75588 | 1 |
| HCHON2009766 | 0.010602205 | 145551 | 1 |
| HCHON2009795 | 0.010602205 | 13046 | 1 |
| HCHON2010074 | 0.010602205 | 30481 | 1 |
| HCHON2010543 | 0.018677918 | 134404 | 5 |
| HEART1000010 | 0.049042994 | 21506 | 5 |
| HEART1000074 | 0.220726718 | 60269 | 31 |
| HEART1000088 | 0.022464862 | 49457 | 2 |
| HEART1000099 | 0.01117818 | 45227 | 1 |
| HEART1000102 | 0.01117818 | 35888 | 1 |
| HEART1000118 | 0.015117807 | 43130 | 3 |
| HEART1000132 | 0.057689644 | 43128 | 5 |
| HEART1000139 | 0.033643042 | 69689 | 3 |
| HEART1000142 | 0.093988249 | 51283 | 7 |
| HEART1000149 | 0.178424584 | 81037 | 32 |
| HEART1000151 | 0.01117818 | 45223 | 1 |
| HEART1000173 | 0.063840356 | 70047 | 5 |
| HEART1000185 | 0.211938906 | 57208 | 23 |
| HEART2000024 | 0.01117818 | 228339 | 1 |
| HEART2000035 | 0.01117818 | 104735 | 1 |
| HEART2000099 | 0.01117818 | 260986 | 1 |
| HEART2000298 | 0.01117818 | 229589 | 1 |
| HEART2000306 | 0.012367425 | 46612 | 2 |
| HEART2000309 | 0.01117818 | 213852 | 1 |
| HEART2000411 | 0.079417809 | 138027 | 7 |
| HEART2000418 | 0.01117818 | 241174 | 1 |
| HEART2000444 | 0.01989553 | 172999 | 4 |
| HEART2000448 | 0.02235636 | 69697 | 2 |
| HEART2000492 | 0.01117818 | 235982 | 1 |
| HEART2000506 | 0.095544218 | 148473 | 14 |
| HEART2000520 | 0.116199863 | 171346 | 11 |
| HEART2000541 | 0.125233313 | 154993 | 12 |
| HEART2000568 | 0.01117818 | 235971 | 1 |
| HEART2000611 | 0.02235636 | 109962 | 2 |
| HEART2000959 | 0.014225539 | 163957 | 3 |
| HEART2001680 | 0.01117818 | 273158 | 1 |
| HEART2001756 | 0.02235636 | 193083 | 2 |
| HEART2001773 | 0.075126312 | 91738 | 8 |
| HEART2001931 | 0.045147519 | 177409 | 5 |
| HEART2002129 | 0.01117818 | 90745 | 1 |
| HEART2002184 | 0.01117818 | 193412 | 1 |
| HEART2002220 | 0.01117818 | 58213 | 1 |
| HEART2002432 | 0.01117818 | 253027 | 1 |
| HEART2002531 | 0.01117818 | 268691 | 1 |
| HEART2002598 | 0.01117818 | 53797 | 1 |
| HEART2002717 | 0.01117818 | 146320 | 1 |
| HEART2003027 | 0.01117818 | 41622 | 1 |
| HEART2003168 | 0.013036295 | 42732 | 2 |
| HEART2003432 | 0.01117818 | 201533 | 1 |
| HEART2003440 | 0.057239903 | 152416 | 2 |
| HEART2003781 | 0.01117818 | 156417 | 1 |
| HEART2003836 | 0.081444128 | 143072 | 8 |
| HEART2003852 | 0.043005124 | 138957 | 6 |
| HEART2004570 | 0.01117818 | 120195 | 1 |
| HEART2004764 | 0.013368737 | 113879 | 3 |
| HEART2004931 | 0.024640527 | 119883 | 3 |
| HEART2004940 | 0.01117818 | 188184 | 1 |
| HEART2004941 | 0.035276163 | 202388 | 5 |
| HEART2004980 | 0.01117818 | 246209 | 1 |
| HEART2005244 | 0.01117818 | 267169 | 1 |
| HEART2005310 | 0.024034014 | 95327 | 3 |
| HEART2005581 | 0.01117818 | 193308 | 1 |
| HEART2005610 | 0.01117818 | 53804 | 1 |
| HEART2006131 | 0.01117818 | 178056 | 1 |
| HEART2006195 | 0.017679339 | 142370 | 4 |
| HEART2006310 | 0.01117818 | 159388 | 1 |
| HEART2006334 | 0.01117818 | 167816 | 1 |
| HEART2006487 | 0.017232548 | 155920 | 2 |
| HEART2006521 | 0.070054992 | 124825 | 15 |
| HEART2006557 | 0.01117818 | 230159 | 1 |
| HEART2006787 | 0.01117818 | 180547 | 1 |
| HEART2006789 | 0.01117818 | 172754 | 1 |
| HEART2006909 | 0.01117818 | 167824 | 1 |
| HEART2007031 | 0.014745755 | 124197 | 3 |
| HEART2007231 | 0.034590572 | 158945 | 11 |
| HEART2007390 | 0.290288676 | 36444 | 21 |
| HEART2007443 | 0.01117818 | 206096 | 1 |
| HEART2007696 | 0.033639989 | 4604 | 5 |
| HEART2007767 | 0.01117818 | 131539 | 1 |
| HEART2008108 | 0.01117818 | 72050 | 1 |
| HEART2008111 | 0.01117818 | 44305 | 1 |
| HEART2008257 | 0.01117818 | 53827 | 1 |
| HEART2008364 | 0.075460802 | 181167 | 7 |
| HEART2008509 | 0.028397926 | 164043 | 3 |
| HEART2008994 | 0.01117818 | 133477 | 1 |
| HEART2009000 | 0.016211509 | 97696 | 3 |
| HEART2009599 | 0.01117818 | 283388 | 1 |
| HEART2009680 | 0.01117818 | 178151 | 1 |
| HEART2010391 | 0.01117818 | 212941 | 1 |
| HEART2010492 | 0.01117818 | 123242 | 1 |
| HEART2010495 | 0.022464862 | 198410 | 2 |
| HELAC2000158 | 0.147058824 | 243951 | 1 |
| HELAC2000301 | 0.147058824 | 243318 | 1 |
| HHDPC1000001 | 0.011798018 | 62665 | 1 |
| HHDPC1000065 | 0.018323554 | 56037 | 5 |
| HHDPC1000083 | 0.17118191 | 5301 | 27 |
| HHDPC1000114 | 0.230602388 | 76468 | 33 |
| HHDPC1000118 | 0.079948571 | 61435 | 19 |
| HHDPC1000163 | 0.133241281 | 72507 | 21 |
| HHDPC2000055 | 0.091632272 | 121305 | 14 |
| HHDPC2000070 | 0.017411258 | 117037 | 4 |
| HHDPC2000095 | 0.220123573 | 66433 | 33 |
| HHDPC2000102 | 0.035288814 | 147613 | 6 |
| HHDPC2000104 | 0.090570063 | 116696 | 11 |
| HHDPC2000115 | 0.064051116 | 122929 | 10 |
| HHDPC2000149 | 0.037498931 | 69887 | 9 |
| HHDPC2000258 | 0.017733793 | 181248 | 2 |
| HHDPC2000315 | 0.011798018 | 126680 | 1 |
| HHDPC2000455 | 0.304913305 | 83824 | 31 |
| HHDPC2000456 | 0.107919863 | 48562 | 17 |
| HHDPC2000462 | 0.014842899 | 157121 | 2 |
| HHDPC2000572 | 0.025592632 | 123565 | 7 |
| HHDPC2000656 | 0.019601597 | 22695 | 3 |
| HHDPC2000692 | 0.011798018 | 181235 | 1 |
| HHDPC2001337 | 0.011798018 | 232626 | 1 |
| HHDPC2001432 | 0.011798018 | 122668 | 1 |
| HHDPC2001896 | 0.017870401 | 158517 | 2 |
| HHDPC2002963 | 0.011798018 | 8549 | 1 |
| HHDPC2003049 | 0.011798018 | 13118 | 1 |
| HHDPC2003113 | 0.011798018 | 219934 | 1 |
| HHDPC2003381 | 0.048043034 | 153643 | 2 |
| HHDPC2003439 | 0.658805628 | 68709 | 109 |
| HHDPC2003472 | 0.012893296 | 39729 | 2 |
| HHDPC2003820 | 0.044469609 | 82943 | 9 |
| HHDPC2003983 | 0.601712808 | 49467 | 110 |
| HHDPC2004757 | 0.011798018 | 182510 | 1 |
| HHDPC2005185 | 0.011798018 | 128495 | 1 |
| HHDPC2005513 | 0.011798018 | 163283 | 1 |
| HHDPC2005742 | 0.013195173 | 34044 | 2 |
| HHDPC2005794 | 0.021064004 | 141825 | 6 |
| HHDPC2006460 | 0.017627732 | 49614 | 3 |
| HHDPC2006862 | 0.022386969 | 62883 | 4 |
| HHDPC2006927 | 0.011798018 | 74694 | 1 |
| HHDPC2007267 | 0.343194637 | 57162 | 52 |
| HHDPC2007302 | 0.011798018 | 264356 | 1 |
| HHDPC2007775 | 0.015505879 | 108163 | 3 |
| HHDPC2008123 | 0.013195173 | 14188 | 2 |
| HHDPC2008185 | 0.011798018 | 78478 | 1 |
| HHDPC2008279 | 0.011798018 | 201628 | 1 |
| HHDPC2008297 | 0.022400223 | 157308 | 2 |
| HHDPC2008414 | 0.011798018 | 194567 | 1 |
| HHDPC2008474 | 0.011798018 | 12860 | 1 |
| HHDPC2008816 | 0.013656132 | 154642 | 2 |
| HHDPC2008843 | 0.011798018 | 263265 | 1 |
| HHDPC2009114 | 0.011798018 | 66636 | 1 |
| HHDPC2009178 | 0.016507188 | 143140 | 3 |
| HHDPC2009208 | 0.011798018 | 12769 | 1 |
| HHDPC2009272 | 0.01367486 | 94691 | 2 |
| HHDPC2009528 | 0.025780516 | 124939 | 9 |
| HLUNG1000017 | 0.006145526 | 72627 | 1 |
| HLUNG1000024 | 0.006145526 | 78128 | 1 |
| HLUNG1000030 | 0.006145526 | 76039 | 1 |
| HLUNG1000034 | 0.006145526 | 70805 | 1 |
| HLUNG1000037 | 0.01906854 | 65539 | 5 |
| HLUNG1000041 | 0.042734607 | 49300 | 15 |
| HLUNG1000053 | 0.006145526 | 71892 | 1 |
| HLUNG1000055 | 0.309541219 | 74617 | 56 |
| HLUNG1000064 | 0.024606665 | 67427 | 6 |
| HLUNG1000076 | 0.199173756 | 72867 | 29 |
| HLUNG1000084 | 0.006145526 | 67966 | 1 |
| HLUNG1000091 | 0.011322534 | 72763 | 3 |
| HLUNG1000099 | 0.015524287 | 46123 | 7 |
| HLUNG1000104 | 0.006145526 | 59147 | 1 |
| HLUNG1000105 | 0.015854264 | 74322 | 2 |
| HLUNG1000110 | 0.007240804 | 76890 | 2 |
| HLUNG1000151 | 0.006145526 | 59012 | 1 |
| HLUNG1000169 | 0.006145526 | 67046 | 1 |
| HLUNG2000004 | 0.066243039 | 115888 | 11 |
| HLUNG2000014 | 0.006145526 | 180932 | 1 |
| HLUNG2000027 | 0.006145526 | 41556 | 1 |
| HLUNG2000063 | 0.067937545 | 63277 | 16 |
| HLUNG2000068 | 0.006145526 | 103991 | 1 |
| HLUNG2000116 | 0.006145526 | 78124 | 1 |
| HLUNG2000125 | 0.046965116 | 116845 | 3 |
| HLUNG2000130 | 0.006145526 | 262864 | 1 |
| HLUNG2000142 | 0.012291052 | 100905 | 2 |
| HLUNG2000174 | 0.006145526 | 185033 | 1 |
| HLUNG2000176 | 0.007542681 | 103313 | 2 |
| HLUNG2000217 | 0.006145526 | 98761 | 1 |
| HLUNG2000232 | 0.013295058 | 99956 | 5 |
| HLUNG2000255 | 0.006145526 | 167263 | 1 |
| HLUNG2000281 | 0.006145526 | 147023 | 1 |
| HLUNG2000314 | 0.068300052 | 58776 | 9 |
| HLUNG2000315 | 0.0109856 | 123552 | 3 |
| HLUNG2000340 | 0.006145526 | 215325 | 1 |
| HLUNG2000362 | 0.006145526 | 159246 | 1 |
| HLUNG2000363 | 0.006145526 | 147846 | 1 |
| HLUNG2000412 | 0.062576097 | 146816 | 21 |
| HLUNG2000431 | 0.006145526 | 198509 | 1 |
| HLUNG2000480 | 0.006145526 | 270444 | 1 |
| HLUNG2000489 | 0.006145526 | 168597 | 1 |
| HLUNG2000501 | 0.195322804 | 122488 | 23 |
| HLUNG2000549 | 0.006145526 | 35872 | 1 |
| HLUNG2000557 | 0.006145526 | 282138 | 1 |
| HLUNG2000570 | 0.006145526 | 129597 | 1 |
| HLUNG2000751 | 0.009697915 | 242649 | 3 |
| HLUNG2000761 | 0.006145526 | 178685 | 1 |
| HLUNG2000777 | 0.006145526 | 225200 | 1 |
| HLUNG2000846 | 0.037087907 | 29445 | 19 |
| HLUNG2000870 | 0.006145526 | 240667 | 1 |
| HLUNG2000884 | 0.078279812 | 100688 | 17 |
| HLUNG2000926 | 0.006145526 | 210476 | 1 |
| HLUNG2000950 | 0.013553035 | 68684 | 4 |
| HLUNG2000955 | 0.006145526 | 236406 | 1 |
| HLUNG2000970 | 0.006145526 | 114929 | 1 |
| HLUNG2001013 | 0.006145526 | 139663 | 1 |
| HLUNG2001126 | 0.006145526 | 171876 | 1 |
| HLUNG2001144 | 0.006145526 | 212936 | 1 |
| HLUNG2001146 | 0.031964516 | 36493 | 13 |
| HLUNG2001214 | 0.006145526 | 272875 | 1 |
| HLUNG2001439 | 0.189995764 | 110658 | 12 |
| HLUNG2001459 | 0.025566028 | 125304 | 4 |
| HLUNG2001507 | 0.033460848 | 148830 | 7 |
| HLUNG2001518 | 0.153231919 | 112210 | 57 |
| HLUNG2001633 | 0.020926599 | 162390 | 7 |
| HLUNG2001677 | 0.047685159 | 151366 | 4 |
| HLUNG2001712 | 0.050819864 | 158995 | 9 |
| HLUNG2001814 | 0.006145526 | 96912 | 1 |
| HLUNG2001996 | 0.00800364 | 140256 | 2 |
| HLUNG2002006 | 0.006145526 | 232850 | 1 |
| HLUNG2002050 | 0.006145526 | 277250 | 1 |
| HLUNG2002059 | 0.061168426 | 124953 | 14 |
| HLUNG2002085 | 0.006145526 | 132761 | 1 |
| HLUNG2002145 | 0.012058138 | 122238 | 2 |
| HLUNG2002295 | 0.006145526 | 124756 | 1 |
| HLUNG2002303 | 0.006145526 | 132758 | 1 |
| HLUNG2002334 | 0.018228262 | 117124 | 4 |
| HLUNG2002405 | 0.006145526 | 137055 | 1 |
| HLUNG2002429 | 0.006145526 | 36838 | 1 |
| HLUNG2002465 | 0.138538996 | 109565 | 24 |
| HLUNG2002562 | 0.006145526 | 172863 | 1 |
| HLUNG2002648 | 0.046357932 | 6932 | 12 |
| HLUNG2002659 | 0.006145526 | 82510 | 1 |
| HLUNG2002707 | 0.006145526 | 132794 | 1 |
| HLUNG2002811 | 0.068764045 | 128957 | 16 |
| HLUNG2002916 | 0.019421838 | 79803 | 4 |
| HLUNG2002942 | 0.006145526 | 66941 | 1 |
| HLUNG2002949 | 0.009060383 | 254290 | 2 |
| HLUNG2002958 | 0.006145526 | 118783 | 1 |
| HLUNG2002982 | 0.006145526 | 136347 | 1 |
| HLUNG2003003 | 0.020966941 | 136346 | 9 |
| HLUNG2003042 | 0.070389005 | 74489 | 12 |
| HLUNG2003049 | 0.006145526 | 115207 | 1 |
| HLUNG2003061 | 0.006145526 | 143985 | 1 |
| HLUNG2003162 | 0.006145526 | 164928 | 1 |
| HLUNG2003246 | 0.006145526 | 30639 | 1 |
| HLUNG2003306 | 0.013085839 | 82150 | 4 |
| HLUNG2003331 | 0.020238563 | 121772 | 3 |
| HLUNG2003335 | 0.006145526 | 56828 | 1 |
| HLUNG2003378 | 0.018097956 | 157593 | 5 |
| HLUNG2003497 | 0.007542681 | 104940 | 2 |
| HLUNG2003691 | 0.006145526 | 175642 | 1 |
| HLUNG2003710 | 0.006145526 | 158435 | 1 |
| HLUNG2003714 | 0.006145526 | 249799 | 1 |
| HLUNG2003716 | 0.007334771 | 131110 | 2 |
| HLUNG2003778 | 0.032482039 | 276412 | 4 |
| HLUNG2003833 | 0.006145526 | 145569 | 1 |
| HLUNG2003872 | 0.006145526 | 150308 | 1 |
| HLUNG2003918 | 0.006145526 | 150282 | 1 |
| HLUNG2003991 | 0.006145526 | 134026 | 1 |
| HLUNG2004014 | 0.006145526 | 256664 | 1 |
| HLUNG2004037 | 0.010975793 | 120070 | 3 |
| HLUNG2004067 | 0.015217137 | 155154 | 4 |
| HLUNG2004154 | 0.016112319 | 130330 | 4 |
| HLUNG2004159 | 0.092417776 | 82653 | 28 |
| HLUNG2004170 | 0.027192647 | 88725 | 10 |
| HLUNG2004217 | 0.006145526 | 216197 | 1 |
| HLUNG2004273 | 0.006145526 | 100851 | 1 |
| HLUNG2004388 | 0.037525551 | 67635 | 6 |
| HLUNG2004416 | 0.006145526 | 122402 | 1 |
| HLUNG2004521 | 0.006145526 | 167872 | 1 |
| HLUNG2004534 | 0.057763737 | 40889 | 5 |
| HLUNG2004684 | 0.049632352 | 128944 | 3 |
| HLUNG2004707 | 0.006145526 | 145388 | 1 |
| HLUNG2004774 | 0.010322461 | 122122 | 2 |
| HLUNG2004775 | 0.006145526 | 116347 | 1 |
| HLUNG2005012 | 0.006145526 | 257417 | 1 |
| HLUNG2005030 | 0.006145526 | 284372 | 1 |
| HLUNG2005045 | 0.006145526 | 265170 | 1 |
| HLUNG2005076 | 0.006145526 | 88164 | 1 |
| HLUNG2005133 | 0.023653453 | 188485 | 7 |
| HLUNG2005203 | 0.008022368 | 219697 | 2 |
| HLUNG2005230 | 0.073302178 | 152388 | 12 |
| HLUNG2005479 | 0.006145526 | 234756 | 1 |
| HLUNG2005524 | 0.006145526 | 249278 | 1 |
| HLUNG2005656 | 0.007334771 | 222721 | 2 |
| HLUNG2005738 | 0.009127485 | 145313 | 2 |
| HLUNG2005774 | 0.006145526 | 164685 | 1 |
| HLUNG2005813 | 0.006145526 | 182087 | 1 |
| HLUNG2005924 | 0.006145526 | 52917 | 1 |
| HLUNG2006026 | 0.006145526 | 270806 | 1 |
| HLUNG2006067 | 0.006145526 | 148774 | 1 |
| HLUNG2006094 | 0.006145526 | 132742 | 1 |
| HLUNG2006370 | 0.013688208 | 135498 | 3 |
| HLUNG2006397 | 0.006145526 | 75269 | 1 |
| HLUNG2006570 | 0.025925218 | 128800 | 7 |
| HLUNG2006582 | 0.042754388 | 125879 | 19 |
| HLUNG2006599 | 0.006145526 | 87557 | 1 |
| HLUNG2006614 | 0.006145526 | 117771 | 1 |
| HLUNG2006671 | 0.006145526 | 114640 | 1 |
| HLUNG2006781 | 0.006145526 | 130438 | 1 |
| HLUNG2006812 | 0.056664505 | 93210 | 16 |
| HLUNG2006843 | 0.016143548 | 145481 | 4 |
| HLUNG2006852 | 0.006145526 | 142367 | 1 |
| HLUNG2006935 | 0.006145526 | 122209 | 1 |
| HLUNG2007041 | 0.006145526 | 162624 | 1 |
| HLUNG2007163 | 0.012436936 | 22585 | 4 |
| HLUNG2007210 | 0.08151364 | 52666 | 5 |
| HLUNG2007219 | 0.007542681 | 217068 | 2 |
| HLUNG2007245 | 0.048232005 | 198625 | 3 |
| HLUNG2007433 | 0.006145526 | 160830 | 1 |
| HLUNG2007931 | 0.006145526 | 170288 | 1 |
| HLUNG2008066 | 0.006145526 | 251045 | 1 |
| HLUNG2008139 | 0.014619794 | 150094 | 7 |
| HLUNG2008153 | 0.024615669 | 106811 | 7 |
| HLUNG2008225 | 0.010155661 | 232783 | 3 |
| HLUNG2008235 | 0.006145526 | 117325 | 1 |
| HLUNG2008247 | 0.006145526 | 78365 | 1 |
| HLUNG2008333 | 0.008143927 | 172882 | 2 |
| HLUNG2008348 | 0.013186071 | 10845 | 5 |
| HLUNG2008384 | 0.006145526 | 171465 | 1 |
| HLUNG2008396 | 0.006145526 | 154567 | 1 |
| HLUNG2008439 | 0.006145526 | 128850 | 1 |
| HLUNG2008479 | 0.009266332 | 53154 | 2 |
| HLUNG2008480 | 0.006145526 | 172886 | 1 |
| HLUNG2008521 | 0.006145526 | 54814 | 1 |
| HLUNG2008637 | 0.099191534 | 130778 | 12 |
| HLUNG2008833 | 0.006145526 | 62087 | 1 |
| HLUNG2008875 | 0.006145526 | 144491 | 1 |
| HLUNG2008885 | 0.015521841 | 43100 | 5 |
| HLUNG2008958 | 0.006145526 | 39490 | 1 |
| HLUNG2008959 | 0.006145526 | 157210 | 1 |
| HLUNG2009067 | 0.00800364 | 86169 | 2 |
| HLUNG2009215 | 0.01208765 | 100215 | 2 |
| HLUNG2009225 | 0.111100879 | 41575 | 42 |
| HLUNG2009253 | 0.006145526 | 154964 | 1 |
| HLUNG2009303 | 0.006145526 | 131693 | 1 |
| HLUNG2009413 | 0.006145526 | 106841 | 1 |
| HLUNG2009729 | 0.006145526 | 194036 | 1 |
| HLUNG2010181 | 0.006145526 | 166438 | 1 |
| HLUNG2010464 | 0.006145526 | 164109 | 1 |
| HLUNG2010562 | 0.006145526 | 178578 | 1 |
| HLUNG2010845 | 0.006145526 | 227112 | 1 |
| HLUNG2011041 | 0.006145526 | 246578 | 1 |
| HLUNG2011298 | 0.017432208 | 130706 | 2 |
| HLUNG2011461 | 0.007821073 | 189369 | 2 |
| HLUNG2011833 | 0.04081959 | 102116 | 2 |
| HLUNG2012049 | 0.006145526 | 274426 | 1 |
| HLUNG2012287 | 0.006145526 | 142907 | 1 |
| HLUNG2012315 | 0.006145526 | 220327 | 1 |
| HLUNG2012546 | 0.006145526 | 4680 | 1 |
| HLUNG2012600 | 0.006145526 | 284241 | 1 |
| HLUNG2012727 | 0.006145526 | 118456 | 1 |
| HLUNG2013097 | 0.006145526 | 3394 | 1 |
| HLUNG2013204 | 0.006145526 | 240482 | 1 |
| HLUNG2013304 | 0.006145526 | 178511 | 1 |
| HLUNG2013350 | 0.006145526 | 184878 | 1 |
| HLUNG2013622 | 0.006145526 | 176158 | 1 |
| HLUNG2013643 | 0.006145526 | 187907 | 1 |
| HLUNG2013784 | 0.047951546 | 62356 | 2 |
| HLUNG2013851 | 0.006145526 | 160035 | 1 |
| HLUNG2014262 | 0.006145526 | 203114 | 1 |
| HLUNG2014288 | 0.006145526 | 184962 | 1 |
| HLUNG2014449 | 0.006145526 | 188574 | 1 |
| HLUNG2014821 | 0.009899209 | 182831 | 3 |
| HLUNG2015184 | 0.012796353 | 119676 | 5 |
| HLUNG2015418 | 0.008022368 | 98339 | 2 |
| HLUNG2015548 | 0.006145526 | 8040 | 1 |
| HLUNG2015578 | 0.006145526 | . 217563 | 1 |
| HLUNG2015617 | 0.006145526 | 170170 | 1 |
| HLUNG2016022 | 0.006145526 | 243719 | 1 |
| HLUNG2016862 | 0.006145526 | 248898 | 1 |
| HLUNG2017262 | 0.105658876 | 82144 | 50 |
| HLUNG2017286 | 0.006145526 | 193371 | 1 |
| HLUNG2017307 | 0.006145526 | 202242 | 1 |
| HLUNG2017350 | 0.006145526 | 268615 | 1 |
| HLUNG2017546 | 0.047478784 | 200901 | 3 |
| HLUNG2017806 | 0.006145526 | 169566 | 1 |
| HLUNG2018029 | 0.006145526 | 150492 | 1 |
| HLUNG2018282 | 0.006145526 | 277543 | 1 |
| HLUNG2018626 | 0.006145526 | 206788 | 1 |
| HLUNG2019058 | 0.006145526 | 221400 | 1 |
| HSYRA1000062 | 0.39118207 | 44116 | 62 |
| HSYRA1000119 | 0.3644211 | 78027 | 67 |
| HSYRA1000131 | 0.012523482 | 87609 | 1 |
| HSYRA1000137 | 0.044959562 | 43985 | 11 |
| HSYRA1000148 | 0.02540901 | 73950 | 4 |
| HSYRA1000152 | 0.140480179 | 48229 | 31 |
| HSYRA1000168 | 0.015505441 | 47014 | 2 |
| HSYRA1000178 | 0.066823107 | 18635 | 22 |
| HSYRA2000044 | 0.013920637 | 108021 | 2 |
| HSYRA2000135 | 0.042364748 | 118875 | 6 |
| HSYRA2000137 | 0.073922883 | 96221 | 11 |
| HSYRA2000159 | 1.389739123 | 9434 | 373 |
| HSYRA2000190 | 0.070547807 | 4816 | 12 |
| HSYRA2000221 | 0.01770049 | 89827 | 3 |
| HSYRA2000224 | 0.093183687 | 119952 | 18 |
| HSYRA2000232 | 0.22660084 | 33605 | 58 |
| HSYRA2000237 | 0.012523482 | 128212 | 1 |
| HSYRA2000248 | 0.246532535 | 78679 | 51 |
| HSYRA2000253 | 0.019832899 | 12156 | 3 |
| HSYRA2000255 | 0.373425306 | 60161 | 30 |
| HSYRA2000332 | 0.214826099 | 37573 | 17 |
| HSYRA2000347 | 0.234037348 | 89493 | 75 |
| HSYRA2000371 | 0.012523482 | 12968 | 1 |
| HSYRA2000424 | 0.014400323 | 105492 | 2 |
| HSYRA2000507 | 0.01361876 | 62084 | 2 |
| HSYRA2000510 | 0.012523482 | 240891 | 1 |
| HSYRA2000589 | 0.133555816 | 79239 | 29 |
| HSYRA2000605 | 0.028004799 | 135640 | 4 |
| HSYRA2000629 | 0.050203248 | 141543 | 7 |
| HSYRA2000640 | 0.196682364 | 29086 | 51 |
| HSYRA2000706 | 0.013712726 | 175272 | 2 |
| HSYRA2000743 | 0.216513216 | 79998 | 83 |
| HSYRA2000760 | 0.012523482 | 116092 | 1 |
| HSYRA2000787 | 0.05387047 | 7699 | 12 |
| HSYRA2000792 | 0.069187409 | 17868 | 3 |
| HSYRA2000828 | 0.11761207 | 120053 | 21 |
| HSYRA2000832 | 0.018669008 | 171140 | 2 |
| HSYRA2000925 | 0.756199735 | 94955 | 25 |
| HSYRA2000927 | 0.012523482 | 145681 | 1 |
| HSYRA2001003 | 0.019517904 | 107400 | 3 |
| HSYRA2001103 | 0.197559673 | 153858 | 17 |
| HSYRA2001105 | 0.060814345 | 98214 | 19 |
| HSYRA2001138 | 0.657241129 | 12270 | 103 |
| HSYRA2001142 | 0.012523482 | 262427 | 1 |
| HSYRA2001153 | 0.01832866 | 182301 | 2 |
| HSYRA2001225 | 0.226655906 | 146100 | 20 |
| HSYRA2001232 | 0.043312461 | 77421 | 5 |
| HSYRA2001255 | 0.012523482 | 283297 | 1 |
| HSYRA2001332 | 0.012523482 | 13018 | 1 |
| HSYRA2001353 | 0.033022593 | 183425 | 5 |
| HSYRA2001396 | 0.110290147 | 51644 | 21 |
| HSYRA2001420 | 0.030072353 | 128094 | 5 |
| HSYRA2001476 | 0.347074026 | 12734 | 58 |
| HSYRA2001484 | 0.018565048 | 223669 | 2 |
| HSYRA2001552 | 0.012523482 | 57036 | 1 |
| HSYRA2001567 | 0.074766449 | 134837 | 16 |
| HSYRA2001574 | 0.222687322 | 104873 | 21 |
| HSYRA2001580 | 0.030571812 | 184490 | 5 |
| HSYRA2001595 | 0.294133599 | 58519 | 49 |
| HSYRA2001615 | 0.080792807 | 111771 | 25 |
| HSYRA2001621 | 0.913125636 | 8427 | 57 |
| HSYRA2001631 | 0.028735737 | 18844 | 6 |
| HSYRA2001638 | 0.074083011 | 100148 | 16 |
| HSYRA2001983 | 0.034716479 | 45554 | 7 |
| HSYRA2002103 | 0.012523482 | 209450 | 1 |
| HSYRA2002590 | 0.012523482 | 221431 | 1 |
| HSYRA2002741 | 0.023810163 | 186938 | 2 |
| HSYRA2003168 | 0.012523482 | 12852 | 1 |
| HSYRA2004550 | 0.012523482 | 225056 | 1 |
| HSYRA2004858 | 0.012523482 | 202723 | 1 |
| HSYRA2005456 | 0.012523482 | 132446 | 1 |
| HSYRA2005496 | 0.012523482 | 91848 | 1 |
| HSYRA2005628 | 0.024983801 | 108705 | 3 |
| HSYRA2005970 | 0.012523482 | 212951 | 1 |
| HSYRA2006049 | 0.024745432 | 209870 | 2 |
| HSYRA2006873 | 0.012523482 | 135852 | 1 |
| HSYRA2007241 | 0.087372584 | 113081 | 10 |
| HSYRA2007338 | 0.012523482 | 184145 | 1 |
| HSYRA2007650 | 0.035226902 | 144430 | 6 |
| HSYRA2007667 | 0.018736591 | 60166 | 2 |
| HSYRA2008154 | 0.012523482 | 260439 | 1 |
| HSYRA2008376 | 0.107159275 | 79132 | 20 |
| HSYRA2008714 | 0.012523482 | 109472 | 1 |
| HSYRA2009075 | 0.139291562 | 132443 | 11 |
| HSYRA2009102 | 0.012523482 | 101249 | 1 |
| IMR321000102 | 0.122740821 | 55164 | 35 |
| IMR321000155 | 0.005912262 | 17127 | 1 |
| IMR321000158 | 0.220123573 | 66433 | 33 |
| IMR321000161 | 0.007309417 | 58575 | 2 |
| IMR321000165 | 0.005912262 | 281603 | 1 |
| IMR321000193 | 0.038175412 | 27719 | 6 |
| IMR321000207 | 0.011984645 | 62926 | 2 |
| IMR321000210 | 0.165308284 | 33089 | 32 |
| IMR321000219 | 0.596981765 | 61702 | 163 |
| IMR321000230 | 0.005912262 | 48009 | 1 |
| IMR321000242 | 0.029795622 | 51438 | 10 |
| IMR321000253 | 0.164511759 | 49580 | 16 |
| IMR321000266 | 0.059209506 | 54334 | 16 |
| IMR322000010 | 0.017699155 | 204132 | 2 |
| IMR322000072 | 0.208825484 | 101999 | 18 |
| IMR322000107 | 0.005912262 | 271654 | 1 |
| IMR322000121 | 0.150306421 | 120136 | 21 |
| IMR322000127 | 0.10816545 | 104430 | 34 |
| IMR322000144 | 0.102727316 | 142314 | 11 |
| IMR322000152 | 0.020683373 | 69863 | 3 |
| IMR322000164 | 0.005912262 | 269073 | 1 |
| IMR322000178 | 0.090092024 | 51443 | 6 |
| IMR322000223 | 0.246099782 | 72126 | 32 |
| IMR322000243 | 0.195299125 | 9027 | 34 |
| IMR322000302 | 0.028243819 | 145027 | 5 |
| IMR322000316 | 0.403797085 | 70462 | 140 |
| IMR322000374 | 0.030418644 | 114705 | 4 |
| IMR322000379 | 0.005912262 | 270234 | 1 |
| IMR322000383 | 0.005912262 | 92544 | 1 |
| IMR322000418 | 0.005912262 | 269496 | 1 |
| IMR322000518 | 0.054556436 | 120334 | 12 |
| IMR322000555 | 0.028085634 | 122224 | 8 |
| IMR322000604 | 0.007587809 | 95204 | 2 |
| IMR322000609 | 0.007587809 | 236913 | 2 |
| IMR322000656 | 0.005912262 | 83674 | 1 |
| IMR322000672 | 0.005912262 | 274780 | 1 |
| IMR322000730 | 1.222498247 | 29663 | 86 |
| IMR322000742 | 0.10118721 | 131842 | 13 |
| IMR322000775 | 0.040412625 | 71255 | 10 |
| IMR322000791 | 0.032295588 | 164874 | 4 |
| IMR322000811 | 0.011854386 | 56634 | 2 |
| IMR322000819 | 0.039310974 | 181455 | 5 |
| IMR322000838 | 0.007770376 | 153601 | 2 |
| IMR322000859 | 0.008290751 | 88163 | 3 |
| IMR322000863 | 0.024838293 | 36361 | 5 |
| IMR322000917 | 0.014184766 | 166215 | 4 |
| IMR322000918 | 0.057162195 | 124283 | 12 |
| IMR322000919 | 0.005912262 | 58220 | 1 |
| IMR322000935 | 0.111914818 | 130132 | 20 |
| IMR322000953 | 0.005912262 | 188928 | 1 |
| IMR322000973 | 0.207800666 | 168676 | 13 |
| IMR322000984 | 0.005912262 | 142377 | 1 |
| IMR322001018 | 0.005912262 | 269611 | 1 |
| IMR322001049 | 0.028924778 | 35412 | 7 |
| IMR322001167 | 0.005912262 | 102010 | 1 |
| IMR322001185 | 0.011953828 | 235571 | 2 |
| IMR322001218 | 0.149668632 | 174073 | 11 |
| IMR322001245 | 0.005912262 | 178089 | 1 |
| IMR322001317 | 0.212184704 | 99451 | 32 |
| IMR322001318 | 0.007600539 | 176795 | 2 |
| IMR322001322 | 0.007600539 | 260831 | 2 |
| IMR322001332 | 0.062933101 | 149208 | 14 |
| IMR322001380 | 0.084524886 | 101282 | 25 |
| IMR322001435 | 0.178350114 | 42954 | 43 |
| IMR322001438 | 0.020163157 | 31743 | 3 |
| IMR322001452 | 0.005912262 | 266043 | 1 |
| IMR322001491 | 0.017376208 | 145174 | 2 |
| IMR322001534 | 0.020278088 | 171468 | 4 |
| IMR322001541 | 0.099046094 | 141041 | 20 |
| IMR322001584 | 0.134229588 | 117797 | 21 |
| IMR322001600 | 1.040602874 | 60974 | 67 |
| IMR322001665 | 0.005912262 | 160858 | 1 |
| IMR322001670 | 0.036727557 | 165836 | 12 |
| IMR322001710 | 0.086393499 | 60954 | 22 |
| IMR322001724 | 0.005912262 | 275502 | 1 |
| IMR322001879 | 0.017385434 | 128107 | 6 |
| IMR322002035 | 0.009263356 | 142001 | 3 |
| IMR322002110 | 0.005912262 | 219548 | 1 |
| IMR322002470 | 0.005912262 | 241286 | 1 |
| IMR322002660 | 0.012371582 | 138479 | 4 |
| IMR322002760 | 0.031464993 | 141357 | 7 |
| IMR322002993 | 0.010764599 | 176882 | 4 |
| IMR322003675 | 0.005912262 | 201098 | 1 |
| IMR322004768 | 0.005912262 | 258479 | 1 |
| IMR322005293 | 0.005912262 | 116468 | 1 |
| IMR322005833 | 0.005912262 | 239102 | 1 |
| IMR322006012 | 0.005912262 | 244876 | 1 |
| IMR322006222 | 0.005912262 | 218415 | 1 |
| IMR322006495 | 0.005912262 | 158814 | 1 |
| IMR322006520 | 0.005912262 | 49736 | 1 |
| IMR322006886 | 0.005912262 | 31507 | 1 |
| IMR322006947 | 0.018134213 | 227599 | 2 |
| IMR322007078 | 0.005912262 | 240831 | 1 |
| IMR322007225 | 0.005912262 | 275698 | 1 |
| IMR322007704 | 0.005912262 | 126059 | 1 |
| IMR322008651 | 0.005912262 | 161927 | 1 |
| IMR322009710 | 0.007101507 | 169728 | 2 |
| IMR322009807 | 0.005912262 | 209191 | 1 |
| IMR322010295 | 0.005912262 | 227571 | 1 |
| IMR322010953 | 0.011824524 | 220657 | 2 |
| IMR322011659 | 0.039310974 | 181455 | 5 |
| IMR322011689 | 0.005912262 | 271209 | 1 |
| IMR322012314 | 0.005912262 | 177940 | 1 |
| IMR322012529 | 0.035581801 | 120080 | 7 |
| IMR322013053 | 0.005912262 | 278083 | 1 |
| IMR322013396 | 0.005912262 | 136777 | 1 |
| IMR322013731 | 0.005912262 | 204384 | 1 |
| IMR322013805 | 0.012403885 | 118755 | 5 |
| IMR322015523 | 0.005912262 | 169120 | 1 |
| IMR322016146 | 0.005912262 | 65914 | 1 |
| IMR322017049 | 0.005912262 | 168010 | 1 |
| IMR322018117 | 0.005912262 | 63289 | 1 |
| IMR322018192 | 0.005912262 | 2075 | 1 |
| IMR322019070 | 0.062625801 | 98019 | 19 |
| JCMLC2000273 | 0.054217819 | 130058 | 4 |
| KIDNE1000008 | 0.082976718 | 63106 | 9 |
| KIDNE1000012 | 0.005841463 | 60829 | 1 |
| KIDNE1000015 | 0.128512179 | 59968 | 22 |
| KIDNE1000028 | 0.005841463 | 51288 | 1 |
| KIDNE1000036 | 0.007839864 | 55820 | 2 |
| KIDNE1000050 | 0.67004871 | 54853 | 182 |
| KIDNE1000064 | 0.221975583 | 71795 | 38 |
| KIDNE1000104 | 0.01010341 | 46834 | 4 |
| KIDNE1000121 | 0.005841463 | 51641 | 1 |
| KIDNE1000143 | 0.251975035 | 59474 | 28 |
| KIDNE1000145 | 0.06059032 | 58772 | 18 |
| KIDNE1000152 | 0.005841463 | 17567 | 1 |
| KIDNE2000026 | 0.005841463 | 195182 | 1 |
| KIDNE2000041 | 0.005841463 | 97572 | 1 |
| KIDNE2000046 | 0.005841463 | 86608 | 1 |
| KIDNE2000051 | 0.005841463 | 124342 | 1 |
| KIDNE2000070 | 0.017524388 | 121155 | 3 |
| KIDNE2000085 | 0.162944329 | 113039 | 28 |
| KIDNE2000167 | 0.06425609 | 71798 | 11 |
| KIDNE2000174 | 0.005841463 | 257161 | 1 |
| KIDNE2000192 | 0.005841463 | 157877 | 1 |
| KIDNE2000227 | 0.005841463 | 150642 | 1 |
| KIDNE2000244 | 0.005841463 | 104614 | 1 |
| KIDNE2000245 | 0.005841463 | 97983 | 1 |
| KIDNE2000252 | 0.196056856 | 106109 | 53 |
| KIDNE2000266 | 0.039794587 | 79211 | 15 |
| KIDNE2000287 | 0.005841463 | 260665 | 1 |
| KIDNE2000315 | 0.024536653 | 128325 | 4 |
| KIDNE2000317 | 0.011777238 | 218754 | 2 |
| KIDNE2000328 | 0.005841463 | 65974 | 1 |
| KIDNE2000330 | 0.049079159 | 141919 | 4 |
| KIDNE2000335 | 0.005841463 | 231117 | 1 |
| KIDNE2000349 | 0.264932664 | 136134 | 21 |
| KIDNE2000375 | 0.005841463 | 103964 | 1 |
| KIDNE2000383 | 0.034022867 | 63503 | 11 |
| KIDNE2000394 | 0.011682925 | 141243 | 2 |
| KIDNE2000403 | 0.202819655 | 84424 | 25 |
| KIDNE2000422 | 0.00751701 | 249935 | 2 |
| KIDNE2000493 | 0.005841463 | 180462 | 1 |
| KIDNE2000497 | 0.05343476 | 225318 | 6 |
| KIDNE2000510 | 0.005841463 | 237100 | 1 |
| KIDNE2000513 | 0.005841463 | 97569 | 1 |
| KIDNE2000517 | 0.106986654 | 162158 | 19 |
| KIDNE2000519 | 0.017524388 | 126722 | 3 |
| KIDNE2000543 | 0.01940123 | 167445 | 4 |
| KIDNE2000555 | 0.005841463 | 212748 | 1 |
| KIDNE2000574 | 0.223844312 | 89428 | 32 |
| KIDNE2000624 | 0.005841463 | 112536 | 1 |
| KIDNE2000665 | 0.086555664 | 55127 | 11 |
| KIDNE2000678 | 0.005841463 | 93275 | 1 |
| KIDNE2000717 | 0.005841463 | 265465 | 1 |
| KIDNE2000721 | 0.007922975 | 114165 | 2 |
| KIDNE2000722 | 0.005841463 | 261395 | 1 |
| KIDNE2000777 | 0.087805408 | 138411 | 10 |
| KIDNE2000801 | 0.005841463 | 151482 | 1 |
| KIDNE2000832 | 0.053769404 | 112555 | 4 |
| KIDNE2000833 | 0.023258906 | 191590 | 3 |
| KIDNE2000846 | 0.011682925 | 143372 | 2 |
| KIDNE2000867 | 0.005841463 | 165319 | 1 |
| KIDNE2000909 | 0.081614836 | 120033 | 13 |
| KIDNE2000947 | 0.066295682 | 117622 | 17 |
| KIDNE2001117 | 0.00751701 | 208576 | 2 |
| KIDNE2001140 | 0.005841463 | 102580 | 1 |
| KIDNE2001162 | 0.358985708 | 38426 | 50 |
| KIDNE2001269 | 0.005841463 | 247228 | 1 |
| KIDNE2001361 | 0.007238618 | 113213 | 2 |
| KIDNE2001373 | 0.023517396 | 169434 | 3 |
| KIDNE2001467 | 0.642849001 | 82298 | 141 |
| KIDNE2001636 | 0.035458216 | 164793 | 9 |
| KIDNE2001713 | 0.0108138 | 159342 | 3 |
| KIDNE2001802 | 0.005841463 | 195821 | 1 |
| KIDNE2001840 | 0.005841463 | 44088 | 1 |
| KIDNE2001847 | 0.046041714 | 167204 | 4 |
| KIDNE2001857 | 0.005841463 | 59401 | 1 |
| KIDNE2001873 | 0.005841463 | 93271 | 1 |
| KIDNE2001897 | 0.005841463 | 136978 | 1 |
| KIDNE2001931 | 0.005841463 | 3831 | 1 |
| KIDNE2001979 | 0.028439911 | 103544 | 5 |
| KIDNE2002015 | 0.005841463 | 9882 | 1 |
| KIDNE2002162 | 0.005841463 | 147142 | 1 |
| KIDNE2002168 | 0.005841463 | 57156 | 1 |
| KIDNE2002191 | 0.0235069 | 528 | 9 |
| KIDNE2002198 | 0.005841463 | 154733 | 1 |
| KIDNE2002252 | 0.076863312 | 110087 | 22 |
| KIDNE2002262 | 0.005841463 | 144824 | 1 |
| KIDNE2002265 | 0.092128348 | 151505 | 13 |
| KIDNE2002438 | 0.008936959 | 82164 | 2 |
| KIDNE2002483 | 0.005841463 | 177668 | 1 |
| KIDNE2002725 | 0.016285294 | 139242 | 6 |
| KIDNE2002768 | 0.005841463 | 241326 | 1 |
| KIDNE2002795 | 0.005841463 | 76723 | 1 |
| KIDNE2002798 | 0.013845103 | 96858 | 3 |
| KIDNE2002839 | 0.022824821 | 65999 | 3 |
| KIDNE2002845 | 0.005841463 | 280387 | 1 |
| KIDNE2002872 | 0.29304986 | 181809 | 10 |
| KIDNE2002882 | 0.020365791 | 98344 | 2 |
| KIDNE2002883 | 0.073875916 | 121295 | 13 |
| KIDNE2002980 | 0.05316418 | 150533 | 3 |
| KIDNE2002991 | 0.005841463 | 237472 | 1 |
| KIDNE2003185 | 0.005841463 | 237586 | 1 |
| KIDNE2003188 | 0.005841463 | 59402 | 1 |
| KIDNE2003233 | 0.007030707 | 145793 | 2 |
| KIDNE2003305 | 0.199074898 | 136785 | 25 |
| KIDNE2003316 | 0.022146317 | 150501 | 4 |
| KIDNE2003335 | 0.005841463 | 241449 | 1 |
| KIDNE2003357 | 0.011332196 | 163340 | 3 |
| KIDNE2003373 | 0.0108138 | 109563 | 3 |
| KIDNE2003377 | 0.005841463 | 56207 | 1 |
| KIDNE2003507 | 0.049489618 | 170414 | 4 |
| KIDNE2003752 | 0.005841463 | 247329 | 1 |
| KIDNE2003764 | 0.015554722 | 99361 | 5 |
| KIDNE2003837 | 0.005841463 | 230814 | 1 |
| KIDNE2003941 | 0.332198807 | 88220 | 26 |
| KIDNE2003994 | 0.020779955 | 160572 | 3 |
| KIDNE2004034 | 0.005841463 | 164706 | 1 |
| KIDNE2004054 | 0.012835885 | 91568 | 3 |
| KIDNE2004084 | 0.005841463 | 155885 | 1 |
| KIDNE2004095 | 0.005841463 | 111437 | 1 |
| KIDNE2004115 | 0.007030707 | 94365 | 2 |
| KIDNE2004233 | 0.005841463 | 252100 | 1 |
| KIDNE2004262 | 0.005841463 | 147843 | 1 |
| KIDNE2004279 | 0.005841463 | 218219 | 1 |
| KIDNE2004294 | 0.005841463 | 164772 | 1 |
| KIDNE2004295 | 0.026245245 | 217115 | 5 |
| KIDNE2004305 | 0.008936959 | 16862 | 2 |
| KIDNE2004344 | 0.007238618 | 58414 | 2 |
| KIDNE2004394 | 0.005841463 | 246820 | 1 |
| KIDNE2004411 | 0.019662533 | 132354 | 5 |
| KIDNE2004475 | 0.065093671 | 157276 | 17 |
| KIDNE2004519 | 0.005841463 | 137793 | 1 |
| KIDNE2004520 | 0.005841463 | 246543 | 1 |
| KIDNE2004531 | 0.007238618 | 102911 | 2 |
| KIDNE2004534 | 0.044889885 | 141678 | 9 |
| KIDNE2004562 | 0.005841463 | 246808 | 1 |
| KIDNE2004579 | 0.005841463 | 255164 | 1 |
| KIDNE2004626 | 0.013000357 | 79706 | 3 |
| KIDNE2004681 | 0.005841463 | 238002 | 1 |
| KIDNE2004828 | 0.023365851 | 170845 | 4 |
| KIDNE2004846 | 0.025403278 | 34738 | 2 |
| KIDNE2004864 | 0.017161365 | 106686 | 5 |
| KIDNE2004879 | 0.005841463 | 89625 | 1 |
| KIDNE2004981 | 0.005841463 | 224981 | 1 |
| KIDNE2004985 | 0.005841463 | 224983 | 1 |
| KIDNE2004999 | 0.005841463 | 230960 | 1 |
| KIDNE2005038 | 0.005841463 | 238153 | 1 |
| KIDNE2005042 | 0.020811331 | 129777 | 7 |
| KIDNE2005062 | 0.005841463 | 224984 | 1 |
| KIDNE2005266 | 0.005841463 | 157721 | 1 |
| KIDNE2005296 | 0.005841463 | 183236 | 1 |
| KIDNE2005321 | 0.016957128 | 149152 | 4 |
| KIDNE2005327 | 0.029207314 | 194363 | 5 |
| KIDNE2005336 | 0.010018398 | 186730 | 2 |
| KIDNE2005400 | 0.005841463 | 244905 | 1 |
| KIDNE2005424 | 0.005841463 | 84682 | 1 |
| KIDNE2005477 | 0.005841463 | 166982 | 1 |
| KIDNE2005526 | 0.005841463 | 136844 | 1 |
| KIDNE2005543 | 0.009409037 | 39520 | 3 |
| KIDNE2005603 | 0.005841463 | 81671 | 1 |
| KIDNE2005629 | 0.023365851 | 116682 | 4 |
| KIDNE2005676 | 0.119099198 | 43143 | 45 |
| KIDNE2005781 | 0.005841463 | 36705 | 1 |
| KIDNE2005798 | 0.005841463 | 218140 | 1 |
| KIDNE2005854 | 0.005841463 | 138208 | 1 |
| KIDNE2005908 | 0.005841463 | 72328 | 1 |
| KIDNE2005937 | 0.030916774 | 133122 | 6 |
| KIDNE2006009 | 0.045448317 | 154557 | 10 |
| KIDNE2006014 | 0.056429638 | 149434 | 15 |
| KIDNE2006030 | 0.07851576 | 34026 | 25 |
| KIDNE2006039 | 0.010614434 | 124421 | 3 |
| KIDNE2006053 | 0.011682925 | 144826 | 2 |
| KIDNE2006062 | 0.059610867 | 165627 | 5 |
| KIDNE2006092 | 0.005841463 | 228204 | 1 |
| KIDNE2006145 | 0.005841463 | 258040 | 1 |
| KIDNE2006149 | 0.005841463 | 90614 | 1 |
| KIDNE2006210 | 0.023904876 | 178787 | 3 |
| KIDNE2006248 | 0.538918881 | 20243 | 49 |
| KIDNE2006299 | 0.005841463 | 109245 | 1 |
| KIDNE2006334 | 0.005841463 | 235919 | 1 |
| KIDNE2006353 | 0.042844954 | 118025 | 23 |
| KIDNE2006376 | 0.005841463 | 226494 | 1 |
| KIDNE2006378 | 0.005841463 | 162293 | 1 |
| KIDNE2006465 | 0.054150435 | 136539 | 21 |
| KIDNE2006580 | 0.023891517 | 88235 | 10 |
| KIDNE2006652 | 0.01940123 | 167445 | 4 |
| KIDNE2006687 | 0.005841463 | 95104 | 1 |
| KIDNE2006733 | 0.005841463 | 171215 | 1 |
| KIDNE2006760 | 0.024838834 | 152670 | 6 |
| KIDNE2006775 | 0.091455179 | 114362 | 13 |
| KIDNE2006811 | 0.051490468 | 101052 | 7 |
| KIDNE2006820 | 0.005841463 | 52561 | 1 |
| KIDNE2006880 | 0.052190513 | 81038 | 6 |
| KIDNE2007005 | 0.00875632 | 182657 | 2 |
| KIDNE2007040 | 0.007839864 | 219935 | 2 |
| KIDNE2007077 | 0.005841463 | 178790 | 1 |
| KIDNE2007186 | 0.00875632 | 141775 | 2 |
| KIDNE2007222 | 0.005841463 | 183153 | 1 |
| KIDNE2007328 | 0.189383507 | 101133 | 45 |
| KIDNE2007352 | 0.40306683 | 59267 | 83 |
| KIDNE2007356 | 0.011783586 | 252070 | 2 |
| KIDNE2007422 | 0.005841463 | 153769 | 1 |
| KIDNE2007569 | 0.011682925 | 152303 | 2 |
| KIDNE2007810 | 0.017524388 | 164707 | 3 |
| KIDNE2007811 | 0.019557654 | 170075 | 4 |
| KIDNE2007910 | 0.018488773 | 147451 | 4 |
| KIDNE2007944 | 0.016668661 | 132220 | 2 |
| KIDNE2007954 | 0.079484867 | 8648 | 23 |
| KIDNE2008022 | 0.006936741 | 41638 | 2 |
| KIDNE2008048 | 0.312345136 | 76422 | 86 |
| KIDNE2008069 | 0.14355133 | 99721 | 37 |
| KIDNE2008072 | 0.005841463 | 259485 | 1 |
| KIDNE2008116 | 0.144417246 | 32354 | 16 |
| KIDNE2008117 | 0.005841463 | 107715 | 1 |
| KIDNE2008218 | 0.005841463 | 262493 | 1 |
| KIDNE2008315 | 0.011682925 | 164689 | 2 |
| KIDNE2008362 | 0.036695152 | 36442 | 9 |
| KIDNE2008378 | 0.005841463 | 250677 | 1 |
| KIDNE2008403 | 0.123204427 | 89019 | 69 |
| KIDNE2008404 | 0.036134804 | 85611 | 4 |
| KIDNE2008446 | 0.005841463 | 89873 | 1 |
| KIDNE2008473 | 0.005841463 | 169529 | 1 |
| KIDNE2008480 | 0.005841463 | 261199 | 1 |
| KIDNE2008649 | 0.19005511 | 113533 | 33 |
| KIDNE2008666 | 0.00751701 | 261141 | 2 |
| KIDNE2008697 | 0.005841463 | 283080 | 1 |
| KIDNE2008758 | 0.007699577 | 198464 | 2 |
| KIDNE2008788 | 0.005841463 | 260591 | 1 |
| KIDNE2008795 | 0.005841463 | 260590 | 1 |
| KIDNE2008824 | 0.005841463 | 190329 | 1 |
| KIDNE2008832 | 0.005841463 | 92562 | 1 |
| KIDNE2008869 | 0.005841463 | 246472 | 1 |
| KIDNE2008914 | 0.009632435 | 140499 | 3 |
| KIDNE2008975 | 0.005841463 | 157597 | 1 |
| KIDNE2008987 | 0.058166891 | 36264 | 5 |
| KIDNE2009019 | 0.007922975 | 74230 | 2 |
| KIDNE2009109 | 0.005841463 | 192992 | 1 |
| KIDNE2009180 | 0.005841463 | 139249 | 1 |
| KIDNE2009191 | 0.005841463 | 39975 | 1 |
| KIDNE2009332 | 0.008886344 | 226666 | 2 |
| KIDNE2009367 | 0.222316502 | 89088 | 106 |
| KIDNE2009426 | 0.005841463 | 265033 | 1 |
| KIDNE2009467 | 0.005841463 | 101240 | 1 |
| KIDNE2009490 | 0.005841463 | 17577 | 1 |
| KIDNE2009553 | 0.005841463 | 253927 | 1 |
| KIDNE2009605 | 0.011095134 | 139982 | 4 |
| KIDNE2009628 | 0.005841463 | 159310 | 1 |
| KIDNE2009647 | 0.005841463 | 237873 | 1 |
| KIDNE2009676 | 0.005841463 | 98857 | 1 |
| KIDNE2009723 | 0.005841463 | 102830 | 1 |
| KIDNE2010007 | 0.005841463 | 44082 | 1 |
| KIDNE2010049 | 0.048733552 | 171247 | 5 |
| KIDNE2010052 | 0.005841463 | 186001 | 1 |
| KIDNE2010084 | 0.005841463 | 143367 | 1 |
| KIDNE2010137 | 0.005841463 | 274099 | 1 |
| KIDNE2010151 | 0.005841463 | 59998 | 1 |
| KIDNE2010264 | 0.005841463 | 143393 | 1 |
| KIDNE2010265 | 0.070921038 | 117677 | 7 |
| KIDNE2010271 | 0.005841463 | 162997 | 1 |
| KIDNE2010419 | 0.005841463 | 82090 | 1 |
| KIDNE2010430 | 0.017570502 | 149556 | 5 |
| KIDNE2010574 | 0.005841463 | 171107 | 1 |
| KIDNE2010674 | 0.005841463 | 153297 | 1 |
| KIDNE2010739 | 0.005841463 | 34571 | 1 |
| KIDNE2010750 | 0.015731596 | 132621 | 5 |
| KIDNE2010762 | 0.005841463 | 151481 | 1 |
| KIDNE2010863 | 0.005841463 | 201466 | 1 |
| KIDNE2010973 | 0.005841463 | 143373 | 1 |
| KIDNE2010989 | 0.005841463 | 167520 | 1 |
| KIDNE2011200 | 0.005841463 | 107238 | 1 |
| KIDNE2011314 | 0.007699577 | 101352 | 2 |
| KIDNE2011508 | 0.008962269 | 132593 | 2 |
| KIDNE2011532 | 0.005841463 | 177530 | 1 |
| KIDNE2011635 | 0.012043782 | 2290 | 2 |
| KIDNE2011752 | 0.007030707 | 157537 | 2 |
| KIDNE2011782 | 0.005841463 | 150504 | 1 |
| KIDNE2011858 | 0.005841463 | 81046 | 1 |
| KIDNE2012009 | 0.017602018 | 168369 | 2 |
| KIDNE2012025 | 0.071403765 | 53754 | 4 |
| KIDNE2012188 | 0.01876609 | 179530 | 4 |
| KIDNE2012291 | 0.007030707 | 197105 | 2 |
| KIDNE2012316 | 0.009879746 | 160552 | 2 |
| KIDNE2012361 | 0.011682925 | 179550 | 2 |
| KIDNE2012440 | 0.094659283 | 30801 | 14 |
| KIDNE2012453 | 0.005841463 | 179558 | 1 |
| KIDNE2012563 | 0.005841463 | 186041 | 1 |
| KIDNE2012601 | 0.005841463 | 162359 | 1 |
| KIDNE2012613 | 0.005841463 | 181549 | 1 |
| KIDNE2012710 | 0.017694873 | 153418 | 6 |
| KIDNE2012745 | 0.005841463 | 29961 | 1 |
| KIDNE2012775 | 0.005841463 | 171433 | 1 |
| KIDNE2012784 | 0.009838265 | 143652 | 3 |
| KIDNE2012945 | 0.011986989 | 162368 | 2 |
| KIDNE2013045 | 0.005841463 | 243287 | 1 |
| KIDNE2013095 | 0.005841463 | 152577 | 1 |
| KIDNE2013158 | 0.009237019 | 118760 | 3 |
| KIDNE2013218 | 0.005841463 | 143115 | 1 |
| KIDNE2013263 | 0.005841463 | 164843 | 1 |
| KIDNE2013346 | 0.008032019 | 13633 | 3 |
| KIDNE2013388 | 0.005841463 | 56929 | 1 |
| KIDNE2013413 | 0.008936959 | 170067 | 2 |
| KIDNE2013489 | 0.005841463 | 105329 | 1 |
| KIDNE2013639 | 0.005841463 | 279995 | 1 |
| KIDNE2013731 | 0.005841463 | 87918 | 1 |
| KIDNE2013734 | 0.005841463 | 162160 | 1 |
| KIDNE2013775 | 0.005841463 | 85851 | 1 |
| KIDNE2013801 | 0.005841463 | 153265 | 1 |
| KIDNE2013845 | 0.149912658 | 2957 | 39 |
| KIDNE2013866 | 0.006936741 | 123374 | 2 |
| KIDNE2014087 | 0.005841463 | 119807 | 1 |
| KIDNE2014112 | 0.005841463 | 167563 | 1 |
| KIDNE2014119 | 0.011754074 | 171320 | 2 |
| KIDNE2014170 | 0.005841463 | 197149 | 1 |
| KIDNE2014184 | 0.005841463 | 48528 | 1 |
| KIDNE2014268 | 0.005841463 | 164446 | 1 |
| KIDNE2014290 | 0.239007391 | 94988 | 60 |
| KIDNE2014298 | 0.005841463 | 103908 | 1 |
| KIDNE2014320 | 0.007699577 | 72871 | 2 |
| KIDNE2014325 | 0.044292984 | 76099 | 8 |
| KIDNE2014415 | 0.005841463 | 92025 | 1 |
| KIDNE2014489 | 0.005841463 | 185937 | 1 |
| KIDNE2014496 | 0.005841463 | 162202 | 1 |
| KIDNE2014661 | 0.005841463 | 55801 | 1 |
| KIDNE2014717 | 0.005841463 | 163430 | 1 |
| KIDNE2014789 | 0.005841463 | 82635 | 1 |
| KIDNE2014808 | 0.007718304 | 126721 | 2 |
| KIDNE2014890 | 0.005841463 | 137416 | 1 |
| KIDNE2014978 | 0.007529739 | 190402 | 2 |
| KIDNE2015073 | 0.005841463 | 127158 | 1 |
| KIDNE2015221 | 0.005841463 | 86813 | 1 |
| KIDNE2015244 | 0.005841463 | 94431 | 1 |
| KIDNE2015313 | 0.005841463 | 104623 | 1 |
| KIDNE2015433 | 0.018962576 | 52811 | 6 |
| KIDNE2015445 | 0.04877164 | 91753 | 9 |
| KIDNE2015483 | 0.005841463 | 172844 | 1 |
| KIDNE2015515 | 0.005841463 | 91705 | 1 |
| KIDNE2015546 | 0.012043782 | 141220 | 2 |
| KIDNE2015556 | 0.011682925 | 162166 | 2 |
| KIDNE2015598 | 0.007238618 | 69821 | 2 |
| KIDNE2015660 | 0.005841463 | 14900 | 1 |
| KIDNE2015710 | 0.089512404 | 152846 | 4 |
| KIDNE2015987 | 0.005841463 | 59983 | 1 |
| KIDNE2016000 | 0.005841463 | 62068 | 1 |
| KIDNE2016036 | 0.005841463 | 154599 | 1 |
| KIDNE2016096 | 0.005841463 | 143374 | 1 |
| KIDNE2016101 | 0.007030707 | 139888 | 2 |
| KIDNE2016142 | 0.005841463 | 59793 | 1 |
| KIDNE2016188 | 0.005841463 | 185453 | 1 |
| KIDNE2016327 | 0.009576419 | 223464 | 3 |
| KIDNE2016371 | 0.018597235 | 3640 | 7 |
| KIDNE2016388 | 0.005841463 | 141789 | 1 |
| KIDNE2016464 | 0.005841463 | 211929 | 1 |
| KIDNE2016539 | 0.011753725 | 132849 | 2 |
| KIDNE2016570 | 0.006936741 | 167578 | 2 |
| KIDNE2016918 | 0.005841463 | 59999 | 1 |
| KIDNE2017007 | 0.005841463 | 224923 | 1 |
| KIDNE2017040 | 0.049828212 | 152509 | 11 |
| KIDNE2017052 | 0.007238618 | 211940 | 2 |
| KIDNE2017151 | 0.005841463 | 204719 | 1 |
| KIDNE2017153 | 0.007030707 | 218663 | 2 |
| KIDNE2017315 | 0.005841463 | 116673 | 1 |
| KIDNE2017332 | 0.005841463 | 130116 | 1 |
| KIDNE2017343 | 0.050732526 | 101406 | 10 |
| KIDNE2017454 | 0.005841463 | 284919 | 1 |
| KIDNE2017603 | 0.005841463 | 230111 | 1 |
| KIDNE2017956 | 0.005841463 | 146072 | 1 |
| KIDNE2018071 | 0.011895831 | 234734 | 2 |
| KIDNE2018166 | 0.005841463 | 192198 | 1 |
| KIDNE2018167 | 0.005841463 | 156167 | 1 |
| KIDNE2018217 | 0.027611917 | 156173 | 5 |
| KIDNE2018254 | 0.005841463 | 215295 | 1 |
| KIDNE2018268 | 0.005841463 | 281773 | 1 |
| KIDNE2018269 | 0.005841463 | 268983 | 1 |
| KIDNE2018287 | 0.005841463 | 107290 | 1 |
| KIDNE2018294 | 0.005841463 | 154541 | 1 |
| KIDNE2018317 | 0.006936741 | 145953 | 2 |
| KIDNE2018352 | 0.046986847 | 151862 | 14 |
| KIDNE2018462 | 0.005841463 | 104999 | 1 |
| KIDNE2018493 | 0.047023149 | 102937 | 12 |
| KIDNE2018617 | 0.005841463 | 22470 | 1 |
| KIDNE2018678 | 0.005841463 | 170406 | 1 |
| KIDNE2018727 | 0.005841463 | 59992 | 1 |
| KIDNE2018863 | 0.005841463 | 198380 | 1 |
| KIDNE2018891 | 0.027475964 | 70931 | 8 |
| KIDNE2018989 | 0.005841463 | 49498 | 1 |
| KIDNE2018996 | 0.005841463 | 186076 | 1 |
| KIDNE2019074 | 0.005841463 | 170278 | 1 |
| KIDNE2019187 | 0.005841463 | 158649 | 1 |
| LIVER1000017 | 1.127224016 | 55076 | 70 |
| LIVER1000050 | 0.014524328 | 63808 | 1 |
| LIVER1000055 | 0.014524328 | 80879 | 1 |
| LIVER1000058 | 0.014524328 | 87072 | 1 |
| LIVER1000063 | 0.014524328 | 74134 | 1 |
| LIVER1000067 | 0.241381518 | 45313 | 14 |
| LIVER1000073 | 0.014524328 | 53701 | 1 |
| LIVER1000079. | 0.095625863 | 7710 | 18 |
| LIVER1000082 | 0.014524328 | 69867 | 1 |
| LIVER1000099 | 0.014524328 | 70122 | 1 |
| LIVER1000104 | 0.813015278 | 44077 | 87 |
| LIVER1000111 | 0.026531826 | 79172 | 3 |
| LIVER1000126 | 0.021855964 | 77137 | 3 |
| LIVER1000132 | 0.334841432 | 108373 | 38 |
| LIVER1000139 | 0.014524328 | 23707 | 1 |
| LIVER1000155 | 0.014524328 | 33623 | 1 |
| LIVER1000165 | 0.014524328 | 50213 | 1 |
| LIVER1000175 | 0.014524328 | 76038 | 1 |
| LIVER1000224 | 0.029048656 | 137479 | 2 |
| LIVER1000230 | 0.028892369 | 52005 | 6 |
| LIVER1000278 | 0.014524328 | 259488 | 1 |
| LIVER1000303 | 0.018715794 | 87074 | 4 |
| LIVER1000397 | 0.014524328 | 260977 | 1 |
| LIVER1000421 | 0.02043694 | 7985 | 2 |
| LIVER1000433 | 0.014524328 | 186415 | 1 |
| LIVER1000479 | 0.014524328 | 196881 | 1 |
| LIVER1000482 | 0.014524328 | 66532 | 1 |
| LIVER1000542 | 0.509239555 | 109803 | 51 |
| LIVER2000003 | 0.014524328 | 265627 | 1 |
| LIVER2000033 | 0.121122217 | 151526 | 10 |
| LIVER2000037 | 2.648325881 | 97070 | 100 |
| LIVER2000158 | 0.024233066 | 202224 | 2 |
| LIVER2000216 | 0.014524328 | 1971 | 1 |
| LIVER2000237 | 0.043572985 | 187971 | 3 |
| LIVER2000247 | 0.014524328 | 214426 | 1 |
| LIVER2000360 | 0.014524328 | 233230 | 1 |
| LIVER2000416 | 0.074052587 | 126378 | 8 |
| LIVER2000446 | 0.040733555 | 194458 | 4 |
| LIVER2000515 | 0.043572985 | 219997 | 3 |
| LIVER2000592 | 0.014524328 | 210687 | 1 |
| LIVER2000626 | 0.014524328 | 51706 | 1 |
| LIVER2000682 | 0.014524328 | 168557 | 1 |
| LIVER2000769 | 0.014524328 | 97593 | 1 |
| LIVER2000775 | 0.014524328 | 250788 | 1 |
| LIVER2001051 | 0.014524328 | 98689 | 1 |
| LIVER2001076 | 0.014524328 | 97553 | 1 |
| LIVER2001099 | 0.014524328 | 172545 | 1 |
| LIVER2001113 | 0.015619607 | 152117 | 2 |
| LIVER2001164 | 0.014524328 | 95457 | 1 |
| LIVER2001191 | 0.014524328 | 275525 | 1 |
| LIVER2001265 | 0.016212605 | 124333 | 2 |
| LIVER2001329 | 0.014524328 | 101528 | 1 |
| LIVER2001389 | 0.014524328 | 135987 | 1 |
| LIVER2001461 | 0.014524328 | 182285 | 1 |
| LIVER2001539 | 0.033646648 | 133742 | 7 |
| LIVER2001608 | 0.127587354 | 98471 | 15 |
| LIVER2001835 | 0.020460103 | 163420 | 2 |
| LIVER2002638 | 0.020565894 | 239093 | 2 |
| LIVER2002644 | 0.014524328 | 96584 | 1 |
| LIVER2002660 | 0.014524328 | 164147 | 1 |
| LIVER2002702 | 0.014524328 | 275619 | 1 |
| LIVER2002842 | 0.087770458 | 74323 | 24 |
| LIVER2003008 | 0.016212605 | 246153 | 2 |
| LIVER2003065 | 0.026043738 | 186110 | 2 |
| LIVER2003234 | 0.01652273 | 90853 | 2 |
| LIVER2003511 | 0.020365791 | 156137 | 2 |
| LIVER2003524 | 0.014524328 | 264507 | 1 |
| LIVER2003568 | 0.014524328 | 228163 | 1 |
| LIVER2003581 | 0.050048307 | 48527 | 2 |
| LIVER2003800 | 0.160723159 | 153720 | 5 |
| LIVER2003854 | 0.014524328 | 239331 | 1 |
| LIVER2004074 | 0.014524328 | 204293 | 1 |
| LIVER2004392 | 0.014524328 | 162596 | 1 |
| LIVER2004514 | 0.01640117 | 108285 | 2 |
| LIVER2004565 | 0.014524328 | 160461 | 1 |
| LIVER2005028 | 0.022175912 | 81176 | 3 |
| LIVER2005198 | 0.014524328 | 112292 | 1 |
| LIVER2005218 | 0.067937294 | 98474 | 5 |
| LIVER2005376 | 0.014524328 | 48121 | 1 |
| LIVER2005385 | 0.014524328 | 272916 | 1 |
| LIVER2005512 | 0.014524328 | 97330 | 1 |
| LIVER2005520 | 0.014524328 | 267175 | 1 |
| LIVER2005527 | 0.014524328 | 71251 | 1 |
| LIVER2005544 | 0.014524328 | 58825 | 1 |
| LIVER2005625 | 0.014524328 | 240295 | 1 |
| LIVER2005728 | 0.029048656 | 17782 | 2 |
| LIVER2005789 | 0.186122649 | 168945 | 15 |
| LIVER2005973 | 0.016233788 | 82267 | 2 |
| LIVER2005981 | 0.058518202 | 45805 | 3 |
| LIVER2006006 | 0.235617442 | 51513 | 19 |
| LIVER2006251 | 0.014524328 | 261194 | 1 |
| LIVER2006410 | 0.016382443 | 100019 | 2 |
| LIVER2006469 | 0.014524328 | 11714 | 1 |
| LIVER2006644 | 0.014524328 | 44089 | 1 |
| LIVER2006764 | 0.014524328 | 170276 | 1 |
| LIVER2007415 | 0.014524328 | 118556 | 1 |
| LIVER2007548 | 0.014524328 | 147039 | 1 |
| LIVER2007568 | 0.014524328 | 258669 | 1 |
| LIVER2007721 | 0.014524328 | 138529 | 1 |
| LIVER2007783 | 0.081661418 | 65980 | 10 |
| LIVER2008053 | 0.014524328 | 141188 | 1 |
| LIVER2008465 | 0.025317831 | 117099 | 6 |
| LIVER2008473 | 0.014524328 | 172573 | 1 |
| LIVER2008580 | 0.014524328 | 147325 | 1 |
| LIVER2008706 | 0.037563149 | 186228 | 3 |
| LIVER2008751 | 0.0192973 | 17050 | 3 |
| LIVER2008945 | 0.014524328 | 153028 | 1 |
| LIVER2008998 | 0.014524328 | 151693 | 1 |
| LIVER2009110 | 0.014524328 | 117175 | 1 |
| LIVER2009118 | 0.014524328 | 74451 | 1 |
| LIVER2009554 | 0.014524328 | 132207 | 1 |
| LYMPB1000141 | 0.039118092 | 131764 | 2 |
| LYMPB1000158 | 0.038022814 | 251564 | 1 |
| LYMPB2000083 | 0.038022814 | 103124 | 1 |
| MAMGL1000017 | 0.551111774 | 59524 | 3 |
| MAMGL1000032 | 1.039921212 | 30853 | 46 |
| MAMGL1000035 | 0.832075063 | 17419 | 37 |
| MAMGL1000056 | 0.584454109 | 94457 | 13 |
| MAMGL1000083 | 0.578386106 | 14366 | 20 |
| MAMGL1000096 | 0.556072719 | 57473 | 2 |
| MAMGL1000100 | 0.567554653 | 5632 | 6 |
| MAMGL1000132 | 0.543478261 | 32038 | 1 |
| MAMGL1000151 | 0.543478261 | 68645 | 1 |
| MAMGL1000173 | 0.632159969 | 44347 | 16 |
| MAMGL1000178 | 0.547790273 | 8955 | 3 |
| MAMGL1000182 | 0.543478261 | 37855 | 1 |
| MAMGL1000184 | 0.543478261 | 65284 | 1 |
| MESAN1000032 | 0.008071224 | 21005 | 2 |
| MESAN1000035 | 0.00621311 | 40530 | 1 |
| MESAN1000050 | 0.10437495 | 45654 | 23 |
| MESAN1000079 | 0.011657526 | 64979 | 4 |
| MESAN1000080 | 0.117354162 | 72243 | 12 |
| MESAN1000101 | 0.029028899 | 69446 | 12 |
| MESAN1000121 | 0.00621311 | 50003 | 1 |
| MESAN1000126 | 0.093829107 | 61776 | 20 |
| MESAN1000147 | 0.016341156 | 42348 | 4 |
| MESAN1000180 | 0.00621311 | 23908 | 1 |
| MESAN2000023 | 0.00621311 | 192936 | 1 |
| MESAN2000026 | 0.00621311 | 236371 | 1 |
| MESAN2000067 | 0.009308606 | 143501 | 2 |
| MESAN2000092 | 0.018935193 | 157449 | 7 |
| MESAN2000149 | 0.06820933 | 131401 | 16 |
| MESAN2000167 | 0.00621311 | 115003 | 1 |
| MESAN2000238 | 0.402506729 | 91756 | 67 |
| MESAN2000264 | 0.085027976 | 137712 | 11 |
| MESAN2000267 | 0.409174084 | 50495 | 78 |
| MESAN2000291 | 0.007308388 | 27545 | 2 |
| MESAN2000337 | 0.021131934 | 219460 | 3 |
| MESAN2000434 | 0.007308388 | 139268 | 2 |
| MESAN2000457 | 0.042308733 | 107271 | 12 |
| MESAN2000462 | 0.00621311 | 46273 | 1 |
| MESAN2000501 | 0.055850323 | 204130 | 7 |
| MESAN2000572 | 0.163888782 | 117236 | 41 |
| MESAN2000608 | 0.213075464 | 28732 | 29 |
| MESAN2000620 | 0.009195069 | 252269 | 2 |
| MESAN2000711 | 0.00621311 | 119795 | 1 |
| MESAN2000815 | 0.010731071 | 122318 | 3 |
| MESAN2000894 | 0.008211511 | 136814 | 2 |
| MESAN2000909 | 0.030336812 | 179017 | 5 |
| MESAN2001154 | 0.125760223 | 41668 | 14 |
| MESAN2001450 | 0.058100158 | 59450 | 8 |
| MESAN2001627 | 0.01281038 | 114857 | 4 |
| MESAN2001770 | 0.00621311 | 70800 | 1 |
| MESAN2001979 | 0.012426219 | 144971 | 2 |
| MESAN2001980 | 0.012358636 | 107695 | 2 |
| MESAN2002086 | 0.402506729 | 91756 | 67 |
| MESAN2002113 | 0.00621311 | 87821 | 1 |
| MESAN2002122 | 0.018890012 | 52964 | 8 |
| MESAN2002147 | 0.00621311 | 39642 | 1 |
| MESAN2002186 | 0.076436153 | 92223 | 6 |
| MESAN2002424 | 0.008211511 | 200502 | 2 |
| MESAN2002519 | 0.036128095 | 131882 | 9 |
| MESAN2002687 | 0.00621311 | 192739 | 1 |
| MESAN2002709 | 0.00621311 | 98698 | 1 |
| MESAN2002724 | 0.181696741 | 97935 | 35 |
| MESAN2002790 | 0.068509697 | 87165 | 14 |
| MESAN2002844 | 0.023123682 | 53028 | 7 |
| MESAN2002940 | 0.052685086 | 126143 | 18 |
| MESAN2002978 | 0.00621311 | 254679 | 1 |
| MESAN2003035 | 0.023379497 | 148936 | 7 |
| MESAN2003037 | 0.00621311 | 85891 | 1 |
| MESAN2003039 | 0.010293023 | 99445 | 3 |
| MESAN2003058 | 0.027977249 | 139201 | 8 |
| MESAN2003101 | 0.00621311 | 100388 | 1 |
| MESAN2003190 | 0.009468379 | 22818 | 3 |
| MESAN2003322 | 0.00621311 | 185360 | 1 |
| MESAN2003444 | 0.044978915 | 79440 | 15 |
| MESAN2003490 | 0.00621311 | 250337 | 1 |
| MESAN2003529 | 0.00621311 | 166489 | 1 |
| MESAN2003622 | 0.278812027 | 97643 | 28 |
| MESAN2003646 | 0.008071224 | 70254 | 2 |
| MESAN2003662 | 0.00621311 | 53111 | 1 |
| MESAN2003709 | 0.020201654 | 186592 | 4 |
| MESAN2003851 | 0.00621311 | 84220 | 1 |
| MESAN2003852 | 0.166281879 | 99772 | 37 |
| MESAN2004138 | 0.00621311 | 125779 | 1 |
| MESAN2004288 | 0.032244849 | 144871 | 4 |
| MESAN2004575 | 0.025962295 | 180208 | 3 |
| MESAN2005284 | 0.034553936 | 184030 | 8 |
| MESAN2005303 | 0.021149473 | 165010 | 6 |
| MESAN2005371 | 0.00621311 | 242346 | 1 |
| MESAN2005633 | 0.019521102 | 58808 | 8 |
| MESAN2005689 | 0.054008359 | 106448 | 18 |
| MESAN2005724 | 0.00621311 | 149657 | 1 |
| MESAN2005766 | 0.169915307 | 88288 | 40 |
| MESAN2005808 | 0.00621311 | 87668 | 1 |
| MESAN2005811 | 0.00621311 | 77594 | 1 |
| MESAN2005957 | 0.033936434 | 110107 | 13 |
| MESAN2005958 | 0.062218274 | 17625 | 20 |
| MESAN2006022 | 0.00621311 | 55165 | 1 |
| MESAN2006043 | 0.021037077 | 105300 | 6 |
| MESAN2006328 | 0.00621311 | 216829 | 1 |
| MESAN2006401 | 0.092619948 | 51003 | 33 |
| MESAN2006563 | 0.242649079 | 50179 | 33 |
| MESAN2006580 | 0.008211511 | 182782 | 2 |
| MESAN2006599 | 0.00621311 | 278390 | 1 |
| MESAN2006743 | 0.04346969 | 53998 | 10 |
| MESAN2006953 | 0.024435922 | 120366 | 6 |
| MESAN2006971 | 0.02788572 | 185376 | 3 |
| MESAN2007032 | 0.00621311 | 43348 | 1 |
| MESAN2007440 | 0.00621311 | 82340 | 1 |
| MESAN2007597 | 0.00621311 | 67163 | 1 |
| MESAN2007755 | 0.018315664 | 124281 | 5 |
| MESAN2008222 | 0.00621311 | 262155 | 1 |
| MESAN2008334 | 0.020381848 | 144118 | 5 |
| MESAN2008415 | 0.015667586 | 76296 | 2 |
| MESAN2008460 | 0.00621311 | 82850 | 1 |
| MESAN2008536 | 0.00621311 | 260114 | 1 |
| MESAN2008679 | 0.023912265 | 28821 | 3 |
| MESAN2008729 | 0.007402354 | 202280 | 2 |
| MESAN2008821 | 0.00621311 | 240842 | 1 |
| MESAN2008926 | 0.052485634 | 65393 | 6 |
| MESAN2008936 | 0.042118119 | 123560 | 15 |
| MESAN2009019 | 0.021448765 | 179018 | 5 |
| MESAN2009081 | 0.035597418 | 103309 | 8 |
| MESAN2009105 | 0.00621311 | 137709 | 1 |
| MESAN2009156 | 0.016112319 | 130330 | 4 |
| MESAN2009311 | 0.00621311 | 154516 | 1 |
| MESAN2009418 | 0.00621311 | 238318 | 1 |
| MESAN2009503 | 0.010057194 | 50552 | 2 |
| MESAN2009522 | 0.00621311 | 143649 | 1 |
| MESAN2009580 | 0.150876604 | 132500 | 13 |
| MESAN2010031 | 0.00621311 | 3477 | 1 |
| MESAN2010042 | 0.066319138 | 118392 | 17 |
| MESAN2010114 | 0.00621311 | 96491 | 1 |
| MESAN2010312 | 0.00621311 | 177125 | 1 |
| MESAN2010321 | 0.00621311 | 15928 | 1 |
| MESAN2010664 | 0.019306872 | 131585 | 5 |
| MESAN2010681 | 0.00621311 | 216654 | 1 |
| MESAN2011545 | 0.00621311 | 208549 | 1 |
| MESAN2011597 | 0.00621311 | 54585 | 1 |
| MESAN2011627 | 0.044522139 | 129431 | 4 |
| MESAN2011632 | 0.00792257 | 56483 | 2 |
| MESAN2011977 | 0.00621311 | 161310 | 1 |
| MESAN2011984 | 0.098214026 | 16810 | 22 |
| MESAN2012054 | 0.065685408 | 91419 | 23 |
| MESAN2012113 | 0.028399837 | 104578 | 7 |
| MESAN2012586 | 0.00621311 | 168500 | 1 |
| MESAN2012708 | 0.00621311 | 151405 | 1 |
| MESAN2012735 | 0.016815315 | 170857 | 2 |
| MESAN2013019 | 0.00621311 | 75146 | 1 |
| MESAN2013022 | 0.00621311 | 179640 | 1 |
| MESAN2013183 | 0.00621311 | 181032 | 1 |
| MESAN2013211 | 0.00621311 | 11286 | 1 |
| MESAN2013283 | 0.010057194 | 260206 | 2 |
| MESAN2013284 | 0.136426651 | 49064 | 49 |
| MESAN2013611 | 0.00621311 | 127163 | 1 |
| MESAN2013674 | 0.00621311 | 11974 | 1 |
| MESAN2013845 | 0.030029415 | 60353 | 4 |
| MESAN2013908 | 0.024284853 | 119331 | 4 |
| MESAN2013936 | 0.00621311 | 50439 | 1 |
| MESAN2014192 | 0.007610265 | 14817 | 2 |
| MESAN2014295 | 0.008211511 | 111348 | 2 |
| MESAN2014298 | 0.00621311 | 186159 | 1 |
| MESAN2014412 | 0.008705543 | 117617 | 3 |
| MESAN2014624 | 0.00621311 | 102347 | 1 |
| MESAN2015277 | 0.00621311 | 180796 | 1 |
| MESAN2015365 | 0.007402354 | 28539 | 2 |
| MESAN2015391 | 0.008294622 | 134048 | 2 |
| MESAN2015401 | 0.00621311 | 27944 | 1 |
| MESAN2015501 | 0.00621311 | 133000 | 1 |
| MESAN2015515 | 0.063076038 | 110366 | 6 |
| MESAN2015708 | 0.018011128 | 275419 | 2 |
| MESAN2016159 | 0.007308388 | 230963 | 2 |
| MESAN2016304 | 0.00621311 | 274014 | 1 |
| MESAN2016409 | 0.010057194 | 185888 | 2 |
| MESAN2016552 | 0.01377169 | 144925 | 5 |
| MESAN2016965 | 0.00621311 | 177672 | 1 |
| MESAN2017133 | 0.019097239 | 127923 | 6 |
| MESAN2017152 | 0.008211511 | 193140 | 2 |
| MESAN2017373 | 0.047837489 | 149079 | 3 |
| MESAN2017417 | 0.00621311 | 9337 | 1 |
| MESAN2018209 | 0.00621311 | 118478 | 1 |
| MESAN2018576 | 0.015921848 | 221004 | 2 |
| MESAN2018670 | 0.00621311 | 82391 | 1 |
| MESAN2018699 | 0.010883345 | 126608 | 3 |
| MESTC1000042 | 0.149258139 | 113402 | 4 |
| MESTC1000147 | 0.1447178 | 238409 | 1 |
| MESTC2000150 | 0.1447178 | 247832 | 1 |
| MESTC2000153 | 0.1447178 | 255090 | 1 |
| MESTC2000170 | 0.1447178 | 236425 | 1 |
| N1ESE2000698 | 0.039448906 | 15070 | 2 |
| NB9N41000011 | 0.078728801 | 8604 | 9 |
| NB9N41000047 | 0.063749775 | 65266 | 4 |
| NB9N41000121 | 0.223129215 | 67523 | 14 |
| NB9N41000135 | 0.075549107 | 80203 | 5 |
| NB9N41000142 | 0.06258574 | 34767 | 3 |
| NB9N41000146 | 0.056689342 | 66569 | 1 |
| NB9N41000340 | 0.115533323 | 49241 | 10 |
| NB9N42000042 | 0.086235482 | 104154 | 13 |
| NB9N42000104 | 0.202136096 | 145316 | 14 |
| NB9N42000122 | 0.056689342 | 216519 | 1 |
| NB9N42000196 | 0.165899693 | 250956 | 3 |
| NB9N42000281 | 0.087630223 | 125130 | 12 |
| NB9N42000314 | 0.056689342 | 218830 | 1 |
| NB9N42000342 | 0.232387732 | 103773 | 19 |
| NB9N42000430 | 0.060533426 | 100042 | 2 |
| NB9N42000495 | 0.056689342 | 67307 | 1 |
| NB9N42001300 | 0.061898551 | 52194 | 4 |
| NCRRP1000129 | 0.143061516 | 251354 | 1 |
| NESOP1000010 | 0.035650624 | 230824 | 1 |
| NESOP1000087 | 0.041563235 | 157486 | 2 |
| NESOP1000108 | 0.035650624 | 112218 | 1 |
| NESOP2000231 | 0.035650624 | 235751 | 1 |
| NESOP2000452 | 0.035650624 | 63439 | 1 |
| NESOP2000499 | 0.035650624 | 227862 | 1 |
| NESOP2000504 | 0.037047779 | 72164 | 2 |
| NESOP2000744 | 0.035650624 | 192316 | 1 |
| NESOP2001144 | 0.035650624 | 275693 | 1 |
| NESOP2001433 | 0.035650624 | 266209 | 1 |
| NESOP2001656 | 0.041852944 | 163725 | 2 |
| NESOP2001694 | 0.035650624 | 274555 | 1 |
| NESOP2001752 | 0.036839868 | 238005 | 2 |
| NESOP2002352 | 0.035650624 | 249061 | 1 |
| NESOP2002418 | 0.035650624 | 270810 | 1 |
| NESOP2002487 | 0.035650624 | 280908 | 1 |
| NESOP2002738 | 0.035650624 | 234571 | 1 |
| NESOP2002780 | 0.035650624 | 203339 | 1 |
| NETRP1000034 | 0.010827198 | 134951 | 1 |
| NETRP1000082 | 0.010827198 | 246140 | 1 |
| NETRP2000086 | 0.010827198 | 235690 | 1 |
| NETRP2000182 | 0.010827198 | 231597 | 1 |
| NETRP2000439 | 0.032481594 | 134784 | 3 |
| NHNPC1000034 | 0.043494297 | 133294 | 2 |
| NHNPC1000037 | 0.04180602 | 72536 | 1 |
| NHNPC1000084 | 0.04180602 | 97108 | 1 |
| NHNPC1000101 | 0.04180602 | 50677 | 1 |
| NHNPC1000124 | 0.04180602 | 71538 | 1 |
| NHNPC2000062 | 0.079954704 | 90681 | 10 |
| NHNPC2000187 | 0.370266431 | 100418 | 27 |
| NHNPC2000206 | 0.243998186 | 121013 | 62 |
| NHNPC2000212 | 0.048078306 | 18948 | 4 |
| NHNPC2000606 | 0.04180602 | 102204 | 1 |
| NHNPC2000877 | 0.04180602 | 185877 | 1 |
| NHNPC2001108 | 0.042901298 | 176597 | 2 |
| NHNPC2001223 | 0.044901516 | 102205 | 2 |
| NHNPC2001312 | 0.043887532 | 189476 | 2 |
| NHNPC2001816 | 0.04180602 | 269601 | 1 |
| NHNPC2001931 | 0.35109498 | 53308 | 28 |
| NHNPC2002565 | 0.04180602 | 170376 | 1 |
| NHNPC2002749 | 0.04180602 | 209207 | 1 |
| NOVAR1000015 | 0.055180045 | 160312 | 5 |
| NOVAR1000091 | 0.086526567 | 130741 | 10 |
| NOVAR1000102 | 0.120903585 | 143806 | 4 |
| NOVAR2000038 | 0.269744409 | 20263 | 50 |
| NOVAR2000136 | 0.039936102 | 282788 | 1 |
| NOVAR2000352 | 0.039936102 | 162230 | 1 |
| NOVAR2000412 | 0.039936102 | 71074 | 1 |
| NOVAR2000710 | 0.047565028 | 79451 | 5 |
| NOVAR2000783 | 0.044302137 | 196080 | 4 |
| NOVAR2000962 | 0.039936102 | 205724 | 1 |
| NOVAR2001108 | 0.039936102 | 275495 | 1 |
| NOVAR2001620 | 0.039936102 | 277910 | 1 |
| NOVAR2001783 | 0.041611649 | 192621 | 2 |
| NOVAR2001844 | 0.079018445 | 63416 | 8 |
| NOVAR2001876 | 0.039936102 | 267952 | 1 |
| NOVAR2002039 | 0.041333258 | 127798 | 2 |
| NT2NE1000004 | 0.020289133 | 65538 | 4 |
| NT2NE1000014 | 0.01927591 | 48558 | 7 |
| NT2NE1000018 | 0.067381772 | 51526 | 23 |
| NT2NE1000023 | 0.006072383 | 59163 | 1 |
| NT2NE1000024 | 0.043645394 | 50046 | 9 |
| NT2NE1000059 | 0.030978051 | 51277 | 4 |
| NT2NE1000063 | 0.012144766 | 52662 | 2 |
| NT2NE1000073 | 0.021042129 | 40425 | 5 |
| NT2NE1000083 | 0.006072383 | 48093 | 1 |
| NT2NE1000120 | 0.006072383 | 73851 | 1 |
| NT2NE1000122 | 0.007469538 | 53434 | 2 |
| NT2NE1000156 | 0.02649962 | 45436 | 5 |
| NT2NE1000163 | 0.413660566 | 49243 | 102 |
| NT2NE1000175 | 0.006072383 | 58574 | 1 |
| NT2NE1000181 | 0.009178998 | 30936 | 3 |
| NT2NE1000185 | 0.006072383 | 48982 | 1 |
| NT2NE2000038 | 0.006072383 | 88586 | 1 |
| NT2NE2000054 | 0.006072383 | 78336 | 1 |
| NT2NE2000056 | 0.426534229 | 37647 | 131 |
| NT2NE2000057 | 0.006072383 | 262434 | 1 |
| NT2NE2000064 | 0.006072383 | 146491 | 1 |
| NT2NE2000109 | 0.006072383 | 279204 | 1 |
| NT2NE2000137 | 0.006072383 | 265070 | 1 |
| NT2NE2000156 | 0.013155547 | 93786 | 3 |
| NT2NE2000174 | 0.439109139 | 77740 | 32 |
| NT2NE2000195 | 0.109001549 | 103469 | 15 |
| NT2NE2000214 | 0.131161861 | 116807 | 34 |
| NT2NE2000222 | 0.006072383 | 268088 | 1 |
| NT2NE2000259 | 0.05956676 | 158400 | 14 |
| NT2NE2000260 | 1.387073374 | 103993 | 89 |
| NT2NE2000284 | 0.006072383 | 114817 | 1 |
| NT2NE2000299 | 0.006072383 | 104697 | 1 |
| NT2NE2000327 | 0.066605006 | 22381 | 33 |
| NT2NE2000353 | 0.009167879 | 153808 | 2 |
| NT2NE2000356 | 0.007469538 | 176369 | 2 |
| NT2NE2000369 | 0.006072383 | 70435 | 1 |
| NT2NE2000374 | 0.006072383 | 187976 | 1 |
| NT2NE2000383 | 0.110267507 | 69725 | 32 |
| NT2NE2000384 | 0.072799264 | 83009 | 24 |
| NT2NE2000386 | 0.010843426 | 157388 | 3 |
| NT2NE2000390 | 0.006072383 | 270932 | 1 |
| NT2NE2000392 | 0.187681029 | 48071 | 66 |
| NT2NE2000455 | 0.008070784 | 134795 | 2 |
| NT2NE2000470 | 0.030704566 | 134793 | 7 |
| NT2NE2000488 | 0.006072383 | 273469 | 1 |
| NT2NE2000517 | 0.011798942 | 110083 | 3 |
| NT2NE2000536 | 0.019468635 | 186254 | 3 |
| NT2NE2000546 | 0.006072383 | 266324 | 1 |
| NT2NE2000548 | 0.007930497 | 160652 | 2 |
| NT2NE2000550 | 0.006072383 | 169321 | 1 |
| NT2NE2000575 | 0.696988405 | 17919 | 122 |
| NT2NE2000586 | 0.006072383 | 164805 | 1 |
| NT2NE2000612 | 0.006072383 | 147874 | 1 |
| NT2NE2000636 | 0.046404186 | 129610 | 11 |
| NT2NE2000658 | 0.006072383 | 222682 | 1 |
| NT2NE2000706 | 0.006072383 | 129087 | 1 |
| NT2NE2000707 | 0.500699743 | 52066 | 121 |
| NT2NE2000763 | 0.139619037 | 76345 | 33 |
| NT2NE2000787 | 0.169706805 | 109741 | 51 |
| NT2NE2000802 | 0.006072383 | 60033 | 1 |
| NT2NE2000808 | 0.006072383 | 265767 | 1 |
| NT2NE2000809 | 0.049432317 | 142817 | 11 |
| NT2NE2000864 | 0.007261627 | 182617 | 2 |
| NT2NE2000909 | 0.051185943 | 16713 | 16 |
| NT2NE2000913 | 0.022715338 | 13867 | 6 |
| NT2NE2000950 | 0.031968947 | 105203 | 7 |
| NT2NE2000951 | 0.006072383 | 163355 | 1 |
| NT2NE2000963 | 0.730406971 | 10433 | 69 |
| NT2NE2000980 | 0.010975379 | 102392 | 3 |
| NT2NE2001000 | 0.194695265 | 58103 | 29 |
| NT2NE2001005 | 0.007167661 | 166974 | 2 |
| NT2NE2001021 | 0.006072383 | 174056 | 1 |
| NT2NE2001040 | 0.010808018 | 128473 | 4 |
| NT2NE2001048 | 0.111946578 | 80591 | 26 |
| NT2NE2001049 | 0.019067089 | 18802 | 4 |
| NT2NE2001142 | 0.006072383 | 79591 | 1 |
| NT2NE2001156 | 0.029812719 | 96235 | 5 |
| NT2NE2001176 | 0.146698192 | 36550 | 40 |
| NT2NE2001180 | 0.012008158 | 206129 | 2 |
| NT2NE2001181 | 0.00898724 | 226151 | 2 |
| NT2NE2001188 | 0.006072383 | 68172 | 1 |
| NT2NE2001191 | 0.006072383 | 32216 | 1 |
| NT2NE2001247 | 0.006072383 | 269958 | 1 |
| NT2NE2001252 | 0.006072383 | 189199 | 1 |
| NT2NE2001324 | 0.007261627 | 41267 | 2 |
| NT2NE2001326 | 0.007949224 | 75722 | 2 |
| NT2NE2001337 | 0.036872043 | 74584 | 11 |
| NT2NE2001364 | 0.015672564 | 85122 | 4 |
| NT2NE2001372 | 0.006072383 | 27949 | 1 |
| NT2NE2001397 | 0.01273958 | 115606 | 4 |
| NT2NE2001403 | 0.032296527 | 122120 | 10 |
| NT2NE2001428 | 0.006072383 | 267723 | 1 |
| NT2NE2001432 | 0.006072383 | 32992 | 1 |
| NT2NE2001435 | 0.157605798 | 10147 | 69 |
| NT2NE2001442 | 0.033530102 | 93501 | 10 |
| NT2NE2001524 | 0.006072383 | 172655 | 1 |
| NT2NE2001530 | 0.023947856 | 57996 | 4 |
| NT2NE2001545 | 0.093514659 | 8806 | 31 |
| NT2NE2001598 | 0.013209227 | 154295 | 3 |
| NT2NE2001617 | 0.036932165 | 100046 | 7 |
| NT2NE2001623 | 0.006072383 | 225743 | 1 |
| NT2NE2001626 | 0.012144766 | 131707 | 2 |
| NT2NE2001634 | 0.006072383 | 85724 | 1 |
| NT2NE2001648 | 0.019305756 | 109733 | 4 |
| NT2NE2001655 | 0.119679354 | 11444 | 23 |
| NT2NE2001660 | 0.021393067 | 94956 | 7 |
| NT2NE2001666 | 0.006072383 | 9176 | 1 |
| NT2NE2001677 | 0.006072383 | 101330 | 1 |
| NT2NE2001697 | 0.030688551 | 159241 | 4 |
| NT2NE2001698 | 0.006072383 | 210391 | 1 |
| NT2NE2001723 | 0.006072383 | 208829 | 1 |
| NT2NE2001725 | 0.006072383 | 211817 | 1 |
| NT2NE2001793 | 0.161541388 | 60629 | 74 |
| NT2NE2001874 | 0.006072383 | 158706 | 1 |
| NT2NE2001889 | 0.006072383 | 77320 | 1 |
| NT2NE2001923 | 0.006072383 | 19176 | 1 |
| NT2NE2002100 | 0.007469538 | 152601 | 2 |
| NT2NE2002162 | 0.013705896 | 88624 | 3 |
| NT2NE2002186 | 0.006072383 | 259275 | 1 |
| NT2NE2002285 | 0.006072383 | 258902 | 1 |
| NT2NE2002311 | 0.006072383 | 86549 | 1 |
| NT2NE2002620 | 0.175174974 | 37247 | 3 |
| NT2NE2002651 | 0.006072383 | 148687 | 1 |
| NT2NE2002732 | 0.006072383 | 261036 | 1 |
| NT2NE2002768 | 0.006072383 | 243284 | 1 |
| NT2NE2002791 | 0.006072383 | 254786 | 1 |
| NT2NE2002856 | 0.006072383 | 261103 | 1 |
| NT2NE2002870 | 0.073402966 | 149096 | 7 |
| NT2NE2003150 | 0.006072383 | 264291 | 1 |
| NT2NE2003185 | 0.044201935 | 115411 | 13 |
| NT2NE2003252 | 0.006072383 | 181083 | 1 |
| NT2NE2003280 | 0.023023737 | 151479 | 7 |
| NT2NE2003308 | 0.006072383 | 194541 | 1 |
| NT2NE2003309 | 0.006072383 | 33587 | 1 |
| NT2NE2003315 | 0.006072383 | 171456 | 1 |
| NT2NE2003393 | 0.13035634 | 163132 | 3 |
| NT2NE2003408 | 0.006072383 | 193697 | 1 |
| NT2NE2003457 | 0.006072383 | 185775 | 1 |
| NT2NE2003485 | 0.071984942 | 106455 | 14 |
| NT2NE2003540 | 0.006072383 | 173634 | 1 |
| NT2NE2003569 | 0.096548347 | 139448 | 20 |
| NT2NE2003571 | 0.006072383 | 192465 | 1 |
| NT2NE2003705 | 0.006072383 | 259184 | 1 |
| NT2NE2003752 | 0.006072383 | 238693 | 1 |
| NT2NE2003887 | 0.026846204 | 94549 | 6 |
| NT2NE2003921 | 0.027465583 | 121717 | 6 |
| NT2NE2004255 | 0.028130935 | 121306 | 8 |
| NT2NE2004378 | 0.006072383 | 256633 | 1 |
| NT2NE2004490 | 0.006072383 | 241788 | 1 |
| NT2NE2004519 | 0.093506362 | 101732 | 15 |
| NT2NE2004606 | 0.006072383 | 235581 | 1 |
| NT2NE2004716 | 0.087297185 | 132046 | 6 |
| NT2NE2004740 | 0.007949224 | 251575 | 2 |
| NT2NE2004787 | 0.006072383 | 135224 | 1 |
| NT2NE2004916 | 0.006072383 | 51258 | 1 |
| NT2NE2005035 | 0.006072383 | 259213 | 1 |
| NT2NE2005223 | 0.006072383 | 93982 | 1 |
| NT2NE2005276 | 0.006072383 | 236380 | 1 |
| NT2NE2005317 | 0.013176752 | 176124 | 4 |
| NT2NE2005323 | 0.007469538 | 240074 | 2 |
| NT2NE2005358 | 0.008070784 | 97809 | 2 |
| NT2NE2005371 | 0.030388261 | 47610 | 6 |
| NT2NE2005395 | 0.012263375 | 67331 | 3 |
| NT2NE2005419 | 0.014471111 | 98619 | 6 |
| NT2NE2005441 | 0.053931326 | 102319 | 3 |
| NT2NE2005517 | 0.006072383 | 57515 | 1 |
| NT2NE2005537 | 0.006072383 | 249068 | 1 |
| NT2NE2005555 | 0.006072383 | 240742 | 1 |
| NT2NE2005588 | 0.009916467 | 193051 | 2 |
| NT2NE2005676 | 0.006072383 | 104634 | 1 |
| NT2NE2005688 | 0.224390279 | 112983 | 21 |
| NT2NE2005720 | 0.017835705 | 51662 | 2 |
| NT2NE2005784 | 0.006072383 | 263061 | 1 |
| NT2NE2005804 | 0.006072383 | 248927 | 1 |
| NT2NE2005821 | 0.006072383 | 260612 | 1 |
| NT2NE2005876 | 0.113589258 | 17299 | 31 |
| NT2NE2005890 | 0.009448936 | 200105 | 3 |
| NT2NE2005921 | 0.006072383 | 17703 | 1 |
| NT2NE2005968 | 0.006072383 | 42688 | 1 |
| NT2NE2006075 | 0.021270419 | 208725 | 5 |
| NT2NE2006087 | 0.016507765 | 124208 | 4 |
| NT2NE2006103 | 0.019351688 | 155724 | 4 |
| NT2NE2006217 | 0.006072383 | 249352 | 1 |
| NT2NE2006288 | 0.006072383 | 104497 | 1 |
| NT2NE2006382 | 0.006072383 | 259217 | 1 |
| NT2NE2006387 | 0.006072383 | 260624 | 1 |
| NT2NE2006427 | 0.009117264 | 69474 | 2 |
| NT2NE2006432 | 0.006072383 | 253576 | 1 |
| NT2NE2006458 | 0.118540551 | 154135 | 13 |
| NT2NE2006478 | 0.015594036 | 96791 | 6 |
| NT2NE2006531 | 0.006072383 | 257218 | 1 |
| NT2NE2006659 | 0.009260029 | 134874 | 3 |
| NT2NE2006784 | 0.006072383 | 254058 | 1 |
| NT2NE2006813 | 0.006072383 | 284911 | 1 |
| NT2NE2006894 | 0.006072383 | 258203 | 1 |
| NT2NE2006909 | 0.587286909 | 58210 | 14 |
| NT2NE2006942 | 0.006072383 | 249199 | 1 |
| NT2NE2006958 | 0.010249318 | 129910 | 2 |
| NT2NE2007052 | 0.006072383 | 235608 | 1 |
| NT2NE2007220 | 0.006072383 | 150744 | 1 |
| NT2NE2007425 | 0.006072383 | 161080 | 1 |
| NT2NE2007725 | 0.006072383 | 70715 | 1 |
| NT2NE2007727 | 0.027386554 | 151577 | 8 |
| NT2NE2007786 | 0.04205655 | 164480 | 2 |
| NT2NE2007877 | 0.012144766 | 151701 | 2 |
| NT2NE2007967 | 0.006072383 | 105635 | 1 |
| NT2NE2008017 | 0.006072383 | 264849 | 1 |
| NT2NE2008060 | 0.008153895 | 280285 | 2 |
| NT2NE2008077 | 0.017197091 | 224642 | 2 |
| NT2NE2008213 | 0.020134968 | 180367 | 4 |
| NT2NE2008260 | 0.006072383 | 150833 | 1 |
| NT2NE2008378 | 0.006072383 | 265591 | 1 |
| NT2NE2008607 | 0.006072383 | 258913 | 1 |
| NT2NE2008639 | 0.006072383 | 223459 | 1 |
| NT2NE2008727 | 0.006072383 | 161289 | 1 |
| NT2NE2008783 | 0.006072383 | 260596 | 1 |
| NT2NE2008785 | 0.017859276 | 282541 | 2 |
| NT2NE2008803 | 0.007261627 | 162623 | 2 |
| NT2NE2008867 | 0.006072383 | 277149 | 1 |
| NT2NE2008961 | 0.012144766 | 243285 | 2 |
| NT2NE2008997 | 0.006072383 | 19170 | 1 |
| NT2NE2009138 | 0.006072383 | 255513 | 1 |
| NT2NE2009295 | 0.083985743 | 46492 | 9 |
| NT2NE2009426 | 0.006072383 | 75879 | 1 |
| NT2NE2009523 | 0.021562885 | 149685 | 5 |
| NT2NE2009608 | 0.006072383 | 18833 | 1 |
| NT2NE2009691 | 0.006072383 | 167636 | 1 |
| NT2NE2010056 | 0.020909833 | 29945 | 9 |
| NT2NE2010105 | 0.006072383 | 150524 | 1 |
| NT2NE2010150 | 0.006072383 | 164442 | 1 |
| NT2NE2010400 | 0.006072383 | 175757 | 1 |
| NT2NE2010632 | 0.006072383 | 63318 | 1 |
| NT2NE2010633 | 0.006072383 | 154412 | 1 |
| NT2NE2010654 | 0.006072383 | 167651 | 1 |
| NT2NE2010688 | 0.00774793 | 143496 | 2 |
| NT2NE2010781 | 0.006072383 | 227047 | 1 |
| NT2NE2010842 | 0.177706809 | 109238 | 41 |
| NT2NE2010854 | 0.007167661 | 126577 | 2 |
| NT2NE2011033 | 0.009167879 | 112056 | 2 |
| NT2NE2011036 | 0.012144766 | 179262 | 2 |
| NT2NE2011107 | 0.013932163 | 158385 | 7 |
| NT2NE2011119 | 0.036186709 | 125008 | 7 |
| NT2NE2011154 | 0.006072383 | 54552 | 1 |
| NT2NE2011221 | 0.011646726 | 91779 | 4 |
| NT2NE2011411 | 0.006072383 | 102565 | 1 |
| NT2NE2011485 | 0.008843208 | 130445 | 3 |
| NT2NE2011650 | 0.009117264 | 23794 | 2 |
| NT2NE2011684 | 0.019134034 | 197356 | 2 |
| NT2NE2011687 | 0.006072383 | 185793 | 1 |
| NT2NE2011691 | 0.006072383 | 127863 | 1 |
| NT2NE2011758 | 0.006072383 | 102320 | 1 |
| NT2NE2011823 | 0.006072383 | 29687 | 1 |
| NT2NE2011896 | 0.006072383 | 59554 | 1 |
| NT2NE2011998 | 0.008153895 | 81516 | 2 |
| NT2NE2012113 | 0.006072383 | 161166 | 1 |
| NT2NE2012199 | 0.013833042 | 163215 | 3 |
| NT2NE2012243 | 0.007930497 | 193548 | 2 |
| NT2NE2012361 | 0.006072383 | 56528 | 1 |
| NT2NE2012448 | 0.006072383 | 55032 | 1 |
| NT2NE2012457 | 0.006072383 | 239597 | 1 |
| NT2NE2012493 | 0.006072383 | 48388 | 1 |
| NT2NE2012505 | 0.009138469 | 109045 | 3 |
| NT2NE2012533 | 0.006072383 | 109166 | 1 |
| NT2NE2012603 | 0.006072383 | 50635 | 1 |
| NT2NE2012747 | 0.006072383 | 19081 | 1 |
| NT2NE2012790 | 0.02793118 | 65056 | 5 |
| NT2NE2012847 | 0.006072383 | 210723 | 1 |
| NT2NE2012928 | 0.019245317 | 98772 | 4 |
| NT2NE2013019 | 0.009117264 | 156238 | 2 |
| NT2NE2013081 | 0.006072383 | 161376 | 1 |
| NT2NE2013189 | 0.006072383 | 93664 | 1 |
| NT2NE2013217 | 0.006072383 | 137391 | 1 |
| NT2NE2013501 | 0.110236555 | 92380 | 37 |
| NT2NE2013547 | 0.006072383 | 97970 | 1 |
| NT2NE2014013 | 0.006072383 | 75501 | 1 |
| NT2NE2014028 | 0.006072383 | 133098 | 1 |
| NT2NE2014104 | 0.099827368 | 52557 | 14 |
| NT2NE2014113 | 0.018465695 | 183524 | 4 |
| NT2NE2014176 | 0.012162146 | 109003 | 3 |
| NT2NE2014221 | 0.006072383 | 183651 | 1 |
| NT2NE2014525 | 0.006072383 | 3986 | 1 |
| NT2NE2014650 | 0.006072383 | 37833 | 1 |
| NT2NE2014651 | 0.006072383 | 207671 | 1 |
| NT2NE2014681 | 0.006072383 | 189721 | 1 |
| NT2NE2014758 | 0.006072383 | 33535 | 1 |
| NT2NE2014869 | 0.006072383 | 283898 | 1 |
| NT2NE2014950 | 0.006072383 | 192405 | 1 |
| NT2NE2015061 | 0.006072383 | 161172 | 1 |
| NT2NE2015262 | 0.007469538 | 151576 | 2 |
| NT2NE2015275 | 0.006072383 | 191366 | 1 |
| NT2NE2015362 | 0.009138469 | 116784 | 3 |
| NT2NE2015480 | 0.006072383 | 52825 | 1 |
| NT2NE2015511 | 0.006072383 | 237414 | 1 |
| NT2NE2015565 | 0.006072383 | 129902 | 1 |
| NT2NE2015626 | 0.006072383 | 127866 | 1 |
| NT2NE2015712 | 0.133701175 | 71102 | 22 |
| NT2NE2015747 | 0.006072383 | 54689 | 1 |
| NT2NE2015860 | 0.009117264 | 190403 | 2 |
| NT2NE2015885 | 0.006072383 | 151613 | 1 |
| NT2NE2015904 | 0.007781843 | 149472 | 2 |
| NT2NE2015974 | 0.021934332 | 54112 | 11 |
| NT2NE2016041 | 0.006072383 | 62695 | 1 |
| NT2NE2016387 | 0.00774793 | 51954 | 2 |
| NT2NE2016419 | 0.006072383 | 221100 | 1 |
| NT2NE2016519 | 0.006072383 | 104646 | 1 |
| NT2NE2016608 | 0.006072383 | 65079 | 1 |
| NT2NE2016766 | 0.006072383 | 173601 | 1 |
| NT2NE2016774 | 0.006072383 | 189704 | 1 |
| NT2NE2016971 | 0.012238071 | 54418 | 3 |
| NT2NE2017259 | 0.006072383 | 60055 | 1 |
| NT2NE2017332 | 0.006072383 | 141459 | 1 |
| NT2NE2017397 | 0.006072383 | 131867 | 1 |
| NT2NE2017480 | 0.010110666 | 167627 | 2 |
| NT2NE2017492 | 0.009928898 | 233573 | 3 |
| NT2NE2017508 | 0.006072383 | 159969 | 1 |
| NT2NE2017562 | 0.006072383 | 179655 | 1 |
| NT2NE2017590 | 0.006072383 | 194254 | 1 |
| NT2NE2017612 | 0.006072383 | 175656 | 1 |
| NT2NE2017721 | 0.006072383 | 161552 | 1 |
| NT2NE2017752 | 0.01427369 | 106435 | 4 |
| NT2NE2017792 | 0.008949904 | 173685 | 3 |
| NT2NE2017982 | 0.007167661 | 180775 | 2 |
| NT2NE2018165 | 0.006072383 | 126253 | 1 |
| NT2NE2018176 | 0.006072383 | 86243 | 1 |
| NT2NE2018180 | 0.044561852 | 167633 | 9 |
| NT2NE2018273 | 0.006072383 | 75823 | 1 |
| NT2NE2018376 | 0.006072383 | 173719 | 1 |
| NT2NE2018472 | 0.006072383 | 179874 | 1 |
| NT2NE2018490 | 0.007167661 | 130179 | 2 |
| NT2NE2018594 | 0.009916467 | 172593 | 2 |
| NT2NE2018739 | 0.006072383 | 124942 | 1 |
| NT2NE2018916 | 0.012144766 | 58507 | 2 |
| NT2NE2019056 | 0.006072383 | 175858 | 1 |
| NT2NE2019076 | 0.018595864 | 175854 | 2 |
| NT2NE2019091 | 0.088507346 | 126422 | 18 |
| NT2NE2019099 | 0.006072383 | 27233 | 1 |
| NT2RI1000016 | 0.006631183 | 63833 | 3 |
| NT2RI1000027 | 0.165718781 | 50916 | 17 |
| NT2RI1000035 | 0.00885006 | 61879 | 2 |
| NT2RI1000048 | 0.197336203 | 64352 | 17 |
| NT2RI1000127 | 0.092317238 | 76270 | 19 |
| NT2RI1000164 | 0.003044882 | 56827 | 1 |
| NT2RI1000172 | 0.013155547 | 78304 | 3 |
| NT2RI2000004 | 0.003044882 | 270754 | 1 |
| NT2RI2000007 | 0.005043283 | 134288 | 2 |
| NT2RI2000064 | 0.311298378 | 84067 | 46 |
| NT2RI2000107 | 0.072337994 | 110490 | 17 |
| NT2RI2000116 | 0.288108079 | 35941 | 124 |
| NT2RI2000117 | 0.003044882 | 52717 | 1 |
| NT2RI2000128 | 0.003044882 | 67613 | 1 |
| NT2RI2000133 | 0.065392303 | 134275 | 5 |
| NT2RI2000167 | 0.014503052 | 48732 | 5 |
| NT2RI2000173 | 0.007083164 | 262237 | 2 |
| NT2RI2000187 | 0.003044882 | 262236 | 1 |
| NT2RI2000255 | 0.139986191 | 34452 | 19 |
| NT2RI2000263 | 0.097952942 | 8609 | 20 |
| NT2RI2000270 | 0.003044882 | 139254 | 1 |
| NT2RI2000276 | 0.003044882 | 66761 | 1 |
| NT2RI2000282 | 0.529374764 | 79584 | 53 |
| NT2RI2000284 | 0.367548357 | 94056 | 35 |
| NT2RI2000294 | 0.088528718 | 107318 | 22 |
| NT2RI2000313 | 0.009164676 | 145861 | 3 |
| NT2RI2000341 | 0.003044882 | 19244 | 1 |
| NT2RI2000344 | 0.206956181 | 11149 | 15 |
| NT2RI2000348 | 0.018908463 | 72638 | 8 |
| NT2RI2000412 | 0.021738892 | 124256 | 10 |
| NT2RI2000421 | 0.055063444 | 108331 | 5 |
| NT2RI2000480 | 0.003044882 | 244831 | 1 |
| NT2RI2000575 | 0.003044882 | 149159 | 1 |
| NT2RI2000578 | 0.501074101 | 63367 | 31 |
| NT2RI2000588 | 0.003044882 | 129730 | 1 |
| NT2RI2000597 | 0.003044882 | 41065 | 1 |
| NT2RI2000669 | 0.013647087 | 122047 | 2 |
| NT2RI2000671 | 0.159298608 | 138197 | 20 |
| NT2RI2000685 | 0.169117153 | 30433 | 51 |
| NT2RI2000688 | 0.003044882 | 136901 | 1 |
| NT2RI2000689 | 0.10836064 | 134628 | 14 |
| NT2RI2000704 | 0.003044882 | 101241 | 1 |
| NT2RI2000727 | 0.647670214 | 18625 | 92 |
| NT2RI2000738 | 0.003044882 | 266131 | 1 |
| NT2RI2000775 | 0.009824719 | 207766 | 4 |
| NT2RI2000822 | 0.015728719 | 171711 | 3 |
| NT2RI2000829 | 0.043869045 | 133120 | 14 |
| NT2RI2000841 | 0.003044882 | 259233 | 1 |
| NT2RI2000865 | 0.059734224 | 157220 | 2 |
| NT2RI2000932 | 0.003044882 | 33427 | 1 |
| NT2RI2000974 | 0.07767935 | 144388 | 17 |
| NT2RI2000987 | 0.003044882 | 164859 | 1 |
| NT2RI2001010 | 0.004442037 | 155313 | 2 |
| NT2RI2001017 | 0.003044882 | 110452 | 1 |
| NT2RI2001030 | 0.005126394 | 127086 | 2 |
| NT2RI2001054 | 0.281610305 | 101025 | 26 |
| NT2RI2001083 | 0.003044882 | 57062 | 1 |
| NT2RI2001091 | 0.047658284 | 113969 | 8 |
| NT2RI2001167 | 0.00414016 | 240721 | 2 |
| NT2RI2001178 | 0.003044882 | 271855 | 1 |
| NT2RI2001230 | 0.217223438 | 79682 | 16 |
| NT2RI2001235 | 0.003044882 | 266711 | 1 |
| NT2RI2001244 | 0.003044882 | 179531 | 1 |
| NT2RI2001307 | 0.003044882 | 262280 | 1 |
| NT2RI2001342 | 0.106154394 | 12062 | 10 |
| NT2RI2001385 | 0.042528621 | 91061 | 7 |
| NT2RI2001409 | 0.014549714 | 151891 | 2 |
| NT2RI2001410 | 0.003044882 | 47821 | 1 |
| NT2RI2001422 | 0.045071428 | 10964 | 8 |
| NT2RI2001425 | 0.003044882 | 260083 | 1 |
| NT2RI2001449 | 0.029390737 | 61372 | 9 |
| NT2RI2001450 | 0.094565248 | 110620 | 38 |
| NT2RI2001474 | 0.003044882 | 265130 | 1 |
| NT2RI2001519 | 0.003044882 | 85438 | 1 |
| NT2RI2001540 | 2.943484976 | 18066 | 326 |
| NT2RI2001595 | 0.021101854 | 72422 | 5 |
| NT2RI2001621 | 0.054737539 | 171697 | 14 |
| NT2RI2001624 | 0.003044882 | 199443 | 1 |
| NT2RI2001657 | 0.31315763 | 34843 | 53 |
| NT2RI2001726 | 0.015868295 | 138336 | 7 |
| NT2RI2001731 | 0.088472215 | 106604 | 16 |
| NT2RI2001769 | 0.075374793 | 65315 | 7 |
| NT2RI2001782 | 0.003044882 | 266659 | 1 |
| NT2RI2001836 | 0.003044882 | 92674 | 1 |
| NT2RI2001846 | 0.034002249 | 127692 | 8 |
| NT2RI2001859 | 0.172666004 | 144954 | 42 |
| NT2RI2001866 | 0.011546406 | 108092 | 5 |
| NT2RI2001910 | 0.022433577 | 108147 | 7 |
| NT2RI2001952 | 0.007998492 | 146188 | 3 |
| NT2RI2002022 | 0.005126394 | 107272 | 2 |
| NT2RI2002041 | 0.003044882 | 266360 | 1 |
| NT2RI2002091 | 12.49272458 | 2604 | 1148 |
| NT2RI2002117 | 0.035566328 | 116219 | 7 |
| NT2RI2002120 | 0.021314171 | 169692 | 7 |
| NT2RI2002152 | 0.133240831 | 38406 | 19 |
| NT2RI2002153 | 0.003044882 | 264222 | 1 |
| NT2RI2002179 | 0.003044882 | 173643 | 1 |
| NT2RI2002211 | 0.403797085 | 70462 | 140 |
| NT2RI2002243 | 0.050938581 | 177528 | 6 |
| NT2RI2002252 | 0.003044882 | 214288 | 1 |
| NT2RI2002260 | 0.007083164 | 53369 | 2 |
| NT2RI2002266 | 0.066853669 | 97891 | 9 |
| NT2RI2002270 | 0.003044882 | 133802 | 1 |
| NT2RI2002316 | 0.208991284 | 114022 | 30 |
| NT2RI2002334 | 0.003044882 | 267590 | 1 |
| NT2RI2002359 | 0.020359769 | 133183 | 4 |
| NT2RI2002391 | 0.206616486 | 90765 | 34 |
| NT2RI2002447 | 0.003044882 | 276180 | 1 |
| NT2RI2002481 | 0.003044882 | 214460 | 1 |
| NT2RI2002507 | 0.003044882 | 274438 | 1 |
| NT2RI2002517 | 0.007083164 | 175210 | 2 |
| NT2RI2002530 | 0.02775887 | 50487 | 6 |
| NT2RI2002540 | 0.096926156 | 104804 | 29 |
| NT2RI2002541 | 0.095062189 | 112662 | 25 |
| NT2RI2002549 | 0.024319054 | 27726 | 6 |
| NT2RI2002554 | 0.092407576 | 15000 | 23 |
| NT2RI2002564 | 0.003044882 | 114625 | 1 |
| NT2RI2002585 | 0.012887308 | 128115 | 9 |
| NT2RI2002592 | 0.009086448 | 71443 | 2 |
| NT2RI2002602 | 0.021396088 | 162258 | 5 |
| NT2RI2002654 | 0.285223786 | 92848 | 41 |
| NT2RI2002699 | 0.014654697 | 136900 | 5 |
| NT2RI2002729 | 0.079664769 | 105954 | 6 |
| NT2RI2002802 | 0.005828436 | 176599 | 3 |
| NT2RI2002819 | 0.045005688 | 13987 | 11 |
| NT2RI2002822 | 0.033395273 | 113877 | 11 |
| NT2RI2002847 | 0.133480395 | 82109 | 35 |
| NT2RI2002852 | 0.00414016 | 251097 | 2 |
| NT2RI2002865 | 0.024710287 | 126630 | 6 |
| NT2RI2002923 | 0.003044882 | 266191 | 1 |
| NT2RI2002926 | 0.021231213 | 179949 | 4 |
| NT2RI2002958 | 0.17403637 | 82780 | 52 |
| NT2RI2002970 | 0.003044882 | 221862 | 1 |
| NT2RI2003011 | 0.048526964 | 188873 | 6 |
| NT2RI2003019 | 0.032037681 | 154019 | 9 |
| NT2RI2003051 | 0.16367109 | 78501 | 74 |
| NT2RI2003067 | 0.003044882 | 267074 | 1 |
| NT2RI2003154 | 0.008025098 | 150129 | 4 |
| NT2RI2003184 | 0.008886344 | 92677 | 2 |
| NT2RI2003205 | 0.052050932 | 70523 | 15 |
| NT2RI2003220 | 0.098656868 | 105048 | 44 |
| NT2RI2003222 | 0.006591273 | 144534 | 3 |
| NT2RI2003301 | 0.029521302 | 79538 | 13 |
| NT2RI2003304 | 0.638497477 | 73979 | 90 |
| NT2RI2003317 | 0.106297246 | 65520 | 35 |
| NT2RI2003338 | 0.165480193 | 75398 | 41 |
| NT2RI2003344 | 0.086041944 | 62148 | 55 |
| NT2RI2003360 | 0.003044882 | 988 | 1 |
| NT2RI2003361 | 0.033927008 | 28116 | 9 |
| NT2RI2003383 | 0.009235874 | 58084 | 3 |
| NT2RI2003392 | 0.003044882 | 265084 | 1 |
| NT2RI2003399 | 0.005235438 | 97734 | 3 |
| NT2RI2003407 | 0.138438197 | 108306 | 20 |
| NT2RI2003419 | 0.014842899 | 156419 | 2 |
| NT2RI2003420 | 0.108807576 | 73396 | 34 |
| NT2RI2003481 | 0.086771745 | 55408 | 21 |
| NT2RI2003487 | 0.044850902 | 223149 | 2 |
| NT2RI2003556 | 0.440412738 | 138145 | 29 |
| NT2RI2003655 | 0.008577242 | 97073 | 3 |
| NT2RI2003667 | 0.009117264 | 211893 | 2 |
| NT2RI2003678 | 0.088002754 | 136314 | 21 |
| NT2RI2003695 | 0.212783955 | 88709 | 47 |
| NT2RI2003738 | 0.957797157 | 77246 | 98 |
| NT2RI2003741 | 0.361548428 | 88362 | 93 |
| NT2RI2003751 | 0.12095225 | 98060 | 16 |
| NT2RI2003884 | 0.003044882 | 264344 | 1 |
| NT2RI2003921 | 0.35330779 | 57124 | 36 |
| NT2RI2003973 | 0.013348283 | 146329 | 5 |
| NT2RI2003993 | 0.044136676 | 168309 | 8 |
| NT2RI2004059 | 0.024589442 | 126823 | 5 |
| NT2RI2004078 | 0.004720429 | 175319 | 2 |
| NT2RI2004079 | 0.003044882 | 167611 | 1 |
| NT2RI2004093 | 0.006089763 | 69274 | 2 |
| NT2RI2004136 | 0.003044882 | 269521 | 1 |
| NT2RI2004157 | 0.005043283 | 102109 | 2 |
| NT2RI2004188 | 0.058353236 | 74415 | 9 |
| NT2RI2004190 | 0.003044882 | 159678 | 1 |
| NT2RI2004209 | 0.022822697 | 115204 | 5 |
| NT2RI2004230 | 0.1460284 | 100022 | 42 |
| NT2RI2004284 | 0.104236124 | 154196 | 6 |
| NT2RI2004290 | 0.007853965 | 86522 | 3 |
| NT2RI2004304 | 0.040236186 | 83227 | 21 |
| NT2RI2004312 | 0.011038656 | 160856 | 3 |
| NT2RI2004381 | 0.003044882 | 262480 | 1 |
| NT2RI2004398 | 0.033633816 | 94823 | 11 |
| NT2RI2004410 | 0.067181817 | 109096 | 24 |
| NT2RI2004442 | 0.003044882 | 89749 | 1 |
| NT2RI2004468 | 0.003044882 | 241362 | 1 |
| NT2RI2004504 | 0.596981765 | 61702 | 163 |
| NT2RI2004535 | 0.087535334 | 88770 | 28 |
| NT2RI2004606 | 0.004720429 | 73678 | 2 |
| NT2RI2004608 | 0.045288383 | 92709 | 10 |
| NT2RI2004618 | 0.003044882 | 277304 | 1 |
| NT2RI2004783 | 0.00776531 | 136794 | 3 |
| NT2RI2004818 | 0.003044882 | 137370 | 1 |
| NT2RI2004840 | 0.003044882 | 219054 | 1 |
| NT2RI2004884 | 0.216864488 | 98742 | 17 |
| NT2RI2004916 | 0.010128046 | 156110 | 3 |
| NT2RI2004962 | 0.003044882 | 101242 | 1 |
| NT2RI2004984 | 0.003044882 | 35529 | 1 |
| NT2RI2004985 | 0.003044882 | 130525 | 1 |
| NT2RI2005048 | 0.003044882 | 161917 | 1 |
| NT2RI2005061 | 0.03841027 | 148193 | 10 |
| NT2RI2005069 | 0.003044882 | 170390 | 1 |
| NT2RI2005087 | 0.039080918 | 141352 | 2 |
| NT2RI2005096 | 0.00414016 | 111223 | 2 |
| NT2RI2005115 | 0.027419703 | 105208 | 7 |
| NT2RI2005116 | 0.131078745 | 119430 | 26 |
| NT2RI2005130 | 0.012162146 | 167607 | 3 |
| NT2RI2005150 | 0.243160742 | 17820 | 42 |
| NT2RI2005166 | 0.006424683 | 180327 | 4 |
| NT2RI2005239 | 0.042487094 | 85121 | 15 |
| NT2RI2005246 | 0.01804796 | 15530 | 3 |
| NT2RI2005315 | 0.006165688 | 81983 | 2 |
| NT2RI2005335 | 0.432856271 | 23512 | 133 |
| NT2RI2005353 | 0.14545991 | 114168 | 22 |
| NT2RI2005358 | 0.008164089 | 157041 | 3 |
| NT2RI2005368 | 0.082926386 | 119652 | 16 |
| NT2RI2005382 | 0.00847057 | 77602 | 4 |
| NT2RI2005405 | 0.596878174 | 87082 | 73 |
| NT2RI2005473 | 0.051301787 | 243301 | 3 |
| NT2RI2005520 | 0.003044882 | 3204 | 1 |
| NT2RI2005564 | 0.037618515 | 61213 | 15 |
| NT2RI2005579 | 0.01785392 | 70182 | 6 |
| NT2RI2005621 | 0.003044882 | 189481 | 1 |
| NT2RI2005628 | 0.003044882 | 185344 | 1 |
| NT2RI2005647 | 0.253382024 | 4698 | 26 |
| NT2RI2005670 | 0.003044882 | 128365 | 1 |
| NT2RI2005710 | 0.032099741 | 53296 | 8 |
| NT2RI2005713 | 0.113302567 | 93611 | 26 |
| NT2RI2005723 | 0.333894126 | 113962 | 63 |
| NT2RI2005772 | 0.009134645 | 165778 | 3 |
| NT2RI2005811 | 0.109366709 | 159985 | 20 |
| NT2RI2005814 | 0.128650226 | 98883 | 11 |
| NT2RI2005816 | 0.026214124 | 32105 | 11 |
| NT2RI2005818 | 0.065786717 | 67172 | 20 |
| NT2RI2005851 | 0.044962203 | 121097 | 16 |
| NT2RI2005966 | 0.008987005 | 281478 | 2 |
| NT2RI2006070 | 0.08152819 | 134910 | 14 |
| NT2RI2006071 | 0.059355294 | 30415 | 42 |
| NT2RI2006072 | 0.218997109 | 166427 | 26 |
| NT2RI2006081 | 0.003044882 | 279262 | 1 |
| NT2RI2006126 | 0.003044882 | 151537 | 1 |
| NT2RI2006127 | 0.013872079 | 187634 | 2 |
| NT2RI2006183 | 0.017518546 | 186256 | 5 |
| NT2RI2006210 | 0.028243819 | 145027 | 5 |
| NT2RI2006257 | 0.003044882 | 277940 | 1 |
| NT2RI2006271 | 0.059990396 | 150039 | 6 |
| NT2RI2006294 | 0.003044882 | 61397 | 1 |
| NT2RI2006345 | 0.003044882 | 57811 | 1 |
| NT2RI2006403 | 0.003044882 | 191348 | 1 |
| NT2RI2006412 | 0.204407794 | 70407 | 65 |
| NT2RI2006445 | 0.003044882 | 12994 | 1 |
| NT2RI2006487 | 0.003044882 | 194241 | 1 |
| NT2RI2006506 | 0.012437974 | 161711 | 5 |
| NT2RI2006553 | 0.045302344 | 154796 | 7 |
| NT2RI2006565 | 0.028040899 | 145153 | 8 |
| NT2RI2006640 | 0.026659501 | 99387 | 10 |
| NT2RI2006647 | 0.036540964 | 135251 | 10 |
| NT2RI2006659 | 0.007083164 | 210389 | 2 |
| NT2RI2006662 | 0.003044882 | 95367 | 1 |
| NT2RI2006667 | 0.003044882 | 276968 | 1 |
| NT2RI2006679 | 0.003044882 | 71261 | 1 |
| NT2RI2006682 | 0.068092458 | 132383 | 18 |
| NT2RI2006686 | 0.014331563 | 193529 | 2 |
| NT2RI2006703 | 0.010338434 | 195054 | 4 |
| NT2RI2006716 | 0.003044882 | 234816 | 1 |
| NT2RI2006735 | 0.010645973 | 126312 | 5 |
| NT2RI2006762 | 0.003044882 | 277525 | 1 |
| NT2RI2006788 | 0.003044882 | 76851 | 1 |
| NT2RI2006825 | 0.061115679 | 112900 | 17 |
| NT2RI2006838 | 0.003044882 | 235587 | 1 |
| NT2RI2006853 | 0.074701306 | 152329 | 13 |
| NT2RI2006855 | 0.019436977 | 33831 | 5 |
| NT2RI2006856 | 0.357722292 | 10092 | 37 |
| NT2RI2006943 | 0.137764997 | 105442 | 29 |
| NT2RI2006973 | 0.003044882 | 66509 | 1 |
| NT2RI2007046 | 0.003044882 | 121712 | 1 |
| NT2RI2007048 | 0.003044882 | 178696 | 1 |
| NT2RI2007054 | 0.129060942 | 141191 | 27 |
| NT2RI2007084 | 0.154013668 | 134319 | 19 |
| NT2RI2007096 | 0.03232221 | 135052 | 13 |
| NT2RI2007116 | 0.003044882 | 277095 | 1 |
| NT2RI2007133 | 0.427118898 | 18766 | 23 |
| NT2RI2007148 | 0.177422997 | 122768 | 10 |
| NT2RI2007214 | 0.007083164 | 152627 | 2 |
| NT2RI2007254 | 0.212783955 | 88709 | 47 |
| NT2RI2007277 | 0.170915185 | 83693 | 51 |
| NT2RI2007303 | 0.003044882 | 269437 | 1 |
| NT2RI2007334 | 0.013280288 | 99955 | 4 |
| NT2RI2007377 | 0.143065284 | 15432 | 46 |
| NT2RI2007384 | 0.386921652 | 44065 | 46 |
| NT2RI2007386 | 0.003044882 | 222351 | 1 |
| NT2RI2007406 | 0.009117264 | 225482 | 2 |
| NT2RI2007439 | 0.009117264 | 166218 | 2 |
| NT2RI2007445 | 0.093447469 | 132737 | 15 |
| NT2RI2007468 | 0.040101278 | 118435 | 12 |
| NT2RI2007469 | 0.005235438 | 166352 | 3 |
| NT2RI2007498 | 0.410872964 | 105083 | 43 |
| NT2RI2007507 | 0.003044882 | 274289 | 1 |
| NT2RI2007572 | 0.224251358 | 125514 | 49 |
| NT2RI2007589 | 0.156897572 | 106862 | 30 |
| NT2RI2007593 | 0.050855316 | 164367 | 12 |
| NT2RI2007629 | 0.083120959 | 104318 | 19 |
| NT2RI2007723 | 0.047362479 | 12494 | 9 |
| NT2RI2007729 | 0.014508827 | 174315 | 2 |
| NT2RI2007751 | 0.003044882 | 143596 | 1 |
| NT2RI2007754 | 0.017267943 | 146783 | 3 |
| NT2RI2007827 | 0.022319642 | 54119 | 3 |
| NT2RI2007853 | 0.07483736 | 121114 | 6 |
| NT2RI2007877 | 0.003044882 | 281851 | 1 |
| NT2RI2007879 | 0.105570783 | 7840 | 8 |
| NT2RI2007884 | 0.065999801 | 132268 | 17 |
| NT2RI2007891 | 0.003044882 | 104677 | 1 |
| NT2RI2007956 | 0.003044882 | 165126 | 1 |
| NT2RI2007965 | 0.007083164 | 123285 | 2 |
| NT2RI2007987 | 0.093058368 | 114503 | 27 |
| NT2RI2008007 | 0.008657175 | 31356 | 3 |
| NT2RI2008045 | 0.215801178 | 81138 | 33 |
| NT2RI2008050 | 0.003044882 | 238265 | 1 |
| NT2RI2008141 | 0.078359933 | 2764 | 20 |
| NT2RI2008171 | 0.037735551 | 145131 | 9 |
| NT2RI2008188 | 0.005828436 | 103711 | 3 |
| NT2RI2008204 | 0.026996023 | 156829 | 4 |
| NT2RI2008221 | 0.151134943 | 47257 | 5 |
| NT2RI2008233 | 0.00885006 | 124666 | 2 |
| NT2RI2008336 | 0.418255567 | 63559 | 57 |
| NT2RI2008396 | 0.380124981 | 54223 | 72 |
| NT2RI2008455 | 0.003044882 | 261425 | 1 |
| NT2RI2008481 | 0.226557321 | 63654 | 18 |
| NT2RI2008502 | 0.003044882 | 276425 | 1 |
| NT2RI2008526 | 0.218544249 | 12110 | 22 |
| NT2RI2008566 | 0.017814449 | 161829 | 8 |
| NT2RI2008598 | 0.019974768 | 77898 | 3 |
| NT2RI2008622 | 0.053094151 | 148594 | 9 |
| NT2RI2008656 | 0.061695007 | 172428 | 6 |
| NT2RI2008714 | 0.011419083 | 106996 | 5 |
| NT2RI2008724 | 0.091237502 | 70597 | 17 |
| NT2RI2008749 | 0.296348512 | 127582 | 27 |
| NT2RI2008791 | 0.143610146 | 66082 | 21 |
| NT2RI2008801 | 0.003044882 | 100350 | 1 |
| NT2RI2008808 | 0.036961263 | 90643 | 13 |
| NT2RI2008812 | 0.023144058 | 178312 | 5 |
| NT2RI2008841 | 0.003044882 | 280095 | 1 |
| NT2RI2008942 | 0.159783144 | 96198 | 59 |
| NT2RI2008952 | 0.003044882 | 184776 | 1 |
| NT2RI2009005 | 0.003044882 | 150884 | 1 |
| NT2RI2009037 | 0.198750868 | 102294 | 48 |
| NT2RI2009065 | 0.358761545 | 113793 | 52 |
| NT2RI2009066 | 0.061133341 | 123911 | 23 |
| NT2RI2009083 | 0.016341141 | 91711 | 9 |
| NT2RI2009101 | 0.003044882 | 271187 | 1 |
| NT2RI2009103 | 0.005235438 | 74366 | 3 |
| NT2RI2009144 | 0.003044882 | 173644 | 1 |
| NT2RI2009151 | 0.003044882 | 165498 | 1 |
| NT2RI2009173 | 0.150224077 | 132145 | 22 |
| NT2RI2009194 | 0.170496847 | 72698 | 29 |
| NT2RI2009215 | 0.003044882 | 197964 | 1 |
| NT2RI2009233 | 0.074284106 | 135421 | 23 |
| NT2RI2009239 | 0.119877738 | 49116 | 16 |
| NT2RI2009259 | 0.01431663 | 63363 | 3 |
| NT2RI2009269 | 0.169558506 | 123881 | 38 |
| NT2RI2009281 | 0.119052761 | 130866 | 21 |
| NT2RI2009289 | 0.003044882 | 109857 | 1 |
| NT2RI2009301 | 0.003044882 | 217643 | 1 |
| NT2RI2009402 | 0.053422391 | 86534 | 20 |
| NT2RI2009406 | 0.059555623 | 110874 | 17 |
| NT2RI2009517 | 0.003044882 | 84334 | 1 |
| NT2RI2009583 | 0.748889309 | 20637 | 112 |
| NT2RI2009585 | 0.003044882 | 146048 | 1 |
| NT2RI2009595 | 0.003044882 | 133190 | 1 |
| NT2RI2009855 | 0.006089763 | 194418 | 2 |
| NT2RI2010283 | 0.022640858 | 133082 | 4 |
| NT2RI2010508 | 0.003044882 | 67418 | 1 |
| NT2RI2010795 | 0.010158847 | 160033 | 5 |
| NT2RI2011422 | 0.003044882 | 150362 | 1 |
| NT2RI2011450 | 0.00659727 | 176848 | 3 |
| NT2RI2011683 | 0.003044882 | 180964 | 1 |
| NT2RI2011803 | 0.003044882 | 194297 | 1 |
| NT2RI2012350 | 0.418632789 | 144259 | 15 |
| NT2RI2012659 | 0.012354928 | 132403 | 8 |
| NT2RI2012726 | 0.036118115 | 115299 | 17 |
| NT2RI2012990 | 0.012499358 | 119171 | 2 |
| NT2RI2013345 | 0.01771657 | 127779 | 4 |
| NT2RI2013357 | 0.00414016 | 151758 | 2 |
| NT2RI2013373 | 0.003044882 | 211678 | 1 |
| NT2RI2014247 | 0.010985407 | 168213 | 3 |
| NT2RI2014551 | 0.003044882 | 134756 | 1 |
| NT2RI2014683 | 0.003044882 | 17687 | 1 |
| NT2RI2014733 | 0.00671883 | 191281 | 3 |
| NT2RI2015239 | 0.003044882 | 285005 | 1 |
| NT2RI2015342 | 0.111379131 | 57240 | 29 |
| NT2RI2015533 | 0.107813525 | 90441 | 43 |
| NT2RI2016128 | 0.003044882 | 182040 | 1 |
| NT2RI2016134 | 0.003044882 | 233335 | 1 |
| NT2RI2017529 | 0.172691052 | 140179 | 8 |
| NT2RI2018311 | 0.007799223 | 250765 | 3 |
| NT2RI2018363 | 0.003044882 | 126729 | 1 |
| NT2RI2018448 | 0.026610305 | 174603 | 7 |
| NT2RI2018883 | 0.006165688 | 169641 | 2 |
| NT2RI2018950 | 0.003044882 | 149010 | 1 |
| NT2RI2019751 | 0.004234126 | 163732 | 2 |
| NT2RI2019826 | 0.003044882 | 248705 | 1 |
| NT2RI2020390 | 0.003044882 | 226308 | 1 |
| NT2RI2020703 | 0.003044882 | 136587 | 1 |
| NT2RI2021463 | 0.006089763 | 143886 | 2 |
| NT2RI2021625 | 0.008792625 | 42350 | 3 |
| NT2RI2022468 | 0.024995459 | 144876 | 5 |
| NT2RI2022584 | 0.003044882 | 21601 | 1 |
| NT2RI2023303 | 0.003044882 | 233779 | 1 |
| NT2RI2023671 | 0.003044882 | 118766 | 1 |
| NT2RI2024008 | 0.004921723 | 149403 | 2 |
| NT2RI2024313 | 0.011435733 | 146362 | 7 |
| NT2RI2024460 | 0.003044882 | 151439 | 1 |
| NT2RI2024496 | 0.003044882 | 242707 | 1 |
| NT2RI2024935 | 0.03856886 | 144166 | 2 |
| NT2RI2024971 | 0.141887343 | 139080 | 14 |
| NT2RI2025075 | 0.009883213 | 125638 | 5 |
| NT2RI2025255 | 0.004902996 | 209782 | 2 |
| NT2RI2025564 | 0.003044882 | 243380 | 1 |
| NT2RI2025565 | 0.003044882 | 42513 | 1 |
| NT2RI2025909 | 0.003044882 | 69262 | 1 |
| NT2RI2025957 | 0.003044882 | 109316 | 1 |
| NT2RI2025976 | 0.003044882 | 158116 | 1 |
| NT2RI2026758 | 0.003044882 | 150895 | 1 |
| NT2RI2027081 | 0.003044882 | 229968 | 1 |
| NT2RI2027157 | 0.006089763 | 232592 | 2 |
| NT2RI2027224 | 0.004921723 | 270109 | 2 |
| NT2RI2027323 | 0.005043283 | 130031 | 2 |
| NT2RI2027396 | 0.003044882 | 182073 | 1 |
| NT2RI2027484 | 0.003044882 | 259189 | 1 |
| NT2RI2027975 | 0.004234126 | 228832 | 2 |
| NT2RI2028213 | 0.003044882 | 143927 | 1 |
| NT2RI2028222 | 0.003044882 | 283238 | 1 |
| NT2RI2028537 | 0.003044882 | 125227 | 1 |
| NT2RI2028642 | 0.003044882 | 183719 | 1 |
| NT2RI3000174 | 0.004921723 | 136817 | 2 |
| NT2RI3000213 | 0.003044882 | 269708 | 1 |
| NT2RI3000423 | 0.034845616 | 114406 | 15 |
| NT2RI3000622 | 0.019570545 | 77861 | 3 |
| NT2RI3001132 | 0.003044882 | 86569 | 1 |
| NT2RI3001263 | 0.003044882 | 235130 | 1 |
| NT2RI3001279 | 0.003044882 | 189135 | 1 |
| NT2RI3001295 | 0.011362752 | 169855 | 3 |
| NT2RI3001348 | 0.016032144 | 91744 | 3 |
| NT2RI3001445 | 0.026455484 | 184476 | 12 |
| NT2RI3001458 | 0.003044882 | 73885 | 1 |
| NT2RI3001515 | 0.015470709 | 39278 | 8 |
| NT2RI3001573 | 0.003044882 | 145584 | 1 |
| NT2RI3001967 | 0.003044882 | 279800 | 1 |
| NT2RI3001981 | 0.157446218 | 2665 | 33 |
| NT2RI3002303 | 0.003044882 | 198651 | 1 |
| NT2RI3002557 | 0.003044882 | 218158 | 1 |
| NT2RI3002842 | 0.008941279 | 139266 | 3 |
| NT2RI3002892 | 0.095212005 | 86533 | 46 |
| NT2RI3003027 | 0.003044882 | 174130 | 1 |
| NT2RI3003031 | 0.003044882 | 174131 | 1 |
| NT2RI3003095 | 0.003044882 | 152334 | 1 |
| NT2RI3003104 | 0.010711829 | 50141 | 8 |
| NT2RI3003162 | 0.003044882 | 132808 | 1 |
| NT2RI3003362 | 0.003044882 | 197726 | 1 |
| NT2RI3003382 | 0.004234126 | 121782 | 2 |
| NT2RI3003409 | 0.003044882 | 233343 | 1 |
| NT2RI3003738 | 0.00923839 | 125360 | 4 |
| NT2RI3003925 | 0.004754342 | 175982 | 2 |
| NT2RI3004133 | 0.003044882 | 38690 | 1 |
| NT2RI3004161 | 0.035628456 | 162731 | 13 |
| NT2RI3004381 | 0.003044882 | 164379 | 1 |
| NT2RI3004510 | 0.005943586 | 218272 | 3 |
| NT2RI3005202 | 0.003044882 | 130236 | 1 |
| NT2RI3005403 | 0.004902996 | 207252 | 2 |
| NT2RI3005416 | 0.003044882 | 69882 | 1 |
| NT2RI3005724 | 0.028271764 | 141725 | 18 |
| NT2RI3005861 | 0.003044882 | 284680 | 1 |
| NT2RI3005923 | 0.008595672 | 117247 | 4 |
| NT2RI3005928 | 0.006089763 | 67160 | 2 |
| NT2RI3006132 | 0.003044882 | 179692 | 1 |
| NT2RI3006171 | 0.003044882 | 219880 | 1 |
| NT2RI3006284 | 0.080982065 | 124928 | 16 |
| NT2RI3006340 | 0.020518922 | 22737 | 6 |
| NT2RI3006376 | 0.003044882 | 103518 | 1 |
| NT2RI3006666 | 0.008025242 | 173492 | 3 |
| NT2RI3006673 | 0.016789762 | 65471 | 8 |
| NT2RI3006796 | 0.006089763 | 202253 | 2 |
| NT2RI3007065 | 0.003044882 | 163412 | 1 |
| NT2RI3007095 | 0.003044882 | 194435 | 1 |
| NT2RI3007158 | 0.003044882 | 156763 | 1 |
| NT2RI3007167 | 0.003044882 | 114140 | 1 |
| NT2RI3007213 | 0.004234126 | 243335 | 2 |
| NT2RI3007291 | 0.013386563 | 142861 | 8 |
| NT2RI3007443 | 0.003044882 | 206758 | 1 |
| NT2RI3007543 | 0.197485233 | 119874 | 39 |
| NT2RI3007684 | 0.003044882 | 75412 | 1 |
| NT2RI3007757 | 0.013248852 | 139899 | 4 |
| NT2RI3007878 | 0.003044882 | 64176 | 1 |
| NT2RI3007978 | 0.003044882 | 283720 | 1 |
| NT2RI3008055 | 0.00758522 | 194446 | 4 |
| NT2RI3008162 | 0.003044882 | 109056 | 1 |
| NT2RI3008179 | 0.003044882 | 163400 | 1 |
| NT2RI3008228 | 0.003044882 | 281552 | 1 |
| NT2RI3008442 | 0.003044882 | 207022 | 1 |
| NT2RI3008652 | 0.003044882 | 271688 | 1 |
| NT2RI3008697 | 0.003044882 | 32350 | 1 |
| NT2RI3008974 | 0.003044882 | 209444 | 1 |
| NT2RI3009158 | 0.006658773 | 250563 | 2 |
| NT2RI3009480 | 0.004442037 | 136647 | 2 |
| NT2RI3009524 | 0.004720429 | 153188 | 2 |
| NT2RP6000005 | 0.076178169 | 15691 | 9 |
| NT2RP6000008 | 0.010455055 | 54556 | 4 |
| NT2RP6000017 | 0.111988015 | 61289 | 22 |
| NT2RP6000032 | 0.0213659 | 30083 | 4 |
| NT2RP6000033 | 0.040283299 | 61675 | 2 |
| NT2RP6000035 | 0.380124981 | 54223 | 72 |
| NT2RP6000039 | 0.093440803 | 64972 | 30 |
| NT2RP6000043 | 0.04411439 | 70295 | 10 |
| NT2RP6000059 | 0.007724961 | 29351 | 3 |
| NT2RP6000060 | 0.004038283 | 5023 | 1 |
| NT2RP6000072 | 0.10369656 | 43553 | 22 |
| NT2RP6000077 | 0.02606703 | 34972 | 9 |
| NT2RP6000078 | 0.466009153 | 63995 | 158 |
| NT2RP6000085 | 0.036228731 | 5879 | 21 |
| NT2RP6000086 | 0.005227527 | 67000 | 2 |
| NT2RP6000100 | 0.007652175 | 27365 | 2 |
| NT2RP6000109 | 0.087729946 | 67233 | 21 |
| NT2RP6000110 | 0.061189315 | 44496 | 28 |
| NT2RP6000119 | 0.004038283 | 6447 | 1 |
| NT2RP6000123 | 0.428032826 | 65407 | 72 |
| NT2RP6000127 | 0.004038283 | 30409 | 1 |
| NT2RP6000130 | 0.020932399 | 54818 | 4 |
| NT2RP6000140 | 0.004038283 | 868 | 1 |
| NT2RP6000150 | 0.004038283 | 49374 | 1 |
| NT2RP7000041 | 0.004038283 | 269761 | 1 |
| NT2RP7000069 | 0.049742589 | 113385 | 27 |
| NT2RP7000076 | 0.274938982 | 76237 | 55 |
| NT2RP7000086 | 0.013102697 | 52165 | 6 |
| NT2RP7000109 | 0.004038283 | 91775 | 1 |
| NT2RP7000112 | 0.010431807 | 46277 | 4 |
| NT2RP7000173 | 0.301387854 | 83961 | 96 |
| NT2RP7000238 | 0.129438892 | 111656 | 37 |
| NT2RP7000259 | 0.012530034 | 142332 | 5 |
| NT2RP7000271 | 0.05524676 | 120030 | 18 |
| NT2RP7000311 | 0.004038283 | 37611 | 1 |
| NT2RP7000359 | 0.004038283 | 131027 | 1 |
| NT2RP7000364 | 0.004038283 | 154609 | 1 |
| NT2RP7000380 | 0.004038283 | 220361 | 1 |
| NT2RP7000391 | 0.004038283 | 274012 | 1 |
| NT2RP7000425 | 0.008215218 | 185000 | 2 |
| NT2RP7000466 | 0.19248918 | 150521 | 19 |
| NT2RP7000477 | 0.084691945 | 74926 | 28 |
| NT2RP7000579 | 0.00926581 | 114563 | 3 |
| NT2RP7000586 | 0.015250128 | 116473 | 7 |
| NT2RP7000600 | 0.075614279 | 95942 | 38 |
| NT2RP7000624 | 0.038404074 | 113338 | 17 |
| NT2RP7000812 | 0.020998132 | 47819 | 3 |
| NT2RP7000876 | 0.074083011 | 100148 | 16 |
| NT2RP7000906 | 0.128445545 | 36763 | 50 |
| NT2RP7000926 | 0.040382829 | 139609 | 10 |
| NT2RP7001074 | 0.004038283 | 164447 | 1 |
| NT2RP7001080 | 0.050060872 | 52876 | 20 |
| NT2RP7001156 | 0.005133561 | 192119 | 2 |
| NT2RP7001166 | 0.224035746 | 113203 | 58 |
| NT2RP7001231 | 0.040572531 | 95153 | 18 |
| NT2RP7001283 | 0.29768157 | 22378 | 44 |
| NT2RP7001306 | 0.023035709 | 97659 | 3 |
| NT2RP7001319 | 0.004038283 | 99441 | 1 |
| NT2RP7001335 | 0.080201941 | 78877 | 22 |
| NT2RP7001364 | 0.00572656 | 279408 | 2 |
| NT2RP7001475 | 0.009537578 | 133602 | 4 |
| NT2RP7001532 | 0.004038283 | 163280 | 1 |
| NT2RP7001591 | 0.064447731 | 143106 | 7 |
| NT2RP7001602 | 0.004038283 | 266651 | 1 |
| NT2RP7001678 | 0.010772981 | 117859 | 3 |
| NT2RP7001745 | 0.108807576 | 73396 | 34 |
| NT2RP7001856 | 0.048889185 | 107453 | 3 |
| NT2RP7001962 | 0.063075103 | 104839 | 19 |
| NT2RP7002028 | 0.083379247 | 115298 | 15 |
| NT2RP7002064 | 0.007571944 | 55122 | 3 |
| NT2RP7002151 | 0.006119795 | 216146 | 2 |
| NT2RP7002163 | 0.004038283 | 222728 | 1 |
| NT2RP7002243 | 0.008201307 | 92650 | 3 |
| NT2RP7002282 | 0.010911494 | 65397 | 4 |
| NT2RP7002376 | 0.013809916 | 87666 | 6 |
| NT2RP7002379 | 0.332851147 | 91392 | 82 |
| NT2RP7002449 | 0.082940386 | 116018 | 34 |
| NT2RP7002450 | 0.06278006 | 110745 | 8 |
| NT2RP7002478 | 0.005747743 | 71529 | 2 |
| NT2RP7002554 | 0.004038283 | 269221 | 1 |
| NT2RP7002594 | 0.012266286 | 7603 | 5 |
| NT2RP7002603 | 0.004038283 | 275386 | 1 |
| NT2RP7002619 | 0.153785301 | 115895 | 21 |
| NT2RP7002650 | 0.004038283 | 270976 | 1 |
| NT2RP7002738 | 0.029107266 | 94672 | 13 |
| NT2RP7002779 | 0.005133561 | 87622 | 2 |
| NT2RP7002802 | 0.045849277 | 48683 | 17 |
| NT2RP7002829 | 0.019162507 | 112813 | 5 |
| NT2RP7002841 | 0.004038283 | 270005 | 1 |
| NT2RP7002875 | 0.181580261 | 45064 | 70 |
| NT2RP7002906 | 0.059763947 | 130515 | 21 |
| NT2RP7002978 | 0.004038283 | 67366 | 1 |
| NT2RP7002982 | 0.043102829 | 130158 | 11 |
| NT2RP7003025 | 0.004038283 | 184472 | 1 |
| NT2RP7003050 | 0.004038283 | 260561 | 1 |
| NT2RP7003055 | 0.015878856 | 153647 | 5 |
| NT2RP7003084 | 0.004038283 | 114420 | 1 |
| NT2RP7003091 | 0.004038283 | 134514 | 1 |
| NT2RP7003107 | 0.004038283 | 142608 | 1 |
| NT2RP7003134 | 0.004038283 | 190805 | 1 |
| NT2RP7003148 | 0.004038283 | 141947 | 1 |
| NT2RP7003203 | 0.090709641 | 59937 | 33 |
| NT2RP7003261 | 0.123329298 | 41599 | 20 |
| NT2RP7003304 | 0.118635564 | 90575 | 55 |
| NT2RP7003319 | 0.183647528 | 105733 | 64 |
| NT2RP7003439 | 0.004038283 | 232331 | 1 |
| NT2RP7003511 | 0.050705643 | 125339 | 16 |
| NT2RP7003629 | 0.014808438 | 77952 | 7 |
| NT2RP7003632 | 0.10290126 | 128010 | 24 |
| NT2RP7003647 | 0.142371141 | 85875 | 69 |
| NT2RP7003680 | 0.022461425 | 84281 | 9 |
| NT2RP7003688 | 0.226913699 | 119317 | 88 |
| NT2RP7003724 | 0.018144711 | 131537 | 9 |
| NT2RP7003960 | 0.10113201 | 144088 | 23 |
| NT2RP7004027 | 0.004038283 | 224197 | 1 |
| NT2RP7004080 | 0.004038283 | 109925 | 1 |
| NT2RP7004114 | 0.030048915 | 44516 | 11 |
| NT2RP7004123 | 0.087315579 | 56288 | 14 |
| NT2RP7004173 | 0.219398702 | 86661 | 51 |
| NT2RP7004196 | 0.127886066 | 104021 | 37 |
| NT2RP7004204 | 0.098263684 | 90300 | 21 |
| NT2RP7004233 | 0.01401255 | 150859 | 6 |
| NT2RP7004260 | 0.006530717 | 99380 | 3 |
| NT2RP7004348 | 0.074143863 | 18669 | 21 |
| NT2RP7004352 | 0.098656868 | 105048 | 44 |
| NT2RP7004358 | 0.004038283 | 73573 | 1 |
| NT2RP7004373 | 0.142361962 | 76852 | 49 |
| NT2RP7004396 | 0.004038283 | 142300 | 1 |
| NT2RP7004428 | 0.049683901 | 58797 | 22 |
| NT2RP7004481 | 0.031155526 | 93784 | 12 |
| NT2RP7004541 | 0.00695314 | 103974 | 2 |
| NT2RP7004559 | 0.019658121 | 101315 | 4 |
| NT2RP7004641 | 0.006530717 | 142071 | 3 |
| NT2RP7004656 | 0.007791966 | 100550 | 3 |
| NT2RP7004687 | 0.008887244 | 176653 | 4 |
| NT2RP7004722 | 0.004038283 | 281205 | 1 |
| NT2RP7004728 | 0.106603688 | 132891 | 36 |
| NT2RP7004751 | 0.357991542 | 21028 | 29 |
| NT2RP7004766 | 0.004038283 | 268800 | 1 |
| NT2RP7004790 | 0.021469442 | 105506 | 8 |
| NT2RP7004884 | 0.061033184 | 112079 | 25 |
| NT2RP7004911 | 0.095062189 | 112662 | 25 |
| NT2RP7004915 | 0.022570023 | 133371 | 10 |
| NT2RP7004925 | 0.132701628 | 63684 | 35 |
| NT2RP7004946 | 0.194637197 | 59625 | 68 |
| NT2RP7004961 | 0.009953407 | 150743 | 3 |
| NT2RP7004964 | 0.004038283 | 218051 | 1 |
| NT2RP7004975 | 0.004038283 | 220202 | 1 |
| NT2RP7005118 | 0.004038283 | 271306 | 1 |
| NT2RP7005205 | 0.004038283 | 218013 | 1 |
| NT2RP7005219 | 0.006416772 | 96869 | 3 |
| NT2RP7005323 | 0.004038283 | 236568 | 1 |
| NT2RP7005468 | 0.009164676 | 201673 | 3 |
| NT2RP7005493 | 0.013846334 | 148931 | 4 |
| NT2RP7005502 | 0.119877738 | 49116 | 16 |
| NT2RP7005513 | 0.099133722 | 31539 | 11 |
| NT2RP7005520 | 0.055859713 | 156866 | 12 |
| NT2RP7005529 | 0.022694623 | 141686 | 11 |
| NT2RP7005629 | 0.004038283 | 161150 | 1 |
| NT2RP7005631 | 0.004038283 | 208386 | 1 |
| NT2RP7005669 | 0.004038283 | 153070 | 1 |
| NT2RP7005675 | 0.057614967 | 75515 | 33 |
| NT2RP7005750 | 0.046827762 | 98076 | 16 |
| NT2RP7005846 | 0.004038283 | 188809 | 1 |
| NT2RP7006033 | 0.021795628 | 193708 | 8 |
| NT2RP7006075 | 0.063952128 | 136518 | 14 |
| NT2RP7006141 | 0.226889501 | 45902 | 26 |
| NT2RP7006160 | 0.004038283 | 197282 | 1 |
| NT2RP7006162 | 0.005227527 | 76680 | 2 |
| NT2RP7006188 | 0.064360101 | 88606 | 25 |
| NT2RP7006223 | 0.03965534 | 17718 | 19 |
| NT2RP7006263 | 0.04161189 | 76426 | 11 |
| NT2RP7006296 | 0.005133561 | 192274 | 2 |
| NT2RP7006304 | 0.005133561 | 27331 | 2 |
| NT2RP7006374 | 0.004038283 | 273470 | 1 |
| NT2RP7006395 | 0.009786026 | 35644 | 3 |
| NT2RP7006408 | 0.004038283 | 119602 | 1 |
| NT2RP7006457 | 0.008076566 | 161066 | 2 |
| NT2RP7006490 | 0.078060076 | 141871 | 14 |
| NT2RP7006527 | 0.165480193 | 75398 | 41 |
| NT2RP7006539 | 0.057592728 | 130971 | 13 |
| NT2RP7006547 | 0.005133561 | 5916 | 2 |
| NT2RP7006601 | 0.005915125 | 200548 | 2 |
| NT2RP7006619 | 0.075911455 | 35829 | 18 |
| NT2RP7006621 | 0.004038283 | 212913 | 1 |
| NT2RP7006636 | 0.004038283 | 51186 | 1 |
| NT2RP7006701 | 0.033326419 | 8826 | 9 |
| NT2RP7006717 | 0.254681494 | 47020 | 65 |
| NT2RP7006853 | 0.004038283 | 268915 | 1 |
| NT2RP7006886 | 0.01761676 | 18761 | 6 |
| NT2RP7006887 | 0.004038283 | 266775 | 1 |
| NT2RP7006980 | 0.009935099 | 113362 | 3 |
| NT2RP7006986 | 0.044602784 | 89686 | 12 |
| NT2RP7006995 | 0.007791966 | 221987 | 3 |
| NT2RP7007100 | 0.216513216 | 79998 | 83 |
| NT2RP7007114 | 0.010227459 | 37630 | 4 |
| NT2RP7007154 | 0.293831862 | 76905 | 29 |
| NT2RP7007177 | 0.123039235 | 51733 | 23 |
| NT2RP7007221 | 0.004038283 | 114433 | 1 |
| NT2RP7007226 | 0.004038283 | 122054 | 1 |
| NT2RP7007252 | 0.024400287 | 120769 | 14 |
| NT2RP7007269 | 0.007133779 | 222429 | 2 |
| NT2RP7007310 | 0.044993474 | 106402 | 3 |
| NT2RP7007359 | 0.004038283 | 194321 | 1 |
| NT2RP7007381 | 0.010684351 | 167370 | 5 |
| NT2RP7007387 | 0.081631537 | 118997 | 25 |
| NT2RP7007406 | 0.005435438 | 256496 | 2 |
| NT2RP7007480 | 0.008076566 | 88575 | 2 |
| NT2RP7007504 | 0.004038283 | 55564 | 1 |
| NT2RP7007524 | 0.049128914 | 143252 | 23 |
| NT2RP7007530 | 0.076428255 | 84475 | 15 |
| NT2RP7007537 | 0.136267014 | 50935 | 33 |
| NT2RP7007580 | 0.007010403 | 123339 | 3 |
| NT2RP7007594 | 0.004038283 | 179431 | 1 |
| NT2RP7007610 | 0.057895736 | 78252 | 21 |
| NT2RP7007617 | 0.010650739 | 89498 | 4 |
| NT2RP7007643 | 0.005133561 | 72414 | 2 |
| NT2RP7007766 | 0.005435438 | 187003 | 2 |
| NT2RP7007842 | 0.087359377 | 137047 | 20 |
| NT2RP7007925 | 0.004038283 | 195304 | 1 |
| NT2RP7007930 | 0.005133561 | 234959 | 2 |
| NT2RP7007975 | 0.122783079 | 98768 | 42 |
| NT2RP7008013 | 0.004038283 | 106409 | 1 |
| NT2RP7008015 | 0.166064989 | 135633 | 8 |
| NT2RP7008085 | 0.253789888 | 84562 | 87 |
| NT2RP7008133 | 0.004038283 | 62179 | 1 |
| NT2RP7008137 | 0.004038283 | 154411 | 1 |
| NT2RP7008142 | 0.004038283 | 266350 | 1 |
| NT2RP7008144 | 0.004038283 | 48898 | 1 |
| NT2RP7008161 | 0.026688097 | 102587. | 11 |
| NT2RP7008167 | 0.184617537 | 32039 | 46 |
| NT2RP7008190 | 0.126314021 | 69191 | 21 |
| NT2RP7008315 | 0.022327348 | 127190 | 6 |
| NT2RP7008360 | 0.010128046 | 42161 | 3 |
| NT2RP7008396 | 0.013611515 | 93862 | 5 |
| NT2RP7008406 | 0.004038283 | 93781 | 1 |
| NT2RP7008435 | 0.004038283 | 189554 | 1 |
| NT2RP7008441 | 0.008323023 | 117286 | 3 |
| NT2RP7008454 | 0.004038283 | 239197 | 1 |
| NT2RP7008487 | 0.010950062 | 23020 | 4 |
| NT2RP7008543 | 0.060904559 | 73412 | 18 |
| NT2RP7008544 | 0.004038283 | 271803 | 1 |
| NT2RP7008550 | 0.01515973 | 71600 | 4 |
| NT2RP7008557 | 0.004038283 | 108227 | 1 |
| NT2RP7008623 | 0.038930118 | 89603 | 2 |
| NT2RP7008657 | 0.004038283 | 233387 | 1 |
| NT2RP7008714 | 0.043205302 | 36975 | 17 |
| NT2RP7008720 | 0.007083164 | 175173 | 2 |
| NT2RP7008855 | 0.004038283 | 89344 | 1 |
| NT2RP7008863 | 0.004038283 | 90649 | 1 |
| NT2RP7009012 | 0.014778223 | 40000 | 6 |
| NT2RP7009019 | 0.004038283 | 19155 | 1 |
| NT2RP7009030 | 0.017416598 | 131055 | 6 |
| NT2RP7009087 | 0.068972966 | 111939 | 23 |
| NT2RP7009097 | 0.093197918 | 129012 | 11 |
| NT2RP7009108 | 0.188525251 | 24105 | 47 |
| NT2RP7009147 | 0.105182831 | 64980 | 28 |
| NT2RP7009149 | 0.14006429 | 87758 | 24 |
| NT2RP7009168 | 0.006119795 | 131468 | 2 |
| NT2RP7009215 | 0.004038283 | 235426 | 1 |
| NT2RP7009225 | 0.059569243 | 129171 | 16 |
| NT2RP7009236 | 0.004038283 | 238266 | 1 |
| NT2RP7009253 | 0.004038283 | 146725 | 1 |
| NT2RP7009259 | 0.298230805 | 74339 | 101 |
| NT2RP7009267 | 0.004038283 | 220500 | 1 |
| NT2RP7009322 | 0.015502229 | 182783 | 2 |
| NT2RP7009363 | 0.004038283 | 210048 | 1 |
| NT2RP7009370 | 0.005133561 | 136611 | 2 |
| NT2RP7009373 | 0.041709609 | 76469 | 18 |
| NT2RP7009394 | 0.138568949 | 82993 | 46 |
| NT2RP7009397 | 0.004038283 | 195193 | 1 |
| NT2RP7009429 | 0.004038283 | 178085 | 1 |
| NT2RP7009439 | 0.012114849 | 150342 | 3 |
| NT2RP7009466 | 0.005435438 | 111733 | 2 |
| NT2RP7009481 | 0.040912856 | 120940 | 16 |
| NT2RP7009482 | 0.361548428 | 88362 | 93 |
| NT2RP7009498 | 0.166273189 | 144037 | 10 |
| NT2RP7009502 | 0.004038283 | 206966 | 1 |
| NT2RP7009507 | 0.00571383 | 203782 | 2 |
| NT2RP7009577 | 0.007133779 | 7340 | 2 |
| NT2RP7009743 | 0.004038283 | 102207 | 1 |
| NT2RP7009867 | 0.004038283 | 135882 | 1 |
| NT2RP7010109 | 0.010662966 | 143053 | 4 |
| NT2RP7010110 | 0.072708957 | 107156 | 11 |
| NT2RP7010128 | 0.004038283 | 175182 | 1 |
| NT2RP7010235 | 0.019198013 | 179581 | 5 |
| NT2RP7010275 | 0.004038283 | 74820 | 1 |
| NT2RP7010521 | 0.004038283 | 134569 | 1 |
| NT2RP7010599 | 0.005227527 | 178225 | 2 |
| NT2RP7010612 | 0.007746144 | 141449 | 3 |
| NT2RP7010817 | 0.004038283 | 78314 | 1 |
| NT2RP7011086 | 0.004038283 | 201855 | 1 |
| NT2RP7011132 | 0.004038283 | 232449 | 1 |
| NT2RP7011268 | 0.004038283 | 202775 | 1 |
| NT2RP7011318 | 0.007133779 | 145973 | 2 |
| NT2RP7011452 | 0.004038283 | 208857 | 1 |
| NT2RP7011570 | 0.004038283 | 190769 | 1 |
| NT2RP7011615 | 0.028439911 | 103544 | 5 |
| NT2RP7011721 | 0.004038283 | 90109 | 1 |
| NT2RP7011909 | 0.59115051 | 161342 | 3 |
| NT2RP7012291. | 0.004038283 | 184850 | 1 |
| NT2RP7012516 | 0.004038283 | 205767 | 1 |
| NT2RP7012776 | 0.019935962 | 115000 | 4 |
| NT2RP7013002 | 0.007083164 | 157326 | 2 |
| NT2RP7013374 | 0.004038283 | 11400 | 1 |
| NT2RP7013499 | 0.004038283 | 219720 | 1 |
| NT2RP7013573 | 0.023252827 | 117327 | 9 |
| NT2RP7013729 | 0.004038283 | 205226 | 1 |
| NT2RP7013764 | 0.014758364 | 181221 | 5 |
| NT2RP7013795 | 0.038712347 | 173210 | 2 |
| NT2RP7013999 | 0.004038283 | 158185 | 1 |
| NT2RP7014005 | 0.028800602 | 92920 | 8 |
| NT2RP7014178 | 0.004038283 | 189229 | 1 |
| NT2RP7014348 | 0.004038283 | 179928 | 1 |
| NT2RP7014420 | 0.004038283 | 265682 | 1 |
| NT2RP7014583 | 0.010240603 | 183428 | 2 |
| NT2RP7014721 | 0.004038283 | 175200 | 1 |
| NT2RP7014743 | 0.004038283 | 2217 | 1 |
| NT2RP7014778 | 0.004038283 | 174196 | 1 |
| NT2RP7014906 | 0.004038283 | 218451 | 1 |
| NT2RP7014910 | 0.140138393 | 92250 | 33 |
| NT2RP7015080 | 0.004038283 | 200646 | 1 |
| NT2RP7015431 | 0.004038283 | 178181 | 1 |
| NT2RP7015503 | 0.052914656 | 184774 | 5 |
| NT2RP7015512 | 0.025923118 | 135904 | 7 |
| NT2RP7015676 | 0.004038283 | 223128 | 1 |
| NT2RP7015789 | 0.004038283 | 97395 | 1 |
| NT2RP7015996 | 0.004038283 | 117328 | 1 |
| NT2RP7016508 | 0.004038283 | 35406 | 1 |
| NT2RP7016574 | 0.007584674 | 76256 | 3 |
| NT2RP7016622 | 0.004038283 | 104981 | 1 |
| NT2RP7016911 | 0.004038283 | 117384 | 1 |
| NT2RP7017139 | 0.004038283 | 164222 | 1 |
| NT2RP7017284 | 0.004038283 | 101101 | 1 |
| NT2RP7017365 | 0.004038283 | 202699 | 1 |
| NT2RP7017474 | 0.008901476 | 137193 | 4 |
| NT2RP7017546 | 0.008076566 | 283109 | 2 |
| NT2RP7017567 | 0.004038283 | 164154 | 1 |
| NT2RP7017795 | 0.004038283 | 148488 | 1 |
| NT2RP7017971 | 0.004038283 | 132306 | 1 |
| NT2RP7018032 | 0.004038283 | 205 | 1 |
| NT2RP7018126 | 0.009950545 | 194781 | 2 |
| NT2RP7018197 | 0.004038283 | 183502 | 1 |
| NT2RP7018206 | 0.004038283 | 276028 | 1 |
| NT2RP7018340 | 0.004038283 | 202005 | 1 |
| NT2RP7018586 | 0.004038283 | 229410 | 1 |
| NT2RP7018802 | 0.008076566 | 195035 | 2 |
| NT2RP7018871 | 0.004038283 | 189361 | 1 |
| NT2RP7019064 | 0.013047083 | 196960 | 4 |
| NT2RP7019135 | 0.00571383 | 118773 | 2 |
| NT2RP7019273 | 0.004038283 | 1216 | 1 |
| NT2RP7019367 | 0.004038283 | 263250 | 1 |
| NT2RP7019401 | 0.016137788 | 33936 | 4 |
| NT2RP7019445 | 0.004038283 | 75302 | 1 |
| NT2RP7019543 | 0.015840394 | 146229 | 4 |
| NT2RP7019682 | 0.004038283 | 54346 | 1 |
| NT2RP7019835 | 0.160786693 | 58635 | 4 |
| NT2RP7020112 | 0.009879746 | 207374 | 2 |
| NT2RP7020123 | 0.039398962 | 144471 | 2 |
| NT2RP7020343 | 0.004038283 | 277655 | 1 |
| NT2RP7020379 | 0.004038283 | 246576 | 1 |
| NT2RP8000137 | 0.004038283 | 192623 | 1 |
| NT2RP8000296 | 0.004038283 | 77947 | 1 |
| NT2RP8000435 | 0.012557557 | 156683 | 5 |
| NT2RP8000483 | 0.004038283 | 281854 | 1 |
| NT2RP8000521 | 0.006416772 | 199019 | 3 |
| NT2RP8000633 | 0.004038283 | 275280 | 1 |
| NT2RP8001363 | 0.004038283 | 250513 | 1 |
| NT2RP8001407 | 0.004038283 | 257737 | 1 |
| NT2RP8001584 | 0.004038283 | 281882 | 1 |
| NT2RP8001604 | 0.024148442 | 181892 | 6 |
| NT2RP8001605 | 0.00571383 | 171246 | 2 |
| NT2RP8003490 | 0.004038283 | 222150 | 1 |
| NT2RP8003657 | 0.004038283 | 259812 | 1 |
| NT2RP8003787 | 0.004038283 | 186112 | 1 |
| NT2RP8004306 | 0.004038283 | 21976 | 1 |
| NT2RP8005546 | 0.006832594 | 114418 | 3 |
| NT2RP8006452 | 0.004038283 | 226920 | 1 |
| NT2RP8006521 | 0.004038283 | 276216 | 1 |
| NT2RP8007416 | 0.004038283 | 269019 | 1 |
| NT2RP8007503 | 0.004038283 | 260794 | 1 |
| NT2RP8007715 | 0.013335599 | 30421 | 8 |
| NT2RP8007920 | 0.08470369 | 156290 | 27 |
| NT2RP8008057 | 0.005915125 | 119986 | 2 |
| NT2RP8009119 | 0.036305904 | 102896 | 28 |
| NT2RP8009248 | 0.004038283 | 101043 | 1 |
| NTONG1000006 | 0.012348728 | 53037 | 1 |
| NTONG1000033 | 0.136758931 | 54910 | 53 |
| NTONG1000047 | 0.02788718 | 63430 | 4 |
| NTONG1000052 | 0.350823542 | 94424 | 31 |
| NTONG1000064 | 0.387403789 | 34429 | 51 |
| NTONG1000075 | 0.025148392 | 55312 | 3 |
| NTONG1000087 | 0.012348728 | 74103 | 1 |
| NTONG1000098 | 0.262993716 | 23511 | 95 |
| NTONG1000123 | 0.035984138 | 13134 | 3 |
| NTONG1000130 | 0.01422557 | 65535 | 2 |
| NTONG1000161 | 0.012348728 | 30236 | 1 |
| NTONG1000172 | 0.012348728 | 65517 | 1 |
| NTONG1000182 | 0.135343266 | 65504 | 23 |
| NTONG1000213 | 0.012348728 | 35234 | 1 |
| NTONG1000214 | 0.045275933 | 35396 | 7 |
| NTONG1000246 | 0.107159275 | 79132 | 20 |
| NTONG1000247 | 0.012348728 | 112253 | 1 |
| NTONG1000257 | 0.152254757 | 57465 | 19 |
| NTONG1000264 | 0.171910462 | 52878 | 57 |
| NTONG2000040 | 0.012348728 | 193180 | 1 |
| NTONG2000107 | 0.015712552 | 79459 | 3 |
| NTONG2000223 | 0.082907015 | 121274 | 15 |
| NTONG2000231 | 0.012348728 | 264438 | 1 |
| NTONG2000265 | 0.196548465 | 93345 | 75 |
| NTONG2000334 | 0.08195273 | 21695 | 11 |
| NTONG2000363 | 0.012348728 | 146685 | 1 |
| NTONG2000413 | 0.015525518 | 196061 | 3 |
| NTONG2000492 | 0.024697456 | 174181 | 2 |
| NTONG2000499 | 0.018153906 | 171241 | 2 |
| NTONG2000531 | 0.012348728 | 272314 | 1 |
| NTONG2000583 | 0.012348728 | 278144 | 1 |
| NTONG2000800 | 0.316441064 | 121153 | 19 |
| NTONG2000858 | 0.058015149 | 146317 | 5 |
| NTONG2000876 | 0.120334926 | 134760 | 17 |
| NTONG2000878 | 0.141887343 | 139080 | 14 |
| NTONG2000966 | 0.012348728 | 153836 | 1 |
| NTONG2000977 | 0.170446213 | 113626 | 15 |
| NTONG2000985 | 0.089875138 | 165062 | 8 |
| NTONG2001014 | 0.012348728 | 67982 | 1 |
| NTONG2001079 | 0.01443024 | 118831 | 2 |
| NTONG2001137 | 0.146407897 | 89033 | 13 |
| NTONG2001157 | 0.012348728 | 41655 | 1 |
| NTONG2001173 | 0.012348728 | 222018 | 1 |
| NTONG2001220 | 0.02911805 | 12118 | 3 |
| NTONG2001222 | 0.202062765 | 143035 | 11 |
| NTONG2001362 | 0.047999352 | 230795 | 2 |
| NTONG2001428 | 0.015895119 | 99341 | 3 |
| NTONG2001532 | 0.012348728 | 88521 | 1 |
| NTONG2001550 | 0.048140878 | 101969 | 13 |
| NTONG2001567 | 0.546437024 | 100109 | 48 |
| NTONG2001587 | 0.562851169 | 162441 | 26 |
| NTONG2001612 | 0.149263756 | 65582 | 23 |
| NTONG2001619 | 0.012348728 | 280445 | 1 |
| NTONG2001642 | 0.012348728 | 86736 | 1 |
| NTONG2001657 | 0.025270294 | 100024 | 2 |
| NTONG2001676 | 0.023488518 | 99714 | 4 |
| NTONG2001762 | 0.092918664 | 130311 | 35 |
| NTONG2002278 | 0.012348728 | 158242 | 1 |
| NTONG2002582 | 0.044549777 | 22394 | 14 |
| NTONG2002807 | 0.014206842 | 91287 | 2 |
| NTONG2002948 | 0.075460802 | 181167 | 7 |
| NTONG2002970 | 0.012348728 | 194985 | 1 |
| NTONG2002985 | 0.014024275 | 122207 | 2 |
| NTONG2003158 | 0.012348728 | 57219 | 1 |
| NTONG2003210 | 0.012348728 | 199293 | 1 |
| NTONG2003316 | 0.012348728 | 102292 | 1 |
| NTONG2003409 | 0.012348728 | 53319 | 1 |
| NTONG2003454 | 0.056203927 | 98363 | 22 |
| NTONG2003515 | 0.017121699 | 187810 | 3 |
| NTONG2003805 | 0.012348728 | 133262 | 1 |
| NTONG2003839 | 0.063906949 | 67108 | 10 |
| NTONG2003852 | 0.024943187 | 117128 | 2 |
| NTONG2003897 | 0.012348728 | 60491 | 1 |
| NTONG2003928 | 0.028763503 | 197631 | 5 |
| NTONG2004095 | 0.018249654 | 218809 | 5 |
| NTONG2004308 | 0.01422557 | 147480 | 2 |
| NTONG2004521 | 0.012348728 | 78495 | 1 |
| NTONG2004614 | 0.028294695 | 17896 | 5 |
| NTONG2004690 | 0.012348728 | 11320 | 1 |
| NTONG2004806 | 0.024155104 | 216592 | 2 |
| NTONG2004829 | 0.012348728 | 183710 | 1 |
| NTONG2004844 | 0.103390513 | 66619 | 11 |
| NTONG2004918 | 0.025874448 | 121520 | 5 |
| NTONG2004991 | 0.012348728 | 175391 | 1 |
| NTONG2005062 | 0.012348728 | 184823 | 1 |
| NTONG2005086 | 0.012348728 | 90027 | 1 |
| NTONG2005137 | 0.012348728 | 9910 | 1 |
| NTONG2005153 | 0.021176197 | 162608 | 3 |
| NTONG2005265 | 0.012348728 | 198322 | 1 |
| NTONG2005277 | 0.012348728 | 140262 | 1 |
| NTONG2005363 | 0.014347129 | 161085 | 2 |
| NTONG2005373 | 0.028432412 | 39609 | 4 |
| NTONG2005391 | 0.071874571 | 129330 | 31 |
| NTONG2005480 | 0.012348728 | 207976 | 1 |
| NTONG2005497 | 0.611408917 | 134823 | 4 |
| NTONG2005520 | 0.251713915 | 33660 | 54 |
| NTONG2005577 | 0.012348728 | 183800 | 1 |
| NTONG2005657 | 0.024697456 | 152462 | 2 |
| NTONG2005801 | 0.094050386 | 84743 | 31 |
| NTONG2005822 | 0.01826134 | 140970 | 2 |
| NTONG2005897 | 0.024410459 | 116472 | 5 |
| NTONG2005969 | 0.052160241 | 126188 | 13 |
| NTONG2006099 | 0.012348728 | 74368 | 1 |
| NTONG2006187 | 0.012348728 | 121156 | 1 |
| NTONG2006301 | 0.030486948 | 147488 | 3 |
| NTONG2006324 | 0.012348728 | 140332 | 1 |
| NTONG2006354 | 0.012348728 | 158120 | 1 |
| NTONG2006440 | 0.012348728 | 222450 | 1 |
| NTONG2006484 | 0.038763008 | 97254 | 6 |
| NTONG2006501 | 0.012348728 | 147494 | 1 |
| NTONG2006646 | 0.012348728 | 147476 | 1 |
| NTONG2006709 | 0.018489106 | 89616 | 3 |
| NTONG2006783 | 0.059183732 | 116666 | 6 |
| NTONG2007020 | 0.012348728 | 111926 | 1 |
| NTONG2007028 | 0.014024275 | 179445 | 2 |
| NTONG2007034 | 0.02363541 | 131415 | 2 |
| NTONG2007052 | 0.039364238 | 79287 | 7 |
| NTONG2007249 | 0.012348728 | 113152 | 1 |
| NTONG2007517 | 0.012348728 | 221997 | 1 |
| NTONG2007522 | 0.012348728 | 87900 | 1 |
| NTONG2007658 | 0.083728568 | 66678 | 10 |
| NTONG2007693 | 0.023812674 | 121145 | 2 |
| NTONG2007756 | 0.024824654 | 155771 | 3 |
| NTONG2008088 | 0.016397399 | 157349 | 4 |
| NTONG2008093 | 0.012348728 | 36460 | 1 |
| NTONG2008143 | 0.015421431 | 123769 | 3 |
| NTONG2008365 | 0.012348728 | 191045 | 1 |
| NTONG2008483 | 0.209724219 | 119804 | 44 |
| NTONG2008522 | 0.042756755 | 127388 | 5 |
| NTONG2008672 | 0.012348728 | 164712 | 1 |
| NTONG2008862 | 0.012348728 | 166531 | 1 |
| NTONG2008939 | 0.012348728 | 233372 | 1 |
| NTONG2008944 | 0.057384338 | 114730 | 22 |
| NTONG2009060 | 0.06013176 | 134574 | 3 |
| NTONG2009068 | 0.012348728 | 124960 | 1 |
| NTONG2009229 | 0.012348728 | 41389 | 1 |
| NTONG2009233 | 0.012348728 | 166519 | 1 |
| NTONG2009468 | 0.017642074 | 100811 | 4 |
| NTONG2009576 | 0.023581774 | 149978 | 7 |
| OCBBF1000016 | 0.002081512 | 36356 | 1 |
| OCBBF1000030 | 0.003790972 | 76364 | 2 |
| OCBBF1000042 | 0.002081512 | 68171 | 1 |
| OCBBF1000049 | 0.03371824 | 35934 | 15 |
| OCBBF1000054 | 0.002081512 | 108231 | 1 |
| OCBBF1000067 | 0.045837097 | 22079 | 14 |
| OCBBF1000080 | 0.00876331 | 38621 | 4 |
| OCBBF1000085 | 0.004163024 | 60213 | 2 |
| OCBBF1000086 | 0.218899346 | 73638 | 26 |
| OCBBF1000091 | 0.004163024 | 34976 | 2 |
| OCBBF1000104 | 0.016269141 | 78020 | 6 |
| OCBBF1000114 | 0.065727455 | 37092 | 10 |
| OCBBF1000118 | 0.002081512 | 77875 | 1 |
| OCBBF1000119 | 0.002081512 | 106812 | 1 |
| OCBBF1000122 | 0.004163024 | 94797 | 2 |
| OCBBF1000130 | 0.002081512 | 6405 | 1 |
| OCBBF1000138 | 0.002081512 | 106388 | 1 |
| OCBBF1000140 | 0.067835682 | 50807 | 21 |
| OCBBF1000145 | 0.003939626 | 69439 | 2 |
| OCBBF1000146 | 0.194247437 | 66341 | 59 |
| OCBBF1000153 | 0.019747592 | 37782 | 9 |
| OCBBF1000175 | 0.023262008 | 75110 | 7 |
| OCBBF1000185 | 0.03980012 | 45258 | 15 |
| OCBBF1000188 | 0.160904706 | 71218 | 26 |
| OCBBF1000192 | 0.002081512 | 78433 | 1 |
| OCBBF1000254 | 0.003769789 | 181531 | 2 |
| OCBBF1000315 | 0.019150674 | 86515 | 11 |
| OCBBF2000013 | 0.086109145 | 138184 | 22 |
| OCBBF2000015 | 0.211786145 | 55887 | 86 |
| OCBBF2000074 | 0.036401716 | 14609 | 14 |
| OCBBF2000126 | 0.043109901 | 134567 | 11 |
| OCBBF2000178 | 0.153683174 | 66291 | 58 |
| OCBBF2000231 | 0.04277493 | 123966 | 17 |
| OCBBF2000277 | 0.022806825 | 83294 | 11 |
| OCBBF2000287 | 0.007994124 | 225450 | 2 |
| OCBBF2000323 | 0.01104222 | 54174 | 4 |
| OCBBF2000452 | 0.002081512 | 222761 | 1 |
| OCBBF2000467 | 0.083073577 | 28185 | 22 |
| OCBBF2000522 | 0.066263823 | 42101 | 14 |
| OCBBF2000523 | 0.01785392 | 70182 | 6 |
| OCBBF2000677 | 0.010286152 | 118233 | 5 |
| OCBBF2000703 | 0.002081512 | 186946 | 1 |
| OCBBF2000712 | 0.1773015 | 2458 | 8 |
| OCBBF2000719 | 0.004163024 | 33520 | 2 |
| OCBBF2000824 | 0.02788448 | 85154 | 7 |
| OCBBF2000831 | 0.034801238 | 102289 | 18 |
| OCBBF2000846 | 0.228965121 | 103503 | 25 |
| OCBBF2000904 | 0.041454731 | 144127 | 11 |
| OCBBF2000982 | 0.192139698 | 121587 | 28 |
| OCBBF2000986 | 0.173870224 | 86050 | 32 |
| OCBBF2000998 | 0.002081512 | 34375 | 1 |
| OCBBF2001075 | 0.002081512 | 125228 | 1 |
| OCBBF2001101 | 0.002081512 | 126673 | 1 |
| OCBBF2001124 | 0.002081512 | 52260 | 1 |
| OCBBF2001140 | 0.029635973 | 82555 | 18 |
| OCBBF2001166 | 0.0305713 | 82890 | 17 |
| OCBBF2001176 | 0.002081512 | 83976 | 1 |
| OCBBF2001186 | 0.009465192 | 124096 | 4 |
| OCBBF2001196 | 0.010580523 | 130168 | 4 |
| OCBBF2001210 | 0.046211943 | 50387 | 24 |
| OCBBF2001252 | 0.036401716 | 14609 | 14 |
| OCBBF2001307 | 0.100739283 | 106222 | 50 |
| OCBBF2001323 | 0.019486196 | 131695 | 7 |
| OCBBF2001389 | 0.002081512 | 282111 | 1 |
| OCBBF2001402 | 0.005177008 | 109335 | 2 |
| OCBBF2001408 | 0.008677223 | 56975 | 5 |
| OCBBF2001416 | 0.008029128 | 48643 | 5 |
| OCBBF2001473 | 0.002081512 | 84922 | 1 |
| OCBBF2001492 | 0.244207829 | 34142 | 65 |
| OCBBF2001494 | 0.007922975 | 24094 | 2 |
| OCBBF2001527 | 0.002081512 | 278969 | 1 |
| OCBBF2001528 | 0.005053632 | 100761 | 3 |
| OCBBF2001586 | 0.046730494 | 146803 | 12 |
| OCBBF2001639 | 0.035631759 | 109810 | 11 |
| OCBBF2001681 | 0.002081512 | 16642 | 1 |
| O*CBBF2001687 | 0.087108275 | 6874 | 22 |
| OCBBF2001706 | 0.003478667 | 235606 | 2 |
| OCBBF2001749 | 0.005177008 | 157261 | 2 |
| OCBBF2001794 | 0.035441514 | 32723 | 12 |
| OCBBF2001910 | 0.004163024 | 117932 | 2 |
| OCBBF2001931 | 0.010006147 | 133821 | 3 |
| OCBBF2001961 | 0.004079913 | 60998 | 2 |
| OCBBF2001983 | 0.023193627 | 60773 | 7 |
| OCBBF2002015 | 0.012114648 | 150985 | 8 |
| OCBBF2002083 | 0.015247912 | 82893 | 5 |
| OCBBF2002086 | 0.052007922 | 40767 | 22 |
| OCBBF2002124 | 0.002081512 | 183935 | 1 |
| OCBBF2002161 | 0.002081512 | 131222 | 1 |
| OCBBF2002290 | 0.016613995 | 120332 | 6 |
| OCBBF2002357 | 0.002081512 | 191004 | 1 |
| OCBBF2002376 | 0.022008448 | 147455 | 5 |
| OCBBF2002615 | 0.002081512 | 140519 | 1 |
| OCBBF2002626 | 0.198413943 | 106947 | 21 |
| OCBBF2002656 | 0.101296218 | 1664 | 24 |
| OCBBF2002663 | 0.005177008 | 148373 | 2 |
| OCBBF2002805 | 0.002081512 | 44235 | 1 |
| OCBBF2002865 | 0.00317679 | 74233 | 2 |
| OCBBF2002920 | 0.002081512 | 158048 | 1 |
| OCBBF2002980 | 0.003790972 | 146805 | 2 |
| OCBBF2003028 | 0.002081512 | 49688 | 1 |
| OCBBF2003052 | 0.044113724 | 132884 | 19 |
| OCBBF2003091 | 0.002081512 | 22155 | 1 |
| OCBBF2003246 | 0.017121104 | 114831 | 8 |
| OCBBF2003327 | 0.002081512 | 123760 | 1 |
| OCBBF2003518 | 0.162544289 | 118129 | 10 |
| OCBBF2003543 | 0.003478667 | 21586 | 2 |
| OCBBF2003593 | 0.003757059 | 48347 | 2 |
| OCBBF2003744 | 0.003958354 | 133138 | 2 |
| OCBBF2003819 | 0.007952337 | 82076 | 5 |
| OCBBF2003925 | 0.201100195 | 140007 | 14 |
| OCBBF2004038 | 0.006258447 | 48766 | 2 |
| OCBBF2004104 | 0.122339577 | 104010 | 39 |
| OCBBF2004147 | 0.002081512 | 200508 | 1 |
| OCBBF2004168 | 0.002081512 | 207583 | 1 |
| OCBBF2004235 | 0.014048492 | 195820 | 3 |
| OCBBF2004259 | 0.003939626 | 86978 | 2 |
| OCBBF2004273 | 0.186932187 | 130331 | 27 |
| OCBBF2004385 | 0.002081512 | 138045 | 1 |
| OCBBF2004418 | 0.01179025 | 169228 | 2 |
| OCBBF2004478 | 0.002081512 | 80025 | 1 |
| OCBBF2004482 | 0.002081512 | 53096 | 1 |
| OCBBF2004533 | 0.00831787 | 137466 | 2 |
| OCBBF2004567 | 0.002081512 | 67515 | 1 |
| OCBBF2004612 | 0.002081512 | 226334 | 1 |
| OCBBF2004647 | 0.002081512 | 118191 | 1 |
| OCBBF2004669 | 0.003790972 | 92758 | 2 |
| OCBBF2004757 | 0.067608748 | 117850 | 22 |
| OCBBF2004826 | 0.002081512 | 105278 | 1 |
| OCBBF2004866 | 0.002081512 | 180466 | 1 |
| OCBBF2004883 | 0.002081512 | 150946 | 1 |
| OCBBF2004889 | 0.003757059 | 146777 | 2 |
| OCBBF2004930 | 0.016144097 | 89689 | 4 |
| OCBBF2004984 | 0.007035122 | 87327 | 3 |
| OCBBF2005066 | 0.004163024 | 80840 | 2 |
| OCBBF2005077 | 0.002081512 | 134671 | 1 |
| OCBBF2005161 | 0.003790972 | 94696 | 2 |
| OCBBF2005189 | 0.23397385 | 86681 | 40 |
| OCBBF2005343 | 0.002081512 | 165812 | 1 |
| OCBBF2005349 | 0.002081512 | 42064 | 1 |
| OCBBF2005373 | 0.002081512 | 96263 | 1 |
| OCBBF2005420 | 0.007920083 | 141936 | 4 |
| OCBBF2005428 | 0.050827112 | 184980 | 4 |
| OCBBF2005433 | 0.002081512 | 109541 | 1 |
| OCBBF2005476 | 0.035841242 | 123024 | 9 |
| OCBBF2005546 | 0.003790972 | 265997 | 2 |
| OCBBF2005843 | 0.002081512 | 158114 | 1 |
| OCBBF2005901 | 0.002081512 | 129438 | 1 |
| OCBBF2005956 | 0.059023062 | 71573 | 19 |
| OCBBF2006005 | 0.035146236 | 86625 | 14 |
| OCBBF2006030 | 0.002081512 | 169451 | 1 |
| OCBBF2006058 | 0.002081512 | 30929 | 1 |
| OCBBF2006113 | 0.006366253 | 195137 | 3 |
| OCBBF2006148 | 0.024741948 | 96646 | 6 |
| OCBBF2006151 | 0.029063487 | 15823 | 8 |
| OCBBF2006154 | 0.002081512 | 198178 | 1 |
| OCBBF2006172 | 0.031956714 | 132460 | 8 |
| OCBBF2006214 | 0.002081512 | 181767 | 1 |
| OCBBF2006241 | 0.002081512 | 183886 | 1 |
| OCBBF2006313 | 0.029344902 | 152182 | 8 |
| OCBBF2006332 | 0.449223397 | 100968 | 136 |
| OCBBF2006388 | 0.002081512 | 164800 | 1 |
| OCBBF2006438 | 0.002081512 | 157278 | 1 |
| OCBBF2006567 | 0.002081512 | 150803 | 1 |
| OCBBF2006624 | 0.002081512 | 136500 | 1 |
| OCBBF2006639 | 0.004163024 | 45282 | 2 |
| OCBBF2006660 | 0.011100739 | 124091 | 4 |
| OCBBF2006764 | 0.010919321 | 21352 | 6 |
| OCBBF2006849 | 0.082907015 | 121274 | 15 |
| OCBBF2006859 | 0.02236498 | 181783 | 4 |
| OCBBF2006972 | 0.003757059 | 97909 | 2 |
| OCBBF2006987 | 0.012962816 | 157075 | 5 |
| OCBBF2007028 | 0.121351916 | 42184 | 62 |
| OCBBF2007039 | 0.002081512 | 284822 | 1 |
| OCBBF2007051 | 0.003790972 | 148068 | 2 |
| OCBBF2007068 | 0.003790972 | 210094 | 2 |
| OCBBF2007114 | 0.015675457 | 144794 | 6 |
| OCBBF2007121 | 0.003958354 | 131948 | 2 |
| OCBBF2007184 | 0.002081512 | 126574 | 1 |
| OCBBF2007194 | 0.002081512 | 117714 | 1 |
| OCBBF2007196 | 0.009340032 | 174052 | 4 |
| OCBBF2007224 | 0.017745425 | 101669 | 7 |
| OCBBF2007232 | 0.098693552 | 123744 | 43 |
| OCBBF2007238 | 0.002081512 | 83372 | 1 |
| OCBBF2007354 | 0.166783836 | 94307 | 25 |
| OCBBF2007414 | 0.003478667 | 123594 | 2 |
| OCBBF2007415 | 0.20924961 | 34419 | 63 |
| OCBBF2007428 | 0.002081512 | 206949 | 1 |
| OCBBF2007478 | 0.002081512 | 176475 | 1 |
| OCBBF2007485 | 0.002081512 | 164585 | 1 |
| OCBBF2007520 | 0.002081512 | 207064 | 1 |
| OCBBF2007555 | 0.004163024 | 238792 | 2 |
| OCBBF2007610 | 0.002081512 | 169189 | 1 |
| OCBBF2007622 | 0.045639803 | 92541 | 4 |
| OCBBF2007756 | 0.002081512 | 46166 | 1 |
| OCBBF2007762 | 0.010656257 | 148430 | 5 |
| OCBBF2007829 | 0.305167583 | 87150 | 99 |
| OCBBF2007892 | 0.039332193 | 108414 | 10 |
| OCBBF2007931 | 0.004163024 | 104496 | 2 |
| OCBBF2007946 | 0.002081512 | 66275 | 1 |
| OCBBF2008005 | 0.105527079 | 97111 | 35 |
| OCBBF2008041 | 0.080162019 | 111911 | 13 |
| OCBBF2008116 | 0.002081512 | 93596 | 1 |
| OCBBF2008138 | 0.002081512 | 130170 | 1 |
| OCBBF2008144 | 0.058101046 | 111940 | 13 |
| OCBBF2008283 | 0.002081512 | 436 | 1 |
| OCBBF2008330 | 0.002081512 | 228201 | 1 |
| OCBBF2008340 | 0.021705749 | 91002 | 10 |
| OCBBF2008366 | 0.013844834 | 189685 | 2 |
| OCBBF2008466 | 0.019586597 | 82533 | 5 |
| OCBBF2008483 | 0.019247786 | 228136 | 3 |
| OCBBF2008511 | 0.015176425 | 181663 | 6 |
| OCBBF2008520 | 0.002081512 | 147205 | 1 |
| OCBBF2008569 | 0.007993774 | 251999 | 2 |
| OCBBF2008586 | 0.002081512 | 223609 | 1 |
| OCBBF2008640 | 0.004163024 | 209824 | 2 |
| OCBBF2008691 | 0.002081512 | 101423 | 1 |
| OCBBF2008701 | 0.015460842 | 140231 | 7 |
| OCBBF2008724 | 0.002081512 | 181842 | 1 |
| OCBBF2008760 | 0.010075286 | 123810 | 3 |
| OCBBF2008768 | 0.002081512 | 220261 | 1 |
| OCBBF2008770 | 0.012530733 | 109191 | 5 |
| OCBBF2008775 | 0.003769789 | 155067 | 2 |
| OCBBF2008790 | 0.002081512 | 215323 | 1 |
| OCBBF2008814 | 0.004163024 | 174214 | 2 |
| OCBBF2008822 | 0.070290265 | 109405 | 3 |
| OCBBF2009095 | 0.025494519 | 135510 | 8 |
| OCBBF2009115 | 0.002081512 | 139247 | 1 |
| OCBBF2009242 | 0.059175937 | 54064 | 25 |
| OCBBF2009301 | 0.080831708 | 11165 | 18 |
| OCBBF2009352 | 0.029766781 | 7697 | 12 |
| OCBBF2009391 | 0.005177008 | 176458 | 2 |
| OCBBF2009424 | 0.023088978 | 106847 | 7 |
| OCBBF2009536 | 0.003939626 | 197400 | 2 |
| OCBBF2009571 | 0.002081512 | 27613 | 1 |
| OCBBF2009583 | 0.002081512 | 155953 | 1 |
| OCBBF2009603 | 0.005063471 | 6032 | 2 |
| OCBBF2009772 | 0.142290854 | 110977 | 54 |
| OCBBF2009788 | 0.002081512 | 191020 | 1 |
| OCBBF2009836 | 0.040891613 | 121038 | 17 |
| OCBBF2009920 | 0.003790972 | 191017 | 2 |
| OCBBF2009926 | 0.004163024 | 183545 | 2 |
| OCBBF2010040 | 0.002081512 | 150853 | 1 |
| OCBBF2010140 | 0.005154214 | 93818 | 3 |
| OCBBF2010281 | 0.003958354 | 170482 | 2 |
| OCBBF2010313 | 0.012215794 | 183591 | 4 |
| OCBBF2010404 | 0.002081512 | 160058 | 1 |
| OCBBF2010416 | 0.002081512 | 172400 | 1 |
| OCBBF2010420 | 0. 004163024 | 96182 | 2 |
| OCBBF2010557 | 0.009144401 | 129884 | 5 |
| OCBBF2010604 | 0.002081512 | 102705 | 1 |
| OCBBF2010709 . | 0.002081512 | 282266 | 1 |
| OCBBF2010792 | 0.002081512 | 140692 | 1 |
| OCBBF2010819 | 0.013357928 | 67332 | 6 |
| OCBBF2010830 | 0.190423858 | 4893 | 30 |
| OCBBF2010841 | 0.026956667 | 75402 | 11 |
| OCBBF2010843 | 0.011249391 | 98744 | 3 |
| OCBBF2010858 | 0.025153856 | 86788 | 12 |
| OCBBF2010863 | 0.003769789 | 179423 | 2 |
| OCBBF2010871 | 0.036657763 | 20959 | 18 |
| OCBBF2010931 | 0.002081512 | 130987 | 1 |
| OCBBF2010945 | 0.004079913 | 117005 | 2 |
| OCBBF2010978 | 0.002081512 | 156073 | 1 |
| OCBBF2010989 | 0.002081512 | 157266 | 1 |
| OCBBF2011021 | 0.115923486 | 95125 | 60 |
| OCBBF2011073 | 0.049104411 | 110021 | 25 |
| OCBBF2011137 | 0.002081512 | 66313 | 1 |
| OCBBF2011160 | 0.009598522 | 104681 | 3 |
| OCBBF2011177 | 0.002081512 | 188019 | 1 |
| OCBBF2011228 | 0.007836972 | 86215 | 4 |
| OCBBF2011232 | 0.020912567 | 84828 | 10 |
| OCBBF2011283 | 0.028288408 | 181857 | 14 |
| OCBBF2011311 | 0.002081512 | 176508 | 1 |
| OCBBF2011536 | 0.08399109 | 86213 | 16 |
| OCBBF2011625 | 0.006258447 | 136410 | 2 |
| OCBBF2011669 | 0.003270756 | 181745 | 2 |
| OCBBF2011685 | 0.002081512 | 14778 | 1 |
| OCBBF2011706 | 0.002081512 | 163278 | 1 |
| OCBBF2011722 | 0.008326048 | 140784 | 4 |
| OCBBF2011759 | 0.17929562 | 81197 | 101 |
| OCBBF2011767 | 0.005633901 | 163811 | 3 |
| OCBBF2011855 | 0.002081512 | 157236 | 1 |
| OCBBF2011872 | 0.002081512 | 115593 | 1 |
| OCBBF2011887 | 0.035628456 | 162731 | 13 |
| OCBBF2011897 | 0.009705564 | 196278 | 3 |
| OCBBF2011914 | 0.045952233 | 160238 | 11 |
| OCBBF2011960 | 0.002081512 | 158501 | 1 |
| OCBBF2011981 | 0.002081512 | 157275 | 1 |
| OCBBF2012001 | 0.006729179 | 130426 | 4 |
| OCBBF2012028 | 0.002081512 | 100637 | 1 |
| OCBBF2012039 | 0.109245667 | 86993 | 63 |
| OCBBF2012095 | 0.010949866 | 109152 | 4 |
| OCBBF2012139 | 0.002081512 | 94801 | 1 |
| OCBBF2012191 | 0.005177008 | 39488 | 2 |
| OCBBF2012262 | 0.011552401 | 131464 | 5 |
| OCBBF2012320 | 0.002081512 | 172330 | 1 |
| OCBBF2012436 | 0.003478667 | 102904 | 2 |
| OCBBF2012525 | 0.007433781 | 153756 | 4 |
| OCBBF2012553 | 0.142863832 | 56815 | 26 |
| OCBBF2012678 | 0.024291424 | 140891 | 13 |
| OCBBF2012704 | 0.008025098 | 150129 | 4 |
| OCBBF2012714 | 0.028866765 | 147541 | 5 |
| OCBBF2012755 | 0.002081512 | 152756 | 1 |
| OCBBF2012812 | 0.00813588 | 97614 | 2 |
| OCBBF2012821 | 0.066966268 | 138033 | 10 |
| OCBBF2012881 | 0.003769789 | 199285 | 2 |
| OCBBF2012908 | 0.05113242 | 96930 | 17 |
| OCBBF2012936 | 0.003757059 | 11689 | 2 |
| OCBBF2012990 | 0.002081512 | 159752 | 1 |
| OCBBF2013011 | 0.022191901 | 142051 | 8 |
| OCBBF2013049 | 0.002081512 | 128337 | 1 |
| OCBBF2013091 | 0.002081512 | 104565 | 1 |
| OCBBF2013123 | 0.007343361 | 28827 | 4 |
| OCBBF2013127 | 0.002081512 | 281651 | 1 |
| OCBBF2013149 | 0.108029691 | 87706 | 34 |
| OCBBF2013208 | 0.002081512 | 74373 | 1 |
| OCBBF2013285 | 0.003270756 | 75509 | 2 |
| OCBBF2013319 | 0.002081512 | 103246 | 1 |
| OCBBF2013324 | 0.004460001 | 87179 | 3 |
| OCBBF2013366 | 0.062073628 | 77068 | 9 |
| OCBBF2013481 | 0.030648354 | 61532 | 12 |
| OCBBF2013507 | 0.002081512 | 110930 | 1 |
| OCBBF2013721 | 0.002081512 | 209201 | 1 |
| OCBBF2013789 | 0.011102604 | 163465 | 4 |
| OCBBF2013843 | 0.313578061 | 44329 | 66 |
| OCBBF2013883 | 0.008153895 | 118909 | 2 |
| OCBBF2013926 | 0.013503056 | 181760 | 6 |
| OCBBF2014052 | 0.003958354 | 165252 | 2 |
| OCBBF2014064 | 0.122675426 | 117126 | 20 |
| OCBBF2014089 | 0.008490217 | 32775 | 4 |
| OCBBF2014292 | 0.161779207 | 27574 | 48 |
| OCBBF2014322 | 0.008116561 | 83650 | 3 |
| OCBBF2014386 | 0.009215291 | 139111 | 3 |
| OCBBF2014541 | 0.042734607 | 49300 | 15 |
| OCBBF2014576 | 0.045856326 | 173185 | 6 |
| OCBBF2014707 | 0.002081512 | 11378 | 1 |
| OCBBF2014745 | 0.003270756 | 169866 | 2 |
| OCBBF2014828 | 0.002081512 | 241112 | 1 |
| OCBBF2014873 | 0.011813937 | 114131 | 7 |
| OCBBF2014880 | 0.005177008 | 133168 | 2 |
| OCBBF2014928 | 0.005177008 | 43556 | 2 |
| OCBBF2015031 | 0.002081512 | 134973 | 1 |
| OCBBF2015081 | 0.002081512 | 162891 | 1 |
| OCBBF2015115 | 0.009390551 | 131549 | 4 |
| OCBBF2015232 | 0.002081512 | 153786 | 1 |
| OCBBF2015233 | 0.005352268 | 172085 | 3 |
| OCBBF2015285 | 0.002081512 | 187182 | 1 |
| OCBBF2015334 | 0.00317679 | 102309 | 2 |
| OCBBF2015335 | 0.002081512 | 62254 | 1 |
| OCBBF2015503 | 0.022327869 | 111563 | 8 |
| OCBBF2015506 | 0.006244536 | 128301 | 3 |
| OCBBF2015599 | 0.005177008 | 172068 | 2 |
| OCBBF2015645 | 0.002081512 | 132872 | 1 |
| OCBBF2015659 | 0.002081512 | 116698 | 1 |
| OCBBF2015751 | 0.059125739 | 177150 | 9 |
| OCBBF2015797 | 0.00317679 | 31555 | 2 |
| OCBBF2015931 | 0.00831787 | 187073 | 2 |
| OCBBF2015980 | 0.002081512 | 153022 | 1 |
| OCBBF2016038 | 0.002081512 | 5698 | 1 |
| OCBBF2016405 | 0.021722016 | 146920 | 8 |
| OCBBF2016467 | 0.002081512 | 135030 | 1 |
| OCBBF2016590 | 0.002081512 | 189851 | 1 |
| OCBBF2016591 | 0.002081512 | 91643 | 1 |
| OCBBF2016612 | 0.003958354 | 242051 | 2 |
| OCBBF2016689 | 0.005352268 | 45072 | 3 |
| OCBBF2016690 | 0.002081512 | 130138 | 1 |
| OCBBF2016729 | 0.01838075 | 102420 | 8 |
| OCBBF2016841 | 0.027428655 | 89112 | 4 |
| OCBBF2016928 | 0.061183899 | 166278 | 10 |
| OCBBF2017035 | 0.138211456 | 118822 | 32 |
| OCBBF2017055 | 0.009338148 | 133794 | 5 |
| OCBBF2017069 | 0.002081512 | 282867 | 1 |
| OCBBF2017306 | 0.003757059 | 172093 | 2 |
| OCBBF2017325 | 0.002081512 | 18917 | 1 |
| OCBBF2017398 | 0.130254954 | 84775 | 43 |
| OCBBF2017458 | 0.03009693 | 120303 | 11 |
| OCBBF2017489 | 0.002081512 | 83053 | 1 |
| OCBBF2017516 | 0.002081512 | 163975 | 1 |
| OCBBF2017665 | 0.002081512 | 209622 | 1 |
| OCBBF2017754 | 0.04462691 | 85474 | 22 |
| OCBBF2017791 | 0.130817703 | 70226 | 51 |
| OCBBF2017815 | 0.00317679 | 48226 | 2 |
| OCBBF2017882 | 0.023484027 | 93895 | 8 |
| OCBBF2017888 | 0.009834514 | 101169 | 4 |
| OCBBF2017893 | 0.096598963 | 72639 | 14 |
| OCBBF2017899 | 0.089875138 | 165062 | 8 |
| OCBBF2017920 | 0.013012814 | 80716 | 6 |
| OCBBF2018012 | 0.059697615 | 78102 | 11 |
| OCBBF2018014 | 0.002081512 | 52945 | 1 |
| OCBBF2018084 | 0.037998299 | 133834 | 10 |
| OCBBF2018167 | 0.002081512 | 110333 | 1 |
| OCBBF2018206 | 0.003790972 | 48642 | 2 |
| OCBBF2018229 | 0.002081512 | 149607 | 1 |
| OCBBF2018234 | 0.025868858 | 51553 | 13 |
| OCBBF2018362 | 0.00728383 | 95400 | 3 |
| OCBBF2018380 | 0.002081512 | 176886 | 1 |
| OCBBF2018563 | 0.008147311 | 94684 | 5 |
| OCBBF2018581 | 0.038946716 | 185391 | 10 |
| OCBBF2018618 | 0.010331586 | 144649 | 6 |
| OCBBF2018663 | 0.002081512 | 95263 | 1 |
| OCBBF2018687 | 0.002081512 | 173151 | 1 |
| OCBBF2018707 | 0.074503849 | 44141 | 14 |
| OCBBF2018827. | 0.034148765 | 133435 | 14 |
| OCBBF2018828 | 0.002081512 | 243418 | 1 |
| OCBBF2018873 | 0.120916169 | 74910 | 23 |
| OCBBF2018934 | 0.002081512 | 163061 | 1 |
| OCBBF2018956 | 0.099003332 | 131743 | 16 |
| OCBBF2019108 | 0.004875823 | 116958 | 3 |
| OCBBF2019195 | 0.002081512 | 66329 | 1 |
| OCBBF2019327 | 0.002081512 | 109165 | 1 |
| OCBBF2019684 | 0.002081512 | 148505 | 1 |
| OCBBF2019761 | 0.002081512 | 166324 | 1 |
| OCBBF2019823 | 0.002081512 | 268450 | 1 |
| OCBBF2019950 | 0.003790972 | 6731 | 2 |
| OCBBF2020048 | 0.00317679 | 76141 | 2 |
| OCBBF2020318 | 0.003478667 | 172434 | 2 |
| OCBBF2020343 | 0.003270756 | 84376 | 2 |
| OCBBF2020453 | 0.002081512 | 198562 | 1 |
| OCBBF2020639 | 0.007418294 | 127946 | 4 |
| OCBBF2020741 | 0.011875724 | 102282 | 7 |
| OCBBF2020801 | 0.003478667 | 167537 | 2 |
| OCBBF2020825 | 0.002081512 | 133139 | 1 |
| OCBBF2020838 | 0.002081512 | 280456 | 1 |
| OCBBF2021020 | 0.062844985 | 61109 | 4 |
| OCBBF2021214 | 0.002081512 | 264551 | 1 |
| OCBBF2021286 | 0.018157332 | 117157 | 9 |
| OCBBF2021323 | 0.002081512 | 172759 | 1 |
| OCBBF2021518 | 0.002081512 | 158404 | 1 |
| OCBBF2021788 | 0.002081512 | 282481 | 1 |
| OCBBF2021833 | 0.011553201 | 157205 | 6 |
| OCBBF2022351 | 0.003270756 | 67225 | 2 |
| OCBBF2022573 | 0.002081512 | 178307 | 1 |
| OCBBF2022574 | 0.004163024 | 90390 | 2 |
| OCBBF2023162 | 0.016296206 | 166299 | 6 |
| OCBBF2023545 | 0.02005848 | 135477 | 9 |
| OCBBF2023598 | 0.002081512 | 166295 | 1 |
| OCBBF2023643 | 0.002081512 | 197431 | 1 |
| OCBBF2024284 | 0.018984488 | 193278 | 7 |
| OCBBF2024463 | 0.002081512 | 154001 | 1 |
| OCBBF2024589 | 0.002081512 | 285660 | 1 |
| OCBBF2024719 | 0.005649087 | 144920 | 3 |
| OCBBF2024779 | 0.002081512 | 206643 | 1 |
| OCBBF2024781 | 0.002081512 | 275016 | 1 |
| OCBBF2024850 | 0.075396546 | 137903 | 33 |
| OCBBF2025028 | 0.002081512 | 144418 | 1 |
| OCBBF2025115 | 0.002081512 | 157707 | 1 |
| OCBBF2025198 | 0.002081512 | 143253 | 1 |
| OCBBF2025451 | 0.002081512 | 283335 | 1 |
| OCBBF2025458 | 0.002081512 | 285593 | 1 |
| OCBBF2025527 | 0.003270756 | 8698 | 2 |
| OCBBF2025631 | 0.002081512 | 277587 | 1 |
| OCBBF2025730 | 0.002081512 | 181776 | 1 |
| OCBBF2026025 | 0.002081512 | 278096 | 1 |
| OCBBF2026035 | 0.002081512 | 144199 | 1 |
| OCBBF2026144 | 0.002081512 | 271183 | 1 |
| OCBBF2026368 | 0.020277754 | 135511 | 9 |
| OCBBF2026645 | 0.002081512 | 150997 | 1 |
| OCBBF2026649 | 0.003270756 | 164049 | 2 |
| OCBBF2026690 | 0.006244536 | 153876 | 3 |
| OCBBF2026981 | 0.004163024 | 135734 | 2 |
| OCBBF2027148 | 0.00720072 | 36165 | 3 |
| OCBBF2027197 | 0.003270756 | 158730 | 2 |
| OCBBF2027354 | 0.002081512 | 145847 | 1 |
| OCBBF2027423 | 0.002081512 | 128117 | 1 |
| OCBBF2027478 | 0.002081512 | 156778 | 1 |
| OCBBF2027481 | 0.002081512 | 277571 | 1 |
| OCBBF2027511 | 0.002081512 | 270240 | 1 |
| OCBBF2027661 | 0.004460001 | 147148 | 3 |
| OCBBF2027728 | 0.002081512 | 173308 | 1 |
| OCBBF2027782 | 0.002081512 | 166305 | 1 |
| OCBBF2027868 | 0.002081512 | 159722 | 1 |
| OCBBF2028055 | 0.007514118 | 128278 | 4 |
| OCBBF2028173 | 0.002081512 | 181827 | 1 |
| OCBBF2028421 | 0.003958354 | 110875 | 2 |
| OCBBF2028935 | 0.14472965 | 126539 | 32 |
| OCBBF2029471 | 0.002081512 | 275248 | 1 |
| OCBBF2029901 | 0.002081512 | 88476 | 1 |
| OCBBF2030116 | 0.003790972 | 46886 | 2 |
| OCBBF2030329 | 0.003769789 | 95895 | 2 |
| OCBBF2030354 | 0.002081512 | 144343 | 1 |
| OCBBF2030517 | 0.002081512 | 206438 | 1 |
| OCBBF2030574 | 0.002081512 | 268848 | 1 |
| OCBBF2030708 | 0.002081512 | 246574 | 1 |
| OCBBF2030927 | 0.003769789 | 152312 | 2 |
| OCBBF2030963 | 0.002081512 | 281268 | 1 |
| OCBBF2031167 | 0.002081512 | 164204 | 1 |
| OCBBF2031198 | 0.002081512 | 254832 | 1 |
| OCBBF2031366 | 0.002081512 | 278251 | 1 |
| OCBBF2031561 | 0.005336782 | 87511 | 3 |
| OCBBF2032152 | 0.002081512 | 181495 | 1 |
| OCBBF2032274 | 0.002081512 | 269556 | 1 |
| OCBBF2032539 | 0.002081512 | 218009 | 1 |
| OCBBF2032590 | 0.002081512 | 222070 | 1 |
| OCBBF2032599 | 0.002081512 | 264341 | 1 |
| OCBBF2032611 | 0.002081512 | 276608 | 1 |
| OCBBF2032671 | 0.002081512 | 236779 | 1 |
| OCBBF2032968 | 0.007655602 | 199666 | 3 |
| OCBBF2033295 | 0.03186685 | 75220 | 15 |
| OCBBF2033413 | 0.002081512 | 262312 | 1 |
| OCBBF2033869 | 0.002081512 | 219482 | 1 |
| OCBBF2033948 | 0.002081512 | 246880 | 1 |
| OCBBF2034333 | 0.002081512 | 203604 | 1 |
| OCBBF2034529 | 0.002081512 | 13135 | 1 |
| OCBBF2034637 | 0.002081512 | 157900 | 1 |
| OCBBF2034823 | 0.026992367 | 145607 | 10 |
| OCBBF2034906 | 0.002081512 | 235119 | 1 |
| OCBBF2035110 | 0.002081512 | 194719 | 1 |
| OCBBF2035214 | 0.003769789 | 243761 | 2 |
| OCBBF2035226 | 0.285219087 | 127332 | 13 |
| OCBBF2035390 | 0.002081512 | 108071 | 1 |
| OCBBF2035564 | 0.002081512 | 194785 | 1 |
| OCBBF2035658 | 0.002081512 | 262840 | 1 |
| OCBBF2035823 | 0.004875823 | 178243 | 3 |
| OCBBF2035885 | 0.002081512 | 276141 | 1 |
| OCBBF2035916 | 0.002081512 | 280971 | 1 |
| OCBBF2036019 | 0.002081512 | 102327 | 1 |
| OCBBF2036031 | 0.002081512 | 256314 | 1 |
| OCBBF2036225 | 0.002081512 | 270805 | 1 |
| OCBBF2036476 | 0.002081512 | 235049 | 1 |
| OCBBF2036743 | 0.015159008 | 105049 | 9 |
| OCBBF2036752 | 0.002081512 | 280646 | 1 |
| OCBBF2037068 | 0.011198776 | 66296 | 3 |
| OCBBF2037340 | 0.002081512 | 272665 | 1 |
| OCBBF2037398 | 0.002081512 | 163268 | 1 |
| OCBBF2037464 | 0.002081512 | 280399 | 1 |
| OCBBF2037547 | 0.002081512 | 273560 | 1 |
| OCBBF2037598 | 0.002081512 | 139045 | 1 |
| OCBBF2037638 | 0.002081512 | 264271 | 1 |
| OCBBF2038238 | 0.002081512 | 251782 | 1 |
| OCBBF2038285 | 0.002081512 | 228649 | 1 |
| OCBBF2038317 | 0.016999458 | 138478 | 5 |
| OCBBF3000097 | 0.002081512 | 281772 | 1 |
| OCBBF3000167 | 0.008353798 | 19003 | 4 |
| OCBBF3000213 | 0.002081512 | 276009 | 1 |
| OCBBF3000296 | 0.008844604 | 161430 | 5 |
| OCBBF3000323 | 0.050379897 | 39254 | 19 |
| OCBBF3000372 | 0.002081512 | 217466 | 1 |
| OCBBF3000380 | 0.002081512 | 213957 | 1 |
| OCBBF3000483 | 0.041984388 | 80345 | 22 |
| OCBBF3000743 | 0.002081512 | 194802 | 1 |
| OCBBF3000830 | 0.002081512 | 209136 | 1 |
| OCBBF3001076 | 0.002081512 | 251737 | 1 |
| OCBBF3001202 | 0.002081512 | 252374 | 1 |
| OCBBF3001333 | 0.052150856 | 56997 | 22 |
| OCBBF3001 616 | 0.002081512 | 191894 | 1 |
| OCBBF3002553 | 0.002081512 | 274593 | 1 |
| OCBBF3002600 | 0.002081512 | 277878 | 1 |
| OCBBF3002654 | 0.002081512 | 261718 | 1 |
| OCBBF3003103 | 0.003757059 | 31962 | 2 |
| OCBBF3003209 | 0.002081512 | 240784 | 1 |
| OCBBF3003320 | 0.005177008 | 148775 | 2 |
| OCBBF3003592 | 0.002081512 | 234918 | 1 |
| OCBBF3003745 | 0.002081512 | 267007 | 1 |
| OCBBF3003761 | 0.002081512 | 247541 | 1 |
| OCBBF3004264 | 0.002081512 | 262459 | 1 |
| OCBBF3004314 | 0.002081512 | 152663 | 1 |
| OBBF3004487 | 0.036981384 | 19406 | 15 |
| OCBBF3004908 | 0.017518546 | 186256 | 5 |
| OCBBF3004972 | 0.002081512 | 245944 | 1 |
| OCBBF3005330 | 0.002081512 | 243877 | 1 |
| OCBBF3005597 | 0.002081512 | 251866 | 1 |
| OCBBF3005843 | 0.022896632 | 273128 | 11 |
| OCBBF3006802 | 0.004163024 | 176100 | 2 |
| OCBBF3006986 | 0.002081512 | 237690 | 1 |
| OCBBF3007078 | 0.004163024 | 136202 | 2 |
| OCBBF3007516 | 0.044302137 | 196080 | 4 |
| OCBBF3007704 | 0.002081512 | 237669 | 1 |
| OCBBF3008230 | 0.005615173 | 156207 | 3 |
| OCBBF3008392 | 0.002081512 | 41930 | 1 |
| OCBBF3008835 | 0.003939626 | 130734 | 2 |
| OCBBF3009192 | 0.002081512 | 229002 | 1 |
| OCBBF3009244 | 0.004865067 | 144341 | 3 |
| OCBBF3009279 | 0.002081512 | 68399 | 1 |
| PANCR1000012 | 0.549450549 | 281178 | 1 |
| PANCR1000021 | 0.549450549 | 6611 | 1 |
| PANCR1000081 | 0.560277747 | 79048 | 2 |
| PANCR1000086 | 0.549450549 | 10577 | 1 |
| PANCR1000091 | 0.556842114 | 46146 | 3 |
| PANCR1000185 | 0.549450549 | 38273 | 1 |
| PEBLM1000024 | 0.012594458 | 30084 | 1 |
| PEBLM1000029 | 0.012594458 | 33721 | 1 |
| PEBLM1000034 | 0.02409929 | 15430 | 2 |
| PEBLM1000062 | 0.012594458 | 52061 | 1 |
| PEBLM1000066 | 0.035356037 | 38785 | 6 |
| PEBLM1000068 | 0.012594458 | 73905 | 1 |
| PEBLM1000071 | 0.012594458 | 167585 | 1 |
| PEBLM1000083 | 0.012594458 | 39070 | 1 |
| PEBLM1000144 | 0.027938227 | 19967 | 7 |
| PEBLM1000147 | 0.012594458 | 62124 | 1 |
| PEBLM1000174 | 0.035036537 | 77263 | 7 |
| PEBLM1000180 | 0.012594458 | 15902 | 1 |
| PEBLM2000030 | 0.045526516 | 135158 | 24 |
| PEBLM2000112 | 0.012594458 | 145845 | 1 |
| PEBLM2000126 | 0.015509315 | 126919 | 2 |
| PEBLM2000147 | 0.131342074 | 133636 | 30 |
| PEBLM2000170 | 0.012594458 | 207155 | 1 |
| PEBLM2000180 | 0.012594458 | 275815 | 1 |
| PEBLM2000213 | 0.012594458 | 135244 | 1 |
| PEBLM2000222 | 0.288461073 | 97799 | 16 |
| PEBLM2000248 | 0.012594458 | 129607 | 1 |
| PEBLM2000267 | 0.119163955 | 133717 | 26 |
| PEBLM2000270 | 0.012594458 | 18974 | 1 |
| PEBLM2000308 | 0.084519324 | 102916 | 25 |
| PEBLM2000321 | 0.012594458 | 67784 | 1 |
| PEBLM2000326 | 0.03086749 | 18696 | 13 |
| PEBLM2000333 | 0.012594458 | 156538 | 1 |
| PEBLM2000338 | 0.017605506 | 143249 | 3 |
| PEBLM2000395 | 0.184092338 | 125656 | 30 |
| PEBLM2000479 | 0.035996212 | 126972 | 8 |
| PEBLM2000502 | 0.012594458 | 186313 | 1 |
| PEBLM2001072 | 0.092396545 | 85008 | 27 |
| PEBLM2001312 | 0.012594458 | 181594 | 1 |
| PEBLM2001465 | 0.025188917 | 48617 | 2 |
| PEBLM2001488 | 0.012594458 | 238124 | 1 |
| PEBLM2001803 | 0.01620835 | 126327 | 2 |
| PEBLM2002432 | 0.012594458 | 54344 | 1 |
| PEBLM2002455 | 0.018830816 | 189300 | 2 |
| PEBLM2002594 | 0.012594458 | 189923 | 1 |
| PEBLM2002749 | 0.024381351 | 257254 | 2 |
| PEBLM2002887 | 0.012594458 | 41861 | 1 |
| PEBLM2003426 | 0.012594458 | 142061 | 1 |
| PEBLM2003765 | 0.025188917 | 169706 | 2 |
| PEBLM2003914 | 0.078859739 | 96721 | 24 |
| PEBLM2003935 | 0.012594458 | 228274 | 1 |
| PEBLM2004015 | 0.012594458 | 189214 | 1 |
| PEBLM2004216 | 0.012594458 | 169716 | 1 |
| PEBLM2004290 | 0.012594458 | 225813 | 1 |
| PEBLM2004452 | 0.124147681 | 111666 | 56 |
| PEBLM2004497 | 0.012594458 | 255829 | 1 |
| PEBLM2004666 | 0.045096694 | 95135 | 10 |
| PEBLM2004733 | 0.017533821 | 136973 | 3 |
| PEBLM2005183 | 0.044522139 | 129431 | 4 |
| PEBLM2005282 | 0.014282735 | 149592 | 2 |
| PEBLM2005615 | 0.012594458 | 57736 | 1 |
| PEBLM2005632 | 0.012594458 | 62684 | 1 |
| PEBLM2005697 | 0.012594458 | 141511 | 1 |
| PEBLM2006031 | 0.012594458 | 21395 | 1 |
| PEBLM2006036 | 0.013991614 | 104483 | 2 |
| PEBLM2006049 | 0.012594458 | 48934 | 1 |
| PEBLM2006113 | 0.012594458 | 247663 | 1 |
| PEBLM2006135 | 0.012594458 | 225736 | 1 |
| PEBLM2006215 | 0.012594458 | 109761 | 1 |
| PEBLM2006283 | 0.014303919 | 167957 | 2 |
| PEBLM2006298 | 0.012594458 | 222370 | 1 |
| PEBLM2006366 | 0.060539691 | 9710 | 8 |
| PEBLM2006709 | 0.030116788 | 15102 | 5 |
| PEBLM2006912 | 0.012594458 | 281147 | 1 |
| PEBLM2007112 | 0.012594458 | 167786 | 1 |
| PEBLM2007140 | 0.012594458 | 157636 | 1 |
| PEBLM2007188 | 0.019193932 | 107228 | 4 |
| PEBLM2007296 | 0.096888444 | 69233 | 9 |
| PEBLM2007437 | 0.028103303 | 164651 | 3 |
| PEBLM2007447 | 0.013991614 | 17001 | 2 |
| PEBLM2007598 | 0.025188917 | 113957 | 2 |
| PEBLM2007774 | 0.556323895 | 32081 | 36 |
| PEBLM2007832 | 0.012594458 | 157434 | 1 |
| PEBLM2007834 | 0.012594458 | 280260 | 1 |
| PEBLM2008106 | 0.015715265 | 39307 | 2 |
| PEBLM2008576 | 0.012594458 | 83491 | 1 |
| PEBLM2008605 | 0.012594458 | 46058 | 1 |
| PEBLM2008826 | 0.018066465 | 88934 | 3 |
| PEBLM2008861 | 0.069609533 | 126855 | 4 |
| PERIC1000025 | 0.011286682 | 103613 | 1 |
| PERIC1000147 | 0.011286682 | 215907 | 1 |
| PERIC2000180 | 0.011286682 | 70562 | 1 |
| PERIC2000214 | 0.011286682 | 200235 | 1 |
| PERIC2000386 | 0.011286682 | 132677 | 1 |
| PERIC2000387 | 0.011286682 | 64701 | 1 |
| PERIC2000422 | 0.011286682 | 130524 | 1 |
| PERIC2000473 | 0.026691893 | 86448 | 5 |
| PERIC2000478 | 0.015390783 | 193727 | 3 |
| PERIC2000889 | 0.018835355 | 30504 | 6 |
| PERIC2000914 | 0.011286682 | 166331 | 1 |
| PERIC2001227 | 0.011286682 | 189726 | 1 |
| PERIC2001228 | 0.011286682 | 181564 | 1 |
| PERIC2001379 | 0.011286682 | 227518 | 1 |
| PERIC2001504 | 0.011286682 | 169499 | 1 |
| PERIC2001703 | 0.011286682 | 178937 | 1 |
| PERIC2001764 | 0.011286682 | 199841 | 1 |
| PERIC2002243 | 0.011286682 | 213513 | 1 |
| PERIC2002272 | 0.011286682 | 178940 | 1 |
| PERIC2002452 | 0.017198944 | 187738 | 2 |
| PERIC2002766 | 0.013368194 | 166329 | 2 |
| PERIC2003001 | 0.011286682 | 198035 | 1 |
| PERIC2003090 | 0.033860045 | 173088 | 3 |
| PERIC2003287 | 0.011286682 | 187046 | 1 |
| PERIC2003349 | 0.011286682 | 176351 | 1 |
| PERIC2003452 | 0.011286682 | 171552 | 1 |
| PERIC2003699 | 0.014268641 | 105281 | 2 |
| PERIC2003720 | 0.012974958 | 160208 | 2 |
| PERIC2003794 | 0.01238196 | 162130 | 2 |
| PERIC2003834 | 0.011286682 | 214823 | 1 |
| PERIC2003919 | 0.011286682 | 281469 | 1 |
| PERIC2003957 | 0.011286682 | 206183 | 1 |
| PERIC2004028 | 0.011286682 | 207520 | 1 |
| PERIC2004131 | 0.011286682 | 221197 | 1 |
| PERIC2004259 | 0.011286682 | 157909 | 1 |
| PERIC2004379 | 0.011286682 | 280422 | 1 |
| PERIC2004429 | 0.011286682 | 214091 | 1 |
| PERIC2004510 | 0.011286682 | 280236 | 1 |
| PERIC2004613 | 0.011286682 | 207755 | 1 |
| PERIC2004909 | 0.011286682 | 259979 | 1 |
| PERIC2005111 | 0.011286682 | 153626 | 1 |
| PERIC2005117 | 0.077998528 | 112140 | 7 |
| PERIC2005305 | 0.011286682 | 205154 | 1 |
| PERIC2005347 | 0.011286682 | 207648 | 1 |
| PERIC2005370 | 0.011286682 | 207645 | 1 |
| PERIC2005936 | 0.011286682 | 171566 | 1 |
| PERIC2006035 | 0.011286682 | 200478 | 1 |
| PERIC2006121 | 0.011286682 | 279172 | 1 |
| PERIC2006443 | 0.034500115 | 79859 | 4 |
| PERIC2006945 | 0.011286682 | 196826 | 1 |
| PERIC2006960 | 0.011286682 | 281970 | 1 |
| PERIC2007068 | 0.108591717 | 109548 | 42 |
| PERIC2007439 | 0.011286682 | 161406 | 1 |
| PERIC2007914 | 0.011286682 | 86598 | 1 |
| PERIC2008008 | 0.011286682 | 133478 | 1 |
| PERIC2008385 | 0.011286682 | 275402 | 1 |
| PERIC2009001 | 0.011286682 | 125305 | 1 |
| PERIC2009086 | 0.011286682 | 281601 | 1 |
| PERIC2009329 | 0.011286682 | 243202 | 1 |
| PERIC2009566 | 0.011286682 | 228471 | 1 |
| PLACE5000001 | 0.002981959 | 32814 | 1 |
| PLACE5000013 | 0.105555352 | 59531 | 32 |
| PLACE5000037 | 0.002981959 | 58436 | 1 |
| PLACE5000058 | 0.004840074 | 72392 | 2 |
| PLACE5000066 | 0.01831269 | 18212 | 5 |
| PLACE5000067 | 0.004077237 | 71744 | 2 |
| PLACE5000068 | 0.026680727 | 46897 | 5 |
| PLACE5000070 | 0.002981959 | 41653 | 1 |
| PLACE5000080 | 0.013584164 | 78840 | 2 |
| PLACE5000105 | 0.002981959 | 56014 | 1 |
| PLACE5000113 | 0.097388055 | 57624 | 45 |
| PLACE5000115 | 0.002981959 | 43408 | 1 |
| PLACE5000116 | 0.002981959 | 29697 | 1 |
| PLACE5000129 | 0.002981959 | 9718 | 1 |
| PLACE5000139 | 0.014268641 | 162829 | 2 |
| PLACE5000153 | 0.002981959 | 21397 | 1 |
| PLACE5000159 | 0.073725999 | 66100 | 32 |
| PLACE5000170 | 0.048192951 | 91940 | 17 |
| PLACE5000171 | 0.002981959 | 73763 | 1 |
| PLACE5000172 | 0.128909714 | 78101 | 44 |
| PLACE5000178 | 0.004657506 | 84669 | 2 |
| PLACE5000184 | 0.040642311 | 62231 | 14 |
| PLACE5000245 | 0.002981959 | 211938 | 1 |
| PLACE5000260 | 0.002981959 | 114167 | 1 |
| PLACE5000282 | 0.013584164 | 37600 | 2 |
| PLACE5000297 | 0.004691419 | 225360 | 2 |
| PLACE5000372 | 0.00498036 | 187595 | 2 |
| PLACE5000492 | 0.006826043 | 52889 | 2 |
| PLACE5000522 | 0.002981959 | 73488 | 1 |
| PLACE5000527 | 0.002981959 | 61350 | 1 |
| PLACE6000012 | 0.002981959 | 142402 | 1 |
| PLACE6000044 | 0.002981959 | 233552 | 1 |
| PLACE6000055 | 0.002981959 | 159292 | 1 |
| PLACE6000070 | 0.002981959 | 282621 | 1 |
| PLACE6000080 | 0.040733555 | 194458 | 4 |
| PLACE6000137 | 0.002981959 | 137073 | 1 |
| PLACE6000145 | 0.16167989 | 158642 | 9 |
| PLACE6000191 | 0.002981959 | 86527 | 1 |
| PLACE6000205 | 0.002981959 | 231541 | 1 |
| PLACE6000231 | 0.002981959 | 246390 | 1 |
| PLACE6000263 | 0.110207265 | 38307 | 27 |
| PLACE6000296 | 0.002981959 | 69473 | 1 |
| PLACE6000314 | 0.0109856 | 145628 | 3 |
| PLACE6000325 | 0.020683518 | 151732 | 5 |
| PLACE6000348 | 0.011769097 | 199737 | 3 |
| PLACE6000371 | 0.005896816 | 216226 | 2 |
| PLACE6000379 | 0.948638408 | 8957 | 184 |
| PLACE6000414 | 0.130761853 | 102490 | 34 |
| PLACE6000424 | 0.002981959 | 246212 | 1 |
| PLACE6000426 | 0.002981959 | 282680 | 1 |
| PLACE6000429 | 0.03578351 | 150571 | 12 |
| PLACE6000450 | 0.002981959 | 233544 | 1 |
| PLACE6000463 | 0.021474356 | 108850 | 7 |
| PLACE6000481 | 0.068082902 | 98528 | 25 |
| PLACE6000523 | 0.002981959 | 241171 | 1 |
| PLACE6000550 | 0.002981959 | 262030 | 1 |
| PLACE6000552 | 0.002981959 | 261505 | 1 |
| PLACE6000555 | 0.002981959 | 224941 | 1 |
| PLACE6000619 | 0.004670236 | 147238 | 2 |
| PLACE6000630 | 0.043424282 | 120712 | 4 |
| PLACE6000953 | 0.002981959 | 202422 | 1 |
| PLACE6001064 | 0.292054675 | 45807 | 103 |
| PLACE6001118 | 0.002981959 | 244197. | 1 |
| PLACE6001153 | 0.002981959 | 251398 | 1 |
| PLACE6001185 | 0.007845152 | 142257 | 4 |
| PLACE6001262 | 0.019175337 | 129776 | 4 |
| PLACE6001281 | 0.048671412 | 104937 | 15 |
| PLACE6001294 | 0.002981959 | 67215 | 1 |
| PLACE6001443 | 0.085304876 | 86362 | 19 |
| PLACE6001536 | 0.002981959 | 266076 | 1 |
| PLACE6001557 | 0.014909796 | 177257 | 5 |
| PLACE6001712 | 0.005963918 | 224885 | 2 |
| PLACE6001740 | 0.002981959 | 81387 | 1 |
| PLACE6001823 | 0.005880664 | 124934 | 3 |
| PLACE6001886 | 0.009893619 | 172644 | 4 |
| PLACE6001923 | 0.026657538 | 188536 | 4 |
| PLACE6001925 | 0.002981959 | 223800 | 1 |
| PLACE6001933 | 0.002981959 | 237523 | 1 |
| PLACE6002011 | 0.005896816 | 147840 | 2 |
| PLACE6002016 | 0.002981959 | 92029 | 1 |
| PLACE6002056 | 0.005896816 | 176255 | 2 |
| PLACE6002084 | 0.453700393 | 54927 | 62 |
| PLACE6002102 | 0.118635564 | 90575 | 55 |
| PLACE6002151 | 0.084519324 | 102916 | 25 |
| PLACE6002157 | 0.002981959 | 280333 | 1 |
| PLACE6002312 | 0.002981959 | 268063 | 1 |
| PLACE6002323 | 0.005963918 | 129204 | 2 |
| PLACE6002345 | 0.002981959 | 58982 | 1 |
| PLACE6002419 | 0.016072744 | 150462 | 3 |
| PLACE6002462 | 0.002981959 | 102861 | 1 |
| PLACE6002647 | 0.002981959 | 233924 | 1 |
| PLACE6002668 | 0.031047649 | 35038 | 7 |
| PLACE6002692 | 0.004670236 | 165921 | 2 |
| PLACE6002699 | 0.002981959 | 141477 | 1 |
| PLACE6002710 | 0.002981959 | 152059 | 1 |
| PLACE6002871 | 0.002981959 | 224996 | 1 |
| PLACE6002949 | 0.002981959 | 227915 | 1 |
| PLACE6002960 | 0.150965242 | 108462 | 38 |
| PLACE6003004 | 0.004840074 | 10286 | 2 |
| PLACE6003038 | 0.04642828 | 189643 | 7 |
| PLACE6003077 | 0.006595851 | 10590 | 2 |
| PLACE6003094 | 0.005963918 | 141265 | 2 |
| PLACE6003109 | 0.002981959 | 3992 | 1 |
| PLACE6003204 | 0.007639465 | 189843 | 3 |
| PLACE6003218 | 0.011514708 | 137767 | 5 |
| PLACE6003372 | 0.002981959 | 209685 | 1 |
| PLACE6003383 | 0.140382946 | 157299 | 20 |
| PLACE6003393 | 0.022065385 | 58710 | 4 |
| PLACE6003399 | 0.002981959 | 282920 | 1 |
| PLACE6003539 | 0.002981959 | 187678 | 1 |
| PLACE6003621 | 0.004171204 | 191249 | 2 |
| PLACE6003705 | 0.002981959 | 209018 | 1 |
| PLACE6003740 | 0.002981959 | 193349 | 1 |
| PLACE6003745 | 0.0318873 | 114630 | 10 |
| PLACE6003771 | 0.002981959 | 244586 | 1 |
| PLACE6003796 | 0.002981959 | 246821 | 1 |
| PLACE6003839 | 0.002981959 | 244603 | 1 |
| PLACE6003850 | 0.014936137 | 203681 | 3 |
| PLACE6004005 | 0.169706805 | 109741 | 51 |
| PLACE6004101 | 0.037607998 | 212276 | 2 |
| PLACE6004219 | 0.002981959 | 22188 | 1 |
| PLACE6004312 | 0.277824579 | 74045 | 42 |
| PLACE6004336 | 0.002981959 | 219713 | 1 |
| PLACE6004368 | 0.002981959 | 178791 | 1 |
| PLACE6004380 | 0.002981959 | 174613 | 1 |
| PLACE6004396 | 0.009127485 | 128856 | 2 |
| PLACE6004397 | 0.091161491 | 110015 | 21 |
| PLACE6004454 | 0.002981959 | 141920 | 1 |
| PLACE6004464 | 0.008878775 | 115119 | 3 |
| PLACE6004491 | 0.00498036 | 254838 | 2 |
| PLACE6004578 | 0.135172242 | 67455 | 19 |
| PLACE6004663 | 0.002981959 | 144252 | 1 |
| PLACE6004687 | 0.002981959 | 143498 | 1 |
| PLACE6004738 | 0.00796232 | 47684 | 3 |
| PLACE6004931 | 0.025588656 | 60981 | 3 |
| PLACE6004966 | 0.002981959 | 228624 | 1 |
| PLACE6004993 | 0.008787137 | 87962 | 2 |
| PLACE6005007 | 0.005963918 | 244655 | 2 |
| PLACE6005029 | 0.002981959 | 139585 | 1 |
| PLACE6005108 | 0.002981959 | 237247 | 1 |
| PLACE6005231 | 0.015170733 | 4887 | 9 |
| PLACE6005234 | 0.002981959 | 230943 | 1 |
| PLACE6005283 | 0.002981959 | 123008 | 1 |
| PLACE6005294 | 0.013915382 | 70074 | 4 |
| PLACE6005328 | 0.002981959 | 113499 | 1 |
| PLACE6005351 | 0.002981959 | 228275 | 1 |
| PLACE6005423 | 0.002981959 | 148085 | 1 |
| PLACE6005482 | 0.063366236 | 79410 | 29 |
| PLACE6005487 | 0.044787979 | 122090 | 2 |
| PLACE6005535 | 0.002981959 | 122731 | 1 |
| PLACE6005546 | 0.005963918 | 9510 | 2 |
| PLACE6005559 | 0.002981959 | 228221 | 1 |
| PLACE6005579 | 0.005963918 | 191997 | 2 |
| PLACE6005691 | 0.004670236 | 187091 | 2 |
| PLACE6005786 | 0.002981959 | 261980 | 1 |
| PLACE6006042 | 0.030779023 | 32799 | 7 |
| PLACE6006077 | 0.002981959 | 230941 | 1 |
| PLACE6006137 | 0.049056896 | 83677 | 20 |
| PLACE6006158 | 0.002981959 | 91461 | 1 |
| PLACE6006186 | 0.02316567 | 141263 | 7 |
| PLACE6006217 | 0.002981959 | 36239 | 1 |
| PLACE6006222 | 0.020053767 | 55170 | 6 |
| PLACE6006266 | 0.002981959 | 32020 | 1 |
| PLACE6006287 | 0.005963918 | 206102 | 2 |
| PLACE6006394 | 0.002981959 | 241272 | 1 |
| PLACE6006418 | 0.060438853 | 129737 | 11 |
| PLACE6006474 | 0.002981959 | 243949 | 1 |
| PLACE6006549 | 0.209837509 | 143567 | 24 |
| PLACE6006566 | 0.002981959 | 163855 | 1 |
| PLACE6006697 | 0.002981959 | 205332 | 1 |
| PLACE6006822 | 0.002981959 | 240828 | 1 |
| PLACE6006871 | 0.008945877 | 127180 | 3 |
| PLACE6006905 | 0.002981959 | 185464 | 1 |
| PLACE6006988 | 0.005063471 | 202659 | 2 |
| PLACE6007050 | 0.002981959 | 92951 | 1 |
| PLACE6007180 | 0.015634486 | 152461 | 9 |
| PLACE6007239 | 0.040642311 | 62231 | 14 |
| PLACE6007242 | 0.005963918 | 78475 | 2 |
| PLACE6007309 | 0.009218317 | 65055 | 2 |
| PLACE6007373 | 0.058550326 | 91565 | 4 |
| PLACE6007380 | 0.002981959 | 219634 | 1 |
| PLACE6007433 | 0.002981959 | 244442 | 1 |
| PLACE6007436 | 0.046638229 | 69178 | 13 |
| PLACE6007482 | 0.002981959 | 256521 | 1 |
| PLACE6007687 | 0.002981959 | 127517 | 1 |
| PLACE6007787 | 0.002981959 | 236738 | 1 |
| PLACE6007798 | 0.002981959 | 239802 | 1 |
| PLACE6007807 | 0.002981959 | 252721 | 1 |
| PLACE6007925 | 0.016534781 | 47690 | 3 |
| PLACE6008036 | 0.002981959 | 260248 | 1 |
| PLACE6008094 | 0.002981959 | 224813 | 1 |
| PLACE6008126 | 0.002981959 | 173845 | 1 |
| PLACE6008285 | 0.002981959 | 224811 | 1 |
| PLACE6008315 | 0.120334926 | 134760 | 17 |
| PLACE6008444 | 0.004670236 | 201715 | 2 |
| PLACE6008453 | 0.002981959 | 260252 | 1 |
| PLACE6008640 | 0.002981959 | 101822 | 1 |
| PLACE6008750 | 0.002981959 | 254292 | 1 |
| PLACE6008768 | 0.002981959 | 12946 | 1 |
| PLACE6008775 | 0.090907954 | 64913 | 40 |
| PLACE6008793 | 0.105679668 | 75205 | 10 |
| PLACE6008824 | 0.00498036 | 145425 | 2 |
| PLACE6008989 | 0.002981959 | 217252 | 1 |
| PLACE6009006 | 0.002981959 | 251955 | 1 |
| PLACE6009008 | 0.008924083 | 8038 | 2 |
| PLACE6009228 | 0.005896816 | 193730 | 2 |
| PLACE6009237 | 0.038966126 | 189299 | 2 |
| PLACE6009338 | 0.006668637 | 130112 | 3 |
| PLACE6009419 | 0.002981959 | 255777 | 1 |
| PLACE6009430 | 0.002981959 | 258442 | 1 |
| PLACE6009482 | 0.002981959 | 129207 | 1 |
| PLACE6009524 | 0.002981959 | 255553 | 1 |
| PLACE6009527 | 0.002981959 | 136976 | 1 |
| PLACE6009560 | 0.015505441 | 47014 | 2 |
| PLACE6009590 | 0.002981959 | 209325 | 1 |
| PLACE6009821 | 0.01088164 | 135568 | 3 |
| PLACE6009835 | 0.002981959 | 1907 | 1 |
| PLACE6009853 | 0.002981959 | 124518 | 1 |
| PLACE6010077 | 0.192446976 | 13832 | 21 |
| PLACE6010463 | 0.002981959 | 186685 | 1 |
| PLACE6010484 | 0.108402522 | 91463 | 21 |
| PLACE6010485 | 0.002981959 | 192009 | 1 |
| PLACE6010568 | 0.002981959 | 133287 | 1 |
| PLACE6010701 | 0.008924083 | 142837 | 2 |
| PLACE6010704 | 0.004657506 | 155333 | 2 |
| PLACE6010732 | 0.006826043 | 91093 | 2 |
| PLACE6010765 | 0.014268641 | 207183 | 2 |
| PLACE6010848 | 0.02047616 | 47531 | 5 |
| PLACE6010925 | 0.002981959 | 205614 | 1 |
| PLACE6010936 | 0.013616113 | 194062 | 5 |
| PLACE6010991 | 0.002981959 | 71143 | 1 |
| PLACE6011041 | 0.061584463 | 133852 | 10 |
| PLACE6011057 | 0.074343501 | 138019 | 22 |
| PLACE6011102 | 0.002981959 | 70787 | 1 |
| PLACE6011156 | 0.012867609 | 94430 | 3 |
| PLACE6011211 | 0.002981959 | 92518 | 1 |
| PLACE6011260 | 0.002981959 | 129293 | 1 |
| PLACE6011334 | 0.018642681 | 60769 | 10 |
| PLACE6011627 | 0.005063471 | 62845 | 2 |
| PLACE6011774 | 0.002981959 | 143073 | 1 |
| PLACE6011881 | 0.002981959 | 234564 | 1 |
| PLACE6011907 | 0.002981959 | 240727 | 1 |
| PLACE6011970 | 0.006460627 | 56455 | 3 |
| PLACE6011975 | 0.002981959 | 278157 | 1 |
| PLACE6012028 | 0.026692405 | 80766 | 7 |
| PLACE6012108 | 0.002981959 | 186848 | 1 |
| PLACE6012168 | 0.002981959 | 179133 | 1 |
| PLACE6012297 | 0.002981959 | 87637 | 1 |
| PLACE6012308 | 0.002981959 | 39673 | 1 |
| PLACE6012574 | 0.16393523 | 84610 | 8 |
| PLACE6012604 | 0.007293972 | 53766 | 3 |
| PLACE6012908 | 0.002981959 | 255215 | 1 |
| PLACE6012936 | 0.004379115 | 70979 | 2 |
| PLACE6012942 | 0.00498036 | 3342 | 2 |
| PLACE6013217 | 0.186122649 | 168945 | 15 |
| PLACE6013220 | 0.016584682 | 116953 | 9 |
| PLACE6013222 | 0.034004893 | 179146 | 11 |
| PLACE6013232 | 0.014501369 | 63902 | 2 |
| PLACE6013288 | 0.002981959 | 27684 | 1 |
| PLACE6013386 | 0.002981959 | 43528 | 1 |
| PLACE6013400 | 0.002981959 | 275480 | 1 |
| PLACE6013650 | 0.002981959 | 213359 | 1 |
| PLACE6013672 | 0.006067391 | 85882 | 3 |
| PLACE6013883 | 0.004840074 | 39591 | 2 |
| PLACE6013884 | 0.002981959 | 118409 | 1 |
| PLACE6013885 | 0.002981959 | 115819 | 1 |
| PLACE6014043 | 0.002981959 | 237579 | 1 |
| PLACE6014064 | 0.05474534 | 50978 | 5 |
| PLACE6014516 | 0.002981959 | 198933 | 1 |
| PLACE6014882 | 0.014106667 | 136335 | 2 |
| PLACE6015051 | 0.036347827 | 78385 | 6 |
| PLACE6015211 | 0.002981959 | 190099 | 1 |
| PLACE6015322 | 0.005896816 | 220766 | 2 |
| PLACE6015445 | 0.015077893 | 166201 | 3 |
| PLACE6015460 | 0.002981959 | 191974 | 1 |
| PLACE6015513 | 0.006595851 | 176199 | 2 |
| PLACE6015591 | 0.002981959 | 182279 | 1 |
| PLACE6015729 | 0.004670236 | 220756 | 2 |
| PLACE6015731 | 0.010916135 | 90857 | 3 |
| PLACE6015939 | 0.002981959 | 245924 | 1 |
| PLACE6016030 | 0.002981959 | 121465 | 1 |
| PLACE6016093 | 0.002981959 | 143645 | 1 |
| PLACE6016105 | 0.002981959 | 148330 | 1 |
| PLACE6016160 | 0.002981959 | 232923 | 1 |
| PLACE6016210 | 0.013231277 | 198754 | 3 |
| PLACE6016254 | 0.009170813 | 106930 | 4 |
| PLACE6016383 | 0.025917958 | 150264 | 5 |
| PLACE6016942 | 0.042831316 | 105440 | 10 |
| PLACE6016984 | 0.009070534 | 148094 | 4 |
| PLACE6017002 | 0.002981959 | 209451 | 1 |
| PLACE6017431 | 0.00498036 | 147771 | 2 |
| PLACE6017564 | 0.002981959 | 156714 | 1 |
| PLACE6017578 | 0.016566124 | 73676 | 3 |
| PLACE6017626 | 0.011805381 | 37993 | 3 |
| PLACE6017701 | 0.006826043 | 156072 | 2 |
| PLACE6017714 | 0.002981959 | 176249 | 1 |
| PLACE6017788 | 0.004077237 | 188015 | 2 |
| PLACE6017791 | 0.015255418 | 79365 | 3 |
| PLACE6018031 | 0.024920637 | 130792 | 7 |
| PLACE6018107 | 0.002981959 | 206119 | 1 |
| PLACE6018187 | 0.011927837 | 162369 | 4 |
| PLACE6018235 | 0.002981959 | 105345 | 1 |
| PLACE6018287 | 0.007059197 | 202180 | 3 |
| PLACE6018441 | 0.002981959 | 202821 | 1 |
| PLACE6018487 | 0.251080958 | 102855 | 6 |
| PLACE6018497 | 0.002981959 | 185011 | 1 |
| PLACE6018769 | 0.002981959 | 225171 | 1 |
| PLACE6018834 | 0.002981959 | 233027 | 1 |
| PLACE6018843 | 0.002981959 | 237222 | 1 |
| PLACE6018863 | 0.008823422 | 144823 | 2 |
| PLACE6018938 | 0.021839764 | 83044 | 8 |
| PLACE6019195 | 0.019746648 | 85025 | 8 |
| PLACE6019385 | 0.041308487 | 172131 | 3 |
| PLACE6019542 | 0.002981959 | 232568 | 1 |
| PLACE6019600 | 0.002981959 | 80534 | 1 |
| PLACE6019674 | 0.002981959 | 186850 | 1 |
| PLACE6019676 | 0.002981959 | 58232 | 1 |
| PLACE6019701 | 0.002981959 | 227715 | 1 |
| PLACE6019932 | 0.038632583 | 275162 | 2 |
| PLACE6020031 | 0.008945877 | 182352 | 3 |
| PLACE6020145 | 0.019965318 | 225193 | 3 |
| PLACE7000167 | 0.002981959 | 262219 | 1 |
| PLACE7000170 | 0.002981959 | 253467 | 1 |
| PLACE7000266 | 0.008945877 | 97952 | 3 |
| PLACE7000333 | 0.002981959 | 173394 | 1 |
| PLACE7000410 | 0.004840074 | 117459 | 2 |
| PLACE7000502 | 0.002981959 | 277703 | 1 |
| PLACE7000514 | 0.035226902 | 144430 | 6 |
| PLACE7000707 | 0.002981959 | 269925 | 1 |
| PLACE7001022 | 0.005954079 | 211334 | 3 |
| PLACE7001544 | 0.002981959 | 205948 | 1 |
| PLACE7001759 | 0.002981959 | 243868 | 1 |
| PLACE7001936 | 0.002981959 | 41454 | 1 |
| PLACE7002303 | 0.002981959 | 274816 | 1 |
| PLACE7002641 | 0.002981959 | 249853 | 1 |
| PLACE7003639 | 0.002981959 | 193910 | 1 |
| PLACE7003657 | 0.002981959 | 283762 | 1 |
| PLACE7003684 | 0.002981959 | 196750 | 1 |
| PLACE7003985 | 0.002981959 | 267452 | 1 |
| PLACE7004103 | 0.129563018 | 20704 | 50 |
| PLACE7004961 | 0.002981959 | 147425 | 1 |
| PLACE7005169 | 0.002981959 | 194095 | 1 |
| PLACE7005671 | 0.002981959 | 269594 | 1 |
| PLACE7005840 | 0.004379115 | 111366 | 2 |
| PLACE7006051 | 0.002981959 | 253074 | 1 |
| PLACE7006090 | 0.002981959 | 247503 | 1 |
| PLACE7006240 | 0.006655907 | 169060 | 3 |
| PLACE7006268 | 0.008924083 | 147886 | 2 |
| PLACE7006275 | 0.002981959 | 234796 | 1 |
| PLACE7006498 | 0.002981959 | 94118 | 1 |
| PLACE7006540 | 0.002981959 | 198954 | 1 |
| PLACE7007379 | 0.002981959 | 121060 | 1 |
| PLACE7007644 | 0.002981959 | 256302 | 1 |
| PLACE7007973 | 0.008945877 | 186973 | 3 |
| PLACE7008136 | 0.002981959 | 211294 | 1 |
| PLACE7008261 | 0.005880664 | 123305 | 3 |
| PLACE7008431 | 0.002981959 | 90795 | 1 |
| PLACE7008623 | 0.002981959 | 191577 | 1 |
| PLACE7008766 | 0.002981959 | 208298 | 1 |
| PLACE7009563 | 0.002981959 | 90811 | 1 |
| PROST1000033 | 0.015208456 | 68409 | 3 |
| PROST1000039 | 0.005942124 | 37168 | 1 |
| PROST1000065 | 0.005942124 | 54612 | 1 |
| PROST1000083 | 0.005942124 | 52758 | 1 |
| PROST1000085 | 0.024220018 | 60154 | 4 |
| PROST1000097 | 0.284962709 | 41964 | 74 |
| PROST1000110 | 0.011884247 | 72420 | 2 |
| PROST1000120 | 0.050810917 | 79126 | 9 |
| PROST1000123 | 0.005942124 | 11422 | 1 |
| PROST1000136 | 0.005942124 | 86583 | 1 |
| PROST1000144 | 0.106872442 | 23853 | 15 |
| PROST1000145 | 0.005942124 | 62838 | 1 |
| PROST1000152 | 0.005942124 | 63034 | 1 |
| PROST1000167 | 0.005942124 | 51368 | 1 |
| PROST1000184 | 0.005942124 | 65567 | 1 |
| PROST1000199 | 0.22932478 | 745 | 33 |
| PROST1000215 | 0.032759511 | 143590 | 10 |
| PROST1000217 | 0.005942124 | 268762 | 1 |
| PROST1000220 | 0.24678982 | 41658 | 31 |
| PROST1000226 | 0.093650062 | 139294 | 23 |
| PROST1000246 | 0.015250292 | 71608 | 4 |
| PROST1000262 | 0.005942124 | 96926 | 1 |
| PROST1000272 | 0.053103795 | 15833 | 5 |
| PROST1000298 | 0.005942124 | 82465 | 1 |
| PROST1000322 | 0.01401869 | 260802 | 3 |
| PROST1000334 | 0.005942124 | 60616 | 1 |
| PROST1000343 | 0.058985342 | 20205 | 9 |
| PROST1000362 | 0.005942124 | 171339 | 1 |
| PROST1000377 | 0.011884247 | 173352 | 2 |
| PROST1000383 | 0.007800238 | 235999 | 2 |
| PROST1000412 | 0.005942124 | 264154 | 1 |
| PROST1000419 | 0.005942124 | 256961 | 1 |
| PROST1000451 | 0.005942124 | 254669 | 1 |
| PROST1000480 | 0.008856981 | 247354 | 2 |
| PROST1000485 | 0.005942124 | 265300 | 1 |
| PROST1000493 | 0.005942124 | 258970 | 1 |
| PROST1000512 | 0.077839882 | 142555 | 8 |
| PROST1000526 | 0.066319138 | 118392 | 17 |
| PROST1000527 | 0.005942124 | 263236 | 1 |
| PROST1000528 | 0.005942124 | 251783 | 1 |
| PROST1000536 | 0.027867756 | 142100 | 5 |
| PROST1000559 | 0.005942124 | 226315 | 1 |
| PROST1000564 | 0.124646322 | 66986 | 21 |
| PROST1000575 | 0.005942124 | 279492 | 1 |
| PROST2000036 | 0.059603321 | 107170 | 4 |
| PROST2000053 | 0.005942124 | 132328 | 1 |
| PROST2000067 | 0.005942124 | 172345 | 1 |
| PROST2000079 | 0.055977201 | 115555 | 5 |
| PROST2000105 | 0.005942124 | 69501 | 1 |
| PROST2000138 | 1.933229642 | 4956 | 246 |
| PROST2000143 | 0.005942124 | 191485 | 1 |
| PROST2000162 | 0.005942124 | 184470 | 1 |
| PROST2000165 | 0.018349418 | 19562 | 5 |
| PROST2000176 | 0.066015708 | 113238 | 4 |
| PROST2000203 | 0.005942124 | 186293 | 1 |
| PROST2000206 | 0.007651584 | 118030 | 2 |
| PROST2000212 | 0.005942124 | 248980 | 1 |
| PROST2000241 | 0.027380781 | 11577 | 3 |
| PROST2000267 | 0.005942124 | 153872 | 1 |
| PROST2000273 | 0.007940525 | 98354 | 2 |
| PROST2000274 | 0.016236363 | 186300 | 5 |
| PROST2000277 | 0.007818965 | 167371 | 2 |
| PROST2000284 | 0.047848309 | 38032 | 14 |
| PROST2000325 | 0.023670143 | 89004 | 4 |
| PROST2000337 | 0.00903762 | 207257 | 2 |
| PROST2000432 | 0.012155233 | 43527 | 2 |
| PROST2000452 | 0.005942124 | 181251 | 1 |
| PROST2000463 | 0.009786208 | 157325 | 2 |
| PROST2000470 | 0.005942124 | 274241 | 1 |
| PROST2000505 | 0.005942124 | 45686 | 1 |
| PROST2000567 | 0.011697798 | 142858 | 5 |
| PROST2000572 | 0.005942124 | 143297 | 1 |
| PROST2000717 | 0.005942124 | 98172 | 1 |
| PROST2000760 | 0.059731529 | 101309 | 7 |
| PROST2000761 | 0.017702679 | 196925 | 2 |
| PROST2000835 | 0.007818965 | 217202 | 2 |
| PROST2000893 | 0.010119059 | 71562 | 2 |
| PROST2000961 | 0.005942124 | 260135 | 1 |
| PROST2001002 | 0.008528524 | 43003 | 3 |
| PROST2001116 | 0.007818965 | 253876 | 2 |
| PROST2001138 | 0.005942124 | 238843 | 1 |
| PROST2001180 | 0.067482781 | 56334 | 23 |
| PROST2001213 | 0.020099465 | 108990 | 4 |
| PROST2001283 | 0.078746926 | 165282 | 7 |
| PROST2001414 | 0.005942124 | 262600 | 1 |
| PROST2001415 | 0.005942124 | 267672 | 1 |
| PROST2001465 | 0.005942124 | 277146 | 1 |
| PROST2001521 | 0.137902849 | 56944 | 6 |
| PROST2001540 | 0.005942124 | 250616 | 1 |
| PROST2001552 | 0.19248918 | 150521 | 19 |
| PROST2001599 | 0.005942124 | 260119 | 1 |
| PROST2001611 | 0.005942124 | 255954 | 1 |
| PROST2001636 | 0.005942124 | 250617 | 1 |
| PROST2001676 | 0.277824579 | 74045 | 42 |
| PROST2001739 | 0.050689687 | 168573 | 6 |
| PROST2001796 | 0.005942124 | 67214 | 1 |
| PROST2001805 | 0.005942124 | 250560 | 1 |
| PROST2001823 | 0.00903762 | 183777 | 2 |
| PROST2001883 | 0.187602336 | 106283 | 7 |
| PROST2001899 | 0.005942124 | 195292 | 1 |
| PROST2001997 | 0.028739455 | 171146 | 9 |
| PROST2001998 | 0.005942124 | 260115 | 1 |
| PROST2002078 | 0. 068946598 | 119990 | 4 |
| PROST2002101 | 0.005942124 | 19202 | 1 |
| PROST2002162 | 0.043281024 | 68508 | 3 |
| PROST2002212 | 0.005942124 | 51158 | 1 |
| PROST2002227 | 0.065918687 | 2930 | 3 |
| PROST2002338 | 0.005942124 | 162306 | 1 |
| PROST2002425 | 0.010545257 | 67345 | 3 |
| PROST2002488 | 0.005942124 | 4455 | 1 |
| PROST2002489 | 0.005942124 | 113822 | 1 |
| PROST2002527 | 0.005942124 | 258299 | 1 |
| PROST2002591 | 0.005942124 | 192943 | 1 |
| PROST2002602 | 0.005942124 | 265032 | 1 |
| PROST2002633 | 0.005942124 | 285163 | 1 |
| PROST2002651 | 0.056030105 | 153652 | 12 |
| PROST2002682 | 0.005942124 | 45850 | 1 |
| PROST2002685 | 0.005942124 | 256774 | 1 |
| PROST2002722 | 0.005942124 | 264712 | 1 |
| PROST2002736 | 0.005942124 | 273201 | 1 |
| PROST2002842 | 0.005942124 | 249187 | 1 |
| PROST2002897 | 0.007818965 | 185074 | 2 |
| PROST2002906 | 0.005942124 | 263237 | 1 |
| PROST2002927 | 0.015650862 | 119679 | 2 |
| PROST2002960 | 0.012465673 | 137655 | 4 |
| PROST2003102 | 0.005942124 | 260933 | 1 |
| PROST2003117 | 0.005942124 | 108224 | 1 |
| PROST2003210 | 0.005942124 | 112569 | 1 |
| PROST2003232 | 0.245575329 | 93213 | 41 |
| PROST2003302 | 0.005942124 | 255108 | 1 |
| PROST2003303 | 0.005942124 | 259127 | 1 |
| PROST2003324 | 0.009475785 | 159922 | 3 |
| PROST2003338 | 0.149695946 | 94357 | 35 |
| PROST2003340 | 0.005942124 | 18611 | 1 |
| PROST2003396 | 0.02804639 | 55143 | 12 |
| PROST2003428 | 0.008856981 | 152383 | 2 |
| PROST2003472 | 0.005942124 | 62749 | 1 |
| PROST2003517 | 0.063645034 | 95698 | 12 |
| PROST2003537 | 0.007818965 | 158815 | 2 |
| PROST2003563 | 0.005942124 | 238922 | 1 |
| PROST2003577 | 0.005942124 | 166410 | 1 |
| PROST2003583 | 0.005942124 | 91382 | 1 |
| PROST2003586 | 0.005942124 | 166577 | 1 |
| PROST2003628 | 0.005942124 | 254951 | 1 |
| PROST2003635 | 0.005942124 | 224705 | 1 |
| PROST2003674 | 0.005942124 | 146045 | 1 |
| PROST2003676 | 0.005942124 | 178577 | 1 |
| PROST2003725 | 0.007339279 | 110404 | 2 |
| PROST2003732 | 0.005942124 | 152808 | 1 |
| PROST2003775 | 0.005942124 | 54011 | 1 |
| PROST2003922 | 0.005942124 | 285074 | 1 |
| PROST2003930 | 0.005942124 | 284890 | 1 |
| PROST2004095 | 0.357992553 | 42362 | 118 |
| PROST2004115 | 0.005942124 | 71454 | 1 |
| PROST2004142 | 0.005942124 | 118631 | 1 |
| PROST2004146 | 0.005942124 | 157078 | 1 |
| PROST2004161 | 0.005942124 | 159965 | 1 |
| PROST2004251 | 0.187026454 | 228740 | 3 |
| PROST2004258 | 0.043916771 | 115981 | 10 |
| PROST2004270 | 0.284947649 | 53529 | 60 |
| PROST2004332 | 0.005942124 | 33031 | 1 |
| PROST2004416 | 0.246421275 | 60996 | 32 |
| PROST2004481 | 0.005942124 | 264549 | 1 |
| PROST2004526 | 0.005942124 | 75000 | 1 |
| PROST2004570 | 0.022353615 | 72423 | 5 |
| PROST2004727 | 0.005942124 | 135711 | 1 |
| PROST2004739 | 0.022532564 | 98637 | 12 |
| PROST2004742 | 0.005942124 | 137871 | 1 |
| PROST2004744 | 0.031694293 | 231522 | 4 |
| PROST2004817 | 0.005942124 | 35516 | 1 |
| PROST2005039 | 0.012158426 | 151600 | 3 |
| PROST2005067 | 0.010386638 | 96873 | 4 |
| PROST2005076 | 0.023270371 | 175251 | 3 |
| PROST2005093 | 0.007131368 | 133468 | 2 |
| PROST2005121 | 0. 005942124 | 149591 | 1 |
| PROST2005131 | 0.005942124 | 7268 | 1 |
| PROST2005143 | 0.02469955 | 112012 | 8 |
| PROST2005228 | 0.005942124 | 158160 | 1 |
| PROST2005272 | 0.020403493 | 124137 | 6 |
| PROST2005285 | 0.022448721 | 104123 | 8 |
| PROST2005426 | 0.005942124 | 162545 | 1 |
| PROST2005466 | 0.007131368 | 183605 | 2 |
| PROST2005604 | 0.053867037 | 125604 | 19 |
| PROST2005630 | 0.005942124 | 264954 | 1 |
| PROST2005683 | 0.01841805 | 146611 | 3 |
| PROST2005788 | 0.005942124 | 257860 | 1 |
| PROST2005793 | 0.042726962 | 88935 | 10 |
| PROST2005880 | 0.005942124 | 247222 | 1 |
| PROST2005886 | 0.005942124 | 169519 | 1 |
| PROST2005904 | 0.005942124 | 258772 | 1 |
| PROST2005919 | 0.005942124 | 67219 | 1 |
| PROST2005943 | 0.005942124 | 247219 | 1 |
| PROST2006008 | 0.005942124 | 95067 | 1 |
| PROST2006020 | 0.005942124 | 264658 | 1 |
| PROST2006030 | 0.044283076 | 18674 | 20 |
| PROST2006060 | 0.025094982 | 90846 | 6 |
| PROST2006196 | 0.005942124 | 34444 | 1 |
| PROST2006201 | 0.005942124 | 263247 | 1 |
| PROST2006260 | 0.041148428 | 98945 | 15 |
| PROST2006282 | 0.018862625 | 147172 | 4 |
| PROST2006343 | 0.03789671 | 152612 | 5 |
| PROST2006450 | 0.005942124 | 253513 | 1 |
| PROST2006491 | 0.005942124 | 144216 | 1 |
| PROST2006510 | 0.005942124 | 35391 | 1 |
| PROST2006536 | 0.075507067 | 17279 | 19 |
| PROST2006579 | 0.005942124 | 58330 | 1 |
| PROST2006688 | 0.005942124 | 281706 | 1 |
| PROST2006737 | 0.069132358 | 95107 | 13 |
| PROST2006766 | 0.005942124 | 74271 | 1 |
| PROST2006782 | 0.005942124 | 257846 | 1 |
| PROST2006933 | 0.008856981 | 46348 | 2 |
| PROST2006988 | 0.011474484 | 178694 | 3 |
| PROST2007122 | 0.005942124 | 256014 | 1 |
| PROST2007200 | 0.014185061 | 177898 | 5 |
| PROST2007237 | 0.005942124 | 103378 | 1 |
| PROST2007248 | 0.008989483 | 166582 | 3 |
| PROST2007289 | 0.005942124 | 81524 | 1 |
| PROST2007317 | 0.007037402 | 117924 | 2 |
| PROST2007328 | 0.005942124 | 235208 | 1 |
| PROST2007382 | 0.005942124 | 269592 | 1 |
| PROST2007389 | 0.011884247 | 99275 | 2 |
| PROST2007416 | 0.011884247 | 173386 | 2 |
| PROST2007444 | 0.153231919 | 112210 | 57 |
| PROST2007528 | 0.005942124 | 162203 | 1 |
| PROST2007612 | 0.052470125 | 127075 | 12 |
| PROST2007818 | 0.005942124 | 241787 | 1 |
| PROST2007871 | 0.015191297 | 132816 | 4 |
| PROST2007950 | 0.005942124 | 143659 | 1 |
| PROST2007956 | 0.005942124 | 168227 | 1 |
| PROST2007974 | 0.005942124 | 158080 | 1 |
| PROST2007988 | 0.005942124 | 264140 | 1 |
| PROST2008079 | 0.005942124 | 13312 | 1 |
| PROST2008088 | 0.005942124 | 252945 | 1 |
| PROST2008243 | 0.005942124 | 131356 | 1 |
| PROST2008245 | 0.005942124 | 154052 | 1 |
| PROST2008268 | 0.00903762 | 170782 | 2 |
| PROST2008271 | 0.042754388 | 125879 | 19 |
| PROST2008360 | 0.093096573 | 120267 | 15 |
| PROST2008447 | 0.005942124 | 126269 | 1 |
| PROST2008468 | 0.005942124 | 250007 | 1 |
| PROST2008472 | 0.005942124 | 156943 | 1 |
| PROST2008489 | 0.258721502 | 59756 | 24 |
| PROST2008516 | 0.005942124 | 235806 | 1 |
| PROST2008724 | 0.005942124 | 236780 | 1 |
| PROST2008770 | 0.214802958 | 50355 | 66 |
| PROST2008835 | 0.005942124 | 68965 | 1 |
| PROST2008993 | 0.005942124 | 7528 | 1 |
| PROST2009022 | 0.005942124 | 264164 | 1 |
| PROST2009208 | 0.005942124 | 252939 | 1 |
| PROST2009222 | 0.005942124 | 256347 | 1 |
| PROST2009254 | 0.005942124 | 260282 | 1 |
| PROST2009273 | 0.005942124 | 62071 | 1 |
| PROST2009320 | 0.011854735 | 176817 | 2 |
| PROST2009347 | 0.008840828 | 167130 | 3 |
| PROST2009365 | 0.005942124 | 244082 | 1 |
| PROST2009388 | 0.005942124 | 113104 | 1 |
| PROST2009400 | 0.014050476 | 160196 | 4 |
| PROST2009470 | 0.005942124 | 254806 | 1 |
| PROST2009483 | 0.011884247 | 167995 | 2 |
| PROST2009571 | 0.005942124 | 262051 | 1 |
| PROST2009731 | 0.154504008 | 197671 | 3 |
| PROST2009736 | 0.005942124 | 36655 | 1 |
| PROST2009795 | 0.005942124 | 125611 | 1 |
| PROST2009901 | 0.005942124 | 164995 | 1 |
| PROST2009909 | 0.005942124 | 122661 | 1 |
| PROST2010046 | 0.029710619 | 142556 | 5 |
| PROST2010219 | 0.007818965 | 134050 | 2 |
| PROST2010250 | 0.005942124 | 173339 | 1 |
| PROST2010318 | 0.013882649 | 73588 | 3 |
| PROST2010326 | 0.005942124 | 93 | 1 |
| PROST2010382 | 0.053544083 | 181787 | 3 |
| PROST2010400 | 0.005942124 | 273838 | 1 |
| PROST2010545 | 0.012732224 | 201978 | 4 |
| PROST2010579 | 0.008914244 | 159160 | 3 |
| PROST2010606 | 0.005942124 | 17150 | 1 |
| PROST2010782 | 0.68901586 | 36006 | 21 |
| PROST2010885 | 0.005942124 | 116310 | 1 |
| PROST2011012 | 0.005942124 | 219780 | 1 |
| PROST2011038 | 0.013963143 | 69569 | 3 |
| PROST2011105 | 0.009415891 | 51105 | 4 |
| PROST2011297 | 0.005942124 | 110276 | 1 |
| PROST2011410 | 0.042256439 | 184207 | 3 |
| PROST2011439 | 0.005942124 | 196633 | 1 |
| PROST2011482 | 0.00903762 | 127791 | 2 |
| PROST2011577 | 0.162822998 | 105745 | 26 |
| PROST2011631 | 0.005942124 | 53421 | 1 |
| PROST2011660 | 0.007131368 | 219874 | 2 |
| PROST2012005 | 0.005942124 | 275100 | 1 |
| PROST2012007 | 0.147631125 | 114056 | 18 |
| PROST2012016 | 0.0076304 | 207999 | 2 |
| PROST2012088 | 0.005942124 | 125946 | 1 |
| PROST2012140 | 0.00906293 | 41975 | 2 |
| PROST2012157 | 0.005942124 | 148596 | 1 |
| PROST2012190 | 0.005942124 | 139665 | 1 |
| PROST2012249 | 0.005942124 | 259763 | 1 |
| PROST2012353 | 0.017825152 | 161319 | 3 |
| PROST2012400 | 0.005942124 | 210230 | 1 |
| PROST2012448 | 0.020536025 | 134964 | 9 |
| PROST2012504 | 0.007339279 | 15446 | 2 |
| PROST2012542 | 0.005942124 | 156960 | 1 |
| PROST2012550 | 0.005942124 | 70083 | 1 |
| PROST2012740 | 0.005942124 | 193803 | 1 |
| PROST2012745 | 0.043325874 | 9478 | 8 |
| PROST2012780 | 0.023032916 | 127933 | 3 |
| PROST2012888 | 0.009980407 | 176077 | 2 |
| PROST2012890 | 0.010985407 | 156961 | 3 |
| PROST2013032 | 0.005942124 | 131493 | 1 |
| PROST2013053 | 0.056571608 | 133025 | 4 |
| PROST2013121 | 0.015577881 | 11399 | 7 |
| PROST2013260 | 0.005942124 | 152122 | 1 |
| PROST2013327 | 0.005942124 | 89745 | 1 |
| PROST2013531 | 0.005942124 | 210251 | 1 |
| PROST2013605 | 0.005942124 | 242998 | 1 |
| PROST2013627 | 0.005942124 | 224677 | 1 |
| PROST2013873 | 0.005942124 | 191711 | 1 |
| PROST2013880 | 0.005942124 | 193328 | 1 |
| PROST2014422 | 0.005942124 | 159676 | 1 |
| PROST2014585 | 0.005942124 | 158165 | 1 |
| PROST2014601 | 0.005942124 | 133030 | 1 |
| PROST2014659 | 0.005942124 | 279819 | 1 |
| PROST2014862 | 0.010545257 | 83129 | 3 |
| PROST2014916 | 0.045992275 | 101313 | 3 |
| PROST2014925 | 0.011884247 | 276617 | 2 |
| PROST2015124 | 0.005942124 | 127654 | 1 |
| PROST2015137 | 0.009118914 | 19008 | 3 |
| PROST2015198 | 0.005942124 | 264123 | 1 |
| PROST2015243 | 0.005942124 | 278418 | 1 |
| PROST2015246 | 0.005942124 | 186494 | 1 |
| PROST2015251 | 0.007800238 | 74934 | 2 |
| PROST2015287 | 0.015608069 | 149479 | 4 |
| PROST2015332 | 0.065006855 | 116140 | 28 |
| PROST2015457 | 0.005942124 | 175300 | 1 |
| PROST2015464 | 0.097690609 | 124013 | 14 |
| PROST2015537 | 0.005942124 | 137696 | 1 |
| PROST2015545 | 0.005942124 | 191689 | 1 |
| PROST2015551 | 0.005942124 | 39570 | 1 |
| PROST2015636 | 0.005942124 | 196118 | 1 |
| PROST2015710 | 0.013605416 | 187293 | 3 |
| PROST2015756 | 0.005942124 | 156939 | 1 |
| PROST2015877 | 0.005942124 | 106718 | 1 |
| PROST2015900 | 0.007131368 | 162244 | 2 |
| PROST2015924 | 0.02472645 | 101372 | 6 |
| PROST2015932 | 0.005942124 | 237770 | 1 |
| PROST2016195 | 0.02865235 | 93703 | 4 |
| PROST2016246 | 0.005942124 | 256552 | 1 |
| PROST2016351 | 0.018917082 | 189305 | 3 |
| PROST2016379 | 0.005942124 | 223925 | 1 |
| PROST2016444 | 0.005942124 | 126609 | 1 |
| PROST2016462 | 0.005942124 | 153073 | 1 |
| PROST2016499 | 0.005942124 | 169652 | 1 |
| PROST2016512 | 0.005942124 | 90506 | 1 |
| PROST2016566 | 0.005942124 | 223036 | 1 |
| PROST2016582 | 0.017228805 | 59588 | 2 |
| PROST2016668 | 0.005942124 | 259973 | 1 |
| PROST2016684 | 0.005942124 | 130861 | 1 |
| PROST2016777 | 0.005942124 | 116474 | 1 |
| PROST2016829 | 0.005942124 | 188525 | 1 |
| PROST2016860 | 0.017826371 | 146566 | 3 |
| PROST2016918 | 0.026365287 | 109376 | 10 |
| PROST2016980 | 0.014799104 | 181108 | 3 |
| PROST2017098 | 0.005942124 | 188502 | 1 |
| PROST2017128 | 0.020793924 | 143705 | 4 |
| PROST2017203 | 0.129028512 | 75725 | 22 |
| PROST2017367 | 0.005942124 | 39562 | 1 |
| PROST2017413 | 0.005942124 | 196123 | 1 |
| PROST2017441 | 0.005942124 | 280815 | 1 |
| PROST2017529 | 0.066013596 | 73429 | 6 |
| PROST2017578 | 0.005942124 | 212267 | 1 |
| PROST2017612 | 0.007818965 | 30377 | 2 |
| PROST2017617 | 0.007940525 | 142022 | 2 |
| PROST2017692 | 0.227378107 | 56115 | 21 |
| PROST2017700 | 0.005942124 | 153278 | 1 |
| PROST2017701 | 0.005942124 | 146704 | 1 |
| PROST2017729 | 0.011854735 | 171611 | 2 |
| PROST2017836 | 0.005942124 | 18958 | 1 |
| PROST2017910 | 0.016034775 | 177825 | 3 |
| PROST2017972 | 0.013854029 | 151310 | 3 |
| PROST2018030 | 0.005942124 | 177929 | 1 |
| PROST2018090 | 0.077024597 | 77825 | 3 |
| PROST2018235 | 0.005942124 | 159943 | 1 |
| PROST2018437 | 0.005942124 | 284976 | 1 |
| PROST2018487 | 0.005942124 | 173469 | 1 |
| PROST2018511 | 0.005942124 | 69532 | 1 |
| PROST2018583 | 0.018129216 | 135109 | 3 |
| PROST2018588 | 0.012768167 | 64920 | 3 |
| PROST2018607 | 0.009980407 | 19012 | 2 |
| PROST2018788 | 0.005942124 | 233118 | 1 |
| PROST2018902 | 0.005942124 | 197268 | 1 |
| PROST2018922 | 0.005942124 | 144730 | 1 |
| PROST2018977 | 0.012894135 | 127478 | 4 |
| PROST2019164 | 0.045440065 | 112887 | 10 |
| PROST2019253 | 0.099728261 | 11949 | 32 |
| PROST2019296 | 0.029463234 | 179098 | 3 |
| PROST2019398 | 0.005942124 | 253328 | 1 |
| PROST2019487 | 0.005942124 | 220162 | 1 |
| PROST2019781 | 0.005942124 | 255810 | 1 |
| PUAEN1000039 | 0.009454477 | 67423 | 1 |
| PUAEN1000057 | 0.08161272 | 74496 | 24 |
| PUAEN1000065 | 0.250168757 | 9442 | 42 |
| PUAEN1000081 | 0.012575283 | 53305 | 2 |
| PUAEN1000085 | 0.009454477 | 128647 | 1 |
| PUAEN1000087 | 0.009454477 | 73847 | 1 |
| PUAEN1000121 | 0.009454477 | 21686 | 1 |
| PUAEN1000147 | 0.017969139 | 69830 | 6 |
| PUAEN1000161 | 0.02904803 | 43250 | 9 |
| PUAEN1000164 | 0.420960301 | 72471 | 40 |
| PUAEN1000175 | 0.009454477 | 43752 | 1 |
| PUAEN1000239 | 0.05404878 | 78338 | 14 |
| PUAEN1000275 | 0.009454477 | 281377 | 1 |
| PUAEN1000276 | 0.009454477 | 141955 | 1 |
| PUAEN1000288 | 0.02836343 | 36912 | 3 |
| PUAEN1000322 | 0.131066861 | 14558 | 13 |
| PUAEN2000080 | 0.009454477 | 141142 | 1 |
| PUAEN2000152 | 0.028232203 | 82523 | 8 |
| PUAEN2000175 | 0.04747729 | 103106 | 2 |
| PUAEN2000247 | 0.030327468 | 134137 | 8 |
| PUAEN2000312 | 0.018926049 | 92173 | 5 |
| PUAEN2000374 | 0.523525708 | 65148 | 65 |
| PUAEN2000394 | 0.009454477 | 120625 | 1 |
| PUAEN2000497 | 0.06499439 | 165430 | 5 |
| PUAEN2000535 | 0.208341386 | 87553 | 16 |
| PUAEN2000594 | 0.015667586 | 218383 | 2 |
| PUAEN2000684 | 0.018908953 | 87618 | 2 |
| PUAEN2000942 | 0.012549973 | 96188 | 2 |
| PUAEN2001188 | 0.048276743 | 117193 | 33 |
| PUAEN2001260 | 0.009454477 | 136511 | 1 |
| PUAEN2001345 | 0.057226273 | 103555 | 3 |
| PUAEN2001526 | 0.009454477 | 154880 | 1 |
| PUAEN2001586 | 0.058678854 | 67880 | 9 |
| PUAEN2001882 | 0.015259655 | 61560 | 2 |
| PUAEN2002473 | 0.009454477 | 223222 | 1 |
| PUAEN2002489 | 0.025679813 | 137028 | 9 |
| PUAEN2002552 | 0.009454477 | 245027 | 1 |
| PUAEN2002568 | 0.015367088 | 130005 | 2 |
| PUAEN2002616 | 0.009454477 | 204723 | 1 |
| PUAEN2002758 | 0.009454477 | 225331 | 1 |
| PUAEN2003018 | 0.04998634 | 130876 | 9 |
| PUAEN2003079 | 0.009454477 | 278188 | 1 |
| PUAEN2003116 | 0.009454477 | 172474 | 1 |
| PUAEN2003408 | 0.055636312 | 33679 | 8 |
| PUAEN2003517 | 0.009454477 | 135212 | 1 |
| PUAEN2003947 | 0.083379247 | 115298 | 15 |
| PUAEN2003954 | 0.015366739 | 169759 | 2 |
| PUAEN2004067 | 0.009454477 | 90043 | 1 |
| PUAEN2004083 | 0.009454477 | 112361 | 1 |
| PUAEN2004400 | 0.023257335 | 177058 | 4 |
| PUAEN2004511 | 0.037817907 | 175814 | 4 |
| PUAEN2004525 | 0.009454477 | 175807 | 1 |
| PUAEN2004875 | 0.009454477 | 88895 | 1 |
| PUAEN2005110 | 0.009454477 | 196908 | 1 |
| PUAEN2005247 | 0.009454477 | 133570 | 1 |
| PUAEN2005502 | 0.015655168 | 117317 | 5 |
| PUAEN2005588 | 0.009454477 | 105107 | 1 |
| PUAEN2005930 | 0.018249987 | 35647 | 6 |
| PUAEN2006029 | 0.009454477 | 109817 | 1 |
| PUAEN2006328 | 0.045438644 | 188717 | 2 |
| PUAEN2006335 | 0.013211536 | 28164 | 3 |
| PUAEN2006578 | 0.009454477 | 81690 | 1 |
| PUAEN2006639 | 0.009454477 | 214089 | 1 |
| PUAEN2006701 | 0.074487441 | 46150 | 11 |
| PUAEN2006723 | 0.117118444 | 51065 | 21 |
| PUAEN2006761 | 0.009454477 | 163250 | 1 |
| PUAEN2007044 | 0.116897286 | 113030 | 46 |
| PUAEN2007350 | 0.012499358 | 191416 | 2 |
| PUAEN2007785 | 0.009454477 | 107280 | 1 |
| PUAEN2007898 | 0.015508845 | 220203 | 2 |
| PUAEN2008123 | 0.02836343 | 153908 | 3 |
| PUAEN2008228 | 0.011452878 | 8315 | 2 |
| PUAEN2008314 | 0.015667586 | 51346 | 2 |
| PUAEN2008515 | 0.017258655 | 126473 | 6 |
| PUAEN2008815 | 0.01349276 | 164414 | 2 |
| PUAEN2008939 | 0.048822234 | 33700 | 7 |
| PUAEN2008980 | 0.009454477 | 65222 | 1 |
| PUAEN2008985 | 0.037762988 | 130790 | 13 |
| PUAEN2009174 | 0.05911391 | 158723 | 5 |
| PUAEN2009348 | 0.021217799 | 181211 | 2 |
| PUAEN2009534 | 0.009454477 | 101464 | 1 |
| PUAEN2009655 | 0.045843298 | 86624 | 10 |
| PUAEN2009795 | 0.009454477 | 116244 | 1 |
| PUAEN2009852 | 0.009454477 | 189853 | 1 |
| PUAEN2010346 | 0.016787407 | 30852 | 3 |
| PUAEN2010824 | 0.031983749 | 133594 | 15 |
| RECTM1000051 | 0.072595657 | 183914 | 4 |
| RECTM1000141 | 0.036456435 | 99349 | 1 |
| RECTM2000220 | 0.036456435 | 263986 | 1 |
| RECTM2000349 | 0.039551931 | 165918 | 2 |
| RECTM2000433 | 0.096439513 | 206849 | 4 |
| RECTM2000510 | 0.055590468 | 191599 | 3 |
| RECTM2001307 | 0.036456435 | 158680 | 1 |
| RECTM2001347 | 0.040396061 | 231729 | 3 |
| RECTM2001519 | 0.042398558 | 136175 | 2 |
| RECTM2001666 | 0.036456435 | 231640 | 1 |
| RECTM2001691 | 0.036456435 | 249519 | 1 |
| RECTM2001749 | 0.036456435 | 270399 | 1 |
| RECTM2002172 | 0.042658754 | 174941 | 2 |
| RECTM2002602 | 0.036456435 | 229358 | 1 |
| RECTM2002632 | 0.036456435 | 278074 | 1 |
| SALGL1000005 | 0.55252423 | 56519 | 3 |
| SALGL1000024 | 0.551367738 | 30407 | 2 |
| SALGL1000028 | 0.540540541 | 34449 | 1 |
| SALGL1000047 | 0.565613342 | 22067 | 13 |
| SALGL1000065 | 0.540540541 | 60866 | 1 |
| SALGL1000107 | 0.542731097 | 22721 | 3 |
| SALGL1000157 | 1.288535926 | 23950 | 76 |
| SALGL1000171 | 0.543334851 | 69545 | 3 |
| SKMUS1000014 | 0.022777922 | 44359 | 5 |
| SKMUS1000022 | 0.402562528 | 36477 | 33 |
| SKMUS1000030 | 0.011806375 | 51973 | 1 |
| SKMUS1000064 | 0.357021123 | 51790 | 32 |
| SKMUS1000067 | 0.011806375 | 61805 | 1 |
| SKMUS1000084 | 0.108797944 | 1627 | 27 |
| SKMUS1000102 | 0.017326006 | 72224 | 3 |
| SKMUS1000104 | 0.023612751 | 100625 | 2 |
| SKMUS1000110 | 0.022744331 | 62767 | 5 |
| SKMUS1000118 | 0.011806375 | 90271 | 1 |
| SKMUS1000124 | 0.011806375 | 36433 | 1 |
| SKMUS1000129 | 0.118063754 | 28908 | 10 |
| SKMUS1000138 | 1.744784769 | 78371 | 77 |
| SKMUS1000176 | 0.194213688 | 79808 | 4 |
| SKMUS1000177 | 0.070954434 | 27950 | 24 |
| SKMUS1000186 | 0.4174627 | 42726 | 50 |
| SKMUS2000020 | 0.045492571 | 174802 | 6 |
| SKMUS2000061 | 0.011806375 | 169612 | 1 |
| SKMUS2000074 | 0.032762615 | 94474 | 4 |
| SKMUS2000078 | 0.011806375 | 34531 | 1 |
| SKMUS2000117 | 0.019718858 | 184594 | 3 |
| SKMUS2000137 | 0.011806375 | 238540 | 1 |
| SKMUS2000198 | 0.106085694 | 153834 | 9 |
| SKMUS2000243 | 0.016609512 | 121970 | 3 |
| SKMUS2000271 | 0.026267996 | 170233 | 5 |
| SKMUS2000287 | 0.011806375 | 204543 | 1 |
| SKMUS2000317 | 0.011806375 | 106346 | 1 |
| SKMUS2000330 | 0.011806375 | 257597 | 1 |
| SKMUS2000339 | 0.011806375 | 274903 | 1 |
| SKMUS2000343 | 0.021746785 | 86654 | 7 |
| SKMUS2000361 | 0.156686231 | 118406 | 15 |
| SKMUS2000365 | 0.011806375 | 77154 | 1 |
| SKMUS2000380 | 0.077265157 | 56871 | 11 |
| SKMUS2000390 | 0.023612751 | 37149 | 2 |
| SKMUS2000416 | 0.011806375 | 147703 | 1 |
| SKMUS2000458 | 0.175319547 | 153833 | 15 |
| SKMUS2000467 | 0.014851257 | 175235 | 2 |
| SKMUS2000468 | 0.011806375 | 239334 | 1 |
| SKMUS2000484 | 0.095419817 | 152457 | 23 |
| SKMUS2000515 | 0.014788335 | 178432 | 2 |
| SKMUS2000648 | 0.382534307 | 44066 | 23 |
| SKMUS2000679 | 0.250105605 | 159248 | 20 |
| SKMUS2000690 | 0.03440014 | 190628 | 2 |
| SKMUS2000701 | 0.011806375 | 277491 | 1 |
| SKMUS2000705 | 0.011806375 | 266468 | 1 |
| SKMUS2000710 | 0.011806375 | 178160 | 1 |
| SKMUS2000724 | 0.011806375 | 119105 | 1 |
| SKMUS2000726 | 0.011806375 | 13136 | 1 |
| SKMUS2000757 | 0.033906374 | 118088 | 5 |
| SKMUS2000774 | 0.059031877 | 58750 | 5 |
| SKMUS2000780 | 0.017611554 | 107990 | 2 |
| SKMUS2000821 | 0.211093475 | 14972 | 16 |
| SKMUS2000847 | 0.011806375 | 276453 | 1 |
| SKMUS2000863 | 0.011806375 | 138727 | 1 |
| SKMUS2000873 | 0.30461113 | 53764 | 33 |
| SKMUS2000894 | 0.011806375 | 146381 | 1 |
| SKMUS2000898 | 0.011806375 | 173418 | 1 |
| SKMUS2000902 | 0.030979939 | 81845 | 6 |
| SKMUS2000931 | 0.307921717 | 4828 | 60 |
| SKMUS2000933 | 0.027005847 | 159968 | 4 |
| SKMUS2000945 | 0.023612751 | 234220 | 2 |
| SKMUS2000954 | 0.098584961 | 101802 | 13 |
| SKMUS2000961 | 0.025451383 | 91799 | 3 |
| SKMUS2000973 | 0.011806375 | 270204 | 1 |
| SKMUS2000980 | 0.011806375 | 275623 | 1 |
| SKMUS2001008 | 0.353604424 | 162422 | 31 |
| SKMUS2001014 | 0.011806375 | 272271 | 1 |
| SKMUS2001129 | 0.03294934 | 55098 | 7 |
| SKMUS2001147 | 0.060445442 | 122764 | 12 |
| SKMUS2001157 | 0.046705808 | 109083 | 4 |
| SKMUS2001164 | 0.011806375 | 193506 | 1 |
| SKMUS2001199 | 0.011806375 | 146323 | 1 |
| SKMUS2001201 | 0.094060505 | 178031 | 9 |
| SKMUS2001215 | 0.011806375 | 170024 | 1 |
| SKMUS2001247 | 0.011806375 | 272195 | 1 |
| SKMUS2001254 | 0.013494652 | 103250 | 2 |
| SKMUS2001271 | 0.011806375 | 265617 | 1 |
| SKMUS2001287 | 0.011806375 | 40044 | 1 |
| SKMUS2001323 | 0.095383062 | 54152 | 3 |
| SKMUS2001364 | 0.011806375 | 270303 | 1 |
| SKMUS2001398 | 0.011806375 | 201688 | 1 |
| SKMUS2001402 | 0.039745918 | 168538 | 5 |
| SKMUS2001412 | 0.013494652 | 109555 | 2 |
| SKMUS2001454 | 0.496448704 | 99423 | 80 |
| SKMUS2001492 | 0.011806375 | 131386 | 1 |
| SKMUS2001501 | 0.076873609 | 116061 | 10 |
| SKMUS2001528 | 0.133488985 | 131138 | 15 |
| SKMUS2001543 | 0.015374109 | 148340 | 4 |
| SKMUS2001580 | 0.011806375 | 276418 | 1 |
| SKMUS2001585 | 0.011806375 | 236373 | 1 |
| SKMUS2001608 | 0.011806375 | 110456 | 1 |
| SKMUS2001622 | 0.106085694 | 153834 | 9 |
| SKMUS2001631 | 0.011806375 | 231631 | 1 |
| SKMUS2001634 | 0.127981114 | 118943 | 27 |
| SKMUS2001662 | 0.011806375 | 185076 | 1 |
| SKMUS2001663 | 0.011806375 | 214394 | 1 |
| SKMUS2001668 | 0.339266725 | 173113 | 29 |
| SKMUS2001671 | 0.035419126 | 116067 | 3 |
| SKMUS2001683 | 0.011806375 | 141095 | 1 |
| SKMUS2001693 | 0.034899433 | 34319 | 3 |
| SKMUS2001695 | 0.185783845 | 109082 | 16 |
| SKMUS2001703 | 0.011806375 | 267090 | 1 |
| SKMUS2001740 | 0.011806375 | 40053 | 1 |
| SKMUS2001742 | 0.035419126 | 75473 | 3 |
| SKMUS2001823 | 0.011806375 | 253660 | 1 |
| SKMUS2001850 | 0.011806375 | 90275 | 1 |
| SKMUS2002077 | 0.023612751 | 159251 | 2 |
| SKMUS2002084 | 0.026835902 | 53859 | 4 |
| SKMUS2002153 | 0.011806375 | 49804 | 1 |
| SKMUS2002475 | 0.011806375 | 212934 | 1 |
| SKMUS2002602 | 0.011806375 | 116252 | 1 |
| SKMUS2002634 | 0.097794659 | 146131 | 9 |
| SKMUS2002693 | 0.011806375 | 248602 | 1 |
| SKMUS2002821 | 0.023612751 | 136885 | 2 |
| SKMUS2002840 | 0.011806375 | 212511 | 1 |
| SKMUS2002920 | 0.023612751 | 188014 | 2 |
| SKMUS2003034 | 0.023612751 | 181145 | 2 |
| SKMUS2003074 | 0.011806375 | 13142 | 1 |
| SKMUS2003168 | 0.017748499 | 205356 | 2 |
| SKMUS2003194 | 0.012901654 | 182700 | 2 |
| SKMUS2003744 | 0.011806375 | 240636 | 1 |
| SKMUS2004044 | 0.012901654 | 96617 | 2 |
| SKMUS2004047 | 0.011806375 | 175109 | 1 |
| SKMUS2004483 | 0.023612751 | 226782 | 2 |
| SKMUS2004667 | 0.017748499 | 142699 | 2 |
| SKMUS2004897 | 0.011806375 | 229383 | 1 |
| SKMUS2004903 | 0.023093057 | 109084 | 2 |
| SKMUS2004908 | 0.051011564 | 49066 | 20 |
| SKMUS2005428 | 0.011806375 | 175856 | 1 |
| SKMUS2006394 | 0.034790931 | 171345 | 3 |
| SKMUS2006481 | 0.011806375 | 203829 | 1 |
| SKMUS2006755 | 0.023093057 | 218561 | 2 |
| SKMUS2007315 | 0.011806375 | 166532 | 1 |
| SKMUS2007359 | 0.011806375 | 78060 | 1 |
| SKMUS2007568 | 0.011806375 | 209605 | 1 |
| SKMUS2007740 | 0.023612751 | 159250 | 2 |
| SKMUS2007816 | 0.011806375 | 7590 | 1 |
| SKMUS2007915 | 0.011806375 | 158009 | 1 |
| SKMUS2008338 | 0.011806375 | 167680 | 1 |
| SKMUS2008407 | 0.011806375 | 207212 | 1 |
| SKMUS2008474 | 0.011806375 | 13139 | 1 |
| SKMUS2008585 | 0.023612751 | 214395 | 2 |
| SKMUS2008607 | 0.011806375 | 188005 | 1 |
| SKMUS2009190 | 0.011806375 | 229207 | 1 |
| SKMUS2009232 | 0.011806375 | 187337 | 1 |
| SKMUS2009479 | 0.011806375 | 262641 | 1 |
| SKMUS2009557 | 0.011806375 | 215848 | 1 |
| SKNMC1000110 | 0.046280419 | 57453 | 8 |
| SKNMC1000123 | 0.013061651 | 69231 | 1 |
| SKNMC1000124 | 0.013061651 | 78246 | 1 |
| SKNMC1000137 | 0.158064039 | 54854 | 23 |
| SKNMC1000159 | 0.015866389 | 91566 | 3 |
| SKNMC1000168 | 0.200091079 | 62226 | 23 |
| SKNMC1000229 | 0.031872481 | 40682 | 7 |
| SKNMC1000251 | 0.023888065 | 70807 | 6 |
| SKNMC1000264 | 0.031945921 | 71841 | 5 |
| SKNMC2000065 | 0.155211407 | 81449 | 54 |
| SKNMC2000097 | 0.375933517 | 8504 | 37 |
| SKNMC2000224 | 0.026749288 | 133704 | 4 |
| SKNMC2000256 | 0.013061651 | 124132 | 1 |
| SKNMC2000305 | 0.047569604 | 83573 | 17 |
| SKNMC2000322 | 0.118647954 | 103785 | 14 |
| SKNMC2000356 | 0.018951458 | 146687 | 4 |
| SKNMC2000374 | 0.013061651 | 270474 | 1 |
| SKNMC2000583 | 0.013061651 | 267442 | 1 |
| SKNMC2000593 | 0.019207177 | 148705 | 2 |
| SKNMC2000612 | 0.039184953 | 167270 | 3 |
| SKNMC2000622 | 0.013061651 | 262601 | 1 |
| SKNMC2000635 | 0.934107208 | 77578 | 96 |
| SKNMC2000649 | 0.183657027 | 110797 | 32 |
| SKNMC2000698 | 0.081121106 | 159290 | 16 |
| SKNMC2000877 | 0.141402482 | 105490 | 31 |
| SKNMC2000966 | 0.020692051 | 52399 | 3 |
| SKNMC2001057 | 0.019047249 | 236266 | 4 |
| SKNMC2001113 | 0.017165752 | 32628 | 3 |
| SKNMC2001324 | 0.025947179 | 72405 | 4 |
| SKNMC2001503 | 0.054029779 | 132743 | 9 |
| SKNMC2001555 | 0.013061651 | 71150 | 1 |
| SKNMC2001596 | 0.013061651 | 87870 | 1 |
| SKNMC2001623 | 0.016675543 | 115479 | 2 |
| SKNMC2002402 | 0.022728256 | 105999 | 5 |
| SKNMC2003639 | 0.016182457 | 142789 | 2 |
| SKNMC2003924 | 0.013061651 | 213572 | 1 |
| SKNMC2003987 | 0.013061651 | 283391 | 1 |
| SKNMC2004457 | 0.013061651 | 234096 | 1 |
| SKNMC2004643 | 0.013061651 | 193536 | 1 |
| SKNMC2004651 | 0.013061651 | 225212 | 1 |
| SKNMC2005772 | 0.013061651 | 226517 | 1 |
| SKNMC2006173 | 0.021774346 | 167701 | 5 |
| SKNMC2006327 | 0.013061651 | 257564 | 1 |
| SKNMC2006998 | 0.013061651 | 197841 | 1 |
| SKNMC2007134 | 0.013061651 | 150488 | 1 |
| SKNMC2007502 | 0.013061651 | 175750 | 1 |
| SKNMC2007504 | 0.026123302 | 175749 | 2 |
| SKNMC2007961 | 0.013061651 | 154447 | 1 |
| SKNMC2009450 | 0.013061651 | 141873 | 1 |
| SKNSH1000002 | 0.116570504 | 74749 | 13 |
| SKNSH1000086 | 0.019623028 | 76655 | 4 |
| SKNSH1000101 | 0.260848272 | 63857 | 19 |
| SKNSH1000174 | 0.011504832 | 74273 | 1 |
| SKNSH1000301 | 0.011504832 | 73355 | 1 |
| SKNSH1000308 | 0.011504832 | 62403 | 1 |
| SKNSH1000416 | 0.011504832 | 67019 | 1 |
| SKNSH2000051 | 0.01983088 | 115662 | 5 |
| SKNSH2000101 | 0.025426757 | 80974 | 9 |
| SKNSH2000151 | 0.03079909 | 115194 | 9 |
| SKNSH2000163 | 0.015789573 | 145677 | 3 |
| SKNSH2000245 | 0.176626238 | 108761 | 31 |
| SKNSH2000250 | 0.118930558 | 75476 | 49 |
| SKNSH2000319 | 0.023364378 | 164426 | 3 |
| SKNSH2000347 | 0.074676678 | 125457 | 22 |
| SKNSH2000482 | 0.017189911 | 143153 | 4 |
| SKNSH2000499 | 0.045952233 | 160238 | 11 |
| SKNSH2000516 | 0.011504832 | 204000 | 1 |
| SKNSH2000550 | 0.011504832 | 259620 | 1 |
| SKNSH2000716 | 0.096677532 | 149478 | 13 |
| SKNSH2000819 | 0.067319187 | 124339 | 23 |
| SKNSH2000971 | 0.02330285 | 234478 | 2 |
| SKNSH2001222 | 0.025659598 | 158951 | 4 |
| SKNSH2001875 | 0.025803974 | 130530 | 4 |
| SKNSH2001931 | 0.032787618 | 68013 | 5 |
| SKNSH2002054 | 0.011504832 | 225472 | 1 |
| SKNSH2002325 | 0.043891577 | 127118 | 10 |
| SKNSH2002768 | 0.070767872 | 61033 | 14 |
| SKNSH2002866 | 0.011504832 | 274207 | 1 |
| SKNSH2003064 | 0.021403284 | 152142 | 3 |
| SKNSH2003174 | 0.011504832 | 160137 | 1 |
| SKNSH2003422 | 0.011504832 | 198245 | 1 |
| SKNSH2003466 | 0.011504832 | 240054 | 1 |
| SKNSH2003483 | 0.011504832 | 236918 | 1 |
| SKNSH2003528 | 0.055194048 | 131238 | 8 |
| SKNSH2003633 | 0.024980002 | 102593 | 3 |
| SKNSH2004039 | 0.011504832 | 96243 | 1 |
| SKNSH2005061 | 0.098840742 | 133269 | 12 |
| SKNSH2005120 | 0.011504832 | 217287 | 1 |
| SKNSH2005194 | 0.011504832 | 231893 | 1 |
| SKNSH2005240 | 0.01260011 | 98546 | 2 |
| SKNSH2005792 | 0.011504832 | 160331 | 1 |
| SKNSH2005816 | 0.011504832 | 219933 | 1 |
| SKNSH2005981 | 0.011504832 | 113222 | 1 |
| SKNSH2006234 | 0.047867269 | 199867 | 6 |
| SKNSH2006304 | 0.011504832 | 217391 | 1 |
| SKNSH2006432 | 0.011504832 | 163312 | 1 |
| SKNSH2006822 | 0.011504832 | 163652 | 1 |
| SKNSH2007142 | 0.011504832 | 74153 | 1 |
| SKNSH2007149 | 0.011504832 | 132810 | 1 |
| SKNSH2007429 | 0.011504832 | 78092 | 1 |
| SKNSH2007739 | 0.011504832 | 73330 | 1 |
| SKNSH2008043 | 0.016548115 | 162019 | 3 |
| SKNSH2008777 | 0.04267859 | 140574 | 9 |
| SKNSH2008940 | 0.087381065 | 47826 | 5 |
| SKNSH2008969 | 0.011504832 | 5599 | 1 |
| SKNSH2009197 | 0.011504832 | 49756 | 1 |
| SKNSH2009357 | 0.017539881 | 133546 | 3 |
| SKNSH2009435 | 0.011504832 | 126463 | 1 |
| SKNSH2009991 | 0.011504832 | 245728 | 1 |
| SKNSH2010015 | 0.011504832 | 242553 | 1 |
| SMINT1000012 | 0.005912612 | 17837 | 1 |
| SMINT1000016 | 0.068297746 | 22469 | 9 |
| SMINT1000039 | 0.01703732 | 58332 | 2 |
| SMINT1000042 | 0.062540386 | 15826 | 8 |
| SMINT1000048 | 0.021272859 | 60833 | 6 |
| SMINT1000051 | 0.005912612 | 57880 | 1 |
| SMINT1000054 | 0.008957493 | 62088 | 2 |
| SMINT1000057 | 0.009526503 | 67296 | 2 |
| SMINT1000071 | 0.005912612 | 53496 | 1 |
| SMINT1000075 | 0.005912612 | 37264 | 1 |
| SMINT1000078 | 0.017796859 | 39040 | 3 |
| SMINT1000091 | 0.005912612 | 68174 | 1 |
| SMINT1000100 | 0.06018931 | 168360 | 9 |
| SMINT1000103 | 0.005912612 | 60219 | 1 |
| SMINT1000117 | 0.127393401 | 39029 | 33 |
| SMINT1000118 | 0.005912612 | 40734 | 1 |
| SMINT1000131 | 0.182959568 | 58953 | 17 |
| SMINT1000137 | 0.008827469 | 53603 | 2 |
| SMINT1000178 | 0.070252924 | 41188 | 33 |
| SMINT1000192 | 0.056267369 | 74197 | 4 |
| SMINT2000007 | 0.027856852 | 103945 | 7 |
| SMINT2000018 | 0.039798556 | 109279 | 8 |
| SMINT2000032 | 0.005912612 | 238311 | 1 |
| SMINT2000040 | 0.005912612 | 116718 | 1 |
| SMINT2000060 | 0.005912612 | 263324 | 1 |
| SMINT2000073 | 0.019313987 | 17295 | 7 |
| SMINT2000084 | 0.005912612 | 266101 | 1 |
| SMINT2000145 | 0.005912612 | 171786 | 1 |
| SMINT2000159 | 0.030659106 | 91395 | 7 |
| SMINT2000176 | 0.011984994 | 189644 | 2 |
| SMINT2000185 | 0.005912612 | 37262 | 1 |
| SMINT2000227 | 0.005912612 | 267578 | 1 |
| SMINT2000229 | 0.005912612 | 152042 | 1 |
| SMINT2000232 | 0.011769768 | 276833 | 5 |
| SMINT2000239 | 0.019976545 | 125493 | 4 |
| SMINT2000267 | 0.007770726 | 171618 | 2 |
| SMINT2000277 | 0.092421108 | 121198 | 15 |
| SMINT2000396 | 0.136878017 | 38004 | 35 |
| SMINT2000400 | 0.025047457 | 29117 | 7 |
| SMINT2000441 | 0.371218566 | 37044 | 75 |
| SMINT2000454 | 0.018748294 | 58737 | 8 |
| SMINT2000468 | 0.073234454 | 104983 | 25 |
| SMINT2000518 | 0.005912612 | 268516 | 1 |
| SMINT2000529 | 0.005912612 | 277930 | 1 |
| SMINT2000530 | 0.005912612 | 123081 | 1 |
| SMINT2000537 | 0.007994124 | 110318 | 2 |
| SMINT2000538 | 0.005912612 | 141209 | 1 |
| SMINT2000541 | 0.005912612 | 154387 | 1 |
| SMINT2000545 | 0.070681319 | 115312 | 25 |
| SMINT2000558 | 0.005912612 | 277845 | 1 |
| SMINT2000562 | 0.005912612 | 267785 | 1 |
| SMINT2000568 | 0.005912612 | 63650 | 1 |
| SMINT2000602 | 0.304831045 | 145735 | 15 |
| SMINT2000609 | 0.008957493 | 206000 | 2 |
| SMINT2000629 | 0.062391264 | 165545 | 19 |
| SMINT2000659 | 0.603047235 | 155783 | 12 |
| SMINT2000694 | 0.140968152 | 10798 | 13 |
| SMINT2000747 | 0.010224624 | 157950 | 3 |
| SMINT2000811 | 0.005912612 | 155895 | 1 |
| SMINT2000824 | 0.005912612 | 193789 | 1 |
| SMINT2000933 | 0.164408747 | 150547 | 23 |
| SMINT2000984 | 0.005912612 | 225397 | 1 |
| SMINT2001114 | 0.005912612 | 97647 | 1 |
| SMINT2001158 | 0.005912612 | 178885 | 1 |
| SMINT2001183 | 0.018452584 | 84978 | 10 |
| SMINT2001195 | 0.019557654 | 170075 | 4 |
| SMINT2001199 | 0.01673981 | 102884 | 2 |
| SMINT2001222 | 0.04053865 | 235008 | 2 |
| SMINT2001348 | 0.018061496 | 116977 | 3 |
| SMINT2001397 | 0.005912612 | 156596 | 1 |
| SMINT2001458 . | 0.005912612 | 218819 | 1 |
| SMINT2001461 | 0.005912612 | 225393 | 1 |
| SMINT2001535 | 0.005912612 | 37025 | 1 |
| SMINT2001559 | 0.005912612 | 161956 | 1 |
| SMINT2001609 | 0.005912612 | 24138 | 1 |
| SMINT2001615 | 0.009950895 | 198580 | 2 |
| SMINT2001683 | 0.005912612 | 46266 | 1 |
| SMINT2001731 | 0.005912612 | 181803 | 1 |
| SMINT2001812 | 0.020952521 | 161226 | 6 |
| SMINT2001818 | 0.178744123 | 72429 | 25 |
| SMINT2001950 | 0.015681739 | 136164 | 4 |
| SMINT2002126 | 0.005912612 | 208461 | 1 |
| SMINT2002159 | 0.024437993 | 188276 | 7 |
| SMINT2002202 | 0.005912612 | 156781 | 1 |
| SMINT2002210 | 0.005912612 | 2710 | 1 |
| SMINT2002224 | 0.013235363 | 133499 | 4 |
| SMINT2002281 | 0.011825223 | 267085 | 2 |
| SMINT2002311 | 0.031632486 | 135087 | 4 |
| SMINT2002314 | 0.138443826 | 100201 | 5 |
| SMINT2002328 | 0.009033418 | 135665 | 2 |
| SMINT2002414 | 0.005912612 | 206389 | 1 |
| SMINT2002457 | 0.005912612 | 2004 | 1 |
| SMINT2002620 | 0.073956527 | 109567 | 14 |
| SMINT2002689 | 0.142537791 | 53760 | 70 |
| SMINT2002743 | 0.005912612 | 155091 | 1 |
| SMINT2002778 | 0.007911013 | 20725 | 2 |
| SMINT2002880 | 0.005912612 | 118847 | 1 |
| SMINT2002882 | 0.057704881 | 111997 | 6 |
| SMINT2002884 | 0.045362008 | 127520 | 5 |
| SMINT2002899 | 0.005912612 | 170518 | 1 |
| SMINT2002974 | 0.005912612 | 109566 | 1 |
| SMINT2002976 | 0.032122891 | 23340 | 10 |
| SMINT2003003 | 0.008499011 | 163285 | 3 |
| SMINT2003074 | 0.122234053 | 110951 | 18 |
| SMINT2003128 | 0.005912612 | 193729 | 1 |
| SMINT2003150 | 0.007600888 | 95155 | 2 |
| SMINT2003169 | 0.356225718 | 47147 | 32 |
| SMINT2003317 | 0.005912612 | 171672 | 1 |
| SMINT2003340 | 0.057028111 | 171523 | 10 |
| SMINT2003386 | 0.005912612 | 22706 | 1 |
| SMINT2003423 | 0.015422967 | 110636 | 4 |
| SMINT2003505 | 0.053208096 | 109528 | 6 |
| SMINT2003551 | 0.0120783 | 138784 | 3 |
| SMINT2003569 | 0.005912612 | 150465 | 1 |
| SMINT2003641 | 0.005912612 | 174946 | 1 |
| SMINT2003644 | 0.005912612 | 108635 | 1 |
| SMINT2003866 | 0.005912612 | 56087 | 1 |
| SMINT2003905 | 0.216430435 | 40242 | 17 |
| SMINT2004023 | 0.109863374 | 111780 | 12 |
| SMINT2004037 | 0.008706922 | 39999 | 3 |
| SMINT2004047 | 0.005912612 | 24136 | 1 |
| SMINT2004086 | 0.005912612 | 76378 | 1 |
| SMINT2004144 | 0.369158109 | 19335 | 28 |
| SMINT2004280 | 0.01315123 | 120324 | 3 |
| SMINT2004299 | 0.018565802 | 33018 | 3 |
| SMINT2004339 | 0.005912612 | 136056 | 1 |
| SMINT2004350 | 0.012822826 | 120443 | 5 |
| SMINT2004414 | 0.007101856 | 278430 | 2 |
| SMINT2004473 | 0.005912612 | 101188 | 1 |
| SMINT2004547 | 0.005912612 | 193857 | 1 |
| SMINT2004583 | 0.043251512 | 30859 | 3 |
| SMINT2004589 | 0.041807918 | 124054 | 8 |
| SMINT2004729 | 0.013046391 | 149767 | 3 |
| SMINT2004781 | 0.005912612 | 150061 | 1 |
| SMINT2004872 | 0.079227914 | 152894 | 13 |
| SMINT2004891 | 0.005912612 | 29655 | 1 |
| SMINT2004909 | 0.005912612 | 229946 | 1 |
| SMINT2004972 | 0.021153384 | 99943 | 4 |
| SMINT2004992 | 0.005912612 | 199179 | 1 |
| SMINT2005075 | 0.005912612 | 193818 | 1 |
| SMINT2005161 | 0.005912612 | 192782 | 1 |
| SMINT2005174 | 0.005912612 | 193822 | 1 |
| SMINT2005213 | 0.005912612 | 190263 | 1 |
| SMINT2005330 | 0.01214897 | 131141 | 2 |
| SMINT2005368 | 0.14267833 | 63013 | 23 |
| SMINT2005387 | 0.007309767 | 231605 | 2 |
| SMINT2005402 | 0.007101856 | 218712 | 2 |
| SMINT2005405 | 0.005912612 | 94636 | 1 |
| SMINT2005621 | 0.005912612 | 225457 | 1 |
| SMINT2005623 | 0.005912612 | 251094 | 1 |
| SMINT2005624 | 0.005912612 | 250468 | 1 |
| SMINT2005688 | 0.067136107 | 72659 | 4 |
| SMINT2005800 | 0.005912612 | 191500 | 1 |
| SMINT2005956 | 0.063660854 | 82665 | 16 |
| SMINT2006078 | 0.005912612 | 231524 | 1 |
| SMINT2006205 | 0.005912612 | 30139 | 1 |
| SMINT2006331 | 0.005912612 | 170540 | 1 |
| SMINT2006596 | 0.005912612 | 173405 | 1 |
| SMINT2006641 | 0.005912612 | 173404 | 1 |
| SMINT2006648 | 0.005912612 | 215659 | 1 |
| SMINT2006708 | 0.005912612 | 139327 | 1 |
| SMINT2006716 | 0.014266482 | 104517 | 3 |
| SMINT2006801 | 0.010403447 | 142243 | 4 |
| SMINT2007062 | 0.007309767 | 111631 | 2 |
| SMINT2007101 | 0.007770726 | 135209 | 2 |
| SMINT2007140 | 0.005912612 | 228416 | 1 |
| SMINT2007187 | 0.02495963 | 14651 | 12 |
| SMINT2007219 | 0.005912612 | 17331 | 1 |
| SMINT2007294 | 0.005912612 | 219336 | 1 |
| SMINT2007365 | 0.005912612 | 277043 | 1 |
| SMINT2007391 | 0.005912612 | 248625 | 1 |
| SMINT2007433 | 0.020072751 | 1859 | 3 |
| SMINT2007508 | 0.010089547 | 100062 | 2 |
| SMINT2007526 | 0.005912612 | 15681 | 1 |
| SMINT2007579 | 0.005912612 | 165882 | 1 |
| SMINT2007647 | 0.007600888 | 152098 | 2 |
| SMINT2007729 | 0.029137113 | 34646 | 3 |
| SMINT2007790 | 0.005912612 | 151702 | 1 |
| SMINT2007792 | 0.020046584 | 83583 | 7 |
| SMINT2007796 | 0.005912612 | 212374 | 1 |
| SMINT2007867 | 0.011825223 | 179140 | 2 |
| SMINT2007932 | 0.005912612 | 198829 | 1 |
| SMINT2008042 | 0.005912612 | 217919 | 1 |
| SMINT2008054 | 0.008827469 | 198849 | 2 |
| SMINT2008133 | 0.007101856 | 127106 | 2 |
| SMINT2008277 | 0.005912612 | 197715 | 1 |
| SMINT2008329 | 0.005912612 | 257596 | 1 |
| SMINT2008455 | 0.005912612 | 257326 | 1 |
| SMINT2008491 | 0.005912612 | 104836 | 1 |
| SMINT2008531 | 0.005912612 | 176585 | 1 |
| SMINT2008545 | 0.005912612 | 264501 | 1 |
| SMINT2008558 | 0.005912612 | 164289 | 1 |
| SMINT2008590 | 0.017503822 | 174353 | 5 |
| SMINT2008625 | 0.005912612 | 203608 | 1 |
| SMINT2008672 | 0.012329383 | 125455 | 4 |
| SMINT2008690 | 0.005912612 | 172953 | 1 |
| SMINT2008715 | 0.011825223 | 165750 | 2 |
| SMINT2008720 | 0.005912612 | 201584 | 1 |
| SMINT2008817 | 0.005912612 | 105932 | 1 |
| SMINT2008844 | 0.005912612 | 170519 | 1 |
| SMINT2008869 | 0.011848387 | 128699 | 2 |
| SMINT2008917 | 0.014975542 | 171903 | 3 |
| SMINT2008921 | 0.151013325 | 90844 | 29 |
| SMINT2008960 | 0.244910965 | 11616 | 48 |
| SMINT2009119 | 0.005912612 | 150539 | 1 |
| SMINT2009121 | 0.005912612 | 19047 | 1 |
| SMINT2009212 | 0.005912612 | 149059 | 1 |
| SMINT2009216 | 0.030270392 | 195760 | 3 |
| SMINT2009233 | 0.007101856 | 217329 | 2 |
| SMINT2009259 | 0.005912612 | 60722 | 1 |
| SMINT2009272 | 0.005912612 | 160077 | 1 |
| SMINT2009292 | 0.047238991 | 84423 | 13 |
| SMINT2009363 | 0.005912612 | 224982 | 1 |
| SMINT2009415 | 0.007770726 | 99367 | 2 |
| SMINT2009468 | 0.005912612 | 155509 | 1 |
| SMINT2009505 | 0.005912612 | 190276 | 1 |
| SMINT2009529 | 0.005912612 | 103334 | 1 |
| SMINT2009832 | 0.018134562 | 125287 | 2 |
| SMINT2009895 | 0.005912612 | 198739 | 1 |
| SMINT2009902 | 0.005912612 | 264243 | 1 |
| SMINT2009973 | 0.005912612 | 27731 | 1 |
| SMINT2010015 | 0.005912612 | 206890 | 1 |
| SMINT2010068 | 0.005912612 | 221084 | 1 |
| SMINT2010076 | 0.005912612 | 216266 | 1 |
| SMINT2010084 | 0.048764241 | 20276 | 17 |
| SMINT2010144 | 0.007770726 | 75951 | 2 |
| SMINT2010200 | 0.017675934 | 89421 | 2 |
| SMINT2010278 | 0.005912612 | 280019 | 1 |
| SMINT2010324 | 0.007770726 | 98989 | 2 |
| SMINT2010326 | 0.005912612 | 140434 | 1 |
| SMINT2010369 | 0.007309767 | 109629 | 2 |
| SMINT2010426 | 0.030930367 | 154940 | 4 |
| SMINT2010500 | 0.005912612 | 251222 | 1 |
| SMINT2010533 | 0.011825223 | 38234 | 2 |
| SMINT2010629 | 0.005912612 | 120571 | 1 |
| SMINT2010639 | 0.061855819 | 102851 | 16 |
| SMINT2010672 | 0.005912612 | 270068 | 1 |
| SMINT2010753 | 0.005912612 | 65422 | 1 |
| SMINT2010853 | 0.005912612 | 205384 | 1 |
| SMINT2010897 | 0.005912612 | 152978 | 1 |
| SMINT2010901 | 0.047710153 | 38030 | 15 |
| SMINT2010959 | 0.01082587 | 40139 | 3 |
| SMINT2010997 | 0.005912612 | 148881 | 1 |
| SMINT2011033 | 0.007911013 | 59485 | 2 |
| SMINT2011066 | 0.005912612 | 98590 | 1 |
| SMINT2011273 | 0.005912612 | 271792 | 1 |
| SMINT2011311 | 0.008894571 | 159443 | 2 |
| SMINT2011406 | 0.005912612 | 171677 | 1 |
| SMINT2011509 | 0.005912612 | 203511 | 1 |
| SMINT2011567 | 0.017659914 | 23370 | 3 |
| SMINT2011588 | 0.009008108 | 142752 | 2 |
| SMINT2011656 | 0.007101856 | 159045 | 2 |
| SMINT2011888 | 0.005912612 | 176544 | 1 |
| SMINT2011958 | 0.005912612 | 135257 | 1 |
| SMINT2012040 | 0.005912612 | 127782 | 1 |
| SMINT2012122 | 0.047929182 | 105364 | 8 |
| SMINT2012179 | 0.009019227 | 31383 | 3 |
| SMINT2012195 | 0.007101856 | 135106 | 2 |
| SMINT2012285 | 0.005912612 | 228859 | 1 |
| SMINT2012291 | 0.036335971 | 162430 | 7 |
| SMINT2012501 | 0.021070903 | 12012 | 4 |
| SMINT2012734 | 0.005912612 | 128168 | 1 |
| SMINT2012735 | 0.020403493 | 124137 | 6 |
| SMINT2012793 | 0.005912612 | 272964 | 1 |
| SMINT2013031 | 0.053988011 | 64266 | 11 |
| SMINT2013032 | 0.014009091 | 108496 | 5 |
| SMINT2013129 | 0.005912612 | 66878 | 1 |
| SMINT2013170 | 0.015367088 | 162361 | 2 |
| SMINT2013181 | 0.007600888 | 197608 | 2 |
| SMINT2013228 | 0.005912612 | 170584 | 1 |
| SMINT2013613 | 0.005912612 | 171620 | 1 |
| SMINT2013626 | 0.005912612 | 176932 | 1 |
| SMINT2013695 | 0.007911013 | 18815 | 2 |
| SMINT2013833 | 0.005912612 | 132916 | 1 |
| SMINT2013890 | 0.01826134 | 149571 | 2 |
| SMINT2014166 | 0.005912612 | 158643 | 1 |
| SMINT2014282 | 0.005912612 | 92111 | 1 |
| SMINT2014443 | 0.005912612 | 261523 | 1 |
| SMINT2014480 | 0.105466368 | 57024 | 9 |
| SMINT2014489 | 0.005912612 | 185303 | 1 |
| SMINT2014721 | 0.005912612 | 124025 | 1 |
| SMINT2015006 | 0.005912612 | 139093 | 1 |
| SMINT2015294 | 0.021045914 | 49120 | 3 |
| SMINT2015306 | 0.01673981 | 164156 | 2 |
| SMINT2015326 | 0.005912612 | 49657 | 1 |
| SMINT2015353 | 0.005912612 | 164665 | 1 |
| SMINT2015454 | 0.005912612 | 128688 | 1 |
| SMINT2015518 | 0.005912612 | 36060 | 1 |
| SMINT2015745 | 0.005912612 | 196534 | 1 |
| SMINT2015787 | 0.005912612 | 166126 | 1 |
| SMINT2015810 | 0.005912612 | 154291 | 1 |
| SMINT2016122 | 0.005912612 | 112818 | 1 |
| SMINT2016146 | 0.009756696 | 93871 | 2 |
| SMINT2016286 | 0.005912612 | 175455 | 1 |
| SMINT2016313 | 0.005912612 | 74997 | 1 |
| SMINT2016396 | 0.011848387 | 174416 | 2 |
| SMINT2016412 | 0.088785792 | 131719 | 5 |
| SMINT2016440 | 0.005912612 | 101551 | 1 |
| SMINT2016477 | 0.005912612 | 66508 | 1 |
| SMINT2016544 | 0.005912612 | 48161 | 1 |
| SMINT2016857 | 0.005912612 | 181701 | 1 |
| SMINT2017134 | 0.007770726 | 187006 | 2 |
| SMINT2017319 | 0.005912612 | 182395 | 1 |
| SMINT2017324 | 0.005912612 | 178724 | 1 |
| SMINT2017410 | 0.005912612 | 214871 | 1 |
| SMINT2017431 | 0.005912612 | 187053 | 1 |
| SMINT2017432 | 0.005912612 | 3662 | 1 |
| SMINT2017436 | 0.005912612 | 149041 | 1 |
| SMINT2017599 | 0.040804447 | 158637 | 2 |
| SMINT2017659 | 0.005912612 | 160958 | 1 |
| SMINT2017736 | 0.005912612 | 3387 | 1 |
| SMINT2017781 | 0.005912612 | 256905 | 1 |
| SMINT2017855 | 0.005912612 | 166000 | 1 |
| SMINT2017884 | 0.007101856 | 176133 | 2 |
| SMINT2017964 | 0.034567508 | 90724 | 11 |
| SMINT2017974 | 0.005912612 | 263817 | 1 |
| SMINT2018343 | 0.005912612 | 22523 | 1 |
| SMINT2018353 | 0.017090792 | 36226 | 2 |
| SMINT2018592 | 0.005912612 | 124836 | 1 |
| SMINT2018681 | 0.040067287 | 11655 | 4 |
| SMINT2019017 | 0.005912612 | 270969 | 1 |
| SMINT2019105 | 0.005912612 | 27835 | 1 |
| SMINT2019142 | 0.011825223 | 122385 | 2 |
| SMINT2019153 | 0.005912612 | 175937 | 1 |
| SMINT2019200 | 0.005912612 | 171894 | 1 |
| SPLEN1000013 | 0.017218776 | 15244 | 3 |
| SPLEN1000041 | 0.004312012 | 74650 | 2 |
| SPLEN1000049 | 0.0076859 | 5000 | 3 |
| SPLEN1000056 | 0.004312012 | 74840 | 2 |
| SPLEN1000083 | 0.333649462 | 50383 | 87 |
| SPLEN1000087 | 0.002914857 | 63304 | 1 |
| SPLEN1000089 | 0.168663194 | 2591 | 12 |
| SPLEN1000091 | 0.002914857 | 72285 | 1 |
| SPLEN1000106 | 0.07198732 | 48973 | 32 |
| SPLEN1000116 | 0.338556398 | 73895 | 28 |
| SPLEN1000134 | 0.004791699 | 52904 | 2 |
| SPLEN1000139 | 0.005896816 | 13957 | 2 |
| SPLEN1000143 | 0.002914857 | 2277 | 1 |
| SPLEN1000144 | 0.032052177 | 59490 | 6 |
| SPLEN1000145 | 0.002914857 | 38633 | 1 |
| SPLEN1000156 | 0.002914857 | 79820 | 1 |
| SPLEN1000163 | 0.002914857 | 62664 | 1 |
| SPLEN1000165 | 0.03357555 | 59801 | 10 |
| SPLEN1000166 | 0.148223938 | 23861 | 43 |
| SPLEN1000178 | 0.002914857 | 242569 | 1 |
| SPLEN2000020 | 0.002914857 | 183944 | 1 |
| SPLEN2000047 | 0.010153475 | 177455 | 3 |
| SPLEN2000064 | 0.002914857 | 219170 | 1 |
| SPLEN2000072 | 0.027900558 | 170459 | 5 |
| SPLEN2000098 | 0.013910565 | 144282 | 8 |
| SPLEN2000126 | 0.002914857 | 213785 | 1 |
| SPLEN2000134 | 0.031982906 | 164686 | 7 |
| SPLEN2000143 | 0.007226869 | 101274 | 3 |
| SPLEN2000189 | 0.008744571 | 138974 | 3 |
| SPLEN2000197 | 0.004772971 | 12071 | 2 |
| SPLEN2000205 | 0.002914857 | 216742 | 1 |
| SPLEN2000210 | 0.012623595 | 164407 | 2 |
| SPLEN2000222 | 0.013717889 | 124147 | 4 |
| SPLEN2000242 | 0.017439185 | 141048 | 2 |
| SPLEN2000243 | 0.007707569 | 158484 | 4 |
| SPLEN2000247 | 0.002914857 | 146692 | 1 |
| SPLEN2000255 | 0.109103073 | 123372 | 34 |
| SPLEN2000258 | 0.038273399 | 71707 | 7 |
| SPLEN2000267 | 0.028700894 | 11452 | 15 |
| SPLEN2000270 | 0.002914857 | 115139 | 1 |
| SPLEN2000304 | 0.407966855 | 38528 | 119 |
| SPLEN2000307 | 0.002914857 | 219122 | 1 |
| SPLEN2000310 | 0.012623595 | 129027 | 2 |
| SPLEN2000341 | 0.01237386 | 67931 | 4 |
| SPLEN2000348 | 0.05989707 | 146825 | 12 |
| SPLEN2000350 | 0.002914857 | 128966 | 1 |
| SPLEN2000357 | 0.002914857 | 93707 | 1 |
| SPLEN2000364 | 0.002914857 | 285317 | 1 |
| SPLEN2000402 | 0.002914857 | 219110 | 1 |
| SPLEN2000414 | 0.012623595 | 127298 | 2 |
| SPLEN2000420 | 0.002914857 | 234978 | 1 |
| SPLEN2000443 | 0.007585093 | 185474 | 3 |
| SPLEN2000448 | 0.002914857 | 144581 | 1 |
| SPLEN2000450 | 0.002914857 | 136802 | 1 |
| SPLEN2000464 | 0.002914857 | 197263 | 1 |
| SPLEN2000496 | 0.002914857 | 197266 | 1 |
| SPLEN2000505 | 0.397595113 | 50221 | 57 |
| SPLEN2000515 | 0.002914857 | 215909 | 1 |
| SPLEN2000516 | 0.014017552 | 146888 | 6 |
| SPLEN2000537 | 0.004590404 | 134432 | 2 |
| SPLEN2000541 | 0.002914857 | 219141 | 1 |
| SPLEN2000607 | 0.002914857 | 125819 | 1 |
| SPLEN2000630 | 0.002914857 | 121423 | 1 |
| SPLEN2000695 | 0.002914857 | 136446 | 1 |
| SPLEN2000698 | 0.079018445 | 63416 | 8 |
| SPLEN2000839 | 0.018357265 | 97021 | 4 |
| SPLEN2000874 | 0.343764444 | 104953 | 13 |
| SPLEN2000882 | 0.095383222 | 127390 | 21 |
| SPLEN2001135 | 0.002914857 | 218565 | 1 |
| SPLEN2001141 | 0.177436641 | 59612 | 51 |
| SPLEN2001157 | 0.060917825 | 27211 | 16 |
| SPLEN2001176 | 0.002914857 | 81078 | 1 |
| SPLEN2001227 | 0.002914857 | 197240 | 1 |
| SPLEN2001245 | 0.149973681 | 23990 | 2 |
| SPLEN2001278 | 0.004312012 | 34574 | 2 |
| SPLEN2001354 | 0.002914857 | 124005 | 1 |
| SPLEN2001367 | 0.002914857 | 47344 | 1 |
| SPLEN2001383 | 0.002914857 | 124432 | 1 |
| SPLEN2001476 | 0.004312012 | 88714 | 2 |
| SPLEN2001503 | 0.016656912 | 146686 | 3 |
| SPLEN2001510 | 0.002914857 | 193264 | 1 |
| SPLEN2001599 | 0.023481819 | 119291 | 5 |
| SPLEN2001626 | 0.008956423 | 155295 | 2 |
| SPLEN2001650 | 0.002914857 | 154524 | 1 |
| SPLEN2001689 | 0.01200505 | 63113 | 4 |
| SPLEN2001710 | 0.043211036 | 43332 | 4 |
| SPLEN2001761 | 0.015746765 | 41068 | 5 |
| SPLEN2001781 | 0.002914857 | 178412 | 1 |
| SPLEN2001881 | 0.002914857 | 178421 | 1 |
| SPLEN2001912 | 0.002914857 | 126730 | 1 |
| SPLEN2001945 | 0.002914857 | 65178 | 1 |
| SPLEN2002007 | 0.008780069 | 115624 | 3 |
| SPLEN2002053 | 0.004590404 | 188235 | 2 |
| SPLEN2002133 | 0.004010135 | 165891 | 2 |
| SPLEN2002147 | 0.002914857 | 171784 | 1 |
| SPLEN2002166 | 0.002914857 | 106920 | 1 |
| SPLEN2002175 | 0.002914857 | 188139 | 1 |
| SPLEN2002223 | 0.004791699 | 231695 | 2 |
| SPLEN2002272 | 0.008956423 | 157958 | 2 |
| SPLEN2002314 | 0.002914857 | 253205 | 1 |
| SPLEN2002335 | 0.165480591 | 90306 | 44 |
| SPLEN2002343 | 0.002914857 | 203944 | 1 |
| SPLEN2002354 | 0.002914857 | 108707 | 1 |
| SPLEN2002385 | 0.002914857 | 58410 | 1 |
| SPLEN2002419 | 0.002914857 | 33084 | 1 |
| SPLEN2002451 | 0.004312012 | 146903 | 2 |
| SPLEN2002462 | 0.002914857 | 191105 | 1 |
| SPLEN2002463 | 0.022126491 | 89565 | 13 |
| SPLEN2002467 | 0.051432664 | 185854 | 10 |
| SPLEN2002477 | 0.103114815 | 109807 | 21 |
| SPLEN2002493 | 0.09680775 | 97695 | 51 |
| SPLEN2002662 | 0.002914857 | 45526 | 1 |
| SPLEN2002695 | 0.002914857 | 90699 | 1 |
| SPLEN2002707 | 0.002914857 | 245667 | 1 |
| SPLEN2002744 | 0.075126312 | 91738 | 8 |
| SPLEN2002917 | 0.002914857 | 221413 | 1 |
| SPLEN2002931 | 0.041086754 | 179234 | 3 |
| SPLEN2002957 | 0.005829714 | 178408 | 2 |
| SPLEN2003160 | 0.002914857 | 249846 | 1 |
| SPLEN2003204 | 0.002914857 | 198617 | 1 |
| SPLEN2003219 | 0.002914857 | 183601 | 1 |
| SPLEN2003297 | 0.006310414 | 159270 | 3 |
| SPLEN2003396 | 0.008956423 | 208265 | 2 |
| SPLEN2003678 | 0.004772971 | 234930 | 2 |
| SPLEN2003878 | 0.002914857 | 204112 | 1 |
| SPLEN2003918 | 0.002914857 | 101303 | 1 |
| SPLEN2003924 | 0.002914857 | 9892 | 1 |
| SPLEN2004060 | 0.002914857 | 180659 | 1 |
| SPLEN2004078 | 0.011212672 | 47273 | 4 |
| SPLEN2004124 | 0.002914857 | 22072 | 1 |
| SPLEN2004148 | 0.002914857 | 210540 | 1 |
| SPLEN2004181 | 0.021284736 | 157722 | 4 |
| SPLEN2004220 | 0.058259113 | 113792 | 7 |
| SPLEN2004343 | 0.00931331 | 115882 | 5 |
| SPLEN2004346 | 0.002914857 | 231171 | 1 |
| SPLEN2004368 | 0.002914857 | 147648 | 1 |
| SPLEN2004555 | 0.157417742 | 86802 | 39 |
| SPLEN2004611 | 0.004312012 | 113563 | 2 |
| SPLEN2004662 | 0.0088958 | 173909 | 4 |
| SPLEN2004773 | 0.235431317 | 1655 | 47 |
| SPLEN2004844 | 0.014675412 | 256461 | 2 |
| SPLEN2004880 | 0.568857651 | 31712 | 7 |
| SPLEN2004984 | 0.063790519 | 119307 | 12 |
| SPLEN2005009 | 0.019490293 | 133889 | 8 |
| SPLEN2005142 | 0.157417742 | 86802 | 39 |
| SPLEN2005227 | 0.002914857 | 125072 | 1 |
| SPLEN2005272 | 0.002914857 | 77774 | 1 |
| SPLEN2005342 | 0.031408064 | 92906 | 10 |
| SPLEN2005416 | 0.004791699 | 115677 | 2 |
| SPLEN2005429 | 0.045734728 | 96931 | 19 |
| SPLEN2005450 | 0.002914857 | 102480 | 1 |
| SPLEN2005474 | 0.002914857 | 43252 | 1 |
| SPLEN2005560 | 0.159183453 | 122274 | 5 |
| SPLEN2005727 | 0.002914857 | 227937 | 1 |
| SPLEN2005767 | 0.043916771 | 115981 | 10 |
| SPLEN2005783 | 0.025703173 | 62449 | 11 |
| SPLEN2005790 | 0.004312012 | 8060 | 2 |
| SPLEN2005806 | 0.274005729 | 74118 | 12 |
| SPLEN2005818 | 0.015032227 | 153072 | 7 |
| SPLEN2005838 | 0.004624317 | 140602 | 2 |
| SPLEN2005869 | 0.026090564 | 183059 | 3 |
| SPLEN2005927 | 0.038909785 | 140379 | 6 |
| SPLEN2005939 | 0.002914857 | 234216 | 1 |
| SPLEN2006122 | 0.198950286 | 52473 | 14 |
| SPLEN2006133 | 0.176626238 | 108761 | 31 |
| SPLEN2006143 | 0.074889021 | 89002 | 14 |
| SPLEN2006232 | 0.058412074 | 129454 | 12 |
| SPLEN2006268 | 0.002914857 | 266784 | 1 |
| SPLEN2006283 | 0.002914857 | 43327 | 1 |
| SPLEN2006305 | 0.265258593 | 111292 | 78 |
| SPLEN2006325 | 0.020111948 | 140126 | 3 |
| SPLEN2006374 | 0.110236555 | 92380 | 37 |
| SPLEN2006389 | 0.002914857 | 47968 | 1 |
| SPLEN2006425 | 0.002914857 | 263011 | 1 |
| SPLEN2006671 | 0.008969225 | 102925 | 2 |
| SPLEN2006701 | 0.002914857 | 124791 | 1 |
| SPLEN2006785 | 0.002914857 | 115943 | 1 |
| SPLEN2006875 | 0.002914857 | 122324 | 1 |
| SPLEN2007182 | 0.013109569 | 127097 | 4 |
| SPLEN2007276 | 0.063179234 | 112688 | 7 |
| SPLEN2007314 | 0.002914857 | 280002 | 1 |
| SPLEN2007326 | 0.004312012 | 17406 | 2 |
| SPLEN2007350 | 0.002914857 | 109816 | 1 |
| SPLEN2007388 | 0.006310414 | 73426 | 3 |
| SPLEN2007498 | 0.064024495 | 110806 | 23 |
| SPLEN2007619 | 0.002914857 | 74708 | 1 |
| SPLEN2007653 | 0.002914857 | 117250 | 1 |
| SPLEN2007689 | 0.002914857 | 116899 | 1 |
| SPLEN2007695 | 0.002914857 | 71541 | 1 |
| SPLEN2007739 | 0.027594397 | 34940 | 15 |
| SPLEN2007750 | 0.034632523 | 159116 | 5 |
| SPLEN2007794 | 0.016906471 | 139570 | 3 |
| SPLEN2007879 | 0.011621779 | 10093 | 4 |
| SPLEN2007926 | 0.002914857 | 51442 | 1 |
| SPLEN2007951 | 0.007091792 | 117347 | 2 |
| SPLEN2008007 | 0.002914857 | 13206 | 1 |
| SPLEN2008135 | 0.002914857 | 164479 | 1 |
| SPLEN2008164 | 0.002914857 | 132782 | 1 |
| SPLEN2008317 | 0.002914857 | 207510 | 1 |
| SPLEN2008460 | 0.127900964 | 117911 | 17 |
| SPLEN2008496 | 0.021641601 | 28078 | 3 |
| SPLEN2008591 | 0.004010135 | 48605 | 2 |
| SPLEN2008737 | 0.087450229 | 54463 | 31 |
| SPLEN2008786 | 0.002914857 | 72070 | 1 |
| SPLEN2009081 | 0.025888668 | 107596 | 5 |
| SPLEN2009088 | 0.002914857 | 92525 | 1 |
| SPLEN2009163 | 0.009117177 | 124897 | 2 |
| SPLEN2009315 | 0.004913258 | 222877 | 2 |
| SPLEN2009340 | 0.002914857 | 73613 | 1 |
| SPLEN2009512 | 0.010117191 | 141564 | 3 |
| SPLEN2009513 | 0.02702602 | 105252 | 4 |
| SPLEN2009541 | 0.002914857 | 35663 | 1 |
| SPLEN2009548 | 0.002914857 | 103115 | 1 |
| SPLEN2009555 | 0.019726366 | 97430 | 6 |
| SPLEN2009581 | 0.002914857 | 143353 | 1 |
| SPLEN2009733 | 0.002914857 | 135216 | 1 |
| SPLEN2009803 | 0.002914857 | 41978 | 1 |
| SPLEN2009884 | 0.002914857 | 154849 | 1 |
| SPLEN2009918 | 0.002914857 | 56743 | 1 |
| SPLEN2009970 | 0.032855635 | 142120 | 11 |
| SPLEN2010004 | 0.002914857 | 118013 | 1 |
| SPLEN2010015 | 0.010778292 | 122423 | 6 |
| SPLEN2010112 | 0.002914857 | 115375 | 1 |
| SPLEN2010119 | 0.002914857 | 54489 | 1 |
| SPLEN2010146 | 0.002914857 | 141650 | 1 |
| SPLEN2010187 | 0.002914857 | 115448 | 1 |
| SPLEN2010195 | 0.43188623 | 103198 | 98 |
| SPLEN2010350 | 0.002914857 | 113494 | 1 |
| SPLEN2010395 | 0.002914857 | 263561 | 1 |
| SPLEN2010415 | 0.002914857 | 122061 | 1 |
| SPLEN2010447 | 0.002914857 | 283351 | 1 |
| SPLEN2010469 | 0.006758941 | 14538 | 2 |
| SPLEN2010510 | 0.002914857 | 120657 | 1 |
| SPLEN2010530 | 0.002914857 | 139438 | 1 |
| SPLEN2010534 | 0.002914857 | 141408 | 1 |
| SPLEN2010588 | 0.002914857 | 169428 | 1 |
| SPLEN2010625 | 0.004312012 | 153873 | 2 |
| SPLEN2010800 | 0.002914857 | 42885 | 1 |
| SPLEN2010846 | 0.007293972 | 53766 | 3 |
| SPLEN2010847 | 0.01015847 | 51801 | 3 |
| SPLEN2010862 | 0.002914857 | 257653 | 1 |
| SPLEN2010912 | 0.013528832 | 121273 | 8 |
| SPLEN2010978 | 0.002914857 | 132321 | 1 |
| SPLEN2010986 | 0.008320186 | 50421 | 4 |
| SPLEN2011018 | 0.002914857 | 8437 | 1 |
| SPLEN2011021 | 0.010224624 | 57866 | 3 |
| SPLEN2011025 | 0.008827469 | 145110 | 2 |
| SPLEN2011086 | 0.002914857 | 129479 | 1 |
| SPLEN2011139 | 0.002914857 | 94065 | 1 |
| SPLEN2011145 | 0.002914857 | 135972 | 1 |
| SPLEN2011248 | 0.002914857 | 125718 | 1 |
| SPLEN2011252 | 0.002914857 | 246970 | 1 |
| SPLEN2011419 | 0.070119935 | 21021 | 12 |
| SPLEN2011422 | 0.002914857 | 138473 | 1 |
| SPLEN2011672 | 0.002914857 | 85403 | 1 |
| SPLEN2011678 | 0.147020482 | 115451 | 3 |
| SPLEN2011737 | 0.064312131 | 93659 | 24 |
| SPLEN2011758 | 0.031902823 | 65392 | 7 |
| SPLEN2011766 | 0.010254136 | 110461 | 3 |
| SPLEN2011805 | 0.002914857 | 38362 | 1 |
| SPLEN2011820 | 0.002914857 | 115381 | 1 |
| SPLEN2011830 | 0.005829714 | 110459 | 2 |
| SPLEN2011931 | 0.002914857 | 120907 | 1 |
| SPLEN2011981 | 0.002914857 | 114150 | 1 |
| SPLEN2012115 | 0.040937671 | 198255 | 2 |
| SPLEN2012175 | 0.002914857 | 32655 | 1 |
| SPLEN2012179 | 0.002914857 | 186778 | 1 |
| SPLEN2012185 | 0.002914857 | 45540 | 1 |
| SPLEN2012234 | 0.002914857 | 112345 | 1 |
| SPLEN2012453 | 0.004104101 | 142770 | 2 |
| SPLEN2012523 | 0.156930511 | 54625 | 9 |
| SPLEN2012524 | 0.002914857 | 39246 | 1 |
| SPLEN2012525 | 0.002914857 | 56713 | 1 |
| SPLEN2012571 | 0.052707355 | 137693 | 13 |
| SPLEN2012606 | 0.002914857 | 57108 | 1 |
| SPLEN2012611 | 0.011671177 | 118055 | 3 |
| SPLEN2012619 | 0.004590404 | 107825 | 2 |
| SPLEN2012624 | 0.033137229 | 120091 | 12 |
| SPLEN2012725 | 0.002914857 | 196965 | 1 |
| SPLEN2012743 | 0.002914857 | 58862 | 1 |
| SPLEN2012756 | 0.002914857 | 138971 | 1 |
| SPLEN2012800 | 0.490568327 | 127014 | 14 |
| SPLEN2012846 | 0.006188854 | 9927 | 3 |
| SPLEN2012889 | 0.004312012 | 72891 | 2 |
| SPLEN2012961 | 0.002914857 | 281990 | 1 |
| SPLEN2013108 | 0.004791699 | 125465 | 2 |
| SPLEN2013195 | 0.004104101 | 23933 | 2 |
| SPLEN2013503 | 0.066757206 | 147838 | 8 |
| SPLEN2013644 | 0.002914857 | 61762 | 1 |
| SPLEN2013648 | 0.002914857 | 67298 | 1 |
| SPLEN2013670 | 0.014289976 | 132837 | 6 |
| SPLEN2013673 | 0.002914857 | 113244 | 1 |
| SPLEN2013690 | 0.015515464 | 119263 | 3 |
| SPLEN2013714 | 0.002914857 | 73207 | 1 |
| SPLEN2013753 | 0.004312012 | 125588 | 2 |
| SPLEN2013821 | 0.022476672 | 112139 | 2 |
| SPLEN2013857 | 0.004590404 | 37671 | 2 |
| SPLEN2013860 | 0.002914857 | 39794 | 1 |
| SPLEN2013910 | 0.002914857 | 270087 | 1 |
| SPLEN2013923 | 0.006188854 | 85699 | 3 |
| SPLEN2013936 | 0.025112439 | 139079 | 5 |
| SPLEN2014041 | 0.004312012 | 150722 | 2 |
| SPLEN2014063 | 0.002914857 | 56758 | 1 |
| SPLEN2014073 | 0.014201539 | 35718 | 2 |
| SPLEN2014080 | 0.035364657 | 148087 | 9 |
| SPLEN2014119 | 0.010039876 | 148011 | 3 |
| SPLEN2014136 | 0.005829714 | 160302 | 2 |
| SPLEN2014199 | 0.002914857 | 134877 | 1 |
| SPLEN2014210 | 0.002914857 | 133008 | 1 |
| SPLEN2014293 | 0.002914857 | 124023 | 1 |
| SPLEN2014318 | 0.005829714 | 161544 | 2 |
| SPLEN2014381 | 0.011268727 | 106130 | 3 |
| SPLEN2014452 | 0.009117177 | 129789 | 2 |
| SPLEN2014454 | 0.002914857 | 129788 | 1 |
| SPLEN2014486 | 0.002914857 | 146475 | 1 |
| SPLEN2014543 | 0.002914857 | 138117 | 1 |
| SPLEN2014572 | 0.026923245 | 35256 | 6 |
| SPLEN2014645 | 0.009151215 | 145380 | 2 |
| SPLEN2014669 | 0.005829714 | 117142 | 2 |
| SPLEN2014711 | 0.063175608 | 111782 | 4 |
| SPLEN2014739 | 0.011538882 | 140868 | 5 |
| SPLEN2014860 | 0.005829714 | 113220 | 2 |
| SPLEN2014865 | 0.002914857 | 129901 | 1 |
| SPLEN2014911 | 0.158823404 | 97253 | 28 |
| SPLEN2014919 | 0.002914857 | 179198 | 1 |
| SPLEN2014920 | 0.002914857 | 59356 | 1 |
| SPLEN2014924 | 0.002914857 | 54173 | 1 |
| SPLEN2014937 | 0.002914857 | 147868 | 1 |
| SPLEN2014946 | 0.002914857 | 134758 | 1 |
| SPLEN2015031 | 0.002914857 | 51951 | 1 |
| SPLEN2015094 | 0.002914857 | 67845 | 1 |
| SPLEN2015121 | 0.004312012 | 122862 | 2 |
| SPLEN2015129 | 0.004590404 | 5661 | 2 |
| SPLEN2015158 | 0.002914857 | 150635 | 1 |
| SPLEN2015166 | 0.008827469 | 48798 | 2 |
| SPLEN2015216 | 0.002914857 | 101211 | 1 |
| SPLEN2015258 | 0.002914857 | 125238 | 1 |
| SPLEN2015261 | 0.002914857 | 134163 | 1 |
| SPLEN2015267 | 0.002914857 | 283801 | 1 |
| SPLEN2015276 | 0.002914857 | 142847 | 1 |
| SPLEN2015310 | 0.292333103 | 48032 | 31 |
| SPLEN2015415 | 0.002914857 | 126941 | 1 |
| SPLEN2015433 | 0.002914857 | 113232 | 1 |
| SPLEN2015593 | 0.002914857 | 113200 | 1 |
| SPLEN2015674 | 0.002914857 | 109737 | 1 |
| SPLEN2015679 | 0.002914857 | 17417 | 1 |
| SPLEN2015707 | 0.002914857 | 135155 | 1 |
| SPLEN2015730 | 0.01197524 | 146144 | 3 |
| SPLEN2015746 | 0.002914857 | 281810 | 1 |
| SPLEN2015788 | 0.034613688 | 115925 | 11 |
| SPLEN2015815 | 0.002914857 | 156260 | 1 |
| SPLEN2015890 | 0.004104101 | 72495 | 2 |
| SPLEN2015891 | 0.002914857 | 53226 | 1 |
| SPLEN2015899 | 0.002914857 | 99000 | 1 |
| SPLEN2016045 | 0.002914857 | 165479 | 1 |
| SPLEN2016053 | 0.002914857 | 273281 | 1 |
| SPLEN2016069 | 0.002914857 | 269710 | 1 |
| SPLEN2016098 | 0.009117177 | 100865 | 2 |
| SPLEN2016135 | 0.002914857 | 118167 | 1 |
| SPLEN2016139 | 0.130771767 | 99113 | 9 |
| SPLEN2016241 | 0.002914857 | 164252 | 1 |
| SPLEN2016250 | 0.002914857 | 12746 | 1 |
| SPLEN2016268 | 0.387829361 | 78383 | 48 |
| SPLEN2016304 | 0.002914857 | 110558 | 1 |
| SPLEN2016356 | 0.002914857 | 138736 | 1 |
| SPLEN2016421 | 0.080880372 | 49655 | 17 |
| SPLEN2016480 | 0.002914857 | 138399 | 1 |
| SPLEN2016531 | 0.002914857 | 271544 | 1 |
| SPLEN2016533 | 0.004772971 | 17471 | 2 |
| SPLEN2016541 | 0.002914857 | 217915 | 1 |
| SPLEN2016552 | 0.002914857 | 79500 | 1 |
| SPLEN2016554 | 0.008990496 | 79138 | 4 |
| SPLEN2016602 | 0.002914857 | 169370 | 1 |
| SPLEN2016627 | 0.002914857 | 151306 | 1 |
| SPLEN2016678 | 0.008503479 | 71061 | 5 |
| SPLEN2016743 | 0.029223877 | 72231 | 6 |
| SPLEN2016773 | 0.004104101 | 110853 | 2 |
| SPLEN2016781 | 0.004312012 | 138933 | 2 |
| SPLEN2016840 | 0.002914857 | 242181 | 1 |
| SPLEN2016863 | 0.002914857 | 275752 | 1 |
| SPLEN2016922 | 0.002914857 | 107663 | 1 |
| SPLEN2016932 | 0.004772971 | 153348 | 2 |
| SPLEN2016972 | 0.082631374 | 113807 | 13 |
| SPLEN2016973 | 0.002914857 | 17865 | 1 |
| SPLEN2017031 | 0.138538996 | 109565 | 24 |
| SPLEN2017043 | 0.016098905 | 119417 | 3 |
| SPLEN2017104 | 0.002914857 | 253301 | 1 |
| SPLEN2017121 | 0.021611965 | 135937 | 6 |
| SPLEN2017147 | 0.002914857 | 110292 | 1 |
| SPLEN2017187 | 0.002914857 | 115736 | 1 |
| SPLEN2017189 | 0.002914857 | 115047 | 1 |
| SPLEN2017212 | 0.002914857 | 162530 | 1 |
| SPLEN2017318 | 0.033021481 | 31715 | 16 |
| SPLEN2017351 | 0.055706502 | 135186 | 24 |
| SPLEN2017426 | 0.002914857 | 130108 | 1 |
| SPLEN2017452 | 0.002914857 | 176395 | 1 |
| SPLEN2017538 | 0.002914857 | 6188 | 1 |
| SPLEN2017592 | 0.004104101 | 109937 | 2 |
| SPLEN2017613 | 0.002914857 | 39284 | 1 |
| SPLEN2017620 | 0.032689027 | 60349 | 6 |
| SPLEN2017643 | 0.002914857 | 138421 | 1 |
| SPLEN2017687 | 0.002914857 | 270715 | 1 |
| SPLEN2017740 | 0.002914857 | 83086 | 1 |
| SPLEN2017781 | 0.004104101 | 30939 | 2 |
| SPLEN2017918 | 0.183776349 | 28073 | 21 |
| SPLEN2017981 | 0.051769295 | 91638 | 26 |
| SPLEN2017999 | 0.002914857 | 154787 | 1 |
| SPLEN2018098 | 0.004913258 | 47527 | 2 |
| SPLEN2018112 | 0.002914857 | 134989 | 1 |
| SPLEN2018157 | 0.069466112 | 97654 | 21 |
| SPLEN2018159 | 0.017692446 | 30104 | 4 |
| SPLEN2018180 | 0.002914857 | 36841 | 1 |
| SPLEN2018181 | 0.002914857 | 145736 | 1 |
| SPLEN2018280 | 0.002914857 | 267675 | 1 |
| SPLEN2018285 | 0.002914857 | 86331 | 1 |
| SPLEN2018299 | 0.008142793 | 129049 | 4 |
| SPLEN2018302 | 0.027555401 | 60772 | 4 |
| SPLEN2018364 | 0.002914857 | 154737 | 1 |
| SPLEN2018395 | 0.002914857 | 187543 | 1 |
| SPLEN2018643 | 0.004590404 | 30252 | 2 |
| SPLEN2018683 | 0.004312012 | 82417 | 2 |
| SPLEN2018732 | 0.002914857 | 104525 | 1 |
| SPLEN2018749 | 0.002914857 | 107273 | 1 |
| SPLEN2018933 | 0.002914857 | 186700 | 1 |
| SPLEN2019008 | 0.002914857 | 1122 | 1 |
| SPLEN2019015 | 0.257097629 | 111157 | 31 |
| SPLEN2019043 | 0.002914857 | 62255 | 1 |
| SPLEN2019077 | 0.03158465 | 192819 | 5 |
| SPLEN2019092 | 0.006035663 | 191999 | 2 |
| SPLEN2019102 | 0.002914857 | 178887 | 1 |
| SPLEN2019131 | 0.002914857 | 187019 | 1 |
| SPLEN2019169 | 0.029596594 | 130371 | 18 |
| SPLEN2019257 | 0.004104101 | 29121 | 2 |
| SPLEN2019311 | 0.00691166 | 150786 | 3 |
| SPLEN2019323 | 0.002914857 | 153382 | 1 |
| SPLEN2019349 | 0.004624317 | 206596 | 2 |
| SPLEN2019375 | 0.002914857 | 114599 | 1 |
| SPLEN2019379 | 0.014675412 | 42169 | 2 |
| SPLEN2019405 | 0.117003473 | 35419 | 33 |
| SPLEN2019446 | 0.016102262 | 136509 | 4 |
| SPLEN2019471 | 0.002914857 | 235419 | 1 |
| SPLEN2019480 | 0.002914857 | 184785 | 1 |
| SPLEN2019505 | 0.034178208 | 1156 | 5 |
| SPLEN2019571 | 0.002914857 | 94465 | 1 |
| SPLEN2019575 | 0.002914857 | 71649 | 1 |
| SPLEN2019709 | 0.002914857 | 219496 | 1 |
| SPLEN2019774 | 0.018859953 | 261630 | 3 |
| SPLEN2019793 | 0.002914857 | 130329 | 1 |
| SPLEN2019811 | 0.002914857 | 264766 | 1 |
| SPLEN2019839 | 0.002914857 | 138179 | 1 |
| SPLEN2019956 | 0.005501257 | 17638 | 3 |
| SPLEN2019985 | 0.002914857 | 247445 | 1 |
| SPLEN2020007 | 0.008969225 | 222123 | 2 |
| SPLEN2020034 | 0.006758941 | 106987 | 2 |
| SPLEN2020153 | 0.002914857 | 251920 | 1 |
| SPLEN2020154 | 0.002914857 | 39800 | 1 |
| SPLEN2020166 | 0.002914857 | 240411 | 1 |
| SPLEN2020183 | 0.002914857 | 38398 | 1 |
| SPLEN2020223 | 0.002914857 | 17760 | 1 |
| SPLEN2020359 | 0.002914857 | 149164 | 1 |
| SPLEN2020417 | 0.012003782 | 107233 | 4 |
| SPLEN2020427 | 0.002914857 | 211084 | 1 |
| SPLEN2020467 | 0.002914857 | 245115 | 1 |
| SPLEN2020512 | 0.002914857 | 37024 | 1 |
| SPLEN2020608 | 0.002914857 | 49071 | 1 |
| SPLEN2020777 | 0.002914857 | 32229 | 1 |
| SPLEN2020805 | 0.005829714 | 223978 | 2 |
| SPLEN2021057 | 0.002914857 | 34461 | 1 |
| SPLEN2021122 | 0.010273404 | 88683 | 5 |
| SPLEN2021157 | 0.002914857 | 243715 | 1 |
| SPLEN2021194 | 0.04967068 | 112526 | 12 |
| SPLEN2021231 | 0.002914857 | 229123 | 1 |
| SPLEN2021273 | 0.011361777 | 69503 | 5 |
| SPLEN2021295 | 0.002914857 | 99177 | 1 |
| SPLEN2021298 | 0.002914857 | 49036 | 1 |
| SPLEN2021383 | 0.002914857 | 147669 | 1 |
| SPLEN2021417 | 0.002914857 | 203080 | 1 |
| SPLEN2021440 | 0.002914857 | 178070 | 1 |
| SPLEN2021458 | 0.004772971 | 125487 | 2 |
| SPLEN2021463 | 0.002914857 | 136359 | 1 |
| SPLEN2021560 | 0.002914857 | 278460 | 1 |
| SPLEN2021701 | 0.002914857 | 254978 | 1 |
| SPLEN2021795 | 0.002914857 | 144403 | 1 |
| SPLEN2021983 | 0.002914857 | 223308 | 1 |
| SPLEN2021991 | 0.002914857 | 223312 | 1 |
| SPLEN2022040 | 0.002914857 | 212597 | 1 |
| SPLEN2022162 | 0.005829714 | 140706 | 2 |
| SPLEN2022227 | 0.002914857 | 70966 | 1 |
| SPLEN2022522 | 0.118200644 | 70961 | 13 |
| SPLEN2022591 | 0.002914857 | 237801 | 1 |
| SPLEN2022785 | 0.002914857 | 280669 | 1 |
| SPLEN2022920 | 0.002914857 | 226358 | 1 |
| SPLEN2023024 | 0.002914857 | 167543 | 1 |
| SPLEN2023118 | 0.015052952 | 50841 | 3 |
| SPLEN2023160 | 0.008524334 | 165524 | 5 |
| SPLEN2023423 | 0.002914857 | 164527 | 1 |
| SPLEN2023706 | 0.015521841 | 43100 | 5 |
| SPLEN2023710 | 0.002914857 | 154177 | 1 |
| SPLEN2023733 | 0.008322148 | 136308 | 4 |
| SPLEN2023791 | 0.005896816 | 232557 | 2 |
| SPLEN2023977 | 0.002914857 | 6565 | 1 |
| SPLEN2024070 | 0.002914857 | 105000 | 1 |
| SPLEN2024127 | 0.002914857 | 218242 | 1 |
| SPLEN2024232 | 0.025261465 | 146441 | 3 |
| SPLEN2024273 | 0.002914857 | 248214 | 1 |
| SPLEN2024383 | 0.002914857 | 100265 | 1 |
| SPLEN2024489 | 0.015335638 | 35425 | 6 |
| SPLEN2024530 | 0.002914857 | 32001 | 1 |
| SPLEN2024571 | 0.002914857 | 135218 | 1 |
| SPLEN2024577 | 0.002914857 | 173096 | 1 |
| SPLEN2024617 | 0.002914857 | 136055 | 1 |
| SPLEN2024737 | 0.002914857 | 162799 | 1 |
| SPLEN2024922 | 0.006501159 | 143618 | 3 |
| SPLEN2024956 | 0.002914857 | 209783 | 1 |
| SPLEN2024990 | 0.007226869 | 59340 | 3 |
| SPLEN2025012 | 0.002914857 | 243040 | 1 |
| SPLEN2025017 | 0.004996369 | 220204 | 2 |
| SPLEN2025039 | 0.004104101 | 45184 | 2 |
| SPLEN2025219 | 0.002914857 | 169452 | 1 |
| SPLEN2025491 | 0.004772971 | 199432 | 2 |
| SPLEN2025626 | 0.002914857 | 244518 | 1 |
| SPLEN2025820 | 0.002914857 | 229023 | 1 |
| SPLEN2026144 | 0.002914857 | 139012 | 1 |
| SPLEN2026411 | 0.01470175 | 144177 | 2 |
| SPLEN2026765 | 0.002914857 | 178984 | 1 |
| SPLEN2027113 | 0.102397727 | 132649 | 21 |
| SPLEN2027157 | 0.002914857 | 40822 | 1 |
| SPLEN2027268 | 0.008281036 | 72901 | 3 |
| SPLEN2027395 | 0.002914857 | 46679 | 1 |
| SPLEN2027488 | 0.002914857 | 28869 | 1 |
| SPLEN2027780 | 0.006035663 | 28390 | 2 |
| SPLEN2027852 | 0.002914857 | 102885 | 1 |
| SPLEN2027868 | 0.002914857 | 187751 | 1 |
| SPLEN2027995 | 0.002914857 | 23054 | 1 |
| SPLEN2028046 | 0.002914857 | 180839 | 1 |
| SPLEN2028066 | 0.002914857 | 141052 | 1 |
| SPLEN2028138 | 0.002914857 | 279635 | 1 |
| SPLEN2028365 | 0.048828539 | 239696 | 2 |
| SPLEN2028417 | 0.002914857 | 121117 | 1 |
| SPLEN2028424 | 0.006010353 | 119839 | 2 |
| SPLEN2028466 | 0.405050633 | 50829 | 79 |
| SPLEN2028593 | 0.002914857 | 215986 | 1 |
| SPLEN2028844 | 0.002914857 | 169538 | 1 |
| SPLEN2028914 | 0.060004578 | 56160 | 12 |
| SPLEN2028994 | 0.002914857 | 65100 | 1 |
| SPLEN2029051 | 0.002914857 | 224759 | 1 |
| SPLEN2029176 | 0.002914857 | 200061 | 1 |
| SPLEN2029295 | 0.004791699 | 189924 | 2 |
| SPLEN2029380 | 0.002914857 | 113901 | 1 |
| SPLEN2029522 | 0.002914857 | 180805 | 1 |
| SPLEN2029683 | 0.004791699 | 156689 | 2 |
| SPLEN2029727 | 0.005829714 | 210646 | 2 |
| SPLEN2029904 | 0.005829714 | 236507 | 2 |
| SPLEN2029912 | 0.102573432 | 134449 | 11 |
| SPLEN2030250 | 0.002914857 | 199513 | 1 |
| SPLEN2030335 | 0.002914857 | 74114 | 1 |
| SPLEN2030371 | 0.002914857 | 176014 | 1 |
| SPLEN2030397 | 0.002914857 | 243151 | 1 |
| SPLEN2030479 | 0.002914857 | 235231 | 1 |
| SPLEN2030495 | 0.002914857 | 97300 | 1 |
| SPLEN2030562 | 0.002914857 | 124194 | 1 |
| SPLEN2030736 | 0.009170813 | 106930 | 4 |
| SPLEN2030763. | 0.015910477 | 23741 | 3 |
| SPLEN2030847 | 0.002914857 | 149643 | 1 |
| SPLEN2031004 | 0.002914857 | 210166 | 1 |
| SPLEN2031125 | 0.002914857 | 243143 | 1 |
| SPLEN2031424 | 0.022004007 | 42729 | 7 |
| SPLEN2031547 | 0.106422675 | 86211 | 46 |
| SPLEN2031674 | 0.007567282 | 38472 | 4 |
| SPLEN2031724 | 0.002914857 | 139903 | 1 |
| SPLEN2031780 | 0.002914857 | 75151 | 1 |
| SPLEN2032036 | 0.002914857 | 233320 | 1 |
| SPLEN2032112 | 0.002914857 | 30137 | 1 |
| SPLEN2032154 | 0.004772971 | 30714 | 2 |
| SPLEN2032157 | 0.029322393 | 138209 | 5 |
| SPLEN2032321 | 0.002914857 | 168234 | 1 |
| SPLEN2032356 | 0.008720035 | 201270 | 2 |
| SPLEN2032677 | 0.005829714 | 149572 | 2 |
| SPLEN2032813 | 0.002914857 | 207006 | 1 |
| SPLEN2032924 | 0.002914857 | 264674 | 1 |
| SPLEN2033098 | 0.033671568 | 121865 | 10 |
| SPLEN2033153 | 0.002914857 | 238918 | 1 |
| SPLEN2033333 | 0.002914857 | 184481 | 1 |
| SPLEN2033490 | 0.002914857 | 163929 | 1 |
| SPLEN2033539 | 0.002914857 | 276312 | 1 |
| SPLEN2033921 | 0.002914857 | 254031 | 1 |
| SPLEN2033996 | 0.005829714 | 139283 | 2 |
| SPLEN2034021 | 0.002914857 | 198083 | 1 |
| SPLEN2034081 | 0.002914857 | 66499 | 1 |
| SPLEN2034252 | 0.002914857 | 168153 | 1 |
| SPLEN2034448 | 0.032316465 | 73005 | 12 |
| SPLEN2034551 | 0.002914857 | 266745 | 1 |
| SPLEN2034601 | 0.002914857 | 283931 | 1 |
| SPLEN2034678 | 0.002914857 | 222395 | 1 |
| SPLEN2034781 | 0.005987559 | 187945 | 3 |
| SPLEN2034864 | 0.002914857 | 220768 | 1 |
| SPLEN2034934 | 0.002914857 | 73802 | 1 |
| SPLEN2035318 | 0.002914857 | 75172 | 1 |
| SPLEN2035615 | 0.002914857 | 252961 | 1 |
| SPLEN2036076 | 0.101992709 | 98489 | 25 |
| SPLEN2036103 | 0.002914857 | 241156 | 1 |
| SPLEN2036326 | 0.002914857 | 216122 | 1 |
| SPLEN2036475 | 0.002914857 | 135081 | 1 |
| SPLEN2036501 | 0.004312012 | 153304 | 2 |
| SPLEN2036579 | 0.002914857 | 200967 | 1 |
| SPLEN2036608 | 0.002914857 | 198676 | 1 |
| SPLEN2036702 | 0.005829714 | 175948 | 2 |
| SPLEN2036712 | 0.002914857 | 163537 | 1 |
| SPLEN2036821 | 0.004772971 | 195486 | 2 |
| SPLEN2036932 | 0.057151022 | 92903 | 16 |
| SPLEN2036953 | 0.002914857 | 253831 | 1 |
| SPLEN2037077 | 0.005829714 | 235817 | 2 |
| SPLEN2037194 | 0.004104101 | 192686 | 2 |
| SPLEN2037319 | 0.002914857 | 254821 | 1 |
| SPLEN2037444 | 0.002914857 | 251799 | 1 |
| SPLEN2037580 | 0.002914857 | 141311 | 1 |
| SPLEN2037630 | 0.002914857 | 251678 | 1 |
| SPLEN2037678 | 0.002914857 | 89396 | 1 |
| SPLEN2037722 | 0.10169338 | 103496 | 11 |
| SPLEN2037891 | 0.002914857 | 232681 | 1 |
| SPLEN2038055 | 0.002914857 | 153266 | 1 |
| SPLEN2038180 | 0.002914857 | 228882 | 1 |
| SPLEN2038345 | 0.005698412 | 127873 | 3 |
| SPLEN2038407 | 0.002914857 | 131919 | 1 |
| SPLEN2038473 | 0.012181189 | 221740 | 3 |
| SPLEN2038834 | 0.036169319 | 115808 | 6 |
| SPLEN2039311 | 0.002914857 | 224424 | 1 |
| SPLEN2039379 | 0.057855456 | 70977 | 29 |
| SPLEN2039672 | 0.004312012 | 263345 | 2 |
| SPLEN2039697 | 0.002914857 | 228251 | 1 |
| SPLEN2039845 | 0.005829714 | 53204 | 2 |
| SPLEN2039936 | 0.002914857 | 162533 | 1 |
| SPLEN2040222 | 0.002914857 | 232621 | 1 |
| SPLEN2040237 | 0.004104101 | 234149 | 2 |
| SPLEN2040824 | 0.008124066 | 121828 | 4 |
| SPLEN2041304 | 0.002914857 | 232842 | 1 |
| SPLEN2041306 | 0.021755328 | 125816 | 10 |
| SPLEN2041310 | 0.002914857 | 105698 | 1 |
| SPLEN2041585 | 0.004913258 | 227340 | 2 |
| SPLEN2041645 | 0.002914857 | 256976 | 1 |
| SPLEN2041700 | 0.002914857 | 249112 | 1 |
| SPLEN2041720 | 0.002914857 | 221325 | 1 |
| SPLEN2041977 | 0.002914857 | 250662 | 1 |
| SPLEN2042051 | 0.002914857 | 182559 | 1 |
| SPLEN2042303 | 0.006393524 | 139498 | 3 |
| SPLEN2042521 | 0.002914857 | 169976 | 1 |
| SPLEN2042535 | 0.023534289 | 184674 | 8 |
| SPLEN2042598 | 0.002914857 | 256318 | 1 |
| SPLEN2042714 | 0.004913258 | 145619 | 2 |
| SPLEN2042920 | 0.061571038 | 84778 | 3 |
| STOMA1000013 | 0.011402509 | 31529 | 1 |
| STOMA1000047 | 0.052878615 | 53485 | 12 |
| STOMA1000052 | 0.011402509 | 49765 | 1 |
| STOMA1000074 | 0.011402509 | 60869 | 1 |
| STOMA1000091 | 0.024087821 | 77013 | 3 |
| STOMA1000117 | 0.011402509 | 11297 | 1 |
| STOMA1000133 | 0.011402509 | 56595 | 1 |
| STOMA1000175 | 0.011402509 | 21054 | 1 |
| STOMA1000186 | 0.01340091 | 156471 | 2 |
| STOMA1000189 | 0.107145355 | 69667 | 12 |
| STOMA1000190 | 0.011402509 | 8471 | 1 |
| STOMA1000191 | 0.011402509 | 65857 | 1 |
| STOMA2000032 | 0.011402509 | 115408 | 1 |
| STOMA2000055 | 0.288461073 | 97799 | 16 |
| STOMA2000072 | 0.011402509 | 266304 | 1 |
| STOMA2000088 | 0.011402509 | 171623 | 1 |
| STOMA2000104 | 0.011402509 | 147865 | 1 |
| STOMA2000121 | 0.137498035 | 3725 | 10 |
| STOMA2000148 | 0.014317366 | 236379 | 2 |
| STOMA2000168 | 0.193842645 | 19643 | 17 |
| STOMA2000183 | 0.011402509 | 269473 | 1 |
| STOMA2000188 | 0.011402509 | 128804 | 1 |
| STOMA2000257 | 0.084883416 | 64729 | 11 |
| STOMA2000279 | 0.011402509 | 54996 | 1 |
| STOMA2000289 | 0.061504591 | 98261 | 17 |
| STOMA2000383 | 0.014317366 | 260088 | 2 |
| STOMA2000386 | 0.011402509 | 104256 | 1 |
| STOMA2000395 | 0.078491497 | 28409 | 18 |
| STOMA2000396 | 0.032512699 | 141991 | 8 |
| STOMA2000478 | 0.011402509 | 119714 | 1 |
| STOMA2000482 | 0.043462414 | 152336 | 8 |
| STOMA2000529 | 0.023200526 | 220306 | 2 |
| STOMA2000539 | 0.034207526 | 191824 | 3 |
| STOMA2000567 | 0.011402509 | 267478 | 1 |
| STOMA2000640 | 0.011402509 | 249468 | 1 |
| STOMA2000678 | 0.011402509 | 89418 | 1 |
| STOMA2000686 | 0.011402509 | 101243 | 1 |
| STOMA2000888 | 0.011402509 | 117649 | 1 |
| STOMA2001025 | 0.011402509 | 94387 | 1 |
| STOMA2001389 | 0.02901198 | 193281 | 3 |
| STOMA2002052 | 0.03012564 | 137222 | 12 |
| STOMA2002141 | 0.011402509 | 232751 | 1 |
| STOMA2002166 | 0.286402306 | 49862 | 24 |
| STOMA2002367 | 0.011402509 | 60293 | 1 |
| STOMA2002688 | 0.011402509 | 51423 | 1 |
| STOMA2002828 | 0.03057301 | 149462 | 5 |
| STOMA2003158 | 0.027373949 | 109513 | 7 |
| STOMA2003289 | 0.011402509 | 86576 | 1 |
| STOMA2003444 | 0.012497787 | 215865 | 2 |
| STOMA2003477 | 0.022805017 | 189289 | 2 |
| STOMA2003646 | 0.011402509 | 234771 | 1 |
| STOMA2003716 | 0.014676506 | 7982 | 3 |
| STOMA2003781 | 0.01327935 | 215881 | 2 |
| STOMA2003894 | 0.011402509 | 195946 | 1 |
| STOMA2004194 | 0.011402509 | 220491 | 1 |
| STOMA2004294 | 0.011402509 | 229373 | 1 |
| STOMA2004663 | 0.023996967 | 194702 | 2 |
| STOMA2004668 | 0.011402509 | 99406 | 1 |
| STOMA2004852 | 0.011402509 | 253081 | 1 |
| STOMA2004873 | 0.011402509 | 202344 | 1 |
| STOMA2004884 | 0.011402509 | 176071 | 1 |
| STOMA2004893 | 0.023189402 | 260132 | 2 |
| STOMA2004925 | 0.01340091 | 205048 | 2 |
| STOMA2005120 | 0.013260623 | 220451 | 2 |
| STOMA2005664 | 0.011402509 | 280102 | 1 |
| STOMA2005667 | 0.011402509 | 273810 | 1 |
| STOMA2005746 | 0.011402509 | 16970 | 1 |
| STOMA2005782 | 0.012497787 | 79442 | 2 |
| STOMA2006181 | 0.011402509 | 148331 | 1 |
| STOMA2006213 | 0.011402509 | 206391 | 1 |
| STOMA2006229 | 0.027870128 | 149573 | 4 |
| STOMA2006325 | 0.011402509 | 227761 | 1 |
| STOMA2006330 | 0.011402509 | 236113 | 1 |
| STOMA2006398 | 0.011402509 | 240615 | 1 |
| STOMA2006447 | 0.022805017 | 184625 | 2 |
| STOMA2006780 | 0.019289199 | 189336 | 3 |
| STOMA2006904 | 0.011402509 | 199047 | 1 |
| STOMA2007269 | 0.011402509 | 20645 | 1 |
| STOMA2007680 | 0.011402509 | 221555 | 1 |
| STOMA2007745 | 0.011402509 | 282203 | 1 |
| STOMA2008050 | 0.011402509 | 122137 | 1 |
| STOMA2008361 | 0.011402509 | 270286 | 1 |
| STOMA2008546 | 0.011402509 | 96401 | 1 |
| STOMA2008614 | 0.011402509 | 69248 | 1 |
| STOMA2008638 | 0.022866454 | 146487 | 2 |
| STOMA2008838 | 0.011402509 | 276793 | 1 |
| STOMA2009250 | 0.011402509 | 90400 | 1 |
| STOMA2009253 | 0.011402509 | 280795 | 1 |
| STOMA2009256 | 0.011402509 | 183320 | 1 |
| STOMA2009289 | 0.029205793 | 218254 | 5 |
| SYNOV1000045 . | 0.023852455 | 72118 | 4 |
| SYNOV1000049 | 0.007652175 | 65025 | 2 |
| SYNOV1000118 | 0.039591548 | 48625 | 9 |
| SYNOV1000124 | 0.301223048 | 9436 | 85 |
| SYNOV1000128 | 0.01141413 | 57783 | 3 |
| SYNOV1000164 | 0.043571914 | 59610 | 7 |
| SYNOV1000190 | 0.003613892 | 45988 | 1 |
| SYNOV1000256 | 0.003613892 | 199000 | 1 |
| SYNOV1000374 | 0.008444126 | 159157 | 4 |
| SYNOV2000152 | 0.003613892 | 237386 | 1 |
| SYNOV2000171 | 0.003613892 | 21680 | 1 |
| SYNOV2000173 | 0.003613892 | 274979 | 1 |
| SYNOV2000177 | 0.003613892 | 261581 | 1 |
| SYNOV2000178 | 0.003613892 | 64196 | 1 |
| SYNOV2000193 | 0.003613892 | 201160 | 1 |
| SYNOV2000251 | 0.003613892 | 279584 | 1 |
| SYNOV2000279 | 0.007227784 | 182793 | 2 |
| SYNOV2000291 | 0.005323352 | 144620 | 2 |
| SYNOV2000297 | 0.003613892 | 274374 | 1 |
| SYNOV2000397 | 0.007032812 | 101040 | 3 |
| SYNOV2000426 | 0.003613892 | 274010 | 1 |
| SYNOV2000601 | 0.003613892 | 236868 | 1 |
| SYNOV2000700 | 0.003613892 | 273768 | 1 |
| SYNOV2000820 | 0.004803136 | 191341 | 2 |
| SYNOV2000824 | 0.014455567 | 204721 | 4 |
| SYNOV2000923 | 0.010841675 | 189217 | 3 |
| SYNOV2001014 | 0.003613892 | 115317 | 1 |
| SYNOV2001033 | 0.279470536 | 132567 | 32 |
| SYNOV2001035 | 0.003613892 | 262977 | 1 |
| SYNOV2001088 | 0.003613892 | 278063 | 1 |
| SYNOV2001111 | 0.003613892 | 277402 | 1 |
| SYNOV2001144 | 0.118467456 | 102328 | 12 |
| SYNOV2001212 | 0.015377214 | 169597 | 2 |
| SYNOV2001239 | 0.534030075 | 67581 | 75 |
| SYNOV2001251 | 0.003613892 | 148739 | 1 |
| SYNOV2001262 | 0.080310646 | 127512 | 23 |
| SYNOV2001300 | 0.003613892 | 281048 | 1 |
| SYNOV2001356 | 0.003613892 | 275463 | 1 |
| SYNOV2001374 | 0.091479917 | 60111 | 12 |
| SYNOV2001390 | 0.003613892 | 273646 | 1 |
| SYNOV2001451 | 0.003613892 | 53495 | 1 |
| SYNOV2001457 | 0.003613892 | 43345 | 1 |
| SYNOV2001468 | 0.018359173 | 207870 | 3 |
| SYNOV2001581 | 0.063625364 | 133234 | 6 |
| SYNOV2001648 | 0.154620254 | 54138 | 11 |
| SYNOV2001660 | 0.003613892 | 274189 | 1 |
| SYNOV2001708 | 0.0561349 | 78986 | 2 |
| SYNOV2003326 | 0.003613892 | 9527 | 1 |
| SYNOV2005216 | 0.01715935 | 162569 | 3 |
| SYNOV2005448 | 0.003613892 | 90208 | 1 |
| SYNOV2005541 | 0.011703843 | 255833 | 3 |
| SYNOV2005673 | 0.003613892 | 249806 | 1 |
| SYNOV2005817 | 0.003613892 | 228847 | 1 |
| SYNOV2005962 | 0.003613892 | 75217 | 1 |
| SYNOV2006430 | 0.003613892 | 201271 | 1 |
| SYNOV2006620 | 0.005472006 | 32163 | 2 |
| SYNOV2007758 | 0.008563436 | 131445 | 4 |
| SYNOV2007965 | 0.003613892 | 136221 | 1 |
| SYNOV2008765 | 0.116099831 | 133235 | 2 |
| SYNOV2009172 | 0.321985384 | 124046 | 21 |
| SYNOV2011233 | 0.003613892 | 69147 | 1 |
| SYNOV2012326 | 0.003613892 | 101093 | 1 |
| SYNOV2013365 | 0.003613892 | 156832 | 1 |
| SYNOV2013637 | 0.003613892 | 197633 | 1 |
| SYNOV2014157 | 0.00470917 | 53366 | 2 |
| SYNOV2014364 | 0.003613892 | 183179 | 1 |
| SYNOV2014400 | 0.012911511 | 98799 | 4 |
| SYNOV2016124 | 0.003613892 | 190834 | 1 |
| SYNOV2016837 | 0.003613892 | 73068 | 1 |
| SYNOV2017055 | 0.009924305 | 151032 | 4 |
| SYNOV2017179 | 0.003613892 | 46364 | 1 |
| SYNOV2018921 | 0.003613892 | 214857 | 1 |
| SYNOV2019280 | 0.003613892 | 245355 | 1 |
| SYNOV2020085 | 0.003613892 | 138410 | 1 |
| SYNOV2020463 | 0.038239931 | 182997 | 2 |
| SYNOV2021320 | 0.003613892 | 211037 | 1 |
| SYNOV2021953 | 0.017507311 | 95051 | 9 |
| SYNOV3000184 | 0.003613892 | 279711 | 1 |
| SYNOV3000231 | 0.003613892 | 271871 | 1 |
| SYNOV3000302 | 0.007227784 | 200491 | 2 |
| SYNOV3000345 | 0.003613892 | 285038 | 1 |
| SYNOV4000249 | 0.007032812 | 201872 | 3 |
| SYNOV4000472 | 0.003613892 | 128609 | 1 |
| SYNOV4000598 | 0.005612293 | 71068 | 2 |
| SYNOV4000706 | 0.003613892 | 272086 | 1 |
| SYNOV4001153 | 0.072241522 | 114007 | 34 |
| SYNOV4001224 | 0.005612293 | 234263 | 2 |
| SYNOV4001326 | 0.005490733 | 226248 | 2 |
| SYNOV4001342 | 0.003613892 | 260053 | 1 |
| SYNOV4001395 | 0.009226185 | 266015 | 3 |
| SYNOV4002001 | 0.003613892 | 118215 | 1 |
| SYNOV4002346 | 0.027938227 | 19967 | 7 |
| SYNOV4002392 | 0.003613892 | 159677 | 1 |
| SYNOV4002744 | 0.082954585 | 126331 | 13 |
| SYNOV4002875 | 0.003613892 | 257134 | 1 |
| SYNOV4002883 | 0.006998899 | 65707 | 3 |
| SYNOV4003174 | 0.003613892 | 282533 | 1 |
| SYNOV4003322 | 0.003613892 | 252421 | 1 |
| SYNOV4003681 | 0.003613892 | 272846 | 1 |
| SYNOV4003981 | 0.003613892 | 64201 | 1 |
| SYNOV4004184 | 0.003613892 | 130099 | 1 |
| SYNOV4004210 | 0.177397925 | 117098 | 12 |
| SYNOV4004741 | 0.003613892 | 258543 | 1 |
| SYNOV4004823 | 0.007367575 | 137113 | 3 |
| SYNOV4004914 | 0.003613892 | 266928 | 1 |
| SYNOV4005256 | 0.00470917 | 32026 | 2 |
| SYNOV4005570 | 0.003613892 | 255282 | 1 |
| SYNOV4005739 | 0.003613892 | 252713 | 1 |
| SYNOV4005889 | 0.003613892 | 212673 | 1 |
| SYNOV4005981 | 0.003613892 | 245064 | 1 |
| SYNOV4005989 | 0.008117663 | 119949 | 4 |
| SYNOV4006256, | 0.003613892 | 251201 | 1 |
| SYNOV4006327 | 0.010840761 | 178438 | 4 |
| SYNOV4006542 | 0.045197226 | 86110 | 14 |
| SYNOV4007012 | 0.005011047 | 12308 | 2 |
| SYNOV4007215 | 0.003613892 | 260443 | 1 |
| SYNOV4007360 | 0.003613892 | 67035 | 1 |
| SYNOV4007430 | 0.005289439 | 199727 | 2 |
| SYNOV4007521 | 0.003613892 | 258386 | 1 |
| SYNOV4007553 | 0.005011047 | 127986 | 2 |
| SYNOV4007671 | 0.228572571 | 42872 | 29 |
| SYNOV4007711 | 0.003613892 | 231528 | 1 |
| SYNOV4008245 | 0.018136331 | 157123 | 10 |
| SYNOV4008336 | 0.003613892 | 244623 | 1 |
| SYNOV4008440 | 0.113584757 | 111121 | 28 |
| SYNOV4009129 | 0.021582962 | 137399 | 7 |
| SYNOV4009139 | 0.007227784 | 75216 | 2 |
| SYNOV4009295 | 0.015092343 | 162030 | 6 |
| SYNOV4009298 | 0.005695404 | 78315 | 2 |
| SYNOV4009575 | 0.034970012 | 95138 | 7 |
| T1ESE2000116 | 0.038613382 | 149178 | 2 |
| T1ESE2000609 | 0.073717682 | 34812 | 6 |
| T1ESE2000904 | 0.072576727 | 51440 | 9 |
| T1ESE2002665 | 0.040198185 | 189691 | 2 |
| TBAES2000004 | 0.011786893 | 205550 | 1 |
| TBAES2000059 | 0.111195391 | 152895 | 12 |
| TBAES2000116 | 0.014768852 | 155332 | 2 |
| TBAES2000255 | 0.011786893 | 64951 | 1 |
| TBAES2000315 | 0.023573786 | 153609 | 2 |
| TBAES2000352 | 0.023550215 | 84454 | 2 |
| TBAES2000377 | 0.131115217 | 119962 | 31 |
| TBAES2000932 | 0.011786893 | 252259 | 1 |
| TBAES2001171 | 0.011786893 | 63779 | 1 |
| TBAES2001220 | 0.011786893 | 110967 | 1 |
| TBAES2001229 | 0.011786893 | 237837 | 1 |
| TBAES2001258 | 0.017628356 | 186006 | 2 |
| TBAES2001296 | 0.014872325 | 227185 | 3 |
| TBAES2001492 | 0.011786893 | 169107 | 1 |
| TBAES2001553 | 0.056963783 | 176602 | 4 |
| TBAES2001751 | 0.011786893 | 256602 | 1 |
| TBAES2002197 | 0.021616218 | 126438 | 6 |
| TBAES2003435 | 0.011786893 | 284396 | 1 |
| TBAES2003492 | 0.011786893 | 144056 | 1 |
| TBAES2003550 | 0.011786893 | 267845 | 1 |
| TBAES2003702 | 0.013785294 | 135132 | 2 |
| TBAES2003917 | 0.011786893 | 282672 | 1 |
| TBAES2004055 | 0.011786893 | 271472 | 1 |
| TBAES2004105 | 0.011786893 | 283208 | 1 |
| TBAES2004939 | 0.011786893 | 211922 | 1 |
| TBAES2005157 | 0.011786893 | 123231 | 1 |
| TBAES2005361 | 0.011786893 | 247067 | 1 |
| TBAES2005543 | 0.011786893 | 249725 | 1 |
| TBAES2006568 | 0.011786893 | 245633 | 1 |
| TBAES2006881 | 0.011786893 | 226830 | 1 |
| TBAES2007379 | 0.011786893 | 150529 | 1 |
| TBAES2007428 | 0.031196233 | 8072 | 4 |
| TBAES2007481 | 0.011786893 | 282907 | 1 |
| TBAES2007504 | 0.011786893 | 182933 | 1 |
| TBAES2007548 | 0.011786893 | 169159 | 1 |
| TBAES2007697 | 0.011786893 | 261708 | 1 |
| TBAES2007862 | 0.011786893 | 230864 | 1 |
| TBAES2007964 | 0.023573786 | 259523 | 2 |
| TBAES2008133 | 0.011786893 | 139277 | 1 |
| TBAES2009387 | 0.313458544 | 7997 | 19 |
| TCERX2000171 | 0.035360679 | 140246 | 1 |
| TCERX2000431 | 0.079764438 | 63317 | 7 |
| TCERX2000613 | 0.041355883 | 199690 | 4 |
| TCERX2000998 | 0.035360679 | 281225 | 1 |
| TCERX2002644 | 0.048835849 | 216299 | 3 |
| TCOLN1000037 | 0.035523979 | 41662 | 1 |
| TCOLN1000180 | 0.068438917 | 153138 | 9 |
| TCOLN2000139 | 0.035523979 | 183290 | 1 |
| TCOLN2000236 | 0.035523979 | 30977 | 1 |
| TCOLN2000641 | 0.035523979 | 240028 | 1 |
| TCOLN2001268 | 0.035523979 | 221266 | 1 |
| TCOLN2002047 | 0.035523979 | 278678 | 1 |
| TCOLN2002278 | 0.035523979 | 259462 | 1 |
| TESOP1000035 | 0.037883681 | 208246 | 6 |
| TESOP1000127 | 0.017609472 | 237326 | 2 |
| TESOP1000160 | 0.011463946 | 101000 | 1 |
| TESOP2000083 | 0.011463946 | 184794 | 1 |
| TESOP2000090 | 0.011463946 | 203787 | 1 |
| TESOP2000205 | 0.011463946 | 191566 | 1 |
| TESOP2000312 | 0.034688447 | 41756 | 3 |
| TESOP2000390 | 0.011463946 | 141251 | 1 |
| TESOP2000400 | 0.011463946 | 155861 | 1 |
| TESOP2000510 | 0.011463946 | 42442 | 1 |
| TESOP2000527 | 0.016236917 | 134400 | 3 |
| TESOP2000569 | 0.011463946 | 72611 | 1 |
| TESOP2000739 | 0.011463946 | 261142 | 1 |
| TESOP2000801 | 0.011463946 | 254808 | 1 |
| TESOP2001122 | 0.011463946 | 276585 | 1 |
| TESOP2001166 | 0.011463946 | 204471 | 1 |
| TESOP2001345 | 0.011463946 | 198263 | 1 |
| TESOP2001474 | 0.011463946 | 159221 | 1 |
| TESOP2001605 | 0.01715935 | 162569 | 3 |
| TESOP2001796 | 0.011463946 | 129621 | 1 |
| TESOP2001818 | 0.011463946 | 146677 | 1 |
| TESOP2001849 | 0.011463946 | 189347 | 1 |
| TESOP2001865 | 0.011463946 | 179907 | 1 |
| TESOP2001901 | 0.011463946 | 199053 | 1 |
| TESOP2001953 | 0.012861101 | 268307 | 2 |
| TESOP2002005 | 0.011463946 | 150179 | 1 |
| TESOP2002110 | 0.011463946 | 138420 | 1 |
| TESOP2002150 | 0.011463946 | 272460 | 1 |
| TESOP2002273 | 0.041282319 | 106573 | 12 |
| TESOP2002451 | 0.070540928 | 79461 | 9 |
| TESOP2002489 | 0.011463946 | 135765 | 1 |
| TESOP2002539 | 0.011463946 | 204319 | 1 |
| TESOP2002545 | 0.011463946 | 170351 | 1 |
| TESOP2002804 | 0.011463946 | 190817 | 1 |
| TESOP2002950 | 0.01265319 | 280191 | 2 |
| TESOP2003273 | 0.011463946 | 250570 | 1 |
| TESOP2003308 | 0.011463946 | 162658 | 1 |
| TESOP2003553 | 0.011463946 | 190809 | 1 |
| TESOP2003753 | 0.011463946 | 261893 | 1 |
| TESOP2004110 | 0.011463946 | 279536 | 1 |
| TESOP2004114 | 0.011463946 | 271486 | 1 |
| TESOP2004173 | 0.011463946 | 29836 | 1 |
| TESOP2004214 | 0.011463946 | 249179 | 1 |
| TESOP2005199 | 0.011463946 | 169453 | 1 |
| TESOP2005285 | 0.011463946 | 140935 | 1 |
| TESOP2005485 | 0.011463946 | 183895 | 1 |
| TESOP2005498 | 0.011463946 | 170689 | 1 |
| TESOP2005579 | 0.011463946 | 211732 | 1 |
| TESOP2006041 | 0.011463946 | 102411 | 1 |
| TESOP2006053 | 0.022750628 | 196762 | 2 |
| TESOP2006060 | 0.011463946 | 268697 | 1 |
| TESOP2006068 | 0.011463946 | 272778 | 1 |
| TESOP2006110 | 0.011463946 | 267163 | 1 |
| TESOP2006398 | 0.011463946 | 170614 | 1 |
| TESOP2006670 | 0.014378803 | 93179 | 2 |
| TESOP2006704 | 0.011463946 | 280142 | 1 |
| TESOP2006746 | 0.011463946 | 264503 | 1 |
| TESOP2006865 | 0.011463946 | 134378 | 1 |
| TESOP2006893 | 0.019503913 | 39542 | 3 |
| TESOP2007041 | 0.011463946 | 200517 | 1 |
| TESOP2007052 | 0.022927892 | 211923 | 2 |
| TESOP2007262 | 0.011463946 | 194313 | 1 |
| TESOP2007384 | 0.083471878 | 63178 | 16 |
| TESOP2007636 | 0.011463946 | 239209 | 1 |
| TESOP2007674 | 0.011463946 | 205967 | 1 |
| TESOP2007688 | 0.011463946 | 248963 | 1 |
| TESOP2007725 | 0.011463946 | 73757 | 1 |
| TESOP2007912 | 0.014559442 | 151738 | 2 |
| TESOP2007978 | 0.011463946 | 265637 | 1 |
| TESOP2008127 | 0.011463946 | 191304 | 1 |
| TESOP2008552 | 0.011463946 | 204602 | 1 |
| TESOP2008556 | 0.011463946 | 252327 | 1 |
| TESOP2008563 | 0.011463946 | 272291 | 1 |
| TESOP2008820 | 0.017427864 | 88487 | 3 |
| TESOP2009121 | 0.011463946 | 251601 | 1 |
| TESOP2009201 | 0.011463946 | 160481 | 1 |
| TESOP2009247 | 0.011463946 | 175343 | 1 |
| TESOP2009555 | 0.011463946 | 102879 | 1 |
| TESTI1000018 | 0.033953626 | 21608 | 31 |
| TESTI1000019 | 0.00657167 | 62850 | 6 |
| TESTI1000023 | 0.199795094 | 29350 | 74 |
| TESTI1000025 | 0.005476391 | 73890 | 5 |
| TESTI1000029 | 0.001095278 | 43508 | 1 |
| TESTI1000030 | 0.001095278 | 69542 | 1 |
| TESTI1000042 | 0.016429174 | 68868 | 15 |
| TESTI1000045 | 0.001095278 | 47390 | 1 |
| TESTI1000051 | 0.032173191 | 65277 | 19 |
| TESTI1000055 | 0.005476391 | 74971 | 5 |
| TESTI1000064 | 0.034419358 | 1169 | 27 |
| TESTI1000070 | 0.046405709 | 66041 | 18 |
| TESTI1000085 | 0.002190557 | 68432 | 2 |
| TESTI1000088 | 0.001095278 | 48565 | 1 |
| TESTI1000093 | 0.001095278 | 17846 | 1 |
| TESTI1000094 | 0.016987423 | 77385 | 13 |
| TESTI1000096 | 0.009578075 | 61300 | 5 |
| TESTI1000101 | 0.039118604 | 68646 | 12 |
| TESTI1000109 | 0.001095278 | 34647 | 1 |
| TESTI1000125 | 0.001095278 | 73674 | 1 |
| TESTI1000127 | 0.01861973 | 57463 | 17 |
| TESTI1000128 | 0.002190557 | 27470 | 2 |
| TESTI1000131 | 0.085431704 | 60885 | 78 |
| TESTI1000138 | 0.00561324 | 78320 | 3 |
| TESTI1000142 | 0.024833461 | 69994 | 12 |
| TESTI1000157 | 0.001095278 | 76581 | 1 |
| TESTI1000163 | 0.040663925 | 75196 | 6 |
| TESTI1000168 | 0.053467984 | 46857 | 34 |
| TESTI1000169 | 0.003285835 | 59884 | 3 |
| TESTI1000179 | 0.001095278 | 72121 | 1 |
| TESTI1000183 | 0.001095278 | 73977 | 1 |
| TESTI1000191 | 0.044906408 | 45346 | 41 |
| TESTI1000257 | 0.003285835 | 12790 | 3 |
| TESTI1000266 | 0.00317679 | 197797 | 2 |
| TESTI1000319 | 0.001095278 | 204088 | 1 |
| TESTI1000330 | 0.002284523 | 52657 | 2 |
| TESTI1000348 | 0.001095278 | 152904 | 1 |
| TESTI1000390 | 0.001095278 | 204385 | 1 |
| TESTI1000391 | 0.001095278 | 259869 | 1 |
| TESTI1000459 | 0.001095278 | 281932 | 1 |
| TESTI1000491 | 0.001095278 | 260885 | 1 |
| TESTI1000545 | 0.012048061 | 170428 | 11 |
| TESTI2000006 | 0.016945006 | 32201 | 4 |
| TESTI2000018 | 0.001095278 | 239006 | 1 |
| TESTI2000044 | 0.001095278 | 191090 | 1 |
| TESTI2000100 | 0.008648766 | 98057 | 4 |
| TESTI2000117 | 0.00657167 | 158524 | 6 |
| TESTI2000120 | 0.001095278 | 7353 | 1 |
| TESTI2000147 | 0.001095278 | 203514 | 1 |
| TESTI2000154 | 0.00657167 | 150651 | 6 |
| TESTI2000172 | 0.019586134 | 104323 | 4 |
| TESTI2000177 | 0.006872137 | 91074 | 5 |
| TESTI2000179 | 0.004573946 | 188585 | 3 |
| TESTI2000184 | 0.148226826 | 28573 | 63 |
| TESTI2000207 | 0.001095278 | 142535 | 1 |
| TESTI2000208 | 0.001095278 | 134081 | 1 |
| TESTI2000221 | 0.001095278 | 215078 | 1 |
| TESTI2000238 | 0.005286053 | 53584 | 3 |
| TESTI2000253 | 0.037525172 | 79122 | 22 |
| TESTI2000272 | 0.017304703 | 122737 | 15 |
| TESTI2000278 | 0.001095278 | 188028 | 1 |
| TESTI2000349 | 0.007037402 | 87817 | 2 |
| TESTI2000356 | 0.009555137 | 150284 | 5 |
| TESTI2000358 | 0.001095278 | 9248 | 1 |
| TESTI2000372 | 0.001095278 | 276618 | 1 |
| TESTI2000427 | 0.001095278 | 271140 | 1 |
| TESTI2000431 | 0.005162676 | 131959 | 4 |
| TESTI2000435 | 0.008712949 | 20365 | 3 |
| TESTI2000443 | 0.001095278 | 243255 | 1 |
| TESTI2000452 | 0.001095278 | 183847 | 1 |
| TESTI2000462 | 0.002190557 | 221090 | 2 |
| TESTI2000489 | 0.010422679 | 105330 | 5 |
| TESTI2000520 | 0.044537665 | 150243 | 4 |
| TESTI2000571 | 0.001095278 | 211661 | 1 |
| TESTI2000591 | 0.091754732 | 130695 | 7 |
| TESTI2000598 | 0.033356304 | 143288 | 18 |
| TESTI2000600 | 0.004514199 | 83453 | 3 |
| TESTI2000616 | 0.001095278 | 269203 | 1 |
| TESTI2000627 | 0.007952337 | 130201 | 5 |
| TESTI2000644 | 0.001095278 | 130461 | 1 |
| TESTI2000671 | 0.008762226 | 191413 | 8 |
| TESTI2000683 | 0.004381113 | 136399 | 4 |
| TESTI2000689 | 0.002190557 | 204157 | 2 |
| TESTI2000695 | 0.047096965 | 104901 | 43 |
| TESTI2000699 | 0.017524452 | 102062 | 16 |
| TESTI2000707 | 0.007666948 | 227032 | 7 |
| TESTI2000745 | 0.001095278 | 258623 | 1 |
| TESTI2000756 | 0.001095278 | 207786 | 1 |
| TESTI2000762 | 0.002190557 | 115095 | 2 |
| TESTI2000784 | 0.073727461 | 72398 | 37 |
| TESTI2000808 | 0.007666948 | 139042 | 7 |
| TESTI2000819 | 0.001095278 | 224281 | 1 |
| TESTI2000830 | 0.009857504 | 120679 | 9 |
| TESTI2000840 | 0.001095278 | 196880 | 1 |
| TESTI2000849 | 0.006239227 | 166525 | 5 |
| TESTI2000850 | 0.001095278 | 240625 | 1 |
| TESTI2000874 | 0.013404019 | 40488 | 3 |
| TESTI2000883 | 0.022980289 | 35239 | 7 |
| TESTI2000909 | 0.012855833 | 144727 | 2 |
| TESTI2000951 | 0.001095278 | 179635 | 1 |
| TESTI2000970 | 0.034469079 | 161949 | 10 |
| TESTI2000981 | 0.002190557 | 232411 | 2 |
| TESTI2001008 | 0.001095278 | 104151 | 1 |
| TESTI2001049 | 0.001095278 | 153511 | 1 |
| TESTI2001082 | 0.001095278 | 228216 | 1 |
| TESTI2001099 | 0.341930539 | 32316 | 112 |
| TESTI2001120 | 0.173309652 | 93764 | 6 |
| TESTI2001134 | 0.001095278 | 94768 | 1 |
| TESTI2001141 | 0.017744117 | 119327 | 11 |
| TESTI2001153 | 0.42079305 | 57205 | 61 |
| TESTI2001180 | 0.001095278 | 237087 | 1 |
| TESTI2001208 | 0.019715009 | 146601 | 18 |
| TESTI2001229 | 0.001095278 | 173732 | 1 |
| TESTI2001236 | 0.001095278 | 216980 | 1 |
| TESTI2001237 | 0.001095278 | 114472 | 1 |
| TESTI2001269 | 0.306947036 | 91948 | 49 |
| TESTI2001306 | 0.001095278 | 191329 | 1 |
| TESTI2001313 | 0.002190557 | 63848 | 2 |
| TESTI2001330 | 0.001095278 | 22189 | 1 |
| TESTI2001339 | 0.004010135 | 45797 | 2 |
| TESTI2001345 | 0.001095278 | 112645 | 1 |
| TESTI2001352 | 0.001095278 | 145355 | 1 |
| TESTI2001364 | 0.002190557 | 148748 | 2 |
| TESTI2001412 | 0.007666948 | 158175 | 7 |
| TESTI2001420 | 0.00309368 | 203893 | 2 |
| TESTI2001498 | 0.001095278 | 150988 | 1 |
| TESTI2001511 | 0.09577017 | 166656 | 7 |
| TESTI2001512 | 0.001095278 | 49259 | 1 |
| TESTI2001518 | 0.001095278 | 211570 | 1 |
| TESTI2001556 | 0.001095278 | 111905 | 1 |
| TESTI2001593 | 0.001095278 | 255653 | 1 |
| TESTI2001613 | 0.012265241 | 176173 | 8 |
| TESTI2001621 | 0.002190557 | 234501 | 2 |
| TESTI2001661 | 0.006080146 | 86267 | 5 |
| TESTI2001665 | 0.001095278 | 139587 | 1 |
| TESTI2001671 | 0.001095278 | 115851 | 1 |
| TESTI2001697 | 0.033953626 | 130424 | 31 |
| TESTI2001725 | 0.002190557 | 164747 | 2 |
| TESTI2001758 | 0.001095278 | 192679 | 1 |
| TESTI2001766 | 0.00657167 | 111205 | 6 |
| TESTI2001792 | 0.001095278 | 254726 | 1 |
| TESTI2001793 | 0.001095278 | 247116 | 1 |
| TESTI2001795 | 0.002190557 | 179263 | 2 |
| TESTI2001812 | 0.001095278 | 222469 | 1 |
| TESTI2001815 | 0.00470917 | 123988 | 2 |
| TESTI2001823 | 0.001095278 | 285083 | 1 |
| TESTI2001826 | 0.014975336 | 135124 | 9 |
| TESTI2001827 | 0.001095278 | 108934 | 1 |
| TESTI2001829 | 0.197326509 | 99858 | 14 |
| TESTI2001852 | 0.004381113 | 86844 | 4 |
| TESTI2001862 | 0.063367423 | 149372 | 13 |
| TESTI2001869 | 0.007020874 | 144206 | 3 |
| TESTI2001879 | 0.00297212 | 103983 | 2 |
| TESTI2001894 | 0.002190557 | 175287 | 2 |
| TESTI2001898 | 0.002804738 | 161475 | 2 |
| TESTI2001915 | 0.001095278 | 40832 | 1 |
| TESTI2001950 | 0.00657167 | 139425 | 6 |
| TESTI2001959 | 0.001095278 | 138129 | 1 |
| TESTI2001968 | 0.001095278 | 121250 | 1 |
| TESTI2001985 | 0.028543138 | 32388 | 20 |
| TESTI2001991 | 0.001095278 | 157090 | 1 |
| TESTI2002002 | 0.001095278 | 159843 | 1 |
| TESTI2002012 | 0.001095278 | 184577 | 1 |
| TESTI2002036 | 0.001095278 | 51002 | 1 |
| TESTI2002048 | 0.001095278 | 196931 | 1 |
| TESTI2002057 | 0.005476391 | 175695 | 5 |
| TESTI2002081 | 0.001095278 | 112556 | 1 |
| TESTI2002090 | 0.003285835 | 125929 | 3 |
| TESTI2002105 | 0.012829624 | 87249 | 11 |
| TESTI2002149 | 0.100180728 | 4257 | 10 |
| TESTI2002216 | 0.001095278 | 275644 | 1 |
| TESTI2002223 | 0.006092926 | 114142 | 3 |
| TESTI2002245 | 0.001095278 | 261477 | 1 |
| TESTI2002251 | 0.004381113 | 48549 | 4 |
| TESTI2002256 | 0.004830234 | 226156 | 3 |
| TESTI2002264 | 0.001095278 | 129428 | 1 |
| TESTI2002282 | 0.003285835 | 47089 | 3 |
| TESTI2002293 | 0.003285835 | 32392 | 3 |
| TESTI2002294 | 0.001095278 | 5586 | 1 |
| TESTI2002326 | 0.001095278 | 209610 | 1 |
| TESTI2002351 | 0.001095278 | 232614 | 1 |
| TESTI2002361 | 0.001095278 | 209625 | 1 |
| TESTI2002365 | 0.153442253 | 58041 | 42 |
| TESTI2002369 | 0.011150563 | 121018 | 5 |
| TESTI2002406 | 0.001095278 | 247540 | 1 |
| TESTI2002415 | 0.001095278 | 273017 | 1 |
| TESTI2002423 | 0.001095278 | 62854 | 1 |
| TESTI2002453 | 0.001095278 | 232542 | 1 |
| TESTI2002461 | 0.001095278 | 116441 | 1 |
| TESTI2002465 | 0.028477235 | 149181 | 26 |
| TESTI2002467 | 0.007136844 | 123820 | 2 |
| TESTI2002498 | 0.004381113 | 87416 | 4 |
| TESTI2002516 | 0.001095278 | 234712 | 1 |
| TESTI2002544 | 0.001095278 | 122820 | 1 |
| TESTI2002578 | 0.001095278 | 272292 | 1 |
| TESTI2002580 | 0.001095278 | 233245 | 1 |
| TESTI2002618 | 0.005476391 | 171396 | 5 |
| TESTI2002632 | 0.015475564 | 49878 | 4 |
| TESTI2002676 | 0.004381113 | 135276 | 4 |
| TESTI2002690 | 0.001095278 | 171132 | 1 |
| TESTI2002696 | 0.012901654 | 175601 | 2 |
| TESTI2002698 | 0.001095278 | 90215 | 1 |
| TESTI2002700 | 0.002190557 | 80273 | 2 |
| TESTI2002707 | 0.101125978 | 126296 | 41 |
| TESTI2002724 | 0.001095278 | 252087 | 1 |
| TESTI2002729 | 0.005476391 | 123176 | 5 |
| TESTI2002752 | 0.001095278 | 269762 | 1 |
| TESTI2002753 | 0.001095278 | 169585 | 1 |
| TESTI2002789 | 0.001095278 | 92078 | 1 |
| TESTI2002802 | 0.002190557 | 41654 | 2 |
| TESTI2002806 | 0.001095278 | 245031 | 1 |
| TESTI2002840 | 0.004190774 | 165756 | 2 |
| TESTI2002866 | 0.015748053 | 72286 | 7 |
| TESTI2002877 | 0.004490835 | 135151 | 3 |
| TESTI2002910 | 0.001095278 | 146360 | 1 |
| TESTI2002912 | 0.024096122 | 151158 | 22 |
| TESTI2002928 | 0.003285835 | 145281 | 3 |
| TESTI2002937 | 0.001095278 | 220088 | 1 |
| TESTI2002965 | 0.020543223 | 110181 | 16 |
| TESTI2002993 | 0.002190557 | 50406 | 2 |
| TESTI2003005 | 0.055151874 | 175120 | 6 |
| TESTI2003020 | 0.003285835 | 140905 | 3 |
| TESTI2003031 | 0.001095278 | 79677 | 1 |
| TESTI2003037 | 0.00657167 | 92963 | 6 |
| TESTI2003044 | 0.018358563 | 141578 | 7 |
| TESTI2003059 | 0.001095278 | 111172 | 1 |
| TESTI2003061 | 0.012939161 | 105355 | 9 |
| TESTI2003071 | 0.048025803 | 101971 | 4 |
| TESTI2003074 | 0.00657167 | 150400 | 6 |
| TESTI2003089 | 0.001095278 | 256927 | 1 |
| TESTI2003104 | 0.001095278 | 219919 | 1 |
| TESTI2003109 | 0.014706466 | 127396 | 12 |
| TESTI2003117 | 0.013143339 | 152016 | 12 |
| TESTI2003127 | 0.015692265 | 144358 | 4 |
| TESTI2003130 | 0.001095278 | 142013 | 1 |
| TESTI2003131 | 0.002190557 | 11698 | 2 |
| TESTI2003139 | 0.001095278 | 49143 | 1 |
| TESTI2003141 | 0.029698302 | 169058 | 17 |
| TESTI2003152 | 0.007666948 | 149426 | 7 |
| TESTI2003181 | 0.003285835 | 169633 | 3 |
| TESTI2003193 | 0.001095278 | 261479 | 1 |
| TESTI2003196 | 0.001095278 | 207514 | 1 |
| TESTI2003228 | 0.001095278 | 151488 | 1 |
| TESTI2003236 | 0.001095278 | 198524 | 1 |
| TESTI2003255 | 0.001095278 | 183829 | 1 |
| TESTI2003258 | 0.001095278 | 177099 | 1 |
| TESTI2003277 | 0.002190557 | 158078 | 2 |
| TESTI2003280 | 0.040846566 | 143562 | 13 |
| TESTI2003299 | 0.001095278 | 271594 | 1 |
| TESTI2003311 | 0.002190557 | 102478 | 2 |
| TESTI2003321 | 0.001095278 | 189477 | 1 |
| TESTI2003325 | 0.039591907 | 126123 | 8 |
| TESTI2003327 | 0.001095278 | 114082 | 1 |
| TESTI2003330 | 0.001095278 | 203090 | 1 |
| TESTI2003347 | 0.007666948 | 114395 | 7 |
| TESTI2003354 | 0.001095278 | 217258 | 1 |
| TESTI2003356 | 0.023000843 | 129482 | 21 |
| TESTI2003372 | 0.001095278 | 241195 | 1 |
| TESTI2003376 | 0.001095278 | 275753 | 1 |
| TESTI2003413 | 0.064354769 | 119803 | 16 |
| TESTI2003418 | 0.001095278 | 179834 | 1 |
| TESTI2003419 | 0.001095278 | 52769 | 1 |
| TESTI2003432 | 0.001095278 | 173651 | 1 |
| TESTI2003454 | 0.031553162 | 115718 | 6 |
| TESTI2003475 | 0.001095278 | 128662 | 1 |
| TESTI2003476 | 0.001095278 | 222858 | 1 |
| TESTI2003498 | 0.001095278 | 145194 | 1 |
| TESTI2003512 | 0.017524452 | 125938 | 16 |
| TESTI2003533 | 0.193260242 | 78627 | 23 |
| TESTI2003539 | 0.007037402 | 146522 | 2 |
| TESTI2003541 | 0.005736948 | 93433 | 4 |
| TESTI2003545 | 0.001095278 | 204932 | 1 |
| TESTI2003551 | 0.023000843 | 101336 | 21 |
| TESTI2003573 | 0.022433577 | 108147 | 7 |
| TESTI2003574 | 0.028477235 | 150596 | 26 |
| TESTI2003579 | 0.014464015 | 143369 | 4 |
| TESTI2003580 | 0.001095278 | 227906 | 1 |
| TESTI2003589 | 0.003285835 | 128570 | 3 |
| TESTI2003596 | 0.040217458 | 145726 | 30 |
| TESTI2003598 | 0.001095278 | 15300 | 1 |
| TESTI2003615 | 0.001095278 | 224242 | 1 |
| TESTI2003625 | 0.021891242 | 106628 | 8 |
| TESTI2003638 | 0.001095278 | 115099 | 1 |
| TESTI2003649 | 0.001095278 | 169925 | 1 |
| TESTI2003692 | 0.002804738 | 9686 | 2 |
| TESTI2003718 | 0.001095278 | 170227 | 1 |
| TESTI2003727 | 0.001095278 | 252048 | 1 |
| TESTI2003756 | 0.015971147 | 117819 | 5 |
| TESTI2003768 | 0.001095278 | 265731 | 1 |
| TESTI2003781 | 0.001095278 | 104914 | 1 |
| TESTI2003787 | 0.001095278 | 241921 | 1 |
| TESTI2003824 | 0.001095278 | 179332 | 1 |
| TESTI2003827 | 0.001095278 | 264974 | 1 |
| TESTI2003894 | 0.001095278 | 175304 | 1 |
| TESTI2003911 | 0.001095278 | 55215 | 1 |
| TESTI2003914 | 0.010952783 | 157578 | 10 |
| TESTI2003940 | 0.001095278 | 111215 | 1 |
| TESTI2003962 | 0.001095278 | 221980 | 1 |
| TESTI2003998 | 0.001095278 | 194396 | 1 |
| TESTI2004000 | 0.023900295 | 150117 | 3 |
| TESTI2004031 | 0.41796801 | 94161 | 32 |
| TESTI2004073 | 0.002190557 | 221886 | 2 |
| TESTI2004080 | 0.034333085 | 2702 | 7 |
| TESTI2004085 | 0.001095278 | 123490 | 1 |
| TESTI2004089 | 0.001095278 | 196517 | 1 |
| TESTI2004111 | 0.001095278 | 232498 | 1 |
| TESTI2004119 | 0.001095278 | 113237 | 1 |
| TESTI2004122 | 0.054881643 | 76368 | 7 |
| TESTI2004153 | 0.038622179 | 146384 | 11 |
| TESTI2004163 | 0.014238617 | 159550 | 13 |
| TESTI2004169 | 0.007666948 | 161692 | 7 |
| TESTI2004207 | 0.013143339 | 133969 | 12 |
| TESTI2004215 | 0.054763913 | 103926 | 50 |
| TESTI2004227 | 0.001095278 | 85103 | 1 |
| TESTI2004229 | 0.002190557 | 199241 | 2 |
| TESTI2004243 | 0.002190557 | 196593 | 2 |
| TESTI2004255 | 0.001095278 | 241059 | 1 |
| TESTI2004287 | 0.001095278 | 234657 | 1 |
| TESTI2004295 | 0.020462353 | 138900 | 9 |
| TESTI2004313 | 0.001095278 | 241056 | 1 |
| TESTI2004318 | 0.001095278 | 157092 | 1 |
| TESTI2004322 | 0.00657167 | 63038 | 6 |
| TESTI2004391 | 0.039676011 | 110289 | 29 |
| TESTI2004399 | 0.001095278 | 210238 | 1 |
| TESTI2004423 | 0.036144182 | 144950 | 33 |
| TESTI2004431 | 0.020030803 | 104706 | 9 |
| TESTI2004432 | 0.00297212 | 149161 | 2 |
| TESTI2004452 | 0.002190557 | 101966 | 2 |
| TESTI2004459 | 0.001095278 | 160533 | 1 |
| TESTI2004490 | 0.001095278 | 153831 | 1 |
| TESTI2004539 | 0.008762226 | 138191 | 8 |
| TESTI2004552 | 0.012048061 | 104424 | 11 |
| TESTI2004560 | 0.001095278 | 192469 | 1 |
| TESTI2004574 | 0.004381113 | 139495 | 4 |
| TESTI2004601 | 0.001095278 | 275263 | 1 |
| TESTI2004611 | 0.001095278 | 244629 | 1 |
| TESTI2004649 | 0.004381113 | 16947 | 4 |
| TESTI2004654 | 0.001095278 | 140198 | 1 |
| TESTI2004675 | 0.001095278 | 254270 | 1 |
| TESTI2004687 | 0.002190557 | 116944 | 2 |
| TESTI2004689 | 0.01861973 | 124397 | 17 |
| TESTI2004700 | 0.001095278 | 167092 | 1 |
| TESTI2004706 | 0.012048061 | 164952 | 11 |
| TESTI2004712 | 0.148154102 | 253428 | 2 |
| TESTI2004734 | 0.00488625 | 179957 | 3 |
| TESTI2004737 | 0.094592715 | 113384 | 23 |
| TESTI2004783 | 0.002190557 | 197228 | 2 |
| TESTI2004791 | 0.001095278 | 228537 | 1 |
| TESTI2004793 | 0.001095278 | 83242 | 1 |
| TESTI2004860 | 0.001095278 | 244678 | 1 |
| TESTI2004906 | 0.00657167 | 66844 | 6 |
| TESTI2004920 | 0.002190557 | 1926 | 2 |
| TESTI2004929 | 0.05901212 | 105357 | 10 |
| TESTI2004936 | 0.015333896 | 49269 | 14 |
| TESTI2004941 | 0.001095278 | 91085 | 1 |
| TESTI2004971 | 0.002190557 | 122350 | 2 |
| TESTI2004982 | 0.002190557 | 220971 | 2 |
| TESTI2004993 | 0.001095278 | 99923 | 1 |
| TESTI2004994 | 0.003285835 | 141087 | 3 |
| TESTI2004999 | 0.007666948 | 161516 | 7 |
| TESTI2005017 | 0.050736982 | 126735 | 31 |
| TESTI2005025 | 0.001095278 | 126403 | 1 |
| TESTI2005040 | 0.005286053 | 140802 | 3 |
| TESTI2005060 | 0.001095278 | 260445 | 1 |
| TESTI2005072 | 0.036238149 | 122814 | 33 |
| TESTI2005085 | 0.001095278 | 235790 | 1 |
| TESTI2005112 | 0.008612288 | 118777 | 3 |
| TESTI2005120 | 0.014238617 | 173590 | 13 |
| TESTI2005153 | 0.001095278 | 240998 | 1 |
| TESTI2005155 | 0.001095278 | 243370 | 1 |
| TESTI2005173 | 0.011621917 | 117344 | 6 |
| TESTI2005267 | 0.001095278 | 84861 | 1 |
| TESTI2005268 | 0.013143339 | 104241 | 12 |
| TESTI2005280 | 0.001095278 | 209390 | 1 |
| TESTI2005307 | 0.028988398 | 143164 | 17 |
| TESTI2005326 | 0.007308388 | 224269 | 2 |
| TESTI2005363 | 0.001095278 | 150880 | 1 |
| TESTI2005376 | 0.001095278 | 210826 | 1 |
| TESTI2005378 | 0.001095278 | 102823 | 1 |
| TESTI2005380 | 0.004381113 | 101760 | 4 |
| TESTI2005391 | 0.001095278 | 143906 | 1 |
| TESTI2005395 | 0.004272069 | 132790 | 3 |
| TESTI2005396 | 0.011355233 | 151385 | 5 |
| TESTI2005408 | 0.094575798 | 80759 | 19 |
| TESTI2005415 | 0.001095278 | 227486 | 1 |
| TESTI2005470 | 0.010952783 | 150957 | 10 |
| TESTI2005492 | 0.001095278 | 148638 | 1 |
| TESTI2005564 | 0.0457525 | 124503 | 14 |
| TESTI2005568 | 0.005476391 | 92197 | 5 |
| TESTI2005584 | 0.005476391 | 93837 | 5 |
| TESTI2005588 | 0.00657167 | 152872 | 6 |
| TESTI2005590 | 0.001095278 | 95041 | 1 |
| TESTI2005603 | 0.001095278 | 45383 | 1 |
| TESTI2005609 | 0.001095278 | 235163 | 1 |
| TESTI2005610 | 0.001095278 | 167051 | 1 |
| TESTI2005630 | 0.001095278 | 235167 | 1 |
| TESTI2005672 | 0.001095278 | 34912 | 1 |
| TESTI2005683 | 0.003285835 | 207983 | 3 |
| TESTI2005690 | 0.012048061 | 110293 | 11 |
| TESTI2005692 | 0.00297212 | 158883 | 2 |
| TESTI2005703 | 0.001095278 | 182681 | 1 |
| TESTI2005720 | 0.001095278 | 127867 | 1 |
| TESTI2005731 | 0.050712559 | 96513 | 36 |
| TESTI2005739 | 0.007666948 | 109120 | 7 |
| TESTI2005742 | 0.008216161 | 88067 | 5 |
| TESTI2005743 | 0.076277759 | 176278 | 12 |
| TESTI2005759 | 0.179164756 | 148829 | 15 |
| TESTI2005767 | 0.06126452 | 136272 | 54 |
| TESTI2005775 | 0.001095278 | 224282 | 1 |
| TESTI2005784 | 0.056335086 | 152979 | 24 |
| TESTI2005788 | 0.036229542 | 127716 | 14 |
| TESTI2005827 | 0.001095278 | 240946 | 1 |
| TESTI2005835 | 0.013906175 | 108527 | 12 |
| TESTI2005860 | 0.013143339 | 138381 | 12 |
| TESTI2005892 | 0.00657167 | 105353 | 6 |
| TESTI2005908 | 0.106409059 | 134637 | 16 |
| TESTI2005911 | 0.001095278 | 254177 | 1 |
| TESTI2005933 | 0.001095278 | 236620 | 1 |
| TESTI2005937 | 0.036144182 | 111805 | 33 |
| TESTI2005948 | 0.001095278 | 189482 | 1 |
| TESTI2005979 | 0.001095278 | 217971 | 1 |
| TESTI2005981 | 0.005380019 | 104110 | 3 |
| TESTI2005982 | 0.002190557 | 179406 | 2 |
| TESTI2005984 | 0.001095278 | 215420 | 1 |
| TESTI2005986 | 0.002190557 | 114789 | 2 |
| TESTI2005987 | 0.002190557 | 175378 | 2 |
| TESTI2006007 | 0.012559224 | 241487 | 2 |
| TESTI2006008 | 0.007666948 | 161807 | 7 |
| TESTI2006015 | 0.00657167 | 122736 | 6. |
| TESTI2006016 | 0.004381113 | 88396 | 4 |
| TESTI2006035 | 0.019715009 | 119148 | 18 |
| TESTI2006040 | 0.048192243 | 133968 | 44 |
| TESTI2006041 | 0.00657167 | 159591 | 6 |
| TESTI2006045 | 0.001095278 | 214384 | 1 |
| TESTI2006051 | 0.395698054 | 83105 | 22 |
| TESTI2006067 | 0.003285835 | 113705 | 3 |
| TESTI2006083 | 0.042715852 | 138402 | 39 |
| TESTI2006097 | 0.015903283 | 143544 | 4 |
| TESTI2006109 | 0.001095278 | 251841 | 1 |
| TESTI2006111 | 0.008127008 | 133876 | 4 |
| TESTI2006120 | 0.034525396 | 17311 | 11 |
| TESTI2006125 | 0.001095278 | 236760 | 1 |
| TESTI2006179 | 0.001095278 | 129098 | 1 |
| TESTI2006209 | 0.001095278 | 241298 | 1 |
| TESTI2006210 | 0.019493236 | 111112 | 7 |
| TESTI2006212 | 0.001095278 | 167411 | 1 |
| TESTI2006218 | 0.00657167 | 119158 | 6 |
| TESTI2006238 | 0.009857504 | 148848 | 9 |
| TESTI2006255 | 0.001095278 | 229399 | 1 |
| TESTI2006258 | 0.016340114 | 46160 | 5 |
| TESTI2006278 | 0.001095278 | 222013 | 1 |
| TESTI2006333 | 0.001095278 | 229387 | 1 |
| TESTI2006341 | 0.008197134 | 119970 | 3 |
| TESTI2006360 | 0.001095278 | 59486 | 1 |
| TESTI2006383 | 0.041340921 | 124081 | 22 |
| TESTI2006409 | 0.002804738 | 186831 | 2 |
| TESTI2006425 | 0.001095278 | 243095 | 1 |
| TESTI2006437 | 0.004381113 | 112563 | 4 |
| TESTI2006453 | 0.19910624 | 102991 | 31 |
| TESTI2006457 | 0.001095278 | 282264 | 1 |
| TESTI2006460 | 0.005476391 | 158446 | 5 |
| TESTI2006465 | 0.001095278 | 122732 | 1 |
| TESTI2006483 | 0.002190557 | 73956 | 2 |
| TESTI2006499 | 0.001095278 | 236655 | 1 |
| TESTI2006523 | 0.003285835 | 223630 | 3 |
| TESTI2006543 | 0.005476391 | 189087 | 5 |
| TESTI2006565 | 0.001095278 | 112658 | 1 |
| TESTI2006572 | 0.023000843 | 147361 | 21 |
| TESTI2006588 | 0.309499013 | 58157 | 71 |
| TESTI2006615 | 0.036305904 | 102896 | 28 |
| TESTI2006617 | 0.002190557 | 217331 | 2 |
| TESTI2006624 | 0.002190557 | 150069 | 2 |
| TESTI2006628 | 0.013477238 | 90406 | 3 |
| TESTI2006633 | 0.006267794 | 182672 | 4 |
| TESTI2006643 | 0.001095278 | 227111 | 1 |
| TESTI2006648 | 0.002190557 | 158573 | 2 |
| TESTI2006659 | 0.001095278 | 221354 | 1 |
| TESTI2006660 | 0.001095278 | 239630 | 1 |
| TESTI2006665 | 0.004381113 | 131851 | 4 |
| TESTI2006667 | 0.001095278 | 102423 | 1 |
| TESTI2006677 | 0.002190557 | 262860 | 2 |
| TESTI2006720 | 0.002492434 | 220392 | 2 |
| TESTI2006735 | 0.001095278 | 154587 | 1 |
| TESTI2006744 | 0.002190557 | 102041 | 2 |
| TESTI2006748 | 0.048707779 | 126666 | 15 |
| TESTI2006774 | 0.002284523 | 20868 | 2 |
| TESTI2006853 | 0.001095278 | 246766 | 1 |
| TESTI2006866 | 0.001095278 | 229301 | 1 |
| TESTI2006872 | 0.030491939 | 151499 | 15 |
| TESTI2006877 | 0.012625446 | 180490 | 8 |
| TESTI2006879 | 0.001095278 | 221121 | 1 |
| TESTI2006894 | 0.002190557 | 173742 | 2 |
| TESTI2006940 | 0.004381113 | 136873 | 4 |
| TESTI2006955 | 0.001095278 | 118245 | 1 |
| TESTI2006978 | 0.001095278 | 249533 | 1 |
| TESTI2006979 | 0.001095278 | 237757 | 1 |
| TESTI2006990 | 0.001095278 | 229347 | 1 |
| TESTI2007040 | 0.003285835 | 13254 | 3 |
| TESTI2007074 | 0.001095278 | 171317 | 1 |
| TESTI2007078 | 0.001095278 | 55511 | 1 |
| TESTI2007113 | 0.001095278 | 111798 | 1 |
| TESTI2007152 | 0.001095278 | 224245 | 1 |
| TESTI2007163 | 0.002284523 | 198541 | 2 |
| TESTI2007183 | 0.001095278 | 64845 | 1 |
| TESTI2007211 | 0.050730246 | 45917 | 13 |
| TESTI2007220 | 0.001095278 | 217950 | 1 |
| TESTI2007234 | 0.001095278 | 227314 | 1 |
| TESTI2007311 | 0.043553326 | 42662 | 12 |
| TESTI2007320 | 0.001095278 | 208155 | 1 |
| TESTI2007346 | 0.001095278 | 117550 | 1 |
| TESTI2007358 | 0.001095278 | 116442 | 1 |
| TESTI2007402 | 0.001095278 | 232258 | 1 |
| TESTI2007405 | 0.001095278 | 258810 | 1 |
| TESTI2007407 | 0.05458856 | 155116 | 13 |
| TESTI2007422 | 0.005407291 | 3284 | 3 |
| TESTI2007452 | 0.001095278 | 191270 | 1 |
| TESTI2007464 | 0.041775597 | 51451 | 11 |
| TESTI2007466 | 0.001095278 | 141092 | 1 |
| TESTI2007480 | 0.005476391 | 127637 | 5 |
| TESTI2007490 | 0.001095278 | 94145 | 1 |
| TESTI2007502 | 0.001095278 | 200678 | 1 |
| TESTI2007524 | 0.001095278 | 125307 | 1 |
| TESTI2007533 | 0.001095278 | 121054 | 1 |
| TESTI2007576 | 0.001095278 | 175313 | 1 |
| TESTI2007613 | 0.002190557 | 84860 | 2 |
| TESTI2007657 | 0.004381113 | 62038 | 4 |
| TESTI2007685 | 0.001095278 | 260016 | 1 |
| TESTI2007692 | 0.001095278 | 106625 | 1 |
| TESTI2007749 | 0.001095278 | 244970 | 1 |
| TESTI2007750 | 0.001095278 | 141386 | 1 |
| TESTI2007808 | 0.002190557 | 104428 | 2 |
| TESTI2007814 | 0.001095278 | 173678 | 1 |
| TESTI2007829 | 0.001095278 | 245158 | 1 |
| TESTI2007864 | 0.002492434 | 105027 | 2 |
| TESTI2007867 | 0.001095278 | 105849 | 1 |
| TESTI2007872 | 0.001095278 | 102035 | 1 |
| TESTI2007906 | 0.001095278 | 193030 | 1 |
| TESTI2007922 | 0.042403927 | 141356 | 5 |
| TESTI2007951 | 0.001095278 | 32134 | 1 |
| TESTI2007972 | 0.001095278 | 254421 | 1 |
| TESTI2007973 | 0.001095278 | 258795 | 1 |
| TESTI2007998 | 0.105970221 | 89898 | 25 |
| TESTI2008014 | 0.002190557 | 158826 | 2 |
| TESTI2008020 | 0.043689936 | 48484 | 32 |
| TESTI2008032 | 0.001095278 | 237073 | 1 |
| TESTI2008033 | 0.001095278 | 240211 | 1 |
| TESTI2008046 | 0.001095278 | 203712 | 1 |
| TESTI2008081 | 0.003285835 | 116778 | 3 |
| TESTI2008099 | 0.001095278 | 111813 | 1 |
| TESTI2008139 | 0.007666948 | 149661 | 7 |
| TESTI2008144 | 0.001095278 | 227913 | 1 |
| TESTI2008161 | 0.001095278 | 224236 | 1 |
| TESTI2008189 | 0.001095278 | 141498 | 1 |
| TESTI2008204 | 0.001095278 | 210413 | 1 |
| TESTI2008219 | 0.001095278 | 201042 | 1 |
| TESTI2008233 | 0.012741189 | 179389 | 5 |
| TESTI2008234 | 0.001095278 | 204190 | 1 |
| TESTI2008237 | 0.001095278 | 37288 | 1 |
| TESTI2008240 | 0.67004871 | 54853 | 182 |
| TESTI2008278 | 0.013609072 | 148466 | 8 |
| TESTI2008320 | 0.001095278 | 167579 | 1 |
| TESTI2008331 | 0.001095278 | 215711 | 1 |
| TESTI2008343 | 0.001095278 | 142014 | 1 |
| TESTI2008387 | 0.013143339 | 161634 | 12 |
| TESTI2008389 | 0.001095278 | 209994 | 1 |
| TESTI2008394 | 0.004190774 | 181282 | 2 |
| TESTI2008405 | 0.032176504 | 193604 | 3 |
| TESTI2008420 | 0.001095278 | 181995 | 1 |
| TESTI2008425 | 0.00657167 | 91608 | 6 |
| TESTI2008440 | 0.001095278 | 220546 | 1 |
| TESTI2008567 | 0.003285835 | 138127 | 3 |
| TESTI2008595 | 0.002190557 | 121072 | 2 |
| TESTI2008621 | 0.016584682 | 116953 | 9 |
| TESTI2008636 | 0.001095278 | 140312 | 1 |
| TESTI2008657 | 0.002190557 | 202082 | 2 |
| TESTI2008684 | 0.001095278 | 213559 | 1 |
| TESTI2008699 | 0.017206957 | 134250 | 4 |
| TESTI2008715 | 0.001095278 | 175288 | 1 |
| TESTI2008762 | 0.007666948 | 143792 | 7 |
| TESTI2008774 | 0.001095278 | 65548 | 1 |
| TESTI2008786 | 0.001095278 | 196426 | 1 |
| TESTI2008822 | 0.001095278 | 19035 | 1 |
| TESTI2008835 | 0.015442408 | 180418 | 3 |
| TESTI2008840 | 0.008102819 | 172267 | 3 |
| TESTI2008847 | 0.003878833 | 134355 | 3 |
| TESTI2008884 | 0.002190557 | 185774 | 2 |
| TESTI2008901 | 0.001095278 | 176838 | 1 |
| TESTI2008929 | 0.001095278 | 87412 | 1 |
| TESTI2008946 | 0.001095278 | 205262 | 1 |
| TESTI2008960 | 0.002190557 | 232347 | 2 |
| TESTI2009018 | 0.00657167 | 78633 | 6 |
| TESTI2009091 | 0.001095278 | 224298 | 1 |
| TESTI2009097 | 0.001095278 | 193029 | 1 |
| TESTI2009136 | 0.001095278 | 202945 | 1 |
| TESTI2009185 | 0.001095278 | 219678 | 1 |
| TESTI2009190 | 0.001095278 | 150992 | 1 |
| TESTI2009217 | 0.002190557 | 180488 | 2 |
| TESTI2009239 | 0.002284523 | 156904 | 2 |
| TESTI2009325 | 0.001095278 | 240093 | 1 |
| TESTI2009388 | 0.008356906 | 103868 | 3 |
| TESTI2009390 | 0.001095278 | 197304 | 1 |
| TESTI2009400 | 0.001095278 | 229116 | 1 |
| TESTI2009402 | 0.018277016 | 174258 | 8 |
| TESTI2009412 | 0.004381113 | 166994 | 4 |
| TESTI2009423 | 0.010361611 | 1672 | 3 |
| TESTI2009447 | 0.001095278 | 170160 | 1 |
| TESTI2009462 | 0.001095278 | 167078 | 1 |
| TESTI2009474 | 0.082932325 | 151127 | 15 |
| TESTI2009477 | 0.004381113 | 111210 | 4 |
| TESTI2009497 | 0.00317679 | 64104 | 2 |
| TESTI2009502 | 0.037079445 | 10717 | 2 |
| TESTI2009511 | 0.002190557 | 237859 | 2 |
| TESTI2009520 | 0.223425027 | 89444 | 65 |
| TESTI2009544 | 0.001095278 | 102412 | 1 |
| TESTI2009545 | 0.002190557 | 229079 | 2 |
| TESTI2009551 | 0.001095278 | 213179 | 1 |
| TESTI2009577 | 0.094802209 | 112704 | 24 |
| TESTI2009585 | 0.001095278 | 233349 | 1 |
| TESTI2009588 | 0.001095278 | 21448 | 1 |
| TESTI2009683 | 0.002783555 | 49195 | 2 |
| TESTI2009727 | 0.001095278 | 142737 | 1 |
| TESTI2009739 | 0.001095278 | 194417 | 1 |
| TESTI2009785 | 0.116781786 | 97445 | 37 |
| TESTI2009812 | 0.003285835 | 173878 | 3 |
| TESTI2009835 | 0.023000843 | 132182 | 21 |
| TESTI2009853 | 0.001095278 | 264333 | 1 |
| TESTI2009935 | 0.002190557 | 157711 | 2 |
| TESTI2009977 | 0.006936741 | 145953 | 2 |
| TESTI2009987 | 0.032847288 | 107864 | 14 |
| TESTI2010009 | 0.002190557 | 74835 | 2 |
| TESTI2010154 | 0.007666948 | 32391 | 7 |
| TESTI2010180 | 0.004830234 | 46361 | 3 |
| TESTI2010239 | 0.001095278 | 190273 | 1 |
| TESTI2010280 | 0.001095278 | 127909 | 1 |
| TESTI2010354 | 0.001095278 | 134246 | 1 |
| TESTI2010369 | 0.048192243 | 133968 | 44 |
| TESTI2010396 | 0.004381113 | 114883 | 4 |
| TESTI2010400 | 0.001095278 | 139007 | 1 |
| TESTI2010409 | 0.001095278 | 144675 | 1 |
| TESTI2010513 | 0.42648461 | 20991 | 124 |
| TESTI2010572 | 0.001095278 | 127942 | 1 |
| TESTI2010587 | 0.001095278 | 102829 | 1 |
| TESTI2010591 | 0.001095278 | 139066 | 1 |
| TESTI2010617 | 0.024061147 | 133202 | 10 |
| TESTI2010682 | 0.001095278 | 37515 | 1 |
| TESTI2010724 | 0.044665455 | 146424 | 39 |
| TESTI2010732 | 0.001095278 | 120997 | 1 |
| TESTI2010734 | 0.005476391 | 119013 | 5 |
| TESTI2010755 | 0.001095278 | 260830 | 1 |
| TESTI2010793 | 0.001095278 | 263878 | 1 |
| TESTI2010806 | 0.001095278 | 11274 | 1 |
| TESTI2010833 | 0.001095278 | 248021 | 1 |
| TESTI2010872 | 0.001095278 | 227803 | 1 |
| TESTI2011009 | 0.001095278 | 97592 | 1 |
| TESTI2011020 | 0.001095278 | 112953 | 1 |
| TESTI2011028 | 0.001095278 | 248157 | 1 |
| TESTI2011033 | 0.001095278 | 245329 | 1 |
| TESTI2011254 | 0.001095278 | 104721 | 1 |
| TESTI2011286 | 0.012792762 | 160332 | 3 |
| TESTI2011294 | 0.001095278 | 233429 | 1 |
| TESTI2011314 | 0.001095278 | 214663 | 1 |
| TESTI2011315 | 0.001095278 | 232251 | 1 |
| TESTI2011394 | 0.001095278 | 154139 | 1 |
| TESTI2011407 | 0.002190557 | 205750 | 2 |
| TESTI2011448 | 0.002190557 | 99426 | 2 |
| TESTI2011480 | 0.001095278 | 116354 | 1 |
| TESTI2011549 | 0.002783555 | 138139 | 2 |
| TESTI2011575 | 0.007666948 | 14049 | 7 |
| TESTI2011605 | 0.046487668 | 78989 | 16 |
| TESTI2011612 | 0.001095278 | 11124 | 1 |
| TESTI2011665 | 0.001095278 | 183949 | 1 |
| TESTI2011680 | 0.022398562 | 91617 | 10 |
| TESTI2011683 | 0.005175192 | 86955 | 3 |
| TESTI2011712 | 0.008103168 | 135618 | 3 |
| TESTI2011750 | 0.001095278 | 141737 | 1 |
| TESTI2011840 | 0.003285835 | 212928 | 3 |
| TESTI2011846 | 0.003285835 | 119608 | 3 |
| TESTI2011915 | 0.001095278 | 107491 | 1 |
| TESTI2011928 | 0.002190557 | 224261 | 2 |
| TESTI2011971 | 0.003285835 | 131444 | 3 |
| TESTI2012019 | 0.043221937 | 143805 | 4 |
| TESTI2012050 | 0.001095278 | 262752 | 1 |
| TESTI2012090 | 0.001095278 | 188236 | 1 |
| TESTI2012095 | 0.002190557 | 163587 | 2 |
| TESTI2012104 | 0.017211705 | 166692 | 4 |
| TESTI2012152 | 0.001095278 | 60230 | 1 |
| TESTI2012155 | 0.026147931 | 119533 | 8 |
| TESTI2012171 | 0.017524452 | 53389 | 16 |
| TESTI2012207 | 0.002190557 | 88387 | 2 |
| TESTI2012231 | 0.001095278 | 272337 | 1 |
| TESTI2012244 | 0.001095278 | 65674 | 1 |
| TESTI2012289 | 0.019412667 | 122520 | 4 |
| TESTI2012308 | 0.001095278 | 143489 | 1 |
| TESTI2012356 | 0.057784621 | 145152 | 2 |
| TESTI2012387 | 0.001095278 | 138248 | 1 |
| TESTI2012394 | 0.006257955 | 115014 | 5 |
| TESTI2012444 | 0.003285835 | 141843 | 3 |
| TESTI2012511 | 0.039028571 | 111444 | 3 |
| TESTI2012528 | 0.001095278 | 117811 | 1 |
| TESTI2012544 | 0.001095278 | 134023 | 1 |
| TESTI2012550 | 0.003285835 | 83790 | 3 |
| TESTI2012571 | 0.016358454 | 122471 | 6 |
| TESTI2012592 | 0.001095278 | 53944 | 1 |
| TESTI2012617 | 0.001095278 | 109815 | 1 |
| TESTI2012628 | 0.008762226 | 134322 | 8 |
| TESTI2012778 | 0.90683466 | 65039 | 51 |
| TESTI2012812 | 0.002190557 | 104934 | 2 |
| TESTI2012834 | 0.001095278 | 119001 | 1 |
| TESTI2012835 | 0.001095278 | 105026 | 1 |
| TESTI2012861 | 0.001095278 | 101834 | 1 |
| TESTI2012900 | 0.001095278 | 106562 | 1 |
| TESTI2012915 | 0.001095278 | 92646 | 1 |
| TESTI2012922 | 0.001095278 | 103409 | 1 |
| TESTI2013001 | 0.001095278 | 45272 | 1 |
| TESTI2013012 | 0.001095278 | 197169 | 1 |
| TESTI2013053 | 0.002190557 | 131132 | 2 |
| TESTI2013144 | 0.001095278 | 59885 | 1 |
| TESTI2013163 | 0.001095278 | 129715 | 1 |
| TESTI2013169 | 0.001095278 | 126753 | 1 |
| TESTI2013212 | 0.001095278 | 134321 | 1 |
| TESTI2013231 | 0.084206555 | 114185 | 10 |
| TESTI2013257 | 0.001095278 | 122758 | 1 |
| TESTI2013264 | 0.006669369 | 100871 | 3 |
| TESTI2013268 | 0.002190557 | 142721 | 2 |
| TESTI2013295 | 0.062906448 | 86530 | 20 |
| TESTI2013322 | 0.001095278 | 120720 | 1 |
| TESTI2013381 | 0.001095278 | 141645 | 1 |
| TESTI2013382 | 0.001095278 | 191273 | 1 |
| TESTI2013401 | 0.001095278 | 78635 | 1 |
| TESTI2013427 | 0.001095278 | 114166 | 1 |
| TESTI2013468 | 0.00657167 | 117512 | 6 |
| TESTI2013497 | 0.002190557 | 112639 | 2 |
| TESTI2013545 | 0.001095278 | 67192 | 1 |
| TESTI2013546 | 0.001095278 | 182630 | 1 |
| TESTI2013566 | 0.001095278 | 283942 | 1 |
| TESTI2013601 | 0.004381113 | 150467 | 4 |
| TESTI2013604 | 0.001095278 | 154904 | 1 |
| TESTI2013607 | 0.001095278 | 152291 | 1 |
| TESTI2013610 | 0.001095278 | 161462 | 1 |
| TESTI2013671 | 0.005476391 | 127637 | 5 |
| TESTI2013691 | 0.002190557 | 233350 | 2 |
| TESTI2013699 | 0.002190557 | 173579 | 2 |
| TESTI2013737 | 0.001095278 | 161445 | 1 |
| TESTI2013767 | 0.001095278 | 139408 | 1 |
| TESTI2013832 | 0.001095278 | 108931 | 1 |
| TESTI2013873 | 0.012048061 | 120689 | 11 |
| TESTI2013889 | 0.001095278 | 119992 | 1 |
| TESTI2014007 | 0.001095278 | 50707 | 1 |
| TESTI2014036 | 0.001095278 | 45600 | 1 |
| TESTI2014097 | 0.001095278 | 143829 | 1 |
| TESTI2014160 | 0.001095278 | 241583 | 1 |
| TESTI2014248 | 0.001095278 | 87741 | 1 |
| TESTI2014254 | 0.001095278 | 141034 | 1 |
| TESTI2014269 | 0.001095278 | 42575 | 1 |
| TESTI2014274 | 0.001095278 | 5880 | 1 |
| TESTI2014318 | 0.005828436 | 152052 | 3 |
| TESTI2014324 | 0.001095278 | 148555 | 1 |
| TESTI2014339 | 0.001095278 | 134096 | 1 |
| TESTI2014362 | 0.001095278 | 143116 | 1 |
| TESTI2014385 | 0.001095278 | 135726 | 1 |
| TESTI2014415 | 0.001095278 | 139627 | 1 |
| TESTI2014439 | 0.001095278 | 111171 | 1 |
| TESTI2014474 | 0.001095278 | 120699 | 1 |
| TESTI2014505 | 0.008502787 | 56163 | 4 |
| TESTI2014577 | 0.001095278 | 155589 | 1 |
| TESTI2014578 | 0.001095278 | 134188 | 1 |
| TESTI2014716 | 0.001095278 | 75005 | 1 |
| TESTI2014780 | 0.005900234 | 159060 | 3 |
| TESTI2014800 | 0.00657167 | 161194 | 6 |
| TESTI2014838 | 0.126461488 | 108957 | 105 |
| TESTI2014843 | 0.001095278 | 180903 | 1 |
| TESTI2014979 | 0.001095278 | 159390 | 1 |
| TESTI2014988 | 0.001095278 | 108989 | 1 |
| TESTI2015042 | 0.003900017 | 129176 | 3 |
| TESTI2015092 | 0.05057616 | 93986 | 11 |
| TESTI2015105 | 0.002190557 | 158327 | 2 |
| TESTI2015180 | 0.001095278 | 218209 | 1 |
| TESTI2015213 | 0.326482171 | 56648 | 34 |
| TESTI2015246 | 0.014152782 | 29075 | 6 |
| TESTI2015249 | 0.002190557 | 219007 | 2 |
| TESTI2015327 | 0.001095278 | 68869 | 1 |
| TESTI2015331 | 0.003285835 | 112673 | 3 |
| TESTI2015335 | 0.00746277 | 104899 | 4 |
| TESTI2015350 | 0.001095278 | 122713 | 1 |
| TESTI2015375 | 0.001095278 | 250487 | 1 |
| TESTI2015437 | 0.04103138 | 103739 | 2 |
| TESTI2015553 | 0.053922974 | 99624 | 20 |
| TESTI2015590 | 0.003285835 | 104870 | 3 |
| TESTI2015610 | 0.00657167 | 111253 | 6 |
| TESTI2015626 | 0.509496239 | 72837 | 59 |
| TESTI2015647 | 0.001095278 | 101488 | 1 |
| TESTI2015670 | 0.040912856 | 120940 | 16 |
| TESTI2015675 | 0.004381113 | 77713 | 4 |
| TESTI2015710 | 0.001095278 | 90601 | 1 |
| TESTI2015752 | 0.019715009 | 156787 | 18 |
| TESTI2015830 | 0.001095278 | 142550 | 1 |
| TESTI2015914 | 0.001095278 | 94120 | 1 |
| TESTI2015938 | 0.001095278 | 83398 | 1 |
| TESTI2015947 | 0.001095278 | 163326 | 1 |
| TESTI2015962 | 0.042239159 | 124563 | 20 |
| TESTI2015987 | 0.001095278 | 163321 | 1 |
| TESTI2016033 | 0.096174573 | 105104 | 6 |
| TESTI2016046 | 0.001095278 | 105830 | 1 |
| TESTI2016101 | 0.038646991 | 142812 | 3 |
| TESTI2016162 | 0.001095278 | 171102 | 1 |
| TESTI2016197 | 0.001095278 | 194138 | 1 |
| TESTI2016278 | 0.002953393 | 153417 | 2 |
| TESTI2016298 | 0.004381113 | 159526 | 4 |
| TESTI2016331 | 0.003285835 | 197791 | 3 |
| TESTI2016337 | 0.001095278 | 46653 | 1 |
| TESTI2016410 | 0.001095278 | 247960 | 1 |
| TESTI2016421 | 0.002190557 | 116887 | 2 |
| TESTI2016509 | 0.001095278 | 100231 | 1 |
| TESTI2016568 | 0.001095278 | 135253 | 1 |
| TESTI2016583 | 0.009315576 | 151106 | 4 |
| TESTI2016599 | 0.001095278 | 122062 | 1 |
| TESTI2016629 | 0.001095278 | 151132 | 1 |
| TESTI2016663 | 0.001095278 | 128439 | 1 |
| TESTI2016667 | 0.001095278 | 102767 | 1 |
| TESTI2016688 | 0.003285835 | 191455 | 3 |
| TESTI2016701 | 0.002284523 | 8450 | 2 |
| TESTI2016758 | 0.001095278 | 135294 | 1 |
| TESTI2016848 | 0.001095278 | 169639 | 1 |
| TESTI2016863 | 0.001095278 | 173580 | 1 |
| TESTI2016888 | 0.001095278 | 81290 | 1 |
| TESTI2016896 | 0.003900017 | 47954 | 3 |
| TESTI2016922 | 0.001095278 | 235053 | 1 |
| TESTI2016942 | 0.002190557 | 173730 | 2 |
| TESTI2016950 | 0.001095278 | 153059 | 1 |
| TESTI2016996 | 0.001095278 | 222527 | 1 |
| TESTI2017017 | 0.003285835 | 120395 | 3 |
| TESTI2017028 | 0.001095278 | 283695 | 1 |
| TESTI2017035 | 0.003993983 | 195724 | 3 |
| TESTI2017069 | 0.001095278 | 232402 | 1 |
| TESTI2017089 | 0.001095278 | 163588 | 1 |
| TESTI2017102 | 0.001095278 | 251328 | 1 |
| TESTI2017107 | 0.002783555 | 48433 | 2 |
| TESTI2017113 | 0.001095278 | 133129 | 1 |
| TESTI2017125 | 0.002190557 | 234307 | 2 |
| TESTI2017256 | 0.001095278 | 215307 | 1 |
| TESTI2017305 | 0.001095278 | 107509 | 1 |
| TESTI2017311 | 0.001095278 | 111156 | 1 |
| TESTI2017396 | 0.004381113 | 111912 | 4 |
| TESTI2017507 | 0.001095278 | 48611 | 1 |
| TESTI2017537 | 0.001095278 | 101981 | 1 |
| TESTI2017582 | 0.001095278 | 171225 | 1 |
| TESTI2017584 | 0.001095278 | 147601 | 1 |
| TESTI2017645 | 0.005034905 | 159528 | 3 |
| TESTI2017727 | 0.002190557 | 191380 | 2 |
| TESTI2017740 | 0.001095278 | 167063 | 1 |
| TESTI2017775 | 0.001095278 | 111840 | 1 |
| TESTI2017816 | 0.002190557 | 175887 | 2 |
| TESTI2017832 | 0.001095278 | 113545 | 1 |
| TESTI2017923 | 0.00657167 | 150245 | 6 |
| TESTI2017932 | 0.001095278 | 173679 | 1 |
| TESTI2017951 | 0.001095278 | 140586 | 1 |
| TESTI2017954 | 0.252934652 | 65617 | 33 |
| TESTI2018014 | 0.043470488 | 83984 | 15 |
| TESTI2018031 | 0.001095278 | 155611 | 1 |
| TESTI2018035 | 0.001095278 | 19260 | 1 |
| TESTI2018060 | 0.001095278 | 171183 | 1 |
| TESTI2018083 | 0.001095278 | 191387 | 1 |
| TESTI2018178 | 0.001095278 | 166338 | 1 |
| TESTI2018221 | 0.001095278 | 259105 | 1 |
| TESTI2018276 | 0.001095278 | 139071 | 1 |
| TESTI2018290 | 0.002190557 | 164425 | 2 |
| TESTI2018335 | 0.005476391 | 160170 | 5 |
| TESTI2018337 | 0.001095278 | 184601 | 1 |
| TESTI2018368 | 0.001095278 | 149863 | 1 |
| TESTI2018421 | 0.007136844 | 163573 | 2 |
| TESTI2018428 | 0.001095278 | 245189 | 1 |
| TESTI2018462 | 0.059292988 | 152539 | 23 |
| TESTI2018475 | 0.001095278 | 141222 | 1 |
| TESTI2018482 | 0.001095278 | 155532 | 1 |
| TESTI2018521 | 0.001095278 | 86141 | 1 |
| TESTI2018565 | 0.002190557 | 152426 | 2 |
| TESTI2018581 | 0.002190557 | 163356 | 2 |
| TESTI2018611 | 0.159500105 | 46915 | 8 |
| TESTI2018687 | 0.001095278 | 167033 | 1 |
| TESTI2018697 | 0.001095278 | 151242 | 1 |
| TESTI2018838 | 0.001095278 | 194264 | 1 |
| TESTI2018867 | 0.058840224 | 119609 | 43 |
| TESTI2018941 | 0.003285835 | 124398 | 3 |
| TESTI2018957 | 0.003285835 | 183887 | 3 |
| TESTI2018974 | 0.001095278 | 58580 | 1 |
| TESTI2018975 | 0.001095278 | 201723 | 1 |
| TESTI2018997 | 0.080451144 | 157343 | 10 |
| TESTI2019042 | 0.001095278 | 116454 | 1 |
| TESTI2019077 | 0.002492434 | 173691 | 2 |
| TESTI2019228 | 0.001095278 | 173686 | 1 |
| TESTI2019257 | 0.001095278 | 180025 | 1 |
| TESTI2019280 | 0.001095278 | 153818 | 1 |
| TESTI2019282 | 0.001095278 | 164899 | 1 |
| TESTI2019308 | 0.001095278 | 70208 | 1 |
| TESTI2019336 | 0.001095278 | 161950 | 1 |
| TESTI2019352 | 0.001095278 | 159616 | 1 |
| TESTI2019406 | 0.001095278 | 99199 | 1 |
| TESTI2019430 | 0.001095278 | 175901 | 1 |
| TESTI2019455 | 0.001095278 | 23776 | 1 |
| TESTI2019481 | 0.001095278 | 180093 | 1 |
| TESTI2019488 | 0.001095278 | 182698 | 1 |
| TESTI2019501 | 0.001095278 | 128727 | 1 |
| TESTI2019648 | 0.001095278 | 129855 | 1 |
| TESTI2019669 | 0.001095278 | 120268 | 1 |
| TESTI2019697 | 0.001095278 | 9406 | 1 |
| TESTI2019729 | 0.004216085 | 106807 | 2 |
| TESTI2019760 | 0.003681678 | 54172 | 3 |
| TESTI2019787 | 0.001095278 | 194283 | 1 |
| TESTI2019794 | 0.001095278 | 123800 | 1 |
| TESTI2019854 | 0.001095278 | 169687 | 1 |
| TESTI2019859 | 0.001095278 | 46525 | 1 |
| TESTI2019860 | 0.001095278 | 151240 | 1 |
| TESTI2019872 | 0.001095278 | 151940 | 1 |
| TESTI2019911 | 0.003285835 | 175311 | 3 |
| TESTI2019917 | 0.001095278 | 180759 | 1 |
| TESTI2019975 | 0.001095278 | 216634 | 1 |
| TESTI2019998 | 0.003285835 | 166992 | 3 |
| TESTI2020012 | 0.007331636 | 77150 | 2 |
| TESTI2020026 | 0.012901654 | 188234 | 2 |
| TESTI2020029 | 0.001095278 | 204184 | 1 |
| TESTI2020071 | 0.002190557 | 13143 | 2 |
| TESTI2020084 | 0.001095278 | 79302 | 1 |
| TESTI2020176 | 0.007666948 | 109120 | 7 |
| TESTI2020265 | 0.001095278 | 224386 | 1 |
| TESTI2020283 | 0.001095278 | 239052 | 1 |
| TESTI2020316 | 0.001095278 | 224231 | 1 |
| TESTI2020344 | 0.001095278 | 199910 | 1 |
| TESTI2020379 | 0.001095278 | 245535 | 1 |
| TESTI2020426 | 0.001095278 | 131163 | 1 |
| TESTI2020445 | 0.001095278 | 133916 | 1 |
| TESTI2020510 | 0.023163784 | 148824 | 8 |
| TESTI2020515 | 0.001095278 | 237627 | 1 |
| TESTI2020525 | 0.005133561 | 81411 | 2 |
| TESTI2020579 | 0.004381113 | 170062 | 4 |
| TESTI2020717 | 0.001095278 | 214199 | 1 |
| TESTI2020840 | 0.010253348 | 101332 | 9 |
| TESTI2020871 | 0.00309368 | 179808 | 2 |
| TESTI2020905 | 0.001095278 | 119474 | 1 |
| TESTI2020906 | 0.004381113 | 32393 | 4 |
| TESTI2020946 | 0.013689737 | 234487 | 2 |
| TESTI2020956 | 0.03893756 | 210067 | 3 |
| TESTI2020981 | 0.001095278 | 240059 | 1 |
| TESTI2020999 | 0.001095278 | 185893 | 1 |
| TESTI2021003 | 0.001095278 | 30056 | 1 |
| TESTI2021038 | 0.00396007 | 82744 | 3 |
| TESTI2021057 | 0.001095278 | 204292 | 1 |
| TESTI2021077 | 0.001095278 | 174579 | 1 |
| TESTI2021112 | 0.001095278 | 171096 | 1 |
| TESTI2021114 | 0.002190557 | 134348 | 2 |
| TESTI2021116 | 0.001095278 | 109145 | 1 |
| TESTI2021118 | 0.015619607 | 85701 | 2 |
| TESTI2021122 | 0.001095278 | 96941 | 1 |
| TESTI2021124 | 0.029317298 | 149185 | 14 |
| TESTI2021138 | 0.003285835 | 161757 | 3 |
| TESTI2021297 | 0.02440723 | 175070 | 5 |
| TESTI2021315 | 0.008268012 | 166879 | 4 |
| TESTI2021358 | 0.004951794 | 137372 | 3 |
| TESTI2021425 | 0.097323164 | 102390 | 18 |
| TESTI2021447 | 0.006462625 | 153342 | 5 |
| TESTI2021463 | 0.001095278 | 149350 | 1 |
| TESTI2021531 | 0.010014365 | 23488 | 4 |
| TESTI2021599 | 0.001095278 | 132409 | 1 |
| TESTI2021637 | 0.001095278 | 121497 | 1 |
| TESTI2021654 | 0.017144414 | 107901 | 5 |
| TESTI2021667 | 0.001095278 | 174138 | 1 |
| TESTI2021736 | 0.001095278 | 49506 | 1 |
| TESTI2021785 | 0.001095278 | 196646 | 1 |
| TESTI2021819 | 0.001095278 | 258340 | 1 |
| TESTI2021851 | 0.008855938 | 141718 | 3 |
| TESTI2021895 | 0.001095278 | 106690 | 1 |
| TESTI2021911 | 0.001095278 | 71636 | 1 |
| TESTI2021939 | 0.001095278 | 223310 | 1 |
| TESTI2021999 | 0.001095278 | 213330 | 1 |
| TESTI2022010 | 0.001095278 | 264922 | 1 |
| TESTI2022023 | 0.001095278 | 223315 | 1 |
| TESTI2022065 | 0.001095278 | 223319 | 1 |
| TESTI2022086 | 0.001095278 | 206702 | 1 |
| TESTI2022179 | 0.015953069 | 133860 | 10 |
| TESTI2022203 | 0.001095278 | 52765 | 1 |
| TESTI2022246 | 0.001095278 | 171062 | 1 |
| TESTI2022323 | 0.001095278 | 182755 | 1 |
| TESTI2022338 | 0.001095278 | 31806 | 1 |
| TESTI2022400 | 0.001095278 | 114252 | 1 |
| TESTI2022462 | 0.001095278 | 203695 | 1 |
| TESTI2022495 | 0.001095278 | 196417 | 1 |
| TESTI2022623 | 0.001095278 | 153631 | 1 |
| TESTI2022649 | 0.001095278 | 183561 | 1 |
| TESTI2022652 | 0.003285835 | 105831 | 3 |
| TESTI2022738 | 0.001095278 | 108833 | 1 |
| TESTI2022798 | 0.001095278 | 49797 | 1 |
| TESTI2022812 | 0.002190557 | 129177 | 2 |
| TESTI2022814 | 0.001095278 | 123238 | 1 |
| TESTI2022874 | 0.001095278 | 89841 | 1 |
| TESTI2022940 | 0.001095278 | 167158 | 1 |
| TESTI2022952 | 0.001095278 | 17684 | 1 |
| TESTI2022960 | 0.018642681 | 60769 | 10 |
| TESTI2023025 | 0.013317229 | 175687 | 2 |
| TESTI2023033 | 0.012203146 | 45374 | 7 |
| TESTI2023053 | 0.001095278 | 167080 | 1 |
| TESTI2023085 | 0.017524452 | 152068 | 16 |
| TESTI2023192 | 0.001095278 | 136192 | 1 |
| TESTI2023194 | 0.001095278 | 143912 | 1 |
| TESTI2023214 | 0.002190557 | 111200 | 2 |
| TESTI2023254 | 0.001095278 | 106460 | 1 |
| TESTI2023386 | 0.002190557 | 155436 | 2 |
| TESTI2023414 | 0.041164425 | 135462 | 18 |
| TESTI2023427 | 0.001095278 | 123050 | 1 |
| TESTI2023436 | 0.001095278 | 132838 | 1 |
| TESTI2023599 | 0.001095278 | 191367 | 1 |
| TESTI2023752 | 0.002190557 | 167409 | 2 |
| TESTI2023800 | 0.003285835 | 83118 | 3 |
| TESTI2023861 | 0.001095278 | 192415 | 1 |
| TESTI2023903 | 0.001095278 | 79123 | 1 |
| TESTI2023942 | 0.012048061 | 133907 | 11 |
| TESTI2023947 | 0.001095278 | 227045 | 1 |
| TESTI2023951 | 0.001095278 | 167756 | 1 |
| TESTI2023968 | 0.002190557 | 247379 | 2 |
| TESTI2024009 | 0.001095278 | 189770 | 1 |
| TESTI2024090 | 0.004077237 | 73492 | 2 |
| TESTI2024125 | 0.00470917 | 204928 | 2 |
| TESTI2024153 | 0.001095278 | 204475 | 1 |
| TESTI2024192 | 0.001095278 | 175641 | 1 |
| TESTI2024267 | 0.001095278 | 110644 | 1 |
| TESTI2024283 | 0.00891636 | 155749 | 6 |
| TESTI2024299 | 0.001095278 | 195929 | 1 |
| TESTI2024419 | 0.001095278 | 273933 | 1 |
| TESTI2024422 | 0.001095278 | 140255 | 1 |
| TESTI2024443 | 0.001095278 | 173581 | 1 |
| TESTI2024446 | 0.001095278 | 130697 | 1 |
| TESTI2024473 | 0.001095278 | 171364 | 1 |
| TESTI2024476 | 0.008762226 | 173577 | 8 |
| TESTI2024498 | 0.001095278 | 204291 | 1 |
| TESTI2024560 | 0.003285835 | 180181 | 3 |
| TESTI2024567 | 0.001095278 | 132071 | 1 |
| TESTI2024586 | 0.001095278 | 216631 | 1 |
| TESTI2024598 | 0.002804738 | 115255 | 2 |
| TESTI2024641 | 0.001095278 | 171370 | 1 |
| TESTI2024648 | 0.001095278 | 175796 | 1 |
| TESTI2024718 | 0.012048061 | 149593 | 11 |
| TESTI2024744 | 0.001095278 | 129439 | 1 |
| TESTI2024844 | 0.146257055 | 38285 | 11 |
| TESTI2024864 | 0.006067616 | 128611 | 3 |
| TESTI2024885 | 0.01260011 | 77395 | 2 |
| TESTI2024936 | 0.001095278 | 114388 | 1 |
| TESTI2024978 | 0.001095278 | 163403 | 1 |
| TESTI2024999 | 0.001095278 | 124874 | 1 |
| TESTI2025006 | 0.001095278 | 65057 | 1 |
| TESTI2025013 | 0.047072946 | 117261 | 3 |
| TESTI2025022 | 0.002190557 | 111212 | 2 |
| TESTI2025063 | 0.002190557 | 133970 | 2 |
| TESTI2025117 | 0.003285835 | 171381 | 3 |
| TESTI2025144 | 0.001095278 | 175765 | 1 |
| TESTI2025161 | 0.001095278 | 165601 | 1 |
| TESTI2025174 | 0.001095278 | 144627 | 1 |
| TESTI2025229 | 0.001095278 | 161885 | 1 |
| TESTI2025269 | 0.001095278 | 51834 | 1 |
| TESTI2025365 | 0.001095278 | 156562 | 1 |
| TESTI2025403 | 0.001095278 | 79870 | 1 |
| TESTI2025409 | 0.001095278 | 224291 | 1 |
| TESTI2025422 | 0.067506217 | 174323 | 6 |
| TESTI2025448 | 0.005476391 | 77390 | 5 |
| TESTI2025454 | 0.014876357 | 131140 | 5 |
| TESTI2025486 | 0.001095278 | 257479 | 1 |
| TESTI2025499 | 0.02693841 | 109167 | 3 |
| TESTI2025546 | 0.048276743 | 117193 | 33 |
| TESTI2025582 | 0.003285835 | 122865 | 3 |
| TESTI2025609 | 0.001095278 | 70036 | 1 |
| TESTI2025656 | 0.001095278 | 152240 | 1 |
| TESTI2025757 | 0.009881811 | 129887 | 5 |
| TESTI2025791 | 0.001095278 | 179882 | 1 |
| TESTI2025846 | 0.008230586 | 91610 | 6 |
| TESTI2025872 | 0.038646514 | 171066 | 4 |
| TESTI2025879 | 0.001095278 | 146300 | 1 |
| TESTI2025911 | 0.001095278 | 52035 | 1 |
| TESTI2025920 | 0.001095278 | 32336 | 1 |
| TESTI2025924 | 0.001095278 | 165985 | 1 |
| TESTI2026014 | 0.001095278 | 145731 | 1 |
| TESTI2026024 | 0.007037402 | 107454 | 2 |
| TESTI2026064 | 0.001095278 | 179390 | 1 |
| TESTI2026077 | 0.001095278 | 44170 | 1 |
| TESTI2026104 | 0.001095278 | 48494 | 1 |
| TESTI2026116 | 0.01123402 | 23005 | 6 |
| TESTI2026134 | 0.001095278 | 100191 | 1 |
| TESTI2026168 | 0.001095278 | 260944 | 1 |
| TESTI2026196 | 0.001095278 | 180712 | 1 |
| TESTI2026215 | 0.024190153 | 115720 | 6 |
| TESTI2026218 | 0.001095278 | 187766 | 1 |
| TESTI2026233 | 0.001095278 | 196891 | 1 |
| TESTI2026291 | 0.001095278 | 276472 | 1 |
| TESTI2026294 | 0.001095278 | 181622 | 1 |
| TESTI2026401 | 0.001095278 | 241995 | 1 |
| TESTI2026403 | 0.002190557 | 192368 | 2 |
| TESTI2026453 | 0.001095278 | 284444 | 1 |
| TESTI2026461 | 0.001095278 | 179353 | 1 |
| TESTI2026491 | 0.001095278 | 175725 | 1 |
| TESTI2026505 | 0.002190557 | 220206 | 2 |
| TESTI2026515 | 0.001095278 | 185796 | 1 |
| TESTI2026525 | 0.03851191 | 187000 | 9 |
| TESTI2026534 | 0.002804738 | 182822 | 2 |
| TESTI2026537 | 0.001095278 | 149806 | 1 |
| TESTI2026589 | 0.001095278 | 155025 | 1 |
| TESTI2026597 | 0.001095278 | 111069 | 1 |
| TESTI2026605 | 0.00657167 | 173774 | 6 |
| TESTI2026647 | 0.014180569 | 159741 | 4 |
| TESTI2026674 | 0.002190557 | 88778 | 2 |
| TESTI2026794 | 0.001095278 | 166957 | 1 |
| TESTI2026824 | 0.001095278 | 175703 | 1 |
| TESTI2026854 | 0.001095278 | 175710 | 1 |
| TESTI2026925 | 0.005476391 | 107906 | 5 |
| TESTI2026936 | 0.002783555 | 135310 | 2 |
| TESTI2026957 | 0.001095278 | 154688 | 1 |
| TESTI2027013 | 0.001095278 | 213630 | 1 |
| TESTI2027019 | 0.001095278 | 210487 | 1 |
| TESTI2027108 | 0.001095278 | 180804 | 1 |
| TESTI2027165 | 0.00309368 | 52326 | 2 |
| TESTI2027171 | 0.002190557 | 204407 | 2 |
| TESTI2027173 | 0.001095278 | 55277 | 1 |
| TESTI2027179 | 0.001095278 | 67759 | 1 |
| TESTI2027185 | 0.001095278 | 203424 | 1 |
| TESTI2027206 | 0.031763069 | 120175 | 29 |
| TESTI2027238 | 0.001095278 | 203035 | 1 |
| TESTI2027239 | 0.001095278 | 75184 | 1 |
| TESTI2027243 | 0.001095278 | 278341 | 1 |
| TESTI2027271 | 0.031732543 | 39383 | 6 |
| TESTI2027296 | 0.001095278 | 158781 | 1 |
| TESTI2027378 | 0.001095278 | 212458 | 1 |
| TESTI2027411 | 0.001095278 | 15291 | 1 |
| TESTI2027450 | 0.001095278 | 153670 | 1 |
| TESTI2027496 | 0.017834284 | 153894 | 7 |
| TESTI2027503 | 0.001095278 | 143165 | 1 |
| TESTI2027562 | 0.001095278 | 215240 | 1 |
| TESTI2027615 | 0.001095278 | 226842 | 1 |
| TESTI2027645 | 0.001095278 | 27887 | 1 |
| TESTI2027730 | 0.003285835 | 201524 | 3 |
| TESTI2027736 | 0.001095278 | 143147 | 1 |
| TESTI2027791 | 0.001095278 | 276017 | 1 |
| TESTI2027820 | 0.001095278 | 256536 | 1 |
| TESTI2027828 | 0.001095278 | 72623 | 1 |
| TESTI2027840 | 0.002190557 | 179260 | 2 |
| TESTI2027909 | 0.002492434 | 155575 | 2 |
| TESTI2028098 | 0.001095278 | 215392 | 1 |
| TESTI2028151 | 0.00657167 | 182680 | 6 |
| TESTI2028242 | 0.001095278 | 224287 | 1 |
| TESTI2028253 | 0.001095278 | 130573 | 1 |
| TESTI2028254 | 0.004381113 | 142422 | 4 |
| TESTI2028269 | 0.001095278 | 200642 | 1 |
| TESTI2028290 | 0.001095278 | 272910 | 1 |
| TESTI2028296 | 0.067948504 | 104221 | 8 |
| TESTI2028384 | 0.001095278 | 31634 | 1 |
| TESTI2028426 | 0.001095278 | 244211 | 1 |
| TESTI2028488 | 0.001095278 | 208414 | 1 |
| TESTI2028523 | 0.00657167 | 140902 | 6 |
| TESTI2028583 | 0.001095278 | 140579 | 1 |
| TESTI2028613 | 0.001095278 | 261525 | 1 |
| TESTI2028659 | 0.00555852 | 114257 | 4 |
| TESTI2028662 | 0.001095278 | 232286 | 1 |
| TESTI2028670 | 0.001095278 | 123155 | 1 |
| TESTI2028705 | 0.001095278 | 222330 | 1 |
| TESTI2028776 | 0.001095278 | 154075 | 1 |
| TESTI2028811 | 0.001095278 | 226149 | 1 |
| TESTI2028891 | 0.001095278 | 48445 | 1 |
| TESTI2028985 | 0.001095278 | 139159 | 1 |
| TESTI2028997 | 0.001095278 | 221766 | 1 |
| TESTI2029131 | 0.001095278 | 158596 | 1 |
| TESTI2029162 | 0.001095278 | 191158 | 1 |
| TESTI2029196 | 0.001095278 | 145689 | 1 |
| TESTI2029201 | 0.002190557 | 185802 | 2 |
| TESTI2029227 | 0.028226519 | 104191 | 13 |
| TESTI2029244 | 0.001095278 | 161994 | 1 |
| TESTI2029249 | 0.001095278 | 149249 | 1 |
| TESTI2029252 | 0.001095278 | 157399 | 1 |
| TESTI2029259 | 0.050627645 | 14429 | 9 |
| TESTI2029264 | 0.001095278 | 46713 | 1 |
| TESTI2029470 | 0.001095278 | 130184 | 1 |
| TESTI2029672 | 0.001095278 | 233339 | 1 |
| TESTI2029785 | 0.002492434 | 145330 | 2 |
| TESTI2029880 | 0.001095278 | 240165 | 1 |
| TESTI2030136 | 0.001095278 | 180810 | 1 |
| TESTI2030322 | 0.001095278 | 139047 | 1 |
| TESTI2030336 | 0.002492434 | 192451 | 2 |
| TESTI2030342 | 0.001095278 | 265648 | 1 |
| TESTI2030519 | 0.001095278 | 271936 | 1 |
| TESTI2030554 | 0.00468299 | 128614 | 4 |
| TESTI2030556 | 0.001095278 | 202871 | 1 |
| TESTI2030728 | 0.001095278 | 175724 | 1 |
| TESTI2030754 | 0.001095278 | 180868 | 1 |
| TESTI2030770 | 0.001095278 | 48386 | 1 |
| TESTI2030860 | 0.001095278 | 171343 | 1 |
| TESTI2030901 | 0.001095278 | 230690 | 1 |
| TESTI2030917 | 0.001095278 | 128961 | 1 |
| TESTI2030930 | 0.001095278 | 47069 | 1 |
| TESTI2031006 | 0.001095278 | 37087 | 1 |
| TESTI2031007 | 0.005162676 | 118782 | 4 |
| TESTI2031060 | 0.001095278 | 43865 | 1 |
| TESTI2031129 | 0.002190557 | 173639 | 2 |
| TESTI2031356 | 0.001095278 | 139063 | 1 |
| TESTI2031418 | 0.001095278 | 171355 | 1 |
| TESTI2031504 | 0.001095278 | 109724 | 1 |
| TESTI2031529 | 0.001095278 | 40060 | 1 |
| TESTI2031687 | 0.001095278 | 36807 | 1 |
| TESTI2031760 | 0.001095278 | 180445 | 1 |
| TESTI2031809 | 0.001095278 | 133461 | 1 |
| TESTI2031919 | 0.001095278 | 52042 | 1 |
| TESTI2031935 | 0.001095278 | 155727 | 1 |
| TESTI2031986 | 0.002190557 | 133668 | 2 |
| TESTI2032044 | 0.00657167 | 102026 | 6 |
| TESTI2032067 | 0.001095278 | 167010 | 1 |
| TESTI2032079 | 0.001095278 | 226167 | 1 |
| TESTI2032643 | 0.013779115 | 228702 | 3 |
| TESTI2032681 | 0.001095278 | 141395 | 1 |
| TESTI2032761 | 0.001095278 | 242921 | 1 |
| TESTI2032768 | 0.002190557 | 179579 | 2 |
| TESTI2032774 | 0.001095278 | 259279 | 1 |
| TESTI2032828 | 0.007666948 | 172468 | 7 |
| TESTI2033031 | 0.005445826 | 56053 | 4 |
| TESTI2033242 | 0.001095278 | 108152 | 1 |
| TESTI2033254 | 0.001095278 | 74269 | 1 |
| TESTI2033300 | 0.001095278 | 122498 | 1 |
| TESTI2033395 | 0.002190557 | 135127 | 2 |
| TESTI2033441 | 0.152694729 | 109611 | 29 |
| TESTI2033453 | 0.001095278 | 119141 | 1 |
| TESTI2033505 | 0.001095278 | 64173 | 1 |
| TESTI2033520 | 0.001095278 | 222311 | 1 |
| TESTI2033641 | 0.001095278 | 223615 | 1 |
| TESTI2033710 | 0.001095278 | 220552 | 1 |
| TESTI2033905 | 0.024096122 | 131838 | 22 |
| TESTI2034187 | 0.001095278 | 245631 | 1 |
| TESTI2034243 | 0.001095278 | 222485 | 1 |
| TESTI2034249 | 0.042877709 | 73491 | 33 |
| TESTI2034251 | 0.017834284 | 153894 | 7 |
| TESTI2034307 | 0.002492434 | 136584 | 2 |
| TESTI2034357 | 0.001095278 | 19626 | 1 |
| TESTI2034401 | 0.001095278 | 273751 | 1 |
| TESTI2034407 | 0.001095278 | 54245 | 1 |
| TESTI2034506 | 0.001095278 | 107914 | 1 |
| TESTI2034520 | 0.001095278 | 207784 | 1 |
| TESTI2034718 | 0.001095278 | 134249 | 1 |
| TESTI2034730 | 0.001095278 | 115798 | 1 |
| TESTI2034749 | 0.001095278 | 189661 | 1 |
| TESTI2034767 | 0.001095278 | 187767 | 1 |
| TESTI2034774 | 0.001095278 | 189625 | 1 |
| TESTI2034777 | 0.001095278 | 192439 | 1 |
| TESTI2034785 | 0.001095278 | 207622 | 1 |
| TESTI2034799 | 0.001095278 | 189618 | 1 |
| TESTI2034847 | 0.001095278 | 170706 | 1 |
| TESTI2034913 | 0.001095278 | 206879 | 1 |
| TESTI2034916 | 0.022042071 | 104310 | 8 |
| TESTI2034931 | 0.001095278 | 74922 | 1 |
| TESTI2034940 | 0.643197739 | 34012 | 81 |
| TESTI2034953 | 0.001095278 | 142667 | 1 |
| TESTI2034997 | 0.001095278 | 170695 | 1 |
| TESTI2035003 | 0.002284523 | 58322 | 2 |
| TESTI2035078 | 0.001095278 | 167320 | 1 |
| TESTI2035084 | 0.001095278 | 138239 | 1 |
| TESTI2035107 | 0.001095278 | 175290 | 1 |
| TESTI2035121 | 0.001095278 | 191210 | 1 |
| TESTI2035124 | 0.001095278 | 170712 | 1 |
| TESTI2035183 | 0.002190557 | 68871 | 2 |
| TESTI2035236 | 0.006499784 | 77802 | 4 |
| TESTI2035262 | 0.001095278 | 207770 | 1 |
| TESTI2035309 | 0.008232122 | 152686 | 3 |
| TESTI2035437 | 0.042403927 | 141356 | 5 |
| TESTI2035502 | 0.012741189 | 179389 | 5 |
| TESTI2035688 | 0.001095278 | 9973 | 1 |
| TESTI2035775 | 0.001095278 | 238937 | 1 |
| TESTI2035793 | 0.002190557 | 19057 | 2 |
| TESTI2035796 | 0.011116458 | 205584 | 6 |
| TESTI2035898 | 0.001095278 | 277946 | 1 |
| TESTI2035962 | 0.001095278 | 199700 | 1 |
| TESTI2035981 | 0.001095278 | 217343 | 1 |
| TESTI2035997 | 0.001095278 | 208280 | 1 |
| TESTI2036114 | 0.001095278 | 49273 | 1 |
| TESTI2036129 | 0.001095278 | 170707 | 1 |
| TESTI2036285 | 0.001095278 | 199673 | 1 |
| TESTI2036288 | 0.001095278 | 180139 | 1 |
| TESTI2036513 | 0.001095278 | 229885 | 1 |
| TESTI2036589 | 0.001095278 | 235625 | 1 |
| TESTI2036650 | 0.002783555 | 23897 | 2 |
| TESTI2036684 | 0.001095278 | 221277 | 1 |
| TESTI2036691 | 0.007136844 | 115567 | 2 |
| TESTI2036736 | 0.001095278 | 200647 | 1 |
| TESTI2036822 | 0.001095278 | 183034 | 1 |
| TESTI2036833 | 0.001095278 | 138314 | 1 |
| TESTI2036913 | 0.008256356 | 48491 | 6 |
| TESTI2036922 | 0.001095278 | 237809 | 1 |
| TESTI2036951 | 0.002190557 | 120405 | 2 |
| TESTI2036965 | 0.001095278 | 104134 | 1 |
| TESTI2036969 | 0.046634682 | 122870 | 22 |
| TESTI2037002 | 0.001095278 | 141140 | 1 |
| TESTI2037055 | 0.001095278 | 220318 | 1 |
| TESTI2037081 | 0.002190557 | 157400 | 2 |
| TESTI2037085 | 0.001095278 | 218010 | 1 |
| TESTI2037103 | 0.001095278 | 39845 | 1 |
| TESTI2037106 | 0.001095278 | 132156 | 1 |
| TESTI2037111 | 0.001095278 | 220289 | 1 |
| TESTI2037209 | 0.001095278 | 220295 | 1 |
| TESTI2037255 | 0.001095278 | 221426 | 1 |
| TESTI2037359 | 0.001095278 | 125665 | 1 |
| TESTI2037375 | 0.001095278 | 156597 | 1 |
| TESTI2037382 | 0.001095278 | 229818 | 1 |
| TESTI2037534 | 0.001095278 | 111203 | 1 |
| TESTI2037569 | 0.001095278 | 144342 | 1 |
| TESTI2037572 | 0.001095278 | 213320 | 1 |
| TESTI2037643 | 0.001095278 | 170851 | 1 |
| TESTI2037657 | 0.010952783 | 158273 | 10 |
| TESTI2037673 | 0.001095278 | 141842 | 1 |
| TESTI2037723 | 0.001095278 | 152070 | 1 |
| TESTI2037819 | 0.001095278 | 111918 | 1 |
| TESTI2037830 | 0.001095278 | 175531 | 1 |
| TESTI2037845 | 0.003285835 | 175262 | 3 |
| TESTI2037877 | 0.001095278 | 161682 | 1 |
| TESTI2037976 | 0.001095278 | 258240 | 1 |
| TESTI2038048 | 0.001095278 | 45046 | 1 |
| TESTI2038065 | 0.001095278 | 185710 | 1 |
| TESTI2038104 | 0.001095278 | 101689 | 1 |
| TESTI2038275 | 0.001095278 | 111829 | 1 |
| TESTI2038388 | 0.001095278 | 173886 | 1 |
| TESTI2038573 | 0.001095278 | 220445 | 1 |
| TESTI2038596 | 0.001095278 | 222681 | 1 |
| TESTI2038623 | 0.001095278 | 142700 | 1 |
| TESTI2038644 | 0.001095278 | 220472 | 1 |
| TESTI2038733 | 0.001095278 | 182610 | 1 |
| TESTI2038858 | 0.001095278 | 194534 | 1 |
| TESTI2038958 | 0.001095278 | 212921 | 1 |
| TESTI2039025 | 0.001095278 | 43642 | 1 |
| TESTI2039026 | 0.001095278 | 224310 | 1 |
| TESTI2039038 | 0.001095278 | 100266 | 1 |
| TESTI2039041 | 0.001095278 | 236560 | 1 |
| TESTI2039060 | 0.001095278 | 217562 | 1 |
| TESTI2039113 | 0.001095278 | 185855 | 1 |
| TESTI2039121 | 0.001095278 | 220499 | 1 |
| TESTI2039177 | 0.040429071 | 123408 | 25 |
| TESTI2039209 | 0.001095278 | 161758 | 1 |
| TESTI2039225 | 0.001095278 | 118795 | 1 |
| TESTI2039227 | 0.001095278 | 166589 | 1 |
| TESTI2039276 | 0.001095278 | 23784 | 1 |
| TESTI2039342 | 0.004811507 | 99594 | 3 |
| TESTI2039353 | 0.001095278 | 249300 | 1 |
| TESTI2039544 | 0.001095278 | 213203 | 1 |
| TESTI2039613 | 0.001095278 | 198340 | 1 |
| TESTI2039707 | 0.007297598 | 43760 | 2 |
| TESTI2039732 | 0.001095278 | 166678 | 1 |
| TESTI2039738 | 0.001095278 | 165065 | 1 |
| TESTI2039776 | 0.001095278 | 148658 | 1 |
| TESTI2039867 | 0.001095278 | 167960 | 1 |
| TESTI2039908 | 0.002190557 | 173907 | 2 |
| TESTI2040018 | 0.002190557 | 82392 | 2 |
| TESTI2040094 | 0.01361876 | 180254 | 2 |
| TESTI2040102 | 0.001095278 | 196367 | 1 |
| TESTI2040128 | 0.001095278 | 180736 | 1 |
| TESTI2040143 | 0.001095278 | 19137 | 1 |
| TESTI2040297 | 0.001095278 | 166671 | 1 |
| TESTI2040372 | 0.001095278 | 235885 | 1 |
| TESTI2040377 | 0.002190557 | 234525 | 2 |
| TESTI2040424 | 0.001095278 | 48663 | 1 |
| TESTI2040642 | 0.001095278 | 125806 | 1 |
| TESTI2040815 | 0.001095278 | 206869 | 1 |
| TESTI2040897 | 0.001095278 | 127194 | 1 |
| TESTI2040944 | 0.001095278 | 161674 | 1 |
| TESTI2040964 | 0.001095278 | 173889 | 1 |
| TESTI2040983 | 0.001095278 | 122349 | 1 |
| TESTI2040989 | 0.001095278 | 180744 | 1 |
| TESTI2041330 | 0.001095278 | 263218 | 1 |
| TESTI2041362 | 0.001095278 | 207802 | 1 |
| TESTI2041517 | 0.001095278 | 212840 | 1 |
| TESTI2041564 | 0.001095278 | 212980 | 1 |
| TESTI2041664 | 0.001095278 | 106672 | 1 |
| TESTI2041730 | 0.001095278 | 191229 | 1 |
| TESTI2041734 | 0.004272069 | 128030 | 3 |
| TESTI2041778 | 0.001095278 | 113289 | 1 |
| TESTI2041800 | 0.001095278 | 69935 | 1 |
| TESTI2041947 | 0.002190557 | 191391 | 2 |
| TESTI2041956 | 0.001095278 | 200171 | 1 |
| TESTI2041976 | 0.002190557 | 199164 | 2 |
| TESTI2042062 | 0.001095278 | 74575 | 1 |
| TESTI2042149 | 0.001095278 | 170073 | 1 |
| TESTI2042264 | 0.001095278 | 117549 | 1 |
| TESTI2042295 | 0.001095278 | 218024 | 1 |
| TESTI2042302 | 0.001095278 | 113463 | 1 |
| TESTI2042326 | 0.001095278 | 13365 | 1 |
| TESTI2042400 | 0.001095278 | 206278 | 1 |
| TESTI2042450 | 0.001095278 | 281398 | 1 |
| TESTI2042473 | 0.001095278 | 65975 | 1 |
| TESTI2042507 | 0.001095278 | 208148 | 1 |
| TESTI2042508 | 0.001095278 | 23236 | 1 |
| TESTI2042750 | 0.001095278 | 49432 | 1 |
| TESTI2042783 | 0.001095278 | 242809 | 1 |
| TESTI2042806 | 0.001095278 | 202323 | 1 |
| TESTI2042928 | 0.001095278 | 191234 | 1 |
| TESTI2042946 | 0.001095278 | 182670 | 1 |
| TESTI2042958 | 0.003285835 | 175797 | 3 |
| TESTI2043275 | 0.018936539 | 229966 | 3 |
| TESTI2043277 | 0.001095278 | 124130 | 1 |
| TESTI2043282 | 0.001095278 | 198169 | 1 |
| TESTI2043313 | 0.001095278 | 217286 | 1 |
| TESTI2043477 | 0.002190557 | 73800 | 2 |
| TESTI2043585 | 0.001095278 | 177670 | 1 |
| TESTI2043656 | 0.00297212 | 2682 | 2 |
| TESTI2043758 | 0.001095278 | 100820 | 1 |
| TESTI2043817 | 0.001095278 | 284823 | 1 |
| TESTI2043857 | 0.001095278 | 195055 | 1 |
| TESTI2043866 | 0.002190557 | 195951 | 2 |
| TESTI2043869 | 0.002190557 | 211069 | 2 |
| TESTI2044064 | 0.001095278 | 208637 | 1 |
| TESTI2044118 | 0.001095278 | 19320 | 1 |
| TESTI2044194 | 0.001095278 | 31478 | 1 |
| TESTI2044276 | 0.035334498 | 111254 | 16 |
| TESTI2044306 | 0.001095278 | 182666 | 1 |
| TESTI2044309 | 0.001095278 | 152986 | 1 |
| TESTI2044413 | 0.001095278 | 223607 | 1 |
| TESTI2044418 | 0.001095278 | 166930 | 1 |
| TESTI2044754 | 0.001095278 | 136190 | 1 |
| TESTI2044788 | 0.00309368 | 113426 | 2 |
| TESTI2044796 | 0.001095278 | 185715 | 1 |
| TESTI2044833 | 0.001095278 | 196044 | 1 |
| TESTI2044892 | 0.001095278 | 219603 | 1 |
| TESTI2044920 | 0.001095278 | 123073 | 1 |
| TESTI2044968 | 0.001095278 | 128490 | 1 |
| TESTI2045139 | 0.001095278 | 166945 | 1 |
| TESTI2045155 | 0.014546877 | 176527 | 3 |
| TESTI2045171 | 0.001095278 | 177741 | 1 |
| TESTI2045199 | 0.001095278 | 163612 | 1 |
| TESTI2045353 | 0.001095278 | 196591 | 1 |
| TESTI2045509 | 0.001095278 | 187731 | 1 |
| TESTI2045562 | 0.007136844 | 64524 | 2 |
| TESTI2045611 | 0.001095278 | 126797 | 1 |
| TESTI2045819 | 0.001095278 | 191193 | 1 |
| TESTI2045850 | 0.001095278 | 140324 | 1 |
| TESTI2045920 | 0.001095278 | 202494 | 1 |
| TESTI2045964 | 0.001095278 | 21254 | 1 |
| TESTI2045983 | 0.001095278 | 150606 | 1 |
| TESTI2046100 | 0.001095278 | 123870 | 1 |
| TESTI2046188 | 0.001095278 | 223197 | 1 |
| TESTI2046347 | 0.001095278 | 280511 | 1 |
| TESTI2046352 | 0.001095278 | 213684 | 1 |
| TESTI2046358 | 0.001095278 | 58017 | 1 |
| TESTI2046439 | 0.003285835 | 210508 | 3 |
| TESTI2046456 | 0.361985383 | 75965 | 82 |
| TESTI2046535 | 0.001095278 | 46211 | 1 |
| TESTI2046536 | 0.001095278 | 37068 | 1 |
| TESTI2046552 | 0.001095278 | 122071 | 1 |
| TESTI2046569 | 0.001095278 | 79570 | 1 |
| TESTI2046686 | 0.002190557 | 144123 | 2 |
| TESTI2046721 | 0.013689737 | 122896 | 2 |
| TESTI2046732 | 0.001095278 | 9151 | 1 |
| TESTI2046786 | 0.001095278 | 32053 | 1 |
| TESTI2046797 | 0.002190557 | 211229 | 2 |
| TESTI2046863 | 0.001095278 | 193133 | 1 |
| TESTI2046872 | 0.001095278 | 42985 | 1 |
| TESTI2047071 | 0.001095278 | 249934 | 1 |
| TESTI2047141 | 0.007666948 | 67646 | 7 |
| TESTI2047147 | 0.001095278 | 232760 | 1 |
| TESTI2047153 | 0.001095278 | 234778 | 1 |
| TESTI2047212 | 0.001095278 | 245491 | 1 |
| TESTI2047342 | 0.001095278 | 138514 | 1 |
| TESTI2047383 | 0.001095278 | 133979 | 1 |
| TESTI2047605 | 0.01149927 | 165372 | 5 |
| TESTI2047792 | 0.001095278 | 151700 | 1 |
| TESTI2047801 | 0.001095278 | 207856 | 1 |
| TESTI2047818 | 0.002190557 | 142677 | 2 |
| TESTI2047824 | 0.001095278 | 278834 | 1 |
| TESTI2047885 | 0.001095278 | 216740 | 1 |
| TESTI2047930 | 0.001095278 | 194556 | 1 |
| TESTI2048105 | 0.001095278 | 213331 | 1 |
| TESTI2048109 | 0.001095278 | 265230 | 1 |
| TESTI2048465 | 0.001095278 | 246943 | 1 |
| TESTI2048603 | 0.001095278 | 264089 | 1 |
| TESTI2048898 | 0.003285835 | 252442 | 3 |
| TESTI2049041 | 0.001095278 | 87253 | 1 |
| TESTI2049062 | 0.001095278 | 162294 | 1 |
| TESTI2049206 | 0.001095278 | 269564 | 1 |
| TESTI2049246 | 0.006372313 | 99342 | 4 |
| TESTI2049277 | 0.001095278 | 266096 | 1 |
| TESTI2049422 | 0.001095278 | 279530 | 1 |
| TESTI2049452 | 0.001095278 | 264249 | 1 |
| TESTI2049469 | 0.001095278 | 189312 | 1 |
| TESTI2049553 | 0.001095278 | 12055 | 1 |
| TESTI2049576 | 0.001095278 | 282334 | 1 |
| TESTI2049857 | 0.001095278 | 272463 | 1 |
| TESTI2049956 | 0.001095278 | 262002 | 1 |
| TESTI2050137 | 0.001095278 | 141968 | 1 |
| TESTI2050681 | 0.001095278 | 62563 | 1 |
| TESTI2050780 | 0.001095278 | 270964 | 1 |
| TESTI2050987 | 0.001095278 | 262730 | 1 |
| TESTI2051148 | 0.001095278 | 262795 | 1 |
| TESTI2051177 | 0.00700754 | 41869 | 2 |
| TESTI2051279 | 0.001095278 | 266567 | 1 |
| TESTI2051488 | 0.001095278 | 275569 | 1 |
| TESTI2051543 | 0.001095278 | 242256 | 1 |
| TESTI2051627 | 0.001095278 | 245246 | 1 |
| TESTI2051742 | 0.001095278 | 179082 | 1 |
| TESTI2051767 | 0.001095278 | 259239 | 1 |
| TESTI2051791 | 0.001095278 | 263829 | 1 |
| TESTI2051806 | 0.001095278 | 252533 | 1 |
| TESTI2051867 | 0.003285835 | 200798 | 3 |
| TESTI2051873 | 0.001095278 | 262760 | 1 |
| TESTI2052110 | 0.001095278 | 250638 | 1 |
| TESTI2052202 | 0.001095278 | 211683 | 1 |
| TESTI2052211 | 0.001095278 | 248949 | 1 |
| TESTI2052256 | 0.001095278 | 261859 | 1 |
| TESTI2052288 | 0.001095278 | 244427 | 1 |
| TESTI2052525 | 0.001095278 | 258269 | 1 |
| TESTI2052670 | 0.001095278 | 259276 | 1 |
| TESTI2052693 | 0.100082003 | 187540 | 5 |
| TESTI2052698 | 0.001095278 | 138340 | 1 |
| TESTI2052799 | 0.001095278 | 252496 | 1 |
| TESTI2052822 | 0.001095278 | 270804 | 1 |
| TESTI2052985 | 0.001095278 | 256967 | 1 |
| TESTI2053151 | 0.001095278 | 251816 | 1 |
| TESTI2053242 | 0.001095278 | 259085 | 1 |
| TESTI2053399 | 0.001095278 | 21602 | 1 |
| TESTI2053526 | 0.001095278 | 256794 | 1 |
| TESTI2053561 | 0.001095278 | 249163 | 1 |
| TESTI2053621 | 0.001095278 | 130026 | 1 |
| TESTI2053667 | 0.00297212 | 151109 | 2 |
| TESTI2053723 | 0.03701313 | 120236 | 13 |
| TESTI3000002 | 0.001095278 | 39924 | 1 |
| TESTI3000005 | 0.026286678 | 52760 | 24 |
| TESTI4000014 | 0.938263392 | 60637 | 166 |
| TESTI4000068 | 0.001095278 | 153102 | 1 |
| TESTI4000079 | 0.001095278 | 61247 | 1 |
| TESTI4000093 | 0.004381113 | 158843 | 4 |
| TESTI4000134 | 0.001095278 | 200245 | 1 |
| TESTI4000137 | 0.001095278 | 109195 | 1 |
| TESTI4000153 | 0.001095278 | 187359 | 1 |
| TESTI4000155 | 0.001095278 | 204925 | 1 |
| TESTI4000157 | 0.001095278 | 192960 | 1 |
| TESTI4000183 | 0.001095278 | 194326 | 1 |
| TESTI4000209 | 0.06325171 | 153121 | 16 |
| TESTI4000214 | 0.021424469 | 160960 | 19 |
| TESTI4000215 | 0.001095278 | 165944 | 1 |
| TESTI4000228 | 0.001095278 | 49078 | 1 |
| TESTI4000250 | 0.001095278 | 197001 | 1 |
| TESTI4000288 | 0.001095278 | 159819 | 1 |
| TESTI4000319 | 0.003285835 | 188030 | 3 |
| TESTI4000349 | 0.006445887 | 72087 | 5 |
| TESTI4000370 | 0.001095278 | 179356 | 1 |
| TESTI4000394 | 0.001095278 | 141931 | 1 |
| TESTI4000434 | 0.012814546 | 49772 | 10 |
| TESTI4000462 | 0.003285835 | 51147 | 3 |
| TESTI4000530 | 0.002190557 | 194577 | 2 |
| TESTI4000534 | 0.001095278 | 215053 | 1 |
| TESTI4000598 | 0.001095278 | 251449 | 1 |
| TESTI4000600 | 0.002190557 | 92886 | 2 |
| TESTI4000621 | 0.001095278 | 261595 | 1 |
| TESTI4000703 | 0.001095278 | 260353 | 1 |
| TESTI4000724 | 0.001095278 | 121180 | 1 |
| TESTI4000957 | 0.001095278 | 187323 | 1 |
| TESTI4000970 | 0.003285835 | 5741 | 3 |
| TESTI4001036 | 0.001095278 | 263559 | 1 |
| TESTI4001037 | 0.001095278 | 132551 | 1 |
| TESTI4001060 | 0.001095278 | 266434 | 1 |
| TESTI4001100 | 0.040891613 | 121038 | 17 |
| TESTI4001106 | 0.001095278 | 124278 | 1 |
| TESTI4001148 | 0.001095278 | 240884 | 1 |
| TESTI4001176 | 0.001095278 | 245516 | 1 |
| TESTI4001195 | 0.001095278 | 240608 | 1 |
| TESTI4001201 | 0.001095278 | 249981 | 1 |
| TESTI4001206 | 0.004010135 | 191516 | 2 |
| TESTI4001348 | 0.001095278 | 217118 | 1 |
| TESTI4001441 | 0.001095278 | 42952 | 1 |
| TESTI4001467 | 0.030264738 | 59805 | 7 |
| TESTI4001473 | 0.001095278 | 273886 | 1 |
| TESTI4001517 | 0.001095278 | 161876 | 1 |
| TESTI4001527 | 0.001095278 | 22471 | 1 |
| TESTI4001561 | 0.004161364 | 192332 | 3 |
| TESTI4001569 | 0.003900017 | 135865 | 3 |
| TESTI4001604 | 0.005758131 | 245334 | 4 |
| TESTI4001665 | 0.001095278 | 232605 | 1 |
| TESTI4001679 | 0.001095278 | 193021 | 1 |
| TESTI4001795 | 0.007666948 | 170563 | 7 |
| TESTI4001923 | 0.001095278 | 236152 | 1 |
| TESTI4001925 | 0.001095278 | 207691 | 1 |
| TESTI4001984 | 0.002770825 | 241338 | 2 |
| TESTI4002003 | 0.003285835 | 192128 | 3 |
| TESTI4002072 | 0.05017915 | 126605 | 27 |
| TESTI4002141 | 0.001095278 | 270506 | 1 |
| TESTI4002195 | 0.0250447 | 114499 | 6 |
| TESTI4002202 | 0.050541264 | 124222 | 14 |
| TESTI4002290 | 0.002953393 | 216458 | 2 |
| TESTI4002319 | 0.001095278 | 260838 | 1 |
| TESTI4002491 | 0.001095278 | 261542 | 1 |
| TESTI4002520 | 0.001095278 | 282980 | 1 |
| TESTI4002552 | 0.001095278 | 78622 | 1 |
| TESTI4002647 | 0.00317679 | 157852 | 2 |
| TESTI4002703 | 0.001095278 | 265261 | 1 |
| TESTI4002754 | 0.001095278 | 256679 | 1 |
| TESTI4002774 | 0.040839417 | 65581 | 12 |
| TESTI4002799 | 0.379400289 | 77233 | 51 |
| TESTI4002868 | 0.003285835 | 166368 | 3 |
| TESTI4002878 | 0.001095278 | 222020 | 1 |
| TESTI4002889 | 0.001095278 | 259192 | 1 |
| TESTI4002988 | 0.007726483 | 78207 | 5 |
| TESTI4003150 | 0.01128277 | 100256 | 7 |
| TESTI4003179 | 0.004067398 | 43813 | 3 |
| TESTI4003279 | 0.001095278 | 216493 | 1 |
| TESTI4003319 | 0.002190557 | 253395 | 2 |
| TESTI4003404 | 0.001095278 | 214633 | 1 |
| TESTI4003565 | 0.002190557 | 157103 | 2 |
| TESTI4003574 | 0.001095278 | 230059 | 1 |
| TESTI4003579 | 0.00297212 | 175467 | 2 |
| TESTI4003602 | 0.006452503 | 126936 | 5 |
| TESTI4003703 | 0.037866701 | 118926 | 19 |
| TESTI4003733 | 0.001095278 | 241415 | 1 |
| TESTI4003796 | 0.001095278 | 255051 | 1 |
| TESTI4003944 | 0.00480314 | 77460 | 3 |
| TESTI4004003 | 0.001095278 | 261558 | 1 |
| TESTI4004031 | 0.001095278 | 149332 | 1 |
| TESTI4004200 | 0.001095278 | 250967 | 1 |
| TESTI4004210 | 0.004647667 | 125473 | 3 |
| TESTI4004539 | 0.001095278 | 59137 | 1 |
| TESTI4004626 | 0.010177686 | 191821 | 5 |
| TESTI4004653 | 0.001095278 | 13268 | 1 |
| TESTI4004695 | 0.005143949 | 86266 | 4 |
| TESTI4004722 | 0.001095278 | 238982 | 1 |
| TESTI4004887 | 0.001095278 | 20521 | 1 |
| TESTI4004917 | 0.003285835 | 188177 | 3 |
| TESTI4005013 | 0.010253348 | 101332 | 9 |
| TESTI4005015 | 0.001095278 | 254907 | 1 |
| TESTI4005039 | 0.014238617 | 123537 | 13 |
| TESTI4005133 | 0.014051214 | 146299 | 9 |
| TESTI4005158 | 0.001095278 | 228107 | 1 |
| TESTI4005317 | 0.001095278 | 142461 | 1 |
| TESTI4005322 | 0.001095278 | 179829 | 1 |
| TESTI4005399 | 0.021489255 | 154134 | 14 |
| TESTI4005470 | 0.004381113 | 164571 | 4 |
| TESTI4005500 | 0.001095278 | 78218 | 1 |
| TESTI4005534 | 0.006008537 | 66733 | 3 |
| TESTI4005543 | 0.001095278 | 116823 | 1 |
| TESTI4005628 | 0.001095278 | 259280 | 1 |
| TESTI4005635 | 0.010272745 | 150737 | 7 |
| TESTI4005653 | 0.337008866 | 34785 | 103 |
| TESTI4005801 | 0.001095278 | 260835 | 1 |
| TESTI4005805 | 0.001095278 | 264025 | 1 |
| TESTI4005857 | 0.005272213 | 14686 | 2 |
| TESTI4005961 | 0.001095278 | 71049 | 1 |
| TESTI4006053 | 0.001095278 | 259289 | 1 |
| TESTI4006079 | 0.001095278 | 260348 | 1 |
| TESTI4006112 | 0.001095278 | 264771 | 1 |
| TESTI4006137 | 0.006505781 | 138977 | 4 |
| TESTI4006148 | 0.001095278 | 231523 | 1 |
| TESTI4006219 | 0.001095278 | 259178 | 1 |
| TESTI4006234 | 0.001095278 | 251254 | 1 |
| TESTI4006247 | 0.013932163 | 158385 | 7 |
| TESTI4006308 | 0.001095278 | 22166 | 1 |
| TESTI4006326 | 0.004272069 | 142505 | 3 |
| TESTI4006393 | 0.003285835 | 97456 | 3 |
| TESTI4006407 | 0.002953393 | 212390 | 2 |
| TESTI4006412 | 0.003285835 | 215151 | 3 |
| TESTI4006420 | 0.001095278 | 54230 | 1 |
| TESTI4006441 | 0.002770825 | 275980 | 2 |
| TESTI4006473 | 0.001095278 | 74239 | 1 |
| TESTI4006539 | 0.001095278 | 266692 | 1 |
| TESTI4006546 | 0.002190557 | 206213 | 2 |
| TESTI4006567 | 0.001095278 | 57055 | 1 |
| TESTI4006704 | 0.001095278 | 157830 | 1 |
| TESTI4006728 | 0.001095278 | 215677 | 1 |
| TESTI4006802 | 0.001095278 | 180893 | 1 |
| TESTI4006819 | 0.004067398 | 149915 | 3 |
| TESTI4007064 | 0.001095278 | 245587 | 1 |
| TESTI4007163 | 0.003285835 | 93467 | 3 |
| TESTI4007176 | 0.001095278 | 243841 | 1 |
| TESTI4007203 | 0.001095278 | 236230 | 1 |
| TESTI4007239 | 0.007666948 | 193028 | 7 |
| TESTI4007369 | 0.012855833 | 8208 | 2 |
| TESTI4007373 | 0.001095278 | 63092 | 1 |
| TESTI4007382 | 0.004381113 | 180442 | 4 |
| TESTI4007404 | 0.001095278 | 203957 | 1 |
| TESTI4007489 | 0.002190557 | 257961 | 2 |
| TESTI4007565 | 0.001095278 | 284452 | 1 |
| TESTI4007671 | 0.001095278 | 264174 | 1 |
| TESTI4007775 | 0.002190557 | 201476 | 2 |
| TESTI4007778 | 0.001095278 | 248467 | 1 |
| TESTI4007799 | 0.001095278 | 255217 | 1 |
| TESTI4007810 | 0.001095278 | 258571 | 1 |
| TESTI4007816 | 0.038684199 | 144528 | 3 |
| TESTI4007965 | 0.009284109 | 79512 | 6 |
| TESTI4008007 | 0.001095278 | 262093 | 1 |
| TESTI4008018 | 0.004216085 | 34035 | 2 |
| TESTI4008050 | 0.001095278 | 261748 | 1 |
| TESTI4008058 | 0.019010056 | 126677 | 8 |
| TESTI4008086 | 0.001095278 | 264856 | 1 |
| TESTI4008097 | 0.002770825 | 161715 | 2 |
| TESTI4008176 | 0.001095278 | 15372 | 1 |
| TESTI4008209 | 0.001095278 | 253718 | 1 |
| TESTI4008219 | 0.001095278 | 266870 | 1 |
| TESTI4008250 | 0.002190557 | 242479 | 2 |
| TESTI4008302 | 0.001095278 | 166280 | 1 |
| TESTI4008305 | 0.001095278 | 268497 | 1 |
| TESTI4008401 | 0.001095278 | 185692 | 1 |
| TESTI4008417 | 0.001095278 | 149779 | 1 |
| TESTI4008429 | 0.001095278 | 282235 | 1 |
| TESTI4008573 | 0.001095278 | 258324 | 1 |
| TESTI4008797 | 0.01348075 | 61664 | 9 |
| TESTI4008816 | 0.001095278 | 258200 | 1 |
| TESTI4008840 | 0.001095278 | 265030 | 1 |
| TESTI4008935 | 0.001095278 | 52296 | 1 |
| TESTI4008993 | 0.001095278 | 241438 | 1 |
| TESTI4009022 | 0.001095278 | 213560 | 1 |
| TESTI4009028 | 0.001095278 | 260812 | 1 |
| TESTI4009034 | 0.001095278 | 258458 | 1 |
| TESTI4009123 | 0.001095278 | 161874 | 1 |
| TESTI4009160 | 0.001095278 | 223104 | 1 |
| TESTI4009215 | 0.001095278 | 211837 | 1 |
| TESTI4009283 | 0.001095278 | 271702 | 1 |
| TESTI4009286 | 0.231137289 | 35424 | 40 |
| TESTI4009306 | 0.001095278 | 233916 | 1 |
| TESTI4009374 | 0.001095278 | 170997 | 1 |
| TESTI4009406 | 0.001095278 | 173756 | 1 |
| TESTI4009454 | 0.041689175 | 134363 | 14 |
| TESTI4009457 | 0.006257955 | 70902 | 5 |
| TESTI4009501 | 0.001095278 | 250496 | 1 |
| TESTI4009563 | 0.001095278 | 186154 | 1 |
| TESTI4009608 | 0.001095278 | 90338 | 1 |
| TESTI4009638 | 0.001095278 | 259231 | 1 |
| TESTI4009752 | 0.001095278 | 259300 | 1 |
| TESTI4009881 | 0.001095278 | 192460 | 1 |
| TESTI4009886 | 0.001095278 | 257087 | 1 |
| TESTI4009928 | 0.001095278 | 244272 | 1 |
| TESTI4009973 | 0.176782467 | 101801 | 29 |
| TESTI4010076 | 0.001095278 | 266938 | 1 |
| TESTI4010082 | 0.001095278 | 240485 | 1 |
| TESTI4010095 | 0.001095278 | 195931 | 1 |
| TESTI4010211 | 0.001095278 | 260383 | 1 |
| TESTI4010377 | 0.002804738 | 254442 | 2 |
| TESTI4010382 | 0.016272096 | 186082 | 7 |
| TESTI4010475 | 0.001095278 | 257010 | 1 |
| TESTI4010544 | 0.003285835 | 143857 | 3 |
| TESTI4010713 | 0.002190557 | 244611 | 2 |
| TESTI4010721 | 0.001095278 | 41193 | 1 |
| TESTI4010789 | 0.001095278 | 253923 | 1 |
| TESTI4010817 | 0.004381113 | 170437 | 4 |
| TESTI4010831 | 0.001095278 | 232300 | 1 |
| TESTI4010851 | 0.306486226 | 29256 | 90 |
| TESTI4010902 | 0.001095278 | 221968 | 1 |
| TESTI4010928 | 0.004995295 | 166362 | 4 |
| TESTI4010979 | 0.008527483 | 179607 | 4 |
| TESTI4011070 | 0.008762226 | 126026 | 8 |
| TESTI4011072 | 0.001095278 | 285513 | 1 |
| TESTI4011084 | 0.002804738 | 244139 | 2 |
| TESTI4011118 | 0.001095278 | 263136 | 1 |
| TESTI4011161 | 0.007952337 | 82076 | 5 |
| TESTI4011246 | 0.001095278 | 245854 | 1 |
| TESTI4011484 | 0.001095278 | 78263 | 1 |
| TESTI4011505 | 0.001095278 | 269537 | 1 |
| TESTI4011532 | 0.001095278 | 134326 | 1 |
| TESTI4011616 | 0.001095278 | 247898 | 1 |
| TESTI4011744 | 0.001095278 | 272566 | 1 |
| TESTI4011745 | 0.001095278 | 242070 | 1 |
| TESTI4011829 | 0.001095278 | 254628 | 1 |
| TESTI4011926 | 0.001095278 | 253338 | 1 |
| TESTI4011956 | 0.001095278 | 257599 | 1 |
| TESTI4012010 | 0.001095278 | 114228 | 1 |
| TESTI4012086 | 0.001095278 | 225766 | 1 |
| TESTI4012258 | 0.001095278 | 223569 | 1 |
| TESTI4012293 | 0.001095278 | 282037 | 1 |
| TESTI4012329 | 0.001095278 | 264163 | 1 |
| TESTI4012382 | 0.001095278 | 268140 | 1 |
| TESTI4012406 | 0.001095278 | 13070 | 1 |
| TESTI4012448 | 0.001095278 | 276934 | 1 |
| TESTI4012451 | 0.002953393 | 61924 | 2 |
| TESTI4012505 | 0.001095278 | 96659 | 1 |
| TESTI4012556 | 0.002190557 | 188194 | 2 |
| TESTI4012623 | 0.001095278 | 259204 | 1 |
| TESTI4012646 | 0.002770825 | 74897 | 2 |
| TESTI4012679 | 0.001095278 | 251963 | 1 |
| TESTI4012702 | 0.076863312 | 110087 | 22 |
| TESTI4012911 | 0.002190557 | 233430 | 2 |
| TESTI4012956 | 0.001095278 | 253382 | 1 |
| TESTI4012960 | 0.011253356 | 212425 | 4 |
| TESTI4013365 | 0.002190557 | 185163 | 2 |
| TESTI4013369 | 0.001095278 | 266716 | 1 |
| TESTI4013410 | 0.003285835 | 115097 | 3 |
| TESTI4013441 | 1.068316129 | 98128 | 81 |
| TESTI4013474 | 0.162165048 | 98488 | 26 |
| TESTI4013597 | 0.076598619 | 6825 | 26 |
| TESTI4013602 | 0.001095278 | 264914 | 1 |
| TESTI4013667 | 0.001095278 | 250425 | 1 |
| TESTI4013675 | 0.01697089 | 188529 | 11 |
| TESTI4013685 | 0.001095278 | 134025 | 1 |
| TESTI4013735 | 0.00297212 | 256504 | 2 |
| TESTI4013742 | 0.001095278 | 238119 | 1 |
| TESTI4013774 | 0.001095278 | 272781 | 1 |
| TESTI4013817 | 0.006168562 | 84751 | 4 |
| TESTI4013830 | 0.001095278 | 236696 | 1 |
| TESTI4013852 | 0.001095278 | 253280 | 1 |
| TESTI4013894 | 0.011963585 | 136773 | 8 |
| TESTI4013924 | 0.001095278 | 254302 | 1 |
| TESTI4013960 | 0.001095278 | 251888 | 1 |
| TESTI4013962 | 0.001095278 | 259273 | 1 |
| TESTI4014133 | 0.09727873 | 61927 | 23 |
| TESTI4014159 | 0.014295594 | 153848 | 9 |
| TESTI4014175 | 0.006272286 | 108415 | 3 |
| TESTI4014262 | 0.042877709 | 73491 | 33 |
| TESTI4014265 | 0.001095278 | 238115 | 1 |
| TESTI4014276 | 0.001095278 | 238113 | 1 |
| TESTI4014306 | 0.002284523 | 244580 | 2 |
| TESTI4014392 | 0.002190557 | 211692 | 2 |
| TESTI4014415 | 0.002190557 | 199374 | 2 |
| TESTI4014445 | 0.002190557 | 229547 | 2 |
| TESTI4014553 | 0.003473767 | 183813 | 3 |
| TESTI4014661 | 0.001095278 | 238087 | 1 |
| TESTI4014694 | 0.150353417 | 186725 | 5 |
| TESTI4014801 | 0.003285835 | 222939 | 3 |
| TESTI4014818 | 0.002804738 | 249997 | 2 |
| TESTI4014891 | 0.001095278 | 194526 | 1 |
| TESTI4014908 | 0.004647667 | 113477 | 3 |
| TESTI4014924 | 0.001095278 | 68375 | 1 |
| TESTI4014932 | 0.002190557 | 218144 | 2 |
| TESTI4014977 | 0.002190557 | 138350 | 2 |
| TESTI4015012 | 0.008762226 | 203956 | 8 |
| TESTI4015129 | 0.001095278 | 238860 | 1 |
| TESTI4015145 | 0.001095278 | 22049 | 1 |
| TESTI4015204 | 0.006381331 | 61487 | 4 |
| TESTI4015263 | 0.001095278 | 239895 | 1 |
| TESTI4015293 | 0.002190557 | 171444 | 2 |
| TESTI4015299 | 0.001095278 | 261326 | 1 |
| TESTI4015339 | 0.001095278 | 264707 | 1 |
| TESTI4015398 | 0.001095278 | 260813 | 1 |
| TESTI4015442 | 0.008310351 | 206677 | 4 |
| TESTI4015471 | 0.001095278 | 251510 | 1 |
| TESTI4015477 | 0.002190557 | 182384 | 2 |
| TESTI4015600 | 0.002190557 | 199200 | 2 |
| TESTI4015646 | 0.001095278 | 232483 | 1 |
| TESTI4015681 | 0.001095278 | 250757 | 1 |
| TESTI4015688 | 0.001095278 | 259106 | 1 |
| TESTI4015790 | 0.001095278 | 262645 | 1 |
| TESTI4016110 | 0.001095278 | 229649 | 1 |
| TESTI4016238 | 0.001095278 | 243749 | 1 |
| TESTI4016551 | 0.008762226 | 153589 | 8 |
| TESTI4016812 | 0.077764756 | 120203 | 71 |
| TESTI4016822 | 0.001095278 | 258312 | 1 |
| TESTI4016848 | 0.003285835 | 51147 | 3 |
| TESTI4016865 | 0.001095278 | 266111 | 1 |
| TESTI4016882 | 0.001095278 | 262826 | 1 |
| TESTI4016925 | 0.004848962 | 183298 | 3 |
| TESTI4017001 | 0.001095278 | 250724 | 1 |
| TESTI4017116 | 0.002190557 | 284487 | 2 |
| TESTI4017137 | 0.001095278 | 253987 | 1 |
| TESTI4017229 | 0.001095278 | 249141 | 1 |
| TESTI4017254 | 0.001095278 | 215658 | 1 |
| TESTI4017269 | 0.001095278 | 232514 | 1 |
| TESTI4017380 | 0.002190557 | 157332 | 2 |
| TESTI4017382 | 0.001095278 | 229488 | 1 |
| TESTI4017543 | 0.001095278 | 143102 | 1 |
| TESTI4017575 | 0.002190557 | 235480 | 2 |
| TESTI4017647 | 0.001095278 | 259018 | 1 |
| TESTI4017714 | 0.004272069 | 28615 | 3 |
| TESTI4017763 | 0.002190557 | 112201 | 2 |
| TESTI4017848 | 0.001095278 | 261053 | 1 |
| TESTI4017854 | 0.012882171 | 251424 | 2 |
| TESTI4017901 | 0.001095278 | 258424 | 1 |
| TESTI4017961 | 0.001095278 | 249809 | 1 |
| TESTI4017984 | 0.003379801 | 206412 | 3 |
| TESTI4018101 | 0.001095278 | 56150 | 1 |
| TESTI4018152 | 0.007779178 | 122861 | 6 |
| TESTI4018208 | 0.001095278 | 238296 | 1 |
| TESTI4018382 | 0.001095278 | 257236 | 1 |
| TESTI4018436 | 0.032196163 | 136438 | 5 |
| TESTI4018506 | 0.003285835 | 111248 | 3 |
| TESTI4018555 | 0.001095278 | 244990 | 1 |
| TESTI4018751 | 0.001095278 | 245120 | 1 |
| TESTI4018806 | 0.002190557 | 139589 | 2 |
| TESTI4018835 | 0.009857504 | 135580 | 9 |
| TESTI4018859 | 0.001095278 | 248308 | 1 |
| TESTI4018881 | 0.001095278 | 47576 | 1 |
| TESTI4018886 | 0.00309368 | 233647 | 2 |
| TESTI4018938 | 0.009676875 | 81707 | 7 |
| TESTI4019140 | 0.001095278 | 154241 | 1 |
| TESTI4019149 | 0.001095278 | 273650 | 1 |
| TESTI4019299 | 0.007666948 | 186168 | 7 |
| TESTI4019417 | 0.001095278 | 217693 | 1 |
| TESTI4019440 | 0.001095278 | 204512 | 1 |
| TESTI4019566 | 0.002190557 | 131150 | 2 |
| TESTI4019657 | 0.001095278 | 283873 | 1 |
| TESTI4019756 | 0.001095278 | 263749 | 1 |
| TESTI4019843 | 0.004190774 | 151971 | 2 |
| TESTI4020092 | 0.001095278 | 255673 | 1 |
| TESTI4020102 | 0.001095278 | 85070 | 1 |
| TESTI4020342 | 0.001095278 | 274907 | 1 |
| TESTI4020460 | 0.001095278 | 215871 | 1 |
| TESTI4020596 | 0.00317679 | 125073 | 2 |
| TESTI4020806 | 0.001095278 | 179738 | 1 |
| TESTI4020819 | 0.001095278 | 167983 | 1 |
| TESTI4020920 | 0.001095278 | 132886 | 1 |
| TESTI4021129 | 0.001095278 | 149202 | 1 |
| TESTI4021197 | 0.001095278 | 145096 | 1 |
| TESTI4021294 | 0.001095278 | 229706 | 1 |
| TESTI4021377 | 0.001095278 | 260709 | 1 |
| TESTI4021456 | 0.001095278 | 254568 | 1 |
| TESTI4021478 | 0.007582332 | 144708 | 5 |
| TESTI4021482 | 0.007897763 | 77099 | 5 |
| TESTI4021491 | 0.001095278 | 237860 | 1 |
| TESTI4021569 | 0.001095278 | 42538 | 1 |
| TESTI4021713 | 0.001095278 | 156665 | 1 |
| TESTI4021821 | 0.001095278 | 204425 | 1 |
| TESTI4022158 | 0.012554286 | 150104 | 8 |
| TESTI4022577 | 0.001095278 | 192419 | 1 |
| TESTI4022716 | 0.001095278 | 111688 | 1 |
| TESTI4022873 | 0.001095278 | 232363 | 1 |
| TESTI4022936 | 0.006335944 | 102377 | 4 |
| TESTI4023096 | 0.001095278 | 114511 | 1 |
| TESTI4023172 | 0.001095278 | 281426 | 1 |
| TESTI4023546 | 0.016429174 | 134897 | 15 |
| TESTI4023555 | 0.001095278 | 214574 | 1 |
| TESTI4023654 | 0.001095278 | 211754 | 1 |
| TESTI4023722 | 0.001095278 | 186109 | 1 |
| TESTI4023762 | 0.001095278 | 186101 | 1 |
| TESTI4023942 | 0.001095278 | 260233 | 1 |
| TESTI4024096 | 0.001095278 | 279195 | 1 |
| TESTI4024240 | 0.001095278 | 188183 | 1 |
| TESTI4024245 | 0.004970521 | 182565 | 3 |
| TESTI4024294 | 0.001095278 | 193075 | 1 |
| TESTI4024344 | 0.001095278 | 180251 | 1 |
| TESTI4024387 | 0.001095278 | 209217 | 1 |
| TESTI4024420 | 0.004480285 | 192379 | 3 |
| TESTI4024494 | 0.001095278 | 22811 | 1 |
| TESTI4024740 | 0.009314271 | 150671 | 5 |
| TESTI4024874 | 0.001095278 | 141952 | 1 |
| TESTI4024890 | 0.001095278 | 149248 | 1 |
| TESTI4024907 | 0.001095278 | 179945 | 1 |
| TESTI4024930 | 0.001095278 | 149850 | 1 |
| TESTI4025062 | 0.001095278 | 260999 | 1 |
| TESTI4025268 | 0.002770825 | 122913 | 2 |
| TESTI4025401 | 0.001095278 | 199332 | 1 |
| TESTI4025494 | 0.001095278 | 173798 | 1 |
| TESTI4025547 | 0.002804738 | 79327 | 2 |
| TESTI4025731 | 0.001095278 | 206414 | 1 |
| TESTI4025797 | 0.001095278 | 135933 | 1 |
| TESTI4025865 | 0.001095278 | 154298 | 1 |
| TESTI4025908 | 0.001095278 | 182532 | 1 |
| TESTI4025920 | 0.002190557 | 180294 | 2 |
| TESTI4026079 | 0.006586012 | 106872 | 3 |
| TESTI4026080 | 0.001095278 | 61461 | 1 |
| TESTI4026192 | 0.001095278 | 180299 | 1 |
| TESTI4026207 | 0.002804738 | 272038 | 2 |
| TESTI4026295 | 0.001095278 | 177098 | 1 |
| TESTI4026390 | 0.002284523 | 164381 | 2 |
| TESTI4026456 | 0.001095278 | 192386 | 1 |
| TESTI4026510 | 0.001095278 | 100986 | 1 |
| TESTI4026524 | 0.001095278 | 131678 | 1 |
| TESTI4026680 | 0.004381113 | 116436 | 4 |
| TESTI4026700 | 0.001095278 | 109058 | 1 |
| TESTI4026762 | 0.001095278 | 134344 | 1 |
| TESTI4026785 | 0.001095278 | 206441 | 1 |
| TESTI4027139 | 0.001095278 | 279426 | 1 |
| TESTI4027170 | 0.001095278 | 247835 | 1 |
| TESTI4027262 | 0.001095278 | 281562 | 1 |
| TESTI4027516 | 0.001095278 | 242249 | 1 |
| TESTI4027557 | 0.001095278 | 221398 | 1 |
| TESTI4027660 | 0.001095278 | 146701 | 1 |
| TESTI4027821 | 0.002770825 | 213184 | 2 |
| TESTI4027969 | 0.002190557 | 197820 | 2 |
| TESTI4028042 | 0.001095278 | 192194 | 1 |
| TESTI4028059 | 0.002190557 | 42826 | 2 |
| TESTI4028062 | 0.001095278 | 269612 | 1 |
| TESTI4028182 | 0.001095278 | 283473 | 1 |
| TESTI4028429 | 0.003285835 | 101925 | 3 |
| TESTI4028612 | 0.001095278 | 255236 | 1 |
| TESTI4028692 | 0.001095278 | 186281 | 1 |
| TESTI4028809 | 0.001095278 | 245690 | 1 |
| TESTI4028823 | 0.001095278 | 225843 | 1 |
| TESTI4028880 | 0.001095278 | 12859 | 1 |
| TESTI4028904 | 0.001095278 | 241329 | 1 |
| TESTI4028938 | 0.001095278 | 228668 | 1 |
| TESTI4028958 | 0.001095278 | 130557 | 1 |
| TESTI4028983 | 0.001095278 | 249716 | 1 |
| TESTI4029023 | 0.002190557 | 120394 | 2 |
| TESTI4029297 | 0.037866701 | 118926 | 19 |
| TESTI4029348 | 0.001095278 | 239232 | 1 |
| TESTI4029370 | 0.001095278 | 188035 | 1 |
| TESTI4029528 | 0.001095278 | 245597 | 1 |
| TESTI4029651 | 0.001095278 | 180629 | 1 |
| TESTI4029671 | 0.009008804 | 219783 | 4 |
| TESTI4029676 | 0.001095278 | 251292 | 1 |
| TESTI4029690 | 0.001095278 | 242520 | 1 |
| TESTI4029731 | 0.001095278 | 232287 | 1 |
| TESTI4029743 | 0.002190557 | 172493 | 2 |
| TESTI4029836 | 0.002783555 | 140088 | 2 |
| TESTI4030069 | 0.001095278 | 241219 | 1 |
| TESTI4030159 | 0.001095278 | 225940 | 1 |
| TESTI4030319 | 0.001095278 | 275319 | 1 |
| TESTI4030505 | 0.001095278 | 156700 | 1 |
| TESTI4030603 | 0.001095278 | 254817 | 1 |
| TESTI4030669 | 0.002190557 | 238967 | 2 |
| TESTI4030673 | 0.001095278 | 251257 | 1 |
| TESTI4030864 | 0.001095278 | 122070 | 1 |
| TESTI4031 066 | 0.001095278 | 211782 | 1 |
| TESTI4031173 | 0.002190557 | 128307 | 2 |
| TESTI4031335 | 0.001095278 | 214580 | 1 |
| TESTI4031745 | 0.001095278 | 205992 | 1 |
| TESTI4031818 | 0.001095278 | 186273 | 1 |
| TESTI4032090 | 0.002770825 | 175498 | 2 |
| TESTI4032112 | 0.001095278 | 199306 | 1 |
| TESTI4032128 | 0.001095278 | 215321 | 1 |
| TESTI4032270 | 0.001095278 | 221282 | 1 |
| TESTI4032375 | 0.001095278 | 194277 | 1 |
| TESTI4032834 | 0.001095278 | 243607 | 1 |
| TESTI4032856 | 0.001095278 | 239404 | 1 |
| TESTI4032895 | 0.002190557 | 148207 | 2 |
| TESTI4032913 | 0.020672075 | 91743 | 7 |
| TESTI4033146 | 0.002190557 | 211697 | 2 |
| TESTI4033177 | 0.001095278 | 197836 | 1 |
| TESTI4033433 | 0.001095278 | 232260 | 1 |
| TESTI4033690 | 0.00297212 | 130642 | 2 |
| TESTI4034172 | 0.001095278 | 146288 | 1 |
| TESTI4034212 | 0.001095278 | 208305 | 1 |
| TESTI4034432 | 0.001095278 | 154533 | 1 |
| TESTI4034495 | 0.001095278 | 269704 | 1 |
| TESTI4034632 | 0.001095278 | 243364 | 1 |
| TESTI4034633 | 0.001095278 | 232482 | 1 |
| TESTI4034912 | 0.001095278 | 156069 | 1 |
| TESTI4034973 | 0.001095278 | 206152 | 1 |
| TESTI4035063 | 0.001095278 | 135437 | 1 |
| TESTI4035065 | 0.002190557 | 177081 | 2 |
| TESTI4035498 | 0.001095278 | 201060 | 1 |
| TESTI4035508 | 0.001095278 | 61455 | 1 |
| TESTI4035581 | 0.001095278 | 241996 | 1 |
| TESTI4035602 | 0.001095278 | 192094 | 1 |
| TESTI4035637 | 0.001095278 | 248492 | 1 |
| TESTI4035649 | 0.001095278 | 247937 | 1 |
| TESTI4035770 | 0.00317679 | 244389 | 2 |
| TESTI4035872 | 0.001095278 | 239976 | 1 |
| TESTI4035895 | 0.001095278 | 282194 | 1 |
| TESTI4035898 | 0.001095278 | 117895 | 1 |
| TESTI4035989 | 0.001095278 | 254379 | 1 |
| TESTI4036012 | 0.001095278 | 234842 | 1 |
| TESTI4036042 | 0.001095278 | 240025 | 1 |
| TESTI4036048 | 0.001095278 | 269260 | 1 |
| TESTI4036315 | 0.001095278 | 254350 | 1 |
| TESTI4036319 | 0.001095278 | 232367 | 1 |
| TESTI4036449 | 0.001095278 | 216173 | 1 |
| TESTI4036767 | 0.001095278 | 255550 | 1 |
| TESTI4036909 | 0.001095278 | 235074 | 1 |
| TESTI4037066 | 0.001095278 | 125935 | 1 |
| TESTI4037156 | 0.605504292 | 66227 | 79 |
| TESTI4037188 | 0.002783555 | 166357 | 2 |
| TESTI4037228 | 0.001095278 | 258145 | 1 |
| TESTI4037244 | 0.002190557 | 255818 | 2 |
| TESTI4037618 | 0.022532564 | 98637 | 12 |
| TESTI4037727 | 0.001095278 | 207484 | 1 |
| TESTI4037949 | 0.001095278 | 111330 | 1 |
| TESTI4038047 | 0.001095278 | 240753 | 1 |
| TESTI4038099 | 0.007595886 | 50411 | 5 |
| TESTI4038156 | 0.001095278 | 244899 | 1 |
| TESTI4038223 | 0.001095278 | 224171 | 1 |
| TESTI4038258 | 0.001095278 | 198921 | 1 |
| TESTI4038284 | 0.090980089 | 39991 | 29 |
| TESTI4038339 | 0.001095278 | 96909 | 1 |
| TESTI4038492 | 0.001095278 | 198910 | 1 |
| TESTI4038721 | 0.002190557 | 149050 | 2 |
| TESTI4038758 | 0.001095278 | 211918 | 1 |
| TESTI4038779 | 0.001095278 | 206960 | 1 |
| TESTI4038818 | 0.001095278 | 210845 | 1 |
| TESTI4039038 | 0.001095278 | 249909 | 1 |
| TESTI4039086 | 0.001095278 | 231191 | 1 |
| TESTI4039451 | 0.001095278 | 185954 | 1 |
| TESTI4039575 | 0.001095278 | 59556 | 1 |
| TESTI4039659 | 0.001095278 | 199768 | 1 |
| TESTI4039744 | 0.001095278 | 197328 | 1 |
| TESTI4039904 | 0.001095278 | 18953 | 1 |
| TESTI4040197 | 0.001095278 | 235500 | 1 |
| TESTI4040363 | 0.001095278 | 106961 | 1 |
| TESTI4040535 | 0.001095278 | 225793 | 1 |
| TESTI4040559 | 0.002190557 | 185320 | 2 |
| TESTI4040598 | 0.039147029 | 199530 | 2 |
| TESTI4040800 | 0.002190557 | 170359 | 2 |
| TESTI4040804 | 0.001095278 | 210697 | 1 |
| TESTI4040939 | 0.001095278 | 281109 | 1 |
| TESTI4040956 | 0.001095278 | 249496 | 1 |
| TESTI4041049 | 0.001095278 | 284852 | 1 |
| TESTI4041053 | 0.001095278 | 254785 | 1 |
| TESTI4041086 | 0.001095278 | 230544 | 1 |
| TESTI4041099 | 0.002190557 | 118090 | 2 |
| TESTI4041143 | 0.001095278 | 230829 | 1 |
| TESTI4041482 | 0.001095278 | 37801 | 1 |
| TESTI4041519 | 0.001095278 | 235446 | 1 |
| TESTI4041624 | 0.001095278 | 254663 | 1 |
| TESTI4041629 | 0.001095278 | 281176 | 1 |
| TESTI4041832 | 0.001095278 | 247573 | 1 |
| TESTI4041844 | 0.001095278 | 247548 | 1 |
| TESTI4041903 | 0.001095278 | 230867 | 1 |
| TESTI4041954 | 0.001095278 | 247608 | 1 |
| TESTI4041984 | 0.002190557 | 76745 | 2 |
| TESTI4041985 | 0.001095278 | 226807 | 1 |
| TESTI4041986 | 0.001095278 | 218600 | 1 |
| TESTI4042098 | 0.001095278 | 284723 | 1 |
| TESTI4042420 | 0.001095278 | 218571 | 1 |
| TESTI4042440 | 0.001095278 | 124401 | 1 |
| TESTI4042444 | 0.001095278 | 209142 | 1 |
| TESTI4042711 | 0.001095278 | 206775 | 1 |
| TESTI4042846 | 0.001095278 | 230935 | 1 |
| TESTI4043067 | 0.001095278 | 180443 | 1 |
| TESTI4043129 | 0.001095278 | 225818 | 1 |
| TESTI4043140 | 0.060428516 | 131310 | 17 |
| TESTI4043166 | 0.002190557 | 212598 | 2 |
| TESTI4043203 | 0.001095278 | 180026 | 1 |
| TESTI4043223 | 0.006054311 | 138492 | 4 |
| TESTI4043371 | 0.001095278 | 212595 | 1 |
| TESTI4043378 | 0.001095278 | 203763 | 1 |
| TESTI4043512 | 0.002190557 | 221950 | 2 |
| TESTI4043551 | 0.001095278 | 277418 | 1 |
| TESTI4043710 | 0.001095278 | 239618 | 1 |
| TESTI4043947 | 0.001095278 | 218542 | 1 |
| TESTI4044035 | 0.001095278 | 235563 | 1 |
| TESTI4044076 | 0.001095278 | 236416 | 1 |
| TESTI4044084 | 0.001095278 | 240444 | 1 |
| TESTI4044123 | 0.001095278 | 256132 | 1 |
| TESTI4044186 | 0.001095278 | 252354 | 1 |
| TESTI4044234 | 0.001095278 | 240637 | 1 |
| TESTI4044291 | 0.002190557 | 237908 | 2 |
| TESTI4044296 | 0.001095278 | 252319 | 1 |
| TESTI4044300 | 0.001095278 | 252320 | 1 |
| TESTI4044682 | 0.001095278 | 252417 | 1 |
| TESTI4044704 | 0.032874613 | 64394 | 9 |
| TESTI4044770 | 0.001095278 | 235568 | 1 |
| TESTI4045168 | 0.001095278 | 212690 | 1 |
| TESTI4045312 | 0.002190557 | 120404 | 2 |
| TESTI4045330 | 0.001095278 | 181281 | 1 |
| TESTI4045470 | 0.001095278 | 144924 | 1 |
| TESTI4045701 | 0.001095278 | 158326 | 1 |
| TESTI4045908 | 0.001095278 | 231426 | 1 |
| TESTI4045955 | 0.001095278 | 266764 | 1 |
| TESTI4045982 | 0.001095278 | 199723 | 1 |
| TESTI4046073 | 0.008825121 | 157549 | 3 |
| TESTI4046090 | 0.003285835 | 226844 | 3 |
| TESTI4046240 | 0.001095278 | 221953 | 1 |
| TESTI4046245 | 0.001095278 | 79139 | 1 |
| TESTI4046253 | 0.001095278 | 206709 | 1 |
| TESTI4046282 | 0.001095278 | 185412 | 1 |
| TESTI4046328 | 0.001095278 | 230649 | 1 |
| TESTI4046450 | 0.001095278 | 120184 | 1 |
| TESTI4046457 | 0.001095278 | 148584 | 1 |
| TESTI4046487 | 0.001095278 | 182480 | 1 |
| TESTI4046502 | 0.003866104 | 96815 | 3 |
| TESTI4046523 | 0.001095278 | 218154 | 1 |
| TESTI4046819 | 0.008486842 | 121011 | 3 |
| TESTI4046873 | 0.004077237 | 219380 | 2 |
| TESTI4046884 | 0.001095278 | 196134 | 1 |
| TESTI4046896 | 0.001095278 | 214282 | 1 |
| TESTI4046903 | 0.001095278 | 225700 | 1 |
| TESTI4046962 | 0.001095278 | 182857 | 1 |
| TESTI4047069 | 0.001095278 | 176966 | 1 |
| TESTI4047119 | 0.002190557 | 172789 | 2 |
| TESTI4047305 | 0.001095278 | 239962 | 1 |
| TESTI4047328 | 0.001095278 | 226854 | 1 |
| TESTI4047437 | 0.001095278 | 248013 | 1 |
| TESTI4047569 | 0.002190557 | 51155 | 2 |
| TESTI4047746 | 0.001095278 | 223052 | 1 |
| TESTI4047808 | 0.002190557 | 180518 | 2 |
| THYMU1000002 | 0.283081539 | 13295 | 31 |
| THYMU1000010 | 0.062156154 | 9982 | 7 |
| THYMU1000016 | 0.433589622 | 8513 | 43 |
| THYMU1000017 | 0.00557409 | 61283 | 2 |
| THYMU1000025 | 0.001397155 | 67971 | 1 |
| THYMU1000029 | 0.003106616 | 68402 | 2 |
| THYMU1000032 | 0.2146652 | 29347 | 91 |
| THYMU1000033 | 0.001397155 | 71495 | 1 |
| THYMU1000041 | 0.070464806 | 77450 | 30 |
| THYMU1000060 | 0.001397155 | 46511 | 1 |
| THYMU1000074 | 0.001397155 | 76856 | 1 |
| THYMU1000083 | 0.055878048 | 57909 | 21 |
| THYMU1000098 | 0.005588622 | 67676 | 4 |
| THYMU1000103 | 0.095037005 | 31692 | 41 |
| THYMU1000105 | 0.01116705 | 39932 | 4 |
| THYMU1000109 | 0.212893133 | 67579 | 24 |
| THYMU1000136 | 0.001397155 | 12463 | 1 |
| THYMU1000142 | 0.003395557 | 38666 | 2 |
| THYMU1000144 | 0.129554365 | 13387 | 68 |
| THYMU1000158 | 0.001397155 | 77621 | 1 |
| THYMU1000176 | 0.001397155 | 76265 | 1 |
| THYMU1000179 | 0.003273997 | 66210 | 2 |
| THYMU1000191 | 0.004191466 | 89223 | 3 |
| THYMU1000198 | 0.003085432 | 14759 | 2 |
| THYMU1000217 | 0.001397155 | 30221 | 1 |
| THYMU1000240 | 0.001397155 | 110687 | 1 |
| THYMU1000242 | 0.001397155 | 267162 | 1 |
| THYMU1000291 | 0.001397155 | 79043 | 1 |
| THYMU1000316 | 0.003395557 | 185601 | 2 |
| THYMU1000318 | 0.001397155 | 263995 | 1 |
| THYMU1000329 | 0.031439043 | 137063 | 7 |
| THYMU1000331 | 0.003681678 | 133321 | 3 |
| THYMU1000336 | 0.001397155 | 258167 | 1 |
| THYMU1000359 | 0.312579475 | 109518 | 78 |
| THYMU1000366 | 0.001397155 | 273762 | 1 |
| THYMU1000374 | 0.111870084 | 34316 | 36 |
| THYMU1000382 | 0.001397155 | 250667 | 1 |
| THYMU1000393 | 0.001397155 | 265725 | 1 |
| THYMU1000394 | 0.034085076 | 73770 | 9 |
| THYMU1000399 | 0.001397155 | 261134 | 1 |
| THYMU1000426 | 0.001397155 | 119256 | 1 |
| THYMU1000428 | 0.294780918 | 72160 | 29 |
| THYMU1000440 | 0.002794311 | 276993 | 2 |
| THYMU1000459 | 0.004191466 | 113570 | 3 |
| THYMU1000473 | 0.050373735 | 46668 | 3 |
| THYMU1000490 | 0.029220305 | 123055 | 11 |
| THYMU1000491 | 0.001397155 | 89146 | 1 |
| THYMU1000496 | 0.001397155 | 237850 | 1 |
| THYMU1000499 | 0.029356753 | 146535 | 5 |
| THYMU1000527 | 0.001397155 | 265406 | 1 |
| THYMU1000532 | 0.005588622 | 59728 | 4 |
| THYMU1000536 | 0.001397155 | 265405 | 1 |
| THYMU1000554 | 0.001397155 | 251027 | 1 |
| THYMU1000558 | 0.001397155 | 285168 | 1 |
| THYMU1000576 | 0.001397155 | 281822 | 1 |
| THYMU1000600 | 0.002794311 | 230476 | 2 |
| THYMU1000677 | 0.001397155 | 137997 | 1 |
| THYMU1000692 | 0.001397155 | 120913 | 1 |
| THYMU2000022 | 0.001397155 | 188729 | 1 |
| THYMU2000032 | 0.001397155 | 164054 | 1 |
| THYMU2000057 | 0.123541998 | 149586 | 19 |
| THYMU2000120 | 0.004312012 | 21197 | 2 |
| THYMU2000138 | 0.007907695 | 117960 | 5 |
| THYMU2000140 | 0.001397155 | 46673 | 1 |
| THYMU2000155 | 0.00325527 | 218203 | 2 |
| THYMU2000162 | 0.0025864 | 167097 | 2 |
| THYMU2000171 | 0.001397155 | 264286 | 1 |
| THYMU2000176 | 0.001397155 | 213541 | 1 |
| THYMU2000179 | 0.001397155 | 213542 | 1 |
| THYMU2000231 | 0.002794311 | 216378 | 2 |
| THYMU2000236 | 0.007633513 | 140097 | 2 |
| THYMU2000276 | 0.001397155 | 226351 | 1 |
| THYMU2000280 | 0.001397155 | 141333 | 1 |
| THYMU2000300 | 0.057353 | 113559 | 32 |
| THYMU2000317 | 0.001397155 | 214584 | 1 |
| THYMU2000368 | 0.001397155 | 17269 | 1 |
| THYMU2000369 | 0.00932013 | 157899 | 3 |
| THYMU2000382 | 0.276441727 | 126769 | 37 |
| THYMU2000418 | 0.001397155 | 218676 | 1 |
| THYMU2000436 | 0.18184934 | 102755 | 32 |
| THYMU2000440 | 0.001397155 | 38752 | 1 |
| THYMU2000489 | 0.003085432 | 158002 | 2 |
| THYMU2000492 | 0.001397155 | 271322 | 1 |
| THYMU2000519 | 0.001397155 | 205302 | 1 |
| THYMU2000570 | 0.001397155 | 167100 | 1 |
| THYMU2000602 | 0.001397155 | 5421 | 1 |
| THYMU2000660 | 0.001397155 | 170352 | 1 |
| THYMU2000671 | 0.001397155 | 236649 | 1 |
| THYMU2000672 | 0.007407508 | 113346 | 3 |
| THYMU2000684 | 0.040243433 | 91855 | 13 |
| THYMU2000702 | 0.053548641 | 85768 | 4 |
| THYMU2000767 | 0.001397155 | 104070 | 1 |
| THYMU2000775 | 0.01789626 | 21245 | 5 |
| THYMU2000800 | 0.001397155 | 211475 | 1 |
| THYMU2000879 | 0.001397155 | 226164 | 1 |
| THYMU2000932 | 0.001397155 | 245668 | 1 |
| THYMU2000946 | 0.115810319 | 99735 | 28 |
| THYMU2000950 | 0.007599475 | 18146 | 2 |
| THYMU2000971 | 0.00325527 | 132607 | 2 |
| THYMU2000982 | 0.001397155 | 230500 | 1 |
| THYMU2000990 | 0.001397155 | 226155 | 1 |
| THYMU2001007 | 0.061762722 | 78235 | 24 |
| THYMU2001018 | 0.018552145 | 174440 | 5 |
| THYMU2001053 | 0.001397155 | 281450 | 1 |
| THYMU2001071 | 0.001397155 | 231544 | 1 |
| THYMU2001090 | 0.007884209 | 161294 | 5 |
| THYMU2001139 | 0.001397155 | 213952 | 1 |
| THYMU2001195 | 0.004312012 | 17886 | 2 |
| THYMU2001202 | 0.001397155 | 235823 | 1 |
| THYMU2001203 | 0.001397155 | 226245 | 1 |
| THYMU2001256 | 0.001397155 | 231602 | 1 |
| THYMU2001270 | 0.001397155 | 230491 | 1 |
| THYMU2001291 | 0.001397155 | 167535 | 1 |
| THYMU2001314 | 0.005588622 | 151081 | 4 |
| THYMU2001325 | 0.001397155 | 81069 | 1 |
| THYMU2001357 | 0.001397155 | 113813 | 1 |
| THYMU2001381 | 0.024703459 | 113378 | 3 |
| THYMU2001422 | 0.01345275 | 174585 | 4 |
| THYMU2001443 | 0.001397155 | 102286 | 1 |
| THYMU2001515 | 0.011489548 | 69152 | 8 |
| THYMU2001521 | 0.042153599 | 145191 | 8 |
| THYMU2001556 | 0.001397155 | 226228 | 1 |
| THYMU2001622 | 0.001397155 | 118701 | 1 |
| THYMU2001727 | 0.001397155 | 249353 | 1 |
| THYMU2001819 | 0.007309767 | 159938 | 2 |
| THYMU2001825 | 0.002794311 | 66223 | 2 |
| THYMU2001839 | 0.010709459 | 103674 | 5 |
| THYMU2001868 | 0.005241239 | 223394 | 2 |
| THYMU2001898 | 0.001397155 | 136490 | 1 |
| THYMU2001900 | 0.001397155 | 203418 | 1 |
| THYMU2001916 | 0.001397155 | 211487 | 1 |
| THYMU2001926 | 0.008632811 | 18811 | 4 |
| THYMU2001989 | 0.001397155 | 230443 | 1 |
| THYMU2002023 | 0.012503049 | 59355 | 3 |
| THYMU2002037 | 0.001397155 | 238830 | 1 |
| THYMU2002080 | 0.01125891 | 63720 | 4 |
| THYMU2002109 | 0.001397155 | 165753 | 1 |
| THYMU2002147 | 0.007106323 | 69160 | 4 |
| THYMU2002154 | 0.004191466 | 230045 | 3 |
| THYMU2002157 | 0.001397155 | 235988 | 1 |
| THYMU2002162 | 0.01536891 | 16884 | 6 |
| THYMU2002177 | 0.001397155 | 269644 | 1 |
| THYMU2002203 | 0.001397155 | 127210 | 1 |
| THYMU2002229 | 0.015388769 | 242218 | 3 |
| THYMU2002242 | 0.001397155 | 107768 | 1 |
| THYMU2002338 | 0.002794311 | 39389 | 2 |
| THYMU2002356 | 0.001397155 | 96959 | 1 |
| THYMU2002376 | 0.001397155 | 249176 | 1 |
| THYMU2002384 | 0.001397155 | 218810 | 1 |
| THYMU2002450 | 0.007766648 | 145261 | 4 |
| THYMU2002490 | 0.001397155 | 41710 | 1 |
| THYMU2002548 | 0.001397155 | 209027 | 1 |
| THYMU2002583 | 0.323295964 | 88940 | 60 |
| THYMU2002695 | 0.001397155 | 156732 | 1 |
| THYMU2002745 | 0.043765852 | 149146 | 3 |
| THYMU2002756 | 0.022887652 | 14610 | 6 |
| THYMU2002789 | 0.001397155 | 255784 | 1 |
| THYMU2002792 | 0.001397155 | 127283 | 1 |
| THYMU2002815 | 0.005071104 | 132176 | 3 |
| THYMU2002841 | 0.057734742 | 172937 | 10 |
| THYMU2002852 | 0.001397155 | 235585 | 1 |
| THYMU2002910 | 0.001397155 | 235582 | 1 |
| THYMU2002950 | 0.001397155 | 85939 | 1 |
| THYMU2002983 | 0.055988425 | 77410 | 7 |
| THYMU2003012 | 0.191195817 | 6891 | 44 |
| THYMU2003046 | 0.087161916 | 100940 | 30 |
| THYMU2003069 | 0.037910844 | 98201 | 26 |
| THYMU2003107 | 0.001397155 | 135046 | 1 |
| THYMU2003124 | 0.001397155 | 114586 | 1 |
| THYMU2003133 | 0.048292691 | 131790 | 13 |
| THYMU2003166 | 0.001397155 | 172749 | 1 |
| THYMU2003232 | 0.001397155 | 226146 | 1 |
| THYMU2003242 | 0.001397155 | 205214 | 1 |
| THYMU2003249 | 0.001397155 | 200892 | 1 |
| THYMU2003270 | 0.001397155 | 226126 | 1 |
| THYMU2003282 | 0.001397155 | 206536 | 1 |
| THYMU2003287 | 0.0140921 | 124677 | 9 |
| THYMU2003336 | 0.001397155 | 235876 | 1 |
| THYMU2003366 | 0.001397155 | 11709 | 1 |
| THYMU2003397 | 0.011177243 | 110175 | 8 |
| THYMU2003419 | 0.333649462 | 50383 | 87 |
| THYMU2003440 | 0.007202334 | 73697 | 2 |
| THYMU2003446 | 0.070252924 | 41188 | 33 |
| THYMU2003470 | 0.001397155 | 194968 | 1 |
| THYMU2003479 | 0.001397155 | 180030 | 1 |
| THYMU2003499 | 0.011116458 | 205584 | 6 |
| THYMU2003542 | 0.077533087 | 129557 | 9 |
| THYMU2003632 | 0.001397155 | 276680 | 1 |
| THYMU2003650 | 0.001397155 | 123904 | 1 |
| THYMU2003700 | 0.001397155 | 211463 | 1 |
| THYMU2003760 | 0.001397155 | 280339 | 1 |
| THYMU2003803 | 0.001397155 | 282132 | 1 |
| THYMU2003855 | 0.001397155 | 123448 | 1 |
| THYMU2003891 | 1.148350684 | 17403 | 123 |
| THYMU2003907 | 0.01477468 | 103087 | 3 |
| THYMU2003932 | 0.00866256 | 98542 | 5 |
| THYMU2003981 | 0.320836355 | 113852 | 56 |
| THYMU2004003 | 0.001397155 | 241020 | 1 |
| THYMU2004008 | 0.002794311 | 247799 | 2 |
| THYMU2004042 | 0.001397155 | 226055 | 1 |
| THYMU2004108 | 0.012683837 | 193524 | 2 |
| THYMU2004111 | 0.001397155 | 250531 | 1 |
| THYMU2004123 | 0.001397155 | 51144 | 1 |
| THYMU2004139 | 0.001397155 | 226341 | 1 |
| THYMU2004152 | 0.168753016 | 120744 | 37 |
| THYMU2004169 | 0.001397155 | 135410 | 1 |
| THYMU2004284 | 0.02494276 | 163753 | 4 |
| THYMU2004320 | 0.010254136 | 130070 | 3 |
| THYMU2004336 | 0.002794311 | 122295 | 2 |
| THYMU2004344 | 0.131161861 | 116807 | 34 |
| THYMU2004356 | 0.001397155 | 89238 | 1 |
| THYMU2004410 | 0.087450229 | 54463 | 31 |
| THYMU2004452 | 0.007249637 | 99444 | 3 |
| THYMU2004508 | 0.0025864 | 116253 | 2 |
| THYMU2004512 | 0.001397155 | 205079 | 1 |
| THYMU2004635 | 0.003106616 | 32346 | 2 |
| THYMU2004639 | 0.001397155 | 123890 | 1 |
| THYMU2004668 | 0.001397155 | 283209 | 1 |
| THYMU2004677 | 0.001397155 | 198298 | 1 |
| THYMU2004688 | 0.001397155 | 40823 | 1 |
| THYMU2004693 | 0.001397155 | 195197 | 1 |
| THYMU2004733 | 0.029917801 | 88328 | 7 |
| THYMU2004835 | 0.001397155 | 218972 | 1 |
| THYMU2004843 | 0.001397155 | 105346 | 1 |
| THYMU2004906 | 0.013368594 | 3338 | 4 |
| THYMU2004916 | 0.107375141 | 47990 | 8 |
| THYMU2004986 | 0.002794311 | 130714 | 2 |
| THYMU2005001 | 0.001397155 | 195704 | 1 |
| THYMU2005003 | 0.011999361 | 120273 | 2 |
| THYMU2005046 | 0.001397155 | 219011 | 1 |
| THYMU2005134 | 0.001397155 | 162675 | 1 |
| THYMU2005156 | 0.004379115 | 195188 | 2 |
| THYMU2005190 | 0.001397155 | 198500 | 1 |
| THYMU2005225 | 0.001397155 | 216662 | 1 |
| THYMU2005240 | 0.001397155 | 272225 | 1 |
| THYMU2005246 | 0.003273997 | 68958 | 2 |
| THYMU2005270 | 0.001397155 | 82751 | 1 |
| THYMU2005283 | 0.001397155 | 240531 | 1 |
| THYMU2005303 | 0.001397155 | 32451 | 1 |
| THYMU2005321 | 0.001397155 | 280807 | 1 |
| THYMU2005356 | 0.001397155 | 85840 | 1 |
| THYMU2005369 | 0.001397155 | 223207 | 1 |
| THYMU2005439 | 0.030312595 | 48998 | 8 |
| THYMU2005480 | 0.001397155 | 226352 | 1 |
| THYMU2005482 | 0.013617142 | 185054 | 3 |
| THYMU2005507 | 0.004191466 | 163700 | 3 |
| THYMU2005545 | 0.001397155 | 240591 | 1 |
| THYMU2005546 | 0.306336962 | 129372 | 30 |
| THYMU2005574 | 0.0025864 | 209727 | 2 |
| THYMU2005576 | 0.001397155 | 228651 | 1 |
| THYMU2005748 | 0.001397155 | 225873 | 1 |
| THYMU2005759 | 0.001397155 | 252386 | 1 |
| THYMU2005807 | 0.0025864 | 171405 | 2 |
| THYMU2005815 | 0.001397155 | 249428 | 1 |
| THYMU2005855 | 0.001397155 | 245398 | 1 |
| THYMU2005881 | 0.001397155 | 240921 | 1 |
| THYMU2005948 | 0.001397155 | 148630 | 1 |
| THYMU2005980 | 0.020662708 | 73188 | 6 |
| THYMU2005997 | 0.010405633 | 119820 | 4 |
| THYMU2006001 | 0.001397155 | 160646 | 1 |
| THYMU2006048 | 0.001397155 | 197193 | 1 |
| THYMU2006098 | 0.001397155 | 135808 | 1 |
| THYMU2006160 | 0.002794311 | 236411 | 2 |
| THYMU2006170 | 0.002794311 | 216808 | 2 |
| THYMU2006252 | 0.001397155 | 207395 | 1 |
| THYMU2006261 | 0.005778268 | 171063 | 5 |
| THYMU2006277 | 0.144458672 | 216753 | 2 |
| THYMU2006303 | 0.001397155 | 97974 | 1 |
| THYMU2006324 | 0.001397155 | 211400 | 1 |
| THYMU2006350 | 0.001397155 | 231285 | 1 |
| THYMU2006357 | 0.001397155 | 118625 | 1 |
| THYMU2006365 | 0.001397155 | 27946 | 1 |
| THYMU2006420 | 0.001397155 | 10463 | 1 |
| THYMU2006468 | 0.001397155 | 131355 | 1 |
| THYMU2006505 | 0.122740821 | 55164 | 35 |
| THYMU2006610 | 0.012340254 | 122170 | 6 |
| THYMU2006666 | 0.001397155 | 85491 | 1 |
| THYMU2006712 | 0.001397155 | 243554 | 1 |
| THYMU2006813 | 0.105889362 | 102938 | 37 |
| THYMU2006913 | 0.001397155 | 250784 | 1 |
| THYMU2006946 | 0.001397155 | 201499 | 1 |
| THYMU2006965 | 0.001397155 | 219135 | 1 |
| THYMU2007025 | 0.001397155 | 37260 | 1 |
| THYMU2007036 | 0.021701029 | 185348 | 6 |
| THYMU2007060 | 0.039300517 | 121218 | 9 |
| THYMU2007065 | 0.041320358 | 154386 | 11 |
| THYMU2007095 | 0.001397155 | 222962 | 1 |
| THYMU2007112 | 0.036637237 | 141088 | 11 |
| THYMU2007146 | 0.00468299 | 193971 | 4 |
| THYMU2007179 | 0.001397155 | 76348 | 1 |
| THYMU2007258 | 0.001397155 | 252452 | 1 |
| THYMU2007307 | 0.001397155 | 239129 | 1 |
| THYMU2007308 | 0.001397155 | 241273 | 1 |
| THYMU2007339 | 0.005435438 | 255387 | 2 |
| THYMU2007350 | 0.001397155 | 61642 | 1 |
| THYMU2007384 | 0.001397155 | 253581 | 1 |
| THYMU2007415 | 0.069241641 | 84113 | 17 |
| THYMU2007467 | 0.041480917 | 99353 | 10 |
| THYMU2007493 | 0.001397155 | 124277 | 1 |
| THYMU2007521 | 0.001397155 | 184192 | 1 |
| THYMU2007528 | 0.001397155 | 166537 | 1 |
| THYMU2007530 | 0.001397155 | 168942 | 1 |
| THYMU2007532 | 0.010725521 | 14745 | 4 |
| THYMU2007561 | 0.001397155 | 184189 | 1 |
| THYMU2007571 | 0.001397155 | 161676 | 1 |
| THYMU2007635 | 0.002794311 | 61668 | 2 |
| THYMU2007658 | 0.001397155 | 201661 | 1 |
| THYMU2007667 | 0.001397155 | 172725 | 1 |
| THYMU2007694 | 0.008081801 | 97753 | 4 |
| THYMU2007725 | 0.001397155 | 113578 | 1 |
| THYMU2007729 | 0.034283517 | 152349 | 9 |
| THYMU2007802 | 0.00557409 | 149659 | 2 |
| THYMU2007824 | 0.001397155 | 241382 | 1 |
| THYMU2007854 | 0.001397155 | 219027 | 1 |
| THYMU2007886 | 0.121006836 | 97367 | 15 |
| THYMU2007952 | 0.001397155 | 197100 | 1 |
| THYMU2007969 | 0.001397155 | 245086 | 1 |
| THYMU2008035 | 0.003072702 | 120061 | 2 |
| THYMU2008036 | 0.003085432 | 83950 | 2 |
| THYMU2008049 | 0.007418294 | 127946 | 4 |
| THYMU2008072 | 0.001397155 | 192864 | 1 |
| THYMU2008111 | 0.004191466 | 96903 | 3 |
| THYMU2008145 | 0.001397155 | 126326 | 1 |
| THYMU2008149 | 0.02844597 | 131914 | 19 |
| THYMU2008185 | 0.058086498 | 14325 | 2 |
| THYMU2008191 | 0.001397155 | 164370 | 1 |
| THYMU2008207 | 0.001397155 | 158332 | 1 |
| THYMU2008226 | 0.001397155 | 180158 | 1 |
| THYMU2008282 | 0.001397155 | 97258 | 1 |
| THYMU2008321 | 0.057960839 | 80930 | 18 |
| THYMU2008339 | 0.001397155 | 166379 | 1 |
| THYMU2008350 | 0.001397155 | 192832 | 1 |
| THYMU2008351 | 0.002794311 | 58947 | 2 |
| THYMU2008383 | 0.002492434 | 118057 | 2 |
| THYMU2008452 | 0.002794311 | 250986 | 2 |
| THYMU2008643 | 0.001397155 | 279859 | 1 |
| THYMU2008686 | 0.013749135 | 100709 | 5 |
| THYMU2008691 | 0.001397155 | 124056 | 1 |
| THYMU2008714 | 0.001397155 | 190749 | 1 |
| THYMU2008725 | 0.012850033 | 145398 | 3 |
| THYMU2008727 | 0.011271748 | 148652 | 2 |
| THYMU2008781 | 0.006985777 | 75637 | 5 |
| THYMU2008799 | 0.001397155 | 133359 | 1 |
| THYMU2008876 | 0.001397155 | 141859 | 1 |
| THYMU2008910 | 0.001397155 | 132079 | 1 |
| THYMU2008917 | 0.001397155 | 177344 | 1 |
| THYMU2008928 | 0.001397155 | 274274 | 1 |
| THYMU2008990 | 0.001397155 | 31522 | 1 |
| THYMU2008994 | 0.001397155 | 280745 | 1 |
| THYMU2009023 | 0.001397155 | 9173 | 1 |
| THYMU2009046 | 0.001397155 | 154501 | 1 |
| THYMU2009101 | 0.001397155 | 167772 | 1 |
| THYMU2009104 | 0.001397155 | 172698 | 1 |
| THYMU2009134 | 0.001397155 | 164435 | 1 |
| THYMU2009157 | 0.002794311 | 169155 | 2 |
| THYMU2009181 | 0.001397155 | 172709 | 1 |
| THYMU2009186 | 0.004312012 | 173089 | 2 |
| THYMU2009338 | 0.00325527 | 39623 | 2 |
| THYMU2009425 | 0.001397155 | 216245 | 1 |
| THYMU2009516 | 0.001397155 | 171898 | 1 |
| THYMU2009526 | 0.009780088 | 128458 | 7 |
| THYMU2009546 | 0.001397155 | 268640 | 1 |
| THYMU2009592 | 0.006065067 | 56883 | 4 |
| THYMU2009596 | 0.001397155 | 170468 | 1 |
| THYMU2009658 | 0.478334266 | 50760 | 34 |
| THYMU2009792 | 0.059828212 | 145358 | 4 |
| THYMU2009835 | 0.001397155 | 192848 | 1 |
| THYMU2009906 | 0.001397155 | 272886 | 1 |
| THYMU2009948 | 0.001397155 | 244597 | 1 |
| THYMU2010030 | 0.001397155 | 167800 | 1 |
| THYMU2010041 | 0.008986358 | 131106 | 6 |
| THYMU2010046 | 0.001397155 | 177371 | 1 |
| THYMU2010059 | 0.001397155 | 161222 | 1 |
| THYMU2010082 | 0.001397155 | 203089 | 1 |
| THYMU2010094 | 0.028766841 | 119777 | 11 |
| THYMU2010161 | 0.011105893 | 197810 | 2 |
| THYMU2010192 | 0.00325527 | 180155 | 2 |
| THYMU2010448 | 0.031644047 | 101868 | 10 |
| THYMU2010519 | 0.021077877 | 167975 | 14 |
| THYMU2010637 | 0.001397155 | 51179 | 1 |
| THYMU2010671 | 0.001397155 | 172636 | 1 |
| THYMU2010699 | 0.001397155 | 4596 | 1 |
| THYMU2010705 | 0.001397155 | 184170 | 1 |
| THYMU2010779 | 0.003983555 | 152956 | 3 |
| THYMU2010816 | 0.01285287 | 143581 | 5 |
| THYMU2010831 | 0.003072702 | 38475 | 2 |
| THYMU2010848 | 0.001397155 | 137753 | 1 |
| THYMU2010917 | 0.022248538 | 114970 | 7 |
| THYMU2011053 | 0.003273997 | 195135 | 2 |
| THYMU2011063 | 0.031843421 | 22493 | 5 |
| THYMU2011072 | 0.001397155 | 102364 | 1 |
| THYMU2011096 | 0.001397155 | 180700 | 1 |
| THYMU2011118 | 0.001397155 | 123798 | 1 |
| THYMU2011142 | 0.004191466 | 172498 | 3 |
| THYMU2011183 | 0.006985777 | 98200 | 5 |
| THYMU2011257 | 0.001397155 | 192913 | 1 |
| THYMU2011259 | 0.001397155 | 79034. | 1 |
| THYMU2011295 | 0.001397155 | 183201 | 1 |
| THYMU2011308 | 0.039913836 | 116322 | 3 |
| THYMU2011390 | 0.001397155 | 29218 | 1 |
| THYMU2011431 | 0.001397155 | 172672 | 1 |
| THYMU2011447 | 0.001397155 | 153158 | 1 |
| THYMU2011482 | 0.054584543 | 71775 | 17 |
| THYMU2011538 | 0.001397155 | 168902 | 1 |
| THYMU2011548 | 0.001397155 | 204771 | 1 |
| THYMU2011551 | 0.001397155 | 127496 | 1 |
| THYMU2011564 | 0.001397155 | 40916 | 1 |
| THYMU2011573 | 0.001397155 | 155100 | 1 |
| THYMU2011585 | 0.004191466 | 264324 | 3 |
| THYMU2011621 | 0.001397155 | 154007 | 1 |
| THYMU2011666 | 0.011177243 | 163294 | 8 |
| THYMU2011736 | 0.00557409 | 72233 | 2 |
| THYMU2011785 | 0.001397155 | 180698 | 1 |
| THYMU2011806 | 0.001397155 | 148285 | 1 |
| THYMU2011852 | 0.001397155 | 152430 | 1 |
| THYMU2011875 | 0.007469538 | 199092 | 2 |
| THYMU2011881 | 0.016073126 | 156819 | 3 |
| THYMU2011937 | 0.001397155 | 279317 | 1 |
| THYMU2011939 | 0.0025864 | 203373 | 2 |
| THYMU2012002 | 0.006599474 | 14979 | 3 |
| THYMU2012024 | 0.056226295 | 114189 | 15 |
| THYMU2012073 | 0.001397155 | 61274 | 1 |
| THYMU2012104 | 0.006193509 | 38205 | 3 |
| THYMU2012113 | 0.001397155 | 113778 | 1 |
| THYMU2012273 | 0.001397155 | 3666 | 1 |
| THYMU2012401 | 0.007238618 | 195013 | 2 |
| THYMU2012492 | 0.012815491 | 112715 | 7 |
| THYMU2012507 | 0.001397155 | 172871 | 1 |
| THYMU2012565 | 0.001397155 | 158110 | 1 |
| THYMU2012574 | 0.001397155 | 118116 | 1 |
| THYMU2012625 | 0.161294528 | 124991 | 11 |
| THYMU2012631 | 0.002794311 | 172294 | 2 |
| THYMU2012665 | 0.001397155 | 148278 | 1 |
| THYMU2012690 | 0.043119814 | 68520 | 4 |
| THYMU2012701 | 0.001397155 | 188948 | 1 |
| THYMU2012807 | 0.001397155 | 129598 | 1 |
| THYMU2012826 | 0.007565717 | 154250 | 5 |
| THYMU2012882 | 0.001397155 | 158285 | 1 |
| THYMU2012891 | 0.001397155 | 155982 | 1 |
| THYMU2012902 | 0.001397155 | 159799 | 1 |
| THYMU2012919 | 0.001397155 | 108607 | 1 |
| THYMU2013023 | 0.001397155 | 177290 | 1 |
| THYMU2013047 | 0.001397155 | 172911 | 1 |
| THYMU2013051 | 0.007438721 | 76120 | 2 |
| THYMU2013089 | 0.001397155 | 32491 | 1 |
| THYMU2013136 | 0.204858728 | 36632 | 20 |
| THYMU2013170 | 0.007309417 | 177296 | 2 |
| THYMU2013364 | 0.001397155 | 165267 | 1 |
| THYMU2013386 | 0.001397155 | 133799 | 1 |
| THYMU2013426 | 0.001397155 | 167318 | 1 |
| THYMU2013578 | 0.001397155 | 221338 | 1 |
| THYMU2013648 | 0.013991614 | 77052 | 2 |
| THYMU2013705 | 0.001397155 | 71287 | 1 |
| THYMU2013757 | 0.001397155 | 150630 | 1 |
| THYMU2013779 | 0.001397155 | 3699 | 1 |
| THYMU2013863 | 0.001397155 | 32449 | 1 |
| THYMU2013916 | 0.151068608 | 41565 | 42 |
| THYMU2013950 | 0.001397155 | 108348 | 1 |
| THYMU2013981 | 0.001397155 | 158094 | 1 |
| THYMU2014051 | 0.059603321 | 107170 | 4 |
| THYMU2014067 | 0.011105893 | 111895 | 2 |
| THYMU2014068 | 0.001397155 | 274078 | 1 |
| THYMU2014167 | 0.030491603 | 20856 | 9 |
| THYMU2014183 | 0.017246884 | 99909 | 4 |
| THYMU2014204 | 0.001397155 | 165302 | 1 |
| THYMU2014297 | 0.001397155 | 68839 | 1 |
| THYMU2014323 | 0.003085432 | 181696 | 2 |
| THYMU2014327 | 0.001397155 | 49618 | 1 |
| THYMU2014353 | 0.004482587 | 170502 | 3 |
| THYMU2014492 | 0.008706922 | 93447 | 3 |
| THYMU2014500 | 0.001397155 | 167359 | 1 |
| THYMU2014599 | 0.005915117 | 42203 | 3 |
| THYMU2014762 | 0.001397155 | 177203 | 1 |
| THYMU2014777 | 0.001397155 | 269518 | 1 |
| THYMU2014801 | 0.001397155 | 167368 | 1 |
| THYMU2014901 | 0.001397155 | 167331 | 1 |
| THYMU2014923 | 0.001397155 | 183375 | 1 |
| THYMU2015019 | 0.012248787 | 154608 | 3 |
| THYMU2015161 | 0.001397155 | 118937 | 1 |
| THYMU2015316 | 0.001397155 | 123494 | 1 |
| THYMU2015321 | 0.003395557 | 102346 | 2 |
| THYMU2015409 | 0.103117687 | 50384 | 38 |
| THYMU2015439 | 0.001397155 | 157715 | 1 |
| THYMU2015441 | 0.007451524 | 41373 | 2 |
| THYMU2015479 | 0.001397155 | 153197 | 1 |
| THYMU2015762 | 0.169012346 | 57682 | 13 |
| THYMU2015775 | 0.007542681 | 61993 | 2 |
| THYMU2015825 | 0.001397155 | 143702 | 1 |
| THYMU2015918 | 0.003106616 | 97379 | 2 |
| THYMU2015943 | 0.001397155 | 155327 | 1 |
| THYMU2015984 | 0.001397155 | 207 | 1 |
| THYMU2016113 | 0.001397155 | 125341 | 1 |
| THYMU2016164 | 0.001397155 | 72624 | 1 |
| THYMU2016204 | 0.003478667 | 144129 | 2 |
| THYMU2016219 | 0.001397155 | 149802 | 1 |
| THYMU2016360 | 0.001397155 | 165066 | 1 |
| THYMU2016496 | 0.001397155 | 93566 | 1 |
| THYMU2016523 | 0.005078834 | 170568 | 4 |
| THYMU2016840 | 0.007451524 | 37090 | 2 |
| THYMU2016968 | 0.022668431 | 82586 | 3 |
| THYMU2017008 | 0.001397155 | 103669 | 1 |
| THYMU2017010 | 0.001397155 | 89229 | 1 |
| THYMU2017023 | 0.001397155 | 233892 | 1 |
| THYMU2017085 | 0.001397155 | 4204 | 1 |
| THYMU2017099 | 0.001397155 | 14548 | 1 |
| THYMU2017158 | 0.003478667 | 272717 | 2 |
| THYMU2017162 | 0.001397155 | 205058 | 1 |
| THYMU2017215 | 0.001397155 | 64019 | 1 |
| THYMU2017362 | 0.001397155 | 172453 | 1 |
| THYMU2017366 | 0.001397155 | 62106 | 1 |
| THYMU2017398 | 0.001397155 | 150631 | 1 |
| THYMU2017449 | 0.001397155 | 183255 | 1 |
| THYMU2017479 | 0.002794311 | 253512 | 2 |
| THYMU2017522 | 0.001397155 | 276059 | 1 |
| THYMU2017526 | 0.001397155 | 183254 | 1 |
| THYMU2017601 | 0.023726744 | 98543 | 9 |
| THYMU2017675 | 0.001397155 | 142928 | 1 |
| THYMU2017707 | 0.001397155 | 637 | 1 |
| THYMU2017831 | 0.002492434 | 125343 | 2 |
| THYMU2017844 | 0.001397155 | 264332 | 1 |
| THYMU2017878 | 0.0025864 | 88949 | 2 |
| THYMU2017948 | 0.003889589 | 15722 | 3 |
| THYMU2018028 | 0.00557409 | 13810 | 2 |
| THYMU2018071 | 0.001397155 | 141318 | 1 |
| THYMU2018126 | 0.230309261 | 51293 | 10 |
| THYMU2018189 | 0.001397155 | 123002 | 1 |
| THYMU2018197 | 0.001397155 | 15485 | 1 |
| THYMU2018384 | 0.001397155 | 113825 | 1 |
| THYMU2018455 | 0.001397155 | 47567 | 1 |
| THYMU2018547 | 0.001397155 | 172009 | 1 |
| THYMU2018565 | 0.001397155 | 108939 | 1 |
| THYMU2018639 | 0.004312012 | 134868 | 2 |
| THYMU2018673 | 0.001397155 | 148629 | 1 |
| THYMU2018721 | 0.001397155 | 172044 | 1 |
| THYMU2018772 | 0.001397155 | 47847 | 1 |
| THYMU2018819 | 0.001397155 | 47624 | 1 |
| THYMU2018841 | 0.001397155 | 170636 | 1 |
| THYMU2018997 | 0.001397155 | 152079 | 1 |
| THYMU2019021 | 0.03447783 | 103041 | 11 |
| THYMU2019054 | 0.001397155 | 150277 | 1 |
| THYMU2019197 | 0.001397155 | 150862 | 1 |
| THYMU2019210 | 0.001397155 | 73263 | 1 |
| THYMU2019364 | 0.003983555 | 74654 | 3 |
| THYMU2019436 | 0.001397155 | 117914 | 1 |
| THYMU2019442 | 0.001397155 | 122289 | 1 |
| THYMU2019587 | 0.001397155 | 281612 | 1 |
| THYMU2019599 | 0.001397155 | 229715 | 1 |
| THYMU2019686 | 0.002794311 | 158378 | 2 |
| THYMU2019813 | 0.001397155 | 214718 | 1 |
| THYMU2020198 | 0.001397155 | 145741 | 1 |
| THYMU2020289 | 0.001397155 | 108295 | 1 |
| THYMU2020416 | 0.012224353 | 29776 | 2 |
| THYMU2020491 | 0.001397155 | 67968 | 1 |
| THYMU2020499 | 0.001397155 | 251158 | 1 |
| THYMU2020560 | 0.001397155 | 130385 | 1 |
| THYMU2020667 | 0.004792712 | 86246 | 3 |
| THYMU2020830 | 0.001397155 | 269268 | 1 |
| THYMU2020883 | 0.00325527 | 181191 | 2 |
| THYMU2020959 | 0.015114984 | 88942 | 8 |
| THYMU2021361 | 0.001397155 | 34692 | 1 |
| THYMU2021509 | 0.001397155 | 238302 | 1 |
| THYMU2021597 | 0.001397155 | 57027 | 1 |
| THYMU2021684 | 0.003395557 | 127638 | 2 |
| THYMU2021714 | 0.023751642 | 103392 | 17 |
| THYMU2022213 | 0.025148797 | 114440 | 18 |
| THYMU2022289 | 0.004191466 | 73994 | 3 |
| THYMU2022660 | 0.007309417 | 172365 | 2 |
| THYMU2022854 | 0.001397155 | 278606 | 1 |
| THYMU2022922 | 0.001397155 | 116006 | 1 |
| THYMU2023209 | 0.001397155 | 116973 | 1 |
| THYMU2023264 | 0.003085432 | 41729 | 2 |
| THYMU2023576 | 0.010511424 | 85838 | 4 |
| THYMU2023711 | 0.001397155 | 274871 | 1 |
| THYMU2023900 | 0.001397155 | 94568 | 1 |
| THYMU2023943 | 0.003072702 | 5637 | 2 |
| THYMU2023967 | 0.005485104 | 101070 | 4 |
| THYMU2024071 | 0.005083833 | 100557 | 3 |
| THYMU2024121 | 0.005709168 | 41693 | 3 |
| THYMU2024185 | 0.001397155 | 164169 | 1 |
| THYMU2024207 | 0.001397155 | 238321 | 1 |
| THYMU2024616 | 0.001397155 | 96296 | 1 |
| THYMU2024684 | 0.001397155 | 192803 | 1 |
| THYMU2024748 | 0.004312012 | 108109 | 2 |
| THYMU2025042 | 0.001397155 | 266025 | 1 |
| THYMU2025189 | 0.001397155 | 282943 | 1 |
| THYMU2025319 | 0.005071104 | 100575 | 3 |
| THYMU2025325 | 0.012865291 | 144720 | 4 |
| THYMU2025406 | 0.002794311 | 243509 | 2 |
| THYMU2025557 | 0.002794311 | 221682 | 2 |
| THYMU2025572 | 0.001397155 | 199890 | 1 |
| THYMU2025707 | 0.018109526 | 131155 | 5 |
| THYMU2025849 | 0.001397155 | 221783 | 1 |
| THYMU2025909 | 0.001397155 | 197564 | 1 |
| THYMU2025939 | 0.010506348 | 82423 | 6 |
| THYMU2026182 | 0.001397155 | 232555 | 1 |
| THYMU2026276 | 0.034799554 | 150747 | 6 |
| THYMU2026350 | 0.003478667 | 8878 | 2 |
| THYMU2026530 | 0.001397155 | 141411 | 1 |
| THYMU2026531 | 0.001397155 | 266042 | 1 |
| THYMU2026835 | 0.003106616 | 60477 | 2 |
| THYMU2026904 | 0.001397155 | 41947 | 1 |
| THYMU2027053 | 0.001397155 | 200314 | 1 |
| THYMU2027125 | 0.936089559 | 75254 | 93 |
| THYMU2027168 | 0.001397155 | 63852 | 1 |
| THYMU2027249 | 0.001397155 | 263118 | 1 |
| THYMU2027282 | 0.001397155 | 285128 | 1 |
| THYMU2027497 | 0.001397155 | 227955 | 1 |
| THYMU2027695 | 0.001397155 | 277390 | 1 |
| THYMU2027734 | 0.001397155 | 177440 | 1 |
| THYMU2027894 | 0.001397155 | 92500 | 1 |
| THYMU2027975 | 0.04192001 | 34801 | 4 |
| THYMU2027996 | 0.001397155 | 67047 | 1 |
| THYMU2028041 | 0.001397155 | 204986 | 1 |
| THYMU2028368 | 0.001397155 | 261422 | 1 |
| THYMU2028379 | 0.001397155 | 262180 | 1 |
| THYMU2028394 | 0.002794311 | 160498 | 2 |
| THYMU2028412 | 0.001397155 | 247973 | 1 |
| THYMU2028629 | 0.009152086 | 94446 | 4 |
| THYMU2028632 | 0.002794311 | 208151 | 2 |
| THYMU2028724 | 0.004191466 | 183160 | 3 |
| THYMU2028739 | 0.002492434 | 194795 | 2 |
| THYMU2028942 | 0.001397155 | 196605 | 1 |
| THYMU2028978 | 0.001397155 | 205453 | 1 |
| THYMU2029134 | 0.001397155 | 220850 | 1 |
| THYMU2029578 | 0.024368135 | 8483 | 8 |
| THYMU2029676 | 0.001397155 | 184010 | 1 |
| THYMU2029688 | 0.001397155 | 208058 | 1 |
| THYMU2029788 | 0.001397155 | 233730 | 1 |
| THYMU2030068 | 0.001397155 | 229555 | 1 |
| THYMU2030226 | 0.001397155 | 244940 | 1 |
| THYMU2030264 | 0.0025864 | 121308 | 2 |
| THYMU2030462 | 0.001397155 | 233721 | 1 |
| THYMU2030543 | 0.002794311 | 216046 | 2 |
| THYMU2030637 | 0.001397155 | 138915 | 1 |
| THYMU2030655 | 0.007599475 | 184733 | 2 |
| THYMU2030796 | 0.005588622 | 95006 | 4 |
| THYMU2030912 | 0.001397155 | 272830 | 1 |
| THYMU2030982 | 0.001397155 | 244313 | 1 |
| THYMU2031046 | 0.001397155 | 197635 | 1 |
| THYMU2031139 | 0.004191466 | 177398 | 3 |
| THYMU2031218 | 0.002794311 | 17405 | 2 |
| THYMU2031249 | 0.001397155 | 4838 | 1 |
| THYMU2031258 | 0.001397155 | 114579 | 1 |
| THYMU2031341 | 0.048561028 | 17717 | 7 |
| THYMU2031368 | 0.036921134 | 140956 | 2 |
| THYMU2031579 | 0.001397155 | 259836 | 1 |
| THYMU2031847 | 0.004517962 | 123862 | 2 |
| THYMU2031890 | 0.009437123 | 212786 | 3 |
| THYMU2031994 | 0.001397155 | 229447 | 1 |
| THYMU2032014 | 0.010153475 | 68210 | 3 |
| THYMU2032035 | 0.001397155 | 251947 | 1 |
| THYMU2032080 | 0.001397155 | 167803 | 1 |
| THYMU2032150 | 0.001397155 | 192260 | 1 |
| THYMU2032155 | 0.023463307 | 177671 | 3 |
| THYMU2032358 | 0.001397155 | 270510 | 1 |
| THYMU2032437 | 0.001397155 | 252888 | 1 |
| THYMU2032601 | 0.001397155 | 250248 | 1 |
| THYMU2032655 | 0.001397155 | 100967 | 1 |
| THYMU2032696 | 0.159436907 | 73641 | 5 |
| THYMU2032715 | 0.002794311 | 167794 | 2 |
| THYMU2032732 | 0.001397155 | 131410 | 1 |
| THYMU2032825 | 0.018022094 | 93062 | 5 |
| THYMU2032976 | 0.001397155 | 92105 | 1 |
| THYMU2033053 | 0.002794311 | 12336 | 2 |
| THYMU2033059 | 0.007542681 | 183282 | 2 |
| THYMU2033070 | 0.003395557 | 184137 | 2 |
| THYMU2033079 | 0.001397155 | 36227 | 1 |
| THYMU2033085 | 0.001397155 | 184138 | 1 |
| THYMU2033104 | 0.001397155 | 73557 | 1 |
| THYMU2033227 | 0.001397155 | 262649 | 1 |
| THYMU2033263 | 0.015061645 | 226759 | 3 |
| THYMU2033308 | 0.011998687 | 161261 | 4 |
| THYMU2033401 | 0.001397155 | 184126 | 1 |
| THYMU2033583 | 0.003273997 | 254440 | 2 |
| THYMU2033755 | 0.001397155 | 222864 | 1 |
| THYMU2033787 | 0.00325527 | 227276 | 2 |
| THYMU2033816 | 0.001397155 | 172896 | 1 |
| THYMU2034182 | 0.001397155 | 274695 | 1 |
| THYMU2034279 | 0.001397155 | 255055 | 1 |
| THYMU2034314 | 0.001397155 | 220972 | 1 |
| THYMU2034338 | 0.001397155 | 238794 | 1 |
| THYMU2034374 | 0.01332012 | 126712 | 4 |
| THYMU2034647 | 0.004312012 | 172045 | 2 |
| THYMU2034781 | 0.010453737 | 104768 | 3 |
| THYMU2034917 | 0.008623084 | 125330 | 4 |
| THYMU2035064 | 0.001397155 | 199880 | 1 |
| THYMU2035078 | 0.001397155 | 172357 | 1 |
| THYMU2035101 | 0.001397155 | 193574 | 1 |
| THYMU2035319 | 0.001397155 | 178820 | 1 |
| THYMU2035321 | 0.001397155 | 188537 | 1 |
| THYMU2035388 | 0.001397155 | 208469 | 1 |
| THYMU2035400 | 0.001397155 | 184158 | 1 |
| THYMU2035554 | 0.001397155 | 238425 | 1 |
| THYMU2035710 | 0.027943108 | 71638 | 20 |
| THYMU2035735 | 0.127009852 | 96991 | 25 |
| THYMU2036058 | 0.002492434 | 185426 | 2 |
| THYMU2036085 | 0.037910844 | 98201 | 26 |
| THYMU2036105 | 0.001397155 | 193525 | 1 |
| THYMU2036207 | 0.001397155 | 199969 | 1 |
| THYMU2036215 | 0.007283551 | 179703 | 3 |
| THYMU2036252 | 0.001397155 | 229624 | 1 |
| THYMU2036265 | 0.001397155 | 188589 | 1 |
| THYMU2036366 | 0.001397155 | 254304 | 1 |
| THYMU2036459 | 0.001397155 | 121224 | 1 |
| THYMU2036461 | 0.011047141 | 11120 | 4 |
| THYMU2036604 | 0.001397155 | 199956 | 1 |
| THYMU2036653 | 0.001397155 | 153063 | 1 |
| THYMU2037081 | 0.001397155 | 209632 | 1 |
| THYMU2037208 | 0.001397155 | 20695 | 1 |
| THYMU2037226 | 0.009778349 | 66216 | 6 |
| THYMU2037233 | 0.001397155 | 239841 | 1 |
| THYMU2037234 | 0.002794311 | 220922 | 2 |
| THYMU2037348 | 0.001397155 | 241529 | 1 |
| THYMU2037406 | 0.001397155 | 267882 | 1 |
| THYMU2037965 | 0.001397155 | 214770 | 1 |
| THYMU2038058 | 0.001397155 | 231485 | 1 |
| THYMU2038189 | 0.001397155 | 21732 | 1 |
| THYMU2038199 | 0.001397155 | 204689 | 1 |
| THYMU2038220 | 0.008294641 | 137162 | 5 |
| THYMU2038301 | 0.001397155 | 182878 | 1 |
| THYMU2038369 | 0.001397155 | 172758 | 1 |
| THYMU2038389 | 0.001397155 | 184123 | 1 |
| THYMU2038615 | 0.001397155 | 193215 | 1 |
| THYMU2038636 | 0.001397155 | 272458 | 1 |
| THYMU2038739 | 0.001397155 | 270730 | 1 |
| THYMU2038772 | 0.001397155 | 188450 | 1 |
| THYMU2038797 | 0.001397155 | 266841 | 1 |
| THYMU2038906 | 0.001397155 | 198130 | 1 |
| THYMU2039305 | 0.002794311 | 39776 | 2 |
| THYMU2039315 | 0.003072702 | 141642 | 2 |
| THYMU2039334 | 0.147595986 | 233500 | 2 |
| THYMU2039350 | 0.004984867 | 165436 | 4 |
| THYMU2039411 | 0.001397155 | 225544 | 1 |
| THYMU2039634 | 0.003072702 | 138814 | 2 |
| THYMU2039719 | 0.001397155 | 242232 | 1 |
| THYMU2039780 | 0.0025864 | 254828 | 2 |
| THYMU2039788 | 0.001397155 | 124118 | 1 |
| THYMU2039989 | 0.001397155 | 58760 | 1 |
| THYMU2040029 | 0.004191466 | 154293 | 3 |
| THYMU2040110 | 0.001397155 | 256423 | 1 |
| THYMU2040114 | 0.005241239 | 59645 | 2 |
| THYMU2040140 | 0.001397155 | 172727 | 1 |
| THYMU2040412 | 0.001397155 | 164380 | 1 |
| THYMU2040427 | 0.0025864 | 157139 | 2 |
| THYMU2040824 | 0.001397155 | 192843 | 1 |
| THYMU2040925 | 0.0025864 | 34061 | 2 |
| THYMU2040975 | 0.095383222 | 127390 | 21 |
| THYMU2041007 | 0.001397155 | 253683 | 1 |
| THYMU2041015 | 0.001397155 | 274152 | 1 |
| THYMU2041252 | 0.001397155 | 240389 | 1 |
| THYMU3000028 | 0.004191466 | 89740 | 3 |
| THYMU3000036 | 0.001397155 | 281537 | 1 |
| THYMU3000037 | 0.002794311 | 279909 | 2 |
| THYMU3000082 | 0.002794311 | 255343 | 2 |
| THYMU3000133 | 0.034845616 | 114406 | 15 |
| THYMU3000194 | 0.001397155 | 283357 | 1 |
| THYMU3000224 | 0.002794311 | 136766 | 2 |
| THYMU3000269 | 0.001397155 | 282068 | 1 |
| THYMU3000306 | 0.001397155 | 242994 | 1 |
| THYMU3000360 | 0.004261947 | 128157 | 3 |
| THYMU3000390 | 0.001397155 | 272919 | 1 |
| THYMU3000420 | 0.004261947 | 76550 | 3 |
| THYMU3000490 | 0.001397155 | 249970 | 1 |
| THYMU3000504 | 0.001397155 | 209467 | 1 |
| THYMU3000542 | 0.001397155 | 231345 | 1 |
| THYMU3000655 | 0.003273997 | 110631 | 2 |
| THYMU3000776 | 0.010737936 | 110399 | 8 |
| THYMU3000826 | 0.001397155 | 245778 | 1 |
| THYMU3000841 | 0.001397155 | 242939 | 1 |
| THYMU3001077 | 0.00557409 | 216640 | 2 |
| THYMU3001082 | 0.001397155 | 238367 | 1 |
| THYMU3001083 | 0.0025864 | 168993 | 2 |
| THYMU3001133 | 0.001397155 | 251623 | 1 |
| THYMU3001151 | 0.001397155 | 240225 | 1 |
| THYMU3001234 | 0.015114984 | 88942 | 8 |
| THYMU3001379 | 0.006653007 | 52793 | 4 |
| THYMU3001428 | 0.470539281 | 86556 | 72 |
| THYMU3001472 | 0.009943551 | 129060 | 6 |
| THYMU3001593 | 0.001397155 | 253783 | 1 |
| THYMU3001776 | 0.052878615 | 53485 | 12 |
| THYMU3001883 | 0.001397155 | 64251 | 1 |
| THYMU3001939 | 0.003478667 | 202620 | 2 |
| THYMU3001991 | 0.001397155 | 127266 | 1 |
| THYMU3002452 | 0.001397155 | 258017 | 1 |
| THYMU3002578 | 0.005477069 | 133316 | 3 |
| THYMU3002661 | 0.001397155 | 122970 | 1 |
| THYMU3002887 | 0.214802958 | 50355 | 66 |
| THYMU3003007 | 0.008815783 | 153762 | 5 |
| THYMU3003092 | 0.002794311 | 94638 | 2 |
| THYMU3003212 | 0.001397155 | 238933 | 1 |
| THYMU3003309 | 0.00576319 | 160112 | 4 |
| THYMU3003350 | 0.003072702 | 121425 | 2 |
| THYMU3003403 | 0.002794311 | 164217 | 2 |
| THYMU3003565 | 0.005709168 | 78023 | 3 |
| THYMU3003763 | 0.001397155 | 193299 | 1 |
| THYMU3003865 | 0.007968825 | 111888 | 7 |
| THYMU3003958 | 0.001397155 | 197699 | 1 |
| THYMU3003965 | 0.003106616 | 197687 | 2 |
| THYMU3004157 | 0.001397155 | 56980 | 1 |
| THYMU3004628 | 0.005588622 | 142583 | 4 |
| THYMU3004632 | 0.001397155 | 251461 | 1 |
| THYMU3004835 | 0.008529848 | 99747 | 5 |
| THYMU3004866 | 0.001397155 | 254944 | 1 |
| THYMU3005050 | 0.001397155 | 216432 | 1 |
| THYMU3005378 | 0.001397155 | 248901 | 1 |
| THYMU3005407 | 0.001397155 | 284284 | 1 |
| THYMU3005415 | 0.001397155 | 228278 | 1 |
| THYMU3005427 | 0.007099354 | 205639 | 3 |
| THYMU3005482 | 0.001397155 | 281535 | 1 |
| THYMU3005491 | 0.00325527 | 122750 | 2 |
| THYMU3005629 | 0.011089072 | 157105 | 4 |
| THYMU3005696 | 0.001397155 | 248935 | 1 |
| THYMU3006118 | 0.001397155 | 233478 | 1 |
| THYMU3006132 | 0.001397155 | 132151 | 1 |
| THYMU3006168 | 0.001397155 | 231366 | 1 |
| THYMU3006172 | 0.003273997 | 42739 | 2 |
| THYMU3006367 | 0.016874324 | 40087 | 11 |
| THYMU3006371 | 0.001397155 | 278659 | 1 |
| THYMU3006485 | 0.0025864 | 244910 | 2 |
| THYMU3006640 | 0.001397155 | 192871 | 1 |
| THYMU3006767 | 0.003273997 | 209531 | 2 |
| THYMU3006811 | 0.003273997 | 142631 | 2 |
| THYMU3006963 | 0.001397155 | 254993 | 1 |
| THYMU3007137 | 0.004503771 | 64399 | 3 |
| THYMU3007308 | 0.00429586 | 218367 | 3 |
| THYMU3007368 | 0.001397155 | 260613 | 1 |
| THYMU3007423 | 0.017041013 | 50722 | 11 |
| THYMU3007559 | 0.001397155 | 221683 | 1 |
| THYMU3007845 | 0.001397155 | 278117 | 1 |
| THYMU3008060 | 0.001397155 | 245851 | 1 |
| THYMU3008105 | 0.00827356 | 114480 | 5 |
| THYMU3008136 | 0.001397155 | 47542 | 1 |
| THYMU3008171 | 0.016613967 | 92879 | 11 |
| THYMU3008216 | 0.001397155 | 231250 | 1 |
| THYMU3008436 | 0.001397155 | 42825 | 1 |
| THYMU3008672 | 0.001397155 | 258715 | 1 |
| THYMU3008883 | 0.006887889 | 34707 | 3 |
| THYMU3008935 | 0.001397155 | 271592 | 1 |
| THYMU3009006 | 0.00903154 | 230206 | 5 |
| THYMU3009255 | 0.005588622 | 219921 | 4 |
| THYMU3009391 | 0.001397155 | 144778 | 1 |
| THYMU3009643 | 0.006873547 | 116956 | 6 |
| THYMU3009755 | 0.001397155 | 198404 | 1 |
| THYMU3010247 | 0.001397155 | 218601 | 1 |
| THYMU3011012 | 0.001397155 | 90214 | 1 |
| THYMU3011081 | 0.001397155 | 274837 | 1 |
| THYMU3011244 | 0.001397155 | 236663 | 1 |
| THYMU3011360 | 0.002794311 | 196120 | 2 |
| THYMU3011534 | 0.001397155 | 244009 | 1 |
| THYMU3011543 | 0.001397155 | 142963 | 1 |
| THYMU3011556 | 0.001397155 | 270144 | 1 |
| THYMU3011717 | 0.001397155 | 88461 | 1 |
| THYMU3011827 | 0.007213623 | 214964 | 4 |
| THYMU3012402 | 0.001397155 | 53213 | 1 |
| THYMU3012907 | 0.001397155 | 219975 | 1 |
| THYMU3012983 | 0.002492434 | 259506 | 2 |
| THYMU3013114 | 0.001397155 | 260644 | 1 |
| THYMU3013197 | 0.001397155 | 236768 | 1 |
| THYMU3013241 | 0.002794311 | 129264 | 2 |
| THYMU3013386 | 0.223840198 | 39993 | 27 |
| THYMU3013470 | 0.001397155 | 243210 | 1 |
| THYMU3013785 | 0.001397155 | 17361 | 1 |
| THYMU3013897 | 0.001397155 | 263083 | 1 |
| THYMU3014038 | 0.001397155 | 260643 | 1 |
| THYMU3014173 | 0.001397155 | 247110 | 1 |
| THYMU3014372 | 0.001397155 | 71015 | 1 |
| THYMU3014555 | 0.001397155 | 261227 | 1 |
| THYMU3014620 | 0.001397155 | 51142 | 1 |
| THYMU3014701 | 0.002794311 | 219505 | 2 |
| THYMU3015042 | 0.001397155 | 252403 | 1 |
| THYMU3015457 | 0.001397155 | 278317 | 1 |
| THYMU3015571 | 0.015952021 | 123613 | 4 |
| THYMU3015592 | 0.001397155 | 247487 | 1 |
| THYMU3015625 | 0.001397155 | 271811 | 1 |
| THYMU3015647 | 0.002794311 | 144757 | 2 |
| THYMU3015759 | 0.148820031 | 108232 | 27 |
| THYMU3015962 | 0.001397155 | 250559 | 1 |
| THYMU3016022 | 0.001397155 | 260638 | 1 |
| THYMU3016518 | 0.001397155 | 271631 | 1 |
| THYMU3016797 | 0.001397155 | 260637 | 1 |
| THYMU3016822 | 0.001397155 | 14922 | 1 |
| THYMU3016850 | 0.001397155 | 279641 | 1 |
| THYMU3017562 | 0.001397155 | 9018 | 1 |
| THYMU3017688 | 0.001397155 | 277462 | 1 |
| THYMU3017689 | 0.002794311 | 170289 | 2 |
| THYMU3017761 | 0.001397155 | 224947 | 1 |
| THYMU3018151 | 0.001397155 | 223138 | 1 |
| THYMU3018896 | 0.001397155 | 261995 | 1 |
| THYMU3019095 | 0.001397155 | 269072 | 1 |
| THYMU3019476 | 0.003085432 | 92851 | 2 |
| THYMU3019605 | 0.001397155 | 10308 | 1 |
| THYMU3019916 | 0.001397155 | 276273 | 1 |
| THYMU3020221 | 0.001397155 | 260925 | 1 |
| THYMU3020595 | 0.001397155 | 241191 | 1 |
| THYMU3020713 | 0.001397155 | 260917 | 1 |
| THYMU3020856 | 0.001397155 | 285613 | 1 |
| THYMU3020869 | 0.001397155 | 106746 | 1 |
| THYMU3020970 | 0.001397155 | 274340 | 1 |
| THYMU3021404 | 0.001397155 | 259469 | 1 |
| THYMU3021586 | 0.040568248 | 80135 | 22 |
| THYMU3021755 | 0.001397155 | 269248 | 1 |
| THYMU3021900 | 0.001397155 | 270538 | 1 |
| THYMU3022211 | 0.001397155 | 170307 | 1 |
| THYMU3022434 | 0.001397155 | 265498 | 1 |
| THYMU3022528 | 0.001397155 | 132499 | 1 |
| THYMU3022668 | 0.001397155 | 231465 | 1 |
| THYMU3022982 | 0.001397155 | 214108 | 1 |
| THYMU3023107 | 0.002794311 | 169106 | 2 |
| THYMU3023211 | 0.002794311 | 157013 | 2 |
| THYMU3023216 | 0.001397155 | 230218 | 1 |
| THYMU3023394 | 0.001397155 | 138438 | 1 |
| THYMU3023400 | 0.001397155 | 146064 | 1 |
| THYMU3023797 | 0.001397155 | 269007 | 1 |
| THYMU3024164 | 0.001397155 | 213683 | 1 |
| THYMU3024339 | 0.00577627 | 215668 | 3 |
| THYMU3024602 | 0.001397155 | 132919 | 1 |
| THYMU3024879 | 0.001397155 | 224994 | 1 |
| THYMU3025118 | 0.001397155 | 18456 | 1 |
| THYMU3025313 | 0.001397155 | 276374 | 1 |
| THYMU3025642 | 0.004379115 | 70496 | 2 |
| THYMU3025683 | 0.001397155 | 74463 | 1 |
| THYMU3025772 | 0.001397155 | 278490 | 1 |
| THYMU3025865 | 0.001397155 | 285103 | 1 |
| THYMU3026000 | 0.006829762 | 98173 | 4 |
| THYMU3026306 | 0.007240683 | 190039 | 5 |
| THYMU3026350 | 0.01172411 | 166294 | 7 |
| THYMU3026479 | 0.001397155 | 85839 | 1 |
| THYMU3026532 | 0.001397155 | 17882 | 1 |
| THYMU3026783 | 0.001397155 | 135389 | 1 |
| THYMU3026869 | 0.001397155 | 278722 | 1 |
| THYMU3027251 | 0.002794311 | 127981 | 2 |
| THYMU3027540 | 0.001397155 | 136129 | 1 |
| THYMU3027655 | 0.001397155 | 145781 | 1 |
| THYMU3027671. | 0.001397155 | 143757 | 1 |
| THYMU3028075 | 0.001397155 | 187873 | 1 |
| THYMU3028410 | 0.003395557 | 49146 | 2 |
| THYMU3028461 | 0.001397155 | 163347 | 1 |
| THYMU3028702 | 0.001397155 | 106155 | 1 |
| THYMU3029188 | 0.001397155 | 149629 | 1 |
| THYMU3029318 | 0.001397155 | 160083 | 1 |
| THYMU3029421 | 0.001397155 | 129683 | 1 |
| THYMU3029719 | 0.001397155 | 122082 | 1 |
| THYMU3029774 | 0.001397155 | 154357 | 1 |
| THYMU3029795 | 0.015748053 | 72286 | 7 |
| THYMU3029832 | 0.001397155 | 270238 | 1 |
| THYMU3030072 | 0.001397155 | 84183 | 1 |
| THYMU3030231 | 0.001397155 | 21751 | 1 |
| THYMU3030706 | 0.001397155 | 144069 | 1 |
| THYMU3030710 | 0.001397155 | 77815 | 1 |
| THYMU3030752 | 0.001397155 | 56810 | 1 |
| THYMU3031146 | 0.001397155 | 177001 | 1 |
| THYMU3031402 | 0.001397155 | 177004 | 1 |
| THYMU3031610 | 0.001397155 | 177049 | 1 |
| THYMU3031612 | 0.001397155 | 183171 | 1 |
| THYMU3031868 | 0.002794311 | 158871 | 2 |
| THYMU3031878 | 0.001397155 | 284935 | 1 |
| THYMU3032032 | 0.001397155 | 129852 | 1 |
| THYMU3032798 | 0.086652884 | 133051 | 35 |
| THYMU3032867 | 0.013018267 | 120560 | 5 |
| THYMU3033402 | 0.001397155 | 123558 | 1 |
| THYMU3033626 | 0.029810598 | 116011 | 10 |
| THYMU3033630 | 0.001397155 | 195868 | 1 |
| THYMU3033649 | 0.001397155 | 131517 | 1 |
| THYMU3033754 | 0.001397155 | 131916 | 1 |
| THYMU3033759 | 0.001397155 | 70779 | 1 |
| THYMU3034078 | 0.001397155 | 131197 | 1 |
| THYMU3034099 | 0.001397155 | 118889 | 1 |
| THYMU3034453 | 0.001397155 | 136730 | 1 |
| THYMU3034616 | 0.003395557 | 127270 | 2 |
| THYMU3034671 | 0.070255898 | 95008 | 29 |
| THYMU3034853 | 0.002794311 | 89354 | 2 |
| THYMU3034867 | 0.001397155 | 229912 | 1 |
| THYMU3034983 | 0.001397155 | 145517 | 1 |
| THYMU3036200 | 0.001397155 | 85883 | 1 |
| THYMU3036310 | 0.001397155 | 193684 | 1 |
| THYMU3036934 | 0.001397155 | 160193 | 1 |
| THYMU3036953 | 0.001397155 | 74270 | 1 |
| THYMU3037052 | 0.001397155 | 146848 | 1 |
| THYMU3037192 | 0.001397155 | 177752 | 1 |
| THYMU3037617 | 0.004191466 | 154344 | 3 |
| THYMU3037772 | 0.001397155 | 266207 | 1 |
| THYMU3037827 | 0.003072702 | 139321 | 2 |
| THYMU3037836 | 0.001397155 | 285085 | 1 |
| THYMU3037856 | 0.001397155 | 255912 | 1 |
| THYMU3037867 | 0.001397155 | 183027 | 1 |
| THYMU3037909 | 0.001397155 | 124163 | 1 |
| THYMU3037980 | 0.001397155 | 148801 | 1 |
| THYMU3038158 | 0.027208165 | 140556 | 3 |
| THYMU3038167 | 0.001397155 | 233991 | 1 |
| THYMU3038214 | 0.004261947 | 60776 | 3 |
| THYMU3038266 | 0.001397155 | 130507 | 1 |
| THYMU3038328 | 0.001397155 | 156764 | 1 |
| THYMU3038347 | 0.001397155 | 130763 | 1 |
| THYMU3038375 | 0.001397155 | 127530 | 1 |
| THYMU3038603 | 0.001397155 | 233997 | 1 |
| THYMU3038687 | 0.001397155 | 57684 | 1 |
| THYMU3038759 | 0.001397155 | 267875 | 1 |
| THYMU3038879 | 0.001397155 | 276171 | 1 |
| THYMU3038970 | 0.001397155 | 97478 | 1 |
| THYMU3039772 | 0.021240948 | 100742 | 8 |
| THYMU3039807 | 0.001397155 | 116444 | 1 |
| THYMU3039846 | 0.001397155 | 146174 | 1 |
| THYMU3040068 | 0.004312012 | 112312 | 2 |
| THYMU3040126 | 0.001397155 | 274276 | 1 |
| THYMU3040146 | 0.001397155 | 224857 | 1 |
| THYMU3040168 | 0.001397155 | 252069 | 1 |
| THYMU3040172 | 0.001397155 | 116297 | 1 |
| THYMU3040725 | 0.001397155 | 131903 | 1 |
| THYMU3040746 | 0.001397155 | 118726 | 1 |
| THYMU3040816 | 0.014458806 | 43541 | 2 |
| THYMU3040829 | 0.001397155 | 3719 | 1 |
| THYMU3040830 | 0.001397155 | 136652 | 1 |
| THYMU3040986 | 0.001397155 | 151752 | 1 |
| THYMU3041354 | 0.001397155 | 157035 | 1 |
| THYMU3041386 | 0.001397155 | 184312 | 1 |
| THYMU3041428 | 0.657483149 | 22293 | 8 |
| THYMU3041573 | 0.001397155 | 151887 | 1 |
| THYMU3041603 | 0.001397155 | 183090 | 1 |
| THYMU3041736 | 0.001397155 | 201763 | 1 |
| THYMU3041918 | 0.001397155 | 272277 | 1 |
| THYMU3042075 | 0.001397155 | 183111 | 1 |
| THYMU3042321 | 0.001397155 | 90204 | 1 |
| THYMU3042372 | 0.001397155 | 164318 | 1 |
| THYMU3042758 | 0.001397155 | 183047 | 1 |
| THYMU3042929 | 0.001397155 | 161461 | 1 |
| THYMU3043200 | 0.001397155 | 114621 | 1 |
| THYMU3043327 | 0.001397155 | 157126 | 1 |
| THYMU3043482 | 0.001397155 | 234573 | 1 |
| THYMU3043597 | 0.001397155 | 257792 | 1 |
| THYMU3043688 | 0.001397155 | 259521 | 1 |
| THYMU3043779 | 0.001397155 | 132990 | 1 |
| THYMU3043883 | 0.001397155 | 127344 | 1 |
| THYMU3043993 | 0.001397155 | 149288 | 1 |
| THYMU3044075 | 0.008836199 | 131462 | 6 |
| THYMU3044175 | 0.002794311 | 14683 | 2 |
| THYMU3044188 | 0.037433191 | 118200 | 2 |
| THYMU3044441 | 0.001397155 | 129615 | 1 |
| THYMU3044445 | 0.001397155 | 189960 | 1 |
| THYMU3045510 | 0.007106323 | 113478 | 4 |
| THYMU3045673 | 0.001397155 | 162132 | 1 |
| THYMU3045692 | 0.001397155 | 224840 | 1 |
| THYMU3045704 | 0.001397155 | 172359 | 1 |
| THYMU3046140 | 0.001397155 | 149532 | 1 |
| THYMU3046350 | 0.024256846 | 82081 | 6 |
| THYMU3046360 | 0.001397155 | 29998 | 1 |
| THYMU3046856 | 0.001397155 | 178900 | 1 |
| THYMU3047115 | 0.407756673 | 75470 | 37 |
| THYMU3047144 | 0.001397155 | 215565 | 1 |
| THYMU3047156 | 0.001397155 | 227560 | 1 |
| THYMU3047366 | 0.001397155 | 167763 | 1 |
| THYMU3047513 | 0.001397155 | 281819 | 1 |
| THYMU3047542 | 0.001397155 | 110020 | 1 |
| THYMU3047760 | 0.001397155 | 177694 | 1 |
| THYMU3047891 | 0.001397155 | 51202 | 1 |
| TKIDN1000001 | 0.015054857 | 60810 | 4 |
| TKIDN1000044 | 0.017892972 | 94496 | 5 |
| TKIDN1000062 | 0.00620232 | 47631 | 1 |
| TKIDN1000066 | 0.017489001 | 156289 | 2 |
| TKIDN1000164 | 0.027099988 | 113290 | 7 |
| TKIDN1000171 | 0.00620232 | 131961 | 1 |
| TKIDN1000192 | 0.030329007 | 18697 | 10 |
| TKIDN2000127 | 0.009247201 | 140638 | 2 |
| TKIDN2000240 | 0.00620232 | 121619 | 1 |
| TKIDN2000246 | 0.550803718 | 46185 | 133 |
| TKIDN2000319 | 0.00791178 | 146224 | 2 |
| TKIDN2000464 | 0.009117177 | 47879 | 2 |
| TKIDN2000521 | 0.011262874 | 75584 | 4 |
| TKIDN2000701 | 0.007297598 | 206043 | 2 |
| TKIDN2001529 | 0.008283832 | 198111 | 2 |
| TKIDN2001662 | 0.015363597 | 111377 | 6 |
| TKIDN2002318 | 0.03124095 | 122949 | 8 |
| TKIDN2002329 | 0.00620232 | 201319 | 1 |
| TKIDN2002424 | 0.00620232 | 198989 | 1 |
| TKIDN2002632 | 0.00620232 | 37890 | 1 |
| TKIDN2002738 | 0.399538298 | 153488 | 16 |
| TKIDN2003044 | 0.00620232 | 188868 | 1 |
| TKIDN2003059 | 0.00791178 | 202816 | 2 |
| TKIDN2003062 | 0.00620232 | 97689 | 1 |
| TKIDN2003078 | 0.012098717 | 222149 | 3 |
| TKIDN2003366 | 0.00620232 | 261303 | 1 |
| TKIDN2003396 | 0.00620232 | 152961 | 1 |
| TKIDN2003413 | 0.00620232 | 161507 | 1 |
| TKIDN2003597 | 0.021447808 | 58660 | 9 |
| TKIDN2003601 | 0.00878872 | 177417 | 3 |
| TKIDN2004185 | 0.042238356 | 180410 | 2 |
| TKIDN2004386 | 0.00620232 | 128375 | 1 |
| TKIDN2004458 | 0.012404639 | 114769 | 2 |
| TKIDN2004748 | 0.017327028 | 113838 | 2 |
| TKIDN2005487 | 0.233233081 | 132983 | 11 |
| TKIDN2005721 | 0.00620232 | 283957 | 1 |
| TKIDN2005934 | 0.00620232 | 147110 | 1 |
| TKIDN2005947 | 0.00620232 | 193356 | 1 |
| TKIDN2005956 | 0.00620232 | 110641 | 1 |
| TKIDN2006525 | 0.00620232 | 106071 | 1 |
| TKIDN2006761 | 0.007890596 | 233824 | 2 |
| TKIDN2006852 | 0.012404639 | 131268 | 2 |
| TKIDN2007605 | 0.013413037 | 74987 | 5 |
| TKIDN2007667 | 0.00620232 | 178842 | 1 |
| TKIDN2007828 | 0.025113053 | 157976 | 4 |
| TKIDN2008176 | 0.007890596 | 178917 | 2 |
| TKIDN2008778 | 0.00620232 | 271669 | 1 |
| TKIDN2009092 | 0.00620232 | 1703 | 1 |
| TKIDN2009330 | 0.008060434 | 147782 | 2 |
| TKIDN2009481 | 0.00620232 | 129039 | 1 |
| TKIDN2009641 | 0.028980479 | 143560 | 10 |
| TKIDN2009794 | 0.011612823 | 96189 | 4 |
| TKIDN2009889 | 0.009553414 | 142783 | 3 |
| TKIDN2010200 | 0.00620232 | 188293 | 1 |
| TKIDN2010232 | 0.00620232 | 199366 | 1 |
| TKIDN2010278 | 0.00620232 | 177480 | 1 |
| TKIDN2010602 | 0.00620232 | 194701 | 1 |
| TKIDN2010812 | 0.015716289 | 114231 | 6 |
| TKIDN2010934 | 0.008283832 | 160299 | 2 |
| TKIDN2011051 | 0.00620232 | 183324 | 1 |
| TKIDN2011160 | 0.00620232 | 245598 | 1 |
| TKIDN2011289 | 0.028028429 | 114038 | 7 |
| TKIDN2011324 | 0.00620232 | 215425 | 1 |
| TKIDN2012034 | 0.00620232 | 232588 | 1 |
| TKIDN2012771 | 0.00620232 | 48516 | 1 |
| TKIDN2012824 | 0.00620232 | 192579 | 1 |
| TKIDN2013134 | 0.00620232 | 259362 | 1 |
| TKIDN2013287 | 0.009680987 | 150912 | 3 |
| TKIDN2014570 | 0.007877867 | 234722 | 2 |
| TKIDN2014757 | 0.00620232 | 211344 | 1 |
| TKIDN2014771 | 0.00620232 | 222655 | 1 |
| TKIDN2014964 | 0.015288767 | 153329 | 3 |
| TKIDN2015025 | 0.00620232 | 188929 | 1 |
| TKIDN2015071 | 0.012917237 | 59938 | 5 |
| TKIDN2015161 | 0.00620232 | 207382 | 1 |
| TKIDN2015263 | 0.00620232 | 268949 | 1 |
| TKIDN2015285 | 0.016877602 | 198337 | 4 |
| TKIDN2015423 | 0.263199246 | 78702 | 135 |
| TKIDN2015788 | 0.063699701 | 124561 | 12 |
| TKIDN2016309 | 0.00620232 | 211313 | 1 |
| TKIDN2016399 | 0.01772173 | 188903 | 2 |
| TKIDN2016846 | 0.007391564 | 170791 | 2 |
| TKIDN2018926 | 0.007890596 | 199031 | 2 |
| TKIDN2019116 | 0.00620232 | 147919 | 1 |
| TLIVE2000023 | 0.01151941 | 142264 | 1 |
| TLIVE2000142 | 0.01151941 | 184600 | 1 |
| TLIVE2000292 | 0.01151941 | 43746 | 1 |
| TLIVE2000762 | 0.01151941 | 270153 | 1 |
| TLIVE2000979 | 0.015696345 | 112781 | 2 |
| TLIVE2001327 | 0.035724133 | 172673 | 4 |
| TLIVE2001616 | 0.01151941 | 210981 | 1 |
| TLIVE2001684 | 0.01151941 | 143407 | 1 |
| TLIVE2001828 | 0.012916566 | 217660 | 2 |
| TLIVE2001927 | 0.01151941 | 205909 | 1 |
| TLIVE2002046 | 0.092318972 | 41471 | 36 |
| TLIVE2002336 | 0.046077641 | 195878 | 4 |
| TLIVE2002338 | 0.01151941 | 170643 | 1 |
| TLIVE2002562 | 0.01151941 | 156517 | 1 |
| TLIVE2002690 | 0.08341079 | 159249 | 7 |
| TLIVE2002882 | 0.01151941 | 73608 | 1 |
| TLIVE2003197 | 0.01151941 | 221117 | 1 |
| TLIVE2003200 | 0.01322887 | 263530 | 2 |
| TLIVE2003225 | 0.01151941 | 232147 | 1 |
| TLIVE2003381 | 0.01151941 | 197238 | 1 |
| TLIVE2003737 | 0.01151941 | 190352 | 1 |
| TLIVE2003961 | 0.01151941 | 195044 | 1 |
| TLIVE2003970 | 0.072446202 | 127223 | 6 |
| TLIVE2004110 | 0.01622858 | 186237 | 3 |
| TLIVE2004320 | 0.013207687 | 186046 | 2 |
| TLIVE2004331 | 0.01151941 | 203974 | 1 |
| TLIVE2004601 | 0.01151941 | 137573 | 1 |
| TLIVE2005180 | 0.01151941 | 171074 | 1 |
| TLIVE2005390 | 0.01151941 | 162912 | 1 |
| TLIVE2005516 | 0.01151941 | 158841 | 1 |
| TLIVE2005731 | 0.01151941 | 149946 | 1 |
| TLIVE2005866 | 0.01151941 | 160852 | 1 |
| TLIVE2006236 | 0.01151941 | 151698 | 1 |
| TLIVE2006359 | 0.02303882 | 154405 | 2 |
| TLIVE2006529 | 0.01151941 | 182399 | 1 |
| TLIVE2006621 | 0.01151941 | 168164 | 1 |
| TLIVE2006733 | 0.01151941 | 185216 | 1 |
| TLIVE2007000 | 0.01151941 | 272444 | 1 |
| TLIVE2007016 | 0.01151941 | 14167 | 1 |
| TLIVE2007132 | 0.01151941 | 232895 | 1 |
| TLIVE2007192 | 0.01151941 | 215662 | 1 |
| TLIVE2007528 | 0.01151941 | 195857 | 1 |
| TLIVE2007607 | 0.02303882 | 100853 | 2 |
| TLIVE2007736 | 0.012708655 | 34334 | 2 |
| TLIVE2007816 | 0.01151941 | 156385 | 1 |
| TLIVE2007919 | 0.01151941 | 202926 | 1 |
| TLIVE2008083 | 0.01151941 | 264911 | 1 |
| TLIVE2008213 | 0.01151941 | 156360 | 1 |
| TLIVE2008221 | 0.02303882 | 147329 | 2 |
| TLIVE2008229 | 0.012614688 | 14540 | 2 |
| TLIVE2008712 | 0.01151941 | 144065 | 1 |
| TLIVE2008797 | 0.01151941 | 156389 | 1 |
| TLIVE2009087 | 0.01151941 | 170904 | 1 |
| TLIVE2009163 | 0.01151941 | 192482 | 1 |
| TLIVE2009489 | 0.01151941 | 141854 | 1 |
| TLIVE2009541 | 0.01151941 | 109193 | 1 |
| TLUNG2000179 | 0.035863308 | 111619 | 2 |
| TOVAR2000476 | 0.07383765 | 40429 | 2 |
| TOVAR2000575 | 0.037893506 | 17063 | 2 |
| TOVAR2000649 | 0.03649635 | 261104 | 1 |
| TOVAR2000904 | 0.038205811 | 166767 | 2 |
| TOVAR2001281 | 0.03649635 | 167847 | 1 |
| TOVAR2001556 | 0.03649635 | 170966 | 1 |
| TOVAR2001730 | 0.03649635 | 36671 | 1 |
| TOVAR2002247 | 0.03649635 | 68473 | 1 |
| TOVAR2002514 | 0.03649635 | 280288 | 1 |
| TOVAR2002549 | 0.03649635 | 160813 | 1 |
| TOVAR2002800 | 0.03649635 | 283966 | 1 |
| TRACH1000011 | 0.02276324 | 75422 | 7 |
| TRACH1000018 | 0.0081132 | 56050 | 2 |
| TRACH1000030 | 0.242877361 | 81581 | 82 |
| TRACH1000038 | 0.044487921 | 70270 | 15 |
| TRACH1000057 | 0.001876842 | 65038 | 1 |
| TRACH1000063 | 0.077907129 | 96395 | 11 |
| TRACH1000074 | 0.001876842 | 71895 | 1 |
| TRACH1000100 | 0.001876842 | 48143 | 1 |
| TRACH1000106 | 0.028223146 | 68326 | 5 |
| TRACH1000125 | 0.003273997 | 71896 | 2 |
| TRACH1000140 | 0.001876842 | 115480 | 1 |
| TRACH1000144 | 0.001876842 | 52961 | 1 |
| TRACH1000181 | 0.035367649 | 8421 | 10 |
| TRACH1000193 | 0.007713753 | 183820 | 5 |
| TRACH1000205 | 0.042576617 | 63085 | 18 |
| TRACH1000212 | 0.001876842 | 259947 | 1 |
| TRACH1000240 | 0.001876842 | 235796 | 1 |
| TRACH2000002 | 0.001876842 | 271670 | 1 |
| TRACH2000015 | 0.001876842 | 53856 | 1 |
| TRACH2000024 | 0.001876842 | 268675 | 1 |
| TRACH2000032 | 0.005150839 | 192694 | 3 |
| TRACH2000045 | 0.060933075 | 100123 | 30 |
| TRACH2000047 | 0.031284816 | 113471 | 14 |
| TRACH2000079 | 0.30930943 | 67177 | 52 |
| TRACH2000081 | 0.013163523 | 176354 | 2 |
| TRACH2000083 | 0.001876842 | 269548 | 1 |
| TRACH2000148 | 0.001876842 | 75993 | 1 |
| TRACH2000185 | 0.001876842 | 262542 | 1 |
| TRACH2000196 | 0.01270404 | 50423 | 2 |
| TRACH2000235 | 0.001876842 | 267088 | 1 |
| TRACH2000237 | 0.031164375 | 159027 | 9 |
| TRACH2000243 | 0.542417382 | 41893 | 2 |
| TRACH2000248 | 0.240163584 | 56800 | 50 |
| TRACH2000250 | 0.00911546 | 53151 | 3 |
| TRACH2000259 | 0.001876842 | 130854 | 1 |
| TRACH2000272 | 0.001876842 | 268966 | 1 |
| TRACH2000282 | 0.004791699 | 244101 | 2 |
| TRACH2000287 | 0.001876842 | 194550 | 1 |
| TRACH2000289 | 0.001876842 | 270078 | 1 |
| TRACH2000306 | 0.029624832 | 133844 | 5 |
| TRACH2000312 | 0.001876842 | 269781 | 1 |
| TRACH2000321 | 0.341032061 | 121365 | 68 |
| TRACH2000336 | 0.001876842 | 277535 | 1 |
| TRACH2000359 | 0.056186393 | 100850 | 11 |
| TRACH2000393 | 0.004924201 | 37670 | 3 |
| TRACH2000411 | 0.001876842 | 115636 | 1 |
| TRACH2000420 | 0.04996788 | 86495 | 20 |
| TRACH2000461 | 0.097726404 | 82743 | 49 |
| TRACH2000472 | 0.19622716 | 78653 | 33 |
| TRACH2000496 | 0.01126105 | 140260 | 6 |
| TRACH2000497 | 0.001876842 | 92546 | 1 |
| TRACH2000502 | 0.003552389 | 139492 | 2 |
| TRACH2000522 | 0.001876842 | 30404 | 1 |
| TRACH2000540 | 0.117612755 | 60835 | 15 |
| TRACH2000559 | 0.043024088 | 215095 | 3 |
| TRACH2000660 | 0.013211224 | 82315 | 8 |
| TRACH2000665 | 0.001876842 | 267158 | 1 |
| TRACH2000675 | 0.001876842 | 96417 | 1 |
| TRACH2000677 | 0.005943607 | 187195 | 4 |
| TRACH2000702 | 0.04267859 | 140574 | 9 |
| TRACH2000703 | 0.001876842 | 37984 | 1 |
| TRACH2000708 | 0.09145026 | 126067 | 29 |
| TRACH2000767 | 0.004671152 | 133191 | 3 |
| TRACH2000775 | 0.001876842 | 269017 | 1 |
| TRACH2000780 | 0.191708005 | 121982 | 61 |
| TRACH2000807 | 0.001876842 | 138063 | 1 |
| TRACH2000862 | 0.02894482 | 63177 | 7 |
| TRACH2000873 | 0.003565118 | 185084 | 2 |
| TRACH2000894 | 0.001876842 | 7567 | 1 |
| TRACH2000898 | 0.001876842 | 276954 | 1 |
| TRACH2000920 | 0.050541053 | 42080 | 19 |
| TRACH2000923 | 0.001876842 | 271648 | 1 |
| TRACH2000926 | 0.060054739 | 141469 | 10 |
| TRACH2000944 | 0.001876842 | 271064 | 1 |
| TRACH2000959 | 0.686273943 | 2455 | 22 |
| TRACH2000972 | 0.282727177 | 77265 | 30 |
| TRACH2000981 | 0.001876842 | 23469 | 1 |
| TRACH2000986 | 0.001876842 | 270545 | 1 |
| TRACH2001021 | 0.400160761 | 99504 | 39 |
| TRACH2001037 | 0.001876842 | 267477 | 1 |
| TRACH2001060 | 0.004997648 | 146531 | 2 |
| TRACH2001085 | 0.0081132 | 56506 | 2 |
| TRACH2001101 | 0.063036543 | 136261 | 17 |
| TRACH2001109 | 0.80060832 | 71463 | 390 |
| TRACH2001121 | 0.001876842 | 269250 | 1 |
| TRACH2001147 | 0.001876842 | 19995 | 1 |
| TRACH2001154 | 0.003753683 | 714 | 2 |
| TRACH2001188 | 0.001876842 | 268082 | 1 |
| TRACH2001192 | 0.004970521 | 83438 | 3 |
| TRACH2001249 | 0.018271584 | 165083 | 14 |
| TRACH2001275 | 0.001876842 | 270534 | 1 |
| TRACH2001289 | 0.49297219 | 80776 | 84 |
| TRACH2001314 | 0.091670121 | 115103 | 46 |
| TRACH2001335 | 0.001876842 | 271112 | 1 |
| TRACH2001339 | 0.005720926 | 79690 | 2 |
| TRACH2001341 | 0.026288736 | 48920 | 6 |
| TRACH2001347 | 0.001876842 | 45753 | 1 |
| TRACH2001395 | 0.001876842 | 271413 | 1 |
| TRACH2001400 | 0.001876842 | 271985 | 1 |
| TRACH2001403 | 0.001876842 | 23678 | 1 |
| TRACH2001416 | 0.001876842 | 269232 | 1 |
| TRACH2001428 | 0.132760152 | 14067 | 10 |
| TRACH2001432 | 0.150625572 | 139803 | 23 |
| TRACH2001443 | 0.018031859 | 147226 | 6 |
| TRACH2001463 | 0.054724518 | 125516 | 17 |
| TRACH2001474 | 0.001876842 | 109890 | 1 |
| TRACH2001478 | 0.288639963 | 106280 | 39 |
| TRACH2001523 | 0.098556086 | 155471 | 10 |
| TRACH2001525 | 0.001876842 | 270227 | 1 |
| TRACH2001532 | 0.001876842 | 156962 | 1 |
| TRACH2001549 | 0.039627059 | 111162 | 17 |
| TRACH2001592 | 0.001876842 | 135039 | 1 |
| TRACH2001596 | 0.008187255 | 8303 | 4 |
| TRACH2001607 | 0.166827778 | 91495 | 47 |
| TRACH2001612 | 0.041946256 | 28691 | 13 |
| TRACH2001621 | 0.001876842 | 62700 | 1 |
| TRACH2001646 | 0.007409202 | 239919 | 3 |
| TRACH2001651 | 0.01126105 | 100698 | 6 |
| TRACH2001684 | 0.009406581 | 237497 | 3 |
| TRACH2001810 | 0.215764907 | 81504 | 46 |
| TRACH2001898 | 0.164358759 | 148776 | 7 |
| TRACH2001933 | 0.001876842 | 245229 | 1 |
| TRACH2001968 | 0.007507367 | 144291 | 4 |
| TRACH2001996 | 0.005720926 | 248528 | 2 |
| TRACH2002043 | 0.158000009 | 98410 | 3 |
| TRACH2002054 | 0.003875243 | 190593 | 2 |
| TRACH2002092 | 0.059690957 | 84397 | 7 |
| TRACH2002100 | 0.009770005 | 100146 | 4 |
| TRACH2002138 | 0.007789453 | 110238 | 2 |
| TRACH2002333 | 0.011398249 | 179437 | 4 |
| TRACH2002537 | 0.18067368 | 85929 | 39 |
| TRACH2002664 | 0.110102803 | 97543 | 16 |
| TRACH2002784 | 0.003753683 | 253111 | 2 |
| TRACH2002803 | 0.080407312 | 97605 | 30 |
| TRACH2002840 | 0.001876842 | 215099 | 1 |
| TRACH2002847 | 0.036916451 | 228874 | 4 |
| TRACH2002853 | 0.001876842 | 280044 | 1 |
| TRACH2002862 | 0.001876842 | 273260 | 1 |
| TRACH2002954 | 0.001876842 | 267499 | 1 |
| TRACH2002960 | 0.001876842 | 117693 | 1 |
| TRACH2002988 | 0.003273997 | 130109 | 2 |
| TRACH2003070 | 0.003273997 | 89672 | 2 |
| TRACH2003272 | 0.045223526 | 103242 | 18 |
| TRACH2003323 | 0.094146793 | 67151 | 32 |
| TRACH2003347 | 0.105467684 | 91997 | 18 |
| TRACH2003484 | 0.003066086 | 114064 | 2 |
| TRACH2003516 | 0.001876842 | 121916 | 1 |
| TRACH2003605 | 0.003273997 | 111460 | 2 |
| TRACH2003736 | 0.001876842 | 132757 | 1 |
| TRACH2003759 | 0.001876842 | 167889 | 1 |
| TRACH2003941 | 0.001876842 | 55875 | 1 |
| TRACH2004039 | 0.00297212 | 84935 | 2 |
| TRACH2004054 | 0.001876842 | 36904 | 1 |
| TRACH2004087 | 0.001876842 | 158936 | 1 |
| TRACH2004109 | 0.004791699 | 174712 | 2 |
| TRACH2004170 | 0.001876842 | 266070 | 1 |
| TRACH2004183 | 0.001876842 | 37629 | 1 |
| TRACH2004251 | 0.001876842 | 2255 | 1 |
| TRACH2004292 | 0.001876842 | 167887 | 1 |
| TRACH2004336 | 0.00297212 | 120427 | 2 |
| TRACH2004399 | 0.005652486 | 80419 | 4 |
| TRACH2004428 | 0.001876842 | 37902 | 1 |
| TRACH2004458 | 0.00793121 | 90376 | 2 |
| TRACH2004499 | 0.057020452 | 185848 | 4 |
| TRACH2004521 | 0.001876842 | 51880 | 1 |
| TRACH2004570 | 0.001876842 | 156407 | 1 |
| TRACH2004845 | 0.001876842 | 134798 | 1 |
| TRACH2004887 | 0.005630525 | 145885 | 3 |
| TRACH2004950 | 0.001876842 | 175560 | 1 |
| TRACH2005004 | 0.003753683 | 125290 | 2 |
| TRACH2005017 | 0.001876842 | 112274 | 1 |
| TRACH2005066 | 0.001876842 | 273989 | 1 |
| TRACH2005159 | 0.001876842 | 169912 | 1 |
| TRACH2005210 | 0.055244213 | 147531 | 13 |
| TRACH2005314 | 0.001876842 | 108565 | 1 |
| TRACH2005444 | 0.001876842 | 170825 | 1 |
| TRACH2005541 | 0.017231309 | 71063 | 4 |
| TRACH2005666 | 0.001876842 | 190522 | 1 |
| TRACH2005698 | 0.003273997 | 43554 | 2 |
| TRACH2005710 | 0.012479047 | 63434 | 2 |
| TRACH2005720 | 0.001876842 | 73665 | 1 |
| TRACH2005769 | 0.001876842 | 189657 | 1 |
| TRACH2005796 | 0.003734956 | 168801 | 2 |
| TRACH2005800 | 0.139291562 | 132443 | 11 |
| TRACH2005811 | 0.158883397 | 92618 | 36 |
| TRACH2006015 | 0.001876842 | 190218 | 1 |
| TRACH2006049 | 0.003552389 | 96806 | 2 |
| TRACH2006097 | 0.003753683 | 162317 | 2 |
| TRACH2006194 | 0.001876842 | 170842 | 1 |
| TRACH2006327 | 0.001876842 | 137024 | 1 |
| TRACH2006331 | 0.001876842 | 68640 | 1 |
| TRACH2006378 | 0.018746459 | 174949 | 5 |
| TRACH2006387 | 0.001876842 | 146193 | 1 |
| TRACH2006528 | 0.001876842 | 95425 | 1 |
| TRACH2006670 | 0.001876842 | 17645 | 1 |
| TRACH2006762 | 0.007818965 | 84959 | 2 |
| TRACH2006866 | 0.168849307 | 151512 | 16 |
| TRACH2006870 | 0.001876842 | 98450 | 1 |
| TRACH2006918 | 0.001876842 | 273408 | 1 |
| TRACH2007059 | 0.004997648 | 170163 | 2 |
| TRACH2007324 | 0.001876842 | 201035 | 1 |
| TRACH2007358 | 0.01422557 | 4764 | 2 |
| TRACH2007399 | 0.355708299 | 94682 | 31 |
| TRACH2007483 | 0.001876842 | 123987 | 1 |
| TRACH2007542 | 0.001876842 | 207545 | 1 |
| TRACH2007577 | 0.001876842 | 130176 | 1 |
| TRACH2007674 | 0.001876842 | 129182 | 1 |
| TRACH2007676 | 0.001876842 | 130166 | 1 |
| TRACH2007688 | 0.003753683 | 180529 | 2 |
| TRACH2007733 | 0.001876842 | 278304 | 1 |
| TRACH2007754 | 0.11152409 | 87437 | 7 |
| TRACH2007816 | 0.016784271 | 131619 | 7 |
| TRACH2007834 | 0.332198807 | 88220 | 26 |
| TRACH2007894 | 0.003273997 | 101492 | 2 |
| TRACH2007969 | 0.066026047 | 157782 | 10 |
| TRACH2007988 | 0.001876842 | 194589 | 1 |
| TRACH2008081 | 0.13116108 | 21045 | 7 |
| TRACH2008112 | 0.001876842 | 180305 | 1 |
| TRACH2008113 | 0.009001861 | 161608 | 3 |
| TRACH2008127 | 0.001876842 | 174671 | 1 |
| TRACH2008130 | 0.003958354 | 152436 | 2 |
| TRACH2008278 | 0.001876842 | 113823 | 1 |
| TRACH2008300 | 0.098693552 | 123744 | 43 |
| TRACH2008472 | 0.106789876 | 155695 | 29 |
| TRACH2008478 | 0.009382103 | 119335 | 4 |
| TRACH2008540 | 0.021242825 | 163534 | 8 |
| TRACH2008583 | 0.001876842 | 157464 | 1 |
| TRACH2008597 | 0.003273997 | 10298 | 2 |
| TRACH2008716 | 0.033030077 | 158103 | 6 |
| TRACH2008820 | 0.001876842 | 18934 | 1 |
| TRACH2008891 | 0.007723592 | 18155 | 4 |
| TRACH2008921 | 0.003753683 | 170840 | 2 |
| TRACH2009006 | 0.047743908 | 100798 | 13 |
| TRACH2009060 | 0.001876842 | 233306 | 1 |
| TRACH2009107 | 0.013340788 | 61272 | 2 |
| TRACH2009123 | 0.001876842 | 157440 | 1 |
| TRACH2009268 | 0.001876842 | 223225 | 1 |
| TRACH2009291 | 0.008022368 | 167986 | 2 |
| TRACH2009310 | 0.009543789 | 85367 | 8 |
| TRACH2009340 | 0.016928499 | 48800 | 5 |
| TRACH2009348 | 0.001876842 | 140468 | 1 |
| TRACH2009358 | 0.001876842 | 222437 | 1 |
| TRACH2009661 | 0.00297212 | 2682 | 2 |
| TRACH2009730 | 0.005630525 | 227909 | 3 |
| TRACH2009743 | 0.001876842 | 148417 | 1 |
| TRACH2009754 | 0.001876842 | 145875 | 1 |
| TRACH2009851 | 0.011143174 | 150463 | 3 |
| TRACH2009862 | 0.001876842 | 283468 | 1 |
| TRACH2009934 | 0.003273997 | 98938 | 2 |
| TRACH2010159 | 0.001876842 | 110788 | 1 |
| TRACH2010218 | 0.001876842 | 264022 | 1 |
| TRACH2010251 | 0.001876842 | 148437 | 1 |
| TRACH2010272 | 0.001876842 | 136249 | 1 |
| TRACH2010317 | 0.003273997 | 16933 | 2 |
| TRACH2010419 | 0.001876842 | 163476 | 1 |
| TRACH2010451 | 0.001876842 | 148382 | 1 |
| TRACH2010505 | 0.001876842 | 154376 | 1 |
| TRACH2010587 | 0.001876842 | 145809 | 1 |
| TRACH2010600 | 0.004858801 | 92782 | 2 |
| TRACH2010634 | 0.009665945 | 27668 | 3 |
| TRACH2010639 | 0.001876842 | 156236 | 1 |
| TRACH2010677 | 0.001876842 | 132658 | 1 |
| TRACH2010698 | 0.001876842 | 51785 | 1 |
| TRACH2010771 | 0.004921723 | 56191 | 2 |
| TRACH2010824 | 0.009328365 | 113547 | 3 |
| TRACH2010883 | 0.001876842 | 199012 | 1 |
| TRACH2010965 | 0.001876842 | 158166 | 1 |
| TRACH2010974 | 0.003753683 | 125787 | 2 |
| TRACH2011057 | 0.001876842 | 262384 | 1 |
| TRACH2011093 | 0.001876842 | 137969 | 1 |
| TRACH2011113 | 0.035489678 | 114027 | 12 |
| TRACH2011290 | 0.003958354 | 103688 | 2 |
| TRACH2011293 | 0.037527466 | 125652 | 2 |
| TRACH2011302 | 0.01367486 | 71237 | 2 |
| TRACH2011500 | 0.012409369 | 96737 | 4 |
| TRACH2011574 | 0.001876842 | 175762 | 1 |
| TRACH2011894 | 0.018842558 | 86937 | 6 |
| TRACH2011956 | 0.001876842 | 263220 | 1 |
| TRACH2012000 | 0.001876842 | 153704 | 1 |
| TRACH2012138 | 0.001876842 | 179717 | 1 |
| TRACH2012242 | 0.001876842 | 183612 | 1 |
| TRACH2012298 | 0.118079062 | 117877 | 7 |
| TRACH2012370 | 0.001876842 | 190304 | 1 |
| TRACH2012376 | 0.062430239 | 97950 | 8 |
| TRACH2012387 | 0.00297212 | 126040 | 2 |
| TRACH2012497 | 0.025884721 | 176593 | 5 |
| TRACH2012536 | 0.004858801 | 166814 | 2 |
| TRACH2012562 | 0.001876842 | 171403 | 1 |
| TRACH2012647 | 0.025659598 | 158951 | 4 |
| TRACH2012742 | 0.001876842 | 9907 | 1 |
| TRACH2012811 | 0.021492274 | 150004 | 9 |
| TRACH2012823 | 0.001876842 | 253010 | 1 |
| TRACH2012825 | 0.667575146 | 98766 | 29 |
| TRACH2012918 | 0.001876842 | 174628 | 1 |
| TRACH2013081 | 0.014400323 | 154045 | 2 |
| TRACH2013123 | 0.013932344 | 124923 | 5 |
| TRACH2013265 | 0.001876842 | 277538 | 1 |
| TRACH2013369 | 0.003753683 | 117224 | 2 |
| TRACH2013450 | 0.001876842 | 157300 | 1 |
| TRACH2013495 | 0.014938493 | 158093 | 2 |
| TRACH2013552 | 0.016128461 | 98426 | 8 |
| TRACH2013585 | 0.007138691 | 18216 | 4 |
| TRACH2013671 | 0.001876842 | 210889 | 1 |
| TRACH2013726 | 0.001876842 | 167773 | 1 |
| TRACH2013902 | 0.001876842 | 138049 | 1 |
| TRACH2013928 | 0.001876842 | 140485 | 1 |
| TRACH2013982 | 0.001876842 | 14547 | 1 |
| TRACH2013991 | 0.013163523 | 130600 | 2 |
| TRACH2014000 | 0.355708299 | 94682 | 31 |
| TRACH2014018 | 0.001876842 | 147212 | 1 |
| TRACH2014045 | 0.001876842 | 128087 | 1 |
| TRACH2014046 | 0.001876842 | 39655 | 1 |
| TRACH2014077 | 0.049257269 | 34939 | 17 |
| TRACH2014082 | 0.001876842 | 154005 | 1 |
| TRACH2014124 | 0.162328919 | 131813 | 49 |
| TRACH2014268 | 0.001876842 | 256434 | 1 |
| TRACH2014284 | 0.001876842 | 154106 | 1 |
| TRACH2014337 | 0.001876842 | 157366 | 1 |
| TRACH2014356 | 0.001876842 | 49560 | 1 |
| TRACH2014371 | 0.001876842 | 40434 | 1 |
| TRACH2014442 | 0.074424533 | 4108 | 6 |
| TRACH2014483 | 0.001876842 | 123814 | 1 |
| TRACH2014495 | 0.001876842 | 144422 | 1 |
| TRACH2014544 | 0.001876842 | 179848 | 1 |
| TRACH2014573 | 0.001876842 | 162234 | 1 |
| TRACH2014725 | 0.001876842 | 61364 | 1 |
| TRACH2014760 | 0.001876842 | 136283 | 1 |
| TRACH2014815 | 0.001876842 | 14392 | 1 |
| TRACH2014839 | 0.001876842 | 158466 | 1 |
| TRACH2014938 | 0.001876842 | 159914 | 1 |
| TRACH2014950 | 0.001876842 | 154064 | 1 |
| TRACH2014972 | 0.001876842 | 144390 | 1 |
| TRACH2014974 | 0.001876842 | 146088 | 1 |
| TRACH2014997 | 0.047363123 | 23345 | 22 |
| TRACH2015180 | 0.001876842 | 48152 | 1 |
| TRACH2015243 | 0.001876842 | 123656 | 1 |
| TRACH2015316 | 0.001876842 | 160673 | 1 |
| TRACH2015381 | 0.001876842 | 160346 | 1 |
| TRACH2015486 | 0.001876842 | 68799 | 1 |
| TRACH2015654 | 0.008022368 | 78189 | 2 |
| TRACH2015823 | 0.076596152 | 100522 | 29 |
| TRACH2015824 | 0.072099206 | 125594 | 6 |
| TRACH2015939 | 0.029828108 | 21200 | 10 |
| TRACH2015987 | 0.001876842 | 36191 | 1 |
| TRACH2016080 | 0.001876842 | 268764 | 1 |
| TRACH2016131 | 0.005720926 | 134943 | 2 |
| TRACH2016286 | 0.001876842 | 125563 | 1 |
| TRACH2016317 | 0.021490469 | 21301 | 5 |
| TRACH2016347 | 0.001876842 | 127306 | 1 |
| TRACH2016380 | 0.001876842 | 142848 | 1 |
| TRACH2016384 | 0.001876842 | 99180 | 1 |
| TRACH2016410 | 0.001876842 | 175900 | 1 |
| TRACH2016481 | 0.003875243 | 13285 | 2 |
| TRACH2016498 | 0.001876842 | 87826 | 1 |
| TRACH2016533 | 0.011690111 | 32774 | 6 |
| TRACH2016551 | 0.001876842 | 272634 | 1 |
| TRACH2016554 | 0.001876842 | 144659 | 1 |
| TRACH2016651 | 0.001876842 | 152744 | 1 |
| TRACH2016690 | 0.001876842 | 92652 | 1 |
| TRACH2016709 | 0.001876842 | 272735 | 1 |
| TRACH2016722 | 0.001876842 | 127219 | 1 |
| TRACH2016835 | 0.009079175 | 45209 | 3 |
| TRACH2016980 | 0.001876842 | 130485 | 1 |
| TRACH2017086 | 0.001876842 | 71926 | 1 |
| TRACH2017368 | 0.038333276 | 157398 | 2 |
| TRACH2017498 | 0.001876842 | 110805 | 1 |
| TRACH2017609 | 0.005272398 | 103284 | 3 |
| TRACH2017949 | 0.005630525 | 168962 | 3 |
| TRACH2017965 | 0.001876842 | 91013 | 1 |
| TRACH2017980 | 0.001876842 | 166790 | 1 |
| TRACH2018034 | 0.003734956 | 200240 | 2 |
| TRACH2018084 | 0.001876842 | 114368 | 1 |
| TRACH2018262 | 0.001876842 | 52390 | 1 |
| TRACH2018273 | 0.001876842 | 131282 | 1 |
| TRACH2018278 | 0.001876842 | 157470 | 1 |
| TRACH2018317 | 0.057195045 | 56920 | 10 |
| TRACH2018345 | 0.001876842 | 182521 | 1 |
| TRACH2018380 | 0.001876842 | 182531 | 1 |
| TRACH2018446 | 0.040065241 | 66102 | 14 |
| TRACH2018449 | 0.011051281 | 51446 | 4 |
| TRACH2018512 | 0.001876842 | 269664 | 1 |
| TRACH2018646 | 0.001876842 | 77749 | 1 |
| TRACH2018663 | 0.001876842 | 65500 | 1 |
| TRACH2018718 | 0.001876842 | 183680 | 1 |
| TRACH2018835 | 0.001876842 | 169618 | 1 |
| TRACH2018882 | 0.003753683 | 41785 | 2 |
| TRACH2018914 | 0.001876842 | 171952 | 1 |
| TRACH2018950 | 0.022309481 | 152852 | 6 |
| TRACH2019024 | 0.001876842 | 49857 | 1 |
| TRACH2019046 | 0.001876842 | 67409 | 1 |
| TRACH2019080 | 0.005490733 | 84568 | 2 |
| TRACH2019151 | 0.001876842 | 9333 | 1 |
| TRACH2019154 | 0.001876842 | 24123 | 1 |
| TRACH2019176 | 0.001876842 | 144549 | 1 |
| TRACH2019248 | 0.001876842 | 276703 | 1 |
| TRACH2019309 | 0.001876842 | 264427 | 1 |
| TRACH2019425 | 0.014938493 | 183154 | 2 |
| TRACH2019473 | 0.001876842 | 249865 | 1 |
| TRACH2019602 | 0.001876842 | 153583 | 1 |
| TRACH2019661 | 0.001876842 | 114997 | 1 |
| TRACH2019672 | 0.008022368 | 122470 | 2 |
| TRACH2019673 | 0.001876842 | 272726 | 1 |
| TRACH2019844 | 0.001876842 | 253012 | 1 |
| TRACH2019879 | 0.001876842 | 140527 | 1 |
| TRACH2020048 | 0.001876842 | 235990 | 1 |
| TRACH2020336 | 0.001876842 | 243539 | 1 |
| TRACH2020525 | 0.001876842 | 284367 | 1 |
| TRACH2021398 | 0.001876842 | 260706 | 1 |
| TRACH2021622 | 0.001876842 | 277720 | 1 |
| TRACH2021635 | 0.001876842 | 249901 | 1 |
| TRACH2021964 | 0.001876842 | 267288 | 1 |
| TRACH2022042 | 0.001876842 | 282946 | 1 |
| TRACH2022113 | 0.001876842 | 261325 | 1 |
| TRACH2022253 | 0.001876842 | 277822 | 1 |
| TRACH2022425 | 0.001876842 | 274968 | 1 |
| TRACH2022553 | 0.001876842 | 71923 | 1 |
| TRACH2022649 | 0.001876842 | 206747 | 1 |
| TRACH2022839 | 0.003753683 | 220597 | 2 |
| TRACH2023246 | 0.001876842 | 241348 | 1 |
| TRACH2023299 | 0.026250164 | 207353 | 5 |
| TRACH2023306 | 0.001876842 | 255352 | 1 |
| TRACH2023416 | 0.001876842 | 268069 | 1 |
| TRACH2023479 | 0.001876842 | 140549 | 1 |
| TRACH2023684 | 0.001876842 | 259406 | 1 |
| TRACH2023860 | 0.001876842 | 256569 | 1 |
| TRACH2023876 | 0.001876842 | 253096 | 1 |
| TRACH2024139 | 0.001876842 | 268771 | 1 |
| TRACH2024187 | 0.001876842 | 274191 | 1 |
| TRACH2024271 | 0.003753683 | 94919 | 2 |
| TRACH2024293 | 0.01038032 | 87630 | 6 |
| TRACH2024408 | 0.01126105 | 148274 | 6 |
| TRACH2024512 | 0.001876842 | 199706 | 1 |
| TRACH2024559 | 0.001876842 | 280923 | 1 |
| TRACH2024647 | 0.001876842 | 282682 | 1 |
| TRACH2024651 | 0.001876842 | 235220 | 1 |
| TRACH2024730 | 0.001876842 | 269390 | 1 |
| TRACH2024735 | 0.001876842 | 217471 | 1 |
| TRACH2024749 | 0.001876842 | 273443 | 1 |
| TRACH2024821 | 0.001876842 | 265284 | 1 |
| TRACH2025057 | 0.001876842 | 276642 | 1 |
| TRACH2025084 | 0.001876842 | 257781 | 1 |
| TRACH2025155 | 0.001876842 | 245148 | 1 |
| TRACH2025224 | 0.001876842 | 228042 | 1 |
| TRACH2025344 | 0.01701395 | 109067 | 4 |
| TRACH2025379 | 0.001876842 | 275880 | 1 |
| TRACH2025507 | 0.001876842 | 245203 | 1 |
| TRACH2025535 | 0.288606486 | 4524 | 61 |
| TRACH2025569 | 0.001876842 | 253100 | 1 |
| TRACH2025705 | 0.001876842 | 265082 | 1 |
| TRACH2025749 | 0.001876842 | 249624 | 1 |
| TRACH2025753 | 0.001876842 | 283933 | 1 |
| TRACH2025810 | 0.001876842 | 256163 | 1 |
| TRACH2025853 | 0.001876842 | 261787 | 1 |
| TRACH2025911 | 0.003565118 | 283057 | 2 |
| TRACH2025932 | 0.001876842 | 224732 | 1 |
| TRACH3000014 | 0.009216827 | 186805 | 5 |
| TRACH3000017 | 0.003753683 | 245332 | 2 |
| TRACH3000043 | 0.001876842 | 217461 | 1 |
| TRACH3000134 | 0.009266219 | 84174 | 4 |
| TRACH3000180 | 0.001876842 | 228038 | 1 |
| TRACH3000342 | 0.001876842 | 201776 | 1 |
| TRACH3000420 | 0.001876842 | 257836 | 1 |
| TRACH3000515 | 0.001876842 | 280537 | 1 |
| TRACH3000530 | 0.003753683 | 127523 | 2 |
| TRACH3000548 | 0.001876842 | 211071 | 1 |
| TRACH3000558 | 0.001876842 | 202491 | 1 |
| TRACH3000586 | 0.001876842 | 282036 | 1 |
| TRACH3000692 | 0.035750286 | 8476 | 9 |
| TRACH3000780 | 0.003586302 | 271027 | 2 |
| TRACH3000892 | 0.001876842 | 284048 | 1 |
| TRACH3000926 | 0.001876842 | 280089 | 1 |
| TRACH3001119 | 0.007507367 | 32761 | 4 |
| TRACH3001427 | 0.180469591 | 11446 | 57 |
| TRACH3001443 | 0.005630525 | 189457 | 3 |
| TRACH3001550 | 0.003586302 | 236721 | 2 |
| TRACH3001759 | 0.001876842 | 258472 | 1 |
| TRACH3002064 | 0.001876842 | 148286 | 1 |
| TRACH3002107 | 0.001876842 | 234535 | 1 |
| TRACH3002168 | 0.001876842 | 215473 | 1 |
| TRACH3002188 | 0.001876842 | 223484 | 1 |
| TRACH3002192 | 0.268676242 | 60521 | 87 |
| TRACH3002245 | 0.001876842 | 251566 | 1 |
| TRACH3002293 | 0.001876842 | 269341 | 1 |
| TRACH3002533 | 0.001876842 | 243083 | 1 |
| TRACH3002561 | 0.001876842 | 4921 | 1 |
| TRACH3002567 | 0.007507367 | 140929 | 4 |
| TRACH3002605 | 0.003586302 | 266291 | 2 |
| TRACH3002650 | 0.001876842 | 277151 | 1 |
| TRACH3002752 | 0.032805351 | 119633 | 10 |
| TRACH3002866 | 0.001876842 | 215487 | 1 |
| TRACH3002871 | 0.001876842 | 148877 | 1 |
| TRACH3002876 | 0.001876842 | 265394 | 1 |
| TRACH3002890 | 0.001876842 | 269774 | 1 |
| TRACH3003009 | 0.001876842 | 280155 | 1 |
| TRACH3003037 | 0.005227936 | 109915 | 3 |
| TRACH3003357 | 0.001876842 | 276382 | 1 |
| TRACH3003379 | 0.006847363 | 158448 | 4 |
| TRACH3003547 | 0.039621581 | 86472 | 12 |
| TRACH3003586 | 0.001876842 | 251535 | 1 |
| TRACH3003683 | 0.001876842 | 43507 | 1 |
| TRACH3003780 | 0.001876842 | 260725 | 1 |
| TRACH3003805 | 0.001876842 | 264578 | 1 |
| TRACH3003832 | 0.013799318 | 132934 | 3 |
| TRACH3003872 | 0.061047945 | 75455 | 28 |
| TRACH3004068 | 0.117561666 | 74911 | 12 |
| TRACH3004113 | 0.001876842 | 243746 | 1 |
| TRACH3004288 | 0.001876842 | 249698 | 1 |
| TRACH3004303 | 0.003753683 | 155515 | 2 |
| TRACH3004412 | 0.001876842 | 268232 | 1 |
| TRACH3004424 | 0.00985254 | 18797 | 5 |
| TRACH3004456 | 0.001876842 | 229453 | 1 |
| TRACH3004537 | 0.001876842 | 127939 | 1 |
| TRACH3004596 | 0.001876842 | 253107 | 1 |
| TRACH3004651 | 0.001876842 | 49800 | 1 |
| TRACH3004652 | 0.001876842 | 218594 | 1 |
| TRACH3004671 | 0.001876842 | 30389 | 1 |
| TRACH3004721 | 0.057637274 | 70607 | 23 |
| TRACH3004747 | 0.003753683 | 195023 | 2 |
| TRACH3004760 | 0.001876842 | 260142 | 1 |
| TRACH3004783 | 0.001876842 | 233109 | 1 |
| TRACH3004786 | 0.001876842 | 255574 | 1 |
| TRACH3004840 | 0.001876842 | 4942 | 1 |
| TRACH3004931 | 0.001876842 | 235908 | 1 |
| TRACH3004934 | 0.003753683 | 239688 | 2 |
| TRACH3005072 | 0.001876842 | 243884 | 1 |
| TRACH3005085 | 0.001876842 | 248984 | 1 |
| TRACH3005102 | 0.001876842 | 273727 | 1 |
| TRACH3005173 | 0.001876842 | 219772 | 1 |
| TRACH3005191 | 0.001876842 | 271368 | 1 |
| TRACH3005211 | 0.009091915 | 225679 | 4 |
| TRACH3005274 | 0.001876842 | 266317 | 1 |
| TRACH3005294 | 0.04511614 | 131092 | 6 |
| TRACH3005479 | 0.00297212 | 146853 | 2 |
| TRACH3005549 | 0.001876842 | 245278 | 1 |
| TRACH3005699 | 0.017458248 | 155387 | 9 |
| TRACH3005702 | 0.003753683 | 244030 | 2 |
| TRACH3005808 | 0.001876842 | 244043 | 1 |
| TRACH3005852 | 0.001876842 | 210434 | 1 |
| TRACH3005896 | 0.007507367 | 135731 | 4 |
| TRACH3006038 | 0.003066086 | 243120 | 2 |
| TRACH3006149 | 0.001876842 | 243123 | 1 |
| TRACH3006228 | 0.001876842 | 236664 | 1 |
| TRACH3006264 | 0.001876842 | 277392 | 1 |
| TRACH3006336 | 0.003753683 | 208304 | 2 |
| TRACH3006379 | 0.001876842 | 275677 | 1 |
| TRACH3006392 | 0.007507367 | 135570 | 4 |
| TRACH3006395 | 0.001876842 | 277499 | 1 |
| TRACH3006397 | 0.001876842 | 271283 | 1 |
| TRACH3006412 | 0.003066086 | 246480 | 2 |
| TRACH3006470 | 0.001876842 | 194345 | 1 |
| TRACH3006589 | 0.001876842 | 190658 | 1 |
| TRACH3006626 | 0.007507367 | 122228 | 4 |
| TRACH3006699 | 0.001876842 | 265793 | 1 |
| TRACH3006717 | 0.045662706 | 74289 | 24 |
| TRACH3006800 | 0.005752085 | 212445 | 3 |
| TRACH3006889 | 0.001876842 | 233075 | 1 |
| TRACH3006985 | 0.001876842 | 280680 | 1 |
| TRACH3007097 | 0.001876842 | 280993 | 1 |
| TRACH3007268 | 0.001876842 | 273872 | 1 |
| TRACH3007274 | 0.001876842 | 270677 | 1 |
| TRACH3007277 | 0.001876842 | 281544 | 1 |
| TRACH3007391 | 0.001876842 | 281081 | 1 |
| TRACH3007479 | 0.155801381 | 87430 | 34 |
| TRACH3007541 | 0.003273997 | 141938 | 2 |
| TRACH3007595 | 0.001876842 | 280444 | 1 |
| TRACH3007625 | 0.142539258 | 115165 | 45 |
| TRACH3007627 | 0.001876842 | 134003 | 1 |
| TRACH3007689 | 0.007104777 | 151321 | 4 |
| TRACH3007766 | 0.001876842 | 268534 | 1 |
| TRACH3007866 | 0.001876842 | 76441 | 1 |
| TRACH3007995 | 0.003066086 | 251471 | 2 |
| TRACH3008042 | 0.001876842 | 271324 | 1 |
| TRACH3008061 | 0.007507367 | 150881 | 4 |
| TRACH3008093 | 0.06840904 | 144960 | 18 |
| TRACH3008201 | 0.003552389 | 52462 | 2 |
| TRACH3008508 | 0.008746003 | 118708 | 4 |
| TRACH3008535 | 0.005915125 | 113178 | 2 |
| TRACH3008629 | 0.03675527 | 98336 | 20 |
| TRACH3008632 | 0.019828308 | 140383 | 11 |
| TRACH3008688 | 0.001876842 | 245043 | 1 |
| TRACH3008697 | 0.001876842 | 243103 | 1 |
| TRACH3008713 | 0.005915125 | 189254 | 2 |
| TRACH3008887 | 0.001876842 | 247226 | 1 |
| TRACH3008902 | 0.001876842 | 219889 | 1 |
| TRACH3008990 | 0.003753683 | 174885 | 2 |
| TRACH3009001 | 0.00715142 | 225239 | 4 |
| TRACH3009008 | 0.034201874 | 78544 | 12 |
| TRACH3009059 | 0.003753683 | 224664 | 2 |
| TRACH3009061 | 0.003753683 | 131062 | 2 |
| TRACH3009148 | 0.018680989 | 13251 | 8 |
| TRACH3009455 | 0.003552389 | 15498 | 2 |
| TRACH3009701 | 0.001876842 | 258527 | 1 |
| TRACH3009715 | 0.001876842 | 257303 | 1 |
| TRACH3009894 | 0.001876842 | 261097 | 1 |
| TRACH3009956 | 0.004255331 | 154228 | 3 |
| TRACH3010079 | 0.006937396 | 37145 | 4 |
| TRACH3010167 | 0.001876842 | 194380 | 1 |
| TRACH3010331 | 0.001876842 | 49360 | 1 |
| TRACH3010342 | 0.001876842 | 103396 | 1 |
| TRACH3010382 | 0.005630525 | 125847 | 3 |
| TRACH3010757 | 0.001876842 | 224688 | 1 |
| TRACH3010901 | 0.001876842 | 189290 | 1 |
| TRACH3011004 | 0.001876842 | 189294 | 1 |
| TRACH3011082 | 0.001876842 | 195842 | 1 |
| TRACH3011184 | 0.001876842 | 212713 | 1 |
| TRACH3011282 | 0.001876842 | 74226 | 1 |
| TRACH3011313 | 0.001876842 | 53299 | 1 |
| TRACH3011454 | 0.001876842 | 190734 | 1 |
| TRACH3011485 | 0.001876842 | 219644 | 1 |
| TRACH3011503 | 0.001876842 | 131922 | 1 |
| TRACH3011538 | 0.001876842 | 235849 | 1 |
| TRACH3011542 | 0.007507367 | 204878 | 4 |
| TRACH3012106 | 0.001876842 | 18395 | 1 |
| TRACH3012161 | 0.001876842 | 276824 | 1 |
| TRACH3012232 | 0.001876842 | 251815 | 1 |
| TRACH3012261 | 0.003753683 | 116268 | 2 |
| TRACH3012445 | 0.001876842 | 281788 | 1 |
| TRACH3012460 | 0.001876842 | 112841 | 1 |
| TRACH3012659 | 0.001876842 | 272112 | 1 |
| TRACH3012718 | 0.001876842 | 239766 | 1 |
| TRACH3012864 | 0.001876842 | 163082 | 1 |
| TRACH3012891 | 0.001876842 | 179468 | 1 |
| TRACH3012942 | 0.001876842 | 196036 | 1 |
| TRACH3013043 | 0.004067398 | 75168 | 3 |
| TRACH3013072 | 0.003273997 | 128801 | 2 |
| TRACH3013426 | 0.001876842 | 179932 | 1 |
| TRACH3013454 | 0.001876842 | 127425 | 1 |
| TRACH3013558 | 0.003066086 | 189271 | 2 |
| TRACH3013684 | 0.001876842 | 241665 | 1 |
| TRACH3013693 | 0.001876842 | 251797 | 1 |
| TRACH3013700 | 0.001876842 | 78474 | 1 |
| TRACH3013766 | 0.001876842 | 241614 | 1 |
| TRACH3013900 | 0.001876842 | 135300 | 1 |
| TRACH3013926 | 0.001876842 | 168862 | 1 |
| TRACH3014063 | 0.001876842 | 166918 | 1 |
| TRACH3014183 | 0.001876842 | 180685 | 1. |
| TRACH3014215 | 0.001876842 | 190689 | 1 |
| TRACH3014316 | 0.003958354 | 225971 | 2 |
| TRACH3014415 | 0.001876842 | 233155 | 1 |
| TRACH3014580 | 0.001876842 | 229503 | 1 |
| TRACH3014641 | 0.001876842 | 153699 | 1 |
| TRACH3014885 | 0.001876842 | 218065 | 1 |
| TRACH3015136 | 0.001876842 | 236550 | 1 |
| TRACH3015336 | 0.001876842 | 19316 | 1 |
| TRACH3015346 | 0.001876842 | 95166 | 1 |
| TRACH3015354 | 0.001876842 | 87655 | 1 |
| TRACH3015467 | 0.001876842 | 189327 | 1 |
| TRACH3015626 | 0.001876842 | 34608 | 1 |
| TRACH3015951 | 0.001876842 | 243242 | 1 |
| TRACH3016051 | 0.001876842 | 236508 | 1 |
| TRACH3016120 | 0.001876842 | 234557 | 1 |
| TRACH3016264 | 0.003753683 | 222836 | 2 |
| TRACH3016277 | 0.001876842 | 223941 | 1 |
| TRACH3016368 | 0.001876842 | 221489 | 1 |
| TRACH3016455 | 0.001876842 | 213599 | 1 |
| TRACH3016614 | 0.001876842 | 138036 | 1 |
| TRACH3016805 | 0.037214621 | 91739 | 14 |
| TRACH3016885 | 0.042118119 | 123560 | 15 |
| TRACH3016992 | 0.007039518 | 150088 | 5 |
| TRACH3017171 | 0.001876842 | 223891 | 1 |
| TRACH3017409 | 0.001876842 | 165075 | 1 |
| TRACH3017934 | 0.001876842 | 189377 | 1 |
| TRACH3018108 | 0.001876842 | 262013 | 1 |
| TRACH3018191 | 0.001876842 | 138047 | 1 |
| TRACH3018240 | 0.001876842 | 284234 | 1 |
| TRACH3018261 | 0.001876842 | 148648 | 1 |
| TRACH3018519 | 0.001876842 | 56557 | 1 |
| TRACH3018524 | 0.001876842 | 145402 | 1 |
| TRACH3018606 | 0.001876842 | 233123 | 1 |
| TRACH3018783 | 0.001876842 | 166651 | 1 |
| TRACH3018907 | 0.001876842 | 114372 | 1 |
| TRACH3018933 | 0.001876842 | 160812 | 1 |
| TRACH3018943 | 0.001876842 | 166620 | 1 |
| TRACH3019018 | 0.001876842 | 154882 | 1 |
| TRACH3019058 | 0.001876842 | 169335 | 1 |
| TRACH3019142 | 0.001876842 | 169257 | 1 |
| TRACH3019290 | 0.006057552 | 18706 | 4 |
| TRACH3019347 | 0.001876842 | 174757 | 1 |
| TRACH3019370 | 0.001876842 | 142417 | 1 |
| TRACH3019447 | 0.001876842 | 243757 | 1 |
| TRACH3019598 | 0.001876842 | 235379 | 1 |
| TRACH3019621 | 0.001876842 | 160747 | 1 |
| TRACH3019662 | 0.001876842 | 175564 | 1 |
| TRACH3019807 | 0.001876842 | 179283 | 1 |
| TRACH3019924 | 0.001876842 | 117292 | 1 |
| TRACH3019977 | 0.003753683 | 229781 | 2 |
| TRACH3020137 | 0.001876842 | 169268 | 1 |
| TRACH3020410 | 0.001876842 | 246856 | 1 |
| TRACH3020563 | 0.001876842 | 128802 | 1 |
| TRACH3020605 | 0.001876842 | 262103 | 1 |
| TRACH3020750 | 0.001876842 | 166752 | 1 |
| TRACH3020769 | 0.001876842 | 84736 | 1 |
| TRACH3020928 | 0.001876842 | 264847 | 1 |
| TRACH3020930 | 0.001876842 | 277108 | 1 |
| TRACH3021023 | 0.001876842 | 71917 | 1 |
| TRACH3021066 | 0.001876842 | 189461 | 1 |
| TRACH3021212 | 0.005630525 | 194957 | 3 |
| TRACH3021335 | 0.001876842 | 237814 | 1 |
| TRACH3021373 | 0.001876842 | 20519 | 1 |
| TRACH3021544 | 0.003958354 | 155645 | 2 |
| TRACH3021778 | 0.003753683 | 175469 | 2 |
| TRACH3021834 | 0.001876842 | 85372 | 1 |
| TRACH3021883 | 0.001876842 | 150048 | 1 |
| TRACH3022109 | 0.003958354 | 110875 | 2 |
| TRACH3022170 | 0.001876842 | 121920 | 1 |
| TRACH3022198 | 0.020159652 | 62639 | 7 |
| TRACH3022296 | 0.001876842 | 141219 | 1 |
| TRACH3022482 | 0.001876842 | 237780 | 1 |
| TRACH3022732 | 0.001876842 | 119155 | 1 |
| TRACH3022758 | 0.001876842 | 138622 | 1 |
| TRACH3022875 | 0.001876842 | 207178 | 1. |
| TRACH3022910 | 0.003753683 | 140795 | 2 |
| TRACH3022960 | 0.001876842 | 138995 | 1 |
| TRACH3023063 | 0.001876842 | 119639 | 1 |
| TRACH3023203 | 0.001876842 | 129526 | 1 |
| TRACH3023242 | 0.001876842 | 117212 | 1 |
| TRACH3023373 | 0.001876842 | 124177 | 1 |
| TRACH3023516 | 0.001876842 | 45835 | 1 |
| TRACH3023752 | 0.001876842 | 138873 | 1 |
| TRACH3023945 | 0.003753683 | 129954 | 2 |
| TRACH3023960 | 0.001876842 | 250715 | 1 |
| TRACH3024020 | 0.001876842 | 154790 | 1 |
| TRACH3024081 | 0.001876842 | 123327 | 1 |
| TRACH3024342 | 0.003552389 | 116975 | 2 |
| TRACH3024423 | 0.001876842 | 115246 | 1 |
| TRACH3024428 | 0.001876842 | 119587 | 1 |
| TRACH3024512 | 0.001876842 | 132721 | 1 |
| TRACH3024671 | 0.003552389 | 153234 | 2 |
| TRACH3024823 | 0.005630525 | 163611 | 3 |
| TRACH3025302 | 0.001876842 | 55453 | 1 |
| TRACH3025316 | 0.005463143 | 51859 | 3 |
| TRACH3025346 | 0.001876842 | 198391 | 1 |
| TRACH3025520 | 0.001876842 | 260860 | 1 |
| TRACH3025828 | 0.246532535 | 78679 | 51 |
| TRACH3026148 | 0.001876842 | 139092 | 1 |
| TRACH3026283 | 0.001876842 | 86474 | 1 |
| TRACH3026299 | 0.001876842 | 119570 | 1 |
| TRACH3026303 | 0.029895056 | 90578 | 14 |
| TRACH3026542 | 0.001876842 | 242058 | 1 |
| TRACH3026650 | 0.001876842 | 249108 | 1 |
| TRACH3026676 | 0.014405426 | 89205 | 7 |
| TRACH3026744 | 0.001876842 | 250510 | 1 |
| TRACH3026949 | 0.001876842 | 111185 | 1 |
| TRACH3027229 | 0.030925498 | 127139 | 3 |
| TRACH3027527 | 0.001876842 | 148219 | 1 |
| TRACH3027681 | 0.001876842 | 174333 | 1 |
| TRACH3027701 | 0.001876842 | 123857 | 1 |
| TRACH3027885 | 0.001876842 | 174328 | 1 |
| TRACH3028031 | 0.001876842 | 161247 | 1 |
| TRACH3028164 | 0.001876842 | 138712 | 1 |
| TRACH3028180 | 0.001876842 | 148503 | 1 |
| TRACH3028195 | 0.001876842 | 130829 | 1 |
| TRACH3028441 | 0.001876842 | 280152 | 1 |
| TRACH3028597 | 0.001876842 | 8558 | 1 |
| TRACH3028678 | 0.005630525 | 155496 | 3 |
| TRACH3028837 | 0.001876842 | 53784 | 1 |
| TRACH3028847 | 0.001876842 | 161404 | 1 |
| TRACH3028855 | 0.007510742 | 86407 | 4 |
| TRACH3029139 | 0.001876842 | 196882 | 1 |
| TRACH3029329 | 0.001876842 | 262849 | 1 |
| TRACH3029451 | 0.001876842 | 283632 | 1 |
| TRACH3029462 | 0.001876842 | 47701 | 1 |
| TRACH3029520 | 0.001876842 | 207640 | 1 |
| TRACH3029592 | 0.001876842 | 189952 | 1 |
| TRACH3029629 | 0.001876842 | 161388 | 1 |
| TRACH3029665 | 0.001876842 | 167291 | 1 |
| TRACH3029670 | 0.001876842 | 180015 | 1 |
| TRACH3029800 | 0.001876842 | 163806 | 1 |
| TRACH3030111 | 0.001876842 | 83336 | 1 |
| TRACH3030130 | 0.001876842 | 220045 | 1 |
| TRACH3030176 | 0.001876842 | 180062 | 1 |
| TRACH3030252 | 0.001876842 | 140398 | 1 |
| TRACH3030725 | 0.001876842 | 244223 | 1 |
| TRACH3030855 | 0.001876842 | 174411 | 1 |
| TRACH3030883 | 0.001876842 | 260140 | 1 |
| TRACH3031316 | 0.001876842 | 130732 | 1 |
| TRACH3031400 | 0.001876842 | 136996 | 1 |
| TRACH3031463 | 0.001876842 | 212337 | 1 |
| TRACH3031479 | 0.001876842 | 264226 | 1 |
| TRACH3031518 | 0.001876842 | 272754 | 1 |
| TRACH3031660 | 0.001876842 | 129312 | 1 |
| TRACH3031678 | 0.001876842 | 87283 | 1 |
| TRACH3031936 | 0.001876842 | 279863 | 1 |
| TRACH3032044 | 0.001876842 | 172259 | 1 |
| TRACH3032065 | 0.003753683 | 142549 | 2 |
| TRACH3032150 | 0.001876842 | 128390 | 1 |
| TRACH3032372 | 0.001876842 | 187096 | 1 |
| TRACH3032480 | 0.001876842 | 260297 | 1 |
| TRACH3032570 | 0.003552389 | 174903 | 2 |
| TRACH3032755 | 0.001876842 | 279799 | 1 |
| TRACH3032827 | 0.001876842 | 194662 | 1 |
| TRACH3032873 | 0.001876842 | 152946 | 1 |
| TRACH3033535 | 0.001876842 | 281825 | 1 |
| TRACH3033680 | 0.001876842 | 123854 | 1 |
| TRACH3033868 | 0.001876842 | 127273 | 1 |
| TRACH3034145 | 0.001876842 | 200636 | 1 |
| TRACH3034414 | 0.001876842 | 269399 | 1 |
| TRACH3034488 | 0.001876842 | 167054 | 1 |
| TRACH3034680 | 0.001876842 | 71913 | 1 |
| TRACH3034731 | 0.003753683 | 141698 | 2 |
| TRACH3034745 | 0.001876842 | 155463 | 1 |
| TRACH3034762 | 0.001876842 | 164871 | 1 |
| TRACH3034903 | 0.001876842 | 185160 | 1 |
| TRACH3035187 | 0.001876842 | 151231 | 1 |
| TRACH3035199 | 0.001876842 | 115616 | 1 |
| TRACH3035235 | 0.001876842 | 163148 | 1 |
| TRACH3035451 | 0.001876842 | 159501 | 1 |
| TRACH3035482 | 0.001876842 | 192639 | 1 |
| TRACH3035526 | 0.001876842 | 189111 | 1 |
| TRACH3036004 | 0.001876842 | 127213 | 1 |
| TRACH3036103 | 0.001876842 | 146858 | 1 |
| TRACH3036193 | 0.001876842 | 184434 | 1 |
| TRACH3036207 | 0.003753683 | 90727 | 2 |
| TRACH3036278 | 0.001876842 | 128695 | 1 |
| TRACH3036309 | 0.001876842 | 174244 | 1 |
| TRACH3036456 | 0.001876842 | 142032 | 1 |
| TRACH3036609 | 0.001876842 | 159717 | 1 |
| TRACH3036638 | 0.001876842 | 165056 | 1 |
| TRACH3036683 | 0.003753683 | 166997 | 2 |
| TRACH3036750 | 0.001876842 | 146956 | 1 |
| TRACH3036792 | 0.001876842 | 154992 | 1 |
| TRACH3036843 | 0.001876842 | 190067 | 1 |
| TRACH3036897 | 0.001876842 | 163262 | 1 |
| TRACH3036932 | 0.011430014 | 125601 | 6 |
| TRACH3036942 | 0.686716771 | 136569 | 9 |
| TRACH3036997 | 0.001876842 | 133472 | 1 |
| TRACH3037063 | 0.001876842 | 137132 | 1 |
| TRACH3037067 | 0.104236998 | 128155 | 12 |
| TRACH3037267 | 0.001876842 | 142640 | 1 |
| TRACH3037288 | 0.001876842 | 49309 | 1 |
| TRACH3037505 | 0.001876842 | 135467 | 1 |
| TRACH3037573 | 0.001876842 | 133504 | 1 |
| TRACH3037696 | 0.001876842 | 137127 | 1 |
| TRACH3037897 | 0.001876842 | 136236 | 1 |
| TRACH3038086 | 0.001876842 | 114612 | 1 |
| TRACH3038399 | 0.013474237 | 133424 | 7 |
| TSTOM1000135 | 0.039700396 | 208608 | 3 |
| TSTOM1000186 | 0.035984167 | 221436 | 1 |
| TSTOM2000139 | 0.040061404 | 130365 | 3 |
| TSTOM2000235 | 0.037693627 | 136917 | 2 |
| TSTOM2000315 | 0.073523309 | 99167 | 12 |
| TSTOM2000442 | 0.035984167 | 273060 | 1 |
| TSTOM2000553 | 0.099395741 | 100797 | 21 |
| TSTOM2000569 | 0.035984167 | 70584 | 1 |
| TSTOM2000588 | 0.035984167 | 150490 | 1 |
| TSTOM2001195 | 0.035984167 | 231651 | 1 |
| TSTOM2001274 | 0.035984167 | 224127 | 1 |
| TSTOM2001571 | 0.035984167 | 196977 | 1 |
| TSTOM2001996 | 0.035984167 | 138448 | 1 |
| TSTOM2002265 | 0.035984167 | 207420 | 1 |
| TSTOM2002505 | 0.035984167 | 224138 | 1 |
| TSTOM2002561 | 0.035984167 | 199898 | 1 |
| TSTOM2002611 | 0.035984167 | 155921 | 1 |
| TSTOM2002672 | 0.035984167 | 278459 | 1 |
| TSTOM2002682 | 0.035984167 | 270648 | 1 |
| TUTER1000014 | 0.037341299 | 221698 | 1 |
| TUTER1000122 | 0.051336742 | 103372 | 9 |
| TUTER1000137 | 0.039016847 | 222374 | 2 |
| TUTER2000057 | 0.040386181 | 268032 | 2 |
| TUTER2000283 | 0.037341299 | 43096 | 1 |
| TUTER2000425 | 0.037341299 | 38750 | 1 |
| TUTER2000904 | 0.060017599 | 135875 | 14 |
| TUTER2000916 | 0.037341299 | 203159 | 1 |
| TUTER2001286 | 0.037341299 | 176751 | 1 |
| TUTER2001341 | 0.037341299 | 184704 | 1 |
| TUTER2001387 | 0.037341299 | 200612 | 1 |
| TUTER2001433 | 0.037341299 | 266343 | 1 |
| TUTER2001461 | 0.037341299 | 271545 | 1 |
| TUTER2002028 | 0.037341299 | 238559 | 1 |
| TUTER2002074 | 0.037341299 | 253334 | 1 |
| TUTER2002158 | 0.037341299 | 174532 | 1 |
| TUTER2002228 | 0.037341299 | 165834 | 1 |
| TUTER2002323 | 0.05144711 | 145377 | 4 |
| TUTER2002356 | 0.037341299 | 165069 | 1 |
| TUTER2002729 | 0.037341299 | 200723 | 1 |
| UMVEN1000122 | 0.171447898 | 70225 | 3 |
| UMVEN1000138 | 0.313578061 | 44329 | 66 |
| UMVEN1000143 | 0.2040239 | 29627 | 4 |
| UMVEN1000156 | 0.162154818 | 2310 | 2 |
| UMVEN1000186 | 0.634177857 | 17741 | 25 |
| UMVEN2000046 | 0.157977883 | 221603 | 1 |
| UMVEN2000069 | 0.157977883 | 143141 | 1 |
| UMVEN2000121 | 0.161712839 | 143359 | 3 |
| UMVEN2000133 | 0.285863748 | 118115 | 24 |
| UMVEN2000152 | 0.212724986 | 59628 | 12 |
| UMVEN2000354 | 0.203420643 | 156198 | 10 |
| UMVEN2000453 | 0.320907033 | 109109 | 47 |
| UTERU1000008 | 0.003395557 | 66387 | 2 |
| UTERU1000015 | 0.001998401 | 65373 | 1 |
| UTERU1000024 | 0.001998401 | 63730 | 1 |
| UTERU1000031 | 0.001998401 | 49244 | 1 |
| UTERU1000032 | 0.001998401 | 69926 | 1 |
| UTERU1000057 | 0.001998401 | 72104 | 1 |
| UTERU1000065 | 0.001998401 | 31345 | 1 |
| UTERU1000077 | 0.001998401 | 64540 | 1 |
| UTERU1000093 | 0.001998401 | 64580 | 1 |
| UTERU1000096 | 0.008234759 | 61603 | 2 |
| UTERU1000106 | 0.029076363 | 68024 | 7 |
| UTERU1000109 | 0.001998401 | 61936 | 1 |
| UTERU1000131 | 0.001998401 | 58954 | 1 |
| UTERU1000138 | 0.001998401 | 65283 | 1 |
| UTERU1000148 | 0.001998401 | 59066 | 1 |
| UTERU1000160 | 0.001998401 | 62014 | 1 |
| UTERU1000182 | 0.206560133 | 63305 | 18 |
| UTERU1000183 | 0.001998401 | 285325 | 1 |
| UTERU1000187 | 0.001998401 | 72182 | 1 |
| UTERU1000192 | 0.001998401 | 63618 | 1 |
| UTERU1000249 | 0.001998401 | 106727 | 1 |
| UTERU1000337 | 0.001998401 | 94863 | 1 |
| UTERU1000339 | 0.001998401 | 273538 | 1 |
| UTERU1000384 | 0.023509371 | 166464 | 10 |
| UTERU2000023 | 0.016367602 | 132806 | 10 |
| UTERU2000047 | 0.001998401 | 146975 | 1 |
| UTERU2000074 | 0.003187646 | 116970 | 2 |
| UTERU2000095 | 0.404833791 | 3101 | 68 |
| UTERU2000099 | 0.001998401 | 203684 | 1 |
| UTERU2000154 | 0.001998401 | 229 | 1 |
| UTERU2000197 | 0.005093897 | 174717 | 2 |
| UTERU2000218 | 0.001998401 | 134463 | 1 |
| UTERU2000238 | 0.001998401 | 186311 | 1 |
| UTERU2000243 | 0.001998401 | 172247 | 1 |
| UTERU2000260 | 0.008047982 | 41103 | 5 |
| UTERU2000263 | 0.327013605 | 137956 | 20 |
| UTERU2000300 | 0.033971044 | 127225 | 7 |
| UTERU2000329 | 0.005854917 | 239067 | 3 |
| UTERU2000332 | 0.035433658 | 135926 | 7 |
| UTERU2000338 | 0.050226674 | 122285 | 5 |
| UTERU2000349 | 0.001998401 | 147296 | 1 |
| UTERU2000377 | 0.001998401 | 84138 | 1 |
| UTERU2000393 | 0.001998401 | 231627 | 1 |
| UTERU2000418 | 0.005842485 | 254250 | 2 |
| UTERU2000424 | 0.197285034 | 27674 | 40 |
| UTERU2000465 | 0.001998401 | 223989 | 1 |
| UTERU2000485 | 0.013123109 | 170300 | 2 |
| UTERU2000517 | 0.001998401 | 50324 | 1 |
| UTERU2000524 | 0.001998401 | 200514 | 1 |
| UTERU2000537 | 0.032657348 | 131742 | 7 |
| UTERU2000539 | 0.001998401 | 223988 | 1 |
| UTERU2000541 | 0.026567306 | 142056 | 10 |
| UTERU2000542 | 0.018557296 | 73530 | 4 |
| UTERU2000546 | 0.001998401 | 103717 | 1 |
| UTERU2000550 | 0.001998401 | 215259 | 1 |
| UTERU2000569 | 0.001998401 | 189523 | 1 |
| UTERU2000607 | 0.001998401 | 266269 | 1 |
| UTERU2000629 | 0.001998401 | 121534 | 1 |
| UTERU2000649 | 0.001998401 | 268336 | 1 |
| UTERU2000663 | 0.001998401 | 263414 | 1 |
| UTERU2000696 | 0.058883424 | 144431 | 5 |
| UTERU2000794 | 0.003996803 | 199571 | 2 |
| UTERU2000830 | 0.001998401 | 263679 | 1 |
| UTERU2000844 | 0.001998401 | 80903 | 1 |
| UTERU2000922 | 0.001998401 | 75303 | 1 |
| UTERU2000925 | 0.016089745 | 73543 | 4 |
| UTERU2001024 | 0.001998401 | 3377 | 1 |
| UTERU2001110 | 0.008234759 | 96303 | 2 |
| UTERU2001176 | 0.001998401 | 276903 | 1 |
| UTERU2001281 | 0.001998401 | 131442 | 1 |
| UTERU2001389 | 0.001998401 | 99868 | 1 |
| UTERU2001409 | 0.001998401 | 261157 | 1 |
| UTERU2001412 | 0.001998401 | 98066 | 1 |
| UTERU2001504 | 0.001998401 | 223980 | 1 |
| UTERU2001607 | 0.113584757 | 111121 | 28 |
| UTERU2001658 | 0.018916318 | 48224 | 5 |
| UTERU2001747 | 0.001998401 | 153623 | 1 |
| UTERU2001876 | 0.001998401 | 166600 | 1 |
| UTERU2002001 | 0.00498036 | 55492 | 2 |
| UTERU2002011 | 0.001998401 | 96964 | 1 |
| UTERU2002176 | 0.001998401 | 215313 | 1 |
| UTERU2002198 | 0.00309368 | 134252 | 2 |
| UTERU2002294 | 0.092302324 | 17676 | 16 |
| UTERU2002332 | 0.001998401 | 226912 | 1 |
| UTERU2002410 | 0.285374963 | 70646 | 26 |
| UTERU2002473 | 0.001998401 | 280066 | 1 |
| UTERU2002547 | 0.001998401 | 221007 | 1 |
| UTERU2002662 | 0.094171422 | 108330 | 19 |
| UTERU2002693 | 0.001998401 | 56704 | 1 |
| UTERU2002733 | 0.001998401 | 278403 | 1 |
| UTERU2002736 | 0.001998401 | 58849 | 1 |
| UTERU2002737 | 0.001998401 | 272352 | 1 |
| UTERU2002826 | 0.045394069 | 203189 | 3 |
| UTERU2002841 | 0.00309368 | 219651 | 2 |
| UTERU2002964 | 0.007934176 | 169837 | 2 |
| UTERU2002993 | 0.00309368 | 141409 | 2 |
| UTERU2003035 | 0.001998401 | 40501 | 1 |
| UTERU2003057 | 0.06478483 | 125914 | 6 |
| UTERU2003126 | 0.001998401 | 185863 | 1 |
| UTERU2003135 | 0.001998401 | 60031 | 1 |
| UTERU2003321 | 0.004188958 | 142542 | 3 |
| UTERU2003399 | 0.001998401 | 253284 | 1 |
| UTERU2003411 | 0.554484589 | 103791 | 2 |
| UTERU2003456 | 0.015493053 | 41365 | 3 |
| UTERU2003577 | 0.001998401 | 13719 | 1 |
| UTERU2003704 | 0.009734723 | 123249 | 5 |
| UTERU2003926 | 0.00498036 | 124106 | 2 |
| UTERU2003973 | 0.001998401 | 202842 | 1 |
| UTERU2004015 | 0.001998401 | 271090 | 1 |
| UTERU2004037 | 0.076420451 | 150477 | 10 |
| UTERU2004039 | 0.001998401 | 161733 | 1 |
| UTERU2004061 | 0.001998401 | 150696 | 1 |
| UTERU2004073 | 0.008570071 | 136317 | 7 |
| UTERU2004163 | 0.001998401 | 74684 | 1 |
| UTERU2004197 | 0.083329792 | 51325 | 22 |
| UTERU2004299 | 0.001998401 | 159949 | 1 |
| UTERU2004461 | 0.006175336 | 50451 | 2 |
| UTERU2004520 | 0.005119208 | 140013 | 2 |
| UTERU2004564 | 0.001998401 | 23680 | 1 |
| UTERU2004664 | 0.098744332 | 66382 | 11 |
| UTERU2004688 | 0.150700082 | 166725 | 9 |
| UTERU2004698 | 0.003996803 | 193584 | 2 |
| UTERU2004807 | 0.166790628 | 104951 | 43 |
| UTERU2004861 | 0.007082235 | 183097 | 4 |
| UTERU2004929 | 0.014433758 | 100100 | 5 |
| UTERU2005004 | 0.004792712 | 43867 | 3 |
| UTERU2005050 | 0.001998401 | 179625 | 1 |
| UTERU2005069 | 0.00309368 | 143875 | 2 |
| UTERU2005074 | 0.014755553 | 148678 | 8 |
| UTERU2005179 | 0.001998401 | 109601 | 1 |
| UTERU2005292 | 0.053810396 | 138204 | 5 |
| UTERU2005346 | 0.001998401 | 83910 | 1 |
| UTERU2005354 | 0.076598619 | 6825 | 26 |
| UTERU2005446 | 0.00805277 | 154624 | 2 |
| UTERU2005449 | 0.010234672 | 98960 | 6 |
| UTERU2005450 | 0.001998401 | 237872 | 1 |
| UTERU2005533 | 0.001998401 | 222768 | 1 |
| UTERU2005548 | 0.048671412 | 104937 | 15 |
| UTERU2005593 | 0.149281574 | 83214 | 41 |
| UTERU2005601 | 0.065734037 | 115239 | 34 |
| UTERU2005621 | 0.074332556 | 29393 | 18 |
| UTERU2005664 | 0.014994543 | 187266 | 3 |
| UTERU2005822 | 0.001998401 | 8502 | 1 |
| UTERU2005903 | 0.001998401 | 104089 | 1 |
| UTERU2005905 | 0.001998401 | 118775 | 1 |
| UTERU2006103 | 0.001998401 | 95939 | 1 |
| UTERU2006115 | 0.021379086 | 143247 | 7 |
| UTERU2006137 | 0.021560217 | 102624 | 2 |
| UTERU2006182 | 0.003996803 | 147740 | 2 |
| UTERU2006400 | 0.013123109 | 138905 | 2 |
| UTERU2006412 | 0.001998401 | 118103 | 1 |
| UTERU2006429 | 0.001998401 | 64441 | 1 |
| UTERU2006486 | 0.003856516 | 174516 | 2 |
| UTERU2006524 | 0.003707861 | 151390 | 2 |
| UTERU2006547 | 0.008234759 | 79342 | 2 |
| UTERU2006568 | 0.001998401 | 161469 | 1 |
| UTERU2006593 | 0.113479648 | 132195 | 41 |
| UTERU2006643 | 0.019732194 | 59845 | 3 |
| UTERU2006651 | 0.001998401 | 271866 | 1 |
| UTERU2006705 | 0.001998401 | 221468 | 1 |
| UTERU2006899 | 0.001998401 | 148288 | 1 |
| UTERU2007004 | 0.008039967 | 241022 | 2 |
| UTERU2007075 | 0.001998401 | 135906 | 1 |
| UTERU2007081 | 0.011452878 | 167288 | 2 |
| UTERU2007128 | 0.001998401 | 51454 | 1 |
| UTERU2007253 | 0.016260617 | 112300 | 5 |
| UTERU2007267 | 0.001998401 | 153321 | 1 |
| UTERU2007444 | 0.00309368 | 158561 | 2 |
| UTERU2007499 | 0.001998401 | 108273 | 1 |
| UTERU2007520 | 0.024981157 | 167548 | 8 |
| UTERU2007639 | 0.001998401 | 193614 | 1 |
| UTERU2007724 | 0.025503558 | 15798 | 4 |
| UTERU2007924 | 0.062648093 | 140717 | 6 |
| UTERU2007942 | 0.001998401 | 104167 | 1 |
| UTERU2008018 | 0.008039967 | 204171 | 2 |
| UTERU2008019 | 0.001998401 | 213230 | 1 |
| UTERU2008027 | 0.043804421 | 42531 | 2 |
| UTERU2008040 | 0.014236966 | 215835 | 6 |
| UTERU2008077 | 0.001998401 | 218802 | 1 |
| UTERU2008085 | 1.855334107 | 34899 | 204 |
| UTERU2008130 | 0.003707861 | 204355 | 2 |
| UTERU2008302 | 0.125417087 | 97790 | 22 |
| UTERU2008347 | 0.001998401 | 269130 | 1 |
| UTERU2008426 | 0.007610199 | 132718 | 4 |
| UTERU2008516 | 0.001998401 | 82007 | 1 |
| UTERU2008561 | 0.001998401 | 218717 | 1 |
| UTERU2008653 | 0.004079913 | 134537 | 2 |
| UTERU2008705 | 0.001998401 | 224032 | 1 |
| UTERU2008707 | 0.001998401 | 5280 | 1 |
| UTERU2008747 | 0.018958528 | 207523 | 6 |
| UTERU2008785 | 0.001998401 | 115157 | 1 |
| UTERU2008845 | 0.001998401 | 19142 | 1 |
| UTERU2008901 | 0.001998401 | 14967 | 1 |
| UTERU2008930 | 0.001998401 | 272015 | 1 |
| UTERU2008938 | 0.068082902 | 98528 | 25 |
| UTERU2008939 | 0.011280225 | 94460 | 5 |
| UTERU2008962 | 0.00309368 | 173614 | 2 |
| UTERU2009094 | 0.001998401 | 162580 | 1 |
| UTERU2009120 | 0.001998401 | 260323 | 1 |
| UTERU2009131 | 0.001998401 | 133684 | 1 |
| UTERU2009147 | 0.001998401 | 125716 | 1 |
| UTERU2009206 | 0.001998401 | 263287 | 1 |
| UTERU2009283 | 0.001998401 | 228785 | 1 |
| UTERU2009335 | 0.055244213 | 147531 | 13 |
| UTERU2009414 | 0.001998401 | 5143 | 1 |
| UTERU2009435 | 0.031333709 | 101088 | 17 |
| UTERU2009483 | 0.001998401 | 183652 | 1 |
| UTERU2009510 | 0.011148266 | 142395 | 3 |
| UTERU2009538 | 0.004913258 | 145821 | 2 |
| UTERU2009540 | 0.001998401 | 32277 | 1 |
| UTERU2009776 | 0.001998401 | 149019 | 1 |
| UTERU2009904 | 0.001998401 | 189763 | 1 |
| UTERU2009951 | 0.001998401 | 123588 | 1 |
| UTERU2009972 | 0.047295514 | 146138 | 21 |
| UTERU2010115 | 0.001998401 | 157626 | 1 |
| UTERU2010124 | 0.001998401 | 152531 | 1 |
| UTERU2010164 | 0.001998401 | 6430 | 1 |
| UTERU2010226 | 0.021240948 | 100742 | 8 |
| UTERU2010231 | 0.001998401 | 137754 | 1 |
| UTERU2010304 | 0.001998401 | 6762 | 1 |
| UTERU2010320 | 0.007934176 | 139155 | 2 |
| UTERU2010417 | 0.531462578 | 36734 | 68 |
| UTERU2010431 | 0.001998401 | 70488 | 1 |
| UTERU2010525 | 0.014220352 | 215460 | 2 |
| UTERU2010651 | 0.001998401 | 81252 | 1 |
| UTERU2010724 | 0.001998401 | 70551 | 1 |
| UTERU2010747 | 0.003187646 | 148141 | 2 |
| UTERU2011195 | 0.001998401 | 107132 | 1 |
| UTERU2011199 | 0.001998401 | 182511 | 1 |
| UTERU2011220 | 0.085610789 | 107268 | 7 |
| UTERU2011261 | 0.001998401 | 97533 | 1 |
| UTERU2011287 | 0.005105017 | 66231 | 3 |
| UTERU2011410 | 0.001998401 | 92897 | 1 |
| UTERU2011574 | 0.001998401 | 23115 | 1 |
| UTERU2011621 | 0.016874324 | 40087 | 11 |
| UTERU2011657 | 0.001998401 | 79864 | 1 |
| UTERU2011741 | 0.014222755 | 107734 | 3 |
| UTERU2011806 | 0.01203677 | 80671 | 4 |
| UTERU2011811 | 0.001998401 | 164668 | 1 |
| UTERU2011897 | 0.003996803 | 134356 | 2 |
| UTERU2011906 | 0.024570185 | 47614 | 12 |
| UTERU2011962 | 0.001998401 | 213368 | 1 |
| UTERU2011968 | 0.001998401 | 60456 | 1 |
| UTERU2012031 | 0.003707861 | 88460 | 2 |
| UTERU2012101 | 0.001998401 | 72326 | 1 |
| UTERU2012114 | 0.001998401 | 90586 | 1 |
| UTERU2012230 | 0.095212005 | 86533 | 46 |
| UTERU2012252 | 0.007803579 | 128908 | 2 |
| UTERU2012286 | 0.066135103 | 52303 | 17 |
| UTERU2012333 | 0.001998401 | 8191 | 1 |
| UTERU2012407 | 0.006807484 | 138956 | 3 |
| UTERU2012526 | 0.018145421 | 198226 | 4 |
| UTERU2012581 | 0.001998401 | 247010 | 1 |
| UTERU2012610 | 0.001998401 | 2829 | 1 |
| UTERU2012615 | 0.001998401 | 146946 | 1 |
| UTERU2012688 | 0.003875243 | 129602 | 2 |
| UTERU2012703 | 0.008143927 | 85083 | 2 |
| UTERU2012715 | 0.005842485 | 41038 | 2 |
| UTERU2012741 | 0.009308168 | 94373 | 3 |
| UTERU2012767 | 0.031464993 | 141357 | 7 |
| UTERU2012786 | 0.001998401 | 235009 | 1 |
| UTERU2012856 | 0.047493293 | 161910 | 9 |
| UTERU2012890 | 0.001998401 | 163981 | 1 |
| UTERU2012938 | 0.004079913 | 70568 | 2 |
| UTERU2012976 | 0.001998401 | 135818 | 1 |
| UTERU2013048 | 0.001998401 | 4112 | 1 |
| UTERU2013078 | 0.012216902 | 11354 | 3 |
| UTERU2013231 | 0.001998401 | 133522 | 1 |
| UTERU2013262 | 0.009130364 | 135895 | 4 |
| UTERU2013280 | 0.001998401 | 82383 | 1 |
| UTERU2013322 | 0.001998401 | 38383 | 1 |
| UTERU2013483 | 0.001998401 | 149577 | 1 |
| UTERU2013491 | 0.014347129 | 55397 | 2 |
| UTERU2013502 | 0.009938926 | 147619 | 3 |
| UTERU2013586 | 0.025430779 | 62908 | 8 |
| UTERU2013926 | 0.001998401 | 29834 | 1 |
| UTERU2013976 | 0.001998401 | 78667 | 1 |
| UTERU2014001 | 0.001998401 | 160270 | 1 |
| UTERU2014024 | 0.012929125 | 31810 | 6 |
| UTERU2014167 | 0.183838856 | 155857 | 10 |
| UTERU2014223 | 0.001998401 | 82195 | 1 |
| UTERU2014398 | 0.001998401 | 239853 | 1 |
| UTERU2014464 | 0.012411694 | 56397 | 3 |
| UTERU2014548 | 0.005093897 | 6955 | 2 |
| UTERU2014601 | 0.024193577 | 197076 | 7 |
| UTERU2014631 | 0.001998401 | 232031 | 1 |
| UTERU2014668 | 0.005093897 | 190549 | 2 |
| UTERU2014678 | 0.004079913 | 85380 | 2 |
| UTERU2014728 | 0.003996803 | 168211 | 2 |
| UTERU2014898 | 0.11011669 | 28697 | 11 |
| UTERU2014998 | 0.001998401 | 239871 | 1 |
| UTERU2015062 | 0.011707139 | 171848 | 2 |
| UTERU2015087 | 0.001998401 | 169383 | 1 |
| UTERU2015108 | 0.026553347 | 132270 | 5 |
| UTERU2015190 | 0.004913258 | 206797 | 2 |
| UTERU2015198 | 0.001998401 | 116811 | 1 |
| UTERU2015202 | 0.001998401 | 146004 | 1 |
| UTERU2015405 | 0.001998401 | 109439 | 1 |
| UTERU2015640 | 0.001998401 | 16690 | 1 |
| UTERU2015653 | 0.287208797 | 97047 | 18 |
| UTERU2015830 | 0.003395557 | 72610 | 2 |
| UTERU2015880 | 0.001998401 | 31843 | 1 |
| UTERU2016147 | 0.001998401 | 81082 | 1 |
| UTERU2016157 | 0.001998401 | 94377 | 1 |
| UTERU2016426 | 0.013184237 | 162313 | 5 |
| UTERU2016464 | 0.001998401 | 269024 | 1 |
| UTERU2016669 | 0.020292735 | 215098 | 3 |
| UTERU2016757 | 0.003673948 | 32349 | 2 |
| UTERU2016761 | 0.001998401 | 171385 | 1 |
| UTERU2016799 | 0.057624333 | 101982 | 21 |
| UTERU2016822 | 0.083362439 | 84687 | 35 |
| UTERU2016896 | 0.001998401 | 162391 | 1 |
| UTERU2016902 | 0.001998401 | 187357 | 1 |
| UTERU2016979 | 0.01619994 | 8837 | 3 |
| UTERU2016981 | 0.001998401 | 47982 | 1 |
| UTERU2017123 | 0.001998401 | 143755 | 1 |
| UTERU2017303 | 0.001998401 | 55874 | 1 |
| UTERU2017421 | 0.001998401 | 197217 | 1 |
| UTERU2017492 | 0.038454836 | 52564 | 2 |
| UTERU2017613 | 0.016294395 | 39175 | 4 |
| UTERU2017623 | 0.00309368 | 122667 | 2 |
| UTERU2017632 | 0.00309368 | 58403 | 2 |
| UTERU2017715 | 0.007152616 | 157433 | 4 |
| UTERU2017761 | 0.003187646 | 141890 | 2 |
| UTERU2017762 | 0.005119208 | 73416 | 2 |
| UTERU2017810 | 0.001998401 | 85672 | 1 |
| UTERU2017988 | 0.001998401 | 165712 | 1 |
| UTERU2018127 | 0.001998401 | 236281 | 1 |
| UTERU2018180 | 0.001998401 | 78505 | 1 |
| UTERU2018200 | 0.006903875 | 149501 | 4 |
| UTERU2018333 | 0.001998401 | 215990 | 1 |
| UTERU2018364 | 0.003707861 | 75498 | 2 |
| UTERU2018514 | 0.001998401 | 190425 | 1 |
| UTERU2018522 | 0.001998401 | 114937 | 1 |
| UTERU2018523 | 0.00498036 | 86740 | 2 |
| UTERU2018544 | 0.001998401 | 90477 | 1 |
| UTERU2018566 | 0.009400755 | 122570 | 3 |
| UTERU2018609 | 0.003996803 | 236279 | 2 |
| UTERU2018674 | 0.001998401 | 188491 | 1 |
| UTERU2018712 | 0.003686678 | 43628 | 2 |
| UTERU2018784 | 0.001998401 | 139259 | 1 |
| UTERU2018789 | 0.003996803 | 210220 | 2 |
| UTERU2018811 | 0.001998401 | 102308 | 1 |
| UTERU2018867 | 0.001998401 | 151070 | 1 |
| UTERU2018881 | 0.001998401 | 268110 | 1 |
| UTERU2018884 | 0.108156749 | 127476 | 11 |
| UTERU2018955 | 0.001998401 | 17650 | 1 |
| UTERU2019005 | 0.02594312 | 20990 | 9 |
| UTERU2019038 | 0.001998401 | 127723 | 1 |
| UTERU2019096 | 0.072738751 | 76116 | 30 |
| UTERU2019163 | 0.001998401 | 223309 | 1 |
| UTERU2019257 | 0.006978762 | 47220 | 3 |
| UTERU2019453 | 0.001998401 | 165048 | 1 |
| UTERU2019491 | 0.001998401 | 234059 | 1 |
| UTERU2019534 | 0.001998401 | 151888 | 1 |
| UTERU2019681 | 0.001998401 | 199570 | 1 |
| UTERU2019706 | 0.003673948 | 17526 | 2 |
| UTERU2019710 | 0.001998401 | 155384 | 1 |
| UTERU2019940 | 0.003856516 | 136041 | 2 |
| UTERU2019959 | 0.001998401 | 143030 | 1 |
| UTERU2019964 | 0.001998401 | 278323 | 1 |
| UTERU2020226 | 0.005092081 | 175149 | 3 |
| UTERU2020242 | 0.001998401 | 212049 | 1 |
| UTERU2020292 | 0.001998401 | 143848 | 1 |
| UTERU2020351 | 0.001998401 | 160825 | 1 |
| UTERU2020491 | 0.001998401 | 119886 | 1 |
| UTERU2020718 | 0.001998401 | 176193 | 1 |
| UTERU2021163 | 0.003707861 | 115116 | 2 |
| UTERU2021380 | 0.001998401 | 129274 | 1 |
| UTERU2021649 | 0.001998401 | 127284 | 1 |
| UTERU2021820 | 0.006721307 | 127280 | 4 |
| UTERU2021917 | 0.078238458 | 113092 | 11 |
| UTERU2022020 | 0.001998401 | 182424 | 1 |
| UTERU2022773 | 0.001998401 | 196087 | 1 |
| UTERU2022955 | 0.001998401 | 283796 | 1 |
| UTERU2022981 | 0.001998401 | 149897 | 1 |
| UTERU2023039 | 0.003673948 | 105684 | 2 |
| UTERU2023045 | 0.018513218 | 100379 | 3 |
| UTERU2023175 | 0.112542976 | 125169 | 22 |
| UTERU2023262 | 0.013285083 | 186947 | 2 |
| UTERU2023550 | 0.020007096 | 128119 | 3 |
| UTERU2023651 | 0.006669554 | 129059 | 4 |
| UTERU2023687 | 0.001998401 | 127449 | 1 |
| UTERU2023712 | 0.001998401 | 127448 | 1 |
| UTERU2023941 | 0.001998401 | 278688 | 1 |
| UTERU2024002 | 0.001998401 | 145867 | 1 |
| UTERU2024042 | 0.001998401 | 131164 | 1 |
| UTERU2024141 | 0.001998401 | 131145 | 1 |
| UTERU2024481 | 0.001998401 | 120326 | 1 |
| UTERU2024656 | 0.003875243 | 131565 | 2 |
| UTERU2024758 | 0.001998401 | 141255 | 1 |
| UTERU2024820 | 0.001998401 | 135201 | 1 |
| UTERU2024881 | 0.001998401 | 268827 | 1 |
| UTERU2024969 | 0.052177104 | 10445 | 14 |
| UTERU2025025 | 0.001998401 | 145590 | 1 |
| UTERU2025041 | 0.001998401 | 161212 | 1 |
| UTERU2025301 | 0.038548325 | 20649 | 3 |
| UTERU2025366 | 0.001998401 | 186379 | 1 |
| UTERU2025415 | 0.001998401 | 158568 | 1 |
| UTERU2025579 | 0.001998401 | 158509 | 1 |
| UTERU2025645 | 0.001998401 | 144423 | 1 |
| UTERU2025891 | 0.001998401 | 141403 | 1 |
| UTERU2026025 | 0.001998401 | 3373 | 1 |
| UTERU2026090 | 0.001998401 | 36823 | 1 |
| UTERU2026142 | 0.001998401 | 2437 | 1 |
| UTERU2026203 | 0.003673948 | 48574 | 2 |
| UTERU2026775 | 0.001998401 | 184618 | 1 |
| UTERU2027023 | 0.001998401 | 253781 | 1 |
| UTERU2027369 | 0.005093897 | 146016 | 2 |
| UTERU2027591 | 0.001998401 | 134957 | 1 |
| UTERU2027616 | 0.001998401 | 153533 | 1 |
| UTERU2027941 | 0.001998401 | 100554 | 1 |
| UTERU2028377 | 0.001998401 | 196538 | 1 |
| UTERU2028734 | 0.007632302 | 166780 | 4 |
| UTERU2029503 | 0.001998401 | 175059 | 1 |
| UTERU2029660 | 0.001998401 | 175054 | 1 |
| UTERU2029742 | 0.001998401 | 147091 | 1 |
| UTERU2029953 | 0.001998401 | 234419 | 1 |
| UTERU2030213 | 0.006801541 | 133222 | 4 |
| UTERU2030270 | 0.001998401 | 219426 | 1 |
| UTERU2030280 | 0.003856516 | 144611 | 2 |
| UTERU2031060 | 0.001998401 | 91885 | 1 |
| UTERU2031084 | 0.007707569 | 165571 | 4 |
| UTERU2031268 | 0.001998401 | 134279 | 1 |
| UTERU2031295 | 0.001998401 | 254920 | 1 |
| UTERU2031521 | 0.001998401 | 234361 | 1 |
| UTERU2031611 | 0.001998401 | 234359 | 1 |
| UTERU2031703 | 0.005995204 | 210851 | 3 |
| UTERU2031834 | 0.001998401 | 193763 | 1 |
| UTERU2031851 | 0.001998401 | 196139 | 1 |
| UTERU2032075 | 0.001998401 | 281401 | 1 |
| UTERU2032220 | 0.001998401 | 214186 | 1 |
| UTERU2032279 | 0.001998401 | 242363 | 1 |
| UTERU2032726 | 0.005995204 | 2436 | 3 |
| UTERU2033172 | 0.001998401 | 261883 | 1 |
| UTERU2033375 | 0.144245912 | 241080 | 2 |
| UTERU2033382 | 0.001998401 | 248120 | 1 |
| UTERU2033420 | 0.001998401 | 143627 | 1 |
| UTERU2033530 | 0.001998401 | 197493 | 1 |
| UTERU2033577 | 0.001998401 | 96343 | 1 |
| UTERU2034053 | 0.001998401 | 254115 | 1 |
| UTERU2034147 | 0.003395557 | 254120 | 2 |
| UTERU2034695 | 0.003996803 | 237328 | 2 |
| UTERU2035114 | 0.003875243 | 203789 | 2 |
| UTERU2035187 | 0.001998401 | 257575 | 1 |
| UTERU2035231 | 0.001998401 | 33924 | 1 |
| UTERU2035306 | 0.001998401 | 211158 | 1 |
| UTERU2035323 | 0.001998401 | 193802 | 1 |
| UTERU2035328 | 0.001998401 | 119566 | 1 |
| UTERU2035331 | 0.001998401 | 69592 | 1 |
| UTERU2035452 | 0.001998401 | 280898 | 1 |
| UTERU2035469 | 0.001998401 | 158462 | 1 |
| UTERU2035503 | 0.001998401 | 60506 | 1 |
| UTERU2035745 | 0.001998401 | 277244 | 1 |
| UTERU2035908 | 0.001998401 | 258967 | 1 |
| UTERU2035926 | 0.001998401 | 282915 | 1 |
| UTERU2035978 | 0.001998401 | 251262 | 1 |
| UTERU2036089 | 0.001998401 | 200000 | 1 |
| UTERU2036347 | 0.005272398 | 223716 | 3 |
| UTERU2036507 | 0.001998401 | 143624 | 1 |
| UTERU2036512 | 0.001998401 | 269886 | 1 |
| UTERU2036530 | 0.001998401 | 223437 | 1 |
| UTERU2036623 | 0.001998401 | 188337 | 1 |
| UTERU2036690 | 0.001998401 | 250009 | 1 |
| UTERU2037224 | 0.001998401 | 182026 | 1 |
| UTERU2037361 | 0.001998401 | 263630 | 1 |
| UTERU2037423 | 0.001998401 | 282513 | 1 |
| UTERU2037577 | 0.001998401 | 277568 | 1 |
| UTERU2037674 | 0.001998401 | 260428 | 1 |
| UTERU2037791 | 0.001998401 | 200187 | 1 |
| UTERU2037843 | 0.001998401 | 269343 | 1 |
| UTERU2038171 | 0.001998401 | 232075 | 1 |
| UTERU2038251 | 0.001998401 | 213908 | 1 |
| UTERU3000226 | 0.003187646 | 176587 | 2 |
| UTERU3000298 | 0.036215441 | 100290 | 21 |
| UTERU3000402 | 0.001998401 | 190735 | 1 |
| UTERU3000645 | 0.005752085 | 190615 | 3 |
| UTERU3000665 | 0.001998401 | 257544 | 1 |
| UTERU3000670 | 0.007184448 | 73796 | 4 |
| UTERU3000727 | 0.007421772 | 95143 | 4 |
| UTERU3000738 | 0.004792712 | 162477 | 3 |
| UTERU3000828 | 0.001998401 | 52798 | 1 |
| UTERU3000844 | 0.00309368 | 40373 | 2 |
| UTERU3000899 | 0.005938028 | 2846 | 3 |
| UTERU3000959 | 0.001998401 | 284948 | 1 |
| UTERU3001029 | 0.001998401 | 102994 | 1 |
| UTERU3001053 | 0.001998401 | 175478 | 1 |
| UTERU3001059 | 0.001998401 | 280380 | 1 |
| UTERU3001158 | 0.00504576 | 197739 | 3 |
| UTERU3001240 | 0.001998401 | 177141 | 1 |
| UTERU3001394 | 0.001998401 | 254737 | 1 |
| UTERU3001542 | 0.003395557 | 19767 | 2 |
| UTERU3001558 | 0.001998401 | 236900 | 1 |
| UTERU3001571 | 0.003856516 | 40689 | 2 |
| UTERU3001572 | 0.091784057 | 141213 | 31 |
| UTERU3001585 | 0.001998401 | 88250 | 1 |
| UTERU3001632 | 0.001998401 | 84899 | 1 |
| UTERU3001652 | 0.001998401 | 60661 | 1 |
| UTERU3001766 | 0.003686678 | 55680 | 2 |
| UTERU3001946 | 0.005752085 | 212445 | 3 |
| UTERU3001988 | 0.001998401 | 128229 | 1 |
| UTERU3002209 | 0.001998401 | 257899 | 1 |
| UTERU3002218 | 0.003875243 | 37749 | 2 |
| UTERU3002383 | 0.001998401 | 230613 | 1 |
| UTERU3002600 | 0.001998401 | 151716 | 1 |
| UTERU3002620 | 0.001998401 | 279430 | 1 |
| UTERU3002667 | 0.001998401 | 106655 | 1 |
| UTERU3002701 | 0.001998401 | 226397 | 1 |
| UTERU3002731 | 0.001998401 | 231875 | 1 |
| UTERU3002768 | 0.001998401 | 237130 | 1 |
| UTERU3002786 | 0.003875243 | 268502 | 2 |
| UTERU3002993 | 0.001998401 | 63801 | 1 |
| UTERU3003116 | 0.001998401 | 284030 | 1 |
| UTERU3003135 | 0.005105017 | 165006 | 3 |
| UTERU3003178 | 0.001998401 | 255016 | 1 |
| UTERU3003465 | 0.001998401 | 111353 | 1 |
| UTERU3003495 | 0.001998401 | 215937 | 1 |
| UTERU3003523 | 0.003996803 | 139194 | 2 |
| UTERU3003660 | 0.02275735 | 114890 | 12 |
| UTERU3003774 | 0.001998401 | 215611 | 1 |
| UTERU3003776 | 0.001998401 | 210170 | 1 |
| UTERU3004384 | 0.003707861 | 228918 | 2 |
| UTERU3004418 | 0.00575546 | 180837 | 3 |
| UTERU3004477 | 0.006978762 | 14725 | 3 |
| UTERU3004523 | 0.001998401 | 260355 | 1 |
| UTERU3004616 | 0.001998401 | 122663 | 1 |
| UTERU3004635 | 0.001998401 | 106981 | 1 |
| UTERU3004709 | 0.003856516 | 261357 | 2 |
| UTERU3004938 | 0.001998401 | 237143 | 1 |
| UTERU3004992 | 0.001998401 | 88667 | 1 |
| UTERU3005049 | 0.003395557 | 177034 | 2 |
| UTERU3005159 | 0.00480314 | 123286 | 3 |
| UTERU3005205 | 0.001998401 | 259676 | 1 |
| UTERU3005230 | 0.006861594 | 232433 | 4 |
| UTERU3005264 | 0.001998401 | 128421 | 1 |
| UTERU3005422 | 0.007899328 | 132008 | 5 |
| UTERU3005460 | 0.003187646 | 130111 | 2 |
| UTERU3005536 | 0.003187646 | 172711 | 2 |
| UTERU3005585 | 0.001998401 | 178505 | 1 |
| UTERU3005767 | 0.140779323 | 2430 | 18 |
| UTERU3005907 | 0.025604786 | 86906 | 10 |
| UTERU3005970 | 0.003187646 | 234920 | 2 |
| UTERU3006008 | 0.001998401 | 253873 | 1 |
| UTERU3006228 | 0.001998401 | 197993 | 1 |
| UTERU3006308 | 0.003187646 | 258132 | 2 |
| UTERU3006538 | 0.001998401 | 269849 | 1 |
| UTERU3006640 | 0.001998401 | 270023 | 1 |
| UTERU3006687 | 0.001998401 | 253166 | 1 |
| UTERU3006720 | 0.001998401 | 88015 | 1 |
| UTERU3006798 | 0.001998401 | 259657 | 1 |
| UTERU3006884 | 0.001998401 | 257570 | 1 |
| UTERU3007021 | 0.005752085 | 224634 | 3 |
| UTERU3007104 | 0.003875243 | 84614 | 2 |
| UTERU3007108 | 0.001998401 | 138678 | 1 |
| UTERU3007134 | 0.001998401 | 168512 | 1 |
| UTERU3007419 | 0.003187646 | 23425 | 2 |
| UTERU3007640 | 0.003673948 | 78942 | 2 |
| UTERU3007731 | 0.001998401 | 5355 | 1 |
| UTERU3007913 | 0.00918285 | 83826 | 5 |
| UTERU3008280 | 0.005383408 | 241632 | 3 |
| UTERU3008463 | 0.001998401 | 245478 | 1 |
| UTERU3008660 | 0.001998401 | 241635 | 1 |
| UTERU3008671 | 0.001998401 | 222375 | 1 |
| UTERU3008722 | 0.001998401 | 199036 | 1 |
| UTERU3008917 | 0.001998401 | 257765 | 1 |
| UTERU3009259 | 0.003707861 | 253574 | 2 |
| UTERU3009490 | 0.001998401 | 251113 | 1 |
| UTERU3009517 | 0.003686678 | 247136 | 2 |
| UTERU3009690 | 0.001998401 | 242362 | 1 |
| UTERU3009775 | 0.001998401 | 226679 | 1 |
| UTERU3009871 | 0.009249512 | 116483 | 6 |
| UTERU3009979 | 0.001998401 | 237177 | 1 |
| UTERU3009987 | 0.001998401 | 250826 | 1 |
| UTERU3010029 | 0.001998401 | 250905 | 1 |
| UTERU3010409 | 0.048410381 | 88653 | 28 |
| UTERU3010526 | 0.001998401 | 274951 | 1 |
| UTERU3010604 | 0.00498036 | 82604 | 2 |
| UTERU3010892 | 0.001998401 | 248839 | 1 |
| UTERU3010919 | 0.001998401 | 119649 | 1 |
| UTERU3011063 | 0.001998401 | 51546 | 1 |
| UTERU3011092 | 0.001998401 | 275021 | 1 |
| UTERU3011273 | 0.001998401 | 122670 | 1 |
| UTERU3011398 | 0.001998401 | 251326 | 1 |
| UTERU3011558 | 0.001998401 | 248534 | 1 |
| UTERU3011579 | 0.005752085 | 36391 | 3 |
| UTERU3011795 | 0.001998401 | 248454 | 1 |
| UTERU3011837 | 0.001998401 | 250782 | 1 |
| UTERU3012293 | 0.001998401 | 198501 | 1 |
| UTERU3012414 | 0.003856516 | 94116 | 2 |
| UTERU3012476 | 0.001998401 | 231872 | 1 |
| UTERU3012599 | 0.001998401 | 242320 | 1 |
| UTERU3012999 | 0.001998401 | 117040 | 1 |
| UTERU3013167 | 0.003673948 | 239030 | 2 |
| UTERU3013302 | 0.003395557 | 122853 | 2 |
| UTERU3013781 | 0.001998401 | 161399 | 1 |
| UTERU3014274 | 0.001998401 | 212983 | 1 |
| UTERU3014446 | 0.001998401 | 274560 | 1 |
| UTERU3014611 | 0.003996803 | 276399 | 2 |
| UTERU3014647 | 0.001998401 | 253717 | 1 |
| UTERU3014791 | 0.001998401 | 258461 | 1 |
| UTERU3014906 | 0.001998401 | 285379 | 1 |
| UTERU3015011 | 0.023741785 | 136725 | 6 |
| UTERU3015069 | 0.026954175 | 166297 | 5 |
| UTERU3015086 | 0.001998401 | 127588 | 1 |
| UTERU3015299 | 0.001998401 | 285157 | 1 |
| UTERU3015412 | 0.001998401 | 98550 | 1 |
| UTERU3015500 | 0.001998401 | 231943 | 1 |
| UTERU3015647 | 0.001998401 | 120558 | 1 |
| UTERU3015844 | 0.001998401 | 245172 | 1 |
| UTERU3016070 | 0.001998401 | 138887 | 1 |
| UTERU3016273 | 0.003707861 | 136846 | 2 |
| UTERU3016274 | 0.001998401 | 249281 | 1 |
| UTERU3016308 | 0.001998401 | 53805 | 1 |
| UTERU3016789 | 0.001998401 | 274239 | 1 |
| UTERU3017176 | 0.001998401 | 197571 | 1 |
| UTERU3017441 | 0.001998401 | 34936 | 1 |
| UTERU3017626 | 0.001998401 | 231963 | 1 |
| UTERU3017995 | 0.015320462 | 179323 | 3 |
| UTERU3018081 | 0.004970521 | 182565 | 3 |
| UTERU3018154 | 0.001998401 | 235077 | 1 |
| UTERU3018172 | 0.005253671 | 89443 | 3 |
| UTERU3018255 | 0.001998401 | 162206 | 1 |
| UTERU3018616 | 0.007940525 | 199429 | 2 |
| UTERU3018711 | 0.013867249 | 106656 | 7 |
| UTERU3019078 | 0.001998401 | 162073 | 1 |

This analysis yielded information on the expression frequencies of the 17,176 genes that include the cDNAs of the present invention, in all tissues and cells used as sources of the respective cDNA libraries and described in Example 1.

Of the 17,176 genes that include the cDNAs of the present invention, genes whose expression levels were high enough to detect in each of the 3NB69, ACTVT, ADIPS, ADRGL, AHMSC, ASTRO, and BEAST libraries were analyzed for expression frequency. The analysis results are described below. Each is a relative value, where the expression level over all tissues is taken as 100. The higher this value, the greater the level of expression.

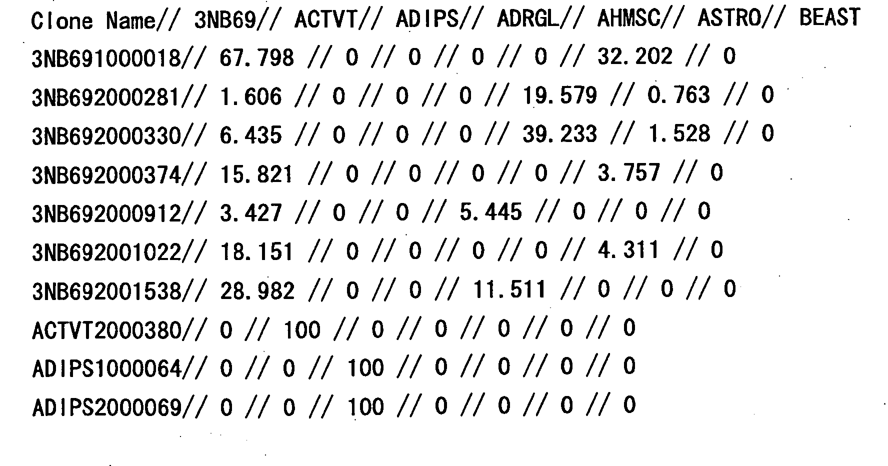

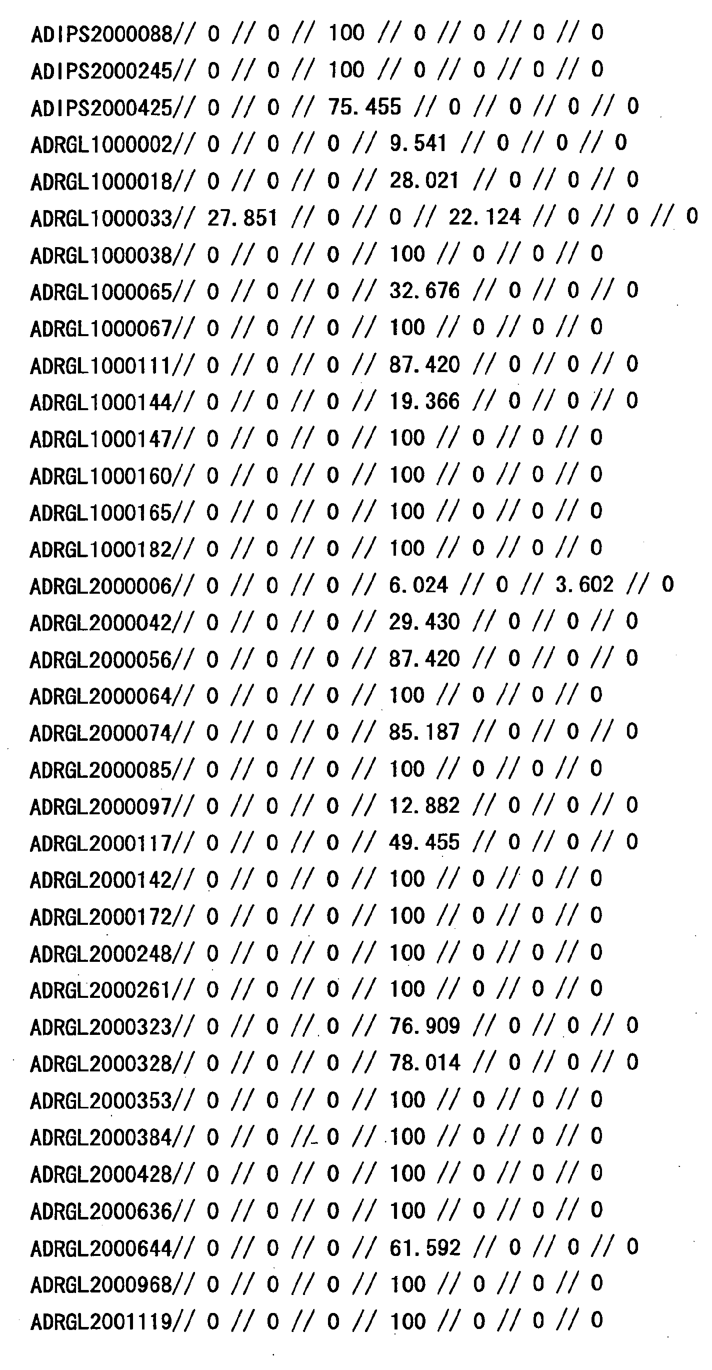

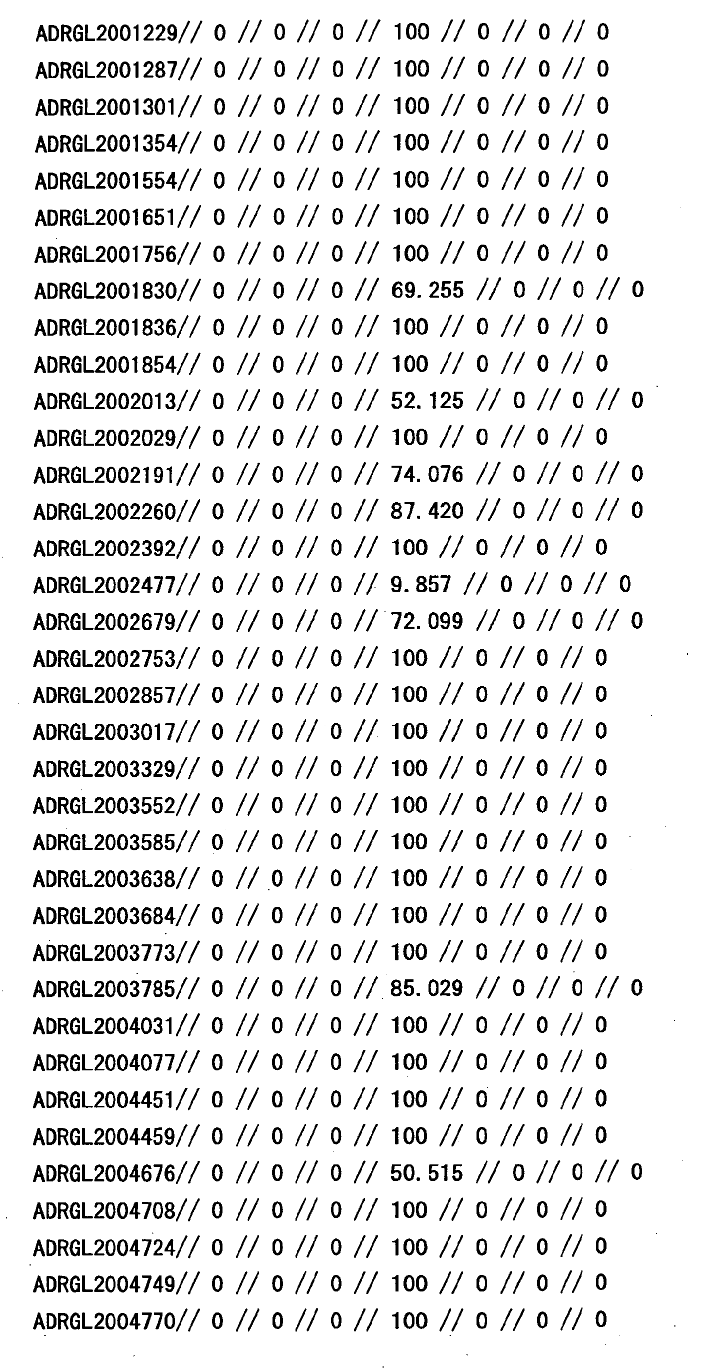

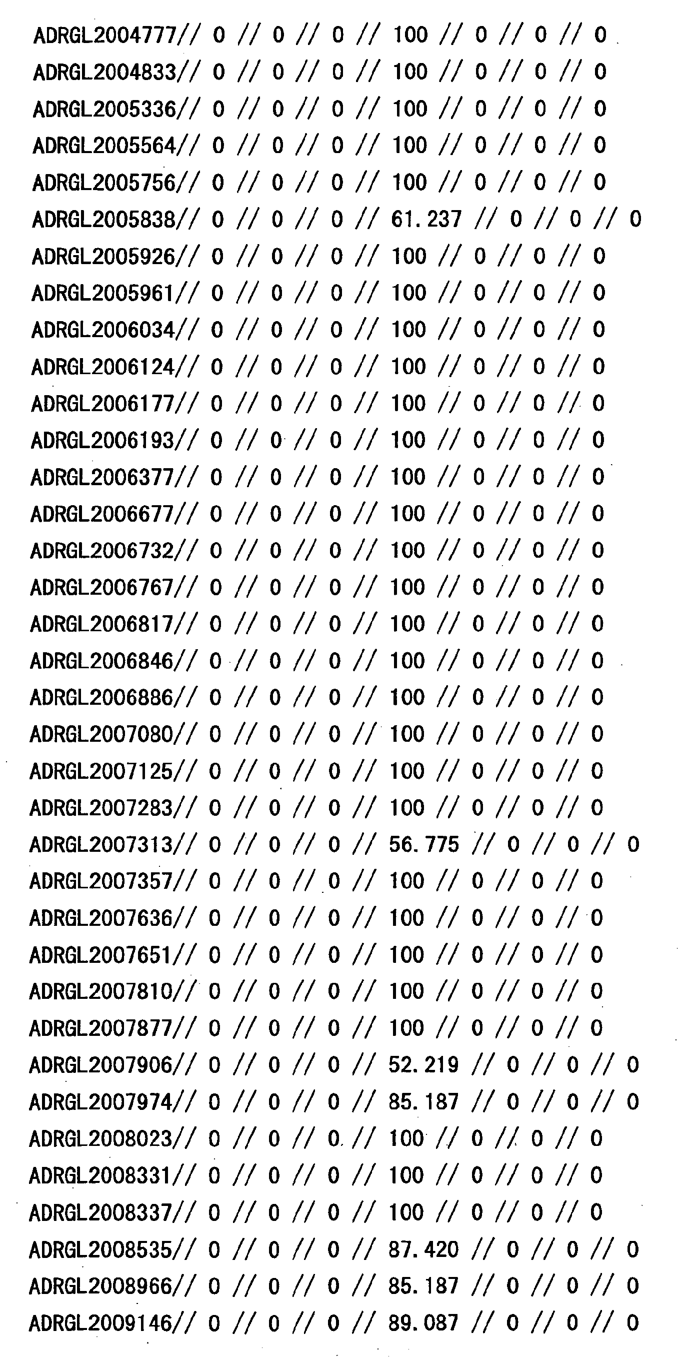

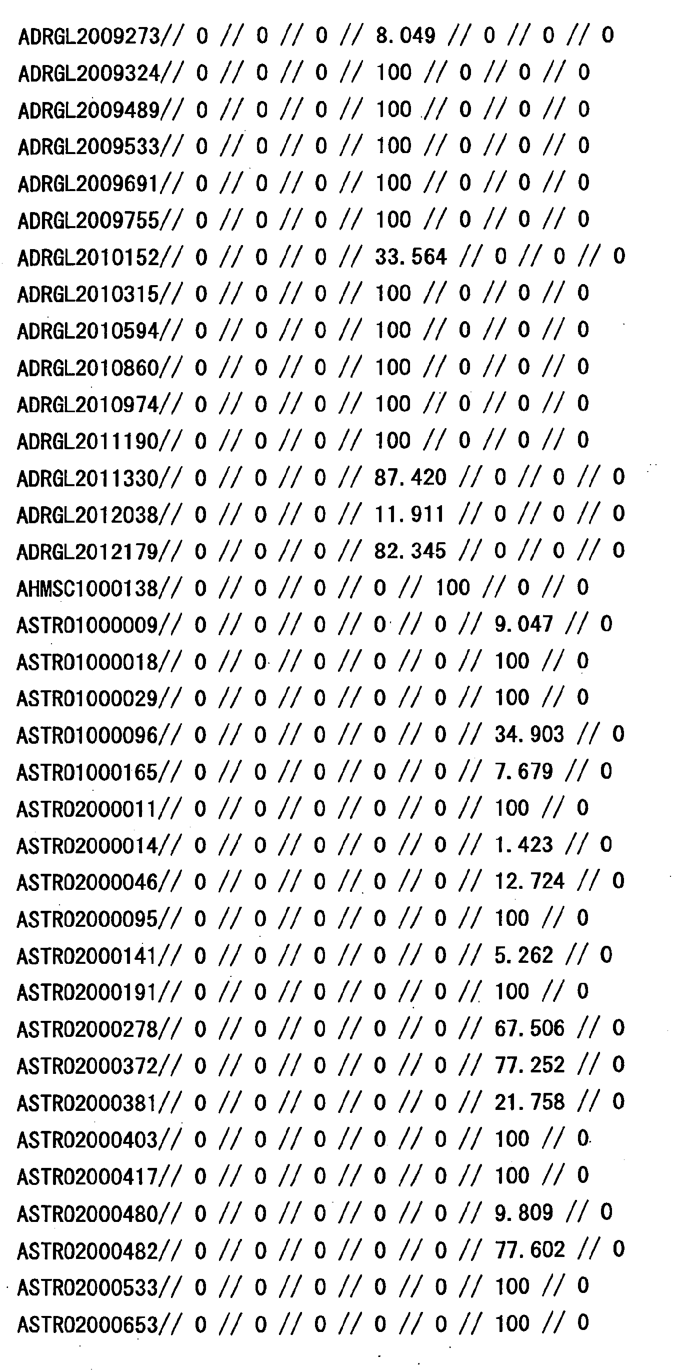

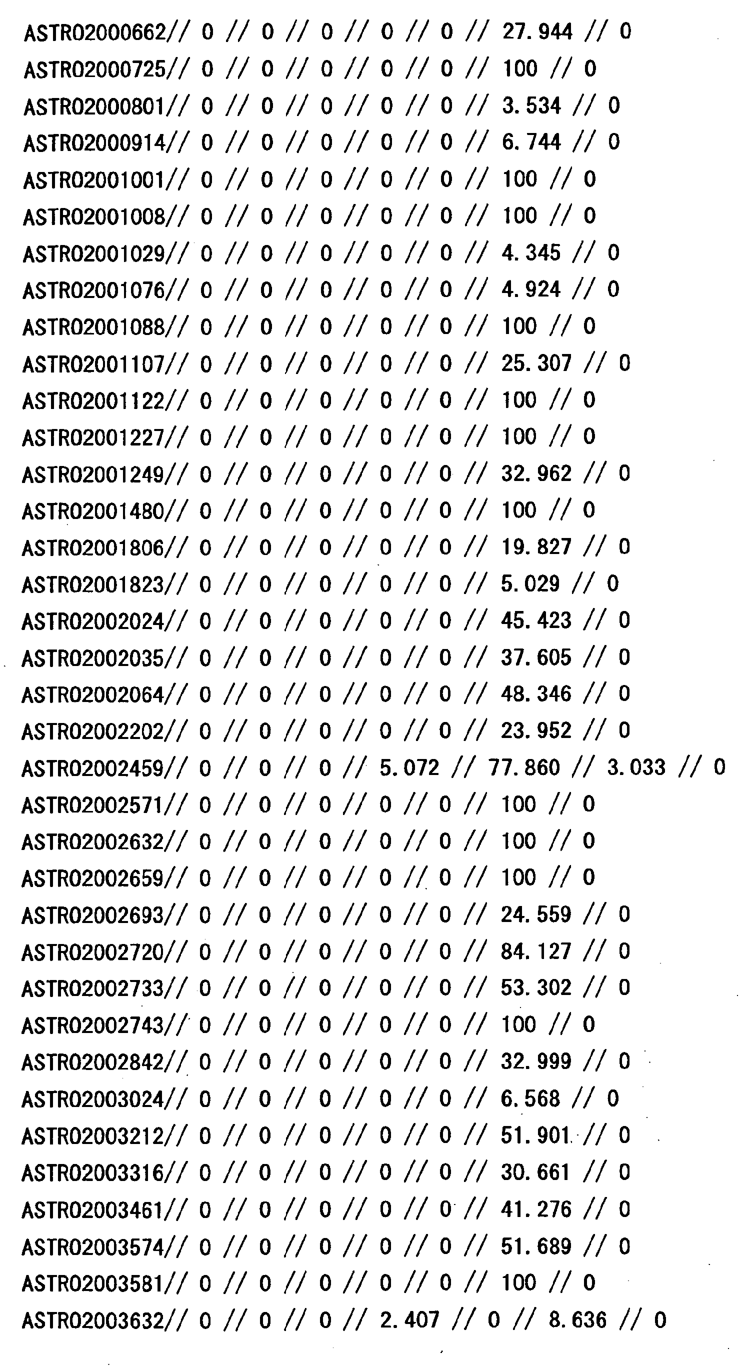

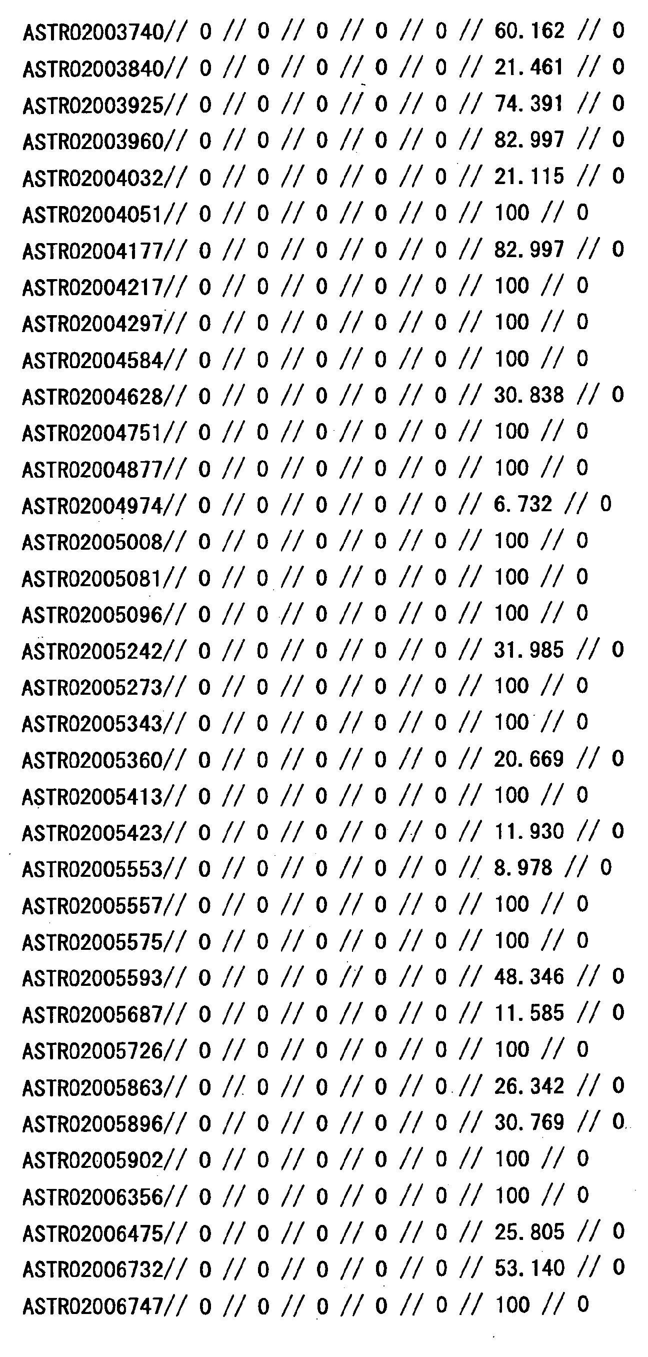

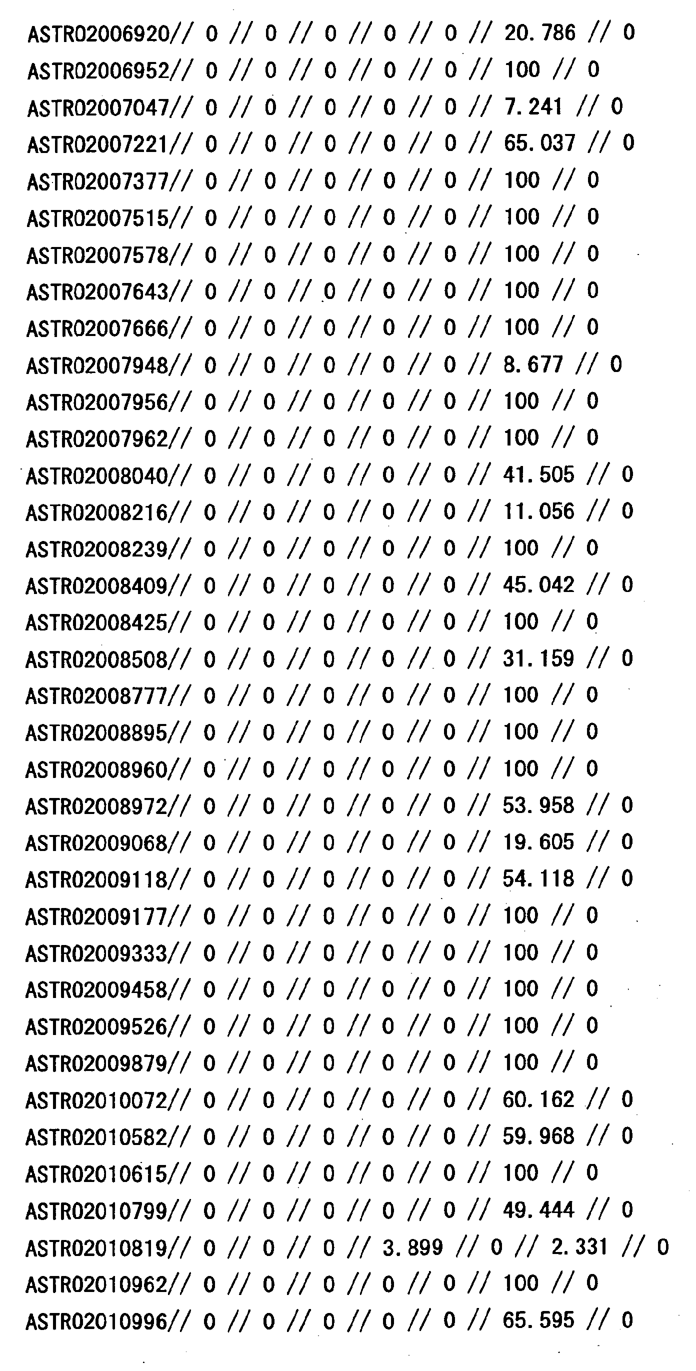

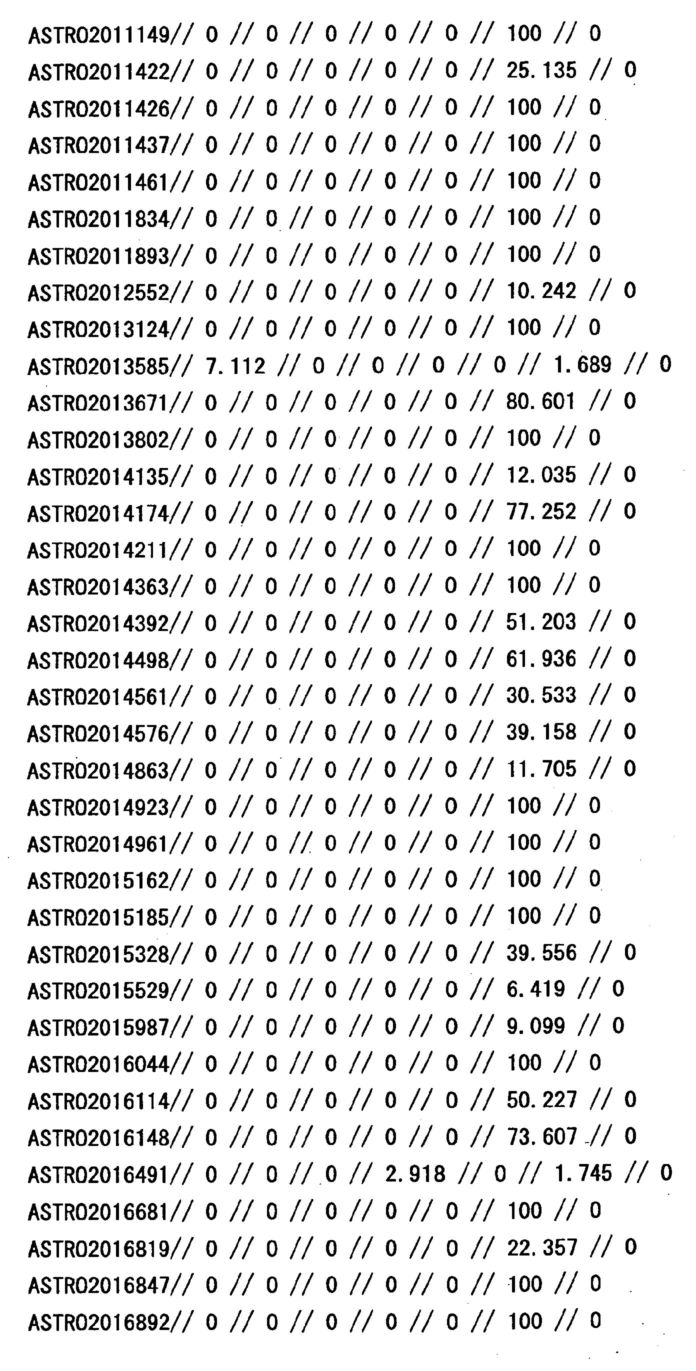

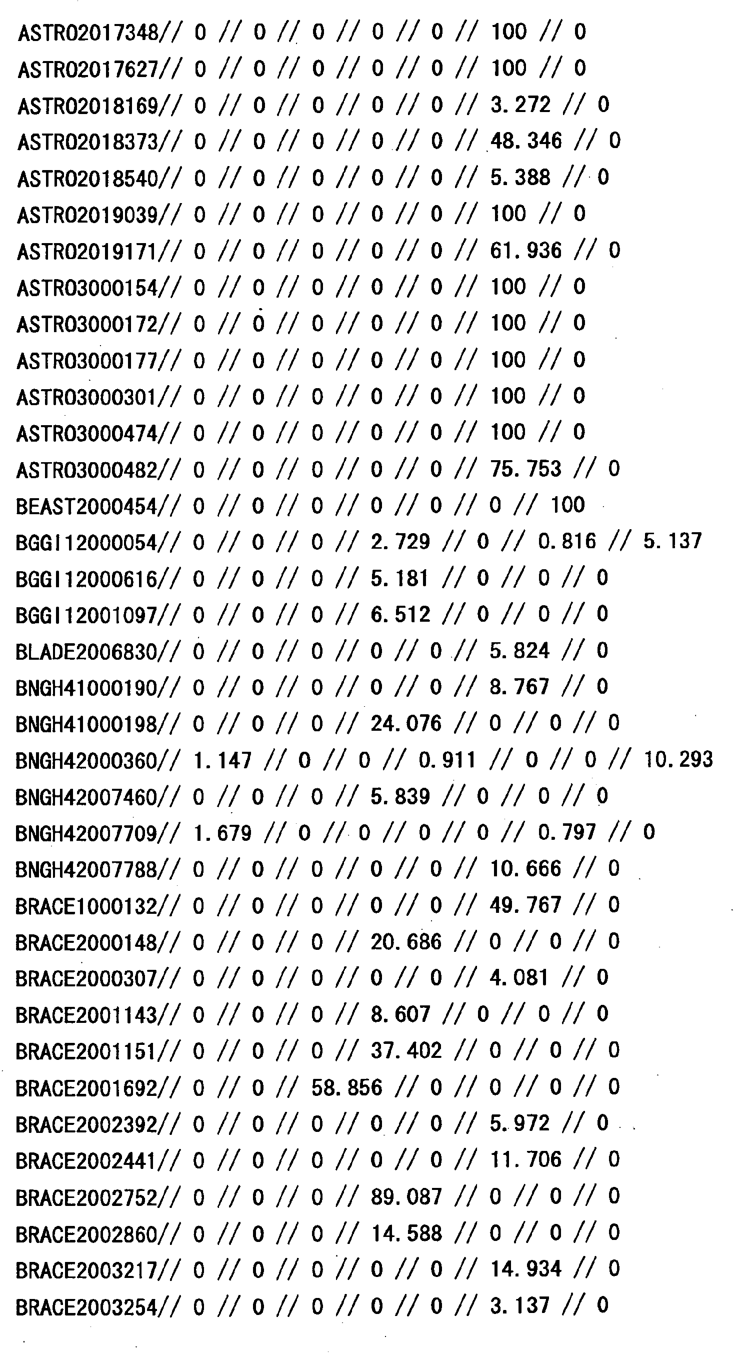

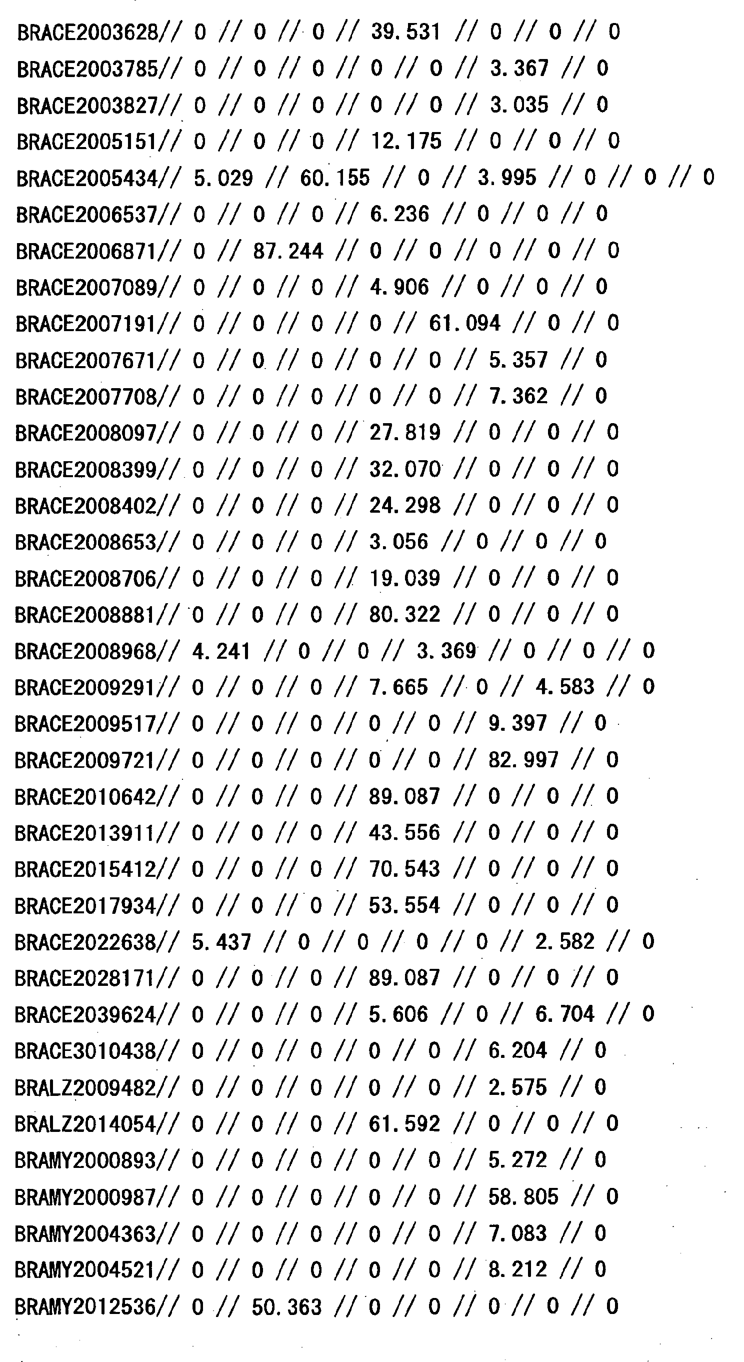

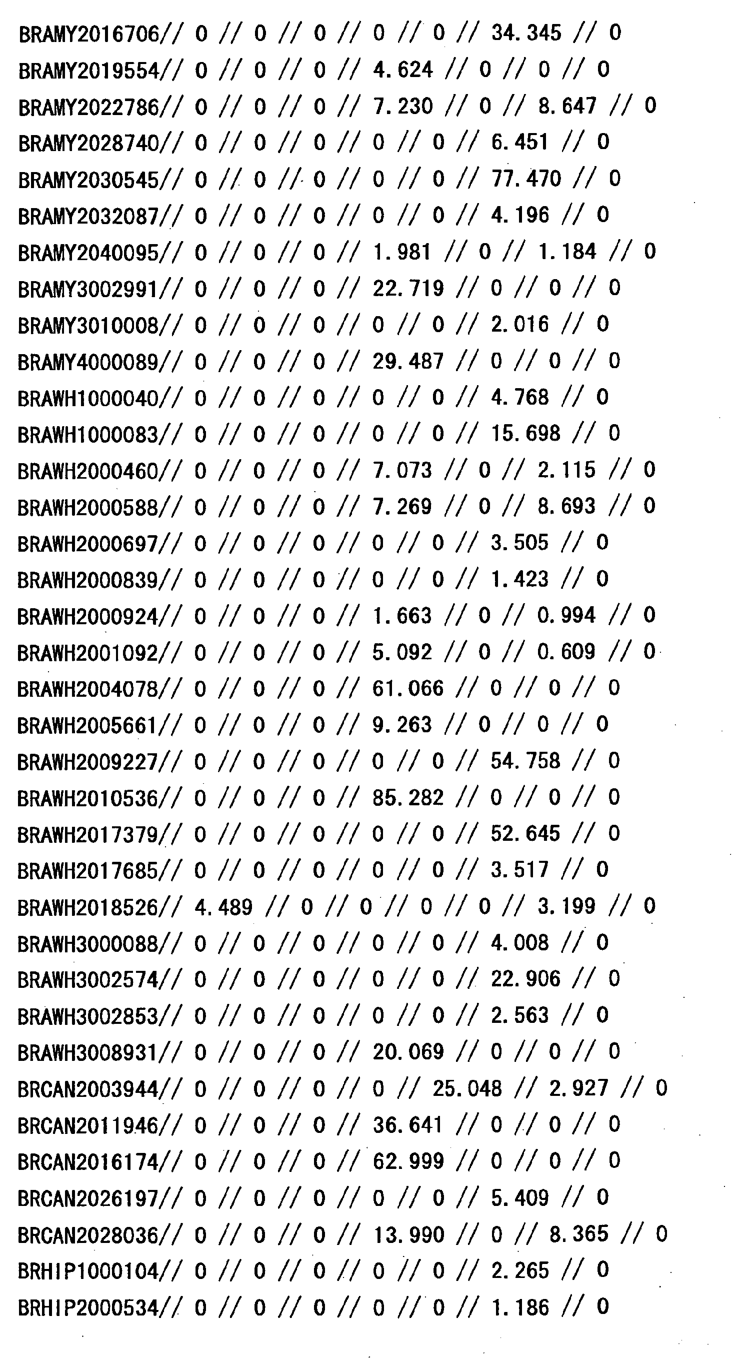

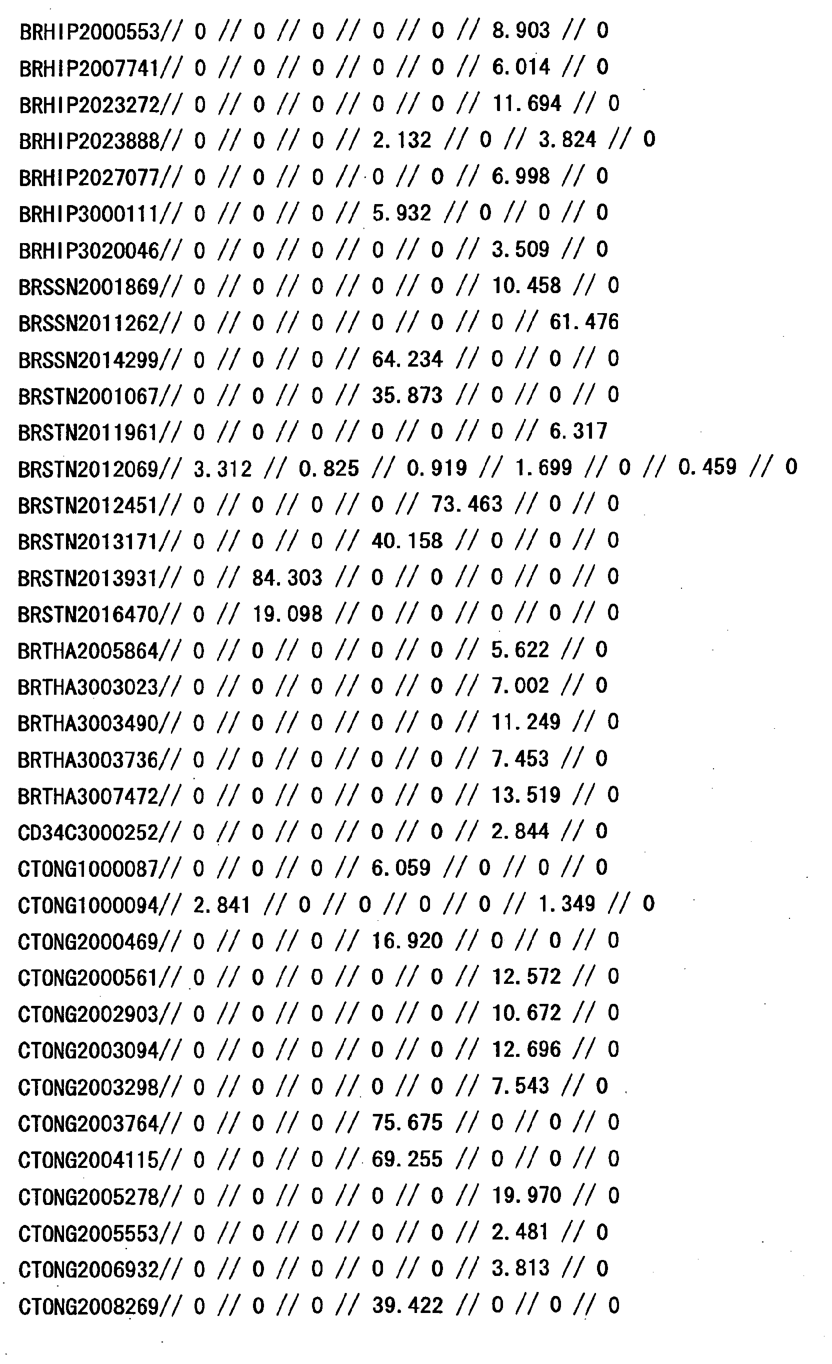

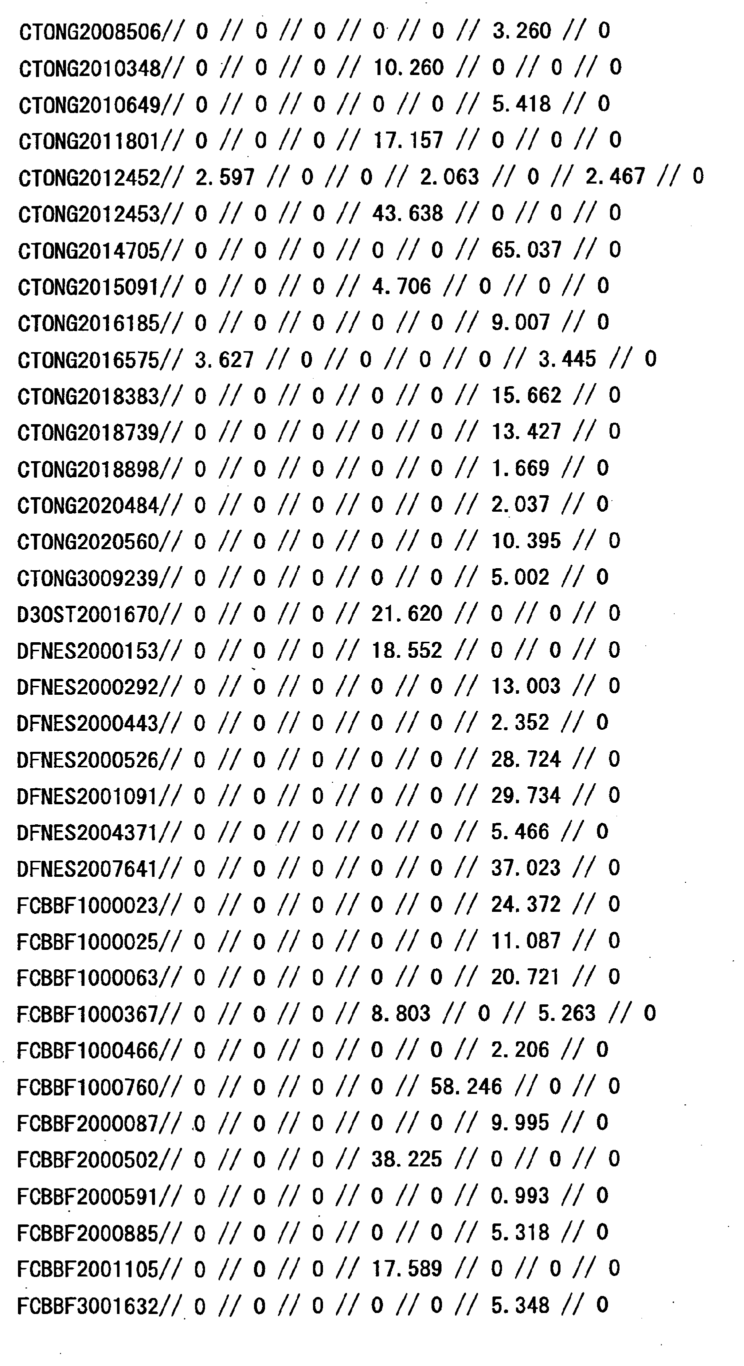

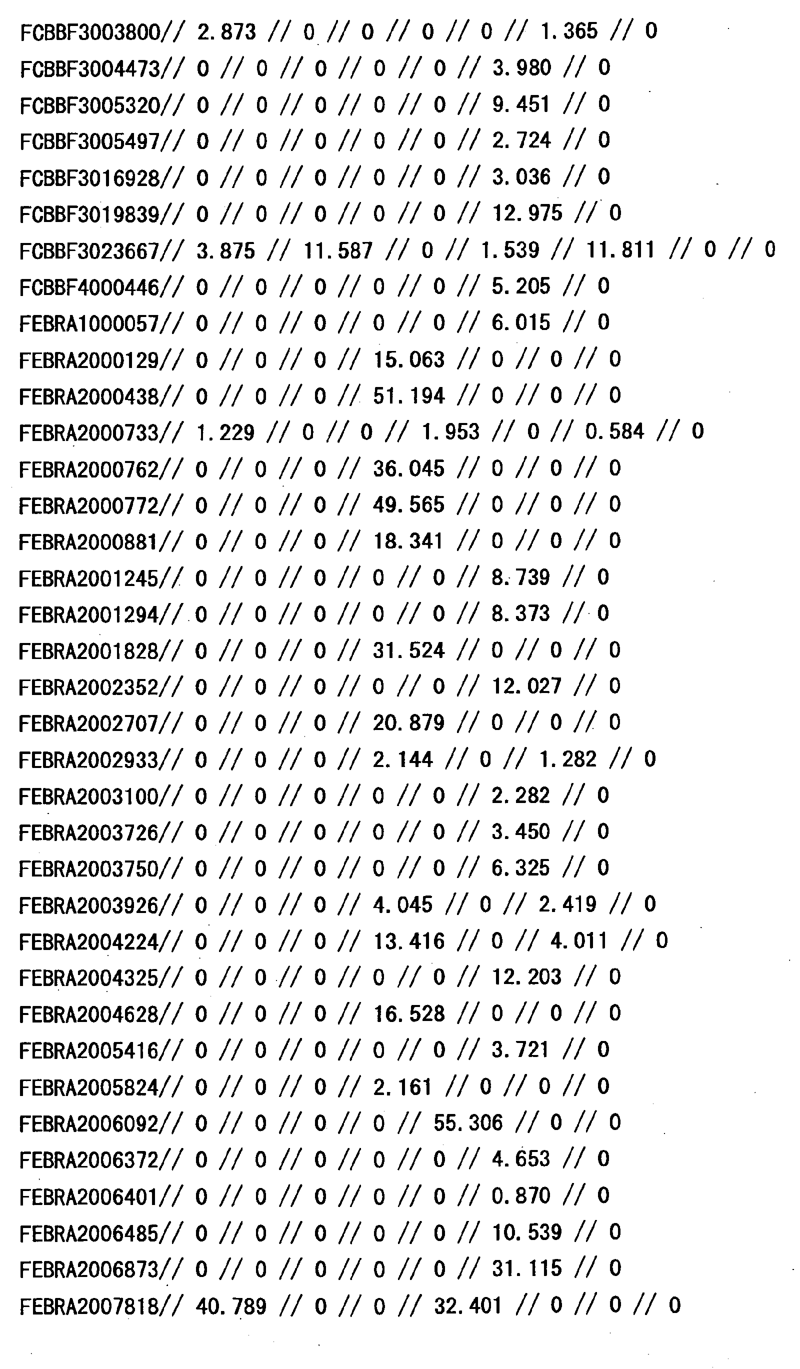

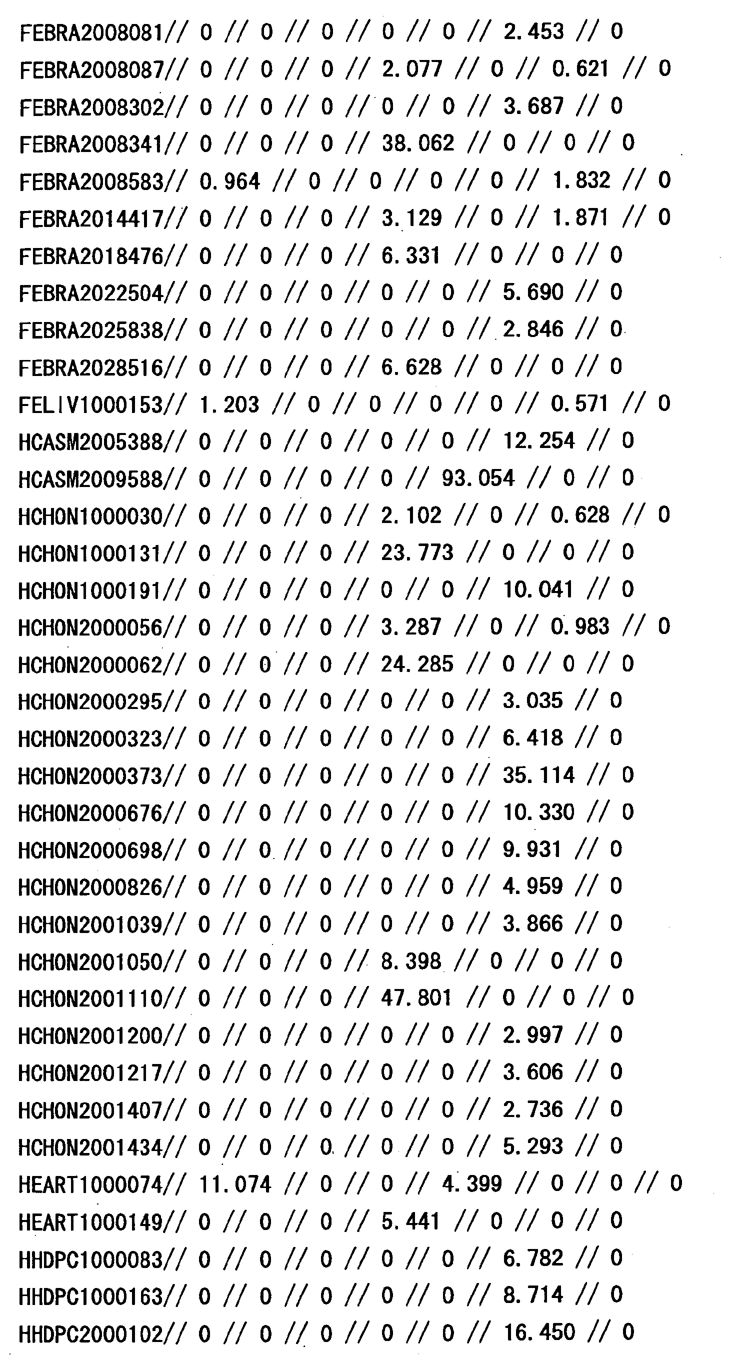

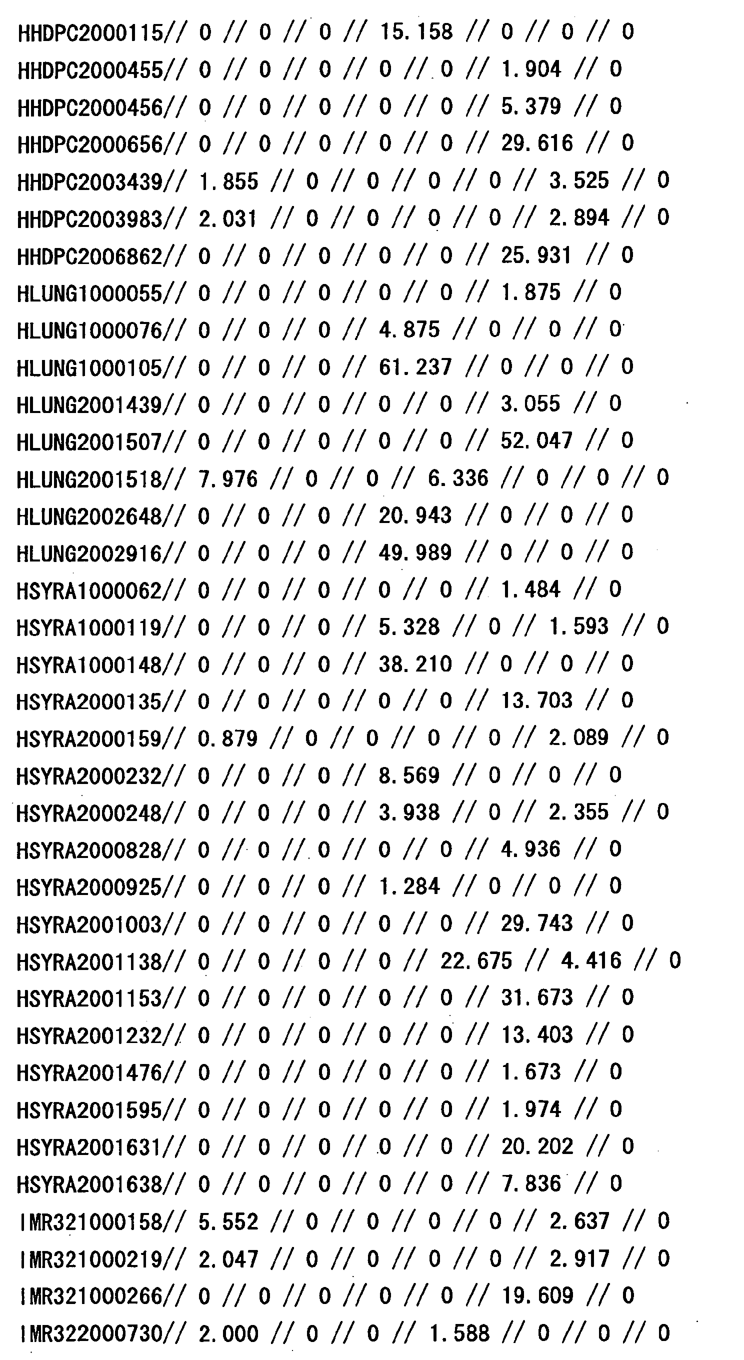

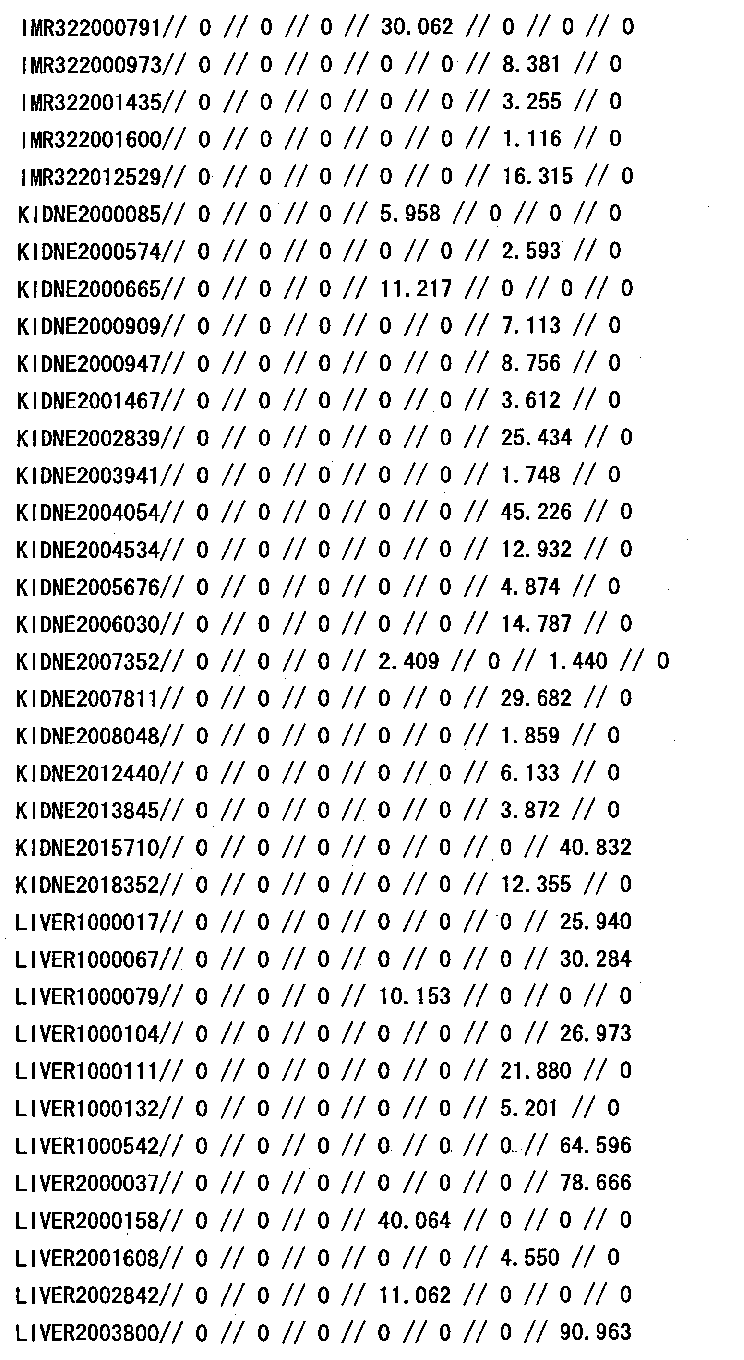

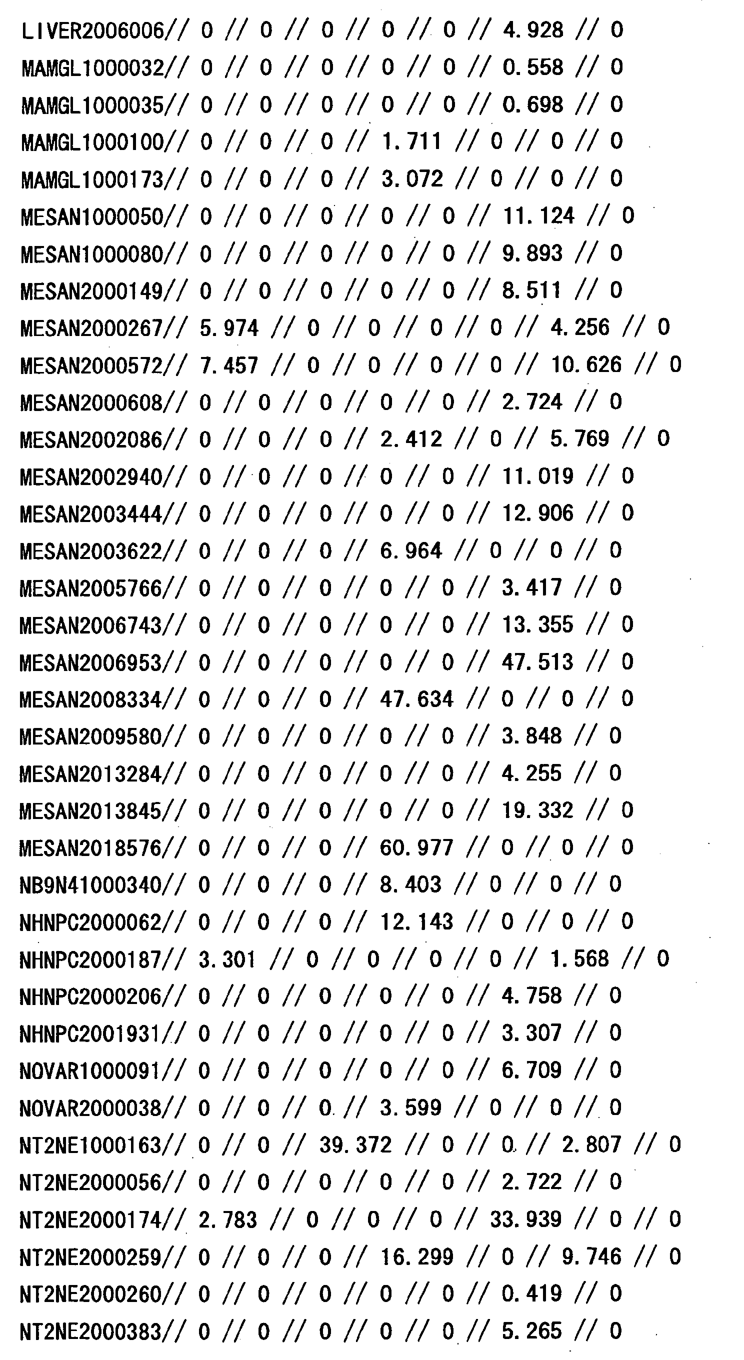

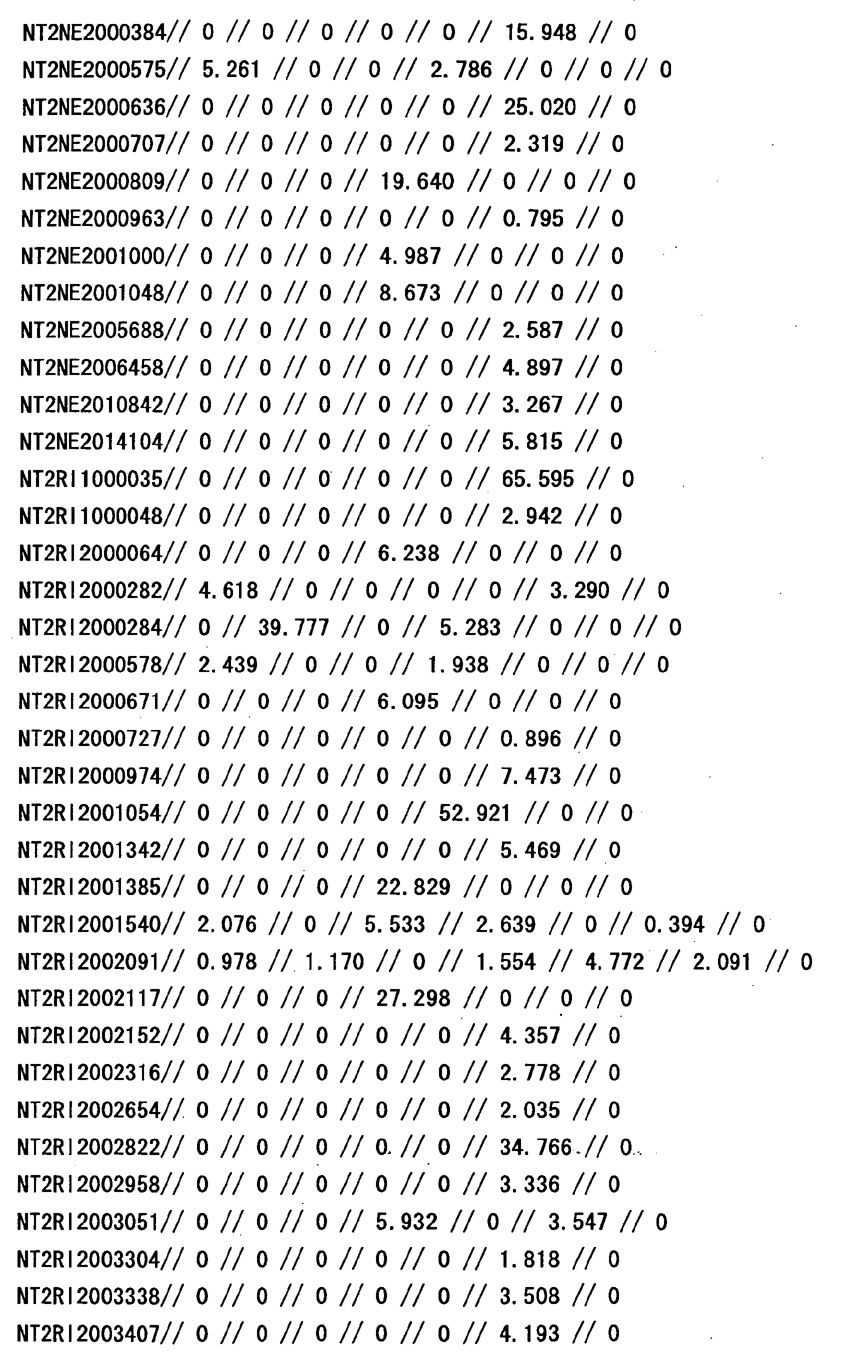

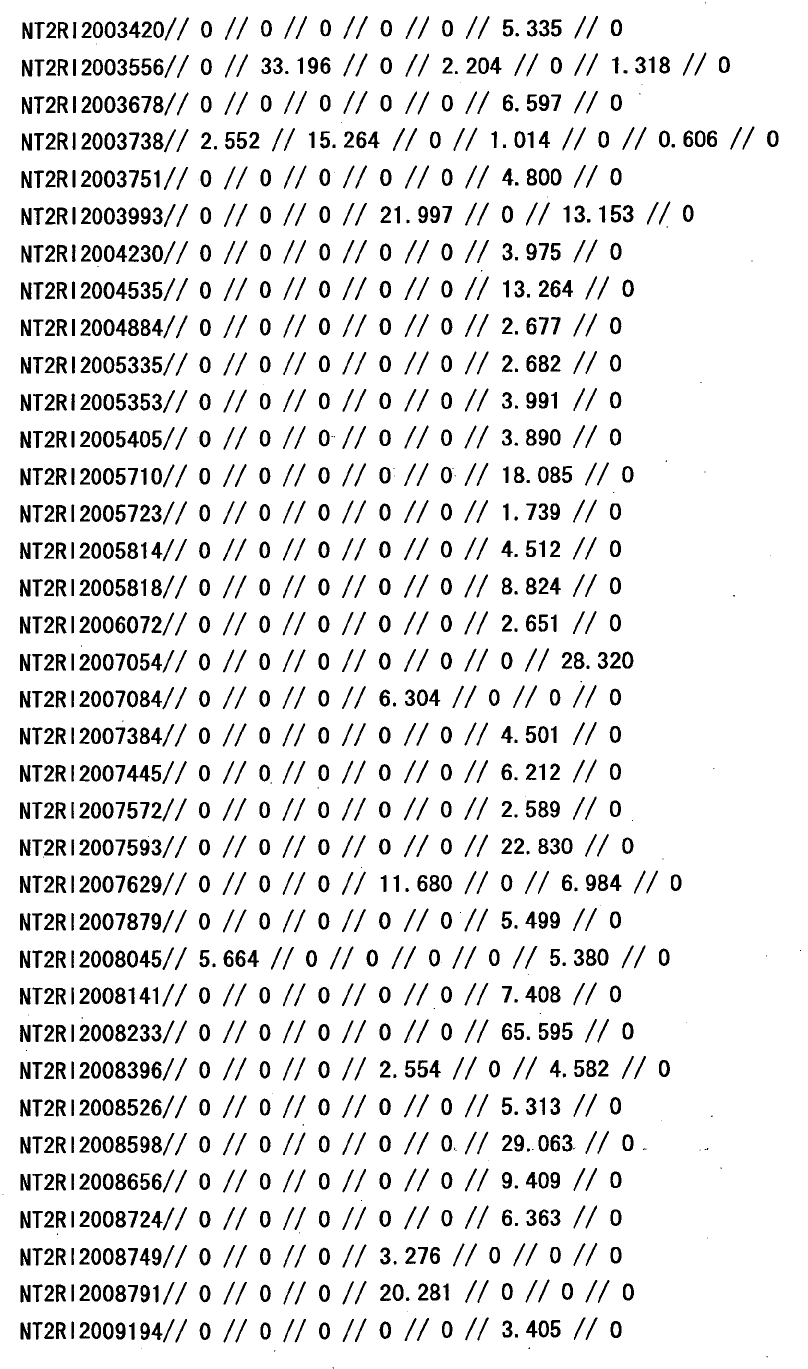

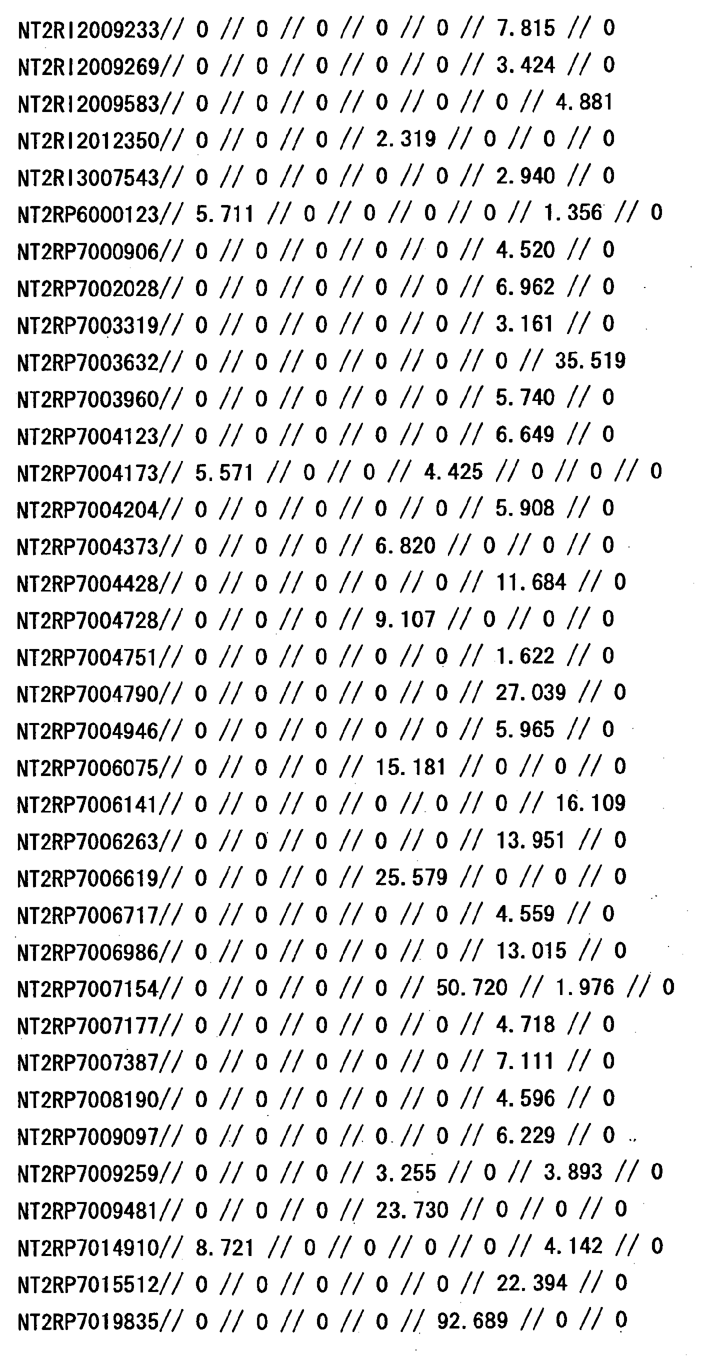

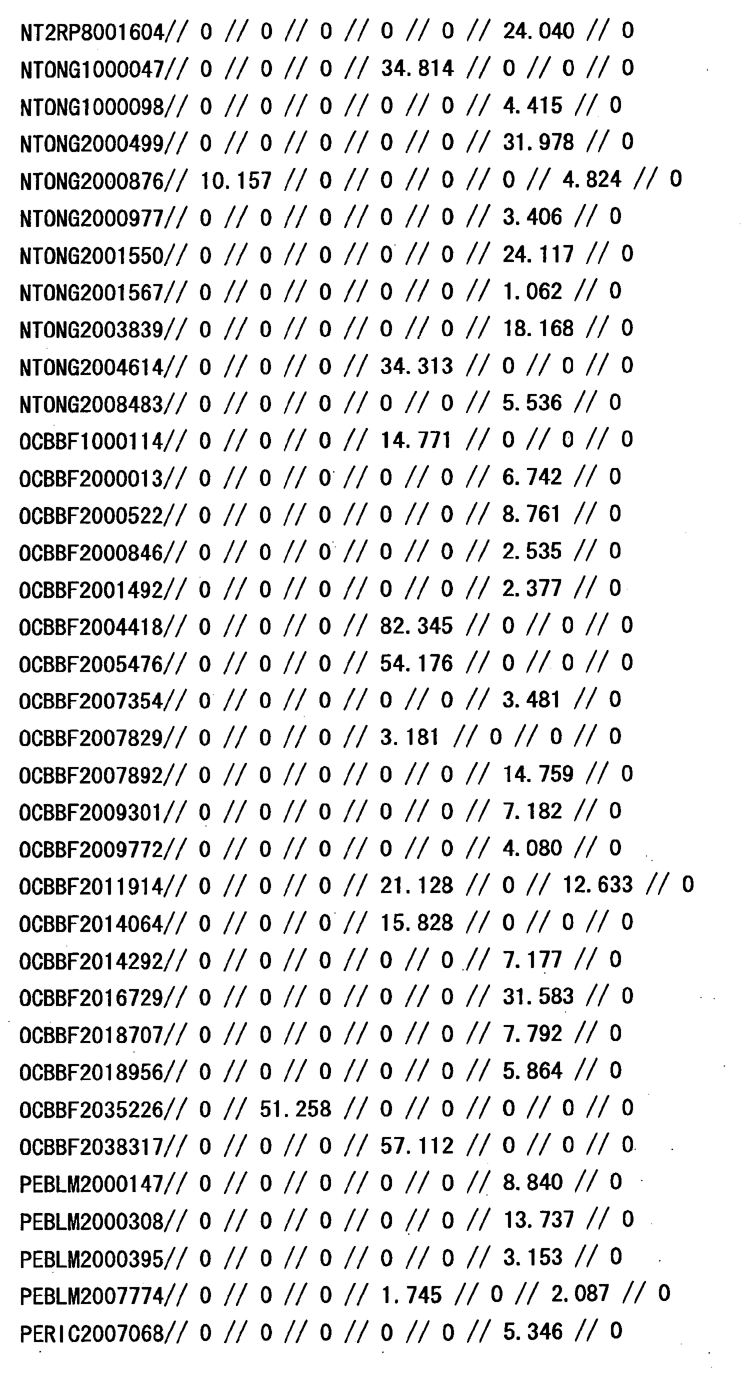

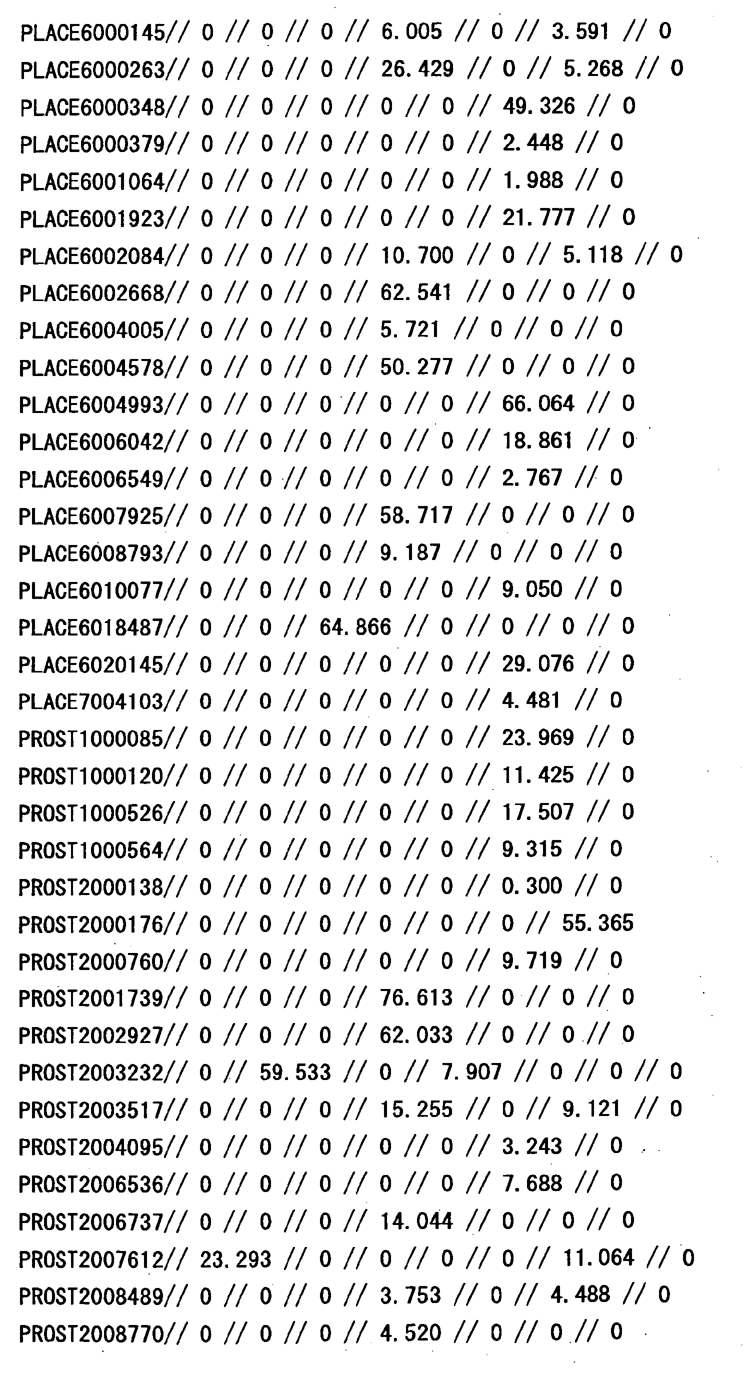

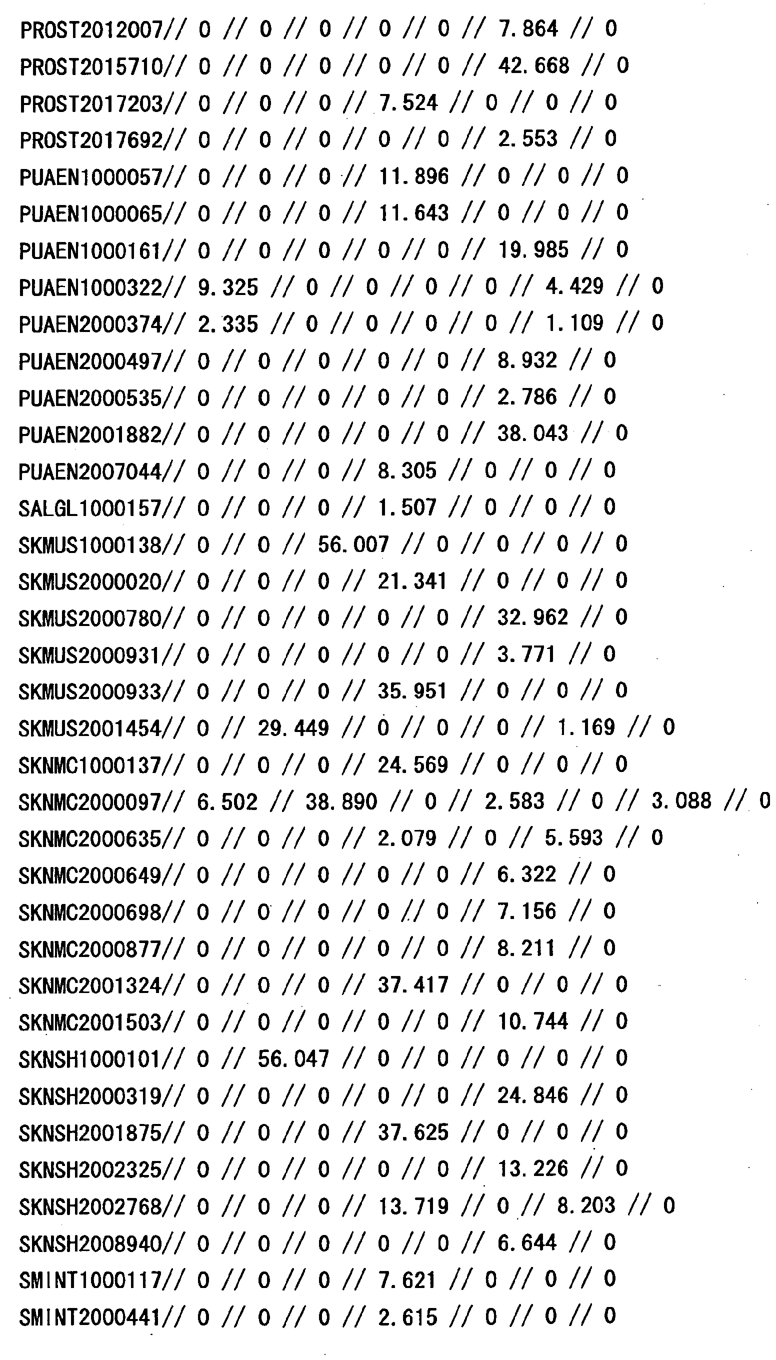

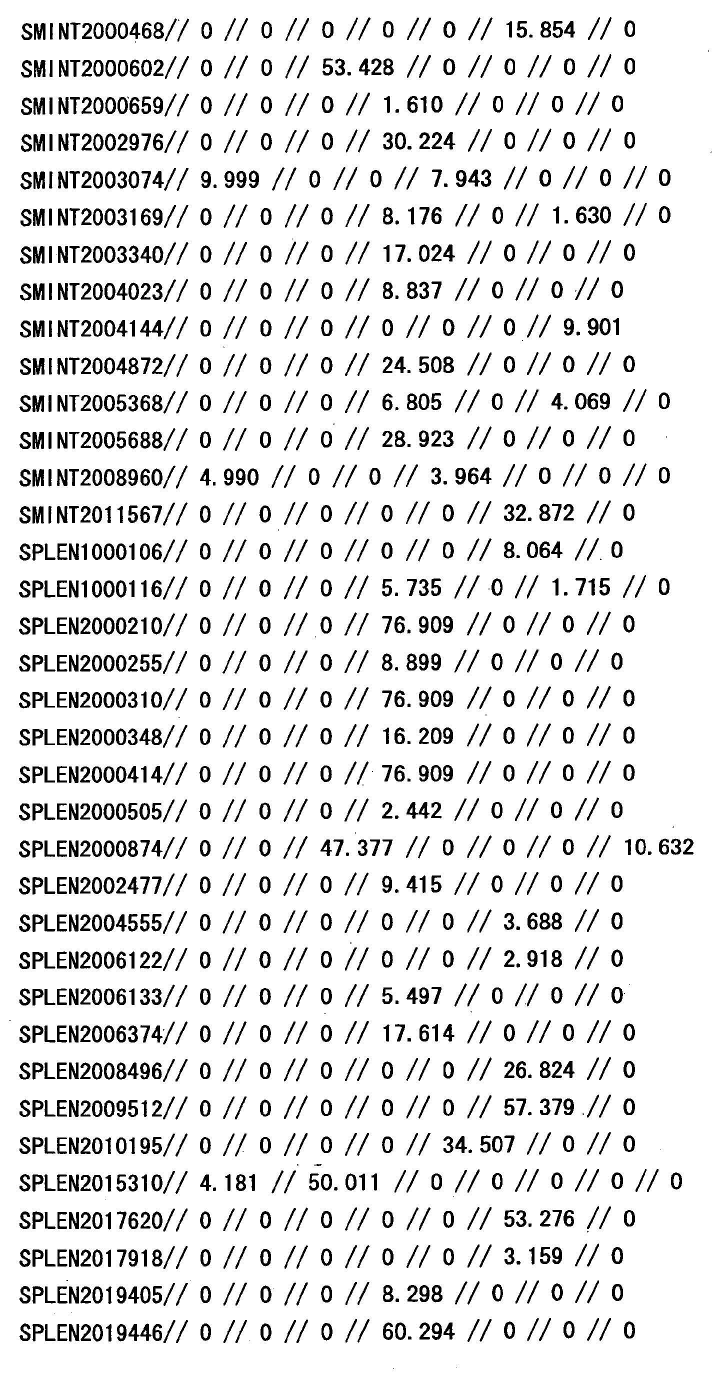

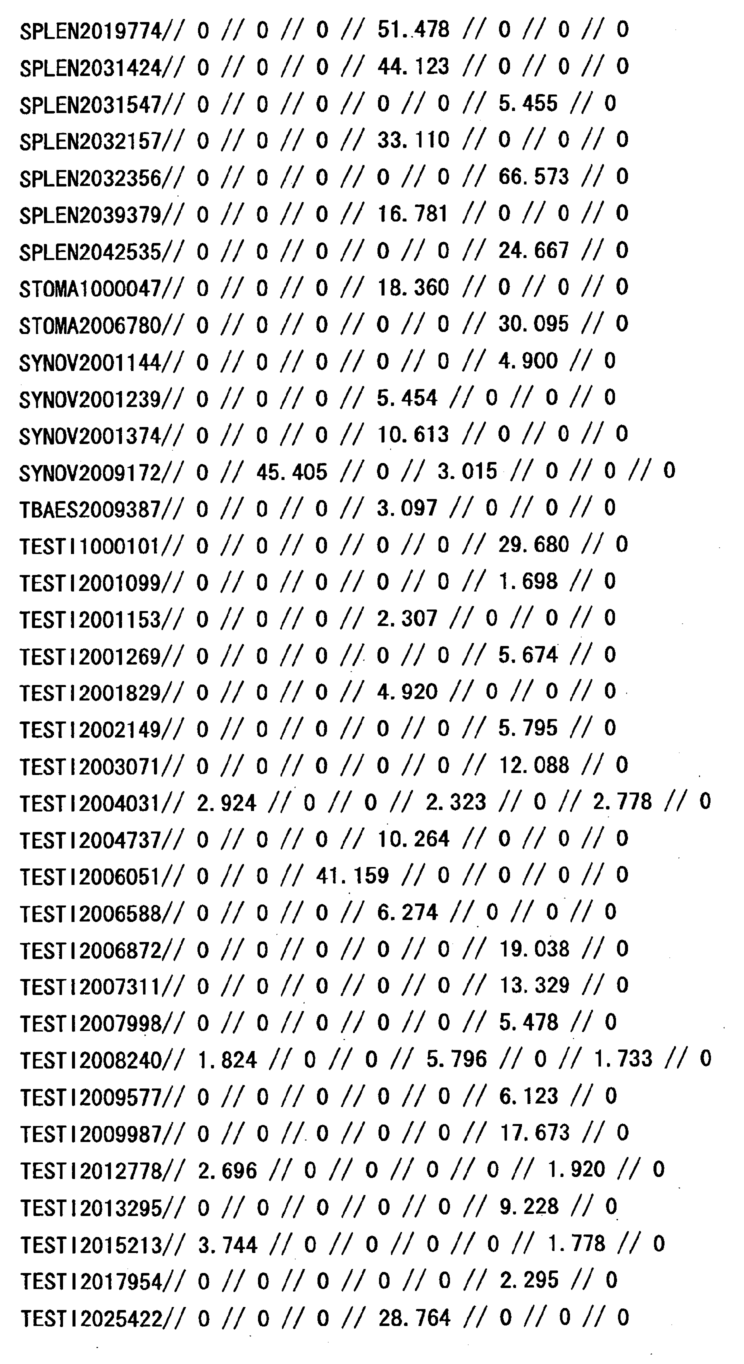

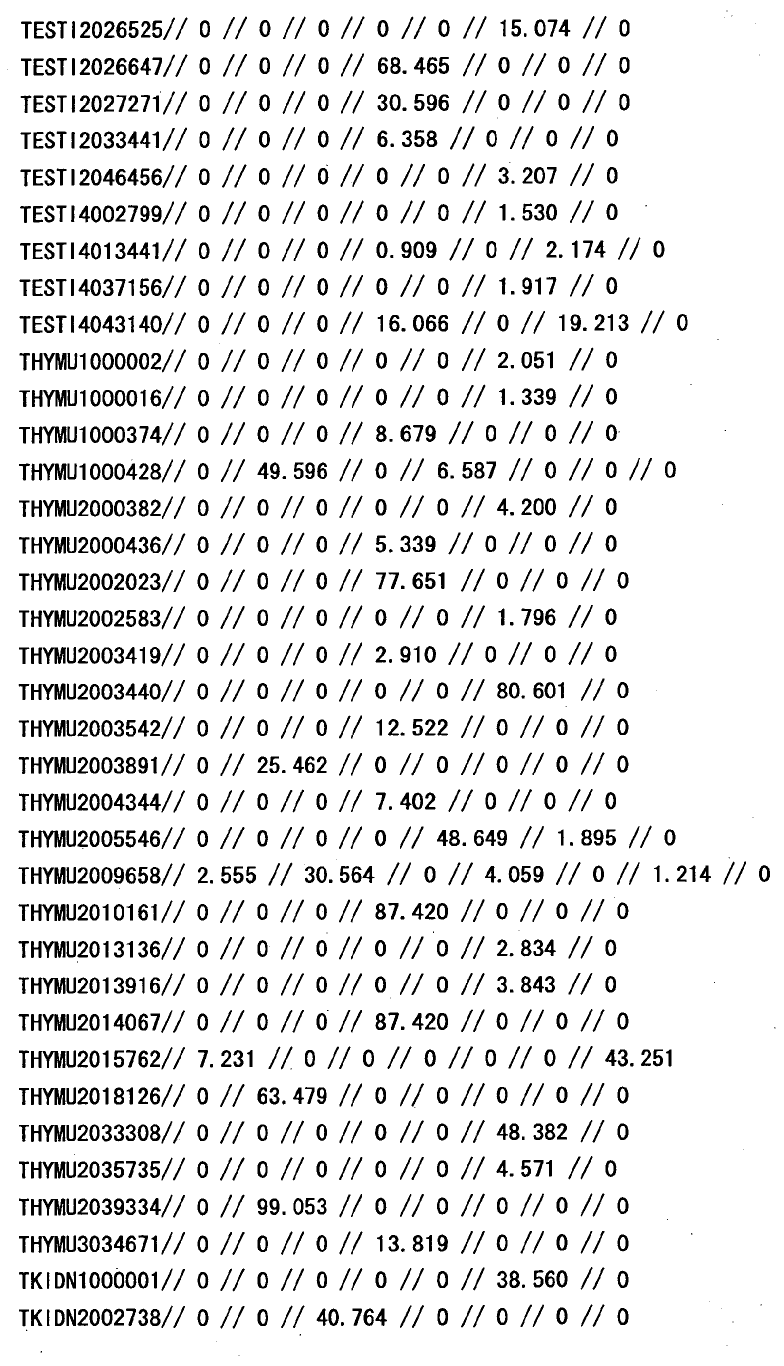

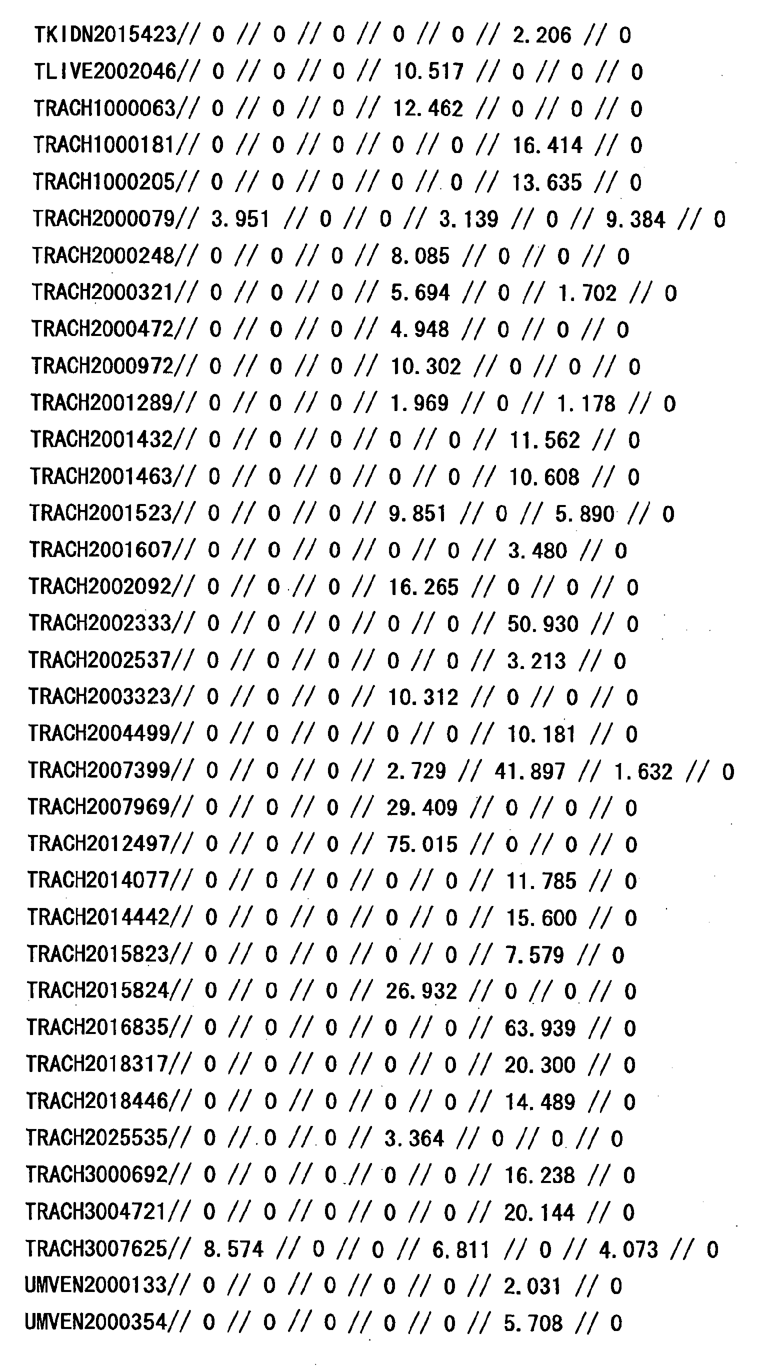

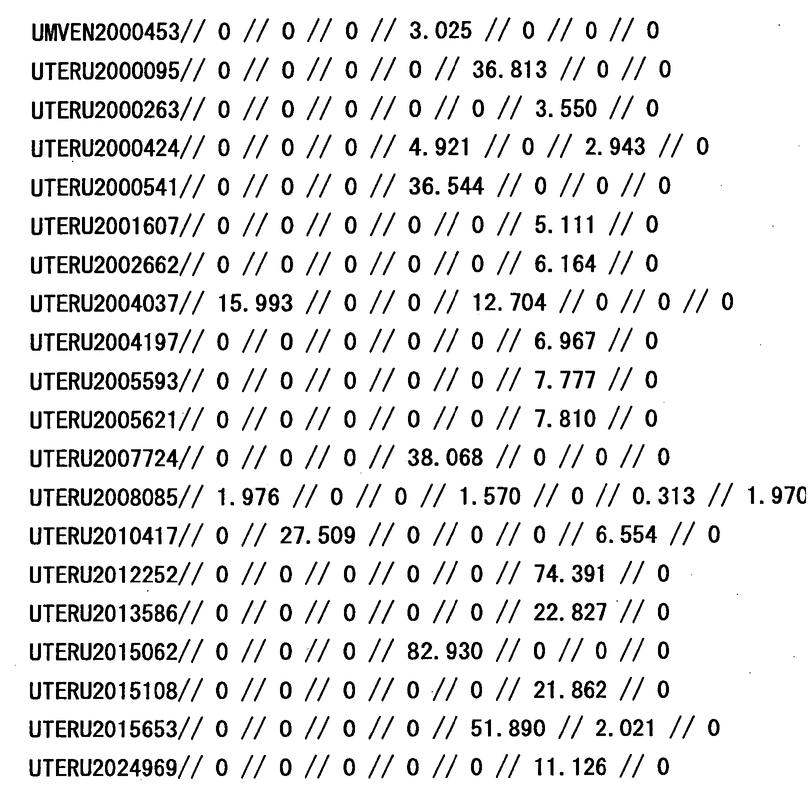

Genes whose expression levels were negligibly low in each of the above-described libraries are shown with their clone names below.
3NB691000085, 3NB691000113, 3NB691000116, 3NB691000129, 3NB691000173, 3NB691000191, 3NB692000012, 3NB692000029, 3NB692000102, 3NB692000154, 3NB692000276, 3NB692000305, 3NB692000429, 3NB692000484, 3NB692000529, 3NB692000545, 3NB692000973, 3NB692001002, 3NB692001034, 3NB692001040, 3NB692001123, 3NB692001267, 3NB692001288, 3NB692001334, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, BGGI11000123, BGGI11000193, BGGI11000285, BGGI12000022, BGGI12000067, BGGI12000161, BGGI12000533, BGGI12000544, BGGI12000684, BGGI12000693, BGGI12000839, BGGI12001002, BGGI12001075, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000177, BNGH41000216, BNGH41000235, BNGH42000130, BNGH42000132, BNGH42000405, BNGH42000532, BNGH42000675, BNGH42000739, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006135, BNGH42006226, BNGH42006234, BNGH42007037, BNGH42007366, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000131, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003172, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003639, BRACE2003700, BRACE2003713, BRACE2003766, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006900, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007682, BRACE2007685, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008401, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008883, BRACE2008917, BRACE2008919, BRACE2008941, BRACE2008948, BRACE2008960, BRACE2008999, BRACE2009014, BRACE2009016, BRACE2009031, BRACE2009037, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2.020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE30469_66, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000585, BRAMY2000653, BRAMY2000814, BRAMY2000946, BRAMY2000981, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004,058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347,. BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000093, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000633, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986, BRHIP2023071, BRHIP2023229, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012174, BRSTN2012284, BRSTN2012320, BRSTN2012380, BRSTN2013354, BRSTN2013502, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003074, BRTHA3003225, BRTHA3003339, BRTHA3003343, BRTHA3003417, BRTHA3003449, BRTHA3003474, BRTHA3003704, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000480, CTONG2000508, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417, CTONG2002418, CTONG2002453, CTONG2002520, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003782, CTONG2003889, CTONG2003937, CTONG2003945, CTONG2004000, CTONG2004062, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006562, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008321, CTONG2008343, CTONG2008398, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010652, CTONG2010803, CTONG2010821, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012425, CTONG2012447, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013907, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018413, CTONG2018483, CTONG2018629, CTONG2018632, CTONG2018637, CTONG2018652, CTONG2018653, CTONG2018655, CTONG2018735, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020522, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028124, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008894, CTONG3008951, CTONG3008959, CTONG3009028, CTONG3009227, CTONG3009287, CTONG3009328, CTONG3009385, D3O5T1000066, D3OST1000109, D3OST1000238, D3OST1000267, D3OST1000270, D3OST2000184, D3OST2000618, D3OST2000654, D3OST2000734, D3OST2001328 D3OST2001669, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000150, DFNES1000185, DFNES2000011, DFNES2000143, DFNES2000146, DFNES2000212, DFNES2000268, DFNES2000335, DFNES2000426, DFNES2000432, DFNES2000457, DFNES2000471, DFNES2000913, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007113, DFNES2007299, DFNES2007332, DFNES2007634, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000024, FCBBF1000027, FCBBF1000038, FCBBF1000053, FCBBF1000061, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000197, FCBBF1000198, FCBBF1000206, FCBBF1000220, FCBBF1000232, FCBBF1000233, FCBBF1000243, FCBBF1000270, FCBBF1000272, FCBBF1000280, FCBBF1000294, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000566, FCBBF2000576, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001470, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005330, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000452, FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000771, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003822, FEBRA2003833, FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004329, FEBRA2004331, FEBRA2004412, FEBRA2004443, FEBRA2004485, FEBRA2004490, FEBRA2004538, FEBRA2004592, FEBRA2004620, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005476, FEBRA2005572, FEBRA2005701, FEBRA2005726, FEBRA2005734, FEBRA2005752, FEBRA2005778, FEBRA2005787, FEBRA2005788, FEBRA2005995, FEBRA2005998, FEBRA2006055, FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006379, FEBRA2006396, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007823, FEBRA2007880, FEBRA2007900, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008311, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005484, HCASM2006131, HCASM2006338, HCASM2006359, HCASM2.006632, HCASM2007047, HCASM2007317, HCASM2007737, HCASM2007773, HCASM2008154, HCASM2008536, HCASM2009405, HCASM2009424, HCASM2009463, HCHON1000015, HCHON1000142, HCHON1000166, HCHON1000176, HCHON2000028, HCHON2000038, HCHON2000087, HCHON2000100, HCHON2000156, HCHON2000160, HCHON2000199, HCHON2000212, HCHON2000226, HCHON2000244, HCHON2000265, HCHON2000271, HCHON2000315, HCHON2000322, HCHON2000344, HCHON2000364, HCHON2000395, HCHON2000417, HCHON2000418, HCHON2000473, HCHON2000475, HCHON2000503, HCHON2000508, HCHON2000626, HCHON2000660, HCHON2000663, HCHON2000673, HCHON2000699, HCHON2000705, HCHON2000738, HCHON2000743, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001084, HCHON2001099, HCHON2001108, HCHON2001116, HCHON2001161, HCHON2001214, HCHON2001233, HCHON2001269, HCHON2001359, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004002, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000088, HEART1000099, HEART1000102, HEART1000118, HEART1000132, HEART1000139, HEART1000142, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC2000055, HHDPC2000070, HHDPC2000104, HHDPC2000149, HHDPC2000258, HHDPC2000315, HHDPC2000462, HHDPC2000572, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006927, HHDPC2007267, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000064, HLUNG1000084, HLUNG1000091, HLUNG1000099, HLUNG1000104, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001459, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002811, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004159, HLUNG2004170, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011_{.}833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000152, HSYRA1000178, HSYRA2000044, HSYRA2000137, HSYRA2000190, HSYRA2000221, HSYRA2000224, HSYRA2000237, HSYRA2000253, HSYRA2000255, HSYRA2000332, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000832, HSYRA2000927, HSYRA2001103, HSYRA2001105, HSYRA2001142, HSYRA2001225, HSYRA2001255, HSYRA2001332, HSYRA2001353, HSYRA2001396, HSYRA2001420, HSYRA2001484, HSYRA2001552, HSYRA2001567, HSYRA2001574, HSYRA2001580, HSYRA2001615, HSYRA2001621, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000210, IMR321000230, IMR321000242, IMR321000253, IMR322000010, IMR322000072, IMR322000107, IMR322000121, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000742, IMR322000775, IMR322000811, IMR322000819, IMR322000838, IMR322000859, IMR322000863, IMR322000917, IMR322000918, IMR322000919, IMR322000935, IMR322000953, IMR322000984, IMR322001018, IMR322001049, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001534, IMR322001541, IMR322001584, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801; KIDNE2000832, KIDNE2000833, KIDNE2000846, KIDNE2000867, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006039, KIDNE2006053, KIDNE2006062, KIDNE2006092, KIDNE2006145, KIDNE2006149, KIDNE2006210, KIDNE2006248, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007910, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014290, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000050, LIVER1000055, LIVER1000058, LIVER1000063, LIVER1000073, LIVER1000082, LIVER1000099, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER2000003, LIVER2000033, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000132, MAMGL1000151, MAMGL1000178, MAMGL1000182, MAMGL1000184, MESAN1000032, MESAN1000035, MESAN1000079, MESAN1000101, MESAN1000121, MESAN1000126, MESAN1000147, MESAN1000180, MESAN2000023, MESAN2000026, MESAN2000067, MESAN2000092, MESAN2000167, MESAN2000264, MESAN2000291, MESAN2000337, MESAN2000434, MESAN2000457, MESAN2000462, MESAN2000501, MESAN2000620, MESAN2000711, MESAN2000815, MESAN2000894, MESAN2000909, MESAN2001154, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002790, MESAN2002844, MESAN2002978, MESAN2003035, MESAN2003037, MESAN2003039, MESAN2003058, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005808, MESAN2005811, MESAN2005957, MESAN2005958, MESAN2006022, MESAN2006043, MESAN2006328, MESAN2006401, MESAN2006563, MESAN2006580, MESAN2006599, MESAN2006971, MESAN2007032, MESAN2007440, MESAN2007597, MESAN2007755, MESAN2008222, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000284, NT2NE2000299, NT2NE2000327, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000658, NT2NE2000706, NT2NE2000763, NT2NE2000802, NT2NE2000808, NT2NE2000864, NT2NE2000909, NT2NE2000913, NT2NE2000950, NT2NE2000951, NT2NE2000980, NT2NE2001005, NT2NE2001021, NT2NE2001040, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000027, NT2RI1000127, NT2RI1000164, NT2RI1000172, NT2RI2000004, NT2RI2000007, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178, NT2RI2001230, NT2RI2001235, NT2RI2001244, NT2RI2001307, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621,. NT2RI2001624, NT2RI2001657, NT2RI2001726, NT2RI2001731, NT2RI2001769, NT2RI2001782, NT2RI2001836, NT2RI2001846, NT2RI2001859, NT2RI2001866, NT2RI2001910, NT2RI2001952, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002847, NT2RI2002852, NT2RI2002865, NT2RI2002923, NT2RI2002926, NT2RI2002970, NT2RI2003011, NT2RI2003019, NT2RI2003067, NT2RI2003184, NT2RI2003205, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667, NT2RI2003695, NT2RI2003884, NT2RI2003921, NT2RI2003973, NT2RI2004059, NT2RI2004078, NT2RI2004079, NT2RI2004093, NT2RI2004136, NT2RI2004157, NT2RI2004188, NT2RI2004190, NT2RI2004209, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005150, NT2RI2005166, NT2RI2005239, NT2RI2005246, NT2RI2005315, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005713, NT2RI2005772, NT2RI2005811, NT2RI2005816, NT2RI2005851, NT2RI2005966, NT2RI2006070, NT2RI2006071, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006856, NT2RI2006943, NT2RI2006973, NT2RI2007046, NT2RI2007048, NT2RI2007096, NT2RI2007116, NT2RI2007133, NT2RI2007148, NT2RI2007214, NT2RI2007277, NT2RI2007303, NT2RI2007334, NT2RI2007377, NT2RI2007386, NT2RI2007406, NT2RI2007439, NT2RI2007468, NT2RI2007469, NT2RI2007498, NT2RI2007507, NT2RI2007589, NT2RI2007723, NT2RI2007729, NT2RI2007751, NT2RI2007754, NT2RI2007827, NT2RI2007853, NT2RI2007877, NT2RI2007884, NT2RI2007891, NT2RI2007956, NT2RI2007965, NT2RI2007987, NT2RI2008007, NT2RI2008050, NT2RI2008171, NT2RI2008188, NT2RI2008204, NT2RI2008221, NT2RI2008336, NT2RI2008455, NT2RI2008481, NT2RI2008502, NT2RI2008566, NT2RI2008622, NT2RI2008714, NT2RI2008801, NT2RI2008808, NT2RI2008812, NT2RI2008841, NT2RI2008942, NT2RI2008952, NT2RI2009005, NT2RI2009037, NT2RI2009065, NT2RI2009066, NT2RI2009083, NT2RI2009101, NT2RI2009103, NT2RI2009144, NT2RI2009151, NT2RI2009173, NT2RI2009215, NT2RI2009239, NT2RI2009259, NT2RI2009281, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000043, NT2RP6000059, NT2RP6000060, NT2RP6000072, NT2RP6000077, NT2RP6000078, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000076, NT2RP7000086, NT2RP7000109, NT2RP7000112, NT2RP7000173, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624, NT2RP7000812, NT2RP7000926, NT2RP7001074, NT2RP7001080, NT2RP7001156, NT2RP7001166, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002064, NT2RP7002151, NT2RP7002163, NT2RP7002243, NT2RP7002282, NT2RP7002376-, NT2RP7002379, NT2RP7002449, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002829, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003439, NT2RP7003511, NT2RP7003629, NT2RP7003647, NT2RP7003680, NT2RP7003688, NT2RP7003724, NT2RP7004027, NT2RP7004080, NT2RP7004114, NT2RP7004196, NT2RP7004233, NT2RP7004260, NT2RP7004348, NT2RP7004352, NT2RP7004358, NT2RP7004396, NT2RP7004481, NT2RP7004541, NT2RP7004559, NT2RP7004641, NT2RP7004656, NT2RP7004687, NT2RP7004722, NT2RP7004766, NT2RP7004884, NT2RP7004911, NT2RP7004915, NT2RP7004925, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006160, NT2RP7006162, NT2RP7006188, NT2RP7006223, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006853, NT2RP7006886, NT2RP7006887, NT2RP7006980, NT2RP7006995, NT2RP7007114, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007842, NT2RP7007925; NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570; NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006,. NTONG1000033, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG1000264, NTONG2000040, NTONG2000107, NTONG2000231, NTONG2000265, NTONG2000334, NTONG2000363, NTONG2000413, NTONG2000492, NTONG2000531, NTONG2000583, NTONG2000800, NTONG2000858, NTONG2000878, NTONG2000966, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001587, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004690, NTONG2004806, NTONG2004829, NTONG2004844, NTONG2004918, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005497, NTONG2005520, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000086, OCBBF1000091, OCBBF1000104, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000287, OCBBF2000323, OCBBF2000452, OCBBF2000467, OCBBF2000677, OCBBF2000703, OCBBF2000712, OCBBF2000719, OCBBF2000824, OCBBF2000831, OCBBF2000904, OCBBF2000982, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009242, OCBBF2009352, OCBBF2009391, OCBBF2009424, OCBBF2009536, OCBBF2009571, OCBBF2009583, OCBBF2009603, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926, OCBBF2014052, OCBBF2014089, OCBBF2014322, OCBBF2014386., OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295,.. OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030, PEBLM2000112, PEBLM2000126, PEBLM2000170, PEBLM2000180, PEBLM2000213, PEBLM2000222, PEBLM2000248, PEBLM2000267, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000371, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001281, PLACE6001294, PLACE6001443, PLACE6001536, PLACE6001557, PLACE6001712, PLACE6001740, PLACE6001823, PLACE6001886, PLACE6001925, PLACE6001933, PLACE6002011, PLACE6002016, PLACE6002056, PLACE6002102, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004312, PLACE6004336, PLACE6004368, PLACE6004380, PLACE6004396, PLACE6004397, PLACE6004454, PLACE6004464, PLACE6004491, PLACE6004663, PLACE6004687, PLACE6004738, PLACE6004931, PLACE6004966, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010752, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000097, PROST1000110, PROST1000123, PROST1000136, PROST1000144, PROST1000145, PROST1000152, PROST1000167, PROST1000184, PROST1000199, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002960, PROST2003102, PROST2003117, PROST2003210, PROST2003302, PROST2003303, PROST2003324, PROST2003338, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004270, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006579; PROST2006688, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010782, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000164, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000394, PUAEN2000594, PUAEN2000684, PUAEN2000942, PUAEN2001260, PUAEN2001345, PUAEN2001526, PUAEN2001586, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000176, SKMUS1000177, SKMUS1000186, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000821, SKMUS2000847, SKMUS2000863, SKMUS2000873, SKMUS2000894, SKMUS2000898, SKMUS2000902, SKMUS2000945, SKMUS2000954, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000168, SKNMC1000229, SKNMC1000251, SKNMC1000264, SKNMC2000065, SKNMC2000224, SKNMC2000256, SKNMC2000305, SKNMC2000322, SKNMC2000356, SKNMC2000374, SKNMC2000583, SKNMC2000593, SKNMC2000612, SKNMC2000622, SKNMC2000966, SKNMC2001113, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000174, SKNSH1000301, SKNSH1000308, SKNSH1000416, SKNSH2000051, SKNSH2000101, SKNSH2000151, SKNSH2000163, SKNSH2000250, SKNSH2000347, SKNSH2000482, SKNSH2000516, SKNSH2000550, SKNSH2000716, SKNSH2000819, SKNSH2000971, SKNSH2001222, SKNSH2001931, SKNSH2002054, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT1000192, SMINT2000007, SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000629, SMINT2000694, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2003003, SMINT2003128, SMINT2003150, SMINT2003317, SMINT2003386, SMINT2003423, SMINT2003505, SMINT2003551, SMINT2003569, SMINT2003641, SMINT2003644, SMINT2003866, SMINT2003905, SMINT2004037, SMINT2004047, SMINT2004086, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545; SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011588, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000222, SPLEN2000242, SPLEN2000243, SPLEN2000247, SPLEN2000258, SPLEN2000267, SPLEN2000270, SPLEN2000307, SPLEN2000341, SPLEN2000350, SPLEN2000357, SPLEN2000364, SPLEN2000402, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000882, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002467, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005927, SPLEN2005939, SPLEN2006143, SPLEN2006232, SPLEN2006268, SPLEN2006283, SPLEN2006305, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008591, SPLEN2008786, SPLEN2009081, SPLEN2009088, SPLEN2009163, SPLEN2009315, SPLEN2009340, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019471, SPLEN2019480, SPLEN2019505, SPLEN2019571, SPLEN2019575, SPLEN2019709, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028466, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031674, SPLEN2031724, SPLEN2031780, SPLEN2032036, SPLEN2032112, SPLEN2032154, SPLEN2032321, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000124, SYNOV1000128, SYNOV1000164, SYNOV1000190; SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001212, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013.365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090; TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767, TESTI2005775, TESTI2005784, TESTI2005788, TESTI2005827, TESTI2005835, TESTI2005860, TESTI2005892, TESTI2005908, TESTI2005911, TESTI20Q5933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010513, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034940, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000014, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003012, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003446, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813, THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027125, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000074, TRACH1000100, TRACH1000106, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000981, TRACH2000986, TRACH2001021, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001443, TRACH2001474, TRACH2001478, TRACH2001525, TRACH2001532, TRACH2001549, TRACH2001592, TRACH2001596, TRACH2001612, TRACH2001621, TRACH2001646, TRACH2001651, TRACH2001684, TRACH2001810, TRACH2001898, TRACH2001933, TRACH2001968, TRACH2001996, TRACH2002043, TRACH2002054, TRACH2002100, TRACH2002138, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014124, TRACH2014268, TRACH2014284, TRACH2014337, TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015087, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the BGGI1, BLADE, BNGH4, BRACE, BRALZ, BRAMY, and BRASW libraries were analyzed for expression frequency. The analysis results are described below. Each is a relative value, where the expression level over all tissues is taken as 100. The larger this value, the greater the level of expression.

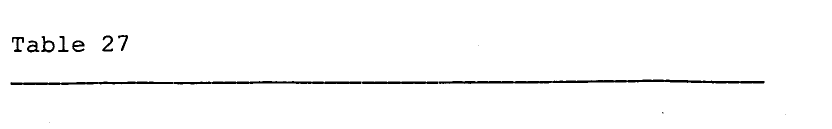

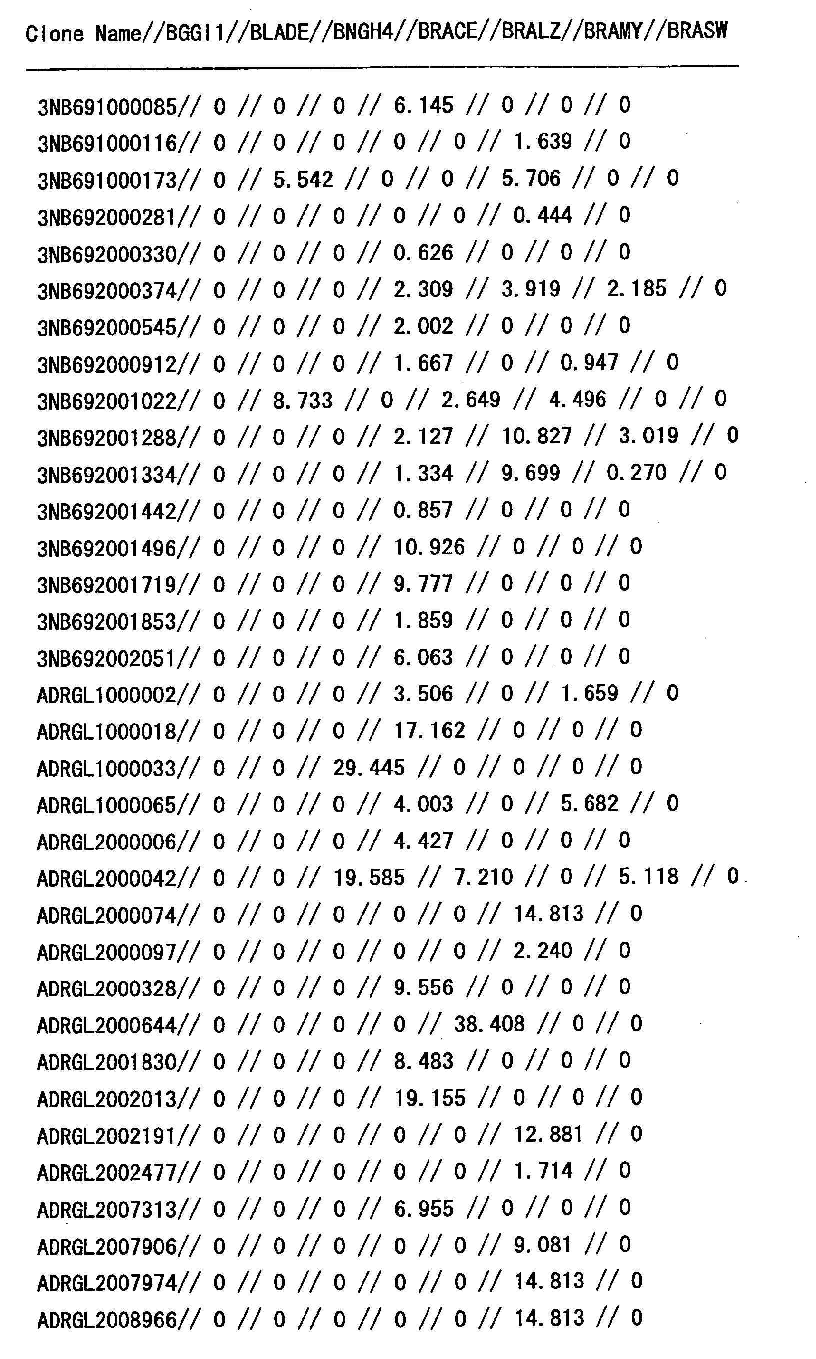

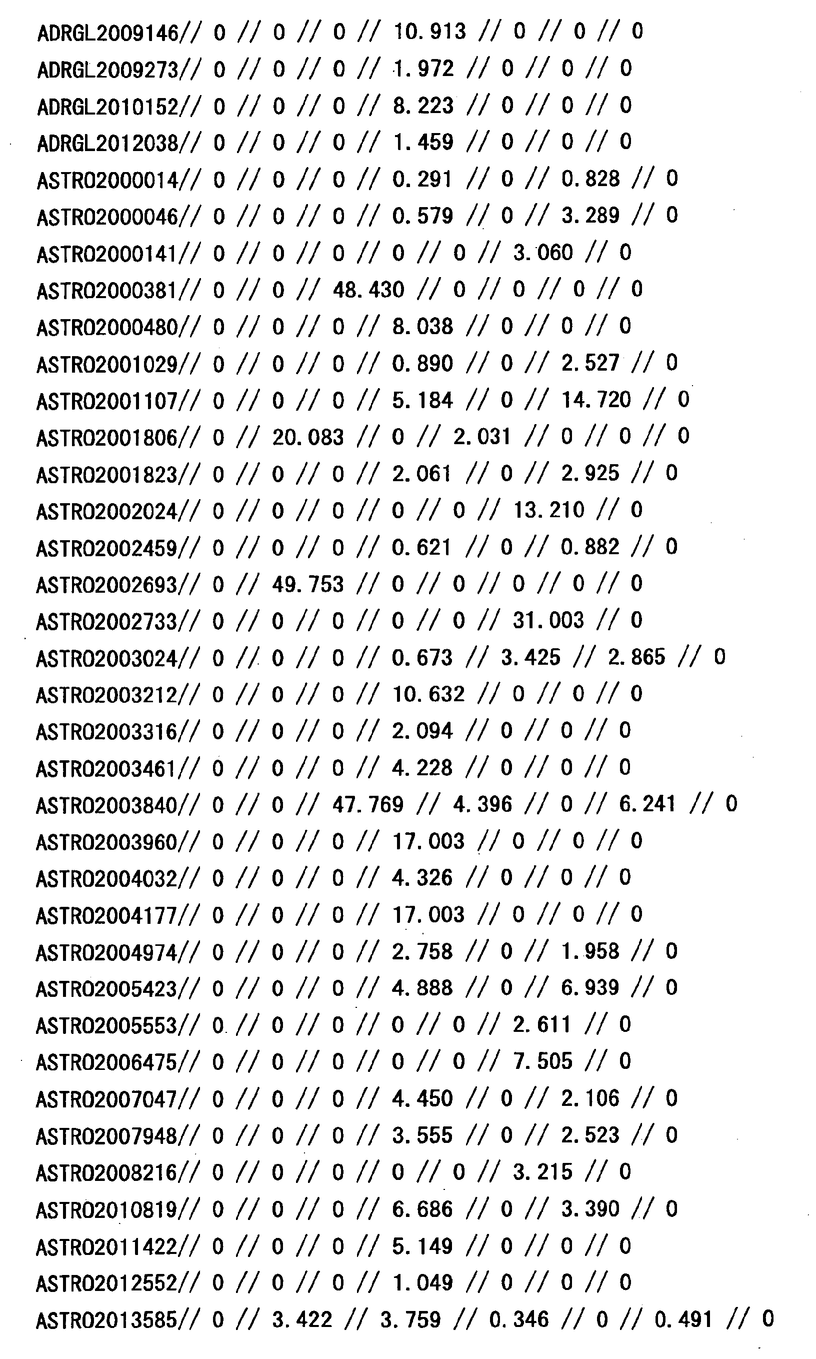

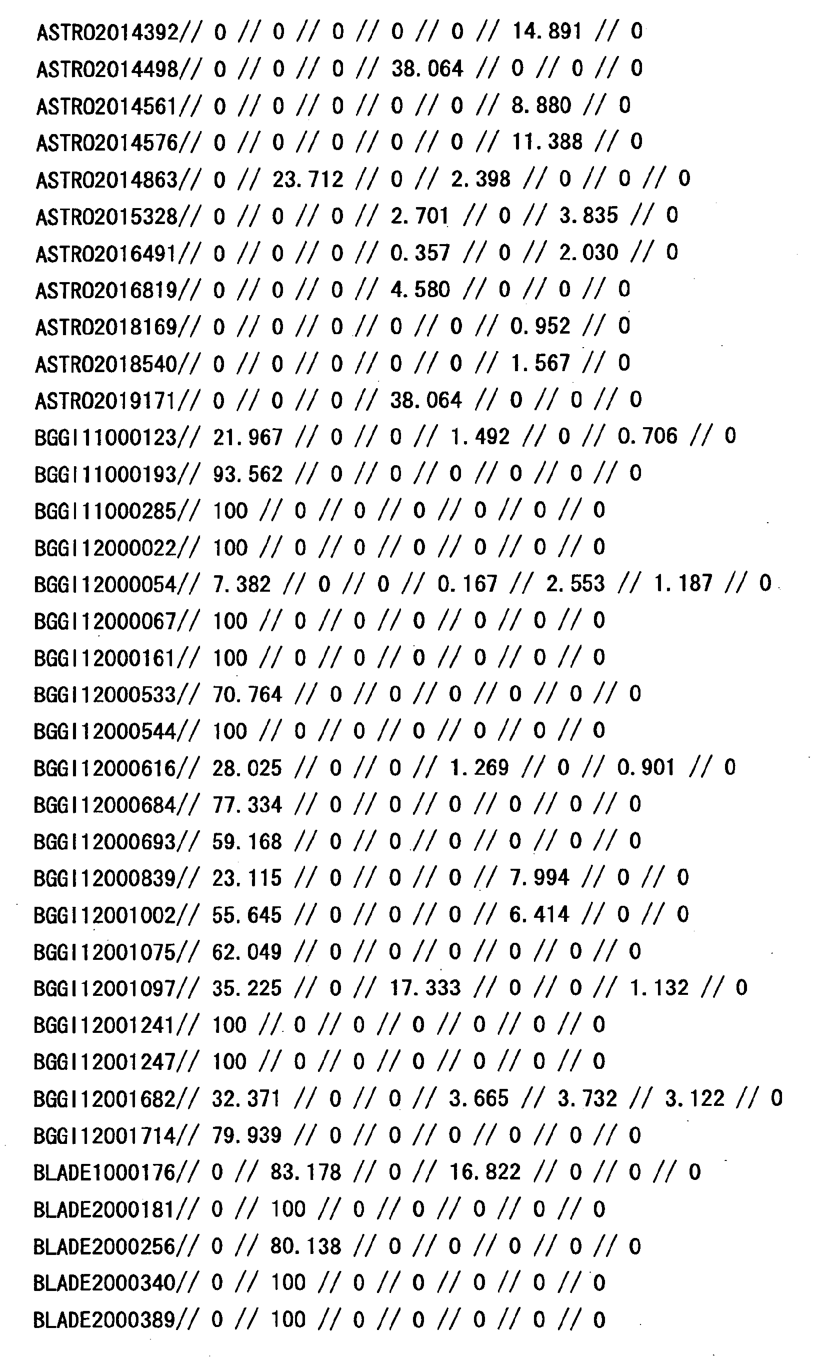

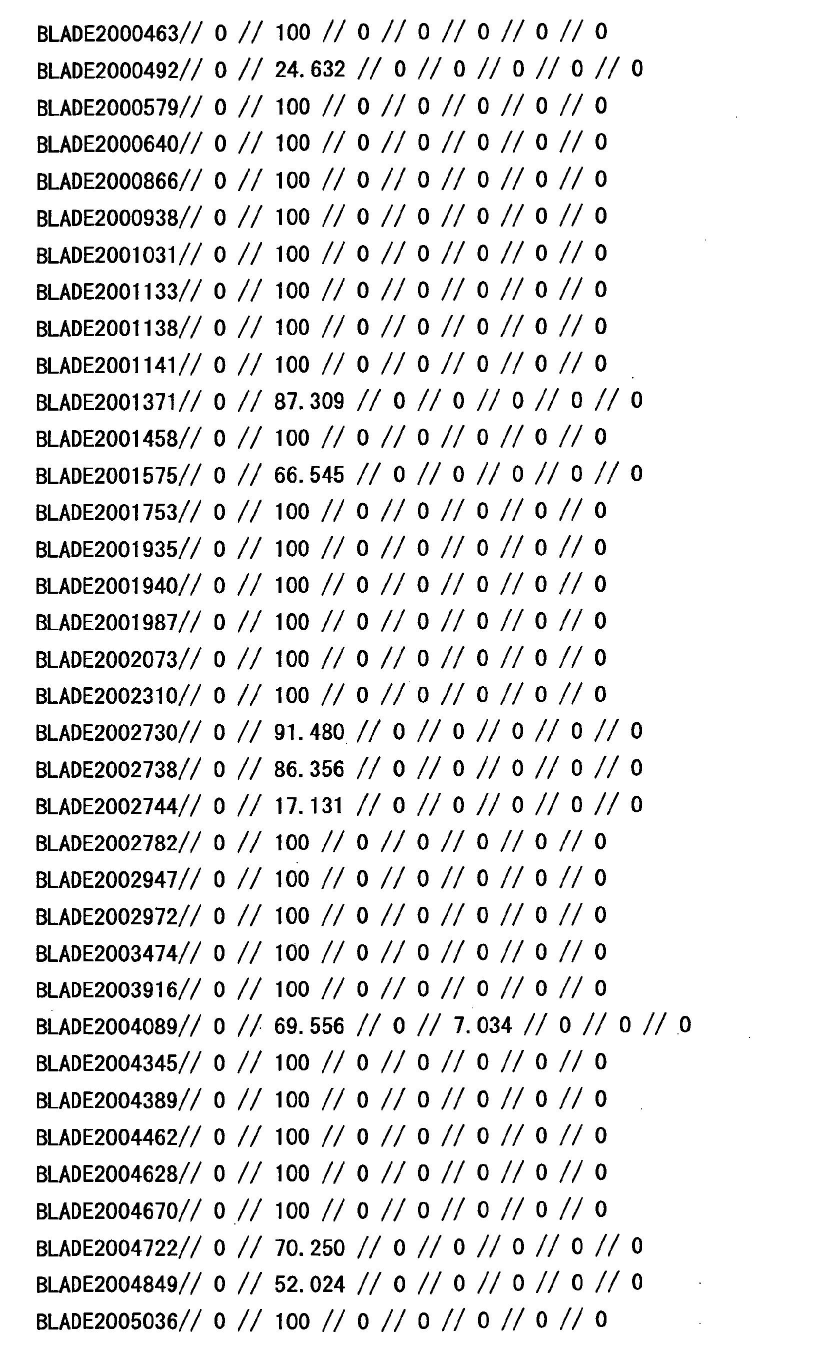

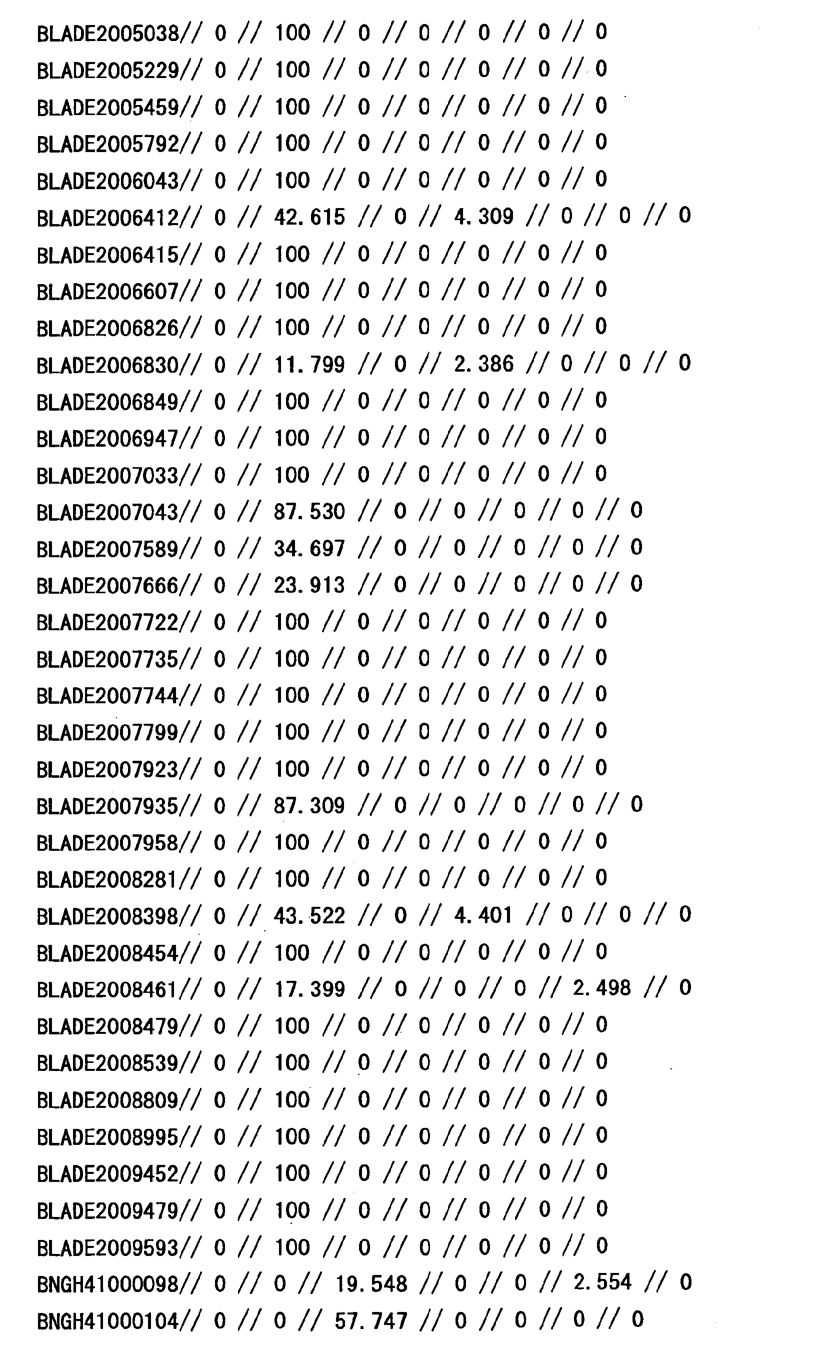

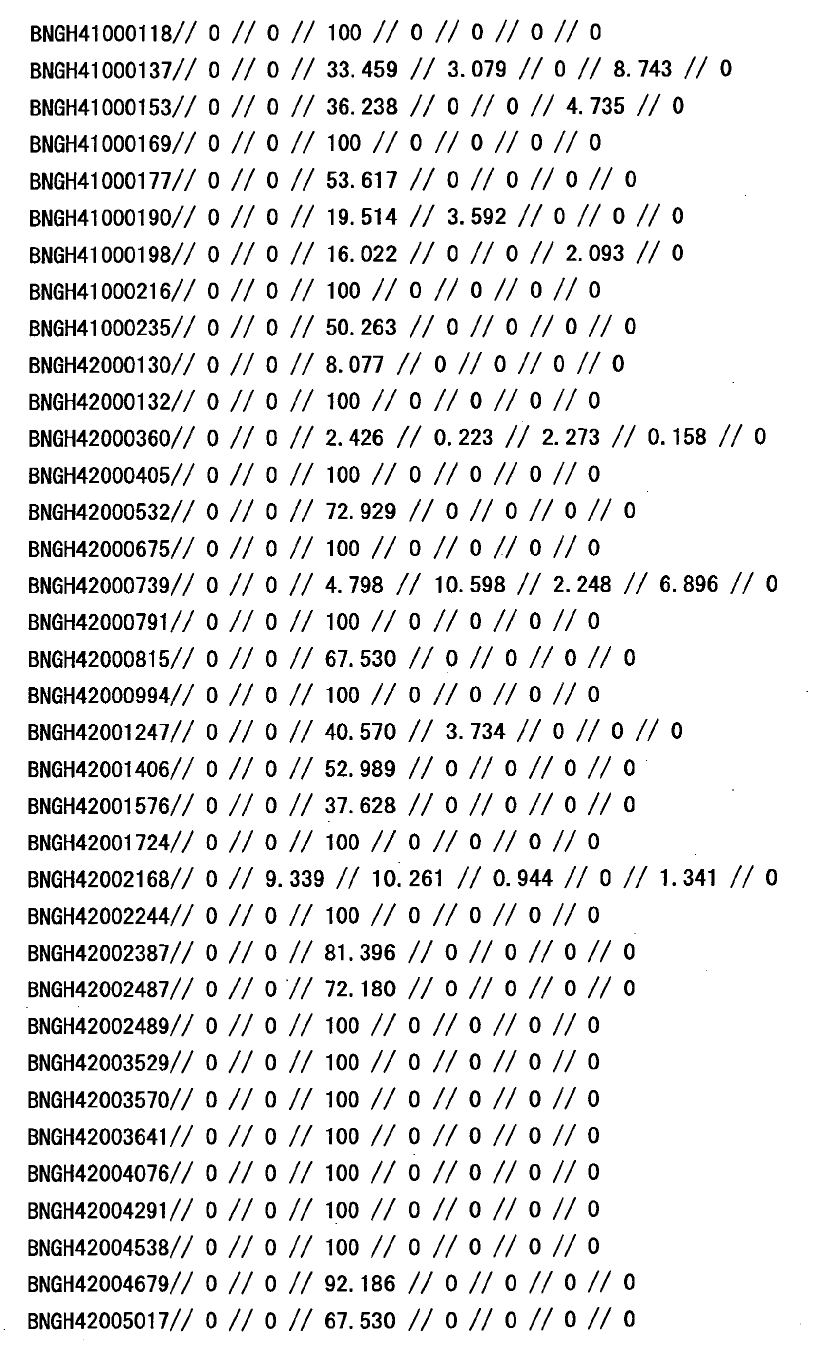

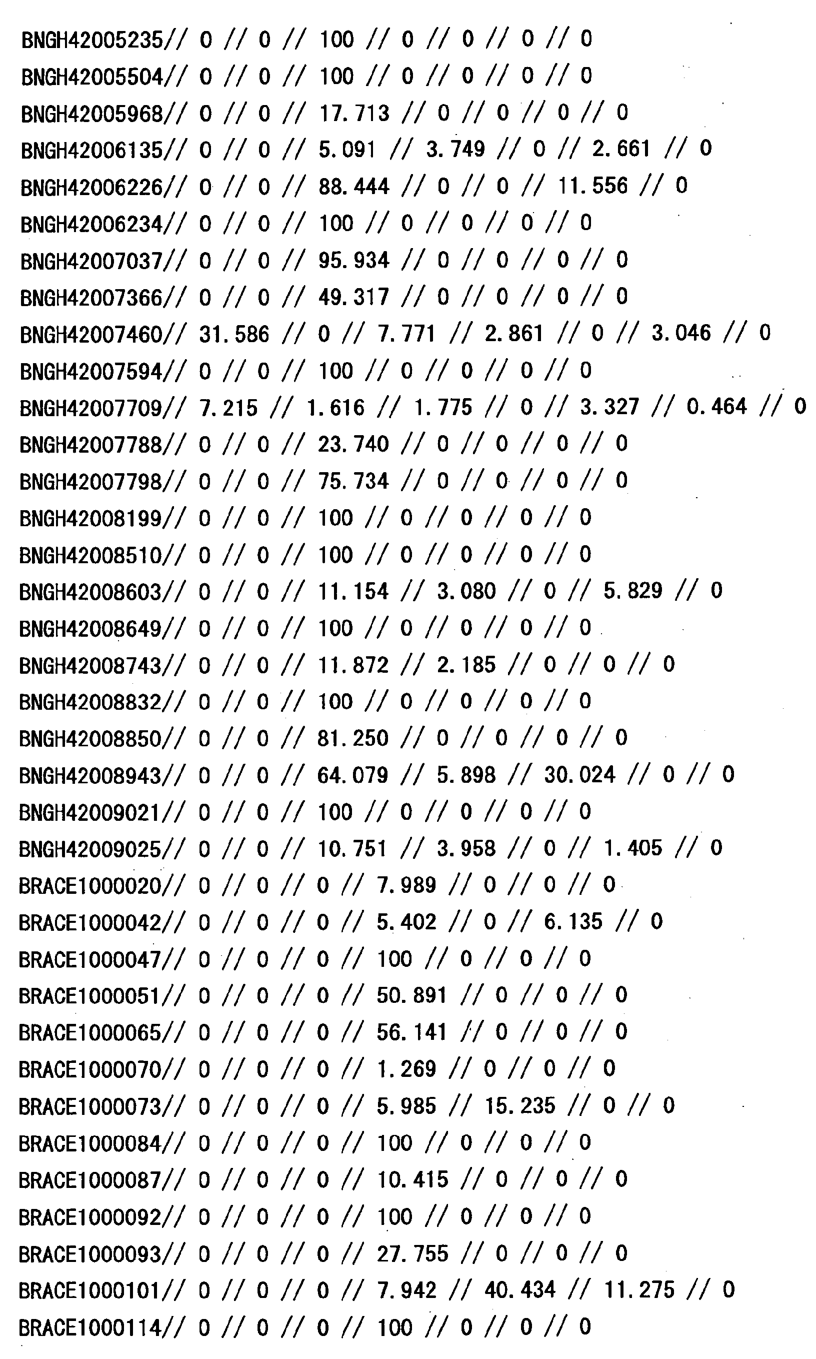

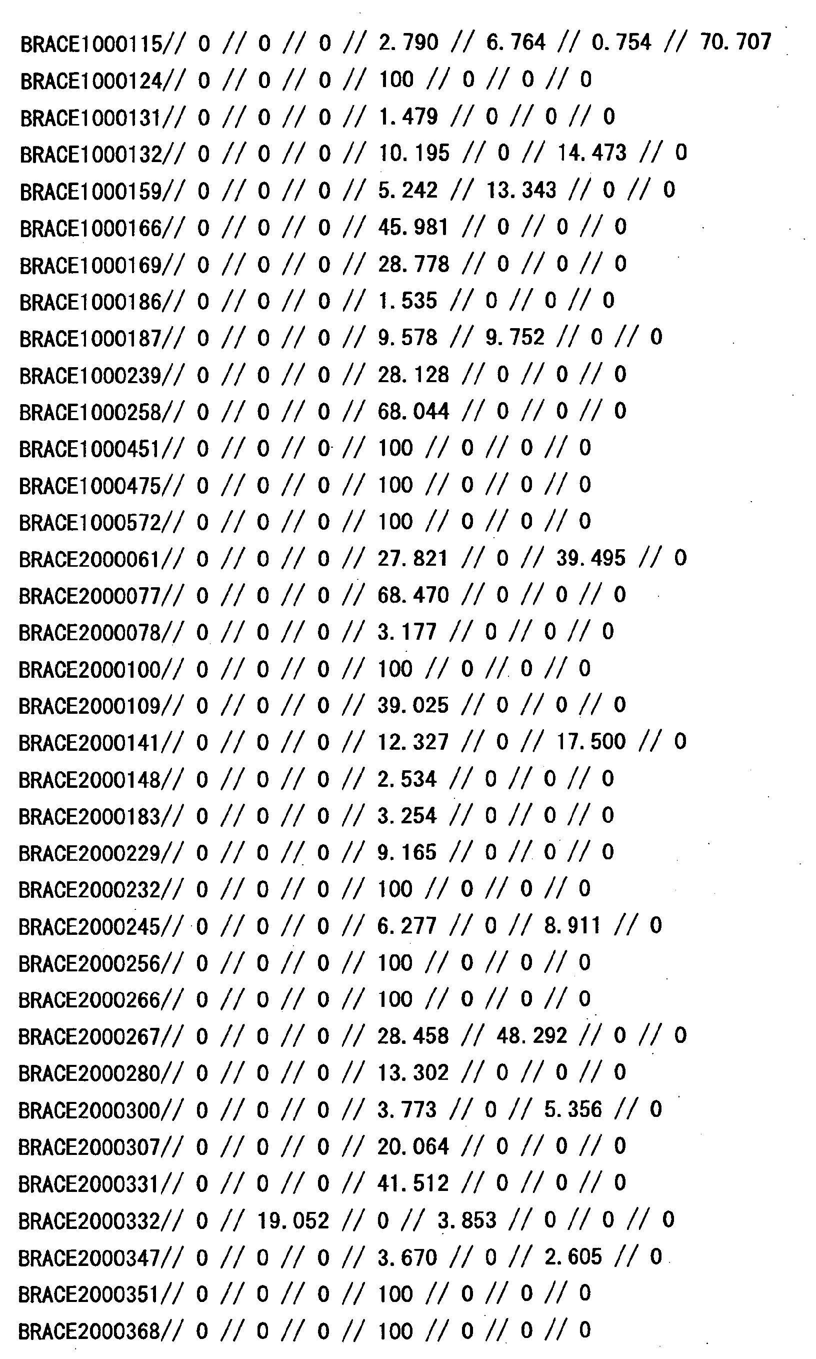

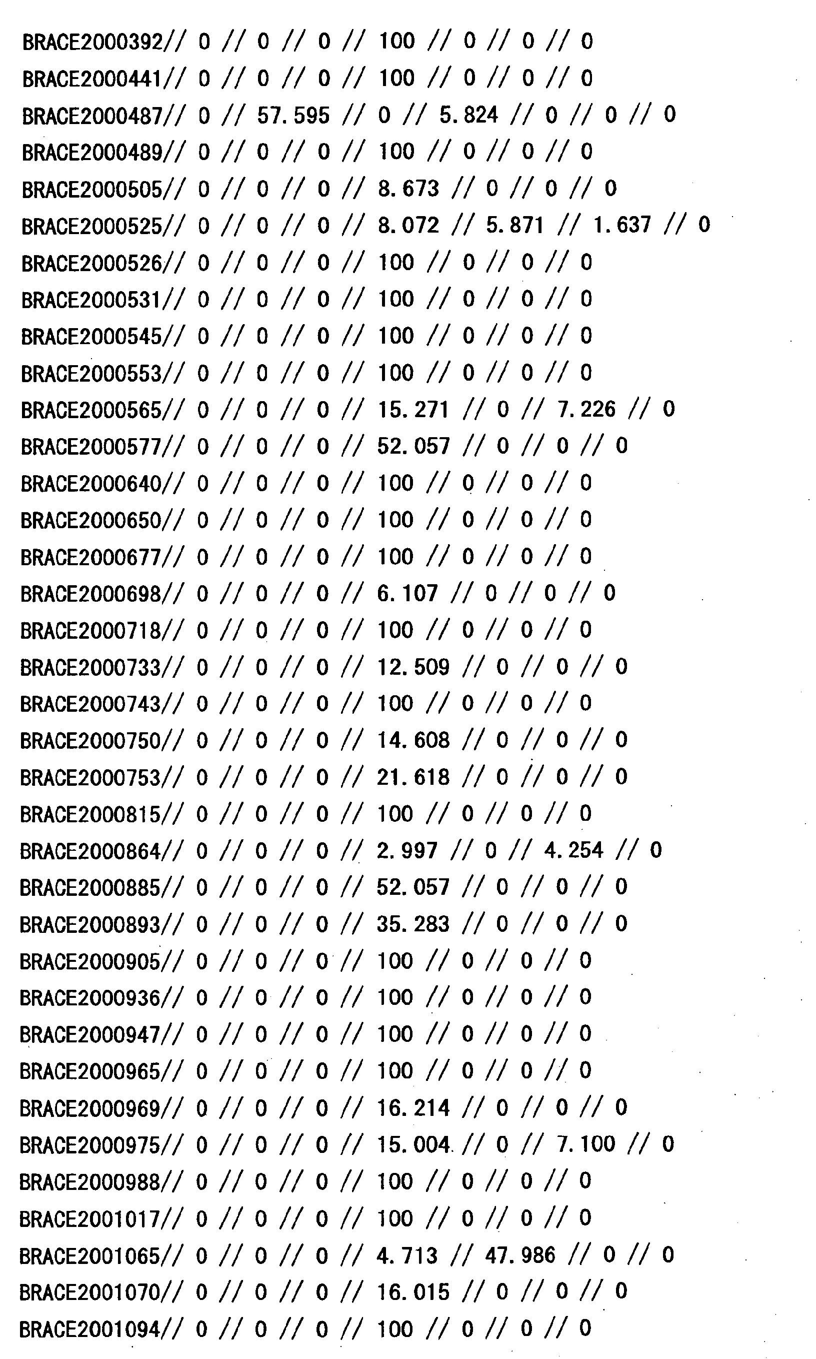

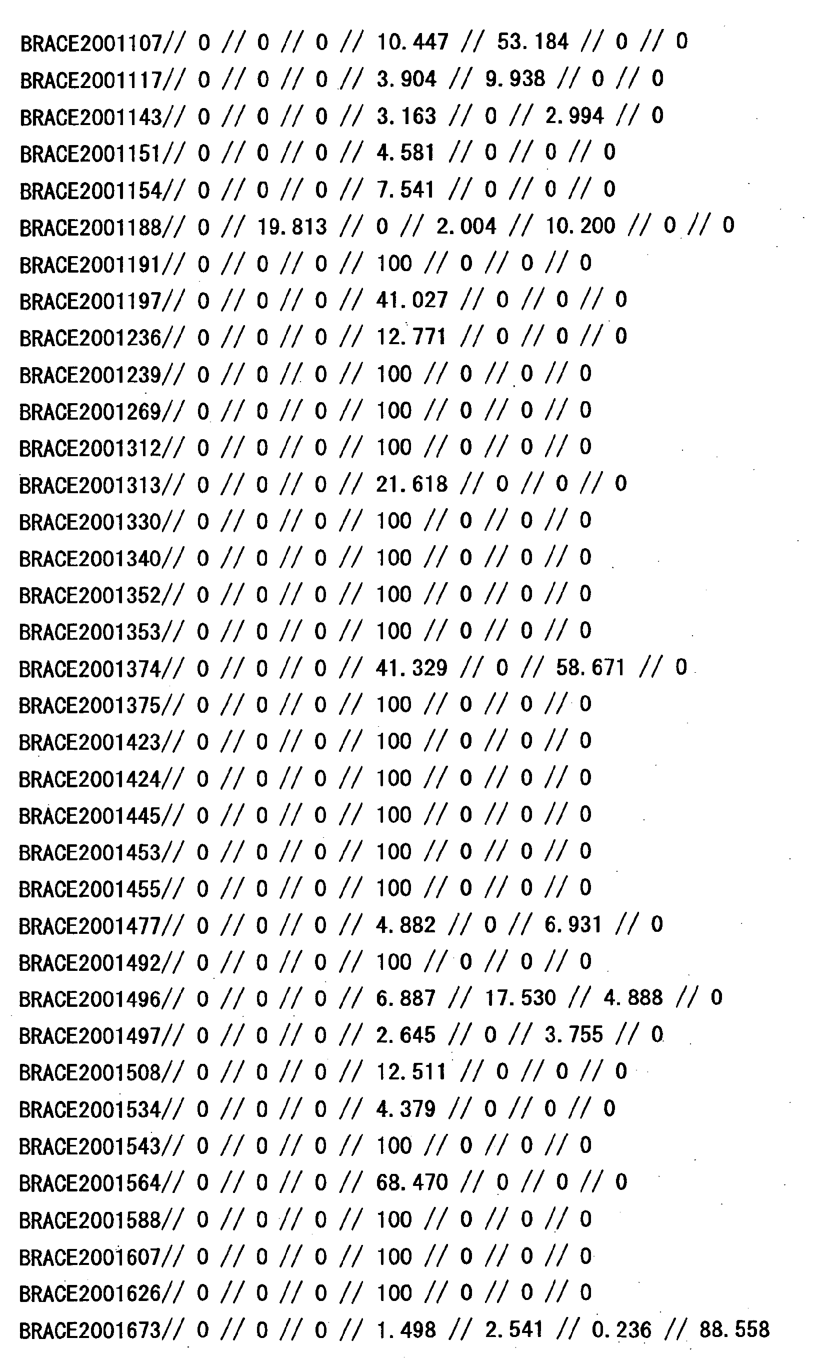

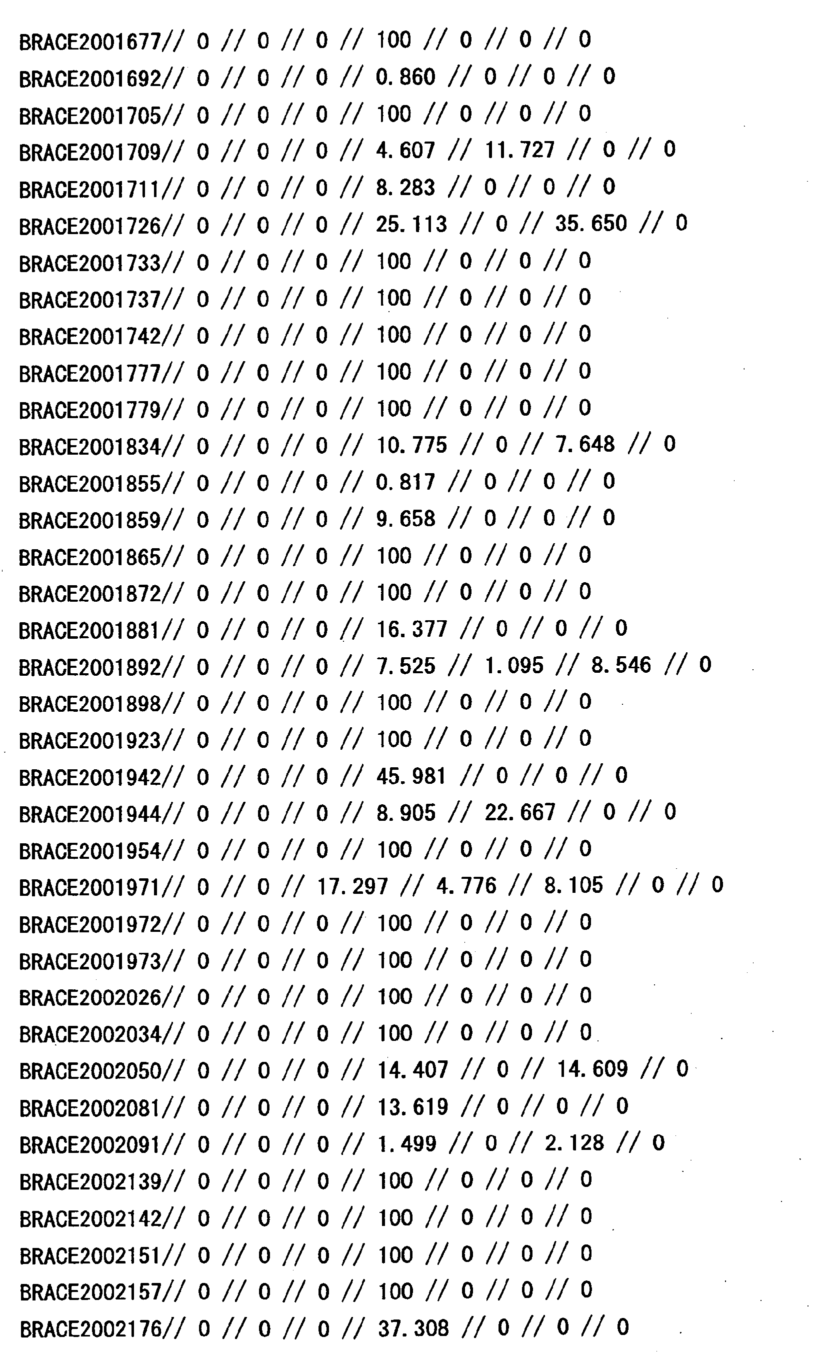

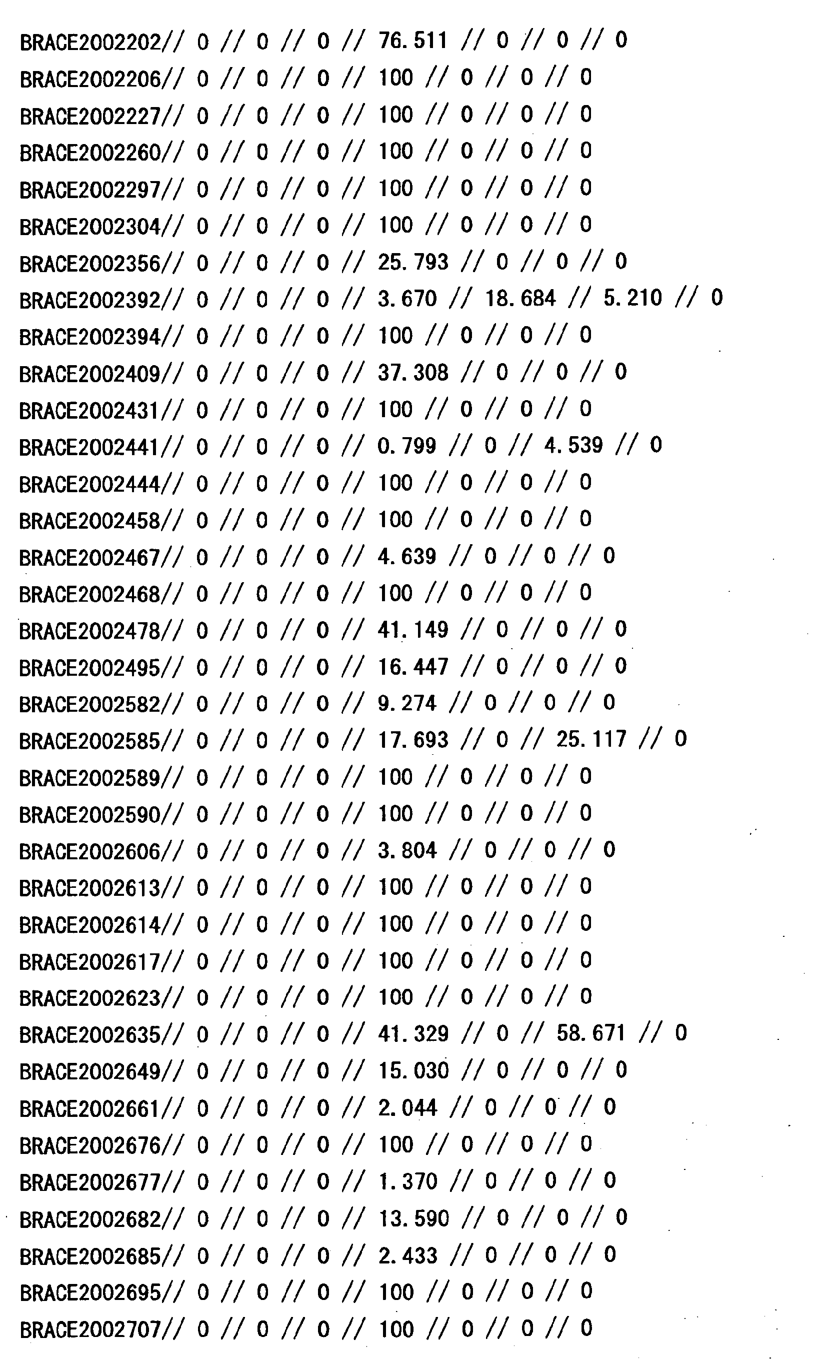

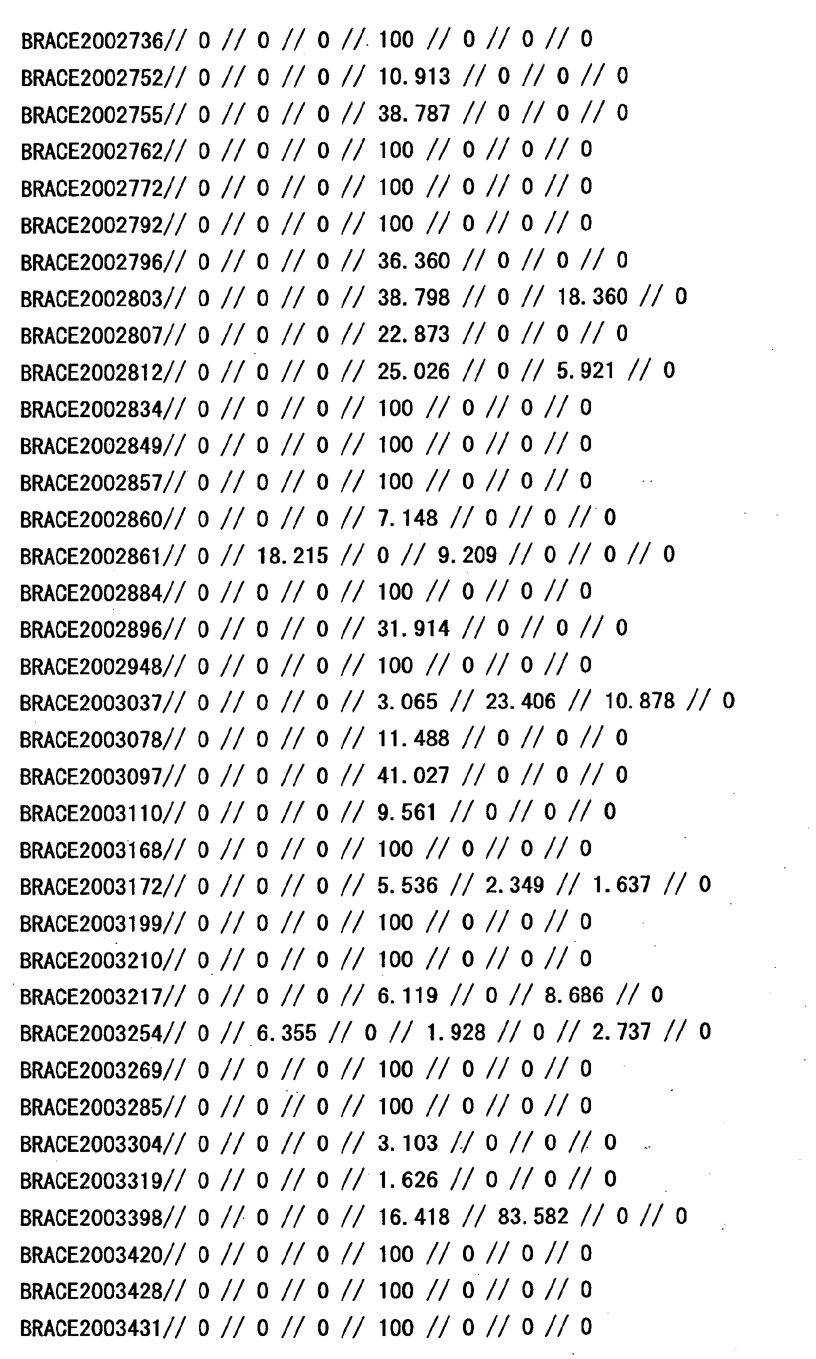

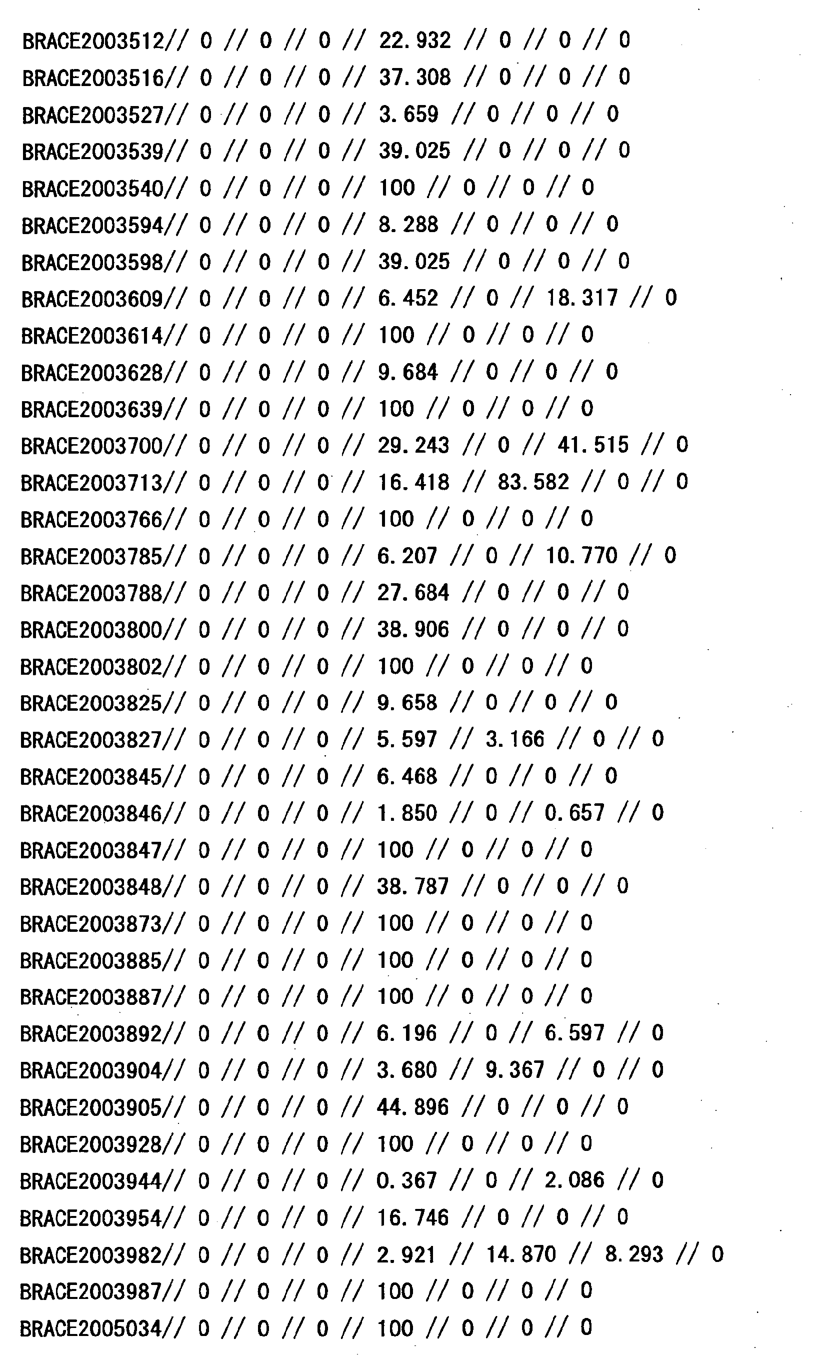

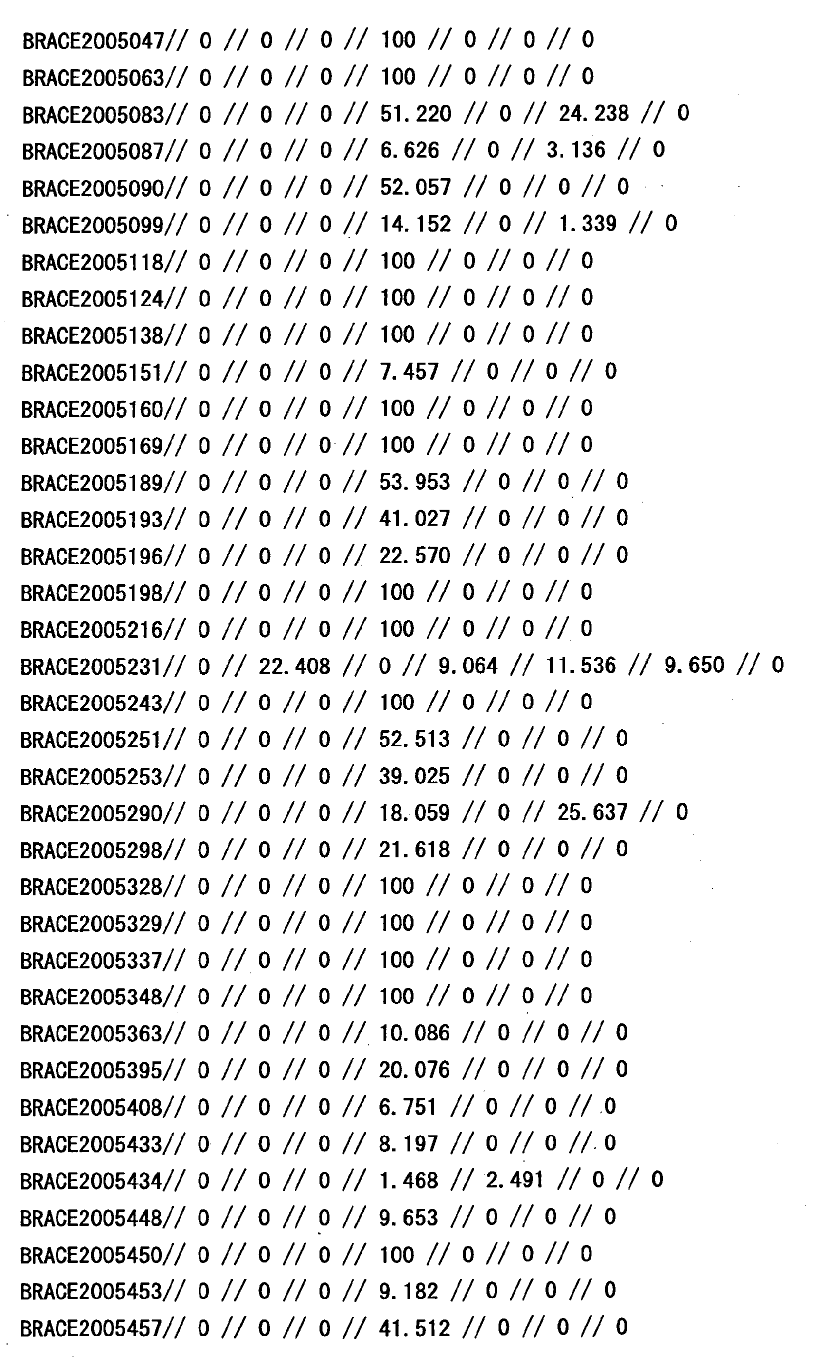

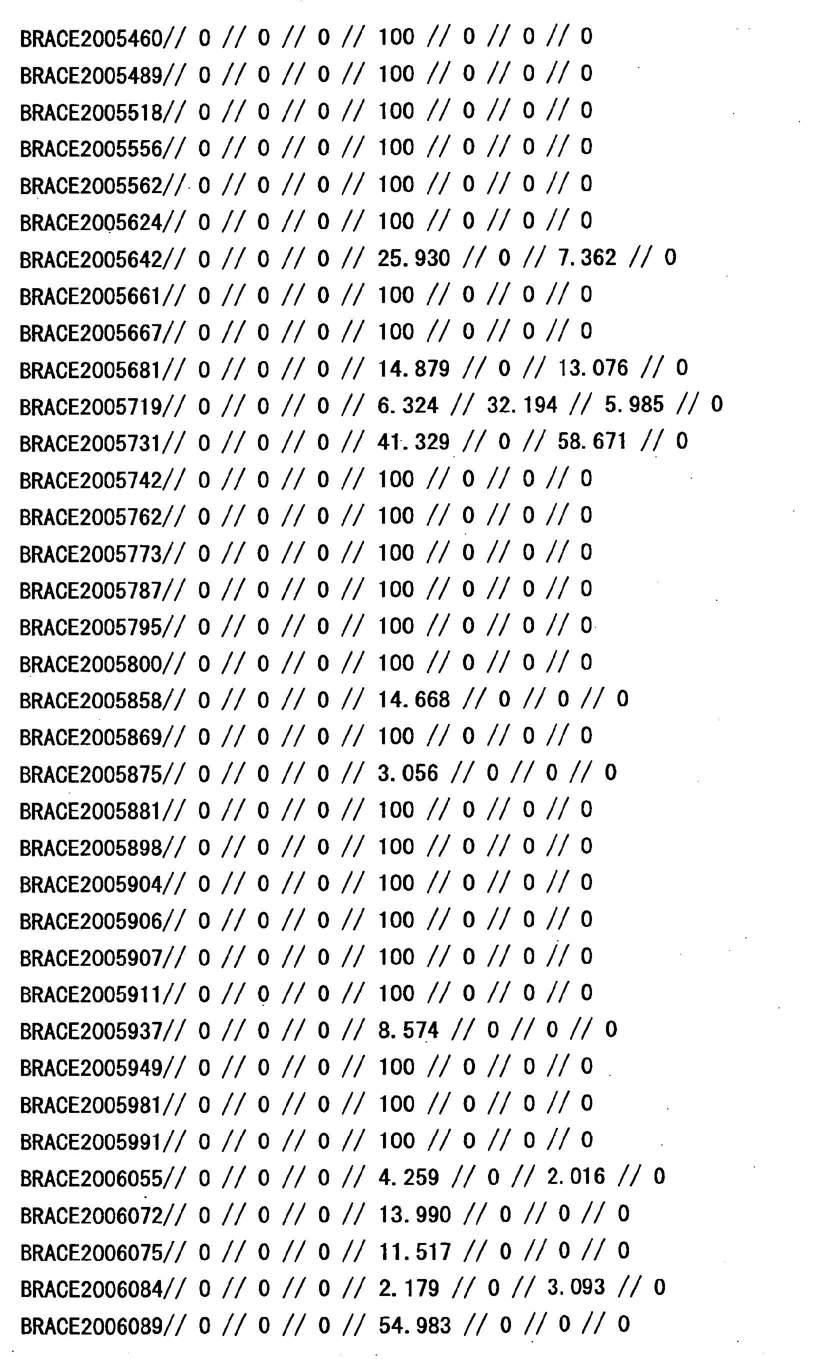

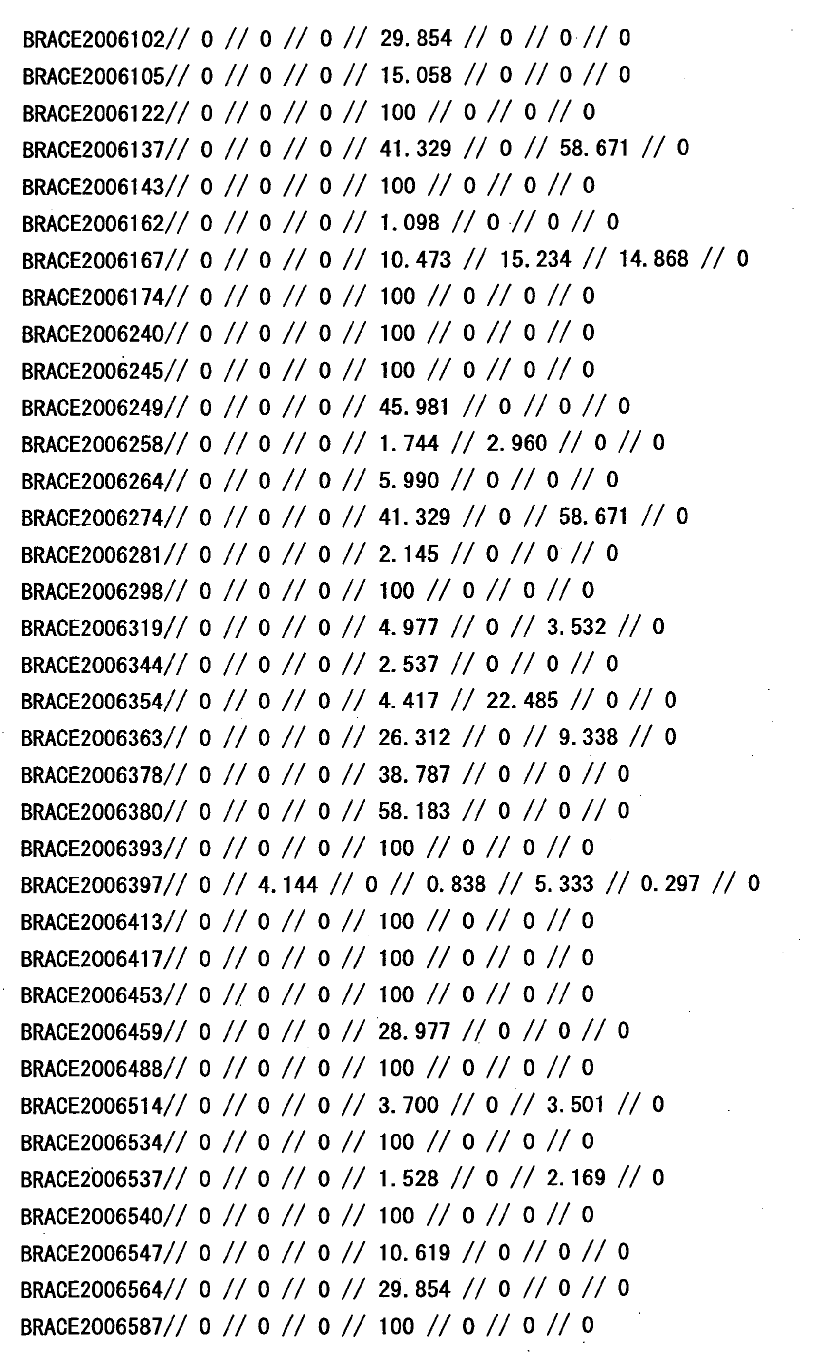

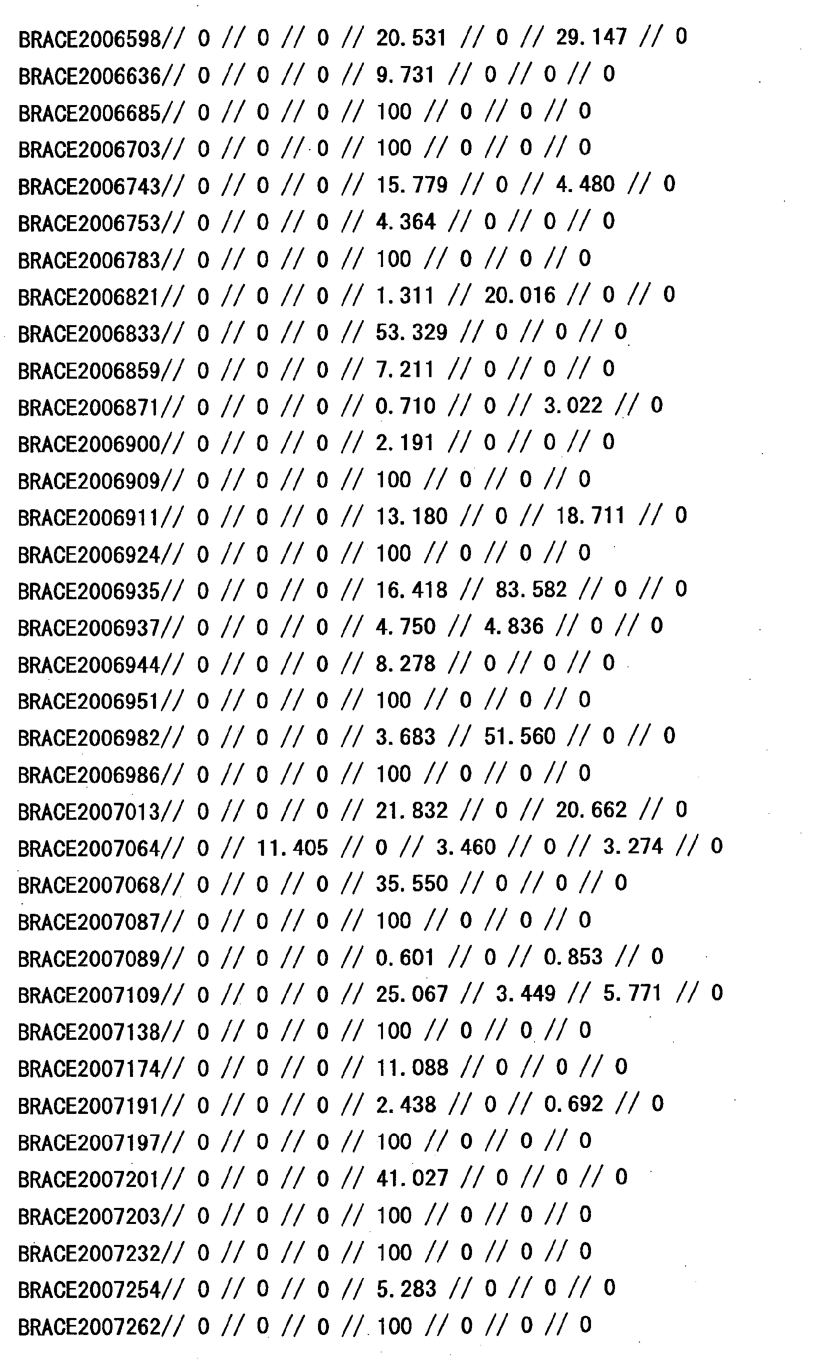

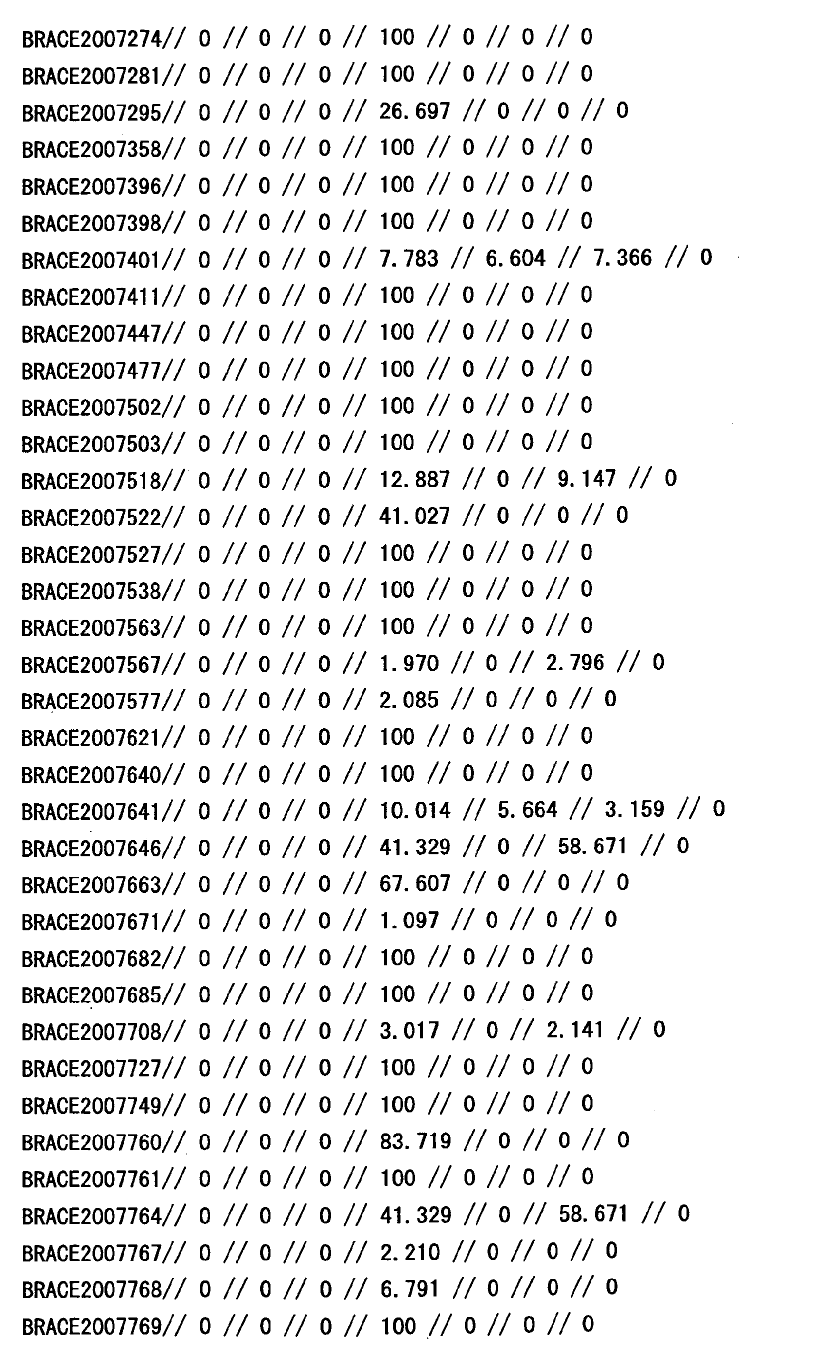

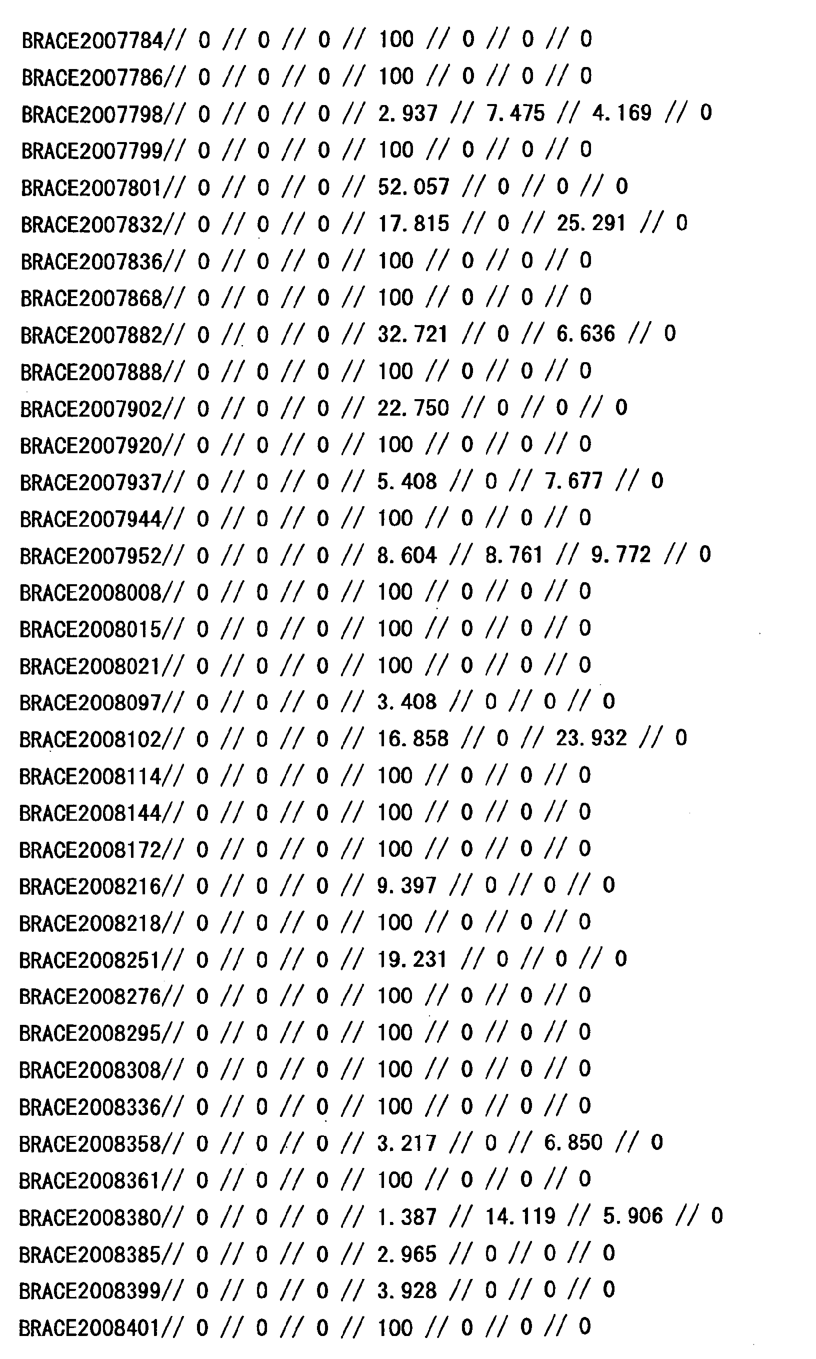

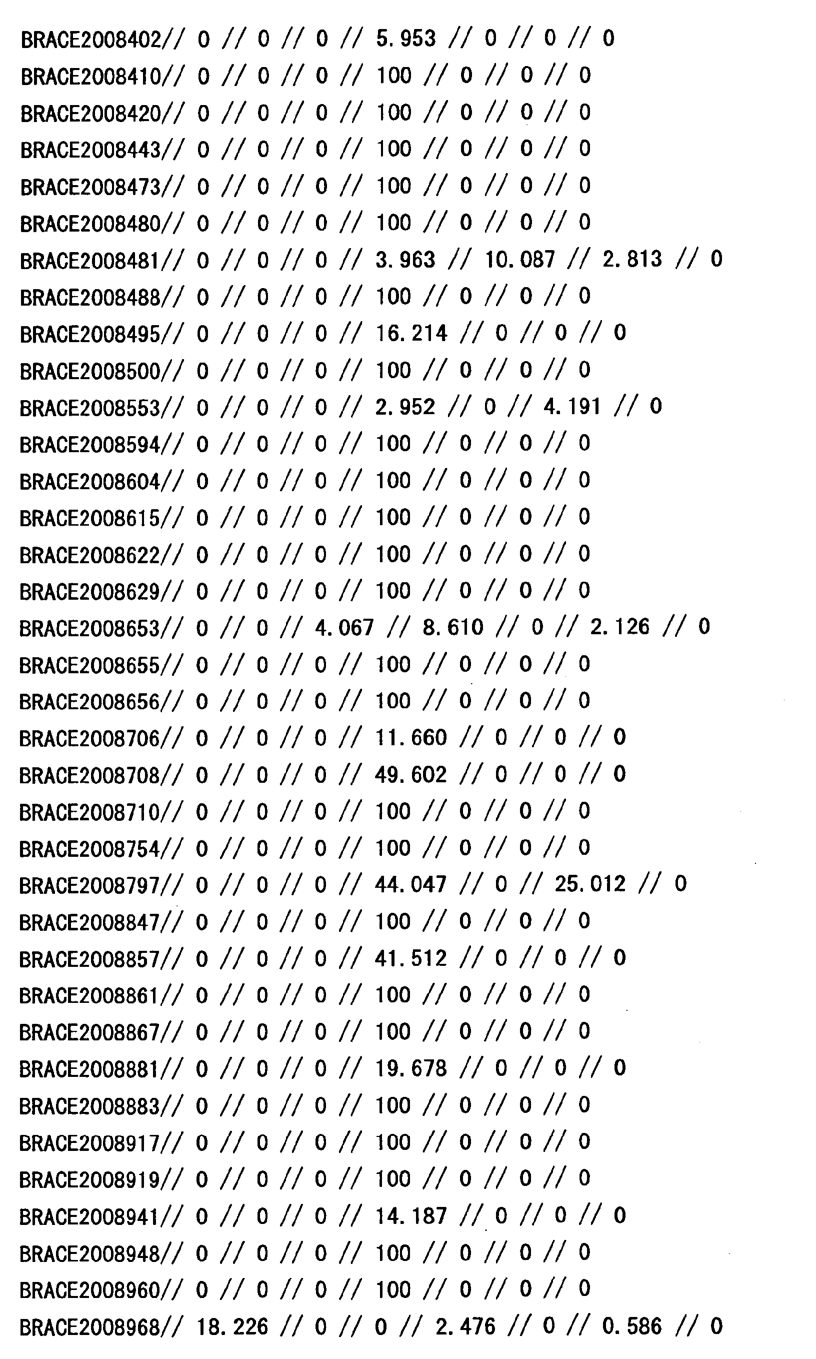

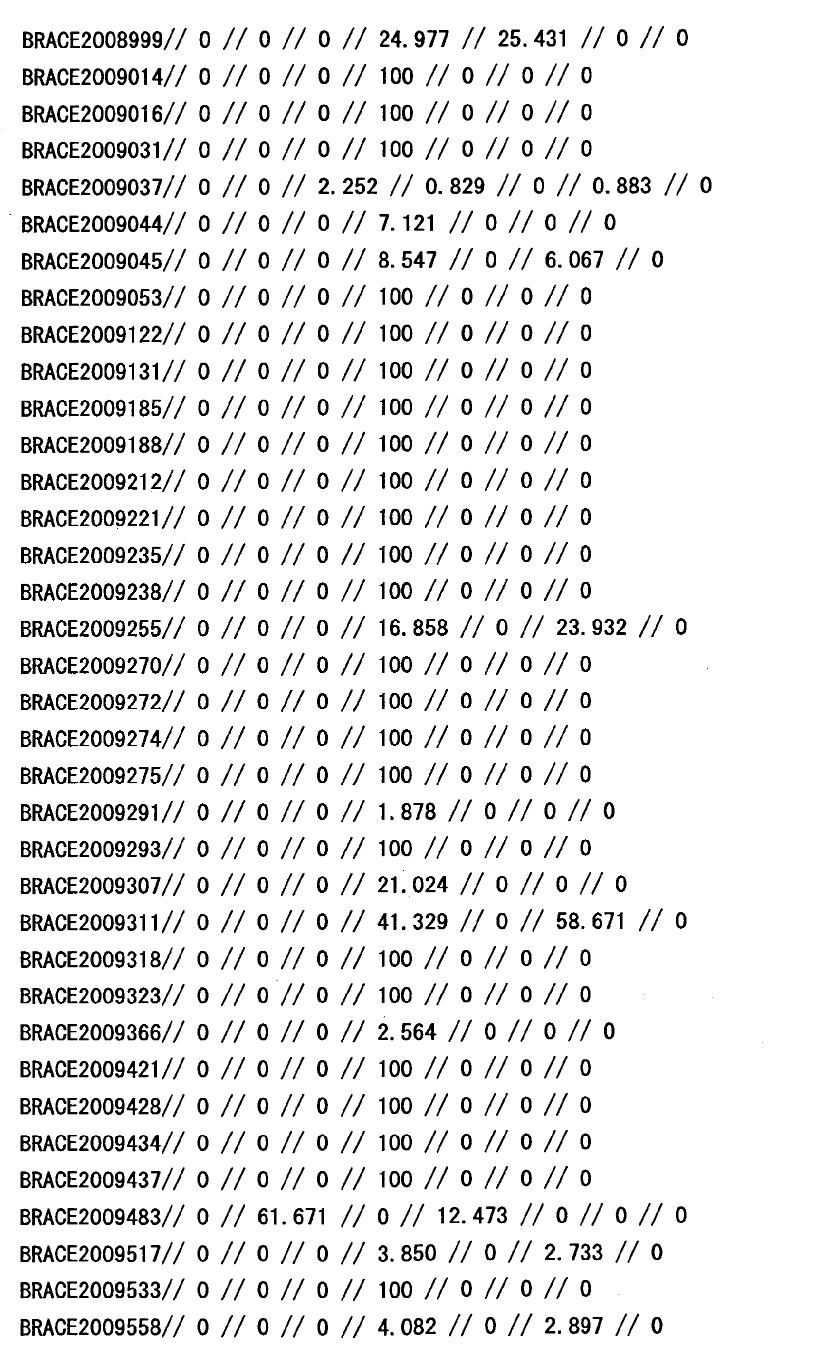

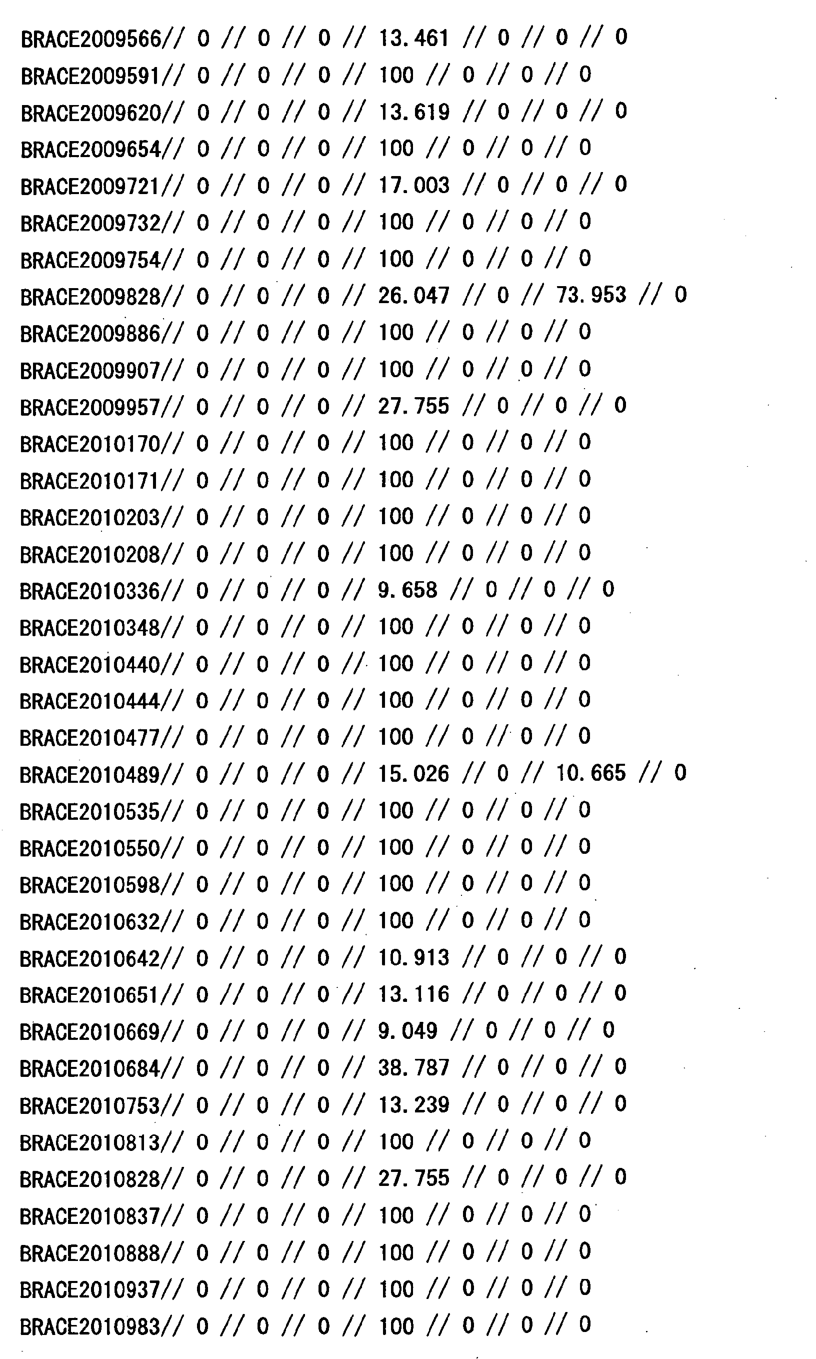

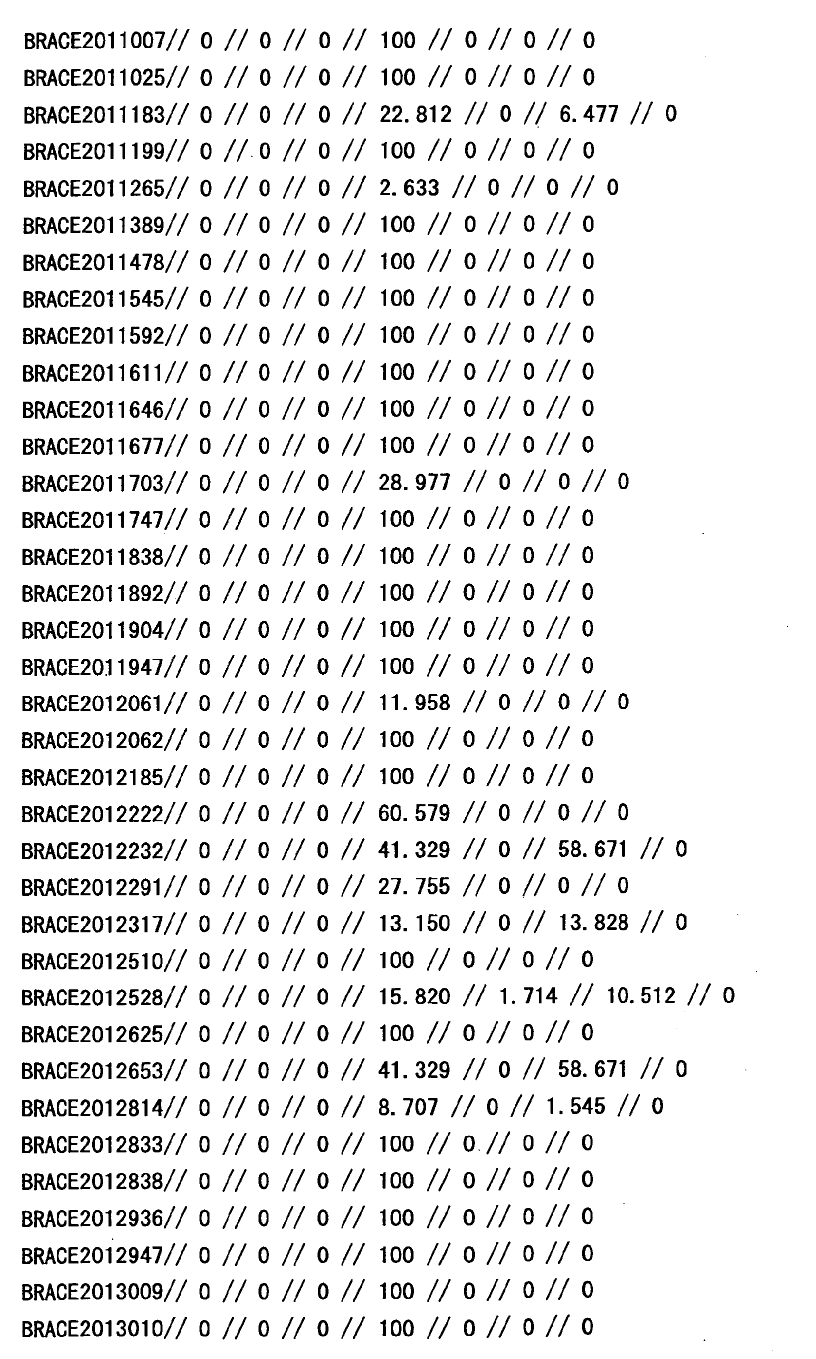

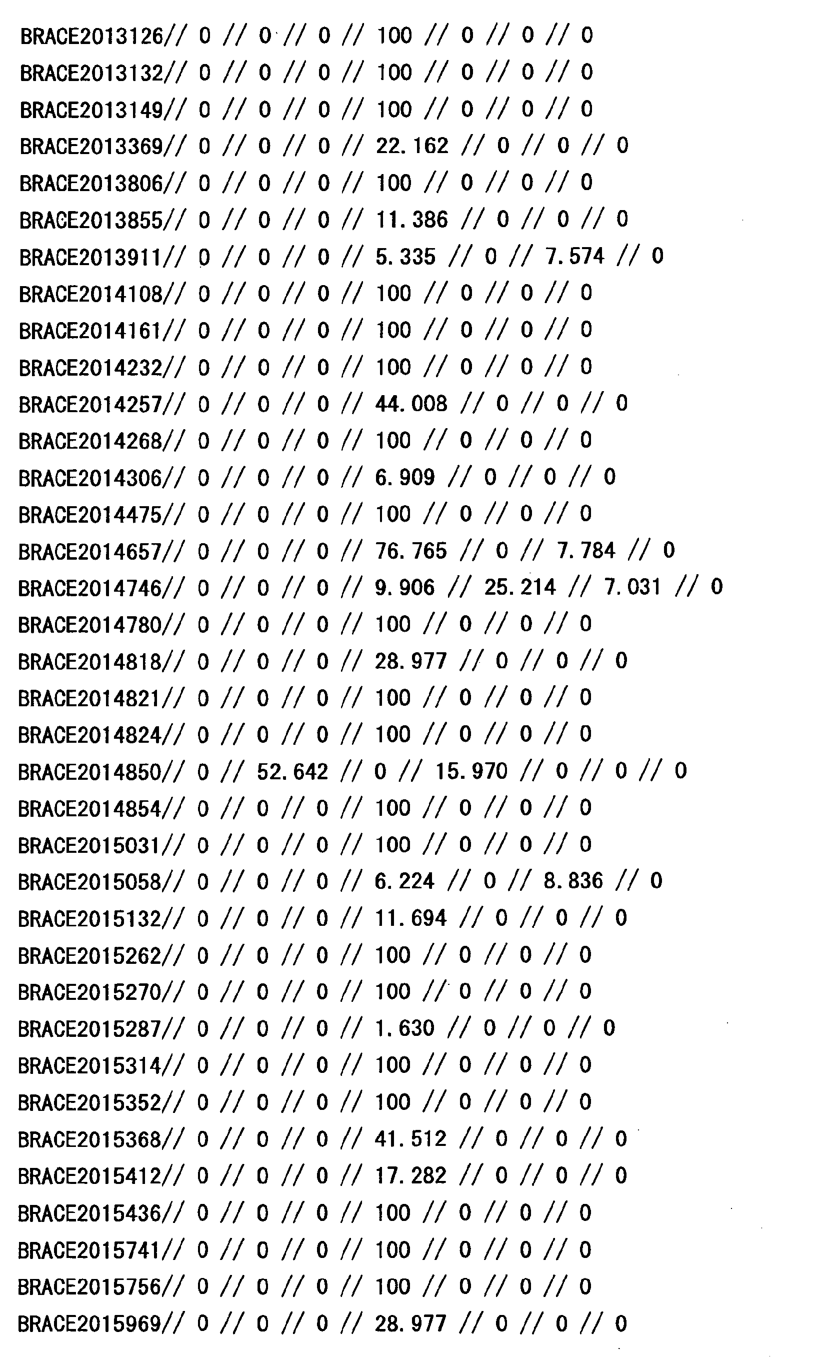

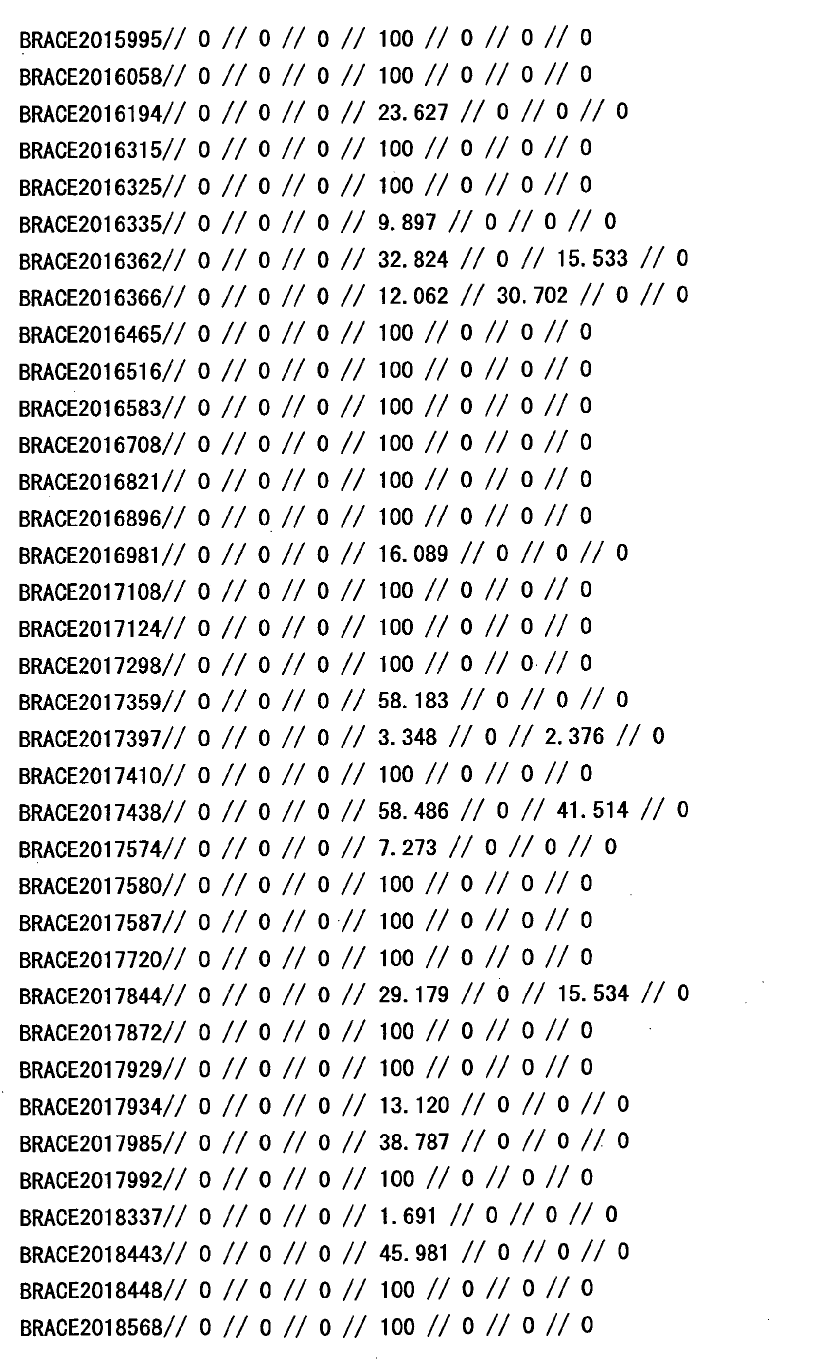

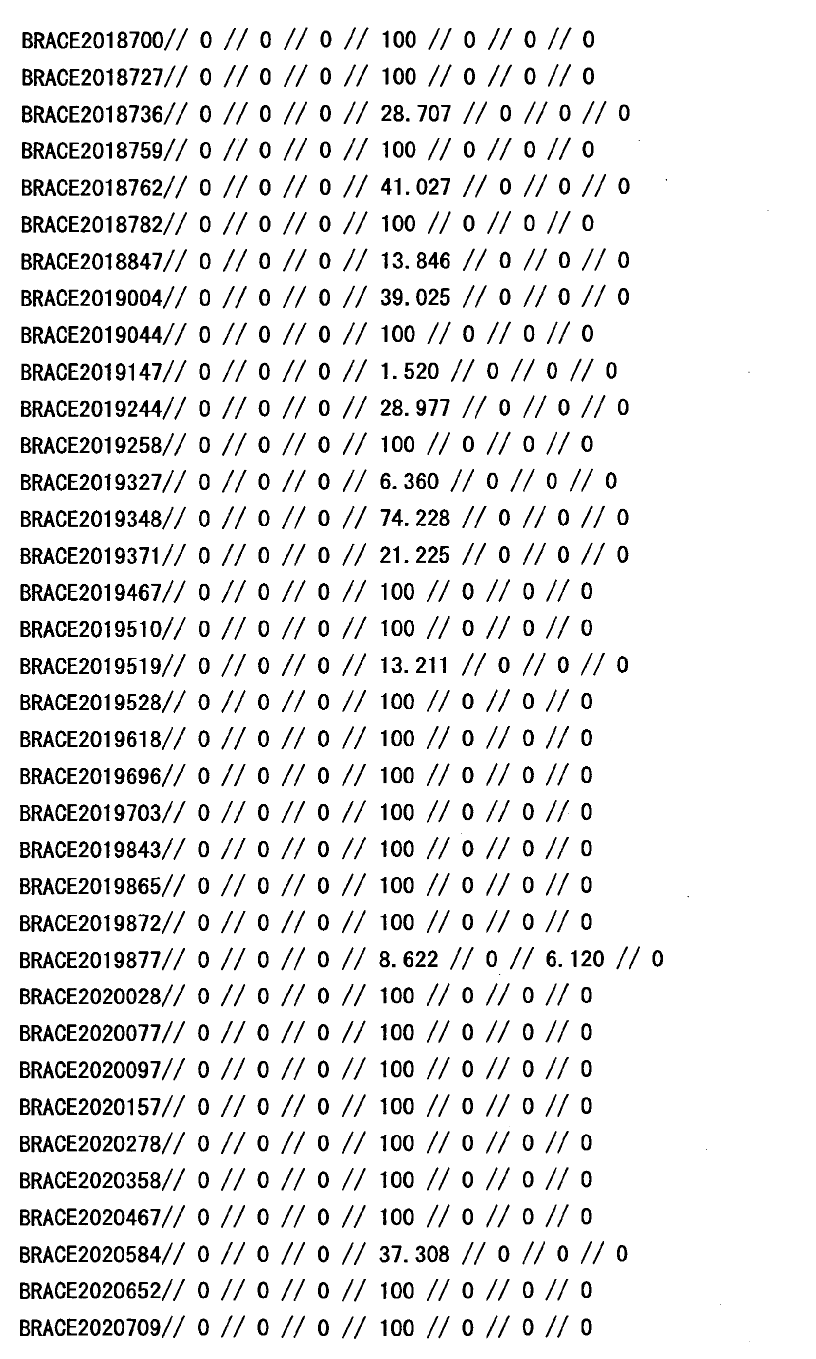

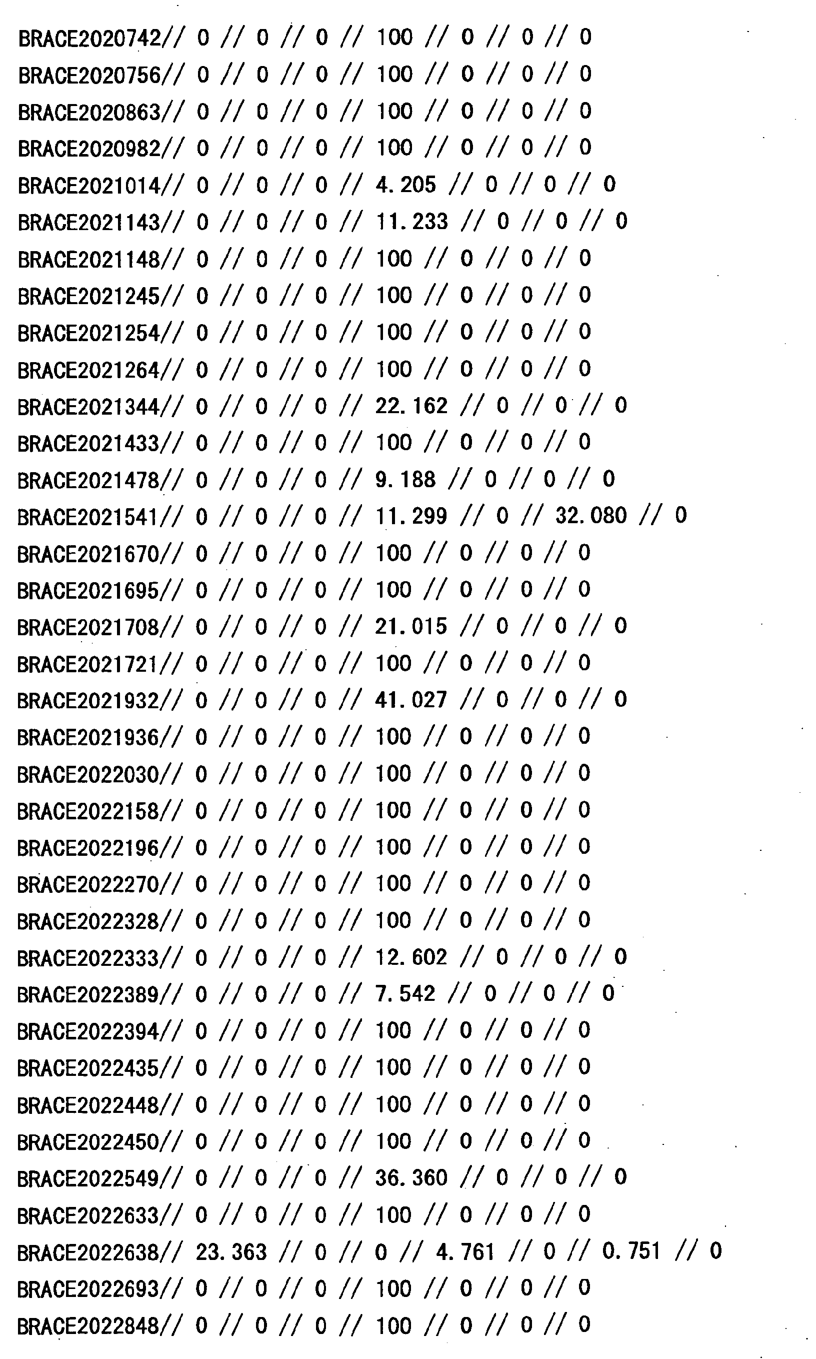

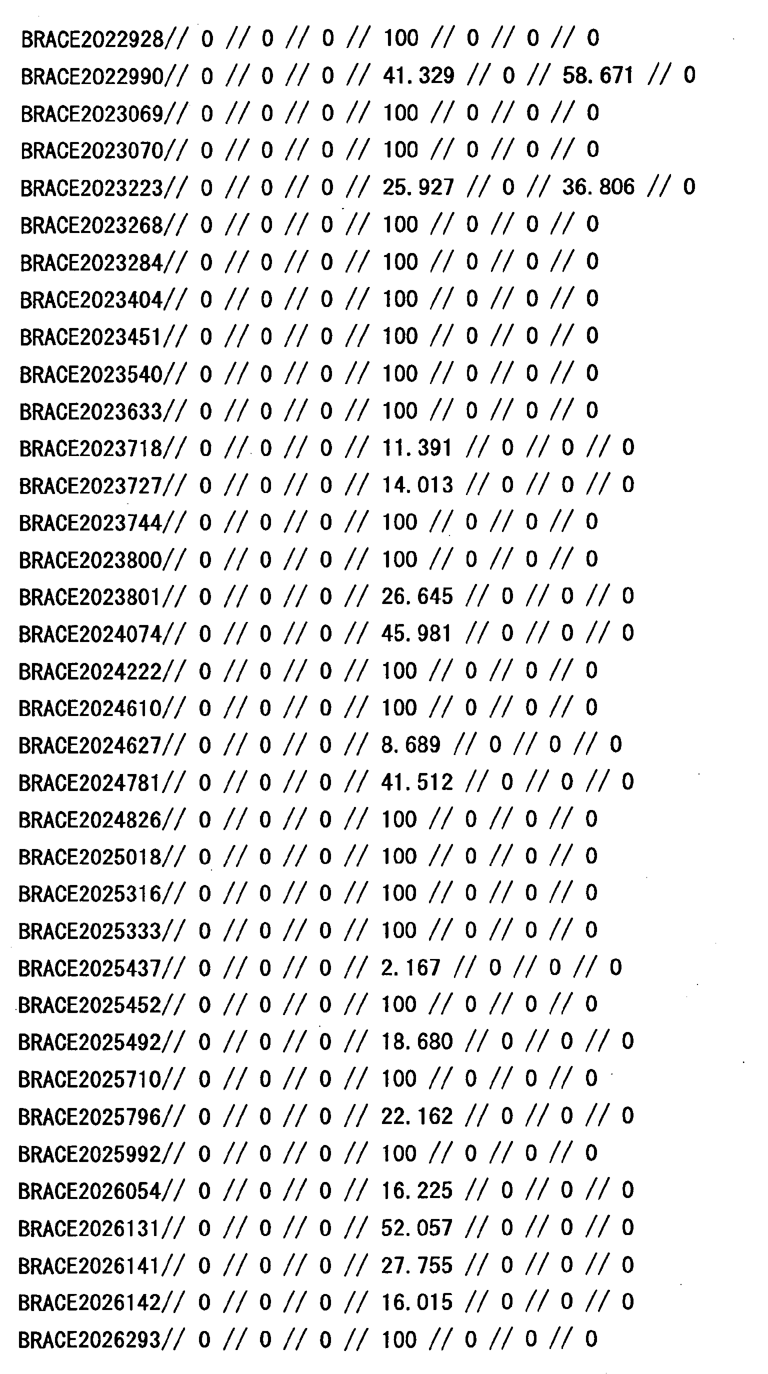

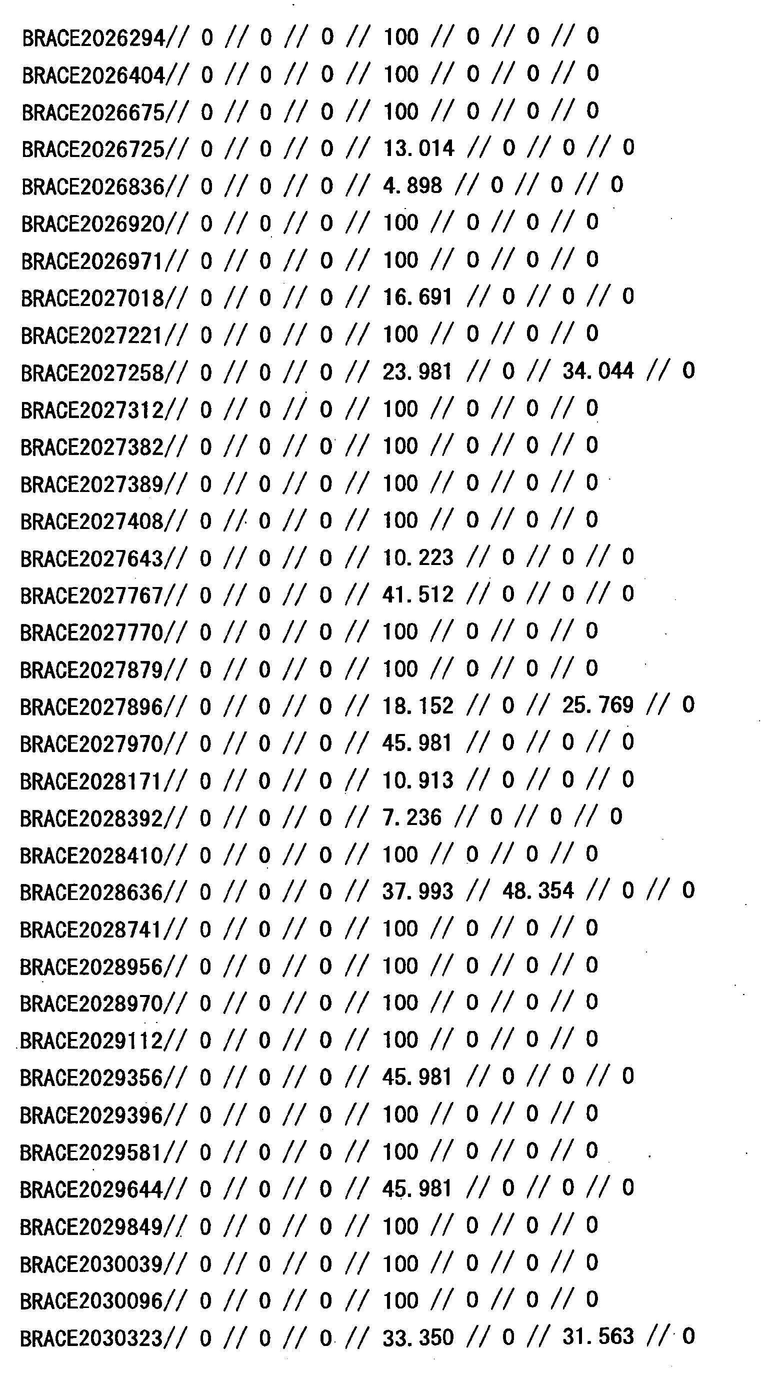

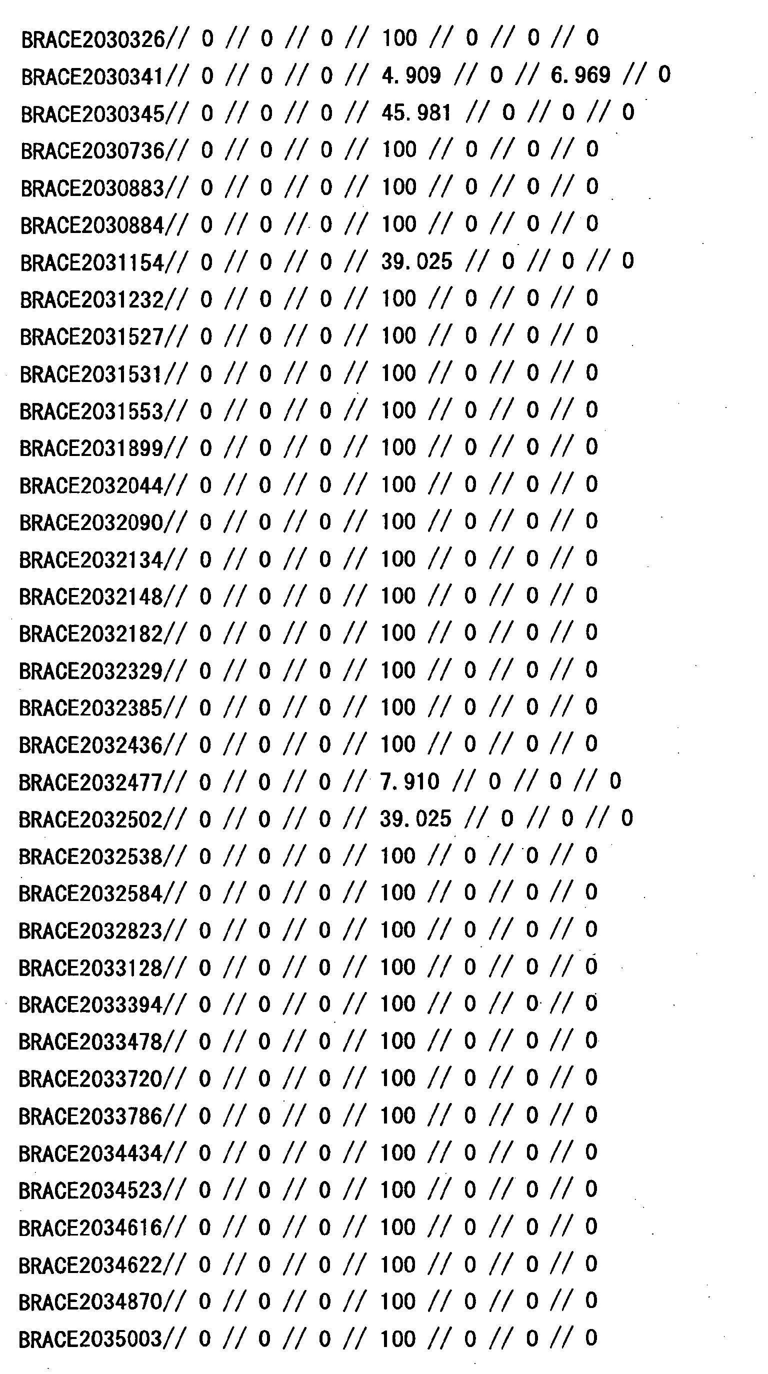

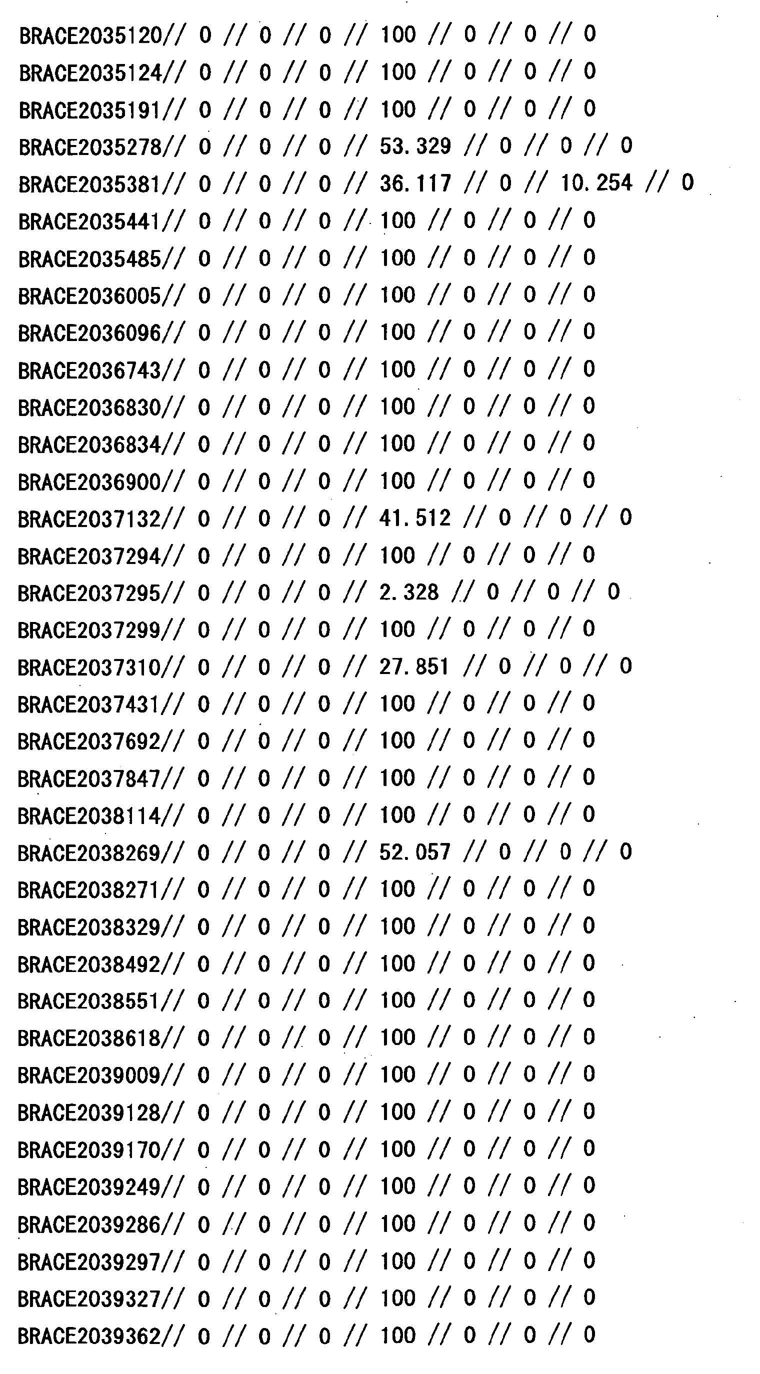

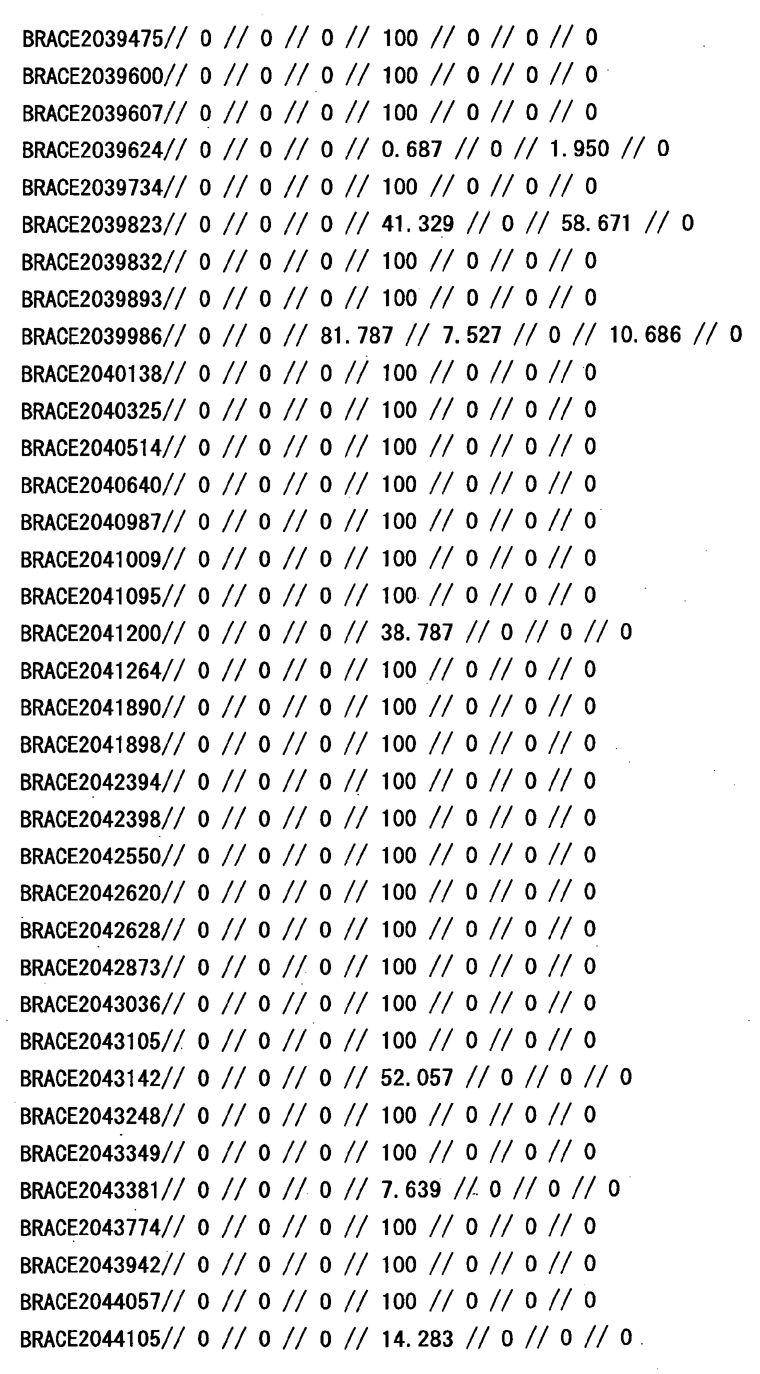

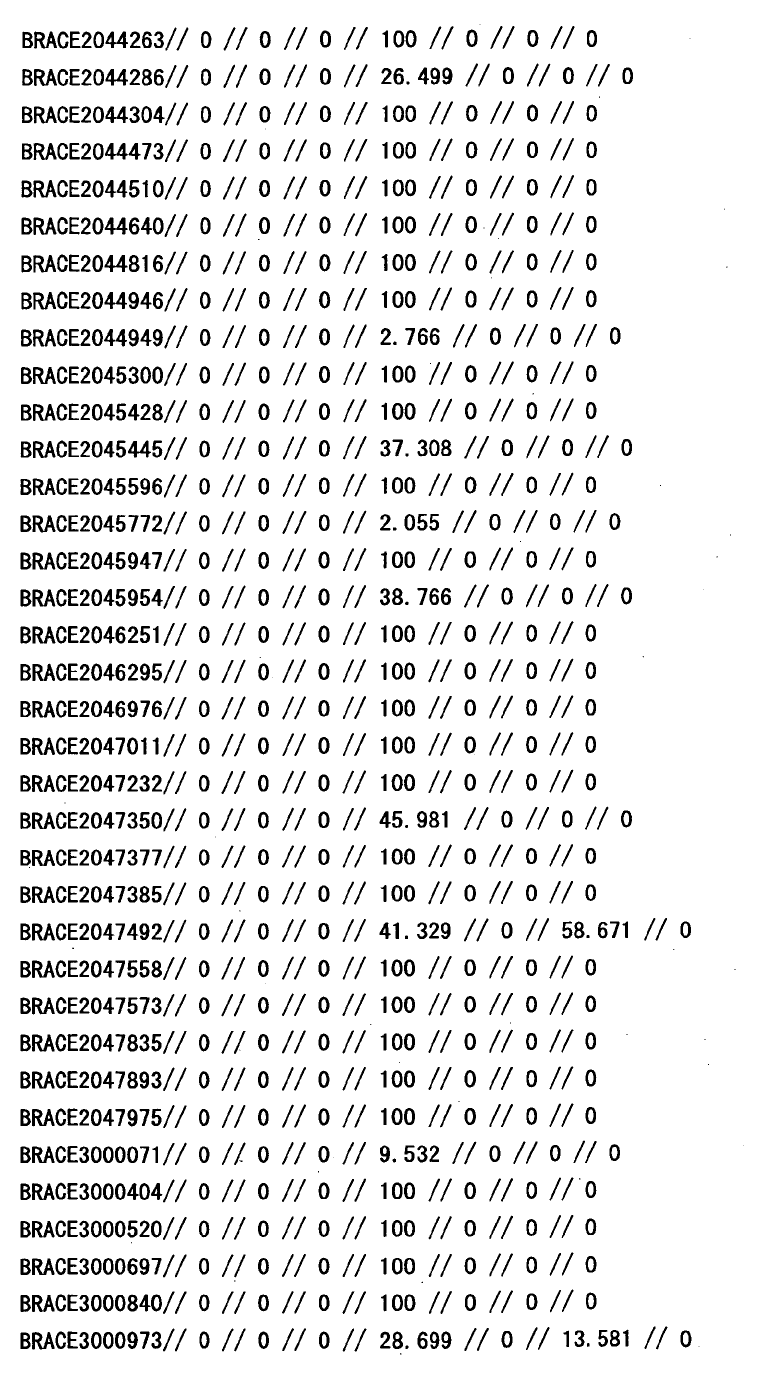

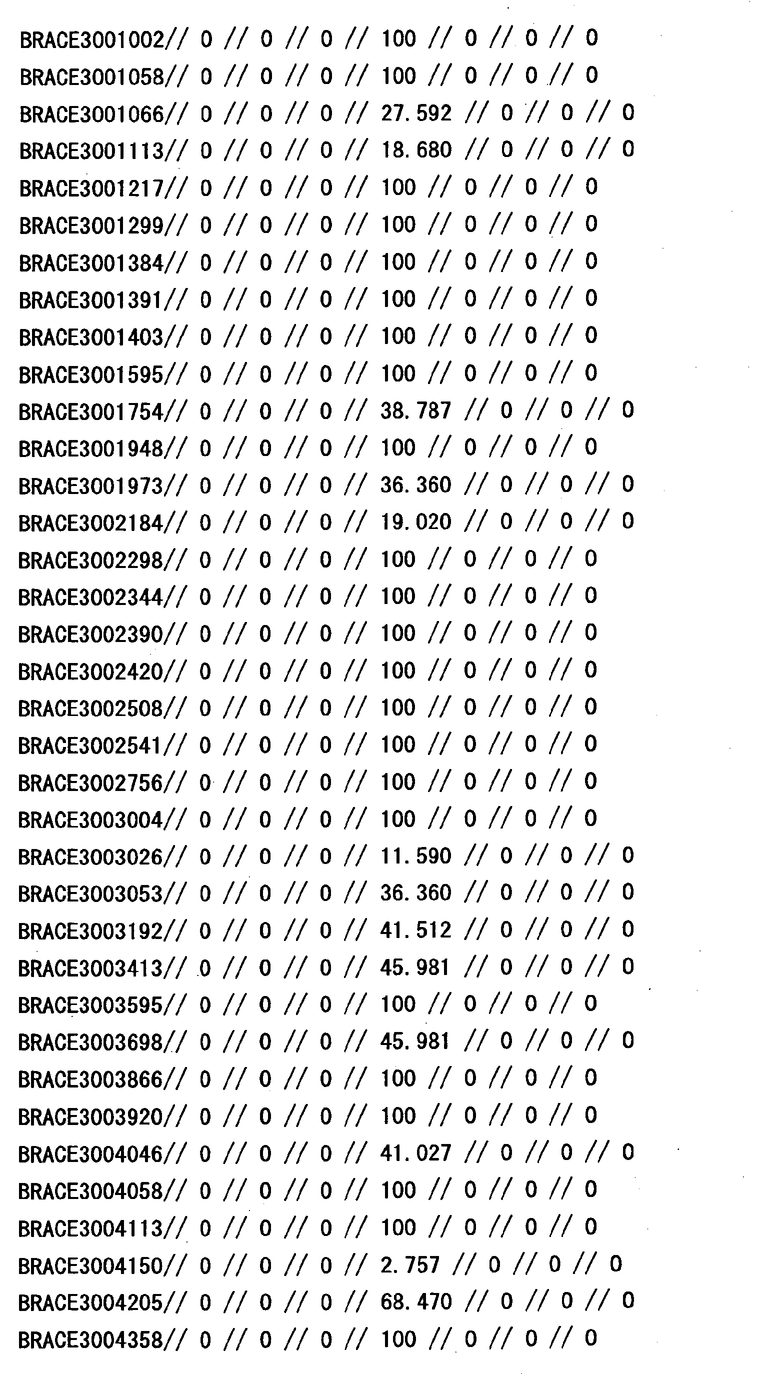

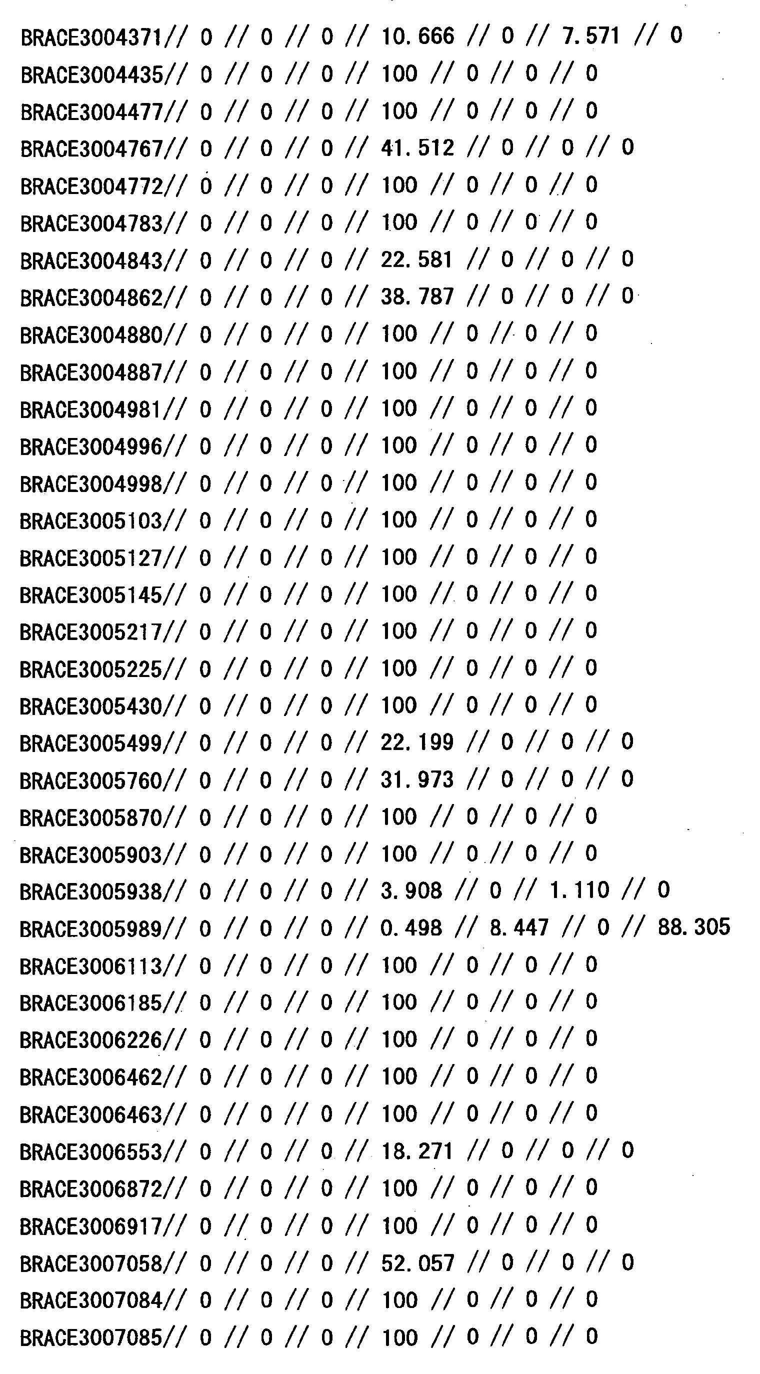

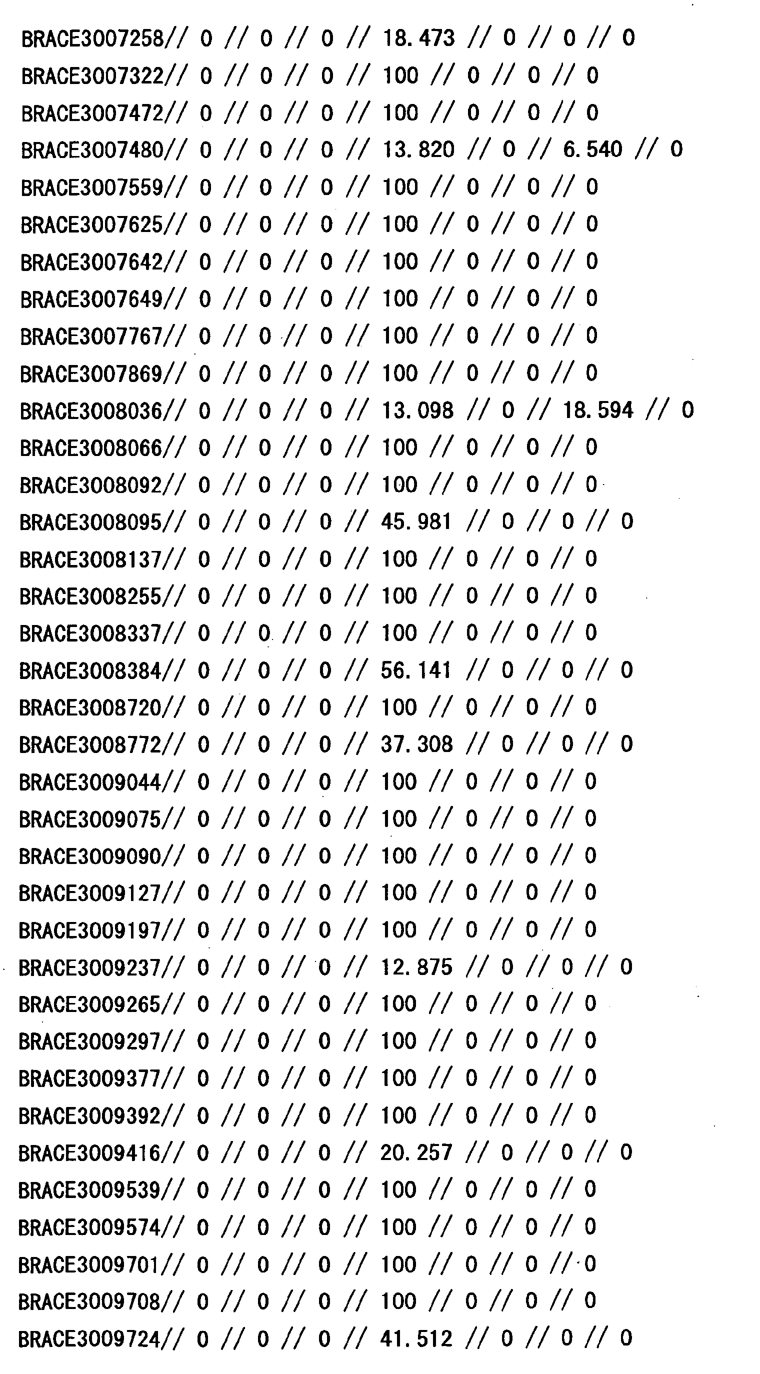

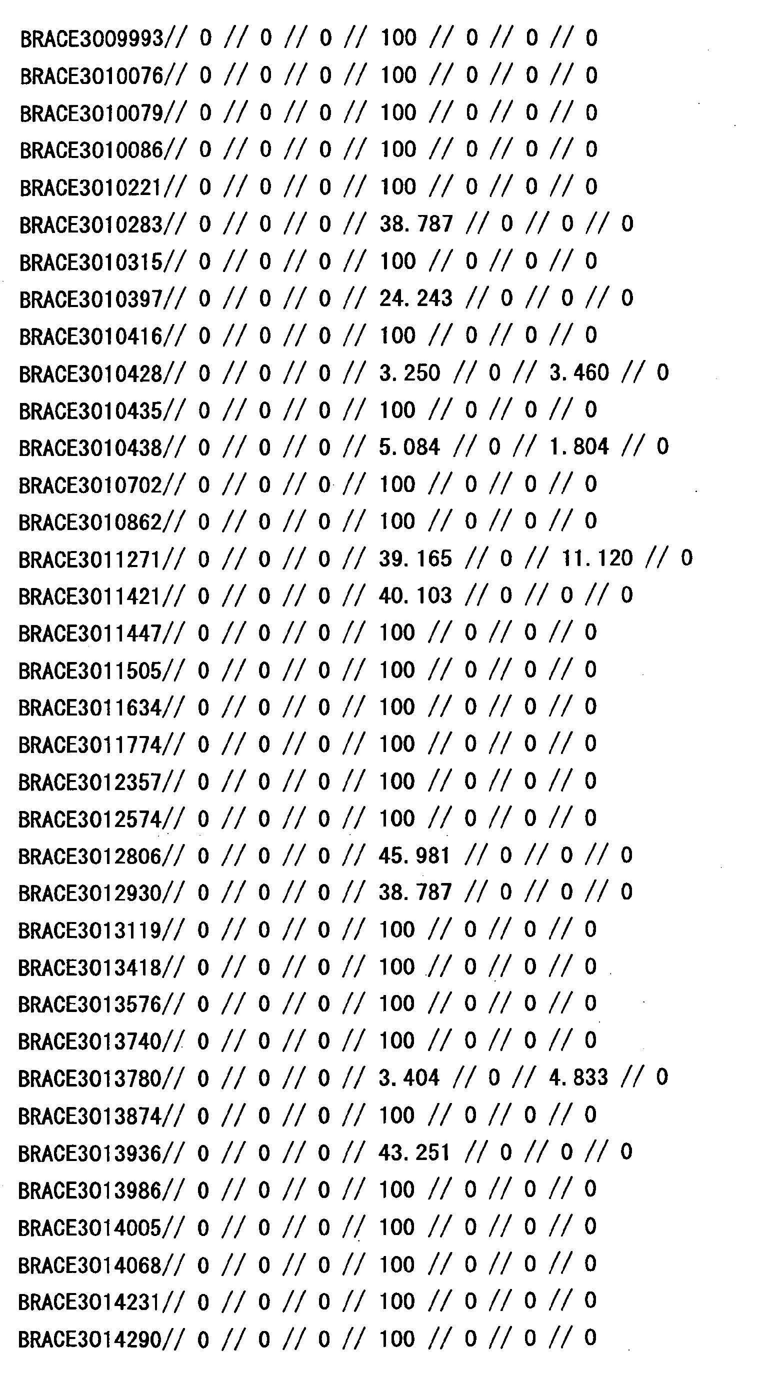

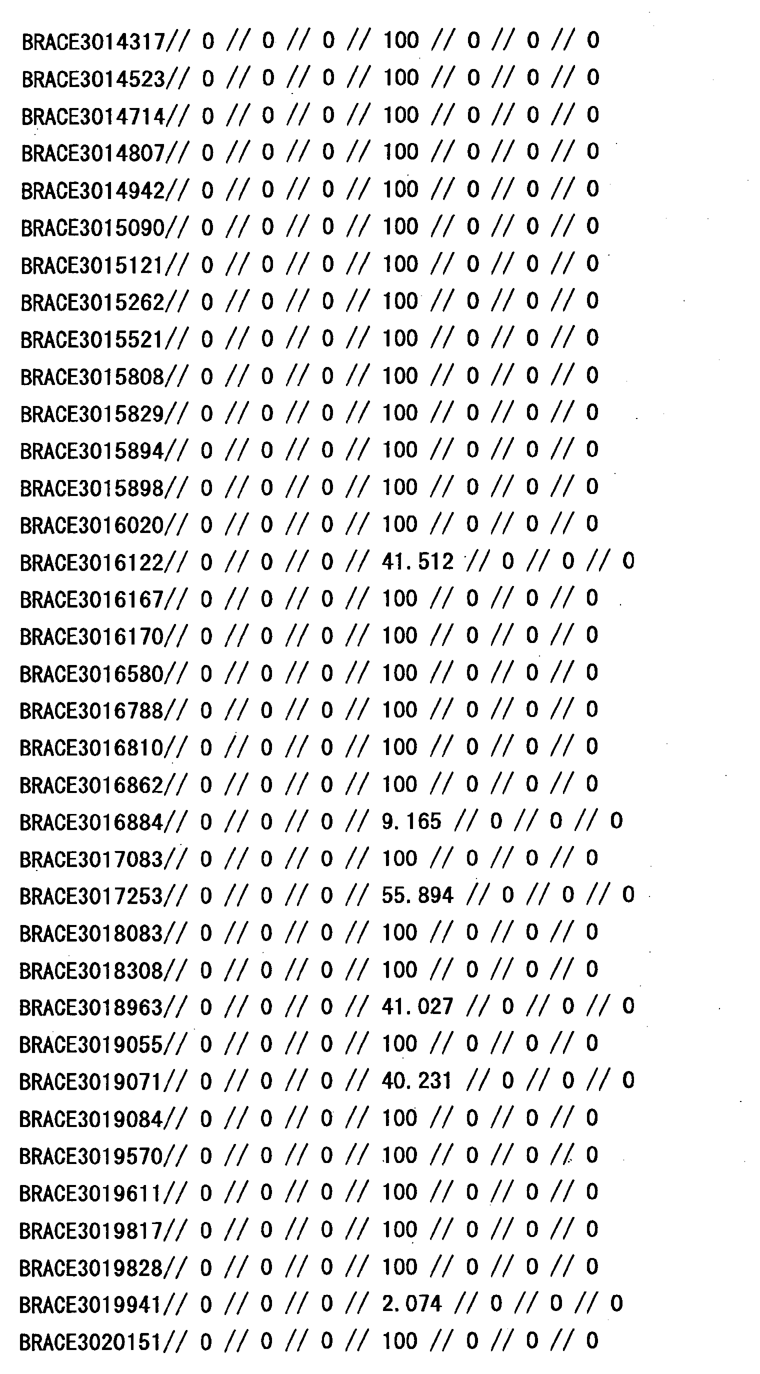

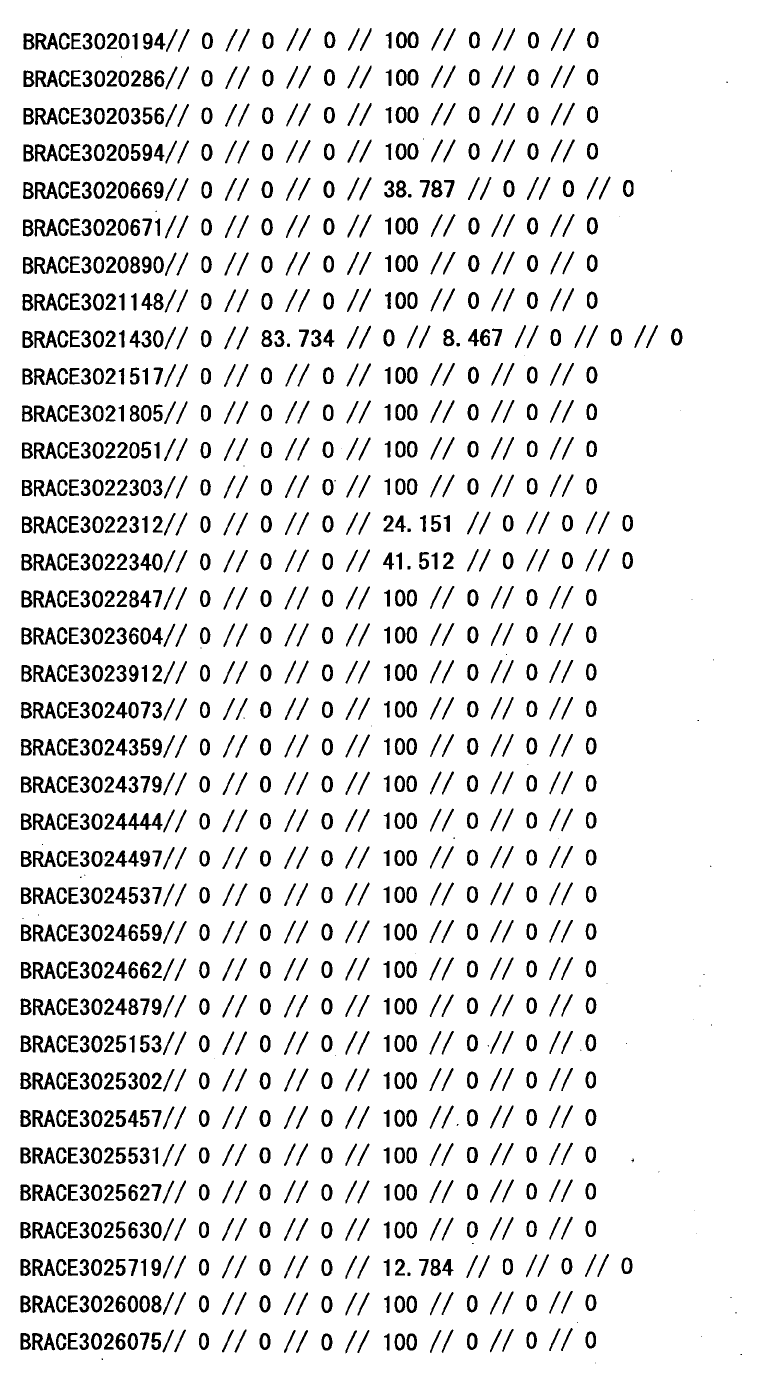

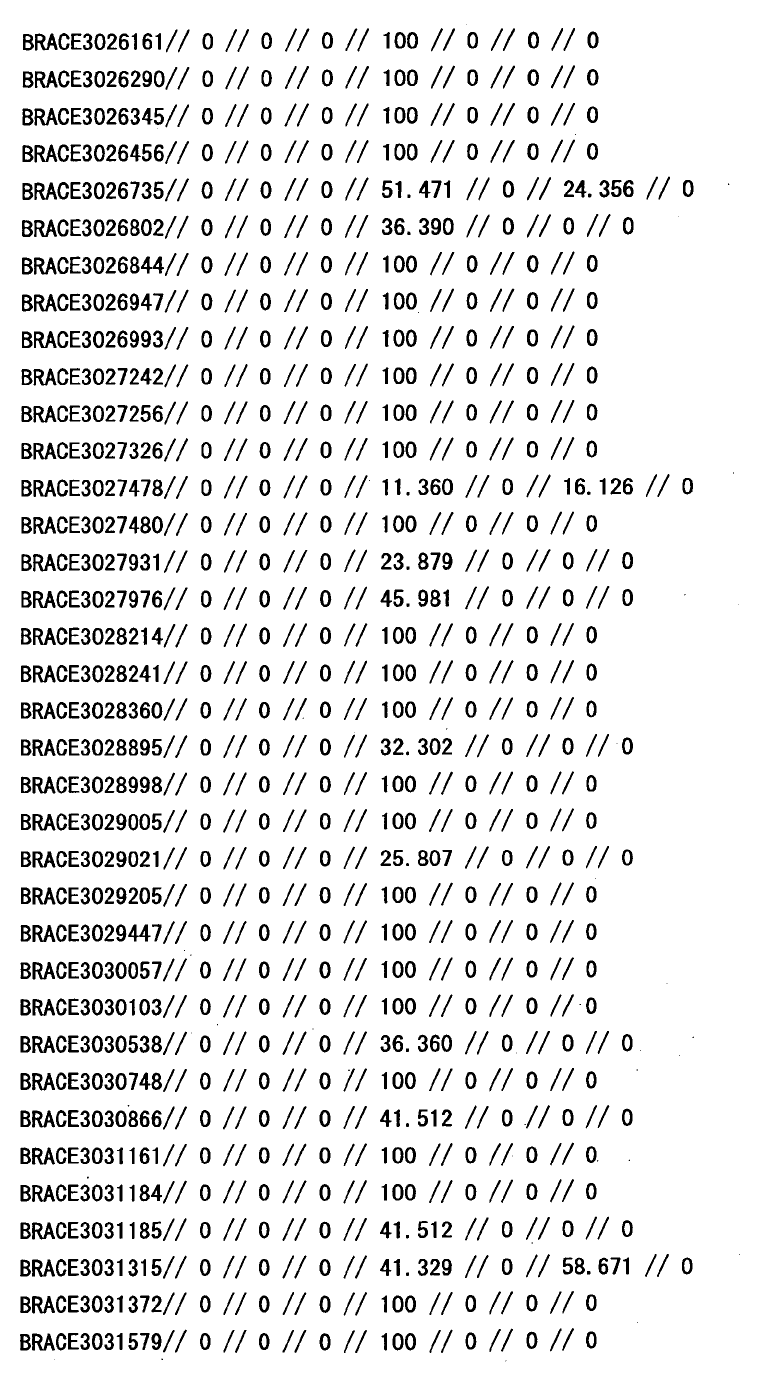

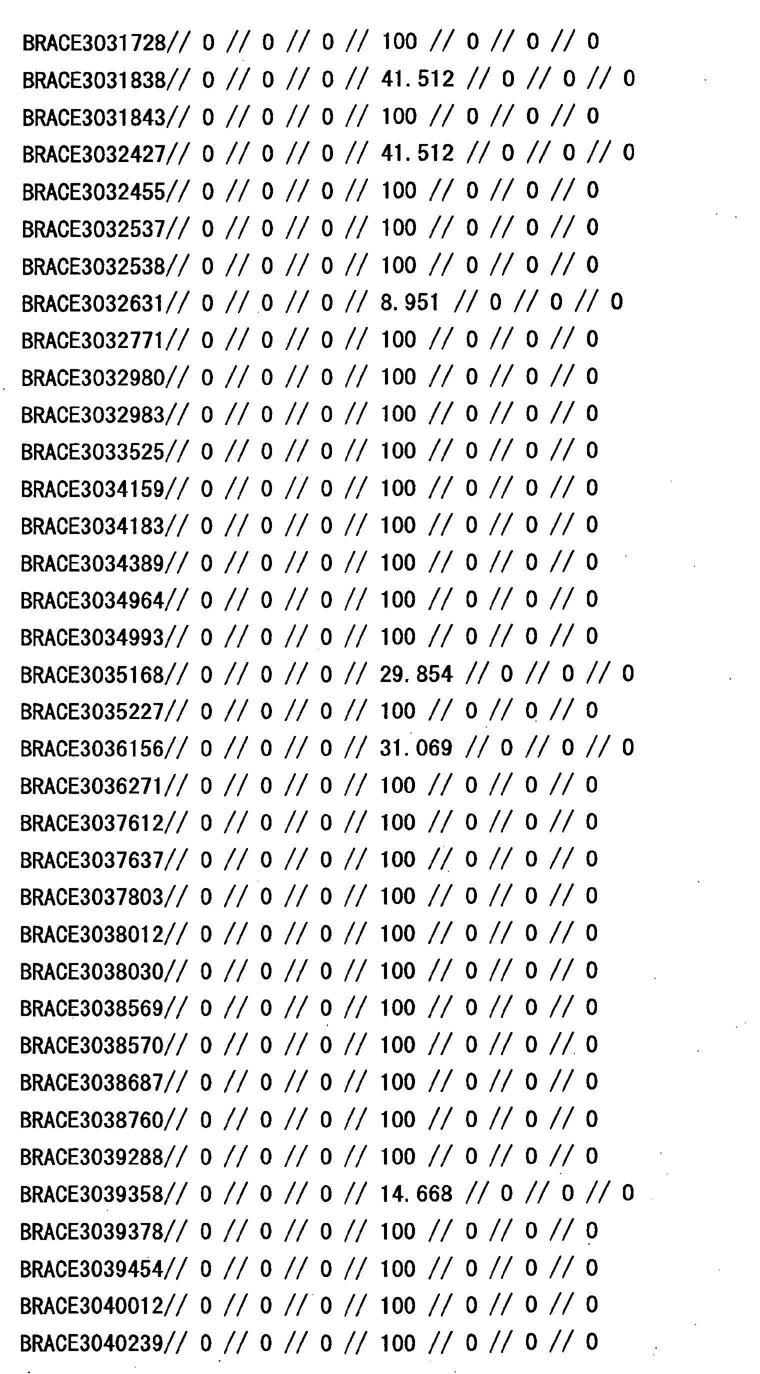

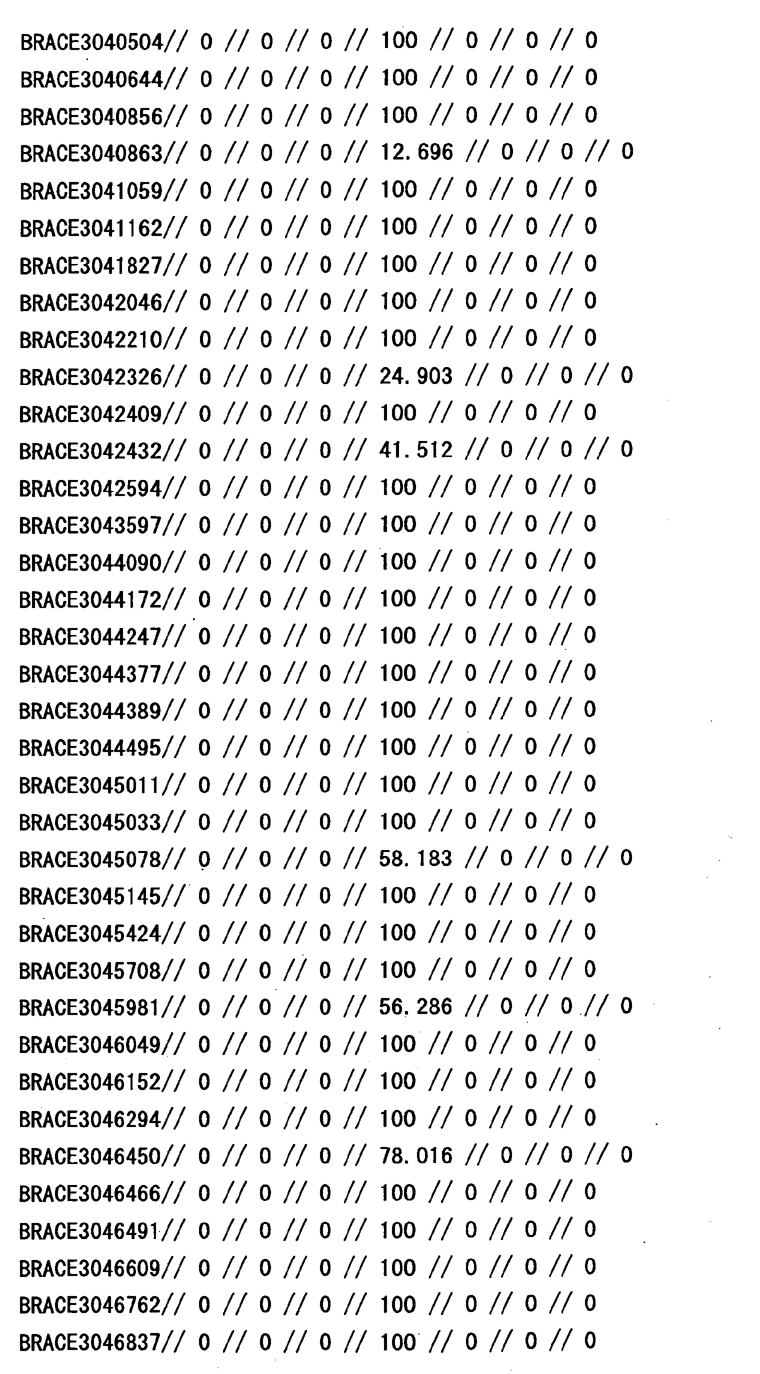

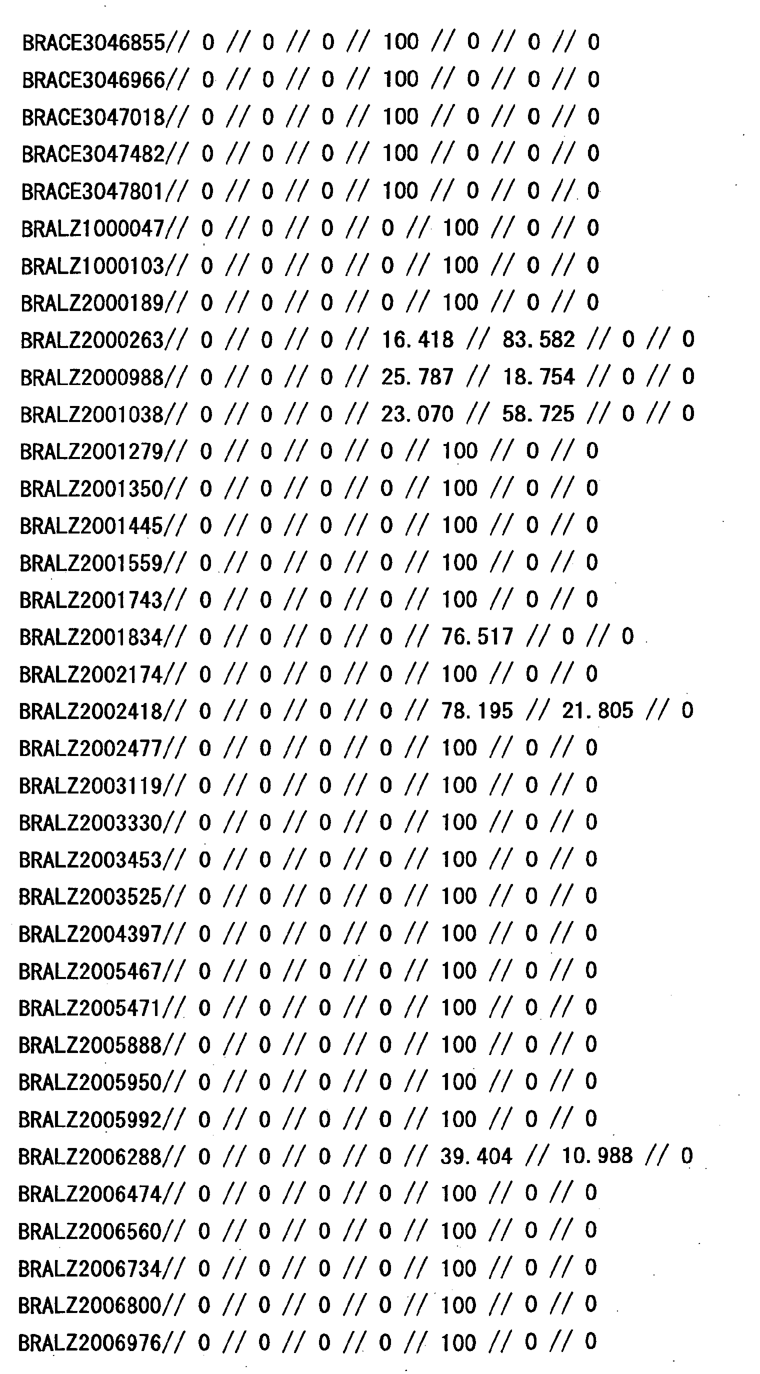

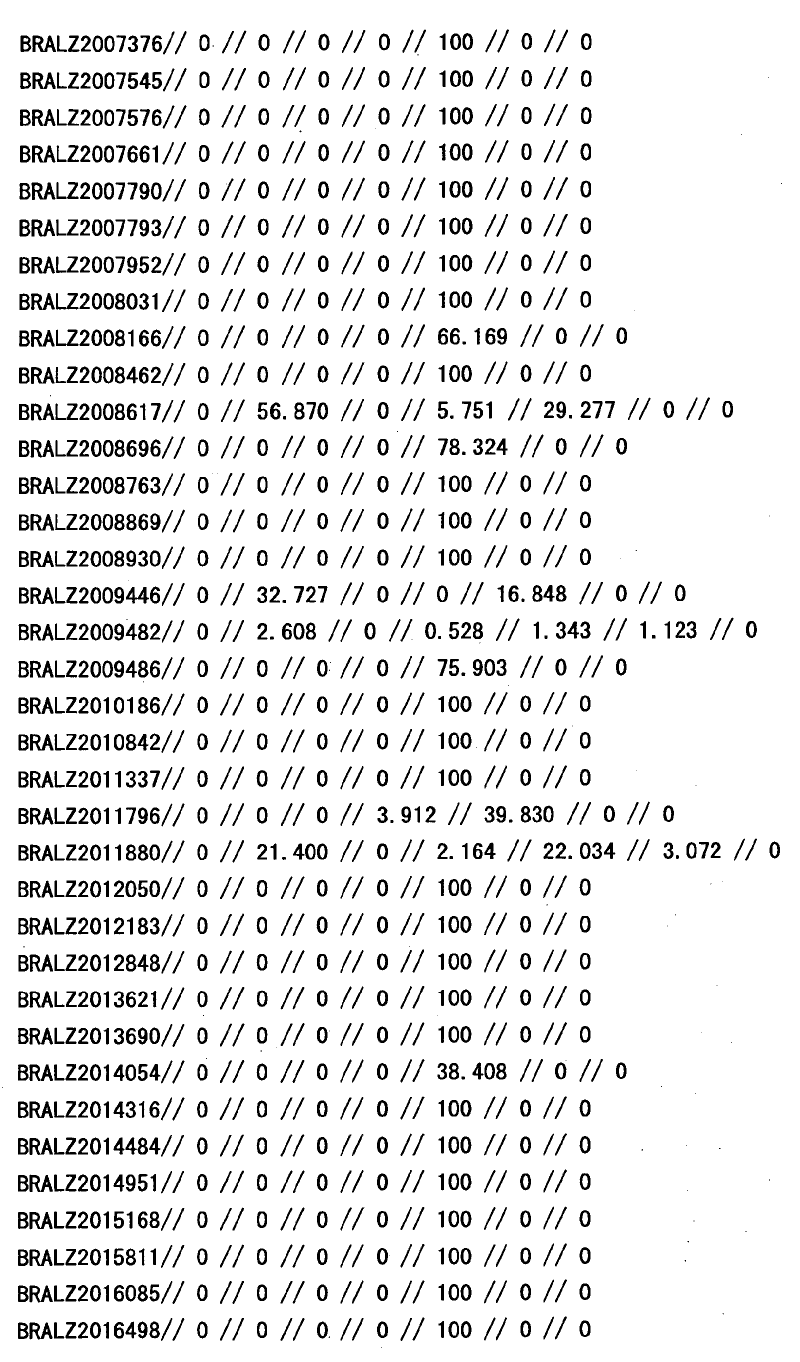

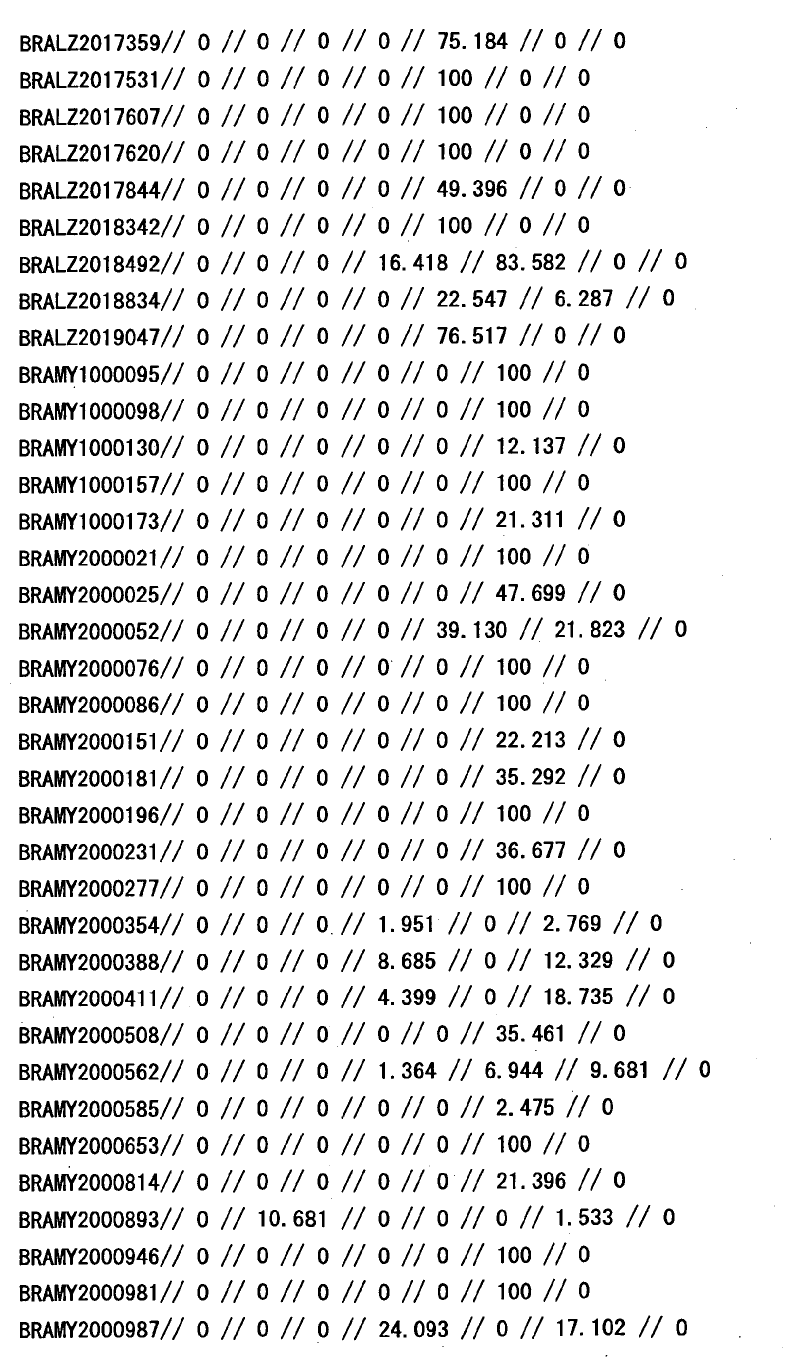

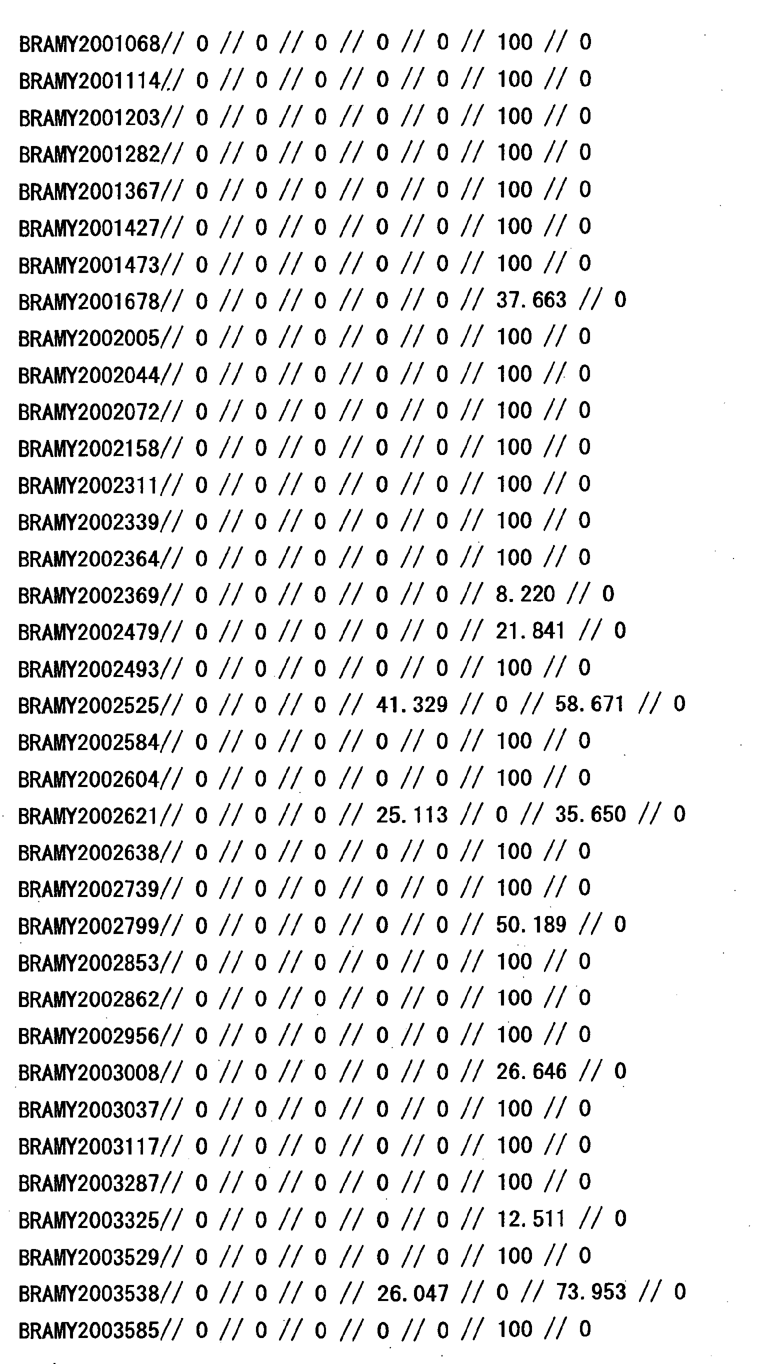

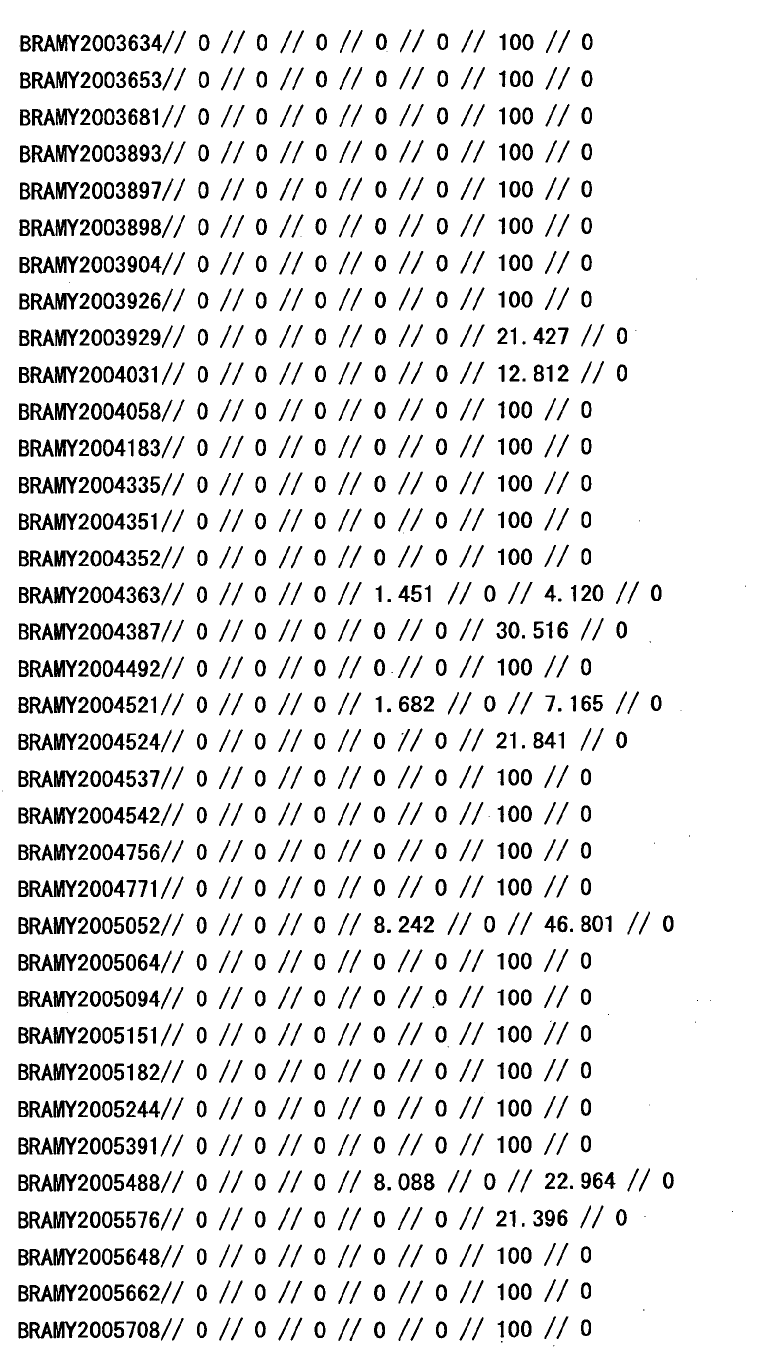

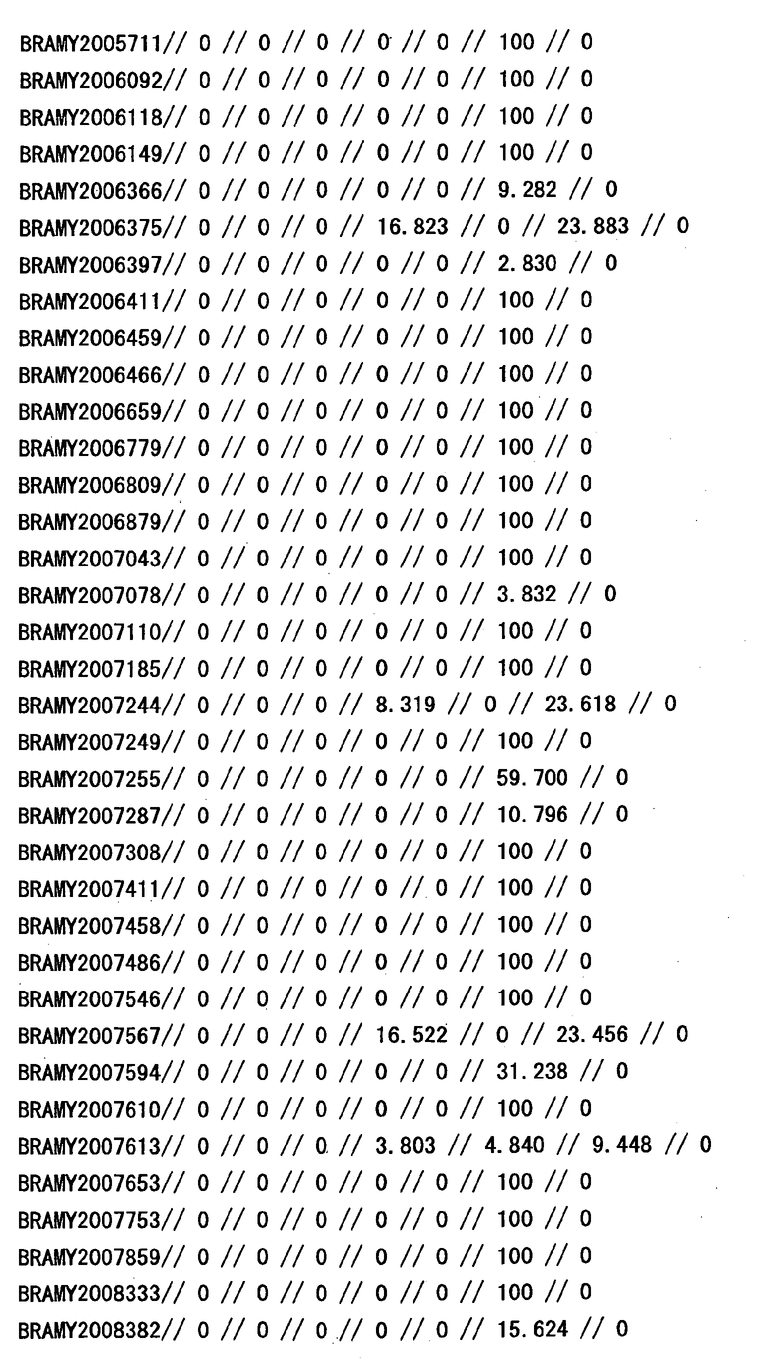

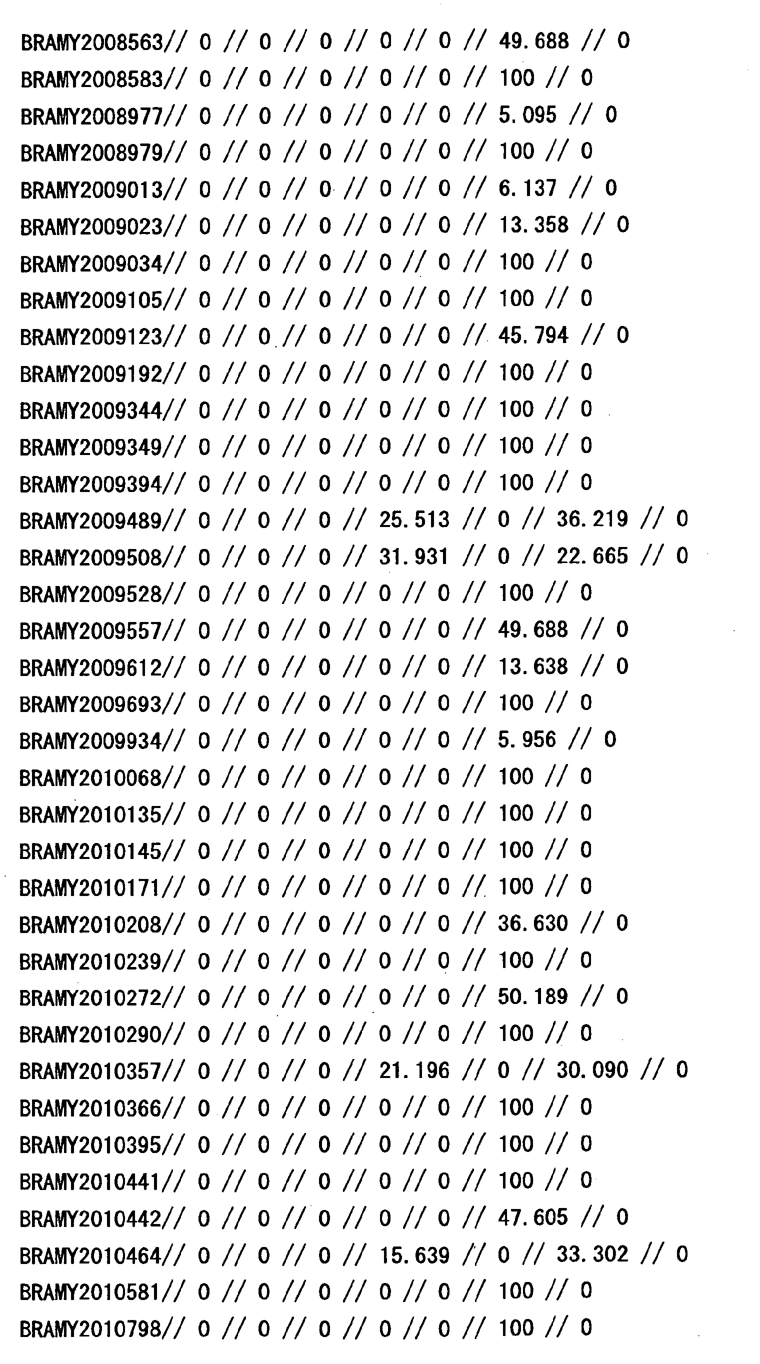

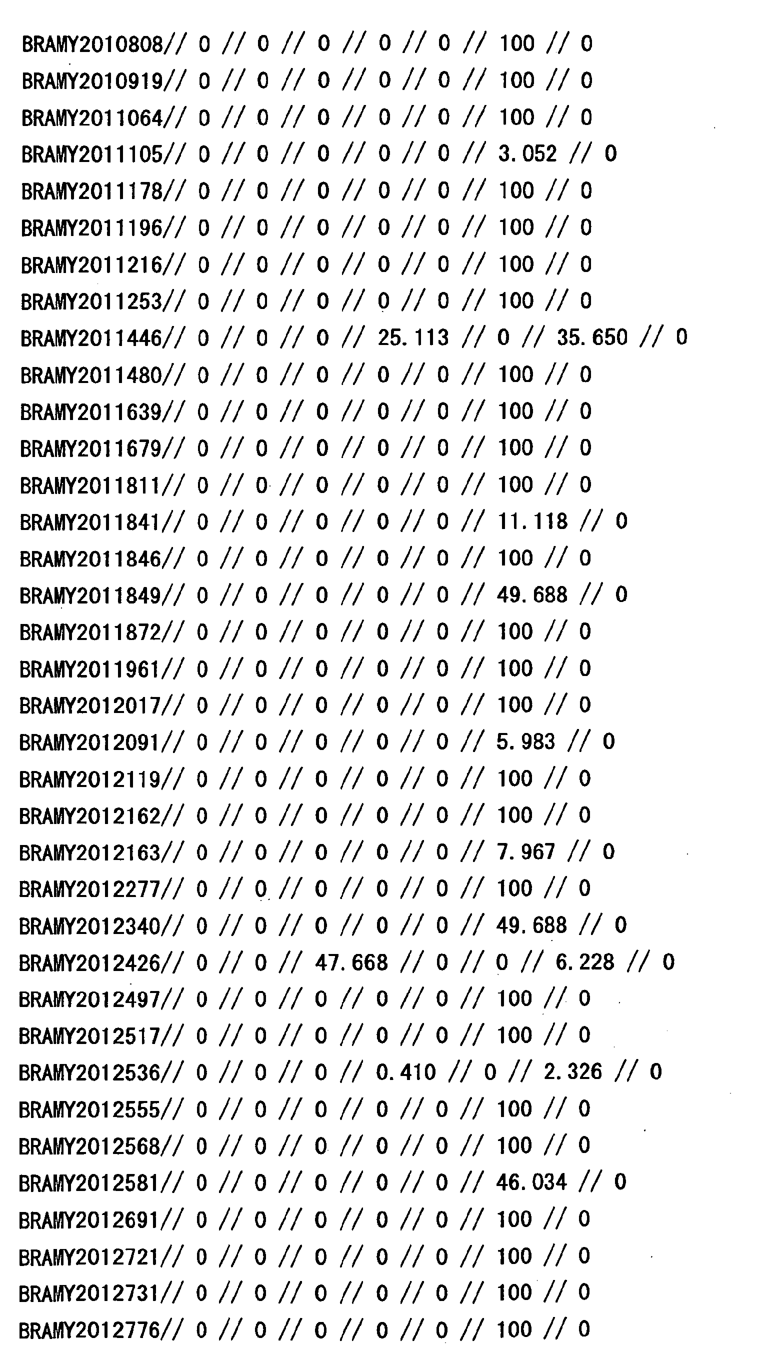

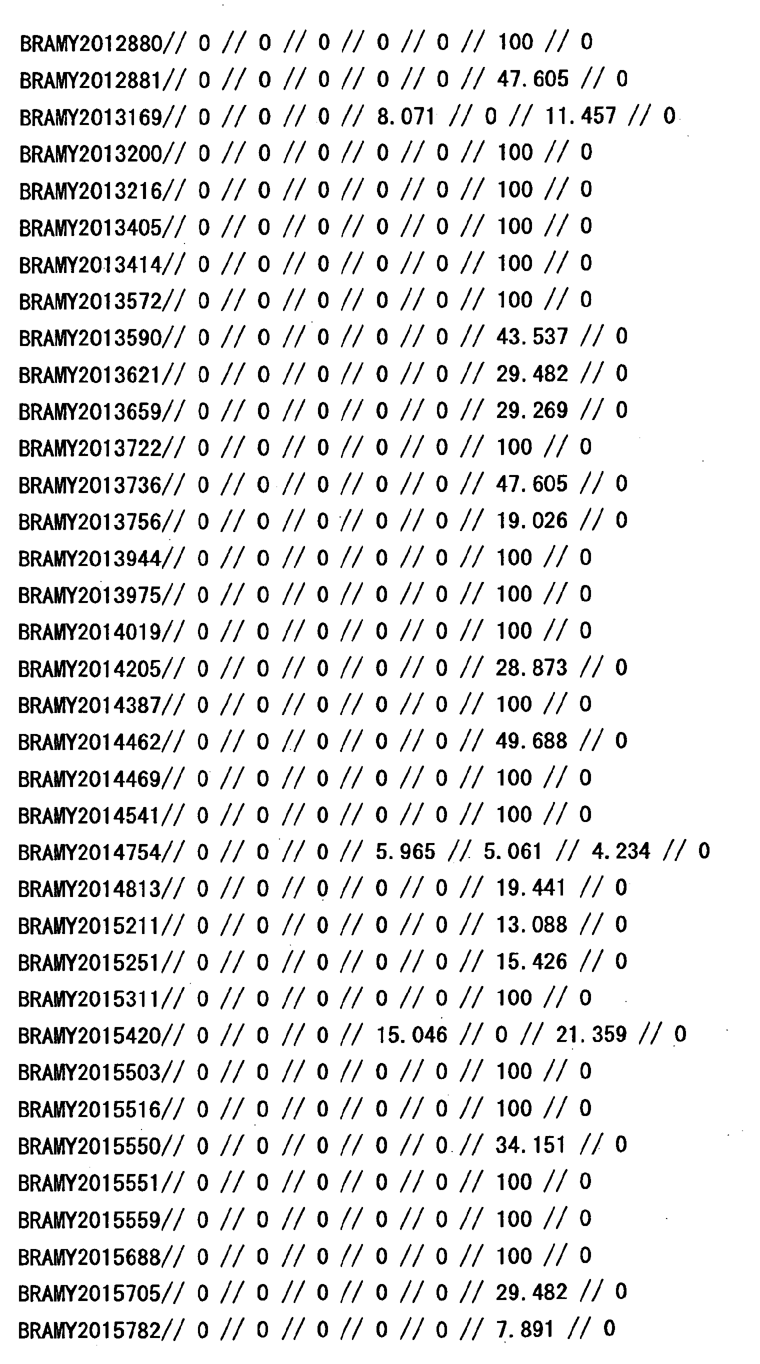

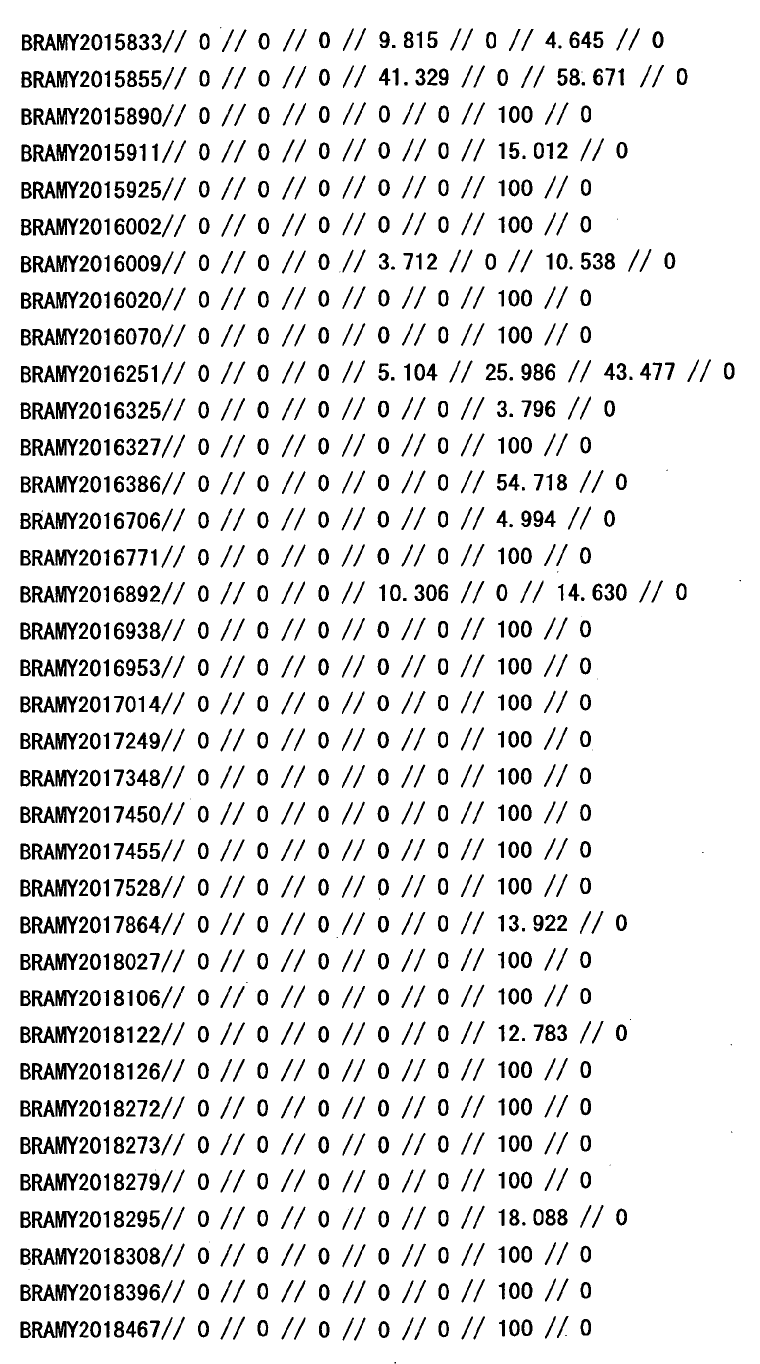

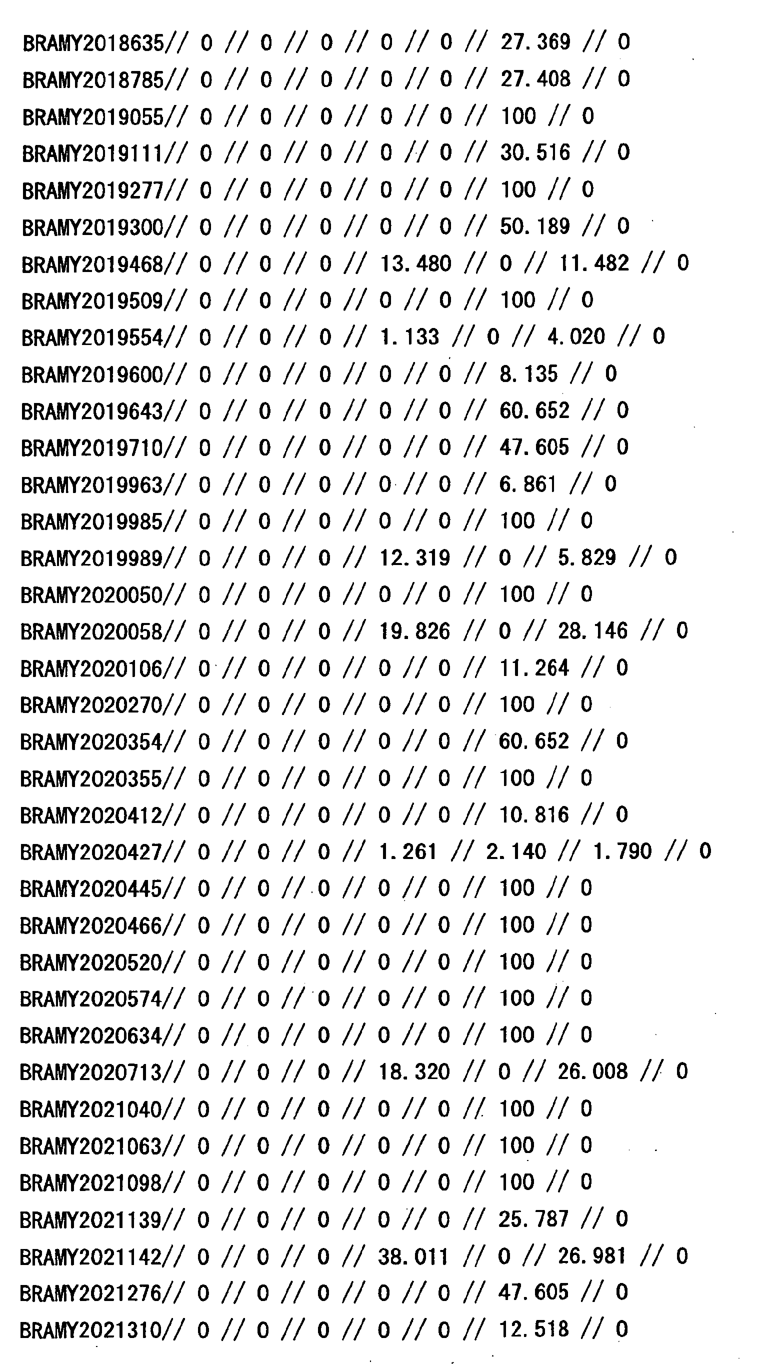

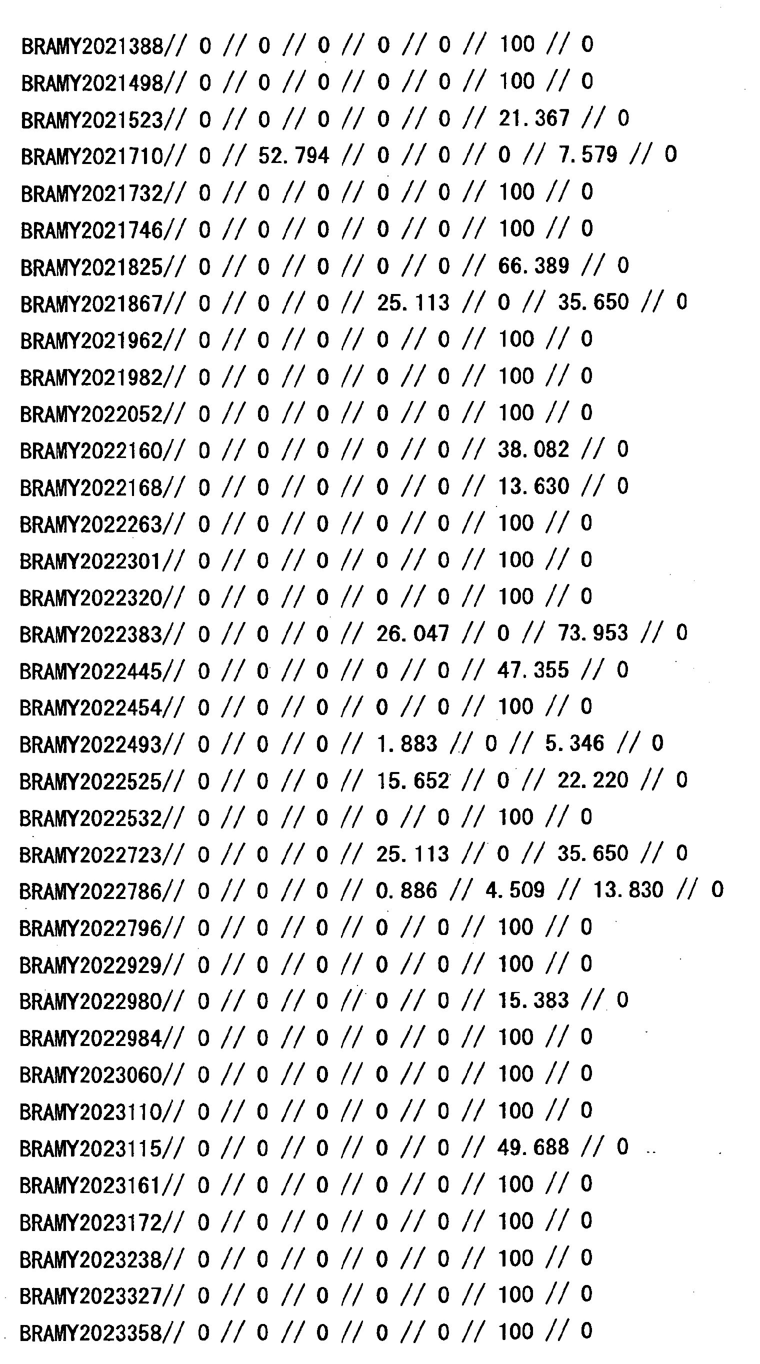

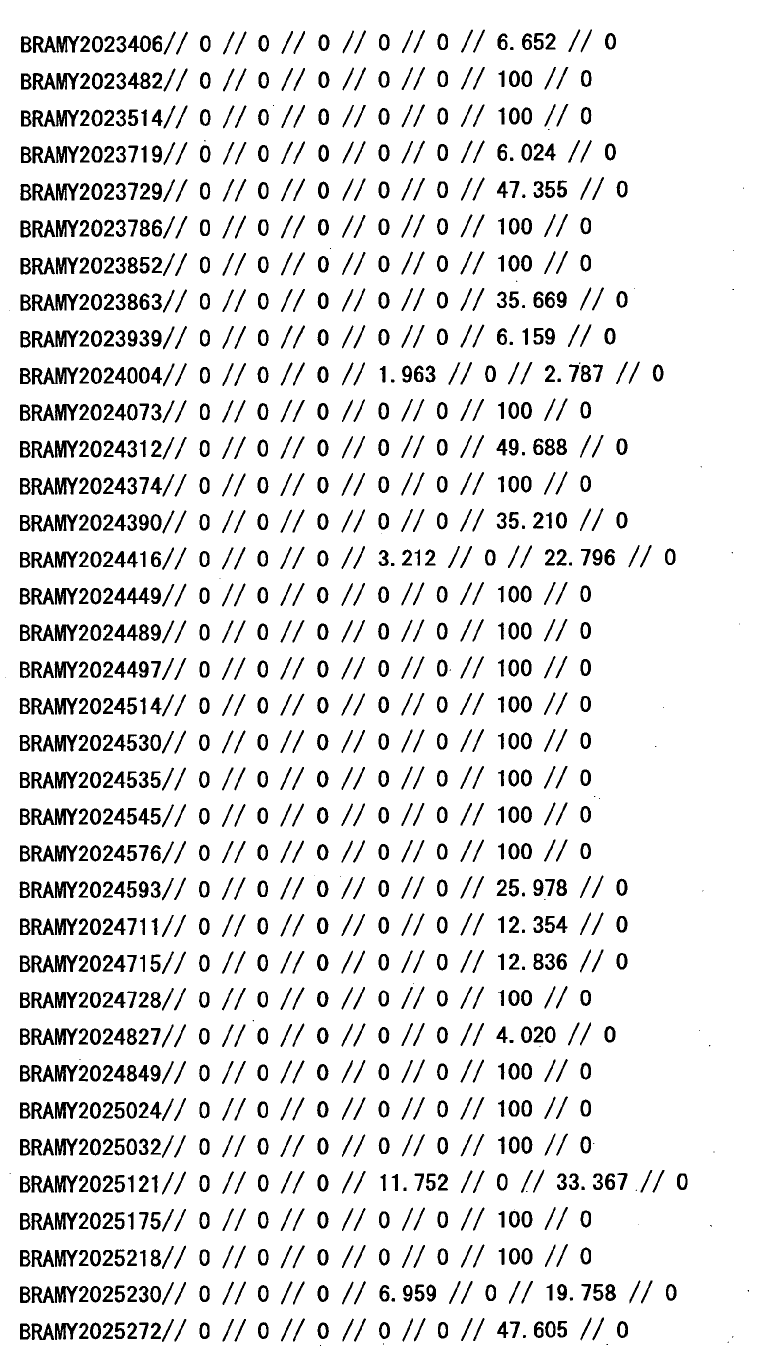

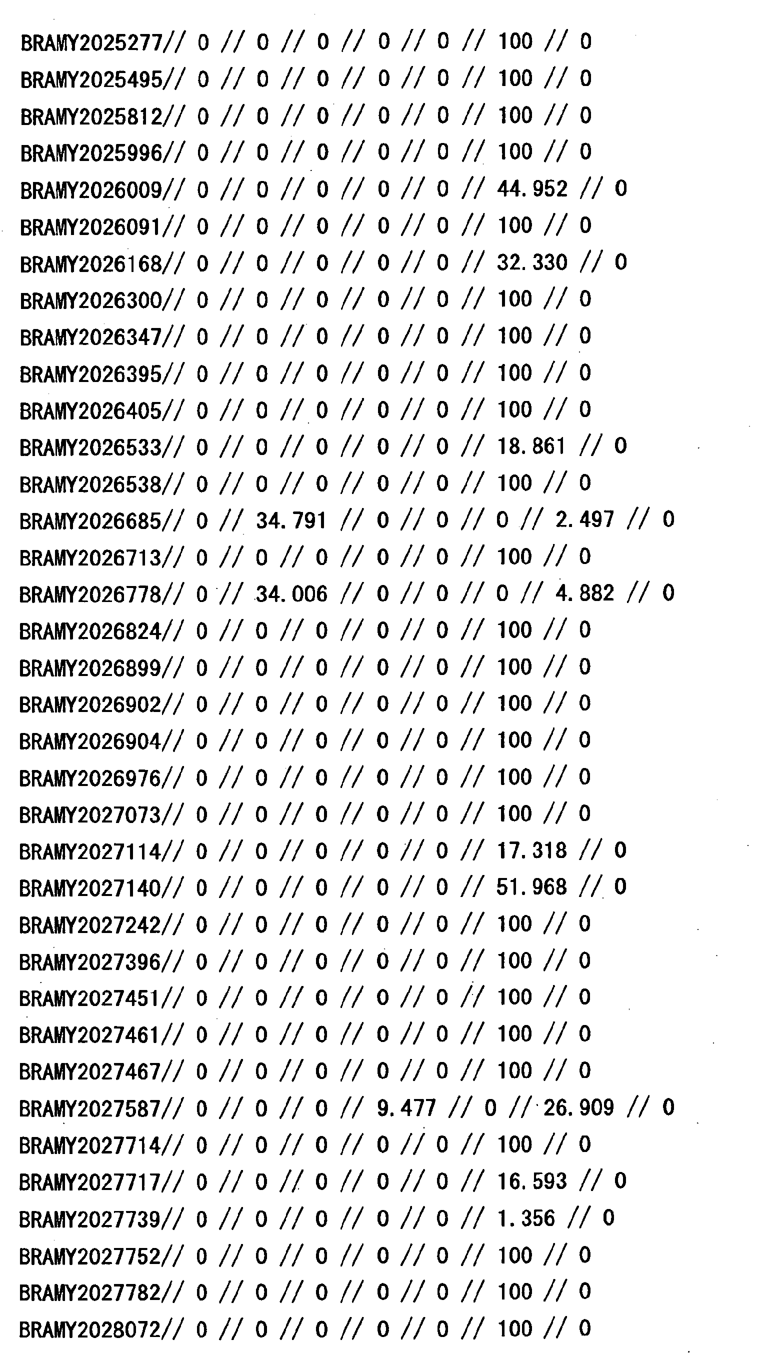

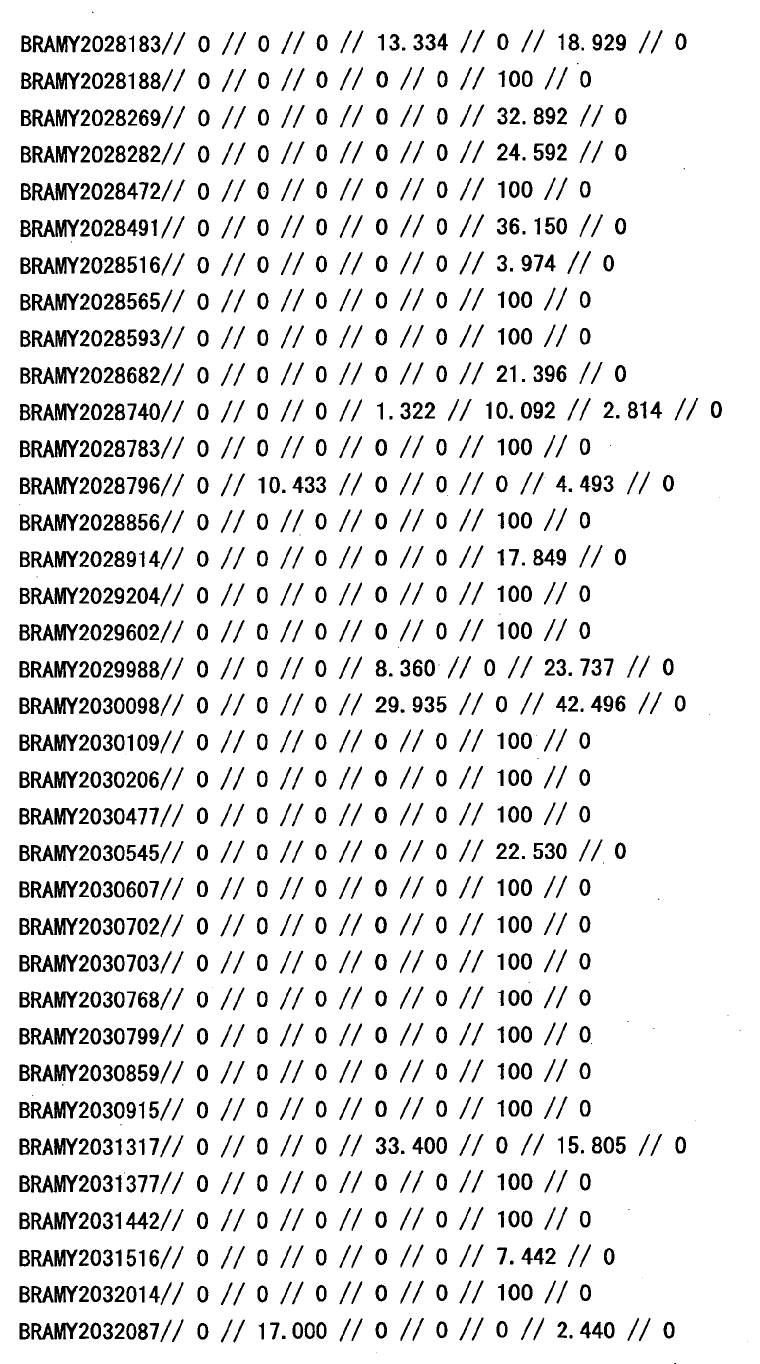

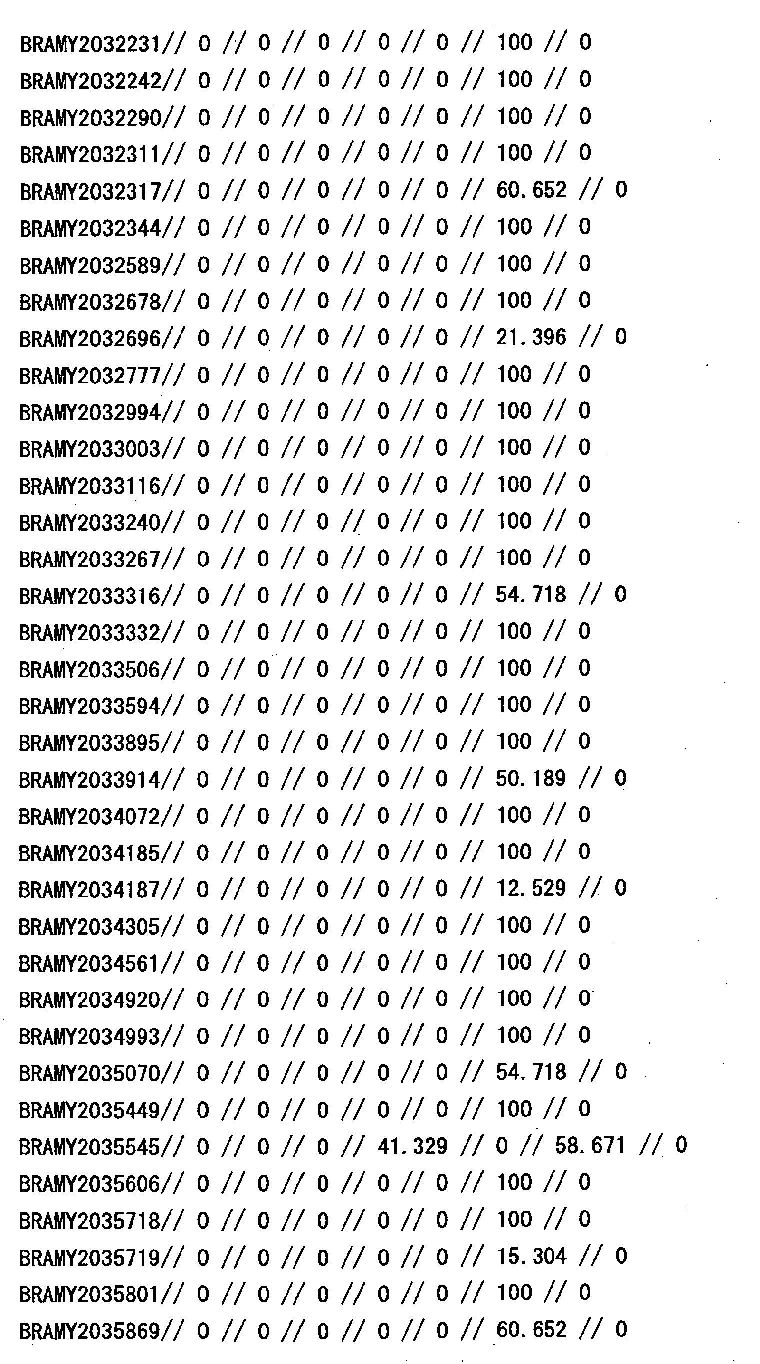

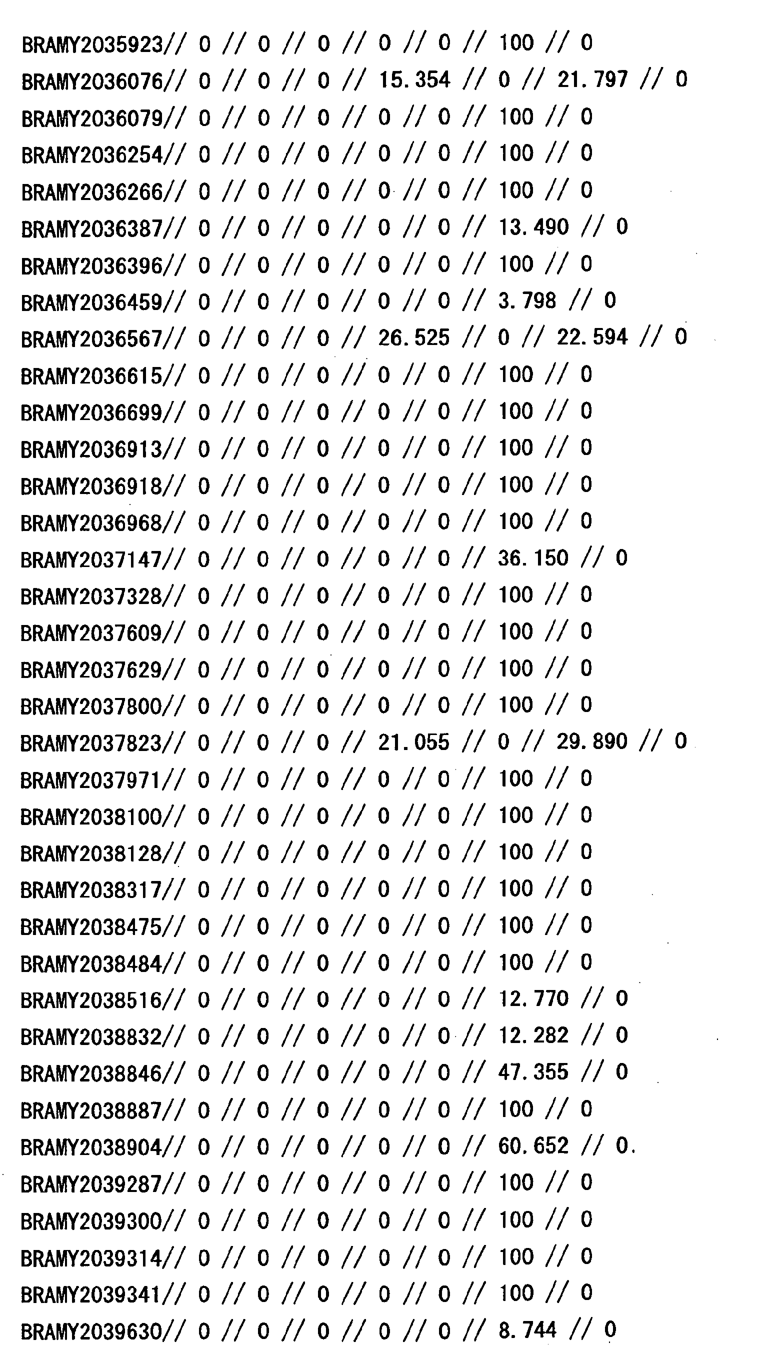

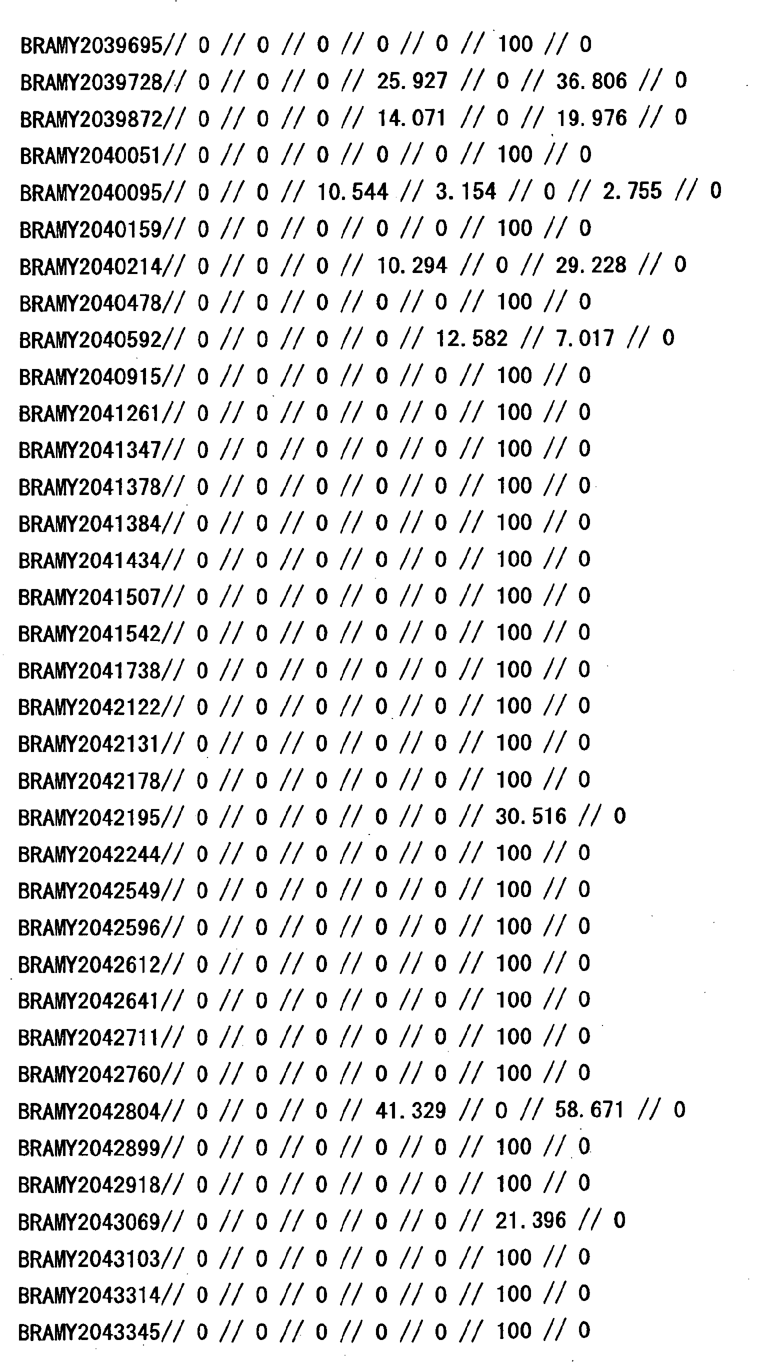

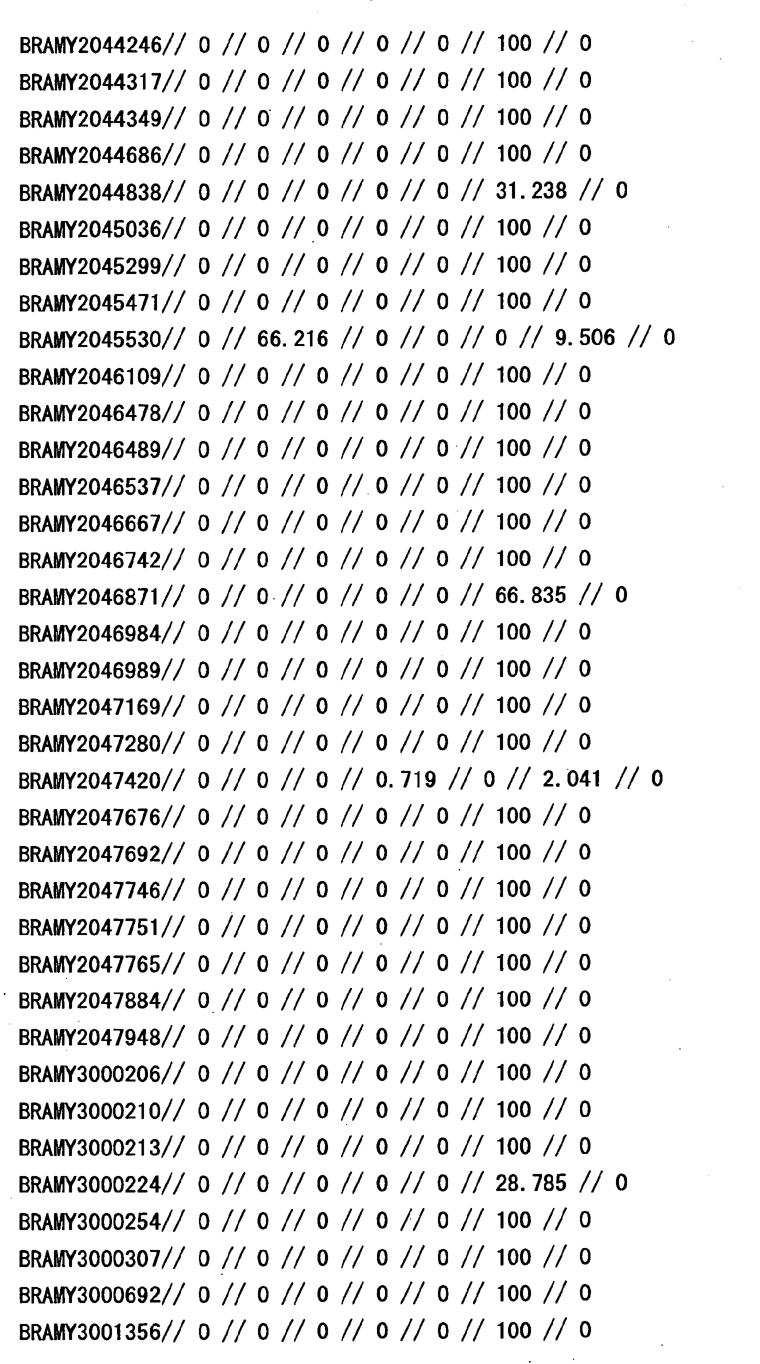

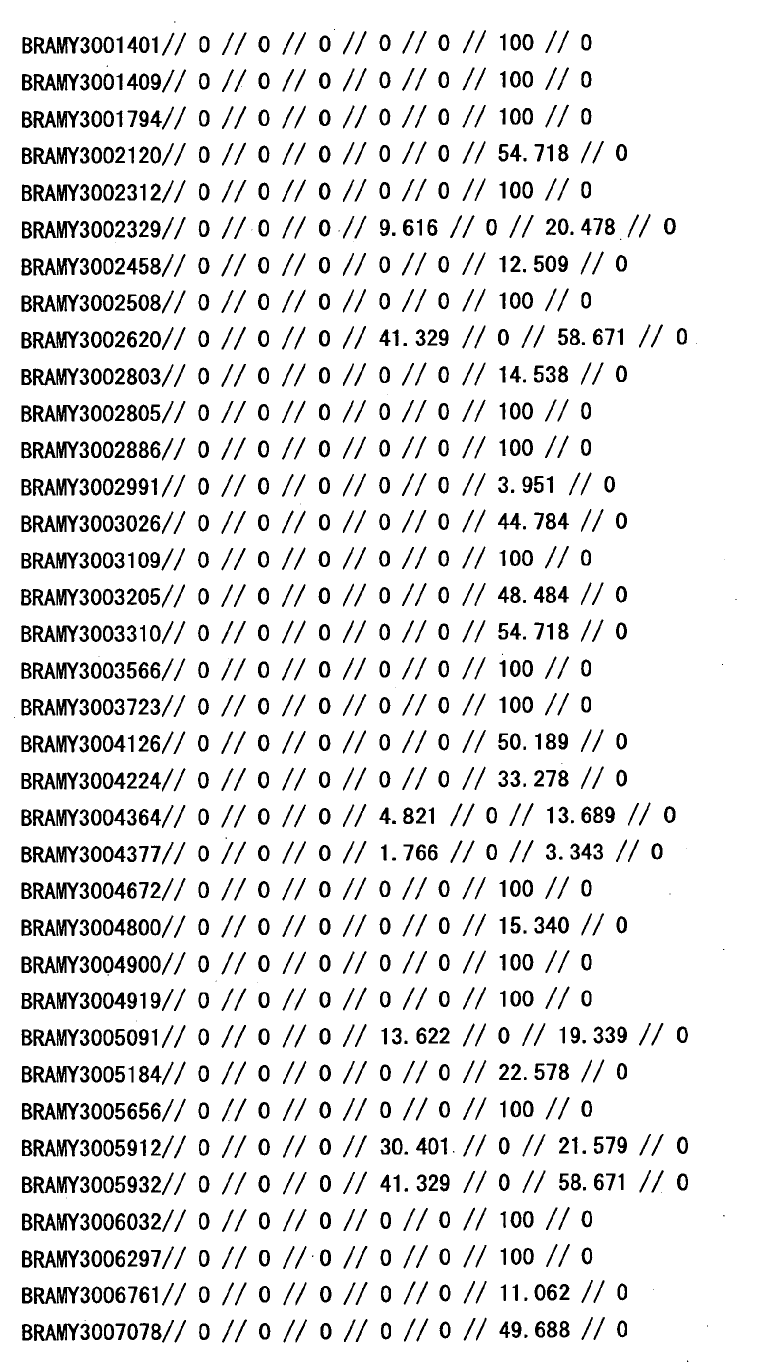

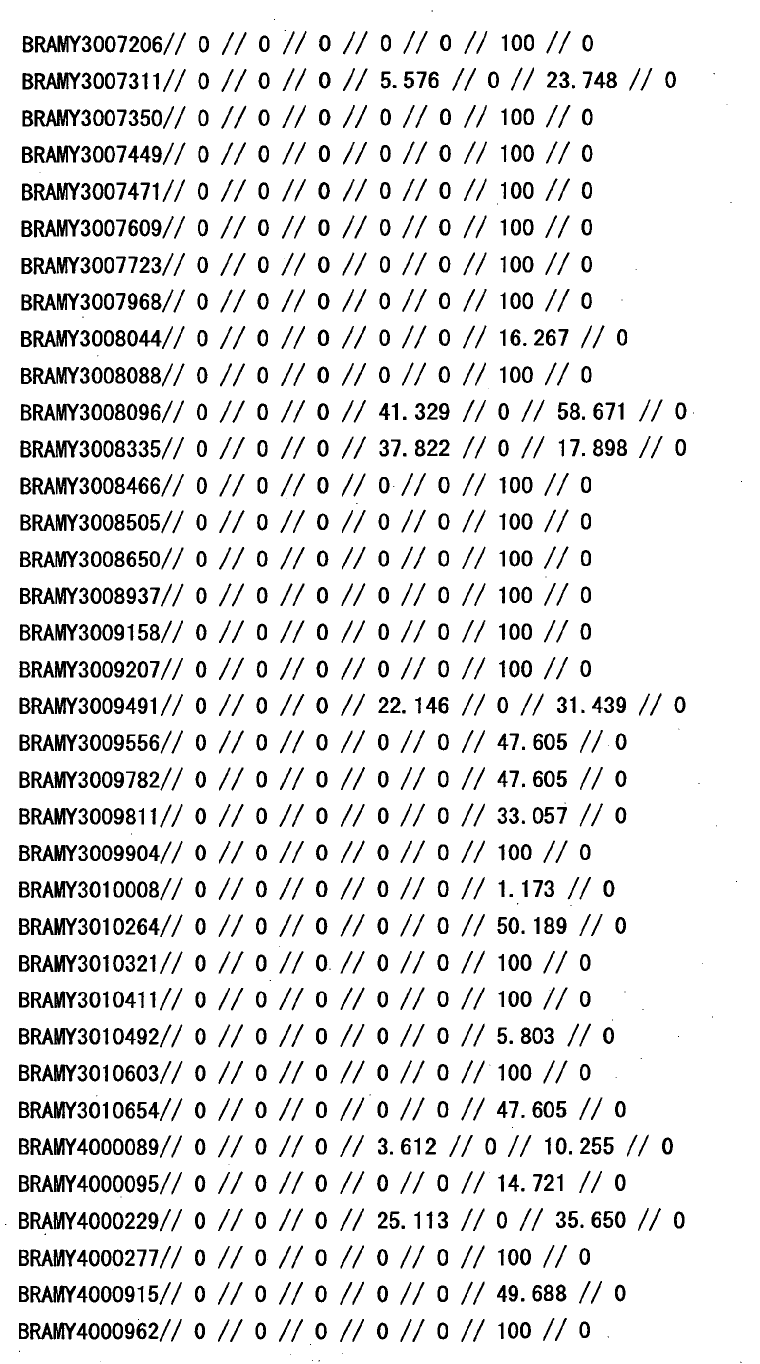

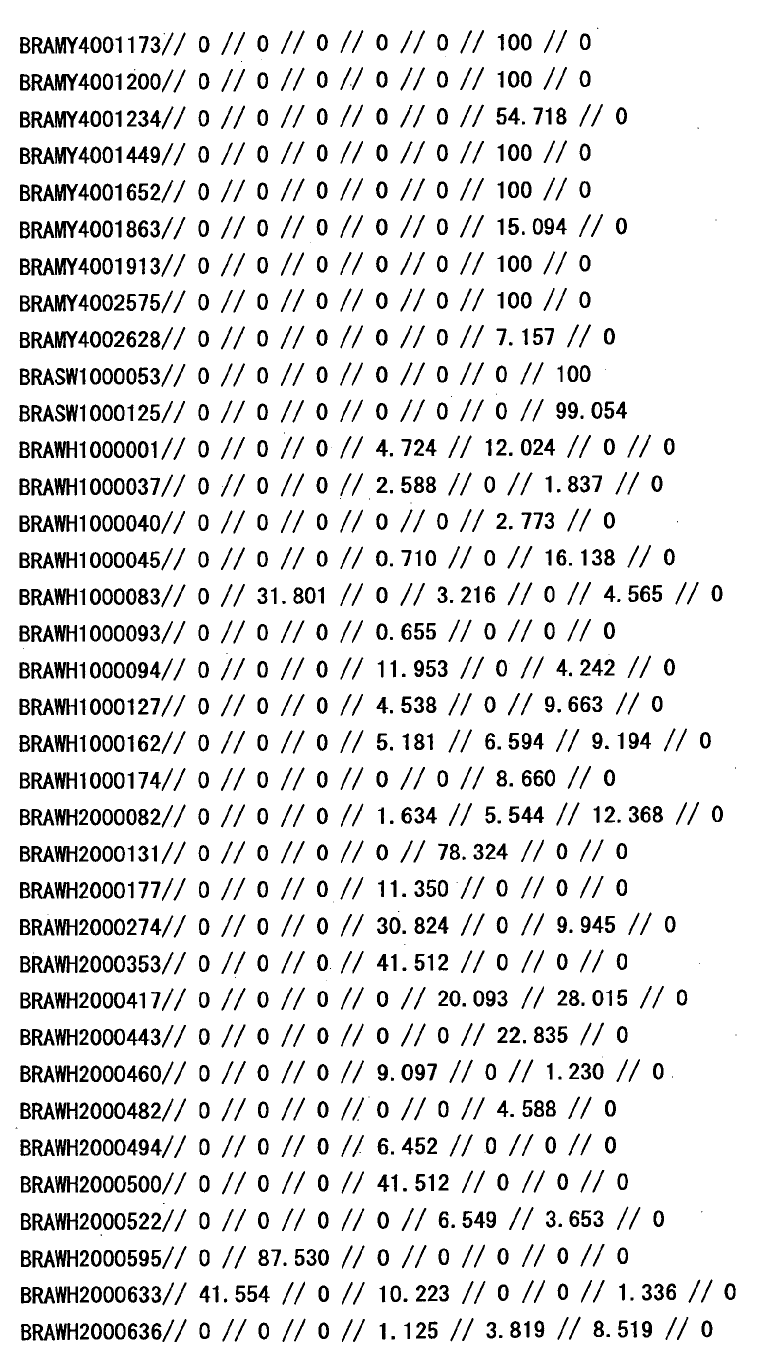

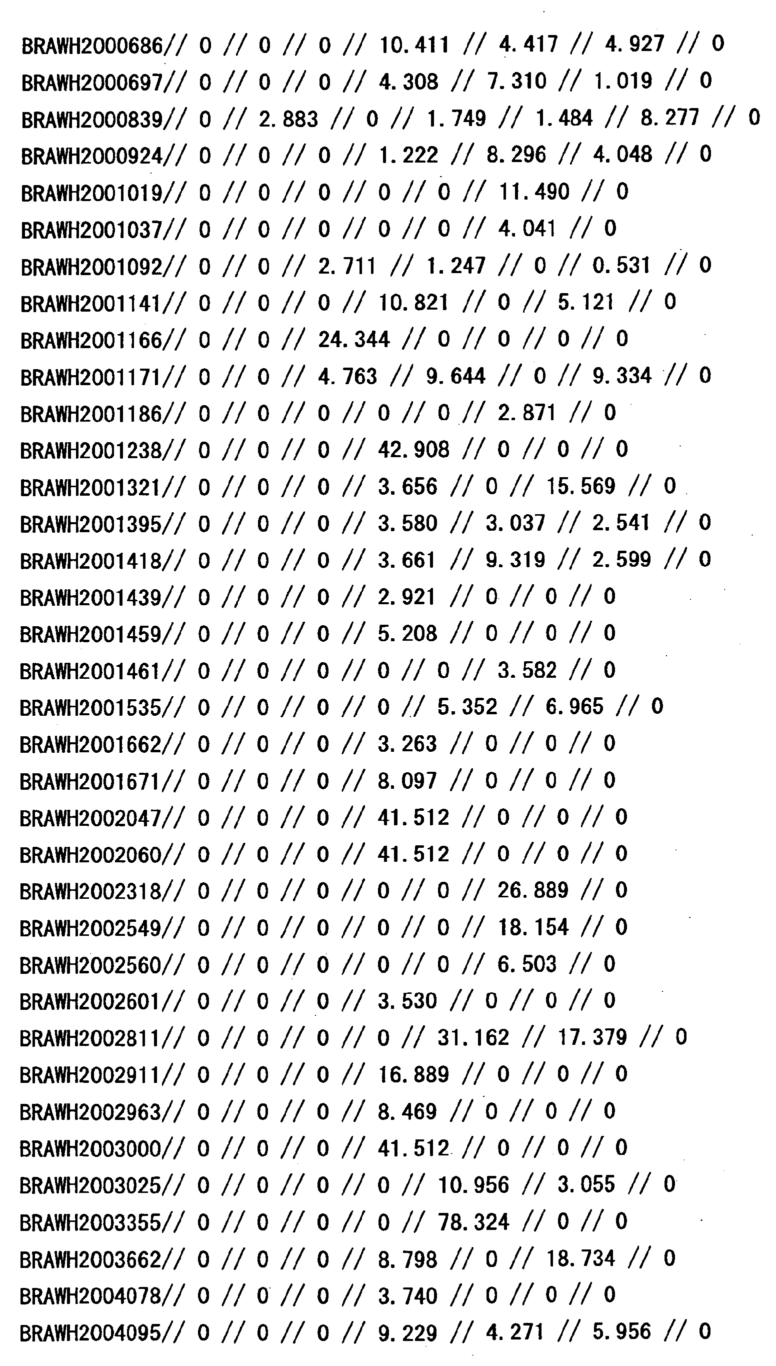

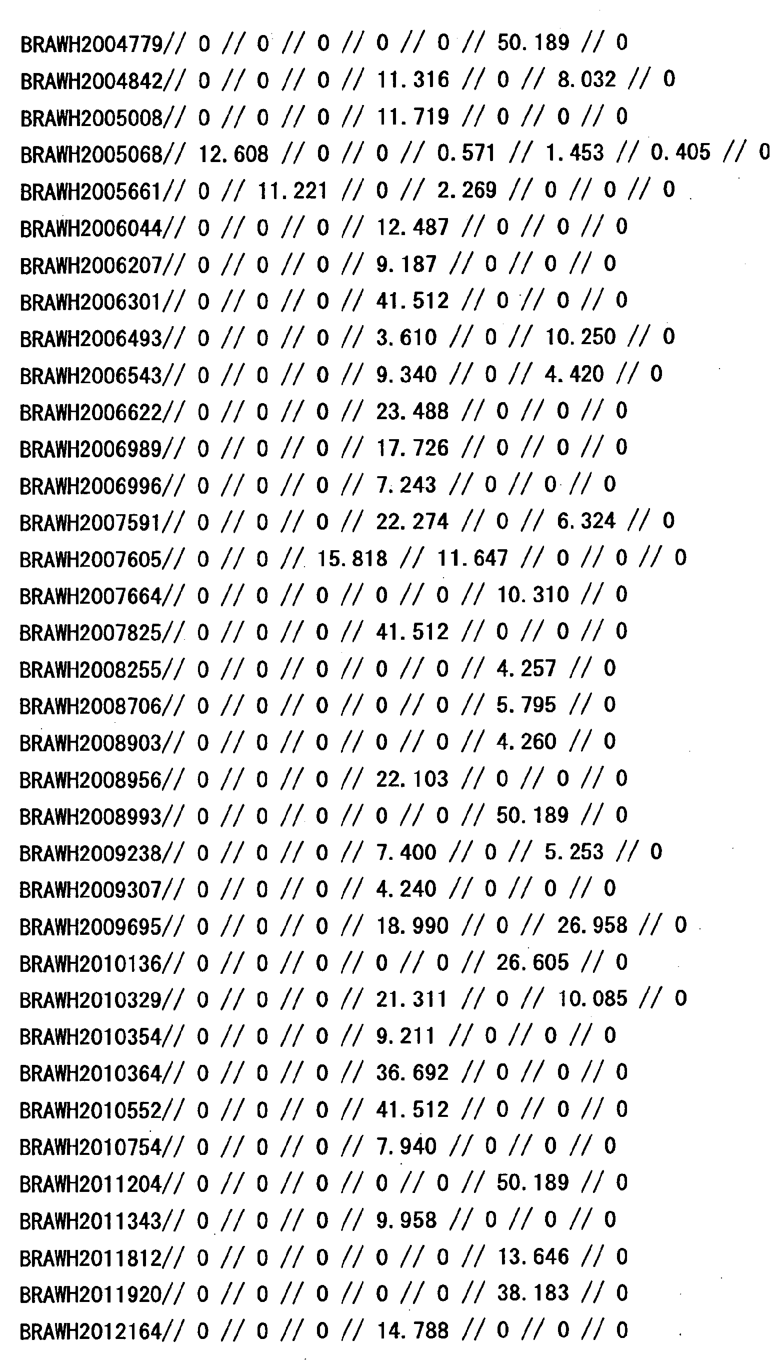

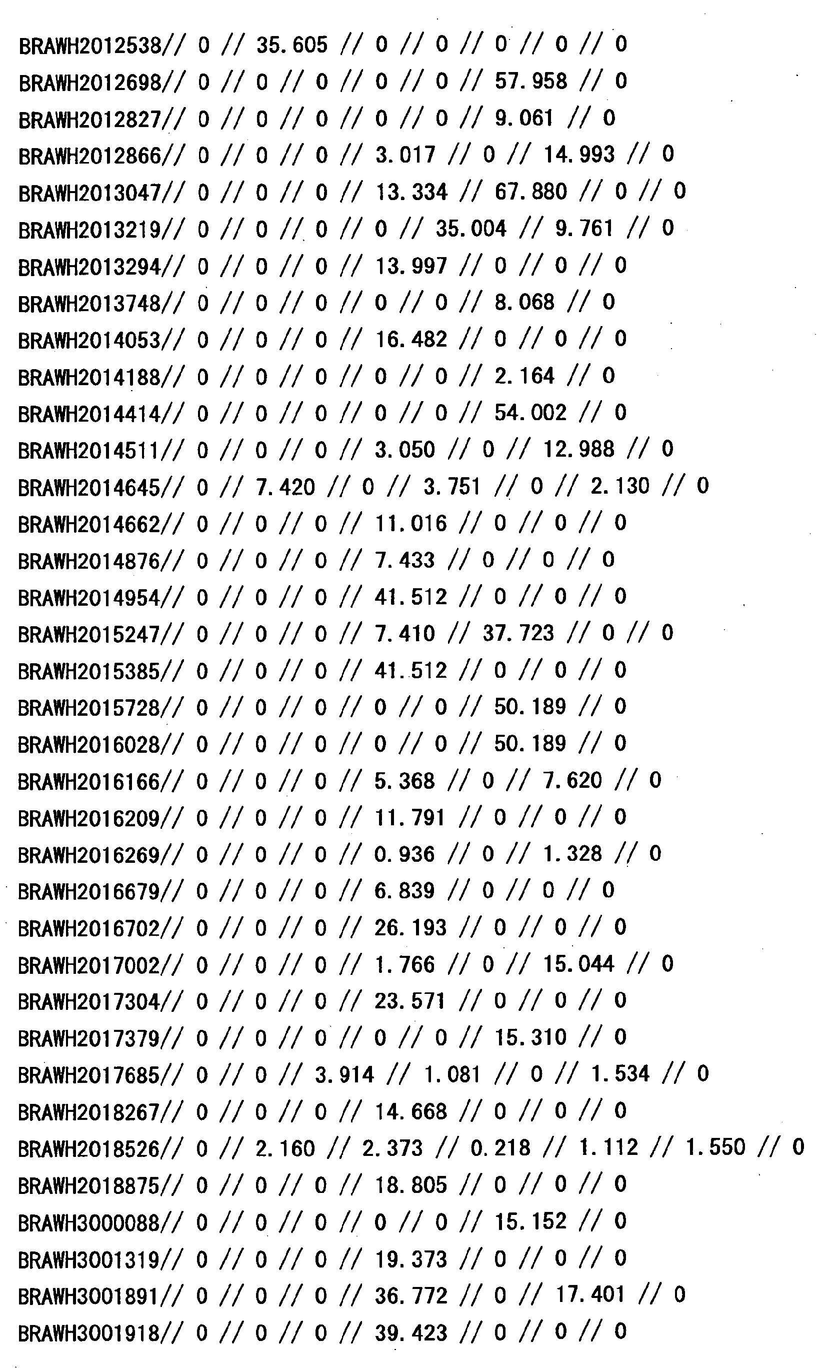

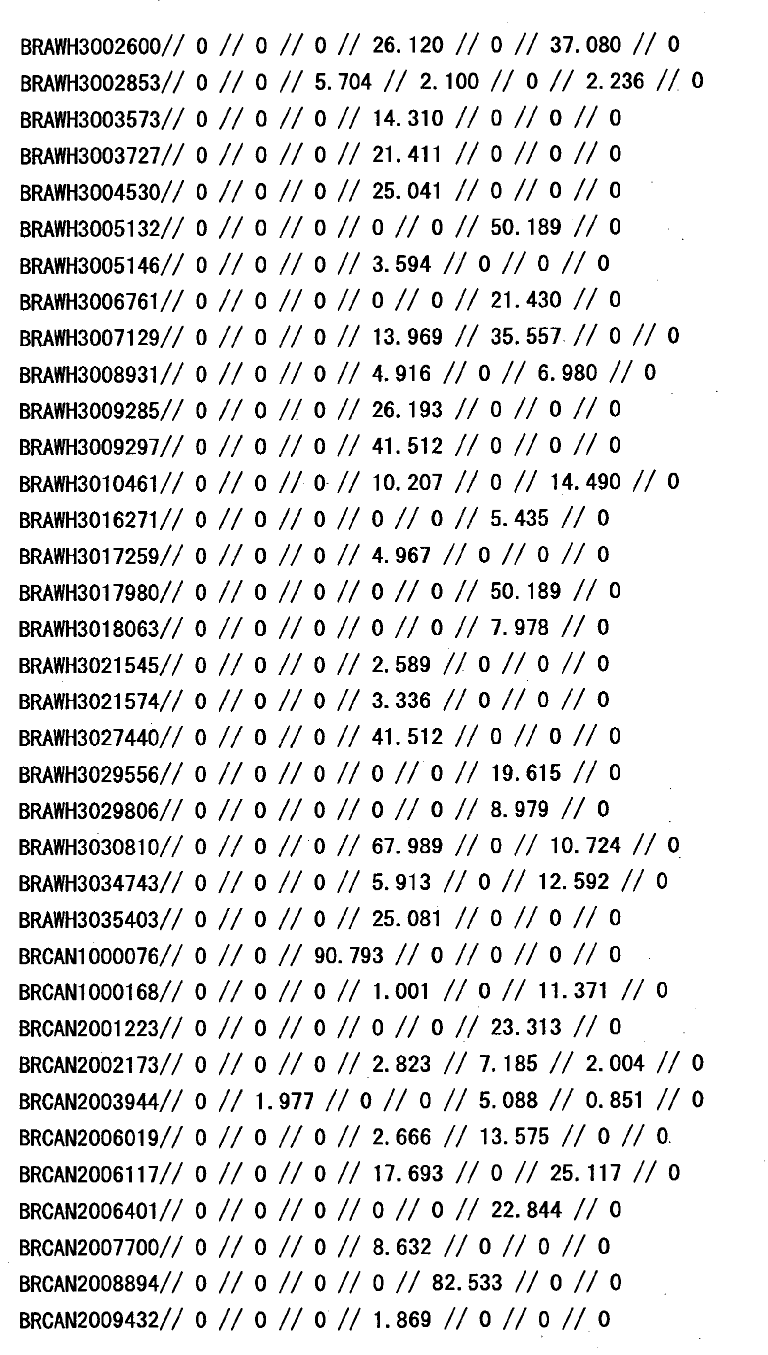

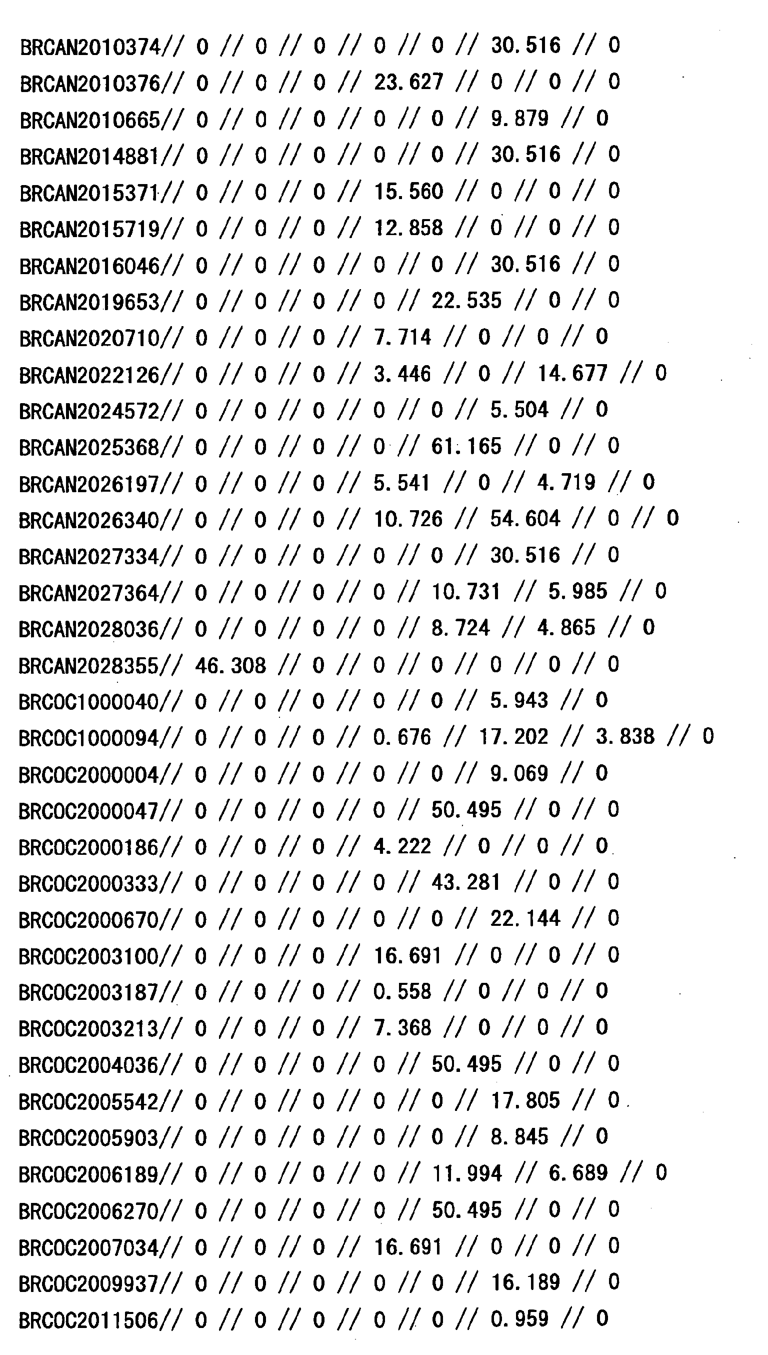

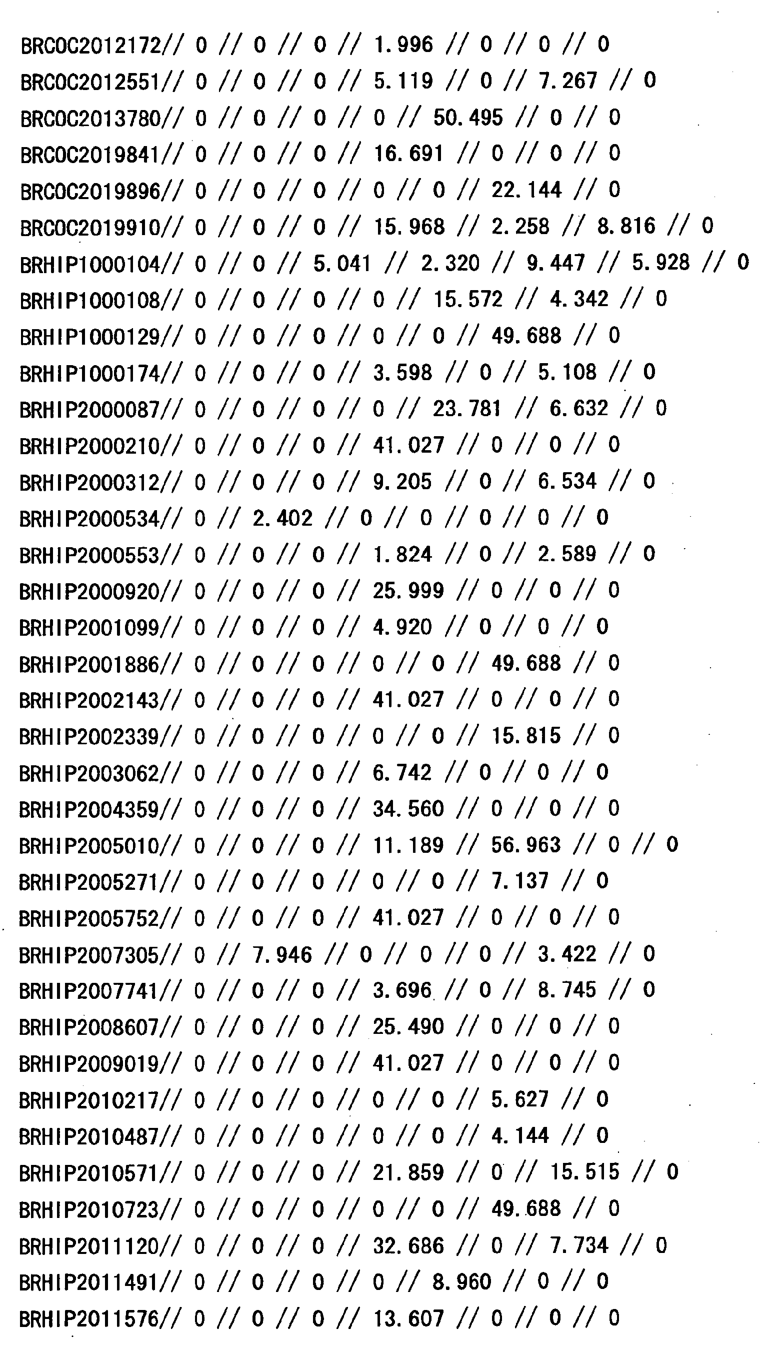

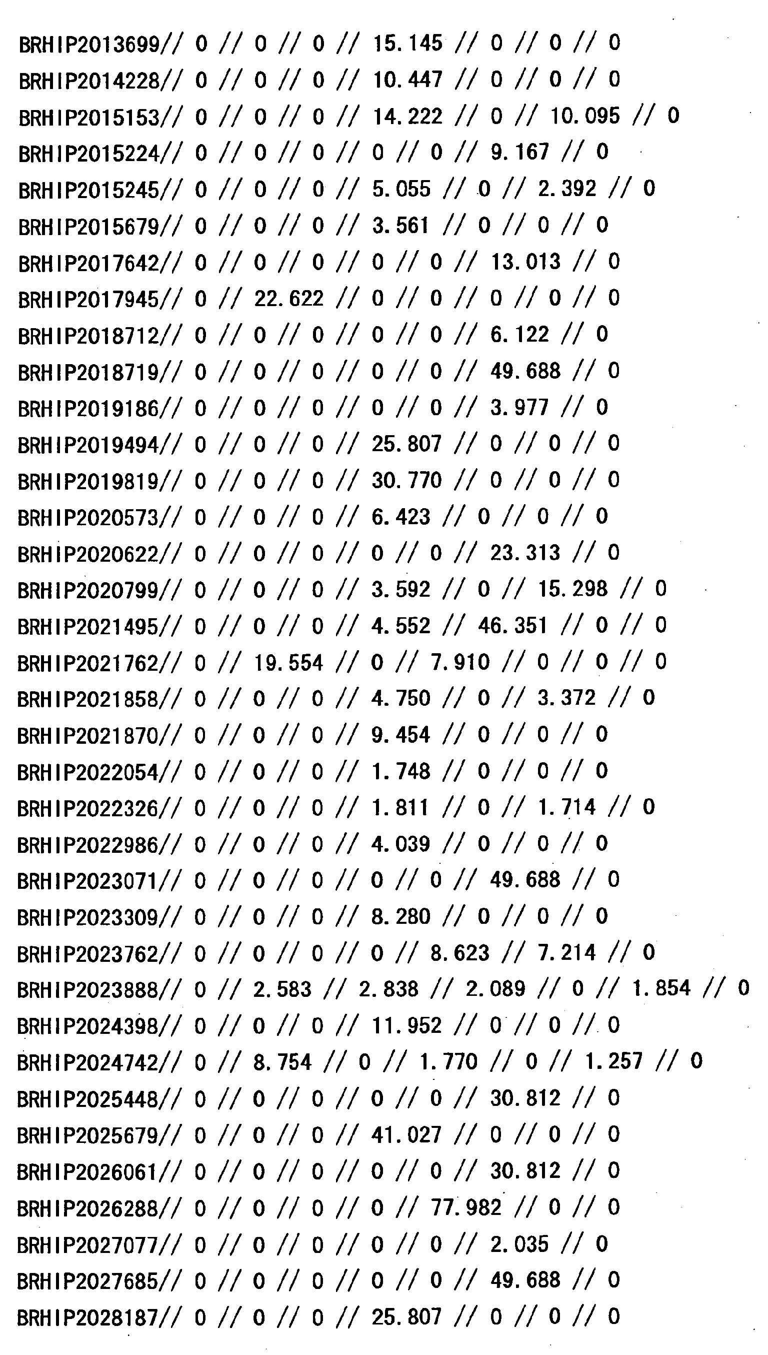

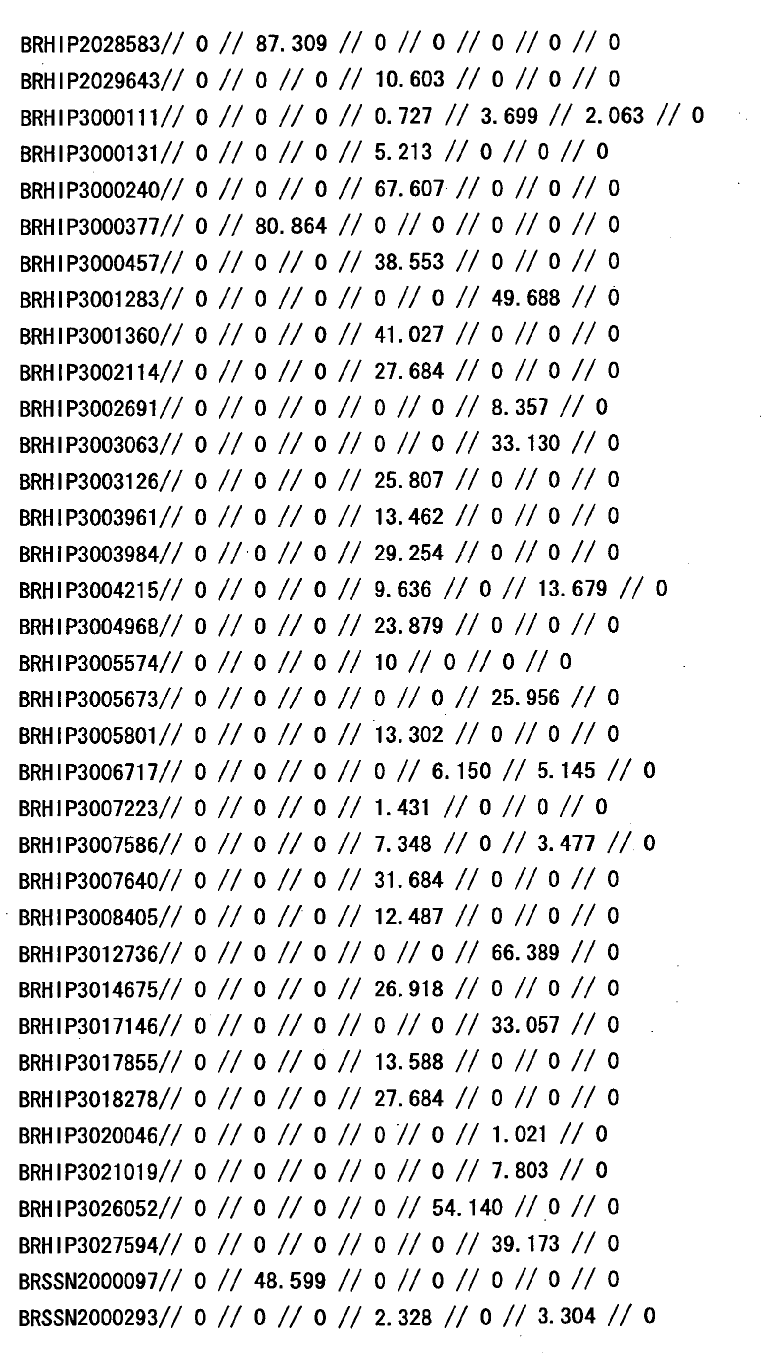

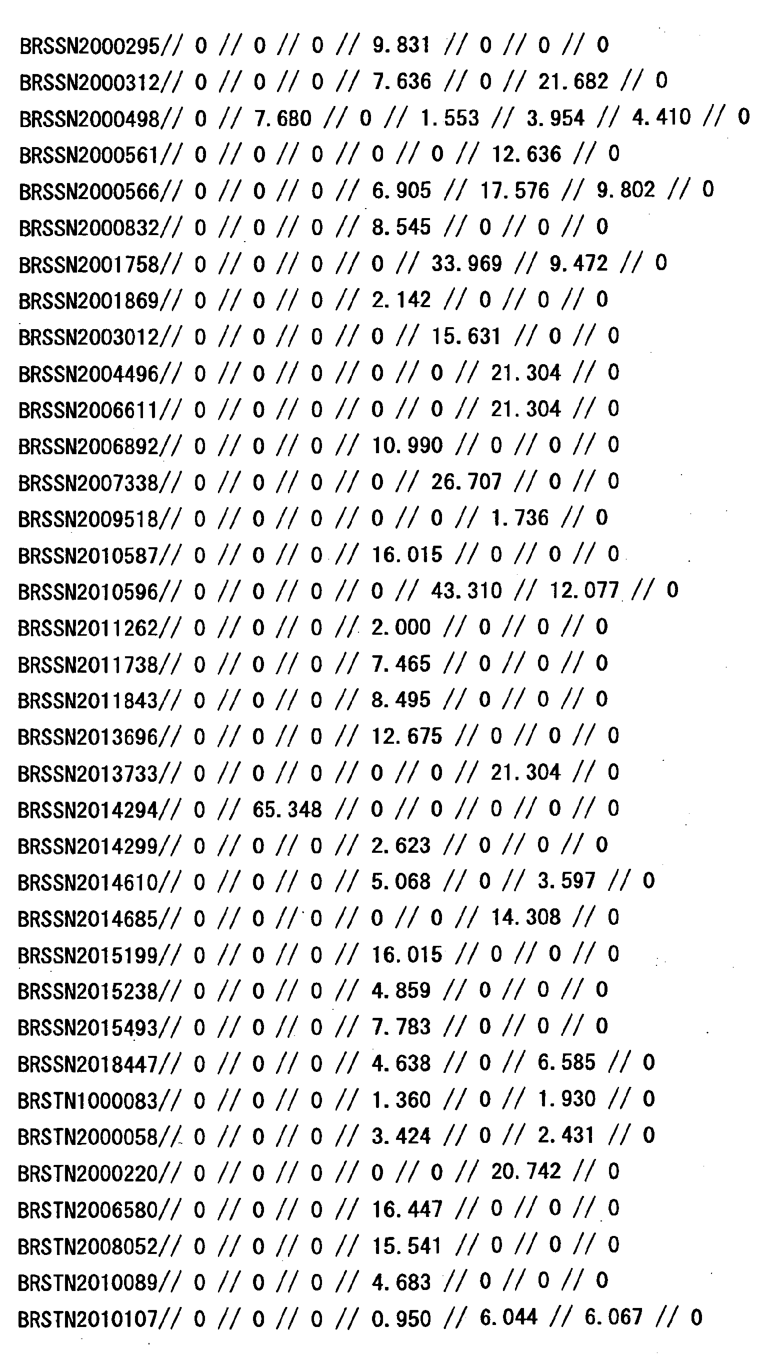

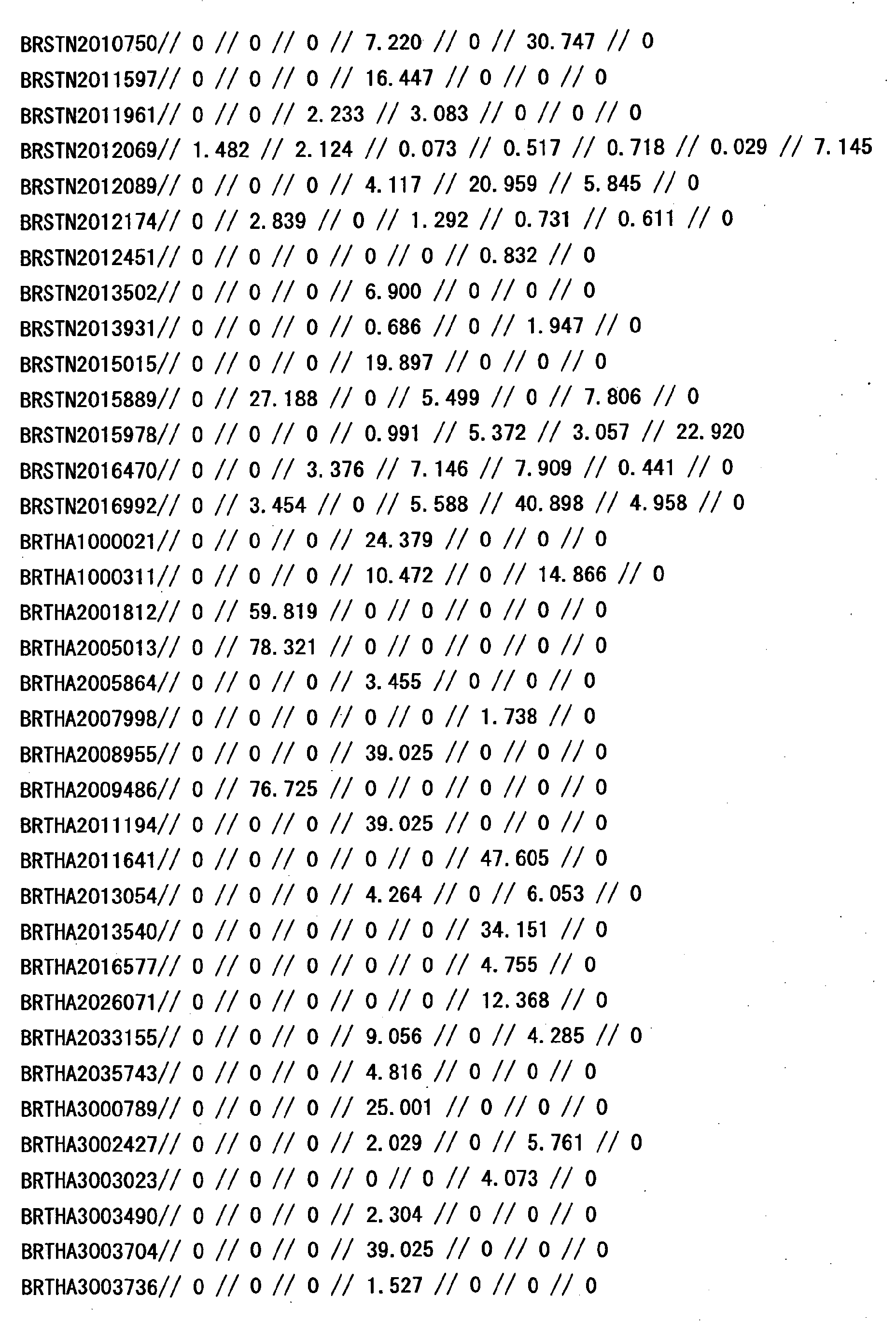

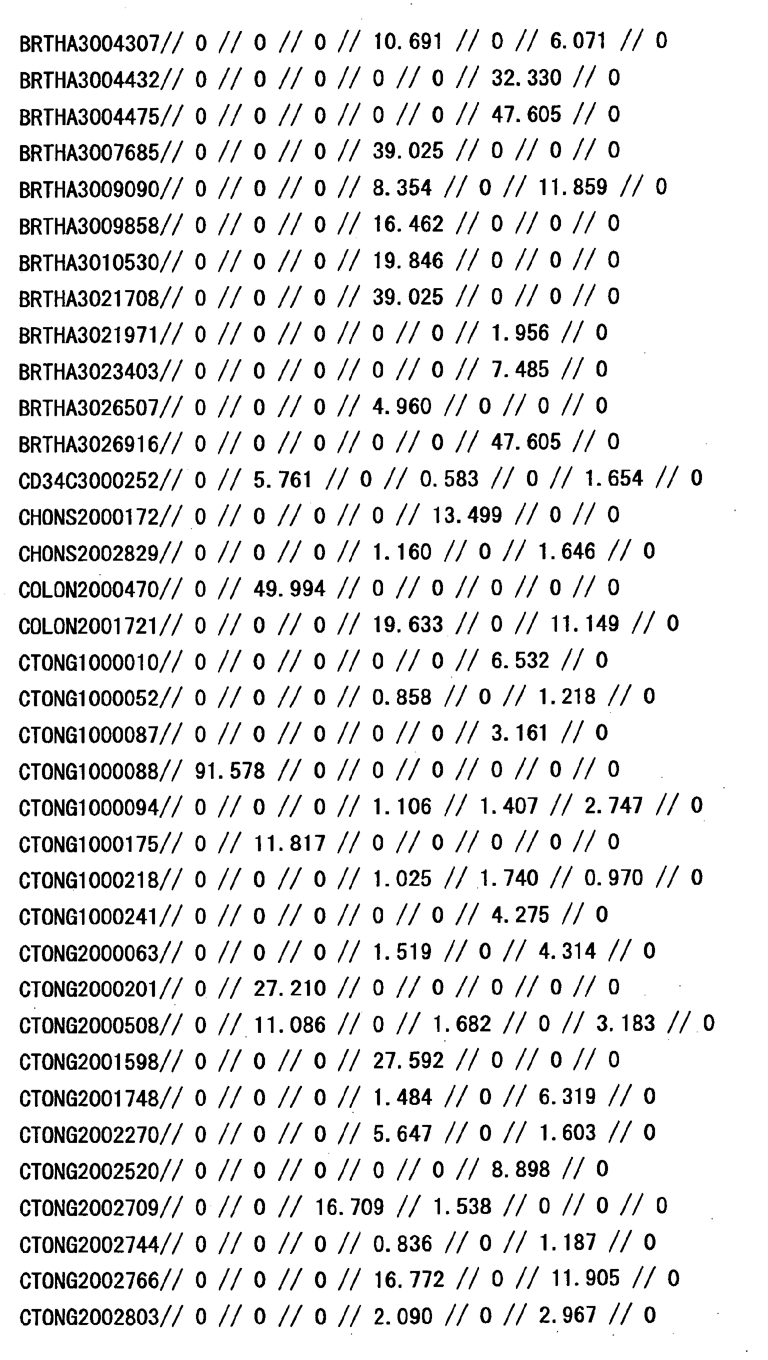

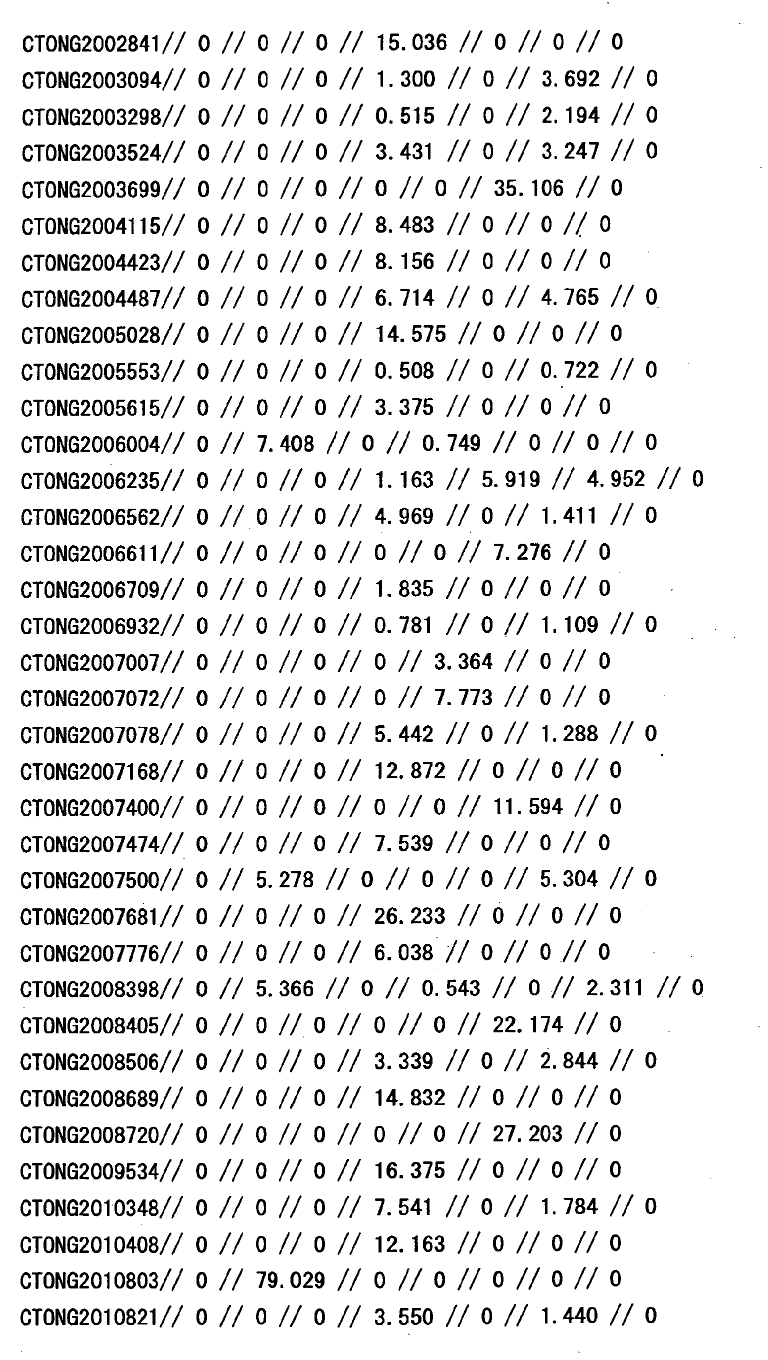

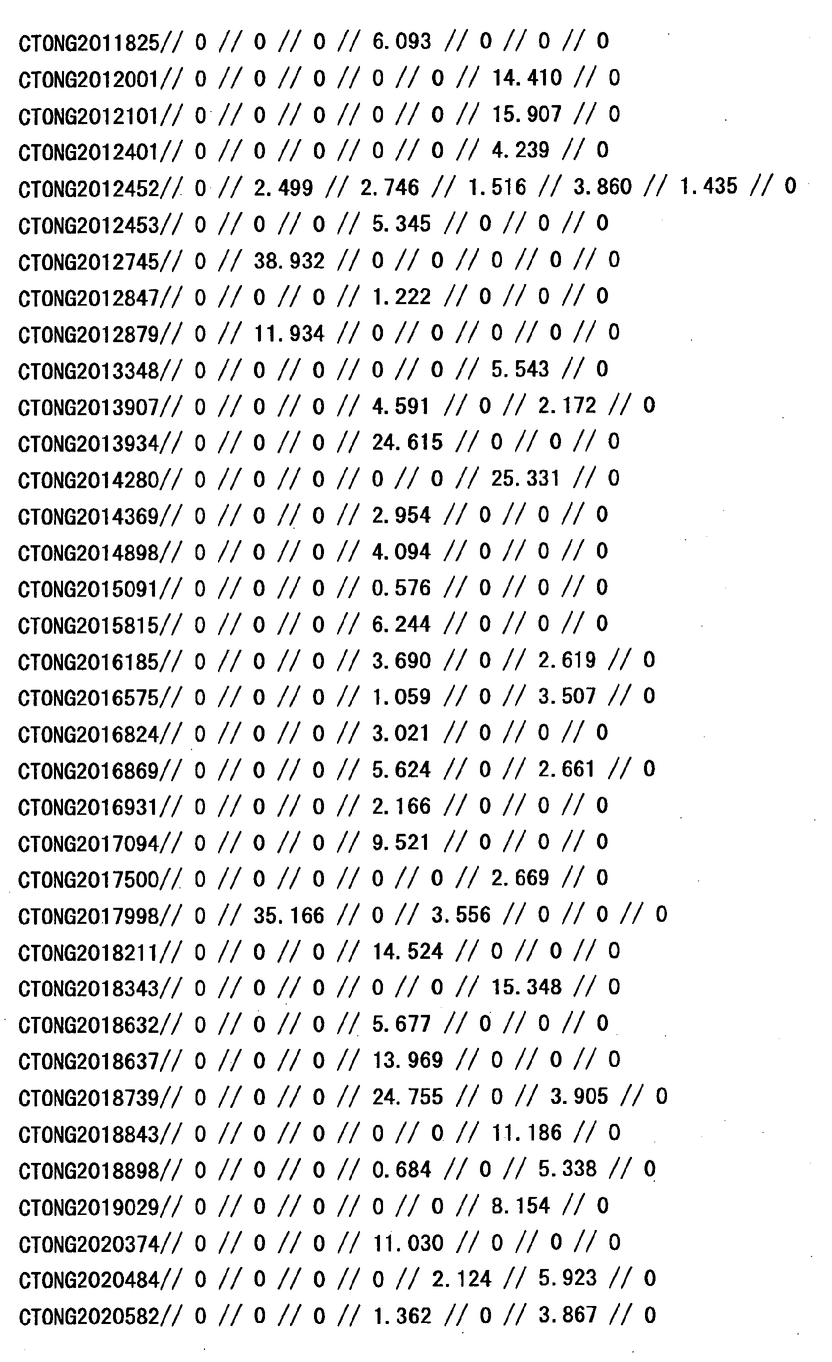

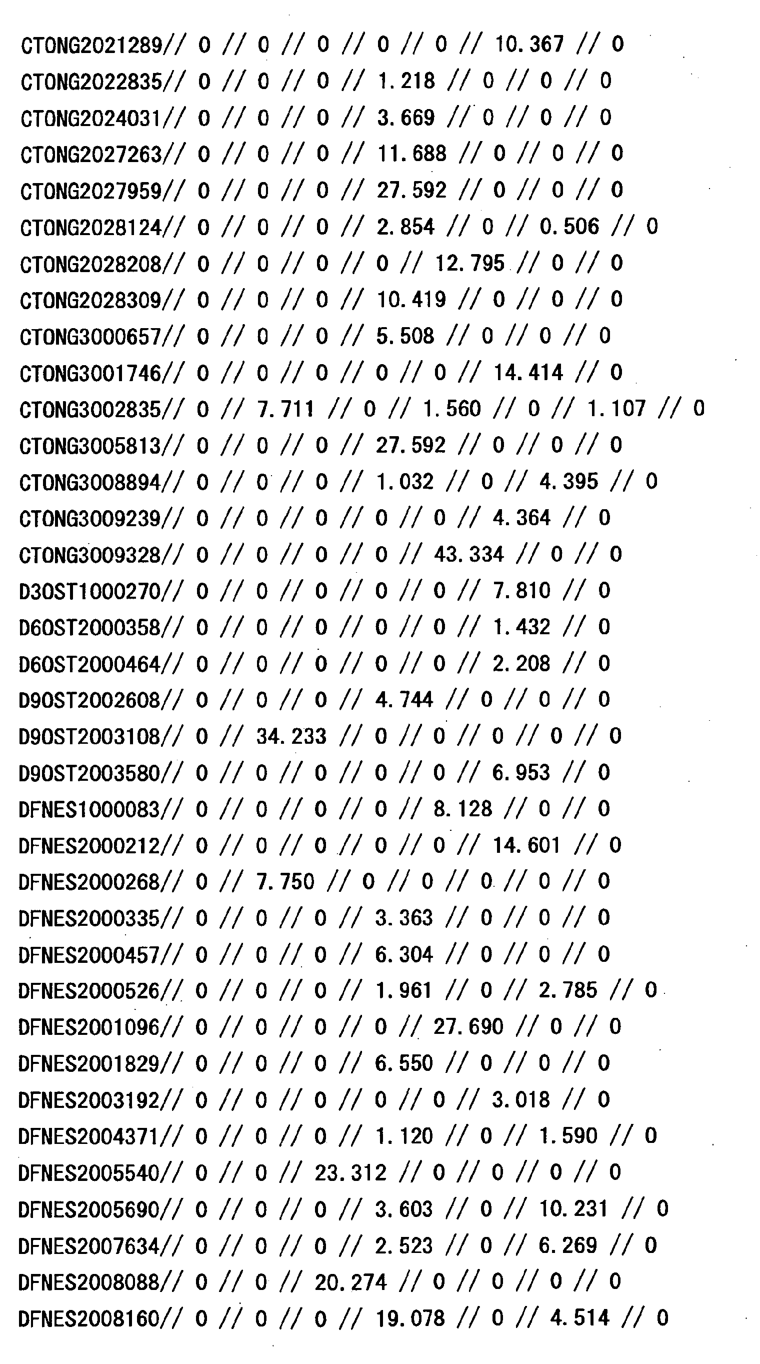

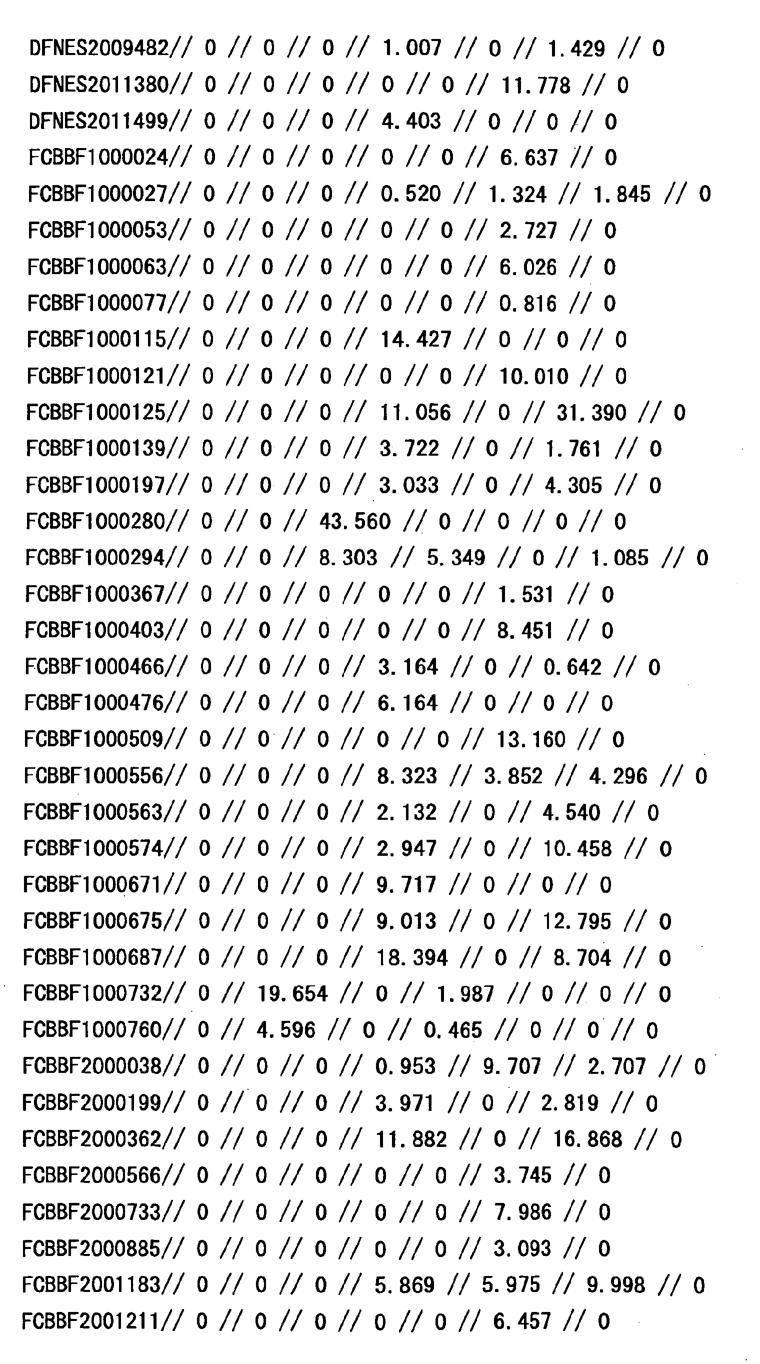

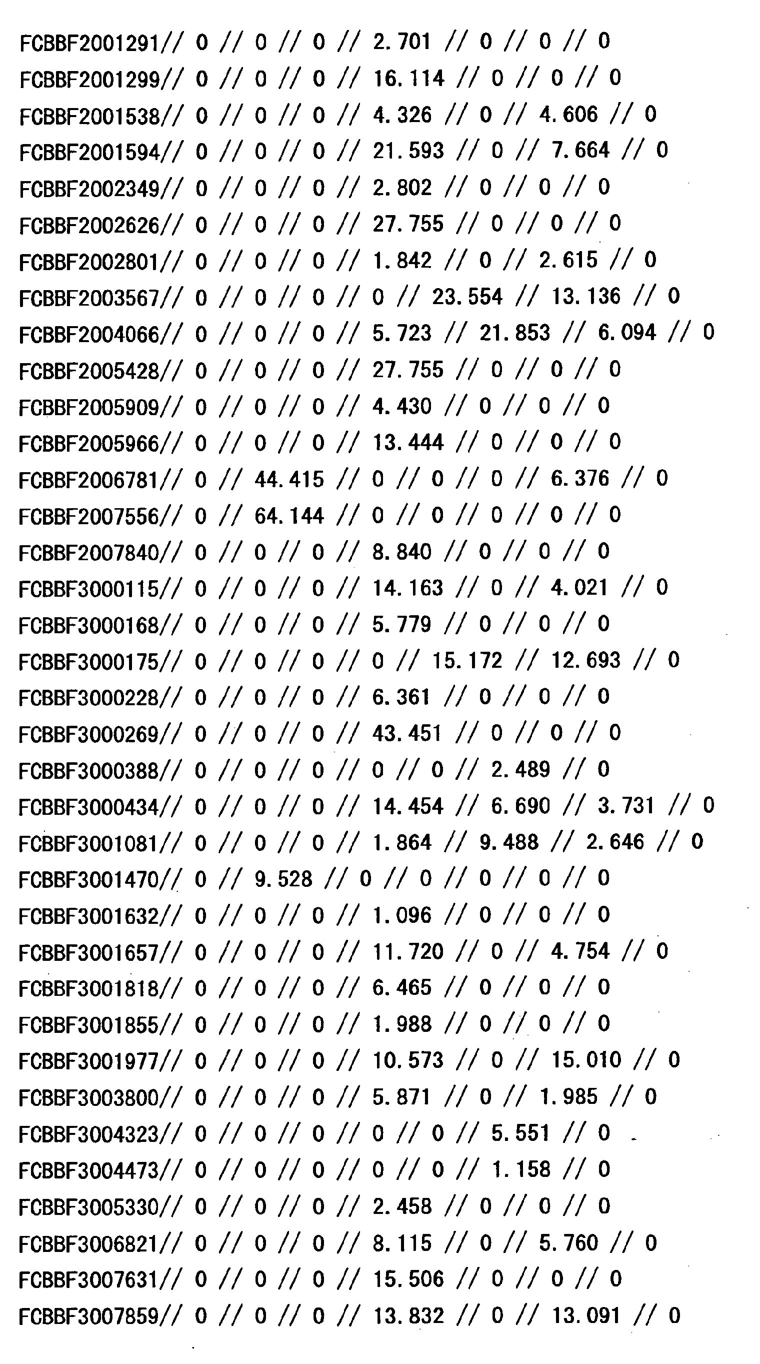

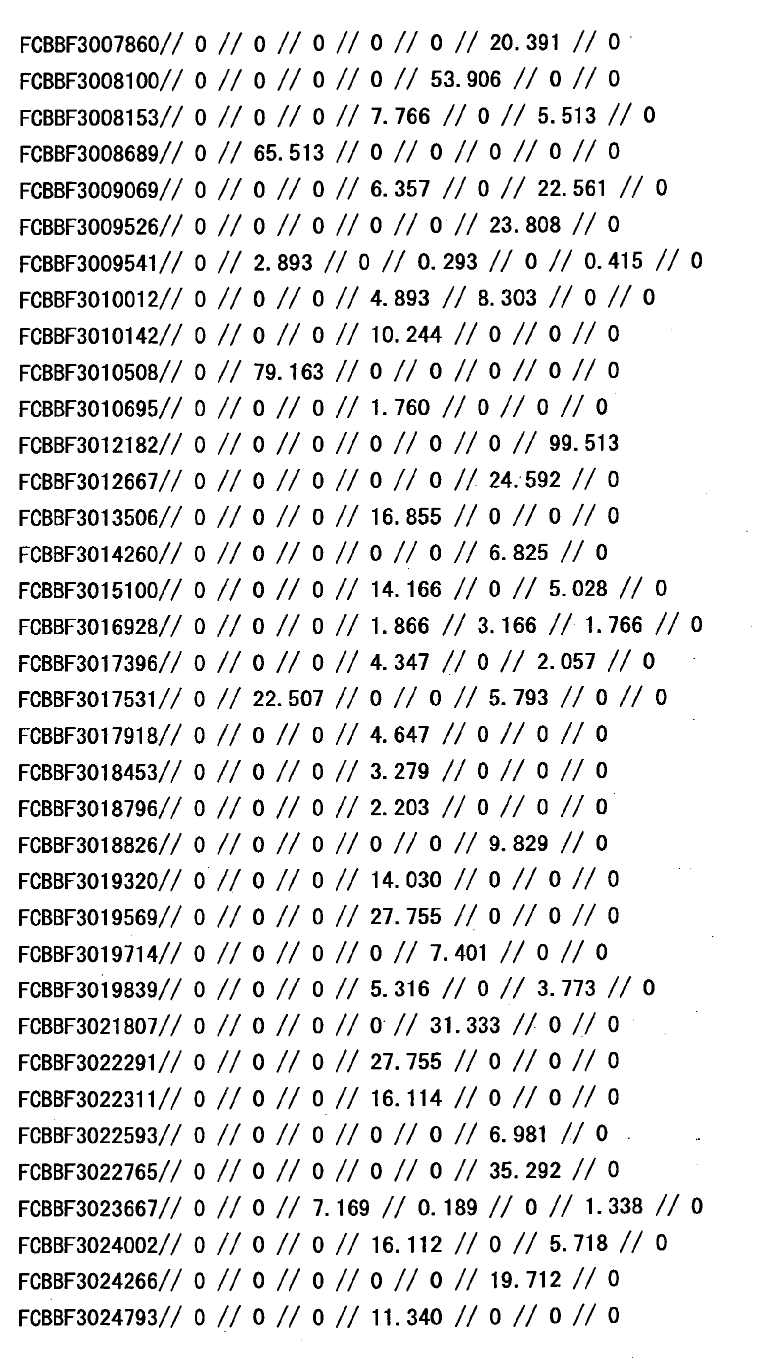

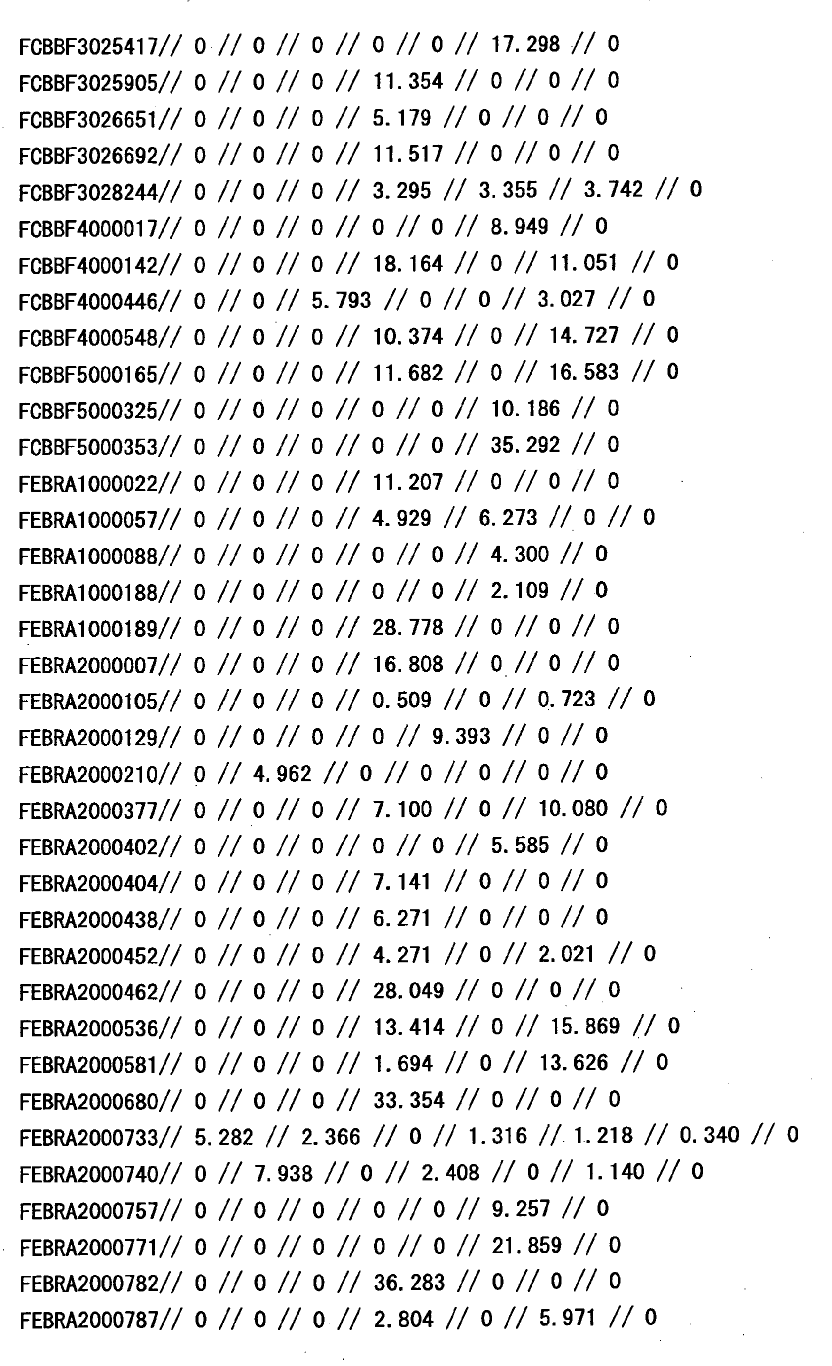

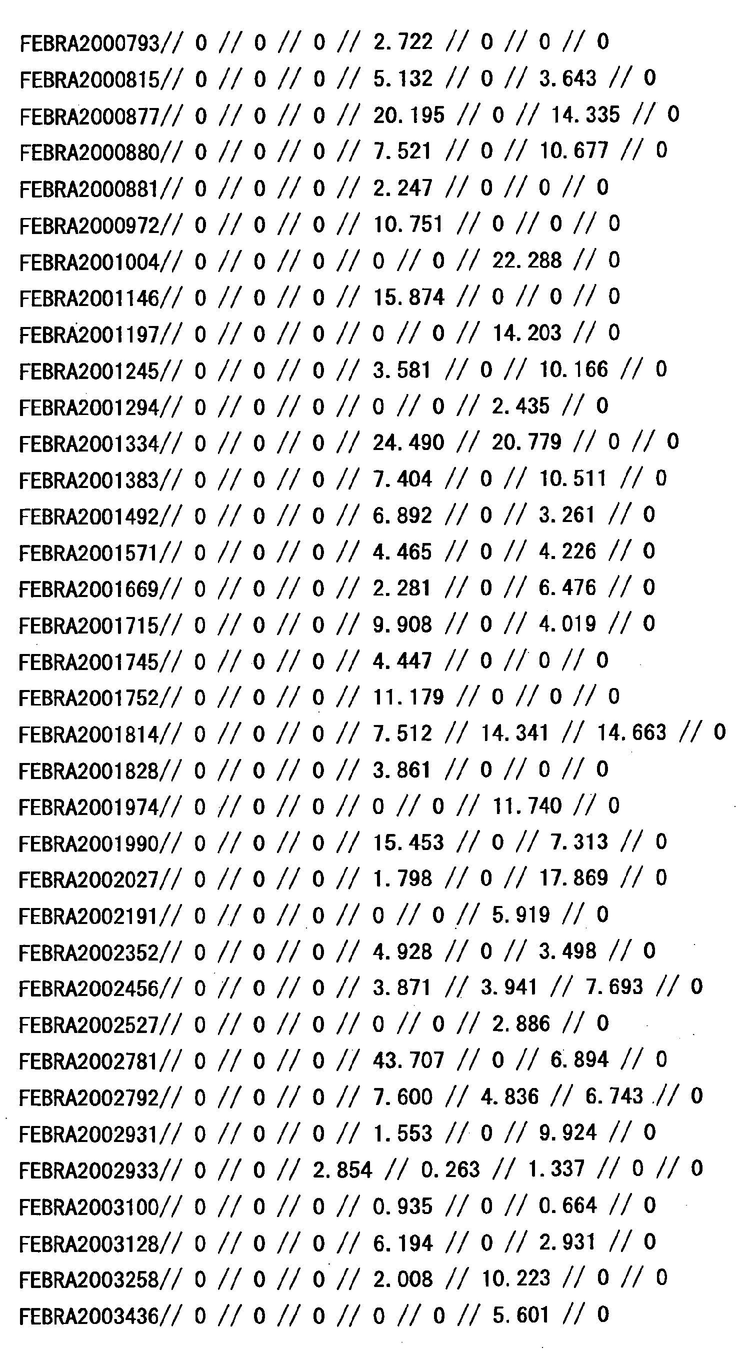

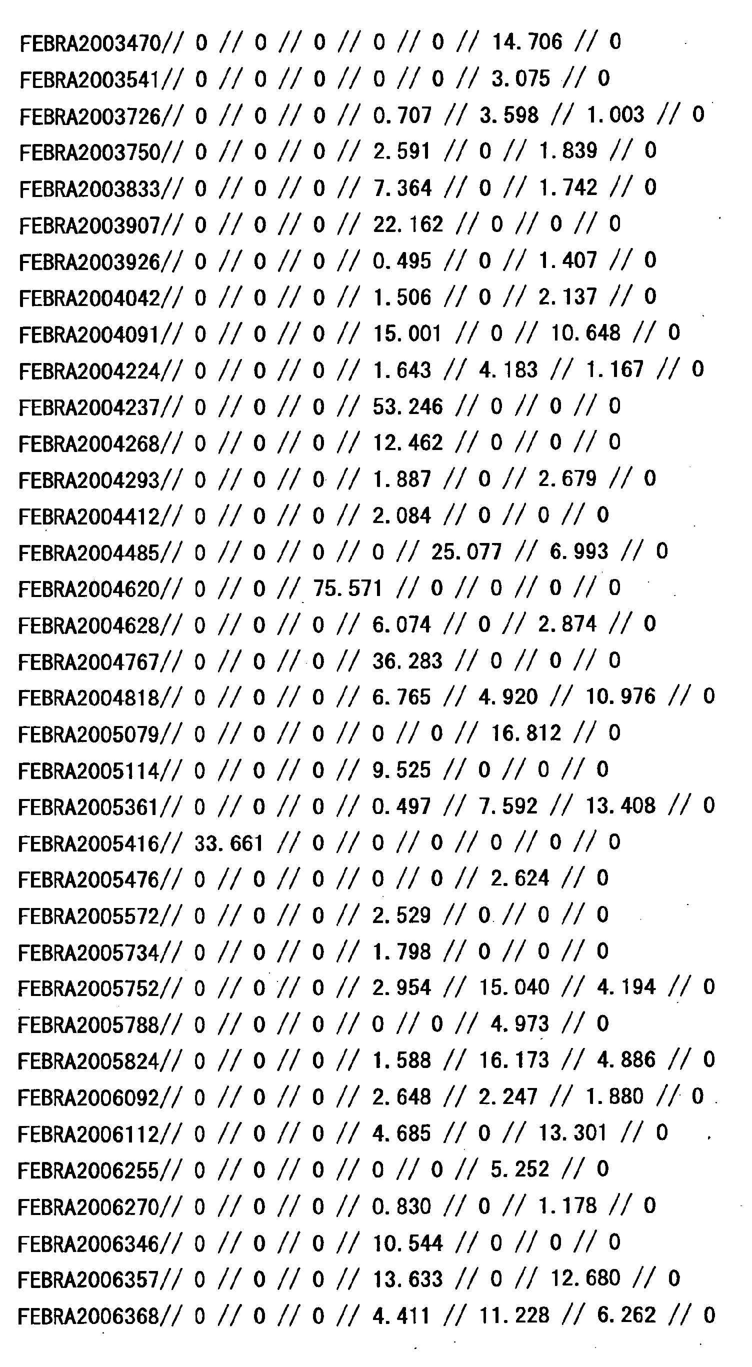

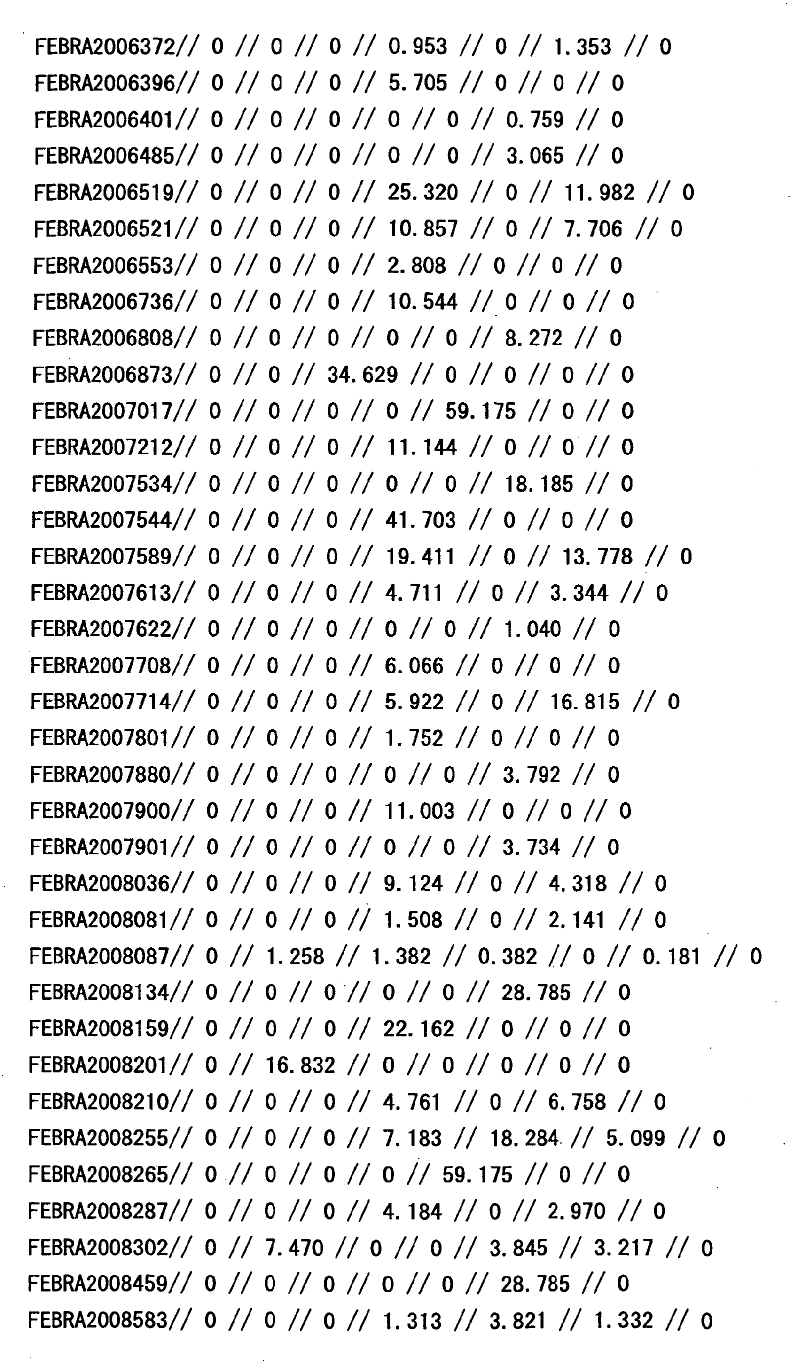

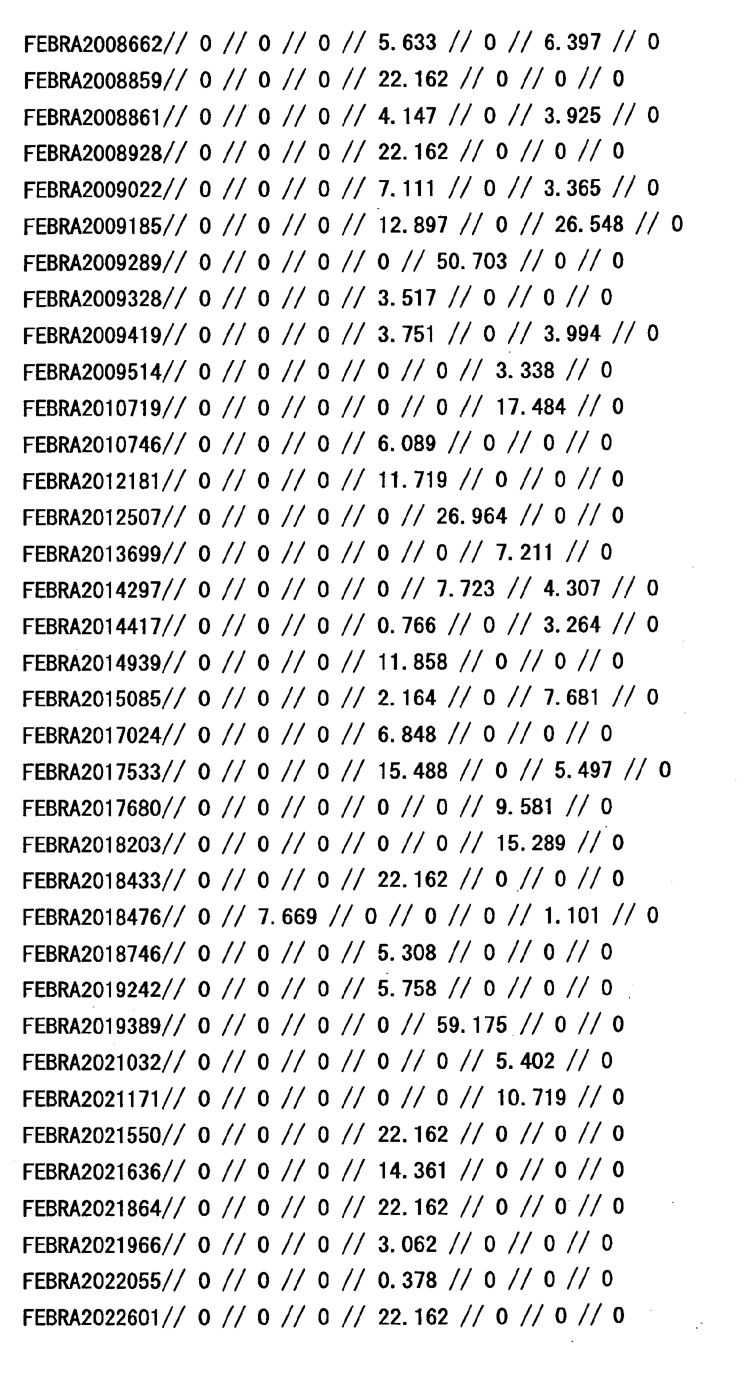

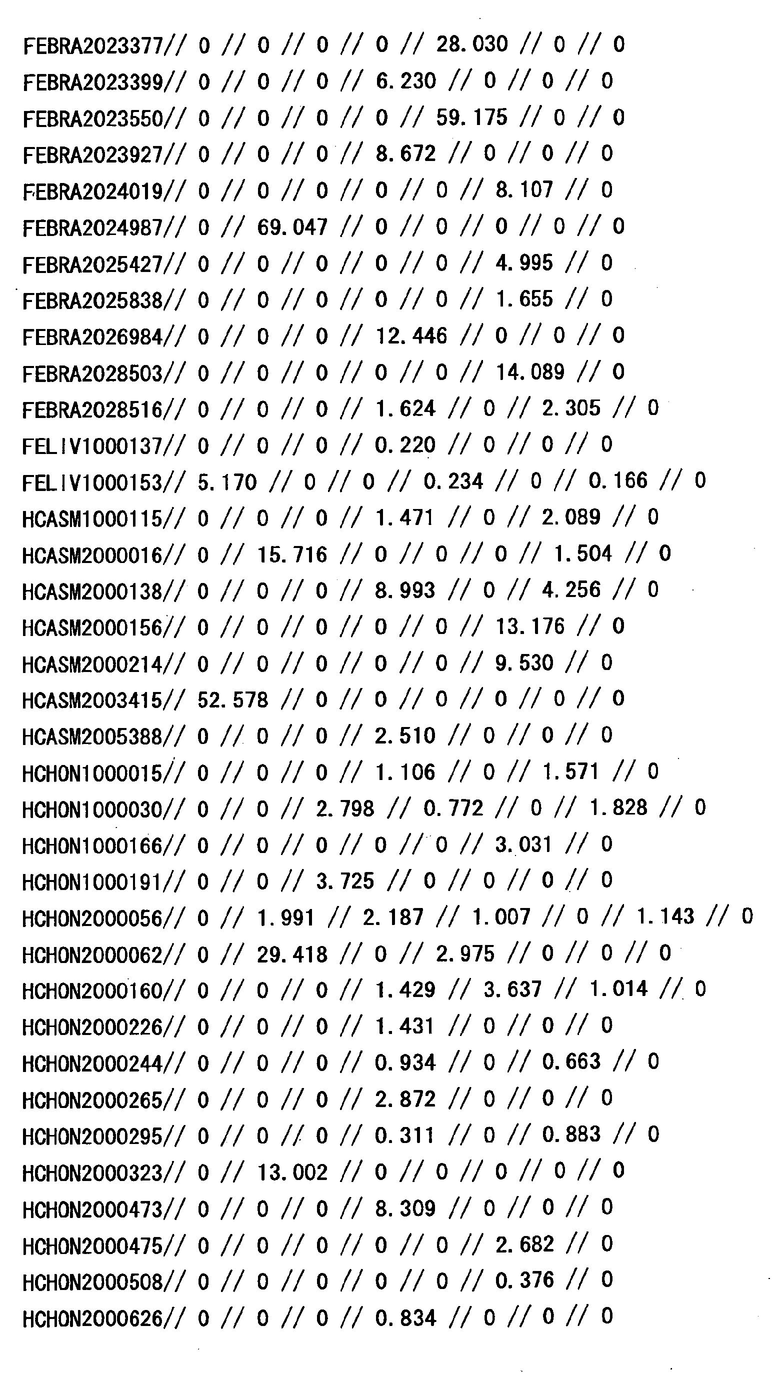

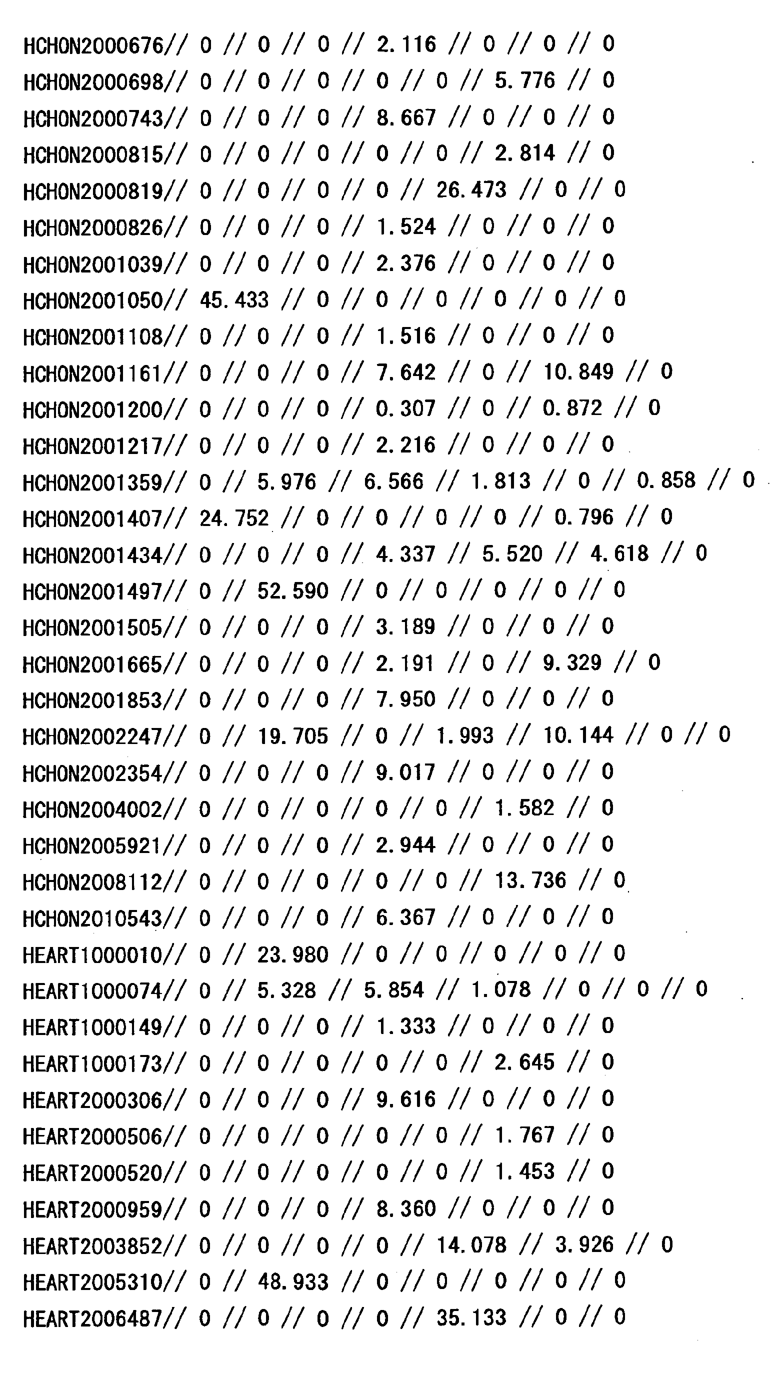

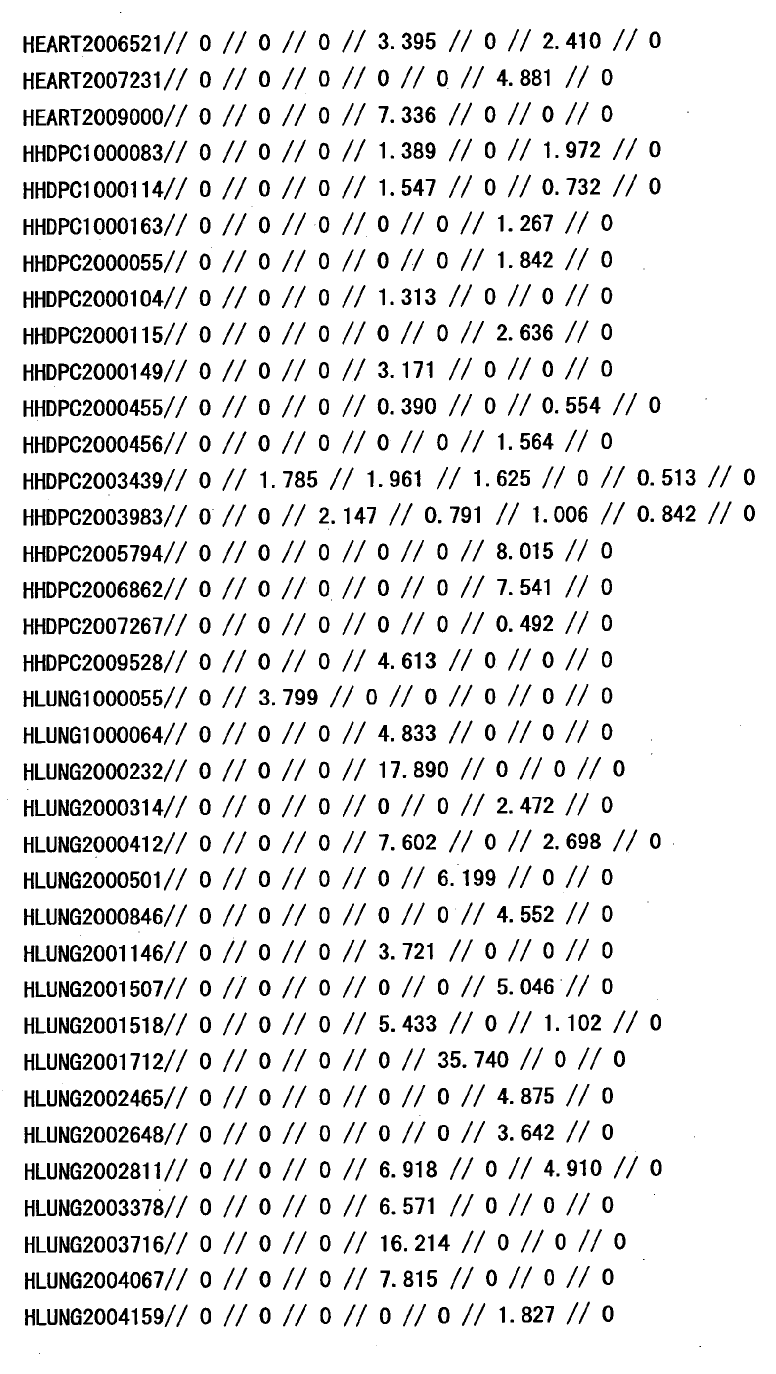

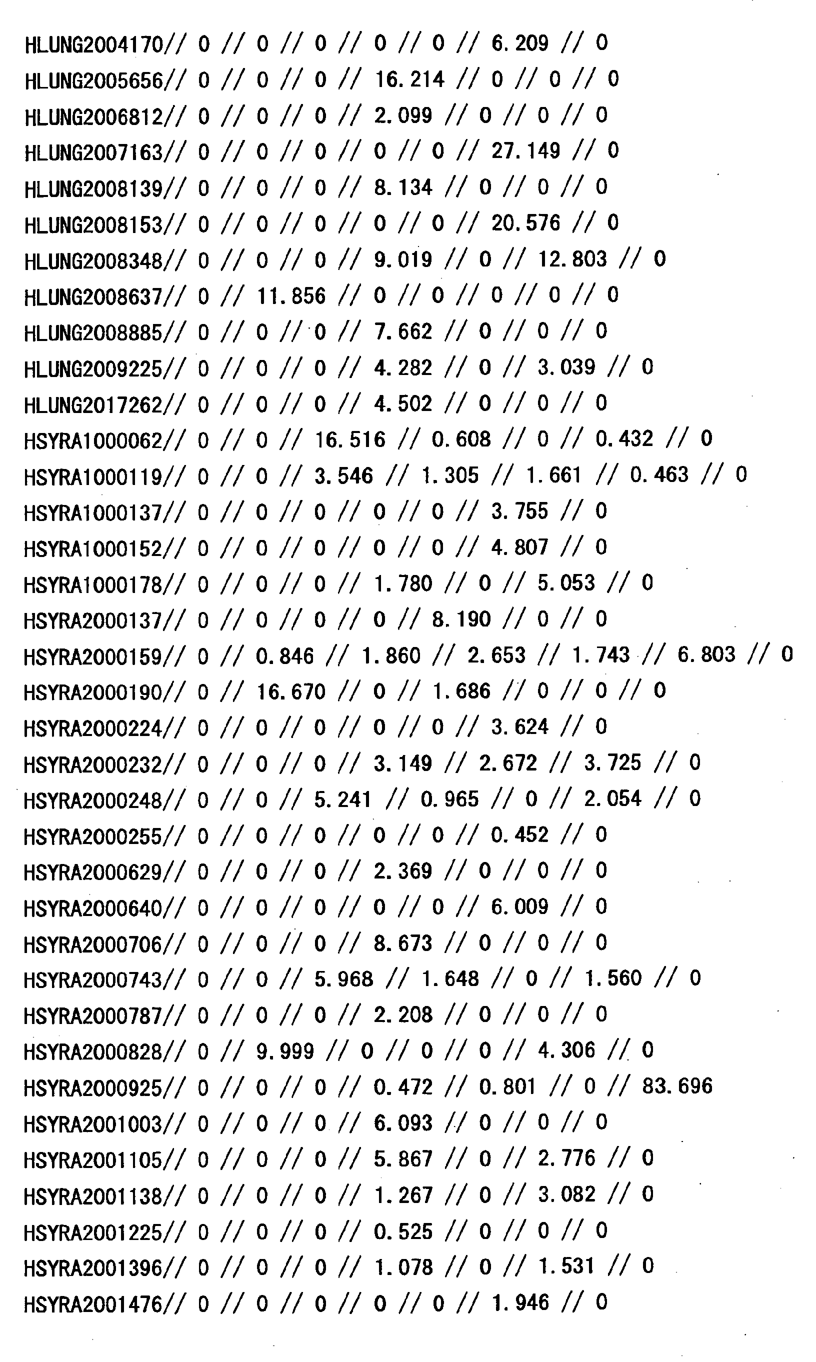

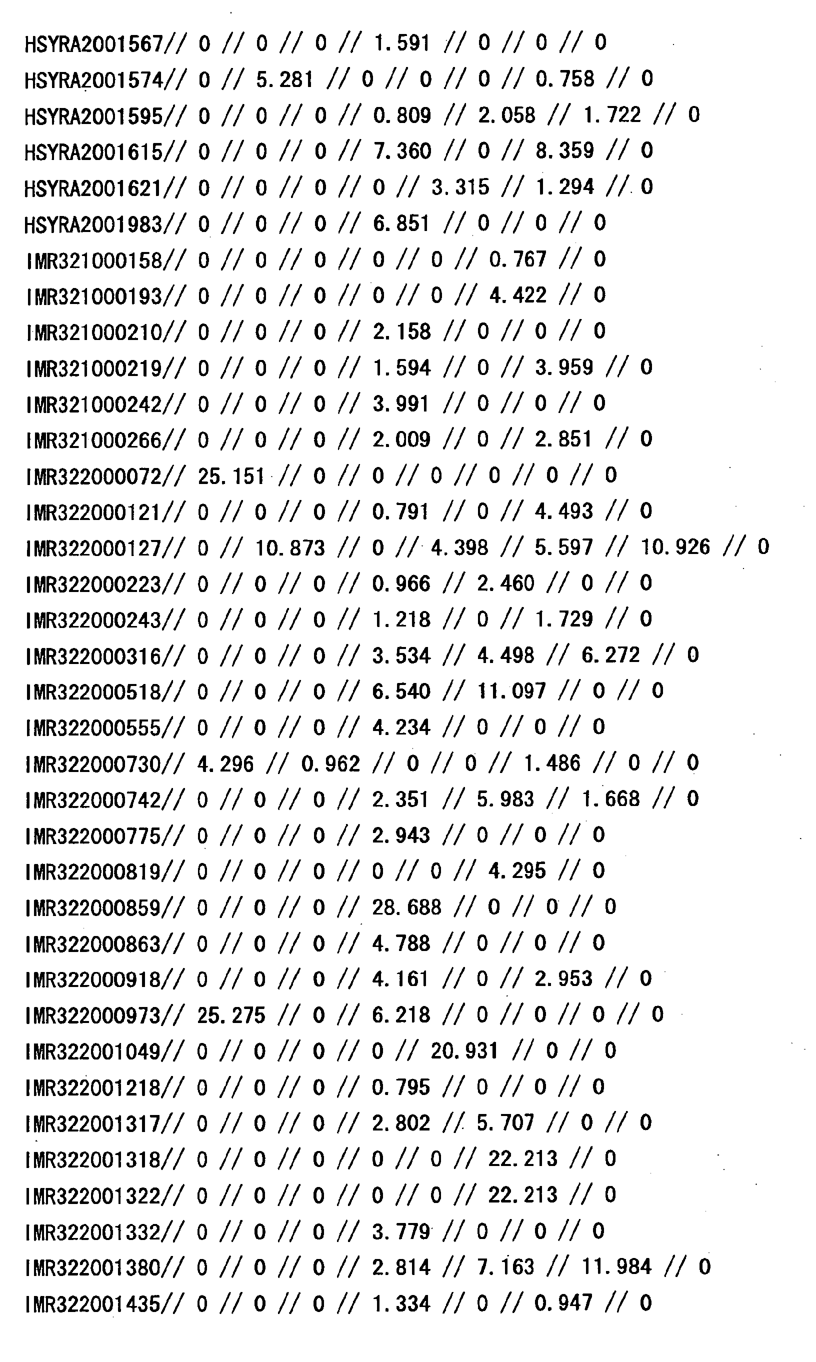

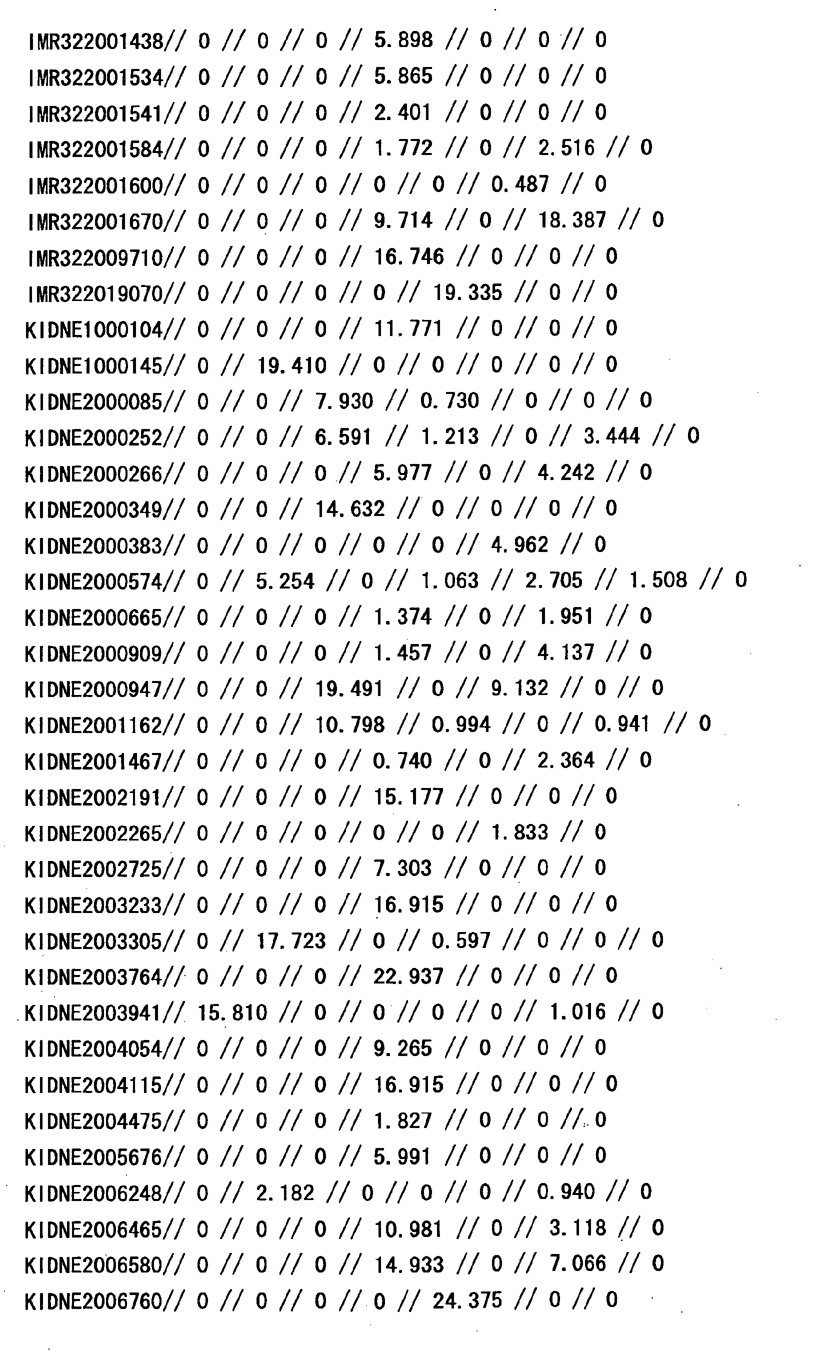

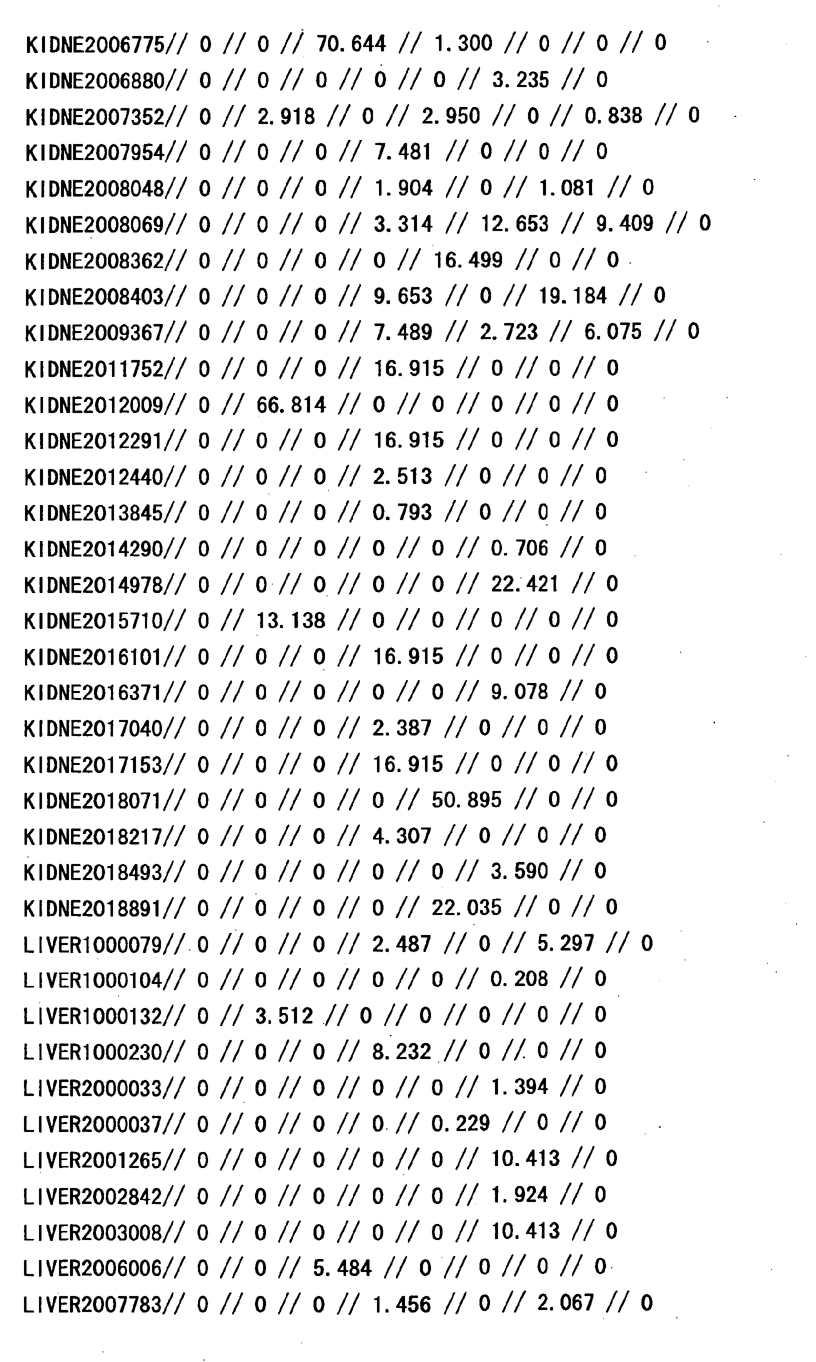

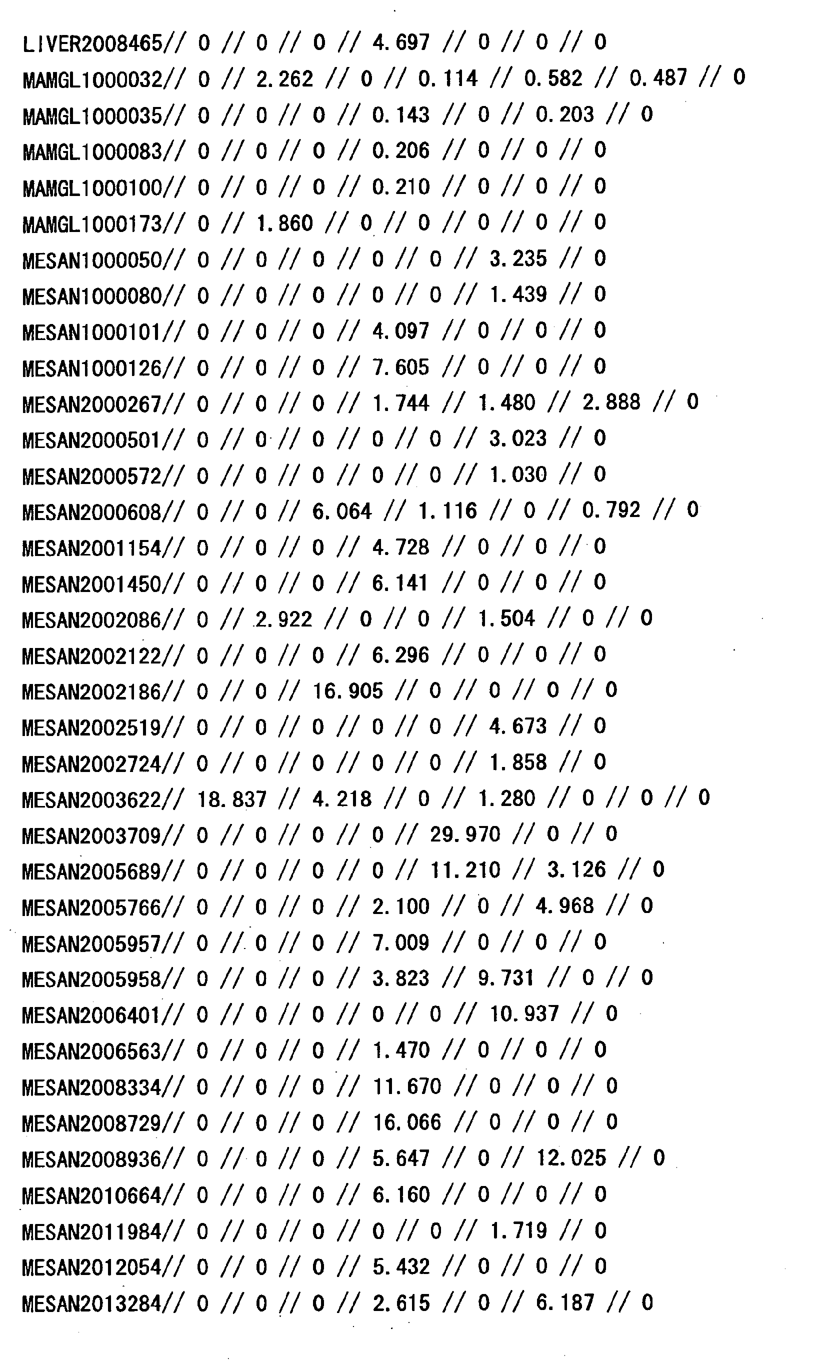

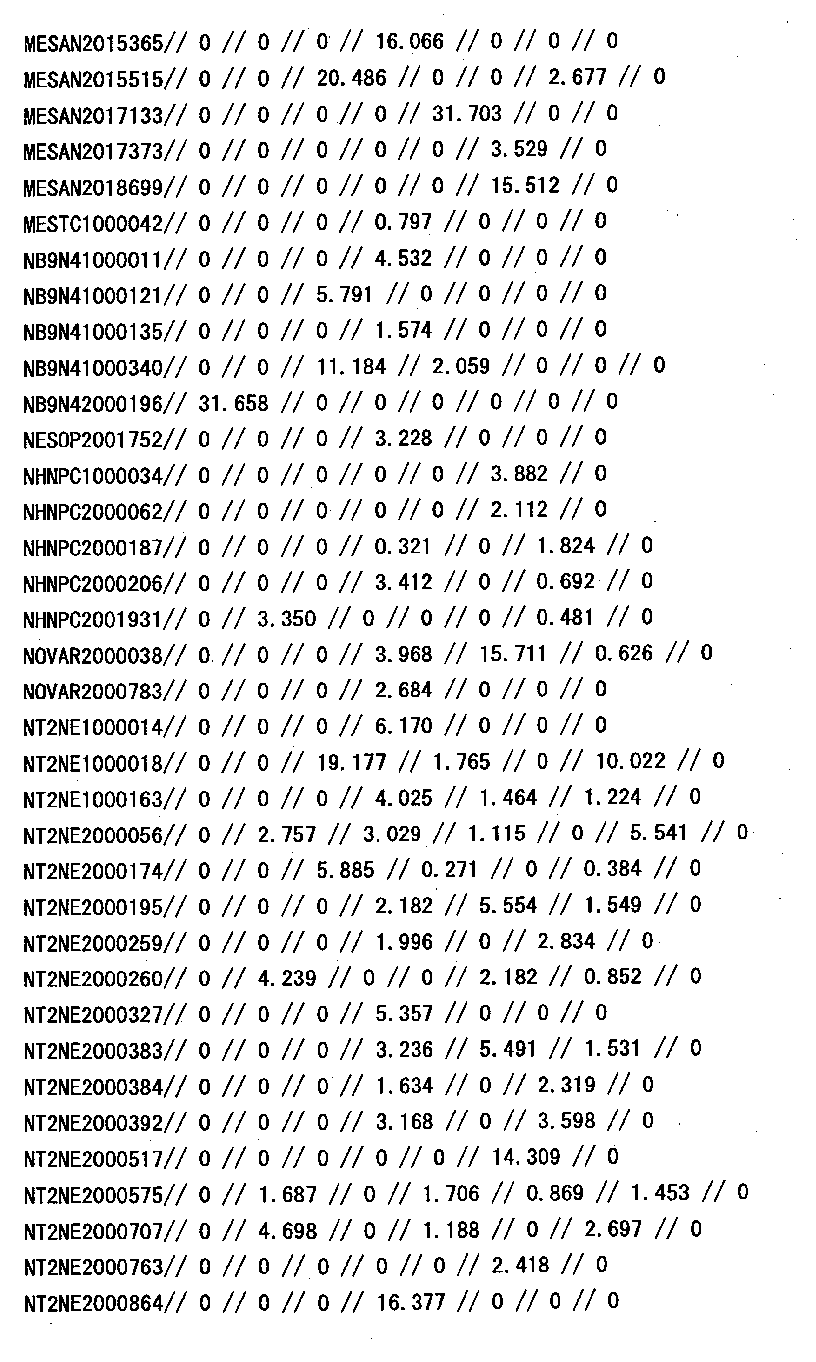

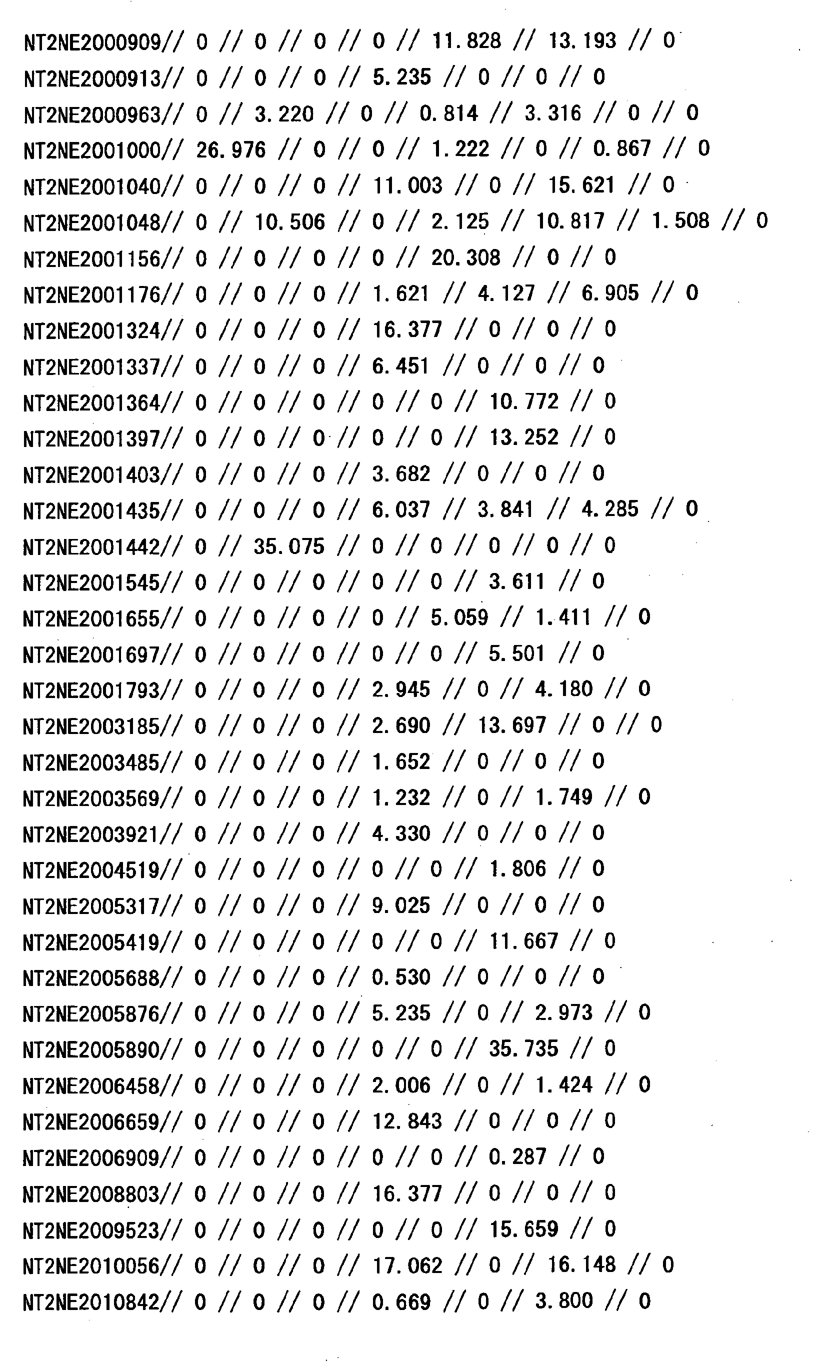

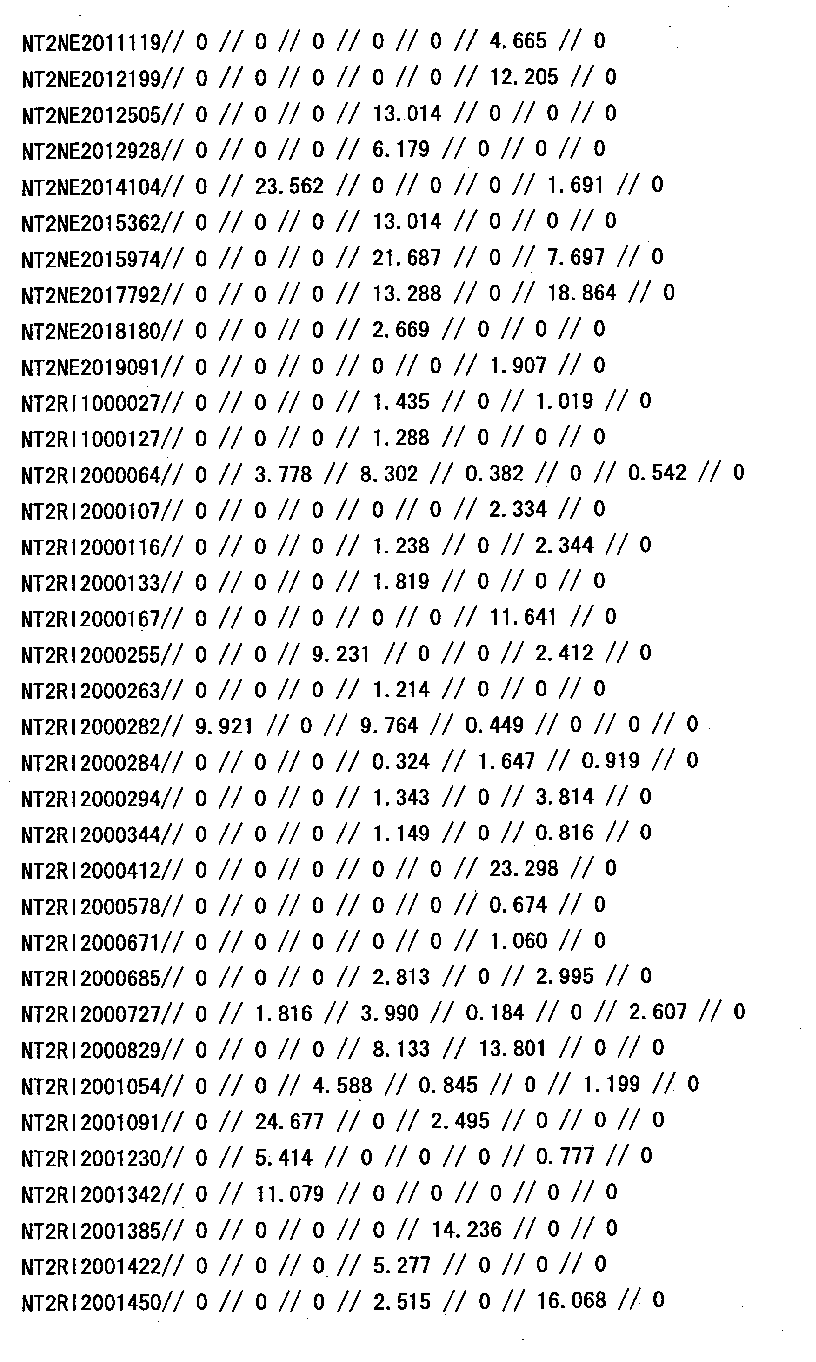

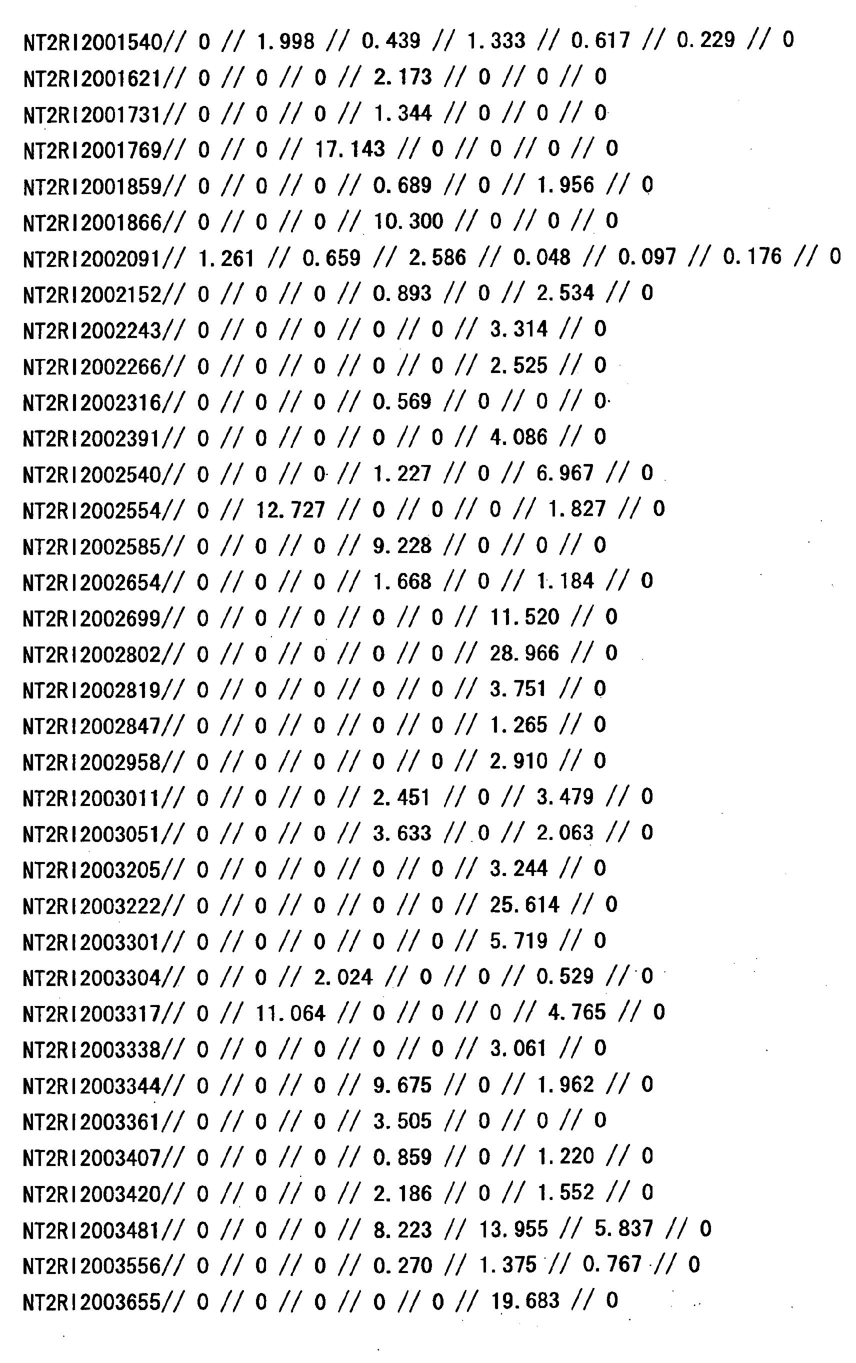

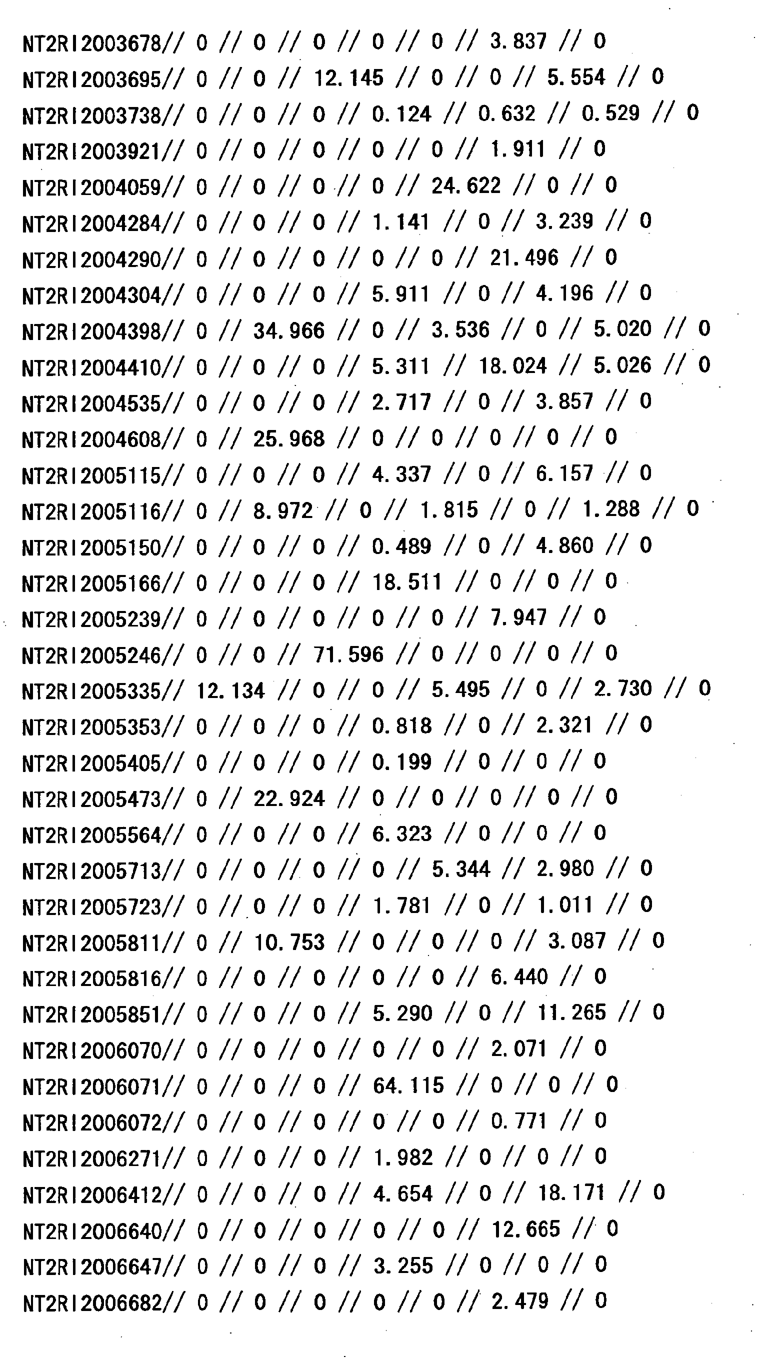

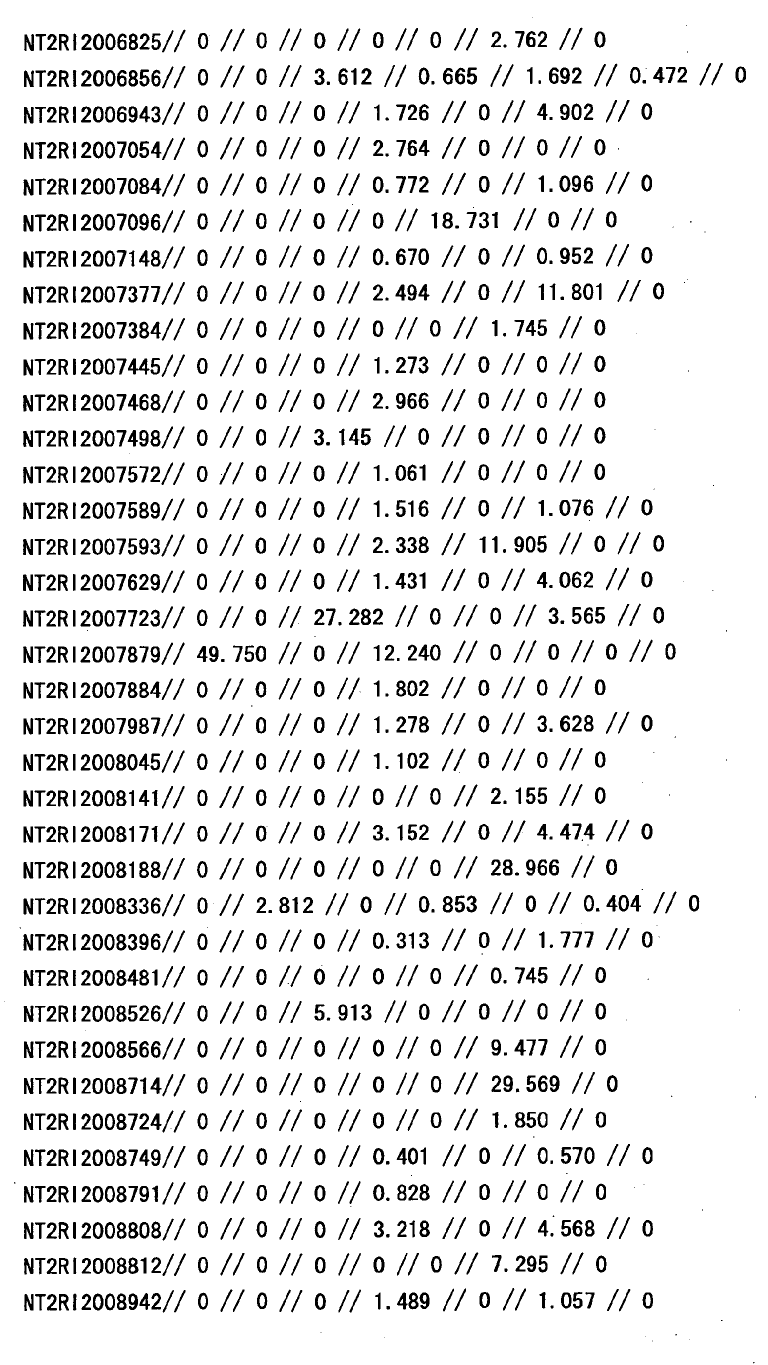

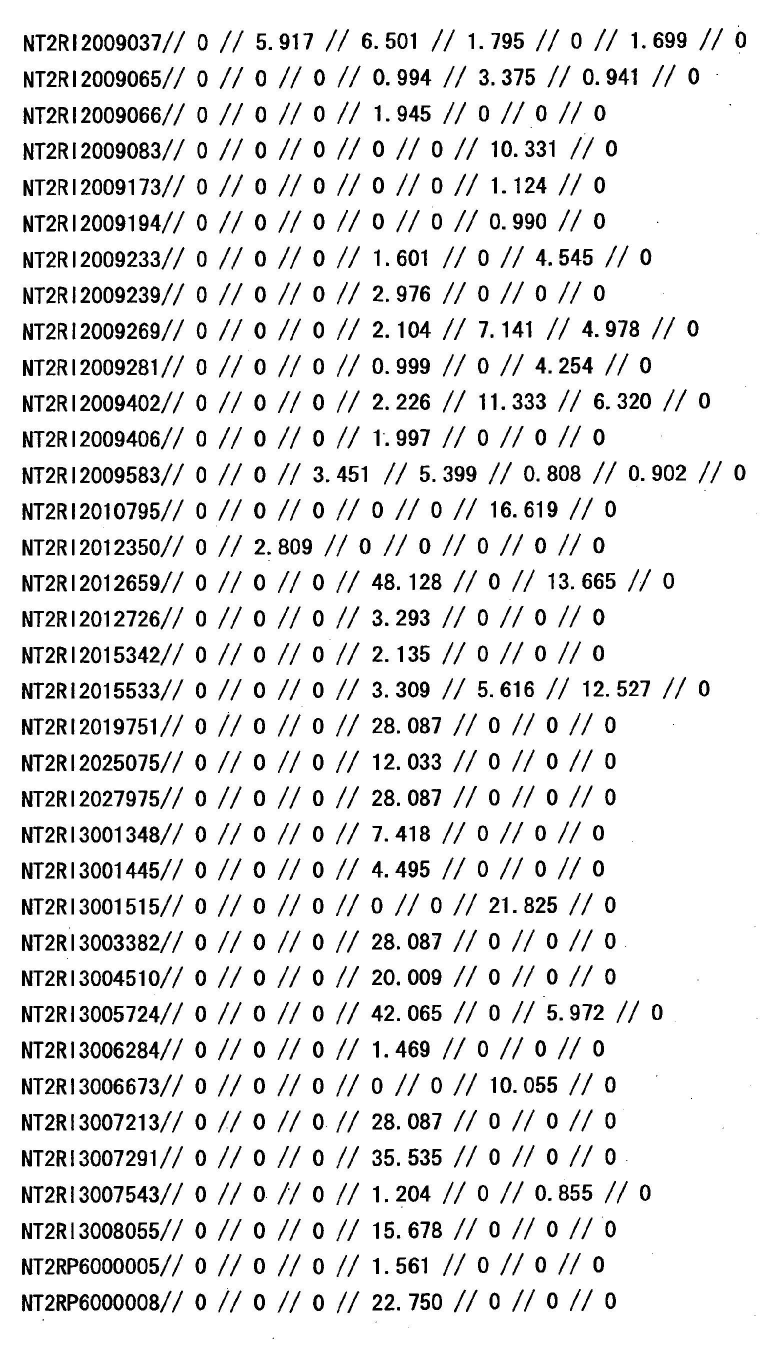

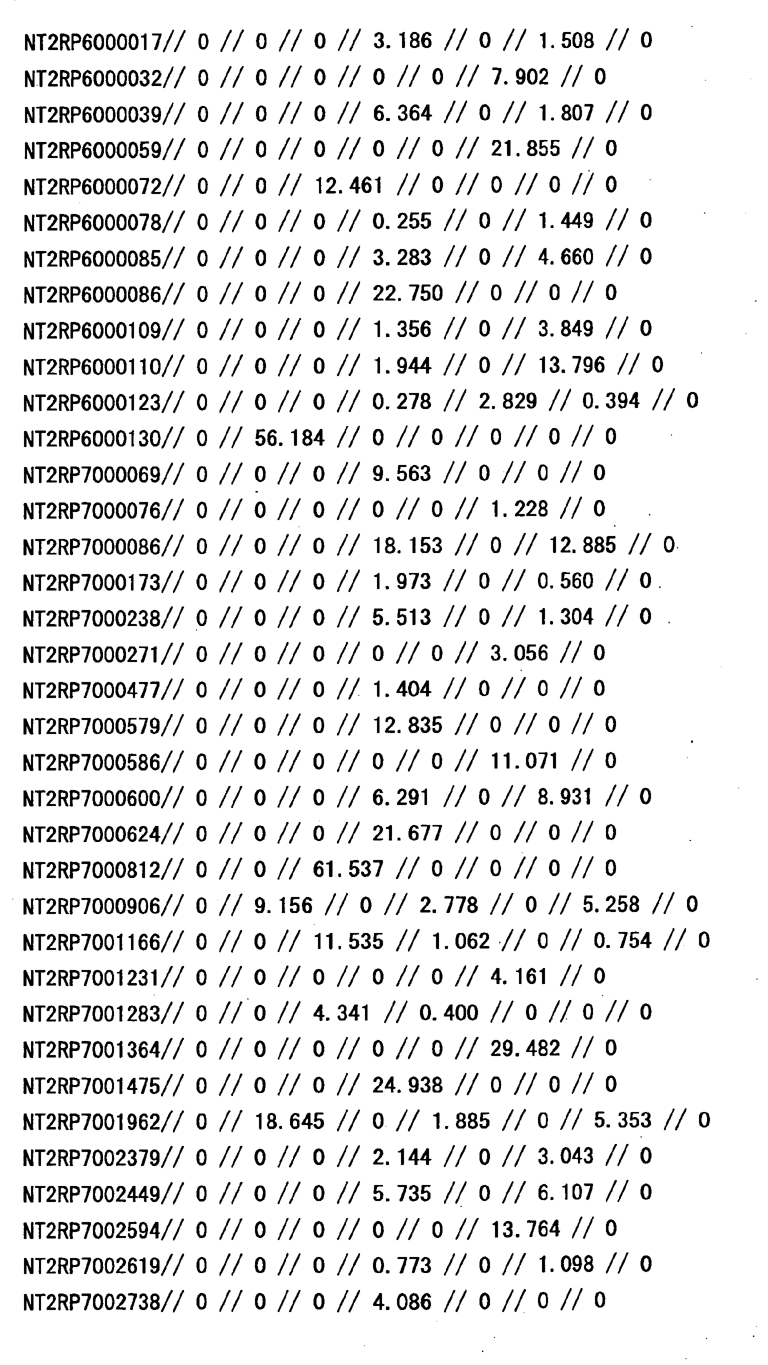

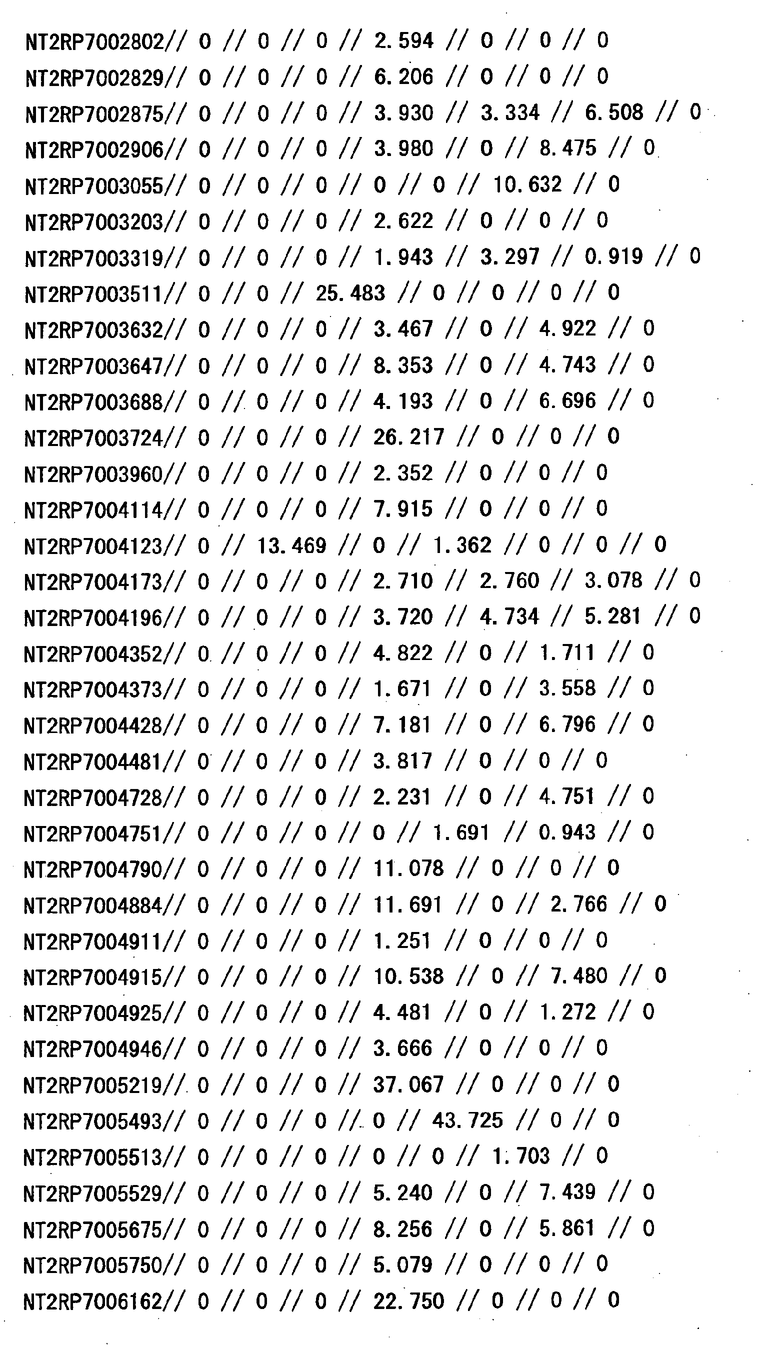

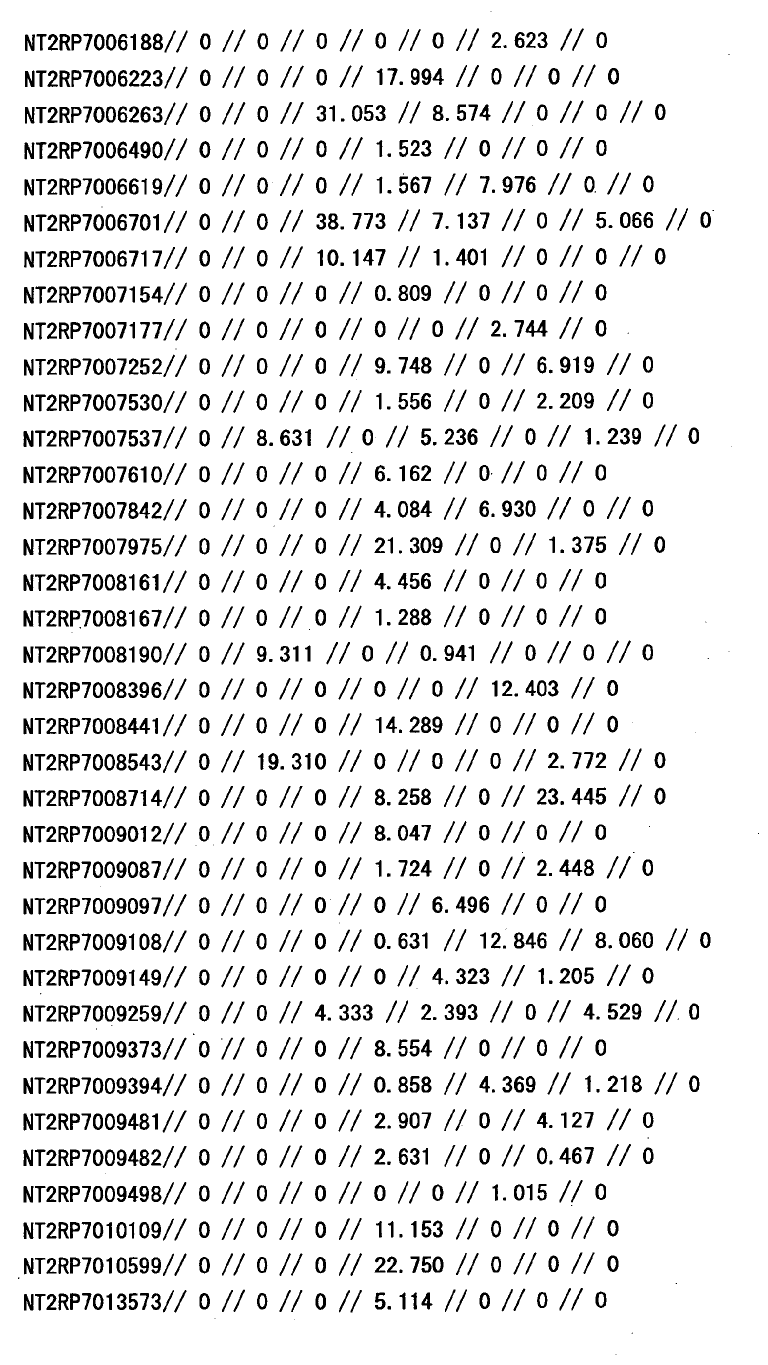

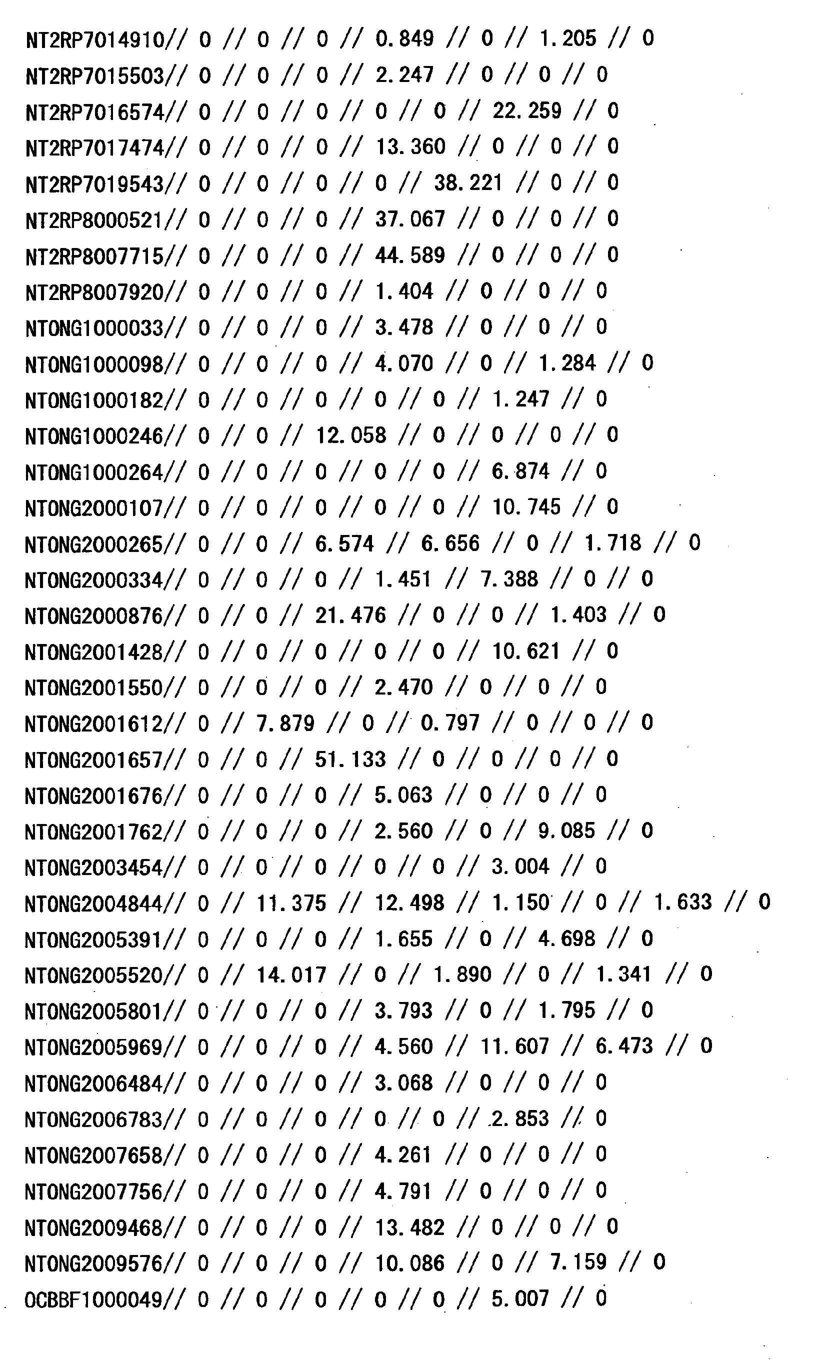

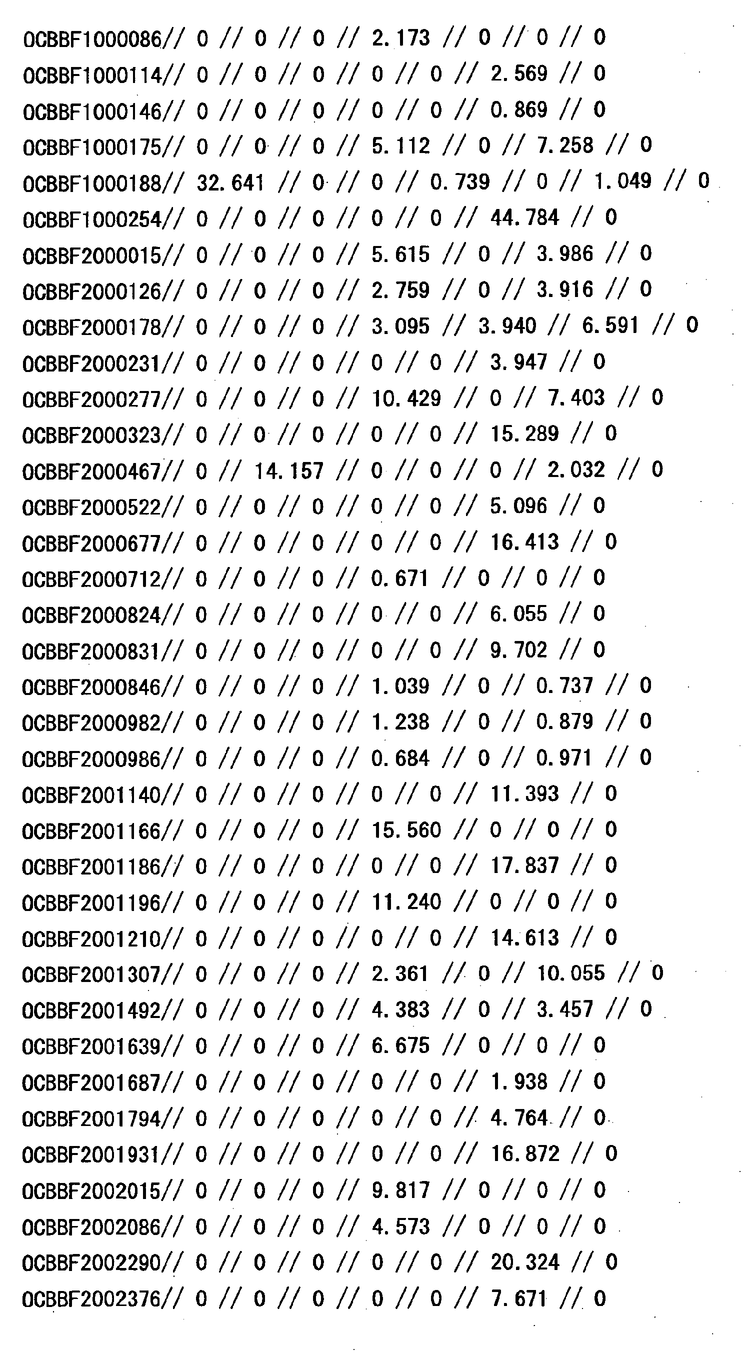

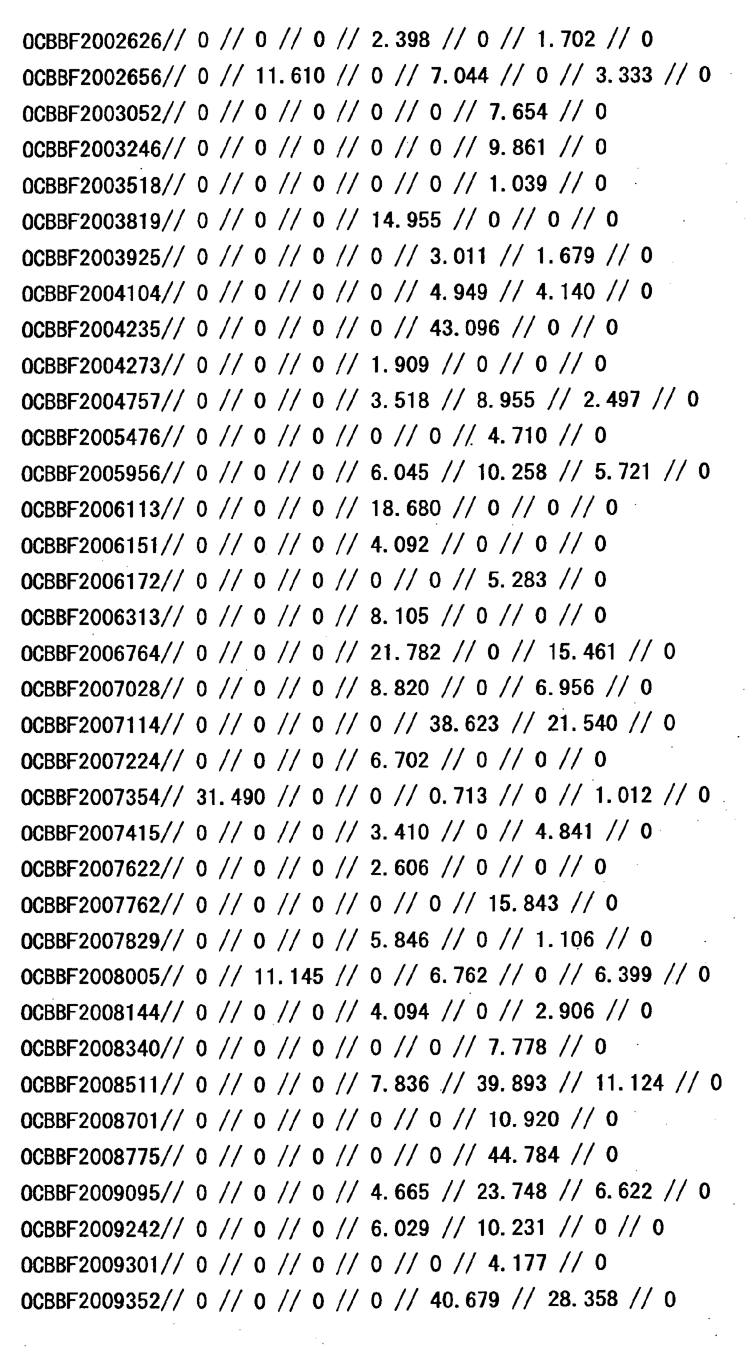

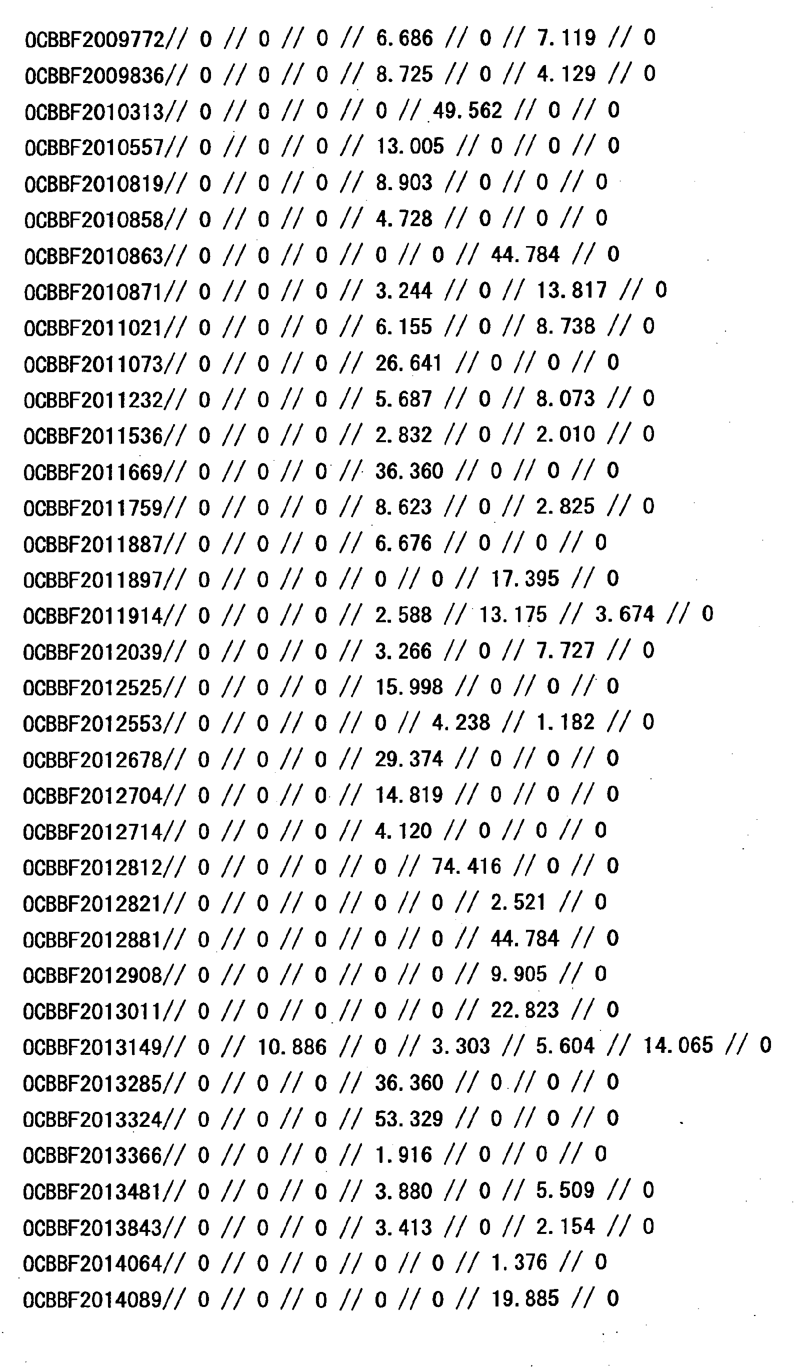

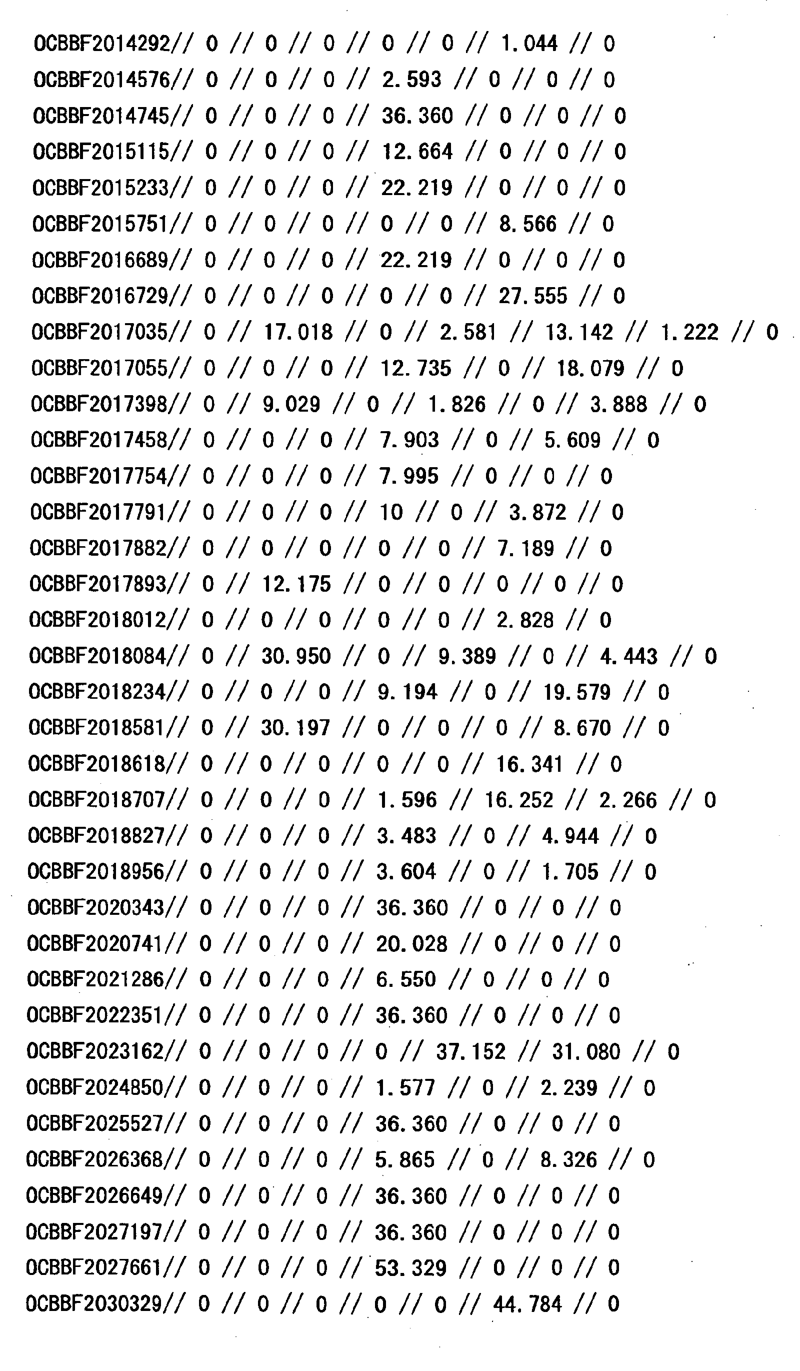

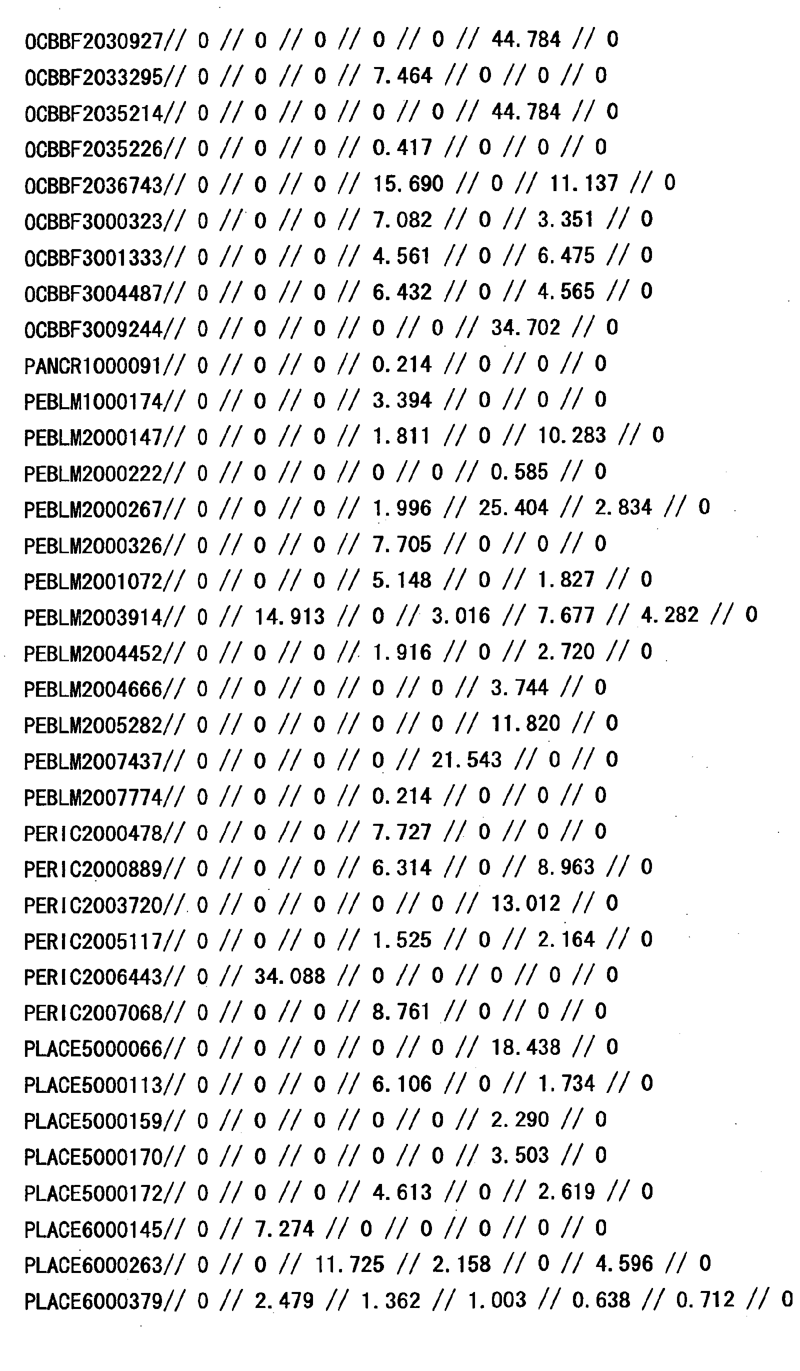

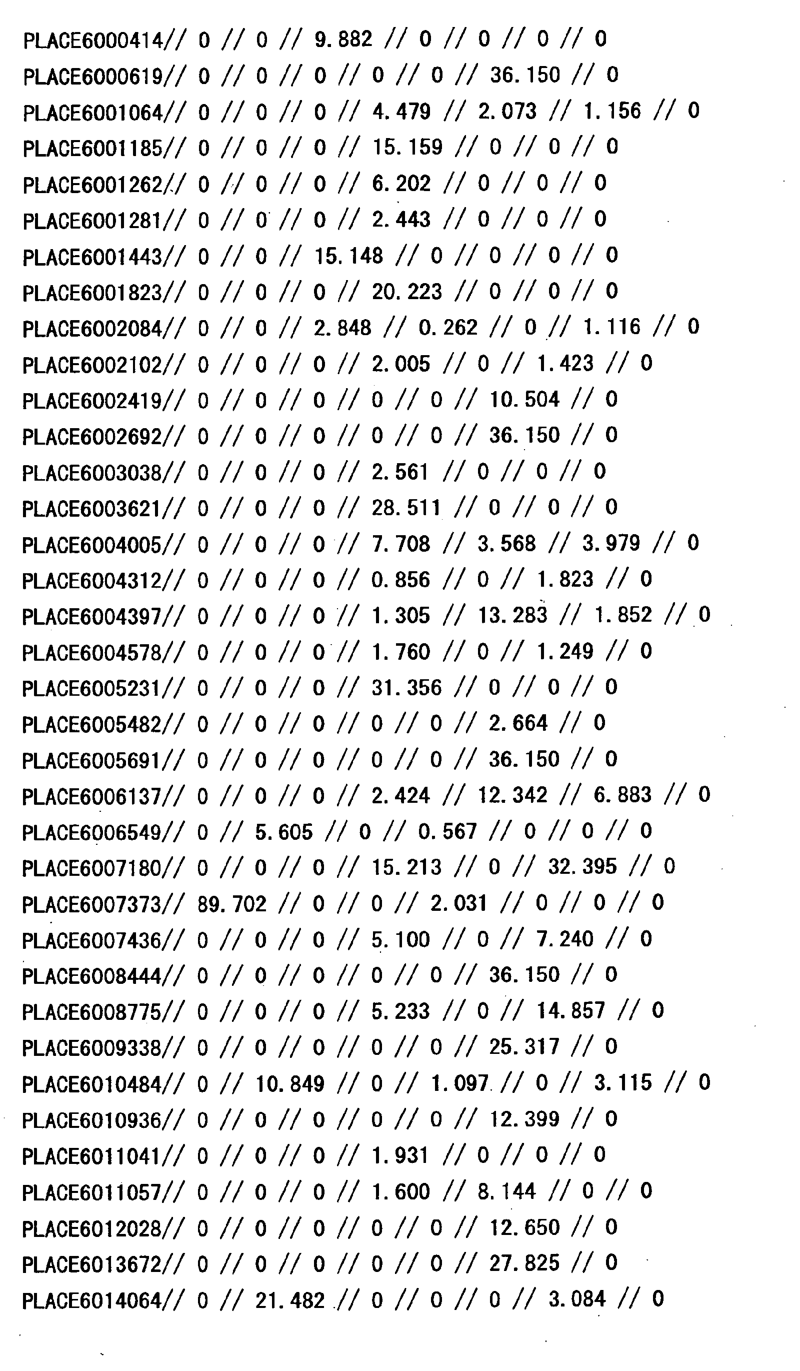

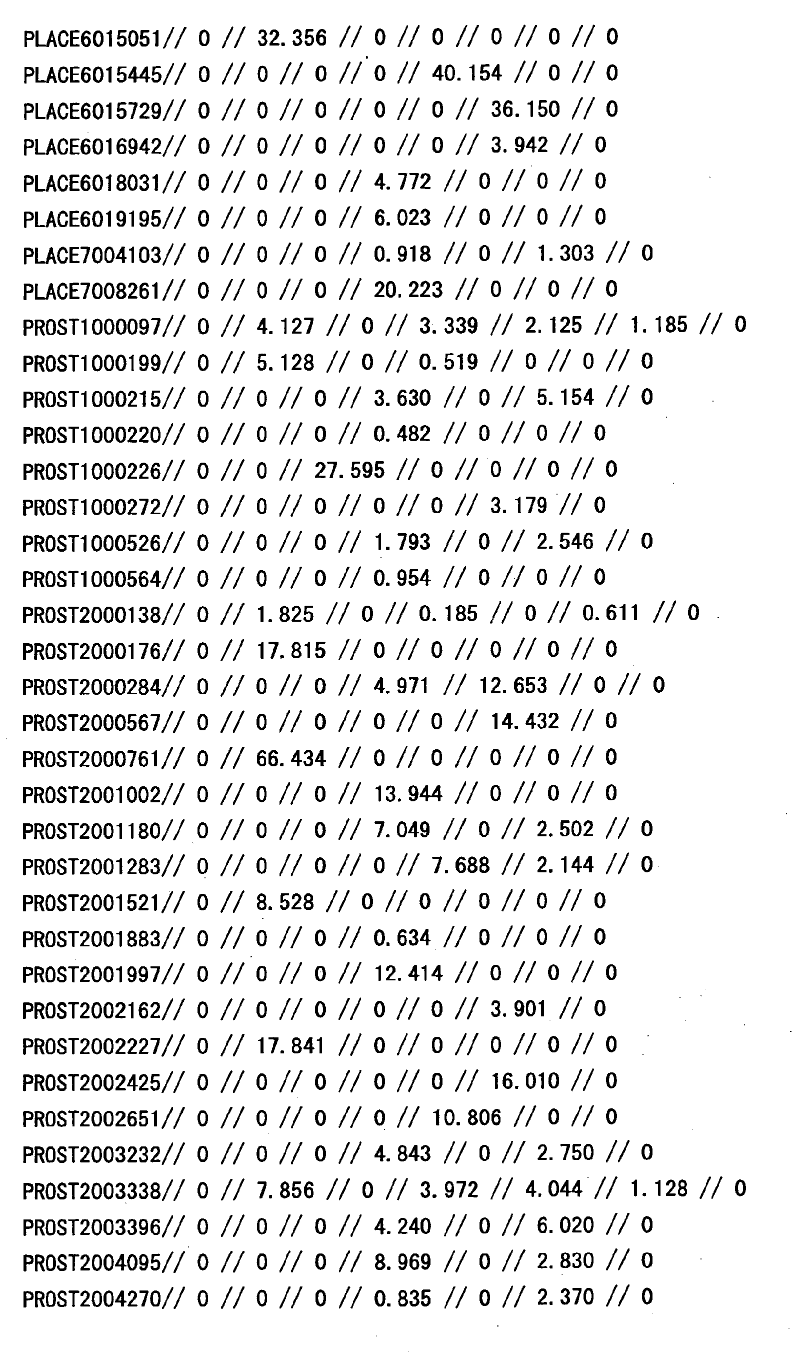

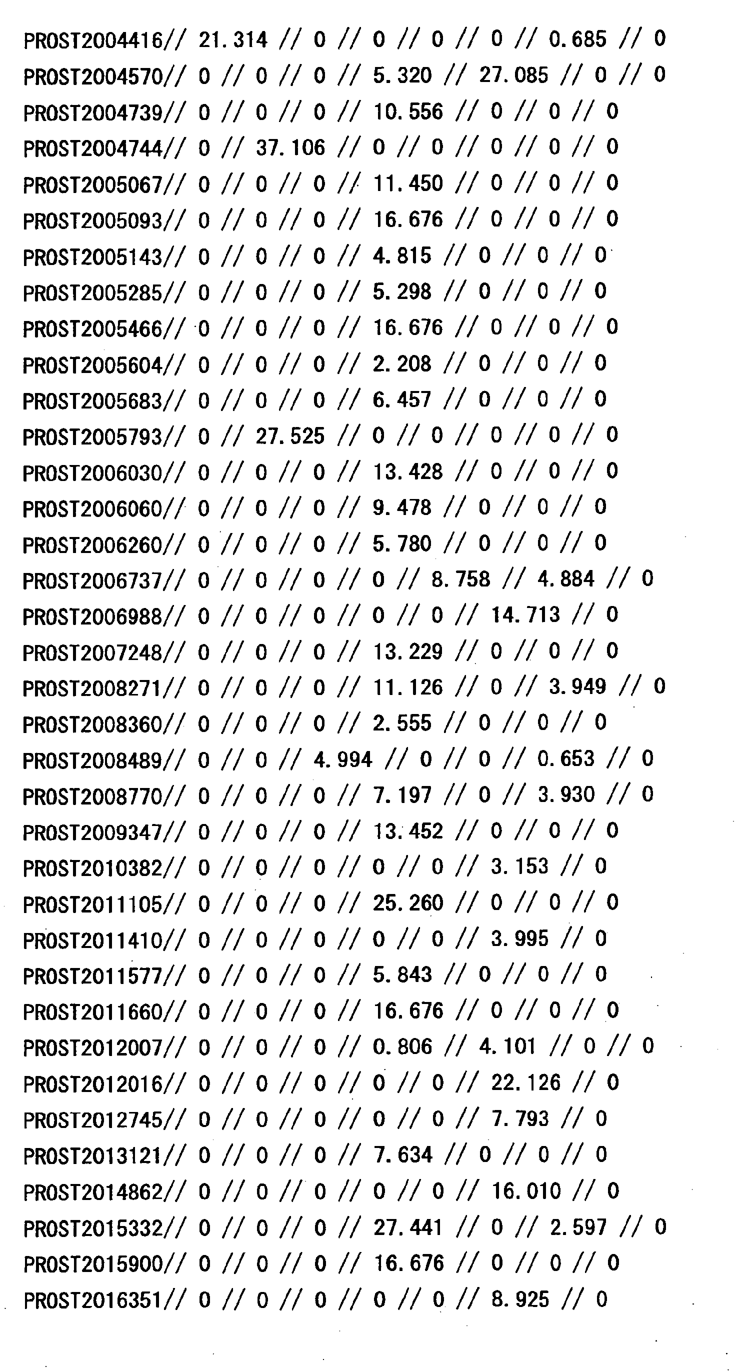

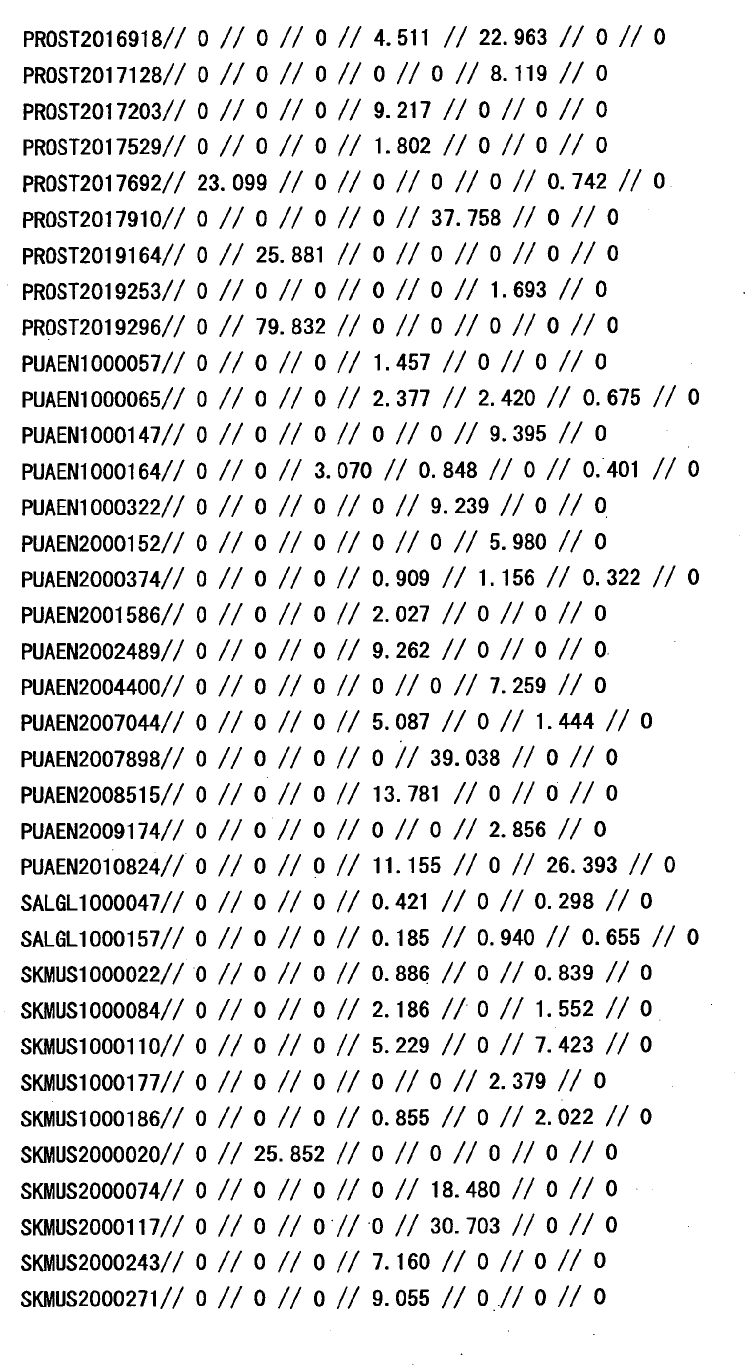

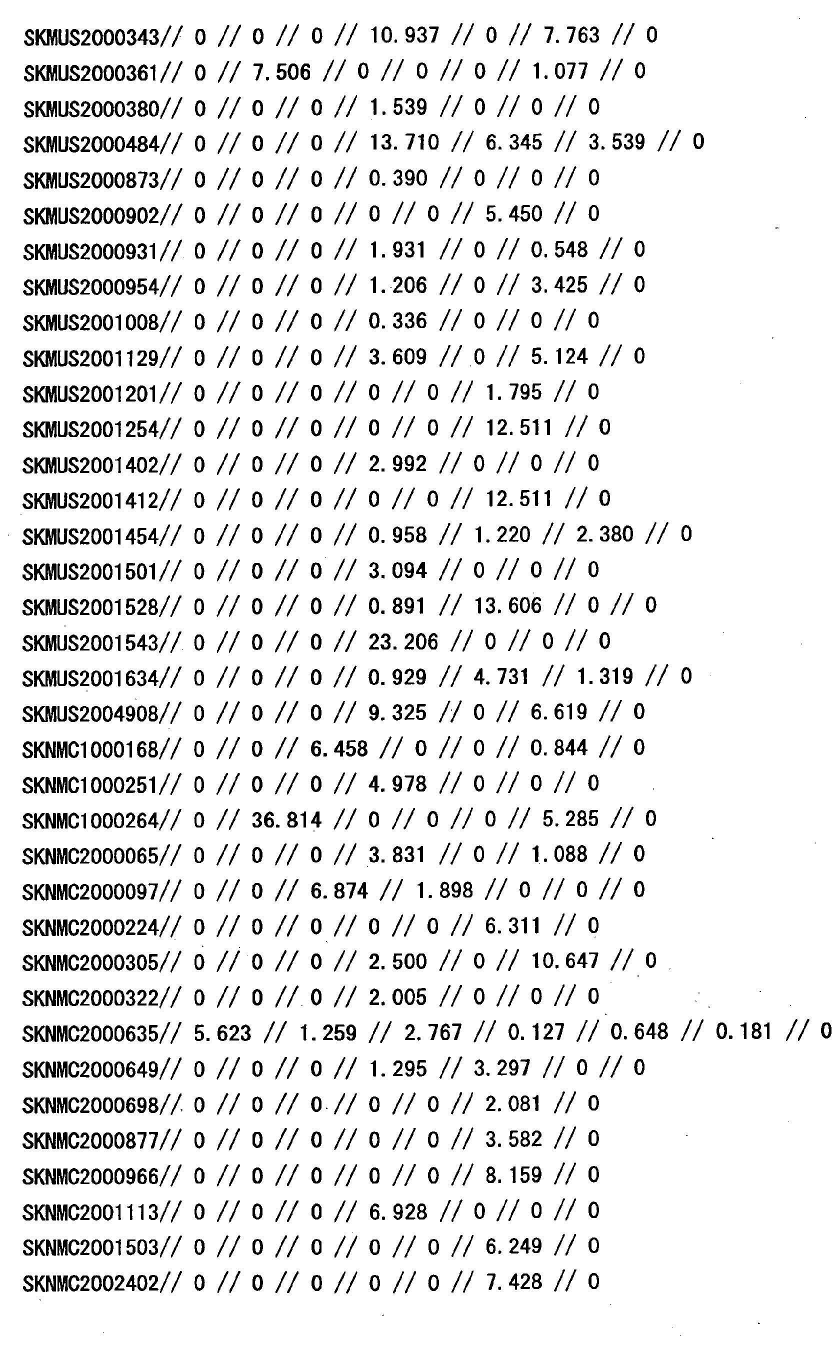

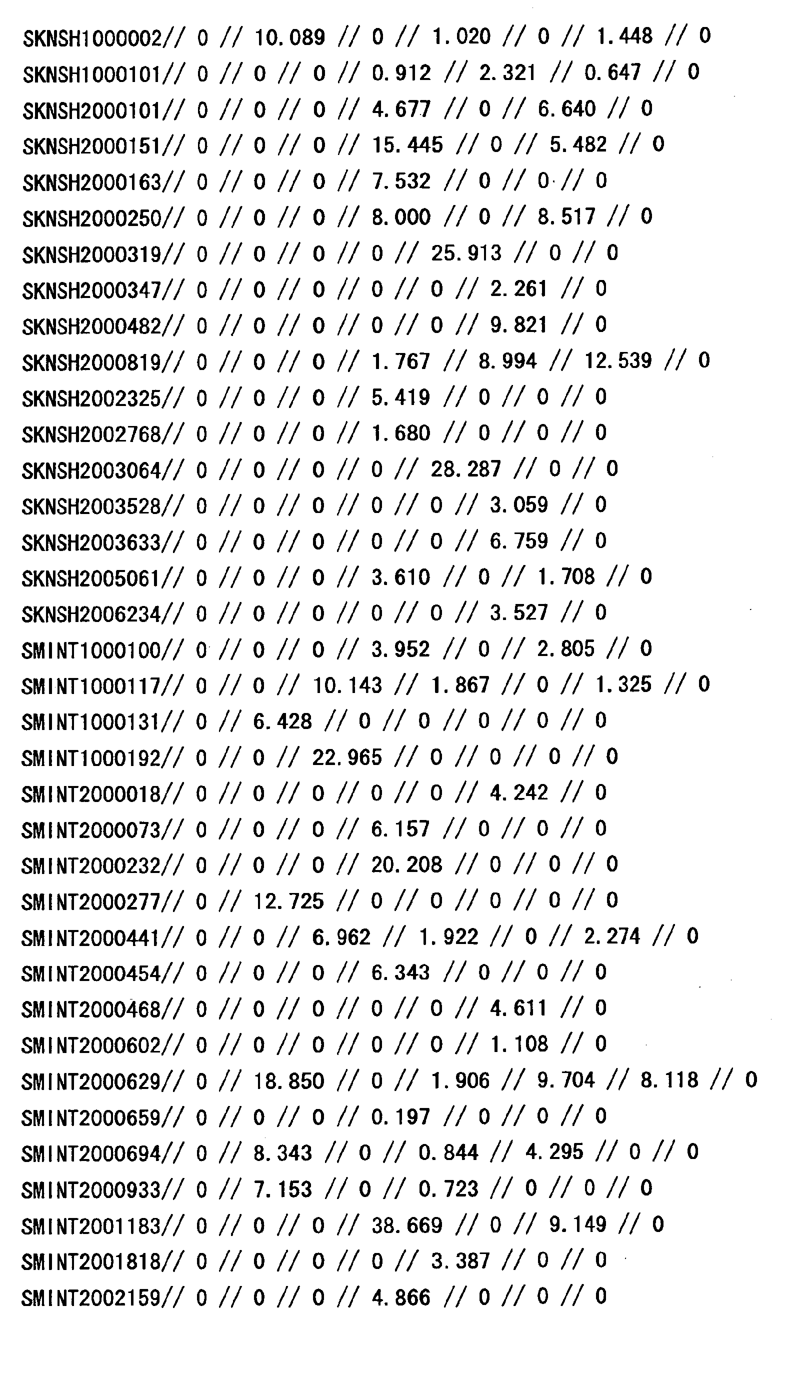

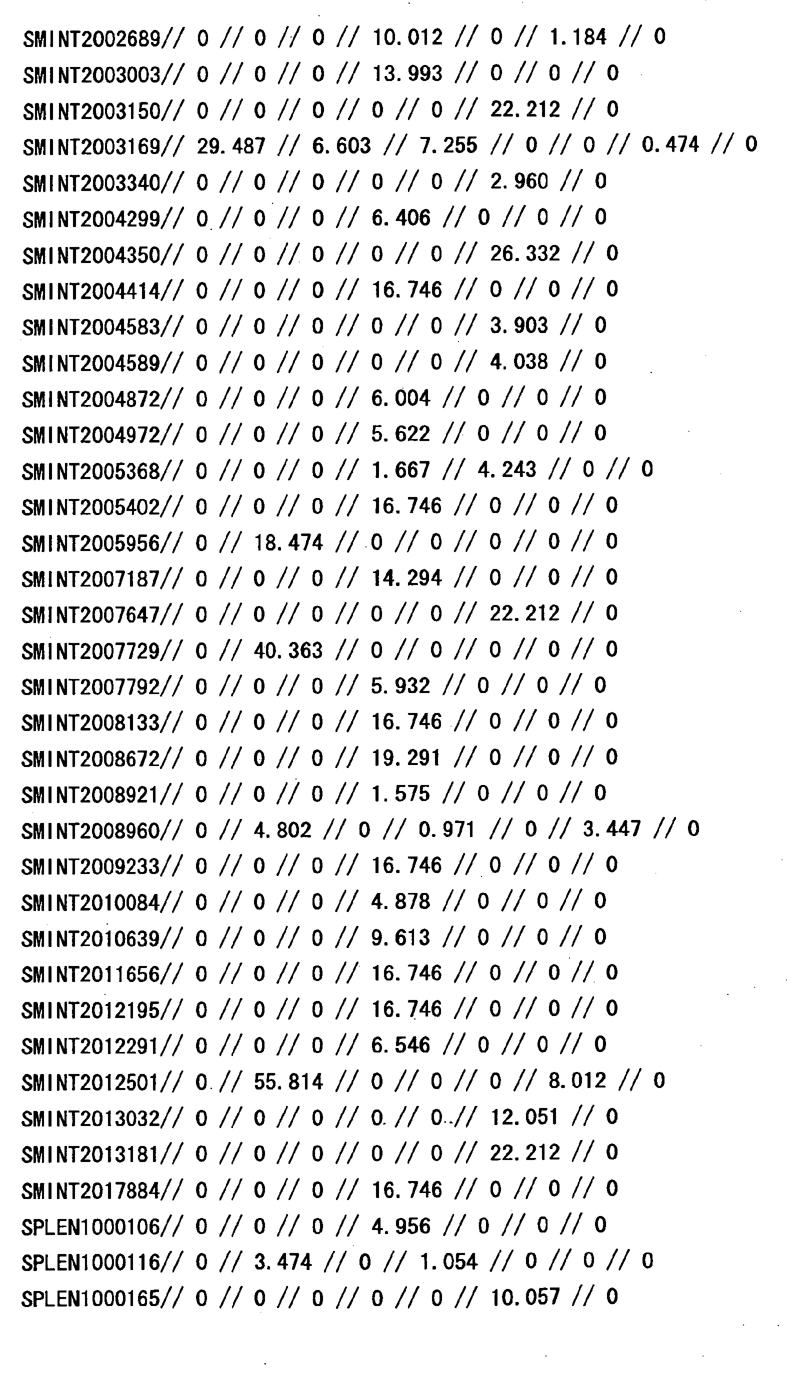

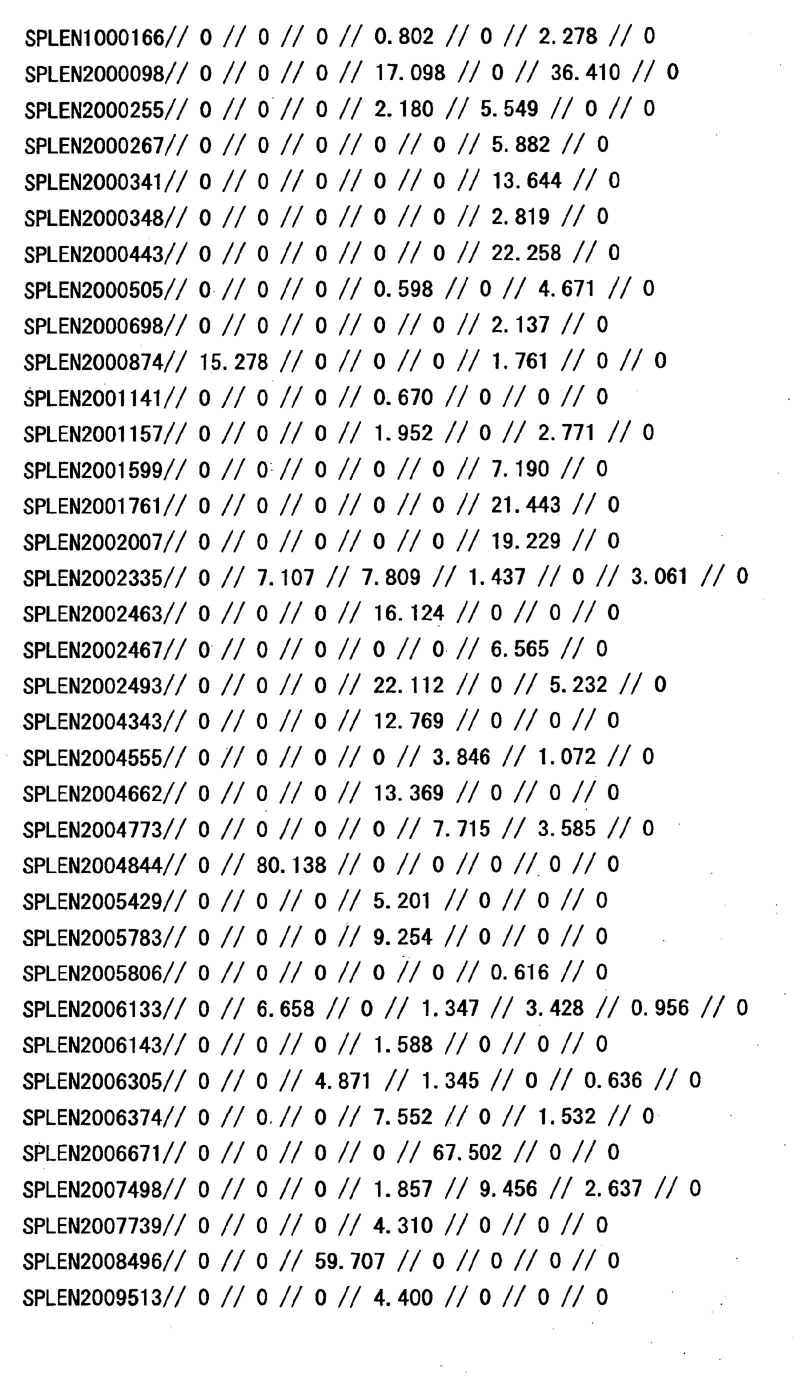

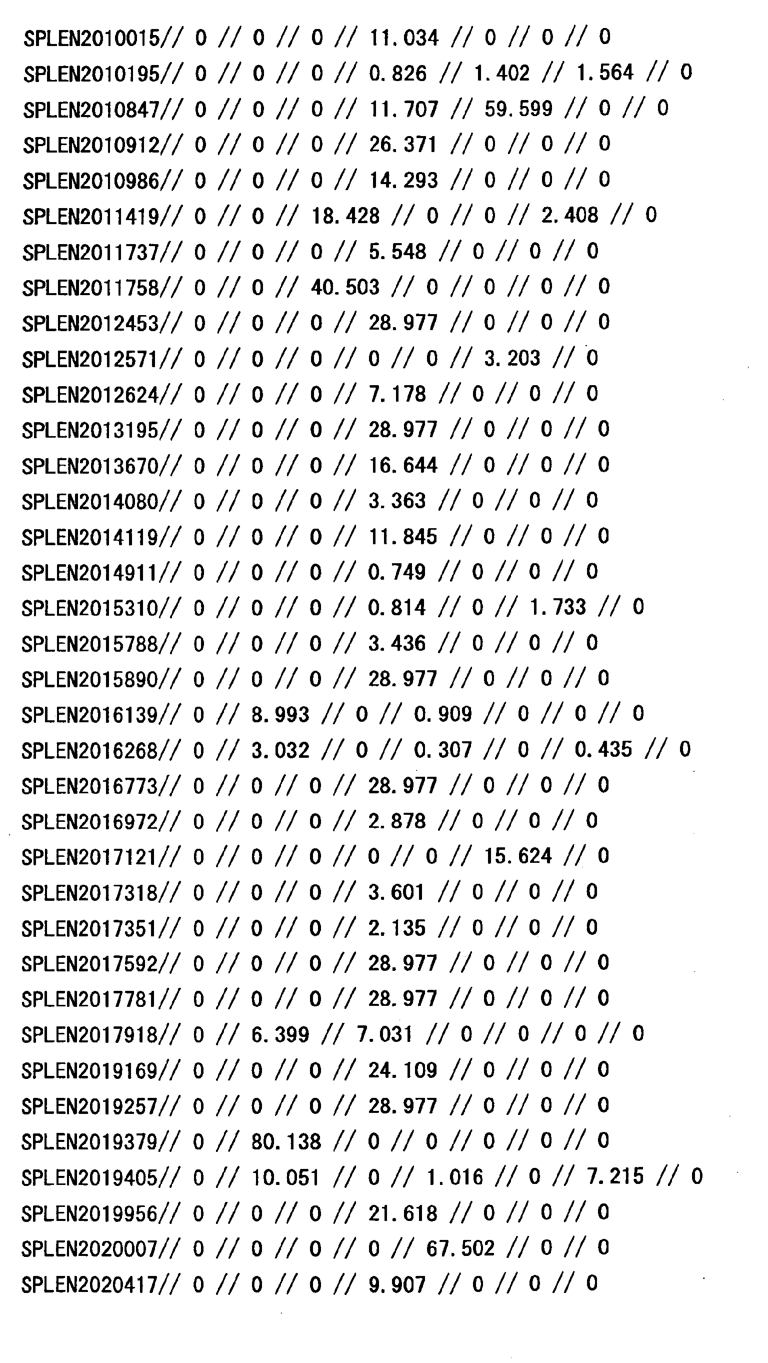

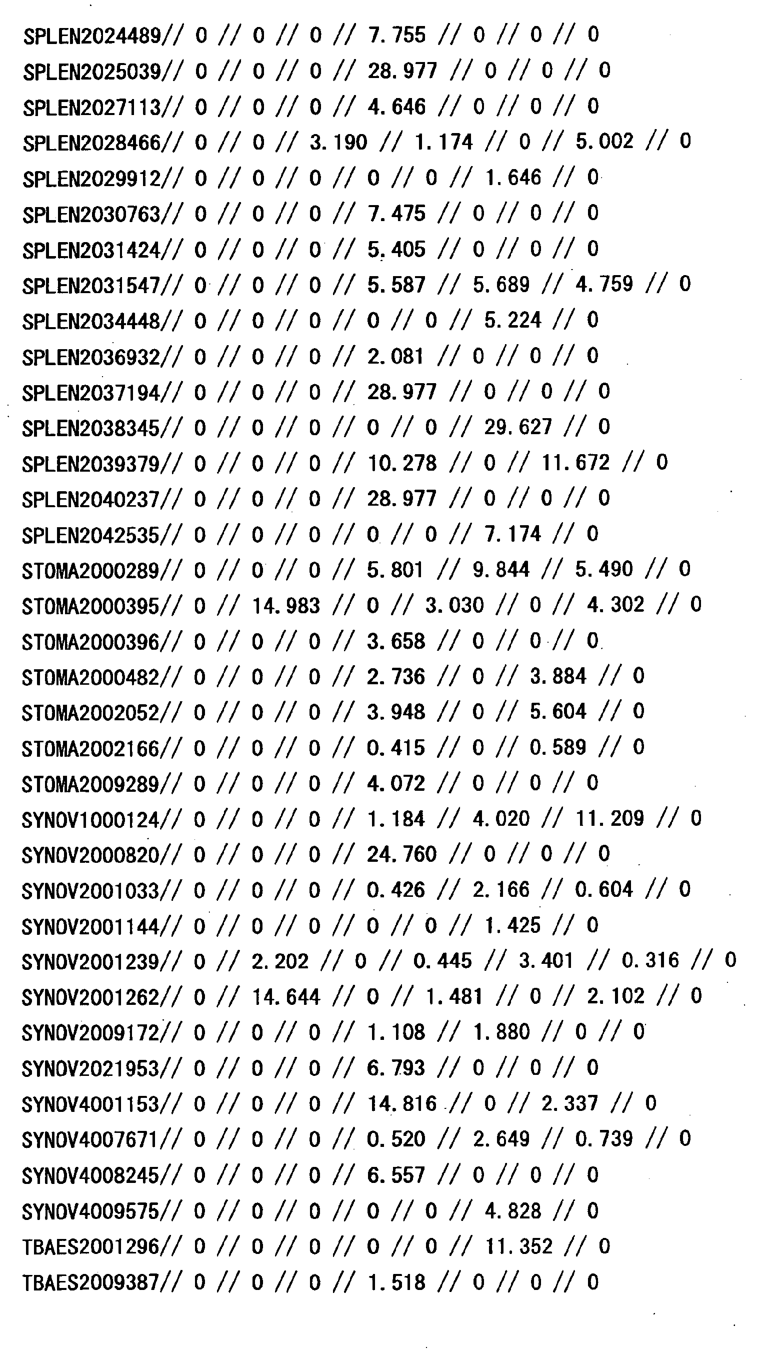

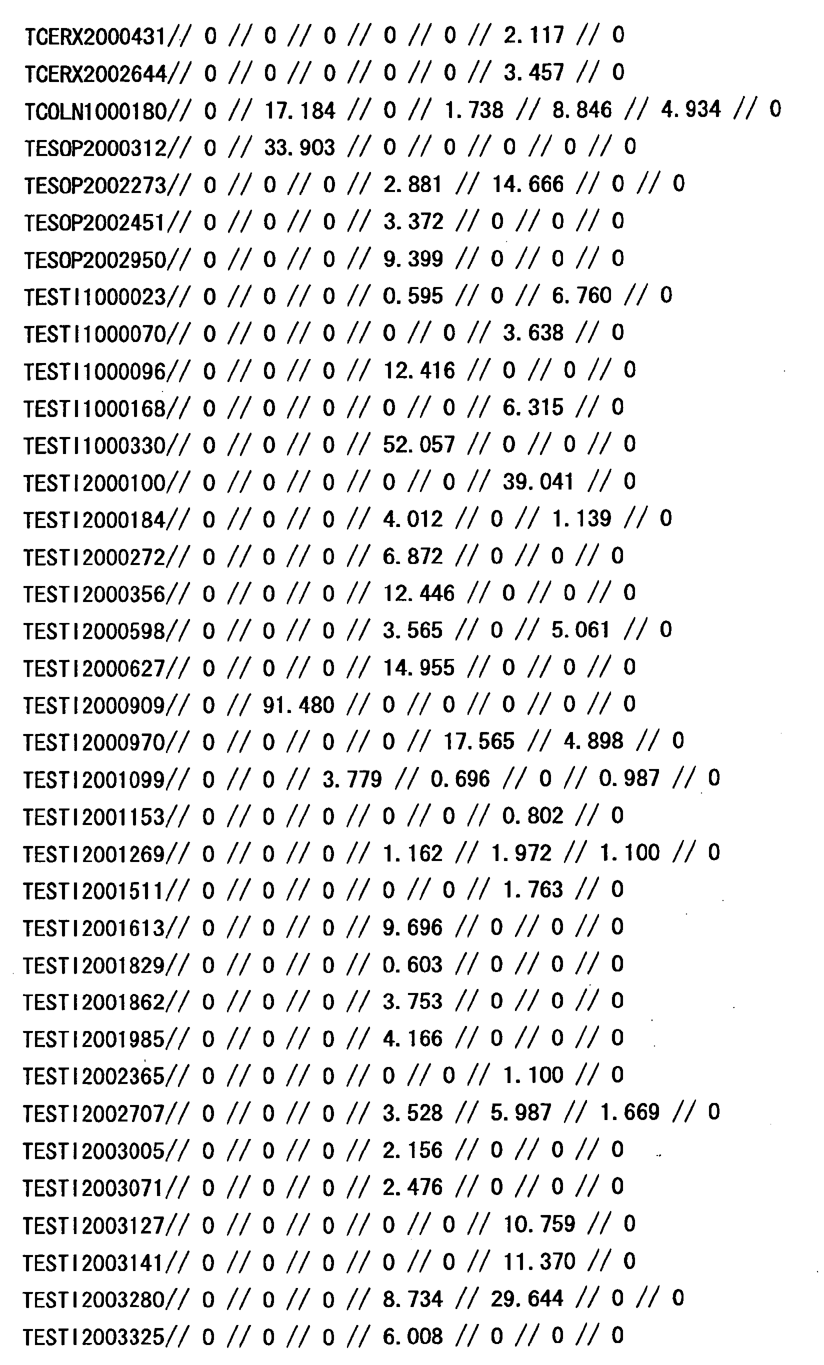

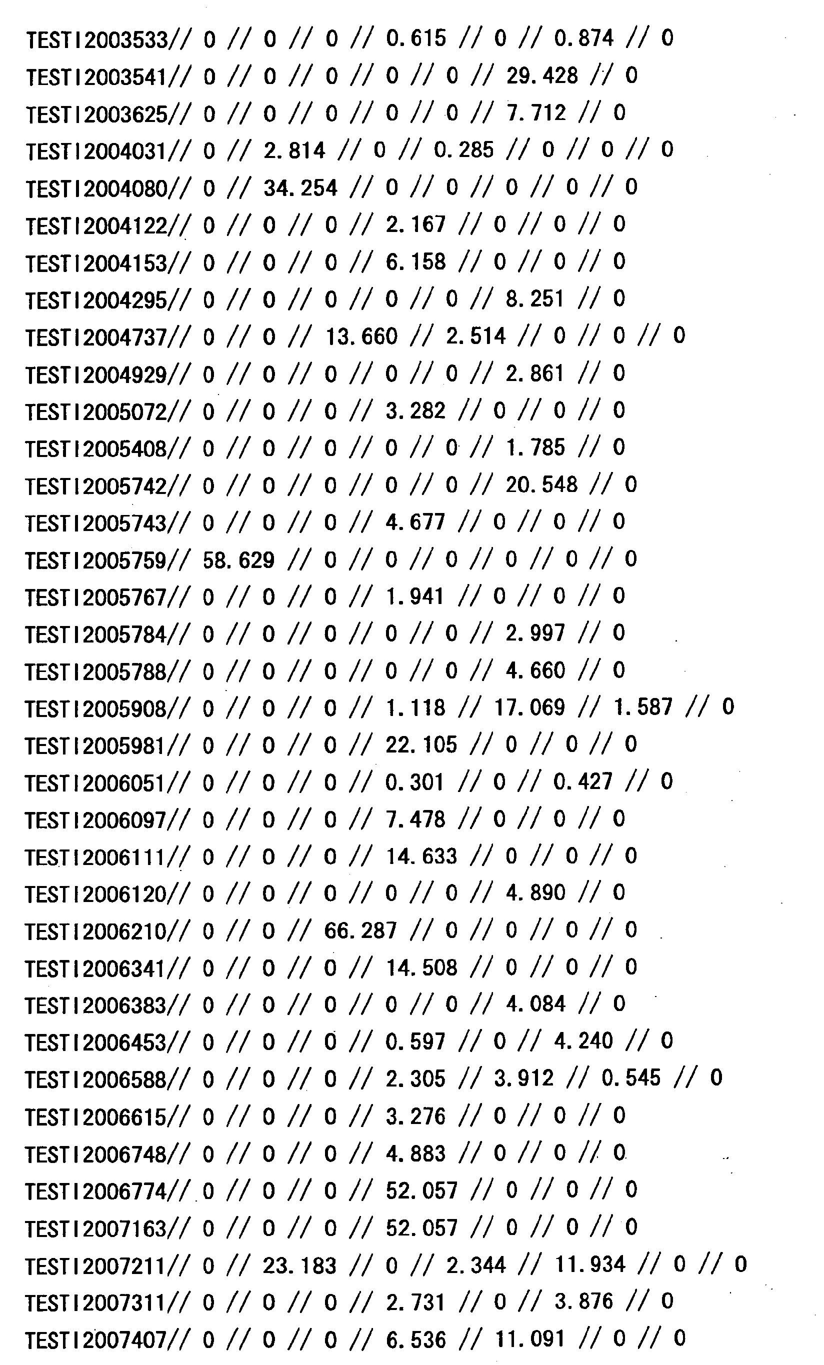

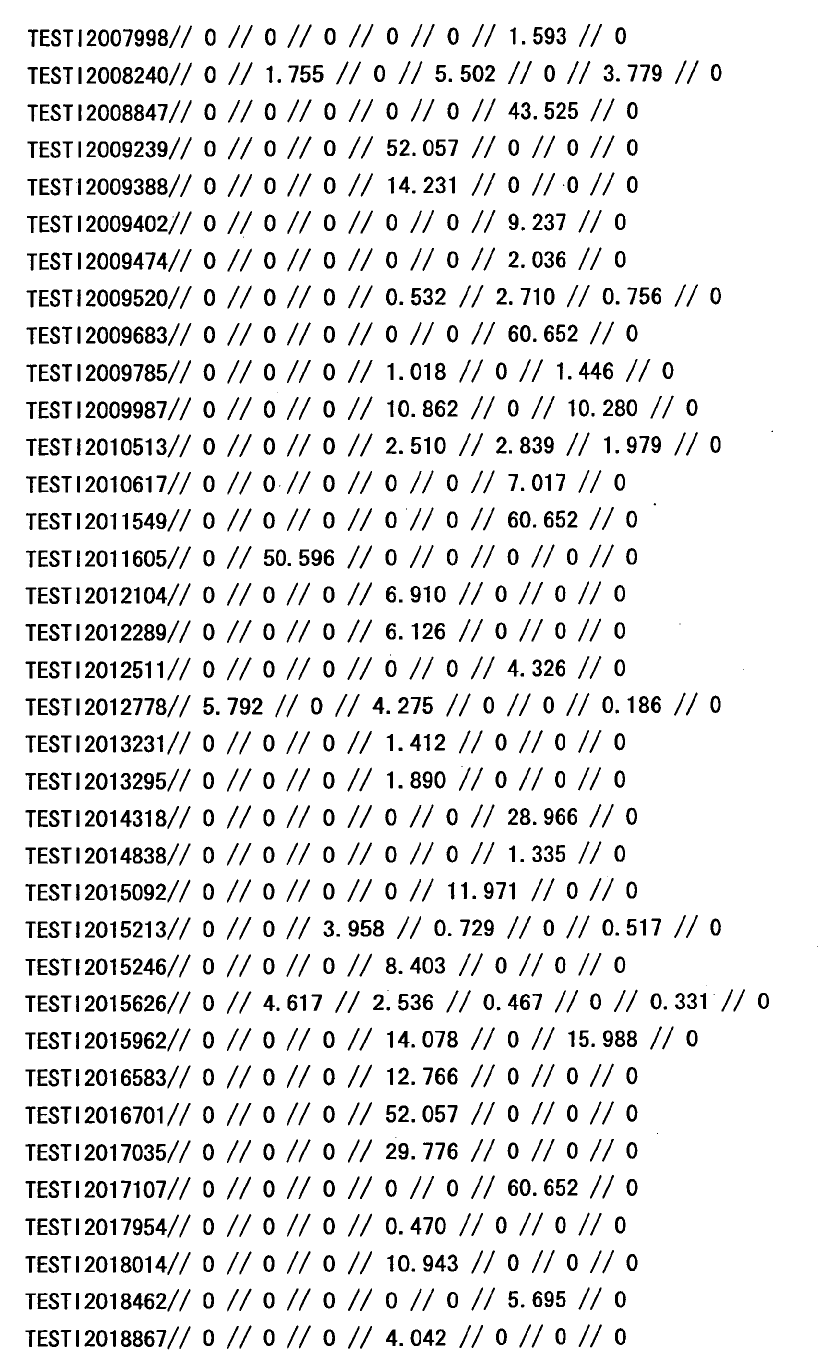

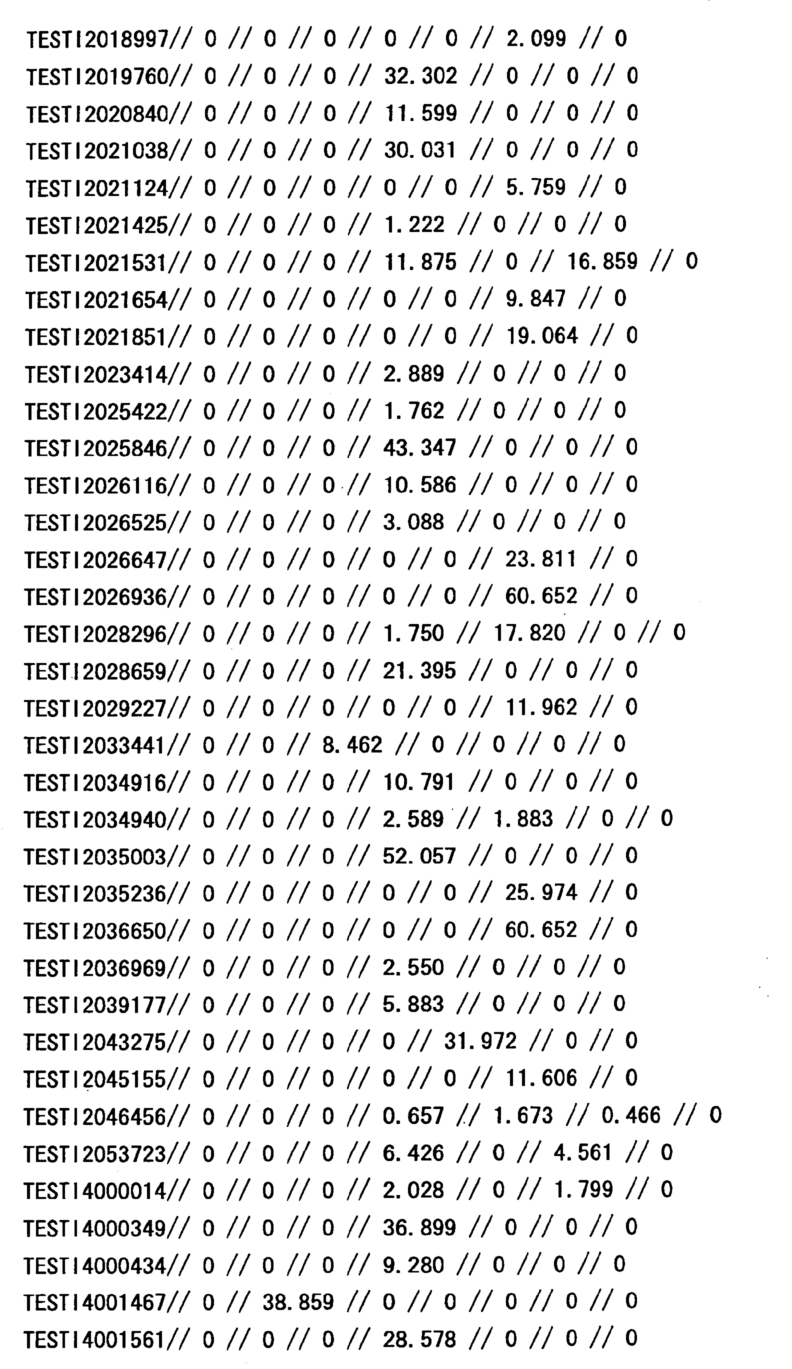

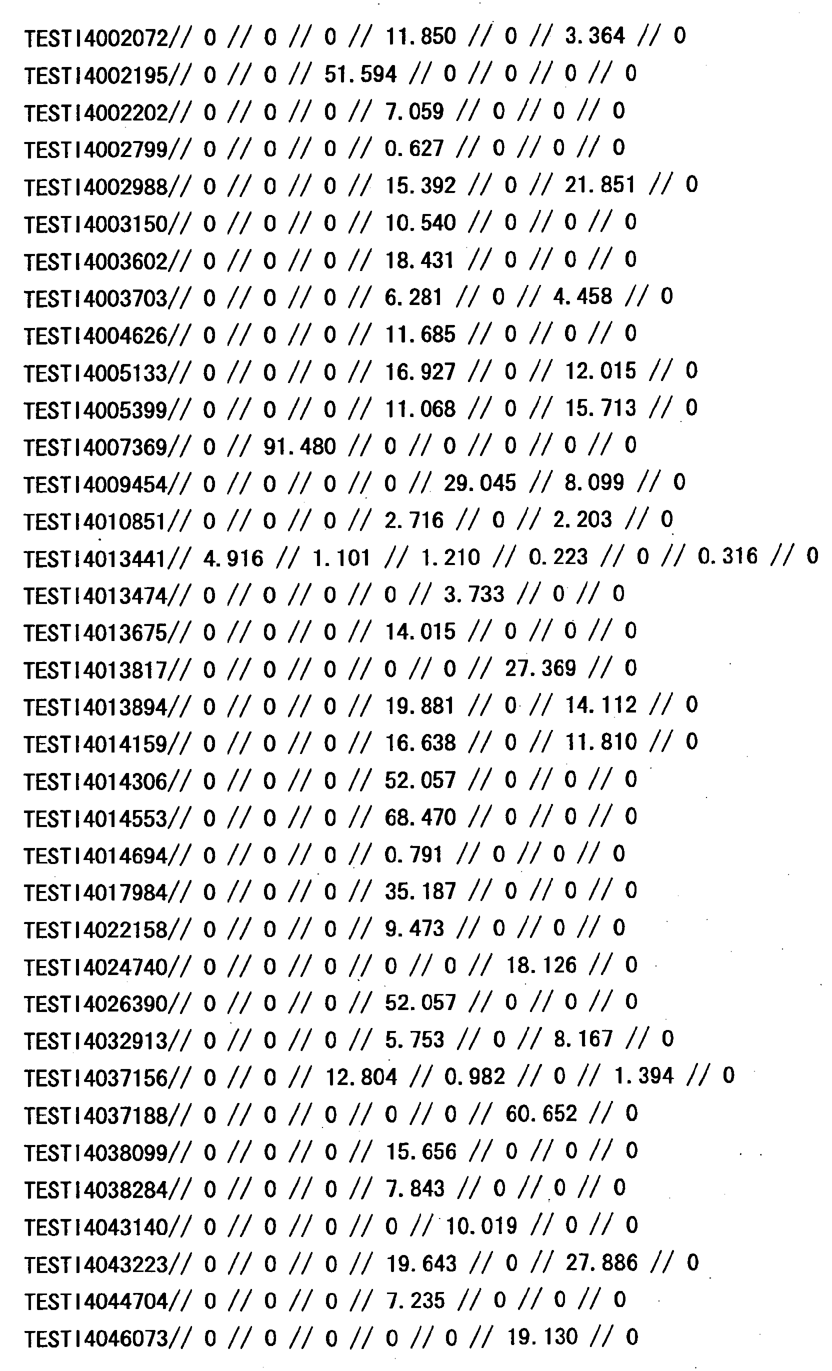

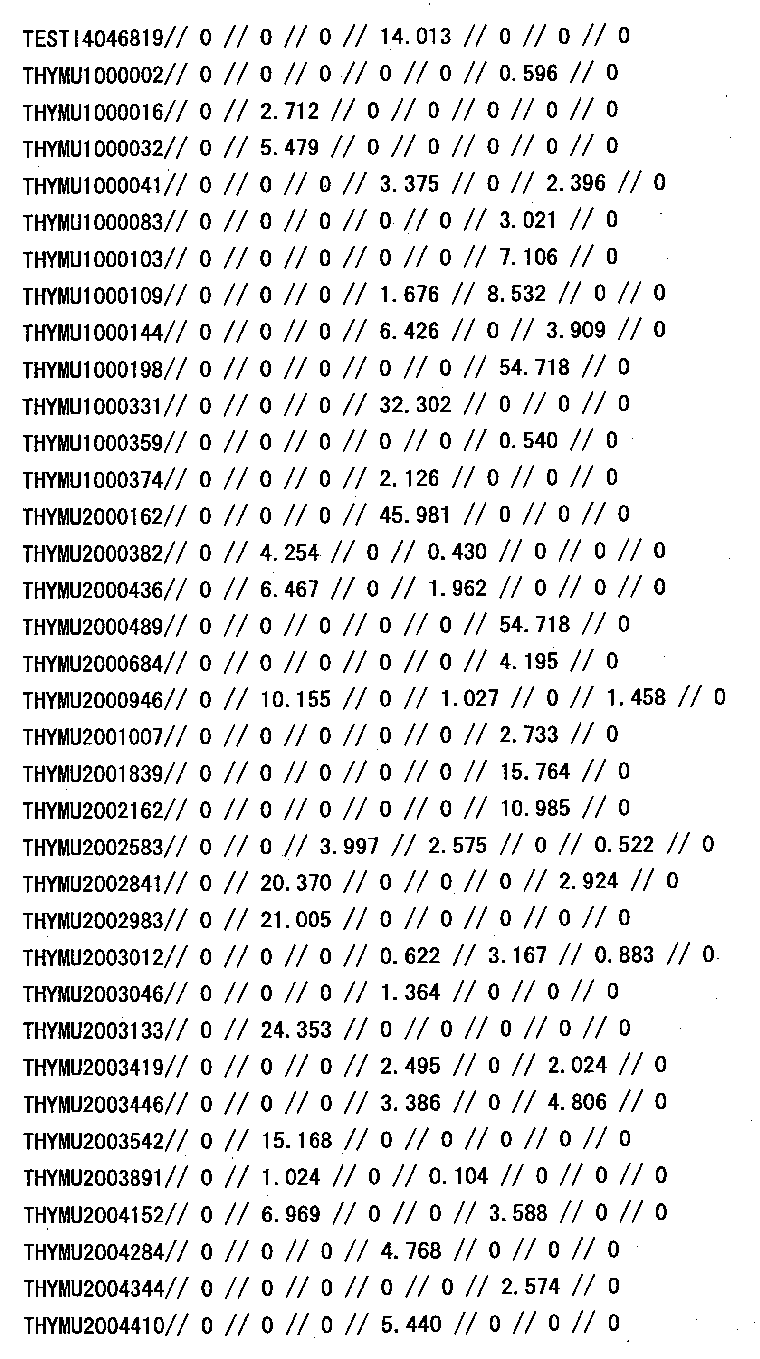

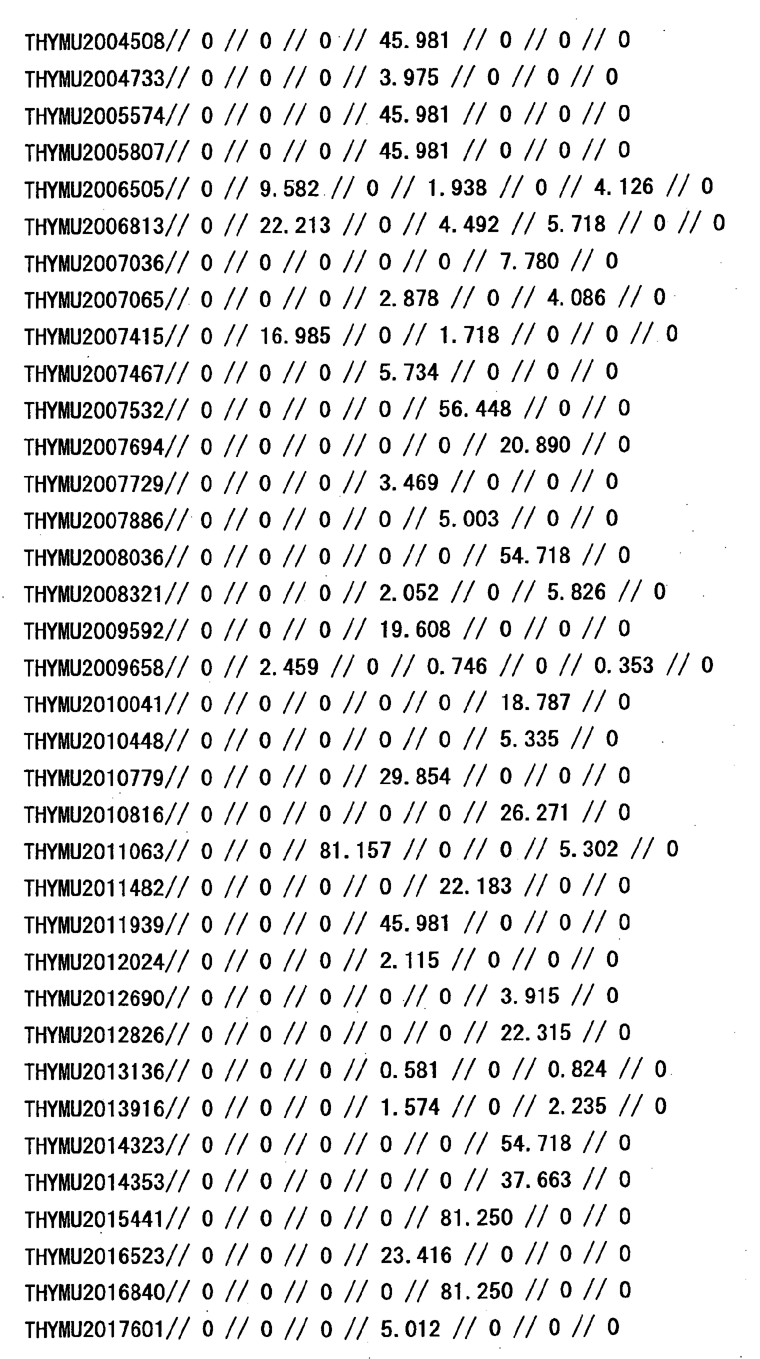

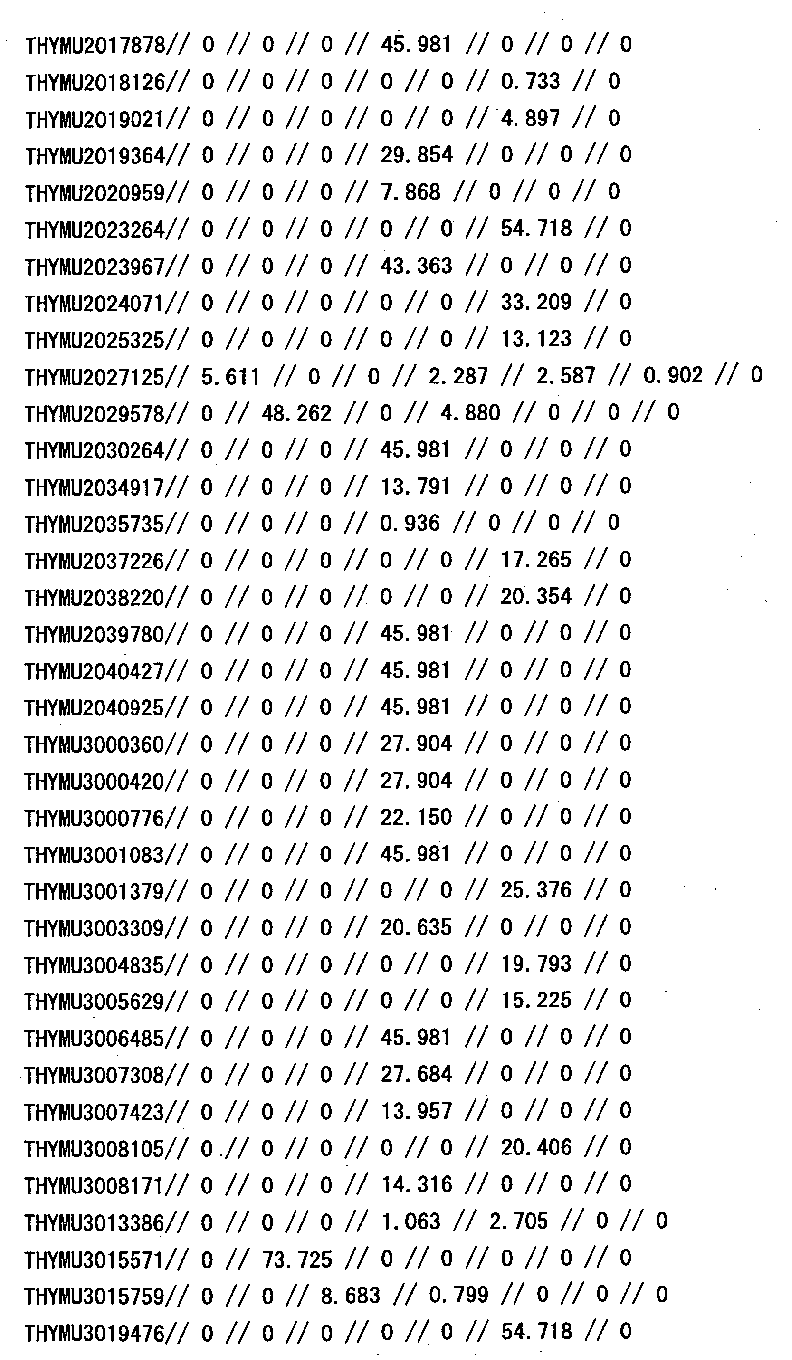

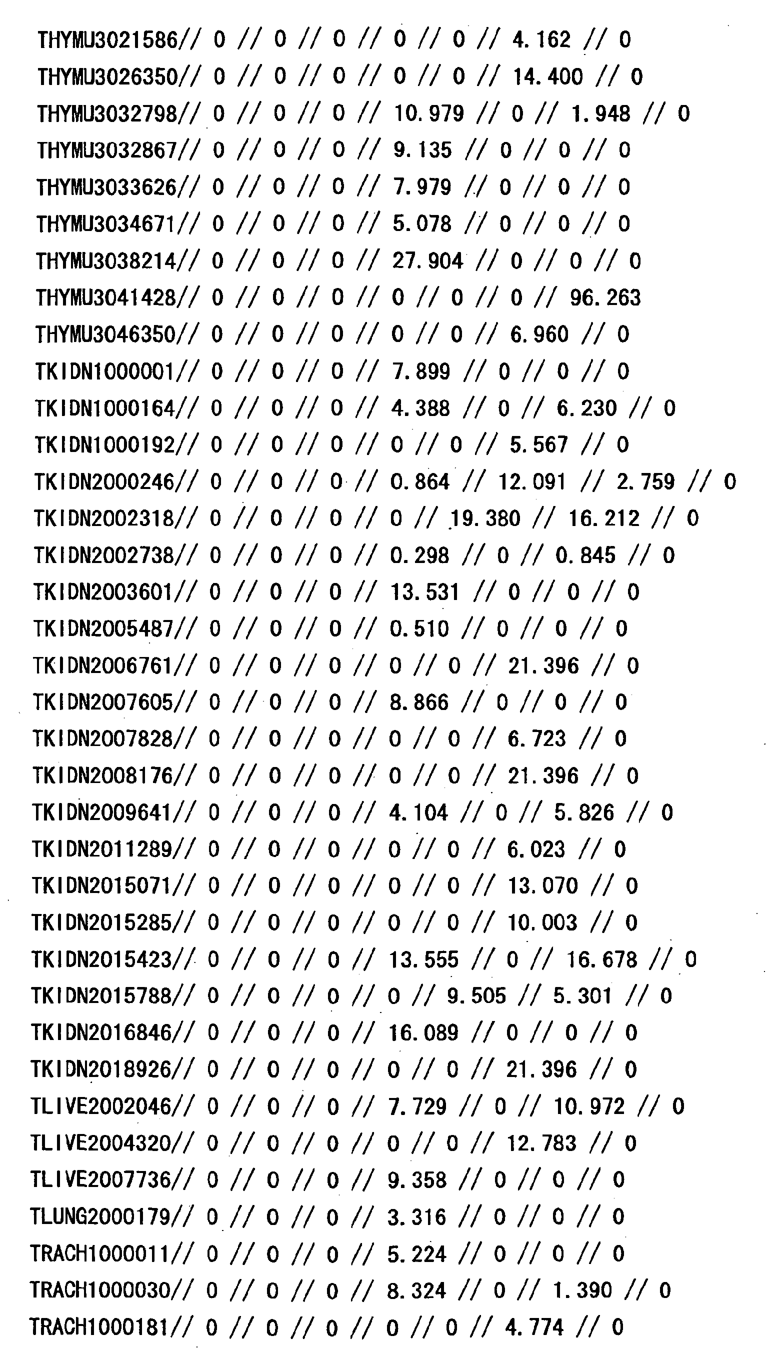

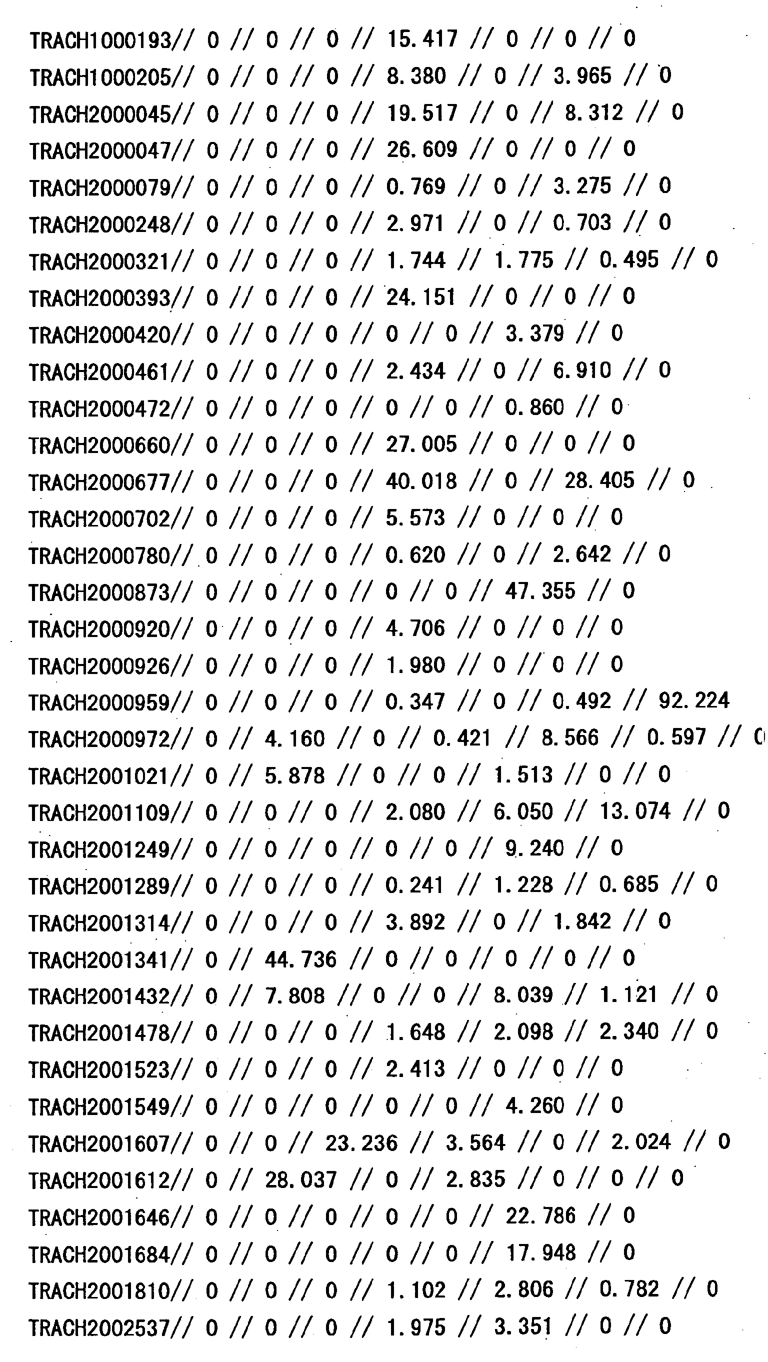

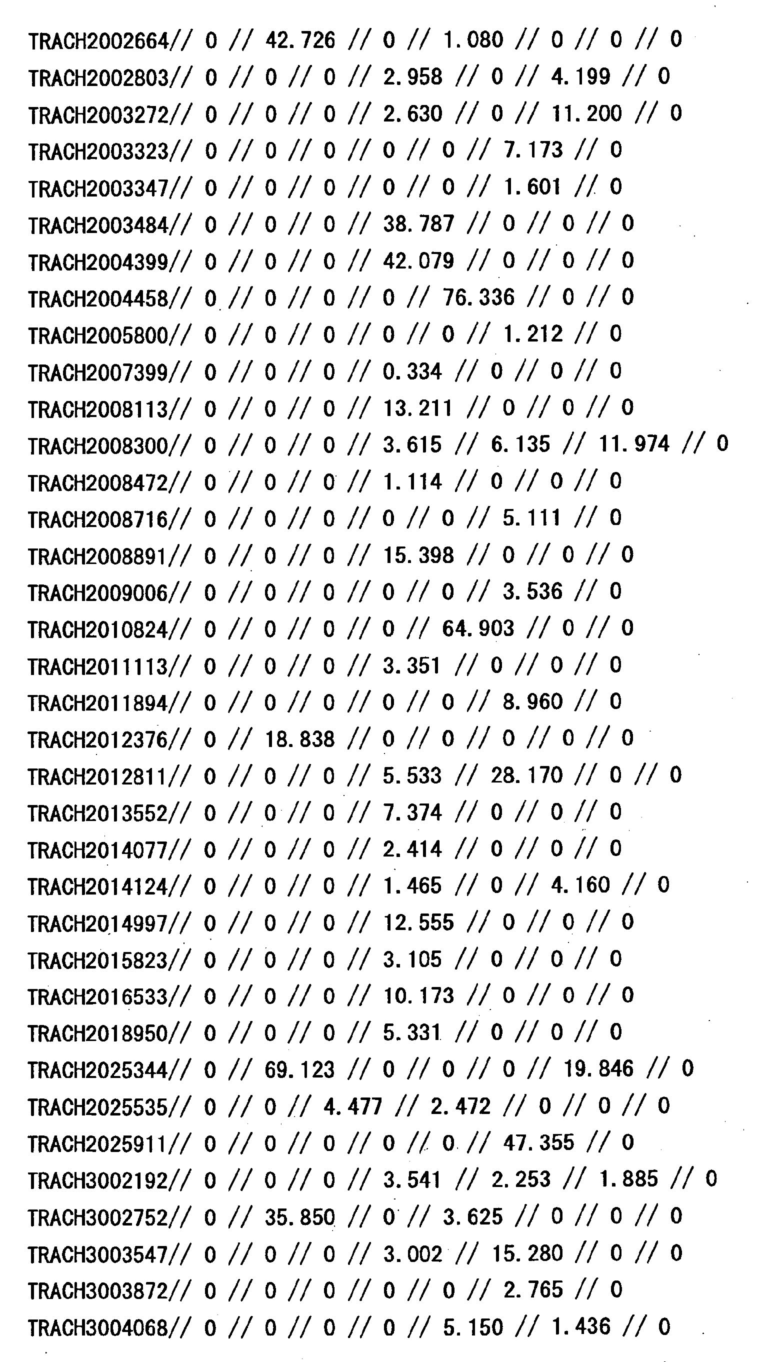

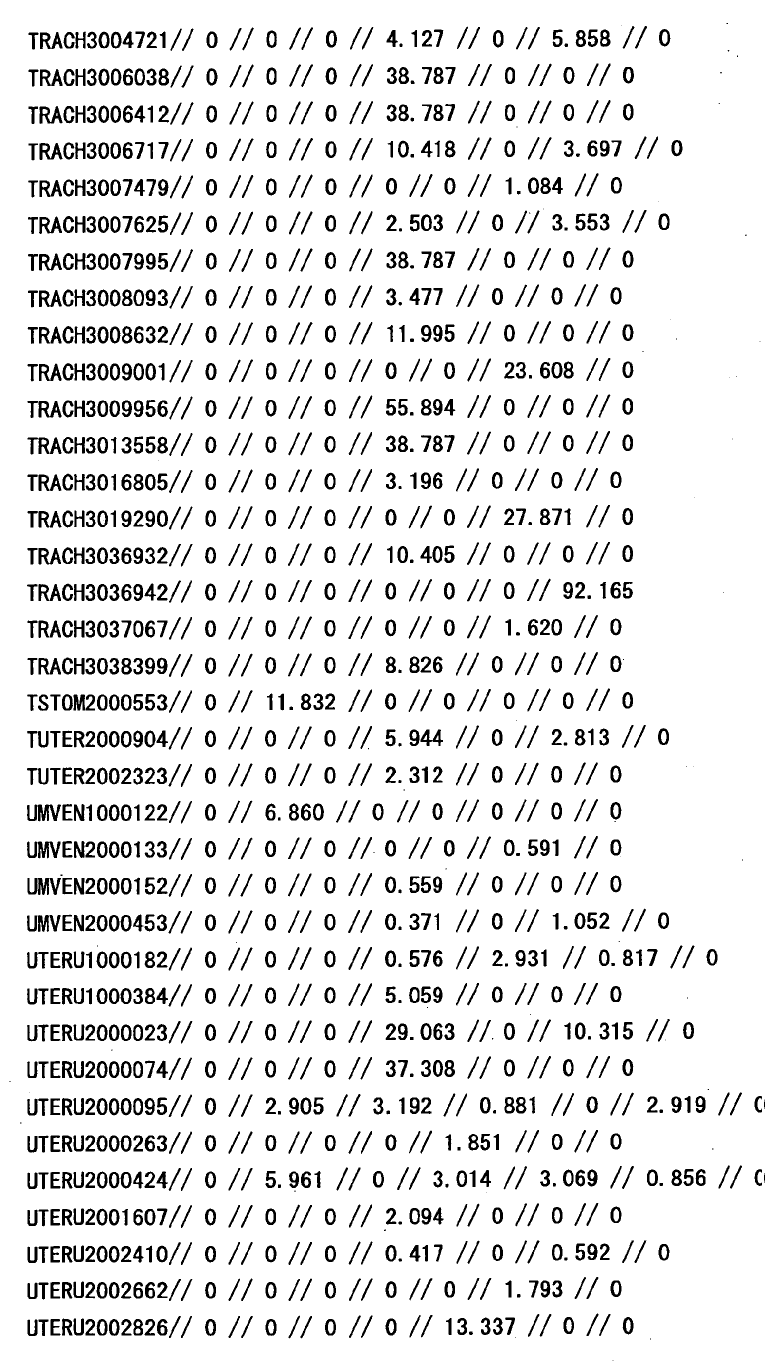

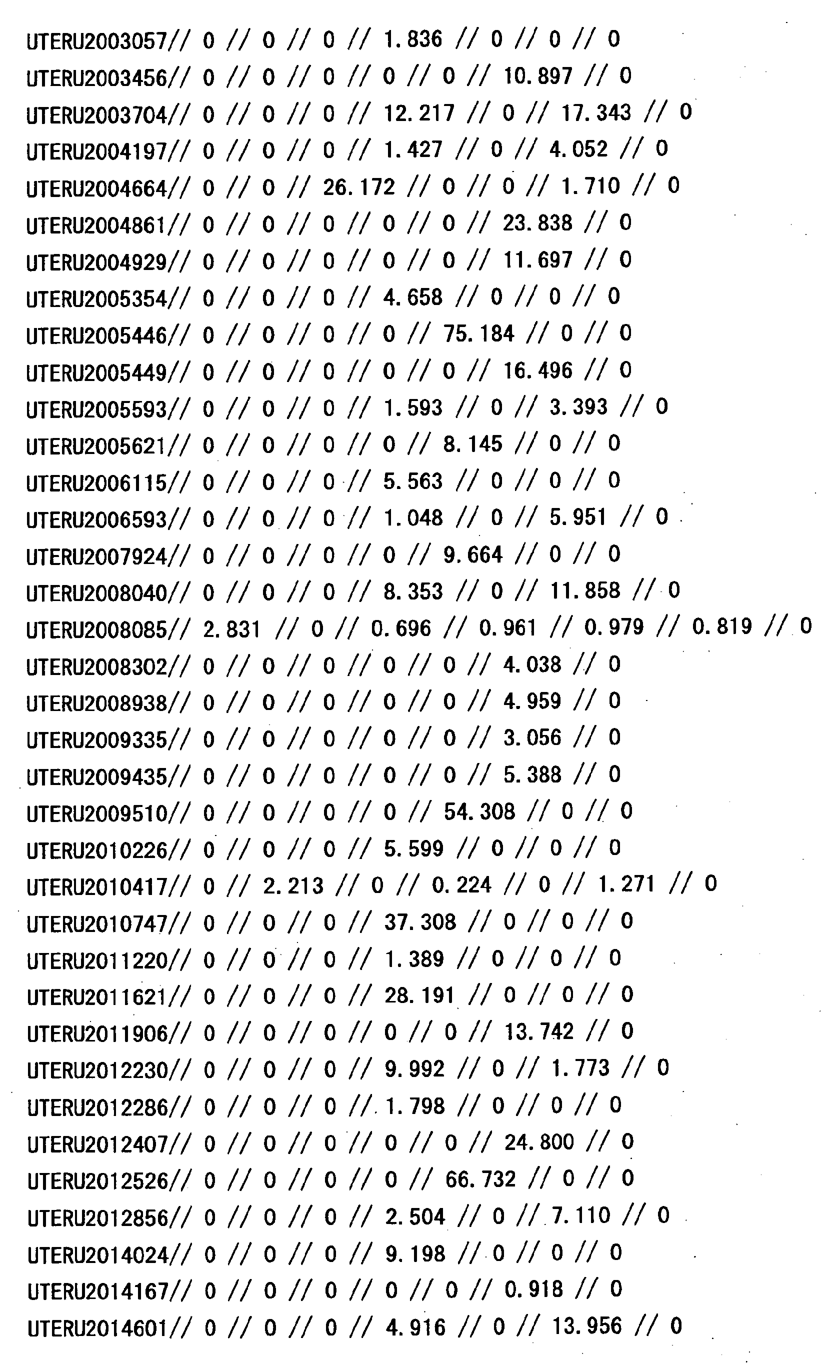

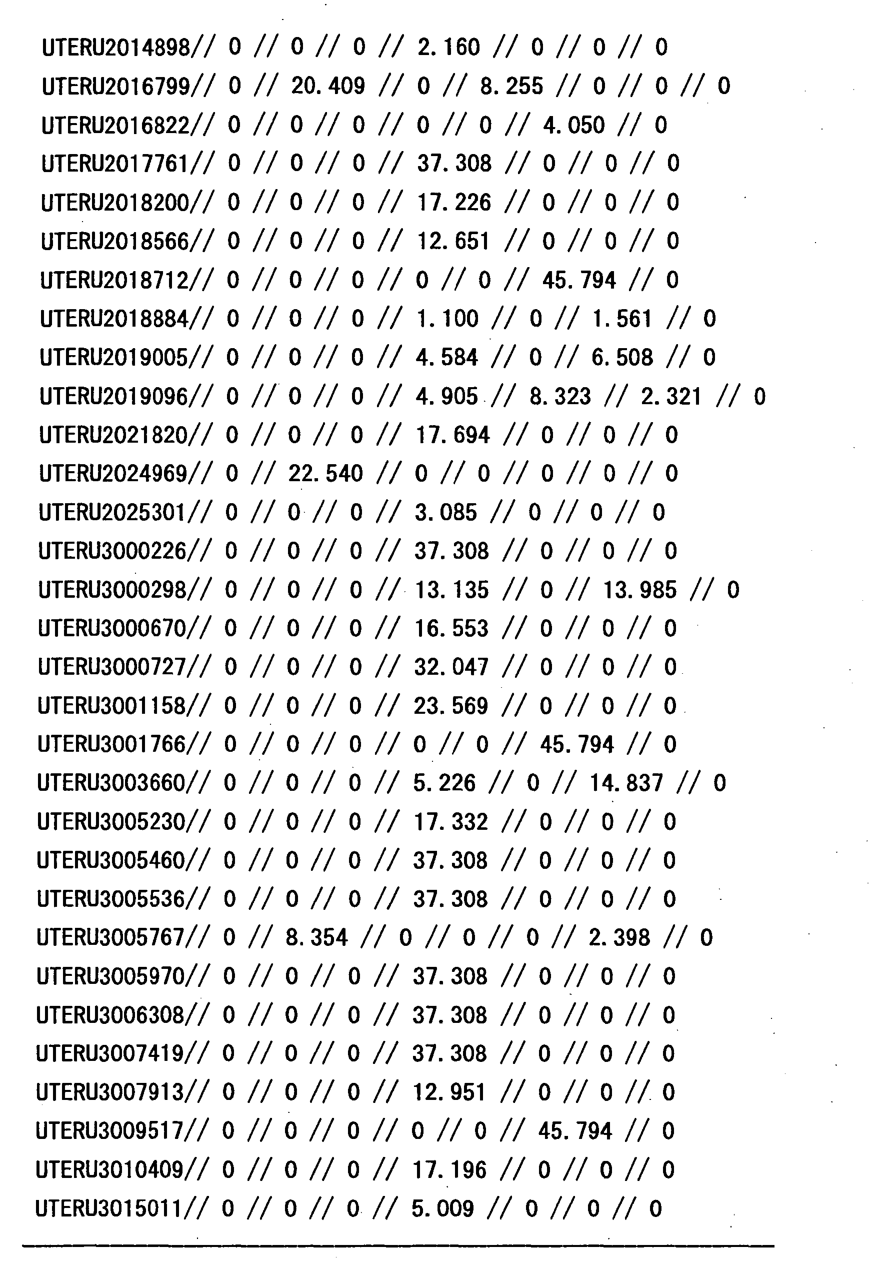

Genes whose expression levels were negligibly low in each of the above-described libraries are shown with their clone names below.
3NB691000018, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001002, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001501, 3NB692001507, 3NB692001511, 3NB692001519, 3NB692001520, 3NB692001528, 3NB692001538, 3NB692001548, 3NB692001557, 3NB692001637, 3NB692001861, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000038, ADRGL1000067, ADRGL1000111, ADRGL1000144, ADRGL1000147, ADRGL1000160, ADRGL1000165, ADRGL1000182, ADRGL2000056, ADRGL2000064, ADRGL2000085, ADRGL2000117, ADRGL2000142, ADRGL2000172, ADRGL2000248, ADRGL2000261, ADRGL2000323, ADRGL2000353, ADRGL2000384, ADRGL2000428, ADRGL2000636, ADRGL2000968, ADRGL2001119, ADRGL2001229, ADRGL2001287, ADRGL2001301, ADRGL2001354, ADRGL2001554, ADRGL2001651, ADRGL2001756, ADRGL2001836, ADRGL2001854, ADRGL2002029, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007357, ADRGL2007636, ADRGL2007651, ADRGL2007810, ADRGL2007877, ADRGL2008023, ADRGL2008331, ADRGL2008337, ADRGL2008535, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012179, AHMSC1000138, ASTRO1000009, ASTRO1000018, ASTRO1000029, ASTRO1000096, ASTRO1000165, ASTRO2000011, ASTRO2000095, ASTRO2000191, ASTRO2000278, ASTRO2000372, ASTRO2000403, ASTRO2000417, ASTRO2000482, ASTRO2000533, ASTRO2000653, ASTRO2000662, ASTRO2000725, ASTRO2000801, ASTRO2000914, ASTRO2001001, ASTRO2001008, ASTRO2001076, ASTRO2001088, ASTRO2001122, ASTRO2001227, ASTRO2001249, ASTRO2001480, ASTRO2002035, ASTRO2002064, ASTRO2002202, ASTRO2002571, ASTRO2002632, ASTRO2002659, ASTRO2002720, ASTRO2002743, ASTRO2002842, ASTRO2003574, ASTRO2003581, ASTRO2003632, ASTRO2003740, ASTRO2003925, ASTRO2004051, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005557, ASTRO2005575, ASTRO2005593, ASTRO2005687, ASTRO2005726, ASTRO2005863, ASTRO2005896, ASTRO2005902, ASTRO2006356, ASTRO2006732, ASTRO2006747, ASTRO2006920, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014923, ASTRO2014961, ASTRO2015162, ASTRO2015185, ASTRO2015529, ASTRO2015987, ASTRO2016044, ASTRO2016114, ASTRO2016148, ASTRO2016681, ASTRO2016847, ASTRO2016892, ASTRO2017348, ASTRO2017627, ASTRO2018373, ASTRO2019039, ASTRO3000154, ASTRO3000172, ASTRO3000177, ASTRO3000301, ASTRO3000474, ASTRO3000482, BEAST2000454, BRAWH1000002, BRAWH1000007, BRAWH1000116, BRAWH1000130, BRAWH1000157, BRAWH1000164, BRAWH1000168, BRAWH1000180, BRAWH1000369, BRAWH2000034, BRAWH2000048, BRAWH2000071, BRAWH2000093, BRAWH2000109, BRAWH2000142, BRAWH2000169, BRAWH2000218, BRAWH2000232, BRAWH2000248, BRAWH2000256, BRAWH2000323, BRAWH2000370, BRAWH2000409, BRAWH2000471, BRAWH2000476, BRAWH2000488, BRAWH2000503, BRAWH2000554, BRAWH2000588, BRAWH2000643, BRAWH2000651, BRAWH2000682, BRAWH2000752, BRAWH2000859, BRAWH2000866, BRAWH2000892, BRAWH2000901, BRAWH2000926, BRAWH2000944, BRAWH2000984, BRAWH2001052, BRAWH2001103, BRAWH2001129, BRAWH2001137, BRAWH2001159, BRAWH2001203, BRAWH2001239, BRAWH2001255, BRAWH2001355, BRAWH2001378, BRAWH2001406, BRAWH2001412, BRAWH2001424, BRAWH2001436, BRAWH2001438, BRAWH2001484, BRAWH2001492, BRAWH2001503, BRAWH2001519, BRAWH2001530, BRAWH2001589, BRAWH2001666, BRAWH2001679, BRAWH2001686, BRAWH2001701, BRAWH2001842, BRAWH2001862, BRAWH2001873, BRAWH2001940, BRAWH2001973, BRAWH2002062, BRAWH2002125, BRAWH2002191, BRAWH2002333, BRAWH2002352, BRAWH2002383, BRAWH2002623, BRAWH2002630, BRAWH2002635, BRAWH2002662, BRAWH2002679, BRAWH2002725, BRAWH2002761, BRAWH2002861, BRAWH2002967, BRAWH2002976, BRAWH2003135, BRAWH2003240, BRAWH2003307, BRAWH2003366, BRAWH2003689, BRAWH2003693, BRAWH2003818, BRAWH2003832, BRAWH2003948, BRAWH2003964, BRAWH2004044, BRAWH2004068, BRAWH2004136, BRAWH2004217, BRAWH2004580, BRAWH2004731, BRAWH2004884, BRAWH2004923, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006083, BRAWH2006395, BRAWH2006405, BRAWH2006450, BRAWH2006526, BRAWH2006760, BRAWH2006960, BRAWH2007069, BRAWH2007308, BRAWH2007406, BRAWH2007550, . BRAWH2007570, BRAWH2007658, BRAWH2007800, BRAWH2007808, BRAWH2007862, BRAWH2007890, BRAWH2008058, BRAWH2008128, BRAWH2008219, BRAWH2008292, BRAWH2008297, BRAWH2008366, BRAWH2008380, BRAWH2008540, BRAWH2008577, BRAWH2008790, BRAWH2009112, BRAWH2009227, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009907, BRAWH2010000, BRAWH2010069, BRAWH2010084, BRAWH2010318, BRAWH2010536, BRAWH2010584, BRAWH2010618, BRAWH2010619, BRAWH2010980, BRAWH2011066, BRAWH2011079, BRAWH2011096, BRAWH2011155, BRAWH2011166, BRAWH2011286, BRAWH2011294, BRAWH2011400, BRAWH2011762, BRAWH2011795, BRAWH2011796, BRAWH2011823, BRAWH2011860, BRAWH2011958, BRAWH2012054, BRAWH2012077, BRAWH2012161, BRAWH2012162, BRAWH2012258, BRAWH2012277, BRAWH2012326, BRAWH2012540, BRAWH2012619, BRAWH2012749, BRAWH2012963, BRAWH2013866, BRAWH2013871, BRAWH2013914, BRAWH2013941, BRAWH2013955, BRAWH2014234, BRAWH2014379, BRAWH2014473, BRAWH2014717, BRAWH2014729, BRAWH2014934, BRAWH2015349, BRAWH2015375, BRAWH2015595, BRAWH2015853, BRAWH2015866, BRAWH2016106, BRAWH2016160, BRAWH2016221, BRAWH2016223, BRAWH2016305, BRAWH2016439, BRAWH2016446, BRAWH2016514, BRAWH2016562, BRAWH2016655, BRAWH2016724, BRAWH2016785, BRAWH2016792, BRAWH2016800, BRAWH2017103, BRAWH2017250, BRAWH2017294, BRAWH2017305, BRAWH2017407, BRAWH2017433, BRAWH2017523, BRAWH2017534, BRAWH2017542, BRAWH2017635, BRAWH2017913, BRAWH2018206, BRAWH2018282, BRAWH2018317, BRAWH2018506, BRAWH2018514, BRAWH2018527, BRAWH2018601, BRAWH2018729, BRAWH2018745, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001326, BRAWH3001475, BRAWH3001636, BRAWH3001638, BRAWH3001712, BRAWH3001760, BRAWH3001783, BRAWH3001833, BRAWH3001879, BRAWH3002433, BRAWH3002467, BRAWH3002513, BRAWH3002574, BRAWH3002819, BRAWH3003019, BRAWH3003136, BRAWH3003244, BRAWH3003343, BRAWH3003411, BRAWH3003522, BRAWH3003598, BRAWH3003801, BRAWH3003975, BRAWH3004222, BRAWH3004335, BRAWH3004350, BRAWH3004453, BRAWH3004666, BRAWH3005037, BRAWH3005047, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006792, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009546, BRAWH3009961, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017260, BRAWH3017477, BRAWH3017537, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3030613, BRAWH3030772, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034775, BRAWH3034890, BRAWH3035154, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001866, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006051, BRCAN2006063, BRCAN2006174, BRCAN2006290, BRCAN2006297, BRCAN2006323, BRCAN2006411, BRCAN2006450, BRCAN2006955, BRCAN2007119, BRCAN2007144, BRCAN2007409, BRCAN2007426, BRCAN2007525, BRCAN2008494, BRCAN2008528, BRCAN2008701, BRCAN2009156, BRCAN2009168, BRCAN2009203, BRCAN2010547, BRCAN2010581, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014952, BRCAN2015402, BRCAN2015464, BRCAN2015757, BRCAN2015886, BRCAN2016025,. BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019773, BRCAN2019907, BRCAN2020234, BRCAN2020331, BRCAN2020412, BRCAN2020467, BRCAN2020880, BRCAN2021024, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025712, BRCAN2025982, BRCAN2026117, BRCAN2026711, BRCAN2027150, BRCAN2027310, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC2000152, BRCOC2000184, BRCOC2000360, BRCOC2000404, BRCOC2000487, BRCOC2000637, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003125, BRCOC2003513, BRCOC2003732, BRCOC2003740, BRCOC2004175, BRCOC2004354, BRCOC2004455, BRCOC2004950, BRCOC2005951, BRCOC2006164, BRCOC2006243, BRCOC2006639, BRCOC2006942, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2010115, BRCOC2010123, BRCOC2010510, BRCOC2010730, BRCOC2010980, BRCOC2010994, BRCOC2011010, BRCOC2011398, BRCOC2011418, BRCOC2011769, BRCOC2011996, BRCOC2012229, BRCOC2012386, BRCOC2012813, BRCOC2013448, BRCOC2013573, BRCOC2013675, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019903, BRCOC2019934, BRCOC2020122, BRCOC2020142, BRHIP1000058, BRHIP1000072, BRHIP1000258, BRHIP2000021, BRHIP2000079, BRHIP2000163, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2001074, BRHIP2001686, BRHIP2001782, BRHIP2001802, BRHIP2001805, BRHIP2001927, BRHIP2002122, BRHIP2002172, BRHIP2002346, BRHIP2002510, BRHIP2002664, BRHIP2002722, BRHIP2002929, BRHIP2003048, BRHIP2003242, BRHIP2003272, BRHIP2003748, BRHIP2003786, BRHIP2003828, BRHIP2003917, BRHIP2004312, BRHIP2004355, BRHIP2004814, BRHIP2004883, BRHIP2004902, BRHIP2005236, BRHIP2005354, BRHIP2005600, BRHIP2005719, BRHIP2005724, BRHIP2005932, BRHIP2006617, BRHIP2006782, BRHIP2006800, BRHIP2006819, BRHIP2006921, BRHIP2007307, BRHIP2007616, BRHIP2007690, BRHIP2007928, BRHIP2007969, BRHIP2008269, BRHIP2008389, BRHIP2008756, BRHIP2008909, BRHIP2009177, BRHIP2009340, BRHIP2009351, BRHIP2009414, BRHIP2009474, BRHIP2009617, BRHIP2009685, BRHIP2010309, BRHIP2010444, BRHIP2010593, BRHIP2010610, BRHIP2010744, BRHIP2011080, BRHIP2011199, BRHIP2011508, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013723, BRHIP2013958, BRHIP2013972, BRHIP2014063, BRHIP2014285, BRHIP2014291, BRHIP2014373, BRHIP2014386, BRHIP2014387, BRHIP2014954, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017714, BRHIP2017780, BRHIP2018014, BRHIP2018369, BRHIP2018612, BRHIP2018635, BRHIP2018650, BRHIP2018805, BRHIP2018998, BRHIP2019007, BRHIP2019047, BRHIP2019149, BRHIP2019177, BRHIP2019291, BRHIP2019589, BRHIP2019641, BRHIP2019884, BRHIP2019972, BRHIP2020182, BRHIP2020509, BRHIP2020695, BRHIP2020743, BRHIP2020842, BRHIP2020859, BRHIP2020930, BRHIP2021102, BRHIP2021289, BRHIP2021615, BRHIP2021744, BRHIP2021929, BRHIP2022006, BRHIP2022221, BRHIP2022228, BRHIP2022298, BRHIP2022467, BRHIP2022708, BRHIP2023229, BRHIP2023272, BRHIP2023292, BRHIP2023369, BRHIP2023438, BRHIP2023695, BRHIP2023735, BRHIP2023773, BRHIP2023860, BRHIP2023869, BRHIP2024046, BRHIP2024165, BRHIP2024201, BRHIP2024347, BRHIP2024549, BRHIP2024789, BRHIP2024911, BRHIP2024941, BRHIP2025245, BRHIP2025366, BRHIP2025739, BRHIP2025797, BRHIP2026021, BRHIP2026155, BRHIP2026214, BRHIP2026346, BRHIP2026557, BRHIP2026723, BRHIP2026877, BRHIP2027017, BRHIP2027054, BRHIP2027563, BRHIP2027762, BRHIP2028303, BRHIP2028330, BRHIP2028480, BRHIP2028593, BRHIP2029176, BRHIP2029283, BRHIP2029393, BRHIP2029529, BRHIP2029663, BRHIP3000017, BRHIP3000060, BRHIP3000070, BRHIP3000081, BRHIP3000475, BRHIP3000488, BRHIP3000526, BRHIP3000626, BRHIP3000859, BRHIP3000907, BRHIP3001076, BRHIP3001079, BRHIP3001141, BRHIP3001338, BRHIP3001481, BRHIP3001878, BRHIP3001964, BRHIP3002124, BRHIP3002141, BRHIP3002363, BRHIP3002920, BRHIP3002931, BRHIP3003306, BRHIP3003340, BRHIP3003395, BRHIP3003484, BRHIP3003644, BRHIP3003688, BRHIP3003795, BRHIP3004193, BRHIP3004416, BRHIP3004710, BRHIP3004725, BRHIP3004774, BRHIP3004786, BRHIP3004863, BRHIP3005037, BRHIP3005137, BRHIP3005142, BRHIP3005231, BRHIP3005307, BRHIP3005768, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007960, BRHIP3008082, BRHIP3008183, BRHIP3008314, BRHIP3008320, BRHIP3008344, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3015620, BRHIP3015751, BRHIP3015854, BRHIP3016032, BRHIP3016213, BRHIP3016302, BRHIP3016421, BRHIP3017109, BRHIP3017256, BRHIP3017325, BRHIP3017558, BRHIP3017637, BRHIP3018784, BRHIP3019643, BRHIP3019824, BRHIP3019880, BRHIP3019956, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026097, BRHIP3026231, BRHIP3026335, BRHIP3026651, BRHIP3027105, BRHIP3027137, BRHIP3027160, BRHIP3027191, BRHIP3027501, BRHIP3027651, BRHIP3027854, BRHIP3027947, BRHIP3028570, BRHIP3028742, BRSSN1000056, BRSSN1000059, BRSSN1000092, BRSSN1000153, BRSSN2000175, BRSSN2000197, BRSSN2000244, BRSSN2000518, BRSSN2000684, BRSSN2000715, BRSSN2001213, BRSSN2001275, BRSSN2001342, BRSSN2001426, BRSSN2001503, BRSSN2001579, BRSSN2001810, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004657, BRSSN2004679, BRSSN2004686, BRSSN2004710, BRSSN2004719, BRSSN2005847, BRSSN2006133, BRSSN2006390, BRSSN2006575, BRSSN2006644, BRSSN2007076, BRSSN2007168, BRSSN2007202, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010830, BRSSN2011653, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013753, BRSSN2013874, BRSSN2014112, BRSSN2014424, BRSSN2014513, BRSSN2014556, BRSSN2014573, BRSSN2014636, BRSSN2015369, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018581, BRSSN2018731, BRSSN2018771, BRSSN2018925, BRSTN1000097, BRSTN2000070, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006583, BRSTN2006638, BRSTN2006865, BRSTN2007000, BRSTN2007118, BRSTN2007284, BRSTN2007765, BRSTN2008283, BRSTN2008367, BRSTN2008418, BRSTN2008457, BRSTN2008475, BRSTN2008930, BRSTN2009247, BRSTN2010363, BRSTN2010416, BRSTN2010500, BRSTN2010569, BRSTN2010923, BRSTN2011211, BRSTN2011688, BRSTN2011799, BRSTN2012284, BRSTN2012320, BRSTN2012380, BRSTN2013171, BRSTN2013354, BRSTN2014490, BRSTN2014633, BRSTN2014751, BRSTN2014770, BRSTN2016066, BRSTN2016335, BRSTN2016678, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2009311, BRTHA2009349, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011321, BRTHA2011351, BRTHA2011500, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013262, BRTHA2013275, BRTHA2013426, BRTHA2013460, BRTHA2013515, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3001221, BRTHA3001623, BRTHA3002265, BRTHA3002401, BRTHA3002411, BRTHA3002864, BRTHA3002933, BRTHA3002955, BRTHA3003000, BRTHA3003074, BRTHA3003225, BRTHA3003339, BRTHA3003343, BRTHA3003417, BRTHA3003449, BRTHA3003474, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009291, BRTHA3010135, BRTHA3010366, BRTHA3010469, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021786, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000797, CHONS2001287, CHONS2001382, CHONS2001797, CHONS2001834, CHONS2002419, COLON1000030, COLON1000084, COLON1000135, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000093, CTONG1000097, CTONG1000113, CTONG1000137, CTONG1000165, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000218, CTONG2000279, CTONG2000303, CTONG2000330, CTONG2000395, CTONG2000411, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001681, CTONG2001724, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002395, CTONG2002417, CTONG2002418, CTONG2002453, CTONG2002558, CTONG2002590, CTONG2002721, CTONG2002816, CTONG2002820, CTONG2002832, CTONG2002903, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003764, CTONG2003782, CTONG2003889, CTONG2003937, CTONG2003945, CTONG2004000, CTONG2004062, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006798, CTONG2006836, CTONG2006942, CTONG2006964, CTONG2007009, CTONG2007091, CTONG2007104, CTONG2007175, CTONG2007259, CTONG2007272, CTONG2007293, CTONG2007297, CTONG2007399, CTONG2007417, CTONG2007441, CTONG2007586, CTONG2007613, CTONG2007623, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008466, CTONG2008518, CTONG2008521, CTONG2008562, CTONG2008577, CTONG2008595, CTONG2008621, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010652, CTONG2011188, CTONG2011429, CTONG2011474, CTONG2011676, CTONG2011716, CTONG2011765, CTONG2011779, CTONG2011801, CTONG2011815, CTONG2011822, CTONG2011920, CTONG2011985, CTONG2012029, CTONG2012050, CTONG2012077, CTONG2012123, CTONG2012158, CTONG2012230, CTONG2012422, CTONG2012425, CTONG2012447, CTONG2012473, CTONG2012476, CTONG2012533, CTONG2012554, CTONG2012564, CTONG2012607, CTONG2012661, CTONG2012724, CTONG2012738, CTONG2012758, CTONG2012843, CTONG2012901, CTONG2012996, CTONG2013128, CTONG2013149, CTONG2013.156, CTONG2013178, CTONG2013222, CTONG2013284, CTONG2013339, CTONG2013352, CTONG2013381, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014389, CTONG2014491, CTONG2014630, CTONG2014635, CTONG2014646, CTONG2014705, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016846, CTONG2016904, CTONG2016942, CTONG2016953, CTONG2017021, CTONG2017292, CTONG2017409, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018379, CTONG2018383, CTONG2018413, CTONG2018483, CTONG2018629, CTONG2018652, CTONG2018653, CTONG2018655, CTONG2018735, CTONG2018808, CTONG2018900, CTONG2018976, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020378, CTONG2020411, CTONG2020445, CTONG2020522, CTONG2020560, CTONG2020638, CTONG2020806, CTONG2020831, CTONG2020974, CTONG2021024, CTONG2021132, CTONG2021168, CTONG2022153, CTONG2022601, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2028097, CTONG2028486, CTONG2028518, CTONG2028687, CTONG2028758, CTONG3000686, CTONG3000707, CTONG3000896, CTONG3001123, CTONG3001420, CTONG3001501, CTONG3001560, CTONG3001605, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009385, D3OST1000066, D3OST1000109, D3OST1000238, D3OST1000267, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000507, D9OST2000031, D9OST2000278, D9OST2000440, D9OST2000869, D9OST2001319, D9OST2001433, D9OST2002397, D9OST2002673, D9OST2003106, D9OST2003137, D9OST2003397, D9OST2003594, D9OST2003762, D9OST2003791, D9OST2003989, D9OST2004018, D9OST2004073, D9OST2004417, DFNES1000003, DFNES1000107, DFNES1000150, DFNES1000185, DFNES2000011, DFNES2000143, DFNES2000146, DFNES2000153, DFNES2000292, DFNES2000426, DFNES2000432, DFNES2000443, DFNES2000471, DFNES2000913, DFNES2001091, DFNES2001108, DFNES2001177, DFNES2001404, DFNES2001564, DFNES2001565, DFNES2001647, DFNES2001800, DFNES2002220, DFNES2002509, DFNES2002550; DFNES2002588, DFNES2002817, DFNES2002966, DFNES2003081, DFNES2003255, DFNES2003742, DFNES2004346, DFNES2004354, DFNES2004383, DFNES2004608, DFNES2004684, DFNES2005266, DFNES2005527, DFNES2005766, DFNES2005779, DFNES2005875, DFNES2006346, DFNES2006944, DFNES2007113, DFNES2007299, DFNES2007332, DFNES2007641, DFNES2007757, DFNES2008280, DFNES2008881, DFNES2010502, DFNES2011192, DFNES2011221, DFNES2011239, DFNES2011245, ERLTF1000021, ERLTF2000324, ERLTF2000465, FCBBF1000023, FCBBF1000025, FCBBF1000038, FCBBF1000061, FCBBF1000069, FCBBF1000117, FCBBF1000118, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000412, FCBBF1000425, FCBBF1000437, FCBBF1000449, FCBBF1000506, FCBBF1000546, FCBBF1000550, FCBBF1000581, FCBBF1000582, FCBBF1000598, FCBBF1000604, FCBBF1000618, FCBBF1000627, FCBBF1000684, FCBBF1000686, FCBBF1000691, FCBBF1000748, FCBBF2000087, FCBBF2000094, FCBBF2000105, FCBBF2000123, FCBBF2000195, FCBBF2000214, FCBBF2000232, FCBBF2000259, FCBBF2000276, FCBBF2000291, FCBBF2000379, FCBBF2000473, FCBBF2000502, FCBBF2000576, FCBBF2000591, FCBBF2000677, FCBBF2000678, FCBBF2000685, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001188, FCBBF2001238, FCBBF2001243, FCBBF2001309, FCBBF2001400, FCBBF2001427, FCBBF2001480, FCBBF2001529, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003636, FCBBF2003823, FCBBF2003895, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005439, FCBBF2005538, FCBBF2005637, FCBBF2005645, FCBBF2005658, FCBBF2005733, FCBBF2006131, FCBBF2006380, FCBBF2006418, FCBBF2006452, FCBBF2006634, FCBBF2007012, FCBBF2007186, FCBBF2007265, FCBBF2007302, FCBBF2007610, FCBBF2007633, FCBBF3000102, FCBBF3000110, FCBBF3000120, FCBBF3000227, FCBBF3000229, FCBBF3000233, FCBBF3000361, FCBBF3000367, FCBBF3000462, FCBBF3000473, FCBBF3000501, FCBBF3000518, FCBBF3000536, FCBBF3000550, FCBBF3000847, FCBBF3001018, FCBBF3001020, FCBBF3001235, FCBBF3001281, FCBBF3001302, FCBBF3001377, FCBBF3001594, FCBBF3001890, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007829, FCBBF3007855, FCBBF3008073, FCBBF3008159, FCBBF3008251, FCBBF3008311, FCBBF3008362, FCBBF3008382, FCBBF3008475, FCBBF3008556, FCBBF3008644, FCBBF3008748, FCBBF3008870, FCBBF3008938, FCBBF3008944, FCBBF3009101, FCBBF3009152, FCBBF3009256, FCBBF3009317, FCBBF3009428, FCBBF3009464, FCBBF3009479, FCBBF3009703, FCBBF3009717, FCBBF3009888, FCBBF3009949, FCBBF3009978, FCBBF3010008, FCBBF3010124, FCBBF3010130, FCBBF3010149, FCBBF3010211, FCBBF3010361, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012288, FCBBF3012332, FCBBF3012347, FCBBF3012443, FCBBF3012588, FCBBF3012842, FCBBF3012901, FCBBF3013041, FCBBF3013058, FCBBF3013205, FCBBF3013266, FCBBF3013307, FCBBF3013341, FCBBF3013589, FCBBF3013623, FCBBF3013800, FCBBF3013846, FCBBF3014229, FCBBF3014355, FCBBF3014567, FCBBF3014770, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017974, FCBBF3018067, FCBBF3018173, FCBBF3018591, FCBBF3018753, FCBBF3018949, FCBBF3019085, FCBBF3019437, FCBBF3019455, FCBBF3019564, FCBBF3019570, FCBBF3019663, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021940, FCBBF3021985, FCBBF3022005, FCBBF3022504, FCBBF3022566, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024096, FCBBF3024225, FCBBF3024364, FCBBF3024623, FCBBF3024911, FCBBF3025098, FCBBF3025252, FCBBF3025280, FCBBF3025285, FCBBF3025528, FCBBF3025568, FCBBF3025650, FCBBF3025674, FCBBF3025730, FCBBF3025737, FCBBF3026021, FCBBF3026048, FCBBF3026251, FCBBF3026308, FCBBF3026678, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028593, FCBBF3028671, FCBBF3028794, FCBBF4000061, FCBBF4000076, FCBBF4000173, FCBBF4000192, FCBBF4000193, FCBBF4000268, FCBBF4000282, FCBBF4000399, FCBBF4000415, FCBBF4000500, FCBBF4000529, FCBBF5000041, FCBBF5000061, FCBBF5000261, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA1000190, FEBRA2000021, FEBRA2000035, FEBRA2000053, FEBRA2000126, FEBRA2000220, FEBRA2000228, FEBRA2000253, FEBRA2000282, FEBRA2000286, FEBRA2000297, FEBRA2000311, FEBRA2000321, FEBRA2000333, FEBRA2000378, FEBRA2000391, FEBRA2000394, FEBRA2000397, FEBRA2000399, FEBRA2000409, FEBRA2000415, FEBRA2000436, FEBRA2000454, FEBRA2000468, FEBRA2000491, FEBRA2000504, FEBRA2000510, FEBRA2000532, FEBRA2000538, FEBRA2000575, FEBRA2000656, FEBRA2000659, FEBRA2000690, FEBRA2000727, FEBRA2000734, FEBRA2000762, FEBRA2000772, FEBRA2000790, FEBRA2000803, FEBRA2000805, FEBRA2000809, FEBRA2000856, FEBRA2000862, FEBRA2000901, FEBRA2000909, FEBRA2000917, FEBRA2000928, FEBRA2000936, FEBRA2000937, FEBRA2000959, FEBRA2001012, FEBRA2001020, FEBRA2001093, FEBRA2001124, FEBRA2001133, FEBRA2001161, FEBRA2001175, FEBRA2001217, FEBRA2001227, FEBRA2001267, FEBRA2001351, FEBRA2001438, FEBRA2001440, FEBRA2001487, FEBRA2001523, FEBRA2001561, FEBRA2001581, FEBRA2001584, FEBRA2001590, FEBRA2001591, FEBRA2001610, FEBRA2001620, FEBRA2001659, FEBRA2001698, FEBRA2001705, FEBRA2001706, FEBRA2001772, FEBRA2001790, FEBRA2001867, FEBRA2001914, FEBRA2001989, FEBRA2002009, FEBRA2002086, FEBRA2002109, FEBRA2002260, FEBRA2002399, FEBRA2002410, FEBRA2002429, FEBRA2002442, FEBRA2002508, FEBRA2002525, FEBRA2002552, FEBRA2002574, FEBRA2002601, FEBRA2002611, FEBRA2002628, FEBRA2002662, FEBRA2002682, FEBRA2002707, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003115, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003520, FEBRA2003524, FEBRA2003675, FEBRA2003707, FEBRA2003711, FEBRA2003822, FEBRA2003897, FEBRA2003930, FEBRA2004023, FEBRA2004026, FEBRA2004029, FEBRA2004053, FEBRA2004056, FEBRA2004110, FEBRA2004191, FEBRA2004202, FEBRA2004219, FEBRA2004325, FEBRA2004329, FEBRA2004331, FEBRA2004443, FEBRA2004490, FEBRA2004538, FEBRA2004592, FEBRA2004651, FEBRA2004813, FEBRA2004840, FEBRA2004852, FEBRA2004867, FEBRA2004914, FEBRA2004933, FEBRA2004987, FEBRA2005014, FEBRA2005036, FEBRA2005216, FEBRA2005291, FEBRA2005377, FEBRA2005380, FEBRA2005427, FEBRA2005458, FEBRA2005701, FEBRA2005726, FEBRA2005778, FEBRA2005787, FEBRA2005995, FEBRA2005998, FEBRA2006055, FEBRA2006061, FEBRA2006150, FEBRA2006165, FEBRA2006315, FEBRA2006354, FEBRA2006370, FEBRA2006379, FEBRA2006409, FEBRA2006418, FEBRA2006427, FEBRA2006476, FEBRA2006491, FEBRA2006627, FEBRA2006664, FEBRA2006667, FEBRA2006726, FEBRA2006793, FEBRA2006817, FEBRA2006890, FEBRA2006942, FEBRA2006977, FEBRA2007176, FEBRA2007200, FEBRA2007247, FEBRA2007280, FEBRA2007414, FEBRA2007436, FEBRA2007449, FEBRA2007458, FEBRA2007551, FEBRA2007566, FEBRA2007620, FEBRA2007793, FEBRA2007818, FEBRA2007823, FEBRA2007931, FEBRA2007957, FEBRA2008009, FEBRA2008086, FEBRA2008135, FEBRA2008165, FEBRA2008172, FEBRA2008266, FEBRA2008282, FEBRA2008311, FEBRA2008341, FEBRA2008360, FEBRA2008468, FEBRA2008475, FEBRA2008481, FEBRA2008542, FEBRA2008660, FEBRA2008681, FEBRA2008692, FEBRA2008741, FEBRA2008755, FEBRA2008836, FEBRA2008866, FEBRA2008881, FEBRA2008983, FEBRA2009016, FEBRA2009029, FEBRA2009074, FEBRA2009117, FEBRA2009120, FEBRA2009162, FEBRA2009240, FEBRA2009265, FEBRA2009276, FEBRA2009327, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012195, FEBRA2012397, FEBRA2012418, FEBRA2012625, FEBRA2013019, FEBRA2013069, FEBRA2013274, FEBRA2013465, FEBRA2013570, FEBRA2013905, FEBRA2013951, FEBRA2014010, FEBRA2014122, FEBRA2014198, FEBRA2014213, FEBRA2014443, FEBRA2014578, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017736, FEBRA2017780, FEBRA2017811, FEBRA2017850, FEBRA2018051, FEBRA2018407, FEBRA2019172, FEBRA2019298, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021134, FEBRA2021339, FEBRA2021431, FEBRA2021497, FEBRA2021571, FEBRA2021892, FEBRA2021908, FEBRA2022013, FEBRA2022148, FEBRA2022204, FEBRA2022255, FEBRA2022322, FEBRA2022504, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023498, FEBRA2023721, FEBRA2023989, FEBRA2023990, FEBRA2024136, FEBRA2024150, FEBRA2024343, FEBRA2024570, FEBRA2024693, FEBRA2024727, FEBRA2024744, FEBRA2024797, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000164, FELNG2000241, HCASM1000021, HCASM1000050, HCASM1000061, HCASM1000130, HCASM2000202, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142, HCHON1000176, HCHON2000028, HCHON2000038, HCHON2000087, HCHON2000100, HCHON2000156, HCHON2000199, HCHON2000212, HCHON2000271, HCHON2000315, HCHON2000322, HCHON2000344, HCHON2000364, HCHON2000373, HCHON2000395, HCHON2000417, HCHON2000418, HCHON2000503, HCHON2000660, HCHON2000663, HCHON2000673, HCHON2000699, HCHON2000705, HCHON2000738, HCHON2000751, HCHON2000817, HCHON2000818, HCHON2000832, HCHON2000851, HCHON2000898, HCHON2000935, HCHON2000942, HCHON2000956, HCHON2000994, HCHON2001084, HCHON2001099, HCHON2001110, HCHON2001116, HCHON2001214, HCHON2001233, HCHON2001269, HCHON2001450, HCHON2001453, HCHON2001519, HCHON2001523, HCHON2001535, HCHON2001546, HCHON2001548, HCHON2001577, HCHON2001598, HCHON2001604, HCHON2001607, HCHON2001646, HCHON2001712, HCHON2001768, HCHON2001801, HCHON2002123, HCHON2002194, HCHON2002241, HCHON2002496, HCHON200267.6, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000088, HEART1000099, HEART1000102, HEART1000118, HEART1000132, HEART1000139, HEART1000142, HEART1000151, HEART1000185, HEART2000024, HEART2000035, HEART2000099, HEART2000298, HEART2000309, HEART2000411, HEART2000418, HEART2000444, HEART2000448, HEART2000492, HEART2000541, HEART2000568, HEART2000611, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2004570, HEART2004764, HEART2004931, HEART2004940, HEART2004941, HEART2004980, HEART2005244, HEART2005581, HEART2005610, HEART2006131, HEART2006195, HEART2006310, HEART2006334, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009599, HEART2009680, HEART2010391, HEART2010492, HEART2010495, HELAC2000158, HELAC2000301, HHDPC1000001, HHDPC1000065, HHDPC1000118, HHDPC2000070, HHDPC2000102, HHDPC2000258, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006460, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000884, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001633, HLUNG2001677, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003497, HLUNG2003691, HLUNG2003710, HLUNG2003714, HLUNG2003778, HLUNG2003833, HLUNG2003872, HLUNG2003918, HLUNG2003991, HLUNG2004014, HLUNG2004037, HLUNG2004154, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007210, HLUNG2007219, HLUNG2007245, HLUNG2007433, HLUNG2007931, HLUNG2008066, HLUNG2008225, HLUNG2008235, HLUNG2008247, HLUNG2008333, HLUNG2008384, HLUNG2008396, HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000148, HSYRA2000044, HSYRA2000135, HSYRA2000221, HSYRA2000237, HSYRA2000253, HSYRA2000332, HSYRA2000371, HSYRA2000424, HSYRA2000507, HSYRA2000510, HSYRA2000605, HSYRA2000760, HSYRA2000792, HSYRA2000832, HSYRA2000927, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001580, HSYRA2001631, HSYRA2001638, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000207, IMR321000230, IMR321000253, IMR322000010, IMR322000107, IMR322000144, IMR322000152, IMR322000164, IMR322000178, IMR322000302, IMR322000374, IMR322000379, IMR322000383, IMR322000418, IMR322000604, IMR322000609, IMR322000656, IMR322000672, IMR322000791, IMR322000811, IMR322000838, IMR322000917, IMR322000919, IMR322000935, IMR322000953, IMR322000984, IMR322001018, IMR322001167, IMR322001185, IMR322001245, IMR322001452, IMR322001491, IMR322001665, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000375, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2001117, KIDNE2001140, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002198, KIDNE2002252, KIDNE2002262, KIDNE2002438, KIDNE2002483, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003316, KIDNE2003335, KIDNE2003357, KIDNE2003373, KIDNE2003377, KIDNE2003507, KIDNE2003752, KIDNE2003837, KIDNE2003994, KIDNE2004034, KIDNE2004084, KIDNE2004095, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006652, KIDNE2006687, KIDNE2006733, KIDNE2006811, KIDNE2006820, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007811, KIDNE2007910, KIDNE2007944, KIDNE2008022, KIDNE2008072, KIDNE2008116, KIDNE2008117, KIDNE2008218, KIDNE2008315, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052; KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011782, KIDNE2011858, KIDNE2012025, KIDNE2012188, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017315, KIDNE2017332, KIDNE2017343, KIDNE2017454, KIDNE2017603, KIDNE2017956, KIDNE2018166, KIDNE2018167, KIDNE2018254, KIDNE2018268, KIDNE2018269, KIDNE2018287, KIDNE2018294, KIDNE2018352, KIDNE2018462, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000111, LIVER1000126, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001329, LIVER2001389, LIVER2001461, LIVER2001539, LIVER2001608, LIVER2001835, LIVER2002638, LIVER2002644, LIVER2002660, LIVER2002702, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000096, MAMGL1000132, MAMGL1000151, MAMGL1000178, MAMGL1000182, MAMGL1000184, MESAN1000032, MESAN1000035, MESAN1000079, MESAN1000121, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000620, MESAN2000711, MESAN2000815, MESAN2000894, MESAN2000909, MESAN2001627, MESAN2001770, MESAN2001979, MESAN2001980, MESAN2002113, MESAN2002147, MESAN2002424, MESAN2002687, MESAN2002709, MESAN2002790, MESAN2002844, MESAN2002940, MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003851, MESAN2004138, MESAN2004288, MESAN2004575, MESAN2005284, MESAN2005303, MESAN2005371, MESAN2005633, MESAN2005724, MESAN2005808, MESAN2005811, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2007755, MESAN2008222, MESAN2008415, MESAN2008460, MESAN2008536, MESAN2008679, MESAN2008821, MESAN2008926, MESAN2009019, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015391, MESAN2015401, MESAN2015501, MESAN2015708, MESAN2016159, MESAN2016304, MESAN2016409, MESAN2016552, MESAN2016965, MESAN2017152, MESAN2017417, MESAN2018209, MESAN2018576, MESAN2018670, MESTC1000147, MESTC2000150, MESTC2000153, MESTC2000170, N1ESE2000698, NB9N41000047, NB9N41000142, NB9N41000146, NB9N42000104, NB9N42000122, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2002352, NESOP2002418, NESOP2002487, NESOP2002738, NESOP2002780, NETRP1000034, NETRP1000082, NETRP2000086, NETRP2000182, NETRP2000439, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, NT2NE1000004, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000455, NT2NE2000470, NT2NE2000488, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000950, NT2NE2000951, NT2NE2000980, NT2NE2001005, NT2NE2001021, NT2NE2001049, NT2NE2001142, NT2NE2001180, NT2NE2001181, NT2NE2001188, NT2NE2001191, NT2NE2001247, NT2NE2001252, NT2NE2001326, NT2NE2001372, NT2NE2001428, NT2NE2001432, NT2NE2001524, NT2NE2001530, NT2NE2001598, NT2NE2001617, NT2NE2001623, NT2NE2001626, NT2NE2001634, NT2NE2001648, NT2NE2001660, NT2NE2001666, NT2NE2001677, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003887, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005323, NT2NE2005358, NT2NE2005371, NT2NE2005395, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008867, NT2NE2008961, NT2NE2008997, NT2NE2009138, NT2NE2009295, NT2NE2009426, NT2NE2009608, NT2NE2009691, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012243, NT2NE2012361, NT2NE2012448, NT2NE2012457, NT2NE2012493, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000035, NT2RI1000048, NT2RI1000164, NT2RI1000172, NT2RI2000004, NT2RI2000007, NT2RI2000117, NT2RI2000128, NT2RI2000173, NT2RI2000187, NT2RI2000270, NT2RI2000276, NT2RI2000313, NT2RI2000341, NT2RI2000348, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000775, NT2RI2000822, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001235, NT2RI2001244, NT2RI2001307, NT2RI2001409, NT2RI2001410, NT2RI2001425, NT2RI2001449, NT2RI2001474, NT2RI2001519, NT2RI2001595, NT2RI2001624, NT2RI2001657, NT2RI2001726, NT2RI2001782, NT2RI2001836, NT2RI2001846, NT2RI2001910, NT2RI2001952, NT2RI2002022, NT2RI2002041, NT2RI2002117, NT2RI2002120, NT2RI2002153, NT2RI2002179, NT2RI2002252, NT2RI2002260, NT2RI2002270, NT2RI2002334, NT2RI2002359, NT2RI2002447, NT2RI2002481, NT2RI2002507, NT2RI2002517, NT2RI2002530, NT2RI2002549, NT2RI2002564, NT2RI2002592, NT2RI2002602, NT2RI2002729, NT2RI2002822, NT2RI2002852, NT2RI2002865, NT2RI2002923, NT2RI2002926, NT2RI2002970, NT2RI2003019, NT2RI2003067, NT2RI2003184, NT2RI2003360, NT2RI2003383, NT2RI2003392, NT2RI2003399, NT2RI2003419, NT2RI2003487, NT2RI2003667, NT2RI2003751, NT2RI2003884, NT2RI2003973, NT2RI2003993, NT2RI2004078, NT2RI2004079, NT2RI2004093, NT2RI2004136, NT2RI2004157, NT2RI2004188, NT2RI2004190, NT2RI2004209, NT2RI2004230, NT2RI2004312, NT2RI2004381, NT2RI2004442, NT2RI2004468, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005130, NT2RI2005315, NT2RI2005358, NT2RI2005368, NT2RI2005382, NT2RI2005520, NT2RI2005579, NT2RI2005621, NT2RI2005628, NT2RI2005647, NT2RI2005670, NT2RI2005710, NT2RI2005772, NT2RI2005814, NT2RI2005818, NT2RI2005966, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506, NT2RI2006553, NT2RI2006565, NT2RI2006659, NT2RI2006662, NT2RI2006667, NT2RI2006679, NT2RI2006686, NT2RI2006703, NT2RI2006716, NT2RI2006735, NT2RI2006762, NT2RI2006788, NT2RI2006838, NT2RI2006853, NT2RI2006855, NT2RI2006973, NT2RI2007046, NT2RI2007048, NT2RI2007116, NT2RI2007133, NT2RI2007214, NT2RI2007277, NT2RI2007303, NT2RI2007334, NT2RI2007386, NT2RI2007406, NT2RI2007439, NT2RI2007469, NT2RI2007507, NT2RI2007729, NT2RI2007751, NT2RI2007754, NT2RI2007827, NT2RI2007853, NT2RI2007877, NT2RI2007891, NT2RI2007956, NT2RI2007965, NT2RI2008007, NT2RI2008050, NT2RI2008204, NT2RI2008221, NT2RI2008233, NT2RI2008455, NT2RI2008502, NT2RI2008598, NT2RI2008622, NT2RI2008656, NT2RI2008801, NT2RI2008841, NT2RI2008952, NT2RI2009005, NT2RI2009101, NT2RI2009103, NT2RI2009144, NT2RI2009151, NT2RI2009215, NT2RI2009259, NT2RI2009289, NT2RI2009301, NT2RI2009517, NT2RI2009585, NT2RI2009595, NT2RI2009855, NT2RI2010283, NT2RI2010508, NT2RI2011422, NT2RI2011450, NT2RI2011683, NT2RI2011803, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018448, NT2RI2018883, NT2RI2018950, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001458, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003409, NT2RI3003738, NT2RI3003925, NT2RI3004133, NT2RI3004381, NT2RI3005202, NT2RI3005403, NT2RI3005416, NT2RI3005861, NT2RI3005923, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007443, NT2RI3007684, NT2RI3007757, NT2RI3007878, NT2RI3007978, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000033, NT2RP6000043, NT2RP6000060, NT2RP6000077, NT2RP6000100, NT2RP6000119, NT2RP6000127, NT2RP6000140, NT2RP6000150, NT2RP7000041, NT2RP7000109, NT2RP7000112, NT2RP7000259, NT2RP7000311, NT2RP7000359, NT2RP7000364, NT2RP7000380, NT2RP7000391, NT2RP7000425, NT2RP7000926, NT2RP7001074, NT2RP7001080, NT2RP7001156, NT2RP7001306, NT2RP7001319, NT2RP7001335, NT2RP7001532, NT2RP7001591, NT2RP7001602, NT2RP7001678, NT2RP7001856, NT2RP7002028, NT2RP7002064, NT2RP7002151, NT2RP7002163, NT2RP7002243, NT2RP7002282, NT2RP7002376, NT2RP7002450, NT2RP7002478, NT2RP7002554, NT2RP7002603, NT2RP7002650, NT2RP7002779, NT2RP7002841, NT2RP7002978, NT2RP7002982, NT2RP7003025, NT2RP7003050, NT2RP7003084, NT2RP7003091, NT2RP7003107, NT2RP7003134, NT2RP7003148, NT2RP7003261, NT2RP7003439, NT2RP7003629, NT2RP7003680, NT2RP7004027, NT2RP7004080, NT2RP7004204, NT2RP7004233, NT2RP7004260, NT2RP7004348, NT2RP7004358, NT2RP7004396, NT2RP7004541, NT2RP7004559, NT2RP7004641, NT2RP7004656, NT2RP7004687, NT2RP7004722, NT2RP7004766, NT2RP7004961, NT2RP7004964, NT2RP7004975, NT2RP7005118, NT2RP7005205, NT2RP7005323, NT2RP7005468, NT2RP7005629, NT2RP7005631, NT2RP7005669, NT2RP7005846, NT2RP7006033, NT2RP7006075, NT2RP7006141, NT2RP7006160, NT2RP7006296, NT2RP7006304, NT2RP7006374, NT2RP7006395, NT2RP7006408, NT2RP7006457, NT2RP7006539, NT2RP7006547, NT2RP7006601, NT2RP7006621, NT2RP7006636, NT2RP7006853, NT2RP7006886, NT2RP7006887, NT2RP7006980, NT2RP7006986, NT2RP7006995, NT2RP7007114, NT2RP7007221, NT2RP7007226, NT2RP7007269, NT2RP7007310, NT2RP7007359, NT2RP7007381, NT2RP7007387, NT2RP7007406, NT2RP7007480, NT2RP7007504, NT2RP7007580, NT2RP7007594, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008315, NT2RP7008360, NT2RP7008406, NT2RP7008435, NT2RP7008454, NT2RP7008487, NT2RP7008544, NT2RP7008550, NT2RP7008557, NT2RP7008623, NT2RP7008657, NT2RP7008720, NT2RP7008855, NT2RP7008863, NT2RP7009019, NT2RP7009030, NT2RP7009147, NT2RP7009168, NT2RP7009215, NT2RP7009225, NT2RP7009236, NT2RP7009253, NT2RP7009267, NT2RP7009322, NT2RP7009363, NT2RP7009370, NT2RP7009397, NT2RP7009429, NT2RP7009439, NT2RP7009466, NT2RP7009502, NT2RP7009507, NT2RP7009577, NT2RP7009743, NT2RP7009867, NT2RP7010128, NT2RP7010235, NT2RP7010275, NT2RP7010521, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015512, NT2RP7015676, NT2RP7015789, NT2RP7015996, NT2RP7016508, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000247, NTONG1000257, NTONG2000040, NTONG2000231, NTONG2000363, NTONG2000413, NTONG2000492, NTONG2000499, NTONG2000531, NTONG2000583, NTONG2000800, NTONG2000858, NTONG2000878, NTONG2000966, NTONG2000977, NTONG2000985, NTONG2001014, NTONG2001079, NTONG2001137, NTONG2001157, NTONG2001173, NTONG2001220, NTONG2001222, NTONG2001362, NTONG2001532, NTONG2001567, NTONG2001587, NTONG2001619, NTONG2001642, NTONG2002278, NTONG2002582, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005480, NTONG2005497, NTONG2005577, NTONG2005657, NTONG2005822, NTONG2005897, NTONG2006099, NTONG2006187, NTONG2006301, NTONG2006324, NTONG2006354, NTONG2006440, NTONG2006501, NTONG2006646, NTONG2006709, NTONG2007020, NTONG2007028, NTONG2007034, NTONG2007249, NTONG2007517, NTONG2007522, NTONG2007693, NTONG2008088, NTONG2008093, NTONG2008143, NTONG2008365, NTONG2008483, NTONG2008522, NTONG2008672, NTONG2008862, NTONG2008939, NTONG2008944, NTONG2009060, NTONG2009068, NTONG2009229, NTONG2009233, OCBBF1000016, OCBBF1000030, OCBBF1000042, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000153, OCBBF1000185, OCBBF1000192, OCBBF1000315, OCBBF2000013, OCBBF2000287, OCBBF2000452, OCBBF2000703, OCBBF2000719, OCBBF2000904, OCBBF2000998, OCBBF2001075, OCBBF2001101, OCBBF2001124, OCBBF2001176, OCBBF2001252, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001681, OCBBF2001706, OCBBF2001749, OCBBF2001910, OCBBF2001961, OCBBF2001983, OCBBF2002083, OCBBF2002124, OCBBF2002161, OCBBF2002357, OCBBF2002615, OCBBF2002663, OCBBF2002805, OCBBF2002865, OCBBF2002920, OCBBF2002980, OCBBF2003028, OCBBF2003091, OCBBF2003327, OCBBF2003543, OCBBF2003593, OCBBF2003744, OCBBF2004038, OCBBF2004147, OCBBF2004168, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433; OCBBF2005546, OCBBF2005843, OCBBF2005901, OCBBF2006005, OCBBF2006030, OCBBF2006058, OCBBF2006148, OCBBF2006154, OCBBF2006214, OCBBF2006241, OCBBF2006388, OCBBF2006438, OCBBF2006567, OCBBF2006624, OCBBF2006639, OCBBF2006660, OCBBF2006849, OCBBF2006859, OCBBF2006972, OCBBF2006987, OCBBF2007039, OCBBF2007051, OCBBF2007068, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007756, OCBBF2007892, OCBBF2007931, OCBBF2007946, OCBBF2008041, OCBBF2008116, OCBBF2008138, OCBBF2008283, OCBBF2008330, OCBBF2008366, OCBBF2008466, OCBBF2008483, OCBBF2008520, OCBBF2008569, OCBBF2008586, OCBBF2008640, OCBBF2008691, OCBBF2008724, OCBBF2008760, OCBBF2008768, OCBBF2008770, OCBBF2008790, OCBBF2008814, OCBBF2008822, OCBBF2009115, OCBBF2009391, OCBBF2009424, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010404, OCBBF2010416, OCBBF2010420, OCBBF2010604, OCBBF2010709, OCBBF2010792, OCBBF2010830, OCBBF2010841, OCBBF2010843, OCBBF2010931, OCBBF2010945, OCBBF2010978, OCBBF2010989, OCBBF2011137, OCBBF2011160, OCBBF2011177, OCBBF2011228, OCBBF2011283, OCBBF2011311, OCBBF2011625, OCBBF2011685, OCBBF2011706, OCBBF2011722, OCBBF2011767, OCBBF2011855, OCBBF2011872, OCBBF2011960, OCBBF2011981, OCBBF2012001, OCBBF2012028, OCBBF2012095, OCBBF2012139, OCBBF2012191, OCBBF2012262, OCBBF2012320, OCBBF2012436, OCBBF2012755, OCBBF2012936, OCBBF2012990, OCBBF2013049, OCBBF2013091, OCBBF2013123, OCBBF2013127, OCBBF2013208, OCBBF2013319, OCBBF2013507, OCBBF2013721, OCBBF2013789, OCBBF2013883, OCBBF2013926, OCBBF2014052, OCBBF2014322, OCBBF2014386, OCBBF2014541, OCBBF2014707, OCBBF2014828, OCBBF2014873, OCBBF2014880, OCBBF2014928, OCBBF2015031, OCBBF2015081, OCBBF2015232, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016690, OCBBF2016841, OCBBF2016928, OCBBF2017069, OCBBF2017306, OCBBF2017325, OCBBF2017489, OCBBF2017516, OCBBF2017665, OCBBF2017815, OCBBF2017888, OCBBF2017920, OCBBF2018014, OCBBF2018167, OCBBF2018206, OCBBF2018229, OCBBF2018362, OCBBF2018380, OCBBF2018563, OCBBF2018663, OCBBF2018687, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020453, OCBBF2020801, OCBBF2020825, OCBBF2020838, OCBBF2021020, OCBBF2021214, OCBBF2021323, OCBBF2021518, OCBBF2021788, OCBBF2021833, OCBBF2022573, OCBBF2022574, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025631, OCBBF2025730, OCBBF2026025, OCBBF2026035, OCBBF2026144, OCBBF2026645, OCBBF2026690, OCBBF2026981, OCBBF2027148, OCBBF2027354, OCBBF2027423, OCBBF2027478, OCBBF2027481, OCBBF2027511, OCBBF2027728, OCBBF2027782, OCBBF2027868, OCBBF2028055, OCBBF2028173, OCBBF2028421, OCBBF2029471, OCBBF2029901, OCBBF2030116, OCBBF2030354, OCBBF2030517, OCBBF2030574, OCBBF2030708, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009279, PANCR1000012, PANCR1000021, PANCR1000081, PANCR1000086, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000170, PEBLM2000180, PEBLM2000213, PEBLM2000248, PEBLM2000270, PEBLM2000308, PEBLM2000321, PEBLM2000333, PEBLM2000338, PEBLM2000395, PEBLM2000479, PEBLM2000502, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2004733, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000630, PLACE6000953, PLACE6001118, PLACE6001153, PLACE6001294, PLACE6001536, PLACE6001557, PLACE6001712, PLACE6001740, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002462, PLACE6002647, PLACE6002668, PLACE6002699, PLACE6002710, PLACE6002871, PLACE6002949, PLACE6002960, PLACE6003004, PLACE6003077, PLACE6003094, PLACE6003109, PLACE6003204, PLACE6003218, PLACE6003372, PLACE6003383, PLACE6003393, PLACE6003399, PLACE6003539, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005786, PLACE6006042, PLACE6006077, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007239, PLACE6007242, PLACE6007309, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848, PLACE6010925, PLACE6010991, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013883, PLACE6013884, PLACE6013885, PLACE6014043, PLACE6014516, PLACE6014882, PLACE6015211, PLACE6015322, PLACE6015460, PLACE6015513, PLACE6015591, PLACE6015731, PLACE6015939, PLACE6016030, PLACE6016093, PLACE6016105, PLACE6016160, PLACE6016210, PLACE6016254, PLACE6016383, PLACE6016984, PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008431, PLACE7008623, PLACE7008766, PLACE7009563, PROST1000033, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000217, PROST1000246, PROST1000262, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512,. PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000203, PROST2000206, PROST2000212, PROST2000241, PROST2000267, PROST2000273, PROST2000274, PROST2000277, PROST2000325, PROST2000337, PROST2000432, PROST2000452, PROST2000463, PROST2000470, PROST2000505, PROST2000572, PROST2000717, PROST2000760, PROST2000835, PROST2000893, PROST2000961, PROST2001116, PROST2001138, PROST2001213, PROST2001414, PROST2001415, PROST2001465, PROST2001540, PROST2001552, PROST2001599, PROST2001611, PROST2001636, PROST2001739, PROST2001796, PROST2001805, PROST2001823, PROST2001899, PROST2001998, PROST2002078, PROST2002101, PROST2002212, PROST2002338, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004727, PROST2004742, PROST2004817, PROST2005039, PROST2005076, PROST2005121, PROST2005131, PROST2005228, PROST2005272, PROST2005426, PROST2005630, PROST2005788, PROST2005880, PROST2005886, PROST2005904, PROST2005919, PROST2005943, PROST2006008, PROST2006020, PROST2006196, PROST2006201, PROST2006282, PROST2006343, PROST2006450, PROST2006491, PROST2006510, PROST2006536, PROST2006579, PROST2006688, PROST2006766, PROST2006782, PROST2006933, PROST2007122, PROST2007200, PROST2007237, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010400, PROST2010545, PROST2010579, PROST2010606, PROST2010782, PROST2010885, PROST2011012, PROST2011038, PROST2011297, PROST2011439, PROST2011482, PROST2011631, PROST2012005, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012780, PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014916, PROST2014925, PROST2015124, PROST2015137, PROST2015198, PROST2015243, PROST2015246, PROST2015251, PROST2015287, PROST2015457, PROST2015464, PROST2015537, PROST2015545, PROST2015551, PROST2015636, PROST2015710, PROST2015756, PROST2015877, PROST2015924, PROST2015932, PROST2016195, PROST2016246, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017367, PROST2017413, PROST2017441, PROST2017578, PROST2017612, PROST2017617, PROST2017700, PROST2017701, PROST2017729, PROST2017836, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019398, PROST2019487, PROST2019781, PUAEN1000039, PUAEN1000081, PUAEN1000085, PUAEN1000087, PUAEN1000121, PUAEN1000161, PUAEN1000175, PUAEN1000239, PUAEN1000275, PUAEN1000276, PUAEN1000288, PUAEN2000080, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001882, PUAEN2002473, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2008123, PUAEN2008228, PUAEN2008314, PUAEN2008815, PUAEN2008939, PUAEN2008980, PUAEN2008985, PUAEN2009348, PUAEN2009534, PUAEN2009655, PUAEN2009795, PUAEN2009852, PUAEN2010346, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000107, SALGL1000171, SKMUS1000014, SKMUS1000030, SKMUS1000064, SKMUS1000067, SKMUS1000102, SKMUS1000104, SKMUS1000118, SKMUS1000124, SKMUS1000129, SKMUS1000138, SKMUS1000176, SKMUS2000061, SKMUS2000078, SKMUS2000137, SKMUS2000198, SKMUS2000287, SKMUS2000317, SKMUS2000330, SKMUS2000339, SKMUS2000365, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000933, SKMUS2000945, SKMUS2000961, SKMUS2000973, SKMUS2000980, SKMUS2001014, SKMUS2001147, SKMUS2001157, SKMUS2001164, SKMUS2001199, SKMUS2001215, SKMUS2001247, SKMUS2001271, SKMUS2001287, SKMUS2001323, SKMUS2001364, SKMUS2001398, SKMUS2001492, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000229, SKNMC2000256, SKNMC2000356, SKNMC2000374, SKNMC2000583, SKNMC2000593, SKNMC2000612, SKNMC2000622, SKNMC2001324, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000516, SKNSH2000550, SKNSH2000716, SKNSH2000971, SKNSH2001222, SKNSH2001875, SKNSH2001931, SKNSH2002054, SKNSH2002866, SKNSH2003174, SKNSH2003422, SKNSH2003466, SKNSH2003483, SKNSH2004039, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000103, SMINT1000118, SMINT1000137, SMINT2000007, SMINT2000032, SMINT2000040, SMINT2000060, SMINT2000084, SMINT2000145, SMINT2000159, SMINT2000176, SMINT2000185, SMINT2000227, SMINT2000229, SMINT2000239, SMINT2000267, SMINT2000396, SMINT2000400, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000747, SMINT2000811, SMINT2000824, SMINT2000984, SMINT2001114, SMINT2001158, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002202, SMINT2002210, SMINT2002224, SMINT2002281, SMINT2002311, SMINT2002314, SMINT2002328, SMINT2002414, SMINT2002457, SMINT2002620, SMINT2002743, SMINT2002778, SMINT2002880, SMINT2002882, SMINT2002884, SMINT2002899, SMINT2002974, SMINT2002976, SMINT2003074, SMINT2003128, SMINT2003317, SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004339, SMINT2004473, SMINT2004547, SMINT2004729, SMINT2004781, SMINT2004891, SMINT2004909, SMINT2004992, SMINT2005075, SMINT2005161, SMINT2005174, SMINT2005213, SMINT2005330, SMINT2005387, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007790, SMINT2007796, SMINT2007867, SMINT2007932, SMINT2008042, SMINT2008054, SMINT2008277, SMINT2008329, SMINT2008455, SMINT2008491, SMINT2008531, SMINT2008545, SMINT2008558, SMINT2008590, SMINT2008625, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010426, SMINT2010500, SMINT2010533, SMINT2010629, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011888, SMINT2011958, SMINT2012040, SMINT2012122, SMINT2012179, SMINT2012285, SMINT2012734, SMINT2012793, SMINT2013031, SMINT2013129, SMINT2013170, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064, SPLEN2000072, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000350, SPLEN2000357, SPLEN2000364, SPLEN2000402, SPLEN2000414, SPLEN2000420, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2000882, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001689, SPLEN2001710, SPLEN2001781, SPLEN2001881, SPLEN2001912, SPLEN2001945, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002477, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004346, SPLEN2004368, SPLEN2004611, SPLEN2004880, SPLEN2004984, SPLEN2005009, SPLEN2005227, SPLEN2005272, SPLEN2005342, SPLEN2005416, SPLEN2005450, SPLEN2005474, SPLEN2005560, SPLEN2005727, SPLEN2005790, SPLEN2005818, SPLEN2005838, SPLEN2005869, SPLEN2005927, SPLEN2005939, SPLEN2006122, SPLEN2006232, SPLEN2006268, SPLEN2006283, SPLEN2006325, SPLEN2006389, SPLEN2006425, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008591, SPLEN2008786, SPLEN2009081, SPLEN2009088, SPLEN2009163, SPLEN2009315, SPLEN2009340, SPLEN2009512, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010862, SPLEN2010978, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012523, SPLEN2012524, SPLEN2012525, SPLEN2012606, SPLEN2012611, SPLEN2012619, SPLEN2012725, SPLEN2012743, SPLEN2012756, SPLEN2012800, SPLEN2012846, SPLEN2012889, SPLEN2012961, SPLEN2013108, SPLEN2013503, SPLEN2013644, SPLEN2013648, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016781, SPLEN2016840, SPLEN2016863, SPLEN2016922, SPLEN2016932, SPLEN2016973, SPLEN2017043, SPLEN2017104, SPLEN2017147, SPLEN2017187, SPLEN2017189, SPLEN2017212, SPLEN2017426, SPLEN2017452, SPLEN2017538, SPLEN2017613, SPLEN2017620, SPLEN2017643, SPLEN2017687, SPLEN2017740, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019311, SPLEN2019323, SPLEN2019349, SPLEN2019375, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019985, SPLEN2020034, SPLEN2020153, SPLEN2020154, SPLEN2020166, SPLEN2020183, SPLEN2020223, SPLEN2020359, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030847, SPLEN2031004, SPLEN2031125, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037319, SPLEN2037444, SPLEN2037580, SPLEN2037630, SPLEN2037678, SPLEN2037722, SPLEN2037891, SPLEN2038055, SPLEN2038180, SPLEN2038407, SPLEN2038473, SPLEN2038834, SPLEN2039311, SPLEN2039672, SPLEN2039697, SPLEN2039845, SPLEN2039936, SPLEN2040222, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000383, STOMA2000386, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000824, SYNOV2000923, SYNOV2001014, SYNOV2001035, SYNOV2001088, SYNOV2001111, SYNOV2001212, SYNOV2001251, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008336, SYNOV4009129, SYNOV4009139, SYNOV4009298, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000613, TCERX2000998, TCOLN1000037, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000085, TESTI1000088, TESTI1000093, TESTI1000094, TESTI1000101, TESTI1000109, TESTI1000125, TESTI1000127, TESTI1000128, TESTI1000131, TESTI1000138, TESTI1000142, TESTI1000157, TESTI1000163, TESTI1000169, TESTI100017.9, TESTI1000183, TESTI1000191, TESTI1000257, TESTI1000266, TESTI1000319, TESTI1000348, TESTI1000390, TESTI1000391, TESTI1000459, TESTI1000491, TESTI1000545, TESTI2000006, TESTI2000018, TESTI2000044, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000951, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879, TESTI2001894, TESTI2001898, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003130, TESTI2003131, TESTI2003139, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003545, TESTI2003551, TESTI2003574, TESTI2003579, TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004085, TESTI2004089, TESTI2004111, TESTI2004119, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005775, TESTI2005827, TESTI2005835, TESTI2005860, TESTI2005892, TESTI2005911, TESTI2005933, TESTI2005937, TESTI2005948, TESTI2005979, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006109, TESTI2006125, TESTI2006179, TESTI2006209, TESTI2006212, TESTI2006218, TESTI2006238, TESTI2006255, TESTI2006258, TESTI2006278, TESTI2006333, TESTI2006360, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007183, TESTI2007220, TESTI2007234, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009325, TESTI2009390, TESTI2009400, TESTI2009412, TESTI2009423, TESTI2009447, TESTI2009462, TESTI2009477, TESTI2009497, TESTI2009502, TESTI2009511, TESTI2009544, TESTI2009545, TESTI2009551, TESTI2009577, TESTI2009585, TESTI2009588, TESTI2009727, TESTI2009739, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011575, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155, TESTI2012171, TESTI2012207, TESTI2012231, TESTI2012244, TESTI2012308, TESTI2012356, TESTI2012387, TESTI2012394, TESTI2012444, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014843, TESTI2014979, TESTI2014988, TESTI2015042, TESTI2015105, TESTI2015180, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016599, TESTI2016629, TESTI2016663, TESTI2016667, TESTI2016688, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017069, TESTI2017089, TESTI2017102, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020871, TESTI2020905, TESTI2020906, TESTI2020946, TESTI2020956, TESTI2020981, TESTI2020999, TESTI2021003, TESTI2021057, TESTI2021077, TESTI2021112, TESTI2021114, TESTI2021116, TESTI2021118, TESTI2021122, TESTI2021138, TESTI2021297, TESTI2021315, TESTI2021358, TESTI2021447, TESTI2021463, TESTI2021599, TESTI2021637, TESTI2021667, TESTI2021736, TESTI2021785, TESTI2021819, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026674, TESTI2026794, TESTI2026824, TESTI2026854, TESTI2026925, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035078, TESTI2035084, TESTI2035107, TESTI2035121, TESTI2035124, TESTI2035183, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001569, TESTI4001604, TESTI4001665, TESTI4001679, TESTI4001795, TESTI4001923, TESTI4001925, TESTI4001984, TESTI4002003, TESTI4002141, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4003179, TESTI4003279, TESTI4003319, TESTI4003404, TESTI4003565, TESTI4003574, TESTI4003579, TESTI4003733, TESTI4003796, TESTI4003944, TESTI4004003, TESTI4004031, TESTI4004200, TESTI4004210, TESTI4004539, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013685, TESTI4013735, TESTI4013742, TESTI4013774, TESTI4013830, TESTI4013852, TESTI4013924, TESTI4013960, TESTI4013962, TESTI401.4175, TESTI4014265, TESTI4014276, TESTI4014392, TESTI4014415, TESTI4014445, TESTI4014661, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037228, TESTI4037244, TESTI4037727, TESTI4037949, TESTI4038047, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000033, THYMU1000060, THYMU1000074, THYMU1000098, THYMU1000105, THYMU1000136, THYMU1000142, THYMU1000158, THYMU1000176, THYMU1000179, THYMU1000191, THYMU1000217, THYMU1000240, THYMU1000242, THYMU1000291, THYMU1000316, THYMU1000318, THYMU1000329, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002852, THYMU2002910, THYMU2002950, THYMU2003069, THYMU2003107, THYMU2003124, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004169, THYMU2004320, THYMU2004336, THYMU2004356, THYMU2004452, THYMU2004512, THYMU2004635, THYMU2004639, THYMU2004668, THYMU2004677, THYMU2004688, THYMU2004693, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005546, THYMU2005576, THYMU2005748, THYMU2005759, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095, THYMU2007112, THYMU2007146, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007561, THYMU2007571, THYMU2007635, THYMU2007658, THYMU2007667, THYMU2007725, THYMU2007802, THYMU2007824, THYMU2007854, THYMU2007952, THYMU2007969, THYMU2008035, THYMU2008049, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009596, THYMU2009792, THYMU2009835, THYMU2009906, THYMU2009948, THYMU2010030, THYMU2010046, THYMU2010059, THYMU2010082, THYMU2010094, THYMU2010161, THYMU2010192, THYMU2010519, THYMU2010637, THYMU2010671, THYMU2010699, THYMU2010705, THYMU2010831, THYMU2010848, THYMU2011053, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012701, THYMU2012807, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014327, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017831, THYMU2017844, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023576, THYMU2023711, THYMU2023900, THYMU2023943, THYMU2024121, THYMU2024185, THYMU2024207, THYMU2024616, THYMU2024684, THYMU2024748, THYMU2025042, THYMU2025189, THYMU2025319, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629,. THYMU2028632, THYMU2028724, THYMU2028739, THYMU2028942, THYMU2028978, THYMU2029134, THYMU2029676, THYMU2029688, THYMU2029788, THYMU2030068, THYMU2030226, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037233, THYMU2037234, THYMU2037348, THYMU2037406, THYMU2037965, THYMU2038058, THYMU2038189, THYMU2038199, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039788, THYMU2039989, THYMU2040029, THYMU2040110, THYMU2040114, THYMU2040140, THYMU2040412, THYMU2040824, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000390, THYMU3000490, THYMU3000504, THYMU3000542, THYMU3000655, THYMU3000826, THYMU3000841, THYMU3001077, THYMU3001082, THYMU3001133, THYMU3001151, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003350, THYMU3003403, THYMU3003565, THYMU3003763, THYMU3003865, THYMU3003958, THYMU3003965, THYMU3004157, THYMU3004628, THYMU3004632, THYMU3004866, THYMU3005050, THYMU3005378, THYMU3005407, THYMU3005415, THYMU3005427, THYMU3005482, THYMU3005491, THYMU3005696, THYMU3006118, THYMU3006132, THYMU3006168, THYMU3006172, THYMU3006371, THYMU3006640, THYMU3006767, THYMU3006811, THYMU3006963, THYMU3007137, THYMU3007368, THYMU3007559, THYMU3007845, THYMU3008060, THYMU3008136, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046360, THYMU3046856, THYMU3047115, THYMU3047144, THYMU3047156, THYMU3047366, THYMU3047513, THYMU3047542, THYMU3047760, THYMU3047891, TKIDN1000044, TKIDN1000062, TKIDN1000066, TKIDN1000171, TKIDN2000127, TKIDN2000240, TKIDN2000319, TKIDN2000464, TKIDN2000521, TKIDN2000701, TKIDN2001529, TKIDN2001662, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2004185, TKIDN2004386, TKIDN2004458, TKIDN2004748, TKIDN2005721, TKIDN2005934, TKIDN2005947, TKIDN2005956, TKIDN2006525, TKIDN2006852, TKIDN2007667, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015161, TKIDN2015263, TKIDN2016309, TKIDN2016399, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000038, TRACH1000057, TRACH1000063, TRACH1000074, TRACH1000100, TRACH1000106, TRACH1000125, TRACH1000140, TRACH1000144, TRACH1000212, TRACH1000240, TRACH2000002, TRACH2000015, TRACH2000024, TRACH2000032, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000411, TRACH2000496, TRACH2000497, TRACH2000502, TRACH2000522, TRACH2000540, TRACH2000559, TRACH2000665, TRACH2000675, TRACH2000703, TRACH2000767, TRACH2000775, TRACH2000807, TRACH2000862, TRACH2000894, TRACH2000898, TRACH2000923, TRACH2000944, TRACH2000981, TRACH2000986, TRACH2001037, TRACH2001060, TRACH2001085, TRACH2001101, TRACH2001121, TRACH2001147, TRACH2001154, TRACH2001188, TRACH2001192, TRACH2001275, TRACH2001335, TRACH2001339, TRACH2001347, TRACH2001395, TRACH2001400, TRACH2001403, TRACH2001416, TRACH2001428, TRACH2001443, TRACH2001463, TRACH2001474, TRACH2001525, TRACH2001532, TRACH2001592, TRACH2001596, TRACH2001621, TRACH2001651, TRACH2001898, TRACH2001933, TRACH2001968, TRACH2001996, TRACH2002043, TRACH2002054, TRACH2002092, TRACH2002100, TRACH2002138, TRACH2002333, TRACH2002784, TRACH2002840, TRACH2002847, TRACH2002853, TRACH2002862, TRACH2002954, TRACH2002960, TRACH2002988, TRACH2003070, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004428, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008127, TRACH2008130, TRACH2008278, TRACH2008478, TRACH2008540, TRACH2008583, TRACH2008597, TRACH2008820, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010883, TRACH2010965, TRACH2010974, TRACH2011057, TRACH2011093, TRACH2011290, TRACH2011293, TRACH2011302, TRACH2011500, TRACH2011574, TRACH2011956, TRACH2012000, TRACH2012138, TRACH2012242, TRACH2012298, TRACH2012370, TRACH2012387, TRACH2012497, TRACH2012536, TRACH2012562, TRACH2012742, TRACH2012823, TRACH2012918, TRACH2013081, TRACH2013123, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025379, TRACH2025507, TRACH2025569, TRACH2025705, TRACH2025749, TRACH2025753, TRACH2025810, TRACH2025853, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006149, TRACH3006228, TRACH3006264, TRACH3006336, TRACH3006379, TRACH3006392, TRACH3006395, TRACH3006397, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689, TRACH3007766, TRACH3007866, TRACH3008042, TRACH3008061, TRACH3008201, TRACH3008508, TRACH3008535, TRACH3008629, TRACH3008688, TRACH3008697, TRACH3008713, TRACH3008887, TRACH3008902, TRACH3008990, TRACH3009008, TRACH3009059, TRACH3009061, TRACH3009148, TRACH3009455, TRACH3009701, TRACH3009715, TRACH3009894, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036997, TRACH3037063, TRACH3037267, TRACH3037288, TRACH3037505, TRACH3037573, TRACH3037696, TRACH3037897, TRACH3038086, TSTOM1000135, TSTOM1000186, TSTOM2000139, TSTOM2000235, TSTOM2000315, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002356, TUTER2002729, UMVEN1000143, UMVEN1000156, UMVEN1000186, UMVEN2000046, UMVEN2000069, UMVEN2000121, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000047, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002294, UTERU2002332, UTERU2002473, UTERU2002547, UTERU2002693, UTERU2002733, UTERU2002736, UTERU2002737, UTERU2002841, UTERU2002964, UTERU2002993, UTERU2003035, UTERU2003126, UTERU2003135, UTERU2003321, UTERU2003399, UTERU2003411, UTERU2003577, UTERU2003926, UTERU2003973, UTERU2004015, UTERU2004037, UTERU2004039, UTERU2004061, UTERU2004073, UTERU2004163, UTERU2004299, UTERU2004461, UTERU2004520, UTERU2004564, UTERU2004688, UTERU2004698, UTERU2004807, UTERU2005004, UTERU2005050, UTERU2005069, UTERU2005074, UTERU2005179, UTERU2005292, UTERU2005346, UTERU2005450, UTERU2005533, UTERU2005601, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009414, UTERU2009483, UTERU2009538, UTERU2009540, UTERU2009776, UTERU2009904, UTERU2009951, UTERU2009972, UTERU2010115, UTERU2010124, UTERU2010164, UTERU2010231, UTERU2010304, UTERU2010320, UTERU2010431, UTERU2010525, UTERU2010651, UTERU2010724, UTERU2011195, UTERU2011199, UTERU2011261, UTERU2011287, UTERU2011410, UTERU2011574, UTERU2011657, UTERU2011741, UTERU2011806, UTERU2011811, UTERU2011897, UTERU2011962, UTERU2011968, UTERU2012031, UTERU2012101, UTERU2012114, UTERU2012252, UTERU2012333, UTERU2012581, UTERU2012610, UTERU2012615, UTERU2012688, UTERU2012703, UTERU2012715, UTERU2012741, UTERU2012767, UTERU2012786, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014223, UTERU2014398, UTERU2014464, UTERU2014548, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017762, UTERU2017810, UTERU2017988, UTERU2018127, UTERU2018180, UTERU2018333, UTERU2018364, UTERU2018514, UTERU2018522, UTERU2018523, UTERU2018544, UTERU2018609, UTERU2018674, UTERU2018784, UTERU2018789, UTERU2018811, UTERU2018867, UTERU2018881, UTERU2018955, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000402, UTERU3000645, UTERU3000665, UTERU3000738, UTERU3000828, UTERU3000844, UTERU3000899, UTERU3000959, UTERU3001029, UTERU3001053, UTERU3001059, UTERU3001240, UTERU3001394, UTERU3001542, UTERU3001558, UTERU3001571, UTERU3001585, UTERU3001632, UTERU3001652, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005264, UTERU3005422, UTERU3005585, UTERU3005907, UTERU3006008, UTERU3006228, UTERU3006538, UTERU3006640, UTERU3006687, UTERU3006720, UTERU3006798, UTERU3006884, UTERU3007021, UTERU3007104, UTERU3007108, UTERU3007134, UTERU3007640, UTERU3007731, UTERU3008280, UTERU3008463, UTERU3008660, UTERU3008671, UTERU3008722, UTERU3008917, UTERU3009259, UTERU3009490, UTERU3009690, UTERU3009775, UTERU3009871, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078
[0413]

Of the 17,176 genes that include the cDNAs of the invention, genes whose expression levels were high en detect in each of the BRAWH, BRCAN, BRCOC, BRHIP, BRSSN, and BRTHA libraries were analyzed for expression frequency. The analysis results are described below. Each is a relative where the expression level over all tissues is taken The larger this value, the greater the level of expression.

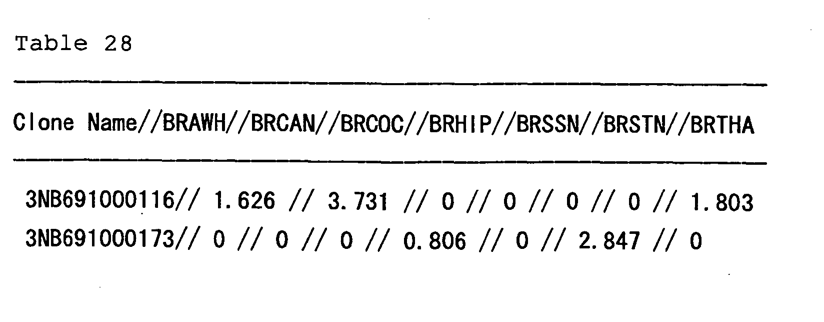

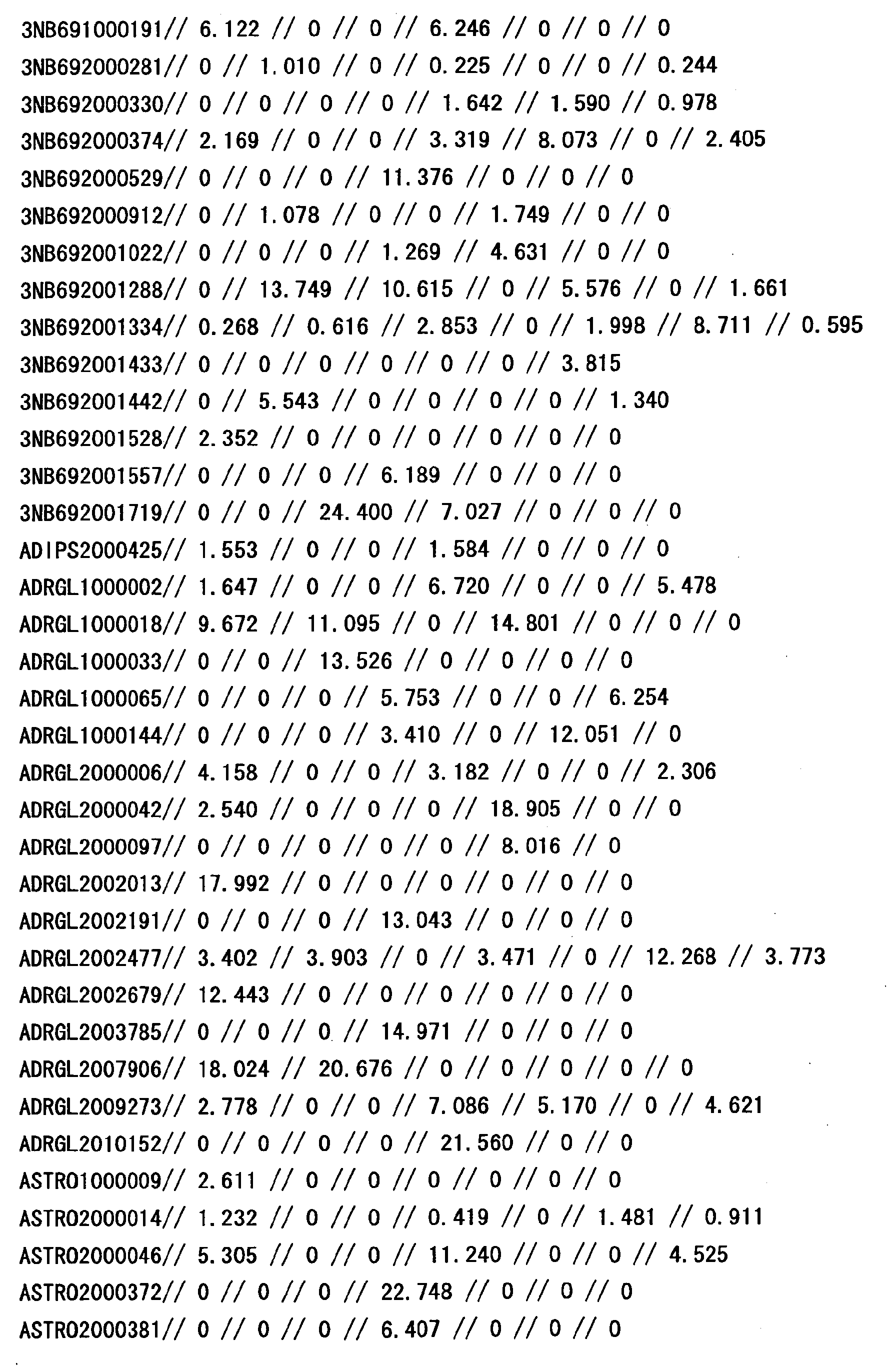

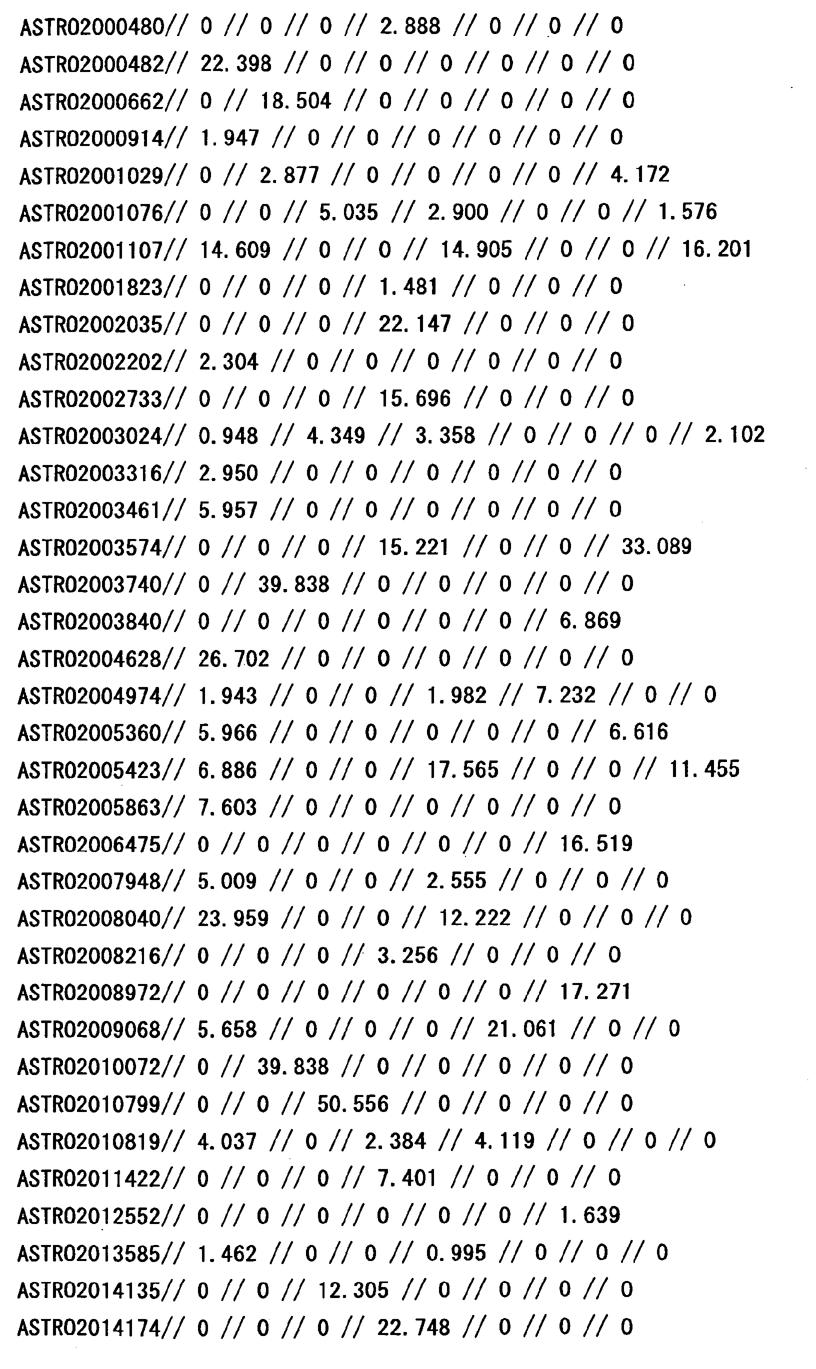

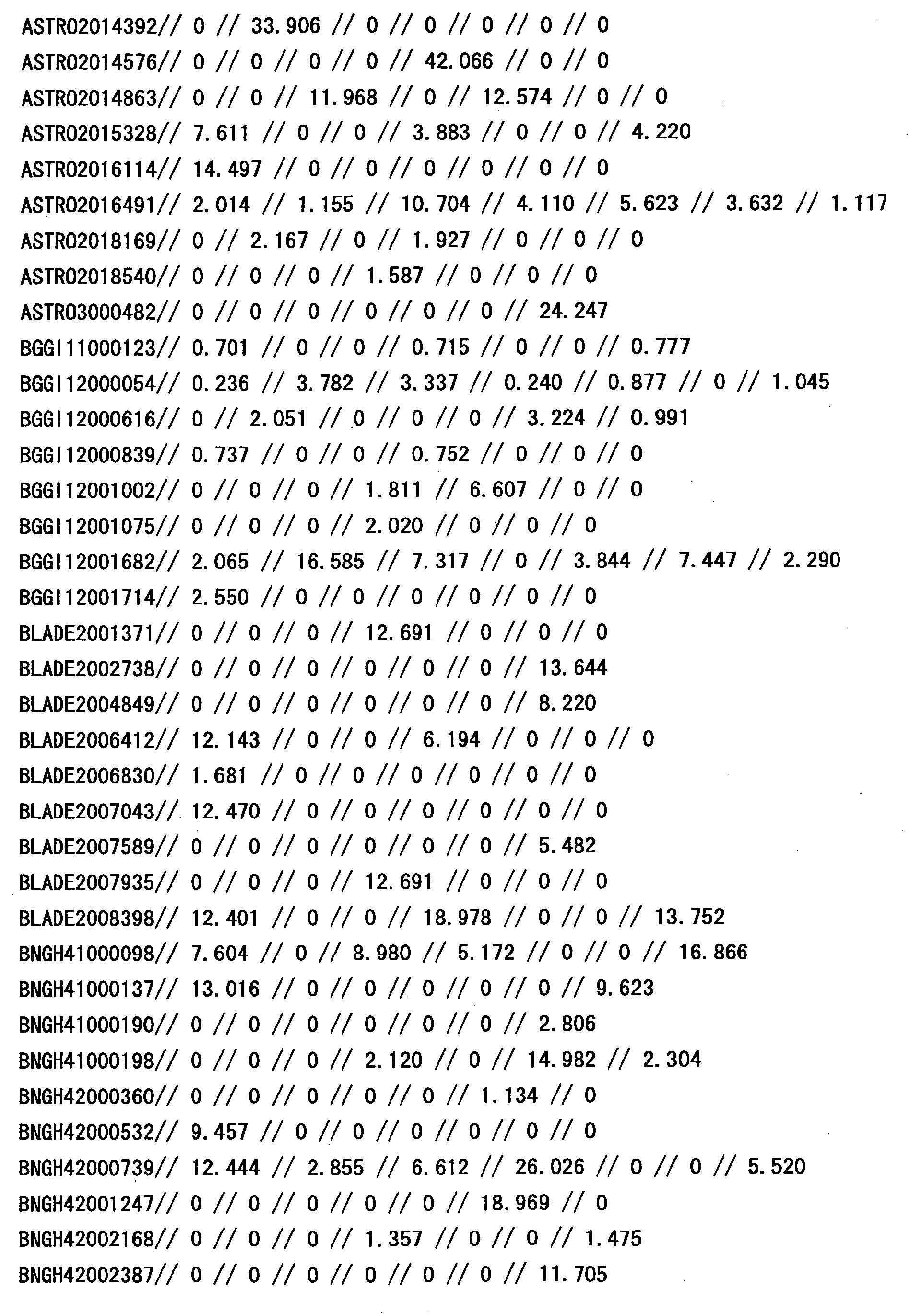

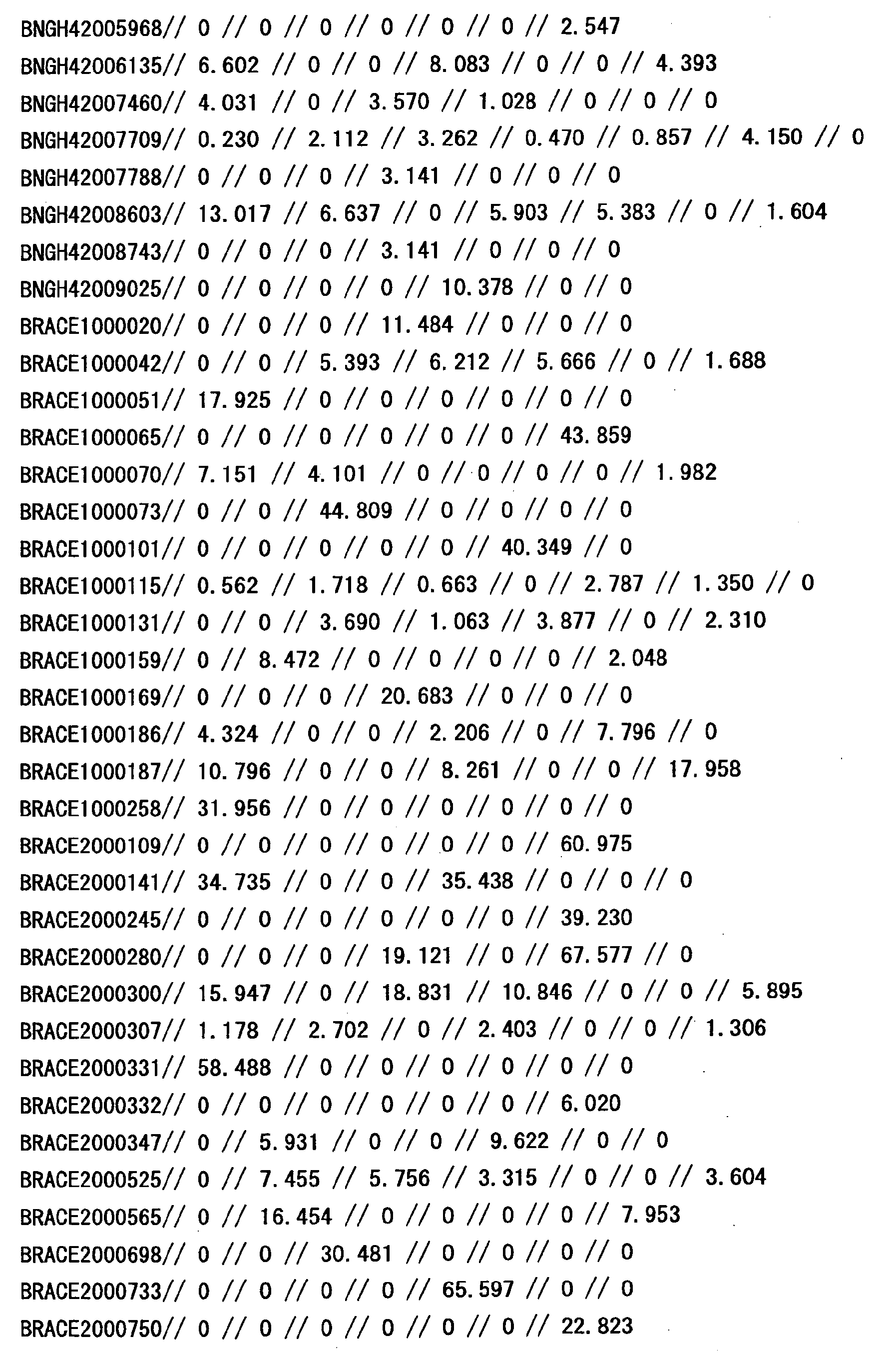

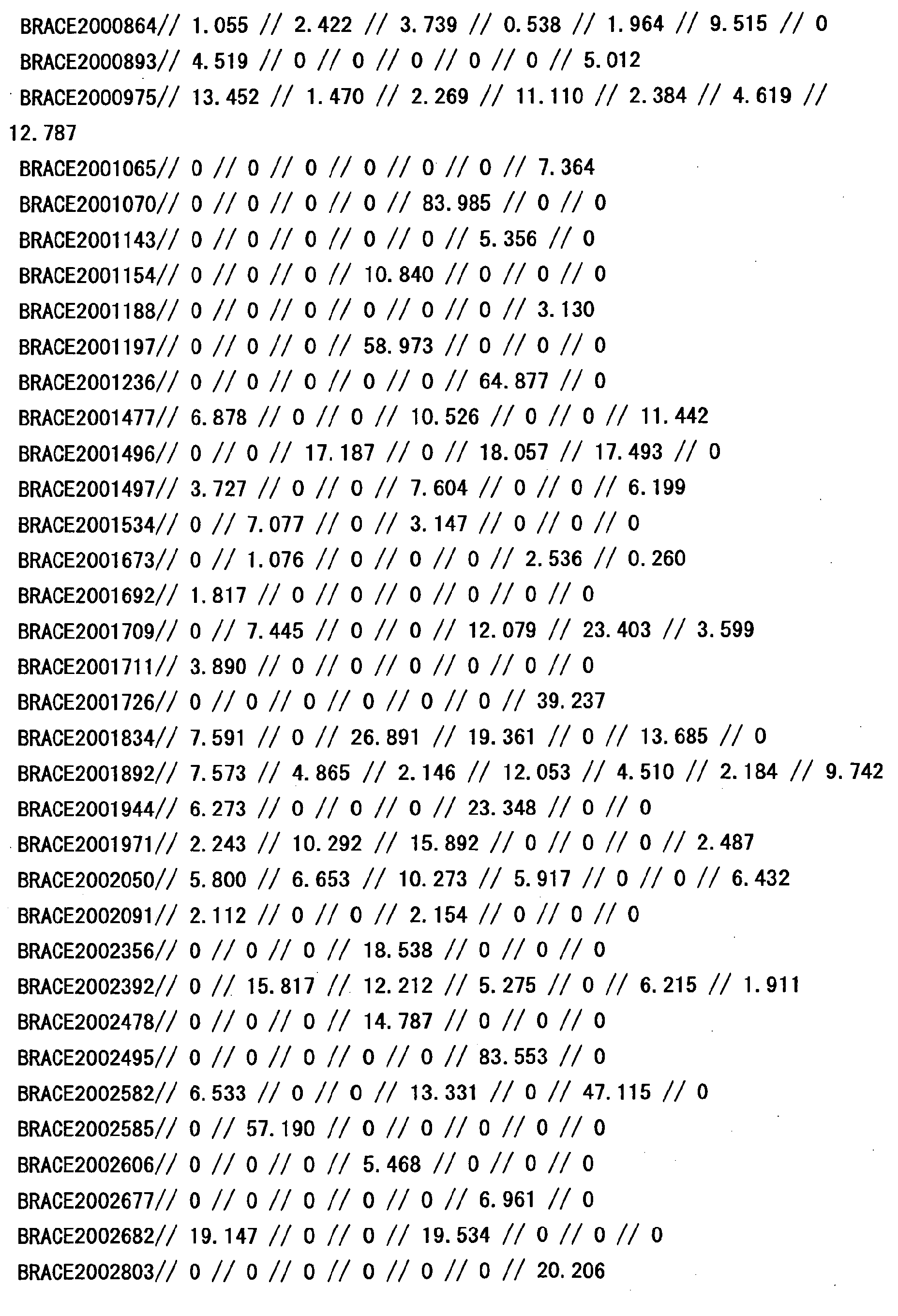

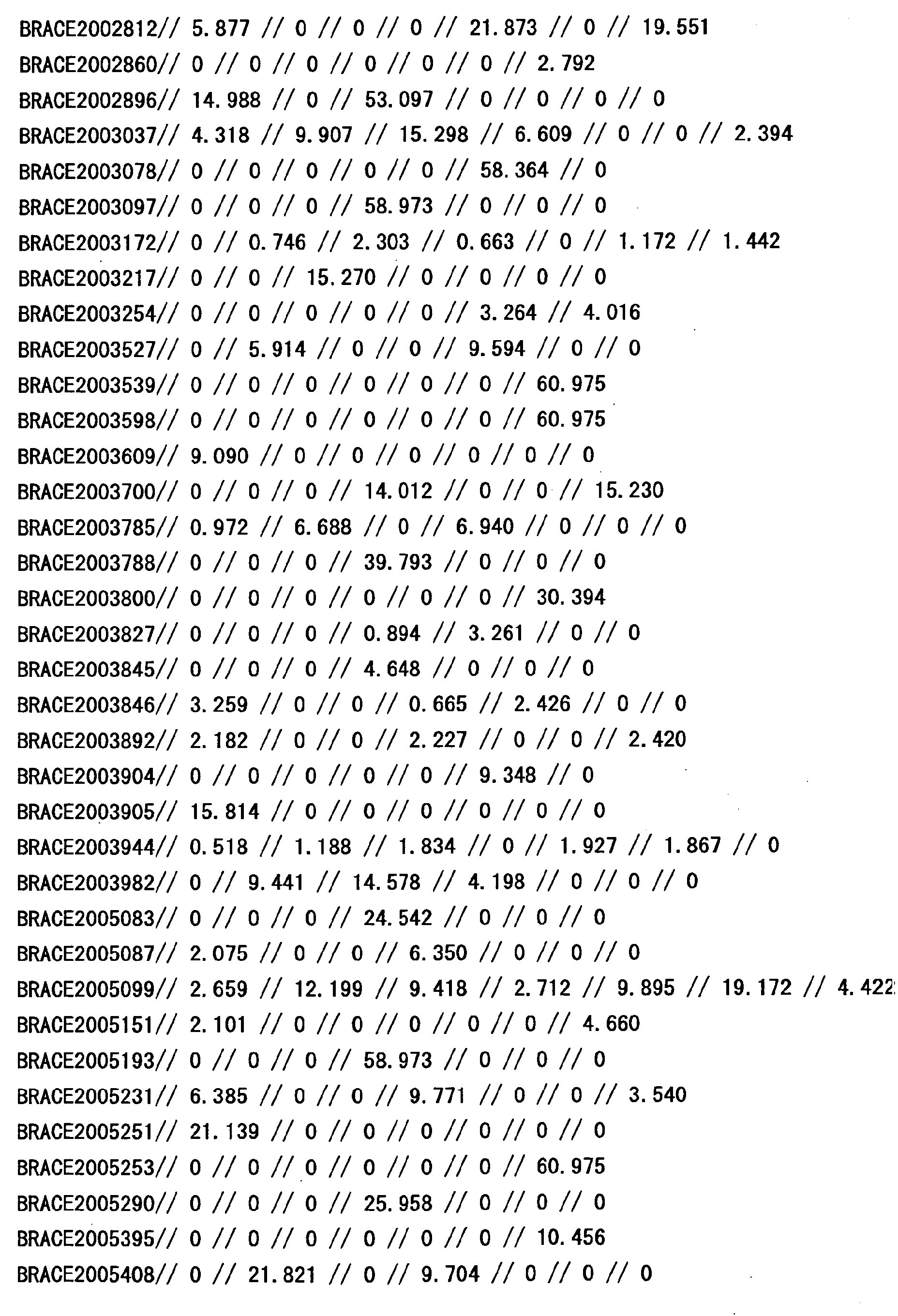

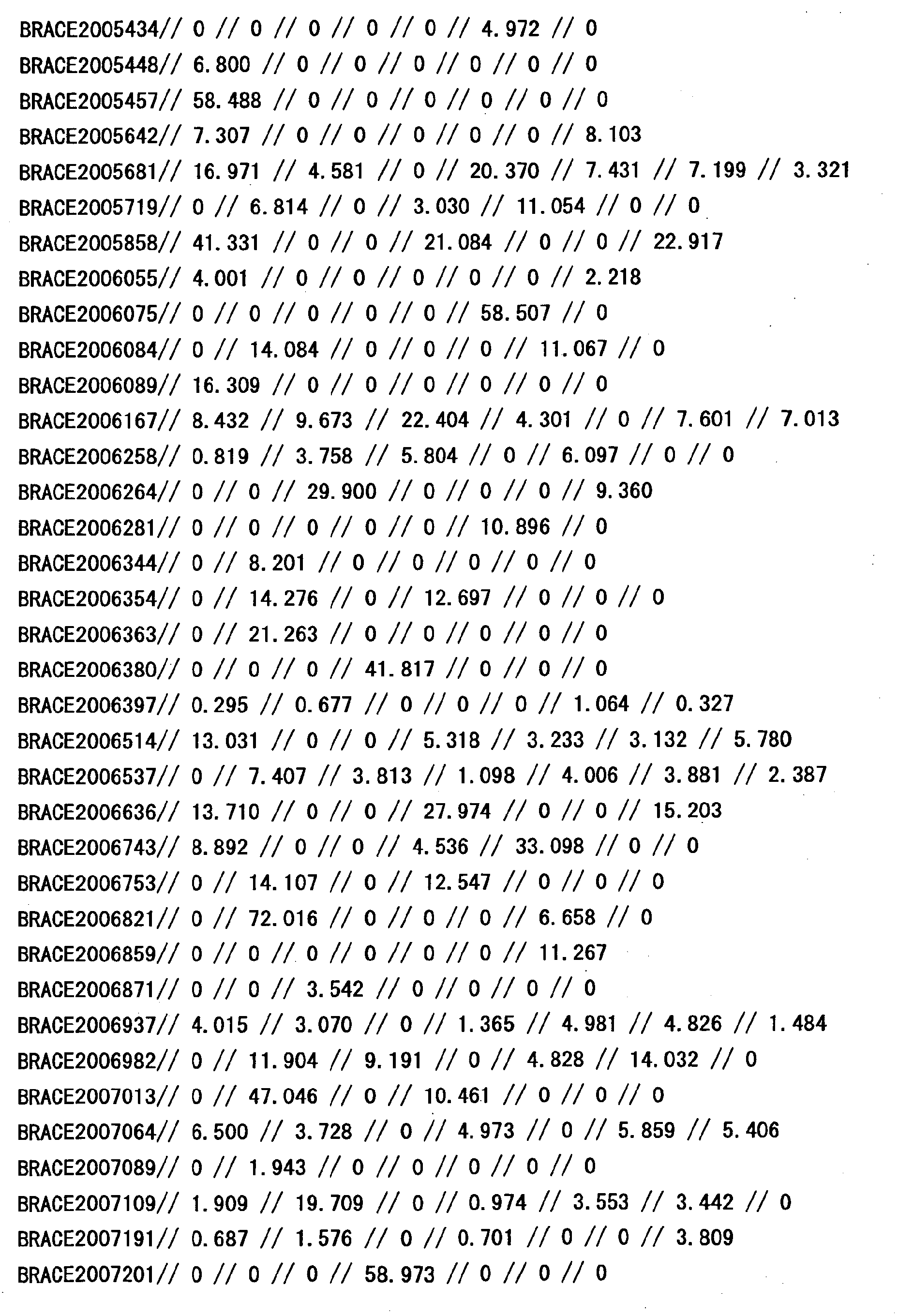

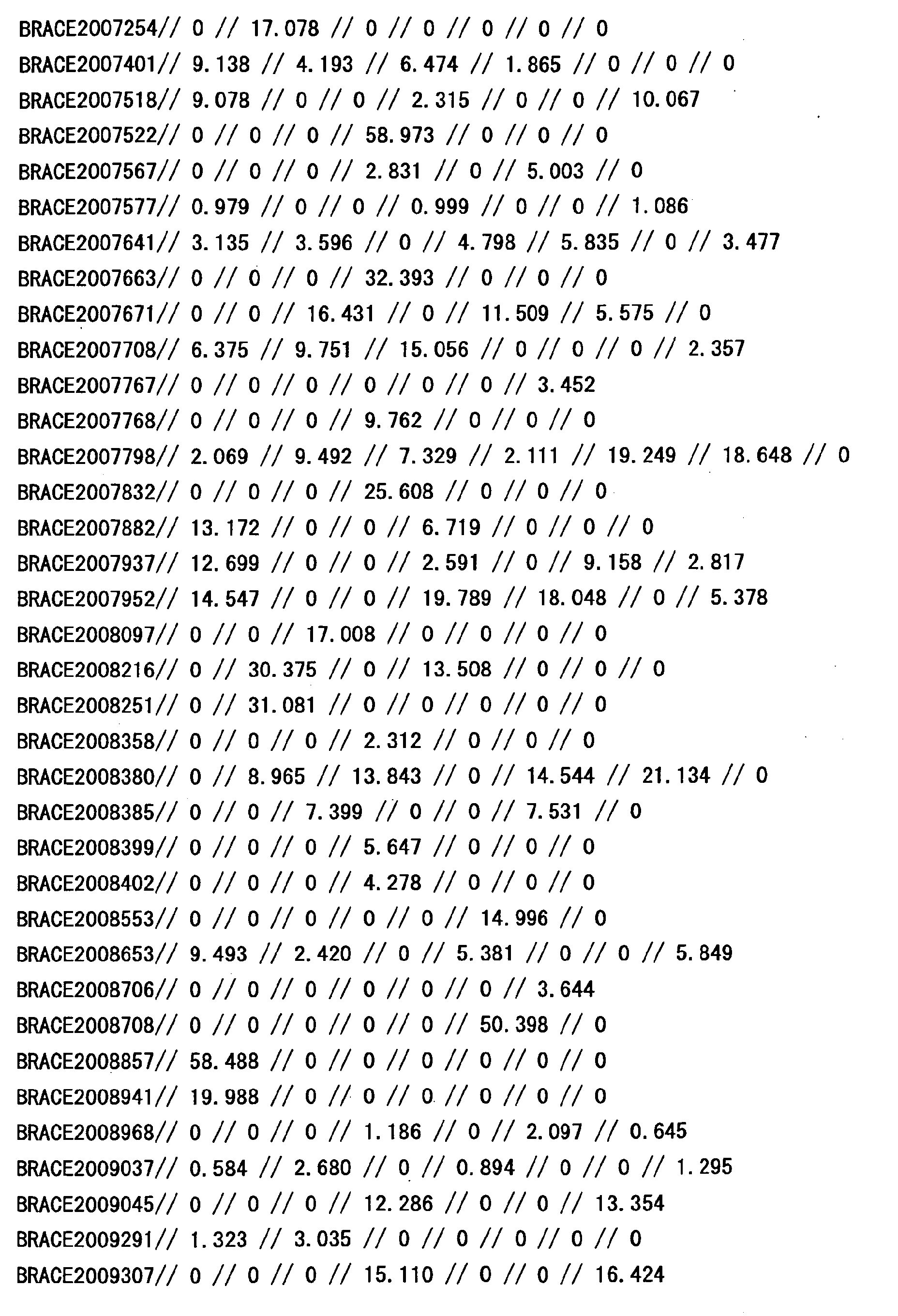

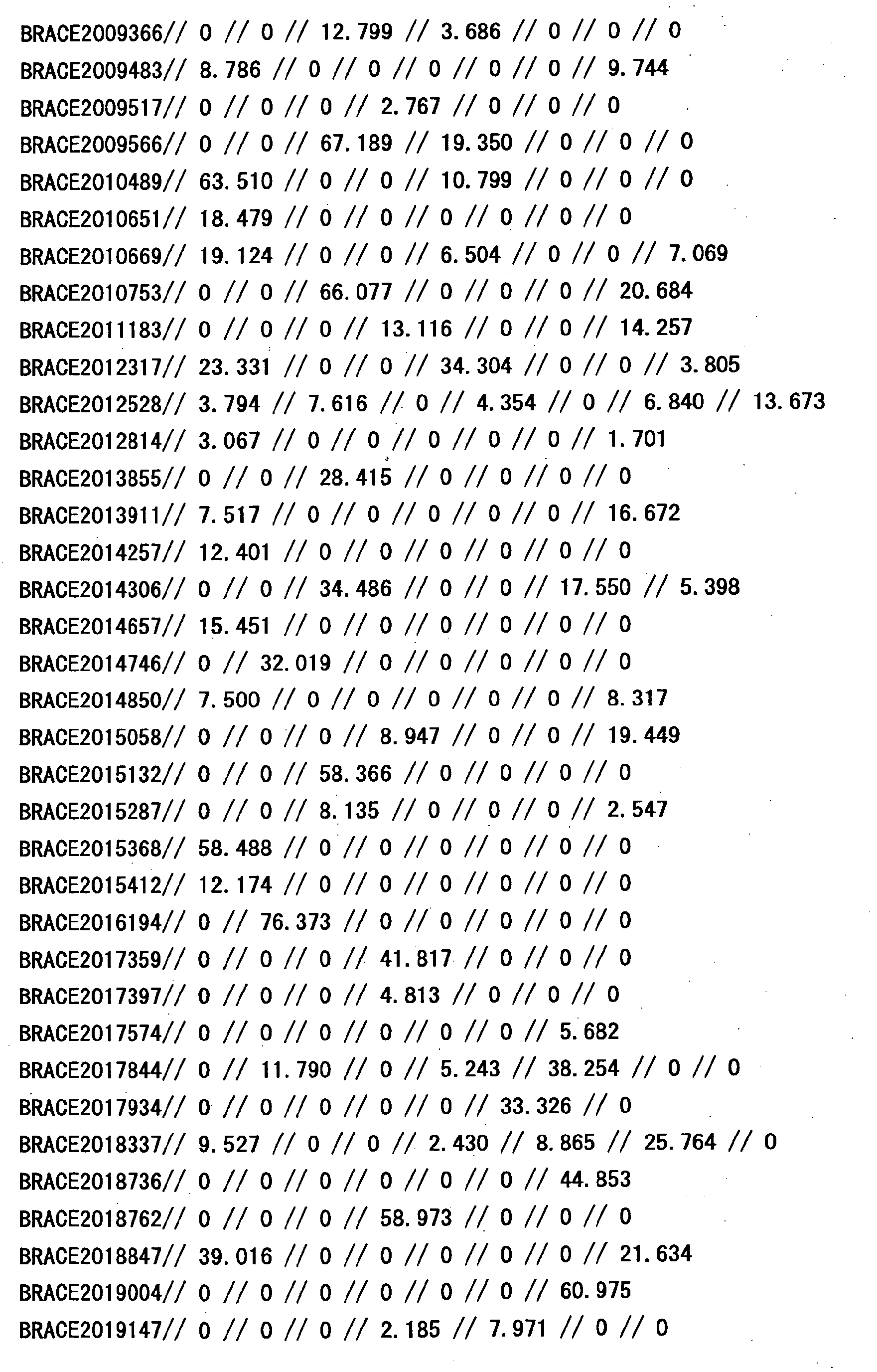

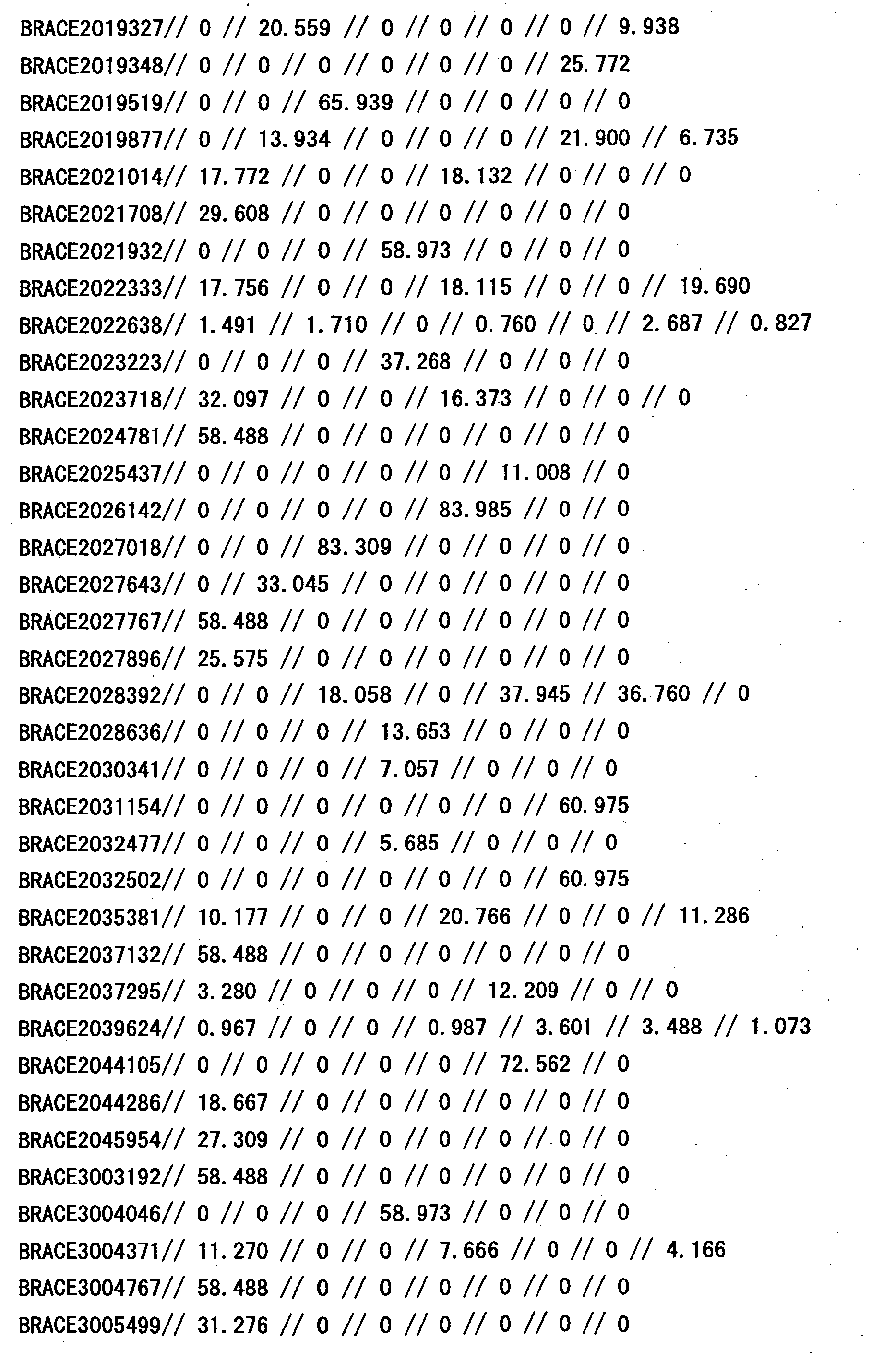

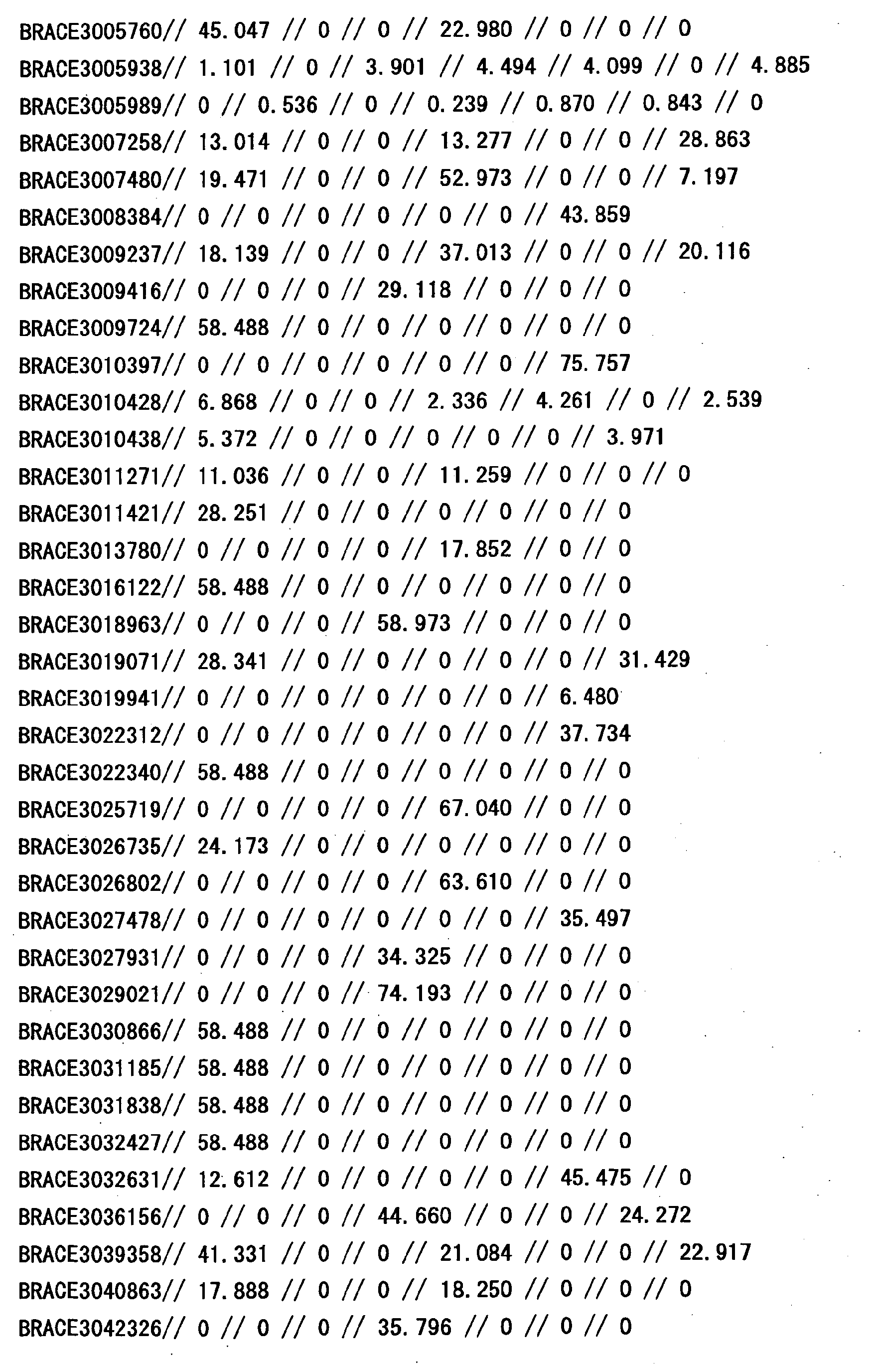

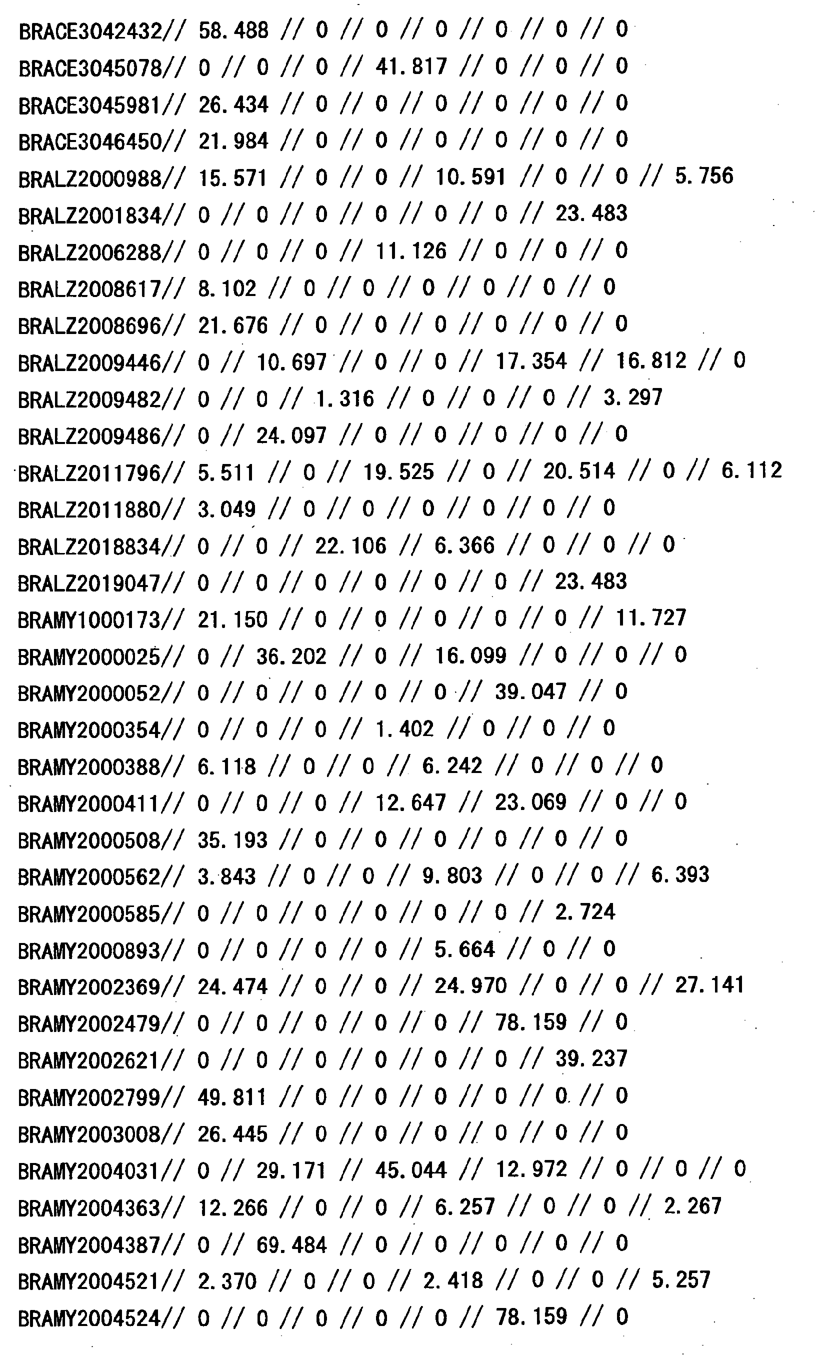

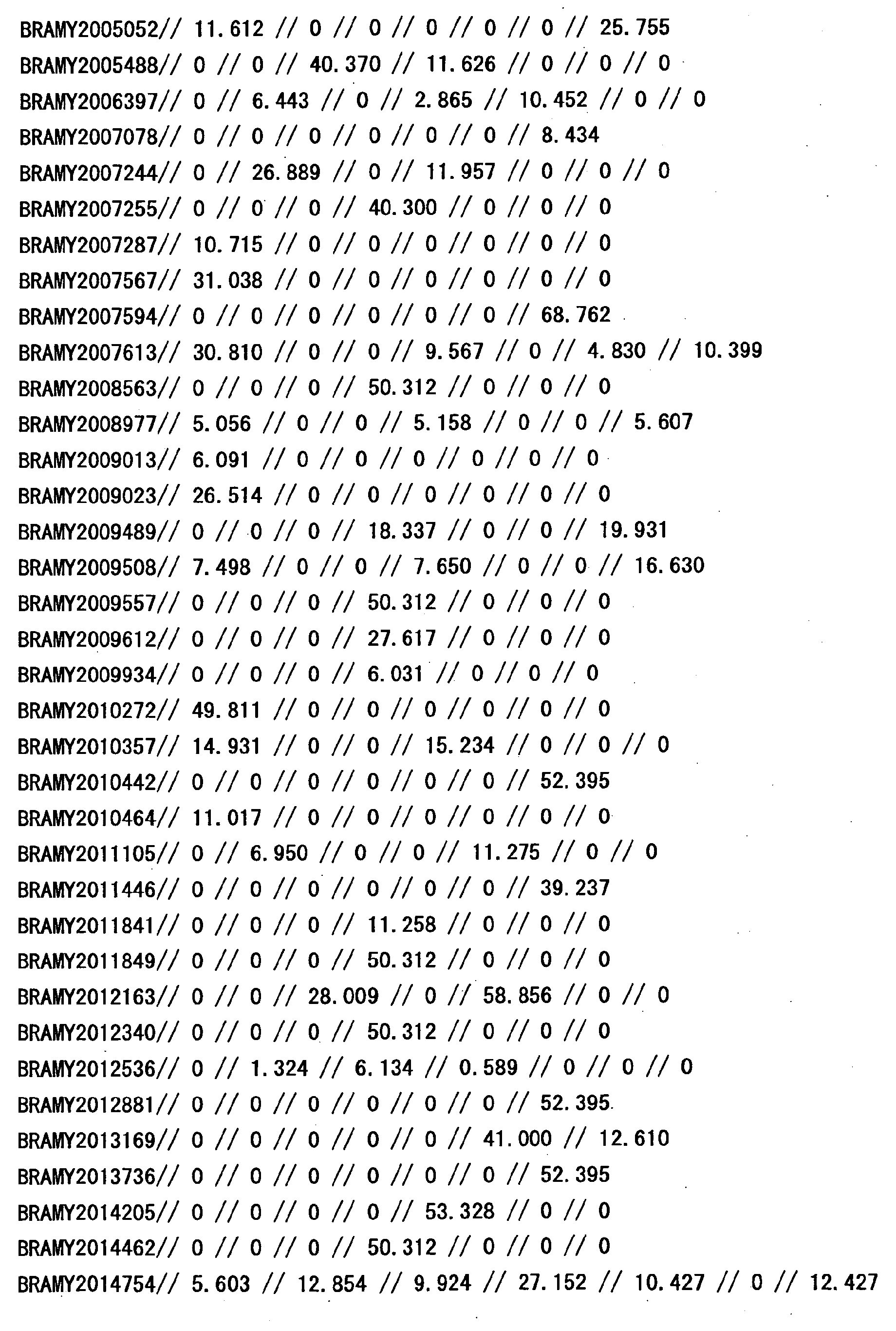

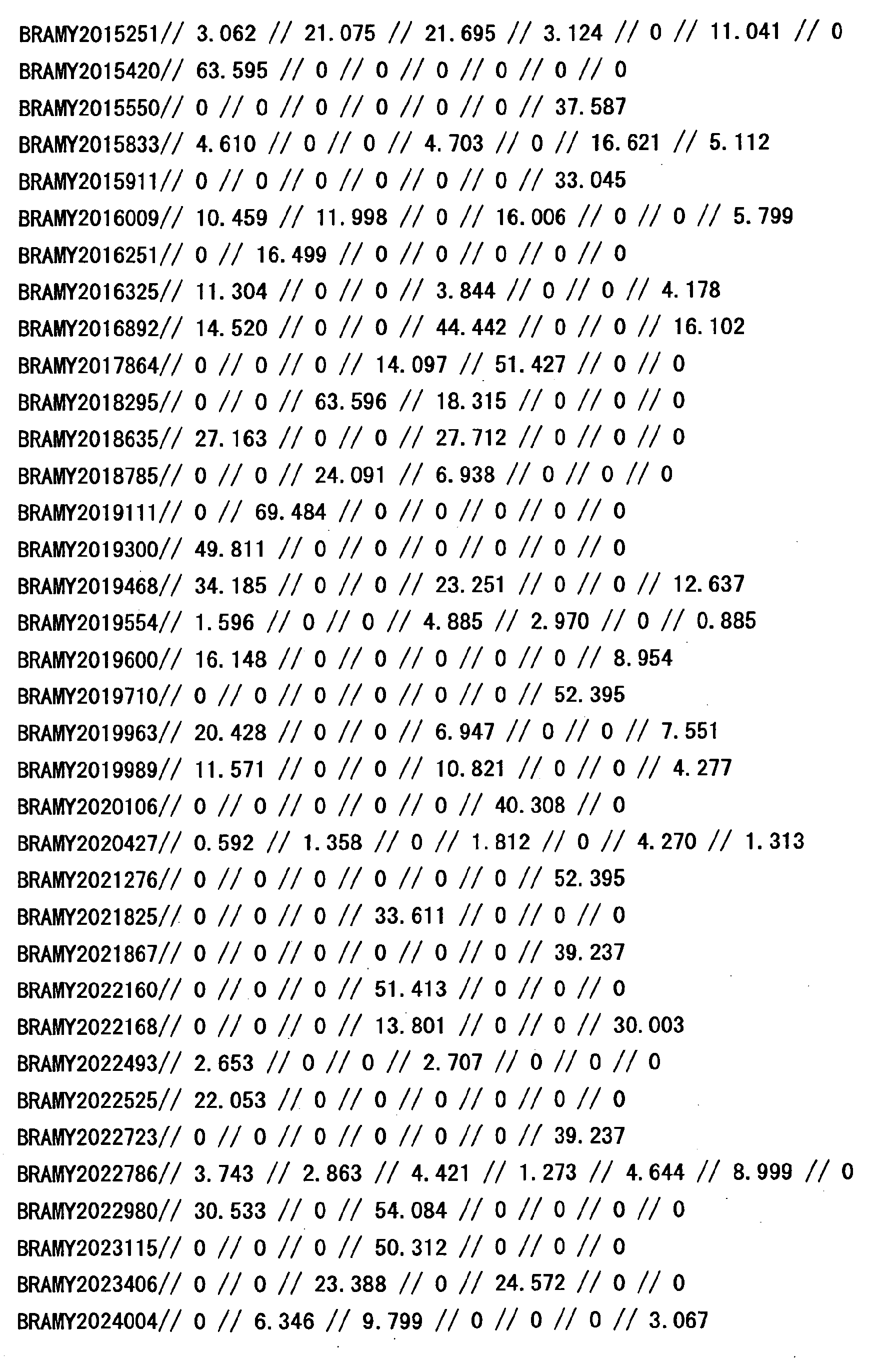

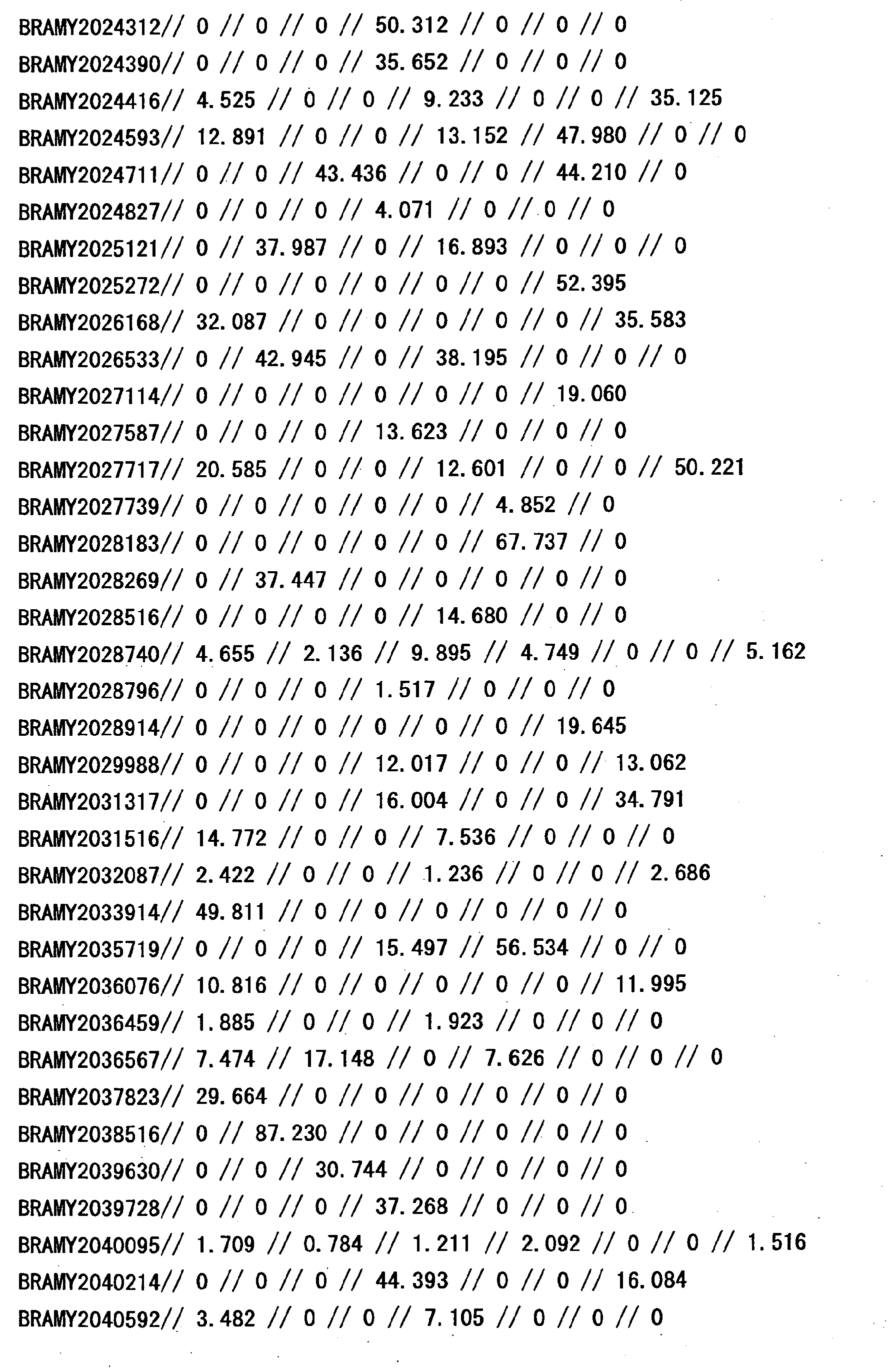

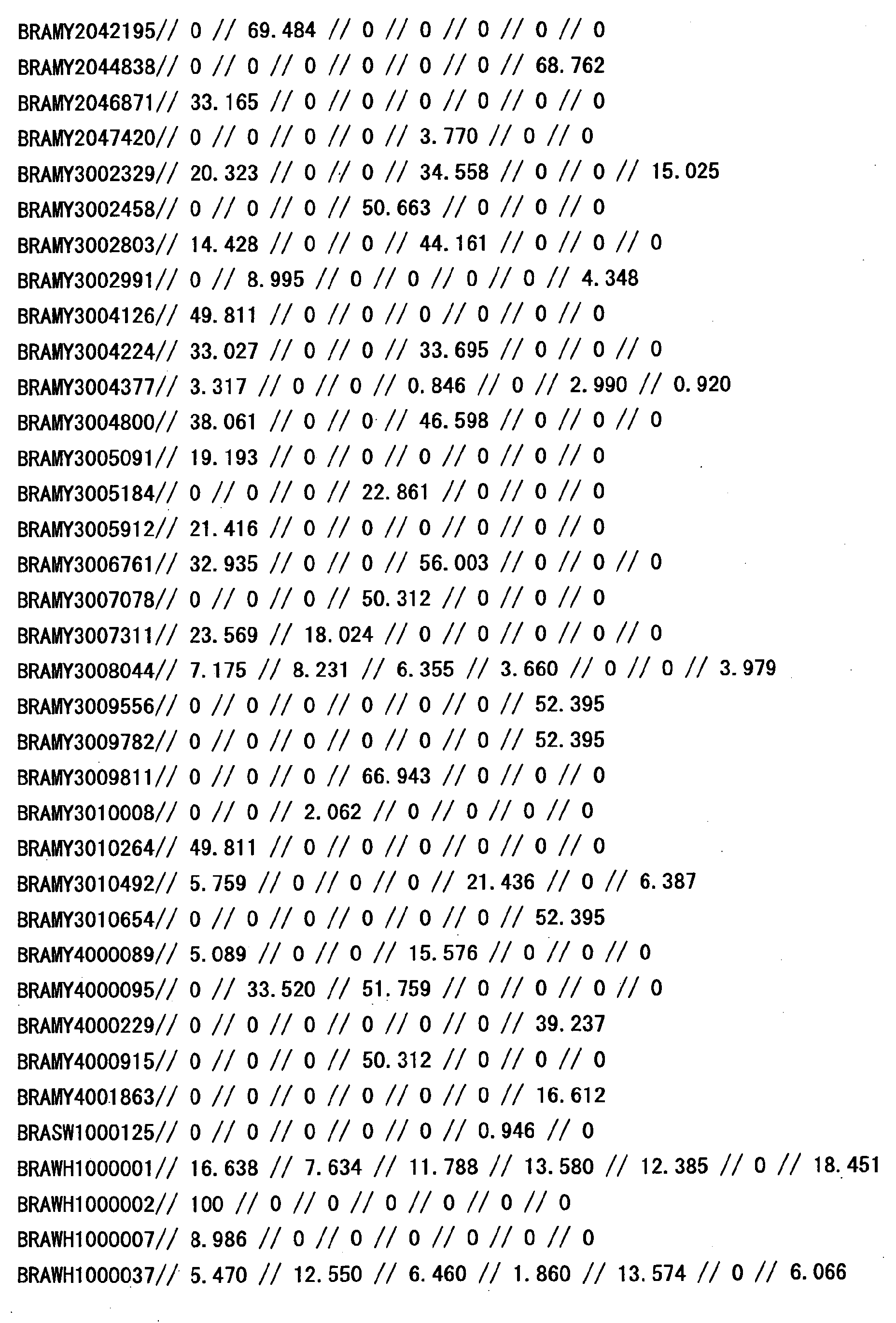

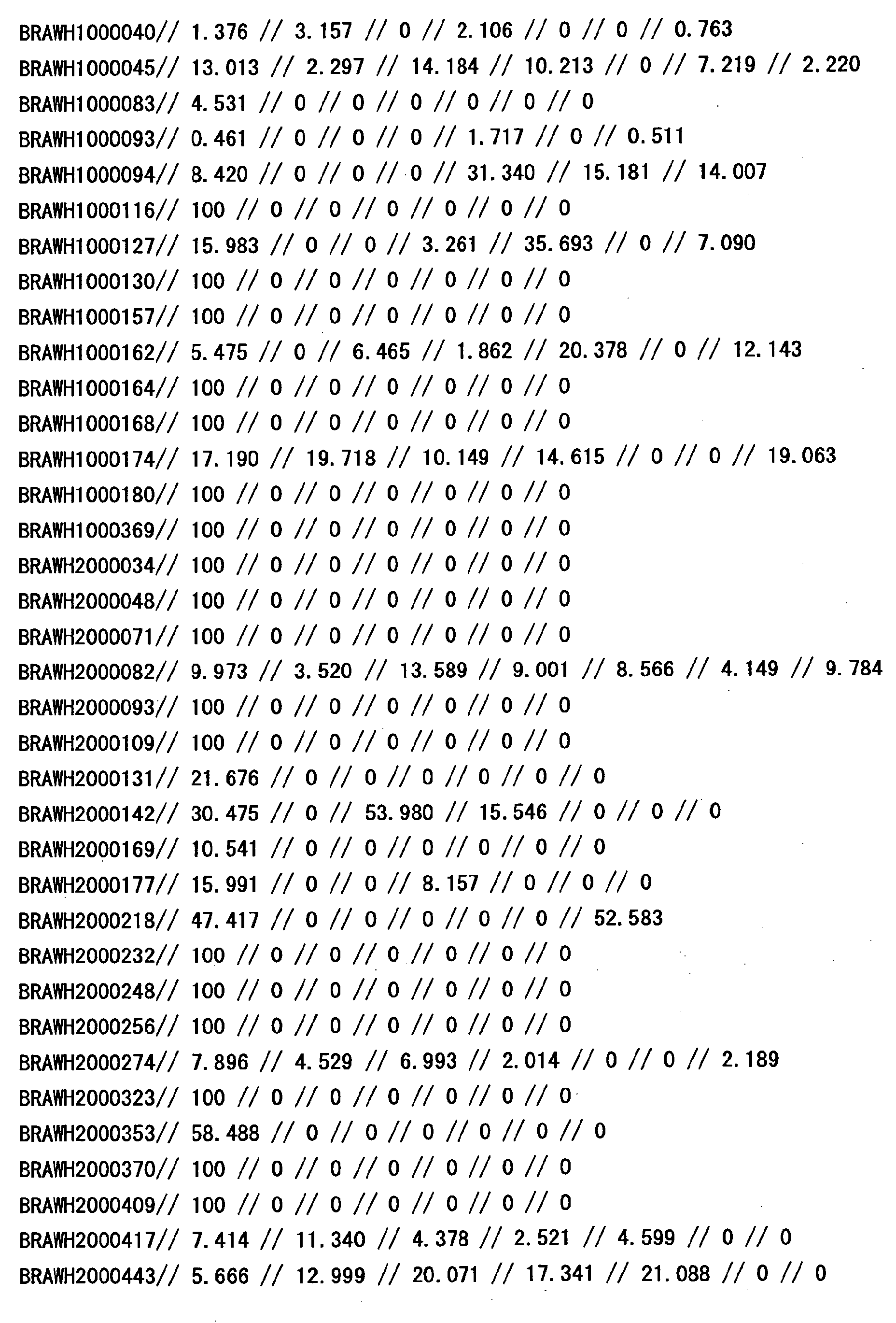

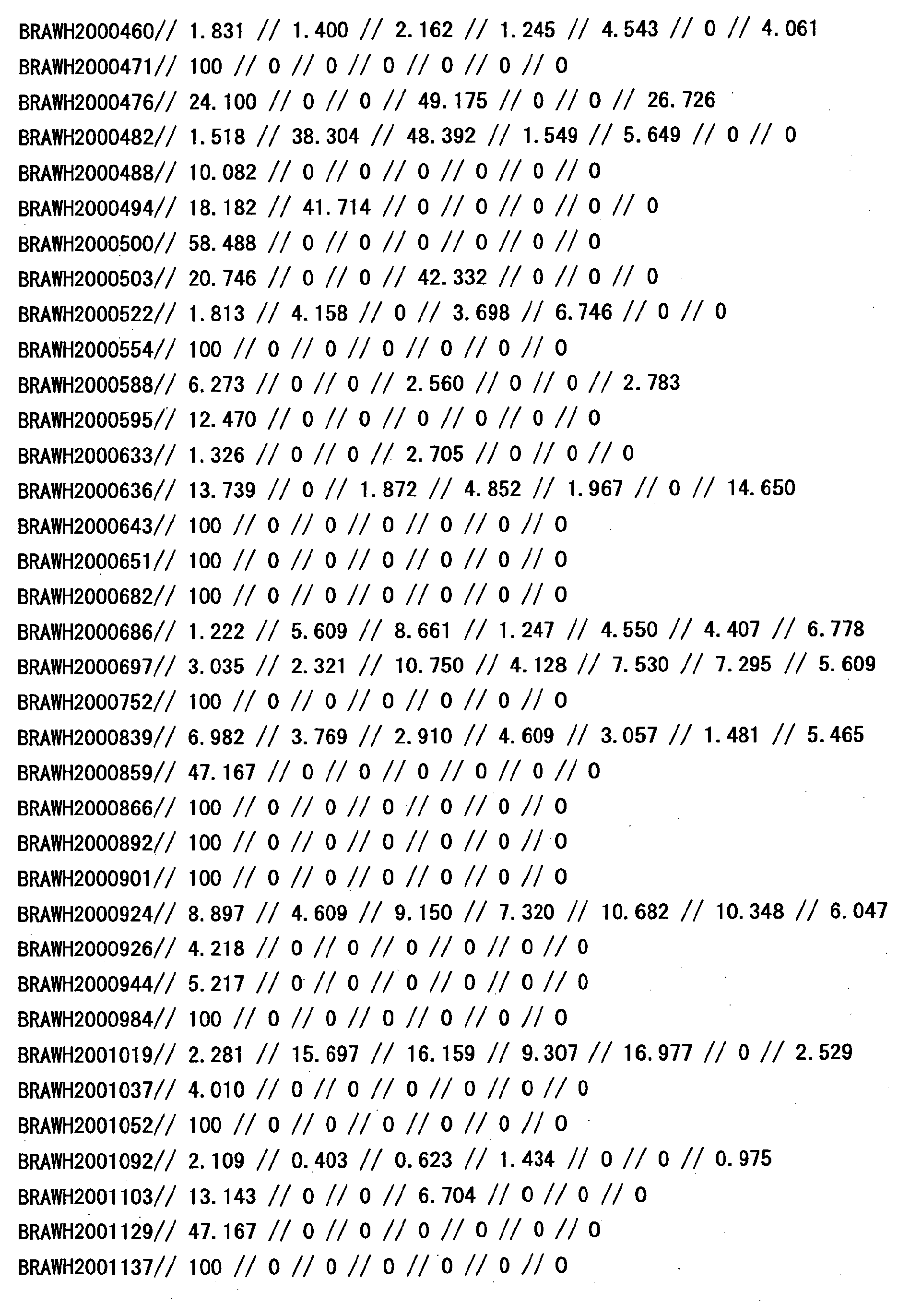

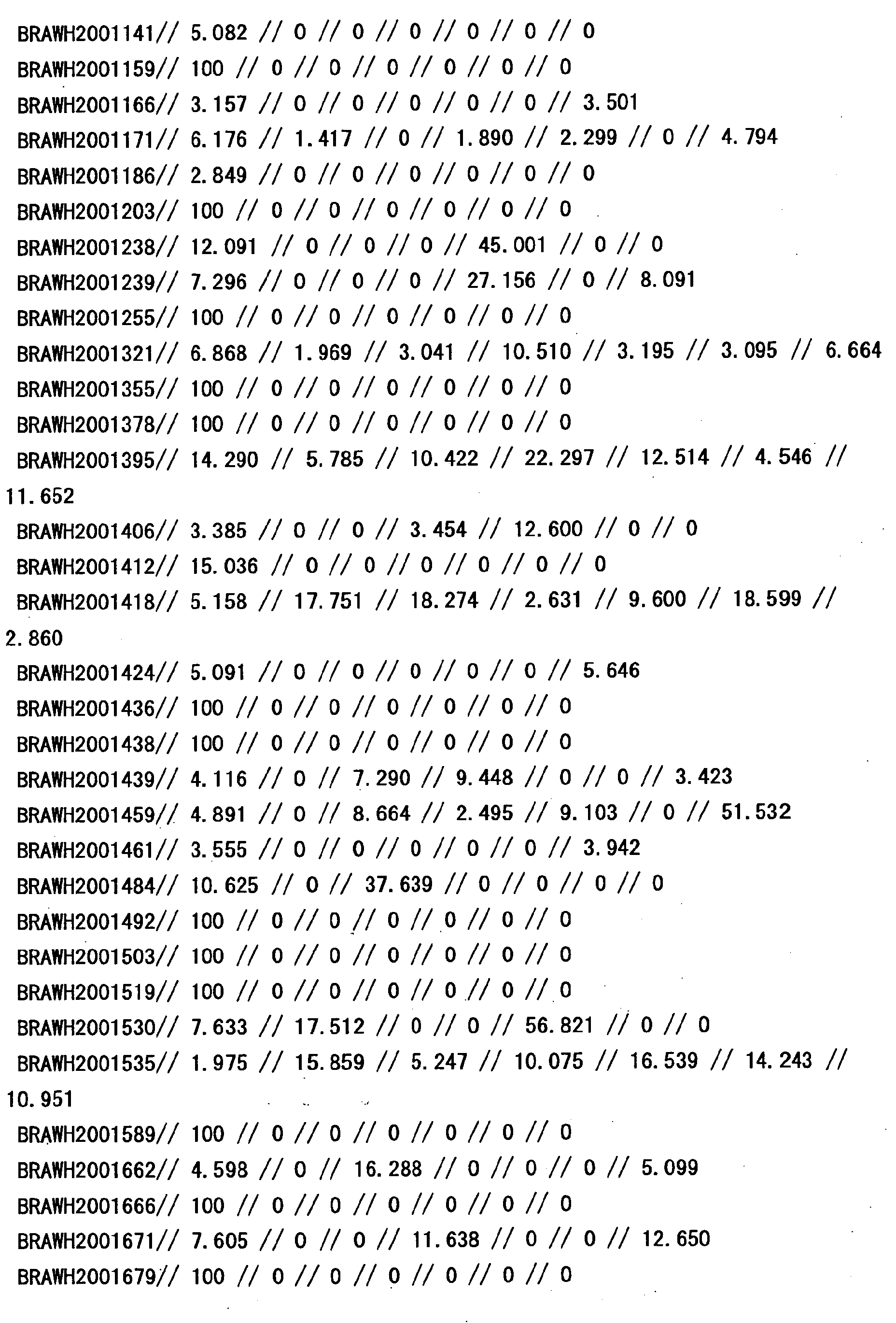

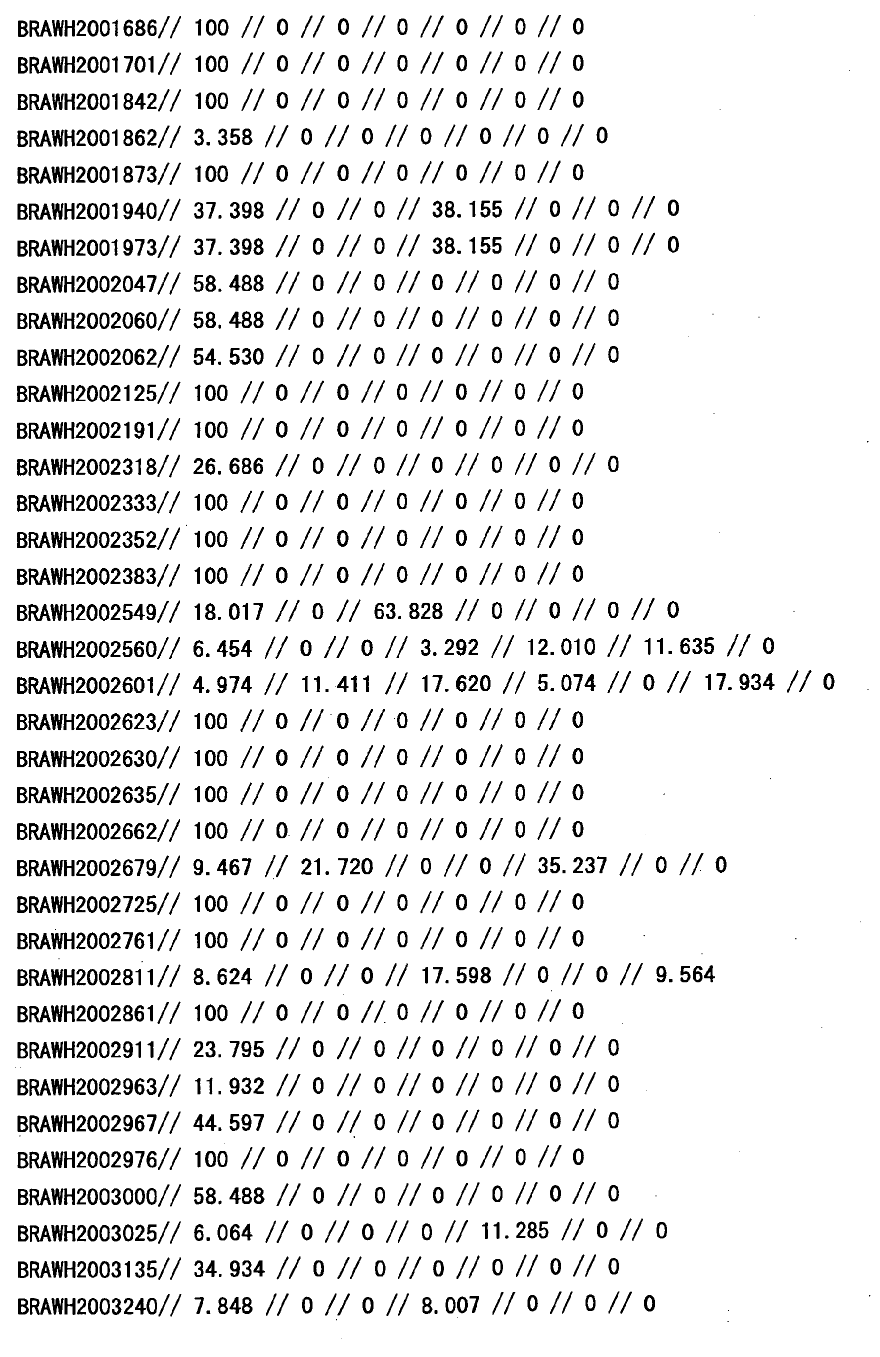

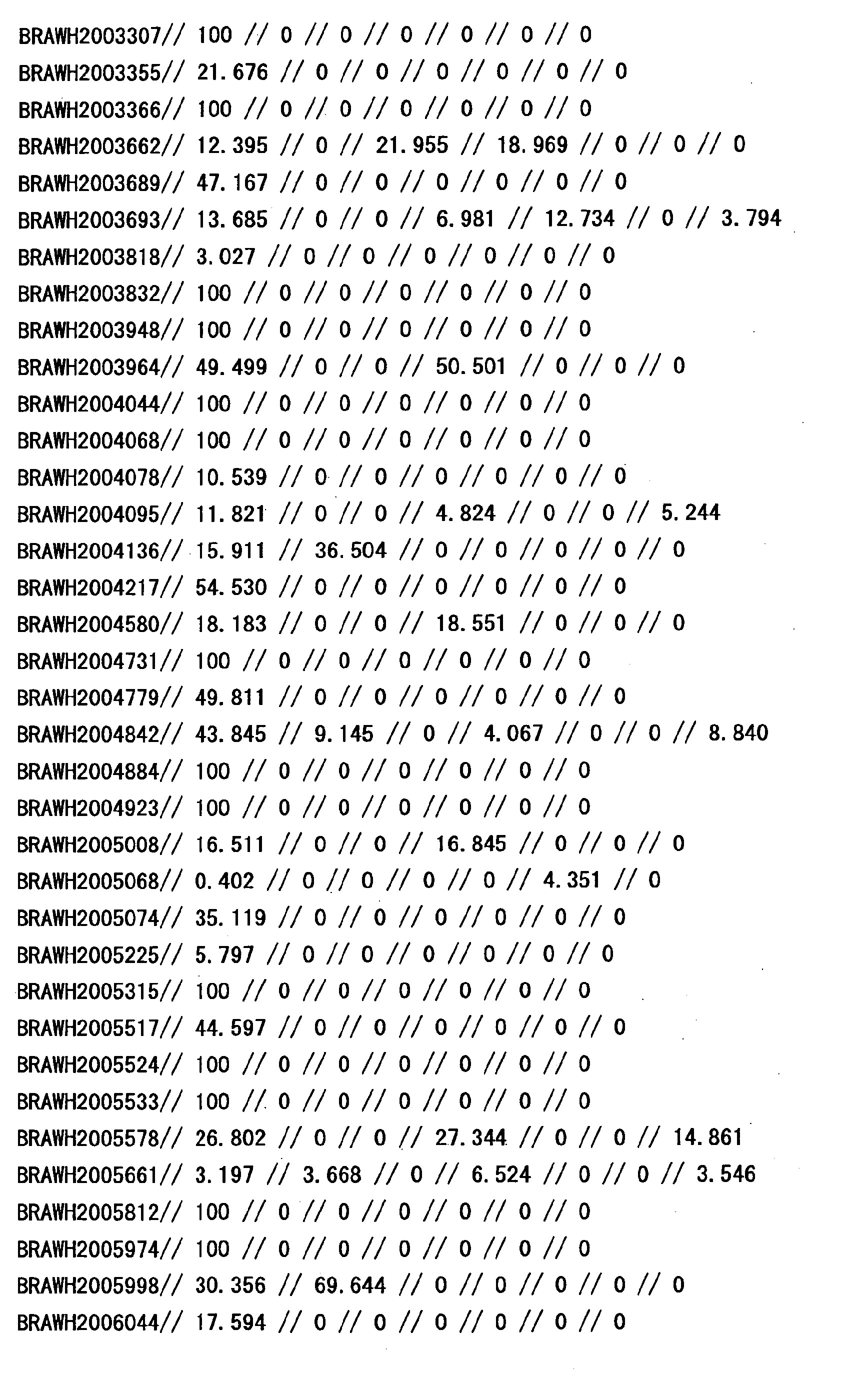

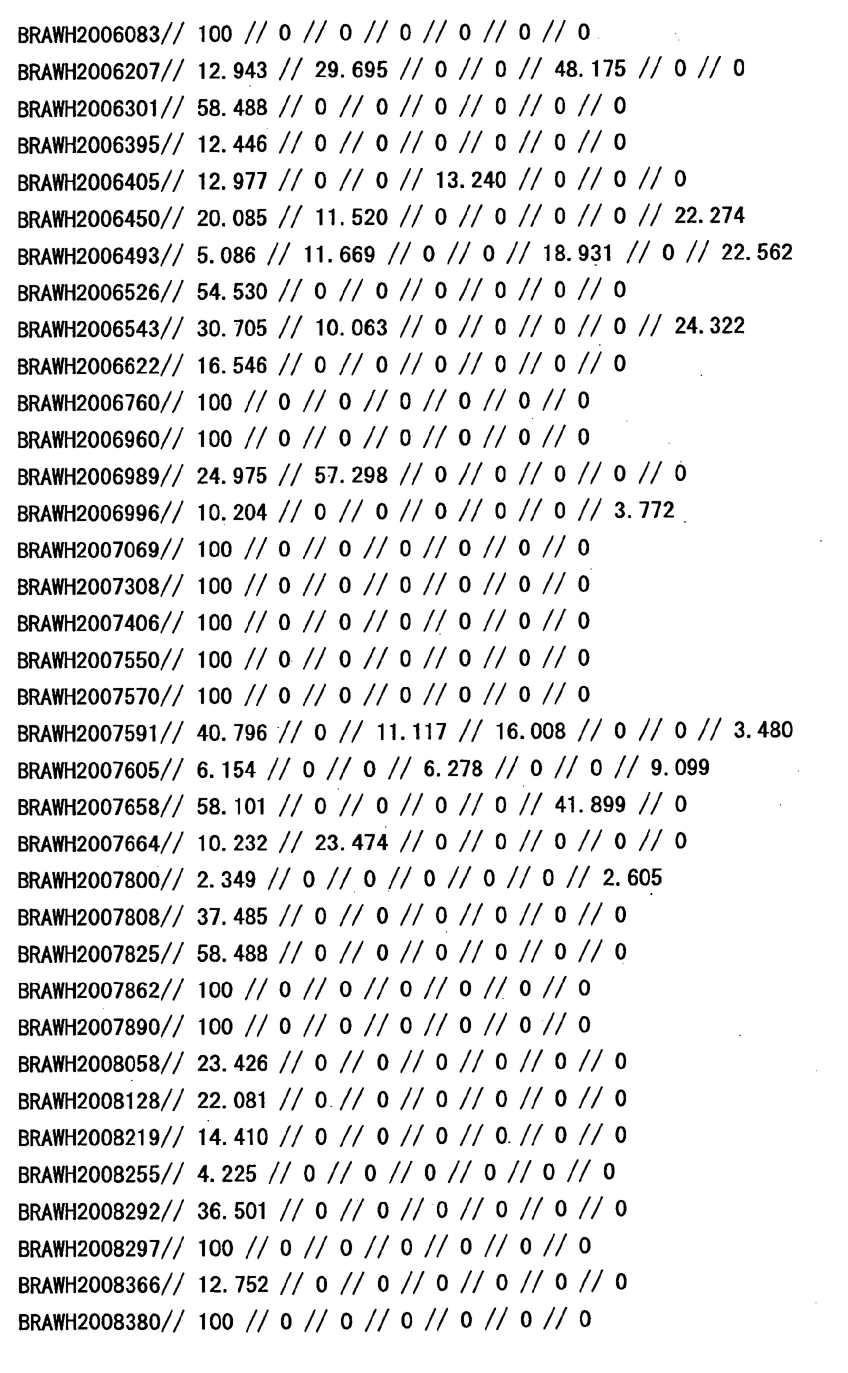

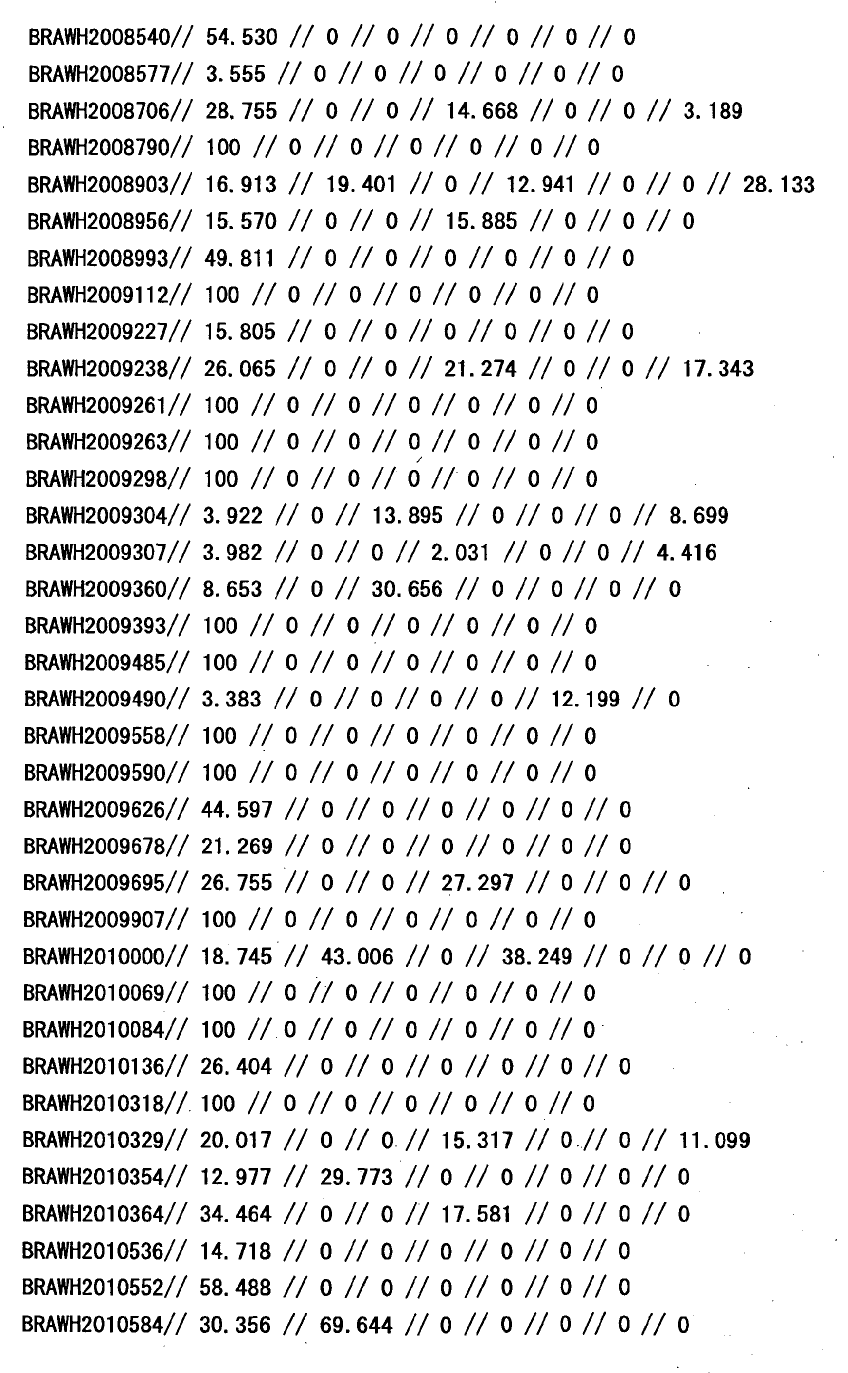

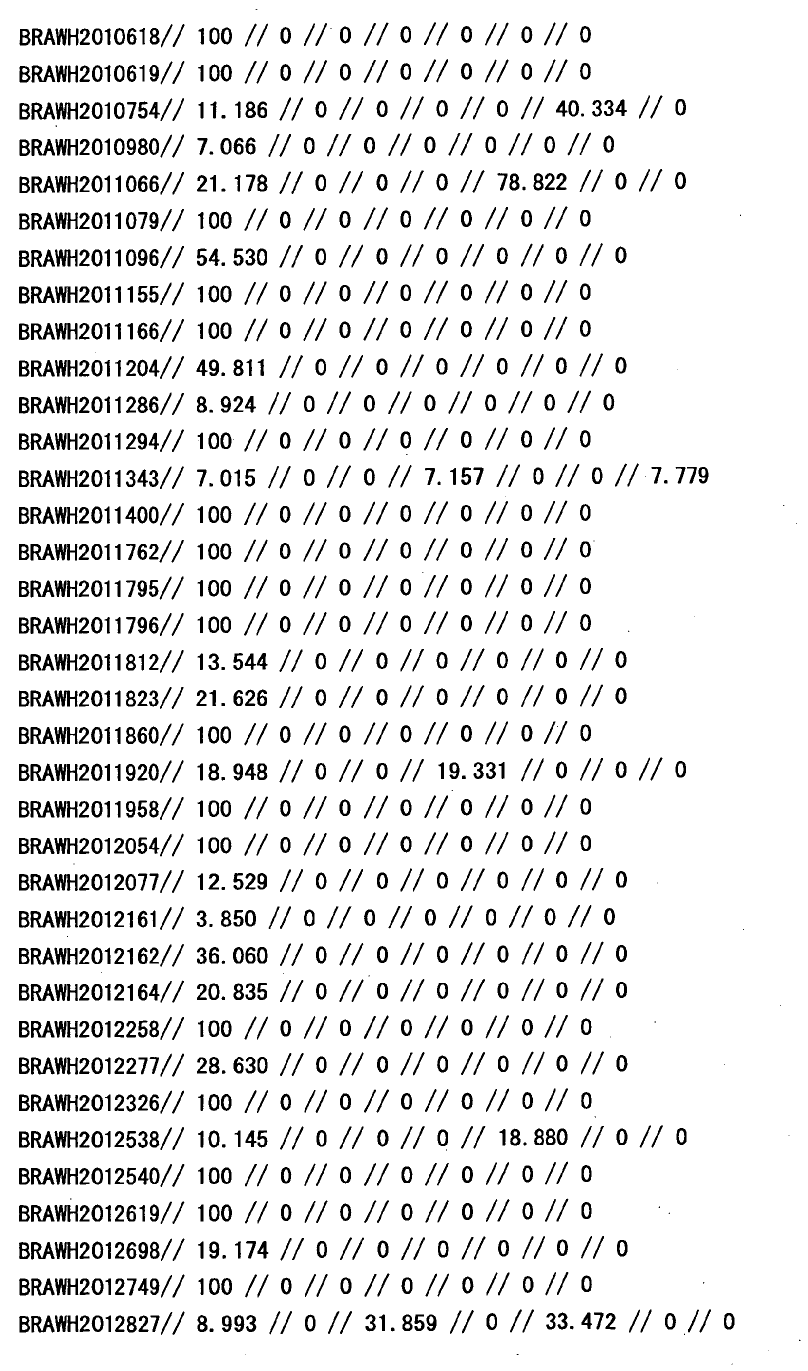

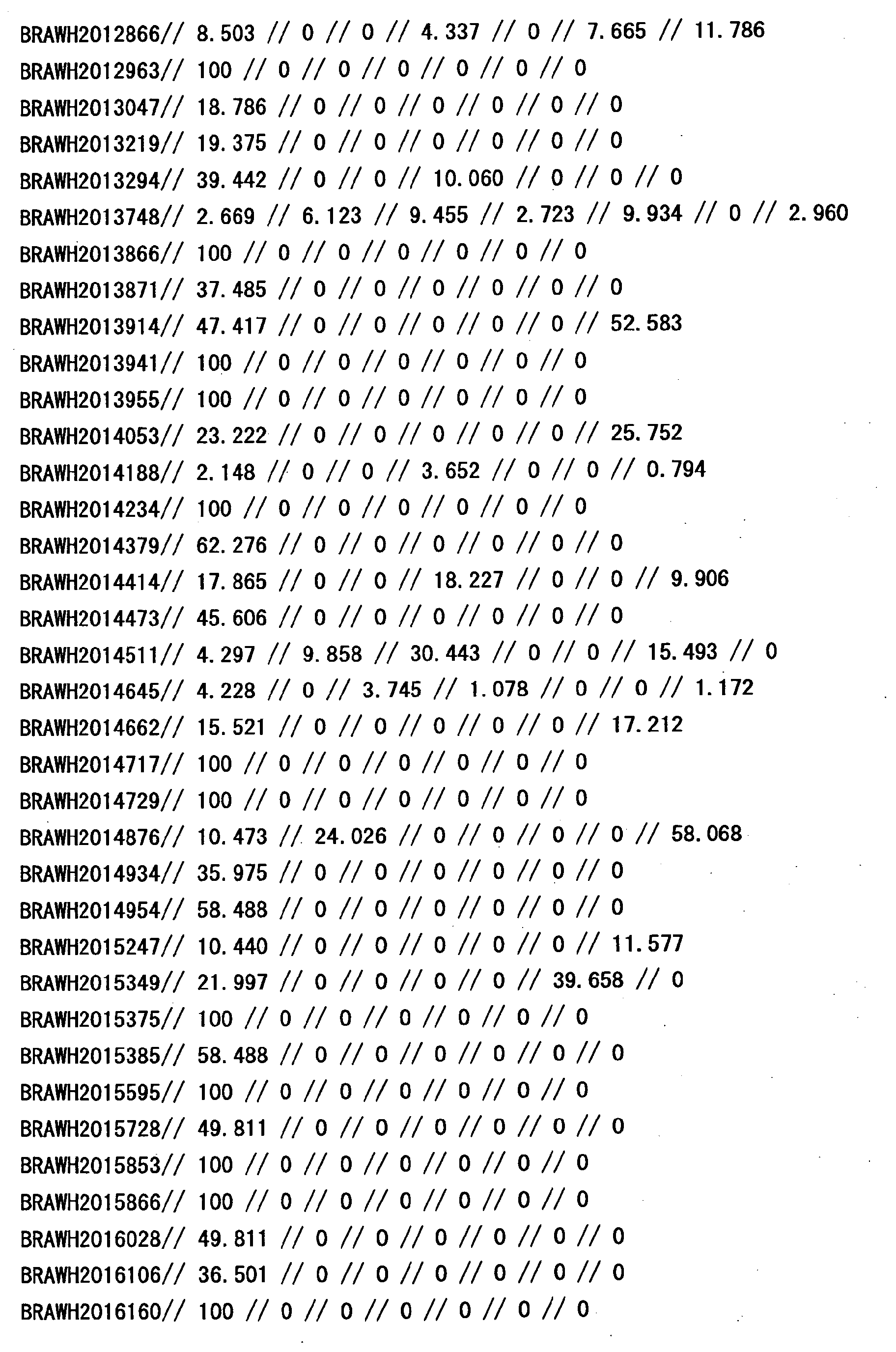

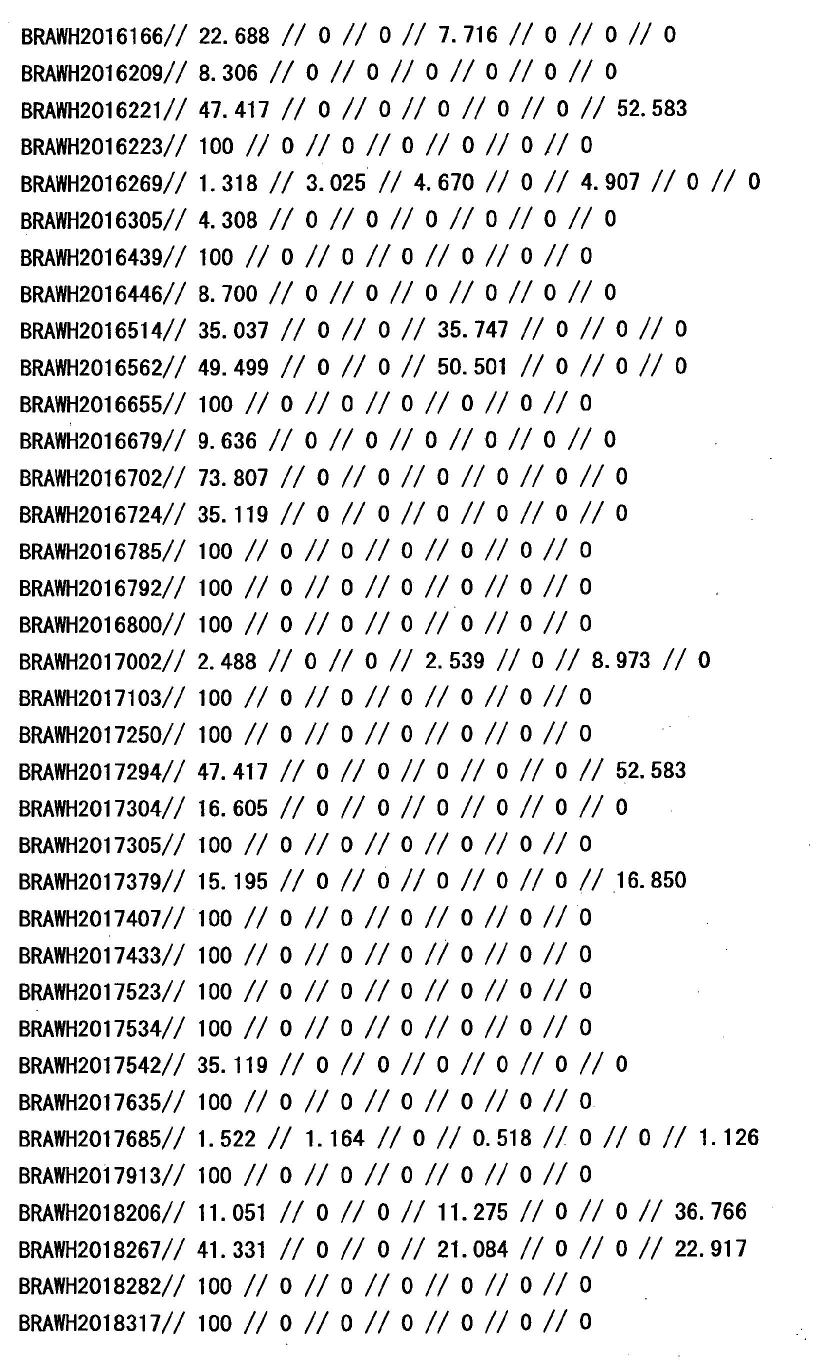

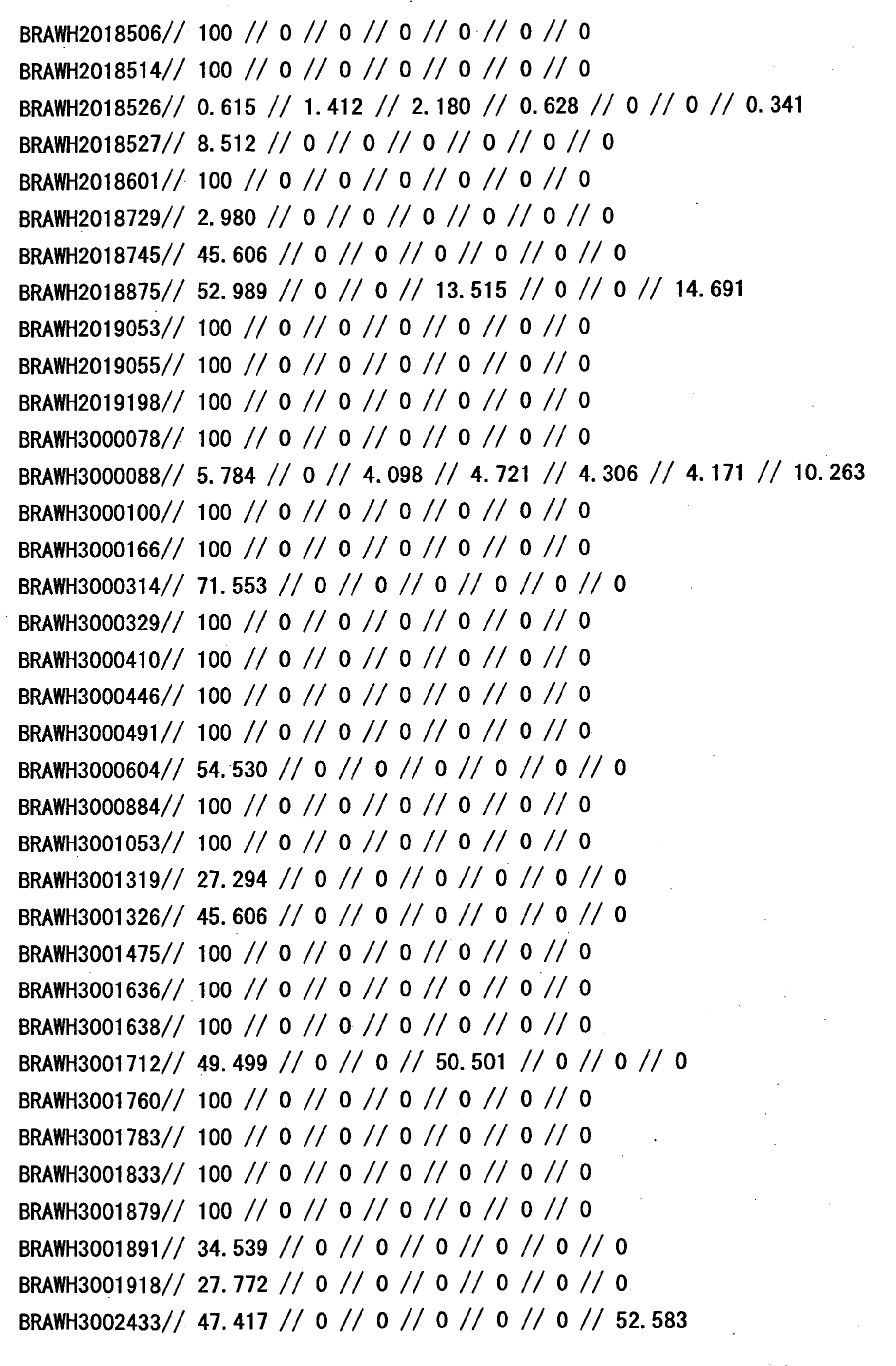

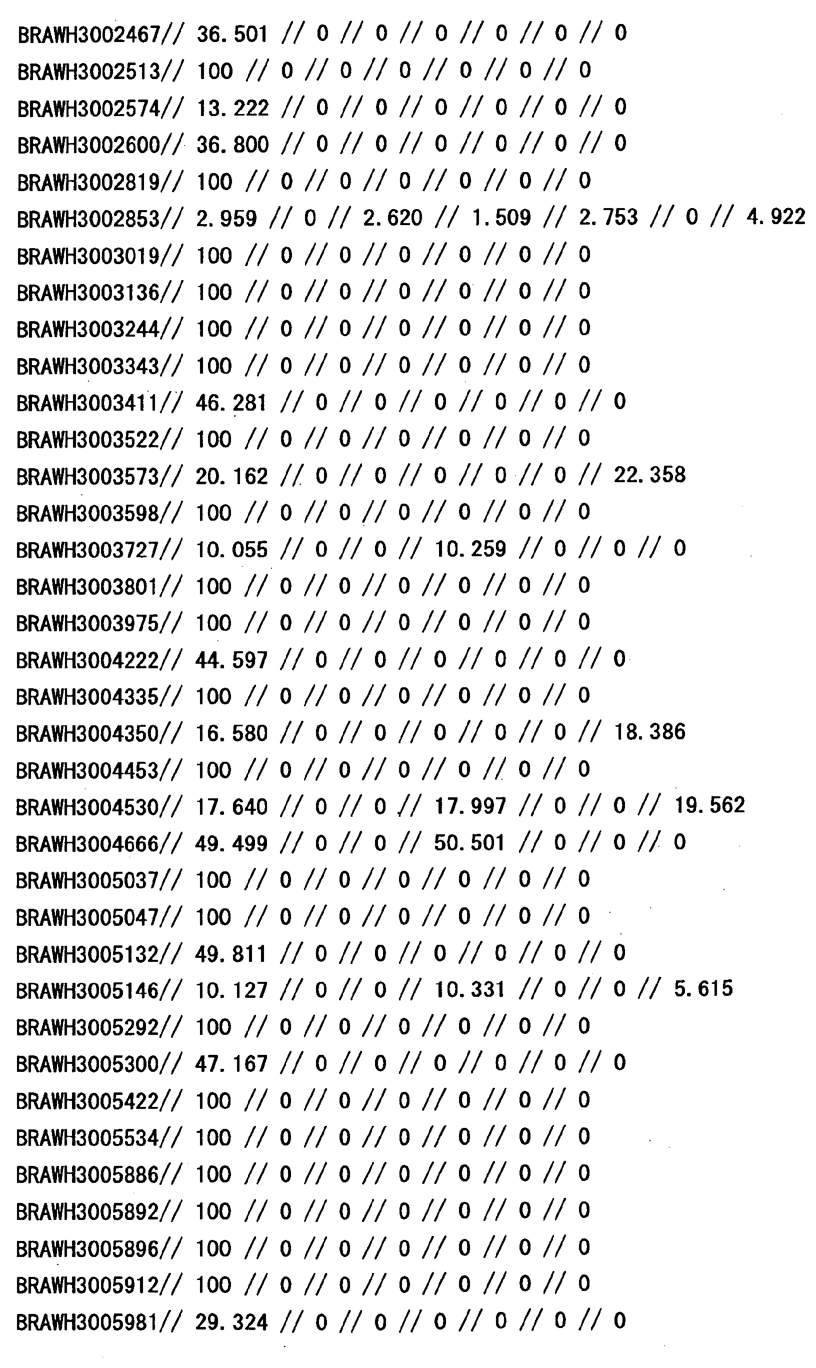

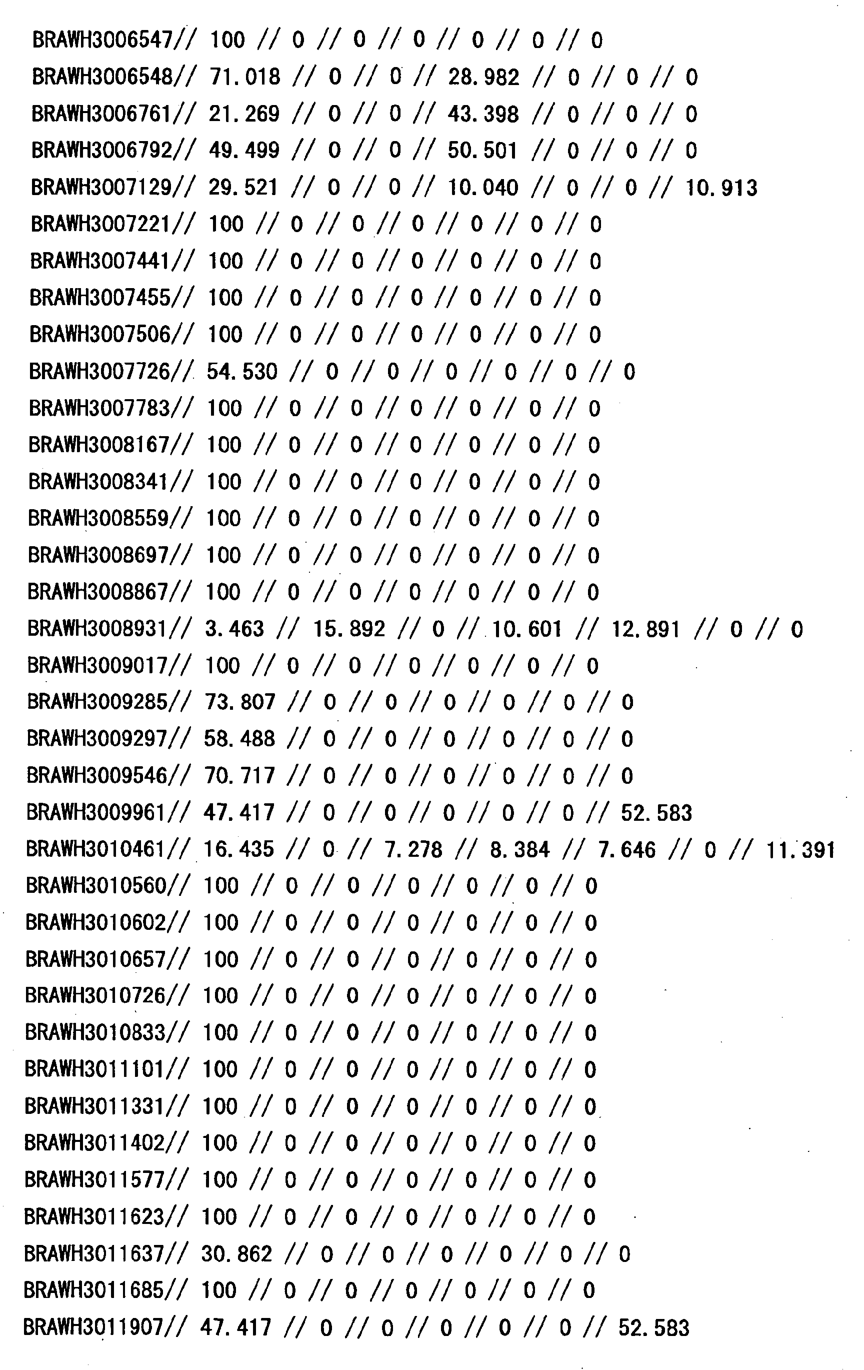

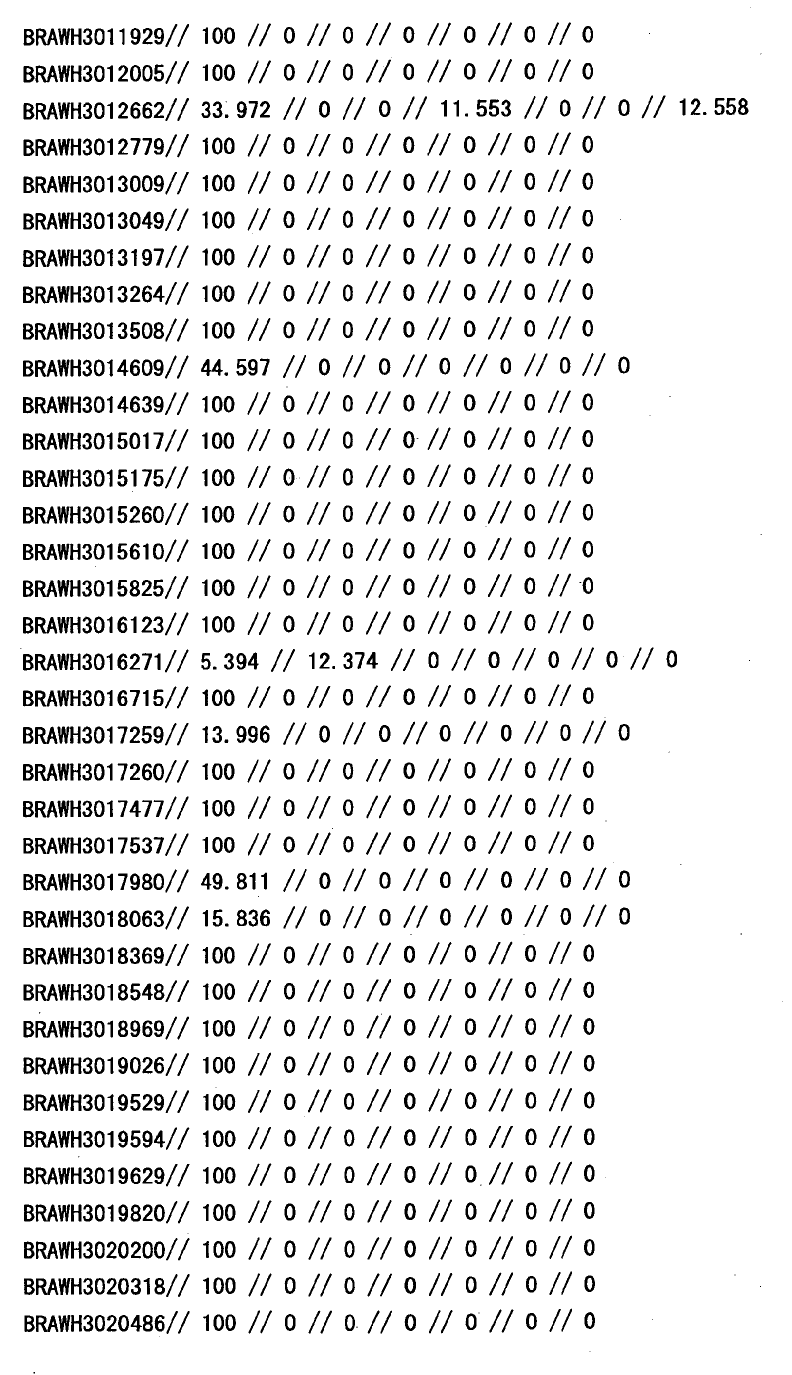

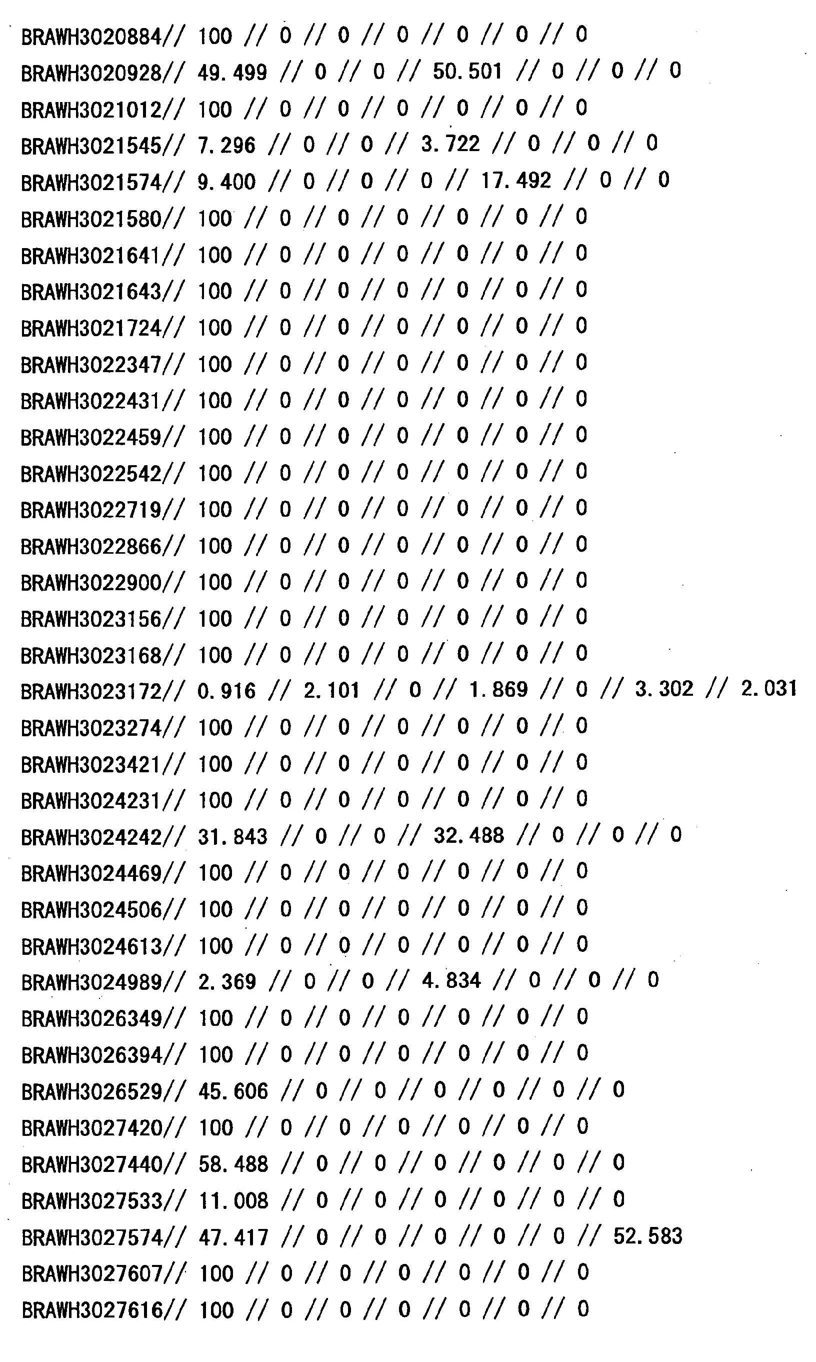

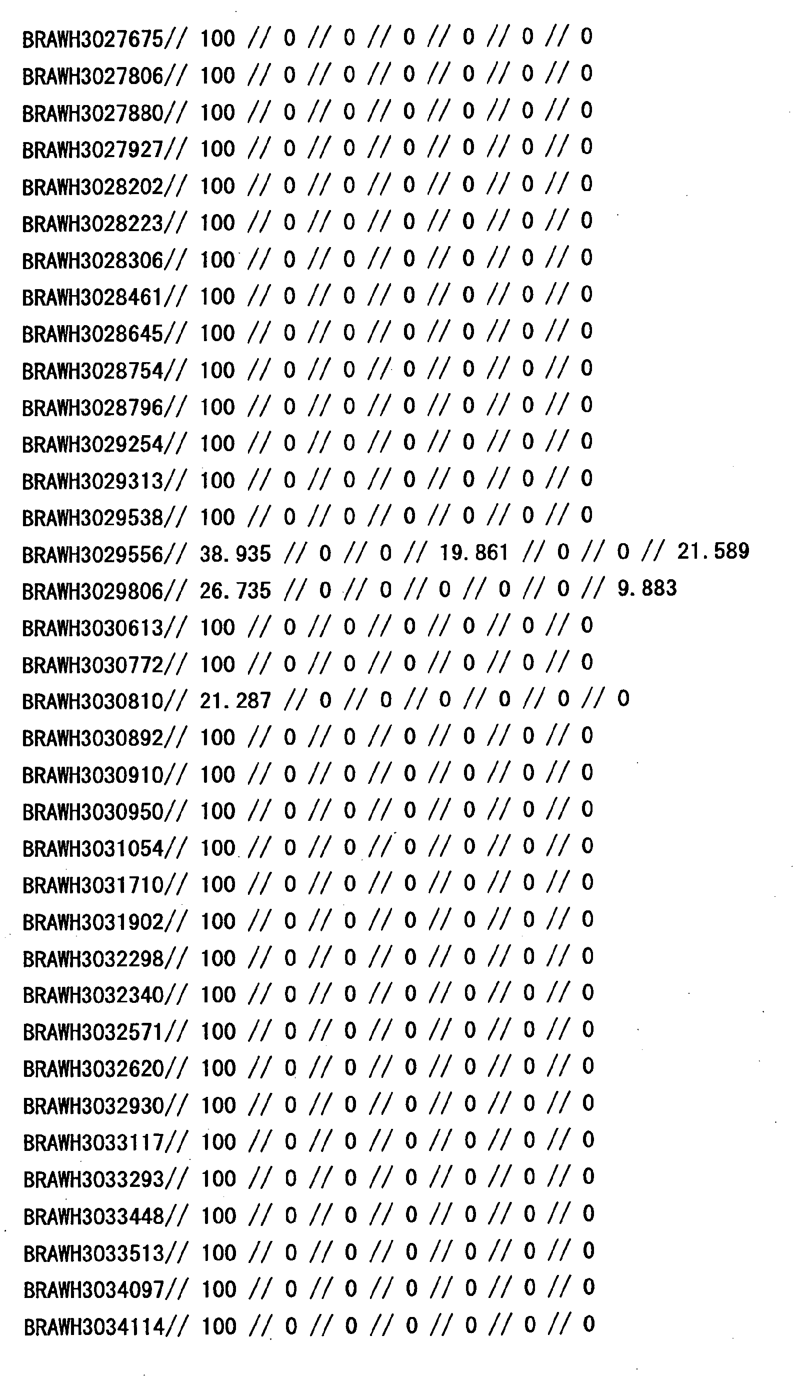

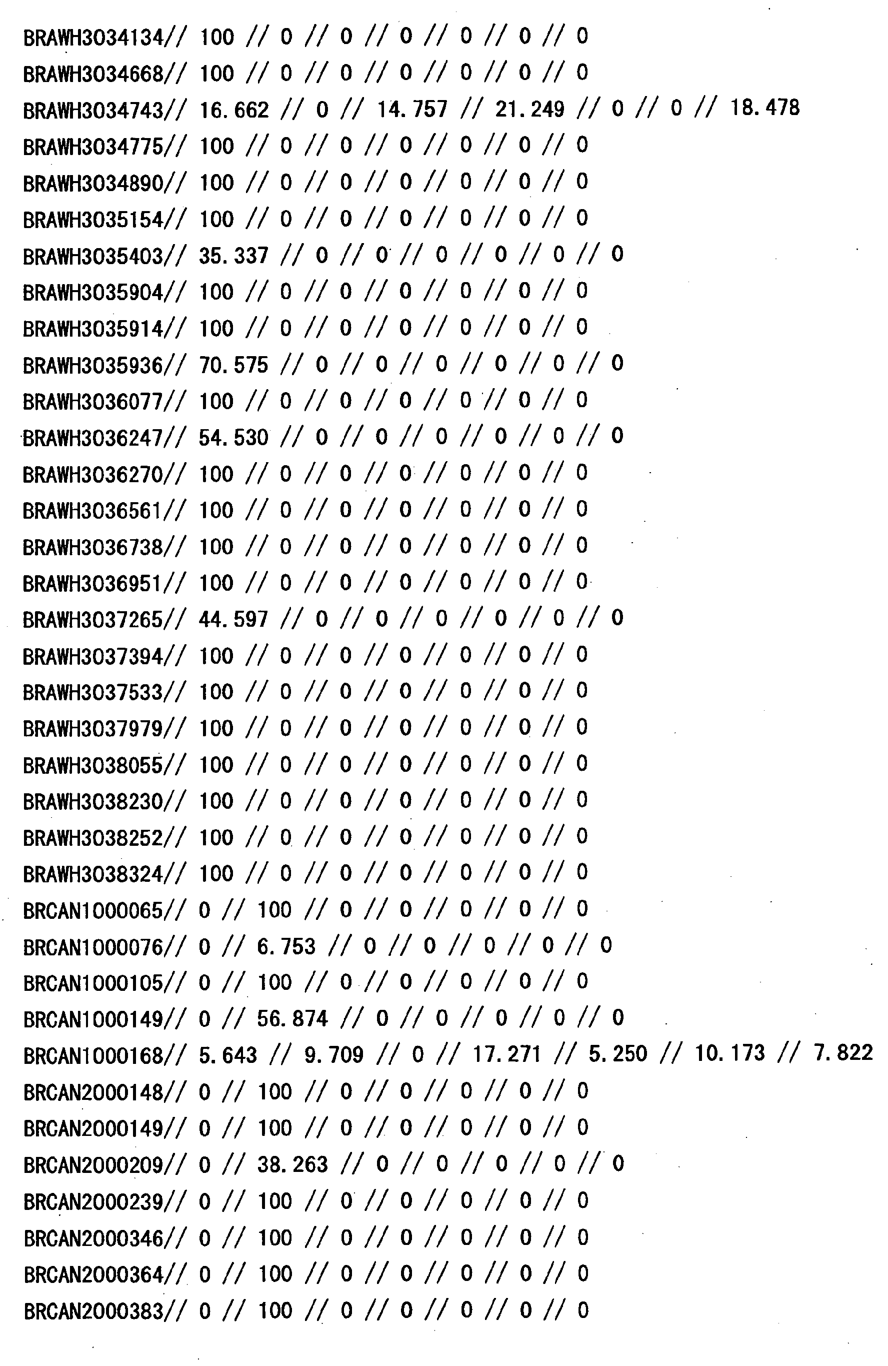

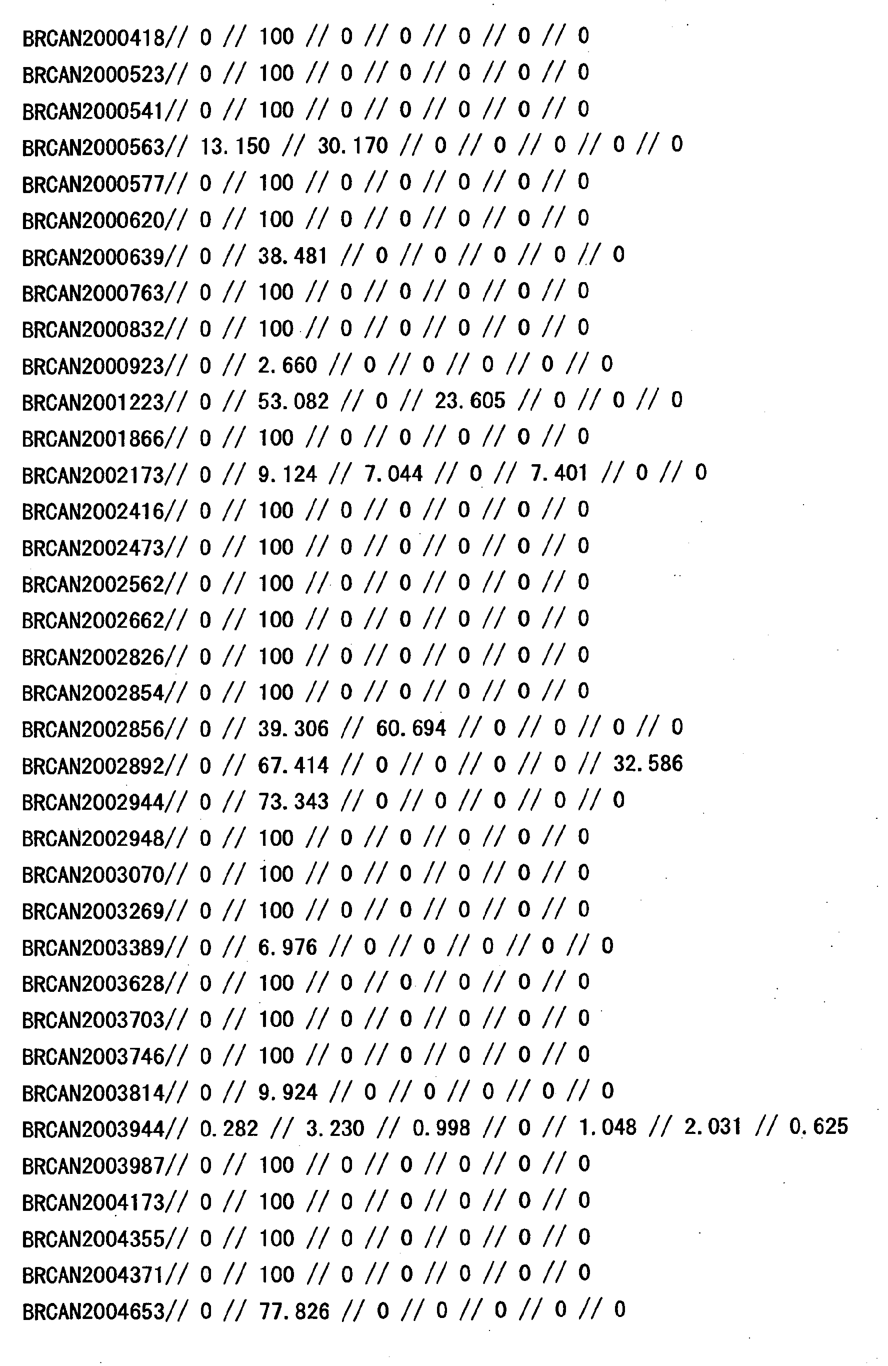

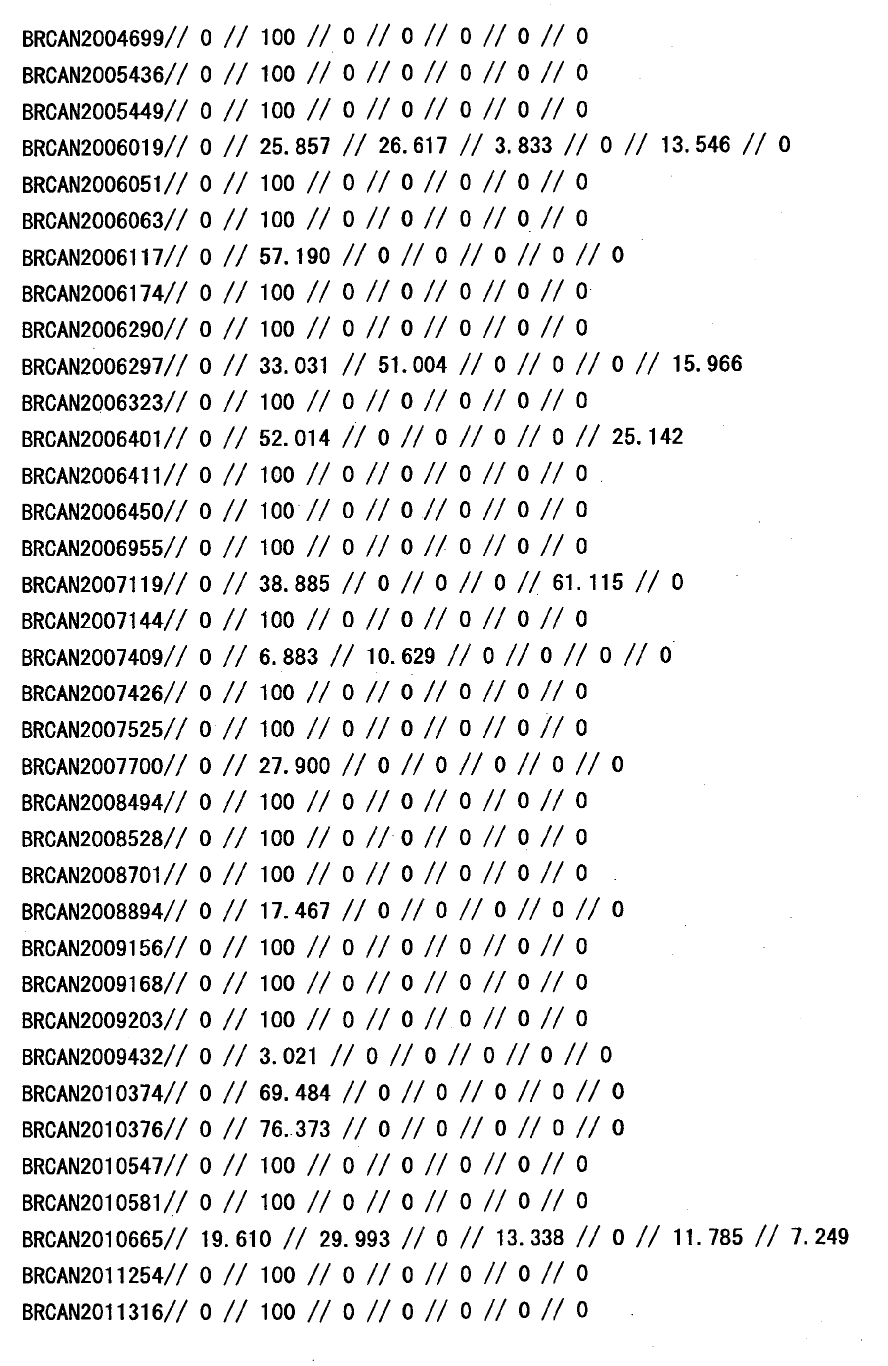

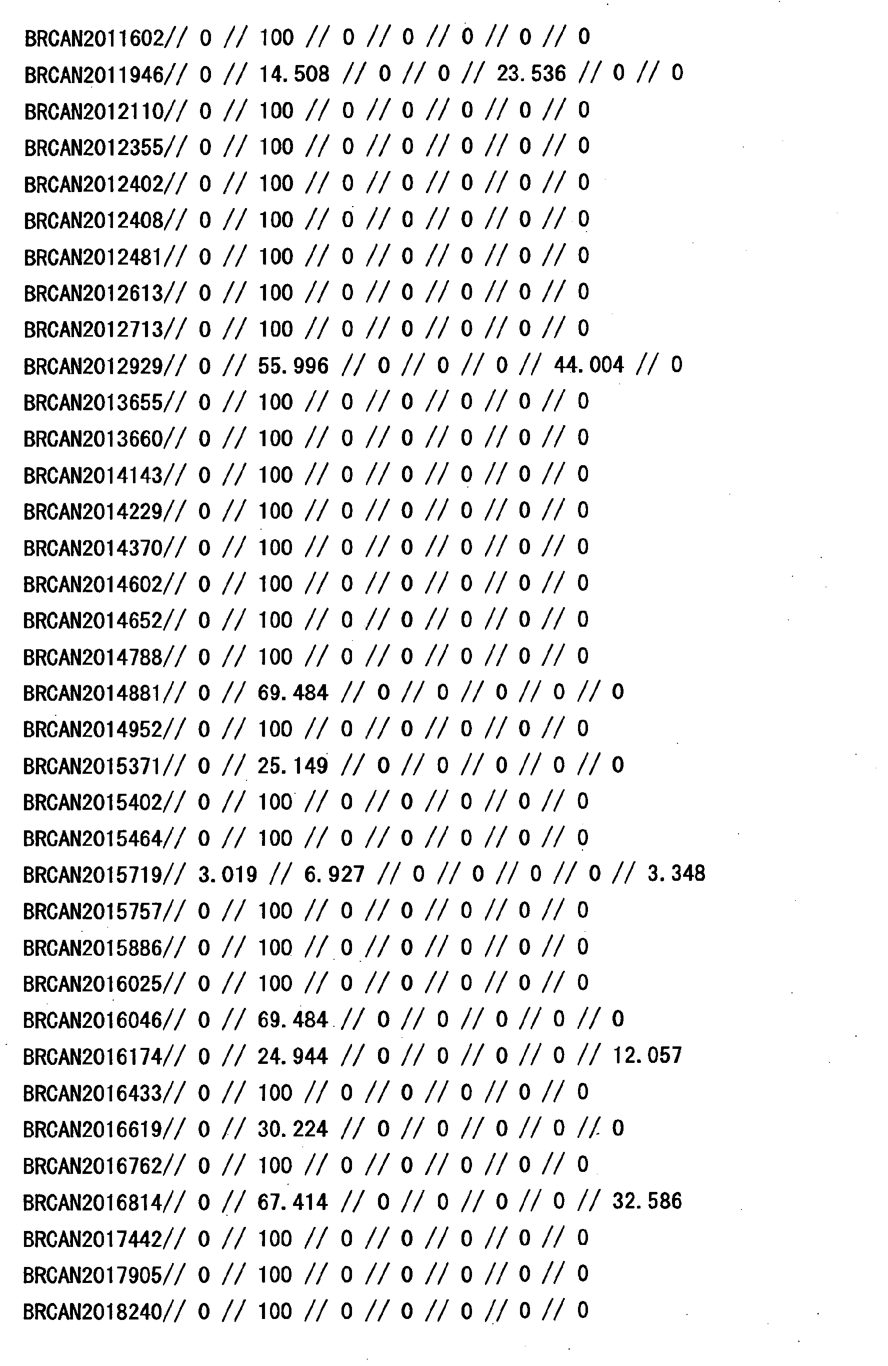

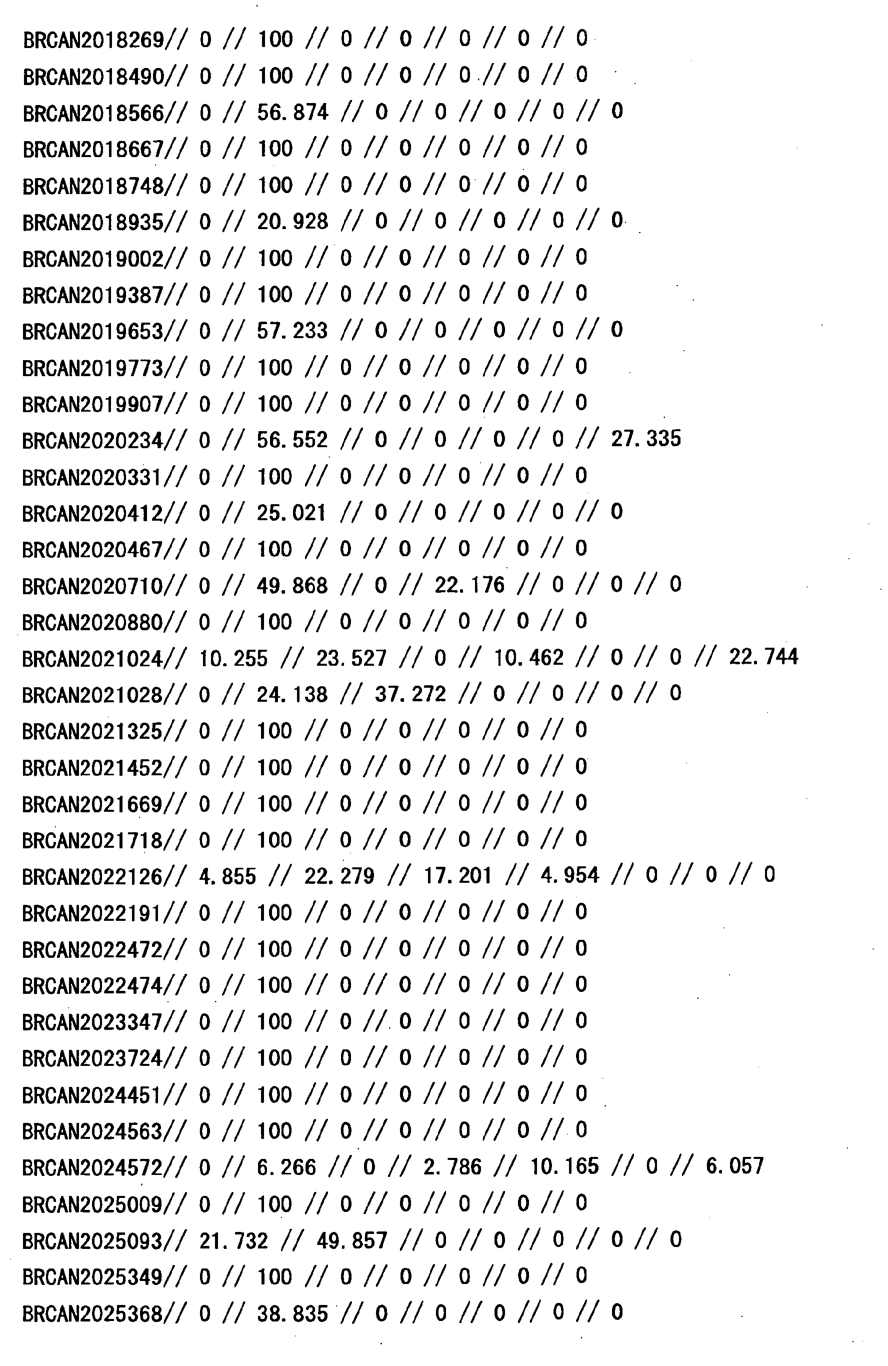

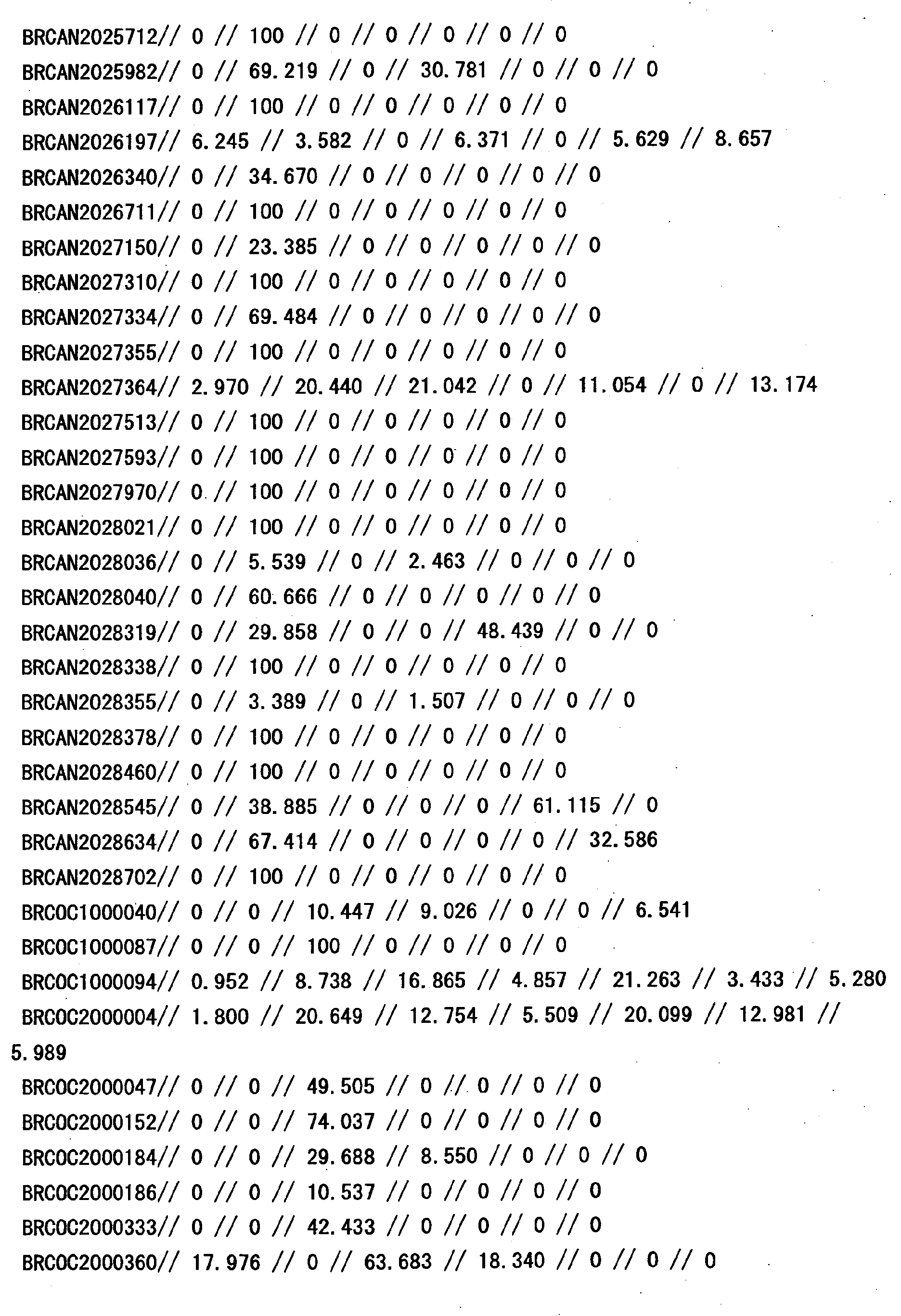

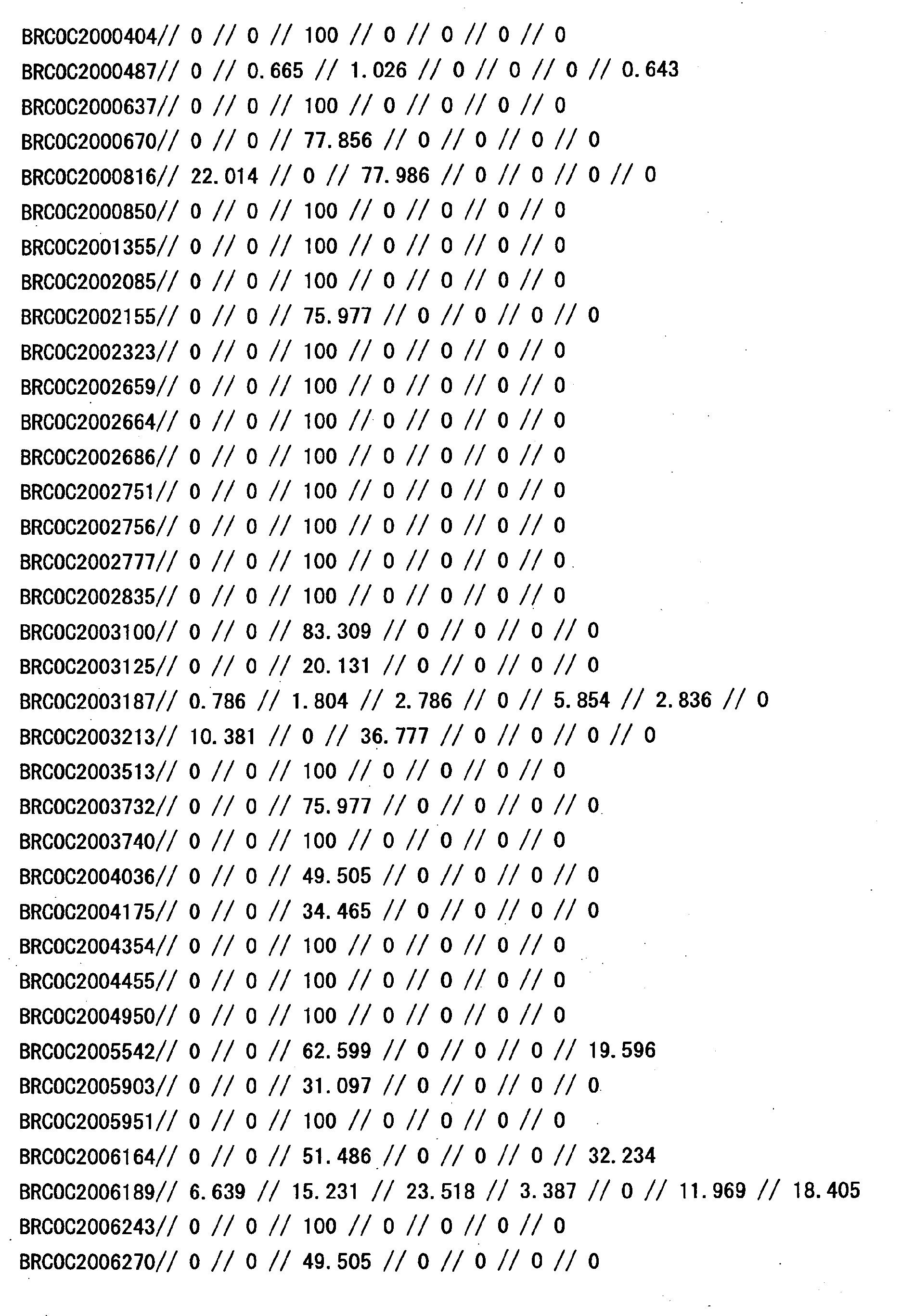

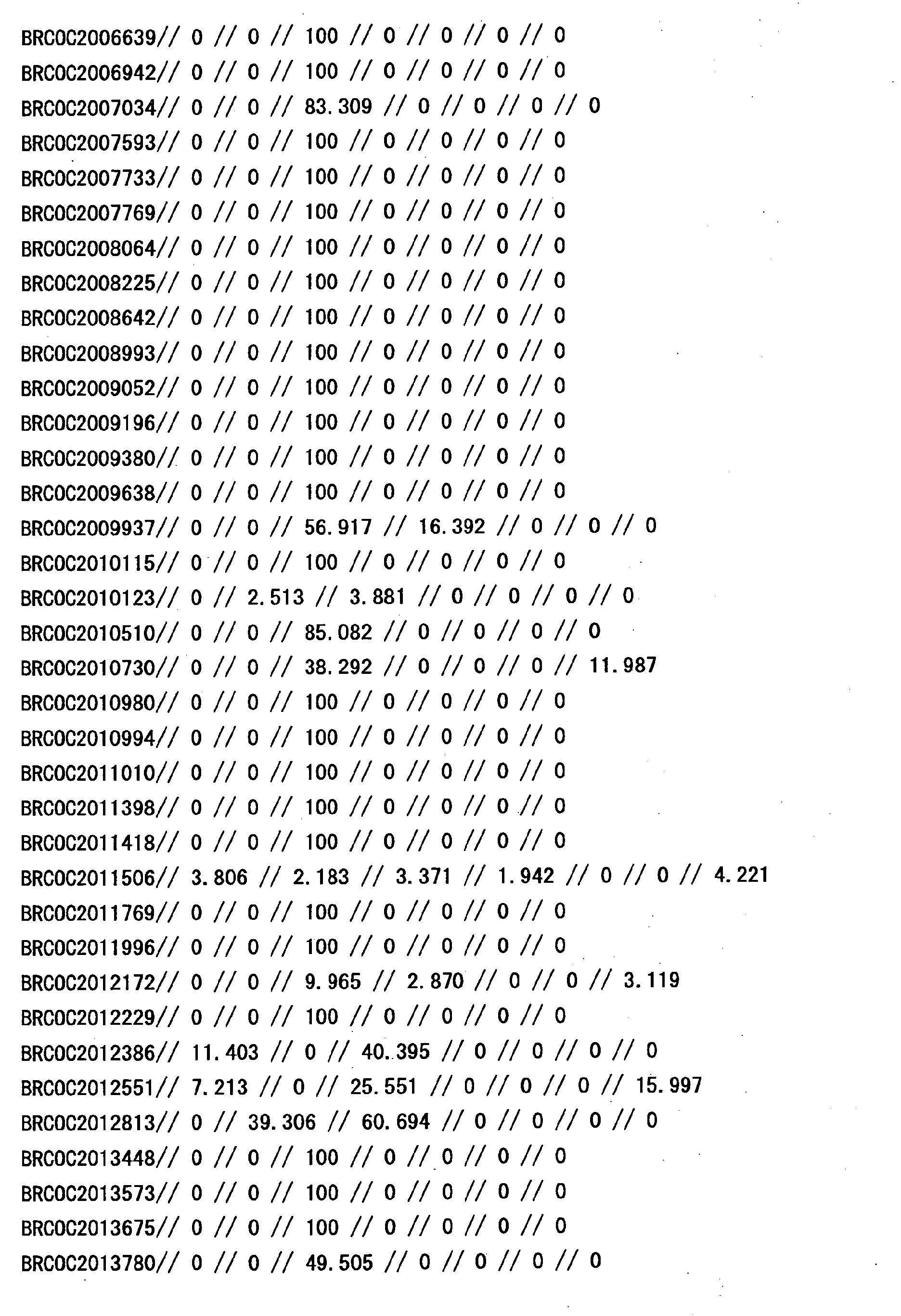

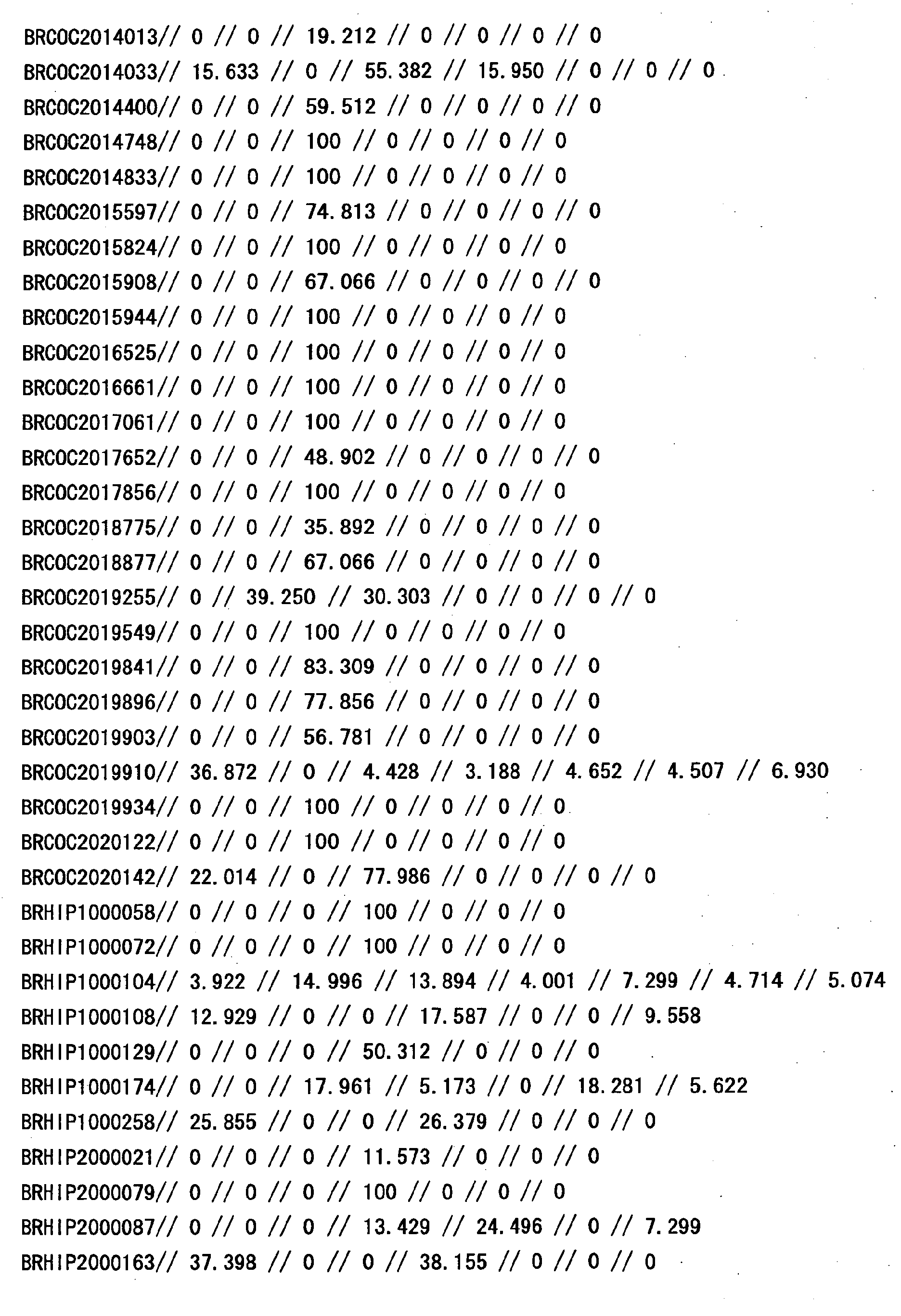

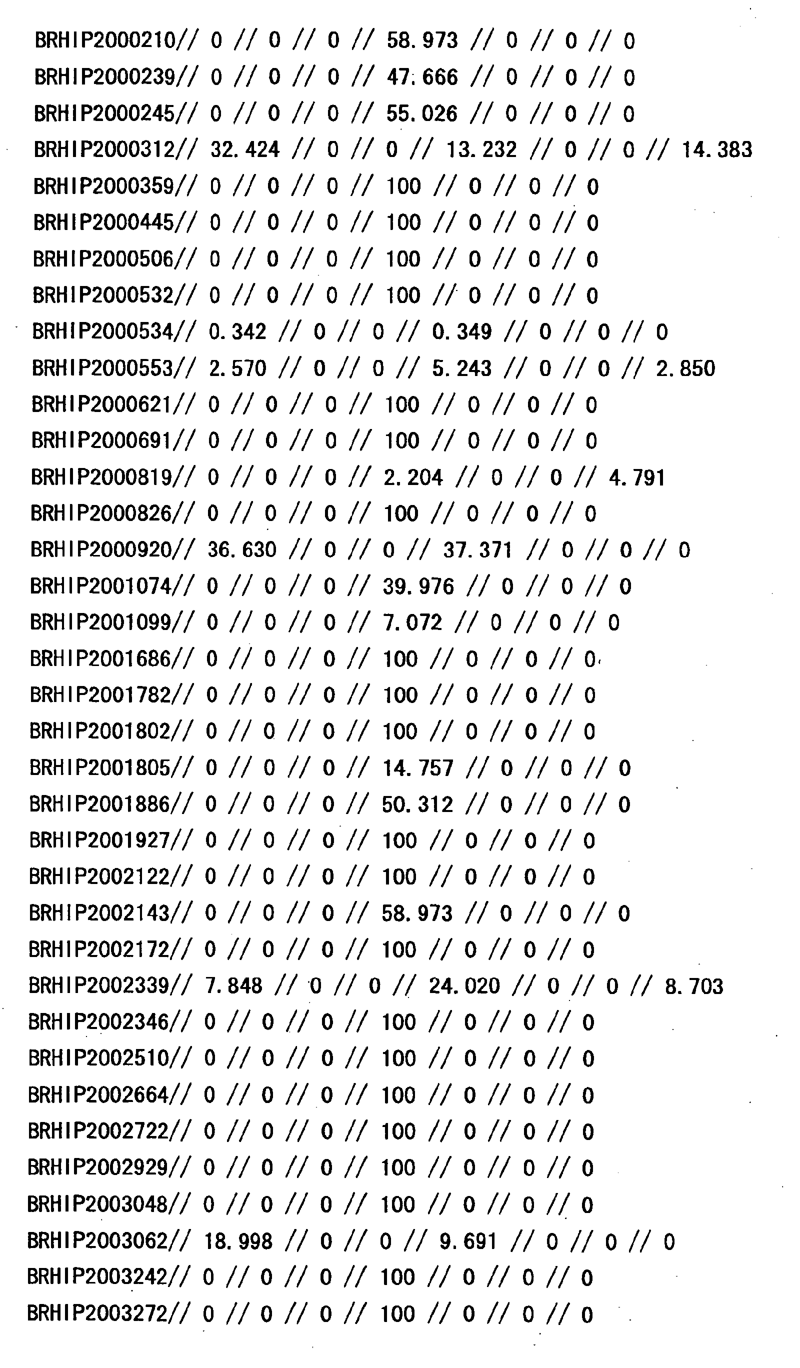

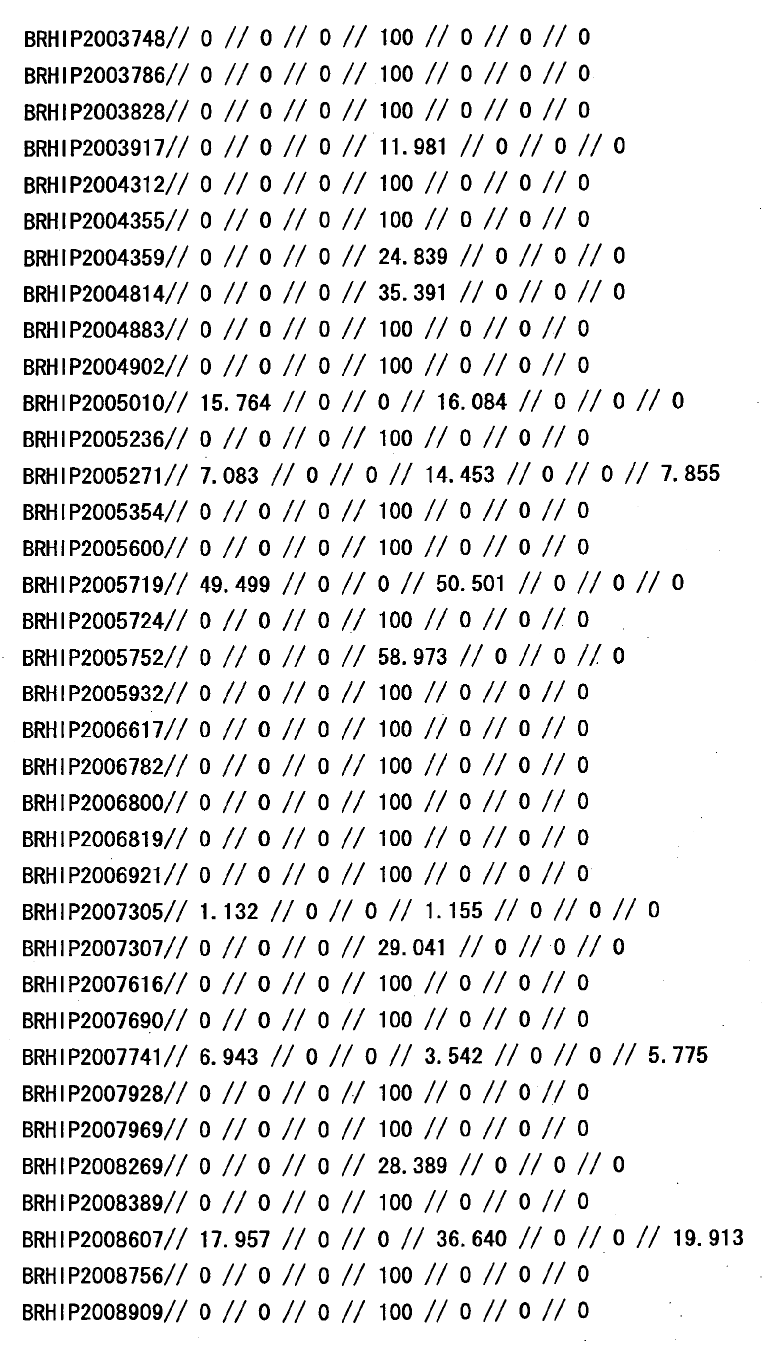

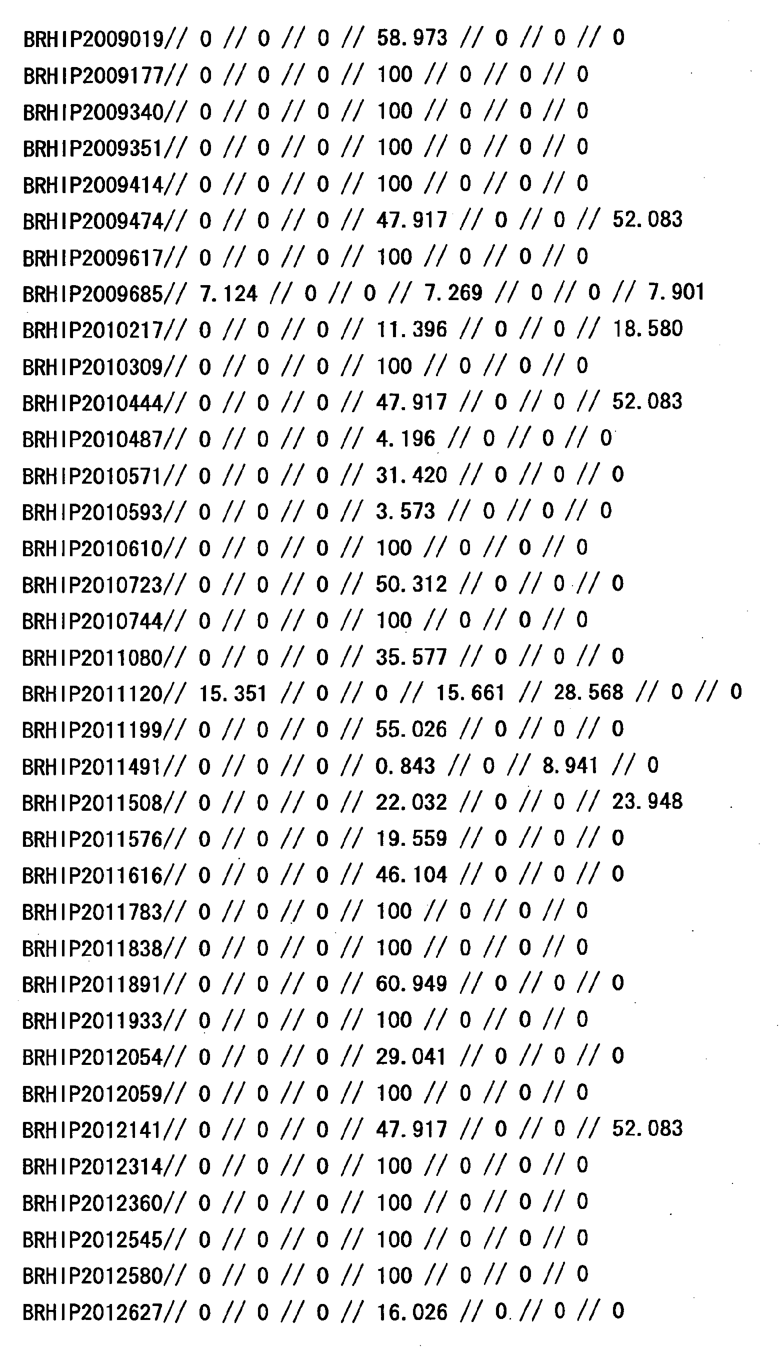

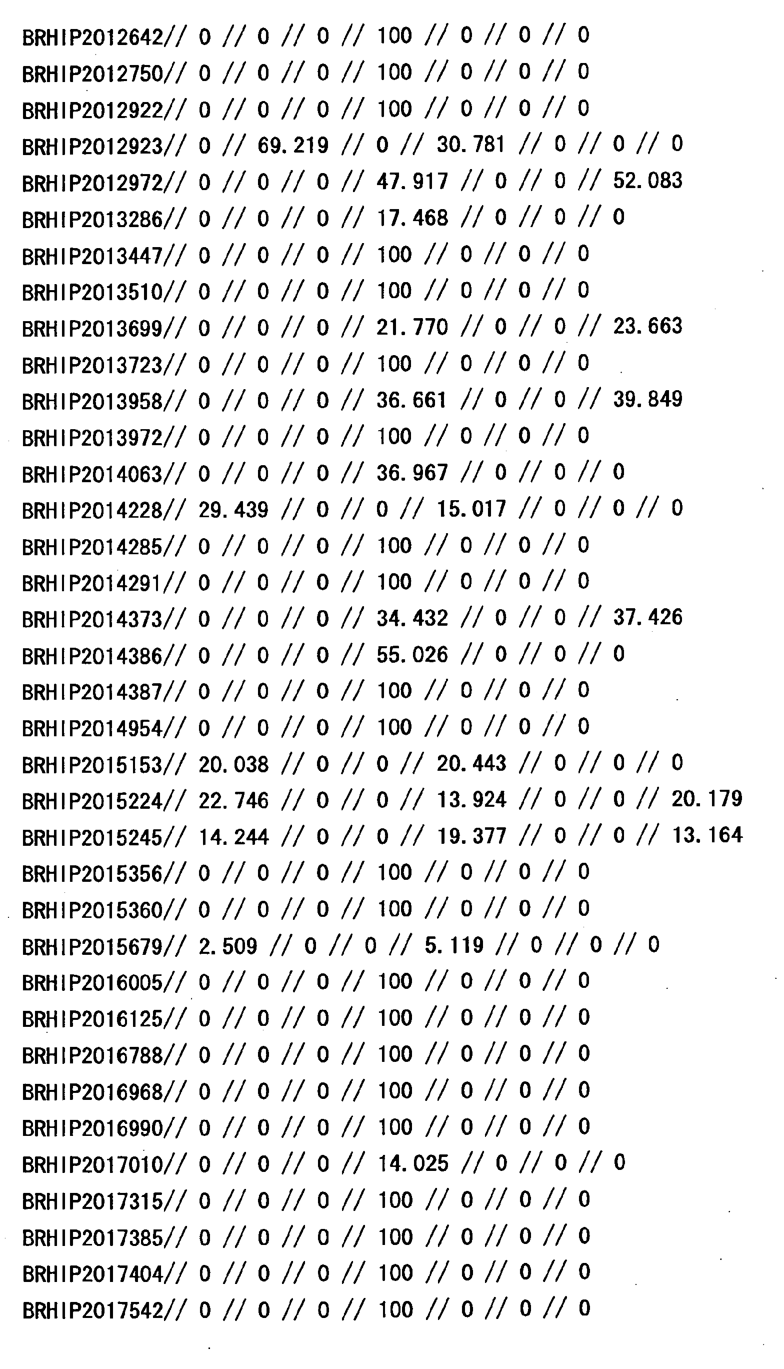

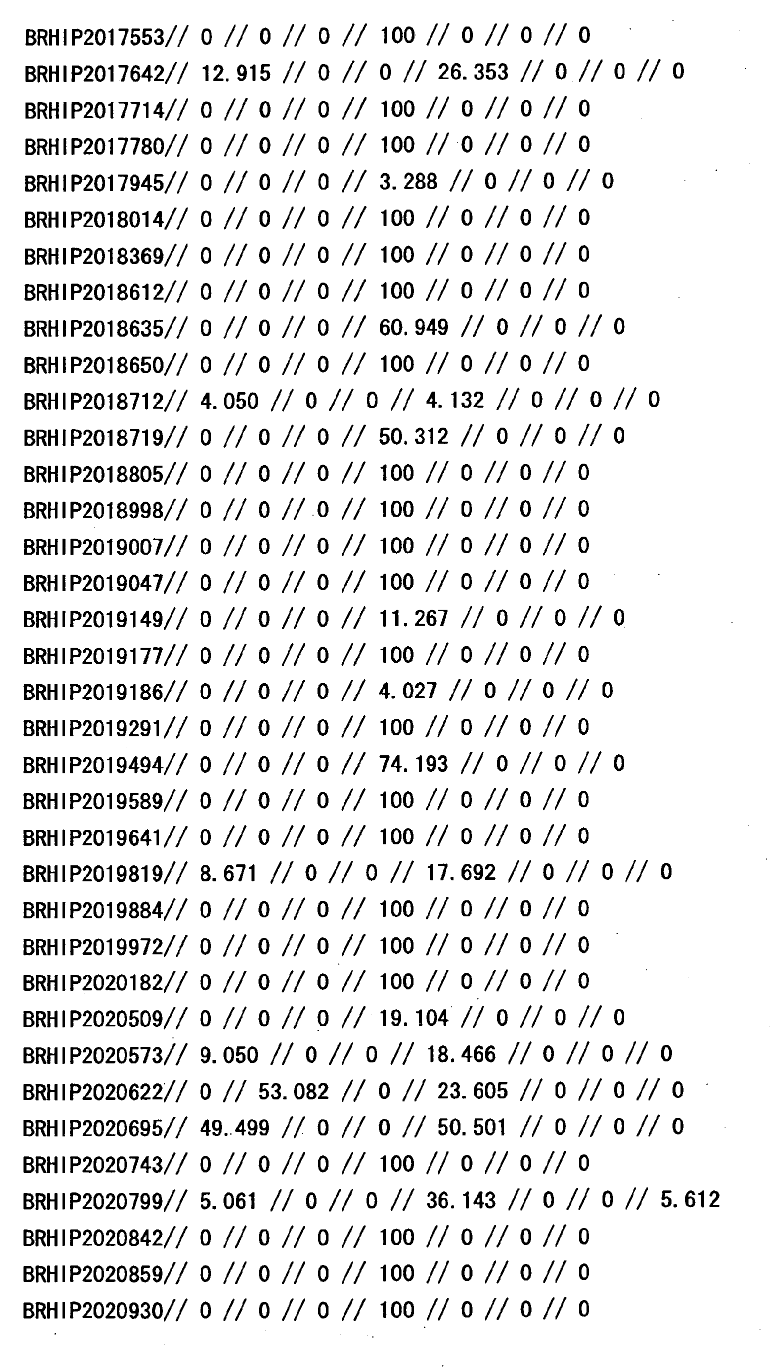

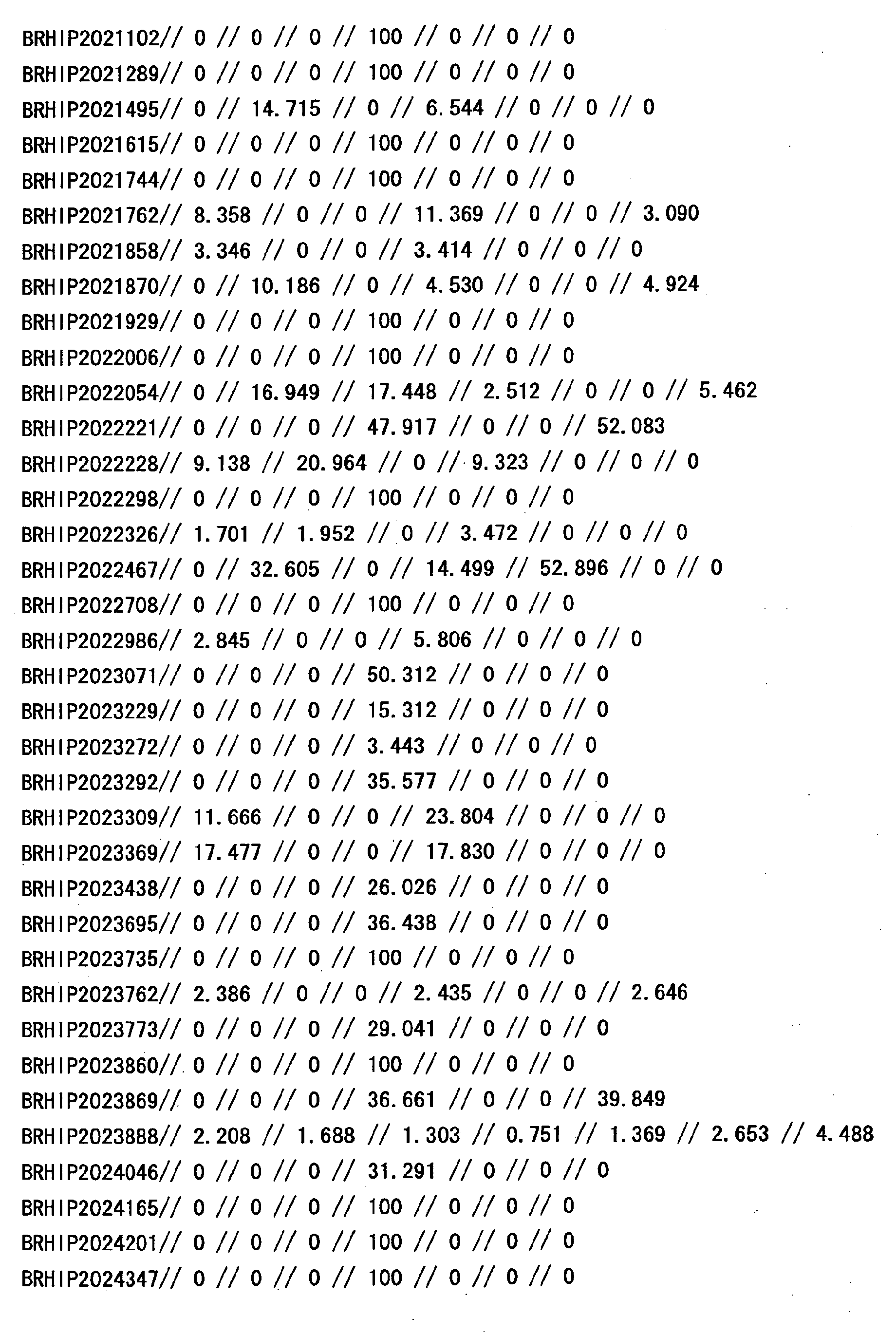

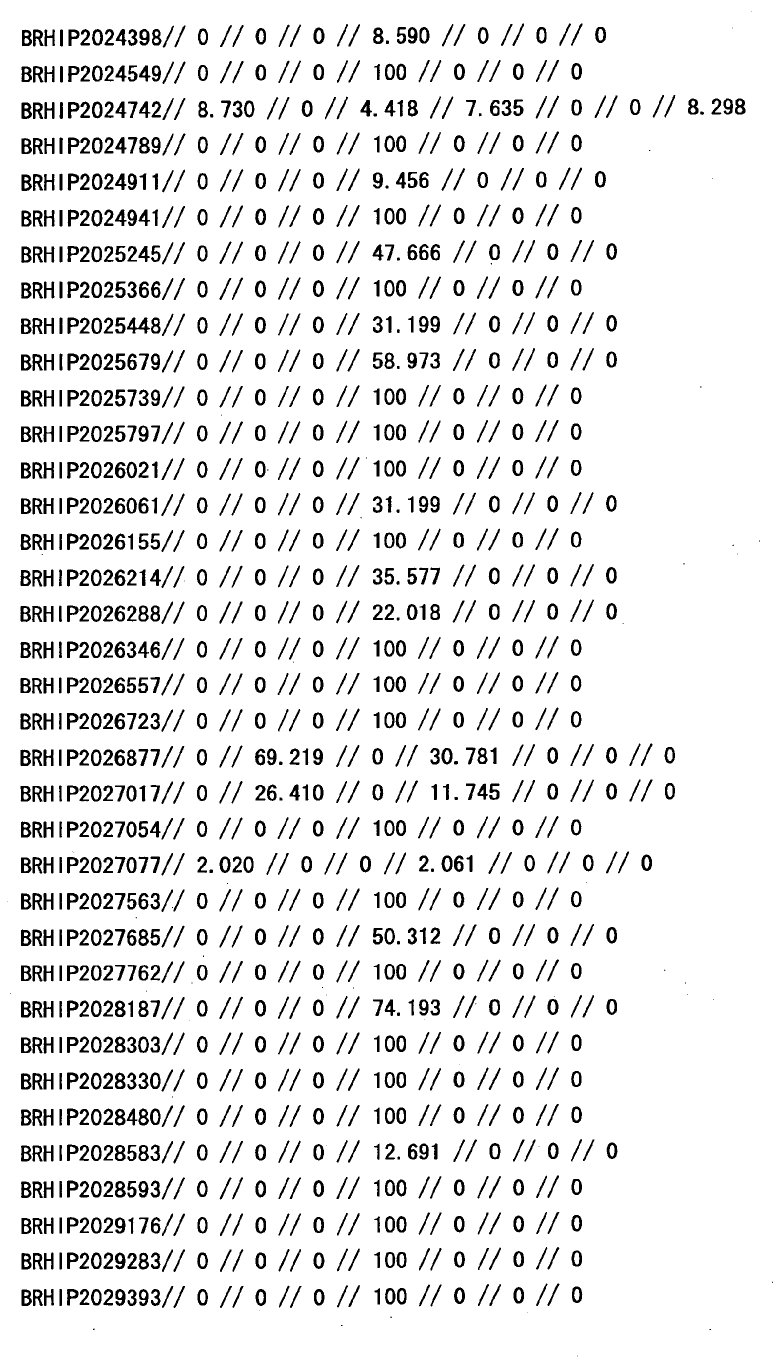

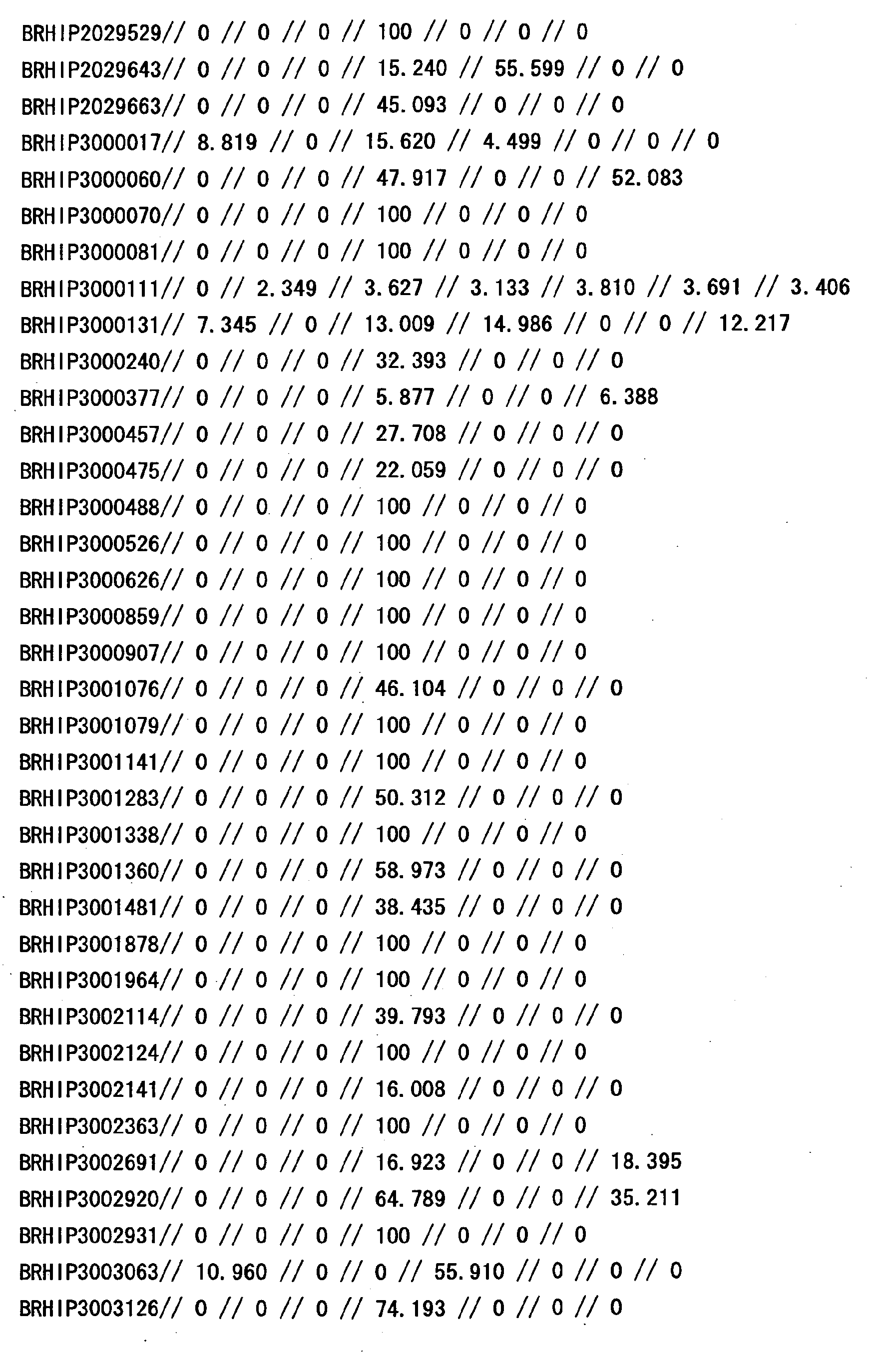

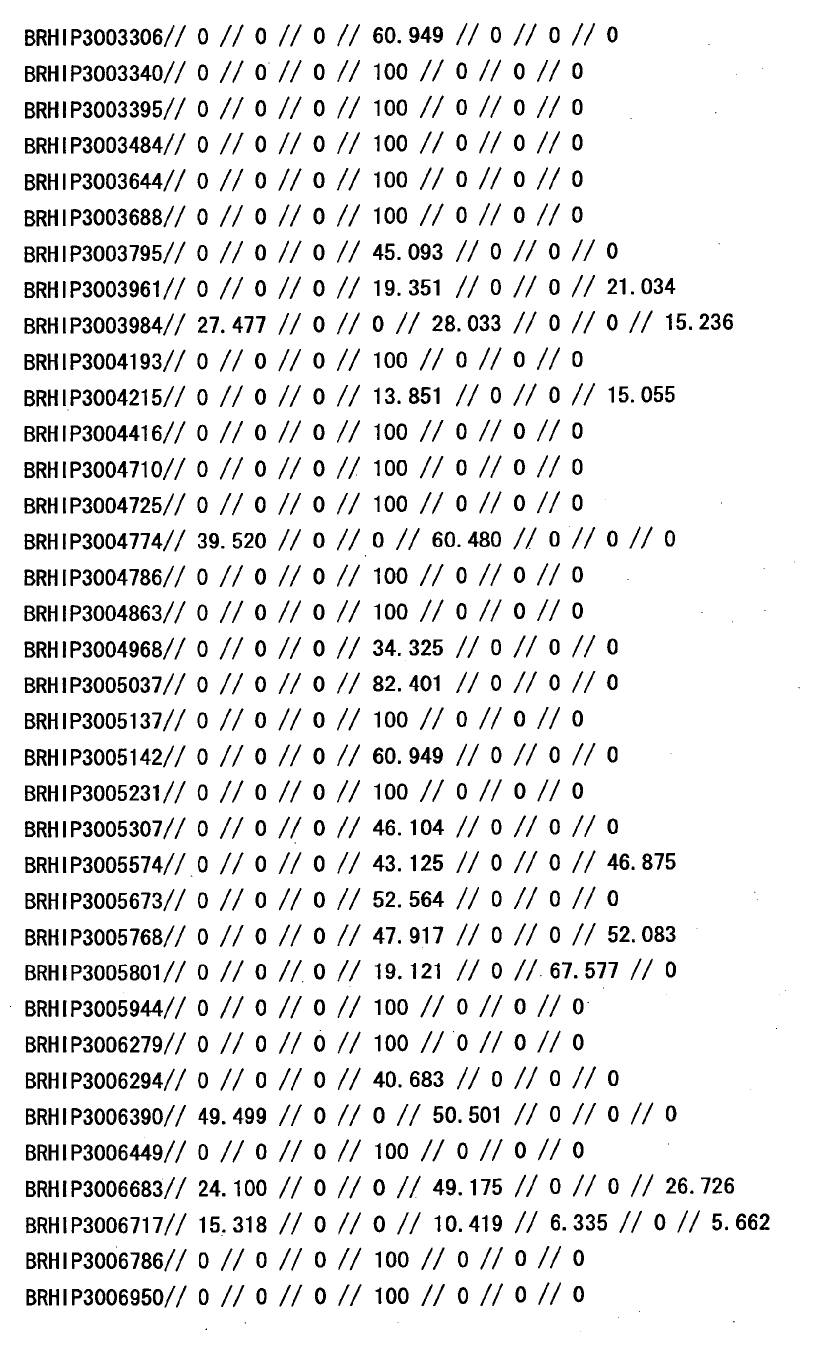

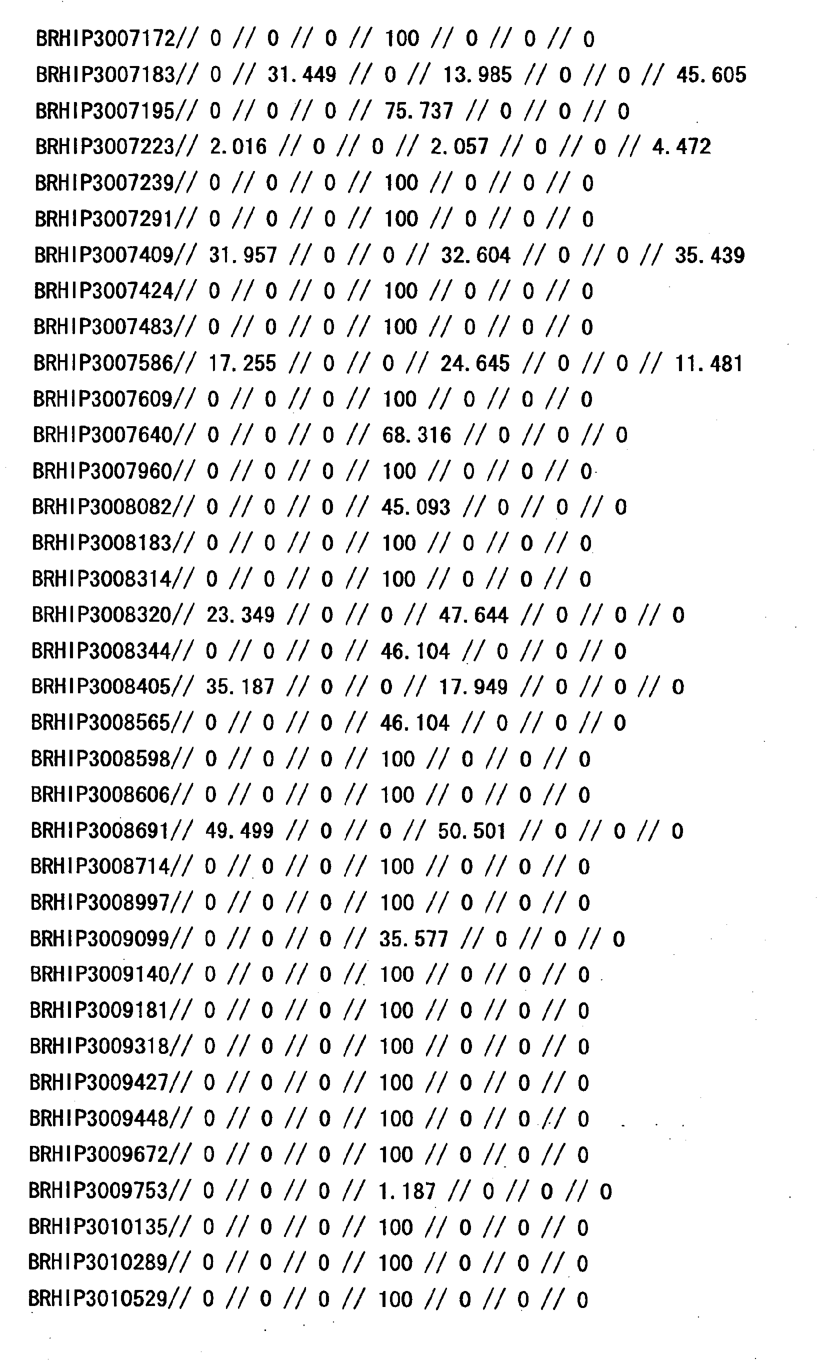

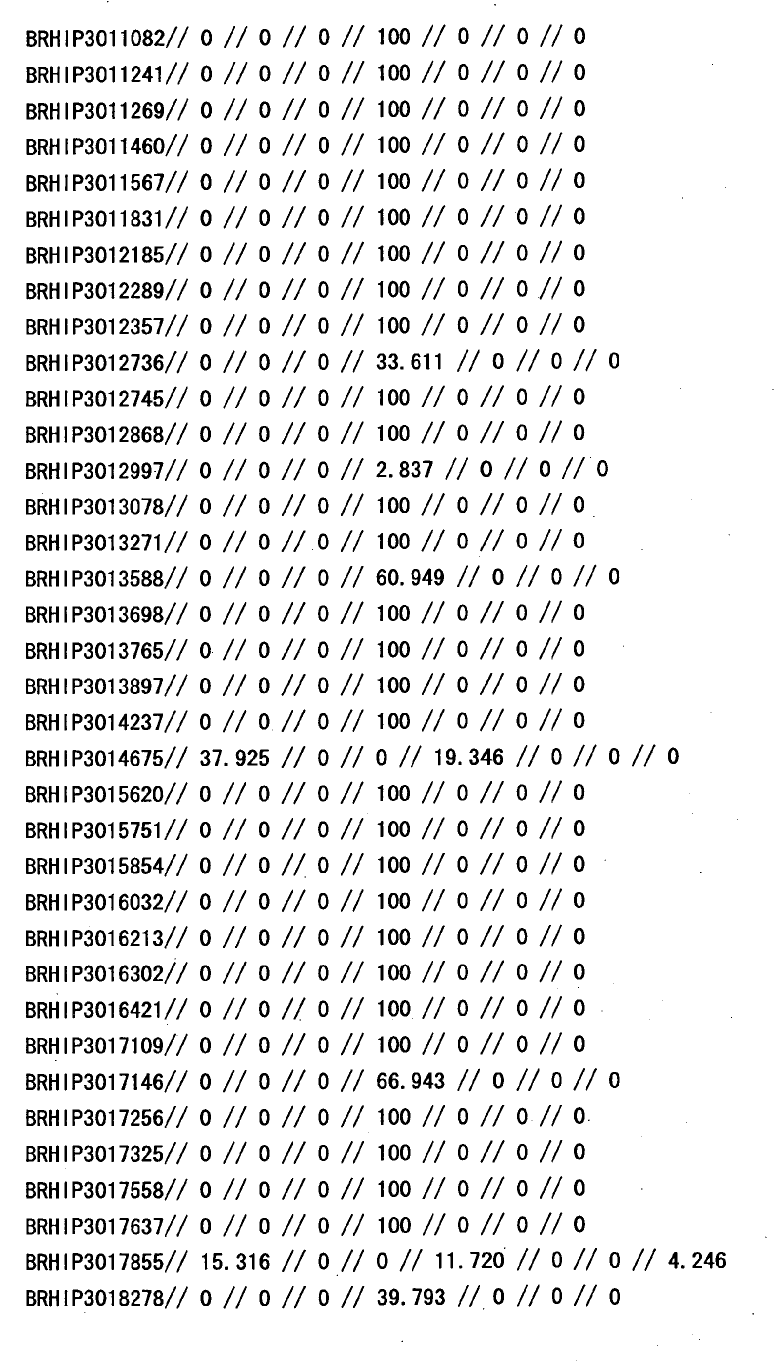

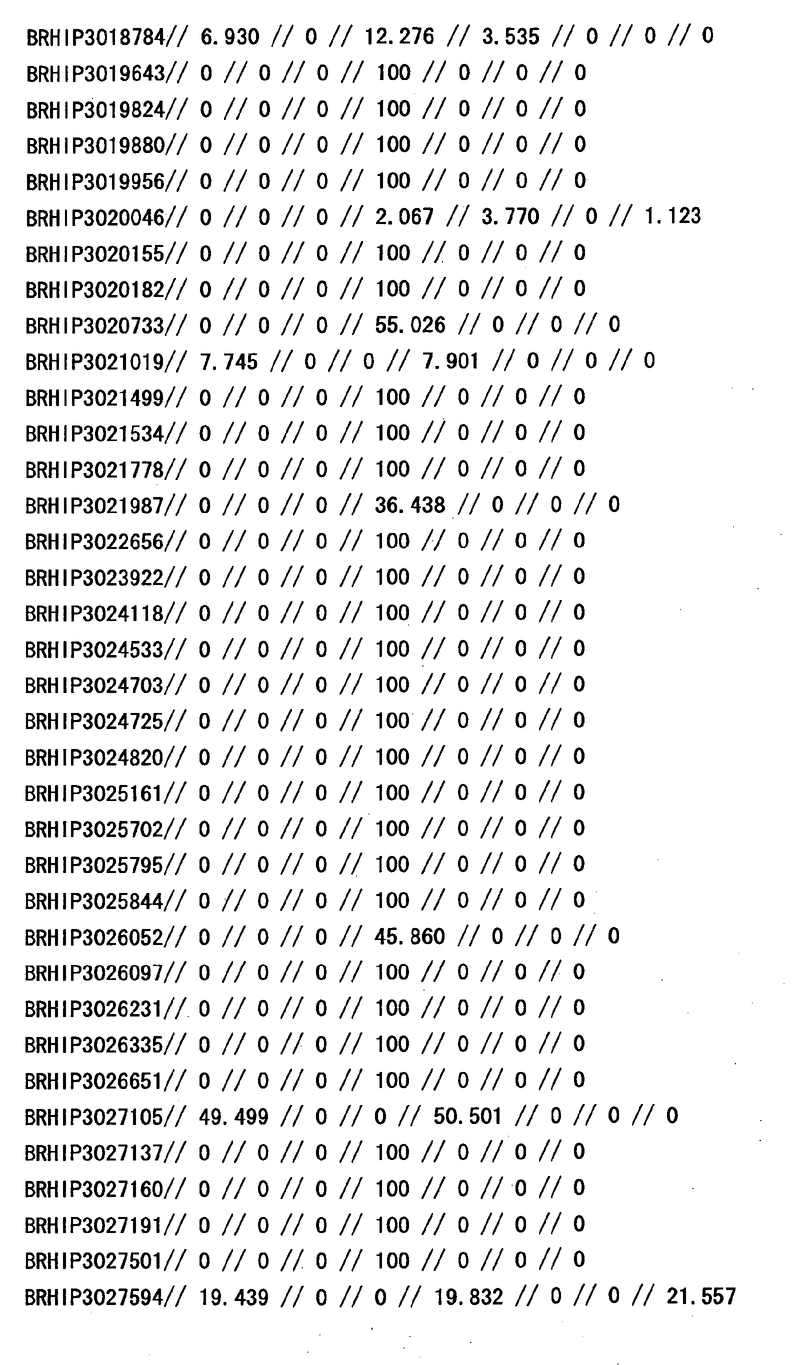

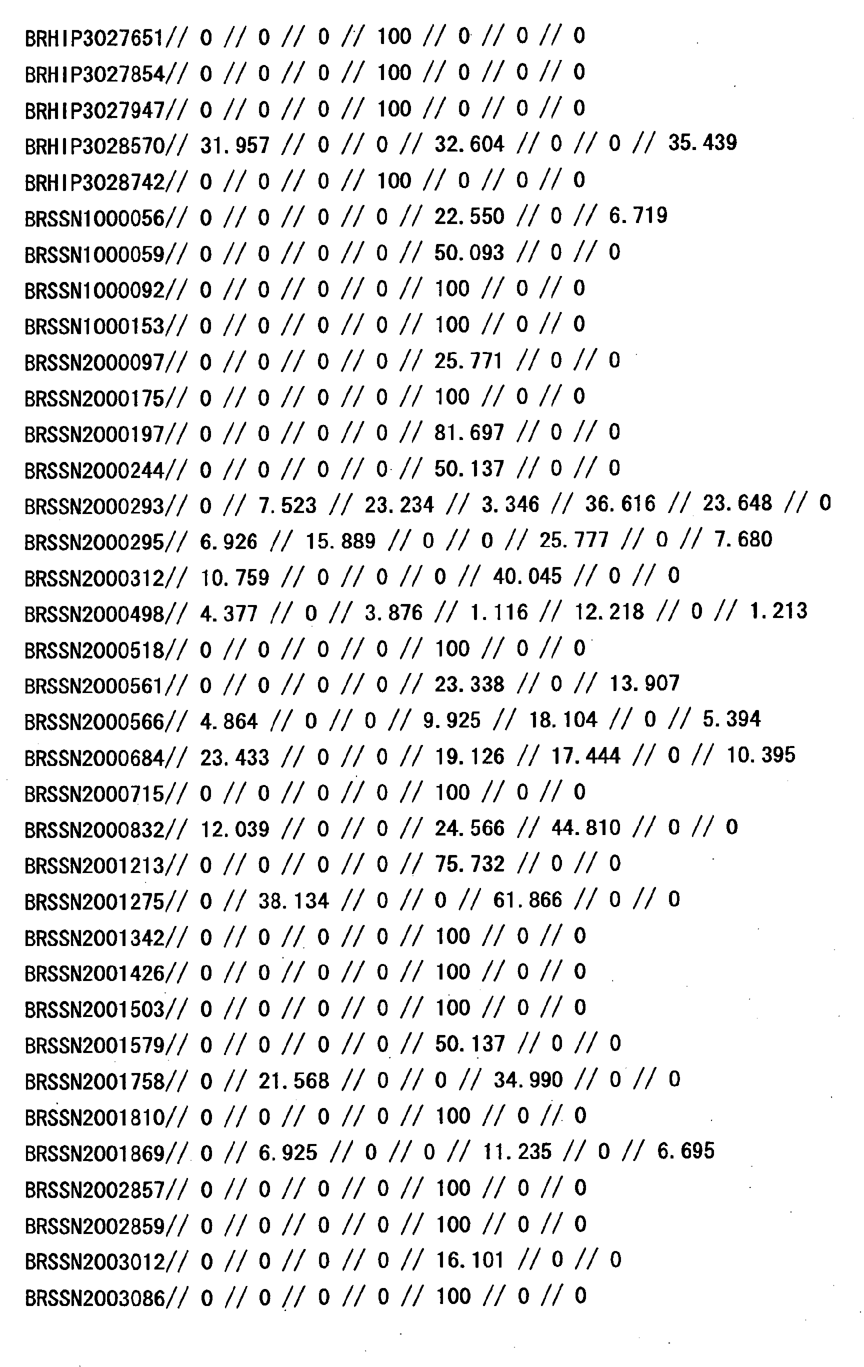

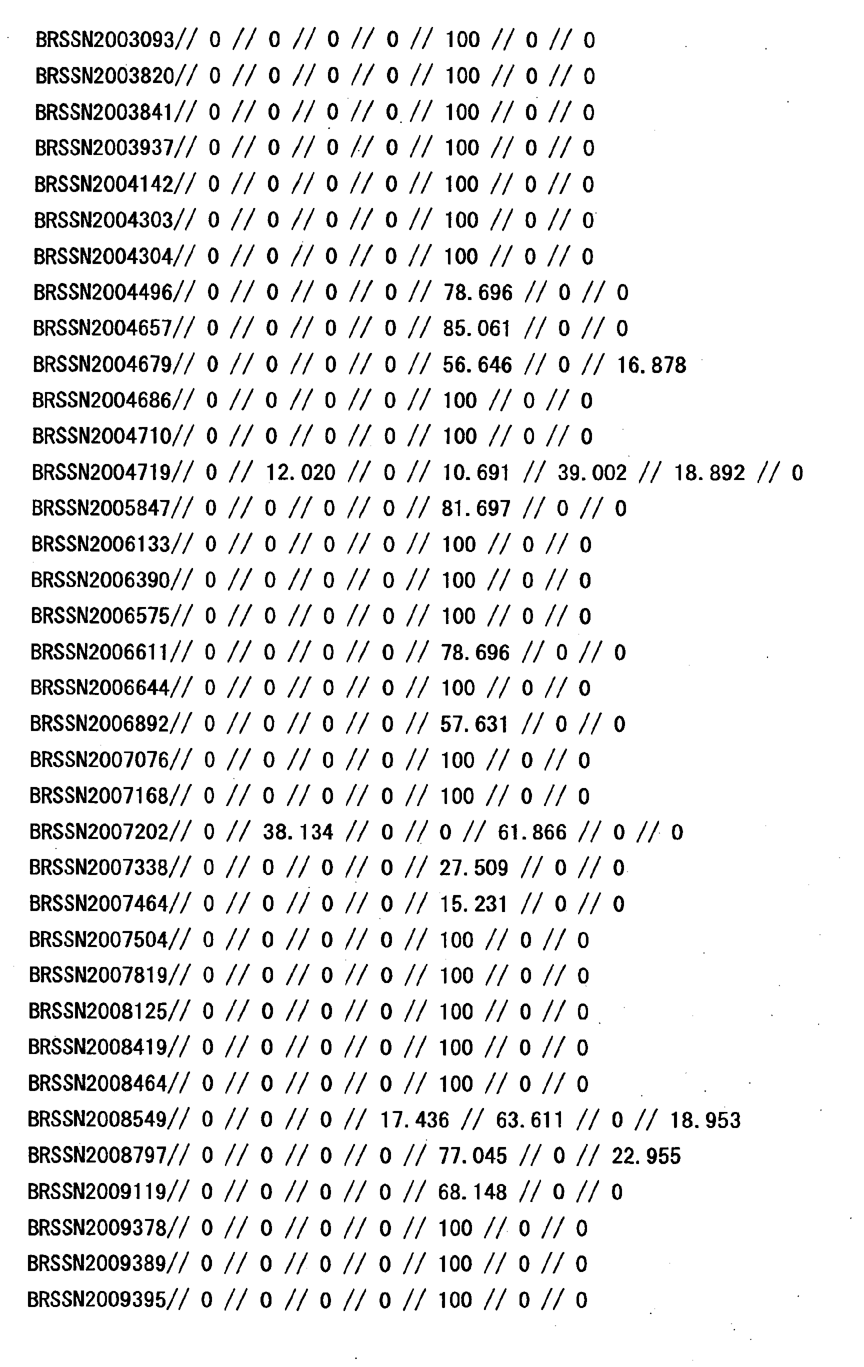

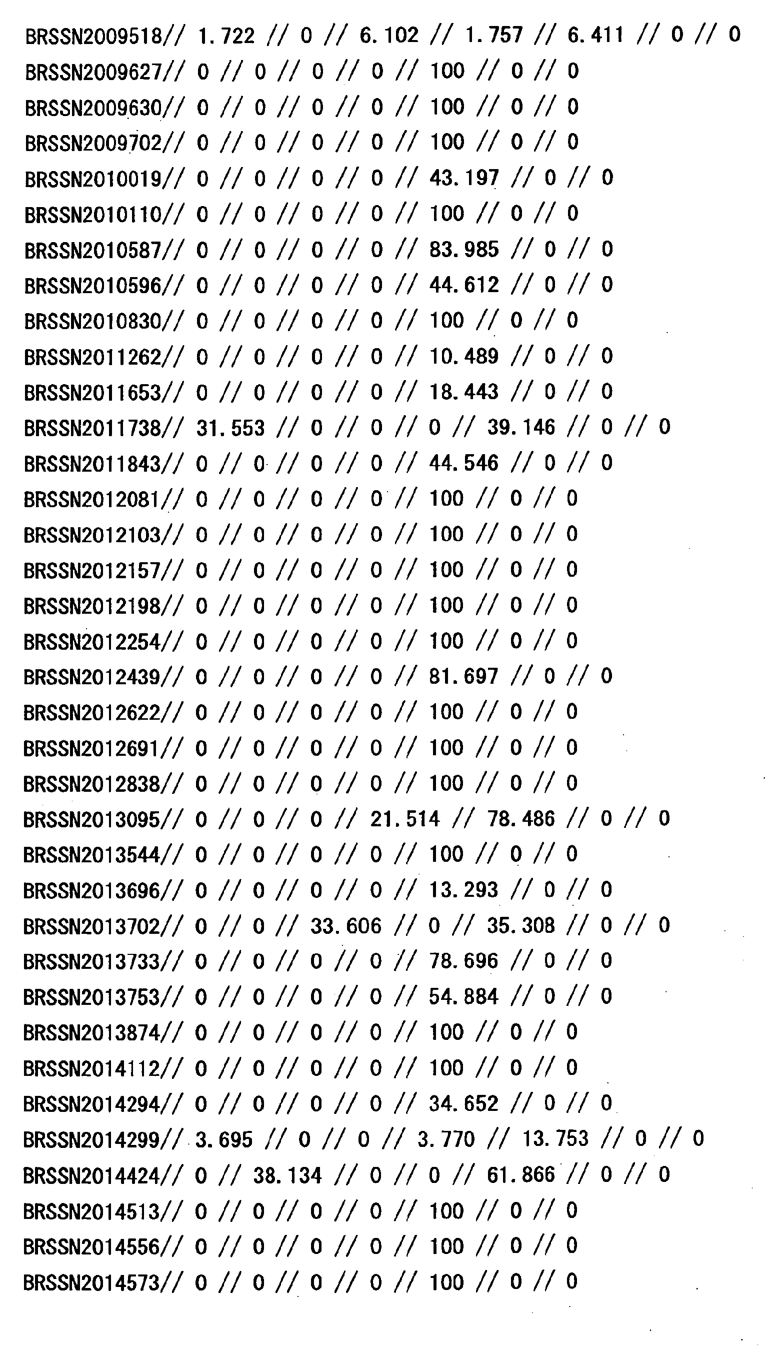

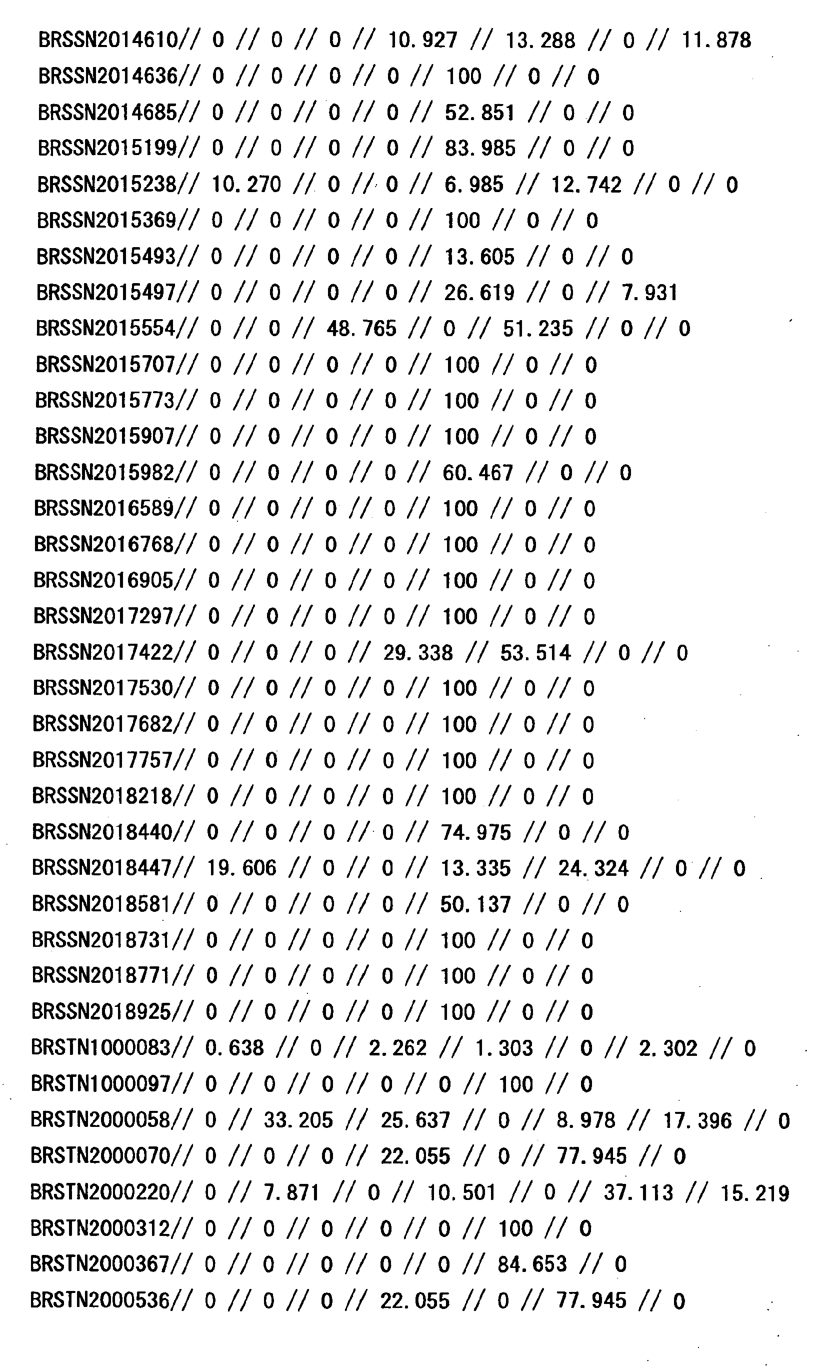

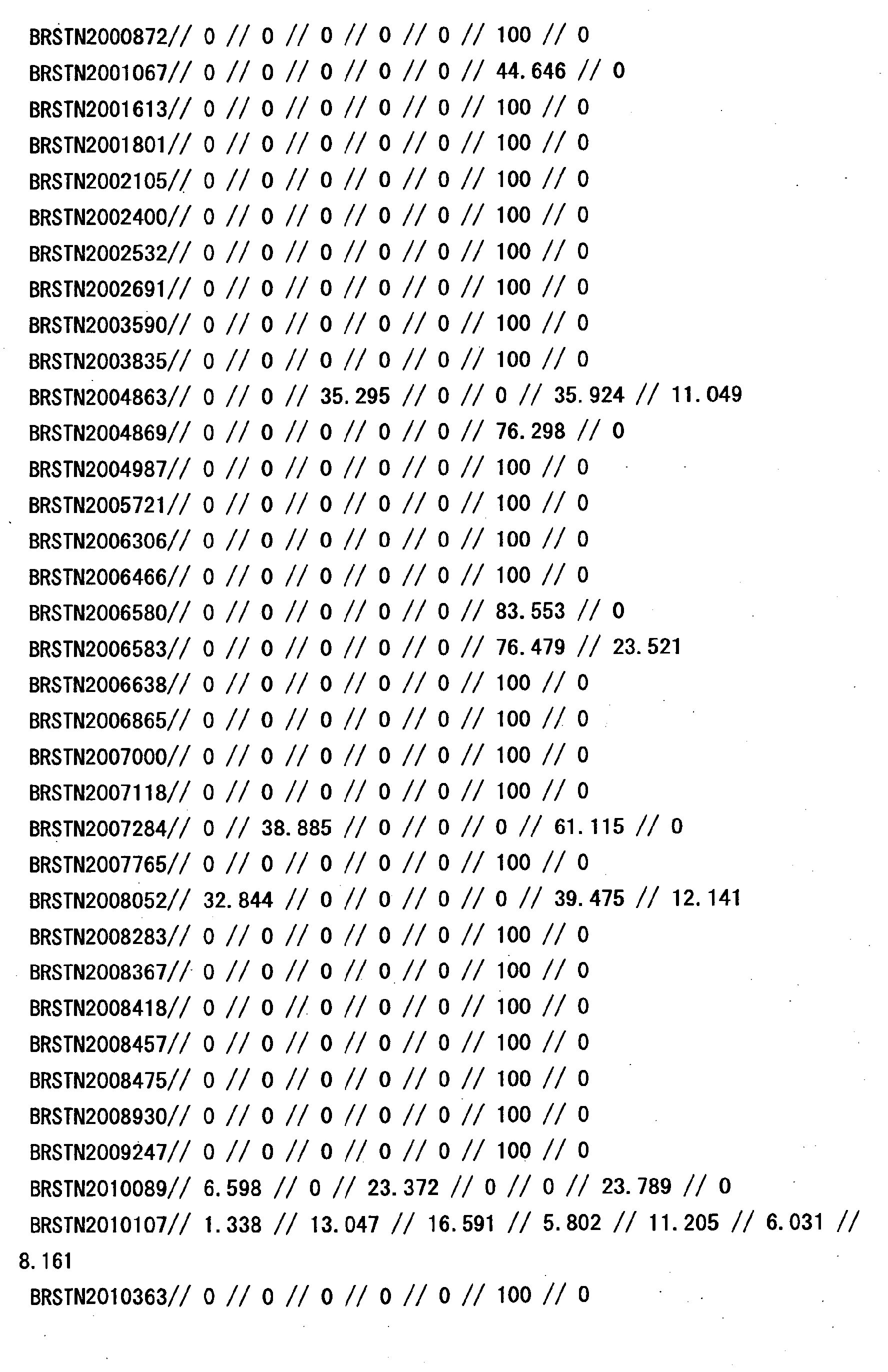

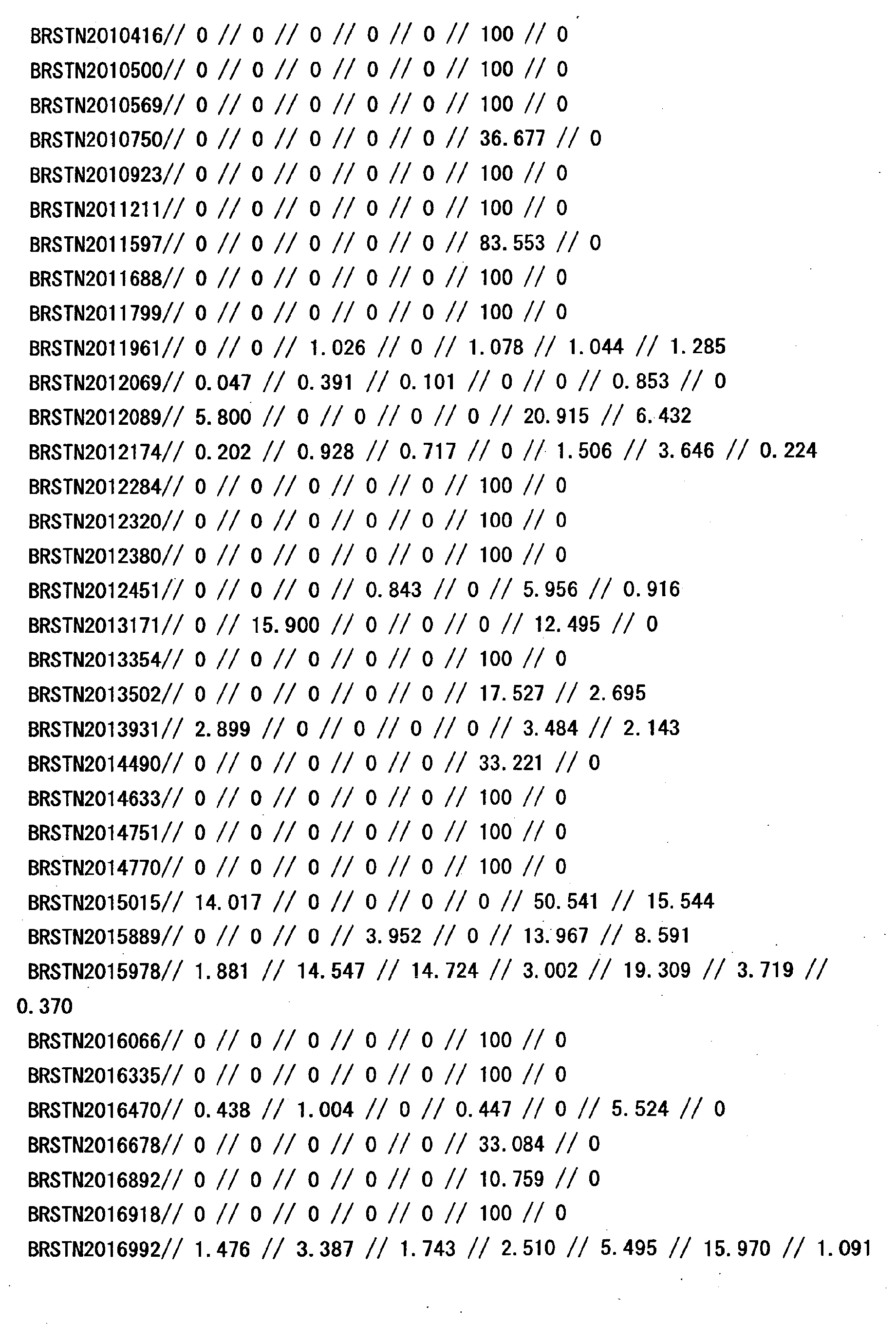

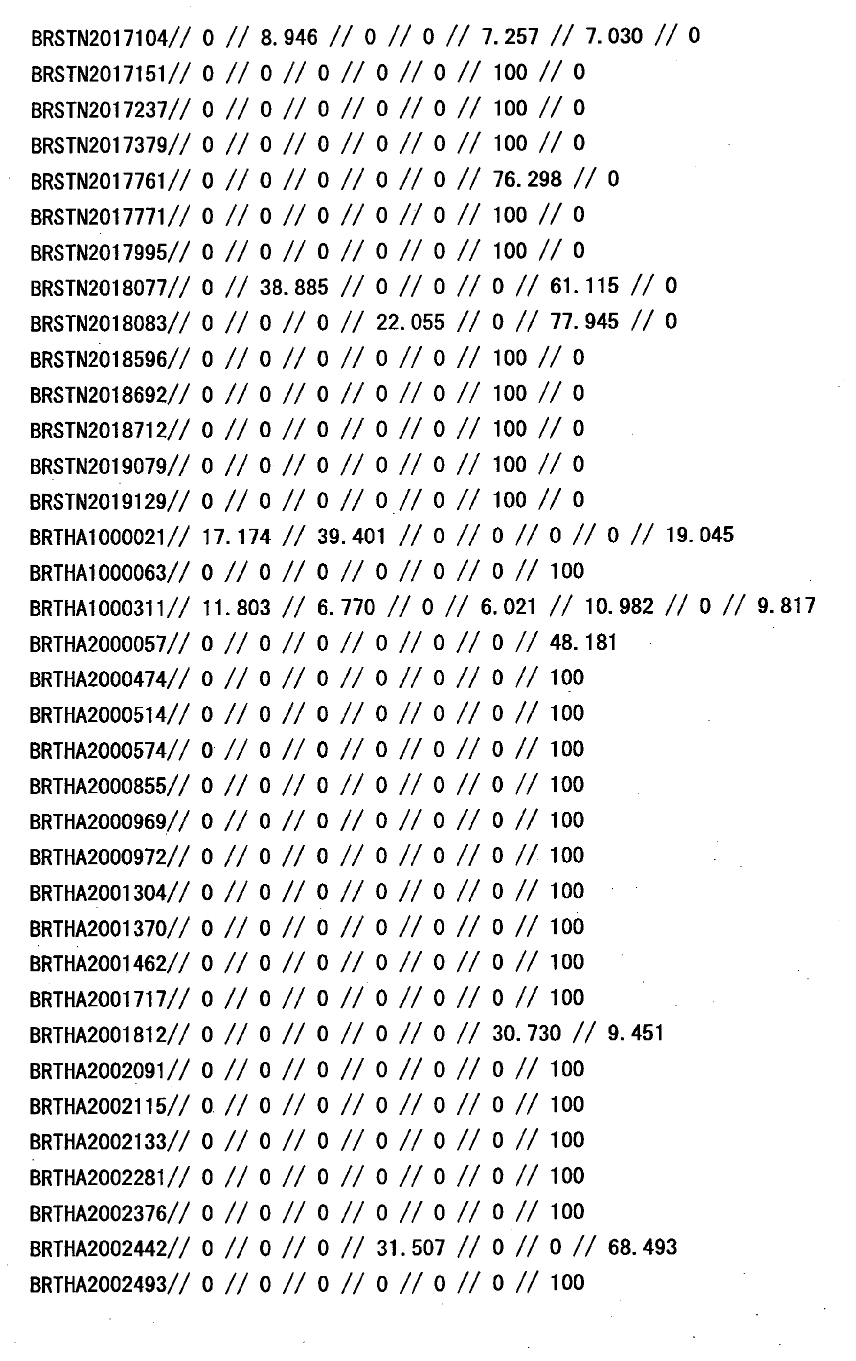

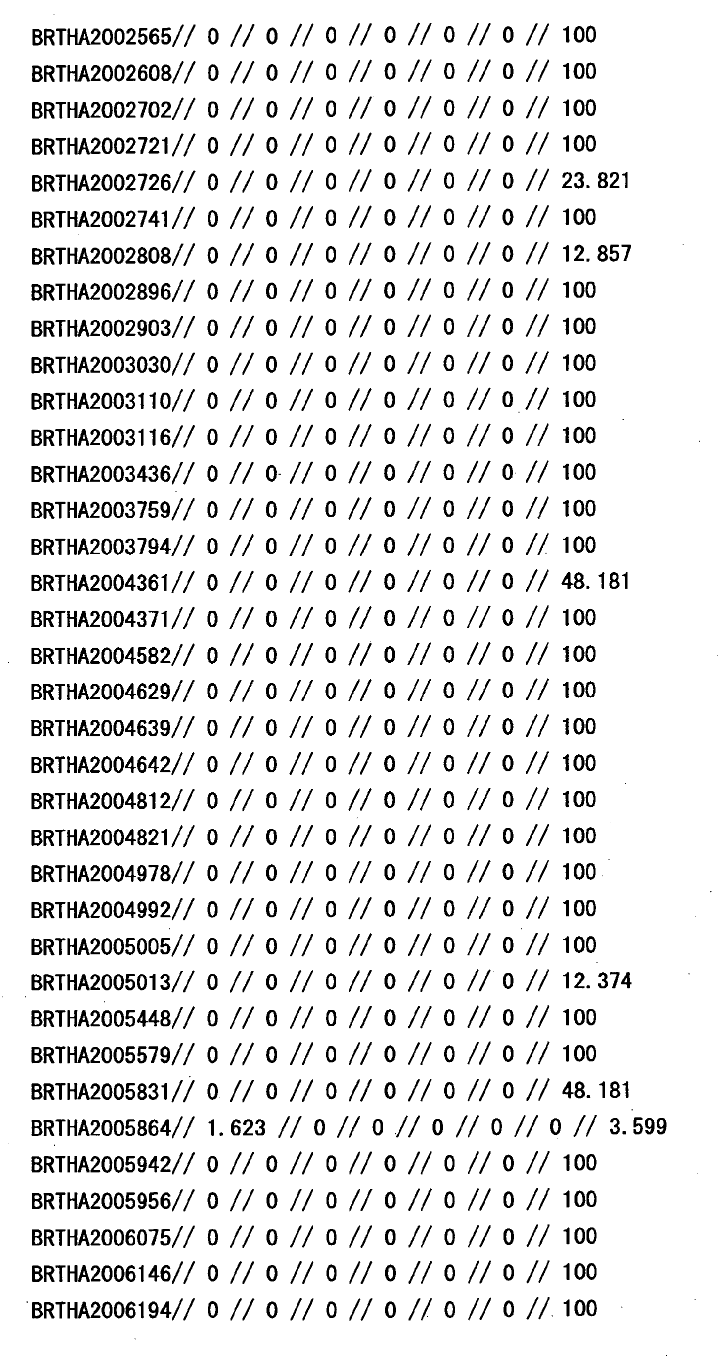

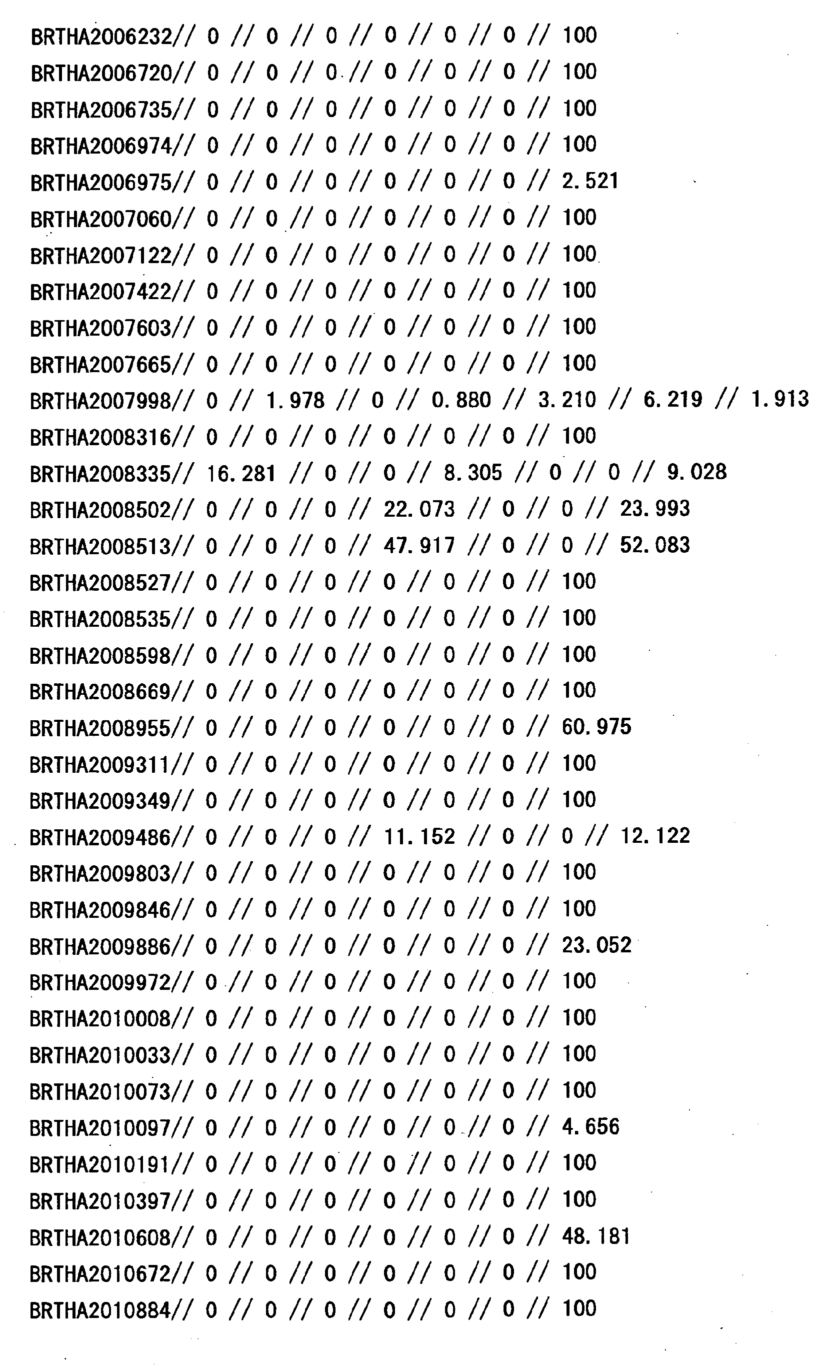

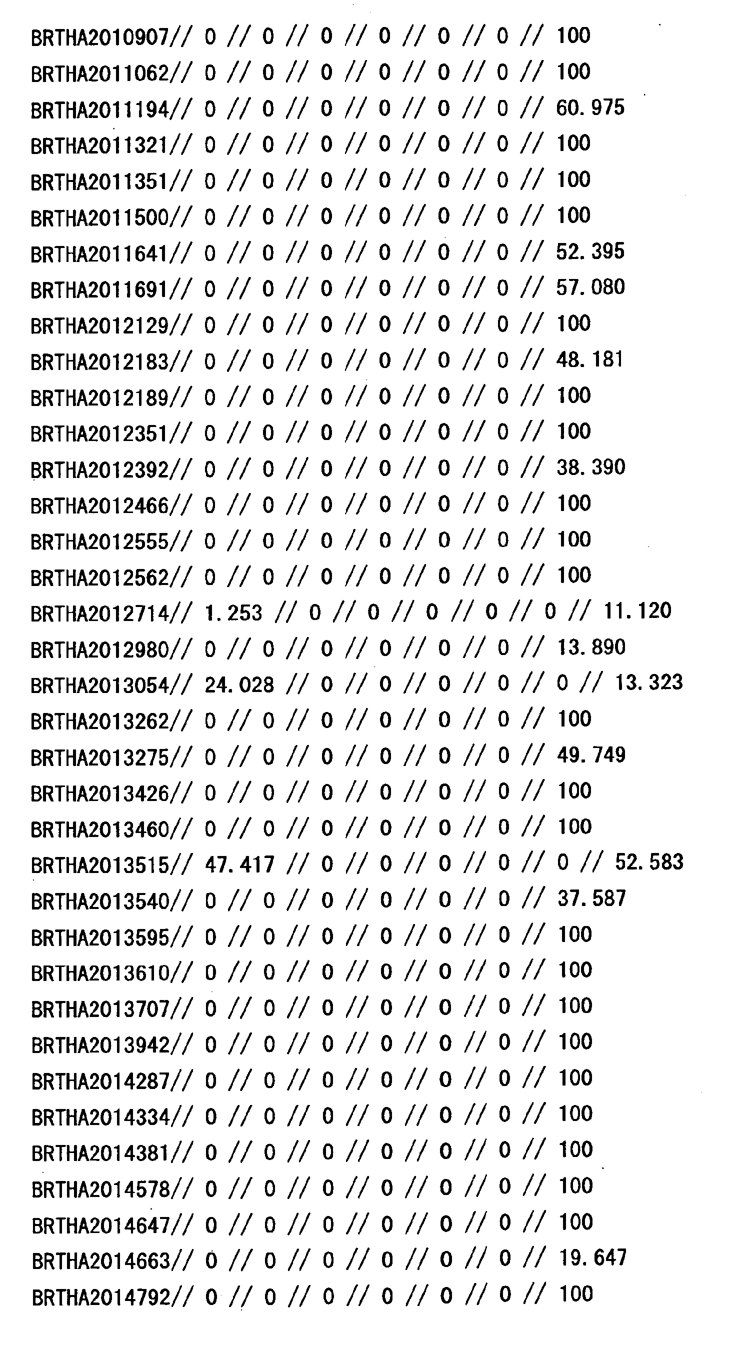

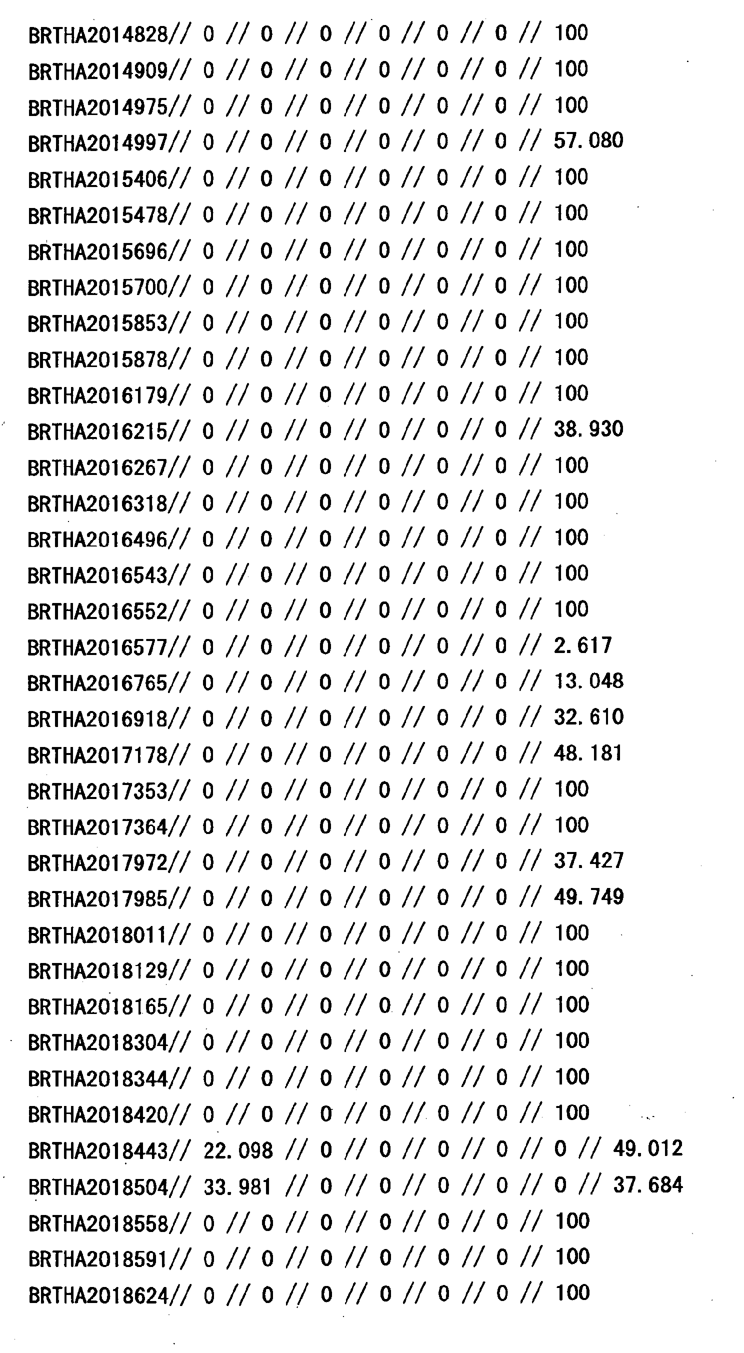

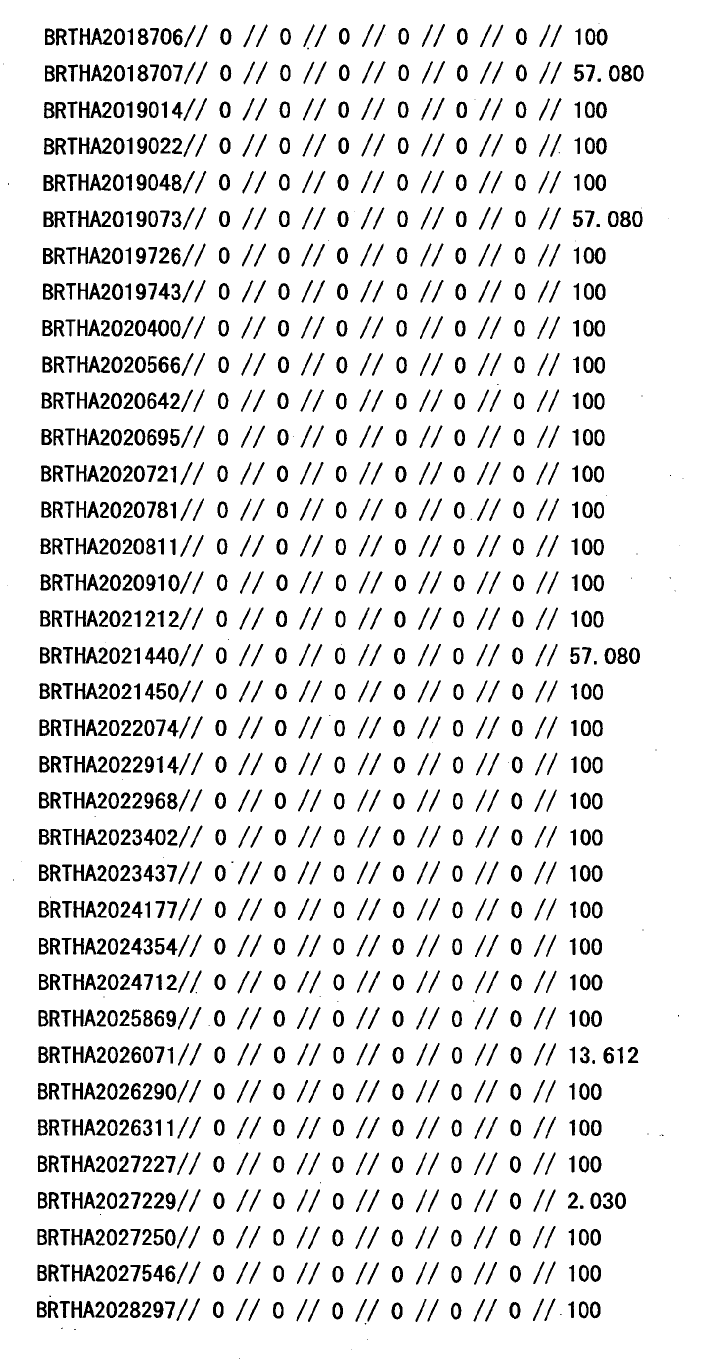

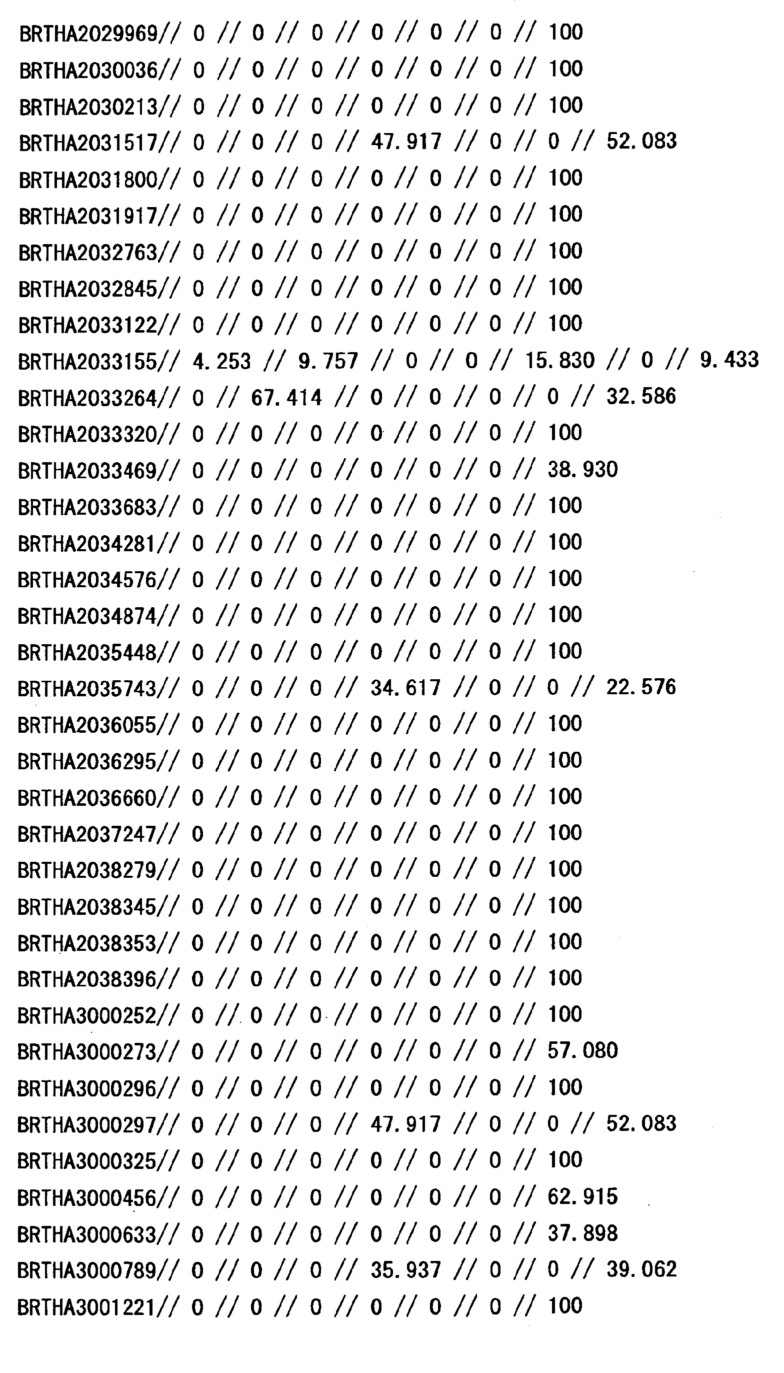

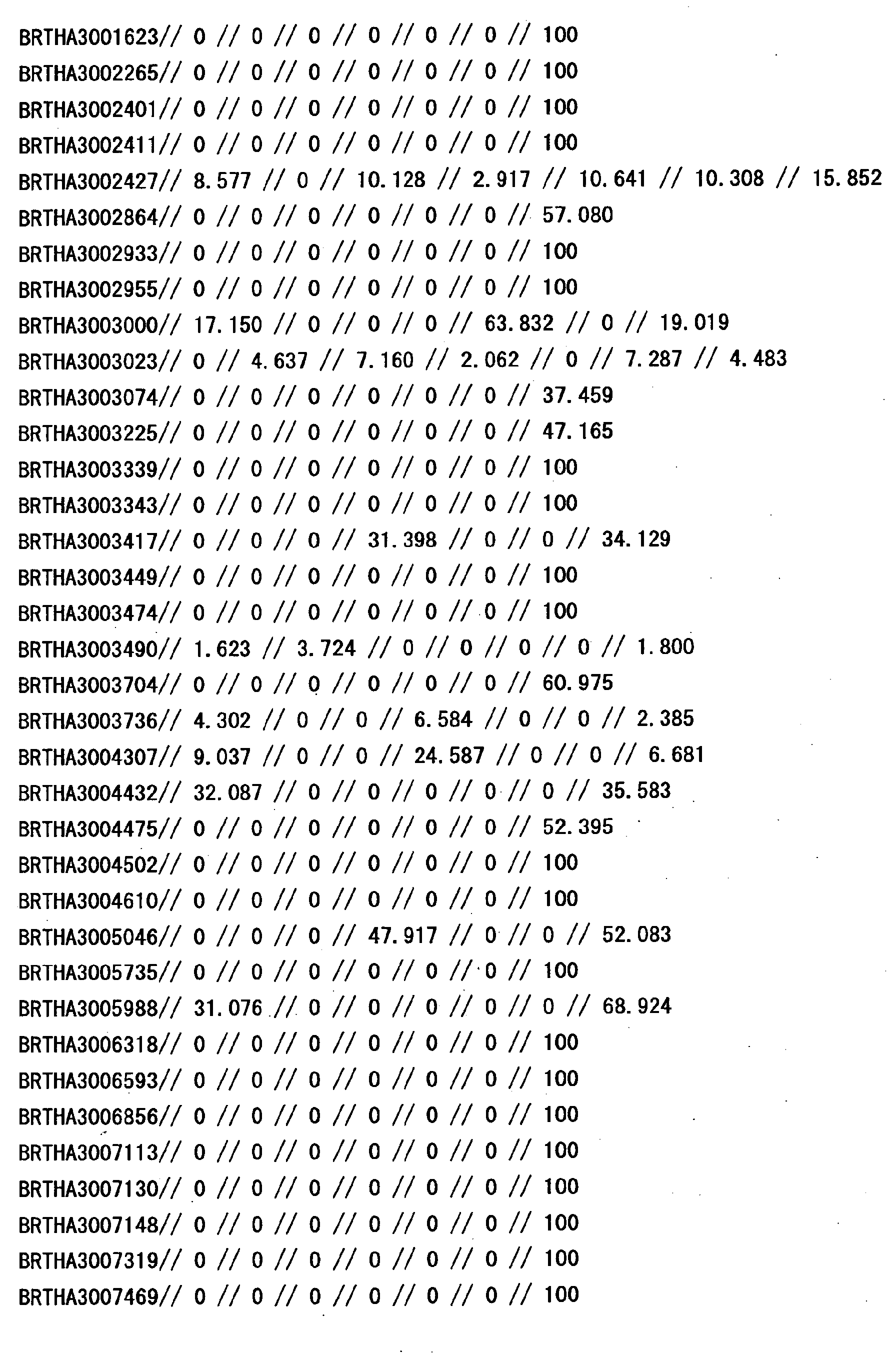

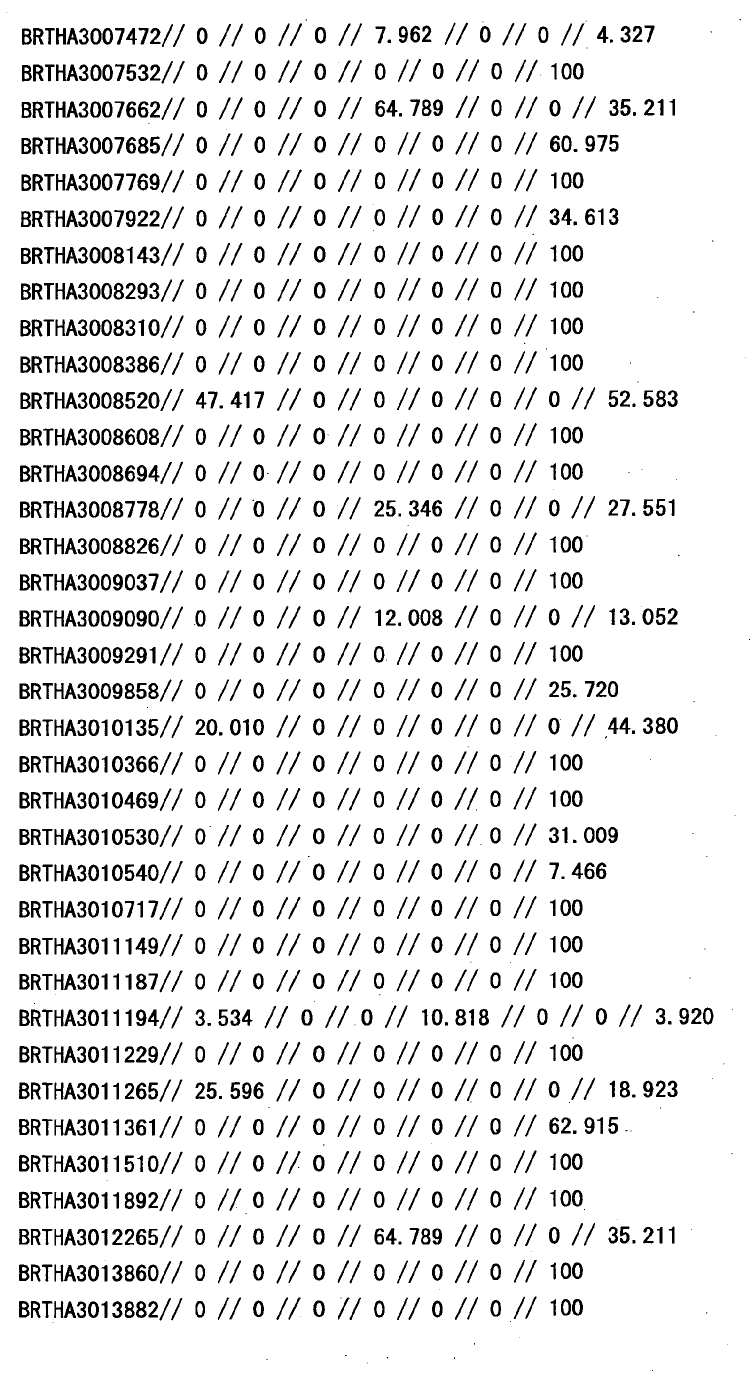

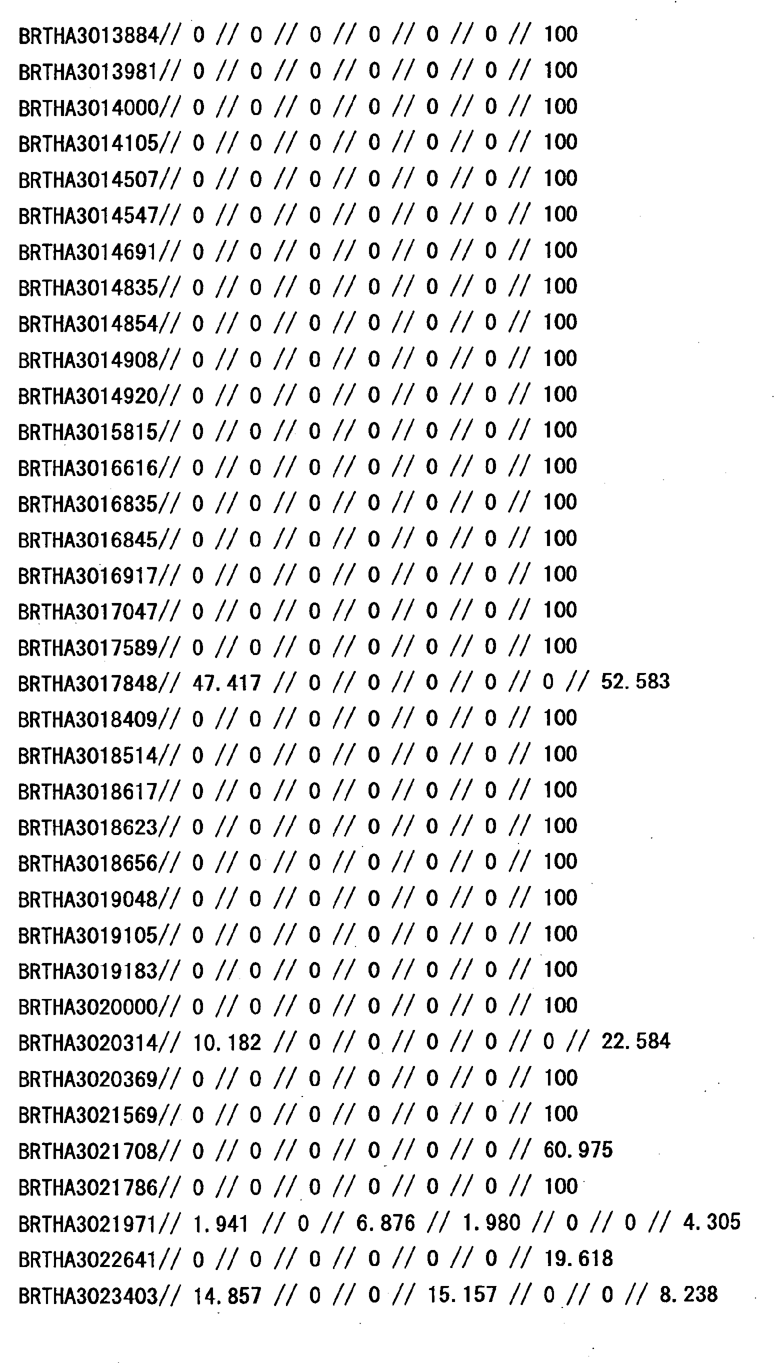

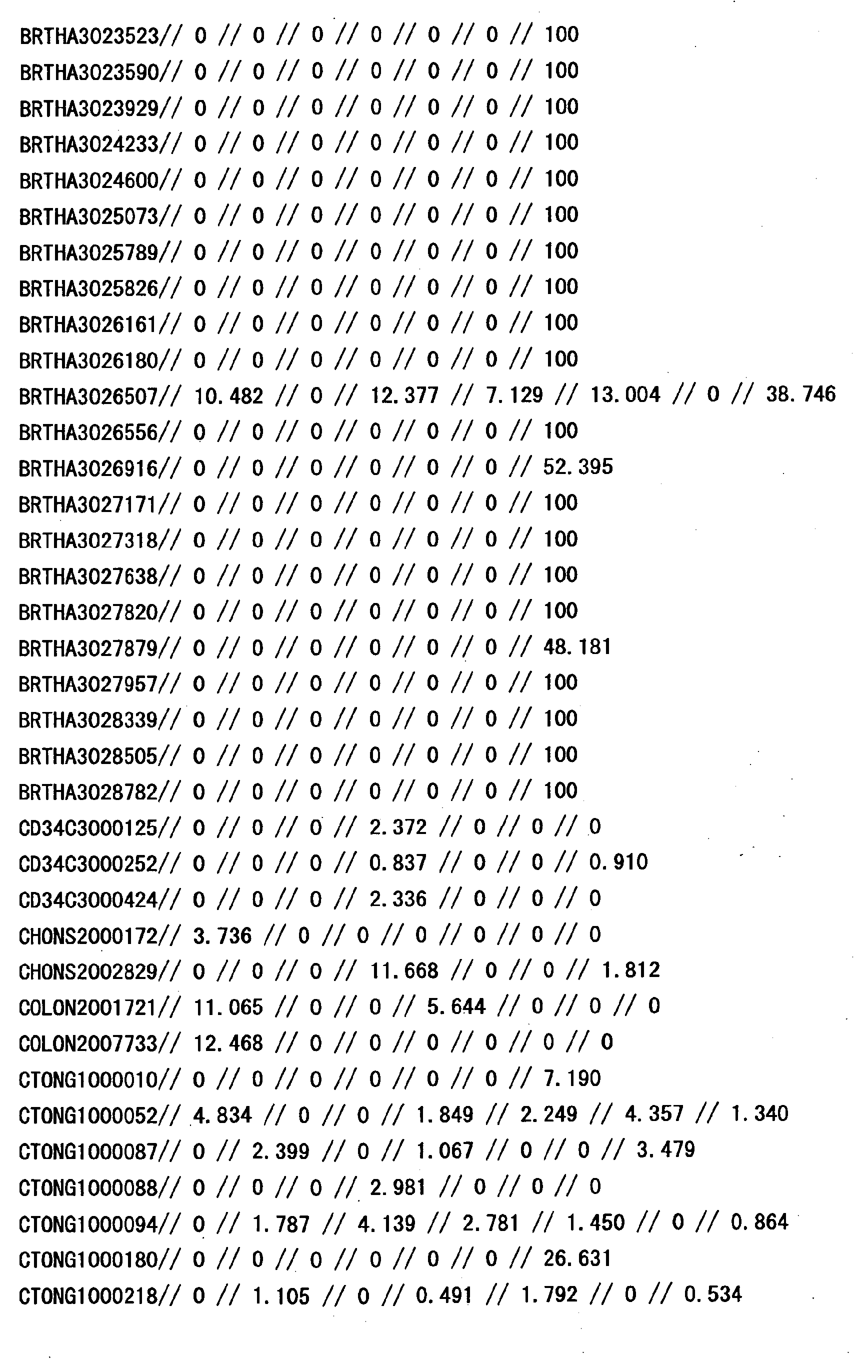

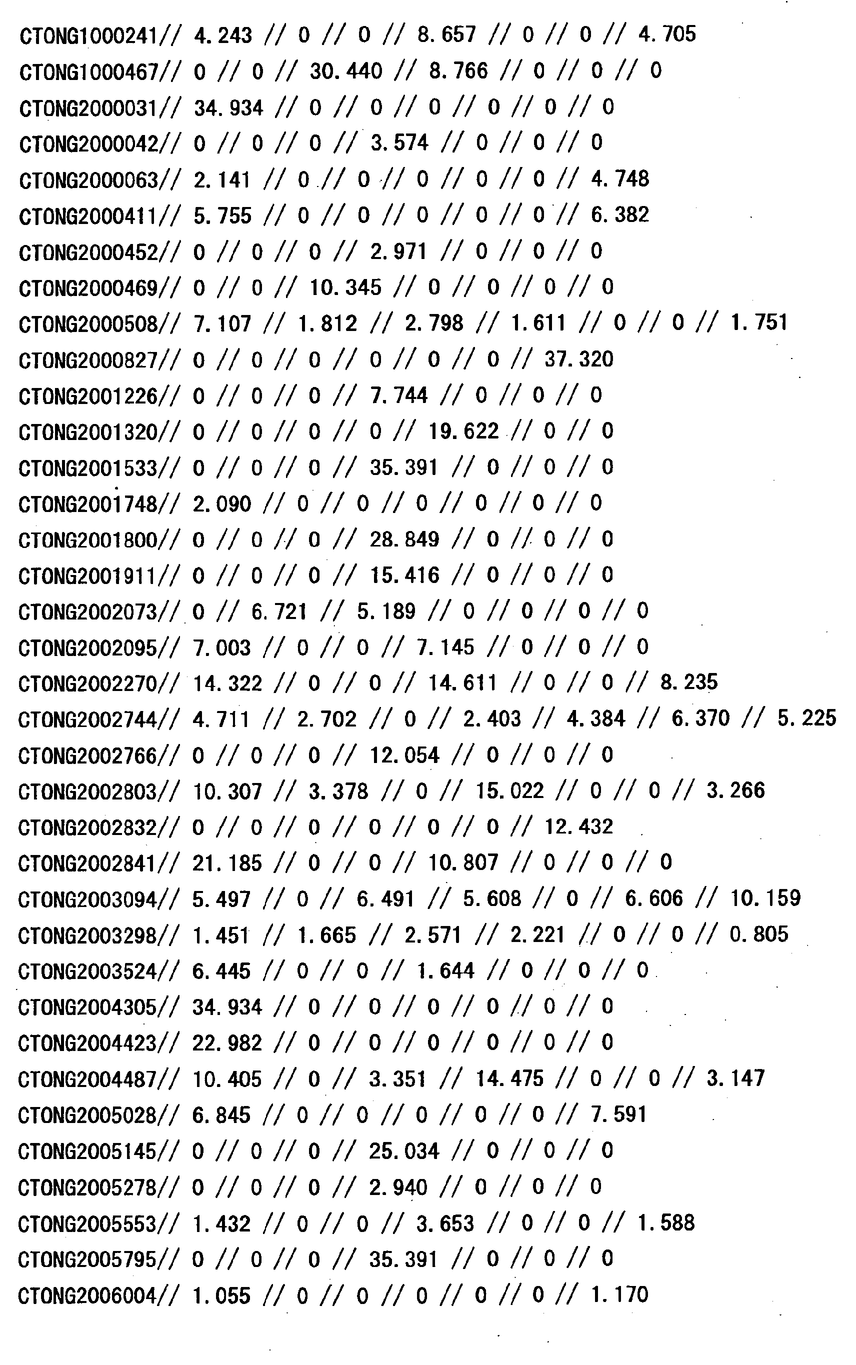

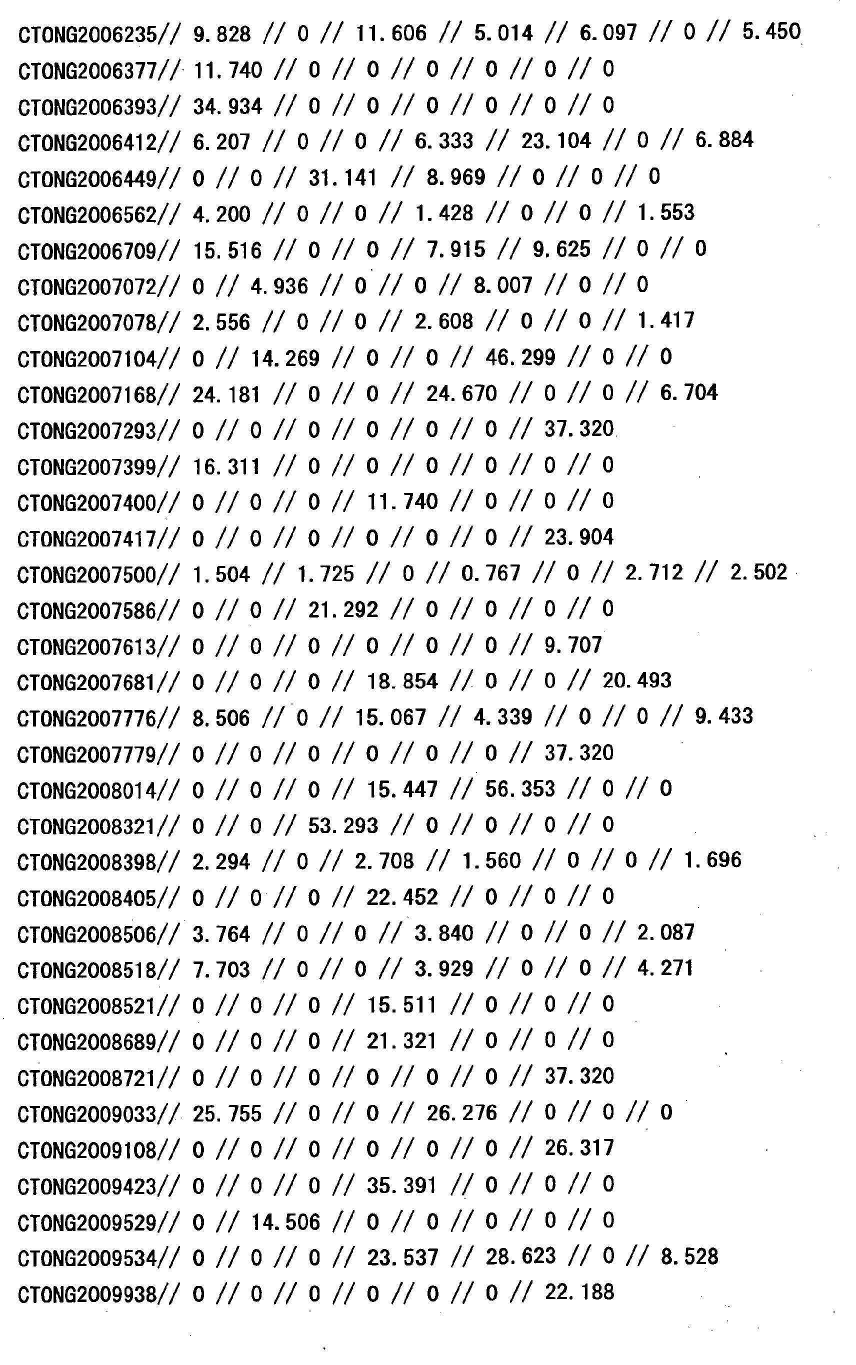

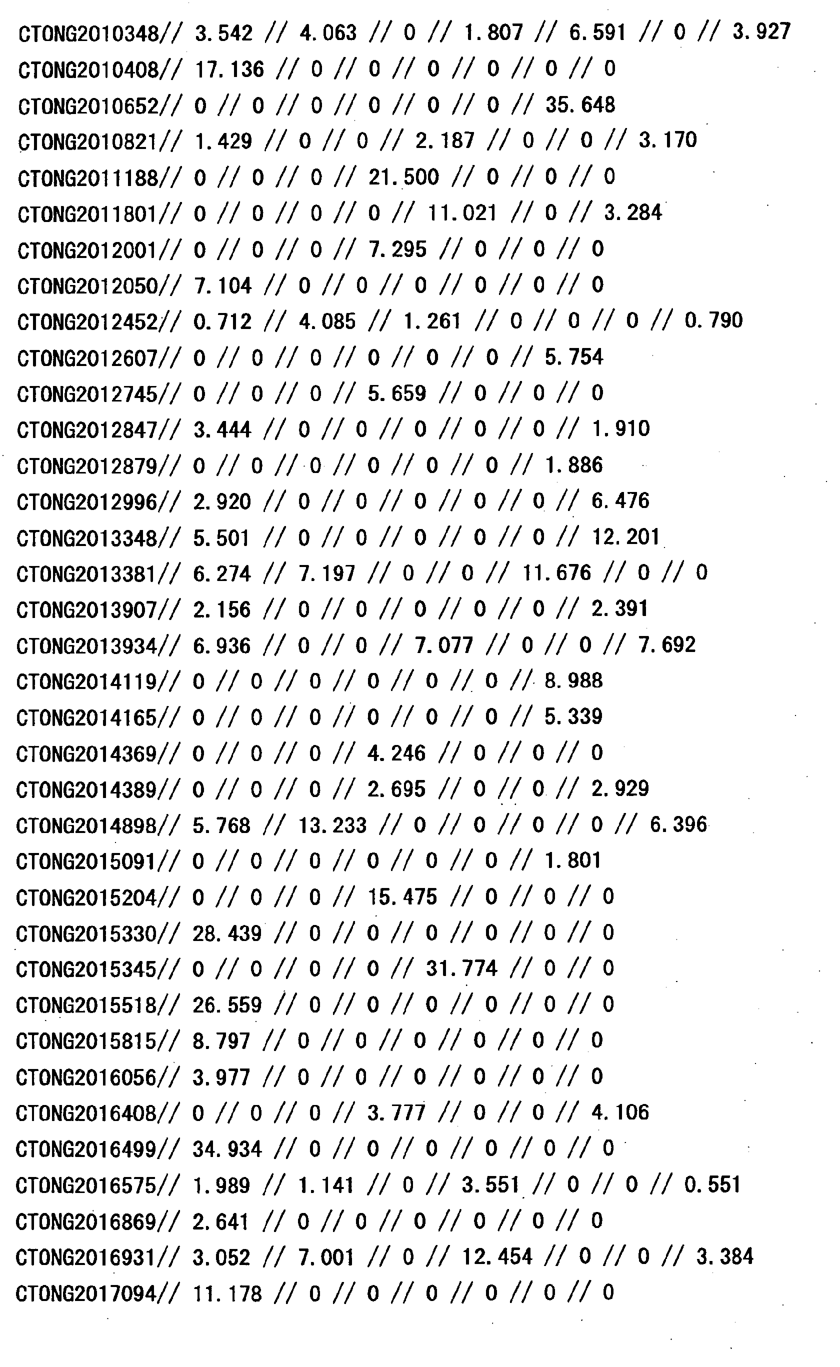

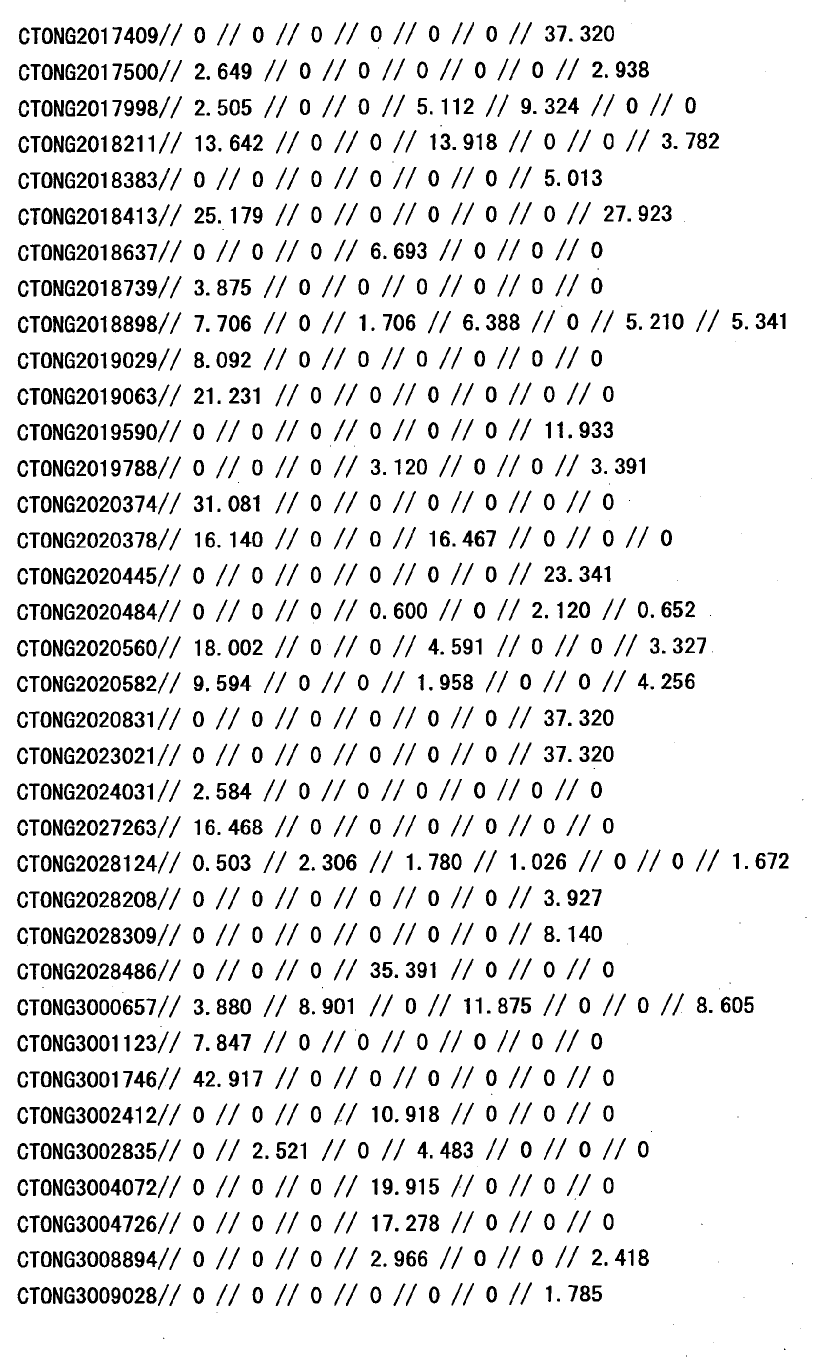

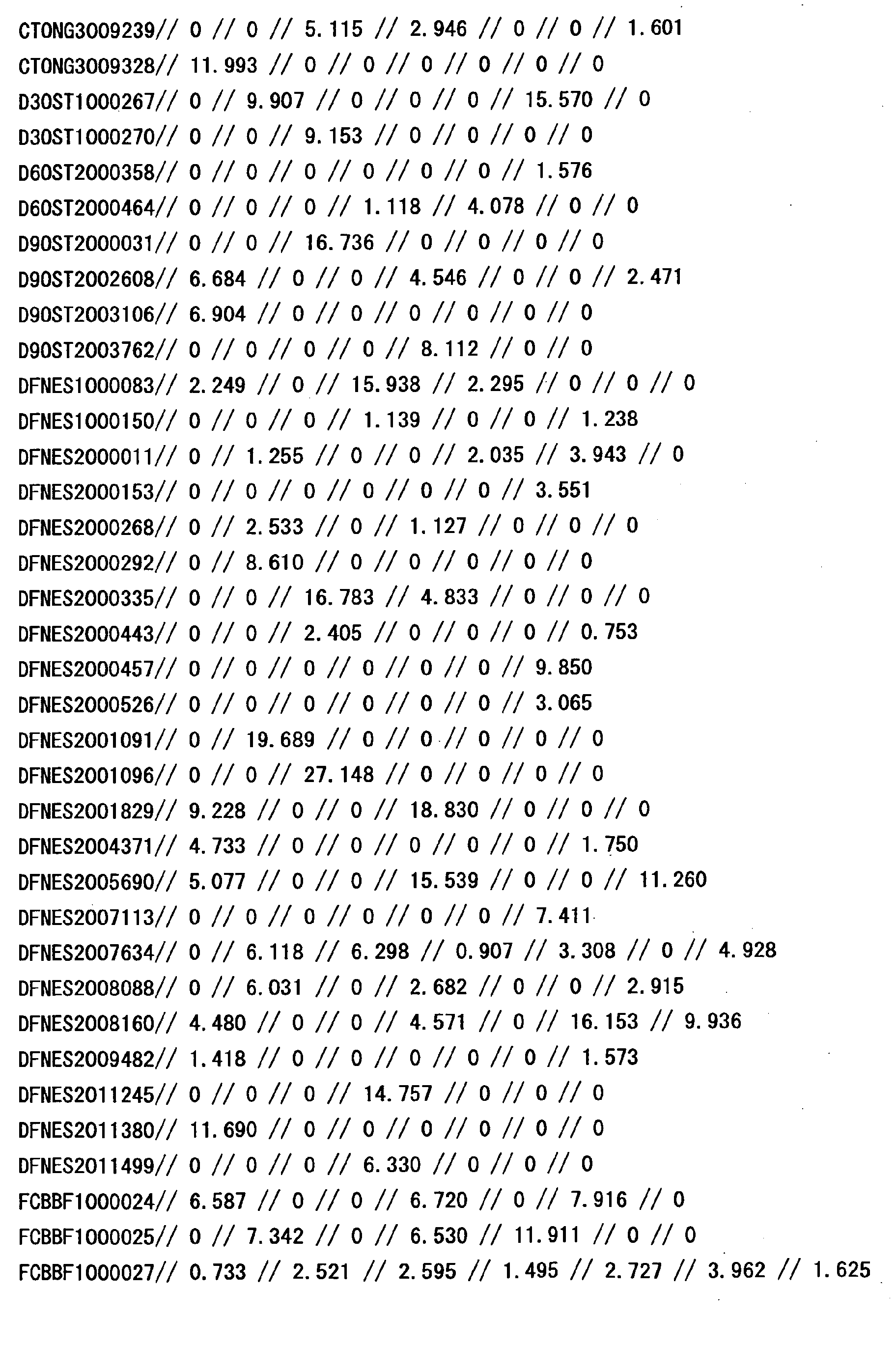

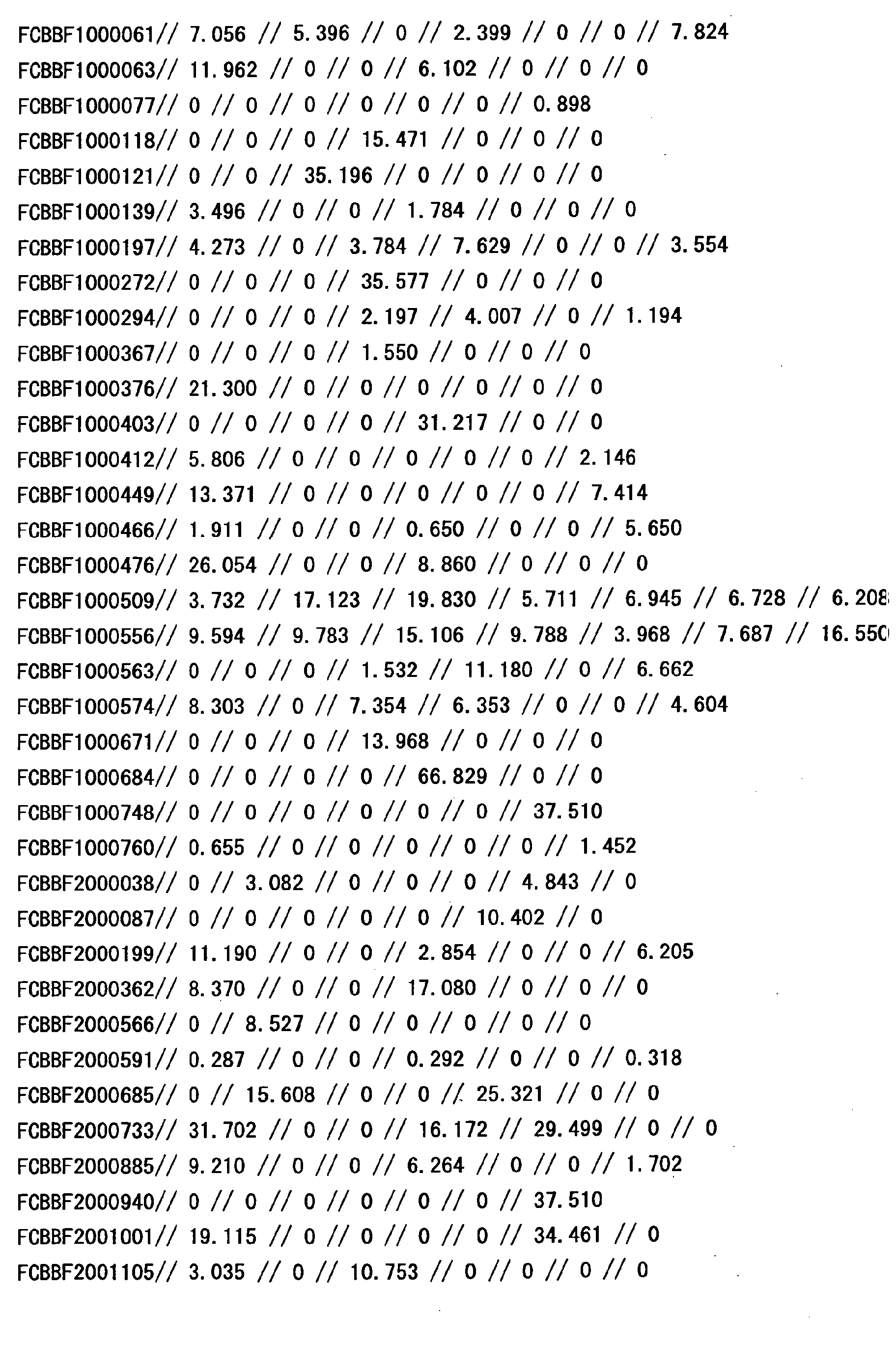

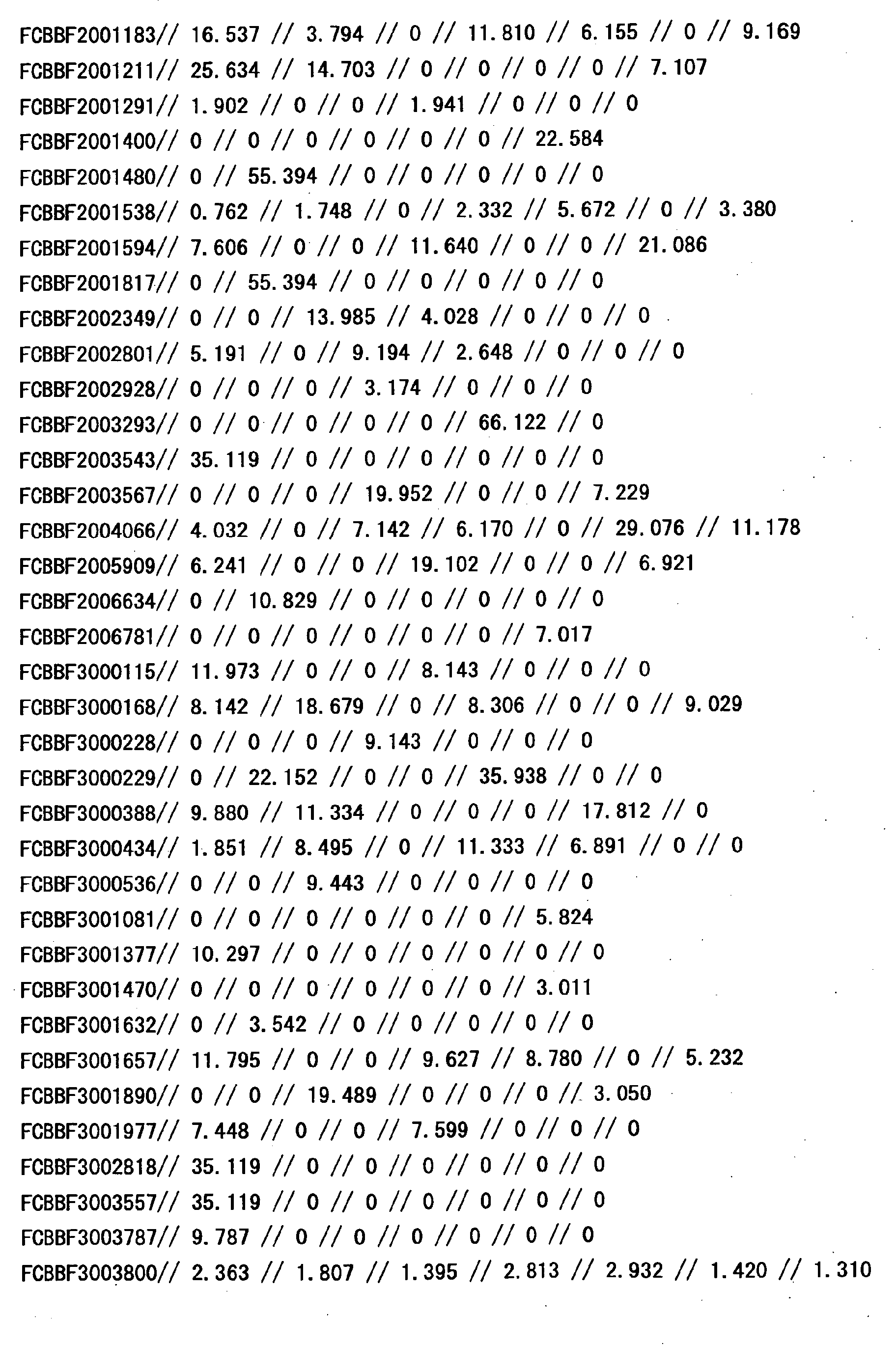

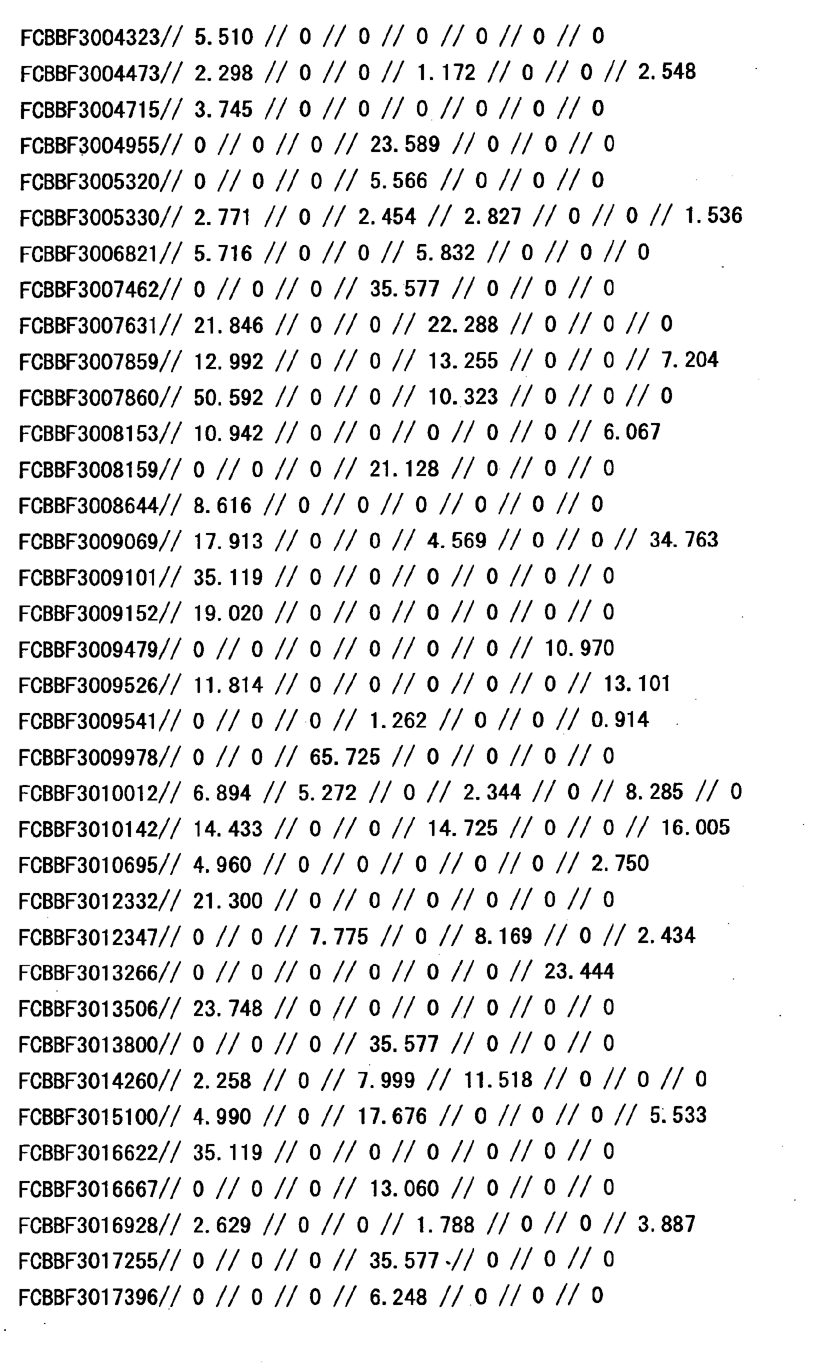

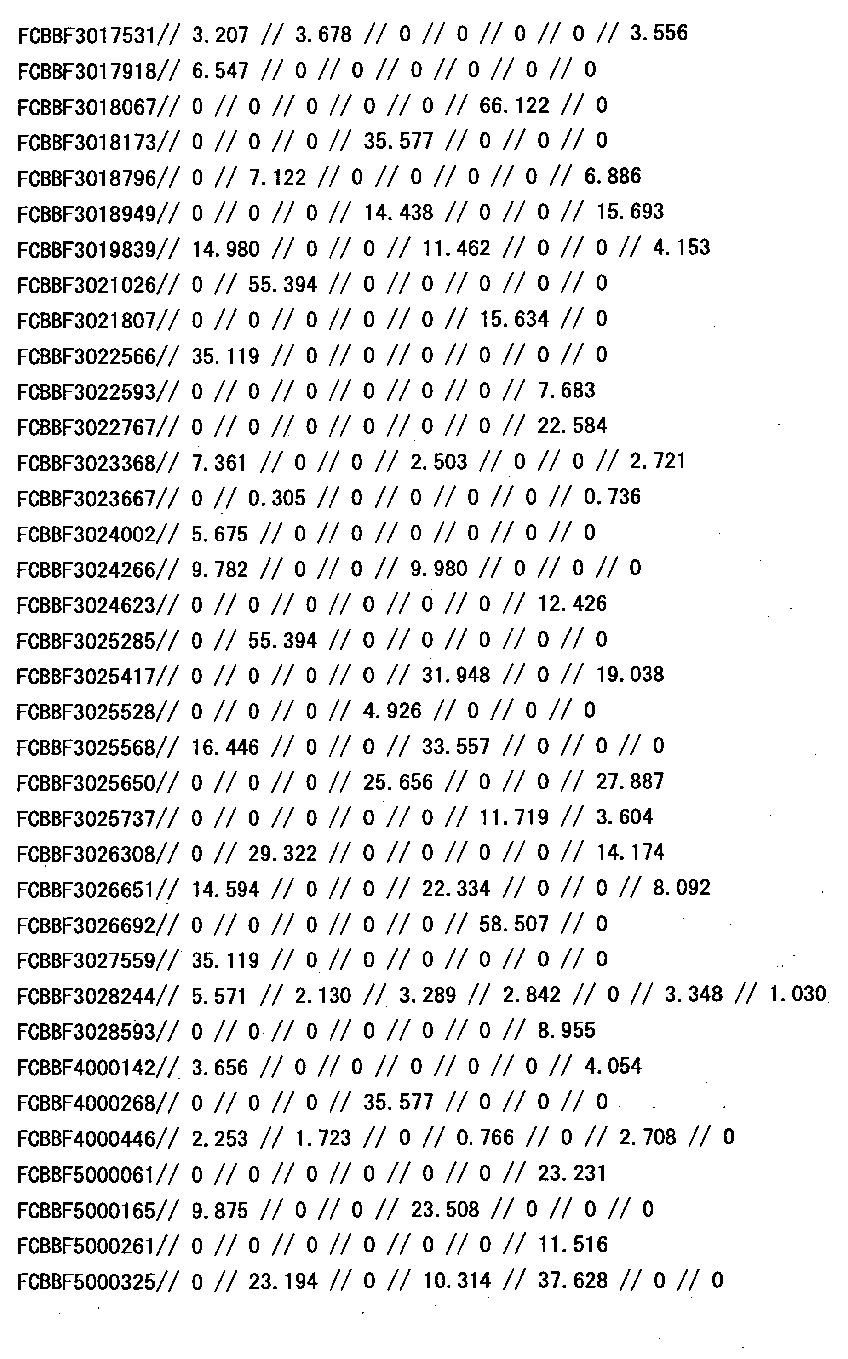

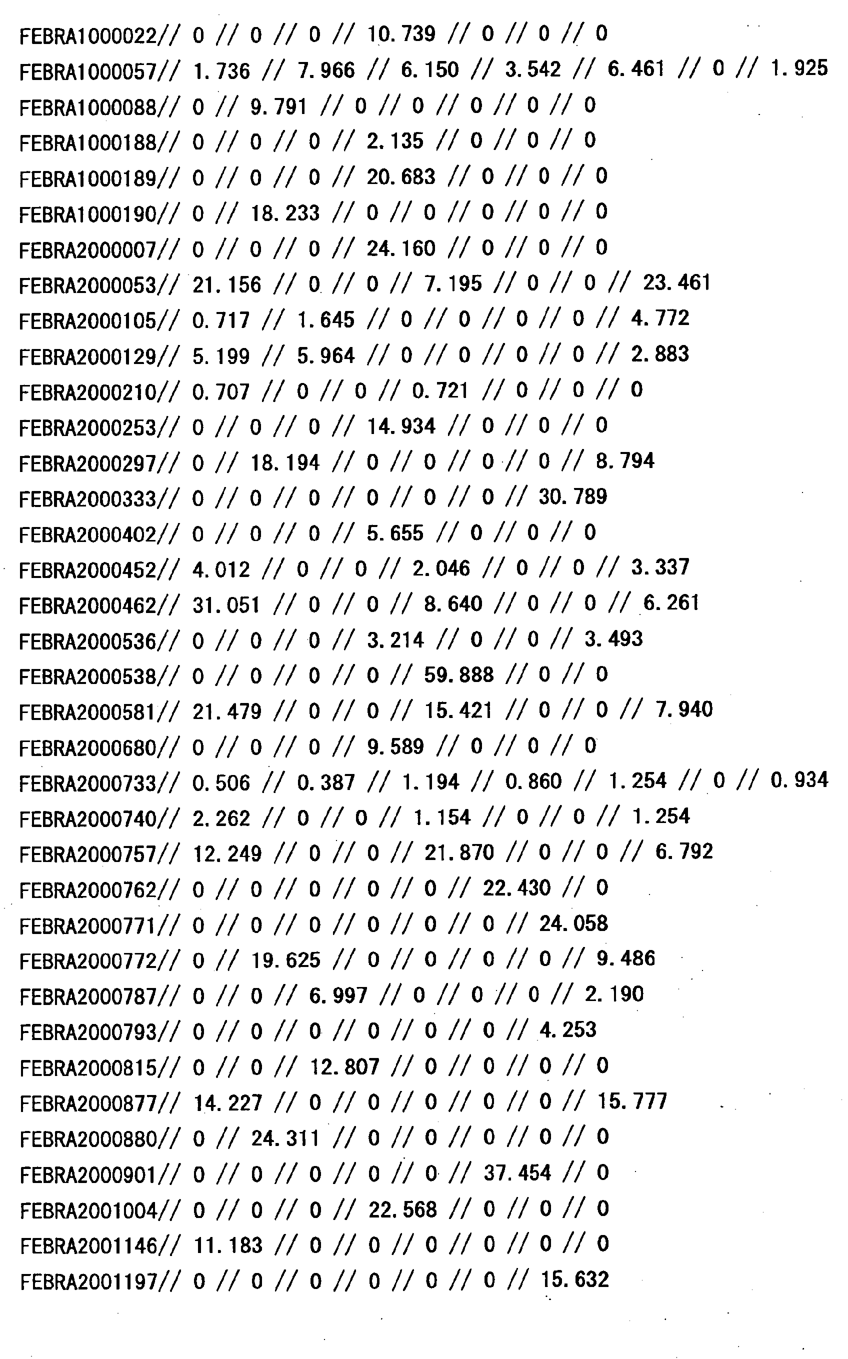

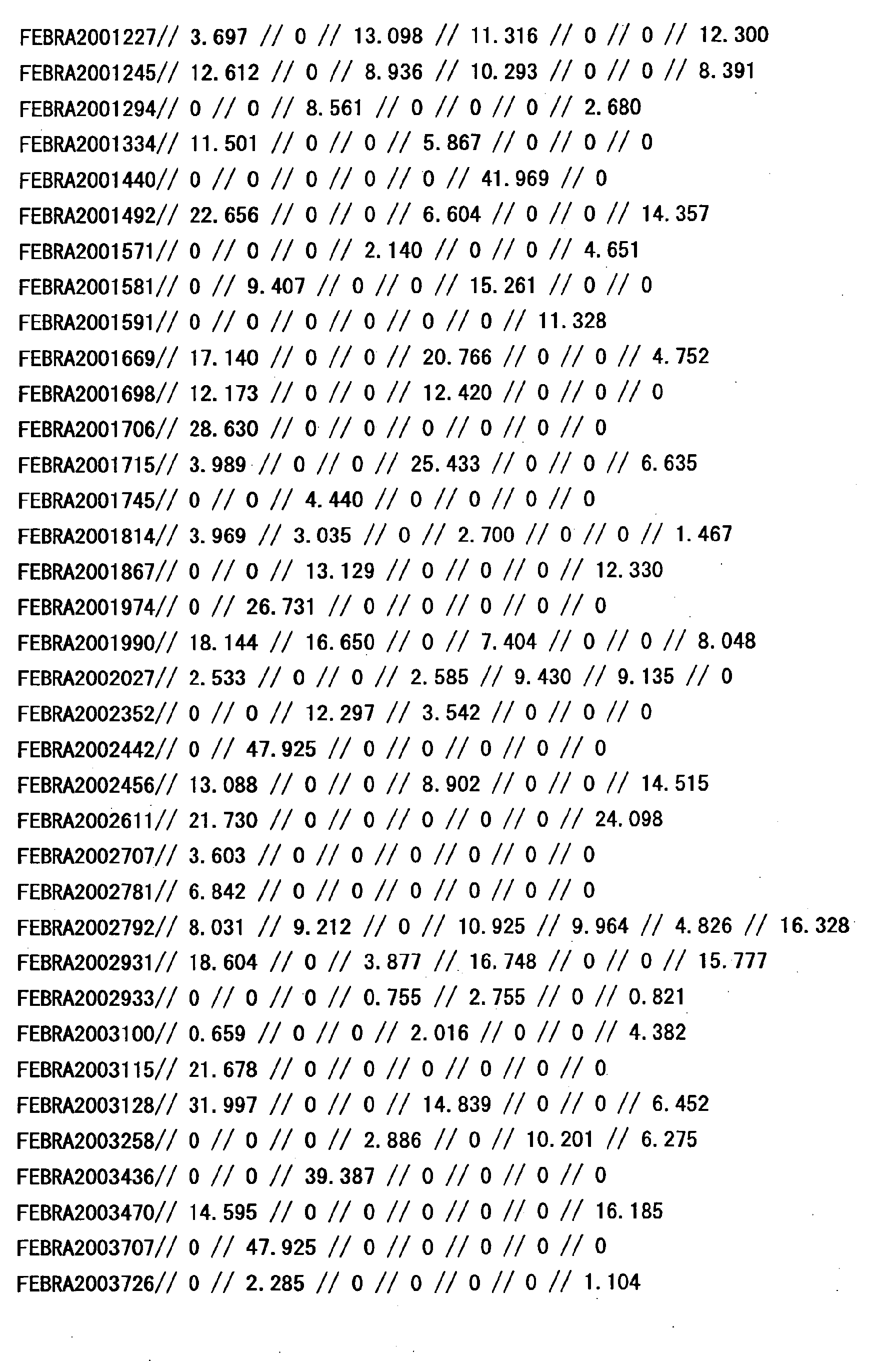

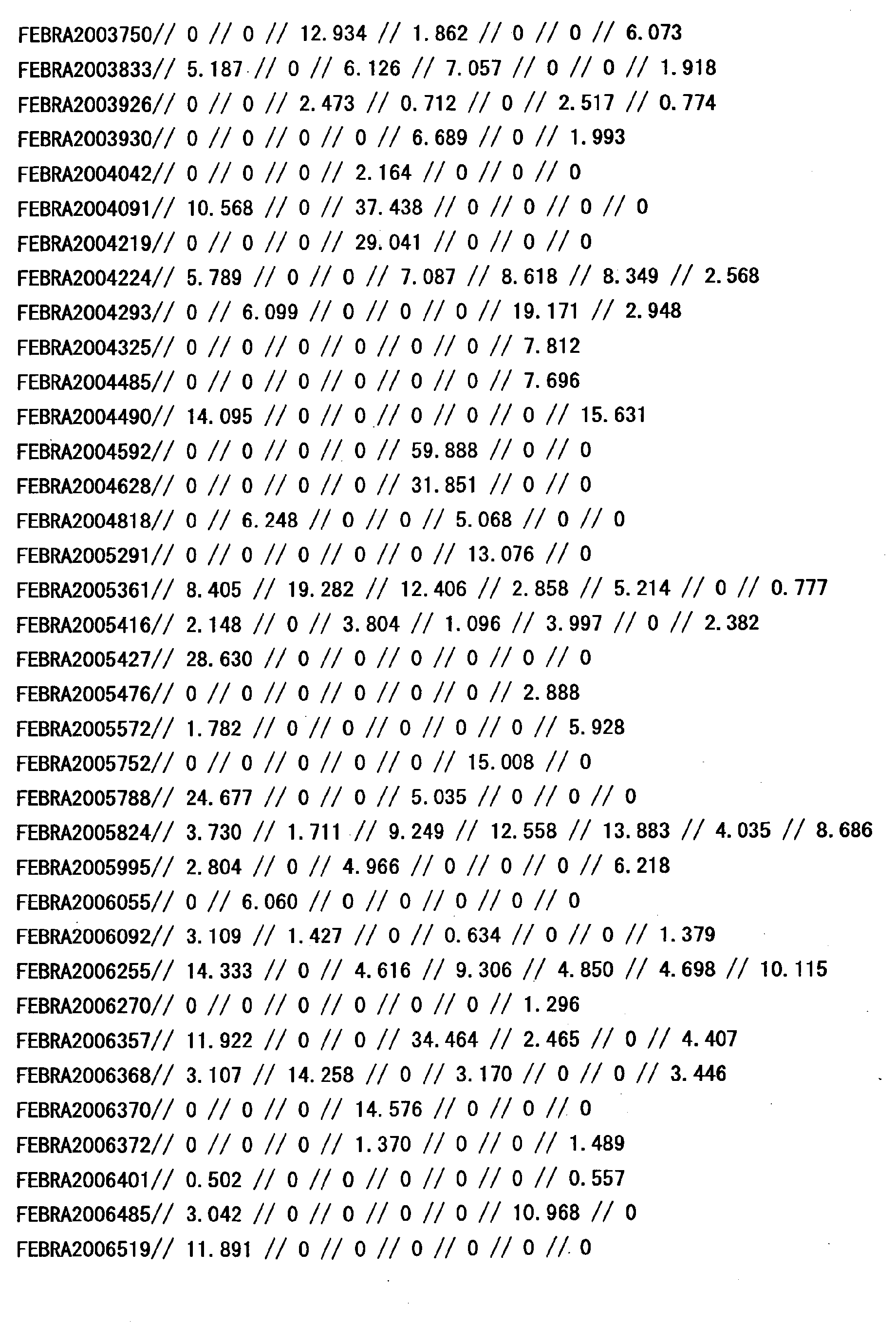

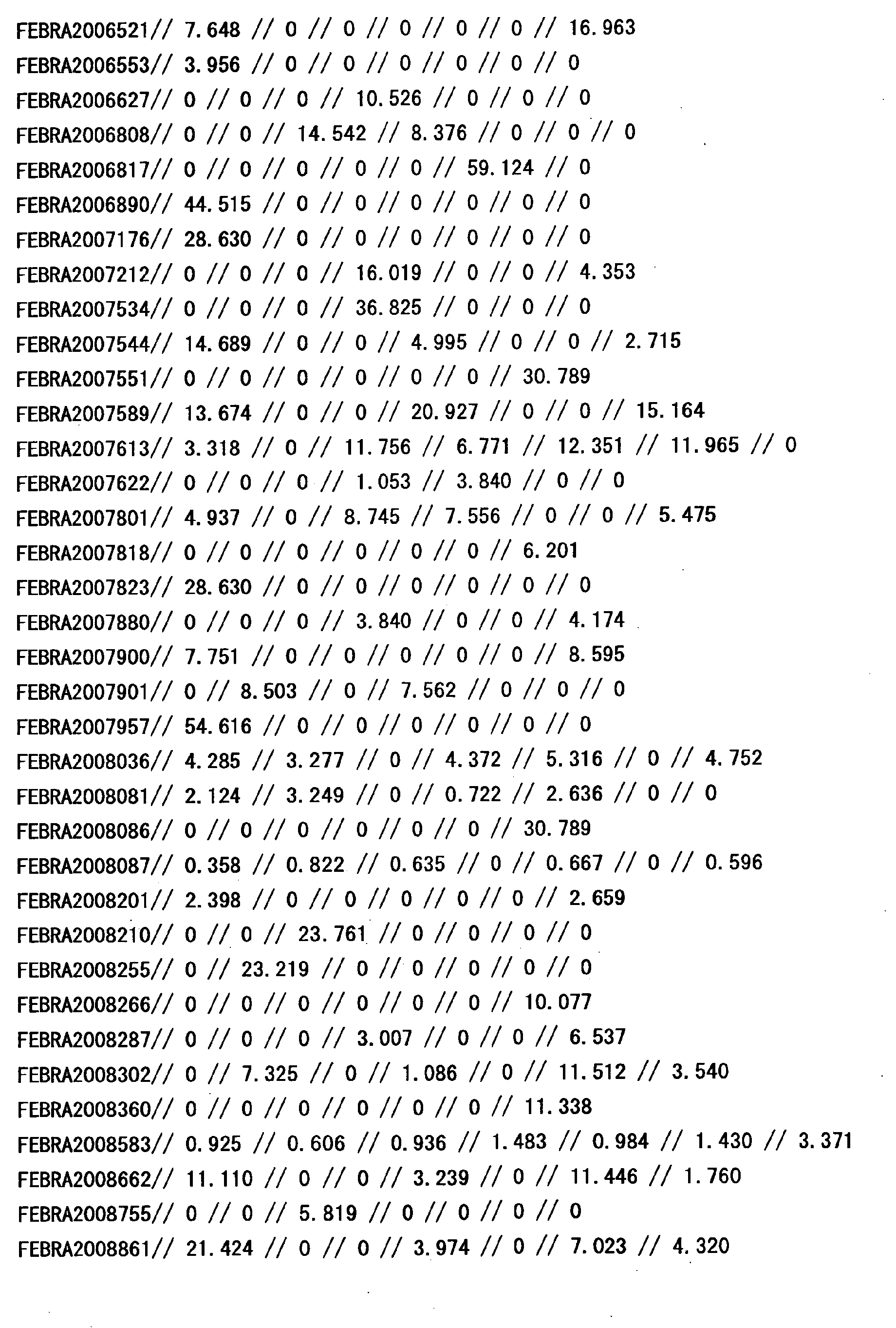

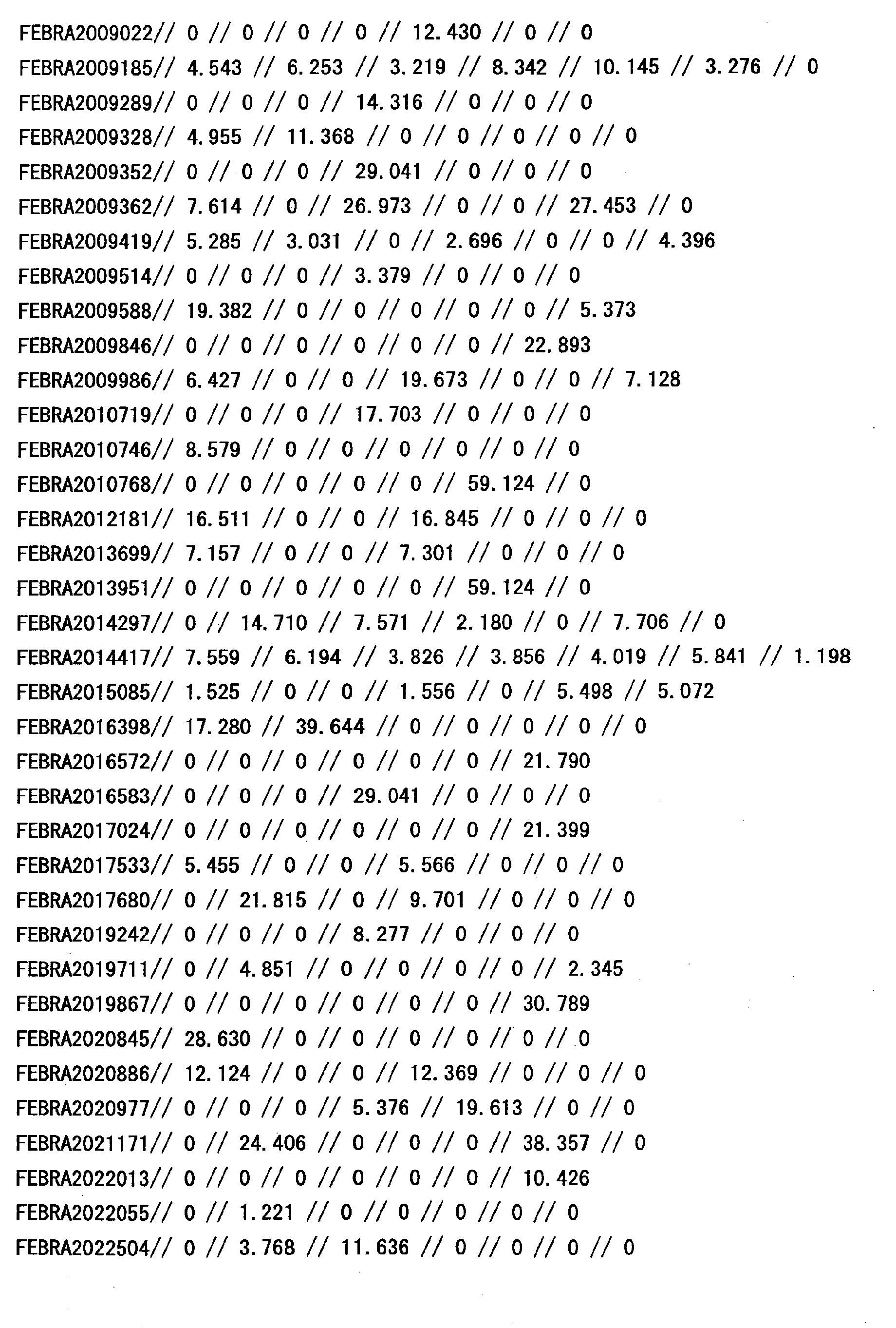

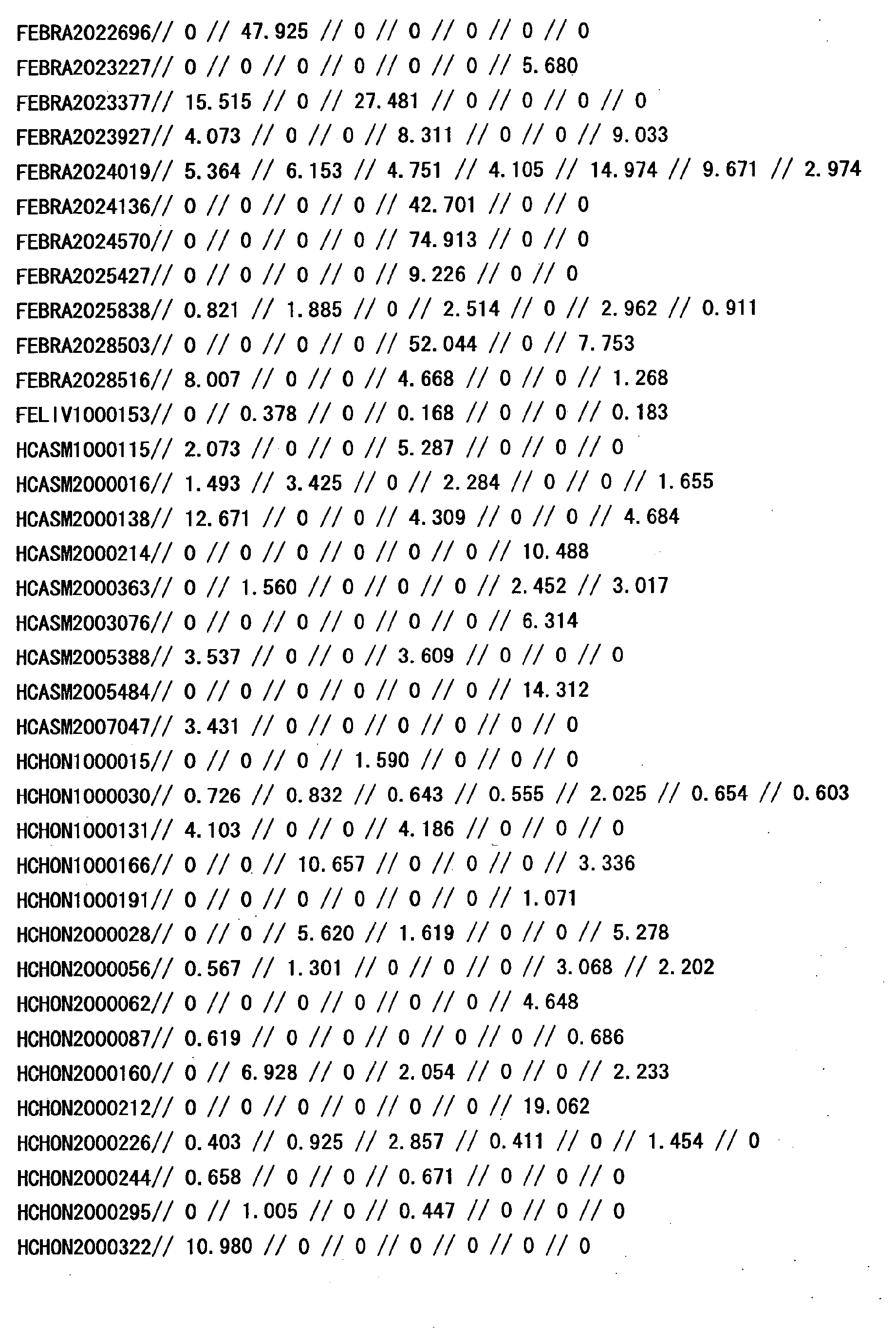

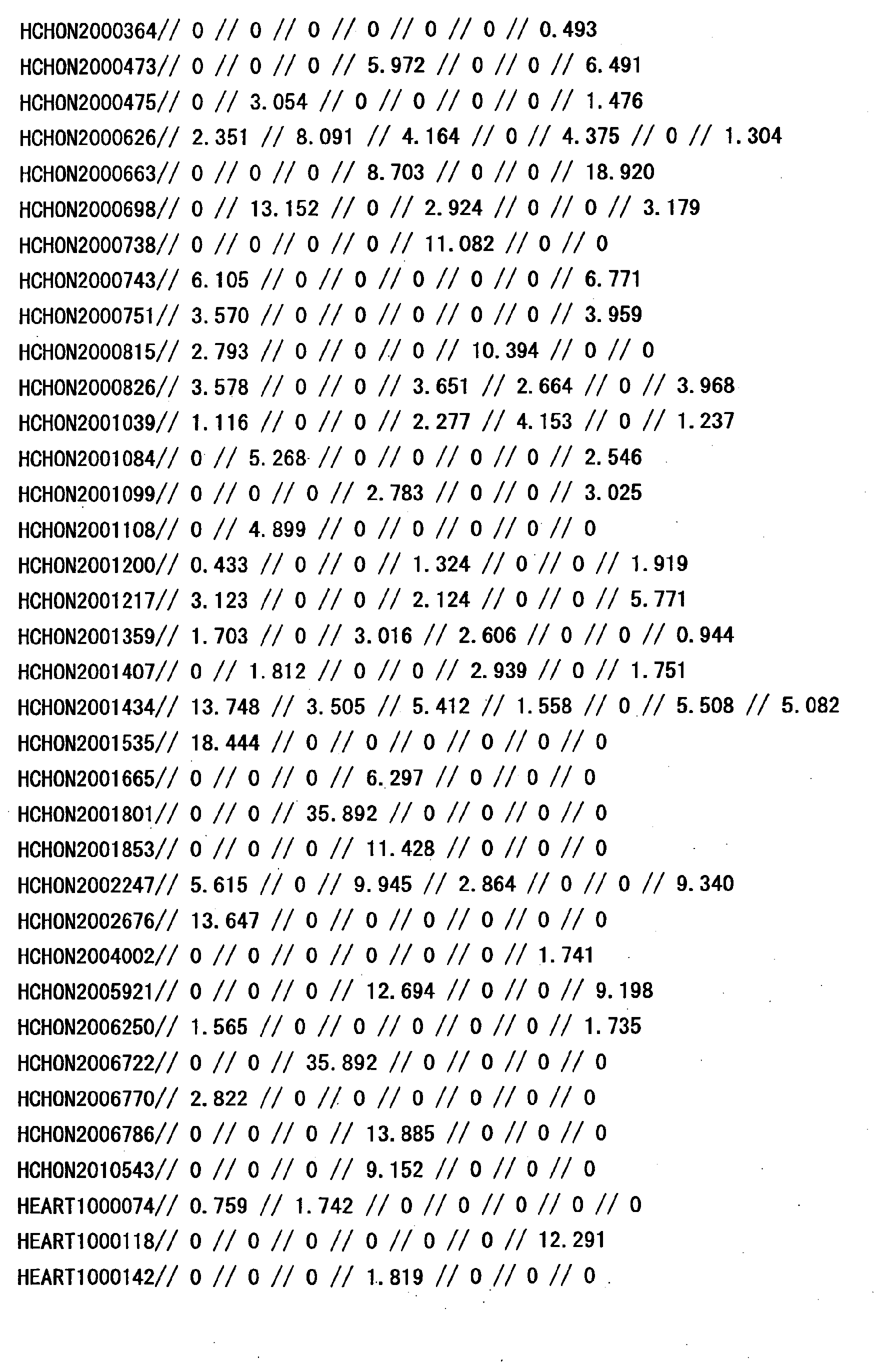

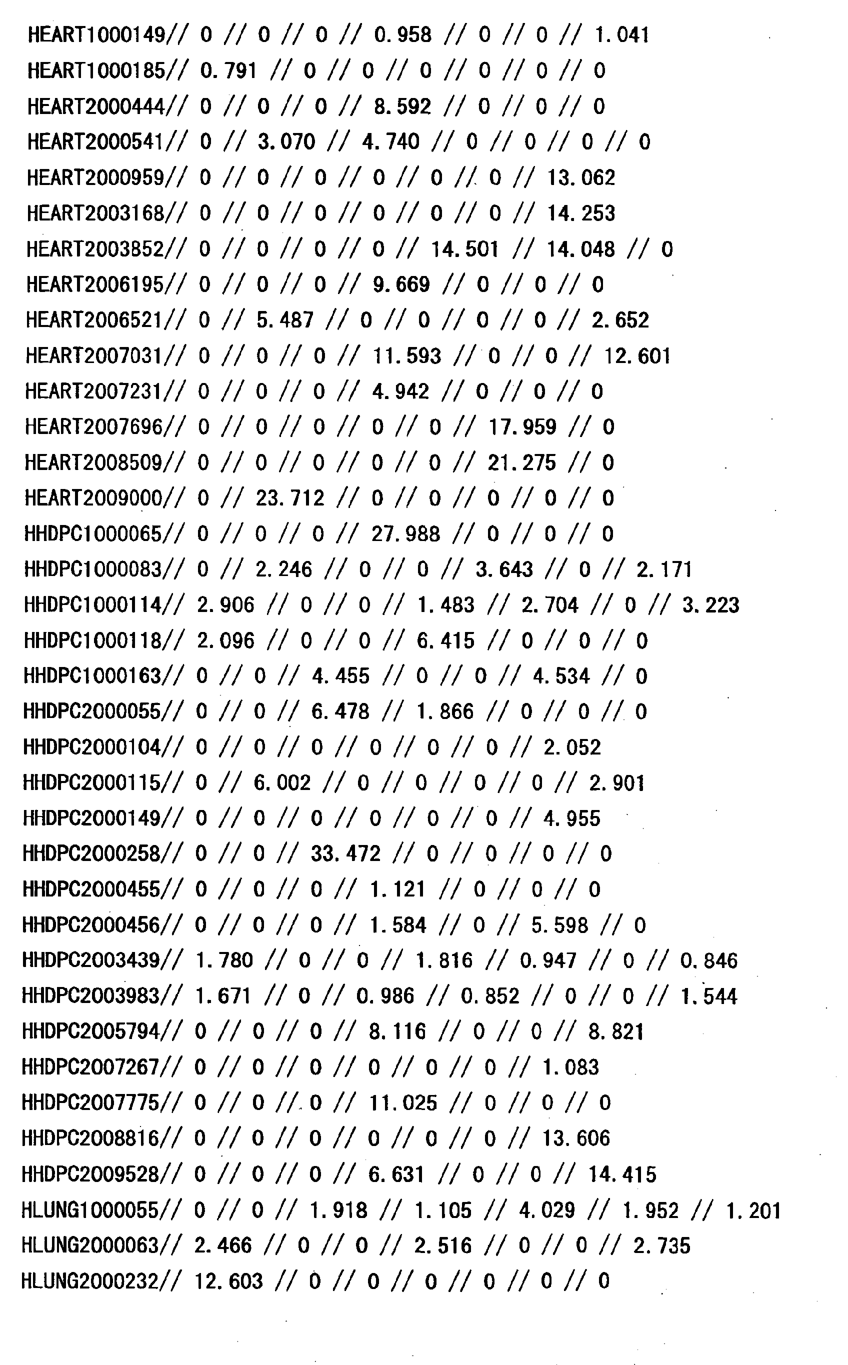

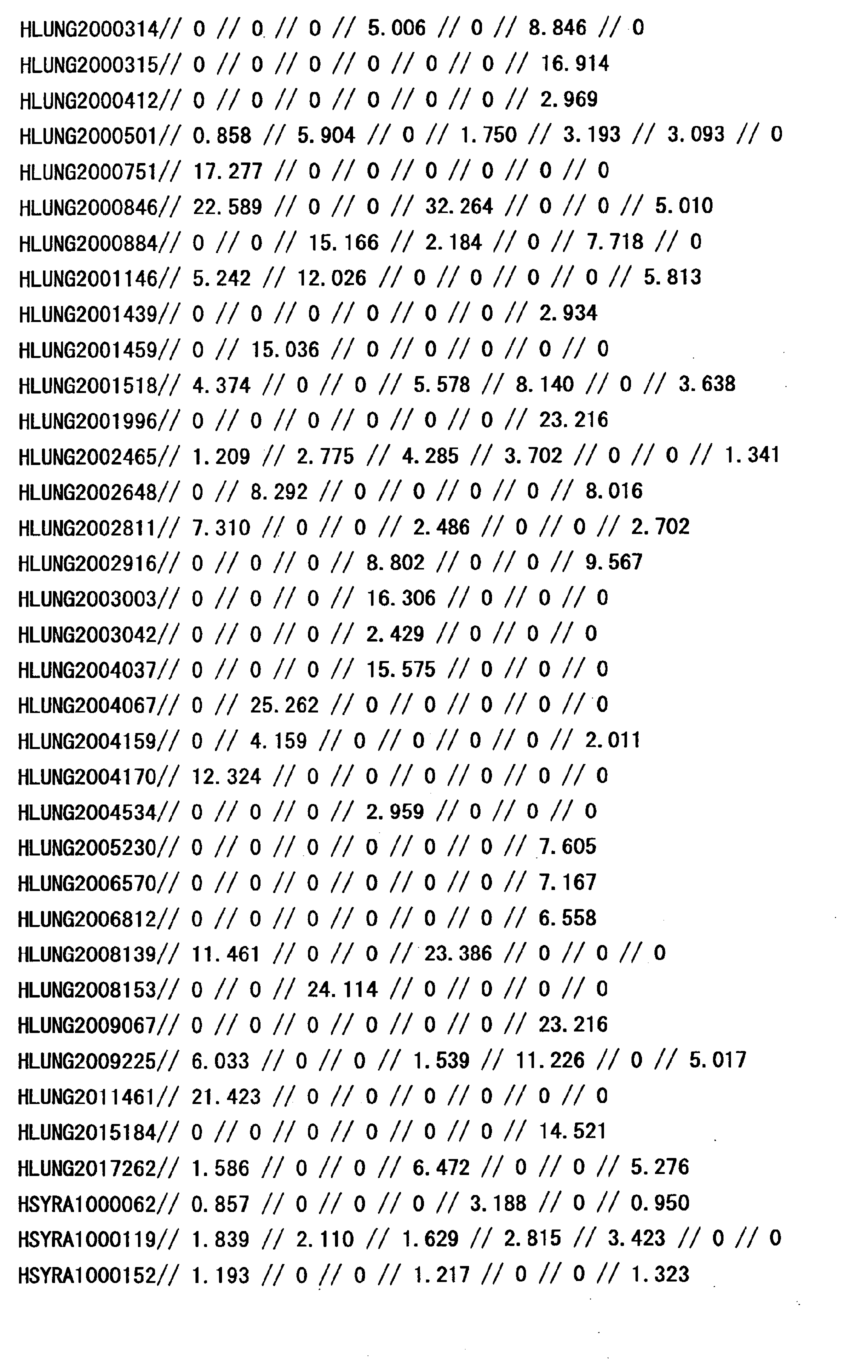

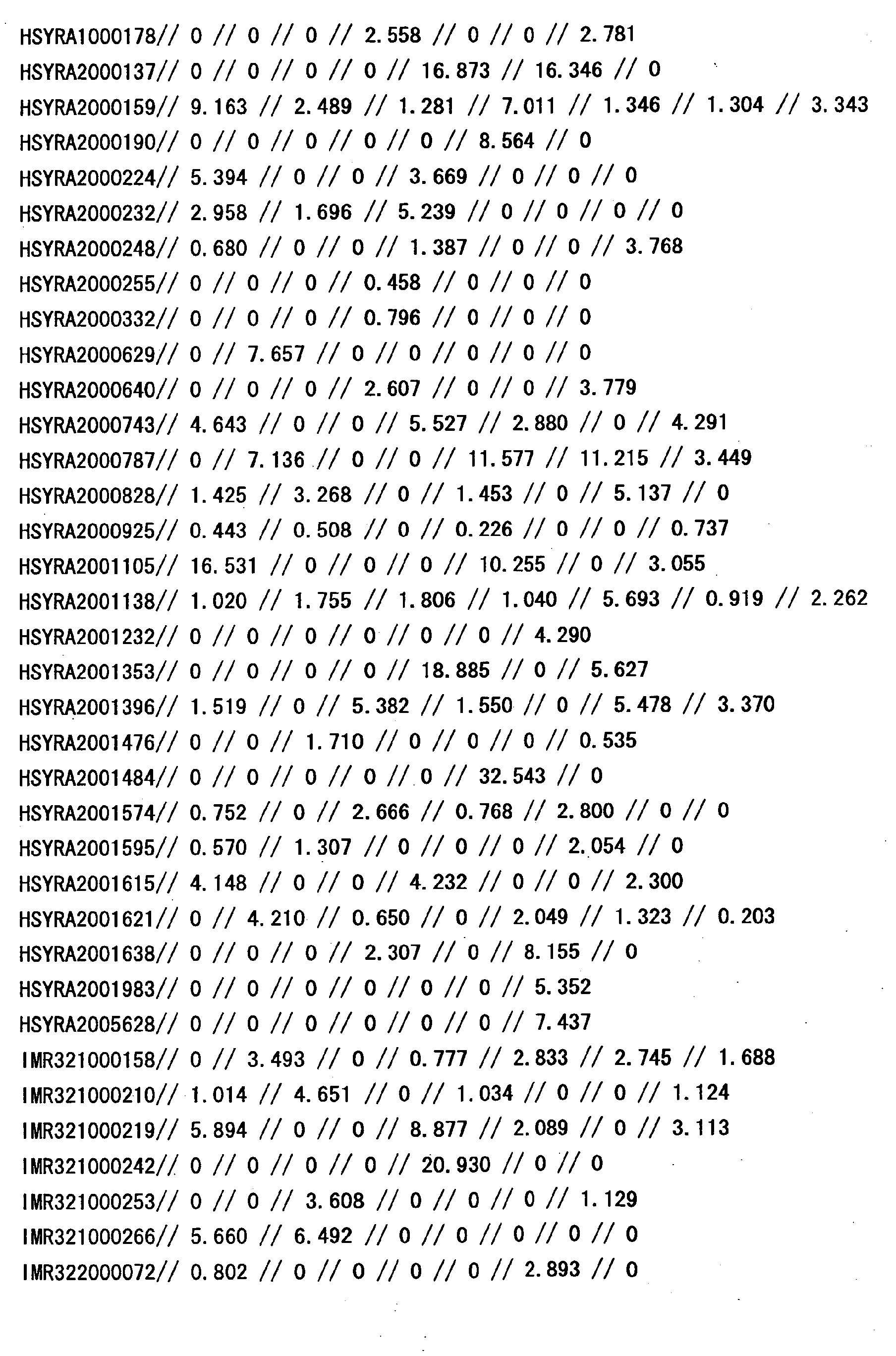

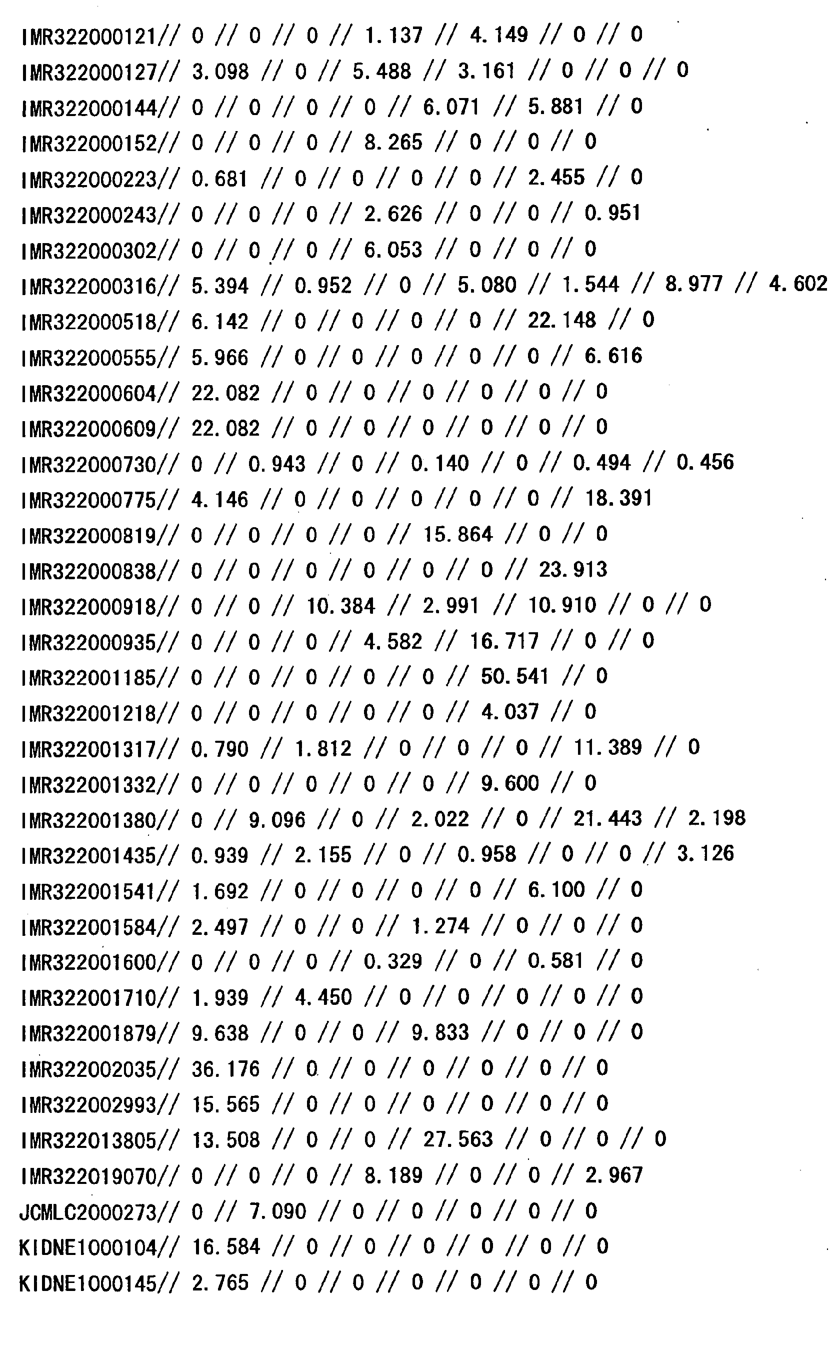

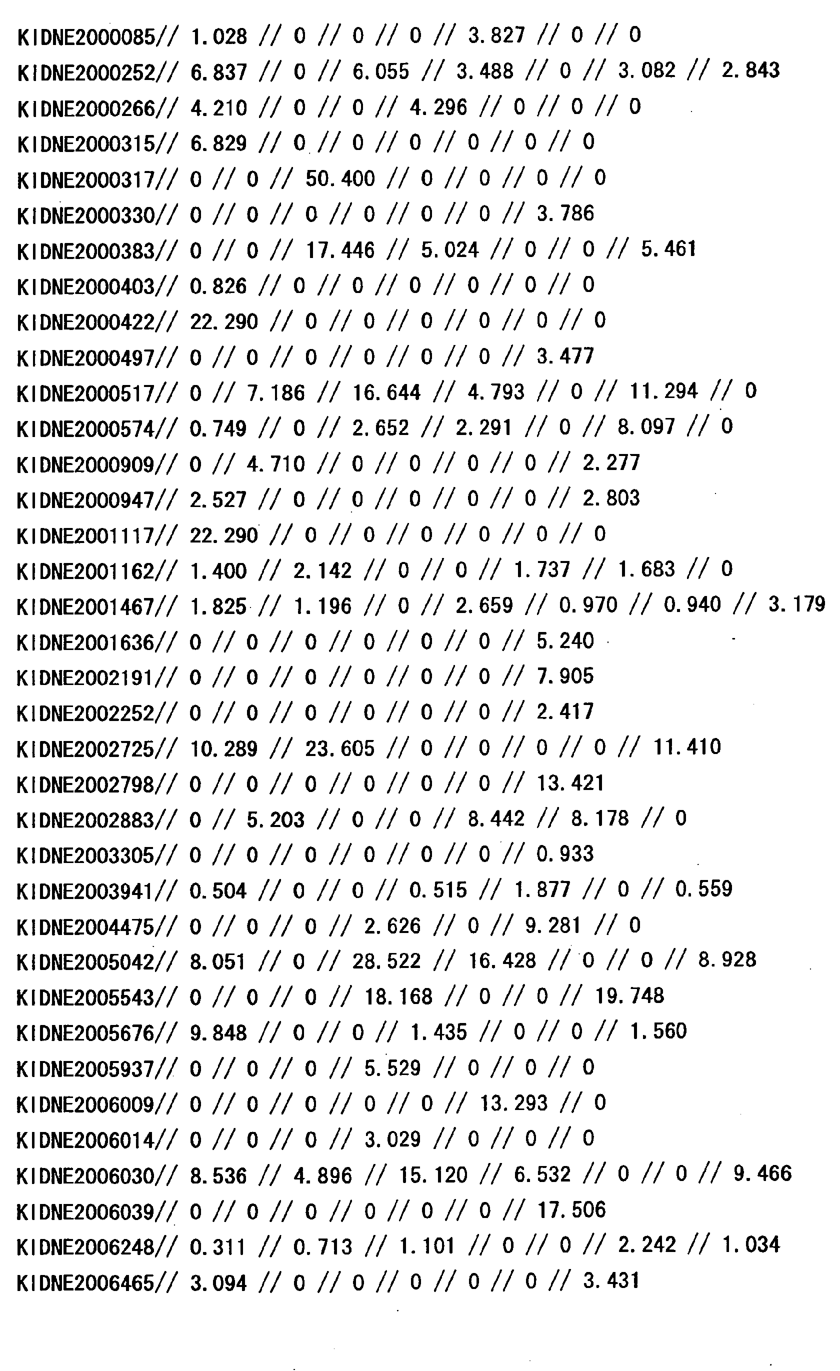

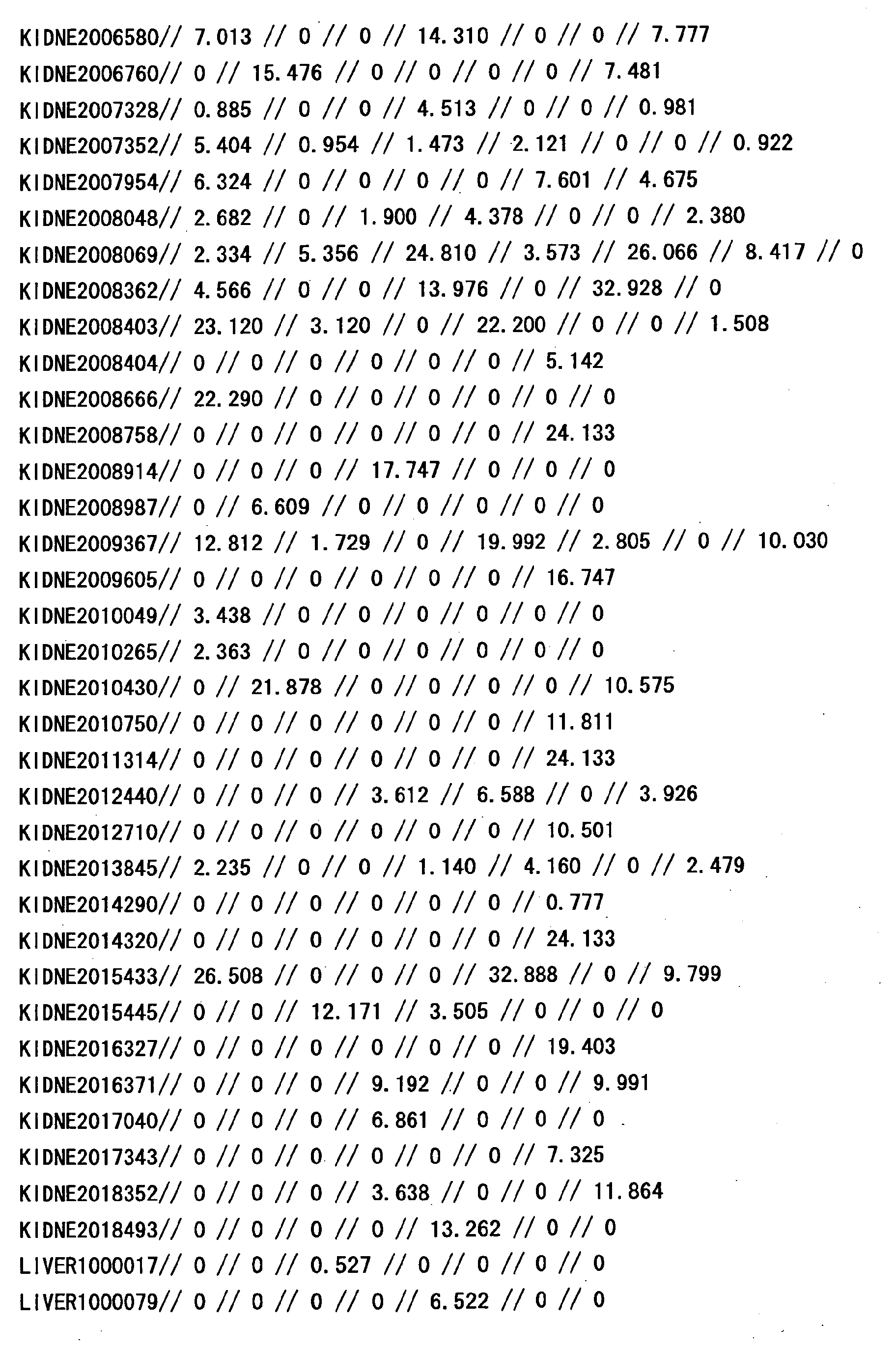

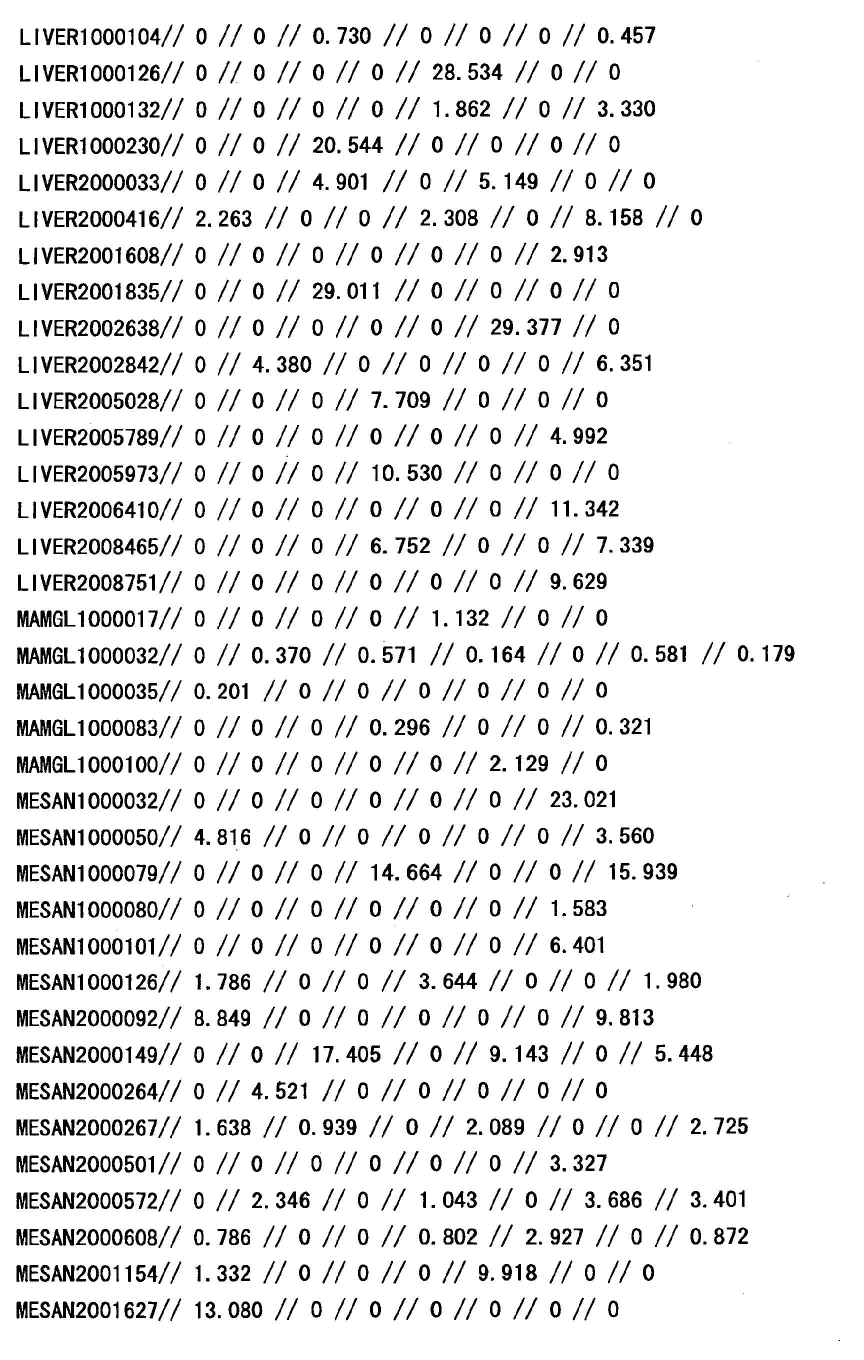

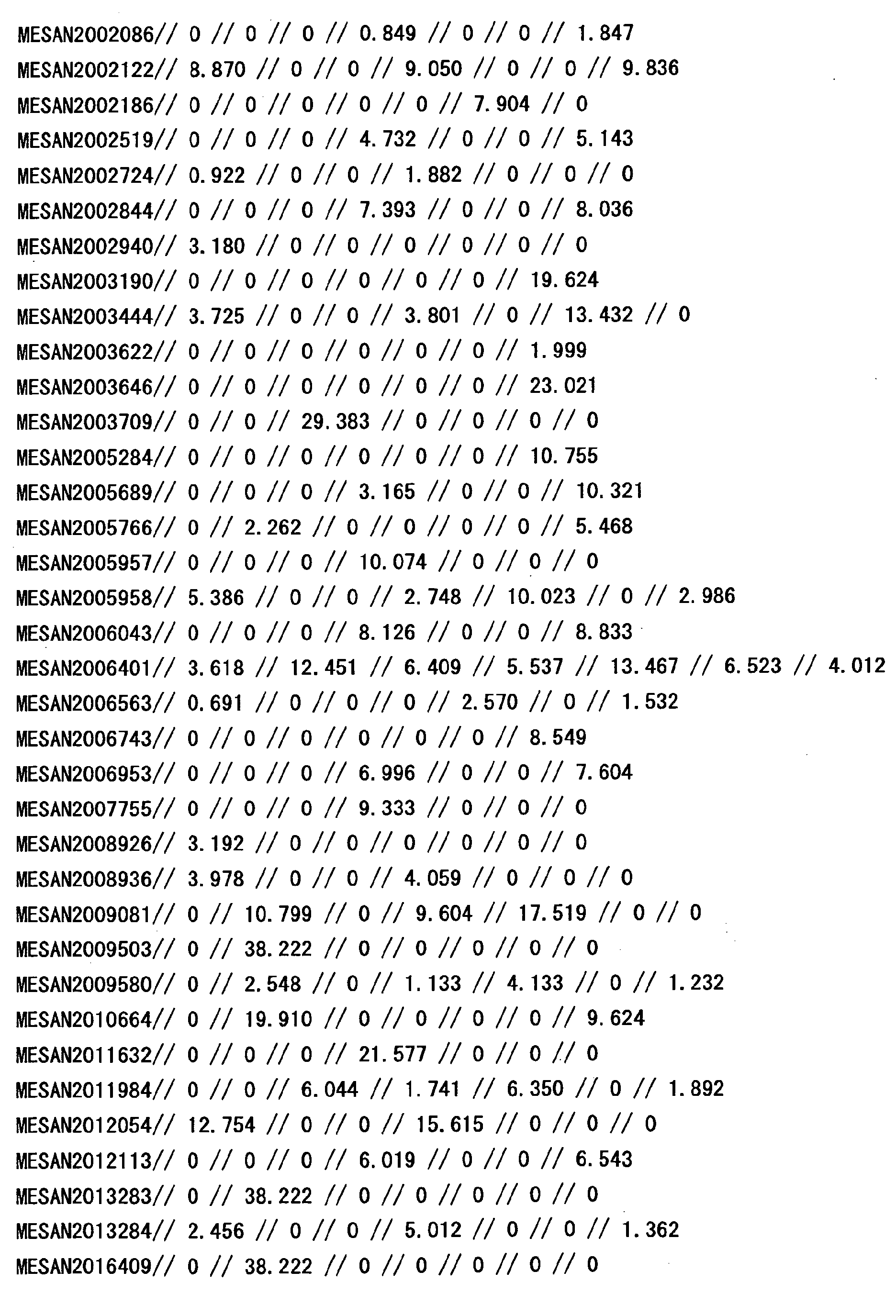

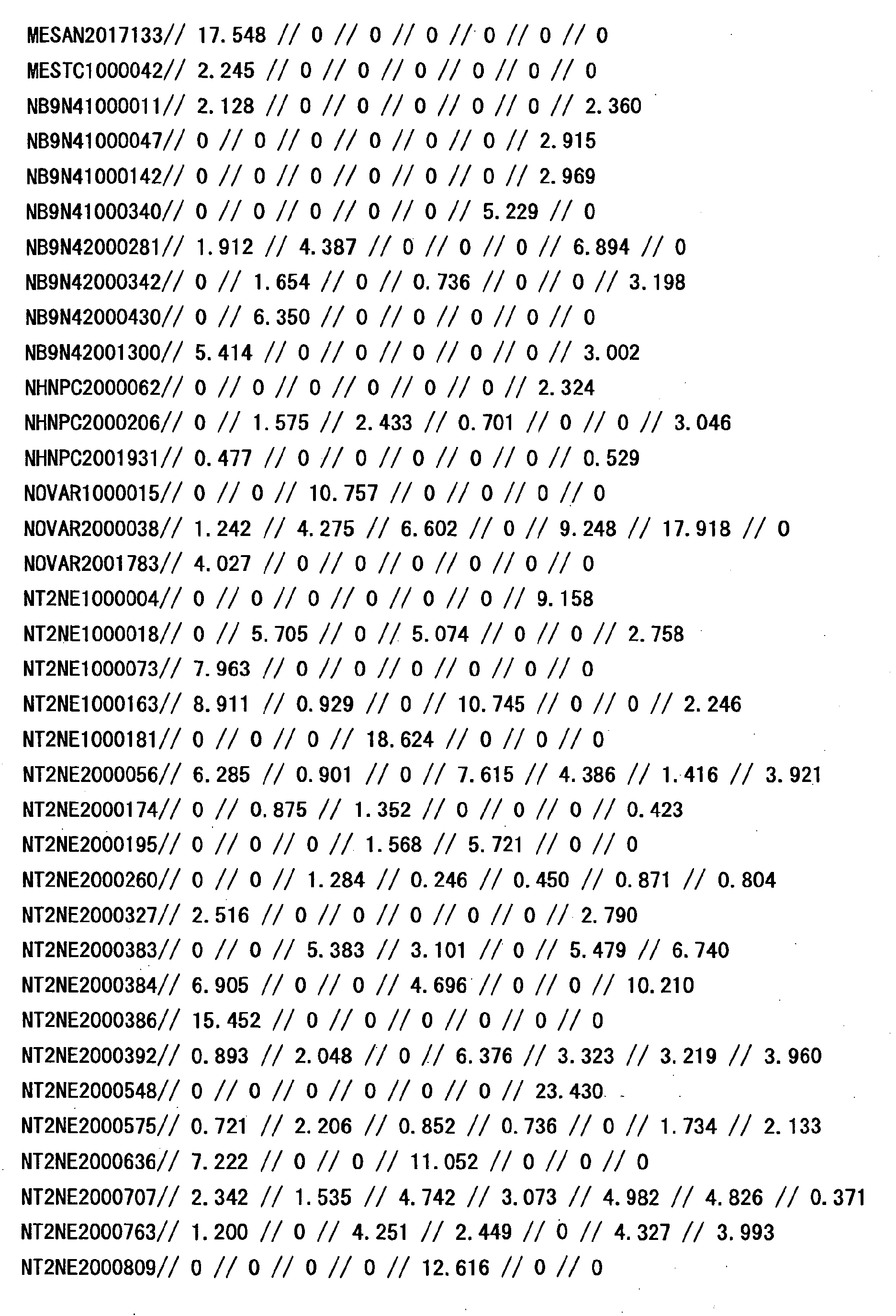

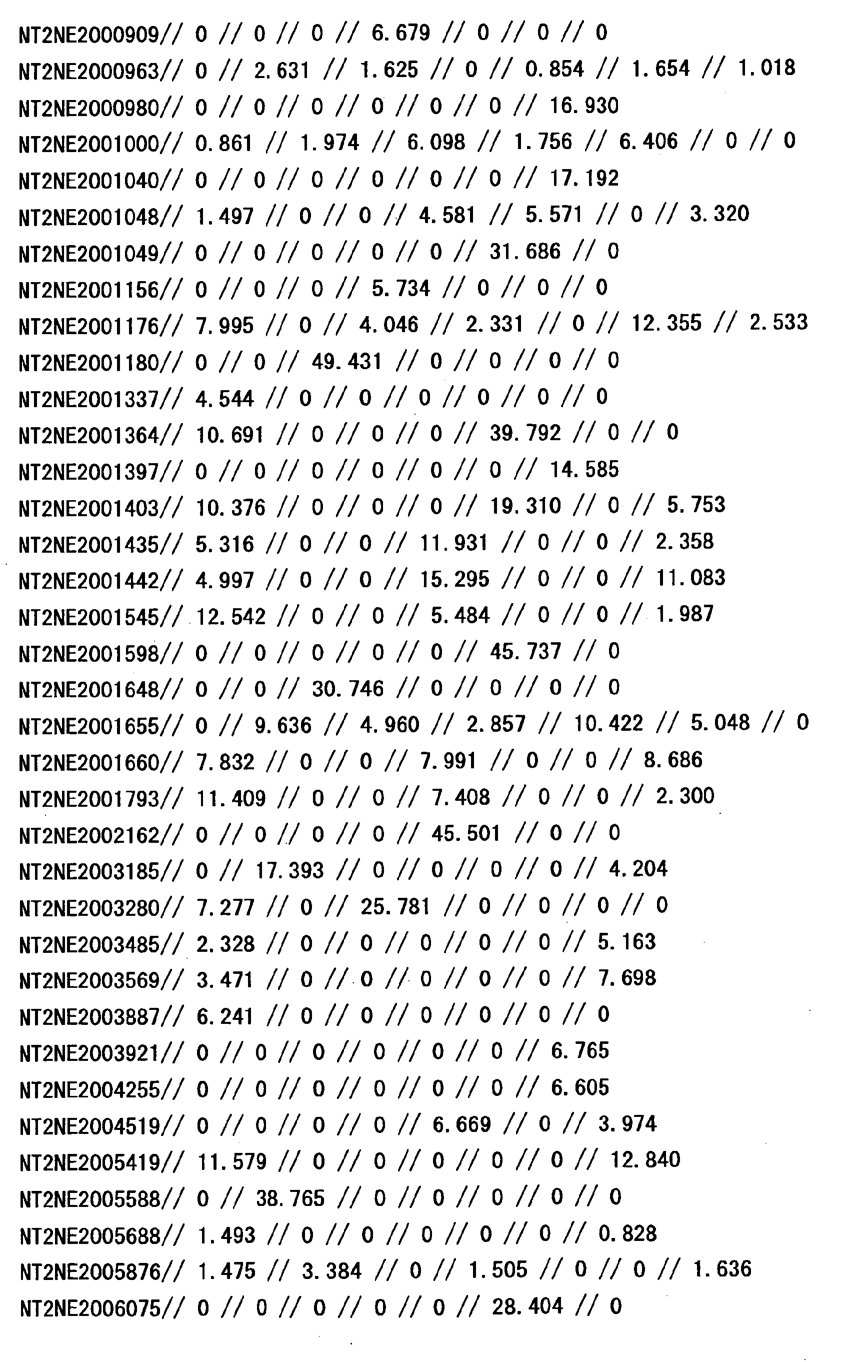

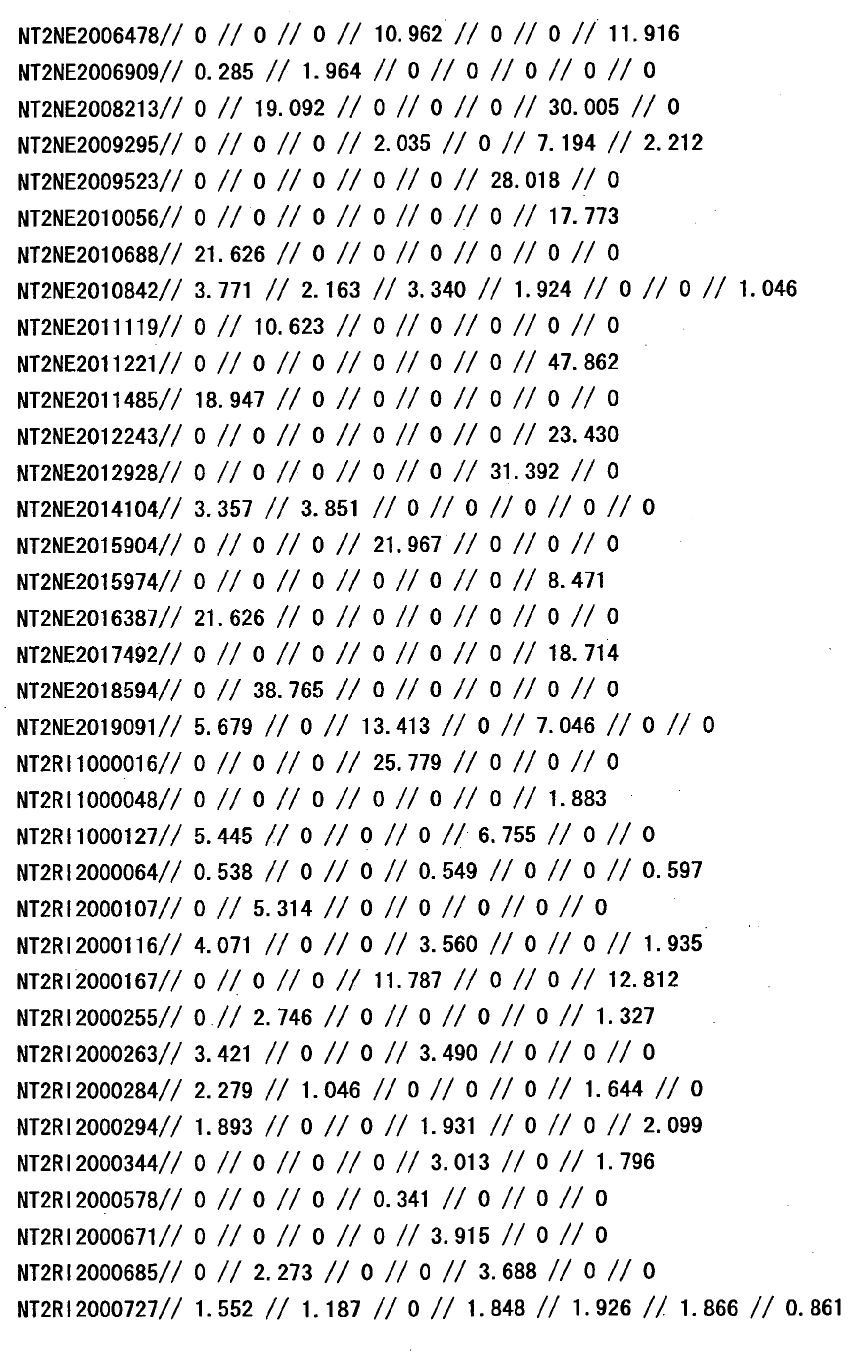

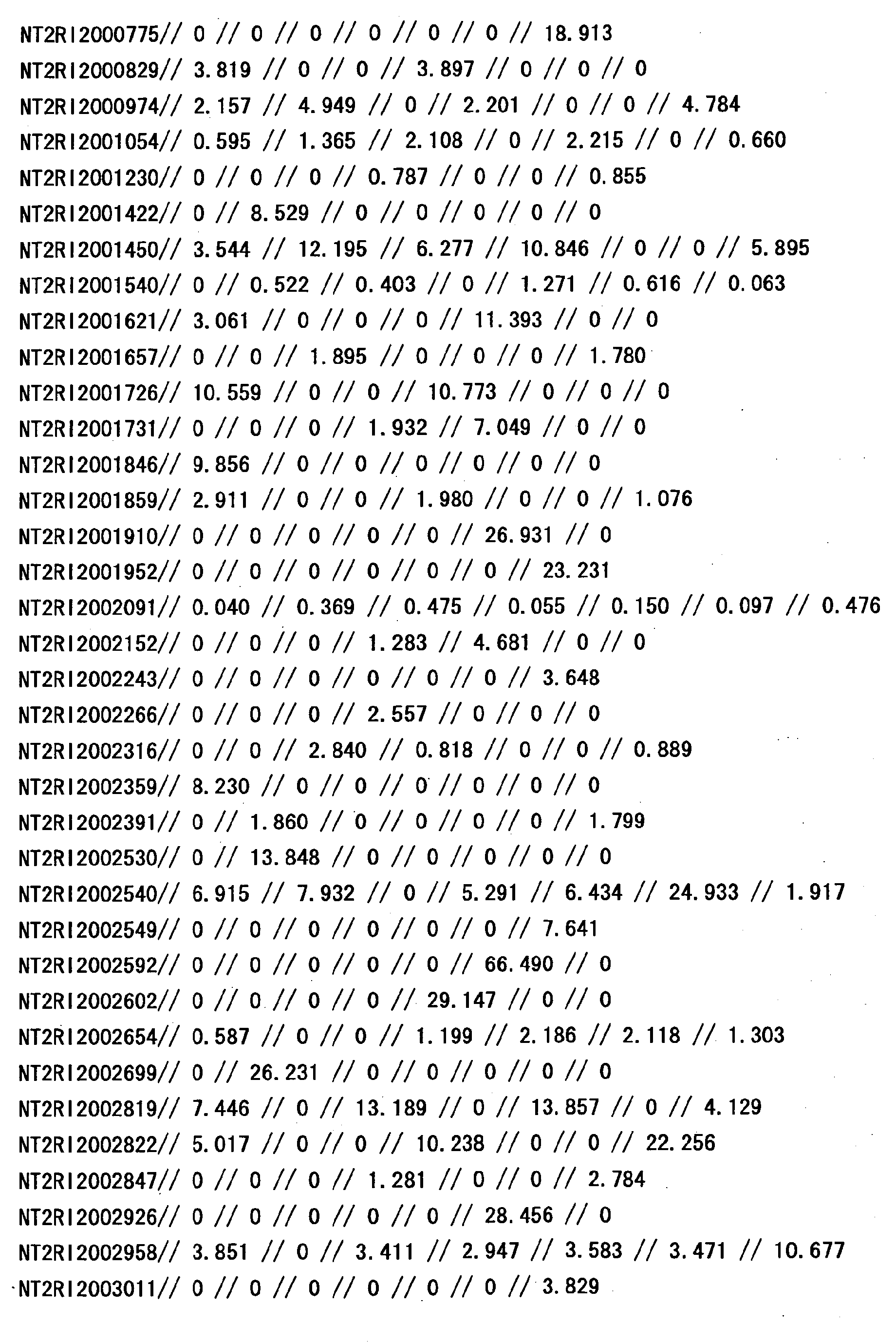

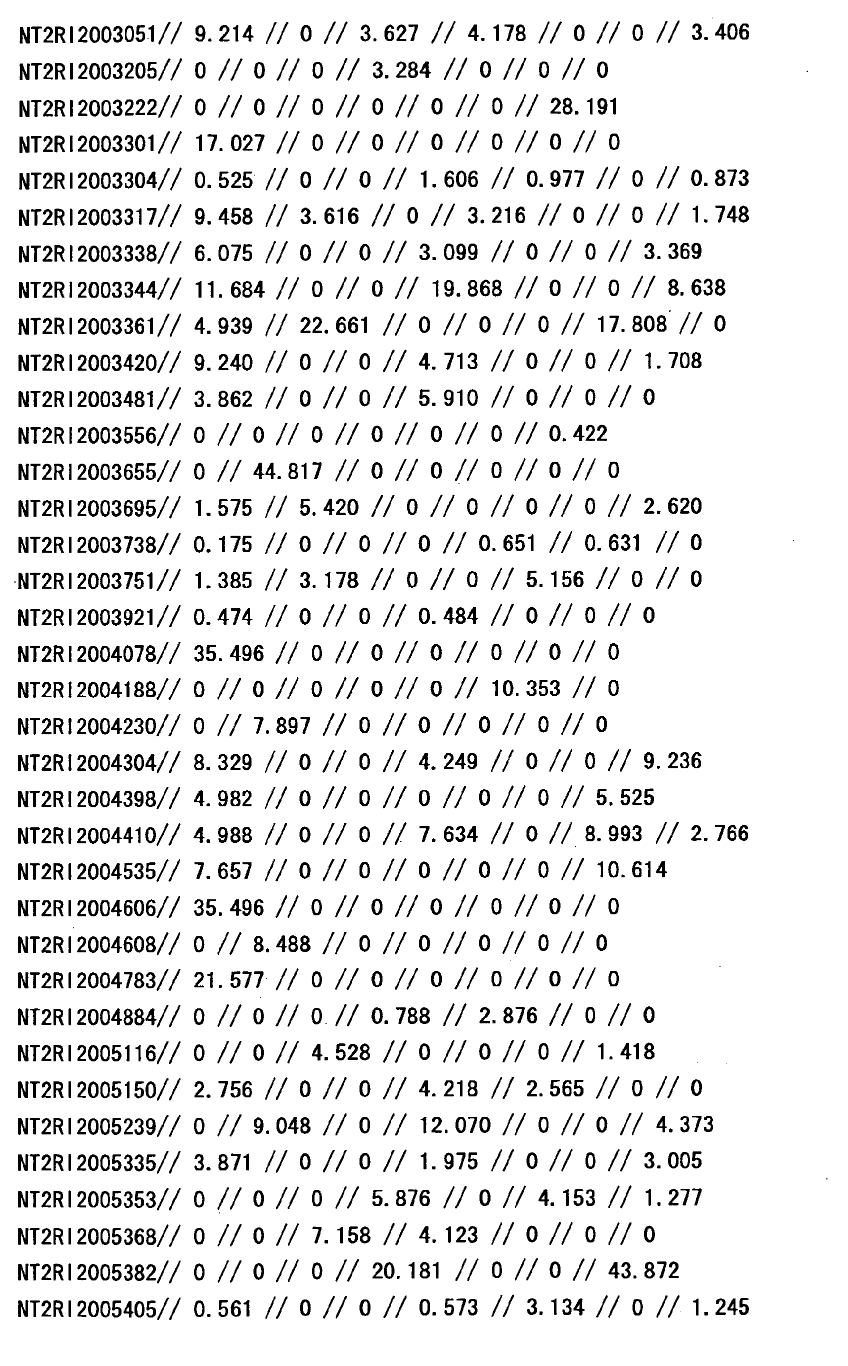

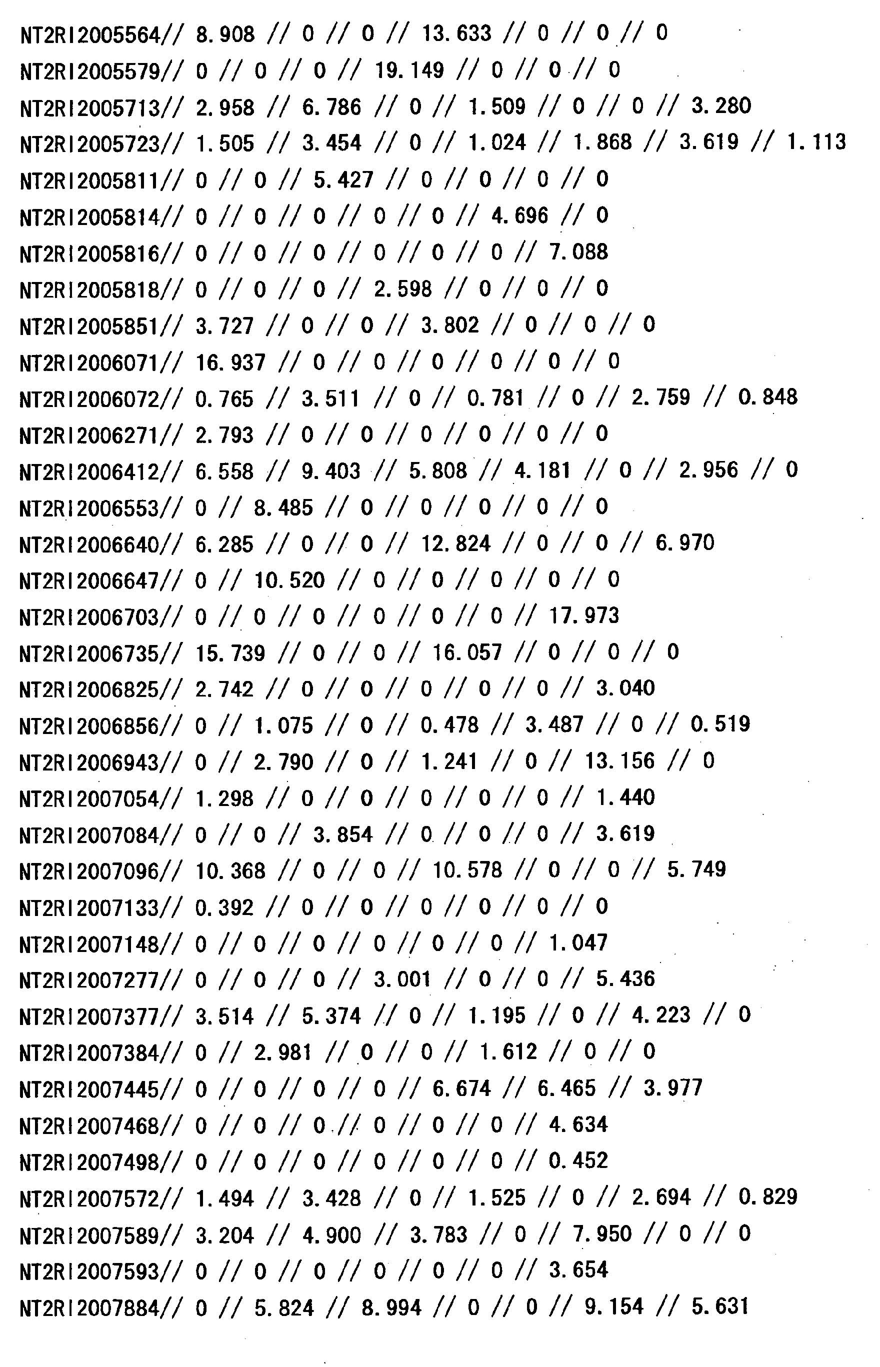

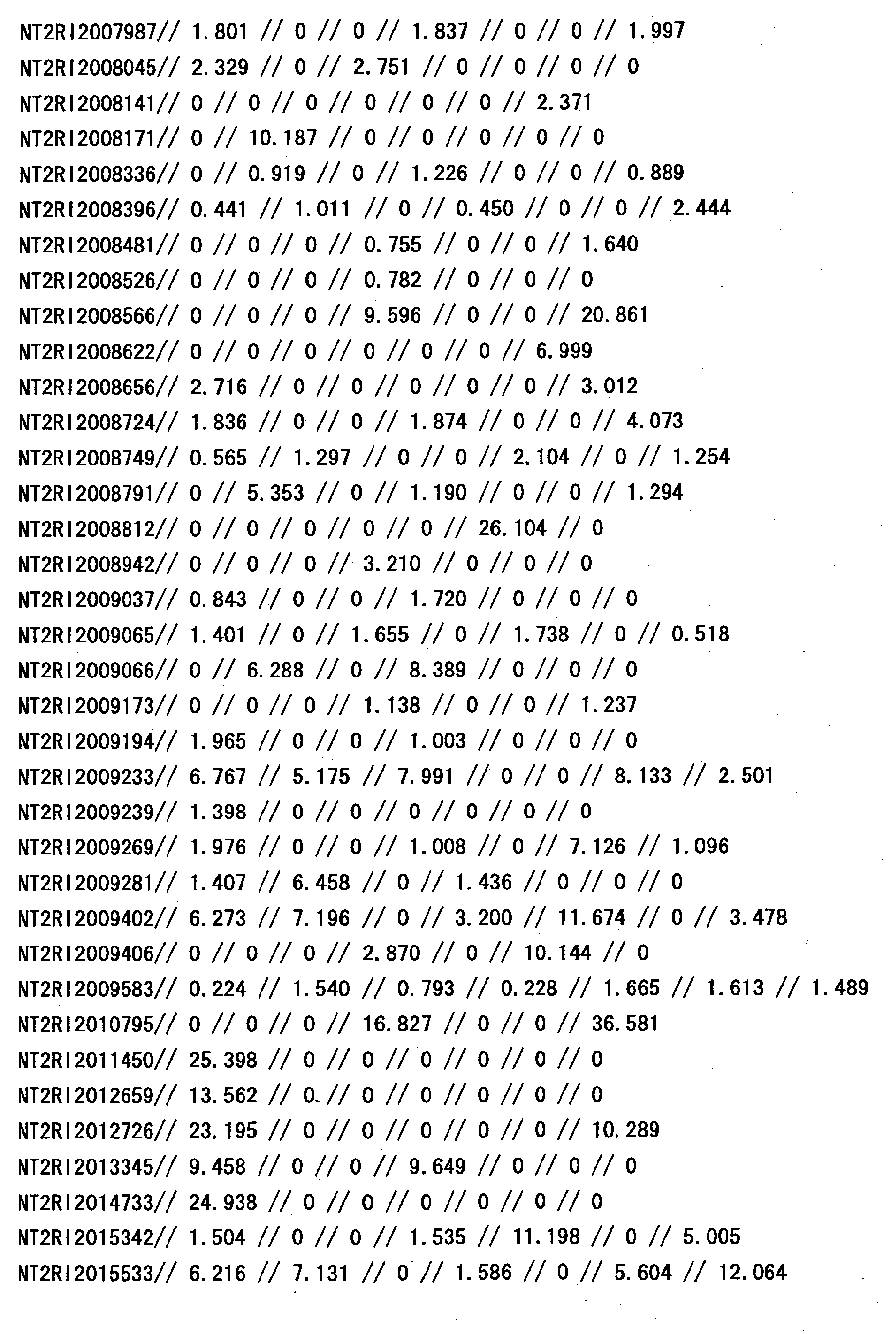

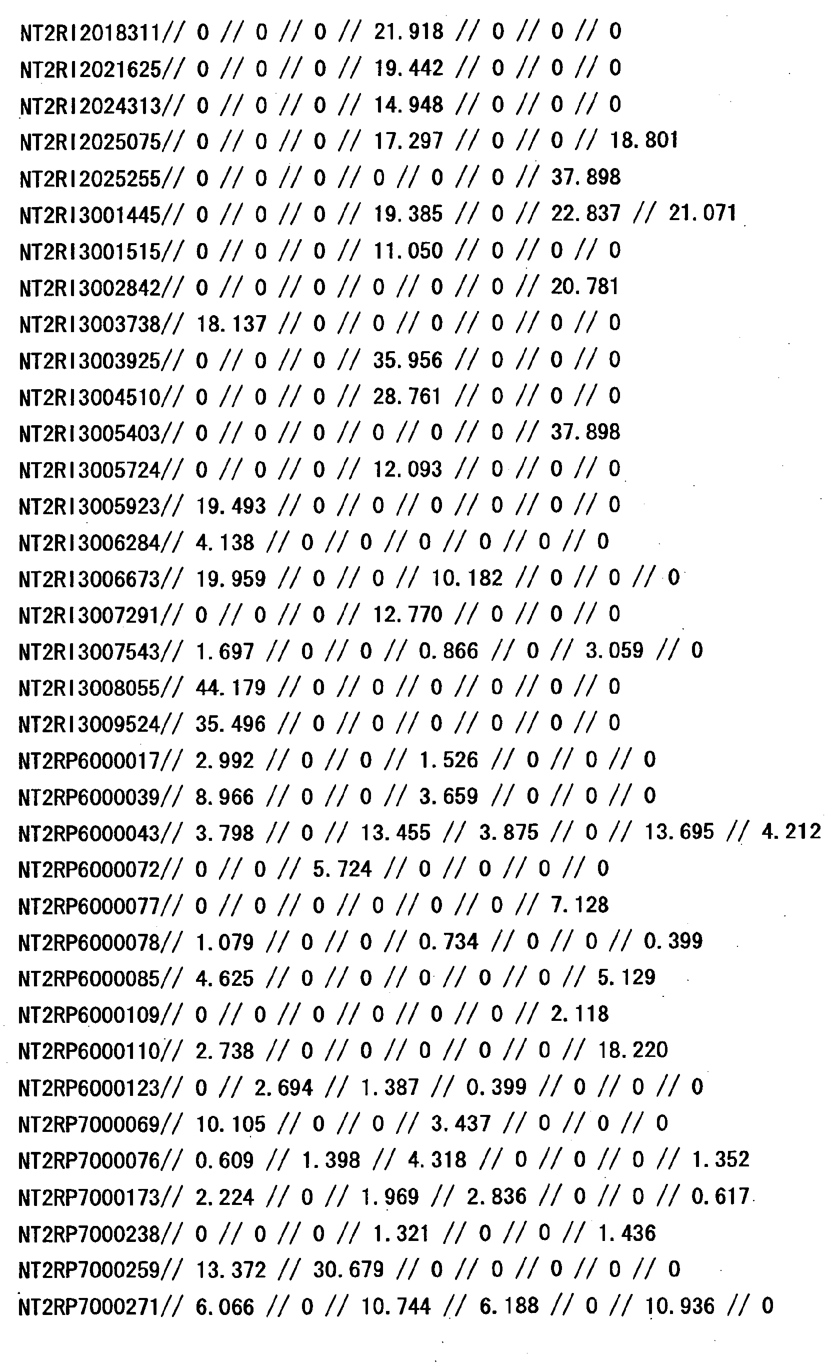

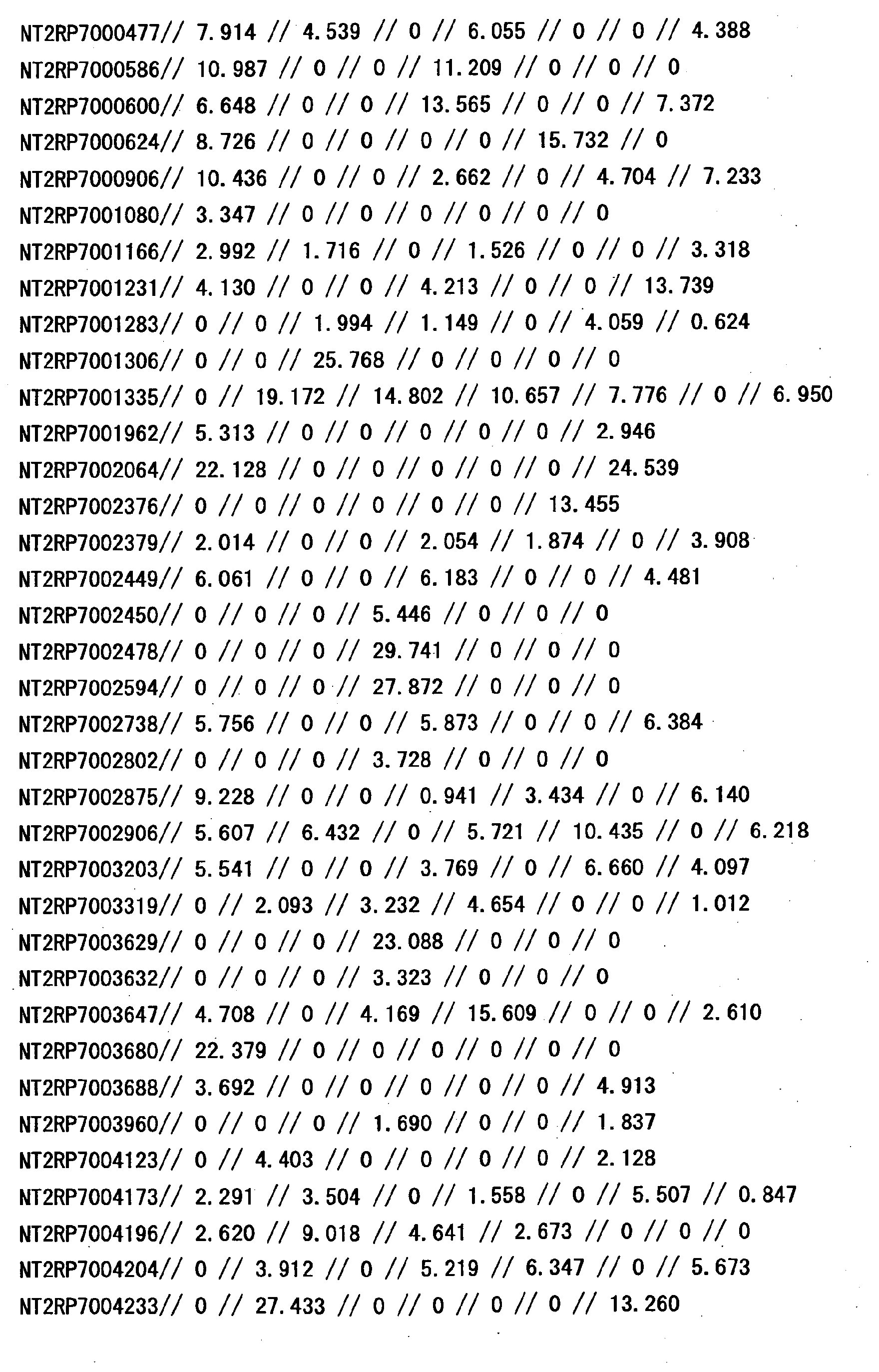

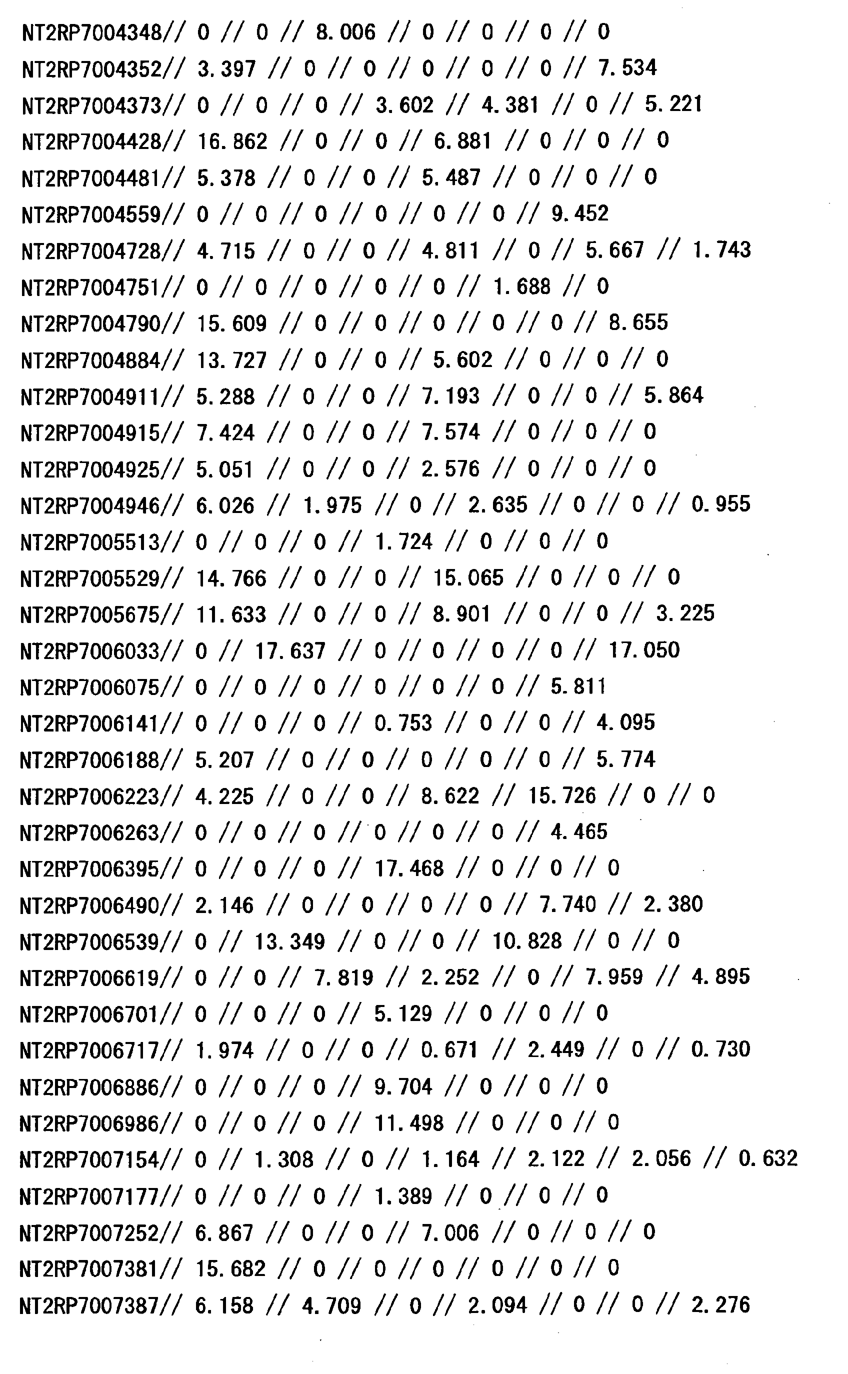

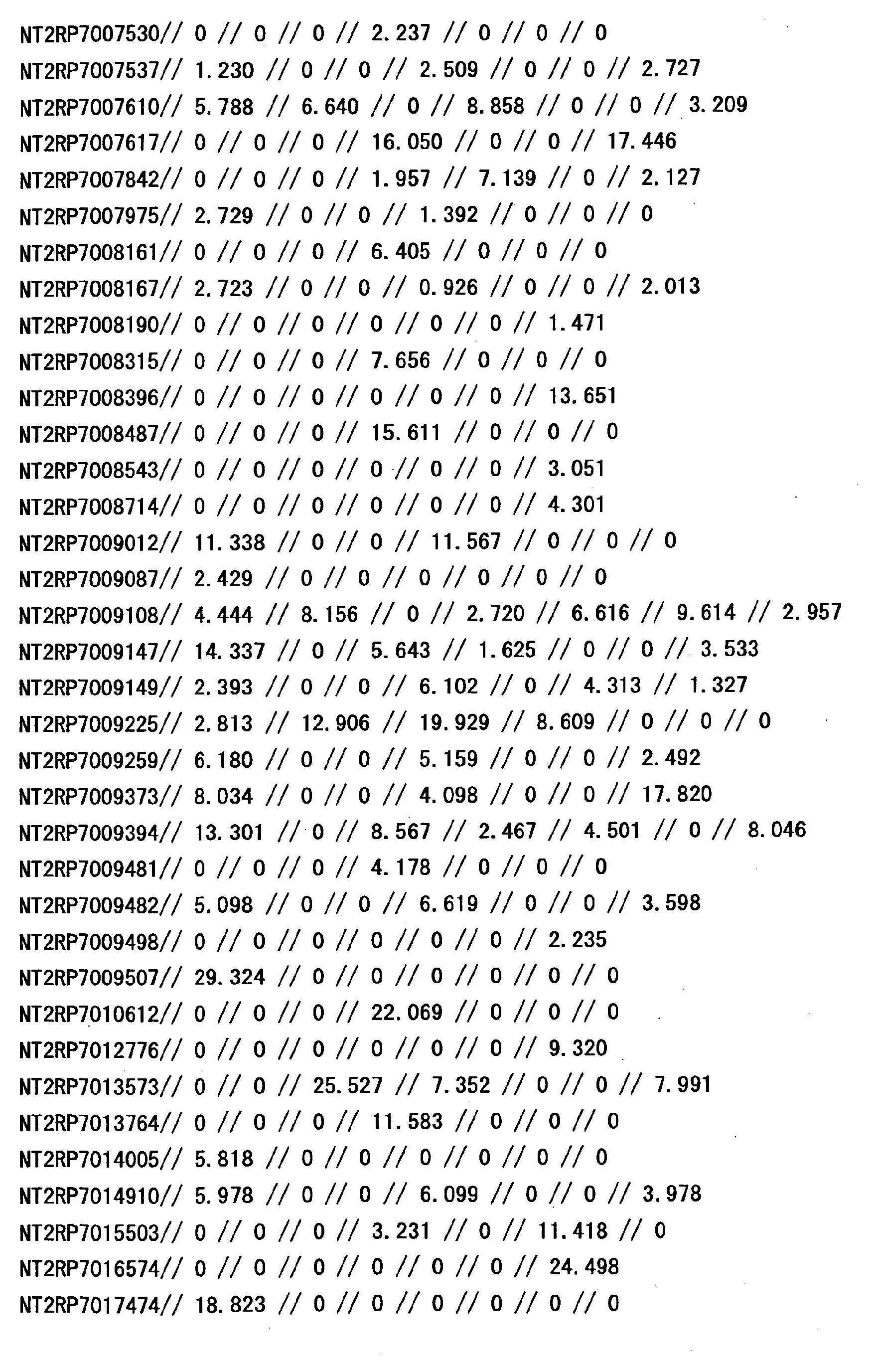

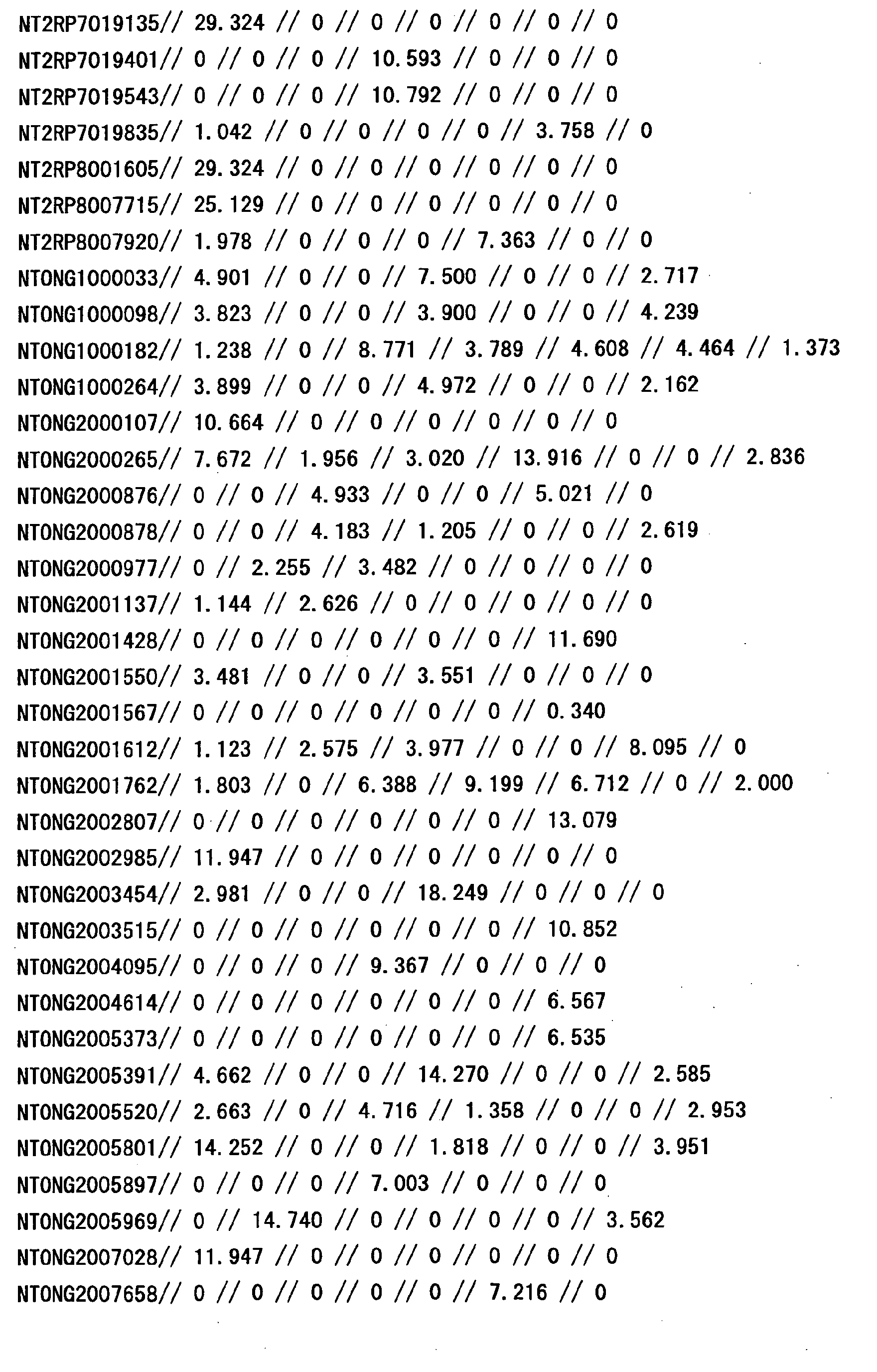

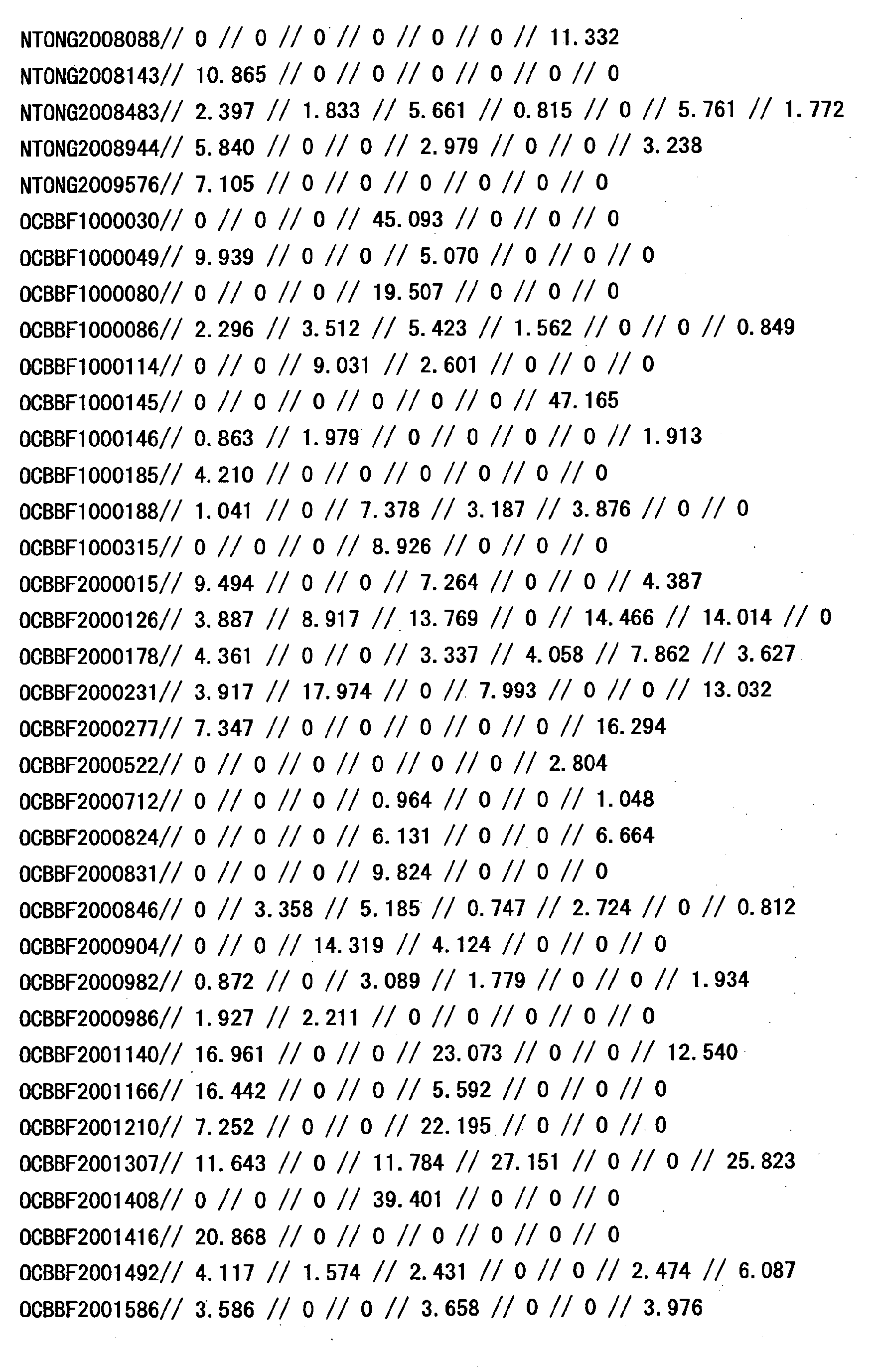

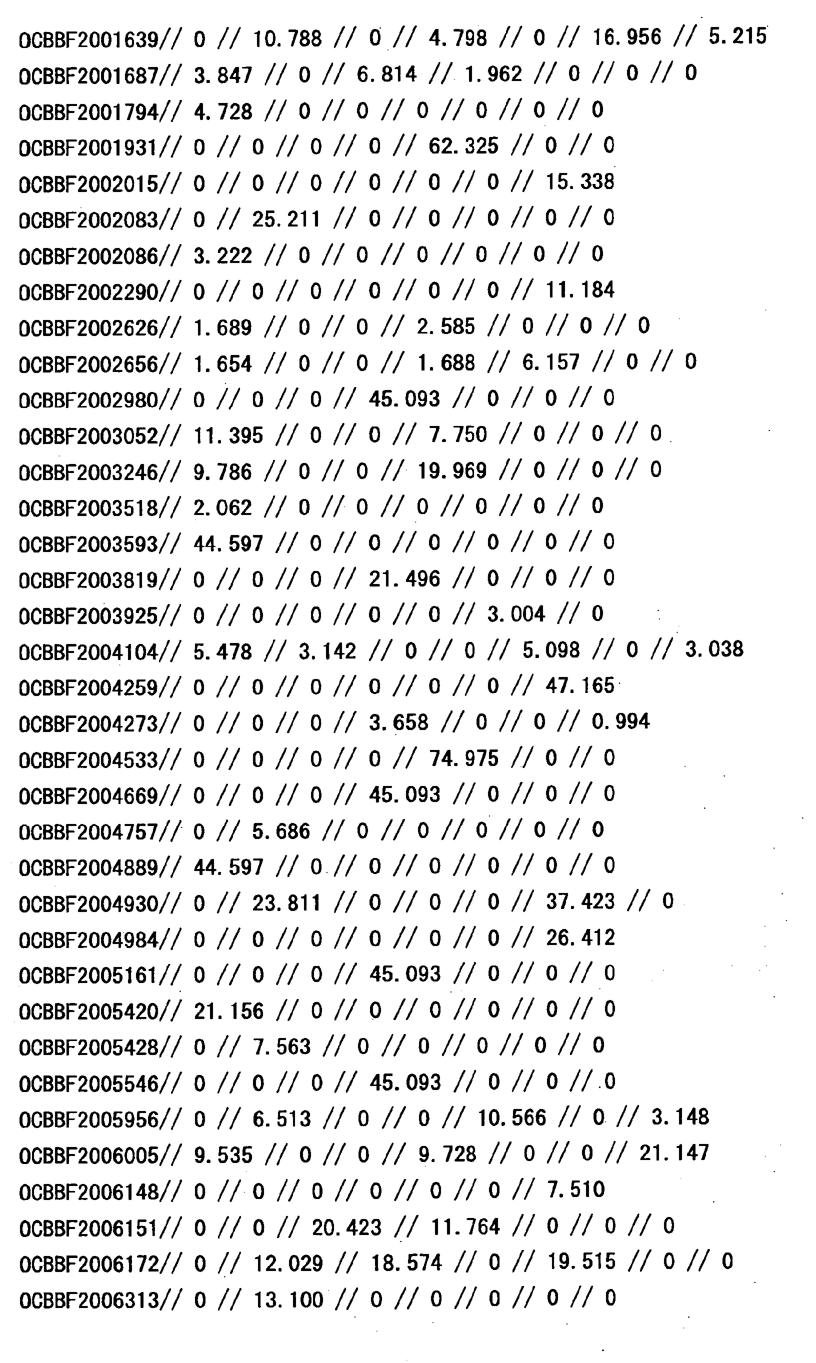

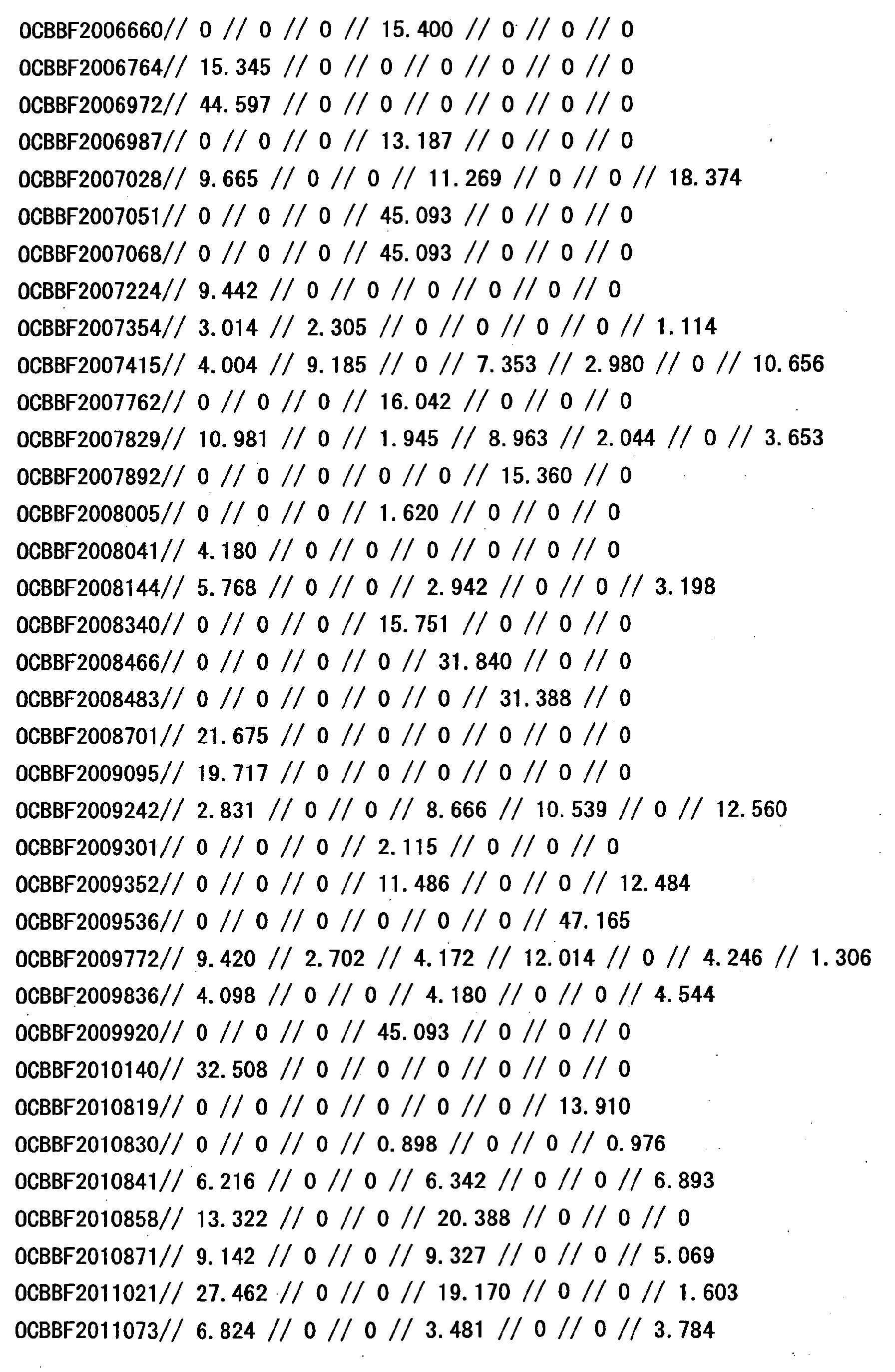

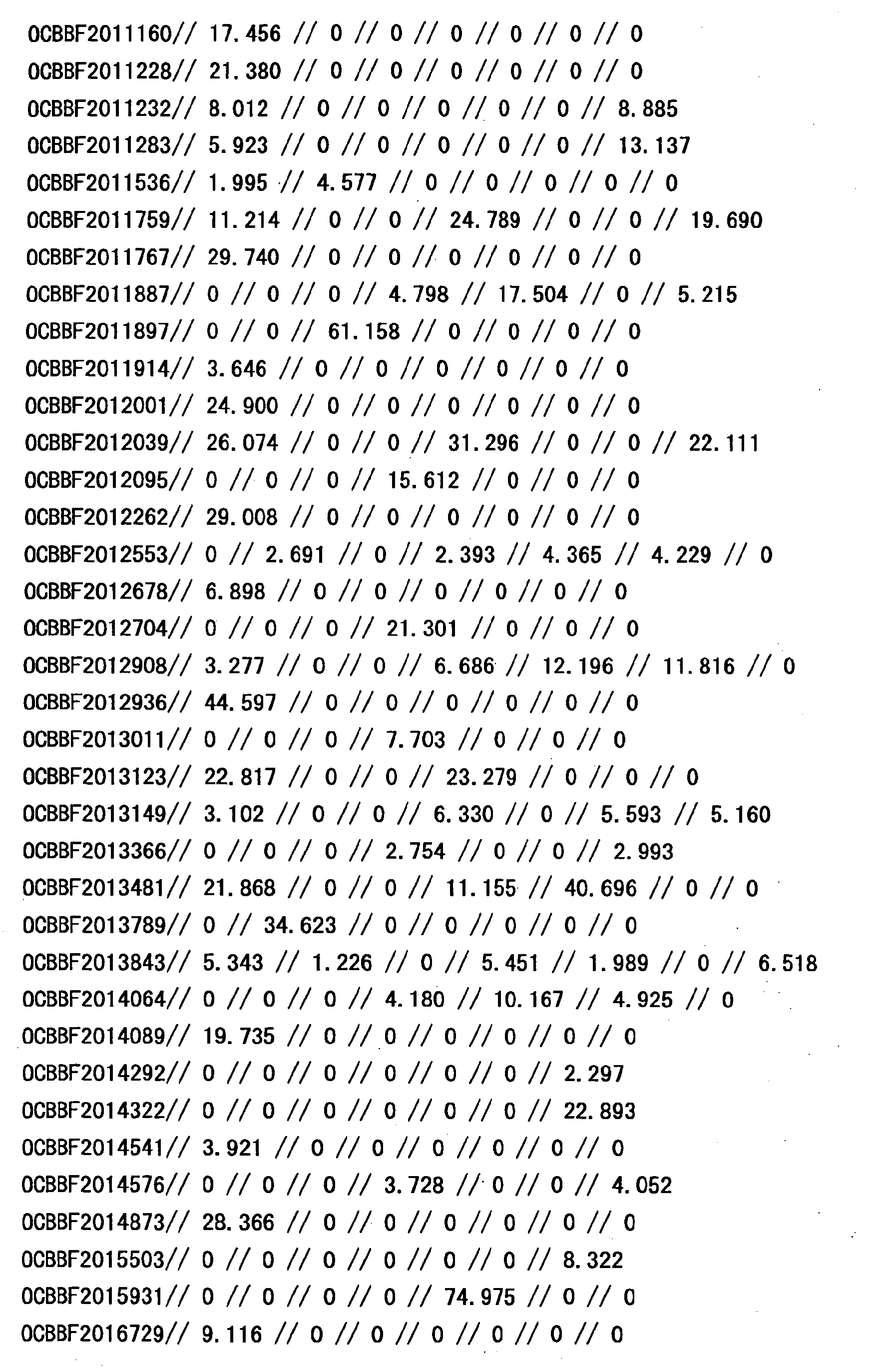

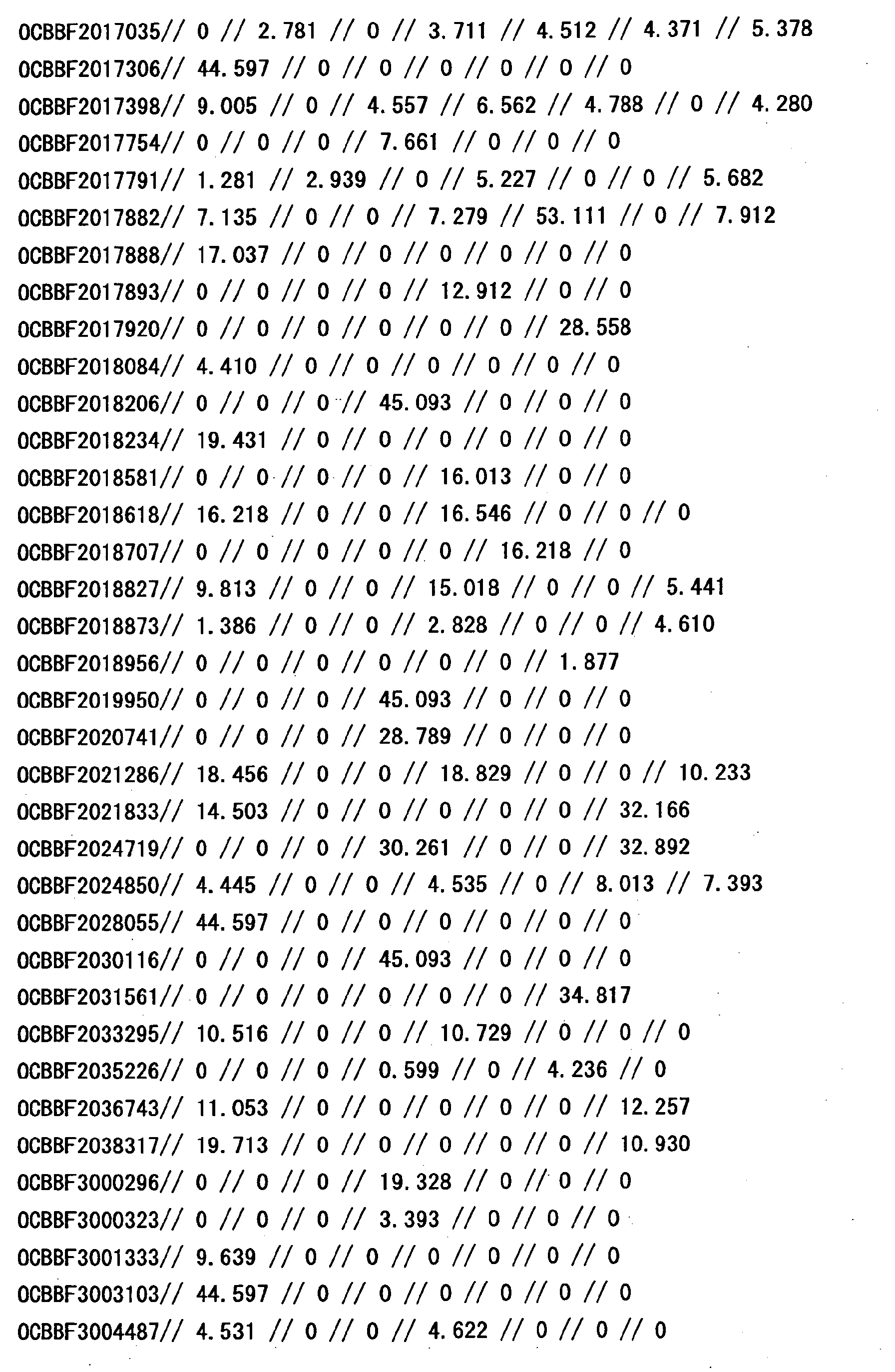

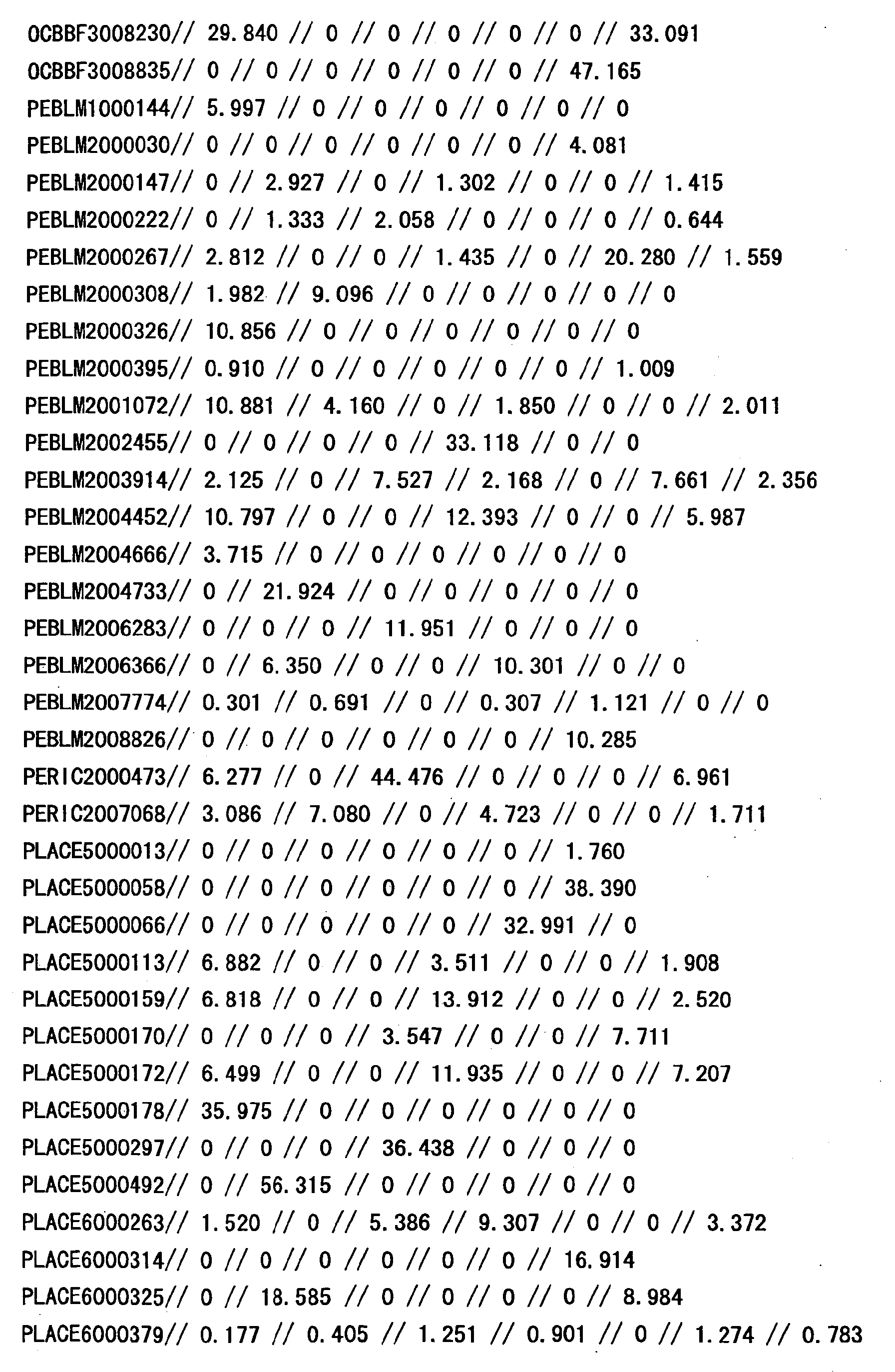

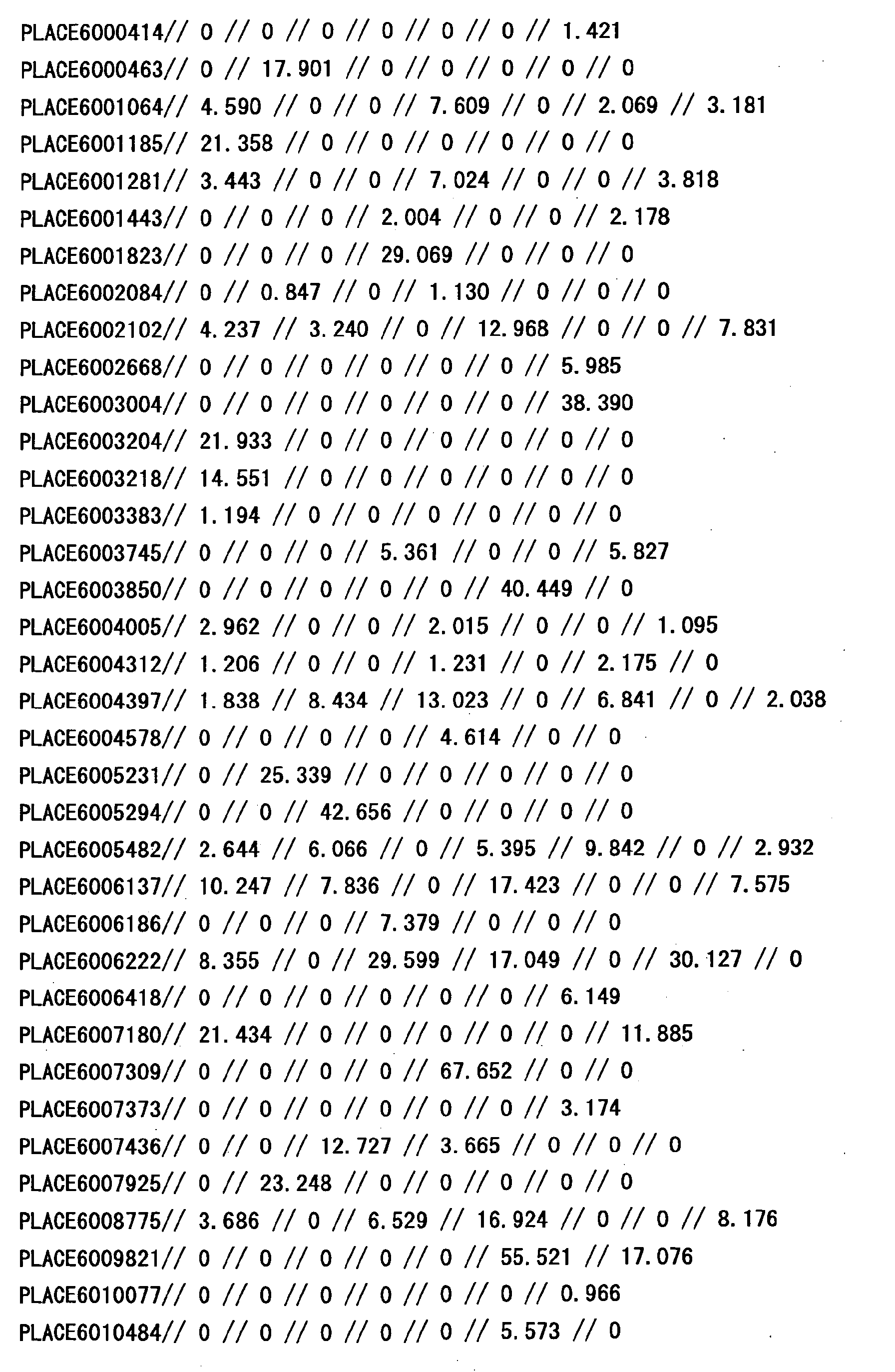

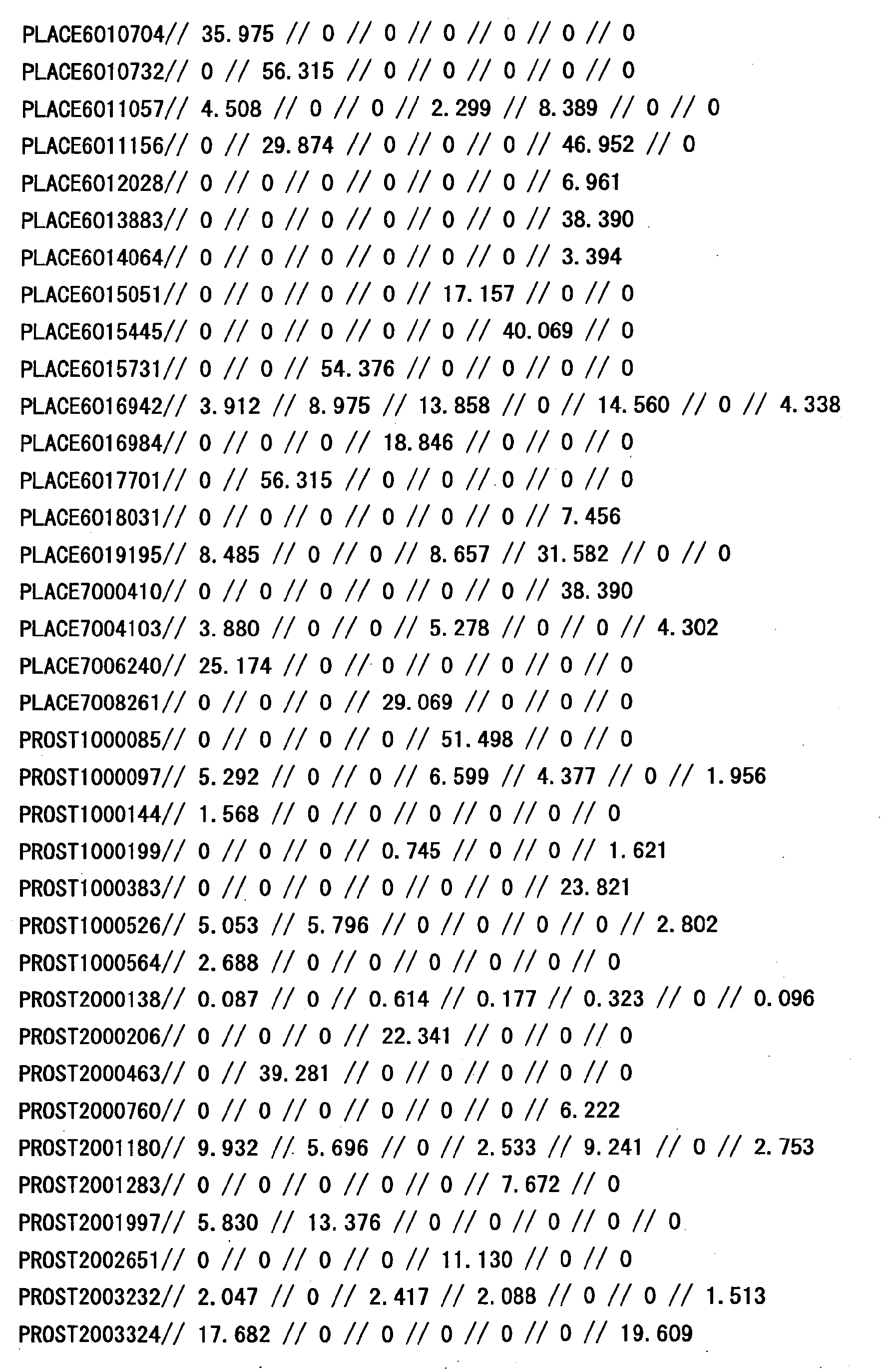

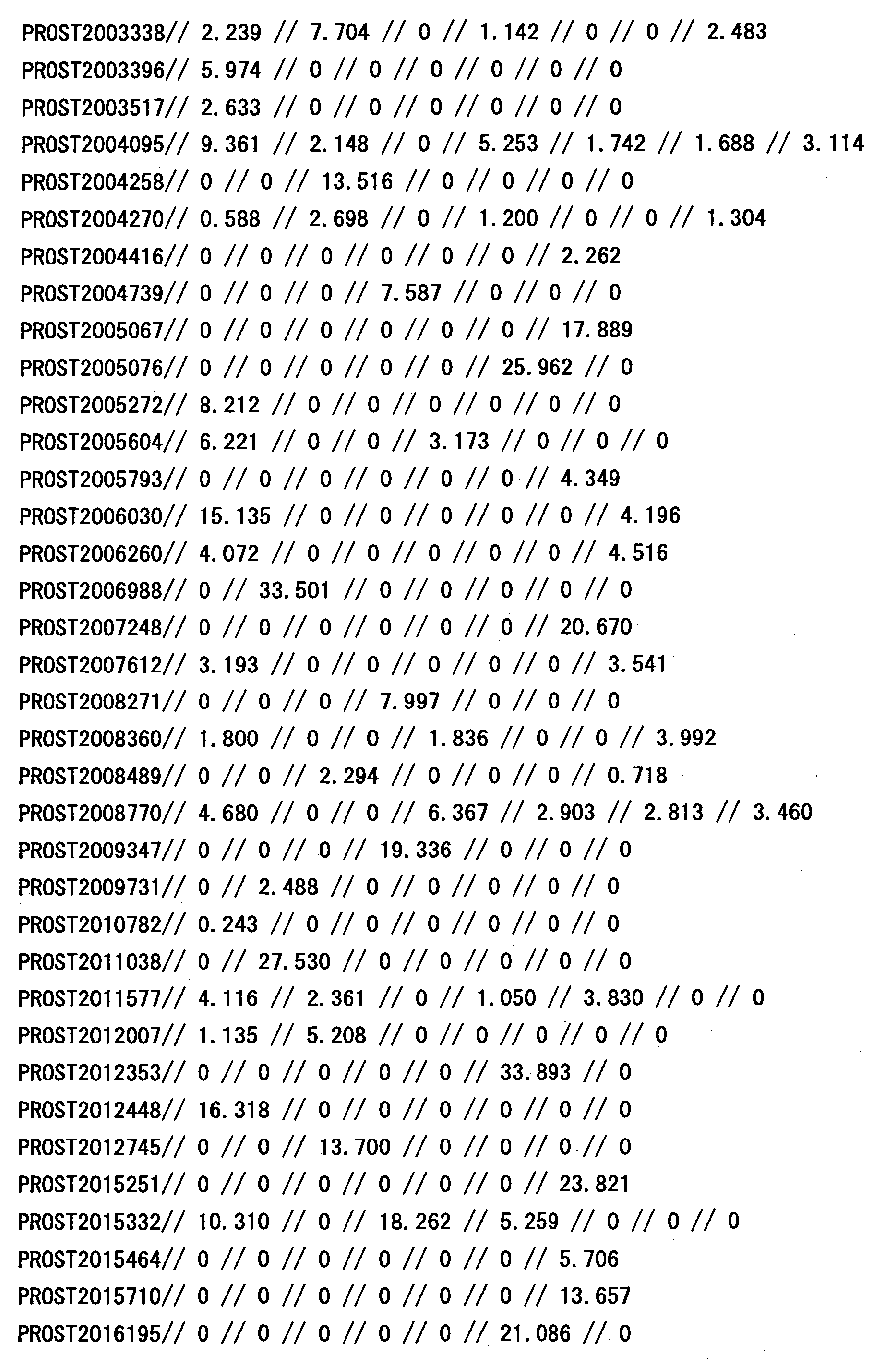

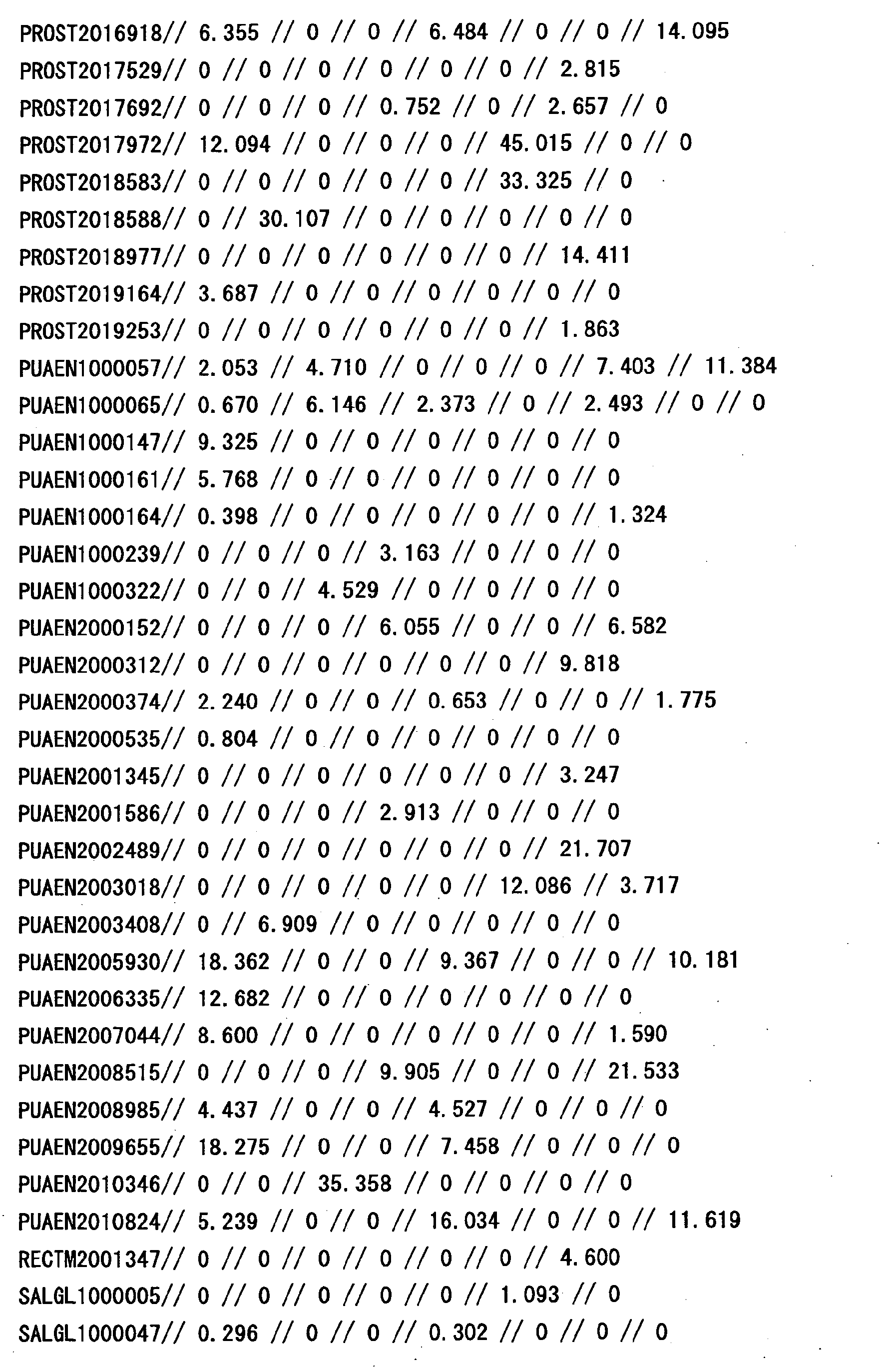

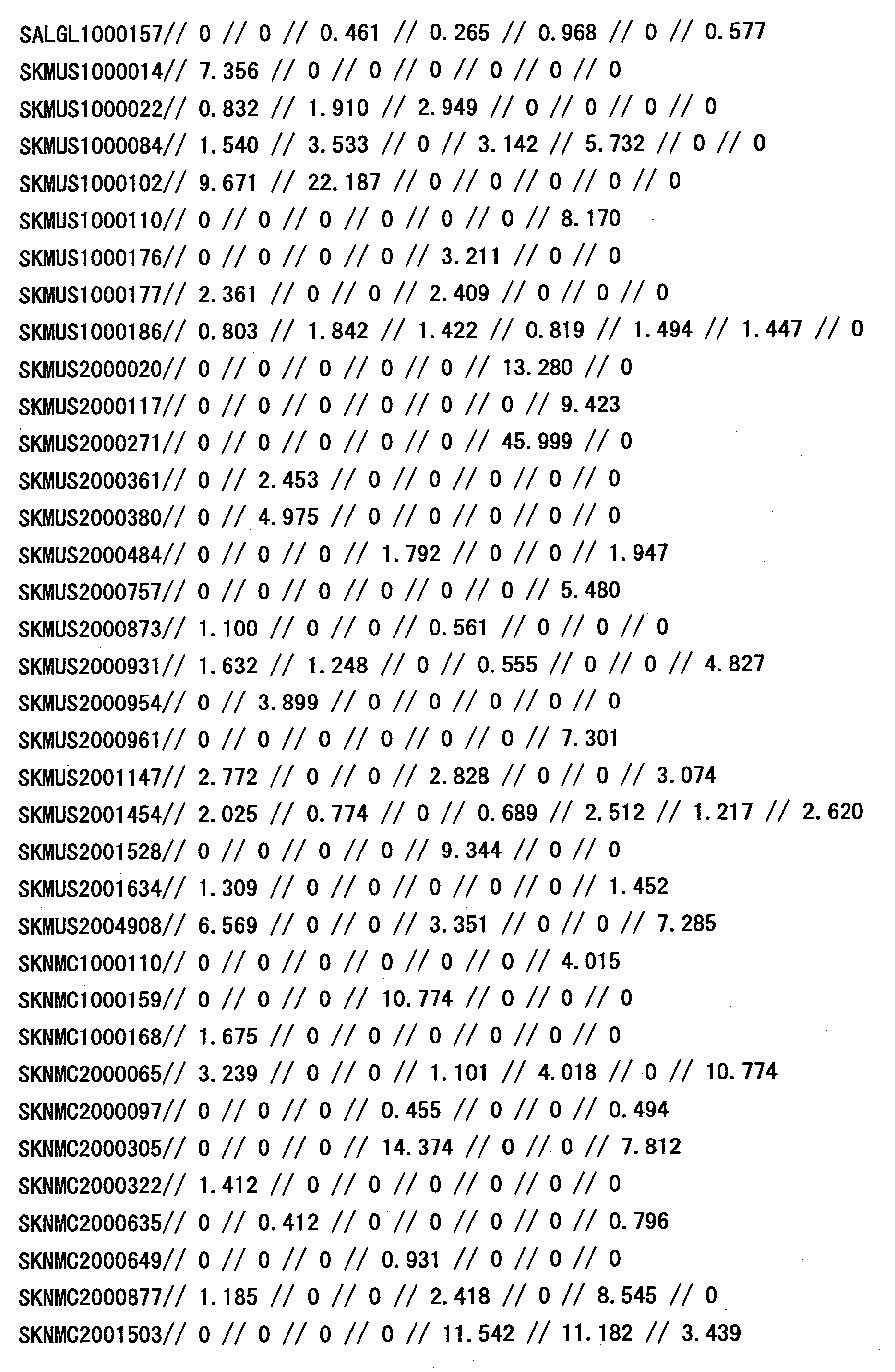

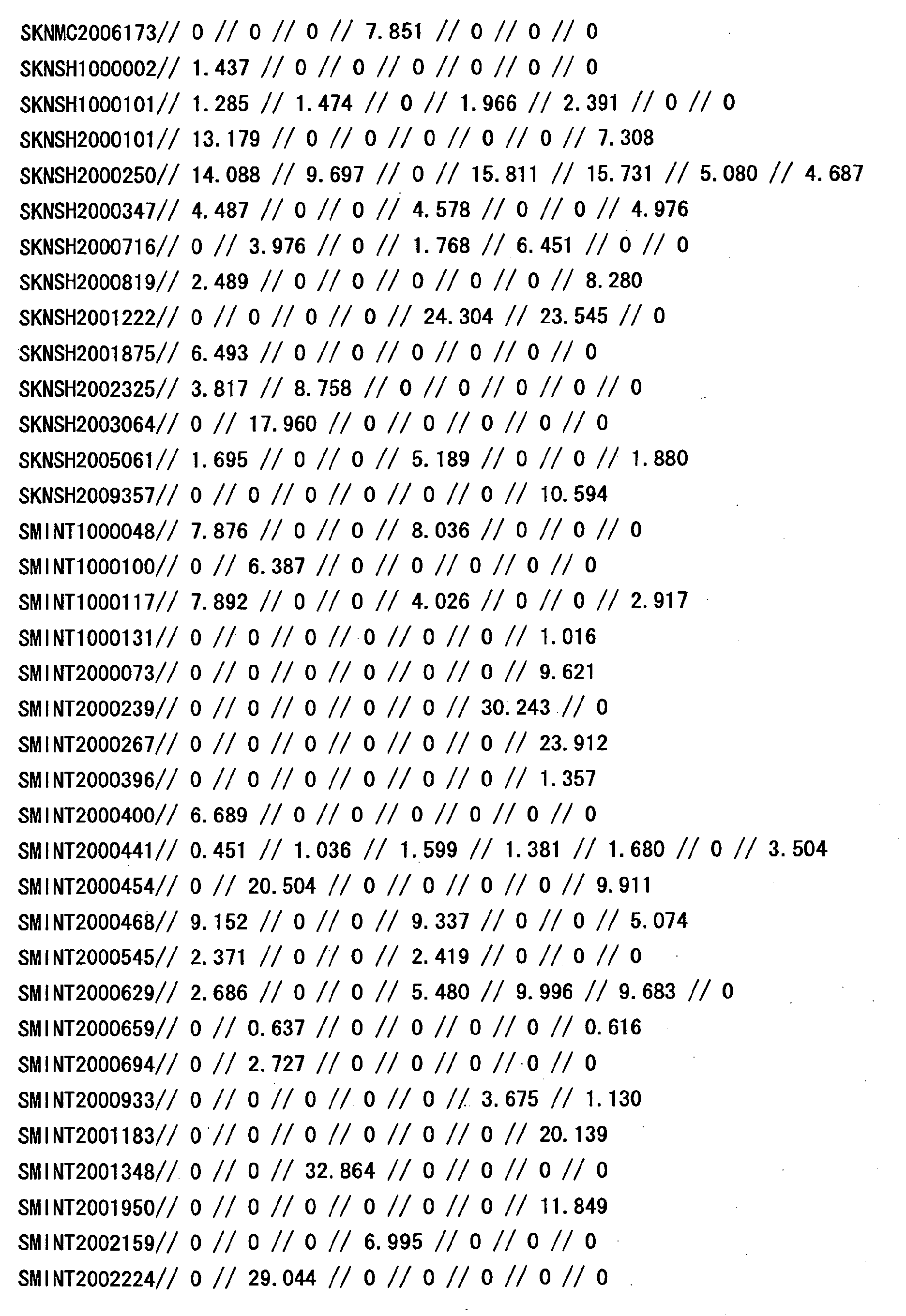

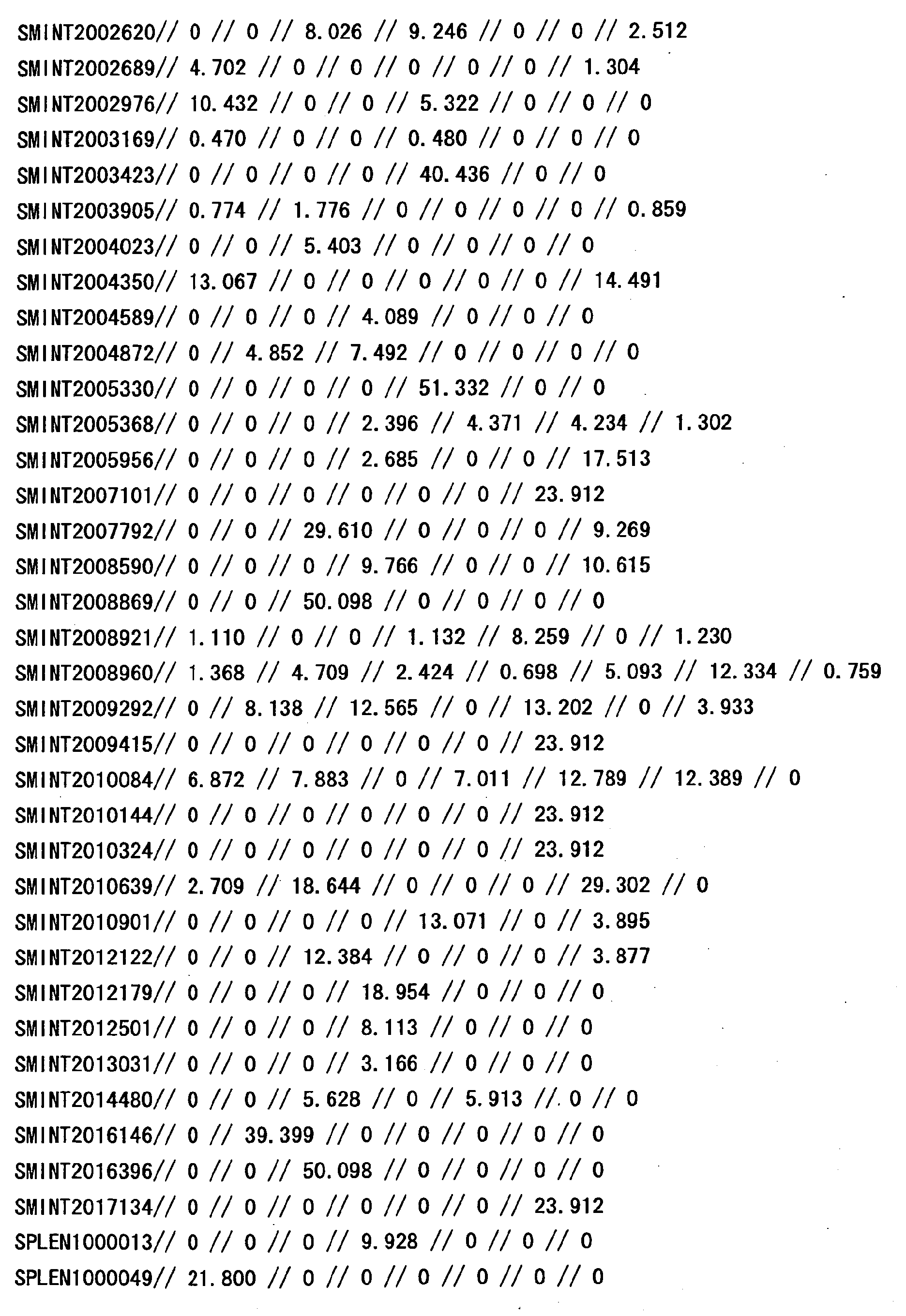

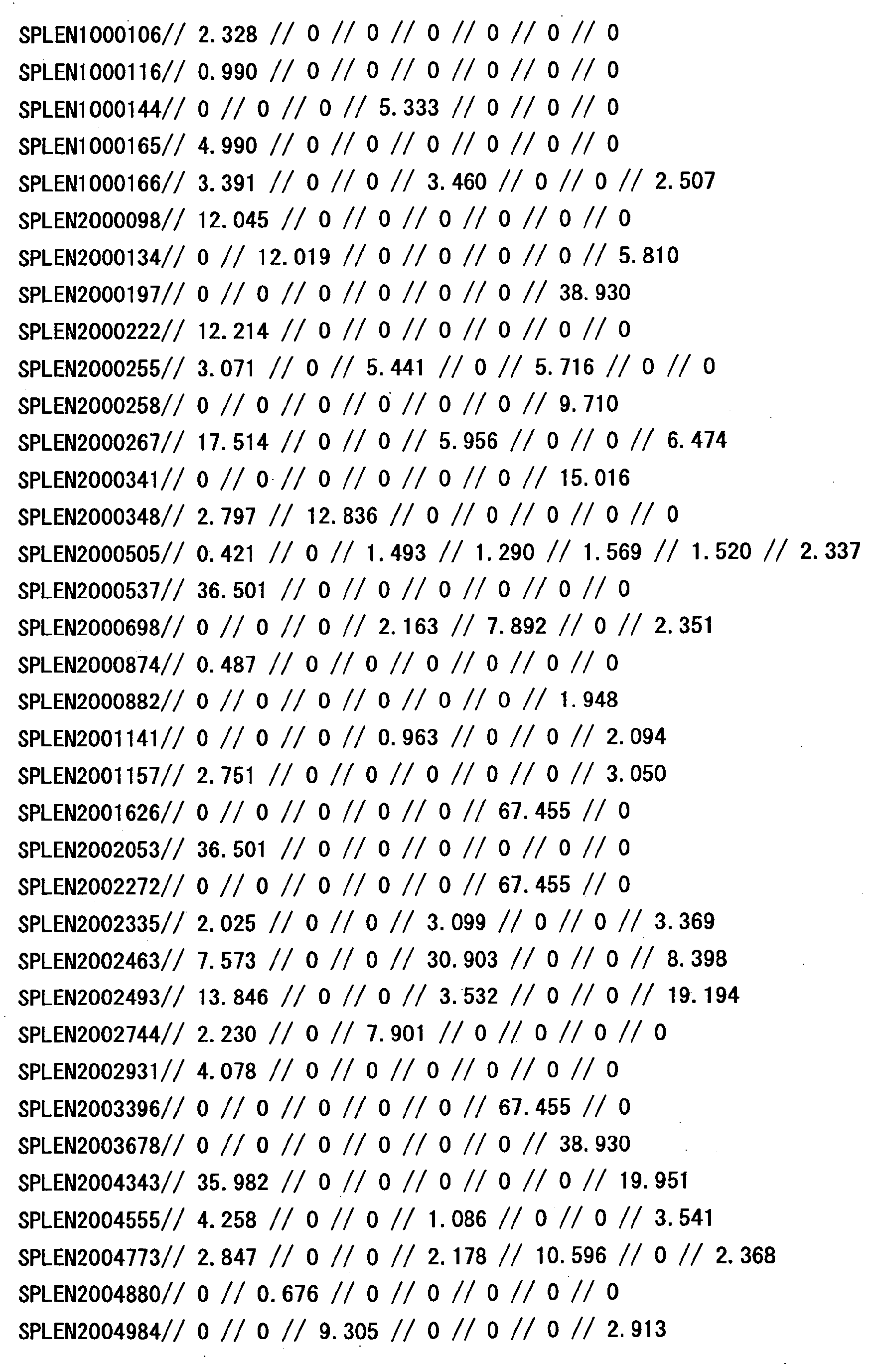

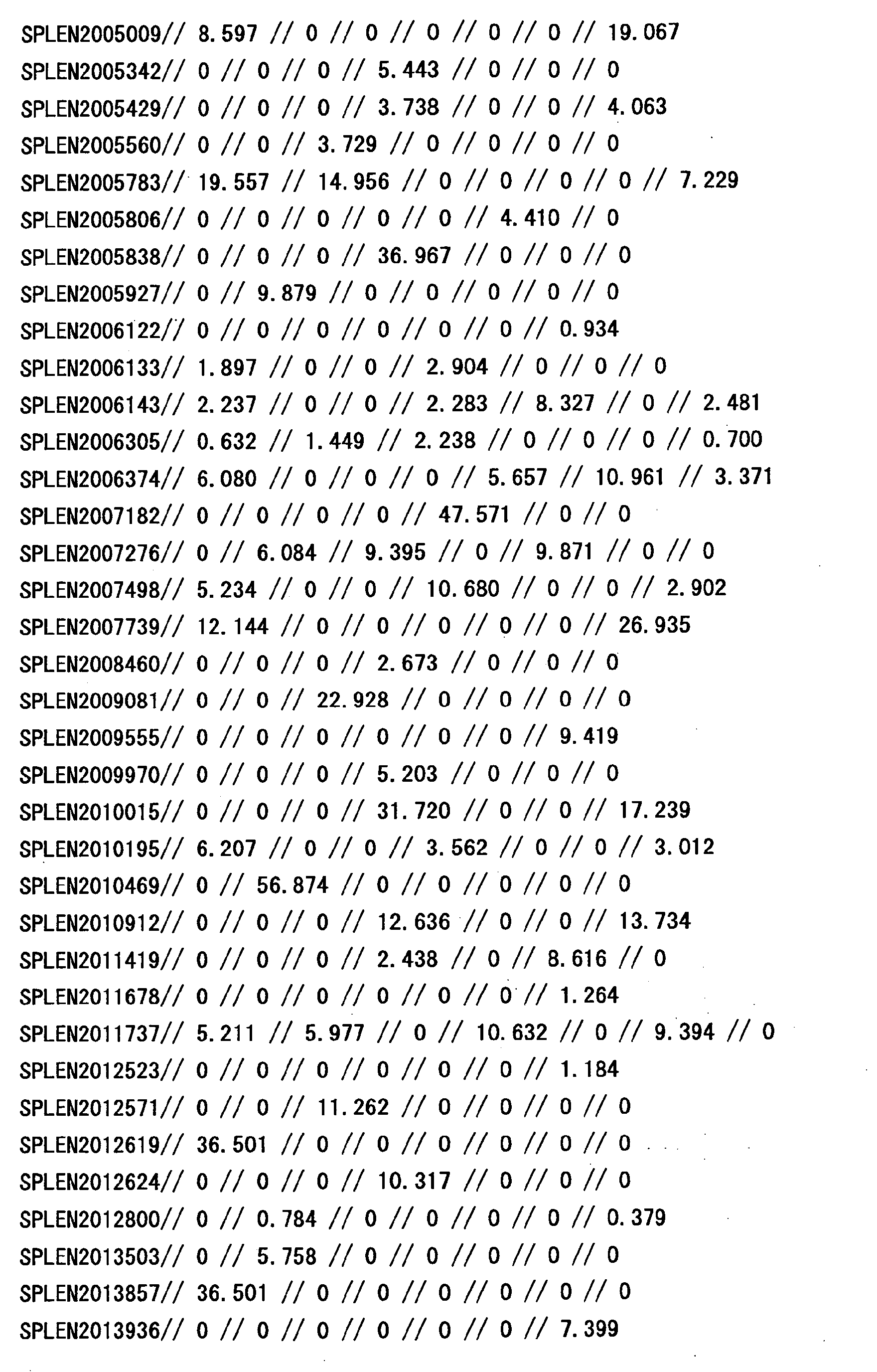

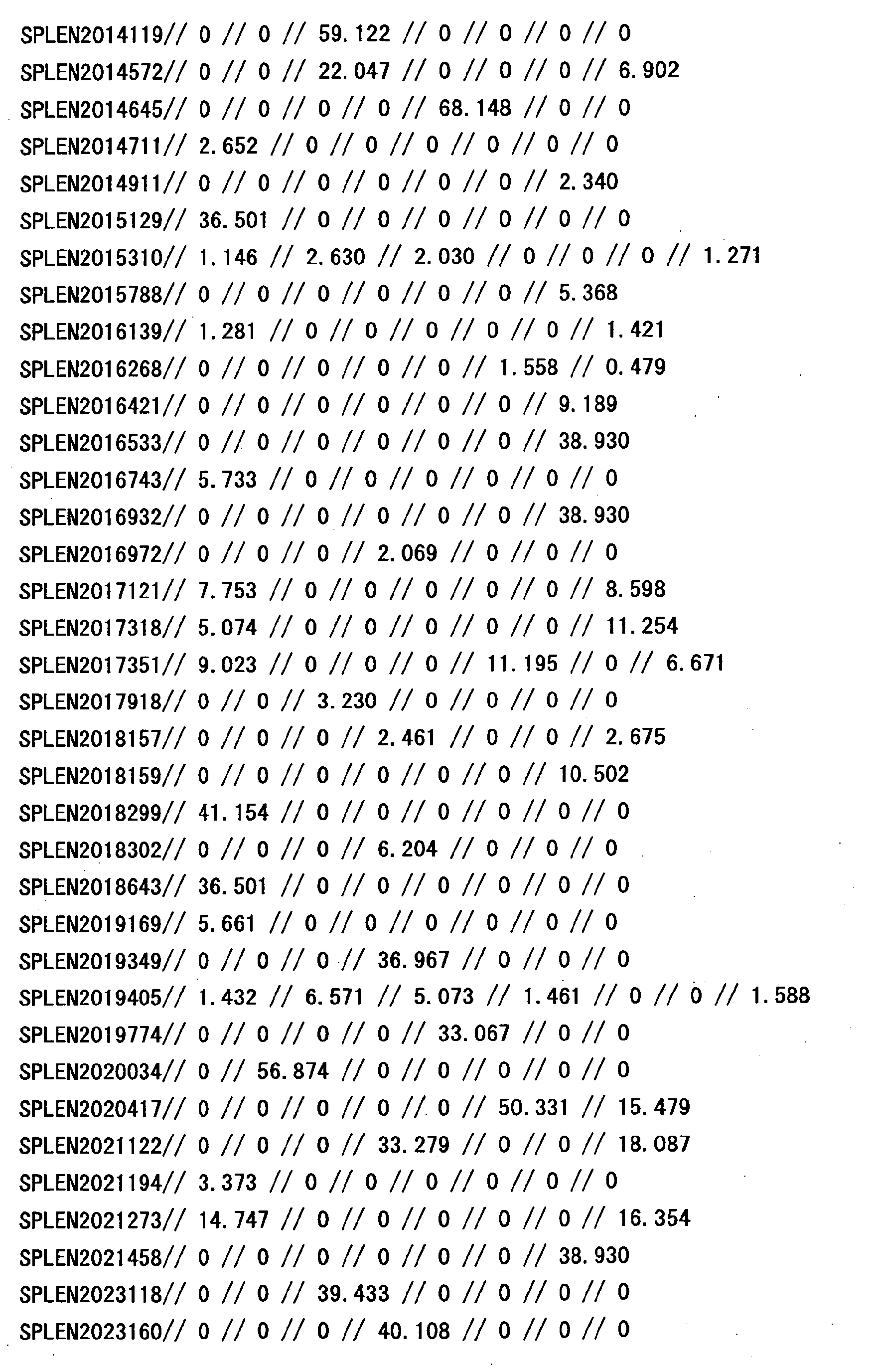

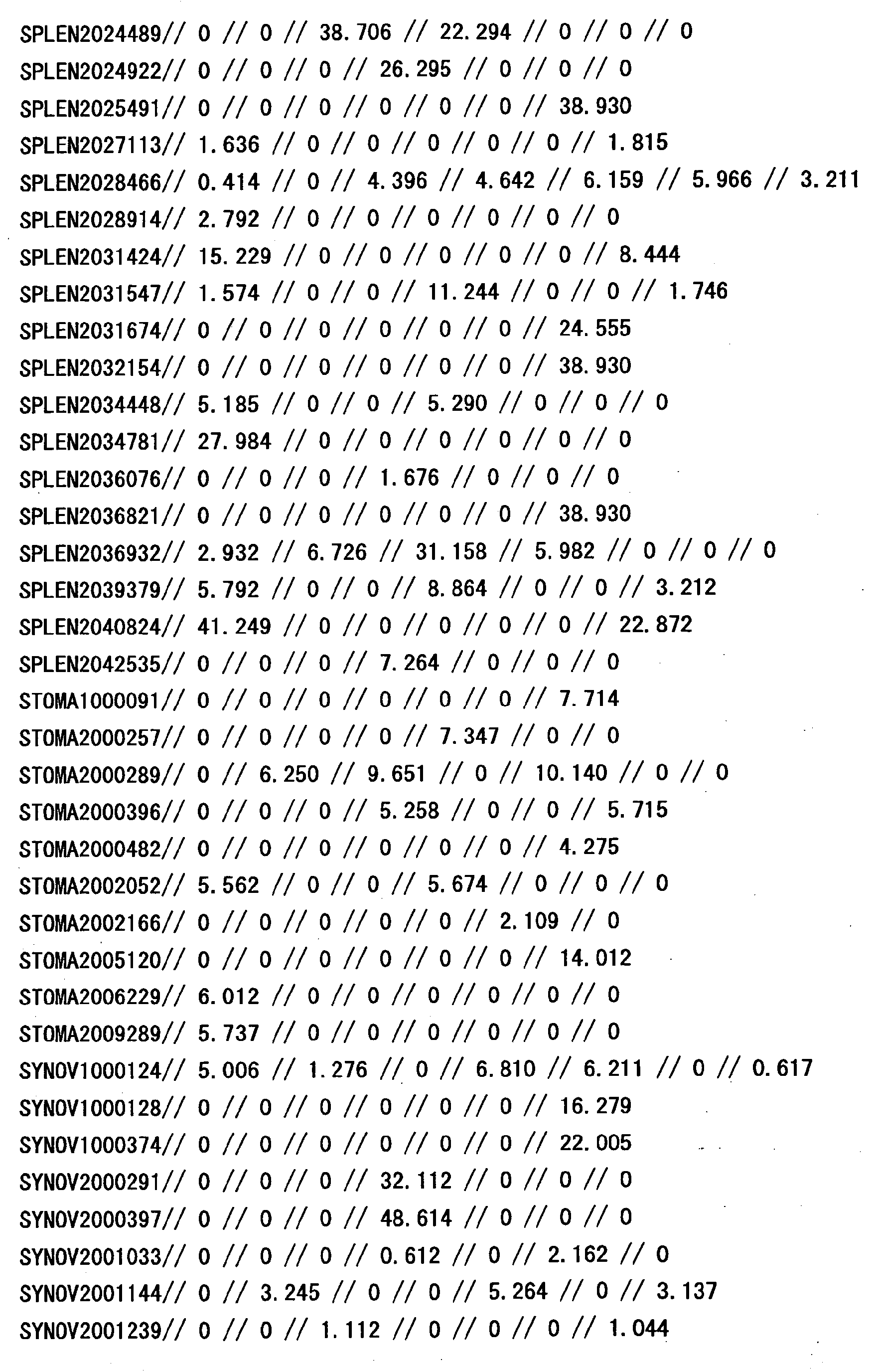

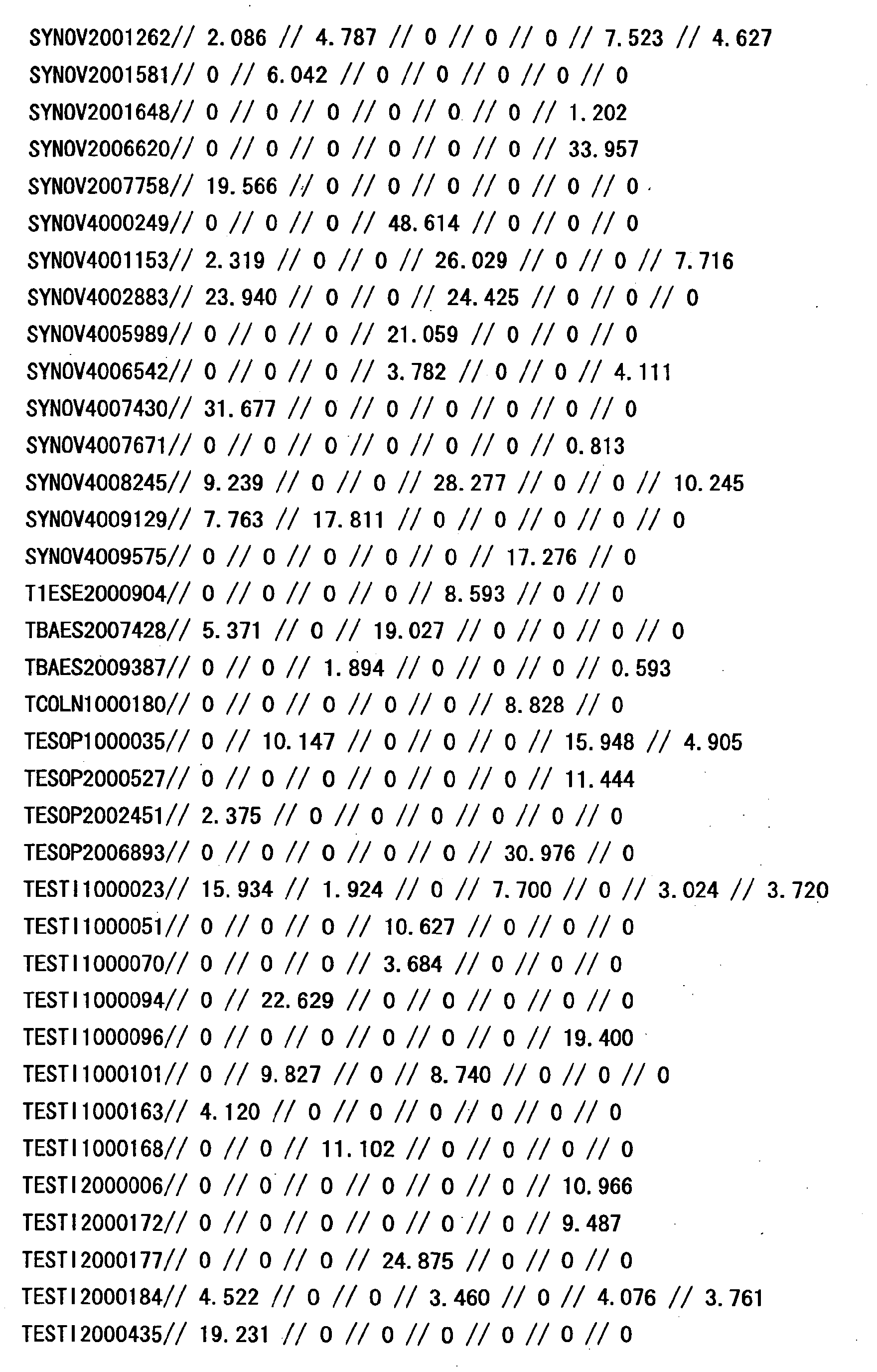

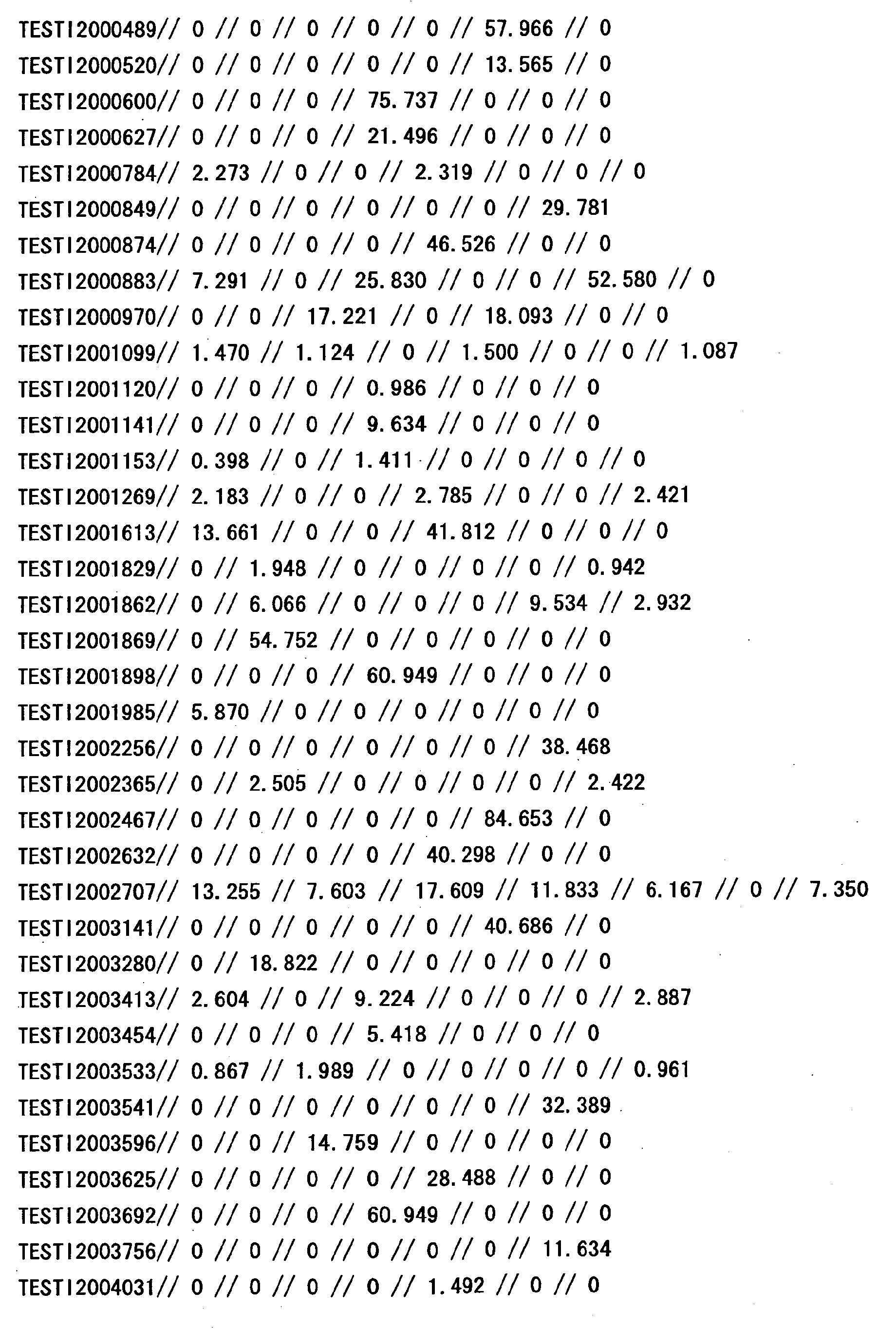

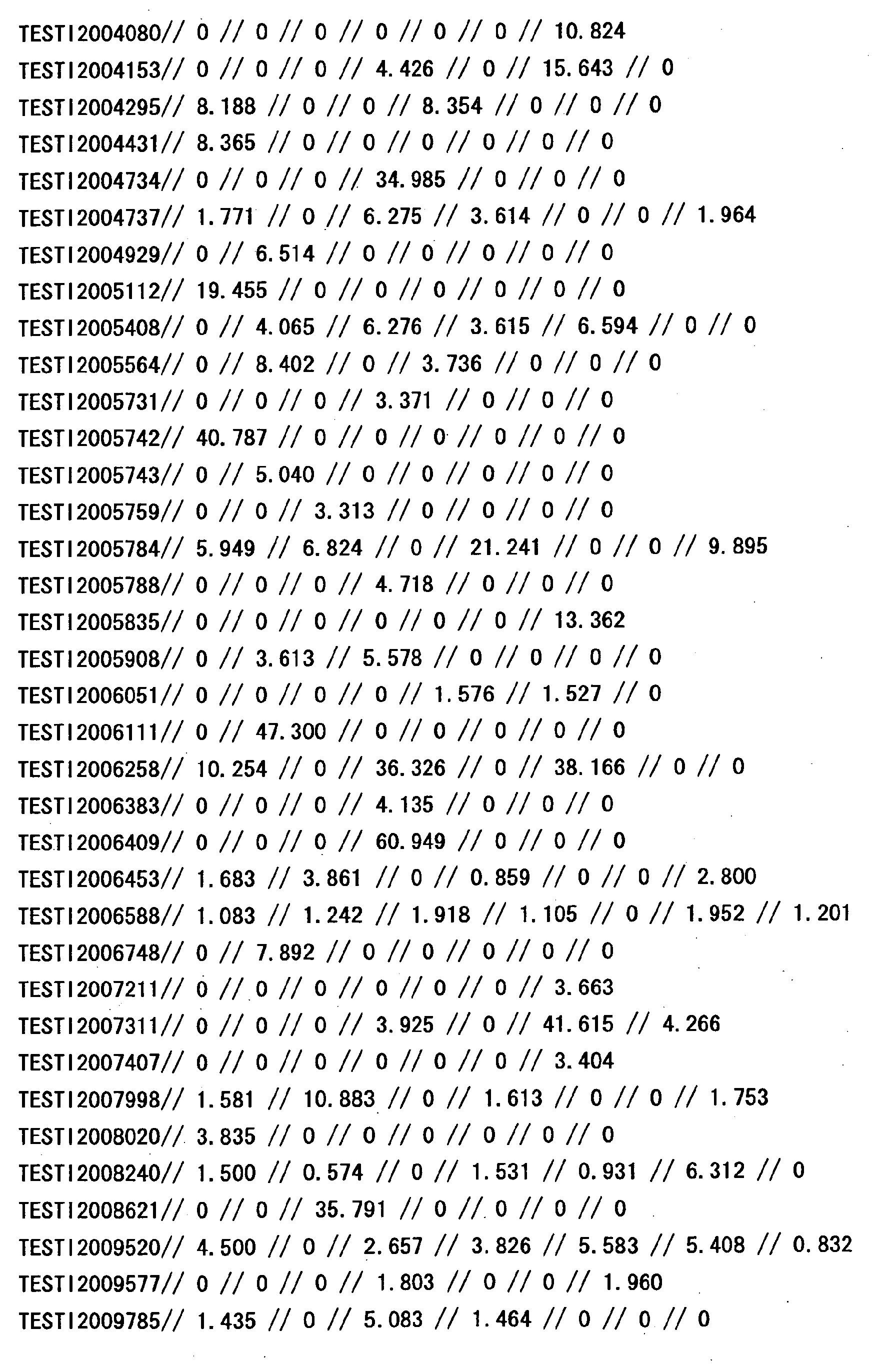

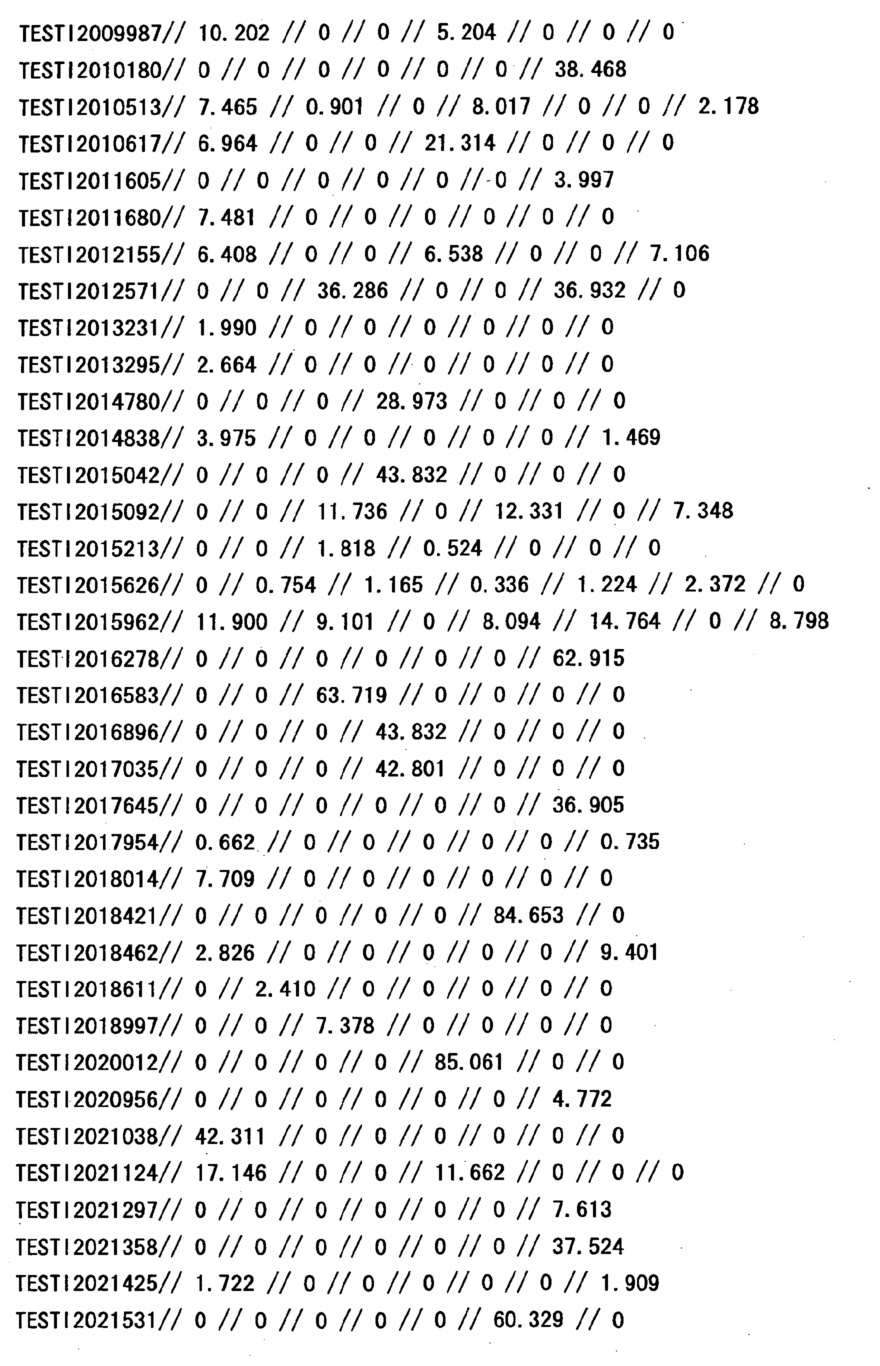

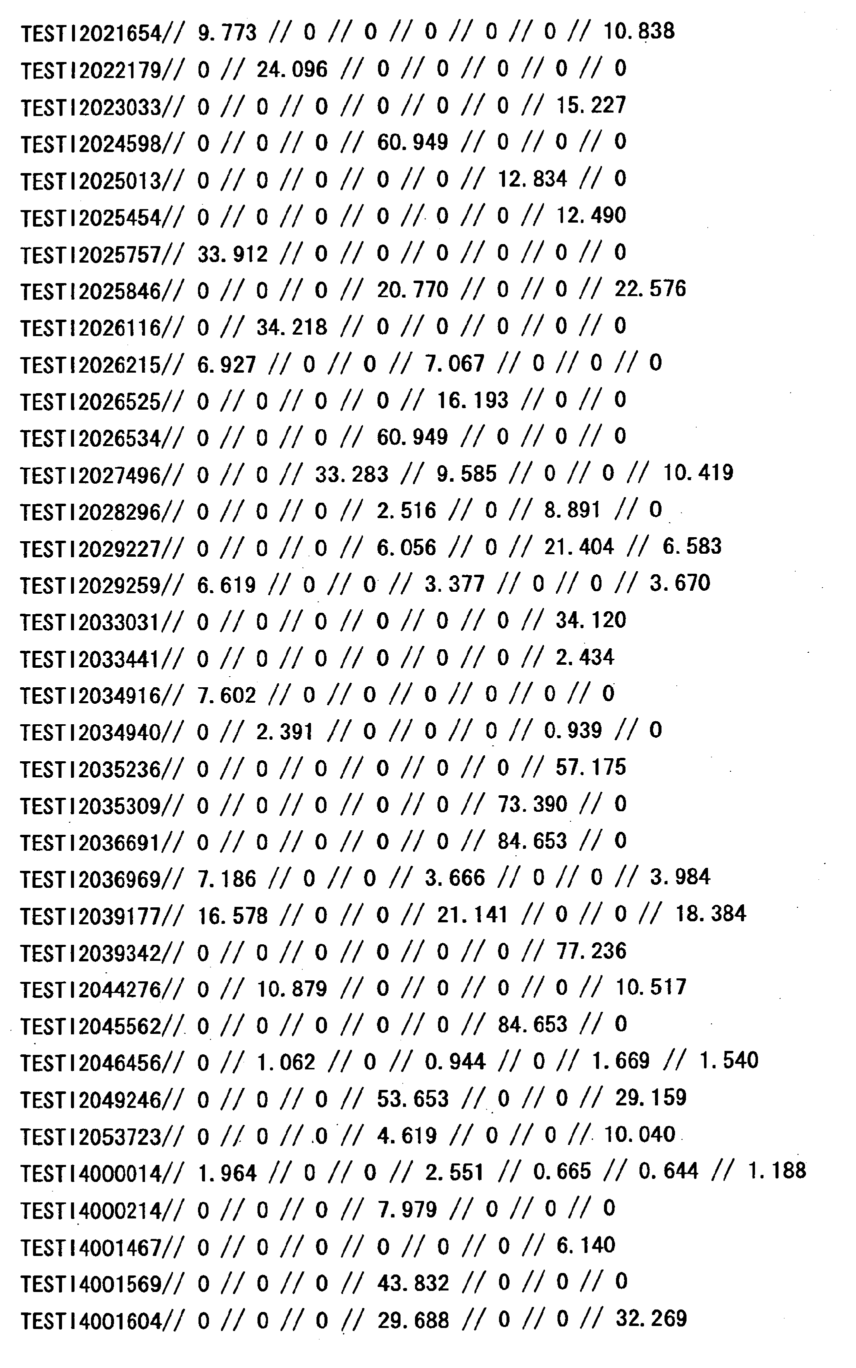

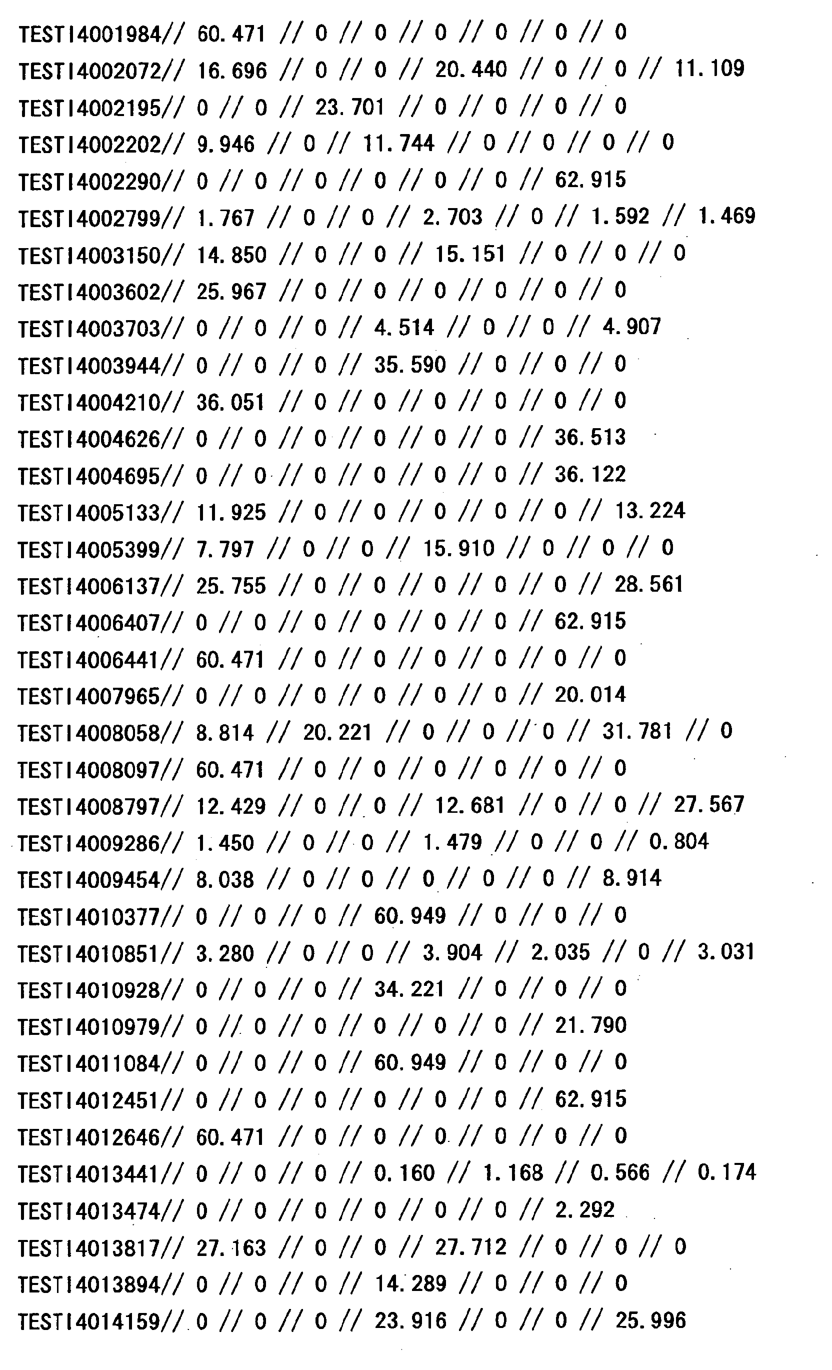

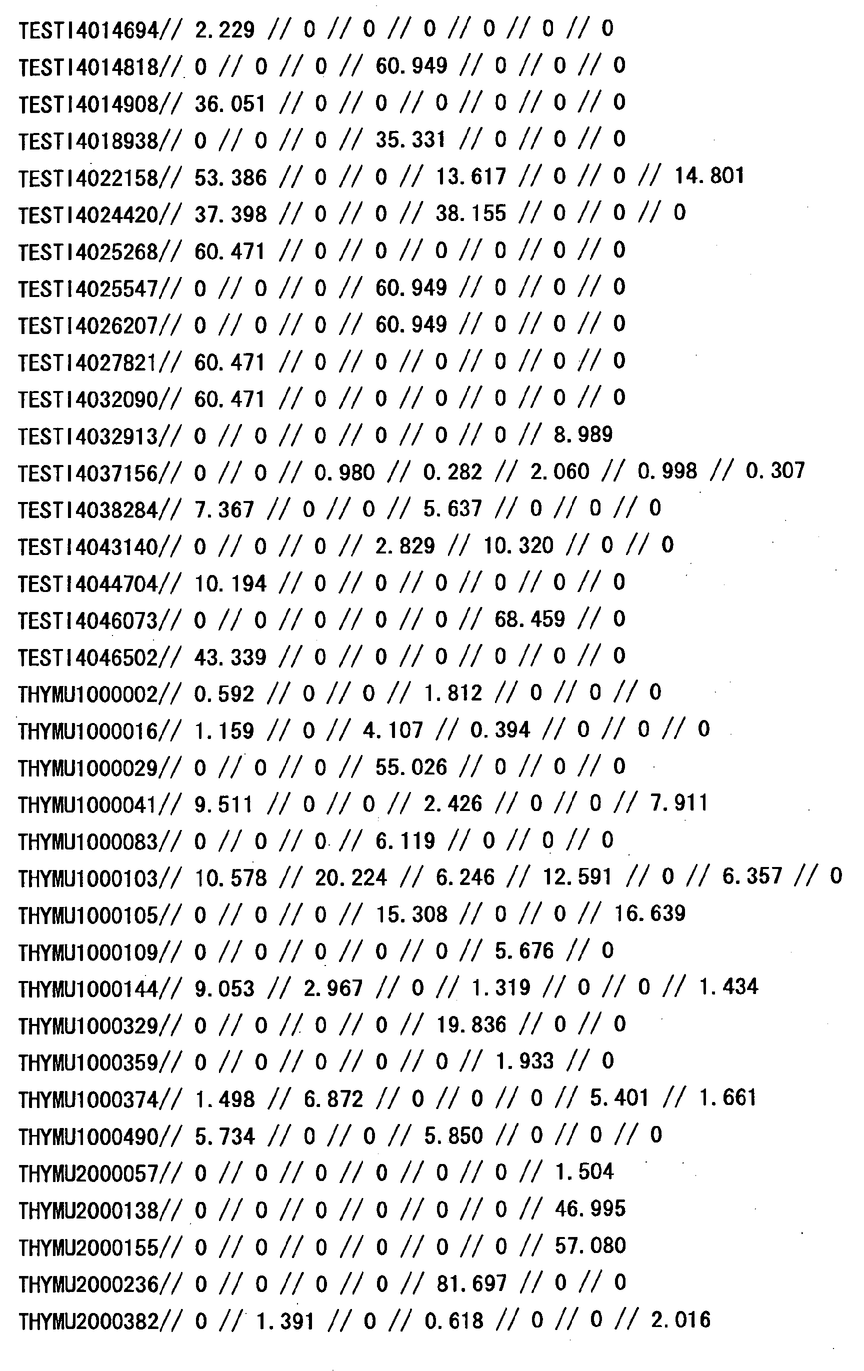

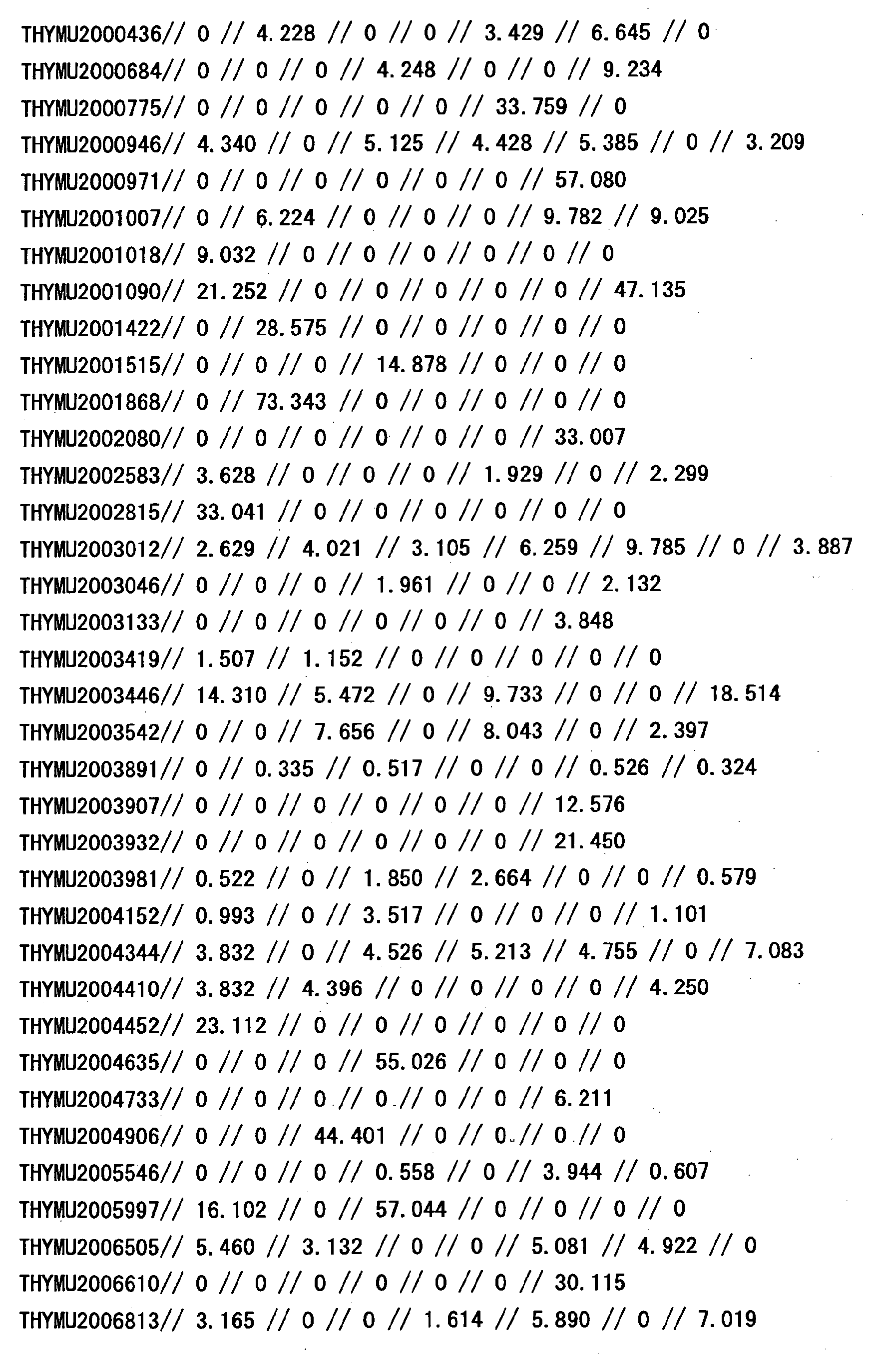

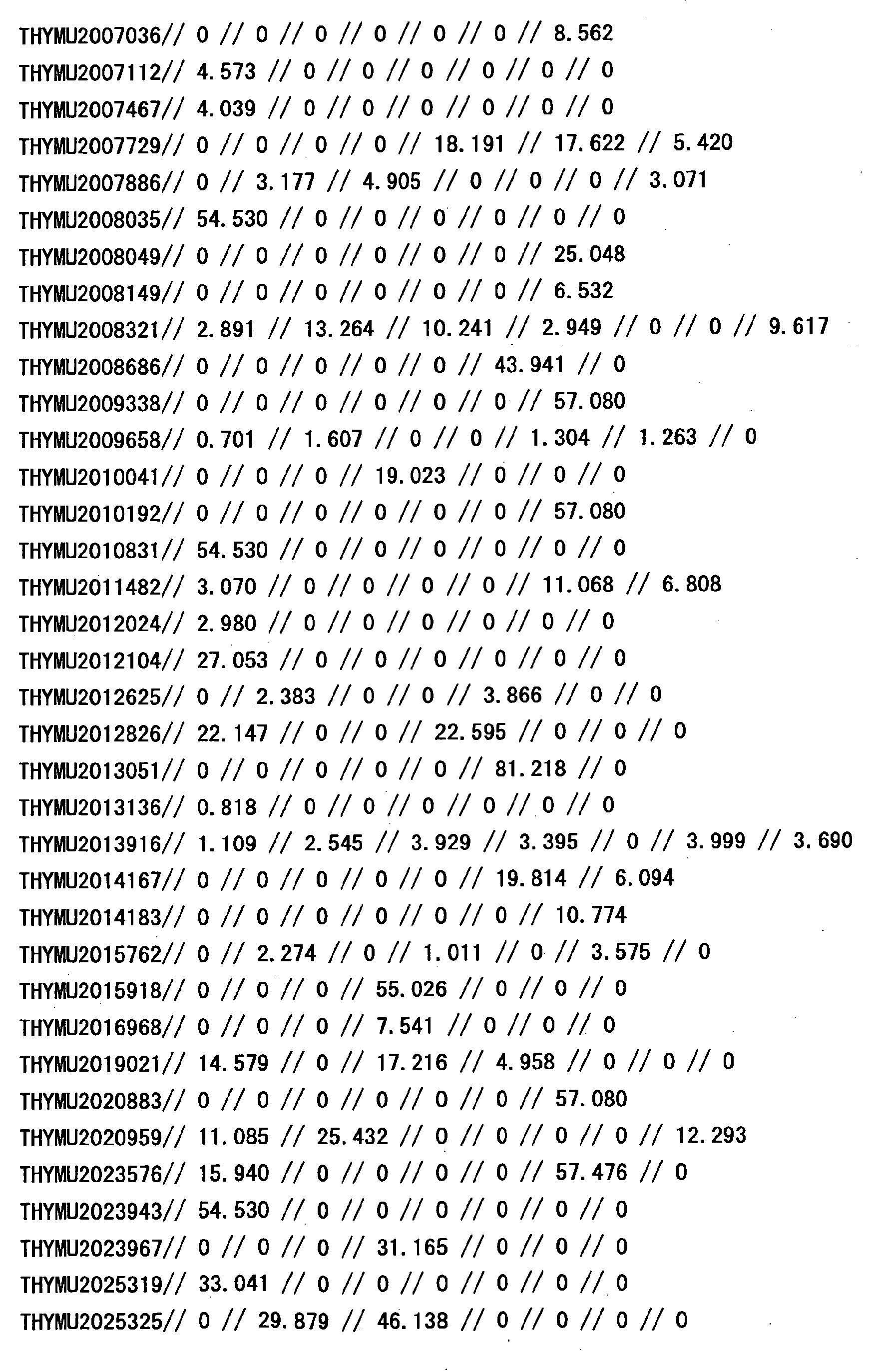

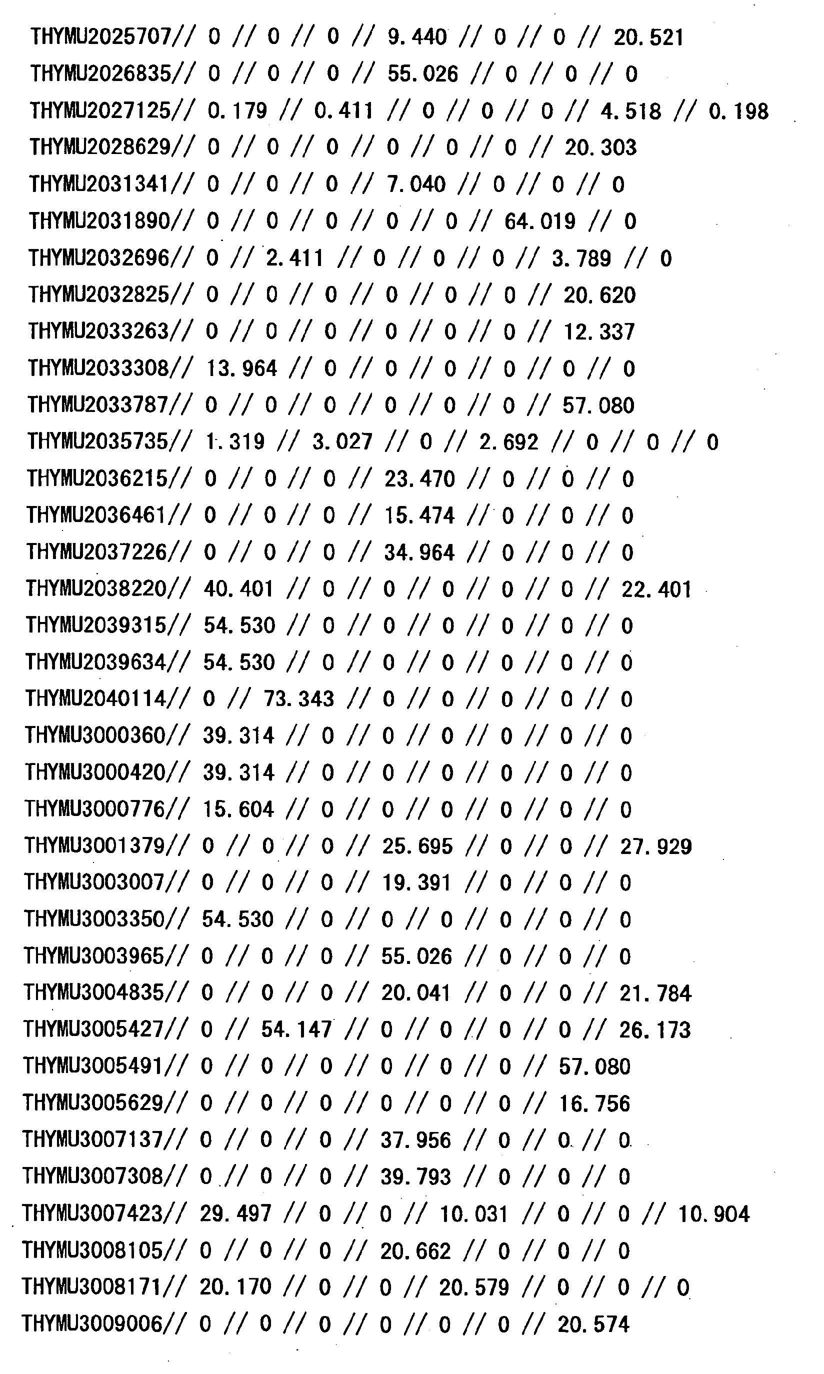

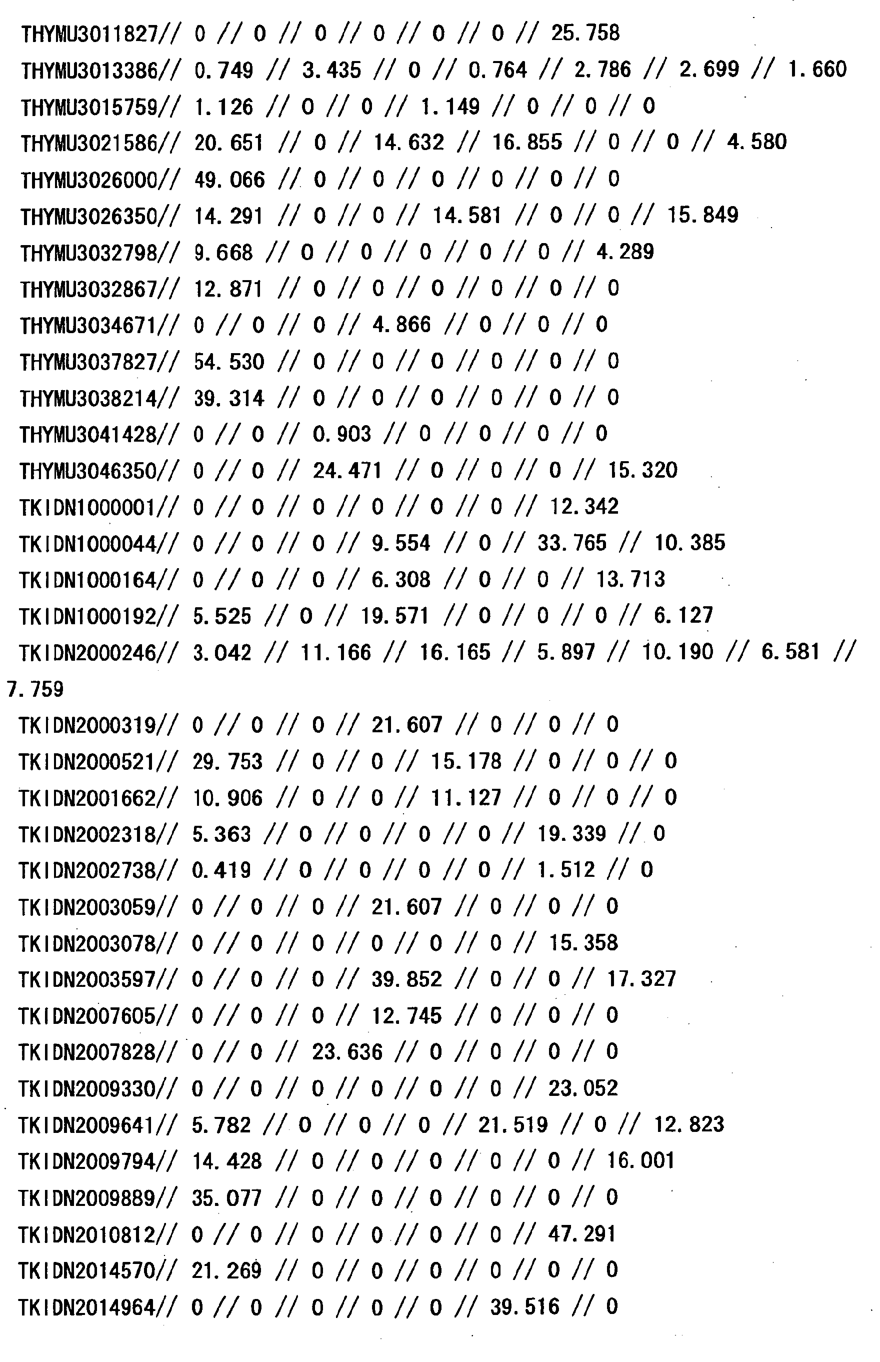

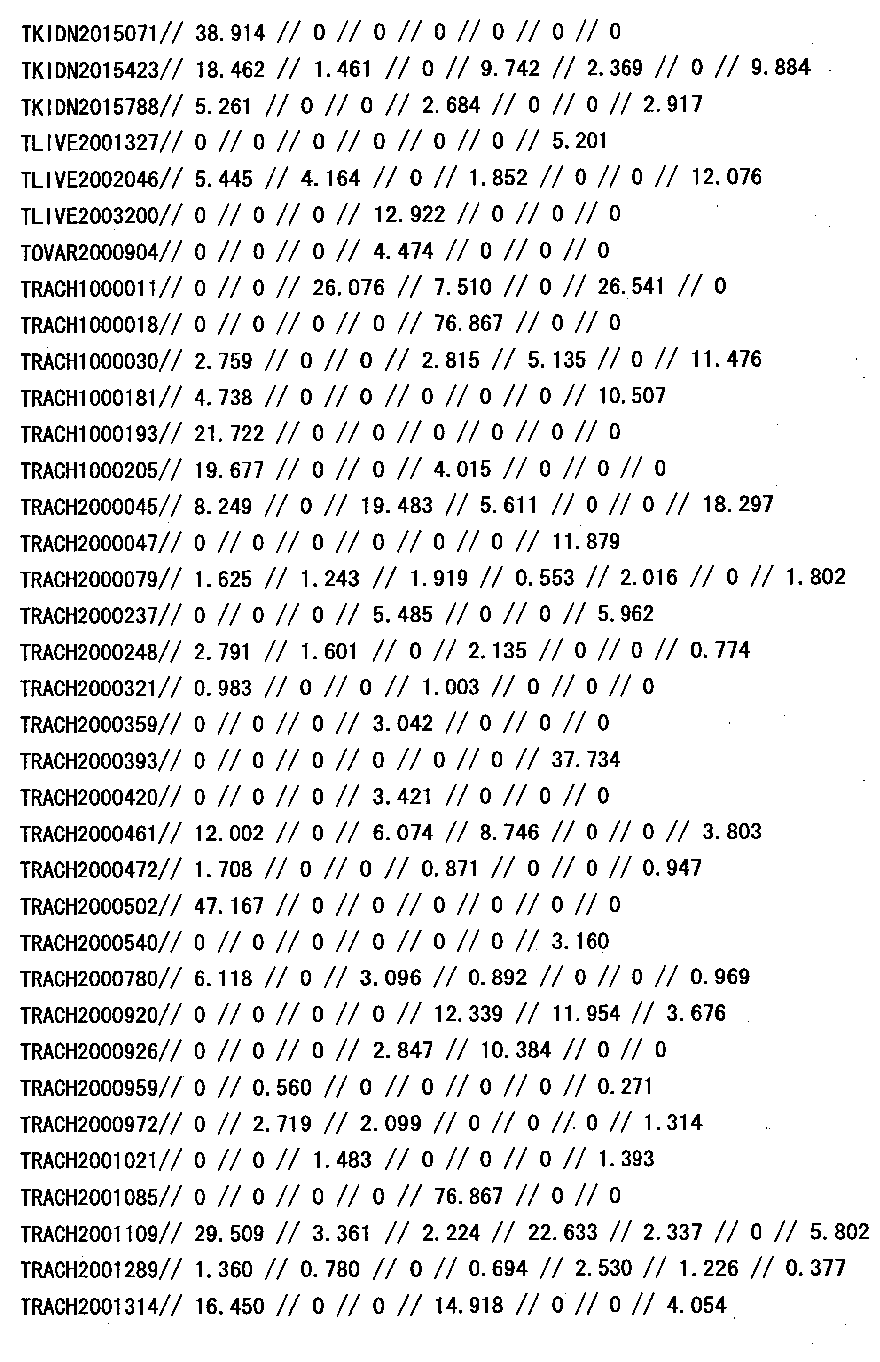

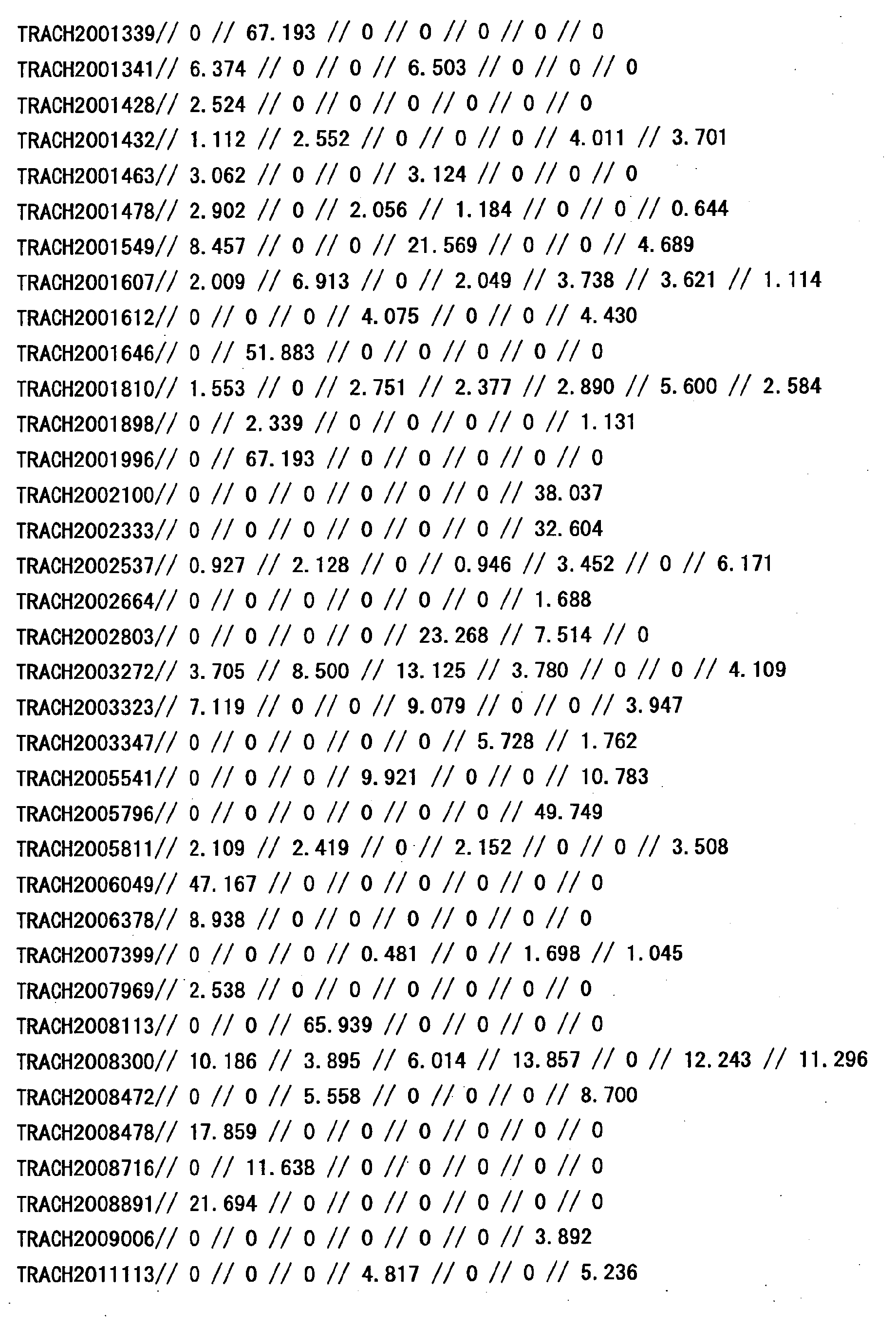

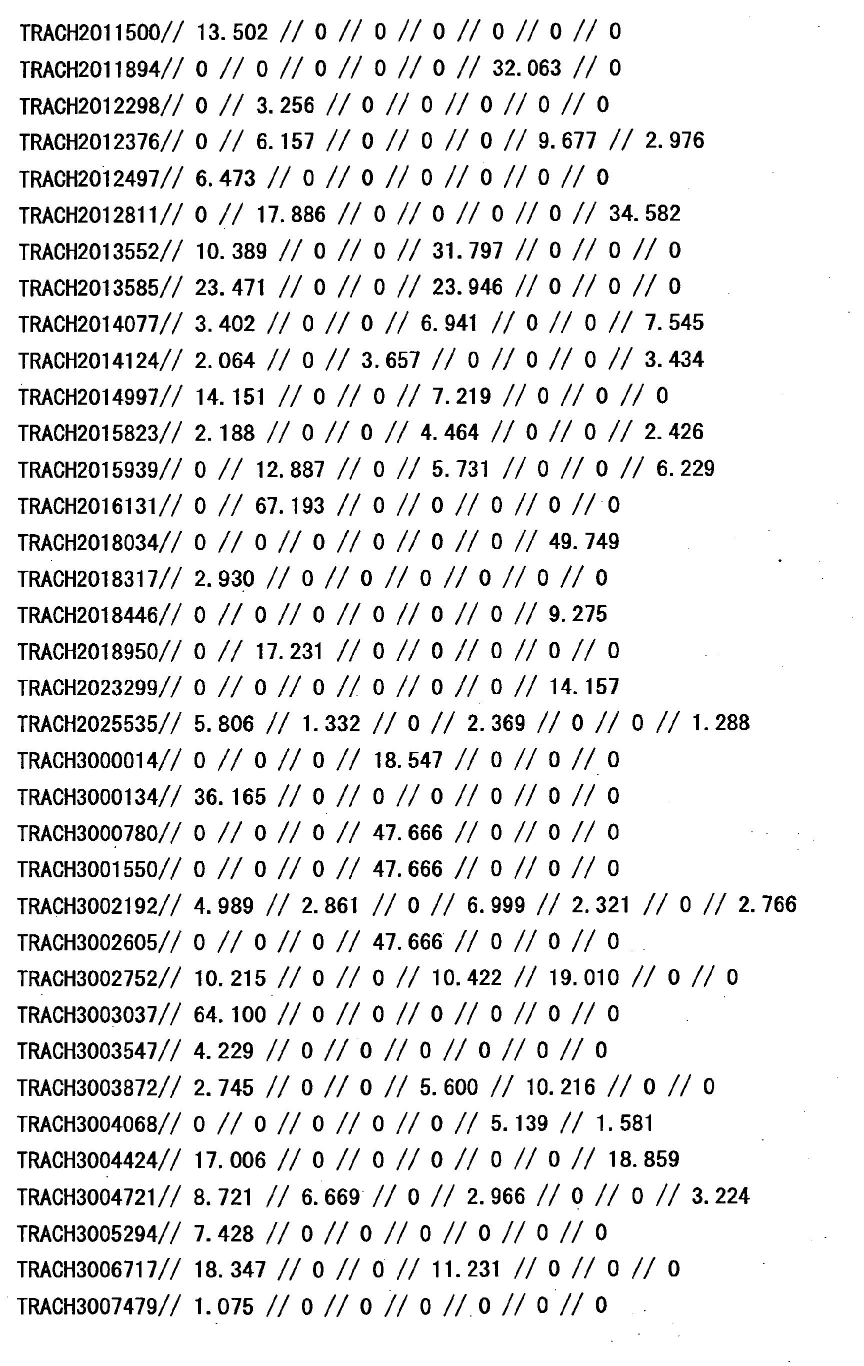

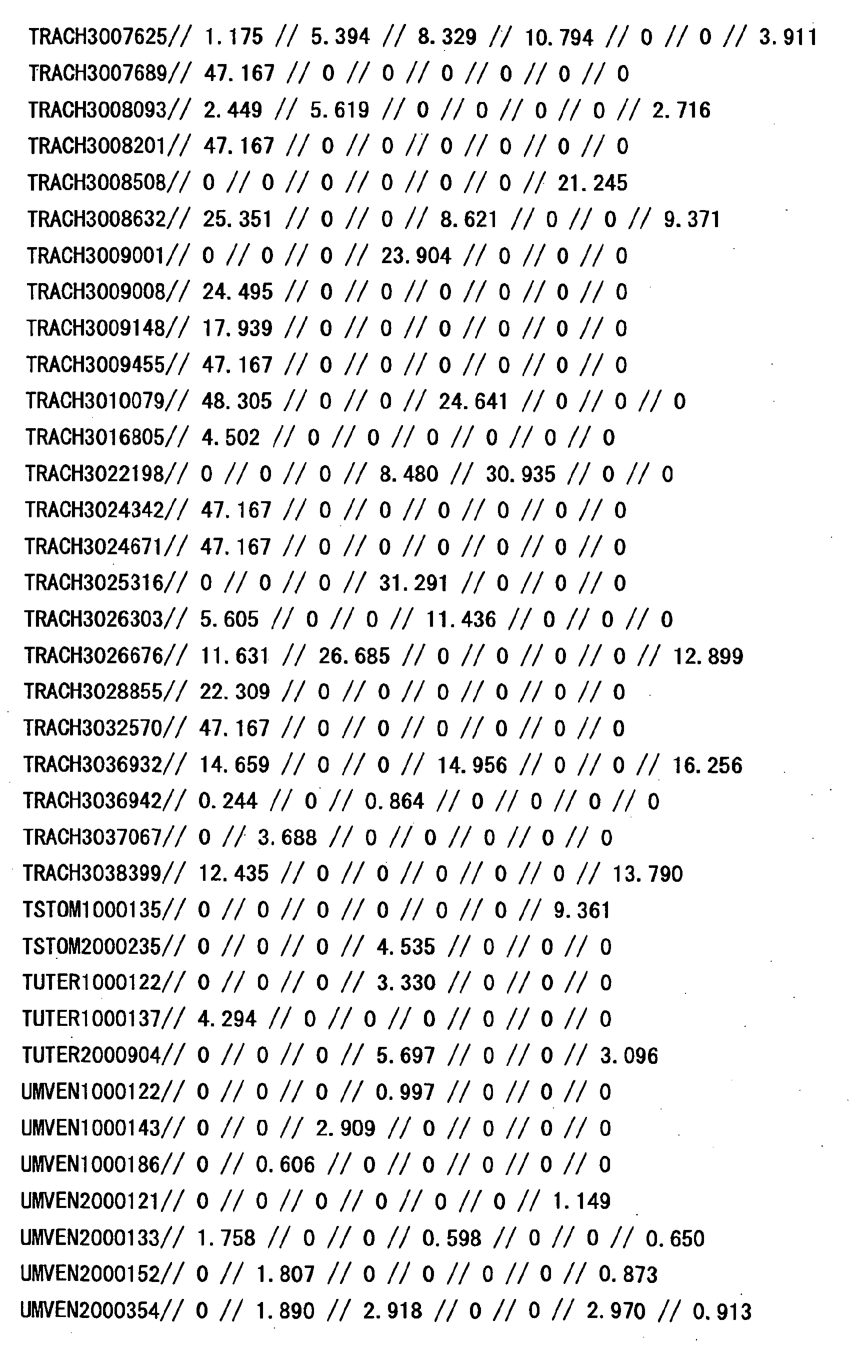

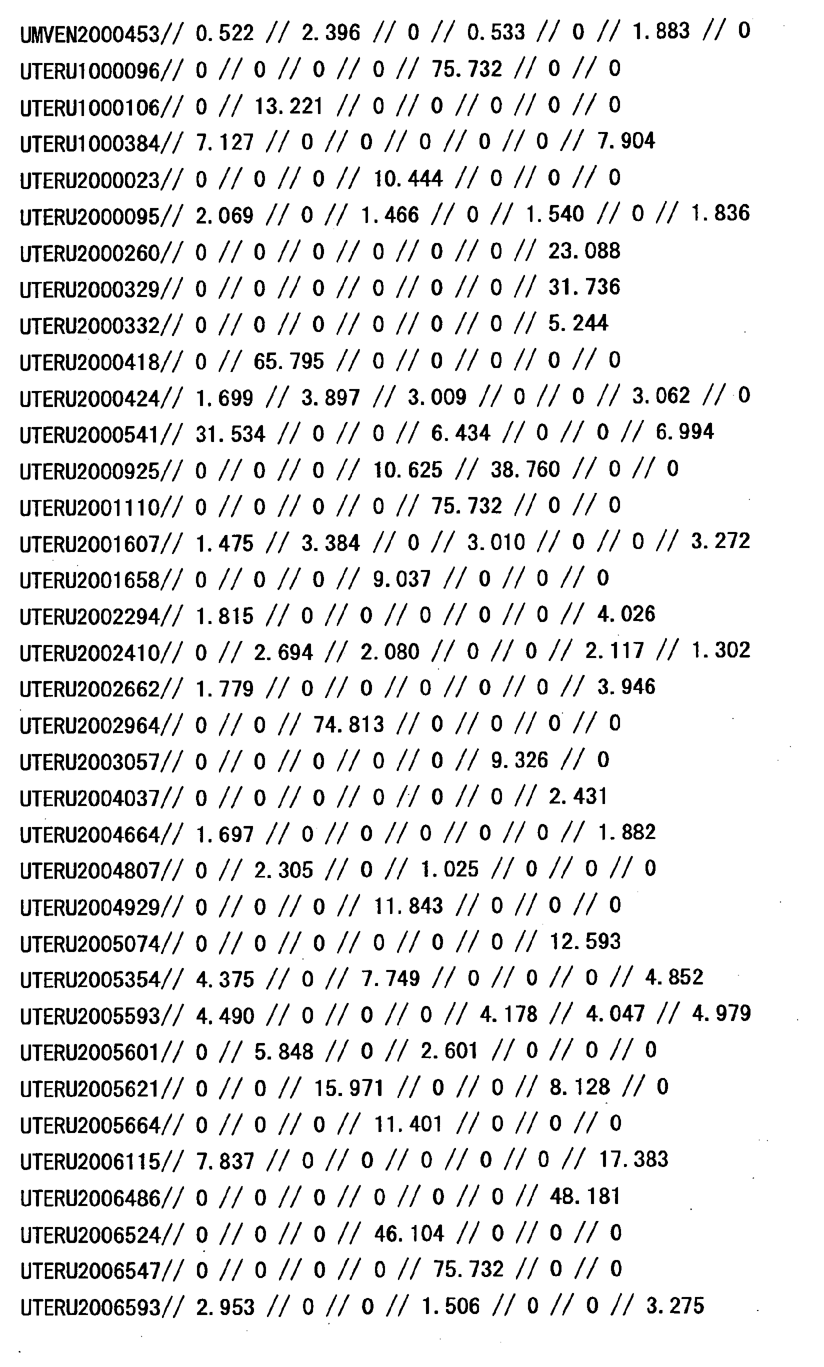

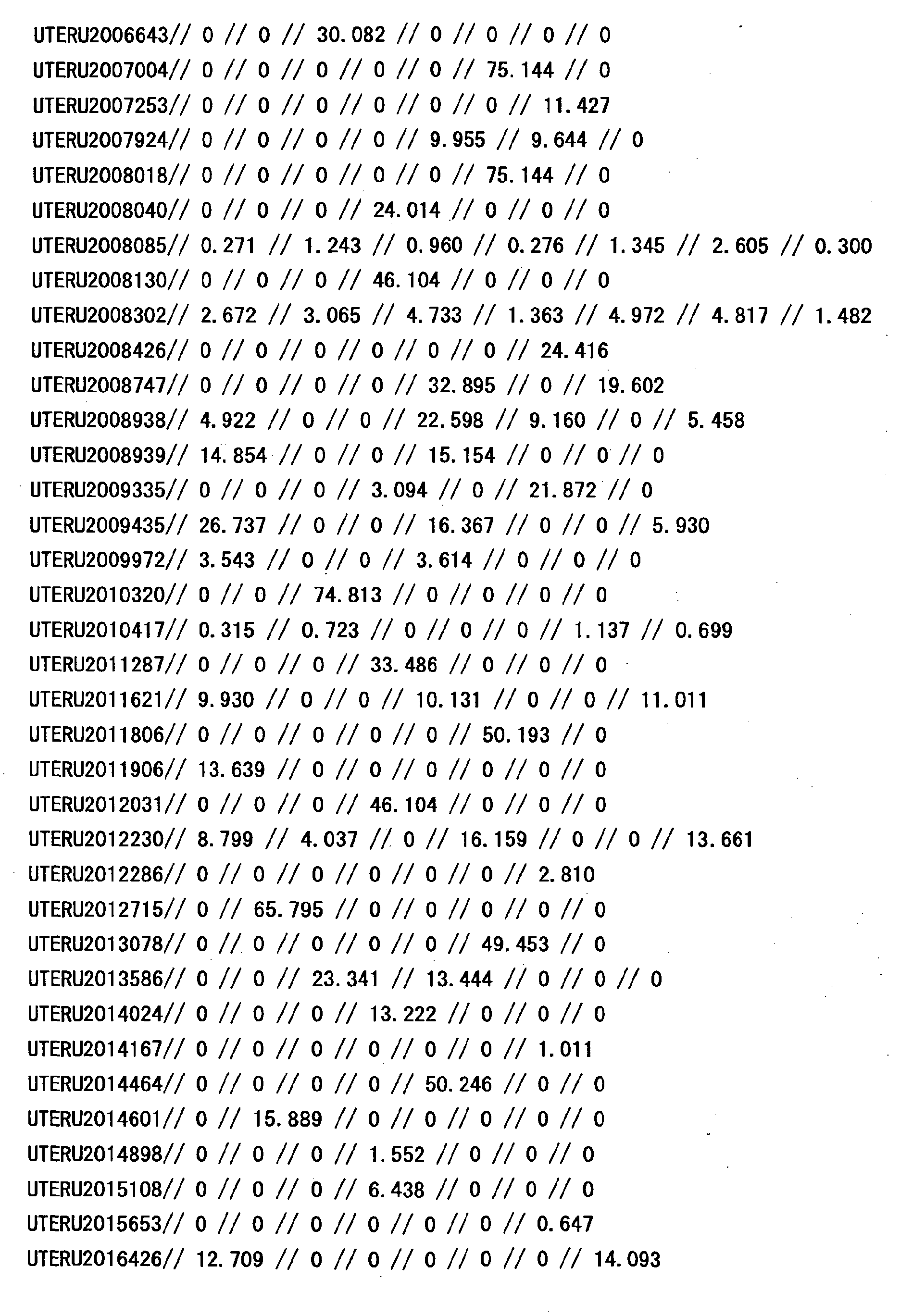

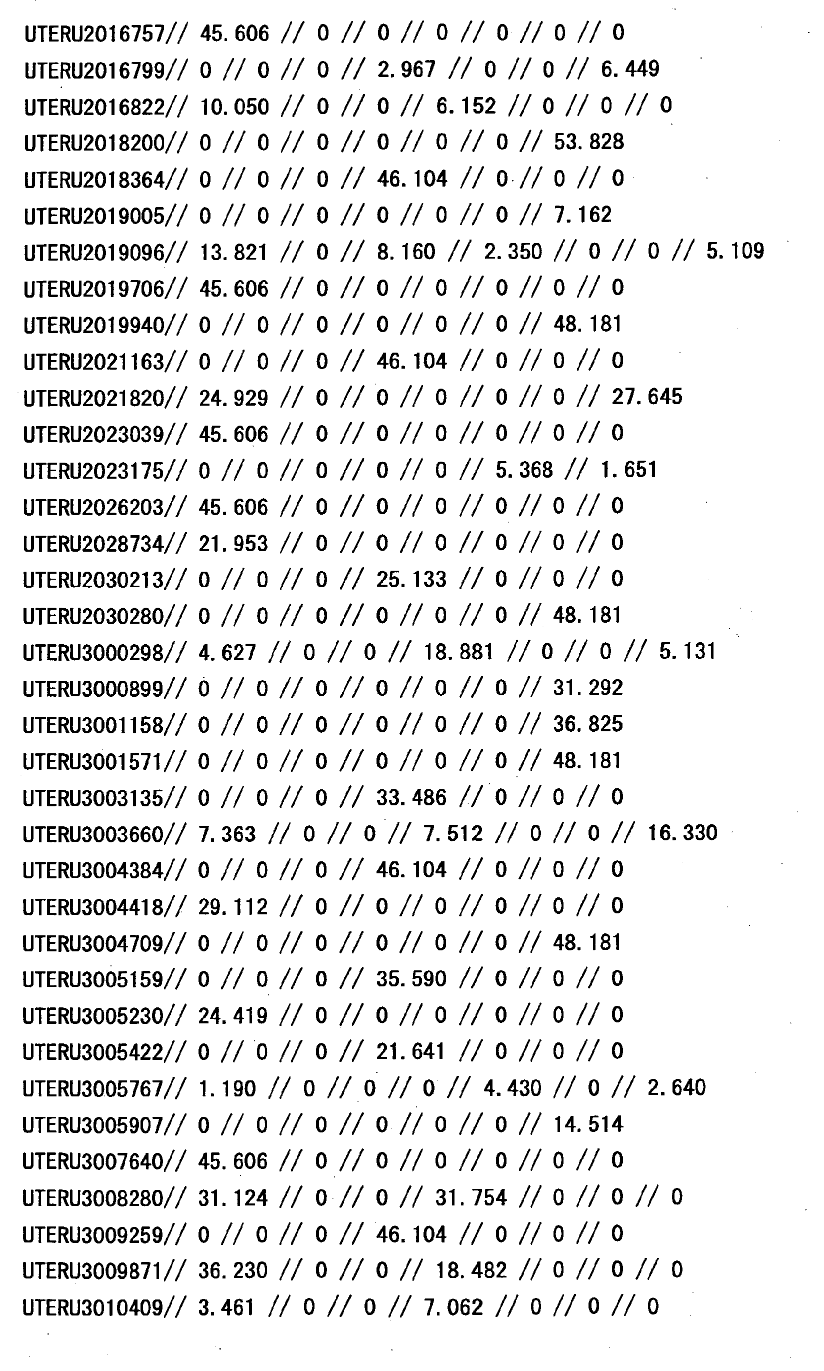

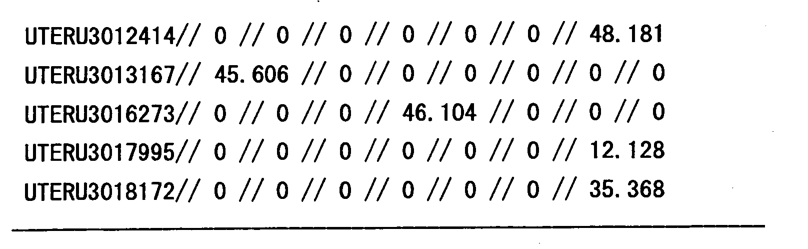

Genes whose expression levels were negligibly low in each of the above-described libraries are shown with their clone names below.
3NB691000018, 3NB691000085, 3NB691000113, 3NB691000129, 3NB692000012, 3NB692000029, 3NB692000102, 3NB692000154, 3NB692000276, 3NB692000305, 3NB692000429, 3NB692000484, 3NB692000545, 3NB692000973, 3NB692001002, 3NB692001034, 3NB692001040, 3NB692001123, 3NB692001267, 3NB692001339, 3NB692001349, 3NB692001366, 3NB692001408, 3NB692001459, 3NB692001471, 3NB692001496, 3NB692001501, 3NB692001507, 3NB692001511, 3NB692001519, 3NB692001520, 3NB692001538, 3NB692001548, 3NB692001637, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADRGL1000038, ADRGL1000067, ADRGL1000111, ADRGL1000147, ADRGL1000160, ADRGL1000165, ADRGL1000182, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002029, ADRGL2002260, ADRGL2002392, ADRGL2002753, ADRGL2002857, ADRGL2003017, ADRGL2003329, ADRGL2003552, ADRGL2003585, ADRGL2003638, ADRGL2003684, ADRGL2003773, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012038, ADRGL2012179, AHMSC1000138, ASTRO1000018, ASTRO1000029, ASTRO1000096, ASTRO1000165, ASTRO2000011, ASTRO2000095, ASTRO2000141, ASTRO2000191, ASTRO2000278, ASTRO2000403, ASTRO2000417, ASTRO2000533, ASTRO2000653, ASTRO2000725, ASTRO2000801, ASTRO2001001, ASTRO2001008, ASTRO2001088, ASTRO2001122, ASTRO2001227, ASTRO2001249, ASTRO2001480, ASTRO2001806, ASTRO2002024, ASTRO2002064, ASTRO2002459, ASTRO2002571, ASTRO2002632, ASTRO2002659, ASTRO2002693, ASTRO2002720, ASTRO2002743, ASTRO2002842, ASTRO2003212, ASTRO2003581, ASTRO2003632, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2005008, ASTRO2005081, ASTRO2005096, ASTRO2005242, ASTRO2005273, ASTRO2005343, ASTRO2005413, ASTRO2005553, ASTRO2005557, ASTRO2005575, ASTRO2005593, ASTRO2005687, ASTRO2005726, ASTRO2005896, ASTRO2005902, ASTRO2006356, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007956, ASTRO2007962, ASTRO2008239, ASTRO2008409, ASTRO2008425, ASTRO2008508, ASTRO2008777, ASTRO2008895, ASTRO2008960, ASTRO2009118, ASTRO2009177, ASTRO2009333, ASTRO2009458, ASTRO2009526, ASTRO2009879, ASTRO2010582, ASTRO2010615, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014211, ASTRO2014363, ASTRO2014498, ASTRO2014561, ASTRO2014923, ASTRO2014961, ASTRO2015162, ASTRO2015185, ASTRO2015529, ASTRO2015987, ASTRO2016044, ASTRO2016148, ASTRO2016681, ASTRO2016819, ASTRO2016847, ASTRO2016892, ASTRO2017348, ASTRO2017627, ASTRO2018373, ASTRO2019039, ASTRO2019171, ASTRO3000154, ASTRO3000172, ASTRO3000177, ASTRO3000301, ASTRO3000474, BEAST2000454, BGGI11000193, BGGI11000285, BGGI12000022, BGGI12000067, BGGI12000161, BGGI12000533, BGGI12000544, BGGI12000684, BGGI12000693, BGGI12001097, BGGI12001241, BGGI12001247, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001458, BLADE2001575, BLADE2001753, BLADE2001935, BLADE2001940, BLADE2001987, BLADE2002073, BLADE2002310, BLADE2002730, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006415, BLADE2006607, BLADE2006826, BLADE2006849, BLADE2006947, BLADE2007033, BLADE2007666, BLADE2007722, BLADE2007735, BLADE2007744, BLADE2007799, BLADE2007923, BLADE2007958, BLADE2008281, BLADE2008454, BLADE2008461, BLADE2008479, BLADE2008539, BLADE2008809, BLADE2008995, BLADE2009452, BLADE2009479, BLADE2009593, BNGH41000104, BNGH41000118, BNGH41000153, BNGH41000169, BNGH41000177, BNGH41000216, BNGH41000235, BNGH42000130, BNGH42000132, BNGH42000405, BNGH42000675, BNGH42000791, BNGH42000815, BNGH42000994, BNGH42001406, BNGH42001576, BNGH42001724, BNGH42002244, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798, BNGH42008199, BNGH42008510, BNGH42008649, BNGH42008832, BNGH42008850, BNGH42008943, BNGH42009021, BRACE1000047, BRACE1000084, BRACE1000087, BRACE1000092, BRACE1000093, BRACE1000114, BRACE1000124, BRACE1000132, BRACE1000166, BRACE1000239, BRACE1000451, BRACE1000475, BRACE1000572, BRACE2000061, BRACE2000077, BRACE2000078, BRACE2000100, BRACE2000148, BRACE2000183, BRACE2000229, BRACE2000232, BRACE2000256, BRACE2000266, BRACE2000267, BRACE2000351, BRACE2000368, BRACE2000392, BRACE2000441, BRACE2000487, BRACE2000489, BRACE2000505, BRACE2000526, BRACE2000531, BRACE2000545, BRACE2000553, BRACE2000577, BRACE2000640, BRACE2000650, BRACE2000677, BRACE2000718, BRACE2000743, BRACE2000753, BRACE2000815, BRACE2000885, BRACE2000905, BRACE2000936, BRACE2000947, BRACE2000965, BRACE2000969, BRACE2000988, BRACE2001017, BRACE2001094, BRACE2001107, BRACE2001117, BRACE2001151, BRACE2001191, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001508, BRACE2001543, BRACE2001564, BRACE2001588, BRACE2001607, BRACE2001626, BRACE2001677, BRACE2001705, BRACE2001733, BRACE2001737, BRACE2001742, BRACE2001777, BRACE2001779, BRACE2001855, BRACE2001859, BRACE2001865, BRACE2001872, BRACE2001881, BRACE2001898, BRACE2001923, BRACE2001942, BRACE2001954, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002394, BRACE2002409, BRACE2002431, BRACE2002441, BRACE2002444, BRACE2002458, BRACE2002467, BRACE2002468, BRACE2002589, BRACE2002590, BRACE2002613, BRACE2002614, BRACE2002617, BRACE2002623, BRACE2002635, BRACE2002649, BRACE2002661, BRACE2002676, BRACE2002685, BRACE2002695, BRACE2002707, BRACE2002736, BRACE2002752, BRACE2002755, BRACE2002762, BRACE2002772, BRACE2002792, BRACE2002796, BRACE2002807, BRACE2002834, BRACE2002849, BRACE2002857, BRACE2002861, BRACE2002884, BRACE2002948, BRACE2003110, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003540, BRACE2003594, BRACE2003614, BRACE2003628, BRACE2003639, BRACE2003713, BRACE2003766, BRACE2003802, BRACE2003825, BRACE2003847, BRACE2003848, BRACE2003873, BRACE2003885, BRACE2003887, BRACE2003928, BRACE2003954, BRACE2003987, BRACE2005034, BRACE2005047, BRACE2005063, BRACE2005090, BRACE2005118, BRACE2005124, BRACE2005138, BRACE2005160, BRACE2005169, BRACE2005189, BRACE2005196, BRACE2005198, BRACE2005216, BRACE2005243, BRACE2005298, BRACE2005328, BRACE2005329, BRACE2005337, BRACE2005348, BRACE2005363, BRACE2005433, BRACE2005450, BRACE2005453, BRACE2005460, BRACE2005489, BRACE2005518, BRACE2005556, BRACE2005562, BRACE2005624, BRACE2005661, BRACE2005667, BRACE2005731, BRACE2005742, BRACE2005762, BRACE2005773, BRACE2005787, BRACE2005795, BRACE2005800, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006102, BRACE2006105, BRACE2006122, BRACE2006137, BRACE2006143, BRACE2006162, BRACE2006174, BRACE2006240, BRACE2006245, BRACE2006249, BRACE2006274, BRACE2006298, BRACE2006319, BRACE2006378, BRACE2006393, BRACE2006413, BRACE2006417, BRACE2006453, BRACE2006459, BRACE2006488, BRACE2006534, BRACE2006540, BRACE2006547, BRACE2006564, BRACE2006587, BRACE2006598, BRACE2006685, BRACE2006703, BRACE2006783, BRACE2006833, BRACE2006900, BRACE2006909, BRACE2006911, BRACE2006924, BRACE2006935, BRACE2006944, BRACE2006951, BRACE2006986, BRACE2007068, BRACE2007087, BRACE2007138, BRACE2007174, BRACE2007197, BRACE2007203, BRACE2007232, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007527, BRACE2007538, BRACE2007563, BRACE2007621, BRACE2007640, BRACE2007646, BRACE2007682, BRACE2007685, BRACE2007727, BRACE2007749, BRACE2007760, BRACE2007761, BRACE2007764, BRACE2007769, BRACE2007784, BRACE2007786, BRACE2007799, BRACE2007801, BRACE2007836, BRACE2007868, BRACE2007888, BRACE2007902, BRACE2007920, BRACE2007944, BRACE2008008, BRACE2008015, BRACE2008021, BRACE2008102, BRACE2008114, BRACE2008144, BRACE2008172, BRACE2008218, BRACE2008276, BRACE2008295, BRACE2008308, BRACE2008336, BRACE2008361, BRACE2008401, BRACE2008410, BRACE2008420, BRACE2008443, BRACE2008473, BRACE2008480, BRACE2008481, BRACE2008488, BRACE2008495, BRACE2008500, BRACE2008594, BRACE2008604, BRACE2008615, BRACE2008622, BRACE2008629, BRACE2008655, BRACE2008656, BRACE2008710, BRACE2008754, BRACE2008797, BRACE2008847, BRACE2008861, BRACE2008867, BRACE2008881, BRACE2008883, BRACE2008917, BRACE2008919, BRACE2008948, BRACE2008960, BRACE2008999, BRACE2009014, BRACE2009016, BRACE2009031, BRACE2009044, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009421, BRACE2009428, BRACE2009434, BRACE2009437, BRACE2009533, BRACE2009558, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010535, BRACE2010550, BRACE2010598, BRACE2010632, BRACE2010642, BRACE2010684, BRACE2010813, BRACE2010828, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2014108, BRACE2014161, BRACE2014232, BRACE2014268, BRACE2014475, BRACE2014780, BRACE2014818, BRACE2014821, BRACE2014824, BRACE2014854, BRACE2015031, BRACE2015262, BRACE2015270, BRACE2015314, BRACE2015352, BRACE2015436, BRACE2015741, BRACE2015756, BRACE2015969, BRACE2015995, BRACE2016058, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017410, BRACE2017438, BRACE2017580, BRACE2017587, BRACE2017720, BRACE2017872, BRACE2017929, BRACE2017985, BRACE2017992, BRACE2018443, BRACE2018448, BRACE2018568, BRACE2018700, BRACE2018727, BRACE2018759, BRACE2018782, BRACE2019044, BRACE2019244, BRACE2019258, BRACE2019371, BRACE2019467, BRACE2019510, BRACE2019528, BRACE2019618, BRACE2019696, BRACE2019703, BRACE2019843, BRACE2019865, BRACE2019872, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254; BRACE2021264, BRACE2021344, BRACE2021433, BRACE2021478, BRACE2021541, BRACE2021670, BRACE2021695, BRACE2021721, BRACE2021936, BRACE2022030, BRACE2022158, BRACE2022196, BRACE2022270, BRACE2022328, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023268, BRACE2023284, BRACE2023404, BRACE2023451, BRACE2023540, BRACE2023633, BRACE2023727, BRACE2023744, BRACE2023800, BRACE2023801, BRACE2024074, BRACE2024222, BRACE2024610, BRACE2024627, BRACE2024826, BRACE2025018, BRACE2025316, BRACE2025333, BRACE2025452, BRACE2025492, BRACE2025710, BRACE2025796, BRACE2025992, BRACE2026054, BRACE2026131, BRACE2026141, BRACE2026293, BRACE2026294, BRACE2026404, BRACE2026675, BRACE2026725, BRACE2026836, BRACE2026920, BRACE2026971, BRACE2027221, BRACE2027258, BRACE2027312, BRACE2027382, BRACE2027389, BRACE2027408, BRACE2027770, BRACE2027879, BRACE2027970, BRACE2028171, BRACE2028410, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035441, BRACE2035485, BRACE2036005, BRACE2036096, BRACE2036743, BRACE2036830, BRACE2036834, BRACE2036900, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003413, BRACE3003595, BRACE3003698, BRACE3003866, BRACE3003920, BRACE3004058, BRACE3004113, BRACE3004150, BRACE3004205, BRACE3004358, BRACE3004435, BRACE3004477, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005870, BRACE3005903, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007322, BRACE3007472, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008720, BRACE3008772, BRACE3009044, BRACE3009075, BRACE3009090, BRACE3009127, BRACE3009197, BRACE3009265, BRACE3009297, BRACE3009377, BRACE3009392, BRACE3009539, BRACE3009574, BRACE3009701, BRACE3009708, BRACE3009993, BRACE3010076, BRACE3010079, BRACE3010086, BRACE3010221, BRACE3010283, BRACE3010315, BRACE3010416, BRACE3010435, BRACE3010702, BRACE3010862, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3019055, BRACE3019084, BRACE3019570, BRACE3019611, BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3026008, BRACE3026075, BRACE3026161, BRACE3026290, BRACE3026345, BRACE3026456, BRACE3026844, BRACE3026947, BRACE3026993, BRACE3027242, BRACE3027256, BRACE3027326, BRACE3027480, BRACE3027976, BRACE3028214, BRACE3028241, BRACE3028360, BRACE3028895, BRACE3028998, BRACE3029005, BRACE3029205, BRACE3029447, BRACE3030057, BRACE3030103, BRACE3030538, BRACE3030748, BRACE3031161, BRACE3031184, BRACE3031315, BRACE3031372, BRACE3031579, BRACE3031728, BRACE3031843, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039378, BRACE3039454, BRACE3040012, BRACE3040239, BRACE3040504, BRACE3040644, BRACE3040856, BRACE3041059, BRACE3041162, BRACE3041827, BRACE3042046, BRACE3042210, BRACE3042409, BRACE3042594, BRACE3043597, BRACE3044090, BRACE3044172, BRACE3044247, BRACE3044377, BRACE3044389, BRACE3044495, BRACE3045011, BRACE3045033, BRACE3045145, BRACE3045424, BRACE3045708, BRACE3046049, BRACE3046152, BRACE3046294, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2001038, BRALZ2001279, BRALZ2001350, BRALZ2001445, BRALZ2001559, BRALZ2001743, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008763, BRALZ2008869, BRALZ2008930, BRALZ2010186, BRALZ2010842, BRALZ2011337, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRAMY1000095, BRAMY1000098, BRAMY1000130, BRAMY1000157, BRAMY2000021, BRAMY2000076, BRAMY2000086, BRAMY2000151, BRAMY2000181, BRAMY2000196, BRAMY2000231, BRAMY2000277, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002493, BRAMY2002525, BRAMY2002584, BRAMY2002604, BRAMY2002638, BRAMY2002739, BRAMY2002853, BRAMY2002862, BRAMY2002956, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004058, BRAMY2004183, BRAMY2004335, BRAMY2004351, -BRAMY2004352, BRAMY2004492, BRAMY2004537, BRAMY2004542, BRAMY2004756, BRAMY2004771, BRAMY2005064, BRAMY2005094, BRAMY2005151, BRAMY2005182, BRAMY2005244, BRAMY2005391, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007249, BRAMY2007308, BRAMY2007411, BRAMY2007458, BRAMY2007486, BRAMY2007546, BRAMY2007610, BRAMY2007653, BRAMY2007753, BRAMY2007859, BRAMY2008333, BRAMY2008382, BRAMY2008583, BRAMY2008979, BRAMY2009034, BRAMY2009105, BRAMY2009123, BRAMY2009192, BRAMY2009344, BRAMY2009349, BRAMY2009394, BRAMY2009528, BRAMY2009693, BRAMY2010068, BRAMY2010135, BRAMY2010145, BRAMY2010171, BRAMY2010208, BRAMY2010239, BRAMY2010290, BRAMY2010366, BRAMY2010395, BRAMY2010441, BRAMY2010581, BRAMY2010798, BRAMY2010808, BRAMY2010919, BRAMY2011064, BRAMY2011178, BRAMY2011196, BRAMY2011216, BRAMY2011253, BRAMY2011480, BRAMY2011639, BRAMY2011679, BRAMY2011811, BRAMY2011846, BRAMY2011872, BRAMY2011961, BRAMY2012017, BRAMY2012091, BRAMY2012119, BRAMY2012162, BRAMY2012277, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2013200, BRAMY2013216, BRAMY2013405, BRAMY2013414, BRAMY2013572, BRAMY2013590, BRAMY2013621, BRAMY2013659, BRAMY2013722, BRAMY2013756, BRAMY2013944, BRAMY2013975, BRAMY2014019, BRAMY2014387, BRAMY2014469, BRAMY2014541, BRAMY2014813, BRAMY2015211, BRAMY2015311, BRAMY2015503, BRAMY2015516, BRAMY2015551, BRAMY2015559, BRAMY2015688, BRAMY2015705, BRAMY2015782, BRAMY2015855, BRAMY2015890, BRAMY2015925, BRAMY2016002, BRAMY2016020, BRAMY2016070, BRAMY2016327, BRAMY2016386, BRAMY2016706, BRAMY2016771, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018122, BRAMY2018126, BRAMY2018272, BRAMY2018273, BRAMY2018279, BRAMY2018308, BRAMY2018396, BRAMY2018467, BRAMY2019055, BRAMY2019277, BRAMY2019509, BRAMY2019643, BRAMY2019985, BRAMY2020050, BRAMY2020058, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021310, BRAMY2021388, BRAMY2021498, BRAMY2021523, BRAMY2021710, BRAMY2021732, BRAMY2021746, BRAMY2021962, BRAMY2021982, BRAMY2022052, BRAMY2022263, BRAMY2022301, BRAMY2022320, BRAMY2022383, BRAMY2022445, BRAMY2022454, BRAMY2022532, BRAMY2022796, BRAMY2022929, BRAMY2022984, BRAMY2023060, BRAMY2023110, BRAMY2023161, BRAMY2023172, BRAMY2023238, BRAMY2023327, BRAMY2023358, BRAMY2023482, BRAMY2023514, BRAMY2023719, BRAMY2023729, BRAMY2023786, BRAMY2023852, BRAMY2023863, BRAMY2023939, BRAMY2024073, BRAMY2024374, BRAMY2024449, BRAMY2024489, BRAMY2024497, BRAMY2024514, BRAMY2024530, BRAMY2024535, BRAMY2024545, BRAMY2024576, BRAMY2024715, BRAMY2024728, BRAMY2024849, BRAMY2025024, BRAMY2025032, BRAMY2025175, BRAMY2025218, BRAMY2025230, BRAMY2025277, BRAMY2025495, BRAMY2025812, BRAMY2025996, BRAMY2026009, BRAMY2026091, BRAMY2026300, BRAMY2026347, BRAMY2026395, BRAMY2026405, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027140, BRAMY2027242, BRAMY2027396, BRAMY2027451, BRAMY2027461, BRAMY2027467, BRAMY2027714, BRAMY2027752, BRAMY2027782, BRAMY2028072, BRAMY2028188, BRAMY2028282, BRAMY2028472, BRAMY2028491, BRAMY2028565, BRAMY2028593, BRAMY2028682, BRAMY2028783, BRAMY2028856, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031377, BRAMY2031442, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035801, BRAMY2035869, BRAMY2035923, BRAMY2036079, BRAMY2036254, BRAMY2036266, BRAMY2036387, BRAMY2036396, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037971, BRAMY2038100, BRAMY2038128, BRAMY2038317, BRAMY2038475, BRAMY2038484, BRAMY2038832, BRAMY2038846, BRAMY2038887, BRAMY2038904, BRAMY2039287, BRAMY2039300, BRAMY2039314, BRAMY2039341, BRAMY2039695, BRAMY2039872, BRAMY2040051, BRAMY2040159, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002508, BRAMY3002620, BRAMY3002805, BRAMY3002886, BRAMY3003026, BRAMY3003109, BRAMY3003205, BRAMY3003310, BRAMY3003566, BRAMY3003723, BRAMY3004364, BRAMY3004672, BRAMY3004900, BRAMY3004919, BRAMY3005656, BRAMY3005932, BRAMY3006032, BRAMY3006297, BRAMY3007206, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009904, BRAMY3010321, BRAMY3010411, BRAMY3010603, BRAMY4000277, BRAMY4000962, BRAMY4001173, BRAMY4001200, BRAMY4001234, BRAMY4001449, BRAMY4001652, BRAMY4001913, BRAMY4002575, BRAMY4002628, BRASW1000053, CD34C2000051, CD34C2000152, CD34C2000175, CD34C2000443, CD34C3000314, CD34C3000494, CERVX1000042, CERVX2000812, CERVX2000968, CERVX2002006, CERVX2002013, CERVX2002430, CERVX2002770, CHONS1000058, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000093, CTONG1000097, CTONG1000113, CTONG1000137, CTONG1000165, CTONG1000175, CTONG1000181, CTONG1000220, CTONG1000277, CTONG1000283, CTONG1000288, CTONG1000302, CTONG1000341, CTONG1000476, CTONG1000488, CTONG1000508, CTONG1000540, CTONG2000034, CTONG2000201, CTONG2000218, CTONG2000279, CTONG2000303, CTONG2000330, CTONG2000395, CTONG2000465, CTONG2000480, CTONG2000561, CTONG2000589, CTONG2000766, CTONG2000771, CTONG2000819, CTONG2000846, CTONG2000855, CTONG2000977, CTONG2001139, CTONG2001366, CTONG2001413, CTONG2001428, CTONG2001513, CTONG2001524, CTONG2001598, CTONG2001681, CTONG2001724, CTONG2001749, CTONG2001820, CTONG2001858, CTONG2001877, CTONG2001932, CTONG2001955, CTONG2002066, CTONG2002143, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002903, CTONG2002965, CTONG2002999, CTONG2003028, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004397, CTONG2004454, CTONG2004550, CTONG2004669, CTONG2004716, CTONG2004912, CTONG2004941, CTONG2004948, CTONG2005049, CTONG2005110, CTONG2005265, CTONG2005290, CTONG2005374, CTONG2005399, CTONG2005468, CTONG2005567, CTONG2005585, CTONG2005615, CTONG2005635, CTONG2005775, CTONG2005890, CTONG2005904, CTONG2005913, CTONG2006129, CTONG2006223, CTONG2006273, CTONG2006310, CTONG2006515, CTONG2006524, CTONG2006568, CTONG2006611, CTONG2006620, CTONG2006666, CTONG2006691, CTONG2006798, CTONG2006836, CTONG2006932, CTONG2006942, CTONG2006964, CTONG2007007, CTONG2007009, CTONG2007091, CTONG2007175, CTONG2007259, CTONG2007272, CTONG2007297, CTONG2007441, CTONG2007474, CTONG2007623, CTONG2007898, CTONG2007959, CTONG2007970, CTONG2008184, CTONG2008262, CTONG2008269, CTONG2008343, CTONG2008402, CTONG2008466, CTONG2008562, CTONG2008577, CTONG2008595, CTONG2008621, CTONG2008720, CTONG2008773, CTONG2008792, CTONG2008989, CTONG2009132, CTONG2009156, CTONG2009181, CTONG2009257, CTONG2009258, CTONG2009268, CTONG2009270, CTONG2009395, CTONG2009440, CTONG2009527, CTONG2009531, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010803, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077, CTONG2012101, CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012758, CTONG2012843, CTONG2012901, CTONG2013128, CTONG2013149, CTONG2013156, CTONG2013178, CTONG2013222, CTONG2013284, CTONG2013339, CTONG2013352, CTONG2013393, CTONG2013511, CTONG2013630, CTONG2013803, CTONG2013985, CTONG2013986, CTONG2014032, CTONG2014058, CTONG2014191, CTONG2014206, CTONG2014234, CTONG2014264, CTONG2014280, CTONG2014491, CTONG2014630, CTONG2014635, CTONG2014646, CTONG2014705, CTONG2014946, CTONG2014954, CTONG2014959, CTONG2014966, CTONG2014995, CTONG2015316, CTONG2015358, CTONG2015434, CTONG2015540, CTONG2015596, CTONG2015633, CTONG2015678, CTONG2015717, CTONG2015804, CTONG2015866, CTONG2015914, CTONG2015953, CTONG2016185, CTONG2016217, CTONG2016355, CTONG2016505, CTONG2016559, CTONG2016658, CTONG2016775, CTONG2016797, CTONG2016824, CTONG2016846, CTONG2016904, CTONG2016942, CTONG2016953, CTONG2017021, CTONG2017292, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018343, CTONG2018379, CTONG2018483, CTONG2018629, CTONG2018632, CTONG2018652, CTONG2018653, CTONG2018655, CTONG2018735, CTONG2018808, CTONG2018843, CTONG2018900, CTONG2018976, CTONG2019144, CTONG2019232, CTONG2019248, CTONG2019400, CTONG2019652, CTONG2019704, CTONG2019822, CTONG2019833, CTONG2020031, CTONG2020108, CTONG2020127, CTONG2020171, CTONG2020411, CTONG2020522, CTONG2020638, CTONG2020806, CTONG2020974, CTONG2021024, CTONG2021132, CTONG2021168, CTONG2021289, CTONG2022153, CTONG2022601, CTONG2022835, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028518, CTONG2028687, CTONG2028758, CTONG3000686, CTONG3000707, CTONG3000896, CTONG3001420, CTONG3001501, CTONG3001560, CTONG3001605, CTONG3002020, CTONG3002127, CTONG3002370, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009385, D3OST1000066, D3OST1000109, D3OST1000238, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000507, D9OST2000278, D9OST2000440, D9OST2000869, D9OST2001319, D9OST2001433, D9OST2002397, D9OST2002673, D9OST2003108, D9OST2003137, D9OST2003397, D9OST2003580, D9OST2003594, D9OST2003791, D9OST2003989, D9OST2004018, D9OST2004073, D9OST2004417, DFNES1000003, DFNES1000107, DFNES1000185, DFNES2000143, DFNES2000146, DFNES2000212, DFNES2000426, DFNES2000432, DFNES2000471, DFNES2000913, DFNES2001108, DFNES2001177, DFNES2001404, DFNES2001564, DFNES2001565, DFNES2001647, DFNES2001800, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005766, DFNES2005779, DFNES2005875, DFNES2006346, DFNES2006944, DFNES2007299, DFNES2007332, DFNES2007641, DFNES2007757, DFNES2008280, DFNES2008881, DFNES2010502, DFNES2011192, DFNES2011221, DFNES2011239, ERLTF1000021, ERLTF2000324, ERLTF2000465, FCBBF1000023, FCBBF1000038, FCBBF1000053, FCBBF1000069, FCBBF1000115, FCBBF1000117, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000280, FCBBF1000297, FCBBF1000305, FCBBF1000314, FCBBF1000322, FCBBF1000349, FCBBF1000374, FCBBF1000377, FCBBF1000395, FCBBF1000397, FCBBF1000425, FCBBF1000437, FCBBF1000506, FCBBF1000546, FCBBF1000550, FCBBF1000581, FCBBF1000582, FCBBF1000598, FCBBF1000604, FCBBF1000618, FCBBF1000627, FCBBF1000675, FCBBF1000686, FCBBF1000687, FCBBF1000691, FCBBF1000732, FCBBF2000094, FCBBF2000105, FCBBF2000123, FCBBF2000195, FCBBF2000214, FCBBF2000232, FCBBF2000259, FCBBF2000276, FCBBF2000291, FCBBF2000379, FCBBF2000473, FCBBF2000502, FCBBF2000576, FCBBF2000677, FCBBF2000678, FCBBF2000782, FCBBF2000808, FCBBF2000815, FCBBF2000825, FCBBF2000951, FCBBF2001088, FCBBF2001116, FCBBF2001188, FCBBF2001238, FCBBF2001243, FCBBF2001299, FCBBF2001309, FCBBF2001427, FCBBF2001529, FCBBF2001672, FCBBF2001675, FCBBF2001718, FCBBF2001720, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003297, FCBBF2003395, FCBBF2003528, FCBBF2003549, FCBBF2003636, FCBBF2003823, FCBBF2003895, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005966, FCBBF2006131, FCBBF2006380, FCBBF2006418, FCBBF2006452, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000120, FCBBF3000175, FCBBF3000227, FCBBF3000233, FCBBF3000269, FCBBF3000361, FCBBF3000367, FCBBF3000462, FCBBF3000473, FCBBF3000501, FCBBF3000518, FCBBF3000550, FCBBF3000847, FCBBF3001018, FCBBF3001020, FCBBF3001235, FCBBF3001281, FCBBF3001302, FCBBF3001594, FCBBF3001818, FCBBF3001855, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782, FCBBF3002925, FCBBF3003216, FCBBF3003305, FCBBF3003373, FCBBF3003435, FCBBF3003475, FCBBF3003481, FCBBF3003902, FCBBF3004021, FCBBF3004041, FCBBF3004261, FCBBF3004390, FCBBF3004409, FCBBF3004502, FCBBF3004842, FCBBF3004847, FCBBF3005160, FCBBF3005218, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007540, FCBBF3007597, FCBBF3007604, FCBBF3007829, FCBBF3007855, FCBBF3008073, FCBBF3008100, FCBBF3008251, FCBBF3008311, FCBBF3008362, FCBBF3008382, FCBBF3008475, FCBBF3008556, FCBBF3008689, FCBBF3008748, FCBBF3008870, FCBBF3008938, FCBBF3008944, FCBBF3009256, FCBBF3009317, FCBBF3009428, FCBBF3009464, FCBBF3009703, FCBBF3009717, FCBBF3009888, FCBBF3009949, FCBBF3010008, FCBBF3010124, FCBBF3010130, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013589, FCBBF3013623, FCBBF3013846, FCBBF3014229, FCBBF3014355, FCBBF3014567, FCBBF3014770, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016644, FCBBF3016987, FCBBF3017024, FCBBF3017059, FCBBF3017071, FCBBF3017123, FCBBF3017233, FCBBF3017288, FCBBF3017535, FCBBF3017681, FCBBF3017729, FCBBF3017873, FCBBF3017974, FCBBF3018453, FCBBF3018591, FCBBF3018753, FCBBF3018826, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021191, FCBBF3021221, FCBBF3021501, FCBBF3021506, FCBBF3021524, FCBBF3021940, FCBBF3021985, FCBBF3022005, FCBBF3022291, FCBBF3022311, FCBBF3022504, FCBBF3022765, FCBBF3022894, FCBBF3023061, FCBBF3023078, FCBBF3023372, FCBBF3023443, FCBBF3023599, FCBBF3023704, FCBBF3023887, FCBBF3023895, FCBBF3024096, FCBBF3024225, FCBBF3024364, FCBBF3024793, FCBBF3024911, FCBBF3025098, FCBBF3025252, FCBBF3025280, FCBBF3025674, FCBBF3025730, FCBBF3025905, FCBBF3026021, FCBBF3026048, FCBBF3026251, FCBBF3026678, FCBBF3027104, FCBBF3027199, FCBBF3027328, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028671, FCBBF3028794, FCBBF4000017, FCBBF4000061, FCBBF4000076, FCBBF4000173, FCBBF4000192, FCBBF4000193, FCBBF4000282, FCBBF4000399, FCBBF4000415, FCBBF4000500, FCBBF4000529, FCBBF4000548, FCBBF5000041, FCBBF5000353, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA2000021, FEBRA2000035, FEBRA2000126, FEBRA2000220, FEBRA2000228, FEBRA2000282, FEBRA2000286, FEBRA2000311, FEBRA2000321, FEBRA2000377, FEBRA2000378, FEBRA2000391, FEBRA2000394, FEBRA2000397, FEBRA2000399, FEBRA2000404, FEBRA2000409, FEBRA2000415, FEBRA2000436, FEBRA2000438, FEBRA2000454, FEBRA2000468, FEBRA2000491, FEBRA2000504, FEBRA2000510, FEBRA2000532, FEBRA2000575, FEBRA2000656, FEBRA2000659, FEBRA2000690, FEBRA2000727, FEBRA2000734, FEBRA2000782, FEBRA2000790, FEBRA2000803, FEBRA2000805, FEBRA2000809, FEBRA2000856, FEBRA2000862, FEBRA2000881, FEBRA2000909, FEBRA2000917, FEBRA2000928, FEBRA2000936, FEBRA2000937, FEBRA2000959, FEBRA2000972, FEBRA2001012, FEBRA2001020, FEBRA2001093, FEBRA2001124, FEBRA2001133, FEBRA2001161, FEBRA2001175, FEBRA2001217, FEBRA2001267, FEBRA2001351, FEBRA2001383, FEBRA2001438, FEBRA2001487, FEBRA2001523, FEBRA2001561, FEBRA2001584, FEBRA2001590, FEBRA2001610, FEBRA2001620, FEBRA2001659, FEBRA2001705, FEBRA2001752, FEBRA2001772, FEBRA2001790, FEBRA2001828, FEBRA2001914, FEBRA2001989, FEBRA2002009, FEBRA2002086, FEBRA2002109, FEBRA2002191, FEBRA2002260, FEBRA2002399, FEBRA2002410, FEBRA2002429, FEBRA2002508, FEBRA2002525, FEBRA2002527, FEBRA2002552, FEBRA2002574, FEBRA2002601, FEBRA2002628, FEBRA2002662, FEBRA2002682, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003520, FEBRA2003524, FEBRA2003541, FEBRA2003675, FEBRA2003711, FEBRA2003822, FEBRA2003897, FEBRA2003907, FEBRA2004023, FEBRA2004026, FEBRA2004029, FEBRA2004053, FEBRA2004056, FEBRA2004110, FEBRA2004191, FEBRA2004202, FEBRA2004237, FEBRA2004268, FEBRA2004329, FEBRA2004331, FEBRA2004412, FEBRA2004443, FEBRA2004538, FEBRA2004620, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005377, FEBRA2005380, FEBRA2005458, FEBRA2005701, FEBRA2005726, FEBRA2005734, FEBRA2005778, FEBRA2005787, FEBRA2005998, FEBRA2006061, FEBRA2006112, FEBRA2006150, FEBRA2006165, FEBRA2006315, FEBRA2006346, FEBRA2006354, FEBRA2006379, FEBRA2006396, FEBRA2006409, FEBRA2006418, FEBRA2006427, FEBRA2006476, FEBRA2006491, FEBRA2006664, FEBRA2006667, FEBRA2006726, FEBRA2006736, FEBRA2006793, FEBRA2006873, FEBRA2006942, FEBRA2006977, FEBRA2007017, FEBRA2007200, FEBRA2007247, FEBRA2007280, FEBRA2007414, FEBRA2007436, FEBRA2007449, FEBRA2007458, FEBRA2007566, FEBRA2007620, FEBRA2007708, FEBRA2007714, FEBRA2007793, FEBRA2007931, FEBRA2008009, FEBRA2008134, FEBRA2008135, FEBRA2008159, FEBRA2008165, FEBRA2008172, FEBRA2008265, FEBRA2008282, FEBRA2008311, FEBRA2008341, FEBRA2008459, FEBRA2008468, FEBRA2008475, FEBRA2008481, FEBRA2008542, FEBRA2008660, FEBRA2008681, FEBRA2008692, FEBRA2008741, FEBRA2008836, FEBRA2008859, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009240, FEBRA2009265, FEBRA2009276, FEBRA2009327, FEBRA2009385, FEBRA2009416, FEBRA2009437, FEBRA2009478, FEBRA2009507, FEBRA2009541, FEBRA2009567, FEBRA2009731, FEBRA2009754, FEBRA2009780, FEBRA2010141, FEBRA2010299, FEBRA2010484, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2014010, FEBRA2014122, FEBRA2014198, FEBRA2014213, FEBRA2014443, FEBRA2014578, FEBRA2014939, FEBRA2015076, FEBRA2015175, FEBRA2015292, FEBRA2015588, FEBRA2016112, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019298, FEBRA2019389, FEBRA2019457, FEBRA2019519, FEBRA2019582, FEBRA2019637, FEBRA2019663, FEBRA2019690, FEBRA2020406, FEBRA2020566, FEBRA2020582, FEBRA2020668, FEBRA2020801, FEBRA2021032, FEBRA2021134, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022148, FEBRA2022204, FEBRA2022255, FEBRA2022322, FEBRA2022601, FEBRA2022729, FEBRA2022787, FEBRA2022911, FEBRA2022956, FEBRA2022963, FEBRA2022972, FEBRA2023285, FEBRA2023351, FEBRA2023399, FEBRA2023498, FEBRA2023550, FEBRA2023721, FEBRA2023989, FEBRA2023990, FEBRA2024150, FEBRA2024343, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000156, HCASM2000202, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003099, HCASM2003212, HCASM2003237, HCASM2003357, HCASM2003415, HCASM2006131, HCASM2006338, HCASM2006359, HCASM2006632, HCASM2007317, HCASM2007737, HCASM2007773, HCASM2008154, HCASM2008536, HCASM2009405, HCASM2009424, HCASM2009463, HCASM2009588, HCHON1000142, HCHON1000176, HCHON2000038, HCHON2000100, HCHON2000156, HCHON2000199, HCHON2000265, HCHON2000271, HCHON2000315, HCHON2000323, HCHON2000344, HCHON2000373, HCHON2000395, HCHON2000417, HCHON2000418, HCHON2000503, HCHON2000508, HCHON2000660, HCHON2000673, HCHON2000676, HCHON2000699, HCHON2000705, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001110, HCHON2001116, HCHON2001161, HCHON2001214, HCHON2001233, HCHON2001269, HCHON2001450, HCHON2001453, HCHON2001497, HCHON2001505, HCHON2001519, HCHON2001523, HCHON2001546, HCHON2001548, HCHON2001577, HCHON2001598, HCHON2001604, HCHON2001607, HCHON2001646, HCHON2001712, HCHON2001768, HCHON2002123, HCHON2002194, HCHON2002241, HCHON2002354, HCHON2002496, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000088, HEART1000099, HEART1000102, HEART1000132, HEART1000139, HEART1000151, HEART1000173, HEART2000024, HEART2000035, HEART2000099, HEART2000298, HEART2000306, HEART2000309, HEART2000411, HEART2000418, HEART2000448, HEART2000492, HEART2000506, HEART2000520, HEART2000568, HEART2000611, HEART2001680, HEART2001756, HEART2002129, HEART2002184, HEART2002220, HEART2002432, HEART2002531, HEART2002598, HEART2002717, HEART2003027, HEART2003432, HEART2003440, HEART2003781, HEART2003836, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007443, HEART2007767, HEART2008108, HEART2008111, HEART2008257, HEART2008994, HEART2009599, HEART2009680, HEART2010391, HEART2010492, HEART2010495, HELAC2000158, HELAC2000301, HHDPC1000001, HHDPC2000070, HHDPC2000102, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006460, HHDPC2006862, HHDPC2006927, HHDPC2007302, HHDPC2008123, HHDPC2008185, HHDPC2008279, HHDPC2008297, HHDPC2008414, HHDPC2008474, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000064, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000340, HLUNG2000362, HLUNG2000363, HLUNG2000431, HLUNG2000480, HLUNG2000489, HLUNG2000549, HLUNG2000557, HLUNG2000570, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001507, HLUNG2001633, HLUNG2001677, HLUNG2001712, HLUNG2001814, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002942, HLUNG2002949, HLUNG2002958, HLUNG2002982, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004154, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA2000044, HSYRA2000135, HSYRA2000221, HSYRA2000237, HSYRA2000253, HSYRA2000371, HSYRA2000424, HSYRA2000507, HSYRA2000510, HSYRA2000605, HSYRA2000706, HSYRA2000760, HSYRA2000792, HSYRA2000832, HSYRA2000927, HSYRA2001003, HSYRA2001103, HSYRA2001142, HSYRA2001153, HSYRA2001225, HSYRA2001255, HSYRA2001332, HSYRA2001420, HSYRA2001552, HSYRA2001567, HSYRA2001580, HSYRA2001631, HSYRA2002103, HSYRA2002590, HSYRA2002741, HSYRA2003168, HSYRA2004550, HSYRA2004858, HSYRA2005456, HSYRA2005496, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000230, IMR322000010, IMR322000107, IMR322000164, IMR322000178, IMR322000374, IMR322000379, IMR322000383, IMR322000418, IMR322000656, IMR322000672, IMR322000742, IMR322000791, IMR322000811, IMR322000859, IMR322000863, IMR322000917, IMR322000919, IMR322000953, IMR322000973, IMR322000984, IMR322001018, IMR322001049, IMR322001167, IMR322001245, IMR322001318, IMR322001322, IMR322001438, IMR322001452, IMR322001491, IMR322001534, IMR322001665, IMR322001670, IMR322001724, IMR322002110, IMR322002470, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322015523, IMR322016146, IMR322017049, IMR322018117, IMR322018192, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000328, KIDNE2000335, KIDNE2000349, KIDNE2000375, KIDNE2000394, KIDNE2000493, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2001140, KIDNE2001269, KIDNE2001361, KIDNE2001373, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002198, KIDNE2002262, KIDNE2002265, KIDNE2002438, KIDNE2002483, KIDNE2002768, KIDNE2002795, KIDNE2002839, KIDNE2002845, KIDNE2002872, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005603, KIDNE2005629, KIDNE2005781, KIDNE2005798, KIDNE2005854, KIDNE2005908, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006652, KIDNE2006687, KIDNE2006733, KIDNE2006775; KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007944, KIDNE2008022, KIDNE2008072, KIDNE2008116, KIDNE2008117, KIDNE2008218, KIDNE2008315, KIDNE2008378, KIDNE2008446, KIDNE2008473, KIDNE2008480, KIDNE2008649, KIDNE2008697, KIDNE2008788, KIDNE2008795, KIDNE2008824, KIDNE2008832, KIDNE2008869, KIDNE2008975, KIDNE2009019, KIDNE2009109, KIDNE2009180, KIDNE2009191, KIDNE2009332, KIDNE2009426, KIDNE2009467, KIDNE2009490, KIDNE2009553, KIDNE2009628, KIDNE2009647, KIDNE2009676, KIDNE2009723, KIDNE2010007, KIDNE2010052, KIDNE2010084, KIDNE2010137, KIDNE2010151, KIDNE2010264, KIDNE2010271, KIDNE2010419, KIDNE2010574, KIDNE2010674, KIDNE2010739, KIDNE2010762, KIDNE2010863, KIDNE2010973, KIDNE2010989, KIDNE2011200, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000050, LIVER1000055, LIVER1000058, LIVER1000063, LIVER1000067, LIVER1000073, LIVER1000082, LIVER1000099, LIVER1000111, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005198, LIVER2005218, LIVER2005376, LIVER2005385, LIVER2005512, LIVER2005520, LIVER2005527, LIVER2005544, LIVER2005625, LIVER2005728, LIVER2005981, LIVER2006006, LIVER2006251, LIVER2006469, LIVER2006644, LIVER2006764, LIVER2007415, LIVER2007548, LIVER2007568, LIVER2007721, LIVER2007783, LIVER2008053, LIVER2008473, LIVER2008580, LIVER2008706, LIVER2008945, LIVER2008998, LIVER2009110, LIVER2009118, LIVER2009554, LYMPB1000141, LYMPB1000158, LYMPB2000083, MAMGL1000096, MAMGL1000132, MAMGL1000151, MAMGL1000173, MAMGL1000178, MAMGL1000182, MAMGL1000184, MESAN1000035, MESAN1000121, MESAN1000147, MESAN1000180, MESAN2000023, MESAN2000026, MESAN2000067, MESAN2000167, MESAN2000291, MESAN2000337, MESAN2000434, MESAN2000457, MESAN2000462, MESAN2000620, MESAN2000711, MESAN2000815, MESAN2000894, MESAN2000909, MESAN2001450, MESAN2001770, MESAN2001979, MESAN2001980, MESAN2002113, MESAN2002147, MESAN2002424, MESAN2002687, MESAN2002709, MESAN2002790, MESAN2002978, MESAN2003035, MESAN2003037, MESAN2003039, MESAN2003058, MESAN2003101, MESAN2003322, MESAN2003490, MESAN2003529, MESAN2003662, MESAN2003851, MESAN2004138, MESAN2004288, MESAN2004575, MESAN2005303, MESAN2005371, MESAN2005633, MESAN2005724, MESAN2005808, MESAN2005811, MESAN2006022, MESAN2006328, MESAN2006580, MESAN2006599, MESAN2006971, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009105, MESAN2009311, MESAN2009418, MESAN2009522, MESAN2010031, MESAN2010114, MESAN2010312, MESAN2010321, MESAN2010681, MESAN2011545, MESAN2011597, MESAN2011627, MESAN2011977, MESAN2012586, MESAN2012708, MESAN2012735, MESAN2013019, MESAN2013022, MESAN2013183, MESAN2013211, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016552, MESAN2016965, MESAN2017152, MESAN2017373, MESAN2017417, MESAN2018209, MESAN2018576, MESAN2018670, MESAN2018699, MESTC1000147, MESTC2000150, MESTC2000153, MESTC2000170, N1ESE2000698, NB9N41000121, NB9N41000135, NB9N41000146, NB9N42000104, NB9N42000122, NB9N42000196, NB9N42000314, NB9N42000495, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2002565, NHNPC2002749, NOVAR1000091, NOVAR1000102, NOVAR2000136, NOVAR2000352, NOVAR2000412, NOVAR2000710, NOVAR2000783, NOVAR2000962, NOVAR2001108, NOVAR2001620, NOVAR2001876, NOVAR2002039, NT2NE1000014, NT2NE1000023, NT2NE1000024, NT2NE1000059, NT2NE1000063, NT2NE1000083, NT2NE1000120, NT2NE1000122, NT2NE1000175, NT2NE1000185, NT2NE2000038, NT2NE2000054, NT2NE2000057, NT2NE2000064, NT2NE2000109, NT2NE2000137, NT2NE2000156, NT2NE2000222, NT2NE2000259, NT2NE2000284, NT2NE2000299, NT2NE2000353, NT2NE2000356, NT2NE2000369, NT2NE2000374, NT2NE2000390, NT2NE2000455, NT2NE2000470, NT2NE2000488, NT2NE2000517, NT2NE2000536, NT2NE2000546, NT2NE2000550, NT2NE2000586, NT2NE2000612, NT2NE2000658, NT2NE2000706, NT2NE2000802, NT2NE2000808, NT2NE2000864, NT2NE2000913, NT2NE2000950, NT2NE2000951, NT2NE2001005, NT2NE2001021, NT2NE2001142, NT2NE2001181, NT2NE2001188, NT2NE2001191, NT2NE2001247, NT2NE2001252, NT2NE2001324, NT2NE2001326, NT2NE2001372, NT2NE2001428, NT2NE2001432, NT2NE2001524, NT2NE2001530, NT2NE2001617, NT2NE2001623, NT2NE2001626, NT2NE2001634, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005441, NT2NE2005517, NT2NE2005537, NT2NE2005555, NT2NE2005676, NT2NE2005720, NT2NE2005784, NT2NE2005804, NT2NE2005821, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006458, NT2NE2006531, NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009608, NT2NE2009691, NT2NE2010105, NT2NE2010150, NT2NE2010400, NT2NE2010632, NT2NE2010633, NT2NE2010654, NT2NE2010781, NT2NE2010854, NT2NE2011033, NT2NE2011036, NT2NE2011107, NT2NE2011154, NT2NE2011411, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2016041, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018739, NT2NE2018916, NT2NE2019056, NT2NE2019076, NT2NE2019099, NT2RI1000027, NT2RI1000035, NT2RI1000164, NT2RI1000172, NT2RI2000004, NT2RI2000007, NT2RI2000117, NT2RI2000128, NT2RI2000133, NT2RI2000173, NT2RI2000187, NT2RI2000270, NT2RI2000276, NT2RI2000282, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000822, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178, NT2RI2001235, NT2RI2001244, NT2RI2001307, NT2RI2001342, NT2RI2001385, NT2RI2001409, NT2RI2001410, NT2RI2001425, NT2RI2001449, NT2RI2001474, NT2RI2001519, NT2RI2001595, NT2RI2001624, NT2RI2001769, NT2RI2001782, NT2RI2001836, NT2RI2001866, NT2RI2002022, NT2RI2002041, NT2RI2002117, NT2RI2002120, NT2RI2002153, NT2RI2002179, NT2RI2002252, NT2RI2002260, NT2RI2002270, NT2RI2002334, NT2RI2002447, NT2RI2002481, NT2RI2002507, NT2RI2002517, NT2RI2002554, NT2RI2002564, NT2RI2002585, NT2RI2002729, NT2RI2002802, NT2RI2002852, NT2RI2002865, NT2RI2002923, NT2RI2002970, NT2RI2003019, NT2RI2003067, NT2RI2003184, NT2RI2003360, NT2RI2003383, NT2RI2003392, NT2RI2003399, NT2RI2003407, NT2RI2003419, NT2RI2003487, NT2RI2003667, NT2RI2003678, NT2RI2003884, NT2RI2003973, NT2RI2003993, NT2RI2004059, NT2RI2004079, NT2RI2004093, NT2RI2004136, NT2RI2004157, NT2RI2004190, NT2RI2004209, NT2RI2004284, NT2RI2004290, NT2RI2004312, NT2RI2004381, NT2RI2004442, NT2RI2004468, NT2RI2004618, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005166, NT2RI2005246, NT2RI2005315, NT2RI2005358, NT2RI2005473, NT2RI2005520, NT2RI2005621, NT2RI2005628, NT2RI2005647, NT2RI2005670, NT2RI2005710, NT2RI2005772, NT2RI2005966, NT2RI2006070, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506, NT2RI2006565, NT2RI2006659, NT2RI2006662, NT2RI2006667, NT2RI2006679, NT2RI2006682, NT2RI2006686, NT2RI2006716, NT2RI2006762, NT2RI2006788, NT2RI2006838, NT2RI2006853, NT2RI2006855, NT2RI2006973, NT2RI2007046, NT2RI2007048, NT2RI2007116, NT2RI2007214, NT2RI2007303, NT2RI2007334, NT2RI2007386, NT2RI2007406, NT2RI2007439, NT2RI2007469, NT2RI2007507, NT2RI2007629, NT2RI2007723, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008188, NT2RI2008204, NT2RI2008221, NT2RI2008233, NT2RI2008455, NT2RI2008502, NT2RI2008598, NT2RI2008714, NT2RI2008801, NT2RI2008808, NT2RI2008841, NT2RI2008952, NT2RI2009005, NT2RI2009083, NT2RI2009101, NT2RI2009103, NT2RI2009144, NT2RI2009151, NT2RI2009215, NT2RI2009259, NT2RI2009289, NT2RI2009301, NT2RI2009517, NT2RI2009585, NT2RI2009595, NT2RI2009855, NT2RI2010283, NT2RI2010508, NT2RI2011422, NT2RI2011683, NT2RI2011803, NT2RI2012350, NT2RI2012990, NT2RI2013357, NT2RI2013373, NT2RI2014247, NT2RI2014551, NT2RI2014683, NT2RI2015239, NT2RI2016128, NT2RI2016134, NT2RI2017529, NT2RI2018363, NT2RI2018448, NT2RI2018883, NT2RI2018950, NT2RI2019751, NT2RI2019826, NT2RI2020390, NT2RI2020703, NT2RI2021463, NT2RI2022468, NT2RI2022584, NT2RI2023303, NT2RI2023671, NT2RI2024008, NT2RI2024460, NT2RI2024496, NT2RI2024935, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001458, NT2RI3001573, NT2RI3001967, NT2RI3002303, NT2RI3002557, NT2RI3003027, NT2RI3003031, NT2RI3003095, NT2RI3003104, NT2RI3003162, NT2RI3003362, NT2RI3003382, NT2RI3003409, NT2RI3004133, NT2RI3004381, NT2RI3005202, NT2RI3005416, NT2RI3005861, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008162, NT2RI3008179, NT2RI3008228, NT2RI3008442, NT2RI3008652, NT2RI3008697, NT2RI3008974, NT2RI3009158, NT2RI3009480, NT2RP6000005, NT2RP6000008, NT2RP6000032, NT2RP6000033, NT2RP6000059, NT2RP6000060, NT2RP6000086, NT2RP6000100, NT2RP6000119, NT2RP6000127, NT2RP6000130, NT2RP6000140, NT2RP6000150, NT2RP7000041, NT2RP7000086, NT2RP7000109, NT2RP7000112, NT2RP7000311, NT2RP7000359, NT2RP7000364, NT2RP7000380, NT2RP7000391, NT2RP7000425, NT2RP7000579, NT2RP7000812, NT2RP7000926, NT2RP7001074, NT2RP7001156, NT2RP7001319, NT2RP7001364, NT2RP7001475, NT2RP7001532, NT2RP7001591, NT2RP7001602, NT2RP7001678, NT2RP7001856, NT2RP7002028, NT2RP7002151, NT2RP7002163, NT2RP7002243, NT2RP7002282, NT2RP7002554, NT2RP7002603, NT2RP7002619, NT2RP7002650, NT2RP7002779, NT2RP7002829, NT2RP7002841, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003261, NT2RP7003439, NT2RP7003511, NT2RP7003724, NT2RP7004027, NT2RP7004080, NT2RP7004114, NT2RP7004260, NT2RP7004358, NT2RP7004396, NT2RP7004541, NT2RP7004641, NT2RP7004656, NT2RP7004687, NT2RP7004722, NT2RP7004766, NT2RP7004961, NT2RP7004964, NT2RP7004975, NT2RP7005118, NT2RP7005205, NT2RP7005219, NT2RP7005323, NT2RP7005468, NT2RP7005493, NT2RP7005629, NT2RP7005631, NT2RP7005669, NT2RP7005750, NT2RP7005846, NT2RP7006160, NT2RP7006162, NT2RP7006296, NT2RP7006304, NT2RP7006374, NT2RP7006408, NT2RP7006457, NT2RP7006547, NT2RP7006601, NT2RP7006621, NT2RP7006636, NT2RP7006853, NT2RP7006887, NT2RP7006980, NT2RP7006995, NT2RP7007114, NT2RP7007221, NT2RP7007226, NT2RP7007269, NT2RP7007310, NT2RP7007359, NT2RP7007406, NT2RP7007480, NT2RP7007504, NT2RP7007580, NT2RP7007594, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008360, NT2RP7008406, NT2RP7008435, NT2RP7008441, NT2RP7008454, NT2RP7008544, NT2RP7008550, NT2RP7008557, NT2RP7008623, NT2RP7008657, NT2RP7008720, NT2RP7008855, NT2RP7008863, NT2RP7009019, NT2RP7009030, NT2RP7009097, NT2RP7009168, NT2RP7009215, NT2RP7009236, NT2RP7009253, NT2RP7009267, NT2RP7009322, NT2RP7009363, NT2RP7009370, NT2RP7009397, NT2RP7009429, NT2RP7009439, NT2RP7009466, NT2RP7009502, NT2RP7009577, NT2RP7009743, NT2RP7009867, NT2RP7010109, NT2RP7010128, NT2RP7010235, NT2RP7010275, NT2RP7010521, NT2RP7010599, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013729, NT2RP7013795, NT2RP7013999, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015512, NT2RP7015676, NT2RP7015789, NT2RP7015996, NT2RP7016508, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019273, NT2RP7019367, NT2RP7019445, NT2RP7019682, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8003490, NT2RP8003657, NT2RP8003787, NT2RP8004306, NT2RP8005546, NT2RP8006452, NT2RP8006521, NT2RP8007416, NT2RP8007503, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000246, NTONG1000247, NTONG1000257, NTONG2000040, NTONG2000231, NTONG2000334, NTONG2000363, NTONG2000413, NTONG2000492, NTONG2000499, NTONG2000531, NTONG2000583, NTONG2000800, NTONG2000858, NTONG2000966, NTONG2000985, NTONG2001014, NTONG2001079, NTONG2001157, NTONG2001173, NTONG2001220, NTONG2001222, NTONG2001362, NTONG2001532, NTONG2001587, NTONG2001619, NTONG2001642, NTONG2001657, NTONG2001676, NTONG2002278, NTONG2002582, NTONG2002948, NTONG2002970, NTONG2003158, NTONG2003210, NTONG2003316, NTONG2003409, NTONG2003805, NTONG2003839, NTONG2003852, NTONG2003897, NTONG2003928, NTONG2004308, NTONG2004521, NTONG2004690, NTONG2004806, NTONG2004829, NTONG2004844, NTONG2004918, NTONG2004991, NTONG2005062, NTONG2005086, NTONG2005137, NTONG2005153, NTONG2005265, NTONG2005277, NTONG2005363, NTONG2005480, NTONG2005497, NTONG2005577, NTONG2005657, NTONG2005822, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007034, NTONG2007249, NTONG2007517, NTONG2007522, NTONG2007693, NTONG2007756, NTONG2008093, NTONG2008365, NTONG2008522, NTONG2008672, NTONG2008862, NTONG2008939, NTONG2009060, NTONG2009068, NTONG2009229, NTONG2009233, NTONG2009468, OCBBF1000016, OCBBF1000042, OCBBF1000054, OCBBF1000067, OCBBF1000085, OCBBF1000091, OCBBF1000104, OCBBF1000118, OCBBF1000119, OCBBF1000122, OCBBF1000130, OCBBF1000138, OCBBF1000153, OCBBF1000175, OCBBF1000192, OCBBF1000254, OCBBF2000013, OCBBF2000287, OCBBF2000323, OCBBF2000452, OCBBF2000467, OCBBF2000677, OCBBF2000703, OCBBF2000719, OCBBF2000998, OCBBF2001075, OCBBF2001101, OCBBF2001124, OCBBF2001176, OCBBF2001186, OCBBF2001196, OCBBF2001252, OCBBF2001323, OCBBF2001389, OCBBF2001402, OCBBF2001473, OCBBF2001494, OCBBF2001527, OCBBF2001528, OCBBF2001681, OCBBF2001706, OCBBF2001749, OCBBF2001910, OCBBF2001961, OCBBF2001983, OCBBF2002124, OCBBF2002161, OCBBF2002357, OCBBF2002376, OCBBF2002615, OCBBF2002663, OCBBF2002805, OCBBF2002865, OCBBF2002920, OCBBF2003028, OCBBF2003091, OCBBF2003327, OCBBF2003543, OCBBF2003744, OCBBF2004038, OCBBF2004147, OCBBF2004168, OCBBF2004235, OCBBF2004385, OCBBF2004418, OCBBF2004478, OCBBF2004482, OCBBF2004567, OCBBF2004612, OCBBF2004647, OCBBF2004826, OCBBF2004866, OCBBF2004883, OCBBF2005066, OCBBF2005077, OCBBF2005343, OCBBF2005349, OCBBF2005373, OCBBF2005433, OCBBF2005476, OCBBF2005843, OCBBF2005901, OCBBF2006030, OCBBF2006058, OCBBF2006113, OCBBF2006154, OCBBF2006214, OCBBF2006241, OCBBF2006388, OCBBF2006438, OCBBF2006567, OCBBF2006624, OCBBF2006639, OCBBF2006849, OCBBF2006859, OCBBF2007039, OCBBF2007114, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007931, OCBBF2007946, OCBBF2008116, OCBBF2008138, OCBBF2008283, OCBBF2008330, OCBBF2008366, OCBBF2008511, OCBBF2008520, OCBBF2008569, OCBBF2008586, OCBBF2008640, OCBBF2008691, OCBBF2008724, OCBBF2008760, OCBBF2008768, OCBBF2008770, OCBBF2008775, OCBBF2008790, OCBBF2008814, OCBBF2008822, OCBBF2009115, OCBBF2009391, OCBBF2009424, OCBBF2009571, OCBBF2009583, OCBBF2009603, OCBBF2009788, OCBBF2009926, OCBBF2010040, OCBBF2010281, OCBBF2010313, OCBBF2010404, OCBBF2010416, OCBBF2010420, OCBBF2010557, OCBBF2010604, OCBBF2010709, OCBBF2010792, OCBBF2010843, OCBBF2010863, OCBBF2010931, OCBBF2010945, OCBBF2010978, OCBBF2010989, OCBBF2011137, OCBBF2011177, OCBBF2011311, OCBBF2011625, OCBBF2011669, OCBBF2011685, OCBBF2011706, OCBBF2011722, OCBBF2011855, OCBBF2011872, OCBBF2011960, OCBBF2011981, OCBBF2012028, OCBBF2012139, OCBBF2012191, OCBBF2012320, OCBBF2012436, OCBBF2012525, OCBBF2012714, OCBBF2012755, OCBBF2012812, OCBBF2012821, OCBBF2012881, OCBBF2012990, OCBBF2013049, OCBBF2013091, OCBBF2013127, OCBBF2013208, OCBBF2013285, OCBBF2013319, OCBBF2013324, OCBBF2013507, OCBBF2013721, OCBBF2013883, OCBBF2013926, OCBBF2014052, OCBBF2014386, OCBBF2014707, OCBBF2014745, OCBBF2014828, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015506, OCBBF2015599, OCBBF2015645, OCBBF2015659, OCBBF2015751, OCBBF2015797, OCBBF2015980, OCBBF2016038, OCBBF2016405, OCBBF2016467, OCBBF2016590, OCBBF2016591, OCBBF2016612, OCBBF2016689, OCBBF2016690, OCBBF2016841, OCBBF2016928, OCBBF2017055, OCBBF2017069, OCBBF2017325, OCBBF2017458, OCBBF2017489, OCBBF2017516, OCBBF2017665, OCBBF2017815, OCBBF2018012, OCBBF2018014, OCBBF2018167, OCBBF2018229, OCBBF2018362, OCBBF2018380, OCBBF2018563, OCBBF2018663, OCBBF2018687, OCBBF2018828, OCBBF2018934, OCBBF2019108, OCBBF2019195, OCBBF2019327, OCBBF2019684, OCBBF2019761, OCBBF2019823, OCBBF2020048, OCBBF2020318, OCBBF2020343, OCBBF2020453, OCBBF2020801, OCBBF2020825, OCBBF2020838, OCBBF2021020, OCBBF2021214, OCBBF2021323, OCBBF2021518, OCBBF2021788, OCBBF2022351, OCBBF2022573, OCBBF2022574, OCBBF2023162, OCBBF2023598, OCBBF2023643, OCBBF2024463, OCBBF2024589, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028173, OCBBF2028421, OCBBF2029471, OCBBF2029901, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF3000097, OCBBF3000213, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008392, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000248, PEBLM2000270, PEBLM2000321, PEBLM2000333, PEBLM2000338, PEBLM2000479, PEBLM2000502, PEBLM2001312, PEBLM2001465, PEBLM2001488, PEBLM2001803, PEBLM2002432, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006298, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008861, PERIC1000025, PERIC1000147, PERIC2000180, PERIC2000214, PERIC2000386, PERIC2000387, PERIC2000422, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000245, PLACE5000260, PLACE5000282, PLACE5000372, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000296, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001262, PLACE6001294, PLACE6001536, PLACE6001557, PLACE6001712, PLACE6001740, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6002960, PLACE6003038, PLACE6003077, PLACE6003094, PLACE6003109, PLACE6003372, PLACE6003393, PLACE6003399, PLACE6003539, PLACE6003621, PLACE6003705, PLACE6003740, PLACE6003771, PLACE6003796, PLACE6003839, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005328, PLACE6005351, PLACE6005423, PLACE6005487, PLACE6005535, PLACE6005546, PLACE6005559, PLACE6005579, PLACE6005691, PLACE6005786, PLACE6006042, PLACE6006077, PLACE6006158, PLACE6006217, PLACE6006266, PLACE6006287, PLACE6006394, PLACE6006474, PLACE6006549, PLACE6006566, PLACE6006697, PLACE6006822, PLACE6006871, PLACE6006905, PLACE6006988, PLACE6007050, PLACE6007239, PLACE6007242, PLACE6007380, PLACE6007433, PLACE6007482, PLACE6007687, PLACE6007787, PLACE6007798, PLACE6007807, PLACE6008036, PLACE6008094, PLACE6008126, PLACE6008285, PLACE6008444, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009835, PLACE6009853, PLACE6010463, PLACE6010485, PLACE6010568, PLACE6010701, PLACE6010765, PLACE6010848, PLACE6010925, PLACE6010936, PLACE6010991, PLACE6011041, PLACE6011102, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013884, PLACE6013885, PLACE6014043, PLACE6014516, PLACE6014882, PLACE6015211, PLACE6015322, PLACE6015460, PLACE6015513, PLACE6015591, PLACE6015729, PLACE6015939, PLACE6016030, PLACE6016093, PLACE6016105, PLACE6016160, PLACE6016210, PLACE6016254, PLACE6016383, PLACE6017002, PLACE6017431, PLACE6017564, PLACE6017578, PLACE6017626, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006268, PLACE7006275, PLACE7006498, PLACE7006540, PLACE7007379, PLACE7007644, PLACE7007973, PLACE7008136, PLACE7008431, PLACE7008623, PLACE7008766, PLACE7009563, PROST1000033, PROST1000039, PROST1000065, PROST1000083, PROST1000110, PROST1000120, PROST1000123, PROST1000136, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000212, PROST2000241, PROST2000267, PROST2000273, PROST2000274, PROST2000277, PROST2000284, PROST2000325, PROST2000337, PROST2000432, PROST2000452, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001213, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003340, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004742, PROST2004744, PROST2004817, PROST2005039, PROST2005093, PROST2005121, PROST2005131, PROST2005143, PROST2005228, PROST2005285, PROST2005426, PROST2005466, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006282, PROST2006343, PROST2006450, PROST2006491, PROST2006510, PROST2006536, PROST2006579, PROST2006688, PROST2006737, PROST2006766, PROST2006782, PROST2006933, PROST2007122, PROST2007200, PROST2007237, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009365, PROST2009388, PROST2009400, PROST2009470, PROST2009483, PROST2009571, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012400, PROST2012504, PROST2012542, PROST2012550, PROST2012740, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015287, PROST2015457, PROST2015537, PROST2015545, PROST2015551, PROST2015636, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017203, PROST2017367, PROST2017413, PROST2017441, PROST2017578, PROST2017612, PROST2017617, PROST2017700, PROST2017701, PROST2017729, PROST2017836, PROST2017910, PROST2018030, PROST2018090, PROST2018235, PROST2018437, PROST2018487, PROST2018511, PROST2018607, PROST2018788, PROST2018902, PROST2018922, PROST2019296, PROST2019398, PROST2019487, PROST2019781, PUAEN1000039, PUAEN1000081, PUAEN1000085, PUAEN1000087, PUAEN1000121, PUAEN1000175, PUAEN1000275, PUAEN1000276, PUAEN1000288, PUAEN2000080, PUAEN2000175, PUAEN2000247, PUAEN2000394, PUAEN2000497, PUAEN2000594, PUAEN2000684, PUAEN2000942, PUAEN2001260, PUAEN2001526, PUAEN2001882, PUAEN2002473, PUAEN2002552, PUAEN2002568, PUAEN2002616, PUAEN2002758, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2006029, PUAEN2006328, PUAEN2006578, PUAEN2006639, PUAEN2006761, PUAEN2007350, PUAEN2007785, PUAEN2007898, PUAEN2008123, PUAEN2008228, PUAEN2008314, PUAEN2008815, PUAEN2008939, PUAEN2008980, PUAEN2009174, PUAEN2009348, PUAEN2009534, PUAEN2009795, PUAEN2009852, RECTM1000051, RECTM1000141, RECTM2000220, RECTM2000349, RECTM2000433, RECTM2000510, RECTM2001307, RECTM2001519, RECTM2001666, RECTM2001691, RECTM2001749, RECTM2002172, RECTM2002602, RECTM2002632, SALGL1000024, SALGL1000028, SALGL1000065, SALGL1000107, SALGL1000171, SKMUS1000030, SKMUS1000064, SKMUS1000067, SKMUS1000104, SKMUS1000118, SKMUS1000124, SKMUS1000129, SKMUS1000138, SKMUS2000061, SKMUS2000074, SKMUS2000078, SKMUS2000137, SKMUS2000198, SKMUS2000243, SKMUS2000287, SKMUS2000317, SKMUS2000330, SKMUS2000339, SKMUS2000343, SKMUS2000365, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705, SKMUS2000710, SKMUS2000724, SKMUS2000726, SKMUS2000774, SKMUS2000780, SKMUS2000821, SKMUS2000847, SKMUS2000863, SKMUS2000894, SKMUS2000898, SKMUS2000902, SKMUS2000933, SKMUS2000945, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000229, SKNMC1000251, SKNMC1000264, SKNMC2000224, SKNMC2000256, SKNMC2000356, SKNMC2000374, SKNMC2000583, SKNMC2000593, SKNMC2000612, SKNMC2000622, SKNMC2000698, SKNMC2000966, SKNMC2001113, SKNMC2001324, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000151, SKNSH2000163, SKNSH2000319, SKNSH2000482, SKNSH2000516, SKNSH2000550, SKNSH2000971, SKNSH2001931, SKNSH2002054, SKNSH2002768, SKNSH2002866, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009435, SKNSH2009991, SKNSH2010015, SMINT1000012, SMINT1000016, SMINT1000039, SMINT1000042, SMINT1000051, SMINT1000054, SMINT1000057, SMINT1000071, SMINT1000075, SMINT1000078, SMINT1000091, SMINT1000103, SMINT1000118, SMINT1000137, SMINT1000192, SMINT2000007, SMINT2000018, SMINT2000032, SMINT2000040, SMINT2000060, SMINT2000084, SMINT2000145, SMINT2000159, SMINT2000176, SMINT2000185, SMINT2000227, SMINT2000229, SMINT2000232, SMINT2000277, SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000747, SMINT2000811, SMINT2000824, SMINT2000984, SMINT2001114, SMINT2001158, SMINT2001199, SMINT2001222, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2002126, SMINT2002202, SMINT2002210, SMINT2002281, SMINT2002311, SMINT2002314, SMINT2002328, SMINT2002414, SMINT2002457, SMINT2002743, SMINT2002778, SMINT2002880, SMINT2002882, SMINT2002884, SMINT2002899, SMINT2002974, SMINT2003003, SMINT2003074, SMINT2003128, SMINT2003150, SMINT2003317, SMINT2003340, SMINT2003386, SMINT2003505, SMINT2003551, SMINT2003569, SMINT2003641, SMINT2003644, SMINT2003866, SMINT2004037, SMINT2004047, SMINT2004086, SMINT2004144, SMINT2004280, SMINT2004299, SMINT2004339, SMINT2004414, SMINT2004473, SMINT2004547, SMINT2004583, SMINT2004729, SMINT2004781, SMINT2004891, SMINT2004909, SMINT2004972, SMINT2004992, SMINT2005075, SMINT2005161, SMINT2005174, SMINT2005213, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008672, SMINT2008690, SMINT2008715, SMINT2008720, SMINT2008817, SMINT2008844, SMINT2008917, SMINT2009119, SMINT2009121, SMINT2009212, SMINT2009216, SMINT2009233, SMINT2009259, SMINT2009272, SMINT2009363, SMINT2009468, SMINT2009505, SMINT2009529, SMINT2009832, SMINT2009895, SMINT2009902, SMINT2009973, SMINT2010015, SMINT2010068, SMINT2010076, SMINT2010200, SMINT2010278, SMINT2010326, SMINT2010369, SMINT2010426, SMINT2010500, SMINT2010533, SMINT2010629, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012195, SMINT2012285, SMINT2012291, SMINT2012734, SMINT2012793, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016286, SMINT2016313, SMINT2016412, SMINT2016440, SMINT2016477, SMINT2016544, SMINT2016857, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000041, SPLEN1000056, SPLEN1000087, SPLEN1000089, SPLEN1000091, SPLEN1000134, SPLEN1000139, SPLEN1000143, SPLEN1000145, SPLEN1000156, SPLEN1000163, SPLEN1000178, SPLEN2000020, SPLEN2000047, SPLEN2000064, SPLEN2000072, SPLEN2000126, SPLEN2000143, SPLEN2000189, SPLEN2000205, SPLEN2000210, SPLEN2000242, SPLEN2000243, SPLEN2000247, SPLEN2000270, SPLEN2000307, SPLEN2000310, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000541, SPLEN2000607, SPLEN2000630, SPLEN2000695, SPLEN2000839, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001650, SPLEN2001689, SPLEN2001710, SPLEN2001761, SPLEN2001781, SPLEN2001881, SPLEN2001912, SPLEN2001945, SPLEN2002007, SPLEN2002133, SPLEN2002147, SPLEN2002166, SPLEN2002175, SPLEN2002223, SPLEN2002314, SPLEN2002343, SPLEN2002354, SPLEN2002385, SPLEN2002419, SPLEN2002451, SPLEN2002462, SPLEN2002467, SPLEN2002477, SPLEN2002662, SPLEN2002695, SPLEN2002707, SPLEN2002917, SPLEN2002957, SPLEN2003160, SPLEN2003204, SPLEN2003219, SPLEN2003297, SPLEN2003878, SPLEN2003918, SPLEN2003924, SPLEN2004060, SPLEN2004078, SPLEN2004124, SPLEN2004148, SPLEN2004181, SPLEN2004220, SPLEN2004346, SPLEN2004368, SPLEN2004611, SPLEN2004662, SPLEN2004844, SPLEN2005227, SPLEN2005272, SPLEN2005416, SPLEN2005450, SPLEN2005474, SPLEN2005727, SPLEN2005790, SPLEN2005818, SPLEN2005869, SPLEN2005939, SPLEN2006232, SPLEN2006268, SPLEN2006283, SPLEN2006325, SPLEN2006389, SPLEN2006425, SPLEN2006671, SPLEN2006701, SPLEN2006785, SPLEN2006875, SPLEN2007314, SPLEN2007326, SPLEN2007350, SPLEN2007388, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2010004, SPLEN2010112, SPLEN2010119, SPLEN2010146, SPLEN2010187, SPLEN2010350, SPLEN2010395, SPLEN2010415, SPLEN2010447, SPLEN2010510, SPLEN2010530, SPLEN2010534, SPLEN2010588, SPLEN2010625, SPLEN2010800, SPLEN2010846, SPLEN2010847, SPLEN2010862, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012725, SPLEN2012743, SPLEN2012756, SPLEN2012846, SPLEN2012889, SPLEN2012961, SPLEN2013108, SPLEN2013195, SPLEN2013644, SPLEN2013648, SPLEN2013670, SPLEN2013673, SPLEN2013690, SPLEN2013714, SPLEN2013753, SPLEN2013821, SPLEN2013860, SPLEN2013910, SPLEN2013923, SPLEN2014041, SPLEN2014063, SPLEN2014073, SPLEN2014080, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014669, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016480, SPLEN2016531, SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016973, SPLEN2017043, SPLEN2017104, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018180, SPLEN2018181, SPLEN2018280, SPLEN2018285, SPLEN2018364, SPLEN2018395, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019375, SPLEN2019379, SPLEN2019446, SPLEN2019471, SPLEN2019480, SPLEN2019505, SPLEN2019571, SPLEN2019575, SPLEN2019709, SPLEN2019793, SPLEN2019811, SPLEN2019839, SPLEN2019956, SPLEN2019985, SPLEN2020007, SPLEN2020153, SPLEN2020154, SPLEN2020166, SPLEN2020183, SPLEN2020223, SPLEN2020359, SPLEN2020427, SPLEN2020467, SPLEN2020512, SPLEN2020608, SPLEN2020777, SPLEN2020805, SPLEN2021057, SPLEN2021157, SPLEN2021231, SPLEN2021295, SPLEN2021298, SPLEN2021383, SPLEN2021417, SPLEN2021440, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024530, SPLEN2024571, SPLEN2024577, SPLEN2024617, SPLEN2024737, SPLEN2024956, SPLEN2024990, SPLEN2025012, SPLEN2025017, SPLEN2025039, SPLEN2025219, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031724, SPLEN2031780, SPLEN2032036, SPLEN2032112, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034864, SPLEN2034934, SPLEN2035318, SPLEN2035615, SPLEN2036103, SPLEN2036326, SPLEN2036475, SPLEN2036501, SPLEN2036579, SPLEN2036608, SPLEN2036702, SPLEN2036712, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000279, STOMA2000383, STOMA2000386, STOMA2000395, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005664, STOMA2005667, STOMA2005746, STOMA2005782, STOMA2006181, STOMA2006213, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000164, SYNOV1000190, SYNOV1000256, SYNOV2000152, SYNOV2000171, SYNOV2000173, SYNOV2000177, SYNOV2000178, SYNOV2000193, SYNOV2000251, SYNOV2000279, SYNOV2000297, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001251, SYNOV2001300, SYNOV2001356, SYNOV2001374, SYNOV2001390, SYNOV2001451, SYNOV2001457, SYNOV2001468, SYNOV2001660, SYNOV2003326, SYNOV2005216, SYNOV2005448, SYNOV2005541, SYNOV2005673, SYNOV2005817, SYNOV2005962, SYNOV2006430, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4006256, SYNOV4006327, SYNOV4007012, SYNOV4007215, SYNOV4007360, SYNOV4007521, SYNOV4007553, SYNOV4007711, SYNOV4008336, SYNOV4009139, SYNOV4009298, T1ESE2000116, T1ESE2000609, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN2000139, TCOLN2000236, TCOLN2000641, TCOLN2001268, TCOLN2002047, TCOLN2002278, TESOP1000127, TESOP1000160, TESOP2000083, TESOP2000090, TESOP2000205, TESOP2000312, TESOP2000390, TESOP2000400, TESOP2000510, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000064, TESTI1000085, TESTI1000088, TESTI1000093, TESTI1000109, TESTI1000125, TESTI1000127, TESTI1000128, TESTI1000131, TESTI1000138, TESTI1000142, TESTI1000157, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000179, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000443, TESTI2000452, TESTI2000462, TESTI2000571, TESTI2000591, TESTI2000598, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000850, TESTI2000909, TESTI2000951, TESTI2000981, TESTI2001008, TESTI2001049, TESTI2001082, TESTI2001134, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001879, TESTI2001894, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003545, TESTI2003551, TESTI2003574, TESTI2003579, TESTI2003580, TESTI2003589, TESTI2003598, TESTI2003615, TESTI2003638, TESTI2003649, TESTI2003718, TESTI2003727, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005739, TESTI2005767, TESTI2005775, TESTI2005827, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006120, TESTI2006125, TESTI2006179, TESTI2006209, TESTI2006210, TESTI2006212, TESTI2006218, TESTI2006238, TESTI2006255, TESTI2006278, TESTI2006333, TESTI2006341, TESTI2006360, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014800, TESTI2014843, TESTI2014979, TESTI2014988, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016033, TESTI2016046, TESTI2016101, TESTI2016162, TESTI2016197, TESTI2016298, TESTI2016331, TESTI2016337, TESTI2016410, TESTI2016421, TESTI2016509, TESTI2016568, TESTI2016599, TESTI2016629, TESTI2016663, TESTI2016667, TESTI2016688, TESTI2016701, TESTI2016758, TESTI2016848, TESTI2016863, TESTI2016888, TESTI2016922, TESTI2016942, TESTI2016950, TESTI2016996, TESTI2017017, TESTI2017028, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020981, TESTI2020999, TESTI2021003, TESTI2021057, TESTI2021077, TESTI2021112, TESTI2021114, TESTI2021116, TESTI2021118, TESTI2021122, TESTI2021138, TESTI2021315, TESTI2021447, TESTI2021463, TESTI2021599, TESTI2021637, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025791, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035262, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473,. TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001665, TESTI4001679, TESTI4001795, TESTI4001923, TESTI4001925, TESTI4002003, TESTI4002141, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003179, TESTI4003279, TESTI4003319, TESTI4003404, TESTI4003565, TESTI4003574, TESTI4003579, TESTI4003733, TESTI4003796,TESTI4004003, TESTI4004031, TESTI4004200, TESTI4004539, TESTI4004653, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006148, TESTI4006219, TESTI4006234, TESTI4006308, TESTI4006326, TESTI4006393, TESTI4006412, TESTI4006420, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4008007, TESTI4008018, TESTI4008050, TESTI4008086, TESTI4008176, TESTI4008209, TESTI4008219, TESTI4008250, TESTI4008302, TESTI4008305, TESTI4008401, TESTI4008417, TESTI4008429, TESTI4008573, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010475, TESTI4010544, TESTI4010713, TESTI4010721, TESTI4010789, TESTI4010817, TESTI4010831, TESTI4010902, TESTI4011070, TESTI4011072, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012505, TESTI4012556, TESTI4012623, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013830, TESTI4013852, TESTI4013924, TESTI4013960, TESTI4013962, TESTI4014175, TESTI4014265, TESTI4014276, TESTI4014306, TESTI4014392, TESTI4014415, TESTI4014445, TESTI4014553, TESTI4014661, TESTI4014801, TESTI4014891, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024494, TESTI4024740, TESTI4024874, TESTI4024890, TESTI4024907, TESTI4024930, TESTI4025062, TESTI4025401, TESTI4025494, TESTI4025731, TESTI4025797, TESTI4025865, TESTI4025908, TESTI4025920, TESTI4026079, TESTI4026080, TESTI4026192, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046090, TESTI4046240, TESTI4046245, TESTI4046253, TESTI4046282, TESTI4046328, TESTI4046450, TESTI4046457, TESTI4046487, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000032, THYMU1000033, THYMU1000060, THYMU1000074, THYMU1000098, THYMU1000136, THYMU1000142, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000140, THYMU2000162, THYMU2000171, THYMU2000176, THYMU2000179, THYMU2000231, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000800, THYMU2000879, THYMU2000932, THYMU2000950, THYMU2000982, THYMU2000990, THYMU2001053, THYMU2001071, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443, THYMU2001521, THYMU2001556, THYMU2001622, THYMU2001727, THYMU2001819, THYMU2001825, THYMU2001839, THYMU2001898, THYMU2001900, THYMU2001916, THYMU2001926, THYMU2001989, THYMU2002023, THYMU2002037, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002841, THYMU2002852, THYMU2002910, THYMU2002950, THYMU2002983, THYMU2003069, THYMU2003107, THYMU2003124, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004508, THYMU2004512, THYMU2004639, THYMU2004668, THYMU2004677, THYMU2004688, THYMU2004693, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006666, THYMU2006712, THYMU2006913, THYMU2006946, THYMU2006965, THYMU2007025, THYMU2007060, THYMU2007065, THYMU2007095, THYMU2007146, THYMU2007179, THYMU2007258, THYMU2007307, THYMU2007308, THYMU2007339, THYMU2007350, THYMU2007384, THYMU2007415, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007802, THYMU2007824, THYMU2007854, THYMU2007952, THYMU2007969, THYMU2008036, THYMU2008072, THYMU2008111, THYMU2008145, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010046, THYMU2010059, THYMU2010082, THYMU2010094, THYMU2010161, THYMU2010448, THYMU2010519, THYMU2010637, THYMU2010671, THYMU2010699, THYMU2010705, THYMU2010779, THYMU2010816, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012882, THYMU2012891, THYMU2012902, THYMU2012919, THYMU2013023, THYMU2013047, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015943, THYMU2015984, THYMU2016113, THYMU2016164, THYMU2016204, THYMU2016219, THYMU2016360, THYMU2016496, THYMU2016523, THYMU2016840, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023711, THYMU2023900, THYMU2024071, THYMU2024121, THYMU2024185, THYMU2024207, THYMU2024616, THYMU2024684, THYMU2024748, THYMU2025042, THYMU2025189, THYMU2025406, THYMU2025557, THYMU2025572, THYMU2025849, THYMU2025909, THYMU2025939, THYMU2026182, THYMU2026350, THYMU2026530, THYMU2026531, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031994, THYMU2032014, THYMU2032035, THYMU2032080, THYMU2032150, THYMU2032155, THYMU2032358, THYMU2032437, THYMU2032601, THYMU2032655, THYMU2032715, THYMU2032732, THYMU2032976, THYMU2033053, THYMU2033059, THYMU2033070, THYMU2033079, THYMU2033085, THYMU2033104, THYMU2033227, THYMU2033401, THYMU2033583, THYMU2033755, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036252, THYMU2036265, THYMU2036366, THYMU2036459, THYMU2036604, THYMU2036653, THYMU2037081, THYMU2037208, THYMU2037233, THYMU2037234, THYMU2037348, THYMU2037406, THYMU2037965, THYMU2038058, THYMU2038189, THYMU2038199, THYMU2038301, THYMU2038369, THYMU2038389, THYMU2038615, THYMU2038636, THYMU2038739, THYMU2038772, THYMU2038797, THYMU2038906, THYMU2039305, THYMU2039334, THYMU2039350, THYMU2039411, THYMU2039719, THYMU2039780, THYMU2039788, THYMU2039989, THYMU2040029, THYMU2040110, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000390, THYMU3000490, THYMU3000504, THYMU3000542, THYMU3000655, THYMU3000826, THYMU3000841, THYMU3001077, THYMU3001082, THYMU3001083, THYMU3001133, THYMU3001151, THYMU3001472, THYMU3001593, THYMU3001883, THYMU3001939, THYMU3001991, THYMU3002452, THYMU3002578, THYMU3002661, THYMU3003092, THYMU3003212, THYMU3003309, THYMU3003403, THYMU3003565, THYMU3003763, THYMU3003865, THYMU3003958, THYMU3004157, THYMU3004628, THYMU3004632, THYMU3004866, THYMU3005050, THYMU3005378, THYMU3005407, THYMU3005415, THYMU3005482, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007368, THYMU3007559, THYMU3007845, THYMU3008060, THYMU3008136, THYMU3008216, THYMU3008436, THYMU3008672, THYMU3008883, THYMU3008935, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037836, THYMU3037856, THYMU3037867, THYMU3037909, THYMU3037980, THYMU3038158, THYMU3038167, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046360, THYMU3046856, THYMU3047115, THYMU3047144, THYMU3047156, THYMU3047366, THYMU3047513, THYMU3047542, THYMU3047760, THYMU3047891, TKIDN1000062, TKIDN1000066, TKIDN1000171, TKIDN2000127, TKIDN2000240, TKIDN2000464, TKIDN2000701, TKIDN2001529, TKIDN2002329, TKIDN2002424, TKIDN2002632, TKIDN2003044, TKIDN2003062, TKIDN2003366, TKIDN2003396, TKIDN2003413, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007667, TKIDN2008176, TKIDN2008778, TKIDN2009092, TKIDN2009481, TKIDN2010200, TKIDN2010232, TKIDN2010278, TKIDN2010602, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014757, TKIDN2014771, TKIDN2015025, TKIDN2015161, TKIDN2015263, TKIDN2015285, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2001281, TOVAR2001556, TOVAR2001730, TOVAR2002247, TOVAR2002514, TOVAR2002549, TOVAR2002800, TRACH1000038, TRACH1000057, TRACH1000063, TRACH1000074, TRACH1000100, TRACH1000106, TRACH1000125, TRACH1000140, TRACH1000144, TRACH1000212, TRACH1000240, TRACH2000002, TRACH2000015, TRACH2000024, TRACH2000032, TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000306, TRACH2000312, TRACH2000336, TRACH2000411, TRACH2000496, TRACH2000497, TRACH2000522, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000923, TRACH2000944, TRACH2000981, TRACH2000986, TRACH2001037, TRACH2001060, TRACH2001101, TRACH2001121, TRACH2001147, TRACH2001154, TRACH2001188, TRACH2001192, TRACH2001249, TRACH2001275, TRACH2001335, TRACH2001347, TRACH2001395, TRACH2001400, TRACH2001403, TRACH2001416, TRACH2001443, TRACH2001474, TRACH2001523, TRACH2001525, TRACH2001532, TRACH2001592, TRACH2001596, TRACH2001621, TRACH2001651, TRACH2001684, TRACH2001933, TRACH2001968, TRACH2002043, TRACH2002054, TRACH2002092, TRACH2002138, TRACH2002784, TRACH2002840, TRACH2002847, TRACH2002853, TRACH2002862, TRACH2002954, TRACH2002960, TRACH2002988, TRACH2003070, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005666, TRACH2005698, TRACH2005710, TRACH2005720, TRACH2005769, TRACH2005800, TRACH2006015, TRACH2006097, TRACH2006194, TRACH2006327, TRACH2006331, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008081, TRACH2008112, TRACH2008127, TRACH2008130, TRACH2008278, TRACH2008540, TRACH2008583, TRACH2008597, TRACH2008820, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011574, TRACH2011956, TRACH2012000, TRACH2012138, TRACH2012242, TRACH2012370, TRACH2012387, TRACH2012536, TRACH2012562, TRACH2012742, TRACH2012823, TRACH2012918, TRACH2013081, TRACH2013123, TRACH2013265, TRACH2013369, TRACH2013450, TRACH2013495, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015654, TRACH2015824, TRACH2015987, TRACH2016080, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000017, TRACH3000043, TRACH3000180, TRACH3000342, TRACH3000420, TRACH3000515, TRACH3000530, TRACH3000548, TRACH3000558, TRACH3000586, TRACH3000692, TRACH3000892, TRACH3000926, TRACH3001119, TRACH3001443, TRACH3001759, TRACH3002064, TRACH3002107, TRACH3002168, TRACH3002188, TRACH3002245, TRACH3002293, TRACH3002533, TRACH3002561, TRACH3002567, TRACH3002650, TRACH3002866, TRACH3002871, TRACH3002876, TRACH3002890, TRACH3003009, TRACH3003357, TRACH3003379, TRACH3003586, TRACH3003683, TRACH3003780, TRACH3003805, TRACH3003832, TRACH3004113, TRACH3004288, TRACH3004303, TRACH3004412, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007766, TRACH3007866, TRACH3007995, TRACH3008042, TRACH3008061, TRACH3008535, TRACH3008629, TRACH3008688, TRACH3008697, TRACH3008713, TRACH3008887, TRACH3008902, TRACH3008990, TRACH3009059, TRACH3009061, TRACH3009701, TRACH3009715, TRACH3009894, TRACH3009956, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024423, TRACH3024428, TRACH3024512, TRACH3024823, TRACH3025302, TRACH3025346, TRACH3025520, TRACH3026148, TRACH3026283, TRACH3026299, TRACH3026542, TRACH3026650, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036997, TRACH3037063, TRACH3037267, TRACH3037288, TRACH3037505, TRACH3037573, TRACH3037696, TRACH3037897, TRACH3038086, TSTOM1000186, TSTOM2000139, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000156, UMVEN2000046, UMVEN2000069, UTERU1000008, UTERU1000015, UTERU1000024, UTERU1000031, UTERU1000032, UTERU1000057, UTERU1000065, UTERU1000077, UTERU1000093, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU2000047, UTERU2000074, UTERU2000099, UTERU2000154, UTERU2000197, UTERU2000218, UTERU2000238, UTERU2000243, UTERU2000263, UTERU2000300, UTERU2000338, UTERU2000349, UTERU2000377, UTERU2000393, UTERU2000465, UTERU2000485, UTERU2000517, UTERU2000524, UTERU2000537, UTERU2000539, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2001024, UTERU2001176, UTERU2001281, UTERU2001389, UTERU2001409, UTERU2001412, UTERU2001504, UTERU2001747, UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004197, UTERU2004299, UTERU2004461, UTERU2004520, UTERU2004564, UTERU2004688, UTERU2004698, UTERU2004861, UTERU2005004, UTERU2005050, UTERU2005069, UTERU2005179, UTERU2005292, UTERU2005346, UTERU2005446, UTERU2005449, UTERU2005450, UTERU2005533, UTERU2005822, UTERU2005903, UTERU2005905, UTERU2006103, UTERU2006137, UTERU2006182, UTERU2006400, UTERU2006412, UTERU2006429, UTERU2006568, UTERU2006651, UTERU2006705, UTERU2006899, UTERU2007075, UTERU2007081, UTERU2007128, UTERU2007267, UTERU2007444, UTERU2007499, UTERU2007639, UTERU2007724, UTERU2007942, UTERU2008019, UTERU2008027, UTERU2008077, UTERU2008347, UTERU2008516, UTERU2008561, UTERU2008653, UTERU2008705, UTERU2008707, UTERU2008785, UTERU2008845, UTERU2008901, UTERU2008930, UTERU2008962, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010431, UTERU2010525, UTERU2010651, UTERU2010724, UTERU2010747, UTERU2011195, UTERU2011199, UTERU2011220, UTERU2011261, UTERU2011410, UTERU2011574, UTERU2011657, UTERU2011741, UTERU2011811, UTERU2011897, UTERU2011962, UTERU2011968, UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012741, UTERU2012767, UTERU2012786, UTERU2012856, UTERU2012890, UTERU2012938, UTERU2012976, UTERU2013048, UTERU2013231, UTERU2013262, UTERU2013280, UTERU2013322, UTERU2013483, UTERU2013491, UTERU2013502, UTERU2013926, UTERU2013976, UTERU2014001, UTERU2014223, UTERU2014398, UTERU2014548, UTERU2014631, UTERU2014668, UTERU2014678, UTERU2014728, UTERU2014998, UTERU2015062, UTERU2015087, UTERU2015190, UTERU2015198, UTERU2015202, UTERU2015405, UTERU2015640, UTERU2015830, UTERU2015880, UTERU2016147, UTERU2016157, UTERU2016464, UTERU2016669, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018333, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019163, UTERU2019257, UTERU2019453, UTERU2019491, UTERU2019534, UTERU2019681, UTERU2019710, UTERU2019959, UTERU2019964, UTERU2020226, UTERU2020242, UTERU2020292, UTERU2020351, UTERU2020491, UTERU2020718, UTERU2021380, UTERU2021649, UTERU2022020, UTERU2022773, UTERU2022955, UTERU2022981, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026775, UTERU2027023, UTERU2027369, UTERU2027591, UTERU2027616, UTERU2027941, UTERU2028377, UTERU2029503, UTERU2029660, UTERU2029742, UTERU2029953, UTERU2030270, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000402, UTERU3000645, UTERU3000665, UTERU3000670, UTERU3000727, UTERU3000738, UTERU3000828, UTERU3000844, UTERU3000959, UTERU3001029, UTERU3001053, UTERU3001059, UTERU3001240, UTERU3001394, UTERU3001542, UTERU3001558, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003178, UTERU3003465, UTERU3003495, UTERU3003523, UTERU3003774, UTERU3003776, UTERU3004477, UTERU3004523, UTERU3004616, UTERU3004635, UTERU3004938, UTERU3004992, UTERU3005049, UTERU3005205, UTERU3005264, UTERU3005460, UTERU3005536, UTERU3005585, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007731, UTERU3007913, UTERU3008463, UTERU3008660, UTERU3008671, UTERU3008722, UTERU3008917, UTERU3009490, UTERU3009517, UTERU3009690, UTERU3009775, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012476, UTERU3012599, UTERU3012999, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016274, UTERU3016308, UTERU3016789, UTERU3017176, UTERU3017441, UTERU3017626, UTERU3018154, UTERU3018255, UTERU3018616, UTERU3018711, UTERU3019078

Of the 17,176 genes that include the cDNAs of the present invention, genes whose expression levels were high enough to detect in each of the CD34C, CERVX, CHONS, COLON, CORDB, and CTONG libraries were analyzed for expression frequency. The analysis results are described below. Each is a relative value, where the expression level over all tissues is taken as 100. The larger this value, the greater the level of expression.

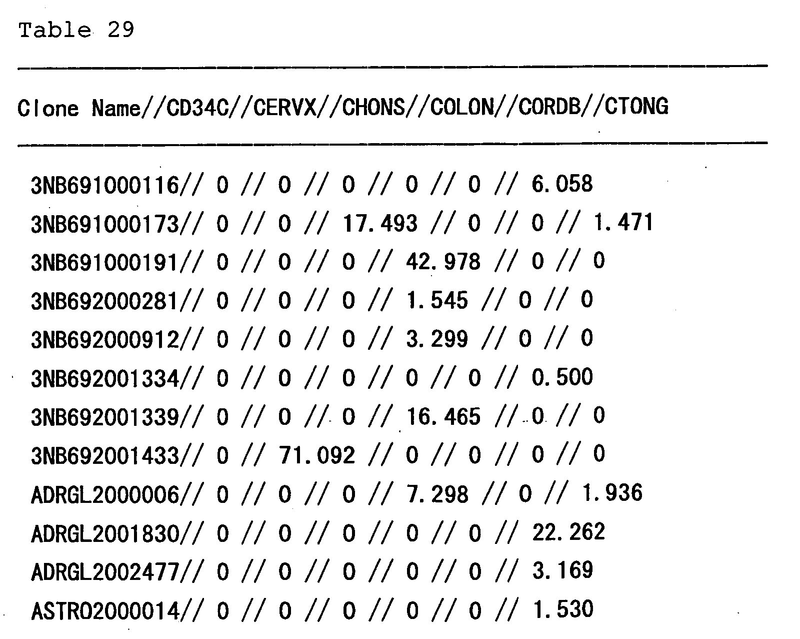

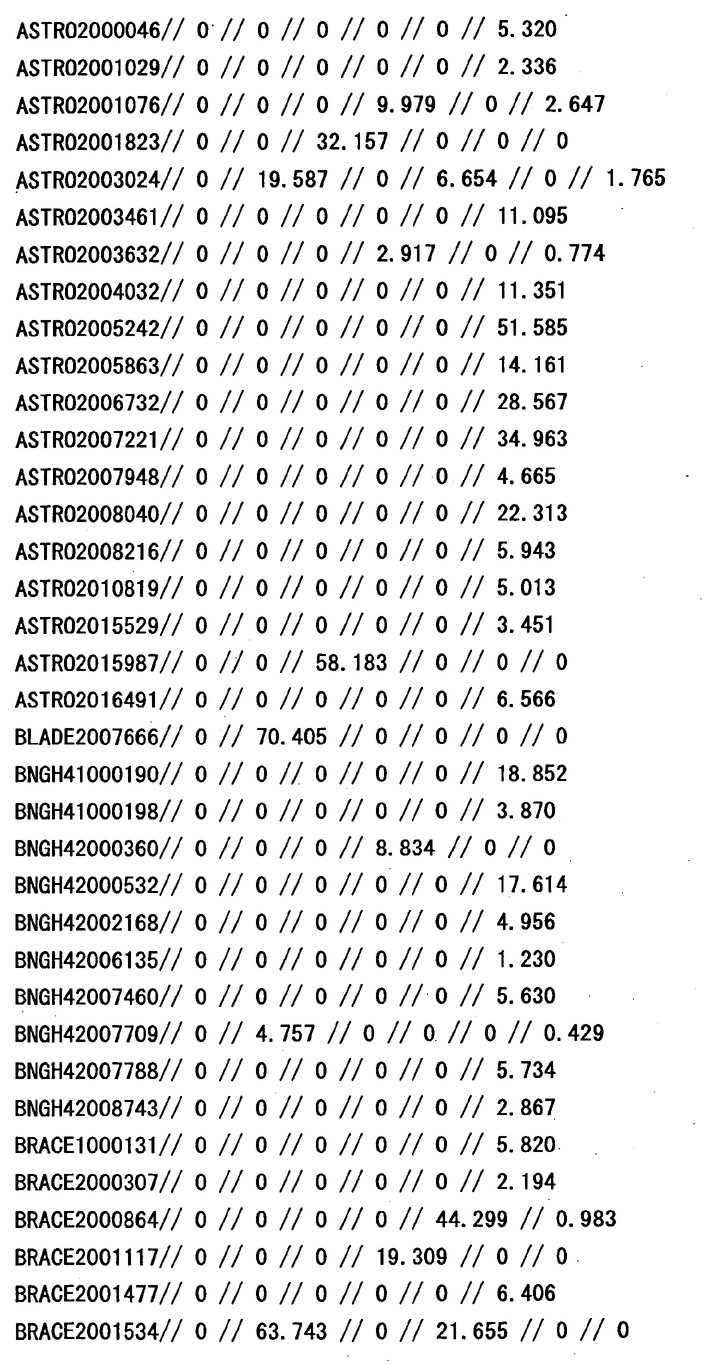

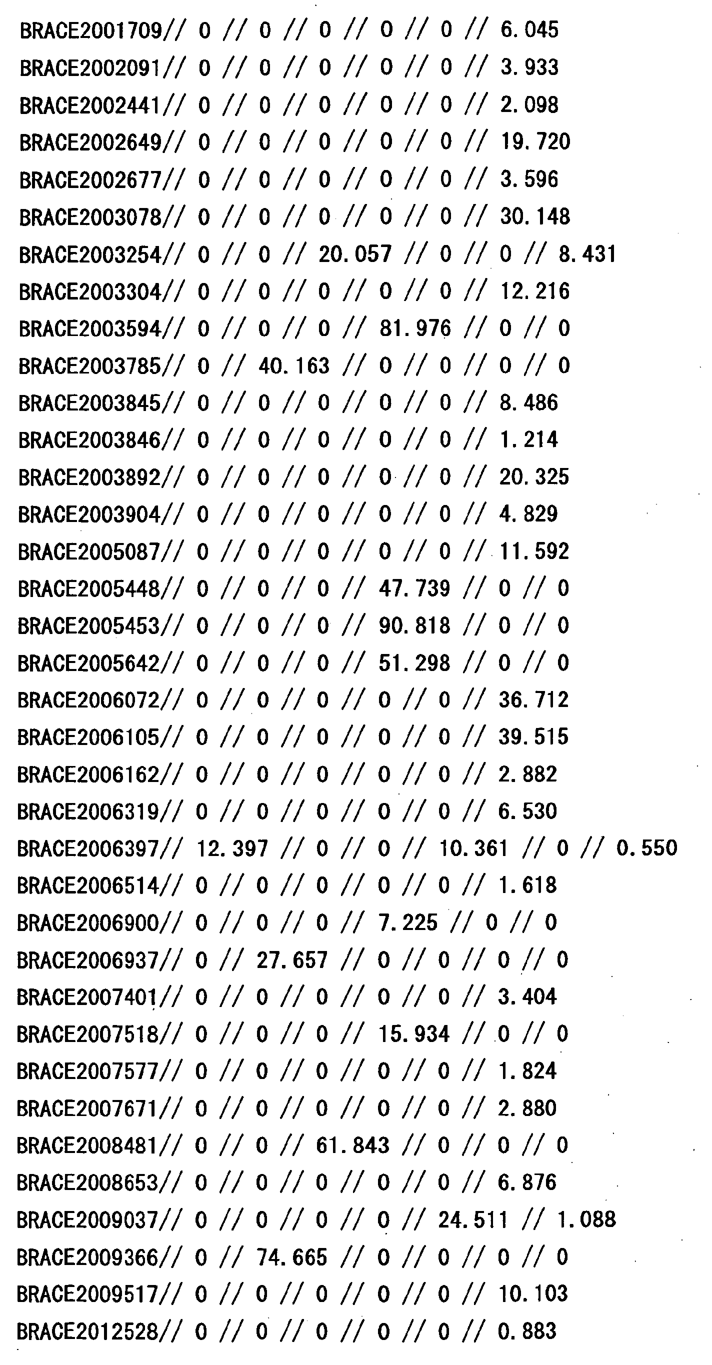

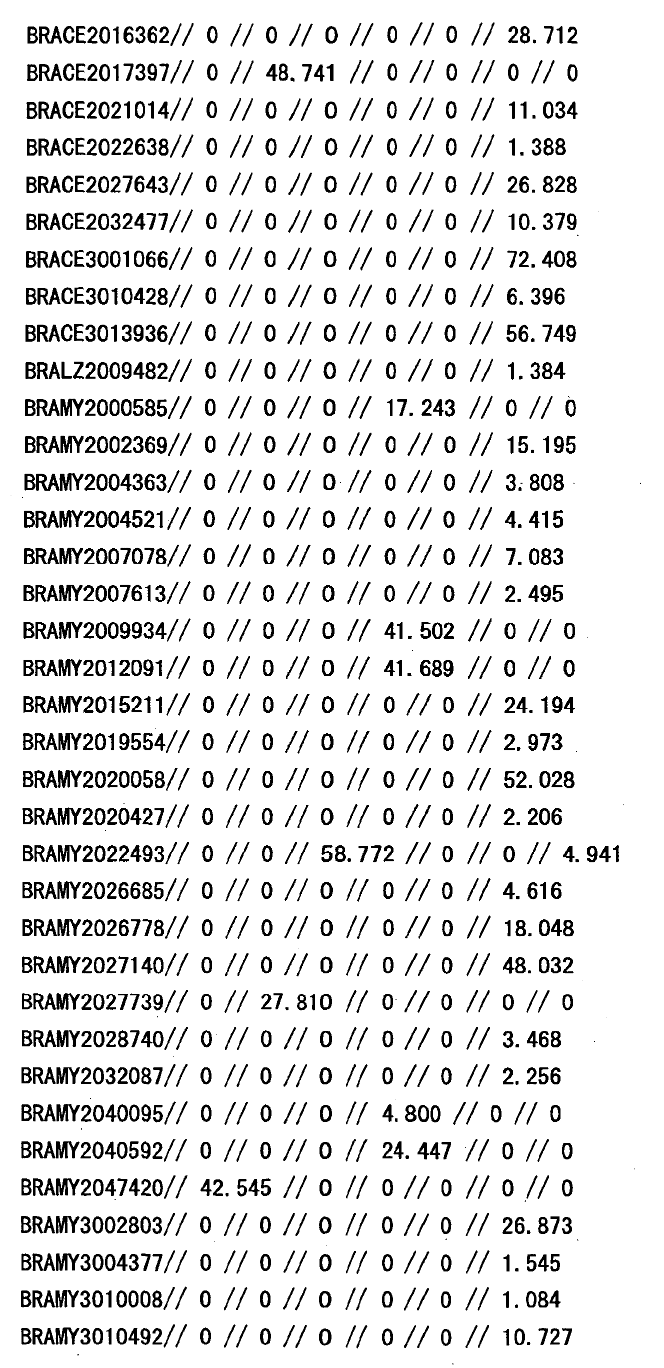

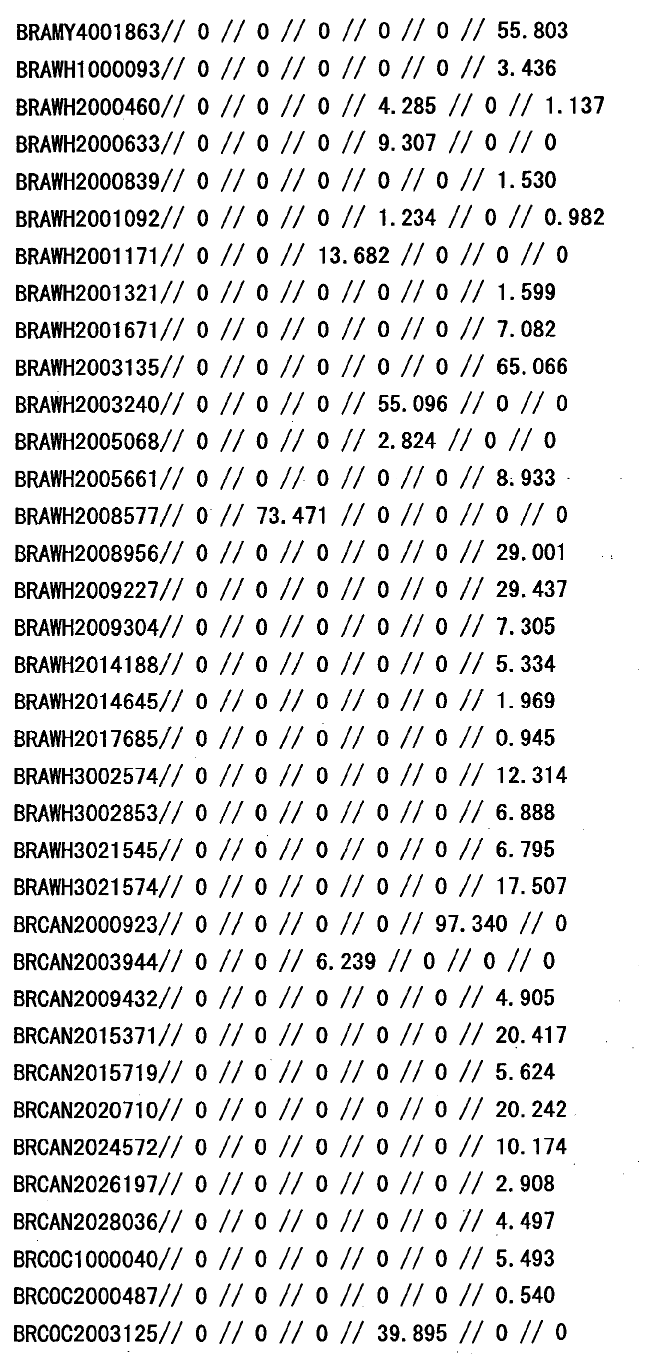

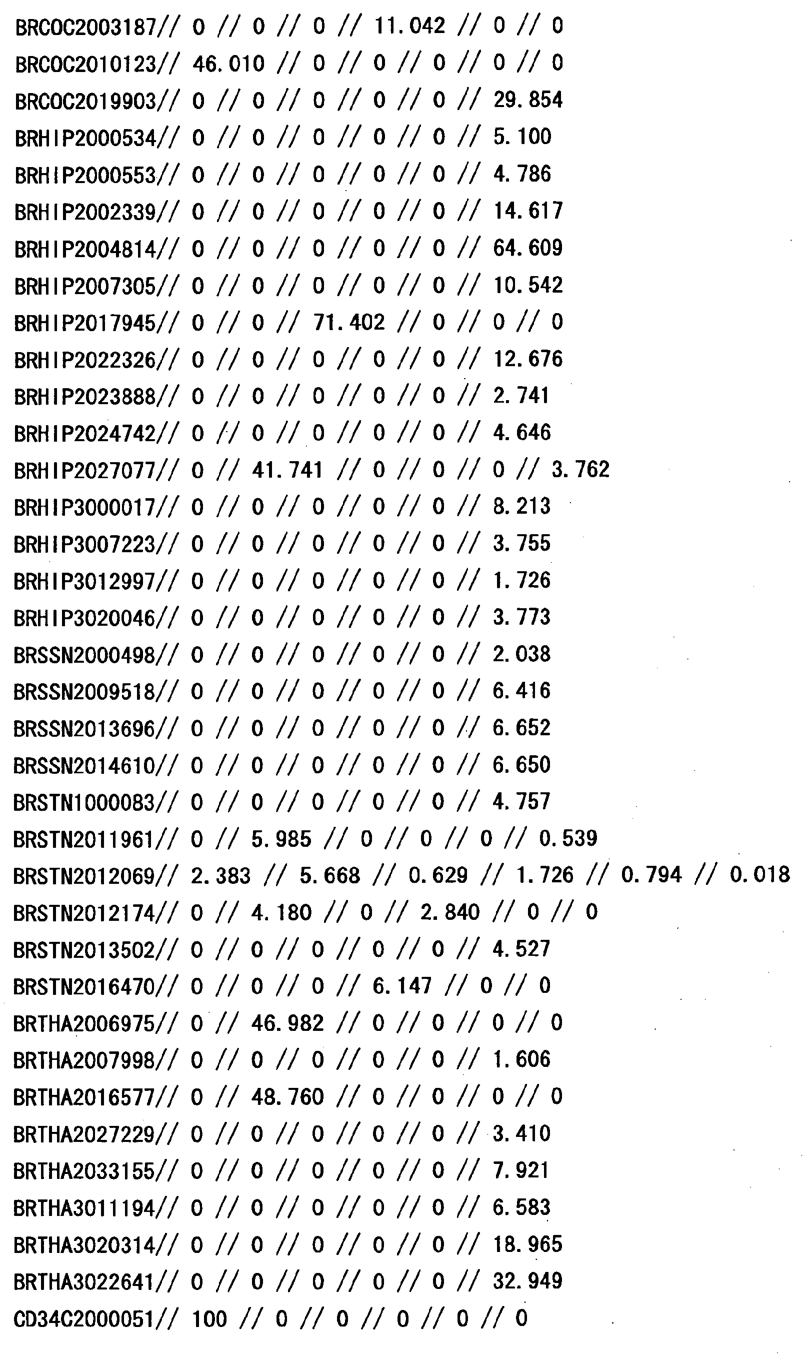

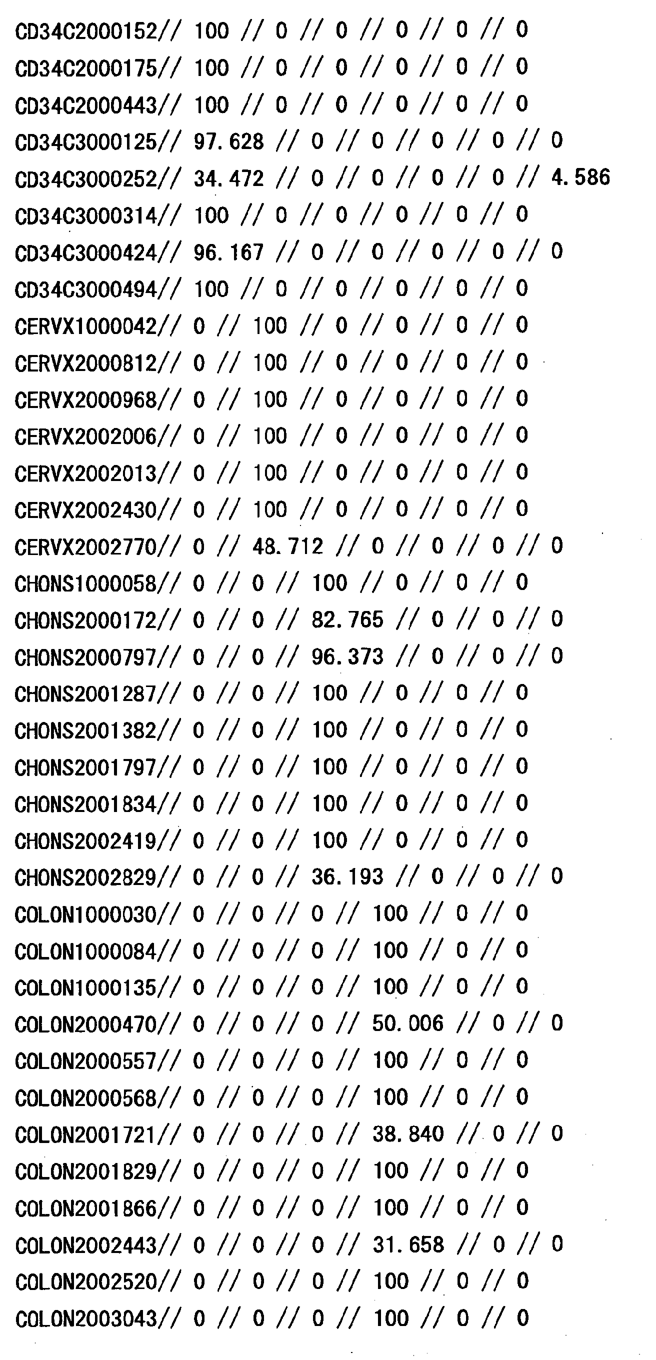

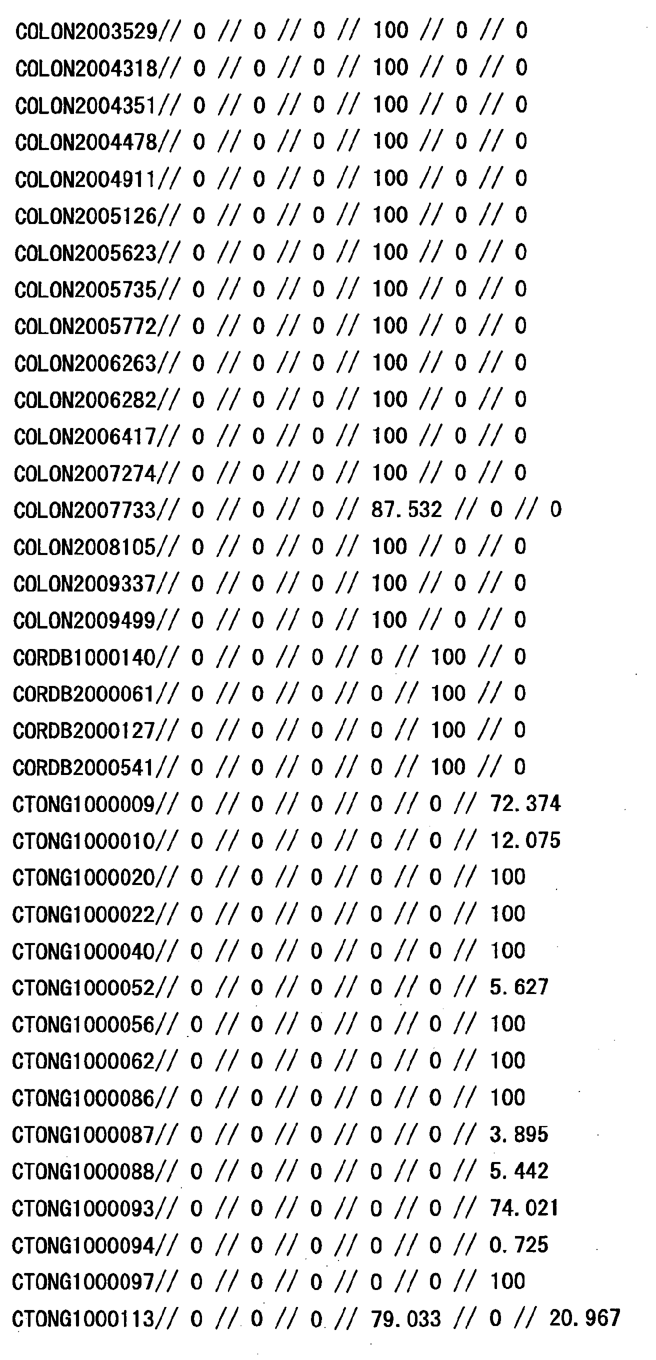

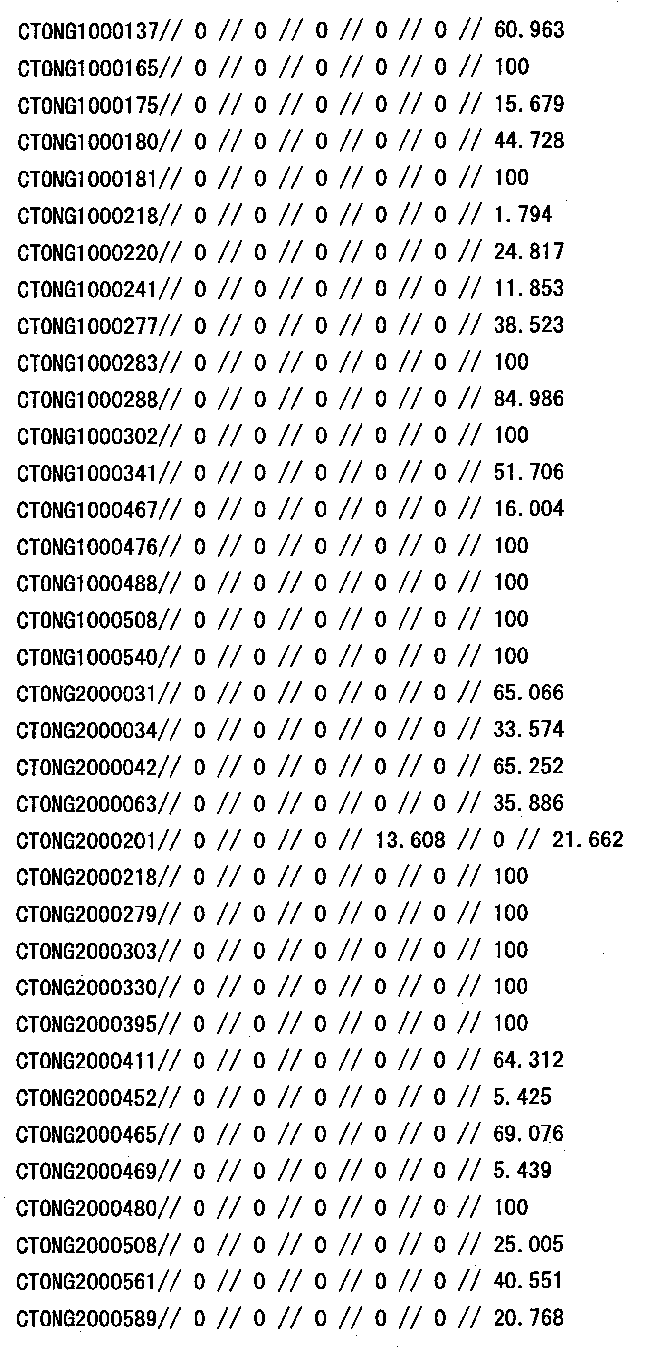

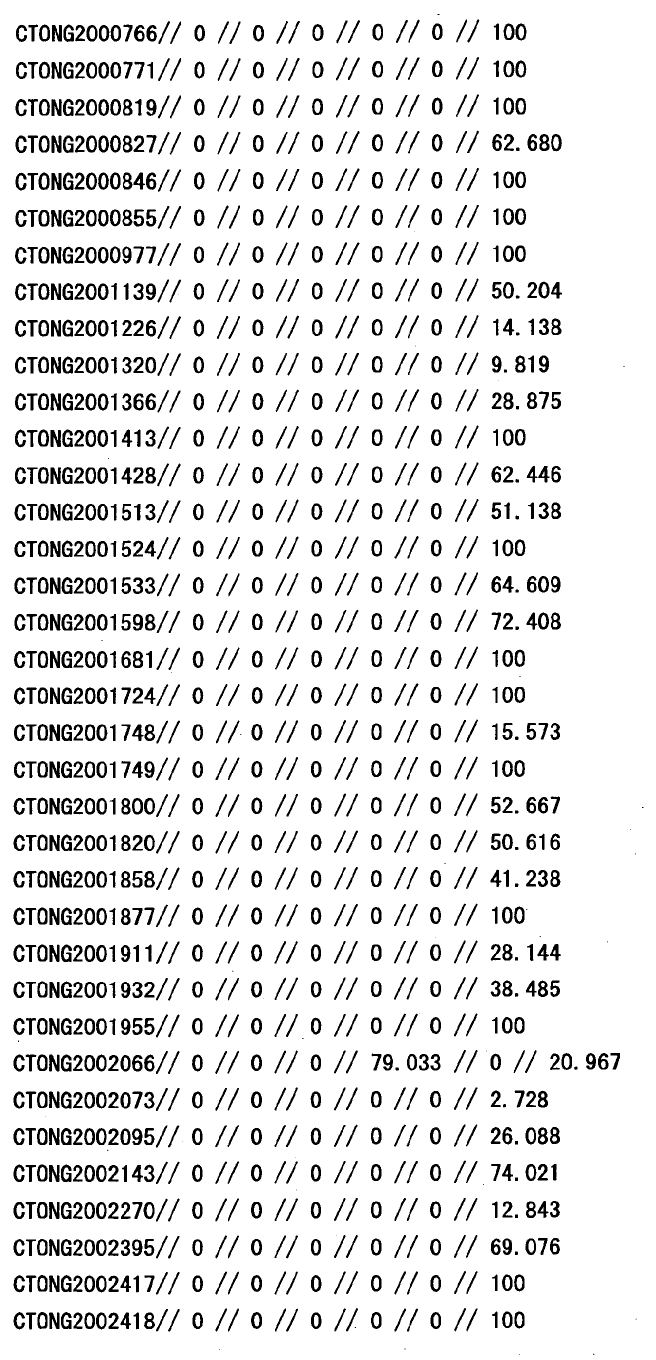

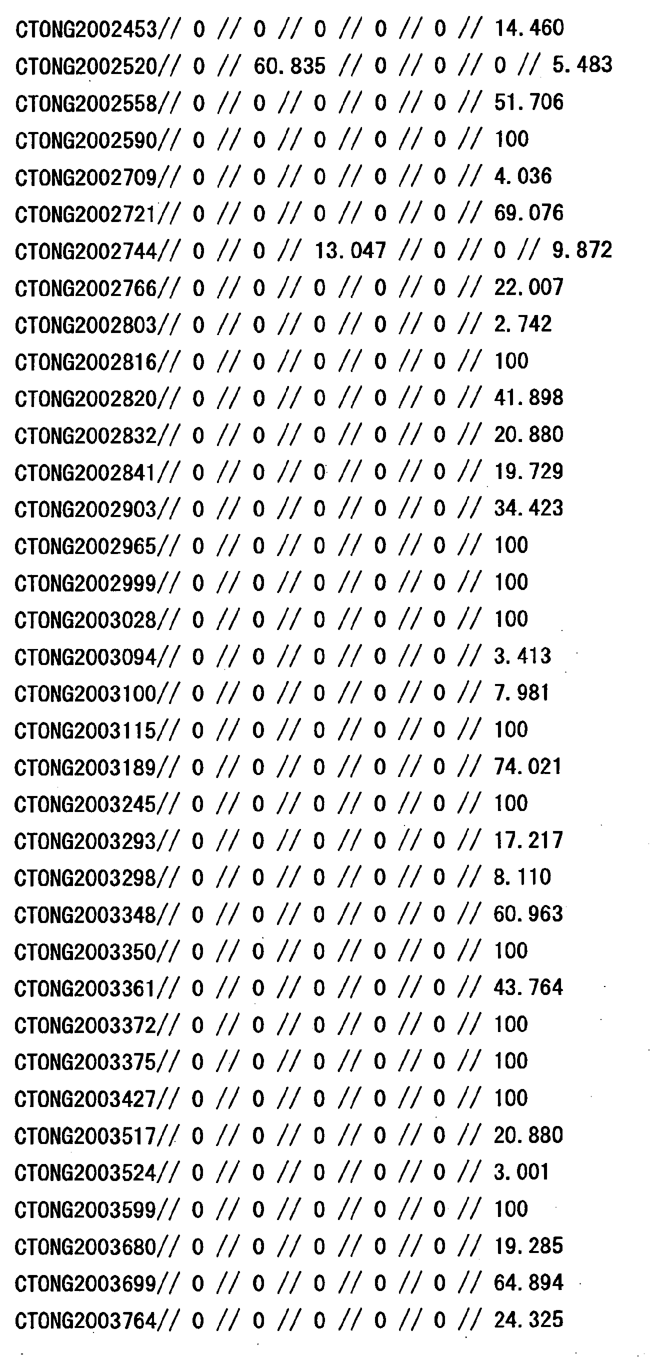

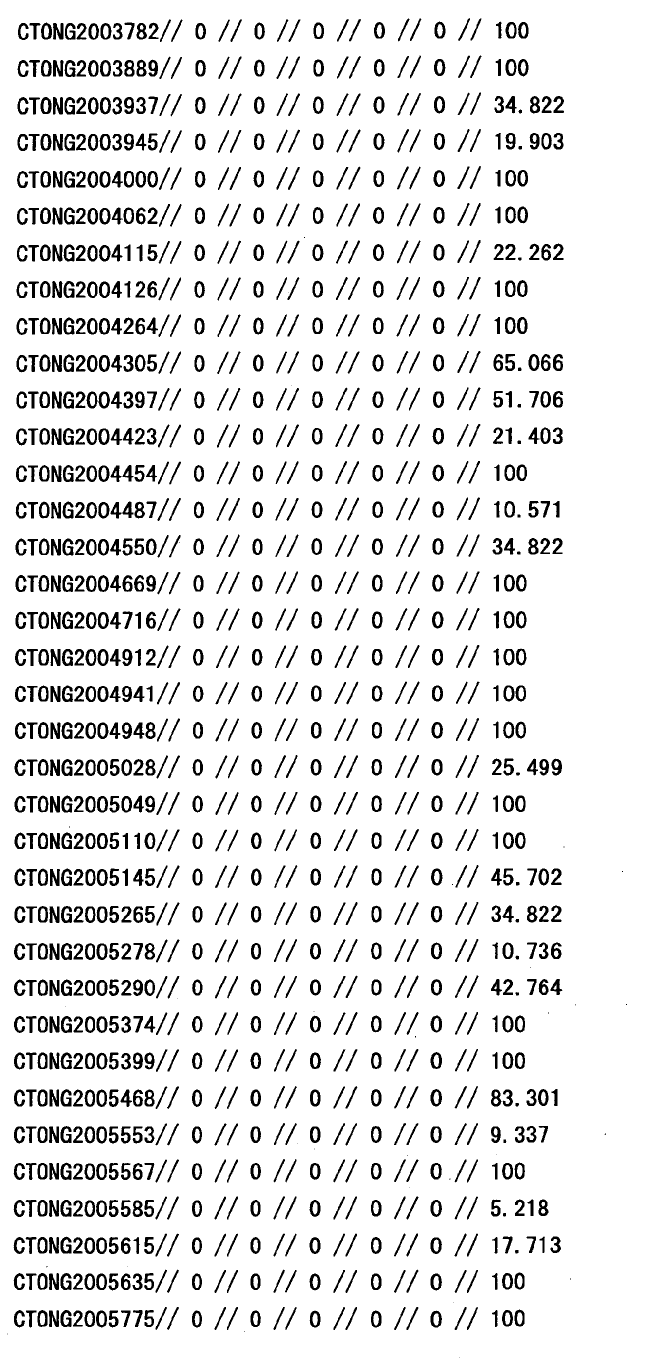

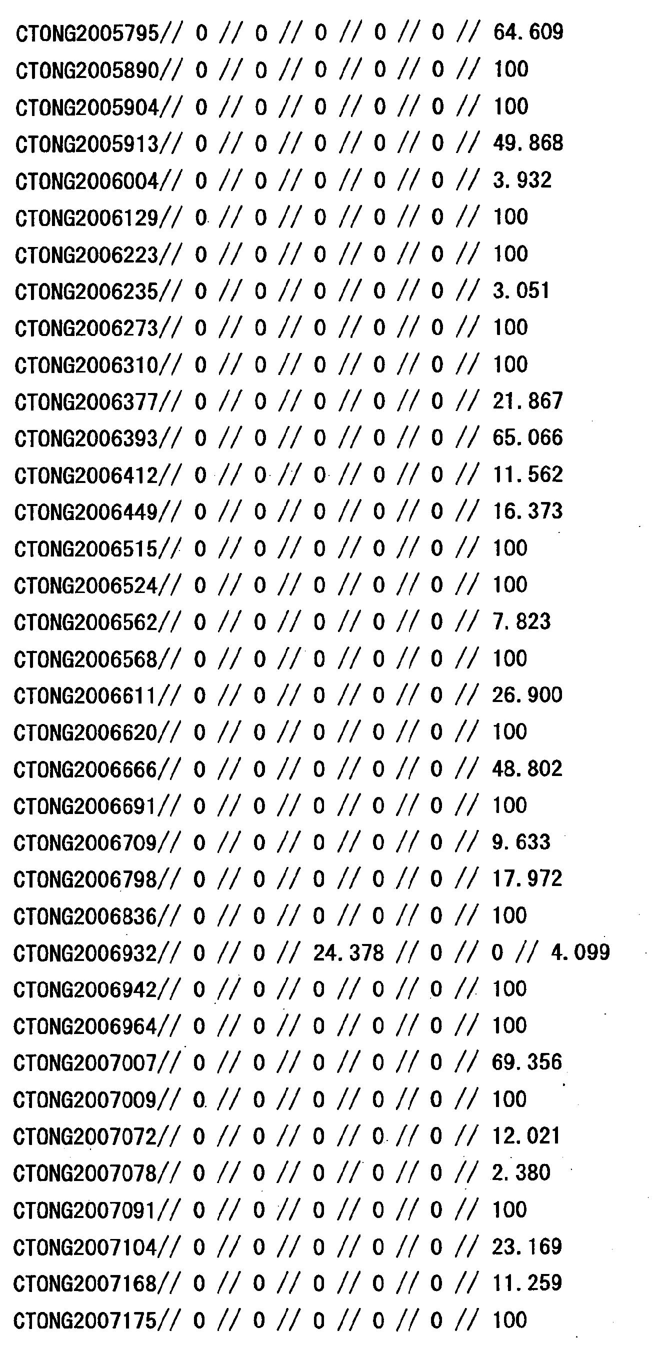

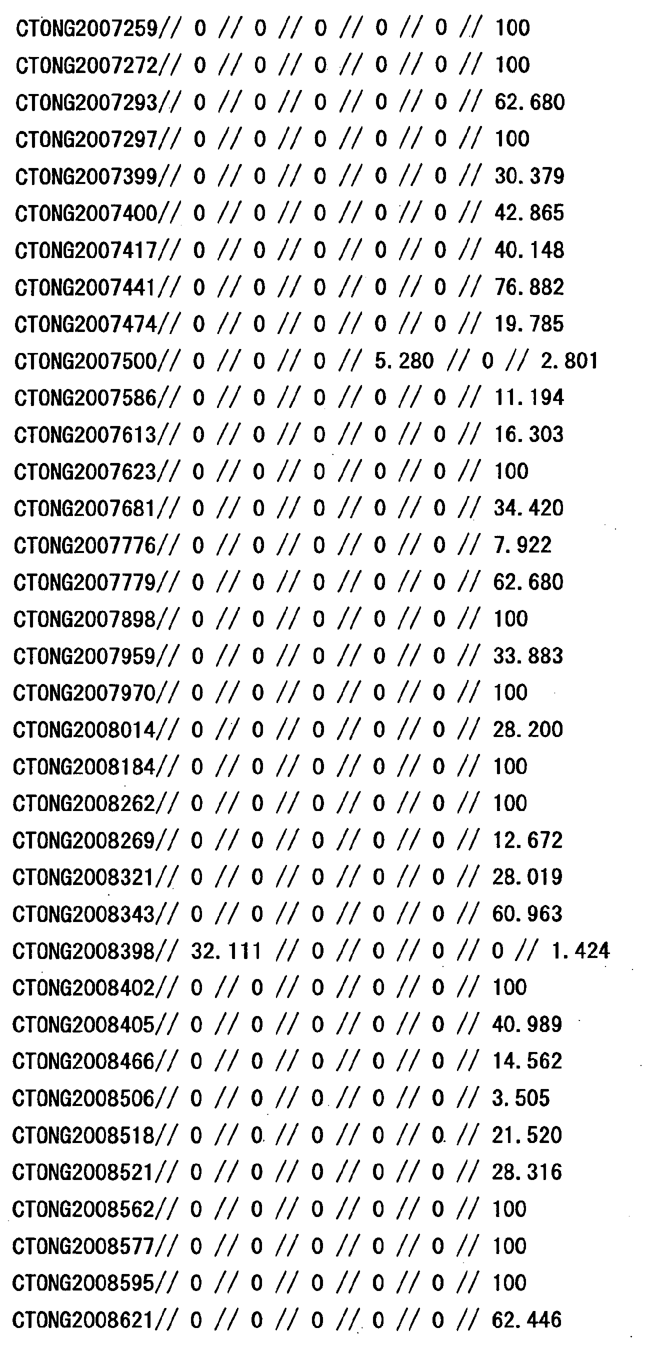

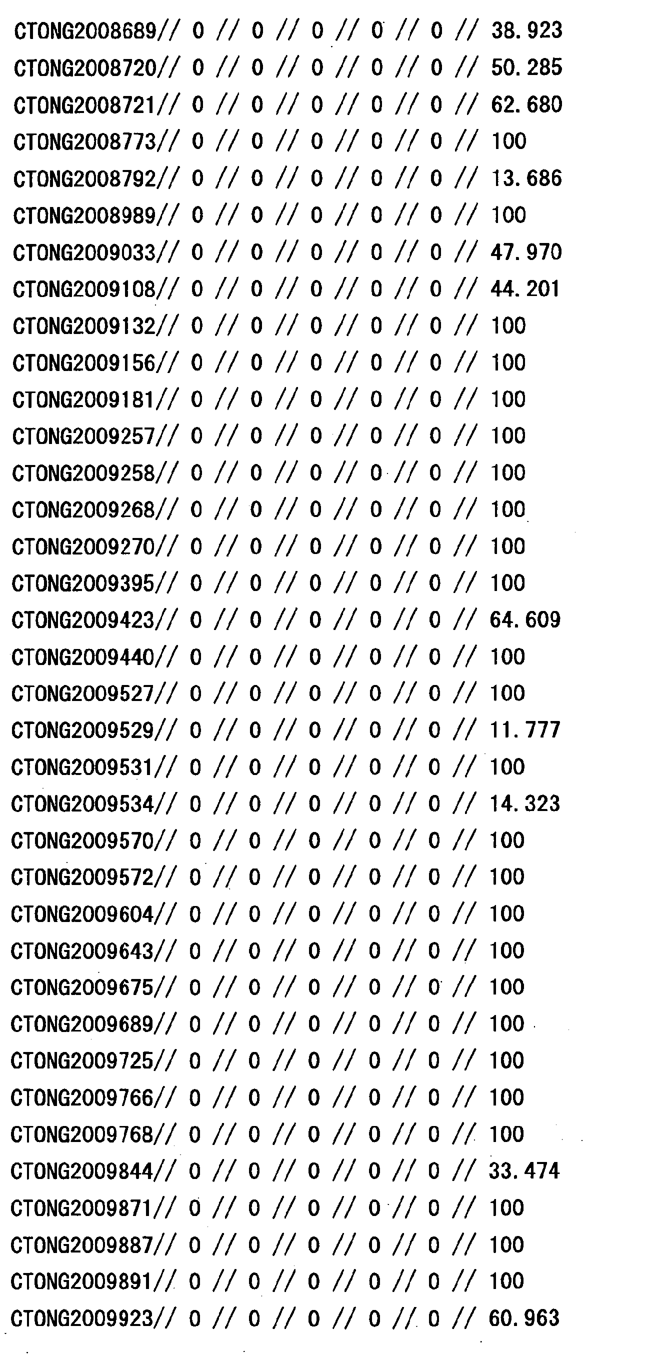

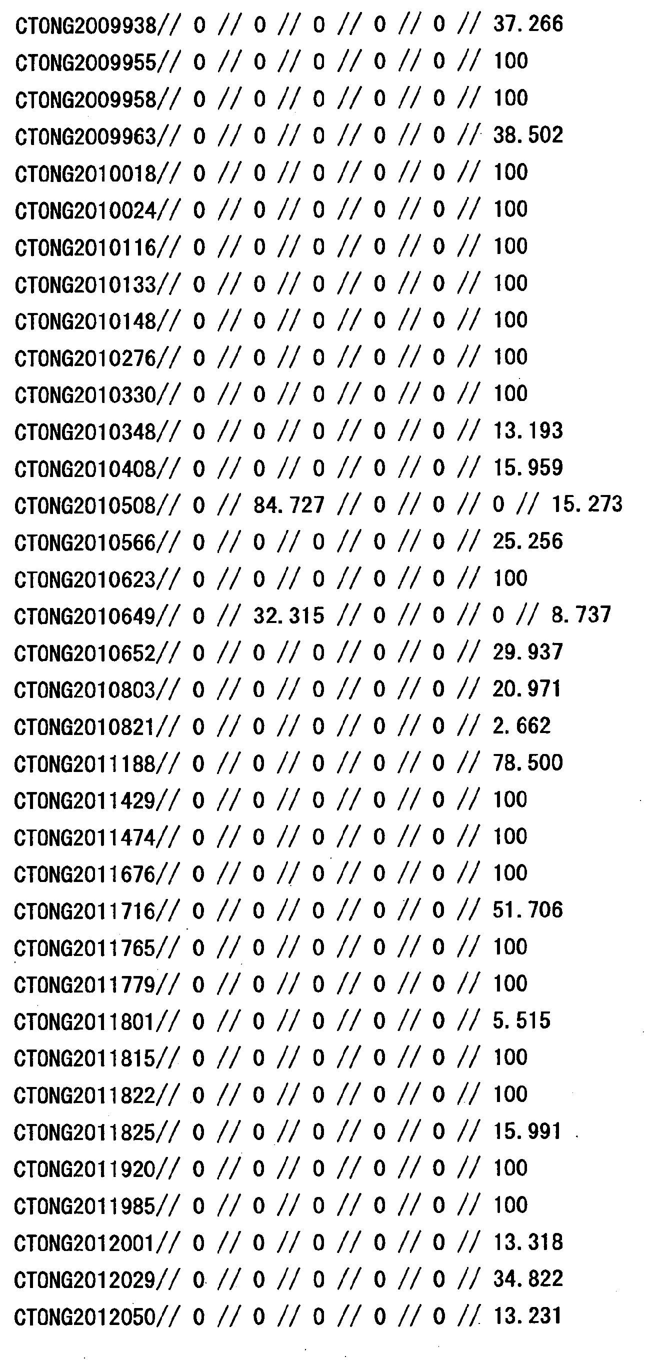

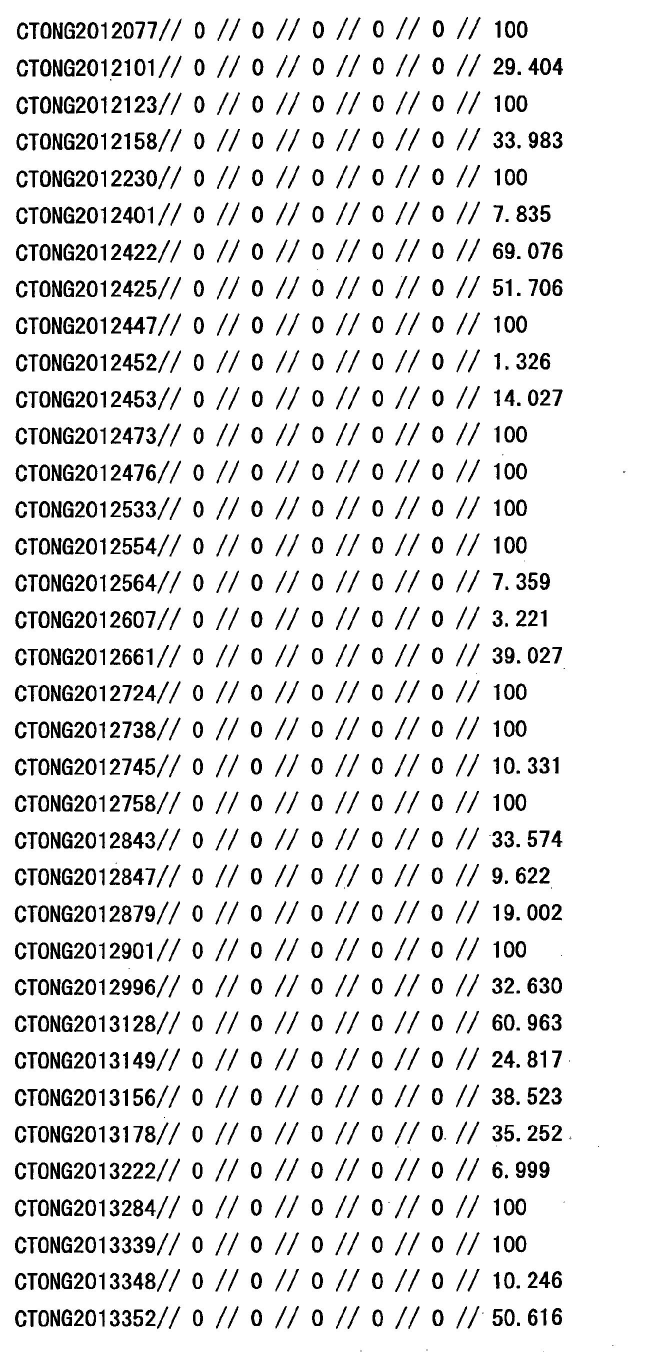

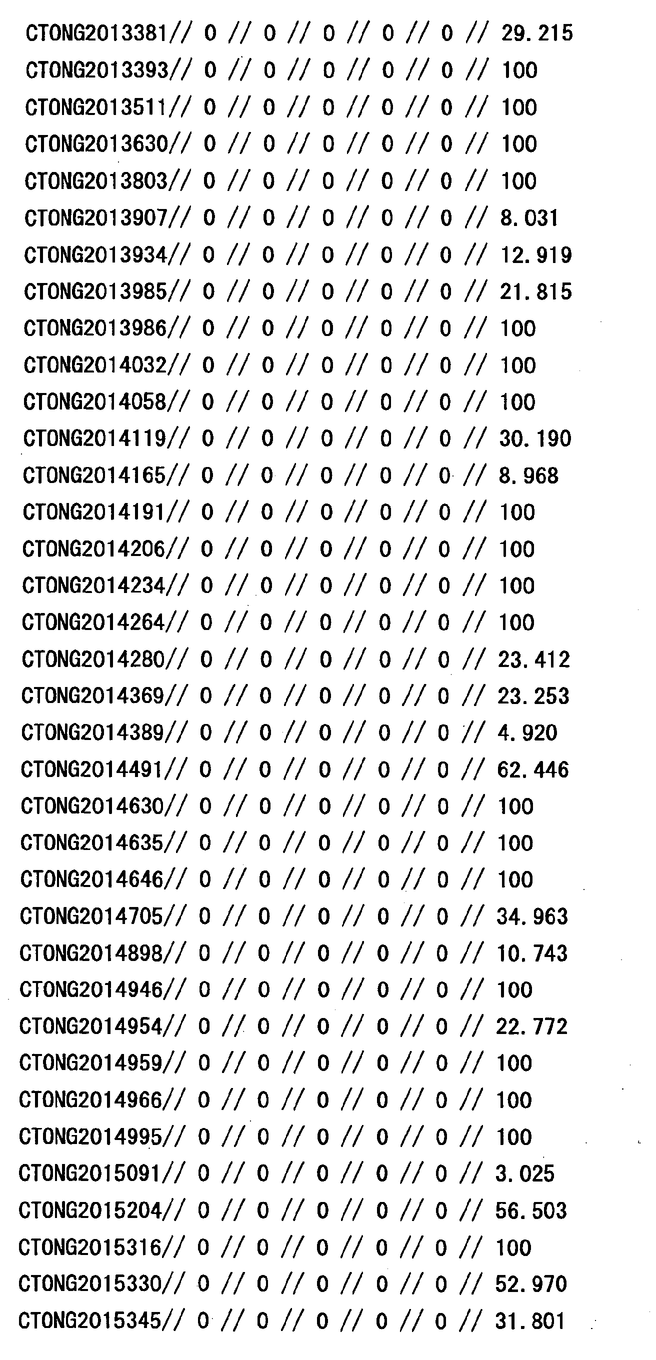

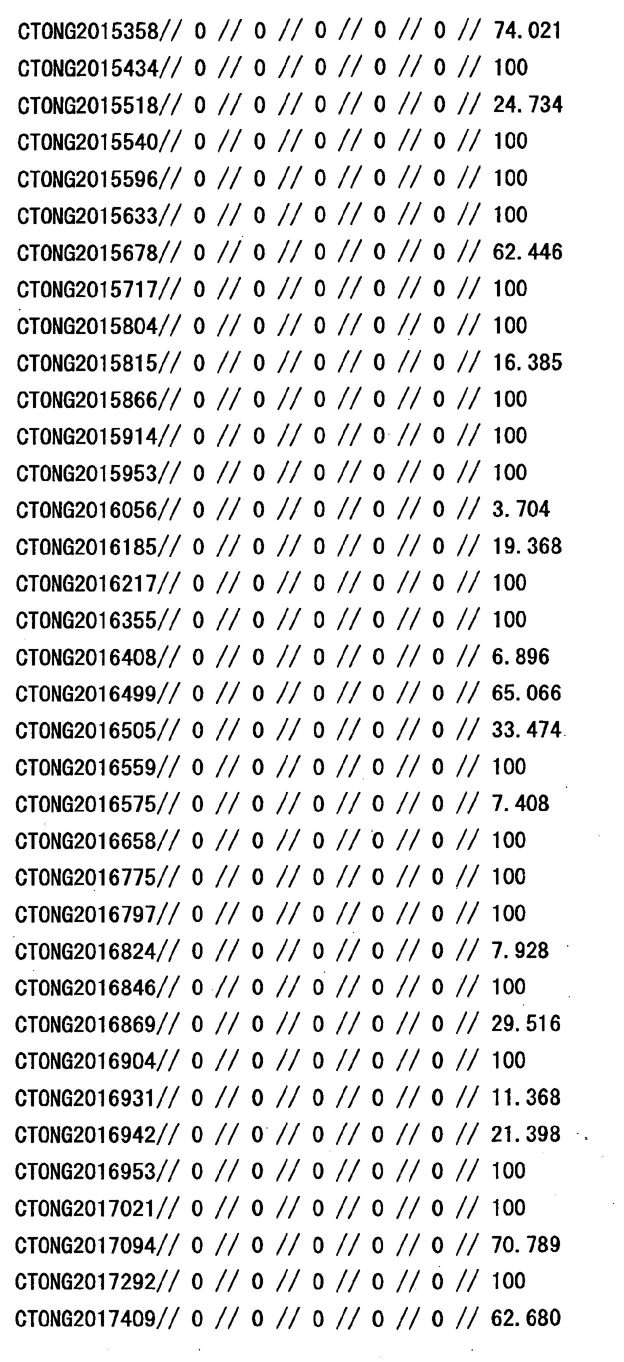

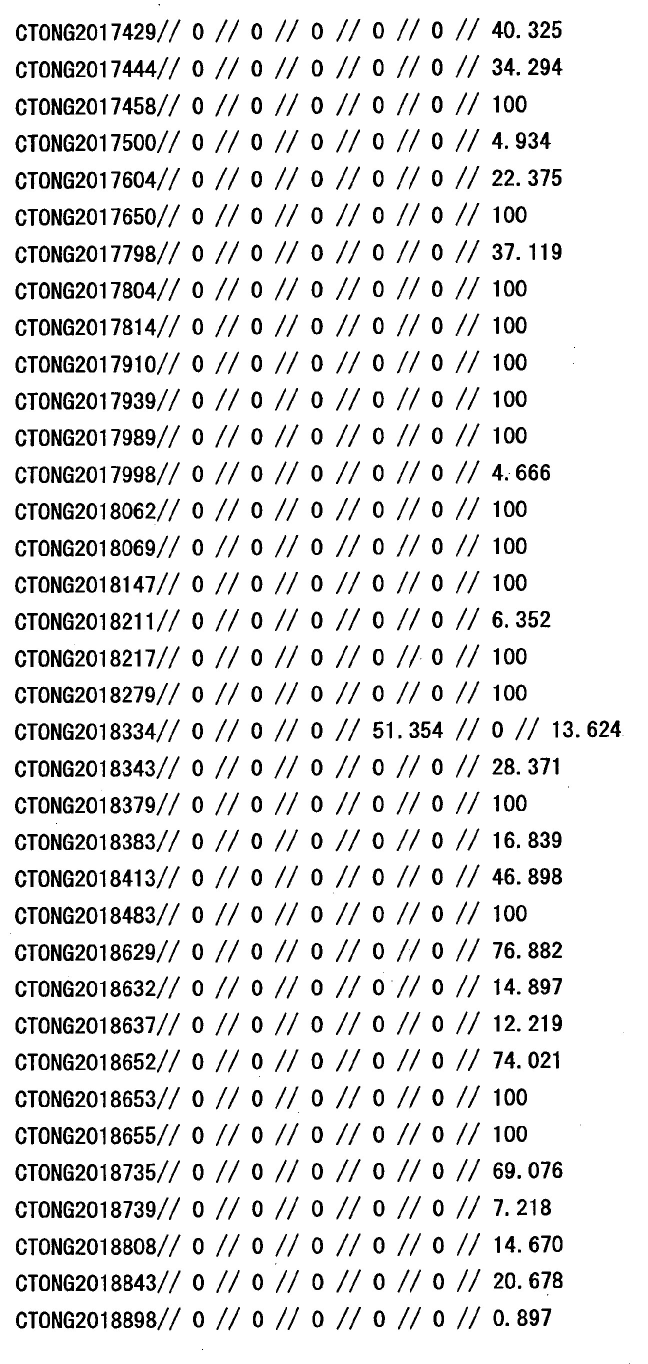

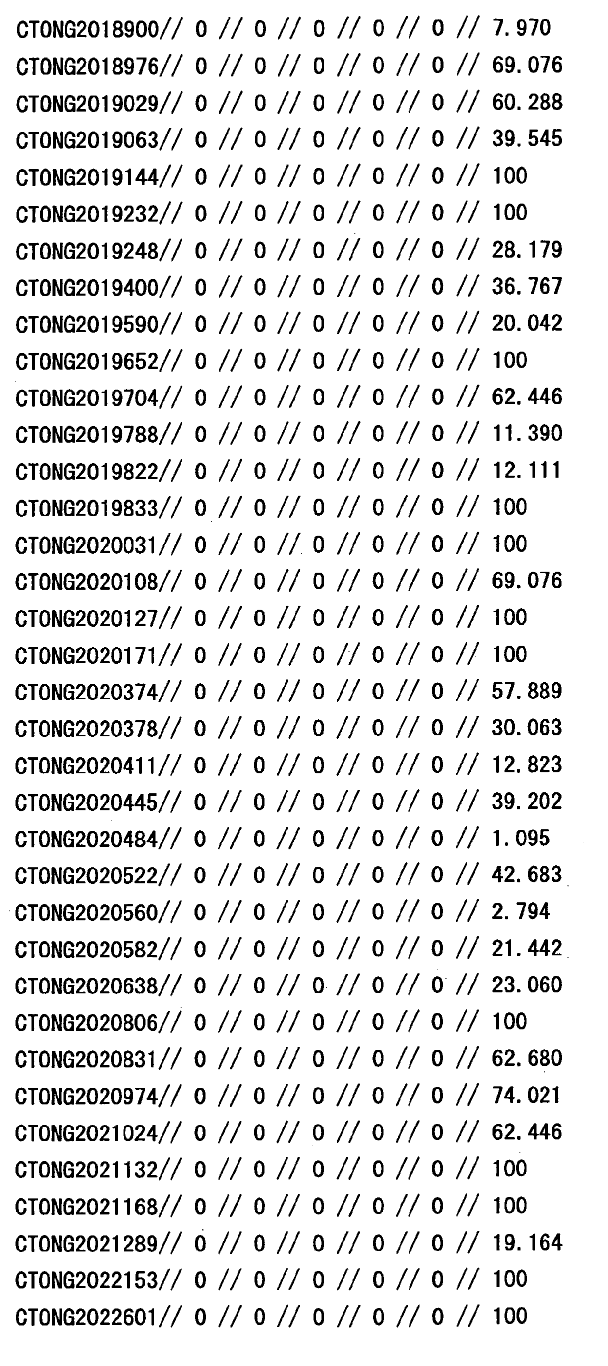

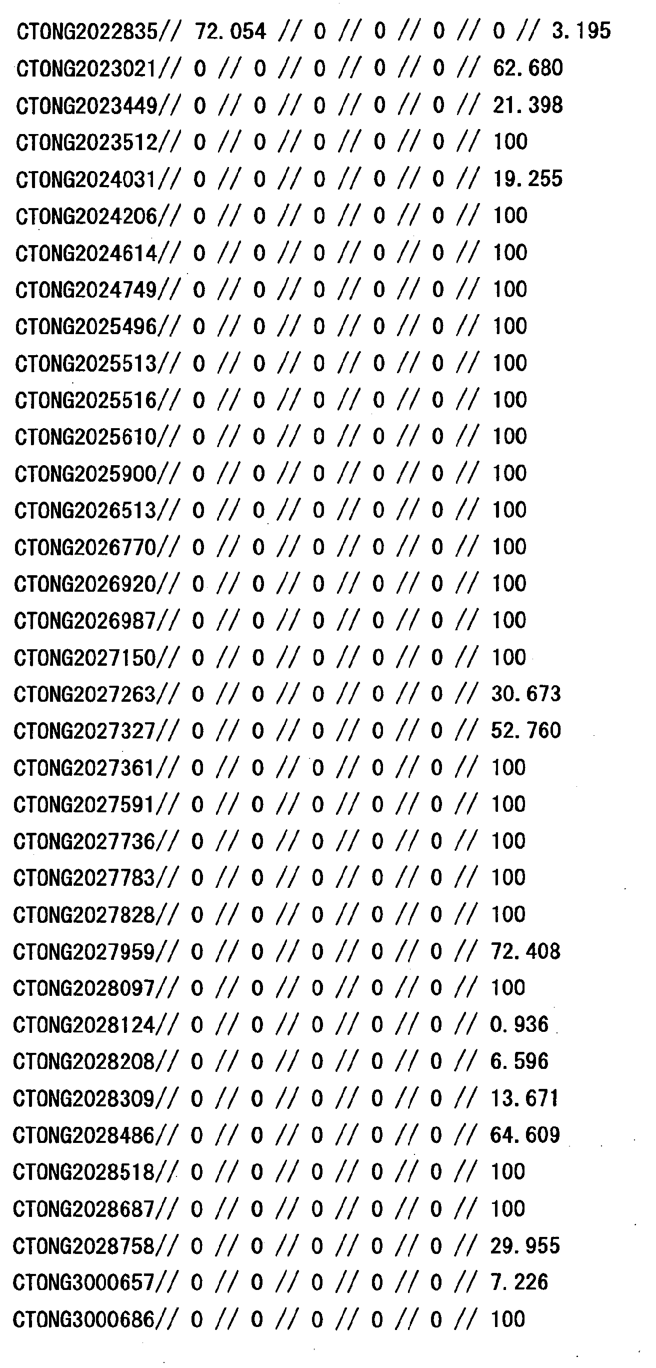

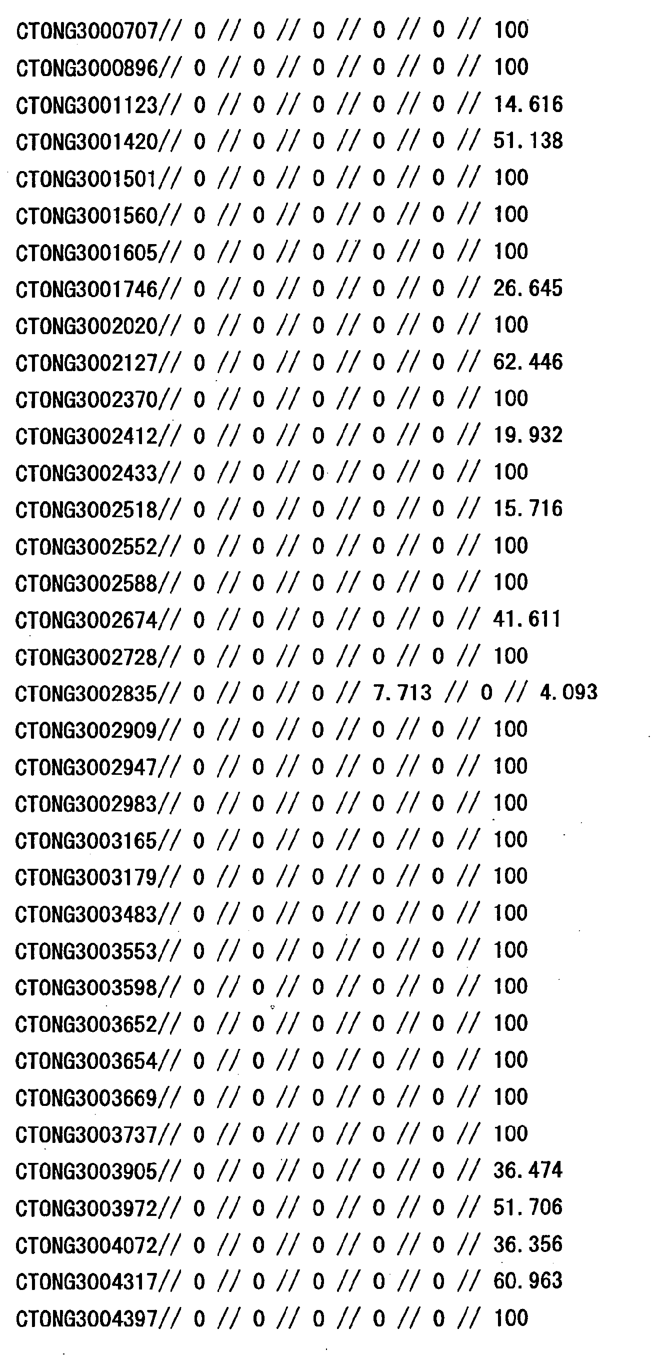

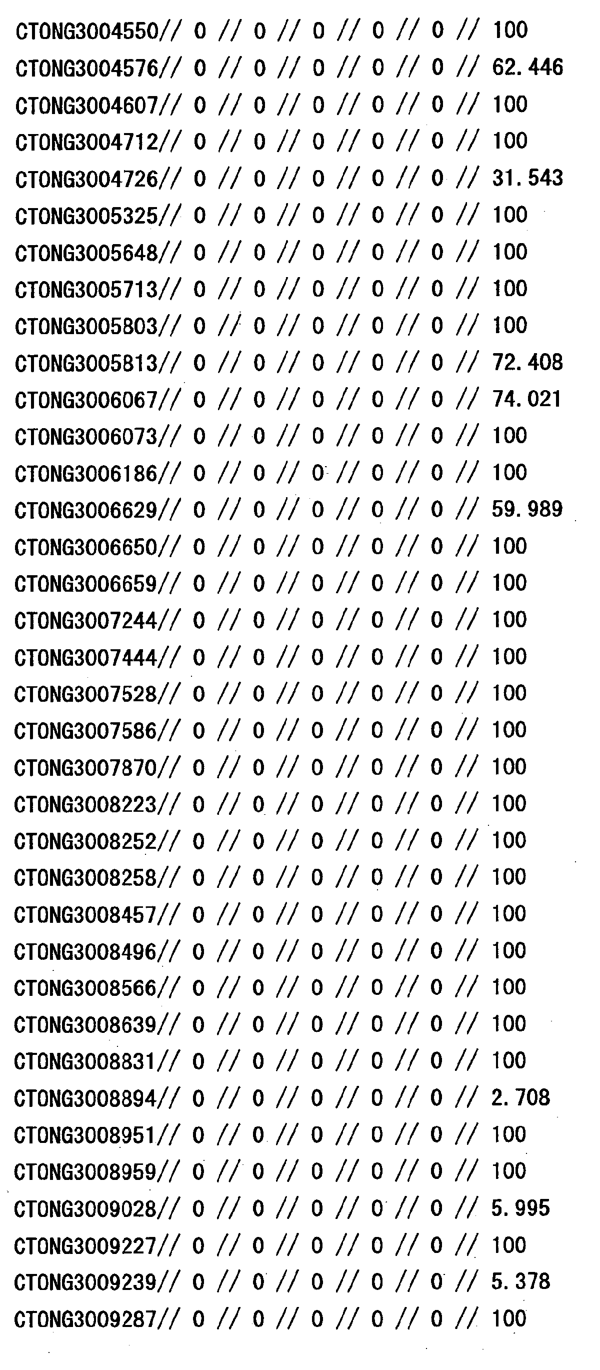

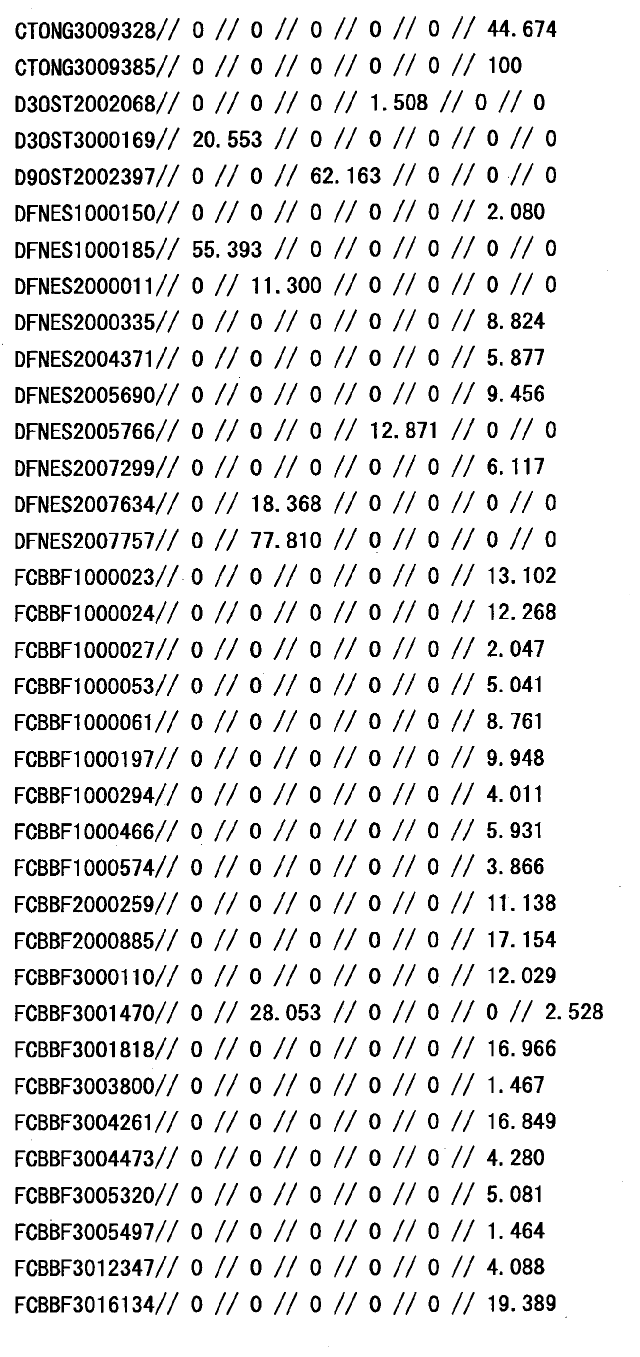

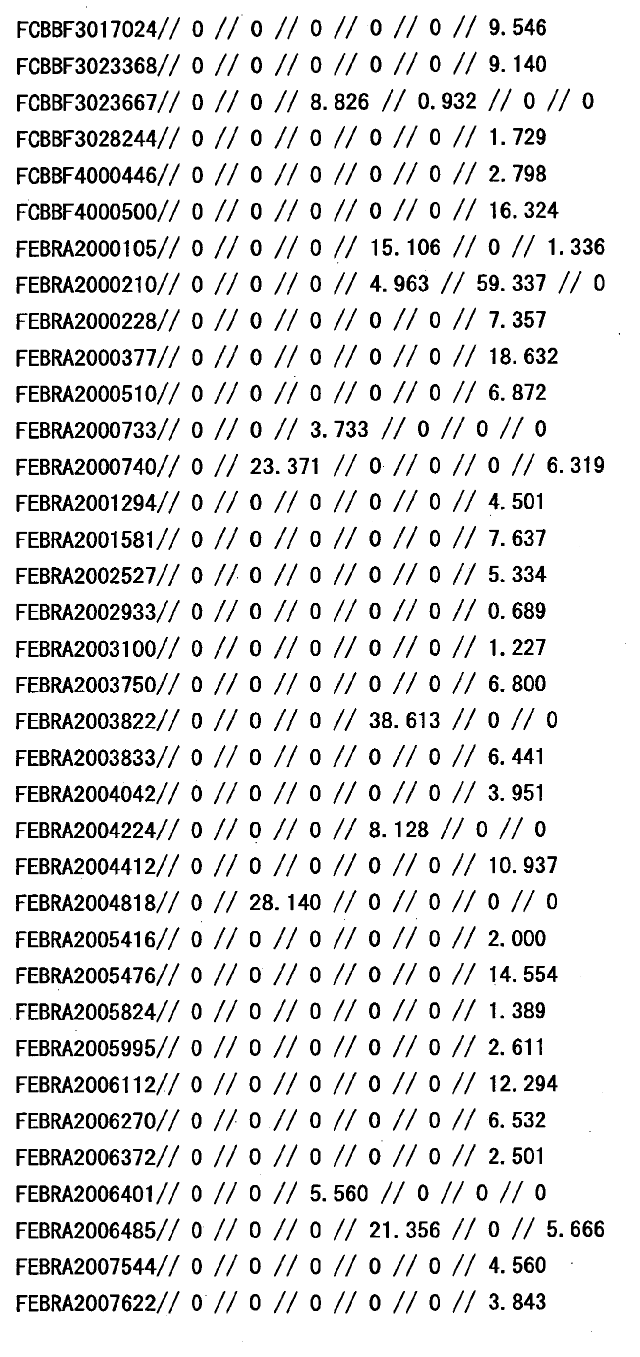

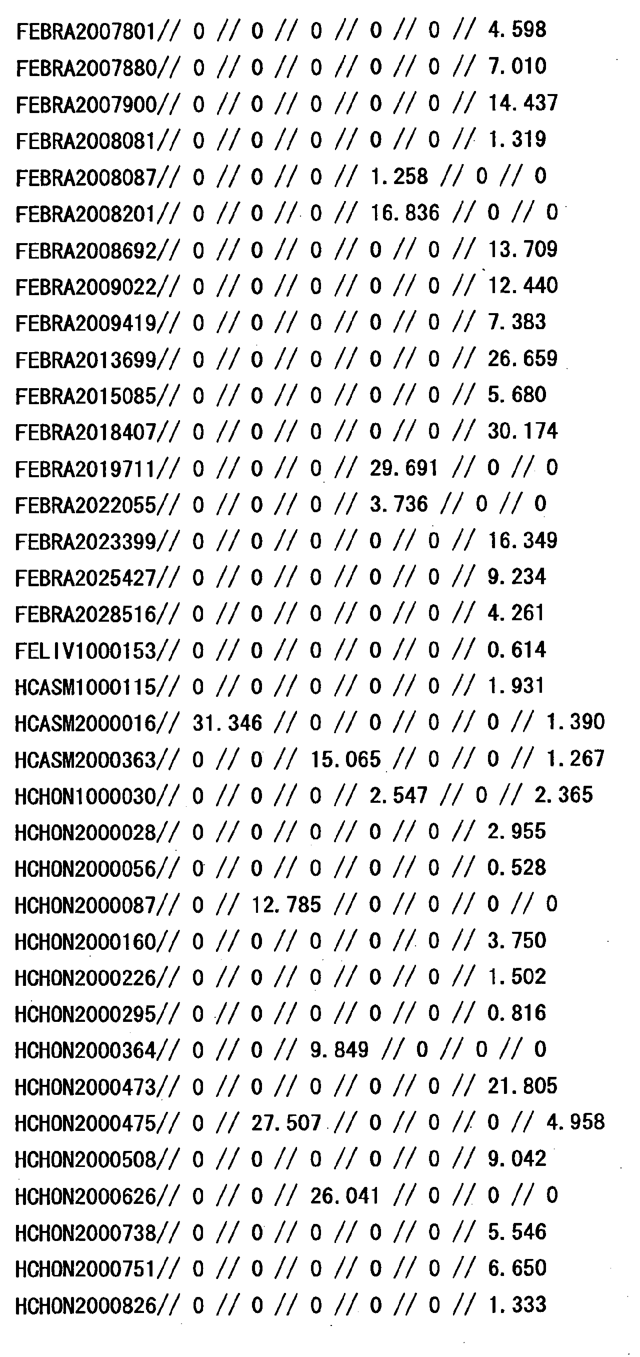

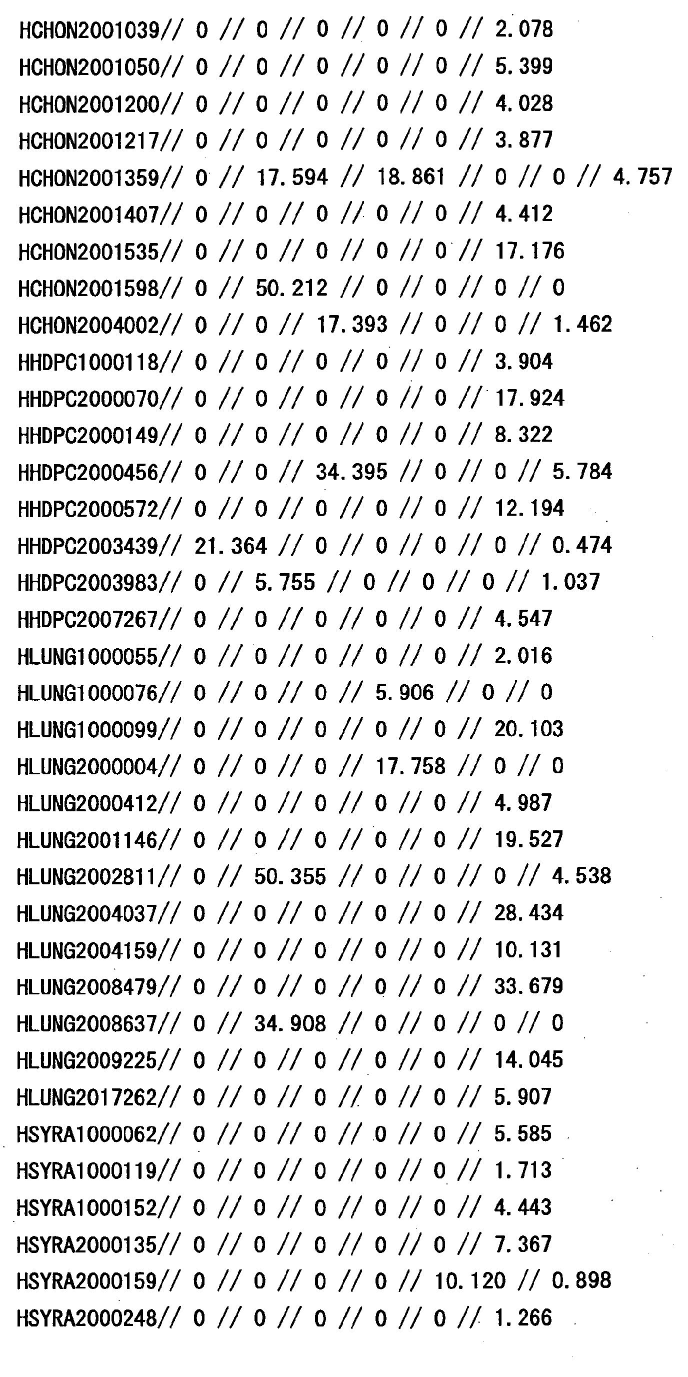

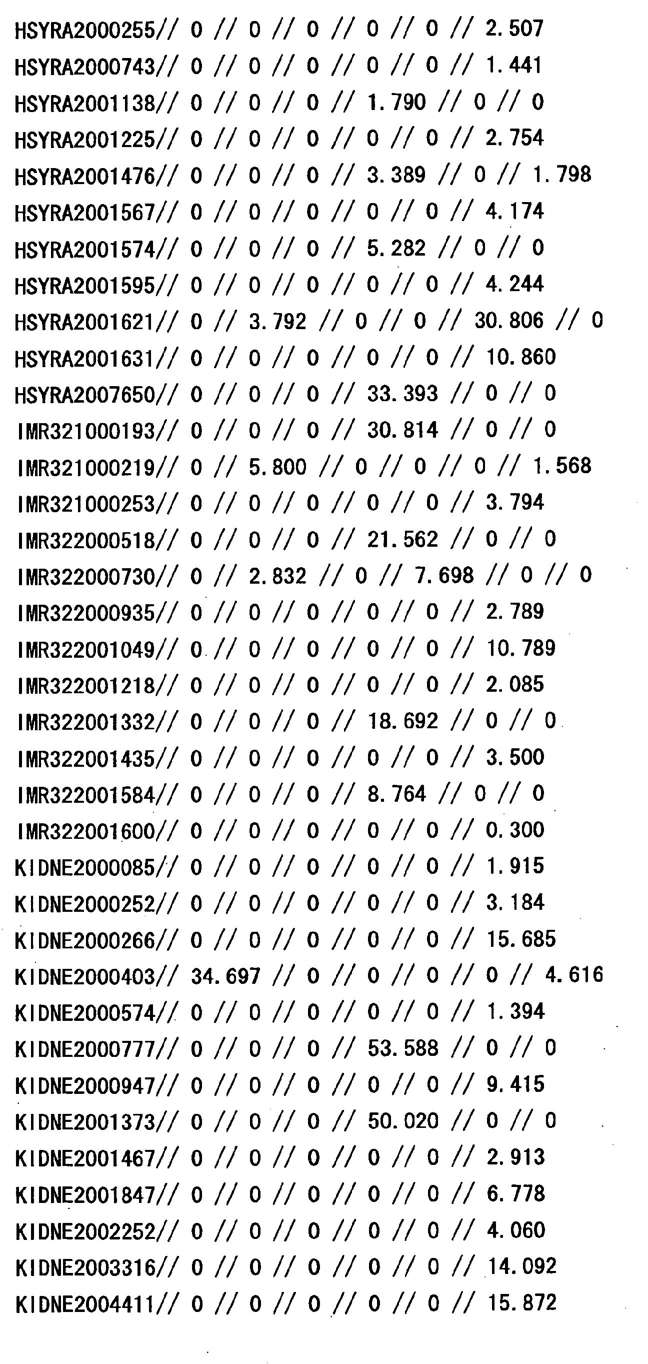

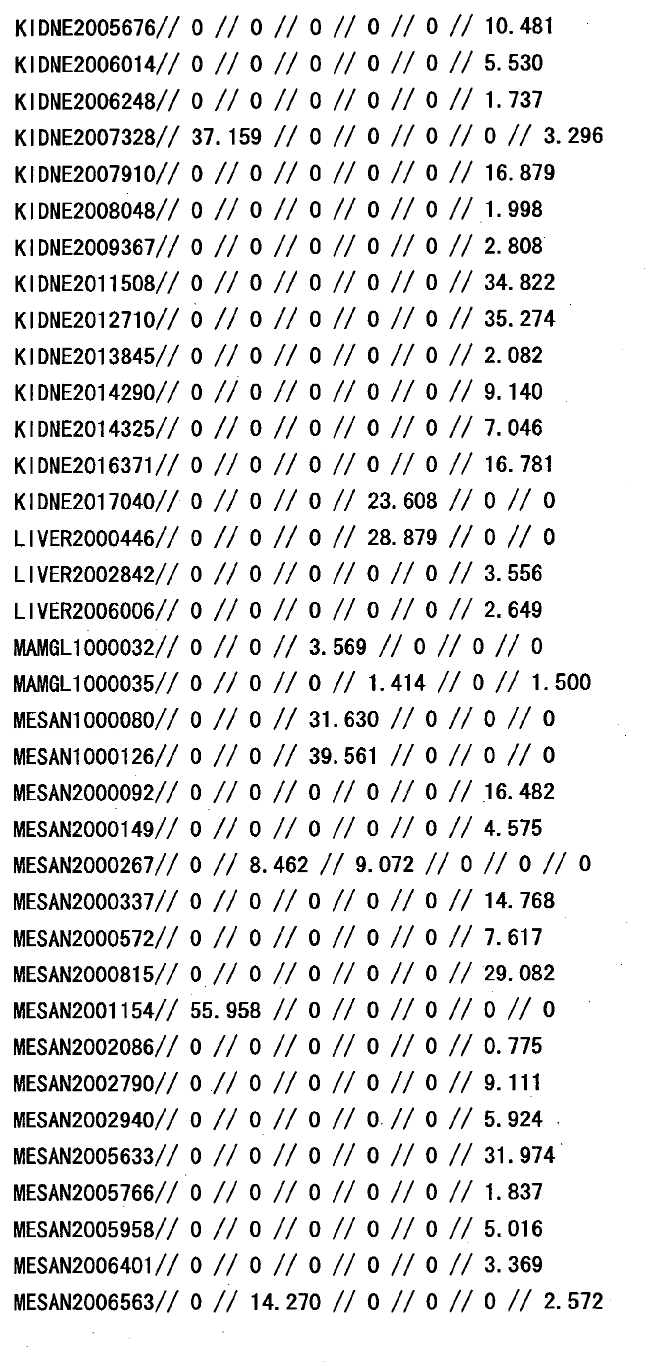

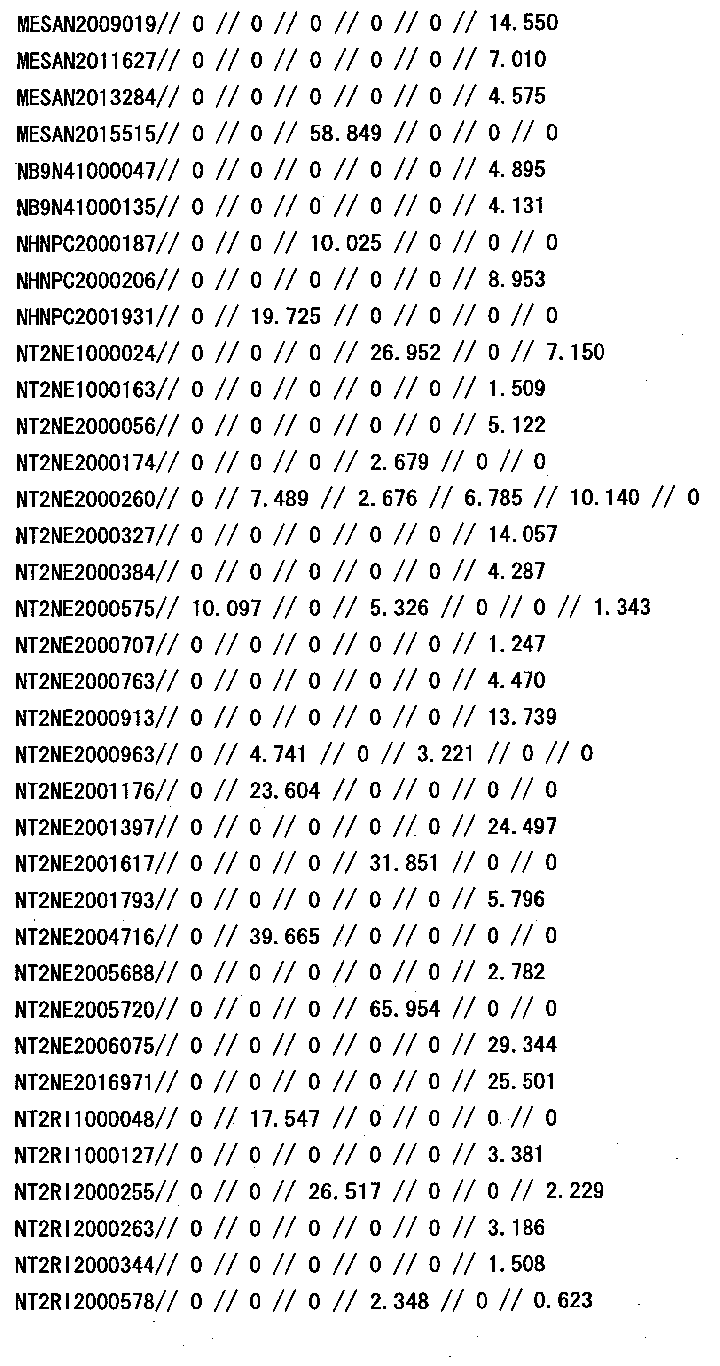

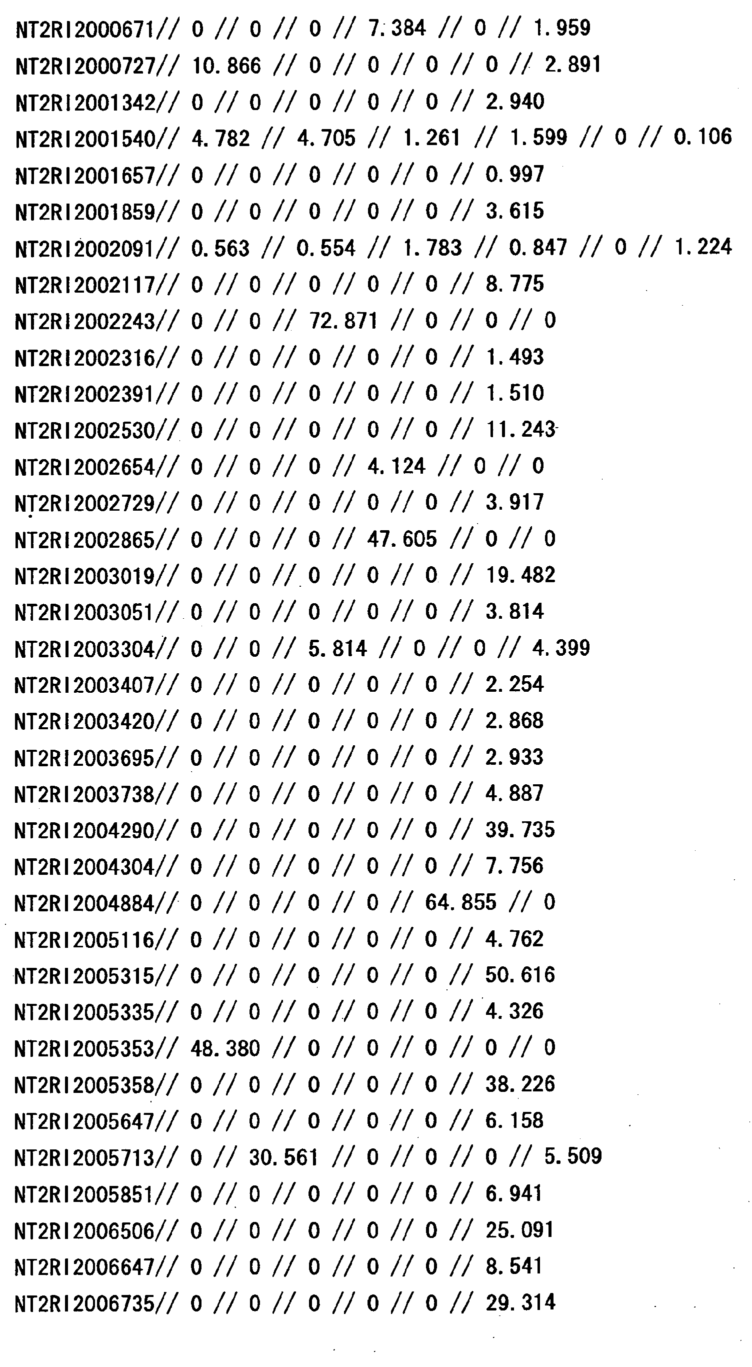

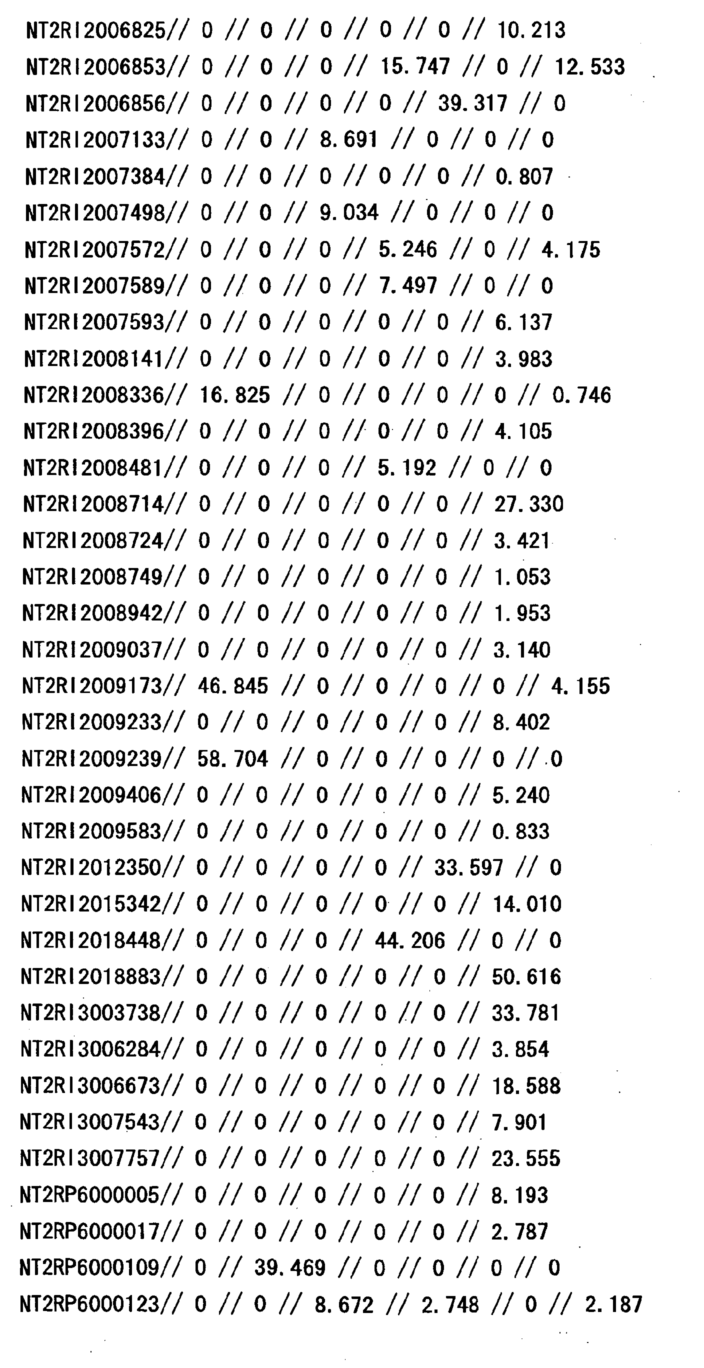

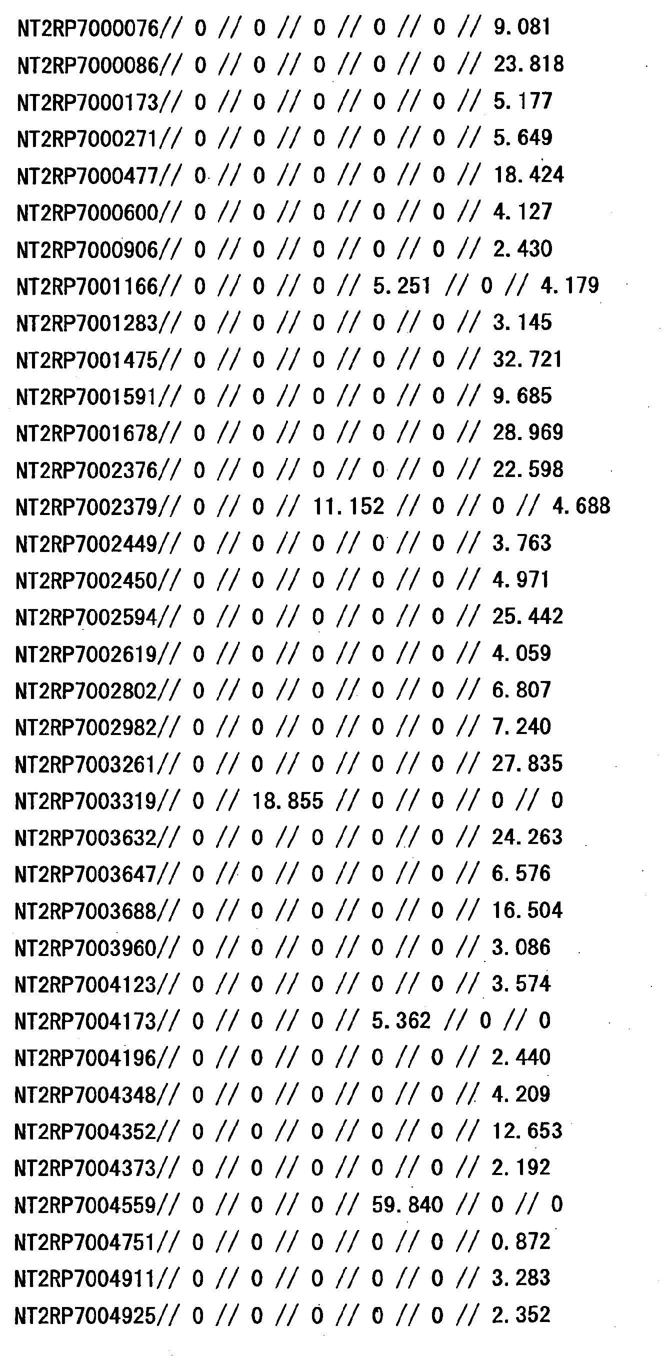

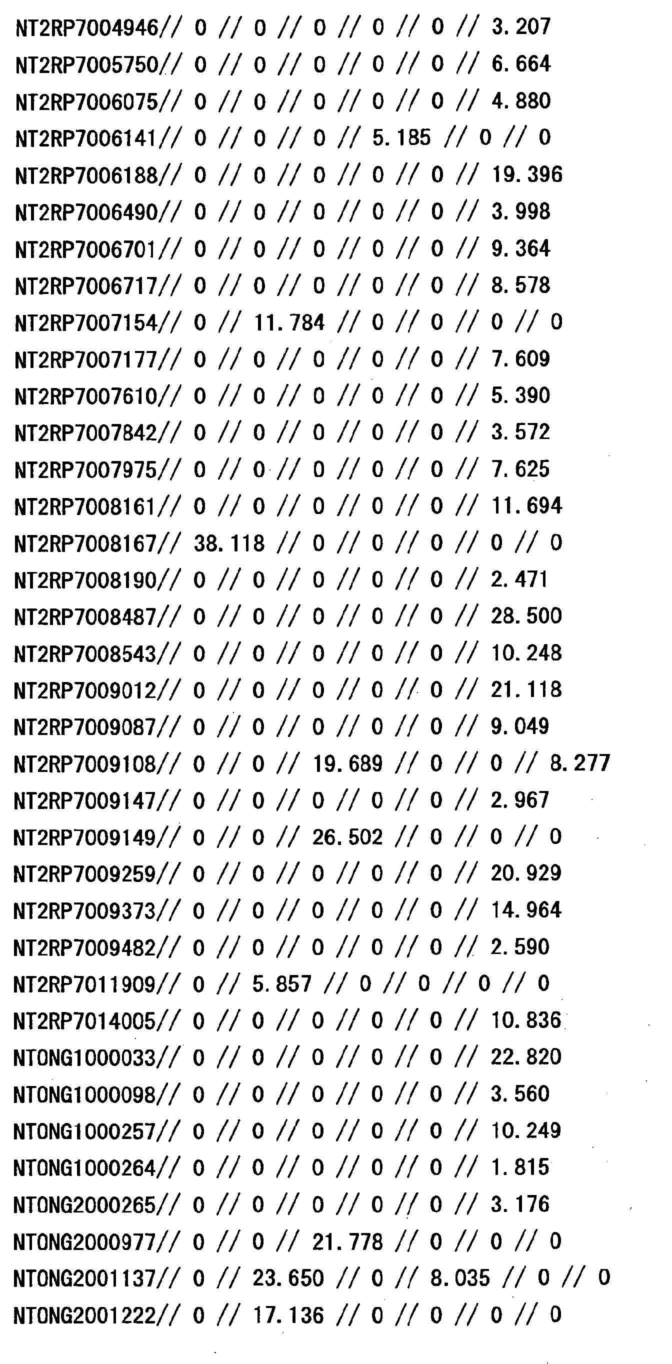

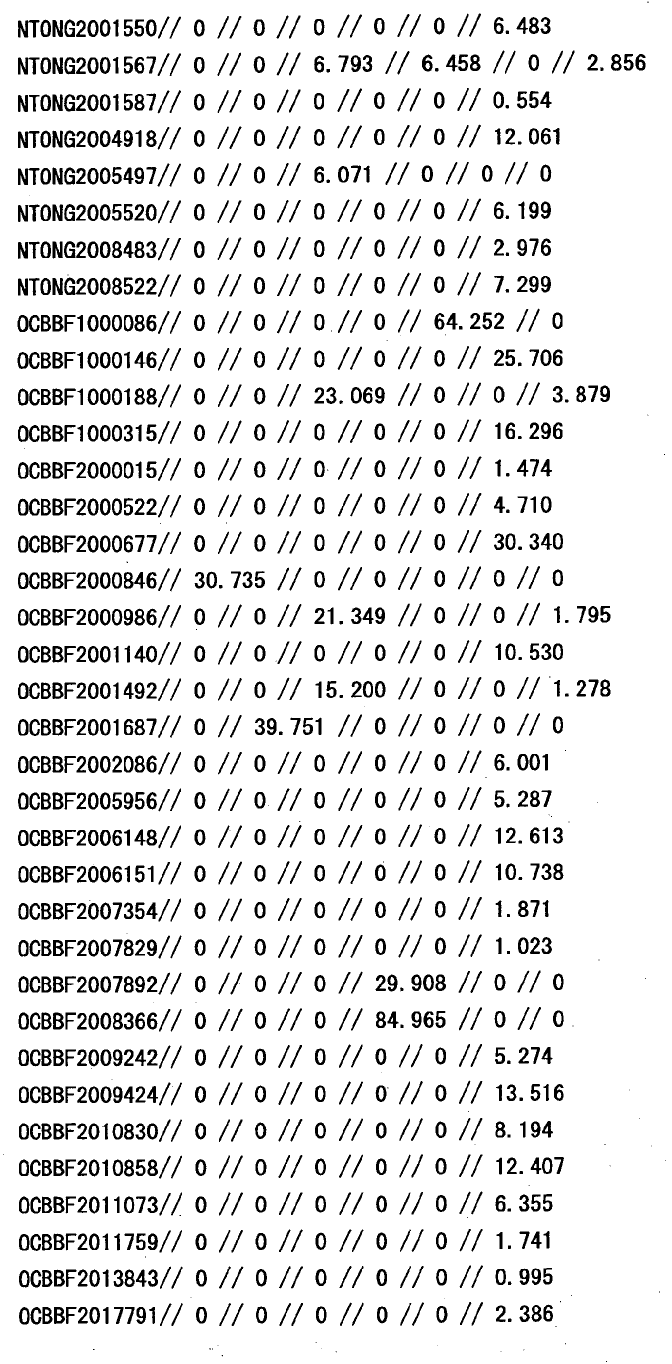

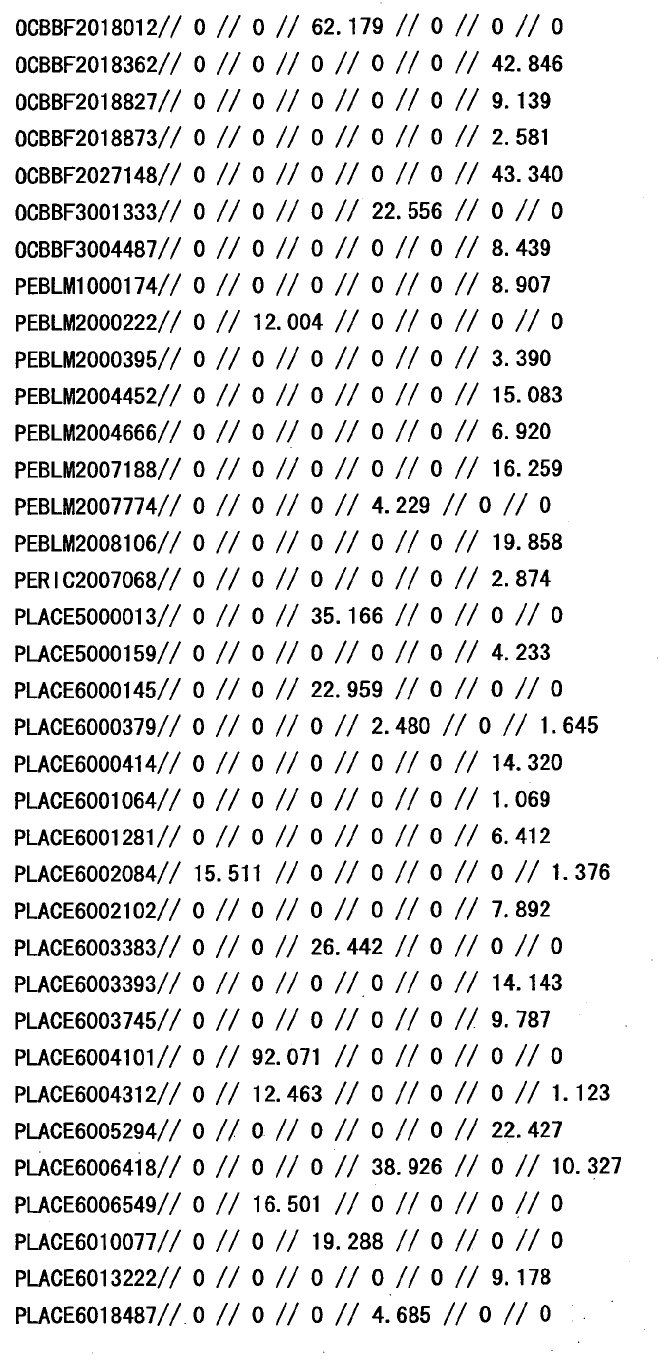

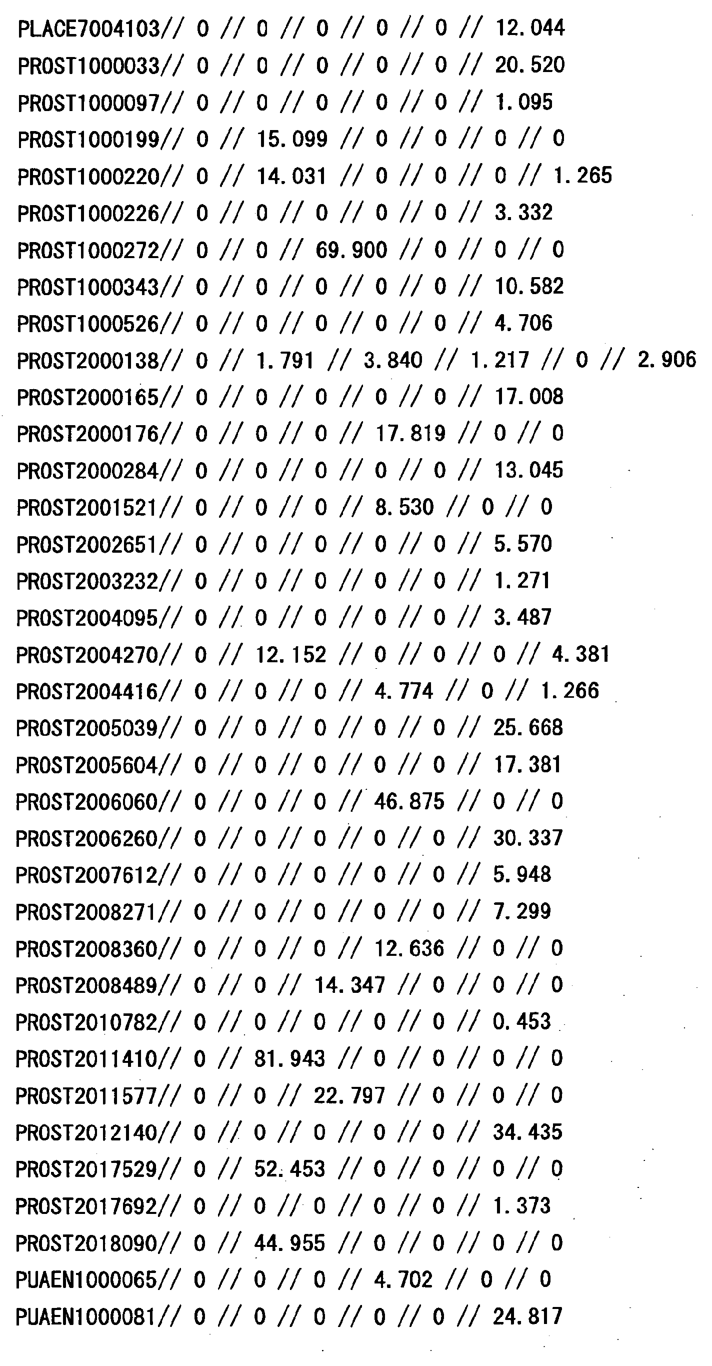

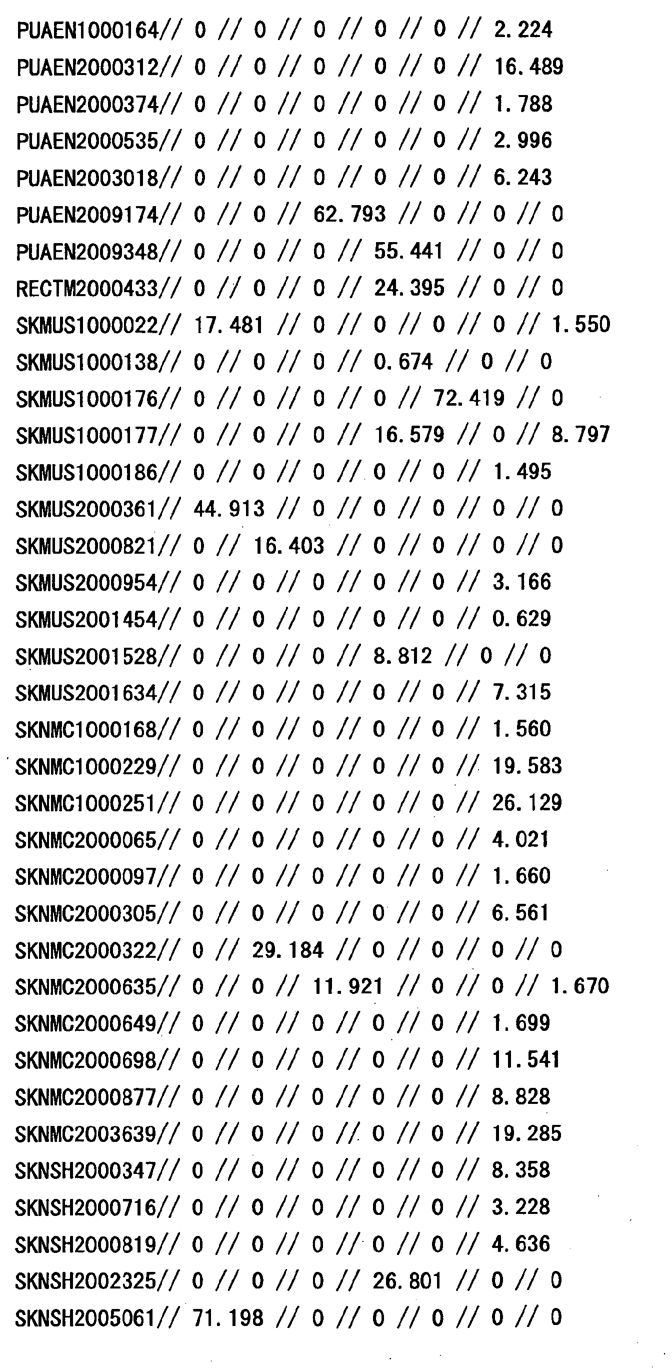

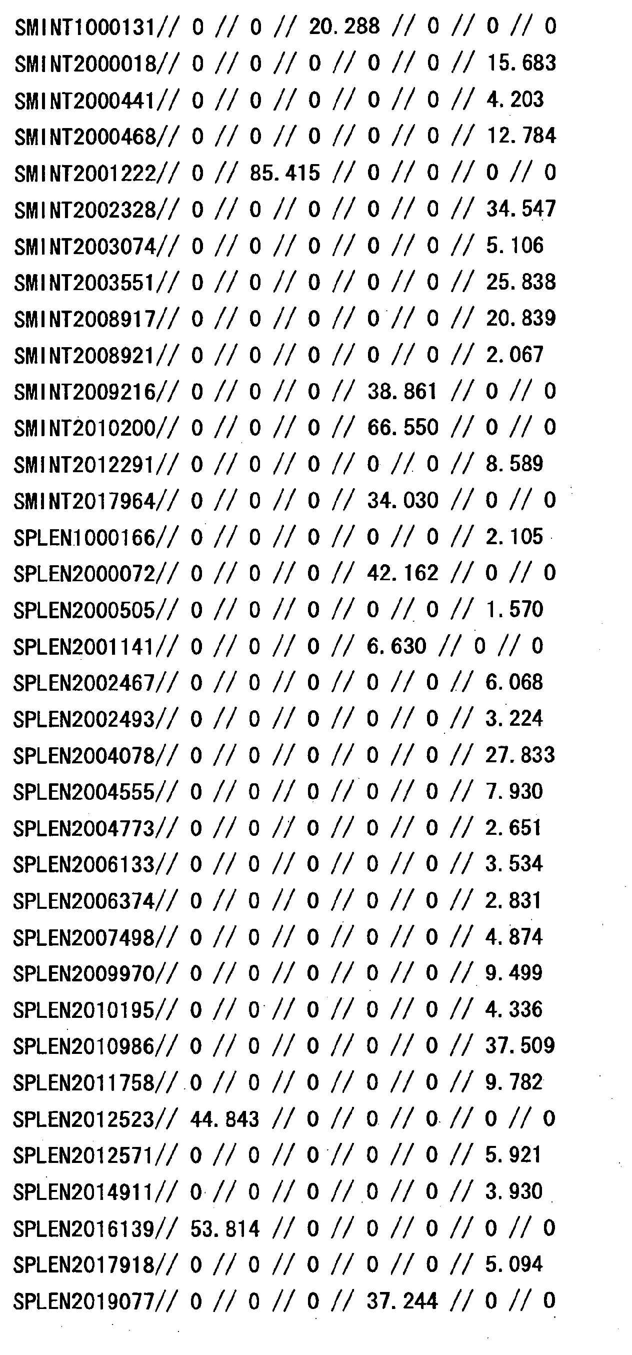

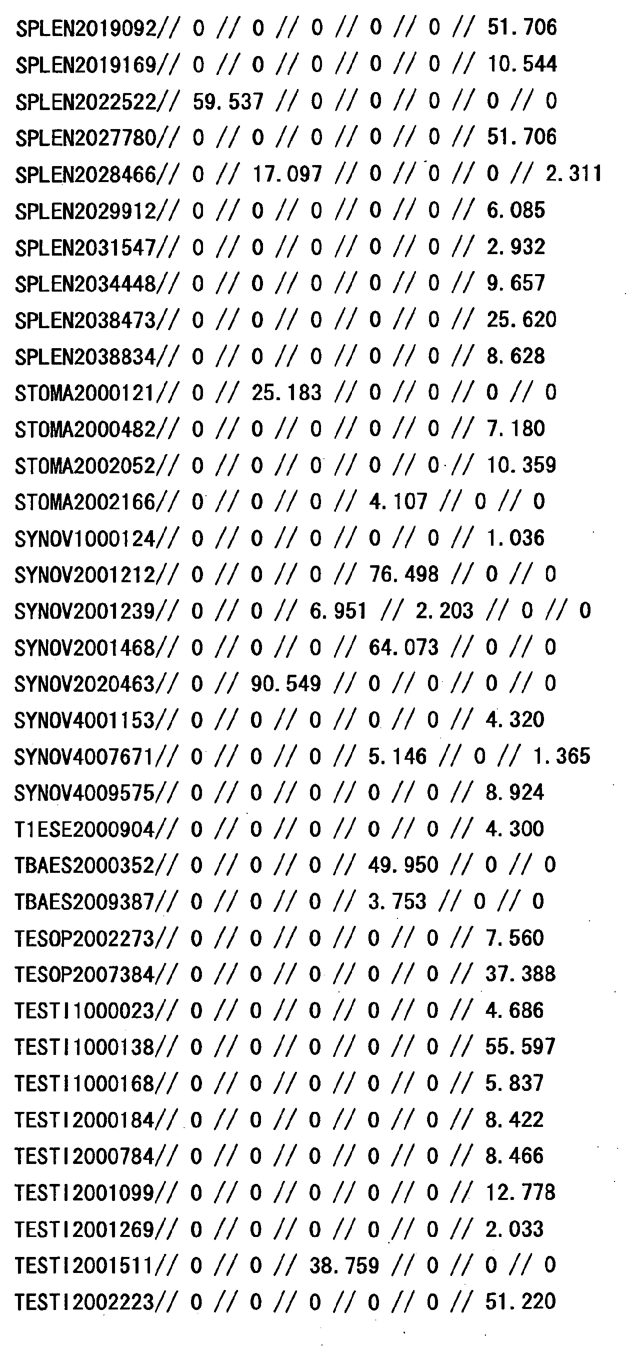

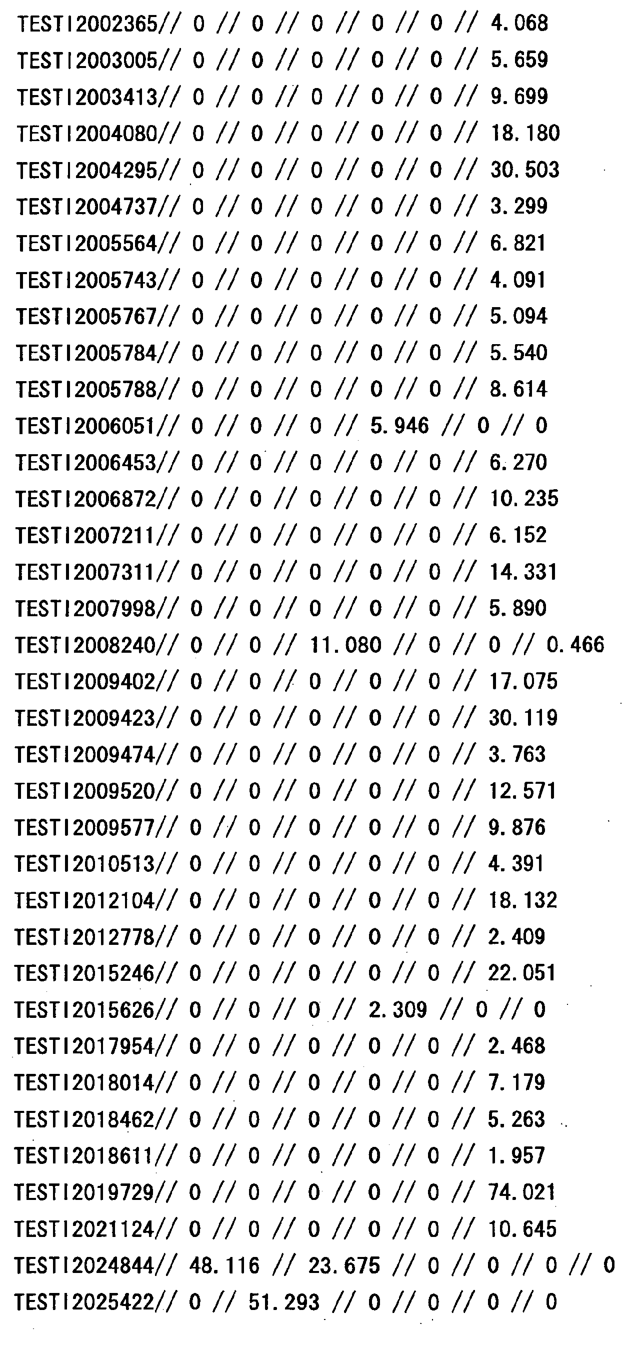

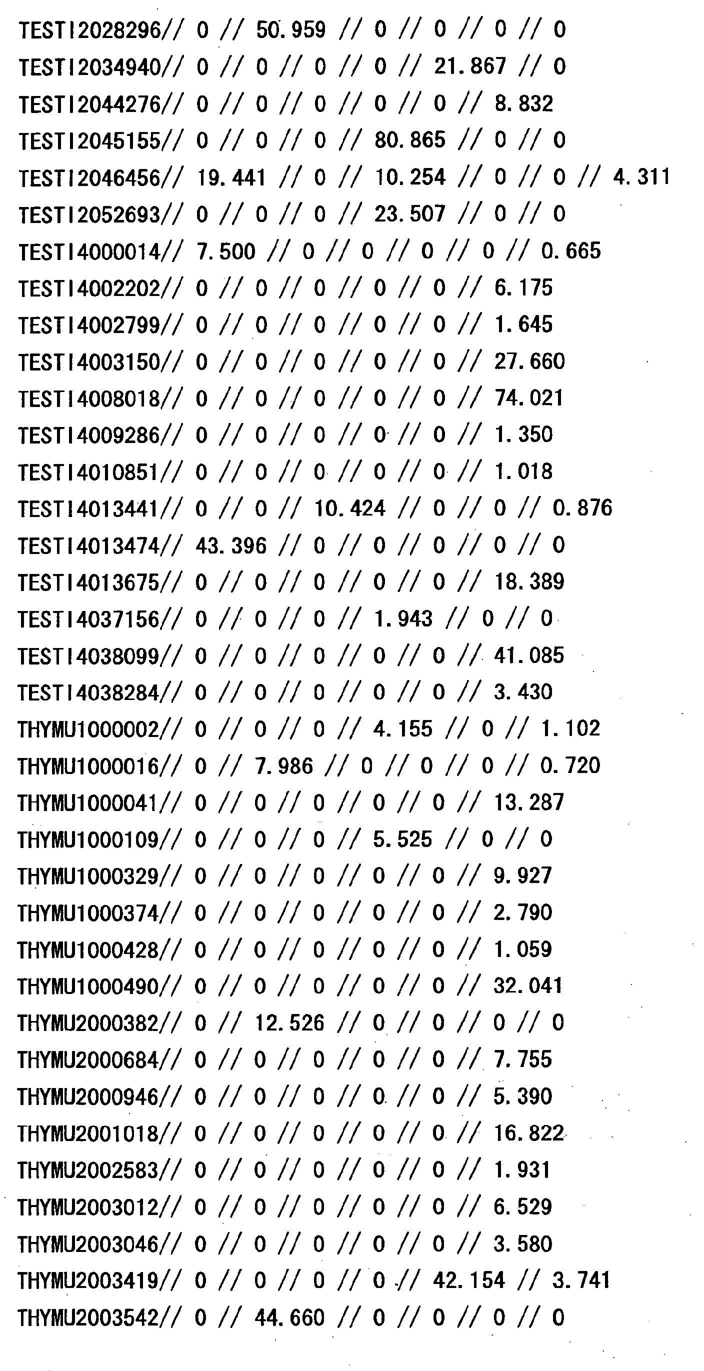

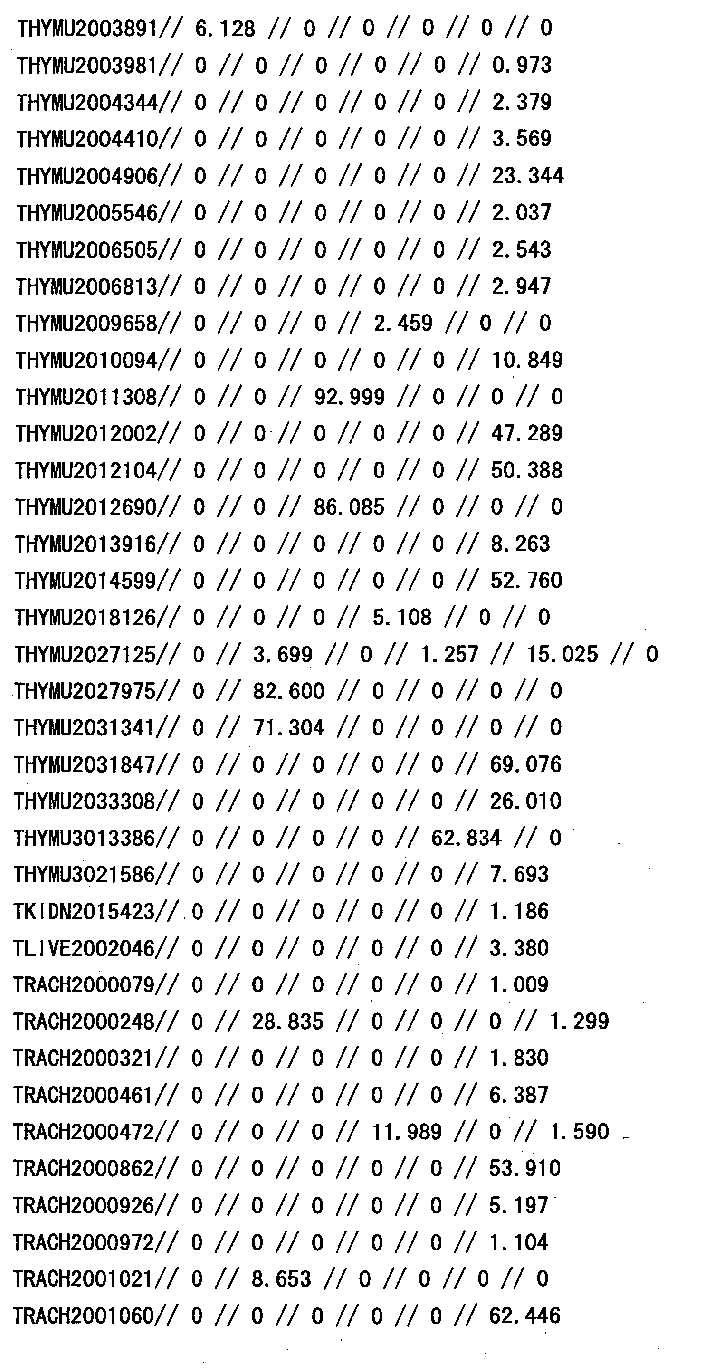

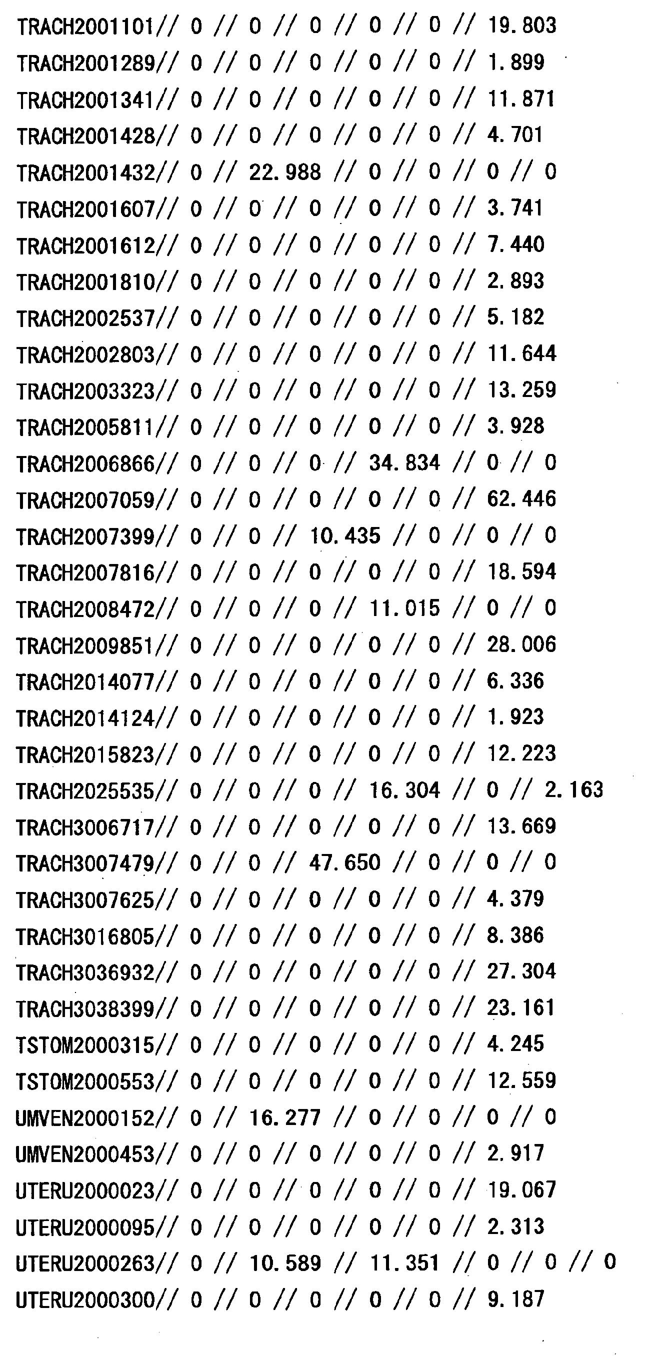

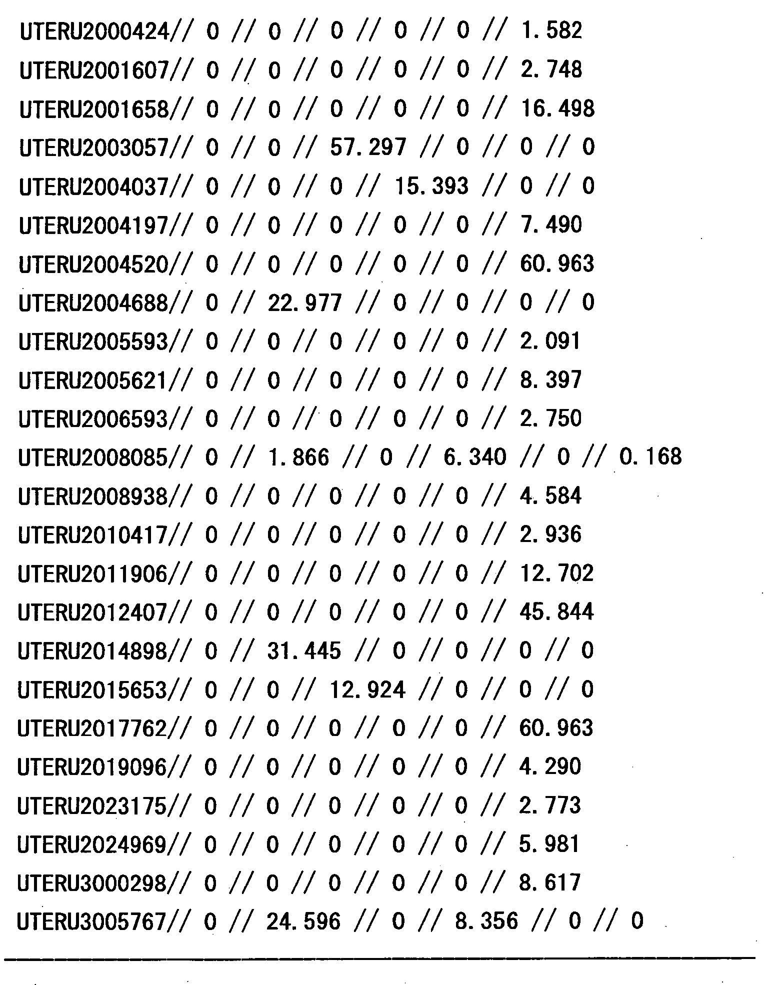

Genes whose expression levels were negligibly low in each of the above-described libraries are shown with their clone names below.
3NB691000018, 3NB691000085, 3NB691000113, 3NB691000129, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001349, 3NB692001366, 3NB692001408, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001836, ADRGL2001854, ADRGL2002013, ADRGL2002029, ADRGL2002191, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003574, ASTRO2003581, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005273, ASTRO2005343, ASTRO2005360, ASTRO2005413, ASTRO2005423, ASTRO2005553, ASTRO2005557, ASTRO2005575, ASTRO2005593, ASTRO2005687, ASTRO2005726, ASTRO2005896, ASTRO2005902, ASTRO2006356, ASTRO2006475, ASTRO2006747, ASTRO2006920, ASTRO2006952, ASTRO2007047, ASTRO2007377, ASTRO2007515, ASTRO2007578, ASTRO2007643, ASTRO2007666, ASTRO2007956, ASTRO2007962, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013585, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000054, BGGI12000067, BGGI12000161, BGGI12000533, BGGI12000544, BGGI12000616, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000177, BNGH41000216, BNGH41000235, BNGH42000130, BNGH42000132, BNGH42000405, BNGH42000675, BNGH42000739, BNGH42000791, BNGH42000815, BNGH42000994, BNGH42001247, BNGH42001406, BNGH42001576, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42007798, BNGH42008199, BNGH42008510, BNGH42008603, BNGH42008649, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001497, BRACE2001508, BRACE2001543, BRACE2001564, BRACE2001588, BRACE2001607, BRACE2001626, BRACE2001673, BRACE2001677, BRACE2001692, BRACE2001705, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003097, BRACE2003110, BRACE2003168, BRACE2003172, BRACE2003199, BRACE2003210, BRACE2003217, BRACE2003269, BRACE2003285, BRACE2003319, BRACE2003398, BRACE2003420, BRACE2003428, BRACE2003431, BRACE2003512, BRACE2003516, BRACE2003527, BRACE2003539, BRACE2003540, BRACE2003598, BRACE2003609, BRACE2003614, BRACE2003628, BRACE2003639, BRACE2003700, BRACE2003713, BRACE2003766, BRACE2003788, BRACE2003800, BRACE2003802, BRACE2003825, BRACE2003827, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005434, BRACE2005450, BRACE2005457, BRACE2005460, BRACE2005489, BRACE2005518, BRACE2005556, BRACE2005562, BRACE2005624, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006075, BRACE2006084, BRACE2006089, BRACE2006102, BRACE2006122, BRACE2006137, BRACE2006143, BRACE2006167, BRACE2006174, BRACE2006240, BRACE2006245, BRACE2006249, BRACE2006258, BRACE2006264, BRACE2006274, BRACE2006281, BRACE2006298, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006944, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527, BRACE2007538, BRACE2007563, BRACE2007567, BRACE2007621, BRACE2007640, BRACE2007641, BRACE2007646, BRACE2007663, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008968, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039624, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113,. BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015251; BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026824, BRAMY2026899, BRAMY2026902, BRAMY2026904, BRAMY2026976, BRAMY2027073, BRAMY2027114, BRAMY2027242, BRAMY2027396, BRAMY2027451, BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001913, BRAMY4002575, BRAMY4002628, BRASW1000053, BRASW1000125, BRAWH1000001, BRAWH1000002, BRAWH1000007, BRAWH1000037, BRAWH1000040, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000588, BRAWH2000595, BRAWH2000636, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001186, BRAWH2001203, BRAWH2001238, BRAWH2001239, BRAWH2001255, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008706, BRANH2008790, BRAWH2008903, BRAWH2008993, BRAWH2009112, BRAWH2009238, BRAWH2009261, BRAWH2009263, BRAWH2009298, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018526, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2Q03987, BRCAN2004173, BRCAN2004355, BRCAN2004371, BRCAN20'04653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015402, BRCAN2015464, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000111, BRHIP3000131, BRHIP3000240, BRHIP3000377, BRHIP3000457, BRHIP3000475,.BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3021971, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670 D3OST2001788, D3OST2002182 D3OST2002223, D3OST2002262 D3OST2002272, D3OST2002417 D3OST2002436, D3OST2002648 D3OST2003024 D3OST2003331 D3OST2003607, D3OST2003797 D3OST2004637 D3OST3000195, D3OST3000258, D3OST3000291 D3OST3000388 D3OST3000493, D6OST2000127, D6OST2000169, D6OST2000245 D6OST2000358 D6OST2000464 D6OST2000507, D9OST2000031 D9OST2000278 D9OST2000440 D9OST2000869, D9OST2001319, D9OST2001433, D9OST2002608, D9OST2002673 D9OST2003106 D9OST2003108 D9OST2003137 D9OST2003397 D9OST2003580 D9OST2003594 D9OST2003762, D9OST2003791 D9OST2003989 D9OST2004018 D9OST2004073 D9OST2004417 DFNES1000003, DFNES1000083, DFNES1000107, DFNES2000143, DFNES2000146, DFNES2000153, DFNES2000212, DFNES2000268, DFNES2000292, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004383, DFNES2004608, DFNES2004684, DFNES2005266, DFNES2005527, DFNES2005540, DFNES2005779, DFNES2005875, DFNES2006346, DFNES2006944, DFNES2007113, DFNES2007332, DFNES2007641, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000025, FCBBF1000038, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094, FCBBF2000105, FCBBF2000123, FCBBF2000195, FCBBF2000199, FCBBF2000214, FCBBF2000232, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005330, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016178, FCBBF3016451, FCBBF3016618, FCBBF3016622, FCBBF3016644, FCBBF3016667, FCBBF3016928, FCBBF3016987, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000220, FEBRA2000253, FEBRA2000282, FEBRA2000286, FEBRA2000297, FEBRA2000311, FEBRA2000321, FEBRA2000333, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003897, FEBRA2003907, FEBRA2003926, FEBRA2003930, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004325, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005572, FEBRA2005701, FEBRA2005726, FEBRA2005734, FEBRA2005752, FEBRA2005778, FEBRA2005787, FEBRA2005788, FEBRA2005998, FEBRA2006055, FEBRA2006061, FEBRA2006092, FEBRA2006150, FEBRA2006165, FEBRA2006255, FEBRA2006315, FEBRA2006346, FEBRA2006354, FEBRA2006357, FEBRA2006368, FEBRA2006370, FEBRA2006379, FEBRA2006396, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007551, FEBRA2007566, FEBRA2007589, FEBRA2007613, FEBRA2007620, FEBRA2007708, FEBRA2007714, FEBRA2007793, FEBRA2007818, FEBRA2007823, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008210, FEBRA2008255, FEBRA2008265, FEBRA2008266, FEBRA2008282, FEBRA2008287, FEBRA2008302, FEBRA2008311, FEBRA2008341, FEBRA2008360, FEBRA2008459, FEBRA2008468, FEBRA2008475, FEBRA2008481, FEBRA2008542, FEBRA2008583, FEBRA2008660, FEBRA2008662, FEBRA2008681, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018433, FEBRA2018476, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000131, HCHON1000142, HCHON1000166, HCHON1000176, HCHON1000191, HCHON2000038, HCHON2000062, HCHON2000100, HCHON2000156, HCHON2000199, HCHON2000212, HCHON2000244, HCHON2000265, HCHON2000271, HCHON2000315, HCHON2000322, HCHON2000323, HCHON2000344, HCHON2000373, HCHON2000395, HCHON2000417, HCHON2000418, HCHON2000503, HCHON2000660, HCHON2000663, HCHON2000673, HCHON2000676, HCHON2000698, HCHON2000699, HCHON2000705, HCHON2000743, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001084, HCHON2001099, HCHON2001108, HCHON2001110, HCHON2001116, HCHON2001161, HCHON2001214, HCHON2001233, HCHON2001269, HCHON2001434, HCHON2001450, HCHON2001453, HCHON2001497, HCHON2001505, HCHON2001519, HCHON2001523, HCHON2001546, HCHON2001548, HCHON2001577, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000114, HHDPC1000163, HHDPC2000055, HHDPC2000102, HHDPC2000104, HHDPC2000115, HHDPC2000258, HHDPC2000315, HHDPC2000455, HHDPC2000462, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000064, HLUNG1000084, HLUNG1000091, HLUNG1000104, HLUNG1000105, HLUNG1000110, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001507, HLUNG2001518, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002648, HLUNG2002659, HLUNG2002707, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004067, HLUNG2004154, HLUNG2004170, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000137, HSYRA2000190, HSYRA2000221, HSYRA2000224, HSYRA2000232, HSYRA2000237, HSYRA2000253, HSYRA2000332, HSYRA2000371, HSYRA2000424, HSYRA2000507, HSYRA2000510, HSYRA2000605, HSYRA2000629, HSYRA2000640, HSYRA2000706, HSYRA2000760, HSYRA2000787, HSYRA2000792, HSYRA2000828, HSYRA2000832, HSYRA2000925, HSYRA2000927, HSYRA2001003, HSYRA2001103, HSYRA2001105, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001580, HSYRA2001615, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000158, IMR321000161, IMR321000165, IMR321000207, IMR321000210, IMR321000230, IMR321000242, IMR321000266, IMR322000010, IMR322000072, IMR322000107, IMR322000121, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917, IMR322000918, IMR322000919, IMR322000953, IMR322000973, IMR322000984, IMR322001018, IMR322001167, IMR322001185, IMR322001245, IMR322001317, IMR322001318, IMR322001322, IMR322001380, IMR322001438, IMR322001452, IMR322001491, IMR322001534, IMR322001541, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722, KIDNE2000801, KIDNE2000832, KIDNE2000833, KIDNE2000846, KIDNE2000867, KIDNE2000909, KIDNE2001117, KIDNE2001140, KIDNE2001162, KIDNE2001269, KIDNE2001361, KIDNE2001636, KIDNE2001713, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003335, KIDNE2003357, KIDNE2003373, KIDNE2003377, KIDNE2003507, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007352, KIDNE2007356, KIDNE2007422, KIDNE2007569, KIDNE2007810, KIDNE2007811, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000050, MESAN1000079, MESAN1000101, MESAN1000121, MESAN1000147, MESAN1000180, MESAN2000023, MESAN2000026, MESAN2000067, MESAN2000167, MESAN2000264, MESAN2000291, MESAN2000434, MESAN2000457, MESAN2000462, MESAN2000501, MESAN2000608, MESAN2000620, MESAN2000711, MESAN2000894, MESAN2000909, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002844, MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003622, MESAN2003646, MESAN2003662, MESAN2003709, MESAN2003851, MESAN2004138, MESAN2004288, MESAN2004575, MESAN2005284, MESAN2005303, MESAN2005371, MESAN2005689, MESAN2005724, MESAN2005808, MESAN2005811, MESAN2005957, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000121, NB9N41000142, NB9N41000146, NB9N41000340, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000259, NT2NE2000284, NT2NE2000299, NT2NE2000353, NT2NE2000356, NT2NE2000369, NT2NE2000374, NT2NE2000383, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909, NT2NE2000950, NT2NE2000951, NT2NE2000980, NT2NE2001000, NT2NE2001005, NT2NE2001021, NT2NE2001040, NT2NE2001048, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005784, NT2NE2005804, NT2NE2005821, NT2NE2005876, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000027, NT2RI1000035, NT2RI1000164, NT2RI1000172, NT2RI2000004, NT2RI2000007, NT2RI2000064, NT2RI2000107, NT2RI2000116, NT2RI2000117, NT2RI2000128, NT2RI2000133, NT2RI2000167, NT2RI2000173, NT2RI2000187, NT2RI2000270, NT2RI2000276, NT2RI2000282, NT2RI2000284, NT2RI2000294, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002120, NT2RI2002152, NT2RI2002153, NT2RI2002179, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002540, NT2RI2002549, NT2RI2002554, NT2RI2002564, NT2RI2002585, NT2RI2002592, NT2RI2002602, NT2RI2002699, NT2RI2002802, NT2RI2002819, NT2RI2002822, NT2RI2002847, NT2RI2002852, NT2RI2002923, NT2RI2002926, NT2RI2002958, NT2RI2002970, NT2RI2003011, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003678, NT2RI2003751, NT2RI2003884, NT2RI2003921, NT2RI2003973, NT2RI2003993, NT2RI2004059, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005150, NT2RI2005166, NT2RI2005239, NT2RI2005246, NT2RI2005368, NT2RI2005382, NT2RI2005405, NT2RI2005473, NT2RI2005520, NT2RI2005564, NT2RI2005579, NT2RI2005621, NT2RI2005628, NT2RI2005670, NT2RI2005710, NT2RI2005723, NT2RI2005772, NT2RI2005811, NT2RI2005814, NT2RI2005816, NT2RI2005818, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006762, NT2RI2006788, NT2RI2006838, NT2RI2006855, NT2RI2006943, NT2RI2006973, NT2RI2007046, NT2RI2007048, NT2RI2007054, NT2RI2007084, NT2RI2007096, NT2RI2007116, NT2RI2007148, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007507, NT2RI2007629, NT2RI2007723, NT2RI2007729, NT2RI2007751, NT2RI2007754, NT2RI2007827, NT2RI2007853, NT2RI2007877, NT2RI2007879, NT2RI2007884, NT2RI2007891, NT2RI2007956, NT2RI2007965, NT2RI2007987, NT2RI2008007, NT2RI2008045, NT2RI2008050, NT2RI2008171, NT2RI2008188, NT2RI2008204, NT2RI2008221, NT2RI2008233, NT2RI2008455, NT2RI2008502, NT2RI2008526, NT2RI2008566, NT2RI2008598, NT2RI2008622, NT2RI2008656, NT2RI2008791, NT2RI2008801, NT2RI2008808, NT2RI2008812, NT2RI2008841, NT2RI2008952, NT2RI2009005, NT2RI2009065, NT2RI2009066, NT2RI2009083, NT2RI2009101, NT2RI2009103, NT2RI2009144, NT2RI2009151, NT2RI2009194, NT2RI2009215, NT2RI2009259, NT2RI2009269, NT2RI2009281, NT2RI2009289, NT2RI2009301, NT2RI2009402, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015533, NT2RI2016128, NT2RI2016134, NT2RI2017529, NT2RI2018311, NT2RI2018363, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007291, NT2RI3007443, NT2RI3007684, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000032, NT2RP6000033, NT2RP6000039, NT2RP6000043, NT2RP6000059, NT2RP6000060, NT2RP6000072, NT2RP6000077, NT2RP6000078, NT2RP6000085, NT2RP6000086, NT2RP6000100, NT2RP6000110, NT2RP6000119, NT2RP6000127, NT2RP6000130, NT2RP6000140, NT2RP6000150, NT2RP7000041, NT2RP7000069, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926, NT2RP7001074, NT2RP7001080, NT2RP7001156, NT2RP7001231, NT2RP7001306, NT2RP7001319, NT2RP7001335, NT2RP7001364, NT2RP7001532, NT2RP7001602, NT2RP7001856, NT2RP7001962, NT2RP7002028, NT2RP7002064, NT2RP7002151, NT2RP7002163, NT2RP7002243, NT2RP7002282, NT2RP7002478, NT2RP7002554, NT2RP7002603, NT2RP7002650, NT2RP7002738, NT2RP7002779, NT2RP7002829, NT2RP7002841, NT2RP7002875, NT2RP7002906, NT2RP7002978, NT2RP7003025, NT2RP7003050, NT2RP7003055, NT2RP7003084, NT2RP7003091, NT2RP7003107, NT2RP7003134, NT2RP7003148, NT2RP7003203, NT2RP7003439, NT2RP7003511, NT2RP7003629, NT2RP7003680, NT2RP7003724, NT2RP7004027, NT2RP7004080, NT2RP7004114, NT2RP7004204, NT2RP7004233, NT2RP7004260, NT2RP7004358, NT2RP7004396, NT2RP7004428, NT2RP7004481, NT2RP7004541, NT2RP7004641, NT2RP7004656, NT2RP7004687, NT2RP7004722, NT2RP7004728, NT2RP7004766, NT2RP7004790, NT2RP7004884, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005846, NT2RP7006033, NT2RP7006160, NT2RP7006162, NT2RP7006223, NT2RP7006263, NT2RP7006296, NT2RP7006304, NT2RP7006374, NT2RP7006395, NT2RP7006408, NT2RP7006457, NT2RP7006539, NT2RP7006547, NT2RP7006601, NT2RP7006619, NT2RP7006621, NT2RP7006636, NT2RP7006853, NT2RP7006886, NT2RP7006887, NT2RP7006980, NT2RP7006986, NT2RP7006995, NT2RP7007114, NT2RP7007221, NT2RP7007226, NT2RP7007252, NT2RP7007269, NT2RP7007310, NT2RP7007359, NT2RP7007381, NT2RP7007387, NT2RP7007406, NT2RP7007480, NT2RP7007504, NT2RP7007530, NT2RP7007537, NT2RP7007580, NT2RP7007594, NT2RP7007617, NT2RP7007643, NT2RP7007766, NT2RP7007925, NT2RP7007930, NT2RP7008013, NT2RP700.8015, NT2RP7008133, NT2RP7008137, NT2RP7008142, NT2RP7008144, NT2RP7008315, NT2RP7008360, NT2RP7008396, NT2RP7008406, NT2RP7008435, NT2RP7008441, NT2RP7008454, NT2RP7008544, NT2RP7008550, NT2RP7008557, NT2RP7008623, NT2RP7008657, NT2RP7008714, NT2RP7008720, NT2RP7008855, NT2RP7008863, NT2RP7009019, NT2RP7009030, NT2RP7009097, NT2RP7009168, NT2RP7009215, NT2RP7009225, NT2RP7009236, NT2RP7009253, NT2RP7009267, NT2RP7009322, NT2RP7009363, NT2RP7009370, NT2RP7009394, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858, NTONG2000876, NTONG2000878, NTONG2000966, NTONG2000985, NTONG2001014, NTONG2001079, NTONG2001157, NTONG2001173, NTONG2001220, NTONG2001362, NTONG2001428, NTONG2001532, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000153, OCBBF1000175, OCBBF1000185, OCBBF1000192, OCBBF1000254, OCBBF2000013, OCBBF2000126, OCBBF2000178, OCBBF2000231, OCBBF2000277, OCBBF2000287, OCBBF2000323, OCBBF2000452, OCBBF2000467, OCBBF2000703, OCBBF2000712, OCBBF2000719, OCBBF2000824, OCBBF2000831, OCBBF2000904, OCBBF2000982, OCBBF2000998, OCBBF2001075, OCBBF2001101, OCBBF2001124, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006005, OCBBF2006030, OCBBF2006058, OCBBF2006113, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009301, OCBBF2009352, OCBBF2009391, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010863, OCBBF2010871, OCBBF2010931, OCBBF2010945, OCBBF2010978, OCBBF2010989, OCBBF2011021, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017815, OCBBF2017882, OCBBF2017888, OCBBF2017893, OCBBF2017920, OCBBF2018014, OCBBF2018084, OCBBF2018167, OCBBF2018206, OCBBF2018229, OCBBF2018234, OCBBF2018380, OCBBF2018563, OCBBF2018581, OCBBF2018618, OCBBF2018663, OCBBF2018687, OCBBF2018707, OCBBF2018828, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264; OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000147, PEBLM2000170, PEBLM2000180, PEBLM2000213, PEBLM2000248, PEBLM2000267, PEBLM2000270, PEBLM2000308, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007296, PEBLM2007437, PEBLM2007447, PEBLM2007598, PEBLM2007832, PEBLM2007834, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000263, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003399, PLACE6003539, PLACE6003621, PLACE6003705, PLACE6003740, PLACE6003771, PLACE6003796, PLACE6003839, PLACE6003850, PLACE6004005, PLACE6004219, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000215, PROST1000217, PROST1000246, PROST1000262, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000564, PROST1000575, PROST2000036, PROST2000053, PROST2000067, PROST2000079, PROST2000105, PROST2000143, PROST2000162, PROST2000203, PROST2000206, PROST2000212, PROST2000241, PROST2000267, PROST2000273, PROST2000274, PROST2000277, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003338, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004570, PROST2004727, PROST2004739, PROST2004742, PROST2004744, PROST2004817, PROST2005067, PROST2005076, PROST2005093, PROST2005121, PROST2005131, PROST2005143, PROST2005228, PROST2005272, PROST2005285, PROST2005426, PROST2005466, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006196, PROST2006201, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011439, PROST2011482, PROST2011631, PROST2011660, PROST2012005, PROST2012007, PROST2012016, PROST2012088, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017578, PROST2017612, PROST2017617, PROST2017700, PROST2017701, PROST2017729, PROST2017836, PROST2017910, PROST2017972, PROST2018030, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000085, PUAEN1000087, PUAEN1000121, PUAEN1000147, PUAEN1000161, PUAEN1000175, PUAEN1000239, PUAEN1000275, PUAEN1000276, PUAEN1000288, PUAEN1000322, PUAEN2000080, PUAEN2000152, PUAEN2000175, PUAEN2000247, PUAEN2000394, PUAEN2000497, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000157, SALGL1000171, SKMUS1000014, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000847, SKMUS2000863, SKMUS2000873, SKMUS2000894, SKMUS2000898, SKMUS2000902, SKMUS2000931, SKMUS2000933, SKMUS2000945, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000264, SKNMC2000224, SKNMC2000256, SKNMC2000356, SKNMC2000374, SKNMC2000583, SKNMC2000593, SKNMC2000612, SKNMC2000622, SKNMC2000966, SKNMC2001113, SKNMC2001324, SKNMC2001503, SKNMC2001555, SKNMC2001596, SKNMC2001623, SKNMC2002402, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000482, SKNSH2000516, SKNSH2000550, SKNSH2000971, SKNSH2001222, SKNSH2001875, SKNSH2001931, SKNSH2002054, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000694, SMINT2000747, SMINT2000811, SMINT2000824, SMINT2000933, SMINT2000984, SMINT2001114, SMINT2001158, SMINT2001183, SMINT2001199, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002159, SMINT2002202, SMINT2002210, SMINT2002224, SMINT2002281, SMINT2002311, SMINT2002314, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2002976, SMINT2003003, SMINT2003128, SMINT2003150, SMINT2003169, SMINT2003317, SMINT2003340, SMINT2003386, SMINT2003423, SMINT2003505, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008960, SMINT2009119, SMINT2009121, SMINT2009212, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000106, SPLEN1000116, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000874, SPLEN2000882, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611, SPLEN2004662, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005927, SPLEN2005939, SPLEN2006122, SPLEN2006143, SPLEN2006232, SPLEN2006268, SPLEN2006283, SPLEN2006305, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019102, SPLEN2019131, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001144, SYNOV2001251, SYNOV2001262, SYNOV2001300, SYNOV2001356, SYNOV2001374, SYNOV2001390, SYNOV2001451, SYNOV2001457, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2002665, TBAES2000004, TBAES2000059, TBAES2000116, TBAES2000255, TBAES2000315, TBAES2000932, TBAES2001171, TBAES2001220, TBAES2001229, TBAES2001258, TBAES2001296, TBAES2001492, TBAES2001553, TBAES2001751,TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000142, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005759, TESTI2005775, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006588, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009447, TESTI2009462, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015213, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052698, TESTI2052799, TESTI20,52822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000060, THYMU1000074, THYMU1000083, THYMU1000098, THYMU1000103, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004356, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012024, THYMU2012073, THYMU2012113, THYMU2012273, THYMU2012401, THYMU2012492, THYMU2012507, THYMU2012565, THYMU2012574, THYMU2012625, THYMU2012631, THYMU2012665, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000780, TRACH2000807, TRACH2000873, TRACH2000894, TRACH2000898, TRACH2000920, TRACH2000923, TRACH2000944, TRACH2000959, TRACH2000981, TRACH2000986, TRACH2001037, TRACH2001085, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001347, TRACH2001395, TRACH2001400, TRACH2001403, TRACH2001416, TRACH2001443, TRACH2001463, TRACH2001474, TRACH2001478, TRACH2001523, TRACH2001525, TRACH2001532, TRACH2001549, TRACH2001592, TRACH2001596, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002100, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006870, TRACH2006918, TRACH2007324, TRACH2007358, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008081, TRACH2008112, TRACH2008113, TRACH2008127, TRACH2008130, TRACH2008278, TRACH2008300, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902,. TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TSTOM1000135, TSTOM1000186, TSTOM2000139, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000133, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000047, UTERU2000074, UTERU2000099, UTERU2000154, UTERU2000197, UTERU2000218, UTERU2000238, UTERU2000243, UTERU2000260, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004299, UTERU2004461, UTERU2004564, UTERU2004664, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011962, UTERU2011968, UTERU2012031, UTERU2012101, UTERU2012114, UTERU2012230, UTERU2012252, UTERU2012286, UTERU2012333, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the D3OST, D6OST, D9OST, DFNES, ERLTF, FCBBF, and FEBRA libraries were analyzed for expression frequency. The analysis results are described below. Each is a relative value, where the expression level over all tissues is taken as 100. The larger this value, the greater the level of expression.

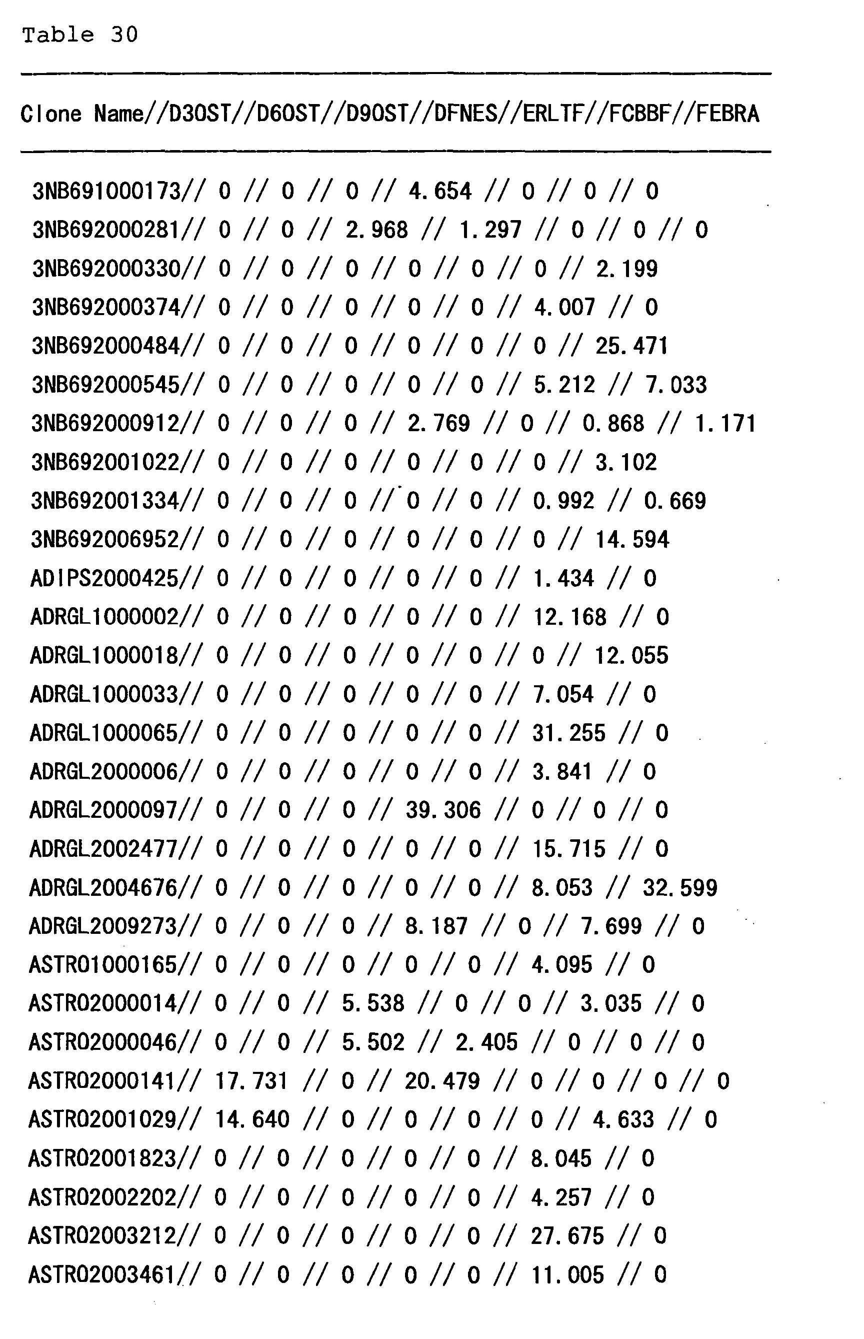

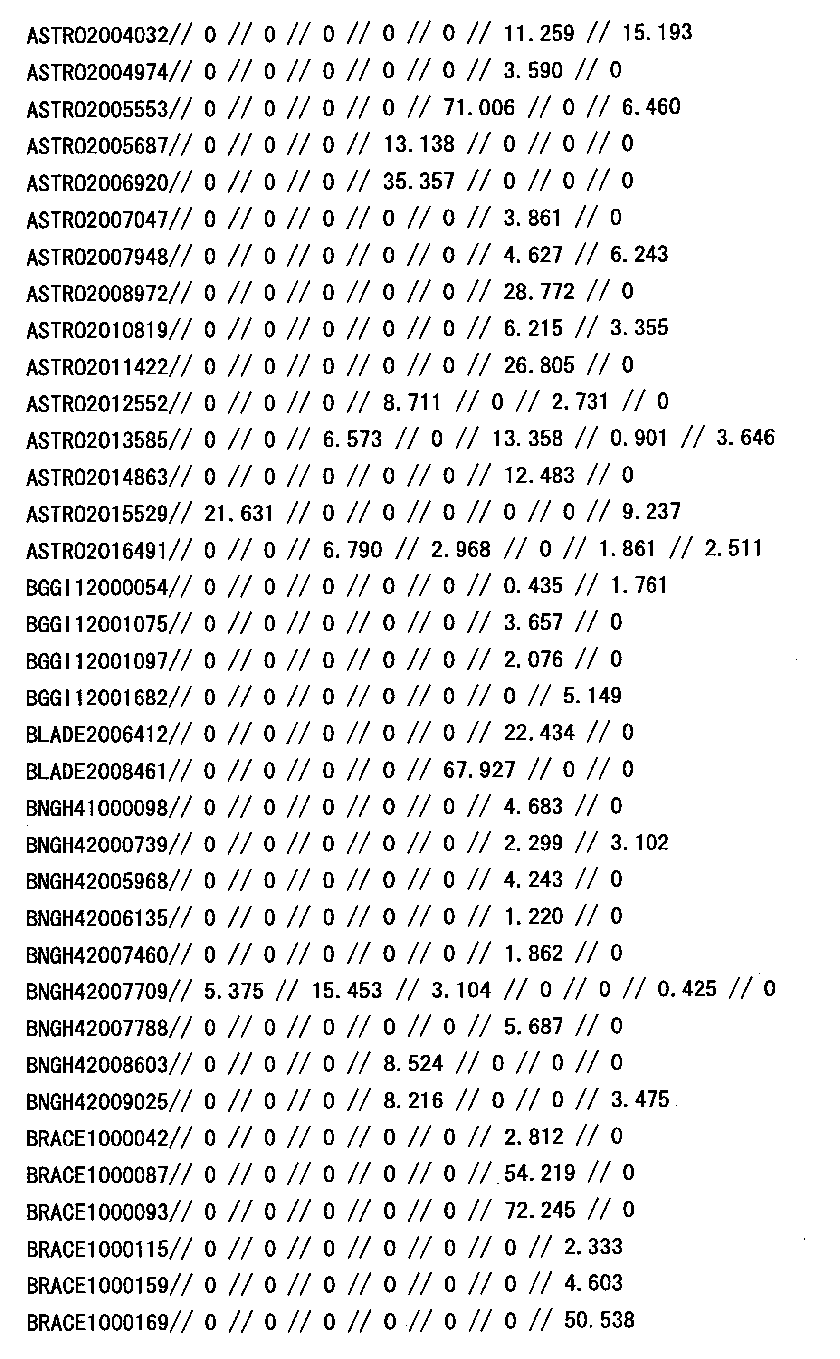

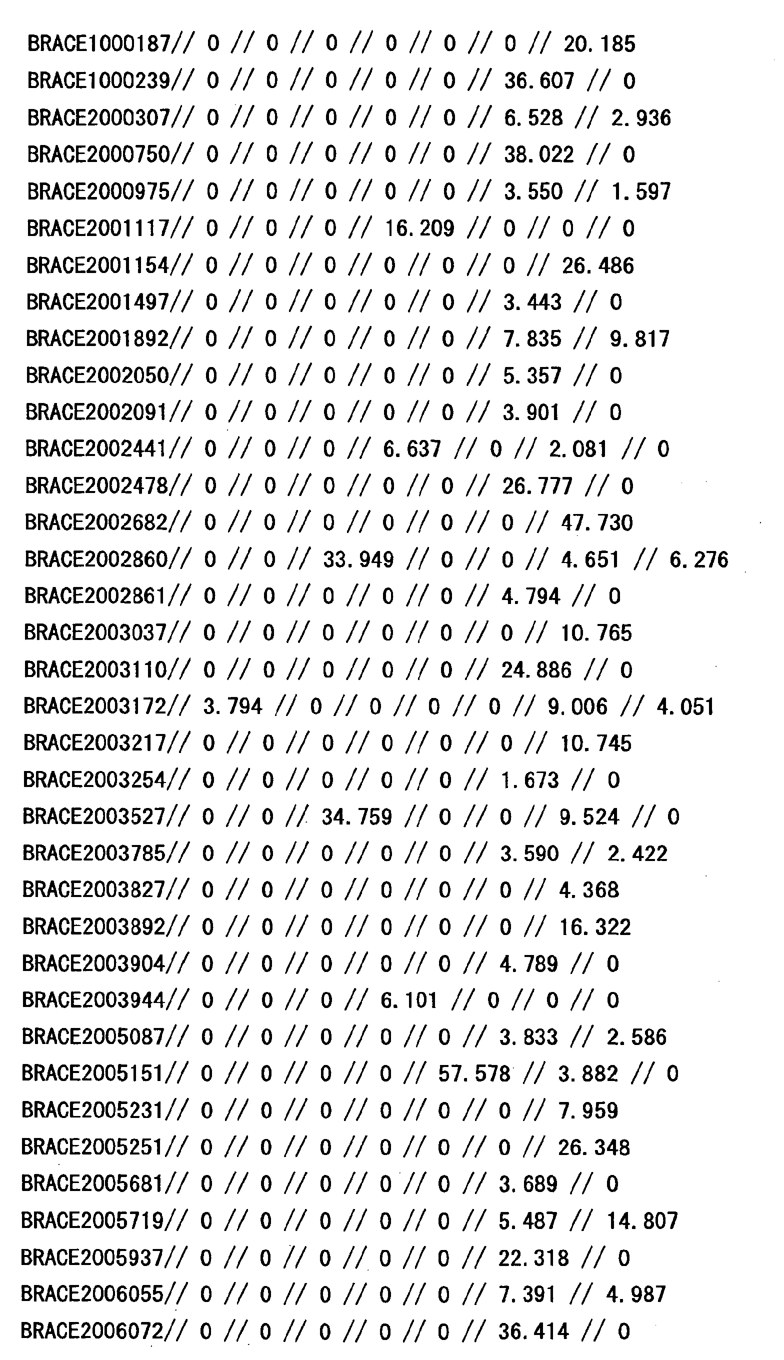

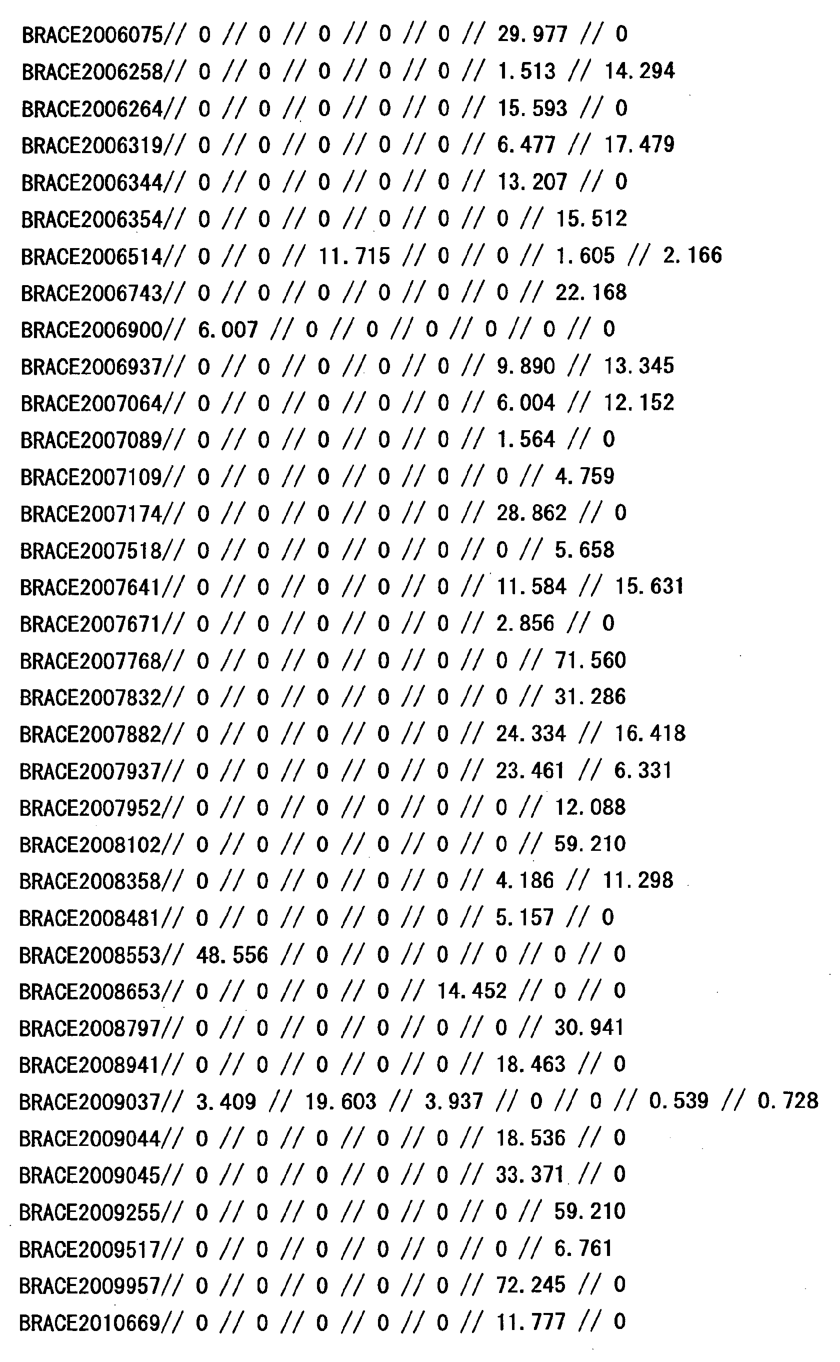

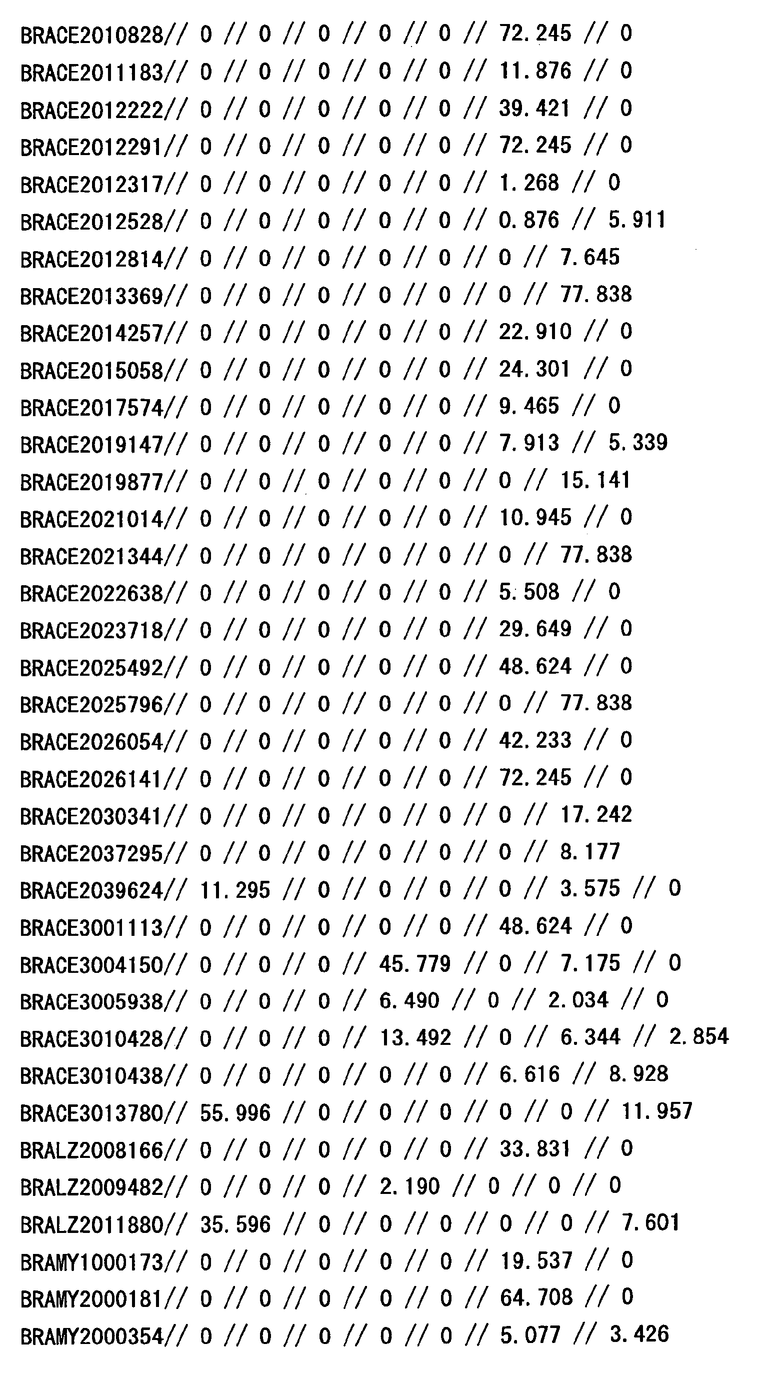

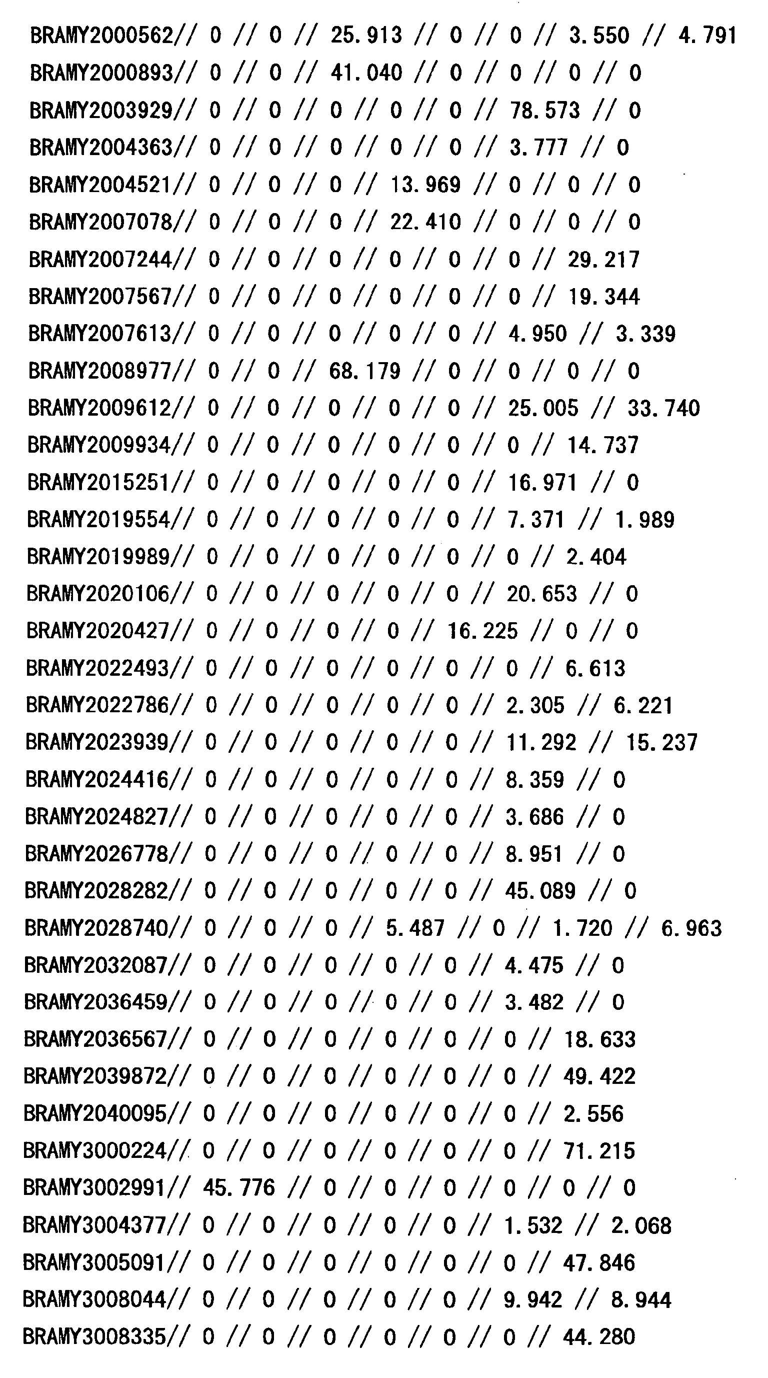

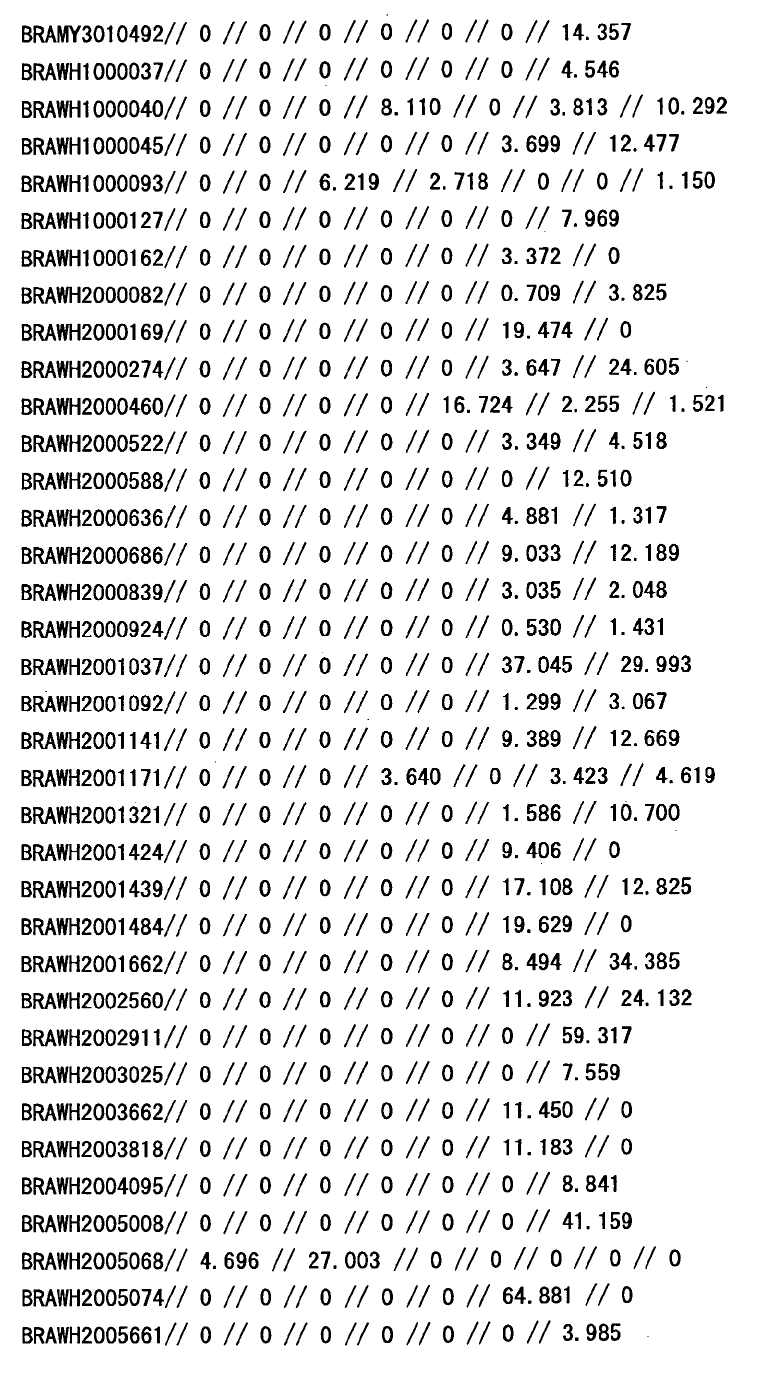

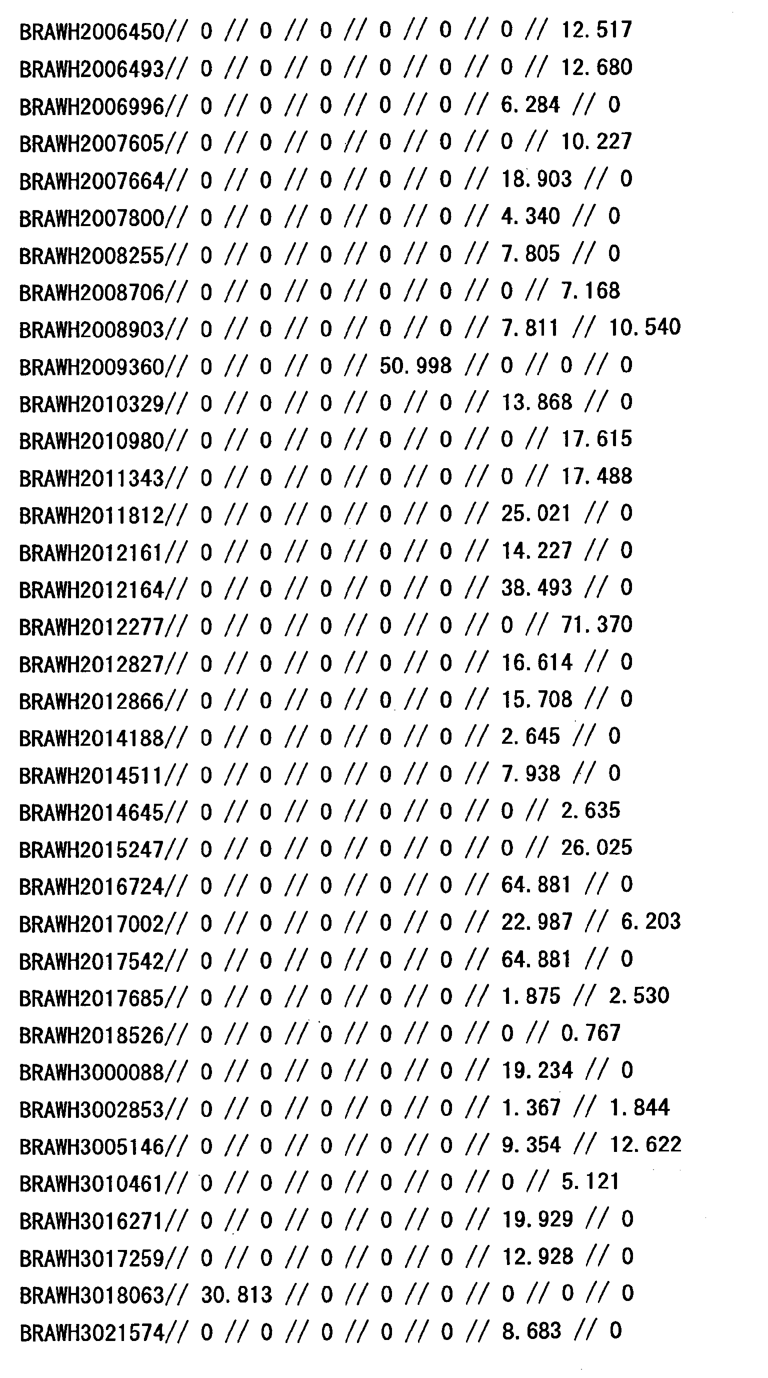

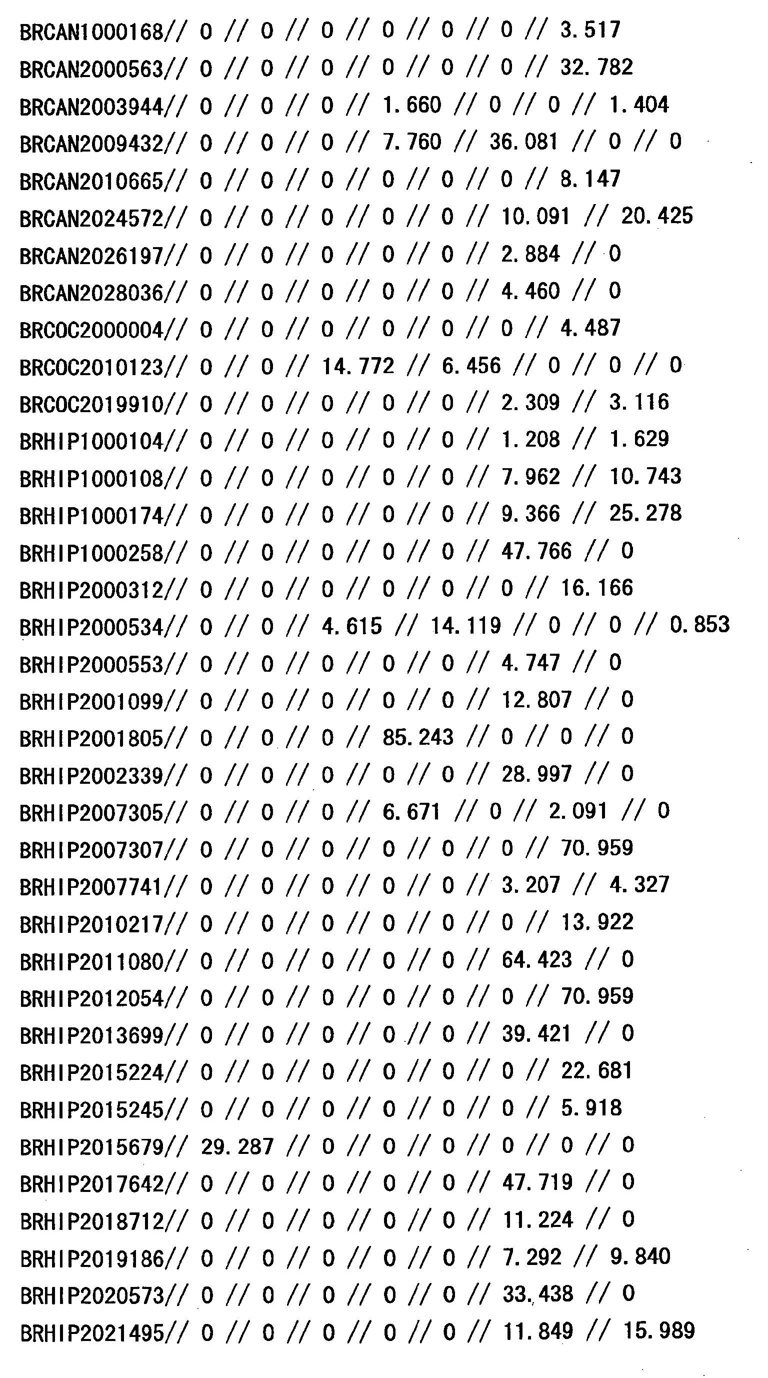

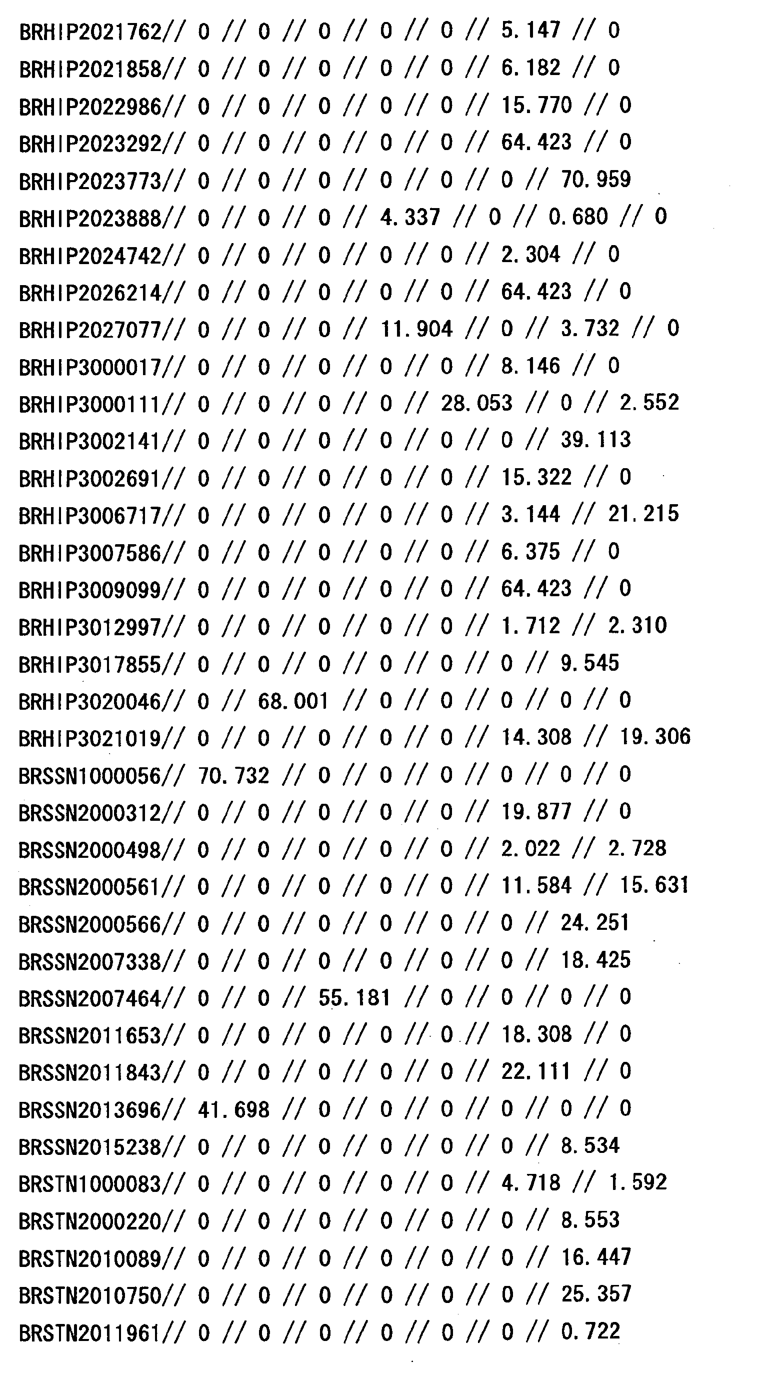

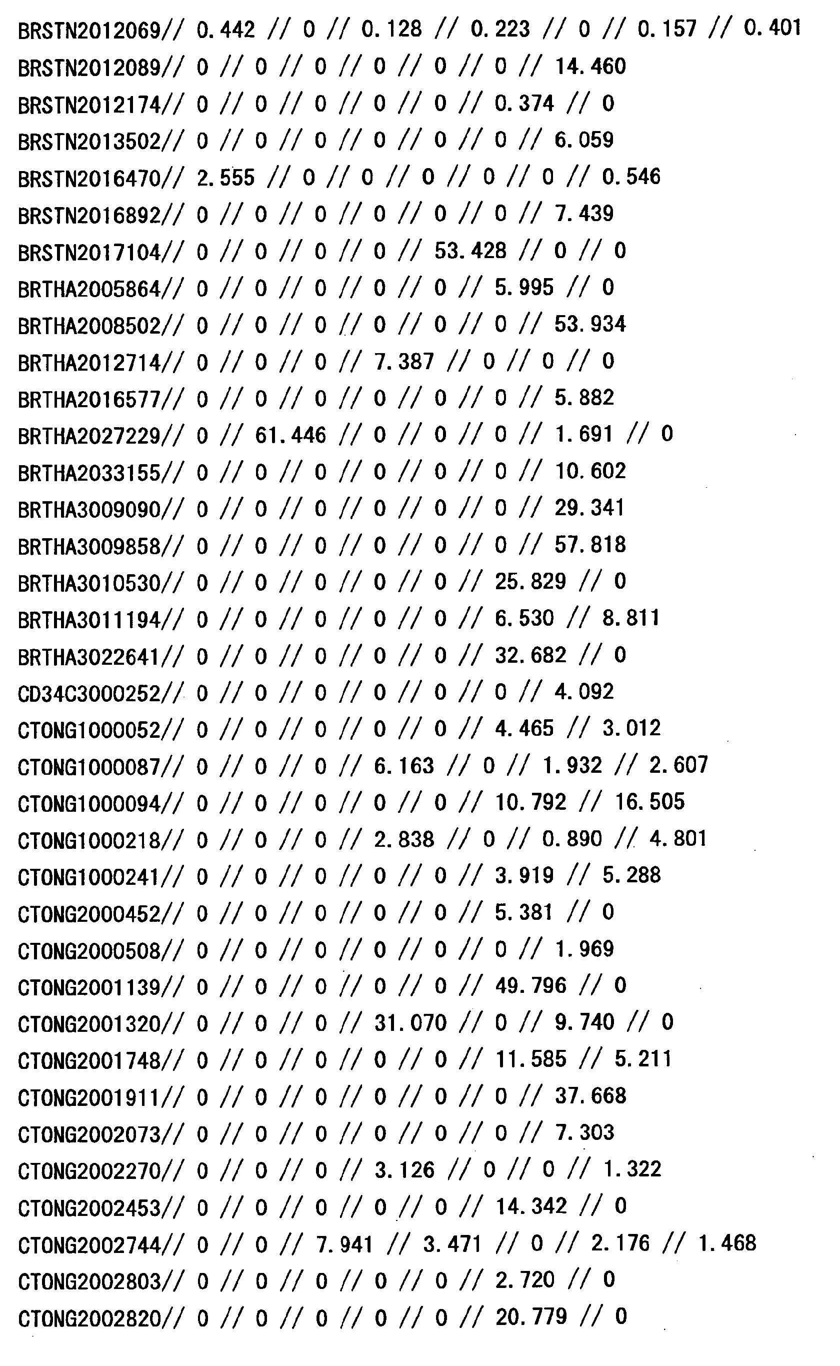

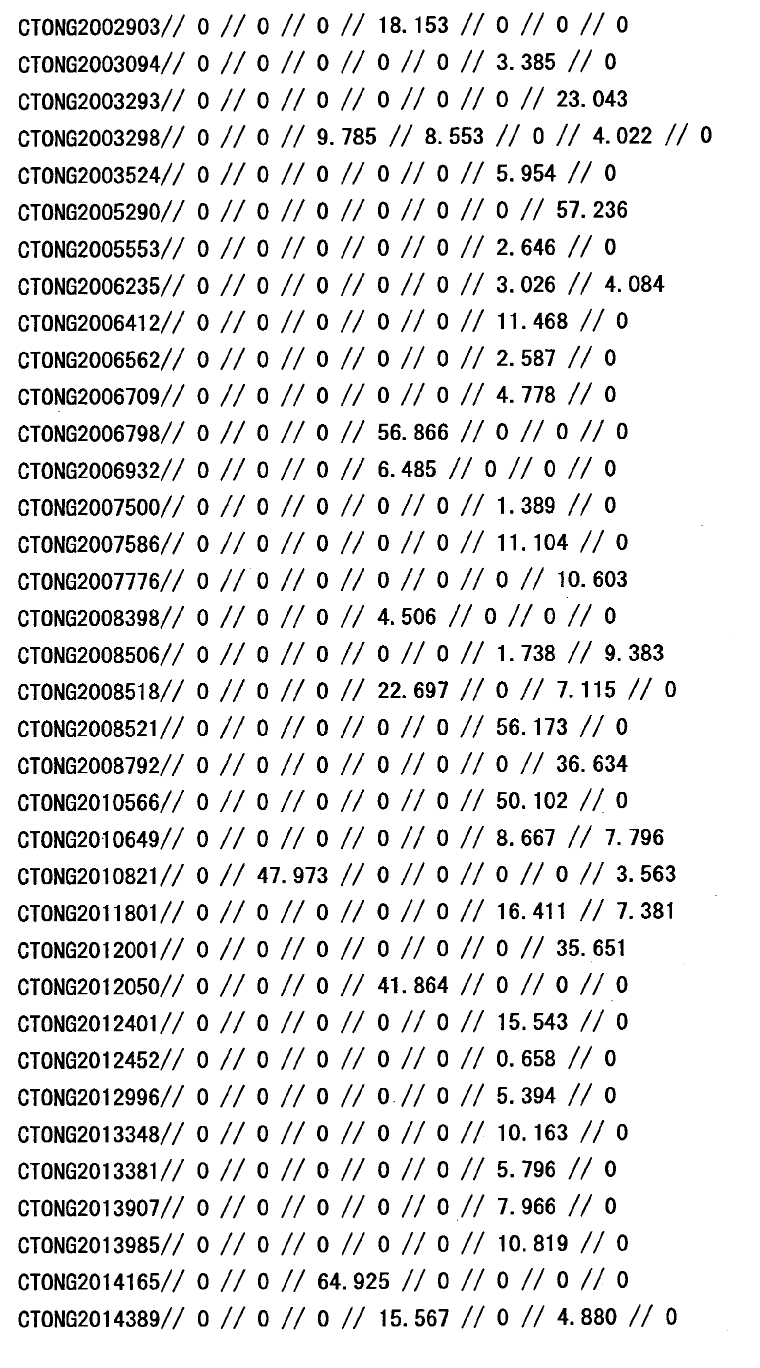

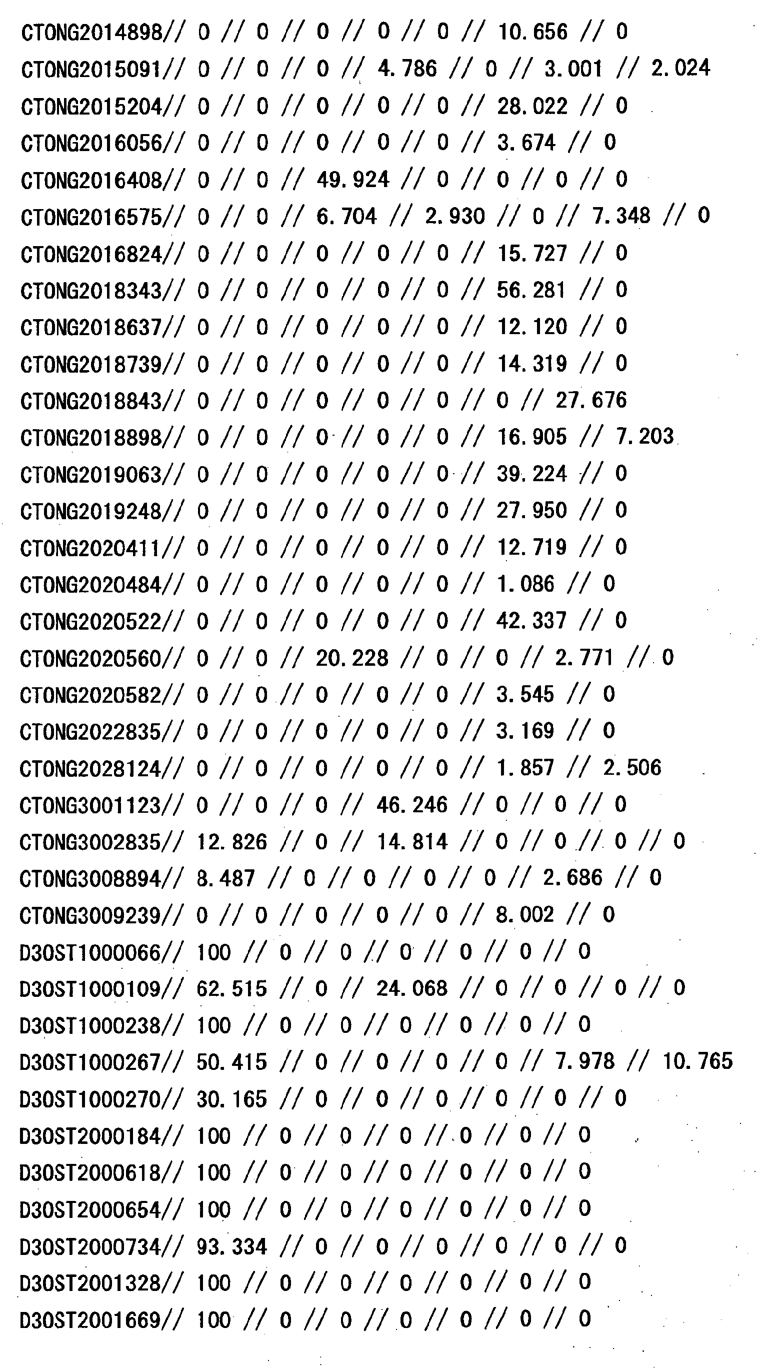

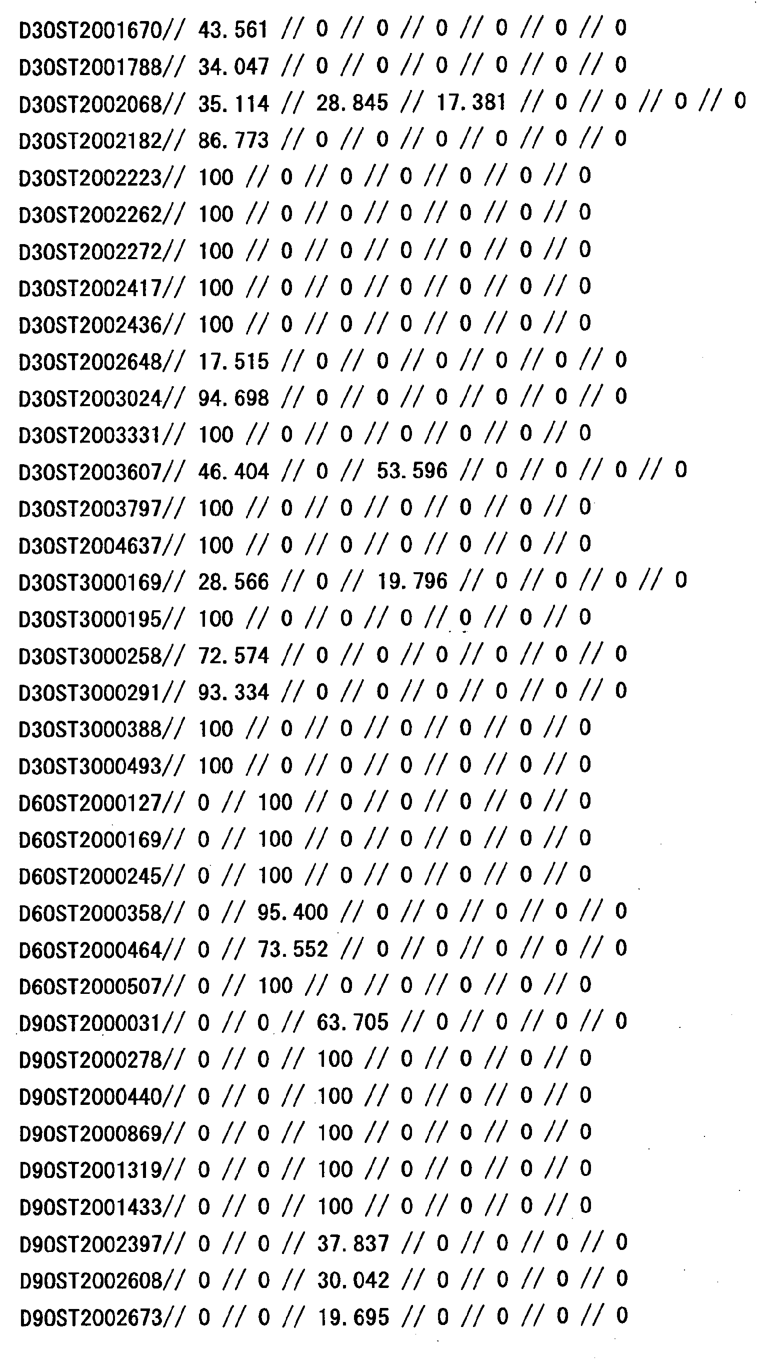

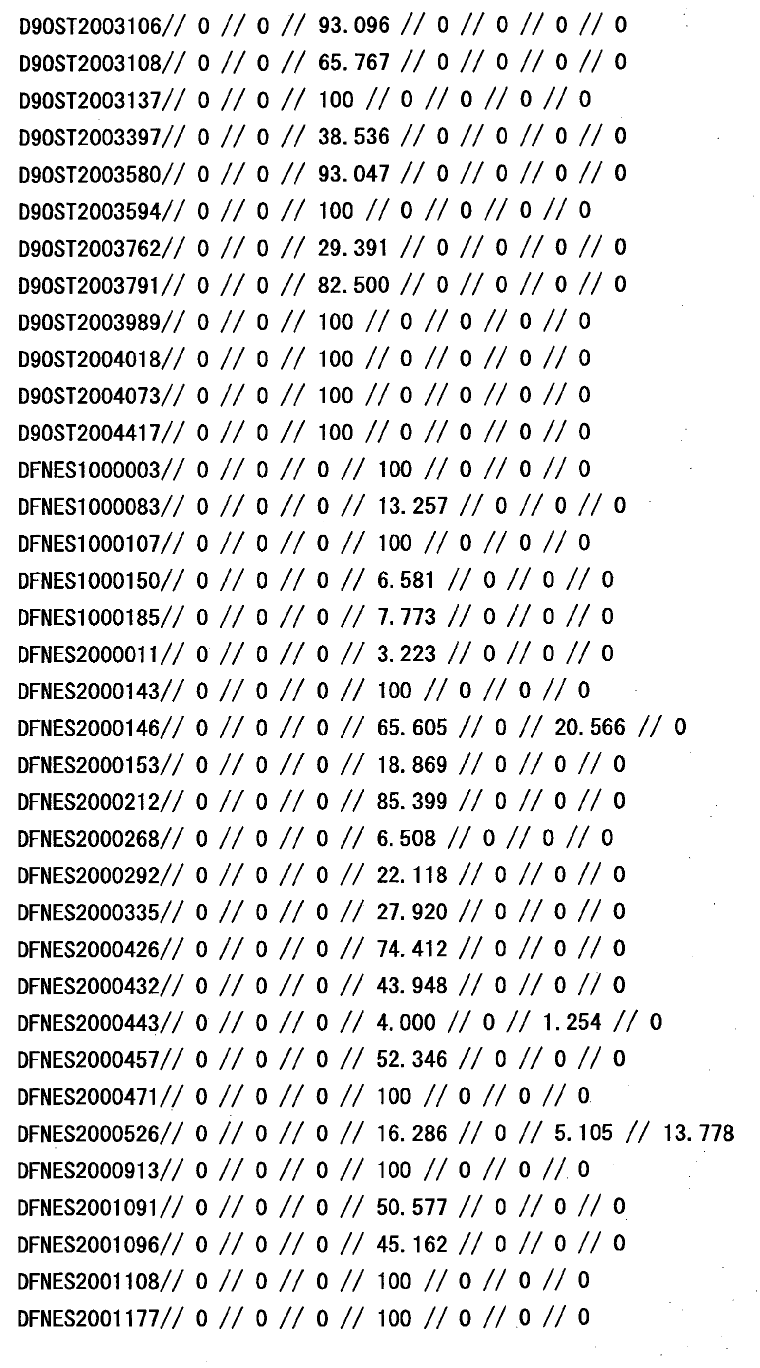

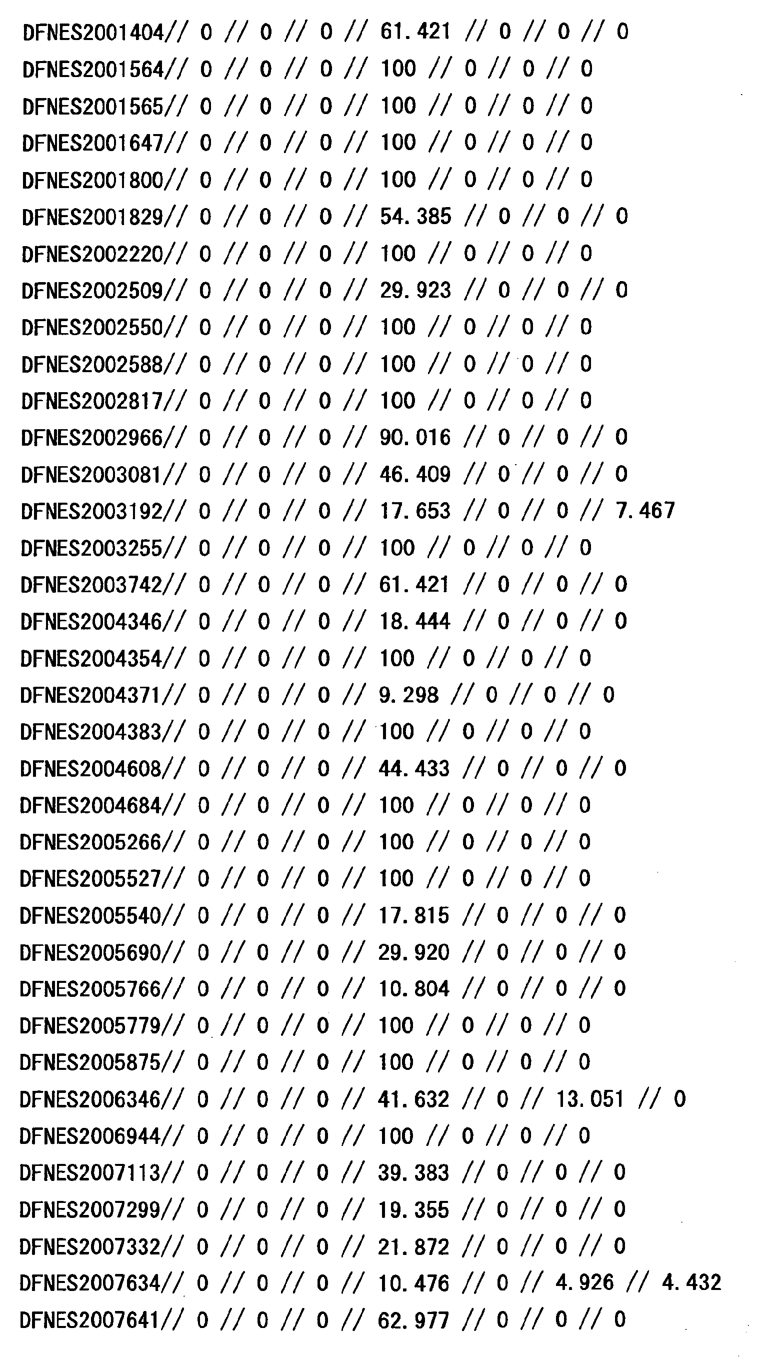

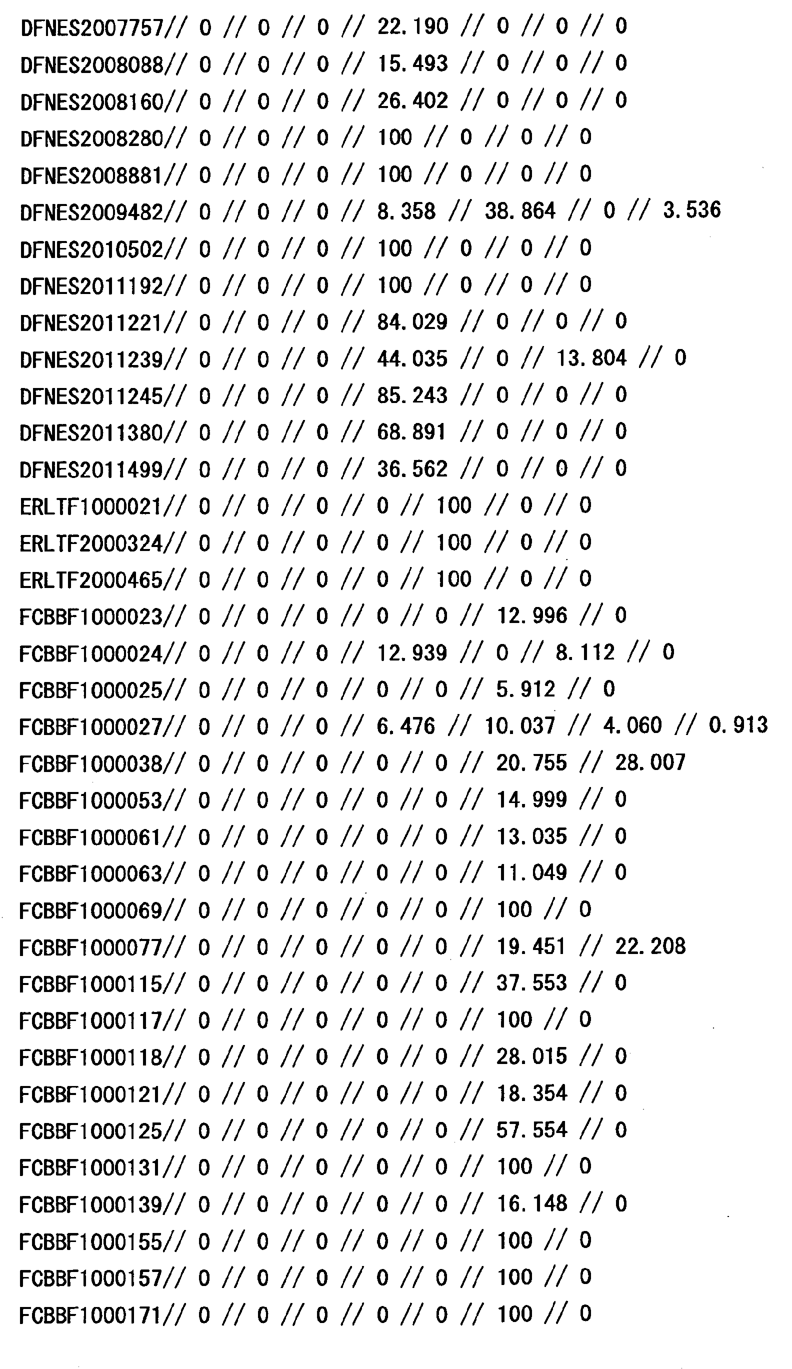

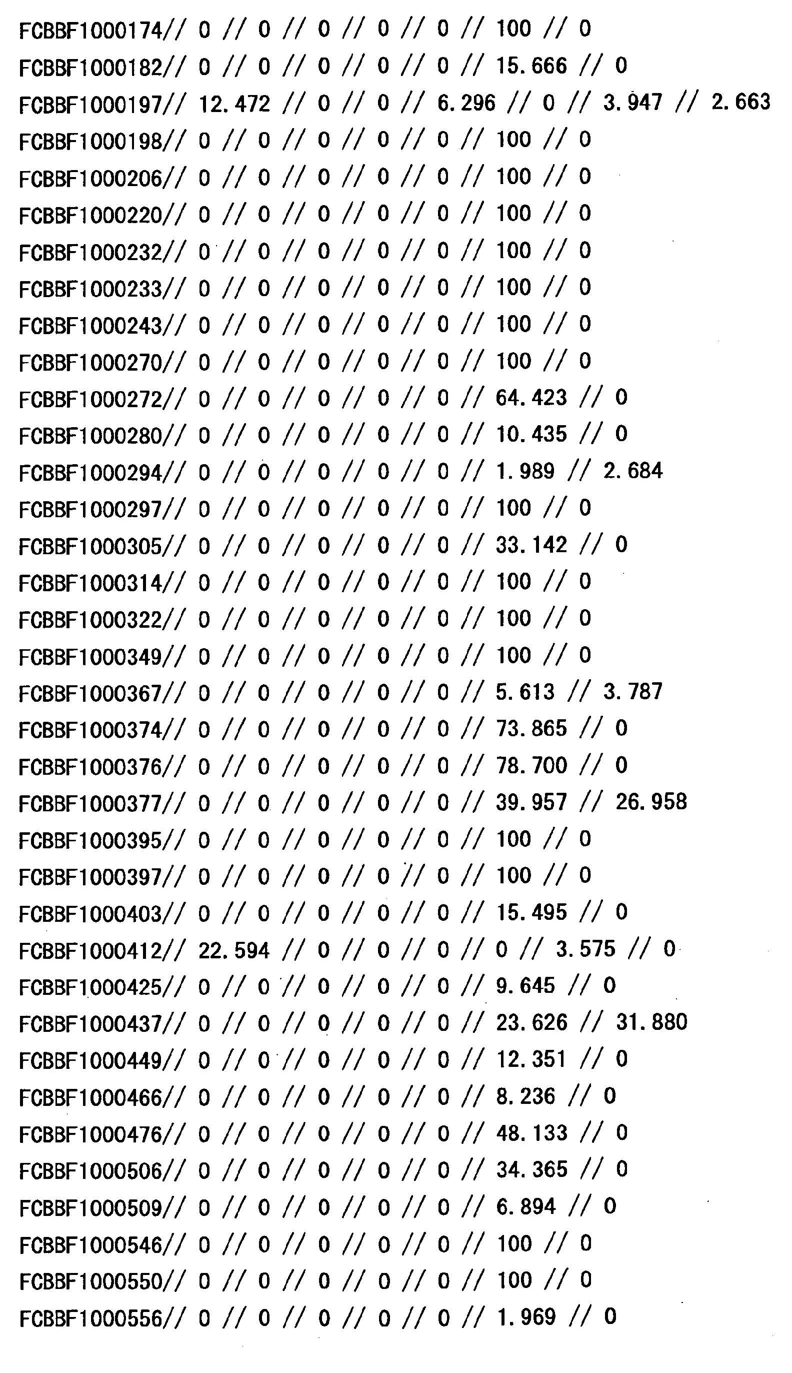

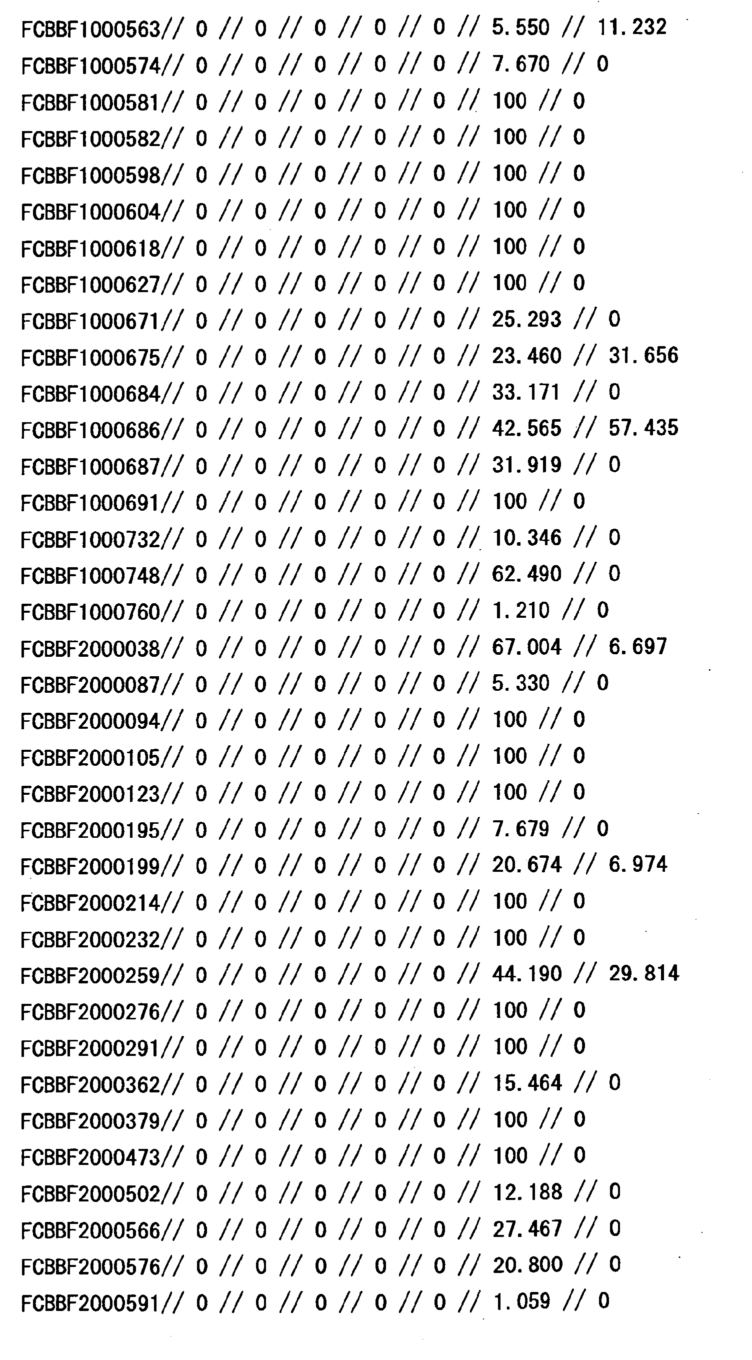

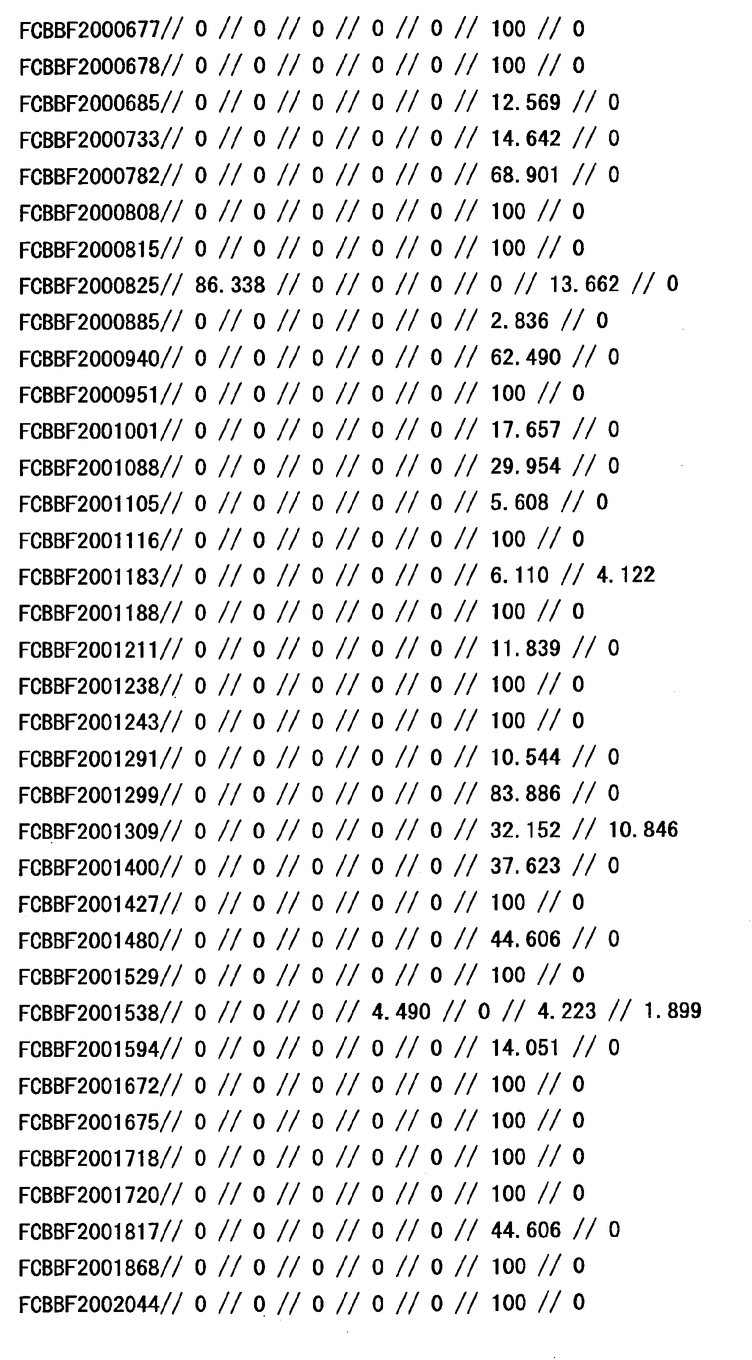

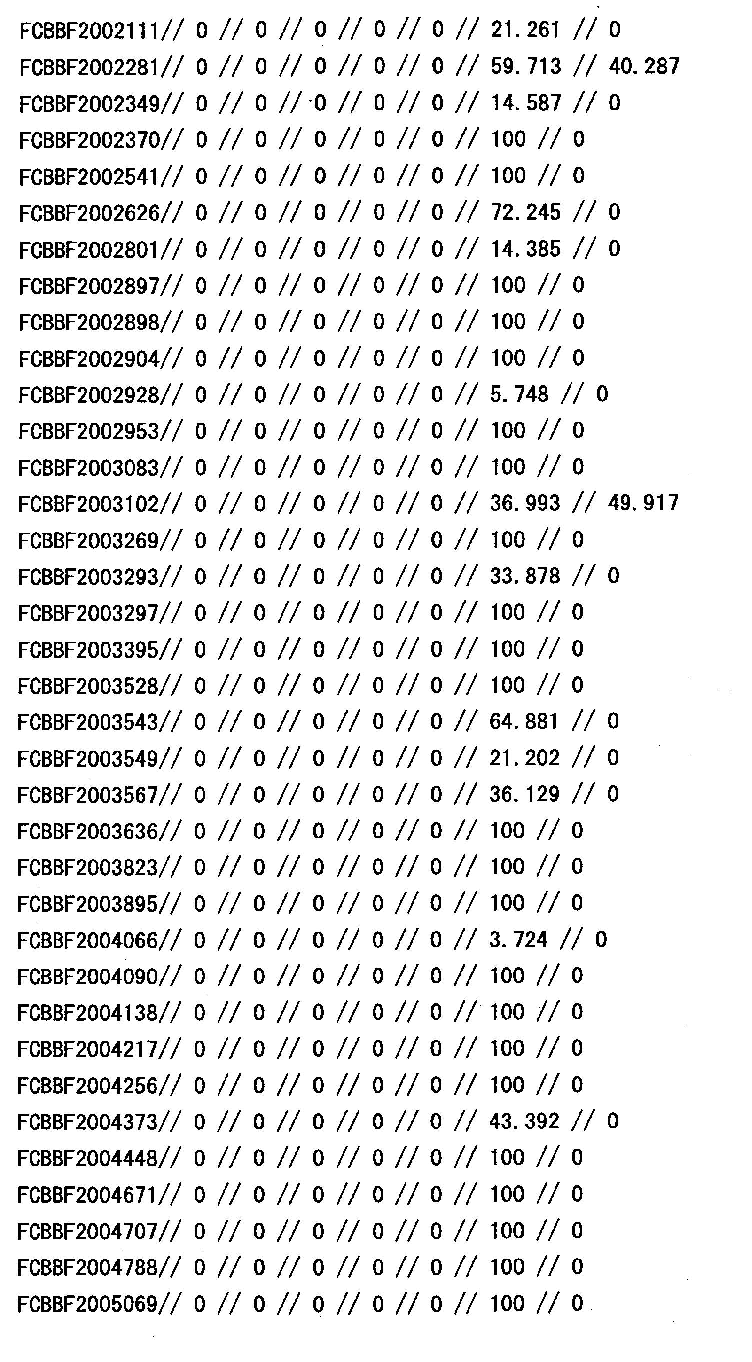

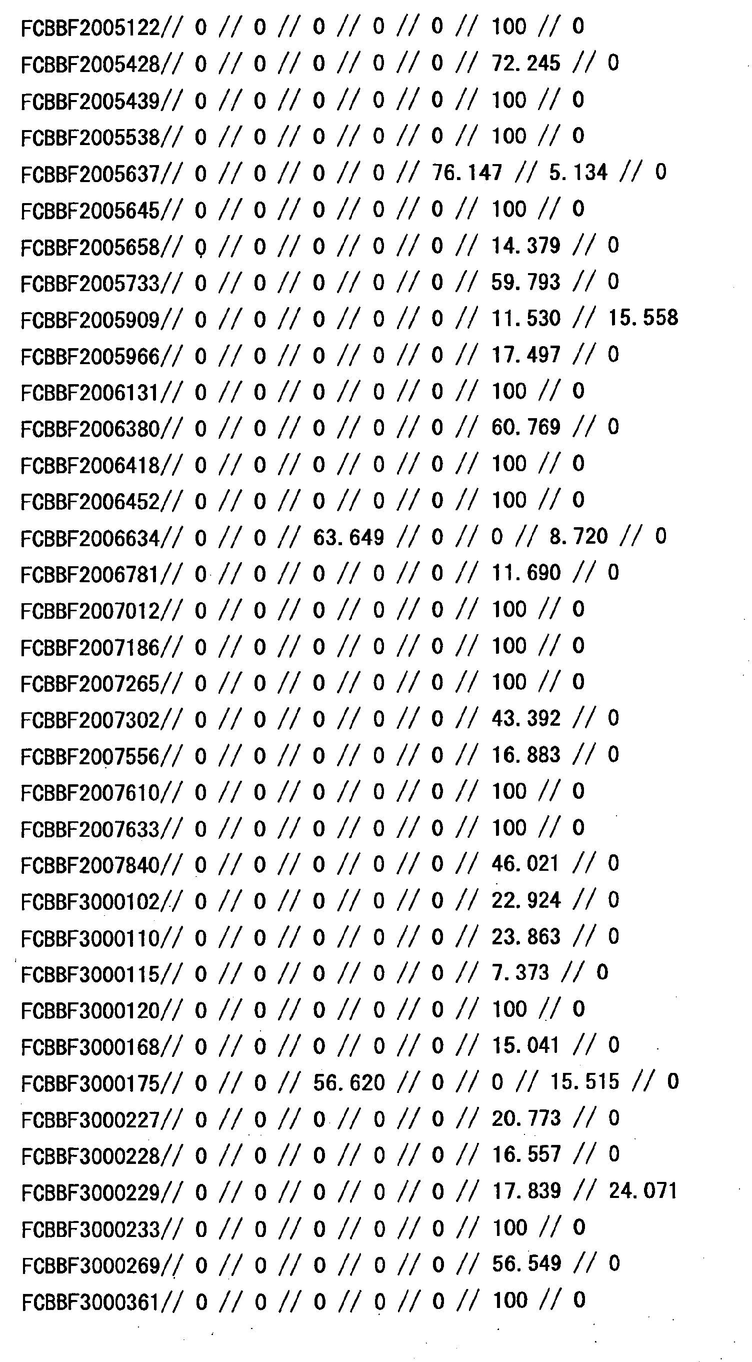

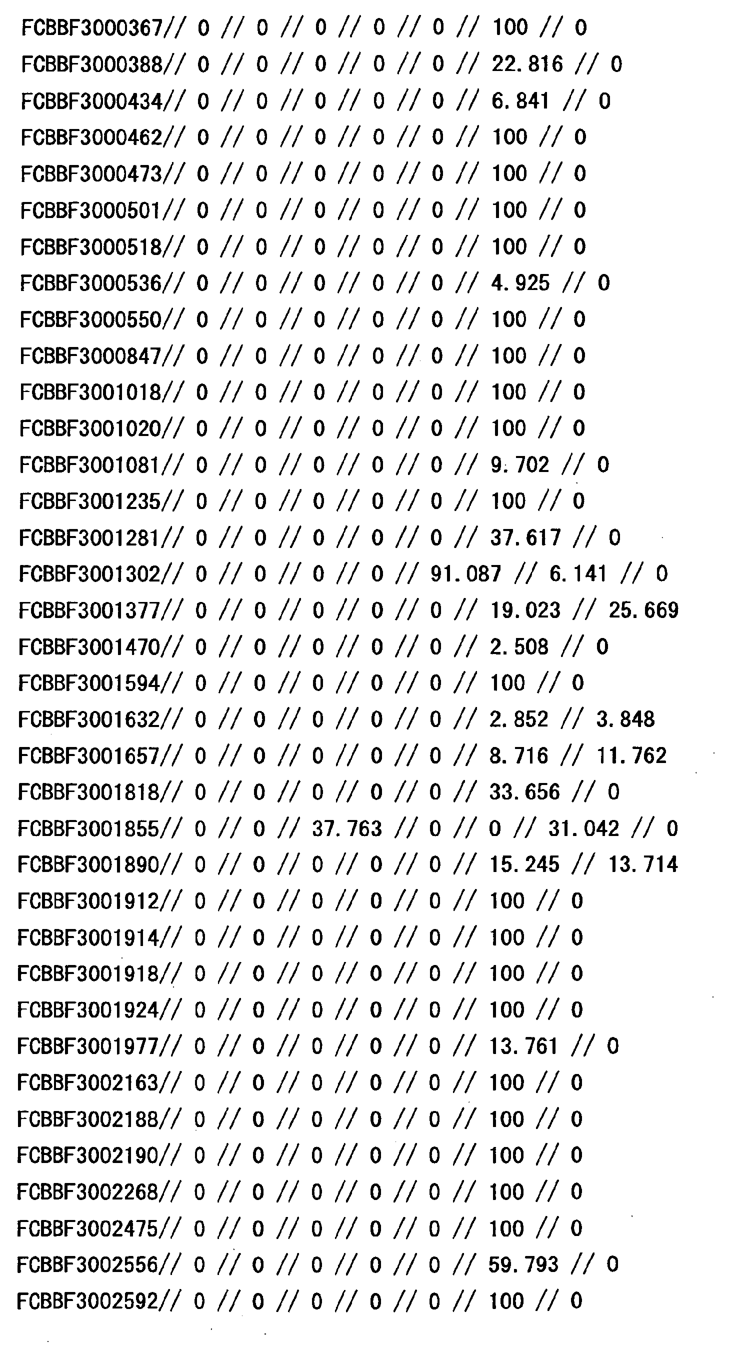

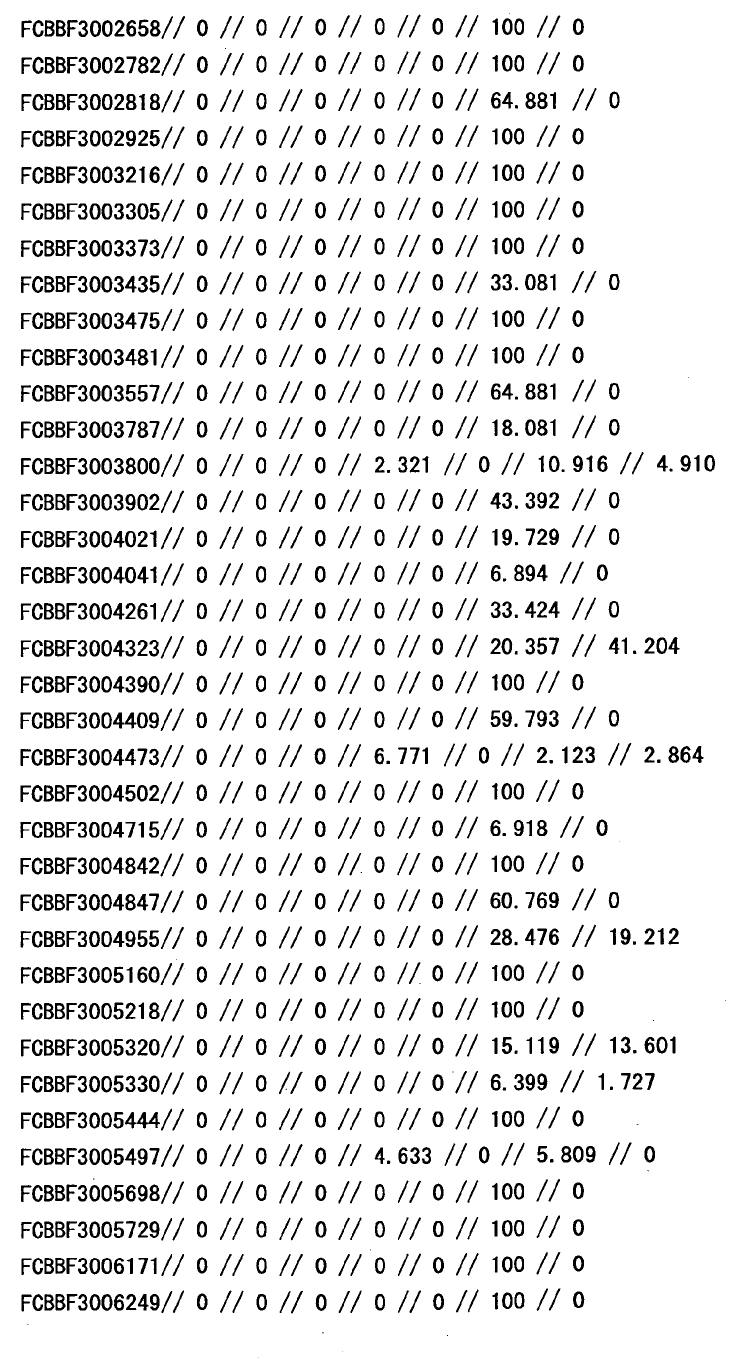

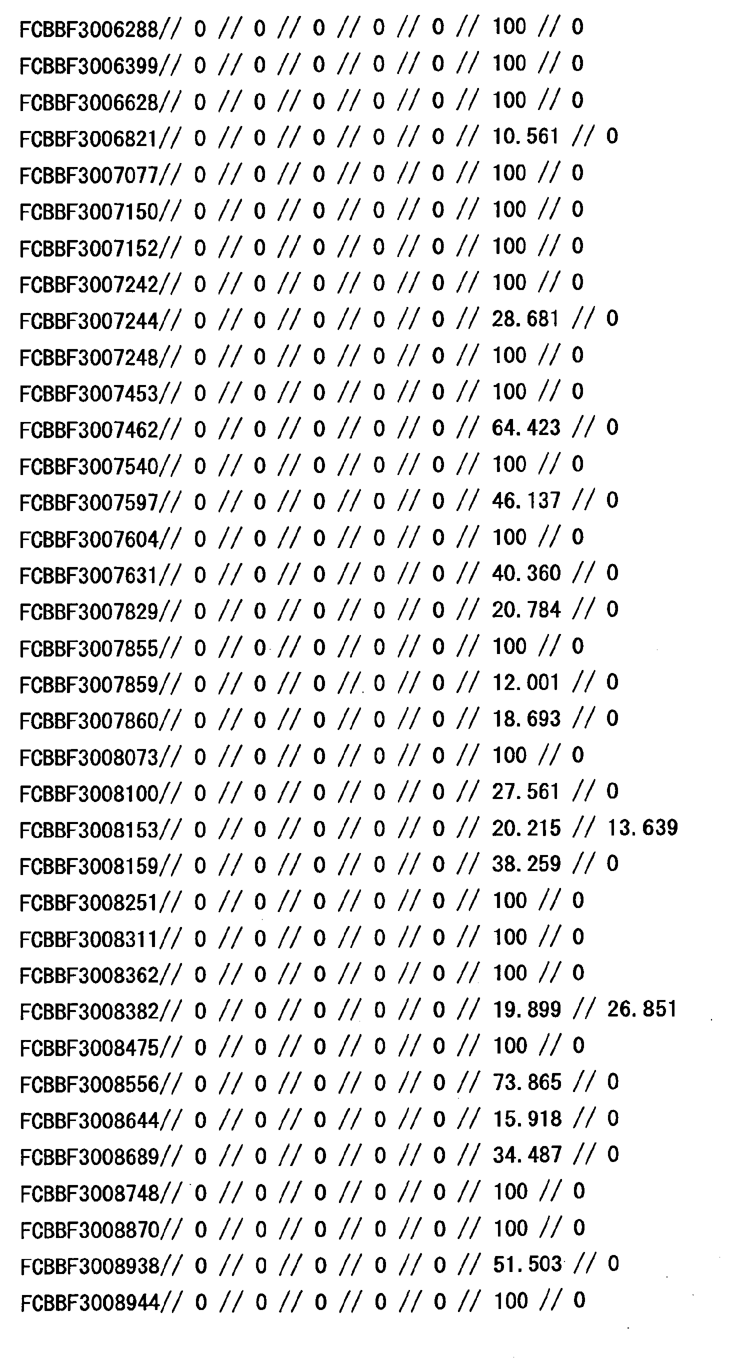

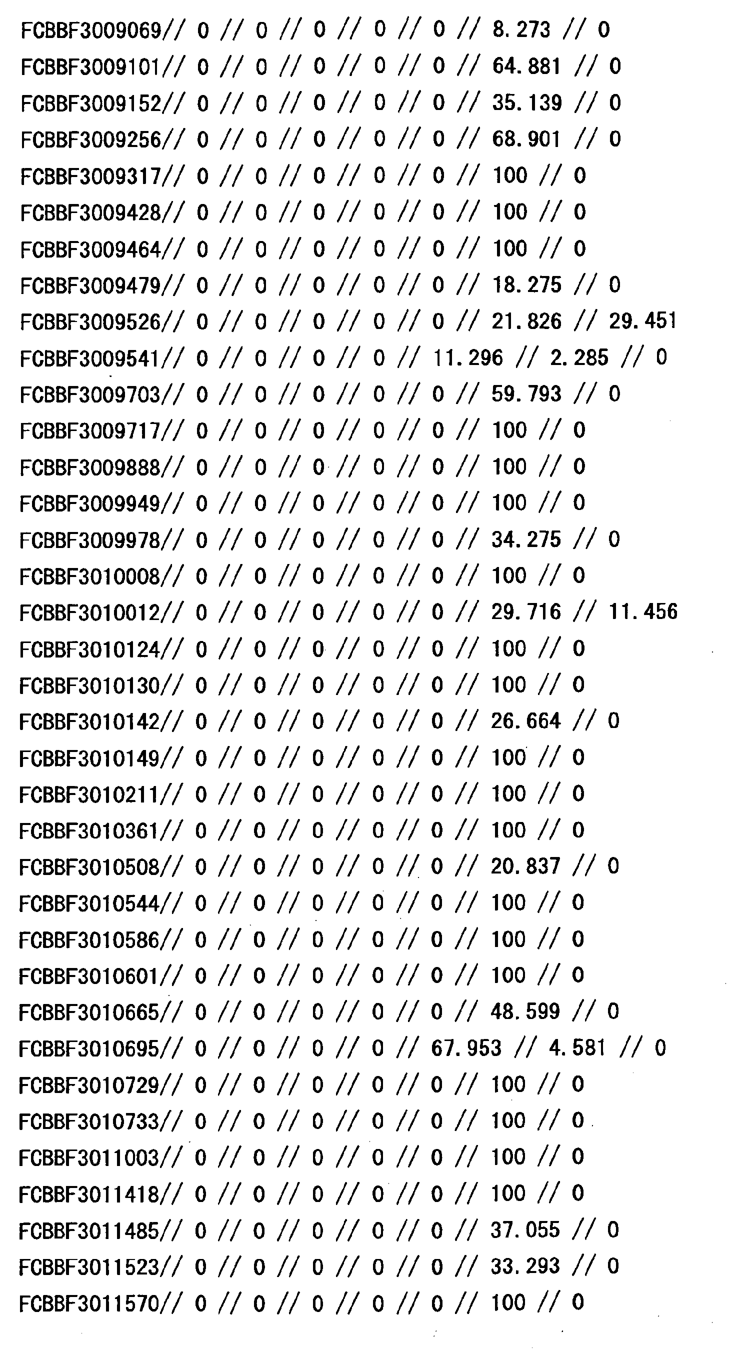

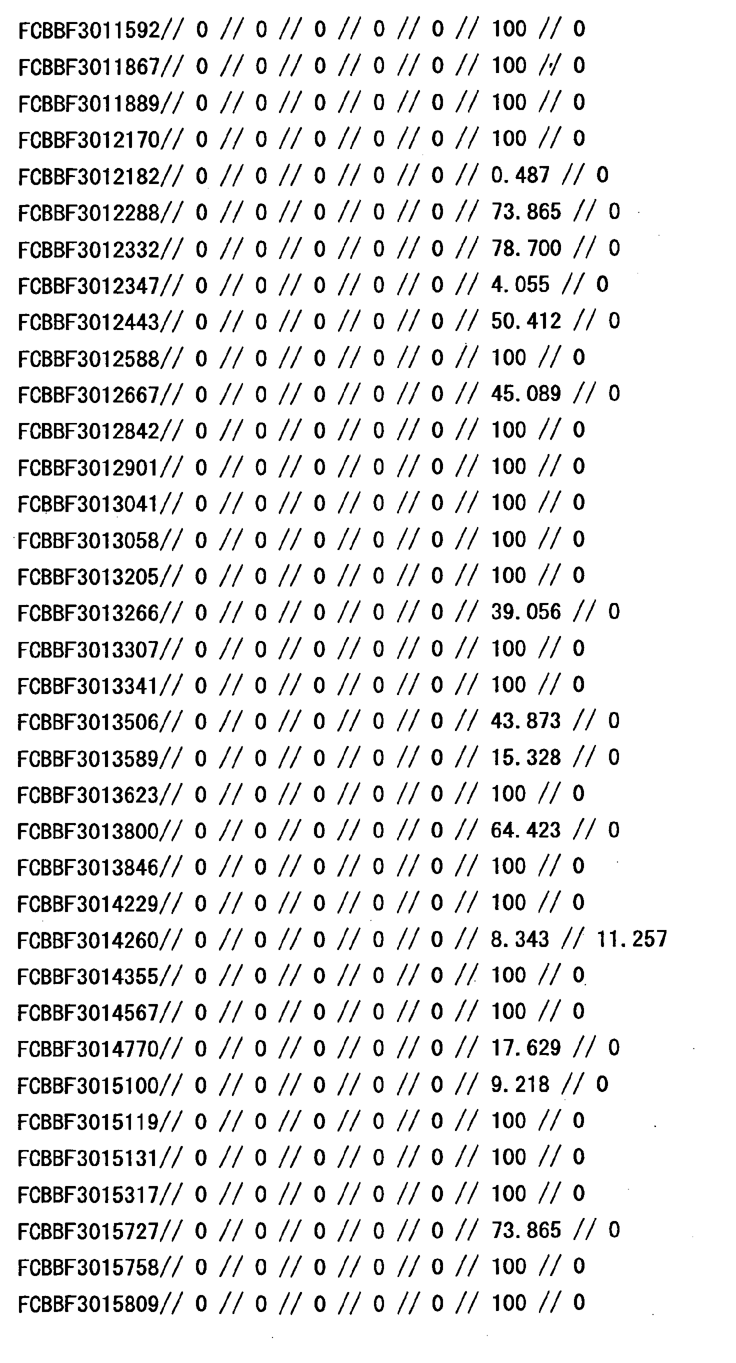

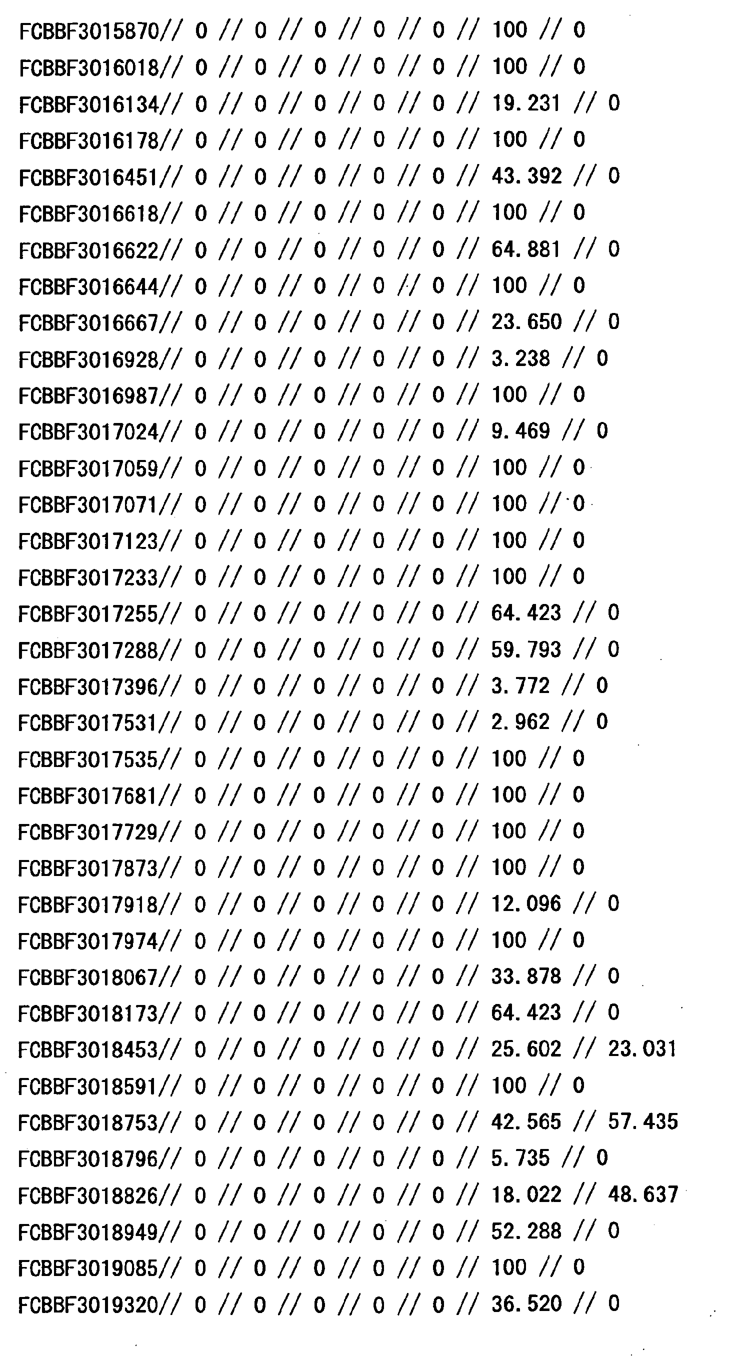

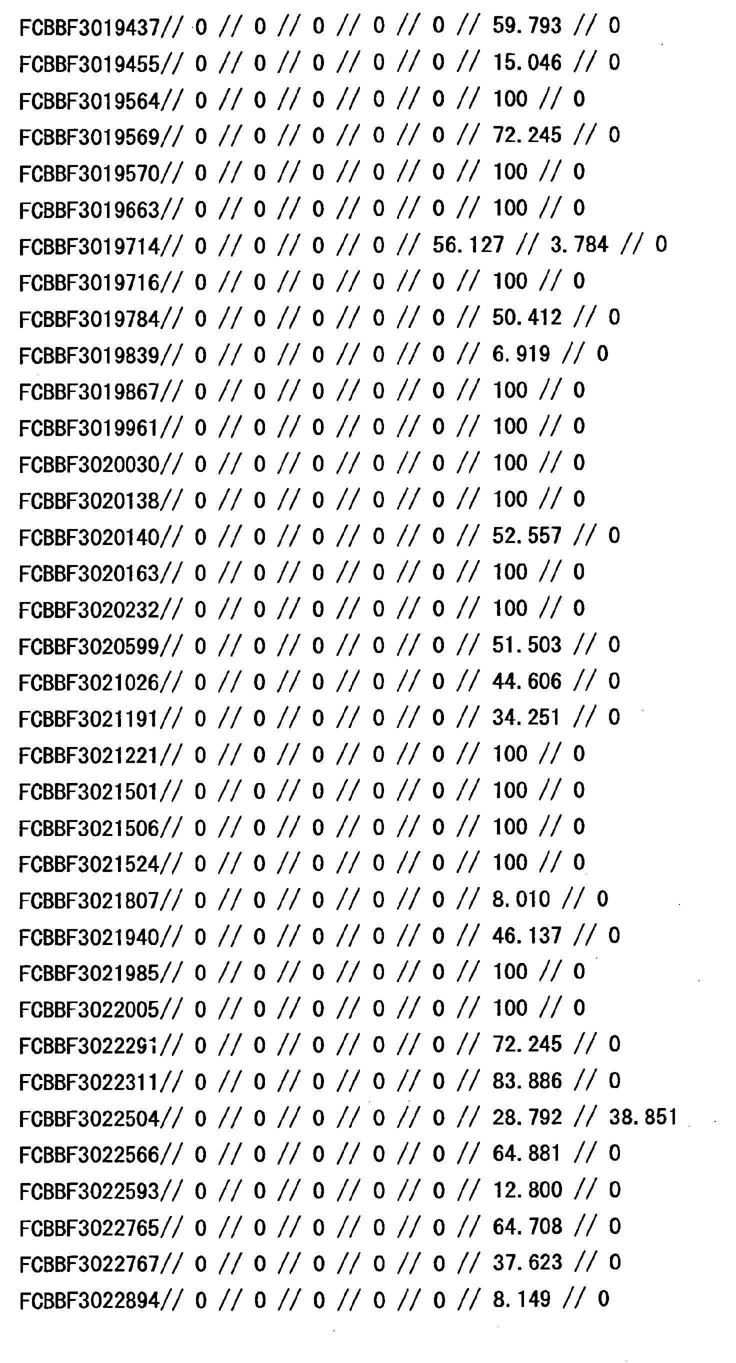

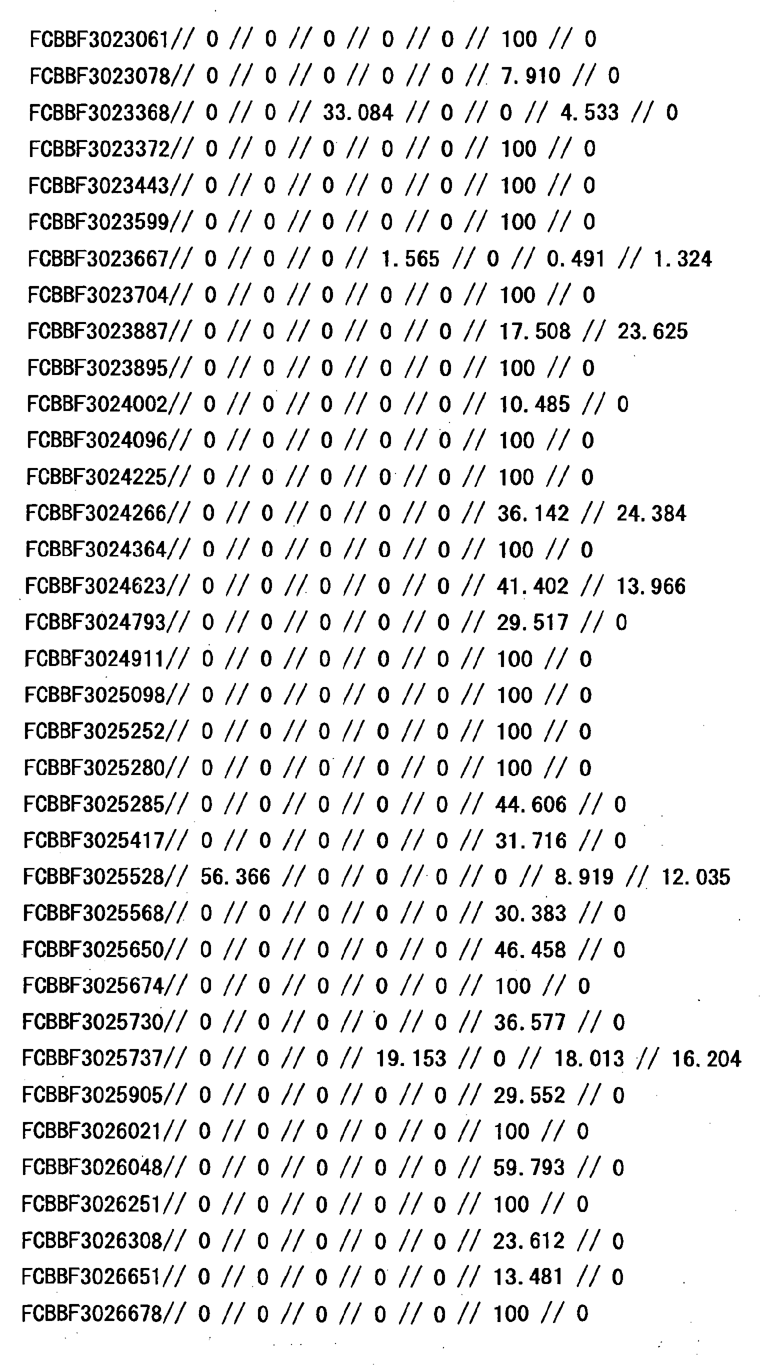

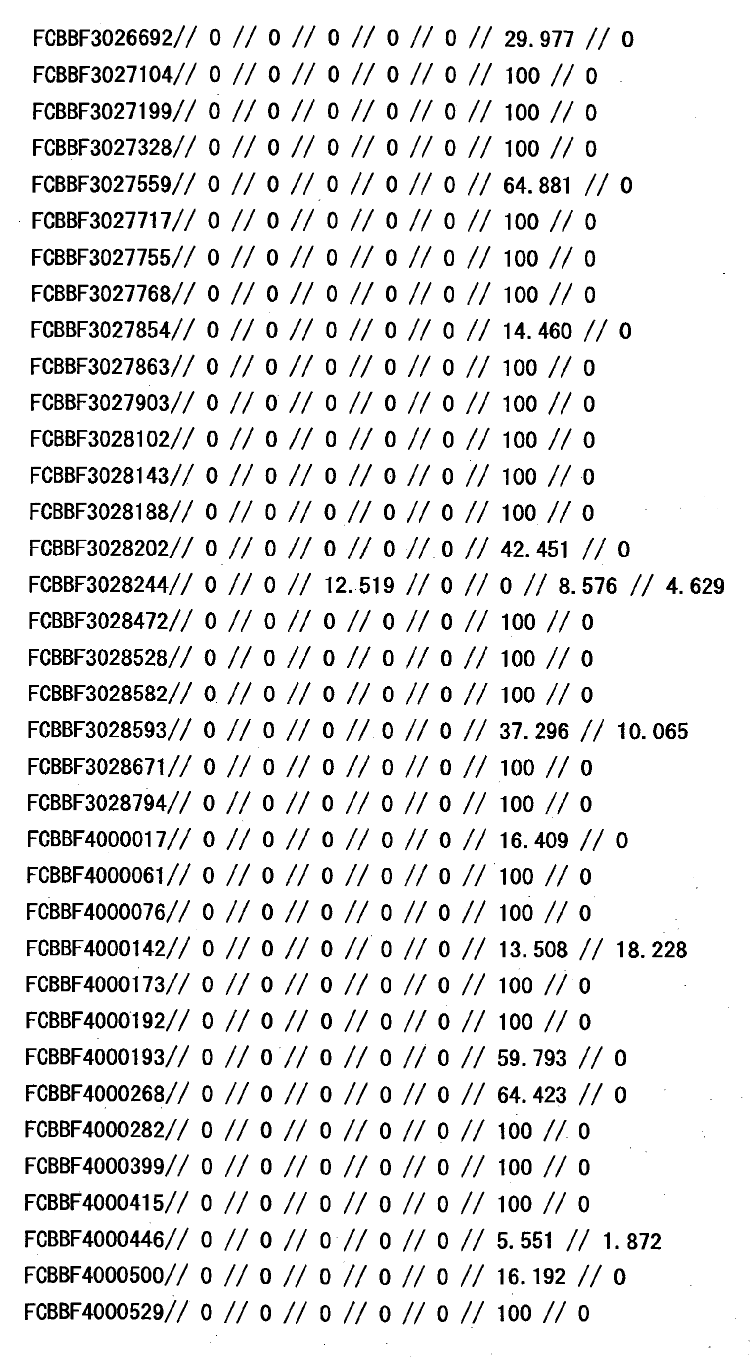

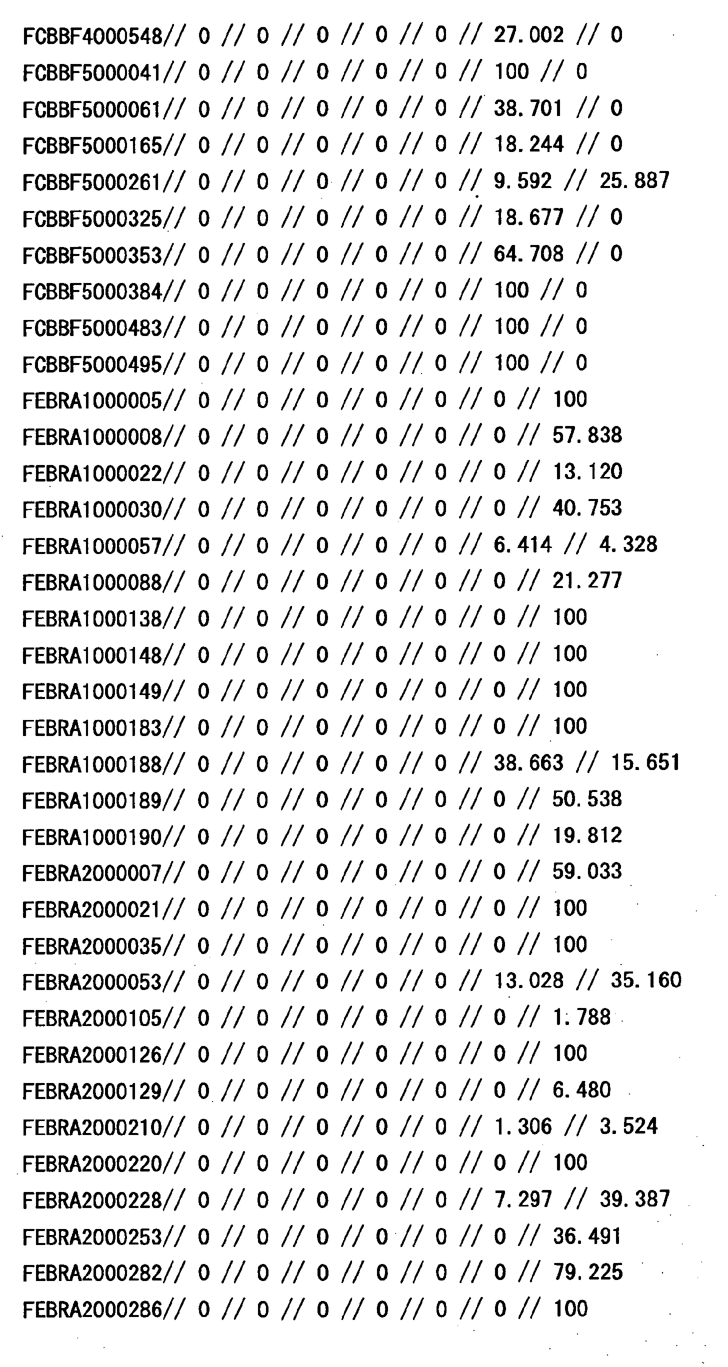

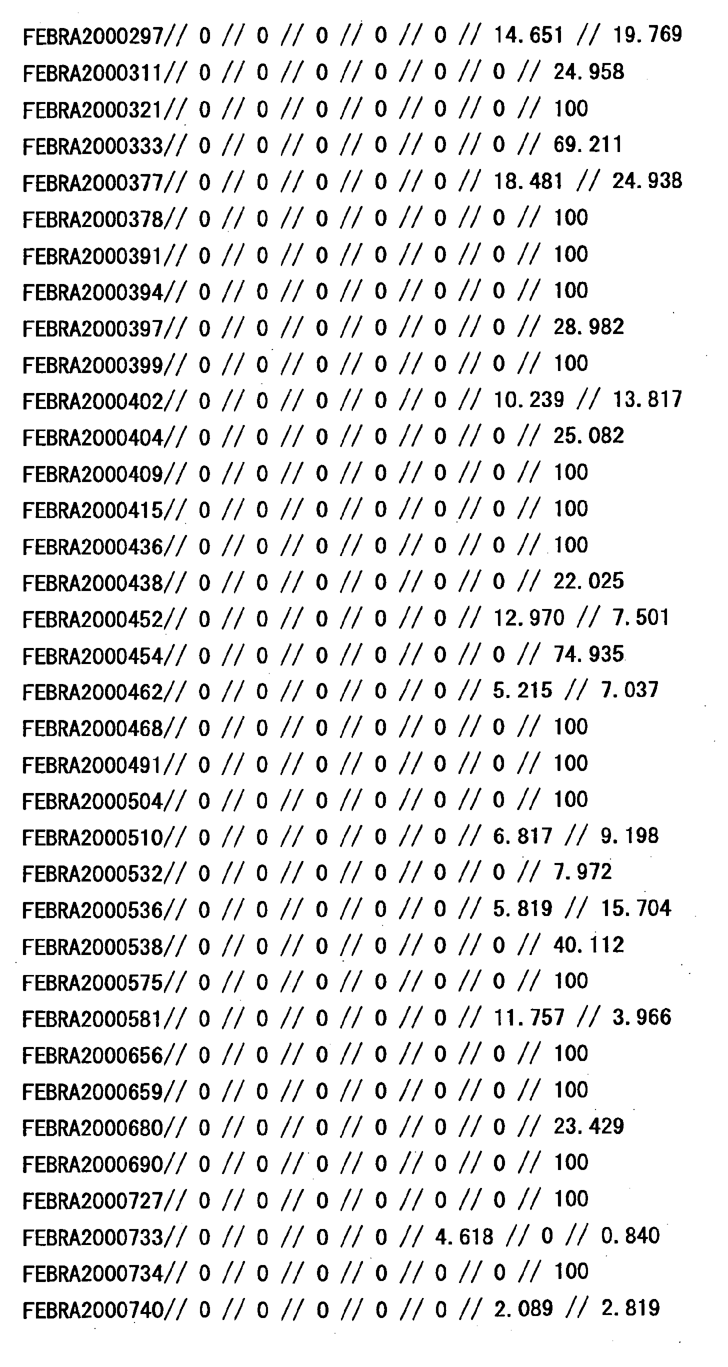

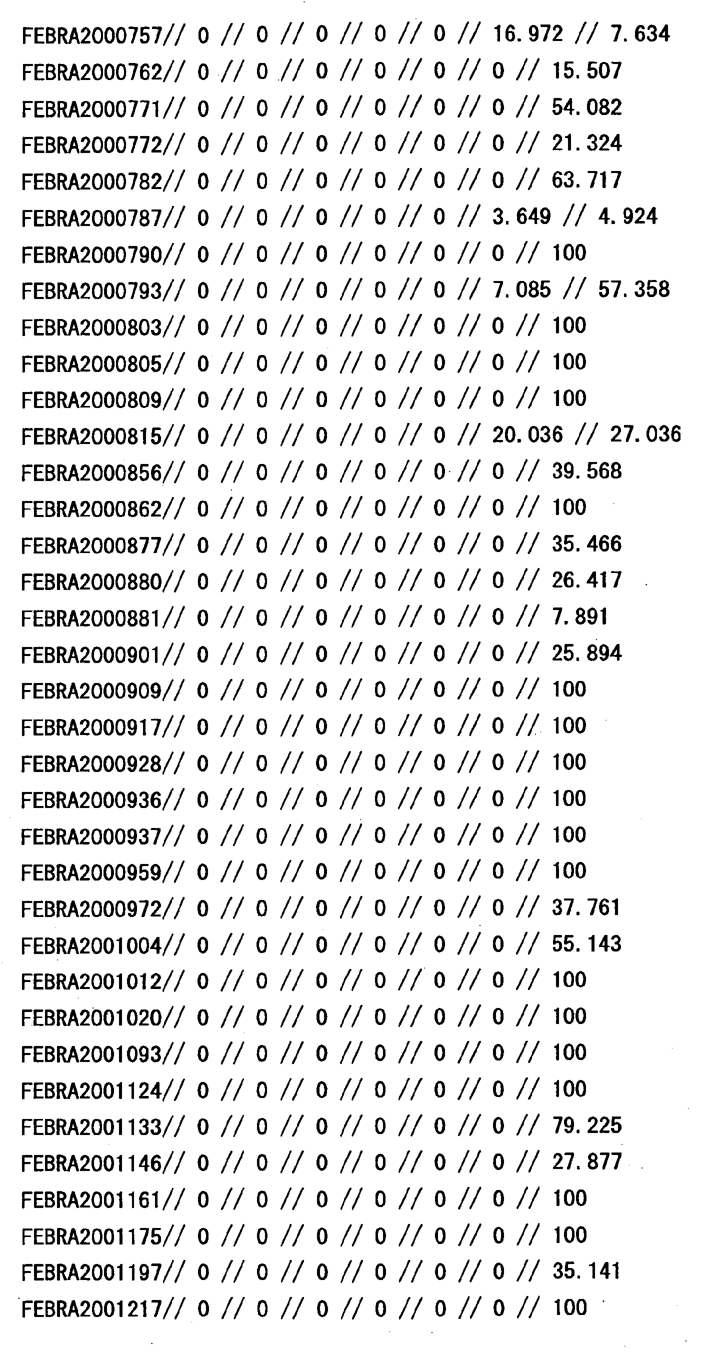

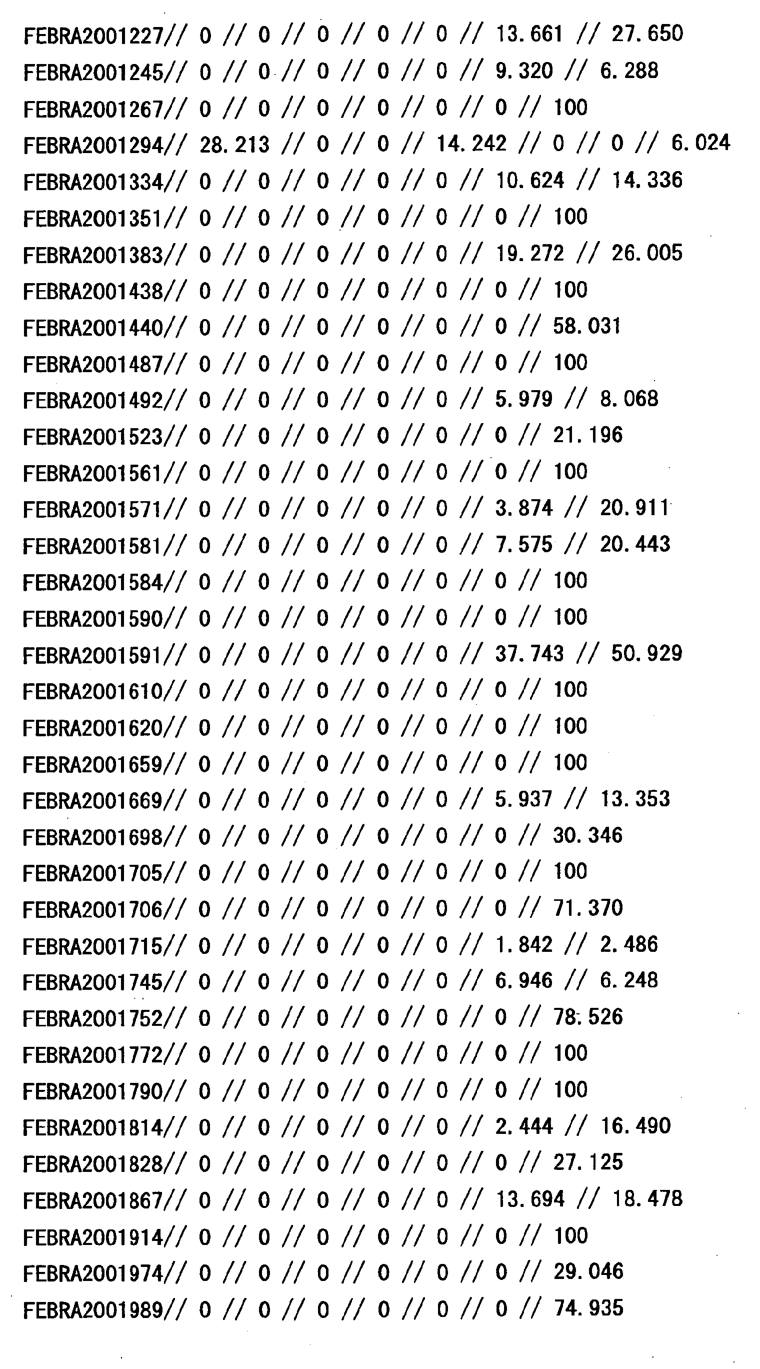

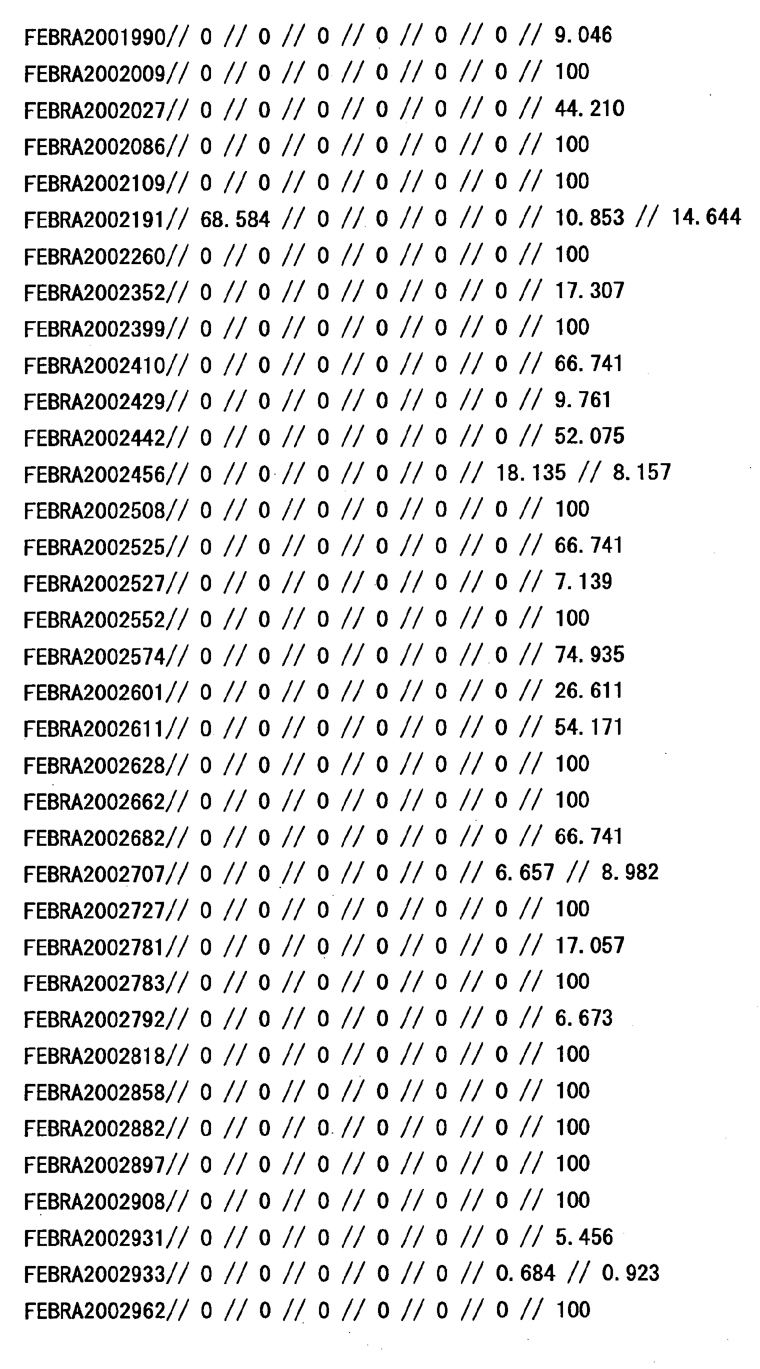

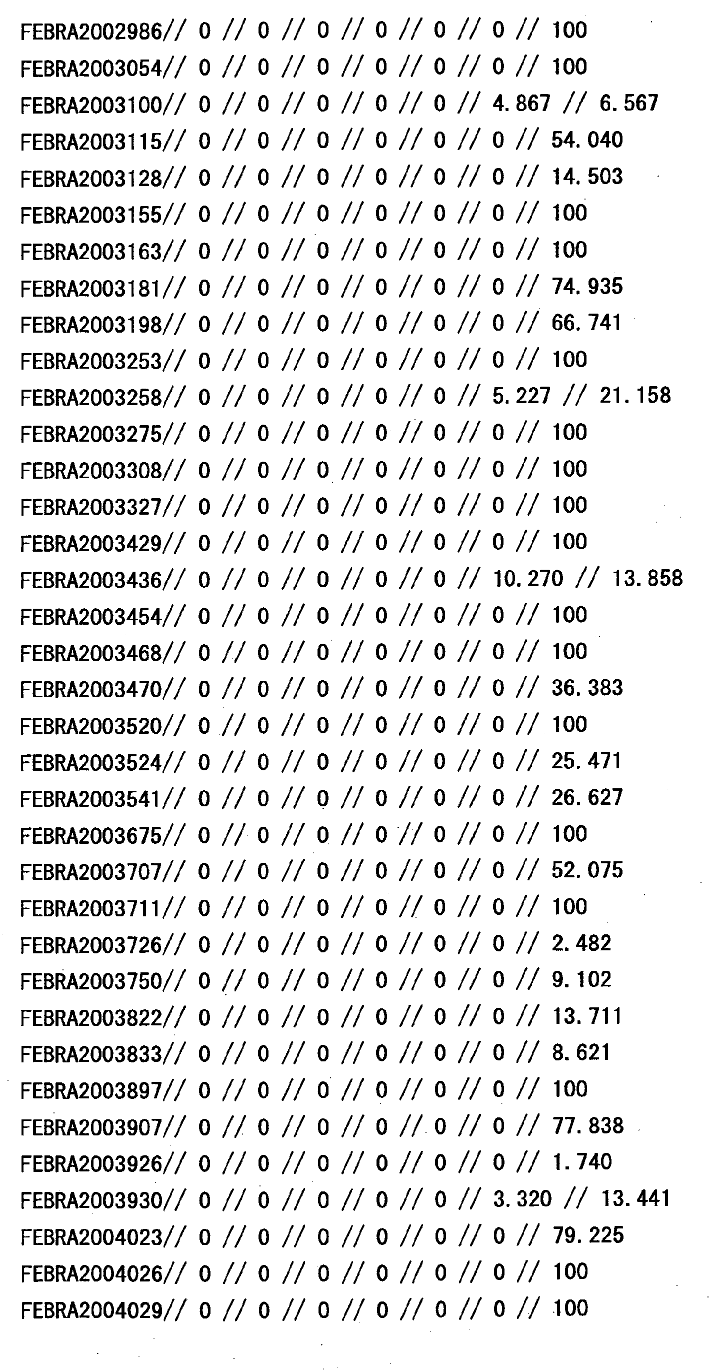

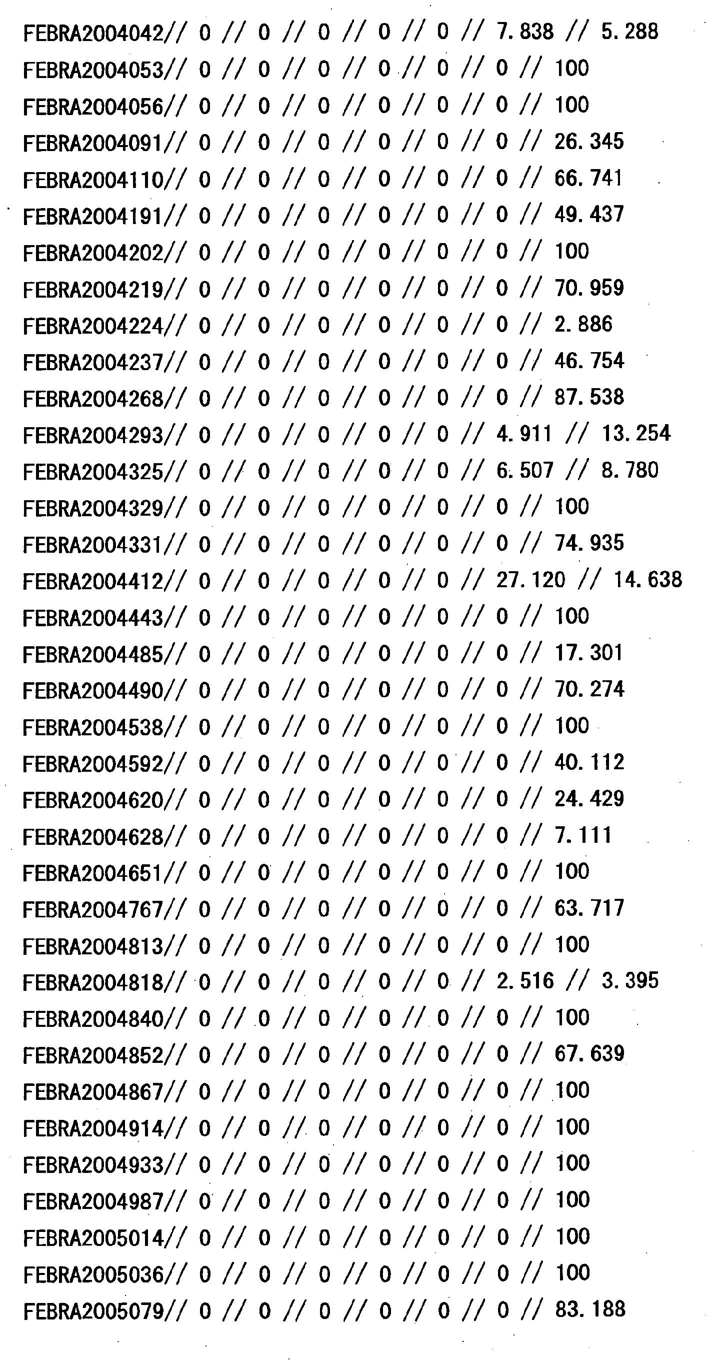

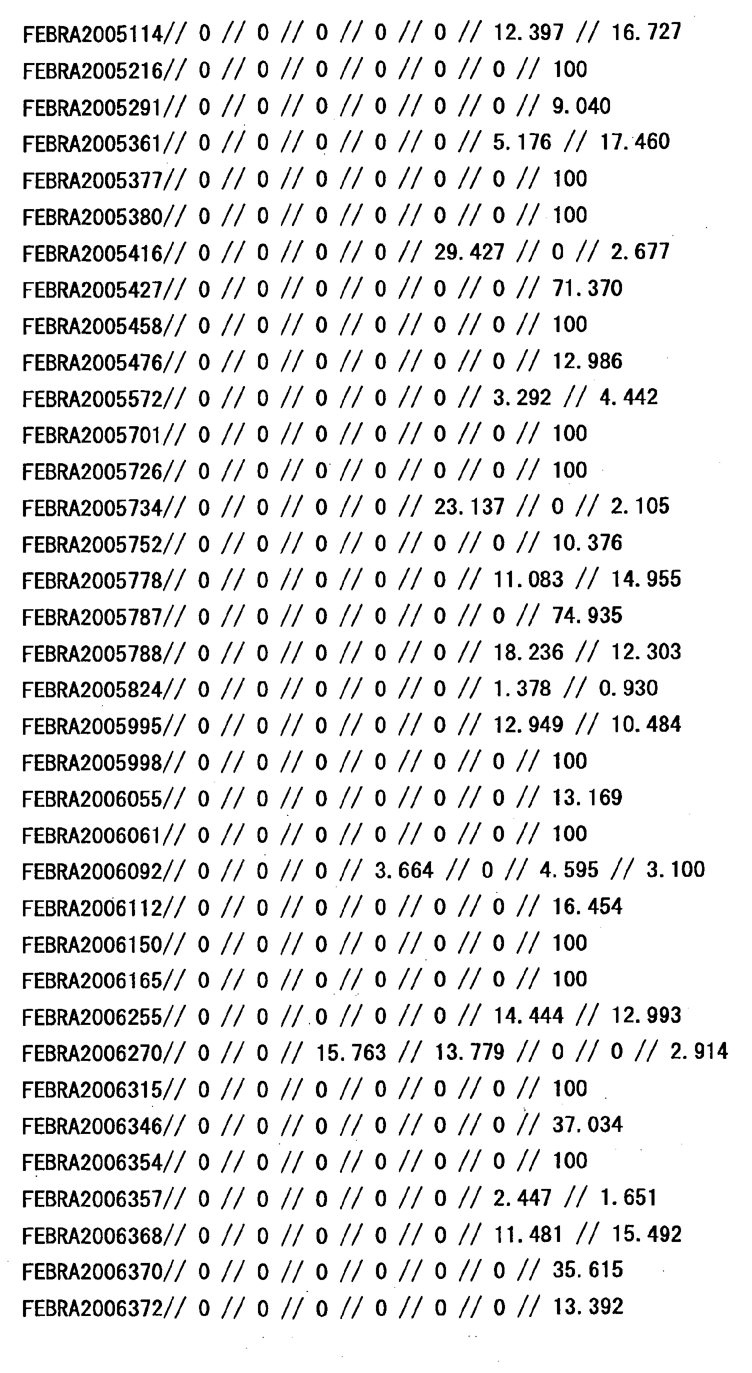

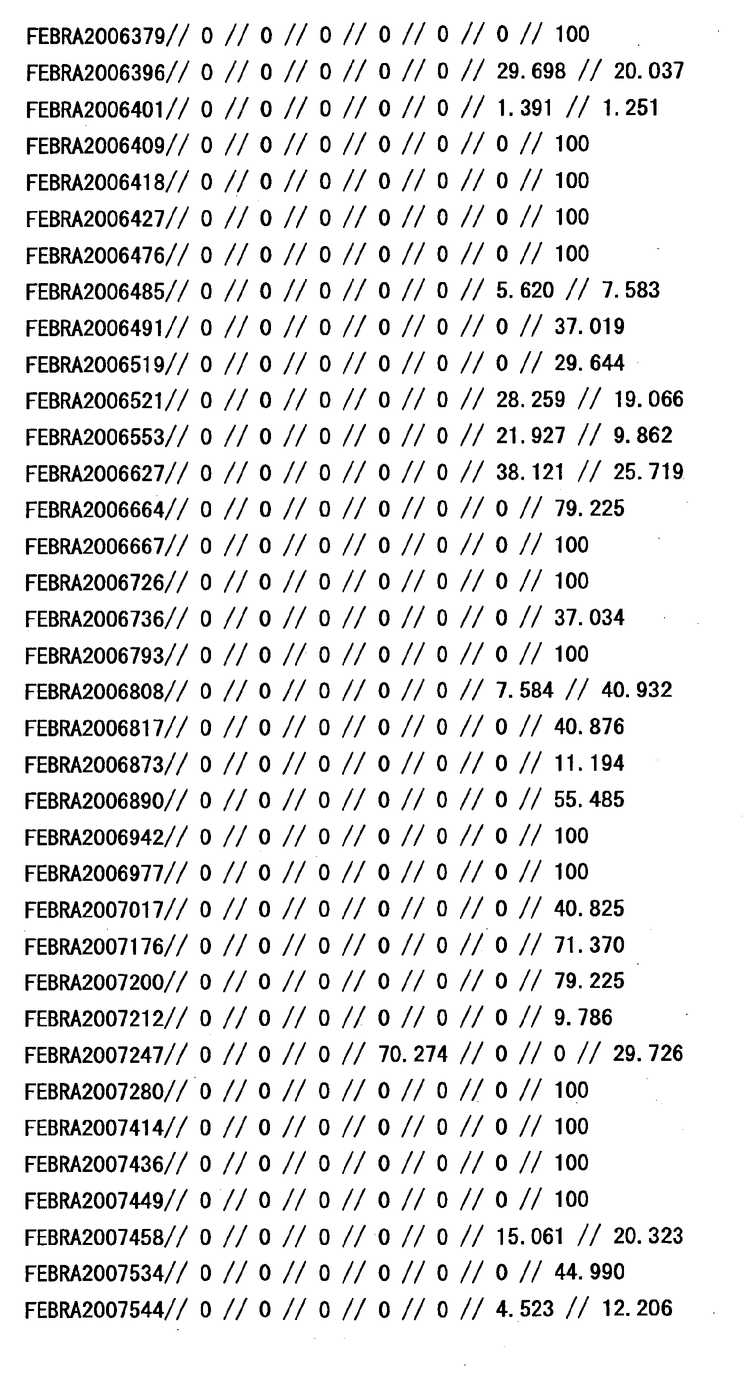

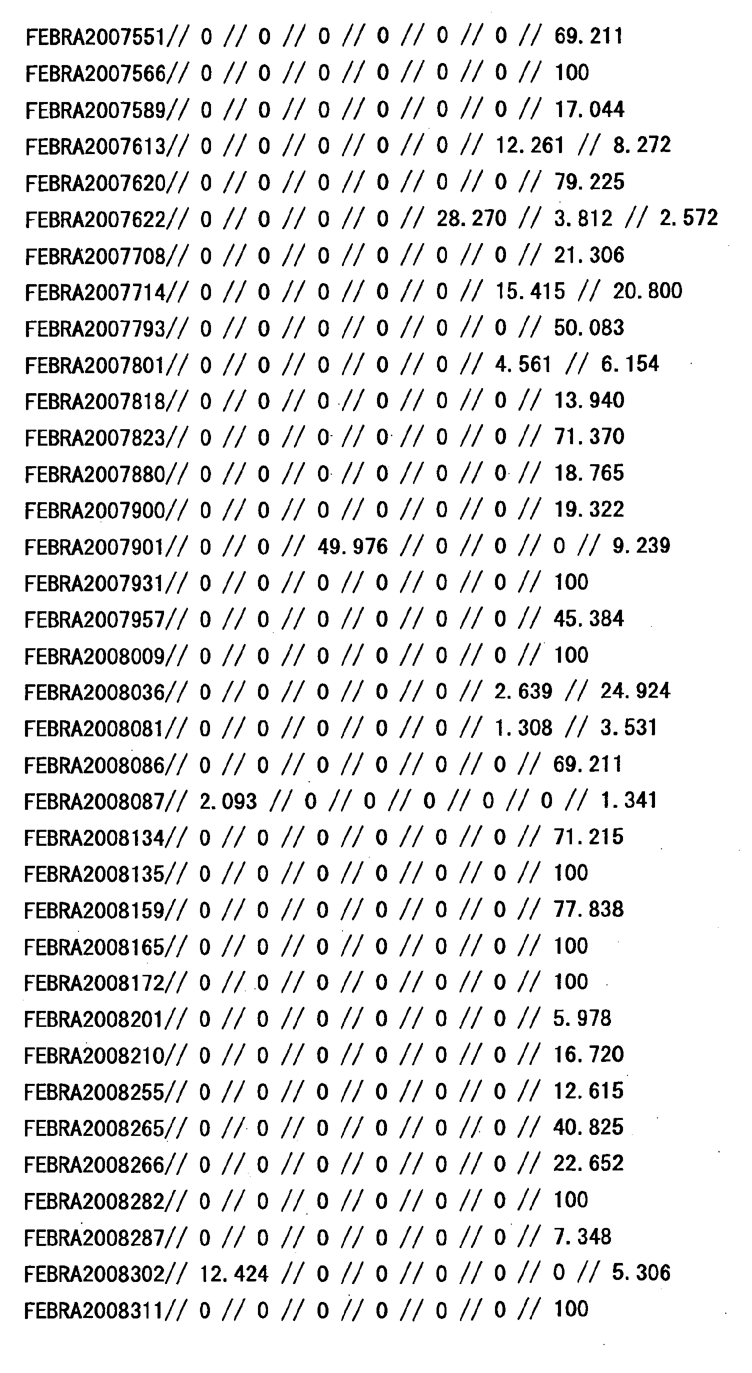

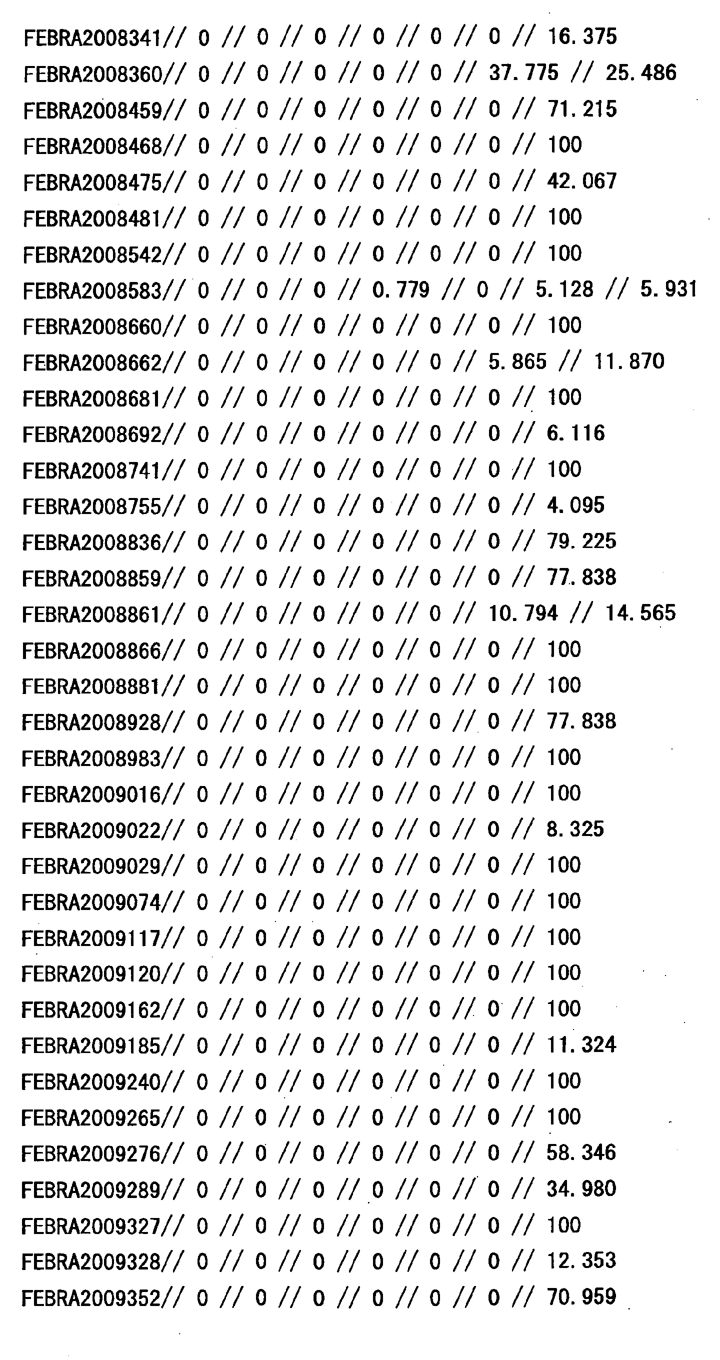

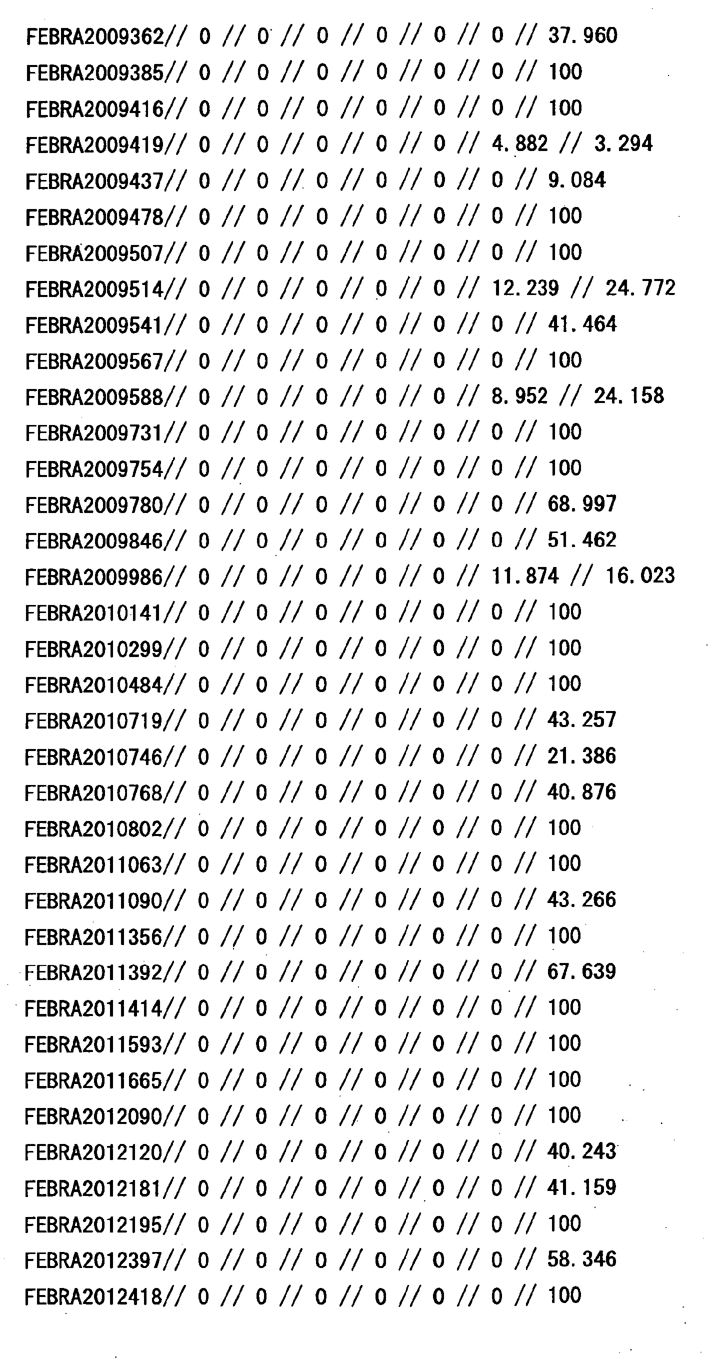

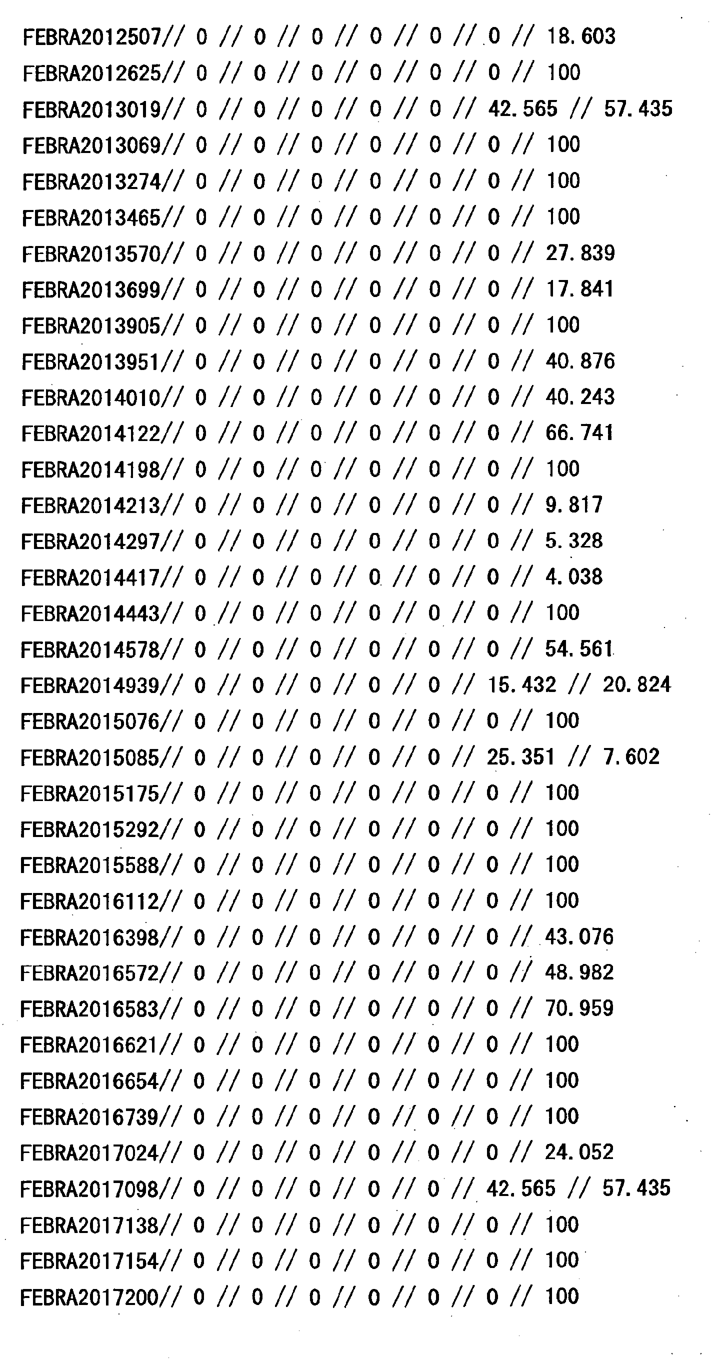

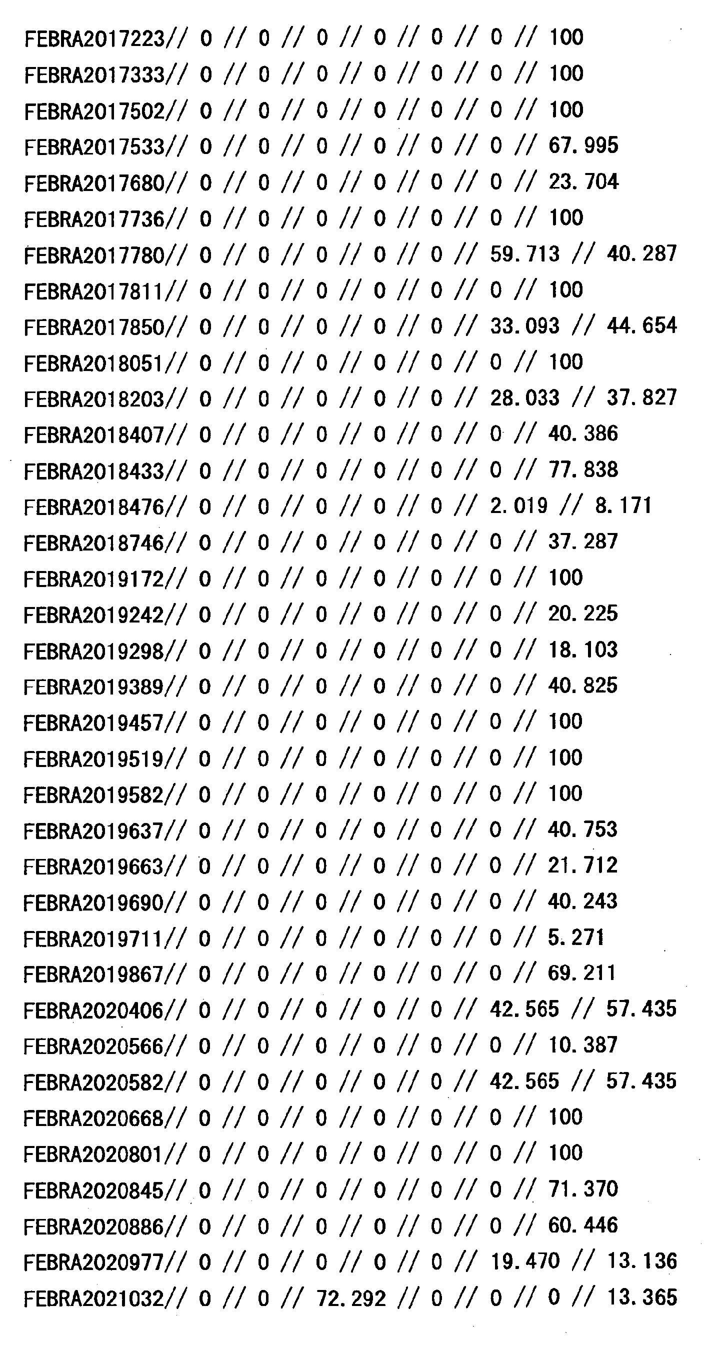

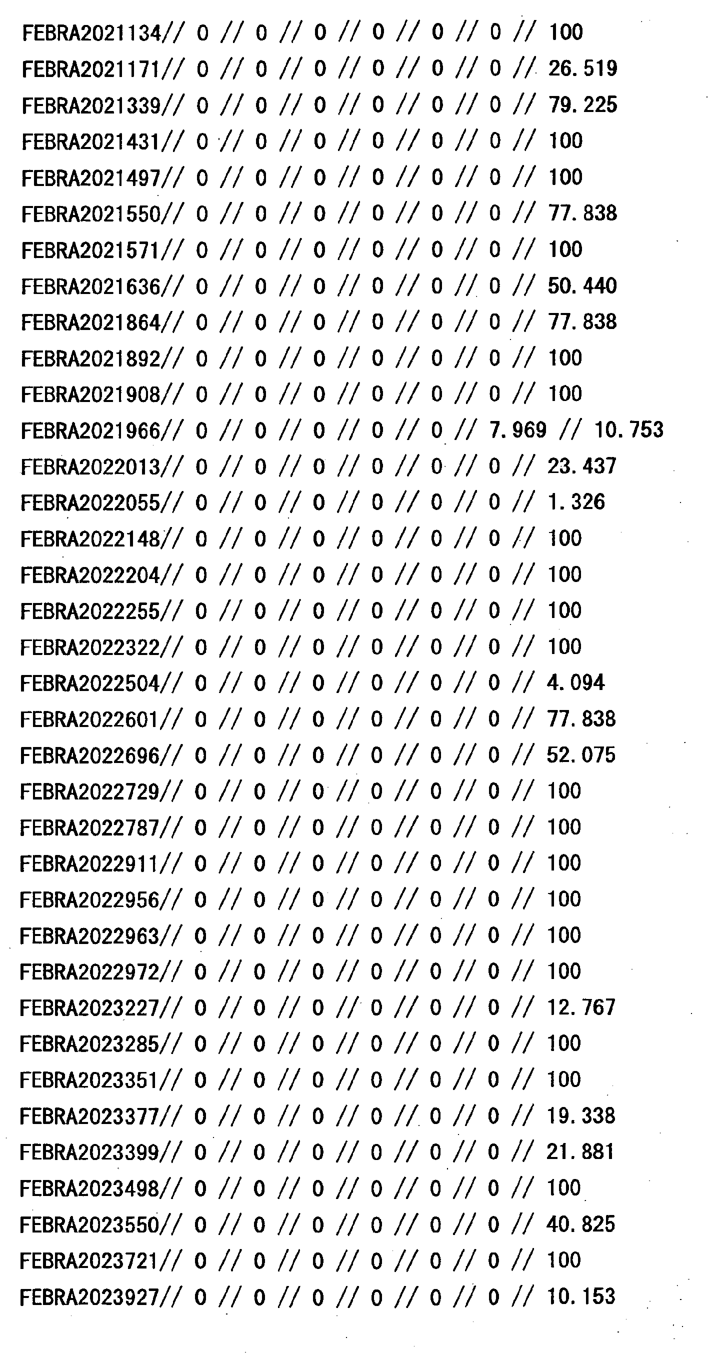

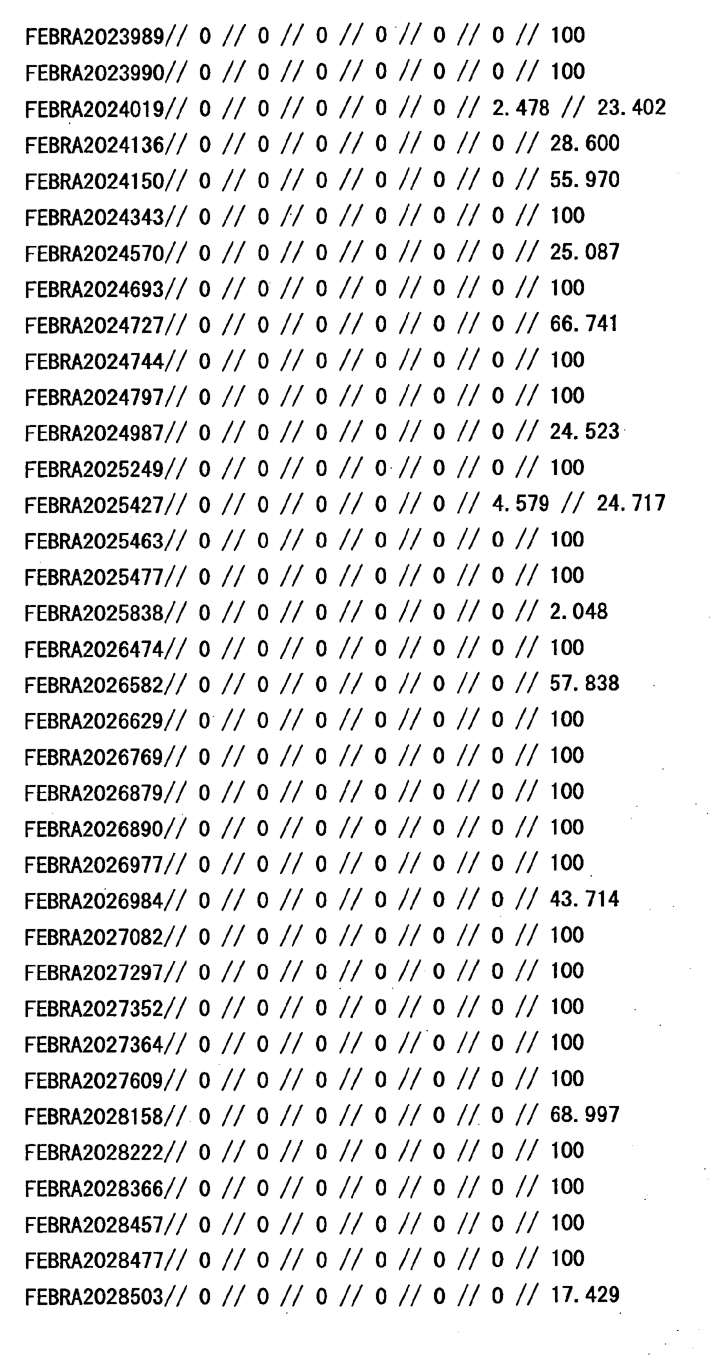

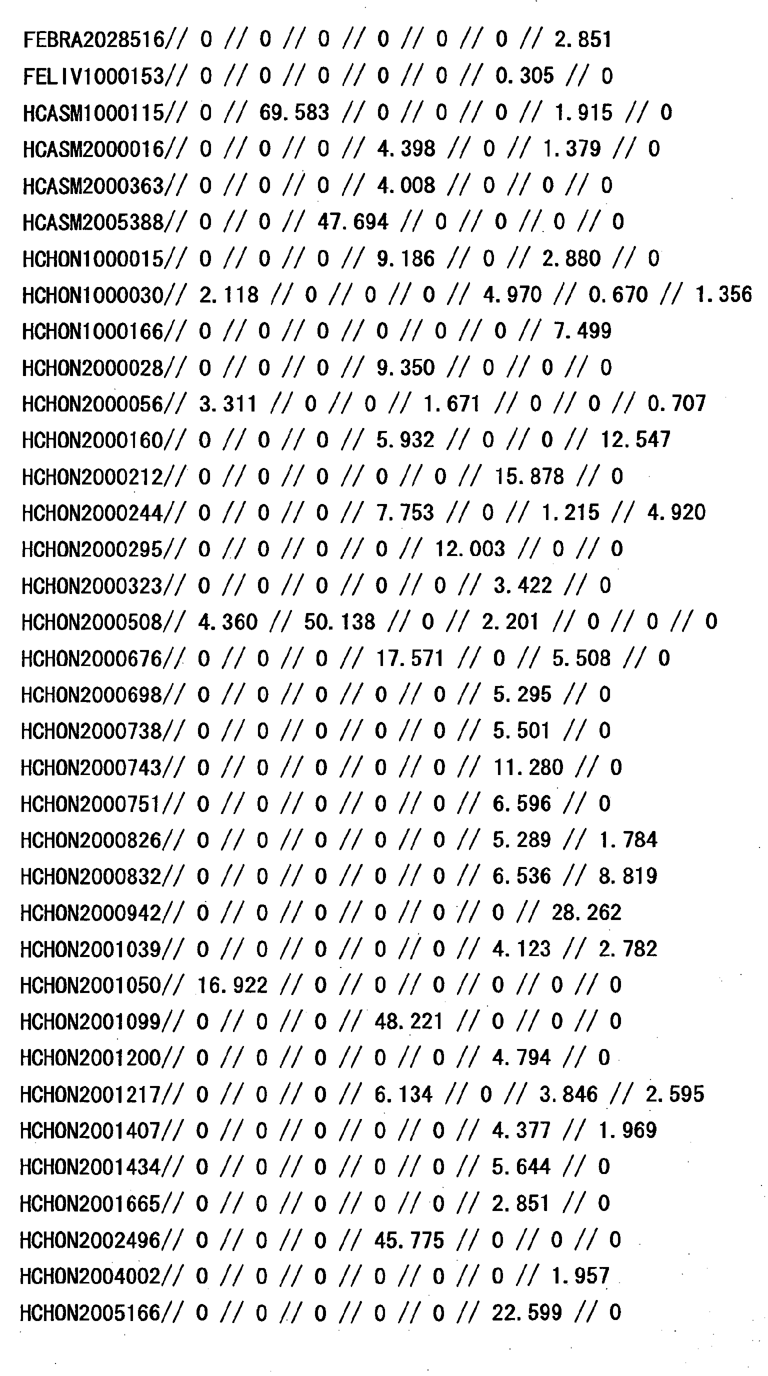

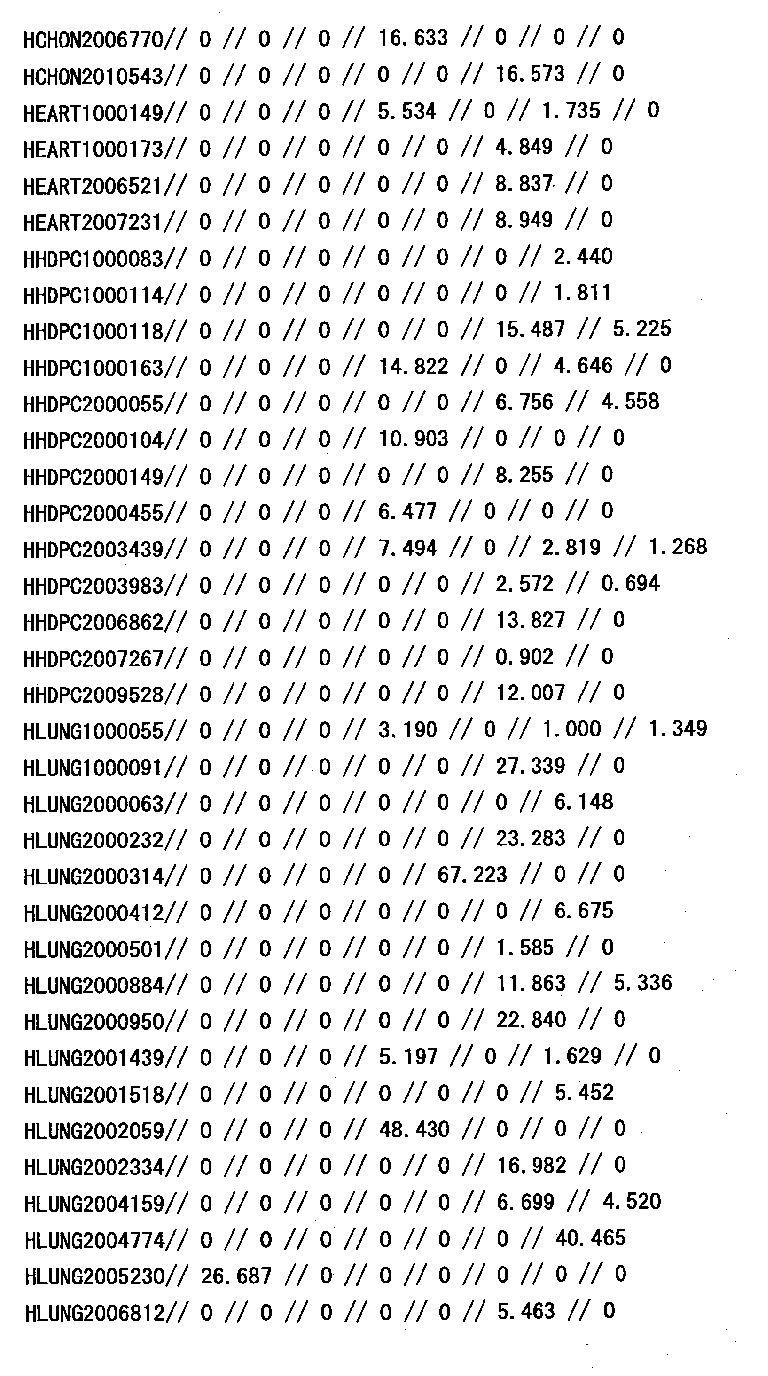

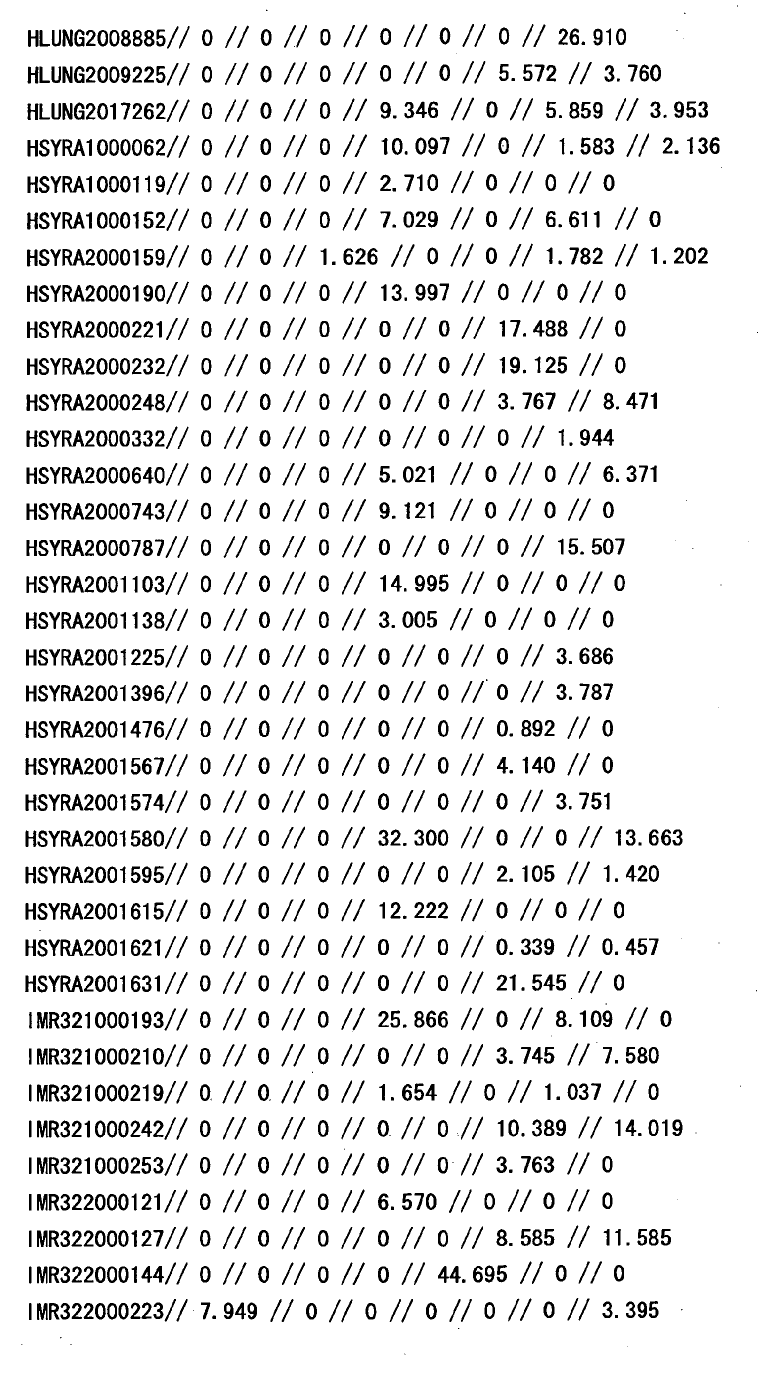

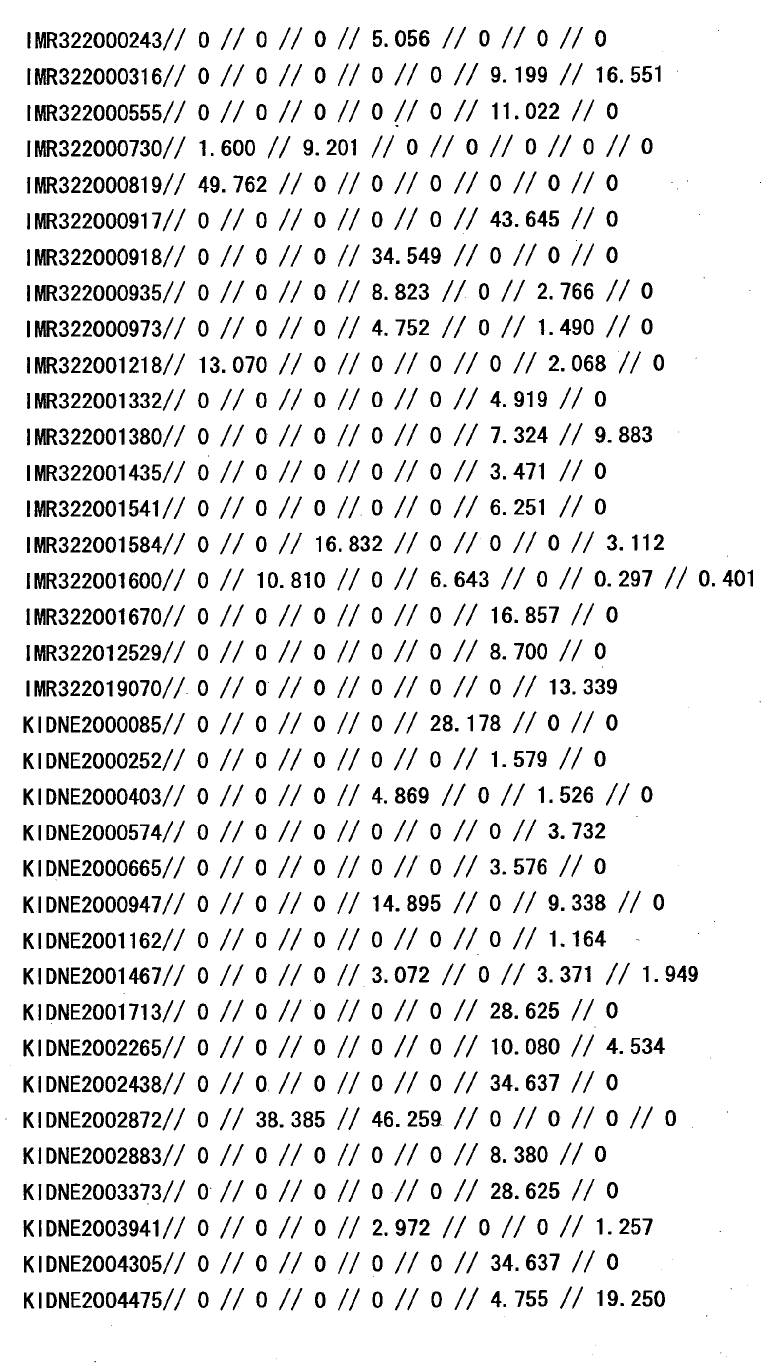

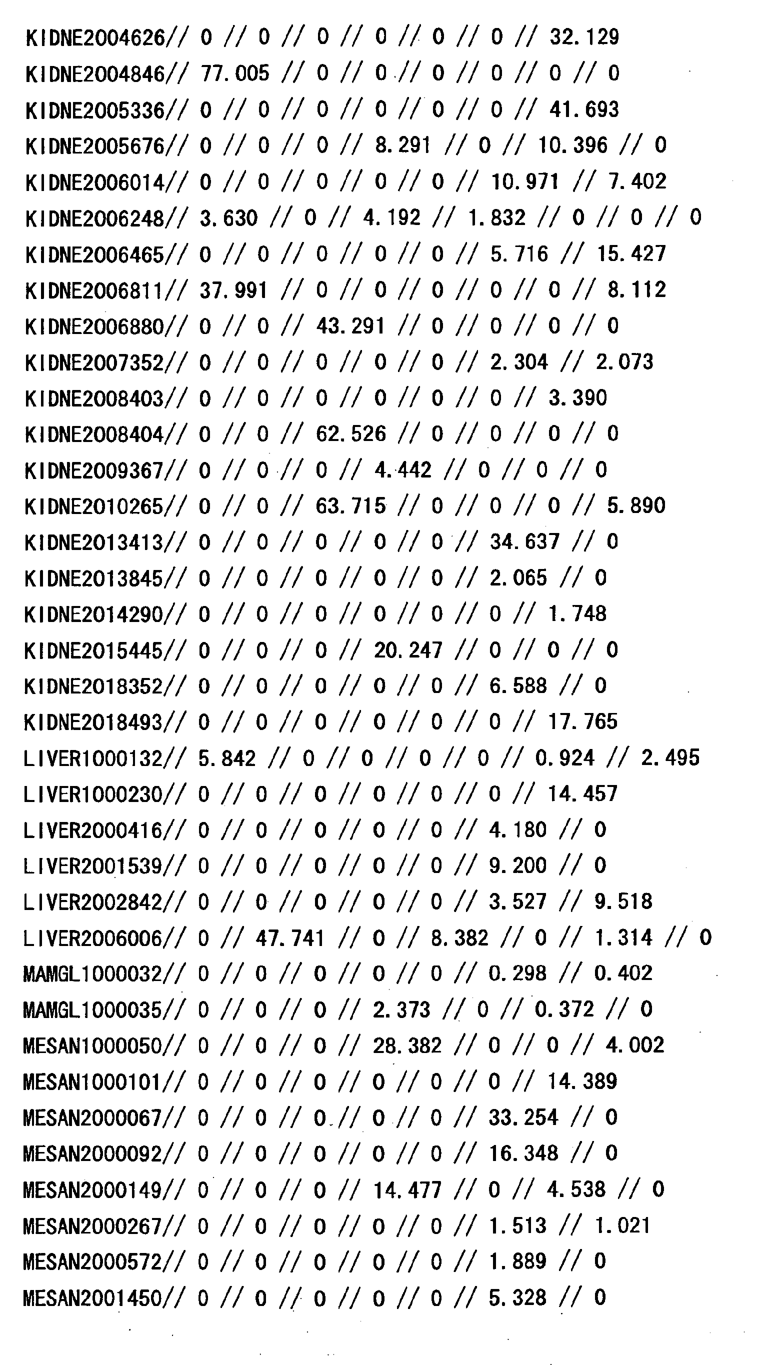

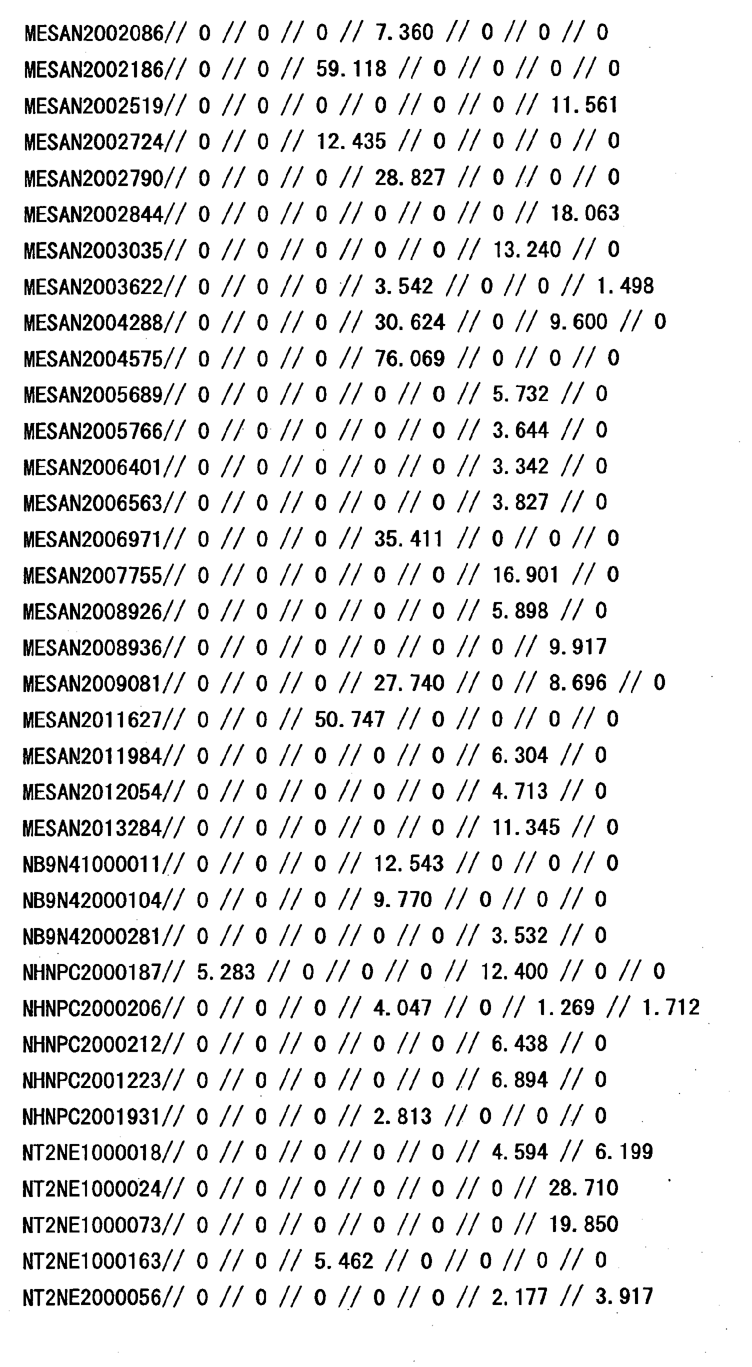

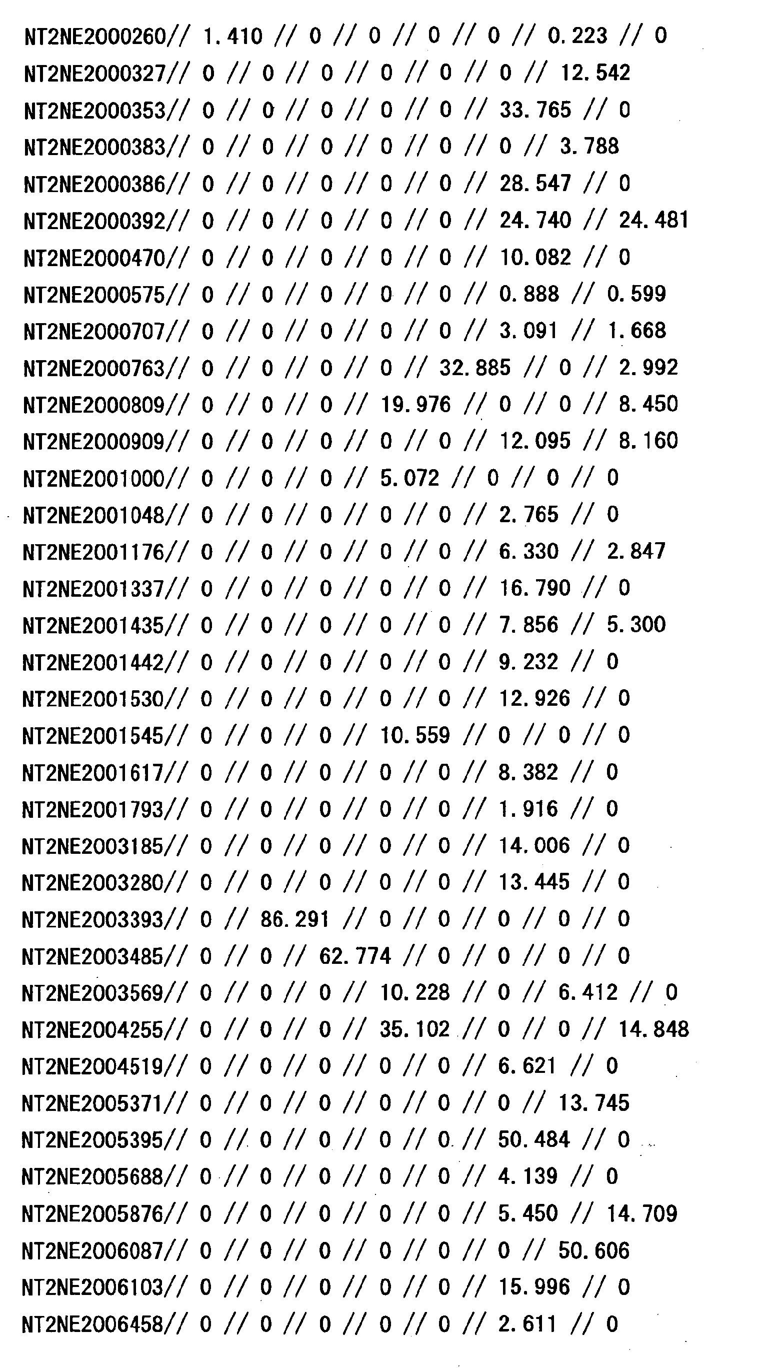

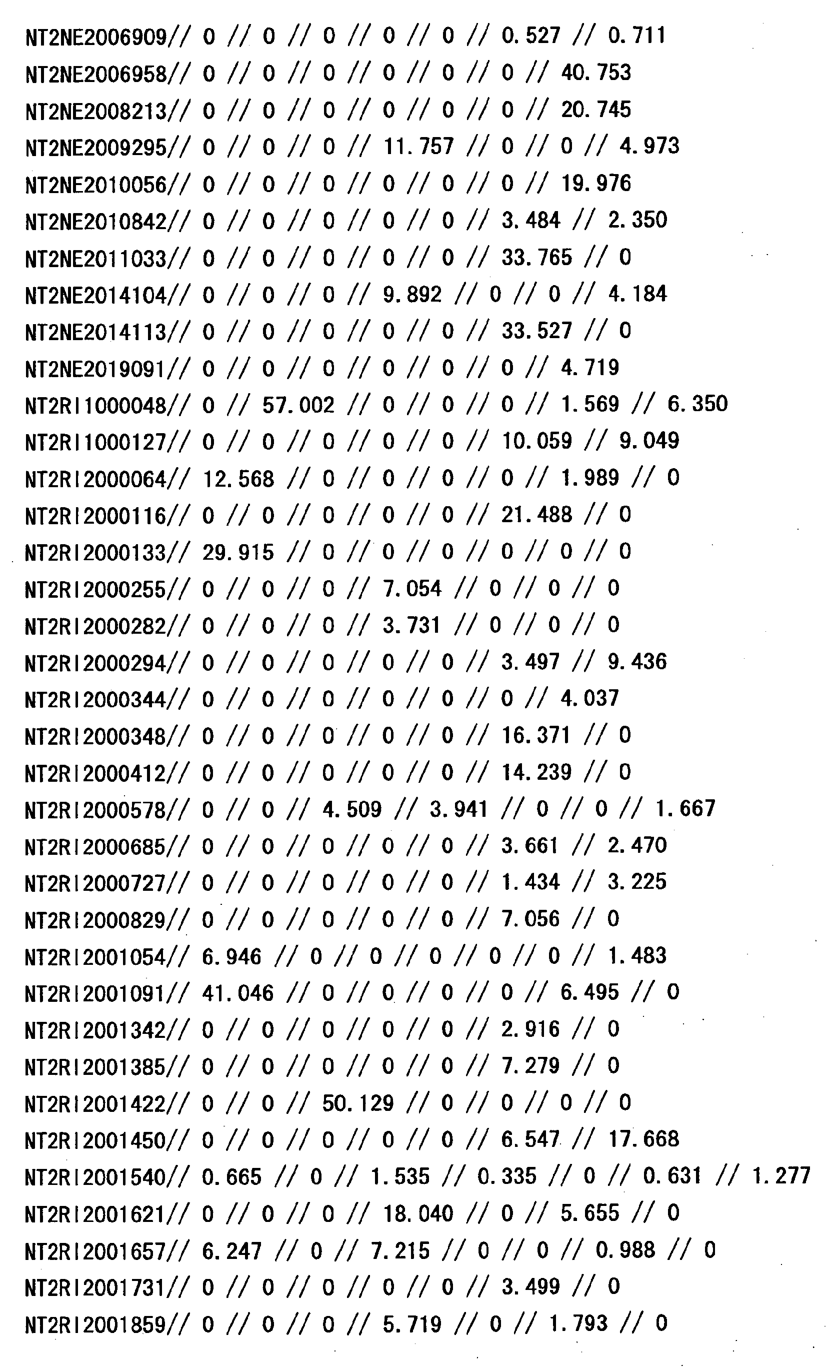

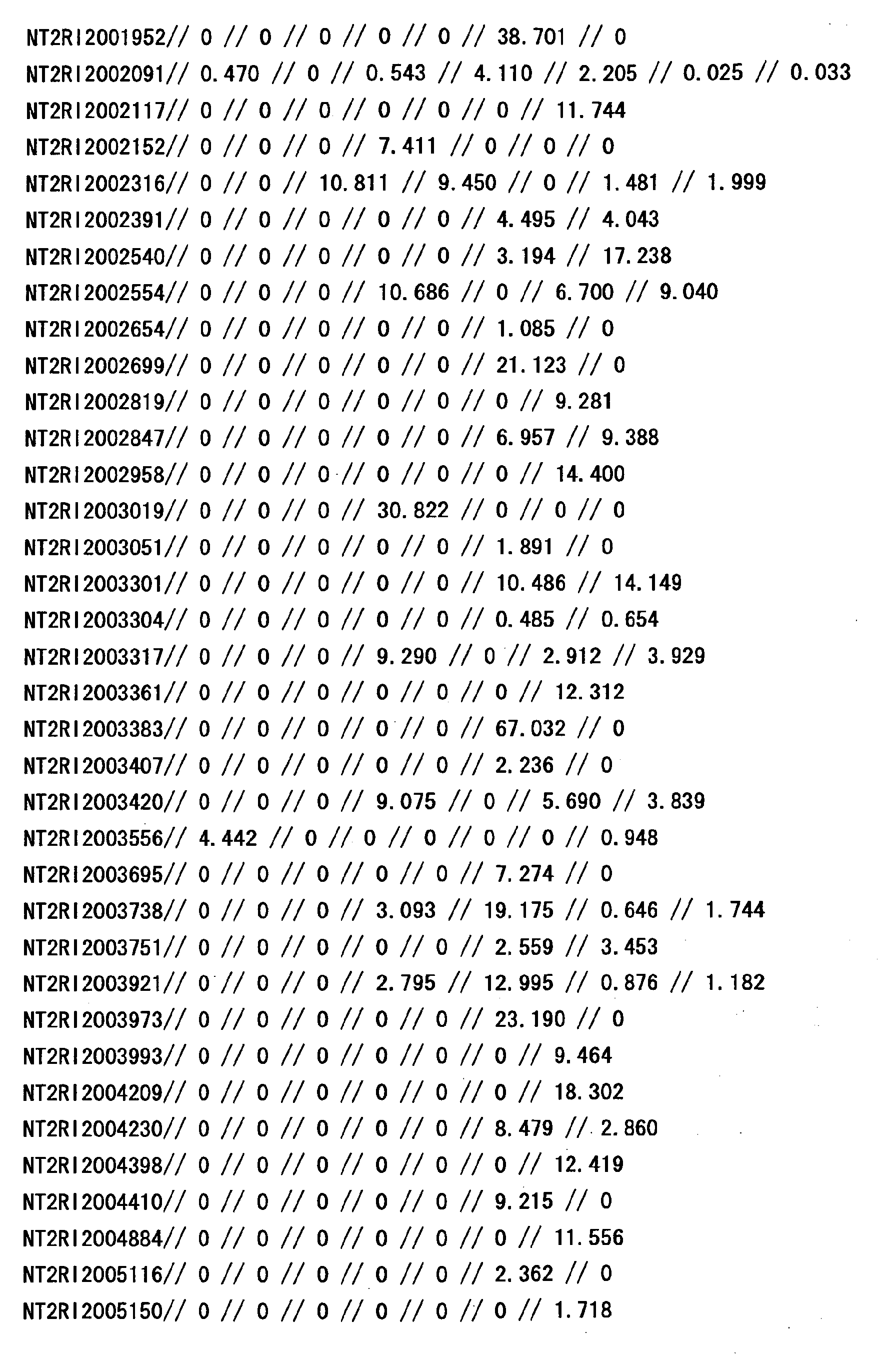

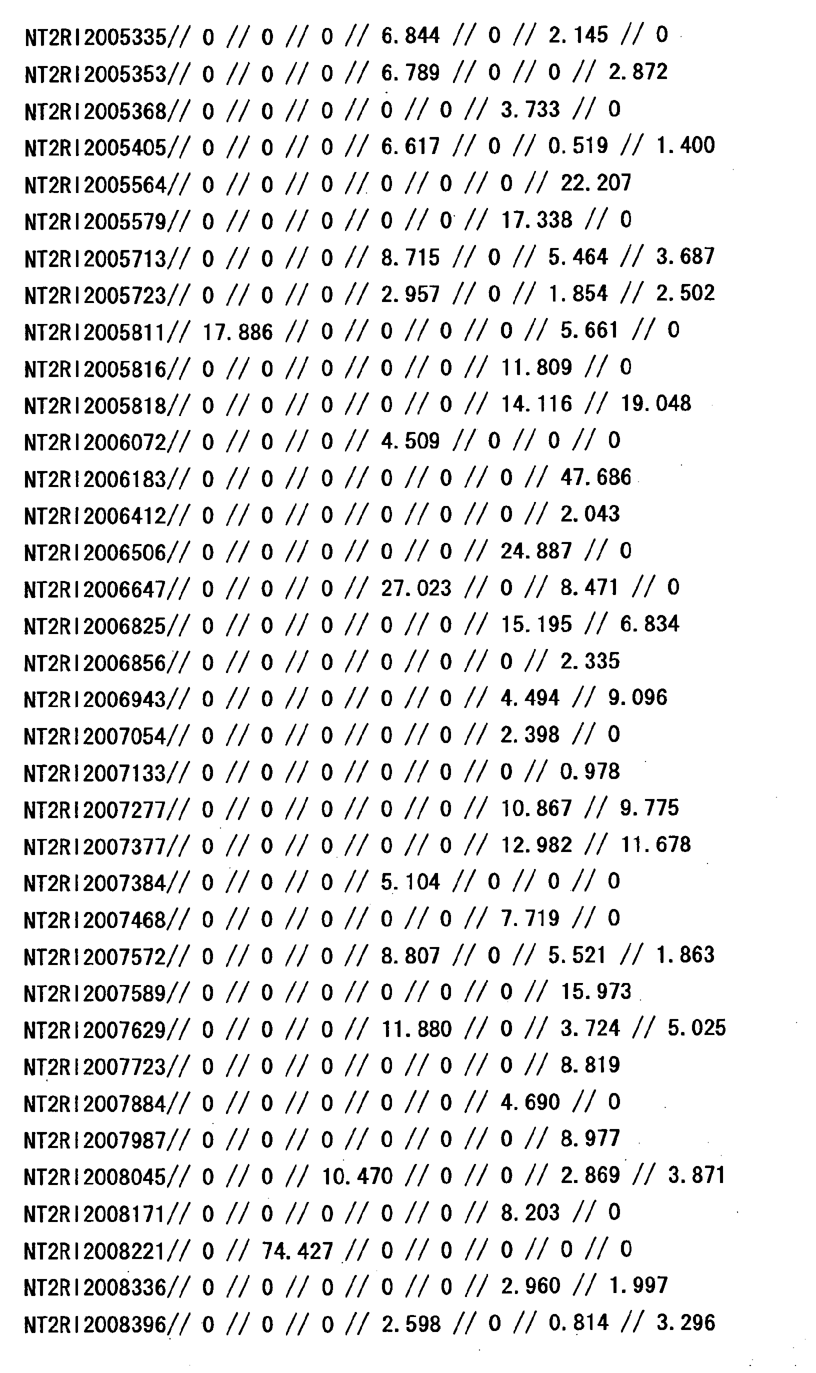

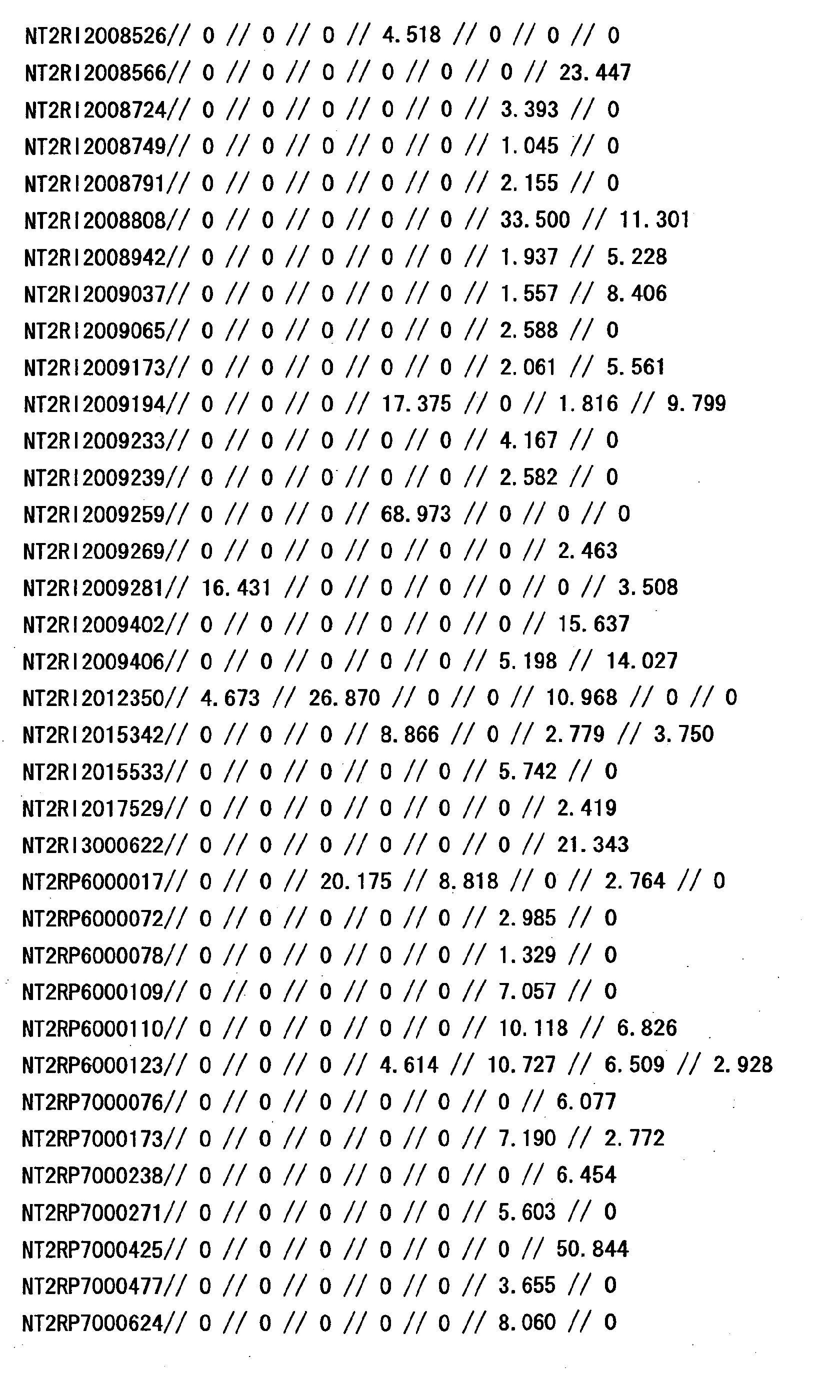

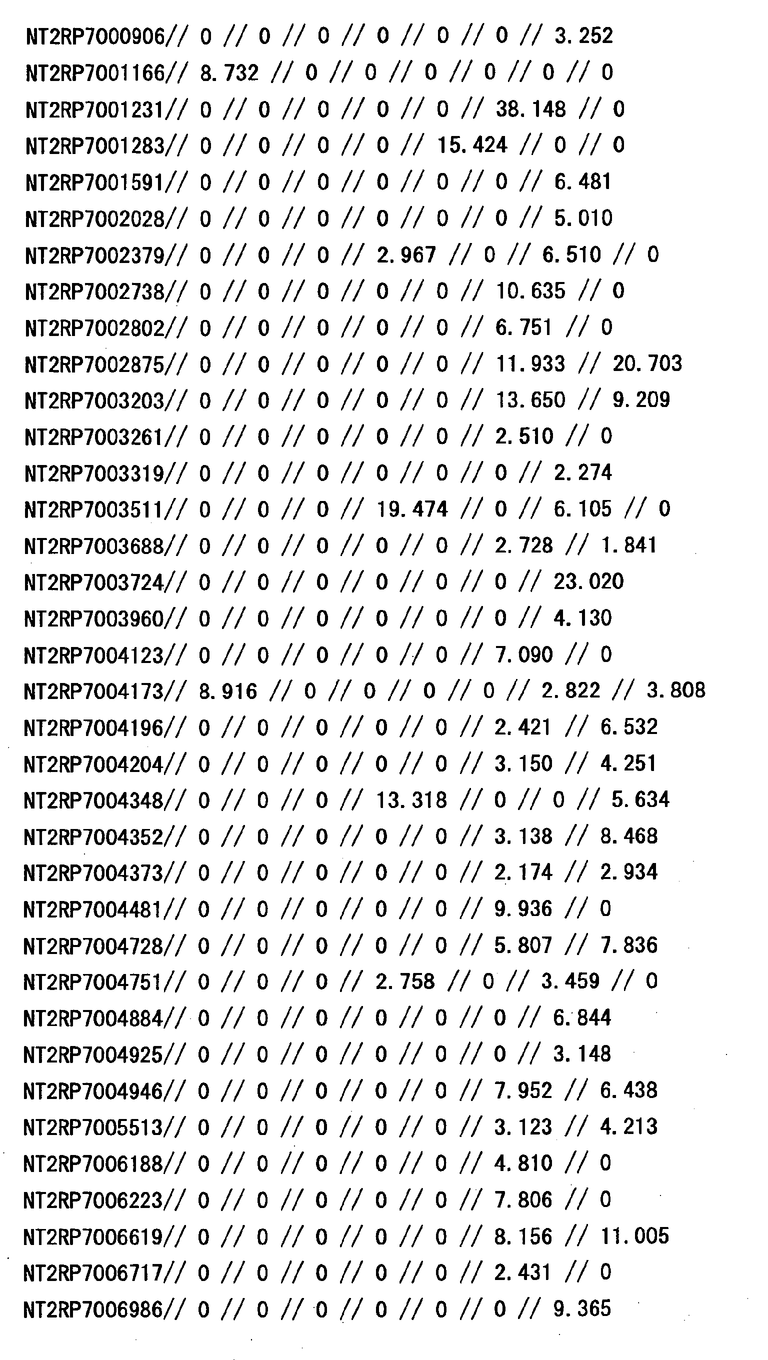

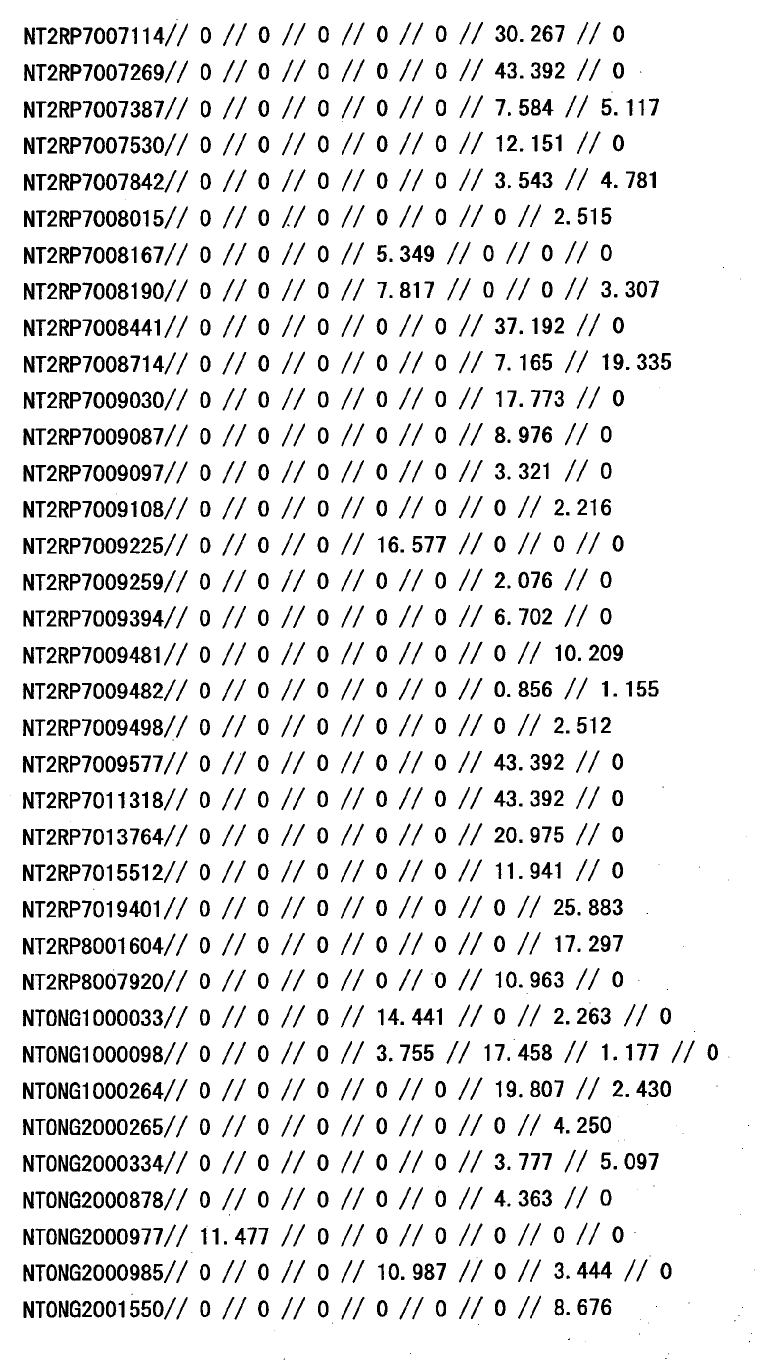

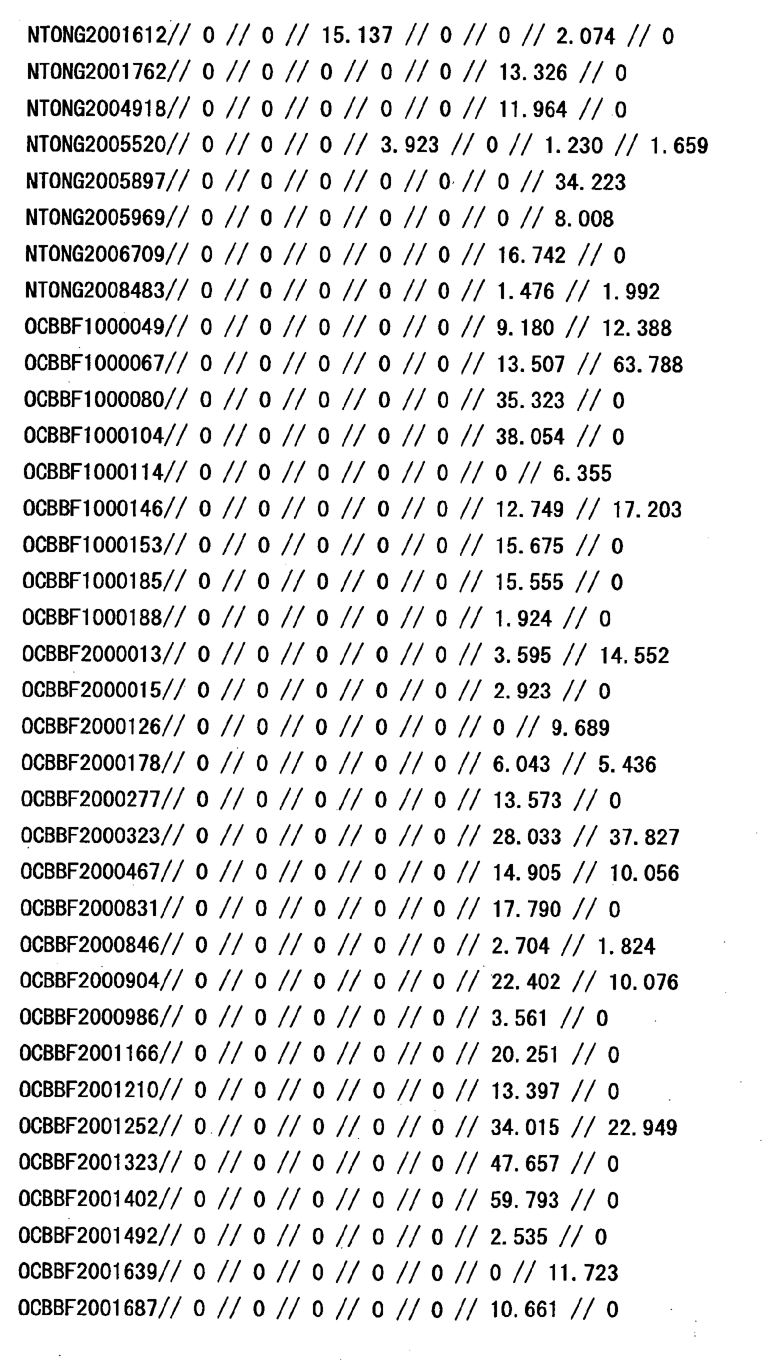

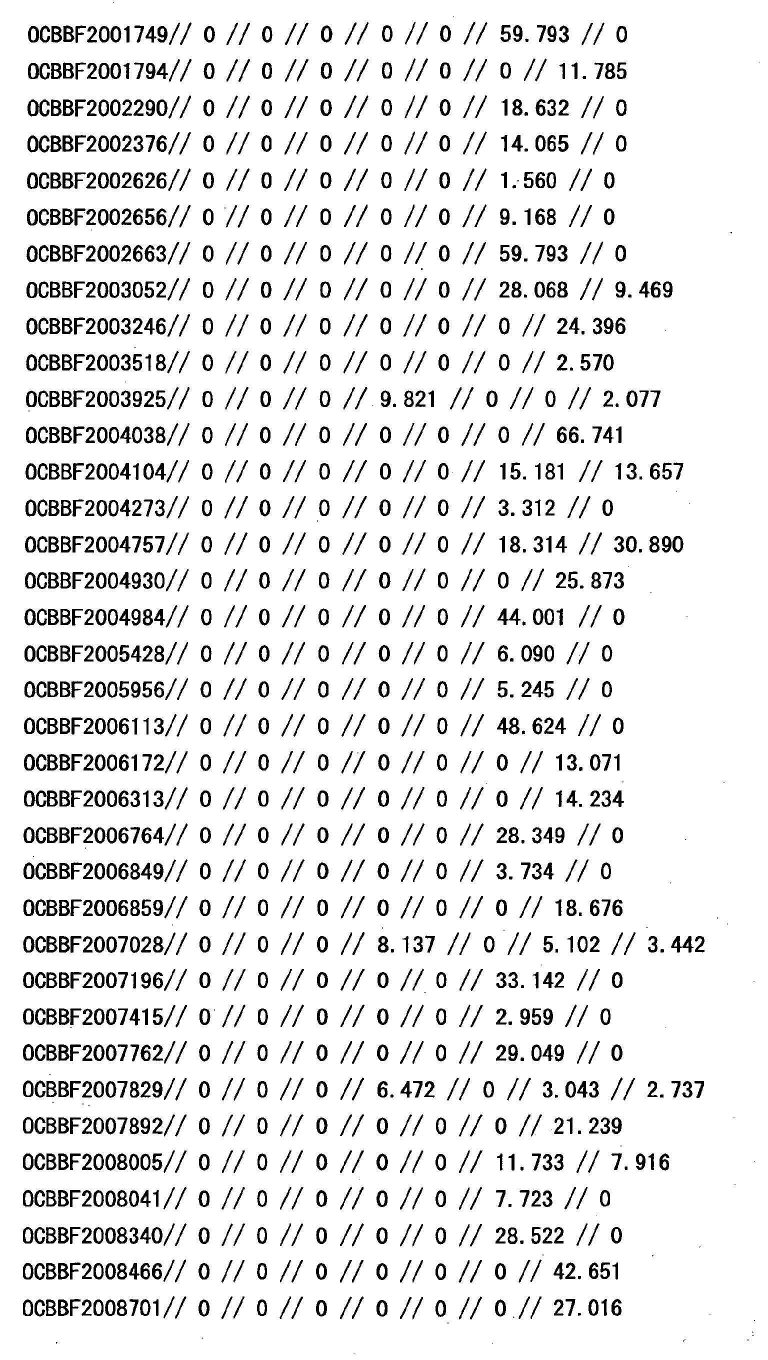

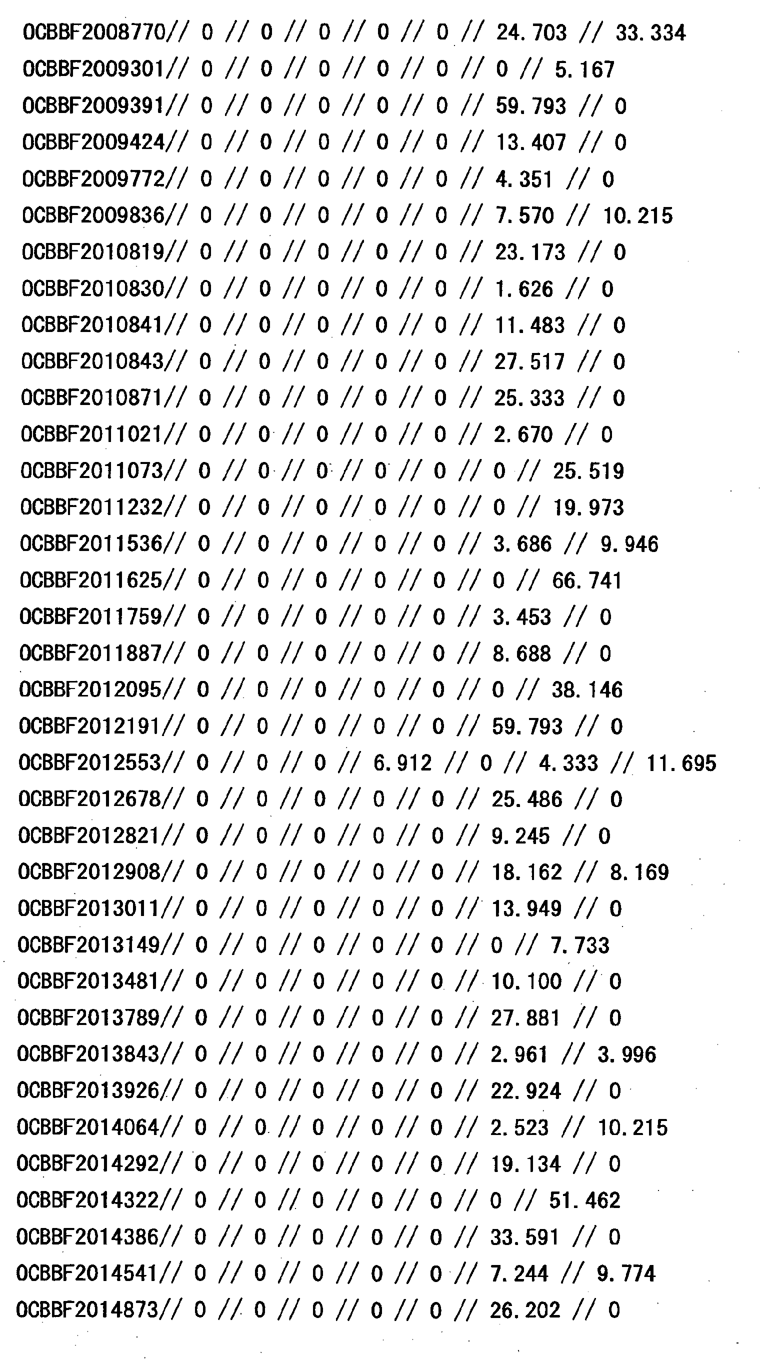

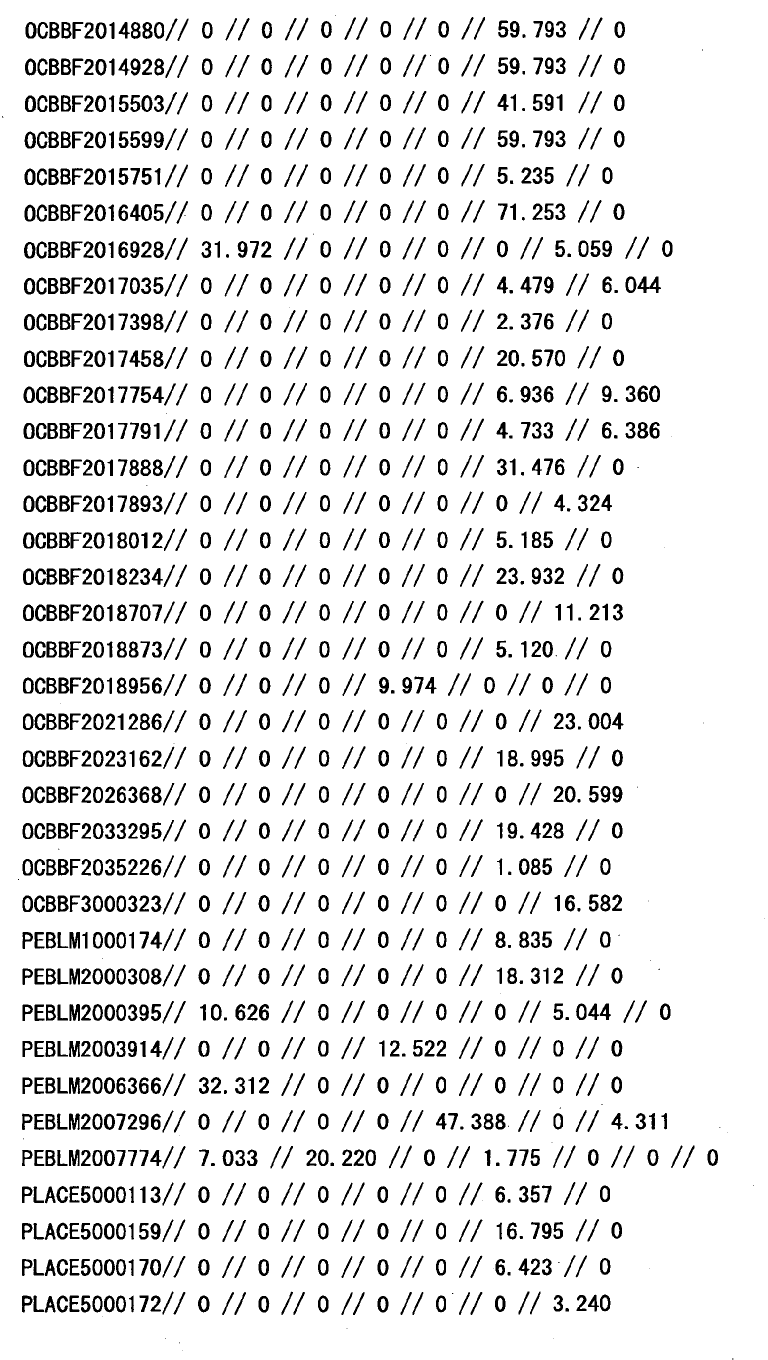

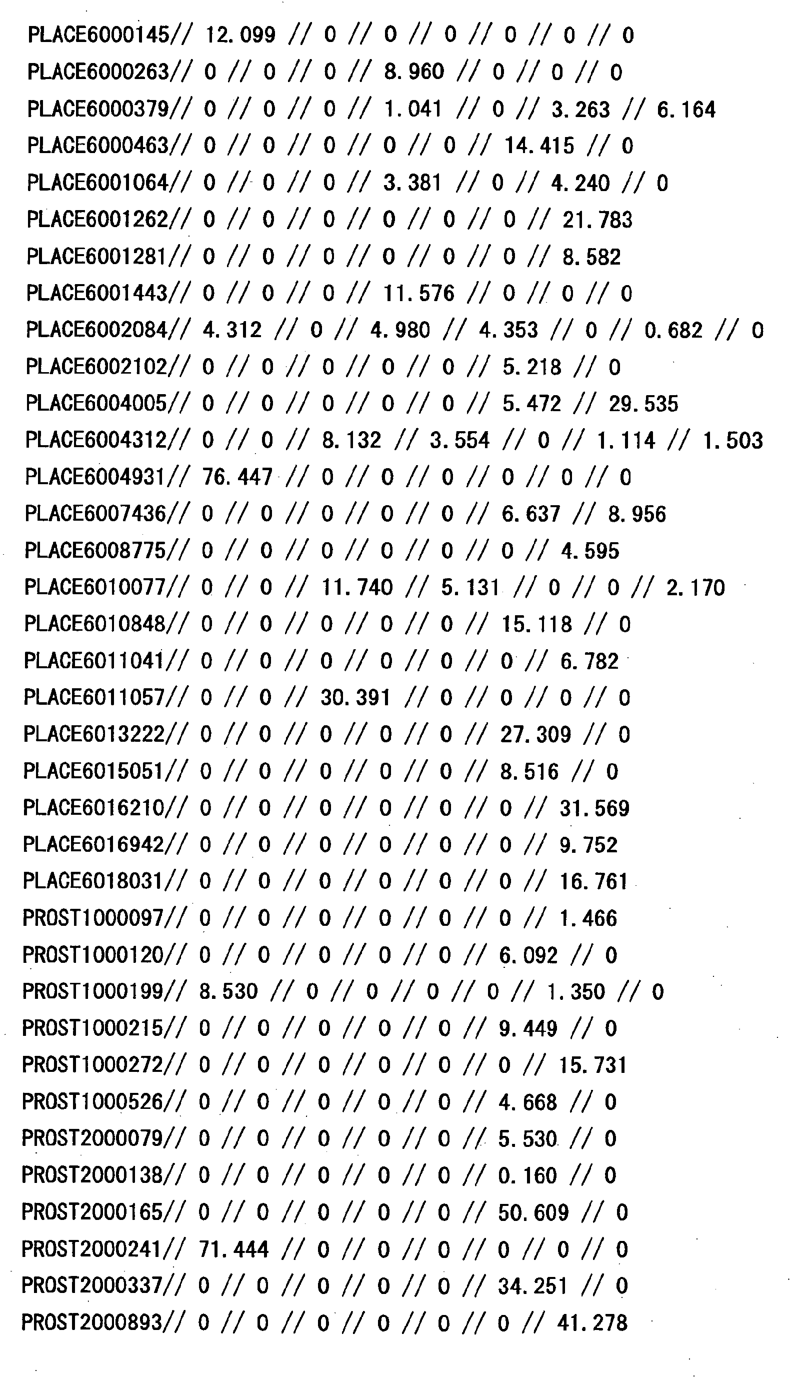

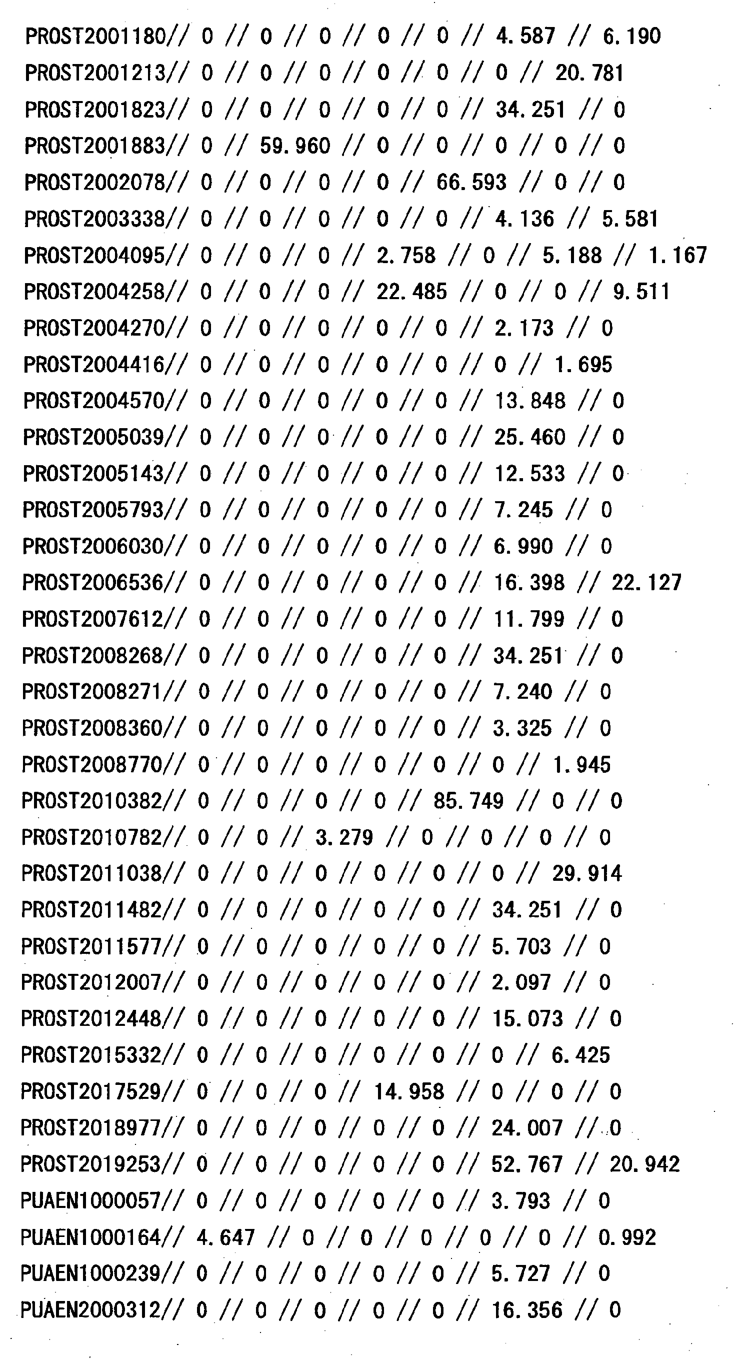

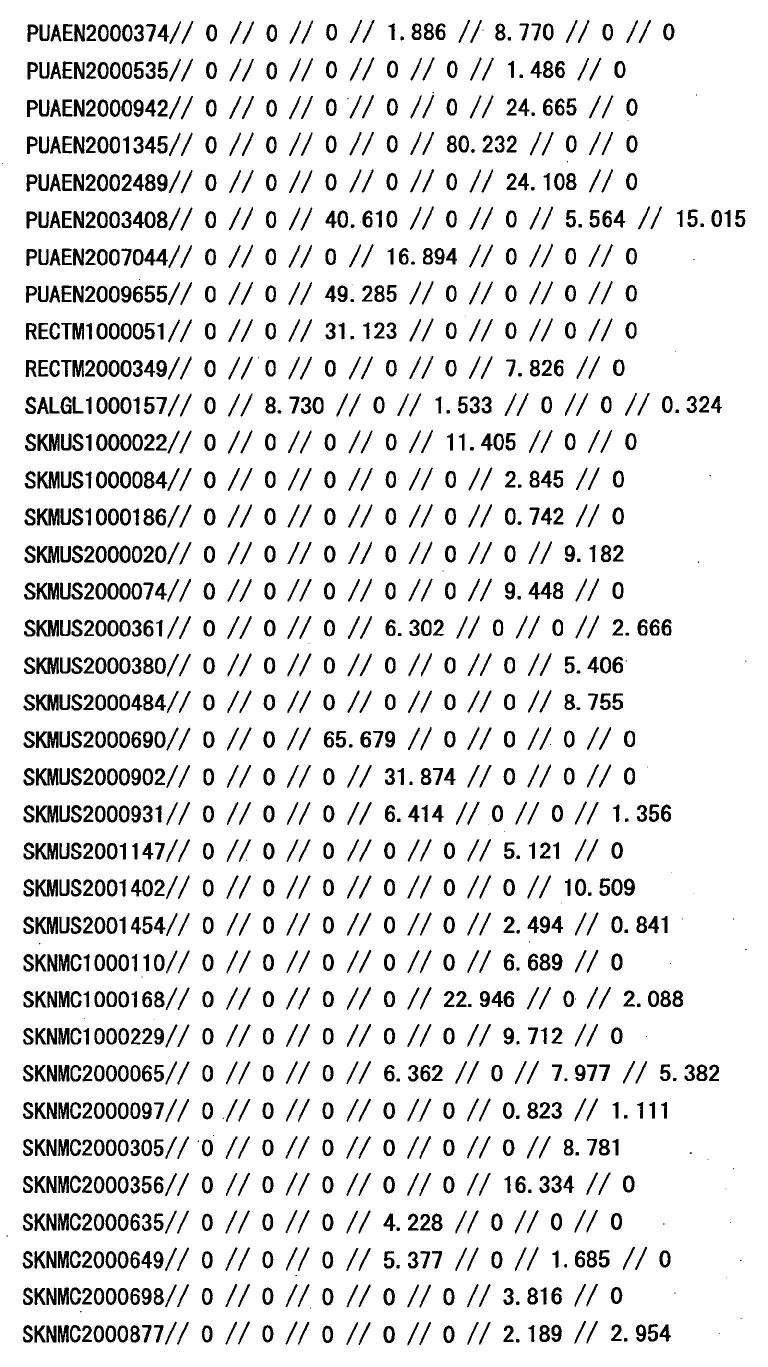

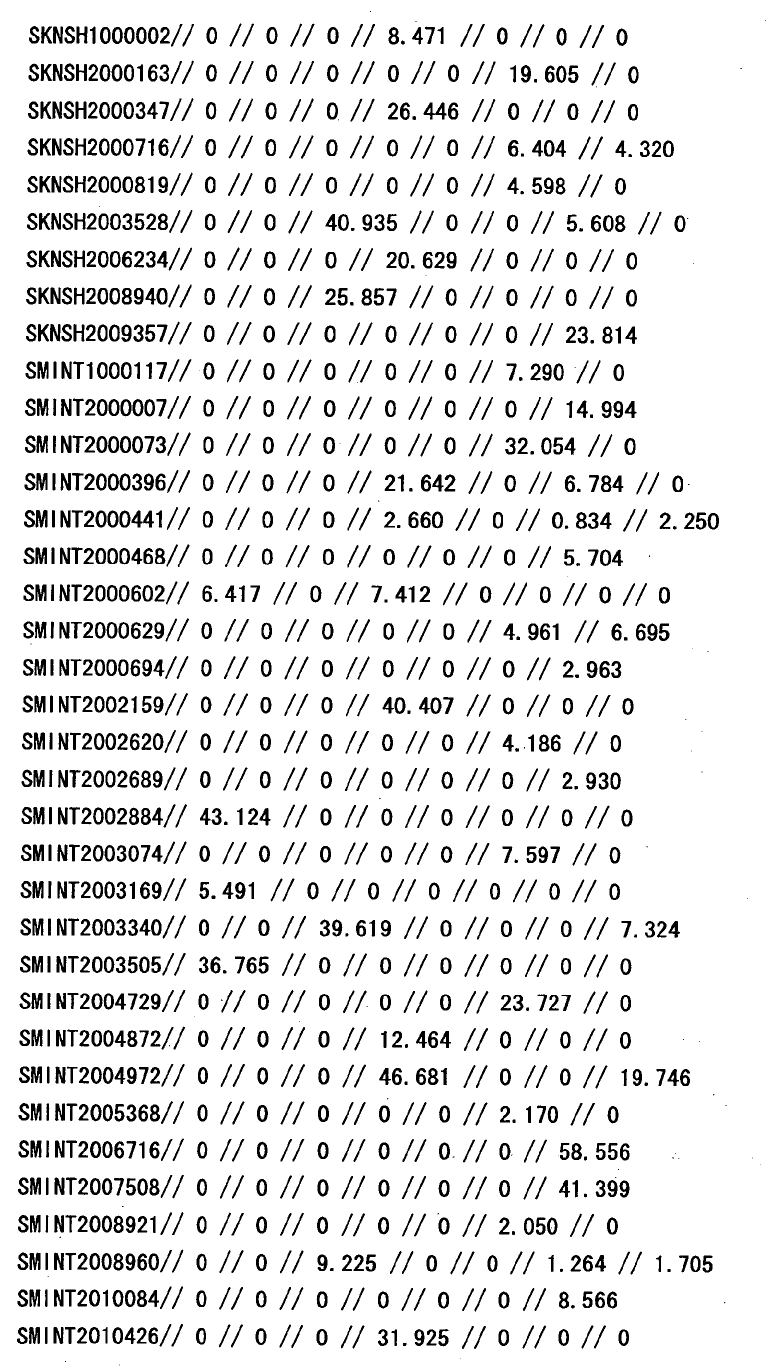

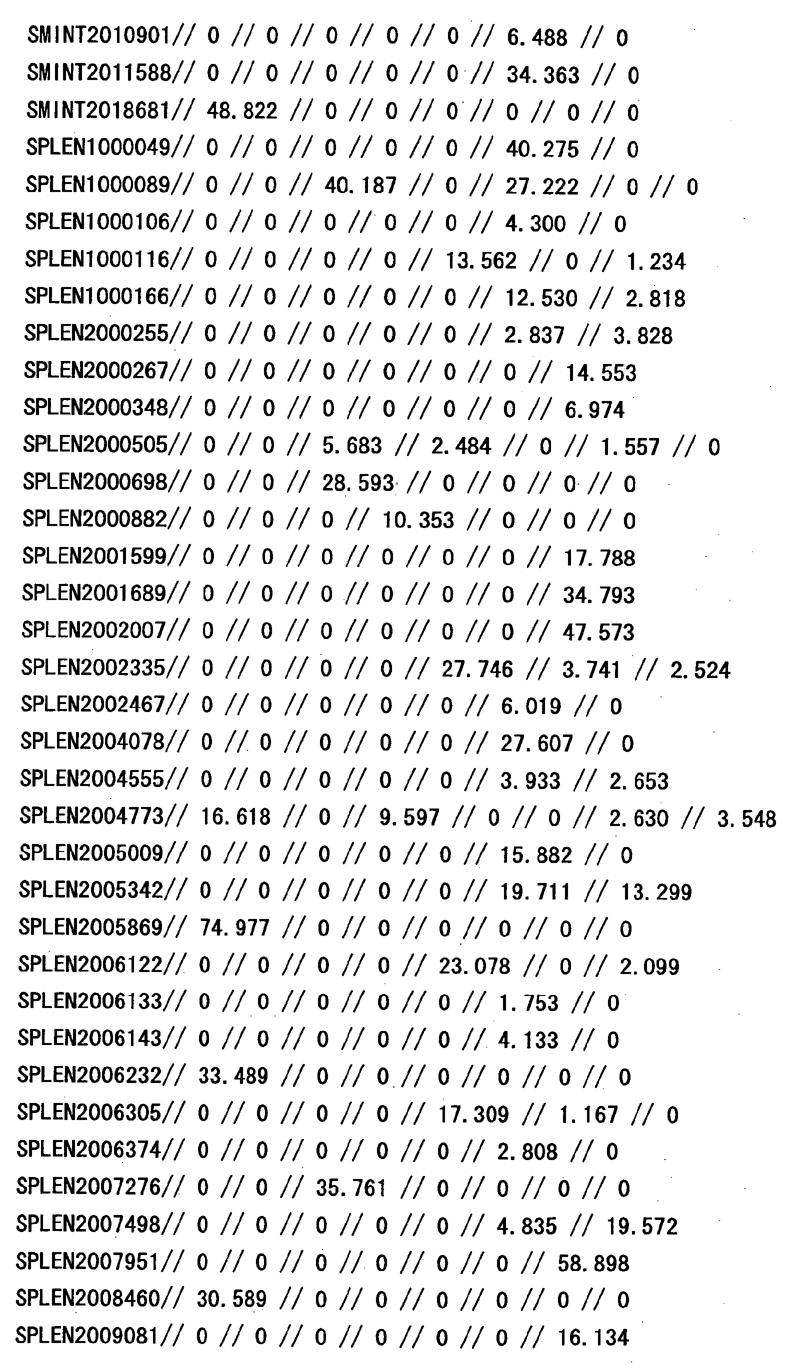

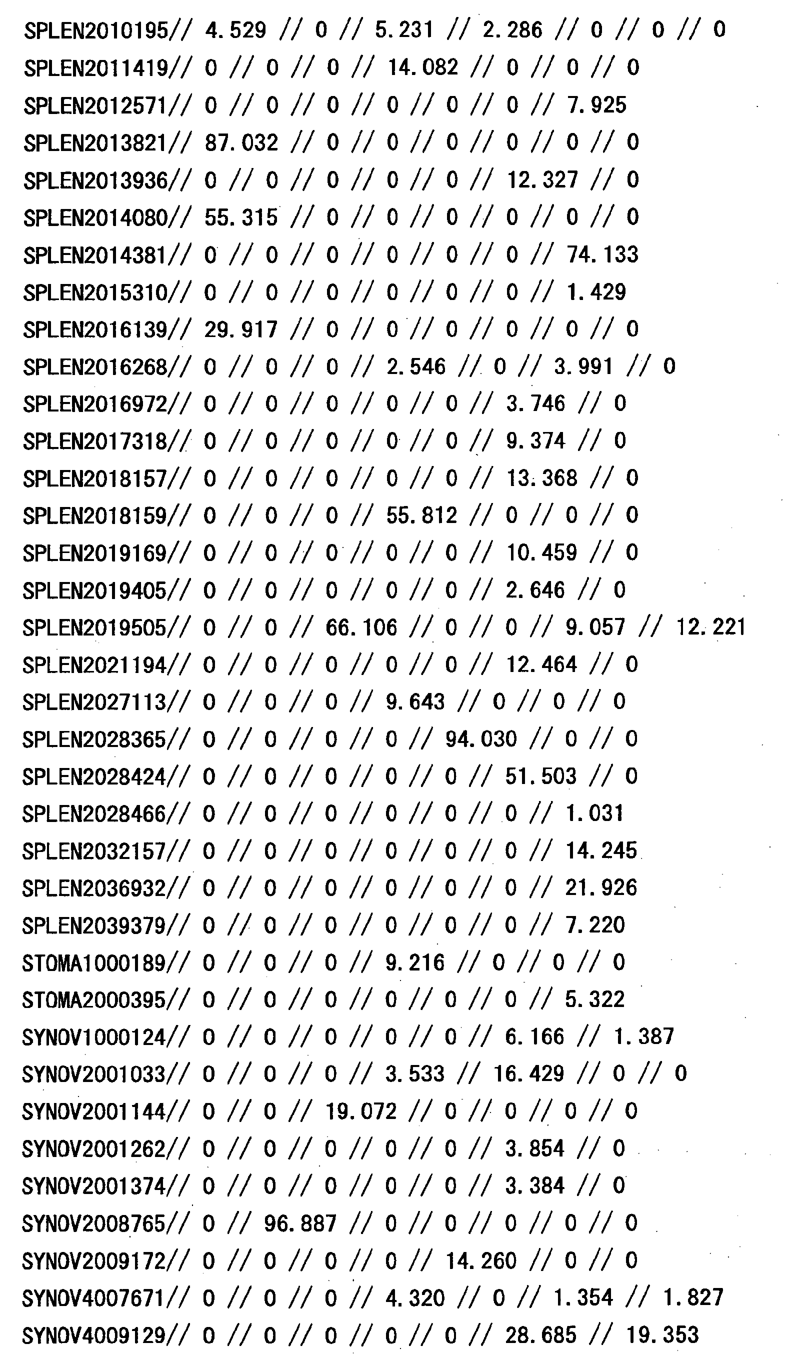

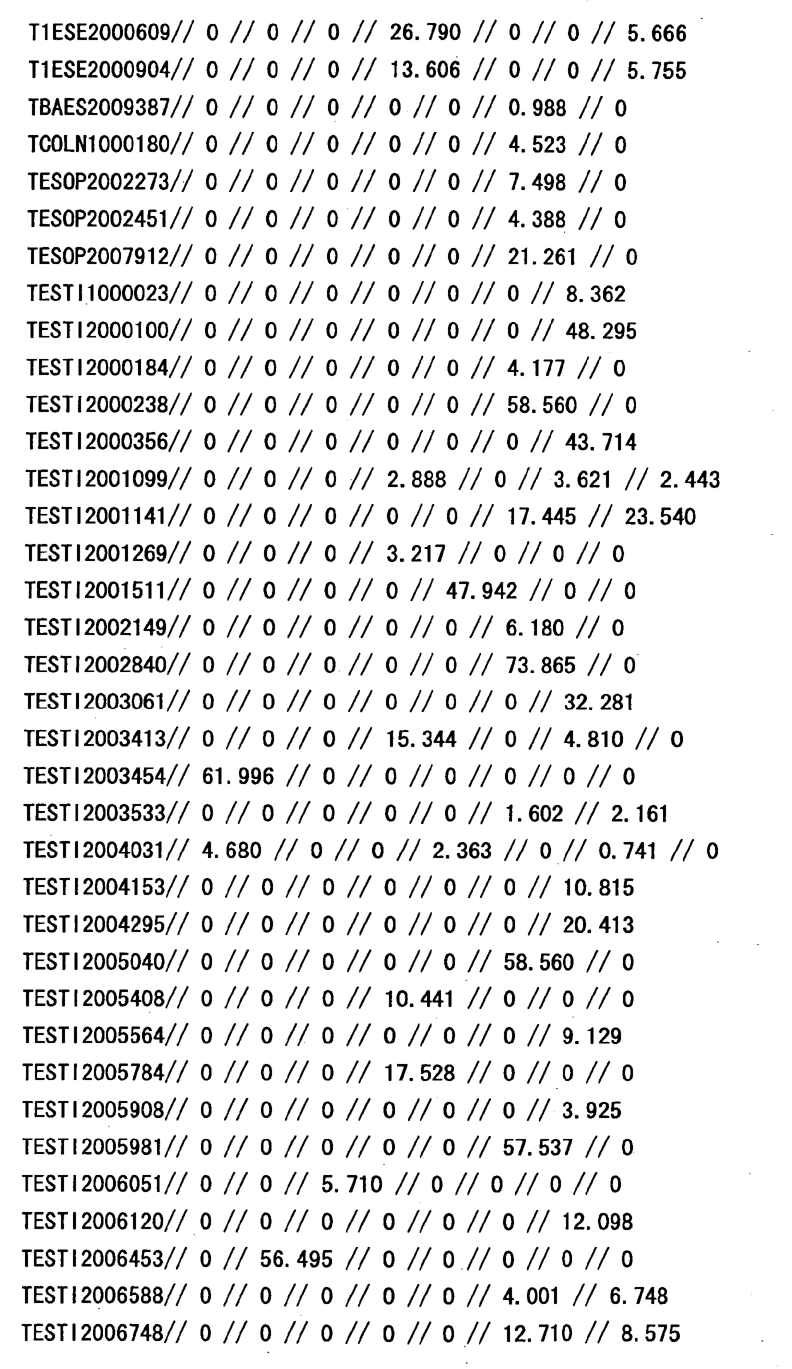

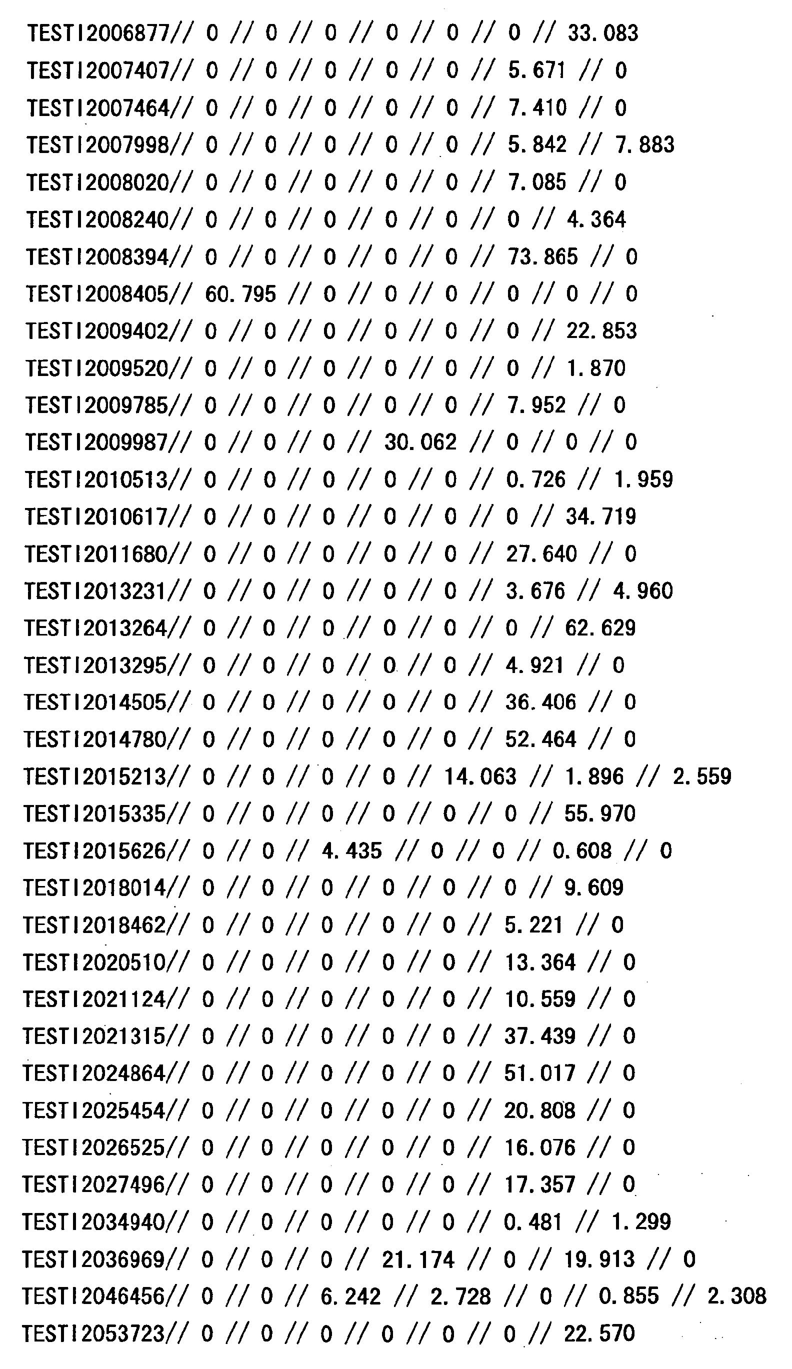

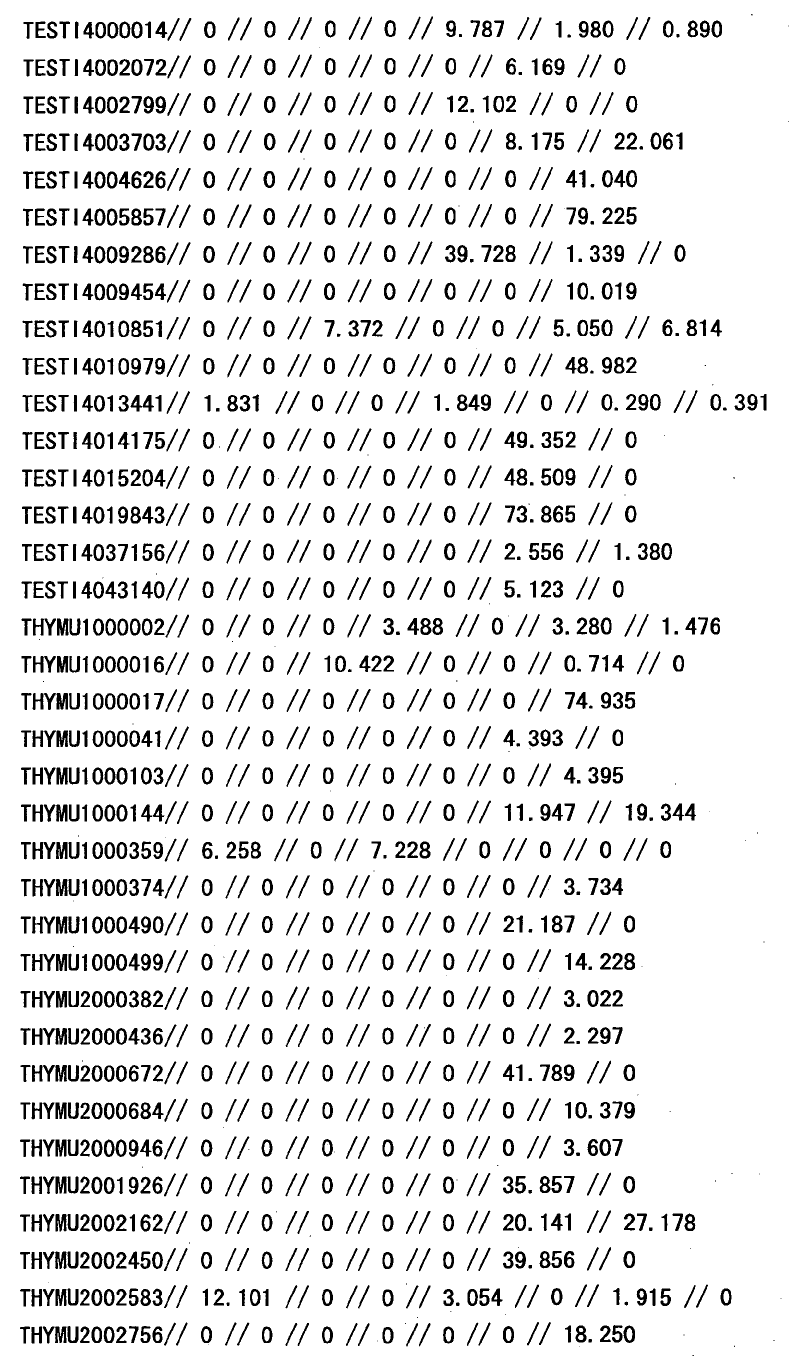

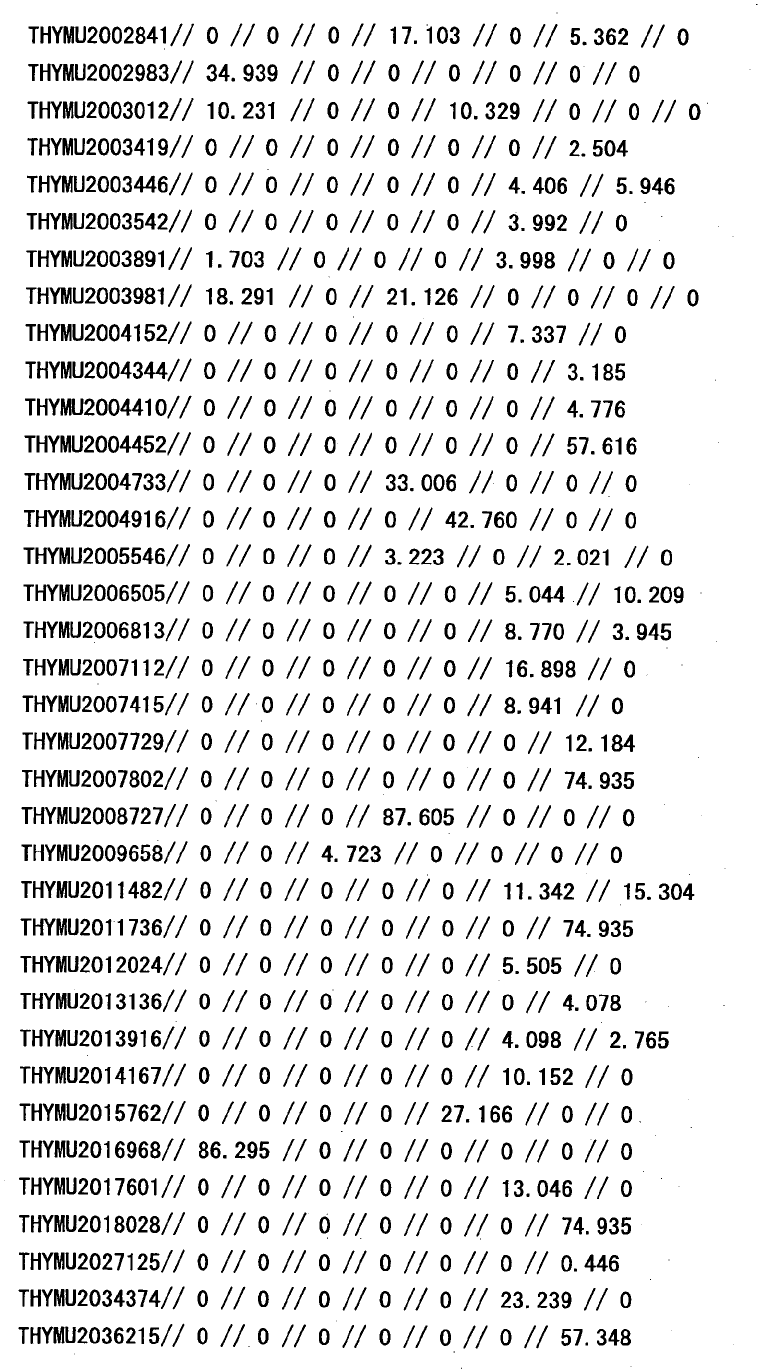

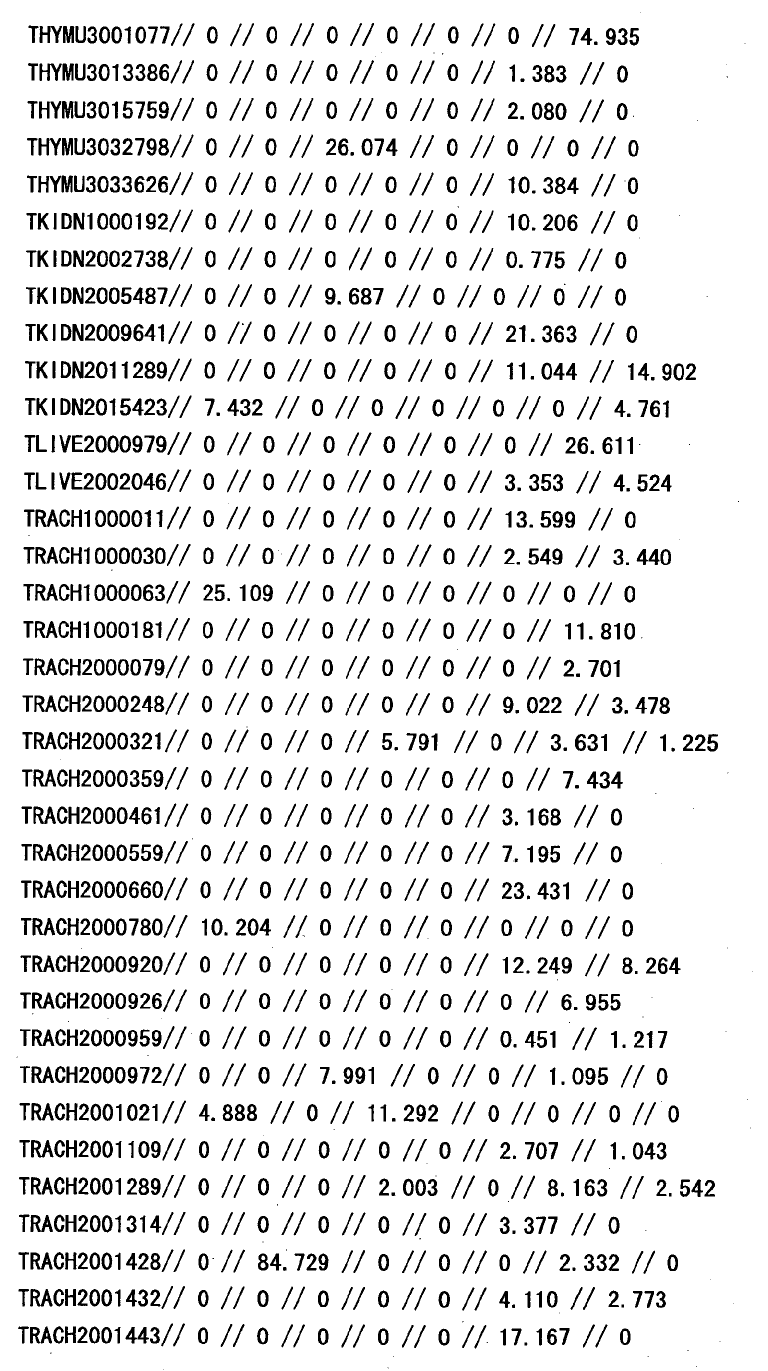

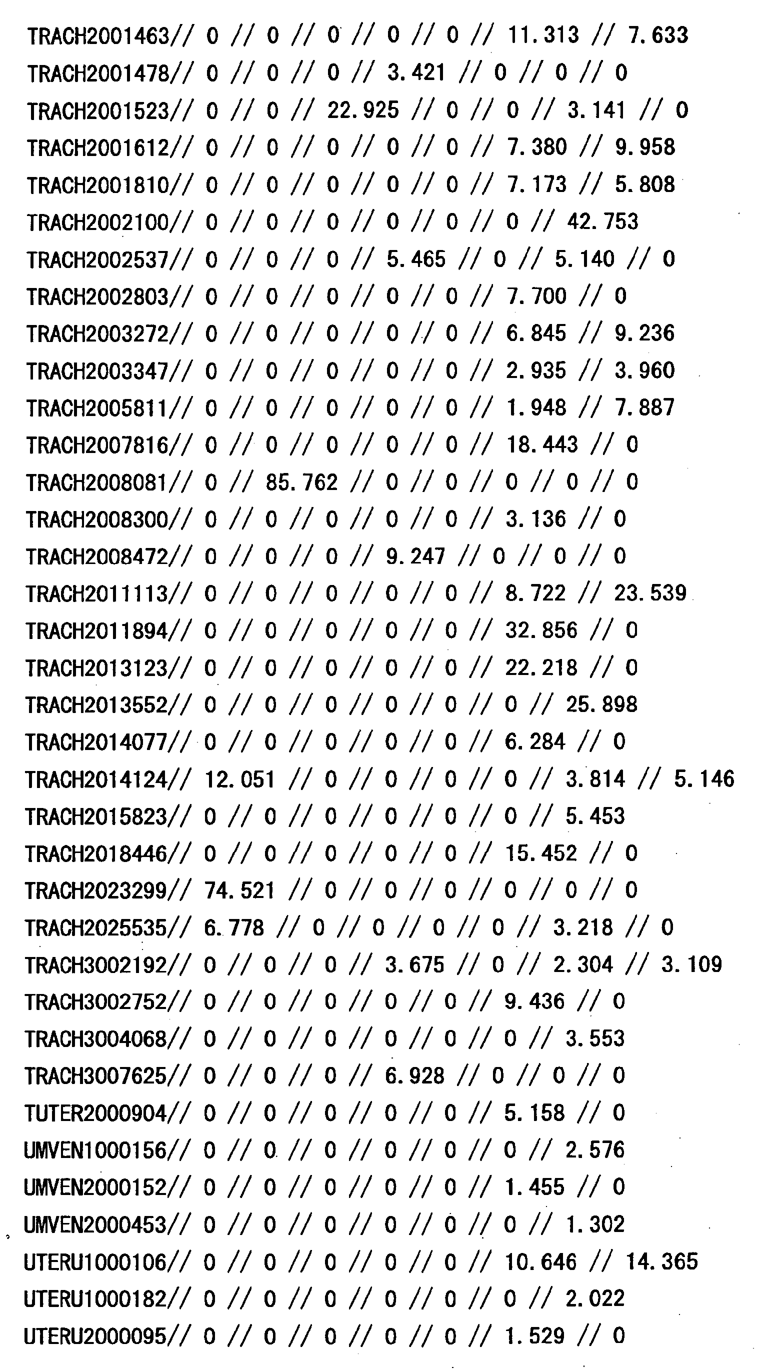

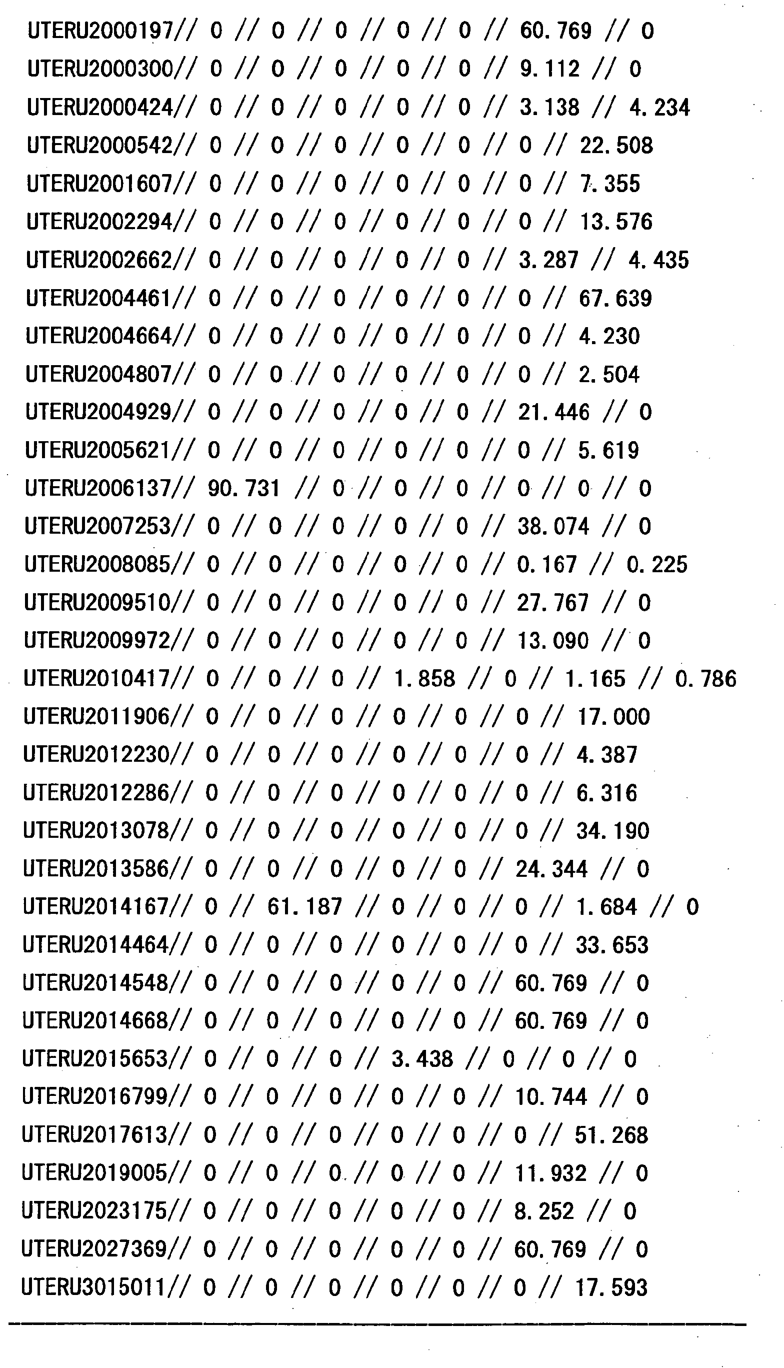

Genes whose expression levels were negligibly low in each of the above-described libraries are shown with their clone names below.
3NB691000018, 3NB691000085, 3NB691000113, 3NB691000116, 3NB691000129, 3NB691000191, 3NB692000012, 3NB692000029, 3NB692000102, 3NB692000154, 3NB692000276, 3NB692000305, 3NB692000429, 3NB692000529, 3NB692000973, 3NB692001002, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692008178, 3NB692008729, ACTVT2000380, ADIPS1000064, ADIPS2000069, ADIPS2000088, ADIPS2000245, ADRGL1000038, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000095, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001076, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003316, ASTRO2003574, ASTRO2003581, ASTRO2003632, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005423, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006952, ASTRO2007221, ASTRO2007377, ASTRO2007515, ASTRO2007578,. ASTRO2007643, ASTRO2007666, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014923, ASTRO2014961, ASTRO2015162, ASTRO2015185, ASTRO2015328, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000616, BGGI12000684, BGGI12000693, BGGI12000839, BGGI12001002, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE20077.44,. BLADE2007799, BLADE2007923, BLADE2007935, BLADE2007958, BLADE2008281, BLADE2008398, BLADE2008454, BLADE2008479, BLADE2008539, BLADE2008809, BLADE2008995, BLADE2009452, BLADE2009479, BLADE2009593, BNGH41000104, BNGH41000118, BNGH41000137, BNGH41000153, BNGH41000169, BNGH41000177, BNGH4100019.0, BNGH41000198, BNGH41000216, BNGH41000235, BNGH42000130, BNGH42000132, BNGH42000360, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798, BNGH42008199, BNGH42008510, BNGH42008649, BNGH42008743, BNGH42008832, BNGH42008850, BNGH42008943, BNGH42009021, BRACE1000020, BRACE1000047, BRACE1000051, BRACE1000065, BRACE1000070, BRACE1000073, BRACE1000084, BRACE1000092, BRACE1000101, BRACE1000114, BRACE1000124, BRACE1000131, BRACE1000132, BRACE1000166, BRACE1000186, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000753, BRACE2000815, BRACE2000864, BRACE2000885, BRACE2000893, BRACE2000905, BRACE2000936, BRACE2000947, BRACE2000965, BRACE2000969, BRACE2000988, BRACE2001017, BRACE2001065, BRACE2001070, BRACE2001094, BRACE2001107, BRACE2001143, BRACE2001151, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626, BRACE2001673, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002884, BRACE2002896, BRACE2002948, BRACE2003078, BRACE2003097, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003628,. BRACE2003639, BRACE2003700, BRACE2003713, BRACE2003766, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003905, BRACE2003928, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005243, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005949, BRACE2005981, BRACE2005991, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006274, BRACE2006281, BRACE2006298, BRACE2006363, BRACE2006378, BRACE2006380, BRACE2006393, BRACE2006397, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006636, BRACE2006685, BRACE2006703, BRACE2006753, BRACE2006783, BRACE2006821, BRACE2006833, BRACE2006859, BRACE2006871, BRACE2006909, BRACE2006911, BRACE2006924, BRACE2006935, BRACE2006944, BRACE2006951, BRACE2006982, BRACE2006986, BRACE2007013, BRACE2007068, BRACE2007087, BRACE2007138, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522,.BRACE200.7527,. BRACE2007538, BRACE2007563, BRACE2007567, BRACE2007577, BRACE2007621, BRACE2007640, BRACE2007646, BRACE2007663, BRACE2007682, BRACE2007685, BRACE2007708, BRACE2007727, BRACE2007749, BRACE2007760, BRACE2007761, BRACE2007764, BRACE2007767, BRACE2007769, BRACE2007784, BRACE2007786, BRACE2007798, BRACE2007799, BRACE2007801, BRACE2007836, BRACE2007868, BRACE2007888, BRACE2007902, BRACE2007920, BRACE2007944, BRACE2008008, BRACE2008015, BRACE2008021, BRACE2008097, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008488, BRACE2008495, BRACE2008500, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008847, BRACE2008857, BRACE2008861, BRACE2008867, BRACE2008881, BRACE2008883, BRACE2008917, BRACE2008919, BRACE2008948, BRACE2008960, BRACE2008968, BRACE2008999, BRACE2009014, BRACE2009016, BRACE2009031, BRACE2009053, BRACE2009122, BRACE2009131, BRACE2009185, BRACE2009188, BRACE2009212, BRACE2009221, BRACE2009235, BRACE2009238, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE 2011947, BRACE2012061, BRACE2012062, BRACE2012185, BRACE2012232, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013806, BRACE2013855, BRACE2013911, BRACE2014108, BRACE2014161, BRACE2014232, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025710, BRACE2025992, BRACE2026131, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY2000021, BRAMY2000025, BRAMY2000052, BRAMY2000076, BRAMY2000086, BRAMY2000151, BRAMY2000196, BRAMY2000231, BRAMY2000277, BRAMY2000388, BRAMY2000411, BRAMY2000508, BRAMY2000585, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007249, BRAMY2007255, BRAMY2007287, BRAMY2007308, BRAMY2007411, BRAMY2007458, BRAMY2007486, BRAMY2007546, BRAMY2007594, BRAMY2007610, BRAMY2007653, BRAMY2007753, BRAMY2007859, BRAMY2008333, BRAMY2008382, BRAMY2008563, BRAMY2008583, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2020050, BRAMY2020058, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2024004, BRAMY2024073, BRAMY2024312, BRAMY2024374, BRAMY2024390, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000083, BRAWH1000094, BRAWH1000116, BRAWH1000130, BRAWH1000157, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000177, BRAWH2000218, BRAWH2000232, BRAWH2000248, BRAWH2000256,. BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000554, BRAWH2000595, BRAWH2000633, BRAWH2000643, BRAWH2000651, BRAWH2000682, BRAWH2000697, BRAWH2000752, BRAWH2000859, BRAWH2000866, BRAWH2000892, BRAWH2000901, BRAWH2000926, BRAWH2000944, BRAWH2000984, BRAWH2001019, BRAWH2001052, BRAWH2001103, BRAWH2001129, BRAWH2001137, BRAWH2001159, BRAWH2001166, BRAWH2001186, BRAWH2001203, BRAWH2001238, BRAWH2001239, BRAWH2001255, BRAWH2001355, BRAWH2001378, BRAWH2001395, BRAWH2001406, BRAWH2001412, BRAWH2001418, BRAWH2001436, BRAWH2001438, BRAWH2001459, BRAWH2001461, BRAWH2001492, BRAWH2001503, BRAWH2001519, BRAWH2001530, BRAWH2001535, BRAWH2001589, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002963, BRAWH2002967, BRAWH2002976, BRAWH2003000, BRAWH2003135, BRAWH2003240, BRAWH2003307, BRAWH2003355, BRAWH2003366, BRAWH2003689, BRAWH2003693, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842, BRAWH2004884, BRAWH2004923, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007658, BRAWH2007808, BRAWH2007825, BRAWH2007862, BRAWH2007890, BRAWH2008058, BRAWH2008128, BRAWH2008219, BRAWH2008292, BRAWH2008297, BRAWH2008366, BRAWH2008380, BRAWH2008540, BRAWH2008577, BRAWH2008790, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010354, BRAWH2010364, BRAWH2010536, BRAWH2010552, BRAWH2010584, BRAWH2010618, BRAWH2010619, BRAWH2010754, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011823, BRAWH2011860, BRAWH2011920, BRAWH2011958, BRAWH2012054, BRAWH2012077, BRAWH2012162, BRAWH2012258, BRAWH2012326, BRAWH2012538, BRAWH2012540, BRAWH2012619, BRAWH2012698, BRAWH2012749, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014662, BRAWH2014717, BRAWH2014729, BRAWH2014876, BRAWH2014934, BRAWH2014954, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016785, BRAWH2016792, BRAWH2016800, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAwH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2-014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019934, BRCOC2020122, BRCOC2020142, BRHIP1000058, BRHIP1000072, BRHIP1000129, BRHIP2000021, BRHIP2000079, BRHIP2000087, BRHIP2000163, BRHIP2000210, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001686, BRHIP2001782, BRHIP2001802, BRHIP2001886, BRHIP2001927, BRHIP2002122, BRHIP2002143, BRHIP2002172, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020622, BRHIP2020695, BRHIP2020743, BRHIP2020799, BRHIP2020842, BRHIP2020859, BRHIP2020930, BRHIP2021102, BRHIP2021289, BRHIP2021615, BRHIP2021744, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2023071, BRHIP2023229, BRHIP2023272, BRHIP2023309, BRHIP2023369, BRHIP2023438, BRHIP2023695, BRHIP2023735, BRHIP2023762, BRHIP2023860, BRHIP2023869, BRHIP2024046, BRHIP2024165, BRHIP2024201, BRHIP2024347, BRHIP2024398, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002363, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000059, BRSSN1000092, BRSSN1000153, BRSSN2000097, BRSSN2000175, BRSSN2000197, BRSSN2000244, BRSSN2000293, BRSSN2000295, BRSSN2000518, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011738, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010923, BRSTN2011211, BRSTN2011597, BRSTN2011688, BRSTN2011799, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279,. BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009291, BRTHA3010135, BRTHA3010366, BRTHA3010469, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001226, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858, CTONG2001877, CTONG2001932, CTONG2001955, CTONG2002066, CTONG2002095, CTONG2002143, CTONG2002395, CTONG2002417, CTONG2002418, CTONG2002520, CTONG2002558, CTONG2002590, CTONG2002709, CTONG2002721, CTONG2002766, CTONG2002816, CTONG2002832, CTONG2002841, CTONG2002965, CTONG2002999, CTONG2003028, CTONG2003100, CTONG2003115, CTONG2003189, CTONG2003245, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006273, CTONG2006310, CTONG2006377, CTONG2006393, CTONG2006449, CTONG2006515, CTONG2006524, CTONG2006568, CTONG2006611, CTONG2006620, CTONG2006666, CTONG2006691, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007613, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008562, CTONG2008577, CTONG2008595, CTONG2008621, CTONG2008689, CTONG2008720, CTONG2008721, CTONG2008773, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077, CTONG2012101, CTONG2012123, CTONG2012158, CTONG2012230, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2013128, CTONG2013149, CTONG2013156, CTONG2013178, CTONG2013222, CTONG2013284, CTONG2013339, CTONG2013352, CTONG2013393, CTONG2013511, CTONG2013630, CTONG2013803, CTONG2013934, CTONG2013986, CTONG2014032, CTONG2014058, CTONG2014119, CTONG2014191, CTONG2014206, CTONG2014234, CTONG2014264, CTONG2014280, CTONG2014369, CTONG2014491, CTONG2014630, CTONG2014635, CTONG2014646, CTONG2014705, CTONG2014946, CTONG2014954, CTONG2014959, CTONG2014966, CTONG2014995, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018379, CTONG2018383, CTONG2018413,. CTONG2018483, CTONG2018629, CTONG2018632, CTONG2018652, CTONG2018653, CTONG2018655, CTONG2018735, CTONG2018808, CTONG2018900, CTONG2018976, CTONG2019029, CTONG2019144, CTONG2019232, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020445, CTONG2020638, CTONG2020806, CTONG2020831, CTONG2020974, CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142, HCHON1000176, HCHON1000191, HCHON2000038, HCHON2000062, HCHON2000087, HCHON2000100, HCHON2000156, HCHON2000199, HCHON2000226, HCHON2000265, HCHON2000271, HCHON2000315, HCHON2000322, HCHON2000344, HCHON2000364, HCHON2000373, HCHON2000395, HCHON2000417, HCHON2000418, HCHON2000473, HCHON2000475, HCHON2000503, HCHON2000626, HCHON2000660, HCHON2000663, HCHON2000673, HCHON2000699, HCHON2000705, HCHON2000815, HCHON2000817, HCHON2000818, HCHON2000819, HCHON2000851, HCHON2000898, HCHON2000935, HCHON2000956, HCHON2000994, HCHON2001084, HCHON2001108, HCHON2001110, HCHON2001116, HCHON2001161, HCHON2001214, HCHON2001233, HCHON2001269, HCHON2001359, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002247, HCHON2002354, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005802, HCHON2005921, HCHON2005987, HCHON2006250, HCHON2006459, HCHON2006714, HCHON2006722, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000074, HEART1000088, HEART1000099, HEART1000102, HEART1000118, HEART1000132, HEART1000139, HEART1000142, HEART1000151, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000070, HHDPC2000102, HHDPC2000115, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024, HLUNG1000030, HLUNG1000034, HLUNG1000037, HLUNG1000053, HLUNG1000064, HLUNG1000076, HLUNG1000084, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000926, HLUNG2000955, HLUNG2000970, HLUNG2001013, HLUNG2001126, HLUNG2001144, HLUNG2001146, HLUNG2001214, HLUNG2001459, HLUNG2001507, HLUNG2001633, HLUNG2001677, HLUNG2001712, HLUNG2001814, HLUNG2001996, HLUNG2002006, HLUNG2002050, HLUNG2002085, HLUNG2002145, HLUNG2002295, HLUNG2002303, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004170, HLUNG2004217, HLUNG2004273, HLUNG2004388, HLUNG2004416, HLUNG2004521, HLUNG2004534, HLUNG2004684, HLUNG2004707, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000135, HSYRA2000137, HSYRA2000224, HSYRA2000237, HSYRA2000253, HSYRA2000255, HSYRA2000371, HSYRA2000424, HSYRA2000507, HSYRA2000510, HSYRA2000605, HSYRA2000629, HSYRA2000706, HSYRA2000760, HSYRA2000792, HSYRA2000828, HSYRA2000832, HSYRA2000925, HSYRA2000927, HSYRA2001003, HSYRA2001105, HSYRA2001142, HSYRA2001153, HSYRA2001232, HSYRA2001255, HSYRA2001332, HSYRA2001353, HSYRA2001420, HSYRA2001484, HSYRA2001552, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000207, IMR321000230, IMR321000266, IMR322000010, IMR322000072, IMR322000107, IMR322000152, IMR322000164, IMR322000178, IMR322000302, IMR322000374, IMR322000379, IMR322000383, IMR322000418, IMR322000518, IMR322000604, IMR322000609, IMR322000656, IMR322000672, IMR322000742, IMR322000775, IMR322000791, IMR322000811, IMR322000838, IMR322000859, IMR322000863, IMR322000919, IMR322000953, IMR322000984, IMR322001018, IMR322001049, IMR322001167, IMR322001185, IMR322001245, IMR322001317, IMR322001318, IMR322001322, IMR322001438, IMR322001452, IMR322001491, IMR322001534, IMR322001665, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002483, KIDNE2002725, KIDNE2002768, KIDNE2002795, KIDNE2002798, KIDNE2002839, KIDNE2002845, KIDNE2002882, KIDNE2002980, KIDNE2002991, KIDNE2003185, KIDNE2003188, KIDNE2003233, KIDNE2003305, KIDNE2003316, KIDNE2003335, KIDNE2003357, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004344, KIDNE2004394, KIDNE2004411, KIDNE2004519, KIDNE2004520, KIDNE2004531, KIDNE2004534, KIDNE2004562, KIDNE2004579, KIDNE2004681, KIDNE2004828, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006580, KIDNE2006652, KIDNE2006687, KIDNE2006733, KIDNE2006760, KIDNE2006775, KIDNE2006820, KIDNE2007005, KIDNE2007040, KIDNE2007077, KIDNE2007186, KIDNE2007222, KIDNE2007328, KIDNE2007356, KIDNE2007422, KIDNE2007569, KIDNE2007810, KIDNE2007811, KIDNE2007910, KIDNE2007944, KIDNE2007954, KIDNE2008022, KIDNE2008048, KIDNE2008069, KIDNE2008072, KIDNE2008116, KIDNE2008117, KIDNE2008218, KIDNE2008315, KIDNE2008362, KIDNE2008378, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000079, MESAN1000080, MESAN1000121, MESAN1000126, MESAN1000147, MESAN1000180, MESAN2000023, MESAN2000026, MESAN2000167, MESAN2000264, MESAN2000291, MESAN2000337, MESAN2000434, MESAN2000457, MESAN2000462, MESAN2000501, MESAN2000608, MESAN2000620, MESAN2000711, MESAN2000815, MESAN2000894, MESAN2000909, MESAN2001154, MESAN2001627, MESAN2001770, MESAN2001979, MESAN2001980, MESAN2002113, MESAN2002122, MESAN2002147, MESAN2002424, MESAN2002687, MESAN2002709, MESAN2002940, MESAN2002978, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2005284, MESAN2005303, MESAN2005371, MESAN2005633, MESAN2005724, MESAN2005808, MESAN2005811, MESAN2005957, MESAN2005958, MESAN2006022, MESAN2006043, MESAN2006328, MESAN2006580, MESAN2006599, MESAN2006743, MESAN2006953, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000047, NB9N41000121, NB9N41000135, NB9N41000142, NB9N41000146, NB9N41000340, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000606, NHNPC2000877, NHNPC2001108, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000023, NT2NE1000059, NT2NE1000063, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000284, NT2NE2000299, NT2NE2000356, NT2NE2000369, NT2NE2000374, NT2NE2000384, NT2NE2000390, NT2NE2000455, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864, NT2NE2000913, NT2NE2000950, NT2NE2000951, NT2NE2000963, NT2NE2000980, NT2NE2001005, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001524, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003308, NT2NE2003309, NT2NE2003315, NT2NE2003408, NT2NE2003457, NT2NE2003540, NT2NE2003571, NT2NE2003705, NT2NE2003752, NT2NE2003887, NT2NE2003921, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006075, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006942, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009523, NT2NE2009608, NT2NE2009691, NT2NE2010105, NT2NE2010150, NT2NE2010400, NT2NE2010632, NT2NE2010633, NT2NE2010654, NT2NE2010688, NT2NE2010781, NT2NE2010854, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000027, NT2RI1000035, NT2RI1000164, NT2RI1000172, NT2RI2000004, NT2RI2000007, NT2RI2000107, NT2RI2000117, NT2RI2000128, NT2RI2000167, NT2RI2000173, NT2RI2000187, NT2RI2000263, NT2RI2000270, NT2RI2000276, NT2RI2000284, NT2RI2000313, NT2RI2000341, NT2RI2000421, NT2RI2000480, NT2RI2000575, NT2RI2000588, NT2RI2000597, NT2RI2000669, NT2RI2000671, NT2RI2000688, NT2RI2000689, NT2RI2000704, NT2RI2000738, NT2RI2000775, NT2RI2000822, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001230, NT2RI2001235, NT2RI2001244, NT2RI2001307, NT2RI2001409, NT2RI2001410, NT2RI2001425, NT2RI2001449, NT2RI2001474, NT2RI2001519, NT2RI2001595, NT2RI2001624, NT2RI2001726, NT2RI2001769, NT2RI2001782, NT2RI2001836, NT2RI2001846, NT2RI2001866, NT2RI2001910, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002549, NT2RI2002564, NT2RI2002585, NT2RI2002592, NT2RI2002602, NT2RI2002729, NT2RI2002802, NT2RI2002822, NT2RI2002852, NT2RI2002865, NT2RI2002923, NT2RI2002926, NT2RI2002970, NT2RI2003011, NT2RI2003067, NT2RI2003184, NT2RI2003205, NT2RI2003222, NT2RI2003338, NT2RI2003344, NT2RI2003360, NT2RI2003392, NT2RI2003399, NT2RI2003419, NT2RI2003481, NT2RI2003487, NT2RI2003655, NT2RI2003667, NT2RI2003678, NT2RI2003884, NT2RI2004059, NT2RI2004078, NT2RI2004079, NT2RI2004093, NT2RI2004136, NT2RI2004157, NT2RI2004188, NT2RI2004190, NT2RI2004284, NT2RI2004290, NT2RI2004304, NT2RI2004312, NT2RI2004381, NT2RI2004442, NT2RI2004468, NT2RI2004535, NT2RI2004606, NT2RI2004608, NT2RI2004618, NT2RI2004783, NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005166, NT2RI2005239, NT2RI2005246, NT2RI2005315, NT2RI2005358, NT2RI2005382, NT2RI2005473, NT2RI2005520, NT2RI2005621, NT2RI2005628, NT2RI2005647, NT2RI2005670, NT2RI2005710, NT2RI2005772, NT2RI2005814, NT2RI2005851, NT2RI2005966, NT2RI2006070, NT2RI2006071, NT2RI2006081, NT2RI2006126, NT2RI2006127, NT2RI2006257, NT2RI2006271, NT2RI2006294, NT2RI2006345, NT2RI2006403, NT2RI2006445, NT2RI2006487, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006973, NT2RI2007046, NT2RI2007048, NT2RI2007084, NT2RI2007096, NT2RI2007116, NT2RI2007148, NT2RI2007214, NT2RI2007303, NT2RI2007334, NT2RI2007386, NT2RI2007406, NT2RI2007439, NT2RI2007445, NT2RI2007469, NT2RI2007498, NT2RI2007507, NT2RI2007593, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008141, NT2RI2008188, NT2RI2008204, NT2RI2008233, NT2RI2008455, NT2RI2008481, NT2RI2008502, NT2RI2008598, NT2RI2008622, NT2RI2008656, NT2RI2008714, NT2RI2008801, NT2RI2008812, NT2RI2008841, NT2RI2008952, NT2RI2009005, NT2RI2009066, NT2RI2009083, NT2RI2009101, NT2RI2009103, NT2RI2009144, NT2RI2009151, NT2RI2009215, NT2RI2009289, NT2RI2009301, NT2RI2009517, NT2RI2009583, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007543, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000032, NT2RP6000033, NT2RP6000039, NT2RP6000043, NT2RP6000059, NT2RP6000060, NT2RP6000077, NT2RP6000085, NT2RP6000086, NT2RP6000100, NT2RP6000119, NT2RP6000127, NT2RP6000130, NT2RP6000140, NT2RP6000150, NT2RP7000041, NT2RP7000069, NT2RP7000086, NT2RP7000109, NT2RP7000112, NT2RP7000259, NT2RP7000311, NT2RP7000359, NT2RP7000364, NT2RP7000380, NT2RP7000391, NT2RP7000579, NT2RP7000586, NT2RP7000600, NT2RP7000812, NT2RP7000926, NT2RP7001074, NT2RP7001080, NT2RP7001156, NT2RP7001306, NT2RP7001319, NT2RP7001335, NT2RP7001364, NT2RP7001475, NT2RP7001532, NT2RP7001602, NT2RP7001678, NT2RP7001856, NT2RP7001962, NT2RP7002064, NT2RP7002151, NT2RP7002163, NT2RP7002243, NT2RP7002282, NT2RP7002376, NT2RP7002449, NT2RP7002450, NT2RP7002478, NT2RP7002554, NT2RP7002594, NT2RP7002603, NT2RP7002619, NT2RP7002650, NT2RP7002779, NT2RP7002829, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003439, NT2RP7003629, NT2RP7003632, NT2RP7003647, NT2RP7003680, NT2RP7004027, NT2RP7004080, NT2RP7004114, NT2RP7004233, NT2RP7004260, NT2RP7004358, NT2RP7004396, NT2RP7004428, NT2RP7004541, NT2RP7004559, NT2RP7004641, NT2RP7004656, NT2RP7004687, NT2RP7004722, NT2RP7004766, NT2RP7004790, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005750, NT2RP7005846, NT2RP7006033, NT2RP7006075, NT2RP7006141, NT2RP7006160, NT2RP7006162, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006853, NT2RP7006886, NT2RP7006887, NT2RP7006980, NT2RP7006995, NT2RP7007154, NT2RP7007177, NT2RP7007221, NT2RP7007226, NT2RP7007252, NT2RP7007310, NT2RP7007359, NT2RP7007381, NT2RP7007406, NT2RP7007480, NT2RP7007504, NT2RP7007537, NT2RP7007580, NT2RP7007594, NT2RP7007610, NT2RP7007617, NT2RP7007643, NT2RP7007766, NT2RP7007925, NT2RP7007930, NT2RP7007975, NT2RP7008013, NT2RP7008133, NT2RP7008137, NT2RP7008142, NT2RP7008144, NT2RP7008161, NT2RP7008315, NT2RP7008360, NT2RP7008396, NT2RP7008406, NT2RP7008435, NT2RP7008454, NT2RP7008487, NT2RP7008543, NT2RP7008544, NT2RP7008550, NT2RP7008557, NT2RP7008623, NT2RP7008657, NT2RP7008720, NT2RP7008855, NT2RP7008863, NT2RP7009012, NT2RP7009019, NT2RP7009147, NT2RP7009149, NT2RP7009168, NT2RP7009215, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009466, NT2RP7009502, NT2RP7009507, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG2000040, NTONG2000107, NTONG2000231, NTONG2000363, NTONG2000413, NTONG2000492, NTONG2000499, NTONG2000531, NTONG2000583, NTONG2000800, NTONG2000858, NTONG2000876, NTONG2000966, NTONG2001014, NTONG2001079, NTONG2001137, NTONG2001157, NTONG2001173, NTONG2001220, NTONG2001222, NTONG2001362, NTONG2001428, NTONG2001532, NTONG2001567, NTONG2001587, NTONG2001619, NTONG2001642, NTONG2001657, NTONG2001676, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005497, NTONG2005577, NTONG2005657, NTONG2005801, NTONG2005822, NTONG2006099, NTONG2006187, NTONG2006301, NTONG2006324, NTONG2006354, NTONG2006440, NTONG2006484, NTONG2006501, NTONG2006646, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000054, OCBBF1000085, OCBBF1000086, OCBBF1000091, OCBBF1000118, OCBBF1000119, OCBBF1000122, OCBBF1000130, OCBBF1000138, OCBBF1000145, OCBBF1000175, OCBBF1000192, OCBBF1000254, OCBBF1000315, OCBBF2000231, OCBBF2000287, OCBBF2000452, OCBBF2000522, OCBBF2000677, OCBBF2000703, OCBBF2000712, OCBBF2000719, OCBBF2000824, OCBBF2000982, OCBBF2000998, OCBBF2001075, OCBBF2001101, OCBBF2001124, OCBBF2001140, OCBBF2001176, OCBBF2001186, OCBBF2001196, OCBBF2001307, OCBBF2001389, OCBBF2001408, OCBBF2001416, OCBBF2001473, OCBBF2001494, OCBBF2001527, OCBBF2001528, OCBBF2001586, OCBBF2001681, OCBBF2001706, OCBBF2001910, OCBBF2001931, OCBBF2001961, OCBBF2001983, OCBBF2002015, OCBBF2002083, OCBBF2002086, OCBBF2002124, OCBBF2002161, OCBBF2002357, OCBBF2002615, OCBBF2002805, OCBBF2002865, OCBBF2002920, OCBBF2002980, OCBBF2003028, OCBBF2003091, OCBBF2003327, OCBBF2003543, OCBBF2003593, OCBBF2003744, OCBBF2003819, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2005066, OCBBF2005077, OCBBF2005161, OCBBF2005343, OCBBF2005349, OCBBF2005373, OCBBF2005420, OCBBF2005433, OCBBF2005476, OCBBF2005546, OCBBF2005843, OCBBF2005901, OCBBF2006005, OCBBF2006030, OCBBF2006058, OCBBF2006148, OCBBF2006151, OCBBF2006154, OCBBF2006214, OCBBF2006241, OCBBF2006388, OCBBF2006438, OCBBF2006567, OCBBF2006624, OCBBF2006639, OCBBF2006660, OCBBF2006972, OCBBF2006987, OCBBF2007039, OCBBF2007051, OCBBF2007068, OCBBF2007114, OCBBF2007121, OCBBF2007184, OCBBF2007194, OCBBF2007224, OCBBF2007238, OCBBF2007354, OCBBF2007414, OCBBF2007428, OCBBF2007478, OCBBF2007485, OCBBF2007520, OCBBF2007555, OCBBF2007610, OCBBF2007622, OCBBF2007756, OCBBF2007931, OCBBF2007946, OCBBF2008116, OCBBF2008138, OCBBF2008144, OCBBF2008283, OCBBF2008330, OCBBF2008366, OCBBF2008483, OCBBF2008511, OCBBF2008520, OCBBF2008569, OCBBF2008586, OCBBF2008640, OCBBF2008691, OCBBF2008724, OCBBF2008760, OCBBF2008768, OCBBF2008775, OCBBF2008790, OCBBF2008814, OCBBF2008822, OCBBF2009095, OCBBF2009115, OCBBF2009242, OCBBF2009352, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010858, OCBBF2010863, OCBBF2010931, OCBBF2010945, OCBBF2010978, OCBBF2010989, OCBBF2011137, OCBBF2011160, OCBBF2011177, OCBBF2011228, OCBBF2011283, OCBBF2011311, OCBBF2011669, OCBBF2011685, OCBBF2011706, OCBBF2011722, OCBBF2011767, OCBBF2011855, OCBBF2011872, OCBBF2011897, OCBBF2011914, OCBBF2011960, OCBBF2011981, OCBBF2012001, OCBBF2012028, OCBBF2012039, OCBBF2012139, OCBBF2012262, OCBBF2012320, OCBBF2012436, OCBBF2012525, OCBBF2012704, OCBBF2012714, OCBBF2012755, OCBBF2012812, OCBBF2012881, OCBBF2012936, OCBBF2012990, OCBBF2013049, OCBBF2013091, OCBBF2013123, OCBBF2013127, OCBBF2013208, OCBBF2013285, OCBBF2013319, OCBBF2013324, OCBBF2013366, OCBBF2013507, OCBBF2013721, OCBBF2013883, OCBBF2014052, OCBBF2014089, OCBBF2014576, OCBBF2014707, OCBBF2014745, OCBBF2014828, OCBBF2015031, OCBBF2015081, OCBBF2015115, OCBBF2015232, OCBBF2015233, OCBBF2015285, OCBBF2015334, OCBBF2015335, OCBBF2015506, OCBBF2015645, OCBBF2015659, OCBBF2015797, OCBBF2015931, OCBBF2015980, OCBBF2016038, OCBBF2016467, OCBBF2016590, OCBBF2016591, OCBBF2016612, OCBBF2016689, OCBBF2016690, OCBBF2016729, OCBBF2016841, OCBBF2017055, OCBBF2017069, OCBBF2017306, OCBBF2017325, OCBBF2017489, OCBBF2017516, OCBBF2017665, OCBBF2017815, OCBBF2017882, OCBBF2017920, OCBBF2018014, OCBBF2018084, OCBBF2018167, OCBBF2018206, OCBBF2018229, OCBBF2018362, OCBBF2018380, OCBBF2018563, OCBBF2018581, OCBBF2018618, OCBBF2018663, OCBBF2018687, OCBBF2018827, OCBBF2018828, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021323, OCBBF2021518, OCBBF2021788, OCBBF2021833, OCBBF2022351, OCBBF2022573, OCBBF2022574, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030, PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006709, PEBLM2006912, PEBLM2007112, PEBLM2007140, PEBLM2007188, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000414, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001185, PLACE6001294, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010925, PLACE6010936, PLACE6010991, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016254, PLACE6016383, PLACE6016984, PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000144, PROST1000145, PROST1000152, PROST1000167, PROST1000184, PROST1000217, PROST1000220, PROST1000226, PROST1000246, PROST1000262, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000564, PROST1000575, PROST2000036, PROST2000053, PROST2000067, PROST2000105, PROST2000143, PROST2000162, PROST2000176, PROST2000203, PROST2000206, PROST2000212, PROST2000267, PROST2000273, PROST2000274, PROST2000277, PROST2000284, PROST2000325, PROST2000432, PROST2000452, PROST2000463, PROST2000470, PROST2000505, PROST2000567, PROST2000572, PROST2000717, PROST2000760, PROST2000761, PROST2000835, PROST2000961, PROST2001002, PROST2001116, PROST2001138, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001899, PROST2001997, PROST2001998, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004727, PROST2004739, PROST2004742, PROST2004744, PROST2004817, PROST2005067, PROST2005076, PROST2005093, PROST2005121, PROST2005131, PROST2005228, PROST2005272, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006260, PROST2006282, PROST2006343, PROST2006450, PROST2006491, PROST2006510, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008447, PROST2008468, PROST2008472, PROST2008489, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010400, PROST2010545, PROST2010579, PROST2010606, PROST2010885, PROST2011012, PROST2011105, PROST2011297, PROST2011410, PROST2011439, PROST2011631, PROST2011660, PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2019164, PROST2019296, PROST2019398, PROST2019487, PROST2019781, PUAEN1000039, PUAEN1000065, PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000394, PUAEN2000497, PUAEN2000594, PUAEN2000684, PUAEN2001260, PUAEN2001526, PUAEN2001586, PUAEN2001882, PUAEN2002473, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009795, PUAEN2009852, PUAEN2010346, PUAEN2010824, RECTM1000141, RECTM2000220, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000030, SKMUS1000064, SKMUS1000067, SKMUS1000102, SKMUS1000104, SKMUS1000110, SKMUS1000118, SKMUS1000124, SKMUS1000129, SKMUS1000138, SKMUS1000176, SKMUS1000177, SKMUS2000061, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000390, SKMUS2000416, SKMUS2000458, SKMUS2000467, SKMUS2000468, SKMUS2000515, SKMUS2000679, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000251, SKNMC1000264, SKNMC2000224, SKNMC2000256, SKNMC2000322, SKNMC2000374, SKNMC2000583, SKNMC2000593, SKNMC2000612, SKNMC2000622, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000101, SKNSH1000174, SKNSH1000301, SKNSH1000308, SKNSH1000416, SKNSH2000051, SKNSH2000101, SKNSH2000151, SKNSH2000250, SKNSH2000319, SKNSH2000482, SKNSH2000516, SKNSH2000550, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT1000192, SMINT2000018, SMINT2000032, SMINT2000040, SMINT2000060, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000659, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2001950, SMINT2002126, SMINT2002202, SMINT2002210, SMINT2002224, SMINT2002281, SMINT2002311, SMINT2002314, SMINT2002328, SMINT2002414, SMINT2002457, SMINT2002743, SMINT2002778, SMINT2002880, SMINT2002882, SMINT2002899, SMINT2002974, SMINT2002976, SMINT2003003, SMINT2003128, SMINT2003150, SMINT2003317, SMINT2003386, SMINT2003423, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004781, SMINT2004891, SMINT2004909, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006801, SMINT2007062, SMINT2007101, SMINT2007140, SMINT2007187, SMINT2007219, SMINT2007294, SMINT2007365, SMINT2007391, SMINT2007433, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142, SMINT2019153, SMINT2019200, SPLEN1000013, SPLEN1000041, SPLEN1000056, SPLEN1000087, SPLEN1000091, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2000874, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001710, SPLEN2001761, SPLEN2001781, SPLEN2001881, SPLEN2001912, SPLEN2001945, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678,.SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611, SPLEN2004662, SPLEN2004844, SPLEN2004880, SPLEN2004984, SPLEN2005227, SPLEN2005272, SPLEN2005416, SPLEN2005429, SPLEN2005450, SPLEN2005474, SPLEN2005560, SPLEN2005727, SPLEN2005783, SPLEN2005790, SPLEN2005806, SPLEN2005818, SPLEN2005838, SPLEN2005927, SPLEN2005939, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2008007, SPLEN2008135, SPLEN2008164, SPLEN2008317, SPLEN2008496, SPLEN2008591, SPLEN2008786, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013857, SPLEN2013860, SPLEN2013910, SPLEN2013923, SPLEN2014041, SPLEN2014063, SPLEN2014073, SPLEN2014119, SPLEN2014136, SPLEN2014199, SPLEN2014210, SPLEN2014293, SPLEN2014318, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019349, SPLEN2019375, SPLEN2019379, SPLEN2019446, SPLEN2019471, SPLEN2019480, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028417, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001239, SYNOV2001251, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009139, SYNOV4009298, SYNOV4009575, T1ESE2000116, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN2000139,TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000117, TESTI2000120, TESTI2000147, TESTI2000154, TESTI2000172, TESTI2000177, TESTI2000179, TESTI2000207, TESTI2000208, TESTI2000221, TESTI2000253, TESTI2000272, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001120, TESTI2001134, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767, TESTI2005775, TESTI2005788, TESTI2005827, TESTI2005835, TESTI2005860, TESTI2005892, TESTI2005911, TESTI2005933, TESTI2005937, TESTI2005948, TESTI2005979, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311,TESTI2007320, TESTI2007346, TESTI2007358, TESTI2007402, TESTI2007405, TESTI2007422, TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009577, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2017954, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4003944, TESTI4004003, TESTI4004031, TESTI4004200, TESTI4004210, TESTI4004539, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000025, THYMU1000029, THYMU1000032, THYMU1000033, THYMU1000060, THYMU1000074, THYMU1000083, THYMU1000098, THYMU1000105, THYMU1000109, THYMU1000136, THYMU1000142, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000382, THYMU1000393, THYMU1000394, THYMU1000399, THYMU1000426, THYMU1000428, THYMU1000440, THYMU1000459, THYMU1000473, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002490, THYMU2002548, THYMU2002695, THYMU2002745, THYMU2002789, THYMU2002792, THYMU2002815, THYMU2002852, THYMU2002910, THYMU2002950, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004508, THYMU2004512, THYMU2004635, THYMU2004639, THYMU2004668, THYMU2004677, THYMU2004688, THYMU2004693, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007036, THYMU2007060, THYMU2007065, THYMU2007095, THYMU2007146, THYMU2007179, THYMU2007258, THYMU2007307, THYMU2007308, THYMU2007339, THYMU2007350, THYMU2007384, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017831, THYMU2017844, THYMU2017878, THYMU2017948, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2001327, TLIVE2001616, TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000038, TRACH1000057, TRACH1000074, TRACH1000100, TRACH1000106, TRACH1000125, TRACH1000140, TRACH1000144, TRACH1000193, TRACH1000205, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000393, TRACH2000411, TRACH2000420, TRACH2000472, TRACH2000496, TRACH2000497, TRACH2000502, TRACH2000522, TRACH2000540, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000923, TRACH2000944, TRACH2000981, TRACH2000986, TRACH2001037, TRACH2001060, TRACH2001085, TRACH2001101, TRACH2001121, TRACH2001147, TRACH2001154, TRACH2001188, TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001474, TRACH2001525, TRACH2001532, TRACH2001549, TRACH2001592, TRACH2001596, TRACH2001607, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003323, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008112, TRACH2008113, TRACH2008127, TRACH2008130, TRACH2008278, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000186, UMVEN2000046, UMVEN2000069, UMVEN2000121, UMVEN2000133, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000218, UTERU2000238, UTERU2000243, UTERU2000260, UTERU2000263, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299, UTERU2004520, UTERU2004564, UTERU2004688, UTERU2004698, UTERU2004861, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005593, UTERU2005601, UTERU2005664, UTERU2005822, UTERU2005903, UTERU2005905, UTERU2006103, UTERU2006115, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011962, UTERU2011968, UTERU2012031, UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013231, UTERU2013262, UTERU2013280, UTERU2013322, UTERU2013483, UTERU2013491, UTERU2013502, UTERU2013926, UTERU2013976, UTERU2014001, UTERU2014024, UTERU2014223, UTERU2014398, UTERU2014601, UTERU2014631, UTERU2014678, UTERU2014728, UTERU2014898, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016822, UTERU2016896, UTERU2016902, UTERU2016979, UTERU2016981, UTERU2017123, UTERU2017303, UTERU2017421, UTERU2017492, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the FEHRT, FEKID, FELIV, FELNG, HCASM, HCHON, and HEART libraries were analyzed for expression frequency. The analysis results are described below. Each is a relative value, where the expression level over all tissues is taken as 100. The larger this value, the greater the level of expression.

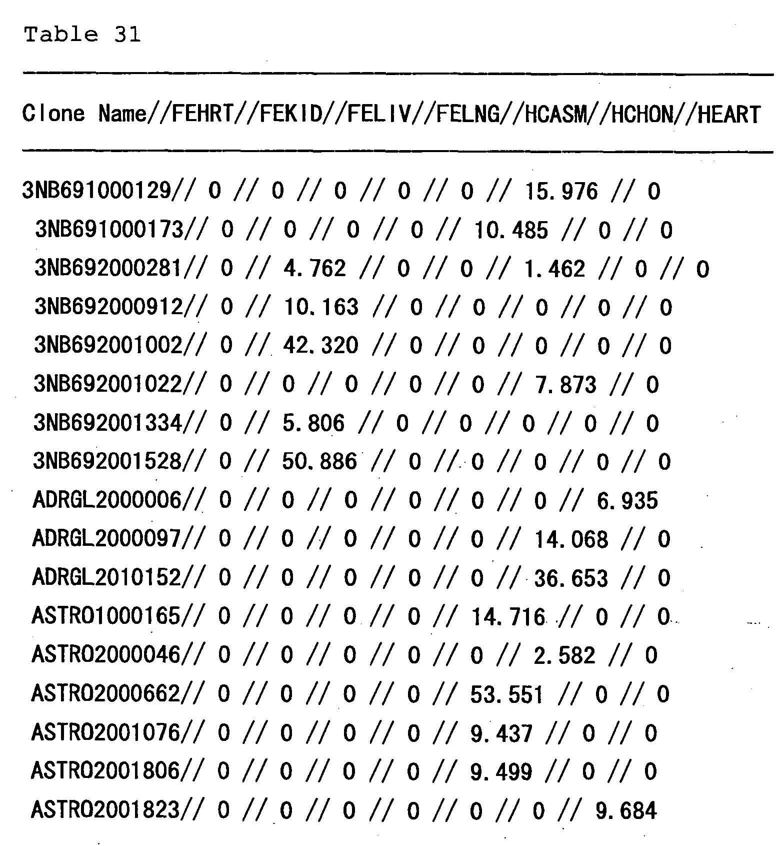

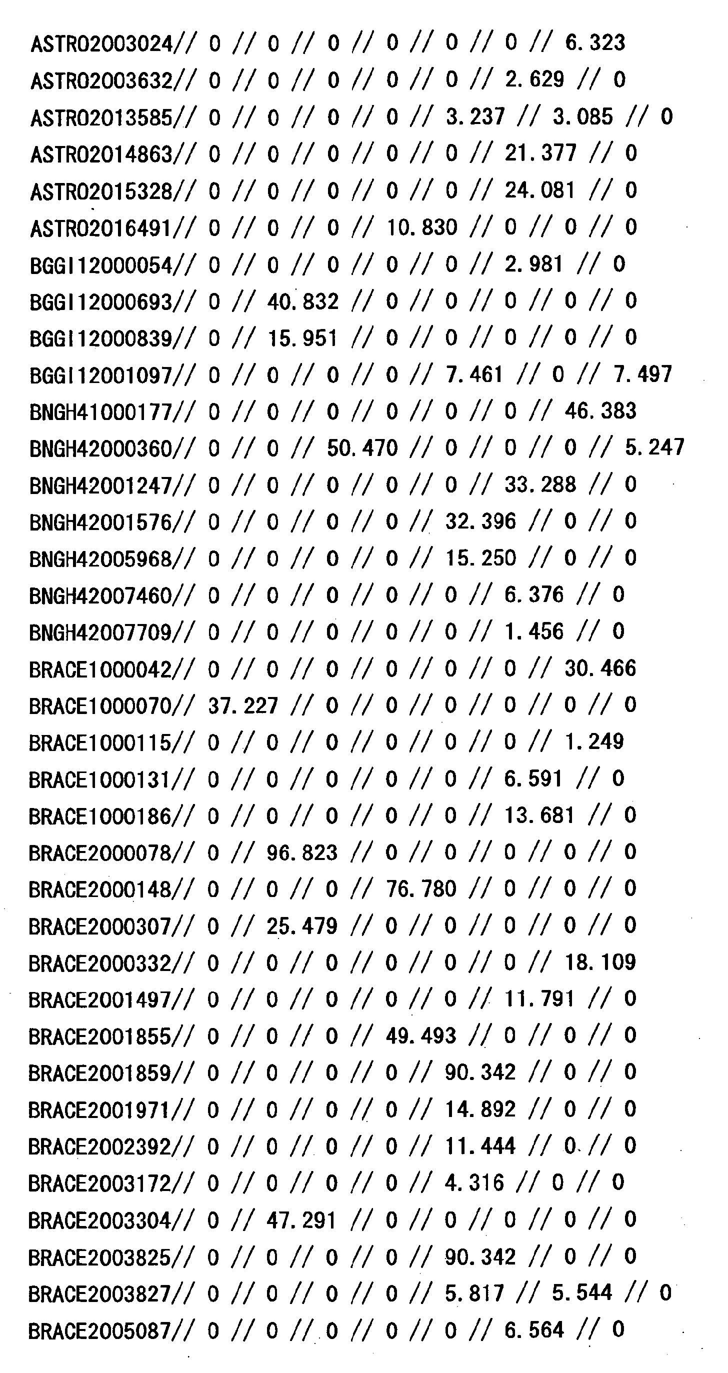

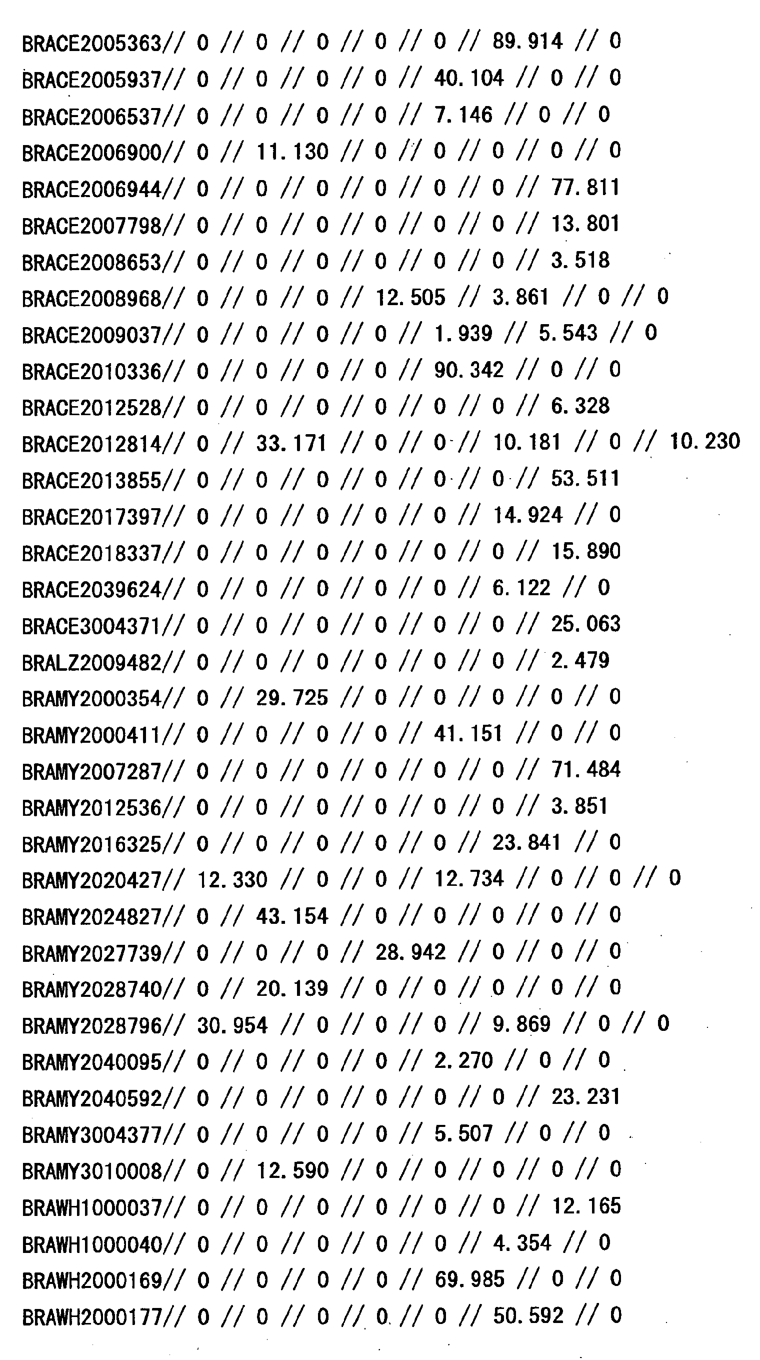

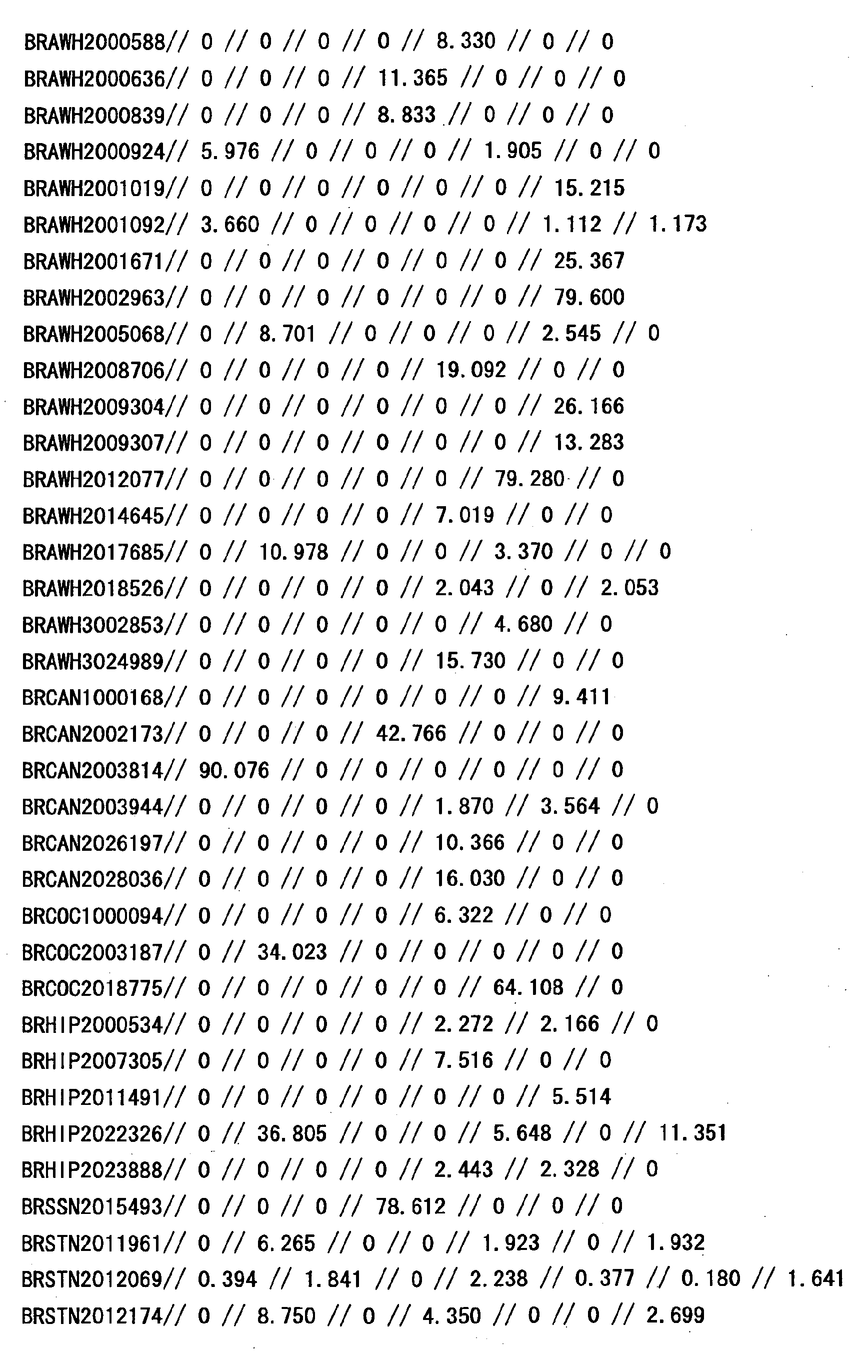

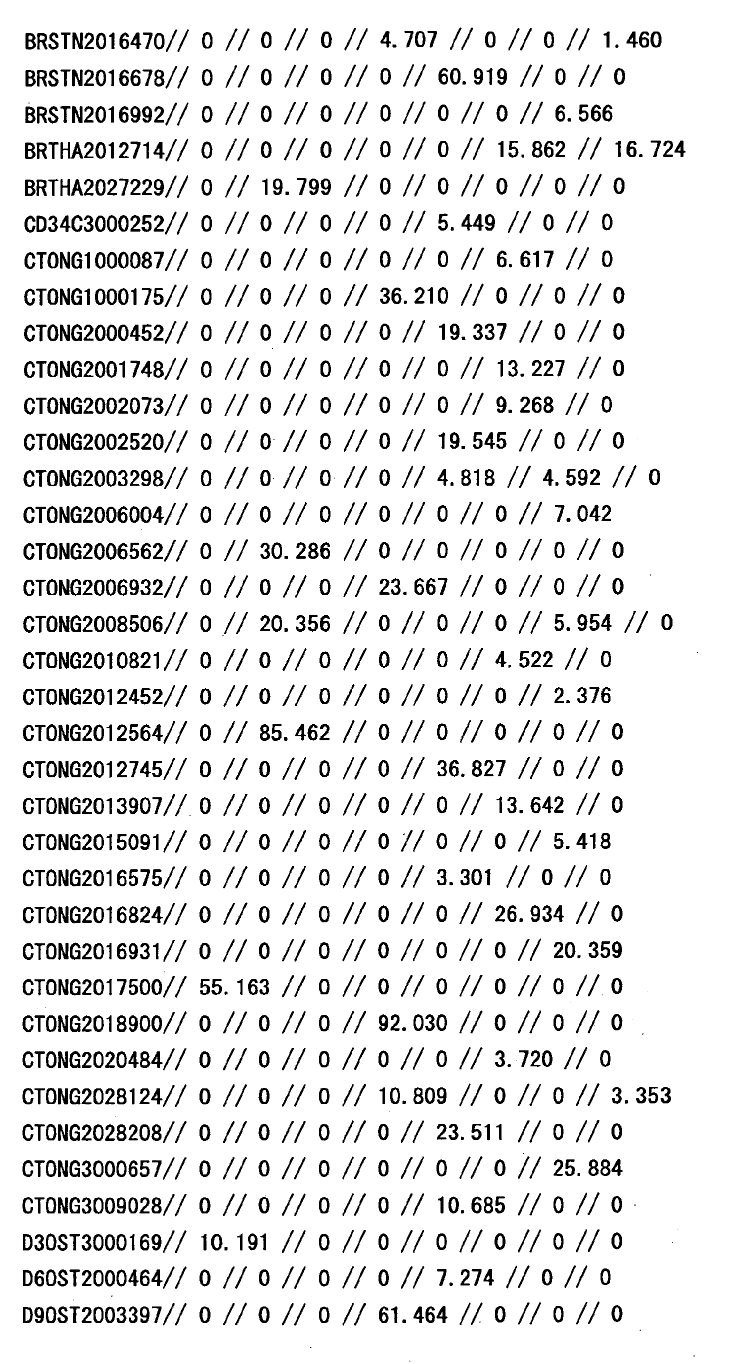

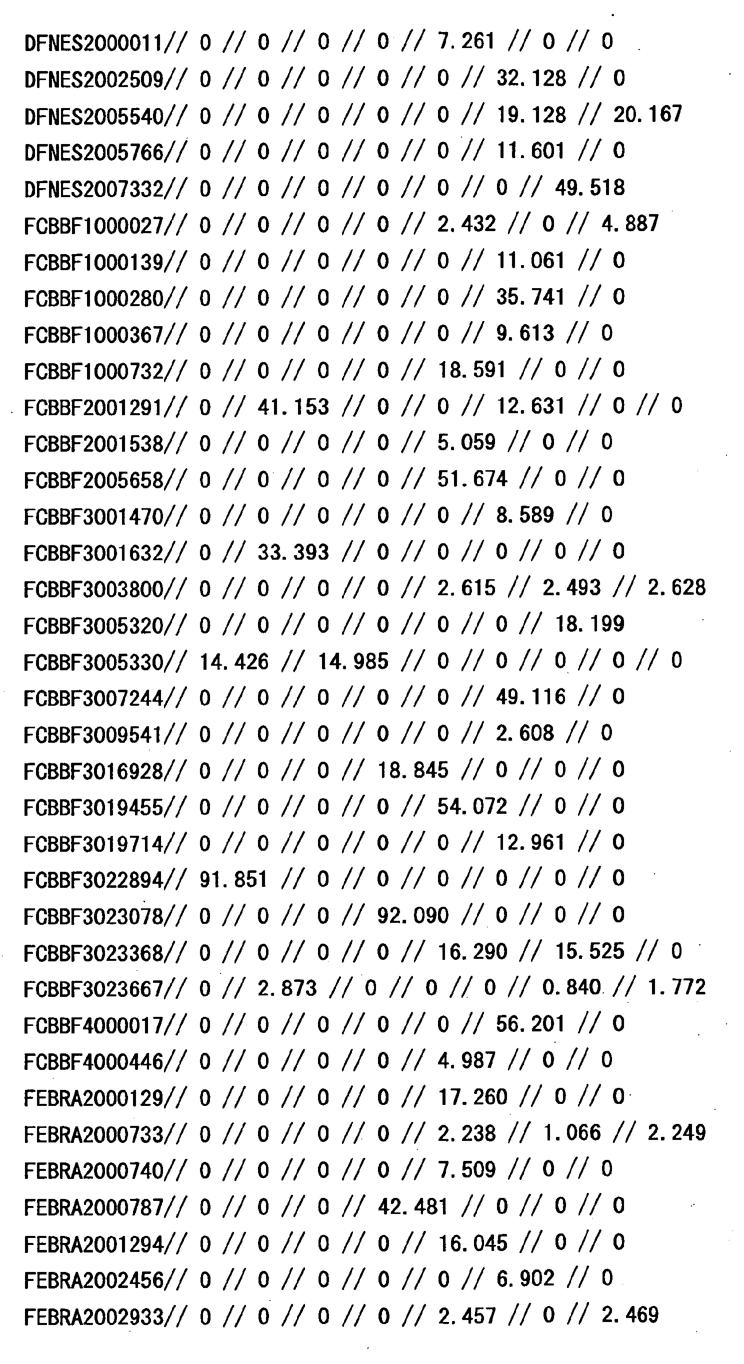

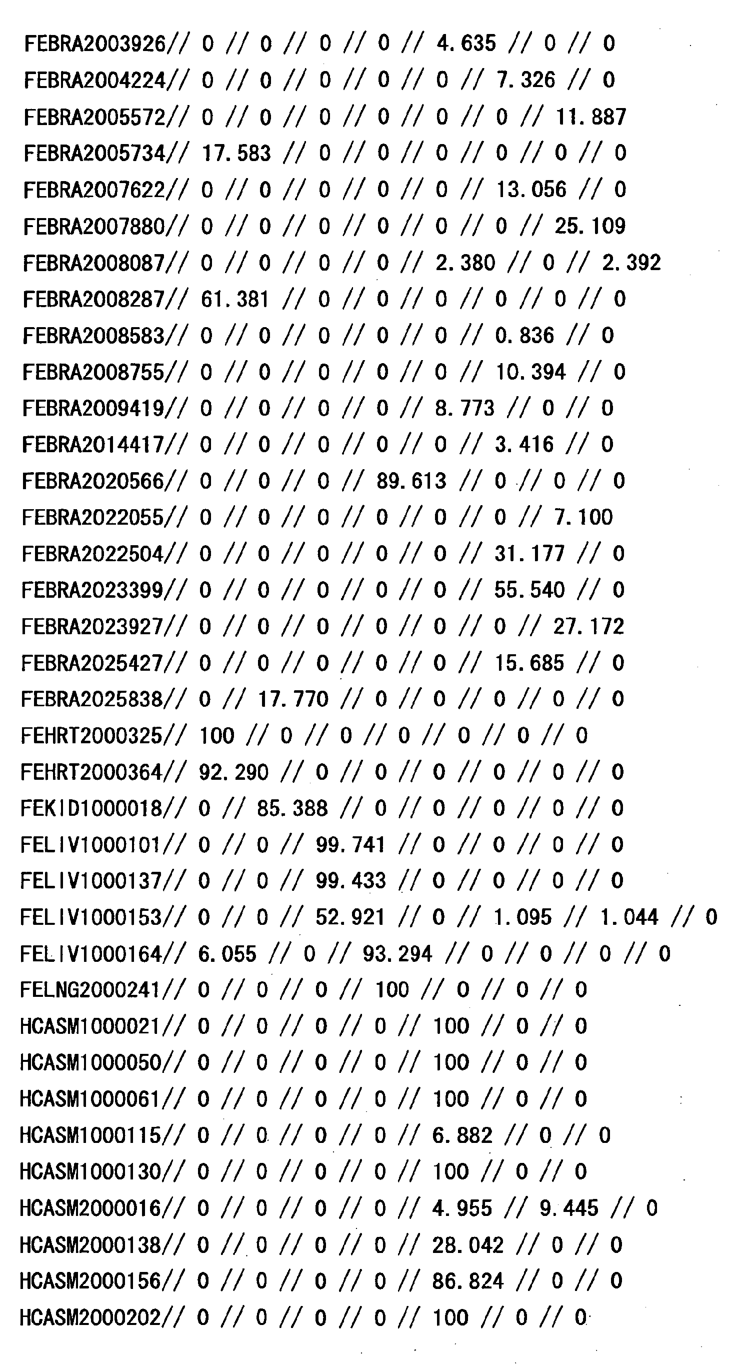

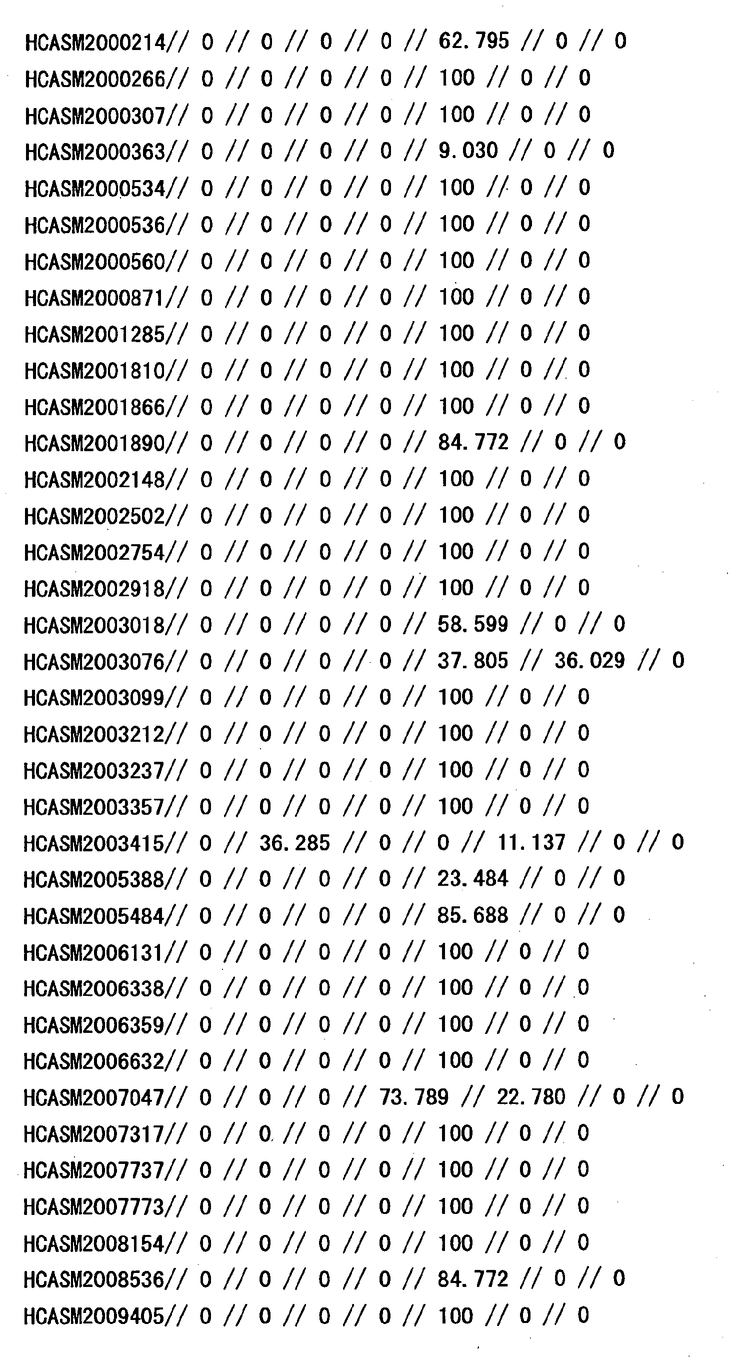

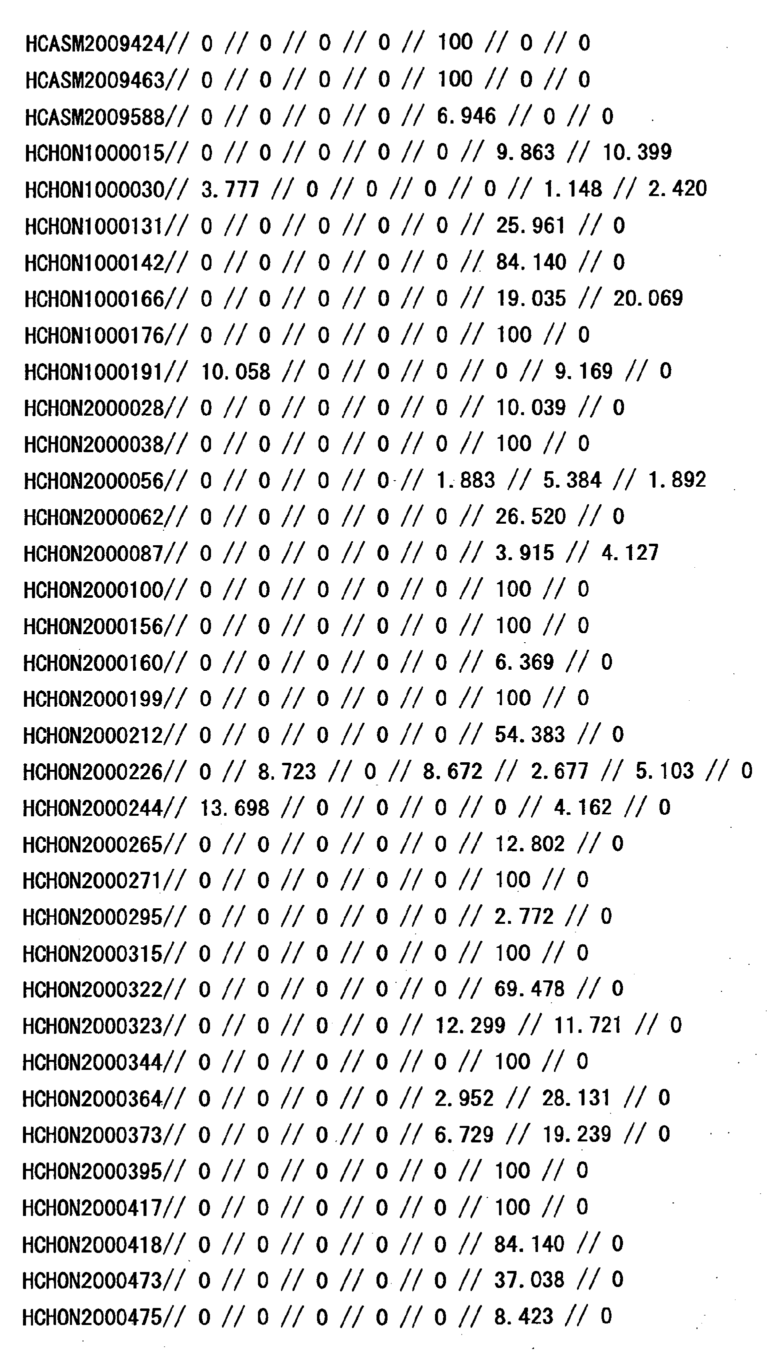

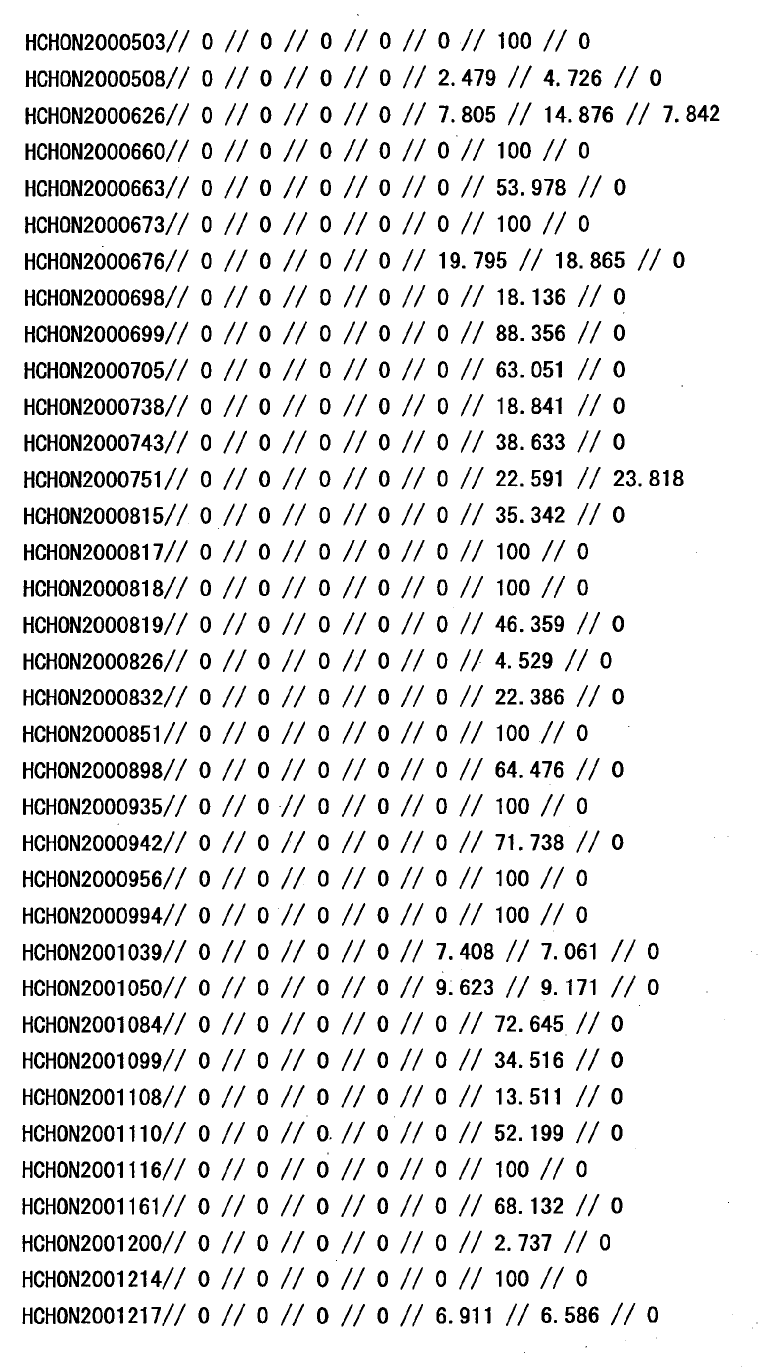

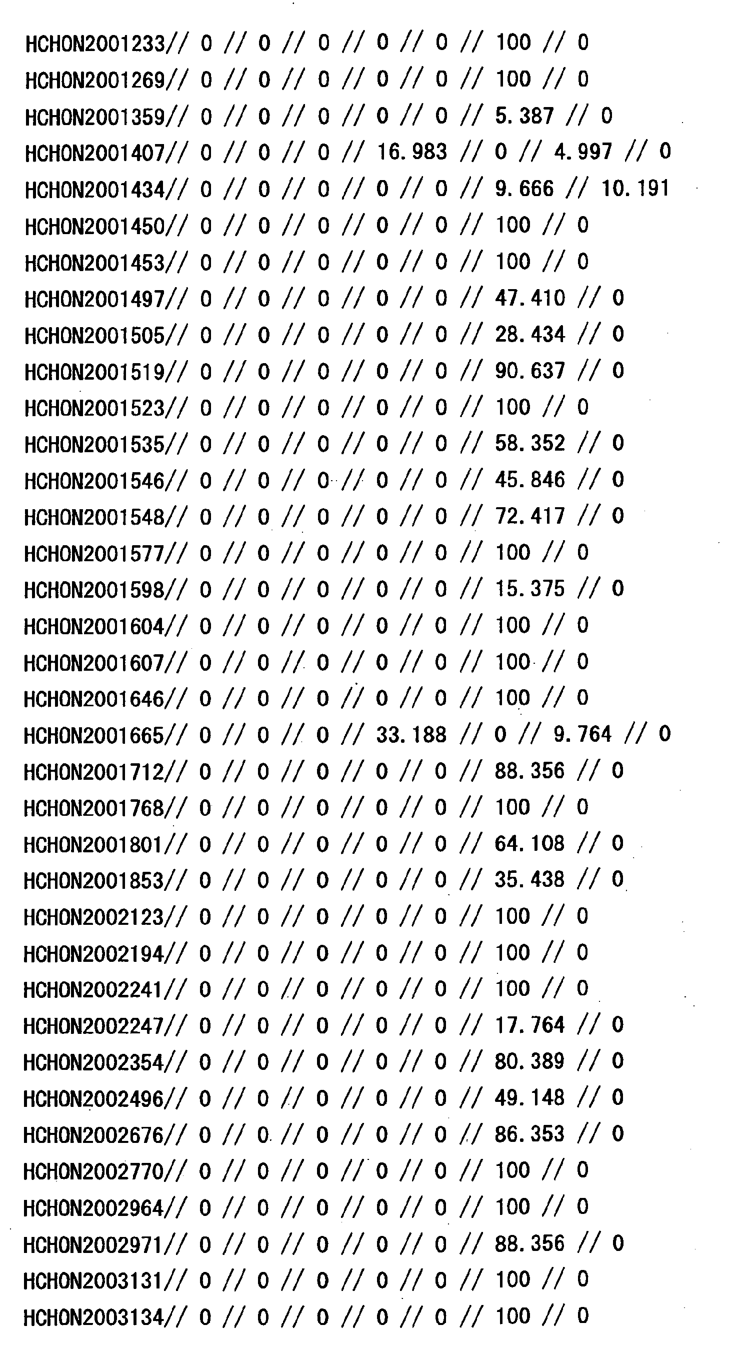

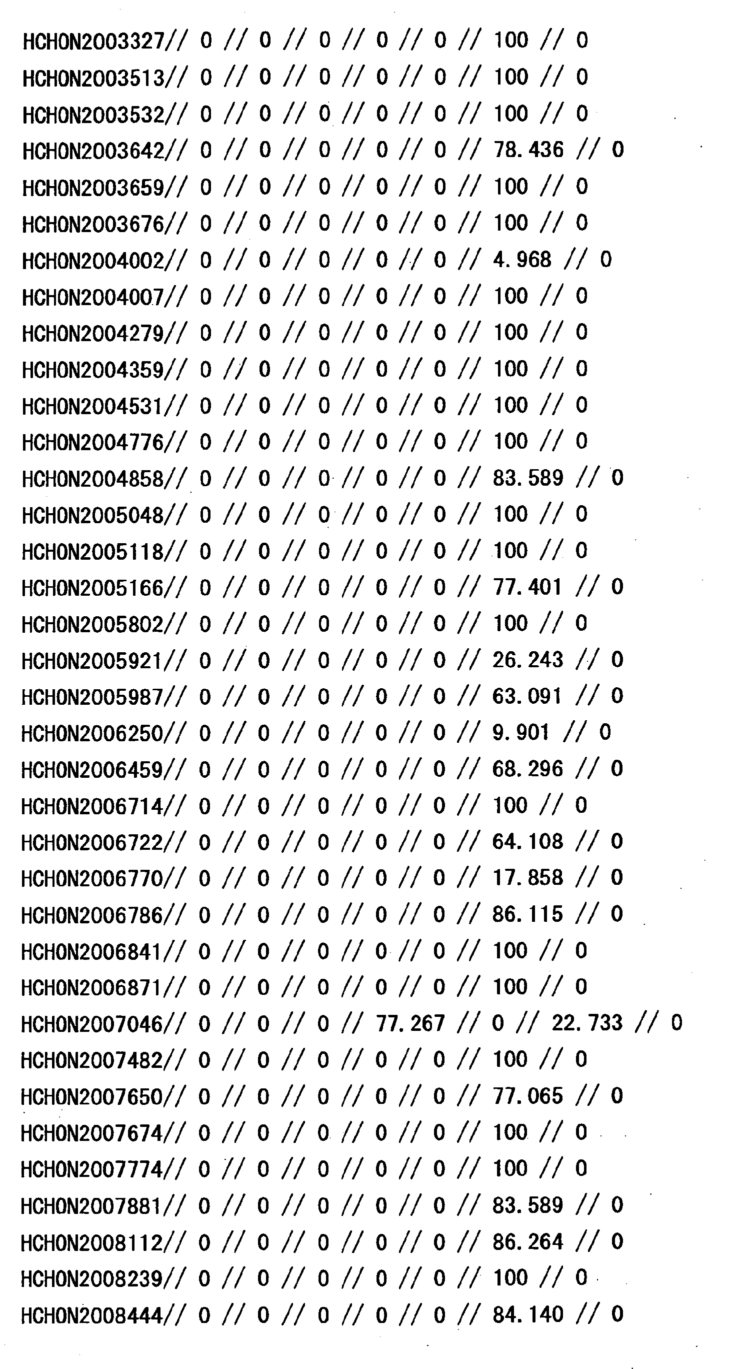

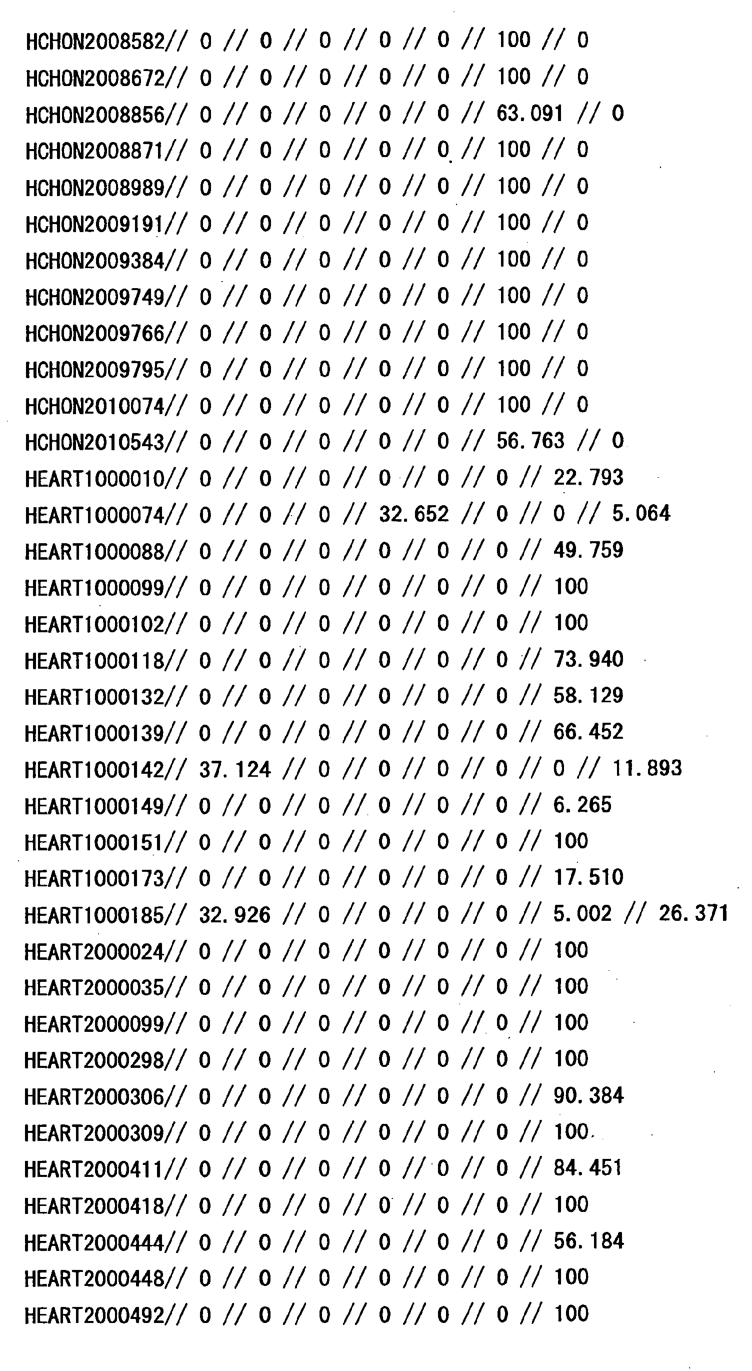

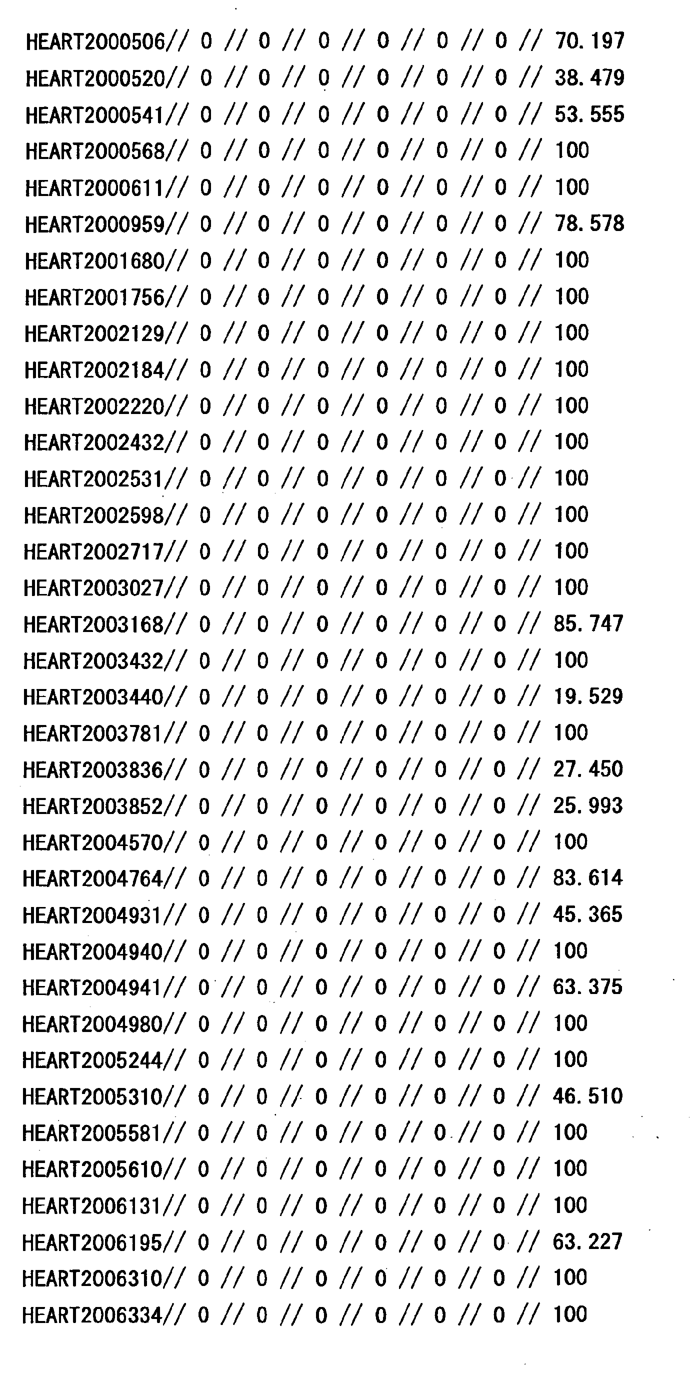

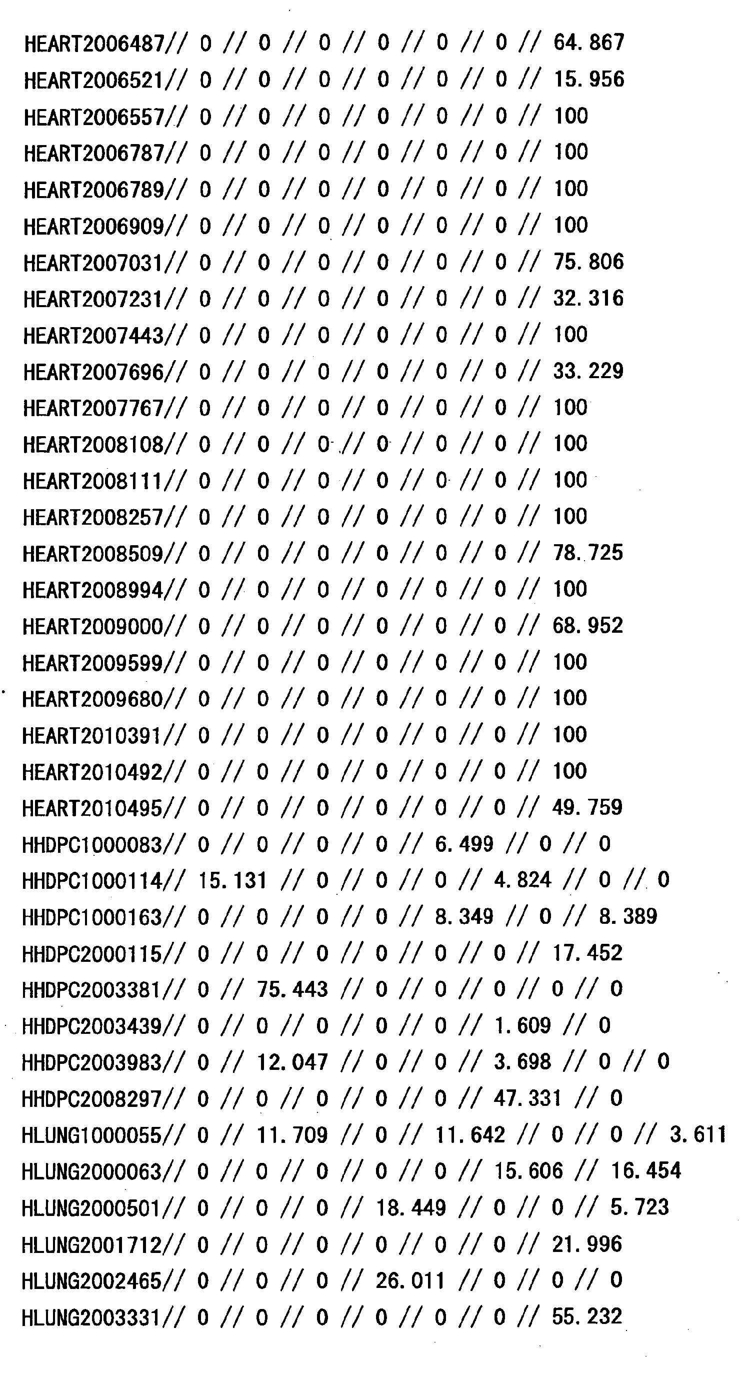

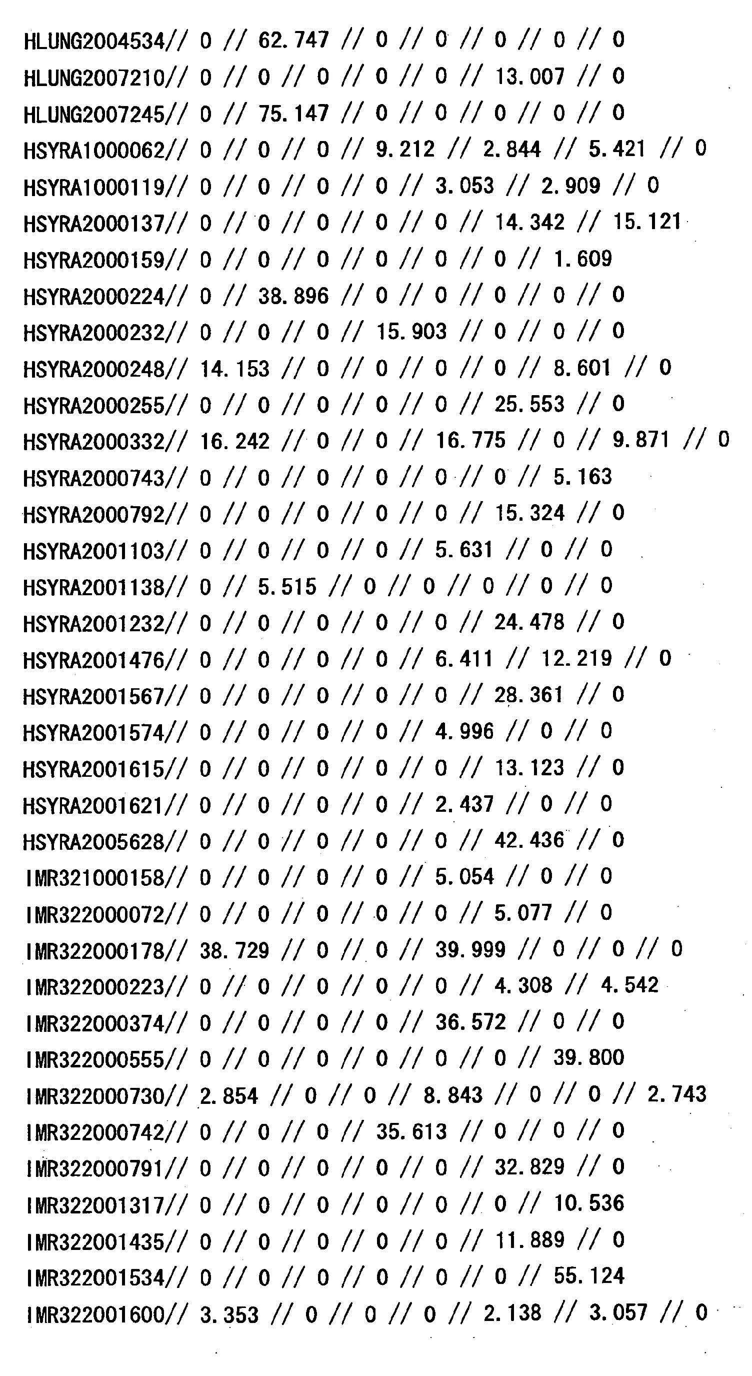

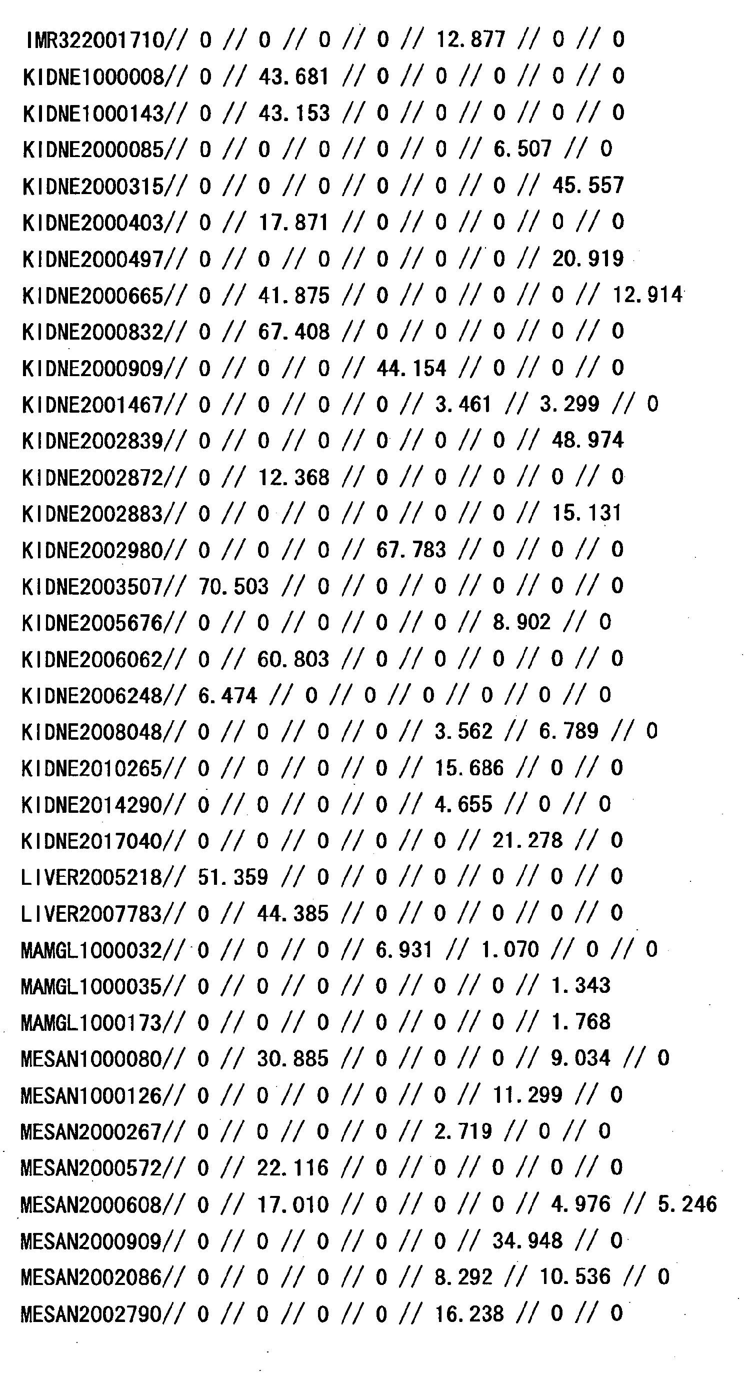

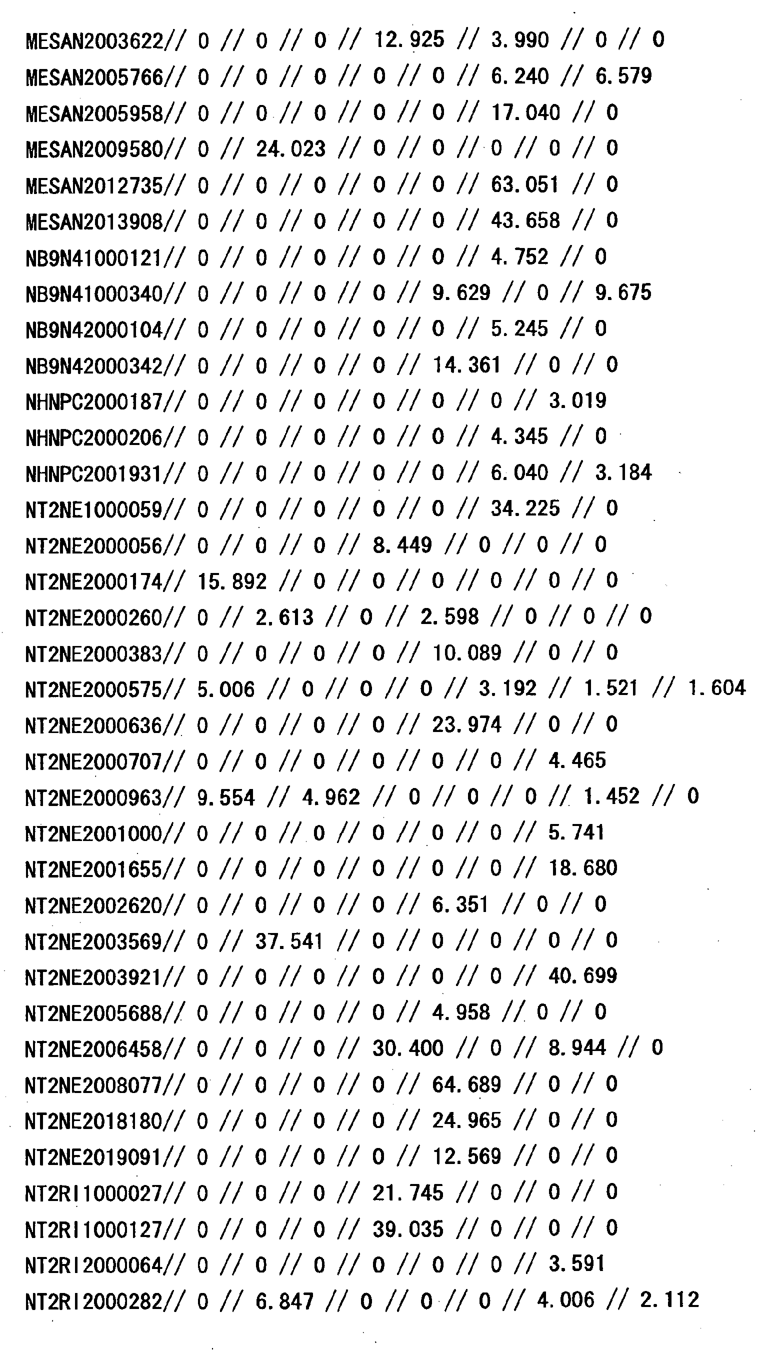

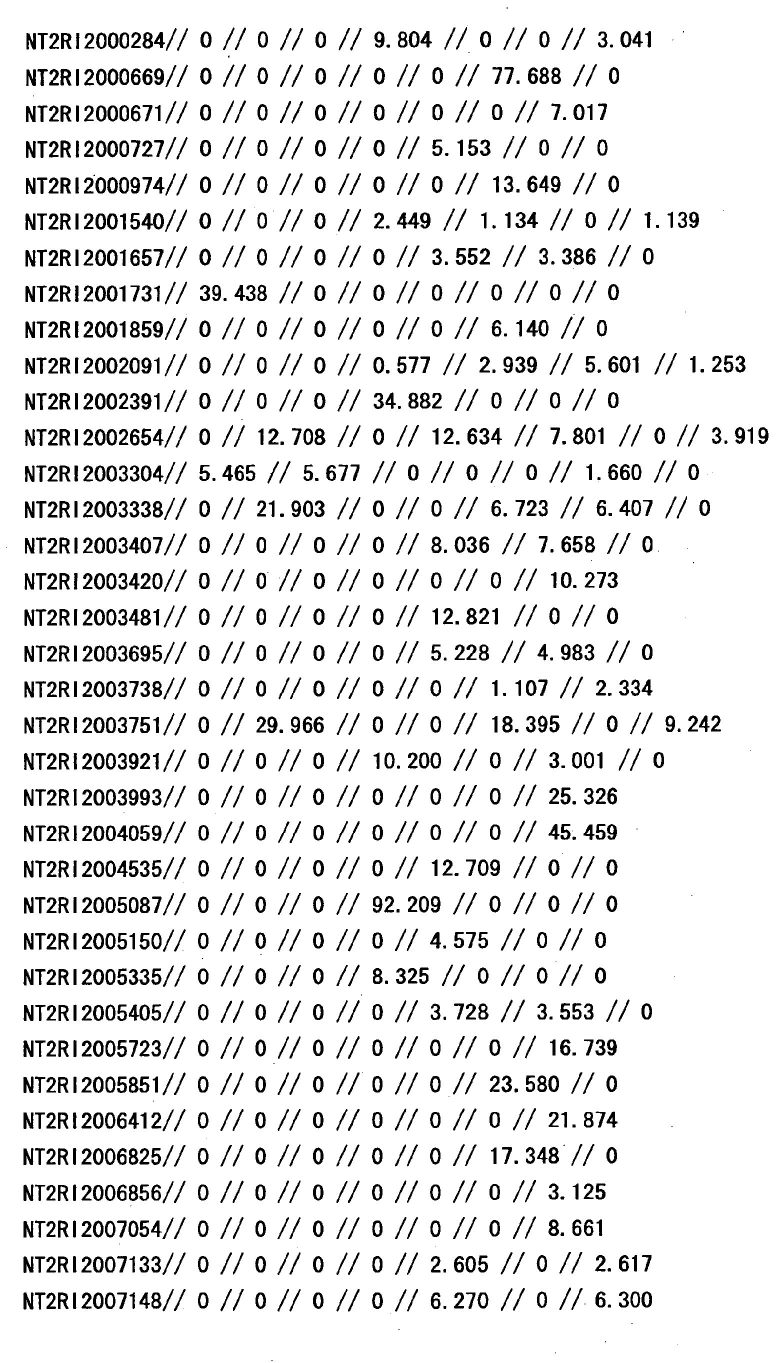

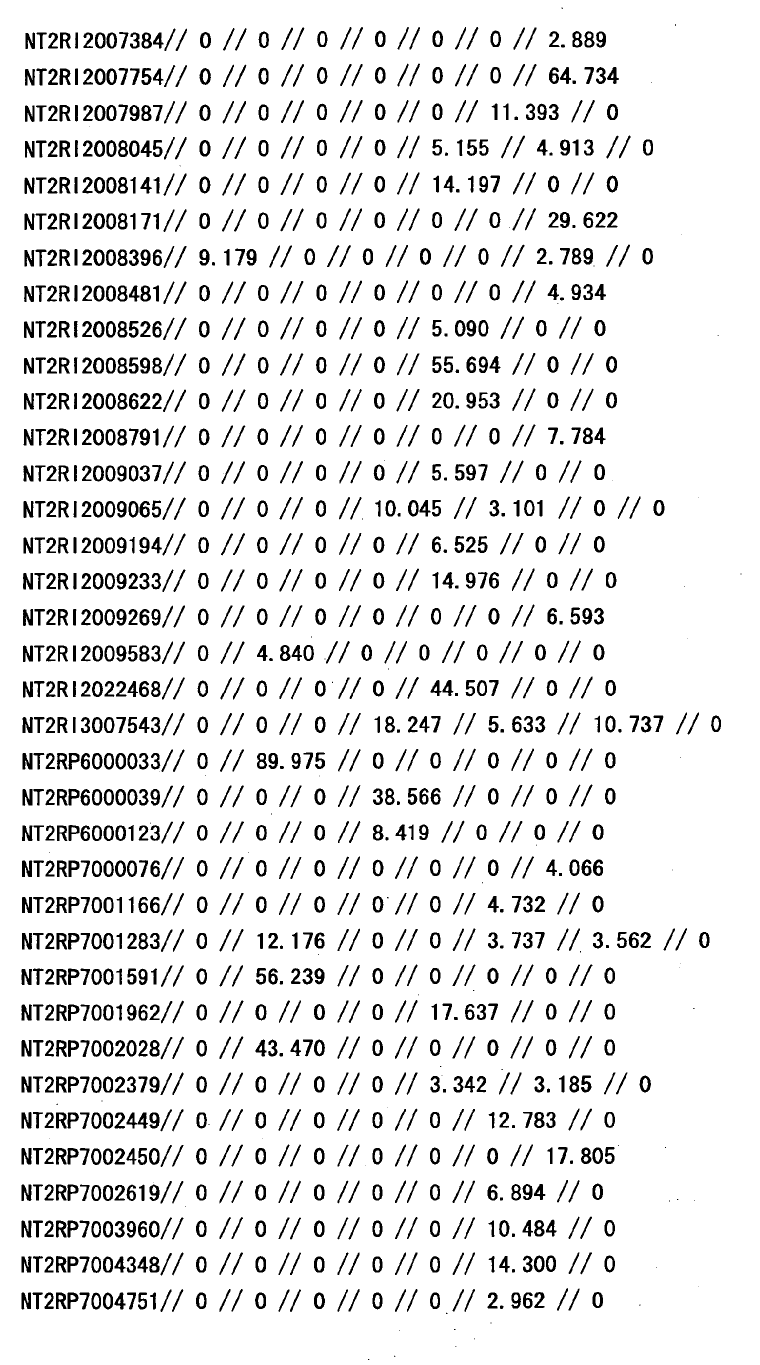

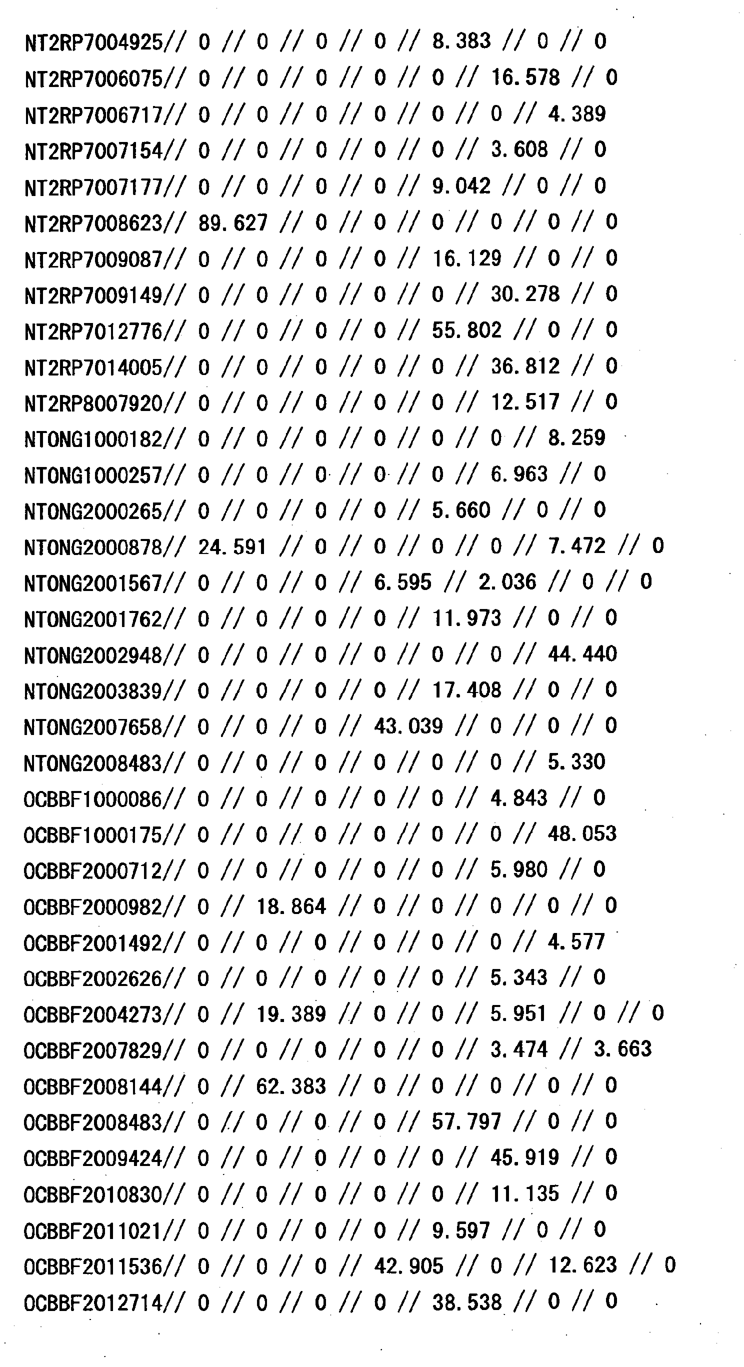

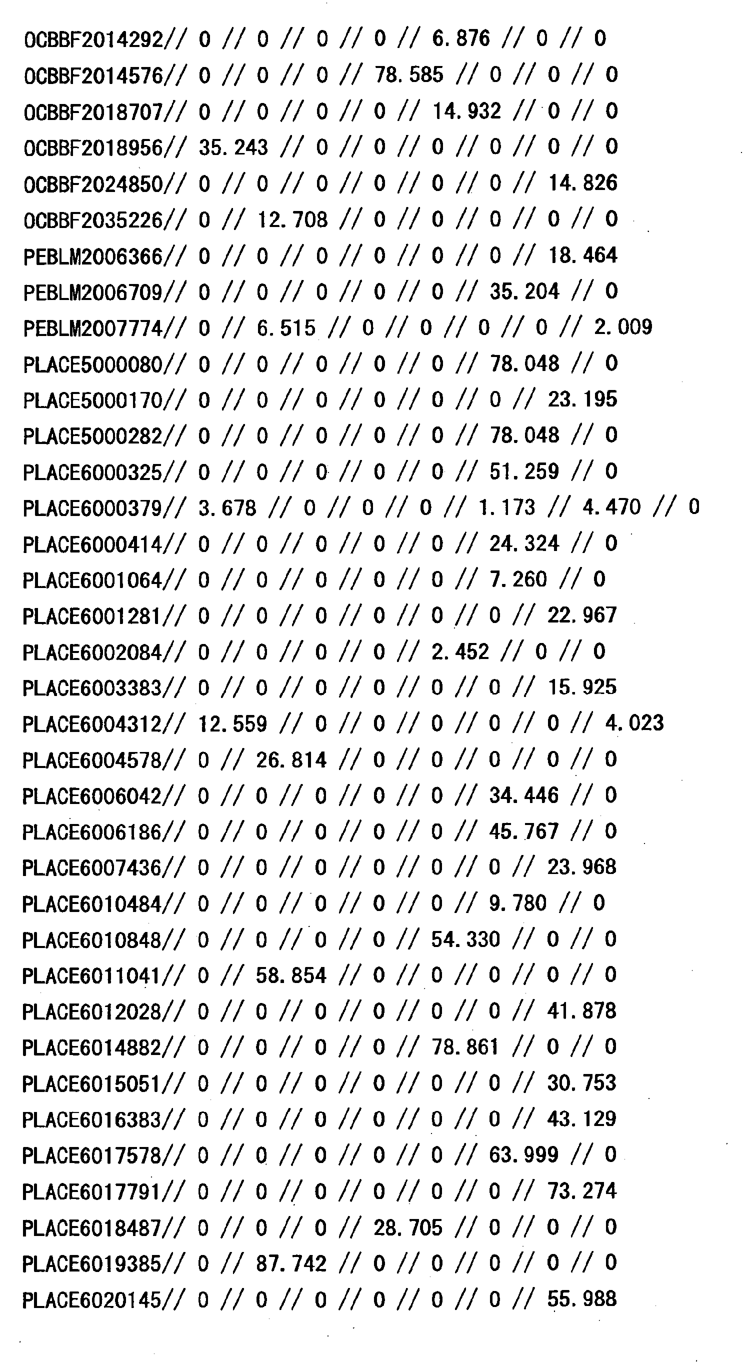

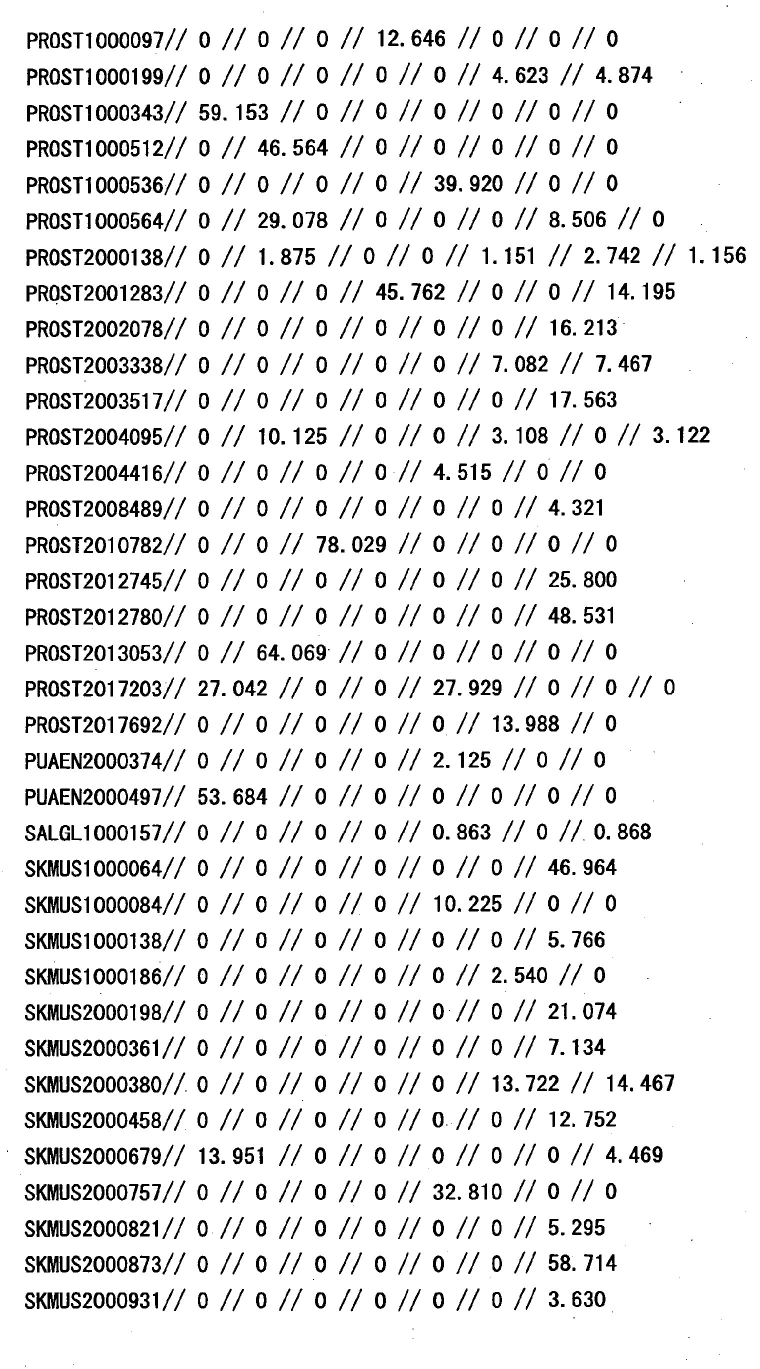

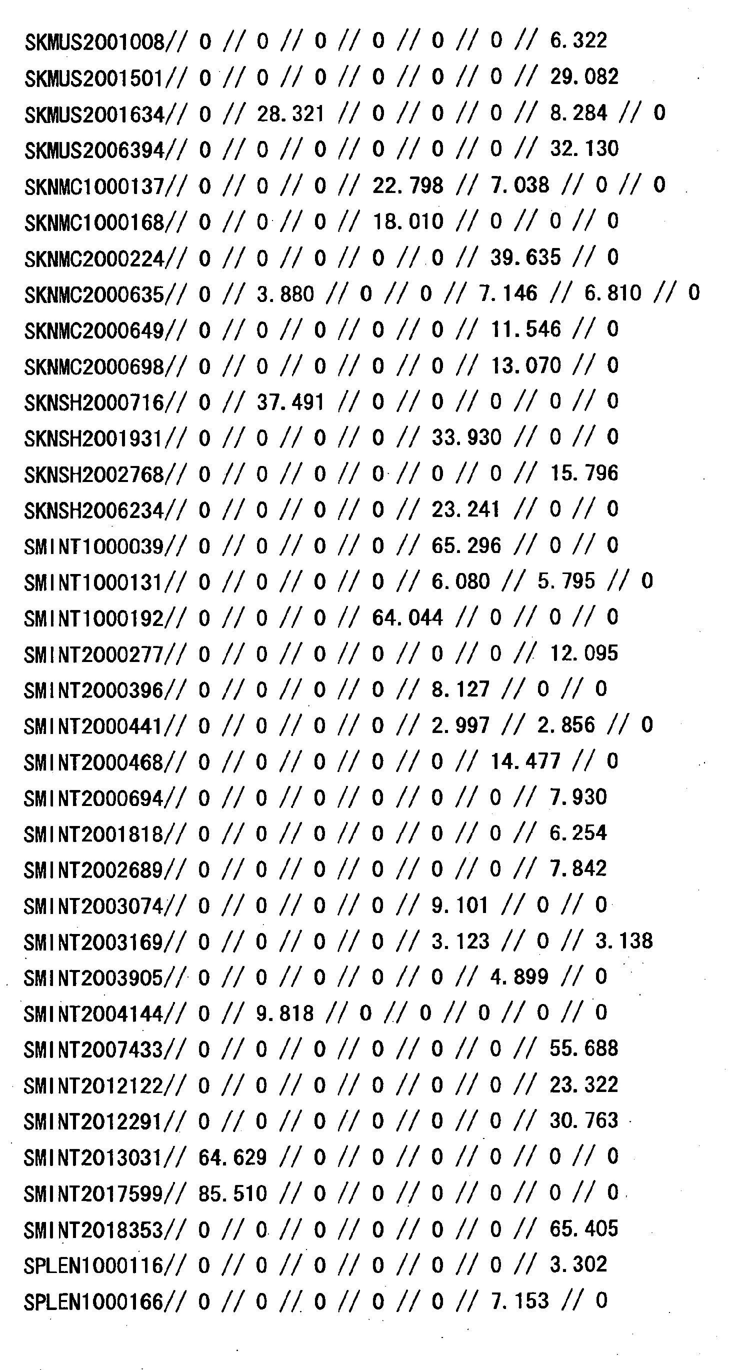

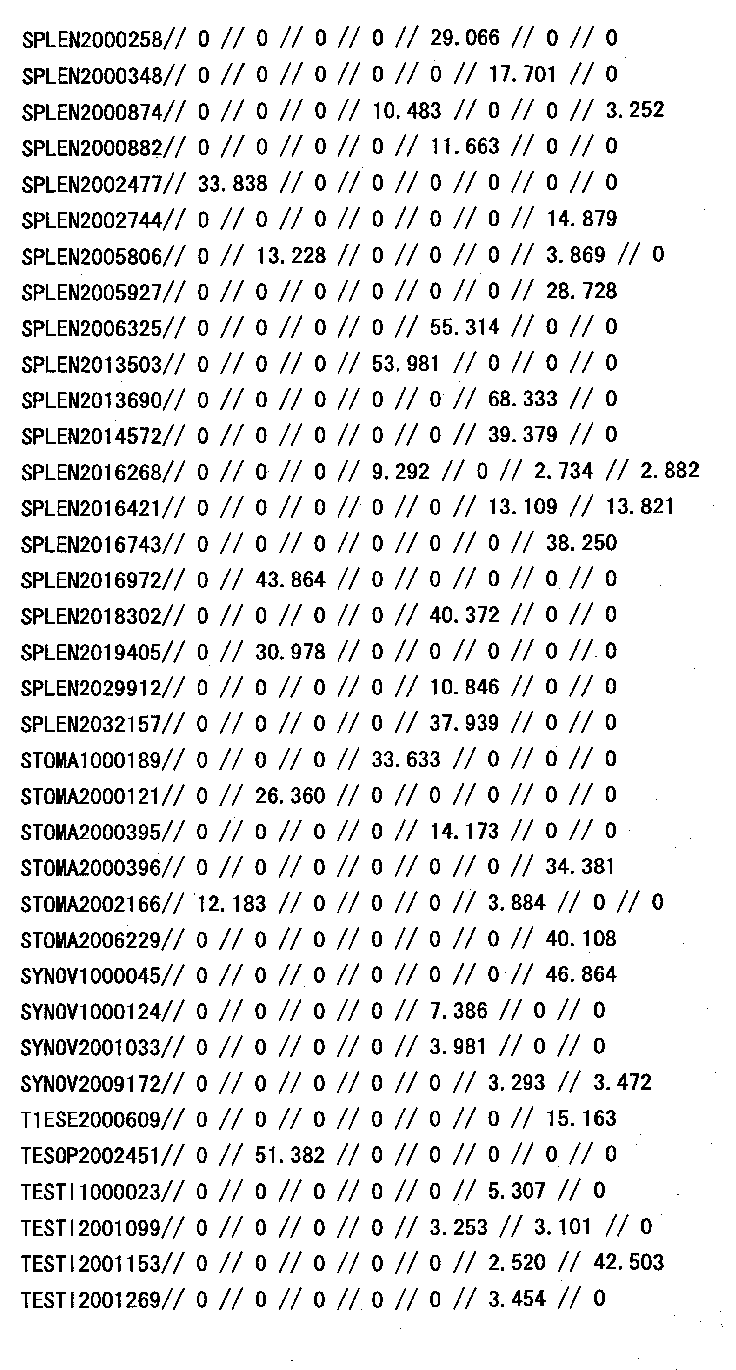

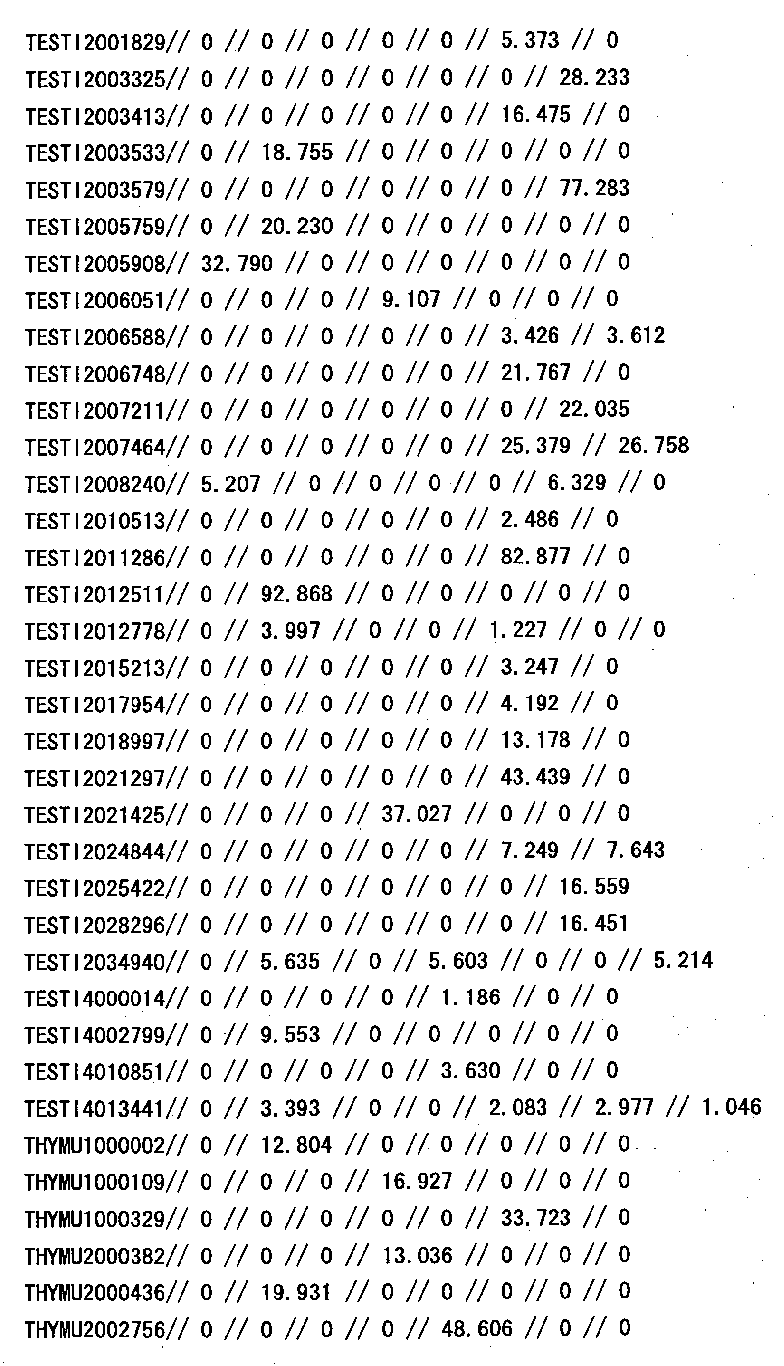

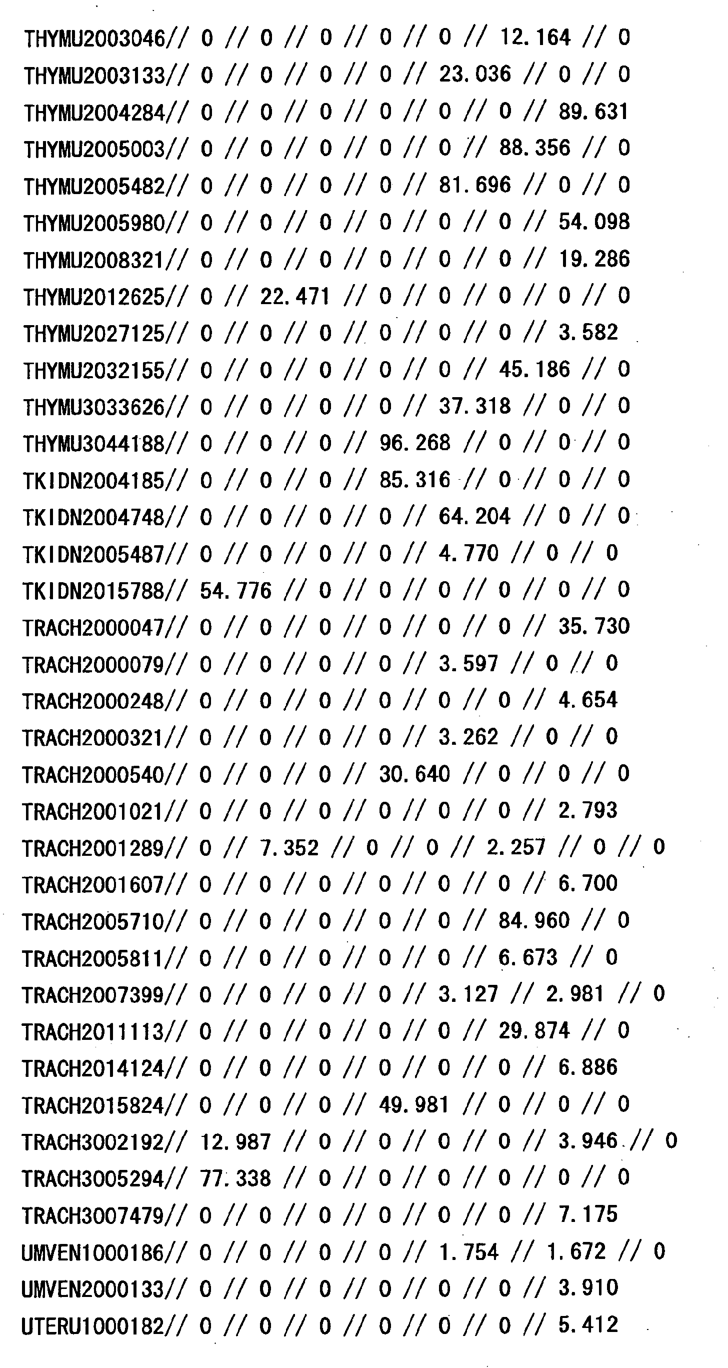

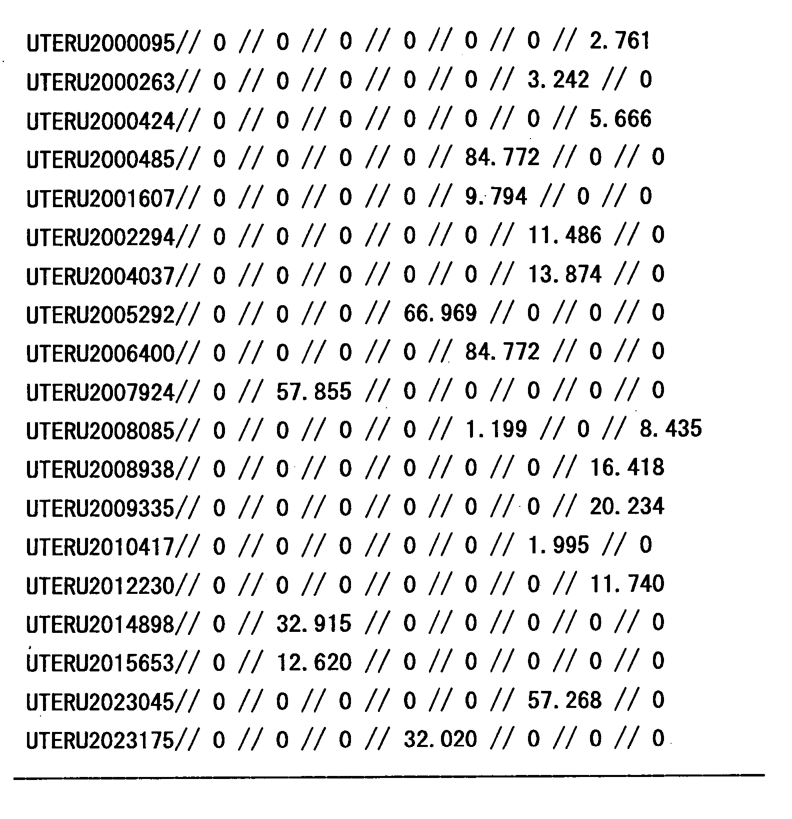

Genes whose expression levels were negligibly low in each of the above-described libraries are shown with their clone names below.
3NB691000018, 3NB691000085, 3NB691000113, 3NB691000116, 3NB691000191, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000545, 3NB692000973, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001538, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045,
3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830,
ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283,
ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000725, ASTRO2000801, ASTRO2000914,
ASTRO2001001, ASTRO2001008, ASTRO2001029, ASTRO2001088, ASTRO2001107, ASTRO2001122, ASTRO2001227, ASTRO2001249, ASTRO2001480, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423,
ASTRO2005553, ASTRO2005557, ASTRO2005575, ASTRO2005593, ASTRO2005687, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010819, ASTRO2010962, ASTRO2010996, ASTRO2011149,
ASTRO2011422, ASTRO2011426, ASTRO2011437, ASTRO2011461, ASTRO2011834, ASTRO2011893, ASTRO2012552, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014923, ASTRO2014961, ASTRO2015162, ASTRO2015185, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067,
BGGI12000161, BGGI12000533, BGGI12000544, BGGI12000616, BGGI12000684, BGGI12001002, BGGI12001075, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722,
BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169, BNGH41000190, BNGH41000198, BNGH41000216, BNGH41000235, BNGH42000130, BNGH42000132, BNGH42000405, BNGH42000532,
BNGH42000675, BNGH42000739, BNGH42000791, BNGH42000815, BNGH42000994, BNGH42001406, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006135, BNGH42006226, BNGH42006234, BNGH42007037, BNGH42007366, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000047, BRACE1000051, BRACE1000065, BRACE1000073, BRACE1000084, BRACE1000087, BRACE1000092, BRACE1000093, BRACE1000101,
BRACE1000114, BRACE1000124, BRACE1000132, BRACE1000159, BRACE1000166, BRACE1000169, BRACE1000187, BRACE1000239, BRACE1000258, BRACE1000451, BRACE1000475, BRACE1000572, BRACE2000061, BRACE2000077, BRACE2000100, BRACE2000109, BRACE2000141, BRACE2000183, BRACE2000229, BRACE2000232, BRACE2000245, BRACE2000256, BRACE2000266, BRACE2000267, BRACE2000280, BRACE2000300, BRACE2000331, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750,
BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE 2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626,
BRACE2001673, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001865, BRACE2001872, BRACE2001881, BRACE2001892, BRACE2001898, BRACE2001923, BRACE2001942, BRACE2001944, BRACE2001954, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002441, BRACE2002444, BRACE2002458, BRACE2002467, BRACE2002468, BRACE2002478,
BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003319, BRACE2003398,
BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193,
BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005949, BRACE2005981,
BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833,
BRACE2006859, BRACE2006871, BRACE2006909, BRACE2006911, BRACE2006924, BRACE2006935, BRACE2006937, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671,
BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008399, BRACE2008401, BRACE2008402, BRACE2008410, BRACE2008420, BRACE2008443,
BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275,
BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983,
BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013911, BRACE2014108, BRACE2014161, BRACE2014232, BRACE2014257, BRACE2014268, BRACE2014306, BRACE2014475, BRACE2014657, BRACE2014746, BRACE2014780,
BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438,. BRACE2017574, BRACE2017580, BRACE2017587, BRACE2017720, BRACE2017844, BRACE2017872, BRACE2017929, BRACE2017934, BRACE2017985, BRACE2017992,
BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344,
BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333,
BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345,
BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299,
BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381,
BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595,
BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989,
BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079,
BRACE3010086, BRACE3010221, BRACE3010283, BRACE3010315, BRACE3010397, BRACE3010416, BRACE3010428, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170,
BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531,
BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771,
BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078,
BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376,
BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025,
BRAMY2000052, BRAMY2000076, BRAMY2000086, BRAMY2000151, BRAMY2000181, BRAMY2000196, BRAMY2000231, BRAMY2000277, BRAMY2000388, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287,
BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466,
BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208,
BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200,
BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771, BRAMY2016892, BRAMY2016938, BRAMY2016953, BRAMY2017014,
BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142,
BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312,
BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451,
BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678,
BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100,
BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205,
BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095,
BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000045, BRAWH1000083, BRAWH1000093, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417,
BRAWH2000443, BRAWH2000460, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000633, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000926, BRAWH2000944, BRAWH2000984, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438,
BRAWH2001439, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002967, BRAWH2002976, BRAWH2003000, BRAWH2003025, BRAWH2003135, BRAWH2003240, BRAWH2003307, BRAWH2003355,
BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406,
BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536,
BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188,
BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534,
BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884,
BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806,
BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149,
BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401,
BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886,
BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340,
BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732,
BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013,
BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000819, BRHIP2000826, BRHIP2000920, BRHIP2001074, BRHIP2001099,
BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269,
BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958,
BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884,
BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911,
BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076,
BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683,
BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745,
BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844,
BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841,
BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198,
BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070,
BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284,
BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA200137.0, BRTHA2001462, BRTHA2001717, BRTHA2001812, BRTHA2002091, BRTHA2002115, BRTHA2002133,
BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665,
BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540,
BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014,
BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448,
BRTHA2035743, BRTHA2036055, BRTHA2036295, BRTHA2036660, BRTHA2037247, BRTHA2038279, BRTHA2038345, BRTHA2038353, BRTHA2038396, BRTHA3000252; BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113,
BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908,
BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152,
CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337,
COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062, CTONG1000086, CTONG1000088, CTONG1000093, CTONG1000094, CTONG1000097, CTONG1000113, CTONG1000137, CTONG1000165, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000465, CTONG2000469, CTONG2000480,
CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858, CTONG2001877, CTONG2001911, CTONG2001932, CTONG2001955, CTONG2002066, CTONG2002095, CTONG2002143, CTONG2002270, CTONG2002395, CTONG2002417, CTONG2002418, CTONG2002453, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002903,
CTONG2002965, CTONG2002999, CTONG2003028, CTONG2003094, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399,
CTONG2005468, CTONG2005553, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500,
CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008398, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604,
CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010652, CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158,
CTONG2012230, CTONG2012401, CTONG2012422, CTONG2012425, CTONG2012447, CTONG2012453, CTONG2012473, CTONG2012476, CTONG2012533, CTONG2012554, CTONG2012607, CTONG2012661, CTONG2012724, CTONG2012738, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630,
CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016185, CTONG2016217, CTONG2016355, CTONG2016408, CTONG2016499, CTONG2016505, CTONG2016559, CTONG2016658, CTONG2016775, CTONG2016797, CTONG2016846, CTONG2016869, CTONG2016904, CTONG2016942, CTONG2016953, CTONG2017021, CTONG2017094, CTONG2017292, CTONG2017409, CTONG2017429, CTONG2017444, CTONG2017458, CTONG2017604,
CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483, CTONG2018629, CTONG2018632, CTONG2018637, CTONG2018652, CTONG2018653, CTONG2018655, CTONG2018735, CTONG2018739, CTONG2018808, CTONG2018843, CTONG2018898, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378,
CTONG2020411, CTONG2020445, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000686, CTONG3000707, CTONG3000896,
CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, .
CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008894, CTONG3008951, CTONG3008959, CTONG3009227, CTONG3009239, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291,
D3OST3000388, D3OST3000493, D6OST2000127, D6OST2000169, D6OST2000245, D6OST2000358, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003580, D9OPST2003594, D9OST2003762, D9OST2003791, D9OST2003989, D9OST2004018, D9OST2004073, D9OST2004417, DFNES1000003, DFNES1000083, DFNES1000107, DFNES1000150, DFNES1000185, DFNES2000143, DFNES2000146, DFNES2000153, DFNES2000212, DFNES2000268, DFNES2000292, DFNES2000335, DFNES2000426, DFNES2000432, DFNES2000443, DFNES2000457, DFNES2000471, DFNES2000526, DFNES2000913, DFNES2001091, DFNES2001096, DFNES2001108, DFNES2001177,
DFNES2001404, DFNES2001564, DFNES2001565, DFNES2001647, DFNES2001800, DFNES2001829, DFNES2002220, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005690, DFNES2005779, DFNES2005875, DFNES2006346, DFNES2006944, DFNES2007113, DFNES2007299, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024,
FCBBF1000025, FCBBF1000038, FCBBF1000053, FCBBF1000061, FCBBF1000063, FCBBF1000069, FCBBF1000077, FCBBF1000115, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000197, FCBBF1000198, FCBBF1000206, FCBBF1000220, FCBBF1000232, FCBBF1000233, FCBBF1000243, FCBBF1000270, FCBBF1000272, FCBBF1000294, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000466, FCBBF1000476, FCBBF1000506, FCBBF1000509, FCBBF1000546, FCBBF1000550, FCBBF1000556,
FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000748, FCBBF1000760, FCBBF2000038, FCBBF2000087, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116,
FCBBF2001183, FCBBF2001188, FCBBF2001211, FCBBF2001238, FCBBF2001243, FCBBF2001299, FCBBF2001309, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373,
FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847,
FCBBF3001018, FCBBF3001020, FCBBF3001081, FCBBF3001235, FCBBF3001281, FCBBF3001302, FCBBF3001377, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004473, FCBBF3004502, FCBBF3004715, FCBBF3004842, FCBBF3004847, FCBBF3004955, FCBBF3005160,
FCBBF3005218, FCBBF3005444, FCBBF3005497, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479,
FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800,
FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019564,
FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3023061, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285,
FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041,
FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452,
FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000762, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175,
FEBRA2001197, FEBRA2001217, FEBRA2001227, FEBRA2001245, FEBRA2001267, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002508,
FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003833,
FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458,
FEBRA2005476, FEBRA2005701, FEBRA2005726, FEBRA2005752, FEBRA2005778, FEBRA2005787, FEBRA2005788, FEBRA2005824, FEBRA2005995, FEBRA2005998, FEBRA2006055, FEBRA2006061, FEBRA2006092, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017,
FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007708, FEBRA2007714, FEBRA2007793, FEBRA2007801, FEBRA2007818, FEBRA2007823, FEBRA2007900, FEBRA2007901, FEBRA2007931, FEBRA2007957, FEBRA2008009, FEBRA2008036, FEBRA2008081, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008302, FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063,
FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502,
FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601,
FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023498, FEBRA2023550, FEBRA2023721, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, HELAC2000158,
HELAC2000301, HHDPC1000001, HHDPC1000065, HHDPC1000118, HHDPC2000055, HHDPC2000070, HHDPC2000102, HHDPC2000104, HHDPC2000149, HHDPC2000258, HHDPC2000315, HHDPC2000455, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267, HHDPC2007302, HHDPC2007775, HHDPC2008123, HHDPC2008185, HHDPC2008279, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030,
HLUNG1000034, HLUNG1000037, HLUNG1000053, HLUNG1000064, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013,
HLUNG2001126, HLUNG2001144, HLUNG2001146, HLUNG2001214, HLUNG2001439, HLUNG2001459, HLUNG2001507, HLUNG2001518, HLUNG2001633, HLUNG2001677, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918,
HLUNG2003991, HLUNG2004014, HLUNG2004037, HLUNG2004067, HLUNG2004154, HLUNG2004159, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007219, HLUNG2007433, HLUNG2007931, HLUNG2008066,
HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288,
HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000152, HSYRA1000178, HSYRA2000044, HSYRA2000135, HSYRA2000190, HSYRA2000221, HSYRA2000237, HSYRA2000253, HSYRA2000371, HSYRA2000424, HSYRA2000507, HSYRA2000510, HSYRA2000605, HSYRA2000629, HSYRA2000640, HSYRA2000706, HSYRA2000760, HSYRA2000787, HSYRA2000828, HSYRA2000832, HSYRA2000925, HSYRA2000927, HSYRA2001003, HSYRA2001105, HSYRA2001142, HSYRA2001153, HSYRA2001225, HSYRA2001255,
HSYRA2001332, HSYRA2001353, HSYRA2001396, HSYRA2001420, HSYRA2001484, HSYRA2001552, HSYRA2001580, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000161, IMR321000165, IMR321000193, IMR321000207, IMR321000210, IMR321000219, IMR321000230, IMR321000242, IMR321000253, IMR321000266, IMR322000010, IMR322000107, IMR322000121, IMR322000127, IMR322000144, IMR322000152, IMR322000164, IMR322000243, IMR322000302, IMR322000316,
IMR322000379, IMR322000383, IMR322000418, IMR322000518, IMR322000604, IMR322000609, IMR322000656, IMR322000672, IMR322000775, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001218, IMR322001245, IMR322001318, IMR322001322, IMR322001332, IMR322001380, IMR322001438, IMR322001452, IMR322001491, IMR322001541, IMR322001584, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222,
IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000012, KIDNE1000015, KIDNE1000028, KIDNE1000036, KIDNE1000064, KIDNE1000104, KIDNE1000121, KIDNE1000145, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000317,
KIDNE2000328, KIDNE2000330, KIDNE2000335, KIDNE2000349, KIDNE2000375, KIDNE2000383, KIDNE2000394, KIDNE2000422, KIDNE2000493, KIDNE2000510, KIDNE2000513, KIDNE2000517, KIDNE2000519, KIDNE2000555, KIDNE2000574, KIDNE2000624, KIDNE2000678, KIDNE2000717, KIDNE2000721, KIDNE2000722, KIDNE2000777, KIDNE2000801, KIDNE2000833, KIDNE2000846, KIDNE2000867, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265,
KIDNE2002438, KIDNE2002483, KIDNE2002725, KIDNE2002768, KIDNE2002795, KIDNE2002798, KIDNE2002845, KIDNE2002882, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879,
KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077,
KIDNE2007186, KIDNE2007222, KIDNE2007328, KIDNE2007352, KIDNE2007356, KIDNE2007422, KIDNE2007569, KIDNE2007810, KIDNE2007811, KIDNE2007910, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628,
KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263,
KIDNE2013346, KIDNE2013388, KIDNE2013413, KIDNE2013489, KIDNE2013639, KIDNE2013731, KIDNE2013734, KIDNE2013775, KIDNE2013801, KIDNE2013845, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327,
KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126,
LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2002842, LIVER2003008,
LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017,
MAMGL1000083, MAMGL1000096, MAMGL1000100, MAMGL1000132, MAMGL1000151, MAMGL1000178, MAMGL1000182, MAMGL1000184, MESAN1000032, MESAN1000035, MESAN1000050, MESAN1000079, MESAN1000101, MESAN1000121, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000501, MESAN2000620, MESAN2000711, MESAN2000815, MESAN2000894, MESAN2001154, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002844, MESAN2002940,
MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005808, MESAN2005811, MESAN2005957, MESAN2006022, MESAN2006043, MESAN2006328, MESAN2006401, MESAN2006563, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926,
MESAN2008936, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2013019, MESAN2013022, MESAN2013183, MESAN2013211, MESAN2013283, MESAN2013284, MESAN2013611, MESAN2013674, MESAN2013845, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552,
MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000135, NB9N41000142, NB9N41000146, NB9N42000122, NB9N42000196, NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182,
NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000063, NT2NE1000073, NT2NE1000083, NT2NE1000120, NT2NE1000122, NT2NE1000163, NT2NE1000175, NT2NE1000181, NT2NE1000185, NT2NE2000038, NT2NE2000054, NT2NE2000057, NT2NE2000064, NT2NE2000109,
NT2NE2000137, NT2NE2000156, NT2NE2000195, NT2NE2000222, NT2NE2000259, NT2NE2000284, NT2NE2000299, NT2NE2000327, NT2NE2000353, NT2NE2000356, NT2NE2000369, NT2NE2000374, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000658, NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191,
NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309,
NT2NE2003315, NT2NE2003393, NT2NE2003408, NT2NE2003457, NT2NE2003485, NT2NE2003540, NT2NE2003571, NT2NE2003705, NT2NE2003752, NT2NE2003887, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427,
NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033,
NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275,
NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000016, NT2RI1000035, NT2RI1000048, NT2RI1000164, NT2RI1000172, NT2RI2000004, NT2RI2000007,
NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000578, NT2RI2000588, NT2RI2000597, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001342, NT2RI2001385,
NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002316, NT2RI2002334, NT2RI2002359, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819,
NT2RI2002822, NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003487, NT2RI2003556, NT2RI2003655, NT2RI2003667, NT2RI2003678, NT2RI2003884, NT2RI2003973, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606,
NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005096, NT2RI2005115, NT2RI2005116, NT2RI2005130, NT2RI2005166, NT2RI2005239, NT2RI2005246, NT2RI2005315, NT2RI2005353, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005713, NT2RI2005772, NT2RI2005811, NT2RI2005814, NT2RI2005816, NT2RI2005818, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271,
NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006943, NT2RI2006973, NT2RI2007046, NT2RI2007048, NT2RI2007084, NT2RI2007096, NT2RI2007116, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007498, NT2RI2007507, NT2RI2007572, NT2RI2007589, NT2RI2007593, NT2RI2007629, NT2RI2007723, NT2RI2007729,
NT2RI2007751, NT2RI2007827, NT2RI2007853, NT2RI2007877, NT2RI2007879, NT2RI2007884, NT2RI2007891, NT2RI2007956, NT2RI2007965, NT2RI2008007, NT2RI2008050, NT2RI2008188, NT2RI2008204, NT2RI2008221, NT2RI2008233, NT2RI2008336, NT2RI2008455, NT2RI2008502, NT2RI2008566, NT2RI2008656, NT2RI2008714, NT2RI2008724, NT2RI2008749, NT2RI2008801, NT2RI2008808, NT2RI2008812, NT2RI2008841, NT2RI2008942, NT2RI2008952, NT2RI2009005, NT2RI2009066, NT2RI2009083, NT2RI2009101, NT2RI2009103, NT2RI2009144, NT2RI2009151, NT2RI2009173, NT2RI2009215, NT2RI2009239, NT2RI2009259, NT2RI2009281, NT2RI2009289, NT2RI2009301, NT2RI2009402, NT2RI2009406, NT2RI2009517, NT2RI2009585, NT2RI2009595, NT2RI2009855, NT2RI2010283,
NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758,
NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284,
NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000043, NT2RP6000059, NT2RP6000060, NT2RP6000072, NT2RP6000077, NT2RP6000078, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086,
NT2RP7000109, NT2RP7000112, NT2RP7000173, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624, NT2RP7000812, NT2RP7000906, NT2RP7000926, NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001602, NT2RP7001678, NT2RP7001856, NT2RP7002064, NT2RP7002151, NT2RP7002163, NT2RP7002243, NT2RP7002282, NT2RP7002376, NT2RP7002478, NT2RP7002554, NT2RP7002594, NT2RP7002603, NT2RP7002650, NT2RP7002738, NT2RP7002779, NT2RP7002802, NT2RP7002829, NT2RP7002841, NT2RP7002875,
NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7004027, NT2RP7004080, NT2RP7004114, NT2RP7004123, NT2RP7004173, NT2RP7004196, NT2RP7004204, NT2RP7004233, NT2RP7004260, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004728, NT2RP7004766, NT2RP7004790, NT2RP7004884, NT2RP7004911, NT2RP7004915, NT2RP7004946,
NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006141, NT2RP7006160, NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007221, NT2RP7007226, NT2RP7007252, NT2RP7007269, NT2RP7007310,
NT2RP7007359, NT2RP7007381, NT2RP7007387, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008190, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008657, NT2RP7008714, NT2RP7008720, NT2RP7008855, NT2RP7008863, NT2RP7009012, NT2RP7009019, NT2RP7009030, NT2RP7009097, NT2RP7009108, NT2RP7009147,
NT2RP7009168, NT2RP7009215, NT2RP7009225, NT2RP7009236, NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013573, NT2RP7013729, NT2RP7013764, NT2RP7013795, NT2RP7013999,
NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137,
NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000033, NTONG1000047, NTONG1000052, NTONG1000075, NTONG1000087, NTONG1000098, NTONG1000123, NTONG1000130, NTONG1000161, NTONG1000172, NTONG1000213, NTONG1000214, NTONG1000246, NTONG1000247, NTONG1000264, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858,
NTONG2000876, NTONG2000966, NTONG2000977, NTONG2000985, NTONG2001014, NTONG2001079, NTONG2001137, NTONG2001157, NTONG2001173, NTONG2001220, NTONG2001222,. NTONG200136.2, NTONG2001428, NTONG2001532, NTONG2001550, NTONG2001587, NTONG2001612, NTONG2001619, NTONG2001642, NTONG2001657, NTONG2001676, NTONG2002278, NTONG2002582, NTONG2002807, NTONG2002970, NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004918, NTONG2004991, NTONG2005062, NTONG2005086, NTONG2005137, NTONG2005153,
NTONG2005265, NTONG2005277, NTONG2005363, NTONG2005373, NTONG2005391, NTONG2005480, NTONG2005497, NTONG2005520, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030,
OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000185, OCBBF1000188, OCBBF1000192, OCBBF1000254, OCBBF1000315, OCBBF2000013, OCBBF2000015, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000719, OCBBF2000824, OCBBF2000831, OCBBF2000846, OCBBF2000904, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196,
OCBBF2001210, OCBBF2001252, OCBBF2001307, OCBBF2001323, OCBBF2001389, OCBBF2001402, OCBBF2001408, OCBBF2001416, OCBBF2001473, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038,
OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624,
OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008005, OCBBF2008041, OCBBF2008116, OCBBF2008138, OCBBF2008283, OCBBF2008330, OCBBF2008340, OCBBF2008366, OCBBF2008466, OCBBF2008511, OCBBF2008520, OCBBF2008569, OCBBF2008586, OCBBF2008640, OCBBF2008691, OCBBF2008701, OCBBF2008724,
OCBBF2008760, OCBBF2008768, OCBBF2008770, OCBBF2008775, OCBBF2008790, OCBBF2008814, OCBBF2008822, OCBBF2009095, OCBBF2009115, OCBBF2009242, OCBBF2009301, OCBBF2009352, OCBBF2009391, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283,
OCBBF2011311, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789,
OCBBF2013843, OCBBF2013883, OCBBF2013926, OCBBF2014052, OCBBF2014064, OCBBF2014089, OCBBF2014322, OCBBF2014386, OCBBF2014541, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458,
OCBBF2017489, OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833,
OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927,
OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380,
OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030,
PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000308, PEBLM2000321, PEBLM2000326, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283,
PEBLM2006298, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131,
PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000105, PLACE5000113, PLACE5000115, PLACE5000116, PLACE5000129, PLACE5000139, PLACE5000153, PLACE5000159, PLACE5000171, PLACE5000172, PLACE5000178, PLACE5000245, PLACE5000260, PLACE5000297, PLACE5000372, PLACE5000492,
PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002102, PLACE6002157, PLACE6002312, PLACE6002323,
PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004397, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108,
PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036, PLACE6008094, PLACE6008126, PLACE6008285,
PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010925, PLACE6010936, PLACE6010991, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012108, PLACE6012168, PLACE6012297,
PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016942, PLACE6016984, PLACE6017002, PLACE6017431, PLACE6017564, PLACE6017626, PLACE6017701, PLACE6017714, PLACE6017788, PLACE6018031, PLACE6018107, PLACE6018187, PLACE6018235, PLACE6018287, PLACE6018441, PLACE6018497, PLACE6018769,
PLACE6018834, PLACE6018843, PLACE6018863, PLACE6018938, PLACE6019195, PLACE6019542, PLACE6019600, PLACE6019674, PLACE6019676, PLACE6019701, PLACE6019932, PLACE6020031, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563,
PROST1000033, PROST1000039, PROST1000065, PROST1000083, PROST1000085, PROST1000110, PROST1000120, PROST1000123, PROST1000136, PROST1000144, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000526, PROST1000527, PROST1000528, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212,
PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002101, PROST2002162, PROST2002212, PROST2002227, PROST2002338, PROST2002425, PROST2002488,
PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004258, PROST2004270, PROST2004332, PROST2004481, PROST2004526,
PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200,
PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468, PROST2008472, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046,
PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012888, PROST2012890, PROST2013032, PROST2013121, PROST2013260, PROST2013327, PROST2013531, PROST2013605, PROST2013627, PROST2013873, PROST2013880, PROST2014422, PROST2014585, PROST2014601, PROST2014659,
PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017700,
PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087, PUAEN1000121, PUAEN1000147, PUAEN1000161, PUAEN1000164, PUAEN1000175, PUAEN1000239, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000535, PUAEN2000594, PUAEN2000684, PUAEN2000942,
PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655,
PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000067, SKMUS1000102, SKMUS1000104, SKMUS1000110, SKMUS1000118, SKMUS1000124, SKMUS1000129, SKMUS1000176, SKMUS1000177, SKMUS2000020, SKMUS2000061, SKMUS2000074, SKMUS2000078, SKMUS2000117, SKMUS2000137, SKMUS2000243, SKMUS2000271, SKMUS2000287, SKMUS2000317, SKMUS2000330, SKMUS2000339,
SKMUS2000343, SKMUS2000365, SKMUS2000390, SKMUS2000416, SKMUS2000467, SKMUS2000468, SKMUS2000484, SKMUS2000515, SKMUS2000690, SKMUS2000701, SKMUS2000705, SKMUS2000710, SKMUS2000724, SKMUS2000726, SKMUS2000774, SKMUS2000780, SKMUS2000847, SKMUS2000863, SKMUS2000894, SKMUS2000898, SKMUS2000902, SKMUS2000933, SKMUS2000945, SKMUS2000954, SKMUS2000961, SKMUS2000973, SKMUS2000980, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001454, SKMUS2001492, SKMUS2001528, SKMUS2001543, SKMUS2001580, SKMUS2001585,
SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607,
SKMUS2009190, SKMUS2009232, SKMUS2009479, SKMUS2009557, SKNMC1000110, SKNMC1000123, SKNMC1000124, SKNMC1000159, SKNMC1000229, SKNMC1000251, SKNMC1000264, SKNMC2000065, SKNMC2000097, SKNMC2000256, SKNMC2000305, SKNMC2000322, SKNMC2000356, SKNMC2000374, SKNMC2000583, SKNMC2000593, SKNMC2000612, SKNMC2000622, SKNMC2000877, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174,
SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000819, SKNSH2000971, SKNSH2001222, SKNSH2001875, SKNSH2002054, SKNSH2002325, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015,
SMINT1000012, SMINT1000016, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT2000007, SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000747, SMINT2000811,
SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002743, SMINT2002778, SMINT2002880, SMINT2002882, SMINT2002884, SMINT2002899, SMINT2002974, SMINT2002976, SMINT2003003, SMINT2003128, SMINT2003150, SMINT2003317, SMINT2003340, SMINT2003386, SMINT2003423, SMINT2003505, SMINT2003551, SMINT2003569,
SMINT2003641, SMINT2003644, SMINT2003866, SMINT2004023, SMINT2004037, SMINT2004047, SMINT2004086, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140,
SMINT2007187, SMINT2007219, SMINT2007294, SMINT2007365, SMINT2007391, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2008960, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529,
SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012501, SMINT2012734, SMINT2012793, SMINT2013032, SMINT2013129, SMINT2013170, SMINT2013181, SMINT2013228, SMINT2013613,
SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017659, SMINT2017736, SMINT2017781, SMINT2017855, SMINT2017884, SMINT2017964, SMINT2017974, SMINT2018343, SMINT2018592, SMINT2018681, SMINT2019017, SMINT2019105, SMINT2019142,
SMINT2019153, SMINT2019200, SPLEN1000013, SPLEN1000041, SPLEN1000049, SPLEN1000056, SPLEN1000087, SPLEN1000089, SPLEN1000091, SPLEN1000106, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000267, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450,
SPLEN2000464, SPLEN2000496, SPLEN2000505, SPLEN2000515, SPLEN2000516, SPLEN2000537, SPLEN2000541, SPLEN2000607, SPLEN2000630, SPLEN2000695, SPLEN2000698, SPLEN2000839, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451,
SPLEN2002462, SPLEN2002463, SPLEN2002467, SPLEN2002493, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869,
SPLEN2005939, SPLEN2006122, SPLEN2006133, SPLEN2006143, SPLEN2006232, SPLEN2006268, SPLEN2006283, SPLEN2006305, SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555,
SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981,
SPLEN2012115, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013644, SPLEN2013648, SPLEN2013670, SPLEN2013673, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543,
SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533,
SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018364, SPLEN2018395, SPLEN2018643, SPLEN2018683, SPLEN2018732, SPLEN2018749, SPLEN2018933,
SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383,
SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157,
SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028466, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033098,
SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845,
SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000289, STOMA2000383, STOMA2000386, STOMA2000478, STOMA2000482, STOMA2000529, STOMA2000539, STOMA2000567, STOMA2000640, STOMA2000678,
STOMA2000686, STOMA2000888, STOMA2001025, STOMA2001389, STOMA2002052, STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253,
STOMA2009256, STOMA2009289, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001239, SYNOV2001251, SYNOV2001262, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673,
SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570,
SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543,
TBAES2006568, TBAES2006881, TBAES2007379, TBAES2007428, TBAES2007481, TBAES2007504, TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005,
TESOP2002110, TESOP2002150, TESOP2002273, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247,
TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154,
TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951,
TESTI2000970, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894,
TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707,
TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003327; TESTI2003330, TESTI2003347,
TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004080, TESTI2004085, TESTI2004089, TESTI2004111, TESTI2004119, TESTI2004122, TESTI2004153,
TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994,
TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005767, TESTI2005775, TESTI2005784,
TESTI2005788, TESTI2005827, TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465,
TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007407, TESTI2007422,
TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405,
TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009577,
TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011294, TESTI2011314, TESTI2011315, TESTI2011394, TESTI2011407, TESTI2011448, TESTI2011480, TESTI2011549, TESTI2011575, TESTI2011605, TESTI2011612,
TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257,
TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988,
TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896,
TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581,
TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426,
TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021315, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323,
TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498,
TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196,
TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736,
TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754,
TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718,
TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081,
TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707,
TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958,
TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046456, TESTI2046535, TESTI2046536, TESTI2046552, TESTI2046569,
TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767,
TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600,
TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665,. TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319,
TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441,
TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034,
TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258,
TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977,
TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555,
TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344,
TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904,
TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498,
TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037156, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800,
TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168,
TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000016, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000136, THYMU1000142, THYMU1000144,
THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000359, THYMU1000366, THYMU1000374, THYMU1000382, THYMU1000393, THYMU1000394, THYMU1000399, THYMU1000426, THYMU1000428, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231,
THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556,
THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002583, THYMU2002695, THYMU2002745, THYMU2002789, THYMU2002792, THYMU2002815, THYMU2002841, THYMU2002852, THYMU2002910, THYMU2002950, THYMU2002983, THYMU2003012, THYMU2003069, THYMU2003107, THYMU2003124, THYMU2003166, THYMU2003232, THYMU2003242, THYMU2003249, THYMU2003270,
THYMU2003282, THYMU2003287, THYMU2003336, THYMU2003366, THYMU2003397, THYMU2003419, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003891, THYMU2003907, THYMU2003932, THYMU2003981, THYMU2004003, THYMU2004008, THYMU2004042, THYMU2004108, THYMU2004111, THYMU2004123, THYMU2004139, THYMU2004152, THYMU2004169, THYMU2004320, THYMU2004336, THYMU2004344, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916,
THYMU2004986, THYMU2005001, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005507, THYMU2005545, THYMU2005546, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813,
THYMU2006913, THYMU2006946, THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282,
THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009658, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637,
THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012631,
THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013916, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441,
THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721,
THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042,
THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796,
THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101,
THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029,
THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763,
THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081,
THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856,
THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706,
THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970,
THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542,
THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004386, TKIDN2004458, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641,
TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200,
TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800,
TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000081, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000472, TRACH2000496, TRACH2000497, TRACH2000502, TRACH2000522, TRACH2000559,
TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001478, TRACH2001523,
TRACH2001525, TRACH2001532, TRACH2001549, TRACH2001592, TRACH2001596, TRACH2001612, TRACH2001621, TRACH2001646, TRACH2001651, TRACH2001684, TRACH2001810, TRACH2001898, TRACH2001933, TRACH2001968, TRACH2001996, TRACH2002043, TRACH2002054, TRACH2002092, TRACH2002100, TRACH2002138, TRACH2002333, TRACH2002537, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292,
TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894,
TRACH2007969, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771,
TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014268, TRACH2014284, TRACH2014337, TRACH2014356,
TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980,
TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299,
TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892,
TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840,
TRACH3004931, TRACH3004934, TRACH3005072, TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061, TRACH3008093,
TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043,
TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058,
TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428,
TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400,
TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063,
TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143,
UMVEN1000156, UMVEN2000046, UMVEN2000069, UMVEN2000121, UMVEN2000152, UMVEN2000354, UMVEN2000453, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000465, UTERU2000517,
UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135,
UTERU2003321, UTERU2003399, UTERU2003411, UTERU2003456, UTERU2003577, UTERU2003704, UTERU2003926, UTERU2003973, UTERU2004015, UTERU2004039, UTERU2004061, UTERU2004073, UTERU2004163, UTERU2004197, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005346, UTERU2005354, UTERU2005446, UTERU2005449, UTERU2005450, UTERU2005533, UTERU2005593, UTERU2005601, UTERU2005621, UTERU2005664, UTERU2005822, UTERU2005903, UTERU2005905, UTERU2006103, UTERU2006115, UTERU2006137, UTERU2006182, UTERU2006412, UTERU2006429, UTERU2006486, UTERU2006524,
UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009414, UTERU2009435, UTERU2009483, UTERU2009510, UTERU2009538,
UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786,
UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902,
UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292,
UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742,
UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423,
UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660,
UTERU3003774, UTERU3003776, UTERU3004384, UTERU3004418, UTERU3004477, UTERU3004523, UTERU3004616, UTERU3004635; UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690,
UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172,
UTERU3018255, UTERU3018616, UTERU3018711, UTERU3019078

Of the 17,176 genes including the cDNAs of the present invention, genes, whose expression levels were high enough to detect in each library of HELAC, HHDPC, HLUNG, HSYRA, IMR32, JCMLC, and KIDNE, were analyzed for the expression frequencies. The analysis results are described below. Each is a relative value, where the expression level over all tissues is taken as 100. The larger this value, the greater the level of expression.

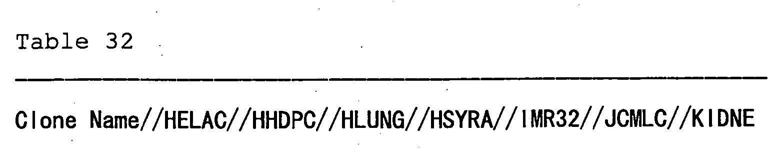

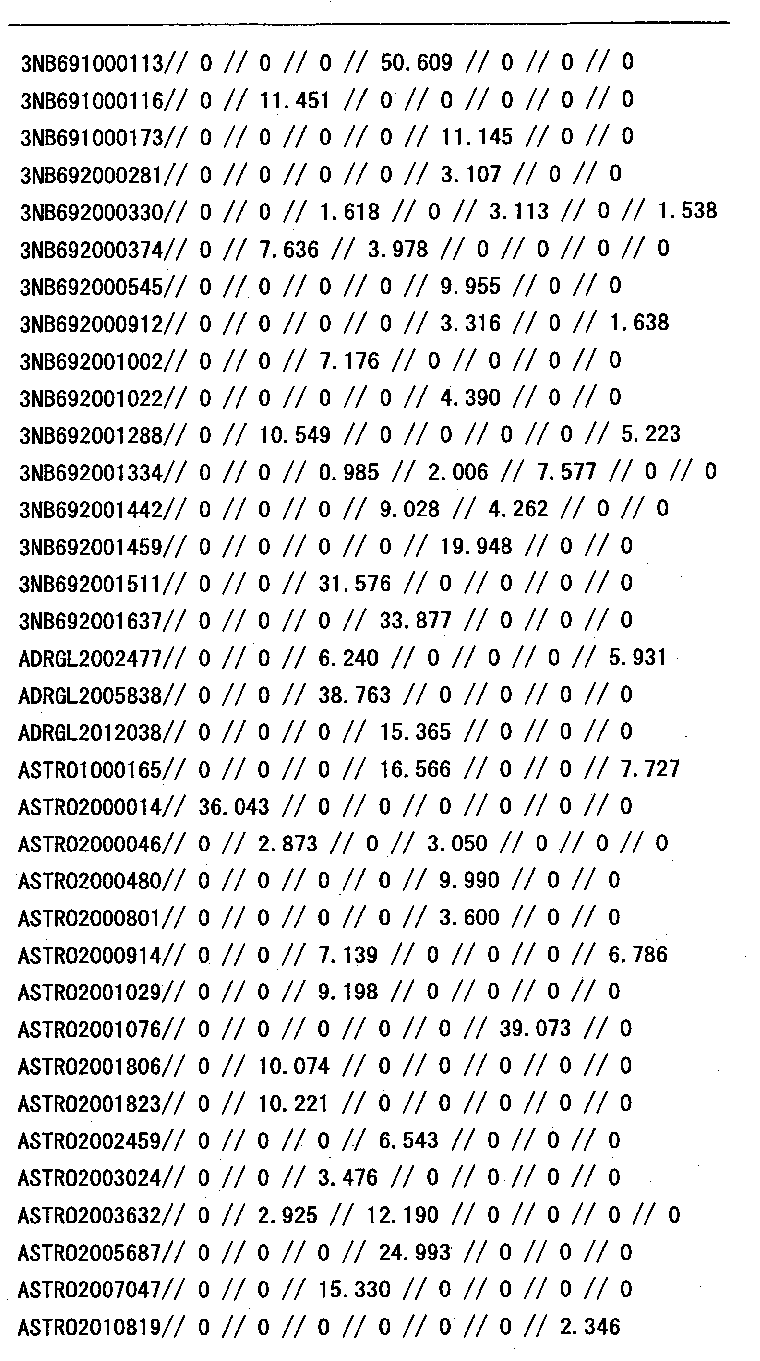

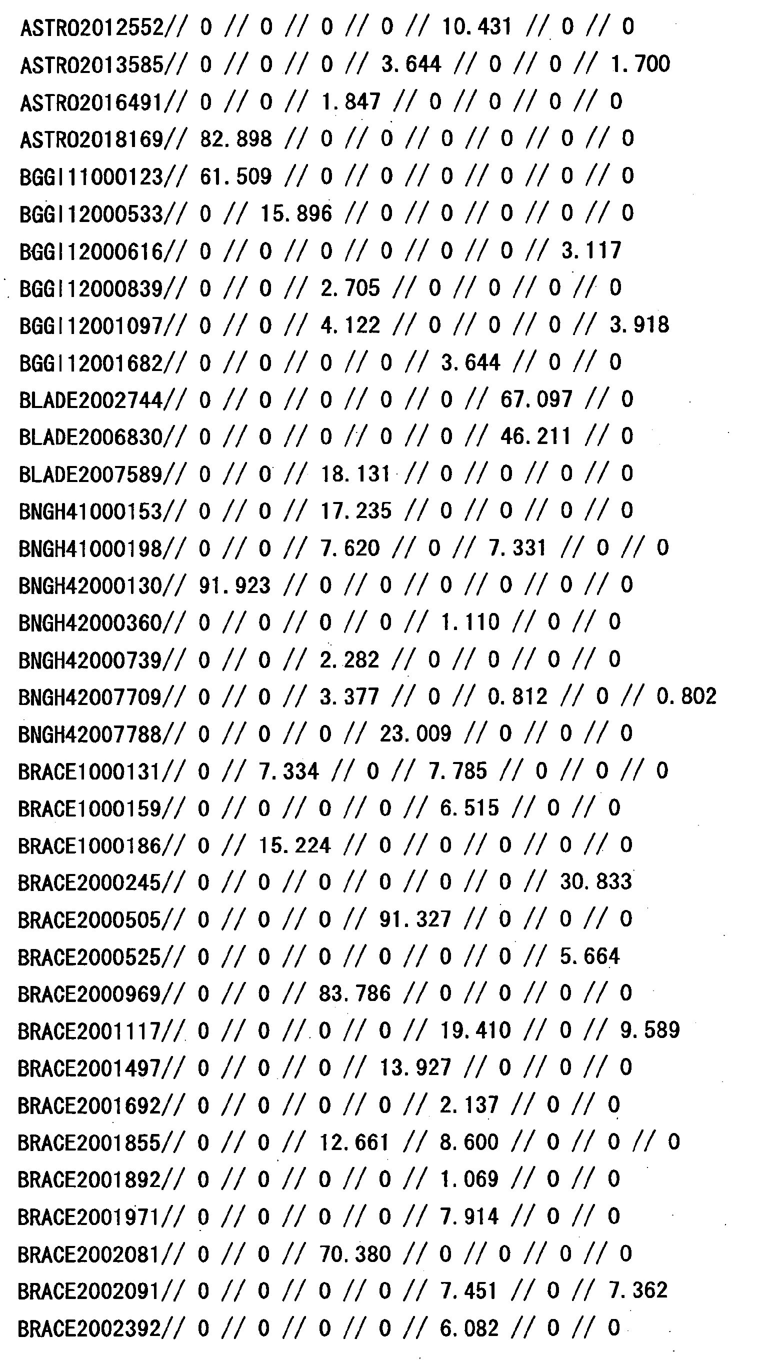

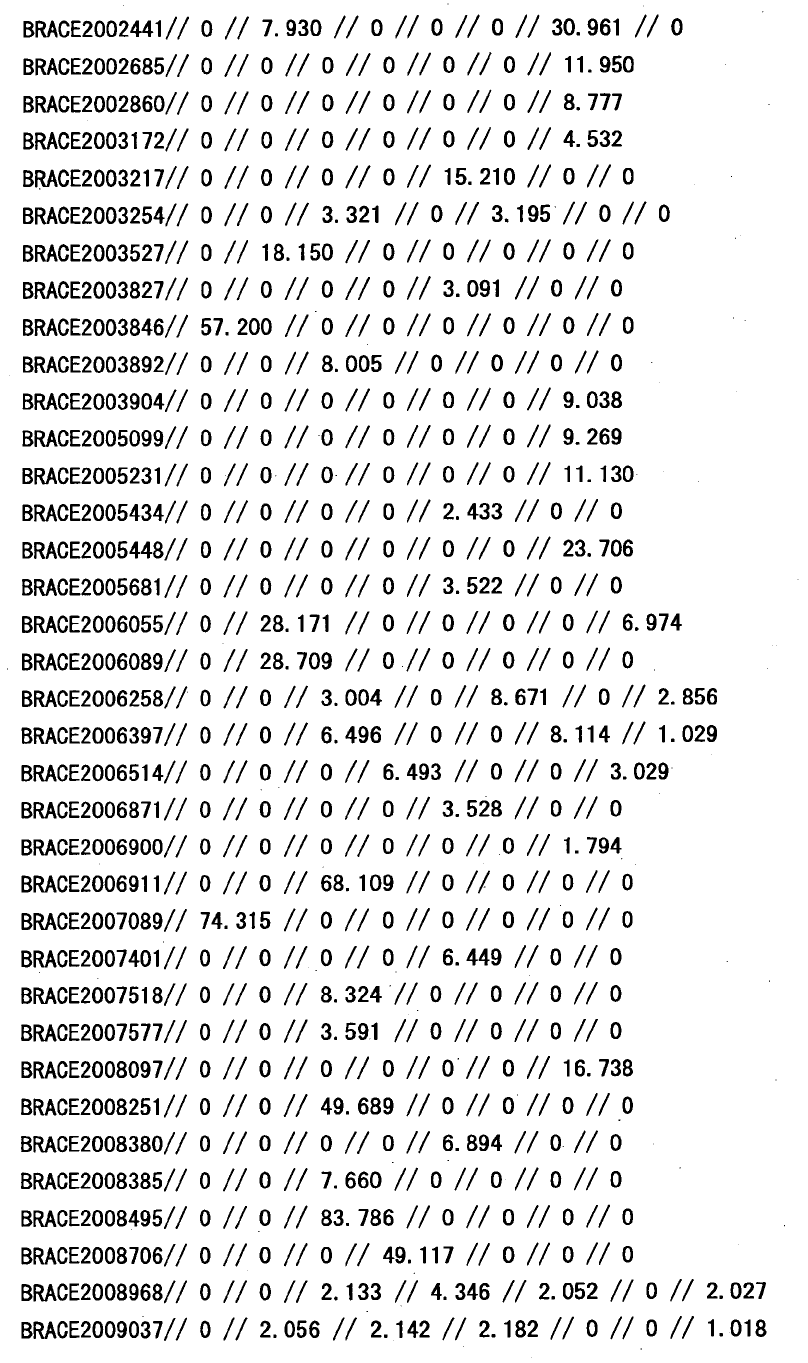

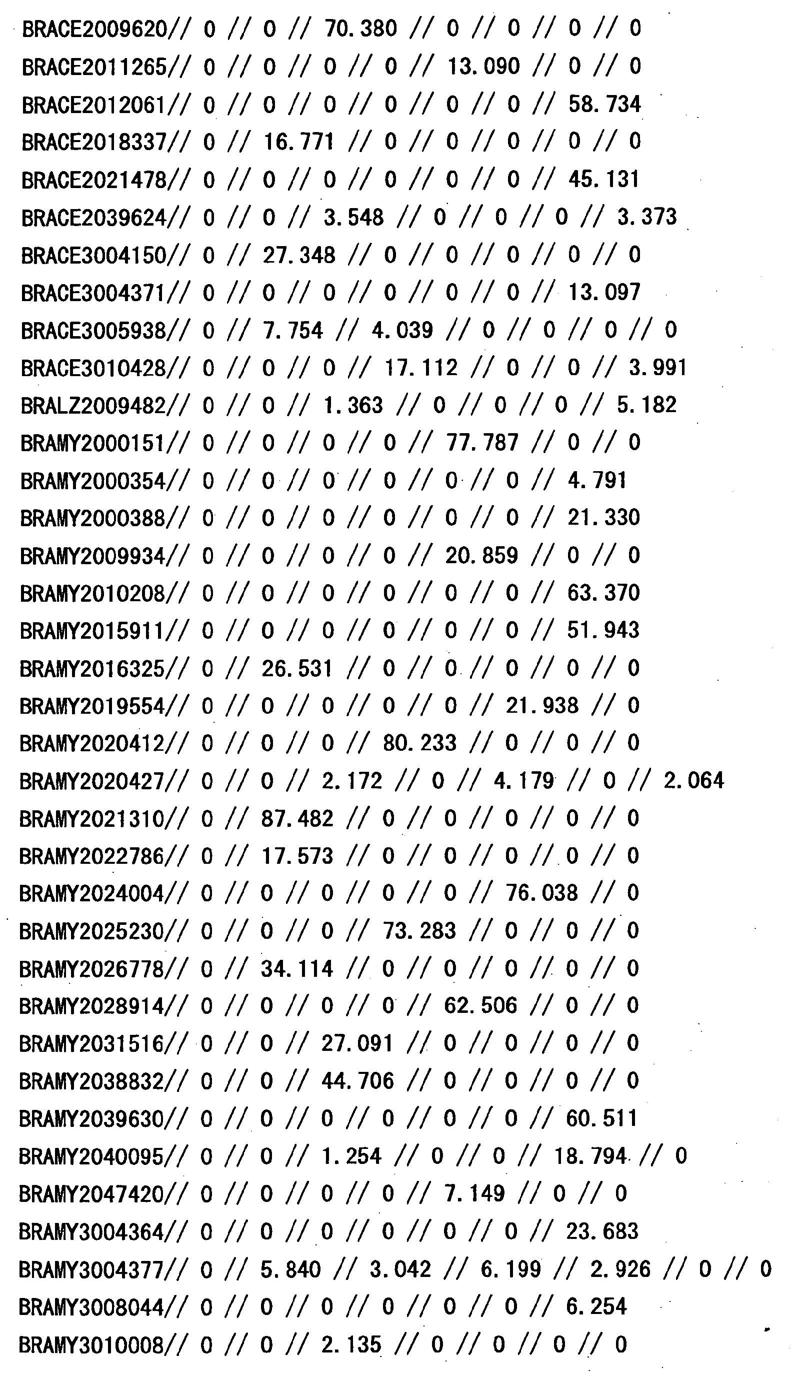

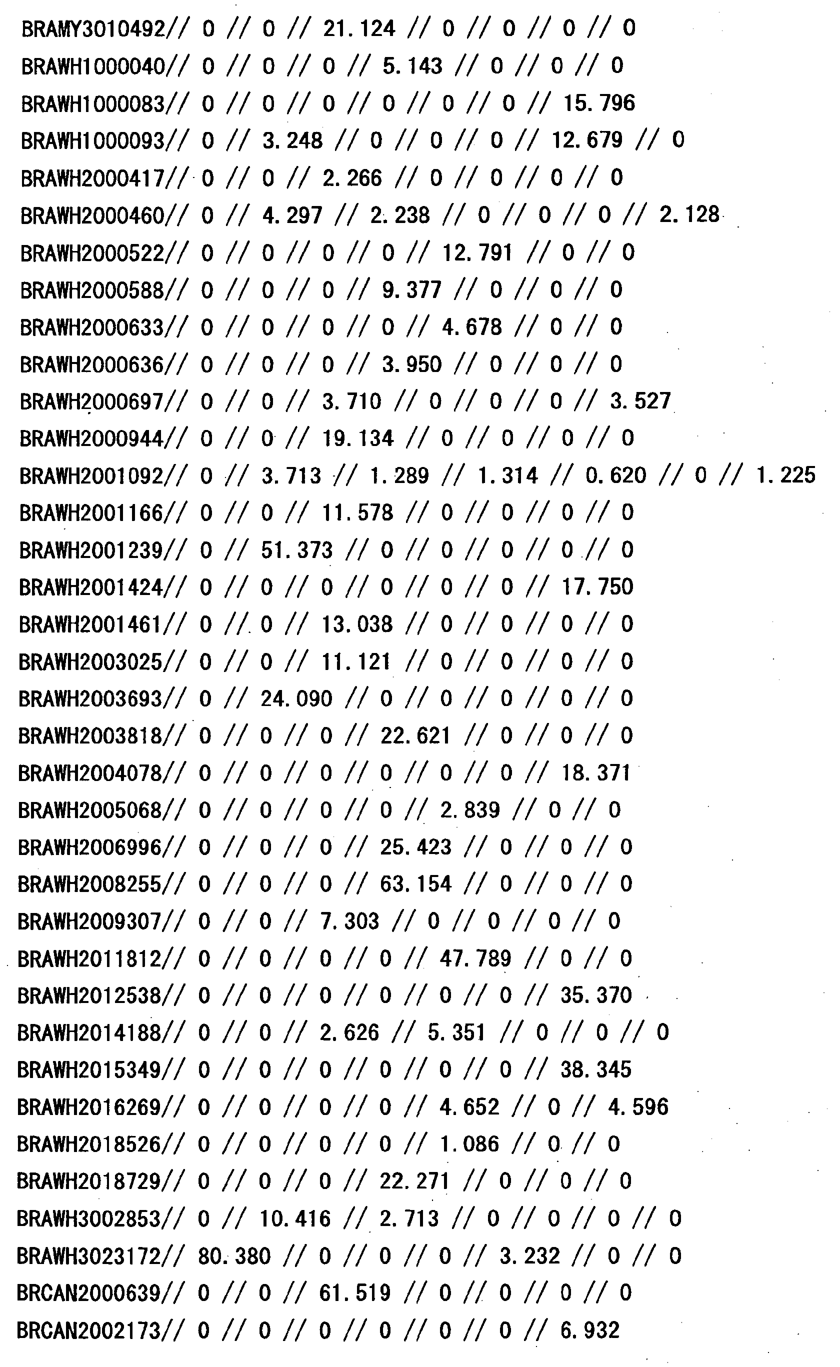

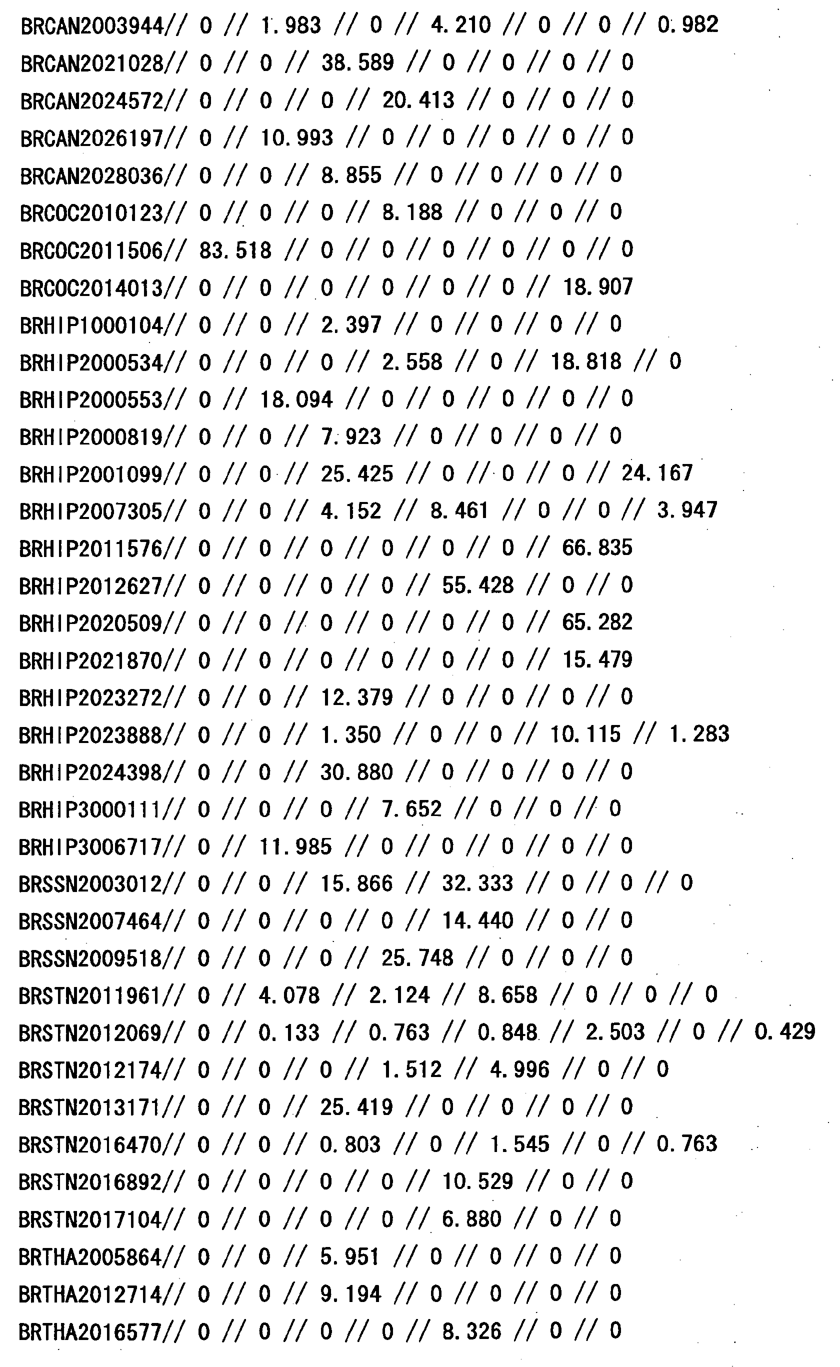

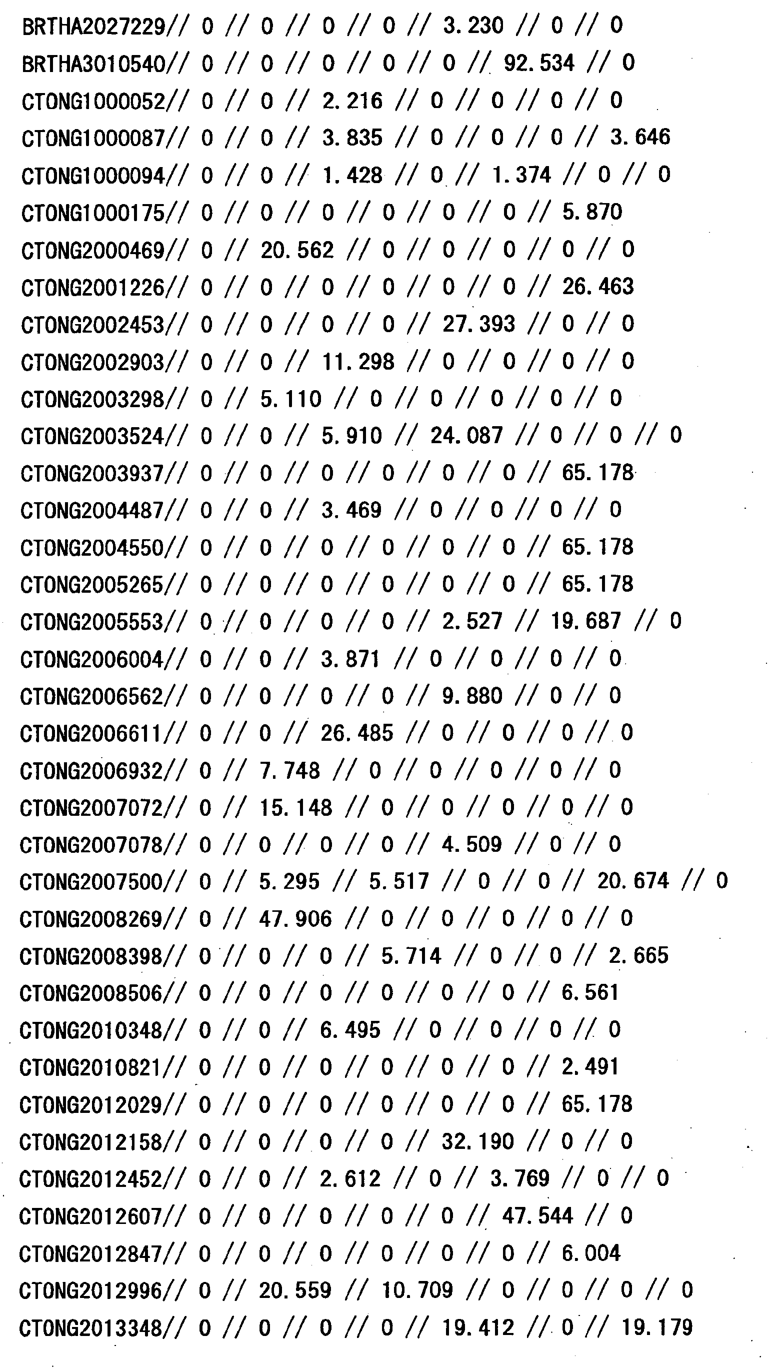

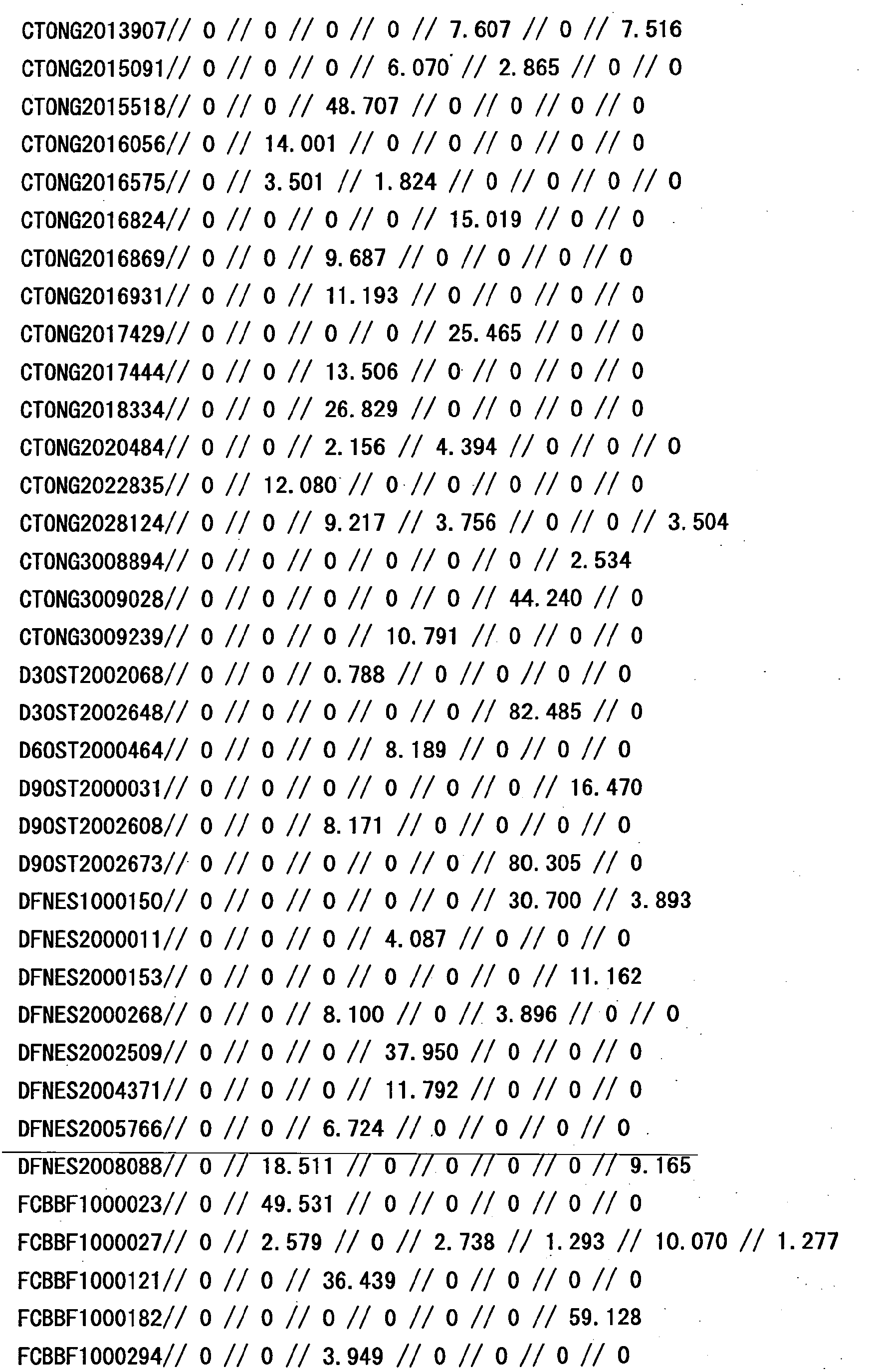

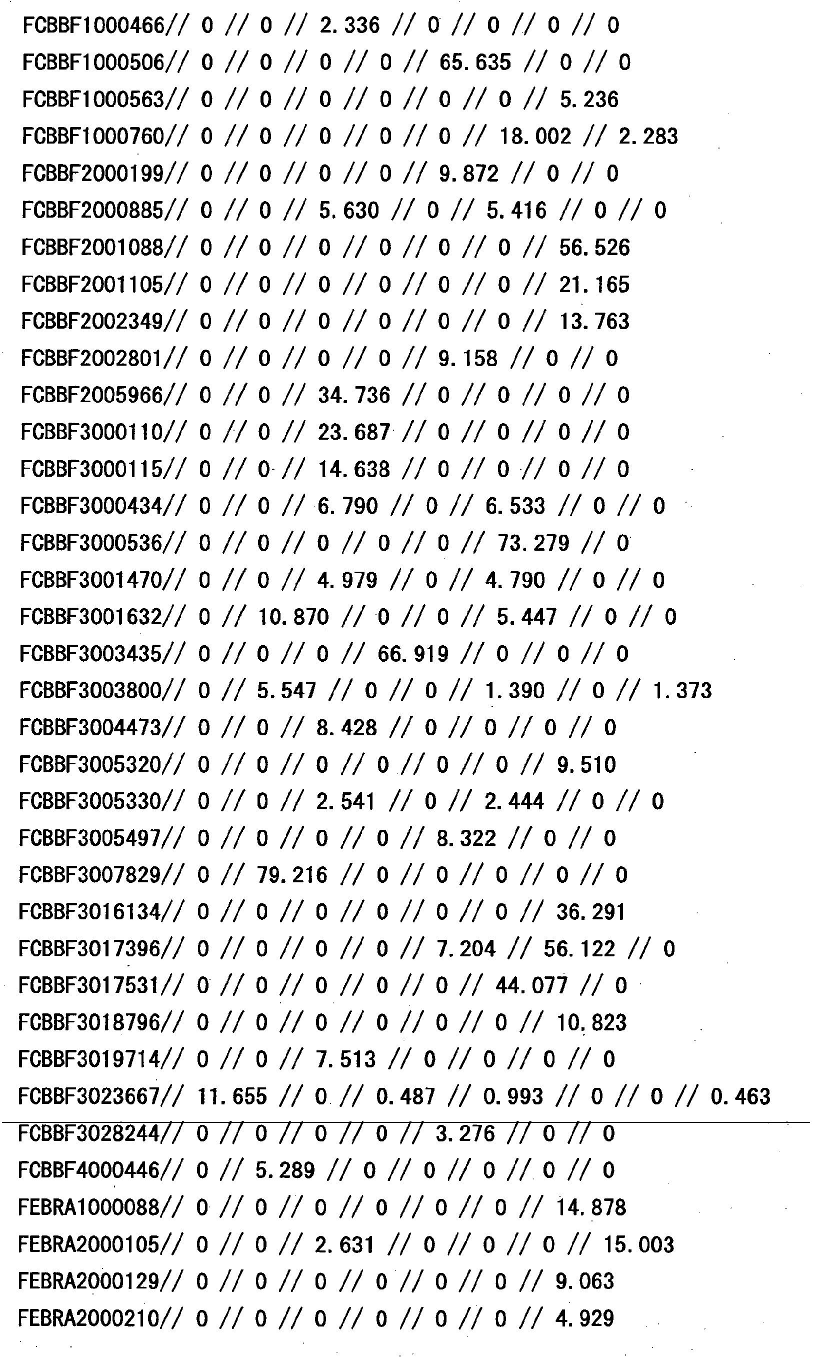

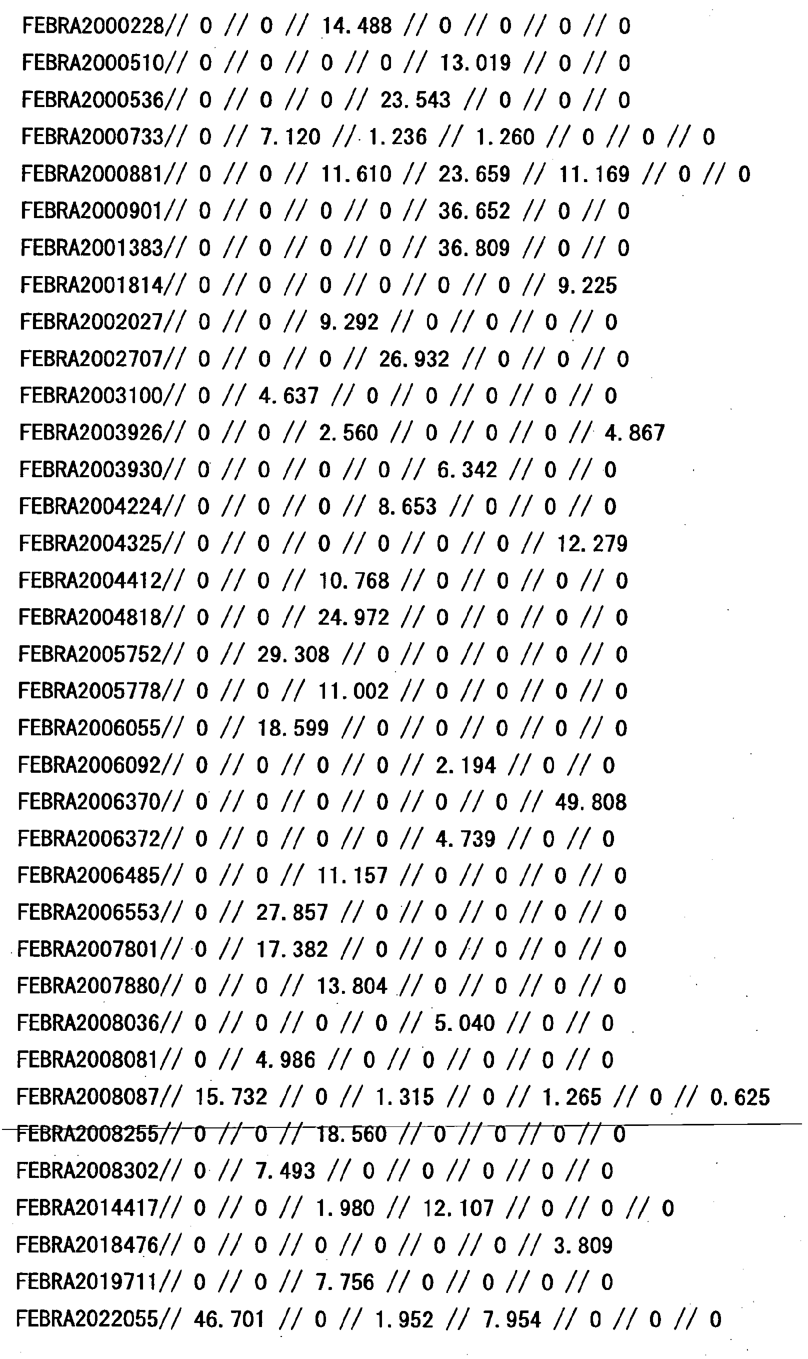

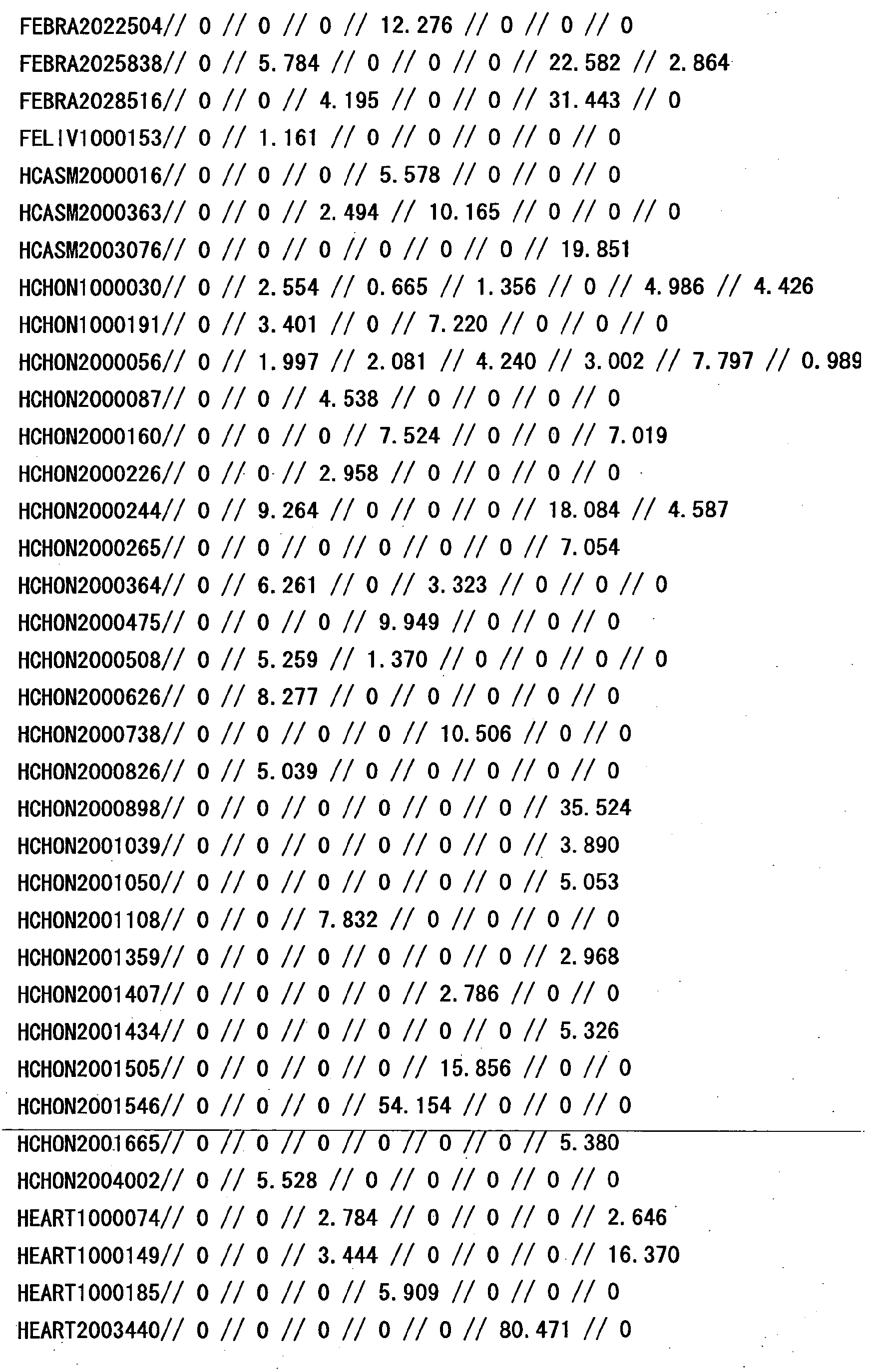

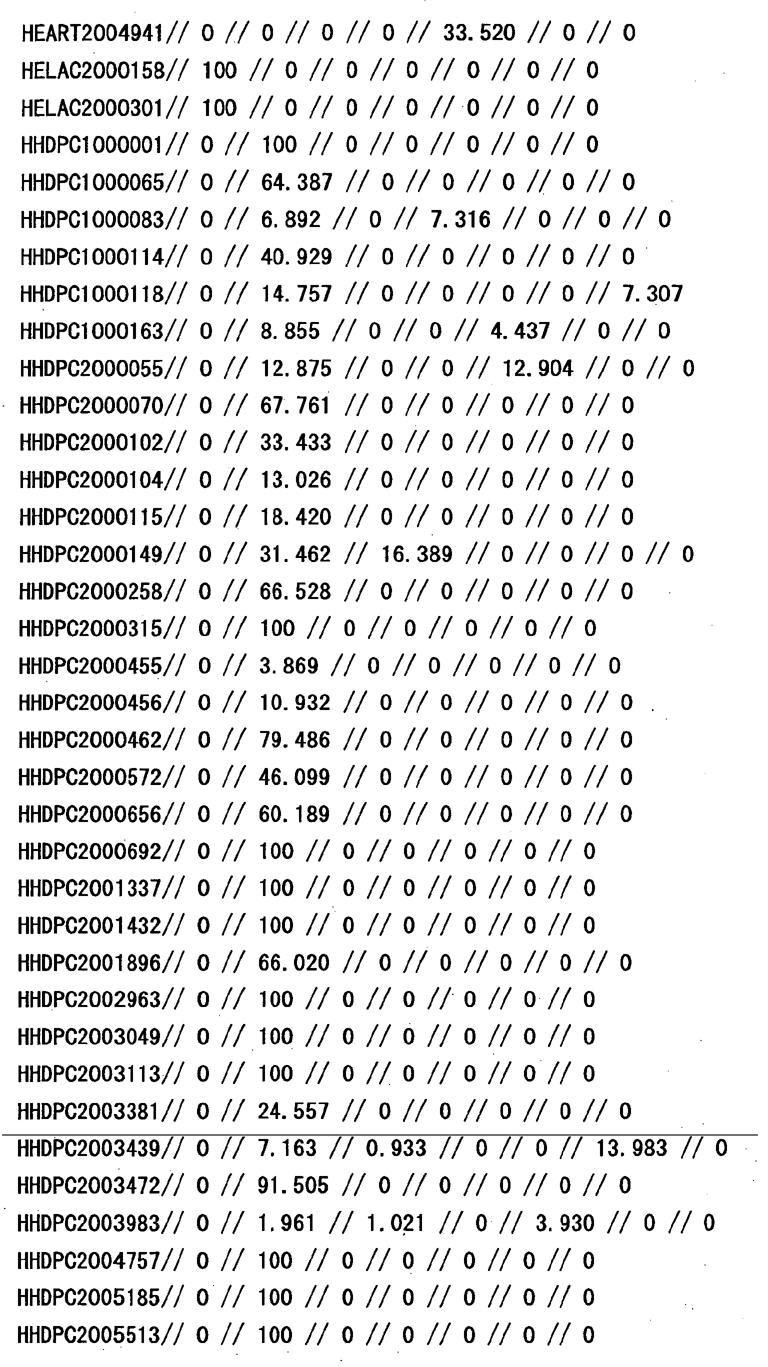

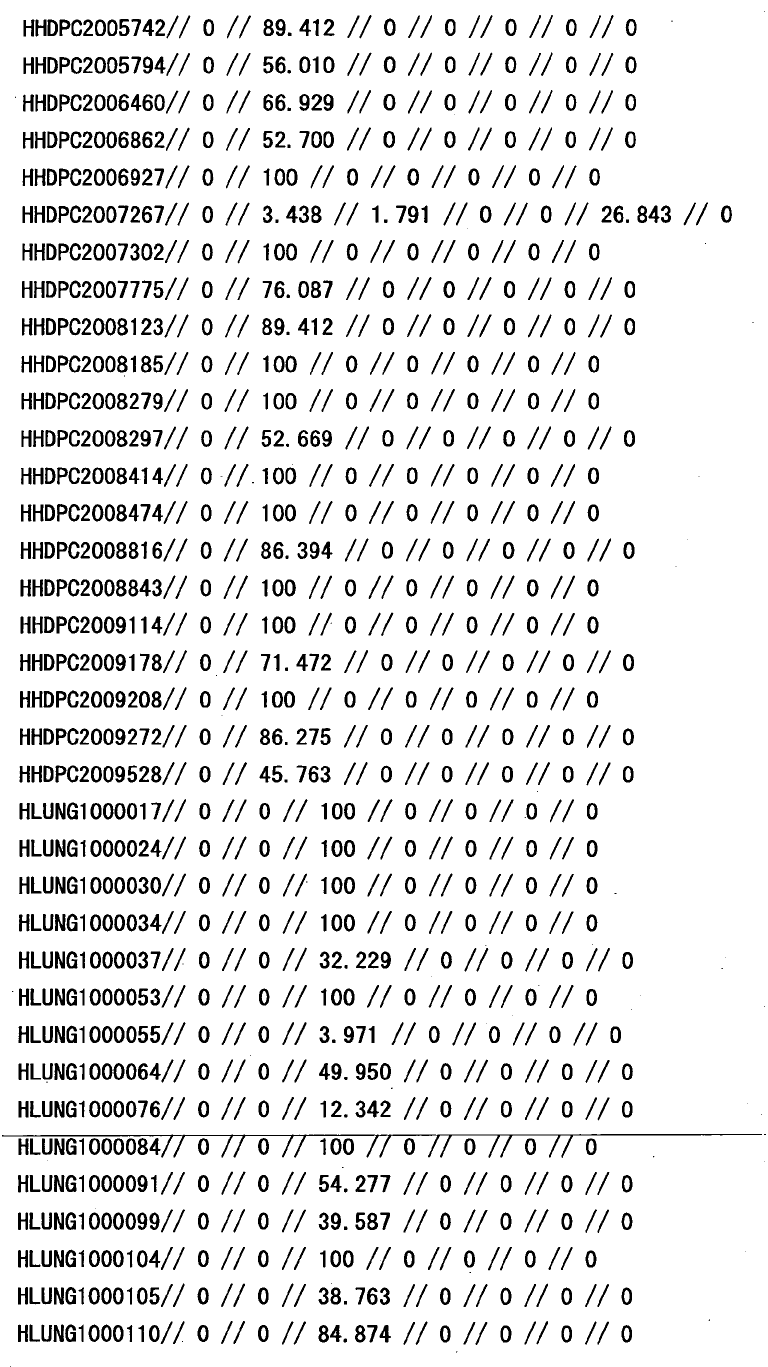

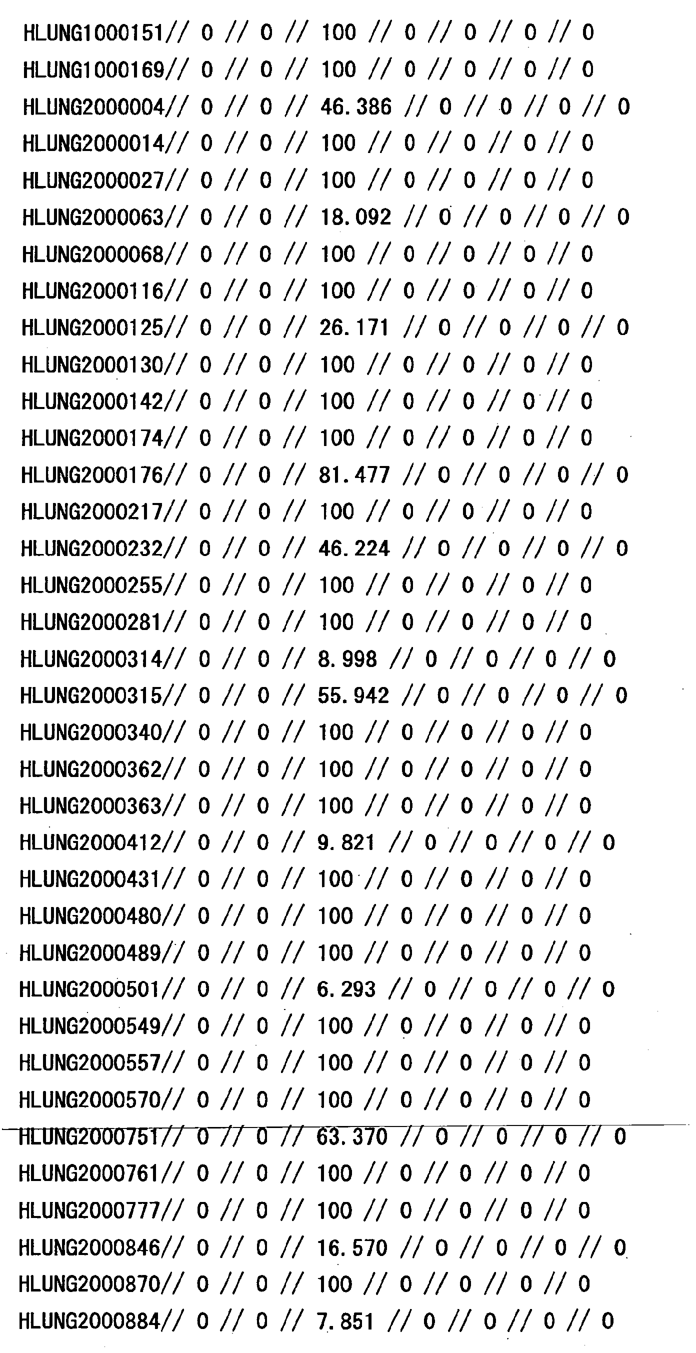

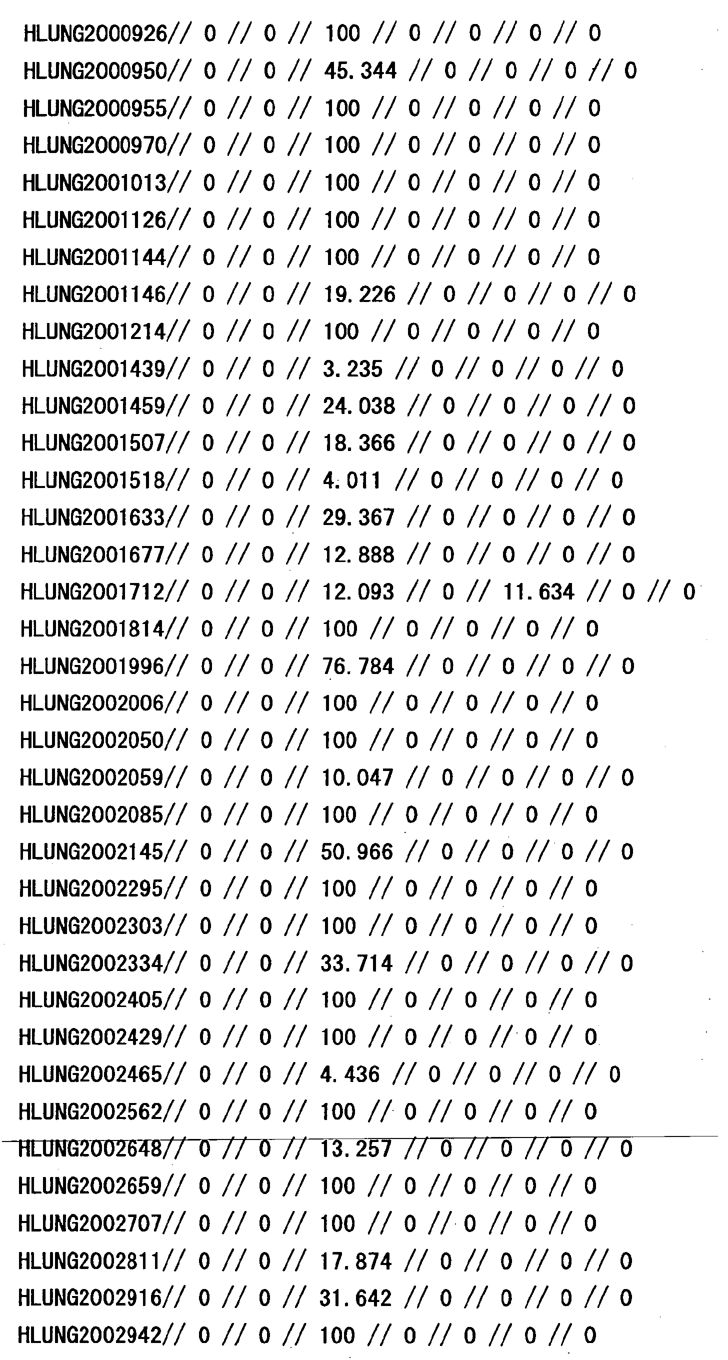

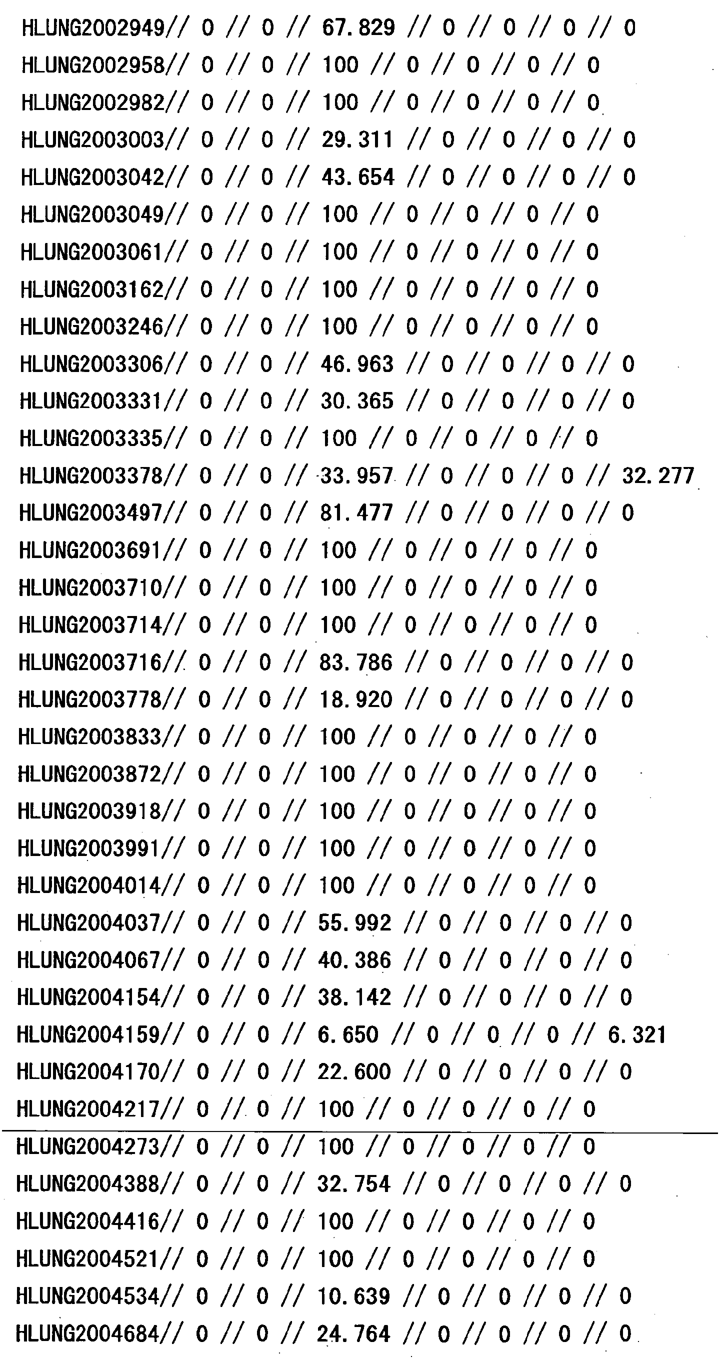

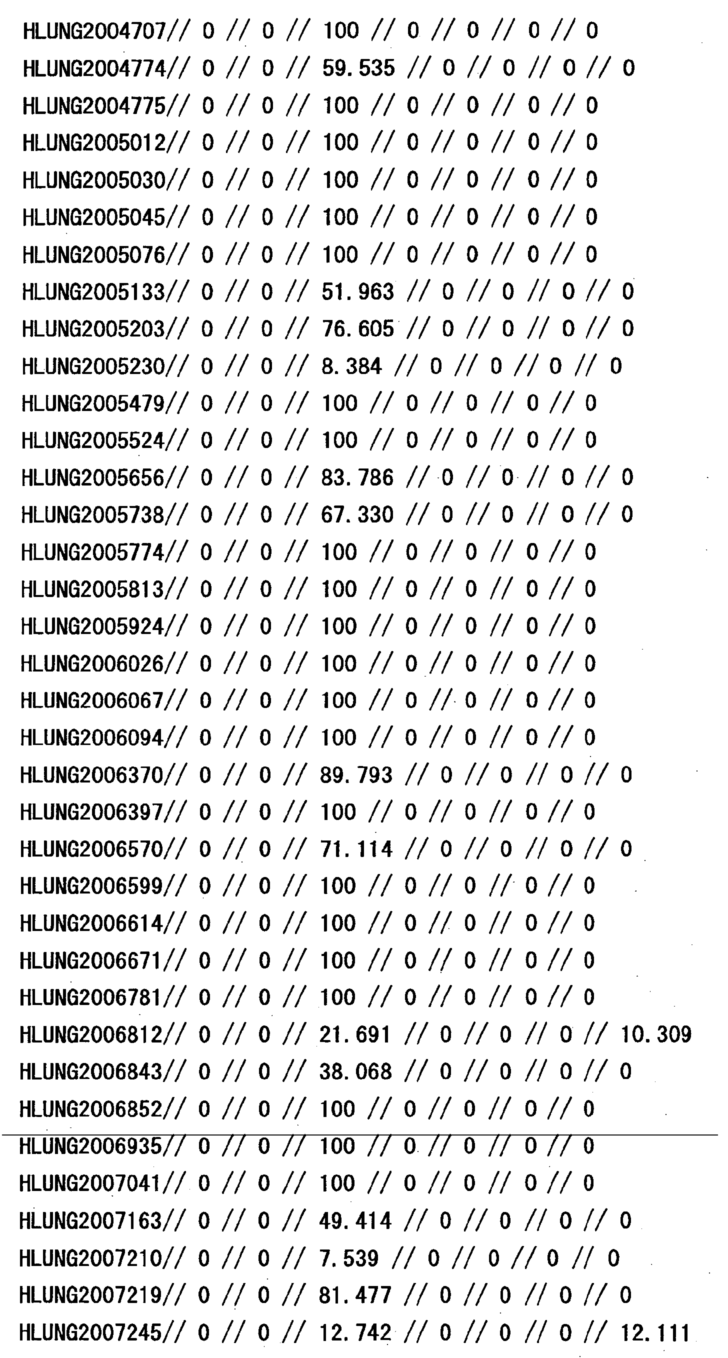

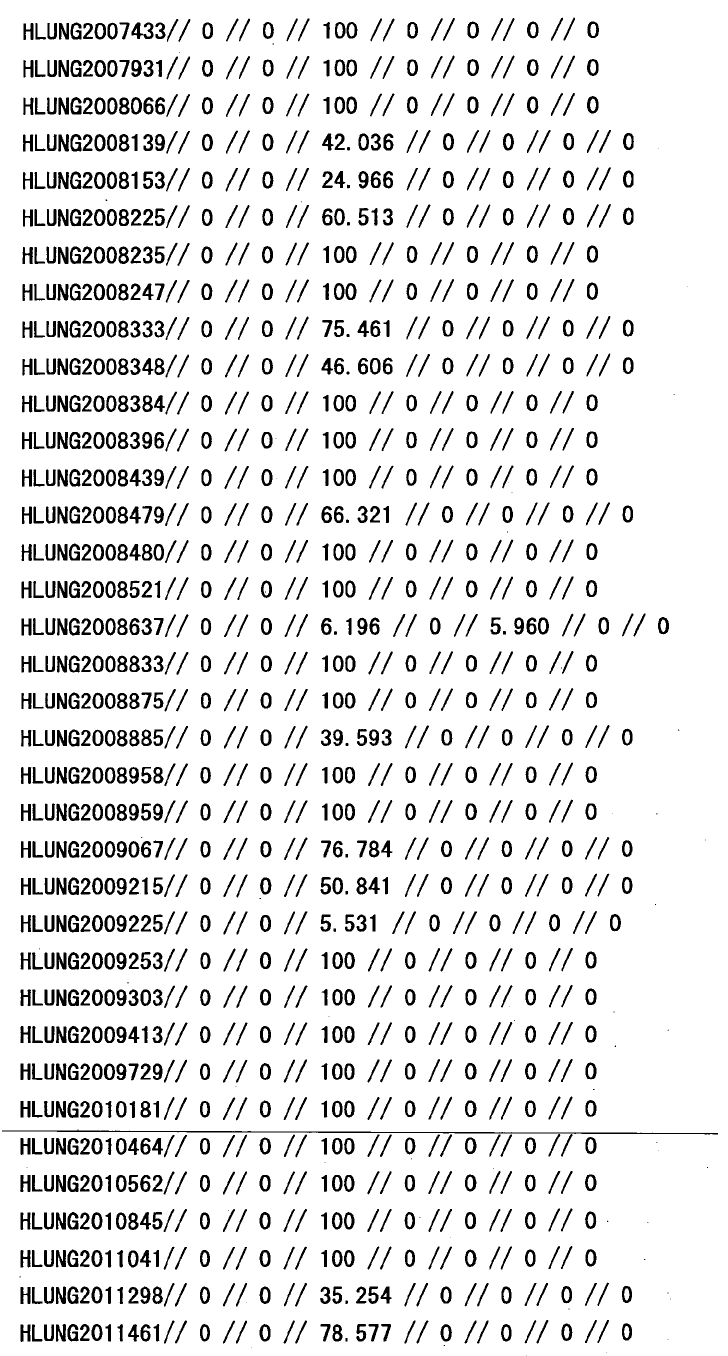

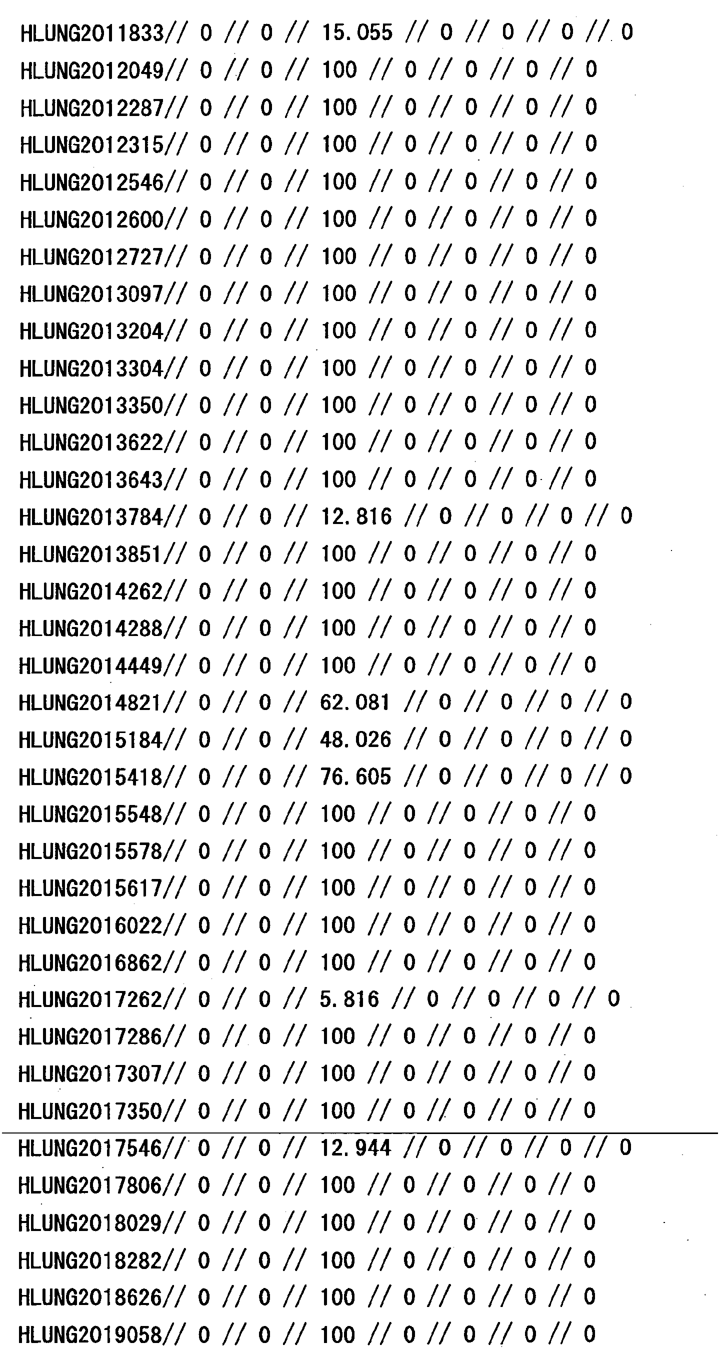

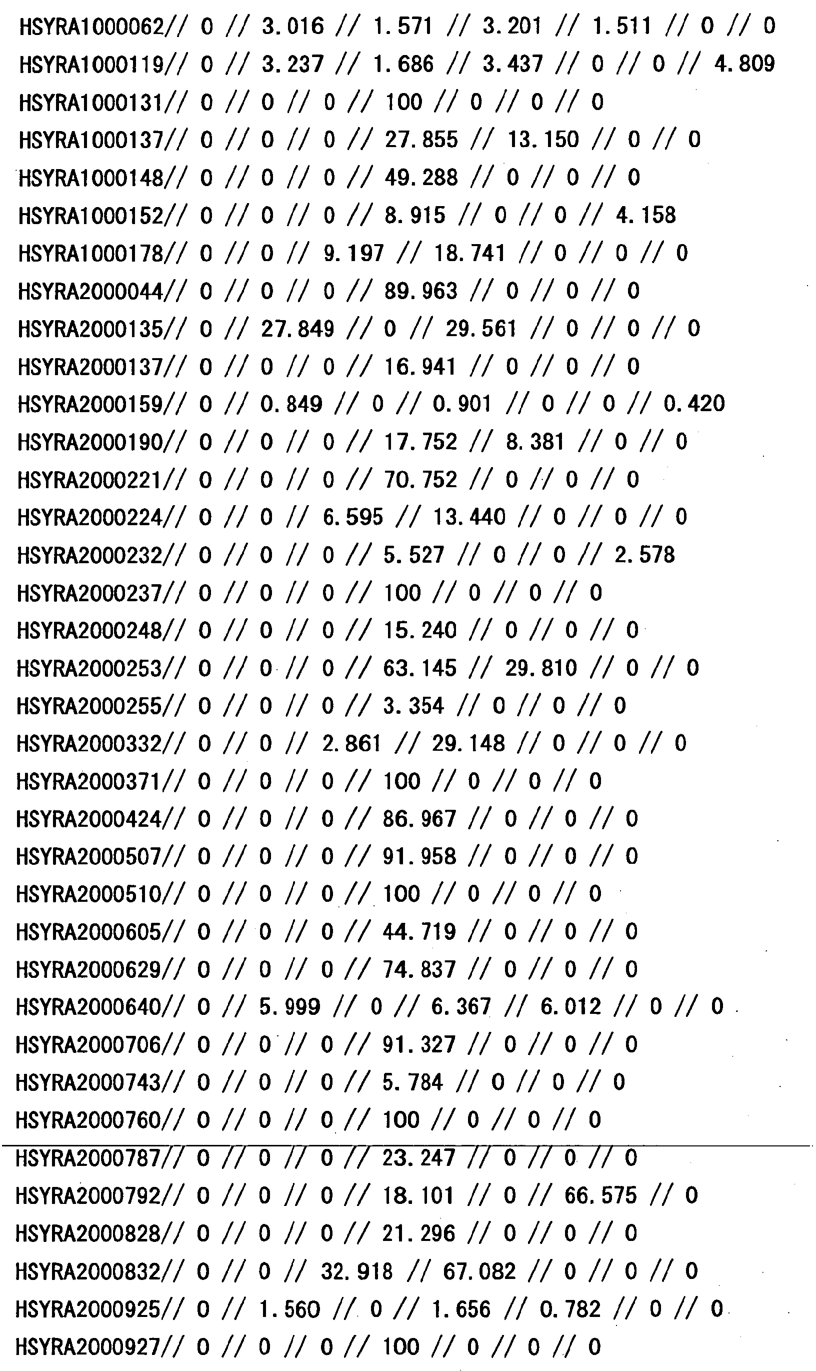

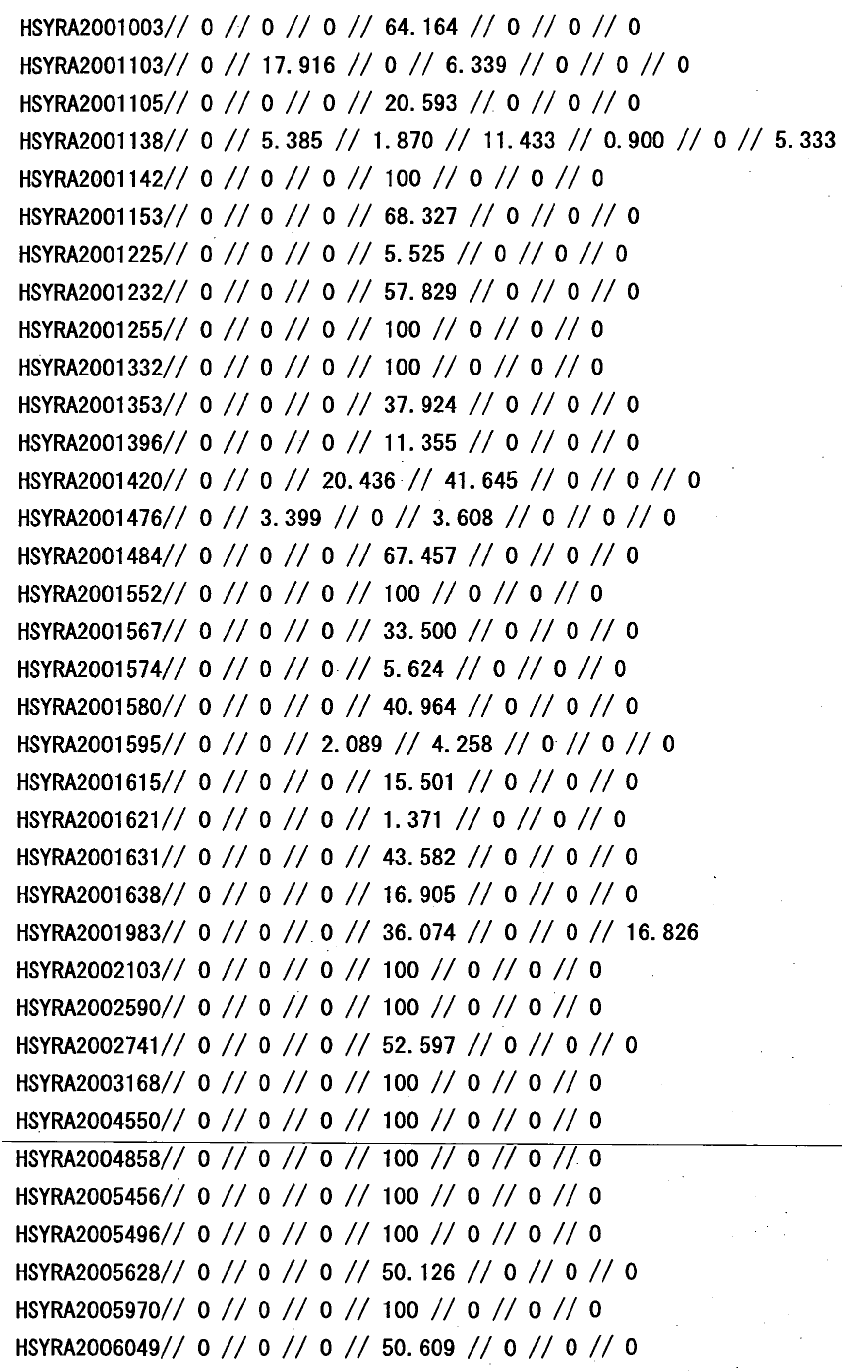

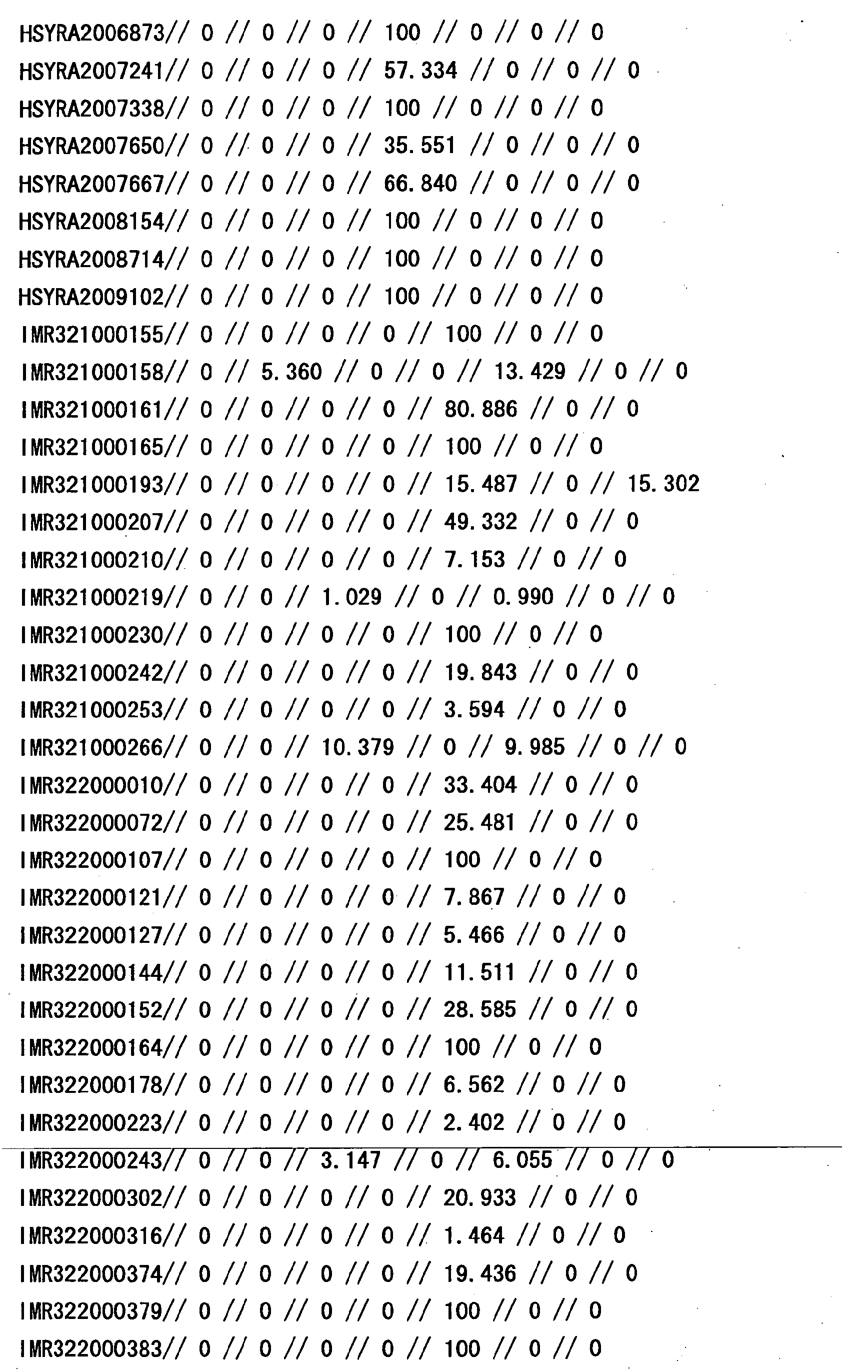

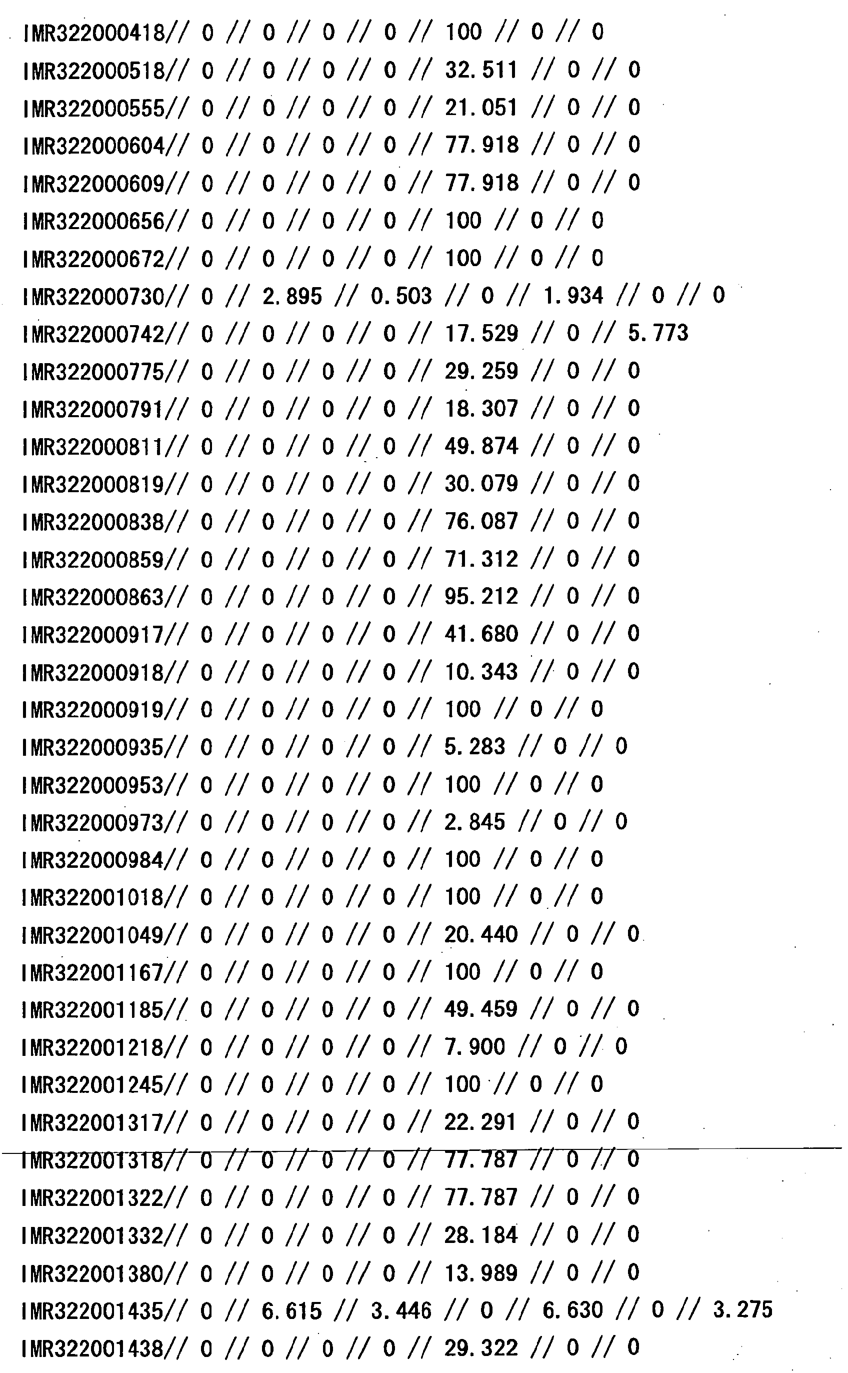

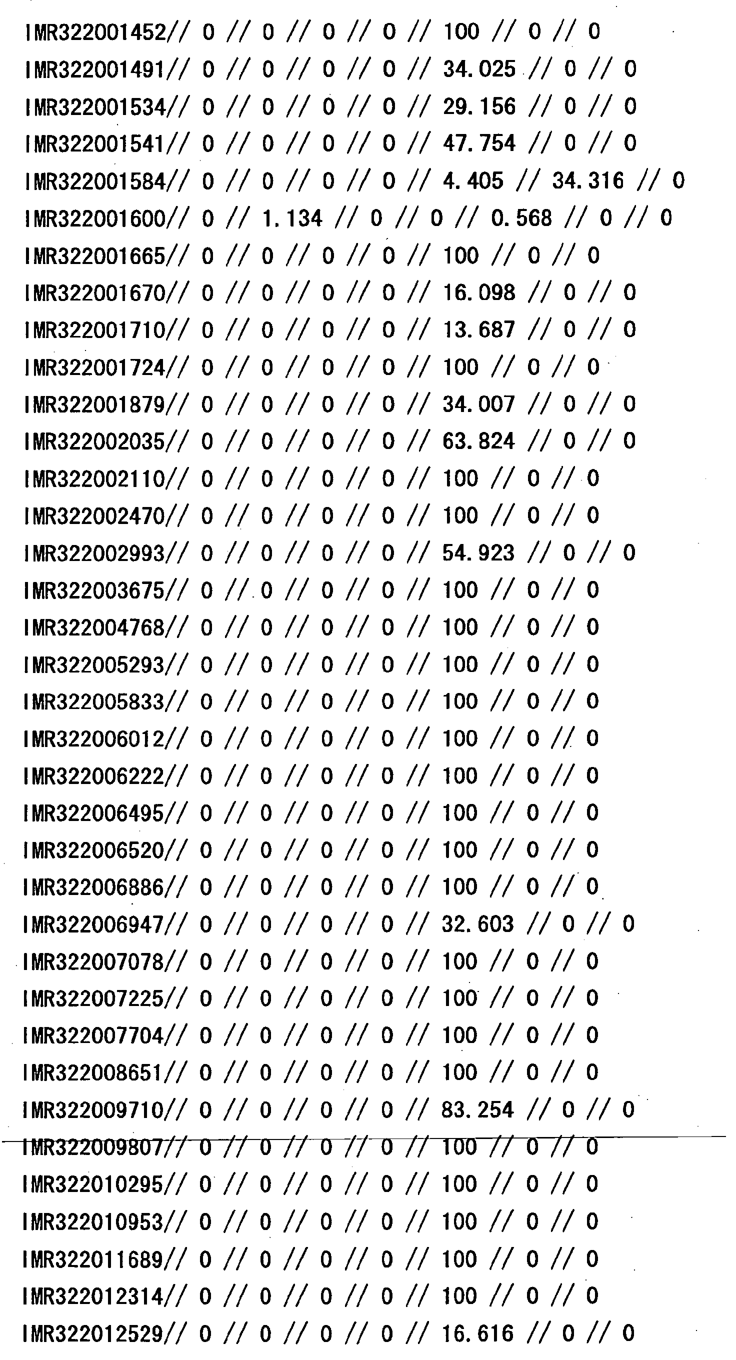

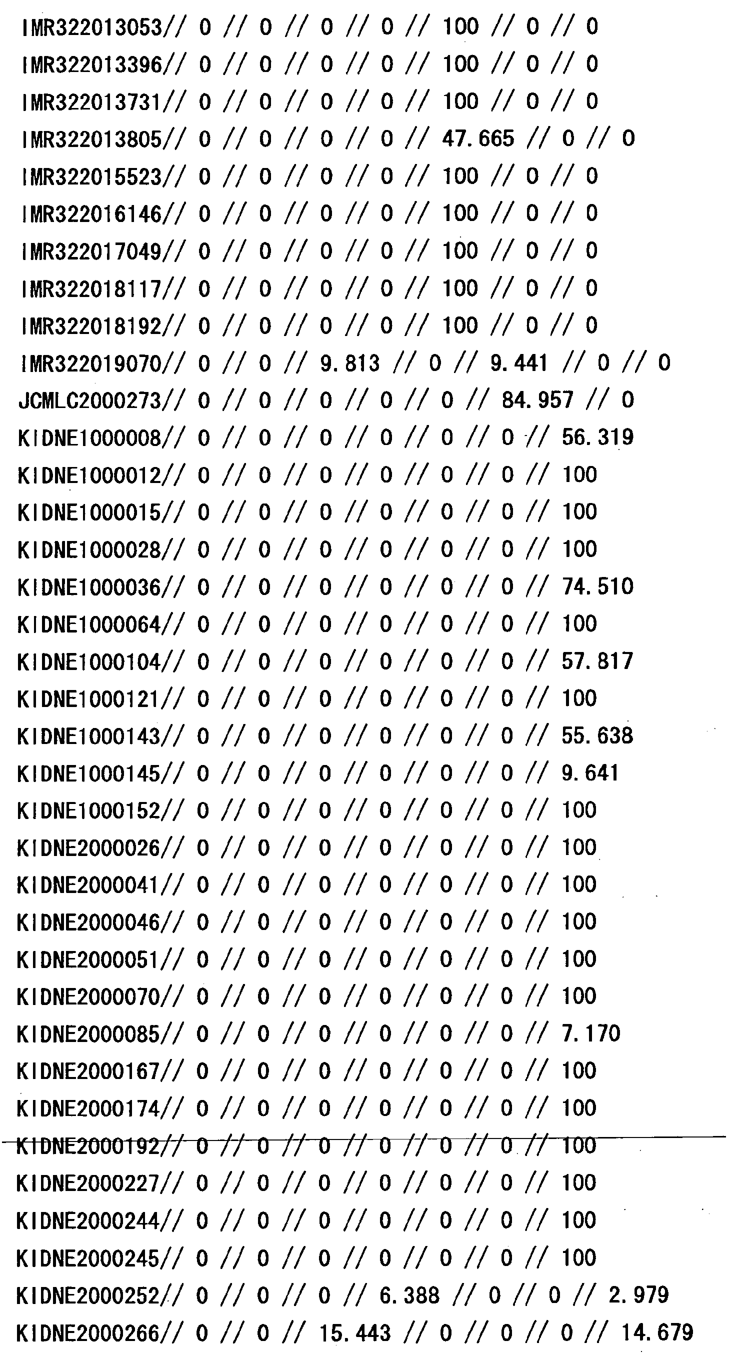

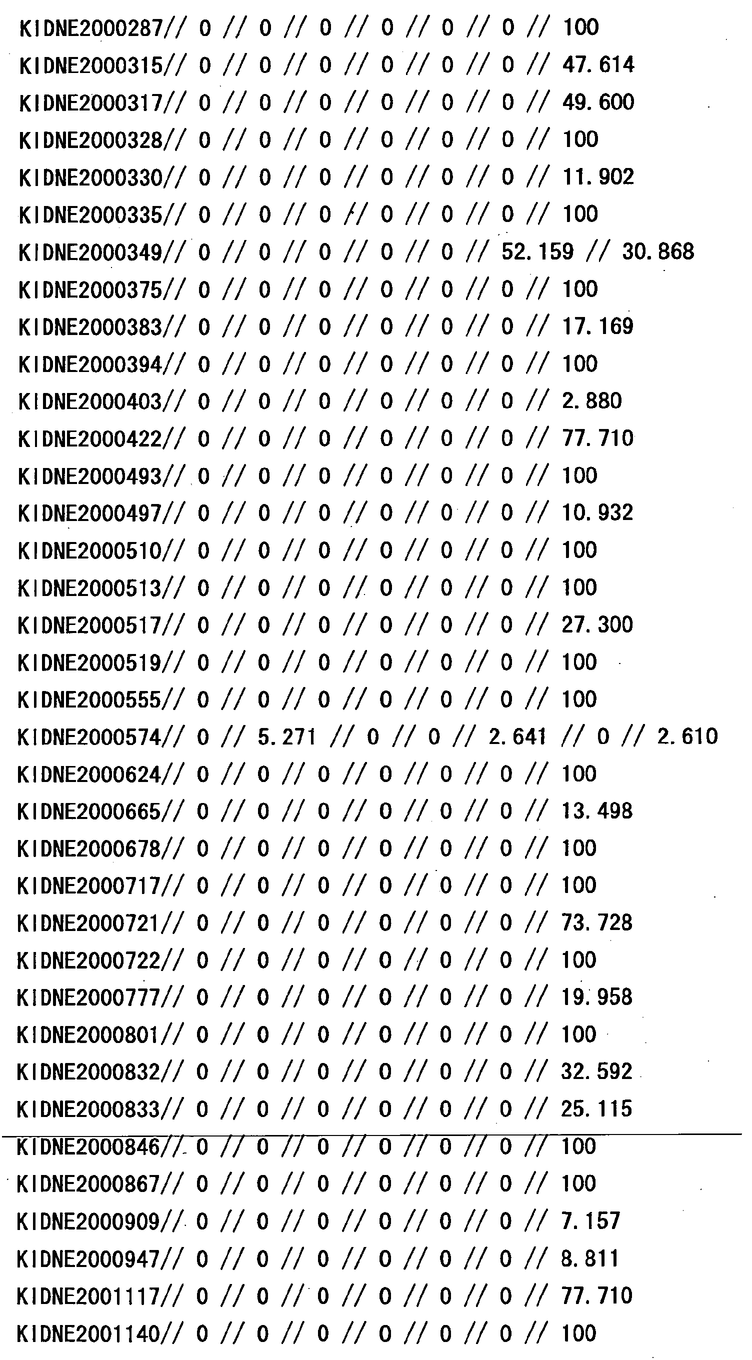

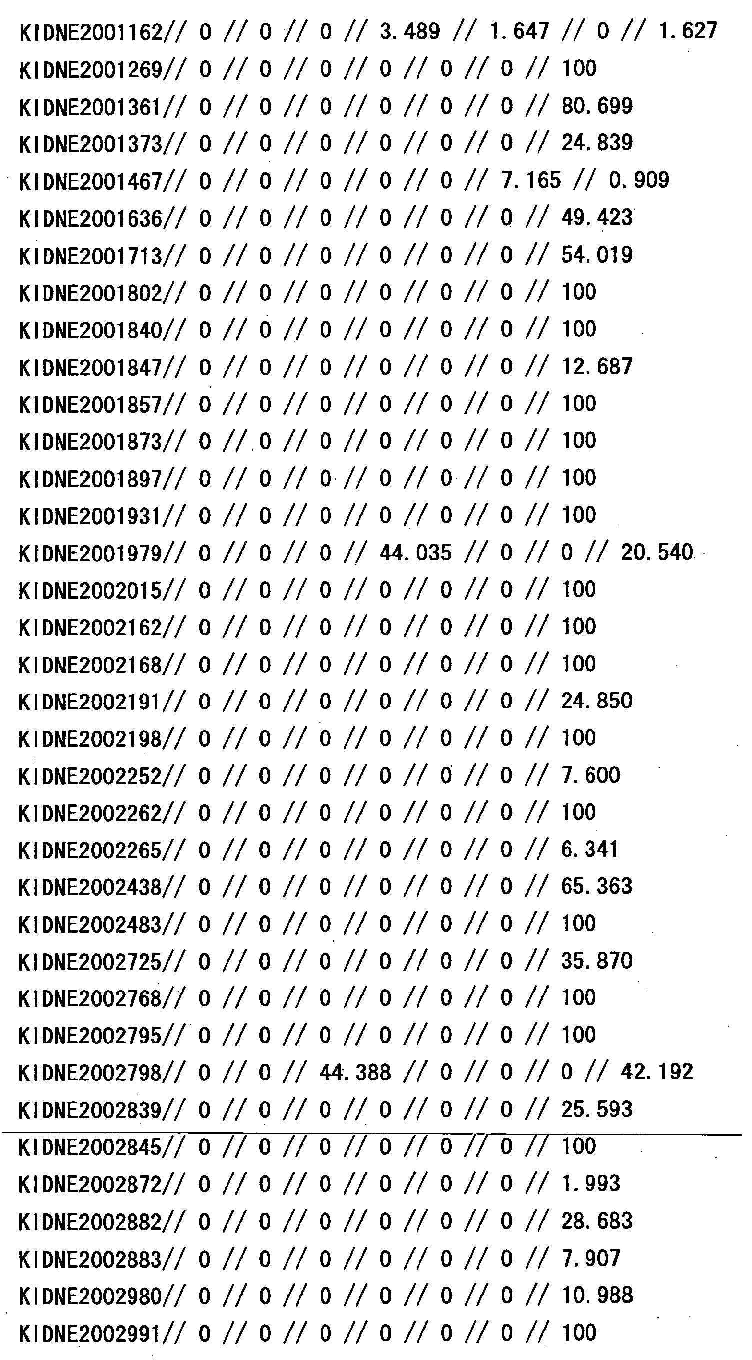

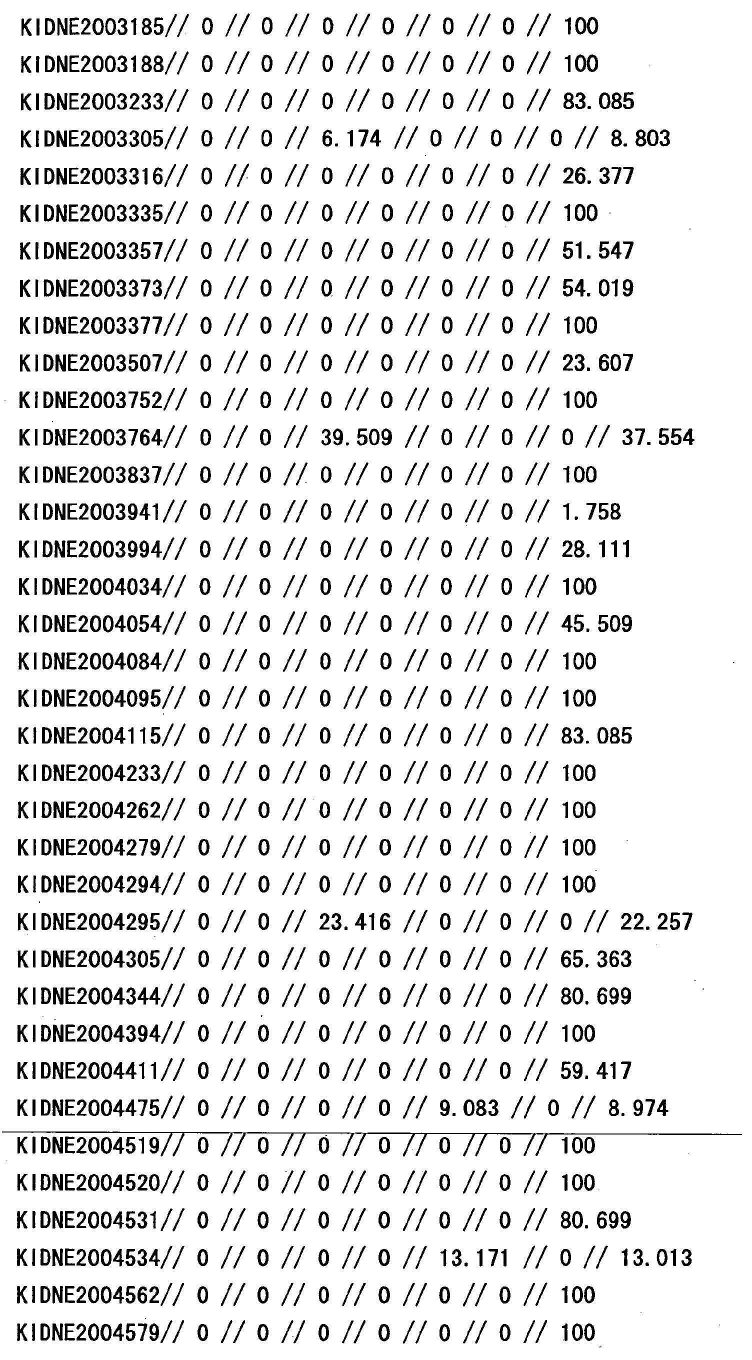

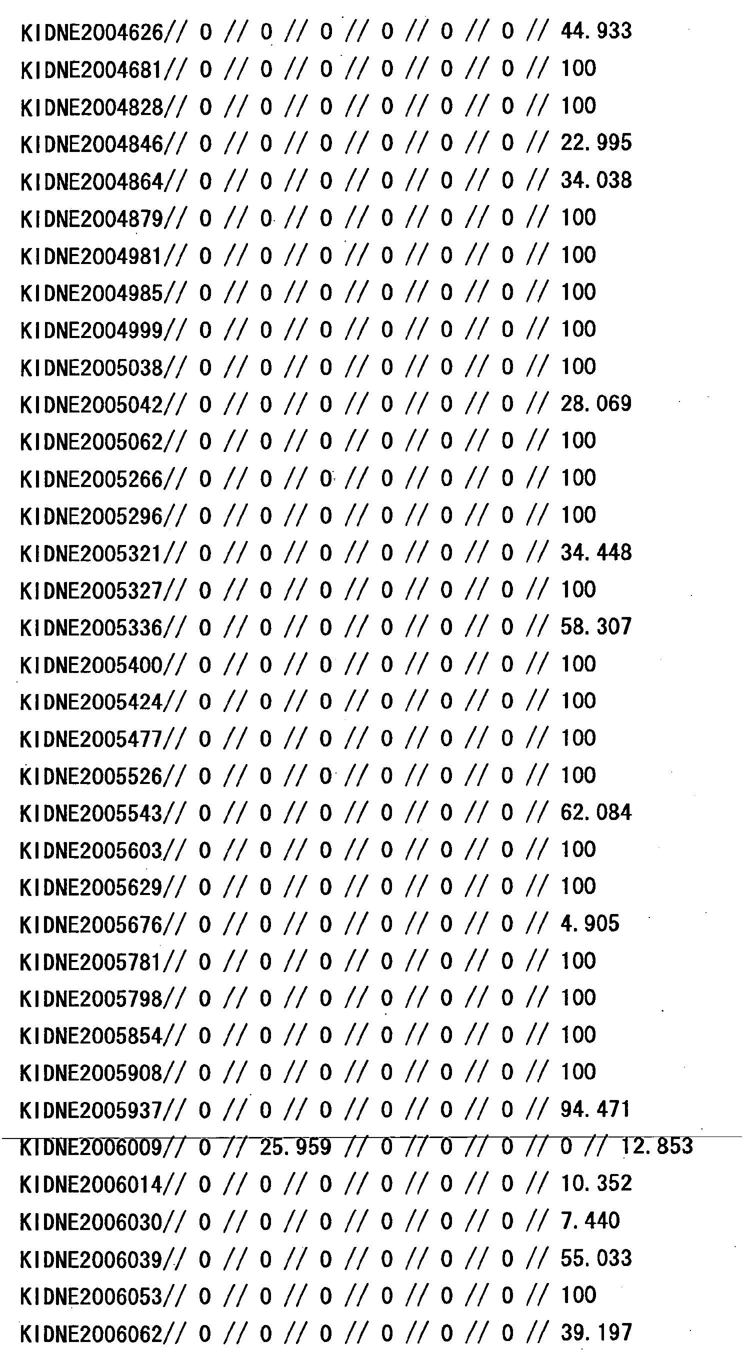

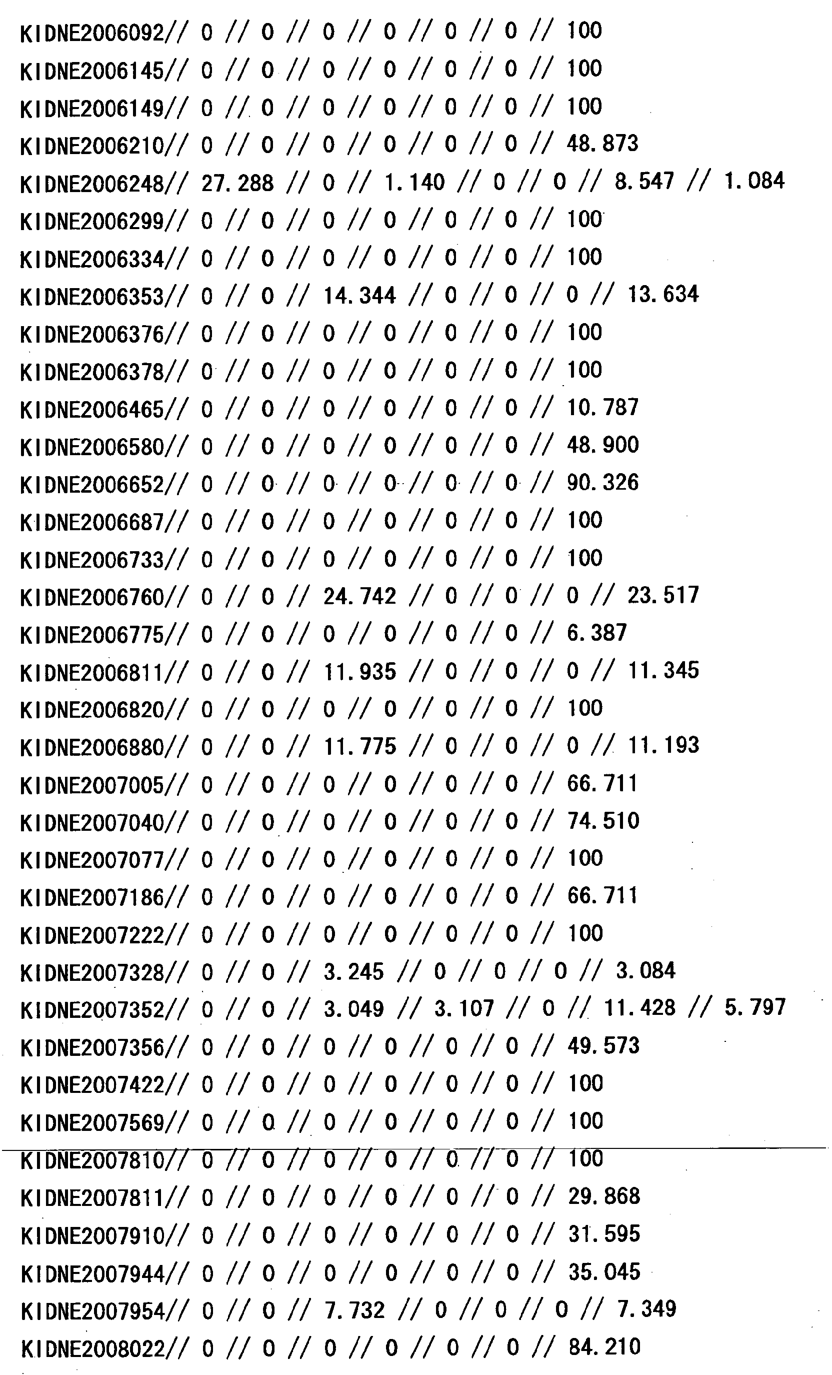

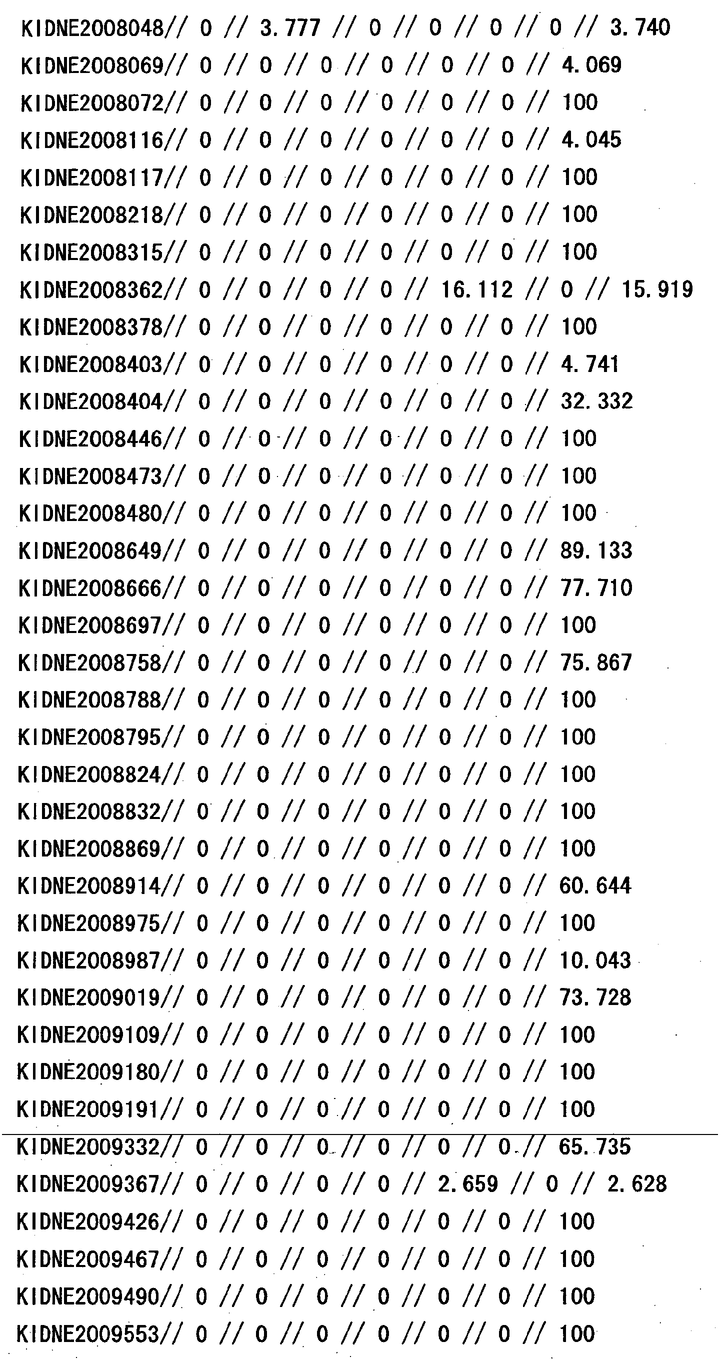

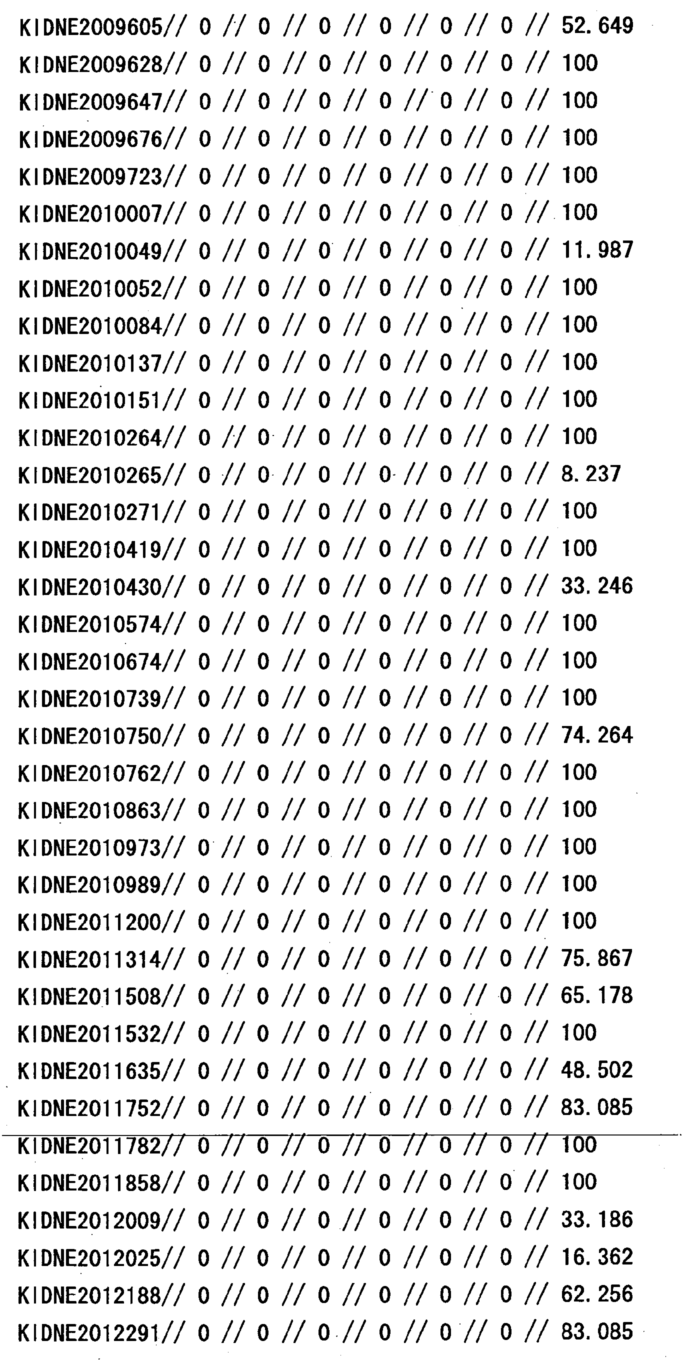

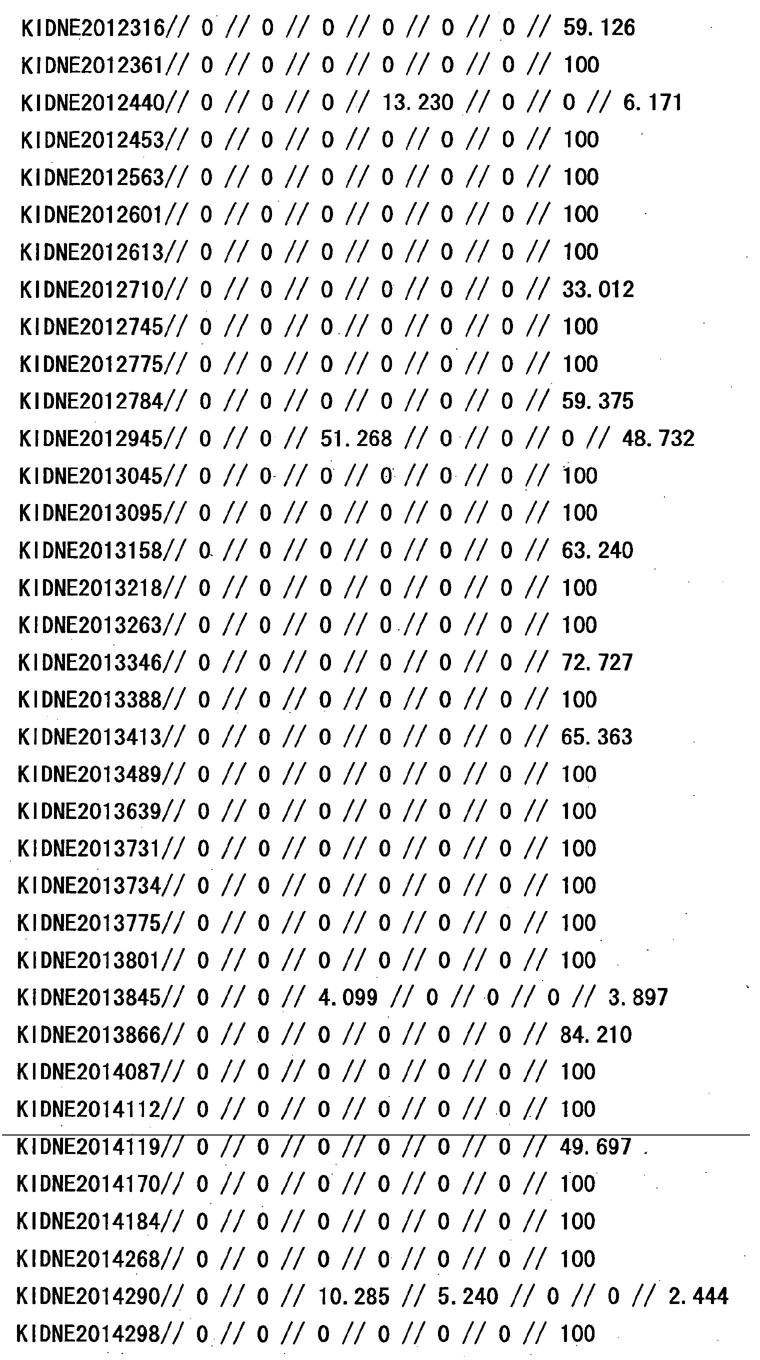

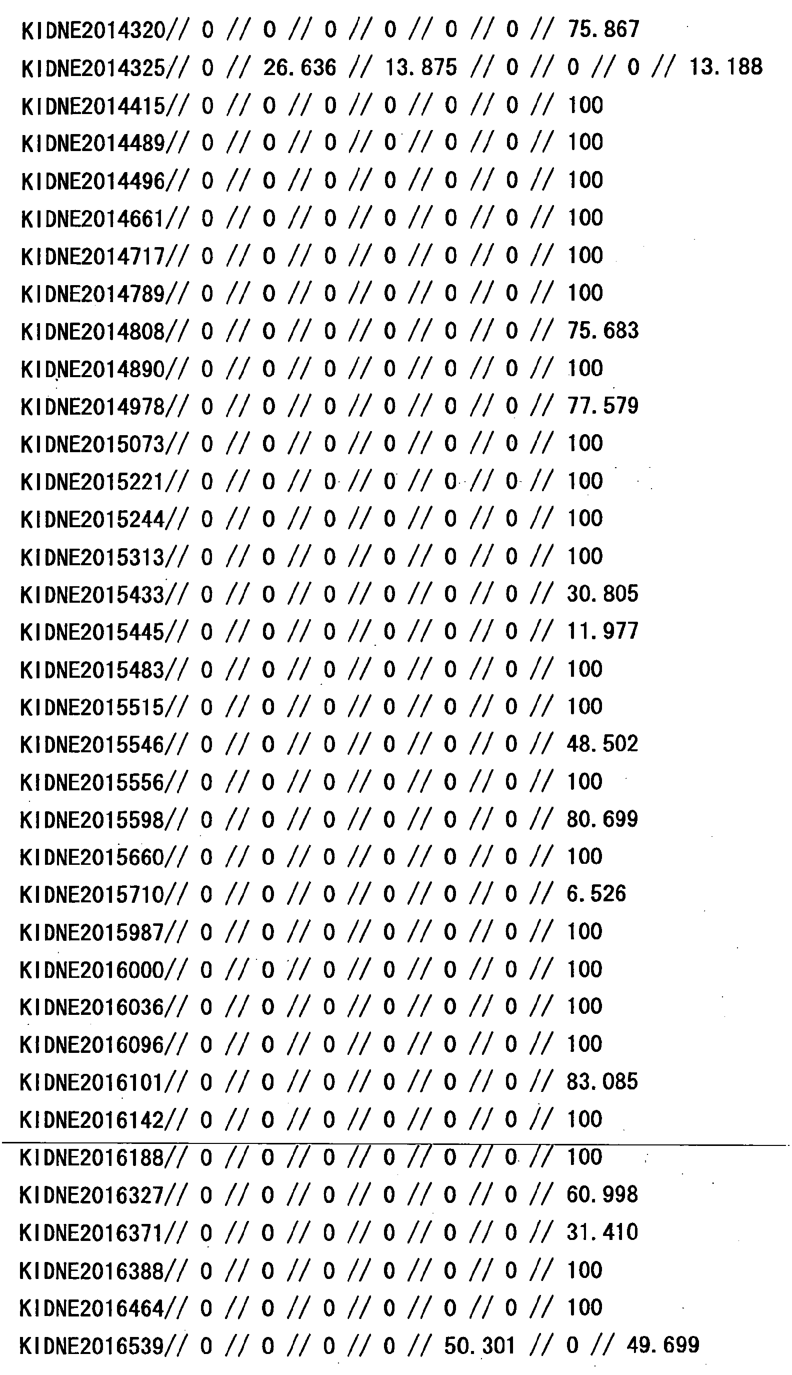

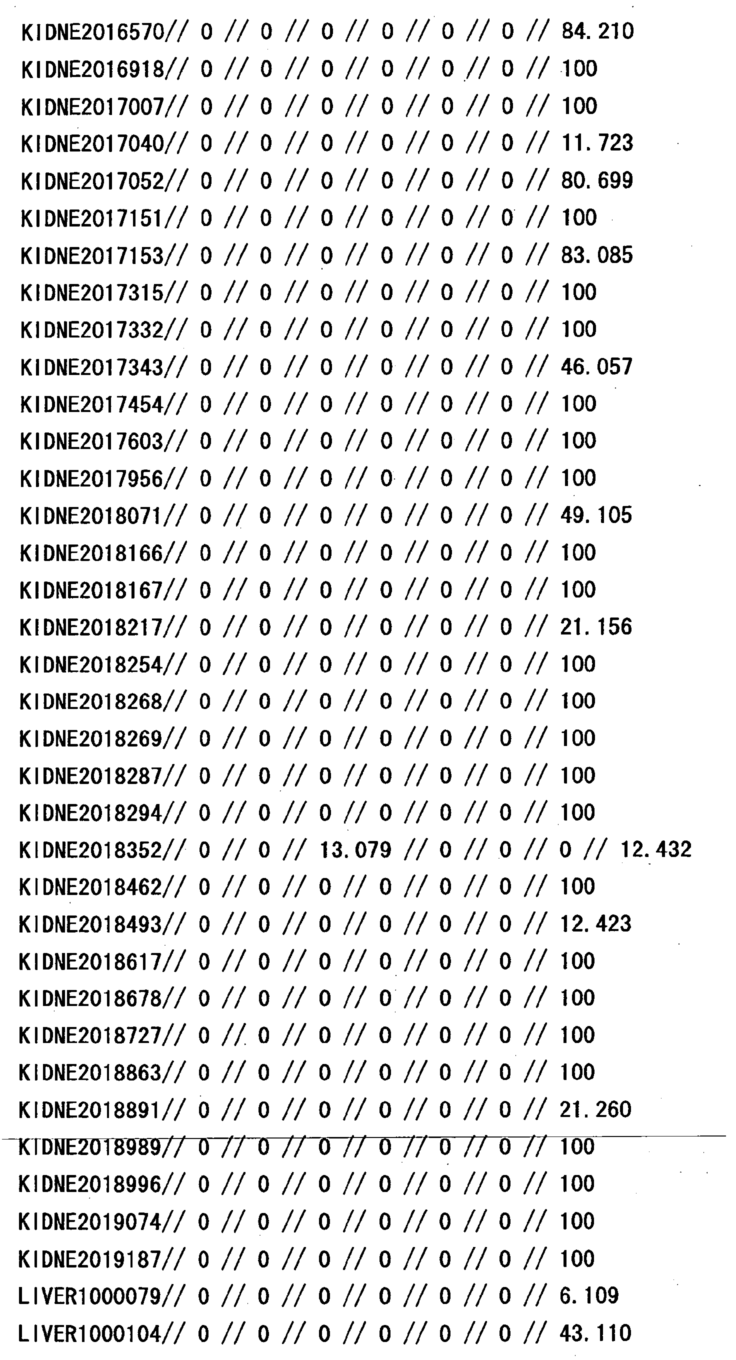

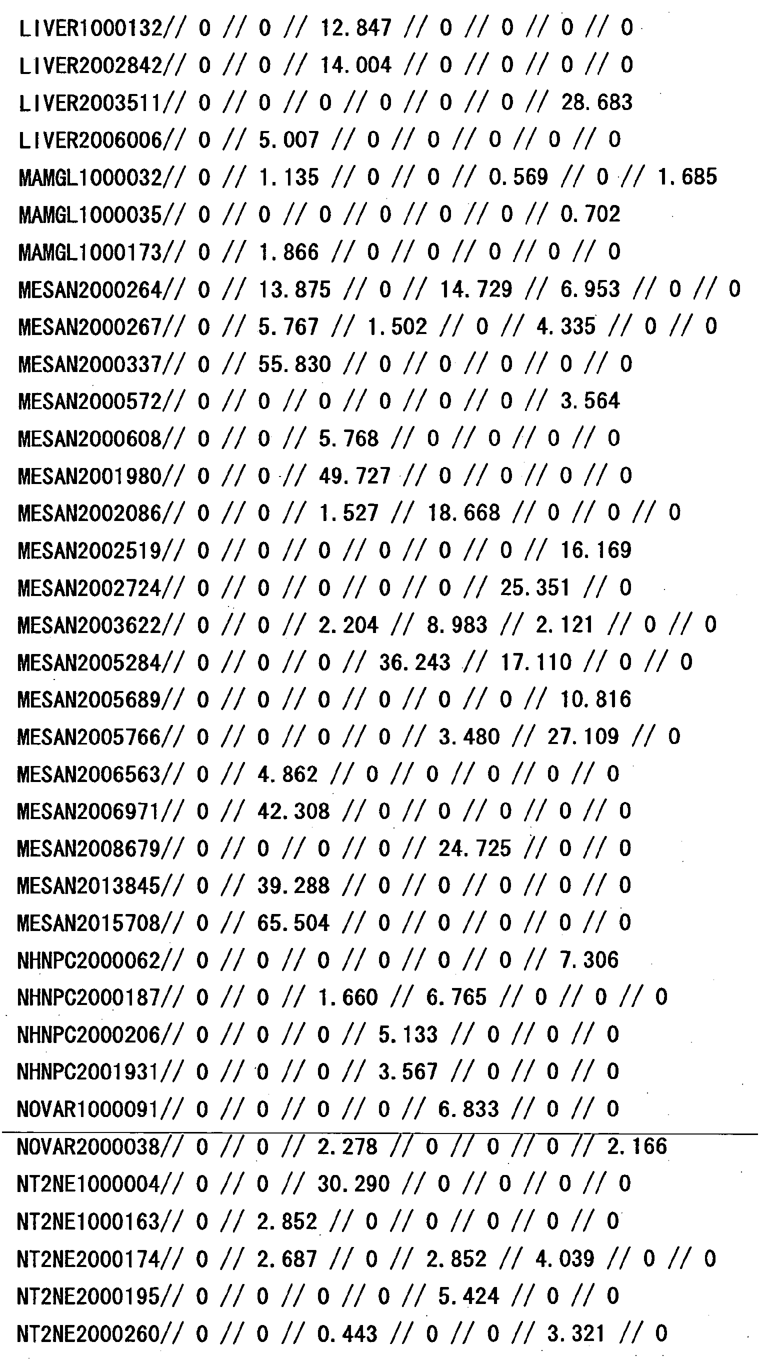

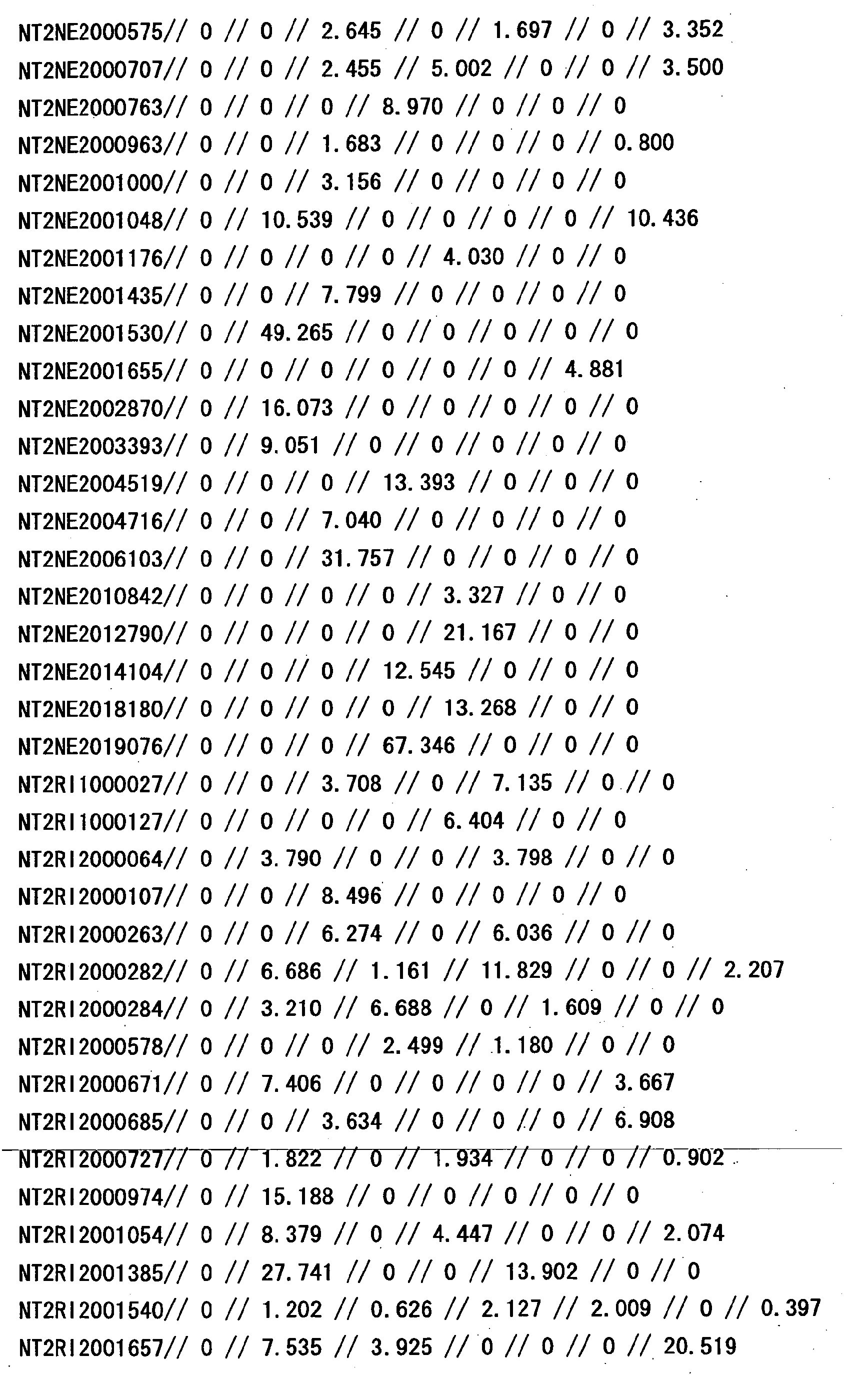

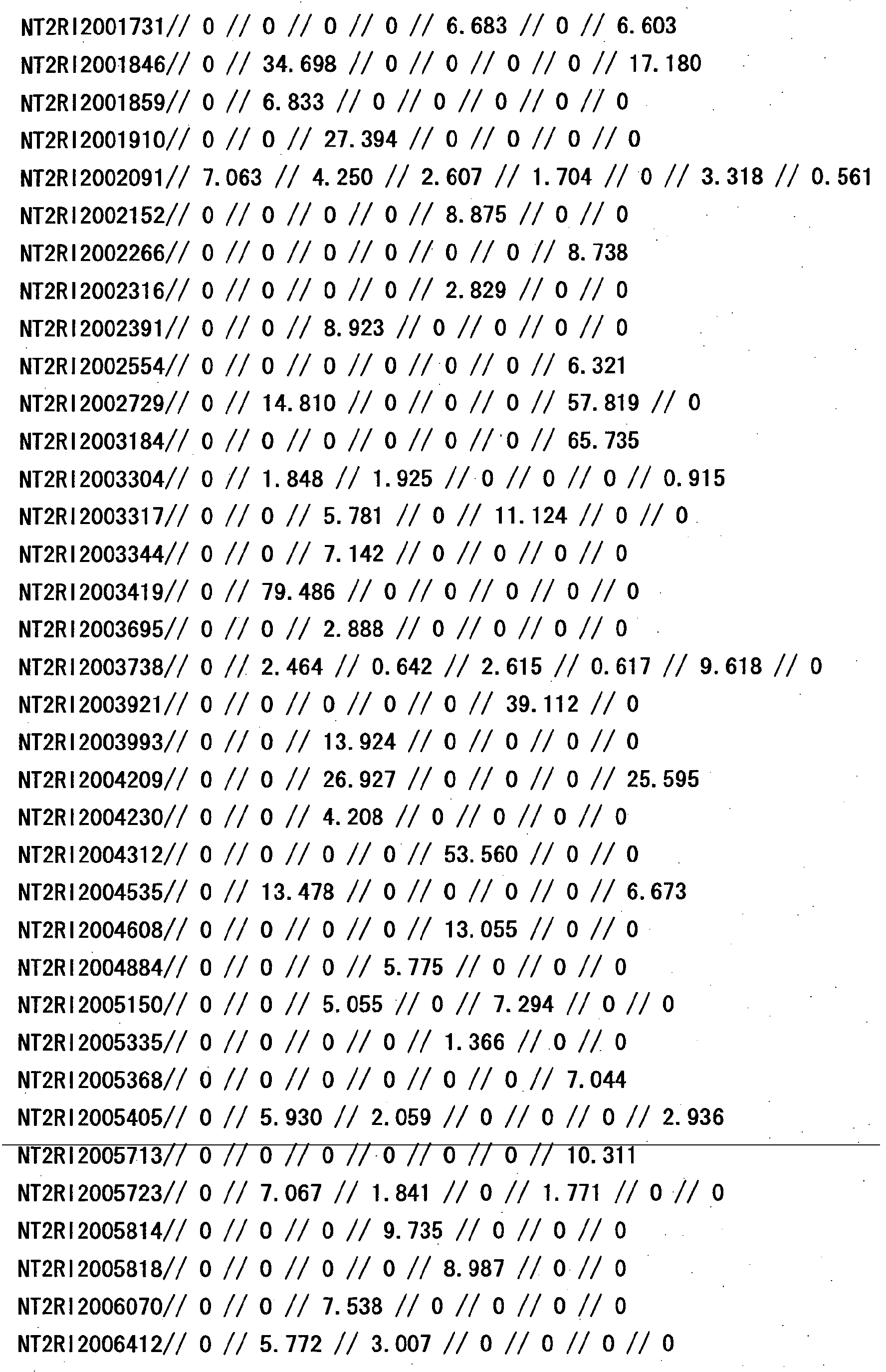

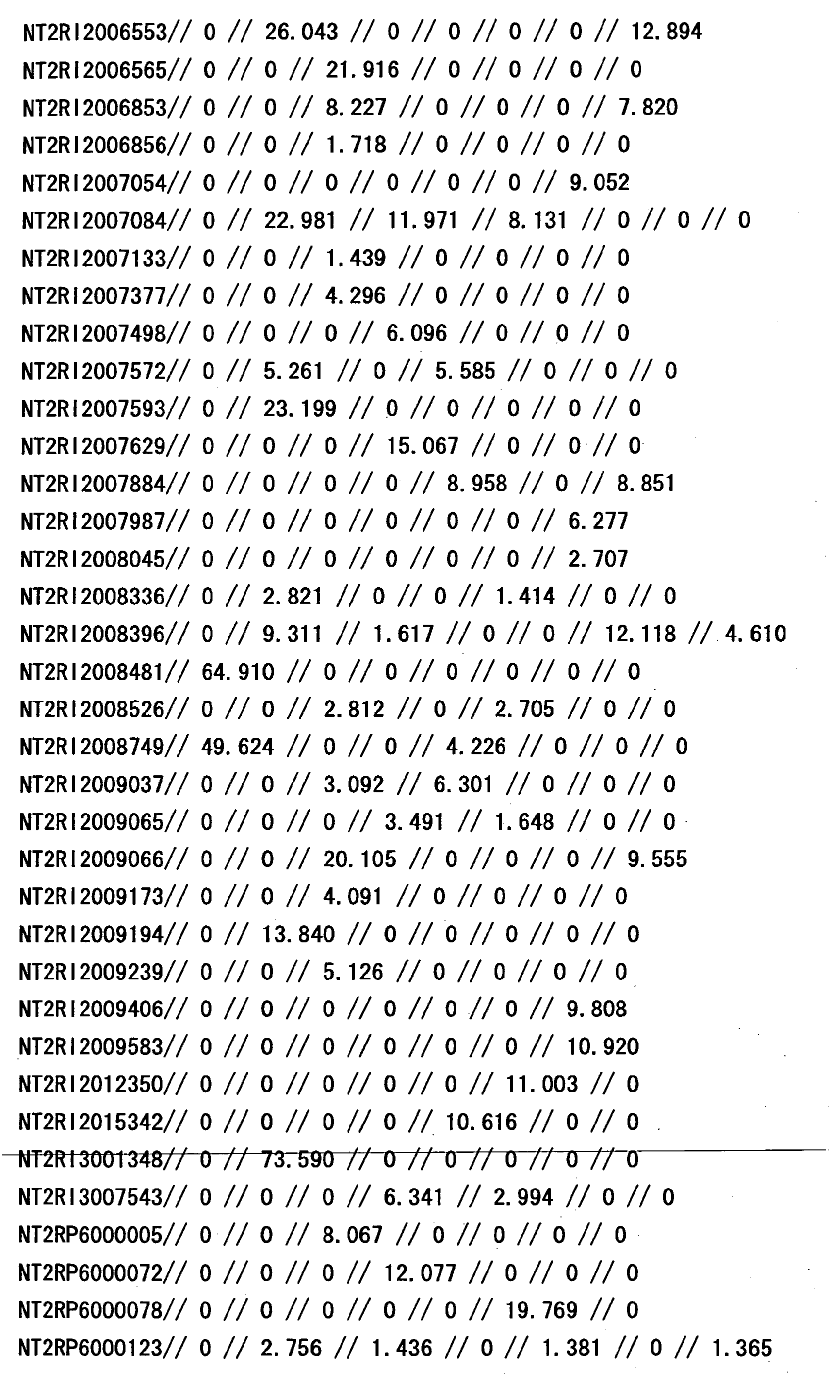

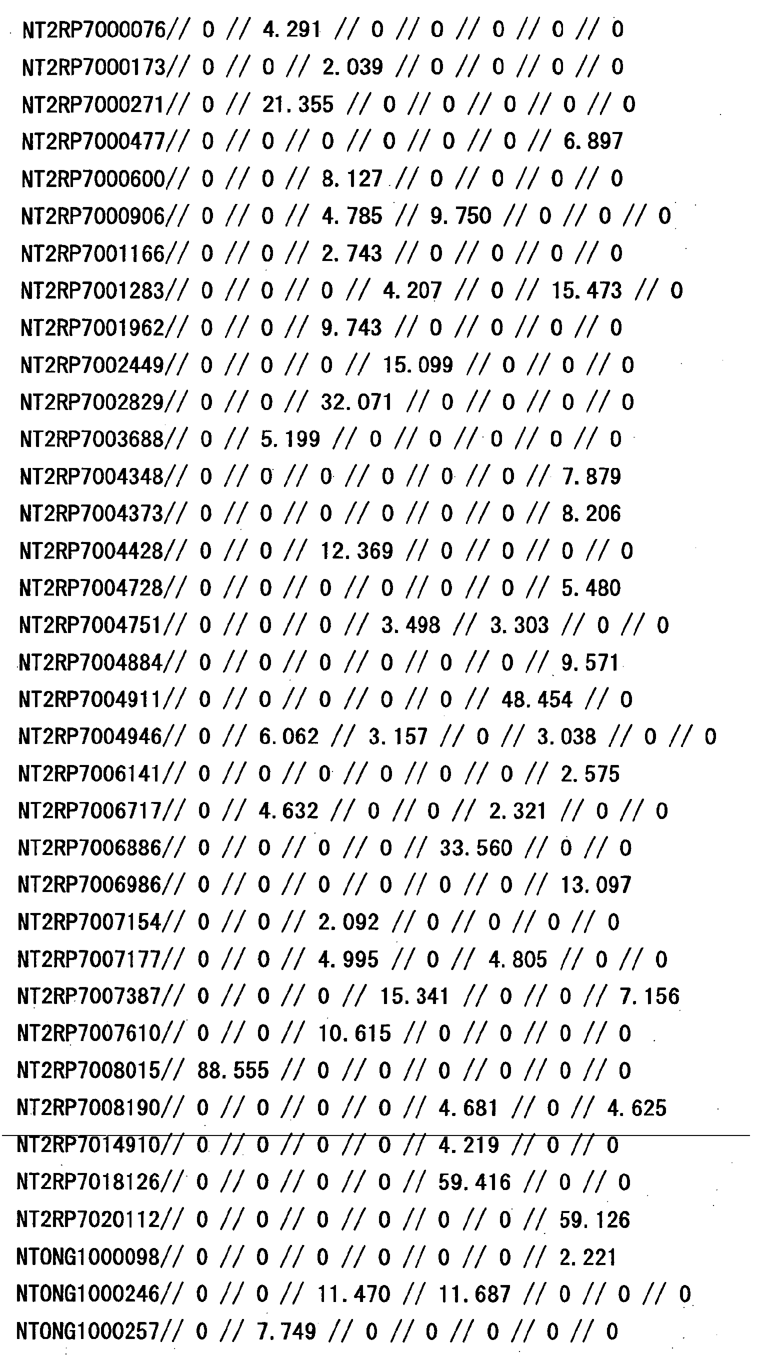

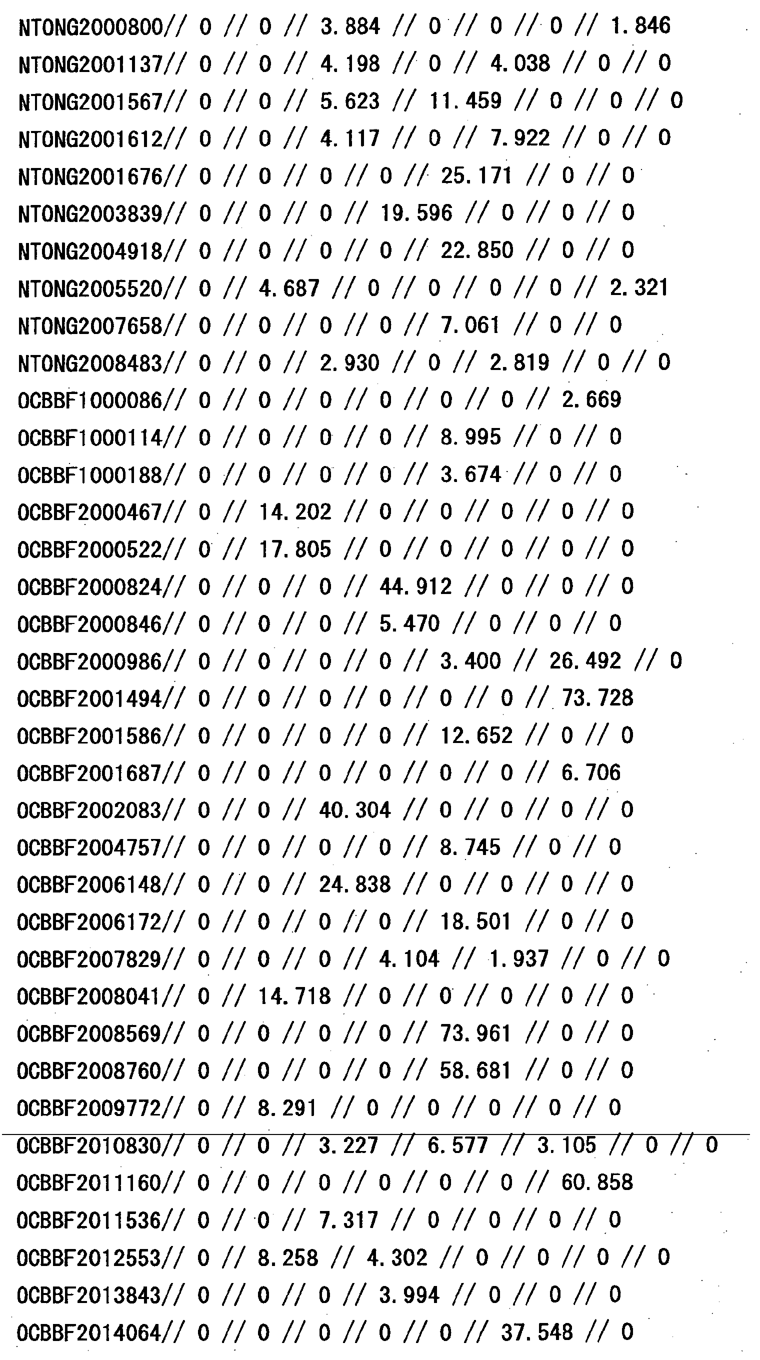

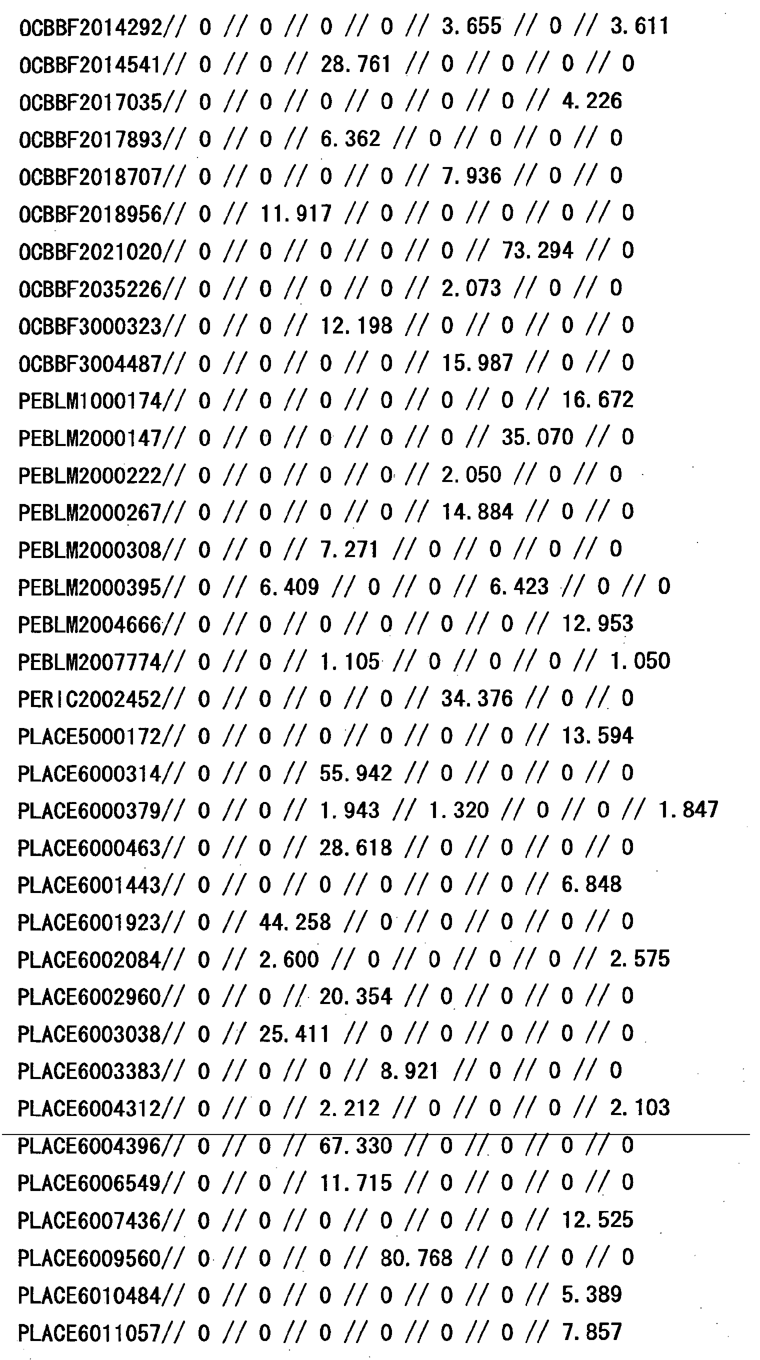

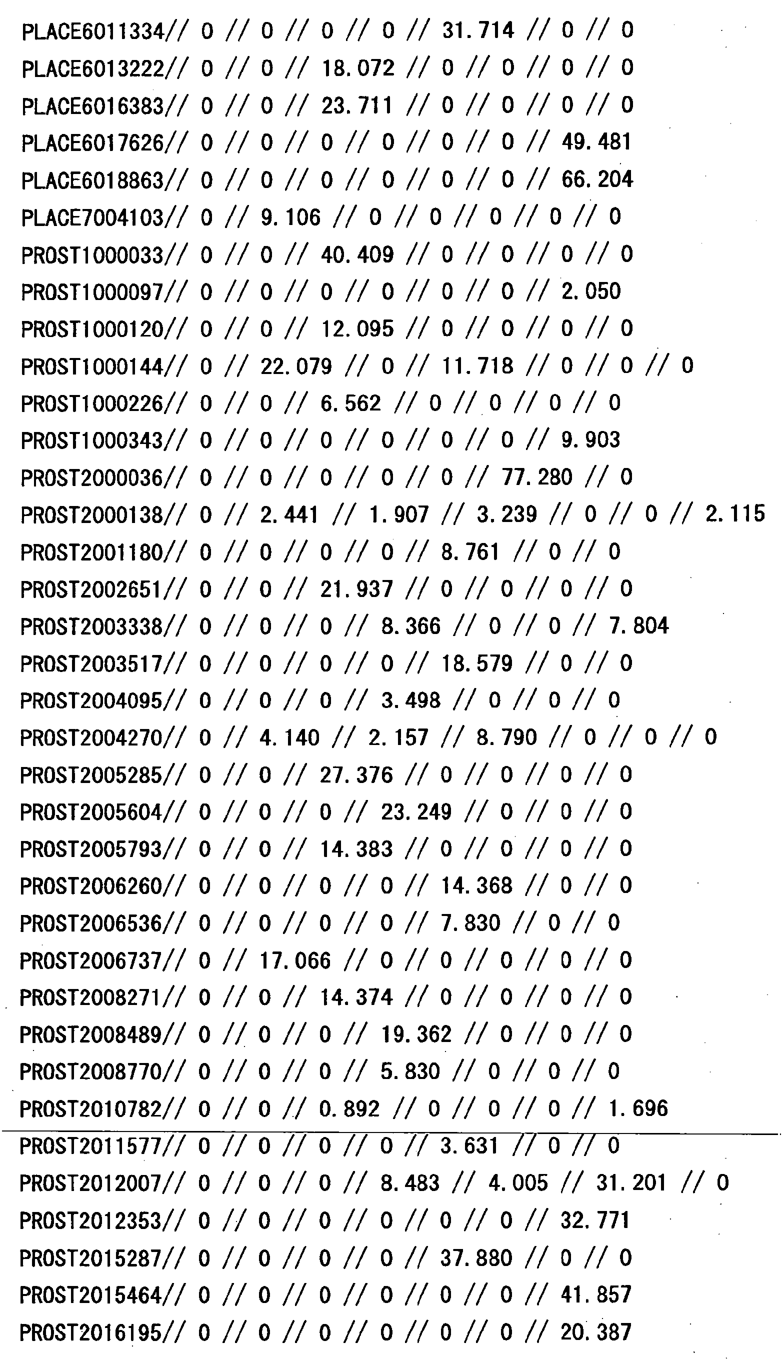

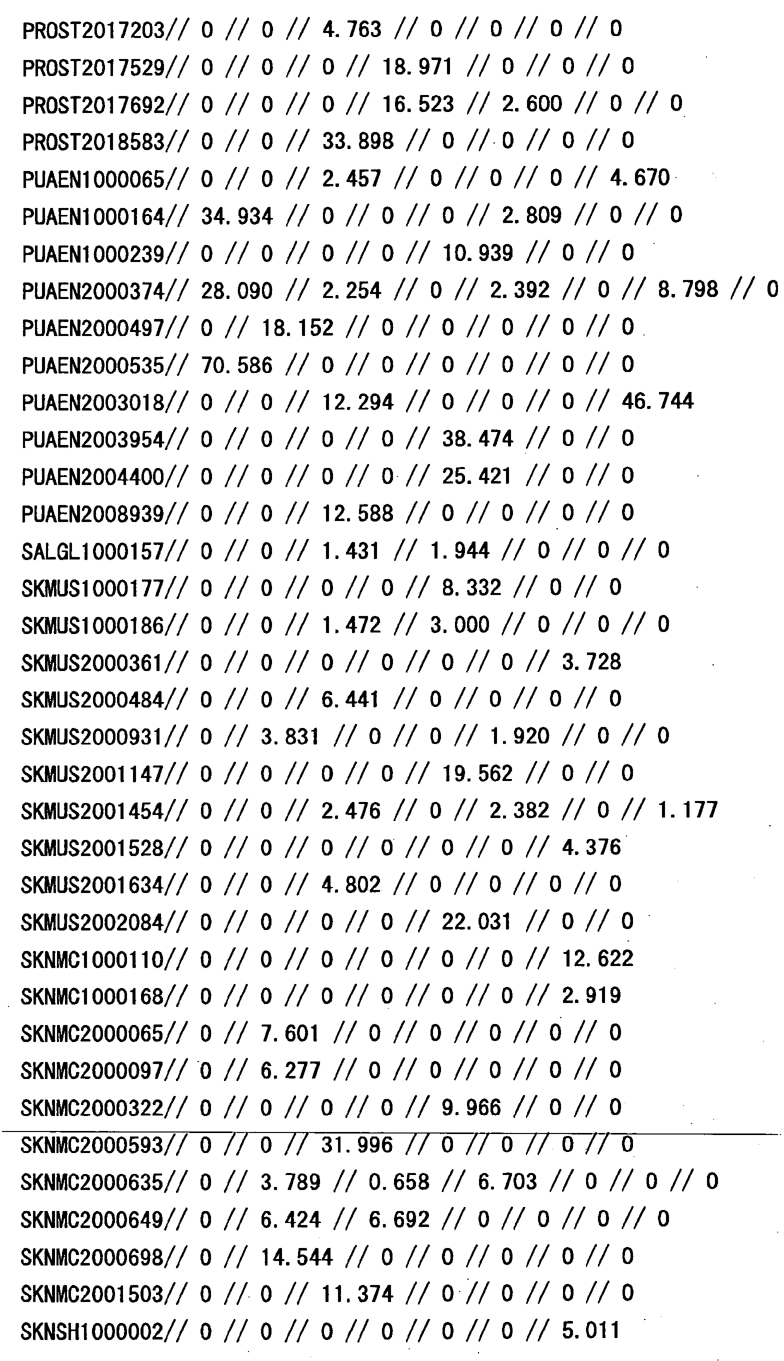

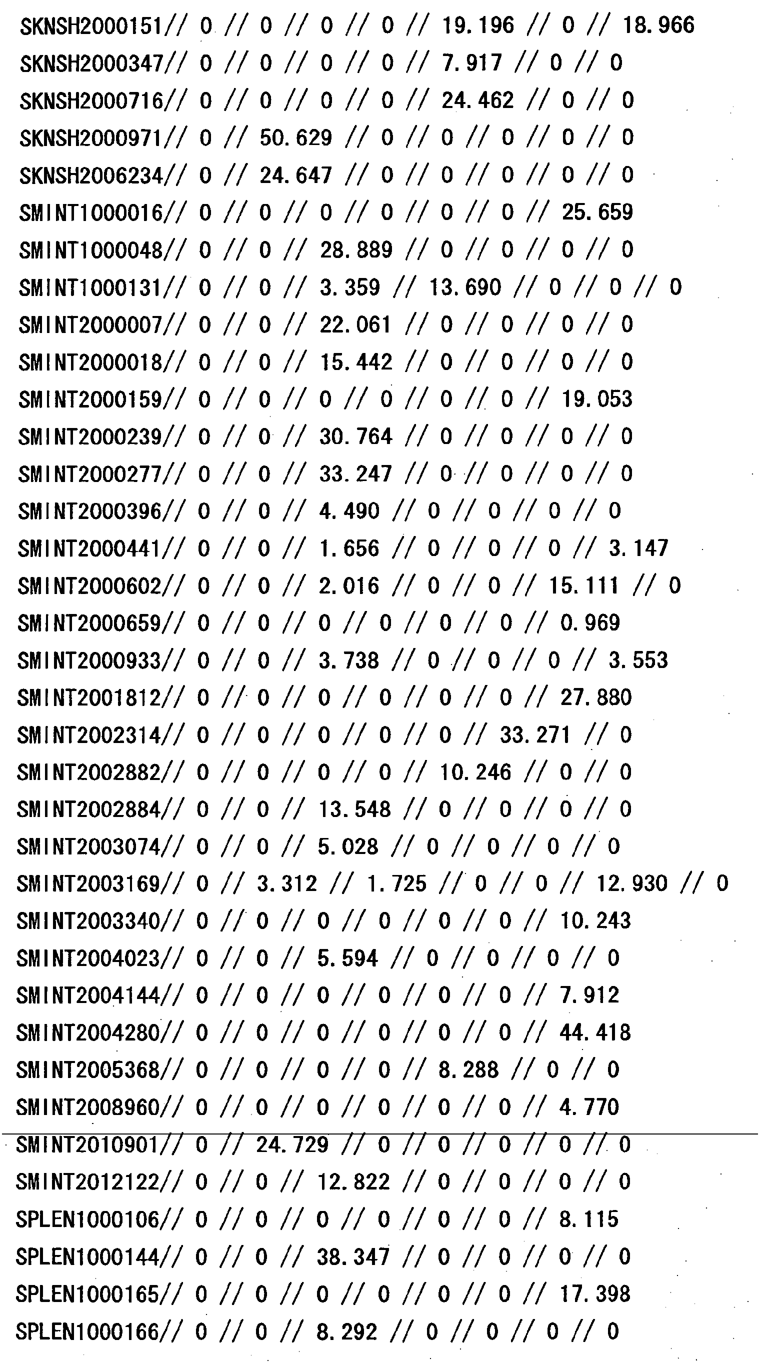

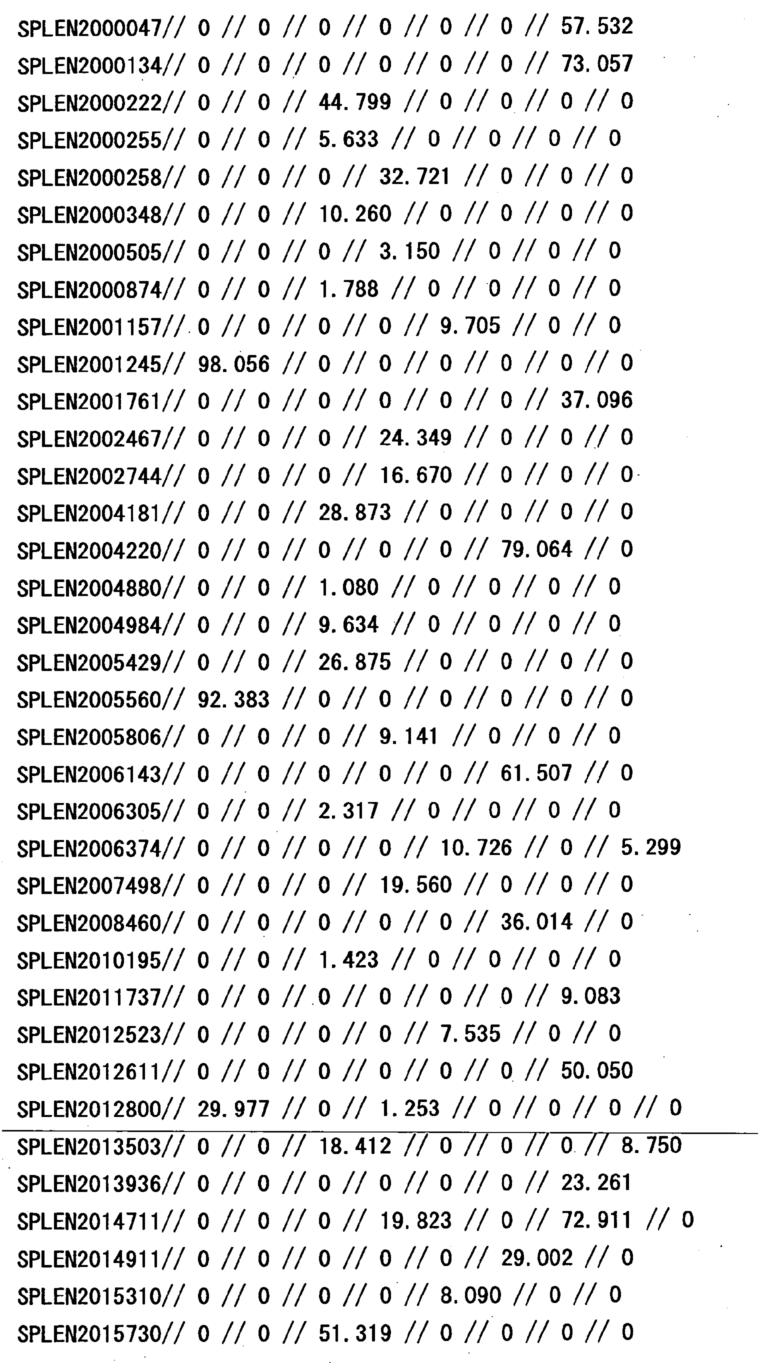

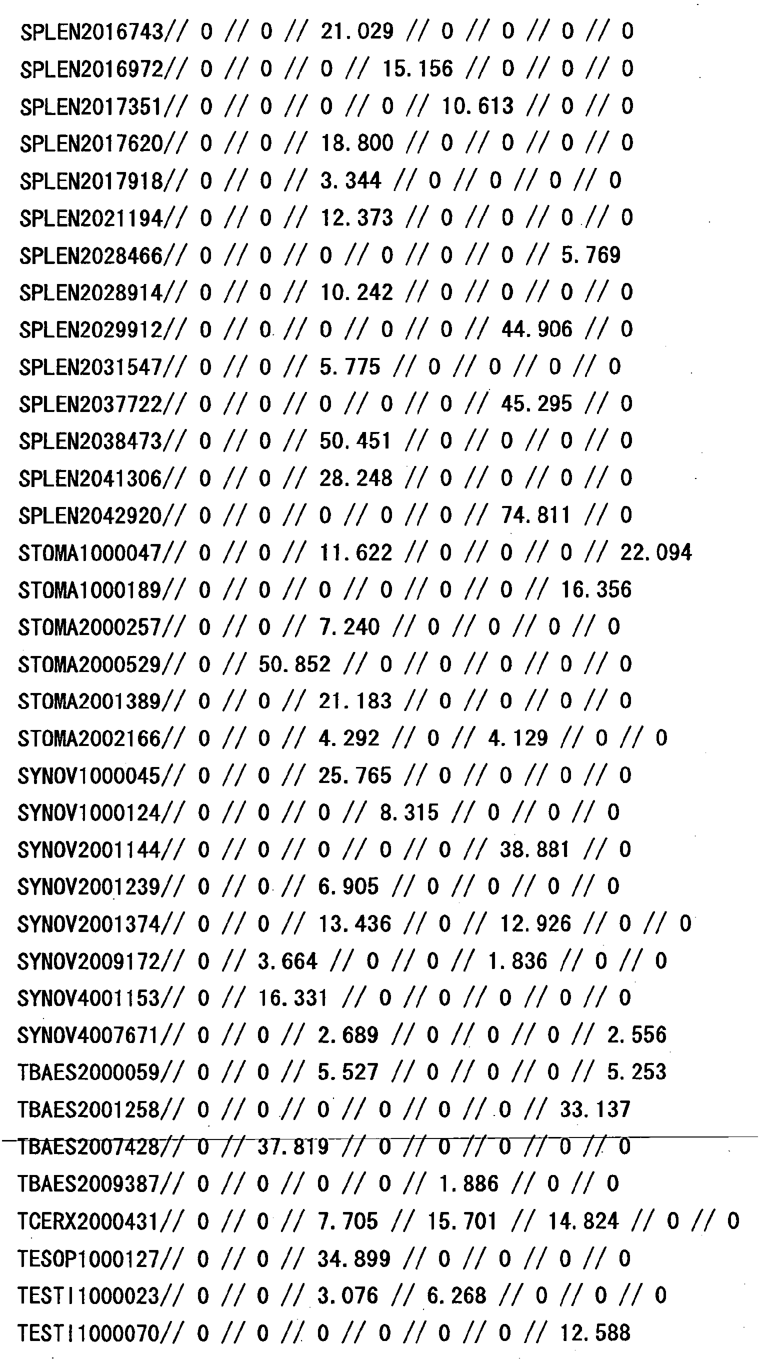

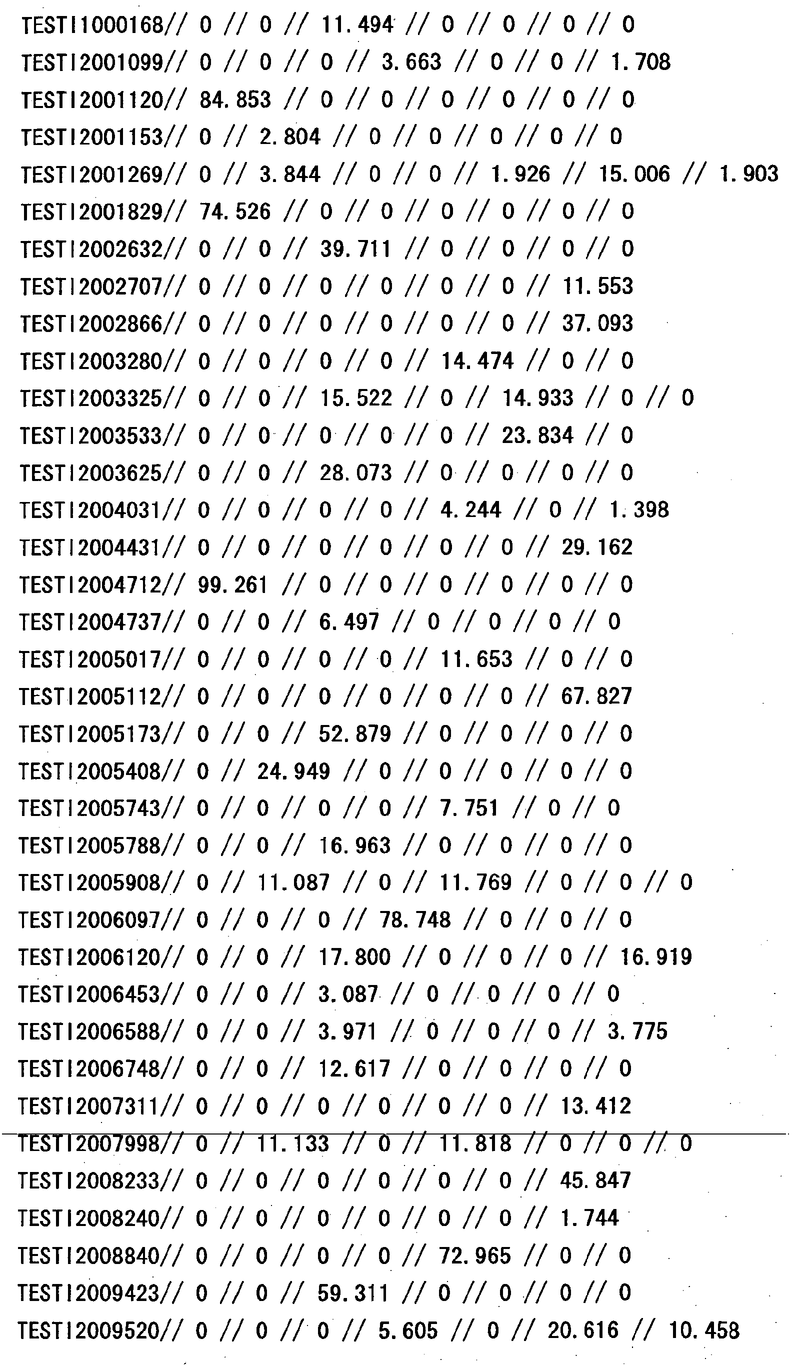

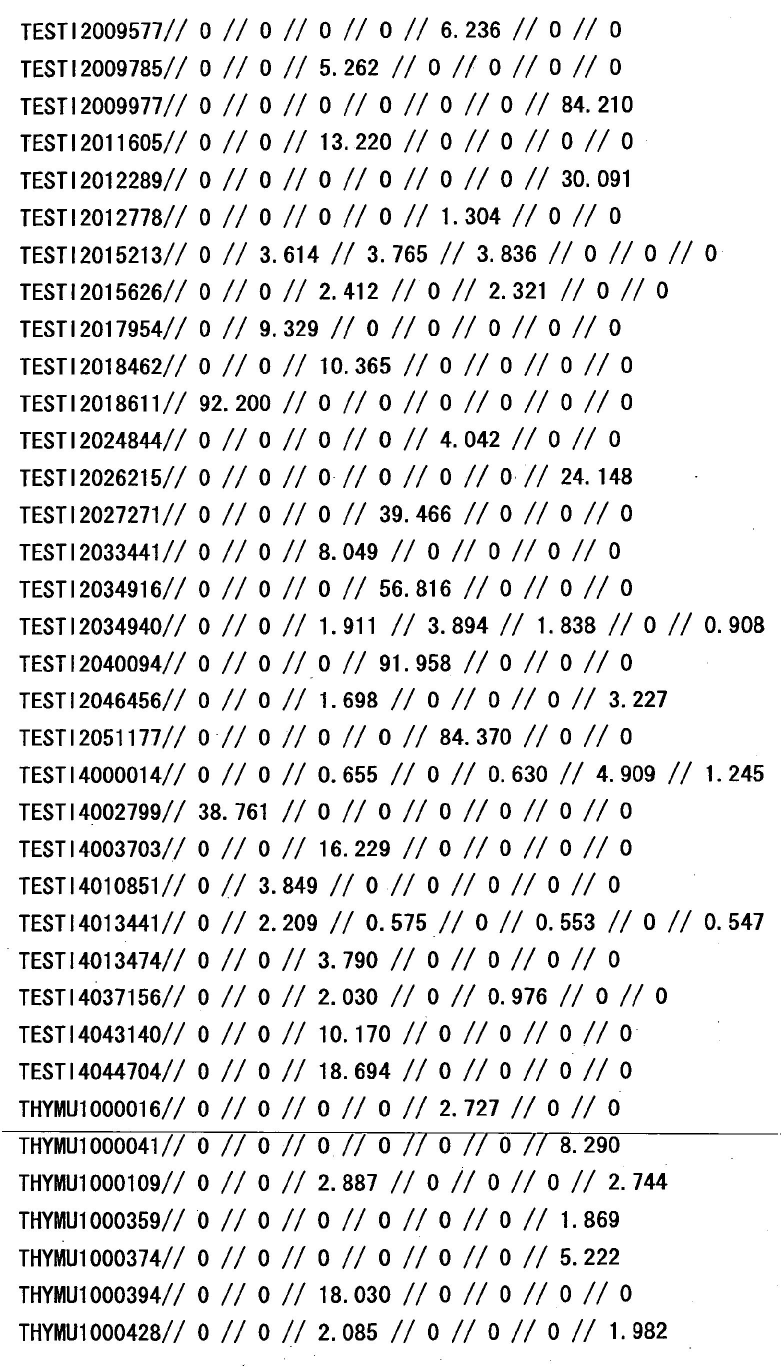

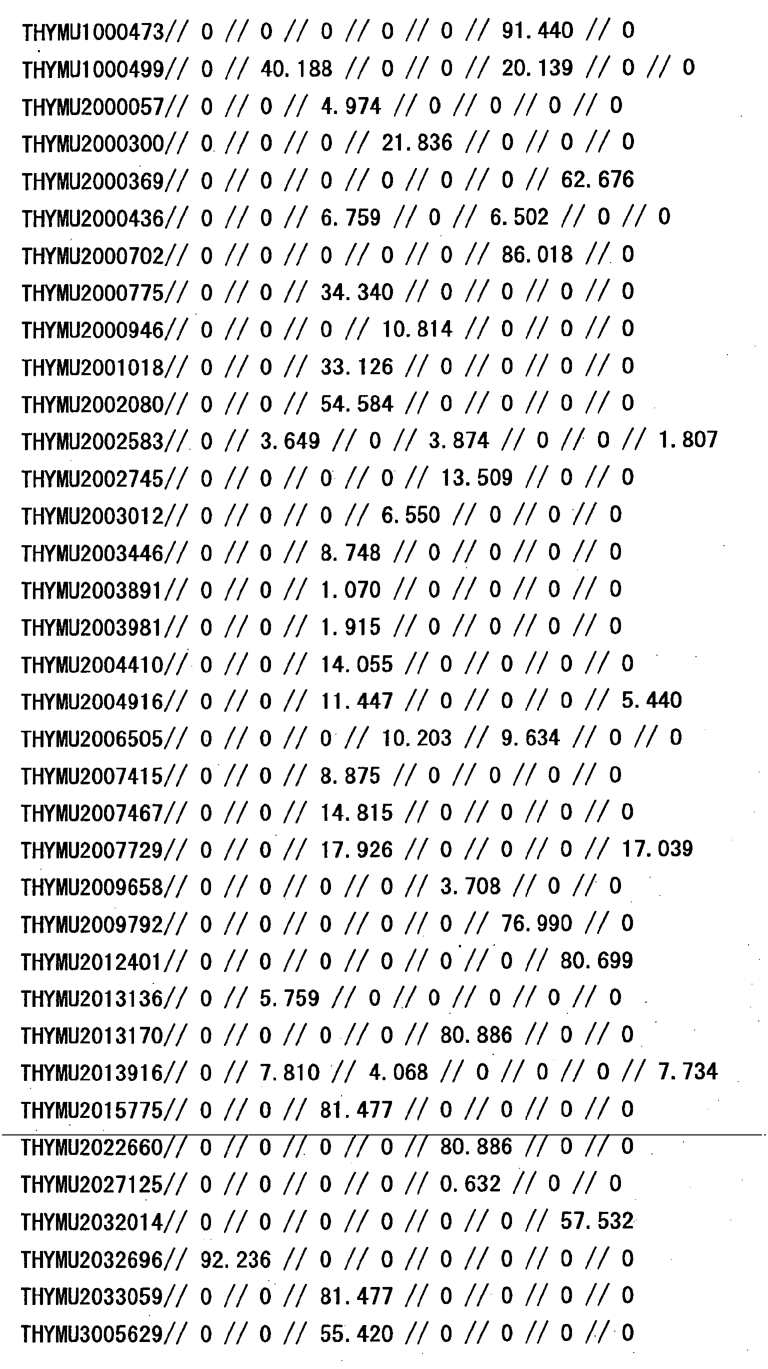

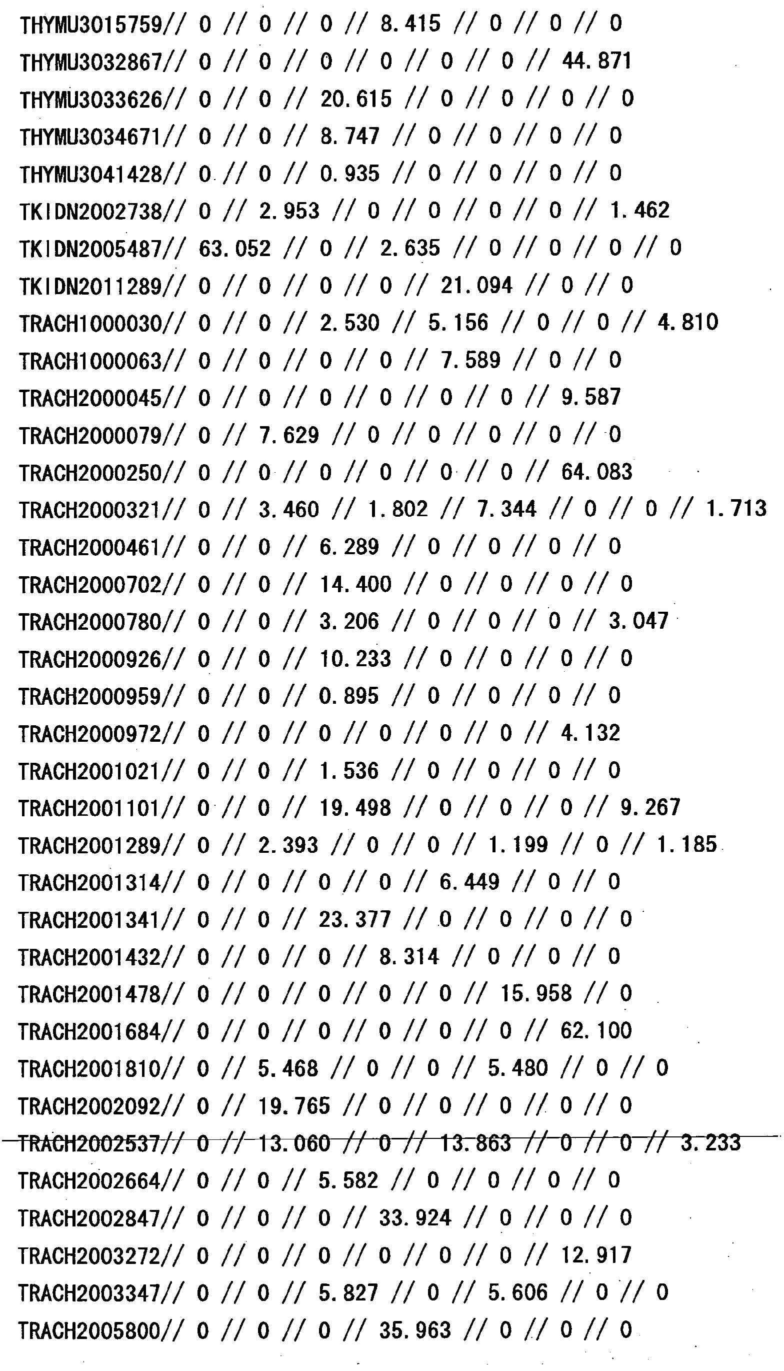

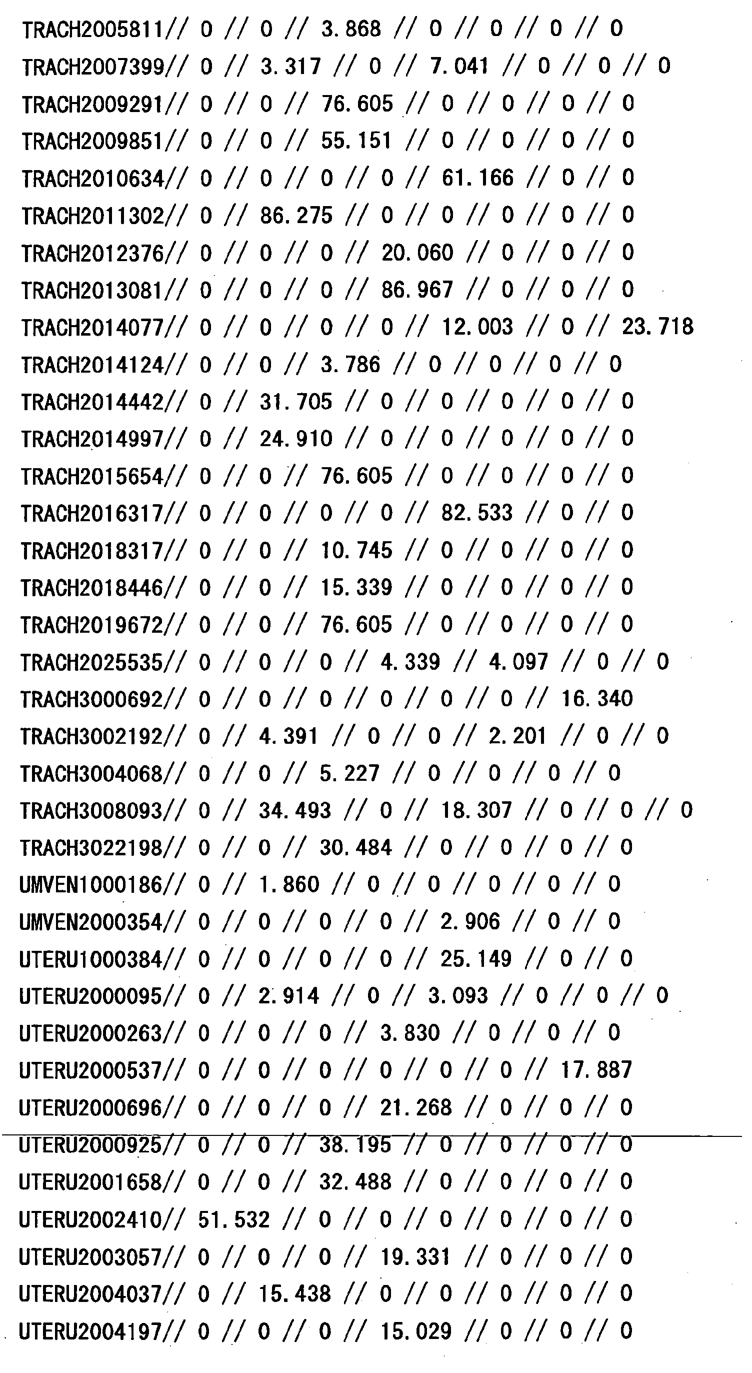

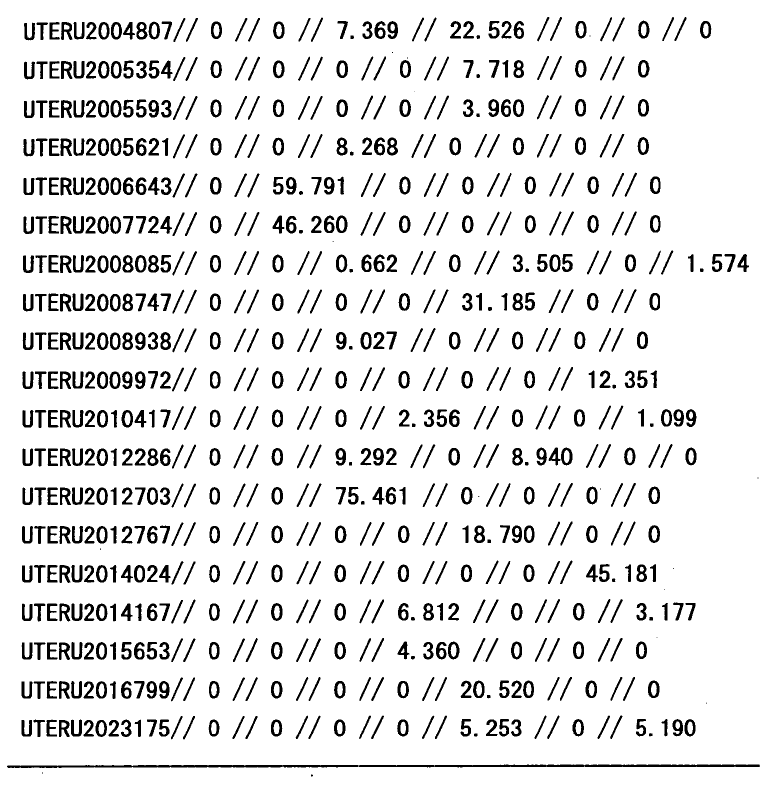

Genes whose expression levels were negligibly low in each of the above-described libraries are shown with their clone names below.
3NB691000018, 3NB691000085, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001034, 3NB692001040, 3NB692001123, 3NB692001267, 3NB692001339, 3NB692001349, 3NB692001366, 3NB692001408, 3NB692001433, 3NB692001471, 3NB692001496, 3NB692001501, 3NB692001507, 3NB692001519, 3NB692001520, 3NB692001528, 3NB692001538, 3NB692001548, 3NB692001557, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069,
ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260,
ADRGL2002392, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974,
ADRGL2008023, ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012179, AHMSC1000138, ASTRO1000009, ASTRO1000018, ASTRO1000029, ASTRO1000096, ASTRO2000011, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001107, ASTRO2001122, ASTRO2001227, ASTRO2001249, ASTRO2001480, ASTRO2002024, ASTRO2002035, ASTRO2002064,
ASTRO2002202, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920,
ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561,
ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285, BGGI12000022, BGGI12000054, BGGI12000067, BGGI12000161, BGGI12000544, BGGI12000684, BGGI12000693, BGGI12001002, BGGI12001075, BGGI12001241, BGGI12001247, BGGI12001714, BLADE1000176, BLADE2000181, BLADE2000256, BLADE2000340, BLADE2000389, BLADE2000463, BLADE2000492,
BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007666, BLADE2007722, BLADE2007735, BLADE2007744,
BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000169, BNGH41000177, BNGH41000190, BNGH41000216, BNGH41000235, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504,
BNGH42005968, BNGH42006135, BNGH42006226, BNGH42006234, BNGH42007037, BNGH42007366, BNGH42007460, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000166, BRACE1000169, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148,
BRACE2000183, BRACE2000229, BRACE2000232, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441, BRACE2000487, BRACE2000489, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000975, BRACE2000988, BRACE2001017, BRACE2001065, BRACE2001070, BRACE2001094, BRACE2001107, BRACE2001143, BRACE2001151,
BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001859, BRACE2001865, BRACE2001872, BRACE2001881, BRACE2001898, BRACE2001923, BRACE2001942, BRACE2001944,
BRACE2001954, BRACE2001972, BRACE2001973, BRACE2002026, BRACE2002034, BRACE2002050, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002695, BRACE2002707, BRACE2002736, BRACE2002752, BRACE2002755, BRACE2002762, BRACE2002772, BRACE2002792,
BRACE2002796, BRACE2002803, BRACE2002807, BRACE2002812, BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003845, BRACE2003847, BRACE2003848, BRACE2003873, BRACE2003885, BRACE2003887,
BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005719, BRACE2005731,
BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE2006084, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006264, BRACE2006274, BRACE2006281, BRACE2006298, BRACE2006319, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534,
BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006909, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527,
BRACE2007538, BRACE2007563, BRACE2007567, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008276, BRACE2008295, BRACE2008308, BRACE2008336, BRACE2008358, BRACE2008361,
BRACE2008399, BRACE2008401, BRACE2008402, BRACE2008410, BRACE2008420, BRACE2008443, BRACE2008473, BRACE2008480, BRACE2008481, BRACE2008488, BRACE2008500, BRACE2008553, BRACE2008594, BRACE2008604, BRACE2008615, BRACE2008622, BRACE2008629, BRACE2008653, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238,
BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813,
BRACE2010828, BRACE2010837, BRACE2010888, BRACE2010937, BRACE2010983, BRACE2011007, BRACE2011025, BRACE2011183, BRACE2011199, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257,
BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587,
BRACE2017720, BRACE2017844, BRACE2017872, BRACE2017929, BRACE2017934, BRACE2017985, BRACE2017992, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982,
BRACE2021014, BRACE2021143, BRACE2021148, BRACE2021245, BRACE2021254, BRACE2021264, BRACE2021344, BRACE2021433, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610,
BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849,
BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830,
BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873,
BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113,
BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004205, BRACE3004358, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499,
BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008.255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708,
BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020,
BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153,
BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537,
BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495,
BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734,
BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157,
BRAMY1000173, BRAMY2000021, BRAMY2000025, BRAMY2000052, BRAMY2000076, BRAMY2000086, BRAMY2000181, BRAMY2000196, BRAMY2000231, BRAMY2000277, BRAMY2000411, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037,
BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411,
BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2010068, BRAMY2010135, BRAMY2010145,
BRAMY2010171, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881,
BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771, BRAMY2016892, BRAMY2016938, BRAMY2016953,
BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276,
BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024073, BRAMY2024312, BRAMY2024374, BRAMY2024390, BRAMY2024416, BRAMY2024449,
BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717,
BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003,
BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484,
BRAMY2038516, BRAMY2038846, BRAMY2038887, BRAMY2038904, BRAMY2039287, BRAMY2039300, BRAMY2039314, BRAMY2039341, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299,
BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004672,
BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466; BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652,
BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000488, BRAWH2000494, BRAWH2000500, BRAWH2000503, BRAWH2000554,
BRAWH2000595, BRAWH2000643, BRAWH2000651, BRAWH2000682, BRAWH2000686, BRAWH2000752, BRAWH2000839, BRAWH2000859, BRAWH2000866, BRAWH2000892, BRAWH2000901, BRAWH2000924, BRAWH2000926, BRAWH2000984, BRAWH2001019, BRAWH2001037, BRAWH2001052, BRAWH2001103, BRAWH2001129, BRAWH2001137, BRAWH2001141, BRAWH2001159, BRAWH2001171, BRAWH2001186, BRAWH2001203, BRAWH2001238, BRAWH2001255, BRAWH2001321, BRAWH2001355, BRAWH2001378, BRAWH2001395, BRAWH2001406, BRAWH2001412, BRAWH2001418, BRAWH2001436, BRAWH2001438, BRAWH2001439, BRAWH2001459, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686,
BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811; BRAWH2002861, BRAWH2002911, BRAWH2002963, BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779,
BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008219, BRAWH2008292, BRAWH2008297, BRAWH2008366, BRAWH2008380,
BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762,
BRAWH2011795, BRAWH2011796, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015375, BRAWH2015385, BRAWH2015595, BRAWH2015728, BRAWH2015853, BRAWH2015866,
BRAWH2016028, BRAWH2016106, BRAWH2016160, BRAWH2016166, BRAWH2016209, BRAWH2016221, BRAWH2016223, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017685, BRAWH2017913, BRAWH2018206, BRAWH2018267, BRAWH2018282, BRAWH2018317, BRAWH2018506, BRAWH2018514, BRAWH2018527, BRAWH2018601, BRAWH2018745, BRAWH2018875, BRAWH2019053, BRAWH2019055, BRAWH2019198, BRAWH3000078, BRAWH3000088, BRAWH3000100,
BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300,
BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197,
BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023274, BRAWH3023421, BRAWH3024231,
BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114,
BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000763, BRCAN2000832, BRCAN2000923, BRCAN2001223, BRCAN2001866, BRCAN2002416, BRCAN2002473, BRCAN2002562,
BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436 , BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376,
BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653,
BRCAN2019773, BRCAN2019907, BRCAN2020234, BRCAN2020331, BRCAN2020412, BRCAN2020467, BRCAN2020710, BRCAN2020880, BRCAN2021024, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087,
BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769,
BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934,
BRCOC2020122, BRCOC2020142, BRHIP1000058, BRHIP1000072, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355,
BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491,
BRHIP2011508, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553,
BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021929, BRHIP2022006, BRHIP2022054, BRHIP2022221, BRHIP2022228, BRHIP2022298, BRHIP2022326, BRHIP2022467, BRHIP2022708,
BRHIP2022986, BRHIP2023071, BRHIP2023229, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176,
BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710,
BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181,
BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046,
BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566,
BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395,
BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768,
BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457,
BRSTN2008475, BRSTN2008930, BRSTN2009247,BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063,
BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831,
BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129,
BRTHA2012183, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985,
BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517,
BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490,
BRTHA3003704, BRTHA3003736, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010717, BRTHA3011149, BRTHA3011187, BRTHA3011194, BRTHA3011229, BRTHA3011265, BRTHA3011361, BRTHA3011510, BRTHA3011892,
BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507,
BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318,
COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000180, CTONG1000181, CTONG1000218, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034,
CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000480, CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417, CTONG2002418,
CTONG2002520, CTONG2002558, CTONG2002590, CTONG2002709, CTONG2002721, CTONG2002744, CTONG2002766, CTONG2002803, CTONG2002816, CTONG2002820, CTONG2002832, CTONG2002841, CTONG2002965, CTONG2002999, CTONG2003028, CTONG2003094, CTONG2003100, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003945, CTONG2004000, CTONG2004062, CTONG2004115, CTONG2004126, CTONG2004264, CTONG2004305, CTONG2004397, CTONG2004423, CTONG2004454, CTONG2004669, CTONG2004716, CTONG2004912, CTONG2004941, CTONG2004948, CTONG2005028,
CTONG2005049, CTONG2005110, CTONG2005145, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006709, CTONG2006798, CTONG2006836, CTONG2006942, CTONG2006964, CTONG2007007, CTONG2007009, CTONG2007091, CTONG2007104, CTONG2007168, CTONG2007175, CTONG2007259, CTONG2007272, CTONG2007293, CTONG2007297, CTONG2007399, CTONG2007400, CTONG2007417,
CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008321, CTONG2008343, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604,
CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010649, CTONG2010652, CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012050, CTONG2012077, CTONG2012101, CTONG2012123, CTONG2012230, CTONG2012401, CTONG2012422,
CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012745, CTONG2012758, CTONG2012843, CTONG2012879, CTONG2012901, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946,
CTONG2014954, CTONG2014959, CTONG2014966, CTONG2014995, CTONG2015204, CTONG2015316, CTONG2015330, CTONG2015345, CTONG2015358, CTONG2015434, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016408, CTONG2016499, CTONG2016505, CTONG2016559, CTONG2016658, CTONG2016775, CTONG2016797, CTONG2016846, CTONG2016904, CTONG2016942, CTONG2016953, CTONG2017021, CTONG2017094, CTONG2017292, CTONG2017409, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062,
CTONG2018069, CTONG2018147, CTONG2018211, CTONG2018217, CTONG2018279, CTONG2018343, CTONG2018379, CTONG2018383, CTONG2018413, CTONG2018483, CTONG2018629, CTONG2018632, CTONG2018637, CTONG2018652, CTONG2018653, CTONG2018655, CTONG2018735, CTONG2018739, CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020522, CTONG2020560, CTONG2020582, CTONG2020638, CTONG2020806, CTONG2020831, CTONG2020974,
CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127,
CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258,
CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002182, D3OST2002223, D3OST2002262, D3OST2002272, D3OST2002417, D3OST2002436, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000507, D9OST2000278, D9OST2000440, D9OST2000869 D9OST2001319,
D9OST2001433, D9OST2002397 D9OST2003106, D9OST2003108 D9OST2003137 D9OST2003397 D9OST2003580 D9OST2003594 D9OST2003762 D9OST2003791, D9OST2003989 D9OST2004018, D9OST2004073 D9OST2004417 DFNES1000003, DFNES1000083, DFNES1000107, DFNES1000185, DFNES2000143, DFNES2000146, DFNES2000212, DFNES2000292, DFNES2000335, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002550, DFNES2002588, DFNES2002817, DFNES2002966, DFNES2003081, DFNES2003192, DFNES2003255, DFNES2003742, DFNES2004346,
DFNES2004354, DFNES2004383, DFNES2004608, DFNES2004684, DFNES2005266, DFNES2005527, DFNES2005540, DFNES2005690, DFNES2005779, DFNES2005875, DFNES2006346, DFNES2006944, DFNES2007113, DFNES2007299, DFNES2007332, DFNES2007634, DFNES2007641, DFNES2007757, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000024, FCBBF1000025, FCBBF1000038, FCBBF1000053, FCBBF1000061, FCBBF1000063, FCBBF1000069, FCBBF1000077, FCBBF1000115, FCBBF1000117, FCBBF1000118, FCBBF1000125, FCBBF1000131, FCBBF1000139, FCBBF1000155, FCBBF1000157, FCBBF1000-171, FCBBF1000174,
FCBBF1000197, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000509, FCBBF1000546, FCBBF1000550, FCBBF1000556, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000087, FCBBF2000094, FCBBF2000105,
FCBBF2000123, FCBBF2000195, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370,
FCBBF2002541, FCBBF2002626, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302,
FCBBF2007556, FCBBF2007610, FCBBF2007633, FCBBF2007840, FCBBF3000102, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925,
FCBBF3003216, FCBBF3003305, FCBBF3003373, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007855, FCBBF3007859, FCBBF3007860, FCBBF3008073, FCBBF3008100, FCBBF3008153, FCBBF3008159, FCBBF3008251,
FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889,
FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016178, FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288,
FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894,
FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202,
FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000253, FEBRA2000282, FEBRA2000286,
FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000909,
FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001828, FEBRA2001867, FEBRA2001914, FEBRA2001974,
FEBRA2001989, FEBRA2001990, FEBRA2002009, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002933, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436,
FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003833, FEBRA2003897, FEBRA2003907, FEBRA2004023, FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004237, FEBRA2004268, FEBRA2004293, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079,
FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701, FEBRA2005726, FEBRA2005734, FEBRA2005787, FEBRA2005788, FEBRA2005824, FEBRA2005995, FEBRA2005998, FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006379, FEBRA2006396, FEBRA2006401, FEBRA2006409, FEBRA2006418, FEBRA2006427, FEBRA2006476, FEBRA2006491, FEBRA2006519, FEBRA2006521, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890,
FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007818, FEBRA2007823, FEBRA2007900, FEBRA2007901, FEBRA2007931, FEBRA2007957, FEBRA2008009, FEBRA2008086, FEBRA2008134, FEBRA2008135, FEBRA2008159, FEBRA2008165, FEBRA2008172, FEBRA2008201, FEBRA2008210, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542,
FEBRA2008583, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746,
FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200,
FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255,
FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366,
FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154,
HCASM2008536, HCASM2009405, HCASM2009424, HCASM2009463, HCASM2009588, HCHON1000015, HCHON1000131, HCHON1000142, HCHON1000166, HCHON1000176, HCHON2000028, HCHON2000038, HCHON2000062, HCHON2000100, HCHON2000156, HCHON2000199, HCHON2000212, HCHON2000271, HCHON2000295, HCHON2000315, HCHON2000322, HCHON2000323, HCHON2000344, HCHON2000373, HCHON2000395, HCHON2000417, HCHON2000418, HCHON2000473, HCHON2000503, HCHON2000660, HCHON2000663, HCHON2000673, HCHON2000676, HCHON2000698, HCHON2000699, HCHON2000705, HCHON2000743, HCHON2000751, HCHON2000815, HCHON2000817, HCHON2000818, HCHON2000819, HCHON2000832, HCHON2000851, HCHON2000935, HCHON2000942, HCHON2000956, HCHON2000994, HCHON2001084, HCHON2001099,
HCHON2001110, HCHON2001116, HCHON2001161, HCHON2001200, HCHON2001214, HCHON2001217, HCHON2001233, HCHON2001269, HCHON2001450, HCHON2001453, HCHON2001497, HCHON2001519, HCHON2001523, HCHON2001535, HCHON2001548, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118,
HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411,
HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003781, HEART2003836, HEART2003852, HEART2004570, HEART2004764, HEART2004931, HEART2004940, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108,
HEART2008111, HEART2008257, HEART2008509, HEART2008994, HEART2009000, HEART2009599, HEART2009680, HEART2010391, HEART2010492, HEART2010495, LIVER1000017, LIVER1000050, LIVER1000055, LIVER1000058, LIVER1000063, LIVER1000067, LIVER1000073, LIVER1000082, LIVER1000099, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769,
LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410, LIVER2006469, LIVER2006644, LIVER2006764, LIVER2007415,
LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000178, MAMGL1000182, MAMGL1000184, MESAN1000032, MESAN1000035, MESAN1000050, MESAN1000079, MESAN1000080, MESAN1000101, MESAN1000121, MESAN1000126, MESAN1000147, MESAN1000180, MESAN2000023, MESAN2000026, MESAN2000067, MESAN2000092, MESAN2000149, MESAN2000167, MESAN2000291, MESAN2000434, MESAN2000457, MESAN2000462, MESAN2000501, MESAN2000620, MESAN2000711,
MESAN2000815, MESAN2000894, MESAN2000909, MESAN2001154, MESAN2001450, MESAN2001627, MESAN2001770, MESAN2001979, MESAN2002113, MESAN2002122, MESAN2002147, MESAN2002186, MESAN2002424, MESAN2002687, MESAN2002709, MESAN2002790, MESAN2002844, MESAN2002940, MESAN2002978, MESAN2003035, MESAN2003037, MESAN2003039, MESAN2003058, MESAN2003101, MESAN2003190, MESAN2003322, MESAN2003444, MESAN2003490, MESAN2003529, MESAN2003646, MESAN2003662, MESAN2003709, MESAN2003851, MESAN2004138, MESAN2004288, MESAN2004575, MESAN2005303, MESAN2005371, MESAN2005633, MESAN2005724, MESAN2005808, MESAN2005811, MESAN2005957, MESAN2005958, MESAN2006022, MESAN2006043, MESAN2006328, MESAN2006401, MESAN2006580, MESAN2006599,
MESAN2006743, MESAN2006953, MESAN2007032, MESAN2007440, MESAN2007597, MESAN2007755, MESAN2008222, MESAN2008334, MESAN2008415, MESAN2008460, MESAN2008536, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013284, MESAN2013611, MESAN2013674, MESAN2013908, MESAN2013936,
MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N41000340, NB9N42000104, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108,
NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000014, NT2NE1000018, NT2NE1000023,
NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000056, NT2NE2000057, NT2NE2000064, NT2NE2000109, NT2NE2000137, NT2NE2000156, NT2NE2000222, NT2NE2000259, NT2NE2000284, NT2NE2000299, NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909,
NT2NE2000913, NT2NE2000950, NT2NE2000951, NT2NE2000980, NT2NE2001005, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001442, NT2NE2001524, NT2NE2001545, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311,
NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821,
NT2NE2005876, NT2NE2005890, NT2NE2005921, NT2NE2005968, NT2NE2006075, NT2NE2006087, NT2NE2006217, NT2NE2006288, NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608,
NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221,
NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916,
NT2NE2019056, NT2NE2019091, NT2NE2019099, NT2RI1000016, NT2RI1000035, NT2RI1000048, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000255, NT2RI2000270, NT2RI2000276, NT2RI2000294, NT2RI2000313, NT2RI2000341, NT2RI2000344, NT2RI2000348, NT2RI2000412, NT2RI2000421, NT2RI2000480, NT2RI2000575, NT2RI2000588, NT2RI2000597, NT2RI2000669, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000987, NT2RI2001010, NT2RI2001017, NT2RI2001030, NT2RI2001083, NT2RI2001091, NT2RI2001167, NT2RI2001178,
NT2RI2001230, NT2RI2001235, NT2RI2001244, NT2RI2001307, NT2RI2001342, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001769, NT2RI2001782, NT2RI2001836, NT2RI2001866, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002270, NT2RI2002334, NT2RI2002359, NT2RI2002447, NT2RI2002481, NT2RI2002507, NT2RI2002517, NT2RI2002530, NT2RI2002540, NT2RI2002549, NT2RI2002564, NT2RI2002585, NT2RI2002592, NT2RI2002602, NT2RI2002654, NT2RI2002699, NT2RI2002802, NT2RI2002819, NT2RI2002822,
NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003205, NT2RI2003222, NT2RI2003301, NT2RI2003338, NT2RI2003360, NT2RI2003361, NT2RI2003383, NT2RI2003392, NT2RI2003399, NT2RI2003407, NT2RI2003420, NT2RI2003481, NT2RI2003487, NT2RI2003556, NT2RI2003655, NT2RI2003667, NT2RI2003678, NT2RI2003751, NT2RI2003884, NT2RI2003973, NT2RI2004059, NT2RI2004078, NT2RI2004079, NT2RI2004093, NT2RI2004136, NT2RI2004157, NT2RI2004188, NT2RI2004190, NT2RI2004284, NT2RI2004290, NT2RI2004304, NT2RI2004381, NT2RI2004398, NT2RI2004410, NT2RI2004442, NT2RI2004468, NT2RI2004606, NT2RI2004618, NT2RI2004783,
NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005166, NT2RI2005239, NT2RI2005246, NT2RI2005315, NT2RI2005353, NT2RI2005358, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005772, NT2RI2005811, NT2RI2005816, NT2RI2005851, NT2RI2005966, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006640,
NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006855, NT2RI2006943, NT2RI2006973, NT2RI2007046, NT2RI2007048, NT2RI2007096, NT2RI2007116, NT2RI2007148, NT2RI2007214, NT2RI2007277, NT2RI2007303, NT2RI2007334, NT2RI2007384, NT2RI2007386, NT2RI2007406, NT2RI2007439, NT2RI2007445, NT2RI2007468, NT2RI2007469, NT2RI2007507, NT2RI2007589, NT2RI2007723, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008141, NT2RI2008171,
NT2RI2008188, NT2RI2008204, NT2RI2008221, NT2RI2008233, NT2RI2008455, NT2RI2008502, NT2RI2008566, NT2RI2008598, NT2RI2008622, NT2RI2008656, NT2RI2008714, NT2RI2008724, NT2RI2008791, NT2RI2008801, NT2RI2008808, NT2RI2008812, NT2RI2008841, NT2RI2008942, NT2RI2008952, NT2RI2009005, NT2RI2009083, NT2RI2009101, NT2RI2009103, NT2RI2009144, NT2RI2009151, NT2RI2009215, NT2RI2009233, NT2RI2009259, NT2RI2009269, NT2RI2009281, NT2RI2009289, NT2RI2009301, NT2RI2009402, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373,
NT2RI2014247, NT2RI2014551, NT2RI2014683, NT2RI2014733, NT2RI2015239, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213,
NT2RI3000423, NT2RI3000622, NT2RI3001132, NT2RI3001263, NT2RI3001279, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757,
NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000017, NT2RP6000032, NT2RP6000033, NT2RP6000039, NT2RP6000043, NT2RP6000059, NT2RP6000060, NT2RP6000077, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926,
NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7002028, NT2RP7002064, NT2RP7002151, NT2RP7002163, NT2RP7002243, NT2RP7002282, NT2RP7002376, NT2RP7002379, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511,
NT2RP7003629, NT2RP7003632, NT2RP7003647, NT2RP7003680, NT2RP7003724, NT2RP7003960, NT2RP7004027, NT2RP7004080, NT2RP7004114, NT2RP7004123, NT2RP7004173, NT2RP7004196, NT2RP7004204, NT2RP7004233, NT2RP7004260, NT2RP7004352, NT2RP7004358, NT2RP7004396, NT2RP7004481, NT2RP7004541, NT2RP7004559, NT2RP7004641, NT2RP7004656, NT2RP7004687, NT2RP7004722, NT2RP7004766, NT2RP7004790, NT2RP7004915, NT2RP7004925, NT2RP7004961, NT2RP7004964, NT2RP7004975, NT2RP7005118, NT2RP7005205, NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006075, NT2RP7006160, NT2RP7006162,
NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006887, NT2RP7006980, NT2RP7006995, NT2RP7007114, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007530, NT2RP7007537, NT2RP7007580, NT2RP7007594, NT2RP7007617, NT2RP7007643, NT2RP7007766, NT2RP7007842, NT2RP7007925, NT2RP7007930, NT2RP7007975, NT2RP7008013, NT2RP7008133, NT2RP7008137, NT2RP7008142, NT2RP7008144, NT2RP7008161, NT2RP7008167,
NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009097, NT2RP7009108, NT2RP7009147, NT2RP7009149, NT2RP7009168, NT2RP7009215, NT2RP7009225, NT2RP7009236, NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867,
NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365,
NT2RP7017474, NT2RP7017546, NT2RP7017567, NT2RP7017795, NT2RP7017971, NT2RP7018032, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033,
NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000247, NTONG1000264, NTONG2000040, NTONG2000107, NTONG2000231, NTONG2000265, NTONG2000334, NTONG2000363, NTONG2000413, NTONG2000492, NTONG2000499, NTONG2000531, NTONG2000583, NTONG2000858, NTONG2000876, NTONG2000878, NTONG2000966, NTONG2000977, NTONG2000985, NTONG2001014, NTONG2001079, NTONG2001157, NTONG2001173, NTONG2001220, NTONG2001222, NTONG2001362, NTONG2001428, NTONG2001532, NTONG2001550, NTONG2001587, NTONG2001619, NTONG2001642, NTONG2001657, NTONG2001762, NTONG2002278, NTONG2002582, NTONG2002807, NTONG2002948, NTONG2002970,
NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004844, NTONG2004991, NTONG2005062, NTONG2005086, NTONG2005137, NTONG2005153, NTONG2005265, NTONG2005277, NTONG2005363, NTONG2005373, NTONG2005391, NTONG2005480, NTONG2005497, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028,
NTONG2007034, NTONG2007249, NTONG2007517, NTONG2007522, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000192, OCBBF1000254, OCBBF1000315, OCBBF2000013, OCBBF2000015, OCBBF2000126, OCBBF2000178, OCBBF2000231, OCBBF2000277,
OCBBF2000287, OCBBF2000323, OCBBF2000452, OCBBF2000677, OCBBF2000703, OCBBF2000712, OCBBF2000719, OCBBF2000831, OCBBF2000904, OCBBF2000982, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389, OCBBF2001402, OCBBF2001408, OCBBF2001416, OCBBF2001473, OCBBF2001492, OCBBF2001527, OCBBF2001528, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002086, OCBBF2002124, OCBBF2002161, OCBBF2002290, OCBBF2002357, OCBBF2002376, OCBBF2002615, OCBBF2002626, OCBBF2002656,
OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843,
OCBBF2005901, OCBBF2005956, OCBBF2006005, OCBBF2006030, OCBBF2006058, OCBBF2006113, OCBBF2006151, OCBBF2006154, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008005, OCBBF2008116,
OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008586, OCBBF2008640, OCBBF2008691, OCBBF2008701, OCBBF2008724, OCBBF2008768, OCBBF2008770, OCBBF2008775, OCBBF2008790, OCBBF2008814, OCBBF2008822, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843,
OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011177, OCBBF2011228, OCBBF2011232, OCBBF2011283, OCBBF2011311, OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011,
OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014089, OCBBF2014322, OCBBF2014386, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612,
OCBBF2016689, OCBBF2016690, OCBBF2016729, OCBBF2016841, OCBBF2016928, OCBBF2017055, OCBBF2017069, OCBBF2017306, OCBBF2017325, OCBBF2017398, OCBBF2017458, OCBBF2017489, OCBBF2017516, OCBBF2017665, OCBBF2017754, OCBBF2017791, OCBBF2017815, OCBBF2017882, OCBBF2017888, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741,
OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173,
OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598,
OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066,
PEBLM1000068, PEBLM1000071, PEBLM1000083, PEBLM1000144, PEBLM1000147, PEBLM1000180, PEBLM2000030, PEBLM2000112, PEBLM2000126, PEBLM2000170, PEBLM2000180, PEBLM2000213, PEBLM2000248, PEBLM2000270, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215,
PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957,
PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000178, PLACE5000245, PLACE5000260,
PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000325, PLACE6000348, PLACE6000371, PLACE6000414, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001064, PLACE6001118, PLACE6001153, PLACE6001185, PLACE6001262, PLACE6001281, PLACE6001294, PLACE6001536, PLACE6001557, PLACE6001712, PLACE6001740, PLACE6001823, PLACE6001886, PLACE6001925, PLACE6001933, PLACE6002011, PLACE6002016, PLACE6002056,
PLACE6002102, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004397, PLACE6004454, PLACE6004464, PLACE6004491, PLACE6004578, PLACE6004663, PLACE6004687, PLACE6004738, PLACE6004931, PLACE6004966, PLACE6004993, PLACE6005007,
PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007482, PLACE6007687, PLACE6007787, PLACE6007798, PLACE6007807, PLACE6007925, PLACE6008036,
PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011627, PLACE6011774, PLACE6011881, PLACE6011907, PLACE6011970, PLACE6011975, PLACE6012028,
PLACE6012108, PLACE6012168, PLACE6012297, PLACE6012308, PLACE6012574, PLACE6012908, PLACE6012936, PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016942, PLACE6016984, PLACE6017002, PLACE6017431, PLACE6017564, PLACE6017578, PLACE6017701, PLACE6017714, PLACE6017788, PLACE6017791, PLACE6018031, PLACE6018107, PLACE6018187,
PLACE6018235, PLACE6018287, PLACE6018441, PLACE6018487, PLACE6018497, PLACE6018769, PLACE6018834, PLACE6018843, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973,
PLACE7008136, PLACE7008261, PLACE7008431, PLACE7008623, PLACE7008766, PLACE7009563, PROST1000039, PROST1000065, PROST1000083, PROST1000085, PROST1000110, PROST1000123, PROST1000136, PROST1000145, PROST1000152, PROST1000167, PROST1000184, PROST1000199, PROST1000215, PROST1000217, PROST1000220, PROST1000246, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000564, PROST1000575, PROST2000053, PROST2000067, PROST2000079, PROST2000105, PROST2000143, PROST2000162,
PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101,
PROST2002162, PROST2002212, PROST2002227, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003340, PROST2003396, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251,
PROST2004258, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005426, PROST2005466, PROST2005630, PROST2005683, PROST2005788, PROST2005880, PROST2005886, PROST2005904, PROST2005919, PROST2005943, PROST2006008, PROST2006020, PROST2006030, PROST2006060, PROST2006196, PROST2006201, PROST2006282, PROST2006343, PROST2006450, PROST2006491, PROST2006510, PROST2006579, PROST2006688, PROST2006766, PROST2006782, PROST2006933, PROST2006988, PROST2007122, PROST2007200, PROST2007237,
PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318,
PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925,
PROST2015124, PROST2015137, PROST2015198, PROST2015243, PROST2015246, PROST2015251, PROST2015332, PROST2015457, PROST2015537, PROST2015545, PROST2015551, PROST2015636, PROST2015710, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017578, PROST2017612, PROST2017617, PROST2017700, PROST2017701, PROST2017729, PROST2017836, PROST2017910, PROST2017972, PROST2018030, PROST2018090,
PROST2018235, PROST2018437, PROST2018487, PROST2018511, PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003079,
PUAEN2003116, PUAEN2003408, PUAEN2003517, PUAEN2004067, PUAEN2004083, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749,
RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000138, SKMUS1000176, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000515, SKMUS2000679, SKMUS2000690, SKMUS2000701, SKMUS2000705,
SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695,
SKMUS2001703, SKMUS2001740, SKMUS2001742, SKMUS2001823, SKMUS2001850, SKMUS2002077, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000229,
SKNMC1000251, SKNMC1000264, SKNMC2000224, SKNMC2000256, SKNMC2000305, SKNMC2000356, SKNMC2000374, SKNMC2000583, SKNMC2000612, SKNMC2000622, SKNMC2000877, SKNMC2000966, SKNMC2001113, SKNMC2001324, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000101, SKNSH1000174, SKNSH1000301, SKNSH1000308, SKNSH1000416, SKNSH2000051, SKNSH2000101, SKNSH2000163, SKNSH2000250, SKNSH2000319, SKNSH2000482, SKNSH2000516, SKNSH2000550, SKNSH2000819, SKNSH2001222, SKNSH2001875,
SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357; SKNSH2009435, SKNSH2009991, SKNSH2010015, SMINT1000012, SMINT1000039, SMINT1000042, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137,
SMINT1000192, SMINT2000032, SMINT2000040, SMINT2000060, SMINT2000073, SMINT2000084, SMINT2000145, SMINT2000176, SMINT2000185, SMINT2000227, SMINT2000229, SMINT2000232, SMINT2000267, SMINT2000400, SMINT2000454, SMINT2000468, SMINT2000518, SMINT2000529, SMINT2000530, SMINT2000537, SMINT2000538, SMINT2000541, SMINT2000545, SMINT2000558, SMINT2000562, SMINT2000568, SMINT2000609, SMINT2000629, SMINT2000694, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001818, SMINT2001950,
SMINT2002126, SMINT2002159, SMINT2002202, SMINT2002210, SMINT2002224, SMINT2002281, SMINT2002311, SMINT2002328, SMINT2002414, SMINT2002457, SMINT2002620, SMINT2002689, SMINT2002743, SMINT2002778, SMINT2002880, SMINT2002899, SMINT2002974, SMINT2002976, SMINT2003003, SMINT2003128, SMINT2003150, SMINT2003317, SMINT2003386, SMINT2003423, SMINT2003505, SMINT2003551, SMINT2003569, SMINT2003641, SMINT2003644, SMINT2003866, SMINT2003905, SMINT2004037, SMINT2004047, SMINT2004086, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545,
SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010959, SMINT2010997,
SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313,
SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000116, SPLEN1000134, SPLEN1000139, SPLEN1000143, SPLEN1000145, SPLEN1000156, SPLEN1000163, SPLEN1000178, SPLEN2000020, SPLEN2000064, SPLEN2000072, SPLEN2000098, SPLEN2000126,
SPLEN2000143, SPLEN2000189, SPLEN2000197, SPLEN2000205, SPLEN2000210, SPLEN2000242, SPLEN2000243, SPLEN2000247, SPLEN2000267, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000698, SPLEN2000839, SPLEN2000882, SPLEN2001135, SPLEN2001141, SPLEN2001176, SPLEN2001227, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710,
SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004343, SPLEN2004346, SPLEN2004368, SPLEN2004555, SPLEN2004611, SPLEN2004662, SPLEN2004773, SPLEN2004844,
SPLEN2005009, SPLEN2005227, SPLEN2005272, SPLEN2005342, SPLEN2005416, SPLEN2005450, SPLEN2005474, SPLEN2005727, SPLEN2005783, SPLEN2005790, SPLEN2005818, SPLEN2005838, SPLEN2005869, SPLEN2005927, SPLEN2005939, SPLEN2006122, SPLEN2006133, SPLEN2006232, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786,
SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672,
SPLEN2011678, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012571, SPLEN2012606, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318,
SPLEN2014381, SPLEN2014452, SPLEN2014454, SPLEN2014486, SPLEN2014543, SPLEN2014572, SPLEN2014645, SPLEN2014669, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016268, SPLEN2016304, SPLEN2016356,
SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017318, SPLEN2017426, SPLEN2017452, SPLEN2017538, SPLEN2017592, SPLEN2017613, SPLEN2017643, SPLEN2017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732,
SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295,
SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765,
SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683,SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098,
SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038834, SPLEN2039311, SPLEN2039379, SPLEN2039672, SPLEN2039697, SPLEN2039845, SPLEN2039936, SPLEN2040222,
SPLEN2040237, SPLEN2040824, SPLEN2041304, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, STOMA1000013, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000279, STOMA2000289, STOMA2000383, STOMA2000386, STOMA2000395, STOMA2000396, STOMA2000478, STOMA2000482, STOMA2000539, STOMA2000567, STOMA2000640, STOMA2000678, STOMA2000686, STOMA2000888, STOMA2001025, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000049, SYNOV1000118,
SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111, SYNOV2001212, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965,
SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542,
SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000116, TBAES2000255, TBAES2000315, TBAES2000352, TBAES2000932, TBAES2001171, TBAES2001220, TBAES2001229, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007481, TBAES2007504, TBAES2007548, TBAES2007697, TBAES2007862, TBAES2007964,
TBAES2008133, TCERX2000171, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753,
TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000085,
TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358,
TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313,
TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105,
TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002877, TESTI2002910, TESTI2002912, TESTI2002928, TESTI2002937, TESTI2002965, TESTI2002993, TESTI2003005, TESTI2003020,
TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579,
TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004432, TESTI2004452, TESTI2004459,
TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004734, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005025, TESTI2005040, TESTI2005060, TESTI2005072, TESTI2005085, TESTI2005120, TESTI2005153, TESTI2005155, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415,
TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005759, TESTI2005767, TESTI2005775, TESTI2005784, TESTI2005827, TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006109,
TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940,
TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032,
TESTI2008033, TESTI2008046, TESTI2008081, TESTI2008099, TESTI2008139, TESTI2008144, TESTI2008161, TESTI2008189, TESTI2008204, TESTI2008219, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239,
TESTI2009325, TESTI2009388, TESTI2009390, TESTI2009400, TESTI2009402, TESTI2009412, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009987, TESTI2010009, TESTI2010154, TESTI2010180, TESTI2010239, TESTI2010280, TESTI2010354, TESTI2010396, TESTI2010400, TESTI2010409, TESTI2010513, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009,
TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812,
TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339,
TESTI2014362, TESTI2014385, TESTI2014415, TESTI2014439; TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421,
TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645,
TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178,
TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917,
TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736,
TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968,
TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757,
TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206,
TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252,
TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505,
TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650,
TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041,
TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302,
TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964,
TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137,
TESTI2050681, TESTI2050780, TESTI2050987, TESTI2051148, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288,
TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647,
TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148,
TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429,
TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661,
TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961,
TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654,
TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062,
TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432,
TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TEST14035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744,
TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291,
TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000002, THYMU1000010, THYMU1000017, THYMU1000025, THYMU1000029, THYMU1000032, THYMU1000033, THYMU1000060, THYMU1000074, THYMU1000083, THYMU1000098, THYMU1000103,
THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000382, THYMU1000393, THYMU1000399, THYMU1000426, THYMU1000440, THYMU1000459, THYMU1000490, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276,
THYMU2000280, THYMU2000317, THYMU2000368, THYMU2000382, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000767, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898,
THYMU2001900, THYMU2001916, THYMU2001926, THYMU2001989, THYMU2002023, THYMU2002037, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002756, THYMU2002789, THYMU2002792, THYMU2002815, THYMU2002841, THYMU2002852, THYMU2002910, THYMU2002950, THYMU2002983, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003419, THYMU2003440, THYMU2003470,
THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004152, THYMU2004169, THYMU2004284, THYMU2004320, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283,
THYMU2005303, THYMU2005321, THYMU2005356, THYMU2005369, THYMU2005439, THYMU2005480, THYMU2005482, THYMU2005507, THYMU2005545, THYMU2005546, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006813, THYMU2006913, THYMU2006946, THYMU2006965, THYMU2007025, THYMU2007036, THYMU2007060, THYMU2007065, THYMU2007095, THYMU2007112, THYMU2007146,
THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007802, THYMU2007824, THYMU2007854, THYMU2007886, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383 , THYMU2008452 , THYMU2008643 , THYMU2008351, THYMU2008383, THYMU2008452, THYMU2008643, THYMU2008686, THYMU2008691, THYMU2008714, THYMU2008725, THYMU2008727, THYMU2008781,
THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183,
THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089,. THYMU2013364,
THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010,
THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416,
THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249,
THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032035, THYMU2032080, THYMU2032150, THYMU2032155, THYMU2032358, THYMU2032437,
THYMU2032601, THYMU2032655, THYMU2032715, THYMU2032732, THYMU2032825, THYMU2032976, THYMU2033053, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226,
THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420,
THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620,
THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313,
THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200,
THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200,
THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062,
TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263,
TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528,
TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000038, TRACH1000057, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000047, TRACH2000081, TRACH2000083, TRACH2000148, TRACH2000185,
TRACH2000196, TRACH2000235, TRACH2000237, TRACH2000243, TRACH2000248, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000472, TRACH2000496, TRACH2000497, TRACH2000502, TRACH2000522, TRACH2000540, TRACH2000559, TRACH2000660, TRACH2000665, TRACH2000675, TRACH2000677, TRACH2000703, TRACH2000767, TRACH2000775, TRACH2000807, TRACH2000862, TRACH2000873, TRACH2000894, TRACH2000898, TRACH2000920, TRACH2000923, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001192,
TRACH2001249, TRACH2001275, TRACH2001335, TRACH2001339, TRACH2001347, TRACH2001395, TRACH2001400, TRACH2001403, TRACH2001416, TRACH2001428, TRACH2001443, TRACH2001463, TRACH2001474, TRACH2001523, TRACH2001525, TRACH2001532, TRACH2001549, TRACH2001592, TRACH2001596, TRACH2001607, TRACH2001612, TRACH2001621, TRACH2001646, TRACH2001651, TRACH2001898, TRACH2001933, TRACH2001968, TRACH2001996, TRACH2002043, TRACH2002054, TRACH2002100, TRACH2002138, TRACH2002333, TRACH2002784, TRACH2002803, TRACH2002840, TRACH2002853, TRACH2002862, TRACH2002954, TRACH2002960, TRACH2002988, TRACH2003070, TRACH2003323, TRACH2003484, TRACH2003516, TRACH2003605, TRACH2003736, TRACH2003759, TRACH2003941, TRACH2004039,
TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009310, TRACH2009340, TRACH2009348, TRACH2009358, TRACH2009661, TRACH2009730, TRACH2009743, TRACH2009754, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600,
TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011500, TRACH2011574, TRACH2011894, TRACH2011956, TRACH2012000, TRACH2012138, TRACH2012242, TRACH2012298, TRACH2012370, TRACH2012387, TRACH2012497, TRACH2012536, TRACH2012562, TRACH2012742, TRACH2012811, TRACH2012823, TRACH2012918, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337,
TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015823, TRACH2015824, TRACH2015939, TRACH2015987, TRACH2016080, TRACH2016131, TRACH2016286, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084,
TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860,
TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107,
TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211,
TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008201, TRACH3008508, TRACH3008535, TRACH3008629, TRACH3008632, TRACH3008688, TRACH3008697,
TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700,
TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621,
TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148,
TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065,
TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207,. TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086,
TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122,UMVEN1000143, UMVEN1000156, UMVEN2000046, UMVEN2000069, UMVEN2000121, UMVEN2000133, UMVEN2000152, UMVEN2000453, UTERU1000008,
UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000424, UTERU2000465, UTERU2000485, UTERU2000517, UTERU2000524, UTERU2000539, UTERU2000541, UTERU2000542, UTERU2000546, UTERU2000550,
UTERU2000569, UTERU2000607, UTERU2000629, UTERU2000649, UTERU2000663, UTERU2000794, UTERU2000830, UTERU2000844, UTERU2000922, UTERU2001024, UTERU2001110, UTERU2001176, UTERU2001281, UTERU2001389, UTERU2001409, UTERU2001412, UTERU2001504, UTERU2001607, UTERU2001747, UTERU2001876, UTERU2002001, UTERU2002011, UTERU2002176, UTERU2002198, UTERU2002294, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004039,
UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2004929, UTERU2005004, UTERU2005050, UTERU2005069, UTERU2005074, UTERU2005179, UTERU2005292, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006651, UTERU2006705, UTERU2006899, UTERU2007004, UTERU2007075, UTERU2007081, UTERU2007128, UTERU2007253, UTERU2007267, UTERU2007444,
UTERU2007499, UTERU2007639, UTERU2007924, UTERU2007942, UTERU2008018, UTERU2008019, UTERU2008027, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008785, UTERU2008845, UTERU2008901, UTERU2008930, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2010115, UTERU2010124, UTERU2010164, UTERU2010226, UTERU2010231, UTERU2010304, UTERU2010320, UTERU2010431, UTERU2010525, UTERU2010651,
UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012333, UTERU2012407, UTERU2012526, UTERU2012581, UTERU2012610, UTERU2012615, UTERU2012688, UTERU2012715, UTERU2012741, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001,
UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016426, UTERU2016464, UTERU2016669, UTERU2016757, UTERU2016761, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514,
UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941,
UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172,
UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959,
UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460,
UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837,
UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the LIVER, LYMPB, MAMGL, MESAN, MESTC, N1ESE, and NB9N4 libraries were analyzed for expression frequency. The analysis results are described below. Each is a relative value, where the expression level over all tissues is taken as 100. The larger this value, the greater the level of expression.

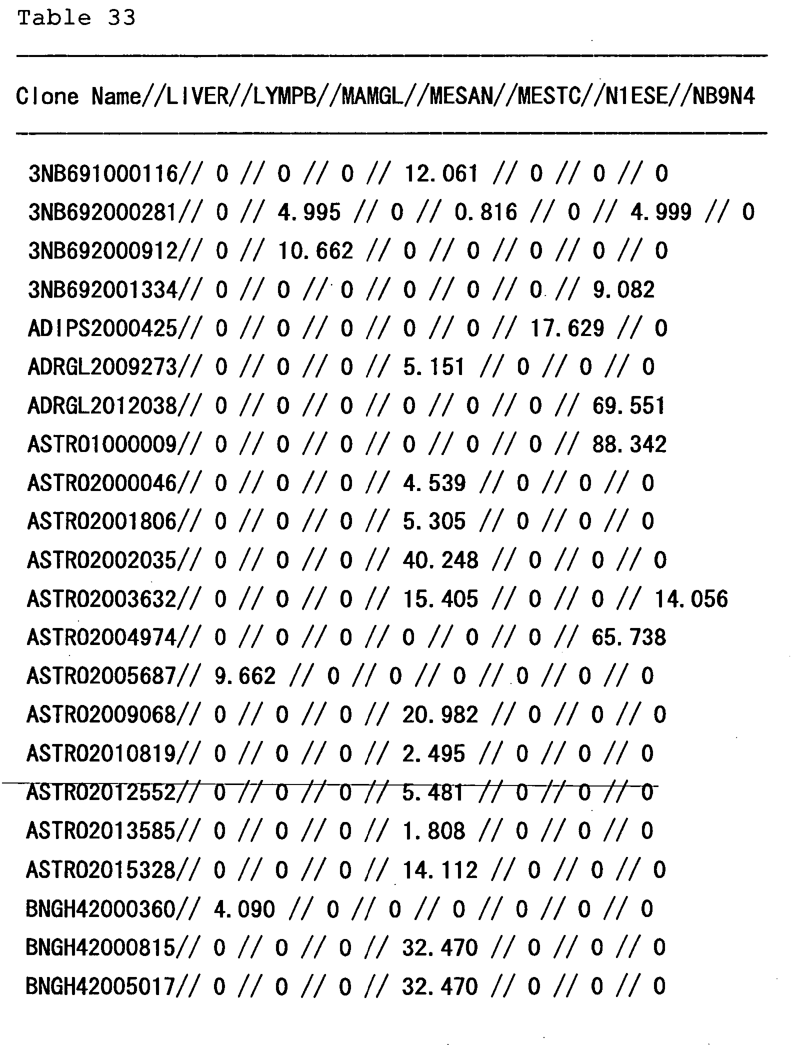

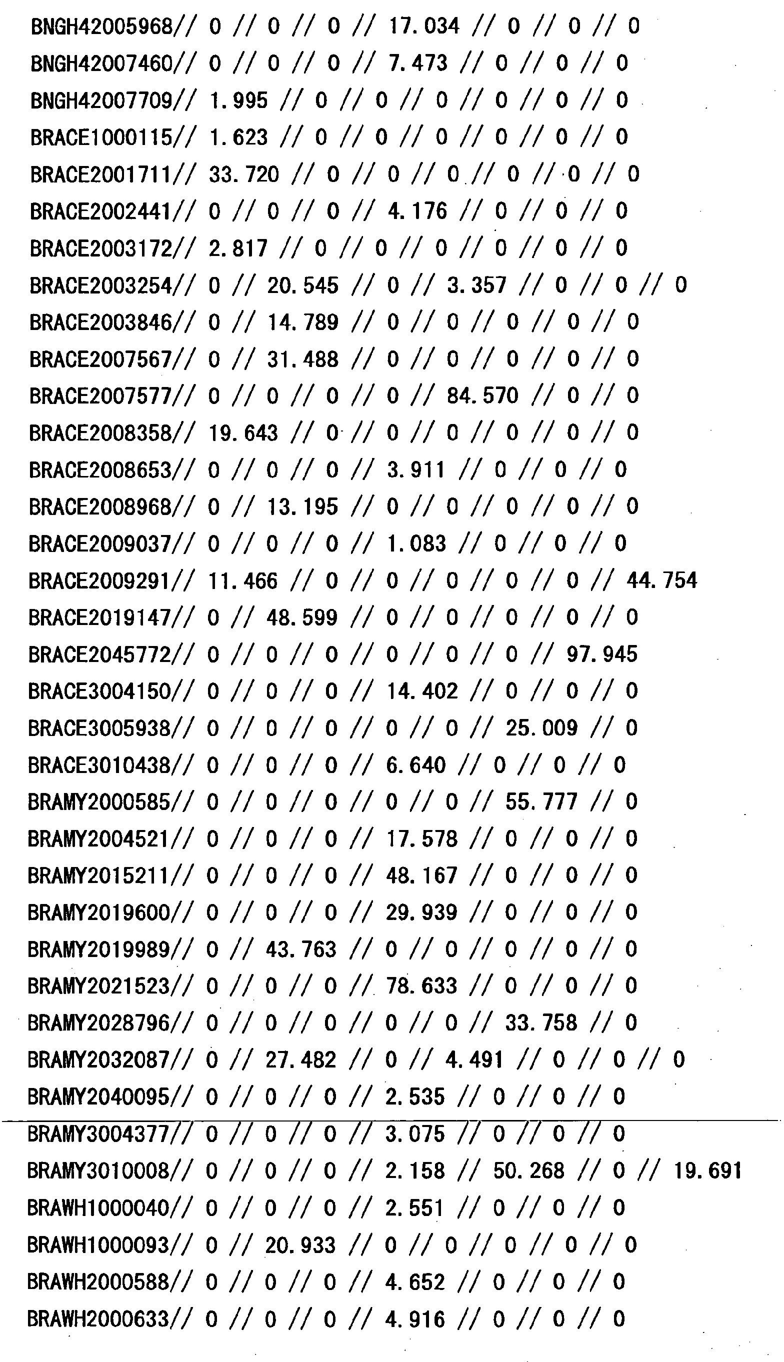

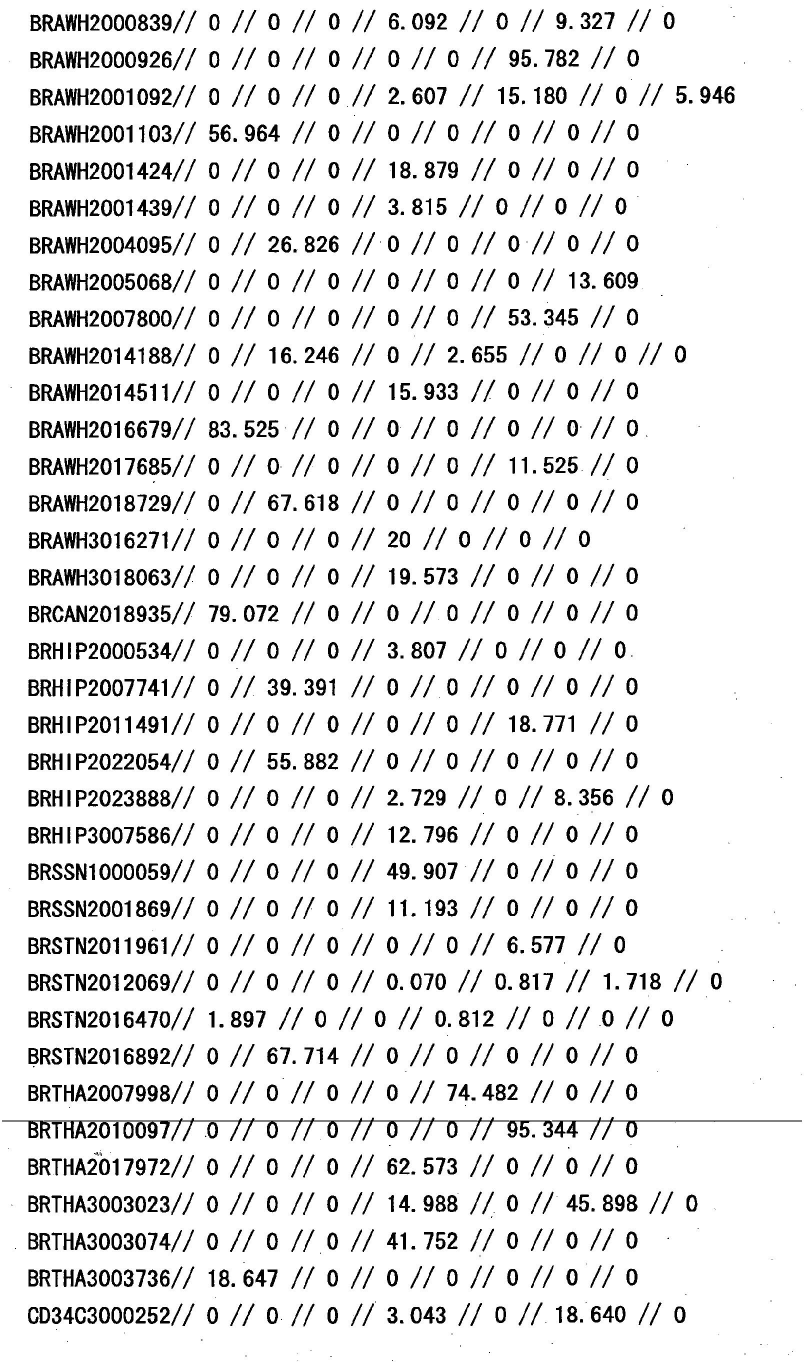

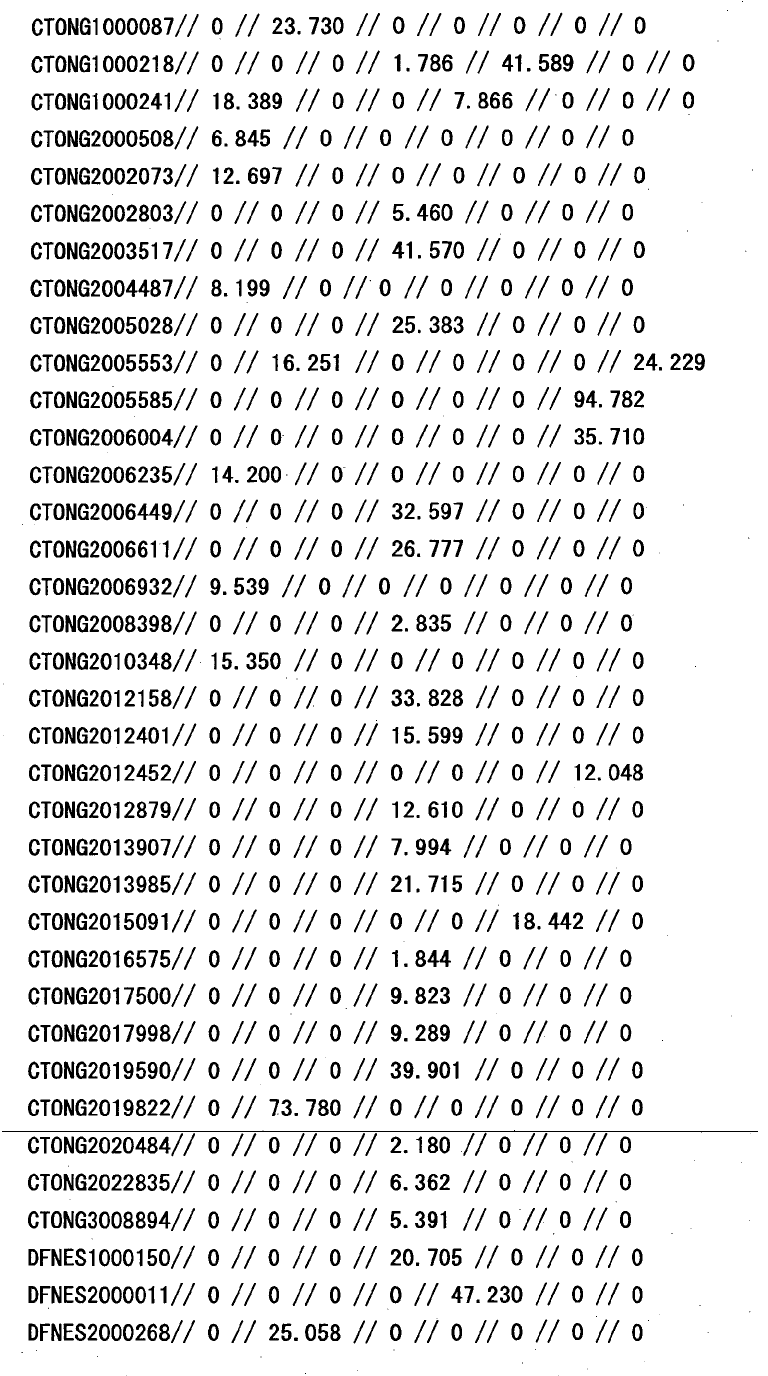

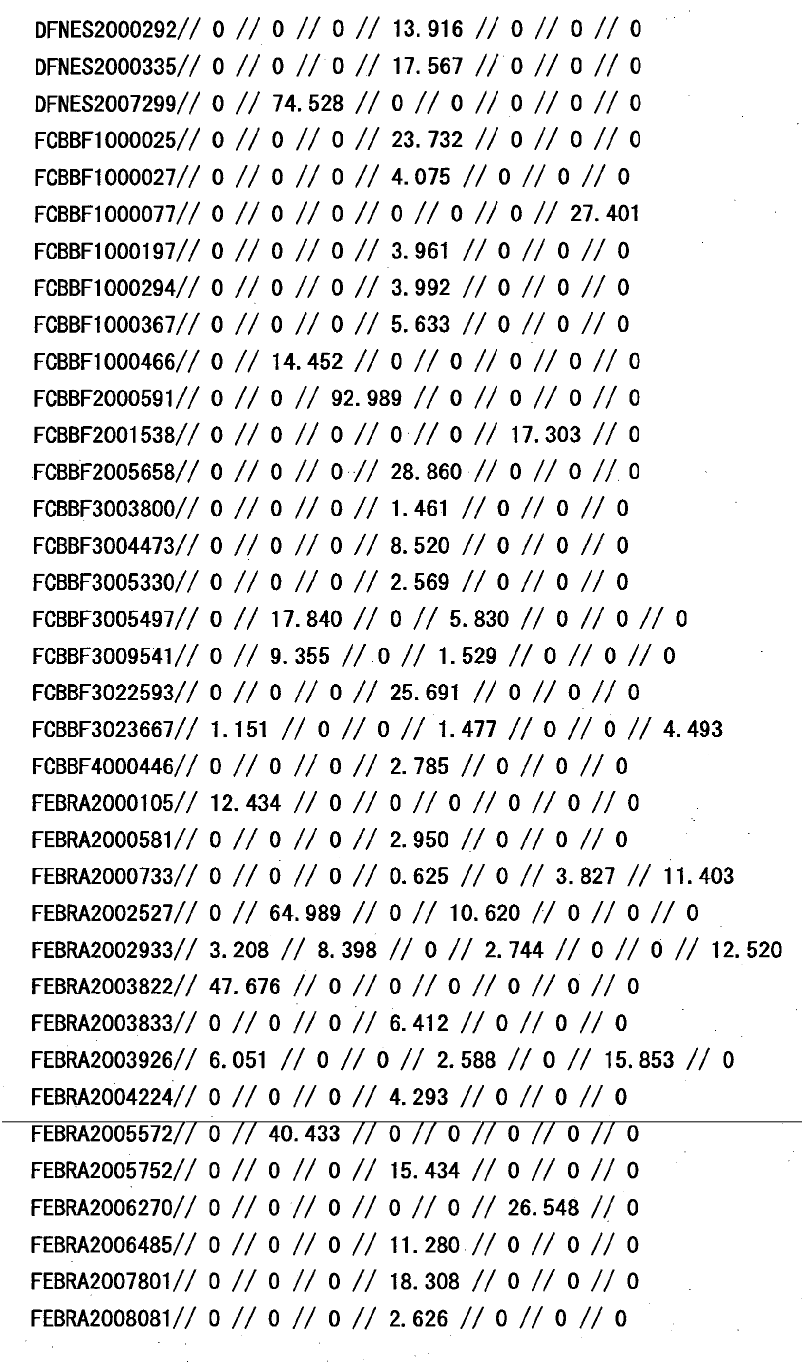

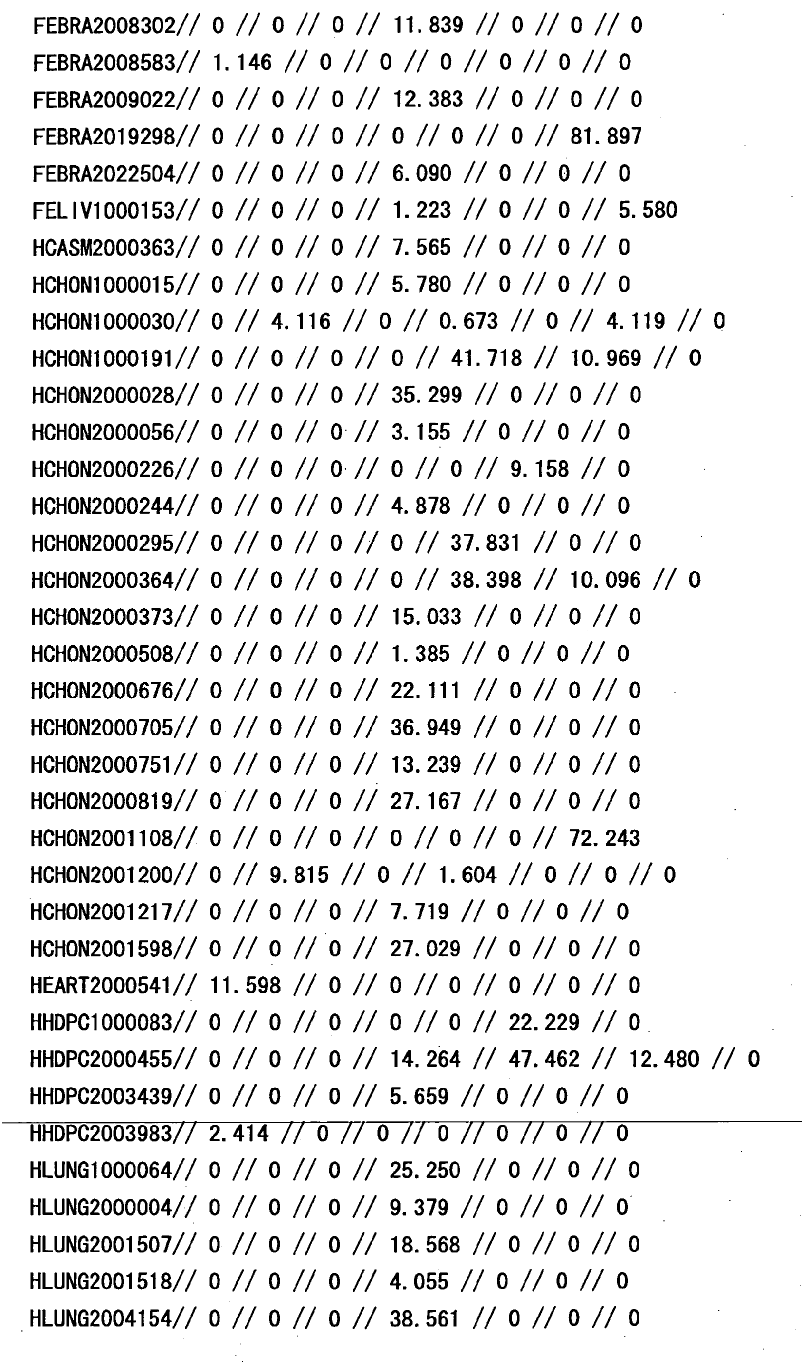

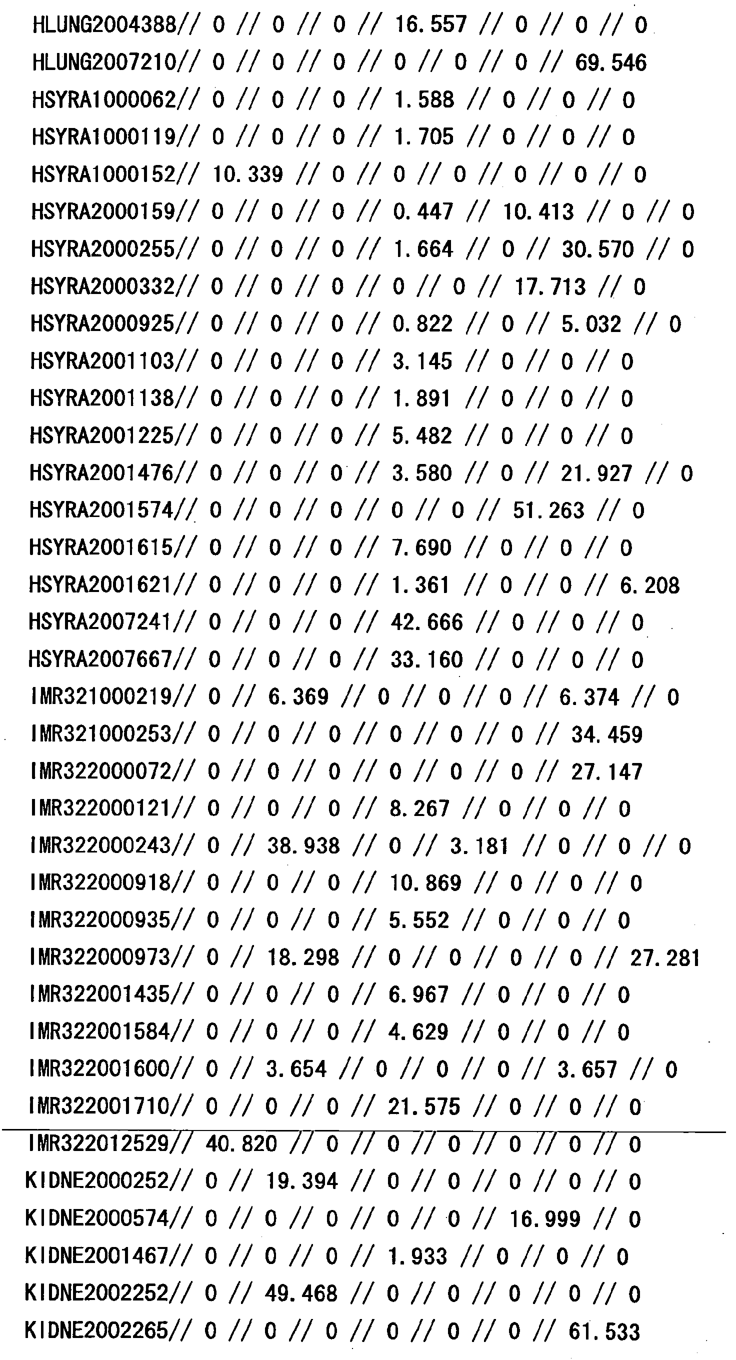

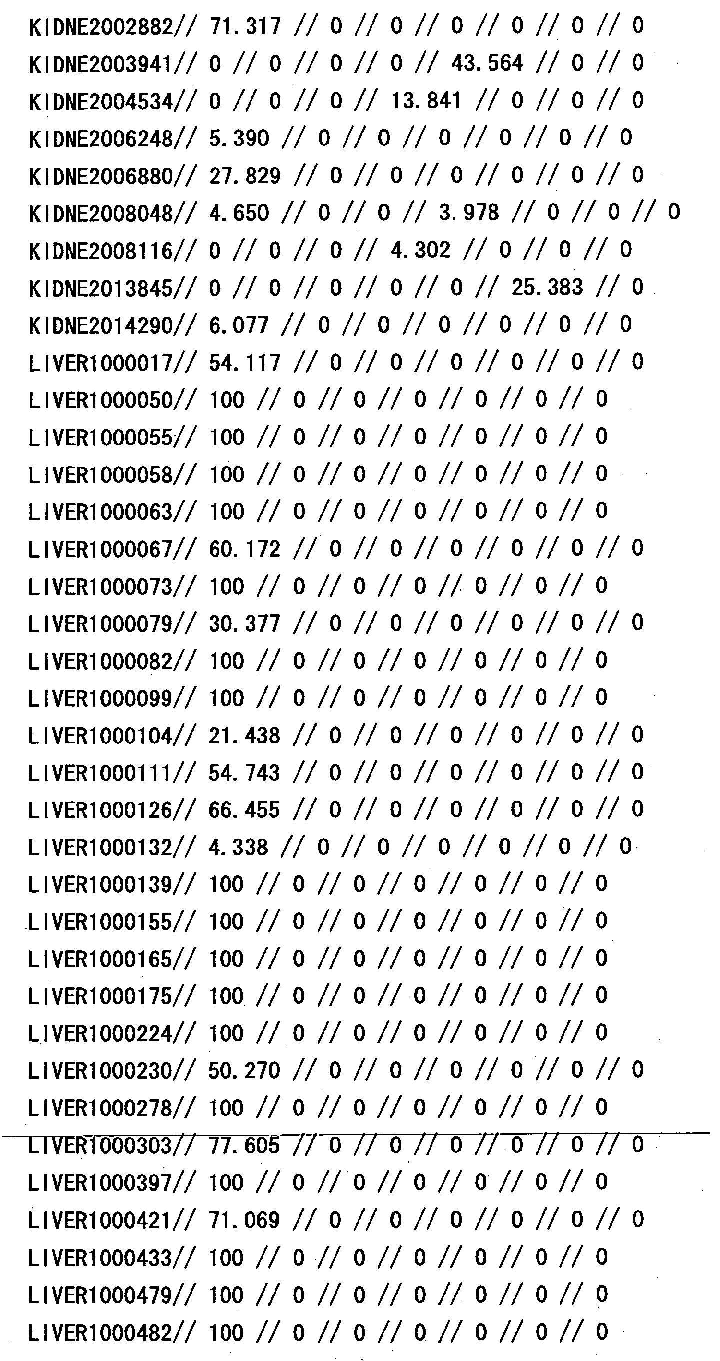

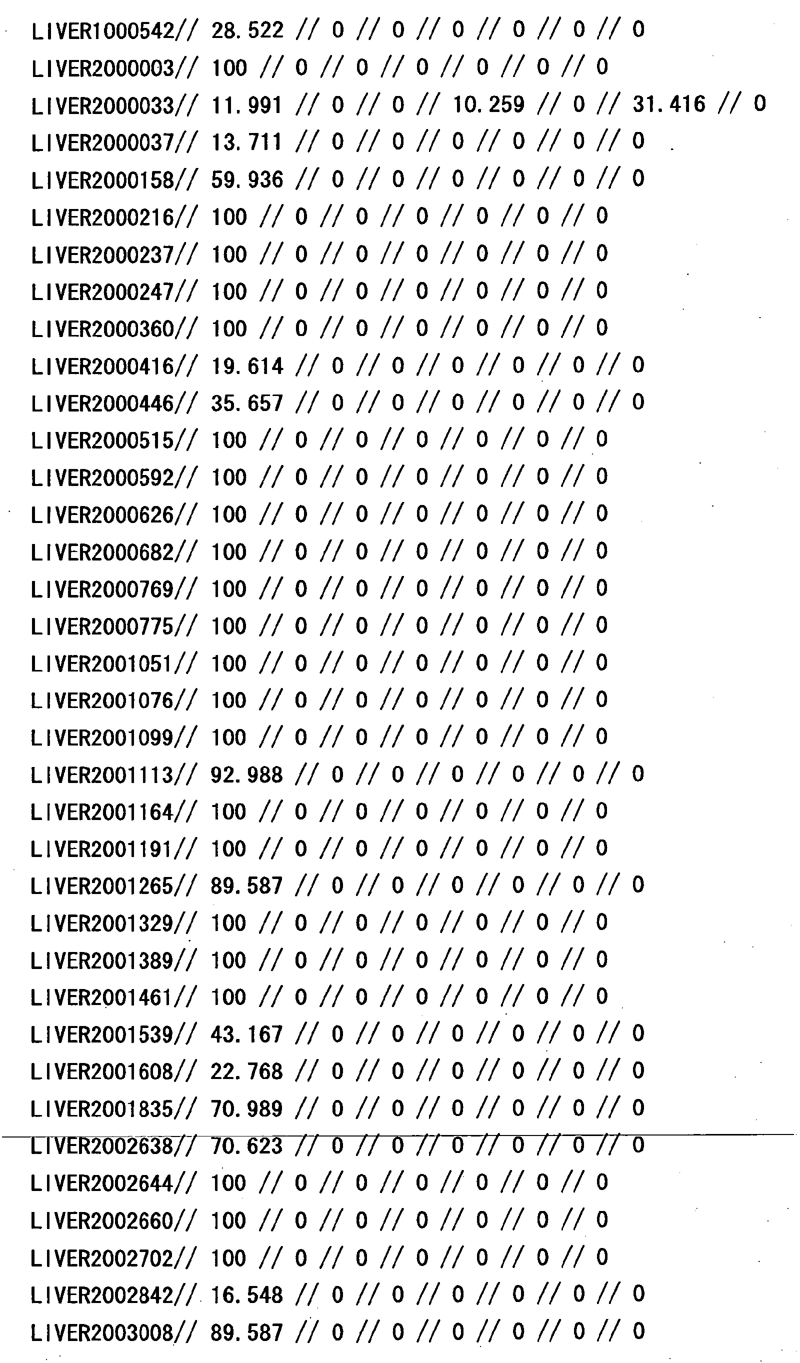

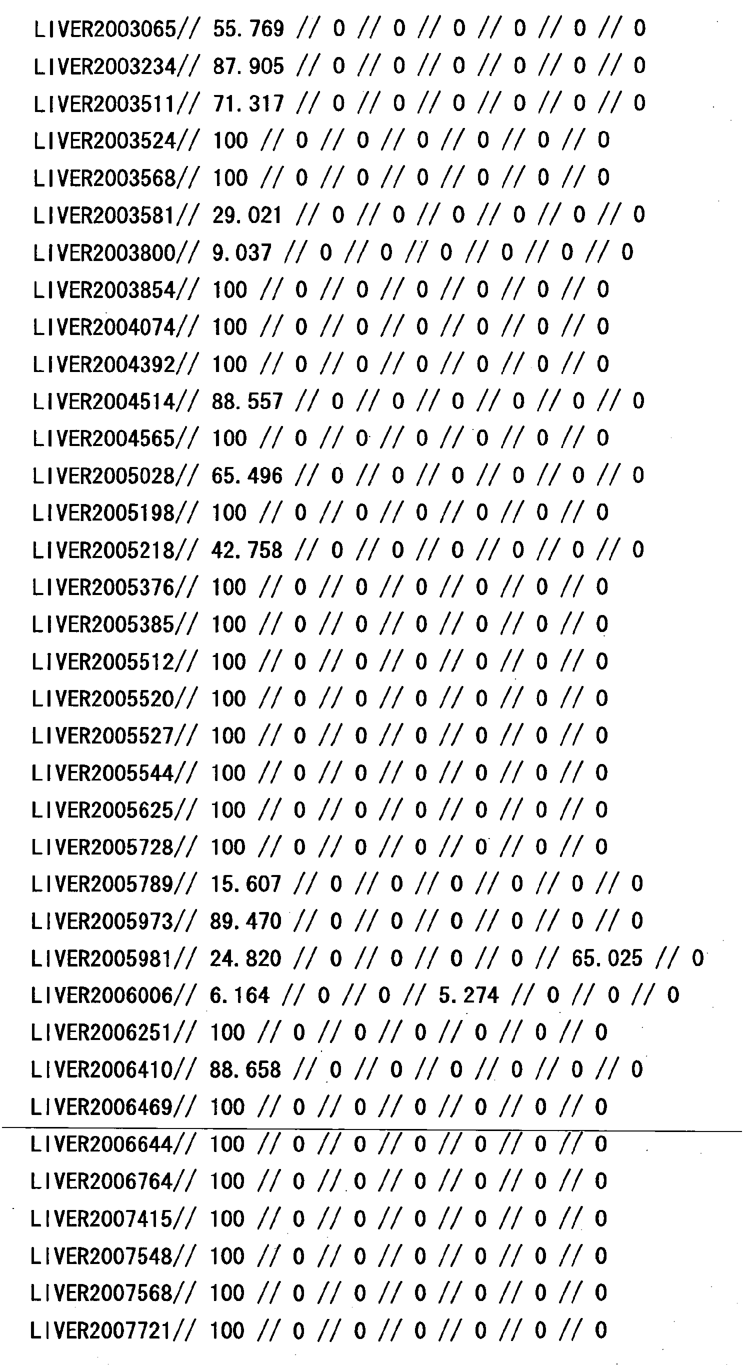

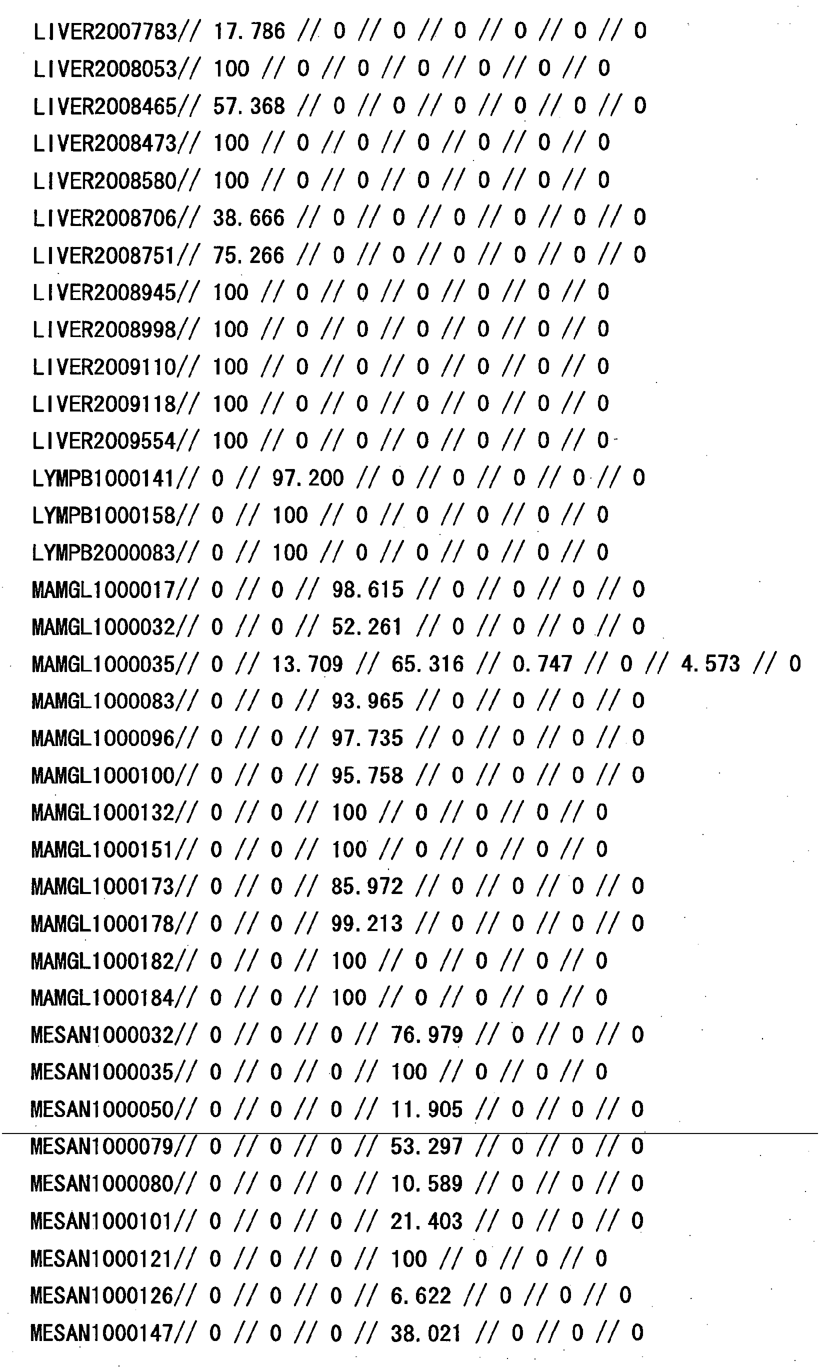

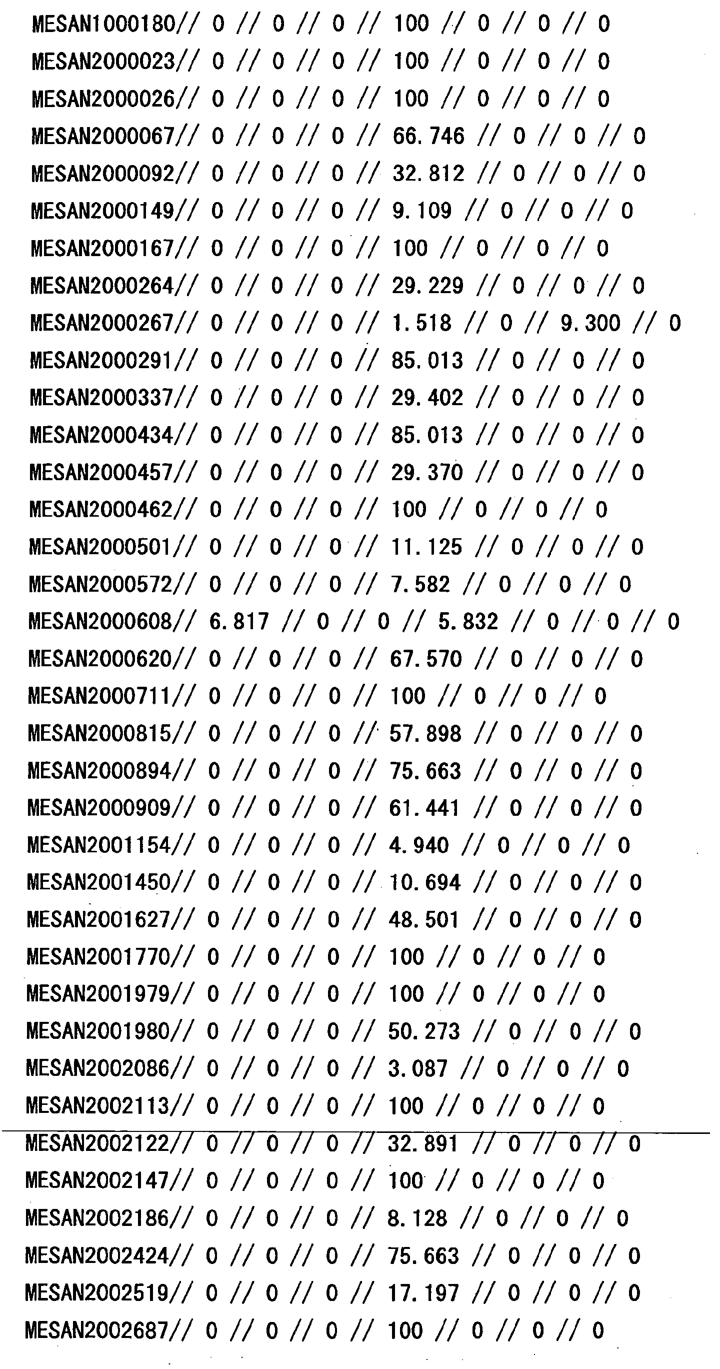

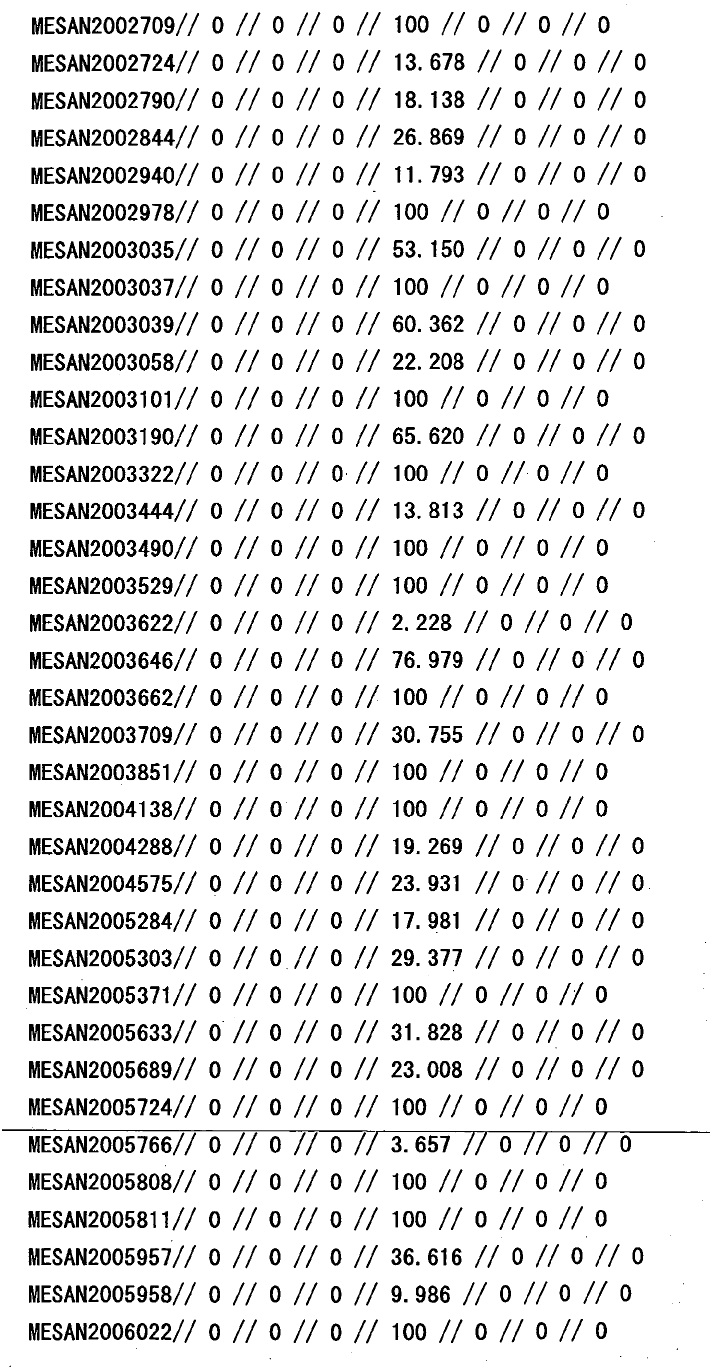

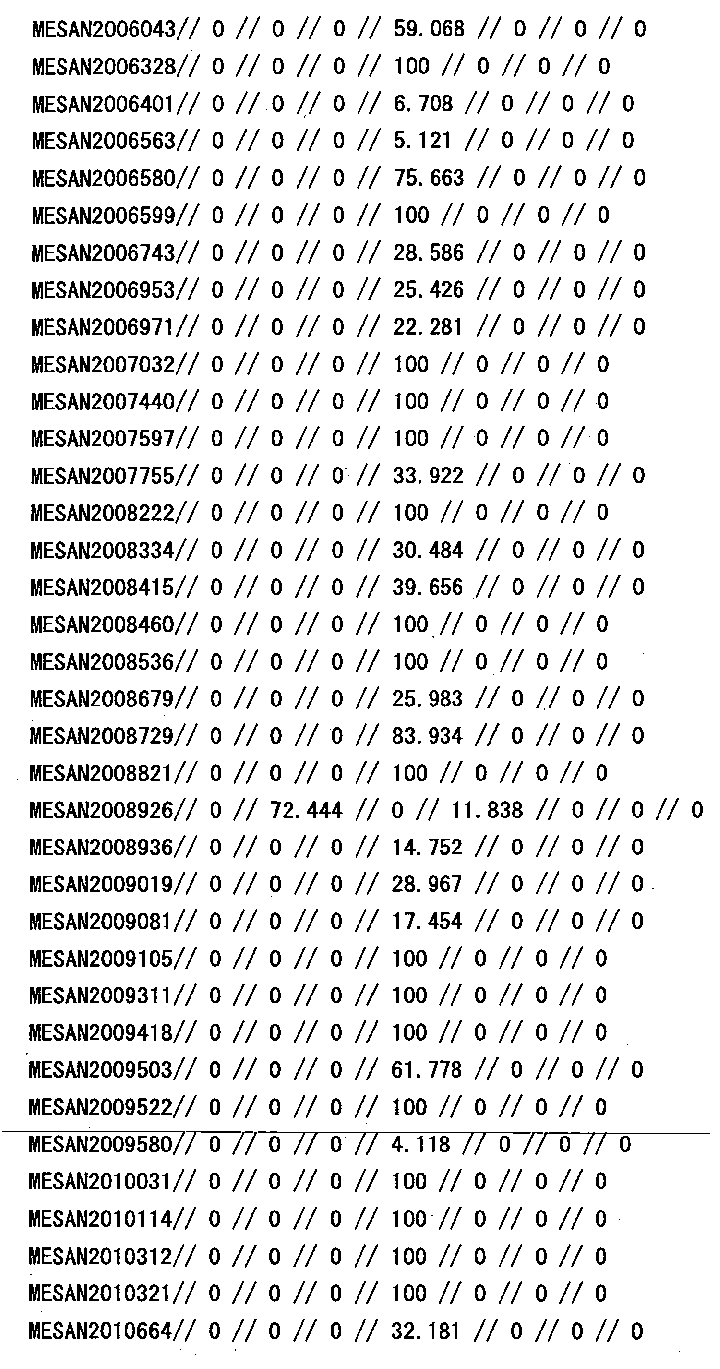

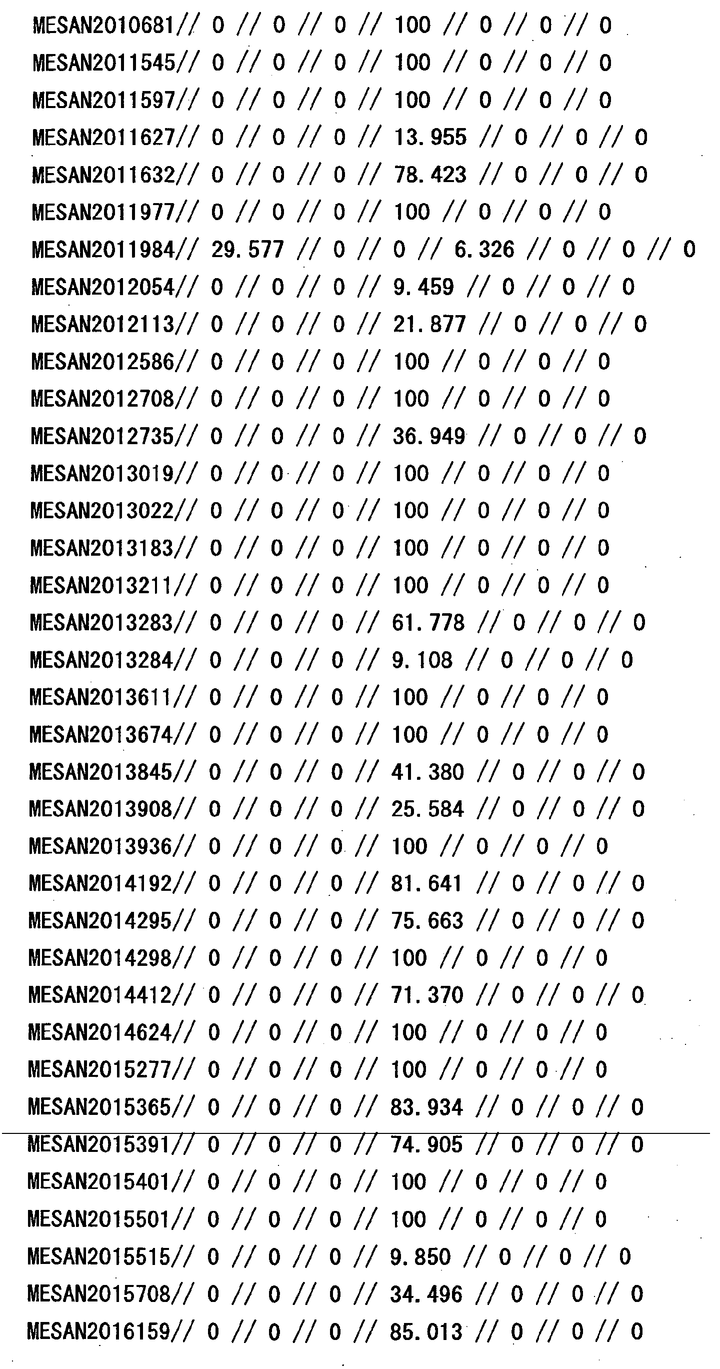

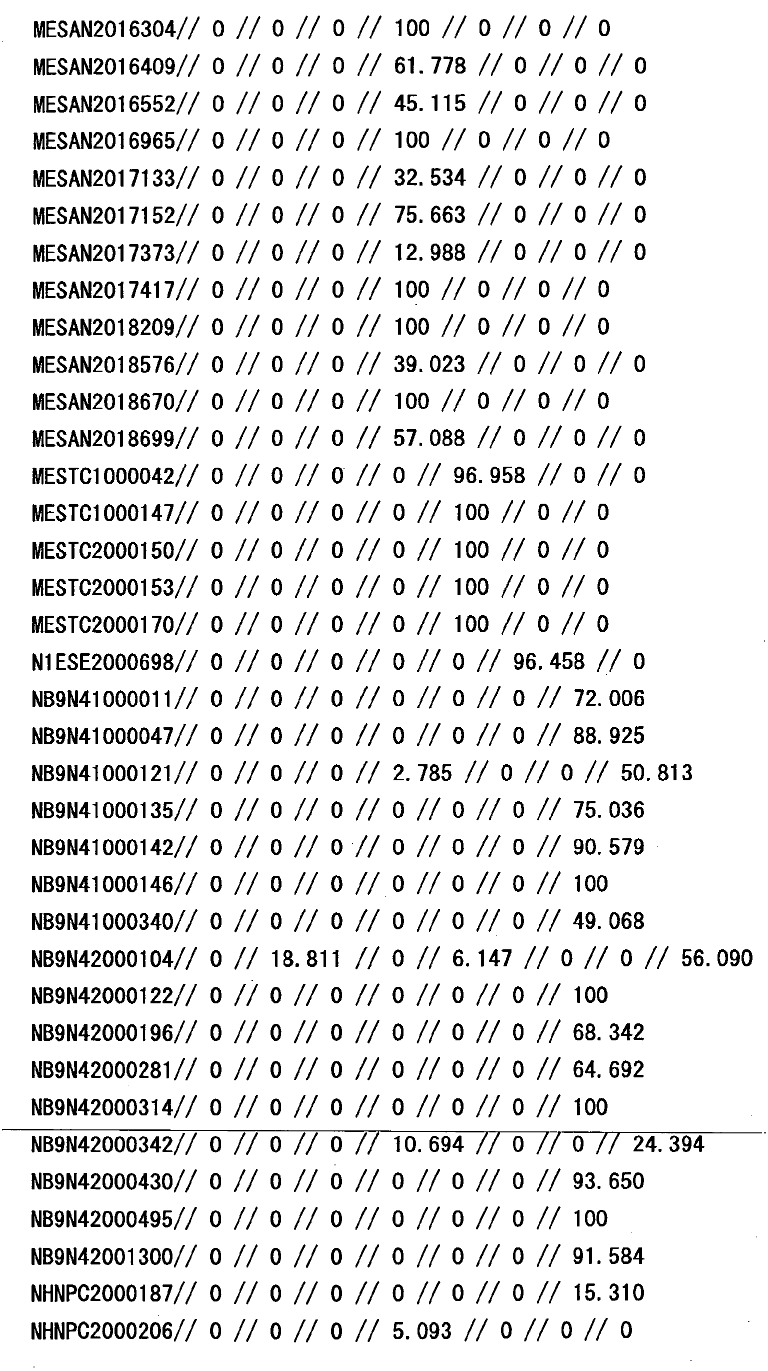

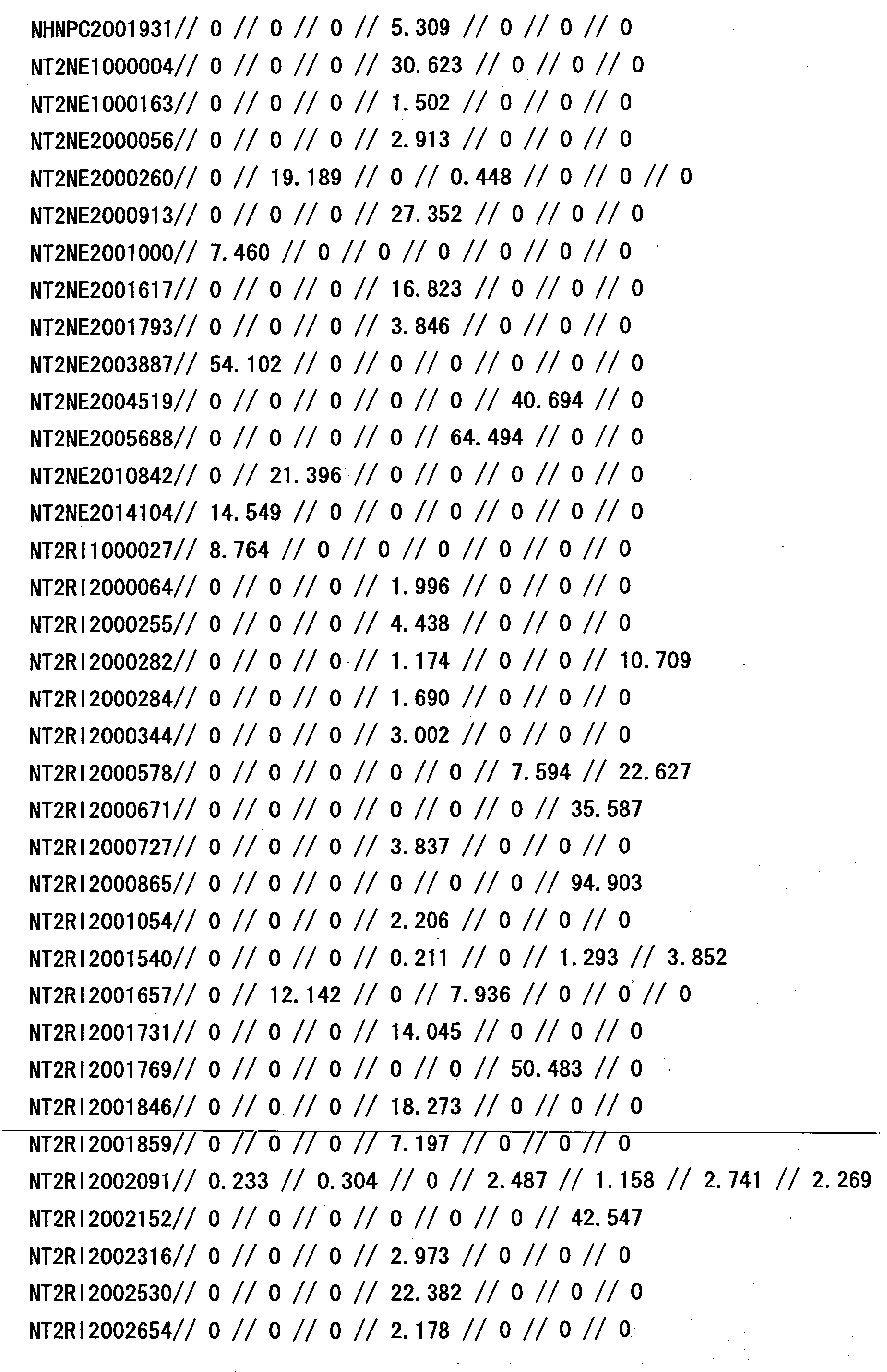

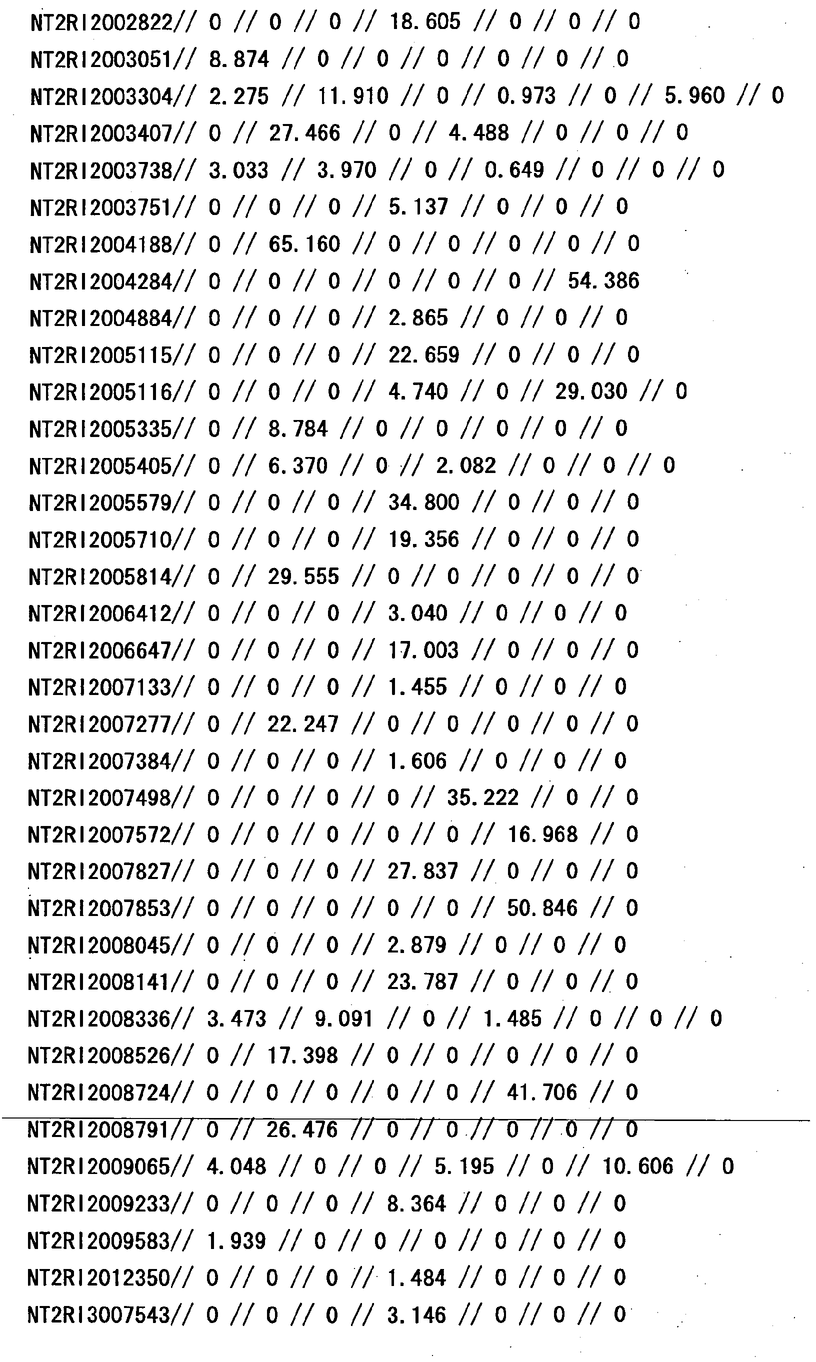

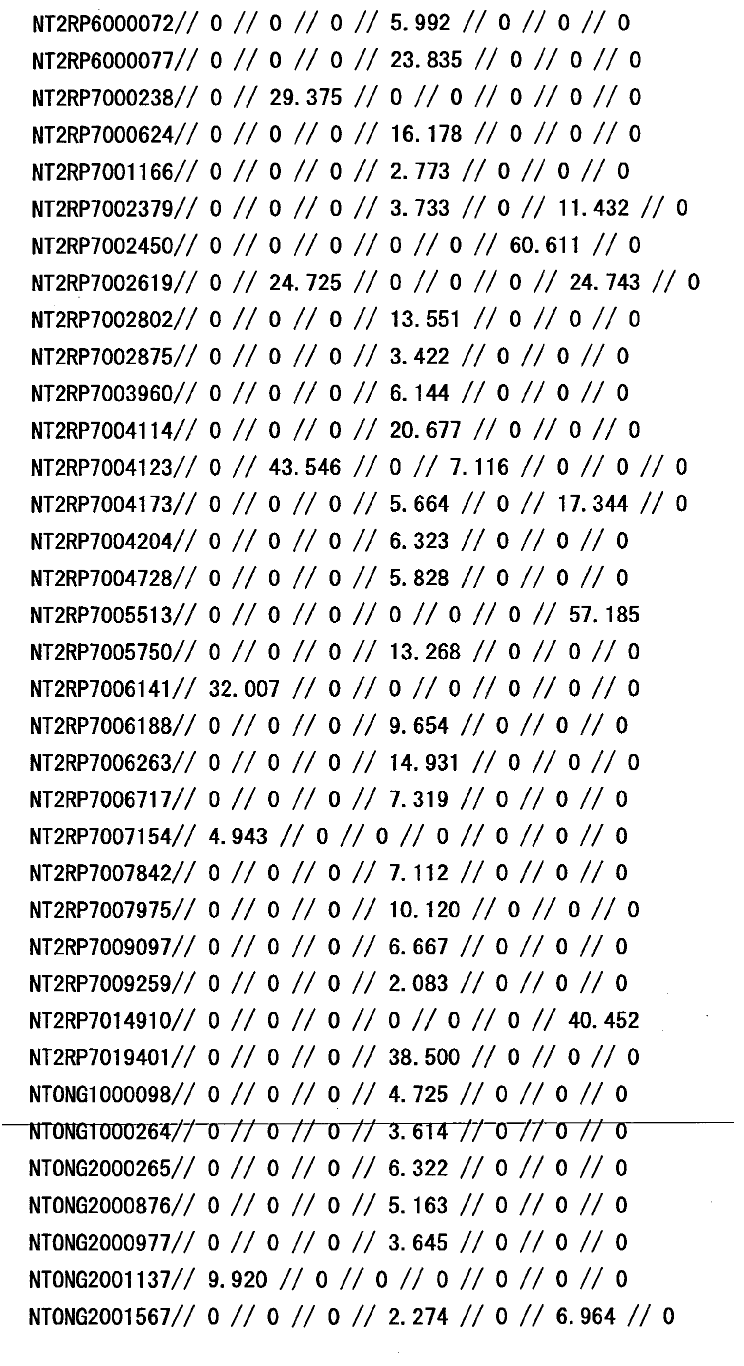

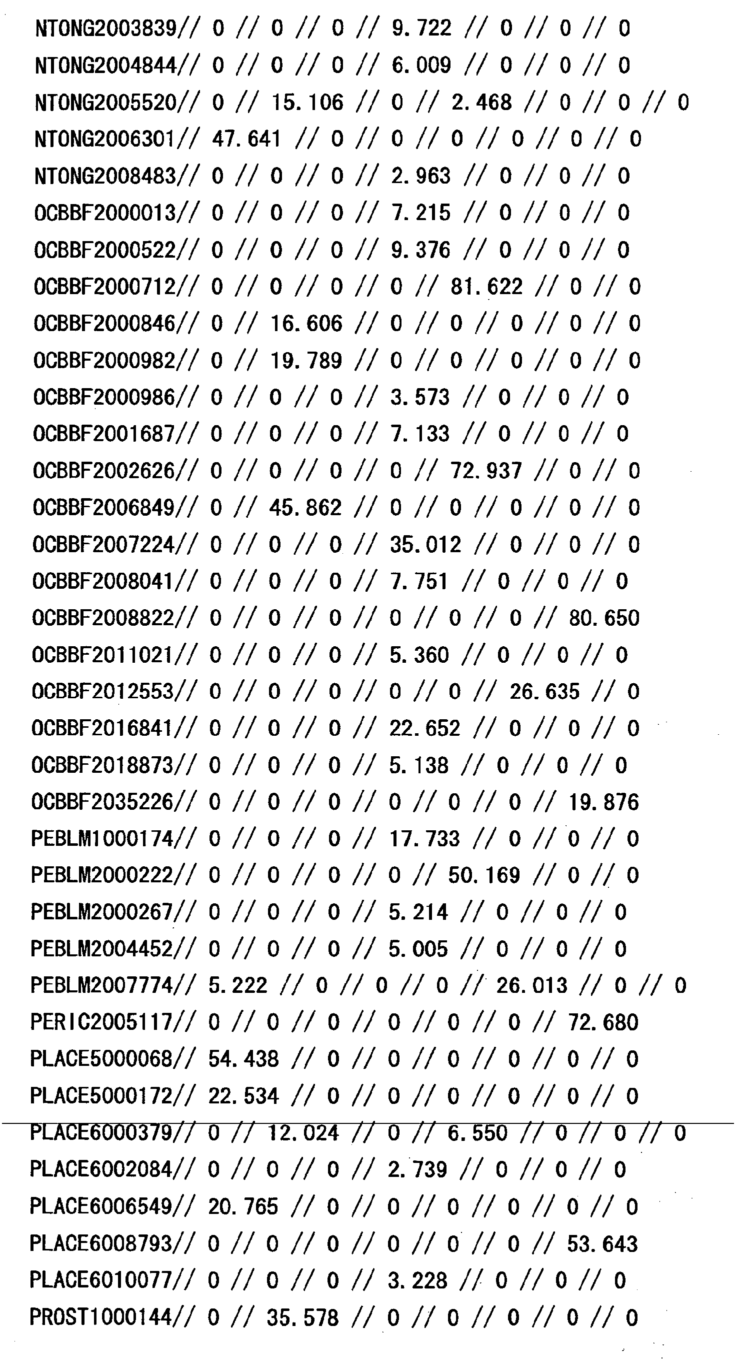

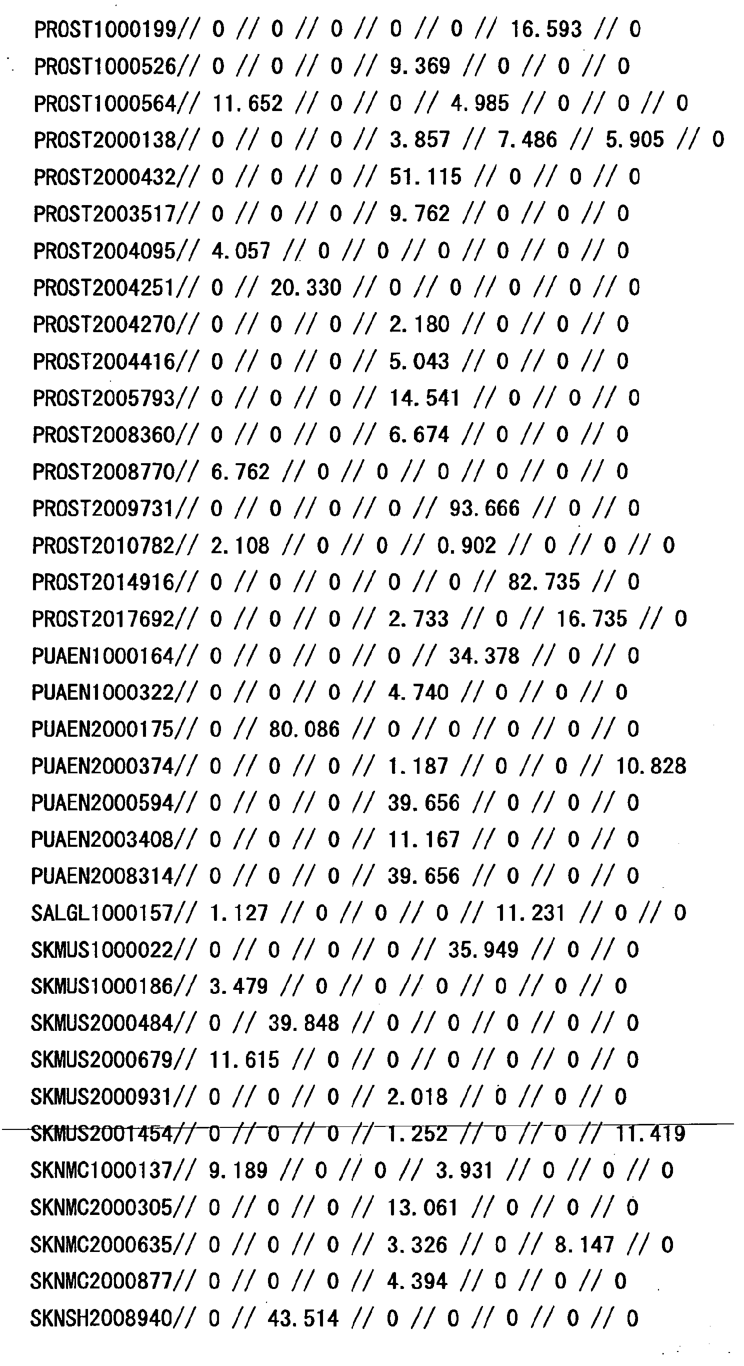

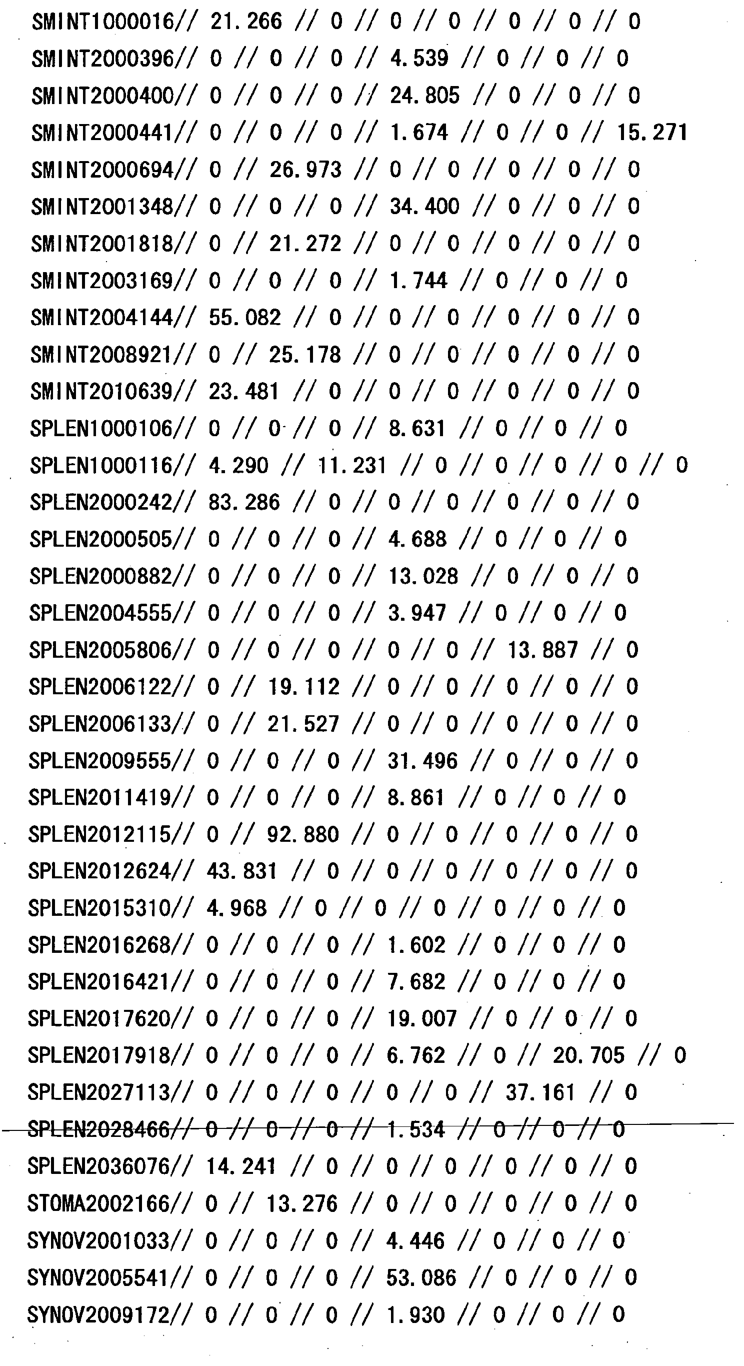

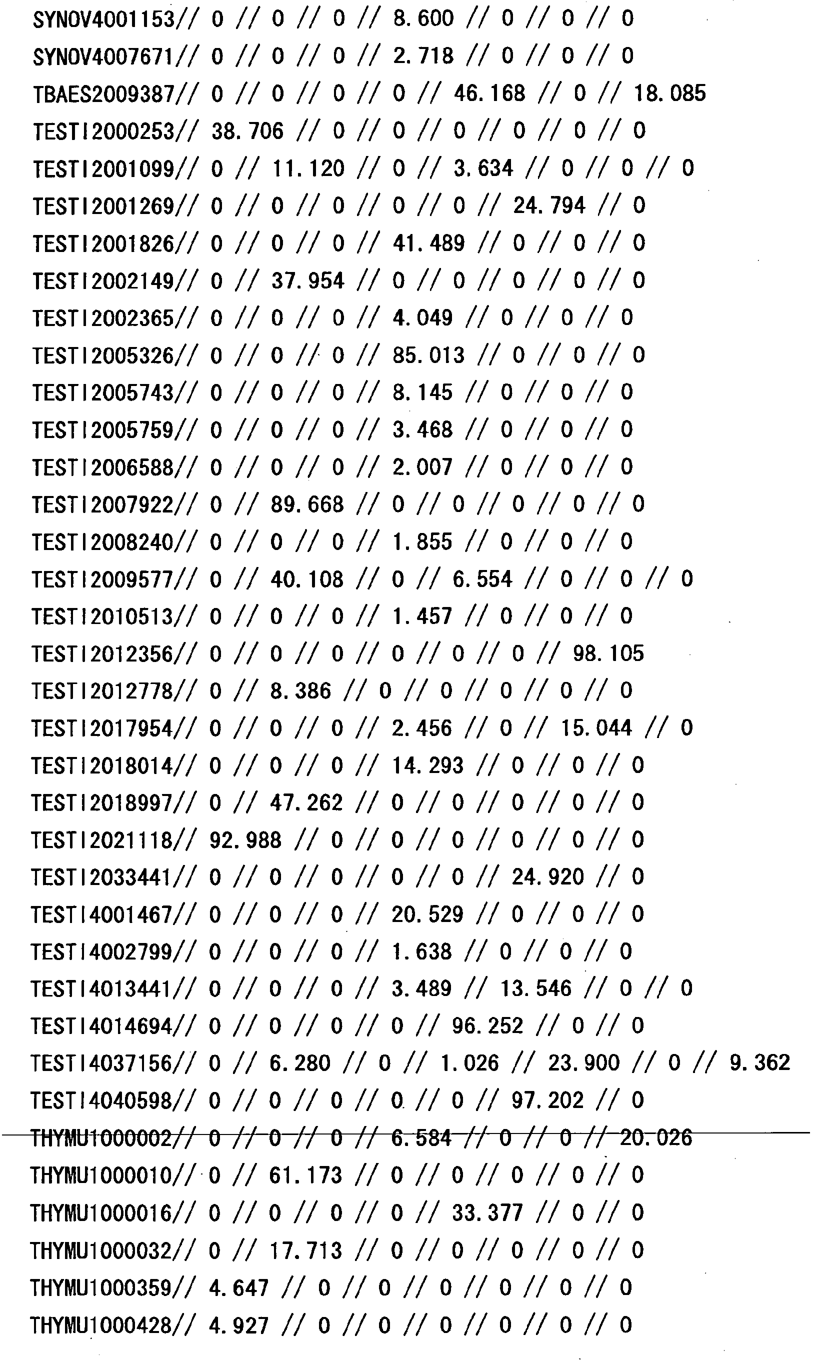

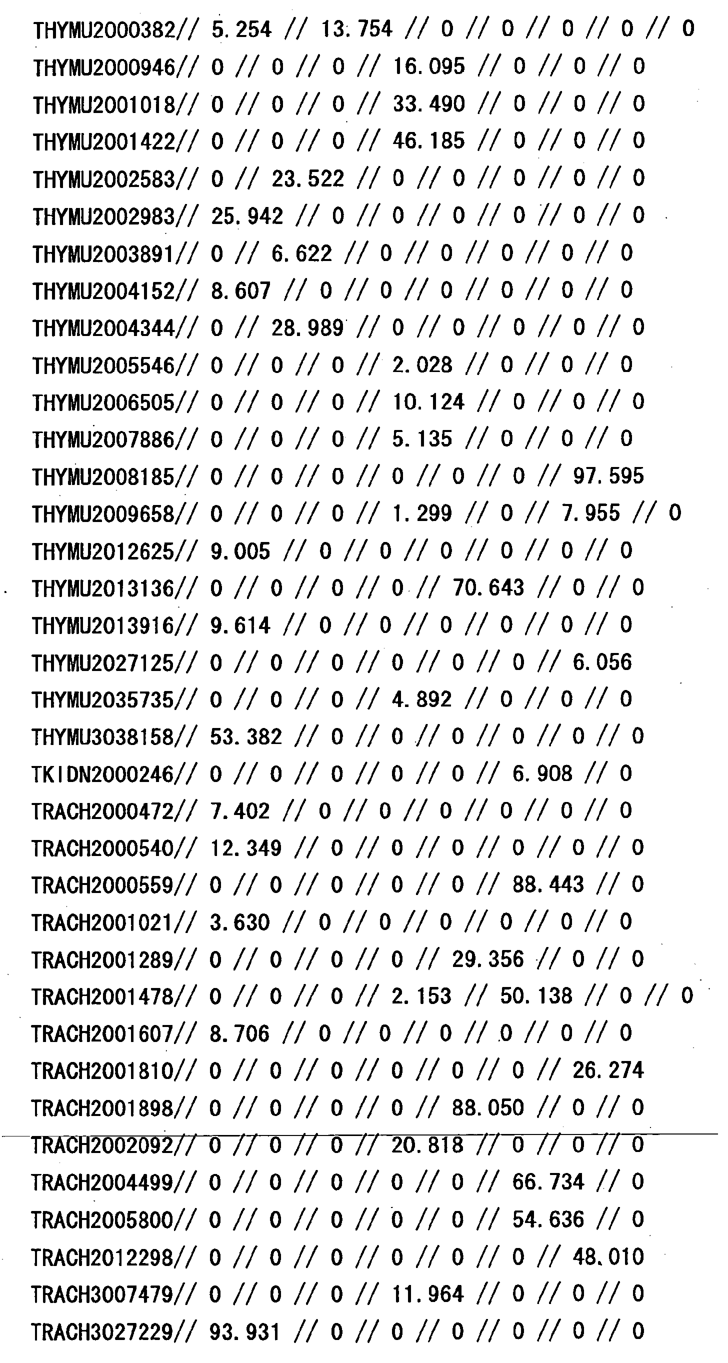

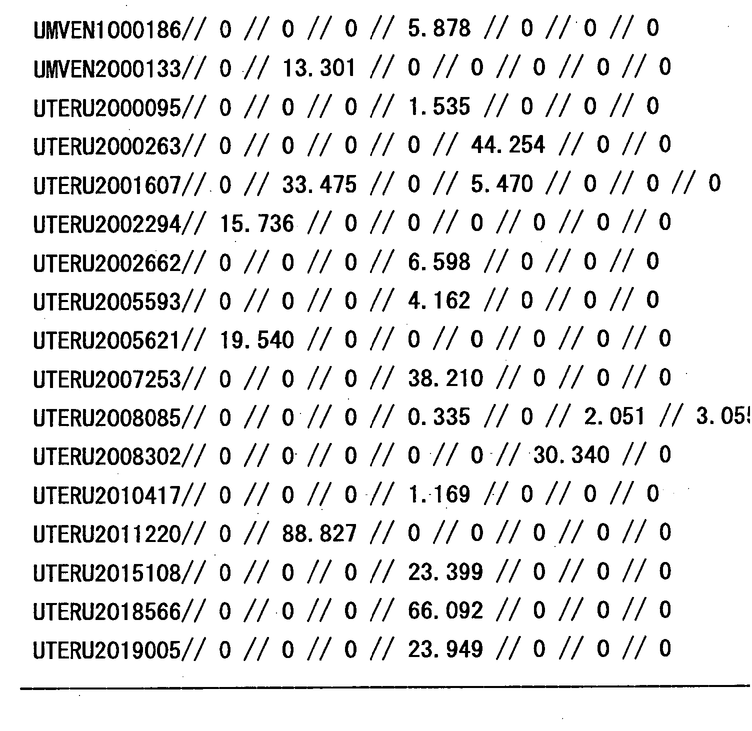

Genes whose expression levels were negligibly low in each of the above-described libraries are shown with their clone names below.
3NB691000018, 3NB691000085, 3NB691000113, 3NB691000129, 3NB691000173, 3NB691000191, 3NB692000012, 3NB692000029, 3NB692000102, 3NB692000154, 3NB692000276, 3NB692000305, 3NB692000330, 3NB692000374, 3NB692000429,3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365,
3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301,
ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817,
ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012179, AHMSC1000138, ASTRO1000018, ASTRO1000029, ASTRO1000096, ASTRO1000165, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000480, ASTRO2000482, ASTRO2000533,
ASTRO2000653, ASTRO2000662, ASTRO2000725, ASTRO2000801, ASTRO2000914, ASTRO2001001, ASTRO2001008, ASTRO2001029, ASTRO2001076, ASTRO2001088, ASTRO2001107, ASTRO2001122, ASTRO2001227, ASTRO2001249, ASTRO2001480, ASTRO2001823, ASTRO2002024, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096,
ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799,
ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015529, ASTRO2015987, ASTRO2016044, ASTRO2016114, ASTRO2016148, ASTRO2016491, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123,
BGGI11000193, BGGI11000285, BGGI12000022, BGGI12000054, BGGI12000067, BGGI12000161, BGGI12000533, BGGI12000544, BGGI12000616, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474,
BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000137, BNGH41000153, BNGH41000169,
BNGH41000177, BNGH41000190, BNGH41000198, BNGH41000216, BNGH41000235, BNGH42000130, BNGH42000132, BNGH42000405, BNGH42000532, BNGH42000675, BNGH42000739, BNGH42000791, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005235, BNGH42005504, BNGH42006135, BNGH42006226, BNGH42006234, BNGH42007037, BNGH42007366, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020,
BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000124, BRACE1000131, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441, BRACE2000487,
BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353,
BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001692, BRACE2001705, BRACE2001709, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002091, BRACE2002139,
BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002677, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834,
BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003827, BRACE2003845, BRACE2003847, BRACE2003848, BRACE2003873, BRACE2003885, BRACE2003887, BRACE2003892,
BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450, BRACE2005453, BRACE2005457, BRACE2005460, BRACE2005489, BRACE2005518, BRACE2005556, BRACE2005562, BRACE2005624,
BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006319, BRACE2006344, BRACE2006354, BRACE2006363,
BRACE2006378, BRACE2006380, BRACE2006393, BRACE2006397, BRACE2006413, BRACE2006417, BRACE2006453, BRACE2006459, BRACE2006488, BRACE2006514, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006900, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007089, BRACE2007109, BRACE2007138, BRACE2007174, BRACE2007191, BRACE2007197, BRACE2007201, BRACE2007203, BRACE2007232,
BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944,
BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883,
BRACE2008917, DRACE2008919, BRACE2008941, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957,
BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317,
BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194,
BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519,
BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549,
BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2U25333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258,
BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584,
BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600,
BRACE2039607, BRACE2039624, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045947, BRACE2045954, BRACE2046251,
BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046,
BRACE3004058, BRACE3004113, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036,
BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418,
BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594,
BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241,
BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358,
BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988,
BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009482, BRALZ2009486, BRALZ2010186, BRALZ2010842, BRALZ2011337, BRALZ2011796, BRALZ2011880, BRALZ2012050,
BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203,
BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387,
BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653,
BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480,
BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813,
BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055,
BRAMY2019111, BRAMY2019277, BRAMY2019300, BRAMY2019468, BRAMY2019509, BRAMY2019554, BRAMY2019643, BRAMY2019710, BRAMY2019963, BRAMY2019985, BRAMY2020050, BRAMY2020058, BRAMY2020106, BRAMY2020270, BRAMY2020354, BRAMY2020355,. BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493,
BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218,
BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783,
BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993,
BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214,
BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420,
BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078,
BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037,
BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000488, BRAWH2000494, BRAWH2000500, BRAWH2000503, BRAWH2000522, BRAWH2000554, BRAWH2000595, BRAWH2000636, BRAWH2000643, BRAWH2000651, BRAWH2000682, BRAWH2000686,
BRAWH2000697, BRAWH2000752, BRAWH2000859, BRAWH2000866, BRAWH2000892, BRAWH2000901, BRAWH2000924, BRAWH2000944, BRAWH2000984, BRAWH2001019, BRAWH2001037, BRAWH2001052, BRAWH2001129, BRAWH2001137, BRAWH2001141, BRAWH2001159, BRAWH2001166, BRAWH2001171, BRAWH2001186, BRAWH2001203,. BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001436, BRAWH2001438, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873,
BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842,
BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380,
BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400,
DRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595,
BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018526, BRAWH2018527, BRAWH2018601, BRAWH2018745, BRAWH2018875, BRAWH2019053, BRAWH2019055, BRAWH2019198,
BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3002853, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047,
BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662,
BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172,
BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448,
BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223,
BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003944, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894,
BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566,
BRCAN2018667, BRCAN2018748, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036, BRCAN2028040, BRCAN2028319,
BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164,
BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661,
BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172,
BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414,
BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954,
BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842,
BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022221, BRHIP2022228, BRHIP2022298, BRHIP2022326, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061,
BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114,
BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291,
BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675,
BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501,
BRHIP3027594, BRHIP3027651, BRHIP3027854, BRHIP3027947, BRHIP3028570, BRHIP3028742, BRSSN1000056, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133,
BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874,
BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532,
BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012174, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751,
BRSTN2014770, BRSTN2015015, BRSTN2015889, BRSTN2015978, BRSTN2016066, BRSTN2016335, BRSTN2016678, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726,
BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955,
BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663,
BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781,
BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252,
BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003225, BRTHA3003339, BRTHA3003343, BRTHA3003417, BRTHA3003449, BRTHA3003474, BRTHA3003490, BRTHA3003704, BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310,
BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623,
BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770,
CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000052, CTONG1000056, CTONG1000062,
CTONG1000086, CTONG1000088, CTONG1000093, CTONG1000094, CTONG1000097, CTONG1000113, CTONG1000137, CTONG1000165, CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000411, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366,
CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002095, CTONG2002143, CTONG2002270, CTONG2002395, CTONG2002417, CTONG2002418, CTONG2002453, CTONG2002520, CTONG2002558, CTONG2002590, CTONG2002709, CTONG2002721, CTONG2002744, CTONG2002766, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003298, CTONG2003348, CTONG2003350, CTONG2003361,
CTONG2003372, CTONG2003375, CTONG2003427, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005278, CTONG2005290, CTONG2005374, CTONG2005399, CTONG2005468, CTONG2005567, CTONG2005615, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393,
CTONG2006412, CTONG2006515, CTONG2006524, CTONG2006562, CTONG2006568, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007500, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008506,
CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133,
CTONG2010148, CTONG2010276, CTONG2010330, CTONG2010408, CTONG2010508, CTONG2010566, CTONG2010623, CTONG2010649, CTONG2010652, CTONG2010803, CTONG2010821, CTONG2011188, CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012230, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012901, CTONG2012996,
CTONG2013128, CTONG2013149, CTONG2013156, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013934, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717,
CTONG2015804, CTONG2015815, CTONG2015866, CTONG2015914, CTONG2015953, CTONG2016056, CTONG2016185, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483,
CTONG2018629, CTONG2018632, CTONG2018637, CTONG2018652, CTONG2018653, CTONG2018655, CTONG2018735, CTONG2018739, CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019652, CTONG2019704, CTONG2019788, CTONG2019833, CTONG2020031, CTONG2020108, CTONG2020127, CTONG2020171, CTONG2020374, CTONG2020378, CTONG2020411, CTONG2020445, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2023021, CTONG2023449, CTONG2023512, CTONG2024031, CTONG2024206, CTONG2024614,
CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028124, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947,
CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009239, CTONG3009287,
CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6O5T2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106,
D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107, DFNES1000185, DFNES2000143, DFNES2000146, DFNES2000153, DFNES2000212, DFNES2000426, DFNES2000432, DFNES2000443, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383,
DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007113, DFNES2007332, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000038, FCBBF1000053, FCBBF1000061, FCBBF1000063, FCBBF1000069, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182,
FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044,
FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380, FCBBF2006418, FCBBF2006452, FCBBF2006634,
FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001470, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977,
FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453,
FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586,
FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178,
FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599,
FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308,
FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022,
FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000510, FEBRA2000532, FEBRA2000536, FEBRA2000538, FEBRA2000575, FEBRA2000656,
FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001383, FEBRA2001438,
FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682,
FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003897, FEBRA2003907, FEBRA2003930, FEBRA2004023, FEBRA2004026, FEBRA2004029, FEBRA2004042, FEBRA2004053, FEBRA2004056, FEBRA2004091, FEBRA2004110,
FEBRA2004191, FEBRA2004202, FEBRA2004219, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005701, FEBRA2005726, FEBRA2005734, FEBRA2005778, FEBRA2005787, FEBRA2005788, FEBRA2005824, FEBRA2005995, FEBRA2005998, FEBRA2006055,
FEBRA2006061, FEBRA2006092, FEBRA2006112, FEBRA2006150, FEBRA2006165, FEBRA2006255, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566,
FEBRA2007589, FEBRA2007613, FEBRA2007620, FEBRA2007622, FEBRA2007708, FEBRA2007714, FEBRA2007793, FEBRA2007818, FEBRA2007823, FEBRA2007880, FEBRA2007900, FEBRA2007901, FEBRA2007931, FEBRA2007957, FEBRA2008009, FEBRA2008036, FEBRA2008086, FEBRA2008087, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928,
FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397,
FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433,
FEBRA2018476, FEBRA2018746, FEBRA2019172, FEBRA2019242, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285,
FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325, FEHRT2000364, FEKID1000018, FELIV1000101, FELIV1000137, FELIV1000164,
FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000131, HCHON1000142,
HCHON1000166, HCHON1000176, HCHON2000038, HCHON2000062, HCHON2000087, HCHON2000100, HCHON2000156, HCHON2000160, HCHON2000199, HCHON2000212, HCHON2000265, HCHON2000271, HCHON2000315, HCHON2000322, HCHON2000323, HCHON2000344, HCHON2000395, HCHON2000417, HCHON2000418, HCHON2000473, HCHON2000475, HCHON2000503, HCHON2000626, HCHON2000660, HCHON2000663, HCHON2000673, HCHON2000698, HCHON2000699, HCHON2000738, HCHON2000743, HCHON2000815, HCHON2000817, HCHON2000818, HCHON2000826, HCHON2000832, HCHON2000851, HCHON2000898, HCHON2000935, HCHON2000942, HCHON2000956, HCHON2000994, HCHON2001039, HCHON2001050, HCHON2001084, HCHON2001099, HCHON2001110, HCHON2001116, HCHON2001161, HCHON2001214, HCHON2001233,
HCHON2001269, HCHON2001359, HCHON2001407, HCHON2001434, HCHON2001450, HCHON2001453, HCHON2001497, HCHON2001505, HCHON2001519, HCHON2001523, HCHON2001535, HCHON2001546, HCHON2001548, HCHON2001577, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004002, HCHON2004007, HCHON2004279, HCHON2004359, HCHON2004531, HCHON2004776, HCHON2004858, HCHON2005048, HCHON2005118, HCHON2005166,
HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000173, HEART1000185, HEART2000024, HEART2000035, HEART2000099, HEART2000298, HEART2000306,
HEART2000309, HEART2000411, HEART2000418, HEART2000444, HEART2000448, HEART2000492, HEART2000506, HEART2000520, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443,
HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC1000163, HHDPC2000055, HHDPC2000070, HHDPC2000102, HHDPC2000104, HHDPC2000115, HHDPC2000149, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267,
HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000055, HLUNG1000076, HLUNG1000084, HLUNG1000091, HLUNG1000099, HLUNG1000104, HLUNG1000105, HLUNG1000110, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363,
HLUNG2000412, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001439, HLUNG2001459, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958,
HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004159, HLUNG2004170, HLUNG2004217, HLUNG2004273, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026,
HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845,
HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA1000137, HSYRA1000148, HSYRA1000178, HSYRA2000044, HSYRA2000135, HSYRA2000137, HSYRA2000190, HSYRA2000221, HSYRA2000224, HSYRA2000232,
HSYRA2000237, HSYRA2000248, HSYRA2000253, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000828, HSYRA2000832, HSYRA2000927, HSYRA2001003, HSYRA2001105, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001567, HSYRA2001580, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007338, HSYRA2007650,
HSYRA2008154, HSYRA2008714, HSYRA2009102, IMR321000155, IMR321000158, IMR321000161, IMR321000165, IMR321000193, IMR321000207, IMR321000210, IMR321000230, IMR321000242, IMR321000266, IMR322000010, IMR322000107, IMR322000127, IMR322000144, IMR322000152, IMR322000164, IMR322000178, IMR322000223, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000730, IMR322000742, IMR322000775, IMR322000791, IMR322000811, IMR322000819, IMR322000838, IMR322000859, IMR322000863, IMR322000917, IMR322000919, IMR322000953, IMR322000984, IMR322001018, IMR322001049, IMR322001167, IMR322001185,
IMR322001218, IMR322001245, IMR322001317, IMR322001318, IMR322001322, IMR322001332, IMR322001380, IMR322001438, IMR322001452, IMR322001491, IMR322001534, IMR322001541, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070,
JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000145, KIDNE1000152, KIDNE2000026, KIDNE2000041, KIDNE2000046, KIDNE2000051, KIDNE2000070, KIDNE2000085, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722,
KIDNE2000777, KIDNE2000801, KIDNE2000832, KIDNE2000833, KIDNE2000846, KIDNE2000867, KIDNE2000909, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002438, KIDNE2002483, KIDNE2002725, KIDNE2002768, KIDNE2002795, KIDNE2002798, KIDNE2002839, KIDNE2002845, KIDNE2002872, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373,
KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629,
KIDNE2005676, KIDNE2005781, KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2007005, KIDNE2007040, KIDNE2007077, KIDNE2007186, KIDNE2007222, KIDNE2007328, KIDNE2007352, KIDNE2007356, KIDNE2007422, KIDNE2007569, KIDNE2007810, KIDNE2007811, KIDNE2007910, KIDNE2007944, KIDNE2007954, KIDNE2008022, KIDNE2008069, KIDNE2008072, KIDNE2008117, KIDNE2008218,
KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750,
KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298,
KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071,
KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062,
NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000091, NOVAR1000102, NOVAR2000038, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000284, NT2NE2000299,
NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000575, NT2NE2000586, NT2NE2000612, NT2NE2000636, NT2NE2000658, NT2NE2000706, NT2NE2000707, NT2NE2000763, NT2NE2000802, NT2NE2000808, NT2NE2000809, NT2NE2000864, NT2NE2000909, NT2NE2000950, NT2NE2000951, NT2NE2000963, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324,
NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003185, NT2NE2003252, NT2NE2003280, NT2NE2003308, NT2NE2003309, NT2NE2003315, NT2NE2003393, NT2NE2003408,
NT2NE2003457, NT2NE2003485, NT2NE2003540, NT2NE2003569, NT2NE2003571, NT2NE2003705, NT2NE2003752, NT2NE2003921, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006458, NT2NE2006478,
NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107,
NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511,
NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007,
NT2RI2000107, NT2RI2000116, NT2RI2000117, NT2RI2000128, NT2RI2000133, NT2RI2000167, NT2RI2000173, NT2RI2000187, NT2RI2000263, NT2RI2000270, NT2RI2000276, NT2RI2000294, NT2RI2000313, NT2RI2000341, NT2RI2000348, NT2RI2000412, NT2RI2000421, NT2RI2000480, NT2RI2000575, NT2RI2000588, NT2RI2000597, NT2RI2000669, NT2RI2000685, NT2RI2000688, NT2RI2000689, NT2RI2000704, NT2RI2000738, NT2RI2000775, NT2RI2000822, NT2RI2000829, NT2RI2000841, NT2RI2000932, NT2RI2000974, NT2RI2000987, NT2RI2001010, NT2RI2001017, NT2RI2001030, NT2RI2001083, NT2RI2001091, NT2RI2001167, NT2RI2001178, NT2RI2001230, NT2RI2001235, NT2RI2001244, NT2RI2001307, NT2RI2001342, NT2RI2001385, NT2RI2001409, NT2RI2001410, NT2RI2001422,
NT2RI2001425, NT2RI2001449, NT2RI2001450, NT2RI2001474, NT2RI2001519, NT2RI2001595, NT2RI2001621, NT2RI2001624, NT2RI2001726, NT2RI2001782, NT2RI2001836, NT2RI2001866, NT2RI2001910, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447, NT2RI2002481, NT2RI2002507, NT2RI2002517, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002847, NT2RI2002852, NT2RI2002865, NT2RI2002923, NT2RI2002926, NT2RI2002958, NT2RI2002970,
NT2RI2003011, NT2RI2003019, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003419, NT2RI2003420, NT2RI2003481, NT2RI2003487, NT2RI2003556, NT2RI2003655, NT2RI2003667, NT2RI2003678, NT2RI2003695, NT2RI2003884, NT2RI2003921, NT2RI2003973, NT2RI2003993, NT2RI2004059, NT2RI2004078, NT2RI2004079, NT2RI2004093, NT2RI2004136, NT2RI2004157, NT2RI2004190, NT2RI2004209, NT2RI2004230, NT2RI2004290, NT2RI2004304, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783,
NT2RI2004818, NT2RI2004840, NT2RI2004916, NT2RI2004962, NT2RI2004984, NT2RI2004985, NT2RI2005048, NT2RI2005061, NT2RI2005069, NT2RI2005087, NT2RI2005096, NT2RI2005130, NT2RI2005150, NT2RI2005166, NT2RI2005239, NT2RI2005246, NT2RI2005315, NT2RI2005353, NT2RI2005358, NT2RI2005368, NT2RI2005382, NT2RI2005473, NT2RI2005520, NT2RI2005564, NT2RI2005621, NT2RI2005628, NT2RI2005647, NT2RI2005670, NT2RI2005713, NT2RI2005723, NT2RI2005772, NT2RI2005811, NT2RI2005816, NT2RI2005818, NT2RI2005851, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487,
NT2RI2006506, NT2RI2006553, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006856, NT2RI2006943, NT2RI2006973, NT2RI2007046, NT2RI2007048, NT2RI2007054, NT2RI2007084, NT2RI2007096, NT2RI2007116, NT2RI2007148, NT2RI2007214, NT2RI2007303, NT2RI2007334, NT2RI2007377, NT2RI2007386, NT2RI2007406, NT2RI2007439, NT2RI2007445, NT2RI2007468, NT2RI2007469, NT2RI2007507, NT2RI2007589, NT2RI2007593, NT2RI2007629, NT2RI2007723, NT2RI2007729, NT2RI2007751, NT2RI2007754, NT2RI2007877, NT2RI2007879, NT2RI2007884,
NT2RI2007891, NT2RI2007956, NT2RI2007965, NT2RI2007987, NT2RI2008007, NT2RI2008050, NT2RI2008171, NT2RI2008188, NT2RI2008204, NT2RI2008221, NT2RI2008233, NT2RI2008396, NT2RI2008455, NT2RI2008481, NT2RI2008502, NT2RI2008566, NT2RI2008598, NT2RI2008622, NT2RI2008656, NT2RI2008714, NT2RI2008749, NT2RI2008801, NT2RI2008808, NT2RI2008812, NT2RI2008841, NT2RI2008942, NT2RI2008952, NT2RI2009005, NT2RI2009037, NT2RI2009066, NT2RI2009083, NT2RI2009101, NT2RI2009103, NT2RI2009144, NT2RI2009151, NT2RI2009173, NT2RI2009194, NT2RI2009215, NT2RI2009239, NT2RI2009259, NT2RI2009269, NT2RI2009281, NT2RI2009289, NT2RI2009301, NT2RI2009402, NT2RI2009406, NT2RI2009517, NT2RI2009585, NT2RI2009595, NT2RI2009855,
NT2RI2010283, NT2RI2010508, NT2RI2010795, NT2RI2011422, NT2RI2011450, NT2RI2011683, NT2RI2011803, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2015533, NT2RI2016128, NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976,
NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171,
NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000043, NT2RP6000059, NT2RP6000060, NT2RP6000078, NT2RP6000085, NT2RP6000086, NT2RP6000100, NT2RP6000109, NT2RP6000110, NT2RP6000119, NT2RP6000123, NT2RP6000127, NT2RP6000130, NT2RP6000140, NT2RP6000150, NT2RP7000041,
NT2RP7000069, NT2RP7000076, NT2RP7000086, NT2RP7000109, NT2RP7000112, NT2RP7000173, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000812, NT2RP7000906, NT2RP7000926, NT2RP7001074, NT2RP7001080, NT2RP7001156, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002028, NT2RP7002064, NT2RP7002151, NT2RP7002163, NT2RP7002243, NT2RP7002282, NT2RP7002376, NT2RP7002449, NT2RP7002478, NT2RP7002554, NT2RP7002594, NT2RP7002603, NT2RP7002650,
NT2RP7002738, NT2RP7002779, NT2RP7002829, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7004027, NT2RP7004080, NT2RP7004196, NT2RP7004233, NT2RP7004260, NT2RP7004348, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004751, NT2RP7004766, NT2RP7004790, NT2RP7004884, NT2RP7004911, NT2RP7004915,
NT2RP7004925, NT2RP7004946, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005846, NT2RP7006033, NT2RP7006075, NT2RP7006160, NT2RP7006162, NT2RP7006223, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007177, NT2RP7007221, NT2RP7007226, NT2RP7007252, NT2RP7007269, NT2RP7007310, NT2RP7007359,
NT2RP7007381, NT2RP7007387, NT2RP7007406, NT2RP7007480, NT2RP7007504, NT2RP7007530, NT2RP7007537, NT2RP7007580, NT2RP7007594, NT2RP7007610, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008161, NT2RP7008167, NT2RP7008190, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168,
NT2RP7009215, NT2RP7009225, NT2RP7009236, NT2RP7009253, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009482, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005,
NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435,
NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000246, NTONG1000247, NTONG1000257, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000858, NTONG2000878,
NTONG2000966, NTONG2000985, NTONG2001014, NTONG2001079, NTONG2001157, NTONG2001173, NTONG2001220, NTONG2001222, NTONG2001362, NTONG2001428, NTONG2001532, NTONG2001550, NTONG2001587, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277,
NTONG2005363, NTONG2005373, NTONG2005391, NTONG2005480, NTONG2005497, NTONG2005577, NTONG2005657, NTONG2005801, NTONG2005822, NTONG2005897, NTONG2005969, NTONG2006099, NTONG2006187, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054,
OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000287, OCBBF2000323, OCBBF2000452, OCBBF2000467, OCBBF2000677, OCBBF2000703, OCBBF2000719, OCBBF2000824, OCBBF2000831, OCBBF2000904, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210, OCBBF2001252, OCBBF2001307, OCBBF2001323, OCBBF2001389,
OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001706, OCBBF2001749, OCBBF2001794, OCBBF2001910, OCBBF2001931, OCBBF2001961, OCBBF2001983, OCBBF2002015, OCBBF2002083, OCBBF2002086, OCBBF2002124, OCBBF2002161, OCBBF2002290, OCBBF2002357, OCBBF2002376, OCBBF2002615, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925, OCBBF2004038, OCBBF2004104, OCBBF2004147, OCBBF2004168, OCBBF2004235, OCBBF2004259,
OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006859,
OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007238, OCBBF2007354, OCBBF2007414, OCBBF2007415, OCBBF2007428, OCBBF2007478, OCBBF2007485, OCBBF2007520, OCBBF2007555, OCBBF2007610, OCBBF2007622, OCBBF2007756, OCBBF2007762, OCBBF2007829, OCBBF2007892, OCBBF2007931, OCBBF2007946, OCBBF2008005, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775,
OCBBF2008790, OCBBF2008814, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625,
OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883,
OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016928, OCBBF2017035, OCBBF2017055, OCBBF2017069, OCBBF2017306, OCBBF2017325, OCBBF2017398, OCBBF2017458, OCBBF2017489,
OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833,
OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708,
OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372,
OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000180, PEBLM2000030,
PEBLM2000112, PEBLM2000126, PEBLM2000147, PEBLM2000170, PEBLM2000180, PEBLM2000213, PEBLM2000248, PEBLM2000270, PEBLM2000308, PEBLM2000321, PEBLM2000326, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709,
PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259,
PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000178, PLACE5000245, PLACE5000260, PLACE5000282, PLACE5000297, PLACE5000372, PLACE5000492, PLACE5000522,
PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000371, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001064, PLACE6001118, PLACE6001153, PLACE6001185, PLACE6001262, PLACE6001281, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002102,
PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003038, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004312, PLACE6004336, PLACE6004368, PLACE6004380, PLACE6004396, PLACE6004397, PLACE6004454, PLACE6004464, PLACE6004491, PLACE6004578, PLACE6004663, PLACE6004687, PLACE6004738,
PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687,
PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156, PLACE6011211, PLACE6011260, PLACE6011334,
PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016254, PLACE6016383, PLACE6016942, PLACE6016984, PLACE6017002, PLACE6017431,
PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169,
PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000097, PROST1000110, PROST1000120, PROST1000123, PROST1000136, PROST1000145, PROST1000152, PROST1000167, PROST1000184, PROST1000215, PROST1000217, PROST1000220, PROST1000226, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480,
PROST1000485, PROST1000493, PROST1000512, PROST1000527, PROST1000528, PROST1000536, PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521,
PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303, PROST2003324, PROST2003338, PROST2003340, PROST2003396, PROST2003428, PROST2003472, PROST2003537, PROST2003563,
PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004258, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006030,
PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008447, PROST2008468, PROST2008472, PROST2008489, PROST2008516, PROST2008724, PROST2008835, PROST2008993, PROST2009022,
PROST2009208, PROST2009222, PROST2009254, PROST2009273, PROST2009320, PROST2009347, PROST2009365, PROST2009388, PROST2009400, PROST2009470, PROST2009483, PROST2009571, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542,
PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512,
PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017529, PROST2017578, PROST2017612, PROST2017617, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607,PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087,
PUAEN1000121, PUAEN1000147, PUAEN1000161, PUAEN1000175, PUAEN1000239, PUAEN1000275, PUAEN1000276, PUAEN1000288, PUAEN2000080, PUAEN2000152, PUAEN2000247, PUAEN2000312, PUAEN2000394, PUAEN2000497, PUAEN2000535, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761,
PUAEN2007044, PUAEN2007350, PUAEN2007785, PUAEN2007898, PUAEN2008123, PUAEN2008228, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000014, SKMUS1000030, SKMUS1000064, SKMUS1000067, SKMUS1000084, SKMUS1000102, SKMUS1000104, SKMUS1000110, SKMUS1000118,
SKMUS1000124, SKMUS1000129, SKMUS1000138, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980,
SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034,
SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000168, SKNMC1000229, SKNMC1000251, SKNMC1000264, SKNMC2000065, SKNMC2000097, SKNMC2000224, SKNMC2000256, SKNMC2000322, SKNMC2000356, SKNMC2000374, SKNMC2000583, SKNMC2000593, SKNMC2000612, SKNMC2000622, SKNMC2000649,
SKNMC2000698, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002768,
SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000131, SMINT1000137, SMINT1000192, SMINT2000007,
SMINT2000018, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000454, SMINT2000468, SMINT2000518, SMINT2000529, SMINT2000530, SMINT2000537, SMINT2000538, SMINT2000541, SMINT2000545, SMINT2000558, SMINT2000562, SMINT2000568, SMINT2000602, SMINT2000609, SMINT2000629, SMINT2000659, SMINT2000747, SMINT2000811, SMINT2000824, SMINT2000933, SMINT2000984, SMINT2001114, SMINT2001158, SMINT2001183, SMINT2001199, SMINT2001222, SMINT2001397, SMINT2001458, SMINT2001461, SMINT2001535, SMINT2001559, SMINT2001609, SMINT2001615, SMINT2001683,
SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880, SMINT2002882, SMINT2002884, SMINT2002899, SMINT2002974, SMINT2002976, SMINT2003003, SMINT2003074, SMINT2003128, SMINT2003150, SMINT2003317, SMINT2003340, SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004280, SMINT2004299, SMINT2004339, SMINT2004350, SMINT2004414, SMINT2004473, SMINT2004547,
SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796,
SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008960, SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369,
SMINT2010426, SMINT2010500, SMINT2010533, SMINT2010629, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721, SMINT2015006, SMINT2015294,
SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134,
SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000243, SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695,
SPLEN2000698, SPLEN2000839, SPLEN2000874, SPLEN2001135, SPLEN2001141, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002467, SPLEN2002477, SPLEN2002493, SPLEN2002662, SPLEN2002695, SPLEN2002707, SPLEN2002744,
SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869, SPLEN2005927, SPLEN2005939, SPLEN2006143, SPLEN2006232, SPLEN2006268, SPLEN2006283, SPLEN2006305, SPLEN2006325,
SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2009970, SPLEN2010004, SPLEN2010015, SPLEN2010112,
SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012175, SPLEN2012179, SPLEN2012185, SPLEN2012234, SPLEN2012453, SPLEN2012523, SPLEN2012524, SPLEN2012525, SPLEN2012571, SPLEN2012606,
SPLEN2012611, SPLEN2012619, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920,
SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840,
SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017643, SPLEN2.017687, SPLEN2017740, SPLEN2017781, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311,
SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983,
SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138,
SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081,
SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645,
SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168, STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049,
SYNOV1000118, SYNOV1000124, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001239, SYNOV2001251, SYNOV2001262, SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005673, SYNOV2005817, SYNOV2005962, SYNOV2006430,
SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989,
SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428,
TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489,
TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023,
TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179,
TESTI2000184, TESTI2000207, TESTI2000208, TESTI2000221, TESTI2000238, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049,
TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001827, TESTI2001829, TESTI2001852, TESTI2001862, TESTI2001869, TESTI2001879, TESTI2001894, TESTI2001898, TESTI2001915, TESTI2001950,
TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789,
TESTI2002802, TESTI2002806, TESTI2002840, TESTI2002866,
TESTI2002877, TESTI2002910, TESTI2002912, TESTI2002928, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376,
TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004031, TESTI2004073, TESTI2004080, TESTI2004085, TESTI2004089, TESTI2004111, TESTI2004119, TESTI2004122, TESTI2004153, TESTI2004163,
TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920 , TESTI2004929, TESTI2004936, TESTI2004941, TESTI2004971, TESTI2004982, TESTI2004993, TESTI2004941, TESTI2004971, TESTI2004982, TESTI2004993, TESTI2004994, TESTI2004999,
TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005767, TESTI2005775, TESTI2005784, TESTI2005788, TESTI2005827, TESTI2005835,
TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483,
TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007407,
TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394,
TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551,
TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396,TESTI2010400 TESTI2010409, TESTI2010572, TESTI2010587, TESTI2010591, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605,
TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231,
TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979,
TESTI2014988, TESTI2015042, TESTI2015092, TESTI2015105, TESTI2015180, TESTI2015213, TESTI2015246, TESTI2015249, TESTI2015327, TESTI2015331, TESTI2015335, TESTI2015350, TESTI2015375, TESTI2015437, TESTI2015590, TESTI2015610, TESTI2015626, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863,
TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565,
TESTI2018581, TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379,
TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203,
TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473,
TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104,
TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562,
TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342,
TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401,
TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034940, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951,
TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276,
TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750,
TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439,
TESTI2046456, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279,
TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723, TESTI3000002, TESTI3000005, TESTI4000014, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394,
TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001569, TESTI4001604, TESTI4001665,.TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889,
TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326,
TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840,
TESTI4008935, TESTI4008993, TESTI4009022, TESTI4009028, TESTI4009034, TESTI4009123, TESTI4009160, TESTI4009215, TESTI4009283, TESTI4009286, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745,
TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014801, TESTI4014818,
TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152,
TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942,
TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612,
TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633,
TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363,
TESTI4040535, TESTI4040559, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682,
TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000017, THYMU1000025, THYMU1000029, THYMU1000033, THYMU1000041, THYMU1000060, THYMU1000074, THYMU1000083, THYMU1000098, THYMU1000103, THYMU1000105, THYMU1000109, THYMU1000136, THYMU1000142,
THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231,
THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879, THYMU2000932, THYMU2000950, THYMU2000971, THYMU2000982, THYMU2000990, THYMU2001007, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443, THYMU2001515, THYMU2001521, THYMU2001556, THYMU2001622, THYMU2001727,
THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002841, THYMU2002852, THYMU2002910, THYMU2002950, THYMU2003012, THYMU2003046, THYMU2003069, THYMU2003107, THYMU2003124, THYMU2003133, THYMU2003166, THYMU2003232, THYMU2003242, THYMU2003249, THYMU2003270, THYMU2003282,
THYMU2003287, THYMU2003336, THYMU2003366, THYMU2003397, THYMU2003419, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2003981, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003,
THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006813, THYMU2006913, THYMU2006946,
THYMU2006965, THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008191, THYMU2008207, THYMU2008226, THYMU2008282, THYMU2008321, THYMU2008339, THYMU2008350, THYMU2008351,
THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779,
THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807,
THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943,
THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054,
THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572,
THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676,THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249,
THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554,
THYMU2035710, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824,
THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632,
THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717,
THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900,
THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612,
THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168,
THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062,
TKIDN1000066, TKIDN1000164, TKIDN1000171, TKIDN1000192, TKIDN2000127, TKIDN2000240, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200,
TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381,
TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047, TRACH2000079, TRACH2000081, TRACH2000083, TRACH2000148, TRACH2000185, TRACH2000196, TRACH2000235, TRACH2000237, TRACH2000243, TRACH2000248, TRACH2000250, TRACH2000259, TRACH2000272, TRACH2000282, TRACH2000287, TRACH2000289, TRACH2000306, TRACH2000312, TRACH2000321, TRACH2000336, TRACH2000359, TRACH2000393, TRACH2000411, TRACH2000420, TRACH2000461, TRACH2000496, TRACH2000497, TRACH2000502, TRACH2000522,
TRACH2000660, TRACH2000665, TRACH2000675, TRACH2000677, TRACH2000702, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001523, TRACH2001525,
TRACH2001532, TRACH2001549, TRACH2001592, TRACH2001596, TRACH2001612, TRACH2001621, TRACH2001646, TRACH2001651, TRACH2001684, TRACH2001933, TRACH2001968, TRACH2001996, TRACH2002043, TRACH2002054, TRACH2002100, TRACH2002138, TRACH2002333, TRACH2002537, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458,
TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007754, TRACH2007816, TRACH2007894, TRACH2007969, TRACH2007988, TRACH2008081,
TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965,
TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014124, TRACH2014268, TRACH2014284, TRACH2014337, TRACH2014356, TRACH2014371, TRACH2014442,
TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034,
TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306,
TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926,
TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003547, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840,
TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061,
TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942,
TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018,
TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423,
TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400,
TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063,
TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN2000046, UMVEN2000069, UMVEN2000121, UMVEN2000152, UMVEN2000354, UMVEN2000453, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000332, UTERU2000338, UTERU2000349, UTERU2000377, UTERU2000393, UTERU2000418, UTERU2000424,
UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002410, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057,
UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299, UTERU2004461, UTERU2004520, UTERU2004564, UTERU2004664, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412,
UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007267, UTERU2007444, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414,
UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703,
UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757,
UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018609, UTERU2018674, UTERU2018712, UTERU2018784, UTERU2018789, UTERU2018811, UTERU2018867, UTERU2018881, UTERU2018884, UTERU2018955, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964,
UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941,
UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530,
UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135,
UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722,
UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176,
UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the NCRRM, NCRRP, NESOP, NETRP, NHNPC, and NOVAR libraries were analyzed for expression frequency. The analysis results are described below. Each is a relative value, where the expression level over all tissues is taken as 100. The larger this value, the greater the level of expression.

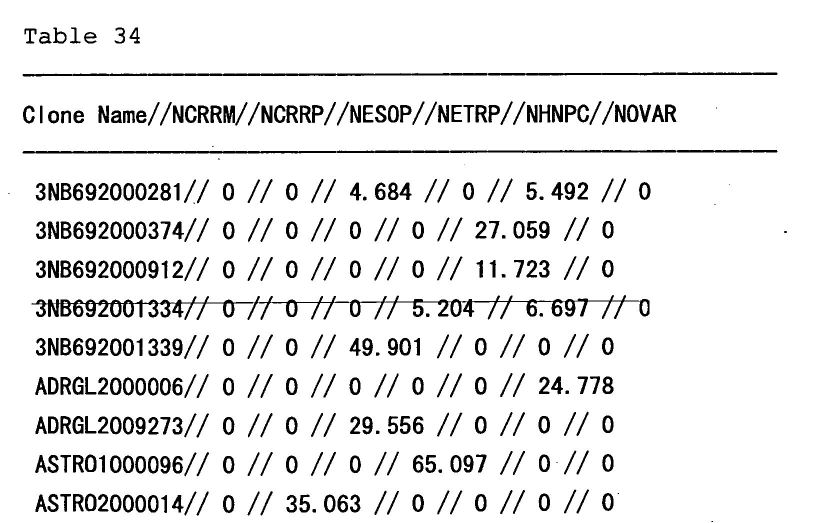

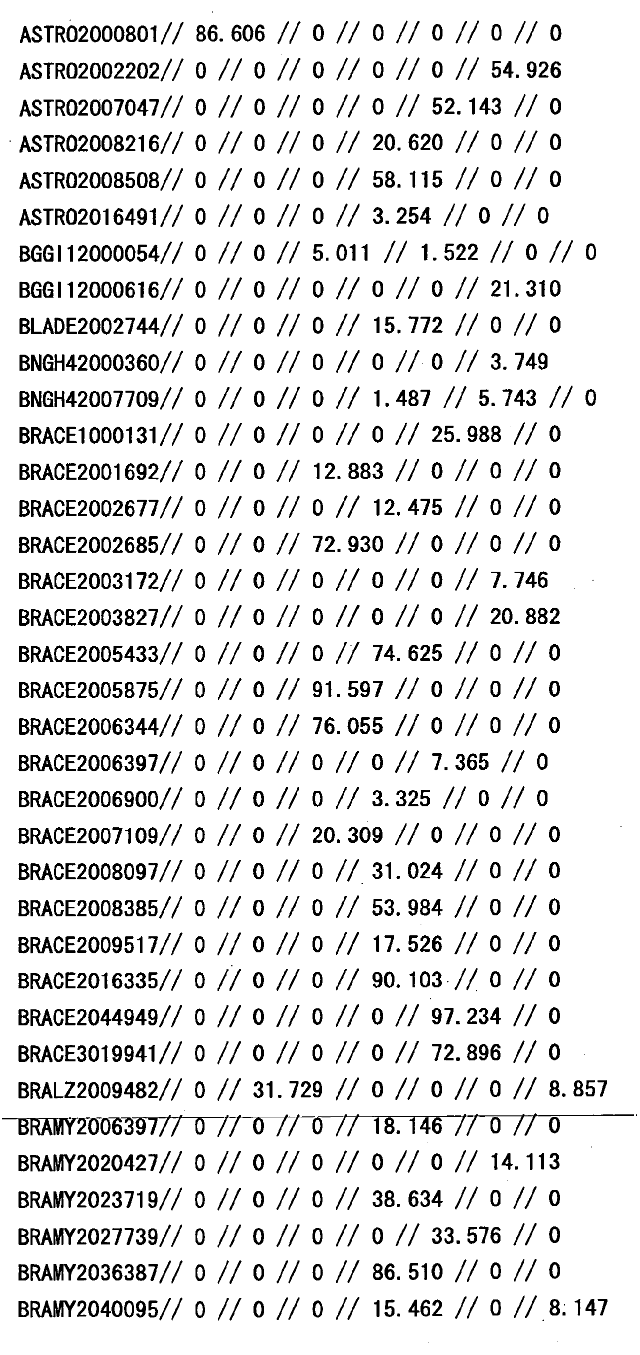

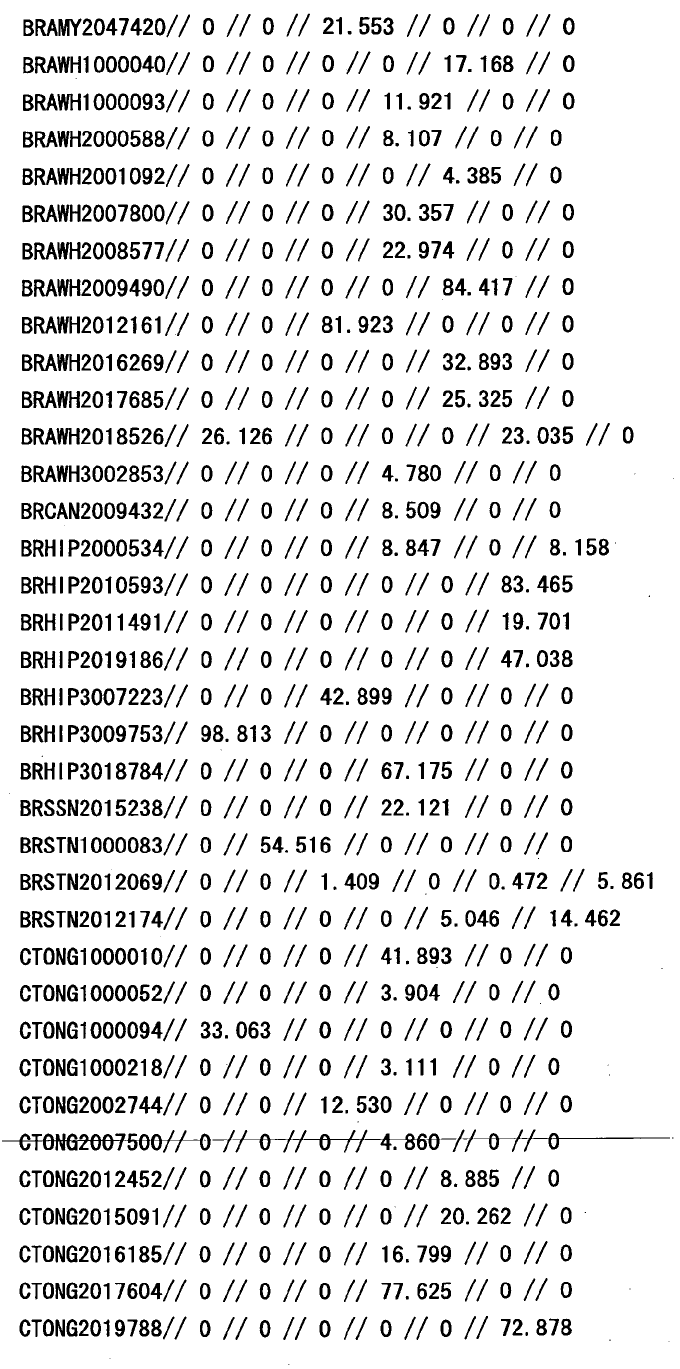

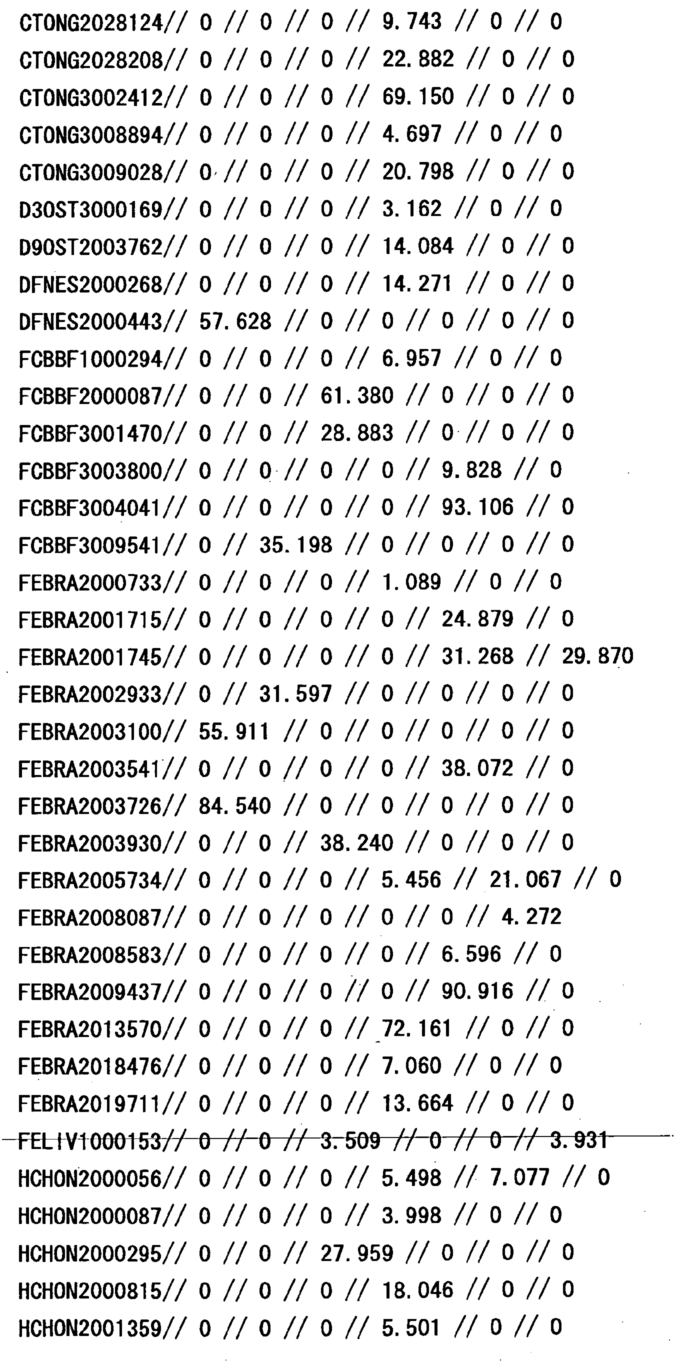

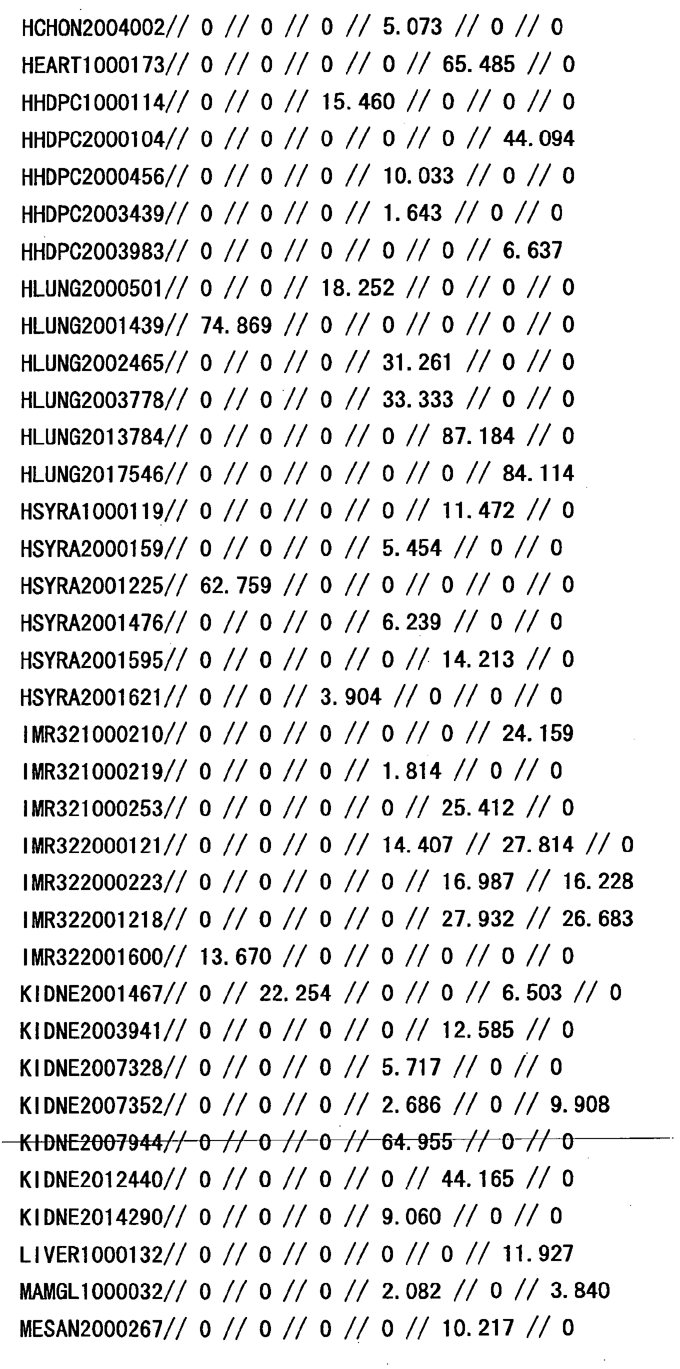

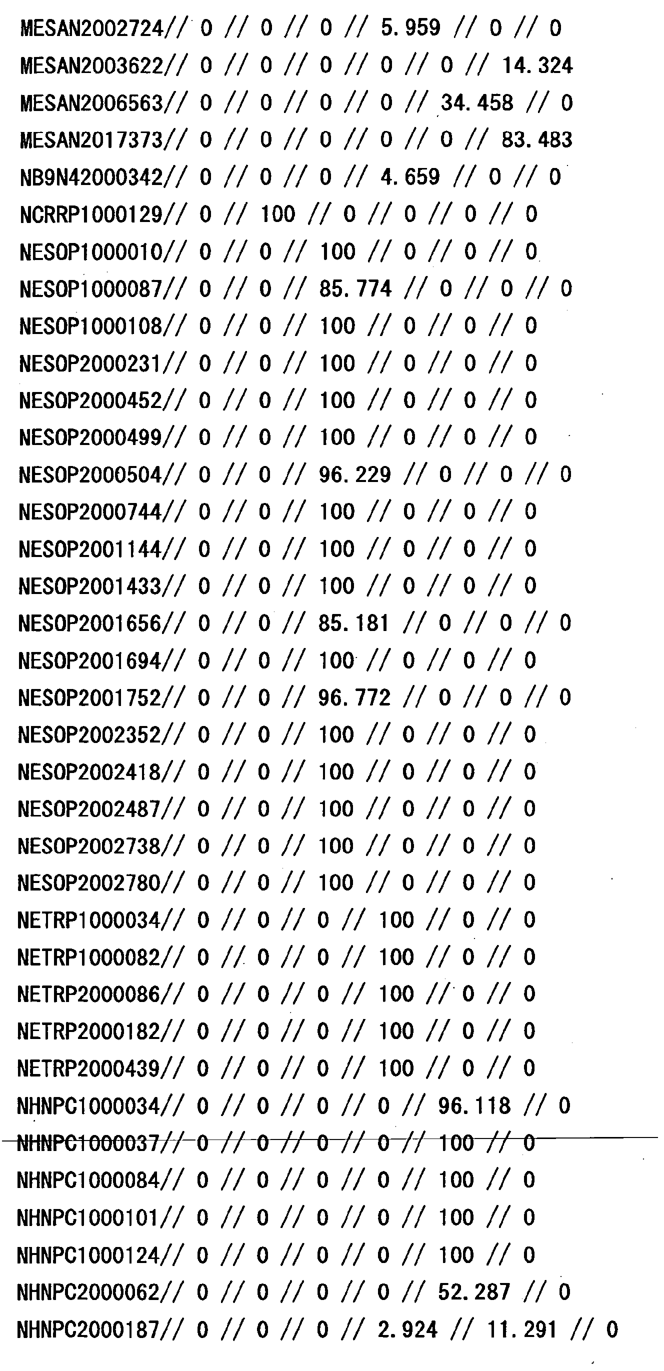

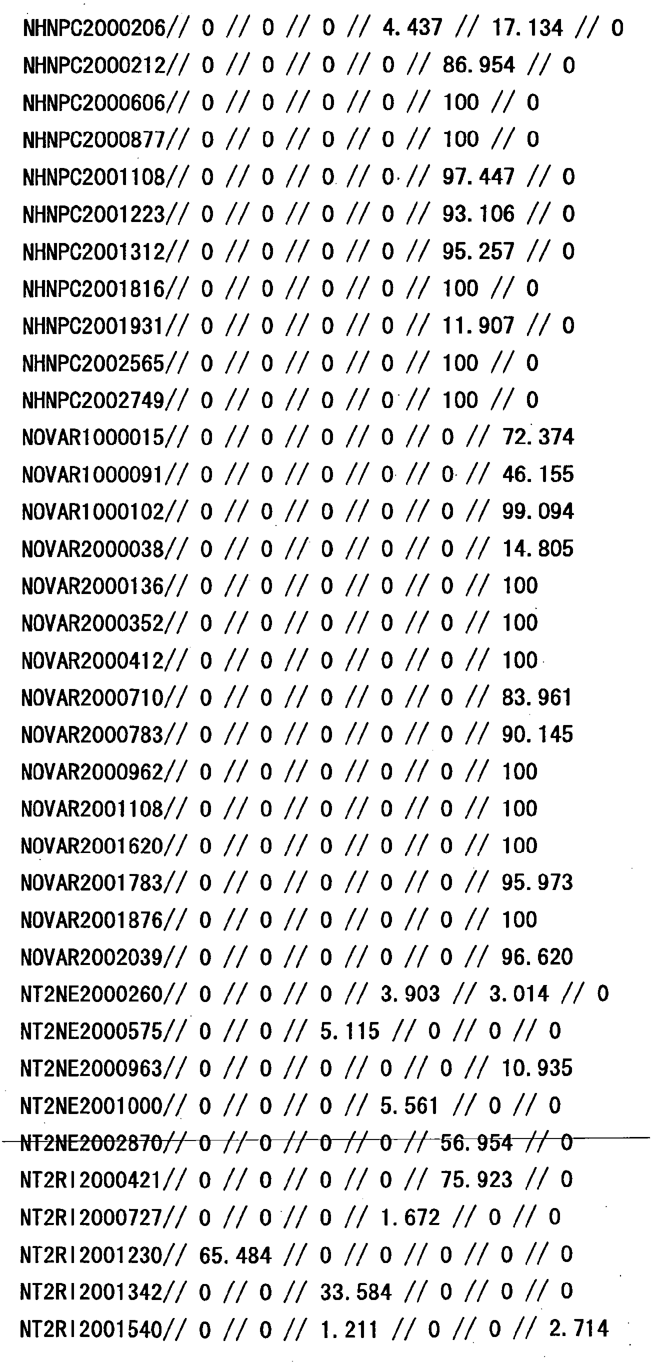

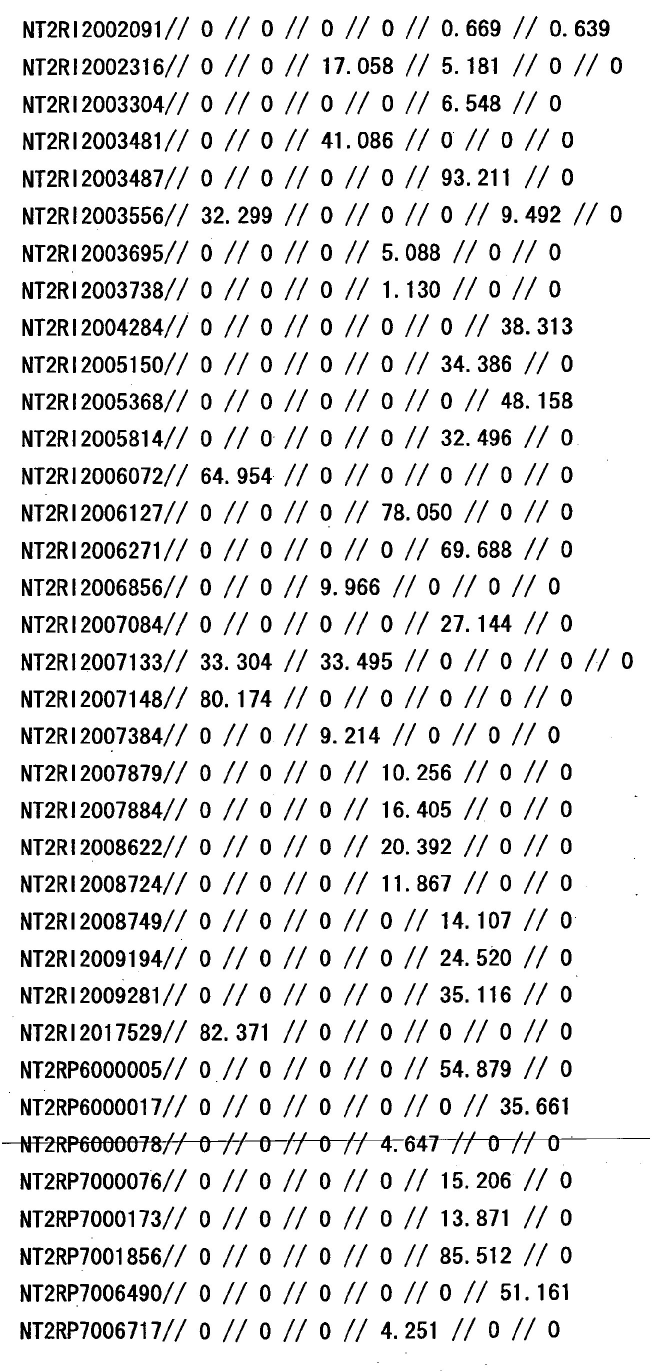

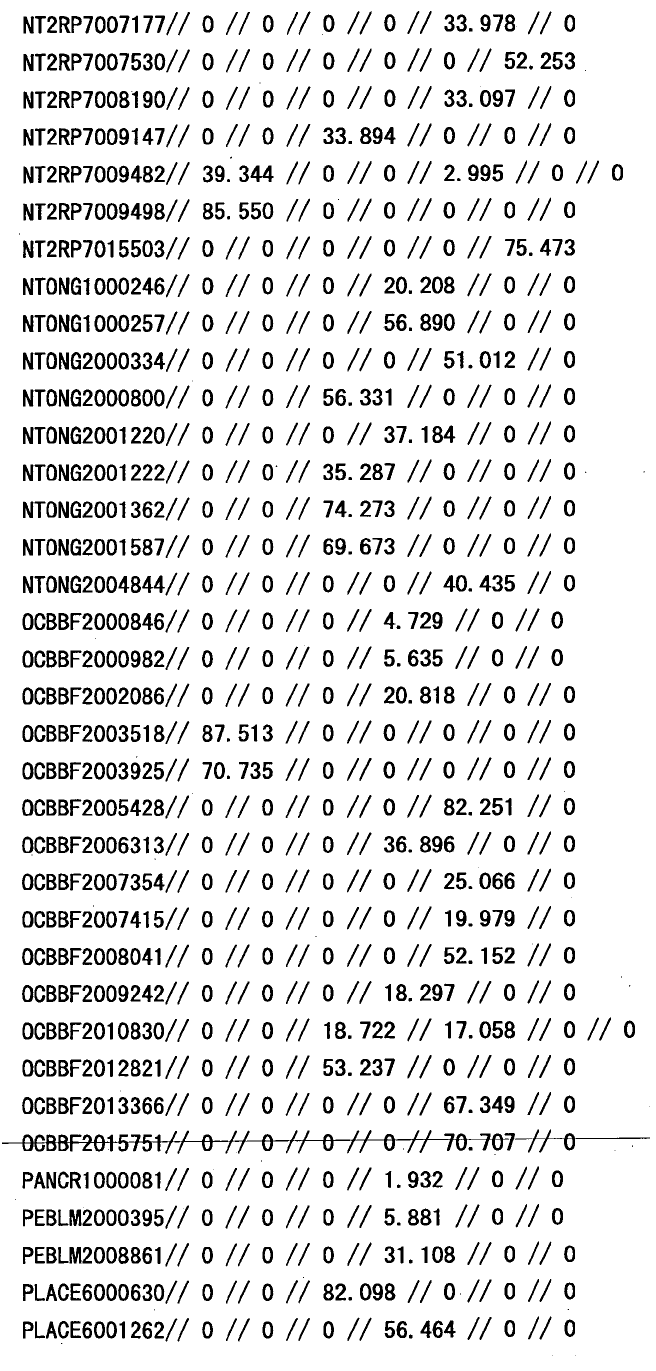

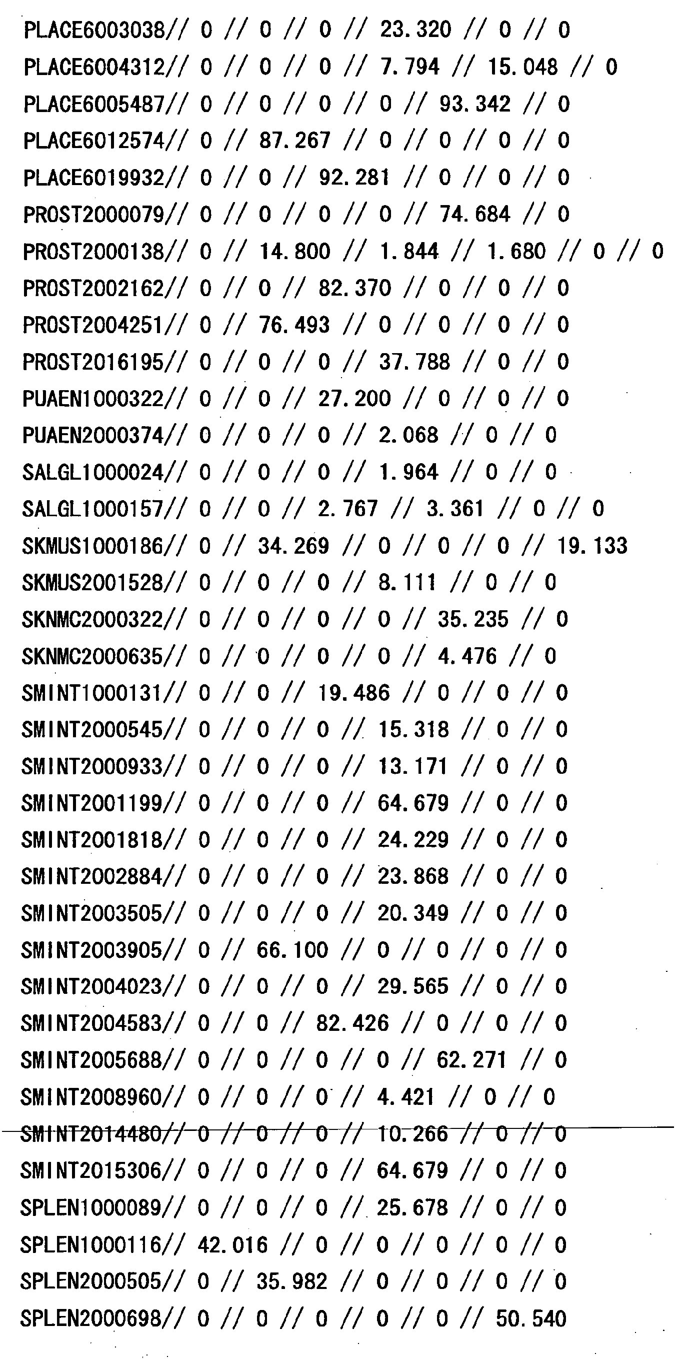

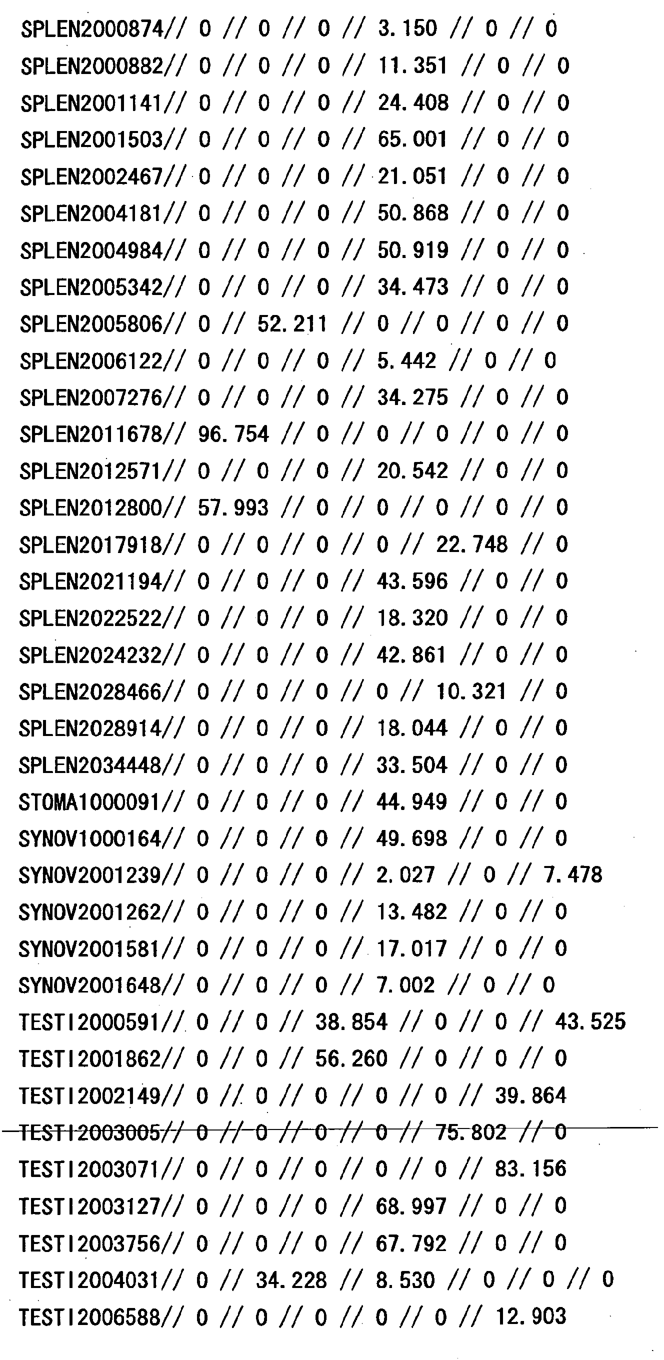

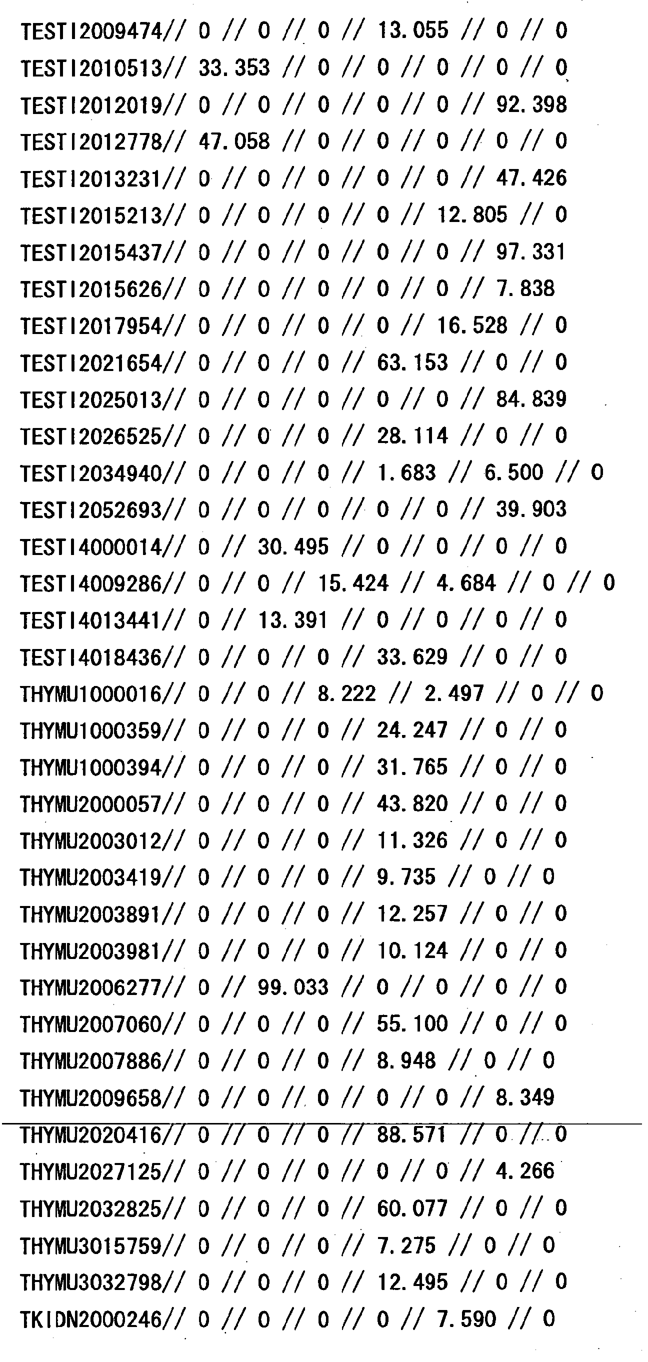

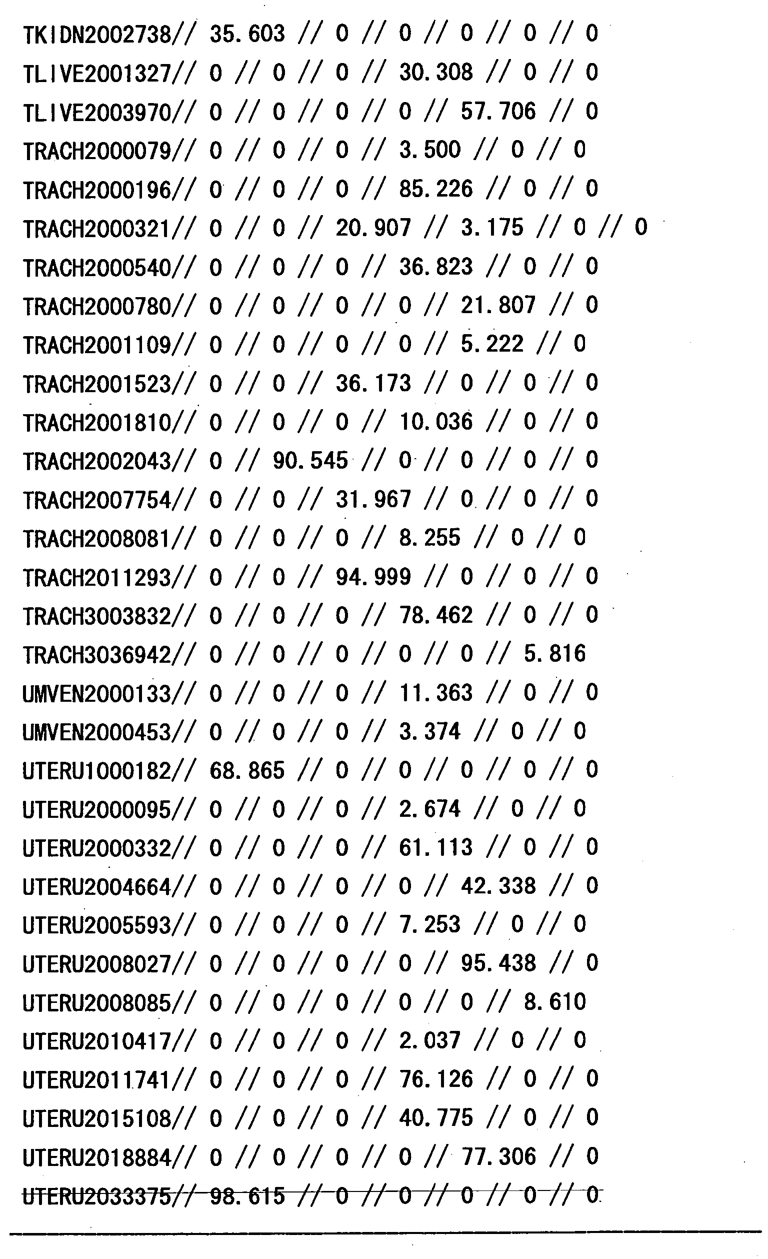

Genes whose expression levels were negligibly low in each of the above-described libraries are shown with their clone names below.
3NB691000018, 3NB691000085, 3NB691000113, 3NB691000116, 3NB691000129, 3NB691000173, 3NB691000191, 3NB692000012, 3NB692000029, 3NB692000102, 3NB692000154, 3NB692000276, 3NB692000305, 3NB692000330, 3NB692000429, 3NB692000484, 3NB692000529, 3NB692000545, 3NB692000973, 3NB692001002, 3NB692001022, 3NB692001034, 3NB692001040, 3NB692001123, 3NB692001267, 3NB692001288, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001853, 3NB692001861, 3NB692002051, 3NB692002365, 3NB692002685,
3NB692002806, 3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767,
ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000165, ASTRO2000011, ASTRO2000046, ASTRO2000095, ASTRO2000141, ASTRO2000191, ASTRO2000278, ASTRO2000372, ASTRO2000381, ASTRO2000403, ASTRO2000417, ASTRO2000480,
ASTRO2000482, ASTRO2000533, ASTRO2000653, ASTRO2000662, ASTRO2000725, ASTRO2000914, ASTRO2001001, ASTRO2001008, ASTRO2001029, ASTRO2001076, ASTRO2001088, ASTRO2001107, ASTRO2001122, ASTRO2001227, ASTRO2001249, ASTRO2001480, ASTRO2001806, ASTRO2001823, ASTRO2002024, ASTRO2002035, ASTRO2002064, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877,
ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557, ASTRO2005575, ASTRO2005593, ASTRO2005687, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008239, ASTRO2008409, ASTRO2008425, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072,
ASTRO2010582, ASTRO2010615, ASTRO2010799, ASTRO2010819, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013585, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172,
ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000684, BGGI12000693, BGGI12000839, BGGI12001002, BGGI12001075, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002782,
BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118,
BNGH41000137, BNGH41000153, BNGH41000169, BNGH41000177, BNGH41000190, BNGH41000198, BNGH41000216, BNGH41000235, BNGH42000130, BNGH42000132, BNGH42000405, BNGH42000532, BNGH42000675, BNGH42000739, BNGH42000791, BNGH42000815, BNGH42000994, BNGH42001247, BNGH42001406, BNGH42001576, BNGH42001724, BNGH42002168, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006135, BNGH42006226, BNGH42006234, BNGH42007037, BNGH42007366, BNGH42007460, BNGH42007594, BNGH42007788, BNGH42007798, BNGH42008199, BNGH42008510, BNGH42008603, BNGH42008649,
BNGH42008743, BNGH42008832, BNGH42008850, BNGH42008943, BNGH42009021, BNGH42009025, BRACE1000020, BRACE1000042, BRACE1000047, BRACE1000051, BRACE1000065, BRACE1000070, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331,
BRACE2000332, BRACE2000347, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001151, BRACE2001154, BRACE2001188, BRACE2001191, BRACE2001197, BRACE2001236, BRACE2001239,
BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972,
BRACE2001973, BRACE2002026, BRACE2002034, BRACE2002050, BRACE2002081, BRACE2002091, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002441, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002661, BRACE2002676, BRACE2002682, BRACE2002695, BRACE2002707, BRACE2002736, BRACE2002752, BRACE2002755, BRACE2002762, BRACE2002772,
BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802, BRACE2003825, BRACE2003845,
BRACE2003846, BRACE2003847, BRACE2003848, BRACE2003873, BRACE2003885, BRACE2003887, BRACE2003892, BRACE2003904, BRACE2003905, BRACE2003928, BRACE2003944, BRACE2003954, BRACE2003982, BRACE2003987, BRACE2005034, BRACE2005047, BRACE2005063, BRACE2005083, BRACE2005087, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005434, BRACE2005448, BRACE2005450, BRACE2005453, BRACE2005457,
BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281,
BRACE2006298, BRACE2006319, BRACE2006354, BRACE2006363, BRACE2006378, BRACE2006380, BRACE2006393, BRACE2006413, BRACE2006417, BRACE2006453, BRACE2006459, BRACE2006488, BRACE2006514, BRACE2006534, BRACE2006537, BRACE2006540, BRACE2006547, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007089, BRACE2007138, BRACE2007174, BRACE2007191, BRACE2007197, BRACE2007201, BRACE2007203,
BRACE2007232, BRACE2007254, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007518, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902,
BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008653, BRACE2008655, BRACE2008656, BRACE2008706, BRACE2008708, BRACE2008710, BRACE2008754, BRACE2008797, BRACE2008847, BRACE2008857, BRACE2008861,
BRACE2008867, BRACE2008881, BRACE2008883, BRACE2008917, BRACE2008919, BRACE2008941, BRACE2008948, BRACE2008960, BRACE2008968, BRACE2008999, BRACE2009014, BRACE2009016, BRACE2009031, BRACE2009037, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732,
BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062,
BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741,
BRACE2015756, BRACE2015969, BRACE2015995, BRACE2016058, BRACE2016194, BRACE2016315, BRACE2016325, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327,
BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022389,
BRACE2022394, BRACE2022435, BRACE2022448, BRACE2022450, BRACE2022549, BRACE2022633, BRACE2022638, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836,
BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385,
BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286,
BRACE2039297, BRACE2039327, BRACE2039362, BRACE2039475, BRACE2039600, BRACE2039607, BRACE2039624, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2045300, BRACE2045428, BRACE2045445,
BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413,
BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480,
BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010428, BRACE3010435, BRACE3010438, BRACE3010702, BRACE3010862, BRACE3011271, BRACE3011421, BRACE3011447, BRACE3011505,
BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580,. BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611,
BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630; BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256,
BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012,
BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018,
BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486,
BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000585, BRAMY2000653, BRAMY2000814,
BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031,
BRAMY2004058, BRAMY2004183, BRAMY2004335, BRAMY2004351, BRAMY2004352, BRAMY2004363, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458,
BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064,
BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019,
BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273,
BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982,
BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711,
BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282,
BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506,
BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300,
BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489,
BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY300212, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919,
BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652,
BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000488, BRAWH2000494,
BRAWH2000500, BRAWH2000503, BRAWH2000522, BRAWH2000554, BRAWH2000595, BRAWH2000633, BRAWH2000636, BRAWH2000643, BRAWH2000651, BRAWH2000682, BRAWH2000686, BRAWH2000697, BRAWH2000752, BRAWH2000839, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001141, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001439, BRAWH2001459, BRAWH2001461,
BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025, BRAWH2003135, BRAWH2003240, BRAWH2003307, BRAWH2003355, BRAWH2003366,
BRAWH2003662, BRAWH2003689, BRAWH2003693, BRAWH2003818, BRAWH2003832, BRAWH2003948, BRAWH2003964, BRAWH2004044, BRAWH2004068, BRAWH2004078, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005661, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308, BRAWH2007406,
DRAWH2007550, BRAWH2007570, BRAWH2007591, BRAWH2007605, BRAWH2007658, BRAWH2007664, BRAWH2007808, BRAWH2007825, BRAWH2007862, BRAWH2007890, BRAWH2008058, BRAWH2008128, BRAWH2008219, BRAWH2008255, BRAWH2008292, BRAWH2008297, BRAWH2008366, BRAWH2008380, BRAWH2008540, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536,
BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014188,
BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, DRAWH2017103, BRAWH2017250, BRAWH2017294, BRAWH2017304, BRAWH2017305, BRAWH2017379, BRAWH2017407, BRAWH2017433, BRAWH2017523, BRAWH2017534,
BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884,
BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556,
BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168,
BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003944, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117,
BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402,
BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368,
BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036, BRCAN2028040, BRCAN2028319, BRCAN2028338, BRCAN2028355, BRCAN2028378, BRCAN2028460, BRCAN2028545, BRCAN2028634, BRCAN2028702, BRCOC1000040, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756,
BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229,
BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506,
BRHIP2000532, BRHIP2000553, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800,
BRHIP2006819, BRHIP2006921, BRHIP2007305, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010610, BRHIP2010723, BRHIP2010744, BRHIP2011080, BRHIP2011120, BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750,
BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047,
BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869,
BRHIP2023888, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000111, BRHIP3000131,
BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307,
BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3010135, BRHIP3010289, BRHIP3010529, BRHIP3011082, BRHIP3011241, BRHIP3011269,
BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3019643, BRHIP3019824, BRHIP3019880, BRHIP3019956, BRHIP3020046, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533,
BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869,
BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011262,
BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581,
BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284,.BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923,
BRSTN2011211, BRSTN2011597, BRSTN2011688, BRSTN2011799, BRSTN2011961, BRSTN2012089, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016470, BRSTN2016678, BRSTN2016892, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855,
BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194,
BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466,
BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165,
BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917,
BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3003736,
BRTHA3004307, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011265, BRTHA3011361, BRTHA3011510, BRTHA3011892, BRTHA3012265,
BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556,
BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, CHONS2002829, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351,
COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000175, CTONG1000180, CTONG1000181, CTONG1000220, CTONG1000241, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042,
CTONG2000063, CTONG2000201, CTONG2000218, CTONG2000279, CTONG2000303, CTONG2000330, CTONG2000395, CTONG2000411, CTONG2000452, CTONG2000465, CTONG2000469, CTONG2000480, CTONG2000508, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001932, CTONG2001955, CTONG2002066, CTONG2002073, CTONG2002095, CTONG2002143, CTONG2002270, CTONG2002395, CTONG2002417,
CTONG2002418, CTONG2002453, CTONG2002520, CTONG2002558, CTONG2002590, CTONG2002709, CTONG2002721, CTONG2002766, CTONG2002803, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003298, CTONG2003348, CTONG2003350, CTONG2003361, CTONG2003372, CTONG2003375, CTONG2003427, CTONG2003517, CTONG2003524, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487,
CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005553, CTONG2005567, CTONG2005585, CTONG2005615, CTONG2005635, CTONG2005775, CTONG2005795, CTONG2005890, CTONG2005904, CTONG2005913, CTONG2006004, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006562, CTONG2006568, CTONG2006611, CTONG2006620, CTONG2006666, CTONG2006691, CTONG2006709, CTONG2006798, CTONG2006836, CTONG2006932, CTONG2006942, CTONG2006964,
CTONG2007007, CTONG2007009, CTONG2007072, CTONG2007078, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008343, CTONG2008398, CTONG2008402, CTONG2008405, CTONG2008466, CTONG2008506, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033,
CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010652, CTONG2010803, CTONG2010821, CTONG2011188,
CTONG2011429, CTONG2011474, CTONG2011676, CTONG2011716, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012847, CTONG2012879, CTONG2012901, CTONG2012996, CTONG2013128, CTONG2013149, CTONG2013156, CTONG2013178, CTONG2013222, CTONG2013284, CTONG2013339, CTONG2013348, CTONG2013352,
CTONG2013381, CTONG2013393, CTONG2013511, CTONG2013630, CTONG2013803, CTONG2013907, CTONG2013934, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217,
CTONG2016355, CTONG2016408, CTONG2016499, CTONG2016505, CTONG2016559, CTONG2016575, CTONG2016658, CTONG2016775, CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018383, CTONG2018413, CTONG2018483, CTONG2018629, CTONG2018632, CTONG2018637, CTONG2018652, CTONG2018653, CTONG2018655,
CTONG2018735, CTONG2018739, CTONG2018808, CTONG2018843, CTONG2018898, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020411, CTONG2020445, CTONG2020484, CTONG2020522, CTONG2020560, CTONG2020582, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513,
CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553, CTONG3003598,
CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009239, CTONG3009287, CTONG3009328, CTONG3009385, D3OST1000066, D3OST1000109, D3OST1000238, D3OST1000267, D3OST1000270,
D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003791, D9OST2003989, D9OST2004018,
D9OST2004073, D9OST2004417, DFNES1000003, DFNES1000083, DFNES1000107, DFNES1000150, DFNES1000185, DFNES2000011, DFNES2000143, DFNES2000146, DFNES2000153, DFNES2000212, DFNES2000292, DFNES2000335, DFNES2000426, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540, DFNES2005690,
DFNES2005766, DFNES2005779, DFNES2005875, DFNES2006346, DFNES2006944, DFNES2007113, DFNES2007299, DFNES2007332, DFNES2007634, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000025, FCBBF1000027, FCBBF1000038, FCBBF1000053, FCBBF1000061, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000197, FCBBF1000198,
FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000374, FCBBF1000376, FCBBF1000377, FCBBF1000395, FCBBF1000397, FCBBF1000403, FCBBF1000412, FCBBF1000425, FCBBF1000437, FCBBF1000449, FCBBF1000466, FCBBF1000476, FCBBF1000506, FCBBF1000509, FCBBF1000546, FCBBF1000550, FCBBF1000556, FCBBF1000563, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000591, FCBBF2000677, FCBBF2000678, FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817,
FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380,
FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000115, FCBBF3000120, FCBBF3000168, FCBBF3000175, FCBBF3000227, FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918,
FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004261, FCBBF3004323, FCBBF3004390, FCBBF3004409, FCBBF3004473, FCBBF3004502, FCBBF3004715, FCBBF3004842, FCBBF3004847, FCBBF3004955, FCBBF3005160, FCBBF3005218, FCBBF3005320, FCBBF3005330, FCBBF3005444, FCBBF3005497, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152,
FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211,
FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809,
FCBBF3015870, FCBBF3016018, FCBBF3016134, FCBBF3016178, FCBBF3016451, FCBBF3016618, FCBBF3016622, FCBBF3016644, FCBBF3016667, FCBBF3016928, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138,
FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023667, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730,
FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000446, FCBBF4000500, FCBBF4000529, FCBBF4000548, FCBBF5000041, FCBBF5000061, FCBBF5000165, FCBBF5000261, FCBBF5000325,
FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000105, FEBRA2000126, FEBRA2000129, FEBRA2000210, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452, FEBRA2000454, FEBRA2000462, FEBRA2000468,
FEBRA2000491, FEBRA2000504, FEBRA2000510, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197,
FEBRA2001217, FEBRA2001227, FEBRA2001245, FEBRA2001267, FEBRA2001294, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001752, FEBRA2001772, FEBRA2001790, FEBRA2001814, FEBRA2001828, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525,
FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003675, FEBRA2003707, FEBRA2003711, FEBRA2003750, FEBRA2003822, FEBRA2003833, FEBRA2003897, FEBRA2003907, FEBRA2003926, FEBRA2004023,
FEBRA2004026, FEBRA2004029, FEBRA2004042, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004293, FEBRA2004325, FEBRA2004329, FEBRA2004331, FEBRA2004412, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701,
FEBRA2005726, FEBRA2005752, FEBRA2005778, FEBRA2005787, FEBRA2005788, FEBRA2005824, FEBRA2005995, FEBRA2005998, FEBRA2006055, FEBRA2006061, FEBRA2006092, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200,
FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007818, FEBRA2007823, FEBRA2007880, FEBRA2007900, FEBRA2007901, FEBRA2007931, FEBRA2007957, FEBRA2008009, FEBRA2008036, FEBRA2008081, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008302, FEBRA2008311, FEBRA2008341, FEBRA2008360, FEBRA2008459, FEBRA2008468, FEBRA2008475, FEBRA2008481,
FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768,
FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013699, FEBRA2013905, FEBRA2013951, FEBRA2014010, FEBRA2014122, FEBRA2014198, FEBRA2014213, FEBRA2014297, FEBRA2014417, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223,
FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255,
FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158,
FEBRA2028222, FEBRA2028366, FEBRA2028457, FEBRA2028477, FEBRA2028503, FEBRA2028516, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359,
HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000030, HCHON1000131, HCHON1000142, HCHON1000166, HCHON1000176, HCHON1000191, HCHON2000028, HCHON2000038, HCHON2000062, HCHON2000100, HCHON2000156, HCHON2000160, HCHON2000199, HCHON2000212, HCHON2000226, HCHON2000244, HCHON2000265, HCHON2000271, HCHON2000315, HCHON2000322, HCHON2000323, HCHON2000344, HCHON2000364, HCHON2000373, HCHON2000395, HCHON2000417, HCHON2000418, HCHON2000473, HCHON2000475, HCHON2000503, HCHON2000508, HCHON2000626, HCHON2000660, HCHON2000663, HCHON2000673, HCHON2000676, HCHON2000698, HCHON2000699,
HCHON2000705, HCHON2000738, HCHON2000743, HCHON2000751, HCHON2000817, HCHON2000818, HCHON2000819, HCHON2000826, HCHON2000832, HCHON2000851, HCHON2000898, HCHON2000935, HCHON2000942, HCHON2000956, HCHON2000994, HCHON2001039, HCHON2001050, HCHON2001084, HCHON2001099, HCHON2001108, HCHON2001110, HCHON2001116, HCHON2001161, HCHON2001200, HCHON2001214, HCHON2001217, HCHON2001233, HCHON2001269, HCHON2001407, HCHON2001434, HCHON2001450, HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123,
HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871,
HCHON2008989, HCHON2009191, HCHON2009384, HCHON2009749, HCHON2009766, HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000149, HEART1000151, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440,
HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007231, HEART2007443, HEART2007696, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000118, HHDPC1000163, HHDPC2000055, HHDPC2000070, HHDPC2000102, HHDPC2000115,
HHDPC2000149, HHDPC2000258, HHDPC2000315, HHDPC2000455, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007267, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000055, HLUNG1000064, HLUNG1000076, HLUNG1000084,
HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926, HLUNG2000950, HLUNG2000955, HLUNG2000970, HLUNG2001013, HLUNG2001126, HLUNG2001144, HLUNG2001146, HLUNG2001214, HLUNG2001459,
HLUNG2001507, HLUNG2001518, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154,
HLUNG2004159, HLUNG2004170, HLUNG2004217, HLUNG2004273, HLUNG2004388, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153,
HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008637, HLUNG2008833, HLUNG2008875, HLUNG2008885, HLUNG2008958, HLUNG2008959, HLUNG2009067, HLUNG2009215, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013851, HLUNG2014262, HLUNG2014288, HLUNG2014449, HLUNG2014821, HLUNG2015184,
HLUNG2015418, HLUNG2015548, HLUNG2015578, HLUNG2015617, HLUNG2016022, HLUNG2016862, HLUNG2017262, HLUNG2017286, HLUNG2017307, HLUNG2017350, HLUNG2017806, HLUNG2018029, HLUNG2018282, HLUNG2018626, HLUNG2019058, HSYRA1000062, HSYRA1000131, HSYRA1000137, HSYRA1000148, HSYRA1000152, HSYRA1000178, HSYRA2000044, HSYRA2000135, HSYRA2000137, HSYRA2000190, HSYRA2000221, HSYRA2000224, HSYRA2000232, HSYRA2000237, HSYRA2000248, HSYRA2000253, HSYRA2000255, HSYRA2000332, HSYRA2000371, HSYRA2000424, HSYRA2000507, HSYRA2000510, HSYRA2000605, HSYRA2000629, HSYRA2000640, HSYRA2000706, HSYRA2000743, HSYRA2000760, HSYRA2000787, HSYRA2000792, HSYRA2000828, HSYRA2000832, HSYRA2000925, HSYRA2000927, HSYRA2001003,
HSYRA2001103, HSYRA2001105, HSYRA2001138, HSYRA2001142, HSYRA2001153, HSYRA2001232, HSYRA2001255, HSYRA2001332, HSYRA2001353, HSYRA2001396, HSYRA2001420, HSYRA2001484, HSYRA2001552, HSYRA2001567, HSYRA2001574, HSYRA2001580, HSYRA2001615, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000193, IMR321000207, IMR321000230, IMR321000242, IMR321000266, IMR322000010, IMR322000072,
IMR322000107, IMR322000127, IMR322000144, IMR322000152, IMR322000164, IMR322000178, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000730, IMR322000742, IMR322000775, IMR322000791, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001245, IMR322001317, IMR322001318, IMR322001322, IMR322001332, IMR322001380, IMR322001435, IMR322001438, IMR322001452, IMR322001491, IMR322001534,
IMR322001541, IMR322001584, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104,
KIDNE1000121, KIDNE1000143, KIDNE1000145, KIDNE1000152, KIDNE2000026, KIDNE2000041, KIDNE2000046, KIDNE2000051, KIDNE2000070, KIDNE2000085, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000330, KIDNE2000335, KIDNE2000349, KIDNE2000375, KIDNE2000383, KIDNE2000394, KIDNE2000403, KIDNE2000422, KIDNE2000493, KIDNE2000497, KIDNE2000510, KIDNE2000513, KIDNE2000517, KIDNE2000519, KIDNE2000555, KIDNE2000574, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867,
KIDNE2000909, KIDNE2000947, KIDNE2001117, KIDNE2001140, KIDNE2001162, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003305, KIDNE2003316, KIDNE2003335, KIDNE2003357, KIDNE2003373, KIDNE2003377, KIDNE2003507, KIDNE2003752,
KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005676, KIDNE2005781,
KIDNE2005798, KIDNE2005854, KIDNE2005908, KIDNE2005937, KIDNE2006009, KIDNE2006014, KIDNE2006030, KIDNE2006039, KIDNE2006053, KIDNE2006062, KIDNE2006092, KIDNE2006145, KIDNE2006149, KIDNE2006210, KIDNE2006248, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007954, KIDNE2008022, KIDNE2008048, KIDNE2008069, KIDNE2008072, KIDNE2008116, KIDNE2008117, KIDNE2008218, KIDNE2008315,
KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762,
KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013845, KIDNE2013866, KIDNE2014087, KIDNE2014112, KIDNE2014119, KIDNE2014170, KIDNE2014184, KIDNE2014268, KIDNE2014298, KIDNE2014320,
KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166,
KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158,
LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2002842, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520,
LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2007783, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000035, MAMGL1000083, MAMGL1000096, MAMGL1000100, MAMGL1000132, MAMGL1000151, MAMGL1000173, MAMGL1000178, MAMGL1000182, MAMGL1000184, MESAN1000032, MESAN1000035, MESAN1000050, MESAN1000079, MESAN1000080, MESAN1000101, MESAN1000121,
MESAN1000126, MESAN1000147, MESAN1000180, MESAN2000023, MESAN2000026, MESAN2000067, MESAN2000092, MESAN2000149, MESAN2000167, MESAN2000264, MESAN2000291, MESAN2000337, MESAN2000434, MESAN2000457, MESAN2000462, MESAN2000501, MESAN2000572, MESAN2000608, MESAN2000620, MESAN2000711, MESAN2000815, MESAN2000894, MESAN2000909, MESAN2001154, MESAN2001450, MESAN2001627, MESAN2001770, MESAN2001979, MESAN2001980, MESAN2002086, MESAN2002113, MESAN2002122, MESAN2002147, MESAN2002186, MESAN2002424, MESAN2002519, MESAN2002687, MESAN2002709, MESAN2002790, MESAN2002844, MESAN2002940, MESAN2002978, MESAN2003035, MESAN2003037, MESAN2003039, MESAN2003058, MESAN2003101, MESAN2003190, MESAN2003322, MESAN2003444,
MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005633, MESAN2005689, MESAN2005724, MESAN2005766, MESAN2005808, MESAN2005811, MESAN2005957, MESAN2005958, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522,
MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013284, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017417, MESAN2018209,
MESAN2018576, MESAN2018670, MESAN2018699, MESTC1000042, MESTC1000147, MESTC2000150, MESTC2000153, MESTC2000170, N1ESE2000698, NB9N41000011, NB9N41000047, NB9N41000121, NB9N41000135, NB9N41000142, NB9N41000146, NB9N41000340, NB9N42000104, NB9N42000122, NB9N42000196, NB9N42000281, NB9N42000314, NB9N42000430, NB9N42000495, NB9N42001300, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000163, NT2NE1000175, NT2NE1000181, NT2NE1000185, NT2NE2000038, NT2NE2000054, NT2NE2000056, NT2NE2000057, NT2NE2000064, NT2NE2000109, NT2NE2000137, NT2NE2000156, NT2NE2000174, NT2NE2000195, NT2NE2000222,
NT2NE2000259, NT2NE2000284, NT2NE2000299, NT2NE2000327, NT2NE2000353, NT2NE2000356, NT2NE2000369, NT2NE2000374, NT2NE2000383, NT2NE2000384, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000707, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247,
NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001617, NT2NE2001623, NT2NE2001626, NT2NE2001634, NT2NE2001648, NT2NE2001655, NT2NE2001660, NT2NE2001666, NT2NE2001677, NT2NE2001697, NT2NE2001698, NT2NE2001723, NT2NE2001725, NT2NE2001793, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309,
NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288,
NT2NE2006382, NT2NE2006387, NT2NE2006427, NT2NE2006432, NT2NE2006458, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654,
NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758,
NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099,
NT2RI1000016, NT2RI1000027, NT2RI1000035, NT2RI1000048, NT2RI1000127, NT2RI1000164, NT2RI1000172, NT2RI2000004, NT2RI2000007, NT2RI2000064, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000282, NT2RI2000284, NT2RI2000294, NT2RI2000313, NT2RI2000341, NT2RI2000344, NT2RI2000348, NT2RI2000412, NT2RI2000480, NT2RI2000575, NT2RI2000578, NT2RI2000588, NT2RI2000597, NT2RI2000669, NT2RI2000671, NT2RI2000685, NT2RI2000688, NT2RI2000689, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987,
NT2RI2001010, NT2RI2001017, NT2RI2001030, NT2RI2001054, NT2RI2001083, NT2RI2001091, NT2RI2001167, NT2RI2001178, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001657, NT2RI2001726, NT2RI2001731, NT2RI2001769, NT2RI2001782, NT2RI2001836, NT2RI2001846, NT2RI2001859, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002334, NT2RI2002359, NT2RI2002391, NT2RI2002447,
NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002654, NT2RI2002699, NT2RI2002729, NT2RI2002802, NT2RI2002819, NT2RI2002822, NT2RI2002847, NT2RI2002852, NT2RI2002865, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003317, NT2RI2003338, NT2RI2003344, NT2RI2003360, NT2RI2003361, NT2RI2003383, NT2RI2003392, NT2RI2003399, NT2RI2003407, NT2RI2003419, NT2RI2003420, NT2RI2003655, NT2RI2003667, NT2RI2003678, NT2RI2003751, NT2RI2003884, NT2RI2003921, NT2RI2003973,
NT2RI2003993, NT2RI2004059, NT2RI2004078, NT2RI2004079, NT2RI2004093, NT2RI2004136, NT2RI2004157, NT2RI2004188, NT2RI2004190, NT2RI2004209, NT2RI2004230, NT2RI2004290, NT2RI2004304, NT2RI2004312, NT2RI2004381, NT2RI2004398, NT2RI2004410, NT2RI2004442, NT2RI2004468, NT2RI2004535, NT2RI2004606, NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005116, NT2RI2005130, NT2RI2005166, NT2RI2005239, NT2RI2005246, NT2RI2005315, NT2RI2005335, NT2RI2005353, NT2RI2005358, NT2RI2005382, NT2RI2005405, NT2RI2005473, NT2RI2005520,
NT2RI2005564, NT2RI2005579, NT2RI2005621, NT2RI2005628, NT2RI2005647, NT2RI2005670, NT2RI2005710, NT2RI2005713, NT2RI2005723, NT2RI2005772, NT2RI2005811, NT2RI2005816, NT2RI2005818, NT2RI2005851, NT2RI2005966, NT2RI2006070, NT2RI2006071, NT2RI2006081, NT2RI2006126, NT2RI2006183, NT2RI2006257, NT2RI2006294, NT2RI2006345, NT2RI2006403, NT2RI2006412, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006825, NT2RI2006838, NT2RI2006853, NT2RI2006855, NT2RI2006943, NT2RI2006973, NT2RI2007046,
NT2RI2007048, NT2RI2007054, NT2RI2007096, NT2RI2007116, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007498, NT2RI2007507, NT2RI2007572, NT2RI2007589, NT2RI2007593, NT2RI2007629, NT2RI2007723, NT2RI2007729, NT2RI2007751, NT2RI2007754, NT2RI2007827, NT2RI2007853, NT2RI2007877, NT2RI2007891, NT2RI2007956, NT2RI2007965, NT2RI2007987, NT2RI2008007, NT2RI2008045, NT2RI2008050, NT2RI2008141, NT2RI2008171, NT2RI2008188, NT2RI2008204, NT2RI2008221, NT2RI2008233, NT2RI2008336, NT2RI2008396, NT2RI2008455, NT2RI2008481, NT2RI2008502, NT2RI2008526, NT2RI2008566, NT2RI2008598, NT2RI2008656,
NT2RI2008714, NT2RI2008791, NT2RI2008801, NT2RI2008808, NT2RI2008812, NT2RI2008841, NT2RI2008942, NT2RI2008952, NT2RI2009005, NT2RI2009037, NT2RI2009065, NT2RI2009066, NT2RI2009083, NT2RI2009101, NT2RI2009103, NT2RI2009144, NT2RI2009151, NT2RI2009173, NT2RI2009215, NT2RI2009233, NT2RI2009239, NT2RI2009259, NT2RI2009269, NT2RI2009289, NT2RI2009301, NT2RI2009402, NT2RI2009406, NT2RI2009517, NT2RI2009583, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014247, NT2RI2014551, NT2RI2014683, NT2RI2014733,
NT2RI2015239, NT2RI2015342, NT2RI2015533, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263,
NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213, NT2RI3007291, NT2RI3007443, NT2RI3007543, NT2RI3007684, NT2RI3007757, NT2RI3007878, NT2RI3007978,
NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000008, NT2RP6000032, NT2RP6000033, NT2RP6000039, NT2RP6000043, NT2RP6000059, NT2RP6000060, NT2RP6000072, NT2RP6000077, NT2RP6000085, NT2RP6000086, NT2RP6000100, NT2RP6000109, NT2RP6000110, NT2RP6000119, NT2RP6000123, NT2RP6000127, NT2RP6000130, NT2RP6000140, NT2RP6000150, NT2RP7000041, NT2RP7000069, NT2RP7000086, NT2RP7000109, NT2RP7000112, NT2RP7000238, NT2RP7000259, NT2RP7000271, NT2RP7000311, NT2RP7000359, NT2RP7000364, NT2RP7000380, NT2RP7000391, NT2RP7000425, NT2RP7000477, NT2RP7000579, NT2RP7000586, NT2RP7000600, NT2RP7000624,
NT2RP7000812, NT2RP7000906, NT2RP7000926, NT2RP7001074, NT2RP7001080, NT2RP7001156, NT2RP7001166, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001962, NT2RP7002028, NT2RP7002064, NT2RP7002151, NT2RP7002163, NT2RP7002243, NT2RP7002282, NT2RP7002376, NT2RP7002379, NT2RP7002449, NT2RP7002450, NT2RP7002478, NT2RP7002554, NT2RP7002594, NT2RP7002603, NT2RP7002619, NT2RP7002650, NT2RP7002738, NT2RP7002779, NT2RP7002802, NT2RP7002829, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107,
NT2RP7003134, NT2RP7003148, NT2RP7003203, NT2RP7003261, NT2RP7003319, NT2RP7003439, NT2RP7003511, NT2RP7003629, NT2RP7003632, NT2RP7003647, NT2RP7003680, NT2RP7003688, NT2RP7003724, NT2RP7003960, NT2RP7004027, NT2RP7004080, NT2RP7004114, NT2RP7004123, NT2RP7004173, NT2RP7004196, NT2RP7004204, NT2RP7004233, NT2RP7004260, NT2RP7004348, NT2RP7004352, NT2RP7004358, NT2RP7004373, NT2RP7004396, NT2RP7004428, NT2RP7004481, NT2RP7004541, NT2RP7004559, NT2RP7004641, NT2RP7004656, NT2RP7004687, NT2RP7004722, NT2RP7004728, NT2RP7004751, NT2RP7004766, NT2RP7004790, NT2RP7004884, NT2RP7004911, NT2RP7004915, NT2RP7004925, NT2RP7004946, NT2RP7004961, NT2RP7004964, NT2RP7004975, NT2RP7005118, NT2RP7005205,
NT2RP7005219, NT2RP7005323, NT2RP7005468, NT2RP7005493, NT2RP7005513, NT2RP7005529, NT2RP7005629, NT2RP7005631, NT2RP7005669, NT2RP7005675, NT2RP7005750, NT2RP7005846, NT2RP7006033, NT2RP7006075, NT2RP7006141, NT2RP7006160, NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006539, NT2RP7006547, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007114, NT2RP7007154, NT2RP7007221, NT2RP7007226, NT2RP7007252, NT2RP7007269, NT2RP7007310, NT2RP7007359, NT2RP7007381, NT2RP7007387, NT2RP7007406,
NT2RP7007480, NT2RP7007504, NT2RP7007537, NT2RP7007580, NT2RP7007594, NT2RP7007610, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008161, NT2RP7008167, NT2RP7008315, NT2RP7008360, NT2RP7008396, NT2RP7008406, NT2RP7008435, NT2RP7008441, NT2RP7008454, NT2RP7008487, NT2RP7008543, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009097, NT2RP7009108, NT2RP7009149, NT2RP7009168, NT2RP7009215, NT2RP7009225, NT2RP7009236,
NT2RP7009253, NT2RP7009259, NT2RP7009267, NT2RP7009322, NT2RP7009363, NT2RP7009370, NT2RP7009373, NT2RP7009394, NT2RP7009397, NT2RP7009429, NT2RP7009439, NT2RP7009466, NT2RP7009481, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583,
NT2RP7014721, NT2RP7014743, NT2RP7014778, NT2RP7014906, NT2RP7014910, NT2RP7015080, NT2RP7015431, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633,
NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000033, NTONG1000047, NTONG1000052, NTONG1000075, NTONG1000087, NTONG1000098, NTONG1000123, NTONG1000130, NTONG1000161, NTONG1000172, NTONG1000182, NTONG1000213, NTONG1000214, NTONG1000247, NTONG1000264, NTONG2000040, NTONG2000107, NTONG2000231, NTONG2000265, NTONG2000363, NTONG2000413, NTONG2000492, NTONG2000499, NTONG2000531, NTONG2000583, NTONG2000858, NTONG2000876, NTONG2000878, NTONG2000966, NTONG2000977, NTONG2000985,
NTONG2001014, NTONG2001079, NTONG2001137, NTONG2001157, NTONG2001173, NTONG2001428, NTONG2001532, NTONG2001550, NTONG2001567, NTONG2001612, NTONG2001619, NTONG2001642, NTONG2001657, NTONG2001676, NTONG2001762, NTONG2002278, NTONG2002582, NTONG2002807, NTONG2002948, NTONG2002970, NTONG2002985, NTONG2003158, NTONG2003210, NTONG2003316, NTONG2003409, NTONG2003454, NTONG2003515, NTONG2003805, NTONG2003839, NTONG2003852, NTONG2003897, NTONG2003928, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005391,
NTONG2005480, NTONG2005497, NTONG2005520, NTONG2005577, NTONG2005657, NTONG2005801, NTONG2005822, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008483, NTONG2008522, NTONG2008672, NTONG2008862, NTONG2008939, NTONG2008944, NTONG2009060, NTONG2009068, NTONG2009229, NTONG2009233, NTONG2009468, NTONG2009576, OCBBF1000016, OCBBF1000030, OCBBF1000042, OCBBF1000049, OCBBF1000054,
OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000175, OCBBF1000185, OCBBF1000188, OCBBF1000192, OCBBF1000254, OCBBF1000315, OCBBF2000013, OCBBF2000015, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000712, OCBBF2000719, OCBBF2000824, OCBBF2000831, OCBBF2000904, OCBBF2000986, OCBBF2000998, OCBBF2001075, OCBBF2001101, OCBBF2001124, OCBBF2001140, OCBBF2001166, OCBBF2001176, OCBBF2001186, OCBBF2001196, OCBBF2001210,
OCBBF2001252, OCBBF2001307, OCBBF2001323, OCBBF2001389, OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001687, OCBBF2001706, OCBBF2001749, OCBBF2001794, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003543, OCBBF2003593, OCBBF2003744, OCBBF2003819, OCBBF2004038, OCBBF2004104, OCBBF2004147,
OCBBF2004168, OCBBF2004235, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006388, OCBBF2006438, OCBBF2006567, OCBBF2006624, OCBBF2006639, OCBBF2006660, OCBBF2006764,
OCBBF2006849, OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007829, OCBBF2007892, OCBBF2007931, OCBBF2007946, OCBBF2008005, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770,
OCBBF2008775, OCBBF2008790, OCBBF2008814, OCBBF2008822, OCBBF2009095, OCBBF2009115, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536,
OCBBF2011625, OCBBF2011669, OCBBF2011685, OCBBF2011706, OCBBF2011722, OCBBF2011759, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012553, OCBBF2012678, OCBBF2012704, OCBBF2012714, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013481, OCBBF2013507, OCBBF2013721, OCBBF2013789, OCBBF2013843, OCBBF2013883,
OCBBF2013926, OCBBF2014052, OCBBF2014064, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017398, OCBBF2017458, OCBBF2017489,
OCBBF2017516, OCBBF2017665, OCBBF2017754, OCBBF2017791, OCBBF2017815, OCBBF2017882, OCBBF2017888, OCBBF2017893, OCBBF2017920, OCBBF2018012, OCBBF2018014, OCBBF2018084, OCBBF2018167, OCBBF2018206, OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018873, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788,
OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574,
OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296,
OCBBF3000323, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000086, PANCR1000091, PANCR1000185, PEBLM1000024, PEBLM1000029, PEBLM1000034, PEBLM1000062, PEBLM1000066, PEBLM1000068, PEBLM1000071, PEBLM1000083, PEBLM1000144, PEBLM1000147, PEBLM1000174,
PEBLM1000180, PEBLM2000030, PEBLM2000112, PEBLM2000126, PEBLM2000147, PEBLM2000170, PEBLM2000180, PEBLM2000213, PEBLM2000222, PEBLM2000248, PEBLM2000267, PEBLM2000270, PEBLM2000308, PEBLM2000321, PEBLM2000326, PEBLM2000333, PEBLM2000338, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215,
PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919,
PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007068, PERIC2007439, PERIC2007914, PERIC2008008, PERIC2008385, PERIC2009001, PERIC2009086, PERIC2009329, PERIC2009566, PLACE5000001, PLACE5000013, PLACE5000037, PLACE5000058, PLACE5000066, PLACE5000067, PLACE5000068, PLACE5000070, PLACE5000080, PLACE5000105, PLACE5000113, PLACE5000115, PLACE5000116, PLACE5000129, PLACE5000139, PLACE5000153, PLACE5000159, PLACE5000170, PLACE5000171, PLACE5000172, PLACE5000178,
PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000371, PLACE6000379, PLACE6000414, PLACE6000424, PLACE6000426, PLACE6000429, PLACE6000450, PLACE6000463, PLACE6000523, PLACE6000550, PLACE6000552, PLACE6000555, PLACE6000619, PLACE6000953, PLACE6001064, PLACE6001118, PLACE6001153, PLACE6001185, PLACE6001281, PLACE6001294, PLACE6001443, PLACE6001536, PLACE6001557, PLACE6001712, PLACE6001740, PLACE6001823, PLACE6001886, PLACE6001923,
PLACE6001925, PLACE6001933, PLACE6002011, PLACE6002016, PLACE6002056, PLACE6002084, PLACE6002102, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6002960, PLACE6003004, PLACE6003077, PLACE6003094, PLACE6003109, PLACE6003204, PLACE6003218, PLACE6003372, PLACE6003383, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004397, PLACE6004454, PLACE6004464,
PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373,
PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008793, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991,
PLACE6011041, PLACE6011057, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210,
PLACE6016254, PLACE6016383, PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657,
PLACE7003684, PLACE7003985, PLACE7004103, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000097, PROST1000110, PROST1000120, PROST1000123, PROST1000136, PROST1000144, PROST1000145, PROST1000152, PROST1000167, PROST1000184, PROST1000199, PROST1000215, PROST1000217, PROST1000220, PROST1000226, PROST1000246, PROST1000262, PROST1000272, PROST1000298, PROST1000322, PROST1000334, PROST1000343,
PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000564, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002,
PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003232, PROST2003302, PROST2003303,
PROST2003324, PROST2003338, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004095, PROST2004115, PROST2004142, PROST2004146, PROST2004161, PROST2004258, PROST2004270, PROST2004332, PROST2004416, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426, PROST2005466,
PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006282, PROST2006343, PROST2006450, PROST2006491, PROST2006510, PROST2006536, PROST2006579, PROST2006688, PROST2006737, PROST2006766, PROST2006782, PROST2006933, PROST2006988, PROST2007122 PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088,
PROST2008243, PROST2008245, PROST2008268, PROST2008271, PROST2008360, PROST2008447, PROST2008468, PROST2008472, PROST2008489, PROST2008516, PROST2008724, PROST2008770, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010782, PROST2010885, PROST2011012, PROST2011038, PROST2011105, PROST2011297, PROST2011410, PROST2011439,
PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012745, PROST2012780, PROST2012888, PROST2012890, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015287, PROST2015332, PROST2015457, PROST2015464,
PROST2015537, PROST2015545, PROST2015551, PROST2015636, PROST2015710, PROST2015756, PROST2015877, PROST2015900, PROST2015924, PROST2015932, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2016980, PROST2017098, PROST2017128, PROST2017203, PROST2017367, PROST2017413, PROST2017441, PROST2017529, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583,
PROST2018588, PROST2018607, PROST2018788, PROST2018902, PROST2018922, PROST2018977, PROST2019164, PROST2019253, PROST2019296, PROST2019398, PROST2019487, PROST2019781, PUAEN1000039, PUAEN1000057, PUAEN1000065, PUAEN1000081, PUAEN1000085, PUAEN1000087, PUAEN1000121, PUAEN1000147, PUAEN1000161, PUAEN1000164, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001586, PUAEN2001882, PUAEN2002473, PUAEN2002489, PUAEN2002552, PUAEN2002568, PUAEN2002616, PUAEN2002758, PUAEN2003018,
PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347,
RECTM2001519, RECTM2001666, RECTM2001691, RECTM2001749, RECTM2002172, RECTM2002602, RECTM2002632, SALGL1000005, SALGL1000028, SALGL1000047, SALGL1000065, SALGL1000107, SALGL1000171, SKMUS1000014, SKMUS1000022, SKMUS1000030, SKMUS1000064, SKMUS1000067, SKMUS1000084, SKMUS1000102, SKMUS1000104, SKMUS1000110, SKMUS1000118, SKMUS1000124, SKMUS1000129, SKMUS1000138, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000361, SKMUS2000365, SKMUS2000380, SKMUS2000390, SKMUS2000416, SKMUS2000458, SKMUS2000467, SKMUS2000468,
SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000931, SKMUS2000933, SKMUS2000945, SKMUS2000954, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001454, SKMUS2001492, SKMUS2001501, SKMUS2001543, SKMUS2001580, SKMUS2001585,
SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474,
SKMUS2008585, SKMUS2008607, SKMUS2009190, SKMUS2009232, SKMUS2009479, SKMUS2009557, SKNMC1000110, SKNMC1000123, SKNMC1000124, SKNMC1000137, SKNMC1000159, SKNMC1000168, SKNMC1000229, SKNMC1000251, SKNMC1000264, SKNMC2000065, SKNMC2000097, SKNMC2000224, SKNMC2000256, SKNMC2000305, SKNMC2000356, SKNMC2000374, SKNMC2000583, SKNMC2000593, SKNMC2000612, SKNMC2000622, SKNMC2000649, SKNMC2000698, SKNMC2000877, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504,
SKNMC2007961, SKNMC2009450, SKNSH1000002, SKNSH1000086, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000347, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149,
SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000042, SMINT1000048, SMINT1000051, SMINT1000054, SMINT1000057, SMINT1000071, SMINT1000075, SMINT1000078, SMINT1000091, SMINT1000100, SMINT1000103, SMINT1000117, SMINT1000118, SMINT1000137, SMINT1000192, SMINT2000007, SMINT2000018, SMINT2000032, SMINT2000040, SMINT2000060, SMINT2000073, SMINT2000084, SMINT200145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000441, SMINT2000454, SMINT2000468,
SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000629, SMINT2000659, SMINT2000694, SMINT2000747, SMINT2000811, SMINT2000824, SMINT2000984, SMINT2001114, SMINT2001158, SMINT2001183, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414, SMINT2002457, SMINT2002620, SMINT2002689, SMINT2002743, SMINT2002778, SMINT2002880,
SMINT2002882, SMINT2002899, SMINT2002974, SMINT2002976, SMINT2003003, SMINT2003074, SMINT2003128, SMINT2003150, SMINT2003169, SMINT2003317, SMINT2003340, SMINT2003386, SMINT2003423, SMINT2003551, SMINT2003569, SMINT2003641, SMINT2003644, SMINT2003866, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387, SMINT2005402, SMINT2005405, SMINT2005621, SMINT2005623, SMINT2005624, SMINT2005800,
SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921,
SMINT2009119, SMINT2009121, SMINT2009212, SMINT2009216, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958,
SMINT2012040, SMINT2012122, SMINT2012179, SMINT2012195, SMINT2012285, SMINT2012291, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432,
SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000091, SPLEN1000106, SPLEN1000134, SPLEN1000139, SPLEN1000143, SPLEN1000144, SPLEN1000145, SPLEN1000156, SPLEN1000163, SPLEN1000165, SPLEN1000166, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243,
SPLEN2000247, SPLEN2000255, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000516, SPLEN2000537, SPLEN2000541, SPLEN2000607, SPLEN2000630, SPLEN2000695, SPLEN2000839, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007,
SPLEN2002053, SPLEN2002133, SPLEN2002147, SPLEN2002166, SPLEN2002175, SPLEN2002223, SPLEN2002272, SPLEN2002314, SPLEN2002335, SPLEN2002343, SPLEN2002354, SPLEN2002385, SPLEN2002419, SPLEN2002451, SPLEN2002462, SPLEN2002463, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2005009, SPLEN2005227,
SPLEN2005009, SPLEN2005416, SPLEN2005429, SPLEN2005450, SPLEN2005272, SPLEN2005416, SPLEN2005429, SPLEN2005450, SPLEN2005474, SPLEN2005560, SPLEN2005727, SPLEN2005783, SPLEN2005790, SPLEN2005818, SPLEN2005838, SPLEN2005869, SPLEN2005927, SPLEN2005939, SPLEN2006133, SPLEN2006143, SPLEN2006232, SPLEN2006268, SPLEN2006283, SPLEN2006305, SPLEN2006325, SPLEN2006374, SPLEN2006389, SPLEN2006425, SPLEN2006671, SPLEN2006701, SPLEN2006785, SPLEN2006875, SPLEN2007182, SPLEN2007314, SPLEN2007326, SPLEN2007350, SPLEN2007388, SPLEN2007498, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496,
SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010195, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252,
SPLEN2011419, SPLEN2011422, SPLEN2011672, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014080,
SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045,
SPLEN2016053, SPLEN2016069, SPLEN2016098, SPLEN2016135, SPLEN2016139, SPLEN2016241, SPLEN2016250, SPLEN2016268, SPLEN2016304, SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098,
SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223,
SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024273, SPLEN2024383, SPLEN2024489, SPLEN2024530, SPLEN2024571, SPLEN2024577, SPLEN2024617, SPLEN2024737, SPLEN2024922,
SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027113, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424,
SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678,
SPLEN2037722, SPLEN2037891, SPLEN2038055, SPLEN2038180, SPLEN2038345, SPLEN2038407, SPLEN2038473, SPLEN2038834, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042535, SPLEN2042598, SPLEN2042714, SPLEN2042920, STOMA1000013, STOMA1000047, STOMA1000052, STOMA1000074, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000121, STOMA2000148, STOMA2000168,
STOMA2000183, STOMA2000188, STOMA2000257, STOMA2000279, STOMA2000289, STOMA2000383, STOMA2000386, STOMA2000395, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213,
STOMA2006229, STOMA2006325, STOMA2006330, STOMA2006398, STOMA2006447, STOMA2006780, STOMA2006904, STOMA2007269, STOMP,2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000124, SYNOV1000128, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001033, SYNOV2001035, SYNOV2001088, SYNOV2001111,
SYNOV2001144, SYNOV2001212, SYNOV2001251, SYNOV2001300, SYNOV2001356, SYNOV2001374, SYNOV2001390, SYNOV2001451, SYNOV2001457, SYNOV2001468, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224,
SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, SYNOV4009575, T1ESE2000116, T1ESE2000609, T1ESE2000904, T1ESE2002665, TBAES2000004, TBAES2000059, TBAES2000116, TBAES2000255, TBAES2000315, TBAES2000352, TBAES2000932, TBAES2001171,
TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312,
TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865,
TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000023, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179,
TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179, TESTI2000184, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000598, TESTI2000600, TESTI2000616, TESTI2000627, TESTI2000644, TESTI2000671,
TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001099, TESTI2001120, TESTI2001134, TESTI2001141, TESTI2001153, TESTI2001180, TESTI2001208, TESTI2001229, TESTI2001236, TESTI2001237, TESTI2001269, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593,
TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365,
TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003074, TESTI2003089, TESTI2003104, TESTI2003109, TESTI2003117, TESTI2003130, TESTI2003131,
TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418, TESTI2003419, TESTI2003432, TESTI2003454, TESTI2003475, TESTI2003476, TESTI2003498, TESTI2003512, TESTI2003533, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727,
TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675,
TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004929, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568,
TESTI2005584, TESTI2005588, TESTI2005590, TESTI2005603, TESTI2005609, TESTI2005610, TESTI2005630, TESTI2005672, TESTI2005683, TESTI2005690,TESTI2005692, TESTI2005703, TESTI2005720, TESTI2005731, TESTI2005739, TESTI2005742, TESTI2005743, TESTI2005759, TESTI2005767, TESTI2005775, TESTI2005784, TESTI2005788, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109,
TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006615, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877,
TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007407, TESTI2007422, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972,
TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008240, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960,
TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009477, TESTI2009497, TESTI2009502, TESTI2009511, TESTI2009520, TESTI2009544, TESTI2009545, TESTI2009551, TESTI2009577, TESTI2009585, TESTI2009588, TESTI2009683, TESTI2009727, TESTI2009739, TESTI2009785, TESTI2009812, TESTI2009835, TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591,
TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356,
TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013295, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889,
TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335, TESTI2015350, TESTI2015375, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033,
TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816,
TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018462, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648,
TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021124, TESTI2021138,
TESTI2021297, TESTI2021315, TESTI2021358, TESTI2021425, TESTI2021447, TESTI2021463, TESTI2021531, TESTI2021599, TESTI2021637, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414,
TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025022, TESTI2025063, TESTI2025117, TESTI2025144, TESTI2025161, TESTI2025174, TESTI2025229, TESTI2025269, TESTI2025365,
TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854, TESTI2026925,
TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776,
TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643,
TESTI2032681, TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309,
TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065,
TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362,
TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796, TESTI2044833,
TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046100, TESTI2046188, TESTI2046347, TESTI2046352, TESTI2046358, TESTI2046439, TESTI2046456, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109,
TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665,
TESTI4001679, TESTI4001795, TESTI4001923, TESTI4001925, TESTI4001984, TESTI4002003, TESTI4002072, TESTI4002141, TESTI4002195, TESTI4002202, TESTI4002290, TESTI4002319, TESTI4002491, TESTI4002520, TESTI4002552, TESTI4002647, TESTI4002703, TESTI4002754, TESTI4002799, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133,
TESTI4005158, TESTI4005317, TESTI4005322, TESTI4005399, TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799,
TESTI4007810, TESTI4007816, TESTI4007965, TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475,
TESTI4010544, TESTI4010713, TESTI4010721, TESTI4010789, TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013675, TESTI4013685, TESTI4013735,
TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865,
TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920,
TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390,
TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335,
TESTI4031745, TESTI4031818, TESTI4032090, TESTI4032112, TESTI4032128, TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037156, TESTI4037188, TESTI4037228, TESTI4037244, TESTI4037727,
TESTI4037949, TESTI4038047, TESTI4038099, TESTI4038156, TESTI4038223, TESTI4038258, TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711,
TESTI4042846, TESTI4043067, TESTI4043129, TESTI4043140, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903,
TESTI4046962, TESTI4047069, TESTI4047119, TESTI4047305, TESTI4047328, TESTI4047437, TESTI4047569, TESTI4047746, TESTI4047808, THYMU1000002, THYMU1000010, THYMU1000017, THYMU1000025, THYMU1000029, THYMU1000032, THYMU1000033, THYMU1000041, THYMU1000060, THYMU1000074, THYMU1000083, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000399, THYMU1000426, THYMU1000428, THYMU1000440, THYMU1000459,
THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000382, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879,
THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242,
THYMU2002338, THYMU2002356, THYMU2002376, THYMU2002384, THYMU2002450, THYMU2002490, THYMU2002548, THYMU2002583, THYMU2002695, THYMU2002745, THYMU2002756, THYMU2002789, THYMU2002792, THYMU2002815, THYMU2002841, THYMU2002852, THYMU2002910, THYMU2002950, THYMU2002983, THYMU2003046, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008,
THYMU2004042, THYMU2004108, THYMU2004111, THYMU2004123, THYMU2004139, THYMU2004152, THYMU2004169, THYMU2004284, THYMU2004320, THYMU2004336, THYMU2004344, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005546, THYMU2005574,
THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006303, THYMU2006324, THYMU2006350, THYMU2006357, THYMU2006365, THYMU2006420, THYMU2006468, THYMU2006505, THYMU2006610, THYMU2006666, THYMU2006712, THYMU2006813, THYMU2006913, THYMU2006946, THYMU2006965, THYMU2007025, THYMU2007036, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528,
THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101,
THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538,
THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757,
THYMU2013779, THYMU2013863, THYMU2013916, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158,
THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020491, THYMU2020499, THYMU2020560, THYMU2020667, THYMU2020830,
THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734,
THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696,
THYMU2032715, THYMU2032732, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504,
THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485,
THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701,
THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642,
THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983,
THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758,
THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059,
TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025,
TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192,
TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000030, TRACH1000038, TRACH1000057, TRACH1000063, TRACH1000074, TRACH1000100, TRACH1000106, TRACH1000125, TRACH1000140, TRACH1000144, TRACH1000181, TRACH1000193, TRACH1000205, TRACH1000212, TRACH1000240, TRACH2000002, TRACH2000015, TRACH2000024, TRACH2000032, TRACH2000045, TRACH2000047,
TRACH2000081, TRACH2000083, TRACH2000148, TRACH2000185, TRACH2000235, TRACH2000237, TRACH2000243, TRACH2000248, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000420, TRACH2000461, TRACH2000472, TRACH2000496, TRACH2000497, TRACH2000502, TRACH2000522, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000920, TRACH2000923, TRACH2000926, TRACH2000944, TRACH2000959, TRACH2000972, TRACH2000981, TRACH2000986, TRACH2001021,
TRACH2001037, TRACH2001060, TRACH2001085, TRACH2001101, TRACH2001121, TRACH2001147, TRACH2001154, TRACH2001188, TRACH2001192, TRACH2001249, TRACH2001275, TRACH2001289, TRACH2001314, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001478, TRACH2001525, TRACH2001532, TRACH2001549, TRACH2001592, TRACH2001596, TRACH2001607, TRACH2001612, TRACH2001621, TRACH2001646, TRACH2001651, TRACH2001684, TRACH2001898, TRACH2001933, TRACH2001968, TRACH2001996, TRACH2002054, TRACH2002092, TRACH2002100, TRACH2002138, TRACH2002333, TRACH2002537, TRACH2002664, TRACH2002784,
TRACH2002803, TRACH2002840, TRACH2002847, TRACH2002853, TRACH2002862, TRACH2002954, TRACH2002960, TRACH2002988, TRACH2003070, TRACH2003272, TRACH2003323, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800,
TRACH2005811, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007399, TRACH2007483, TRACH2007542, TRACH2007577, TRACH2007674, TRACH2007676, TRACH2007688, TRACH2007733, TRACH2007816, TRACH2007894, TRACH2007969, TRACH2007988, TRACH2008112, TRACH2008113, TRACH2008127, TRACH2008130, TRACH2008278, TRACH2008300, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268,
TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562,
TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014124, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015823,
TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080,
TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084,
TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002192, TRACH3002245, TRACH3002293, TRACH3002533, TRACH3002561, TRACH3002567, TRACH3002605, TRACH3002650, TRACH3002752, TRACH3002866, TRACH3002871, TRACH3002876,
TRACH3002890, TRACH3003009, TRACH3003037, TRACH3003357, TRACH3003379, TRACH3003547, TRACH3003586, TRACH3003683, TRACH3003780, TRACH3003805, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005699, TRACH3005702, TRACH3005808, TRACH3005852, TRACH3005896, TRACH3006038, TRACH3006149, TRACH3006228, TRACH3006264,
TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008632, TRACH3008688, TRACH3008697, TRACH3008713, TRACH3008887, TRACH3008902, TRACH3008990, TRACH3009001, TRACH3009008, TRACH3009059, TRACH3009061, TRACH3009148, TRACH3009455, TRACH3009701, TRACH3009715,
TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136,
TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606,TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930,
TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681,
TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145,
TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036997, TRACH3037063, TRACH3037067, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235,TSTOM2000315, TSTOM2000442, TSTOM2000553, TSTOM2000569, TSTOM2000588, TSTOM2001195, TSTOM2001274,
TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131,
UTERU1000138, UTERU1000148, UTERU1000160, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000263, UTERU2000300, UTERU2000329, UTERU2000338, UTERU2000349, UTERU2000377, UTERU2000393, UTERU2000418, UTERU2000424, UTERU2000465, UTERU2000485, UTERU2000517, UTERU2000524, UTERU2000537, UTERU2000539, UTERU20000541, UTERU2000542, UTERU2000546, UTERU2000550, UTERU2000569, UTERU2000607, UTERU2000629, UTERU2000649, UTERU2000663, UTERU2000696, UTERU2000794, UTERU2000830, UTERU2000844, UTERU2000922, UTERU2000925,
UTERU2001024, UTERU2001110, UTERU2001176, UTERU2001281, UTERU2001389, UTERU2001409, UTERU2001412, UTERU2001504, UTERU2001607, UTERU2001658, UTERU2001747, UTERU2001876, UTERU2002001, UTERU2002011, UTERU2002176, UTERU2002198, UTERU2002294, UTERU2002332, UTERU2002410, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004197, UTERU2004299,
UTERU2004461, UTERU2004520, UTERU2004564, UTERU2004688, UTERU2004698, UTERU2004807, UTERU2004861, UTERU2004929, UTERU2005004, UTERU2005050, UTERU2005069, UTERU2005074, UTERU2005179, UTERU2005292, UTERU2005346, UTERU2005354, UTERU2005446, UTERU2005449, UTERU2005450, UTERU2005533, UTERU2005601, UTERU2005621, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499,
UTERU2007639, UTERU2007724, UTERU2007924, UTERU2007942, UTERU2008018, UTERU2008019, UTERU2008040, UTERU2008077, UTERU2008130, UTERU2008302, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431,
UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502,
UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016822, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810,
UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045,
UTERU2023175, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703,
UTERU2031834, UTERU2031851, UTERU2032075, UTERU2032220, UTERU2032279, UTERU2032726, UTERU2033172, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670,
UTERU3000727, UTERU3000738, UTERU3000828, UTERU3000844, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049,
UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092,
UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the NT2NE, NT2RI, NT2RM, NT2RP, NTISM, NTONG, and OCBBF libraries were analyzed for expression frequency. The analysis results are described below. Each is a relative value, where the expression level over all tissues is taken as 100. The larger this value, the greater the level of expression.

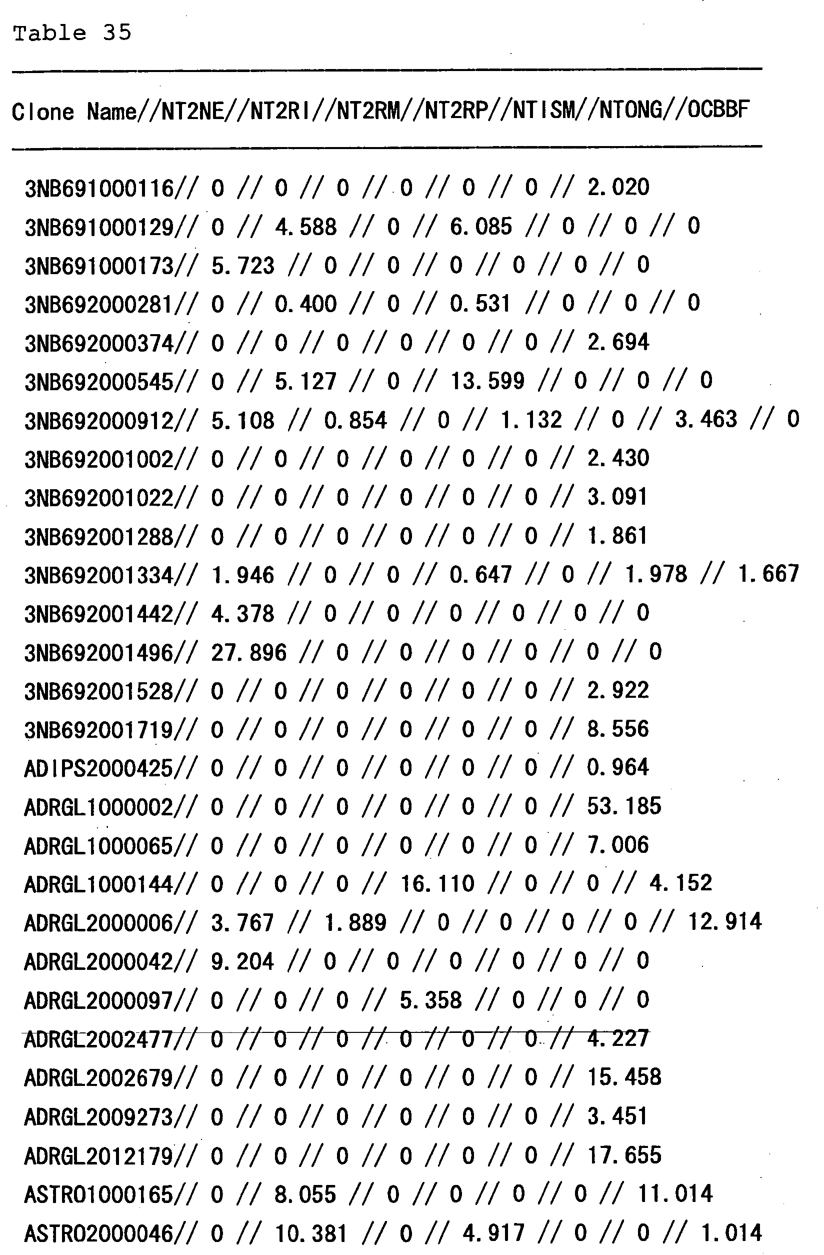

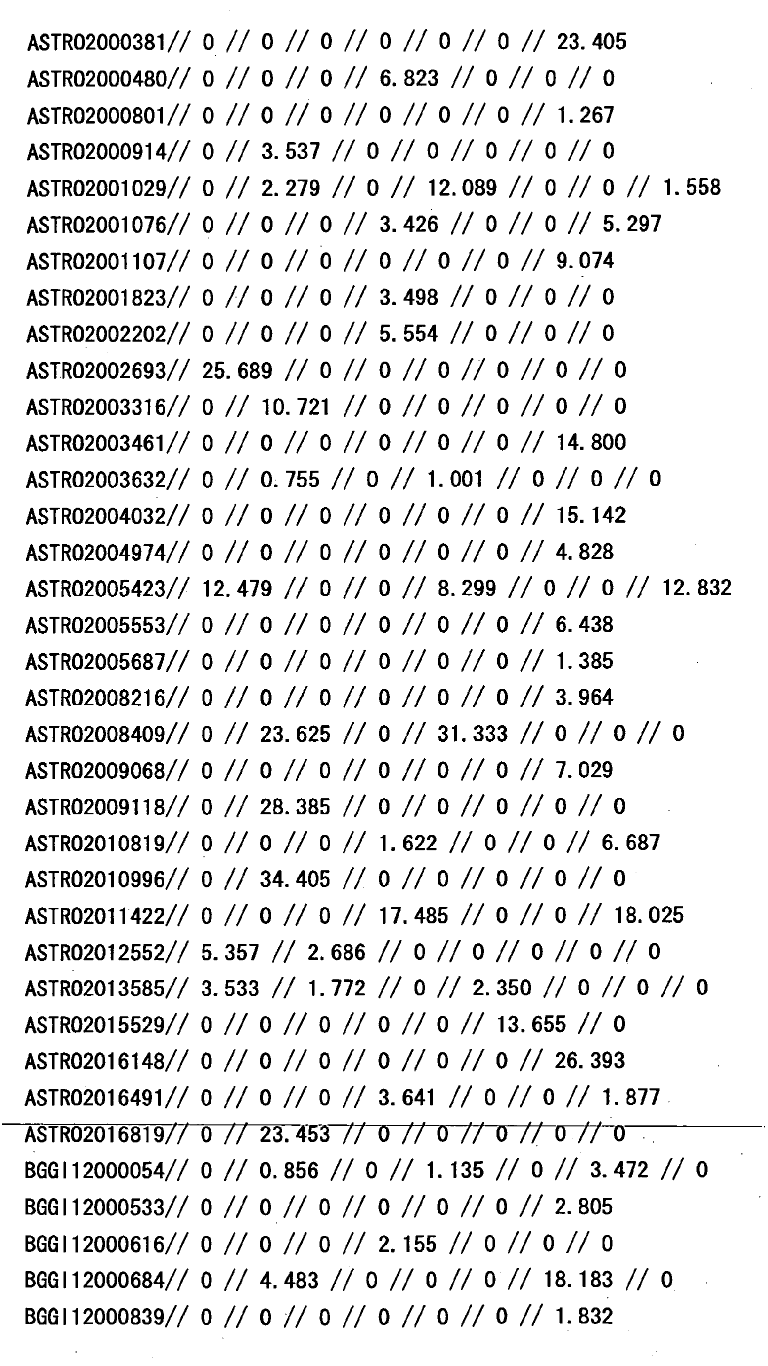

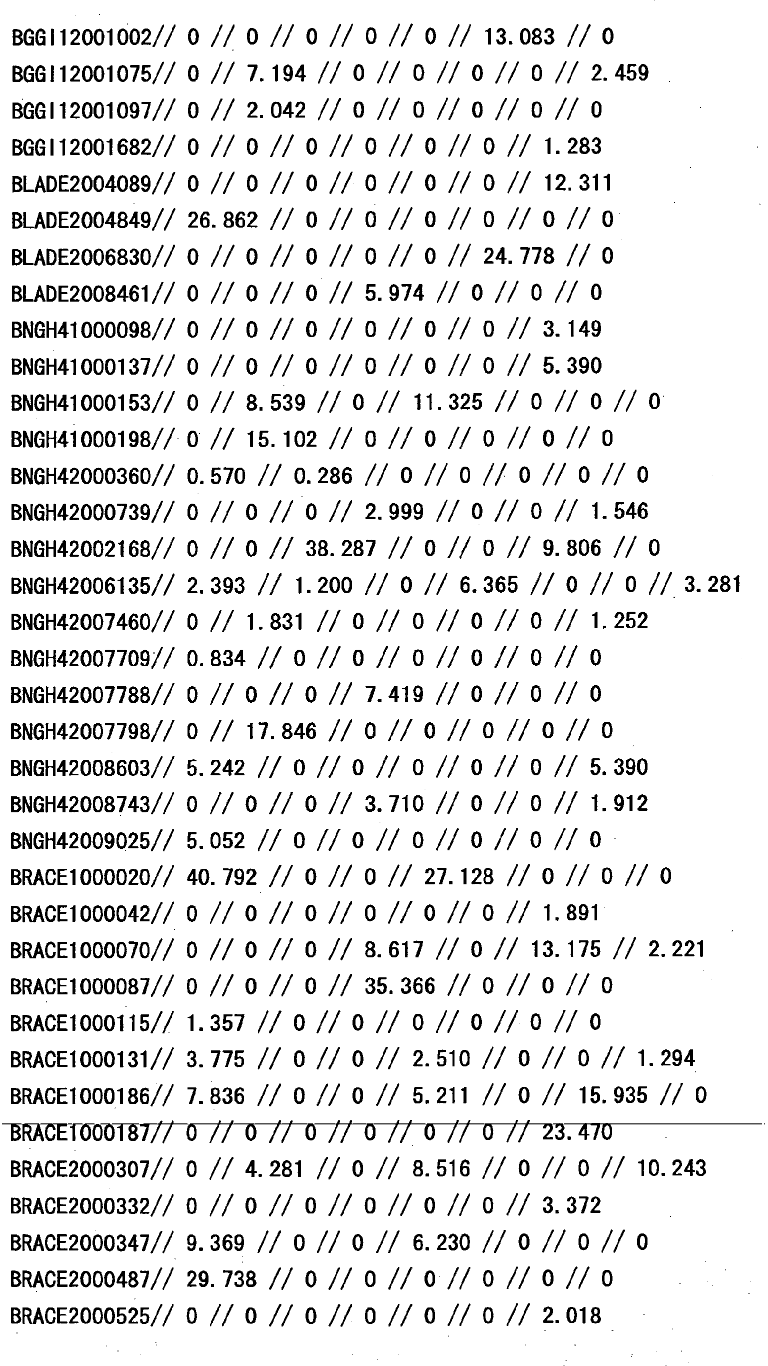

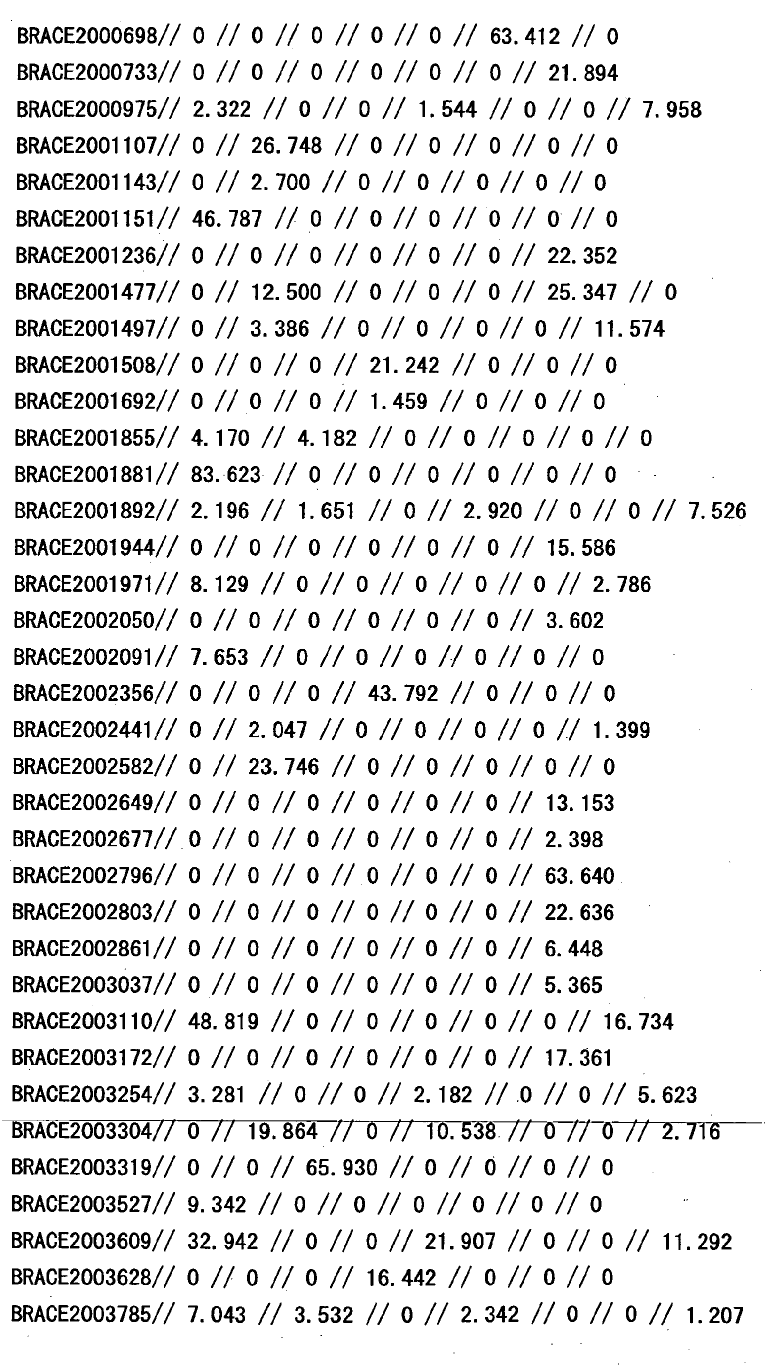

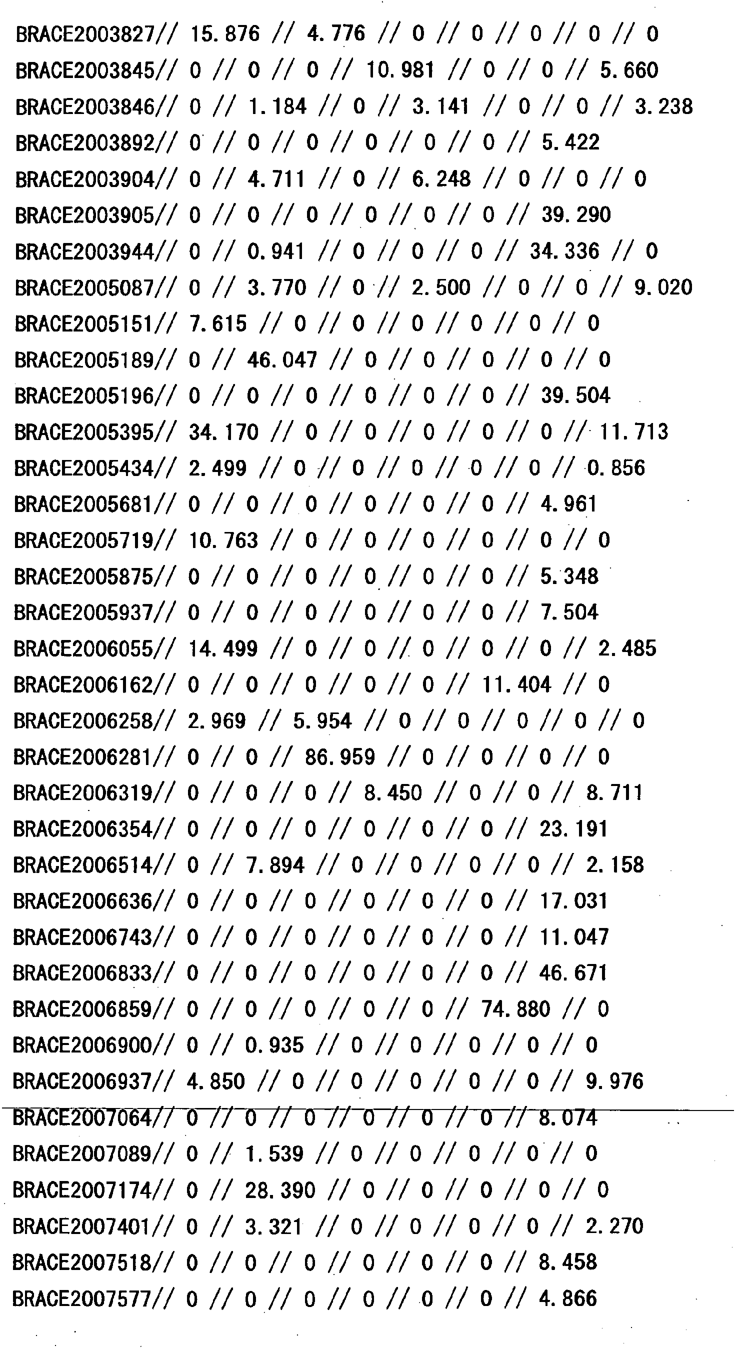

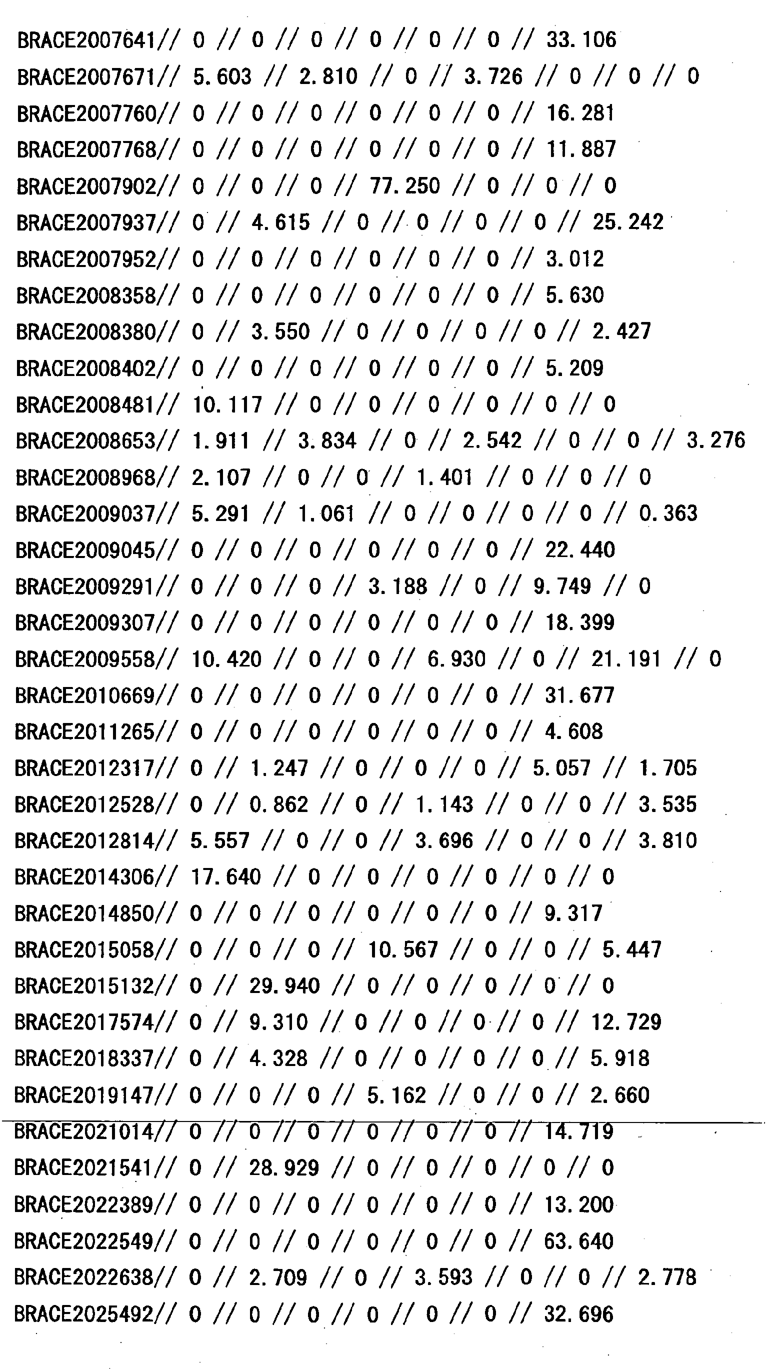

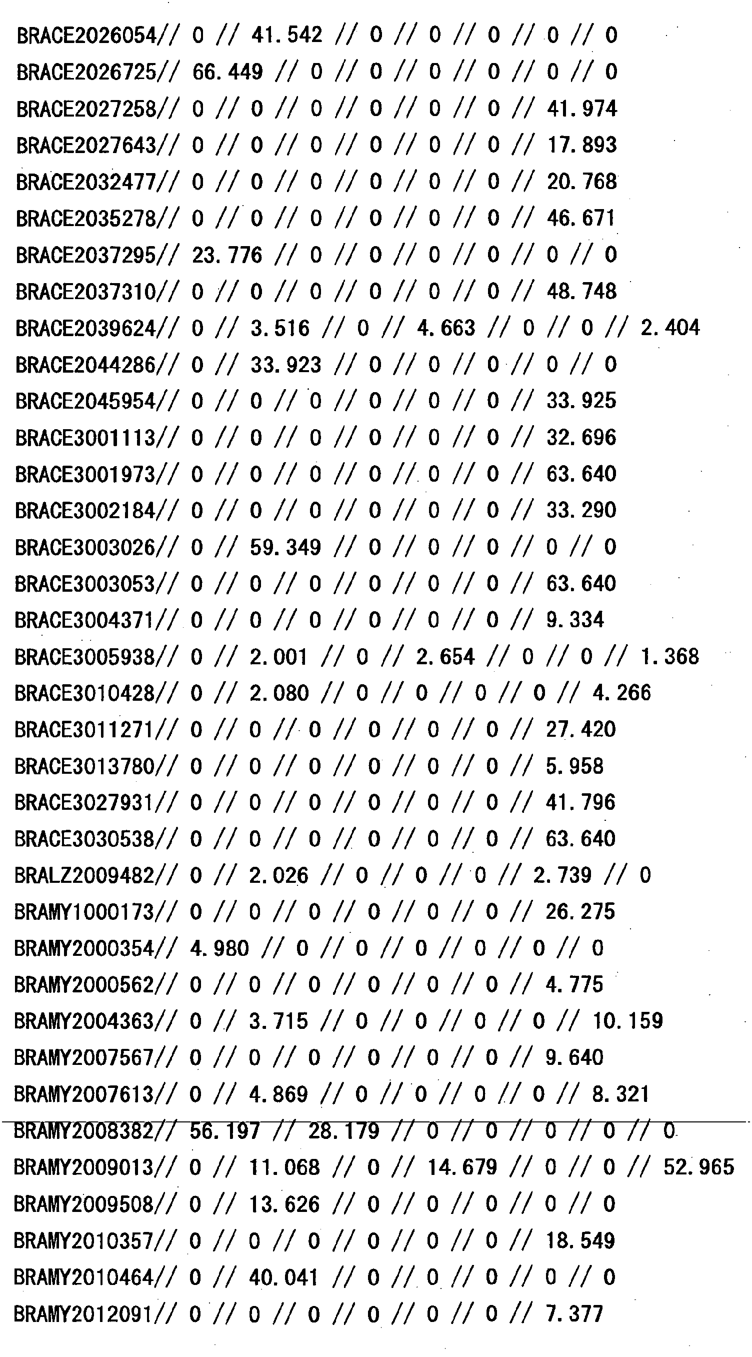

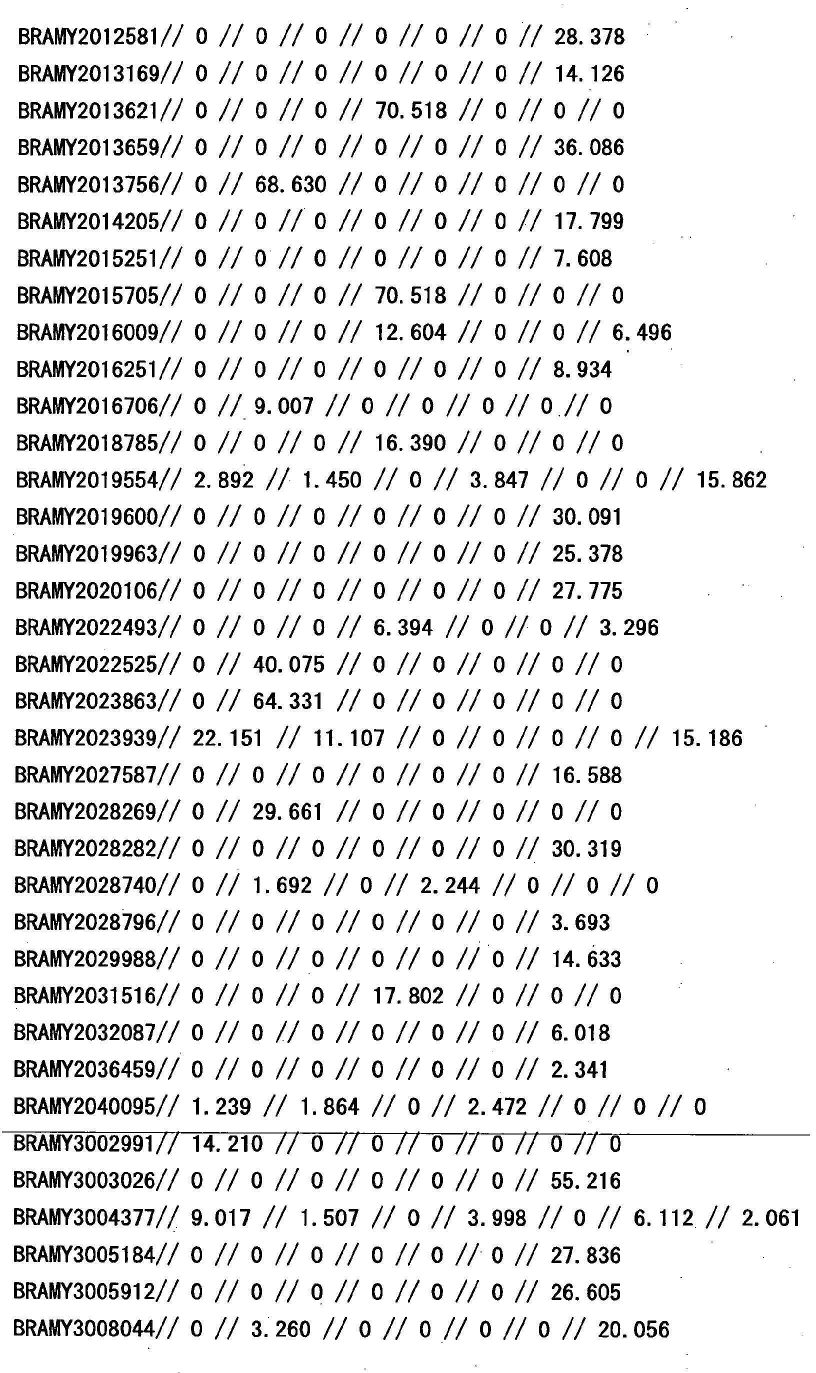

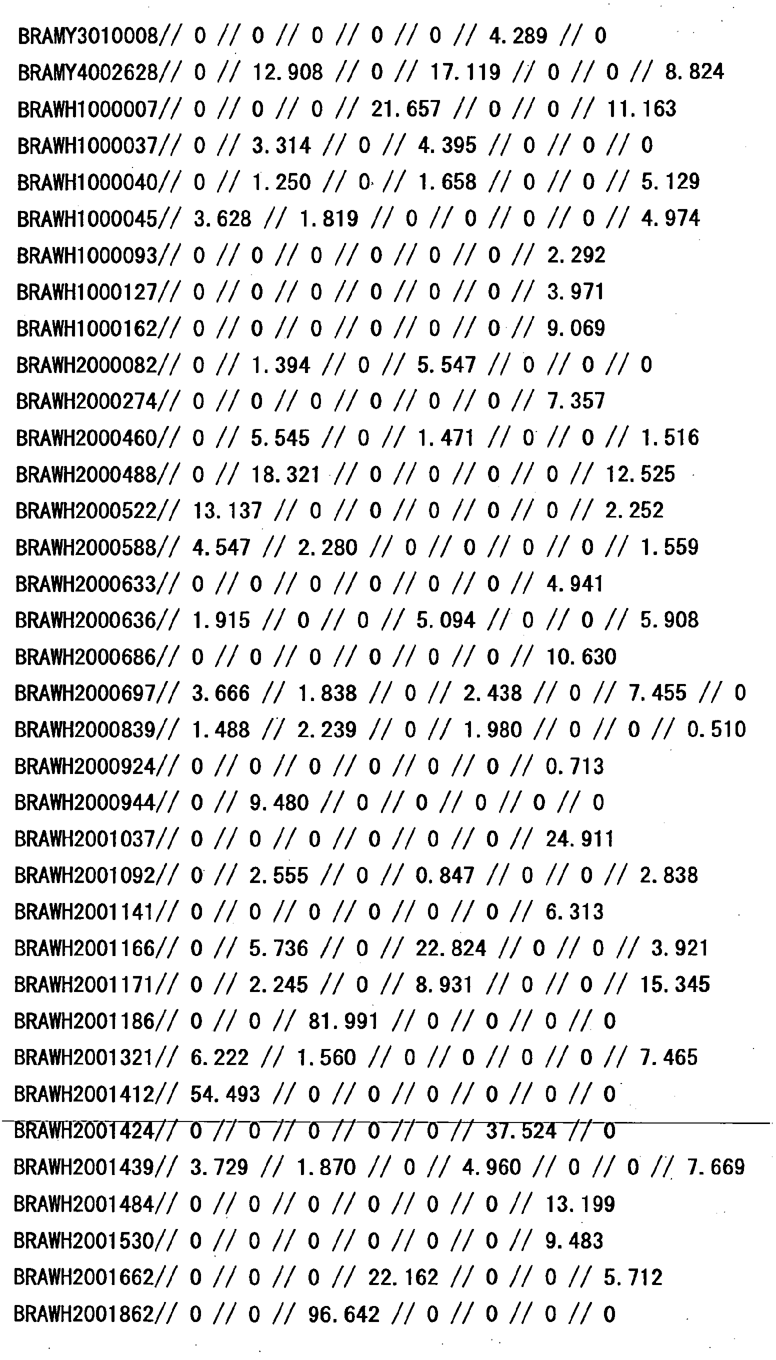

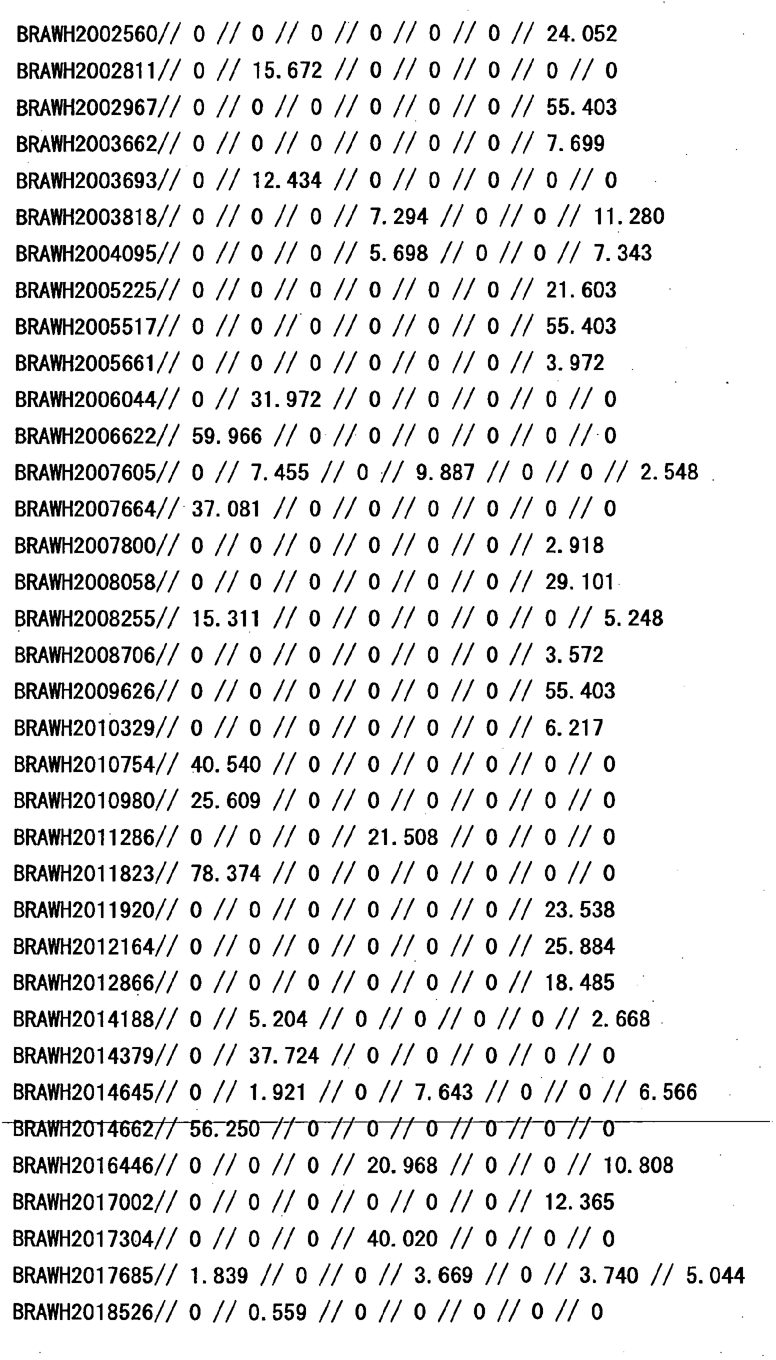

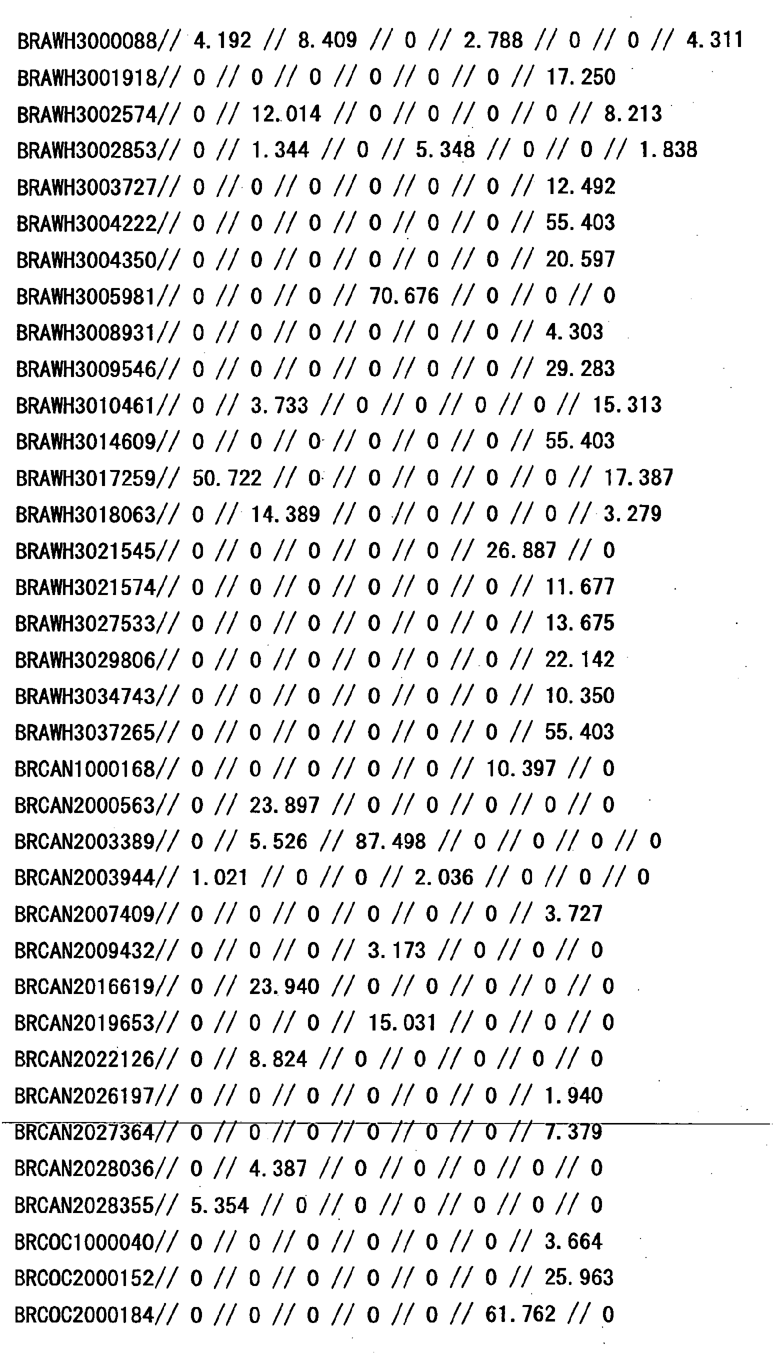

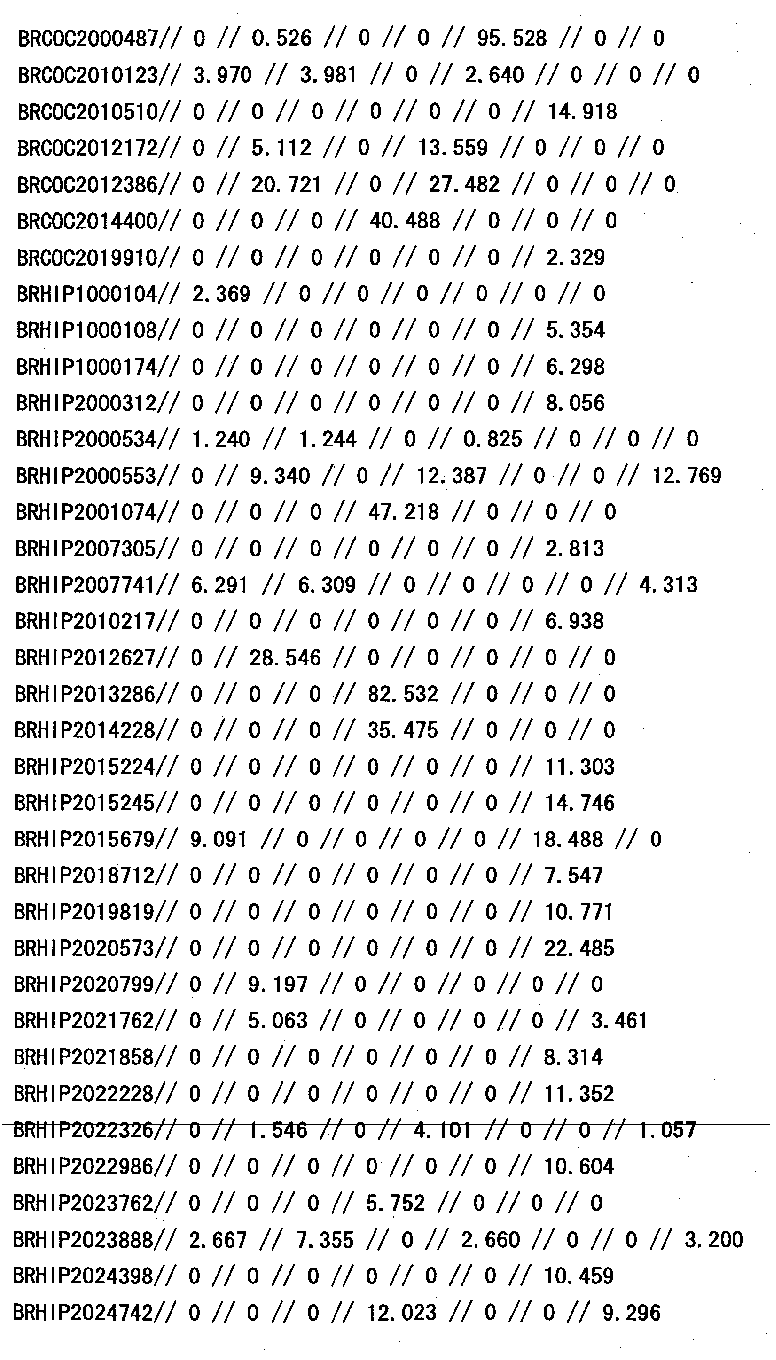

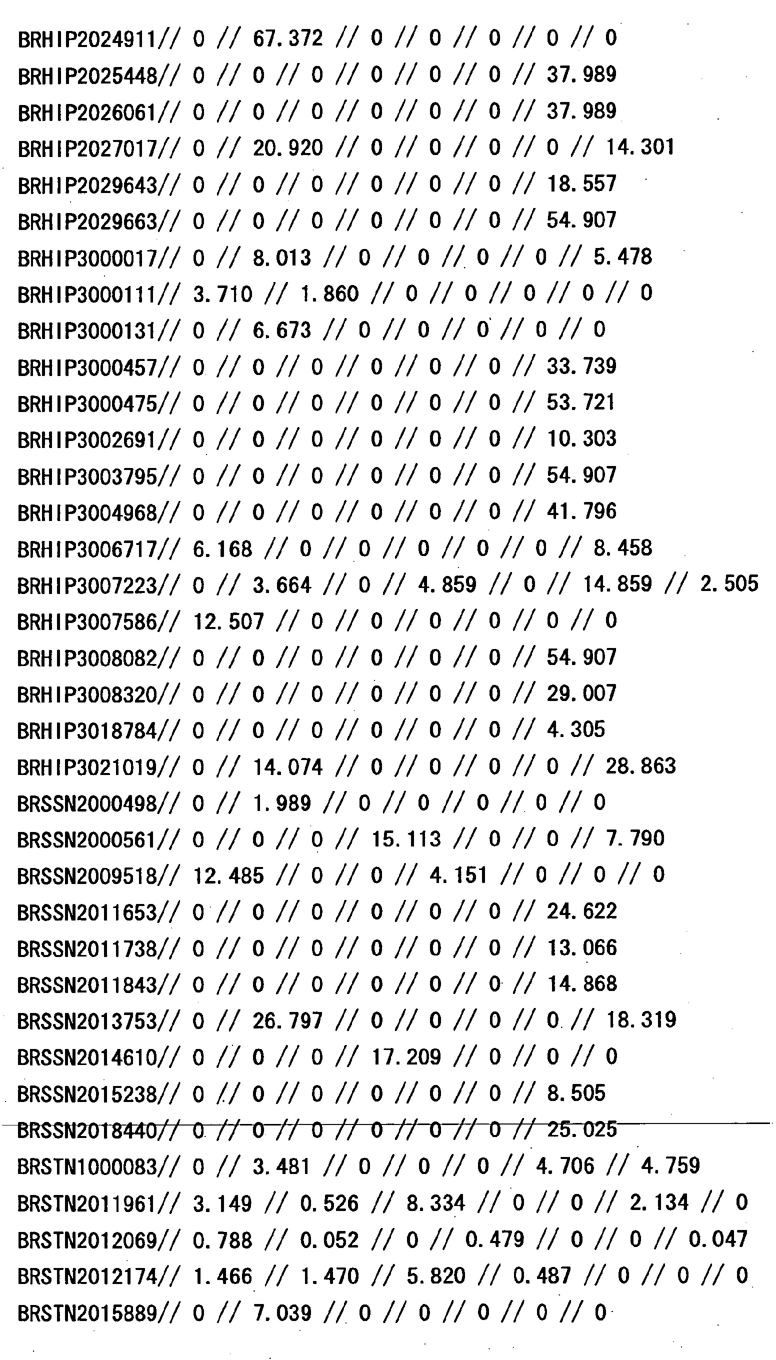

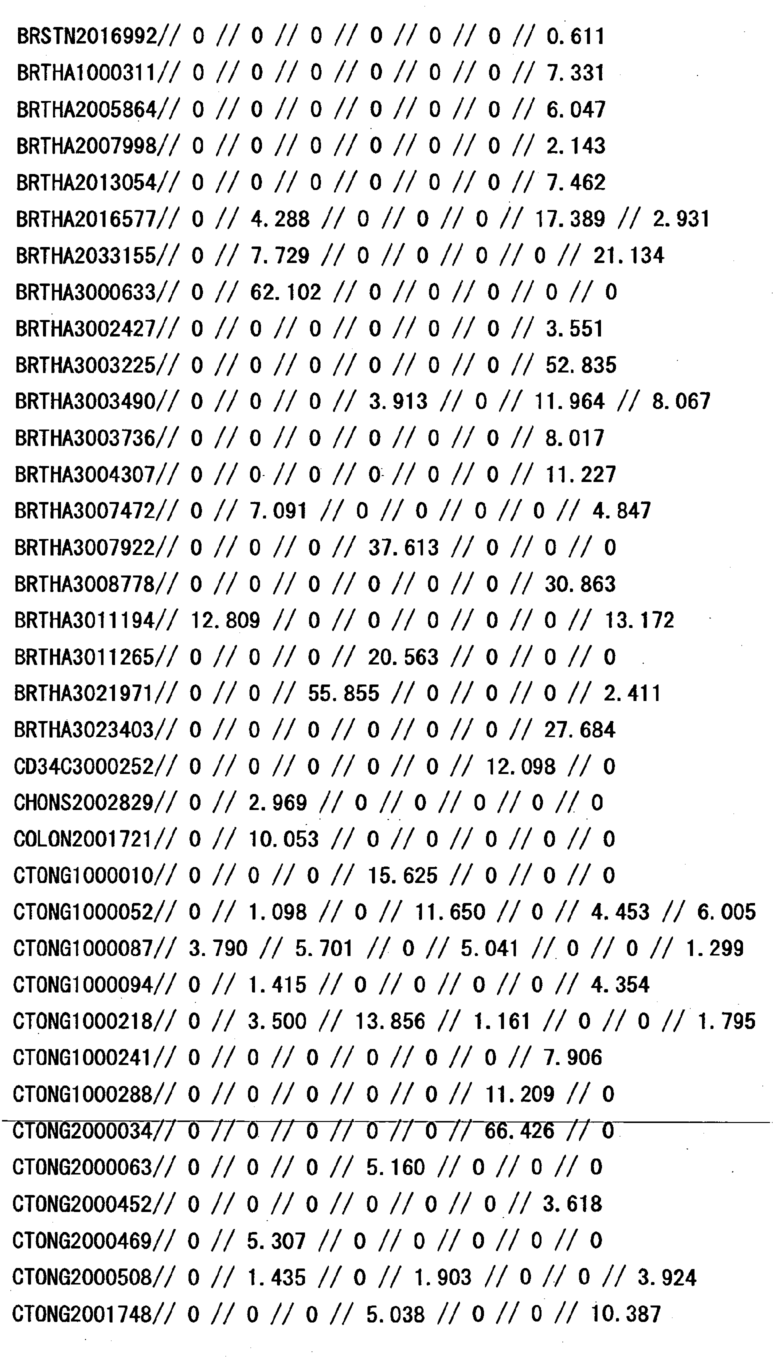

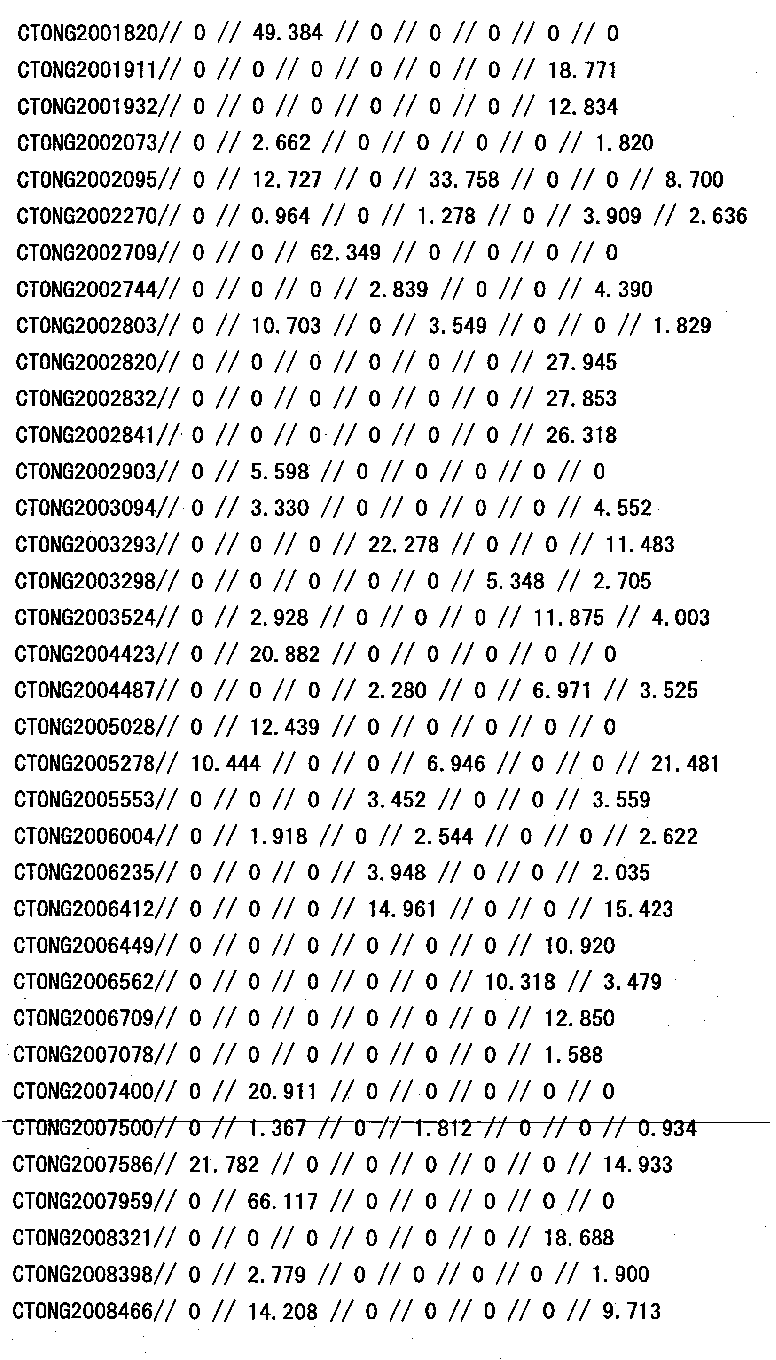

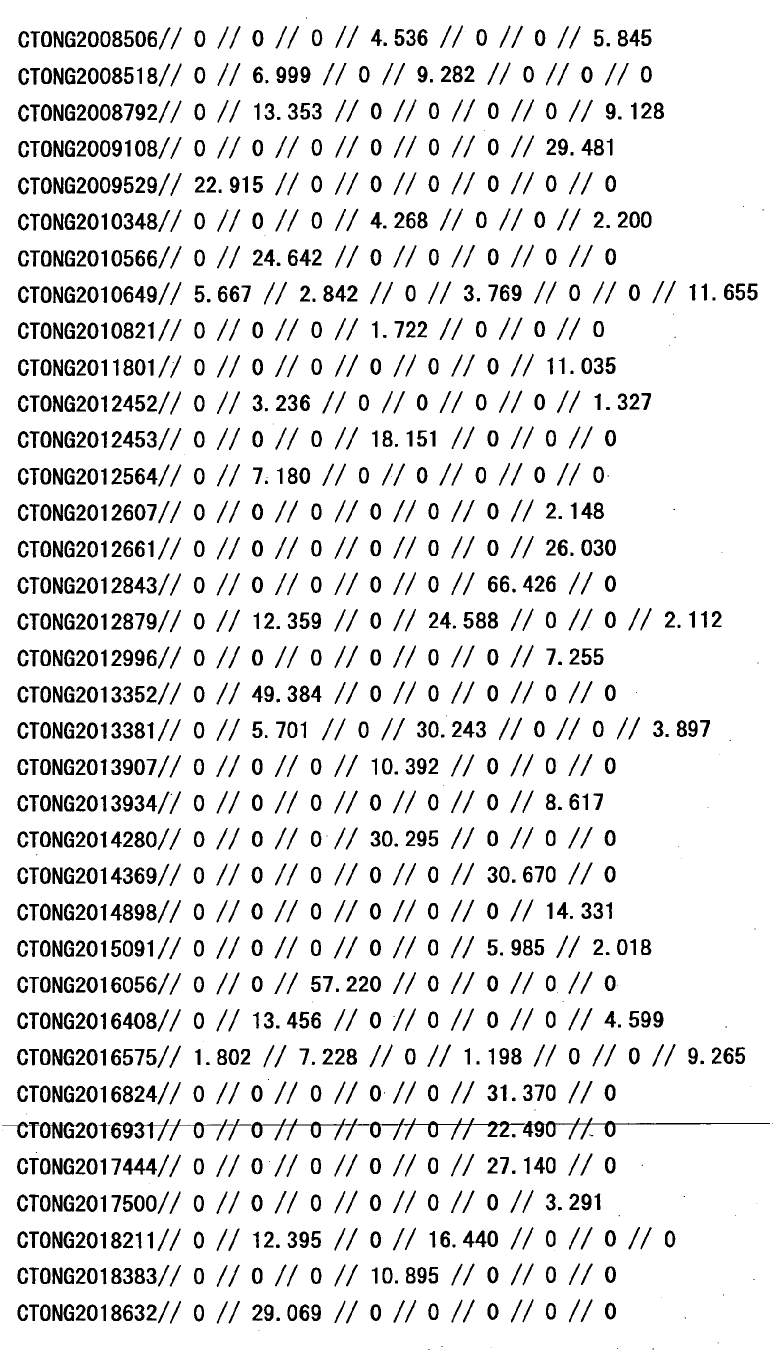

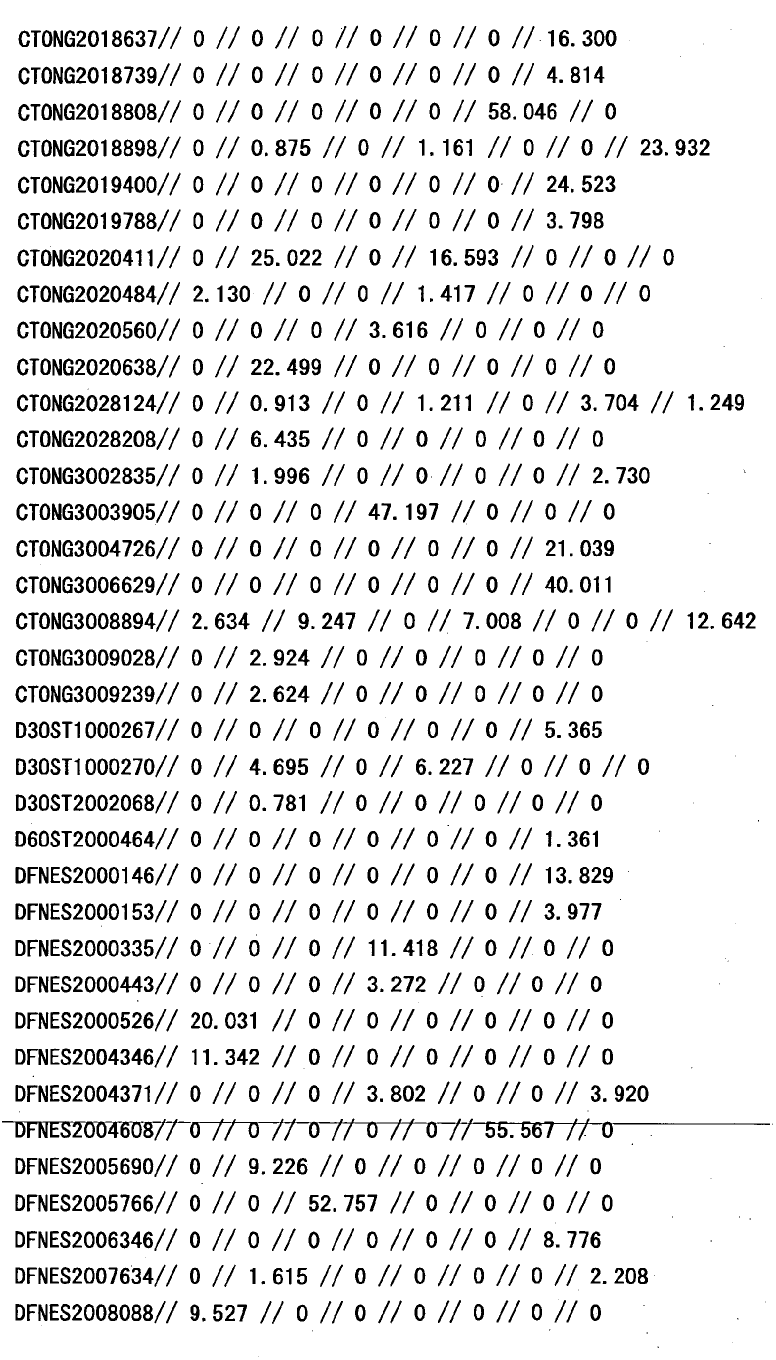

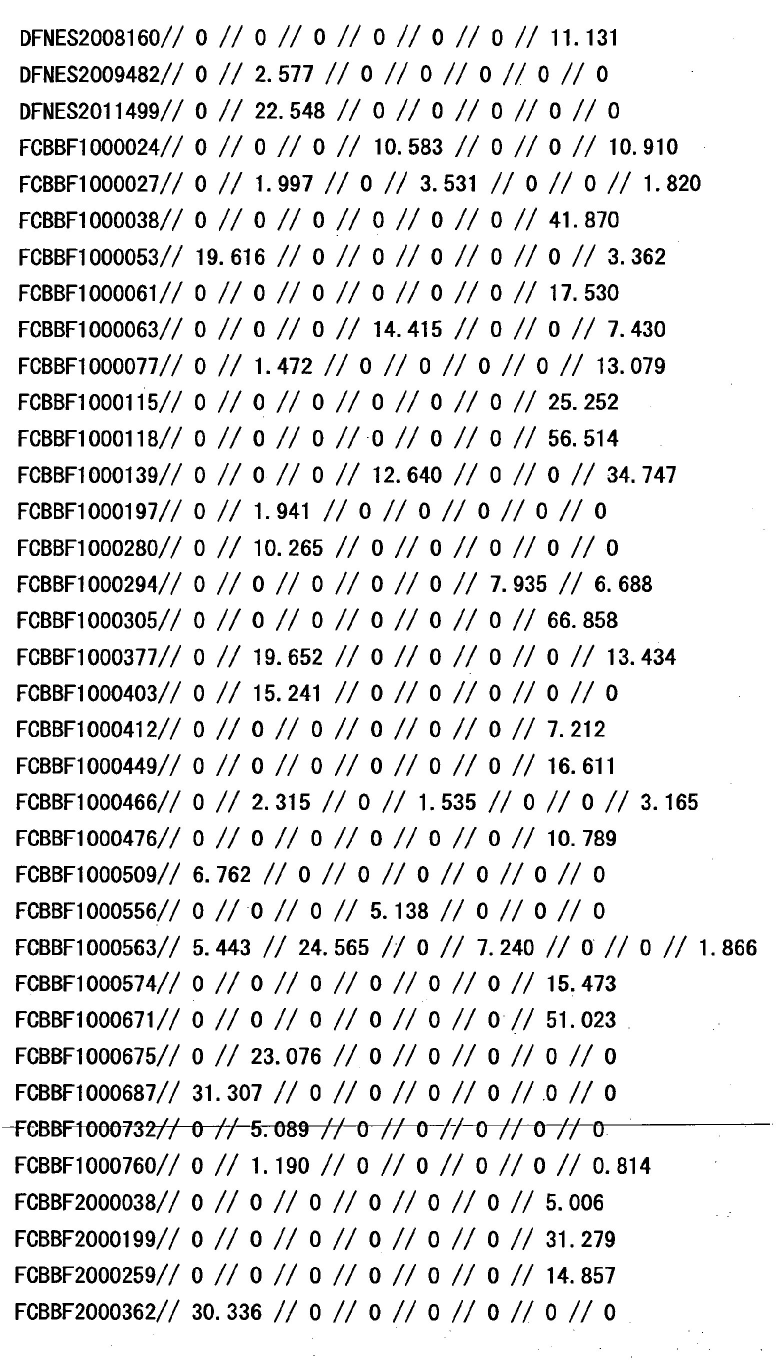

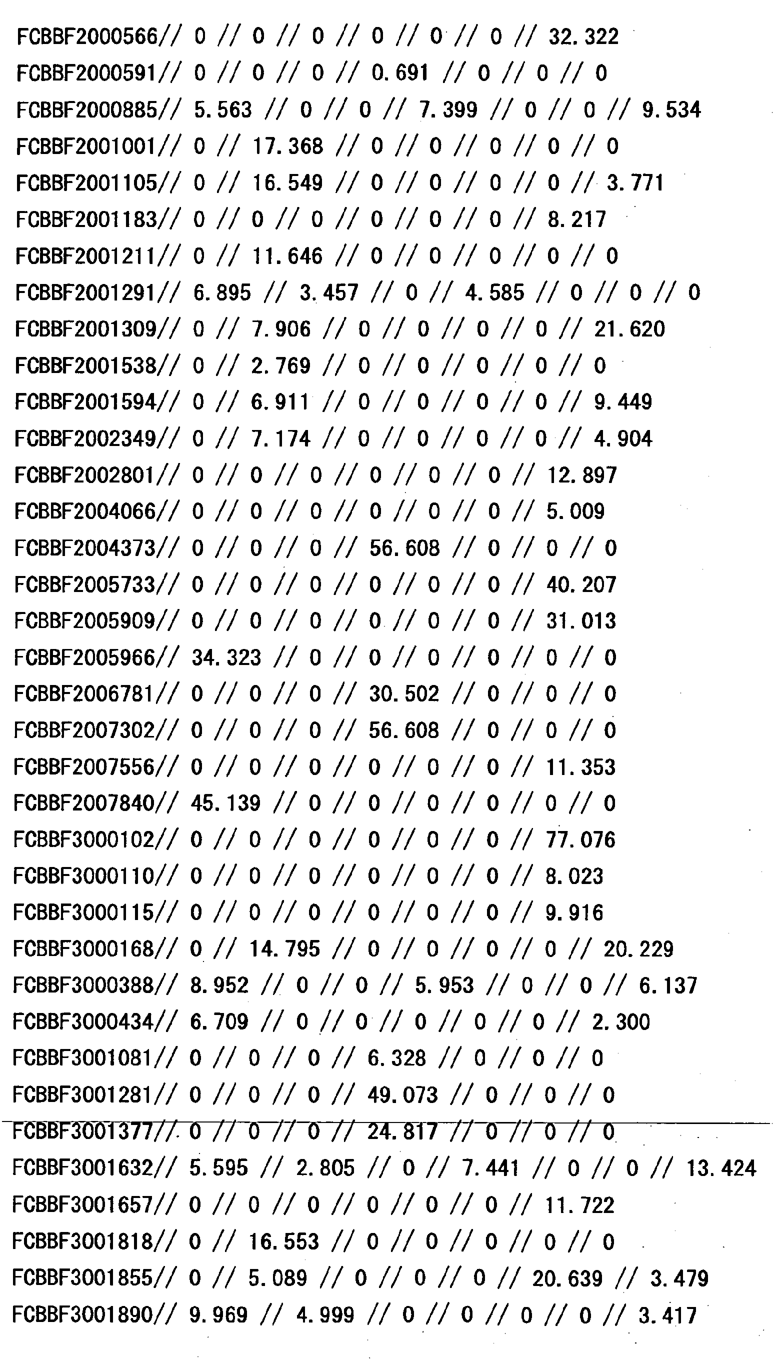

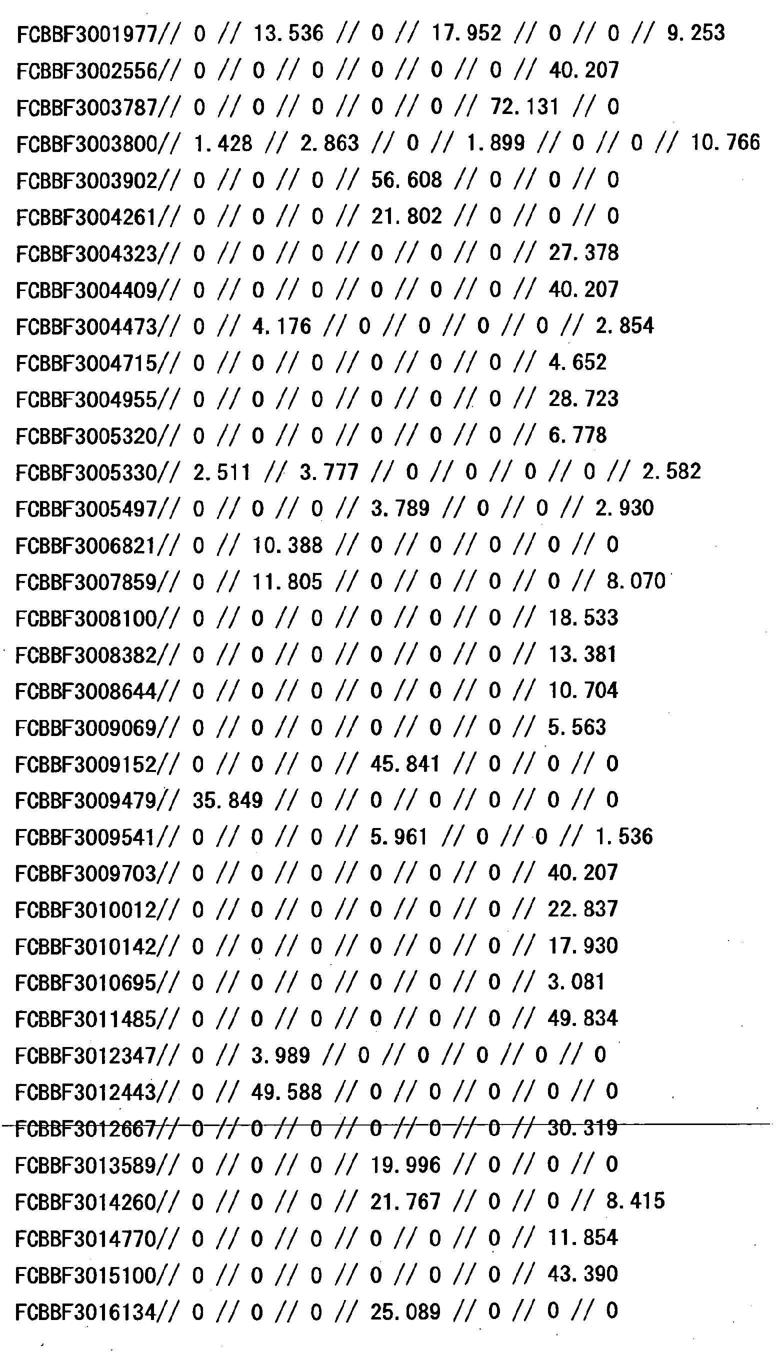

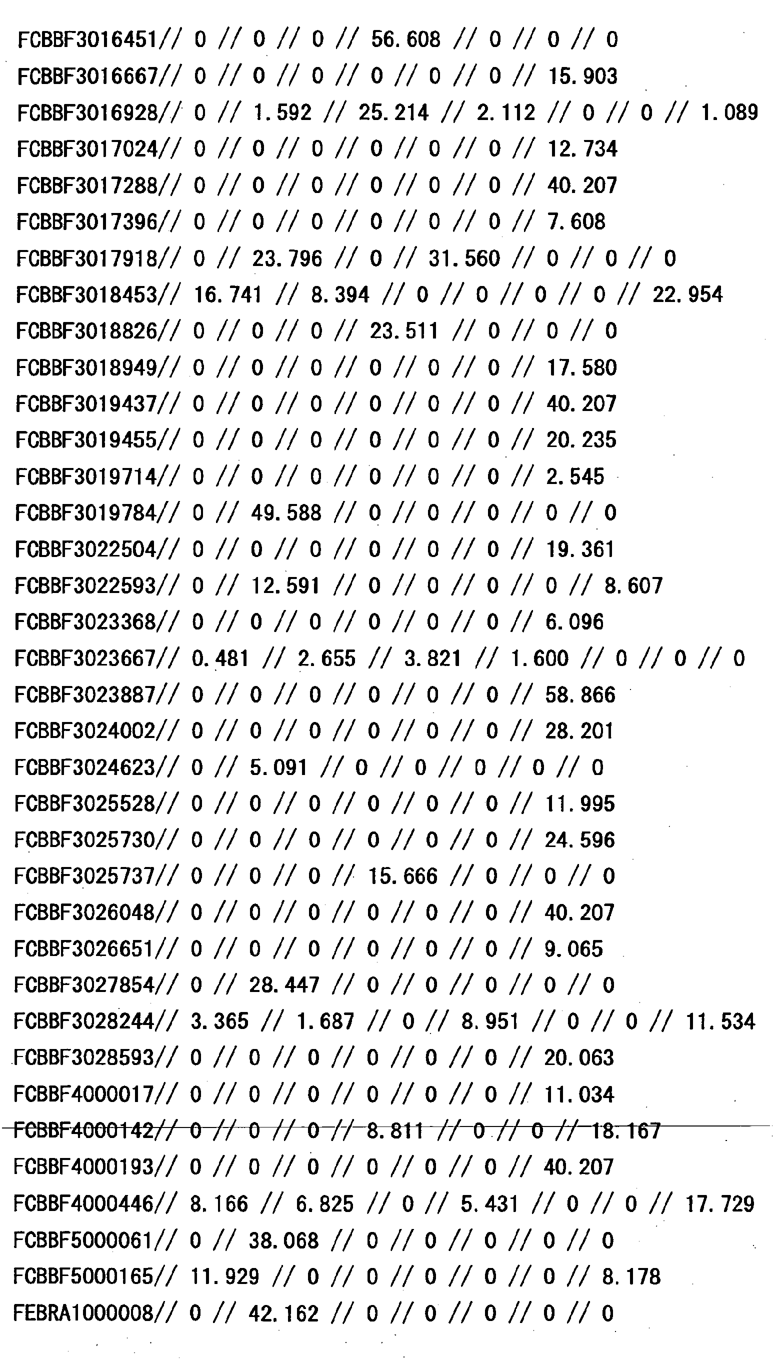

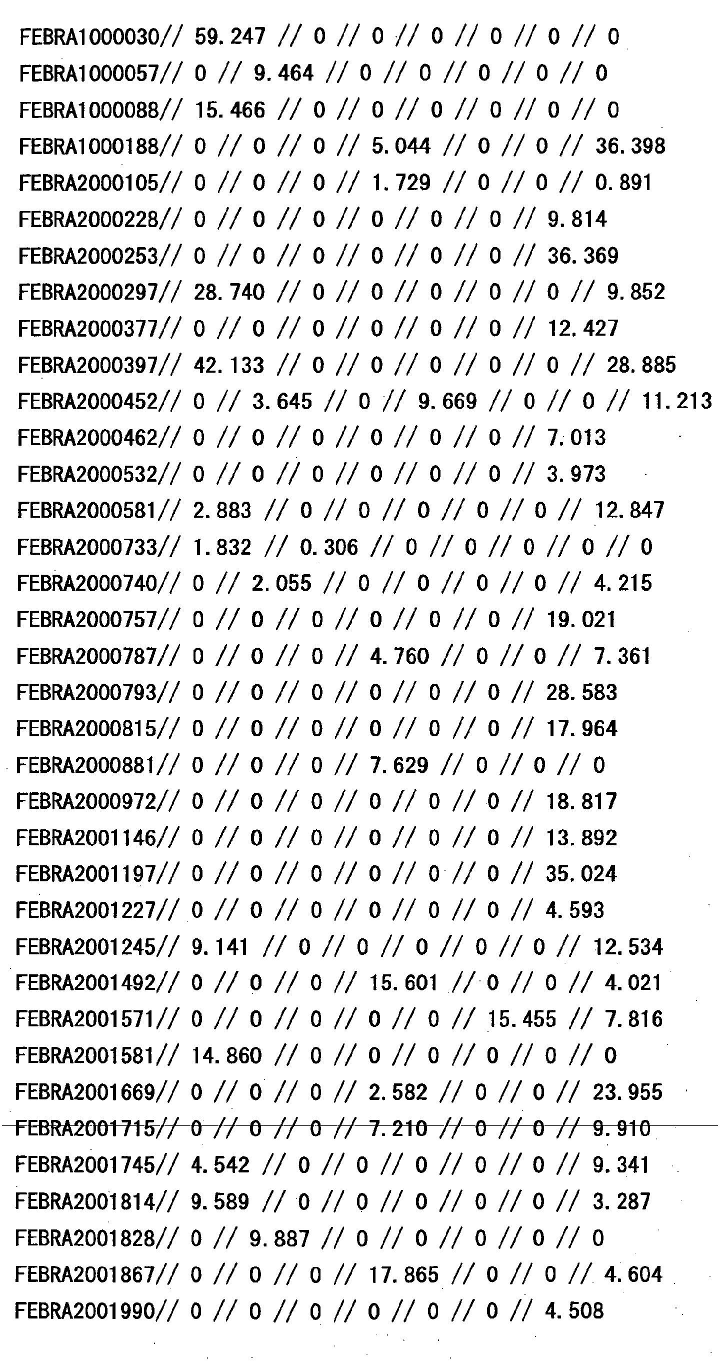

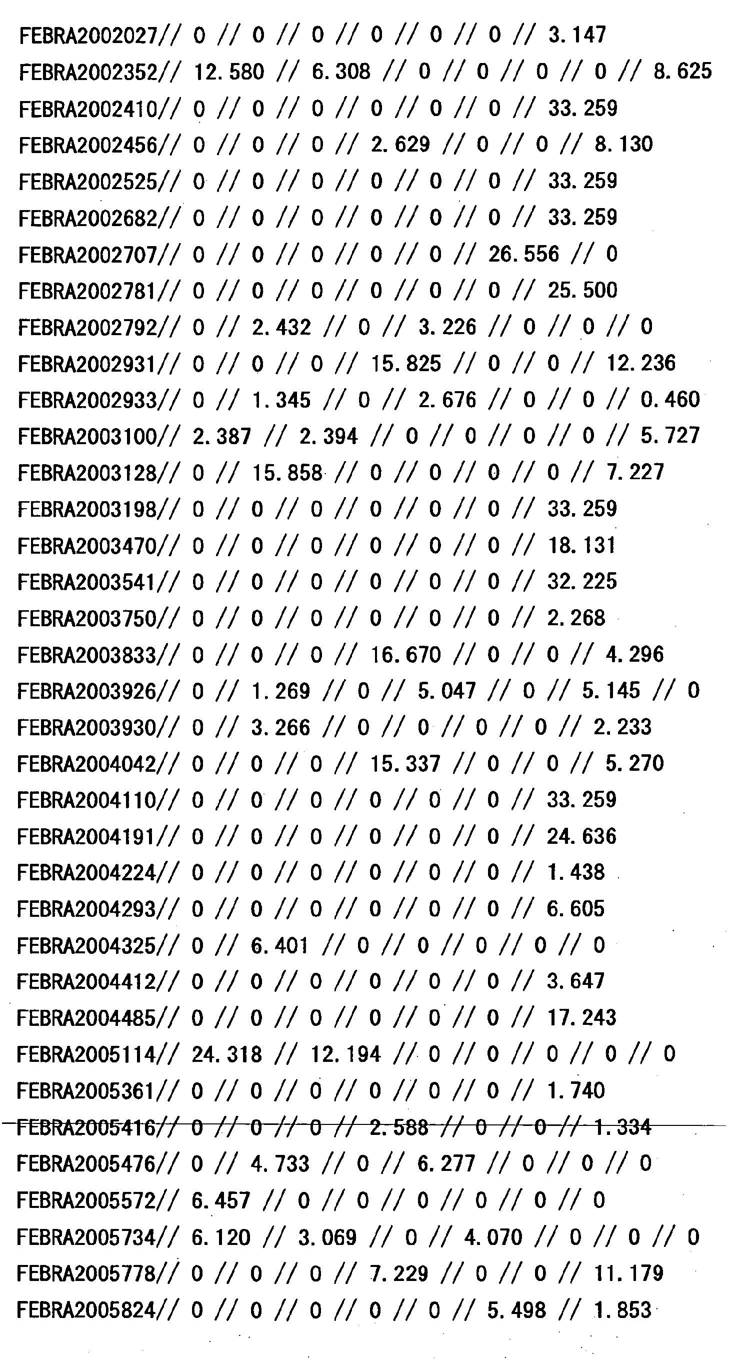

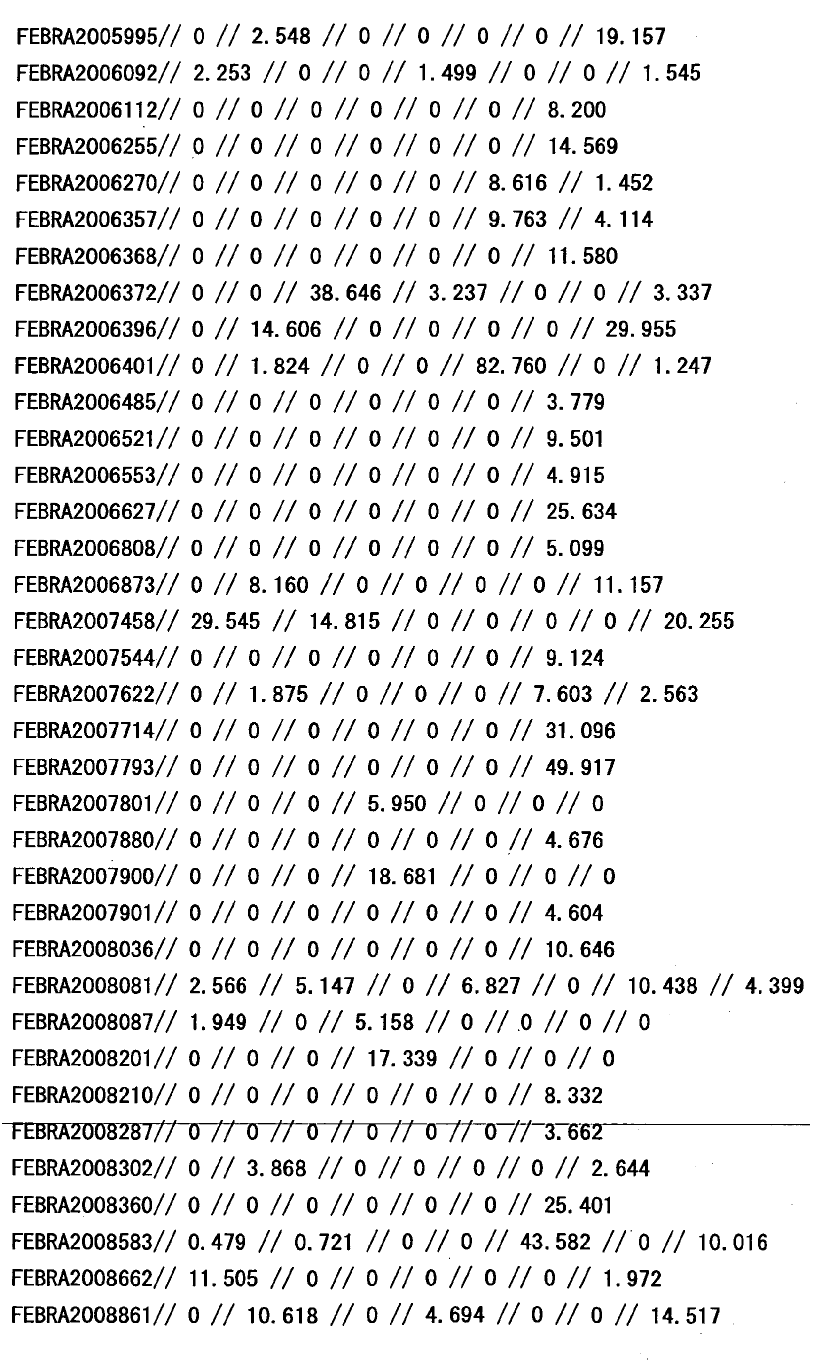

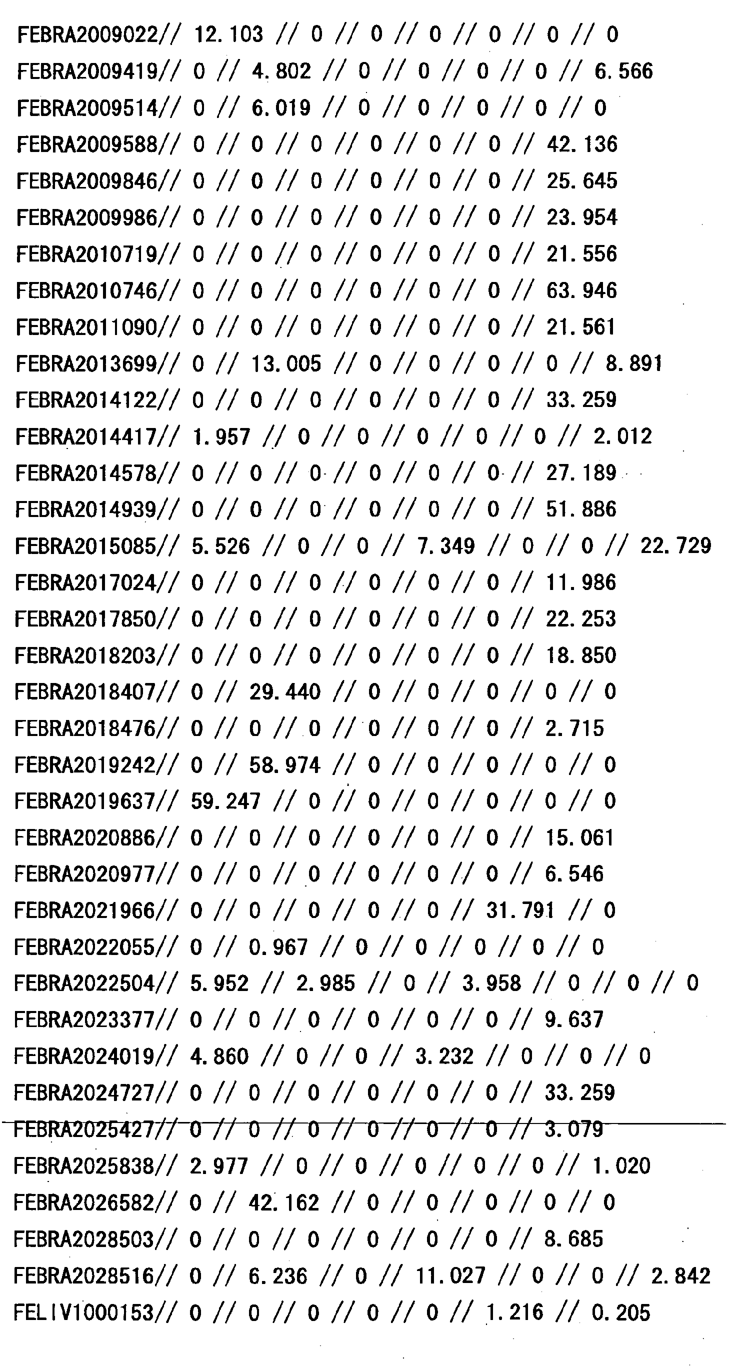

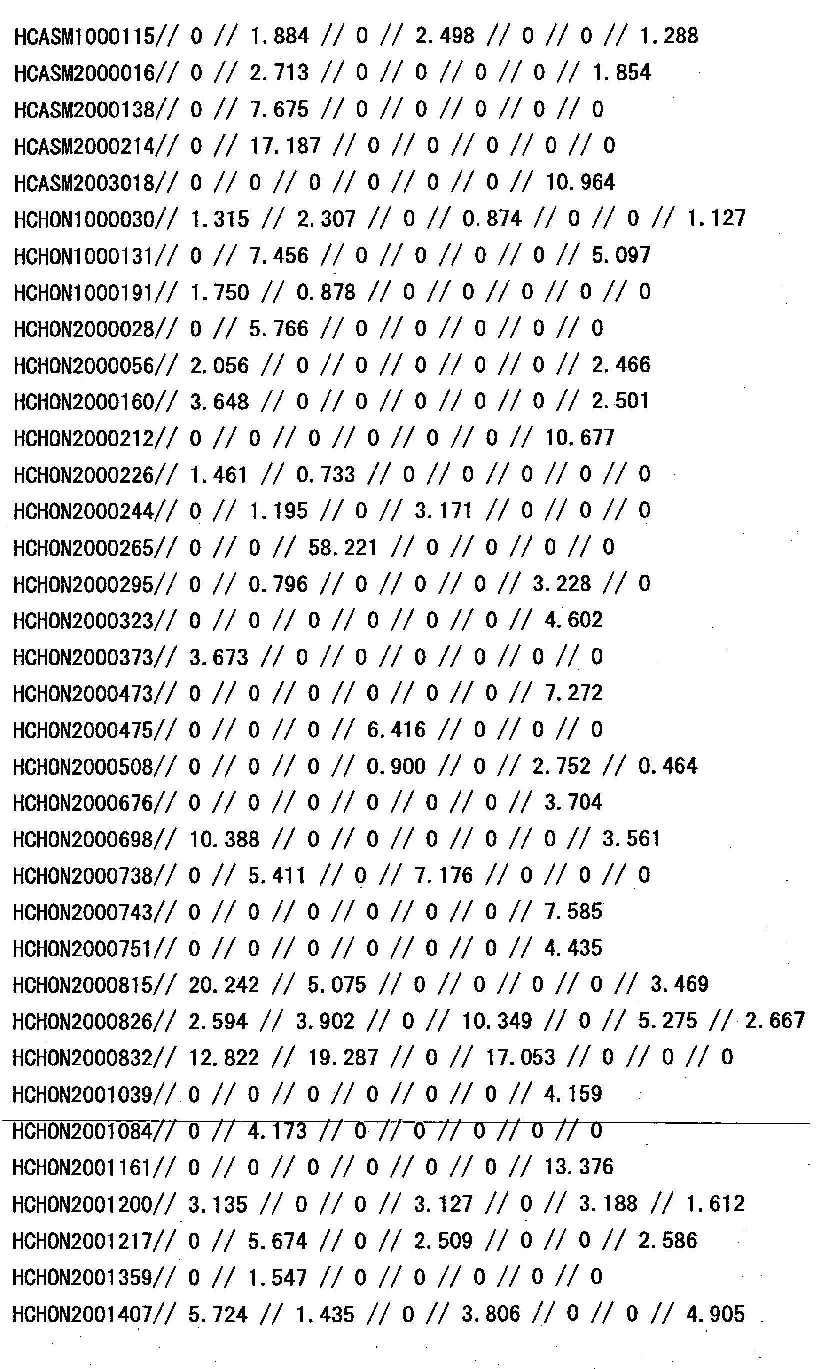

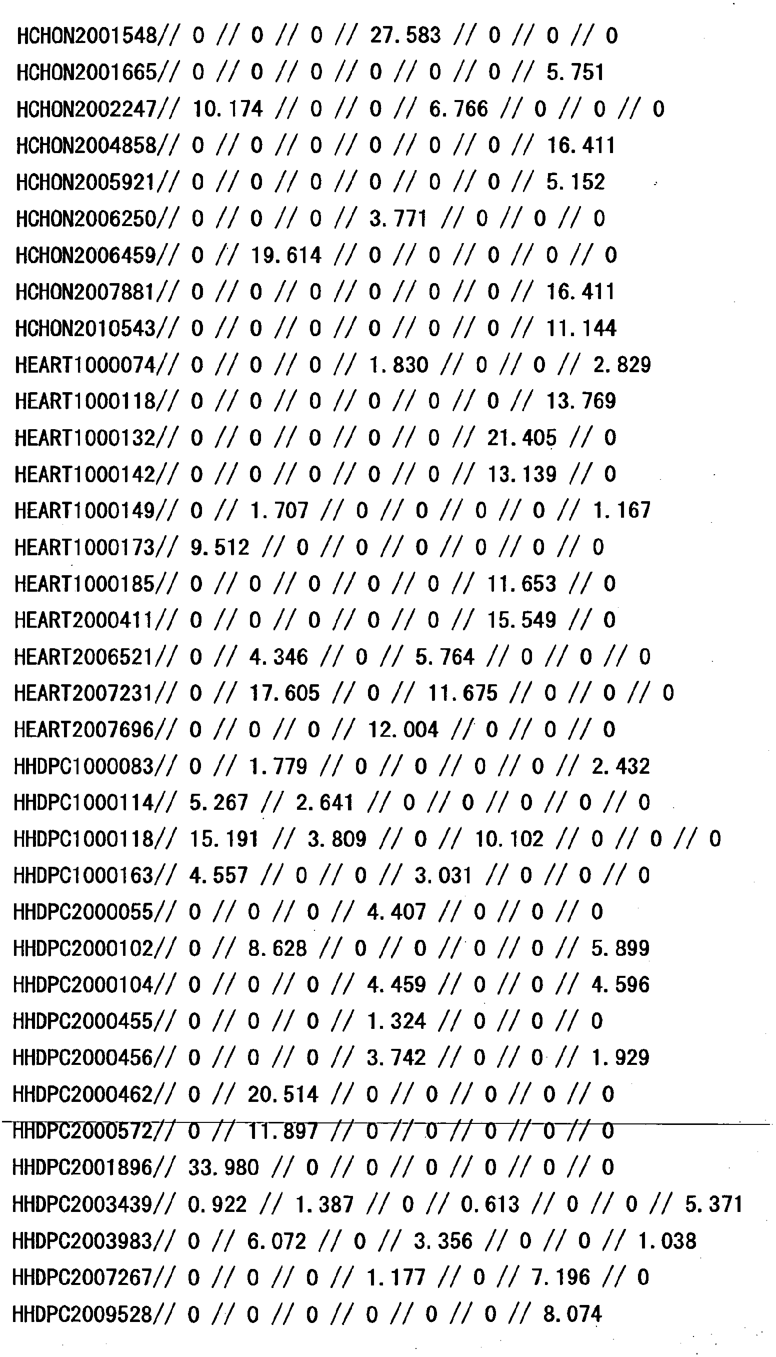

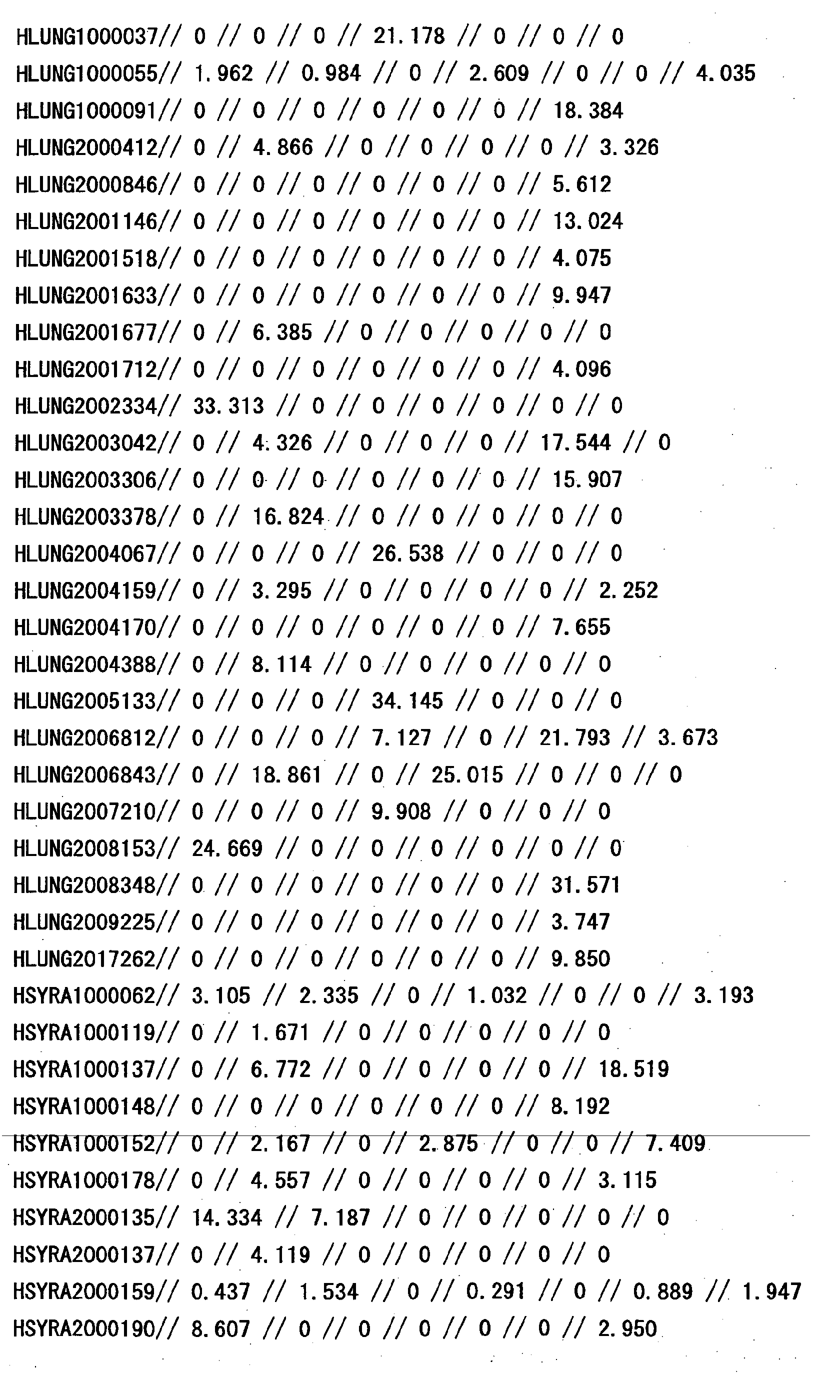

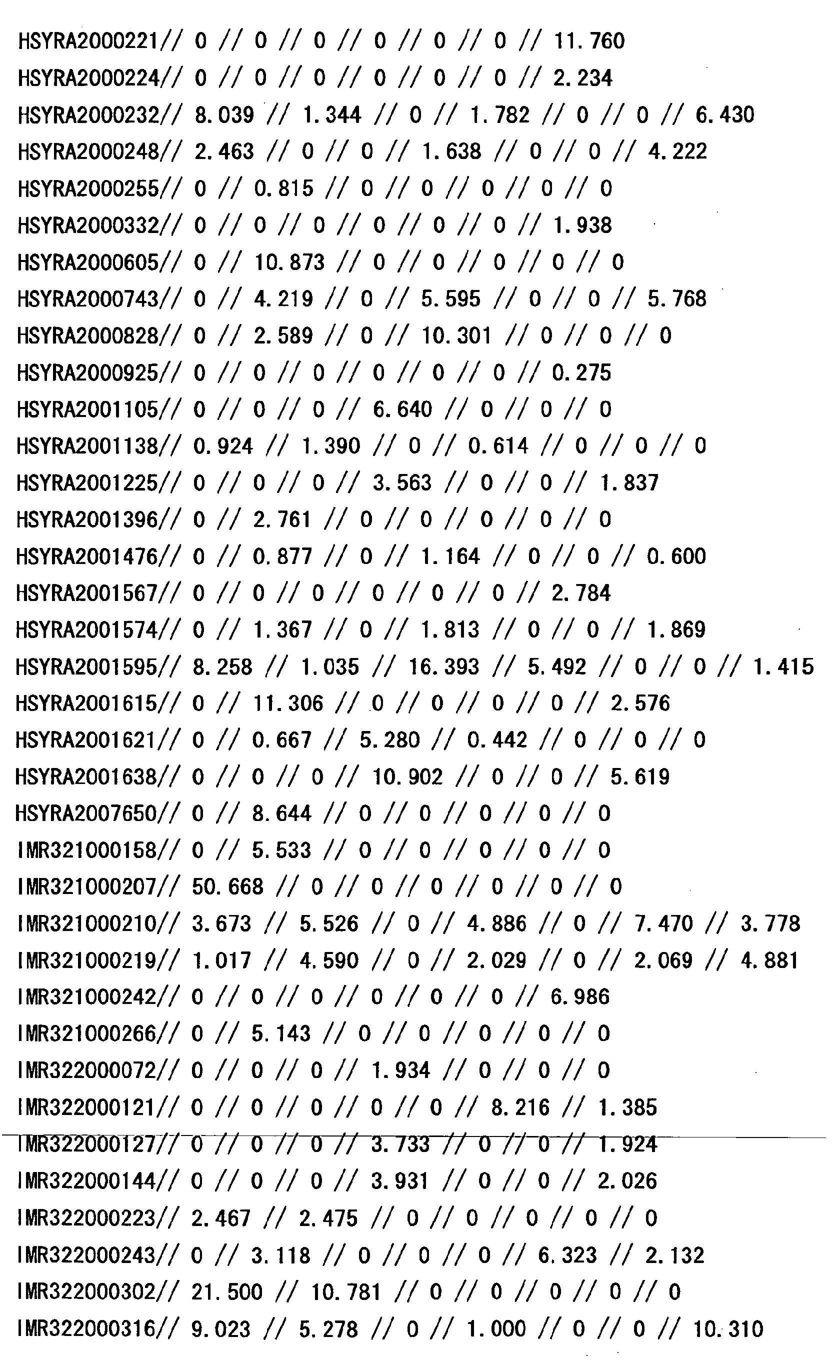

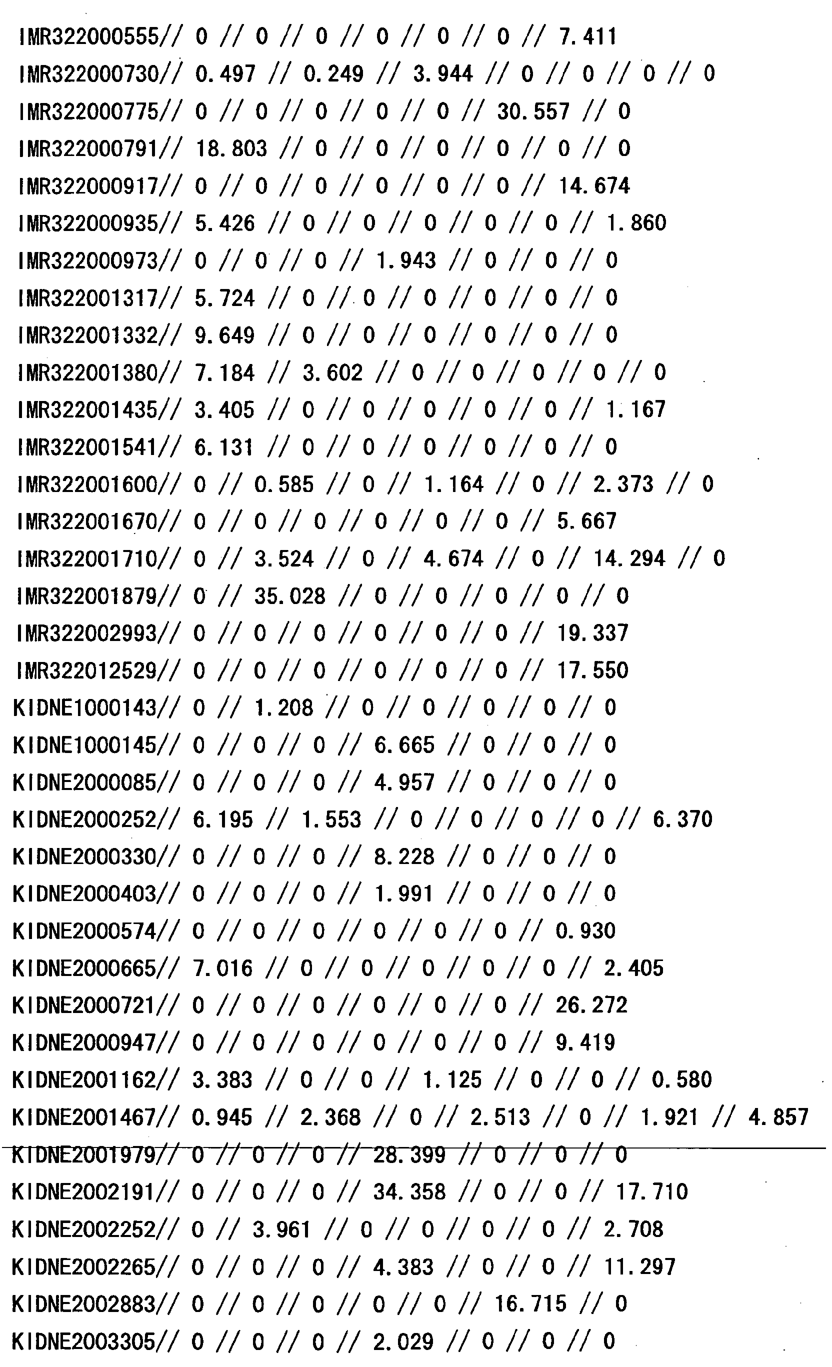

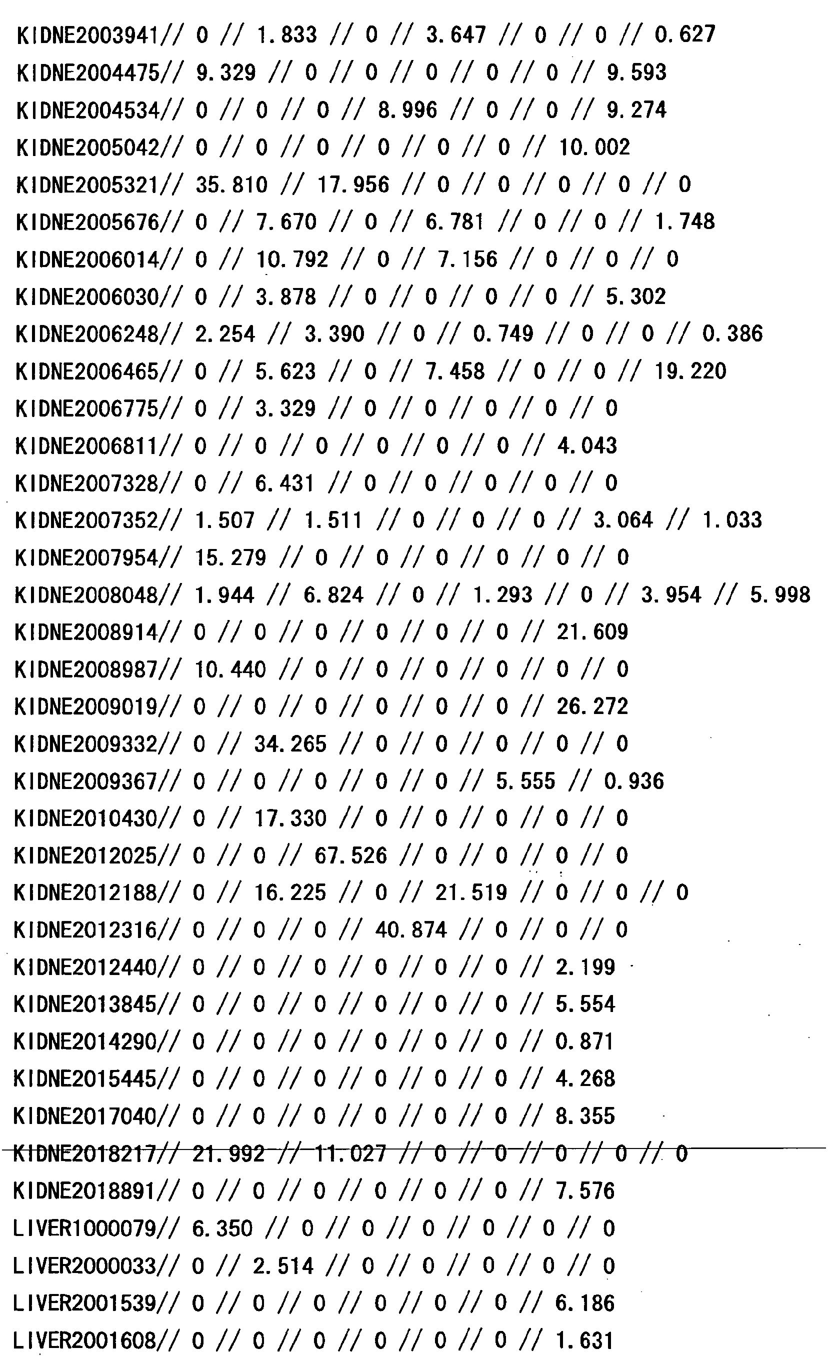

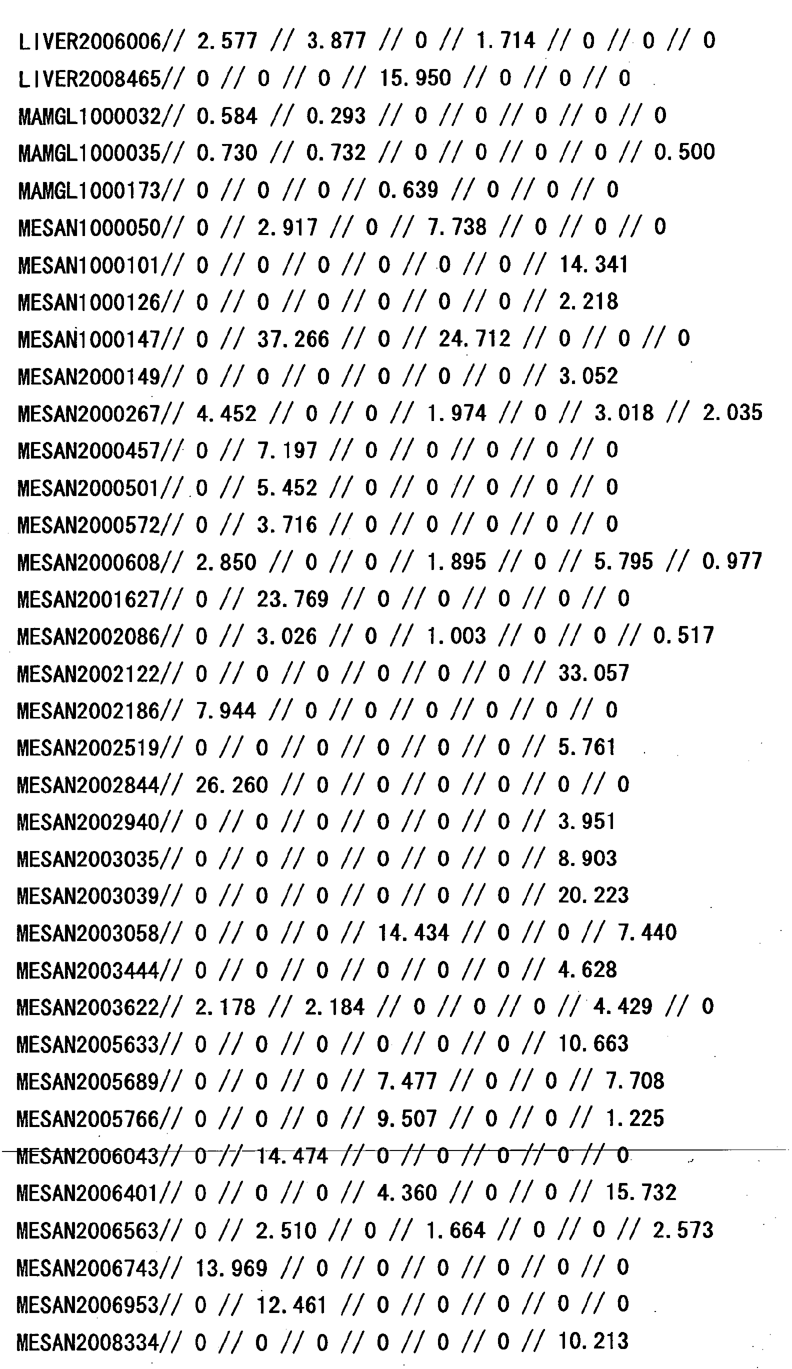

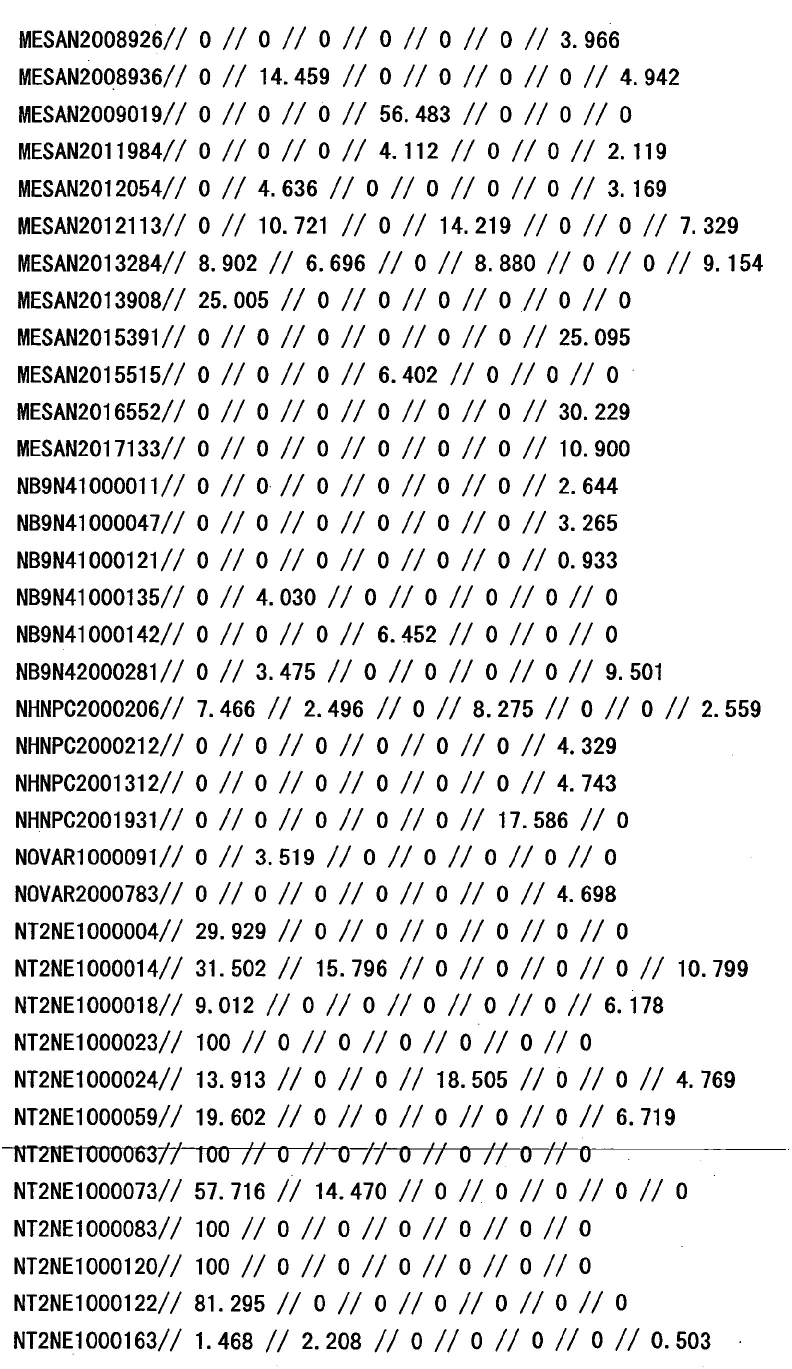

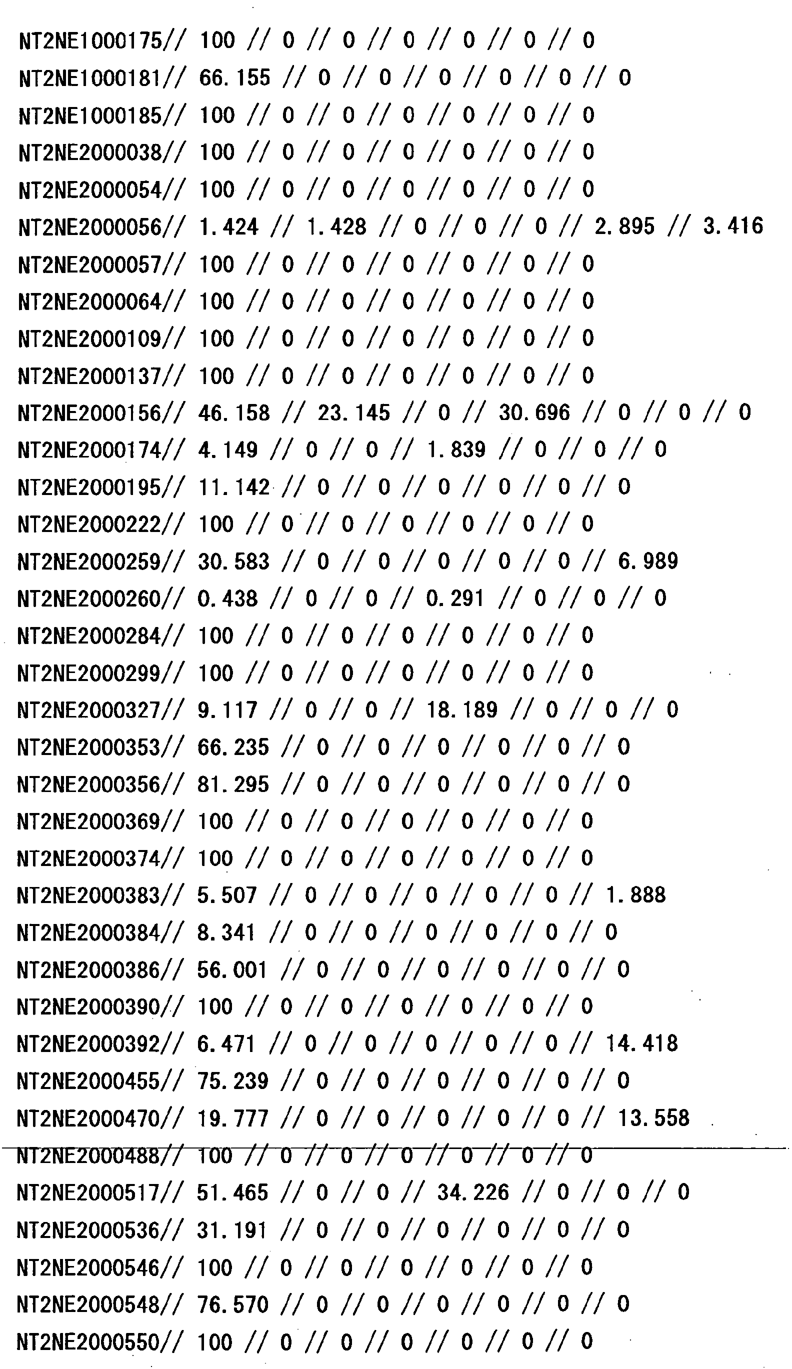

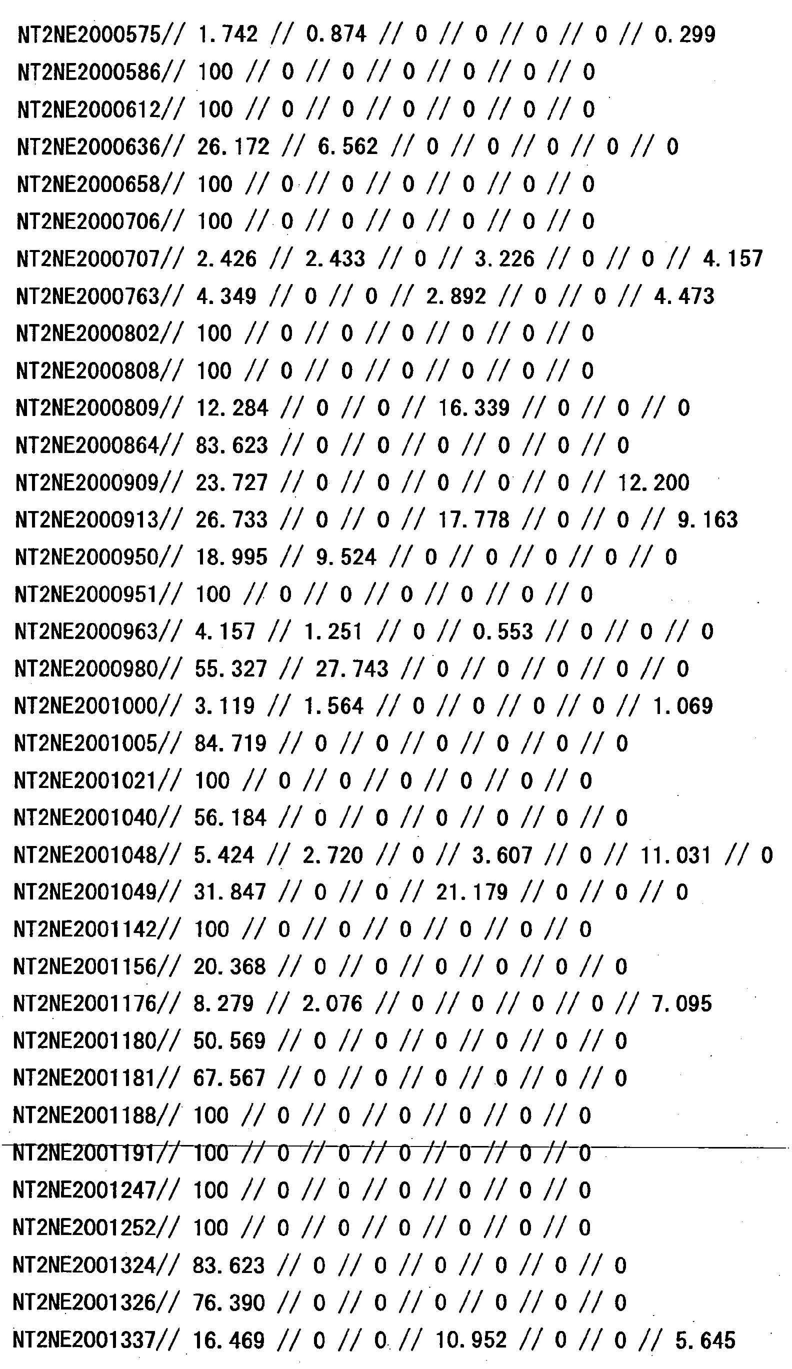

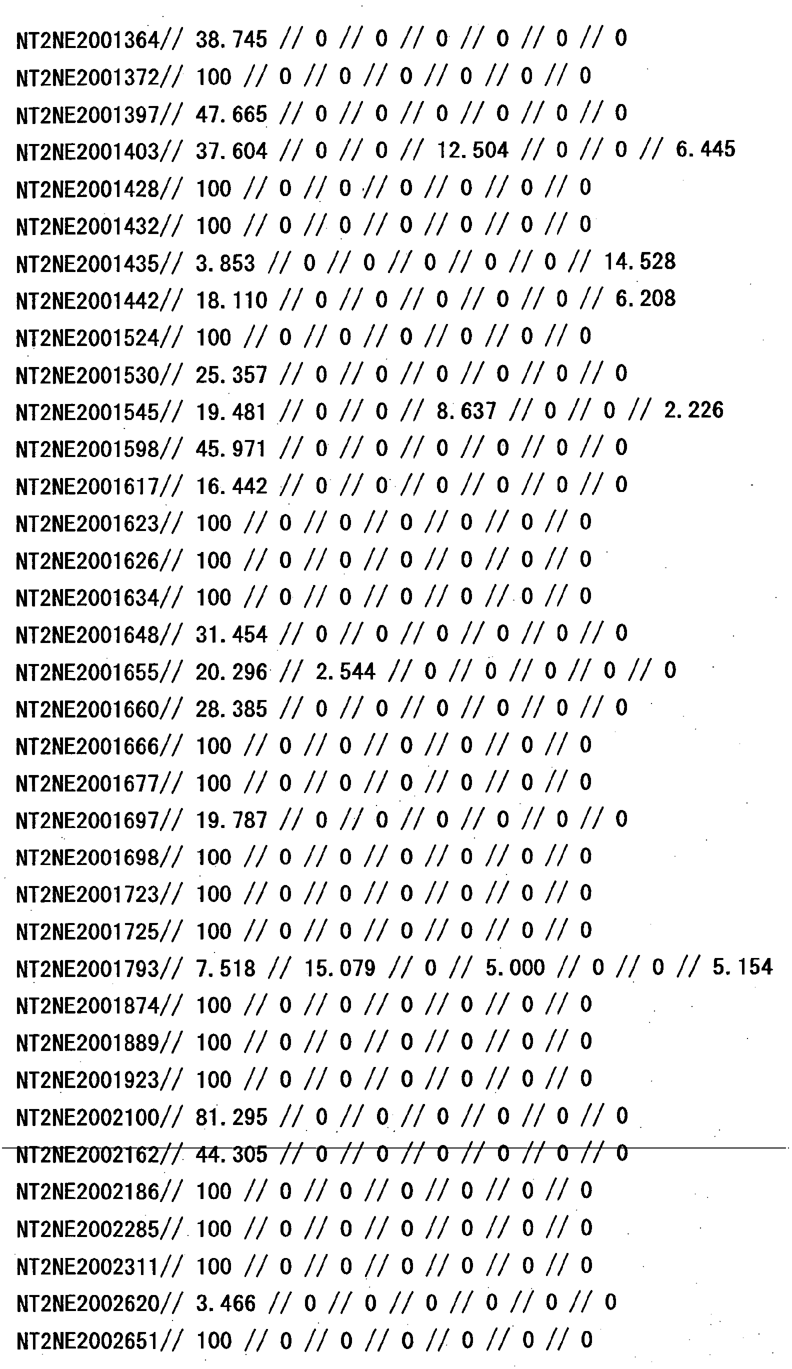

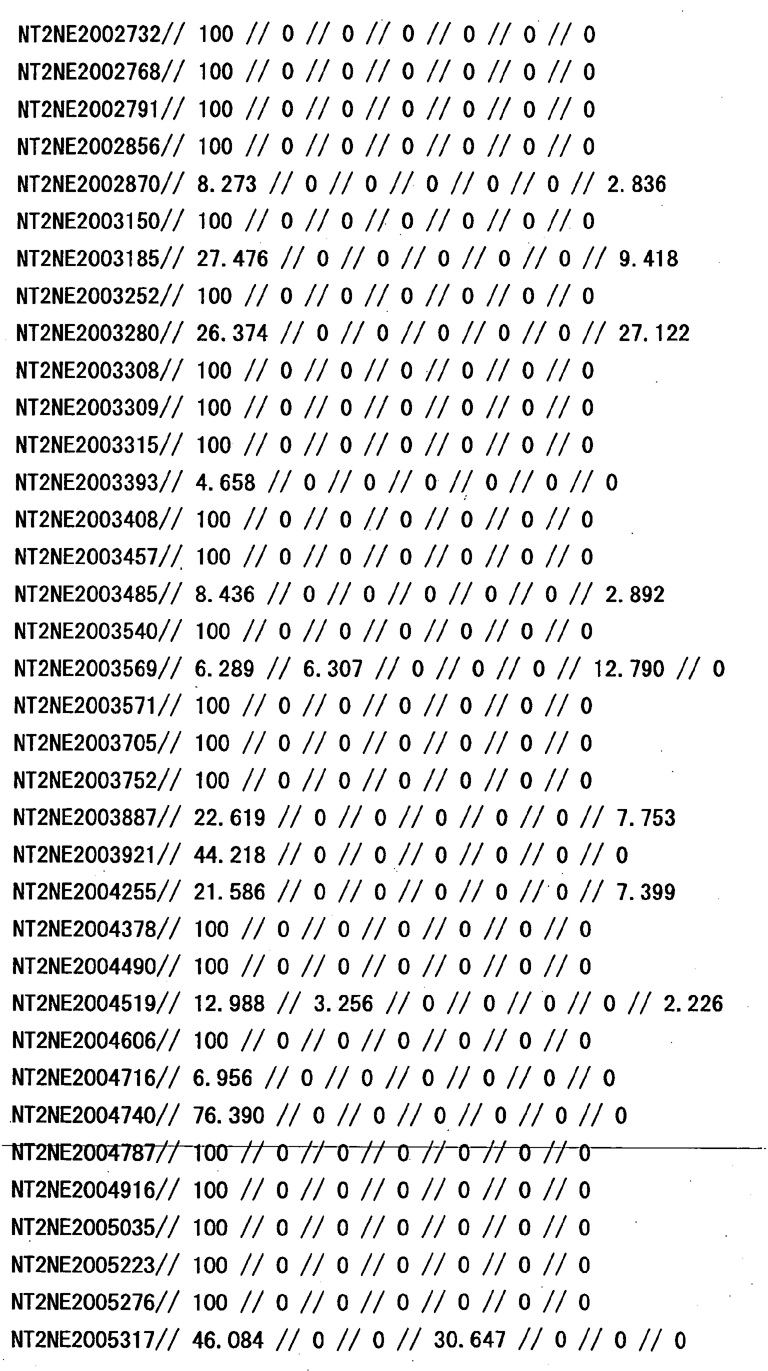

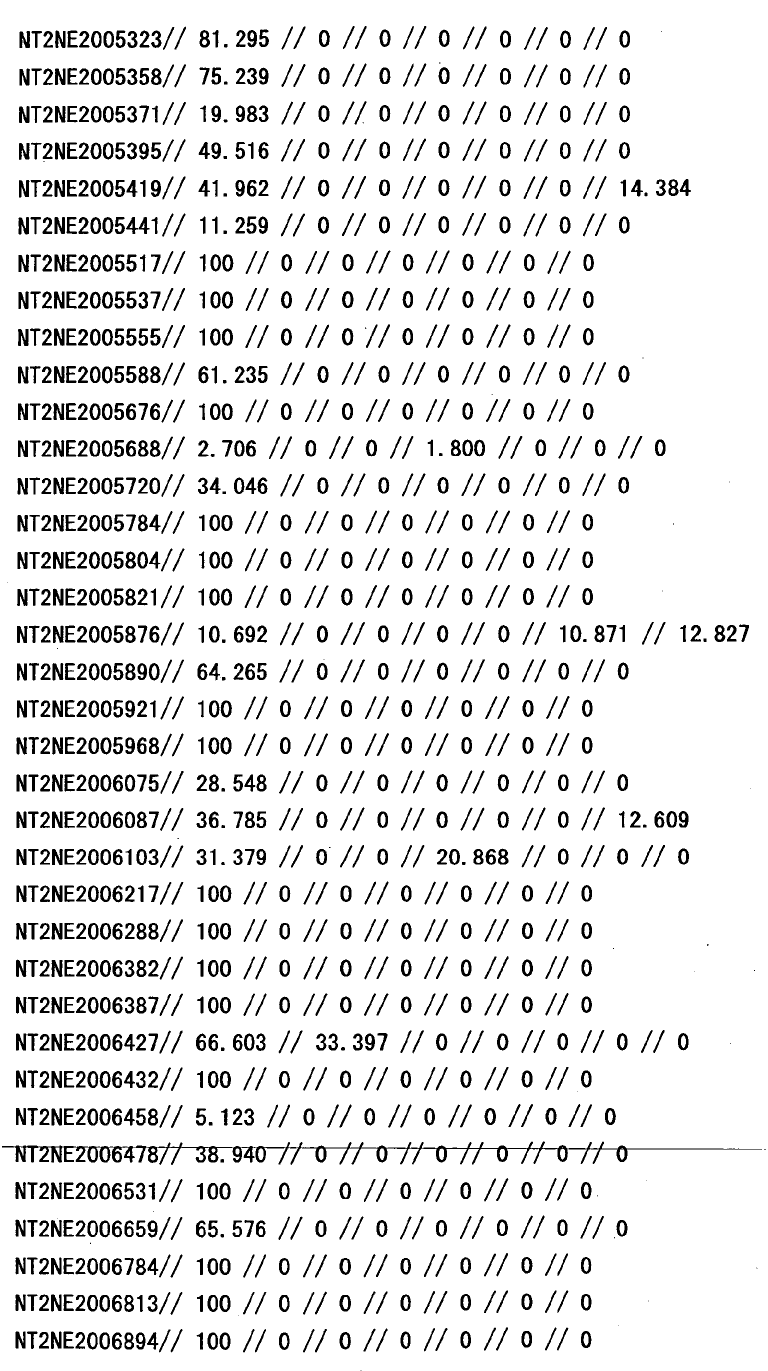

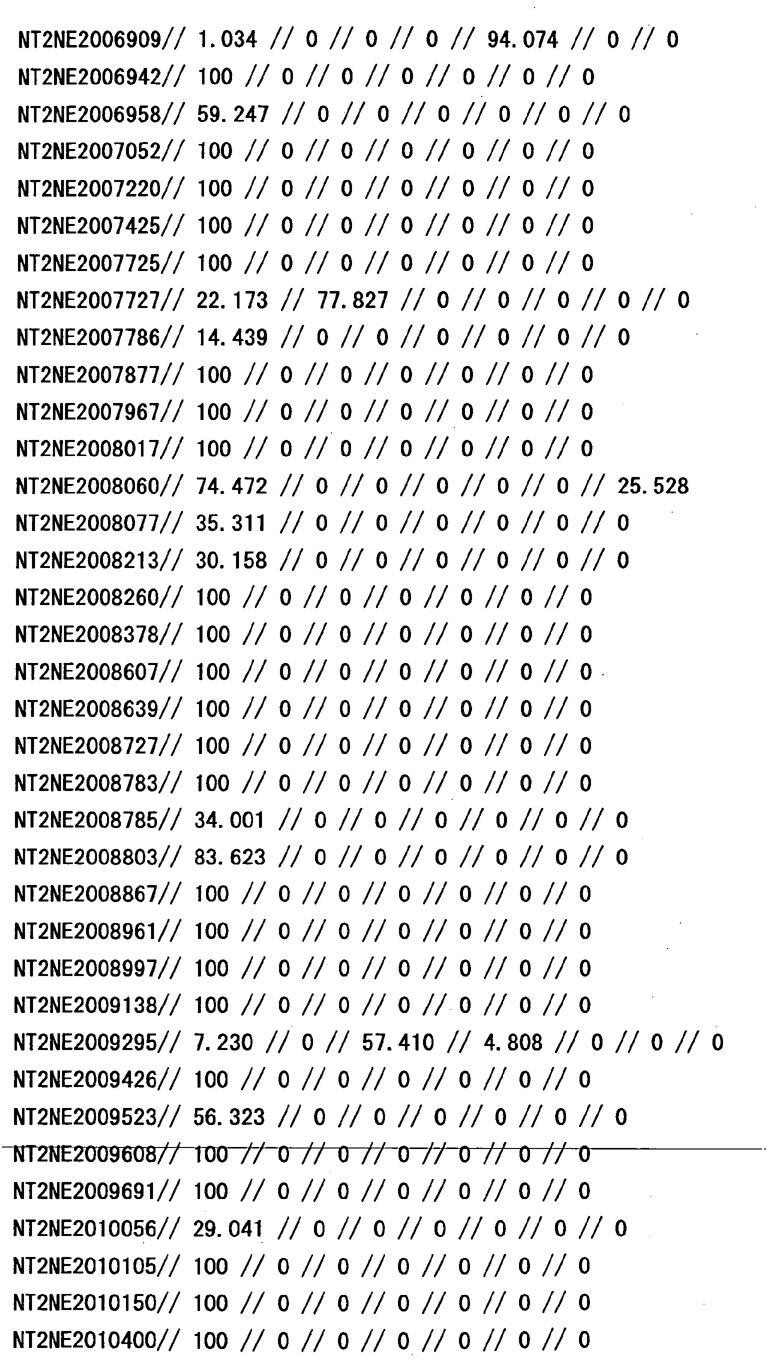

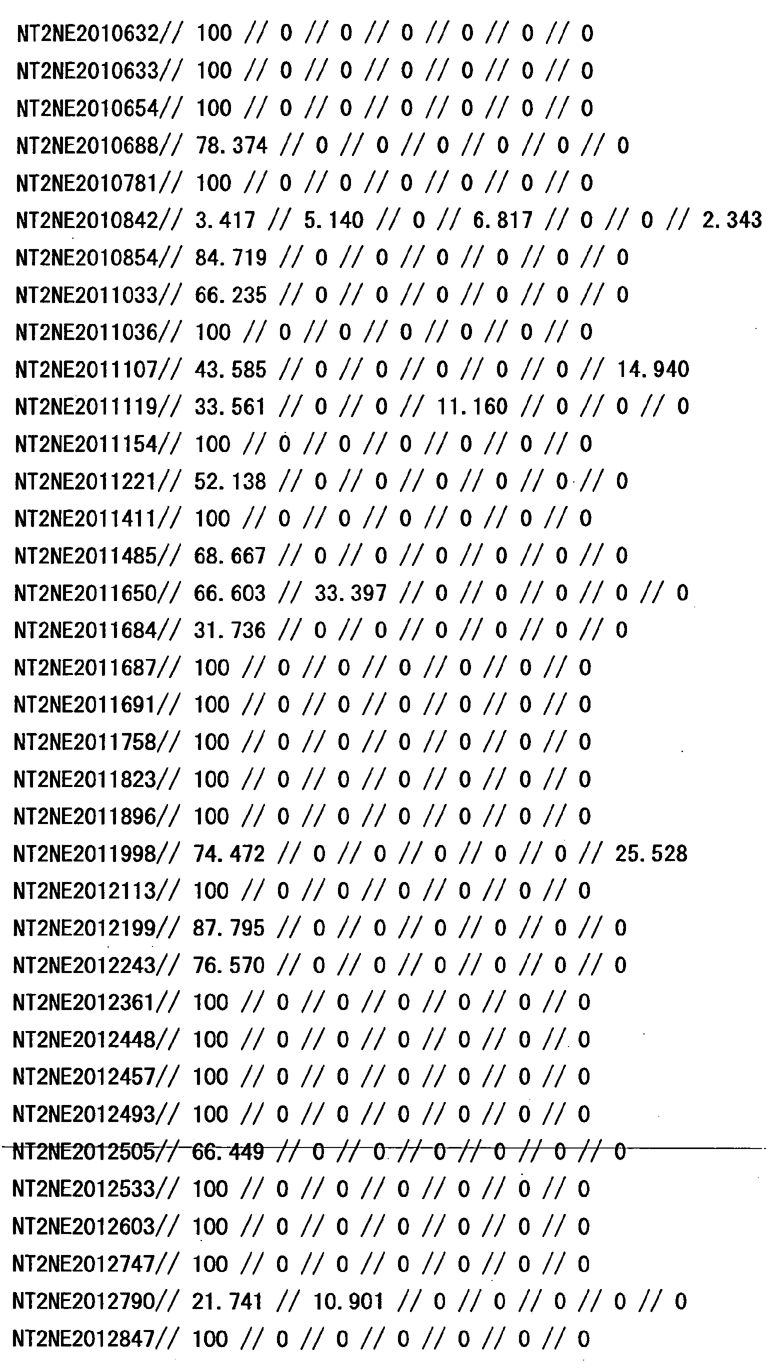

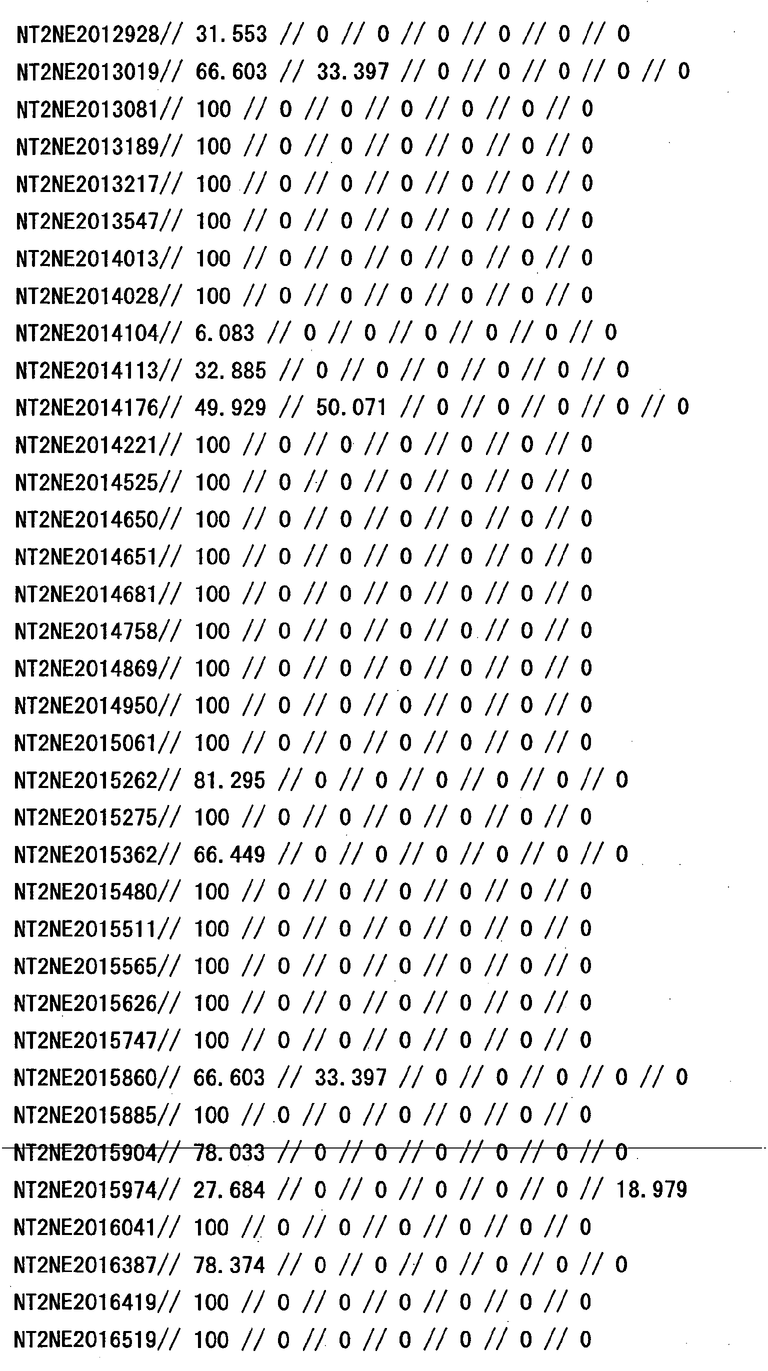

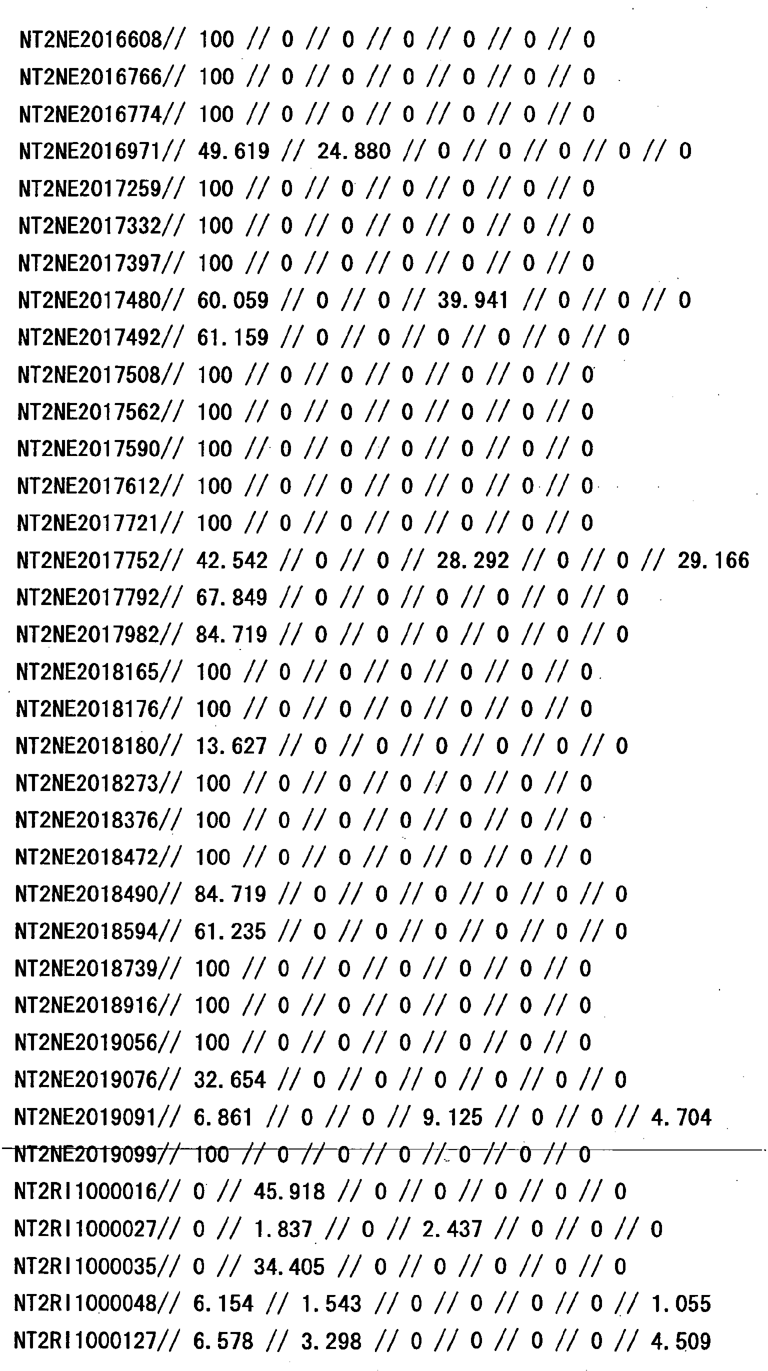

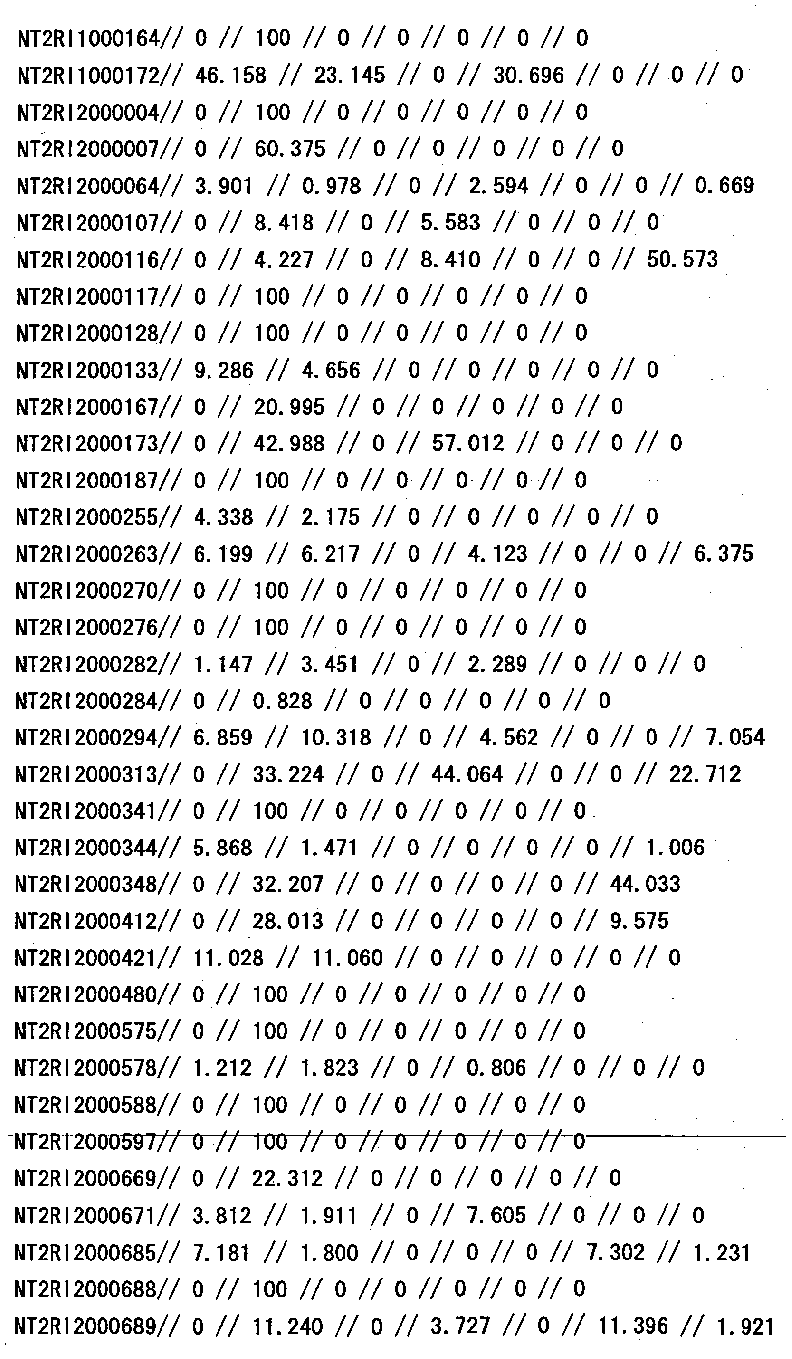

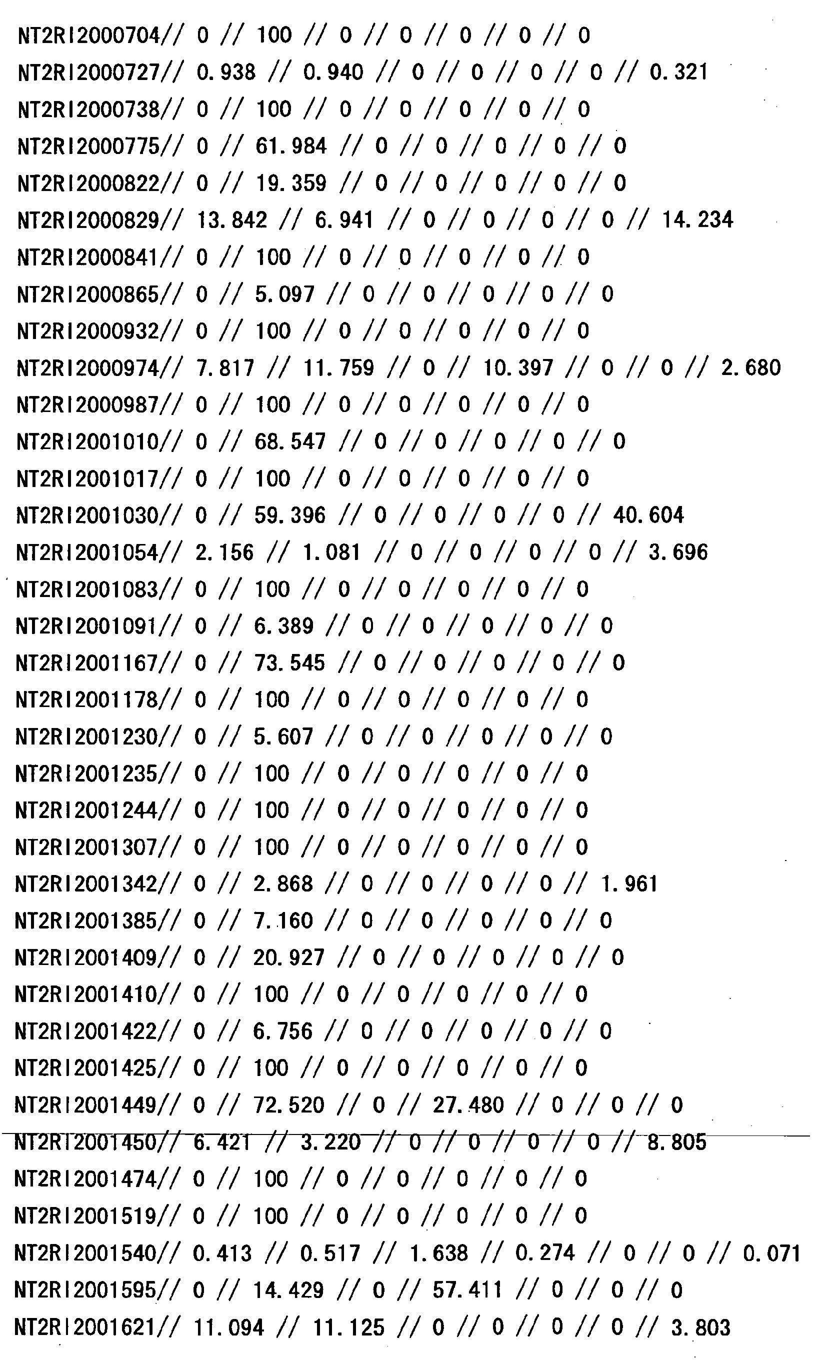

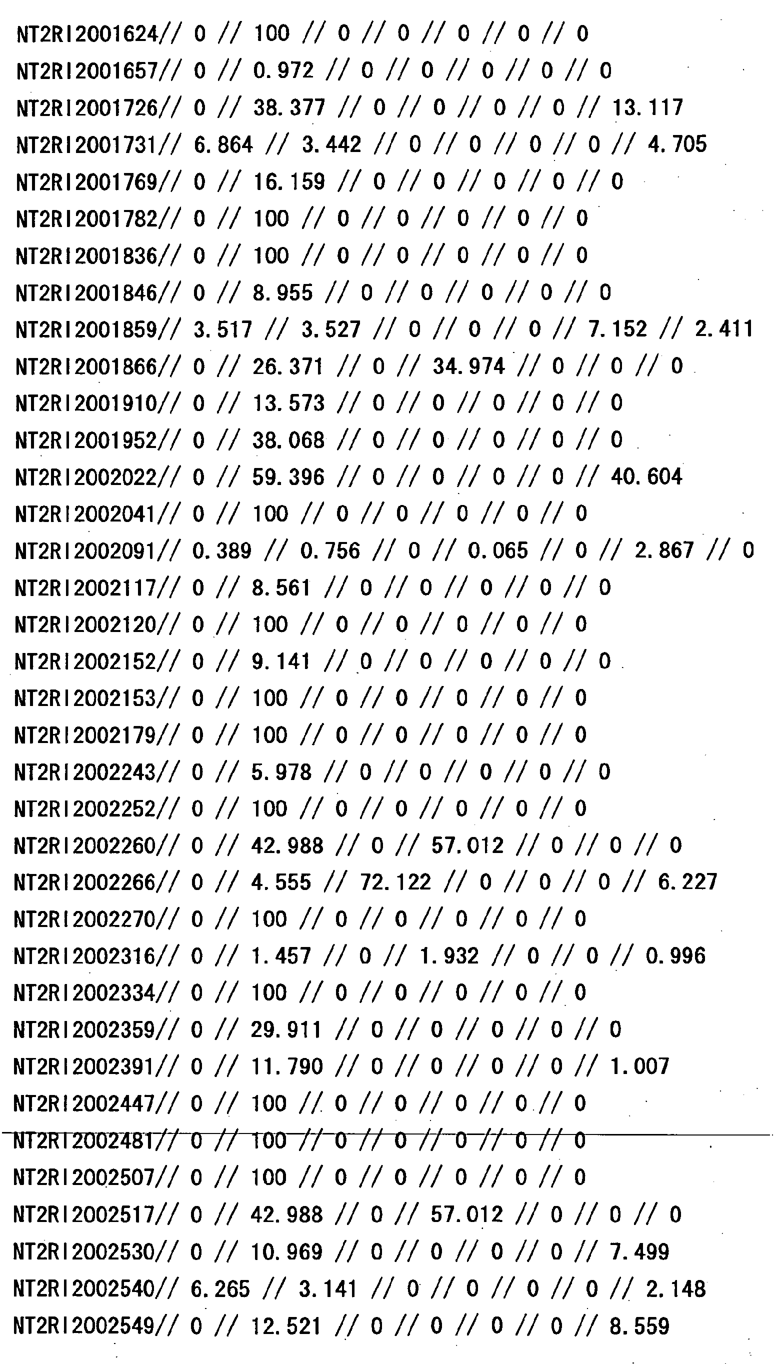

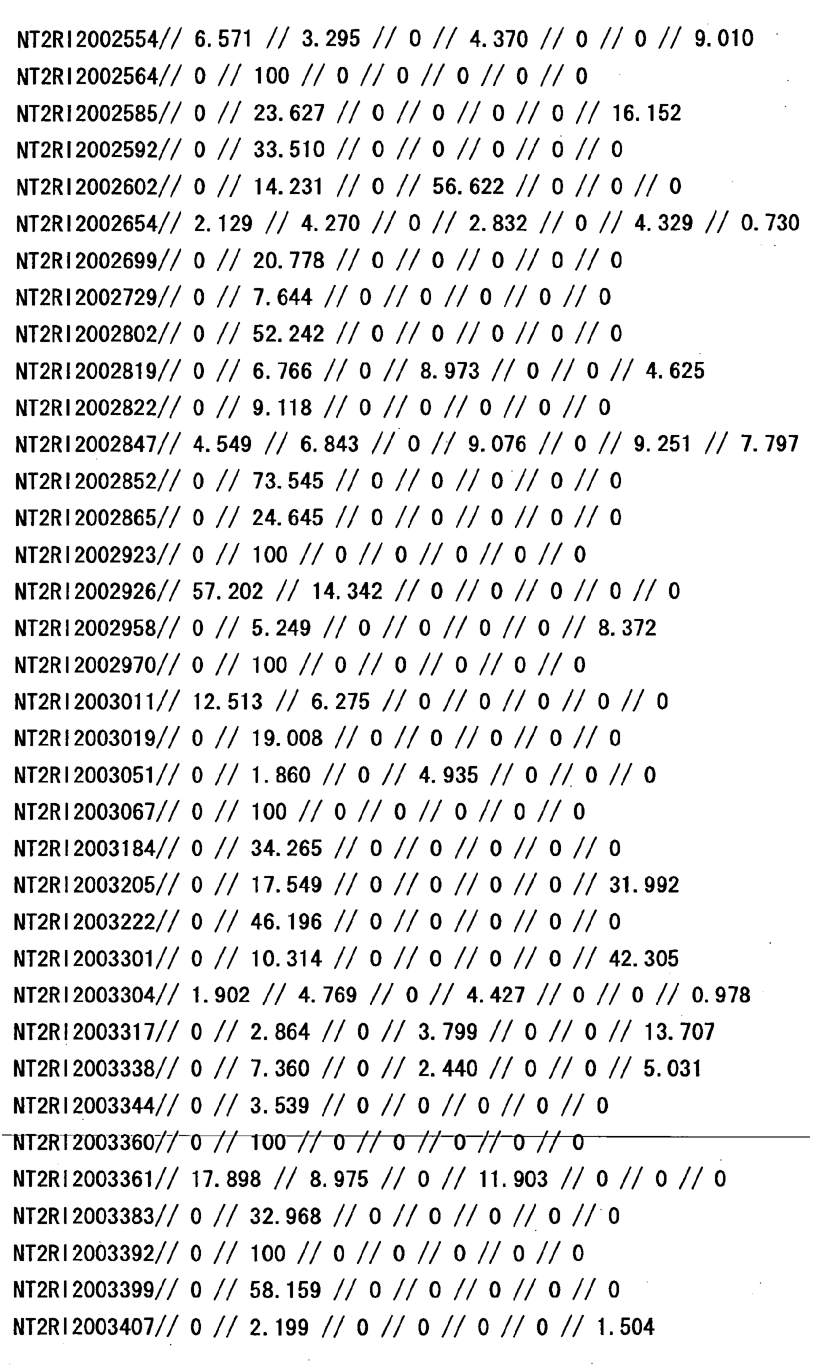

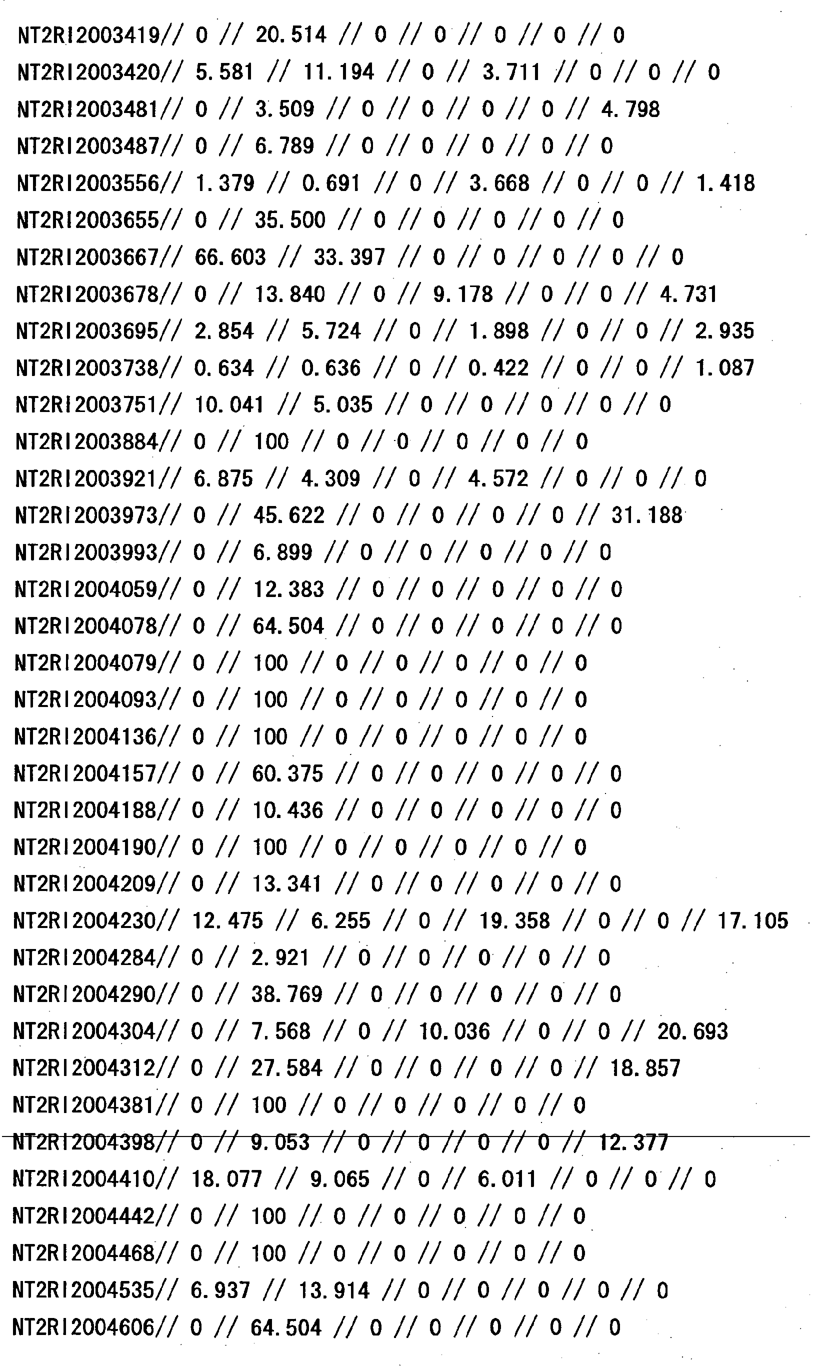

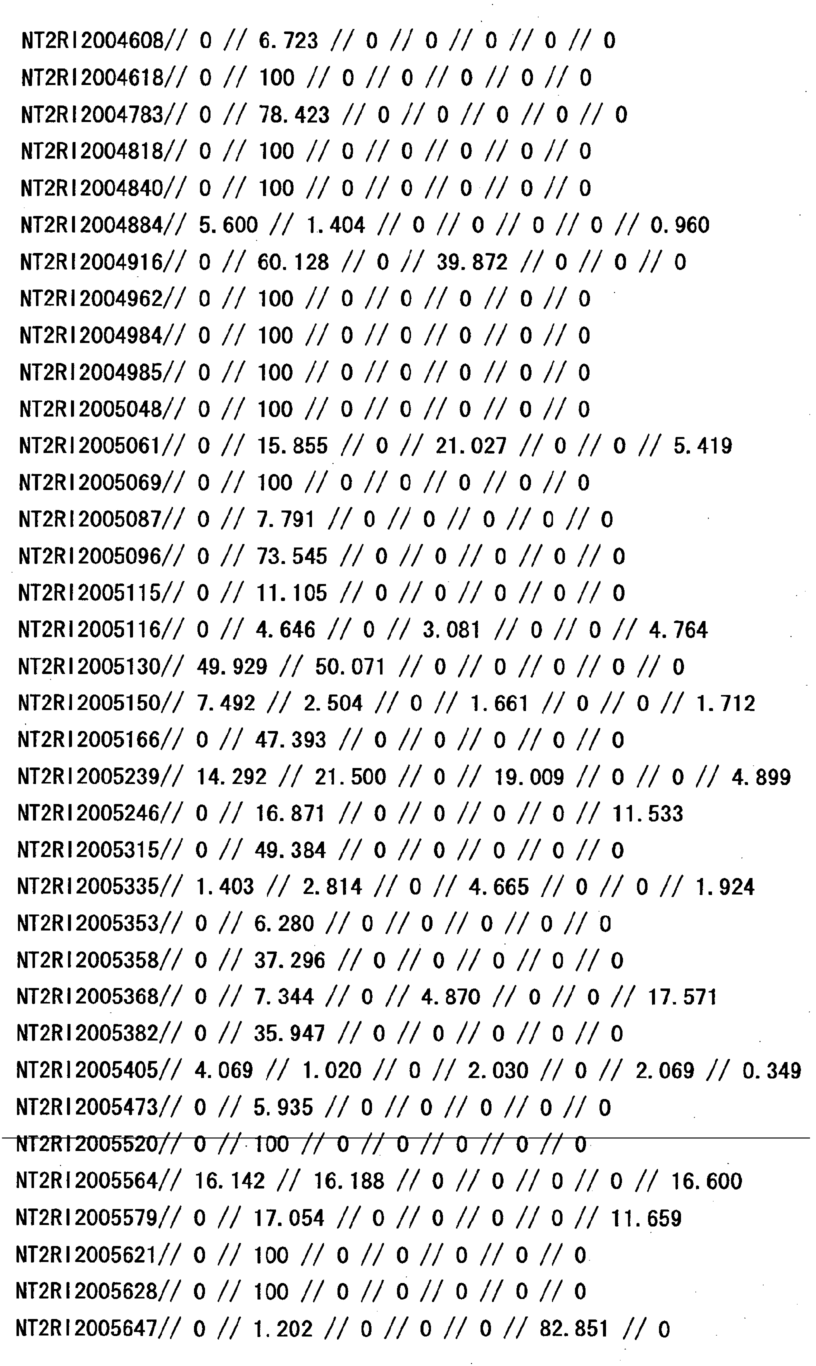

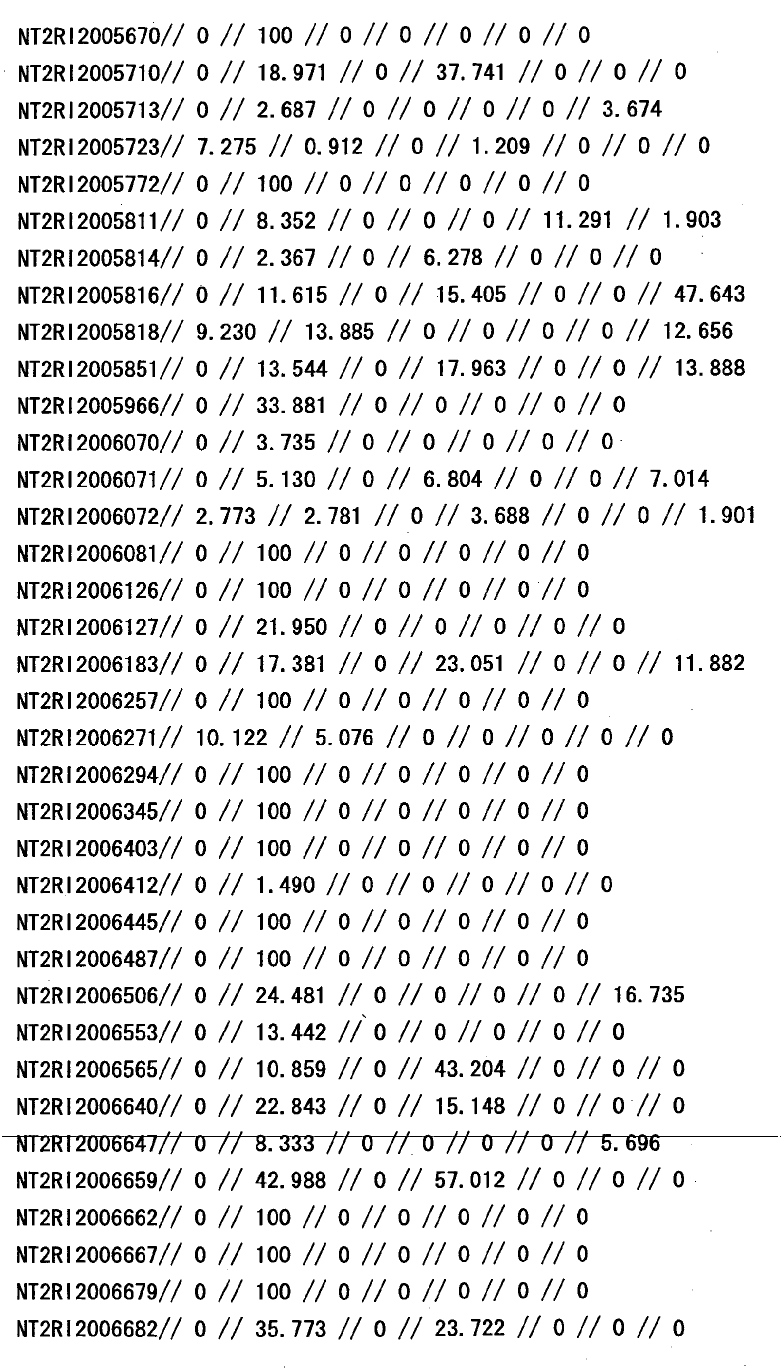

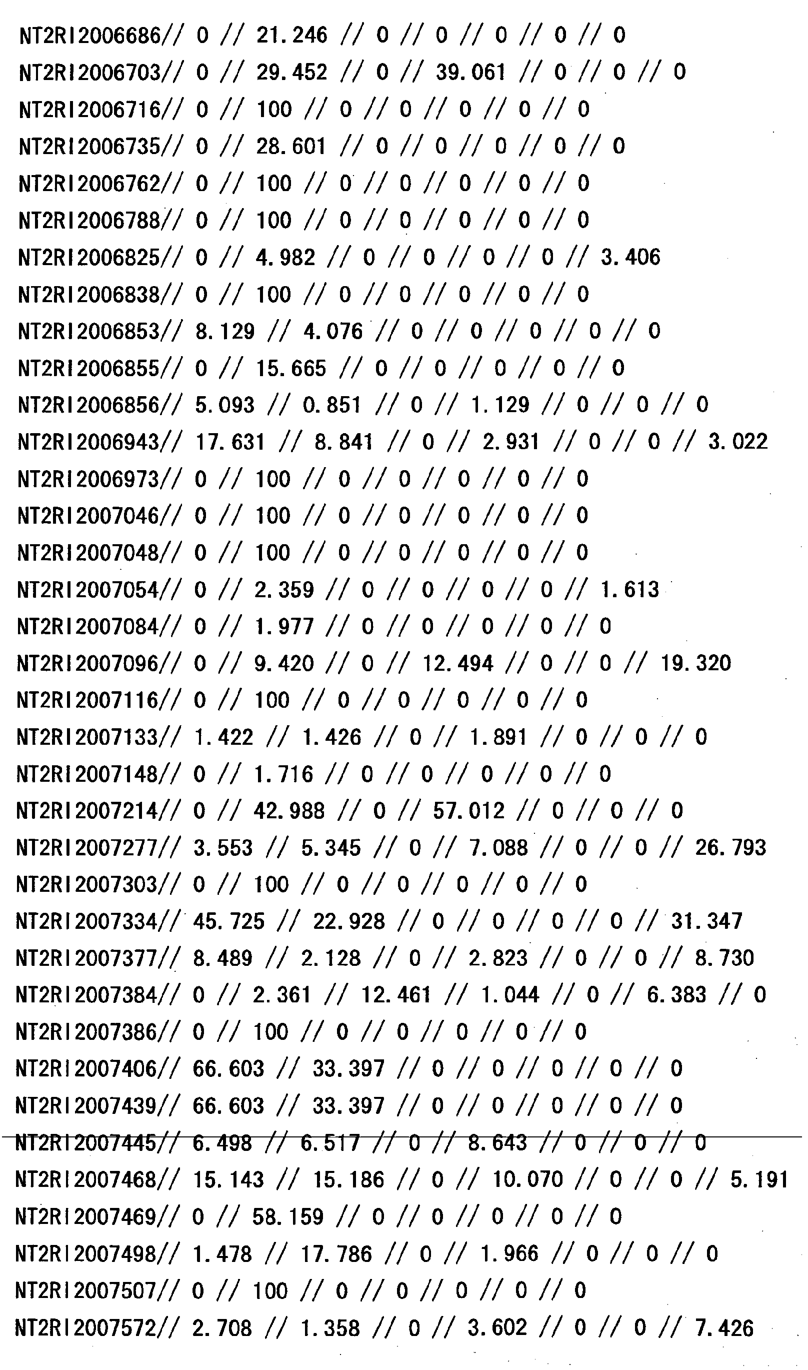

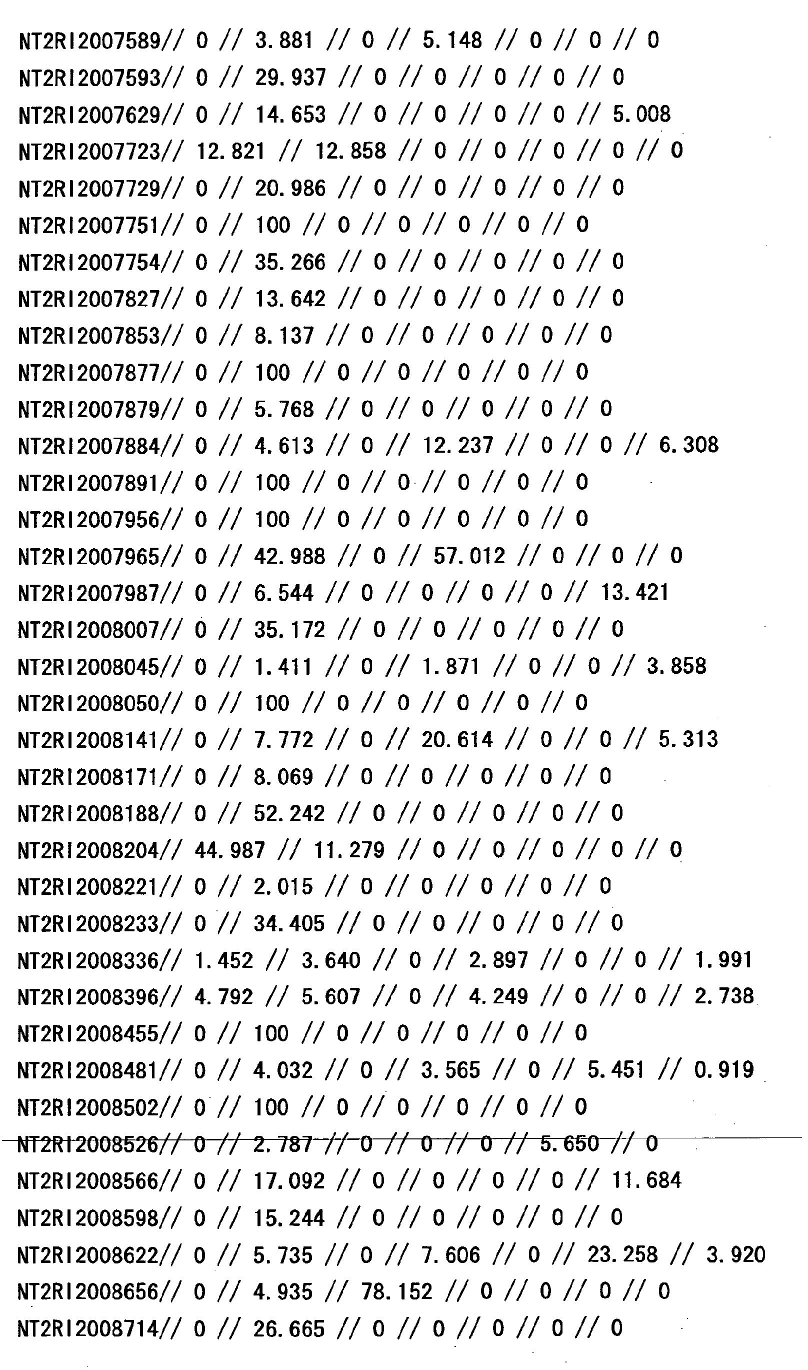

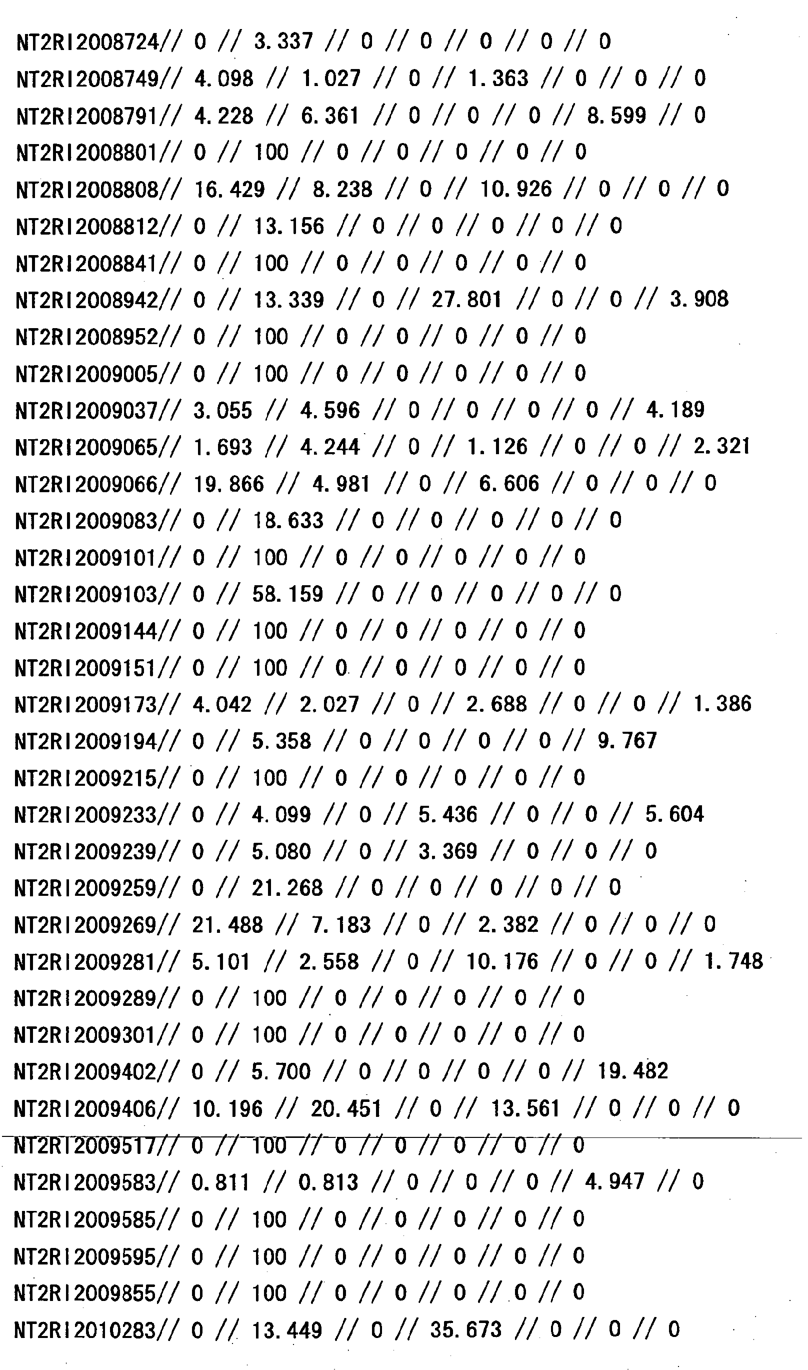

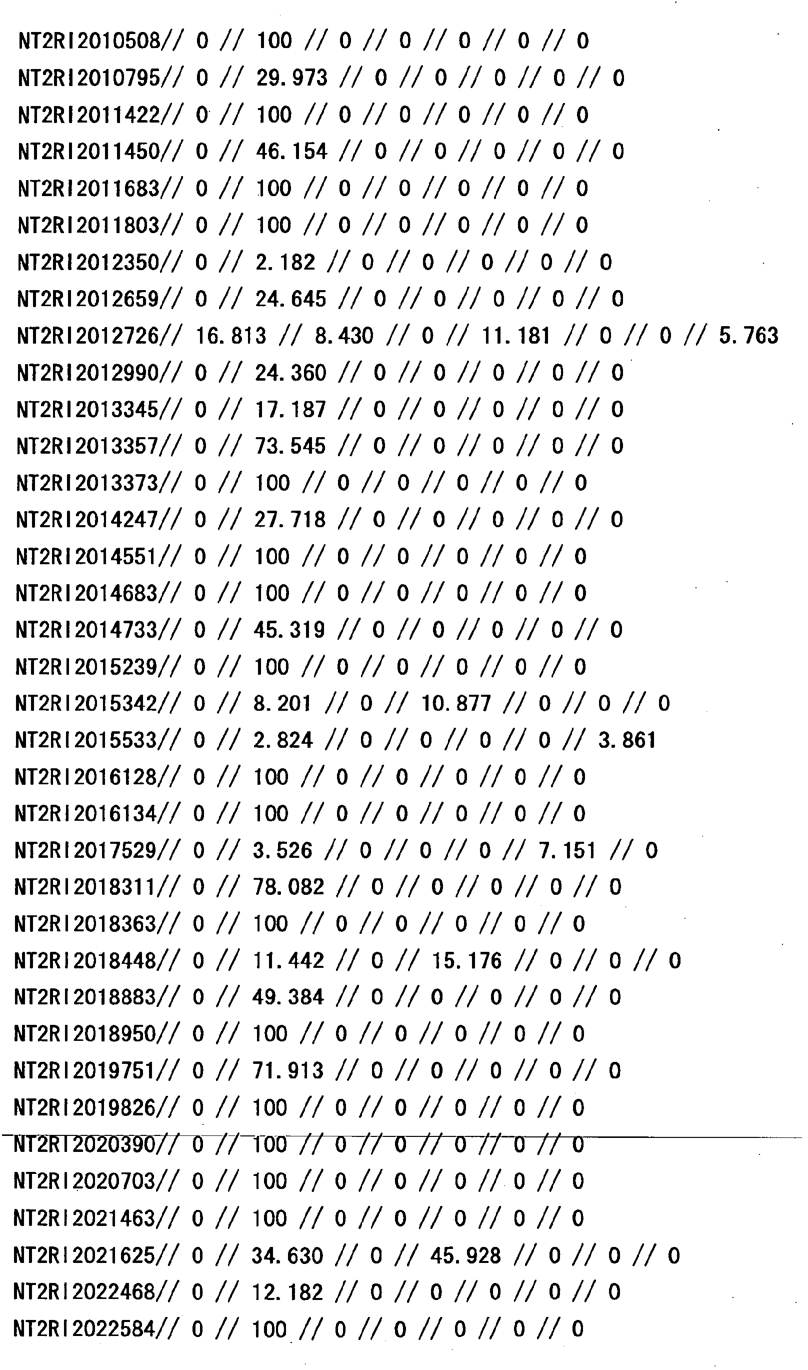

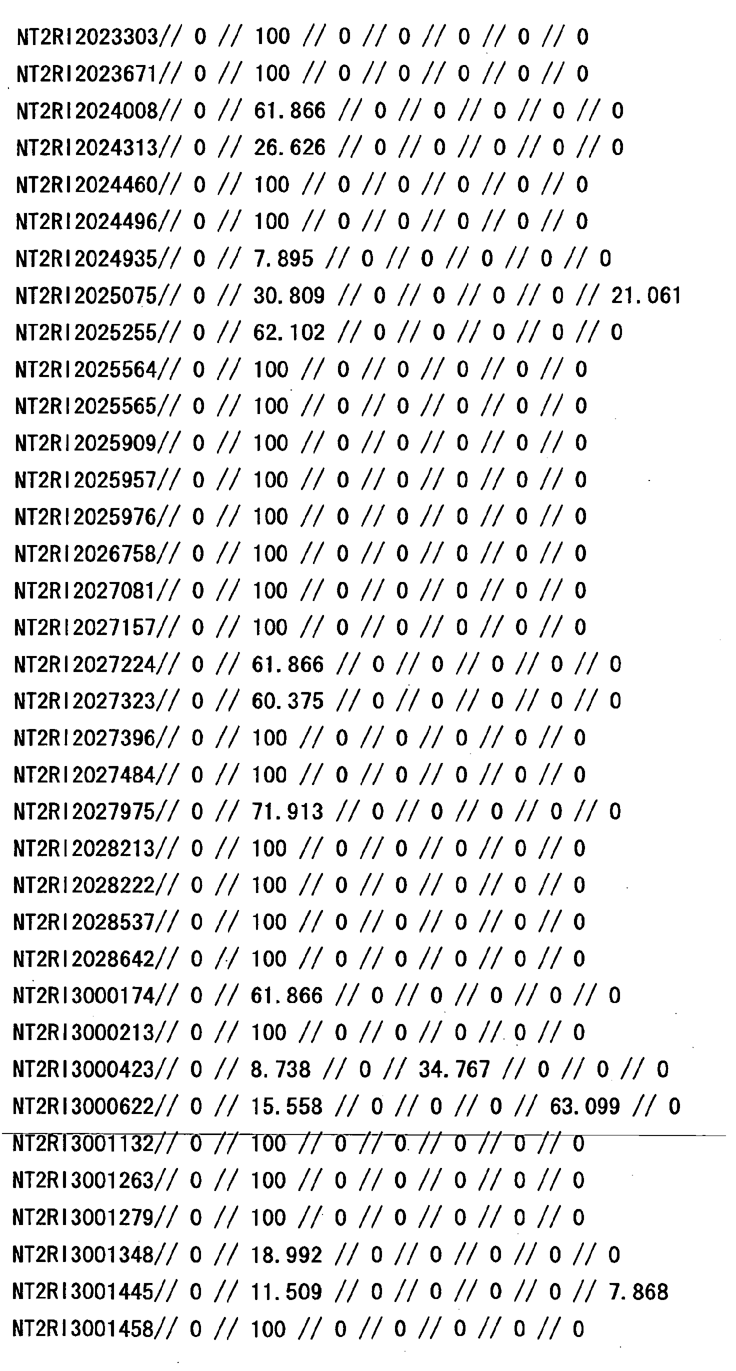

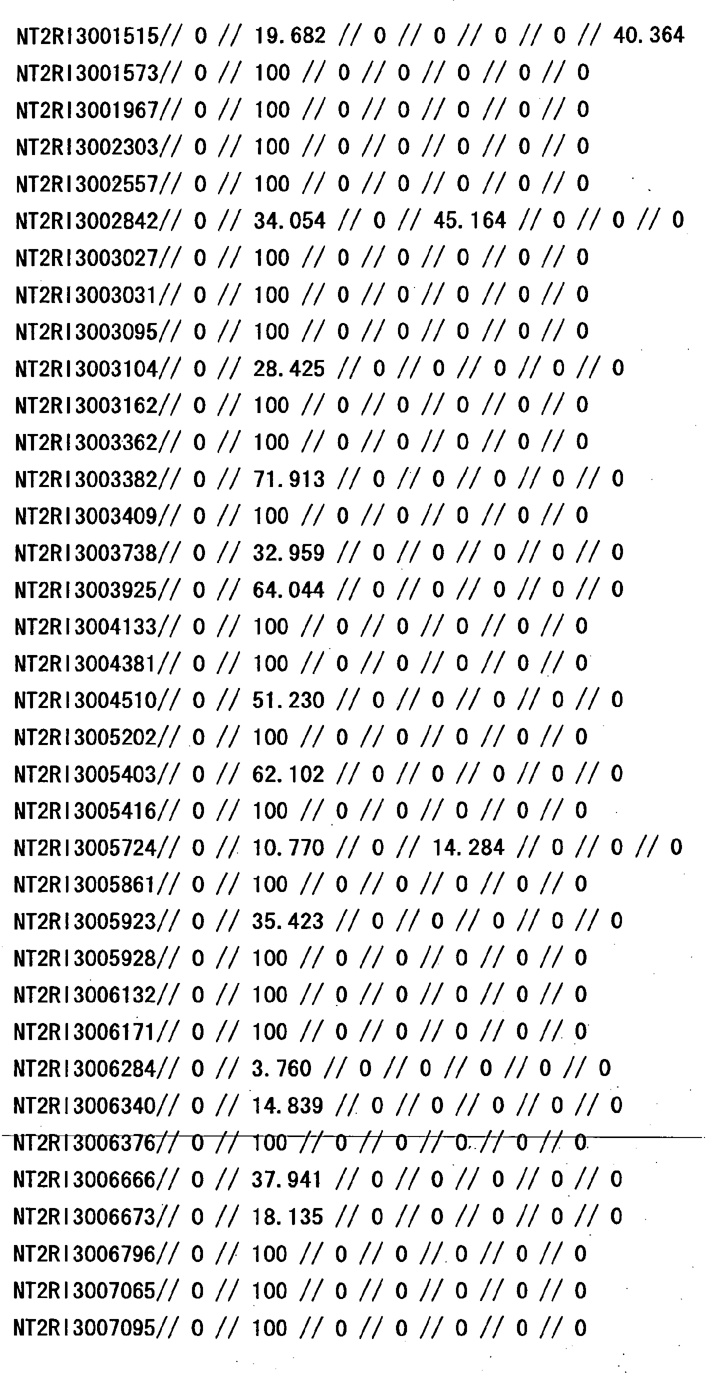

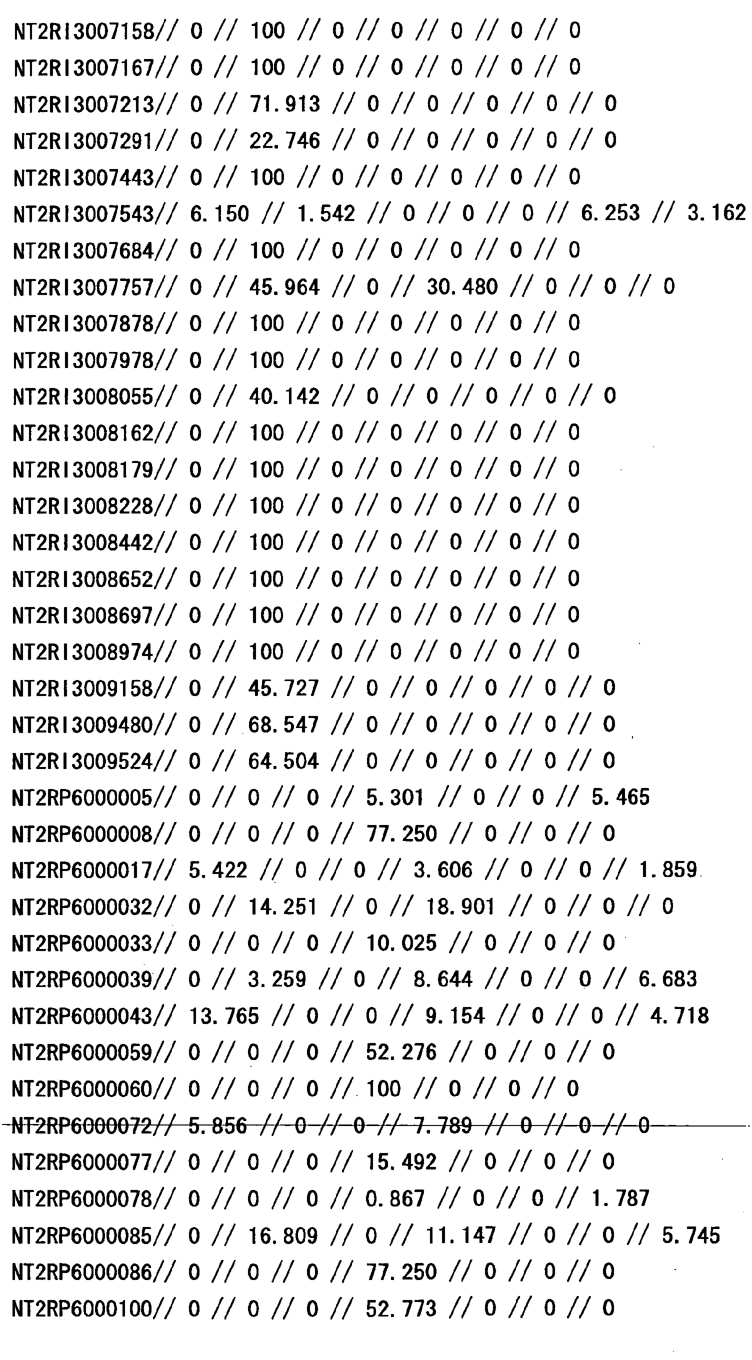

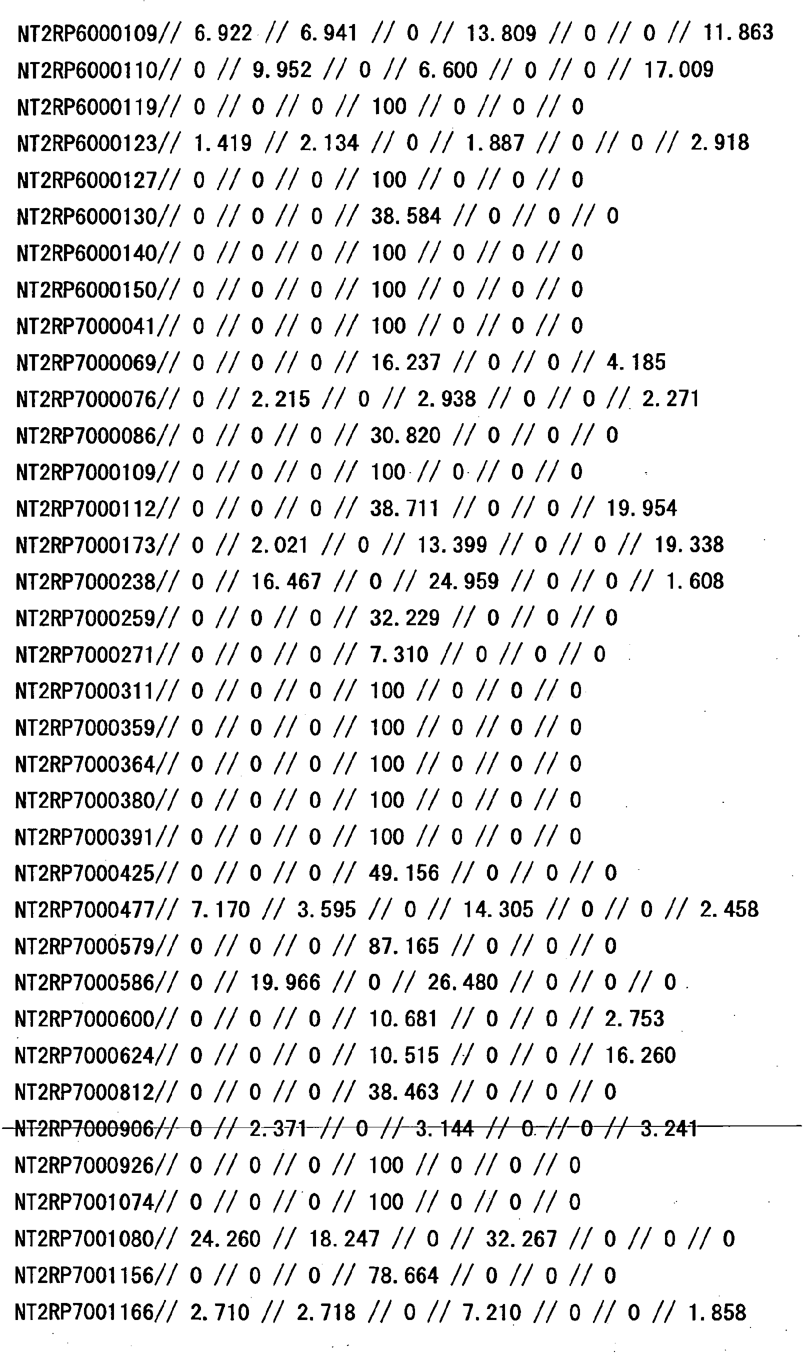

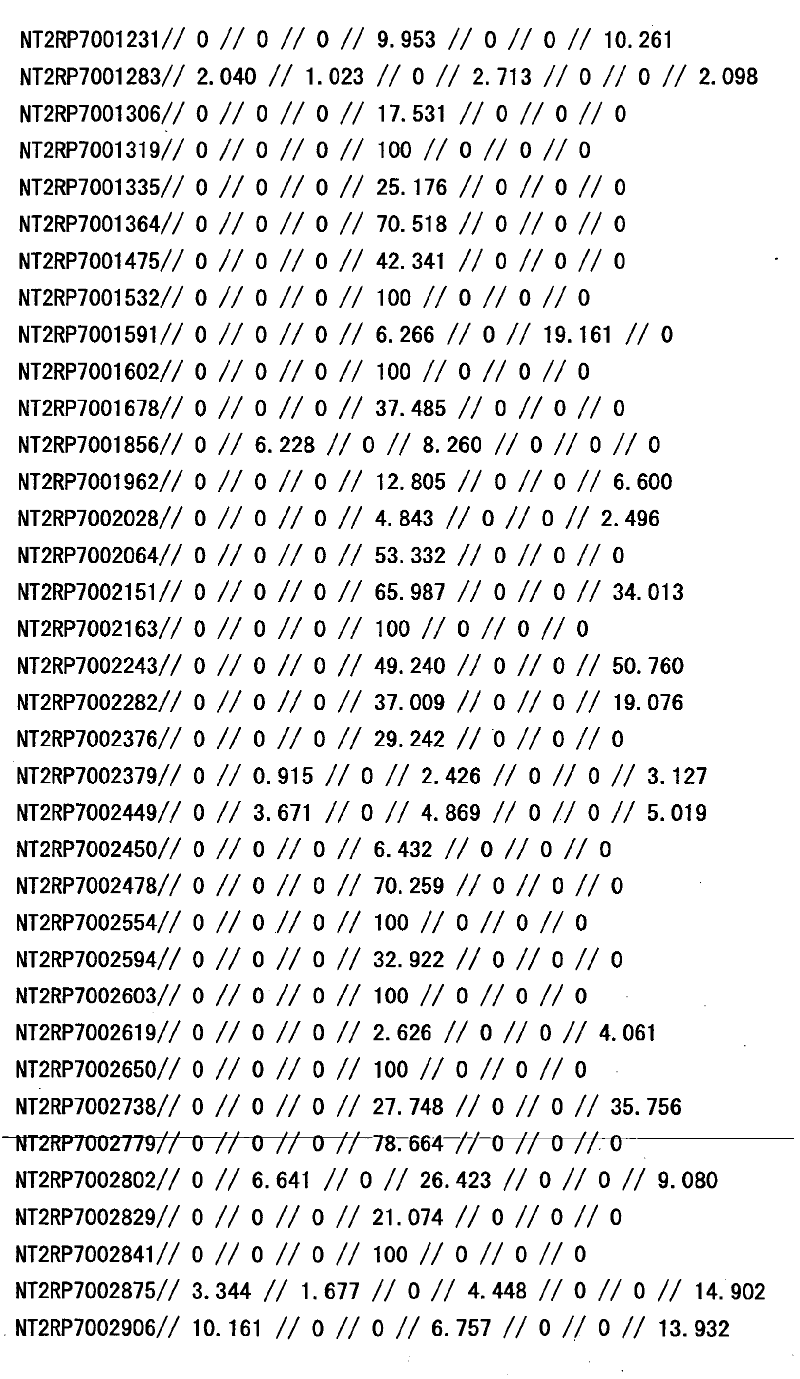

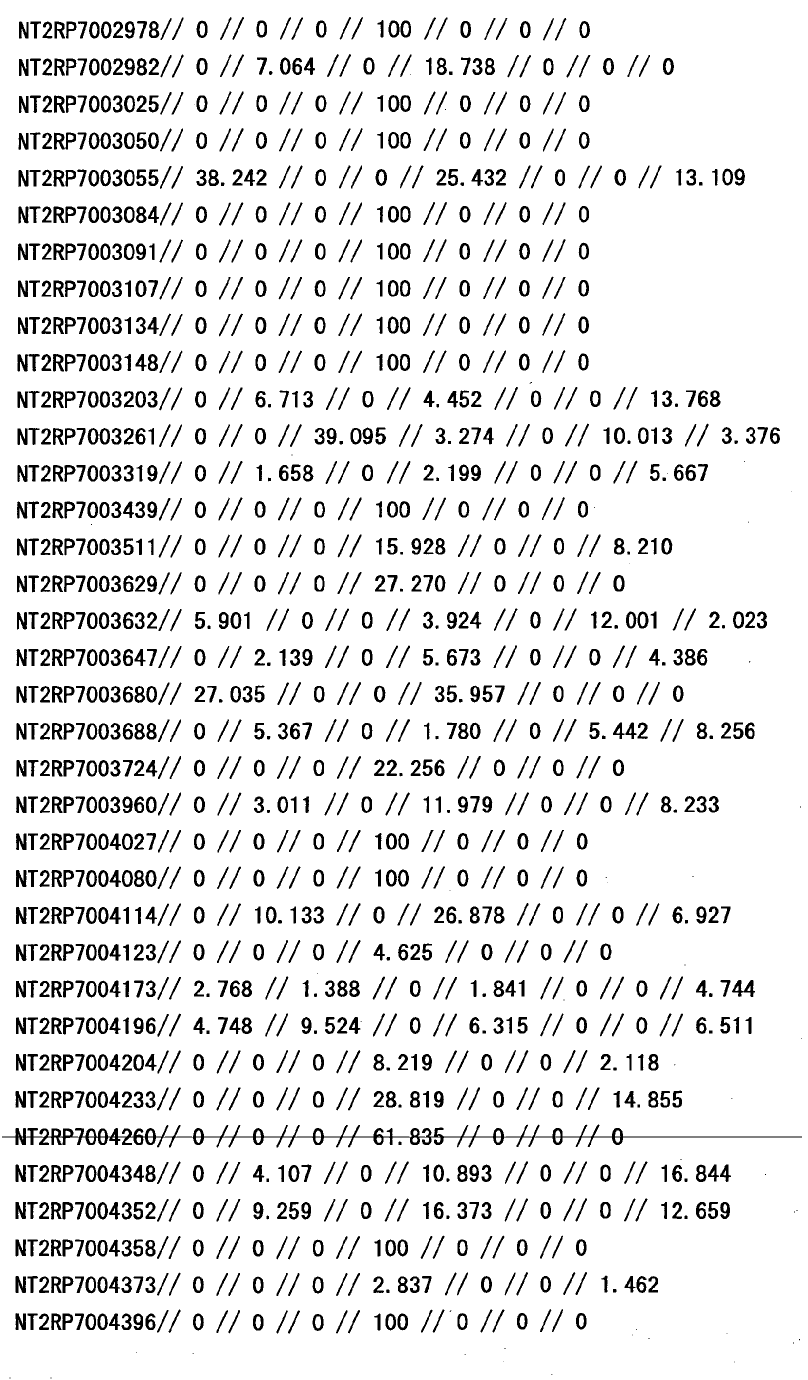

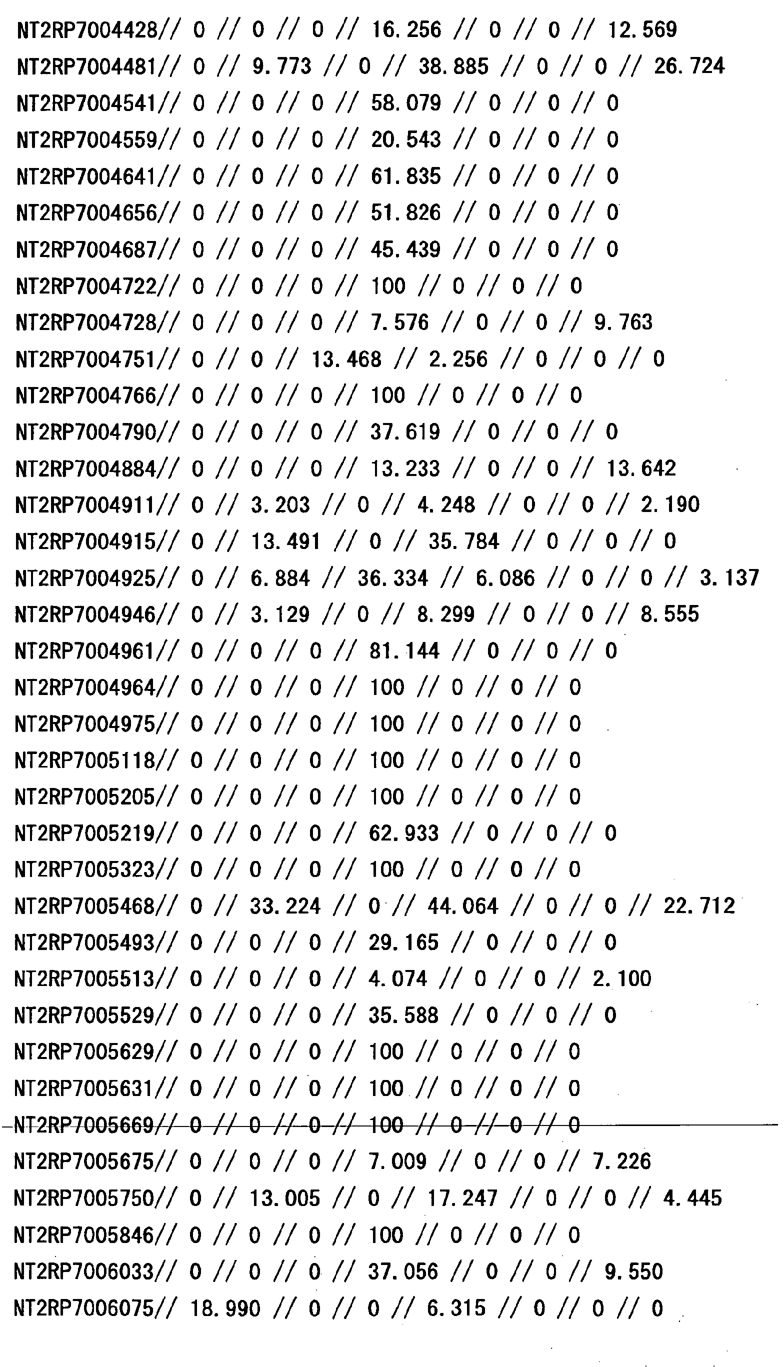

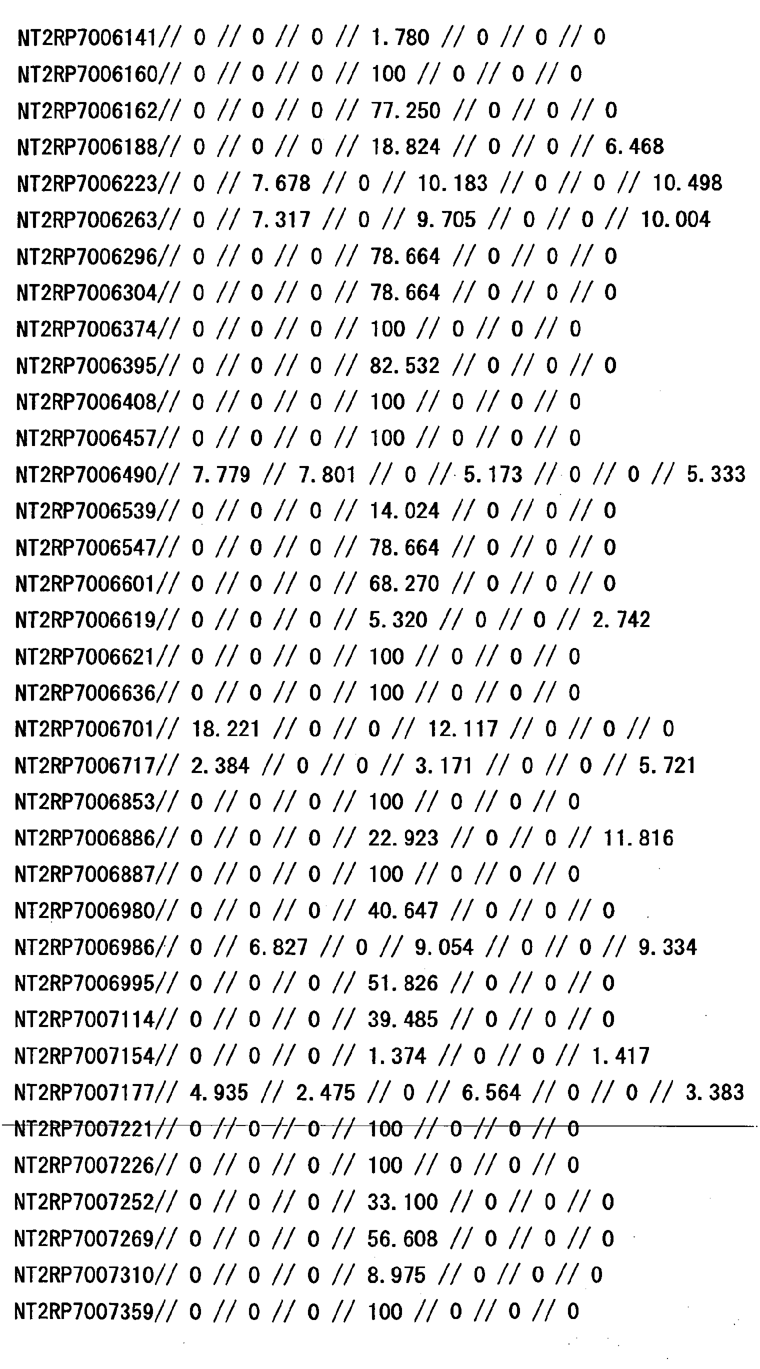

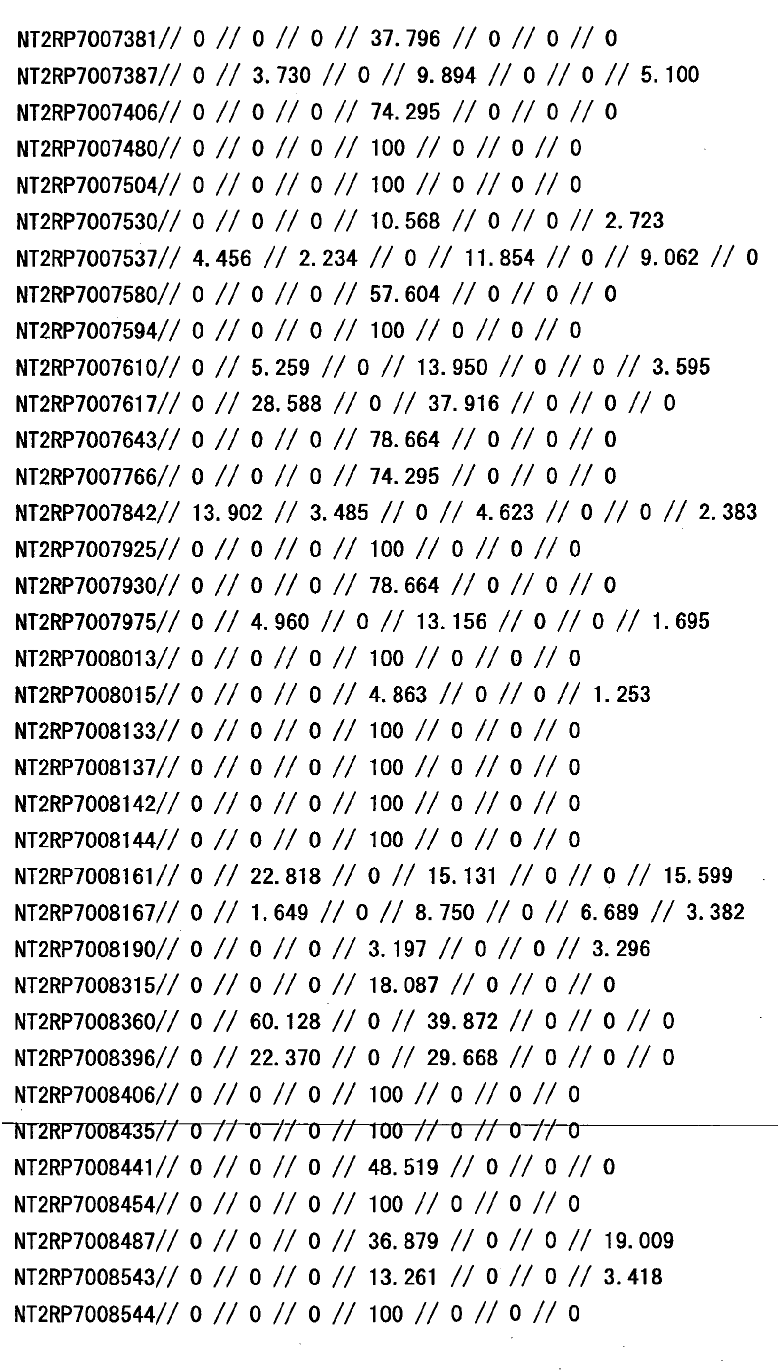

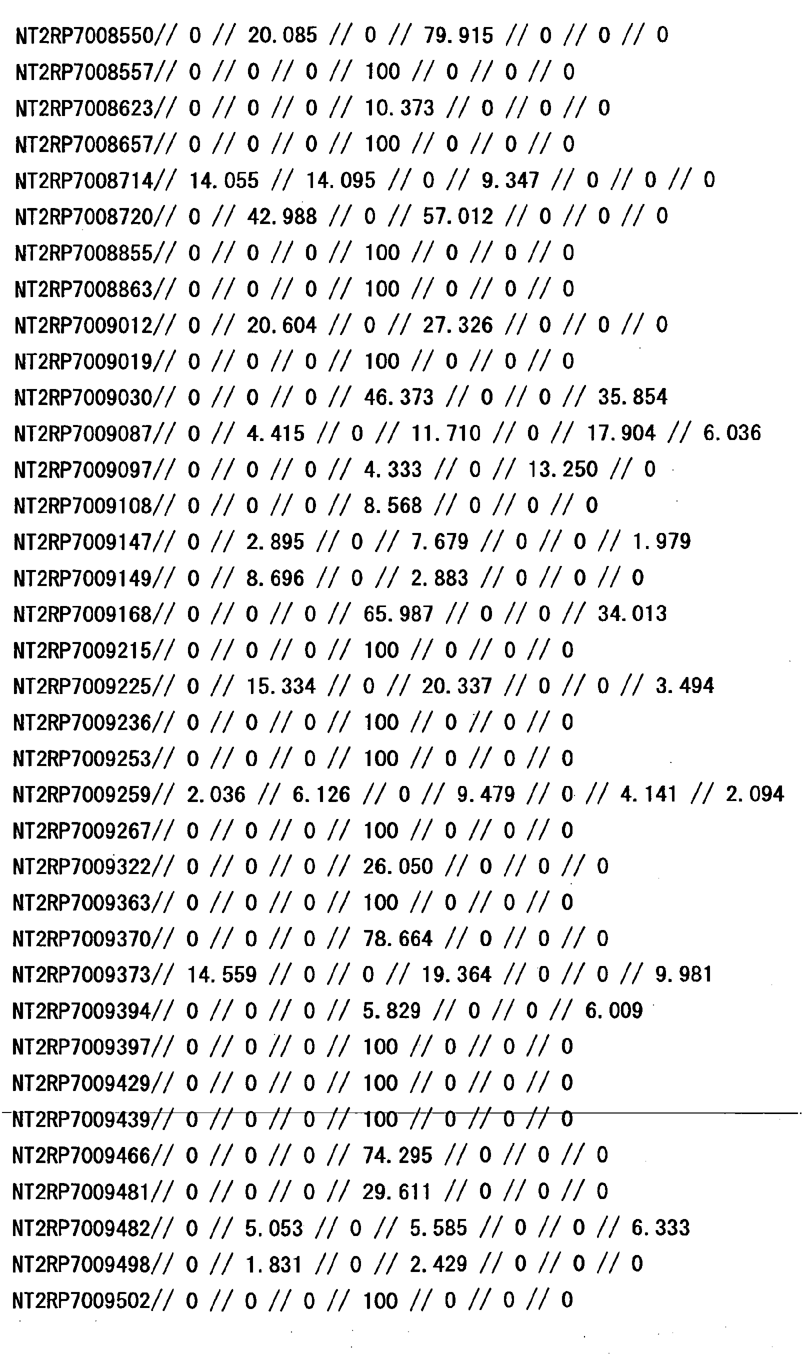

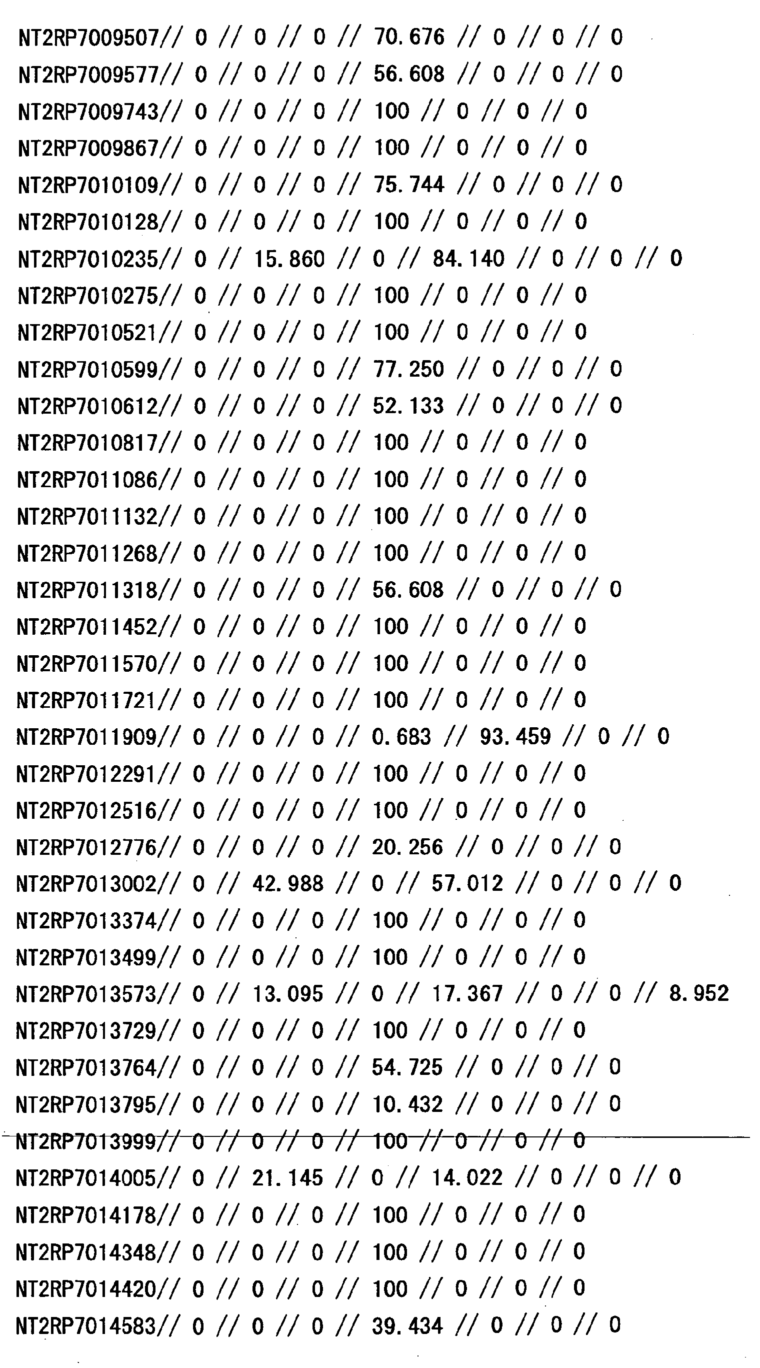

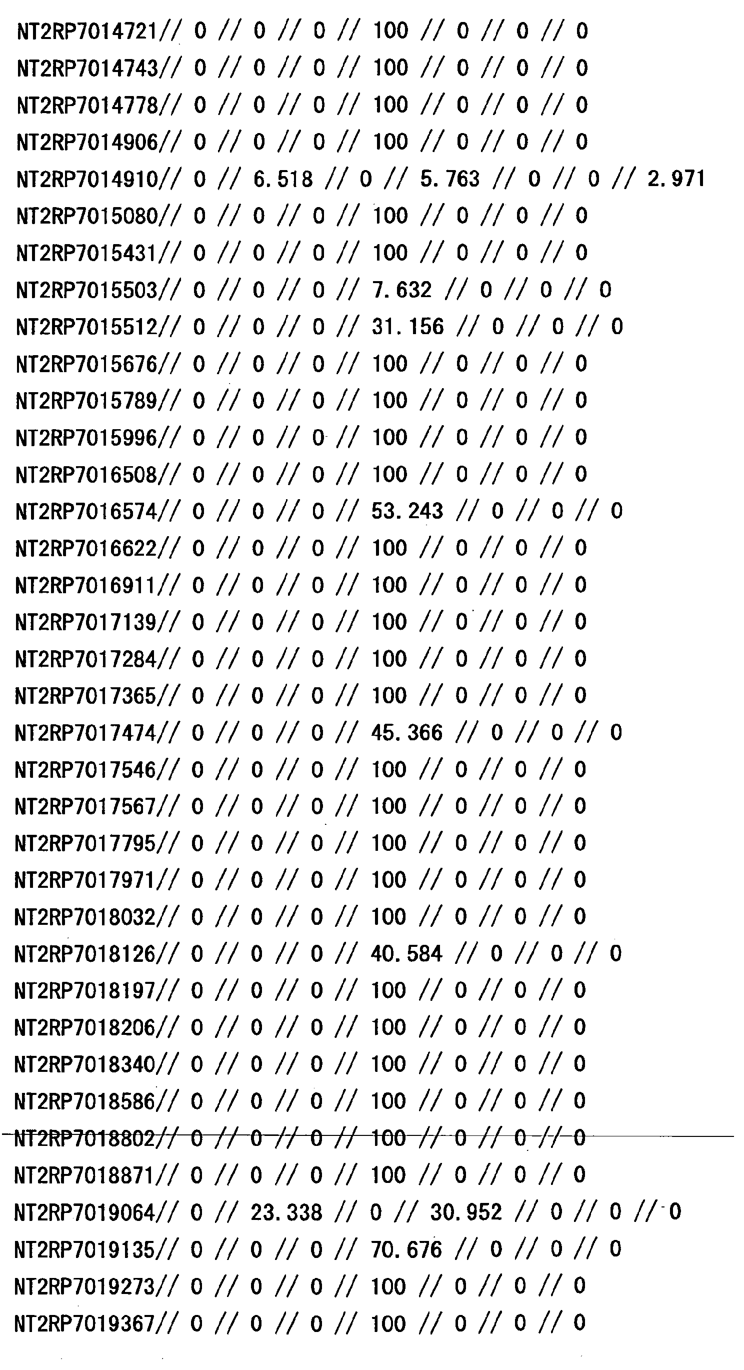

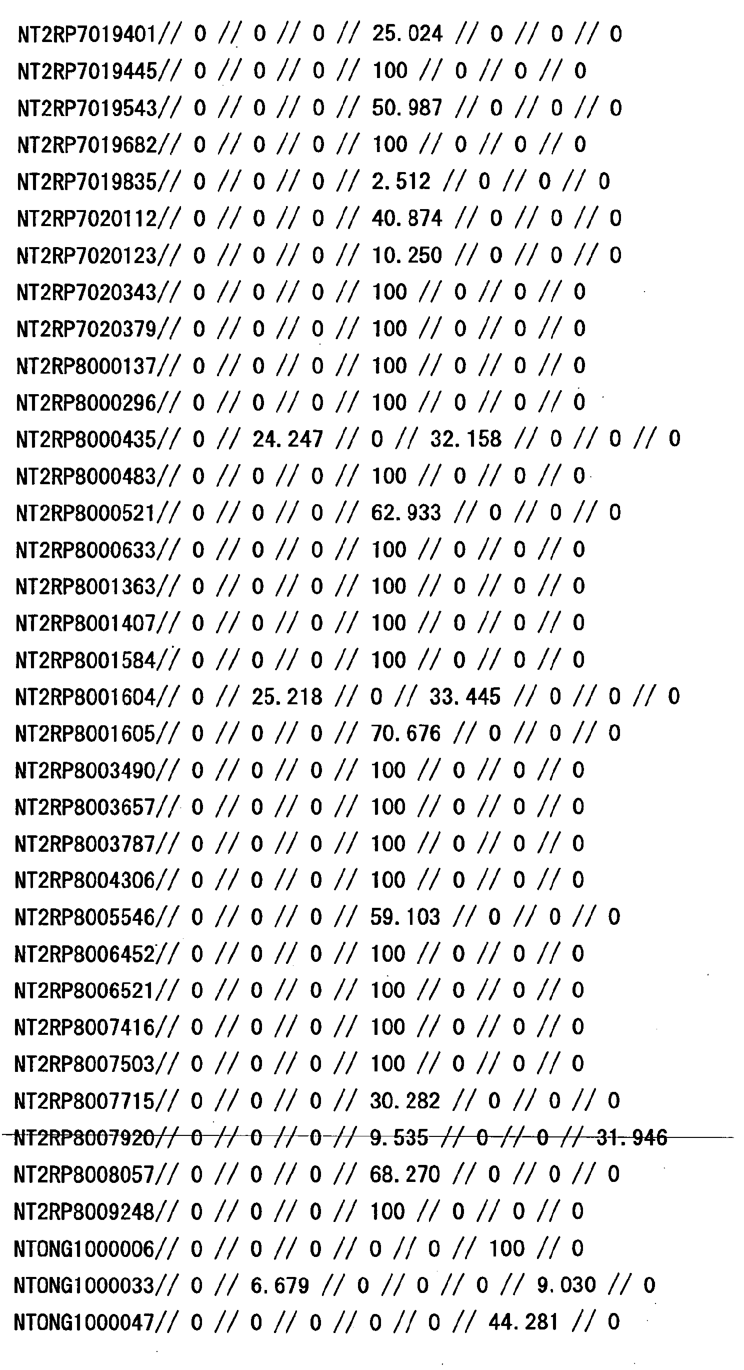

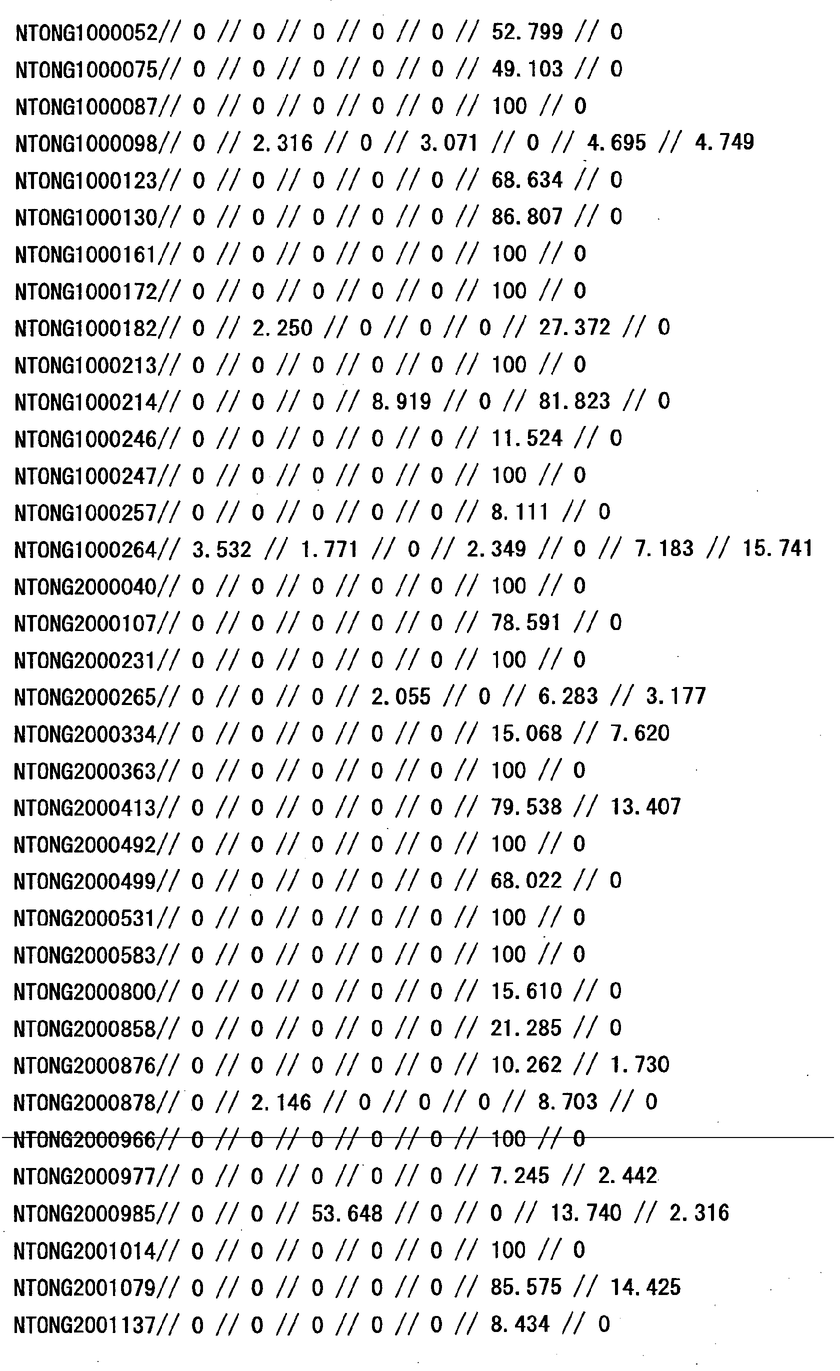

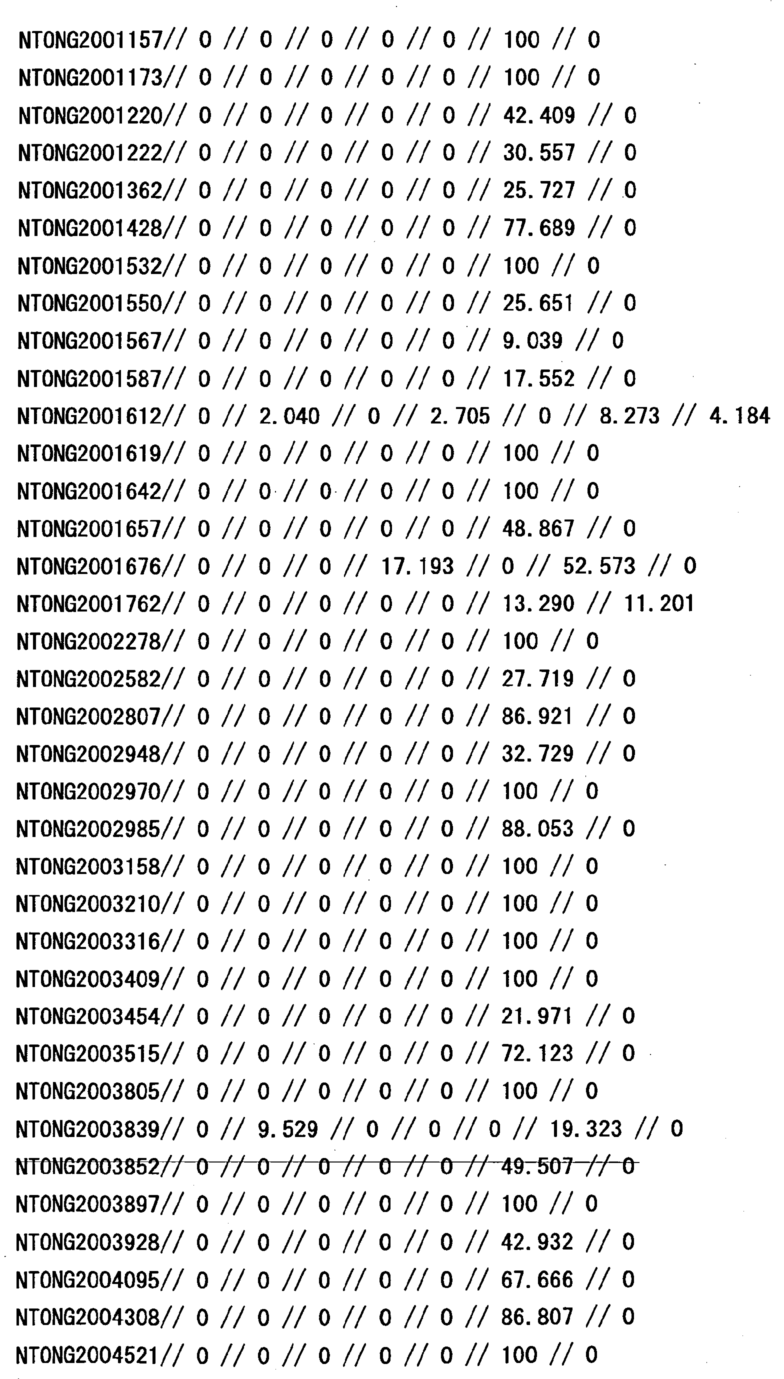

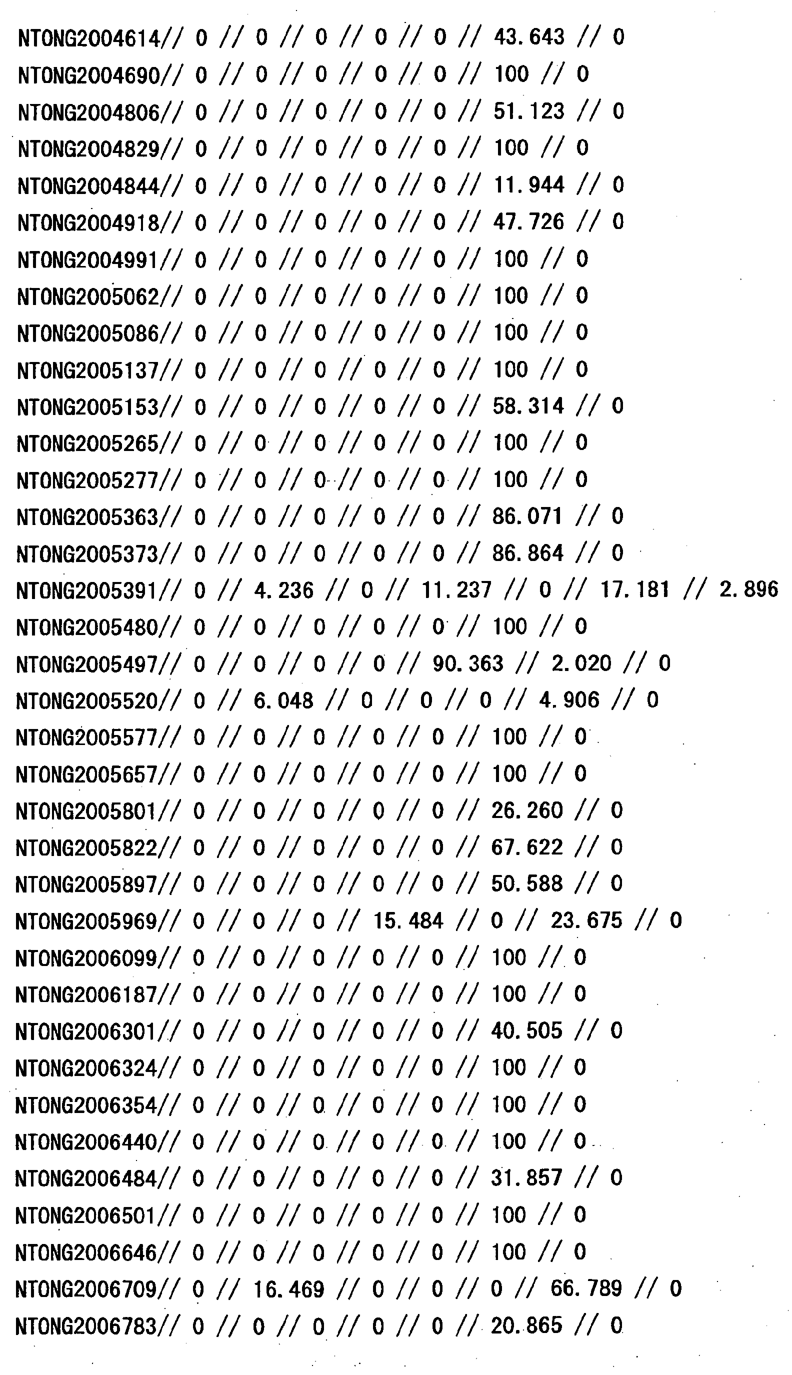

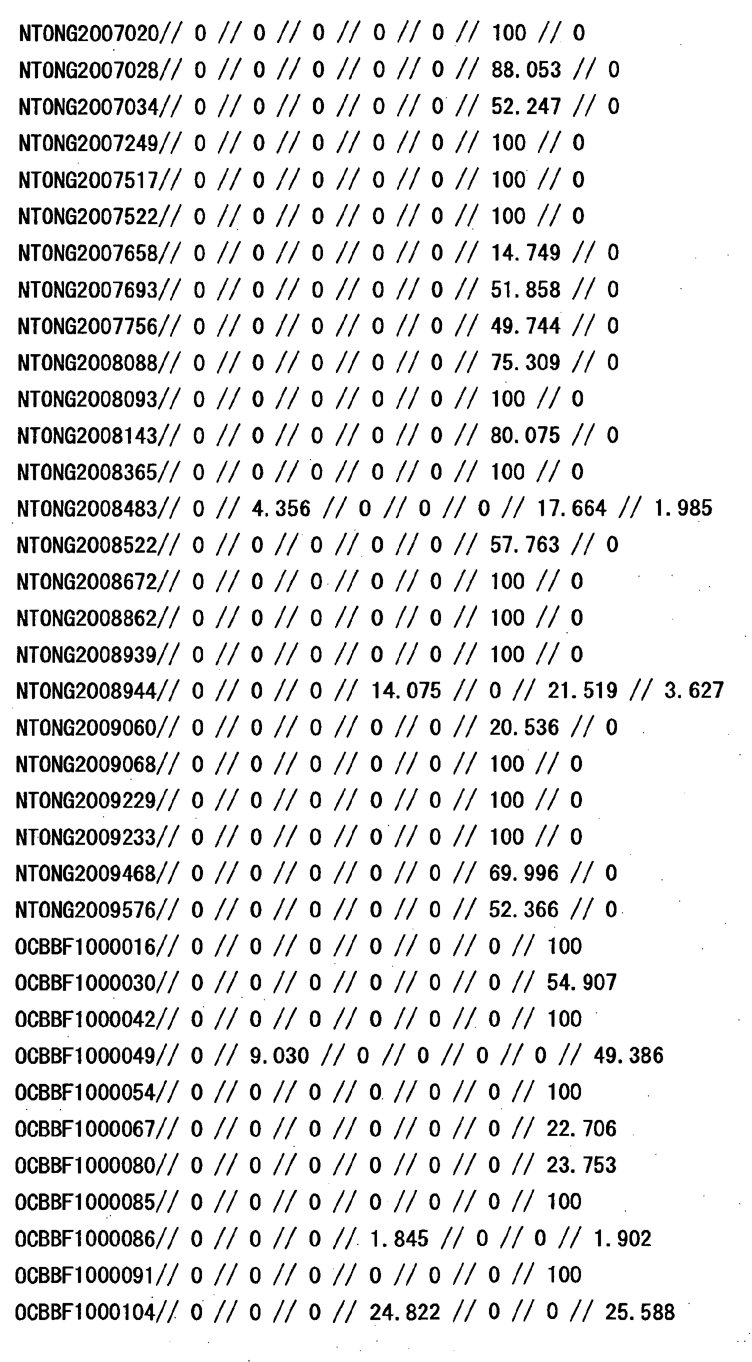

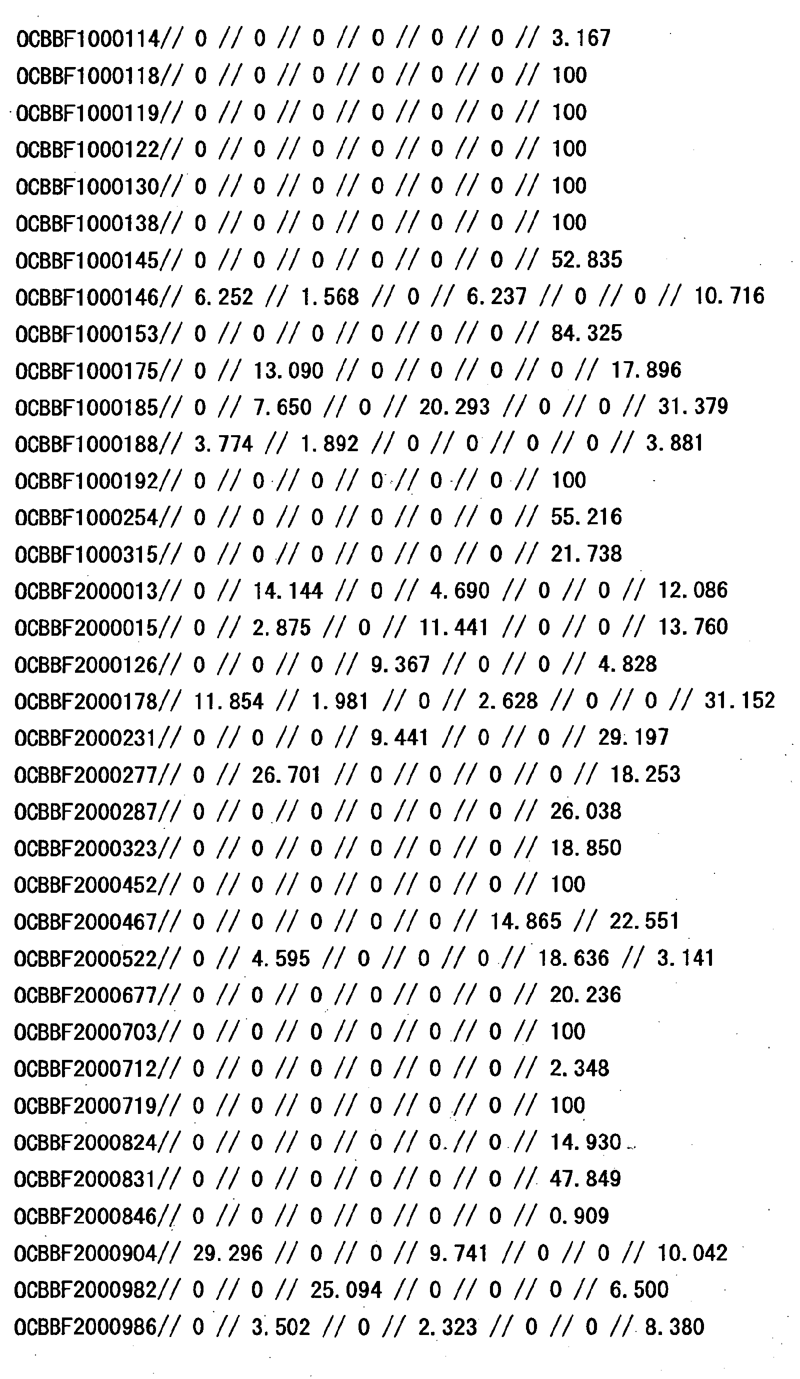

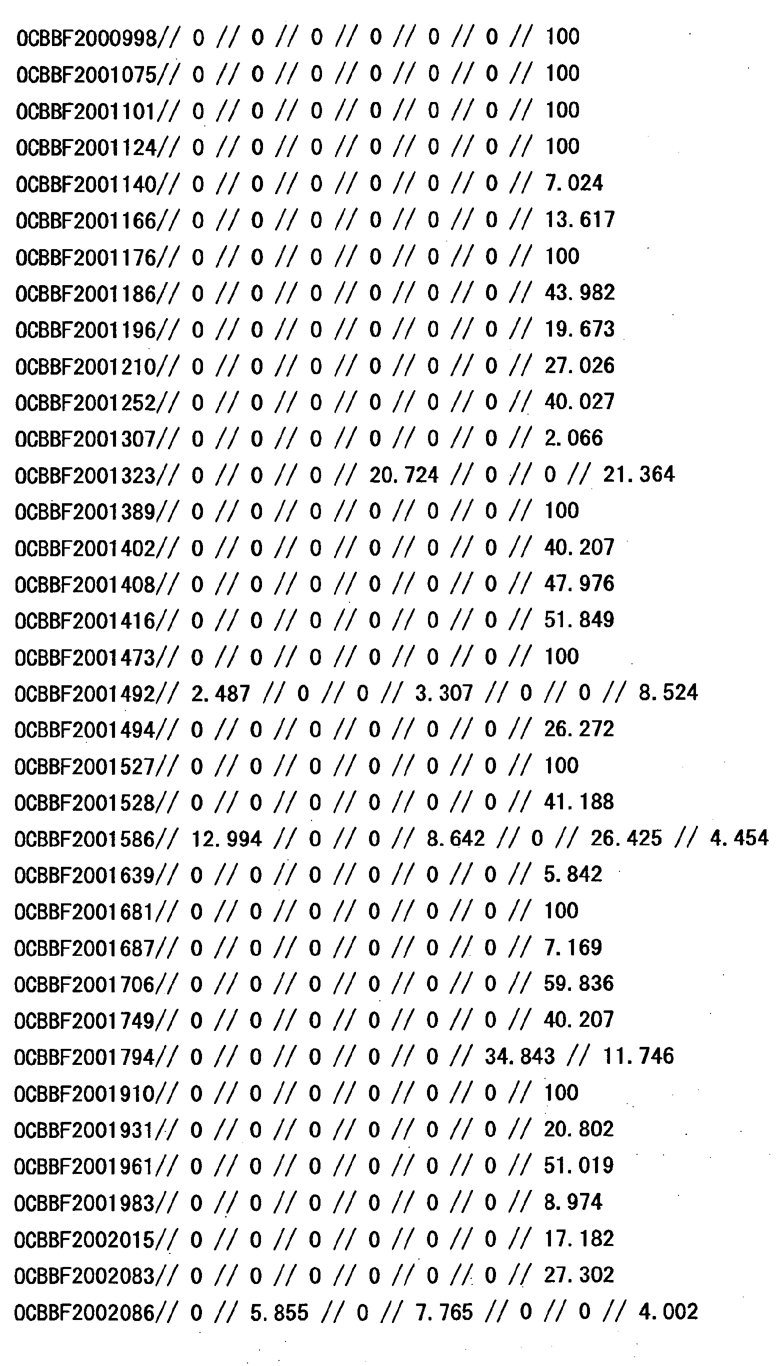

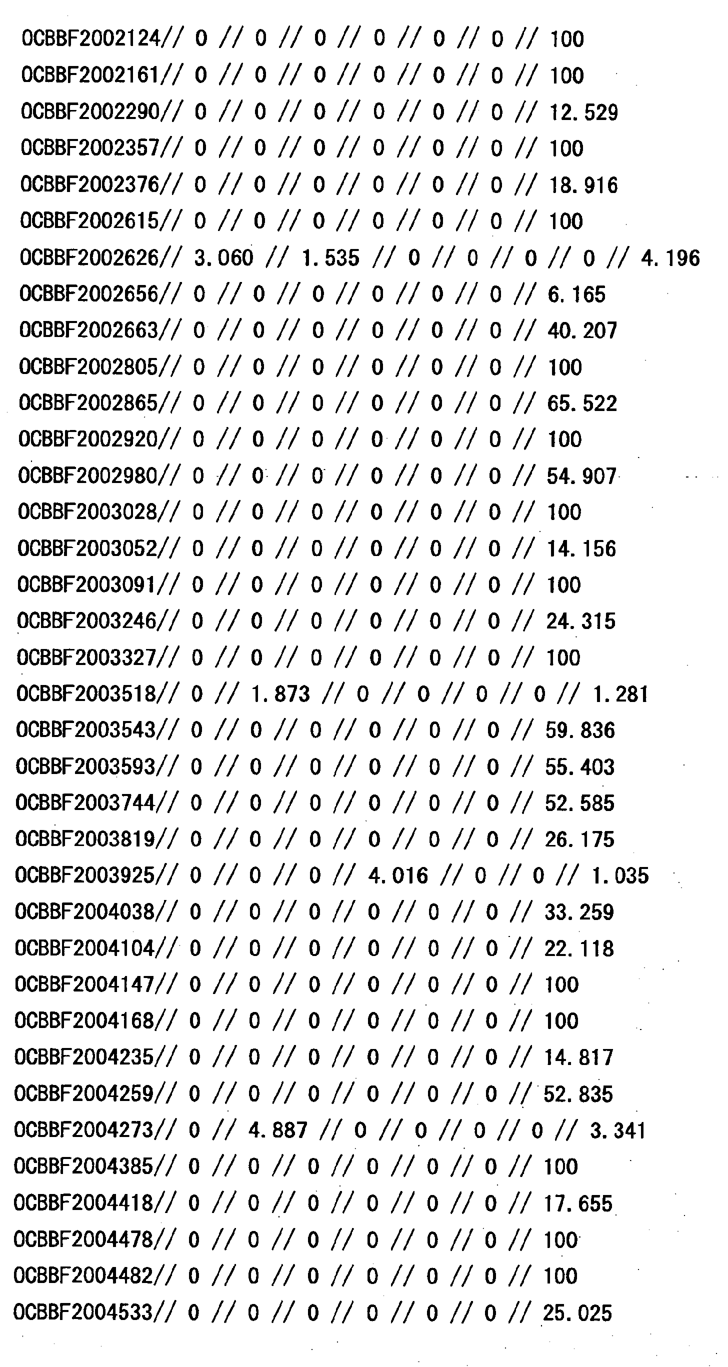

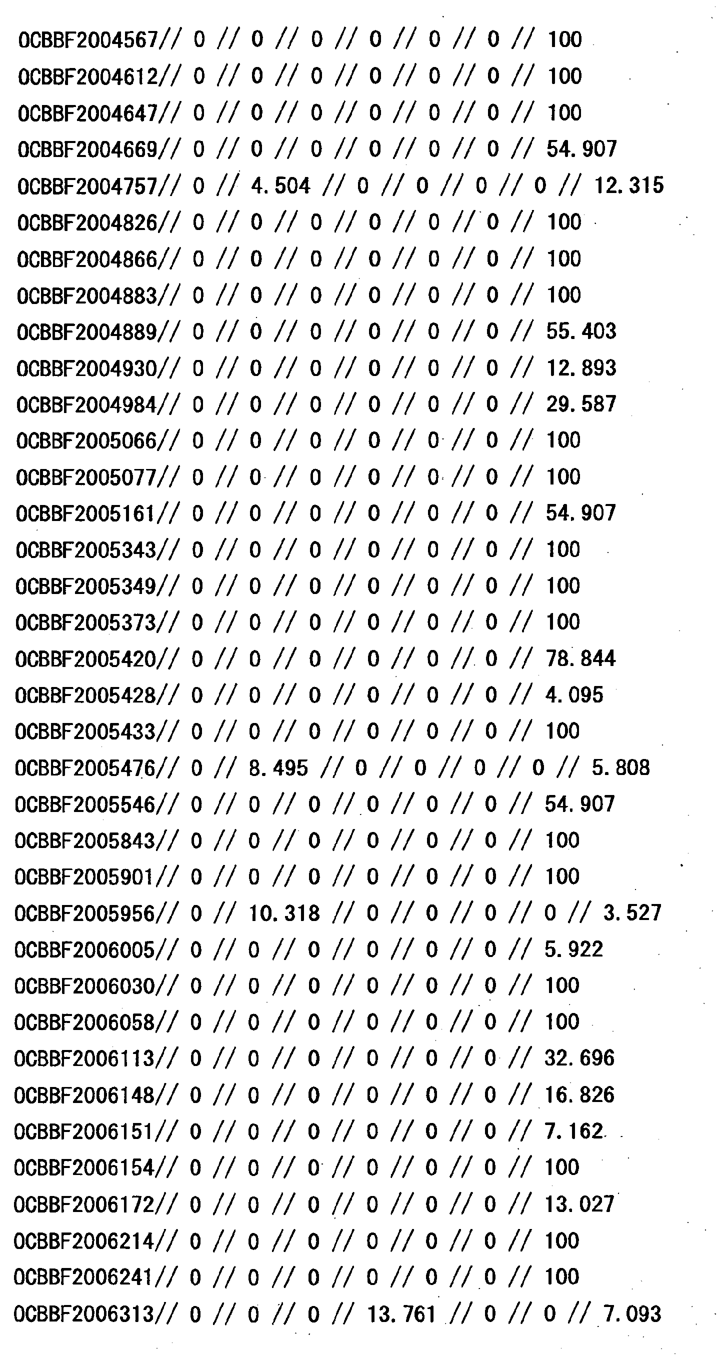

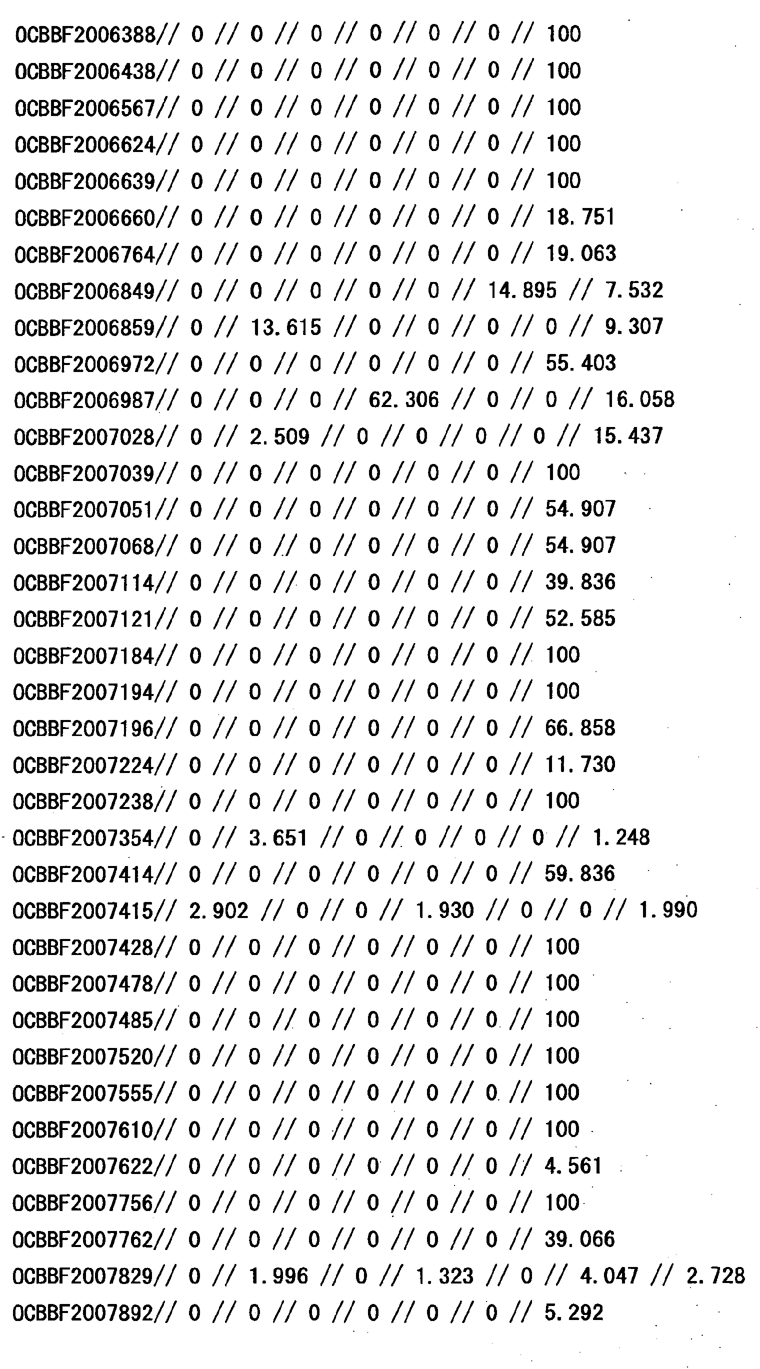

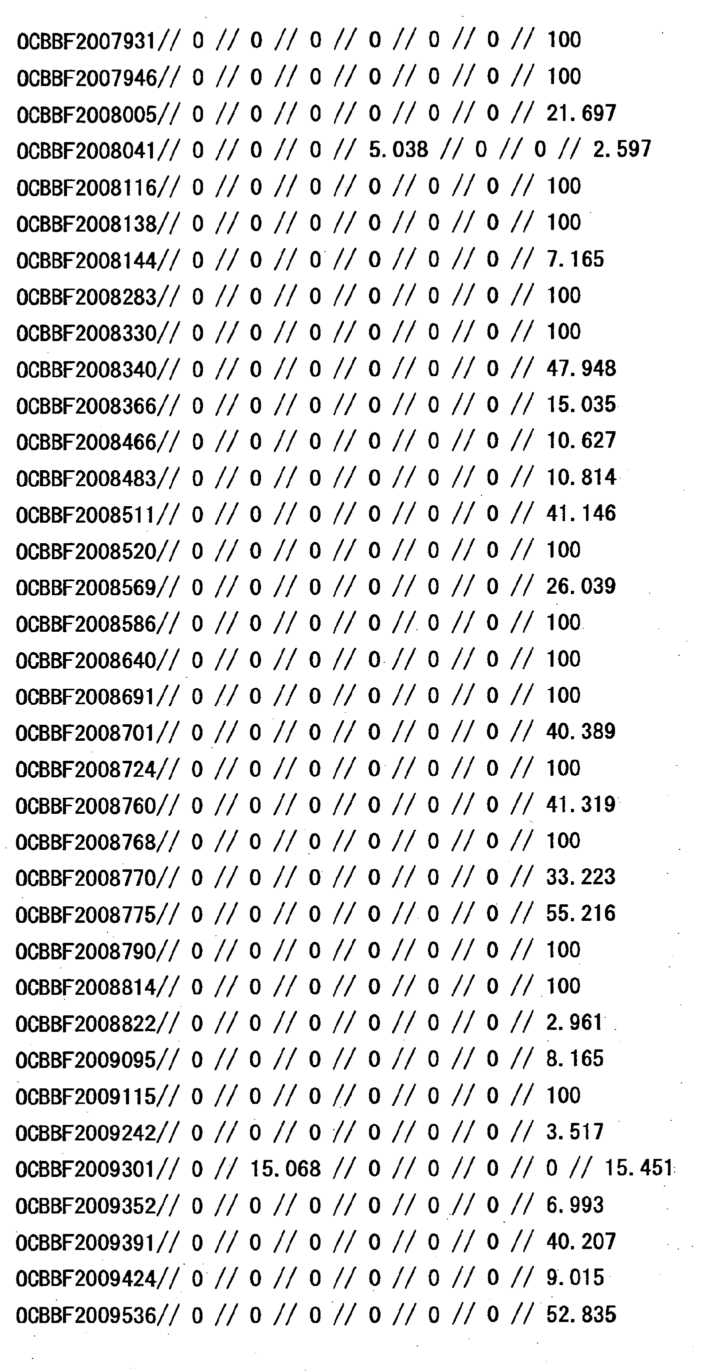

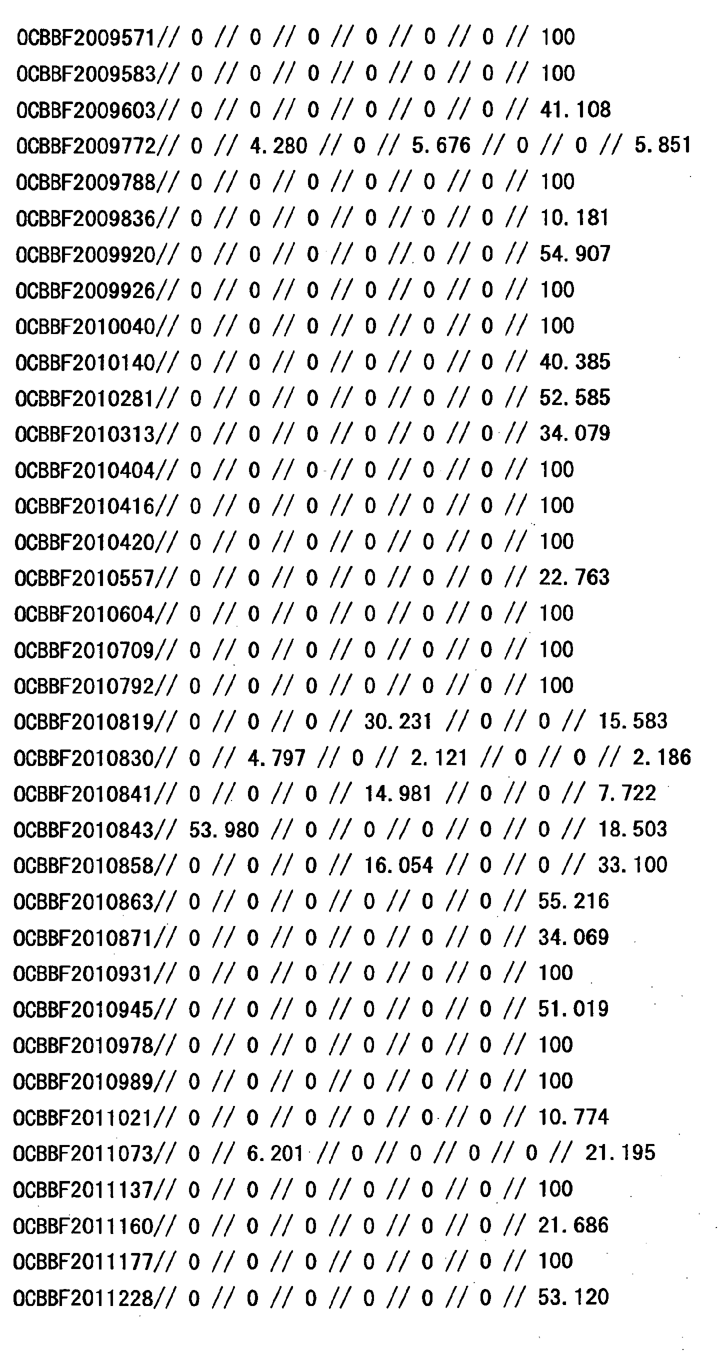

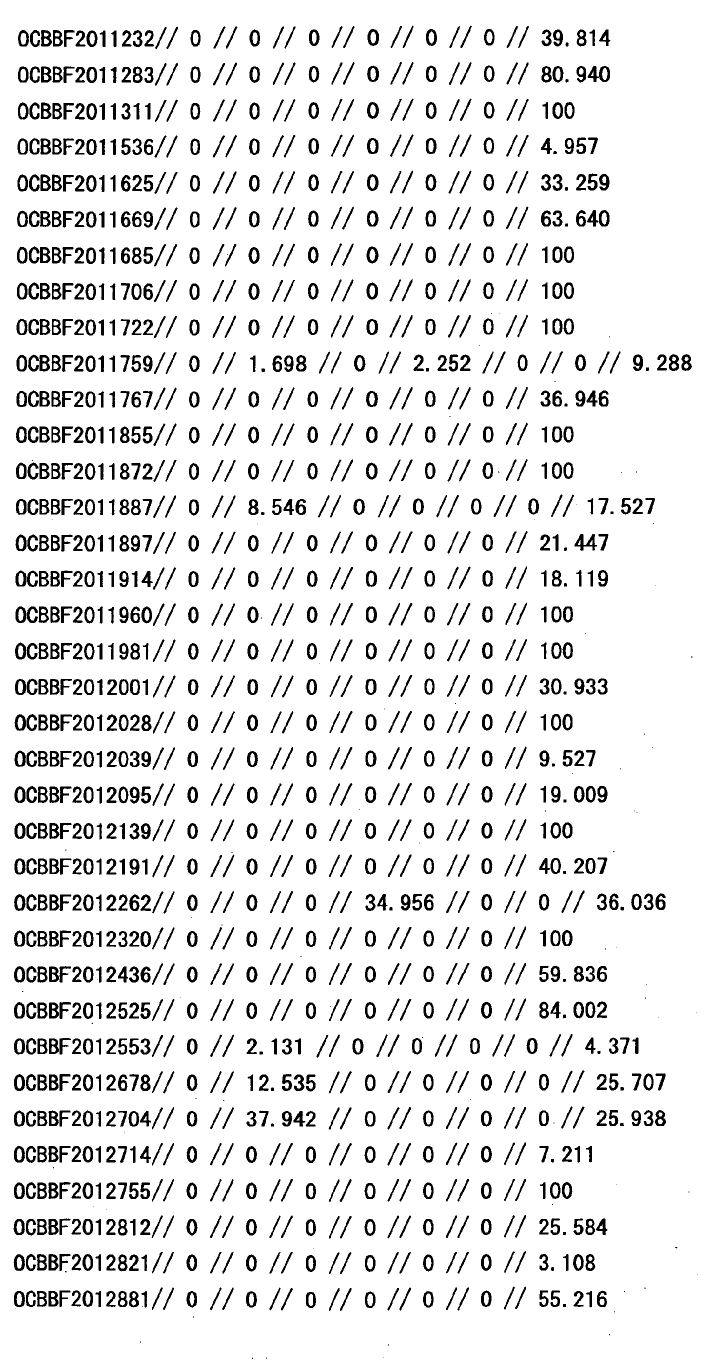

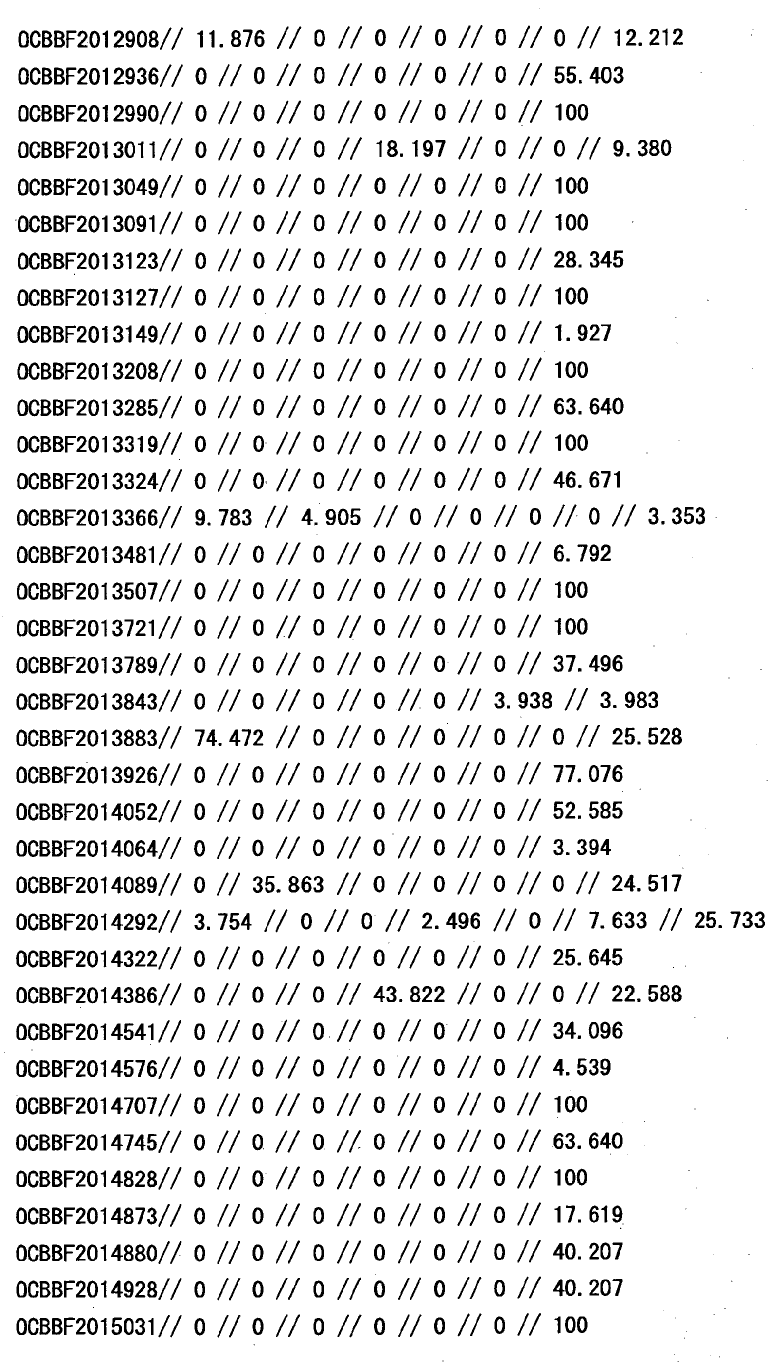

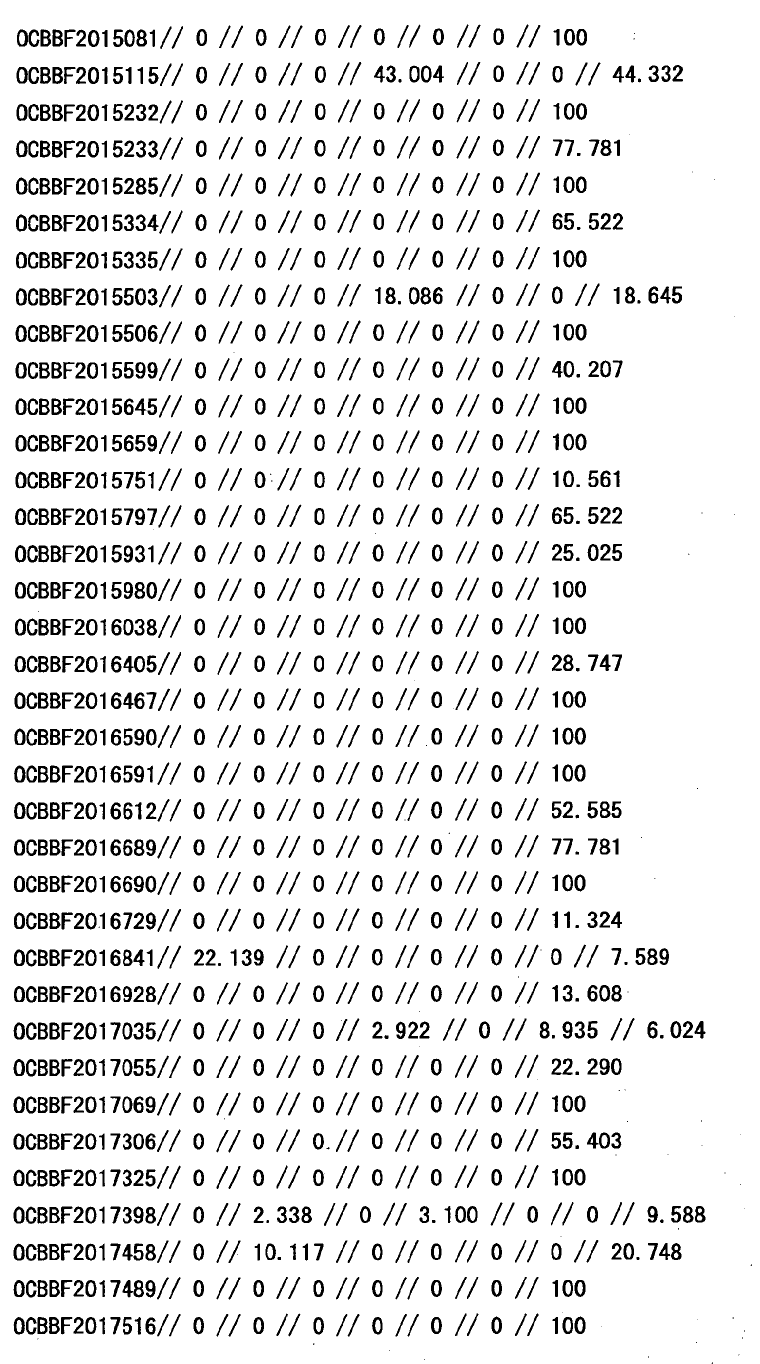

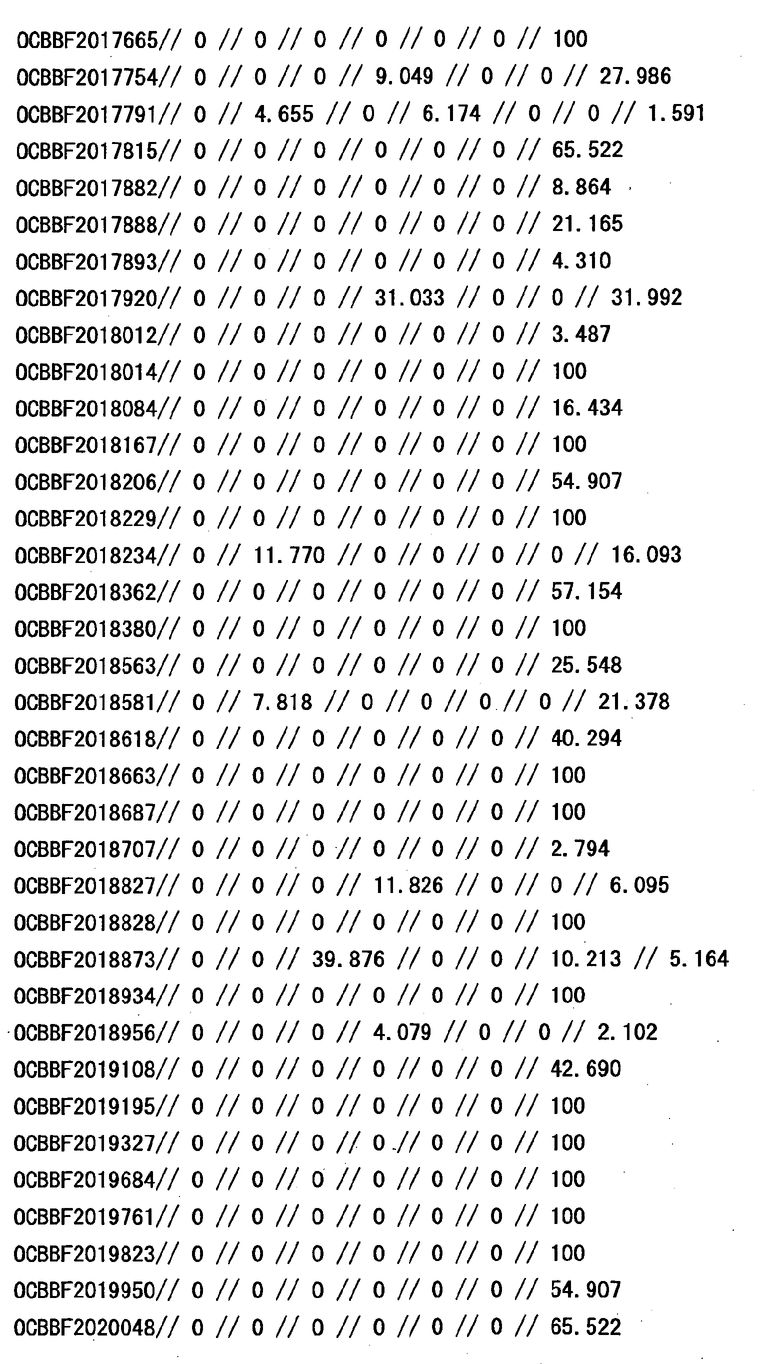

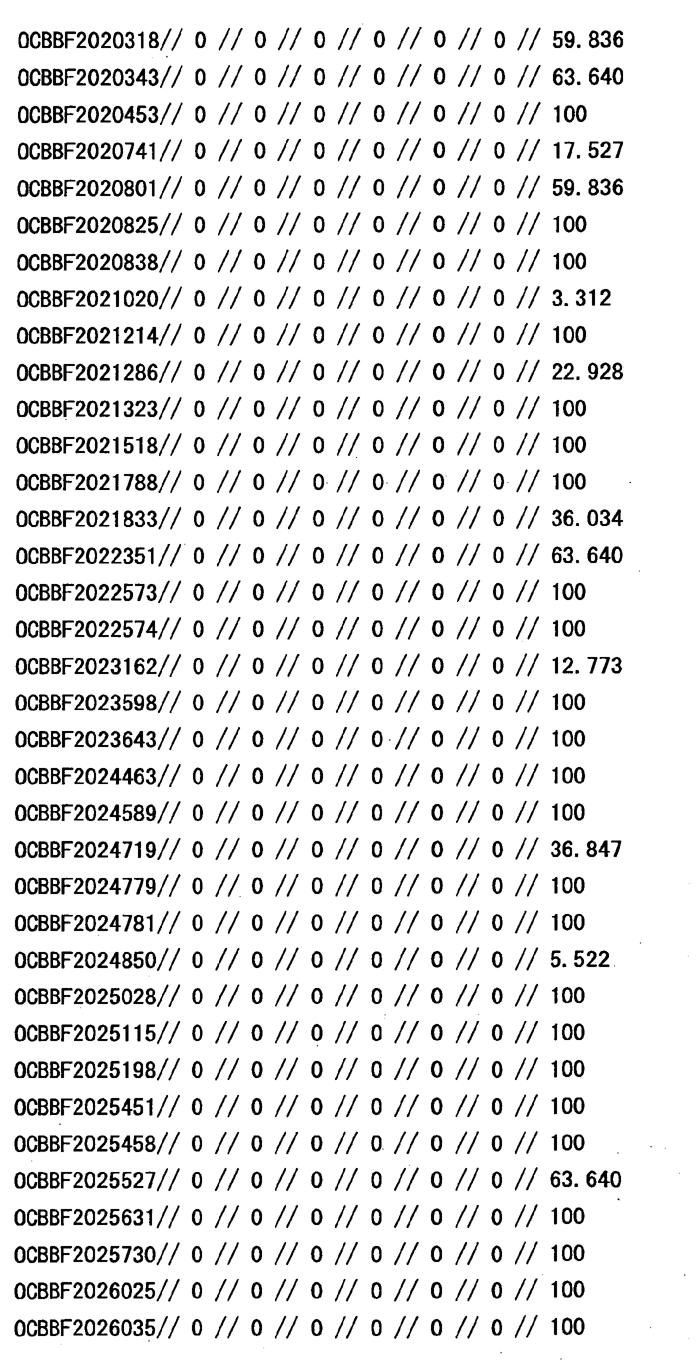

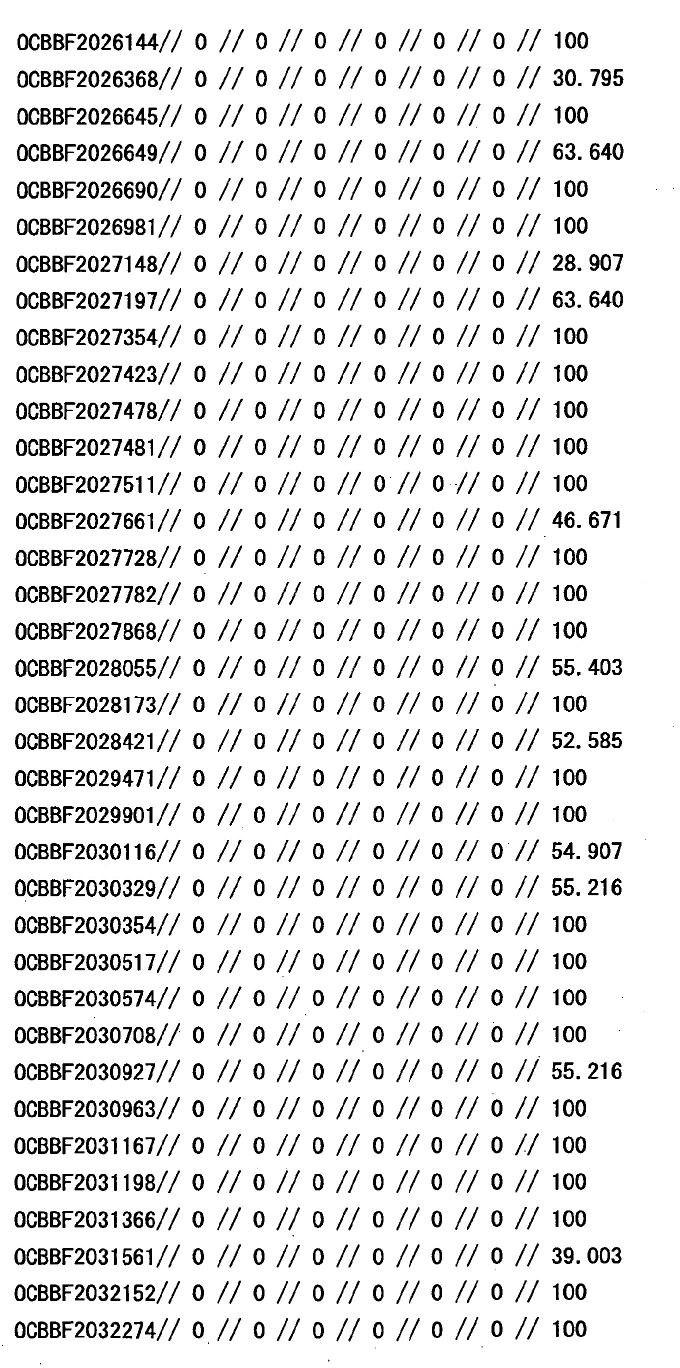

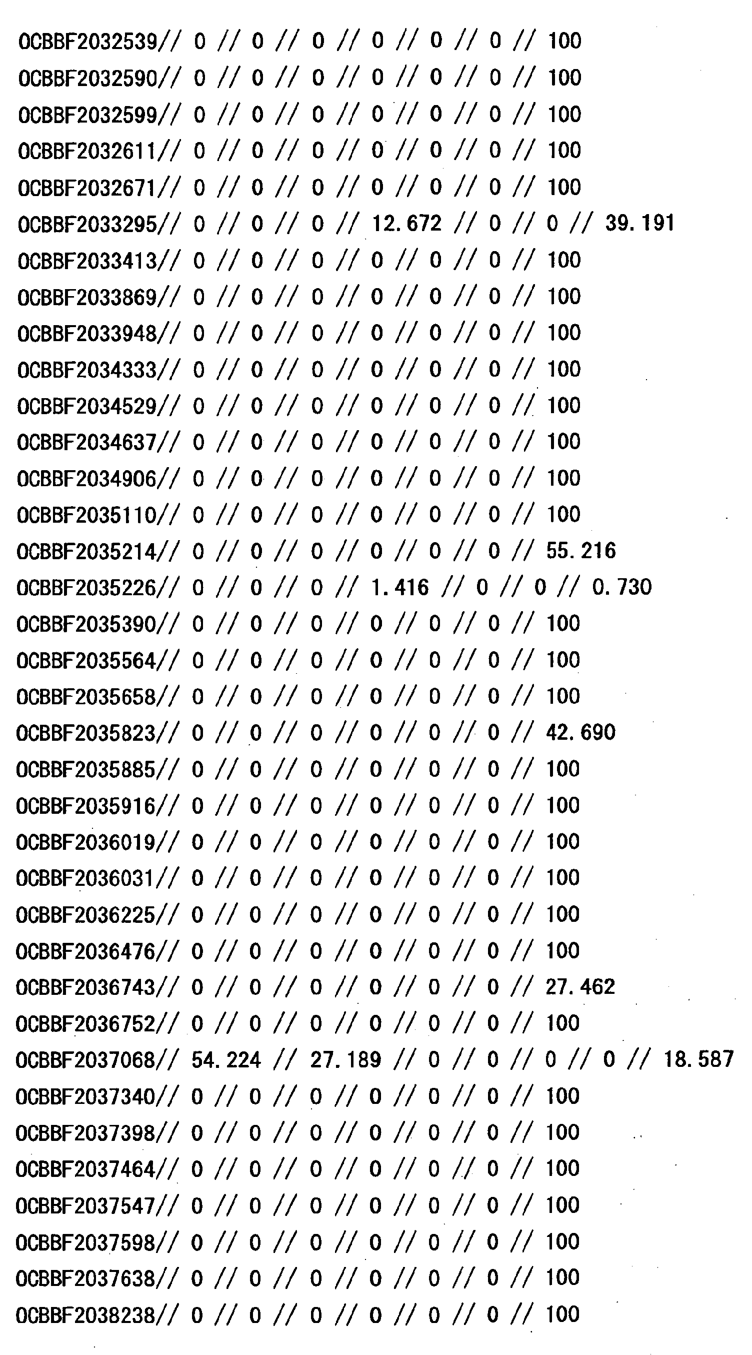

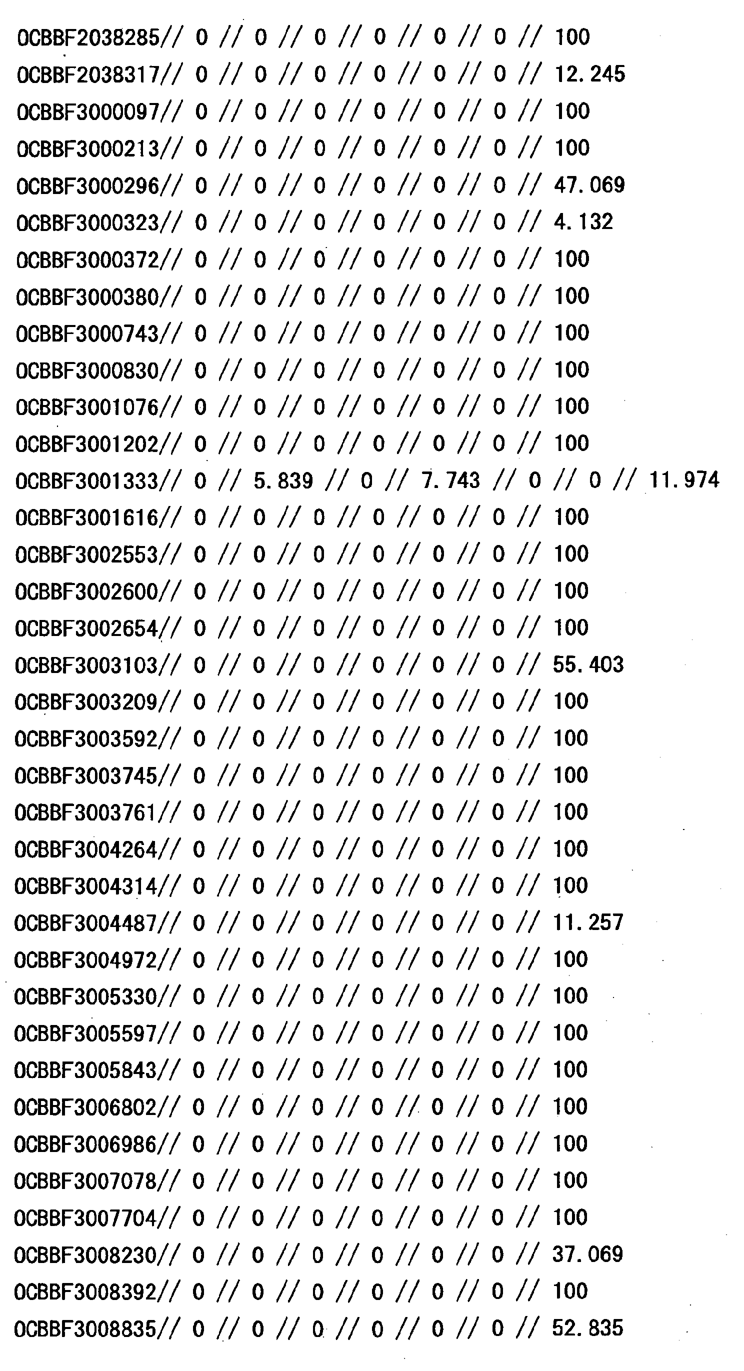

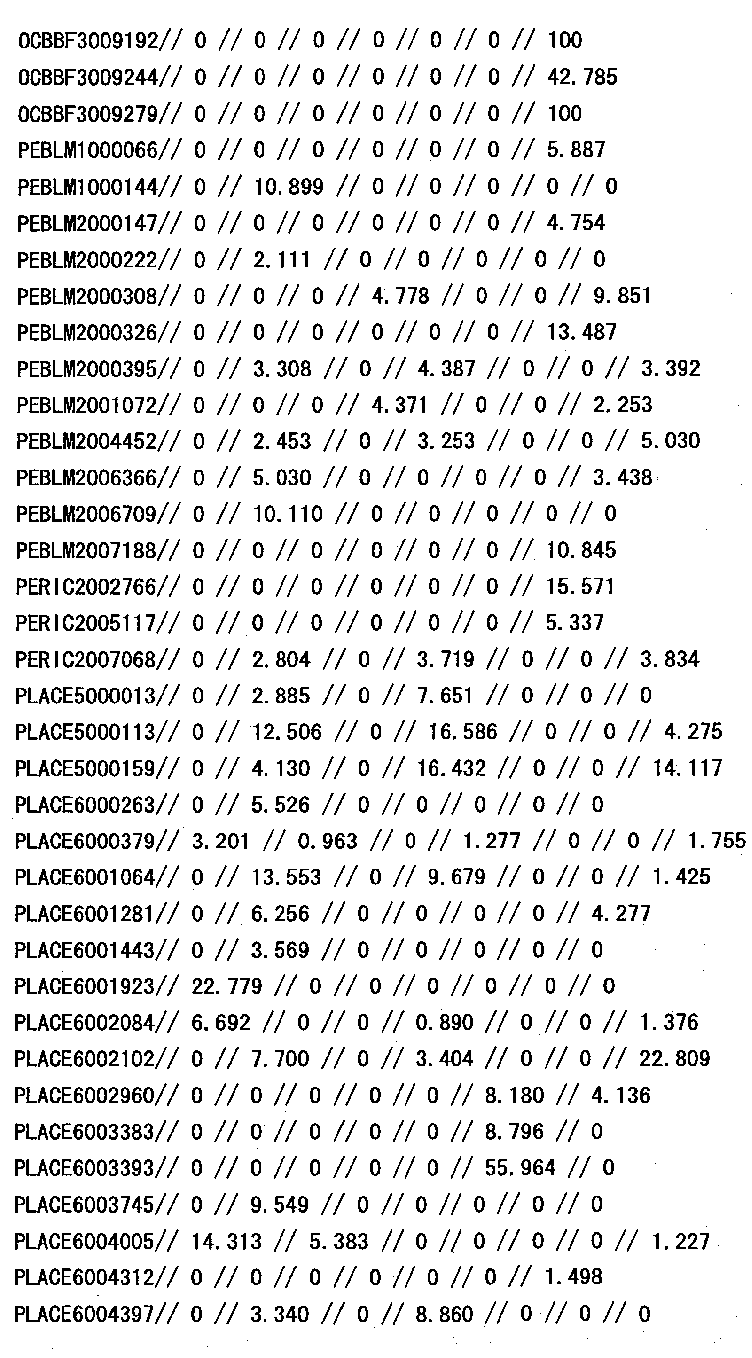

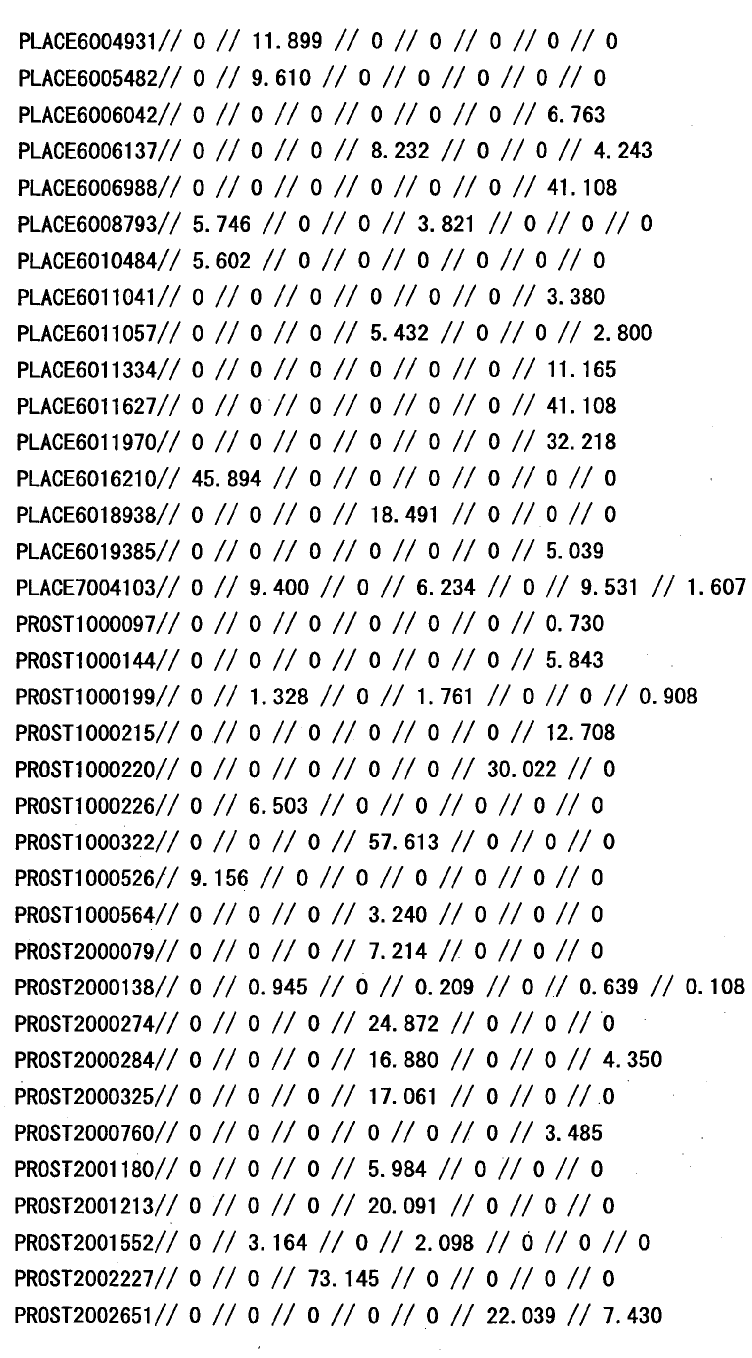

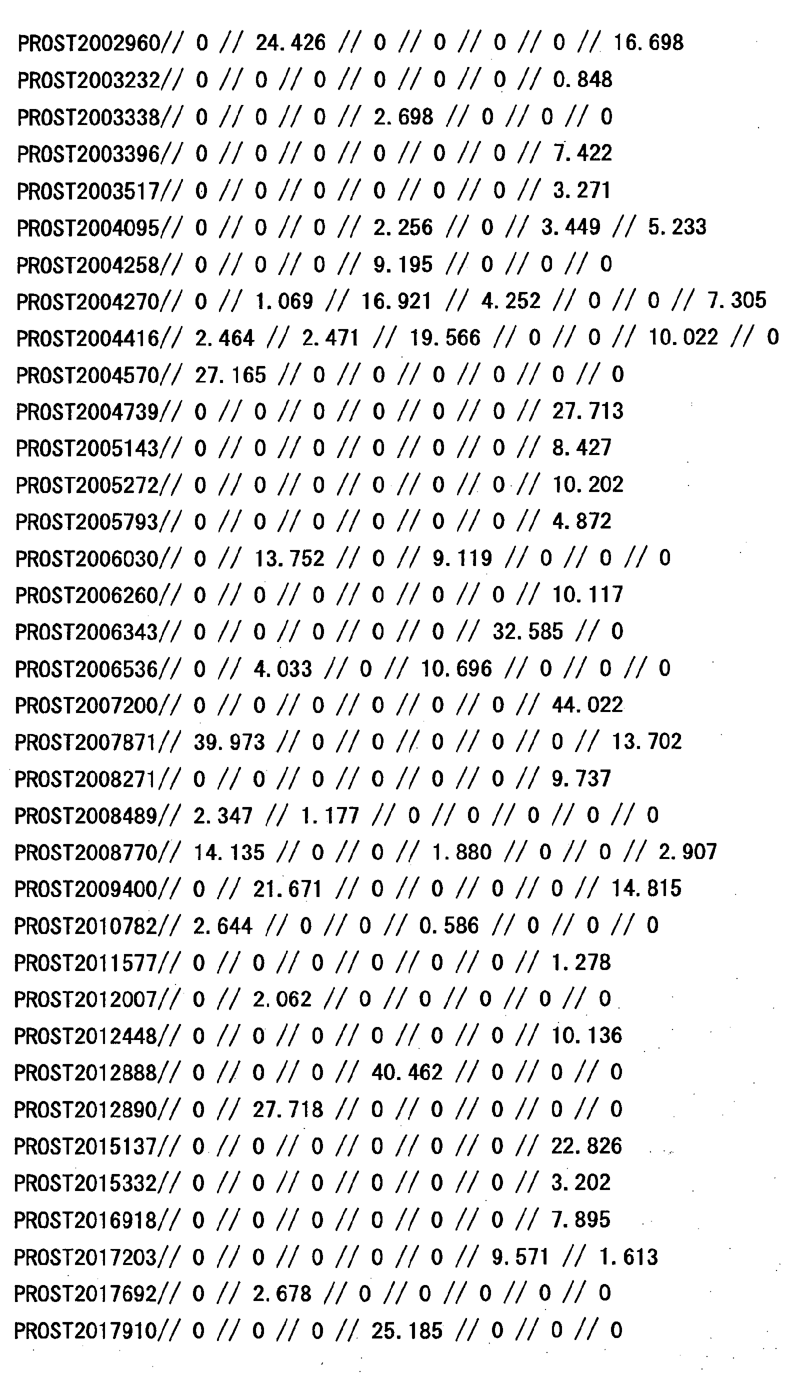

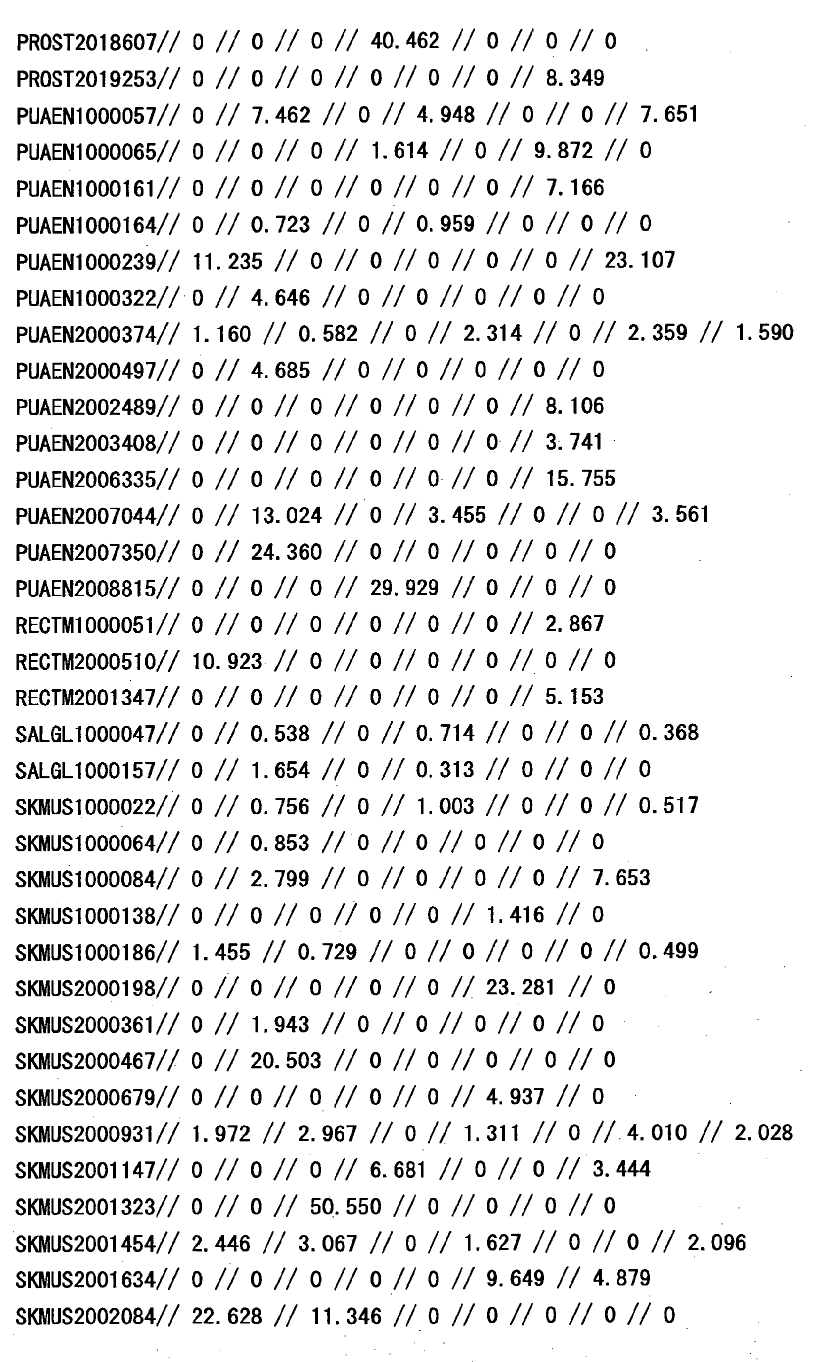

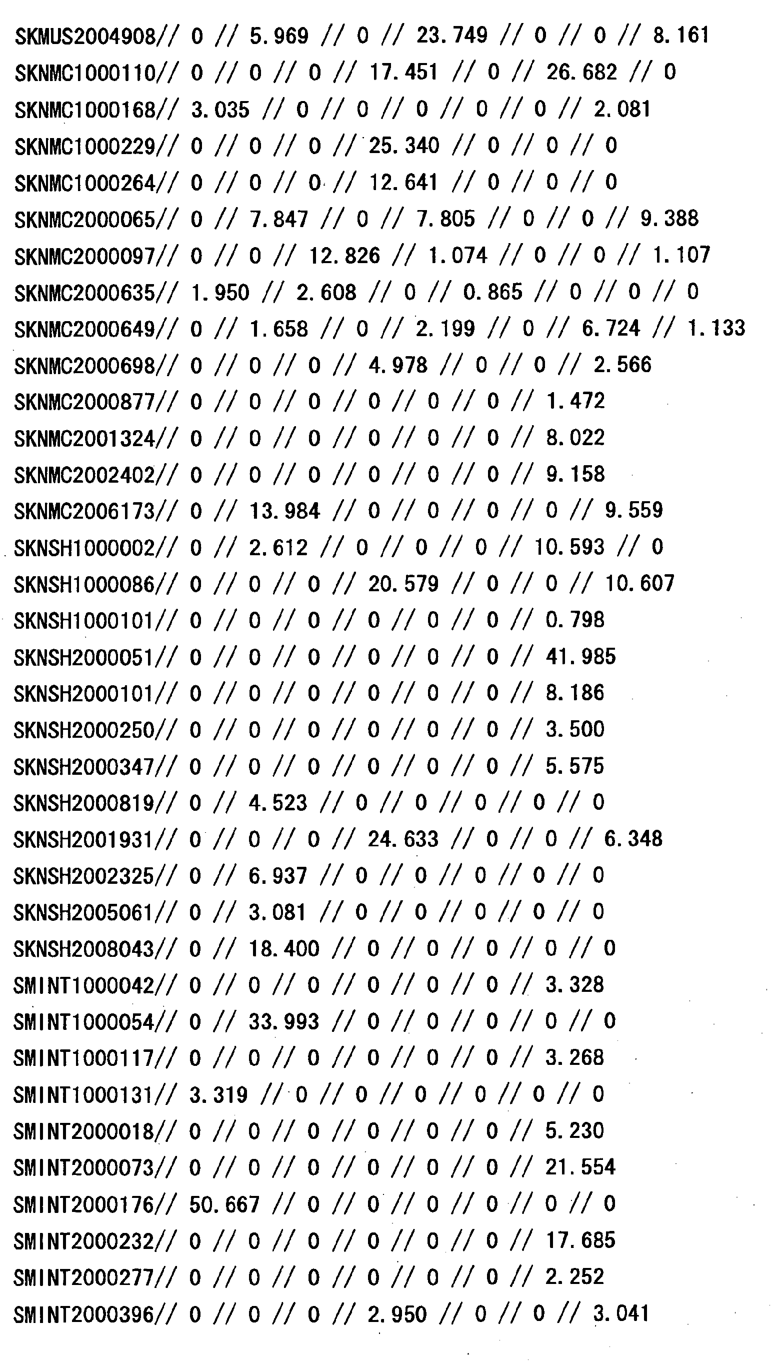

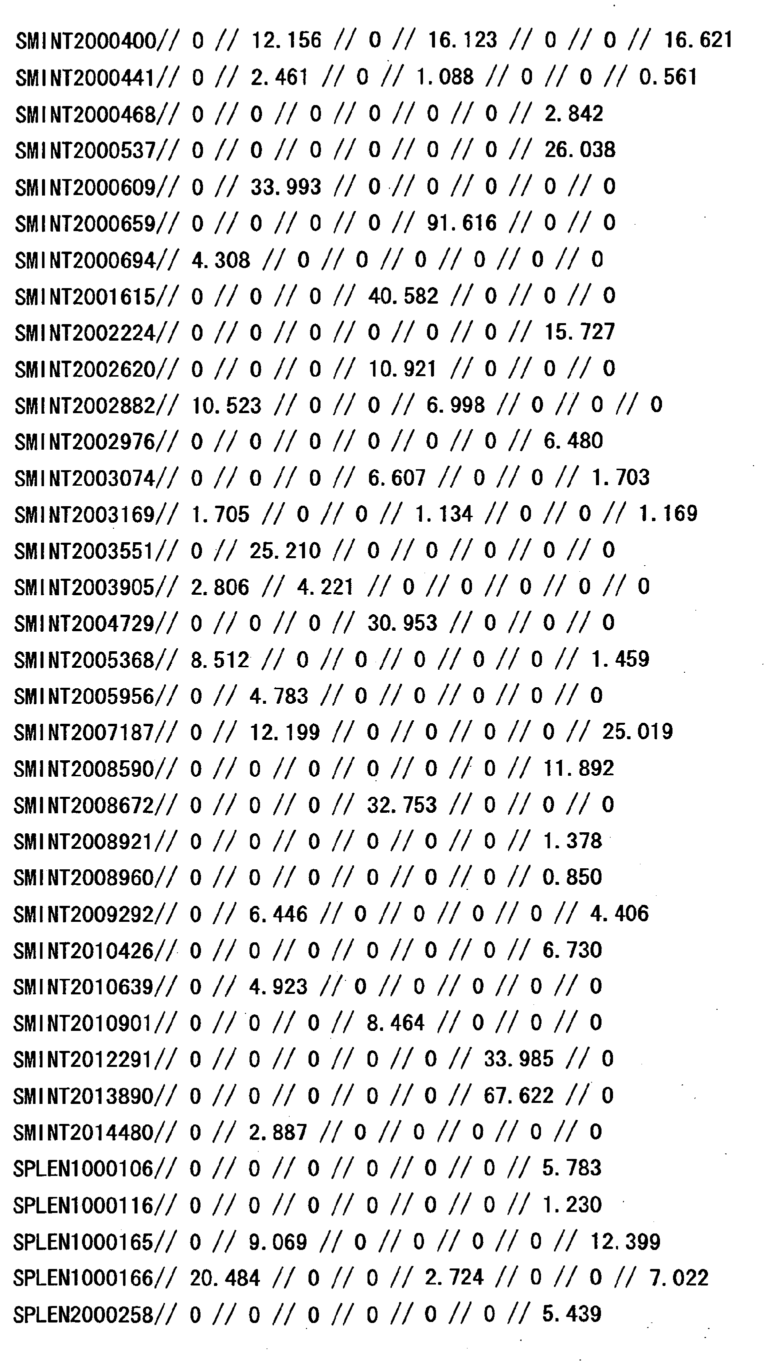

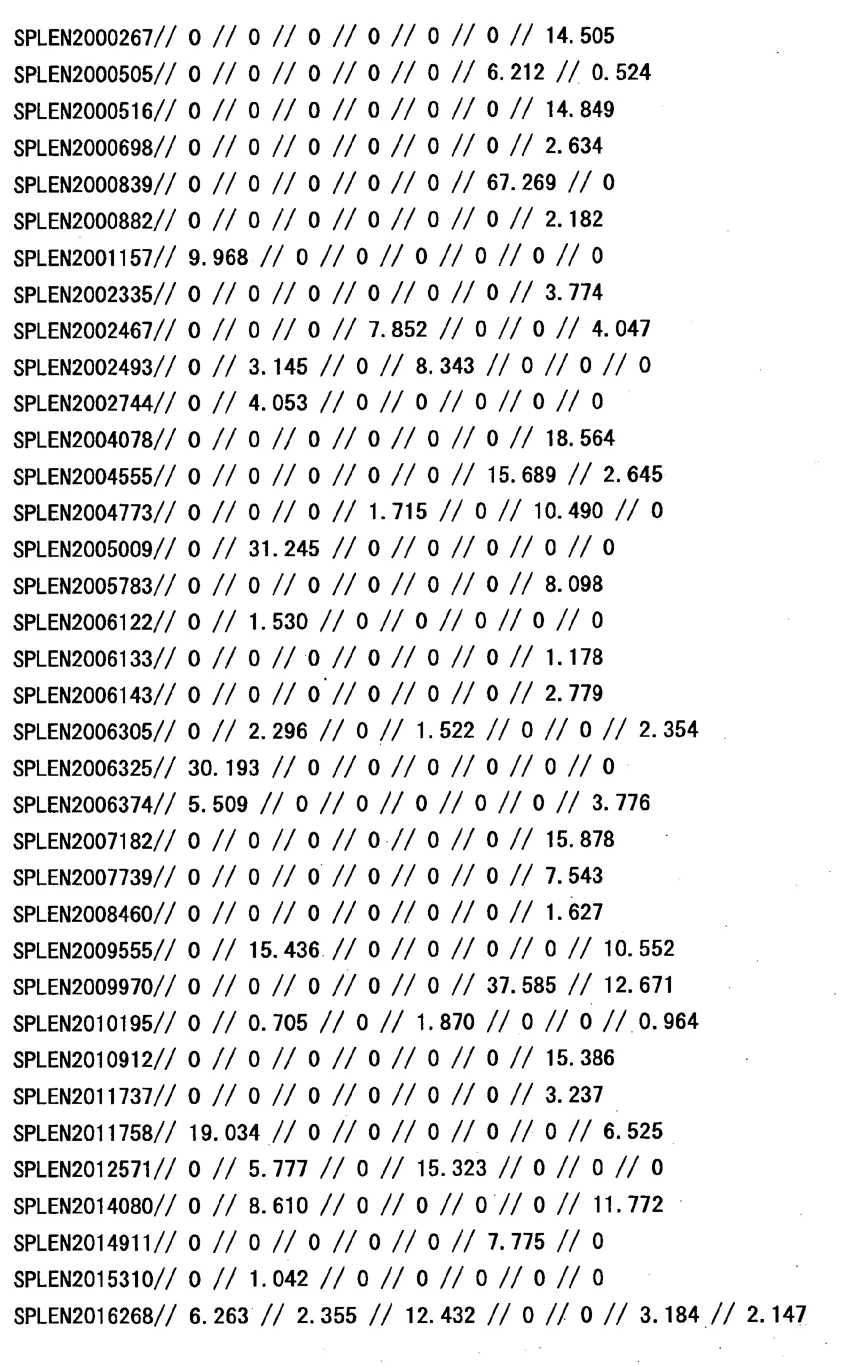

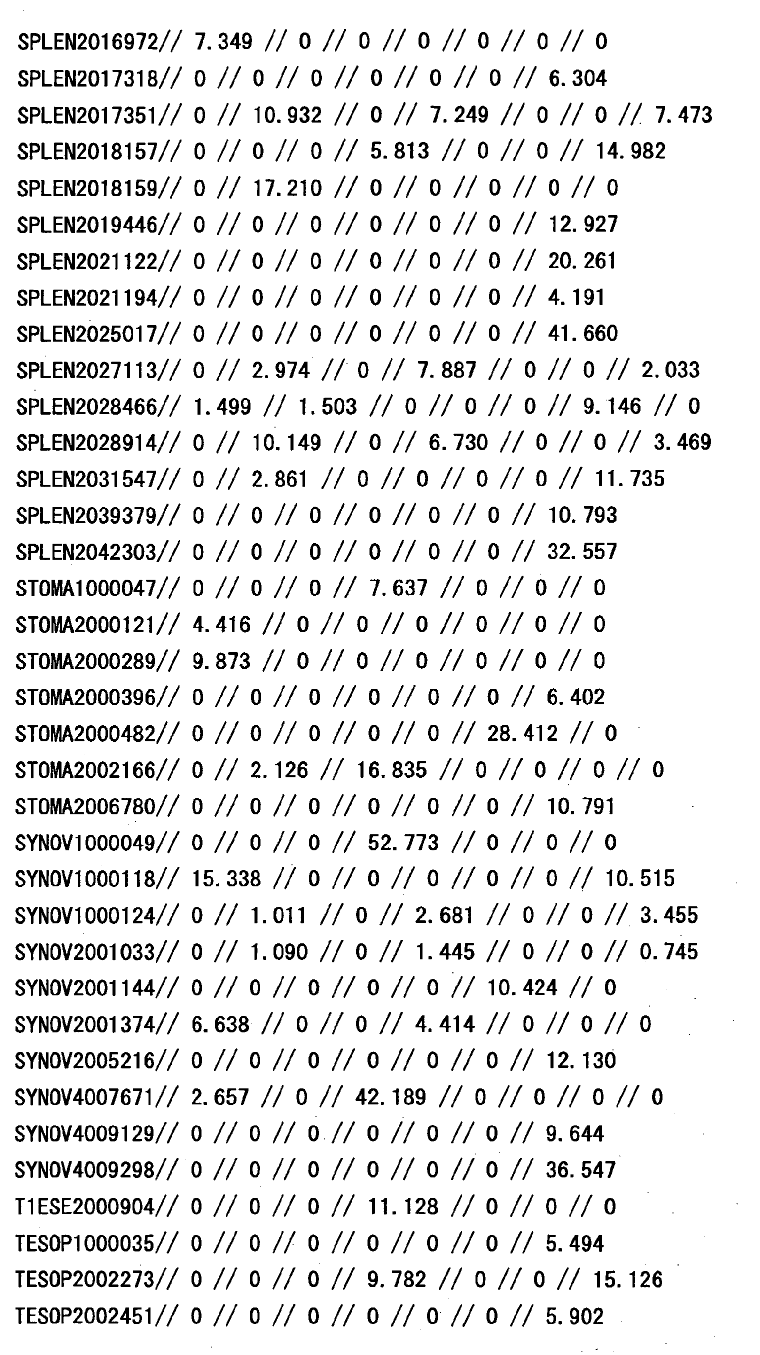

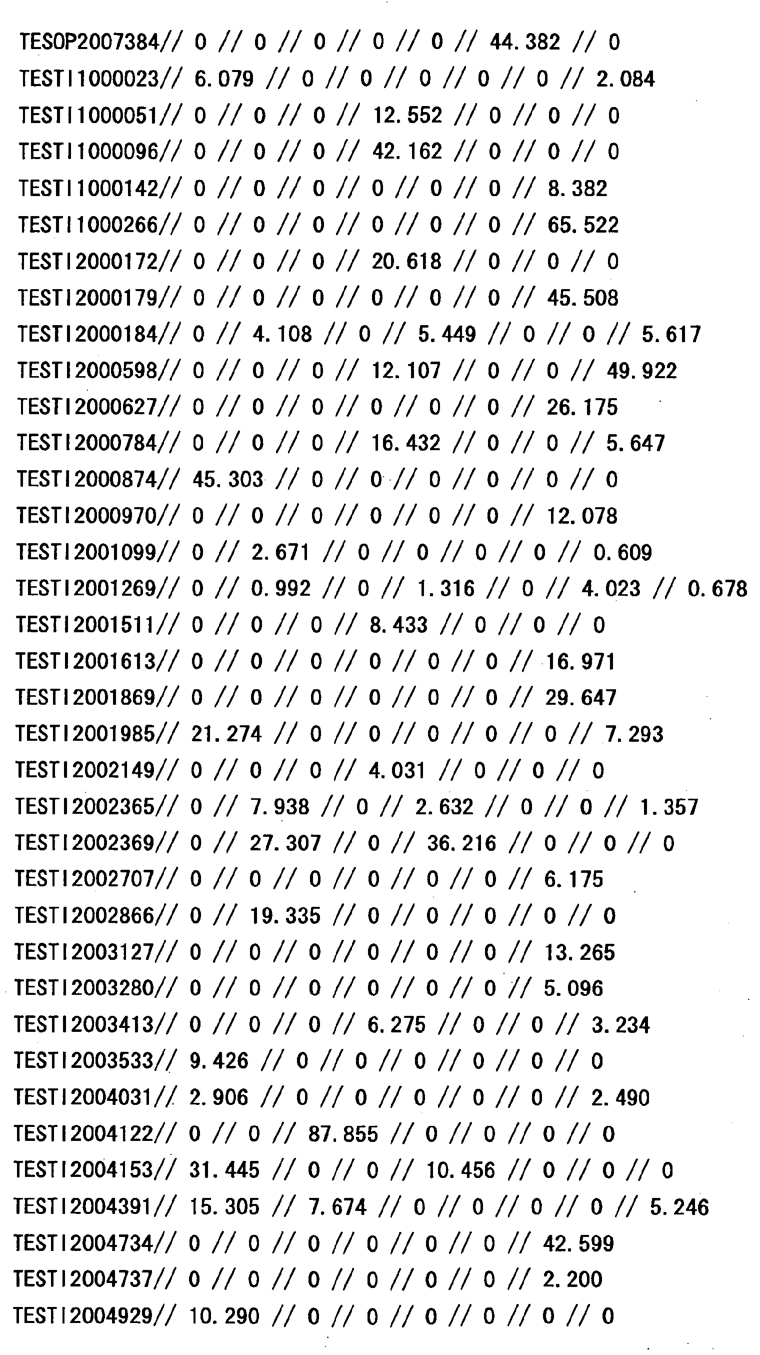

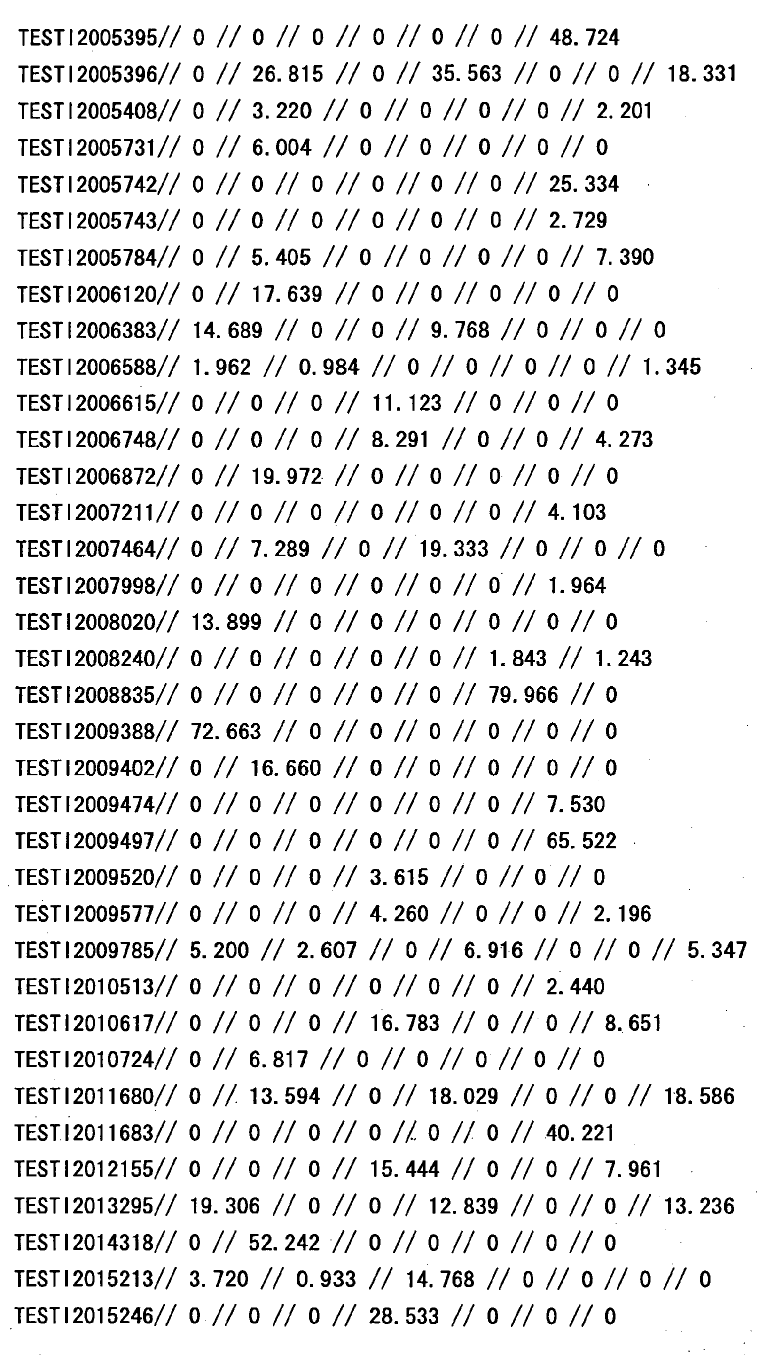

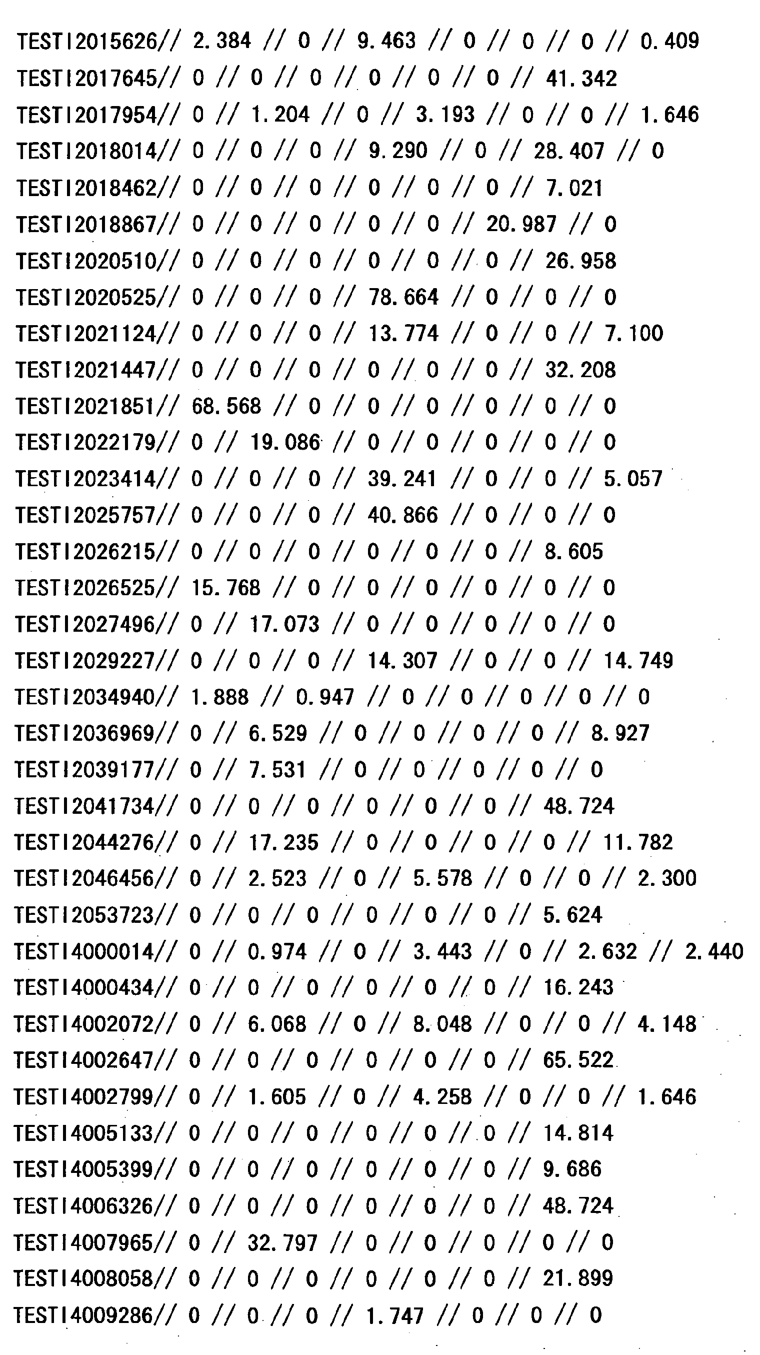

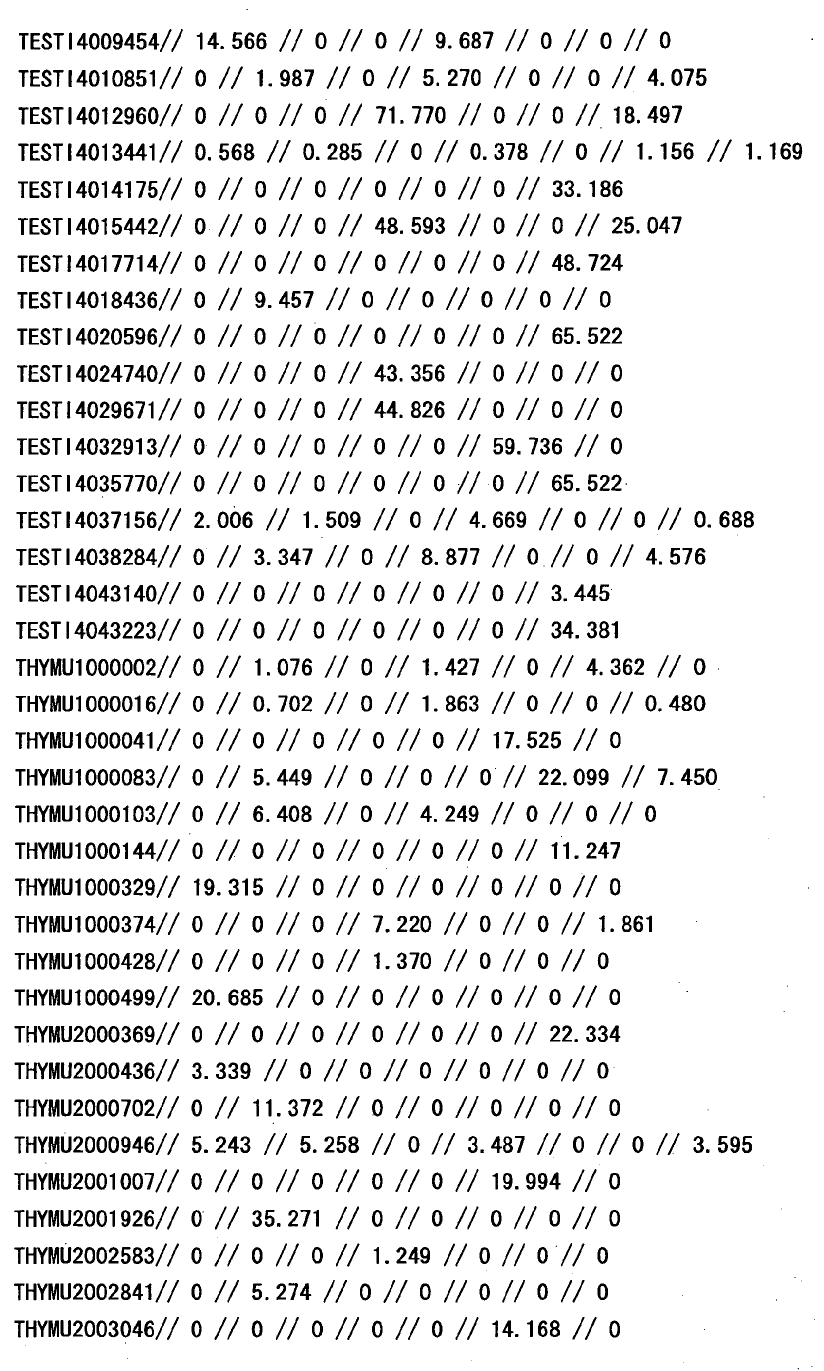

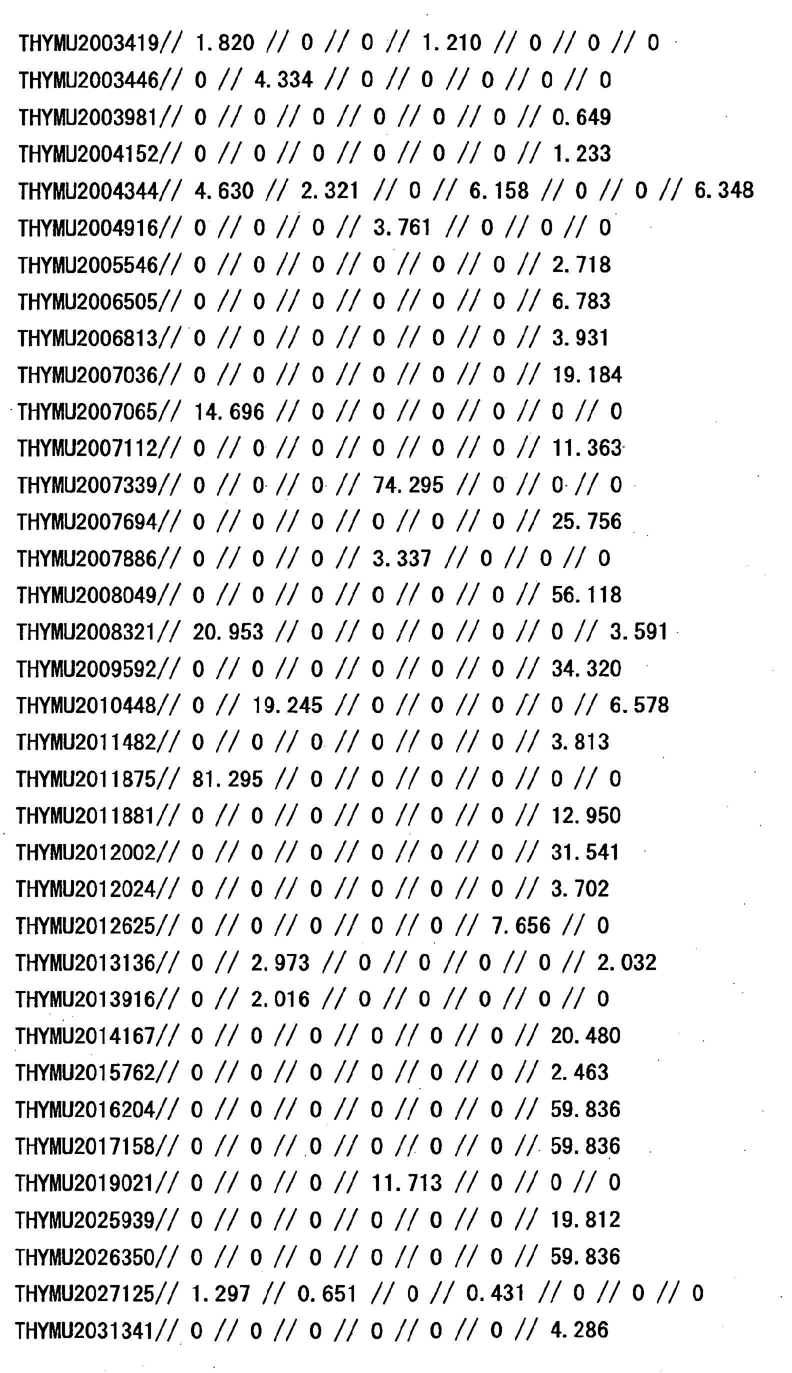

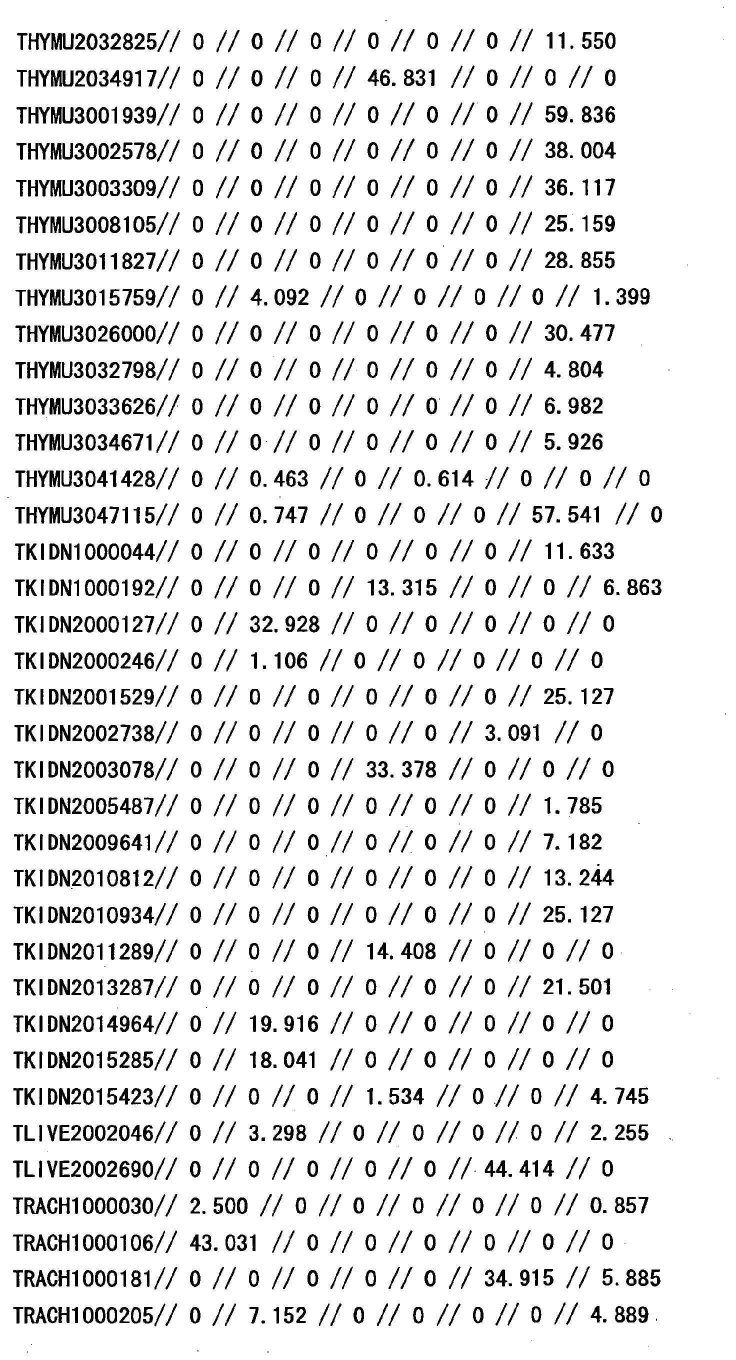

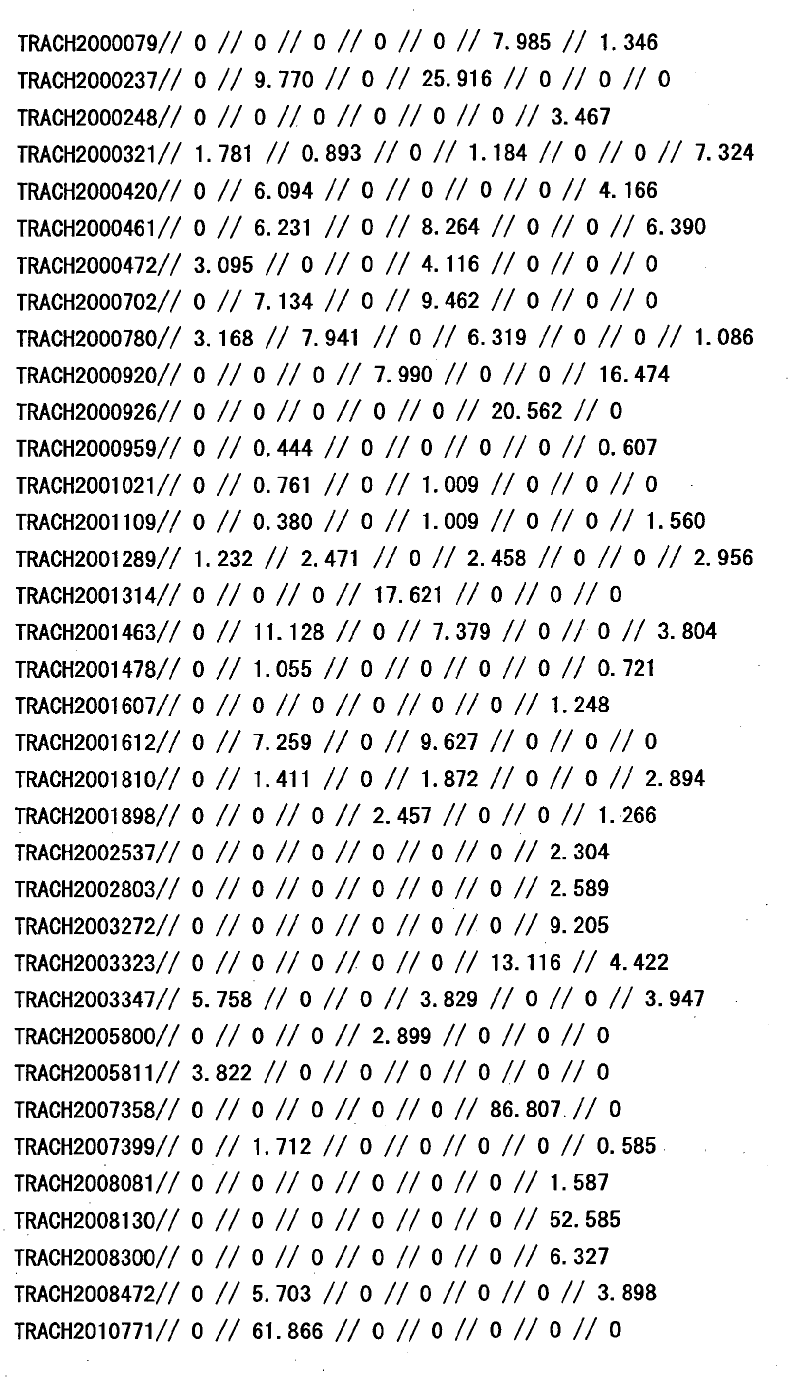

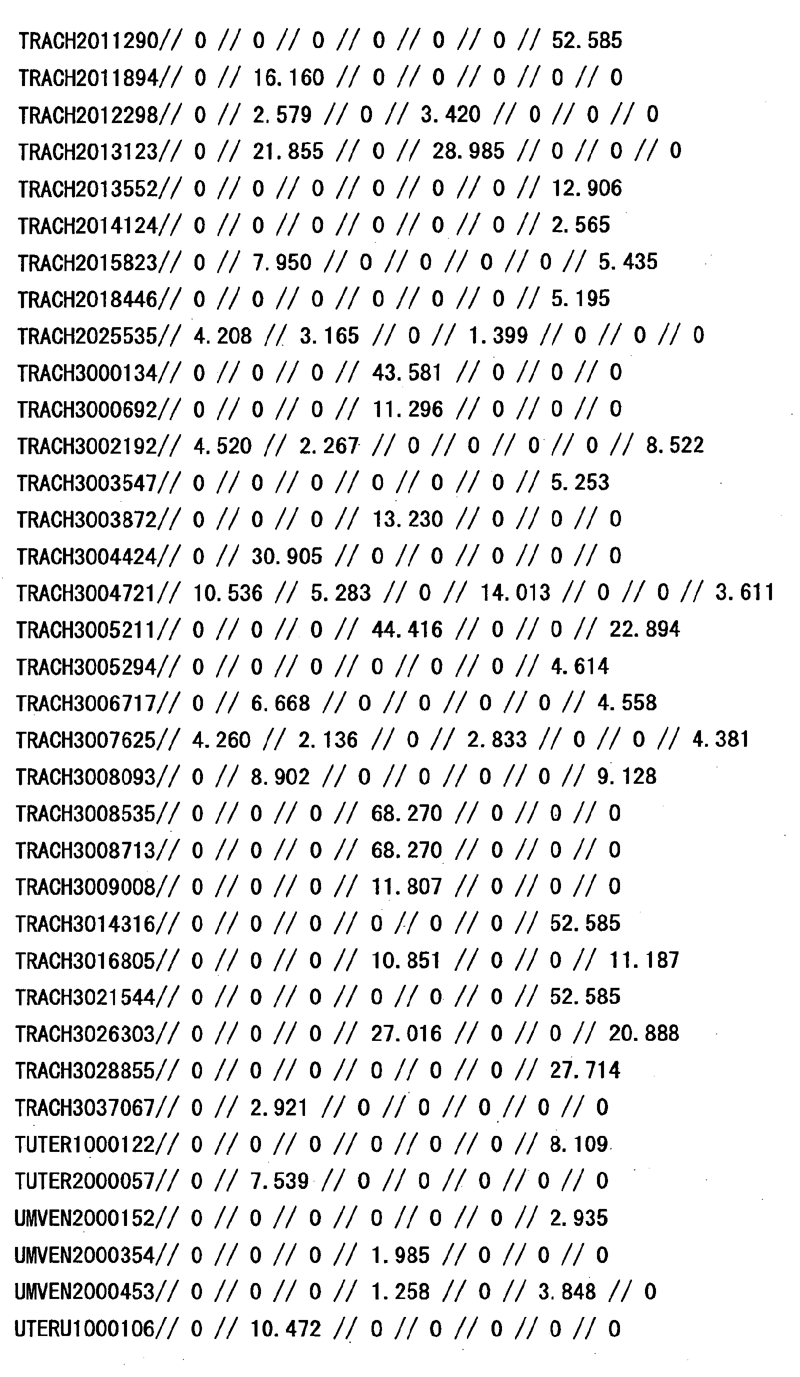

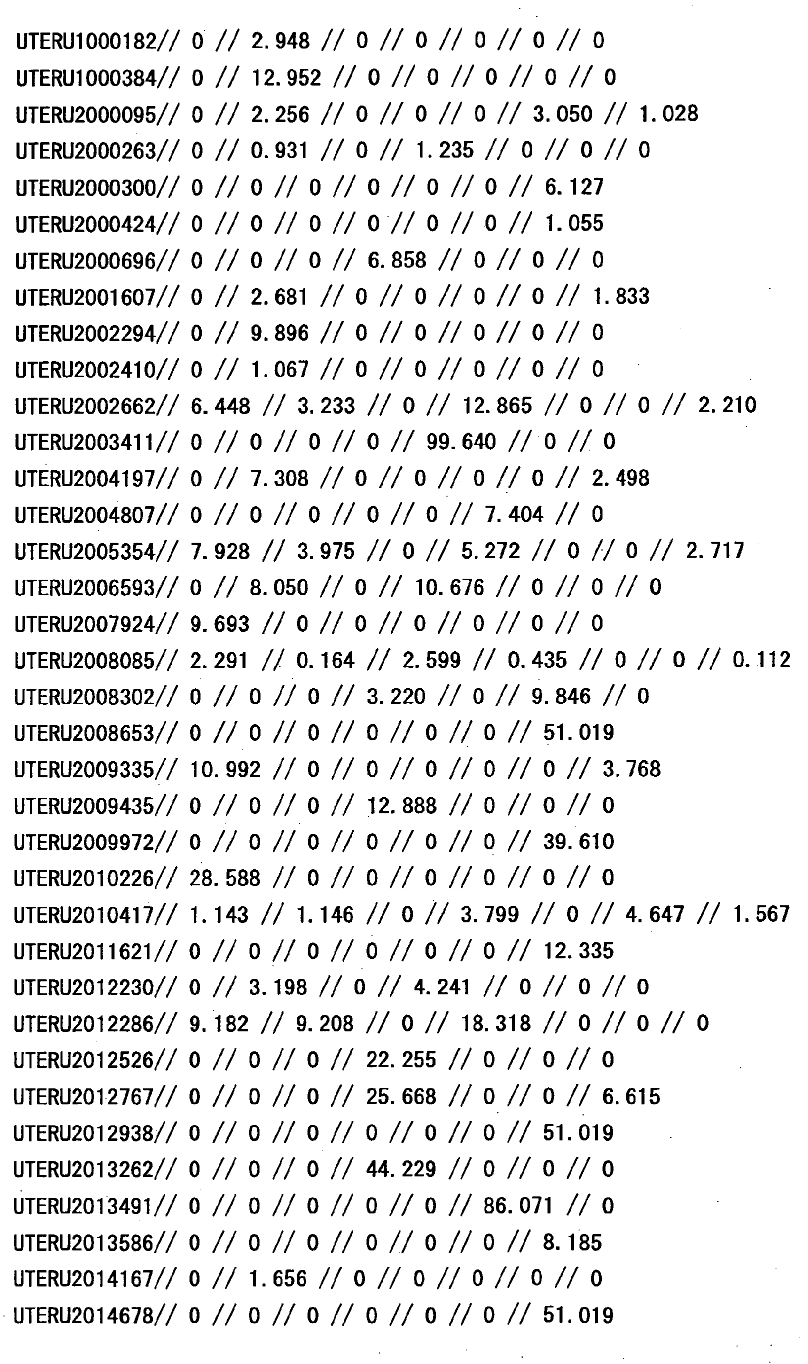

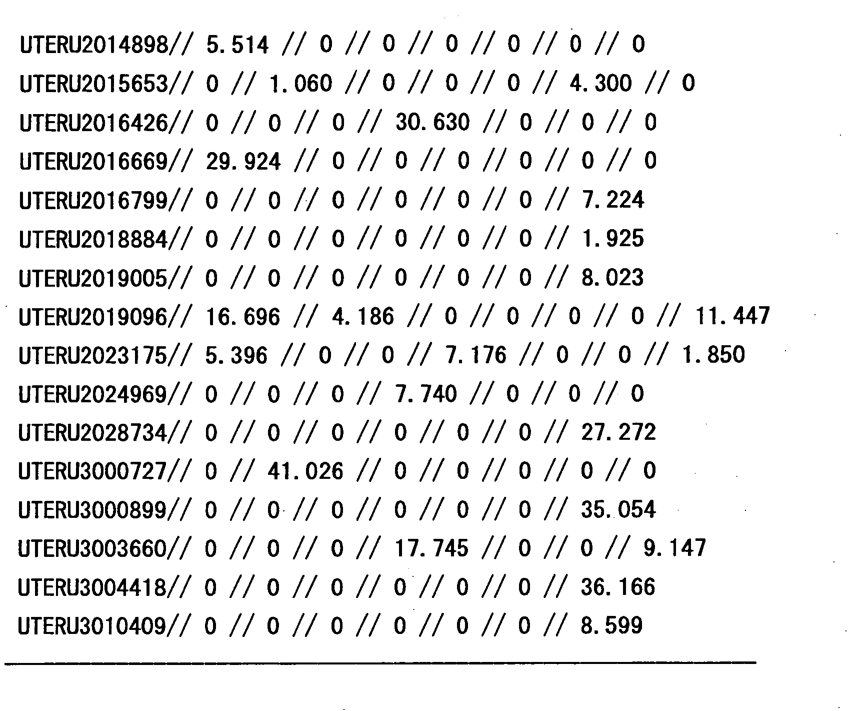

Genes whose expression levels were negligibly low in each of the above-described libraries are shown with their clone names below.
3NB691000018, 3NB691000085, 3NB691000113, 3NB691000191, 3NB692000012, 3NB692000029, 3NB692000102, 3NB692000154, 3NB692000276, 3NB692000305, 3NB692000330, 3NB692000429, 3NB692000484, 3NB692000529, 3NB692000973, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001501, 3NB692001507, 3NB692001511, 3NB692001519, 3NB692001520, 3NB692001538, 3NB692001548, 3NB692001557, 3NB692001637, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADRGL1000018, ADRGL1000033, ADRGL1000038, ADRGL1000067, ADRGL1000111, ADRGL1000147, ADRGL1000160, ADRGL1000165, ADRGL1000182, ADRGL2000056, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002753, ADRGL2002857, ADRGL2003017, ADRGL2003329, ADRGL2003552,
ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146,
ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, AHMSC1000138, ASTRO1000009, ASTRO1000018, ASTRO1000029, ASTRO1000096, ASTRO2000011, ASTRO2000014, ASTRO2000095, ASTRO2000141, ASTRO2000191, ASTRO2000278, ASTRO2000372, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001122, ASTRO2001227, ASTRO2001249, ASTRO2001480, ASTRO2001806, ASTRO2002024, ASTRO2002035, ASTRO2002064, ASTRO2002459, ASTRO2002571, ASTRO2002632, ASTRO2002659, ASTRO2002720, ASTRO2002733, ASTRO2002743,
ASTRO2002842, ASTRO2003024, ASTRO2003212, ASTRO2003574, ASTRO2003581, ASTRO2003740, ASTRO2003840, ASTRO2003925, ASTRO2003960, ASTRO2004051, ASTRO2004177, ASTRO2004217, ASTRO2004297, ASTRO2004584, ASTRO2004628, ASTRO2004751, ASTRO2004877, ASTRO2005008, ASTRO2005081, ASTRO2005096, ASTRO2005242, ASTRO2005273, ASTRO2005343, ASTRO2005360, ASTRO2005413, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007047, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008239,
ASTRO2008425, ASTRO2008508, ASTRO2008777, ASTRO2008895, ASTRO2008960, ASTRO2008972, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2011149, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015987, ASTRO2016044, ASTRO2016114, ASTRO2016681, ASTRO2016847, ASTRO2016892, ASTRO2017348, ASTRO2017627, ASTRO2018169, ASTRO2018373, ASTRO2018540,
ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000544, BGGI12000693, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972,
BLADE2003474, BLADE2003916, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008479, BLADE2008539, BLADE2008809, BLADE2008995, BLADE2009452, BLADE2009479, BLADE2009593, BNGH41000104, BNGH41000118, BNGH41000169, BNGH41000177, BNGH41000190, BNGH41000216, BNGH41000235, BNGH42000130, BNGH42000132,
BNGH42000405, BNGH42000532, BNGH42000675, BNGH42000791, BNGH42000815, BNGH42000994, BNGH42001247, BNGH42001406, BNGH42001576, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42008199, BNGH42008510, BNGH42008649, BNGH42008832, BNGH42008850, BNGH42008943, BNGH42009021, BRACE1000047, BRACE1000051, BRACE1000065, BRACE1000073, BRACE1000084, BRACE1000092, BRACE1000093, BRACE1000101, BRACE1000114, BRACE1000124, BRACE1000132, BRACE1000159, BRACE1000166,
BRACE1000169, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000331, BRACE2000351, BRACE2000368, BRACE2000392, BRACE2000441, BRACE2000489, BRACE2000505, BRACE2000526, BRACE2000531, BRACE2000545, BRACE2000553, BRACE2000565, BRACE2000577, BRACE2000640, BRACE2000650, BRACE2000677, BRACE2000718, BRACE2000743, BRACE2000750, BRACE2000753, BRACE2000815, BRACE2000864, BRACE2000885, BRACE2000893, BRACE2000905, BRACE2000936, BRACE2000947, BRACE2000965,
BRACE2000969, BRACE2000988, BRACE2001017, BRACE2001065, BRACE2001070, BRACE2001094, BRACE2001117, BRACE2001154, BRACE2001188, BRACE2001191, BRACE2001197, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737, BRACE2001742, BRACE2001777, BRACE2001779, BRACE2001834, BRACE2001859, BRACE2001865, BRACE2001872, BRACE2001898,
BRACE2001923, BRACE2001942, BRACE2001954, BRACE2001972, BRACE2001973, BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002392, BRACE2002394, BRACE2002409, BRACE2002431, BRACE2002444, BRACE2002458, BRACE2002467, BRACE2002468, BRACE2002478, BRACE2002495, BRACE2002585, BRACE2002589, BRACE2002590, BRACE2002606, BRACE2002613, BRACE2002614, BRACE2002617, BRACE2002623, BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792,
BRACE2002807, BRACE2002812, BRACE2002834, BRACE2002849, BRACE2002857, BRACE2002860, BRACE2002884, BRACE2002896, BRACE2002948, BRACE2003078, BRACE2003097, BRACE2003168, BRACE2003199, BRACE2003210, BRACE2003217, BRACE2003269, BRACE2003285, BRACE2003398, BRACE2003420, BRACE2003428, BRACE2003431, BRACE2003512, BRACE2003516, BRACE2003539, BRACE2003540, BRACE2003594, BRACE2003598, BRACE2003614, BRACE2003639, BRACE2003700, BRACE2003713, BRACE2003766, BRACE2003788, BRACE2003800, BRACE2003802, BRACE2003825, BRACE2003847, BRACE2003848, BRACE2003873, BRACE2003885, BRACE2003887, BRACE2003928, BRACE2003954, BRACE2003982, BRACE2003987, BRACE2005034, BRACE2005047, BRACE2005063, BRACE2005083, BRACE2005090,
BRACE2005099, BRACE2005118, BRACE2005124, BRACE2005138, BRACE2005160, BRACE2005169, BRACE2005193, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005731, BRACE2005742, BRACE2005762, BRACE2005773, BRACE2005787, BRACE2005795, BRACE2005800, BRACE2005858, BRACE2005869, BRACE2005881, BRACE2005898, BRACE2005904, BRACE2005906, BRACE2005907, BRACE2005911,
BRACE2005949, BRACE2005981, BRACE2005991, BRACE2006072, BRACE2006075, BRACE2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006264, BRACE2006274, BRACE2006298, BRACE2006344, BRACE2006363, BRACE2006378, BRACE2006380, BRACE2006393, BRACE2006397, BRACE2006413, BRACE2006417, BRACE2006453, BRACE2006459, BRACE2006488, BRACE2006534, BRACE2006537, BRACE2006540, BRACE2006547, BRACE2006564, BRACE2006587, BRACE2006598, BRACE2006685, BRACE2006703, BRACE2006753, BRACE2006783, BRACE2006821, BRACE2006871, BRACE2006909, BRACE2006911, BRACE2006924, BRACE2006935, BRACE2006944, BRACE2006951,
BRACE2006982, BRACE2006986, BRACE2007013, BRACE2007068, BRACE2007087, BRACE2007109, BRACE2007138, BRACE2007191, BRACE2007197, BRACE2007201, BRACE2007203, BRACE2007232, BRACE2007254, BRACE2007262, BRACE2007274, BRACE2007281, BRACE2007295, BRACE2007358, BRACE2007396, BRACE2007398, BRACE2007411, BRACE2007447, BRACE2007477, BRACE2007502, BRACE2007503, BRACE2007522, BRACE2007527, BRACE2007538, BRACE2007563, BRACE2007567, BRACE2007621, BRACE2007640, BRACE2007646, BRACE2007663, BRACE2007682, BRACE2007685, BRACE2007708, BRACE2007727, BRACE2007749, BRACE2007761, BRACE2007764, BRACE2007767, BRACE2007769, BRACE2007784, BRACE2007786, BRACE2007798, BRACE2007799, BRACE2007801, BRACE2007832, BRACE2007836,
BRACE2007868, BRACE2007882, BRACE2007888, BRACE2007920, BRACE2007944, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008385, BRACE2008399, BRACE2008401, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881,
BRACE2008883, BRACE2008917, BRACE2008919, BRACE2008941, BRACE2008948, BRACE2008960, BRACE2008999, BRACE2009014, BRACE2009016, BRACE2009031, BRACE2009044, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009366, BRACE2009421, BRACE2009428, BRACE2009434, BRACE2009437, BRACE2009483, BRACE2009517, BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171,
BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838,
BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014475, BRACE2014657, BRACE2014746, BRACE2014780, BRACE2014818, BRACE2014821, BRACE2014824, BRACE2014854, BRACE2015031, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896,
BRACE2016981, BRACE2017108, BRACE2017124, BRACE2017298, BRACE2017359, BRACE2017397, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278,
BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022333, BRACE2022394, BRACE2022435, BRACE2022448, BRACE2022450, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744,
BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025710, BRACE2025796, BRACE2025992, BRACE2026131, BRACE2026141, BRACE2026142, BRACE2026293, BRACE2026294, BRACE2026404, BRACE2026675, BRACE2026836, BRACE2026920, BRACE2026971, BRACE2027018, BRACE2027221, BRACE2027312, BRACE2027382, BRACE2027389, BRACE2027408, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396 BRACE2029581, BRACE2029644, BRACE2029849,
BRACE2030039, BRACE2030096, BRACE2030323, BRACE2030326, BRACE2030341, BRACE2030345, BRACE2030736, BRACE2030883, BRACE2030884,BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035381, BRACE2035441, BRACE2035485, BRACE2036005, BRACE2036096, BRACE2036743, BRACE2036830, BRACE2036834, BRACE2036900,
BRACE2037132, BRACE2037294, BRACE2037299, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248,
BRACE2043349, BRACE2043381, BRACE2043774, BRACE2043942, BRACE2044057, BRACE2044105, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001217, BRACE3001299, BRACE3001384, BRACE3001391, BRACE3001403, BRACE3001595, BRACE3001754,
BRACE3001948, BRACE3002298, BRACE3002344, BRACE3002390, BRACE3002420, BRACE3002508, BRACE3002541, BRACE3002756, BRACE3003004, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553,
BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416,
BRACE3010435, BRACE3010438, BRACE3010702, BRACE3010862, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308,
BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456,
BRACE3026735, BRACE3026802, BRACE3026844, BRACE3026947, BRACE3026993, BRACE3027242, BRACE3027256, BRACE3027326, BRACE3027478, BRACE3027480, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156,
BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609,
BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696,
BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000585,
BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926,
BRAMY2003929, BRAMY2004031, BRAMY2004058, BRAMY2004183, BRAMY2004335, BRAMY2004351, BRAMY2004352, BRAMY2004387, BRAMY2004492, BRAMY2004521, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308,
BRAMY2007411, BRAMY2007458, BRAMY2007486, BRAMY2007546, BRAMY2007594, BRAMY2007610, BRAMY2007653, BRAMY2007753, BRAMY2007859, BRAMY2008333, BRAMY2008563, BRAMY2008583, BRAMY2008977, BRAMY2008979, BRAMY2009023, BRAMY2009034, BRAMY2009105, BRAMY2009123, BRAMY2009192, BRAMY2009344, BRAMY2009349, BRAMY2009394, BRAMY2009489, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010366, BRAMY2010395, BRAMY2010441, BRAMY2010442, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253,
BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012536, BRAMY2012555, BRAMY2012568, BRAMY2012691, BRAMY2012721, BRAMY2012731, BRAMY2012776, BRAMY2012880, BRAMY2012881, BRAMY2013200, BRAMY2013216, BRAMY2013405, BRAMY2013414, BRAMY2013572, BRAMY2013590, BRAMY2013722, BRAMY2013736, BRAMY2013944, BRAMY2013975, BRAMY2014019, BRAMY2014387, BRAMY2014462, BRAMY2014469, BRAMY2014541, BRAMY2014754, BRAMY2014813, BRAMY2015211, BRAMY2015311, BRAMY2015420, BRAMY2015503, BRAMY2015516,
BRAMY2015550, BRAMY2015551, BRAMY2015559, BRAMY2015688, BRAMY2015782, BRAMY2015833, BRAMY2015855, BRAMY2015890, BRAMY2015911, BRAMY2015925, BRAMY2016002, BRAMY2016020, BRAMY2016070, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2019055, BRAMY2019111, BRAMY2019277, BRAMY2019300, BRAMY2019468, BRAMY2019509, BRAMY2019643, BRAMY2019710, BRAMY2019985, BRAMY2019989, BRAMY2020050,
BRAMY2020058, BRAMY2020270, BRAMY2020354, BRAMY2020355, BRAMY2020412, BRAMY2020427, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172,
BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538,
BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027714, BRAMY2027717, BRAMY2027739, BRAMY2027752, BRAMY2027782, BRAMY2028072, BRAMY2028183, BRAMY2028188, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442,
BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968,
BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760,
BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002458,
BRAMY3002508, BRAMY3002620, BRAMY3002803, BRAMY3002805, BRAMY3002886, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005656, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492,
BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRASW1000053, BRASW1000125, BRAWH1000001, BRAWH1000002, BRAWH1000083, BRAWH1000094, BRAWH1000116, BRAWH1000130, BRAWH1000157, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000323, BRAWH2000353, BRAWH2000370, BRAWH2000409, BRAWH2000417, BRAWH2000443, BRAWH2000471,
BRAWH2000476, BRAWH2000482, BRAWH2000494, BRAWH2000500, BRAWH2000503, BRAWH2000554, BRAWH2000595, BRAWH2000643, BRAWH2000651, BRAWH2000682, BRAWH2000752, BRAWH2000859, BRAWH2000866, BRAWH2000892, BRAWH2000901, BRAWH2000926, BRAWH2000984, BRAWH2001019, BRAWH2001052, BRAWH2001103, BRAWH2001129, BRAWH2001137, BRAWH2001159, BRAWH2001203, BRAWH2001238, BRAWH2001239, BRAWH2001255, BRAWH2001355, BRAWH2001378, BRAWH2001395, BRAWH2001406, BRAWH2001418, BRAWH2001436, BRAWH2001438, BRAWH2001459, BRAWH2001461, BRAWH2001492, BRAWH2001503, BRAWH2001519, BRAWH2001535, BRAWH2001589, BRAWH2001666, BRAWH2001671, BRAWH2001679, BRAWH2001686, BRAWH2001701, BRAWH2001842, BRAWH2001873, BRAWH2001940, BRAWH2001973,
BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002601, BRAWH2002623, BRAWH2002630, BRAWH2002635, BRAWH2002662, BRAWH2002679, BRAWH2002725, BRAWH2002761, BRAWH2002861, BRAWH2002911, BRAWH2002963, BRAWH2002976, BRAWH2003000, BRAWH2003025, BRAWH2003135, BRAWH2003240, BRAWH2003307, BRAWH2003355, BRAWH2003366, BRAWH2003689, BRAWH2003832, BRAWH2003948, BRAWH2003964, BRAWH2004044, BRAWH2004068, BRAWH2004078, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005315, BRAWH2005524, BRAWH2005533,
BRAWH2005578, BRAWH2005812, BRAWH2005974, BRAWH2005998, BRAWH2006083, BRAWH2006207, BRAWH2006301, BRAWH2006395, BRAWH2006405, BRAWH2006450, BRAWH2006493, BRAWH2006526, BRAWH2006543, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007658, BRAWH2007808, BRAWH2007825, BRAWH2007862, BRAWH2007890, BRAWH2008128, BRAWH2008219, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393,
BRAWH2009485, BRAWH2009490, BRAWH2009558, BRAWH2009590, BRAWH2009678, BRAWH2009695, BRAWH2009907, BRAWH2010000, BRAWH2010069, BRAWH2010084, BRAWH2010136, BRAWH2010318, BRAWH2010354, BRAWH2010364, BRAWH2010536, BRAWH2010552, BRAWH2010584, BRAWH2010618, BRAWH2010619, BRAWH2011066, BRAWH2011079, BRAWH2011096, BRAWH2011155, BRAWH2011166, BRAWH2011204, BRAWH2011294, BRAWH2011343, BRAWH2011400, BRAWH2011762, BRAWH2011795, BRAWH2011796, BRAWH2011812, BRAWH2011860, BRAWH2011958, BRAWH2012054, BRAWH2012077, BRAWH2012161, BRAWH2012162, BRAWH2012258, BRAWH2012277, BRAWH2012326, BRAWH2012538, BRAWH2012540, BRAWH2012619, BRAWH2012698, BRAWH2012749, BRAWH2012827, BRAWH2012963, BRAWH2013047, BRAWH2013219,
BRAWH2013294, BRAWH2013748, BRAWH2013866, BRAWH2013871, BRAWH2013914, BRAWH2013941, BRAWH2013955, BRAWH2014053, BRAWH2014234, BRAWH2014414, BRAWH2014473, BRAWH2014511, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016514, BRAWH2016562, BRAWH2016655, BRAWH2016679, BRAWH2016702, BRAWH2016724, BRAWH2016785, BRAWH2016792, BRAWH2016800, BRAWH2017103, BRAWH2017250, BRAWH2017294, BRAWH2017305, BRAWH2017379, BRAWH2017407,
BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3002433, BRAWH3002467, BRAWH3002513, BRAWH3002600, BRAWH3002819, BRAWH3003019, BRAWH3003136, BRAWH3003244,
BRAWH3003343, BRAWH3003411, BRAWH3003522, BRAWH3003573, BRAWH3003598, BRAWH3003801, BRAWH3003975, BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009961, BRAWH3010560, BRAWH3010602, BRAWH3010657, BRAWH3010726, BRAWH3010833, BRAWH3011101, BRAWH3011331,
BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014639, BRAWH3015017, BRAWH3015175, BRAWH3015260, BRAWH3015610, BRAWH3015825, BRAWH3016123, BRAWH3016271, BRAWH3016715, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542,
BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620,
BRAWH3032930, BRAWH3033117, BRAWH3033293, BRAWH3033448, BRAWH3033513, BRAWH3034097, BRAWH3034114, BRAWH3034134, BRAWH3034668, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000577, BRCAN2000620, BRCAN2000639, BRCAN2000763, BRCAN2000832, BRCAN2000923, BRCAN2001223,
BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203,
BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387,
BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC1000094, BRCOC2000004,
BRCOC2000047, BRCOC2000186, BRCOC2000333, BRCOC2000360, BRCOC2000404, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003187, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052,
BRCOC2009196, BRCOC2009380, BRCOC2009638, BRCOC2009937, BRCOC2010115, BRCOC2010730, BRCOC2010980, BRCOC2010994, BRCOC2011010, BRCOC2011398, BRCOC2011418, BRCOC2011506, BRCOC2011769, BRCOC2011996, BRCOC2012229, BRCOC2012551, BRCOC2012813, BRCOC2013448, BRCOC2013573, BRCOC2013675, BRCOC2013780, BRCOC2014013, BRCOC2014033, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019934, BRCOC2020122, BRCOC2020142, BRHIP1000058, BRHIP1000072, BRHIP1000129, BRHIP1000258, BRHIP2000021, BRHIP2000079,
BRHIP2000087, BRHIP2000163, BRHIP2000210, BRHIP2000239, BRHIP2000245, BRHIP2000359, BRHIP2000445, BRHIP2000506, BRHIP2000532, BRHIP2000621, BRHIP2000691, BRHIP2000819, BRHIP2000826, BRHIP2000920, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719,
BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360,
BRHIP2012545, BRHIP2012580, BRHIP2012642, BRHIP2012750, BRHIP2012922, BRHIP2012923, BRHIP2012972, BRHIP2013447, BRHIP2013510, BRHIP2013699, BRHIP2013723, BRHIP2013958, BRHIP2013972, BRHIP2014063, BRHIP2014285, BRHIP2014291, BRHIP2014373, BRHIP2014386, BRHIP2014387, BRHIP2014954, BRHIP2015153, BRHIP2015356, BRHIP2015360, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177,
BRHIP2019186, BRHIP2019291, BRHIP2019494, BRHIP2019589, BRHIP2019641, BRHIP2019884, BRHIP2019972, BRHIP2020182, BRHIP2020509, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021870, BRHIP2021929, BRHIP2022006, BRHIP2022054, BRHIP2022221, BRHIP2022298, BRHIP2022467, BRHIP2022708, BRHIP2023071, BRHIP2023229, BRHIP2023272, BRHIP2023292, BRHIP2023309, BRHIP2023369, BRHIP2023438, BRHIP2023695, BRHIP2023735, BRHIP2023773, BRHIP2023860, BRHIP2023869, BRHIP2024046, BRHIP2024165, BRHIP2024201, BRHIP2024347, BRHIP2024549, BRHIP2024789, BRHIP2024941, BRHIP2025245, BRHIP2025366, BRHIP2025679,
BRHIP2025739, BRHIP2025797, BRHIP2026021, BRHIP2026155, BRHIP2026214, BRHIP2026288, BRHIP2026346, BRHIP2026557, BRHIP2026723, BRHIP2026877, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP3000060, BRHIP3000070, BRHIP3000081, BRHIP3000240, BRHIP3000377, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002920, BRHIP3002931,
BRHIP3003063, BRHIP3003126, BRHIP3003306, BRHIP3003340, BRHIP3003395, BRHIP3003484, BRHIP3003644, BRHIP3003688, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007640, BRHIP3007960, BRHIP3008183, BRHIP3008314, BRHIP3008344, BRHIP3008405,
BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558,
BRHIP3017637, BRHIP3017855, BRHIP3018278, BRHIP3019643, BRHIP3019824, BRHIP3019880, BRHIP3019956, BRHIP3020046, BRHIP3020155, BRHIP3020182, BRHIP3020733, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244,
BRSSN2000293, BRSSN2000295, BRSSN2000312, BRSSN2000518, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419,
BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589,
BRSSN2016768, BRSSN2016905, BRSSN2017297, BRSSN2017422, BRSSN2017530, BRSSN2017682, BRSSN2017757, BRSSN2018218, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475,
BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013502, BRSTN2013931, BRSTN2014490, BRSTN2014633, BRSTN2014751, BRSTN2014770, BRSTN2015015, BRSTN2015978, BRSTN2016066, BRSTN2016335, BRSTN2016470, BRSTN2016678, BRSTN2016892, BRSTN2016918, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021,
BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831,
BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183,
BRTHA2012189, BRTHA2012351, BRTHA2012392, BRTHA2012466, BRTHA2012555, BRTHA2012562, BRTHA2012714, BRTHA2012980, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2016918, BRTHA2017178, BRTHA2017353, BRTHA2017364, BRTHA2017972, BRTHA2017985, BRTHA2018011,
BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517,
BRTHA2031800, BRTHA2031917, BRTHA2032763, BRTHA2032845, BRTHA2033122, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000789, BRTHA3001221, BRTHA3001623, BRTHA3002265, BRTHA3002401, BRTHA3002411, BRTHA3002864, BRTHA3002933, BRTHA3002955, BRTHA3003000, BRTHA3003023, BRTHA3003074, BRTHA3003339, BRTHA3003343, BRTHA3003417, BRTHA3003449, BRTHA3003474, BRTHA3003704, BRTHA3004432, BRTHA3004475, BRTHA3004502, BRTHA3004610,
BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3008143, BRTHA3008293, BRTHA3008310, BRTHA3008386, BRTHA3008520, BRTHA3008608, BRTHA3008694, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835,
BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152,
CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140,
CORDB2000061, CORDB2000127, CORDB2000541, CTONG1000009, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165, CTONG1000175, CTONG1000180, CTONG1000181, CTONG1000220, CTONG1000277, CTONG1000283, CTONG1000302, CTONG1000341, CTONG1000467, CTONG1000476, CTONG1000488, CTONG1000508, CTONG1000540, CTONG2000031, CTONG2000042, CTONG2000201, CTONG2000218, CTONG2000279, CTONG2000303, CTONG2000330, CTONG2000395, CTONG2000411, CTONG2000465, CTONG2000480, CTONG2000561, CTONG2000589, CTONG2000766, CTONG2000771, CTONG2000819, CTONG2000827, CTONG2000846, CTONG2000855, CTONG2000977, CTONG2001139,
CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001858, CTONG2001877, CTONG2001955, CTONG2002066, CTONG2002143, CTONG2002395, CTONG2002417, CTONG2002418, CTONG2002453, CTONG2002520, CTONG2002558, CTONG2002590, CTONG2002721, CTONG2002766, CTONG2002816, CTONG2002965, CTONG2002999, CTONG2003028, CTONG2003100, CTONG2003115, CTONG2003189, CTONG2003245, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945,
CTONG2004000, CTONG2004062, CTONG2004115, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2005913, CTONG2006129, CTONG2006223, CTONG2006273, CTONG2006310, CTONG2006377, CTONG2006393, CTONG2006515, CTONG2006524, CTONG2006568, CTONG2006611, CTONG2006620, CTONG2006666, CTONG2006691, CTONG2006798, CTONG2006836, CTONG2006932, CTONG2006942, CTONG2006964, CTONG2007007,
CTONG2007009, CTONG2007072, CTONG2007091, CTONG2007104, CTONG2007168, CTONG2007175, CTONG2007259, CTONG2007272, CTONG2007293, CTONG2007297, CTONG2007399, CTONG2007417, CTONG2007441, CTONG2007474, CTONG2007613, CTONG2007623, CTONG2007681, CTONG2007776, CTONG2007779, CTONG2007898, CTONG2007970, CTONG2008014, CTONG2008184, CTONG2008262, CTONG2008269, CTONG2008343, CTONG2008402, CTONG2008405, CTONG2008521, CTONG2008562, CTONG2008577, CTONG2008595, CTONG2008621, CTONG2008689, CTONG2008720, CTONG2008721, CTONG2008773, CTONG2008989, CTONG2009033, CTONG2009132, CTONG2009156, CTONG2009181, CTONG2009257, CTONG2009258, CTONG2009268, CTONG2009270, CTONG2009395, CTONG2009423, CTONG2009440, CTONG2009527,
CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012001, CTONG2012029, CTONG2012050, CTONG2012077, CTONG2012101, CTONG2012123,
CTONG2012158, CTONG2012230, CTONG2012401, CTONG2012422, CTONG2012425, CTONG2012447, CTONG2012473, CTONG2012476, CTONG2012533, CTONG2012554, CTONG2012724, CTONG2012738, CTONG2012745, CTONG2012758, CTONG2012847, CTONG2012901, CTONG2013128, CTONG2013149, CTONG2013156, CTONG2013178, CTONG2013222, CTONG2013284, CTONG2013339, CTONG2013348, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014389, CTONG2014491, CTONG2014630, CTONG2014635, CTONG2014646, CTONG2014705, CTONG2014946, CTONG2014954, CTONG2014959, CTONG2014966, CTONG2014995, CTONG2015204,
CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016846, CTONG2016869, CTONG2016904, CTONG2016942, CTONG2016953, CTONG2017021, CTONG2017094, CTONG2017292, CTONG2017409, CTONG2017429, CTONG2017458, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018217, CTONG2018279,
CTONG2018334, CTONG2018343, CTONG2018379, CTONG2018413, CTONG2018483, CTONG2018629, CTONG2018652, CTONG2018653, CTONG2018655, CTONG2018735, CTONG2018843, CTONG2018900, CTONG2018976, CTONG2019029, CTONG2019063, CTONG2019144, CTONG2019232, CTONG2019248, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020445, CTONG2020522, CTONG2020582, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024031, CTONG2024206, CTONG2024614, CTONG2024749, CTONG2025496,
CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002909, CTONG3002947, CTONG3002983, CTONG3003165, CTONG3003179, CTONG3003483, CTONG3003553,
CTONG3003598, CTONG3003652, CTONG3003654, CTONG3003669, CTONG3003737, CTONG3003972, CTONG3004072, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073, CTONG3006186; CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST1000066, D3OST1000109, D3OST1000238, D3OST2000184, D3OST2000618, D3OST2000654, D3OST2000734, D3OST2001328,
D3OST2001669, D3OST2001670, D3OST2001788, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000507, D9OST2000031, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073, D9OST2004417, DFNES1000003, DFNES1000083, DFNES1000107,
DFNES1000150, DFNES1000185, DFNES2000011, DFNES2000143, DFNES2000212, DFNES2000268, DFNES2000292, DFNES2000426, DFNES2000432, DFNES2000457, DFNES2000471, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2003742, DFNES2004354, DFNES2004383, DFNES2004684, DFNES2005266, DFNES2005527, DFNES2005540, DFNES2005779, DFNES2005875, DFNES2006944, DFNES2007113, DFNES2007299, DFNES2007332, DFNES2007641, DFNES2007757, DFNES2008280, DFNES2008881, DFNES2010502, DFNES2011192,
DFNES2011221, DFNES2011239, DFNES2011245, DFNES2011380, ERLTF1000021, ERLTF2000324, ERLTF2000465, FCBBF1000023, FCBBF1000025, FCBBF1000069, FCBBF1000117, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000297, FCBBF1000314, FCBBF1000322, FCBBF1000349, FCBBF1000367, FCBBF1000374, FCBBF1000376, FCBBF1000395, FCBBF1000397, FCBBF1000425, FCBBF1000437, FCBBF1000506, FCBBF1000546, FCBBF1000550, FCBBF1000581, FCBBF1000582, FCBBF1000598, FCBBF1000604, FCBBF1000618, FCBBF1000627, FCBBF1000684, FCBBF1000686, FCBBF1000691,
FCBBF1000748, FCBBF2000087, FCBBF2000094, FCBBF2000105, FCBBF2000123, FCBBF2000195, FCBBF2000214, FCBBF2000232, FCBBF2000276, FCBBF2000291, FCBBF2000379, FCBBF2000473, FCBBF2000502, FCBBF2000576, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001088, FCBBF2001116, FCBBF2001188, FCBBF2001238, FCBBF2001243, FCBBF2001299, FCBBF2001400, FCBBF2001427, FCBBF2001480, FCBBF2001529, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002928,
FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004090, FCBBF2004138, FCBBF2004217, FCBBF2004256, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2006131, FCBBF2006380, FCBBF2006418, FCBBF2006452, FCBBF2006634, FCBBF2007012, FCBBF2007186, FCBBF2007265, FCBBF2007610, FCBBF2007633, FCBBF3000120, FCBBF3000175, FCBBF3000227, FCBBF3000228, FCBBF3000229, FCBBF3000233, FCBBF3000269, FCBBF3000361, FCBBF3000367, FCBBF3000462,
FCBBF3000473, FCBBF3000501, FCBBF3000518, FCBBF3000536, FCBBF3000550, FCBBF3000847, FCBBF3001018, FCBBF3001020, FCBBF3001235, FCBBF3001302, FCBBF3001470, FCBBF3001594, FCBBF3001912, FCBBF3001914, FCBBF3001918, FCBBF3001924, FCBBF3002163, FCBBF3002188, FCBBF3002190, FCBBF3002268, FCBBF3002475, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3004021, FCBBF3004041, FCBBF3004390, FCBBF3004502, FCBBF3004842, FCBBF3004847, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3007077,
FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007860, FCBBF3008073, FCBBF3008153; FCBBF3008159, FCBBF3008251, FCBBF3008311, FCBBF3008362, FCBBF3008475, FCBBF3008556, FCBBF3008689, FCBBF3008748, FCBBF3008870, FCBBF3008938, FCBBF3008944, FCBBF3009101, FCBBF3009256, FCBBF3009317, FCBBF3009428, FCBBF3009464, FCBBF3009526, FCBBF3009717, FCBBF3009888, FCBBF3009949, FCBBF3009978, FCBBF3010008, FCBBF3010124, FCBBF3010130, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733,
FCBBF3011003, FCBBF3011418, FCBBF3011523, FCBBF3011570, FCBBF3011592, FCBBF3011867, FCBBF3011889, FCBBF3012170, FCBBF3012182, FCBBF3012288, FCBBF3012332, FCBBF3012588, FCBBF3012842, FCBBF3012901, FCBBF3013041, FCBBF3013058, FCBBF3013205, FCBBF3013266, FCBBF3013307, FCBBF3013341, FCBBF3013506, FCBBF3013623, FCBBF3013800, FCBBF3013846, FCBBF3014229, FCBBF3014355, FCBBF3014567, FCBBF3015119, FCBBF3015131, FCBBF3015317, FCBBF3015727, FCBBF3015758, FCBBF3015809, FCBBF3015870, FCBBF3016018, FCBBF3016178, FCBBF3016618, FCBBF3016622, FCBBF3016644, FCBBF3016987, FCBBF3017059, FCBBF3017071, FCBBF3017123, FCBBF3017233, FCBBF3017255, FCBBF3017531, FCBBF3017535, FCBBF3017681, FCBBF3017729, FCBBF3017873,
FCBBF3017974, FCBBF3018067, FCBBF3018173, FCBBF3018591, FCBBF3018753, FCBBF3018796, FCBBF3019085, FCBBF3019320, FCBBF3019564, FCBBF3019569, FCBBF3019570, FCBBF3019663, FCBBF3019716, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022566, FCBBF3022765, FCBBF3022767, FCBBF3022894, FCBBF3023061, FCBBF3023078, FCBBF3023372, FCBBF3023443, FCBBF3023599, FCBBF3023704, FCBBF3023895, FCBBF3024096, FCBBF3024225, FCBBF3024266, FCBBF3024364, FCBBF3024793,
FCBBF3024911, FCBBF3025098, FCBBF3025252, FCBBF3025280, FCBBF3025285, FCBBF3025417, FCBBF3025568, FCBBF3025650, FCBBF3025674, FCBBF3025905, FCBBF3026021, FCBBF3026251, FCBBF3026308, FCBBF3026678, FCBBF3026692, FCBBF3027104, FCBBF3027199, FCBBF3027328, FCBBF3027559, FCBBF3027717, FCBBF3027755, FCBBF3027768, FCBBF3027863, FCBBF3027903, FCBBF3028102, FCBBF3028143, FCBBF3028188, FCBBF3028202, FCBBF3028472, FCBBF3028528, FCBBF3028582, FCBBF3028671, FCBBF3028794, FCBBF4000061, FCBBF4000076, FCBBF4000173, FCBBF4000192, FCBBF4000268, FCBBF4000282, FCBBF4000399, FCBBF4000415, FCBBF4000500, FCBBF4000529, FCBBF4000548, FCBBF5000041, FCBBF5000261, FCBBF5000325, FCBBF5000353, FCBBF5000384, FCBBF5000483,
FCBBF5000495, FEBRA1000005, FEBRA1000022, FEBRA1000138, FEBRA1000148, FEBRA1000149, FEBRA1000183, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000282, FEBRA2000286, FEBRA2000311, FEBRA2000321, FEBRA2000333, FEBRA2000378, FEBRA2000391, FEBRA2000394, FEBRA2000399, FEBRA2000402, FEBRA2000404, FEBRA2000409, FEBRA2000415, FEBRA2000436, FEBRA2000438, FEBRA2000454, FEBRA2000468, FEBRA2000491, FEBRA2000504, FEBRA2000510, FEBRA2000536, FEBRA2000538, FEBRA2000575, FEBRA2000656, FEBRA2000659, FEBRA2000680, FEBRA2000690, FEBRA2000727, FEBRA2000734, FEBRA2000762, FEBRA2000771, FEBRA2000772, FEBRA2000782,
FEBRA2000790, FEBRA2000803, FEBRA2000805, FEBRA2000809, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2001004, FEBRA2001012, FEBRA2001020, FEBRA2001093, FEBRA2001124, FEBRA2001133, FEBRA2001161, FEBRA2001175, FEBRA2001217, FEBRA2001267, FEBRA2001294, FEBRA2001334, FEBRA2001351, FEBRA2001383, FEBRA2001438, FEBRA2001440, FEBRA2001487, FEBRA2001523, FEBRA2001561, FEBRA2001584, FEBRA2001590, FEBRA2001591, FEBRA2001610, FEBRA2001620, FEBRA2001659, FEBRA2001698, FEBRA2001705, FEBRA2001706, FEBRA2001752, FEBRA2001772, FEBRA2001790, FEBRA2001914, FEBRA2001974, FEBRA2001989, FEBRA2002009,
FEBRA2002086, FEBRA2002109, FEBRA2002191, FEBRA2002260, FEBRA2002399, FEBRA2002429, FEBRA2002442, FEBRA2002508, FEBRA2002527, FEBRA2002552, FEBRA2002574, FEBRA2002601, FEBRA2002611, FEBRA2002628, FEBRA2002662, FEBRA2002727, FEBRA2002783, FEBRA2002818, FEBRA2002858, FEBRA2002882, FEBRA2002897, FEBRA2002908, FEBRA2002962, FEBRA2002986, FEBRA2003054, FEBRA2003115, FEBRA2003155, FEBRA2003163, FEBRA2003181, FEBRA2003253, FEBRA2003258, FEBRA2003275, FEBRA2003308, FEBRA2003327, FEBRA2003429, FEBRA2003436, FEBRA2003454, FEBRA2003468, FEBRA2003520, FEBRA2003524, FEBRA2003675, FEBRA2003707, FEBRA2003711, FEBRA2003726, FEBRA2003822, FEBRA2003897, FEBRA2003907, FEBRA2004023, FEBRA2004026, FEBRA2004029,
FEBRA2004053, FEBRA2004056, FEBRA2004091, FEBRA2004202, FEBRA2004219, FEBRA2004237, FEBRA2004268, FEBRA2004329, FEBRA2004331, FEBRA2004443, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005216, FEBRA2005291, FEBRA2005377, FEBRA2005380, FEBRA2005427, FEBRA2005458, FEBRA2005701, FEBRA2005726, FEBRA2005752, FEBRA2005787, FEBRA2005788, FEBRA2005998, FEBRA2006055, FEBRA2006061, FEBRA2006150, FEBRA2006165, FEBRA2006315, FEBRA2006346, FEBRA2006354, FEBRA2006370, FEBRA2006379, FEBRA2006409,
FEBRA2006418, FEBRA2006427, FEBRA2006476, FEBRA2006491, FEBRA2006519, FEBRA2006664, FEBRA2006667, FEBRA2006726, FEBRA2006736, FEBRA2006793, FEBRA2006817, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007534, FEBRA2007551, FEBRA2007566, FEBRA2007589, FEBRA2007613, FEBRA2007620, FEBRA2007708, FEBRA2007818, FEBRA2007823, FEBRA2007931, FEBRA2007957, FEBRA2008009, FEBRA2008086, FEBRA2008134, FEBRA2008135, FEBRA2008159, FEBRA2008165, FEBRA2008172, FEBRA2008255, FEBRA2008265, FEBRA2008266, FEBRA2008282, FEBRA2008311, FEBRA2008341, FEBRA2008459, FEBRA2008468, FEBRA2008475,
FEBRA2008481, FEBRA2008542, FEBRA2008660, FEBRA2008681, FEBRA2008692, FEBRA2008741, FEBRA2008755, FEBRA2008836, FEBRA2008859, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009541, FEBRA2009567, FEBRA2009731, FEBRA2009754, FEBRA2009780, FEBRA2010141, FEBRA2010299, FEBRA2010484, FEBRA2010768, FEBRA2010802, FEBRA2011063, FEBRA2011356, FEBRA2011392, FEBRA2011414, FEBRA2011593, FEBRA2011665, FEBRA2012090,
FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014010, FEBRA2014198, FEBRA2014213, FEBRA2014297, FEBRA2014443, FEBRA2015076, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2018051, FEBRA2018433, FEBRA2018746, FEBRA2019172, FEBRA2019298, FEBRA2019389, FEBRA2019457, FEBRA2019519,
FEBRA2019582, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2022013, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023399, FEBRA2023498, FEBRA2023550, FEBRA2023721, FEBRA2023927, FEBRA2023989, FEBRA2023990, FEBRA2024136, FEBRA2024150, FEBRA2024343, FEBRA2024570,
FEBRA2024693, FEBRA2024744, FEBRA2024797, FEBRA2024987, FEBRA2025249, FEBRA2025463, FEBRA2025477, FEBRA2026474, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEHRT2000325, FEHRT2000364, FEKID1000018, FELIV1000101, FELIV1000137, FELIV1000164, FELNG2000241, HCASM1000021, HCASM1000050, HCASM1000061, HCASM1000130, HCASM2000156, HCASM2000202, HCASM2000266, HCASM2000307, HCASM2000363, HCASM2000534, HCASM2000536, HCASM2000560, HCASM2000871, HCASM2001285, HCASM2001810, HCASM2001866, HCASM2001890, HCASM2002148, HCASM2002502,
HCASM2002754, HCASM2002918, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000142, HCHON1000166, HCHON1000176, HCHON2000038, HCHON2000062, HCHON2000087, HCHON2000100, HCHON2000156, HCHON2000199, HCHON2000271, HCHON2000315, HCHON2000322, HCHON2000344, HCHON2000364, HCHON2000395, HCHON2000417, HCHON2000418, HCHON2000503, HCHON2000626, HCHON2000660, HCHON2000663, HCHON2000673, HCHON2000699, HCHON2000705, HCHON2000817,
HCHON2000818, HCHON2000819, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001214, HCHON2001233, HCHON2001269, HCHON2001434, HCHON2001450, HCHON2001453, HCHON2001497, HCHON2001505, HCHON2001519, HCHON2001523, HCHON2001535, HCHON2001546, HCHON2001577, HCHON2001598, HCHON2001604, HCHON2001607, HCHON2001646, HCHON2001712, HCHON2001768, HCHON2001801, HCHON2001853, HCHON2002123, HCHON2002194, HCHON2002241, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659,
HCHON2003676, HCHON2004002, HCHON2004007, HCHON2004279, HCHON2004359, HCHON2004531, HCHON2004776, HCHON2005048, HCHON2005118, HCHON2005166, HCHON2005802, HCHON2005987, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HEART1000010, HEART1000088, HEART1000099, HEART1000102, HEART1000139, HEART1000151, HEART2000024, HEART2000035, HEART2000099, HEART2000298, HEART2000306, HEART2000309, HEART2000418,
HEART2000444, HEART2000448, HEART2000492, HEART2000506, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007443, HEART2007767, HEART2008108, HEART2008111, HEART2008257,
HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000070, HHDPC2000115, HHDPC2000149, HHDPC2000258, HHDPC2000315, HHDPC2000656, HHDPC2000692, HHDPC2001337, HHDPC2001432, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HLUNG1000017, HLUNG1000024,
HLUNG1000030, HLUNG1000034, HLUNG1000053, HLUNG1000064, HLUNG1000076, HLUNG1000084, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000751, HLUNG2000761, HLUNG2000777, HLUNG2000870, HLUNG2000884, HLUNG2000926, HLUNG2000950, HLUNG2000955, HLUNG2000970, HLUNG2001013, HLUNG2001126,
HLUNG2001144, HLUNG2001214, HLUNG2001439, HLUNG2001459, HLUNG2001507, HLUNG2001814, HLUNG2001996, HLUNG2002006, HLUNG2002050, HLUNG2002059, HLUNG2002085, HLUNG2002145, HLUNG2002295, HLUNG2002303, HLUNG2002405, HLUNG2002429, HLUNG2002465, HLUNG2002562, HLUNG2002648, HLUNG2002659, HLUNG2002707, HLUNG2002811, HLUNG2002916, HLUNG2002942, HLUNG2002949, HLUNG2002958, HLUNG2002982, HLUNG2003003, HLUNG2003049, HLUNG2003061, HLUNG2003162, HLUNG2003246, HLUNG2003331, HLUNG2003335, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004154, HLUNG2004217, HLUNG2004273, HLUNG2004416,
HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006852, HLUNG2006935, HLUNG2007041, HLUNG2007163, HLUNG2007219, HLUNG2007245, HLUNG2007433, HLUNG2007931, HLUNG2008066, HLUNG2008139, HLUNG2008225, HLUNG2008235, HLUNG2008247, HLUNG2008333, HLUNG2008384, HLUNG2008396, HLUNG2008439, HLUNG2008479, HLUNG2008480, HLUNG2008521, HLUNG2008637,
HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029,
HLUNG2018282, HLUNG2018626, HLUNG2019058, HSYRA1000131, HSYRA2000044, HSYRA2000237, HSYRA2000253, HSYRA2000371, HSYRA2000424, HSYRA2000507, HSYRA2000510, HSYRA2000629, HSYRA2000640, HSYRA2000706, HSYRA2000760, HSYRA2000787, HSYRA2000792, HSYRA2000832, HSYRA2000927, HSYRA2001003, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001580, HSYRA2001631, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714,
HSYRA2009102, IMR321000155, IMR321000161, IMR321000165, IMR321000193, IMR321000230, IMR321000253, IMR322000010, IMR322000107, IMR322000152, IMR322000164, IMR322000178, IMR322000374, IMR322000379, IMR322000383, IMR322000418, IMR322000518, IMR322000604, IMR322000609, IMR322000656, IMR322000672, IMR322000742, IMR322000811, IMR322000819, IMR322000838, IMR322000859, IMR322000863, IMR322000918, IMR322000919, IMR322000953, IMR322000984, IMR322001018, IMR322001049, IMR322001167, IMR322001185, IMR322001218, IMR322001245, IMR322001318, IMR322001322, IMR322001438, IMR322001452, IMR322001491, IMR322001534, IMR322001584, IMR322001665, IMR322001724, IMR322002035, IMR322002110, IMR322002470, IMR322003675,
IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322013053, IMR322013396, IMR322013731, IMR322013805, IMR322015523, IMR322016146, IMR322017049, IMR322018117, IMR322018192, IMR322019070, JCMLC2000273, KIDNE1000008, KIDNE1000012, KIDNE1000015, KIDNE1000028, KIDNE1000036, KIDNE1000064, KIDNE1000104, KIDNE1000121, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245,
KIDNE2000266, KIDNE2000287, KIDNE2000315, KIDNE2000317, KIDNE2000328, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000624, KIDNE2000678, KIDNE2000717, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001713, KIDNE2001802, KIDNE2001840, KIDNE2001847, KIDNE2001857, KIDNE2001873, KIDNE2001897, KIDNE2001931, KIDNE2002015, KIDNE2002162, KIDNE2002168, KIDNE2002198, KIDNE2002262, KIDNE2002438, KIDNE2002483,
KIDNE2002725, KIDNE2002768, KIDNE2002795, KIDNE2002798, KIDNE2002839, KIDNE2002845, KIDNE2002872, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411, KIDNE2004519, KIDNE2004520, KIDNE2004531, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985,
KIDNE2004999, KIDNE2005038, KIDNE2005062, KIDNE2005266, KIDNE2005296, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006580, KIDNE2006652, KIDNE2006687, KIDNE2006733, KIDNE2006760, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910,
KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008975, KIDNE2009109, KIDNE2009180, KIDNE2009191, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010574, KIDNE2010674, KIDNE2010739,
KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012291, KIDNE2012361, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415,
KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018254, KIDNE2018268, KIDNE2018269,
KIDNE2018287, KIDNE2018294, KIDNE2018352, KIDNE2018462, KIDNE2018493, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111, LIVER1000126, LIVER1000132, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000416, LIVER2000446, LIVER2000515,
LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2002842, LIVER2003008, LIVER2003065, LIVER2003234, LIVER2003511, LIVER2003524, LIVER2003568, LIVER2003581, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006251, LIVER2006410,
LIVER2006469, LIVER2006644, LIVER2006764, LIVER2007415, LIVER2007548, LIVER2007568, LIVER2007721, LIVER2007783, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000178, MAMGL1000182, MAMGL1000184, MESAN1000032, MESAN1000035, MESAN1000079, MESAN1000080, MESAN1000121, MESAN1000180, MESAN2000023, MESAN2000026, MESAN2000067, MESAN2000092, MESAN2000167, MESAN2000264, MESAN2000291, MESAN2000337, MESAN2000434, MESAN2000462, MESAN2000620, MESAN2000711, MESAN2000815, MESAN2000894,
MESAN2000909, MESAN2001154, MESAN2001450, MESAN2001770, MESAN2001979, MESAN2001980, MESAN2002113, MESAN2002147, MESAN2002424, MESAN2002687, MESAN2002709, MESAN2002724, MESAN2002790, MESAN2002978, MESAN2003037, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005724, MESAN2005808, MESAN2005811, MESAN2005957, MESAN2005958, MESAN2006022, MESAN2006328, MESAN2006580, MESAN2006599, MESAN2006971, MESAN2007032, MESAN2007440, MESAN2007597, MESAN2007755, MESAN2008222, MESAN2008415, MESAN2008460, MESAN2008536, MESAN2008679, MESAN2008729,
MESAN2008821, MESAN2009081, MESAN2009105, MESAN2009311, MESAN2009418, MESAN2009503, MESAN2009522, MESAN2009580, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013936, MESAN2014192, MESAN2014295, MESAN2014298, MESAN2014412, MESAN2014624, MESAN2015277, MESAN2015365, MESAN2015401, MESAN2015501, MESAN2015708, MESAN2016159, MESAN2016304, MESAN2016409, MESAN2016965, MESAN2017152, MESAN2017373, MESAN2017417, MESAN2018209, MESAN2018576,
MESAN2018670, MESAN2018699, MESTC1000042, MESTC1000147, MESTC2000150, MESTC2000153, MESTC2000170, N1ESE2000698, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000187, NHNPC2000606,
NHNPC2000877, NHNPC2001108, NHNPC2001223, NHNPC2001816, NHNPC2002565, NHNPC2002749, NOVAR1000015, NOVAR1000102, NOVAR2000038, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000068, PEBLM1000071, PEBLM1000083, PEBLM1000147, PEBLM1000174, PEBLM1000180, PEBLM2000030, PEBLM2000112, PEBLM2000126, PEBLM2000170, PEBLM2000180, PEBLM2000213, PEBLM2000248, PEBLM2000267, PEBLM2000270, PEBLM2000321, PEBLM2000333, PEBLM2000338, PEBLM2000479, PEBLM2000502, PEBLM2001312,
PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006912, PEBLM2007112, PEBLM2007140, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386,
PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086,
PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000170, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000296, PLACE6000314, PLACE6000325, PLACE6000348, PLACE6000371, PLACE6000414, PLACE6000424, PLACE6000426, PLACE6000429, PLACE6000450, PLACE6000463, PLACE6000523, PLACE6000550,
PLACE6000552, PLACE6000555, PLACE6000619, PLACE6000630, PLACE6000953, PLACE6001118, PLACE6001153, PLACE6001185, PLACE6001262, PLACE6001294, PLACE6001536, PLACE6001557, PLACE6001712, PLACE6001740, PLACE6001823, PLACE6001886, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003218, PLACE6003372, PLACE6003399, PLACE6003539, PLACE6003621, PLACE6003705, PLACE6003740, PLACE6003771, PLACE6003796, PLACE6003839,
PLACE6003850, PLACE6004101, PLACE6004219, PLACE6004336, PLACE6004368, PLACE6004380, PLACE6004396, PLACE6004454, PLACE6004464, PLACE6004491, PLACE6004578, PLACE6004663, PLACE6004687, PLACE6004738, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006186, PLACE6006217, PLACE6006222, PLACE6006266, PLACE6006287, PLACE6006394, PLACE6006418, PLACE6006474, PLACE6006549, PLACE6006566, PLACE6006697, PLACE6006822, PLACE6006871, PLACE6006905, PLACE6007050,
PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008775, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010077, PLACE6010463, PLACE6010485, PLACE6010568, PLACE6010701, PLACE6010704, PLACE6010732, PLACE6010765, PLACE6010848,
PLACE6010925, PLACE6010936, PLACE6010991, PLACE6011102, PLACE6011156, PLACE6011211, PLACE6011260, PLACE6011774, PLACE6011881, PLACE6011907, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016254, PLACE6016383,
PLACE6016942, PLACE6016984, PLACE6017002, PLACE6017431, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6019195, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004961,
PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000120, PROST1000123, PROST1000136, PROST1000145, PROST1000152, PROST1000167, PROST1000184, PROST1000217, PROST1000246, PROST1000262, PROST1000272, PROST1000298, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527,
PROST1000528, PROST1000536, PROST1000559, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000277, PROST2000337, PROST2000432, PROST2000452, PROST2000463, PROST2000470, PROST2000505, PROST2000567, PROST2000572, PROST2000717, PROST2000761, PROST2000835, PROST2000893, PROST2000961, PROST2001002, PROST2001116, PROST2001138, PROST2001283, PROST2001414, PROST2001415, PROST2001465, PROST2001521, PROST2001540, PROST2001599, PROST2001611, PROST2001636, PROST2001739, PROST2001796, PROST2001805, PROST2001823, PROST2001883, PROST2001899,
PROST2001997, PROST2001998, PROST2002078, PROST2002101, PROST2002162, PROST2002212, PROST2002338, PROST2002425, PROST2002488, PROST2002489, PROST2002527, PROST2002591, PROST2002602, PROST2002633, PROST2002682, PROST2002685, PROST2002722, PROST2002736, PROST2002842, PROST2002897, PROST2002906, PROST2002927, PROST2003102, PROST2003117, PROST2003210, PROST2003302, PROST2003303, PROST2003324, PROST2003340, PROST2003428, PROST2003472, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251,
PROST2004332, PROST2004481, PROST2004526, PROST2004727, PROST2004742, PROST2004744, PROST2004817, PROST2005039, PROST2005067, PROST2005076, PROST2005093, PROST2005121, PROST2005131, PROST2005228, PROST2005285, PROST2005426, PROST2005466, PROST2005604, PROST2005630, PROST2005683, PROST2005788, PROST2005880, PROST2005886, PROST2005904, PROST2005919, PROST2005943, PROST2006008, PROST2006020, PROST2006060, PROST2006196, PROST2006201, PROST2006282, PROST2006450, PROST2006491, PROST2006510, PROST2006579, PROST2006688, PROST2006737, PROST2006766, PROST2006782, PROST2006933, PROST2006988, PROST2007122, PROST2007237, PROST2007248, PROST2007289, PROST2007317, PROST2007328, PROST2007382, PROST2007389,
PROST2007416, PROST2007528, PROST2007612, PROST2007818, PROST2007950, PROST2007956, PROST2007974, PROST2007988, PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010579, PROST2010606, PROST2010885, PROST2011012,
PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015198, PROST2015243, PROST2015246, PROST2015251, PROST2015287, PROST2015457, PROST2015464, PROST2015537, PROST2015545,
PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017367, PROST2017413, PROST2017441, PROST2017529, PROST2017578, PROST2017612, PROST2017617, PROST2017700, PROST2017701, PROST2017729, PROST2017836, PROST2017972, PROST2018030, PROST2018090, PROST2018235, PROST2018437, PROST2018487, PROST2018511, PROST2018583, PROST2018588, PROST2018788, PROST2018902, PROST2018922, PROST2018977,
PROST2019164, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000175, PUAEN1000275, PUAEN1000276, PUAEN1000288, PUAEN2000080, PUAEN2000152, PUAEN2000175, PUAEN2000247, PUAEN2000312, PUAEN2000394, PUAEN2000535, PUAEN2000594, PUAEN2000684, PUAEN2000942, PUAEN2001260, PUAEN2001345, PUAEN2001526, PUAEN2001586, PUAEN2001882, PUAEN2002473, PUAEN2002552, PUAEN2002568, PUAEN2002616, PUAEN2002758, PUAEN2003018, PUAEN2003079, PUAEN2003116, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588,
PUAEN2005930, PUAEN2006029, PUAEN2006328, PUAEN2006578, PUAEN2006639, PUAEN2006761, PUAEN2007785, PUAEN2007898, PUAEN2008123, PUAEN2008228, PUAEN2008314, PUAEN2008515, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000141, RECTM2000220, RECTM2000349, RECTM2000433, RECTM2001307, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000107, SALGL1000171, SKMUS1000014, SKMUS1000030, SKMUS1000067, SKMUS1000102, SKMUS1000104, SKMUS1000110, SKMUS1000118, SKMUS1000124, SKMUS1000129,
SKMUS1000176, SKMUS1000177, SKMUS2000020, SKMUS2000061, SKMUS2000074, SKMUS2000078, SKMUS2000117, SKMUS2000137, SKMUS2000243, SKMUS2000271, SKMUS2000287, SKMUS2000317, SKMUS2000330, SKMUS2000339, SKMUS2000343, SKMUS2000365, SKMUS2000380, SKMUS2000390, SKMUS2000416, SKMUS2000458, SKMUS2000468, SKMUS2000484, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000902, SKMUS2000933, SKMUS2000945, SKMUS2000954, SKMUS2000961, SKMUS2000973, SKMUS2000980, SKMUS2001008, SKMUS2001014, SKMUS2001129, SKMUS2001157, SKMUS2001164,
SKMUS2001199, SKMUS2001201, SKMUS2001215, SKMUS2001247, SKMUS2001254, SKMUS2001271, SKMUS2001287, SKMUS2001364, SKMUS2001398, SKMUS2001402, SKMUS2001412, SKMUS2001492, SKMUS2001501, SKMUS2001528, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897,
SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000137, SKNMC1000159, SKNMC1000251, SKNMC2000224, SKNMC2000256, SKNMC2000305, SKNMC2000322, SKNMC2000356, SKNMC2000374, SKNMC2000583, SKNMC2000593, SKNMC2000612, SKNMC2000622, SKNMC2000966, SKNMC2001113, SKNMC2001503, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006327, SKNMC2006998,
SKNMC2007134, SKNMC2007502, SKNMC2007504, SKNMC2007961, SKNMC2009450, SKNSH1000174, SKNSH1000301, SKNSH1000308, SKNSH1000416, SKNSH2000151, SKNSH2000163, SKNSH2000319, SKNSH2000482, SKNSH2000516, SKNSH2000550, SKNSH2000716, SKNSH2000971, SKNSH2001222, SKNSH2001875, SKNSH2002054, SKNSH2002768, SKNSH2002866, SKNSH2003064, SKNSH2003174, SKNSH2003422, SKNSH2003466, SKNSH2003483, SKNSH2003528, SKNSH2003633, SKNSH2004039, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008940, SKNSH2008969, SKNSH2009197, SKNSH2009357, SKNSH2009435, SKNSH2009991,
SKNSH2010015, SMINT1000012, SMINT1000016, SMINT1000039, SMINT1000048, SMINT1000051, SMINT1000057, SMINT1000071, SMINT1000075, SMINT1000078, SMINT1000091, SMINT1000100, SMINT1000103, SMINT1000118, SMINT1000137, SMINT1000192, SMINT2000007, SMINT2000032, SMINT2000040, SMINT2000060, SMINT2000084, SMINT2000145, SMINT2000159, SMINT2000185, SMINT2000227, SMINT2000229, SMINT2000239, SMINT2000267, SMINT2000454, SMINT2000518, SMINT2000529, SMINT2000530, SMINT2000538, SMINT2000541, SMINT2000545, SMINT2000558, SMINT2000562, SMINT2000568, SMINT2000602, SMINT2000629, SMINT2000747, SMINT2000811, SMINT2000824, SMINT2000933, SMINT2000984, SMINT2001114, SMINT2001158, SMINT2001183, SMINT2001199, SMINT2001222,
SMINT2001348, SMINT2001397, SMINT2001458, SMINT2001461, SMINT2001535, SMINT2001559, SMINT2001609, SMINT2001683, SMINT2001731, SMINT2001812, SMINT2001818, SMINT2001950, SMINT2002126, SMINT2002159, SMINT2002202, SMINT2002210, SMINT2002281, SMINT2002311, SMINT2002314, SMINT2002328, SMINT2002414, SMINT2002457, SMINT2002689, SMINT2002743, SMINT2002778, SMINT2002880, SMINT2002884, SMINT2002899, SMINT2002974, SMINT2003003, SMINT2003128, SMINT2003150, SMINT2003317, SMINT2003340, SMINT2003386, SMINT2003423, SMINT2003505, SMINT2003569, SMINT2003641, SMINT2003644, SMINT2003866, SMINT2004023, SMINT2004037, SMINT2004047, SMINT2004086, SMINT2004144, SMINT2004280, SMINT2004299, SMINT2004339, SMINT2004350,
SMINT2004414, SMINT2004473, SMINT2004547, SMINT2004583, SMINT2004589, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867,
SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010500, SMINT2010533, SMINT2010629, SMINT2010672, SMINT2010753,
SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012179, SMINT2012195, SMINT2012285, SMINT2012501, SMINT2012734, SMINT2012793, SMINT2013031, SMINT2013032, SMINT2013129, SMINT2013170, SMINT2013181, SMINT2013228, SMINT2013613, SMINT2013626, SMINT2013695, SMINT2013833, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146,
SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064,
SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000243, SPLEN2000247, SPLEN2000255, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000874, SPLEN2001135, SPLEN2001141, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599,
SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002477, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004124, SPLEN2004148, SPLEN2004181, SPLEN2004220, SPLEN2004343, SPLEN2004346, SPLEN2004368, SPLEN2004611,
SPLEN2004662, SPLEN2004844, SPLEN2004880, SPLEN2004984, SPLEN2005227, SPLEN2005272, SPLEN2005342, SPLEN2005416, SPLEN2005429, SPLEN2005450, SPLEN2005474, SPLEN2005560, SPLEN2005727, SPLEN2005790, SPLEN2005806, SPLEN2005818, SPLEN2005838, SPLEN2005869, SPLEN2005927, SPLEN2005939, SPLEN2006232, SPLEN2006268, SPLEN2006283, SPLEN2006389, SPLEN2006425, SPLEN2006671, SPLEN2006701, SPLEN2006785, SPLEN2006875, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007750, SPLEN2007794, SPLEN2007879, SPLEN2007926, SPLEN2007951, SPLEN2008007, SPLEN2008135, SPLEN2008164, SPLEN2008317, SPLEN2008496, SPLEN2008591,
SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733, SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011766,
SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012800, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318,
SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860, SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304,
SPLEN2016356, SPLEN2016421, SPLEN2016480, SPLEN2016531, SPLEN2016533, SPLEN2016541, SPLEN2016552, SPLEN2016554, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683,
SPLEN2018732, SPLEN2018749, SPLEN2018933, SPLEN2019008, SPLEN2019043, SPLEN2019077, SPLEN2019092, SPLEN2019102, SPLEN2019131, SPLEN2019169, SPLEN2019257, SPLEN2019311, SPLEN2019323, SPLEN2019349, SPLEN2019375, SPLEN2019379, SPLEN2019405, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021157, SPLEN2021231, SPLEN2021273, SPLEN2021295, SPLEN2021298,
SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395,
SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333,
SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237,
SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042521, SPLEN2042535, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186, STOMA1000189, STOMA1000190, STOMA1000191, STOMA2000032, STOMA2000072, STOMA2000088, STOMA2000104, STOMA2000148, STOMA2000168, STOMA2000183, STOMA2000188, STOMA2000257, STOMA2000279, STOMA2000383, STOMA2000386, STOMA2000395, STOMA2000478, STOMA2000529, STOMA2000539, STOMA2000567, STOMA2000640, STOMA2000678, STOMA2000686, STOMA2000888, STOMA2001025, STOMA2001389, STOMA2002052,
STOMA2002141, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000128, SYNOV1000164,
SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001212, SYNOV2001239, SYNOV2001251, SYNOV2001262, SYNOV2001300, SYNOV2001356, SYNOV2001390, SYNOV2001451, SYNOV2001457, SYNOV2001468, SYNOV2001581, SYNOV2001648, SYNOV2001660, SYNOV2003326, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233,
SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012,
SYNOV4007215, SYNOV4007360, SYNOV4007430, SYNOV4007521, SYNOV4007553, SYNOV4007711, SYNOV4008245, SYNOV4008336, SYNOV4009139, SYNOV4009575, T1ESE2000116, T1ESE2000609, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133,
TBAES2009387, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002489, TESOP2002539, TESOP2002545, TESOP2002804, TESOP2002950, TESOP2003273, TESOP2003308, TESOP2003553, TESOP2003753, TESOP2004110,
TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000064, TESTI1000070, TESTI1000085, TESTI1000088, TESTI1000093,
TESTI1000094, TESTI1000101, TESTI1000109, TESTI1000125, TESTI1000127, TESTI1000128, TESTI1000131, TESTI1000138, TESTI1000157, TESTI1000163, TESTI1000168, TESTI1000169, TESTI1000179, TESTI1000183, TESTI1000191, TESTI1000257, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000177, TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462,
TESTI2000489, TESTI2000520, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000883, TESTI2000909, TESTI2000951, TESTI2000981, TESTI2001008, TESTI2001049, TESTI2001082, TESTI2001120, TESTI2001134, TESTI2001141, TESTI2001153, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518,
TESTI2001556, TESTI2001593, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001862, TESTI2001879, TESTI2001894, TESTI2001898, TESTI2001915, TESTI2001950, TESTI2001959, TESTI2001968, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002406,
TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003130, TESTI2003131, TESTI2003139, TESTI2003141,
TESTI2003152, TESTI2003181, TESTI2003193, TESTI2003196, TESTI2003228, TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824,
TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004783, TESTI2004791,
TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720,
TESTI2005739, TESTI2005759, TESTI2005767, TESTI2005775, TESTI2005788, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006409, TESTI2006425, TESTI2006437,
TESTI2006453, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405,
TESTI2007407, TESTI2007422, TESTI2007452, TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405,
TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786, TESTI2008822, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009390, TESTI2009400, TESTI2009412, TESTI2009423, TESTI2009447, TESTI2009462, TESTI2009477, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835,
TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010682, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011712, TESTI2011750, TESTI2011840, TESTI2011846, TESTI2011915, TESTI2011928, TESTI2011971, TESTI2012019,
TESTI2012050, TESTI2012090, TESTI2012095, TESTI2012104, TESTI2012152, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545,
TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015092, TESTI2015105, TESTI2015180, TESTI2015249, TESTI2015327, TESTI2015331, TESTI2015335, TESTI2015350, TESTI2015375, TESTI2015437, TESTI2015590,
TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256,
TESTI2017305, TESTI2017311, TESTI2017396, TESTI2017507, TESTI2017537, TESTI2017582, TESTI2017584, TESTI2017727, TESTI2017740, TESTI2017775, TESTI2017816, TESTI2017832, TESTI2017923, TESTI2017932, TESTI2017951, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2018997, TESTI2019042, TESTI2019077, TESTI2019228, TESTI2019257, TESTI2019280, TESTI2019282, TESTI2019308, TESTI2019336,
TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112,
TESTI2021114, TESTI2021116, TESTI2021118, TESTI2021122, TESTI2021138, TESTI2021297, TESTI2021315, TESTI2021358, TESTI2021425, TESTI2021463, TESTI2021531, TESTI2021599, TESTI2021637, TESTI2021654, TESTI2021667, TESTI2021736, TESTI2021785, TESTI2021819, TESTI2021895, TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214,
TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174,
TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026534, TESTI2026537, TESTI2026589, TESTI2026597, TESTI2026605, TESTI2026647, TESTI2026674, TESTI2026794, TESTI2026824, TESTI2026854,
TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776,
TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681,
TESTI2032761, TESTI2032768, TESTI2032774, TESTI2032828, TESTI2033031, TESTI2033242, TESTI2033254, TESTI2033300, TESTI2033395, TESTI2033441, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688,
TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275,
TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664,
TESTI2041730, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155,
TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206,
TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526; TESTI2053561, TESTI2053621, TESTI2053667, TESTI3000002, TESTI3000005, TESTI4000068, TESTI4000079, TESTI4000093, TESTI4000134,
TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001467, TESTI4001473, TESTI4001517, TESTI4001527, TESTI4001561, TESTI4001569, TESTI4001604, TESTI4001665, TESTI4001679, TESTI4001795, TESTI4001923, TESTI4001925, TESTI4001984, TESTI4002003, TESTI4002141,
TESTI4002195, TESTI4002202, TESTI4002290, TESTI4002319, TESTI4002491, TESTI4002520, TESTI4002552, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500, TESTI4005534, TESTI4005543, TESTI4005628, TESTI4005801, TESTI4005805, TESTI4005857,
TESTI4005961, TESTI4006053, TESTI4006079, TESTI4006112, TESTI4006137, TESTI4006148, TESTI4006219, TESTI4006234, TESTI4006308, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4008007, TESTI4008018, TESTI4008050, TESTI4008086, TESTI4008097, TESTI4008176, TESTI4008209, TESTI4008219, TESTI4008250, TESTI4008302, TESTI4008305,
TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505,
TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4013365, TESTI4013369, TESTI4013410, TESTI4013474, TESTI4013602, TESTI4013667, TESTI4013675, TESTI4013685, TESTI4013735, TESTI4013742, TESTI4013774, TESTI4013817, TESTI4013830, TESTI4013852, TESTI4013894, TESTI4013924, TESTI4013960, TESTI4013962, TESTI4014159, TESTI4014265, TESTI4014276, TESTI4014306, TESTI4014392, TESTI4014415, TESTI4014445, TESTI4014553, TESTI4014661,
TESTI4014694, TESTI4014801, TESTI4014818, TESTI4014891, TESTI4014908, TESTI4014924, TESTI4014932,TESTI4014977, TESTI4015012, TESTI4015129, TESTI4015145, TESTI4015204, TESTI4015263, TESTI4015293, TESTI4015299, TESTI4015339, TESTI4015398, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017763, TESTI4017848, TESTI4017854, TESTI4017901, TESTI4017961, TESTI4017984, TESTI4018101,
TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096,
TESTI4024240, TESTI4024245, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809,
TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065,
TESTI4035498, TESTI4035508, TESTI4035581, TESTI4035602, TESTI4035637, TESTI4035649, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939,
TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701,
TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017, THYMU1000025, THYMU1000029, THYMU1000032, THYMU1000033, THYMU1000060, THYMU1000074, THYMU1000098, THYMU1000105, THYMU1000109, THYMU1000136, THYMU1000142, THYMU1000158, THYMU1000176, THYMU1000179, THYMU1000191, THYMU1000198, THYMU1000217, THYMU1000240, THYMU1000242,
THYMU1000291, THYMU1000316, THYMU1000318, THYMU1000331, THYMU1000336, THYMU1000359, THYMU1000366, THYMU1000382, THYMU1000393, THYMU1000394, THYMU1000399, THYMU1000426, THYMU1000440, THYMU1000459, THYMU1000473, THYMU1000490, THYMU1000491, THYMU1000496, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000382, THYMU2000418, THYMU2000440, THYMU2000489, THYMU2000492,
THYMU2000519, THYMU2000570, THYMU2000602, THYMU2000660, THYMU2000671, THYMU2000672, THYMU2000684, THYMU2000767, THYMU2000775, THYMU2000800, THYMU2000879, THYMU2000932, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001422, THYMU2001443, THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001989, THYMU2002023, THYMU2002037, THYMU2002080, THYMU2002109,
THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002815, THYMU2002852, THYMU2002910, THYMU2002950, THYMU2002983, THYMU2003012, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003542, THYMU2003632, THYMU2003650, THYMU2003700, THYMU2003760, THYMU2003803,
THYMU2003855, THYMU2003891, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004410, THYMU2004452, THYMU2004508, THYMU2004512, THYMU2004635, THYMU2004639, THYMU2004668, THYMU2004677, THYMU2004688, THYMU2004693, THYMU2004733, THYMU2004835, THYMU2004843, THYMU2004906, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507,
THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095, THYMU2007146, THYMU2007179, THYMU2007258, THYMU2007307, THYMU2007308, THYMU2007350, THYMU2007384, THYMU2007415, THYMU2007467, THYMU2007493, THYMU2007521, THYMU2007528, THYMU2007530, THYMU2007532,
THYMU2007561, THYMU2007571, THYMU2007635, THYMU2007658, THYMU2007667, THYMU2007725, THYMU2007729, THYMU2007802, THYMU2007824, THYMU2007854, THYMU2007952, THYMU2007969, THYMU2008035, THYMU2008036, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186,
THYMU2009338, THYMU2009425, THYMU2009516, THYMU2009526, THYMU2009546, THYMU2009596, THYMU2009658, THYMU2009792, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666,
THYMU2011736, THYMU2011785, THYMU2011806, THYMU2011852, THYMU2011937, THYMU2011939, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297, THYMU2014323, THYMU2014327, THYMU2014353, THYMU2014492,
THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071,
THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943,
THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2026182, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068,
THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338,
THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634,
THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001991, THYMU3002452, THYMU3002661, THYMU3003007, THYMU3003092, THYMU3003212, THYMU3003350, THYMU3003403,
THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012,
THYMU3011081, THYMU3011244, THYMU3011360, THYMU3011534, THYMU3011543, THYMU3011556, THYMU3011717, THYMU3012402, THYMU3012907, THYMU3012983, THYMU3013114, THYMU3013197, THYMU3013241, THYMU3013386, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869,
THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752,
THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126,
THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000062, TKIDN1000066,
TKIDN1000164, TKIDN1000171, TKIDN2000240, TKIDN2000319, TKIDN2000464, TKIDN2000521, TKIDN2000701, TKIDN2001662, TKIDN2002318, TKIDN2002329, TKIDN2002424, TKIDN2002632, TKIDN2003044, TKIDN2003059, TKIDN2003062, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009794, TKIDN2009889, TKIDN2010200, TKIDN2010232, TKIDN2010278, TKIDN2010602, TKIDN2011051, TKIDN2011160, TKIDN2011324, TKIDN2012034, TKIDN2012771,
TKIDN2012824, TKIDN2013134, TKIDN2014570, TKIDN2014757, TKIDN2014771, TKIDN2015025, TKIDN2015071, TKIDN2015161, TKIDN2015263, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002336, TLIVE2002338, TLIVE2002562, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733,
TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000038, TRACH1000057, TRACH1000063, TRACH1000074, TRACH1000100, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047,
TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000496, TRACH2000497, TRACH2000502, TRACH2000522, TRACH2000540, TRACH2000559, TRACH2000660, TRACH2000665, TRACH2000675, TRACH2000677, TRACH2000703, TRACH2000767, TRACH2000775, TRACH2000807, TRACH2000862, TRACH2000873, TRACH2000894, TRACH2000898, TRACH2000923, TRACH2000944, TRACH2000972, TRACH2000981, TRACH2000986, TRACH2001037, TRACH2001060, TRACH2001085, TRACH2001101, TRACH2001121, TRACH2001147, TRACH2001154, TRACH2001188,
TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001474, TRACH2001523, TRACH2001525, TRACH2001532, TRACH2001549, TRACH2001592, TRACH2001596, TRACH2001621, TRACH2001646, TRACH2001651, TRACH2001684, TRACH2001933, TRACH2001968, TRACH2001996, TRACH2002043, TRACH2002054, TRACH2002092, TRACH2002100, TRACH2002138, TRACH2002333, TRACH2002664, TRACH2002784, TRACH2002840, TRACH2002847, TRACH2002853, TRACH2002862, TRACH2002954, TRACH2002960, TRACH2002988, TRACH2003070, TRACH2003484, TRACH2003516, TRACH2003605, TRACH2003736, TRACH2003759, TRACH2003941,
TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008112, TRACH2008113, TRACH2008127, TRACH2008278, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639,
TRACH2010677, TRACH2010698, TRACH2010824, TRACH2010883, TRACH2010965, TRACH2010974, TRACH2011057, TRACH2011093, TRACH2011113, TRACH2011293, TRACH2011302, TRACH2011500, TRACH2011574, TRACH2011956, TRACH2012000, TRACH2012138, TRACH2012242, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013265, TRACH2013369, TRACH2013450, TRACH2013495, TRACH2013585, TRACH2013671, TRACH2013726, TRACH2013902, TRACH2013928, TRACH2013982, TRACH2013991, TRACH2014018, TRACH2014045, TRACH2014046, TRACH2014077, TRACH2014082, TRACH2014268, TRACH2014284, TRACH2014337, TRACH2014356, TRACH2014371, TRACH2014442,
TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084,
TRACH2018262, TRACH2018273, TRACH2018278, TRACH2018317, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673,TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479,
TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064,
TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3004068, TRACH3004113, TRACH3004288, TRACH3004303, TRACH3004412, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005274, TRACH3005479, TRACH3005549,
TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008201, TRACH3008508, TRACH3008629, TRACH3008632, TRACH3008688, TRACH3008697, TRACH3008887, TRACH3008902, TRACH3008990, TRACH3009001, TRACH3009059, TRACH3009061, TRACH3009148,
TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014415, TRACH3014580, TRACH3014641,
TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928,
TRACH3020930, TRACH3021023, TRACH3021066, TRACH3021212, TRACH3021335, TRACH3021373, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026542, TRACH3026650, TRACH3026676, TRACH3026744, TRACH3026949, TRACH3027229, TRACH3027527, TRACH3027681, TRACH3027701,
TRACH3027885, TRACH3028031, TRACH3028164, TRACH3028180, TRACH3028195, TRACH3028441, TRACH3028597, TRACH3028678, TRACH3028837, TRACH3028847, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488,
TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996,
TSTOM2002265, TSTOM2002505, TSTOM2002561, TSTOM2002611, TSTOM2002672, TSTOM2002682, TUTER1000014, TUTER1000137, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000133, UTERU1000008, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192,
UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000542, UTERU2000546, UTERU2000550, UTERU2000569, UTERU2000607, UTERU2000629, UTERU2000649, UTERU2000663, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747,
UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003456, UTERU2003577, UTERU2003704, UTERU2003926, UTERU2003973, UTERU2004015, UTERU2004037, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2004929, UTERU2005004, UTERU2005050, UTERU2005069, UTERU2005074, UTERU2005179, UTERU2005292, UTERU2005346, UTERU2005446,
UTERU2005449, UTERU2005450, UTERU2005533, UTERU2005593, UTERU2005601, UTERU2005621, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008705, UTERU2008707, UTERU2008747, UTERU2008785,
UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012252,
UTERU2012333, UTERU2012407, UTERU2012581, UTERU2012610, UTERU2012615, UTERU2012688, UTERU2012703, UTERU2012715, UTERU2012741, UTERU2012786, UTERU2012856, UTERU2012890, UTERU2012976, UTERU2013048, UTERU2013078, UTERU2013231, UTERU2013280, UTERU2013322, UTERU2013483, UTERU2013502, UTERU2013926, UTERU2013976, UTERU2014001, UTERU2014024, UTERU2014223, UTERU2014398, UTERU2014464, UTERU2014548, UTERU2014601, UTERU2014631, UTERU2014668, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880, UTERU2016147, UTERU2016157, UTERU2016464, UTERU2016757, UTERU2016761, UTERU2016822, UTERU2016896,
UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019038, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491,
UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2029503, UTERU2029660, UTERU2029742, UTERU2029953, UTERU2030213, UTERU2030270,
UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791,
UTERU2037843, UTERU2038171, UTERU2038251, UTERU3000226, UTERU3000298, UTERU3000402, UTERU3000645, UTERU3000665, UTERU3000670, UTERU3000738, UTERU3000828, UTERU3000844, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003774, UTERU3003776, UTERU3004384, UTERU3004477, UTERU3004523, UTERU3004616,
UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010526, UTERU3010604,
UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255, UTERU3018616,
UTERU3018711, UTERU3019078

Of the 17,176 genes that include the cDNAs of the present invention, genes whose expression levels were high enough to detect in each of the PANCR, PEBLM, PERIC, PLACE, PROST, PUAEN, and RECTM libraries were analyzed for expression frequency. The analysis results are described below. Each is a relative value, where the expression level over all tissues is taken as 100. The larger this value, the greater the level of expression.

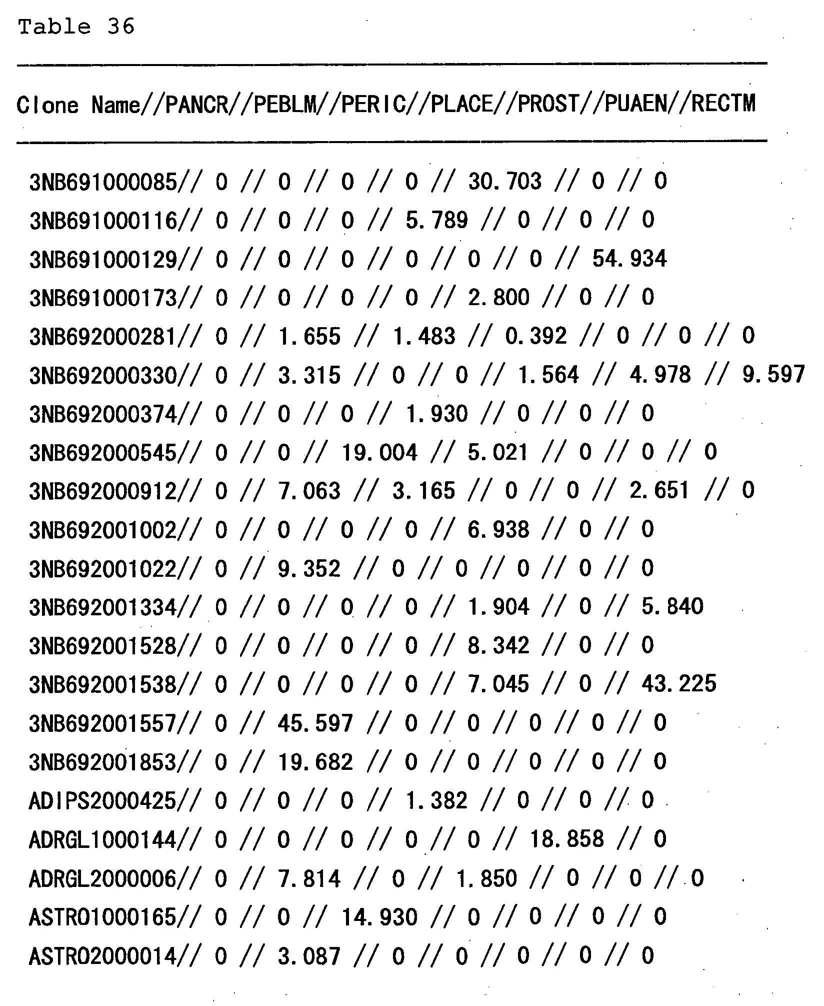

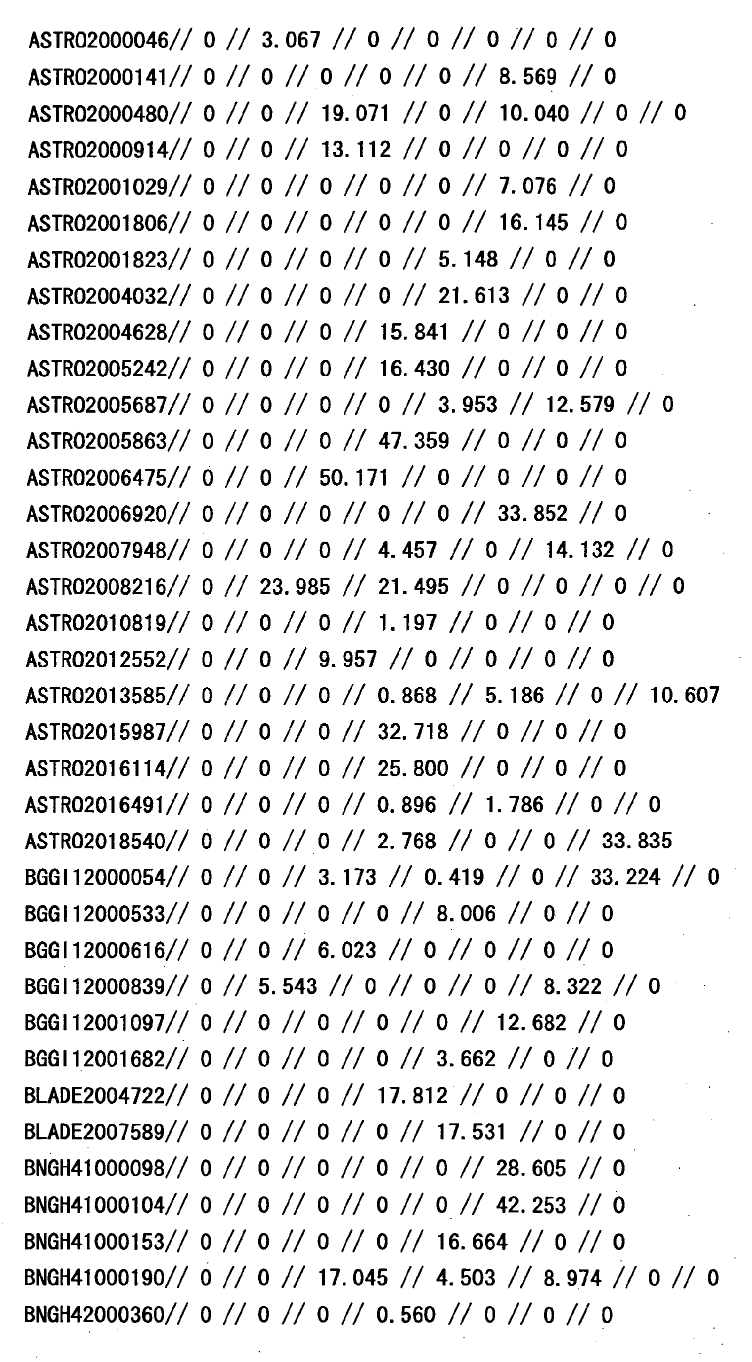

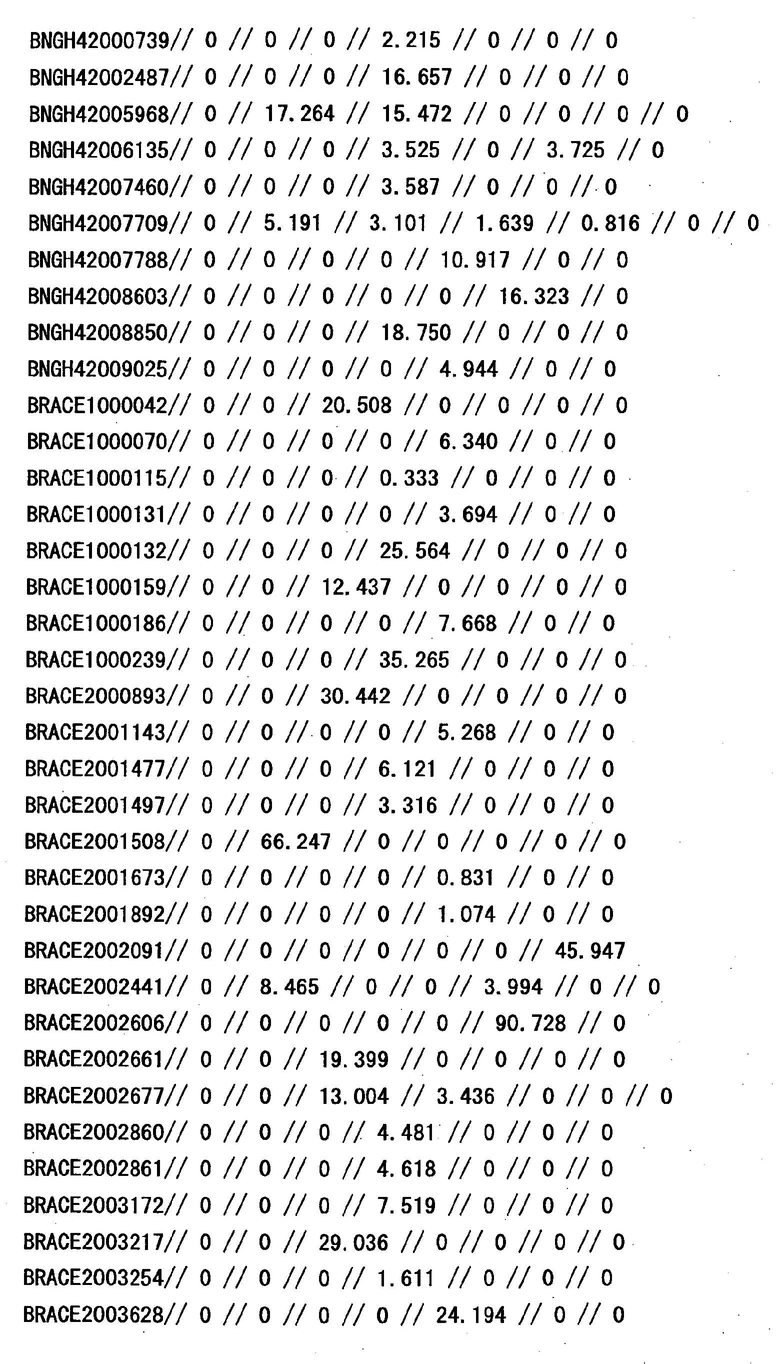

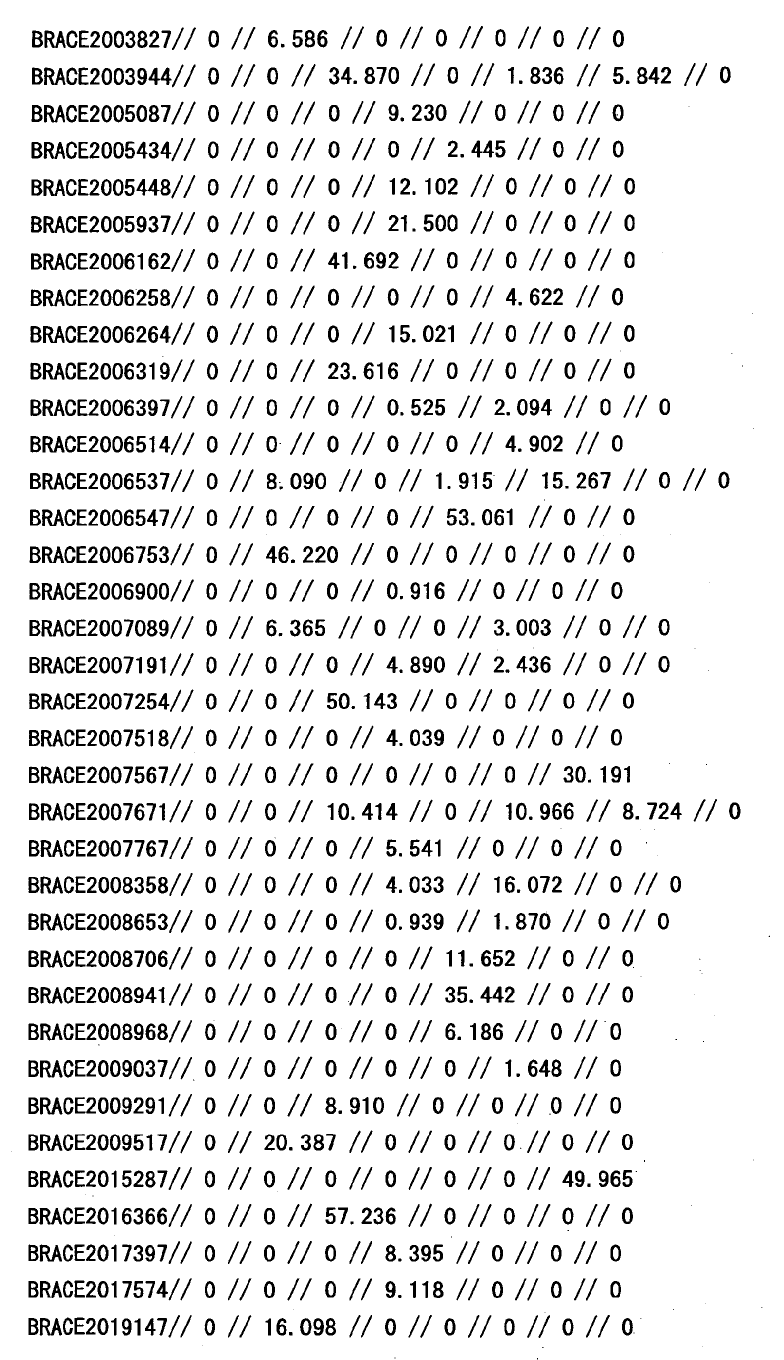

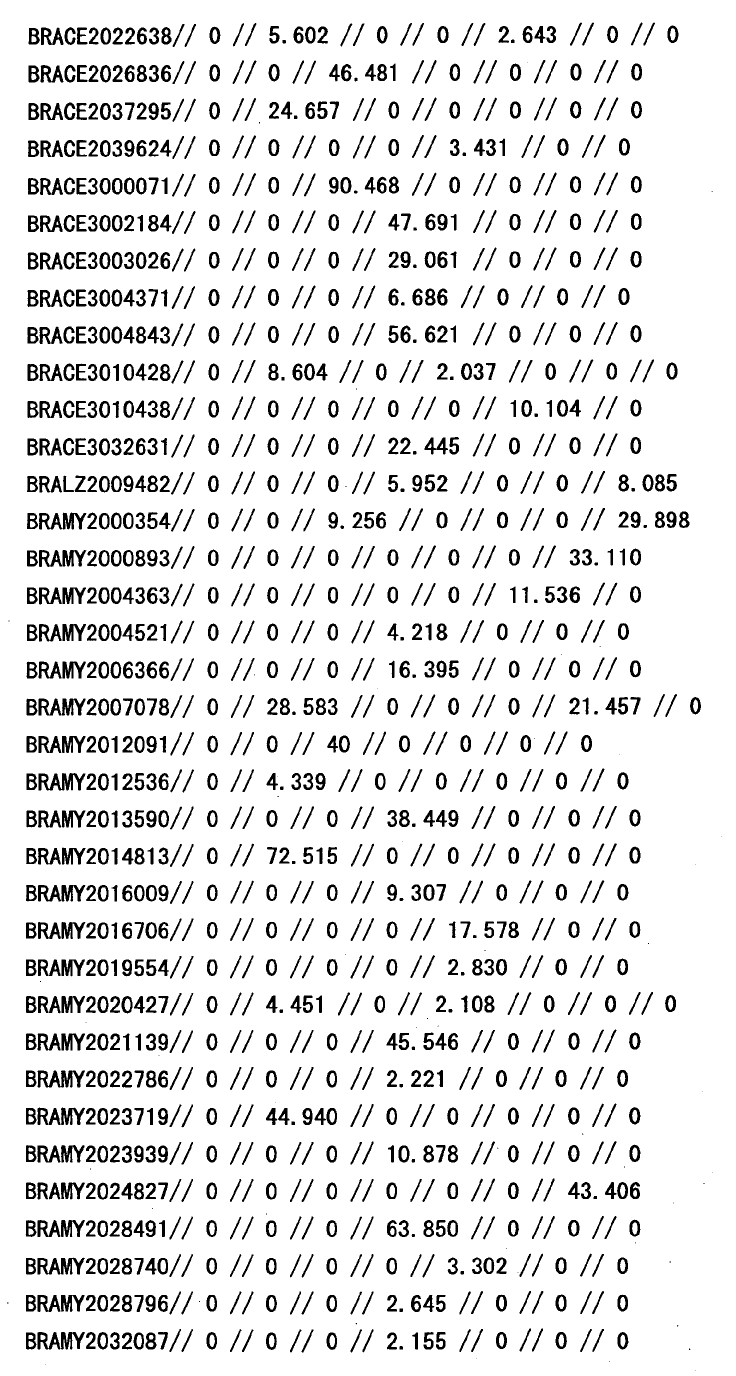

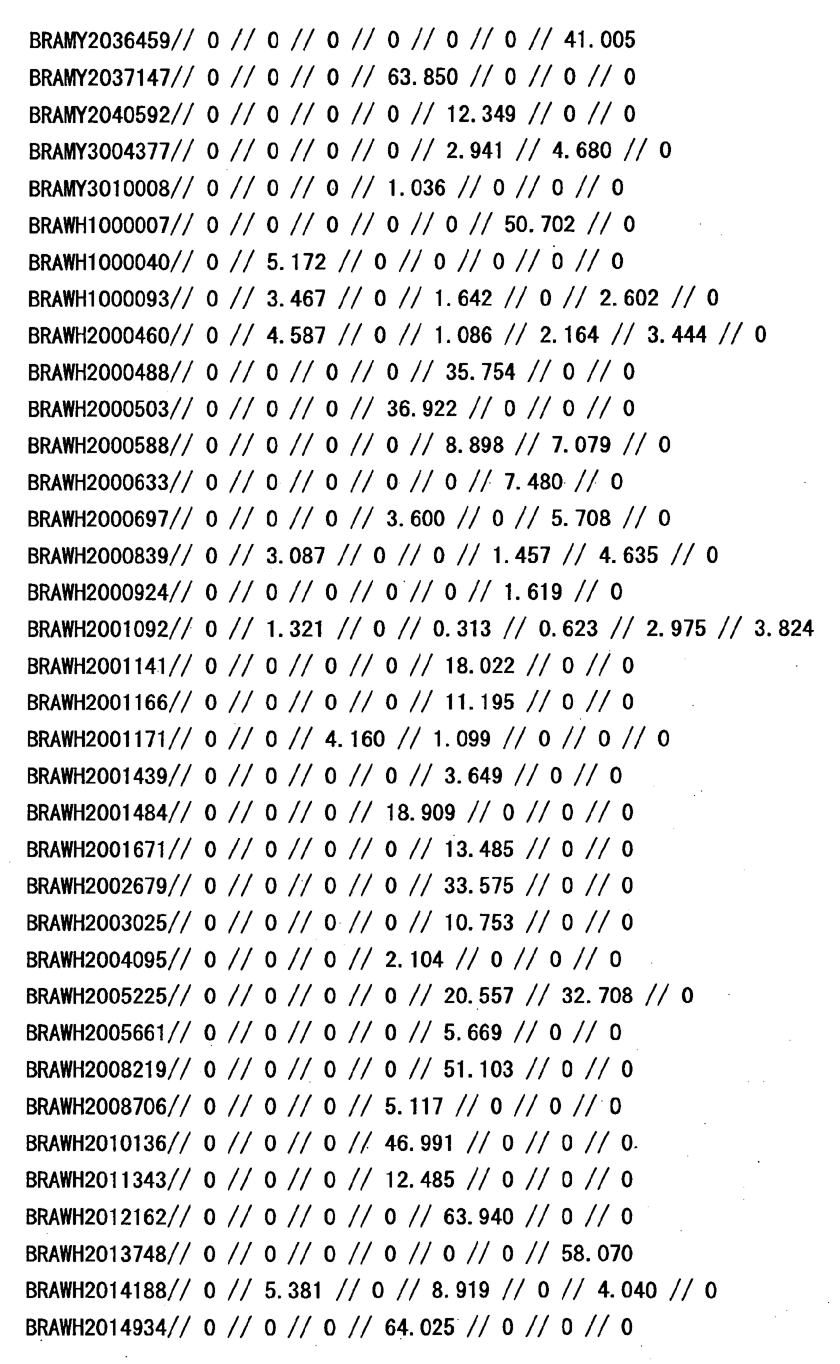

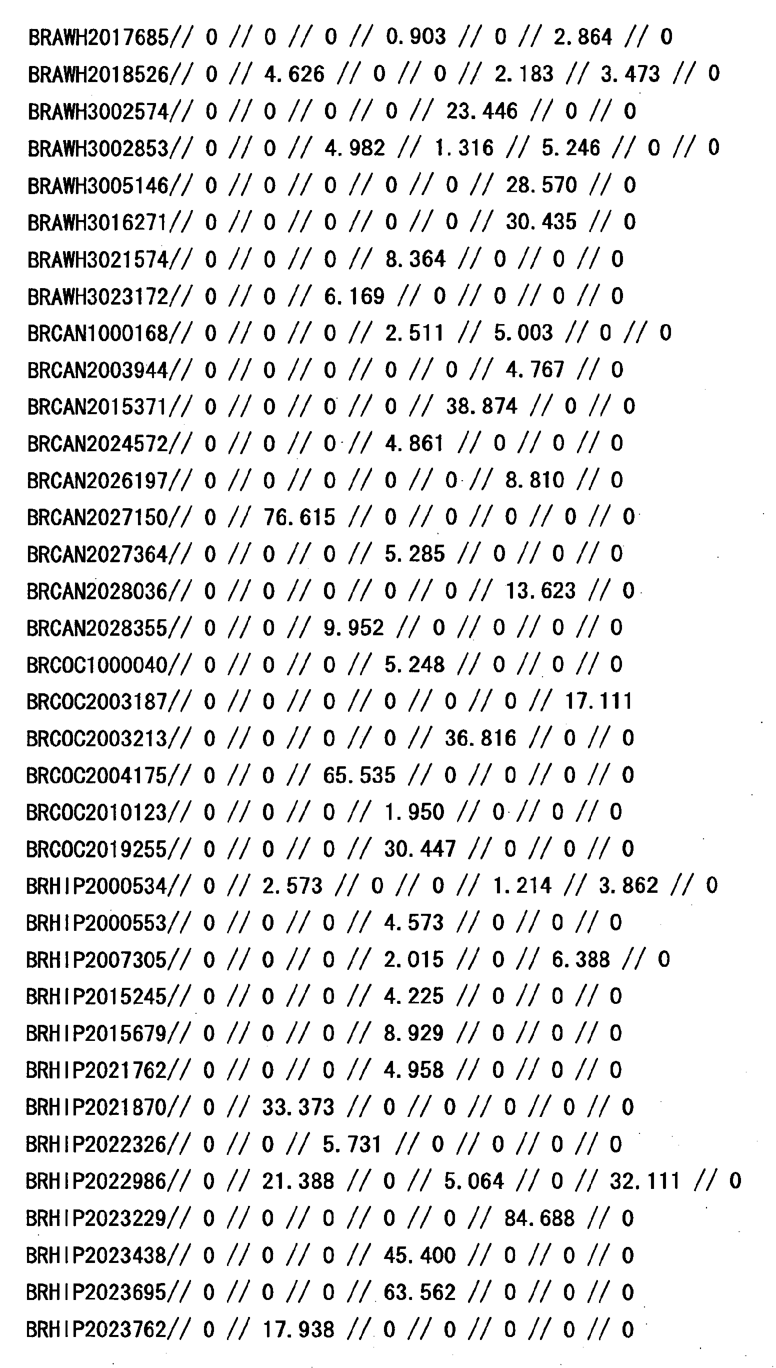

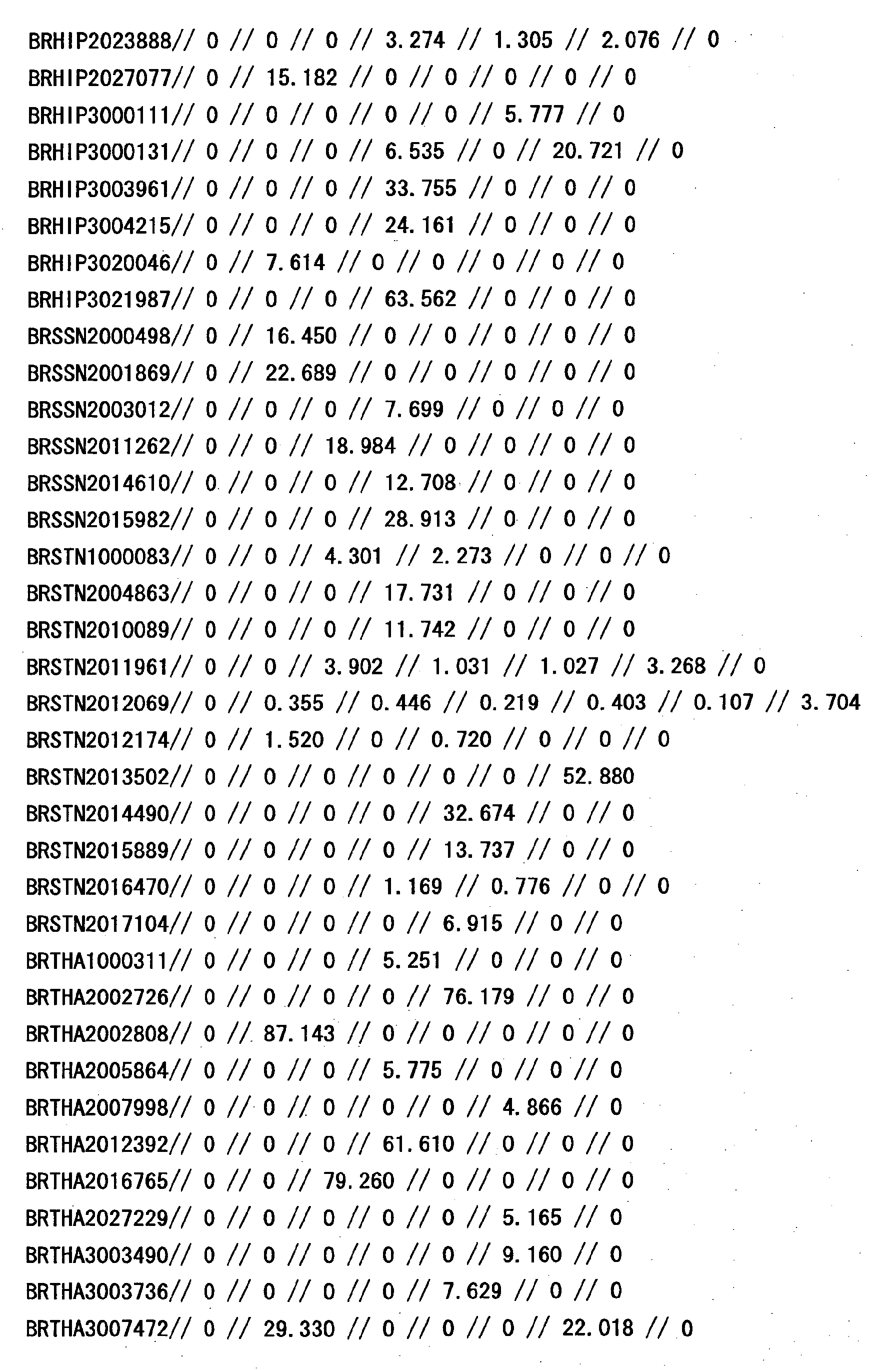

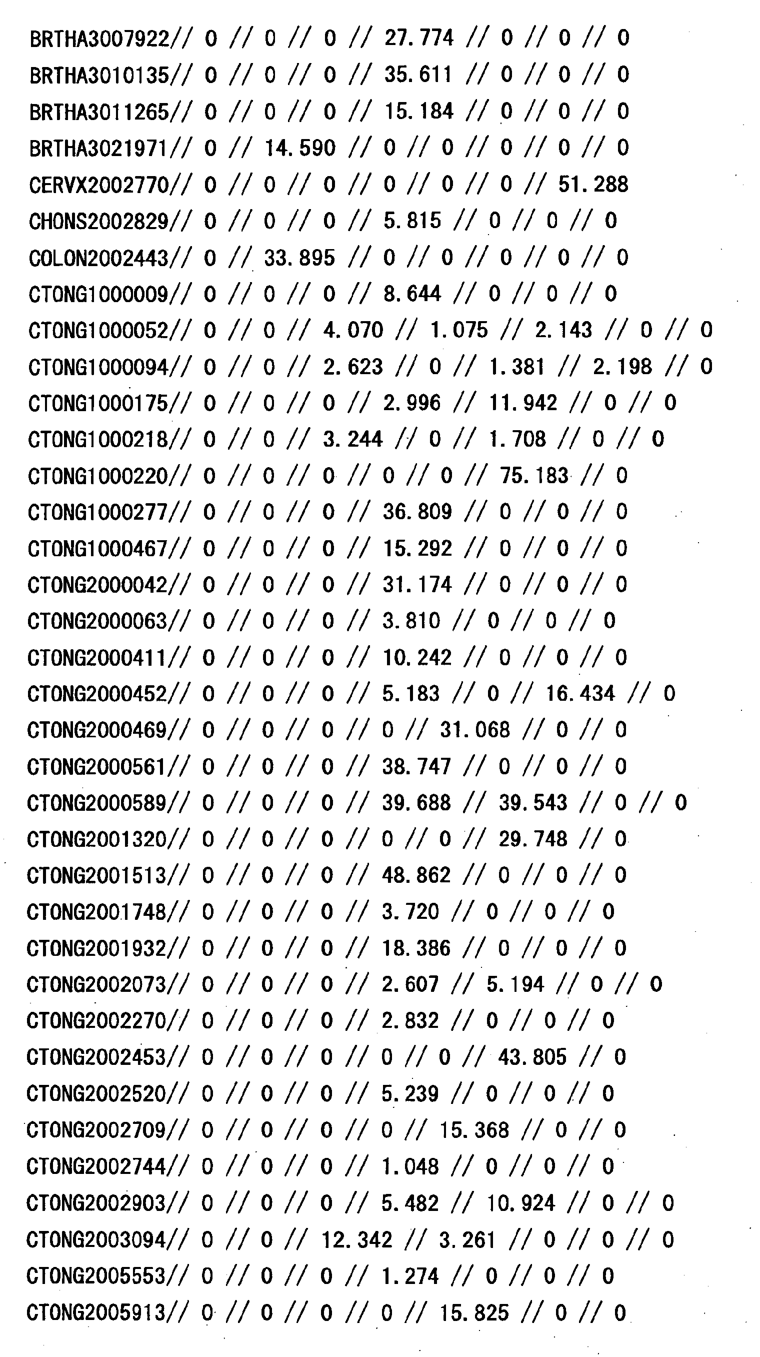

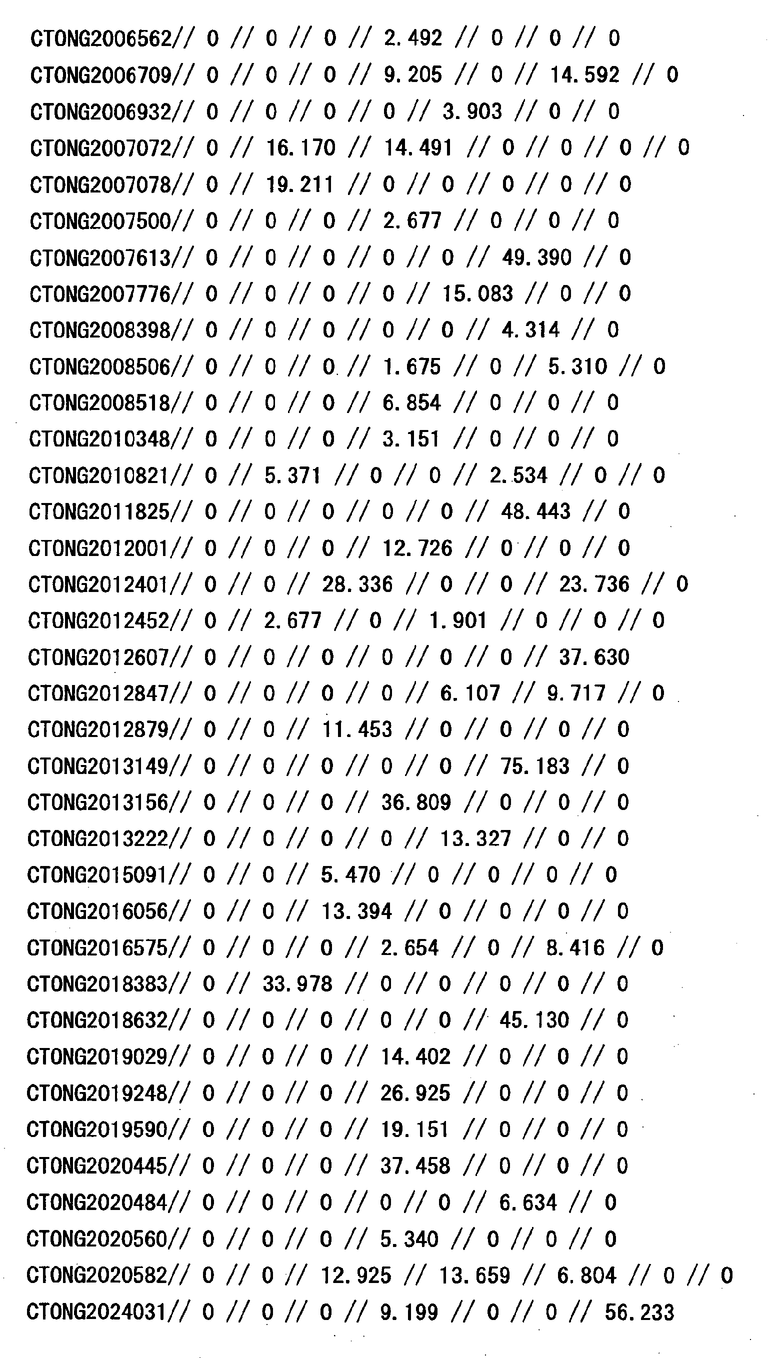

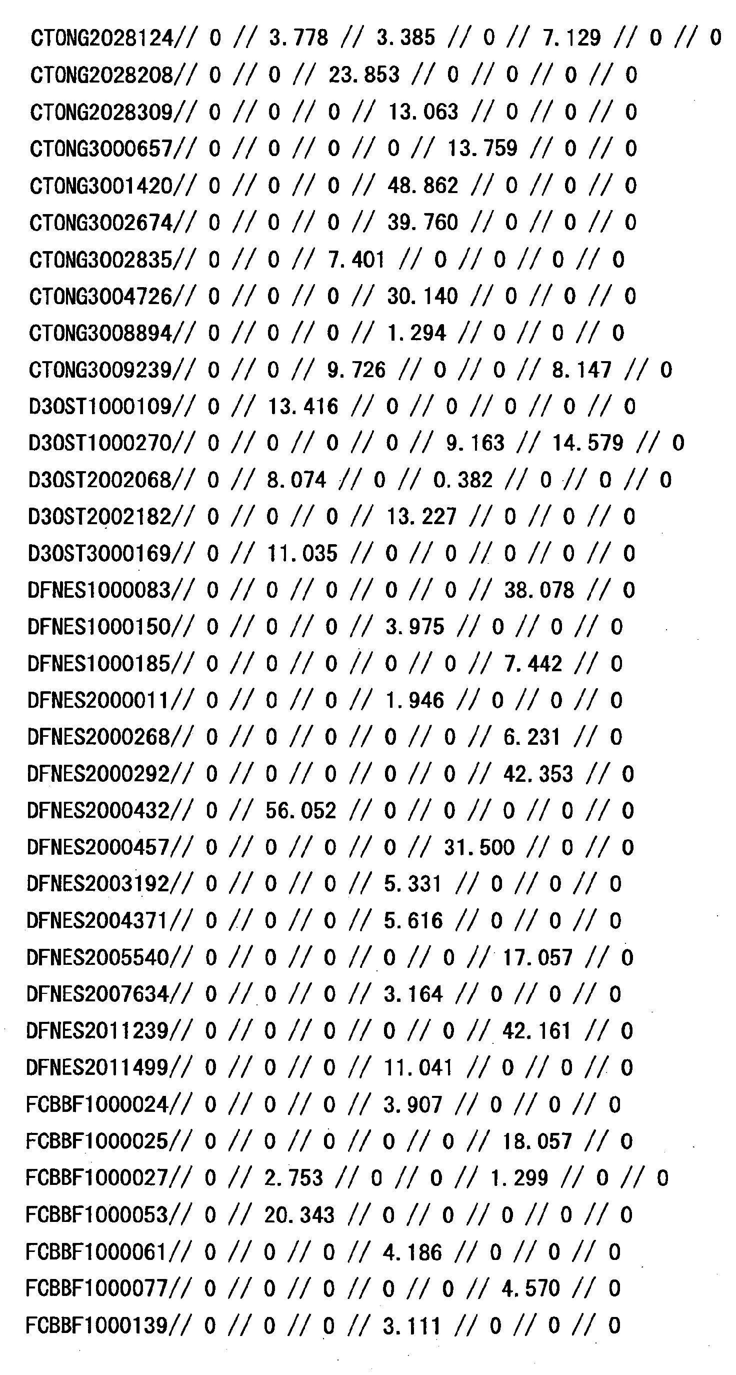

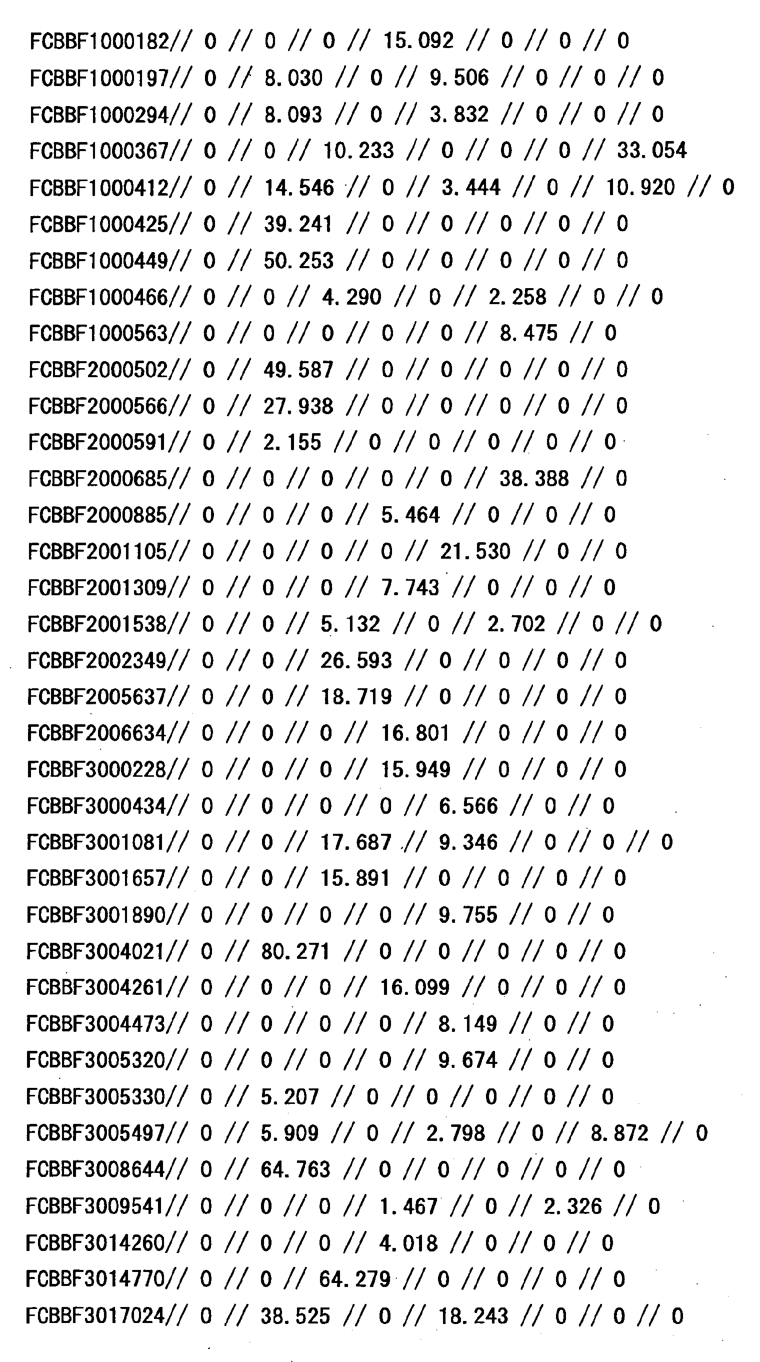

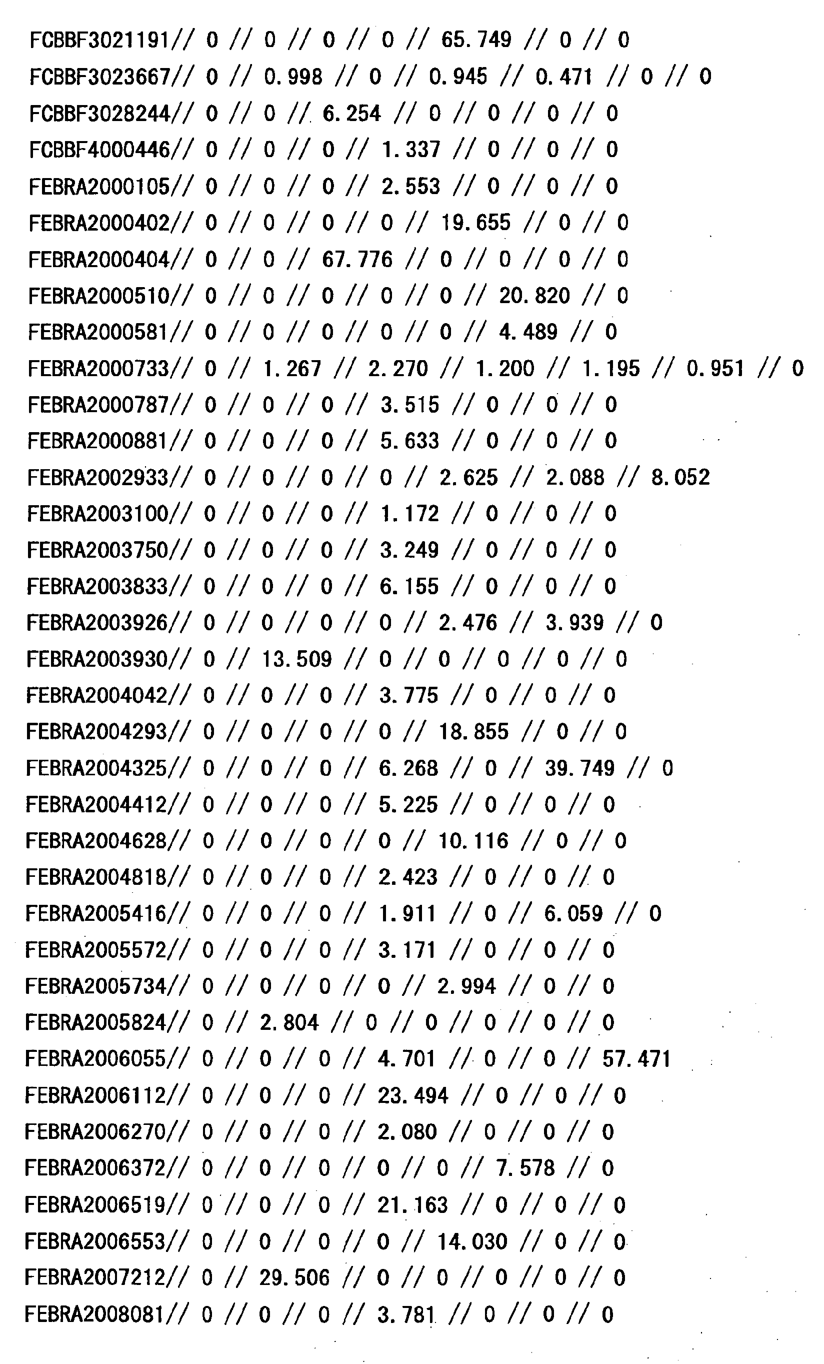

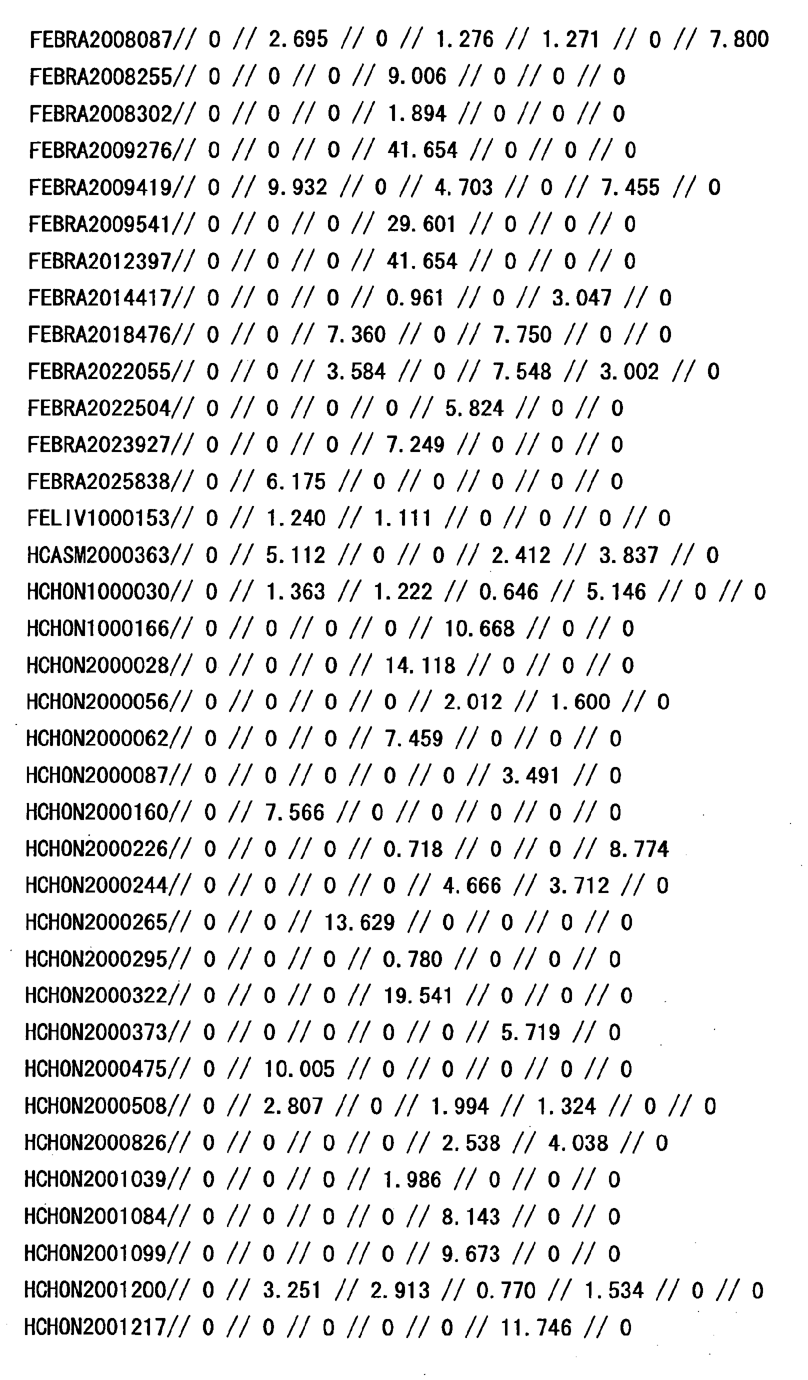

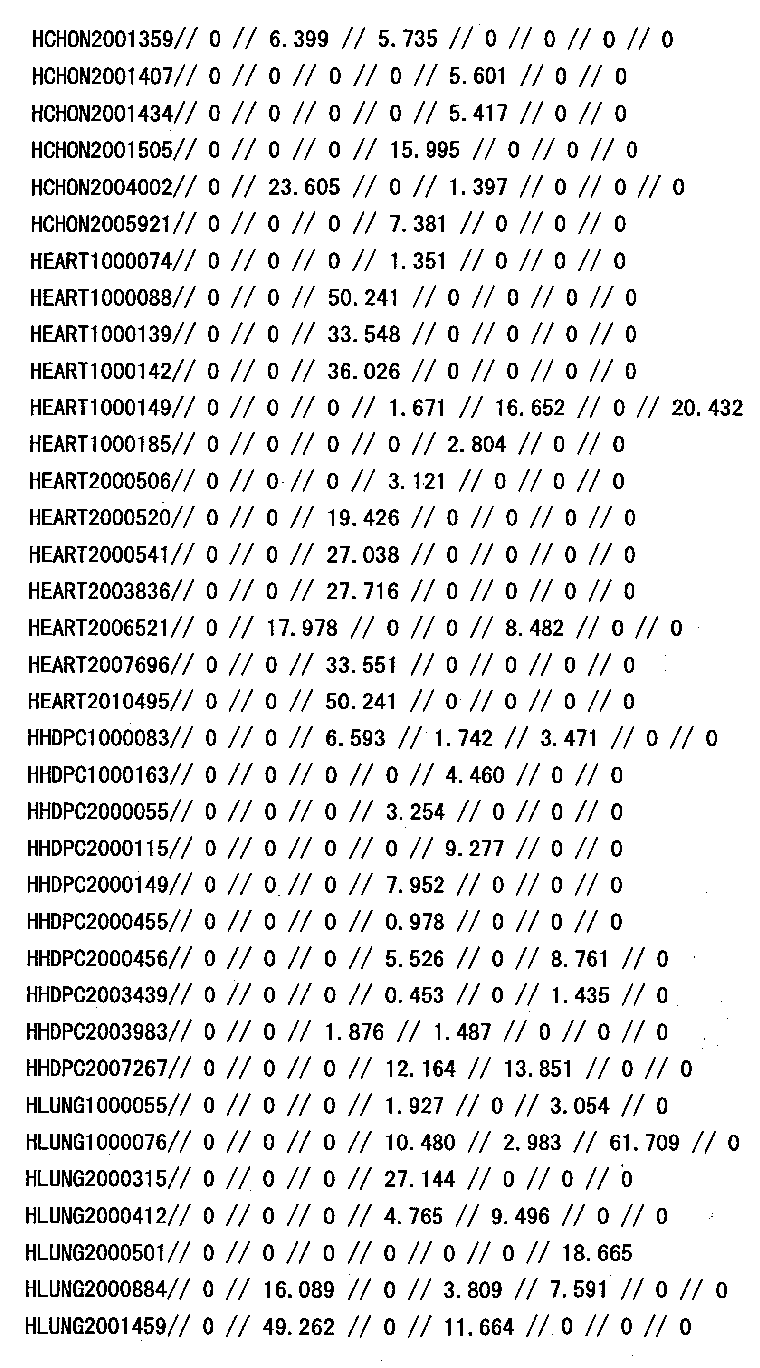

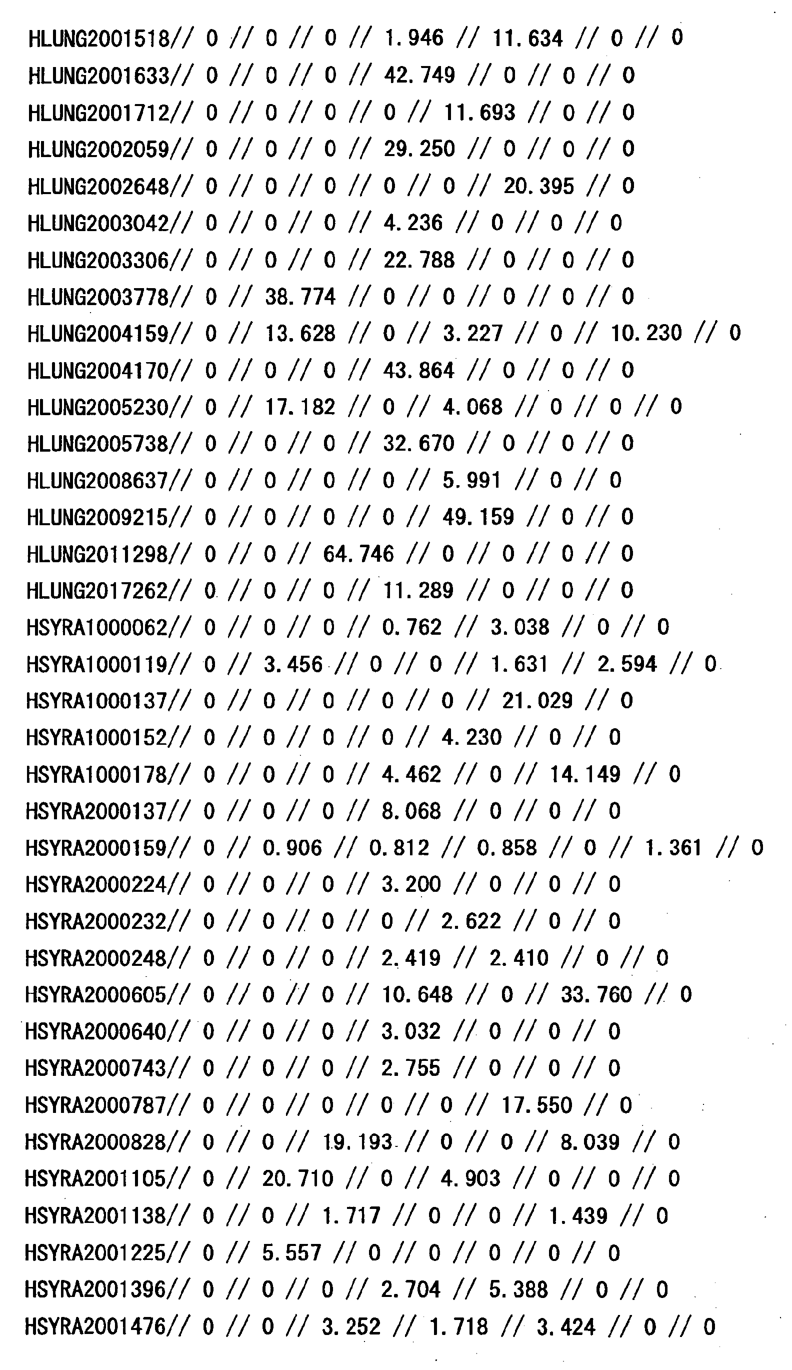

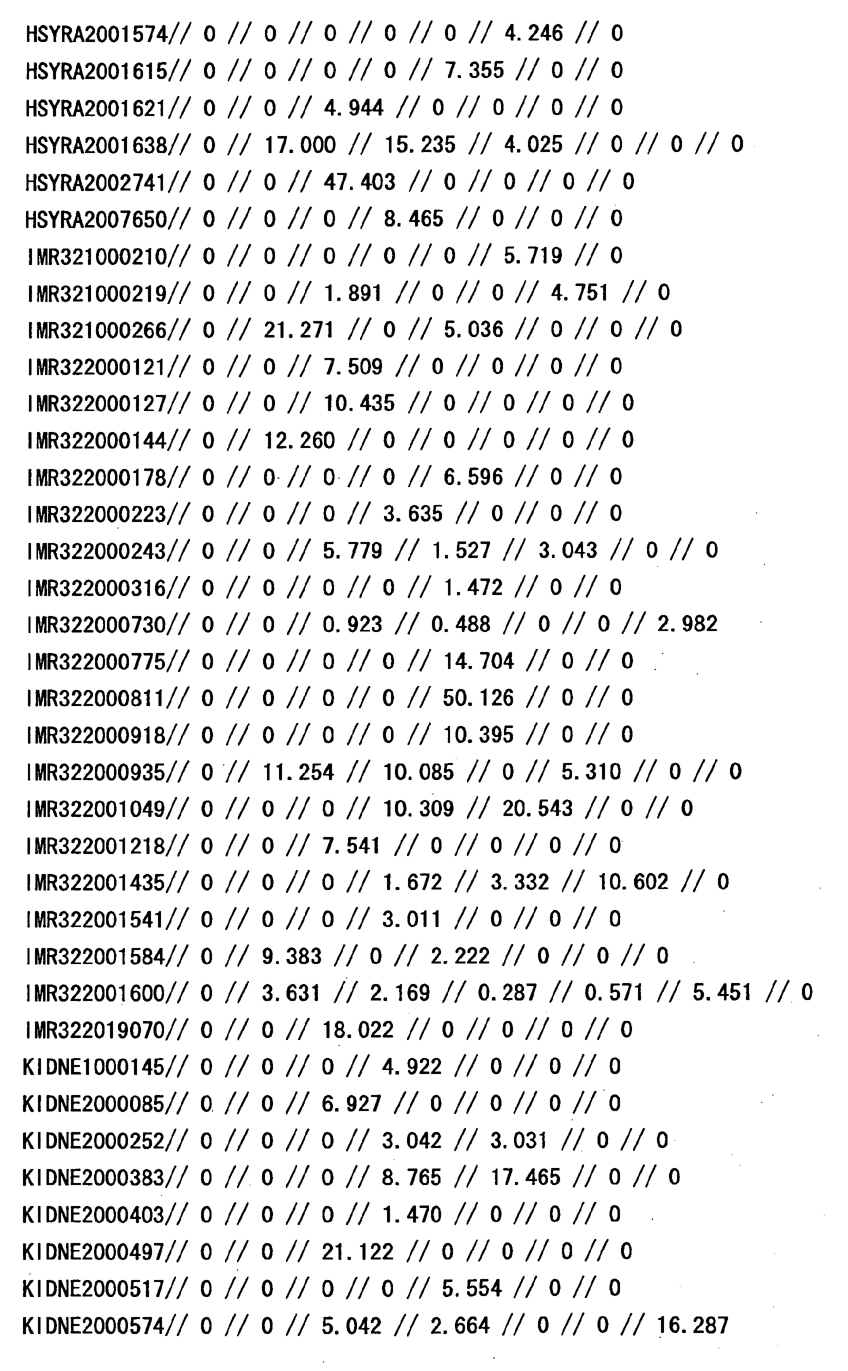

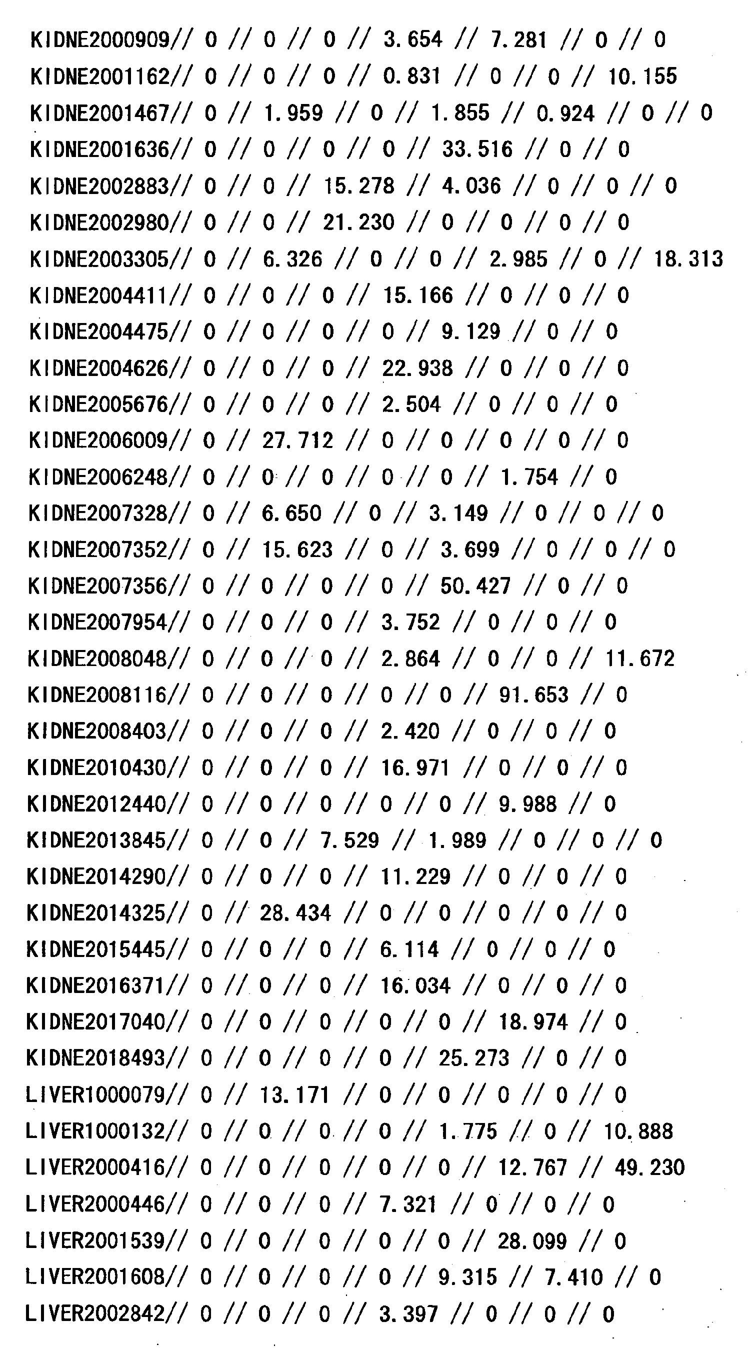

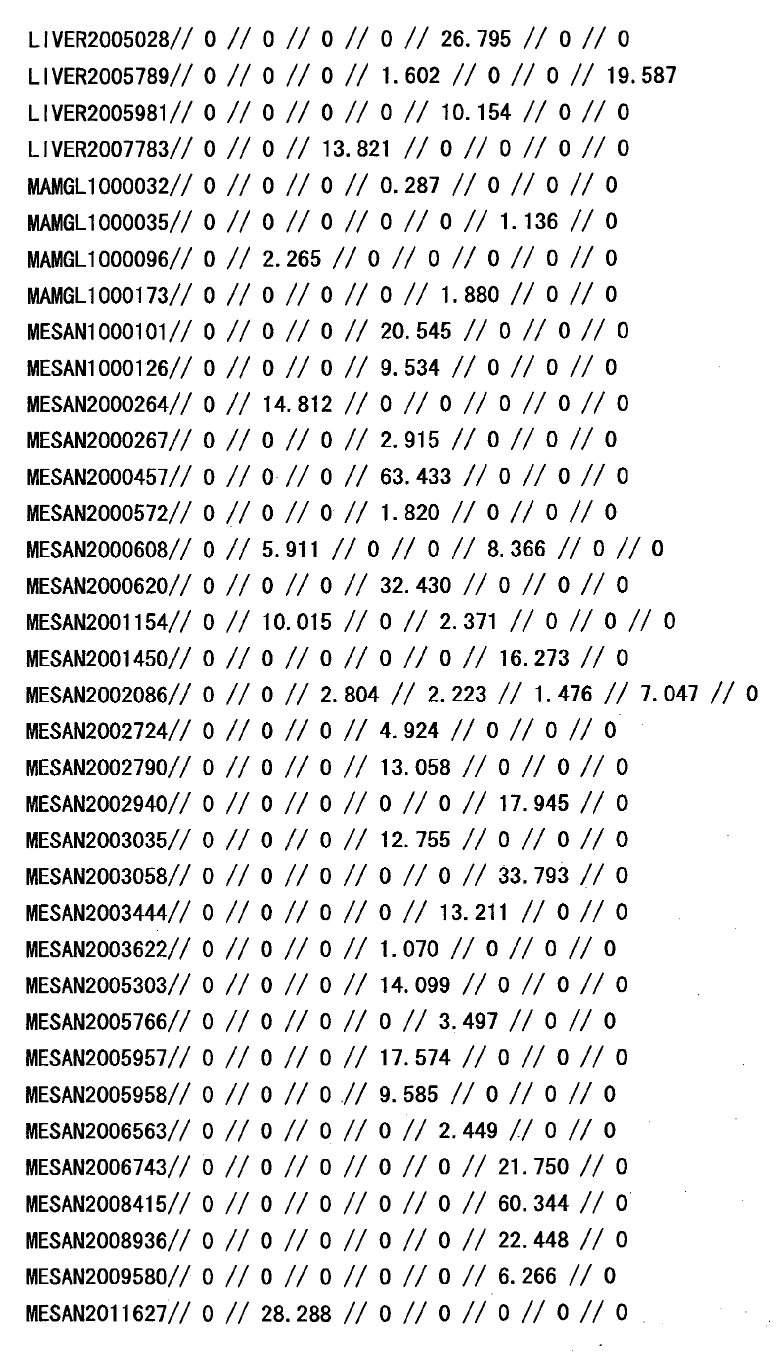

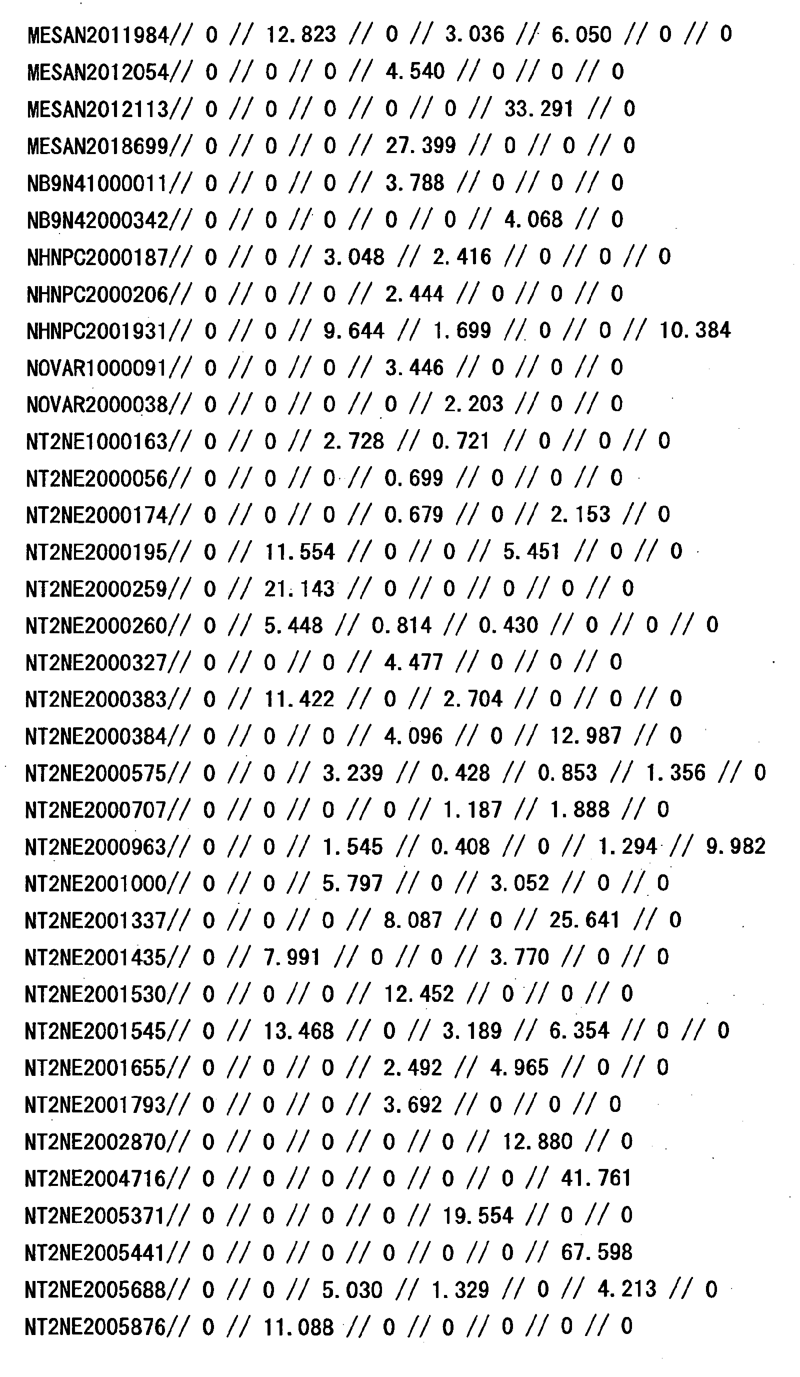

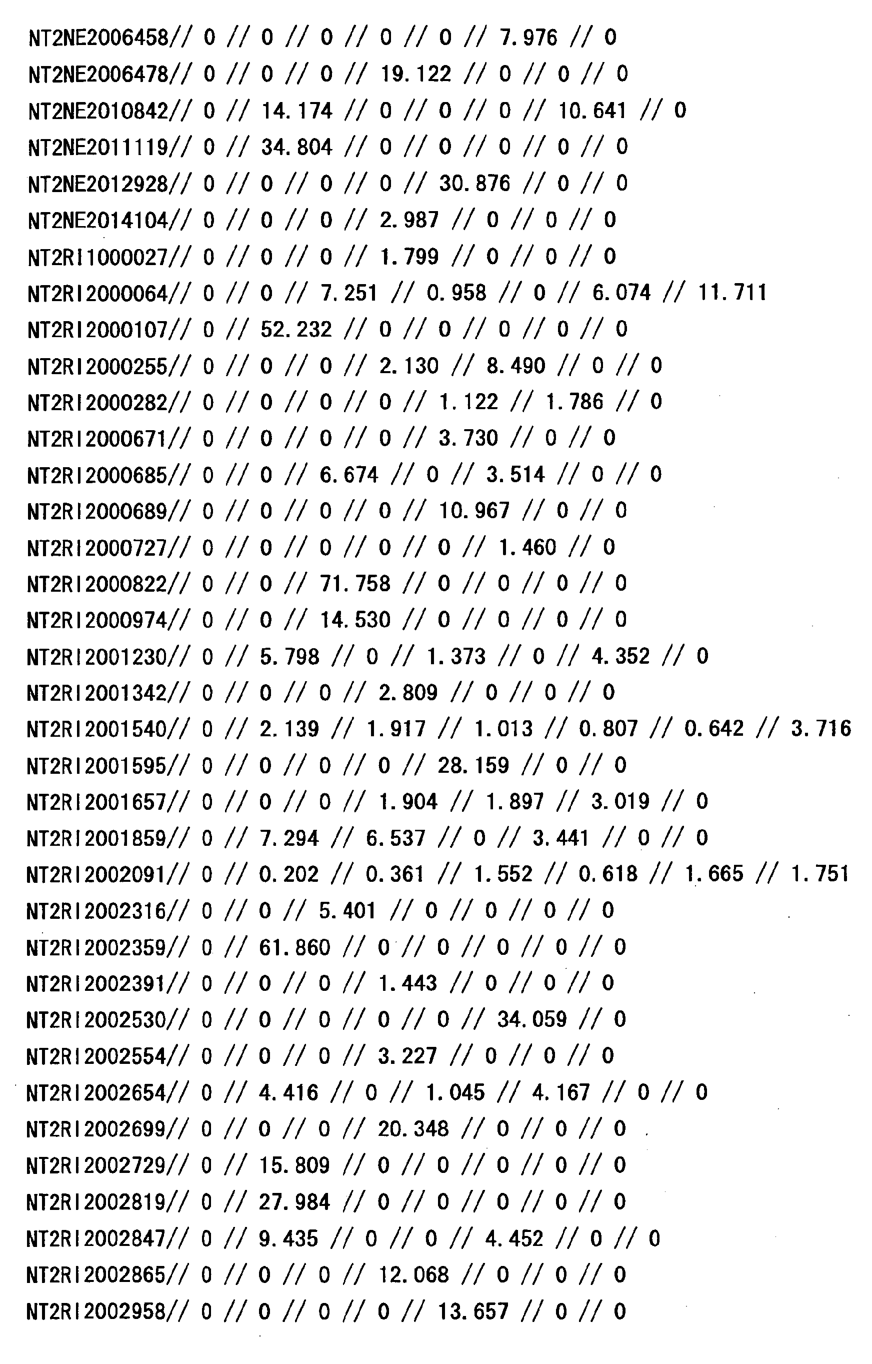

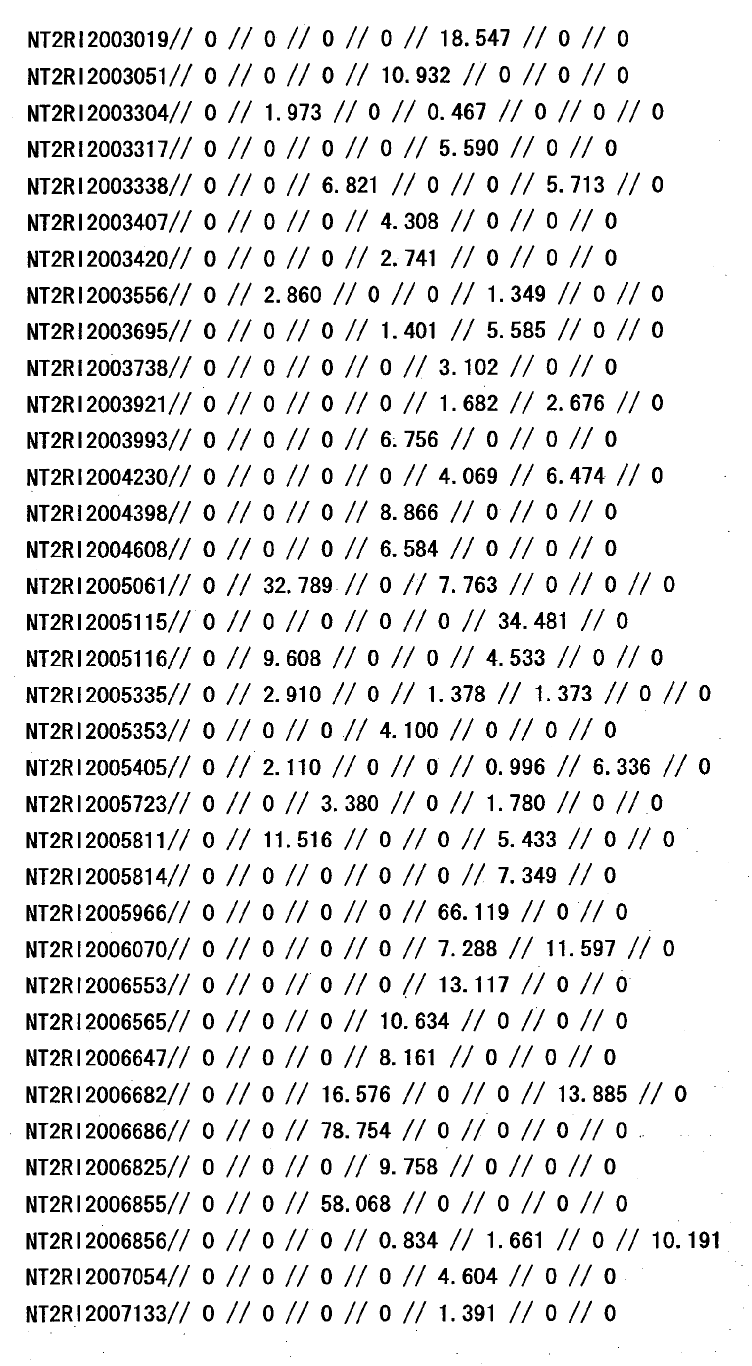

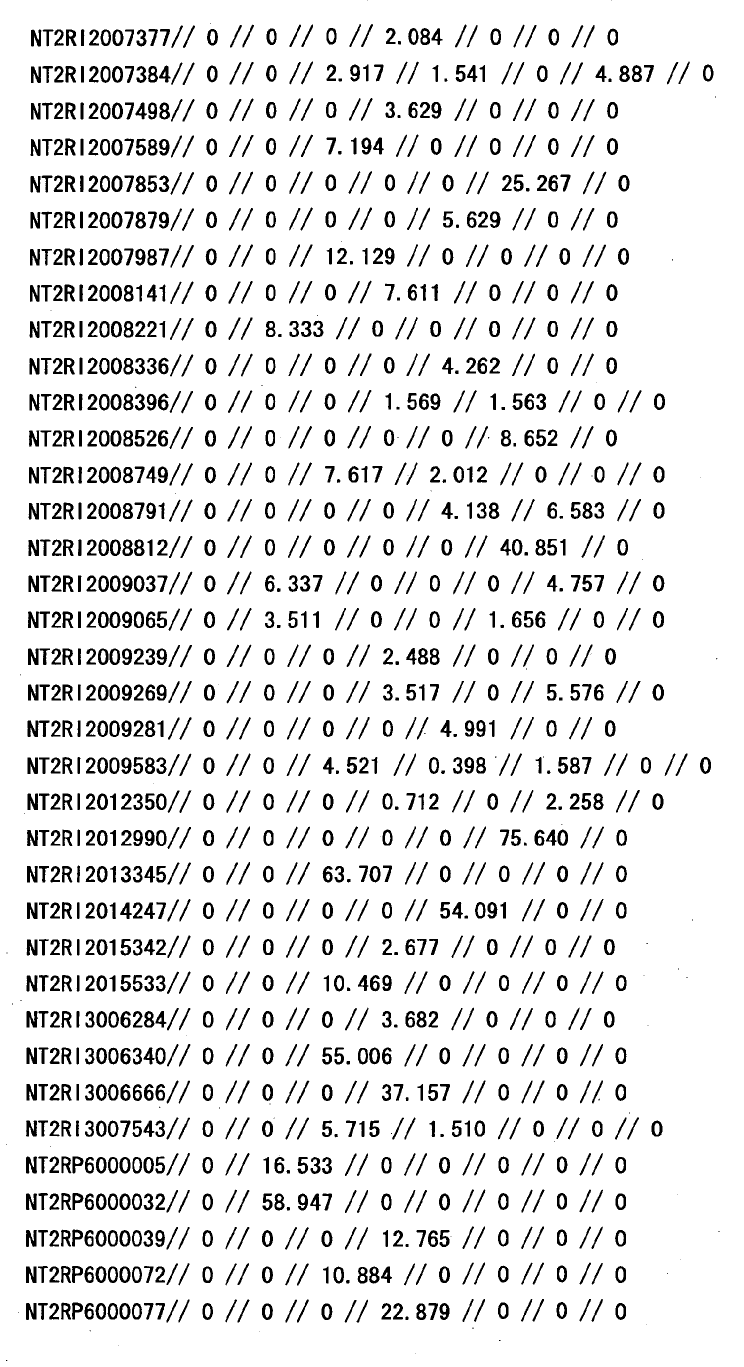

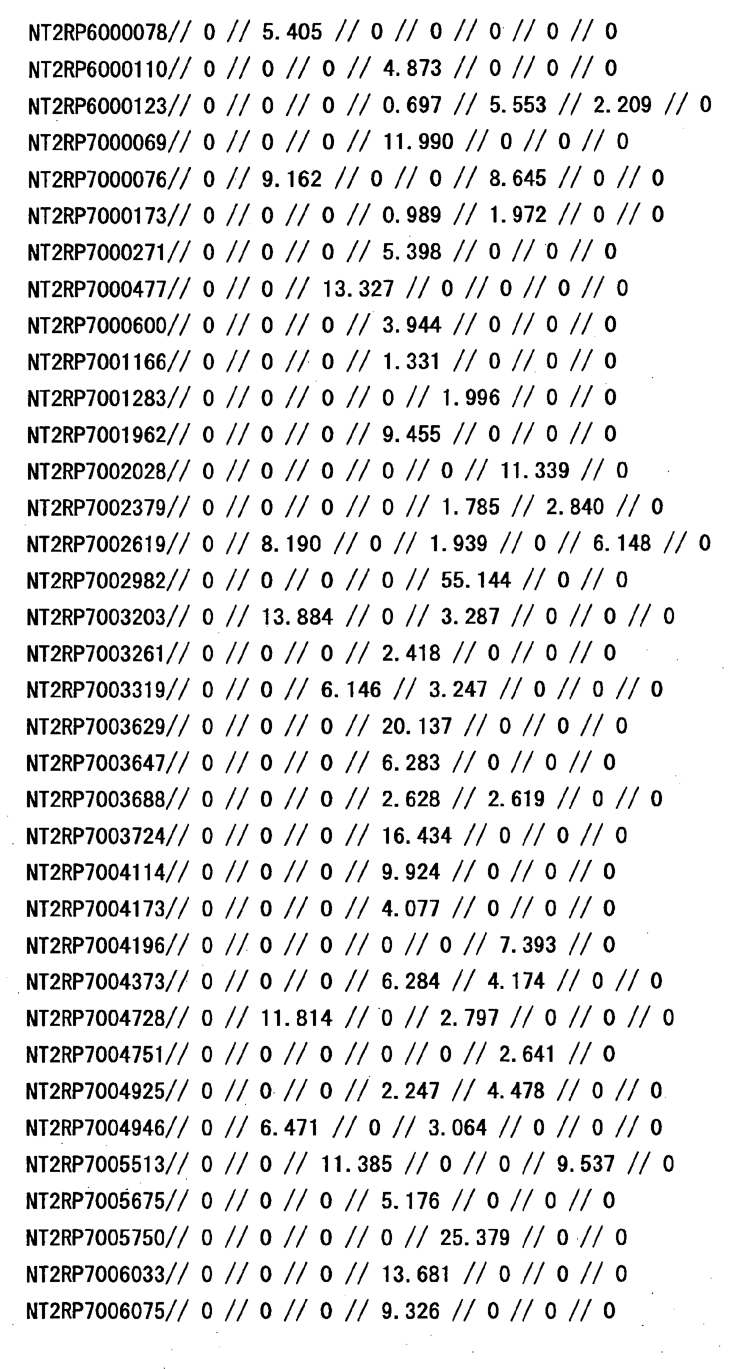

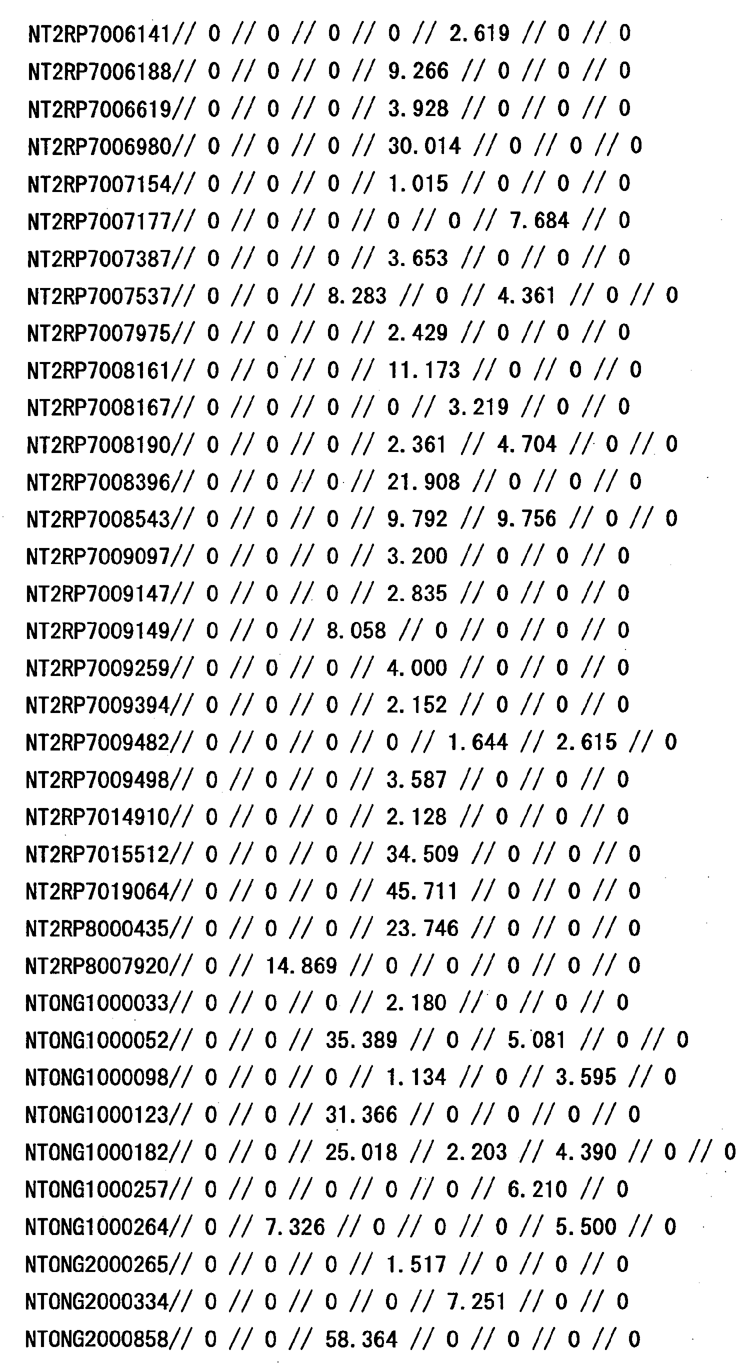

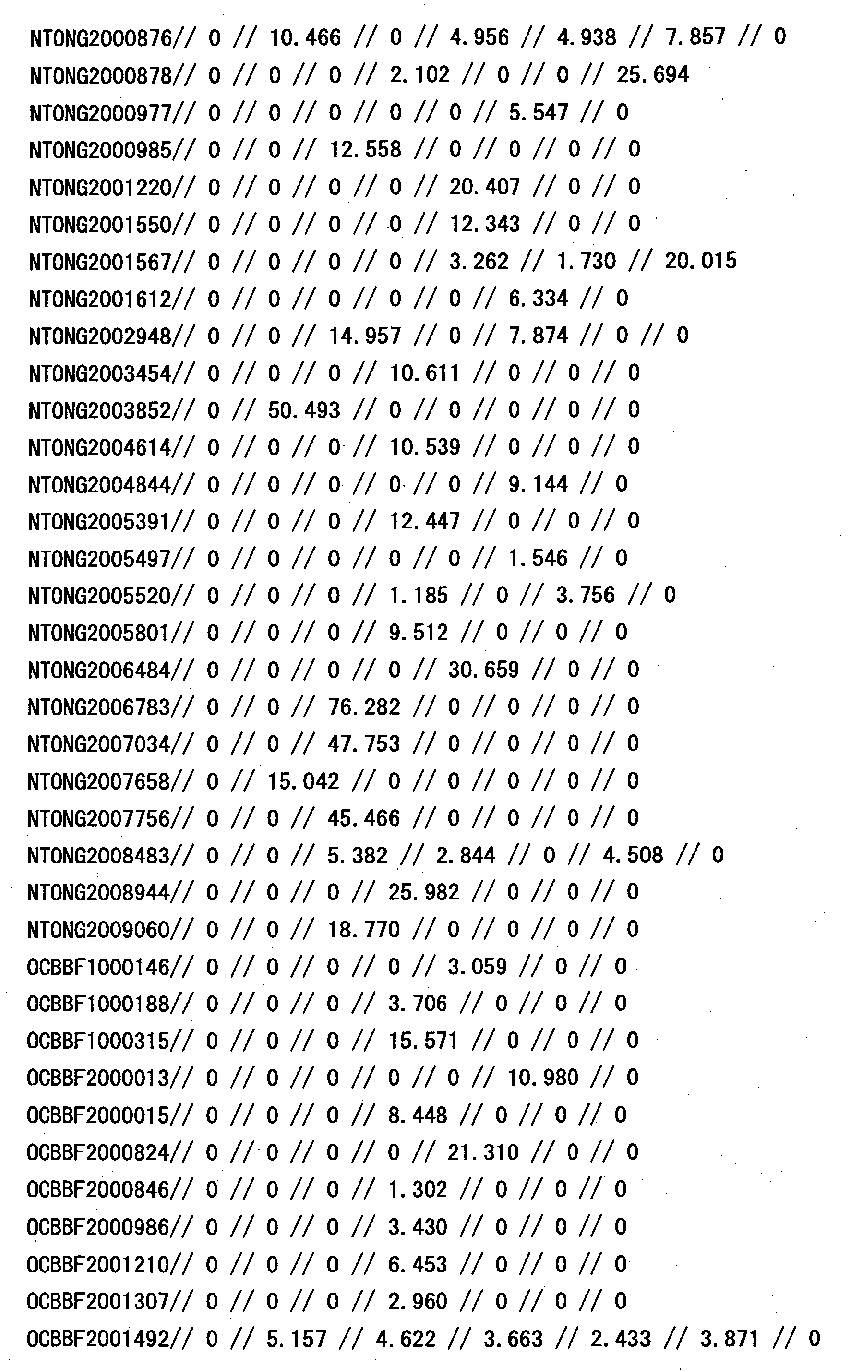

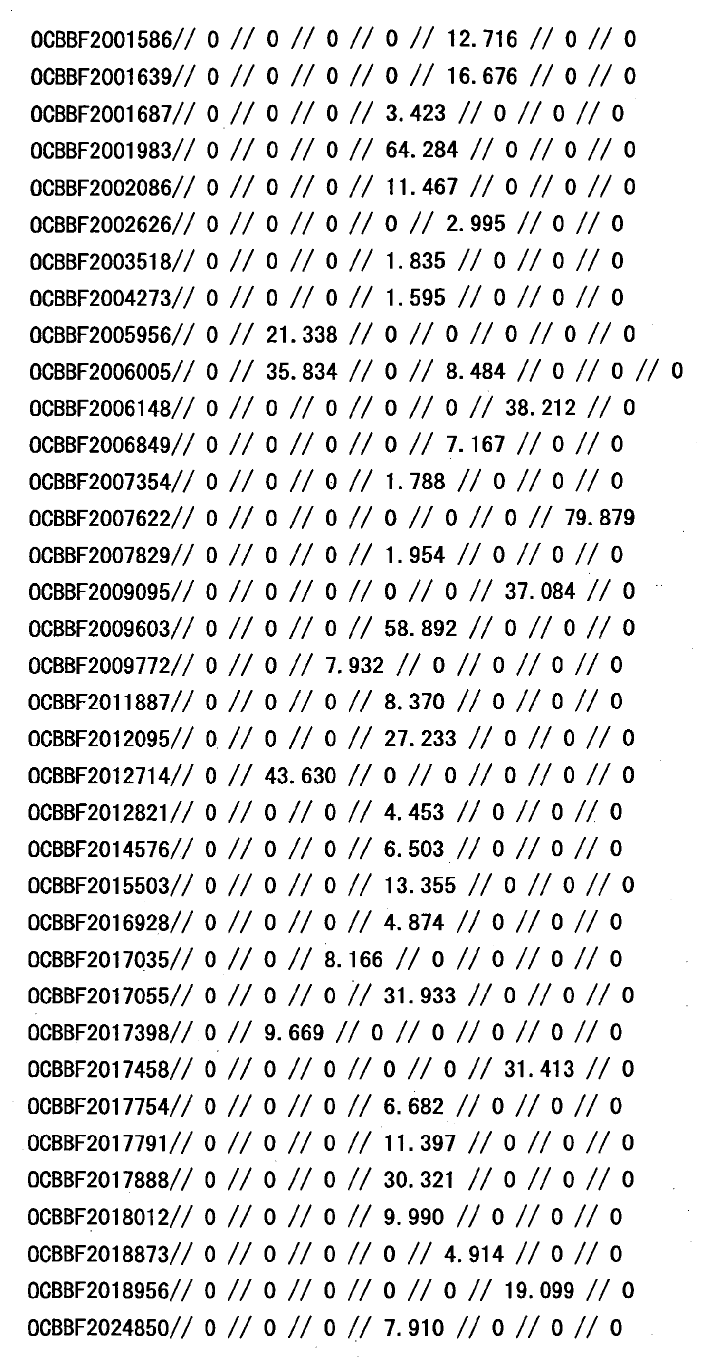

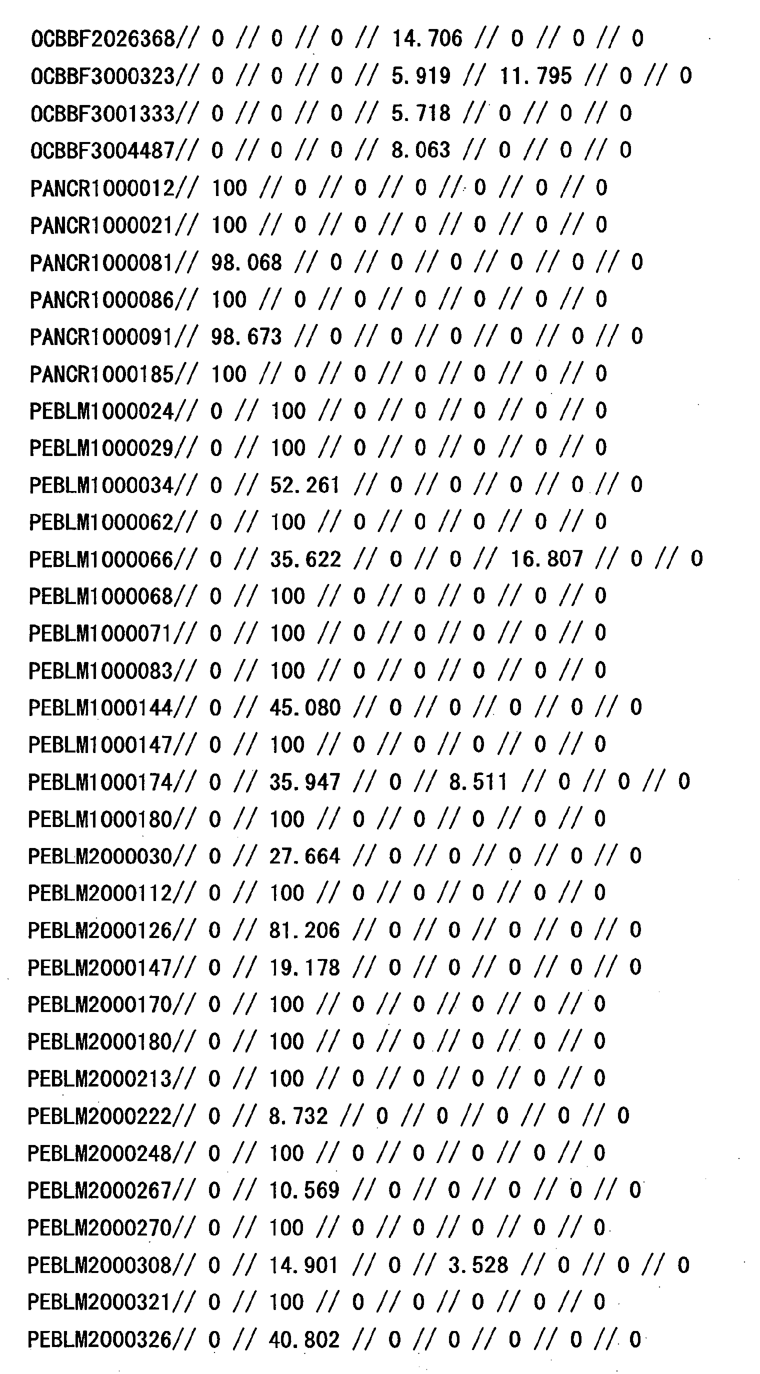

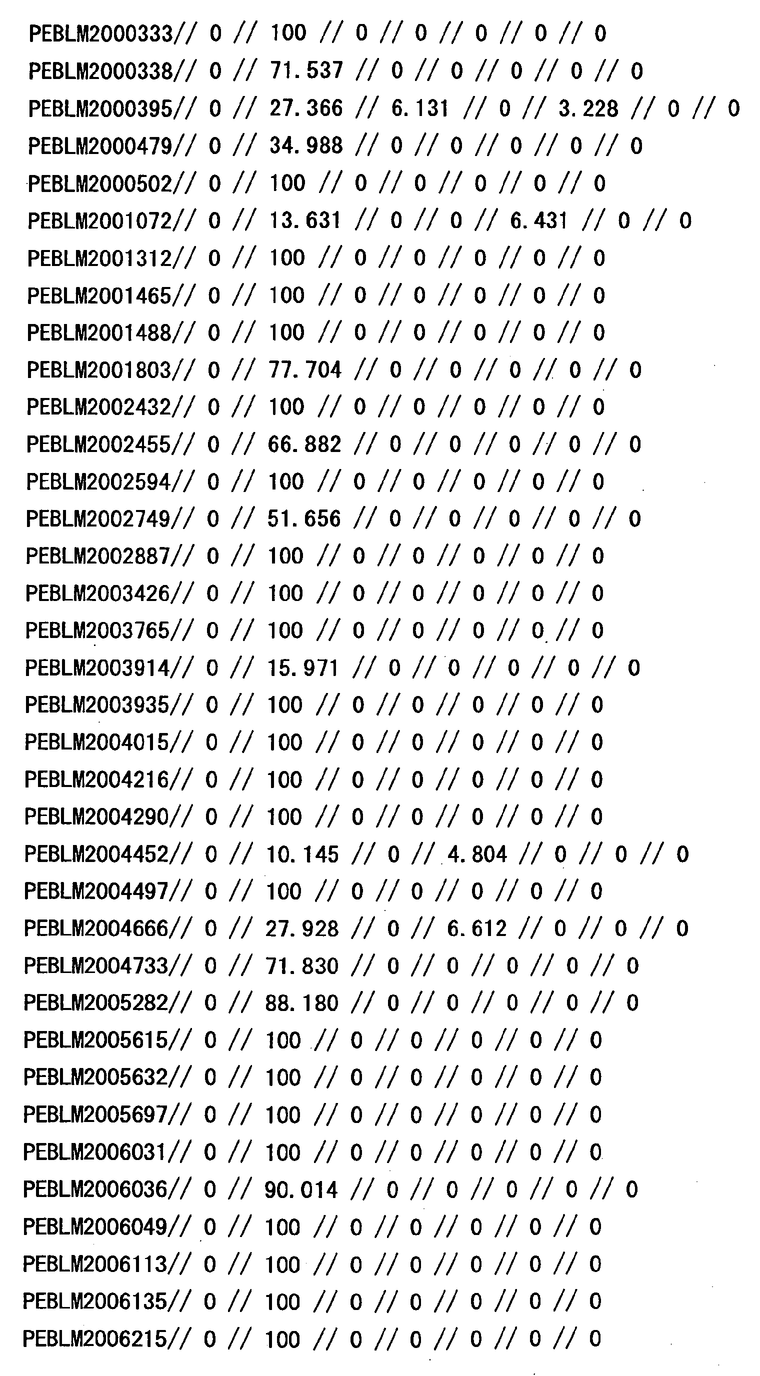

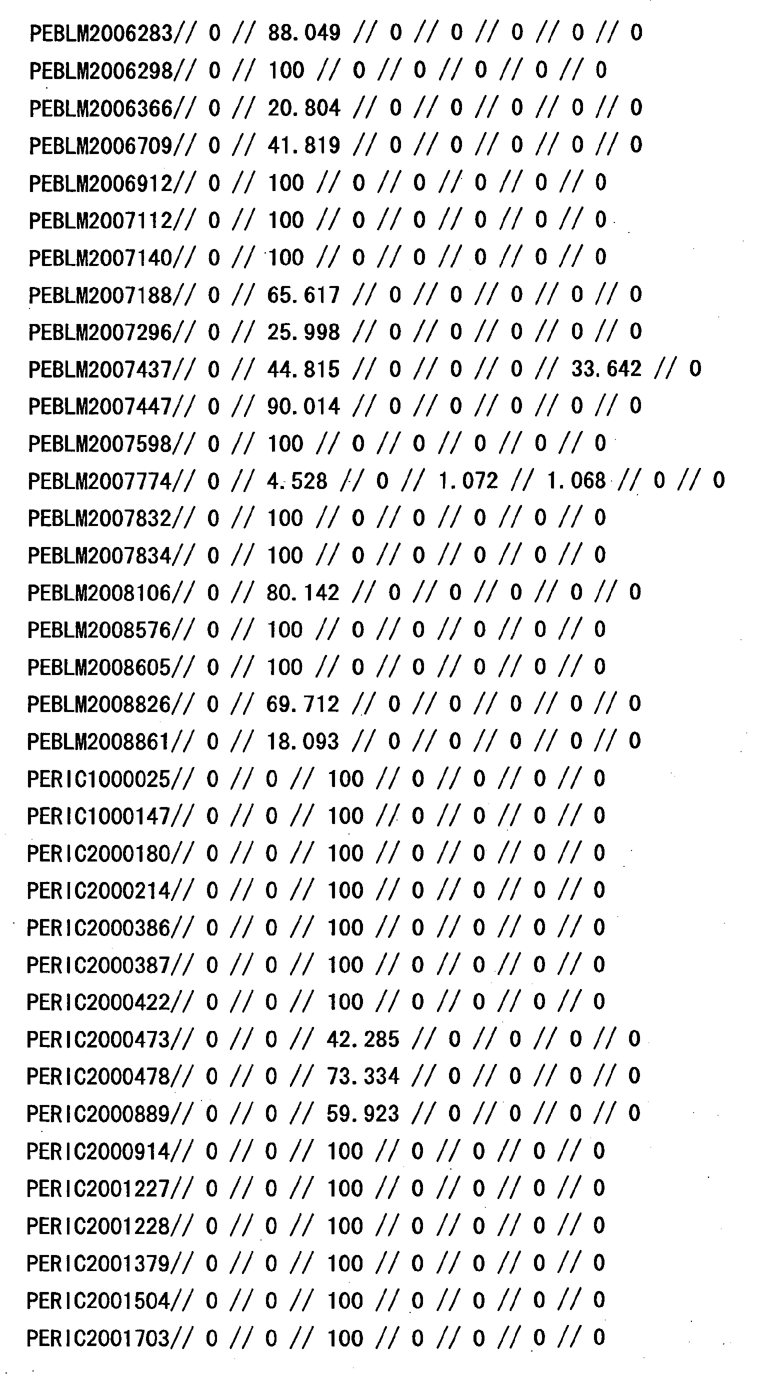

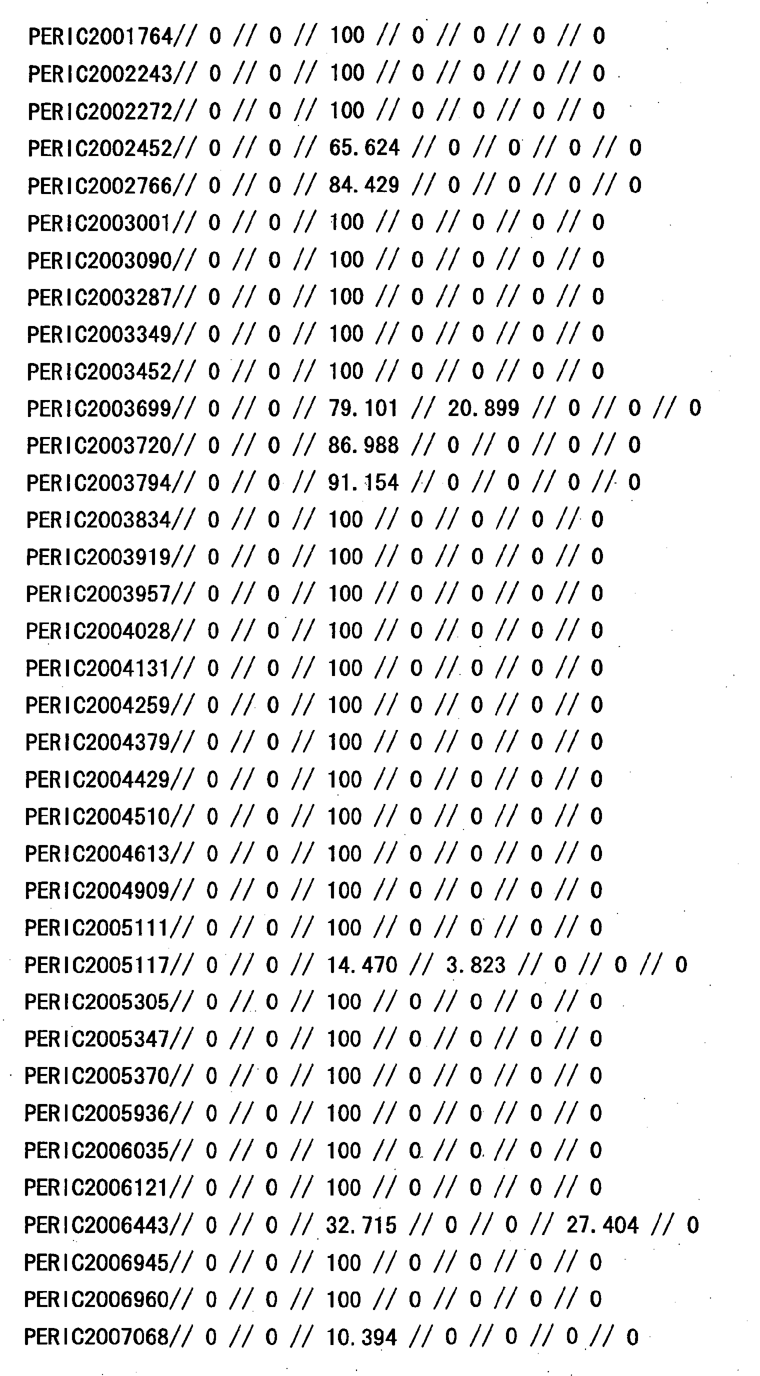

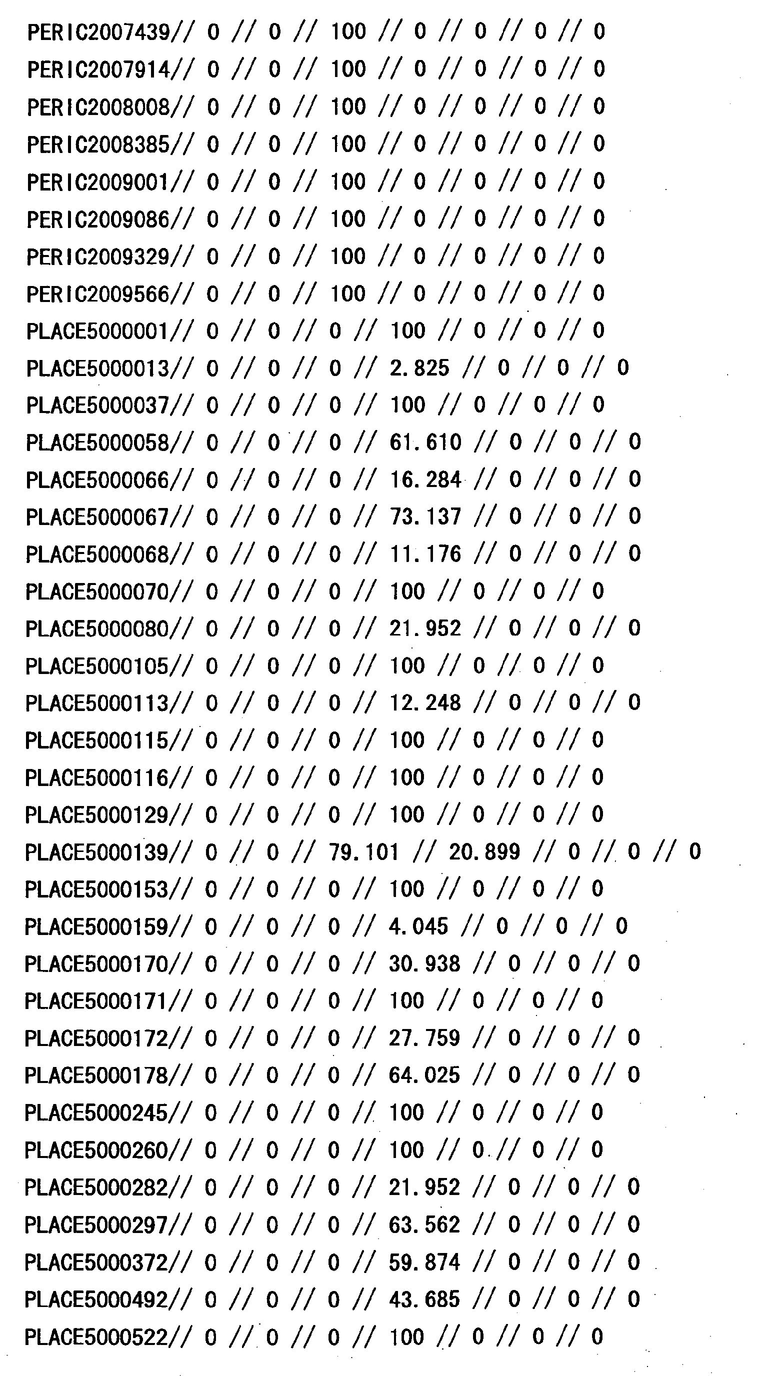

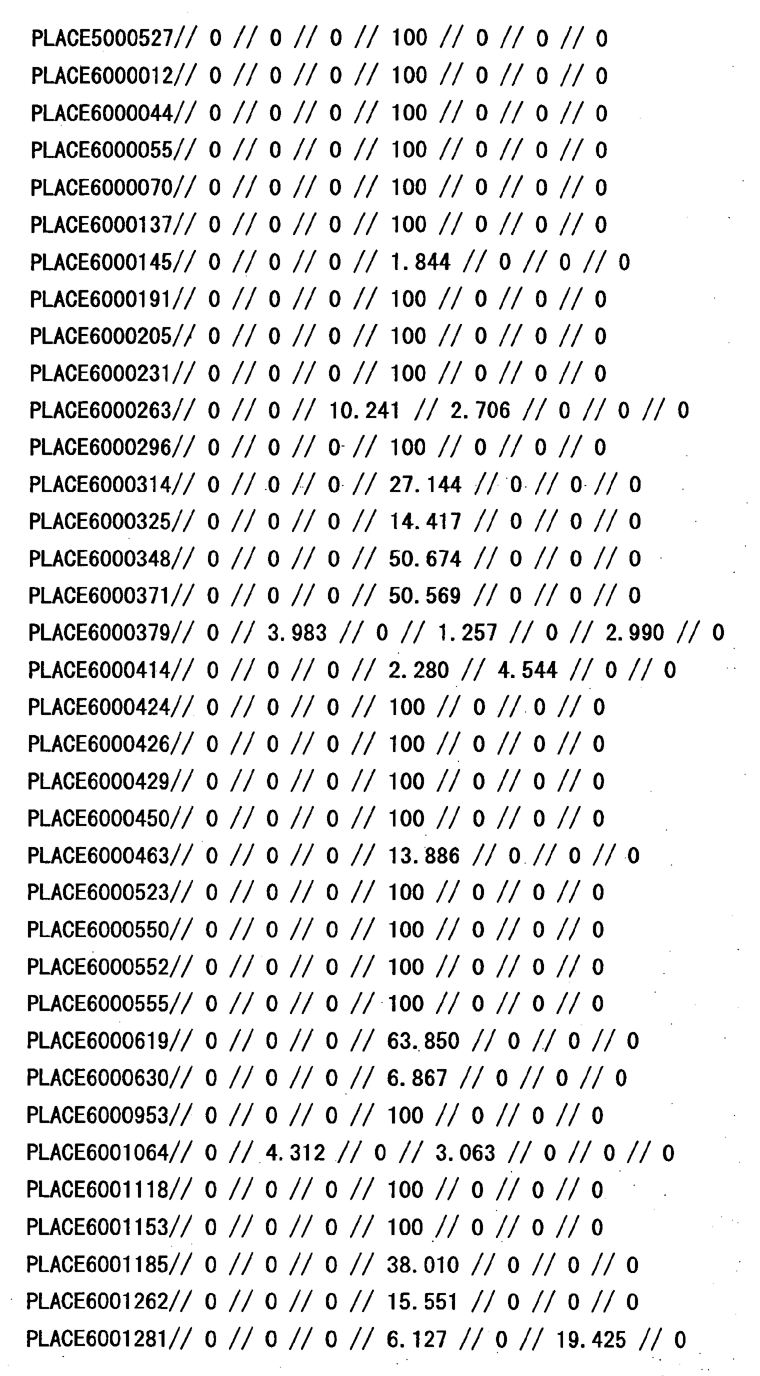

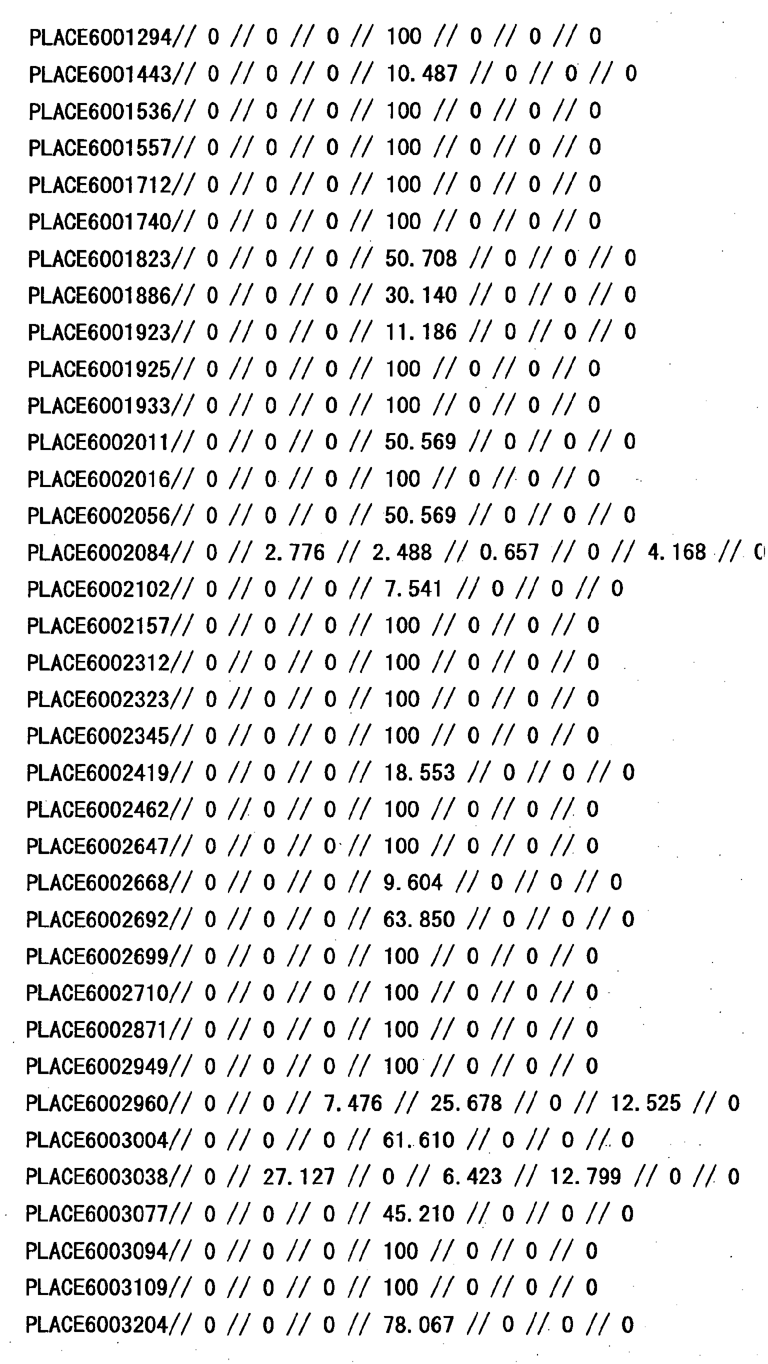

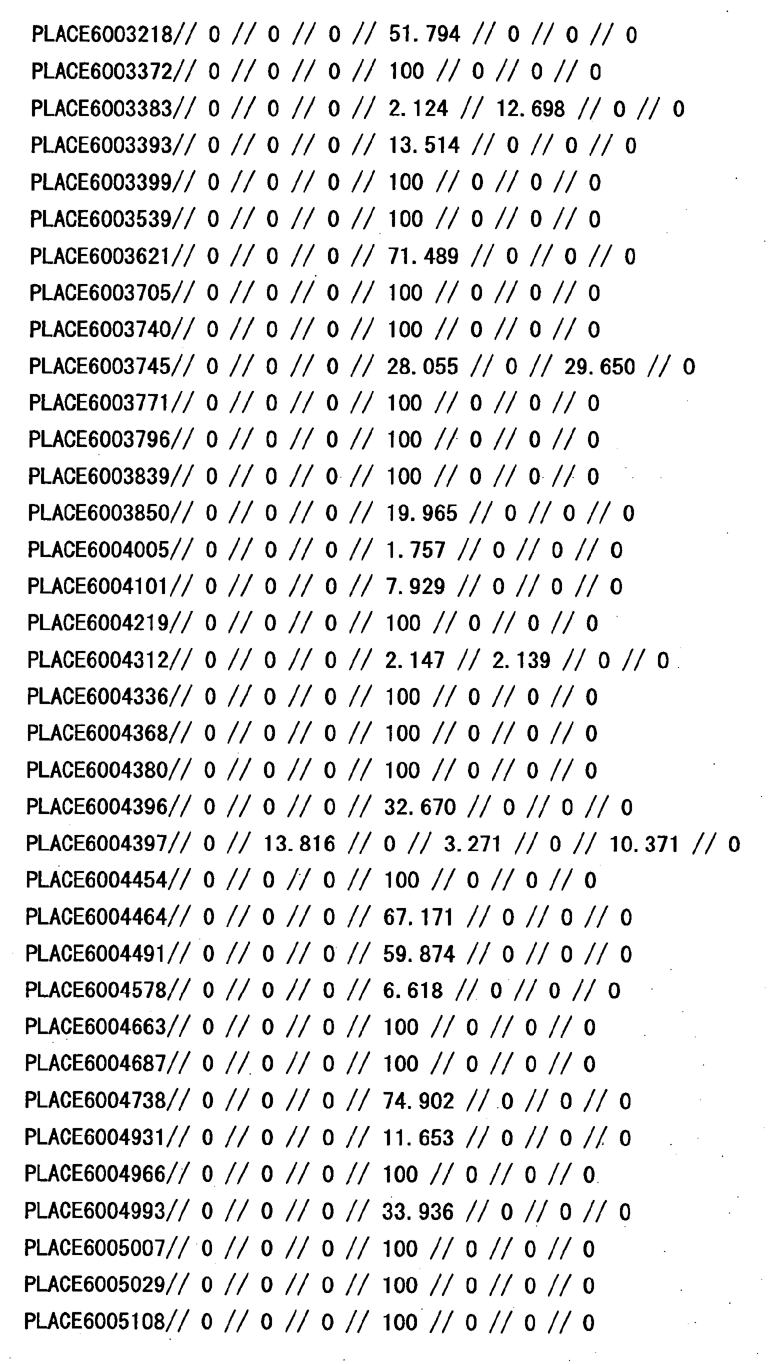

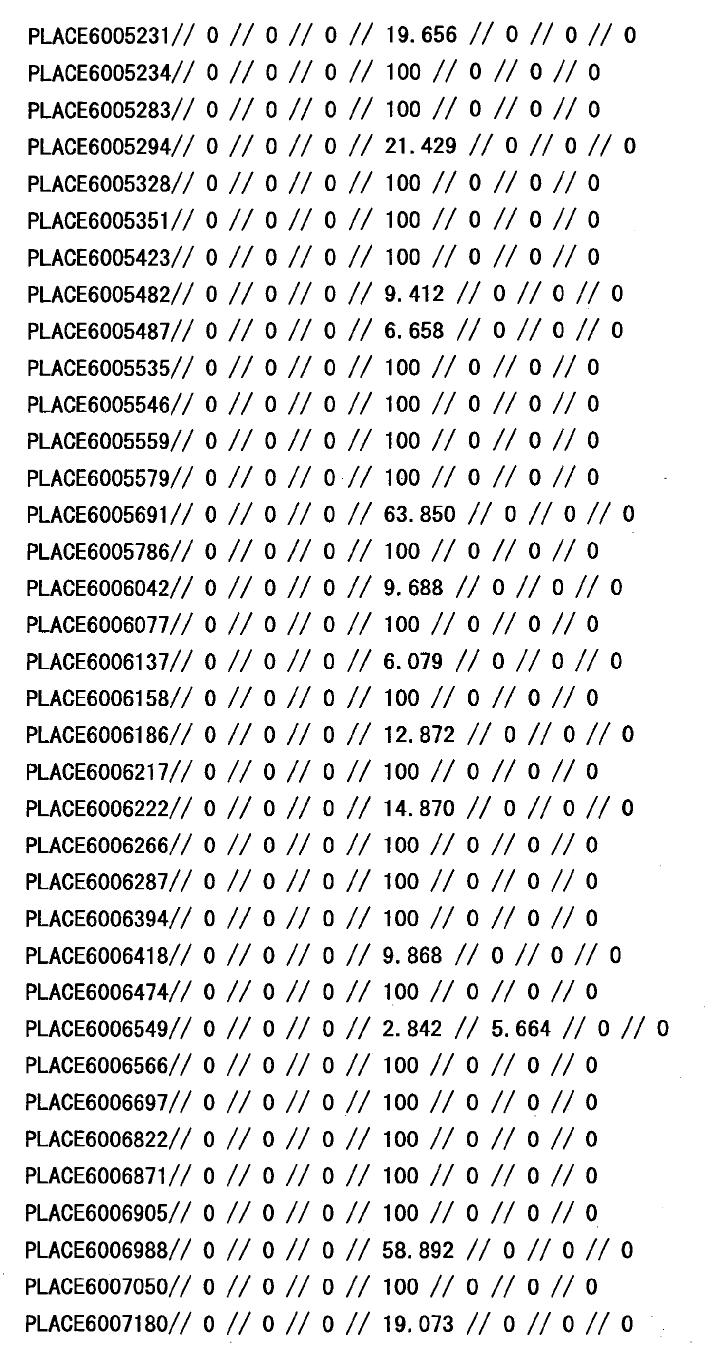

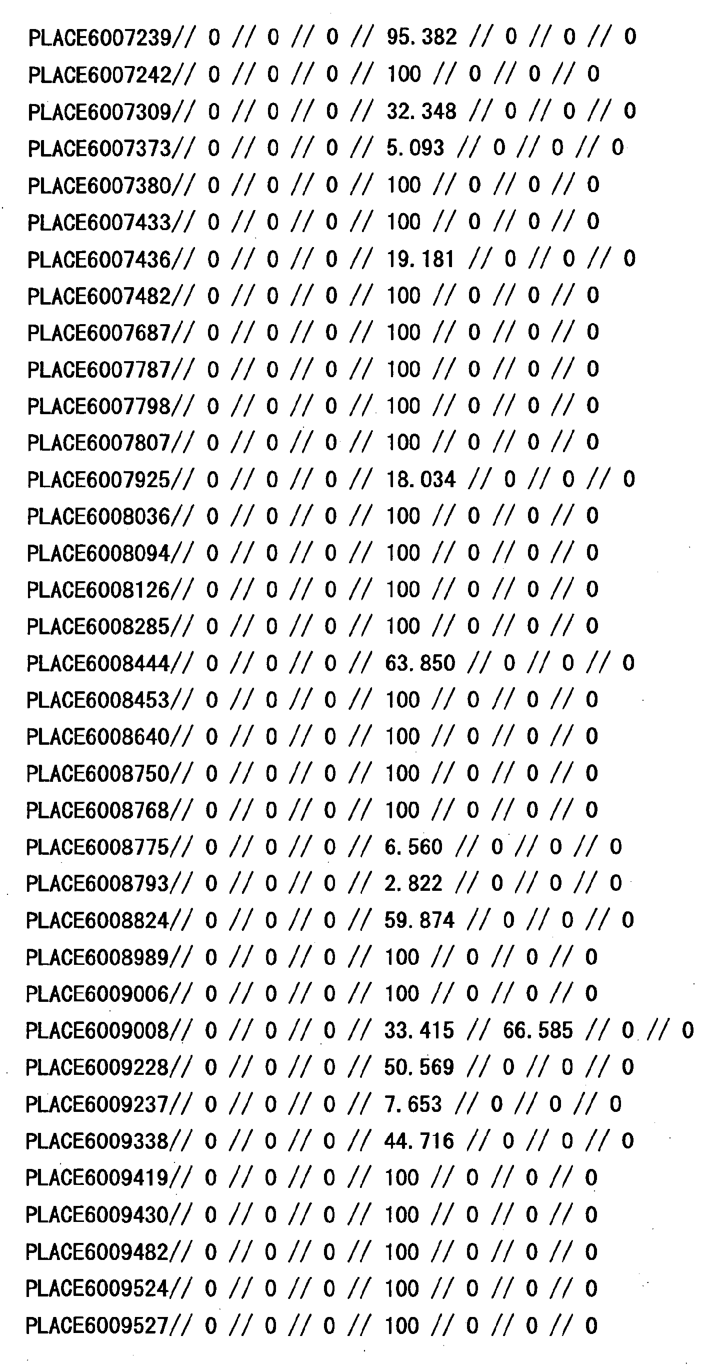

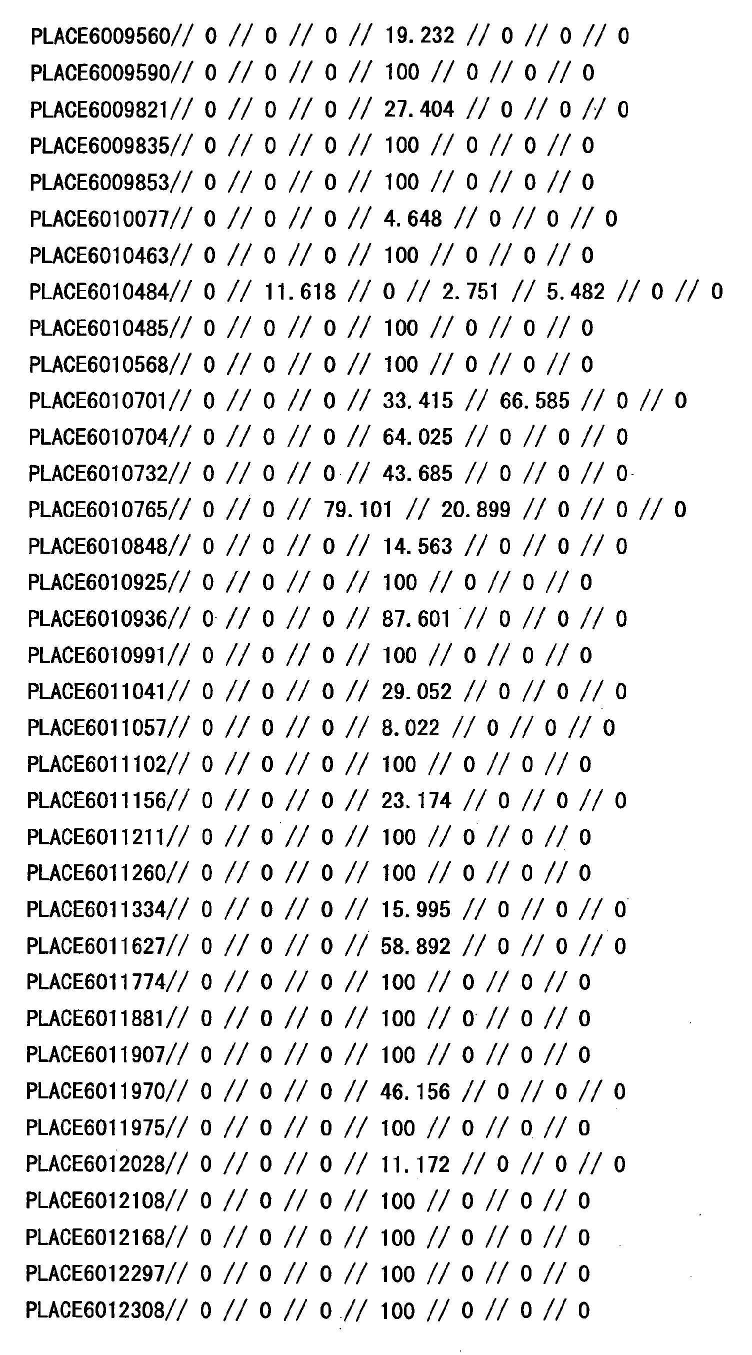

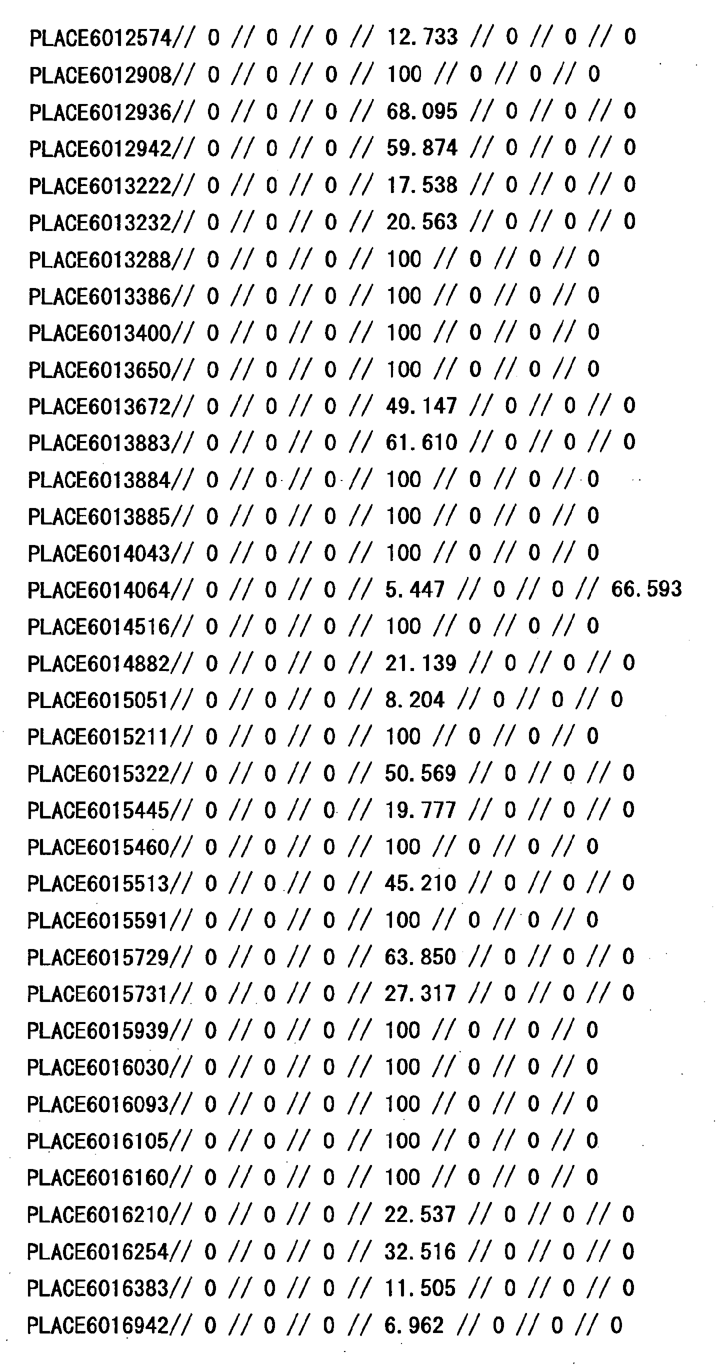

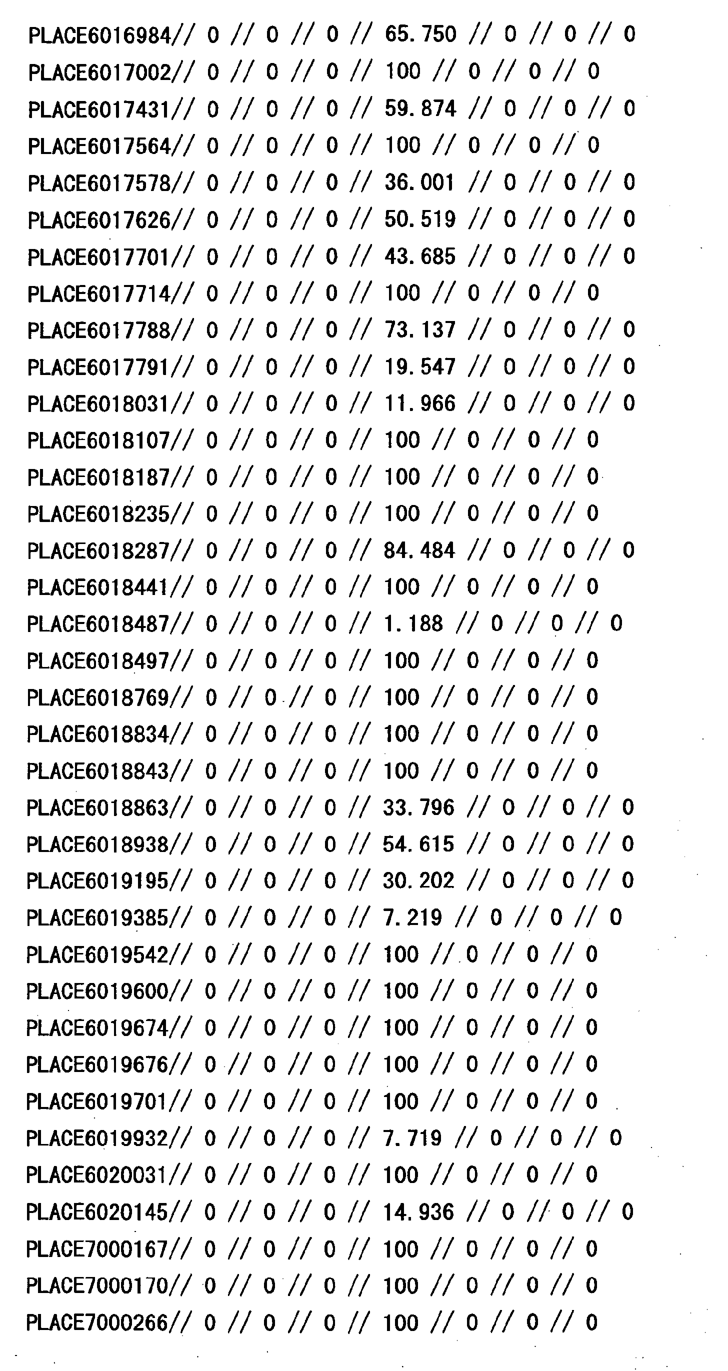

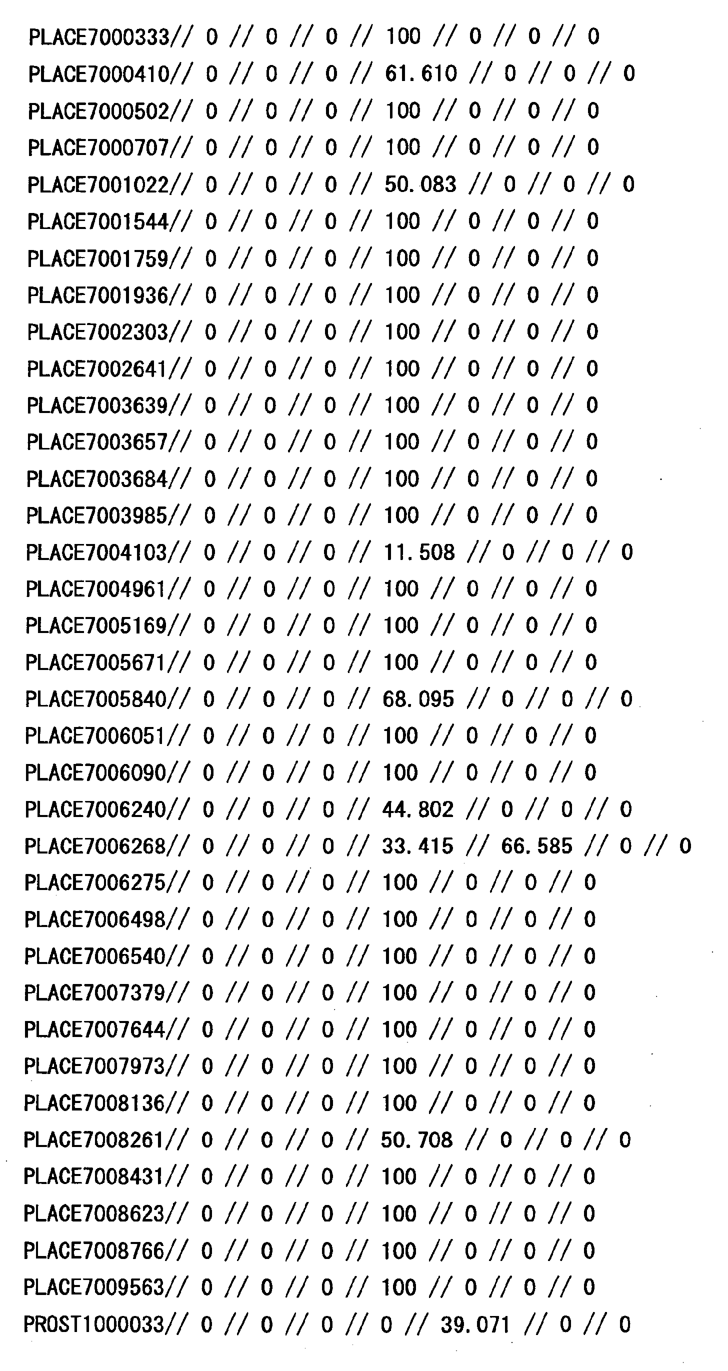

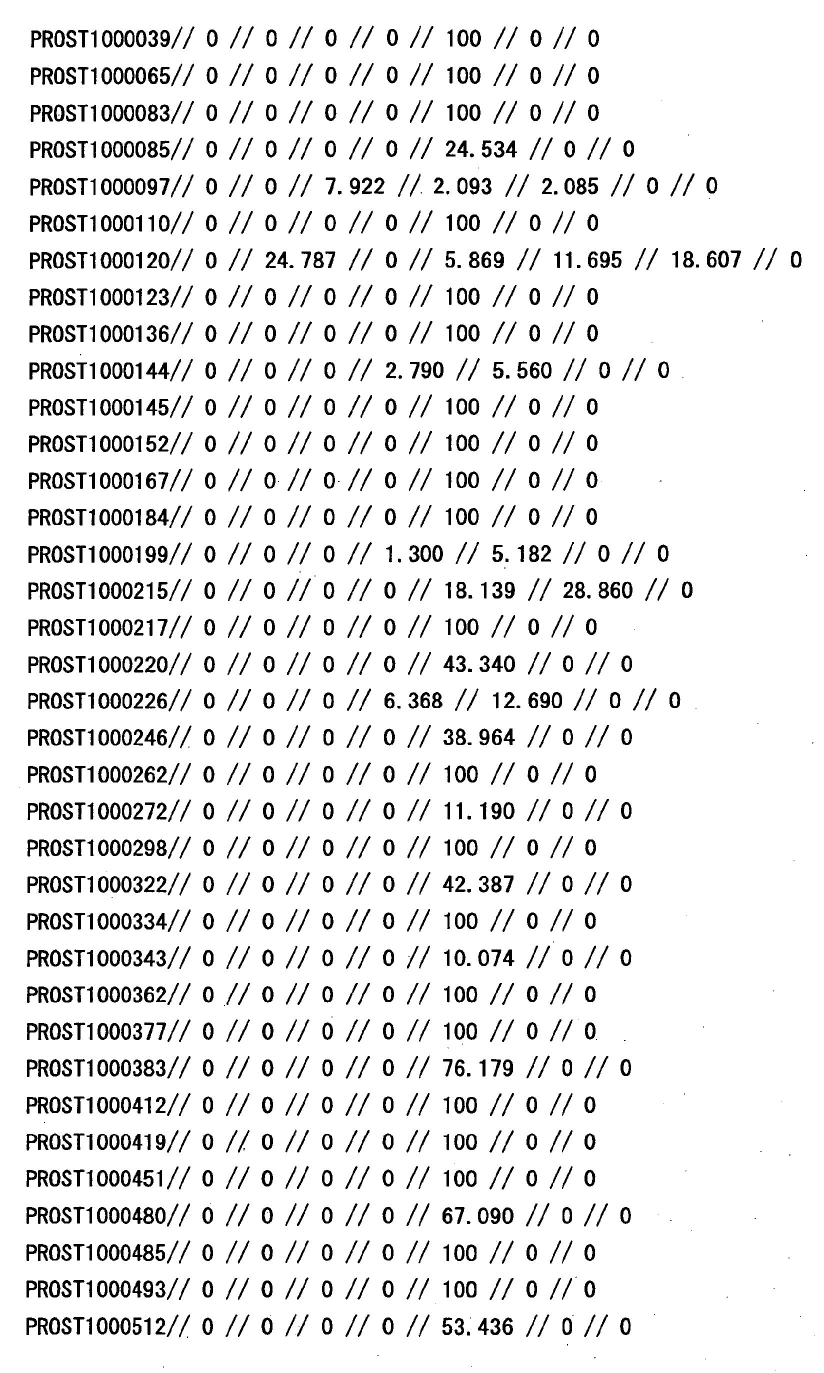

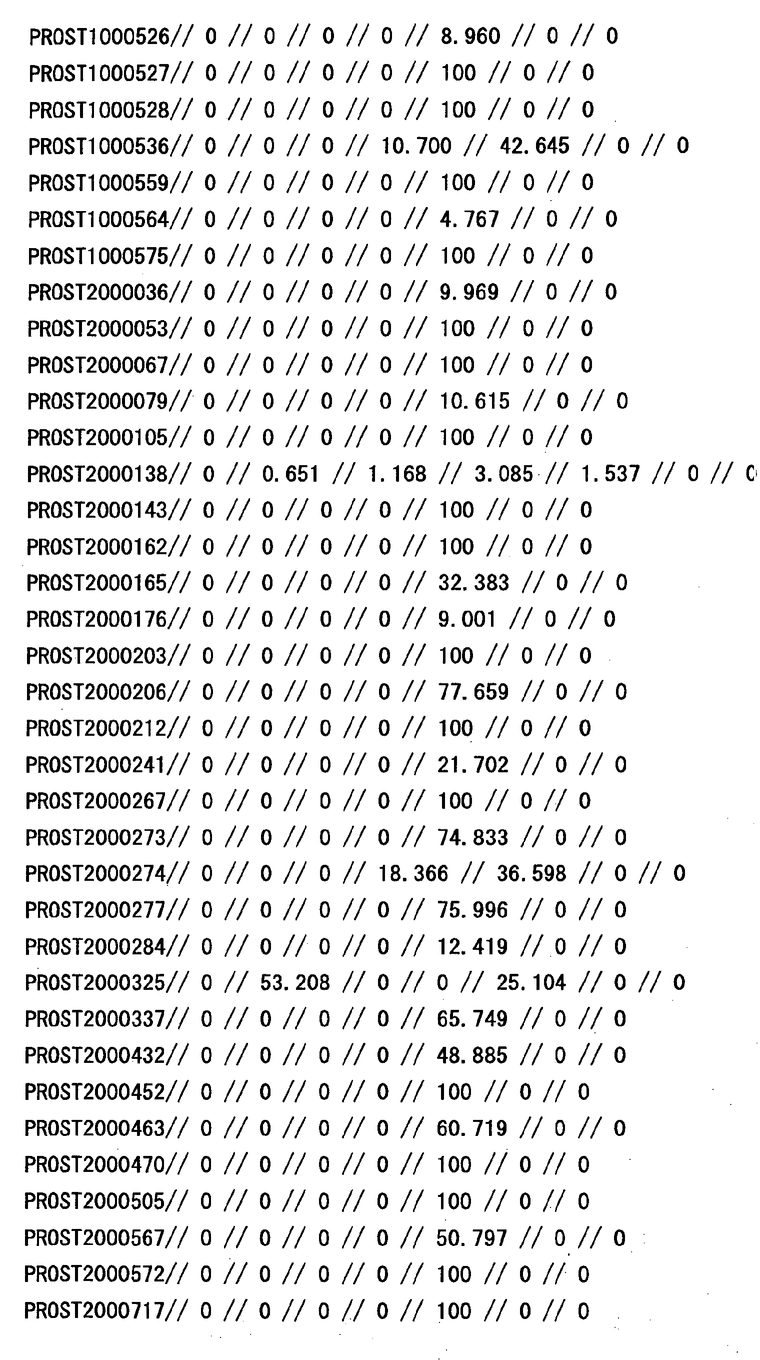

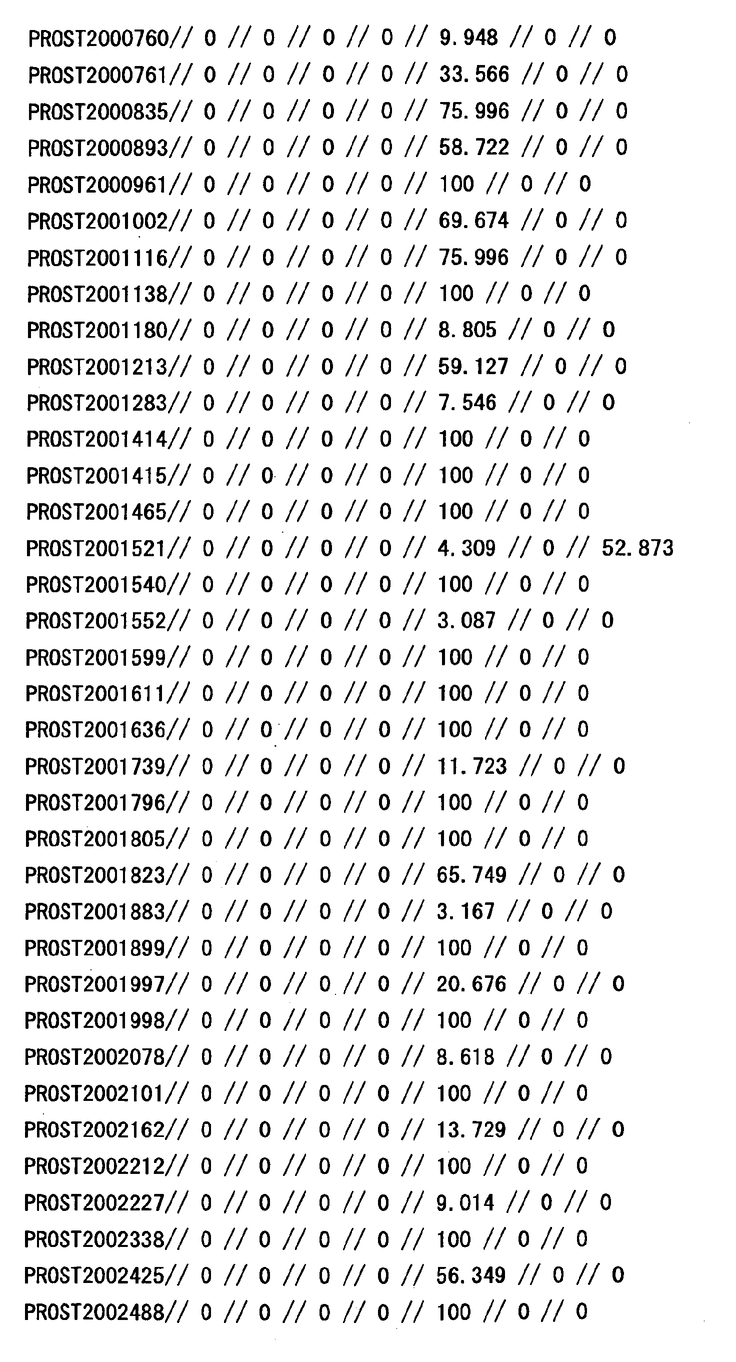

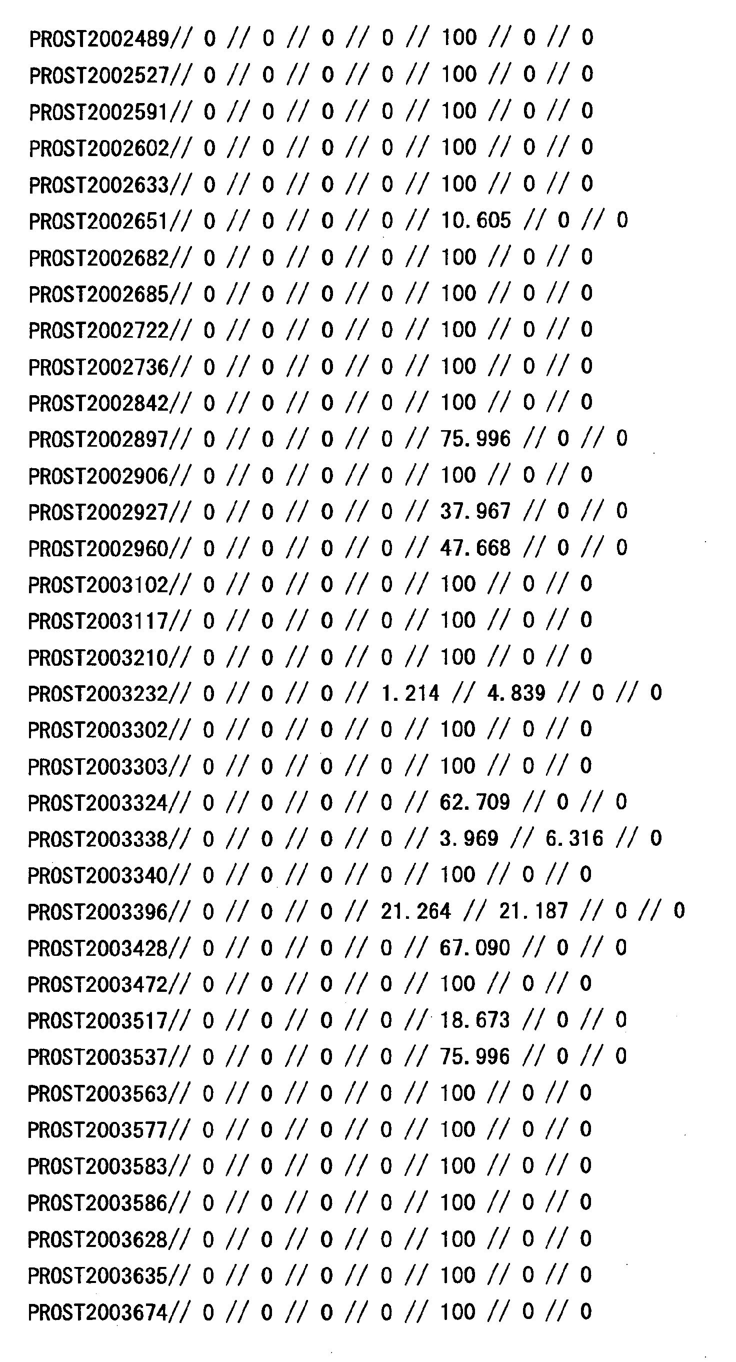

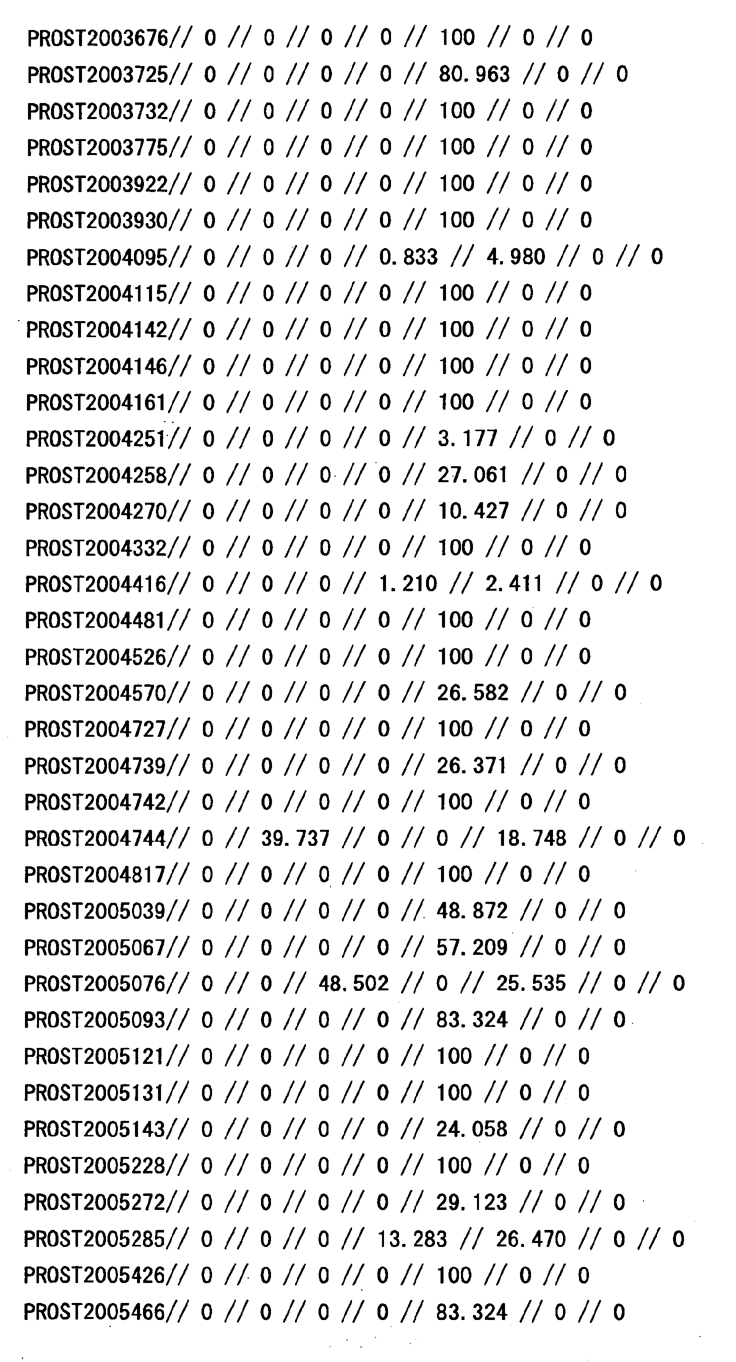

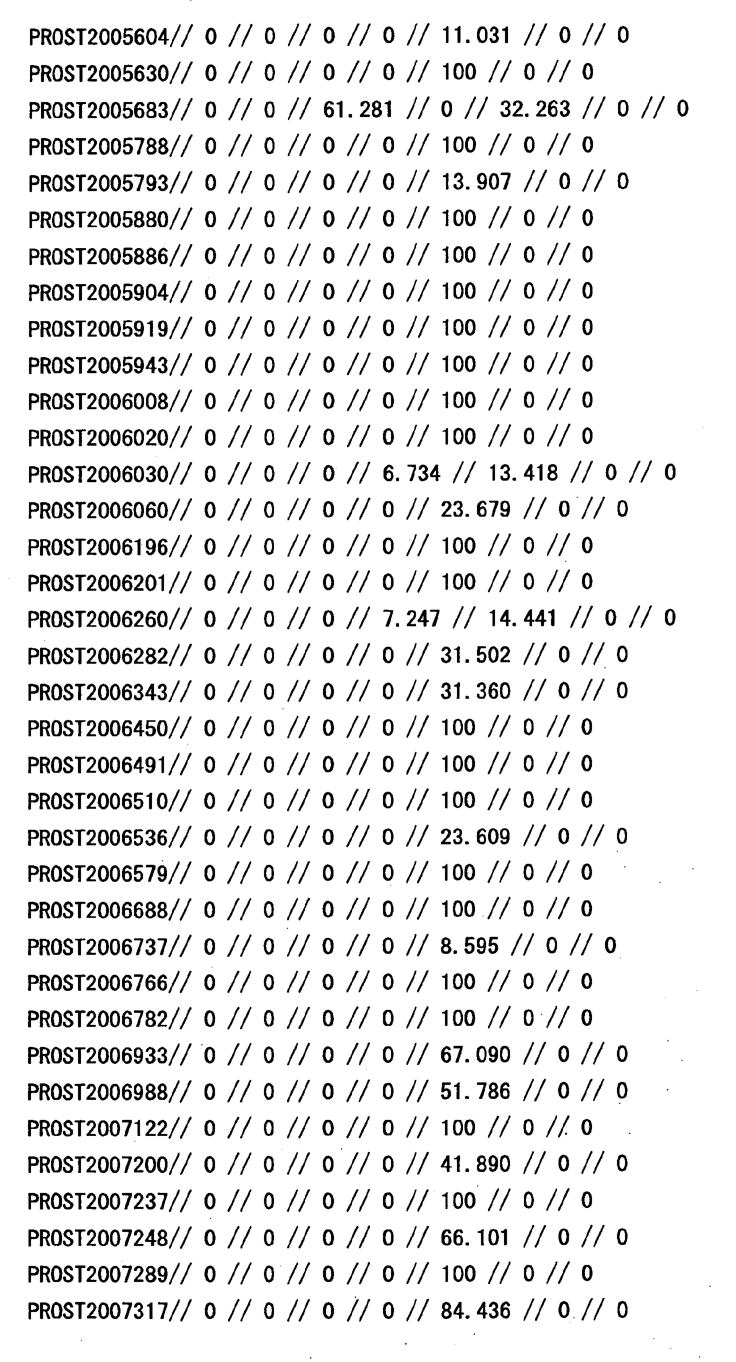

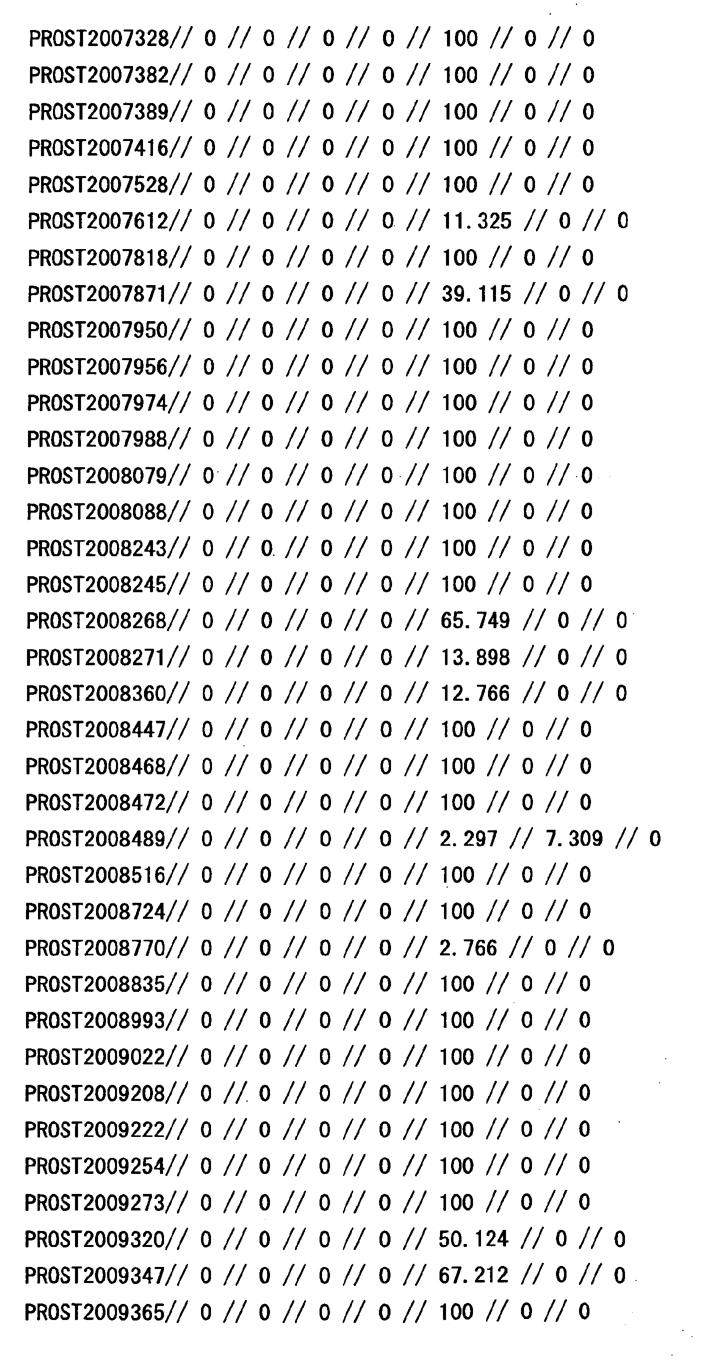

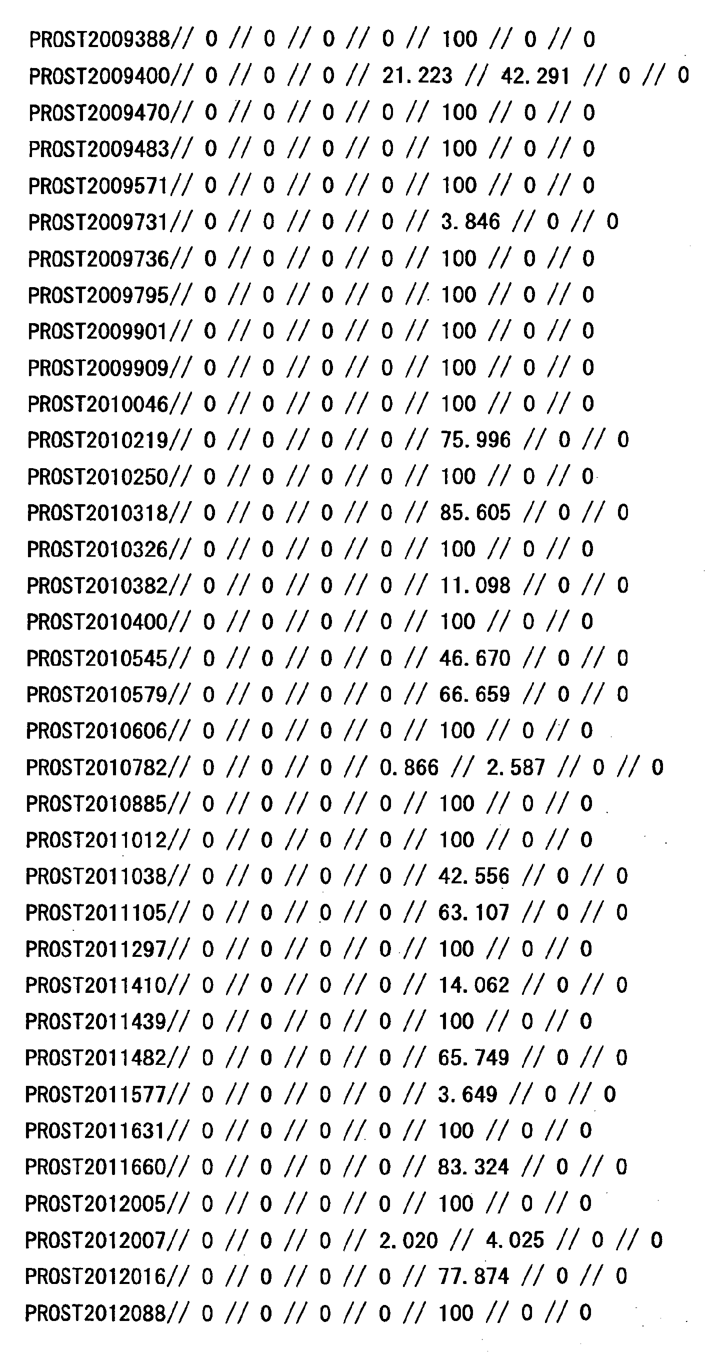

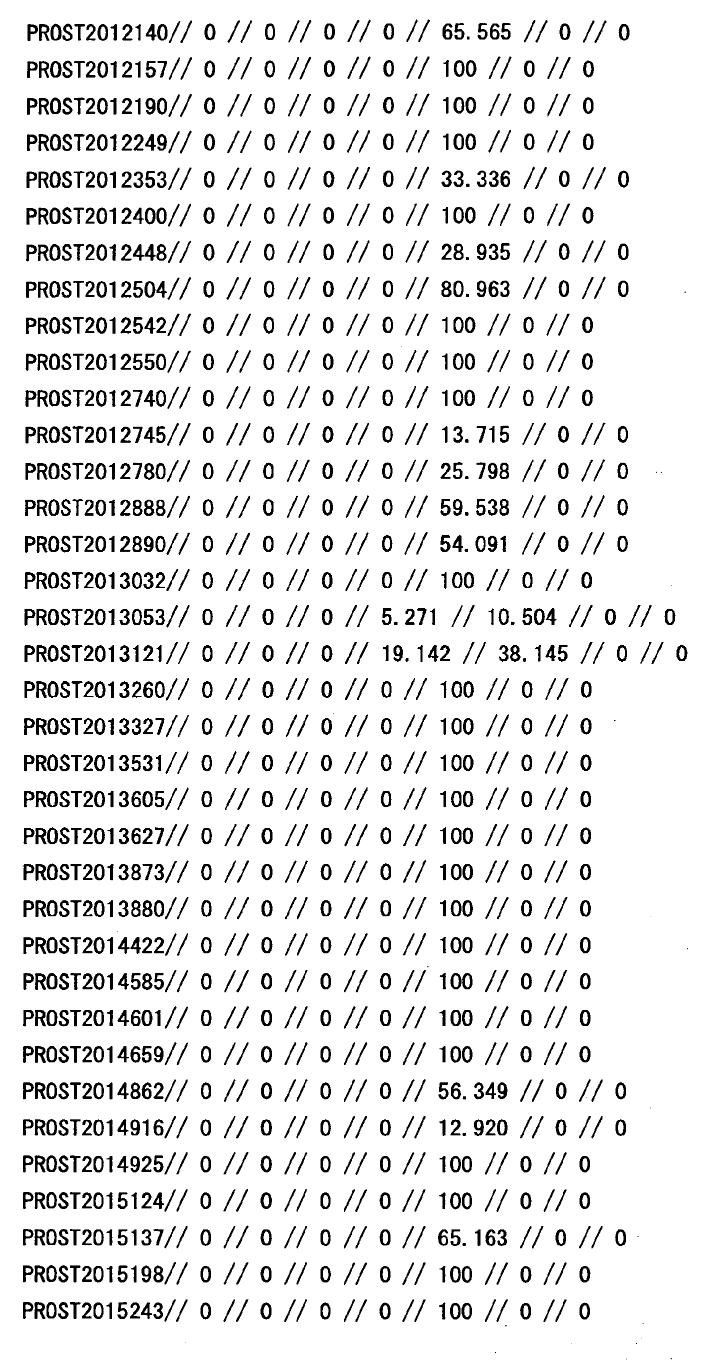

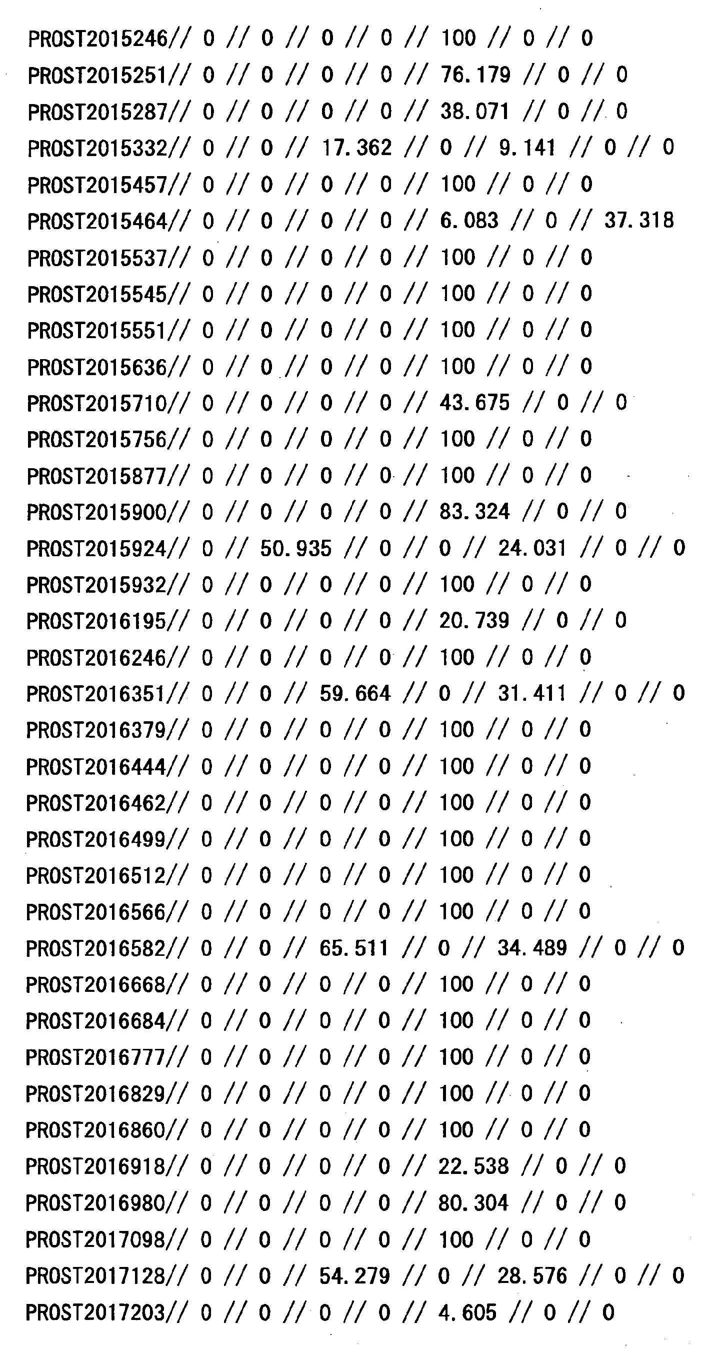

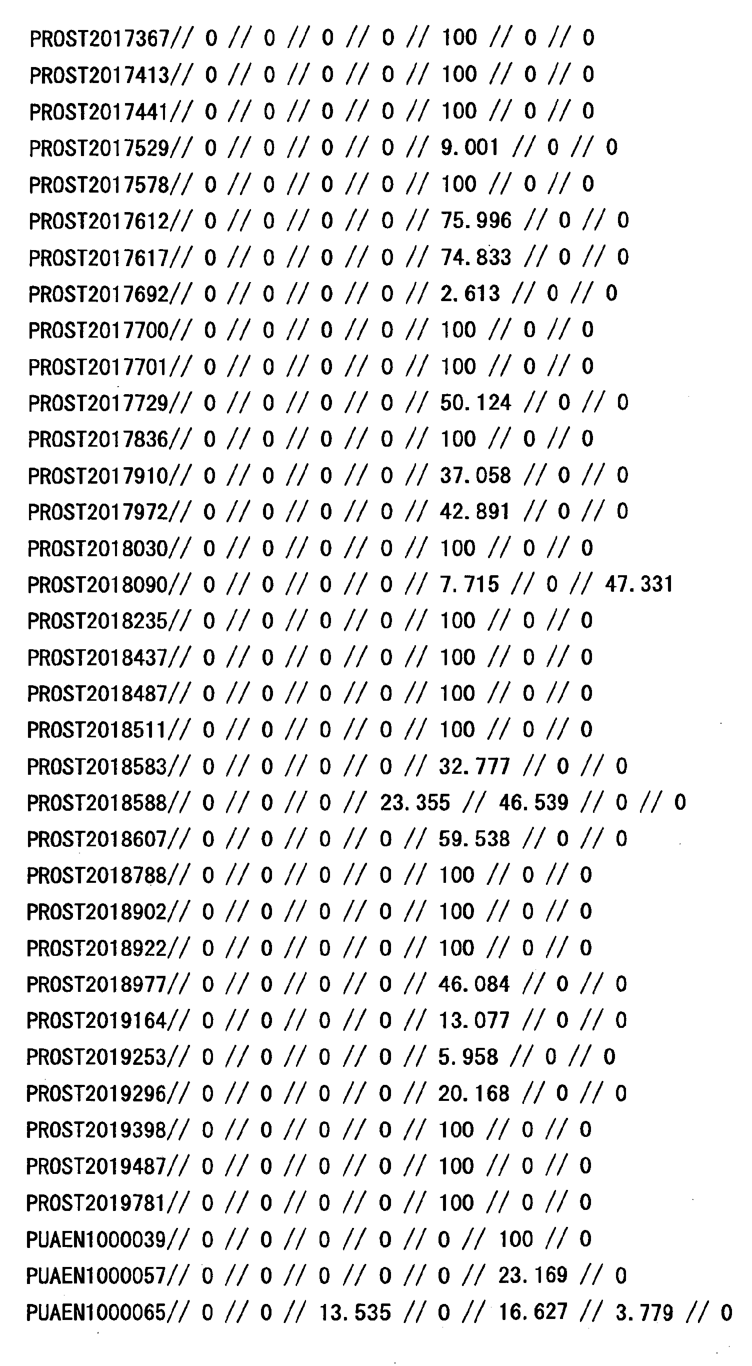

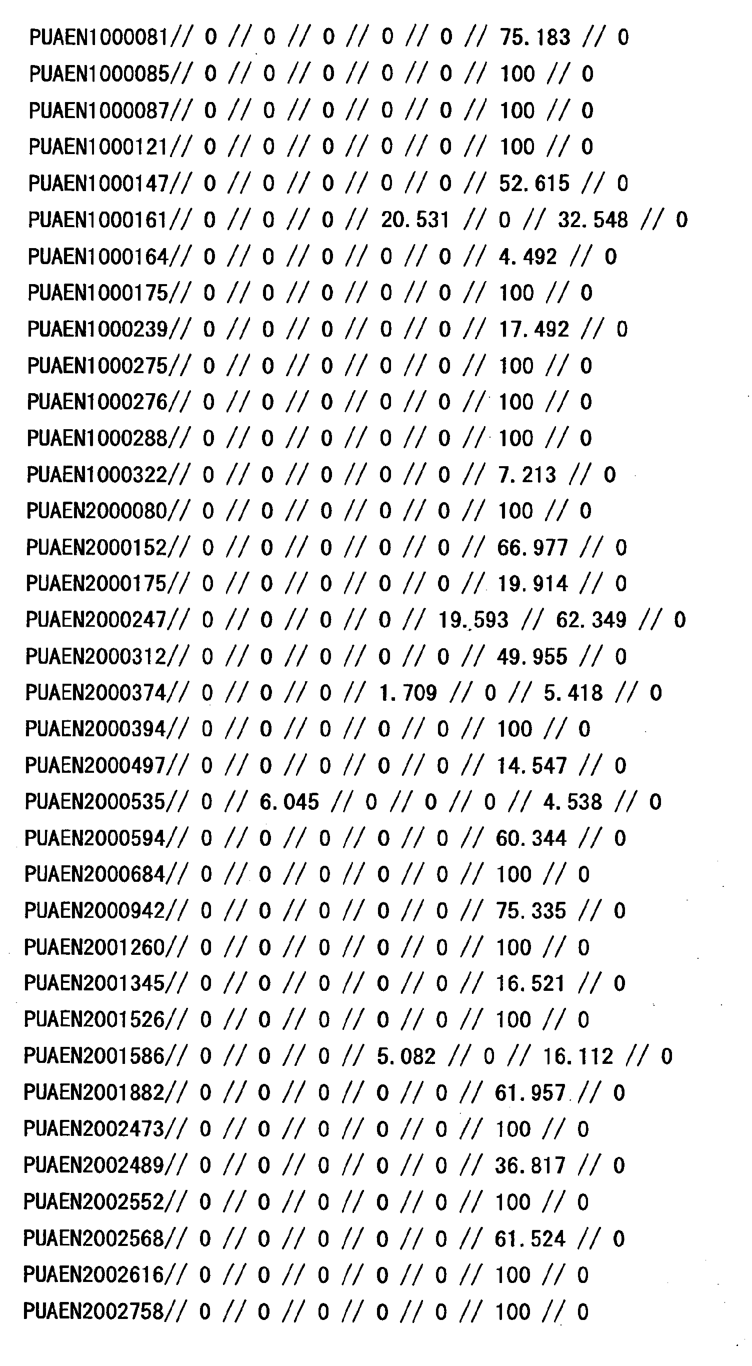

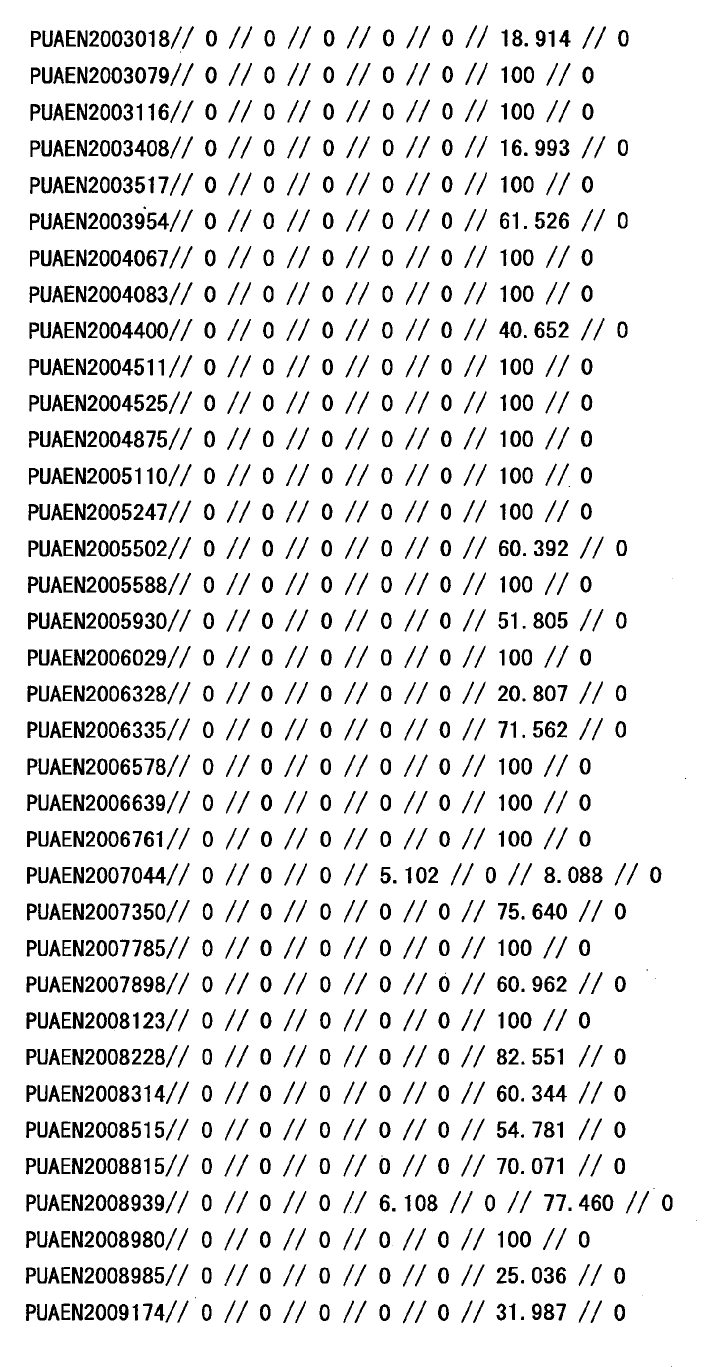

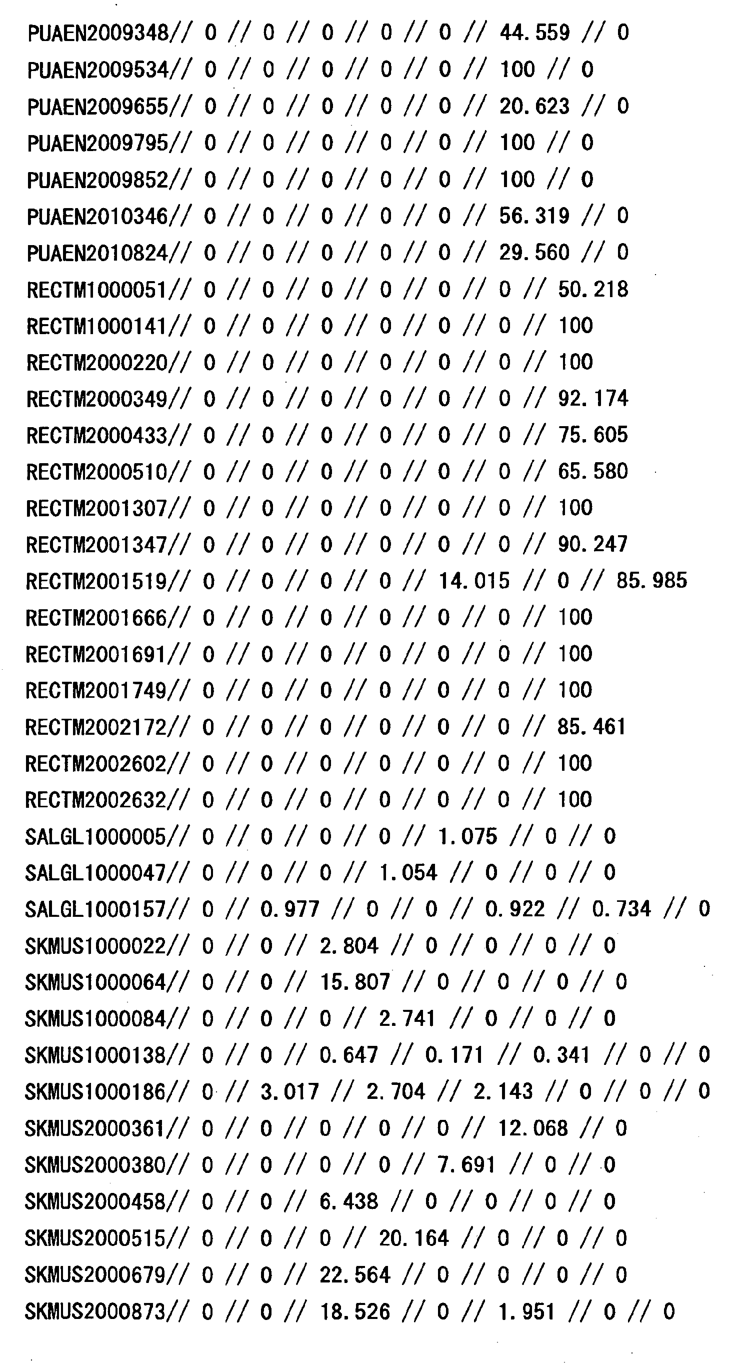

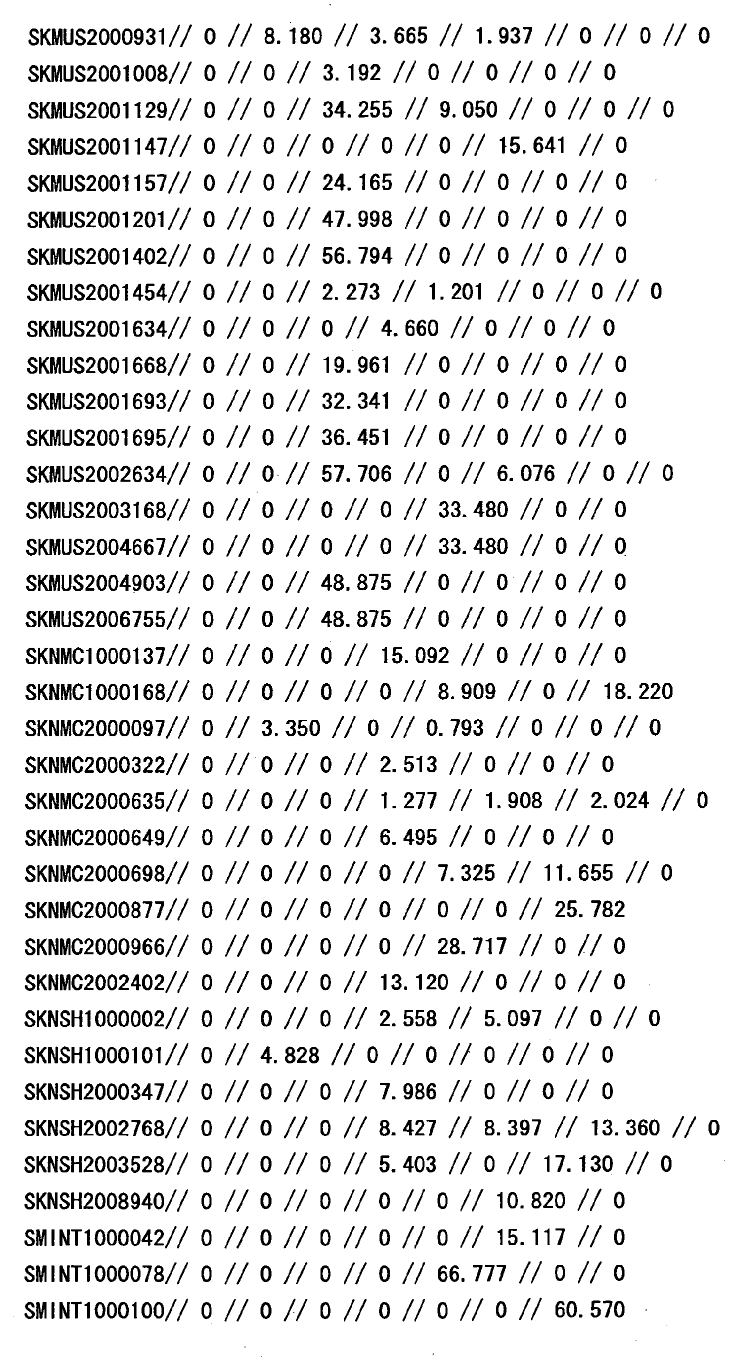

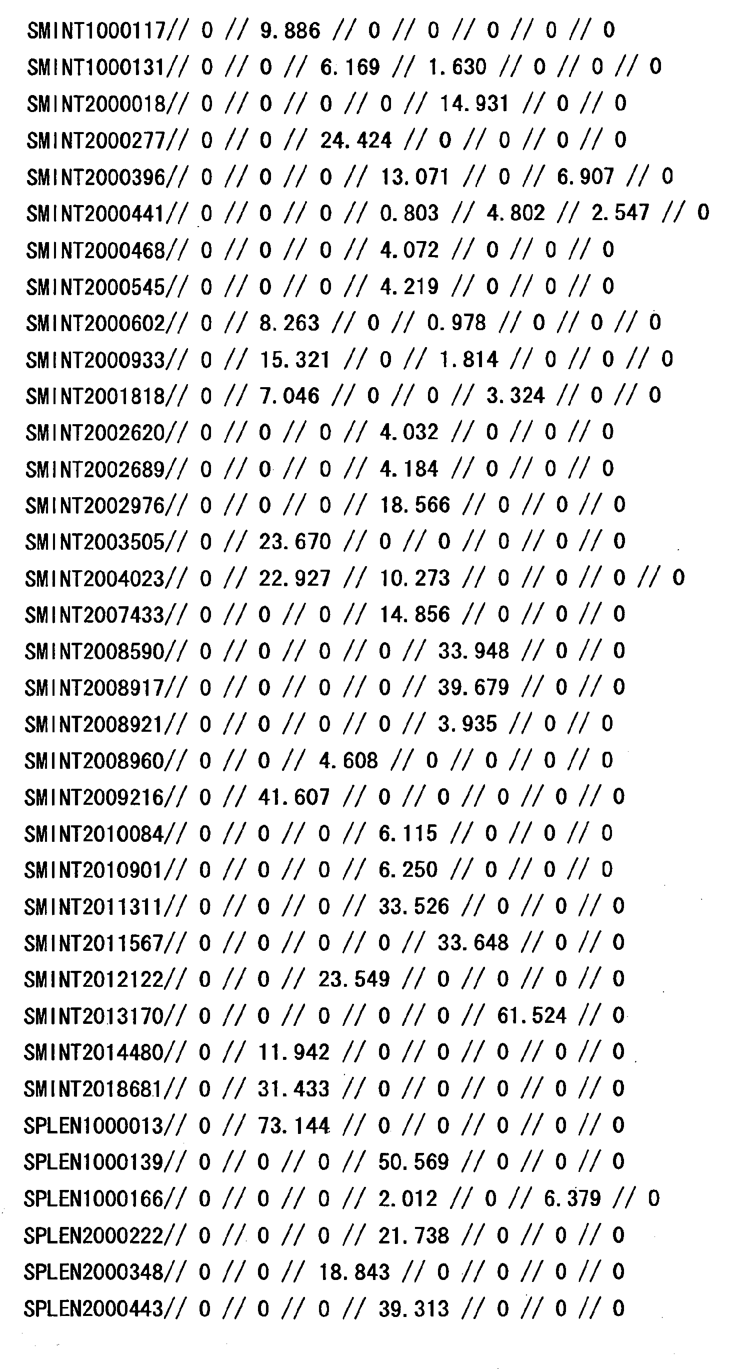

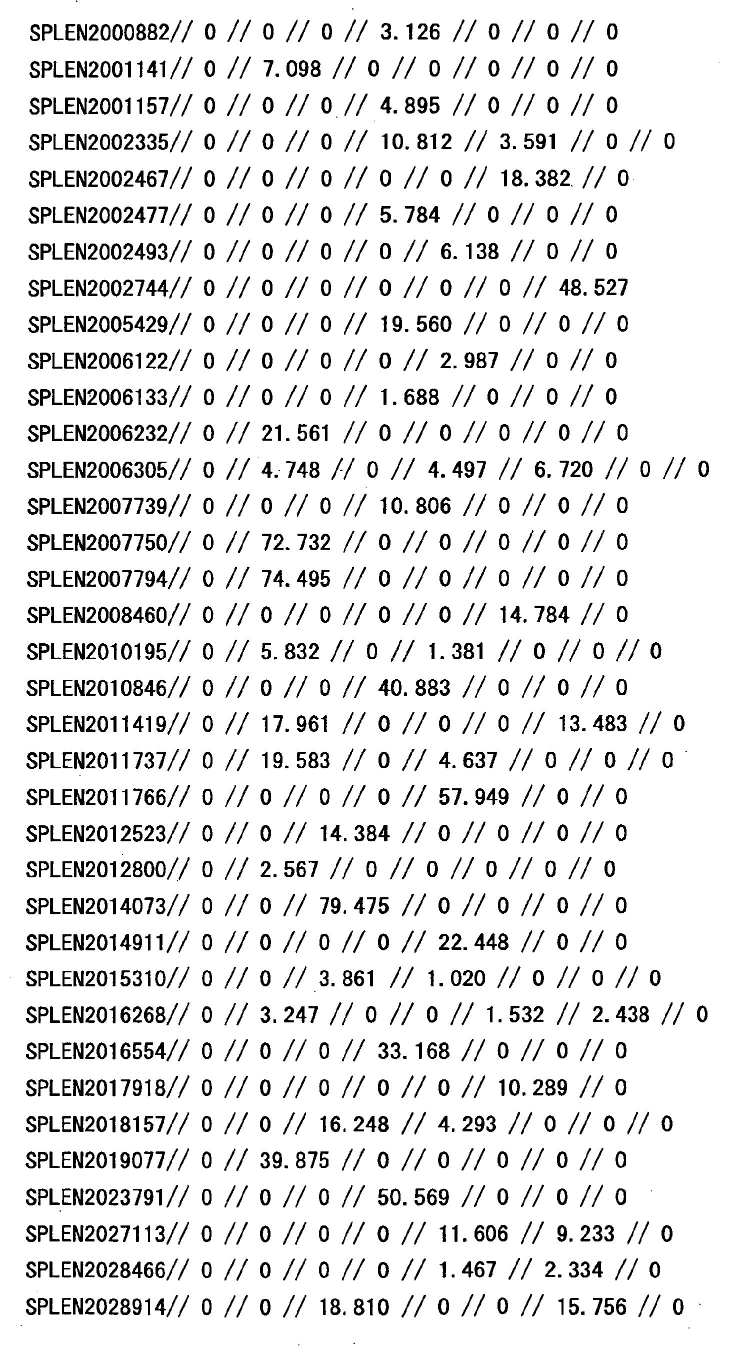

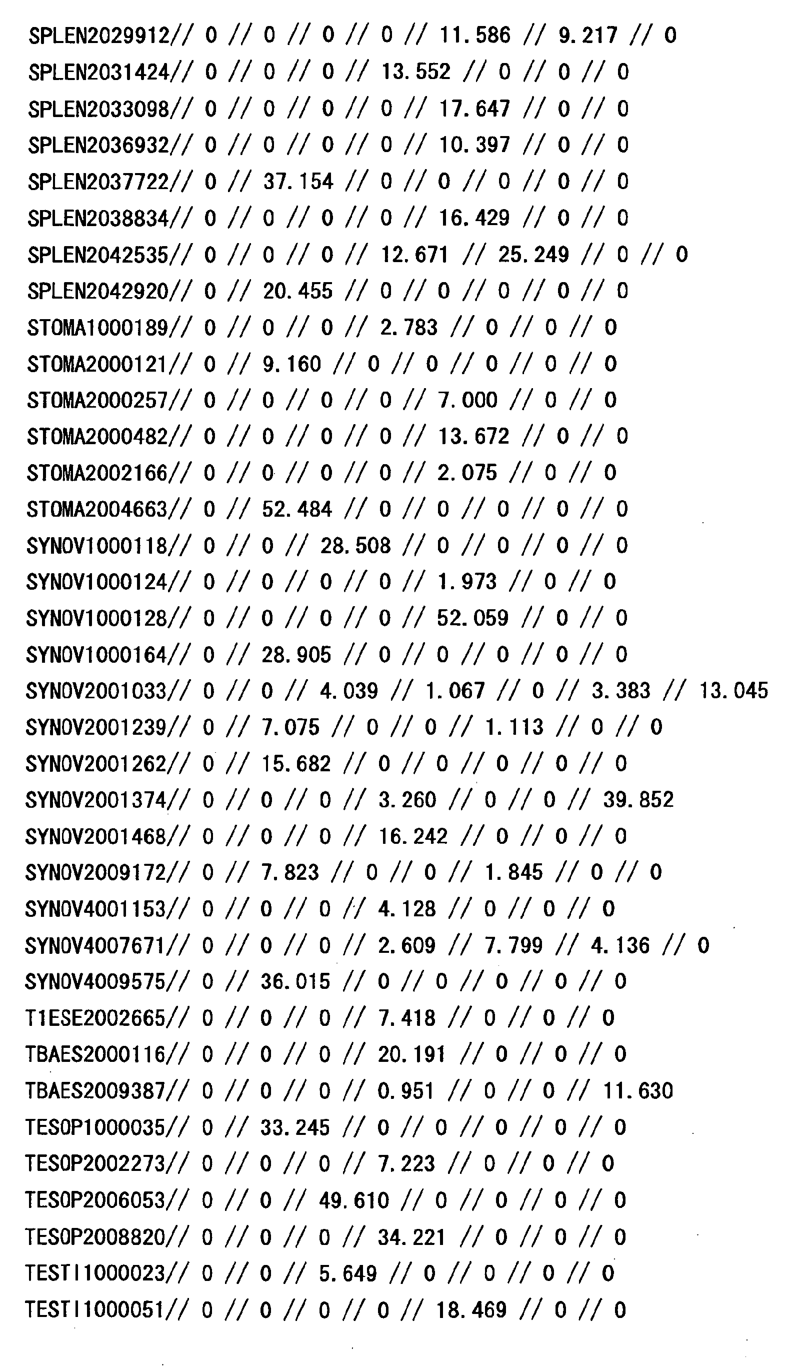

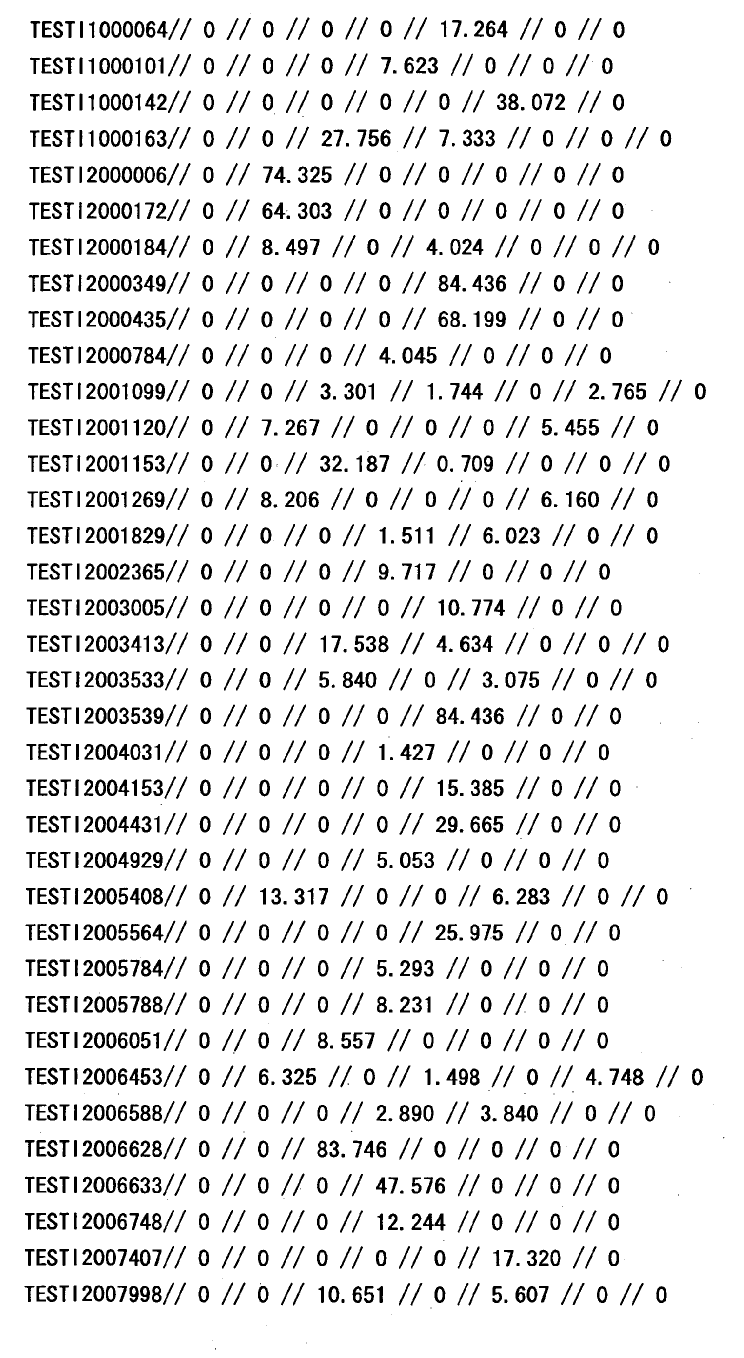

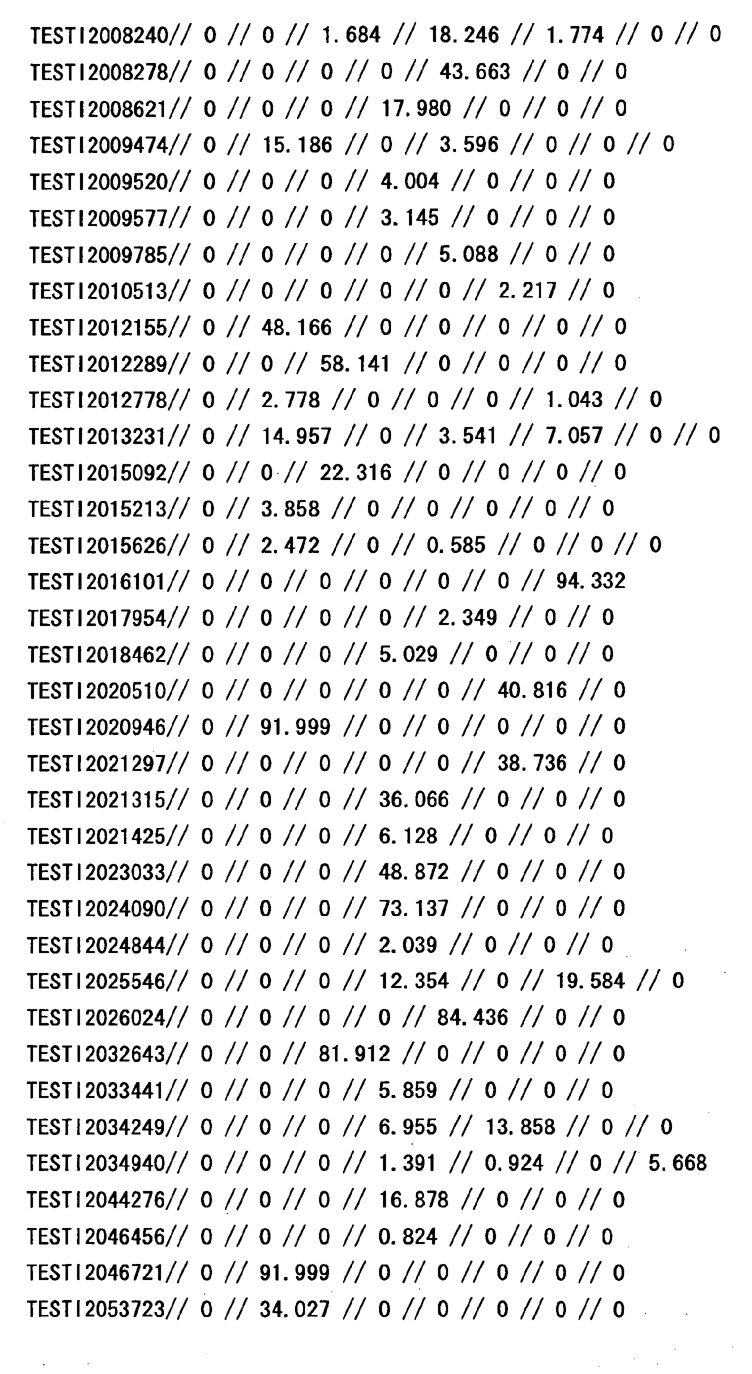

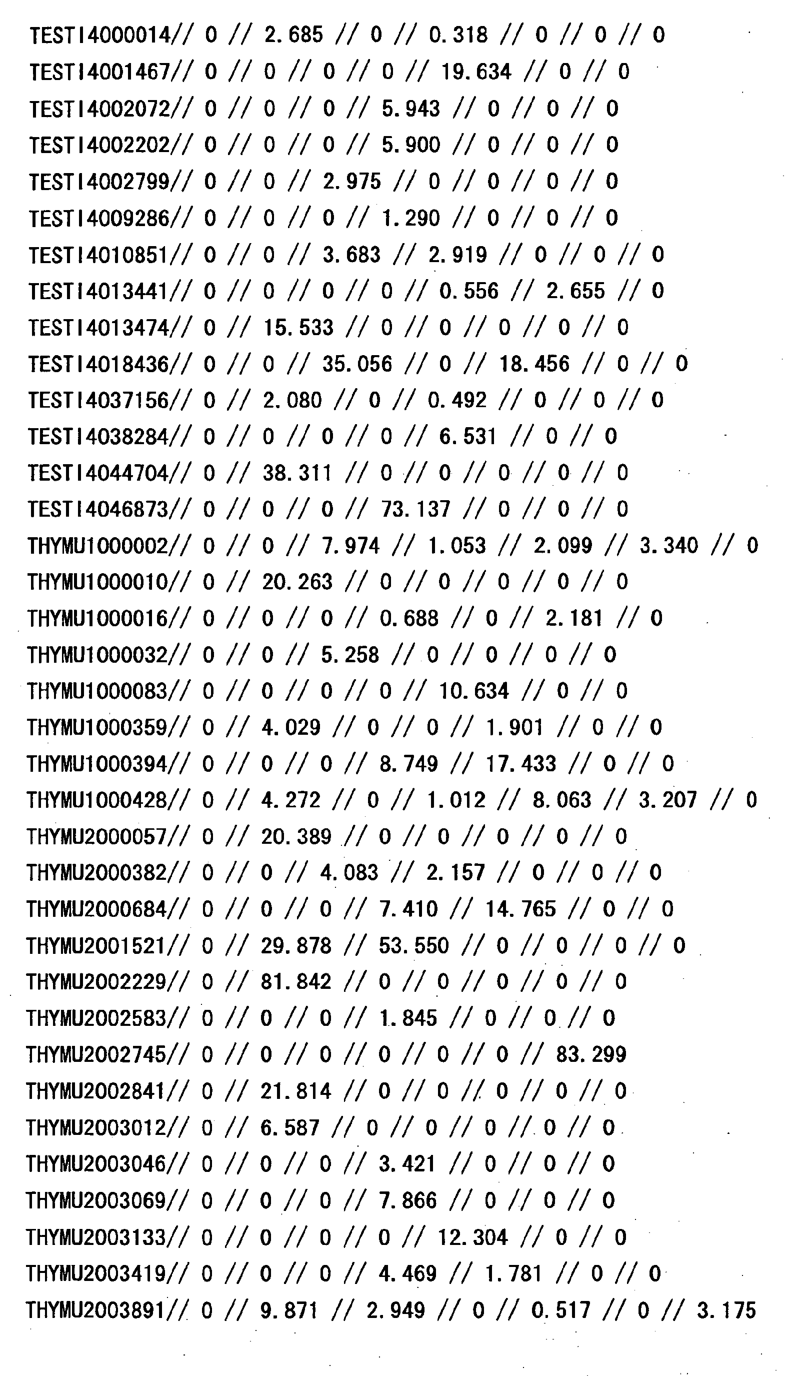

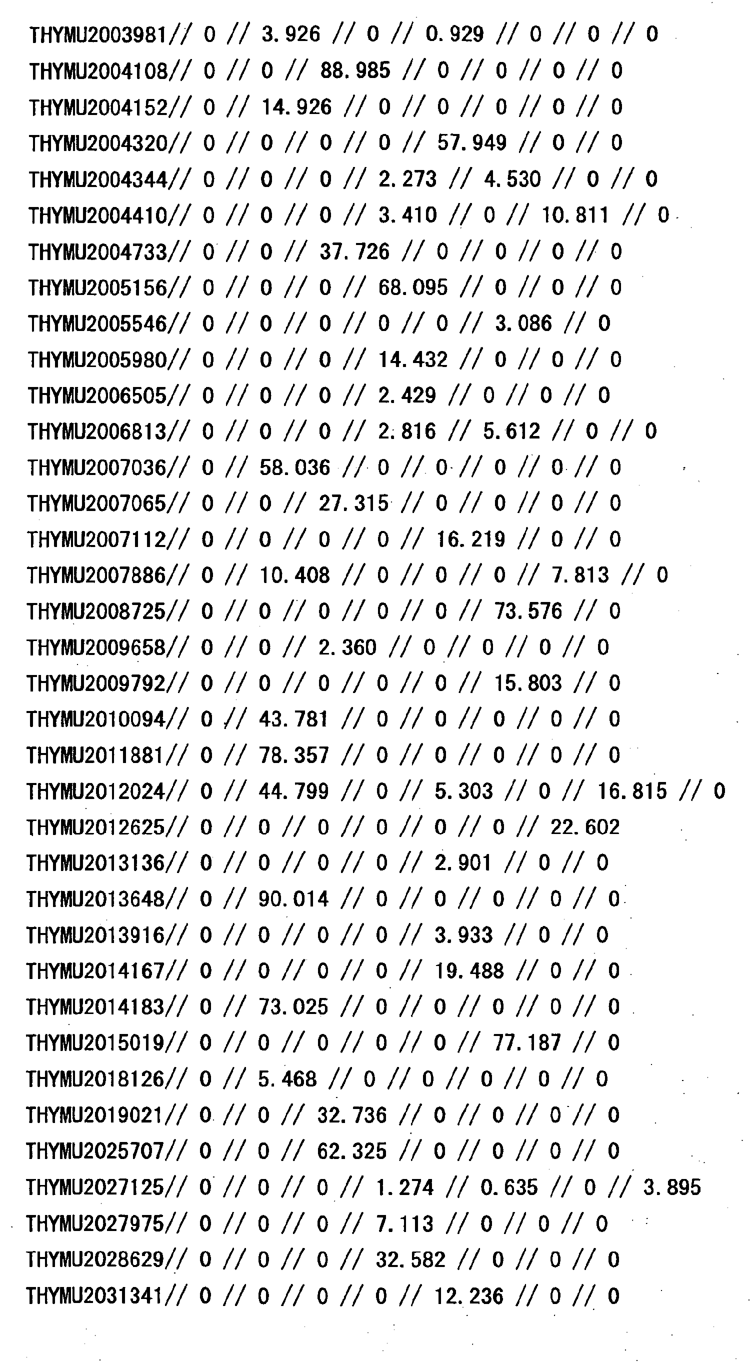

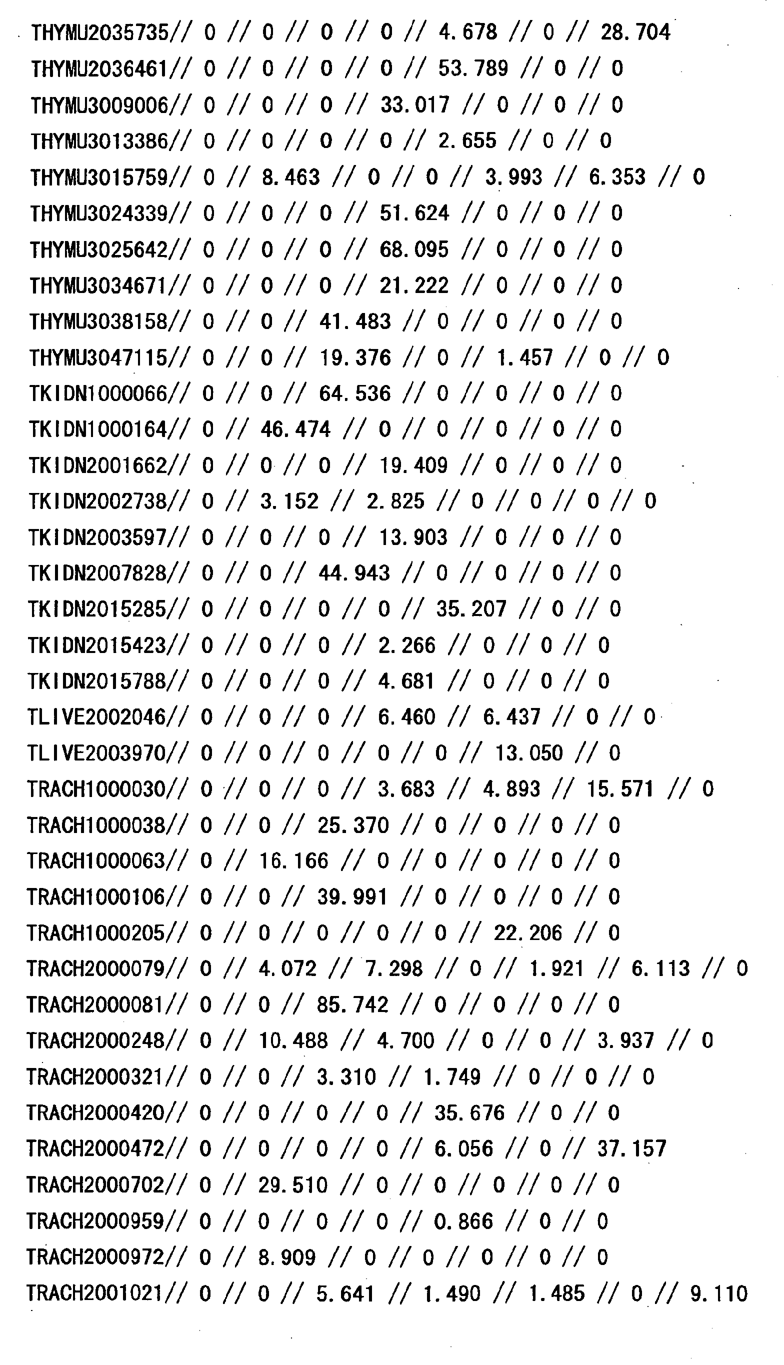

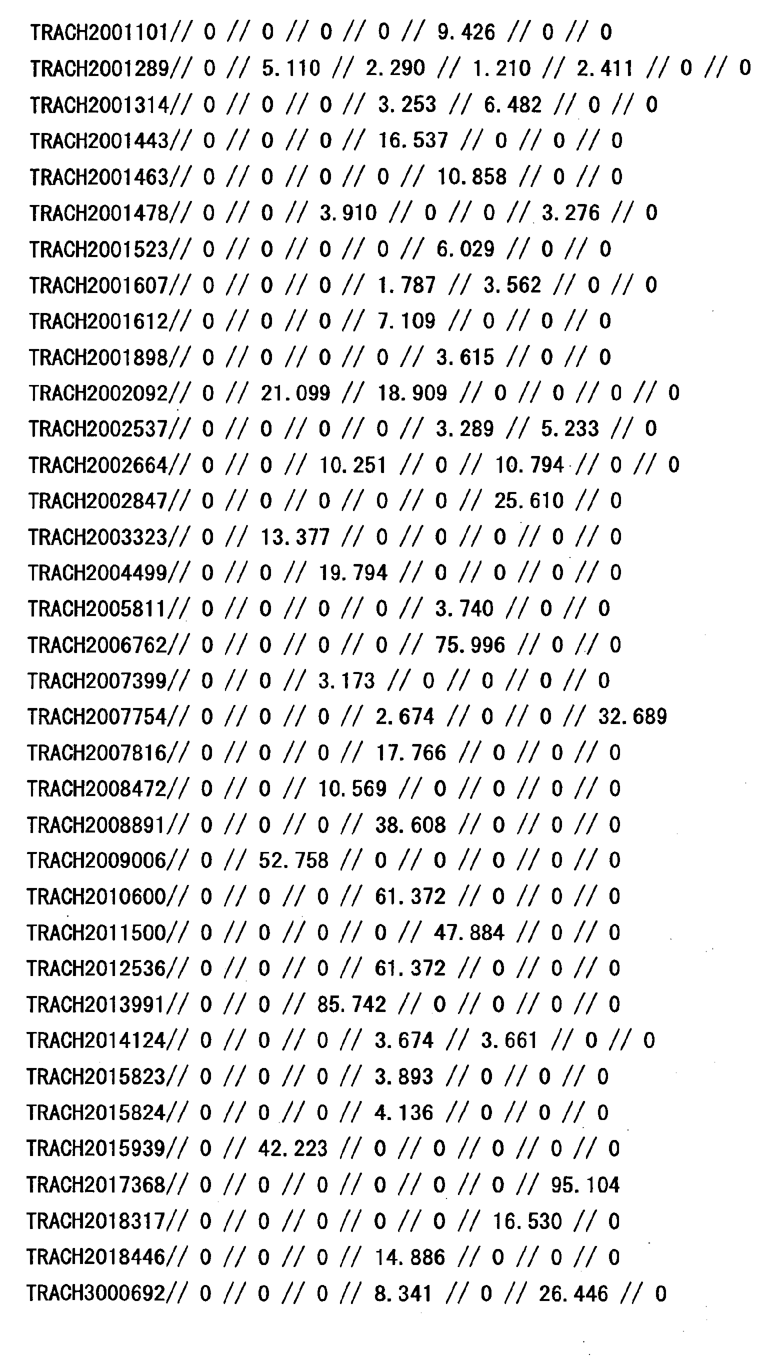

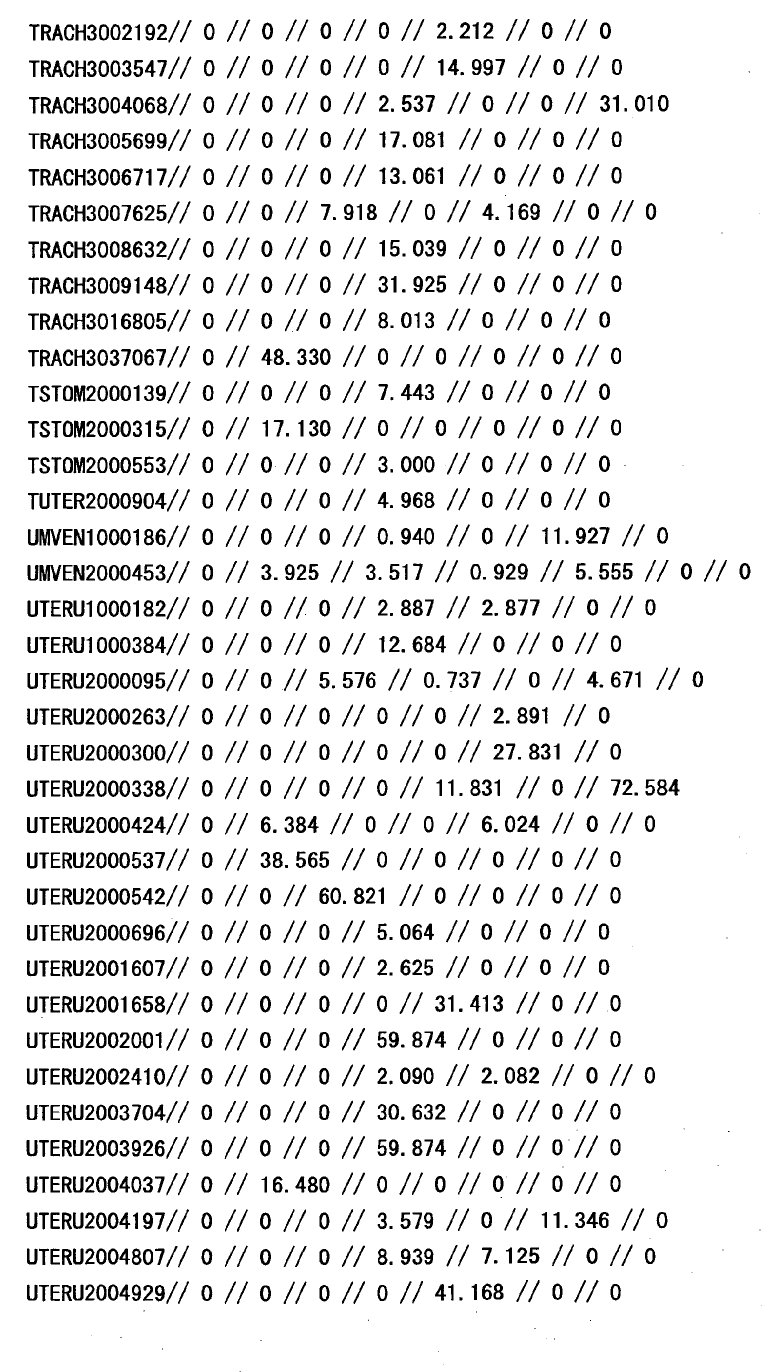

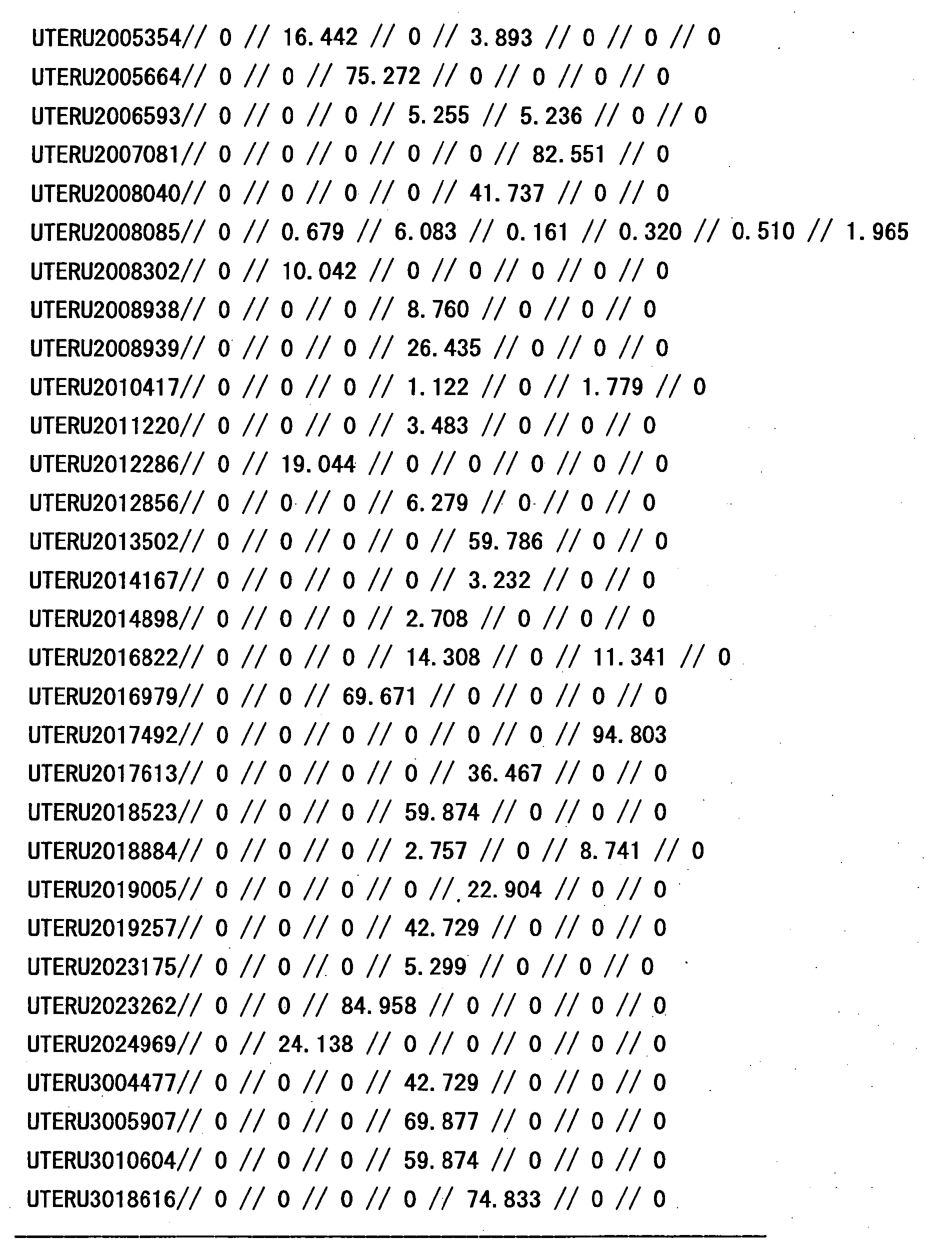

Genes whose expression levels were negligibly low in each of the above-described libraries are shown with their clone names below.
3NB691000018, 3NB691000113, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001548, 3NB692001637, 3NB692001719, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064, ADIPS2000069,
ADIPS2000088, ADIPS2000245, ADRGL1000002, ADRGL1000018, ADRGL1000033, ADRGL1000038, ADRGL1000065, ADRGL1000067, ADRGL1000111, ADRGL1000147, ADRGL1000160, ADRGL1000165, ADRGL1000182, ADRGL2000042, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679,
ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023,
ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000095, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2000801, ASTRO2001001, ASTRO2001008, ASTRO2001076, ASTRO2001088, ASTRO2001107, ASTRO2001122, ASTRO2001227, ASTRO2001249, ASTRO2001480, ASTRO2002024, ASTRO2002035,
ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003024, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004051, ASTRO2004177, ASTRO2004217, ASTRO2004297, ASTRO2004584, ASTRO2004751, ASTRO2004877, ASTRO2004974, ASTRO2005008, ASTRO2005081, ASTRO2005096, ASTRO2005273, ASTRO2005343, ASTRO2005360, ASTRO2005413, ASTRO2005423, ASTRO2005553, ASTRO2005557, ASTRO2005575, ASTRO2005593, ASTRO2005726, ASTRO2005896, ASTRO2005902, ASTRO2006356, ASTRO2006732, ASTRO2006747, ASTRO2006952, ASTRO2007047,
ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO-2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014576, ASTRO2014863, ASTRO2014923,
ASTRO2014961, ASTRO2015162, ASTRO2015185, ASTRO2015328, ASTRO2015529, ASTRO2016044, ASTRO2016148, ASTRO2016681, ASTRO2016819, ASTRO2016847, ASTRO2016892, ASTRO2017348, ASTRO2017627, ASTRO2018169, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000544, BGGI12000684, BGGI12000693, BGGI12001002, BGGI12001075, BGGI12001241, BGGI12001247, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031,
BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007666, BLADE2007722, BLADE2007735, BLADE2007744, BLADE2007799, BLADE2007923, BLADE2007935, BLADE2007958,
BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000118, BNGH41000137, BNGH41000169, BNGH41000177, BNGH41000198, BNGH41000216, BNGH41000235, BNGH42000130, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002168, BNGH42002244, BNGH42002387, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007366, BNGH42007594, BNGH42007798,
BNGH42008199, BNGH42008510, BNGH42008649, BNGH42008743, BNGH42008832, BNGH42008943, BNGH42009021, BRACE1000020, BRACE1000047, BRACE1000051, BRACE1000065, BRACE1000073, BRACE1000084, BRACE1000087, BRACE1000092, BRACE1000093, BRACE1000101, BRACE1000114, BRACE1000124, BRACE1000166, BRACE1000169, BRACE1000187, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000347, BRACE2000351, BRACE2000368, BRACE2000392, BRACE2000441,
BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000753, BRACE2000815, BRACE2000864, BRACE2000885, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001151, BRACE2001154, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374,
BRACE2001375, BRACE2001423, BRACE2001424, BRACE2001445, BRACE2001453, BRACE2001455, BRACE2001492, BRACE2001496, BRACE2001534, BRACE2001543, BRACE2001564, BRACE2001588, BRACE2001607, BRACE2001626, BRACE2001677, BRACE2001692, BRACE2001705, BRACE2001709, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002050, BRACE2002081, BRACE2002139, BRACE2002142, BRACE2002151, BRACE2002157, BRACE2002176, BRACE2002202, BRACE2002206,
BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002613, BRACE2002614, BRACE2002617, BRACE2002623, BRACE2002635, BRACE2002649, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002884, BRACE2002896, BRACE2002948, BRACE2003037, BRACE2003078, BRACE2003097, BRACE2003110,
BRACE2003168, BRACE2003199, BRACE2003210, BRACE2003269, BRACE2003285, BRACE2003304, BRACE2003319, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003639, BRACE2003700, BRACE2003713, BRACE2003766, BRACE2003785, BRACE2003788, BRACE2003800, BRACE2003802, BRACE2003825, BRACE2003845, BRACE2003846, BRACE2003847, BRACE2003848, BRACE2003873, BRACE2003885, BRACE2003887, BRACE2003892, BRACE2003904, BRACE2003905, BRACE2003928, BRACE2003954, BRACE2003982, BRACE2003987, BRACE2005034, BRACE2005047, BRACE2005063, BRACE2005083, BRACE2005090, BRACE2005099, BRACE2005118,
BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005433, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898,
BRACE2005904, BRACE2005906, BRACE2005907, BRACE2005911, BRACE2005949, BRACE2005981, BRACE2005991, BRACE2006055, BRACE2006072, BRACE2006075, BRACE2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006274, BRACE2006281, BRACE2006298, BRACE2006344, BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006540, BRACE2006564, BRACE2006587, BRACE2006598, BRACE2006636, BRACE2006685, BRACE2006703, BRACE2006743, BRACE2006783, BRACE2006821, BRACE2006833, BRACE2006859, BRACE2006871,
BRACE2006909, BRACE2006911, BRACE2006924, BRACE2006935, BRACE2006937, BRACE2006944, BRACE2006951, BRACE2006982, BRACE2006986, BRACE2007013, BRACE2007064, BRACE2007068, BRACE2007087, BRACE2007109, BRACE2007138, BRACE2007174, BRACE2007197, BRACE2007201, BRACE2007203, BRACE2007232, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646, BRACE2007663, BRACE2007682, BRACE2007685, BRACE2007708, BRACE2007727, BRACE2007749, BRACE2007760, BRACE2007761,
BRACE2007764, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594,
BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008948, BRACE2008960, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421, BRACE2009428, BRACE2009434, BRACE2009437,
BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646,
BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012528, BRACE2012625, BRACE2012653, BRACE2012814, BRACE2012833, BRACE2012838, BRACE2012936, BRACE 2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132,
BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016362, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847,
BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158,
BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024627, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294,
BRACE2026404, BRACE2026675, BRACE2026725, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148,
BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170,
BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300,
BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002298, BRACE3002344, BRACE3002390, BRACE3002420, BRACE3002508, BRACE3002541, BRACE3002756, BRACE3003004, BRACE3003053, BRACE3003192, BRACE3003413, BRACE3003595,
BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004862, BRACE3004880, BRACE3004887, BRACE3004981, BRACE3004996, BRACE3004998, BRACE3005103, BRACE3005127, BRACE3005145, BRACE3005217, BRACE3Q05225, BRACE3005430, BRACE3005499, BRACE3005760, BRACE3005870, BRACE3005903, BRACE3005938, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642,
BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806,
BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194,
BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931,
BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760,
BRACE3039288, BRACE3039358, BRACE3039378, BRACE3039454, BRACE3040012, BRACE3040239, BRACE3040504, BRACE3040644, BRACE3040856, BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189,
BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880,
BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000562, BRAMY2000585, BRAMY2000653, BRAMY2000814, BRAMY2000946, BRAMY2000981, BRAMY2000987, BRAMY2001068, BRAMY2001114, BRAMY2001203,
BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492,
BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333,
BRAMY2008382, BRAMY2008563, BRAMY2008583, BRAMY2008977, BRAMY2008979, BRAMY2009013, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811,
BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013621, BRAMY2013659, BRAMY2013722, BRAMY2013736, BRAMY2013756, BRAMY2013944, BRAMY2013975, BRAMY2014019, BRAMY2014205,-BRAMY2014387, BRAMY2014462,. BRAMY2014469, BRAMY2014541, BRAMY2014754, BRAMY2015211, BRAMY2015251, BRAMY2015311, BRAMY2015420, BRAMY2015503, BRAMY2015516, BRAMY2015550,
BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016020, BRAMY2016070, BRAMY2016251, BRAMY2016325, BRAMY2016327, BRAMY2016386, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710,
BRAMY2019963, BRAMY2019985, BRAMY2019989, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022796, BRAMY2022929, BRAMY2022980, BRAMY2022984, BRAMY2023060,
BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023729, BRAMY2023786, BRAMY2023852, BRAMY2023863, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395,
BRAMY2026405, BRAMY2026533, BRAMY2026538, BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028516, BRAMY2028565, BRAMY2028593, BRAMY2028682, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768,
BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387,
BRAMY2036396, BRAMY2036567, BRAMY2036615, BRAMY2036699, BRAMY2036913, BRAMY2036918, BRAMY2036968, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040095, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244,
BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401,
BRAMY3001409, BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937,
BRAMY3009158, BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000037, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082,
BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000494, BRAWH2000500, BRAWH2000522, BRAWH2000554, BRAWH2000595, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000926, BRAWH2000944, BRAWH2000984, BRAWH2001019, BRAWH2001037, BRAWH2001052, BRAWH2001103, BRAWH2001129, BRAWH2001137, BRAWH2001159, BRAWH2001186, BRAWH2001203, BRAWH2001238, BRAWH2001239, BRAWH2001255,
BRAWH2001321, BRAWH2001355, BRAWH2001378, BRAWH2001395, BRAWH2001406, BRAWH2001412, BRAWH2001418, BRAWH2001424, BRAWH2001436, BRAWH2001438, BRAWH2001459, BRAWH2001461, BRAWH2001492, BRAWH2001503, BRAWH2001519, BRAWH2001530, BRAWH 2001535, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002601, BRAWH2002623, BRAWH2002630, BRAWH2002635, BRAWH2002662, BRAWH2002725, BRAWH2002761, BRAWH2002811, BRAWH2002861, BRAWH2002911, BRAWH2002963,
BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003693, BRAWH2003818, BRAWH2003832, BRAWH2003948, BRAWH2003964, BRAWH2004044, BRAWH2004068, BRAWH2004078,. BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960,
BRAWH2006989, BRAWH2006996, BRAWH2007069, BRAWH2007308, BRAWH2007406, BRAWH2007550, BRAWH2007570, BRAWH2007591, BRAWH2007605, BRAWH2007658, BRAWH2007664, BRAWH2007800, BRAWH2007808, BRAWH2007825, BRAWH2007862, BRAWH2007890, BRAWH2008058, BRAWH2008128, BRAWH2008255, BRAWH2008292, BRAWH2008297, BRAWH2008366, BRAWH2008380, BRAWH2008540, BRAWH2008577, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084,
BRAWH2010318, BRAWH2010329, BRAWH2010354, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013866, BRAWH2013871, BRAWH2013914, BRAWH2013941,
BRAWH2013955, BRAWH2014053, BRAWH2014234, BRAWH2014379, BRAWH2014414, BRAWH2014473, BRAWH2014511, BRAWH2014645, BRAWH2014662, BRAWH2014717, BRAWH2014729, BRAWH2014876, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433,
BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018729, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136,
BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666, BRAWH3005037, BRAWH3005047, BRAWH3005132, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461,
BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928,
BRAWH3021012, BRAWH3021545, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772,
BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239,
BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000.923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401,
BRCAN2006411, BRCAN2006450, BRCAN2006955, BRCAN2007119, BRCAN2007144, BRCAN2007409, BRCAN2007426, BRCAN2007525, BRCAN2007700, BRCAN2008494, BRCAN2008528, BRCAN2008701, BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015402, BRCAN2015464, BRCAN2015719, BRCAN2015757, BRCAN2015886, BRCAN2016025,
BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026340, BRCAN2026711, BRCAN2027310,
BRCAN2027334, BRCAN2027355, BRCAN2027513, BRCAN2027593, BRCAN2027970, BRCAN2028021, BRCAN2028040, BRCAN2028319, BRCAN2028338, BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003513, BRCOC2003732, BRCOC2003740, BRCOC2004036, BRCOC2004354, BRCOC2004455, BRCOC2004950, BRCOC2005542,
BRCOC2005903, BRCOC2005951, BRCOC2006164, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012172, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944,
BRCOC2016525, BRCOC2016661, BRCOC2017061, BRCOC2017652, BRCOC2017856, BRCOC2018775, BRCOC2018877, BRCOC2019549, BRCOC2019841, BRCOC2019896, BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172,
BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414,
BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011491, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387,
BRHIP2014954, BRHIP2015153, BRHIP2015224, BRHIP2015356, BRHIP2015360, BRHIP2016005, BRHIP2016.125, BRHIP2016788, BRHIP2016968, BRHIP2016990, BRHIP2017010, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018712, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859,
BRHIP2020930, BRHIP2021102, BRHIP2021289, BRHIP2021495, BRHIP2021615, BRHIP2021744, BRHIP2021858, BRHIP2021929, BRHIP2022006, BRHIP2022054, BRHIP2022221, BRHIP2022228, BRHIP2022298, BRHIP2022467, BRHIP2022708, BRHIP2023071, BRHIP2023272, BRHIP2023292, BRHIP2023309, BRHIP2023369, BRHIP2023735, BRHIP2023773, BRHIP2023860, BRHIP2023869, BRHIP2024046, BRHIP2024165, BRHIP2024201, BRHIP2024347, BRHIP2024398, BRHIP2024549, BRHIP2024742, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017,
BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395,
BRHIP3003484, BRHIP3003644, BRHIP3003688, BRHIP3003795, BRHIP3003984, BRHIP3004193, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405,
BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558,
BRHIP3017637, BRHIP3017855, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244,
BRSSN2000293, BRSSN2000295, BRSSN2000312, BRSSN2000518, BRSSN2000561, BRSSN2000566, BRSSN2000684, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464,
BRSSN2008549, BRSSN2008797, BRSSN2009119, BRSSN2009378, BRSSN2009389, BRSSN2009395, BRSSN2009518, BRSSN2009627, BRSSN2009630, BRSSN2009702, BRSSN2010019, BRSSN2010110, BRSSN2010587, BRSSN2010596, BRSSN2010830, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014636, BRSSN2014685, BRSSN2015199, BRSSN2015238, BRSSN2015369, BRSSN2015493, BRSSN2015497, BRSSN2015554, BRSSN2015707,
BRSSN2015773, BRSSN2015907, BRSSN2016589, BRSSN2016768, BRSSN2016905, BRSSN2017297,BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367,
BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014633, BRSTN2014751, BRSTN2014770, BRSTN2015015, BRSTN2015978, BRSTN2016066, BRSTN2016335, BRSTN2016678, BRSTN2016892, BRSTN2016918, BRSTN2016992, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063,
BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002741, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075,
BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012466,
BRTHA2012555, BRTHA2012562, BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304,
BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845,
BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, 8RTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003704, BRTHA3004307, BRTHA3004432, BRTHA3004475, BRTHA3004502,
BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010366, BRTHA3010469, BRTHA3010530, BRTHA3010540, BRTHA3010717, BRTHA3011149, BRTHA3011187, BRTHA3011194, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547,
BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505,
BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105,
COLON2009337, COLON2009499, CORDB1000140, CORDB2000061, CORDB2000127, CORDB2000541, CTONG1000010, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000180, CTONG1000181, CTONG1000241, CTONG1000283, CTONG1000288, CTONG1000302, CTONG1000341, CTONG1000476, CTONG1000488, CTONG1000508, CTONG1000540, CTONG2000031, CTONG2000034, CTONG2000201, CTONG2000218, CTONG2000279, CTONG2000303, CTONG2000330, CTONG2000395, CTONG2000465, CTONG2000480, CTONG2000508, CTONG2000766, CTONG2000771, CTONG2000819, CTONG2000827, CTONG2000846, CTONG2000855, CTONG2000977, CTONG2001139,
CTONG2001226, CTONG2001366, CTONG2001413, CTONG2001428, CTONG2001524, CTONG2001533, CTONG2001598, CTONG2001681, CTONG2001724, CTONG2001749, CTONG2001800, CTONG2001820, CTONG2001858, CTONG2001877, CTONG2001911, CTONG2001955, CTONG2002066, CTONG2002095, CTONG2002143, CTONG2002395, CTONG2002417, CTONG2002418, CTONG2002558, CTONG2002590, CTONG2002721, CTONG2002766, CTONG2002803, CTONG2002816, CTONG2002820, CTONG2002832, CTONG2002841, CTONG2002965, CTONG2002999, CTONG2003028, CTONG2003100, CTONG2003115, CTONG2003189, CTONG2003245, CTONG2003293, CTONG2003298, CTONG2003348, CTONG2003350, CTONG2003361, CTONG2003372, CTONG2003375, CTONG2003427, CTONG2003517, CTONG2003524, CTONG2003599, CTONG2003680,
CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890, CTONG2005904, CTONG2006004, CTONG2006129, CTONG2006223, CTONG2006235, CTONG2006273, CTONG2006310, CTONG2006377, CTONG2006393, CTONG2006412, CTONG2006449,
CTONG2006515, CTONG2006524, CTONG2006568, CTONG2006611, CTONG2006620, CTONG2006666, CTONG2006691, CTONG2006798, CTONG2006836, CTONG2006942, CTONG2006964, CTONG2007007, CTONG2007009, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008405, CTONG2008466, CTONG2008521, CTONG2008562, CTONG2008577, CTONG2008595, CTONG2008621, CTONG2008689, CTONG2008720, CTONG2008721,
CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2009963, CTONG2010018, CTONG2010024, CTONG2010116, CTONG2010133, CTONG2010148, CTONG2010276, CTONG2010330, CTONG2010408, CTONG2010508, CTONG2010566, CTONG2010623, CTONG2010649, CTONG2010652,
CTONG2010803, CTONG2011188, CTONG2011429, CTONG2011474, CTONG2011676, CTONG2011716, CTONG2011765, CTONG2011779, CTONG2011801, CTONG2011815, CTONG2011822, CTONG2011920, CTONG2011985, CTONG2012029, CTONG2012050, CTONG2012077, CTONG2012101, CTONG2012123, CTONG2012158, CTONG2012230, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012745, CTONG2012758, CTONG2012843, CTONG2012901, CTONG2012996, CTONG2013128, CTONG2013178, CTONG2013284, CTONG2013339, CTONG2013348, CTONG2013352, CTONG2013381, CTONG2013393, CTONG2013511, CTONG2013630, CTONG2013803, CTONG2013907, CTONG2013934,
CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014369, CTONG2014389, CTONG2014491, CTONG2014630, CTONG2014635, CTONG2014646, CTONG2014705, CTONG2014898, CTONG2014946, CTONG2014954, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016408, CTONG2016499, CTONG2016505, CTONG2016559, CTONG2016658, CTONG2016775,
CTONG2016797, CTONG2016824, CTONG2016846, CTONG2016869, CTONG2016904, CTONG2016931, CTONG2016942, CTONG2016953, CTONG2017021, CTONG2017094, CTONG2017292, CTONG2017409, CTONG2017429, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2017998, CTONG2018062, CTONG2018069, CTONG2018147, CTONG2018211, CTONG2018217, CTONG2018279, CTONG2018334, CTONG2018343, CTONG2018379, CTONG2018413, CTONG2018483, CTONG2018629, CTONG2018637, CTONG2018652, CTONG2018653, CTONG2018655, CTONG2018735, CTONG2018739, CTONG2018808, CTONG2018843, CTONG2018898, CTONG2018900, CTONG2018976, CTONG2019063, CTONG2019144,
CTONG2019232, CTONG2019400, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020522, CTONG2020638, CTONG2020806, CTONG2020831, CTONG2020974, CTONG2021024, CTONG2021132, CTONG2021168, CTONG2021289, CTONG2022153, CTONG2022601, CTONG2022835, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959,
CTONG2028097, CTONG2028486, CTONG2028518, CTONG2028687, CTONG2028758, CTONG3000686, CTONG3000707, CTONG3000896, CTONG3001123, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317, CTONG3004397, CTONG3004550, CTONG3004576, CTONG3004607, CTONG3004712, CTONG3005325, CTONG3005648, CTONG3005713, CTONG3005803, CTONG3005813, CTONG3006067, CTONG3006073,
CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, D3OST1000066 D3OST1000238 D3OST1000267, D3OST2000184 D3OST2000618 D3OST2000654 D3OST2000734 D3OST2001328 D3OST2001669 D3OST2001670 D3OST2001788 D3OST2002223 D3OST2002262 D3OST2002272, D3OST2002417 D3OST2002436 D3OST2002648 D3OST2003024 D3OST2003331 D3OST2003607 D3OST2003797 D3OST2004637 D3OST3000195 D3OST3000258 D3OST3000291 D3OST3000388,
D3OST3000493, D6OST2000127, D6OST2000169 D6OST2000245 D6OST2000358 D6OST2000464 D6OST2000507 D9OST2000031 D9OST2000278 D9OST2000440 D9OST2000869 D9OST2001319, D9OST2001433, D9OST2002397 D9OST2002608, D9OST2002673 D9OST2003106, D9OST2003108 D9OST2003137 D9OST2003397 D9OST2003580, D9OST2003594 D9OST2003762, D9OST2003791 D9OST2003989, D9OST2004018 D9OST2004073 D9OST2004417 DFNES1000003, DFNES1000107, DFNES2000143, DFNES2000146, DFNES2000153, DFNES2000212, DFNES2000335, DFNES2000426, DFNES2000443, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829,
DFNES2002220, DFNES2002509, DFNES2002550, DFNES2002588, DFNES2002817, DFNES2002966, DFNES2003081, DFNES2003255, DFNES2003742, DFNES2004346, DFNES2004354, DFNES2004383, DFNES2004608, DFNES2004684, DFNES2005266, DFNES2005527, DFNES2005690, DFNES2005766, DFNES2005779, DFNES2005875, DFNES2006346, DFNES2006944, DFNES2007113, DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011245, DFNES2011380, ERLTF1000021, ERLTF2000324, ERLTF2000465, FCBBF1000023, FCBBF1000038, FCBBF1000063, FCBBF1000069, FCBBF1000115, FCBBF1000117, FCBBF1000118, FCBBF1000121, FCBBF1000125, FCBBF1000131,
FCBBF1000155, FCBBF1000157, FCBBF1000171, FCBBF1000174, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000476, FCBBF1000506, FCBBF1000509, FCBBF1000546, FCBBF1000550, FCBBF1000556, FCBBF1000574, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF1000760, FCBBF2000038, FCBBF2000087, FCBBF2000094,
FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000576, FCBBF2000677, FCBBF2000678, FCBBF2000733, FCBBF2000782, FCBBF2000808, FCBBF2000815, FCBBF2000825, FCBBF2000940, FCBBF2000951, FCBBF2001001, FCBBF2001088, FCBBF2001116, FCBBF2001183, FCBBF2001188, FCBBF2001211, FCBBF2001238, FCBBF2001243, FCBBF2001291, FCBBF2001299, FCBBF2001400, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002801,
FCBBF2002897, FCBBF2002898, FCBBF2002904, FCBBF2002928, FCBBF2002953, FCBBF2003083, FCBBF2003102, FCBBF2003269, FCBBF2003293, FCBBF2003297, FCBBF2003395,FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005645, FCBBF2005658, FCBBF2005733, FCBBF2005909, FCBBF2005966, FCBBF2006131, FCBBF2006380, FCBBF2006418, FCBBF2006452, FCBBF2006781, FCBBF2007012, FCBBF2007186, FCBBF2007265, FCBBF2007302, FCBBF2007556, FCBBF2007610, FCBBF2007633,
FCBBF2007840, FCBBF3000102, FCBBF3000110, FCBBF3000115, FCBBF3000120, FCBBF3000168, FCBBF3000175, FCBBF3000227, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518, FCBBF3000536, FCBBF3000550, FCBBF3000847, FCBBF3001018, FCBBF3001020, FCBBF3001235, FCBBF3001281, FCBBF3001302, FCBBF3001377, FCBBF3001470, FCBBF3001594, FCBBF3001632, FCBBF3001818, FCBBF3001855, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305,
FCBBF3003373, FCBBF3003435, FCBBF3003475, FCBBF3003481, FCBBF3003557, FCBBF3003787, FCBBF3003800, FCBBF3003902, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008153, FCBBF3008159, FCBBF3008251, FCBBF3008311,
FCBBF3008362, FCBBF3008382, FCBBF3008475, FCBBF3008556, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288,
FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014355, FCBBF3014567, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016928, FCBBF3016987, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729,
FCBBF3017873, FCBBF3017918, FCBBF3017974, FCBBF3018067, FCBBF3018173, FCBBF3018453, FCBBF3018591, FCBBF3018753, FCBBF3018796, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019714, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3020599, FCBBF3021026, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078,
FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328; FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028472, FCBBF3028528,
FCBBF3028582, FCBBF3028593, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000022, FEBRA1000030, FEBRA1000057, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000210, FEBRA2000220, FEBRA2000228, FEBRA2000253,
FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000409, FEBRA2000415, FEBRA2000436, FEBRA2000438, FEBRA2000452, FEBRA2000454, FEBRA2000462, FEBRA2000468, FEBRA2000491, FEBRA2000504, FEBRA2000532, FEBRA2000536, FEBRA2000538, FEBRA2000575, FEBRA2000656, FEBRA2000659, FEBRA2000680, FEBRA2000690, FEBRA2000727, FEBRA2000734, FEBRA2000740, FEBRA2000757, FEBRA2000762, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000880, FEBRA2000901, FEBRA2000909,
FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334, FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001752, FEBRA2001772, FEBRA2001790, FEBRA2001814, FEBRA2001828, FEBRA2001867,
FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308,
FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004329, FEBRA2004331, FEBRA2004443, FEBRA2004485, FEBRA2004490, FEBRA2004538, FEBRA2004592, FEBRA2004620, FEBRA2004651, FEBRA2004767, FEBRA2004813, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114,
FEBRA2005216, FEBRA2005291, FEBRA2005361, FEBRA2005377, FEBRA2005380, FEBRA2005427, FEBRA2005458, FEBRA2005476, FEBRA2005701, FEBRA2005726, FEBRA2005752, FEBRA2005778, FEBRA2005787, FEBRA2005788, FEBRA2005995, FEBRA2005998, FEBRA2006061, FEBRA2006092, FEBRA2006150, FEBRA2006165, FEBRA2006255, FEBRA2006315, FEBRA2006346, FEBRA2006354, FEBRA2006357, FEBRA2006368, FEBRA2006370, FEBRA2006379, FEBRA2006396, FEBRA2006401, FEBRA2006409, FEBRA2006418, FEBRA2006427, FEBRA2006476, FEBRA2006485, FEBRA2006491, FEBRA2006521, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017,
FEBRA2007176, FEBRA2007200, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007622, FEBRA2007708, FEBRA2007714, FEBRA2007793, FEBRA2007801, FEBRA2007818, FEBRA2007823, FEBRA2007880, FEBRA2007900, FEBRA2007901, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008210, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008583,
FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090,
FEBRA2011356, FEBRA2011392, FEBRA2011414, FEBRA2011593, FEBRA2011665, FEBRA2012090, FEBRA2012120, FEBRA2012181, FEBRA2012195, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680,
FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601, FEBRA2022696, FEBRA2022729,
FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325,
FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405,
HCASM2009424, HCASM2009463,-HCASM2009588, HCHON1000015, HCHON1000131, HCHON1000142, HCHON1000176, HCHON1000191, HCHON2000038, HCHON2000100, HCHON2000156, HCHON2000199, HCHON2000212, HCHON2000271, HCHON2000315, HCHON2000323, HCHON2000344, HCHON2000364, HCHON2000395, HCHON2000417, HCHON2000418, HCHON2000473, HCHON2000503, HCHON2000626, HCHON2000660, HCHON2000663, HCHON2000673, HCHON2000676, HCHON2000698, HCHON2000699, HCHON2000705, HCHON2000738, HCHON2000743, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001108, HCHON2001110, HCHON2001116, HCHON2001161,
HCHON2001214, HCHON2001233, HCHON2001269, HCHON2001450, HCHON2001453, HCHON2001497, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987,
HCHON2006250, HCHON2006459, HCHON2006714, HCHON2006722, HCHON2006770, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000010, HEART1000099, HEART1000102, HEART1000118, HEART1000132, HEART1000151, HEART1000173, HEART2000024, HEART2000035, HEART2000099, HEART2000298, HEART2000306, HEART2000309, HEART2000411, HEART2000418, HEART2000444, HEART2000448, HEART2000492, HEART2000568, HEART2000611, HEART2000959,
HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007231, HEART2007443, HEART2007767, HEART2008108, HEART2008111, HEART2008257, HEART2008509, HEART2008994, HEART2009000, HEART2009599, HEART2009680, HEART2010391, HEART2010492, HELAC2000158, HELAC2000301,
HHDPC1000001, HHDPC1000065, HHDPC1000114, HHDPC1000118, HHDPC2000070, HHDPC2000102, HHDPC2000104, HHDPC2000258, HHDPC2000315, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053,
HLUNG1000064, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000926, HLUNG2000950, HLUNG2000955, HLUNG2000970, HLUNG2001013, HLUNG2001126, HLUNG2001144, HLUNG2001146, HLUNG2001214, HLUNG2001439, HLUNG2001507,
HLUNG2001677, HLUNG2001814, HLUNG2001996, HLUNG2002006, HLUNG2002050, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002811, HLUNG2002916, HLUNG2002942, HLUNG2002949, HLUNG2002958, HLUNG2002982, HLUNG2003003, HLUNG2003049, HLUNG2003061, HLUNG2003162, HLUNG2003246, HLUNG2003331, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154, HLUNG2004217, HLUNG2004273, HLUNG2004388, HLUNG2004416, HLUNG2004521, HLUNG2004534, HLUNG2004684,
HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521,
HLUNG2008833, HLUNG2008875, HLUNG2008885, HLUNG2008958, HLUNG2008959, HLUNG2009067, HLUNG2009225, HLUNG2009253, HLUNG2009303, HLUNG2009413, HLUNG2009729, HLUNG2010181, HLUNG2010464, HLUNG2010562, HLUNG2010845, HLUNG2011041, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282,
HLUNG2018626, HLUNG2019058, HSYRA1000131, HSYRA1000148, HSYRA2000044, HSYRA2000135, HSYRA2000190, HSYRA2000221, HSYRA2000237, HSYRA2000253, HSYRA2000255, HSYRA2000332, HSYRA2000371, HSYRA2000424, HSYRA2000507, HSYRA2000510, HSYRA2000629, HSYRA2000706, HSYRA2000760, HSYRA2000792, HSYRA2000832, HSYRA2000925, HSYRA2000927, HSYRA2001003, HSYRA2001103, HSYRA2001142, HSYRA2001153, HSYRA2001232, HSYRA2001255, HSYRA2001332, HSYRA2001353, HSYRA2001420, HSYRA2001484, HSYRA2001552, HSYRA2001567, HSYRA2001580, HSYRA2001595, HSYRA2001631, HSYRA2001983, HSYRA2002103, HSYRA2002590, HSYRA2003168,. HSYRA2004550, HSYRA2004858, HSYRA2005456, HSYRA2005496, HSYRA2005628, HSYRA2005970, HSYRA2006049, HSYRA2006873,
HSYRA2007241, HSYRA2007338, HSYRA2007667, HSYRA2008154, HSYRA2008714, HSYRA2009102, IMR321000155, IMR321000158, IMR321000161, IMR321000165, IMR321000193, IMR321000207, IMR321000230, IMR321000242, IMR321000253, IMR322000010, IMR322000072, IMR322000107, IMR322000152, IMR322000164, IMR322000302, IMR322000374, IMR322000379, IMR322000383, IMR322000418, IMR322000518, IMR322000555, IMR322000604, IMR322000609, IMR322000656, IMR322000672, IMR322000742, IMR322000791, IMR322000819, IMR322000838, IMR322000859, IMR322000863, IMR322000917, IMR322000919, IMR322000953, IMR322000973, IMR322000984, IMR322001018, IMR322001167, IMR322001185, IMR322001245, IMR322001317, IMR322001318, IMR322001322, IMR322001332,
IMR322001380, IMR322001438, IMR322001452, IMR322001491, IMR322001534, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036,
KIDNE1000064, KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000266, KIDNE2000287, KIDNE2000315, KIDNE2000317, KIDNE2000328, KIDNE2000330, KIDNE2000335, KIDNE2000349, KIDNE2000375, KIDNE2000394, KIDNE2000422, KIDNE2000493, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000947, KIDNE2001117, KIDNE2001140, KIDNE2001269, KIDNE2001361, KIDNE2001373,
KIDNE2001713, KIDNE2001802, KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002252, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003941, KIDNE2003994, KIDNE2004034, KIDNE2004054, KIDNE2004084, KIDNE2004095, KIDNE2004115, KIDNE2004233, KIDNE2004262, KIDNE2004279,
KIDNE2004294, KIDNE2004295, KIDNE2004305, KIDNE2004344, KIDNE2004394, KIDNE2004519, KIDNE2004520, KIDNE2004531, KIDNE2004534, KIDNE2004562, KIDNE2004579, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006014, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334,
KIDNE2006353, KIDNE2006376, KIDNE2006378, KIDNE2006465, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007040, KIDNE2007077, KIDNE2007186, KIDNE2007222, KIDNE2007422, KIDNE2007569, KIDNE2007810, KIDNE2007811, KIDNE2007910, KIDNE2007944, KIDNE2008022, KIDNE2008069, KIDNE2008072, KIDNE2008117, KIDNE2008218, KIDNE2008315, KIDNE2008362, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109,
KIDNE2009180, KIDNE2009191, KIDNE2009332, KIDNE2009367, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012710,
KIDNE2012745, KIDNE2012775, KIDNE2012784, KIDNE2012945, KIDNE2013045, KIDNE2013095, KIDNE2013158, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000,
KIDNE2016036, KIDNE2016096, KIDNE2016101, KIDNE2016142, KIDNE2016188, KIDNE2016327, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099,
LIVER1000104, LIVER1000111, LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003234, LIVER2003511,
LIVER2003524, LIVER2003568, LIVER2003581, LIVER2003800, LIVER2003854, LIVER2004074, LIVER2004392, LIVER2004514, LIVER2004565, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000100, MAMGL1000132, MAMGL1000151, MAMGL1000178,
MAMGL1000182, MAMGL1000184, MESAN1000032, MESAN1000035, MESAN1000050, MESAN1000079, MESAN1000080, MESAN1000121, MESAN1000147, MESAN1000180, MESAN2000023, MESAN2000026, MESAN2000067, MESAN2000092, MESAN2000149, MESAN2000167, MESAN2000291, MESAN2000337, MESAN2000434, MESAN2000462, MESAN2000501, MESAN2000711, MESAN2000815, MESAN2000894, MESAN2000909, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002844, MESAN2002978, MESAN2003037, MESAN2003039, MESAN2003101, MESAN2003190, MESAN2003322, MESAN2003490, MESAN2003529, MESAN2003646, MESAN2003662, MESAN2003709, MESAN2003851,
MESAN2004138, MESAN2004288, MESAN2004575, MESAN2005284, MESAN2005371, MESAN2005633, MESAN2005689, MESAN2005724, MESAN2005808, MESAN2005811, MESAN2006022, MESAN2006043, MESAN2006328, MESAN2006401, MESAN2006580, MESAN2006599, MESAN2006953, MESAN2006971, MESAN2007032, MESAN2007440, MESAN2007597, MESAN2007755, MESAN2008222, MESAN2008334, MESAN2008460, MESAN2008536, MESAN2008679, MESAN2008729, MESAN2008821, MESAN2008926, MESAN2009019, MESAN2009081, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011632, MESAN2011977, MESAN2012586, MESAN2012708, MESAN2012735,
MESAN2013019, MESAN2013022, MESAN2013183, MESAN2013211, MESAN2013283, MESAN2013284, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESTC1000042, MESTC1000147, MESTC2000150, MESTC2000153, MESTC2000170, N1ESE2000698, NB9N41000047, NB9N41000121, NB9N41000135, NB9N41000142, NB9N41000146, NB9N41000340, NB9N42000104, NB9N42000122, NB9N42000196,
NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000212, NHNPC2000606, NHNPC2000877, NHNPC2001108, NHNPC2001223, NHNPC2001312, NHNPC2001816, NHNPC2002565, NHNPC2002749, NOVAR1000015, NOVAR1000102, NOVAR2000136, NOVAR2000352, NOVAR2000412, NOVAR2000710,
NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658,
NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001442, NT2NE2001524, NT2NE2001598, NT2NE2001617, NT2NE2001623, NT2NE2001626, NT2NE2001634, NT2NE2001648, NT2NE2001660, NT2NE2001666, NT2NE2001677, NT2NE2001697, NT2NE2001698, NT2NE2001723, NT2NE2001725, NT2NE2001874, NT2NE2001889,
NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004519, NT2NE2004606, NT2NE2004740, NT2NE2004787, NT2NE2004916, NT2NE2005035, NT2NE2005223, NT2NE2005276, NT2NE2005317, NT2NE2005323, NT2NE2005358, NT2NE2005395, NT2NE2005419, NT2NE2005517, NT2NE2005537, NT2NE2005555, NT2NE2005588,
NT2NE2005676, NT2NE2005720, NT2NE2005784, NT2NE2005804, NT2NE2005821, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295,
NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011107, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012790, NT2NE2012847, NT2NE2013019, NT2NE2013081, NT2NE2013189, NT2NE2013217, NT2NE2013547, NT2NE2014013, NT2NE2014028, NT2NE2014113,
NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018180, NT2NE2018273, NT2NE2018376, NT2NE2018472, NT2NE2018490,
NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000263, NT2RI2000270, NT2RI2000276, NT2RI2000284, NT2RI2000294, NT2RI2000313, NT2RI2000341, NT2RI2000344, NT2RI2000348, NT2RI2000412, NT2RI2000421, NT2RI2000480, NT2RI2000575, NT2RI2000578, NT2RI2000588, NT2RI2000597, NT2RI2000669, NT2RI2000688, NT2RI2000704, NT2RI2000738, NT2RI2000775, NT2RI2000829, NT2RI2000841, NT2RI2000865, NT2RI2000932, NT2RI2000987, NT2RI2001010, NT2RI2001017,
NT2RI2001030, NT2RI2001054, NT2RI2001083, NT2RI2001091, NT2RI2001167, NT2RI2001178, NT2RI2001235, NT2RI2001244, NT2RI2001307, NT2RI2001385, NT2RI2001409, NT2RI2001410, NT2RI2001422, NT2RI2001425, NT2RI2001449, NT2RI2001450, NT2RI2001474, NT2RI2001519, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120, NT2RI2002152, NT2RI2002153, NT2RI2002179, NT2RI2002243, NT2RI2002252, NT2RI2002260, NT2RI2002266, NT2RI2002270, NT2RI2002334, NT2RI2002447, NT2RI2002481, NT2RI2002507, NT2RI2002517, NT2RI2002540, NT2RI2002549, NT2RI2002564, NT2RI2002585,
NT2RI2002592, NT2RI2002602, NT2RI2002802, NT2RI2002822, NT2RI2002852, NT2RI2002923, NT2RI2002926, NT2RI2002970, NT2RI2003011, NT2RI2003067, NT2RI2003184, NT2RI2003205, NT2RI2003222, NT2RI2003301, NT2RI2003344, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667, NT2RI2003678, NT2RI2003751, NT2RI2003884, NT2RI2003973, NT2RI2004059, NT2RI2004078, NT2RI2004079, NT2RI2004093, NT2RI2004136, NT2RI2004157, NT2RI2004188, NT2RI2004190, NT2RI2004209, NT2RI2004284, NT2RI2004290, NT2RI2004304, NT2RI2004312, NT2RI2004381, NT2RI2004410, NT2RI2004442, NT2RI2004468, NT2RI2004535, NT2RI2004606, NT2RI2004618, NT2RI2004783,
NT2RI2004818, NT2RI2004840, NT2RI2004884, NT2RI2004916, NT2RI2004962, NT2RI2004984, NT2RI2004985, NT2RI2005048, NT2RI2005069, NT2RI2005087, NT2RI2005096, NT2RI2005130, NT2RI2005150, NT2RI2005166, NT2RI2005239, NT2RI2005246, NT2RI2005315, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005713, NT2RI2005772, NT2RI2005816, NT2RI2005818, NT2RI2005851, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006412, NT2RI2006445, NT2RI2006487, NT2RI2006506, NT2RI2006640,
NT2RI2006659, NT2RI2006662, NT2RI2006667, NT2RI2006679, NT2RI2006703, NT2RI2006716, NT2RI2006735, NT2RI2006762, NT2RI2006788, NT2RI2006838, NT2RI2006853, NT2RI2006943, NT2RI2006973, NT2RI2007046, NT2RI2007048, NT2RI2007084, NT2RI2007096, NT2RI2007116, NT2RI2007148, NT2RI2007214, NT2RI2007277, NT2RI2007303, NT2RI2007334, NT2RI2007386, NT2RI2007406, NT2RI2007439, NT2RI2007445, NT2RI2007468, NT2RI2007469, NT2RI2007507, NT2RI2007572, NT2RI2007593, NT2RI2007629, NT2RI2007723, NT2RI2007729, NT2RI2007751, NT2RI2007754, NT2RI2007827, NT2RI2007877, NT2RI2007884, NT2RI2007891, NT2RI2007956, NT2RI2007965, NT2RI2008007, NT2RI2008045, NT2RI2008050, NT2RI2008171, NT2RI2008188, NT2RI2008204, NT2RI2008233,
NT2RI2008455, NT2RI2008481, NT2RI2008502, NT2RI2008566, NT2RI2008598, NT2RI2008622, NT2RI2008656, NT2RI2008714, NT2RI2008724, NT2RI2008801, NT2RI2008808, NT2RI2008841, NT2RI2008942, NT2RI2008952, NT2RI2009005, NT2RI2009066, NT2RI2009083, NT2RI2009101, NT2RI2009103, NT2RI2009144, NT2RI2009151, NT2RI2009173, NT2RI2009194, NT2RI2009215, NT2RI2009233, NT2RI2009259, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012659, NT2RI2012726, NT2RI2013357, NT2RI2013373, NT2RI2014551, NT2RI2014683, NT2RI2014733, NT2RI2015239, NT2RI2016128,
NT2RI2016134, NT2RI2017529, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445,
NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006376, NT2RI3006673, NT2RI3006796, NT2RI3007065, NT2RI3007095, NT2RI3007158, NT2RI3007167, NT2RI3007213, NT2RI3007291, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697,
NT2RI3008974, NT2RI3009158, NT2RI3009480, NT2RI3009524, NT2RP6000008, NT2RP6000017, NT2RP6000033, NT2RP6000043, NT2RP6000059, NT2RP6000060, NT2RP6000085, NT2RP6000086, NT2RP6000100, NT2RP6000109, NT2RP6000119, NT2RP6000127, NT2RP6000130, NT2RP6000140, NT2RP6000150, NT2RP7000041, NT2RP7000086, NT2RP7000109, NT2RP7000112, NT2RP7000238, NT2RP7000259, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000906, NT2RP7000926, NT2RP7001074, NT2RP7001080, NT2RP7001156, NT2RP7001231, NT2RP7001306, NT2RP7001319, NT2RP7001335, NT2RP7001364, NT2RP7001475, NT2RP7001532, NT2RP7001591, NT2RP7001602, NT2RP7001678,
NT2RP7001856, NT2RP7002064, NT2RP7002151, NT2RP7002163, NT2RP7002243, NT2RP7002282, NT2RP7002376, NT2RP7002449, NT2RP7002450, NT2RP7002478, NT2RP7002554, NT2RP7002594, NT2RP7002603, NT2RP7002650, NT2RP7002738, NT2RP7002779, NT2RP7002802, NT2RP7002829, NT2RP7002841, NT2RP7002875, NT2RP7002906, NT2RP7002978, NT2RP7003025, NT2RP7003050, NT2RP7003055, NT2RP7003084, NT2RP7003091, NT2RP7003107, NT2RP7003134, NT2RP7003148, NT2RP7003439, NT2RP7003511, NT2RP7003632, NT2RP7003680, NT2RP7003960, NT2RP7004027, NT2RP7004080, NT2RP7004123, NT2RP7004204, NT2RP7004233, NT2RP7004260, NT2RP7004348, NT2RP7004352, NT2RP7004358, NT2RP7004396, NT2RP7004428, NT2RP7004481, NT2RP7004541, NT2RP7004559, NT2RP7004641,
NT2RP7004656, NT2RP7004687, NT2RP7004722, NT2RP7004766, NT2RP7004790, NT2RP7004884, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005846, NT2RP7006160, NT2RP7006162, NT2RP7006223, NT2RP7006263, NT2RP7006296, NT2RP7006304, NT2RP7006374, NT2RP7006395, NT2RP7006408, NT2RP7006457, NT2RP7006490, NT2RP7006539, NT2RP7006547, NT2RP7006601, NT2RP7006621, NT2RP7006636, NT2RP7006701, NT2RP7006717, NT2RP7006853, NT2RP7006886, NT2RP7006887, NT2RP7006986, NT2RP7006995, NT2RP7007114, NT2RP7007221, NT2RP7007226, NT2RP7007252, NT2RP7007269,
NT2RP7007310, NT2RP7007359, NT2RP7007381, NT2RP7007406, NT2RP7007480, NT2RP7007504, NT2RP7007530, NT2RP7007580, NT2RP7007594, NT2RP7007610, NT2RP7007617, NT2RP7007643, NT2RP7007766, NT2RP7007842, NT2RP7007925, NT2RP7007930, NT2RP7008013, NT2RP7008015, NT2RP7008133, NT2RP7008137, NT2RP7008142, NT2RP7008144, NT2RP7008315, NT2RP7008360, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009168, NT2RP7009215, NT2RP7009225, NT2RP7009236, NT2RP7009253, NT2RP7009267, NT2RP7009322,
NT2RP7009363, NT2RP7009370, NT2RP7009373, NT2RP7009397, NT2RP7009429, NT2RP7009439, NT2RP7009466, NT2RP7009481, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080,
NT2RP7015431, NT2RP7015503, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787,
NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000075, NTONG1000087, NTONG1000130, NTONG1000161, NTONG1000172, NTONG1000213, NTONG1000214, NTONG1000246, NTONG1000247, NTONG2000040, NTONG2000107, NTONG2000231, NTONG2000363, NTONG2000413, NTONG2000492, NTONG2000499, NTONG2000531, NTONG2000583, NTONG2000800, NTONG2000966, NTONG2001014, NTONG2001079, NTONG2001137, NTONG2001157, NTONG2001173, NTONG2001222, NTONG2001362, NTONG2001428, NTONG2001532, NTONG2001587, NTONG2001619, NTONG2001642, NTONG2001657, NTONG2001676, NTONG2001762, NTONG2002278, NTONG2002582, NTONG2002807, NTONG2002970,
NTONG2002985, NTONG2003158, NTONG2003210, NTONG2003316, NTONG2003409, NTONG2003515, NTONG2003805, NTONG2003839, NTONG2003897, NTONG2003928, NTONG2004095, NTONG2004308, NTONG2004521, NTONG2004690, NTONG2004806, NTONG2004829, NTONG2004918, NTONG2004991, NTONG2005062, NTONG2005086, NTONG2005137, NTONG2005153, NTONG2005265, NTONG2005277, NTONG2005363, NTONG2005373, NTONG2005480, NTONG2005577, NTONG2005657, NTONG2005822, NTONG2005897, NTONG2005969, NTONG2006099, NTONG2006187, NTONG2006301, NTONG2006324, NTONG2006354, NTONG2006440, NTONG2006501, NTONG2006646, NTONG2006709, NTONG2007020, NTONG2007028, NTONG2007249, NTONG2007517, NTONG2007522, NTONG2007693, NTONG2008088, NTONG2008093, NTONG2008143,
NTONG2008365, NTONG2008522, NTONG2008672, NTONG2008862, NTONG2008939, NTONG2009068, NTONG2009229, NTONG2009233, NTONG2009468, NTONG2009576, OCBBF1000016, OCBBF1000030, OCBBF1000042, OCBBF1000049, OCBBF1000054, OCBBF1000067, OCBBF1000080, OCBBF1000085, OCBBF1000086, OCBBF1000091, OCBBF1000104, OCBBF1000114, OCBBF1000118, OCBBF1000119, OCBBF1000122, OCBBF1000130, OCBBF1000138, OCBBF1000145, OCBBF1000153, OCBBF1000175, OCBBF1000185, OCBBF1000192, OCBBF1000254, OCBBF2000126, OCBBF2000178, OCBBF2000231, OCBBF2000277, OCBBF2000287, OCBBF2000323, OCBBF2000452, OCBBF2000467, OCBBF2000522, OCBBF2000677, OCBBF2000703, OCBBF2000712, OCBBF2000719, OCBBF2000831, OCBBF2000904, OCBBF2000982, OCBBF2000998,
OCBBF2001075, OCBBF2001101, OCBBF2001124, OCBBF2001140, OCBBF2001166, OCBBF2001176, OCBBF2001186, OCBBF2001196, OCBBF2001252, OCBBF2001323, OCBBF2001389, OCBBF2001402, OCBBF2001408, OCBBF2001416, OCBBF2001473, OCBBF2001494, OCBBF2001527, OCBBF2001528, OCBBF2001681, OCBBF2001706, OCBBF2001749, OCBBF2001794, OCBBF2001910, OCBBF2001931, OCBBF2001961, OCBBF2002015, OCBBF2002083, OCBBF2002124, OCBBF2002161, OCBBF2002290, OCBBF2002357, OCBBF2002376, OCBBF2002615, OCBBF2002656, OCBBF2002663, OCBBF2002805, bCBBF2002865, OCBBF2002920, OCBBF2002980, OCBBF2003028, OCBBF2003052, OCBBF2003091, OCBBF2003246, OCBBF2003327, OCBBF2003543, OCBBF2003593, OCBBF2003744, OCBBF2003819, OCBBF2003925, OCBBF2004038,
OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006030, OCBBF2006058, OCBBF2006113, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764,
OCBBF2006859, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007415, OCBBF2007428, OCBBF2007478, OCBBF2007485, OCBBF2007520, OCBBF2007555, OCBBF2007610, OCBBF2007756, OCBBF2007762, OCBBF2007892, OCBBF2007931, OCBBF2007946, OCBBF2008005, OCBBF2008041, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775,
OCBBF2008790, OCBBF2008814, OCBBF2008822, OCBBF2009115, OCBBF2009242, OCBBF2009301, OCBBF2009352, OCBBF2009391, OCBBF2009424, OCBBF2009536, OCBBF2009571, OCBBF2009583, OCBBF2009788, OCBBF2009836, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010830, OCBBF2010841, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669,
OCBBF2011685, OCBBF2011706, OCBBF2011722, OCBBF2011759, OCBBF2011767, OCBBF2011855, OCBBF2011872, OCBBF2011897, OCBBF2011914, OCBBF2011960, OCBBF2011981, OCBBF2012001, OCBBF2012028, OCBBF2012039, OCBBF2012139, OCBBF2012191, OCBBF2012262, OCBBF2012320, OCBBF2012436, OCBBF2012525, OCBBF2012553, OCBBF2012678, OCBBF2012704, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926, OCBBF2014052, OCBBF2014064, OCBBF2014089,
OCBBF2014292, OCBBF2014322, OCBBF2014386, OCBBF2014541, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2017069, OCBBF2017306, OCBBF2017325, OCBBF2017489, OCBBF2017516, OCBBF2017665, OCBBF2017815, OCBBF2017882, OCBBF2017893, OCBBF2017920, OCBBF2018014, OCBBF2018084, OCBBF2018167, OCBBF2018206,
OCBBF2018229, OCBBF2018234, OCBBF2018362, OCBBF2018380, OCBBF2018563, OCBBF2018581, OCBBF2018618, OCBBF2018663, OCBBF2018687, OCBBF2018707, OCBBF2018827, OCBBF2018828, OCBBF2018934, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451,
OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948,
OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972,
OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, SALGL1000024, SALGL1000028, SALGL1000065, SALGL1000107, SALGL1000171, SKMUS1000014, SKMUS1000030, SKMUS1000067, SKMUS1000102, SKMUS1000104, SKMUS1000110, SKMUS1000118, SKMUS1000124, SKMUS1000129, SKMUS1000176, SKMUS1000177, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000343, SKMUS2000365, SKMUS2000390, SKMUS2000416, SKMUS2000467, SKMUS2000468, SKMUS2000484, SKMUS2000690,
SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000902, SKMUS2000933, SKMUS2000945, SKMUS2000954, SKMUS2000961, SKMUS2000973, SKMUS2000980, SKMUS2001014, SKMUS2001164, SKMUS2001199, SKMUS2001215, SKMUS2001247, SKMUS2001254, SKMUS2001271, SKMUS2001287, SKMUS2001323, SKMUS2001364, SKMUS2001398, SKMUS2001412, SKMUS2001492, SKMUS2001501, SKMUS2001528, SKMUS2001543, SKMUS2001580, SKMUS2001585, SKMUS2001608, SKMUS2001631, SKMUS2001662, SKMUS2001663, SKMUS2001671, SKMUS2001683, SKMUS2001703, SKMUS2001740, SKMUS2001742, SKMUS2001823, SKMUS2001850, SKMUS2002077,
SKMUS2002084, SKMUS2002153, SKMUS2002475, SKMUS2002602, SKMUS2002693, SKMUS2002821, SKMUS2002840, SKMUS2002920, SKMUS2003034, SKMUS2003074, SKMUS2003194, SKMUS2003744, SKMUS2004044, SKMUS2004047, SKMUS2004483, SKMUS2004897, SKMUS2004908, SKMUS2005428, SKMUS2006394, SKMUS2006481, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC1000229, SKNMC1000251, SKNMC1000264, SKNMC2000065, SKNMC2000224, SKNMC2000256, SKNMC2000305, SKNMC2000356, SKNMC2000374, SKNMC2000583, SKNMC2000593,
SKNMC2000612, SKNMC2000622, SKNMC2001113, SKNMC2001324, SKNMC2001503, SKNMC2001555, SKNMC2001596, SKNMC2001623, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000482, SKNSH2000516, SKNSH2000550, SKNSH2000716, SKNSH2000819, SKNSH2000971, SKNSH2001222, SKNSH2001875, SKNSH2001931, SKNSH2002054, SKNSH2002325, SKNSH2002866, SKNSH2003064, SKNSH2003174, SKNSH2003422, SKNSH2003466,
SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008969, SKNSH2009197, SKNSH2009357, SKNSH2009435, SKNSH2009991, SKNSH2010015, SMINT1000012, SMINT1000016, SMINT1000039, SMINT1000048, SMINT1000051, SMINT1000054, SMINT1000057, SMINT1000071, SMINT1000075, SMINT1000091, SMINT1000103, SMINT1000118, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227,
SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000558, SMINT2000562, SMINT2000568, SMINT2000609, SMINT2000629, SMINT2000659, SMINT2000694, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002159, SMINT2002202, SMINT2002210, SMINT2002224, SMINT2002281, SMINT2002311, SMINT2002314, SMINT2002328, SMINT2002414,
SMINT2002457, SMINT2002743, SMINT2002778, SMINT2002880, SMINT2002882, SMINT2002884, SMINT2002899, SMINT2002974, SMINT2003003, SMINT2003074, SMINT2003128, SMINT2003150, SMINT2003169, SMINT2003317, SMINT2003340, SMINT2003386, SMINT2003423, SMINT2003551, SMINT2003569, SMINT2003641, SMINT2003644, SMINT2003866, SMINT2003905, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004589, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005387,
SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2006801, SMINT2007062, SMINT2007101, SMINT2007140, SMINT2007187, SMINT2007219, SMINT2007294, SMINT2007365, SMINT2007391, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008625, SMINT2008672, SMINT2008690, SMINT2008715, SMINT2008720,
SMINT2008817, SMINT2008844, SMINT2008869, SMINT2009119, SMINT2009121, SMINT2009212, SMINT2009233, SMINT2009259, SMINT2009272, SMINT2009292, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011406, SMINT2011509, SMINT2011588, SMINT2011656, SMINT2011888, SMINT2011958, SMINT2012040, SMINT2012179,
SMINT2012195, SMINT2012285, SMINT2012291, SMINT2012501, SMINT2012734, SMINT2012793, SMINT2013031, SMINT2013032, SMINT2013129, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659,
SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142, SMINT2019153, SMINT2019200, SPLEN1000041, SPLEN1000049, SPLEN1000056, SPLEN1000087, SPLEN1000089, SPLEN1000091, SPLEN1000106, SPLEN1000116, SPLEN1000134, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000242, SPLEN2000243, SPLEN2000247, SPLEN2000255, SPLEN2000258, SPLEN2000267, SPLEN2000270, SPLEN2000307,
SPLEN2000310, SPLEN2000341, SPLEN2000350, SPLEN2000357, SPLEN2000364, SPLEN2000402, SPLEN2000414, SPLEN2000420, SPLEN2000448, SPLEN2000450, SPLEN2000464, SPLEN2000496, SPLEN2000505, SPLEN2000515, SPLEN2000516, SPLEN2000537, SPLEN2000541, SPLEN2000607, SPLEN2000630, SPLEN2000695, SPLEN2000698, SPLEN2000839, SPLEN2000874, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001689, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175,
SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002662, SPLEN2002695, SPLEN2002707, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004220, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005009, SPLEN2005227, SPLEN2005272, SPLEN2005342, SPLEN2005416, SPLEN2005450, SPLEN2005474, SPLEN2005560, SPLEN2005727,
SPLEN2005783, SPLEN2005790, SPLEN2005806, SPLEN2005818, SPLEN2005838, SPLEN2005869, SPLEN2005927, SPLEN2005939, SPLEN2006143, SPLEN2006268, SPLEN2006283, SPLEN2006325, SPLEN2006374, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007498, SPLEN2007619, SPLEN2007653, SPLEN2007689, SPLEN2007695, SPLEN2007879, SPLEN2007926, SPLEN2007951, SPLEN2008007, SPLEN2008135, SPLEN2008164, SPLEN2008317, SPLEN2008496, SPLEN2008591, SPLEN2008786, SPLEN2009081, SPLEN2009088, SPLEN2009163, SPLEN2009315, SPLEN2009340, SPLEN2009512, SPLEN2009513, SPLEN2009541, SPLEN2009548, SPLEN2009555,
SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011758, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234,
SPLEN2012453, SPLEN2012524, SPLEN2012525, SPLEN2012571, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014080, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014572, SPLEN2014645, SPLEN2014669, SPLEN2014711, SPLEN2014739, SPLEN2014860,
SPLEN2014865, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016421, SPLEN2016480, SPLEN2016531, SPLEN2016533, SPLEN2016541, SPLEN2016552, SPLEN2016602, SPLEN2016627, SPLEN2016678, SPLEN2016743,
SPLEN2016773, SPLEN2016781, SPLEN2016840, SPLEN2016863, SPLEN2016922, SPLEN2016932, SPLEN2016972, SPLEN2016973, SPLEN2017043, SPLEN2017104, SPLEN2017121, SPLEN2017147, SPLEN2017187, SPLEN2017189, SPLEN2017212, SPLEN2017318, SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018098, SPLEN2018112, SPLEN2018159, SPLEN2018180, SPLEN2018181, SPLEN2018280, SPLEN2018285, SPLEN2018299, SPLEN2018302, SPLEN2018364,SPLEN2018395, SPLEN2018643, SPLEN2018683, SPLEN2018732, SPLEN2018749, SPLEN2018933, SPLEN2019008, SPLEN2019043, SPLEN2019092, SPLEN2019102, SPLEN2019131, SPLEN2019169,
SPLEN2019257, SPLEN2019311, SPLEN2019323, SPLEN2019349, SPLEN2019375, SPLEN2019379, SPLEN2019405, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021194, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701,
SPLEN2021795, SPLEN2021983, SPLEN2021991, SPLEN2022040, SPLEN2022162, SPLEN2022227, SPLEN2022522, SPLEN2022591, SPLEN2022785, SPLEN2022920, SPLEN2023024, SPLEN2023118, SPLEN2023160, SPLEN2023423, SPLEN2023710, SPLEN2023733, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066,
SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522, SPLEN2029683, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031547, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551,
SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038473, SPLEN2039311, SPLEN2039379, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303,
SPLEN2042521, SPLEN2042598, SPLEN2042714, STOMA1000013, STOMA1000047, STOMA1000052, STOMA1000074, STOMA1000091, STOMA1000117, STOMA1000133, STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000289, STOMA2000383, STOMA2000386, STOMA2000395, STOMA2000396, STOMA2000478, STOMA2000529, STOMA2000539, STOMA2000567, STOMA2000640, STOMA2000678, STOMA2000686, STOMA2000888, STOMA2001025, STOMA2001389, STOMA2002052, STOMA2002141, STOMA2002367, STOMA2002688, STOMA2002828, STOMA2003158, STOMA2003289, STOMA2003444, STOMA2003477, STOMA2003646, STOMA2003716, STOMA2003781,
STOMA2003894, STOMA2004194, STOMA2004294, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213, STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291,
SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300, SYNOV2001356, SYNOV2001390, SYNOV2001451, SYNOV2001457, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017055, SYNOV2017179, SYNOV2018921, SYNOV2019280, SYNOV2020085, SYNOV2020463, SYNOV2021320,
SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2000904, TBAES2000004,
TBAES2000059, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000127, TESOP1000160,
TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006060, TESOP2006068, TESOP2006110, TESOP2006398, TESOP2006670, TESOP2006704,
TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055, TESTI1000070, TESTI1000085, TESTI1000088, TESTI1000093, TESTI1000094, TESTI1000096, TESTI1000109, TESTI1000125, TESTI1000127, TESTI1000128, TESTI1000131, TESTI1000138, TESTI1000157, TESTI1000168, TESTI1000169, TESTI1000179, TESTI1000183, TESTI1000191, TESTI1000257, TESTI1000266, TESTI1000319,
TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000177, TESTI2000179, TESTI2000207, TESTI2000208, TESTI2000221, TESTI2000238, TESTI2000253, TESTI2000272, TESTI2000278, TESTI2000356, TESTI2000358, TESTI2000372, TESTI2000427, TESTI2000431, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808,
TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815,
TESTI2001823, TESTI2001826, TESTI2001827, TESTI2001852, TESTI2001862, TESTI2001869, TESTI2001879, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618,
TESTI2002632, TESTI2002676, TESTI2002690, TESTI2002696, TESTI2002698, TESTI2002700, TESTI2002707, TESTI2002724, TESTI2002729, TESTI2002752, TESTI2002753, TESTI2002789, TESTI2002802, TESTI2002806, TESTI2002840, TESTI2002866, TESTI2002877, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280,
TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003454, TESTI2003475, TESTI2003476, TESTI2003498, TESTI2003512, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080,
TESTI2004085, TESTI2004089, TESTI2004111, TESTI2004119, TESTI2004122, TESTI2004163, TESTI2004169, TESTI2004207, TESTI2004215, TESTI2004227, TESTI2004229, TESTI2004243, TESTI2004255, TESTI2004287, TESTI2004295, TESTI2004313, TESTI2004318, TESTI2004322, TESTI2004391, TESTI2004399, TESTI2004423, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004737, TESTI2004783, TESTI2004791, TESTI2004793, TESTI2004860, TESTI2004906, TESTI2004920, TESTI2004936, TESTI2004941, TESTI2004971,
TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005743, TESTI2005759, TESTI2005767,
TESTI2005775, TESTI2005827, TESTI2005835, TESTI2005860, TESTI2005892, TESTI2005908, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006457, TESTI2006460, TESTI2006465,
TESTI2006483, TESTI2006499, TESTI2006523, TESTI2006543, TESTI2006565, TESTI2006572, TESTI2006615, TESTI2006617, TESTI2006624, TESTI2006643, TESTI2006648, TESTI2006659, TESTI2006660, TESTI2006665, TESTI2006667, TESTI2006677, TESTI2006720, TESTI2006735, TESTI2006744, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007422, TESTI2007452, TESTI2007464,
TESTI2007466, TESTI2007480, TESTI2007490, TESTI2007502, TESTI2007524, TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008320, TESTI2008331, TESTI2008343, TESTI2008387, TESTI2008389, TESTI2008394, TESTI2008405, TESTI2008420, TESTI2008425, TESTI2008440,
TESTI2008567, TESTI2008595, TESTI2008636, TESTI2008657, TESTI2008684, TESTI2008699, TESTI2008715, TESTI2008762, TESTI2008774, TESTI2008786, TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835,
TESTI2009853, TESTI2009935, TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846,
TESTI2011915, TESTI2011928, TESTI2011971, TESTI2012019, TESTI2012050, TESTI2012090, TESTI2012095, TESTI2012104, TESTI2012152, TESTI2012171, TESTI2012207, TESTI2012231, TESTI2012244, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013264, TESTI2013268, TESTI2013295, TESTI2013322, TESTI2013381, TESTI2013382, TESTI2013401, TESTI2013427, TESTI2013468,
TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014838, TESTI2014843, TESTI2014979, TESTI2014988, TESTI2015042, TESTI2015105, TESTI2015180, TESTI2015246, TESTI2015249, TESTI2015327, TESTI2015331, TESTI2015335, TESTI2015350,
TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113,
TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042, TESTI2019077, TESTI2019228,
TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020840, TESTI2020871, TESTI2020905, TESTI2020906, TESTI2020956, TESTI2020981, TESTI2020999,
TESTI2021003, TESTI2021038, TESTI2021057, TESTI2021077, TESTI2021112, TESTI2021114, TESTI2021116, TESTI2021118, TESTI2021122, TESTI2021124, TESTI2021138, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025,
TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024864, TESTI2024885, TESTI2024936, TESTI2024978, TESTI2024999, TESTI2025006, TESTI2025013, TESTI2025022, TESTI2025063,
TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605,
TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583,
TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919,
TESTI2031935, TESTI2031986, TESTI2032044, TESTI2032067, TESTI2032079, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183,
TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877,
TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983,
TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044306, TESTI2044309, TESTI2044413, TESTI2044418, TESTI2044754, TESTI2044788,
TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047605, TESTI2047792, TESTI2047801, TESTI2047818, TESTI2047824, TESTI2047885, TESTI2047930, TESTI2048105, TESTI2048109,
TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527, TESTI4001561, TESTI4001569, TESTI4001604, TESTI4001665, TESTI40.01679,
TESTI4001795, TESTI4001923, TESTI4001925, TESTI4001984, TESTI4002003, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317, TESTI4005322, TESTI4005399,
TESTI4005470, TESTI4005500, TESTI4005534, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965, TESTI4008007,
TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894,
TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229,
TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294,.TESTI4021377, TESTI4021456, TESTI4021491,
TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762,
TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270,
TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339,
TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371,
TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000017,
THYMU1000025, THYMU1000029, THYMU1000033, THYMU1000041, THYMU1000060, THYMU1000074, THYMU1000098, THYMU1000103, THYMU1000105, THYMU1000109, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000329, THYMU1000331, THYMU1000336, THYMU1000366, THYMU1000374, THYMU1000382, THYMU1000393, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022,
THYMU2000032, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001195, THYMU2001202, THYMU2001203, THYMU2001256, THYMU2001270,
THYMU2001291, THYMU2001314, THYMU2001325, THYMU2001357, THYMU2001381, THYMU2001422, THYMU2001443, THYMU2001515, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002242, THYMU2002338, THYMU2002356, THYMU2002376, THYMU2002384, THYMU2002450, THYMU2002490, THYMU2002548, THYMU2002695, THYMU2002756, THYMU2002789, THYMU2002792, THYMU2002815, THYMU2002852, THYMU2002910, THYMU2002950, THYMU2002983, THYMU2003107, THYMU2003124, THYMU2003166,
THYMU2003232, THYMU2003242, THYMU2003249, THYMU2003270, THYMU2003282, THYMU2003287, THYMU2003336, THYMU2003366, THYMU2003397, THYMU2003440, THYMU2003446, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004111, THYMU2004123, THYMU2004139, THYMU2004169, THYMU2004284, THYMU2004336, THYMU2004356, THYMU2004452, THYMU2004508, THYMU2004512, THYMU2004635, THYMU2004639, THYMU2004668, THYMU2004677, THYMU2004688, THYMU2004693, THYMU2004835, THYMU2004843, THYMU2004906, THYMU2004916, THYMU2004986, THYMU2005001, THYMU2005003, THYMU2005046,
THYMU2005134, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965, THYMU2007025, THYMU2007060, THYMU2007095,
THYMU2007146, THYMU2007179, THYMU2007258, THYMU2007307, THYMU2007308, THYMU2007339, THYMU2007350, THYMU2007384, THYMU2007415, THYMU2007467, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452, THYMU2008643, THYMU2008686, THYMU2008691, THYMU2008714,
THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010161, THYMU2010192, THYMU2010448, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072, THYMU2011096, THYMU2011118, THYMU2011142,
THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013170, THYMU2013364,
THYMU2013386, THYMU2013426, THYMU2013578, THYMU2013705, THYMU2013757, THYMU2013779, THYMU2013863, THYMU2013950, THYMU2013981, THYMU2014067, THYMU2014068, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099,
THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830,
THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894,
THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031368, THYMU2031579, THYMU2031847, THYMU2031890, THYMU2031994, THYMU2032014, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976,
THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189,
THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841,
THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137,
THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962,
THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024602, THYMU3024879, THYMU3025118, THYMU3025313, THYMU3025683, THYMU3025772, THYMU3025865, THYMU3026000, THYMU3026350, THYMU3026479, THYMU3026532, THYMU3026783, THYMU3026869,
THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032798, THYMU3032867, THYMU3033402, THYMU3033626, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827,
THYMU3037836, THYMU3037856, THYMU3037867, THYMU3037909, THYMU3037980, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175,
THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003601, TKIDN2004185, TKIDN2004386, TKIDN2004458, TKIDN2004748, TKIDN2005487, TKIDN2005721, TKIDN2005934,
TKIDN2005947, TKIDN2005956, TKIDN2006525, TKIDN2006761, TKIDN2006852, TKIDN2007605, TKIDN2007667, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616,
TLIVE2001684, TLIVE2001828, TLIVE2001927, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575,
TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000125, TRACH1000140, TRACH1000144, TRACH1000181, TRACH1000193, TRACH1000212, TRACH1000240, TRACH2000002, TRACH2000015, TRACH2000024, TRACH2000032, TRACH2000045, TRACH2000047, TRACH2000083, TRACH2000148, TRACH2000185, TRACH2000196, TRACH2000235, TRACH2000237, TRACH2000243, TRACH2000250, TRACH2000259, TRACH2000272, TRACH2000282, TRACH2000287, TRACH2000289, TRACH2000306, TRACH2000312, TRACH2000336, TRACH2000359, TRACH2000393, TRACH2000411, TRACH2000461, TRACH2000496, TRACH2000497, TRACH2000502,
TRACH2000522, TRACH2000540, TRACH2000559, TRACH2000660, TRACH2000665, TRACH2000675, TRACH2000677, TRACH2000703, TRACH2000767, TRACH2000775, TRACH2000780, TRACH2000807, TRACH2000862, TRACH2000873, TRACH2000894, TRACH2000898, TRACH2000920, TRACH2000923, TRACH2000926, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001192, TRACH2001249, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001474, TRACH2001525, TRACH2001532, TRACH2001549, TRACH2001592, TRACH2001596, TRACH2001621,
TRACH2001646, TRACH2001651, TRACH2001684, TRACH2001810, TRACH2001933, TRACH2001968, TRACH2001996, TRACH2002043, TRACH2002054, TRACH2002100, TRACH2002138, TRACH2002333, TRACH2002784, TRACH2002803, TRACH2002840, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004039, TRACH2004054, TRACH2004087, TRACH2004109, TRACH2004170, TRACH2004183, TRACH2004251, TRACH2004292, TRACH2004336, TRACH2004399, TRACH2004428, TRACH2004458, TRACH2004521, TRACH2004570, TRACH2004845, TRACH2004887, TRACH2004950, TRACH2005004, TRACH2005017, TRACH2005066, TRACH2005159,
TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006866, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007894, TRACH2007969, TRACH2007988, TRACH2008081, TRACH2008112, TRACH2008113, TRACH2008127, TRACH2008130, TRACH2008278, TRACH2008300, TRACH2008478, TRACH2008540, TRACH2008583, TRACH2008597, TRACH2008716, TRACH2008820, TRACH2008921,
TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011574, TRACH2011894, TRACH2011956, TRACH2012000, TRACH2012138, TRACH2012242, TRACH2012298, TRACH2012370, TRACH2012376, TRACH2012387,
TRACH2012497, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2014018, TRACH2014045, TRACH2014046, TRACH2014077, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015987,
TRACH2016080, TRACH2016131, TRACH2016286, TRACH2016317, TRACH2016347, TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017498, TRACH2017609, TRACH2017949, TRACH2017965, TRACH2017980, TRACH2018034, TRACH2018084, TRACH2018262, TRACH2018273, TRACH2018278, TRACH2018345, TRACH2018380, TRACH2018449, TRACH2018512, TRACH2018646, TRACH2018663, TRACH2018718, TRACH2018835, TRACH2018882, TRACH2018914, TRACH2018950, TRACH2019024, TRACH2019046, TRACH2019080, TRACH2019151, TRACH2019154, TRACH2019176, TRACH2019248, TRACH2019309, TRACH2019425,
TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535,
TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780,
TRACH3003805, TRACH3003832, TRACH3003872, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005211, TRACH3005274, TRACH3005294, TRACH3005479, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699,
TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008688, TRACH3008697, TRACH3008713, TRACH3008887, TRACH3008902, TRACH3008990, TRACH3009001, TRACH3009008, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313,
TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016992,
TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758,
TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451,
TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103,
TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696, TRACH3037897, TRACH3038086, TRACH3038399, TSTOM1000135, TSTOM1000186, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000916, TUTER2001286, TUTER2001341, TUTER2001387,
TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN2000046, UMVEN2000069, UMVEN2000121, UMVEN2000133, UMVEN2000152, UMVEN2000354, UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000260,
UTERU2000329, UTERU2000332, UTERU2000349, UTERU2000377, UTERU2000393, UTERU2000418, UTERU2000465, UTERU2000485, UTERU2000517, UTERU2000524, UTERU2000539, UTERU2000541, UTERU2000546, UTERU2000550, UTERU2000569, UTERU2000607, UTERU2000629, UTERU2000649, UTERU2000663, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001747, UTERU2001876, UTERU2002011, UTERU2002176, UTERU2002198, UTERU2002294, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993,
UTERU2003035, UTERU2003057, UTERU2003126, UTERU2003135, UTERU2003321, UTERU2003399, UTERU2003411, UTERU2003456, UTERU2003577, UTERU2003973, UTERU2004015, UTERU2004039, UTERU2004061, UTERU2004073, UTERU2004163, UTERU2004299, UTERU2004461, UTERU2004520, UTERU2004564, UTERU2004664, UTERU2004688, UTERU2004698, UTERU2004861, UTERU2005004, UTERU2005050, UTERU2005069, UTERU2005074, UTERU2005179, UTERU2005292, UTERU2005346, UTERU2005446, UTERU2005449, UTERU2005450, UTERU2005533, UTERU2005593, UTERU2005601, UTERU2005621, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547,
UTERU2006568, UTERU2006643, UTERU2006651, UTERU2006705, UTERU2006899, UTERU2007004, UTERU2007075, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009538, UTERU2009540, UTERU2009776, UTERU2009904, UTERU2009951,
UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012890, UTERU2012938, UTERU2012976, UTERU2013048, UTERU2013078,
UTERU2013231, UTERU2013262, UTERU2013280, UTERU2013322, UTERU2013483, UTERU2013491, UTERU2013586, UTERU2013926, UTERU2013976, UTERU2014001, UTERU2014024, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016896, UTERU2016902, UTERU2016981, UTERU2017123, UTERU2017303, UTERU2017421, UTERU2017623, UTERU2017632, UTERU2017761, UTERU2017762,
UTERU2017810, UTERU2017988, UTERU2018127, UTERU2018180, UTERU2018200, UTERU2018333, UTERU2018364, UTERU2018514, UTERU2018522, UTERU2018544, UTERU2018566, UTERU2018609, UTERU2018674, UTERU2018712, UTERU2018784, UTERU2018789, UTERU2018811, UTERU2018867, UTERU2018881, UTERU2018955, UTERU2019038, UTERU2019096, UTERU2019163, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023550, UTERU2023651,
UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279,
UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844,
UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422,
UTERU3005460, UTERU3005536, UTERU3005585, UTERU3005767, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010892, UTERU3010919, UTERU3011063, UTERU3011092, UTERU3011273, UTERU3011398, UTERU3011558, UTERU3011579, UTERU3011795, UTERU3011837, UTERU3012293,
UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647; UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018172, UTERU3018255,
UTERU3018711, UTERU3019078

Of the 17,176 genes that include the cDNAs of the present invention, genes whose expression levels were high enough to detect in each of the SALGL, SKMUS, SKNMC, SKNSH, SMINT, SPLEN, and STOMA libraries were analyzed for expression frequency. The analysis results are described below. Each is a relative value, where the expression level over all tissues is taken as 100. The larger this value, the greater the level of expression.

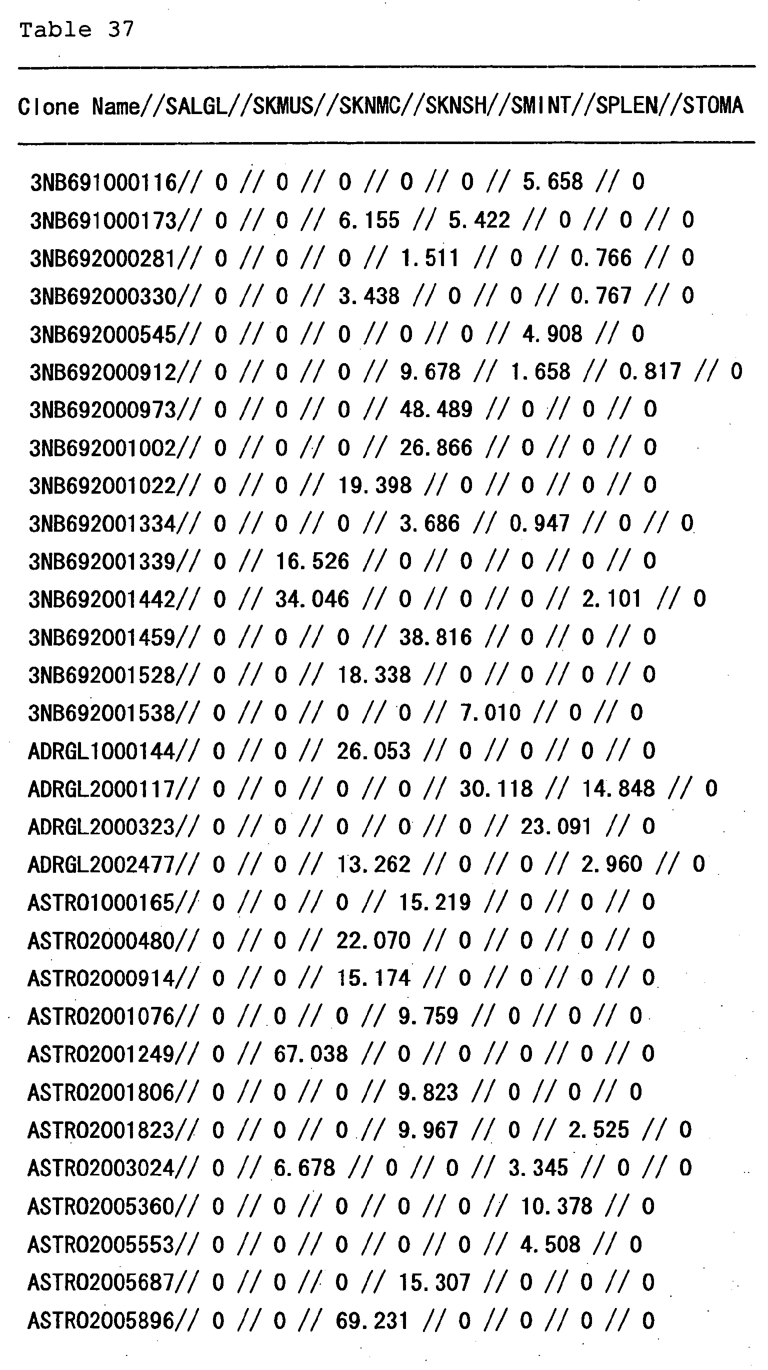

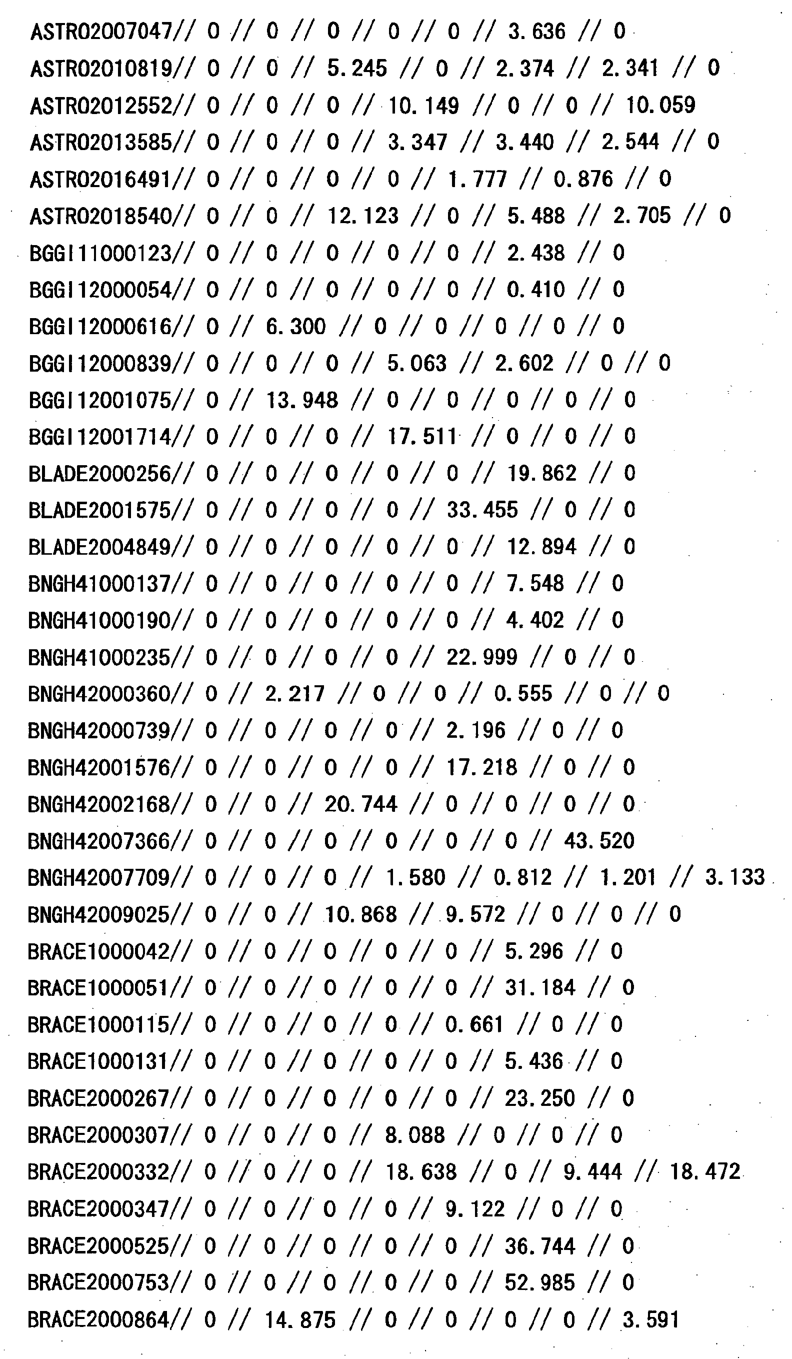

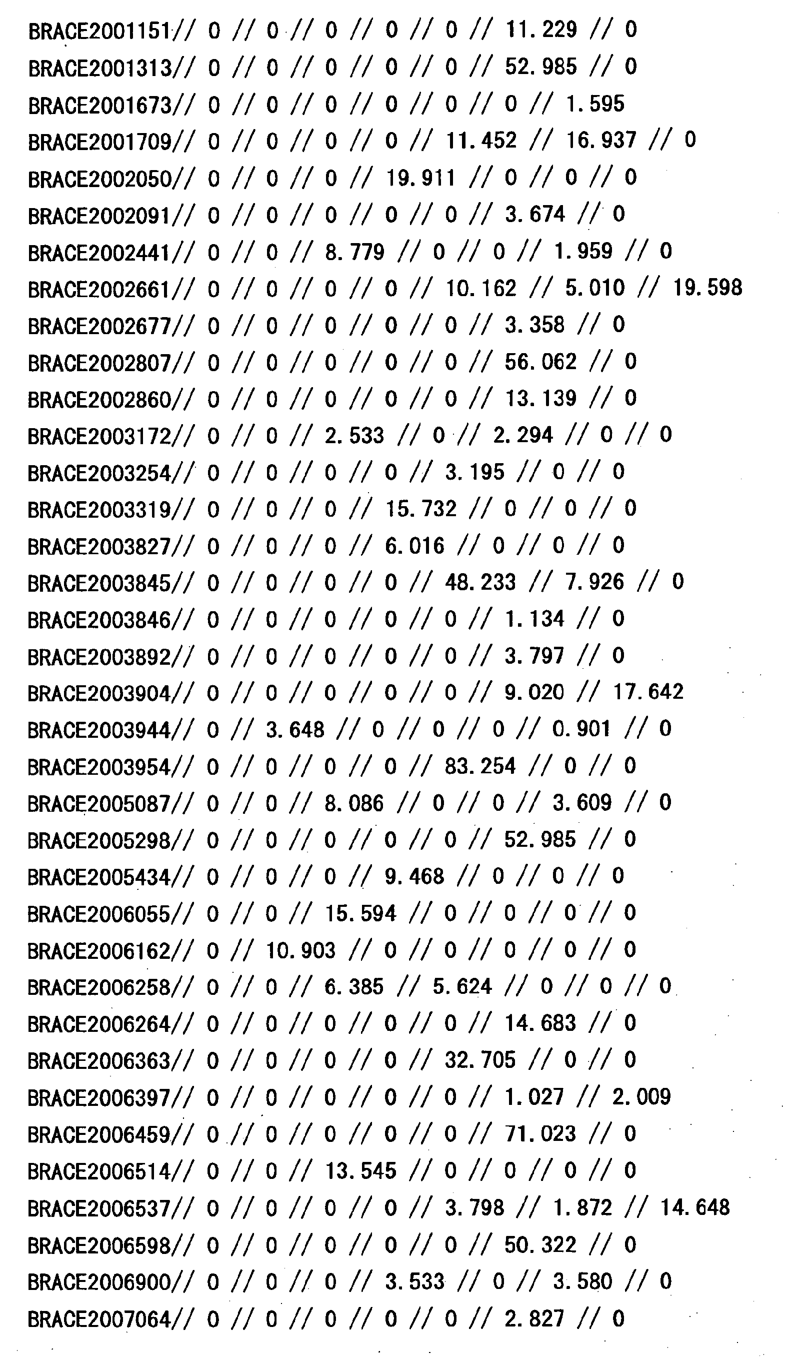

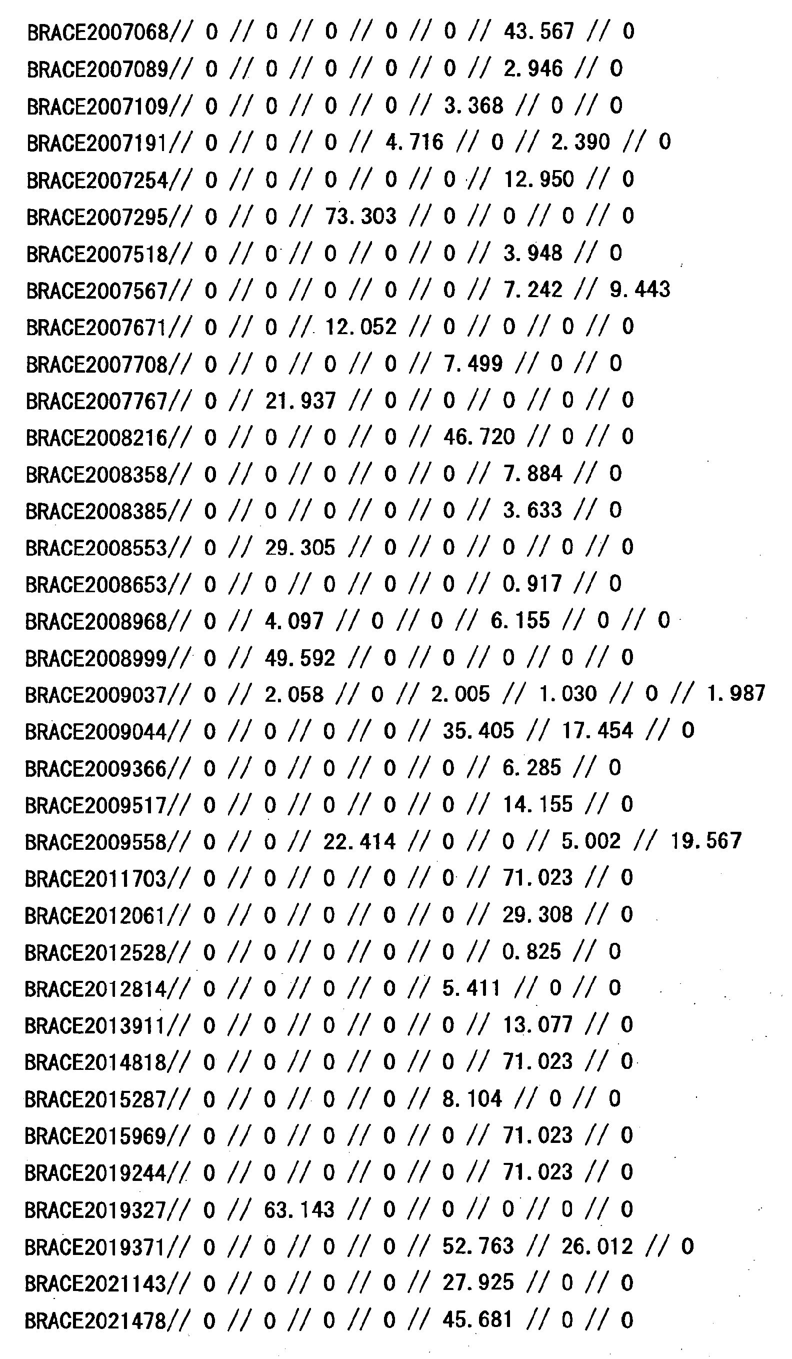

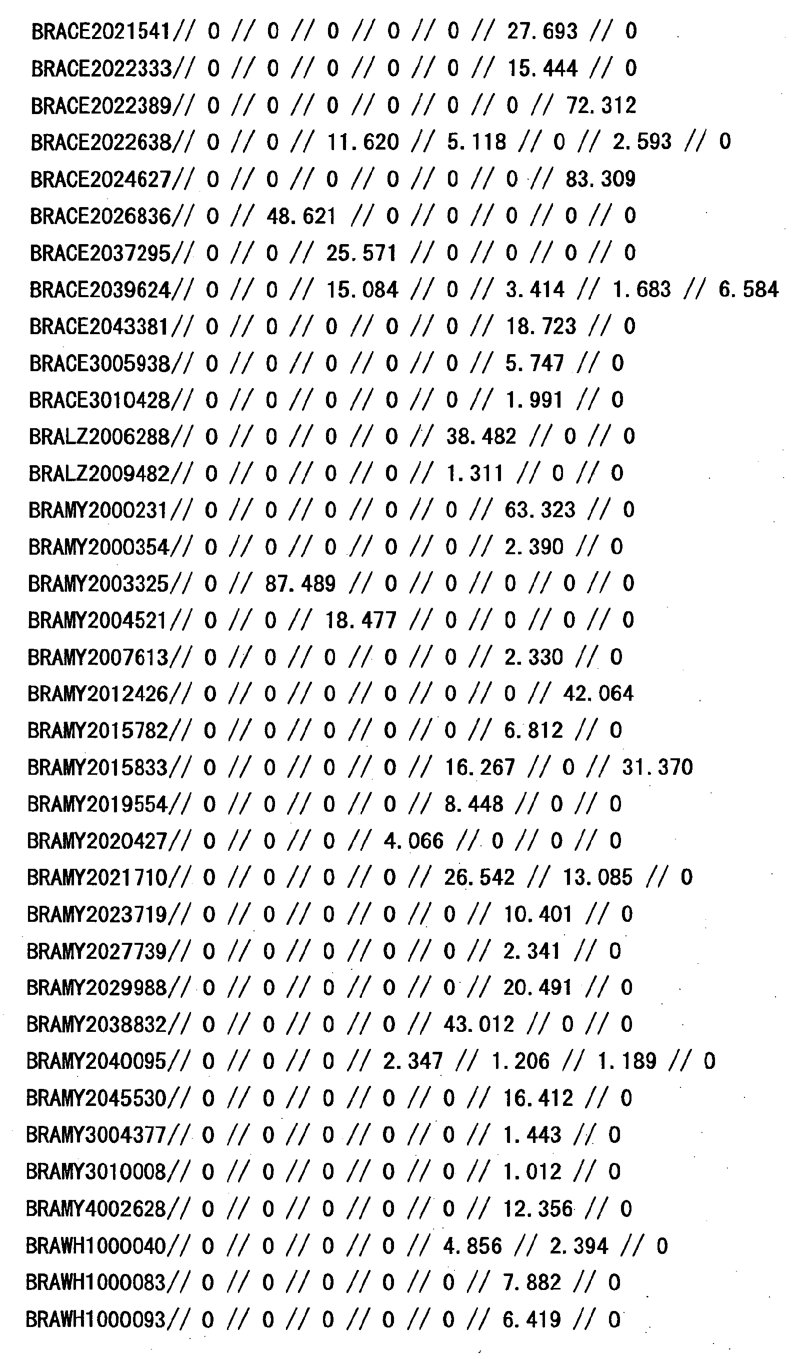

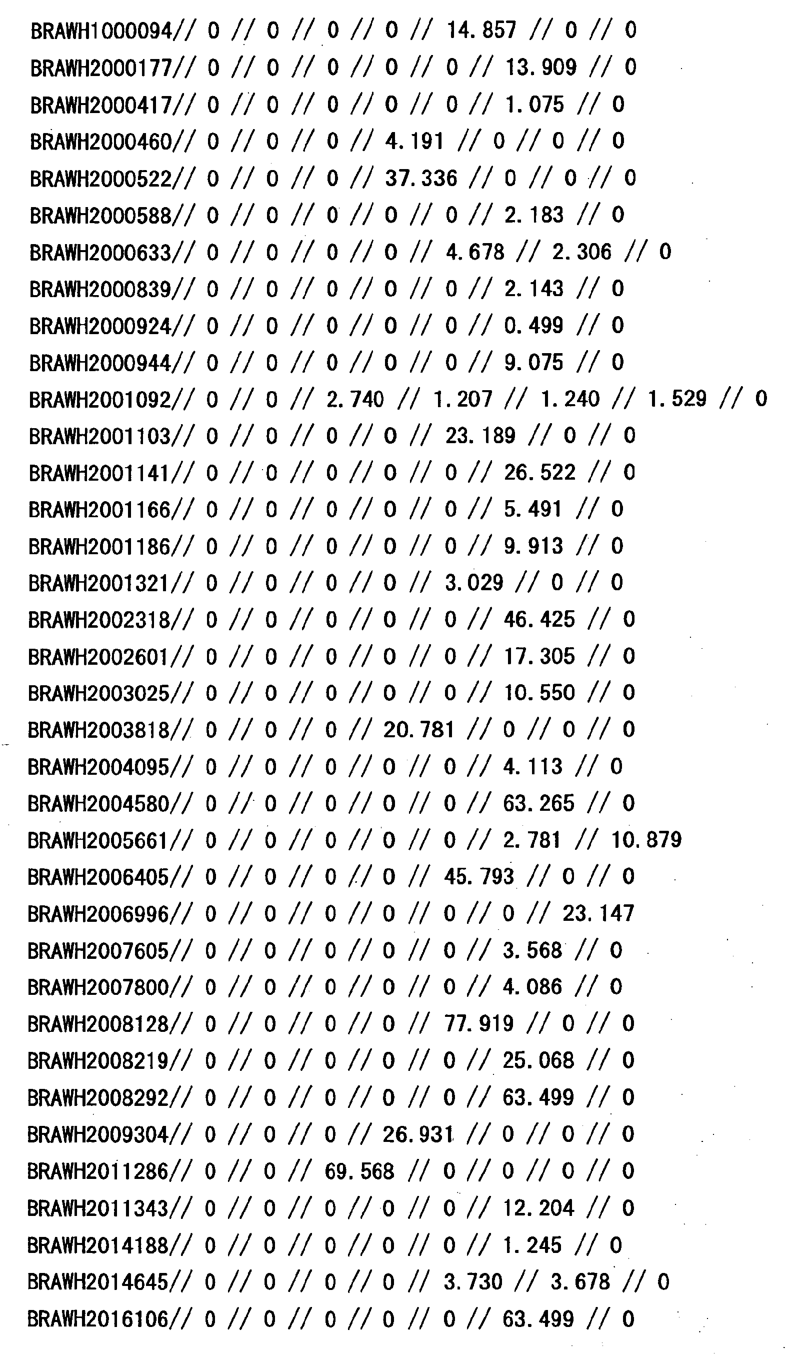

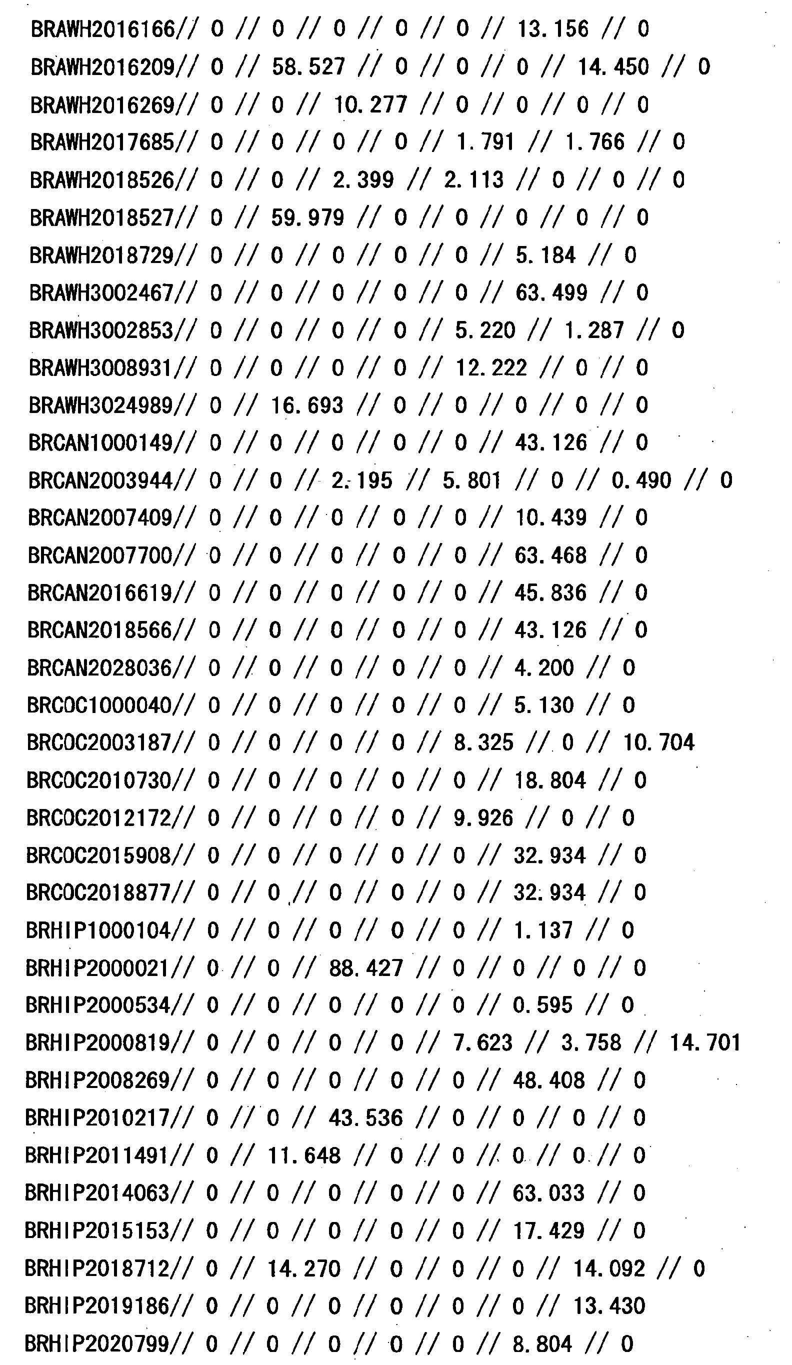

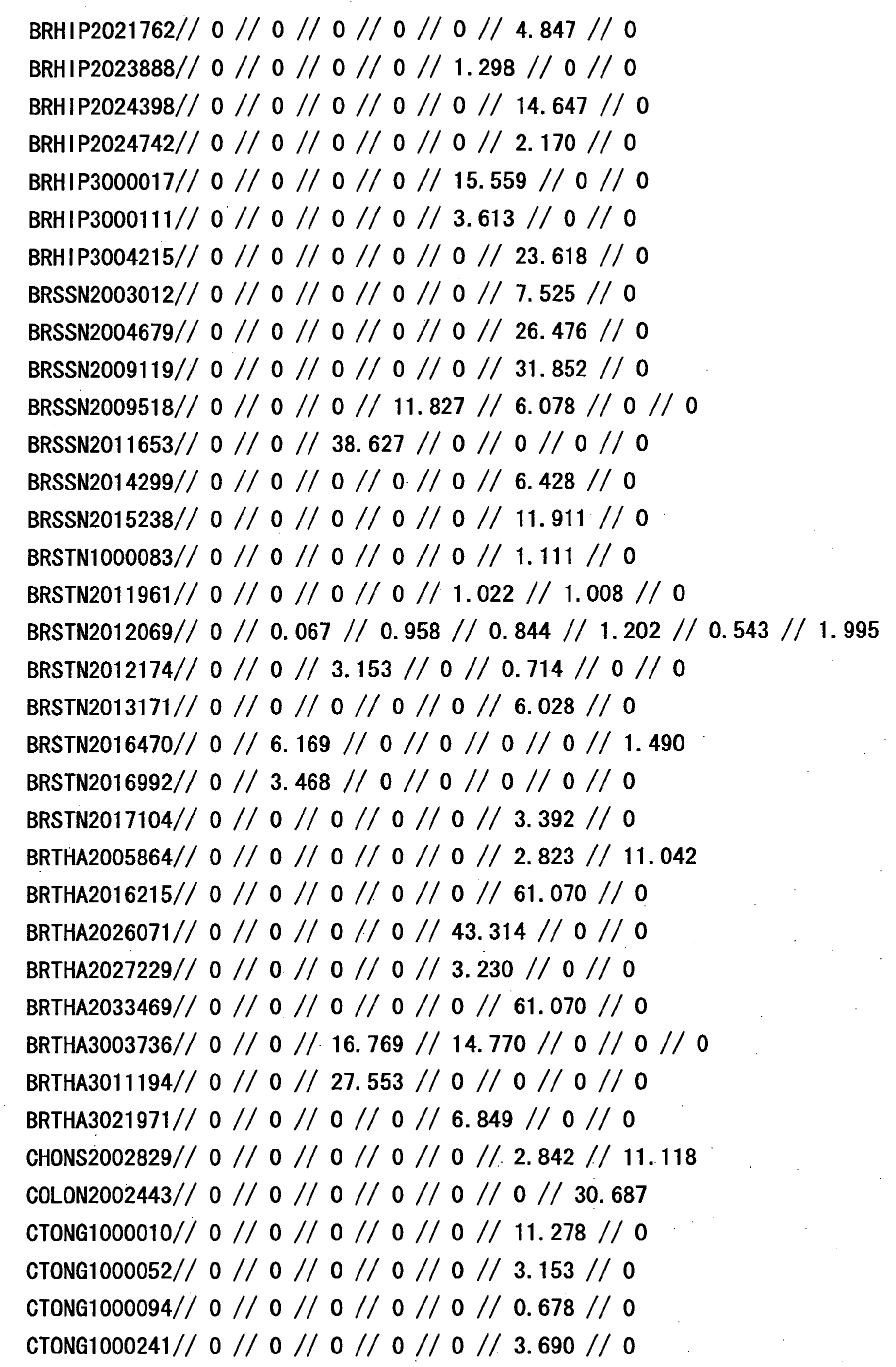

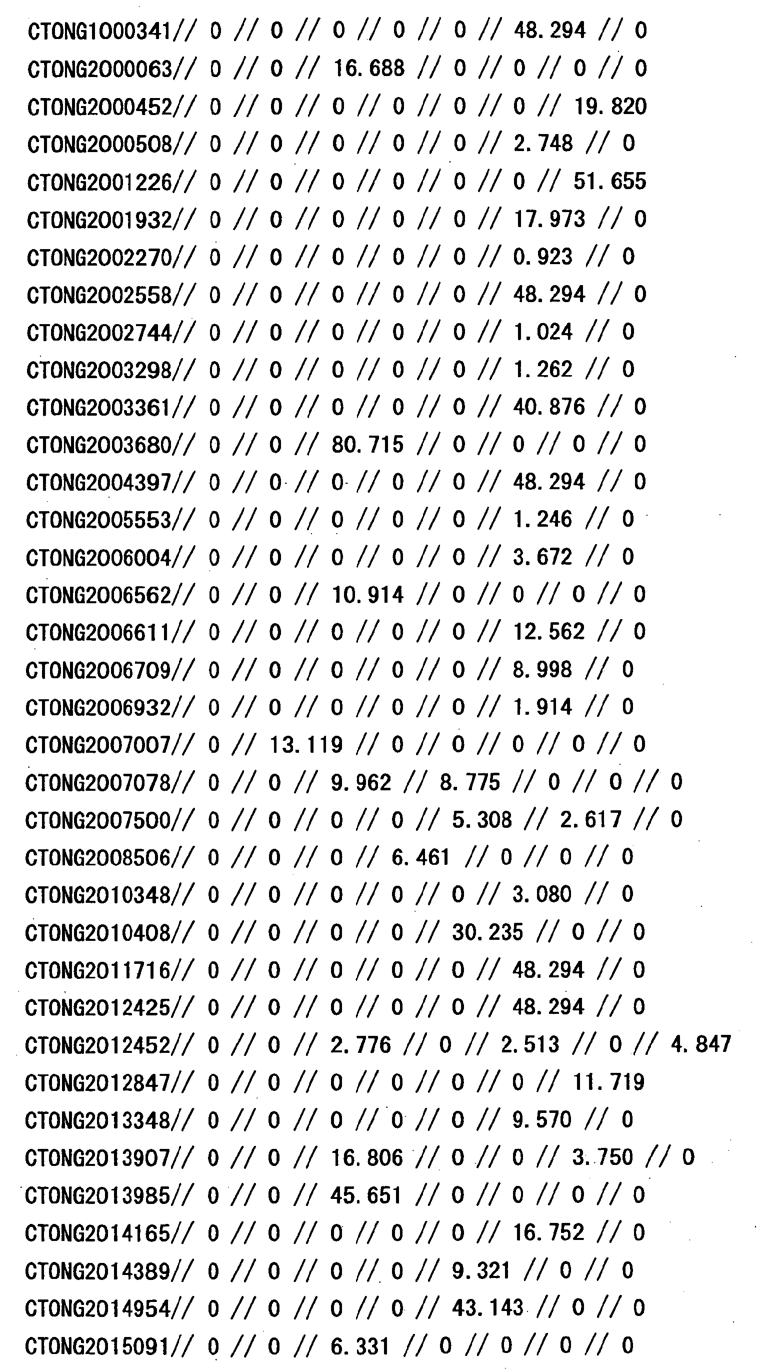

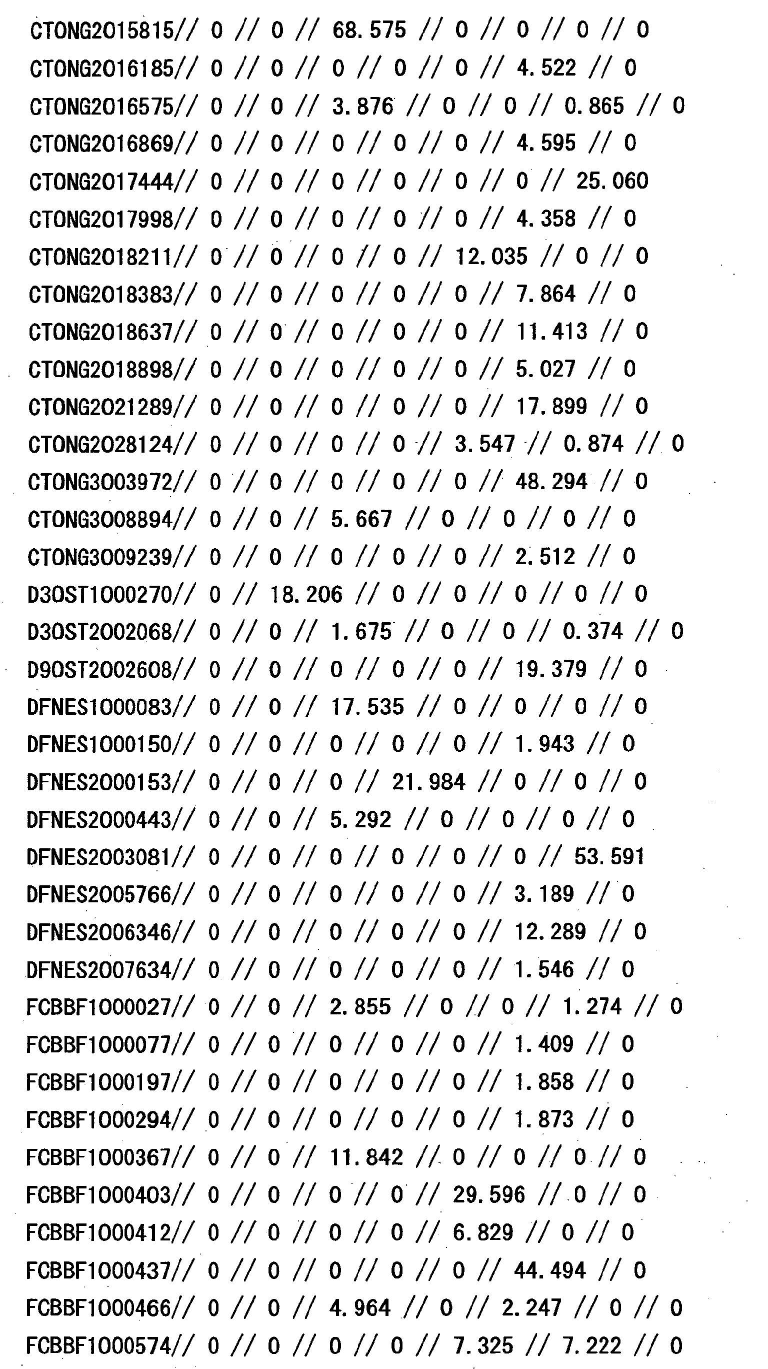

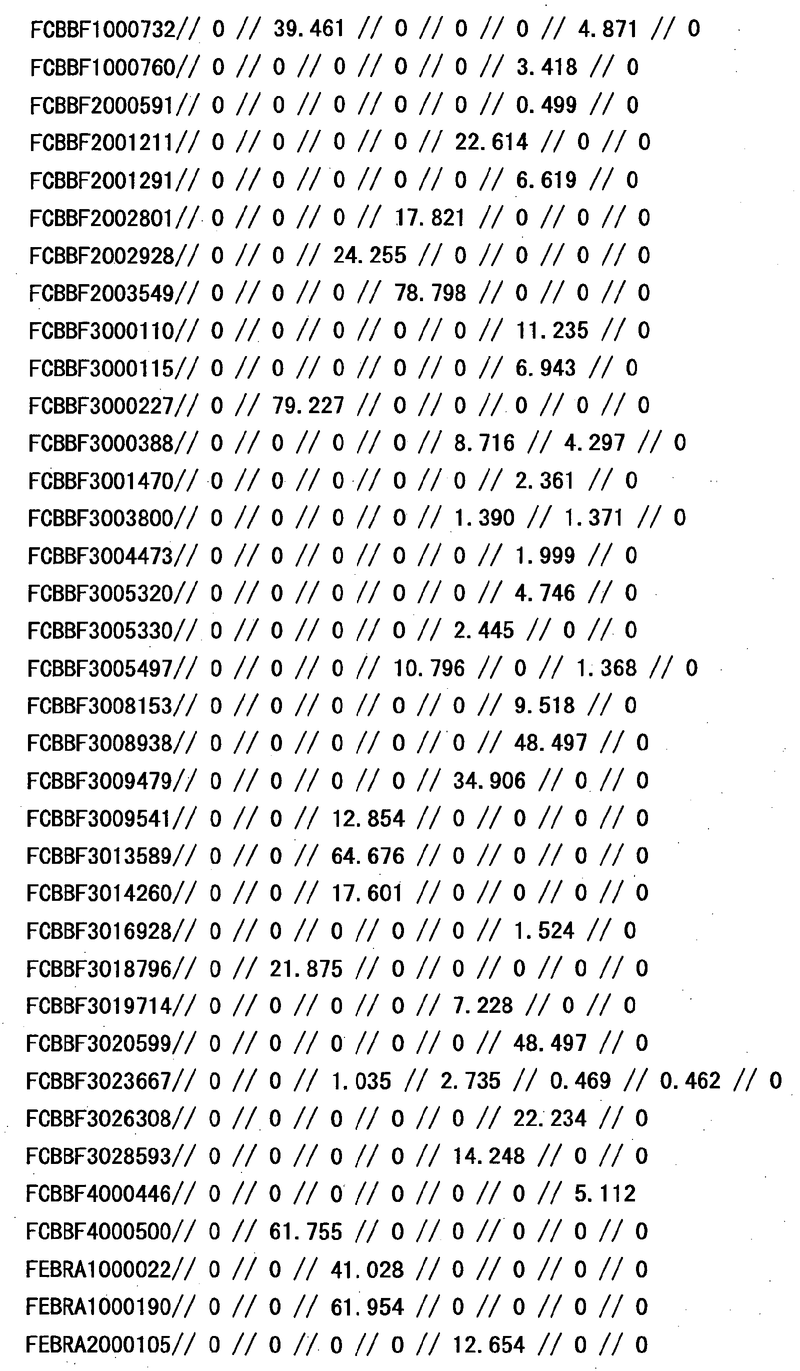

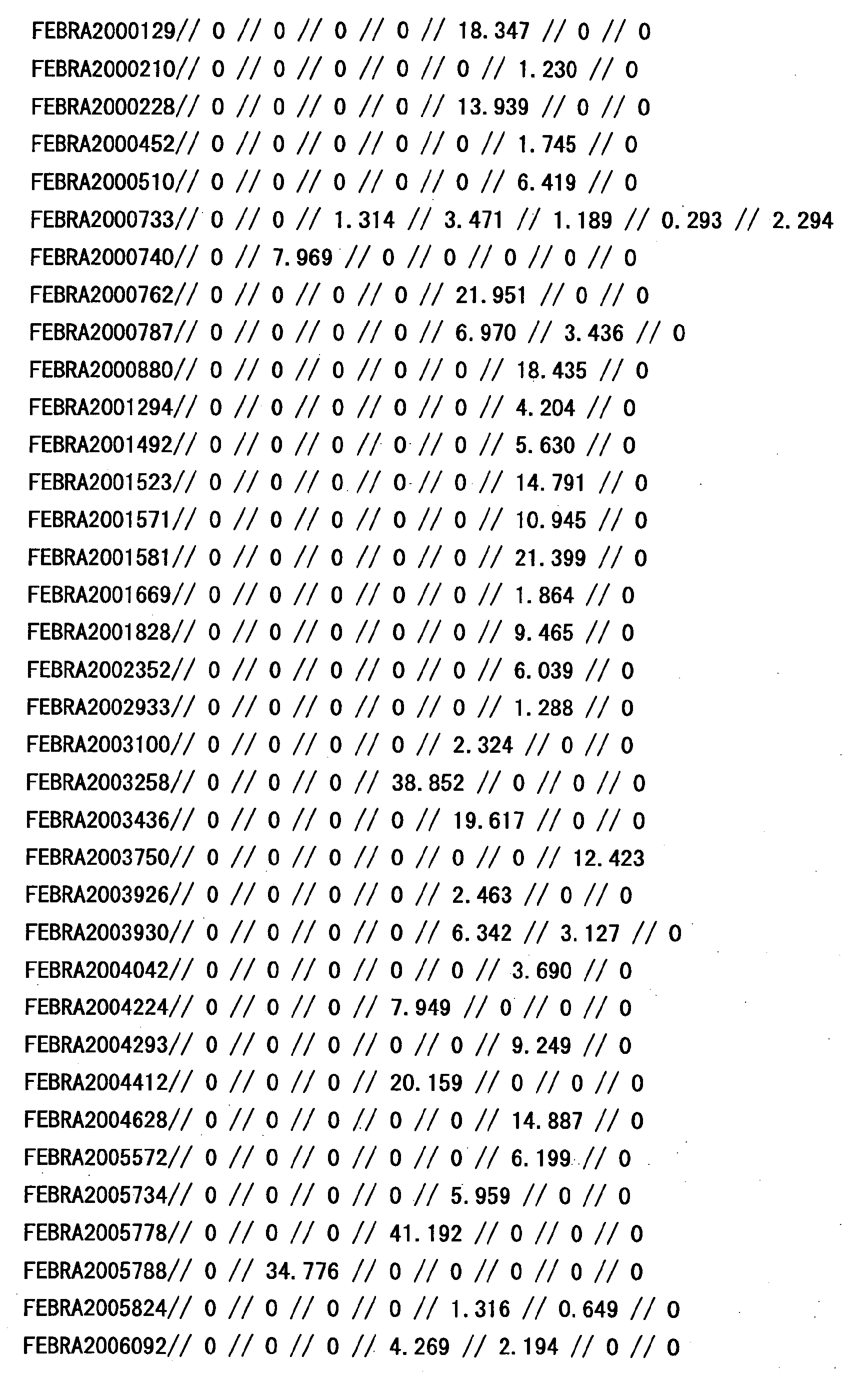

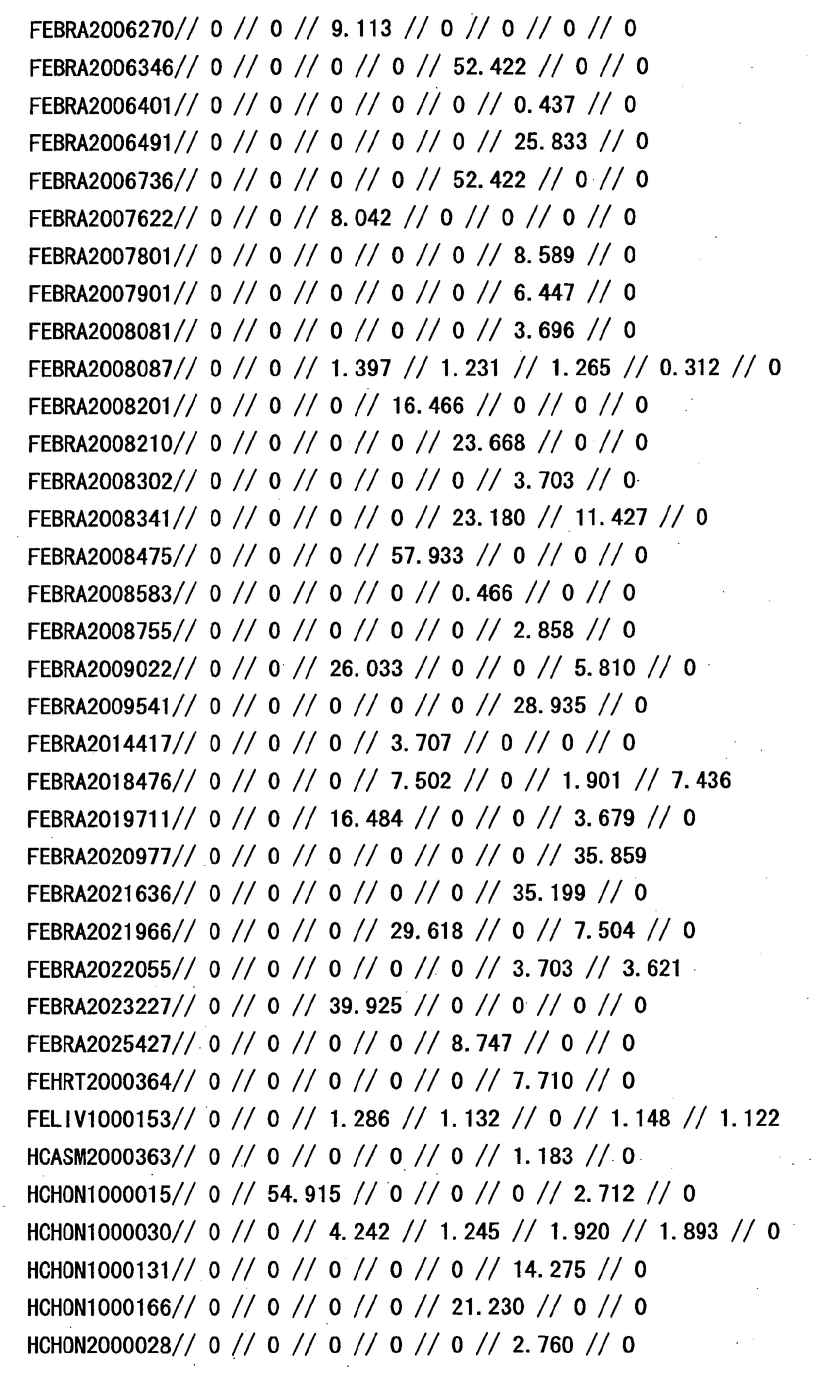

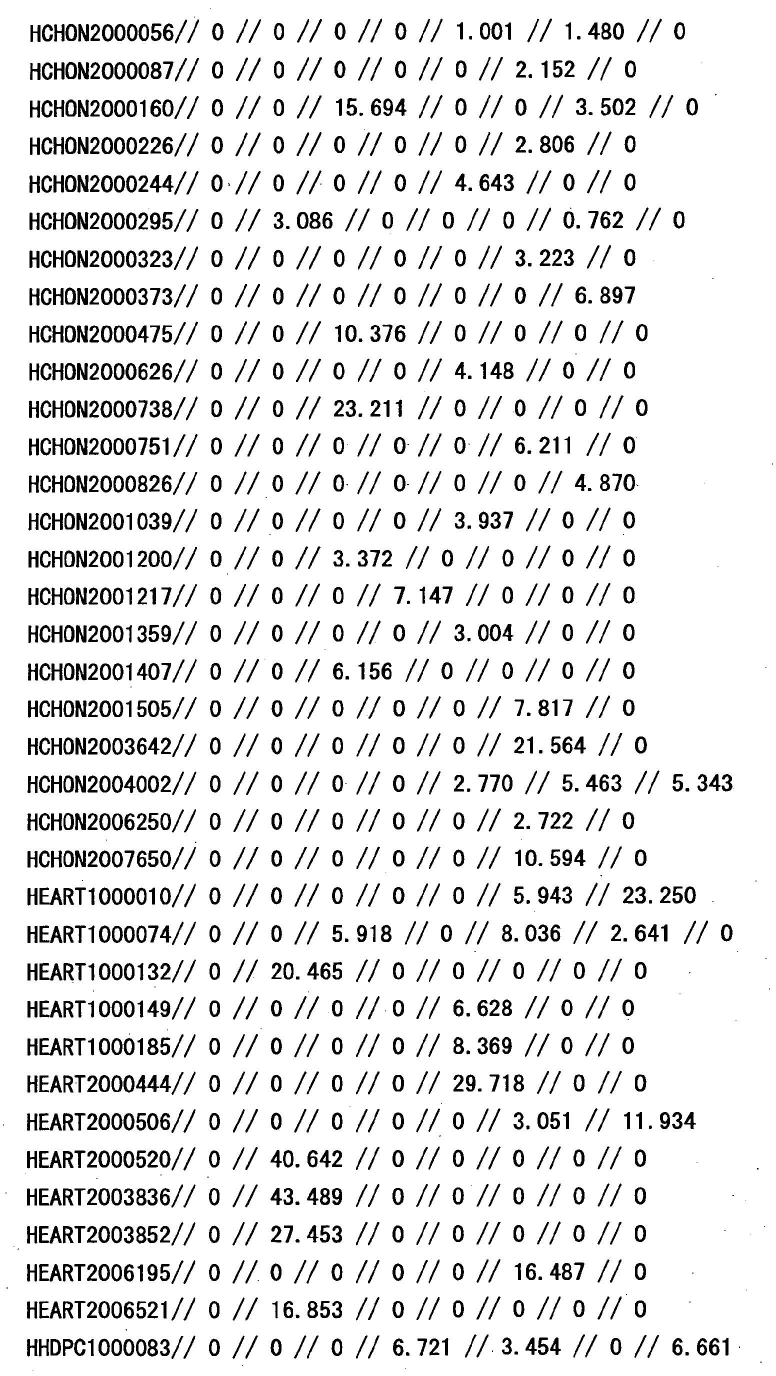

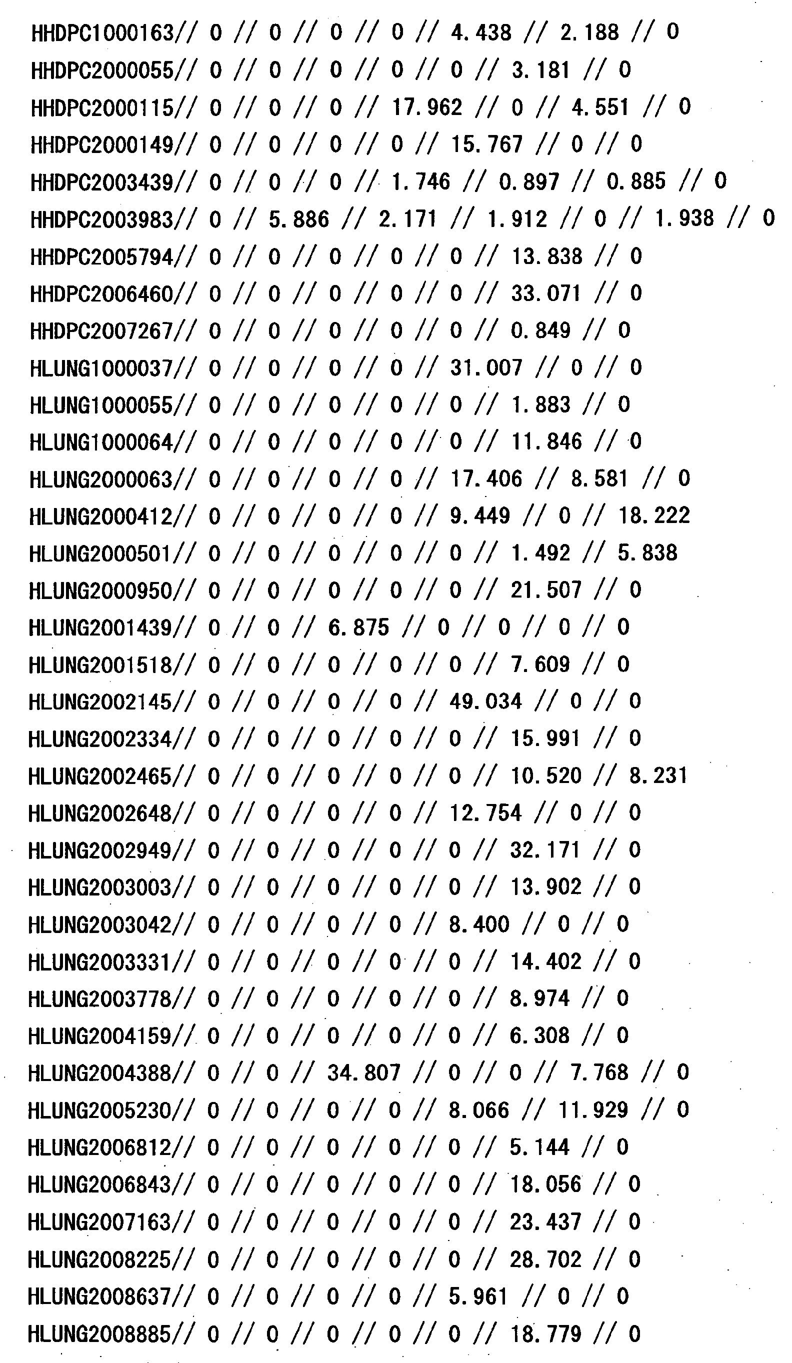

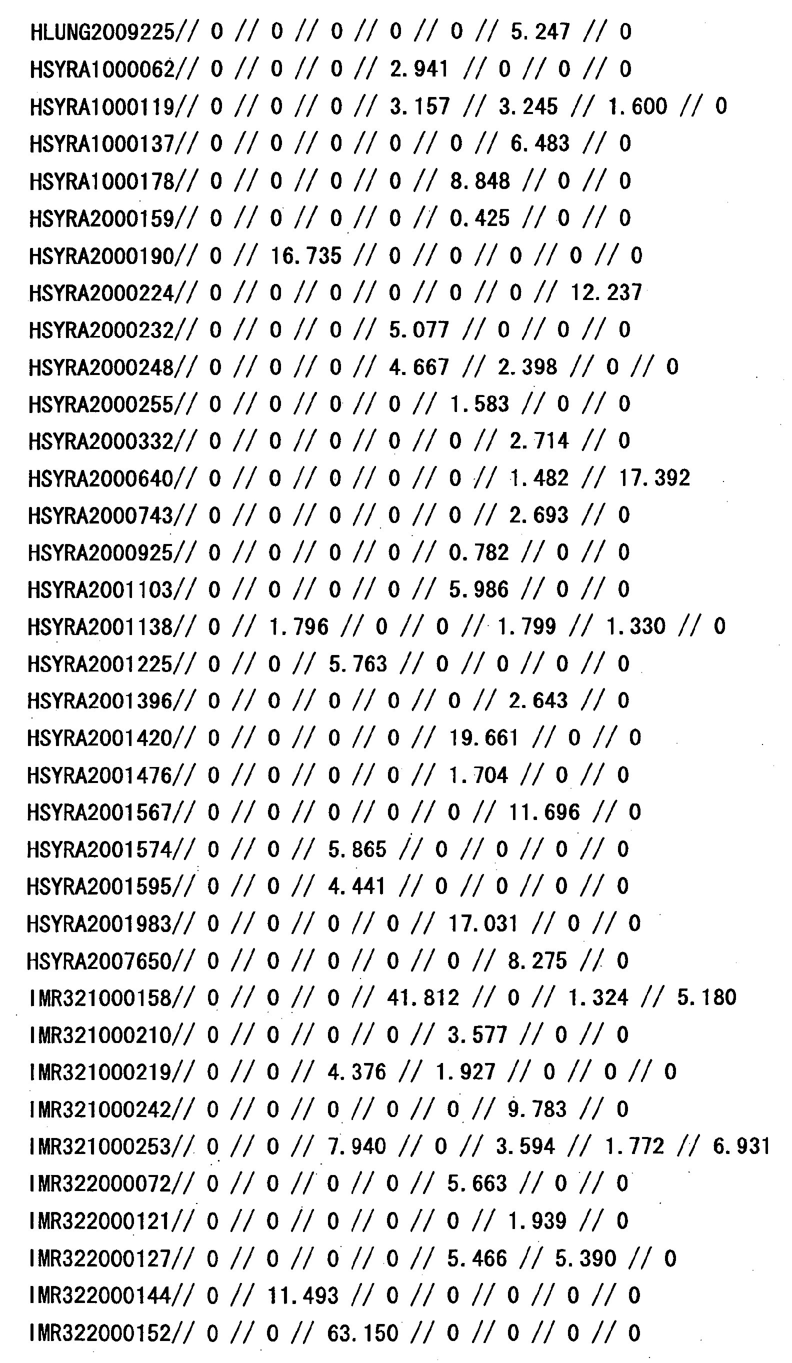

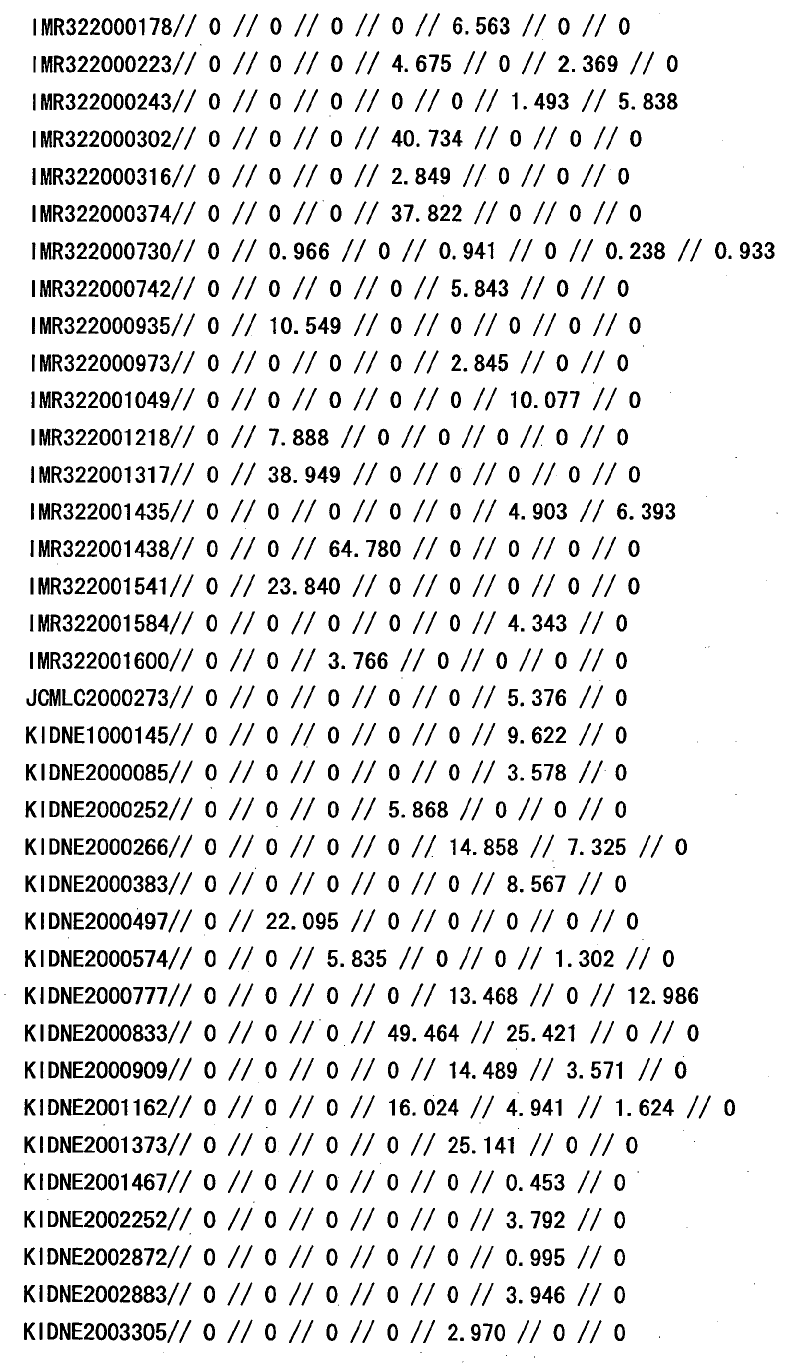

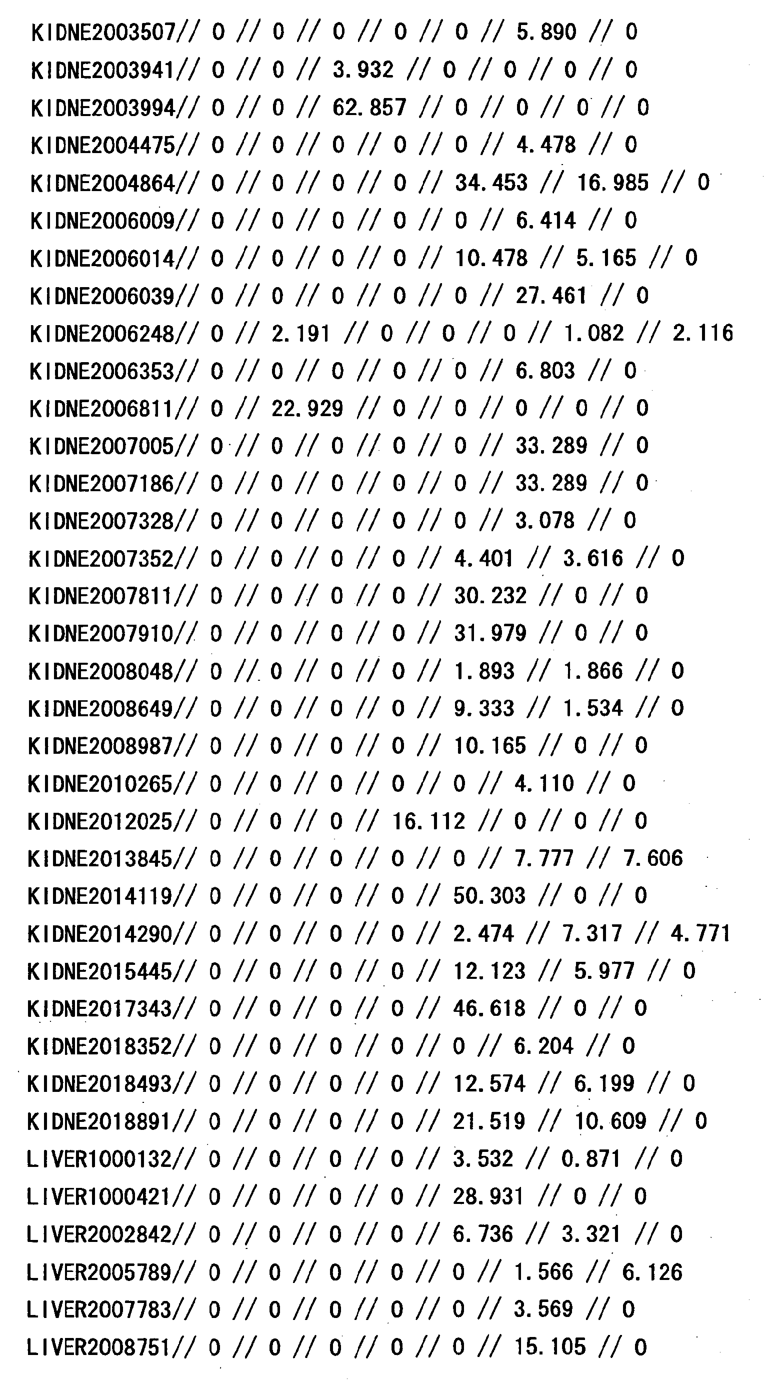

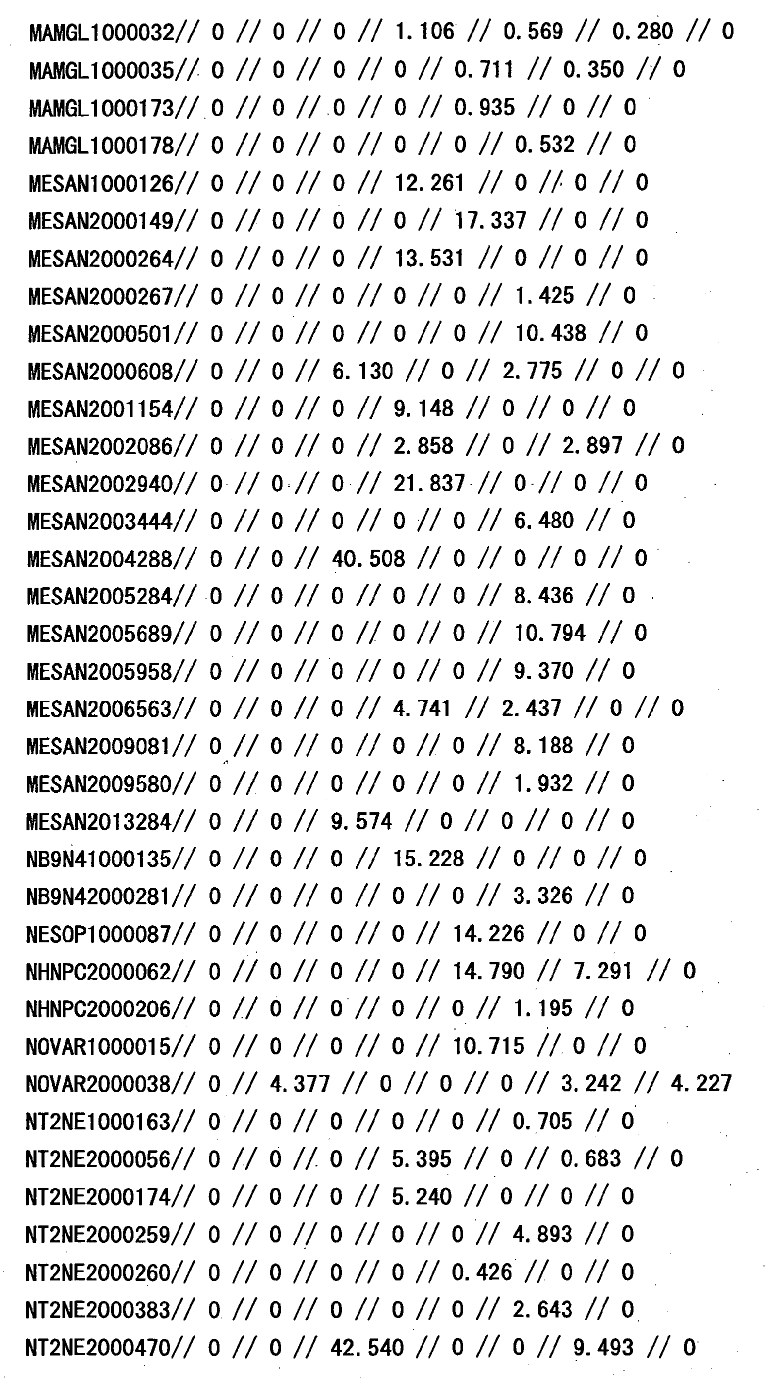

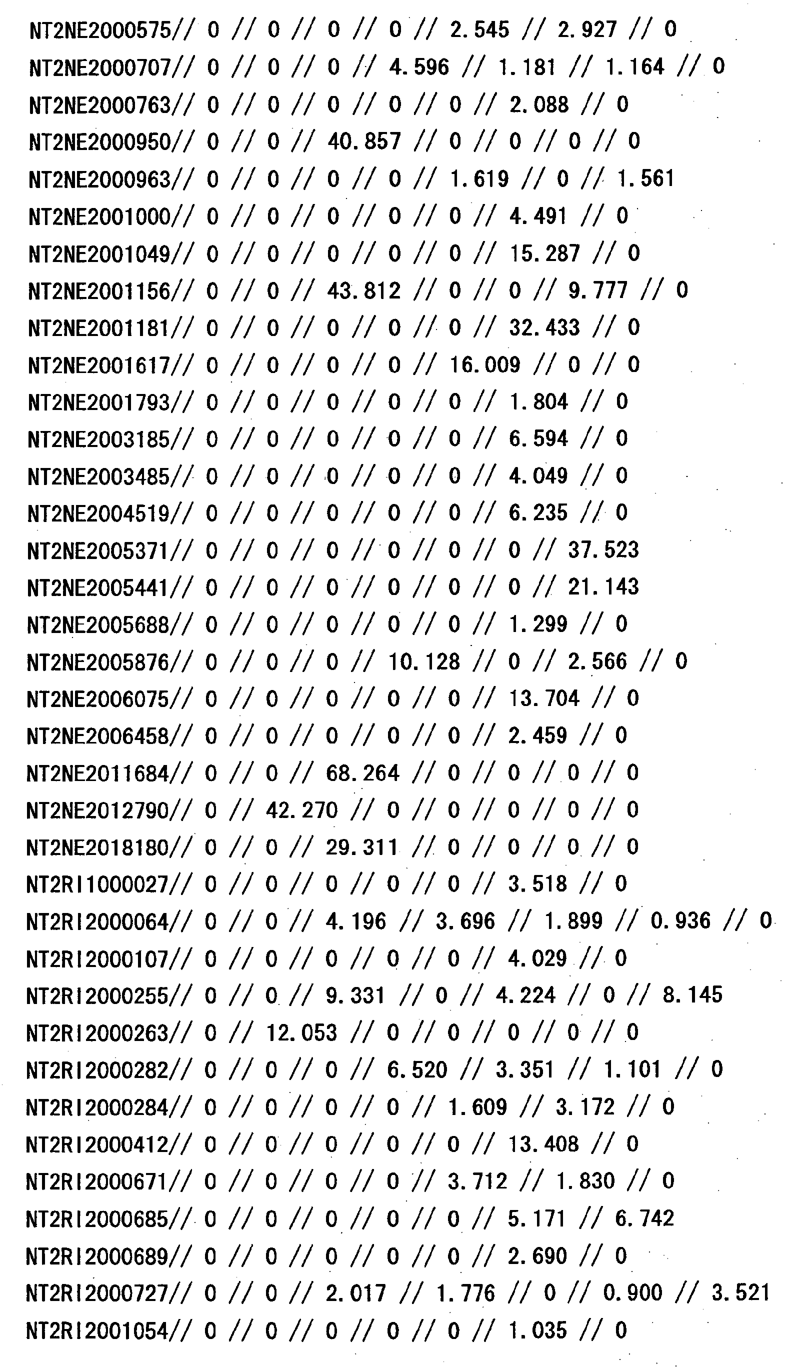

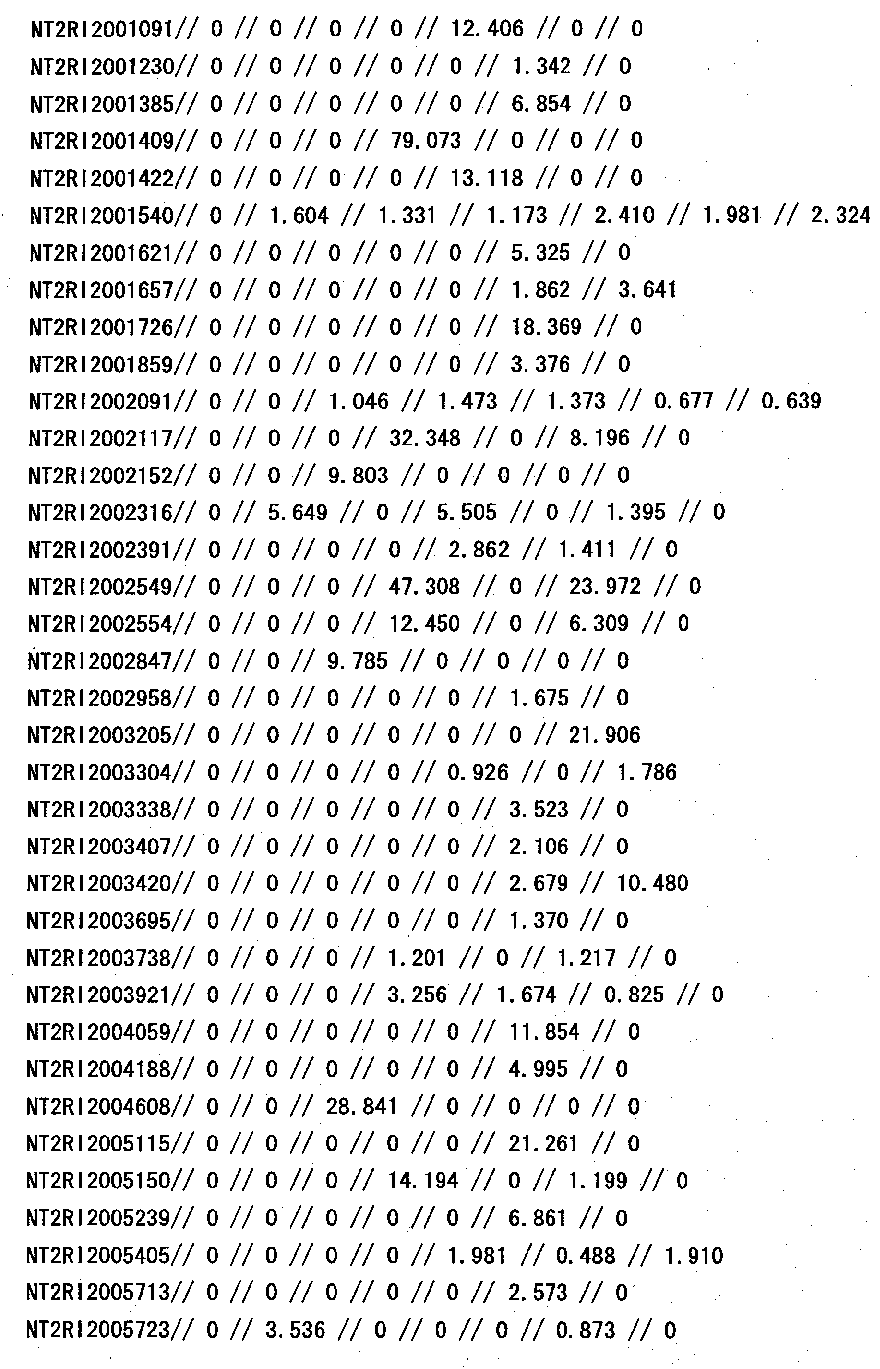

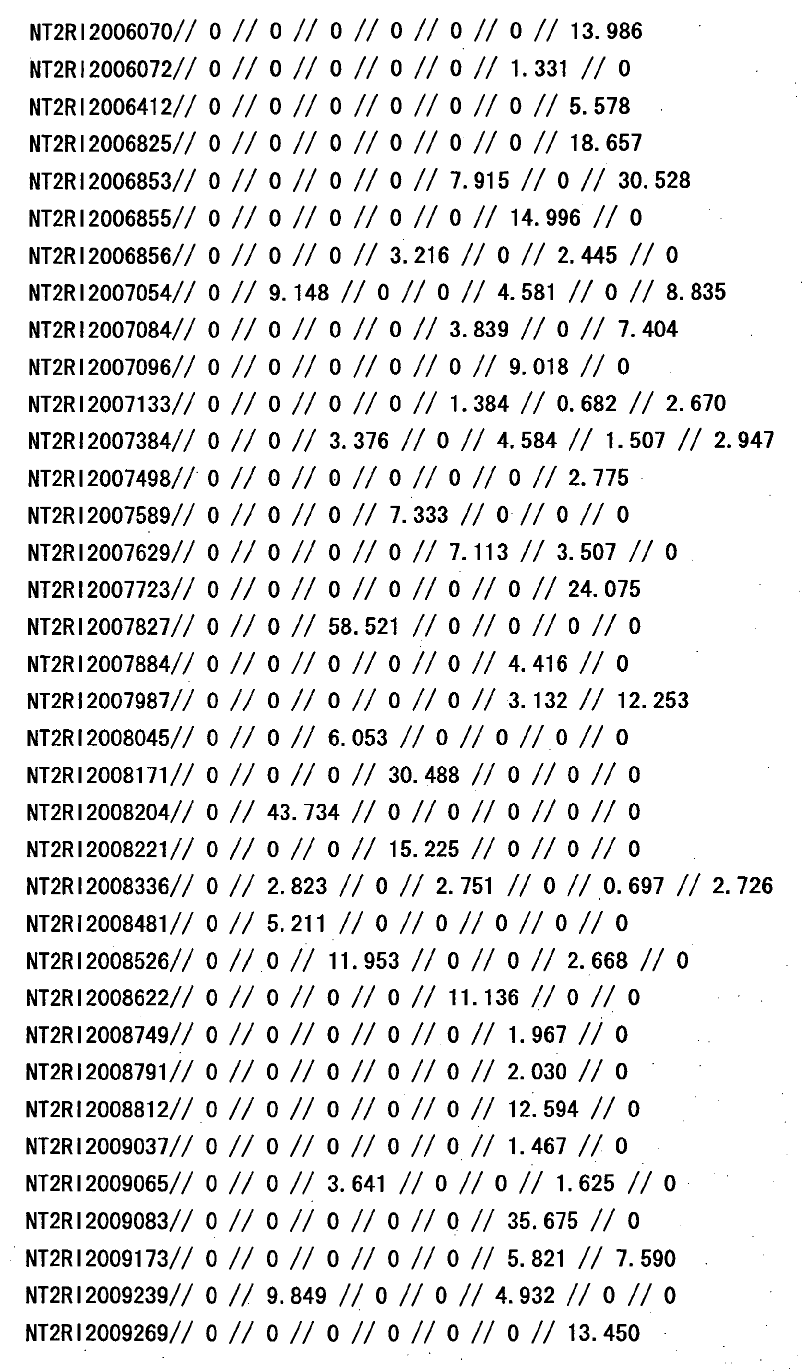

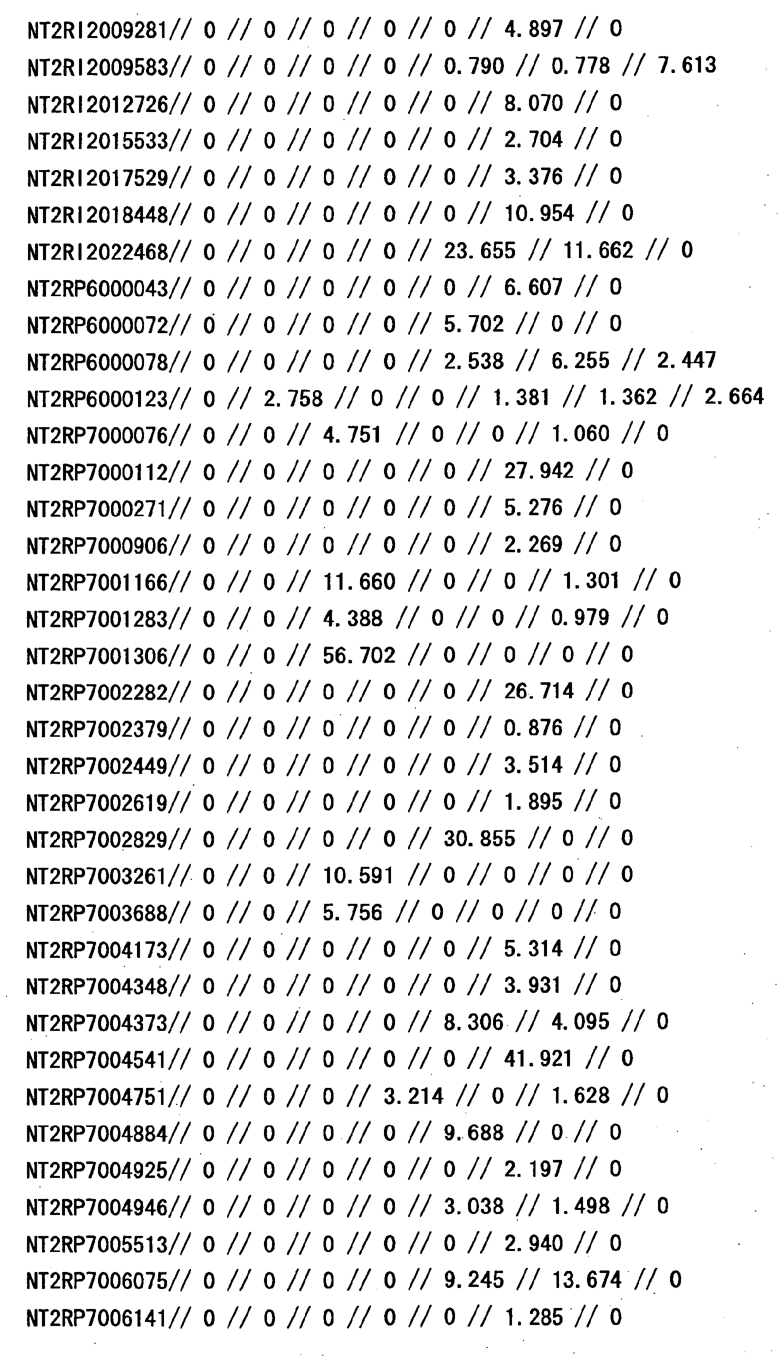

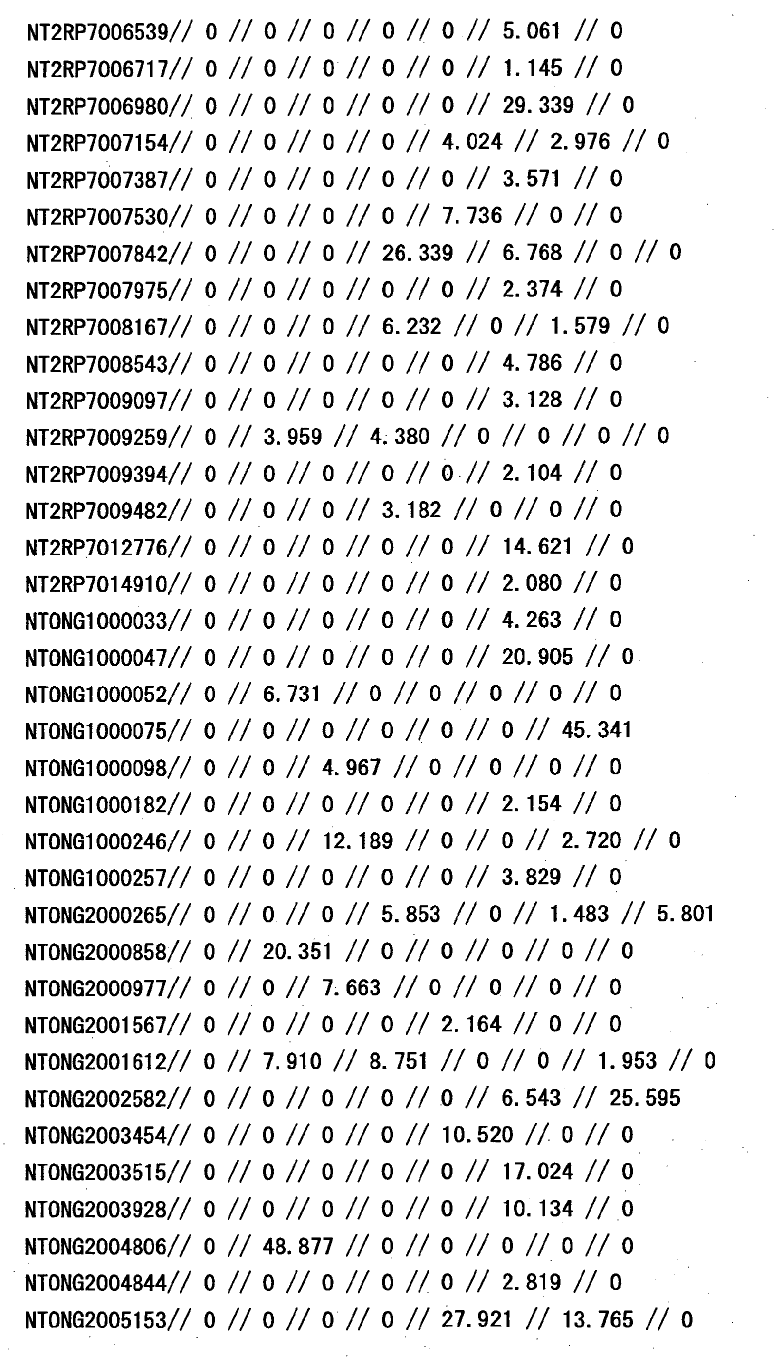

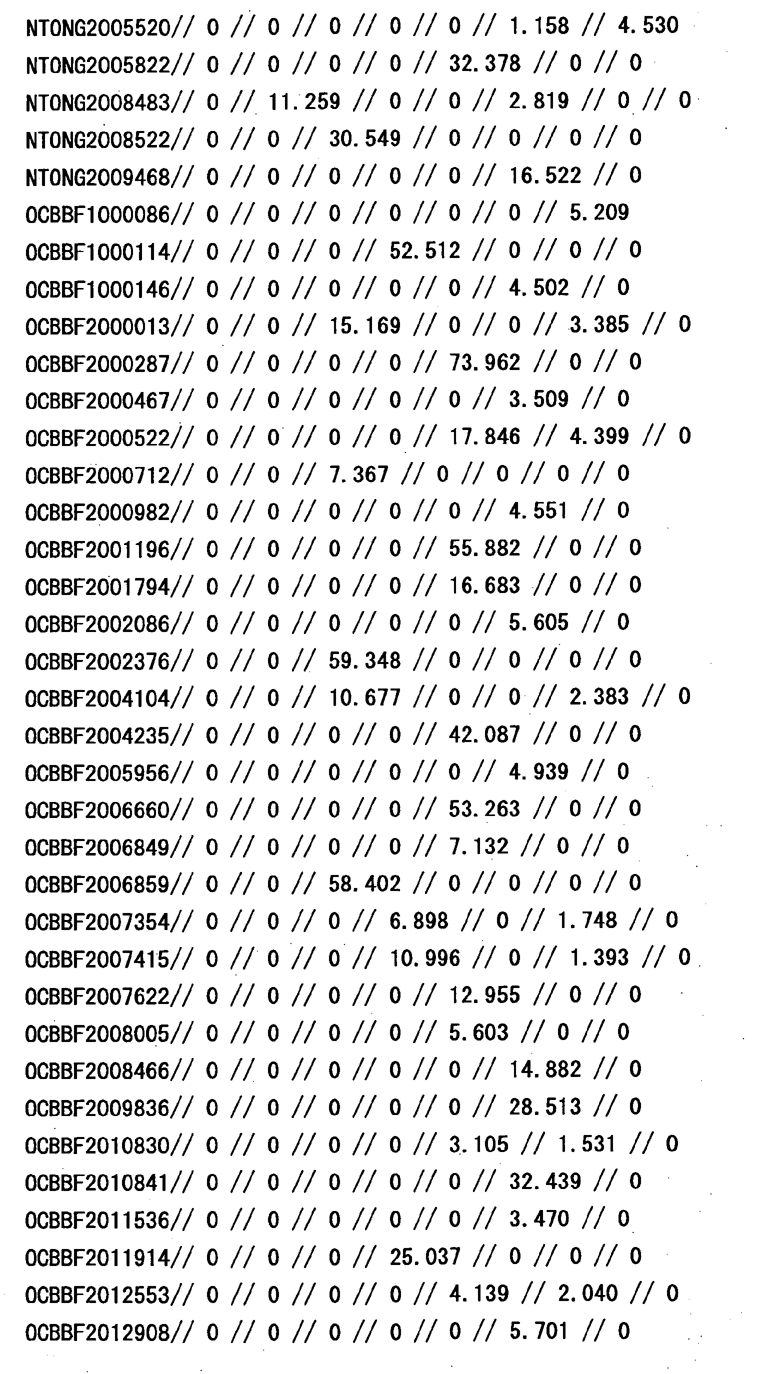

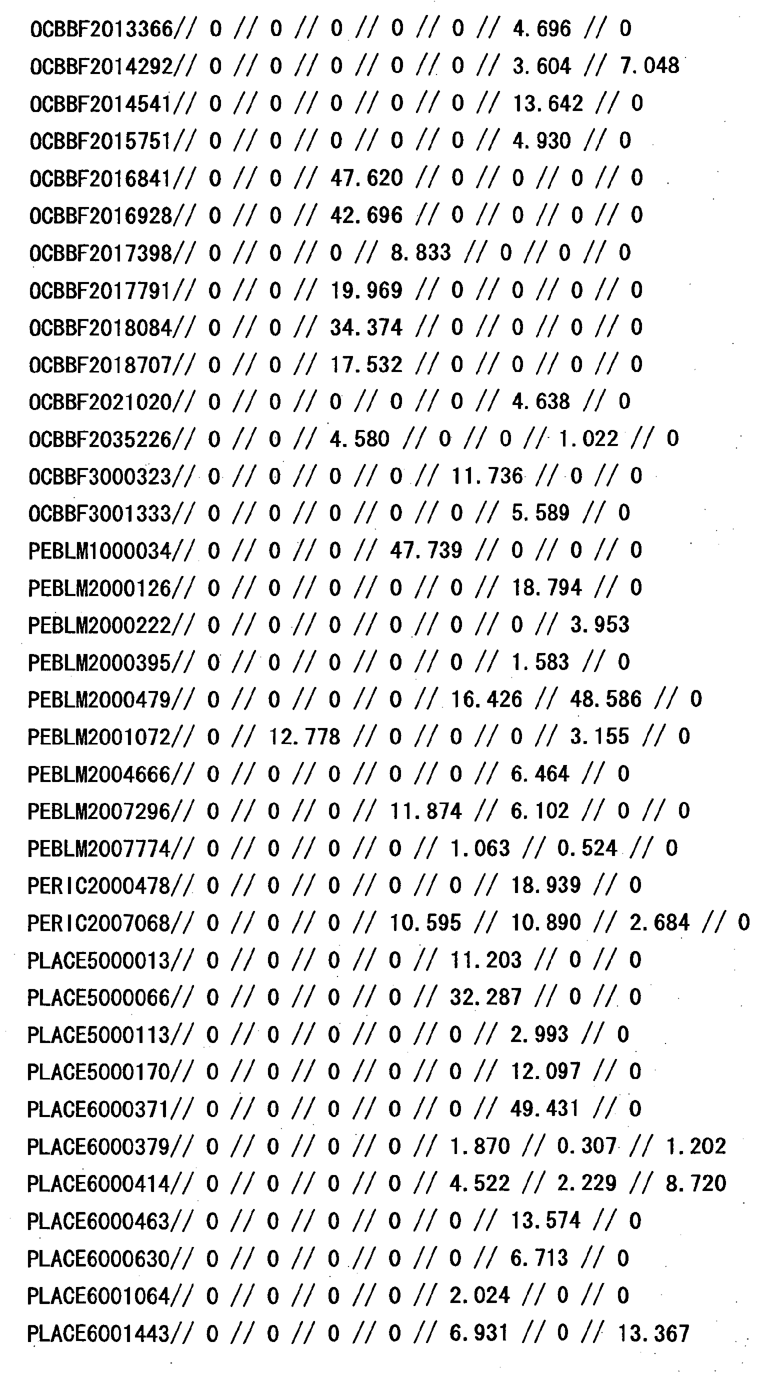

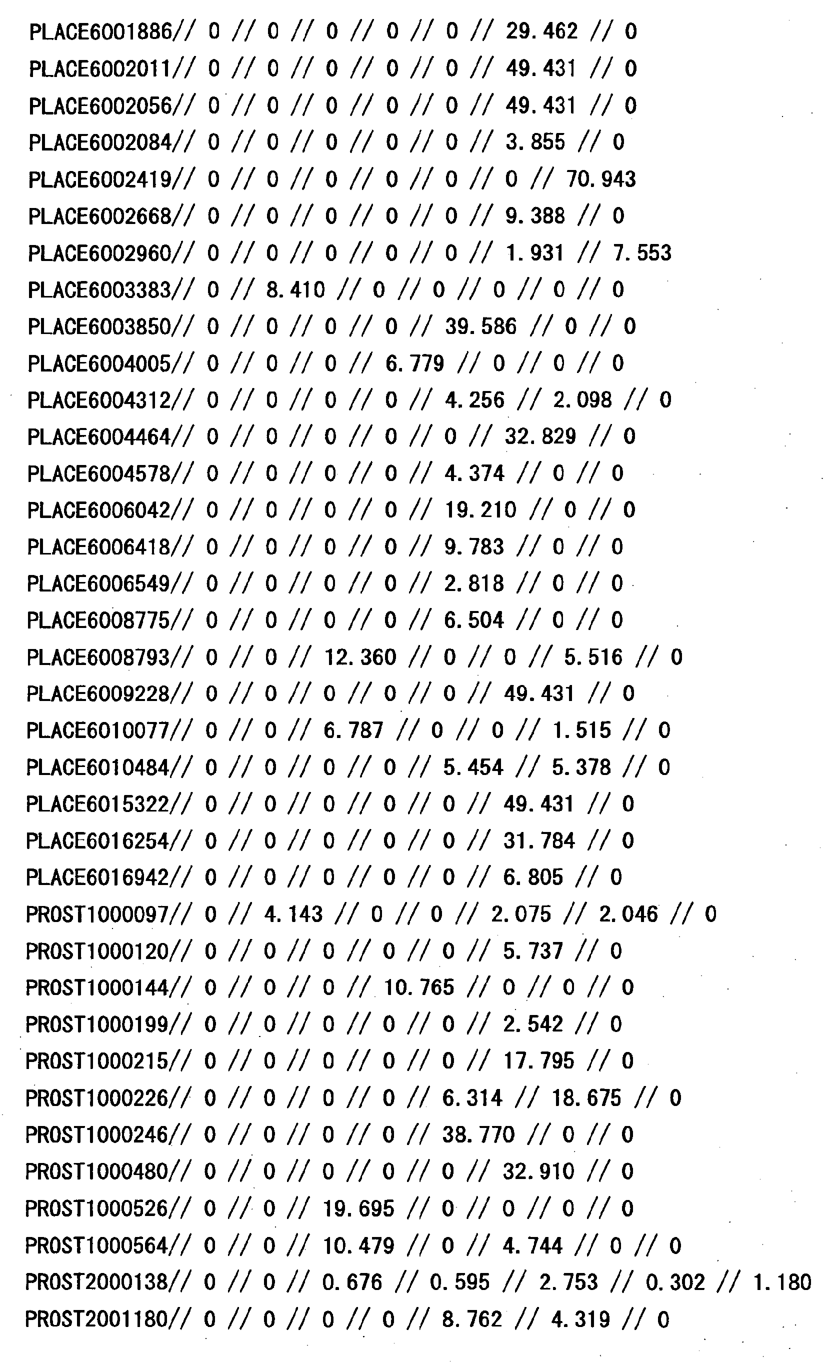

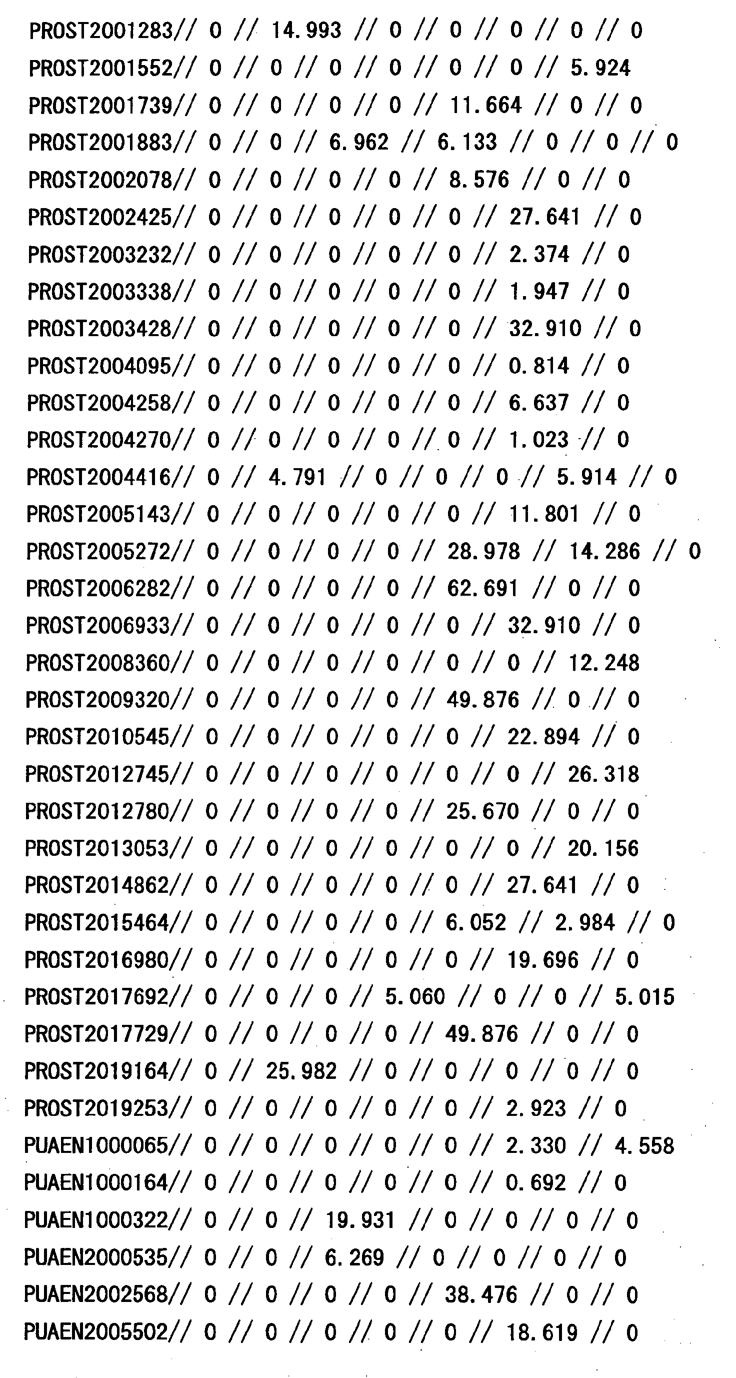

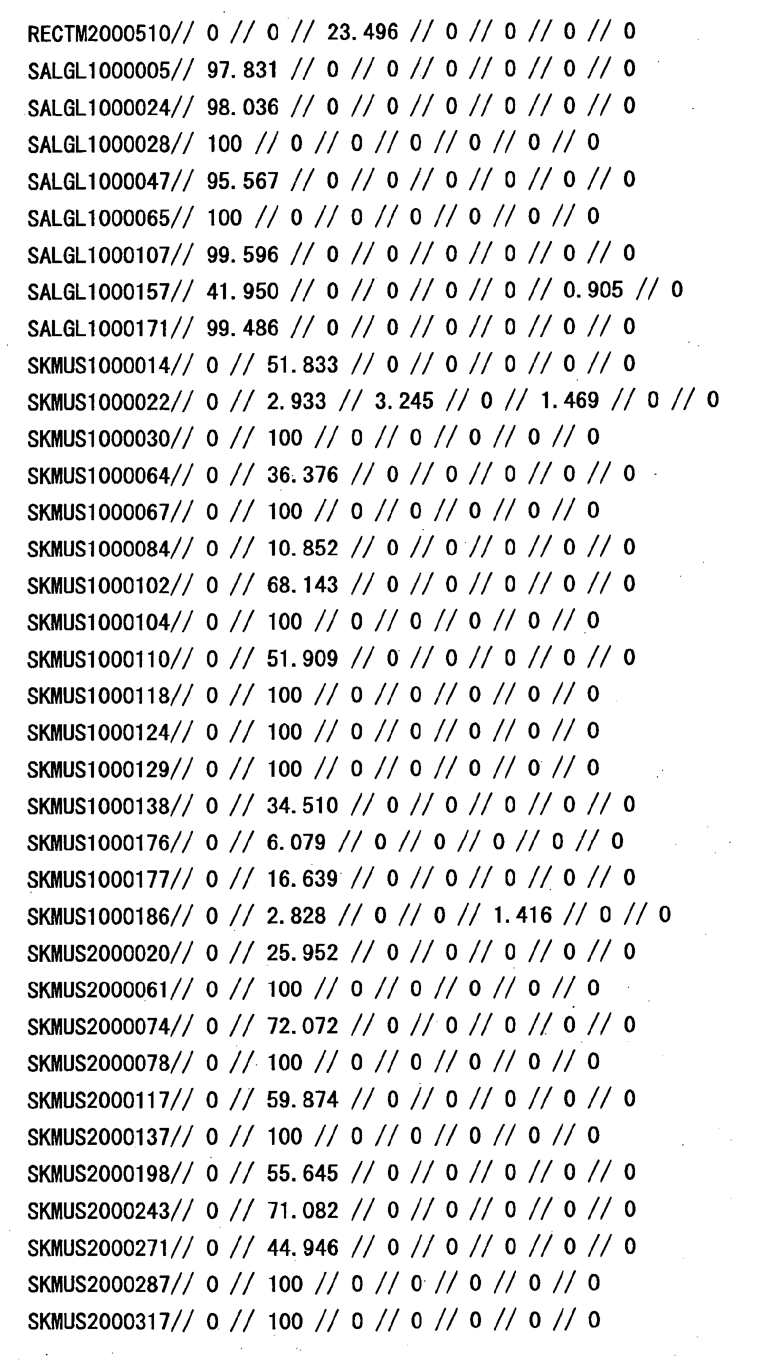

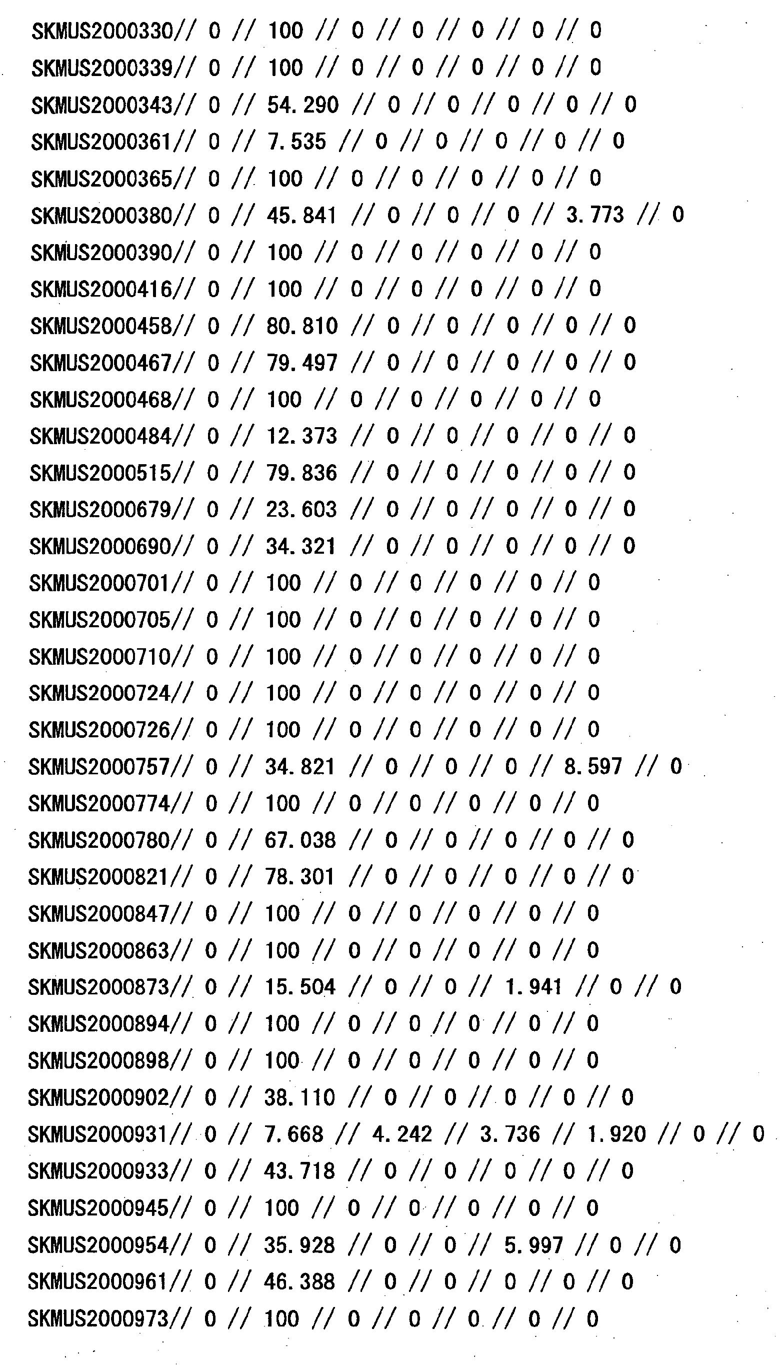

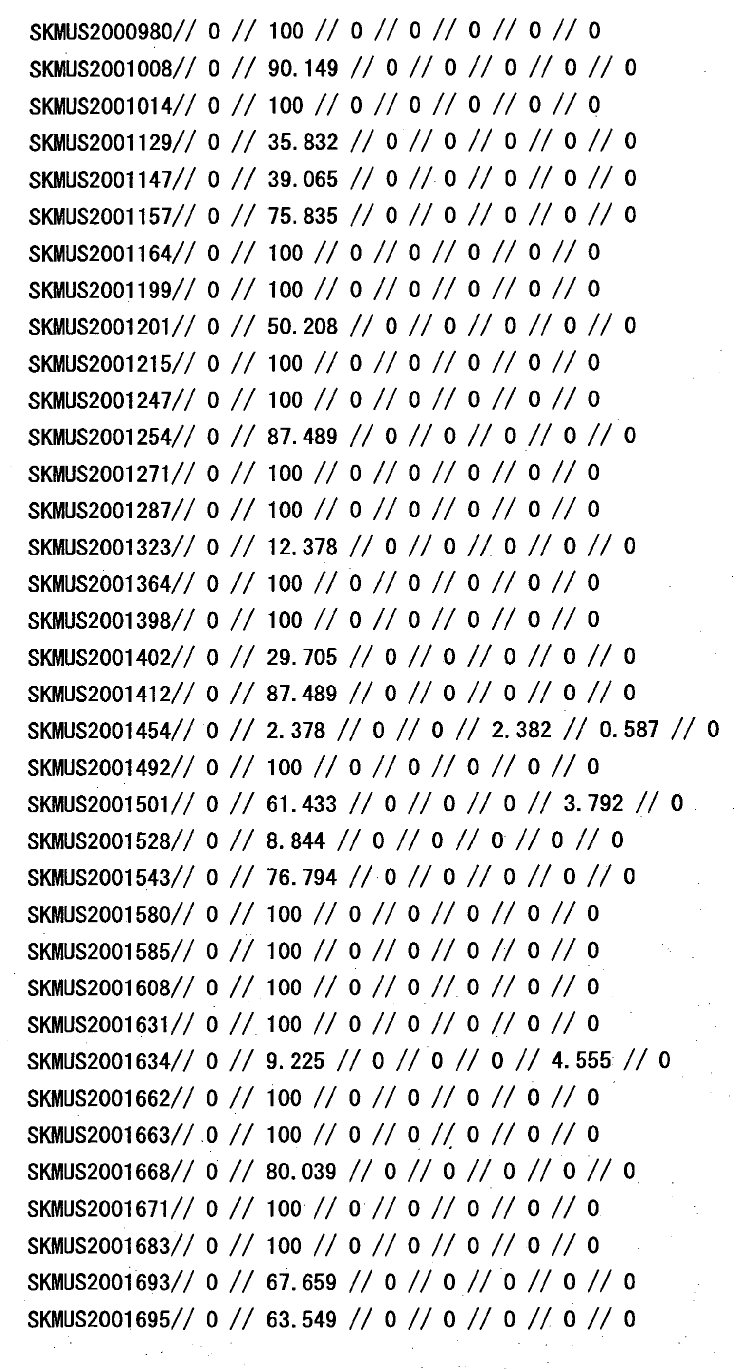

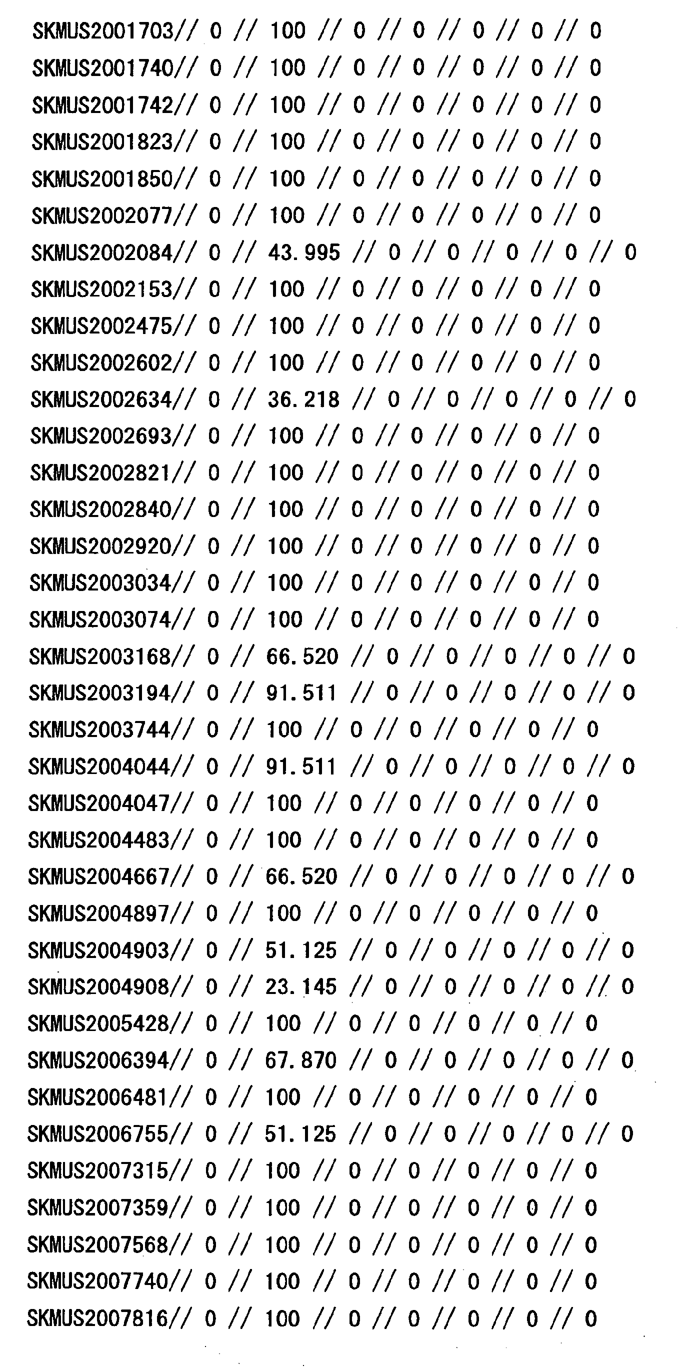

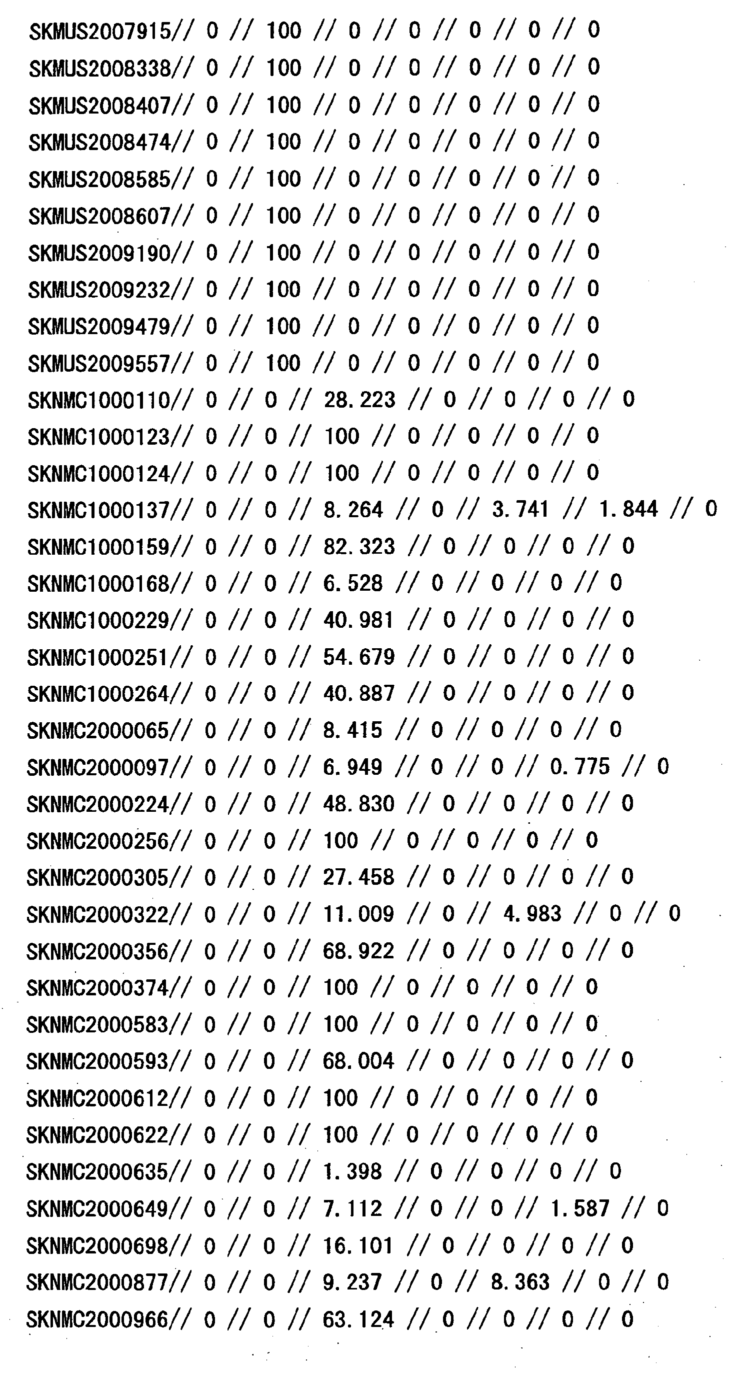

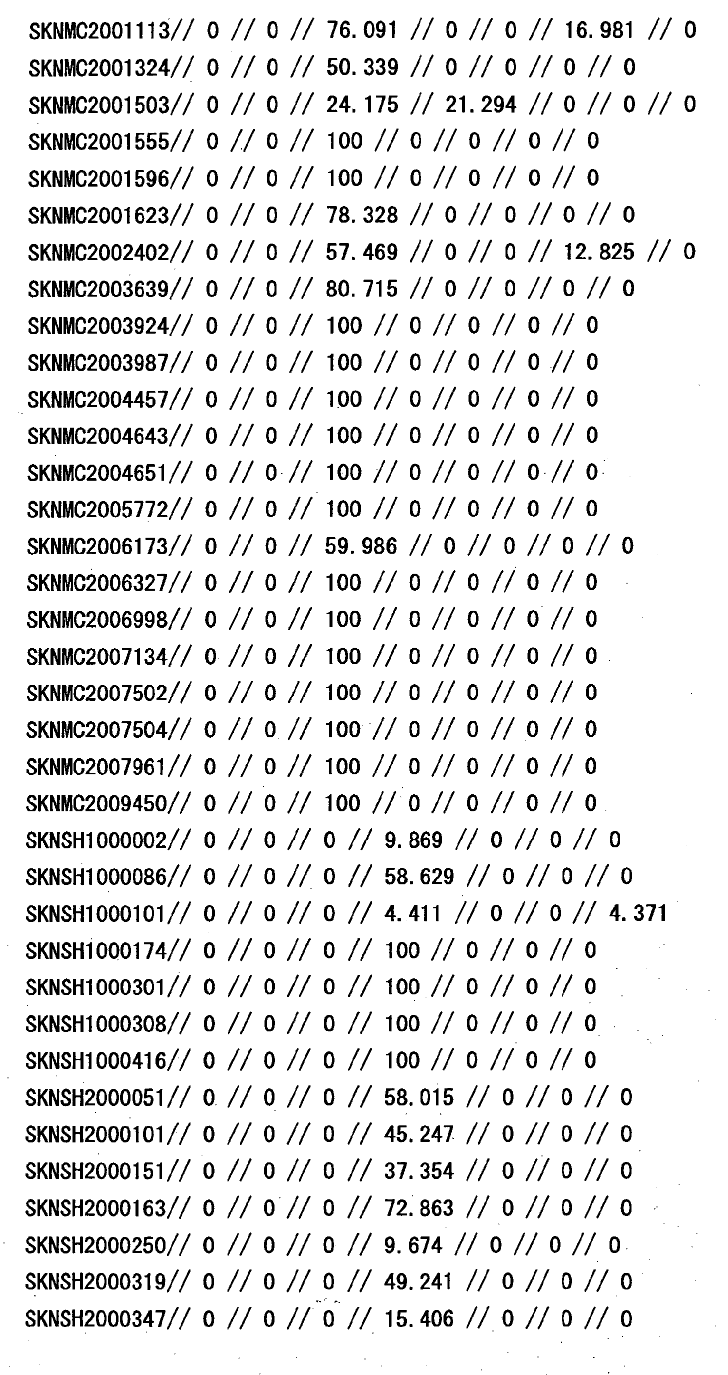

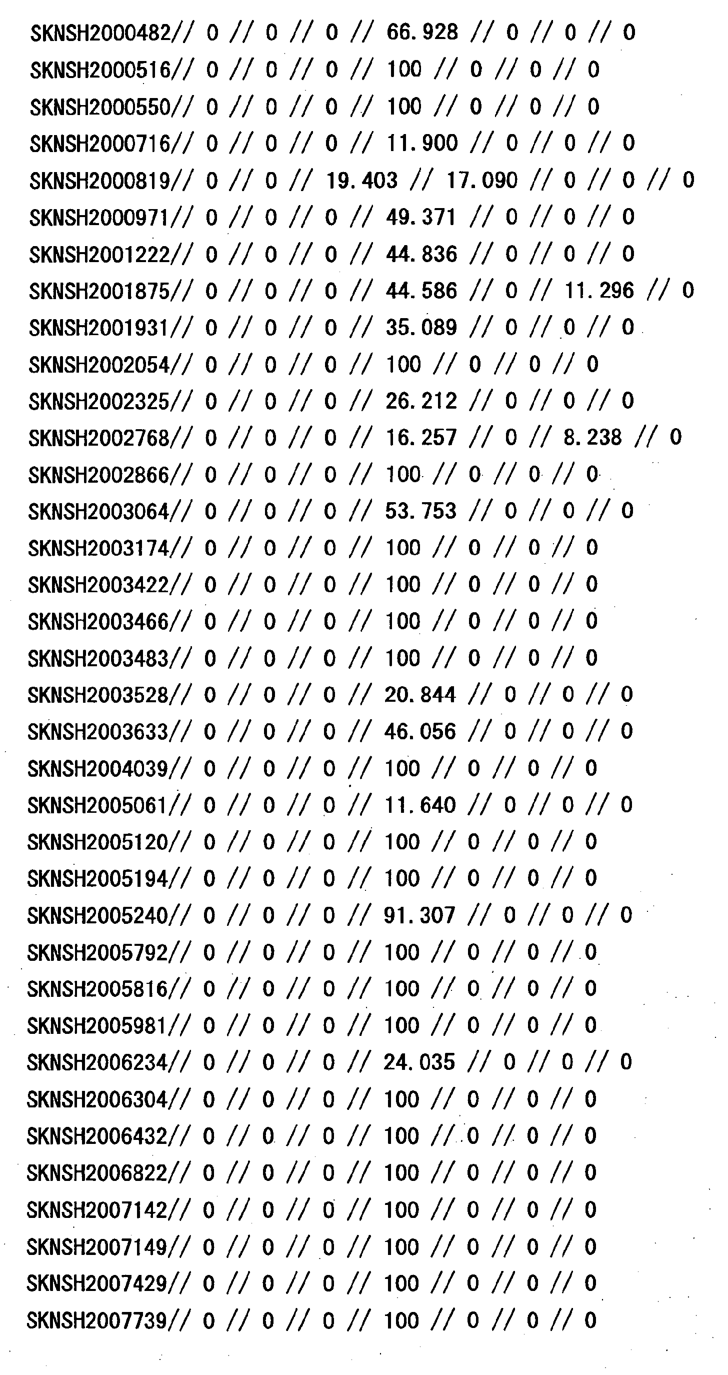

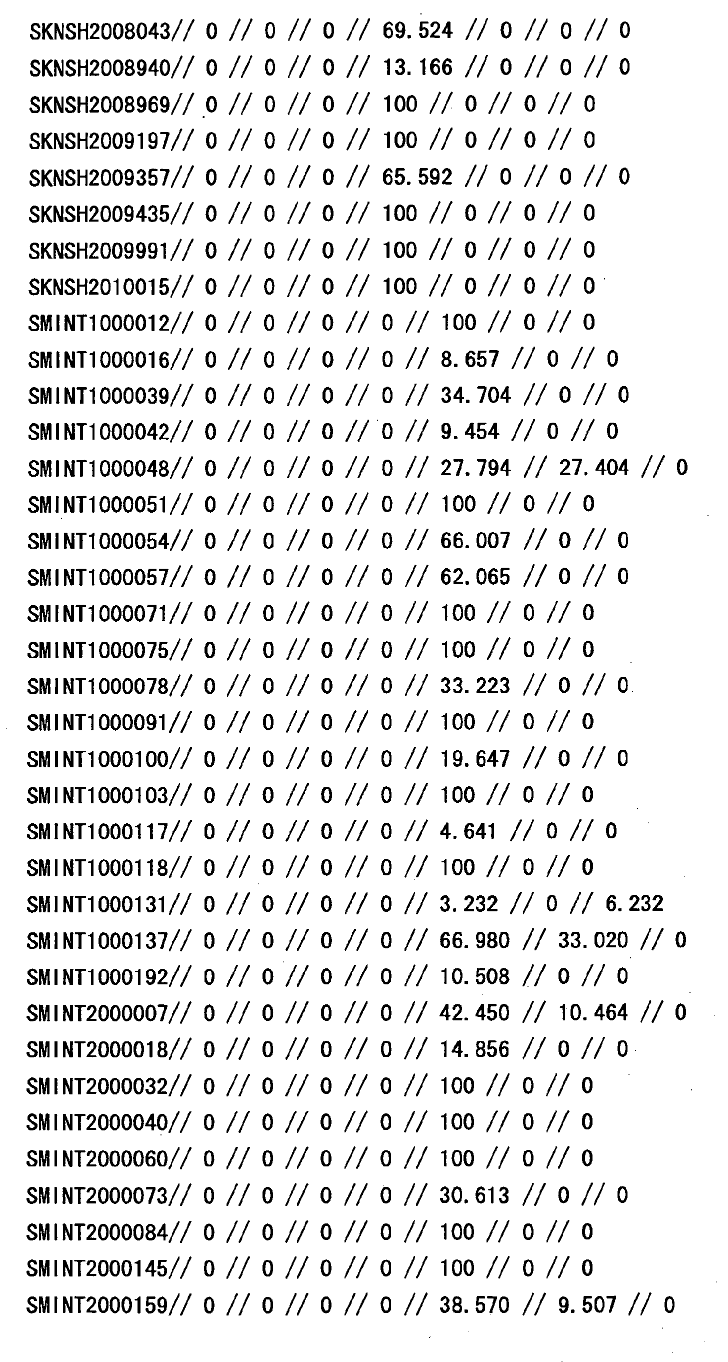

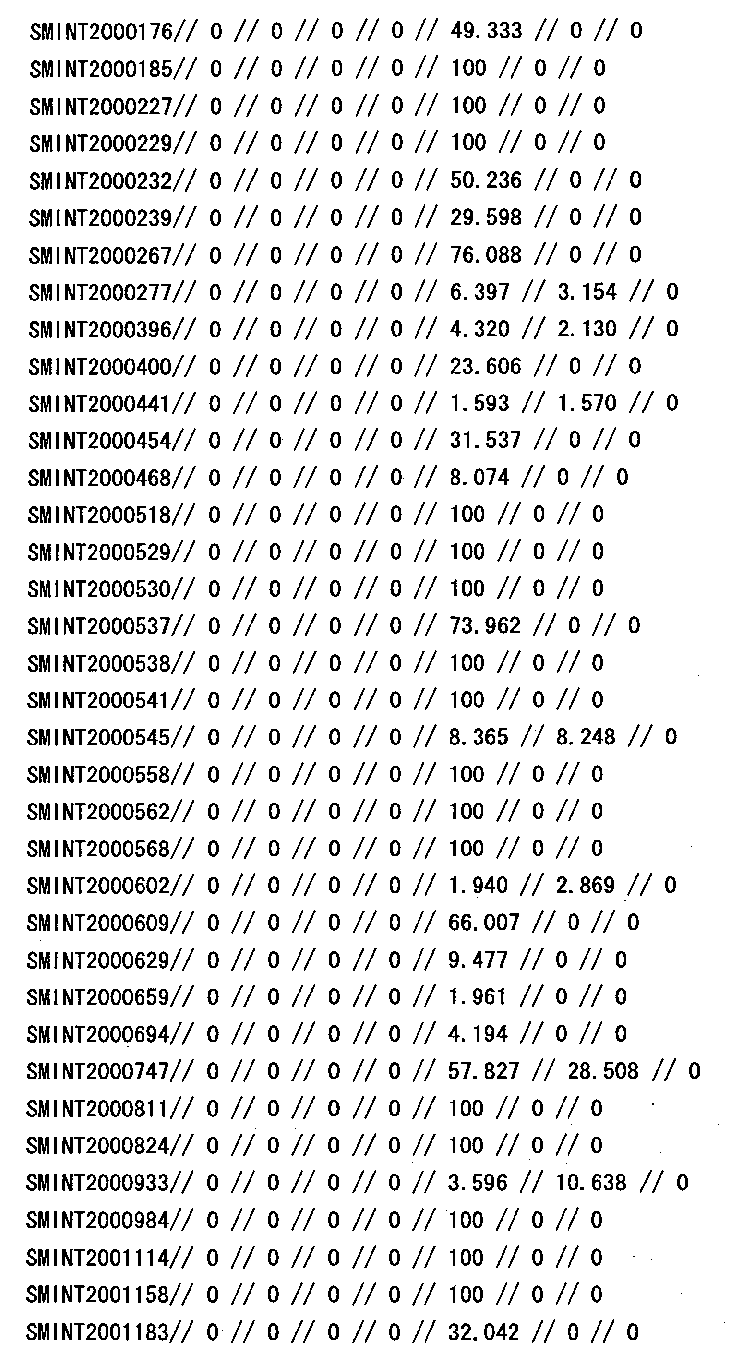

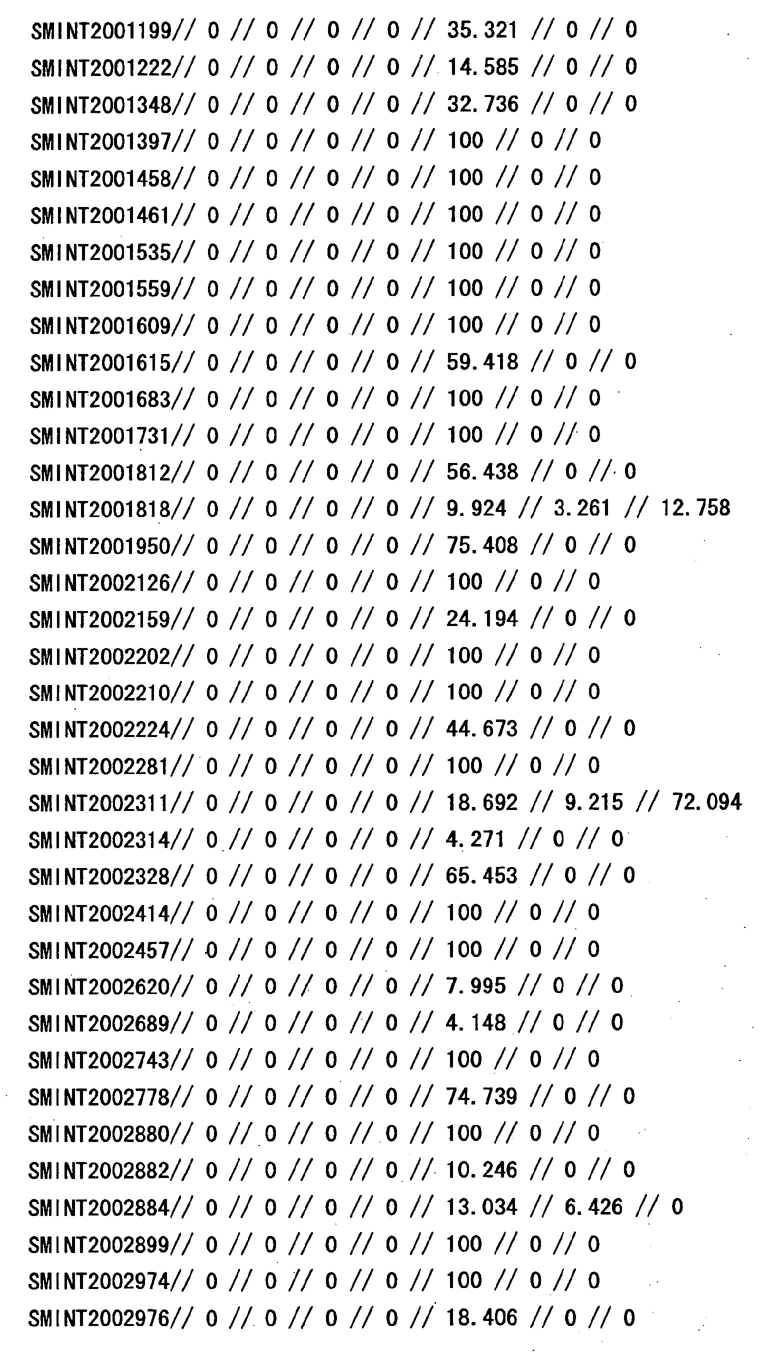

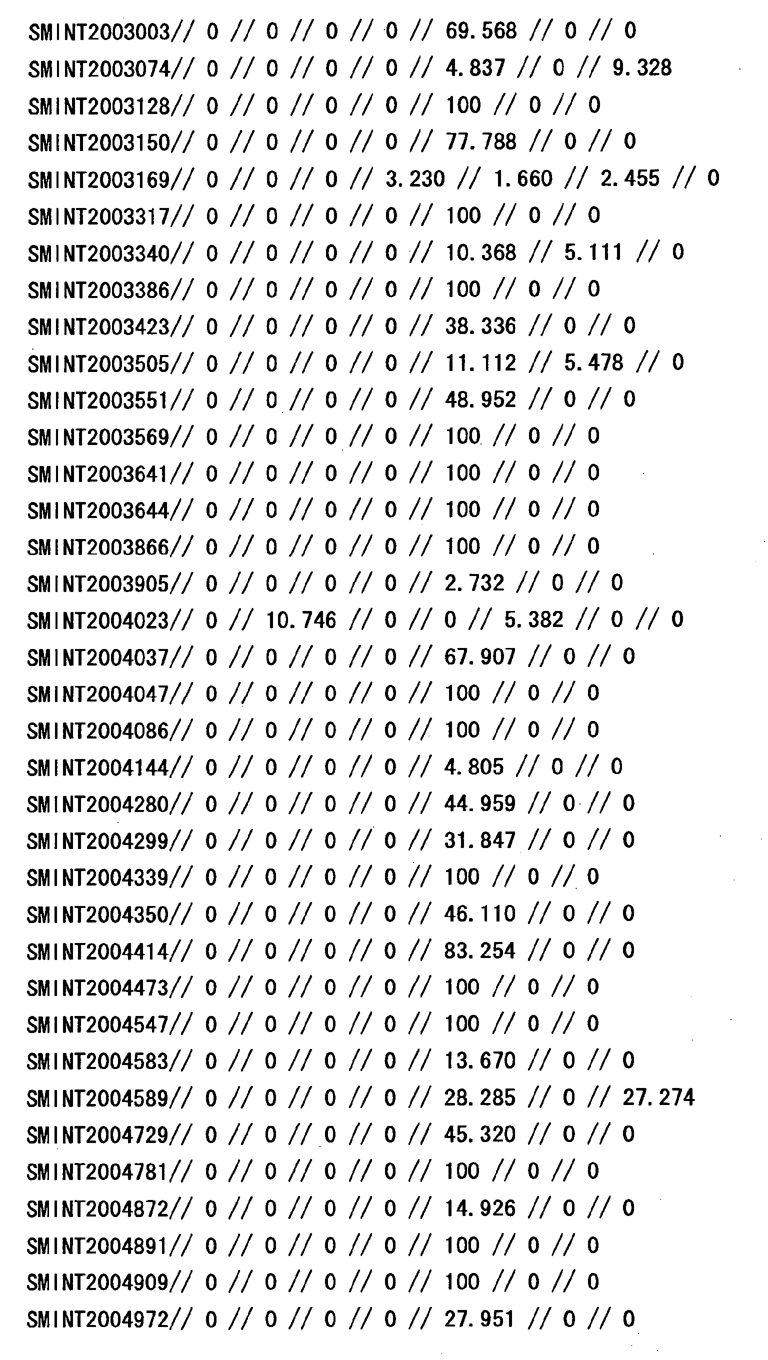

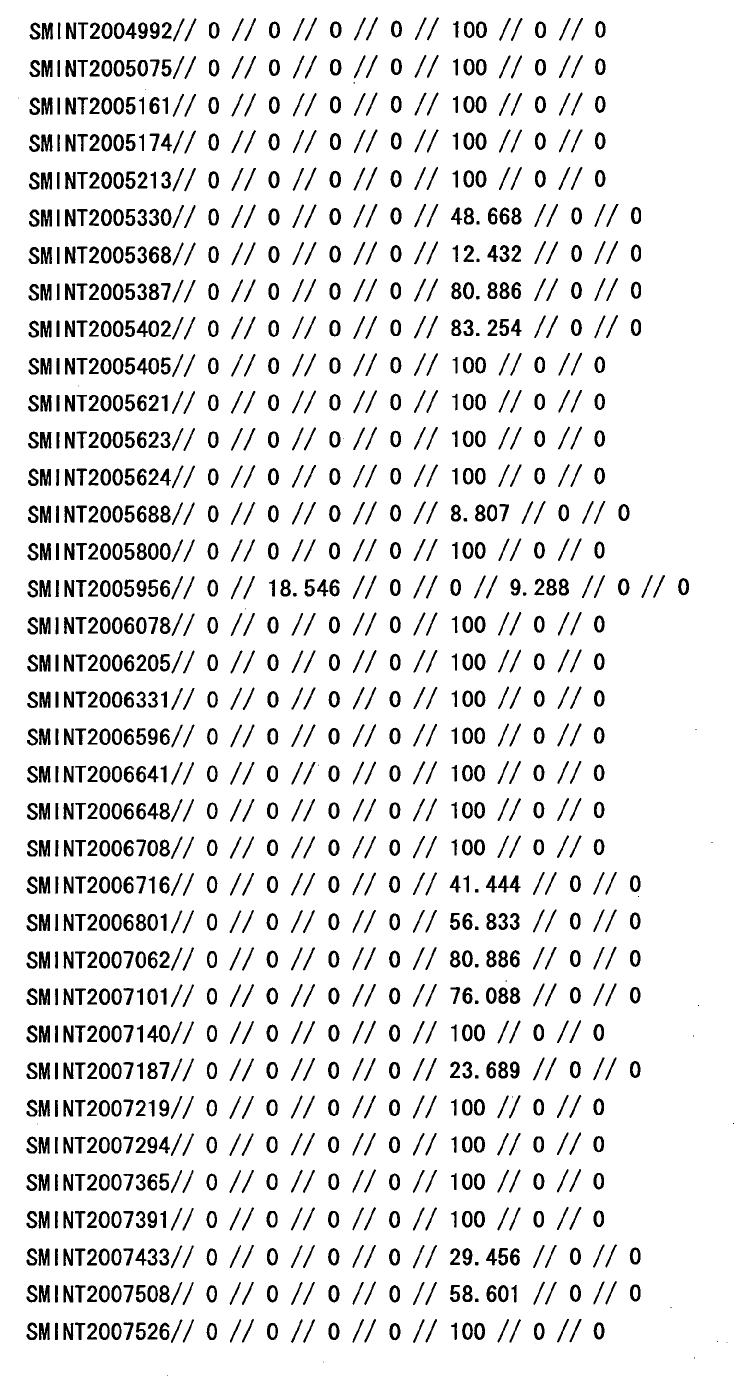

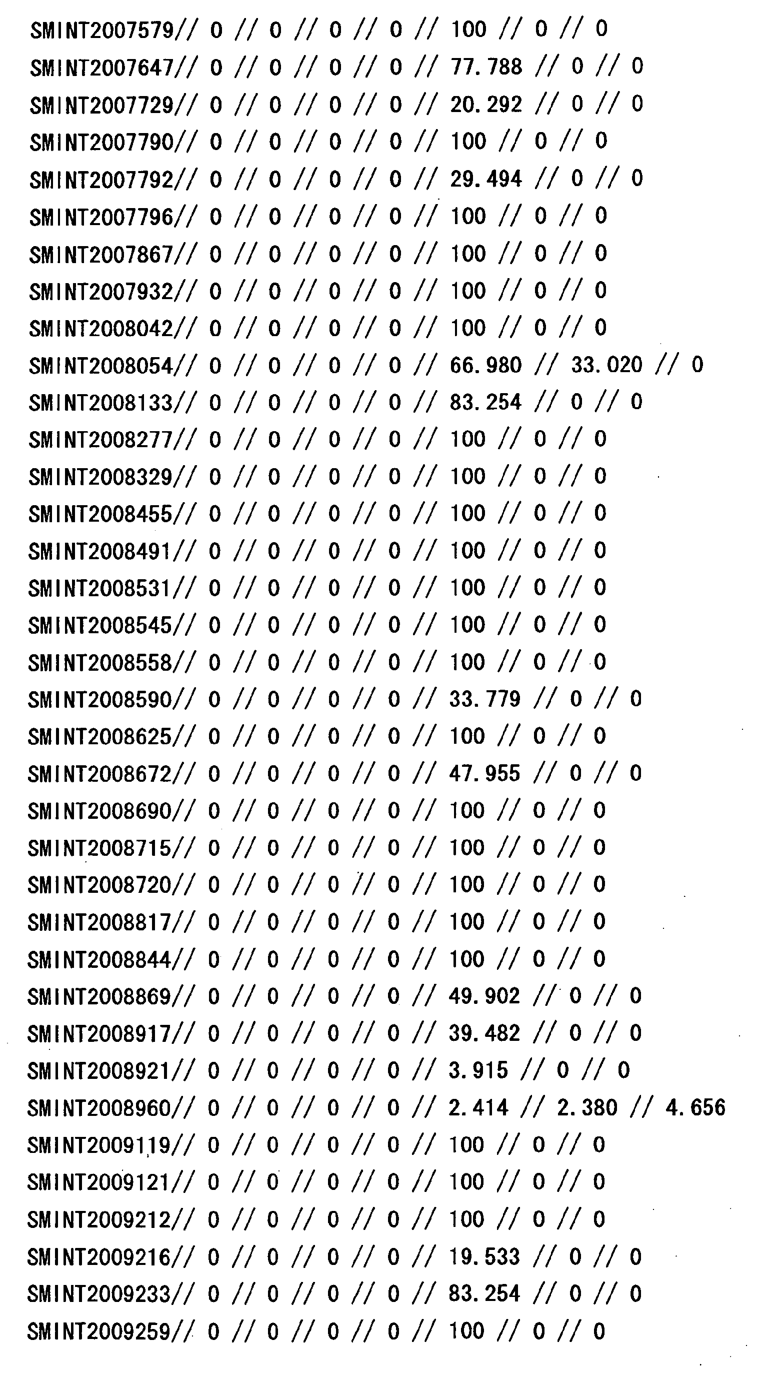

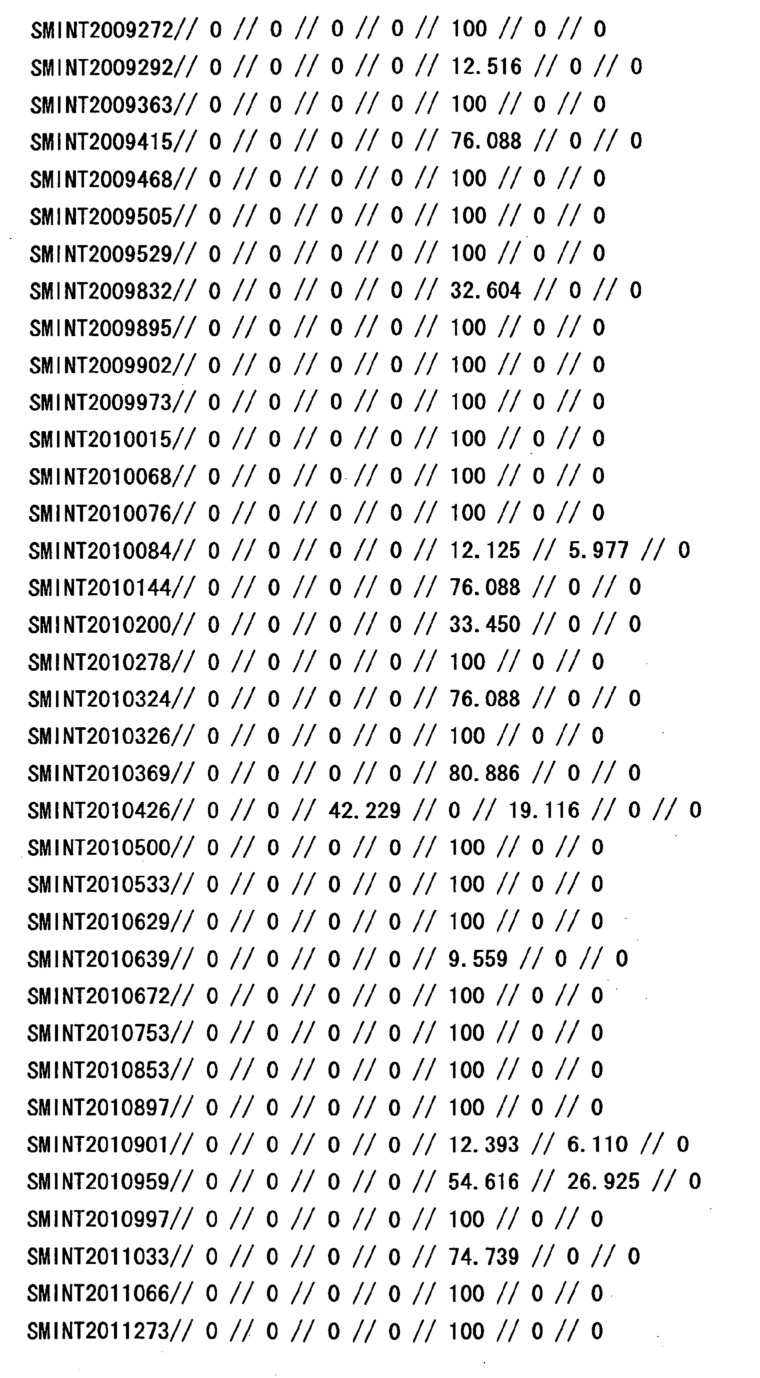

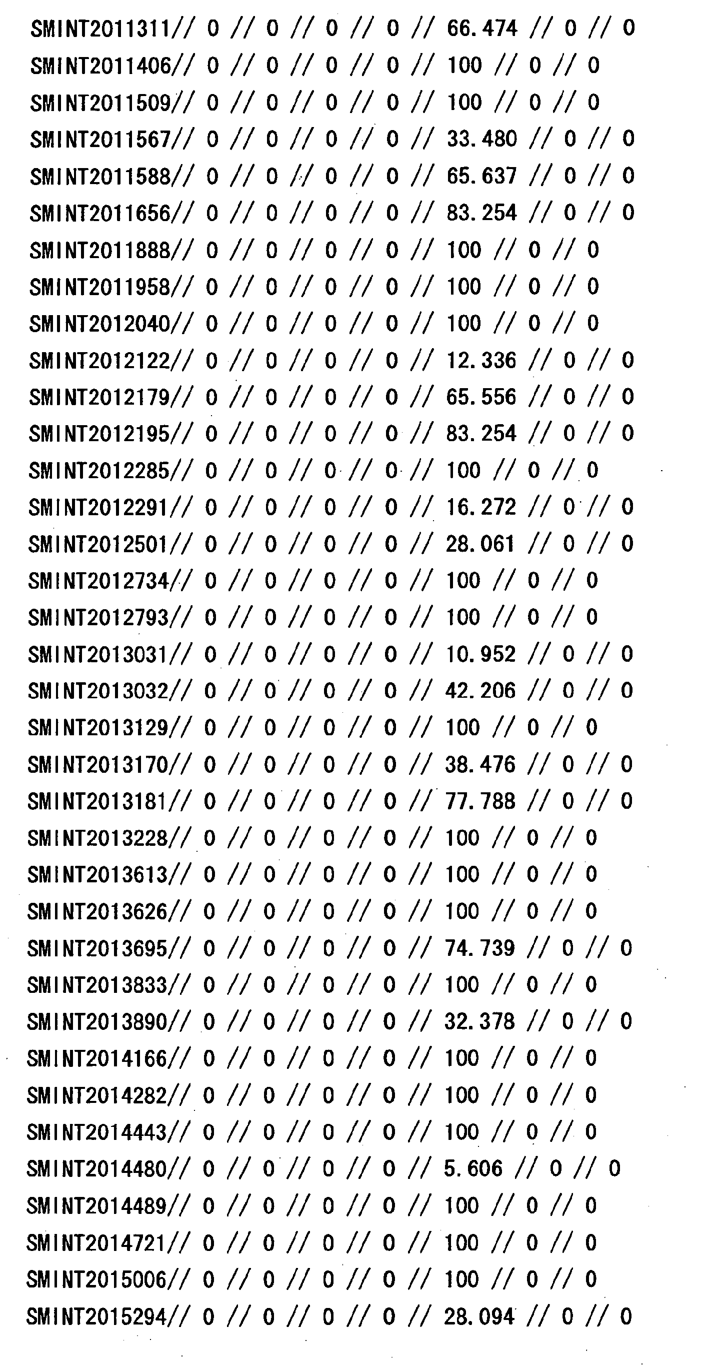

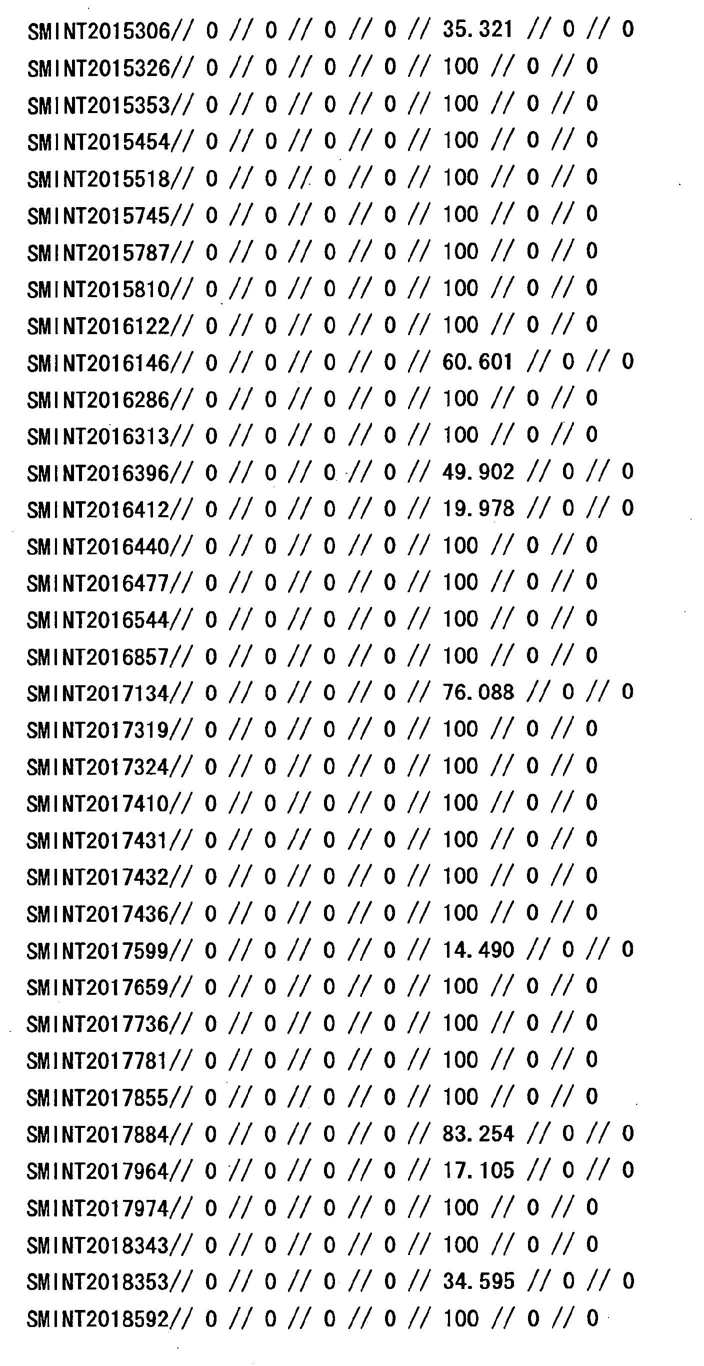

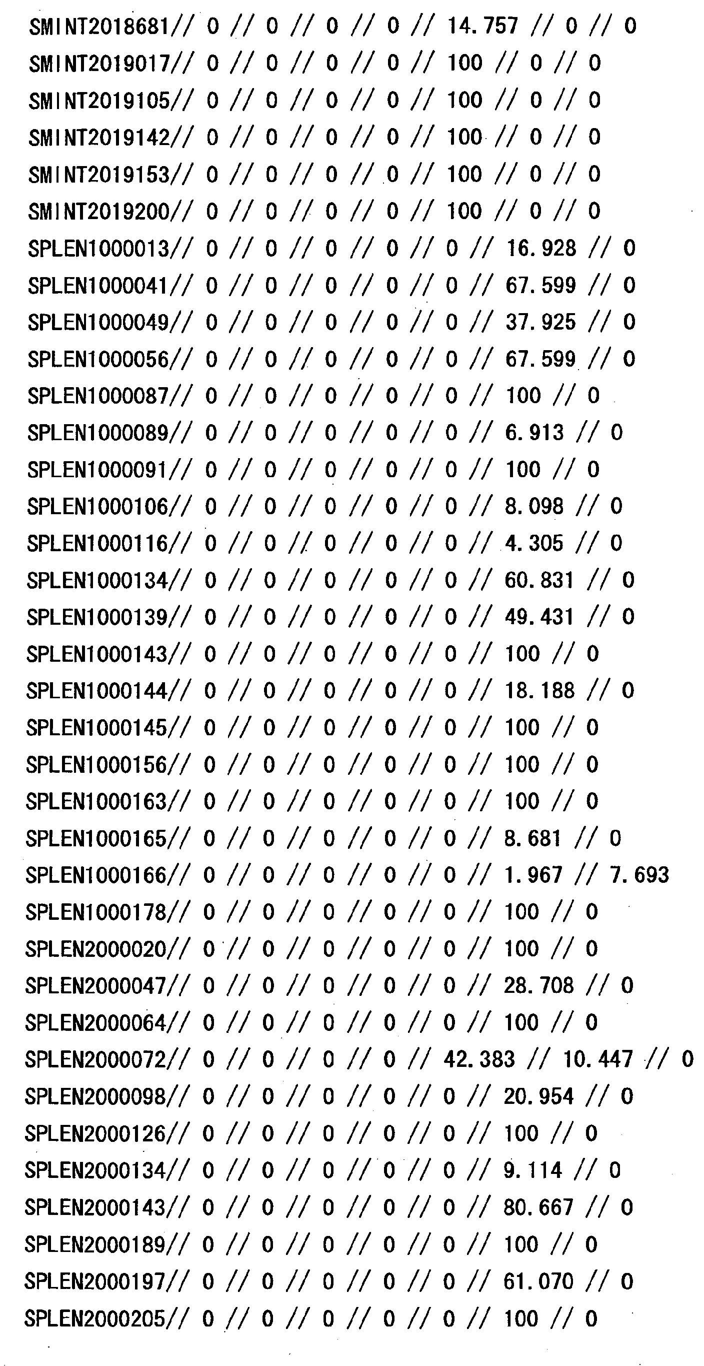

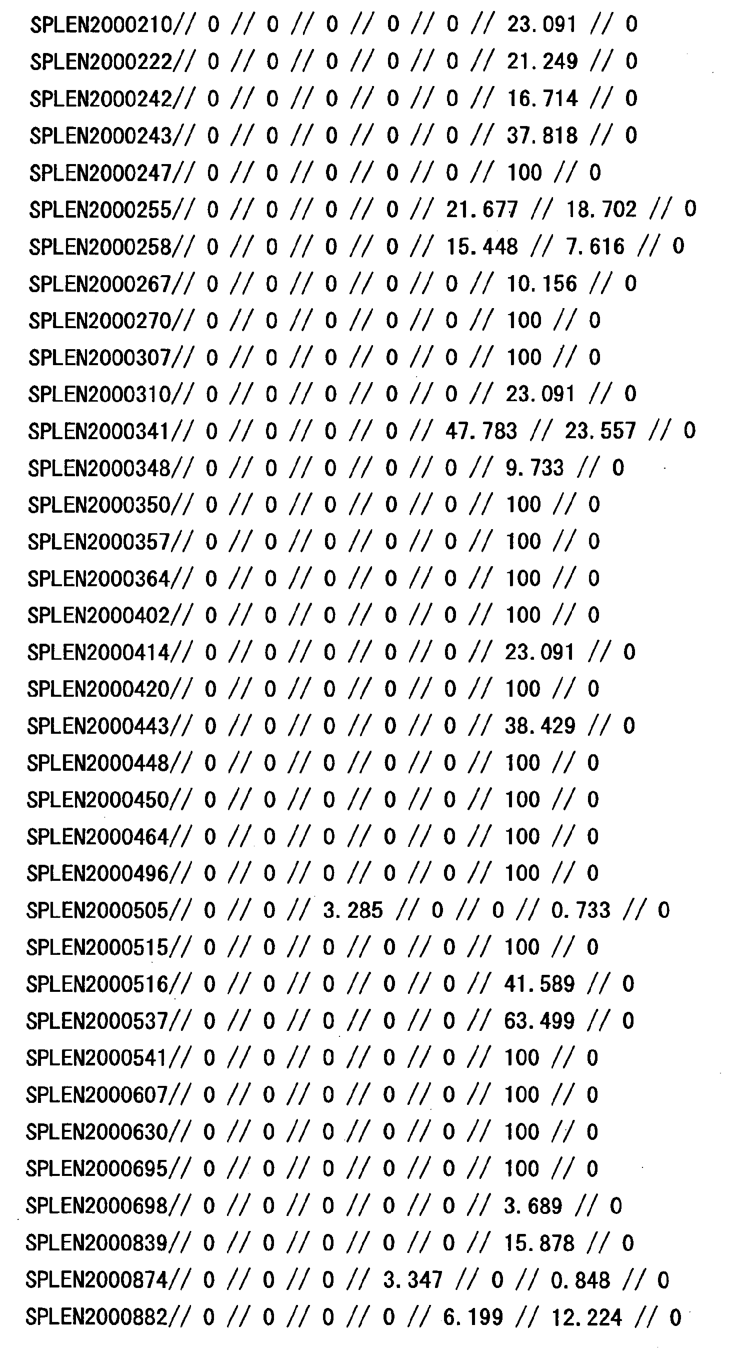

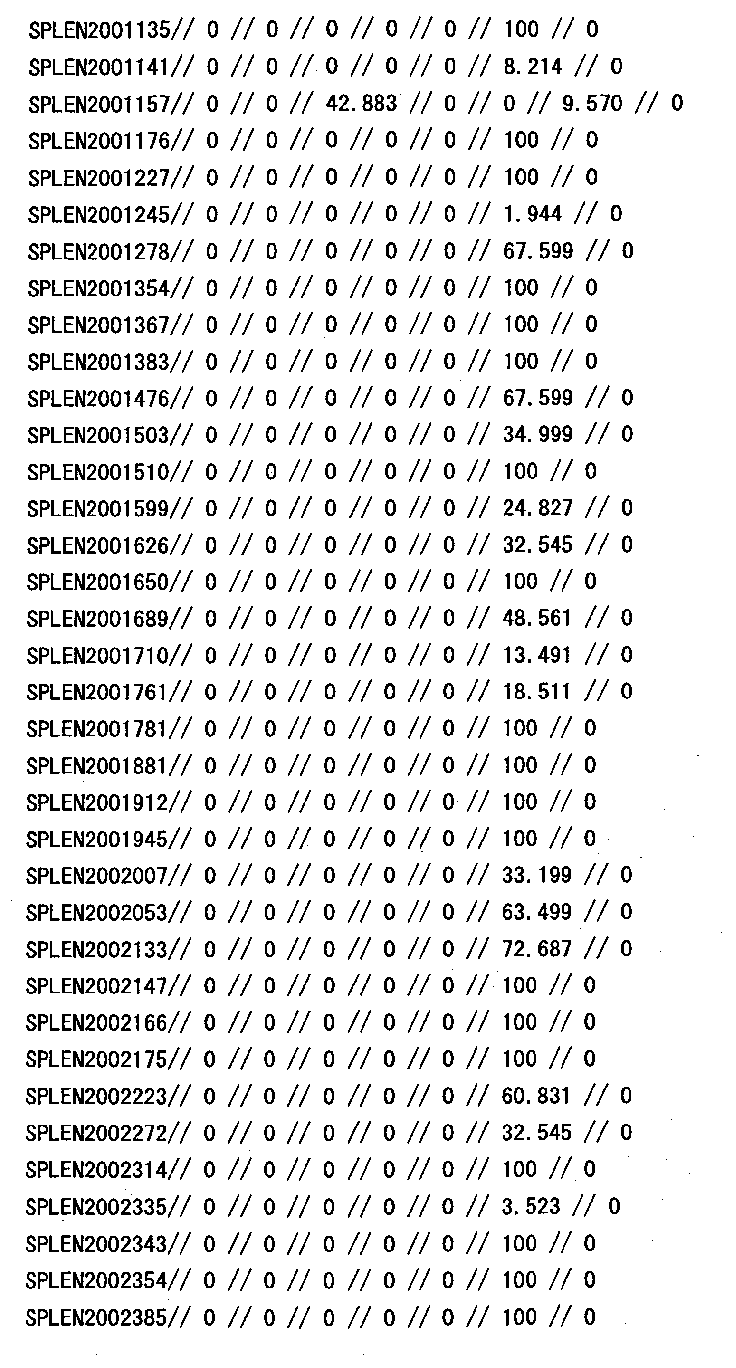

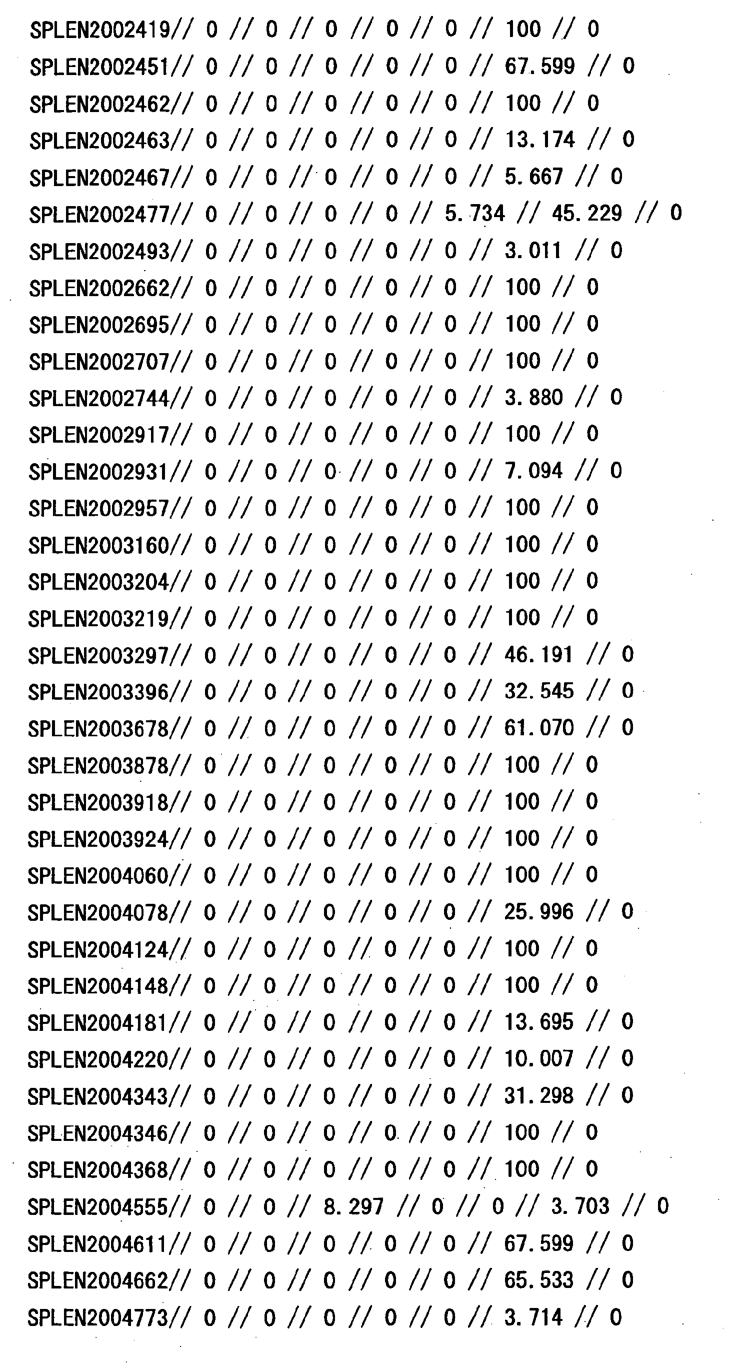

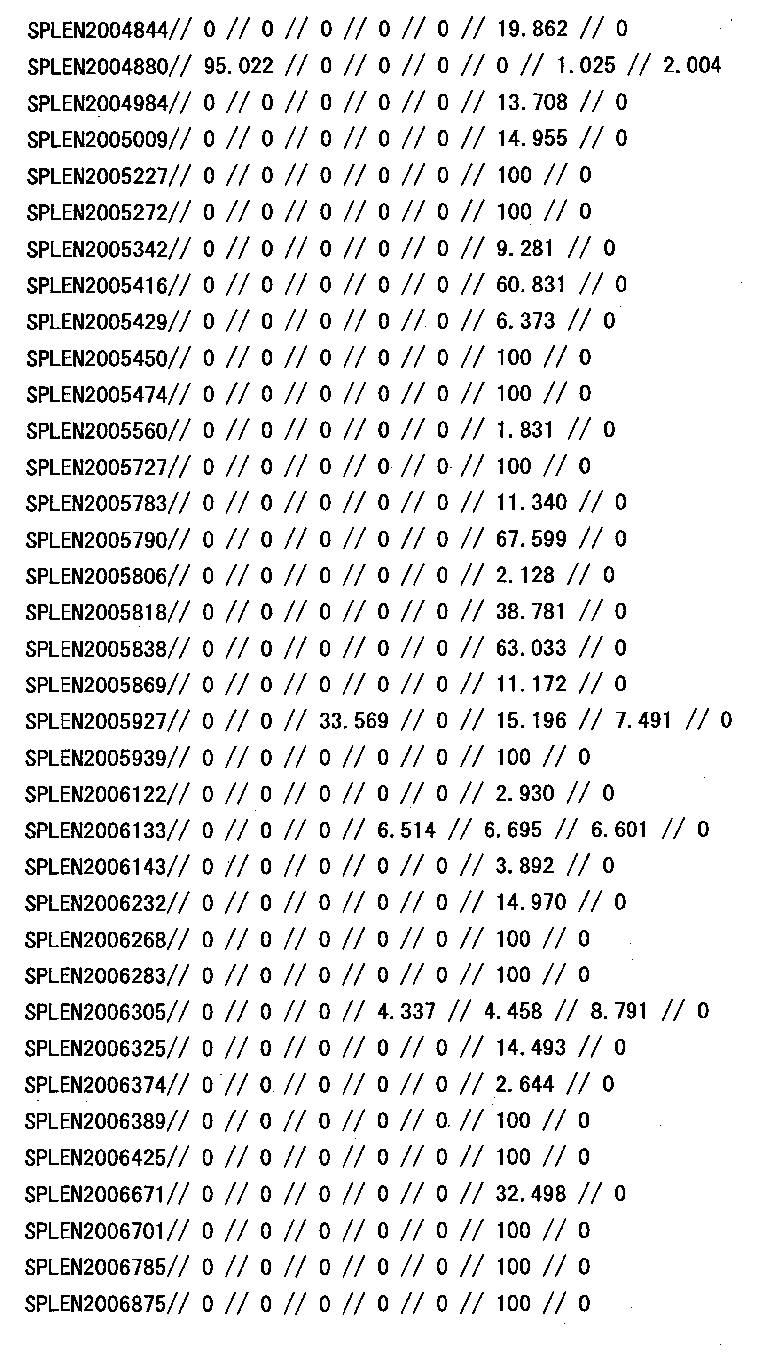

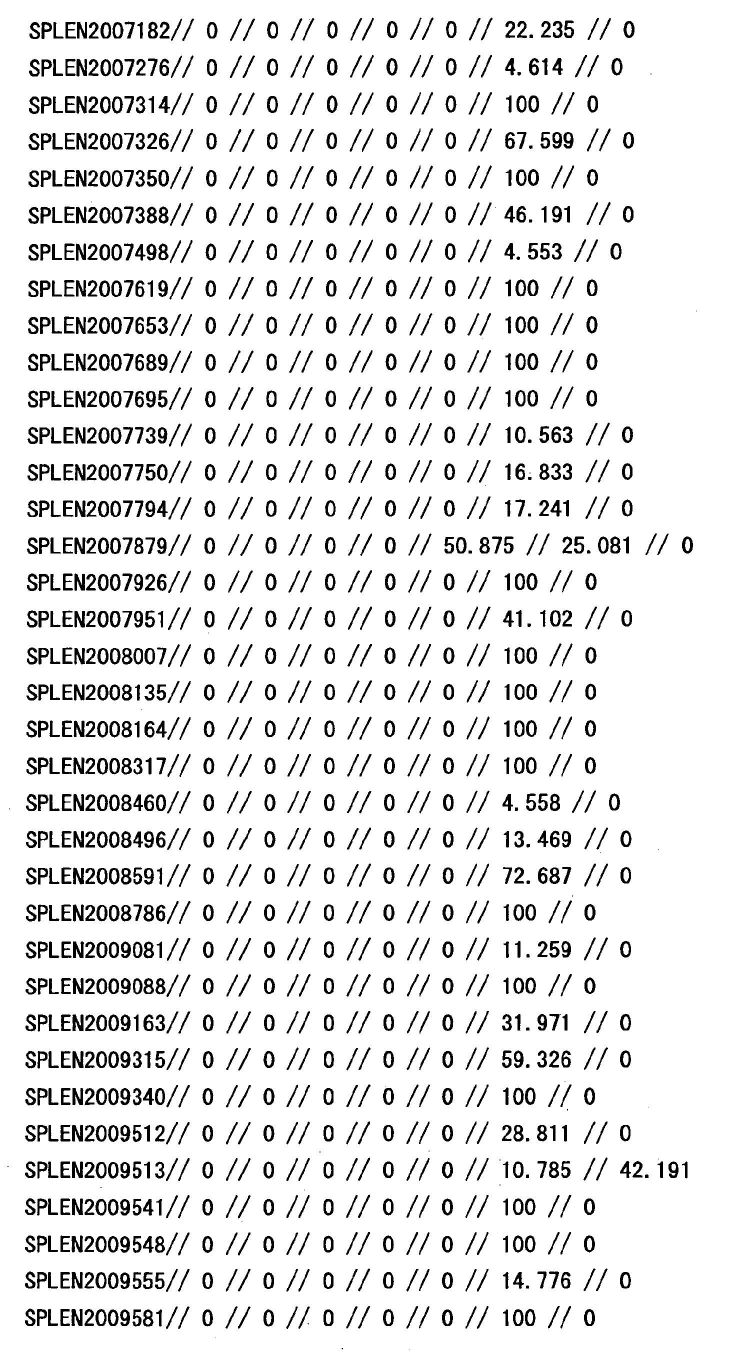

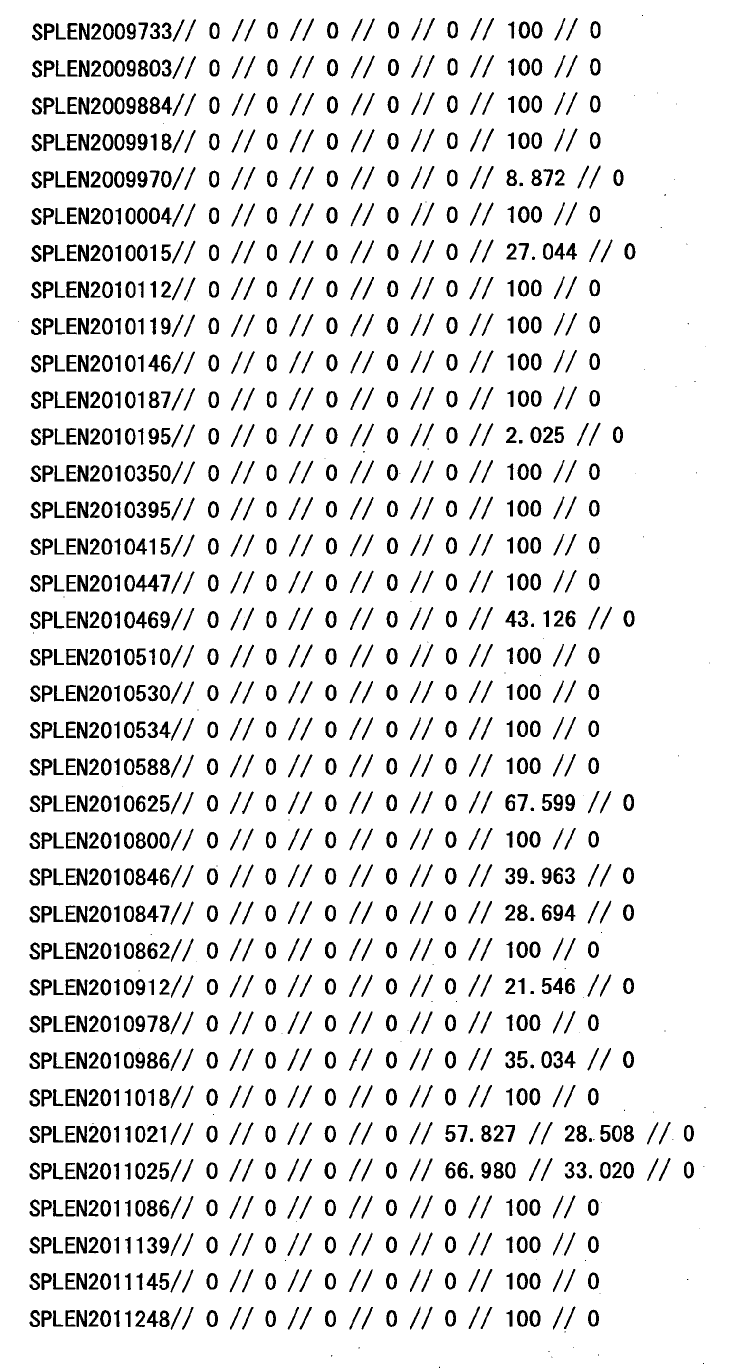

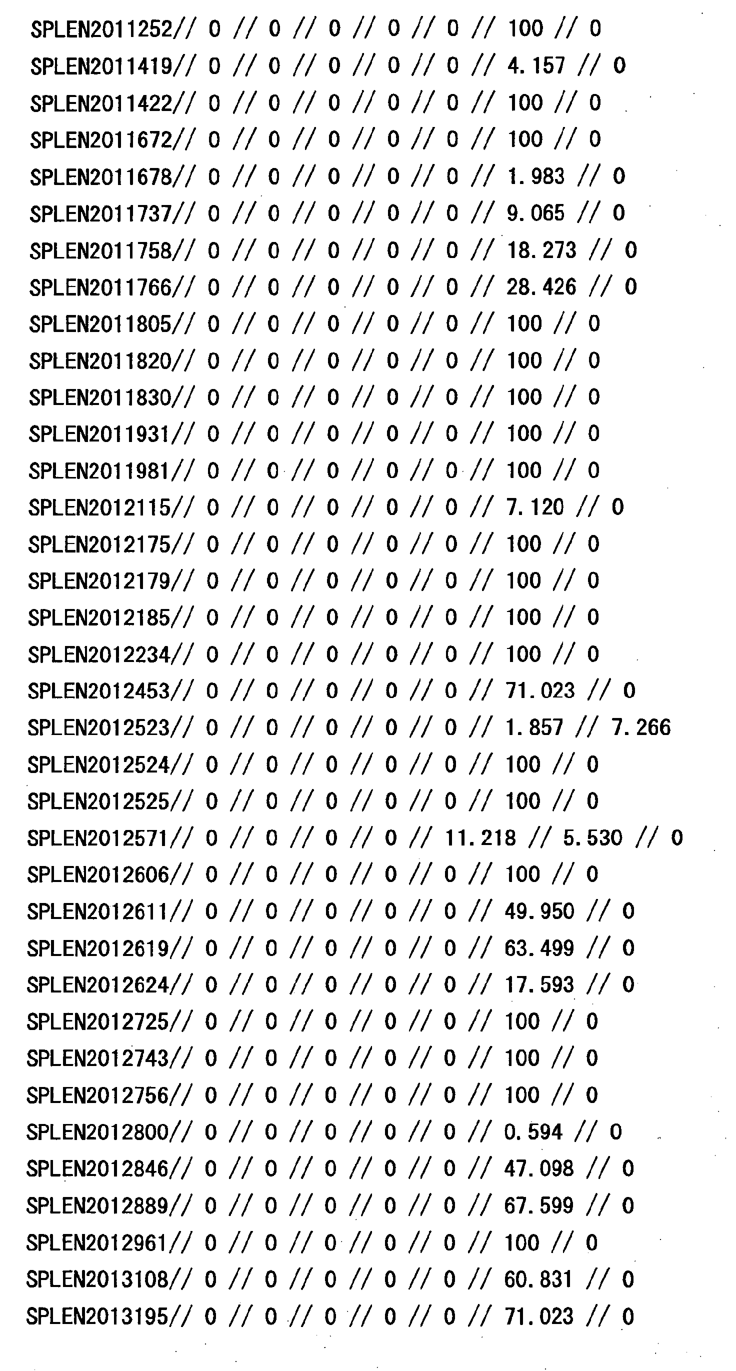

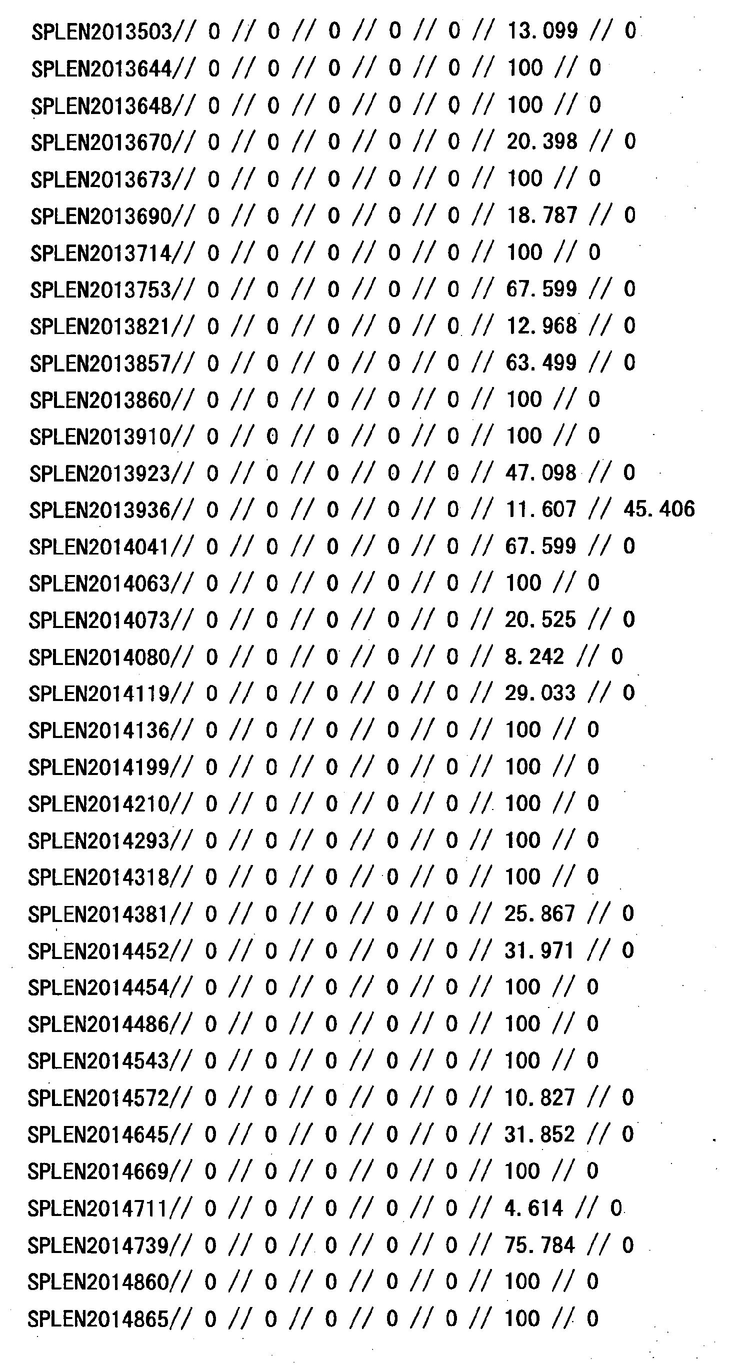

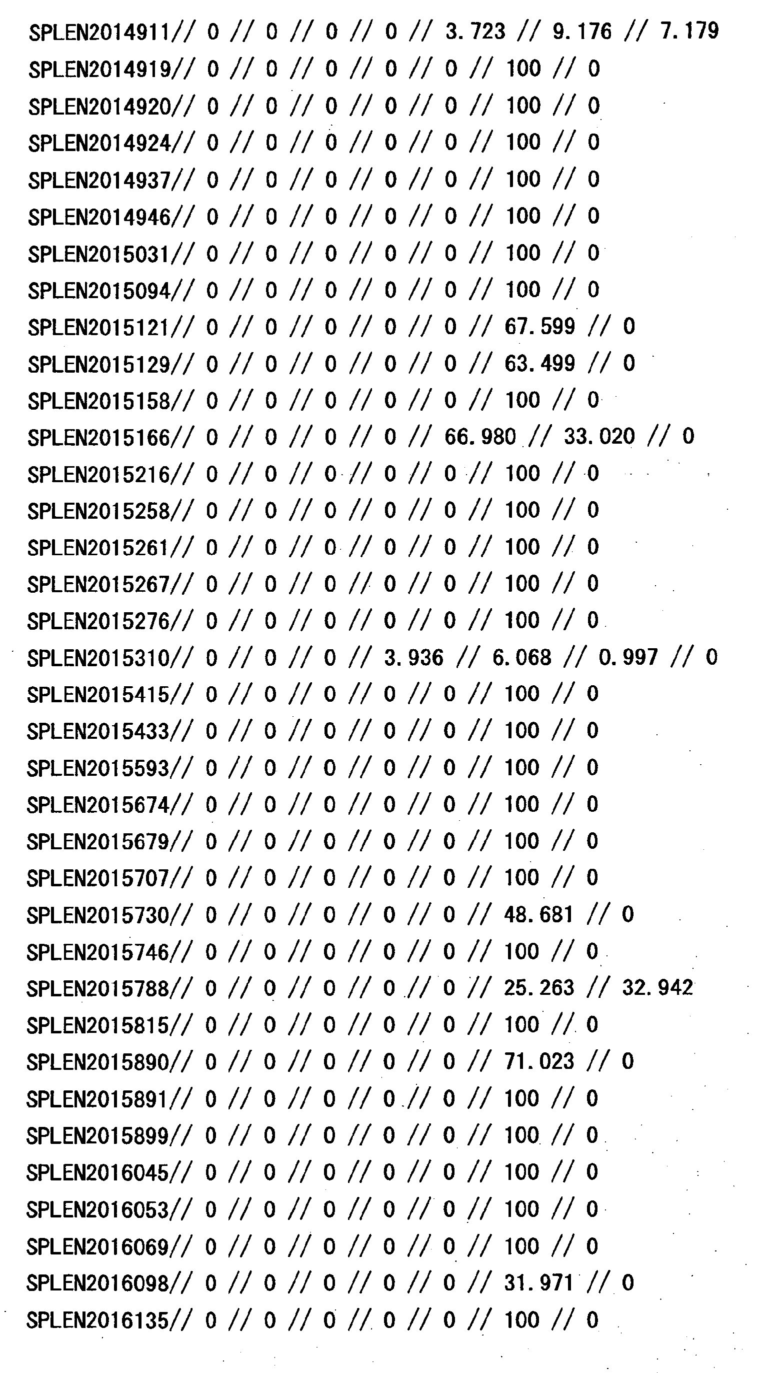

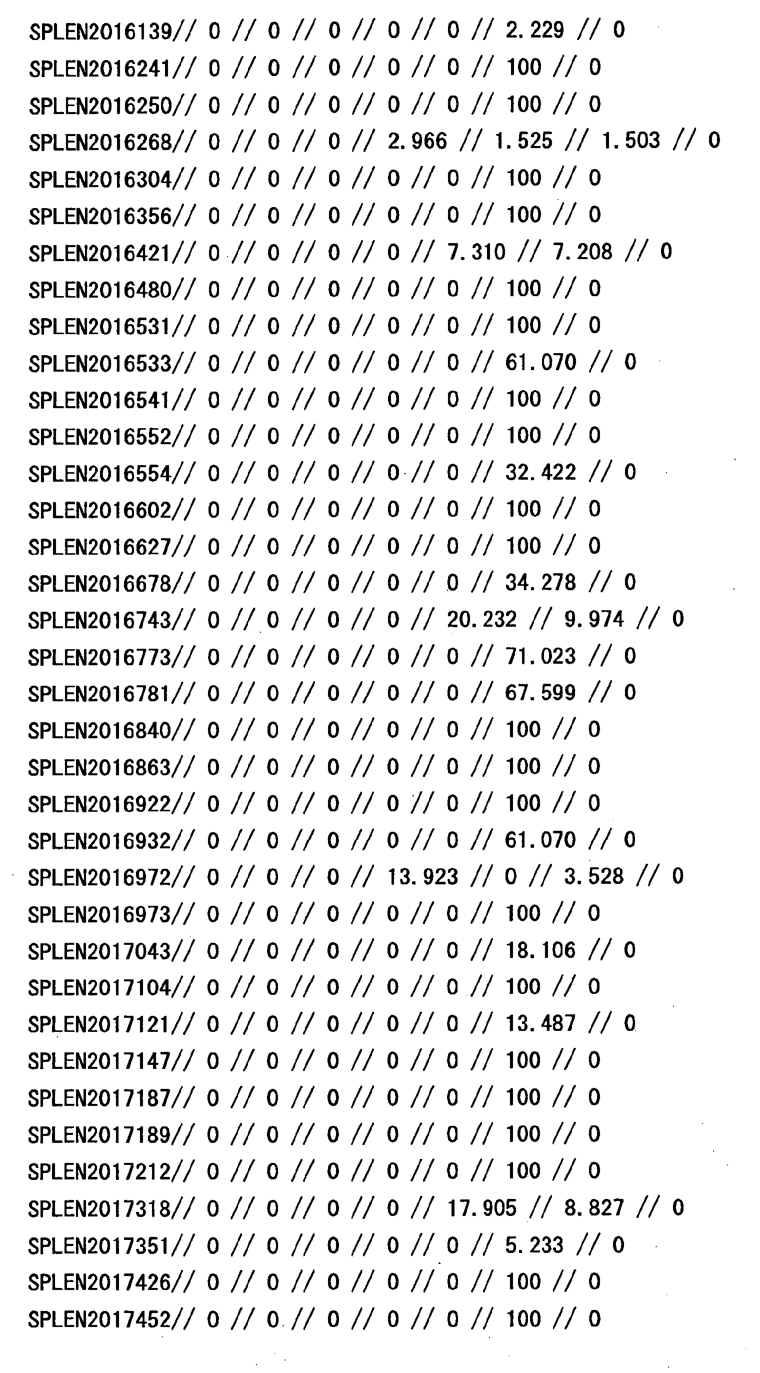

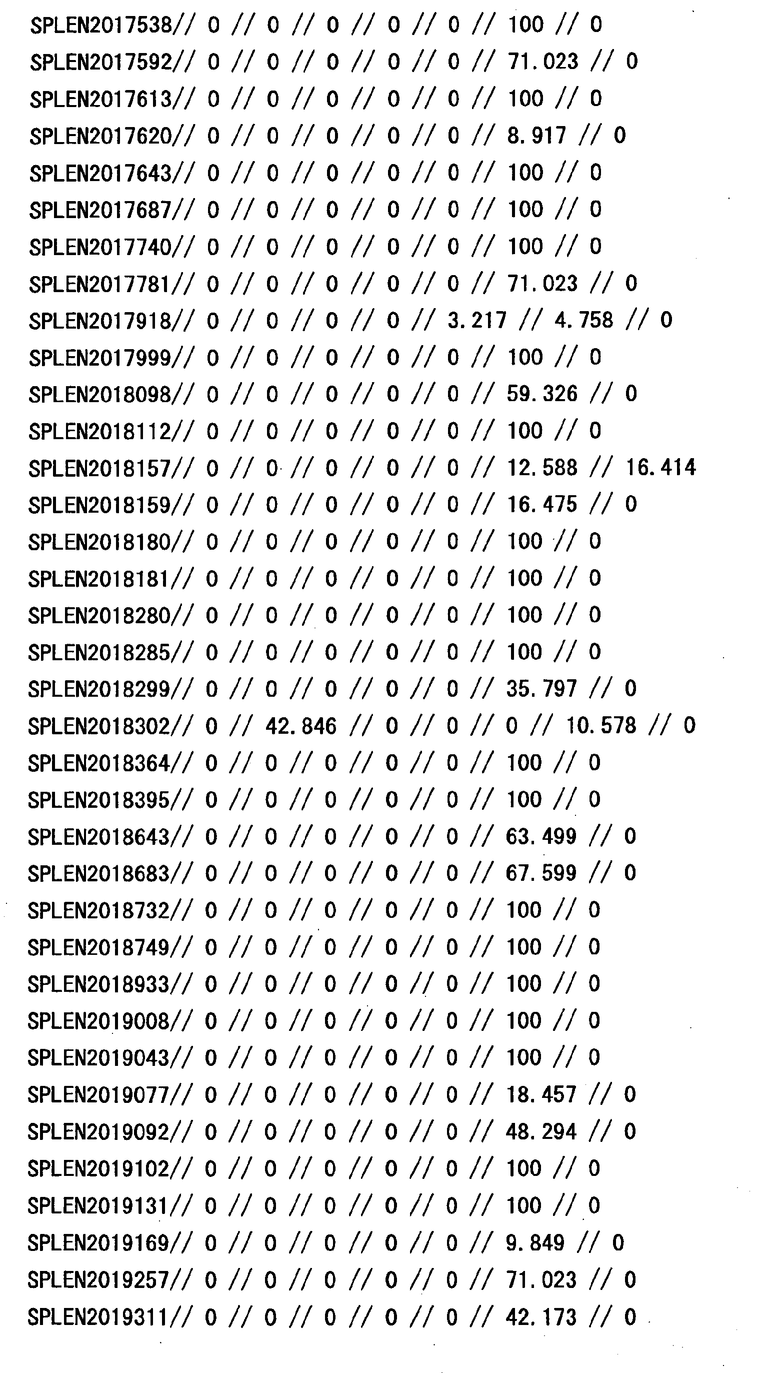

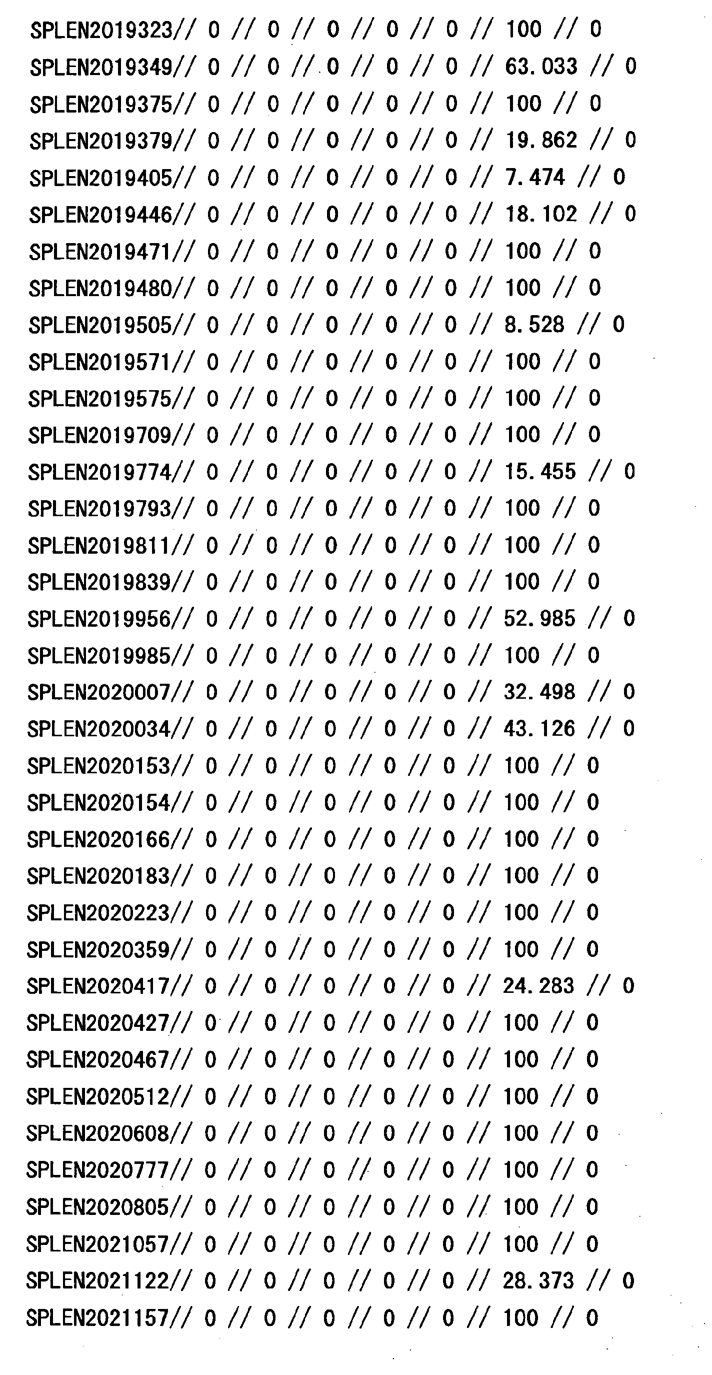

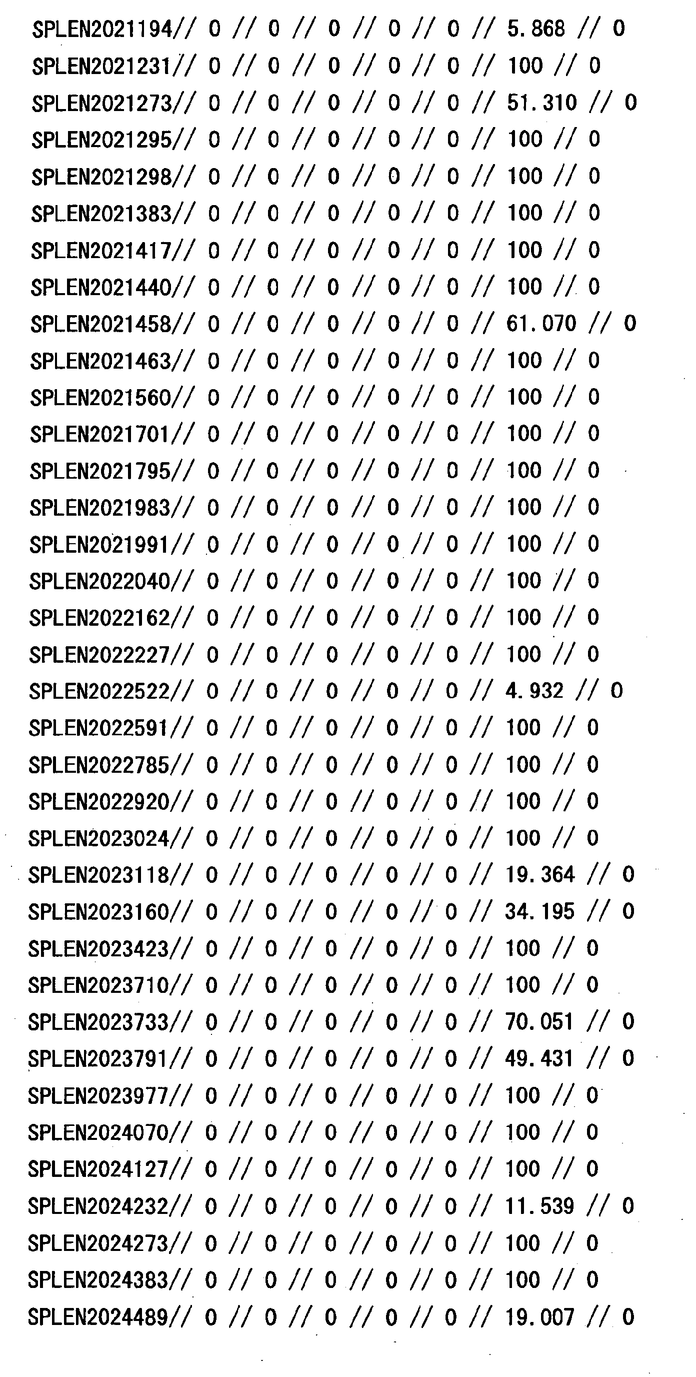

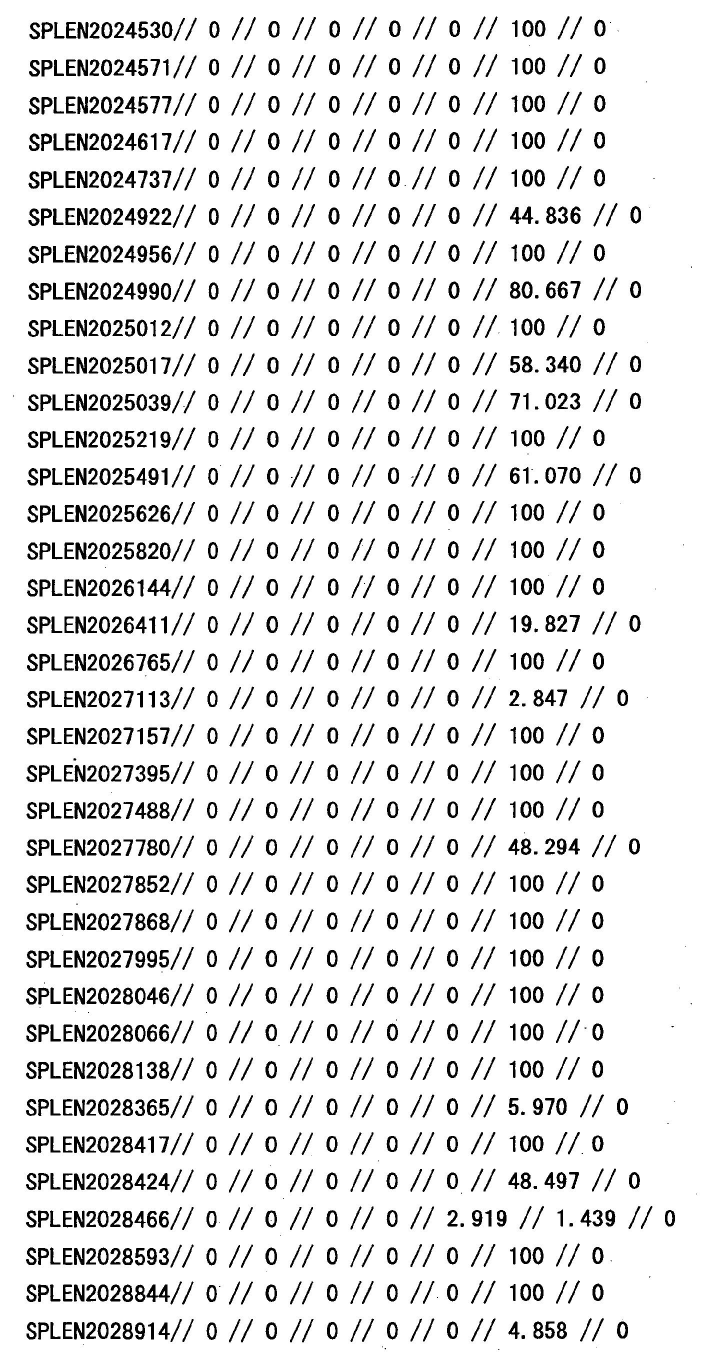

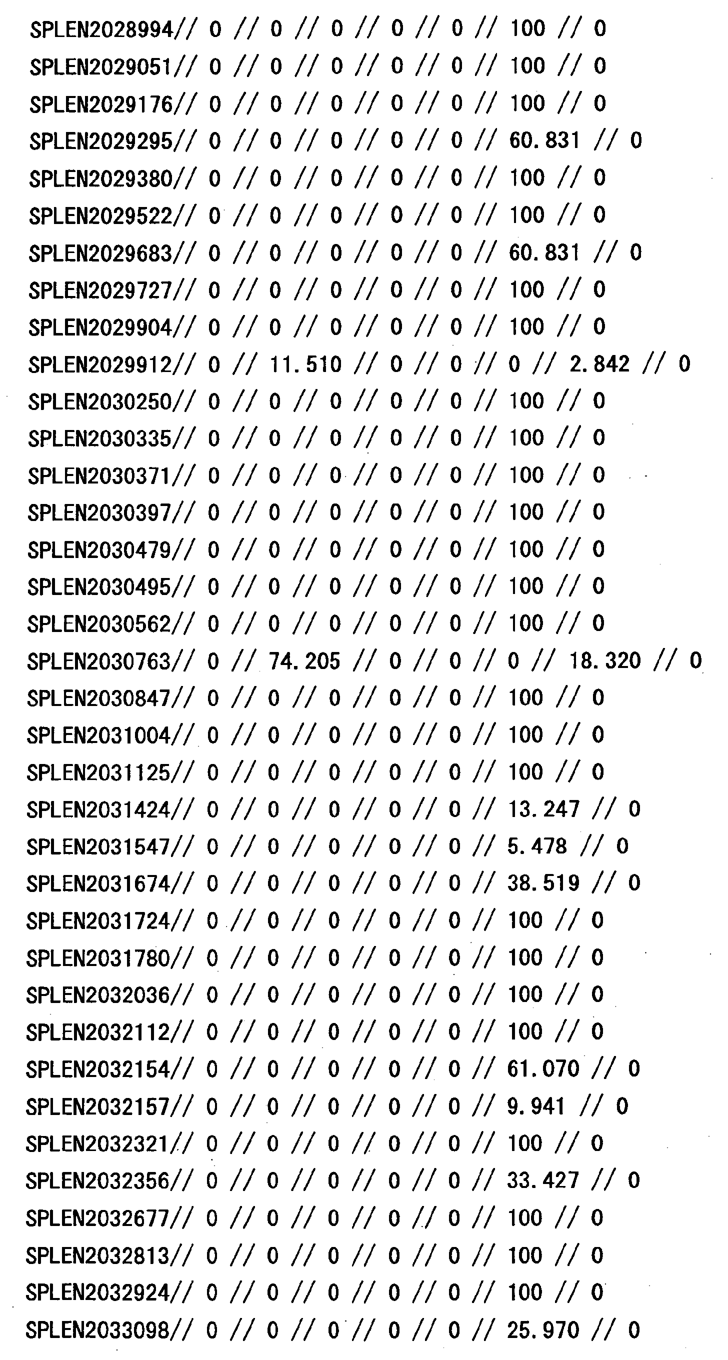

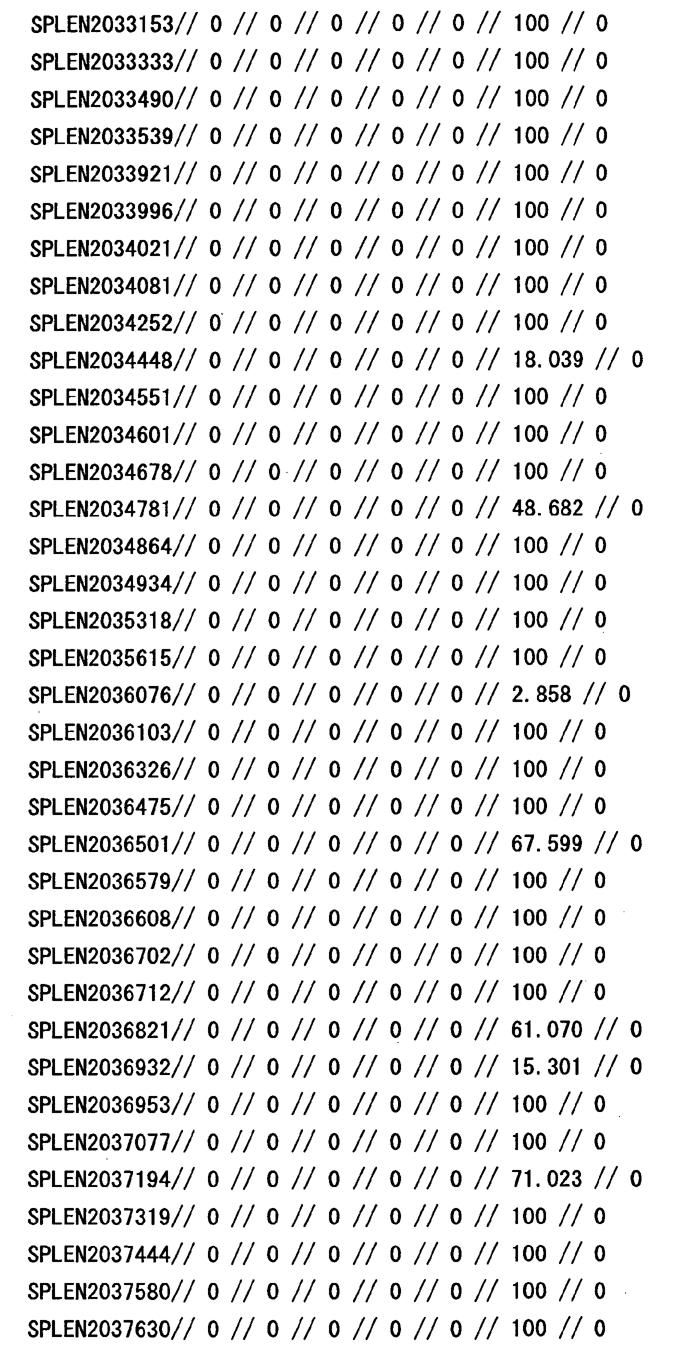

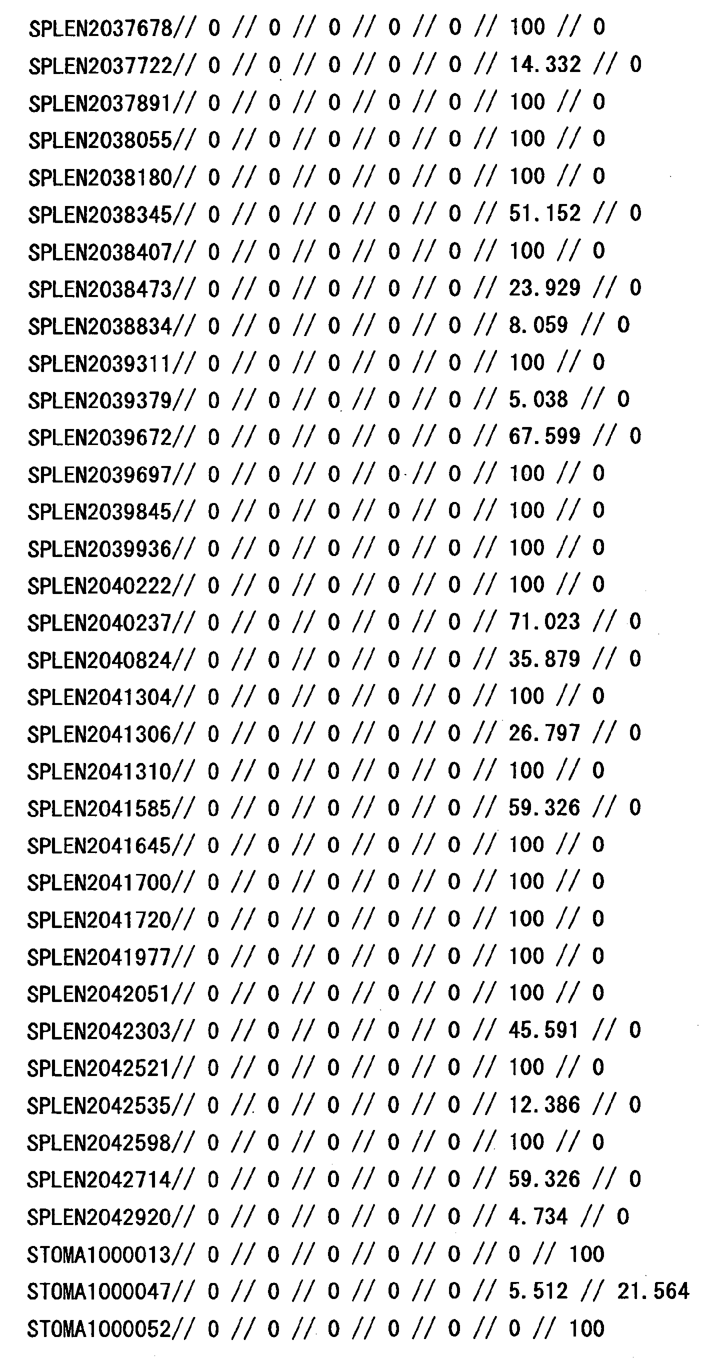

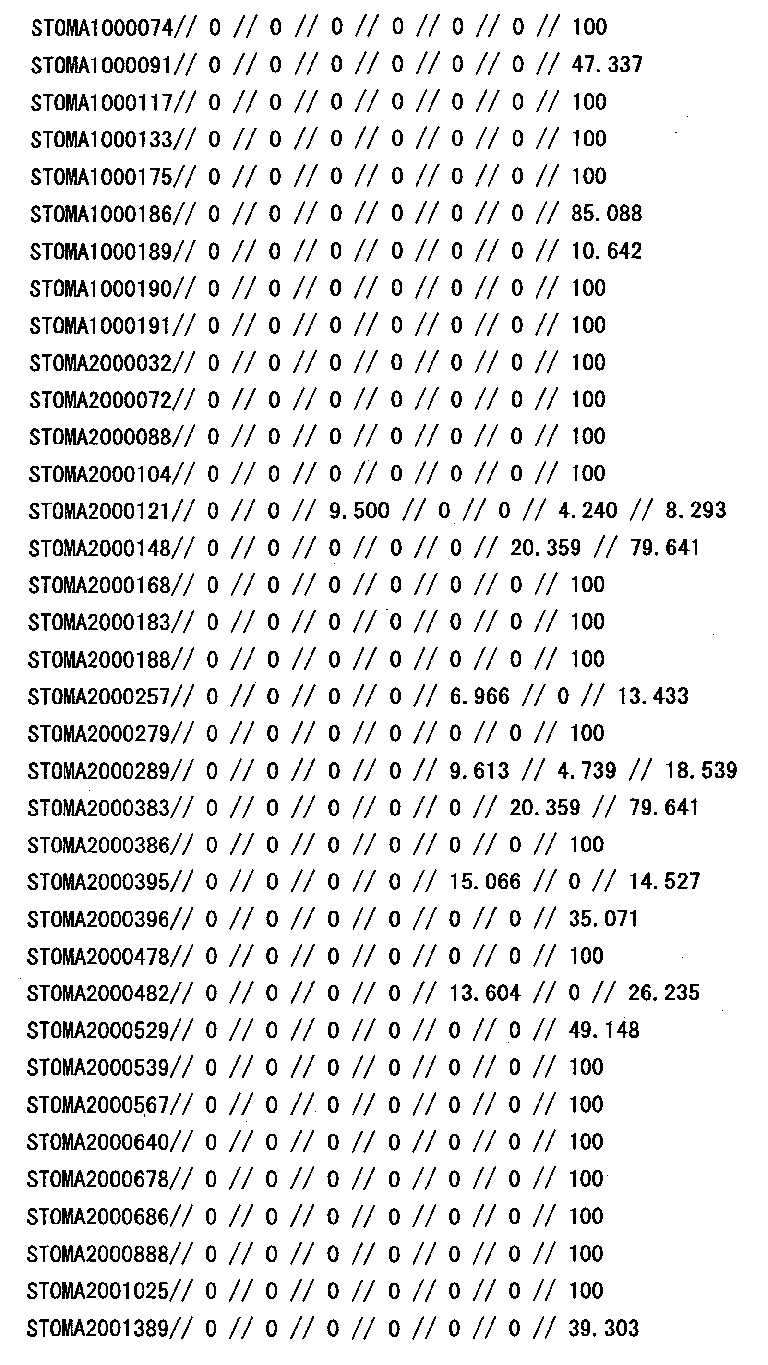

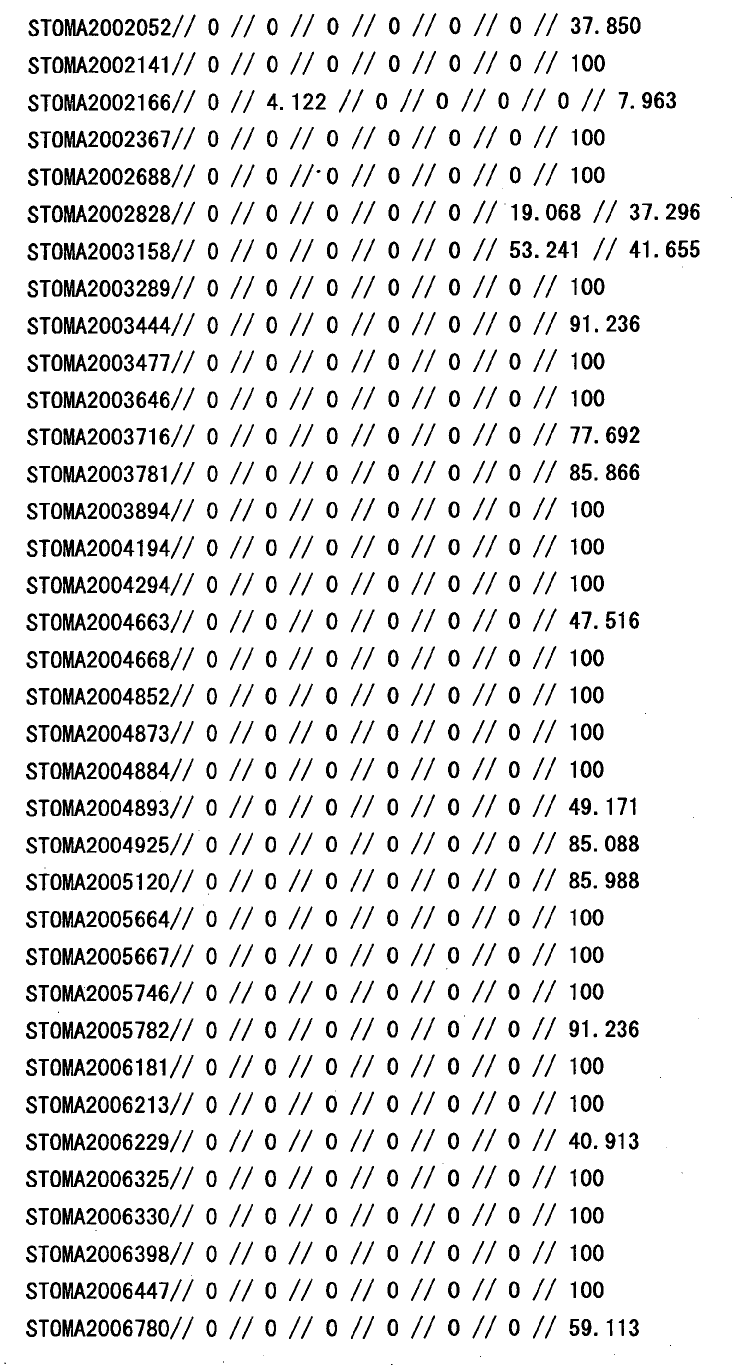

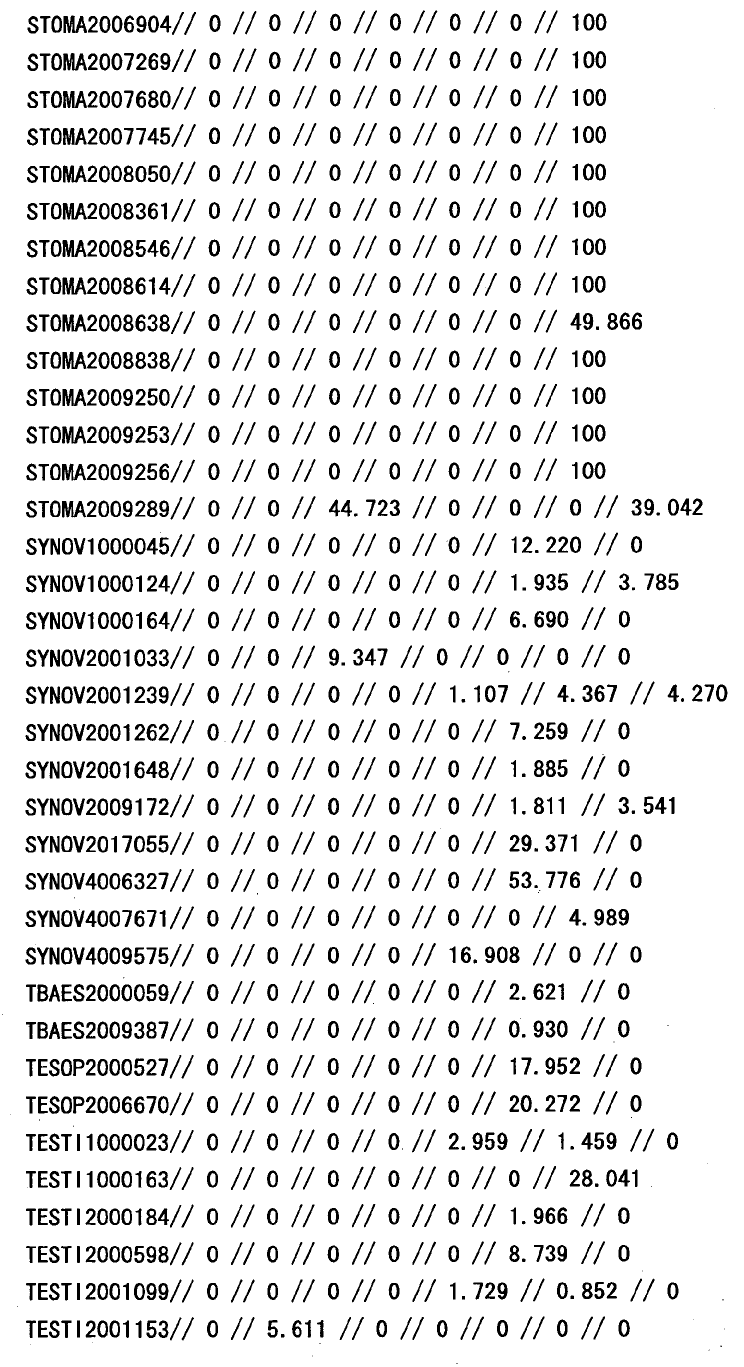

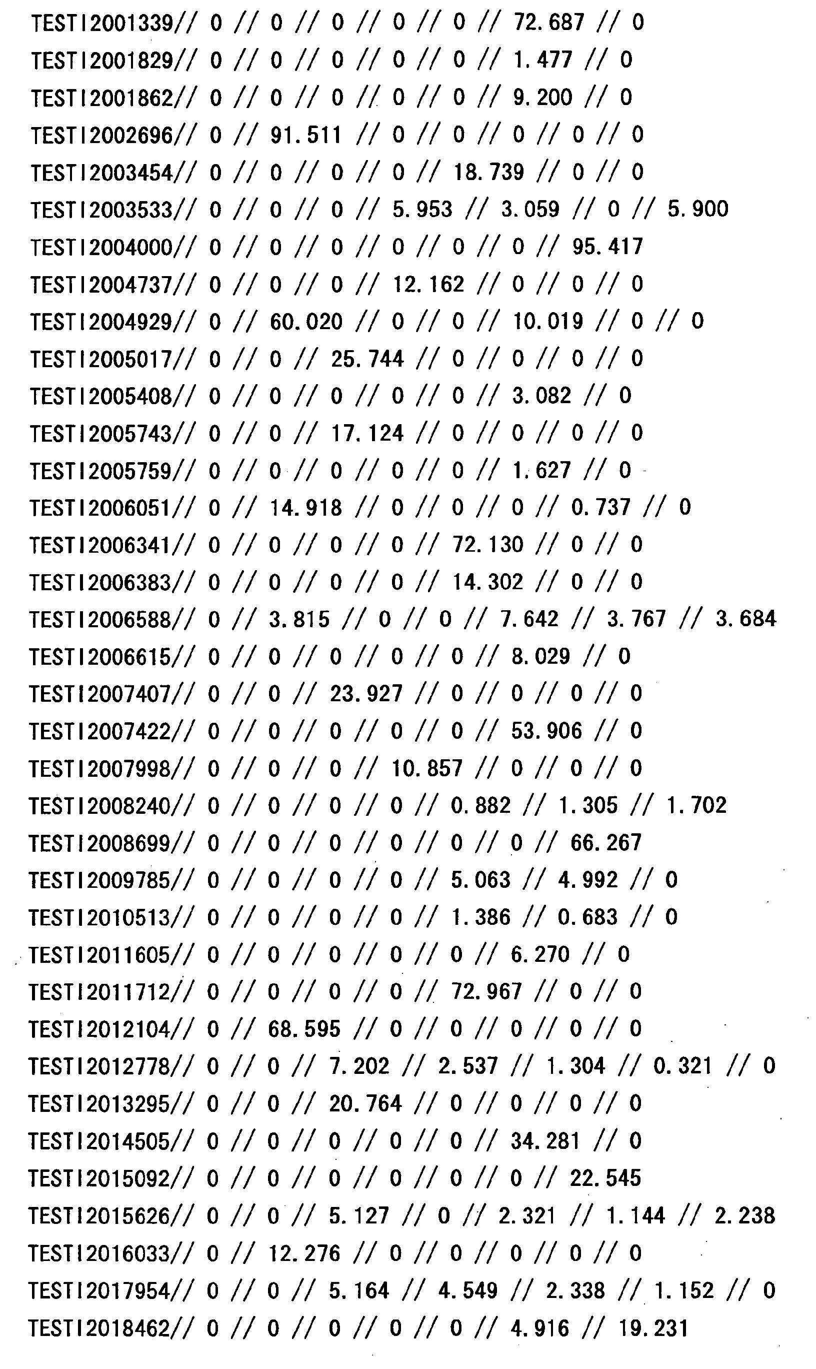

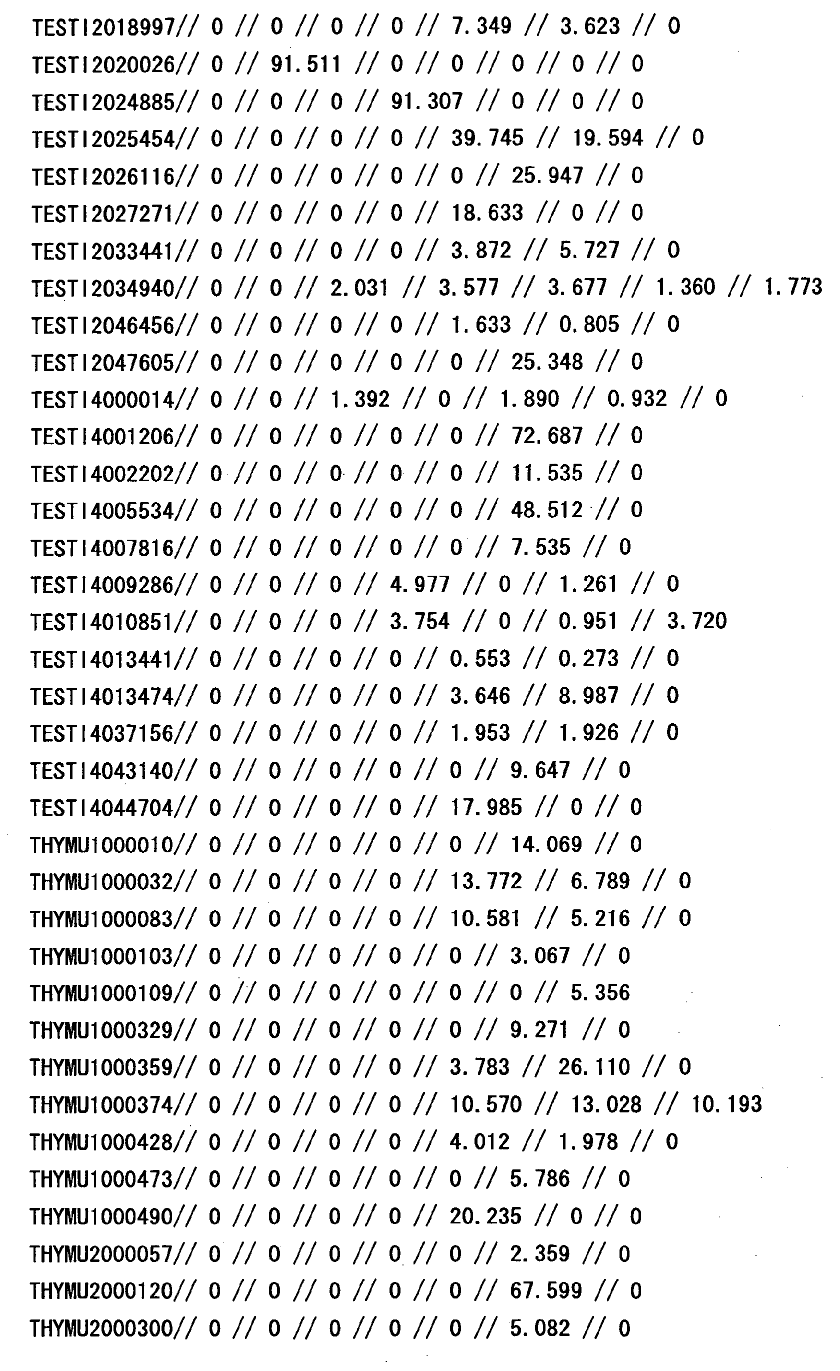

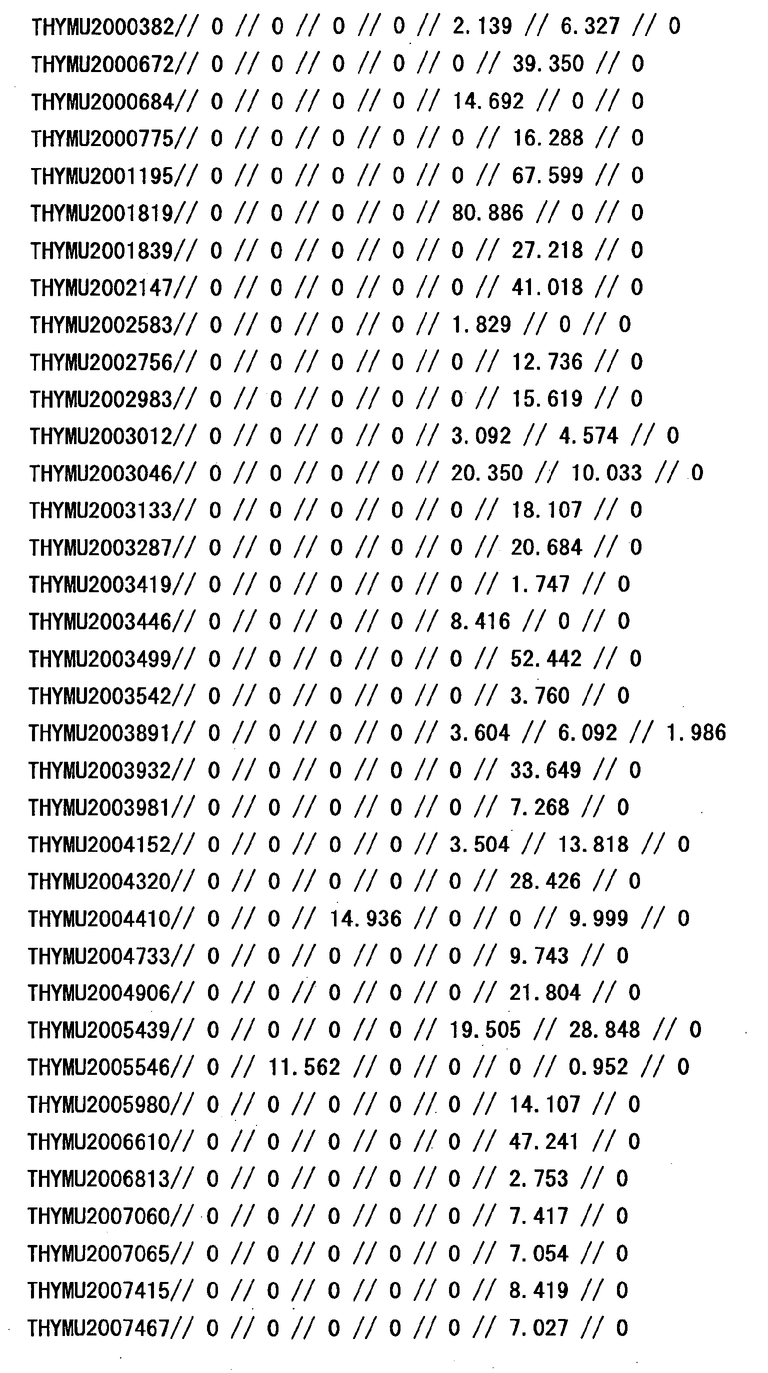

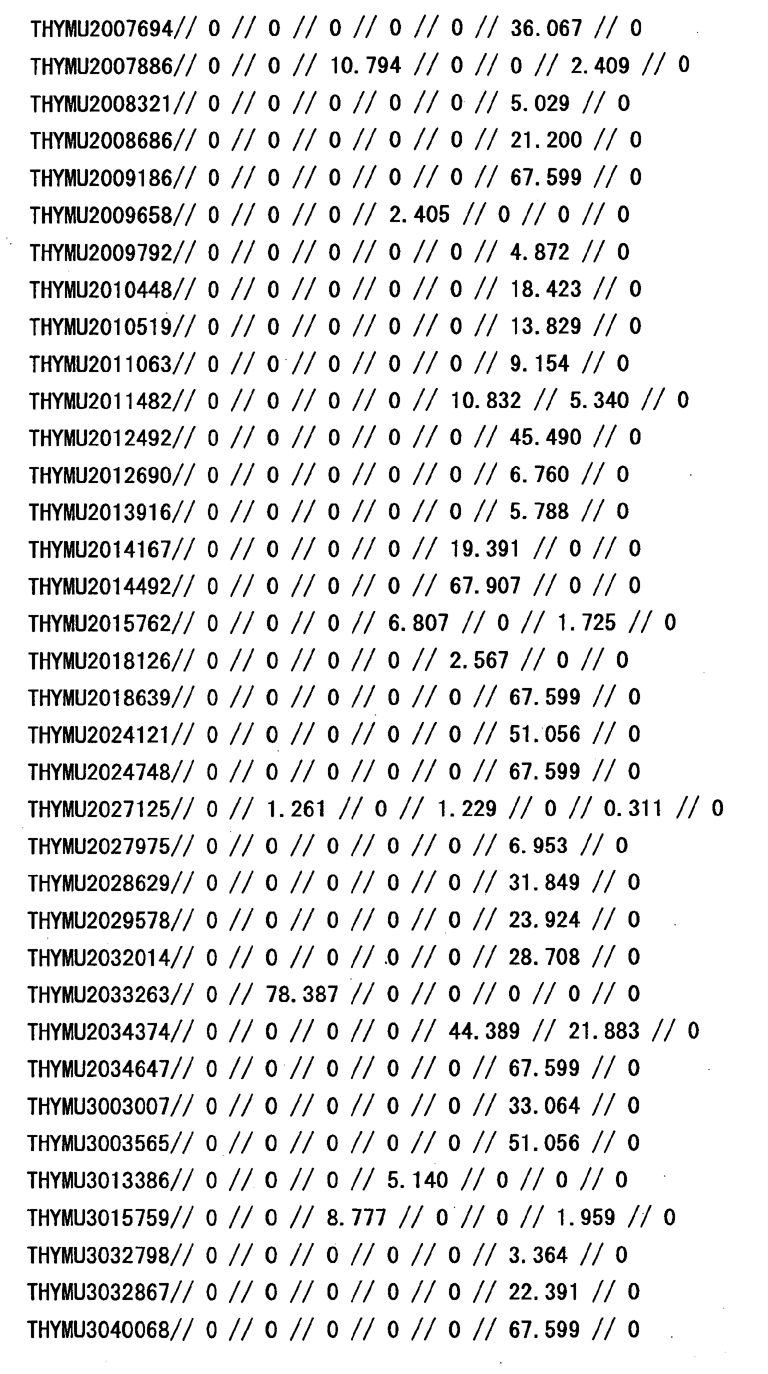

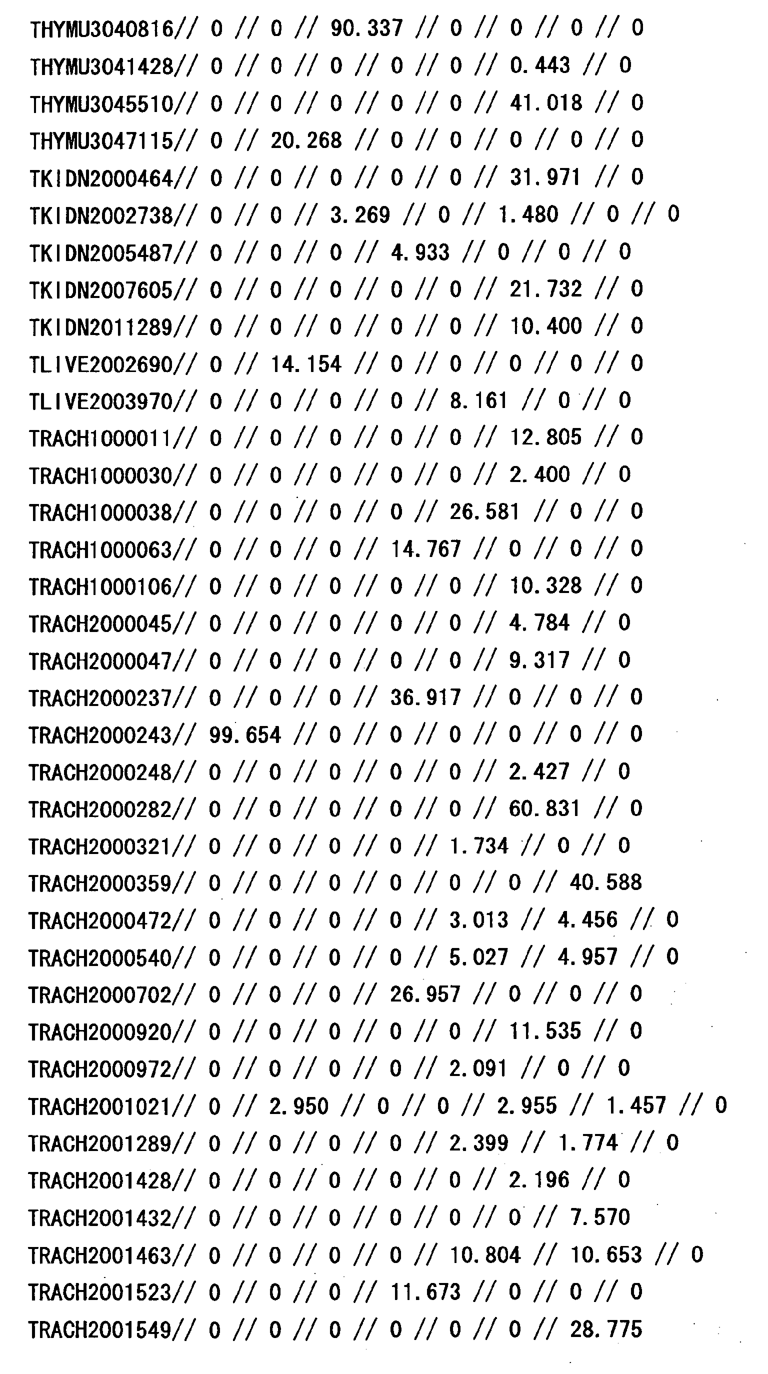

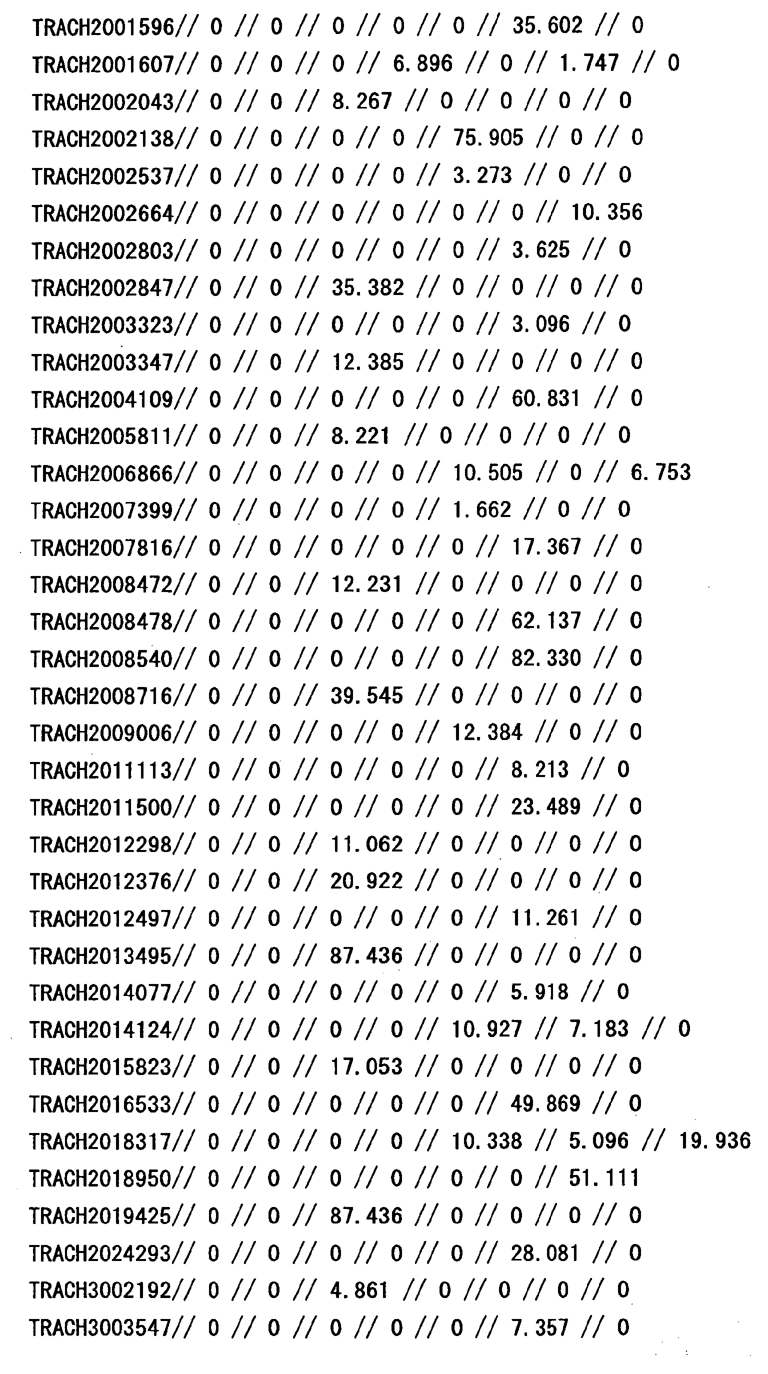

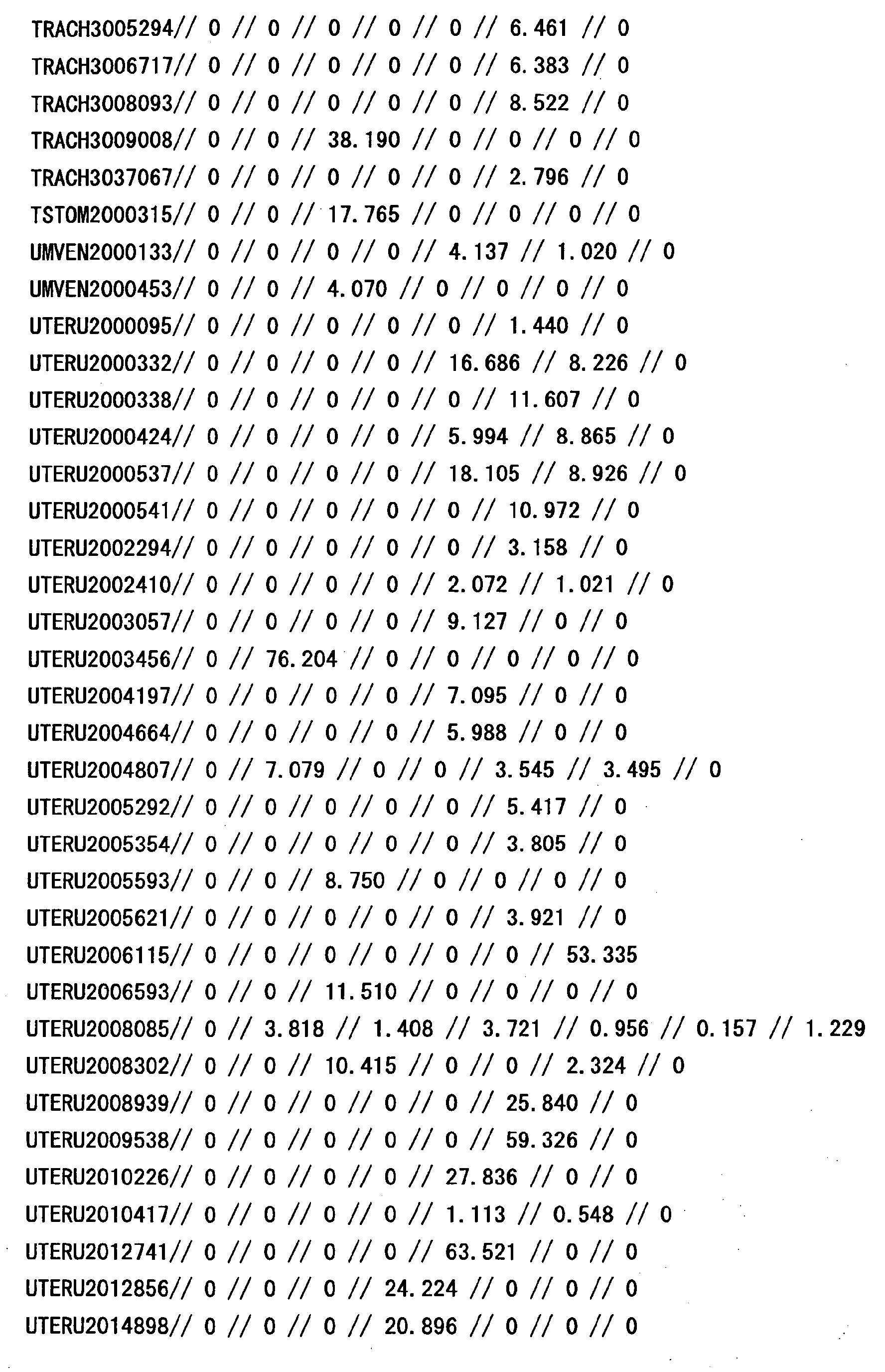

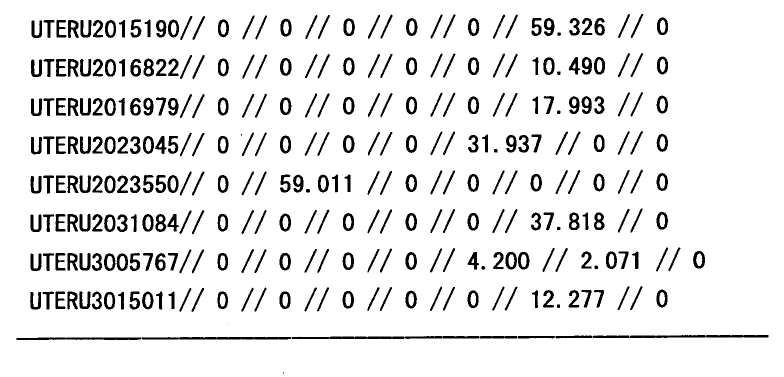

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000374, 3NB692000429, 3NB692000484, 3NB692000529, 3NB692001034, 3NB692001040, 3NB692001123, 3NB692001267, 3NB692001288, 3NB692001349, 3NB692001366, 3NB692001408, 3NB692001433, 3NB692001471, 3NB692001496, 3NB692001501, 3NB692001507, 3NB692001511, 3NB692001519, 3NB692001520, 3NB692001548, 3NB692001557, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000147, ADRGL1000160, ADRGL1000165, ADRGL1000182, ADRGL2000006, ADRGL2000042, ADRGL2000056, ADRGL2000064, ADRGL2000074, ADRGL2000085, ADRGL2000097, ADRGL2000142, ADRGL2000172, ADRGL2000248, ADRGL2000261, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002679,
ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023,
ADRGL2008331, ADRGL2008337, ADRGL2008535, ADRGL2008966, ADRGL2009146, ADRGL2009273, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO2000011, ASTRO2000014, ASTRO2000046, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2000801, ASTRO2001001, ASTRO2001008, ASTRO2001029, ASTRO2001088, ASTRO2001107, ASTRO2001122, ASTRO2001227, ASTRO2001480,
ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003316, ASTRO2003461, ASTRO2003574, ASTRO2003581, ASTRO2003632, ASTRO2003740, ASTRO2003840, ASTRO2003925, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005413, ASTRO2005423, ASTRO2005557, ASTRO2005575, ASTRO2005593, ASTRO2005726, ASTRO2005863, ASTRO2005902, ASTRO2006356, ASTRO2006475, ASTRO2006732,
ASTRO2006747, ASTRO2006920, ASTRO2006952, ASTRO2007221, ASTRO2007377, ASTRO2007515, ASTRO2007578, ASTRO2007643, ASTRO2007666, ASTRO2007948, ASTRO2007956, ASTRO2007962, ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392,
ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285, BGGI12000022, BGGI12000067, BGGI12000161, BGGI12000533, BGGI12000544, BGGI12000684, BGGI12000693, BGGI12001002, BGGI12001097, BGGI12001241, BGGI12001247, BGGI12001682, BLADE1000176, BLADE2000181, BLADE2000340, BLADE2000389, BLADE2000463,
BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2005036, BLADE2005038, BLADE2005229, BLADE2005459, BLADE2005792, BLADE2006043, BLADE2006412, BLADE2006415, BLADE2006607, BLADE2006826, BLADE2006830, BLADE2006849, BLADE2006947, BLADE2007033, BLADE2007043, BLADE2007589, BLADE2007666, BLADE2007722,
BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000153, BNGH41000169, BNGH41000177, BNGH41000198, BNGH41000216, BNGH42000130, BNGH42000132, BNGH42000405, BNGH42000532, BNGH42000675, BNGH42000791, BNGH42000815, BNGH42000994, BNGH42001247, BNGH42001406, BNGH42001724, BNGH42002244, BNGH42002387, BNGH42002487, BNGH42002489, BNGH42003529, BNGH42003570, BNGH42003641, BNGH42004076, BNGH42004291, BNGH42004538, BNGH42004679, BNGH42005017, BNGH42005235, BNGH42005504,
BNGH42005968, BNGH42006135, BNGH42006226, BNGH42006234, BNGH42007037, BNGH42007460, BNGH42007594, BNGH42007788, BNGH42007798, BNGH42008199, BNGH42008510, BNGH42008603, BNGH42008649, BNGH42008743, BNGH42008832, BNGH42008850, BNGH42008943, BNGH42009021, BRACE1000020, BRACE1000047, BRACE1000065, BRACE1000070, BRACE1000073, BRACE1000084, BRACE1000087, BRACE1000092, BRACE1000093, BRACE1000101, BRACE1000114, BRACE1000124, BRACE1000132, BRACE1000159, BRACE1000166, BRACE1000169, BRACE1000186, BRACE1000187, BRACE1000239, BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229,
BRACE2000232, BRACE2000245, BRACE2000256, BRACE2000266, BRACE2000280, BRACE2000300, BRACE2000331, BRACE2000351, BRACE2000368, BRACE2000392, BRACE2000441, BRACE2000487, BRACE2000489, BRACE2000505, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000750, BRACE2000815, BRACE2000885, BRACE2000893, BRACE2000905, BRACE2000936, BRACE2000947, BRACE2000965, BRACE2000969, BRACE2000975, BRACE2000988, BRACE2001017, BRACE2001065, BRACE2001070, BRACE2001094, BRACE2001107, BRACE2001117, BRACE2001143, BRACE2001154, BRACE2001188, BRACE2001191, BRACE2001197, BRACE2001236,
BRACE2001239, BRACE2001269, BRACE2001312, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001677, BRACE2001692, BRACE2001705, BRACE2001711, BRACE2001726, BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001892, BRACE2001898, BRACE2001923, BRACE2001942, BRACE2001944, BRACE2001954, BRACE2001971, BRACE2001972, BRACE2001973,
BRACE2002026, BRACE2002034, BRACE2002081, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002392, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002649, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002812,
BRACE2002834, BRACE2002849, BRACE2002857, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003398, BRACE2003420, BRACE2003428, BRACE2003431, BRACE2003512, BRACE2003516, BRACE2003527, BRACE2003539, BRACE2003540, BRACE2003594, BRACE2003598, BRACE2003609, BRACE2003614, BRACE2003628, BRACE2003639, BRACE2003700, BRACE2003713, BRACE2003766, BRACE2003785, BRACE2003788, BRACE2003800, BRACE2003802,. BRACE2003825, BRACE2003847, BRACE2003848, BRACE2003873, BRACE2003885, BRACE2003887, BRACE2003905, BRACE2003928, BRACE2003982, BRACE2003987,
BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005231, BRACE2005243, BRACE2005251, BRACE2005253, BRACE2005290, BRACE2005328, BRACE2005329, BRACE2005337, BRACE2005348, BRACE2005363, BRACE2005395, BRACE2005408, BRACE2005433, BRACE2005448, BRACE2005450, BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787,
BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE;2006084, BRACE2006089, BRACE2006102, BRACE2006105, BRACE2006122, BRACE2006137, BRACE2006143, BRACE2006167, BRACE2006174, BRACE2006240, BRACE2006245, BRACE2006249, BRACE2006274, BRACE2006281, BRACE2006298, BRACE2006319, BRACE2006344, BRACE2006354, BRACE2006378, BRACE2006380, BRACE2006393, BRACE2006413, BRACE2006417, BRACE2006453, BRACE2006488, BRACE2006534, BRACE2006540, BRACE2006547, BRACE2006564, BRACE2006587, BRACE2006636, BRACE2006685, BRACE2006703,
BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007087, BRACE2007138, BRACE2007174, BRACE2007197, BRACE2007201, BRACE2007203, BRACE2007232, BRACE2007262, BRACE2007274, BRACE2007281, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563,. BRACE2007577, BRACE2007621, BRACE2007640, BRACE2007641, BRACE2007646, BRACE2007663, BRACE2007682, BRACE2007685, BRACE2007727, BRACE2007749,
BRACE2007760, BRACE2007761, BRACE2007764, BRACE2007768, BRACE2007769, BRACE2007784, BRACE2007786, BRACE2007798, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008218, BRACE2008251, BRACE2008276, BRACE2008295, BRACE2008308, BRACE2008336, BRACE2008361, BRACE2008380, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008594, BRACE2008604,
BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2009014, BRACE2009016, BRACE2009031, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009421, BRACE2009428, BRACE2009434, BRACE2009437, BRACE2009483,
BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011747,
BRACE2011838, BRACE2011892, BRACE2011904, BRACE2011947, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2014108, BRACE2014161, BRACE2014232, BRACE2014257, BRACE2014268, BRACE2014306, BRACE2014475, BRACE2014657, BRACE2014746, BRACE2014780, BRACE2014821, BRACE2014824, BRACE2014850, BRACE2014854, BRACE2015031, BRACE2015058, BRACE2015132, BRACE2015262, BRACE2015270, BRACE2015314, BRACE2015352, BRACE2015368, BRACE2015412, BRACE2015436, BRACE2015741,
BRACE2015756, BRACE2015995, BRACE2016058, BRACE2016194, BRACE2016315, BRACE2016325, BRACE2016335, BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017397, BRACE2017410, BRACE2017438, BRACE2017574, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019258, BRACE2019348, BRACE2019467,
BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021014, BRACE2021148, BRACE2021245, BRACE2021254, BRACE2021264, BRACE2021344, BRACE2021433, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022394, BRACE2022435, BRACE2022448, BRACE2022450, BRACE2022549, BRACE2022633, BRACE2022693, BRACE2022848,
BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074, BRACE2024222, BRACE2024610, BRACE2024781, BRACE2024826, BRACE2025018, BRACE2025316, BRACE2025333, BRACE2025437, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026920, BRACE2026971, BRACE2027018, BRACE2027221, BRACE2027258, BRACE2027312, BRACE2027382, BRACE2027389, BRACE2027408, BRACE2027643, BRACE2027767,
BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030341, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786,
BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138,
BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492,
BRACE2047558, BRACE2047573, BRACE2047835, BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004371, BRACE3004435, BRACE3004477,
BRACE3004767, BRACE3004772, BRACE3004783, BRACE3004843, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384,
BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005,
BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805,
BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447,
BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856,
BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834,
BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168,
BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000277, BRAMY2000388, BRAMY2000411, BRAMY2000508, BRAMY2000562, BRAMY2000585, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311,
BRAMY2002339, BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004363, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182,
BRAMY2005244, BRAMY2005391, BRAMY2005488, BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105,
BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162,
BRAMY2012163, BRAMY2012277, BRAMY2012340, BRAMY2012497, BRAMY2012517, BRAMY2012536, BRAMY2012555, BRAMY2012568, BRAMY2012581, BRAMY2012691, BRAMY2012721, BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015855, BRAMY2015890,
BRAMY2015911, BRAMY2015925, BRAMY2016002, BRAMY2016009, BRAMY2016020, BRAMY2016070, BRAMY2016251, BRAMY2016325, BRAMY2016327, BRAMY2016386, BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019600, BRAMY2019643, BRAMY2019710, BRAMY2019963, BRAMY2019985, BRAMY2019989, BRAMY2020050, BRAMY2020058, BRAMY2020106,
BRAMY2020270, BRAMY2020354, BRAMY2020355, BRAMY2020412, BRAMY2020445, BRAMY2020466, BRAMY2020520, BRAMY2020574, BRAMY2020634, BRAMY2020713, BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238,
BRAMY2023327, BRAMY2023358, BRAMY2023406, BRAMY2023482, BRAMY2023514, BRAMY2023729, BRAMY2023786, BRAMY2023852, BRAMY2023863, BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538,
BRAMY2026685, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028740, BRAMY2028783, BRAMY2028796, BRAMY2028856, BRAMY2028914, BRAMY2029204, BRAMY2029602, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859,
BRAMY2030915, BRAMY2031317, BRAMY2031377, BRAMY2031442, BRAMY2031516, BRAMY2032014, BRAMY2032087, BRAMY2032231, BRAMY2032242, BRAMY2032290, BRAMY2032311, BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396,
BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244,
BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409,
BRAMY3001794, BRAMY3002120, BRAMY3002312, BRAMY3002329, BRAMY3002458, BRAMY3002508, BRAMY3002620, BRAMY3002803, BRAMY3002805, BRAMY3002886, BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158,
BRAMY3009207, BRAMY3009491, BRAMY3009556, BRAMY3009782, BRAMY3009811, BRAMY3009904, BRAMY3010264, BRAMY3010321, BRAMY3010411, BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRASW1000053, BRASW1000125, BRAWH1000001, BRAWH1000002, BRAWH1000007, BRAWH1000037, BRAWH1000045, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131,
BRAWH2000142, BRAWH2000169, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000488, BRAWH2000494, BRAWH2000500, BRAWH2000503, BRAWH2000554, BRAWH2000595, BRAWH2000636, BRAWH2000643, BRAWH2000651, BRAWH2000682, BRAWH2000686, BRAWH2000697, BRAWH2000752, BRAWH2000859, BRAWH2000866, BRAWH2000892, BRAWH2000901, BRAWH2000926, BRAWH2000984, BRAWH2001019, BRAWH2001037, BRAWH2001052, BRAWH2001129, BRAWH2001137, BRAWH2001159, BRAWH2001171, BRAWH2001203, BRAWH2001238, BRAWH2001239, BRAWH2001255, BRAWH2001355, BRAWH2001378, BRAWH2001395, BRAWH2001406, BRAWH2001412,
BRAWH2001418, BRAWH2001424, BRAWH2001436, BRAWH2001438, BRAWH2001439, BRAWH2001459, BRAWH2001461, BRAWH2001484, BRAWH2001492, BRAWH2001503, BRAWH2001519, BRAWH2001530, BRAWH2001535, BRAWH2001589; BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003135,
BRAWH2003240, BRAWH2003307, BRAWH2003355, BRAWH2003366, BRAWH2003662, BRAWH2003689, BRAWH2003693, BRAWH2003832, BRAWH2003948, BRAWH2003964, BRAWH2004044,.BRAWH2004068, BRAWH2004078, BRAWH2004136, BRAWH2004217, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006044, BRAWH2006083, BRAWH2006207, BRAWH2006301, BRAWH2006395, BRAWH2006450, BRAWH2006493, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570,
BRAWH2007591, BRAWH2007658, BRAWH2007664, BRAWH2007808, BRAWH2007825, BRAWH2007862, BRAWH2007890, BRAWH2008058, BRAWH2008255, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009227, BRAWH2009238, BRAWH2009261, BRAWH2009263, BRAWH2009298, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364, BRAWH2010536, BRAWH2010552, BRAWH2010584, BRAWH2010618, BRAWH2010619, BRAWH2010754,
BRAWH2010980, BRAWH2011066, BRAWH2011079, BRAWH2011096, BRAWH2011155, BRAWH2011166, BRAWH2011204, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871, BRAWH2013914, BRAWH2013941, BRAWH2013955, BRAWH2014053, BRAWH2014234, BRAWH2014379, BRAWH2014414, BRAWH2014473, BRAWH2014511, BRAWH2014662, BRAWH2014717,
BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016160, BRAWH2016221, BRAWH2016223, BRAWH2016305,. BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206, BRAWH2018267, BRAWH2018282, BRAWH2018317, BRAWH2018506, BRAWH2018514, BRAWH2018601, BRAWH2018745,
BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002513, BRAWH3002574, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003727, BRAWH3003801, BRAWH3003975, BRAWH3004222, BRAWH3004335, BRAWH3004350, BRAWH3004453, BRAWH3004530, BRAWH3004666,
BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011637, BRAWH3011685, BRAWH3011907, BRAWH3011929, BRAWH3012005,
BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021545, BRAWH3021574, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900,
BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3029806, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117,
BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923,
BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2008494, BRCAN2008528, BRCAN2008701-, BRCAN2008894, BRCAN2009156, BRCAN2009168,
BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016762, BRCAN2016814, BRCAN2017442, BRCAN2017905, BRCAN2018240, BRCAN2018269, BRCAN2018490, BRCAN2018667, BRCAN2018748, BRCAN2018935, BRCAN2019002,
BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028040, BRCAN2028319, BRCAN2028338, BRCAN2028355, BRCAN2028378, BRCAN2028460,
BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942, BRCOC2007034,
BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015944, BRCOC2016525, BRCOC2016661, BRCOC2017061, BRCOC2017652, BRCOC2017856, BRCOC2018775, BRCOC2019255, BRCOC2019549, BRCOC2019841, BRCOC2019896, BRCOC2019903,
BRCOC2019910, BRCOC2019934, BRCOC2020122, BRCOC2020142, BRHIP1000058, BRHIP1000072, BRHIP1000108, BRHIP1000129, BRHIP1000174, BRHIP1000258, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000553, BRHIP2000621, BRHIP2000691, BRHIP2000826, BRHIP2000920, BRHIP2001074, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828,
BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007305, BRHIP2007307, BRHIP2007616, BRHIP2007690, BRHIP2007741, BRHIP2007928, BRHIP2007969, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120,
BRHIP2011199, BRHIP2011508, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014228, BRHIP2014285, BRHIP2014291, BRHIP2014373, BRHIP2014386, BRHIP2014387, BRHIP2014954, BRHIP2015224, BRHIP2015245, BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385, BRHIP2017404, BRHIP2017542,
BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021858, BRHIP2021870, BRHIP2021929, BRHIP2022006, BRHIP2022054, BRHIP2022221, BRHIP2022228, BRHIP2022298, BRHIP2022326, BRHIP2022467, BRHIP2022708, BRHIP2022986,
BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024549, BRHIP2024789, BRHIP2024911, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283,
BRHIP2029393, BRHIP2029529, BRHIP2029643, BRHIP2029663, BRHIP3000060, BRHIP3000070, BRHIP3000081, BRHIP3000131, BRHIP3000240, BRHIP3000377, BRHIP3000457, BRHIP3000475, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004416, BRHIP3004710, BRHIP3004725, BRHIP3004774, BRHIP3004786,
BRHIP3004863, BRHIP3004968, BRHIP3005037, BRHIP3005137, BRHIP3005142, BRHIP3005231, BRHIP3005307, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007223, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427,
BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3012997, BRHIP3013078, BRHIP3013271, BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3017855, BRHIP3018278, BRHIP3018784, BRHIP3019643, BRHIP3019824, BRHIP3019880, BRHIP3019956, BRHIP3020046, BRHIP3020155, BRHIP3020182,
BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000498, BRSSN2000518, BRSSN2000561, BRSSN2000566, BRSSN2000684, BRSSN2000715,
BRSSN2000832, BRSSN2001213, BRSSN2001275, BRSSN2001342, BRSSN2001426, BRSSN2001503, BRSSN2001579, BRSSN2001758, BRSSN2001810, BRSSN2001869, BRSSN2002857, BRSSN2002859, BRSSN2003086, BRSSN2003093, BRSSN2003820, BRSSN2003841, BRSSN2003937, BRSSN2004142, BRSSN2004303, BRSSN2004304, BRSSN2004496, BRSSN2004657, BRSSN2004686, BRSSN2004710, BRSSN2004719, BRSSN2005847, BRSSN2006133, BRSSN2006390, BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009378, BRSSN2009389, BRSSN2009395, BRSSN2009627, BRSSN2009630, BRSSN2009702,
BRSSN2010019, BRSSN2010110, BRSSN2010587, BRSSN2010596, BRSSN2010830, BRSSN2011262, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014424, BRSSN2014513, BRSSN2014556, BRSSN2014573, BRSSN2014610, BRSSN2014636, BRSSN2014685, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757,
BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000367, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416,
BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013354, BRSTN2013502, BRSTN2013931, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016892, BRSTN2016918, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969,
BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005942, BRTHA2005956, BRTHA2006075, BRTHA2006146, BRTHA2006194, BRTHA2006232, BRTHA2006720,
BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562,
BRTHA2012714, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016267, BRTHA2016318, BRTHA2016496, BRTHA2016543, BRTHA2016552, BRTHA2016577, BRTHA2016765, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420,
BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264,
BRTHA2033320, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003074, BRTHA3003225, BRTHA3003339, BRTHA3003343, BRTHA3003417, BRTHA3003449, BRTHA3003474, BRTHA3003490, BRTHA3003704, BRTHA3004307, BRTHA3004432, BRTHA3004475, BRTHA3004502, BRTHA3004610, BRTHA3005046, BRTHA3005735,
BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547,
BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020314, BRTHA3020369, BRTHA3021569, BRTHA3021708, BRTHA3021786, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505,
BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733,
COLON2008105, COLON2009337, COLON2009499, CORDB1000140, CORDB2000061, CORDB2000127, CORDB2000541, CTONG1000009, CTONG1000020, CTONG1000022, CTONG1000040, CTONG1000056, CTONG1000062, CTONG1000086, CTONG1000087, CTONG1000088, CTONG1000093, CTONG1000097, CTONG1000113, CTONG1000137, CTONG1000165, CTONG1000175, CTONG1000180, CTONG1000181, CTONG1000218, CTONG1000220, CTONG1000277, CTONG1000283, CTONG1000288, CTONG1000302, CTONG1000467, CTONG1000476, CTONG1000488, CTONG1000508, CTONG1000540, CTONG2000031, CTONG2000034, CTONG2000042, CTONG2000201, CTONG2000218, CTONG2000279, CTONG2000303, CTONG2000330, CTONG2000395, CTONG2000411, CTONG2000465, CTONG2000469, CTONG2000480, CTONG2000561, CTONG2000589,
CTONG2000766, CTONG2000771, CTONG2000819, CTONG2000827, CTONG2000846, CTONG2000855, CTONG2000977, CTONG2001139, CTONG2001320, CTONG2001366, CTONG2001413, CTONG2001428, CTONG2001513, CTONG2001524, CTONG2001533, CTONG2001598, CTONG2001681, CTONG2001724, CTONG2001748, CTONG2001749, CTONG2001800, CTONG2001820, CTONG2001858, CTONG2001877, CTONG2001911, CTONG2001955, CTONG2002066, CTONG2002073, CTONG2002095, CTONG2002143, CTONG2002395, CTONG2002417, CTONG2002418, CTONG2002453, CTONG2002520, CTONG2002590, CTONG2002709, CTONG2002721, CTONG2002766, CTONG2002803, CTONG2002816, CTONG2002820, CTONG2002832, CTONG2002841, CTONG2002903, CTONG2002965, CTONG2002999, CTONG2003028, CTONG2003094, CTONG2003100,
CTONG2003115, CTONG2003189, CTONG2003245, CTONG2003293, CTONG2003348, CTONG2003350, CTONG2003372, CTONG2003375, CTONG2003427, CTONG2003517, CTONG2003524, CTONG2003599, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004423, CTONG2004454, CTONG2004487, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005278, CTONG2005290, CTONG2005374, CTONG2005399, CTONG2005468, CTONG2005567, CTONG2005585, CTONG2005615, CTONG2005635, CTONG2005775, CTONG2005795, CTONG2005890,
CTONG2005904, CTONG2005913, CTONG2006129, CTONG2006223, CTONG2006235, CTONG2006273, CTONG2006310, CTONG2006377, CTONG2006393, CTONG2006412, CTONG2006449, CTONG2006515, CTONG2006524, CTONG2006568, CTONG2006620, CTONG2006666, CTONG2006691, CTONG2006798, CTONG2006836, CTONG2006942, CTONG2006964, CTONG2007009, CTONG2007072, CTONG2007091, CTONG2007104, CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321,
CTONG2008343, CTONG2008398, CTONG2008402, CTONG2008405, CTONG2008466, CTONG2008518, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958,
CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010649, CTONG2010652, CTONG2010803, CTONG2010821, CTONG2011188, CTONG2011429, CTONG2011474, CTONG2011676, CTONG2011765, CTONG2011779, CTONG2011801, CTONG2011815, CTONG2011822, CTONG2011825, CTONG2011920, CTONG2011985, CTONG2012001, CTONG2012029, CTONG2012050, CTONG2012077, CTONG2012101, CTONG2012123, CTONG2012158, CTONG2012230, CTONG2012401, CTONG2012422, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758,
CTONG2012843, CTONG2012879, CTONG2012901, CTONG2012996, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013934, CTONG2013986, CTONG2014032, CTONG2014058, CTONG2014119, CTONG2014191, CTONG2014206, CTONG2014234, CTONG2014264, CTONG2014280, CTONG2014369, CTONG2014491, CTONG2014630, CTONG2014635, CTONG2014646, CTONG2014705, CTONG2014898, CTONG2014946, CTONG2014959, CTONG2014966, CTONG2014995, CTONG2015204, CTONG2015316, CTONG2015330, CTONG2015345, CTONG2015358, CTONG2015434, CTONG2015518, CTONG2015540, CTONG2015596, CTONG2015633, CTONG2015678, CTONG2015717,
CTONG2015804, CTONG2015866, CTONG2015914, CTONG2015953, CTONG2016056, CTONG2016217, CTONG2016355, CTONG2016408, CTONG2016499, CTONG2016505, CTONG2016559, CTONG2016658, CTONG2016775, CTONG2016797, CTONG2016824, CTONG2016846, CTONG2016904, CTONG2016931, CTONG2016942, CTONG2016953, CTONG2017021, CTONG2017094, CTONG2017292, CTONG2017409, CTONG2017429, CTONG2017458, CTONG2017500, CTONG2017604, CTONG2017650, CTONG2017798, CTONG2017804, CTONG2017814, CTONG2017910, CTONG2017939, CTONG2017989, CTONG2018062, CTONG2018069, CTONG2018147, CTONG2018217, CTONG2018279, CTONG2018334, CTONG2018343, CTONG2018379, CTONG2018413, CTONG2018483, CTONG2018629, CTONG2018632, CTONG2018652, CTONG2018653, CTONG2018655,
CTONG2018735, CTONG2018739, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019822, CTONG2019833, CTONG2020031, CTONG2020108, CTONG2020127, CTONG2020171, CTONG2020374, CTONG2020378, CTONG2020411, CTONG2020445, CTONG2020484, CTONG2020522, CTONG2020560, CTONG2020582, CTONG2020638, CTONG2020806, CTONG2020831, CTONG2020974, CTONG2021024, CTONG2021132, CTONG2021168, CTONG2022153, CTONG2022601, CTONG2022835, CTONG2023021, CTONG2023449, CTONG2023512, CTONG2024031, CTONG2024206, CTONG2024614, CTONG2024749, CTONG2025496, CTONG2025513, CTONG2025516,
CTONG2025610, CTONG2025900, CTONG2026513, CTONG2026770, CTONG2026920, CTONG2026987, CTONG2027150, CTONG2027263, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001123, CTONG3001420, CTONG3001501, CTONG3001560, CTONG3001605, CTONG3001746, CTONG3002020, CTONG3002127, CTONG3002370, CTONG3002412, CTONG3002433, CTONG3002518, CTONG3002552, CTONG3002588, CTONG3002674, CTONG3002728, CTONG3002835, CTONG3002909, CTONG3002947, CTONG3002983, CTONG3003165, CTONG3003179, CTONG3003483, CTONG3003553,
CTONG3003598, CTONG3003652, CTONG3003654, CTONG3003669, CTONG3003737, CTONG3003905, CTONG3004072, CTONG3004317, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009028, CTONG3009227, CTONG3009287, CTONG3009328, CTONG3009385, D3OST1000066, D3OST1000109, D3OST1000238, D3OST1000267, D3OST2000184,
D3OST2000618, D3OST2000654, D3OST2000734, D3OST2001328, D3OST2001669, D3OST2001670, D3OST2001788, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000031, D9OST2000278, D9OST2000440, D9OST2000869, D9OST2001319, D9OST2001433, D9OST2002397, D9OST2002673, D9OST2003106, D9OST2003108, D9OST2003137, D9OST2003397, D9OST2003580, D9OST2003594, D9OST2003762, D9OST2003791, D9OST2003989, D9OST2004018, D9OST2004073,
D9OST2004417, DFNES1000003, DFNES1000107, DFNES1000185, DFNES2000011, DFNES2000143, DFNES2000146, DFNES2000212, DFNES2000268, DFNES2000292, DFNES2000335, DFNES2000426, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2002966, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540, DFNES2005690, DFNES2005779, DFNES2005875, DFNES2006944, DFNES2007113,
DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000024, FCBBF1000025, FCBBF1000038, FCBBF1000053, FCBBF1000061, FCBBF1000063, FCBBF1000069, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305,
FCBBF1000314, FCBBF1000322, FCBBF1000349, FCBBF1000374, FCBBF1000376, FCBBF1000377, FCBBF1000395, FCBBF1000397, FCBBF1000425, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000748, FCBBF2000038, FCBBF2000087, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000576, FCBBF2000677, FCBBF2000678,
FCBBF2000685, FCBBF2000733, FCBBF2000782, FCBBF2000808, FCBBF2000815, FCBBF2000825, FCBBF2000885, FCBBF2000940, FCBBF2000951, FCBBF2001001, FCBBF2001088, FCBBF2001105, FCBBF2001116, FCBBF2001183, FCBBF2001188, FCBBF2001238, FCBBF2001243, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003293, FCBBF2003297, FCBBF2003395, FCBBF2003528, FCBBF2003543,
FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000120, FCBBF3000168, FCBBF3000175, FCBBF3000228, FCBBF3000229, FCBBF3000233, FCBBF3000269, FCBBF3000361, FCBBF3000367, FCBBF3000434,
FCBBF3000462, FCBBF3000473, FCBBF3000501, FCBBF3000518, FCBBF3000536, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390,
FCBBF3004409, FCBBF3004502, FCBBF3004715, FCBBF3004842, FCBBF3004847, FCBBF3004955, FCBBF3005160, FCBBF3005218, FCBBF3005444, FCBBF3005698, FCBBF3005729, FCBBF3006171, FCBBF3006249, FCBBF3006288, FCBBF3006399, FCBBF3006628, FCBBF3006821, FCBBF3007077, FCBBF3007150, FCBBF3007152, FCBBF3007242, FCBBF3007244, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008159, FCBBF3008251, FCBBF3008311, FCBBF3008362, FCBBF3008382, FCBBF3008475, FCBBF3008556, FCBBF3008644, FCBBF3008689, FCBBF3008748, FCBBF3008870, FCBBF3008944, FCBBF3009069, FCBBF3009101, FCBBF3009152,
FCBBF3009256, FCBBF3009317, FCBBF3009428, FCBBF3009464, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667, FCBBF3012842, FCBBF3012901, FCBBF3013041, FCBBF3013058, FCBBF3013205, FCBBF3013266, FCBBF3013307, FCBBF3013341,
FCBBF3013506, FCBBF3013623, FCBBF3013800, FCBBF3013846, FCBBF3014229, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017396, FCBBF3017531, FCBBF3017535, FCBBF3017681, FCBBF3017729, FCBBF3017873, FCBBF3017918, FCBBF3017974, FCBBF3018067, FCBBF3018173, FCBBF3018453, FCBBF3018591, FCBBF3018753, FCBBF3018826, FCBBF3018949, FCBBF3019085, FCBBF3019320, FCBBF3019437,
FCBBF3019455, FCBBF3019564, FCBBF3019569, FCBBF3019570, FCBBF3019663, FCBBF3019716, FCBBF3019784, FCBBF3019839, FCBBF3019867, FCBBF3019961, FCBBF3020030, FCBBF3020138, FCBBF3020140, FCBBF3020163, FCBBF3020232, FCBBF3021026, FCBBF3021191, FCBBF3021221, FCBBF3021501, FCBBF3021506, FCBBF3021524, FCBBF3021807, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024002, FCBBF3024096, FCBBF3024225, FCBBF3024266, FCBBF3024364, FCBBF3024623, FCBBF3024793, FCBBF3024911,
FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3025737, FCBBF3025905, FCBBF3026021, FCBBF3026048, FCBBF3026251, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000529,
FCBBF4000548, FCBBF5000041, FCBBF5000061, FCBBF5000165, FCBBF5000261, FCBBF5000325, FCBBF5000353, FCBBF5000384, FCBBF5000483, FCBBF5000495, FEBRA1000005, FEBRA1000008, FEBRA1000030, FEBRA1000057, FEBRA1000088, FEBRA1000138, FEBRA1000148, FEBRA1000149, FEBRA1000183, FEBRA1000188, FEBRA1000189, FEBRA2000007, FEBRA2000021, FEBRA2000035, FEBRA2000053, FEBRA2000126, FEBRA2000220, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000454, FEBRA2000462, FEBRA2000468, FEBRA2000491,
FEBRA2000504, FEBRA2000532, FEBRA2000536, FEBRA2000538, FEBRA2000575, FEBRA2000581, FEBRA2000656, FEBRA2000659, FEBRA2000680, FEBRA2000690, FEBRA2000727, FEBRA2000734, FEBRA2000757, FEBRA2000771, FEBRA2000772, FEBRA2000782, FEBRA2000790, FEBRA2000793, FEBRA2000803, FEBRA2000805, FEBRA2000809, FEBRA2000815, FEBRA2000856, FEBRA2000862, FEBRA2000877, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001334, FEBRA2001351,
FEBRA2001383, FEBRA2001438, FEBRA2001440, FEBRA2001487, FEBRA2001561, FEBRA2001584, FEBRA2001590, FEBRA2001591, FEBRA2001610, FEBRA2001620, FEBRA2001659, FEBRA2001698, FEBRA2001705, FEBRA2001706, FEBRA2001715, FEBRA2001745, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002399, FEBRA2002410, FEBRA2002429, FEBRA2002442, FEBRA2002456, FEBRA2002508, FEBRA2002525, FEBRA2002527, FEBRA2002552, FEBRA2002574, FEBRA2002601, FEBRA2002611, FEBRA2002628, FEBRA2002662, FEBRA2002682, FEBRA2002707, FEBRA2002727, FEBRA2002781, FEBRA2002783,
FEBRA2002792, FEBRA2002818, FEBRA2002858, FEBRA2002882, FEBRA2002897, FEBRA2002908, FEBRA2002931, FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003833, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004237, FEBRA2004268, FEBRA2004325, FEBRA2004329, FEBRA2004331,
FEBRA2004443, FEBRA2004485, FEBRA2004490, FEBRA2004538, FEBRA2004592, FEBRA2004620, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005701, FEBRA2005726, FEBRA2005752, FEBRA2005787, FEBRA2005995, FEBRA2005998, FEBRA2006055, FEBRA2006061, FEBRA2006112, FEBRA2006150, FEBRA2006165, FEBRA2006255, FEBRA2006315, FEBRA2006354, FEBRA2006357, FEBRA2006368, FEBRA2006370, FEBRA2006372, FEBRA2006379, FEBRA2006396, FEBRA2006409,
FEBRA2006418, FEBRA2006427, FEBRA2006476, FEBRA2006485, FEBRA2006519, FEBRA2006521, FEBRA2006553, FEBRA2006627, FEBRA2006664, FEBRA2006667, FEBRA2006726, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007620, FEBRA2007708, FEBRA2007714, FEBRA2007793, FEBRA2007818, FEBRA2007823, FEBRA2007880, FEBRA2007900, FEBRA2007931, FEBRA2007957, FEBRA2008009, FEBRA2008036, FEBRA2008086, FEBRA2008134, FEBRA2008135, FEBRA2008159,
FEBRA2008165, FEBRA2008172, FEBRA2008255, FEBRA2008265, FEBRA2008266, FEBRA2008282, FEBRA2008287, FEBRA2008311, FEBRA2008360, FEBRA2008459, FEBRA2008468, FEBRA2008481, FEBRA2008542, FEBRA2008660, FEBRA2008662, FEBRA2008681, FEBRA2008692, FEBRA2008741, FEBRA2008836, FEBRA2008859, FEBRA2008861, FEBRA2008866, FEBRA2008881, FEBRA2008928, FEBRA2008983, FEBRA2009016, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009419, FEBRA2009437, FEBRA2009478, FEBRA2009507, FEBRA2009514, FEBRA2009567, FEBRA2009588, FEBRA2009731,
FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572,
FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018746, FEBRA2019172, FEBRA2019242, FEBRA2019298, FEBRA2019389, FEBRA2019457, FEBRA2019519, FEBRA2019582, FEBRA2019637, FEBRA2019663, FEBRA2019690, FEBRA2019867, FEBRA2020406, FEBRA2020566, FEBRA2020582, FEBRA2020668, FEBRA2020801, FEBRA2020845, FEBRA2020886, FEBRA2021032, FEBRA2021134, FEBRA2021171, FEBRA2021339, FEBRA2021431, FEBRA2021497, FEBRA2021550, FEBRA2021571, FEBRA2021864,
FEBRA2021892, FEBRA2021908, FEBRA2022013, FEBRA2022148, FEBRA2022204, FEBRA2022255, FEBRA2022322, FEBRA2022504, FEBRA2022601, FEBRA2022696, FEBRA2022729, FEBRA2022787, FEBRA2022911, FEBRA2022956, FEBRA2022963, FEBRA2022972, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2025838, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297,
FEBRA2027352, FEBRA2027364, FEBRA2027609, FEBRA2028158, FEBRA2028222, FEBRA2028366, FEBRA2028457, FEBRA2028477, FEBRA2028503, FEBRA2028516, FEHRT2000325, FEKID1000018, FELIV1000101, FELIV1000137, FELIV1000164, FELNG2000241, HCASM1000021, HCASM1000050, HCASM1000061, HCASM1000115, HCASM1000130, HCASM2000016, HCASM2000138, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131,
HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000142, HCHON1000176, HCHON1000191, HCHON2000038, HCHON2000062, HCHON2000100, HCHON2000156, HCHON2000199, HCHON2000212, HCHON2000265, HCHON2000271, HCHON2000315, HCHON2000322, HCHON2000344, HCHON2000364, HCHON2000395, HCHON2000417, HCHON2000418, HCHON2000473, HCHON2000503, HCHON2000508, HCHON2000660, HCHON2000663, HCHON2000673, HCHON2000676, HCHON2000698, HCHON2000699, HCHON2000705, HCHON2000743, HCHON2000815, HCHON2000817, HCHON2000818, HCHON2000819, HCHON2000832, HCHON2000851, HCHON2000898, HCHON2000935,
HCHON2000942, HCHON2000956, HCHON2000994, HCHON2001050, HCHON2001084, HCHON2001099, HCHON2001108, HCHON2001110, HCHON2001116, HCHON2001161, HCHON2001214, HCHON2001233, HCHON2001269, HCHON2001434, HCHON2001450, HCHON2001453, HCHON2001497, HCHON2001519, HCHON2001523, HCHON2001535, HCHON2001546, HCHON2001548, HCHON2001577, HCHON2001598, HCHON2001604, HCHON2001607, HCHON2001646, HCHON2001665, HCHON2001712, HCHON2001768, HCHON2001801, HCHON2001853, HCHON2002123, HCHON2002194, HCHON2002241, HCHON2002247, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003659, HCHON2003676, HCHON2004007,
HCHON2004279, HCHON2004359, HCHON2004531, HCHON2004776, HCHON2004858, HCHON2005048, HCHON2005118, HCHON2005166, HCHON2005802, HCHON2005921, HCHON2005987, HCHON2006459, HCHON2006714, HCHON2006722, HCHON2006770, HCHON2006786, HCHON2006841, HCHON2006871, HCHON2007046, HCHON2007482, HCHON2007674, HCHON2007774, HCHON2007881, HCHON2008112, HCHON2008239, HCHON2008444, HCHON2008582, HCHON2008672, HCHON2008856, HCHON2008871, HCHON2008989, HCHON2009191, HCHON2009384, HCHON2009749, HCHON2009766, HCHON2009795, HCHON2010074, HCHON2010543, HEART1000088, HEART1000099, HEART1000102, HEART1000118, HEART1000139, HEART1000142, HEART1000151, HEART1000173, HEART2000024, HEART2000035, HEART2000099, HEART2000298,
HEART2000306, HEART2000309, HEART2000411, HEART2000418, HEART2000448, HEART2000492, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007031, HEART2007231, HEART2007443, HEART2007696, HEART2007767, HEART2008108, HEART2008111, HEART2008257,
HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000114, HHDPC1000118, HHDPC2000070, HHDPC2000102, HHDPC2000104, HHDPC2000258, HHDPC2000315, HHDPC2000455, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005742, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114,
HHDPC2009178, HHDPC2009208, HHDPC2009272, HHDPC2009528, HLUNG1000017, HLUNG1000024, HLUNG1000030, HLUNG1000034, HLUNG1000053, HLUNG1000076, HLUNG1000084, HLUNG1000091, HLUNG1000099, HLUNG1000104, HLUNG1000105, HLUNG1000110, HLUNG1000151, HLUNG1000169, HLUNG2000004, HLUNG2000014, HLUNG2000027, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000846, HLUNG2000870, HLUNG2000884, HLUNG2000926,
HLUNG2000955, HLUNG2000970, HLUNG2001013, HLUNG2001126, HLUNG2001144, HLUNG2001146, HLUNG2001214, HLUNG2001459,. HLUNG2001507, HLUNG2001633, HLUNG2001677, HLUNG2001712, HLUNG2001814, HLUNG2001996, HLUNG2002006, HLUNG2002050, HLUNG2002059, HLUNG2002085, HLUNG2002295, HLUNG2002303, HLUNG2002405, HLUNG2002429, HLUNG2002562, HLUNG2002659, HLUNG2002707, HLUNG2002811, HLUNG2002916, HLUNG2002942, HLUNG2002958, HLUNG2002982, HLUNG2003049, HLUNG2003061, HLUNG2003162, HLUNG2003246, HLUNG2003306, HLUNG2003335, HLUNG2003378, HLUNG2003497, HLUNG2003691, HLUNG2003710, HLUNG2003714, HLUNG2003716, HLUNG2003833, HLUNG2003872, HLUNG2003918, HLUNG2003991, HLUNG2004014, HLUNG2004037, HLUNG2004067, HLUNG2004154,
HLUNG2004170, HLUNG2004217, HLUNG2004273, HLUNG2004416, HLUNG2004521, HLUNG2004534, HLUNG2004684, HLUNG2004707, HLUNG2004774, HLUNG2004775, HLUNG2005012, HLUNG2005030, HLUNG2005045, HLUNG2005076, HLUNG2005133, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614, HLUNG2006671, HLUNG2006781, HLUNG2006852, HLUNG2006935, HLUNG2007041, HLUNG2007210, HLUNG2007219, HLUNG2007245, HLUNG2007433, HLUNG2007931, HLUNG2008066, HLUNG2008139, HLUNG2008153, HLUNG2008235, HLUNG2008247, HLUNG2008333, HLUNG2008348, HLUNG2008384, HLUNG2008396,
HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017262, HLUNG2017286, HLUNG2017307,
HLUNG2017350, HLUNG2017546, HLUNG2017806, HLUNG2018029, HLUNG2018282, HLUNG2018626, HLUNG2019058, HSYRA1000131, HSYRA1000148, HSYRA1000152, HSYRA2000044, HSYRA2000135, HSYRA2000137, HSYRA2000221, HSYRA2000237, HSYRA2000253, HSYRA2000371, HSYRA2000424, HSYRA2000507, HSYRA2000510, HSYRA2000605, HSYRA2000629, HSYRA2000706, HSYRA2000760, HSYRA2000787, HSYRA2000792, HSYRA2000828, HSYRA2000832, HSYRA2000927, HSYRA2001003, HSYRA2001105, HSYRA2001142, HSYRA2001153, HSYRA2001232, HSYRA2001255, HSYRA2001332, HSYRA2001353, HSYRA2001484, HSYRA2001552, HSYRA2001580, HSYRA2001615, HSYRA2001621, HSYRA2001631, HSYRA2001638, HSYRA2002103, HSYRA2002590, HSYRA2002741, HSYRA2003168, HSYRA2004550, HSYRA2004858,
HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000161, IMR321000165, IMR321000193, IMR321000207, IMR321000230, IMR321000266, IMR322000010, IMR322000107, IMR322000164, IMR322000379, IMR322000383, IMR322000418, IMR322000518, IMR322000555, IMR322000604, IMR322000609, IMR322000656, IMR322000672, IMR322000775, IMR322000791, IMR322000811, IMR322000819, IMR322000838, IMR322000859, IMR322000863, IMR322000917, IMR322000918, IMR322000919, IMR322000953, IMR322000984, IMR322001018, IMR322001167, IMR322001185, IMR322001245, IMR322001318, IMR322001322, IMR322001332,
IMR322001380, IMR322001452, IMR322001491, IMR322001534, IMR322001665, IMR322001670, IMR322001710, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, IMR322019070, KIDNE1000008, KIDNE1000012, KIDNE1000015, KIDNE1000028, KIDNE1000036, KIDNE1000064,
KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000394, KIDNE2000403, KIDNE2000422, KIDNE2000493, KIDNE2000510, KIDNE2000513, KIDNE2000517, KIDNE2000519, KIDNE2000555, KIDNE2000624, KIDNE2000665, KIDNE2000678, KIDNE2000717, KIDNE2000721, KIDNE2000722, KIDNE2000801, KIDNE2000832, KIDNE2000846, KIDNE2000867, KIDNE2000947, KIDNE2001117, KIDNE2001140, KIDNE2001269, KIDNE2001361, KIDNE2001636, KIDNE2001713, KIDNE2001802,
KIDNE2001840, KIDNE2001847, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002882, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003752, KIDNE2003764, KIDNE2003837, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305, KIDNE2004344, KIDNE2004394, KIDNE2004411,
KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004879, KIDNE2004981, KIDNE2004985, KIDNE2004999, KIDNE2005038, KIDNE2005042,. KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005676, KIDNE2005781, KIDNE2005798, KIDNE2005854, KIDNE2005908, KIDNE2005937, KIDNE2006030, KIDNE2006053, KIDNE2006062, KIDNE2006092, KIDNE2006145, KIDNE2006149, KIDNE2006210, KIDNE2006299, KIDNE2006334, KIDNE2006376, KIDNE2006378, KIDNE2006465, KIDNE2006580, KIDNE2006652, KIDNE2006687,
KIDNE2006733, KIDNE2006760, KIDNE2006775, KIDNE2006820, KIDNE2006880, KIDNE2007040, KIDNE2007077, KIDNE2007222, KIDNE2007356, KIDNE2007422, KIDNE2007569, KIDNE2007810, KIDNE2007944, KIDNE2007954, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2009019, KIDNE2009109, KIDNE2009180, KIDNE2009191, KIDNE2009332, KIDNE2009367, KIDNE2009426, KIDNE2009467, KIDNE2009490, KIDNE2009553, KIDNE2009605, KIDNE2009628,
KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013346,
KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539,
KIDNE2016570, KIDNE2016918, KIDNE2017007, KIDNE2017040, KIDNE2017052, KIDNE2017151, KIDNE2017153, KIDNE2017315, KIDNE2017332, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000079, LIVER1000082, LIVER1000099, LIVER1000104, LIVER1000111, LIVER1000126, LIVER1000139, LIVER1000155, LIVER1000165, LIVER1000175, LIVER1000224, LIVER1000230, LIVER1000278,
LIVER1000303, LIVER1000397, LIVER1000433, LIVER1000479, LIVER1000482, LIVER1000542, LIVER2000003, LIVER2000033, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001539, LIVER2001608, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003581, LIVER2003800, LIVER2003854, LIVER2004074, LIVER2004392,
LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706, LIVER2008945, LIVER2008998, LIVER2009110, LIVER2009118, LIVER2009554, LYMPB1000141, LYMPB1000158, LYMPB2000083, MAMGL1000017, MAMGL1000083, MAMGL1000096, MAMGL1000100, MAMGL1000132, MAMGL1000151, MAMGL1000182, MAMGL1000184, MESAN1000032, MESAN1000035, MESAN1000050, MESAN1000079,
MESAN1000080, MESAN1000101, MESAN1000121, MESAN1000147, MESAN1000180, MESAN2000023, MESAN2000026, MESAN2000067, MESAN2000092, MESAN2000167, MESAN2000291, MESAN2000337, MESAN2000434, MESAN2000457, MESAN2000462, MESAN2000572, MESAN2000620, MESAN2000711, MESAN2000815, MESAN2000894, MESAN2000909, MESAN2001450, MESAN2001627, MESAN2001770, MESAN2001979, MESAN2001980, MESAN2002113, MESAN2002122, MESAN2002147, MESAN2002186, MESAN2002424, MESAN2002519, MESAN2002687, MESAN2002709, MESAN2002724, MESAN2002790, MESAN2002844, MESAN2002978, MESAN2003035, MESAN2003037, MESAN2003039, MESAN2003058, MESAN2003101, MESAN2003190, MESAN2003322, MESAN2003490, MESAN2003529, MESAN2003622, MESAN2003646, MESAN2003662,
MESAN2003709, MESAN2003851, MESAN2004138, MESAN2004575, MESAN2005303, MESAN2005371, MESAN2005633, MESAN2005724, MESAN2005766, MESAN2005808, MESAN2005811, MESAN2005957, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009105, MESAN2009311, MESAN2009418, MESAN2009503, MESAN2009522, MESAN2010031, MESAN2010114, MESAN2010312, MESAN2010321, MESAN2010664, MESAN2010681, MESAN2011545, MESAN2011597, MESAN2011627,
MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, N1ESE2000698, NB9N41000011,
NB9N41000047, NB9N41000121, NB9N41000142, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2001931,
NHNPC2002565, NHNPC2002749, NOVAR1000091, NOVAR1000102, NOVAR2000136, NOVAR2000352, NOVAR2000412, NOVAR2000710, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000195, NT2NE2000222, NT2NE2000284, NT2NE2000299, NT2NE2000327, NT2NE2000353, NT2NE2000356, NT2NE2000369, NT2NE2000374, NT2NE2000384, NT2NE2000386, NT2NE2000390, NT2NE2000392, NT2NE2000455,
NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000809, NT2NE2000864, NT2NE2000909, NT2NE2000913, NT2NE2000951, NT2NE2000980, NT2NE2001005, NT2NE2001021, NT2NE2001040, NT2NE2001048, NT2NE2001142, NT2NE2001176, NT2NE2001180, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001435, NT2NE2001442, NT2NE2001524, NT2NE2001530, NT2NE2001545, NT2NE2001598, NT2NE2001623, NT2NE2001626, NT2NE2001634, NT2NE2001648, NT2NE2001655,
NT2NE2001660, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003569, NT2NE2003571, NT2NE2003705, NT2NE2003752, NT2NE2003887, NT2NE2003921, NT2NE2004255, NT2NE2004378, NT2NE2004490, NT2NE2004606, NT2NE2004716, NT2NE2004740, NT2NE2004787, NT2NE2004916, NT2NE2005035, NT2NE2005223, NT2NE2005276, NT2NE2005317,
NT2NE2005323, NT2NE2005358, NT2NE2005395, NT2NE2005419, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006659, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727,
NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847,
NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721,
NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019091, NT2NE2019099, NT2RI1000016, NT2RI1000035, NT2RI1000048, NT2RI1000127, NT2RI1000164, NT2RI1000172, NT2RI2000004, NT2RI2000007, NT2RI2000116, NT2RI2000117, NT2RI2000128, NT2RI2000133, NT2RI2000167, NT2RI2000173, NT2RI2000187, NT2RI2000270, NT2RI2000276, NT2RI2000294, NT2RI2000313, NT2RI2000341, NT2RI2000344, NT2RI2000348, NT2RI2000421, NT2RI2000480, NT2RI2000575, NT2RI2000578, NT2RI2000588, NT2RI2000597, NT2RI2000669, NT2RI2000688, NT2RI2000704, NT2RI2000738, NT2RI2000775, NT2RI2000822,
NT2RI2000829, NT2RI2000841, NT2RI2000865, NT2RI2000932, NT2RI2000974, NT2RI2000987, NT2RI2001010, NT2RI2001017, NT2RI2001030, NT2RI2001083, NT2RI2001167, NT2RI2001178, NT2RI2001235, NT2RI2001244, NT2RI2001307, NT2RI2001342, NT2RI2001410, NT2RI2001425, NT2RI2001449, NT2RI2001450, NT2RI2001474, NT2RI2001519, NT2RI2001595, NT2RI2001624, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002540,
NT2RI2002564, NT2RI2002585, NT2RI2002592, NT2RI2002602, NT2RI2002654, NT2RI2002699, NT2RI2002729, NT2RI2002802, NT2RI2002819, NT2RI2002822, NT2RI2002852, NT2RI2002865, NT2RI2002923, NT2RI2002926, NT2RI2002970, NT2RI2003011, NT2RI200301.9, NT2RI2003051, NT2RI2003067, NT2RI2003184, NT2RI2003222, NT2RI2003301, NT2RI2003317, NT2RI2003344, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003678, NT2RI2003751, NT2RI2003884, NT2RI2003973, NT2RI2003993, NT2RI2004078, NT2RI2004079, NT2RI2004093, NT2RI2004136, NT2RI2004157, NT2RI2004190, NT2RI2004209, NT2RI2004230, NT2RI2004284, NT2RI2004290,
NT2RI2004304, NT2RI2004312, NT2RI2004381, NT2RI2004398, NT2RI2004410, NT2RI2004442, NT2RI2004468, NT2RI2004535, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005116, NT2RI2005130, NT2RI2005166, NT2RI2005246, NT2RI2005315, NT2RI2005335, NT2RI2005353, NT2RI2005358, NT2RI2005368, NT2RI2005382, NT2RI2005473, NT2RI2005520, NT2RI2005564, NT2RI2005579, NT2RI2005621, NT2RI2005628, NT2RI2005647, NT2RI2005670, NT2RI2005710, NT2RI2005772, NT2RI2005811, NT2RI2005814, NT2RI2005816, NT2RI2005818, NT2RI2005851, NT2RI2005966, NT2RI2006071,
NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006943, NT2RI2006973, NT2RI2007046, NT2RI2007048, NT2RI2007116, NT2RI2007148, NT2RI2007214, NT2RI2007277, NT2RI2007303, NT2RI2007334, NT2RI2007377, NT2RI2007386, NT2RI2007406, NT2RI2007439, NT2RI2007445, NT2RI2007468, NT2RI2007469, NT2RI2007507, NT2RI2007572, NT2RI2007593, NT2RI2007729, NT2RI2007751,
NT2RI2007754, NT2RI2007853, NT2RI2007877, NT2RI2007879, NT2RI2007891, NT2RI2007956, NT2RI2007965, NT2RI2008007, NT2RI2008050, NT2RI2008141, NT2RI2008188, NT2RI2008233, NT2RI2008396, NT2RI2008455, NT2RI2008502, NT2RI2008566, NT2RI2008598, NT2RI2008656, NT2RI2008714, NT2RI2008724, NT2RI2008801, NT2RI2008808, NT2RI2008841, NT2RI2008942, NT2RI2008952, NT2RI2009005, NT2RI2009066, NT2RI2009101, NT2RI2009103, NT2RI2009144, NT2RI2009151, NT2RI2009194, NT2RI2009215, NT2RI2009233, NT2RI2009259, NT2RI2009289, NT2RI2009301, NT2RI2009402, NT2RI2009406, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803,
NT2RI2012350, NT2RI2012659, NT2RI2012990, NT2RI2013345, NT2RI2013357, NT2RI2013373, NT2RI2014247, NT2RI2014551, NT2RI2014683, NT2RI2014733, NT2RI2015239, NT2RI2015342, NT2RI2016128, NT2RI2016134, NT2RI2018311, NT2RI2018363, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303, NT2RI2023671, NT2RI2024008, NT2RI2024313, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975, NT2RI2028213, NT2RI2028222, NT2RI2028537,
NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005724, NT2RI3005861, NT2RI3005923, NT2RI3005928, NT2RI3006132, NT2RI3006171, NT2RI3006284, NT2RI3006340, NT2RI3006376, NT2RI3006666, NT2RI3006673, NT2RI3006796, NT2RI3007065, NT2RI3007095, NT2RI3007158, NT2RI3007167, NT2RI3007213,
NT2RI3007291, NT2RI3007443, NT2RI3007543, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000017, NT2RP6000032, NT2RP6000033, NT2RP6000039, NT2RP6000059, NT2RP6000060, NT2RP6000077, NT2RP6000085, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000069, NT2RP7000086, NT2RP7000109, NT2RP7000173, NT2RP7000238, NT2RP7000259, NT2RP7000311, NT2RP7000359, NT2RP7000364, NT2RP7000380, NT2RP7000391, NT2RP7000425,
NT2RP7000477, NT2RP7000579, NT2RP7000586, NT2RP7000600, NT2RP7000624, NT2RP7000812, NT2RP7000926, NT2RP7001074, NT2RP7001080, NT2RP7001156, NT2RP7001231, NT2RP7001319, NT2RP7001335, NT2RP7001364, NT2RP7001475, NT2RP7001532, NT2RP7001591, NT2RP7001602, NT2RP7001678, NT2RP7001856, NT2RP7001962, NT2RP7002028, NT2RP7002064, NT2RP7002151, NT2RP7002163, NT2RP7002243, NT2RP7002376, NT2RP7002450, NT2RP7002478, NT2RP7002554, NT2RP7002594, NT2RP7002603, NT2RP7002650, NT2RP7002738, NT2RP7002779, NT2RP7002802, NT2RP7002841, NT2RP7002875, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003203,
NT2RP7003319, NT2RP7003439, NT2RP7003511, NT2RP7003629, NT2RP7003632, NT2RP7003647, NT2RP7003680, NT2RP7003724, NT2RP7003960, NT2RP7004027, NT2RP7004080, NT2RP7004114, NT2RP7004123, NT2RP7004196, NT2RP7004204, NT2RP7004233, NT2RP7004260, NT2RP7004352, NT2RP7004358, NT2RP7004396, NT2RP7004428, NT2RP7004481, NT2RP7004559, NT2RP7004641, NT2RP7004656, NT2RP7004687, NT2RP7004722, NT2RP7004728, NT2RP7004766, NT2RP7004790, NT2RP7004911, NT2RP7004915, NT2RP7004961, NT2RP7004964, NT2RP7004975, NT2RP7005118, NT2RP7005205, NT2RP7005219, NT2RP7005323, NT2RP7005468, NT2RP7005493, NT2RP7005529, NT2RP7005629, NT2RP7005631, NT2RP7005669, NT2RP7005675, NT2RP7005750, NT2RP7005846, NT2RP7006033, NT2RP7006160,
NT2RP7006162, NT2RP7006188, NT2RP7006223, NT2RP7006263, NT2RP7006296, NT2RP7006304, NT2RP7006374, NT2RP7006395, NT2RP7006408, NT2RP7006457, NT2RP7006490, NT2RP7006547, NT2RP7006601, NT2RP7006619, NT2RP7006621, NT2RP7006636, NT2RP7006701, NT2RP7006853, NT2RP7006886, NT2RP7006887, NT2RP7006986, NT2RP7006995, NT2RP7007114, NT2RP7007177, NT2RP7007221, NT2RP7007226, NT2RP7007252, NT2RP7007269, NT2RP7007310, NT2RP7007359, NT2RP7007381, NT2RP7007406, NT2RP7007480, NT2RP7007504, NT2RP7007537, NT2RP7007580, NT2RP7007594, NT2RP7007610, NT2RP7007617, NT2RP7007643, NT2RP7007766, NT2RP7007925, NT2RP7007930, NT2RP7008013, NT2RP7008015, NT2RP7008133, NT2RP7008137, NT2RP7008142, NT2RP7008144, NT2RP7008161,
NT2RP7008190, NT2RP7008315, NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009087, NT2RP7009108, NT2RP7009147, NT2RP7009149, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109,. NT2RP7010128, NT2RP7010235, NT2RP7010275,
NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7013002, NT2RP7013374, NT2RP7013499, NT2RP7013573, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971,
NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407; NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000087, NTONG1000123, NTONG1000130, NTONG1000161,
NTONG1000172, NTONG1000213, NTONG1000214, NTONG1000247, NTONG1000264, NTONG2000040, NTONG2000107, NTONG2000231, NTONG2000334, NTONG2000363, NTONG2000413, NTONG2000492, NTONG2000499, NTONG2000531, NTONG2000583, NTONG2000800, NTONG2000876, NTONG2000878, NTONG2000966, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001550, NTONG2001587, NTONG2001619, NTONG2001642, NTONG2001657, NTONG2001676, NTONG2001762, NTONG2002278, NTONG2002807, NTONG2002948, NTONG2002970, NTONG2002985, NTONG2003158, NTONG2003210, NTONG2003316, NTONG2003409, NTONG2003805, NTONG2003839, NTONG2003852, NTONG2003897,
NTONG2004095, NTONG2004308, NTONG2004521, NTONG2004614, NTONG2004690, NTONG2004829, NTONG2004918, NTONG2004991, NTONG2005062, NTONG2005086, NTONG2005137, NTONG2005265, NTONG2005277, NTONG2005363, NTONG2005373, NTONG2005391, NTONG2005480, NTONG2005497, NTONG2005577, NTONG2005657, NTONG2005801, NTONG2005897, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008672, NTONG2008862, NTONG2008939,
NTONG2008944, NTONG2009060, NTONG2009068, NTONG2009229, NTONG2009233, NTONG2009576, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000118, OCBBF1000119, OCBBF1000122, OCBBF1000130, OCBBF1000138, OCBBF1000145, OCBBF1000153, OCBBF1000175, OCBBF1000185, OCBBF1000188, OCBBF1000192, OCBBF1000254, OCBBF1000315, OCBBF2000015, OCBBF2000126, OCBBF2000178, OCBBF2000231, OCBBF2000277, OCBBF2000323, OCBBF2000452, OCBBF2000677, OCBBF2000703, OCBBF2000719, OCBBF2000824, OCBBF2000831, OCBBF2000846, OCBBF2000904, OCBBF2000986, OCBBF2000998, OCBBF2001075, OCBBF2001101, OCBBF2001124, OCBBF2001140, OCBBF2001166,
OCBBF2001176, OCBBF2001186, OCBBF2001210, OCBBF2001252, OCBBF2001307, OCBBF2001323, OCBBF2001389, OCBBF2001402, OCBBF2001408, OCBBF2001416, OCBBF2001473, OCBBF2001492, OCBBF2001494, OCBBF2001527, OCBBF2001528, OCBBF2001586, OCBBF2001639, OCBBF2001681, OCBBF2001687, OCBBF2001706, OCBBF2001749, OCBBF2001910, OCBBF2001931, OCBBF2001961, OCBBF2001983, OCBBF2002015, OCBBF2002083, OCBBF2002124, OCBBF2002161, OCBBF2002290, OCBBF2002357, OCBBF2002615, OCBBF2002626, OCBBF2002656, OCBBF2002663, OCBBF2002805, OCBBF2002865, OCBBF2002920, OCBBF2002980, OCBBF2003028, OCBBF2003052, OCBBF2003091, OCBBF2003246, OCBBF2003327, OCBBF2003518, OCBBF2003543, OCBBF2003593, OCBBF2003744, OCBBF2003819, OCBBF2003925,
OCBBF2004038, OCBBF2004147, OCBBF2004168, OCBBF2004259, OCBBF2004273, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639,
OCBBF2006764, OCBBF2006972, OCBBF2006987, OCBBF2007028, OCBBF2007039, OCBBF2007051, OCBBF2007068, OCBBF2007114, OCBBF2007121, OCBBF2007184, OCBBF2007194, OCBBF2007196, OCBBF2007224, OCBBF2007238, OCBBF2007414, OCBBF2007428, OCBBF2007478, OCBBF2007485, OCBBF2007520, OCBBF2007555, OCBBF2007610, OCBBF2007756, OCBBF2007762, OCBBF2007829, OCBBF2007892, OCBBF2007931, OCBBF2007946, OCBBF2008041, OCBBF2008116, OCBBF2008138, OCBBF2008144, OCBBF2008283, OCBBF2008330, OCBBF2008340, OCBBF2008366, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814,
OCBBF2008822, OCBBF2009095, OCBBF2009115, OCBBF2009242, OCBBF2009301, OCBBF2009352, OCBBF2009391, OCBBF2009424, OCBBF2009536, OCBBF2009571, OCBBF2009583, OCBBF2009603, OCBBF2009772, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010819, OCBBF2010843, OCBBF2010858, OCBBF2010863, OCBBF2010871, OCBBF2010931, OCBBF2010945, OCBBF2010978, OCBBF2010989, OCBBF2011021, OCBBF2011073, OCBBF2011137, OCBBF2011160, OCBBF2011177, OCBBF2011228, OCBBF2011232, OCBBF2011283, OCBBF2011311, OCBBF2011625, OCBBF2011669, OCBBF2011685, OCBBF2011706, OCBBF2011722,
OCBBF2011759, OCBBF2011767, OCBBF2011855, OCBBF2011872, OCBBF2011887; OCBBF2011897, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012821, OCBBF2012881, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013481, OCBBF2013507, OCBBF2013721, OCBBF2013789, OCBBF2013843, OCBBF2013883, OCBBF2013926, OCBBF2014052, OCBBF2014064, OCBBF2014089, OCBBF2014322, OCBBF2014386, OCBBF2014576,
OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503, OCBBF2015506, OCBBF2015599, OCBBF2015645, OCBBF2015659, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2017035, OCBBF2017055, OCBBF2017069, OCBBF2017306, OCBBF2017325, OCBBF2017458, OCBBF2017489, OCBBF2017516, OCBBF2017665, OCBBF2017754, OCBBF2017815, OCBBF2017882, OCBBF2017888, OCBBF2017893, OCBBF2017920, OCBBF2018012, OCBBF2018014, OCBBF2018167, OCBBF2018206,
OCBBF2018229, OCBBF2018234, OCBBF2018362, OCBBF2018380, OCBBF2018563, OCBBF2018581, OCBBF2018618, OCBBF2018663, OCBBF2018687, OCBBF2018827, OCBBF2018828, OCBBF2018873, OCBBF2018934, OCBBF2018956, OCBBF2019108, OCBBF2019195, OCBBF2019327, OCBBF2019684, OCBBF2019761, OCBBF2019823, OCBBF2019950, OCBBF2020048, OCBBF2020318, OCBBF2020343, OCBBF2020453, OCBBF2020741, OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2024850, OCBBF2025028, OCBBF2025115, OCBBF2025198,
OCBBF2025451, OCBBF2025458, OCBBF2025527, OCBBF2025631; OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413,
OCBBF2033869, OCBBF2033948, OCBBF2034333, OCBBF2034529, OCBBF2034637, OCBBF2034906, OCBBF2035110, OCBBF2035214, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314,
OCBBF3004487, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091, PANCR1000185, PEBLM1000024, PEBLM1000029, PEBLM1000062, PEBLM1000066, PEBLM1000068, PEBLM1000071, PEBLM1000083, PEBLM1000144, PEBLM1000147, PEBLM1000174, PEBLM1000180, PEBLM2000030, PEBLM2000112, PEBLM2000147, PEBLM2000170, PEBLM2000180, PEBLM2000213, PEBLM2000248, PEBLM2000267, PEBLM2000270, PEBLM2000308, PEBLM2000321, PEBLM2000326, PEBLM2000333, PEBLM2000338, PEBLM2000502, PEBLM2001312, PEBLM2001465, PEBLM2001488,
PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004452, PEBLM2004497, PEBLM2004733, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007437, PEBLM2007447, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387,
PERIC2000422, PERIC2000473, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086,
PERIC2009329, PERIC2009566, PLACE5000001, PLACE5000037, PLACE5000058, PLACE5000067, PLACE5000068, PLACE5000070, PLACE5000080, PLACE5000105, PLACE5000115, PLACE5000116, PLACE5000129, PLACE5000139, PLACE5000153, PLACE5000159, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000145, PLACE6000191, PLACE6000205, PLACE6000231, PLACE6000263, PLACE6000296, PLACE6000314, PLACE6000325, PLACE6000348, PLACE6000424, PLACE6000426, PLACE6000429, PLACE6000450, PLACE6000523, PLACE6000550, PLACE6000552, PLACE6000555, PLACE6000619,
PLACE6000953, PLACE6001118, PLACE6001153, PLACE6001185, PLACE6001262, PLACE6001281, PLACE6001294, PLACE6001536, PLACE6001557, PLACE6001712, PLACE6001740, PLACE6001823, PLACE6001923, PLACE6001925, PLACE6001933, PLACE6002016, PLACE6002102, PLACE6002157, PLACE6002312, PLACE6002323, PLACE6002345, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003218, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6004101, PLACE6004219, PLACE6004336, PLACE6004368,
PLACE6004380, PLACE6004396, PLACE6004397, PLACE6004454, PLACE6004491, PLACE6004663, PLACE6004687, PLACE6004738, PLACE6004931, PLACE6004966, PLACE6004993, PLACE6005007, PLACE6005029, PLACE6005108, PLACE6005231, PLACE6005234, PLACE6005283, PLACE6005294, PLACE6005328, PLACE6005351, PLACE6005423, PLACE6005482, PLACE6005487, PLACE6005535, PLACE6005546, PLACE6005559, PLACE6005579, PLACE6005691, PLACE6005786, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309,
PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008824, PLACE6008989, PLACE6009006, PLACE6009008, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011057, PLACE6011102, PLACE6011156,
PLACE6011211, PLACE6011260, PLACE6011334, PLACE6011627, PLACE6011774, PLACE6011881,PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6012942, PLACE6013222, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016383, PLACE6016984, PLACE6017002, PLACE6017431,
PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6018938, PLACE6019195, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169,
PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000145, PROST1000152, PROST1000167, PROST1000184, PROST1000217, PROST1000220, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000485, PROST1000493, PROST1000512, PROST1000527, PROST1000528, PROST1000536,
PROST1000559, PROST1000575, PROST2000036, PROST2000053, PROST2000067, PROST2000079, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000284, PROST2000325, PROST2000337, PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001213, PROST2001414, PROST2001415, PROST2001465, PROST2001521, PROST2001540, PROST2001599, PROST2001611, PROST2001636, PROST2001796, PROST2001805, PROST2001823,
PROST2001899, PROST2001997, PROST2001998, PROST2002101, PROST2002162, PROST2002212, PROST2002227, PROST2002338, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003396, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142,
PROST2004146, PROST2004161, PROST2004251, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093, PROST2005121, PROST2005131, PROST2005228, PROST2005285, PROST2005426, PROST2005466, PROST2005604, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006060, PROST2006196, PROST2006201, PROST2006260, PROST2006343, PROST2006450, PROST2006491, PROST2006510, PROST2006536, PROST2006579, PROST2006688, PROST2006737, PROST2006766, PROST2006782, PROST2006988,
PROST2007122, PROST2007200, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988, PROST2008079, PROST2008088, PROST2008243, PROST2008245, PROST2008268, PROST2008271, PROST2008447, PROST2008468, PROST2008472, PROST2008489, PROST2008516, PROST2008724, PROST2008770, PROST2008835, PROST2008993, PROST2009022, PROST2009208, PROST2009222, PROST2009254, PROST2009273, PROST2009347, PROST2009365, PROST2009388, PROST2009400, PROST2009470, PROST2009483, PROST2009571, PROST2009731, PROST2009736, PROST2009795, PROST2009901, PROST2009909,
PROST2010046, PROST2010219, PROST2010250, PROST2010318, PROST2010326, PROST2010382, PROST2010400, PROST2010579, PROST2010606, PROST2010782, PROST2010885, PROST2011012, PROST2011038, PROST2011105, PROST2011297, PROST2011410, PROST2011439, PROST2011482, PROST2011577, PROST2011631, PROST2011660, PROST2012005, PROST2012007, PROST2012016, PROST2012088, PROST2012140, PROST2012157, PROST2012190, PROST2012249, PROST2012353, PROST2012400, PROST2012448, PROST2012504, PROST2012542, PROST2012550, PROST2012740, PROST2012888, PROST2012890, PROST2013032, PROST2013121, PROST2013260, PROST2013327, PROST2013531, PROST2013605, PROST2013627, PROST2013873, PROST2013880, PROST2014422, PROST2014585, PROST2014601,
PROST2014659, PROST2014916, PROST2014925, PROST2015124, PROST2015137, PROST2015198, PROST2015243, PROST2015246, PROST2015251, PROST2015287, PROST2015332, PROST2015457, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016918, PROST2017098, PROST2017128, PROST2017203, PROST2017367, PROST2017413, PROST2017441, PROST2017529, PROST2017578, PROST2017612, PROST2017617, PROST2017700, PROST2017701,
PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000057, PUAEN1000081, PUAEN1000085, PUAEN1000087, PUAEN1000121, PUAEN1000147, PUAEN1000161, PUAEN1000175, PUAEN1000239, PUAEN1000275, PUAEN1000276, PUAEN1000288, PUAEN2000080, PUAEN2000152, PUAEN2000175, PUAEN2000247, PUAEN2000312, PUAEN2000374, PUAEN2000394, PUAEN2000497, PUAEN2000594, PUAEN2000684, PUAEN2000942, PUAEN2001260, PUAEN2001345, PUAEN2001526, PUAEN2001586, PUAEN2001882, PUAEN2002473,
PUAEN2002489, PUAEN2002552, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005588, PUAEN2005930, PUAEN2006029, PUAEN2006328, PUAEN2006335, PUAEN2006578, PUAEN2006639, PUAEN2006761, PUAEN2007044, PUAEN2007350, PUAEN2007785, PUAEN2007898, PUAEN2008123, PUAEN2008228, PUAEN2008314, PUAEN2008515, PUAEN2008815, PUAEN2008939, PUAEN2008980, PUAEN2008985, PUAEN2009174, PUAEN2009348, PUAEN2009534, PUAEN2009655, PUAEN2009795, PUAEN2009852, PUAEN2010346, PUAEN2010824, RECTM1000051, RECTM1000141, RECTM2000220, RECTM2000349,
RECTM2000433, RECTM2001307, RECTM2001347, RECTM2001519, RECTM2001666, RECTM2001691, RECTM2001749, RECTM2002172, RECTM2002602, RECTM2002632, SYNOV1000049, SYNOV1000118, SYNOV1000128, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300, SYNOV2001356, SYNOV2001374, SYNOV2001390, SYNOV2001451, SYNOV2001457, SYNOV2001468, SYNOV2001581, SYNOV2001660, SYNOV2003326,
SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001153, SYNOV4001224, SYNOV4001326, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981, SYNOV4004184, SYNOV4004741,. SYNOV4004823,
SYNOV4004914, SYNOV4005256, SYNOV4005570, SYNOV4005739, SYNOV4005889, SYNOV4005981, SYNOV4005989, SYNOV4006256, SYNOV4006542, SYNOV4007012, SYNOV4007215, SYNOV4007360, SYNOV4007430, SYNOV4007521, SYNOV4007553, SYNOV4007711, SYNOV4008245, SYNOV4008336, SYNOV4009129, SYNOV4009139, SYNOV4009298, T1ESE2000116, T1ESE2000609, T1ESE2000904, T1ESE2002665, TBAES2000004, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543,
TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000613, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2001953, TESOP2002005, TESOP2002110, TESOP2002150,
TESOP2002273, TESOP2002451, TESOP2002489, TESOP2002539, TESOP2002545, TESOP2002804, TESOP2002950, TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006704, TESOP2006746, TESOP2006865, TESOP2006893, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018,
TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018, TESTI2000044, TESTI2000100, TESTI2000117, TESTI2000120, TESTI2000147, TESTI2000154, TESTI2000172, TESTI2000177, TESTI2000179,
TESTI2000207, TESTI2000208, TESTI2000221, TESTI2000238, TESTI2000253, TESTI2000272, TESTI2000278, TESTI2000349, TESTI2000356, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000784, TESTI2000808, TESTI2000819, TESTI2000830, TESTI2000840, TESTI2000849, TESTI2000850, TESTI2000874, TESTI2000883, TESTI2000909, TESTI2000951, TESTI2000970, TESTI2000981, TESTI2001008, TESTI2001049, TESTI2001082,
TESTI2001120, TESTI2001134, TESTI2001141, TESTI2001180, TESTI2001208, TESTI2001229, TESTI2001236, TESTI2001237, TESTI2001269, TESTI2001306, TESTI2001313, TESTI2001330, TESTI2001345, TESTI2001352, TESTI2001364, TESTI2001412, TESTI2001420, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879, TESTI2001894, TESTI2001898, TESTI2001915, TESTI2001950, TESTI2001959, TESTI2001968, TESTI2001985,
TESTI2001991, TESTI2002002, TESTI2002012, TESTI2002036, TESTI2002048, TESTI2002057, TESTI2002081, TESTI2002090, TESTI2002105, TESTI2002149, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002365, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002698, TESTI2002700, TESTI2002707, TESTI2002724, TESTI2002729, TESTI2002752, TESTI2002753, TESTI2002789, TESTI2002802, TESTI2002806,
TESTI2002840, TESTI2002866, TESTI2002877, TESTI2002910, TESTI2002912, TESTI2002928, TESTI2002937, TESTI2002965, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003413, TESTI2003418,
TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004031, TESTI2004073, TESTI2004080, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229,
TESTI2004243, TESTI2004255, TESTI2004287; TESTI2004295, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005025, TESTI2005040, TESTI2005060, TESTI2005072, TESTI2005085, TESTI2005112, TESTI2005120,
TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005731, TESTI2005739, TESTI2005742, TESTI2005767, TESTI2005775, TESTI2005784, TESTI2005788, TESTI2005827, TESTI2005835, TESTI2005860, TESTI2005892, TESTI2005908, TESTI2005911, TESTI2005933, TESTI2005937, TESTI2005948, TESTI2005979,
TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006111, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006360, TESTI2006409, TESTI2006425, TESTI2006437, TESTI2006453, TESTI2006457, TESTI2006460, TESTI2006465, TESTI2006483, TESTI2006499, TESTI2006523, TESTI2006543, TESTI2006565, TESTI2006572, TESTI2006617, TESTI2006624, TESTI2006628, TESTI2006633, TESTI2006643, TESTI2006648,
TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007211, TESTI2007220, TESTI2007234, TESTI2007311, TESTI2007320, TESTI2007346, TESTI2007358, TESTI2007402, TESTI2007405, TESTI2007452, TESTI2007464, TESTI2007466, TESTI2007480, TESTI2007490, TESTI2007502, TESTI2007524, TESTI2007533, TESTI2007576, TESTI2007613, TESTI2007657, TESTI2007685, TESTI2007692,
TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621, TESTI2008636, TESTI2008657, TESTI2008684, TESTI2008715, TESTI2008762, TESTI2008774, TESTI2008786,
TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390,. TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009520, TESTI2009544, TESTI2009545, TESTI2009551, TESTI2009577, TESTI2009585, TESTI2009588, TESTI2009683, TESTI2009727, TESTI2009739, TESTI2009812, TESTI2009835, TESTI2009853, TESTI2009935, TESTI2009977, TESTI2009987, TESTI2010009, TESTI2010154, TESTI2010180,
TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011612, TESTI2011665, TESTI2011680, TESTI2011683, TESTI2011750, TESTI2011840, TESTI2011846, TESTI2011915, TESTI2011928, TESTI2011971, TESTI2012019, TESTI2012050, TESTI2012090, TESTI2012095, TESTI2012152, TESTI2012155,
TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610,
TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014780, TESTI2014800, TESTI2014838, TESTI2014843, TESTI2014979, TESTI2014988, TESTI2015042, TESTI2015105, TESTI2015180, TESTI2015213, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752,
TESTI2015830, TESTI2015914, TESTI2015938, TESTI2015947, TESTI2015962, TESTI2015987, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582,
TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2019042, TESTI2019077, TESTI2019228, TESTI2019257, TESTI2019280, TESTI2019282, TESTI2019308, TESTI2019336, TESTI2019352, TESTI2019406, TESTI2019430, TESTI2019455,
TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020871, TESTI2020905, TESTI2020906, TESTI2020946, TESTI2020956, TESTI2020981, TESTI2020999, TESTI2021003, TESTI2021038, TESTI2021057, TESTI2021077, TESTI2021112, TESTI2021114, TESTI2021116, TESTI2021118,
TESTI2021122, TESTI2021124, TESTI2021138, TESTI2021297, TESTI2021315, TESTI2021358, TESTI2021425, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194,
TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024936, TESTI2024978, TESTI2024999, TESTI2025006, TESTI2025013, TESTI2025022, TESTI2025063, TESTI2025117, TESTI2025144, TESTI2025161,
TESTI2025174, TESTI2025229, TESTI2025269, TESTI2025365, TESTI2025403, TESTI2025409, TESTI2025422, TESTI2025448, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794,
TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028659, TESTI2028662, TESTI2028670,
TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029259, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067,
TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262,
TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036913, TESTI2036922, TESTI2036951, TESTI2036965, TESTI2036969, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048,
TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039177, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330,
TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276, TESTI2044306, TESTI2044309, TESTI2044413, TESTI2044418, TESTI2044754, TESTI2044788, TESTI2044796,
TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047792, TESTI2047801, TESTI2047818, TESTI2047824, TESTI2047885, TESTI2047930, TESTI2048105, TESTI2048109, TESTI2048465,
TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242, TESTI2053399, TESTI2053526, TESTI2053561, TESTI2053621, TESTI2053667, TESTI2053723,
TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001348, TESTI4001441, TESTI4001467, TESTI4001473, TESTI4001517, TESTI4001527, TESTI4001561, TESTI4001569, TESTI4001604, TESTI4001665, TESTI4001679,
TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002799, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003703, TESTI4003733, TESTI4003796, TESTI4003944, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158, TESTI4005317,
TESTI4005322, TESTI4005399, TESTI4005470, TESTI4005500, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007965, TESTI4008007,
TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013735, TESTI4013742, TESTI4013774, TESTI4013817, TESTI4013830, TESTI4013852, TESTI4013894,
TESTI4013924, TESTI4013960, TESTI4013962, TESTI4014159, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229,
TESTI4017254, TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456,
TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024245, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700,
TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029671, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128,
TESTI4032270, TESTI4032375, TESTI4032834, TESTI4032856, TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258,
TESTI4038284, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223,
TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746,
TESTI4047808, THYMU1000002, THYMU1000016, THYMU1000017, THYMU1000025, THYMU1000029, THYMU1000033, THYMU1000041, THYMU1000060, THYMU1000074, THYMU1000098, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000331, THYMU1000336, THYMU1000366, THYMU1000382, THYMU1000393, THYMU1000394, THYMU1000399, THYMU1000426, THYMU1000440, THYMU1000459, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032,
THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000317, THYMU2000368, THYMU2000369, THYMU2000418, THYMU2000436, THYMU2000440, THYMU2000489, THYMU2000492, THYMU2000519, THYMU2000570, THYMU2000602, THYMU2000660, THYMU2000671, THYMU2000702, THYMU2000767, THYMU2000800, THYMU2000879, THYMU2000932, THYMU2000946, THYMU2000950, THYMU2000971, THYMU2000982, THYMU2000990, THYMU2001007, THYMU2001018, THYMU2001053, THYMU2001071, THYMU2001090, THYMU2001139, THYMU2001202, THYMU2001203, THYMU2001256, THYMU2001270, THYMU2001291, THYMU2001314, THYMU2001325, THYMU2001357, THYMU2001381, THYMU2001422,
THYMU2001443, THYMU2001515, THYMU2001521, THYMU2001556, THYMU2001622, THYMU2001727, THYMU2001825, THYMU2001868, THYMU2001898, THYMU2001900, THYMU2001916, THYMU2001926, THYMU2001989, THYMU2002023, THYMU2002037, THYMU2002080, THYMU2002109, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002789, THYMU2002792, THYMU2002815, THYMU2002841, THYMU2002852, THYMU2002910, THYMU2002950, THYMU2003069, THYMU2003107, THYMU2003124, THYMU2003166, THYMU2003232, THYMU2003242, THYMU2003249, THYMU2003270, THYMU2003282, THYMU2003336,
THYMU2003366, THYMU2003397, THYMU2003440, THYMU2003470, THYMU2003479, THYMU2003632, THYMU2003650, THYMU2003700, THYMU2003760, THYMU2003803, THYMU2003855, THYMU2003907, THYMU2004003, THYMU2004008, THYMU2004042, THYMU2004108, THYMU2004111, THYMU2004123, THYMU2004139, THYMU2004169, THYMU2004284, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693, THYMU2004835, THYMU2004843, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321,
THYMU2005356, THYMU2005369, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006505, THYMU2006666, THYMU2006712, THYMU2006913, THYMU2006946, THYMU2006965, THYMU2007025, THYMU2007036, THYMU2007095, THYMU2007112, THYMU2007146, THYMU2007179, THYMU2007258, THYMU2007307, THYMU2007308, THYMU2007339, THYMU2007350, THYMU2007384, THYMU2007493,
THYMU2007521, THYMU2007528, THYMU2007530, THYMU2007532, THYMU2007561, THYMU2007571, THYMU2007635, THYMU2007658, THYMU2007667, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008339, THYMU2008350, THYMU2008351, THYMU2008383, THYMU2008452, THYMU2008643, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104,
THYMU2009134, THYMU2009157, THYMU2009181, THYMU2009338, THYMU2009425, THYMU2009516, THYMU2009526, THYMU2009546, THYMU2009592, THYMU2009596, THYMU2009835, THYMU2009906, THYMU2009948, THYMU2010030, THYMU2010041, THYMU2010046, THYMU2010059, THYMU2010082, THYMU2010094, THYMU2010161, THYMU2010192, THYMU2010637, THYMU2010671, THYMU2010699, THYMU2010705, THYMU2010779, THYMU2010816, THYMU2010831, THYMU2010848, THYMU2011053, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011538, THYMU2011548, THYMU2011551, THYMU2011564, THYMU2011573, THYMU2011585, THYMU2011621, THYMU2011666,
THYMU2011736, THYMU2011785, THYMU2011806, THYMU2011852, THYMU2011875, THYMU2011881, THYMU2011937, THYMU2011939, THYMU2012002, THYMU2012024, THYMU2012073, THYMU2012104, THYMU2012113, THYMU2012273, THYMU2012401, THYMU2012507, THYMU2012565, THYMU2012574, THYMU2012625, THYMU2012631, THYMU2012665, THYMU2012701, THYMU2012807, THYMU2012826, THYMU2012882, THYMU2012891, THYMU2012902, THYMU2012919, THYMU2013023, THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014183, THYMU2014204, THYMU2014297,
THYMU2014323, THYMU2014327, THYMU2014353, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015775, THYMU2015825, THYMU2015918, THYMU2015943, THYMU2015984, THYMU2016113, THYMU2016164, THYMU2016204, THYMU2016219, THYMU2016360, THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831,
THYMU2017844, THYMU2017878, THYMU2017948, THYMU2018028, THYMU2018071, THYMU2018189, THYMU2018197, THYMU2018384, THYMU2018455, THYMU2018547, THYMU2018565, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587, THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264,
THYMU2023576, THYMU2023711, THYMU2023900, THYMU2023943, THYMU2023967, THYMU2024071, THYMU2024185, THYMU2024207, THYMU2024616, THYMU2024684, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027996, THYMU2028041, THYMU2028368, THYMU2028379, THYMU2028394, THYMU2028412, THYMU2028632, THYMU2028724, THYMU2028739, THYMU2028942, THYMU2028978, THYMU2029134, THYMU2029676, THYMU2029688, THYMU2029788,
THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031890, THYMU2031994, THYMU2032035, THYMU2032080, THYMU2032150, THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033308, THYMU2033401, THYMU2033583, THYMU2033755, THYMU2033787, THYMU2033816, THYMU2034182, THYMU2034279, THYMU2034314,
THYMU2034338, THYMU2034917, THYMU2035064, THYMU2035078, THYMU2035101, THYMU2035319, THYMU2035321, THYMU2035388, THYMU2035400, THYMU2035554, THYMU2035710, THYMU2035735, THYMU2036058, THYMU2036105, THYMU2036207, THYMU2036215, THYMU2036252, THYMU2036265, THYMU2036366, THYMU2036459, THYMU2036461, THYMU2036604, THYMU2036653, THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634,
THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390, THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003092, THYMU3003212, THYMU3003309,
THYMU3003350, THYMU3003403, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371, THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755,
THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571, THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713,
THYMU3020856, THYMU3020869, THYMU3020970, THYMU3021404, THYMU3021586, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400,.THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000, THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231,
THYMU3030706, THYMU3030710, THYMU3030752, THYMU3031146, THYMU3031402, THYMU3031610, THYMU3031612, THYMU3031868, THYMU3031878, THYMU3032032, THYMU3033402, THYMU3033626, THYMU3033630, THYMU3033649, THYMU3033754, THYMU3033759, THYMU3034078, THYMU3034099, THYMU3034453, THYMU3034616, THYMU3034671, THYMU3034853, THYMU3034867, THYMU3034983, THYMU3036200, THYMU3036310, THYMU3036934, THYMU3036953, THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970,
THYMU3039807, THYMU3039846, THYMU3040126, THYMU3040146, THYMU3040168, THYMU3040172, THYMU3040725, THYMU3040746, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779, THYMU3043883, THYMU3043993, THYMU3044175, THYMU3044188, THYMU3044441, THYMU3044445, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044,
TKIDN1000062, TKIDN1000066, TKIDN1000164, TKIDN1000171, TKIDN1000192, TKIDN2000127, TKIDN2000240, TKIDN2000246, TKIDN2000319, TKIDN2000521, TKIDN2000701, TKIDN2001529, TKIDN2001662, TKIDN2002318, TKIDN2002329, TKIDN2002424, TKIDN2002632, TKIDN2003044, TKIDN2003059, TKIDN2003062, TKIDN2003078, TKIDN2003366, TKIDN2003396, TKIDN2003413, TKIDN2003597, TKIDN2003601, TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278,
TKIDN2010602, TKIDN2010812, TKIDN2010934, TKIDN2011051, TKIDN2011160, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002882, TLIVE2003197, TLIVE2003200, TLIVE2003225, TLIVE2003381, TLIVE2003737, TLIVE2003961, TLIVE2004110, TLIVE2004320,
TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000018, TRACH1000057, TRACH1000074, TRACH1000100, TRACH1000125, TRACH1000140, TRACH1000144,
TRACH1000181, TRACH1000193, TRACH1000205, TRACH1000212, TRACH1000240, TRACH2000002, TRACH2000015, TRACH2000024, TRACH2000032, TRACH2000079, TRACH2000081, TRACH2000083, TRACH2000148, TRACH2000185, TRACH2000196, TRACH2000235, TRACH2000250, TRACH2000259, TRACH2000272, TRACH2000287, TRACH2000289, TRACH2000306, TRACH2000312, TRACH2000336, TRACH2000393, TRACH2000411, TRACH2000420, TRACH2000461, TRACH2000496, TRACH2000497, TRACH2000502, TRACH2000522, TRACH2000559, TRACH2000660, TRACH2000665, TRACH2000675, TRACH2000677, TRACH2000703, TRACH2000767, TRACH2000775, TRACH2000780, TRACH2000807, TRACH2000862, TRACH2000873, TRACH2000894, TRACH2000898, TRACH2000923, TRACH2000926, TRACH2000944, TRACH2000959,
TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001192, TRACH2001249, TRACH2001275, TRACH2001314, TRACH2001335, TRACH2001339, TRACH2001341, TRACH2001347, TRACH2001395, TRACH2001400, TRACH2001403, TRACH2001416, TRACH2001443, TRACH2001474, TRACH2001478, TRACH2001525, TRACH2001532, TRACH2001592, TRACH2001612, TRACH2001621, TRACH2001646, TRACH2001651, TRACH2001684, TRACH2001810, TRACH2001898, TRACH2001933, TRACH2001968, TRACH2001996, TRACH2002054, TRACH2002092, TRACH2002100, TRACH2002333, TRACH2002784, TRACH2002840, TRACH2002853, TRACH2002862, TRACH2002954, TRACH2002960, TRACH2002988,
TRACH2003070, TRACH2003272, TRACH2003484, TRACH2003516, TRACH2003605, TRACH2003736, TRACH2003759, TRACH2003941, TRACH2004039, TRACH2004054, TRACH2004087, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762,
TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007894, TRACH2007969, TRACH2007988, TRACH2008081, TRACH2008112, TRACH2008113, TRACH2008127, TRACH2008130, TRACH2008278, TRACH2008300, TRACH2008583, TRACH2008597, TRACH2008820, TRACH2008891, TRACH2008921, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419,
TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011574, TRACH2011894, TRACH2011956, TRACH2012000, TRACH2012138, TRACH2012242, TRACH2012370, TRACH2012387, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268,
TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016481, TRACH2016498, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949,
TRACH2017965, TRACH2017980, TRACH2018034, TRACH2018084, TRACH2018262, TRACH2018273, TRACH2018278, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023299, TRACH2023306,
TRACH2023416, TRACH2023479, TRACH2023684, TRACH2023860, TRACH2023876, TRACH2024139, TRACH2024187, TRACH2024271, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119,
TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003379, TRACH3003586, TRACH3003683, TRACH3003780, TRACH3003805, TRACH3003832, TRACH3003872, TRACH3004068, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004721, TRACH3004747, TRACH3004760, TRACH3004783, TRACH3004786, TRACH3004840, TRACH3004931, TRACH3004934, TRACH3005072,
TRACH3005085, TRACH3005102, TRACH3005173, TRACH3005191, TRACH3005211, TRACH3005274, TRACH3005479, TRACH3005549, TRACH3005699, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007479, TRACH3007541, TRACH3007595, TRACH3007625, TRACH3007627, TRACH3007689, TRACH3007766, TRACH3007866, TRACH3007995, TRACH3008042, TRACH3008061, TRACH3008201, TRACH3008508, TRACH3008535, TRACH3008629,
TRACH3008632, TRACH3008688, TRACH3008697, TRACH3008713, TRACH3008887, TRACH3008902, TRACH3008990, TRACH3009001, TRACH3009059, TRACH3009061, TRACH3009148, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684,
TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, -TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447,
TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316,
TRACH3025346, TRACH3025520, TRACH3026148, TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678,
TRACH3031936, TRACH3032044; TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037267, TRACH3037288, TRACH3037505, TRACH3037573, TRACH3037696,
TRACH3037897, TRACH3038086, TRACH3038399, TSTOM1000135, TSTOM1000186, TSTOM2000139, TSTOM2000235, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000122, TUTER1000137, TUTER2000057, TUTER2000283, TUTER2000425, TUTER2000904, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000143, UMVEN1000156, UMVEN1000186, UMVEN2000046, UMVEN2000069, UMVEN2000121, UMVEN2000152, UMVEN2000354,
UTERU1000008, UTERU1000015, UTERU1000024, UTERU1000031, UTERU1000032, UTERU1000057, UTERU1000065, UTERU1000077, UTERU1000093, UTERU1000096, UTERU1000106, UTERU1000109, UTERU1000131, UTERU1000138, UTERU1000148, UTERU1000160, UTERU1000182, UTERU1000183, UTERU1000187, UTERU1000192, UTERU1000249, UTERU1000337, UTERU1000339, UTERU1000384, UTERU2000023, UTERU2000047, UTERU2000074, UTERU2000099, UTERU2000154, UTERU2000197, UTERU2000218, UTERU2000238, UTERU2000243, UTERU2000260, UTERU2000263, UTERU2000300, UTERU2000329, UTERU2000349, UTERU2000377, UTERU2000393, UTERU2000418, UTERU2000465, UTERU2000485, UTERU2000517, UTERU2000524, UTERU2000539, UTERU2000542, UTERU2000546, UTERU2000550, UTERU2000569,
UTERU2000607, UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001607, UTERU2001658, UTERU2001747, UTERU2001876, UTERU2002001, UTERU2002011, UTERU2002176, UTERU2002198, UTERU2002332, UTERU2002473, UTERU2002547, UTERU2002662, UTERU2002693, UTERU2002733, UTERU2002736, UTERU2002737, UTERU2002826, UTERU2002841, UTERU2002964, UTERU2002993, UTERU2003035, UTERU2003126, UTERU2003135, UTERU2003321, UTERU2003399, UTERU2003411, UTERU2003577, UTERU2003704, UTERU2003926, UTERU2003973, UTERU2004015, UTERU2004037,
UTERU2004039, UTERU2004061, UTERU2004073, UTERU2004163, UTERU2004299, UTERU2004461, UTERU2004520, UTERU2004564, UTERU2004688, UTERU2004698, UTERU2004861, UTERU2004929, UTERU2005004, UTERU2005050, UTERU2005069, UTERU2005074, UTERU2005179, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639,
UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008962, UTERU2009094, UTERU2009120, UTERU2009131, UTERU2009147, UTERU2009206, UTERU2009283, UTERU2009335, UTERU2009414, UTERU2009435, UTERU2009483, UTERU2009510, UTERU2009540, UTERU2009776, UTERU2009904, UTERU2009951, UTERU2009972, UTERU2010115, UTERU2010124, UTERU2010164, UTERU2010231,. UTERU2010304, UTERU2010320, UTERU2010431, UTERU2010525, UTERU2010651, UTERU2010724, UTERU2010747,
UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031, UTERU2012101, UTERU2012114, UTERU2012230, UTERU2012252, UTERU2012286, UTERU2012333, UTERU2012407, UTERU2012526, UTERU2012581, UTERU2012610, UTERU2012615, UTERU2012688, UTERU2012703, UTERU2012715, UTERU2012767, UTERU2012786, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024,
UTERU2014167, UTERU2014223, UTERU2014398, UTERU2014464, UTERU2014548, UTERU2014601, UTERU2014631, UTERU2014668, UTERU2014678, UTERU2014728, UTERU2014998, UTERU2015062, UTERU2015087, UTERU2015108, UTERU2015198, UTERU2015202, UTERU2015405, UTERU2015640, UTERU2015653, UTERU2015830, UTERU2015880, UTERU2016147, UTERU2016157, UTERU2016426, UTERU2016464, UTERU2016669, UTERU2016757, UTERU2016761, UTERU2016799, UTERU2016896, UTERU2016902, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522,
UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019096, UTERU2019163, UTERU2019257, UTERU2019453, UTERU2019491, UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023175, UTERU2023262, UTERU2023651, UTERU2023687, UTERU2023712, UTERU2023941, UTERU2024002, UTERU2024042,
UTERU2024141, UTERU2024481, UTERU2024656, UTERU2024758, UTERU2024820, UTERU2024881, UTERU2024969, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941, UTERU2028377, UTERU2028734, UTERU2029503, UTERU2029660, UTERU2029742, UTERU2029953, UTERU2030213, UTERU2030270, UTERU2030280, UTERU2031060, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420,
UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623, UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000844, UTERU3000899, UTERU3000959, UTERU3001029, UTERU3001053, UTERU3001059,
UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465, UTERU3003495, UTERU3003523, UTERU3003660, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005159, UTERU3005205, UTERU3005230, UTERU3005264, UTERU3005422, UTERU3005460, UTERU3005536, UTERU3005585, UTERU3005907, '
UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010409, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599,
UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, ' UTERU3017995, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the SYNOV, T1ESE, TBAES, TCERX, TCOLN, TESOP, and TESTI libraries were analyzed for expression frequency. The analysis results are described below. Each is a relative value, where the expression level over all tissues is taken as 100. The larger this value, the greater the level of expression.

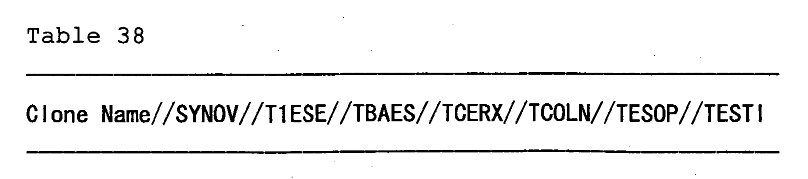

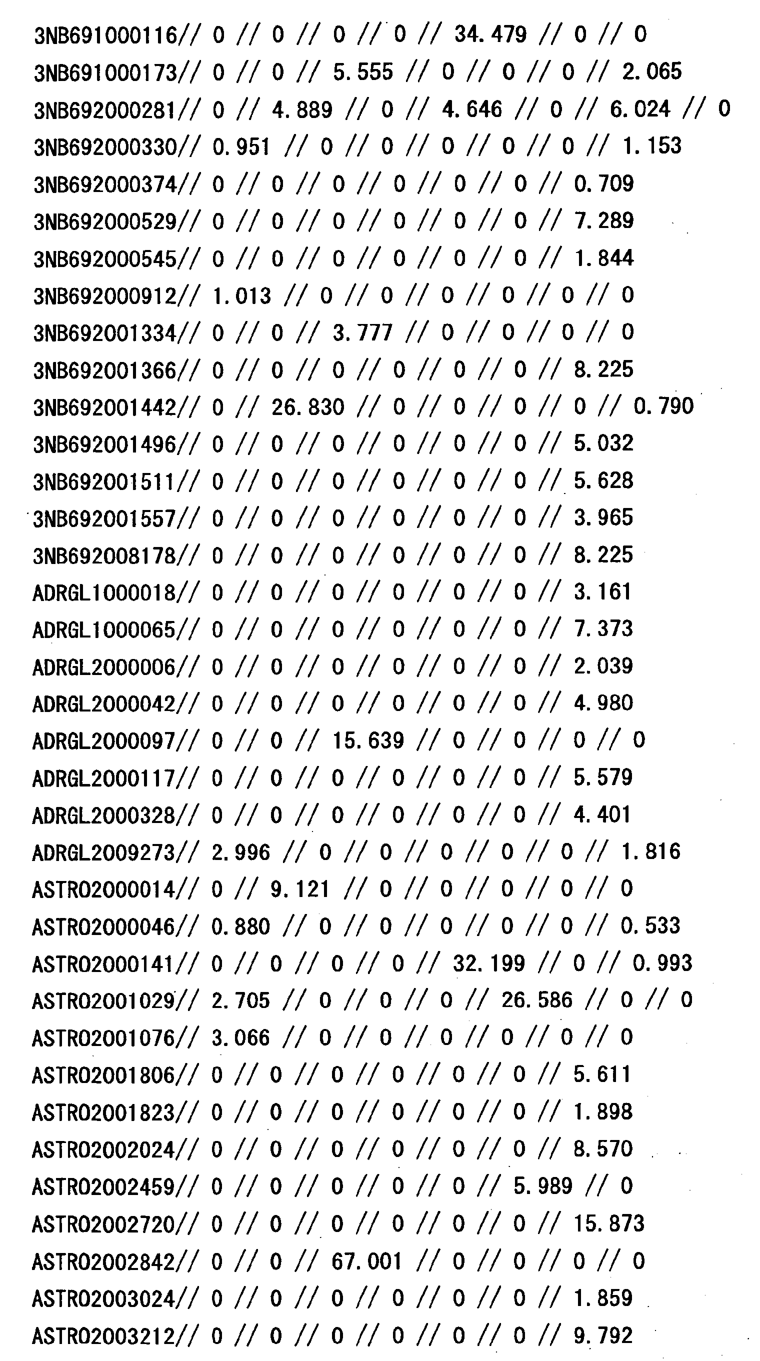

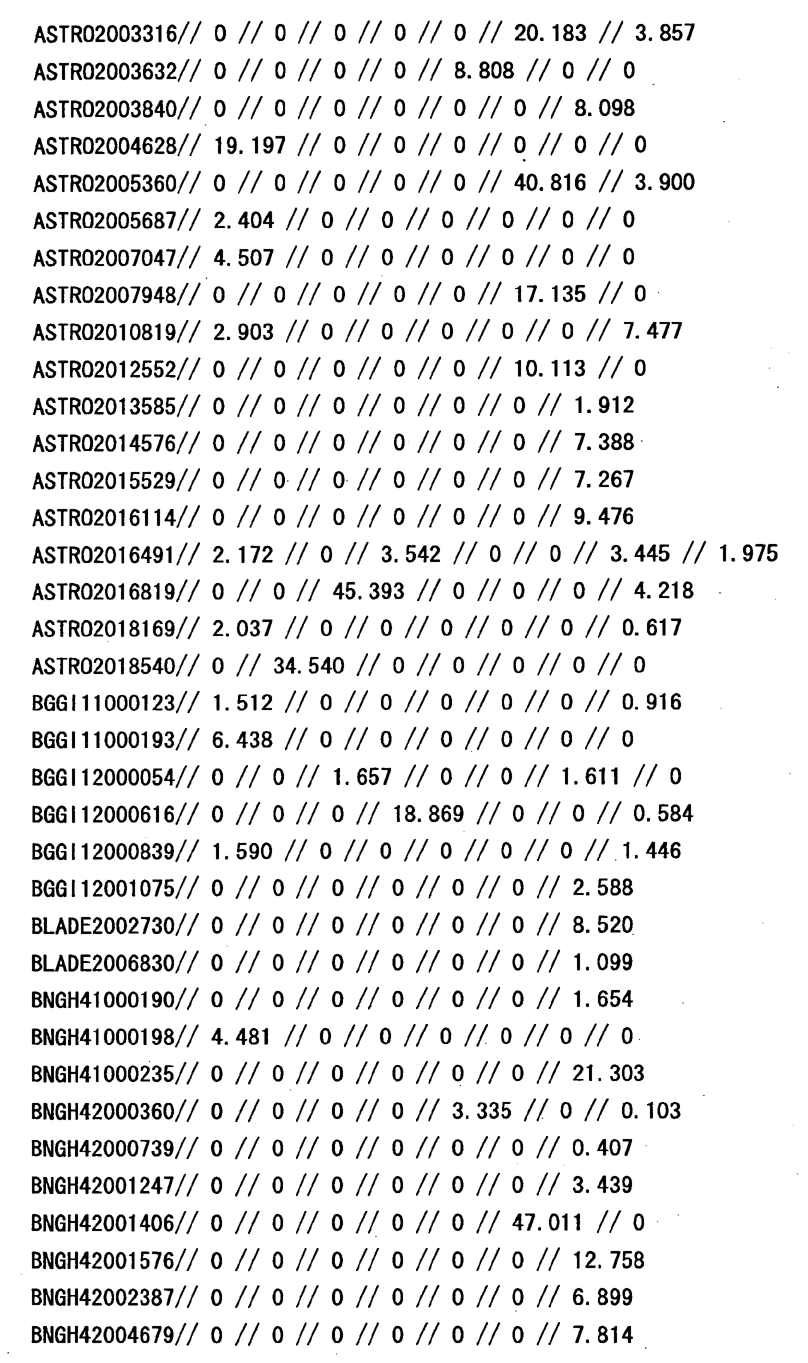

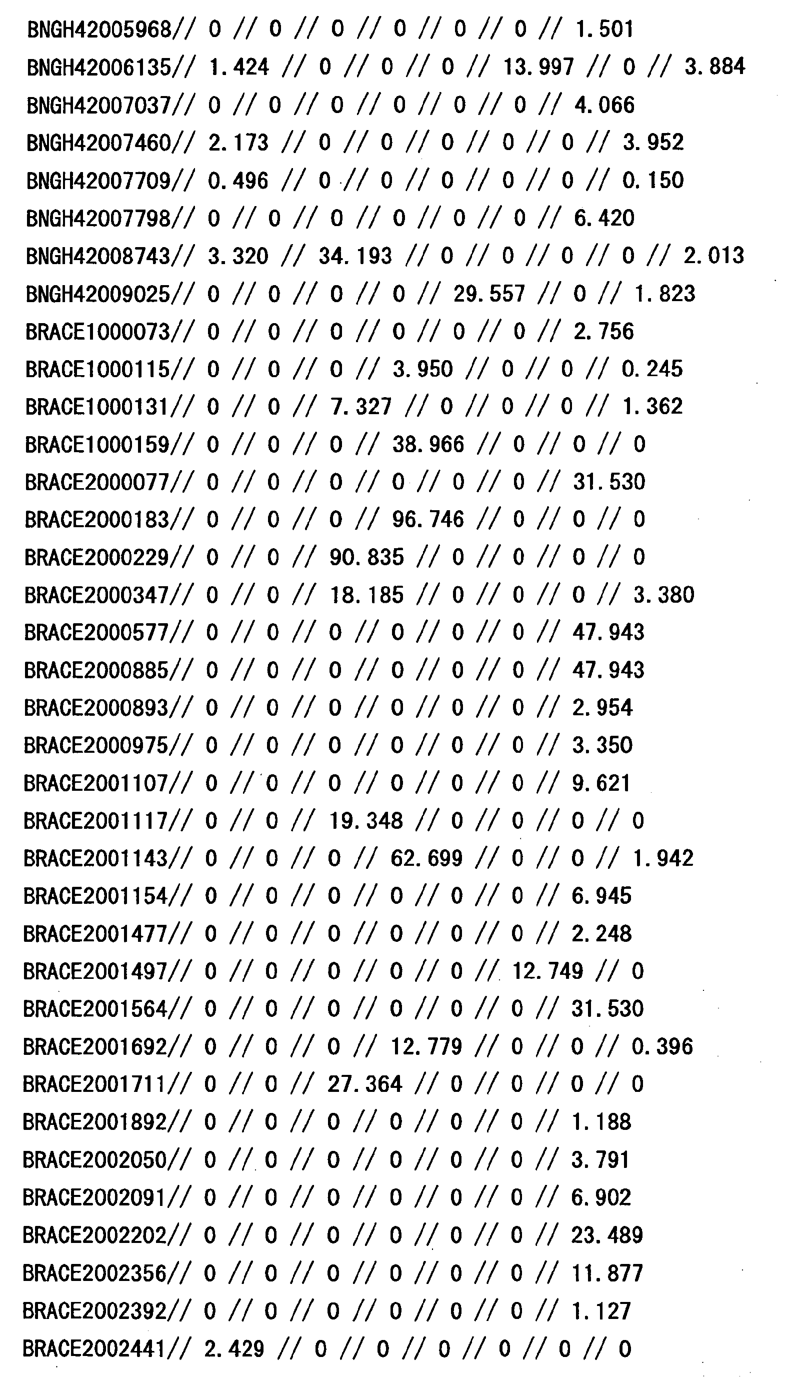

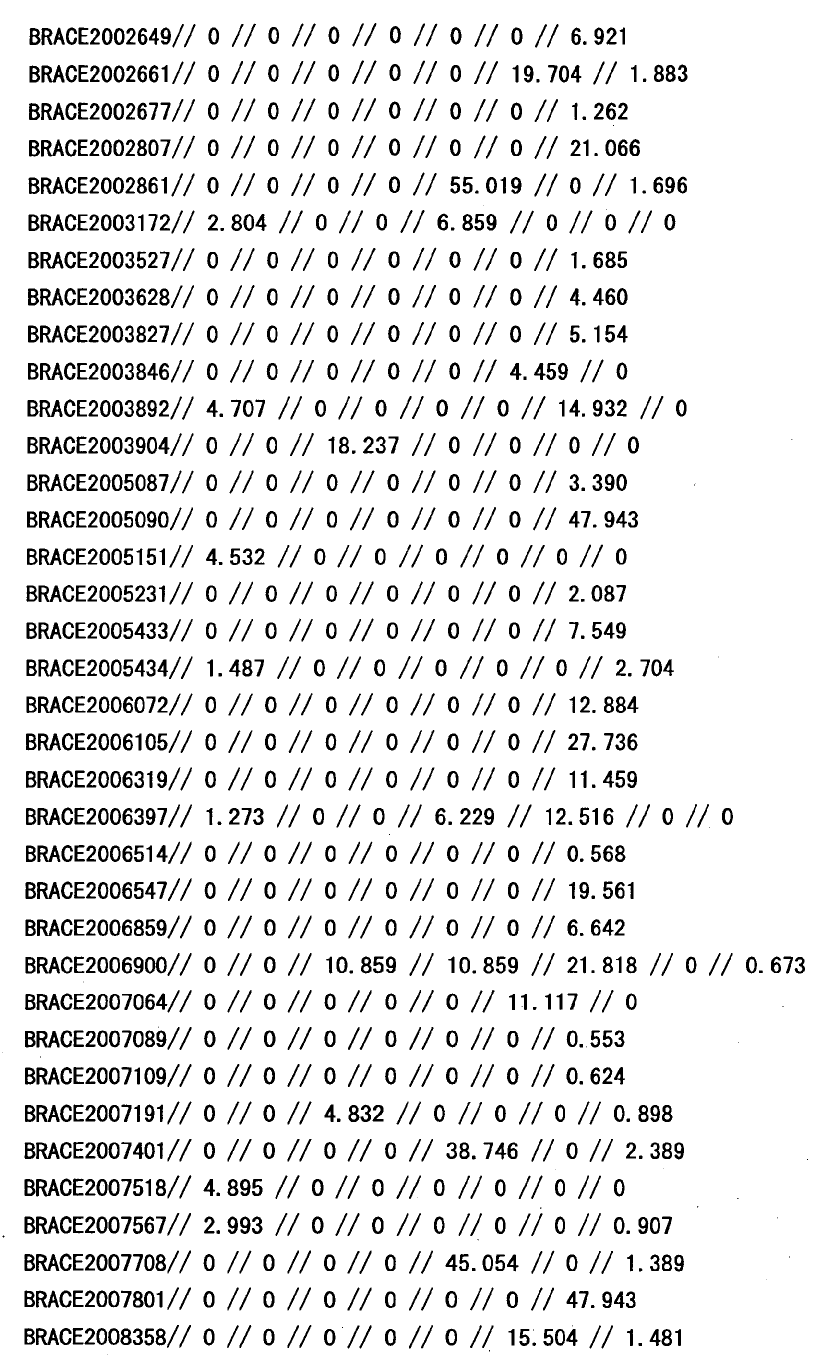

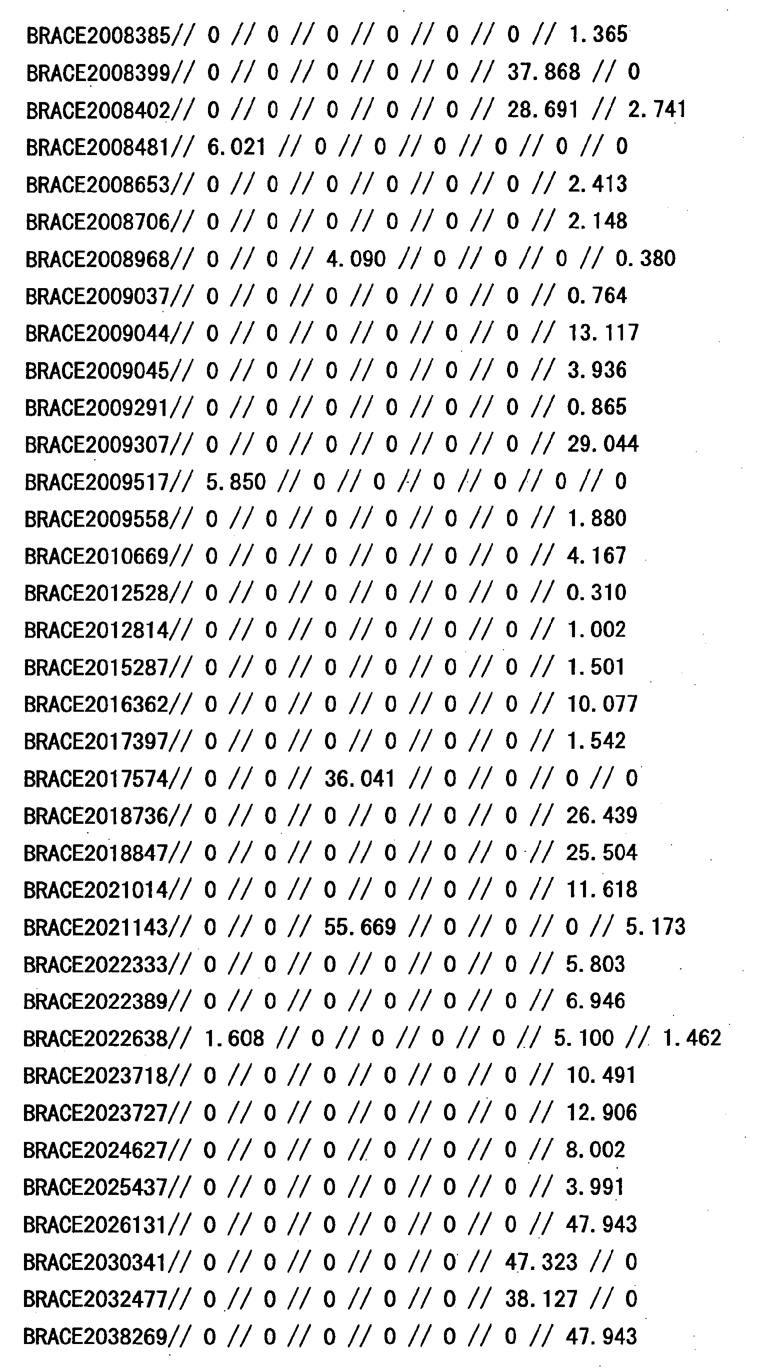

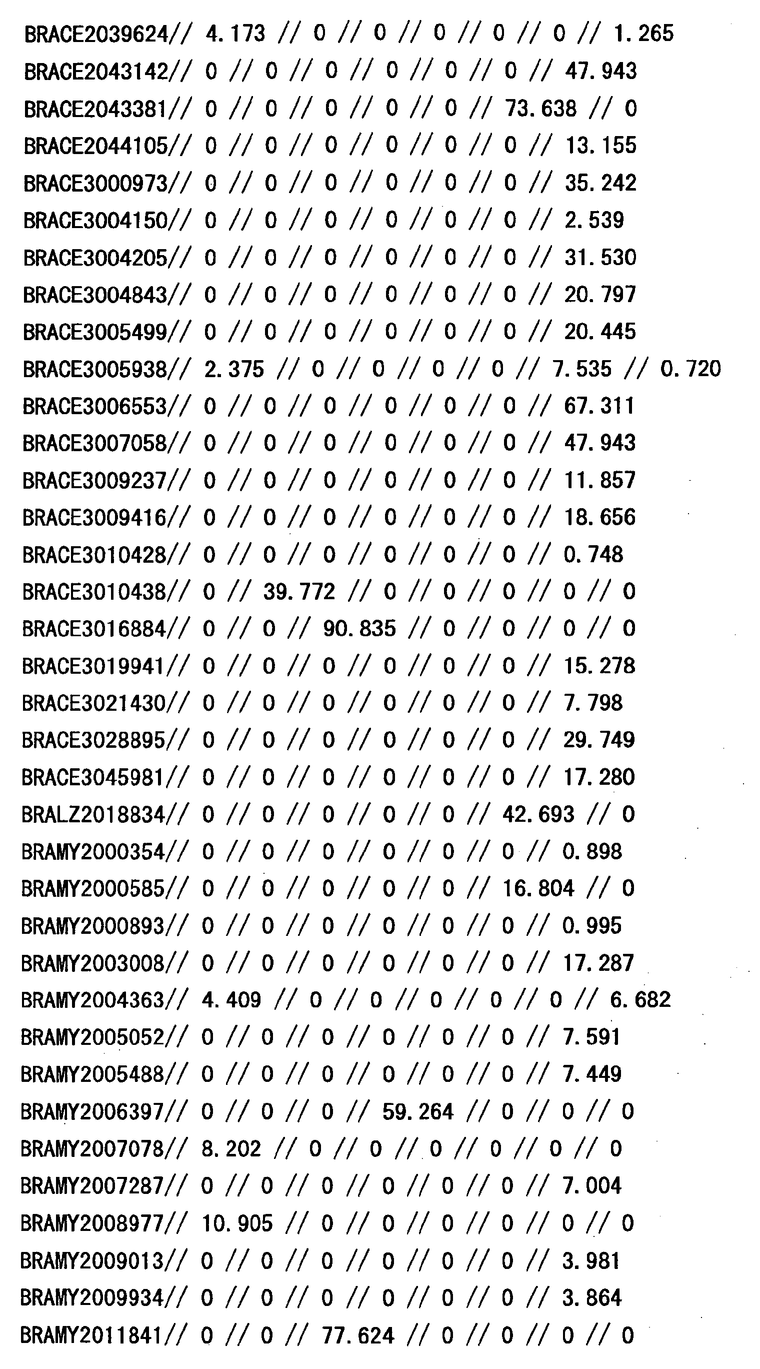

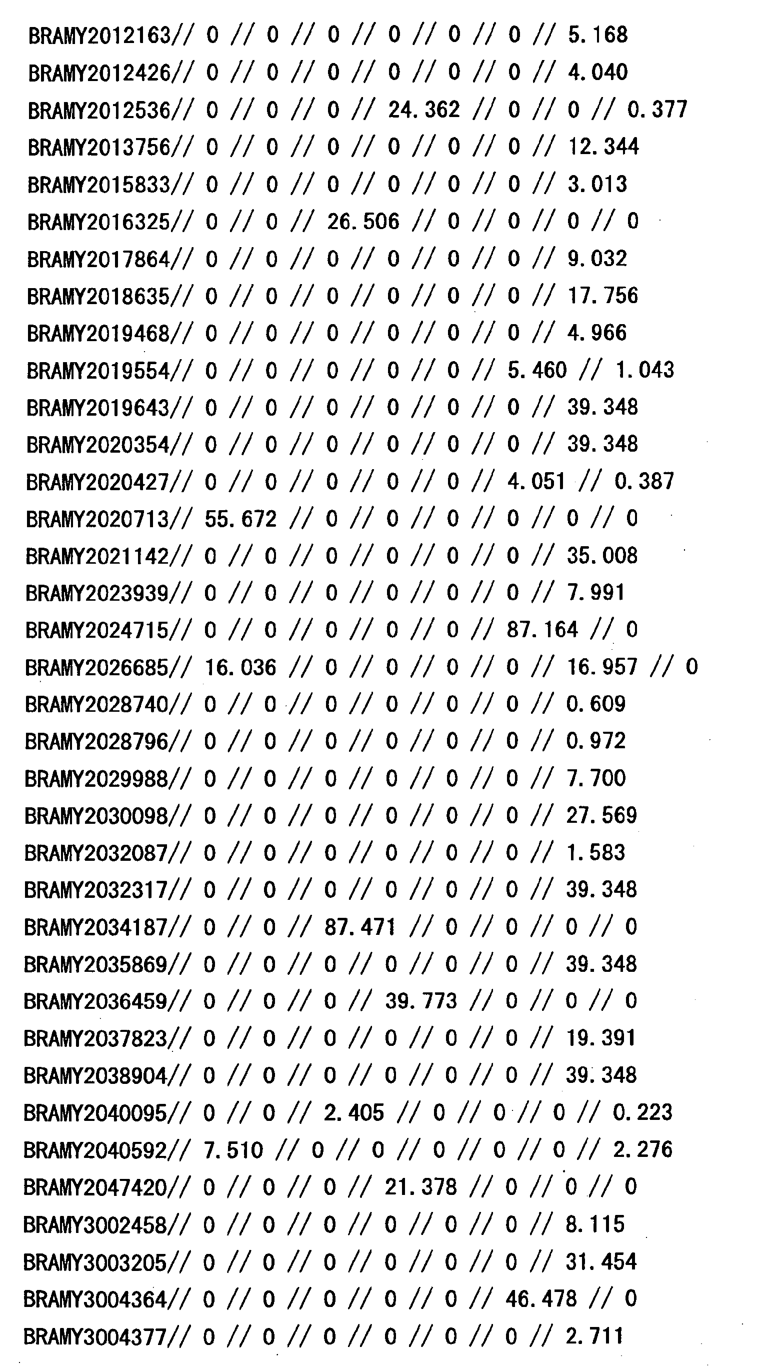

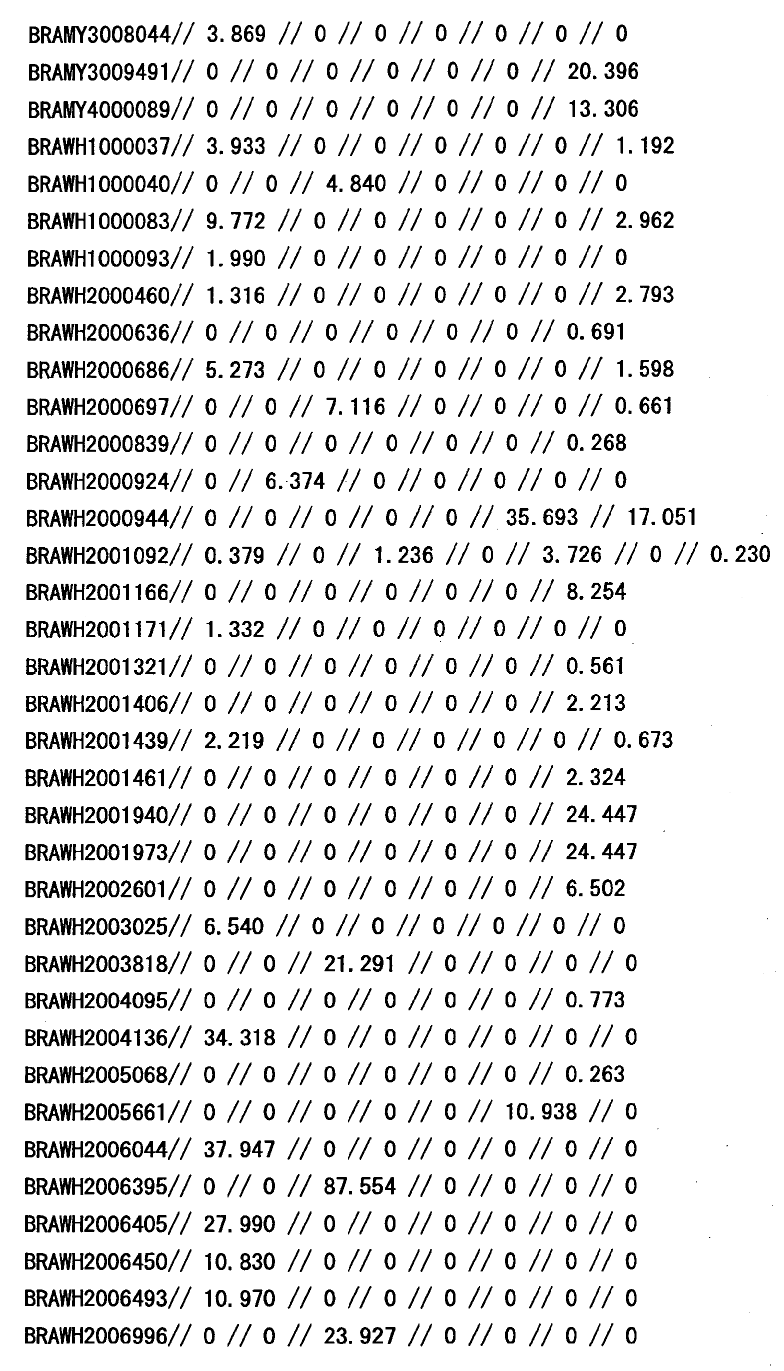

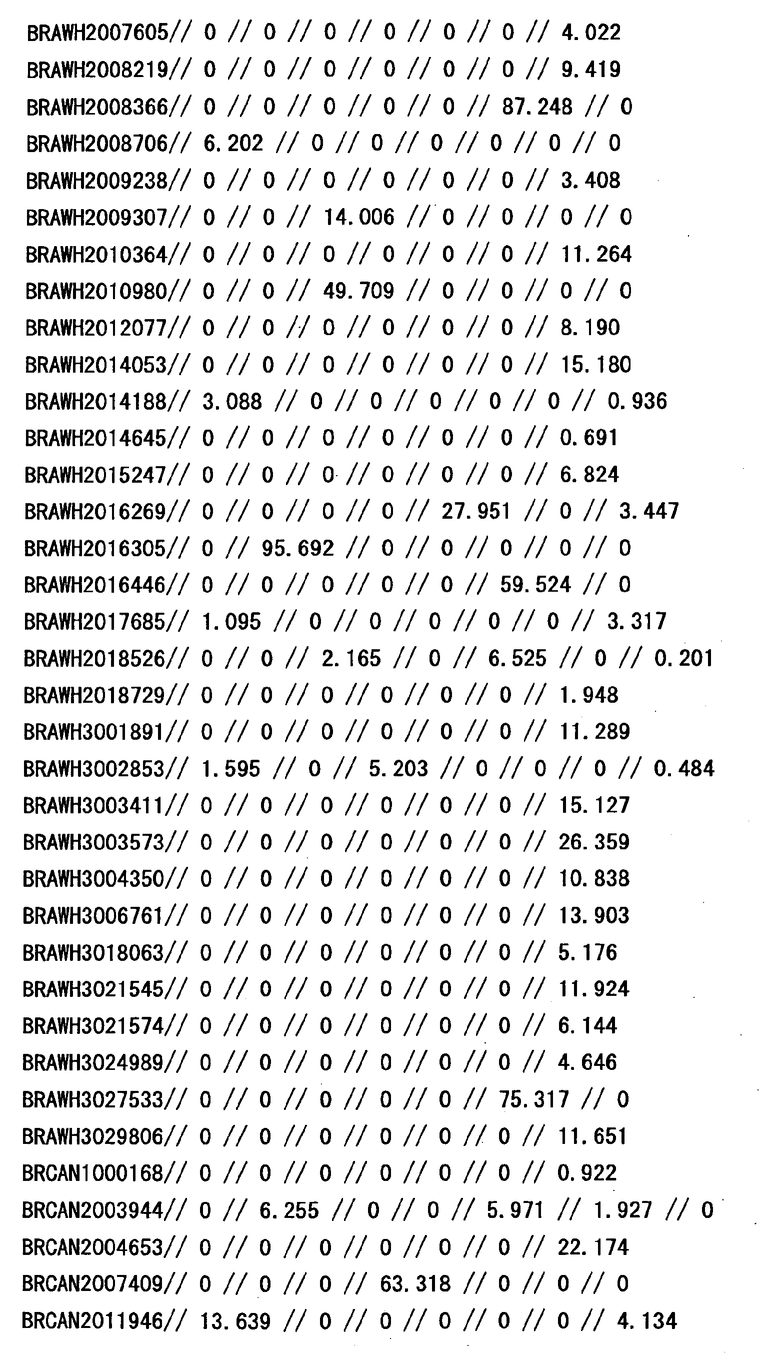

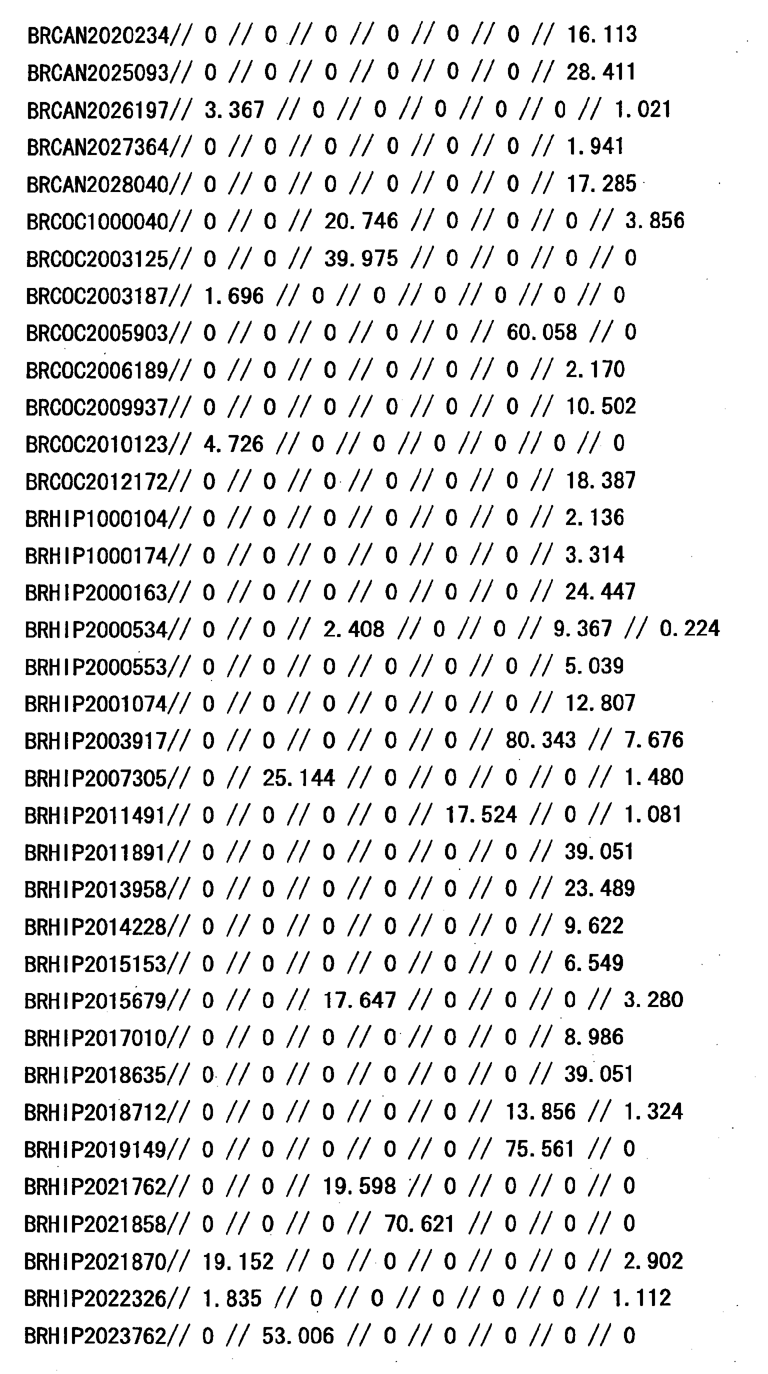

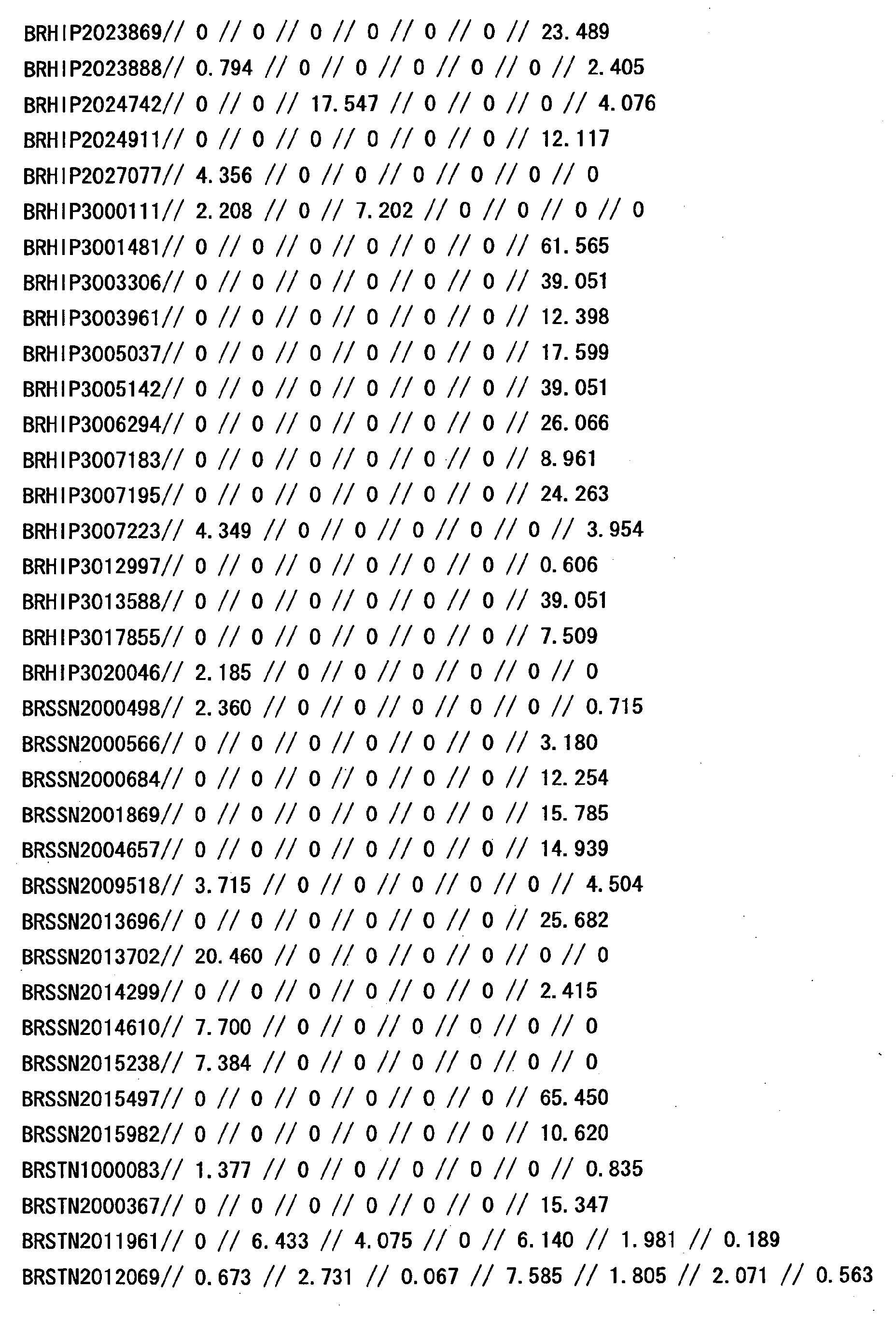

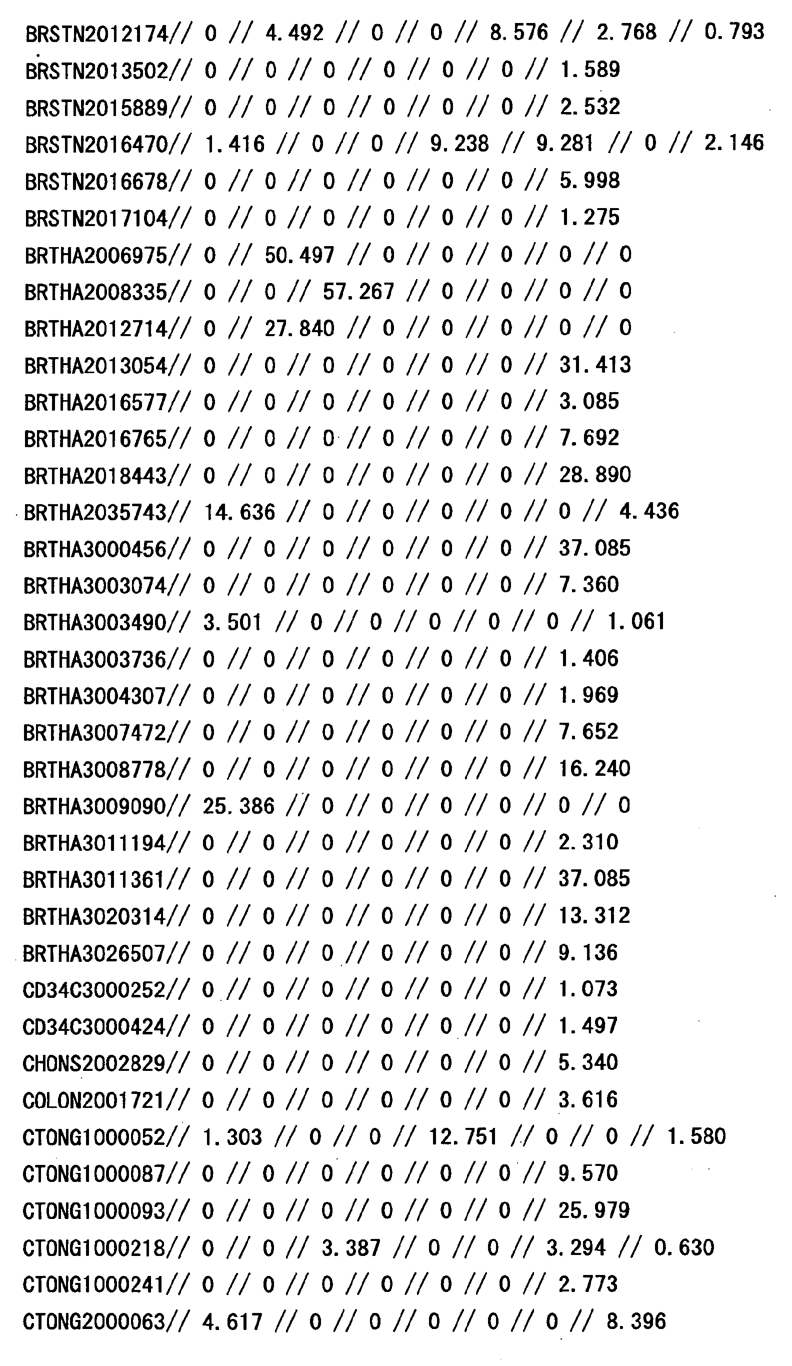

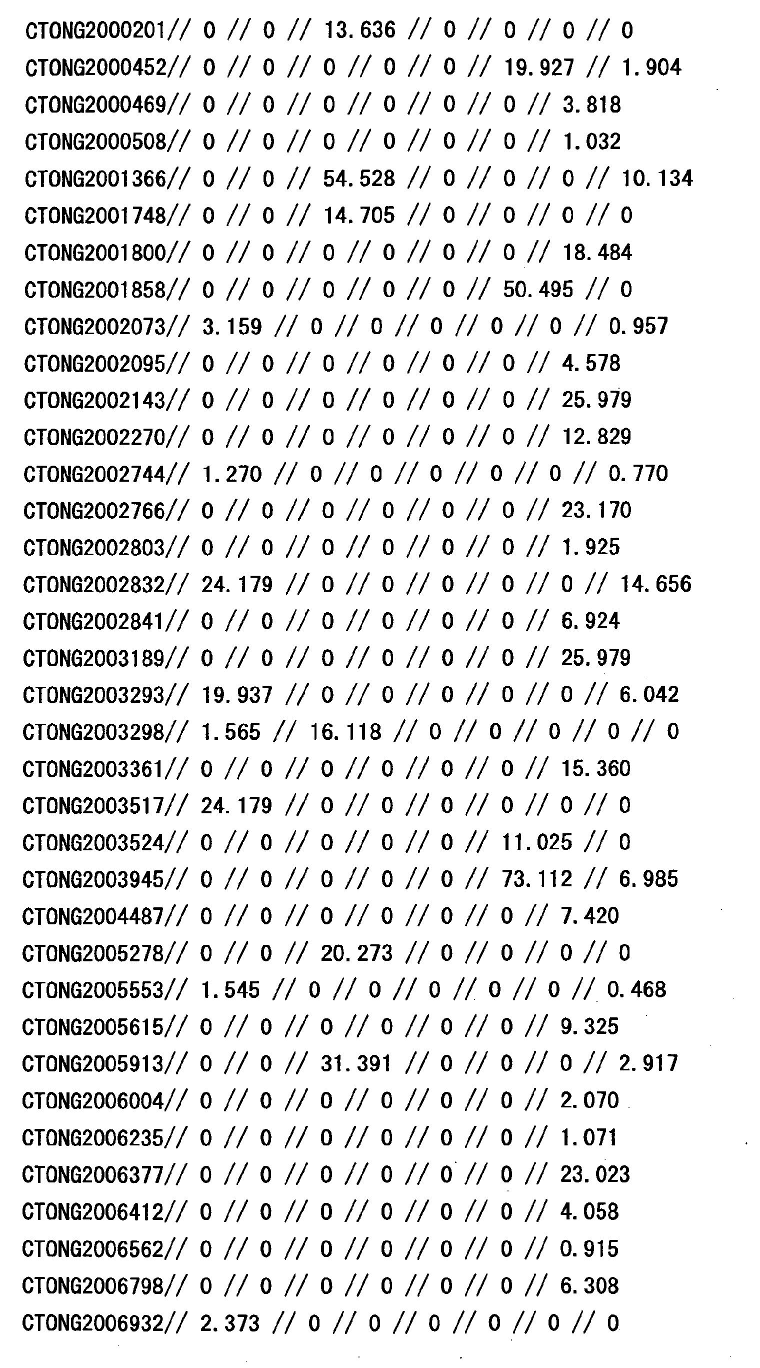

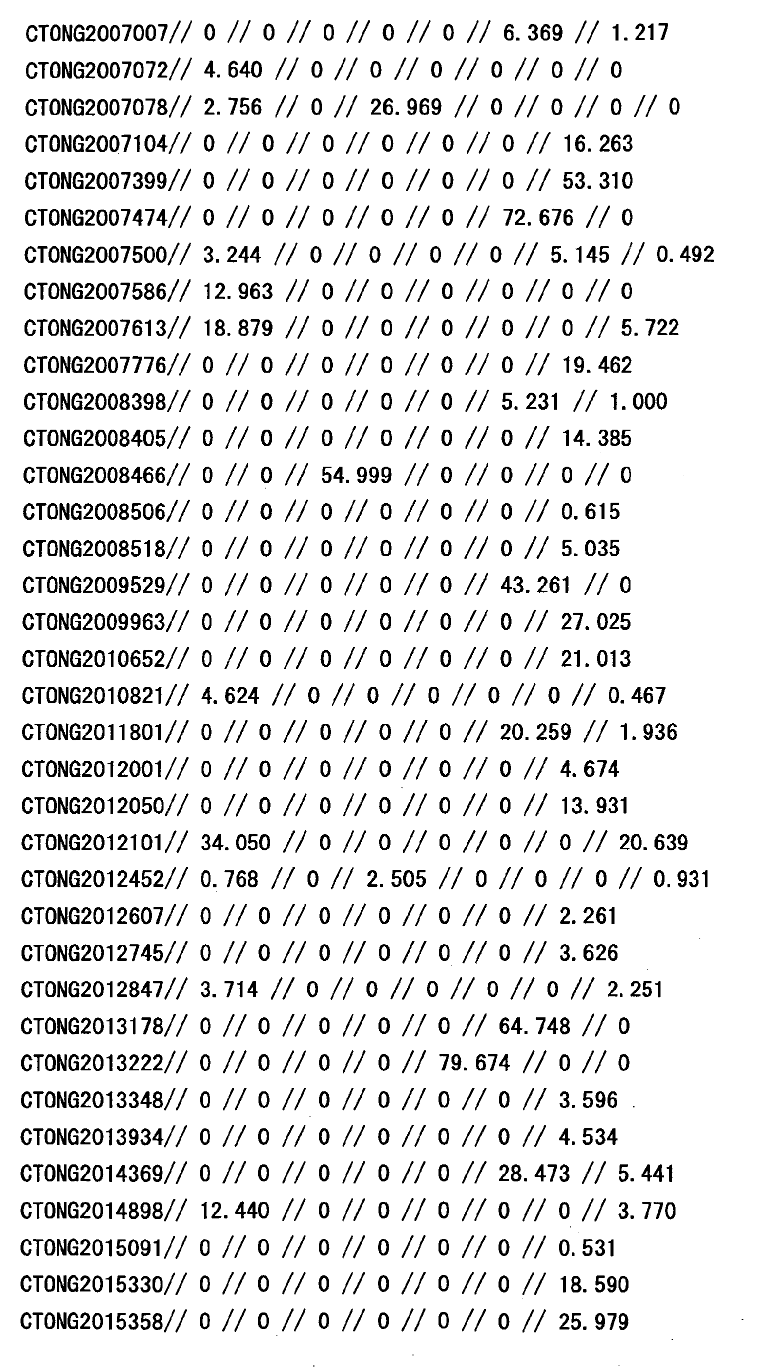

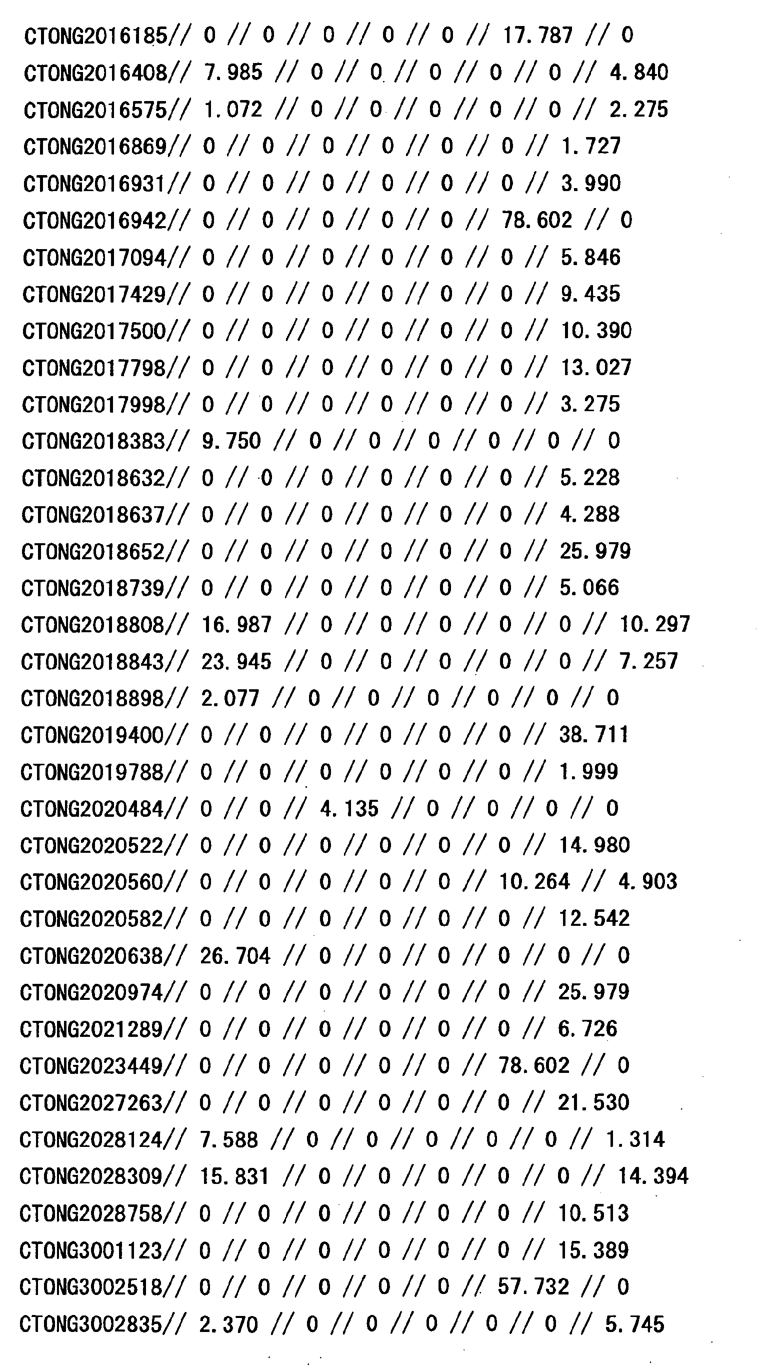

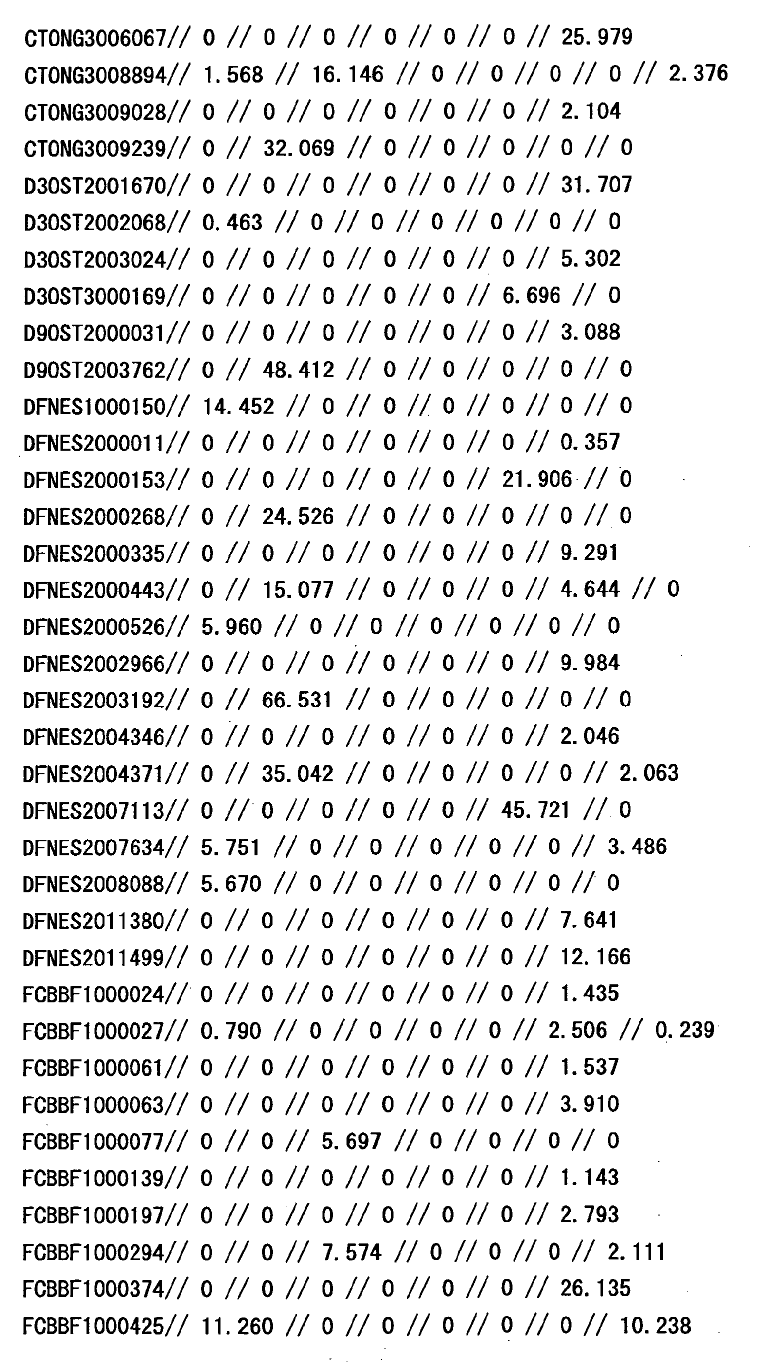

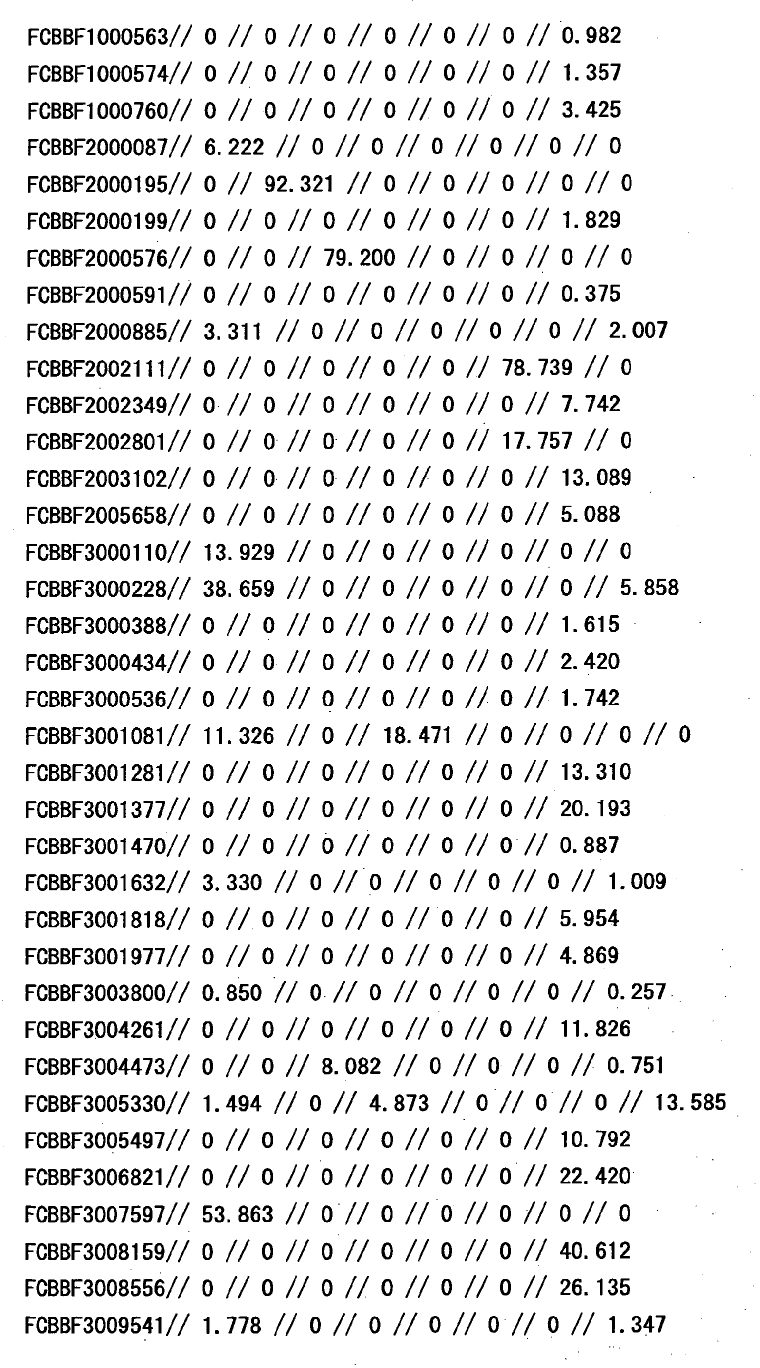

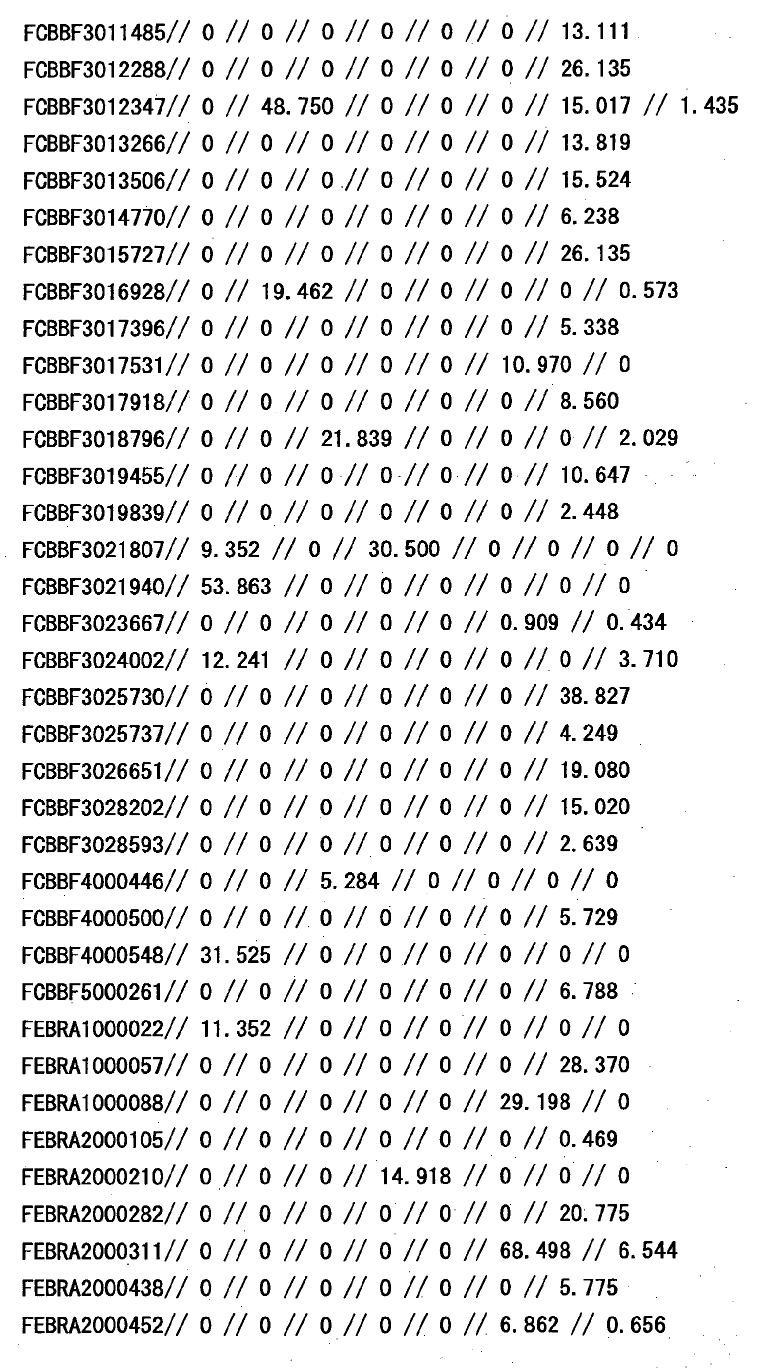

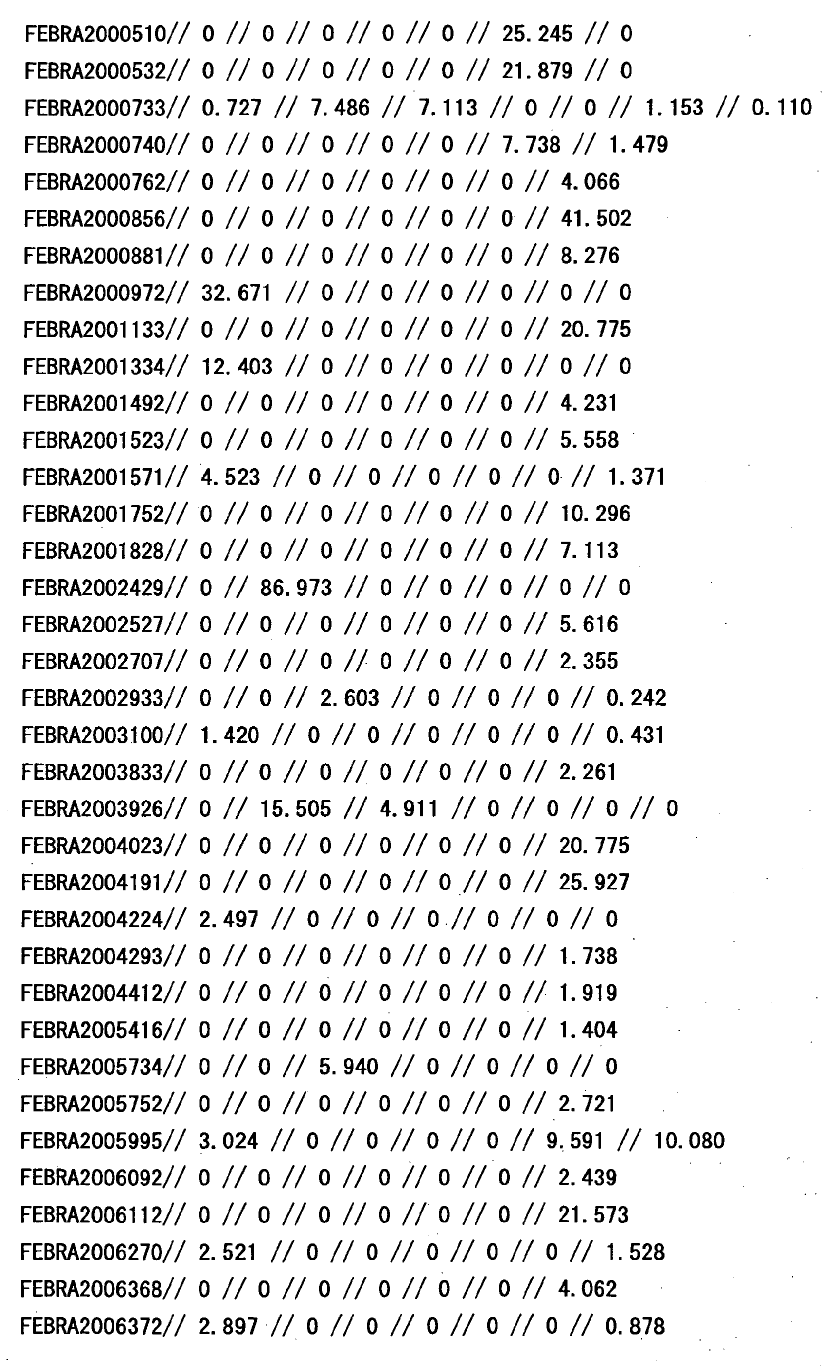

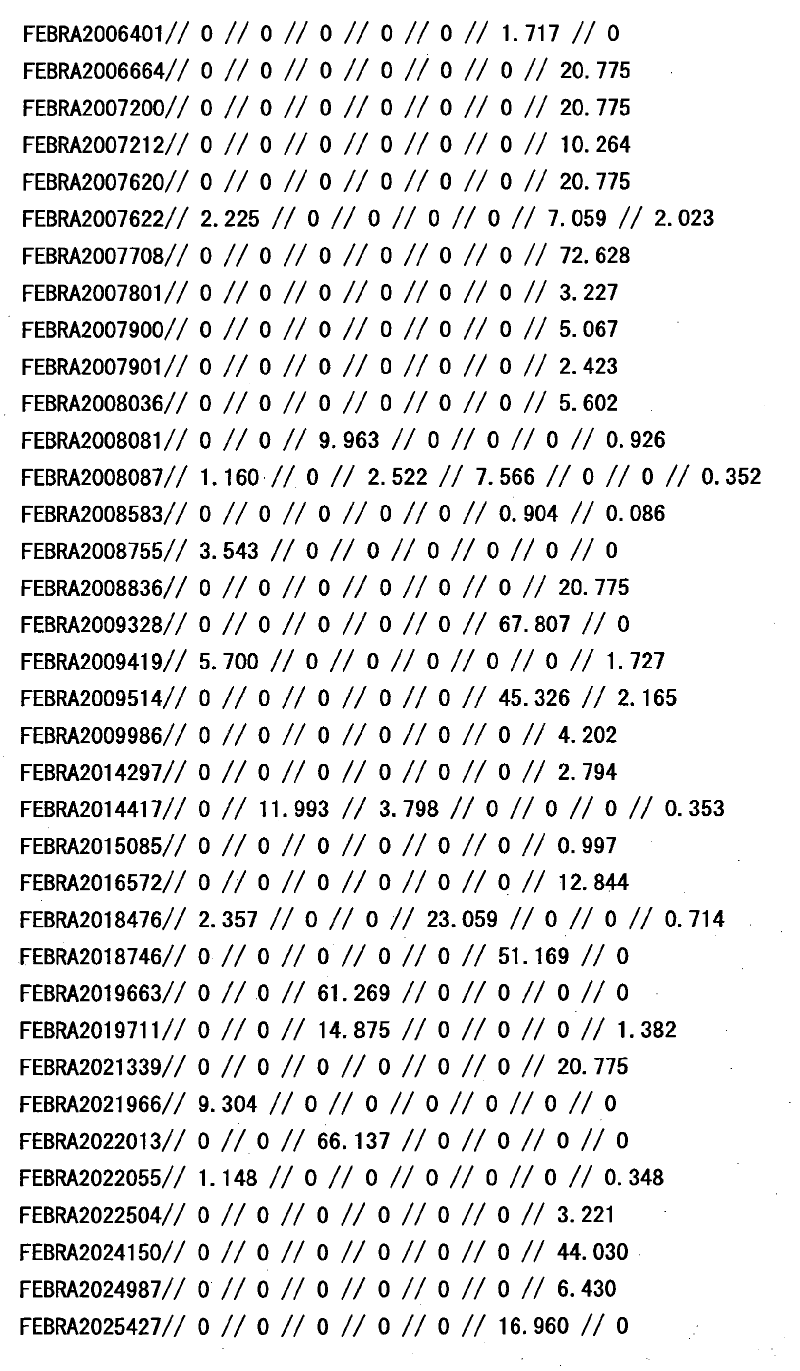

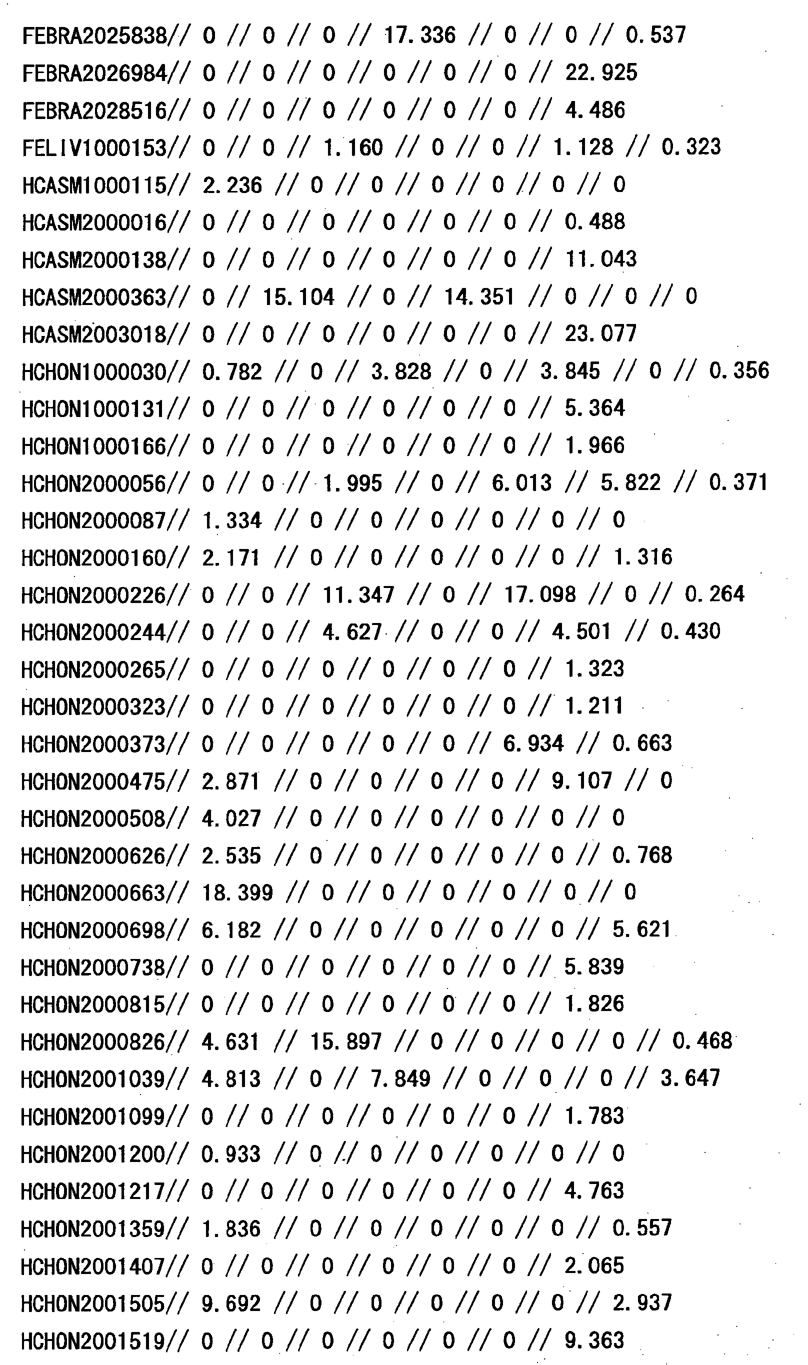

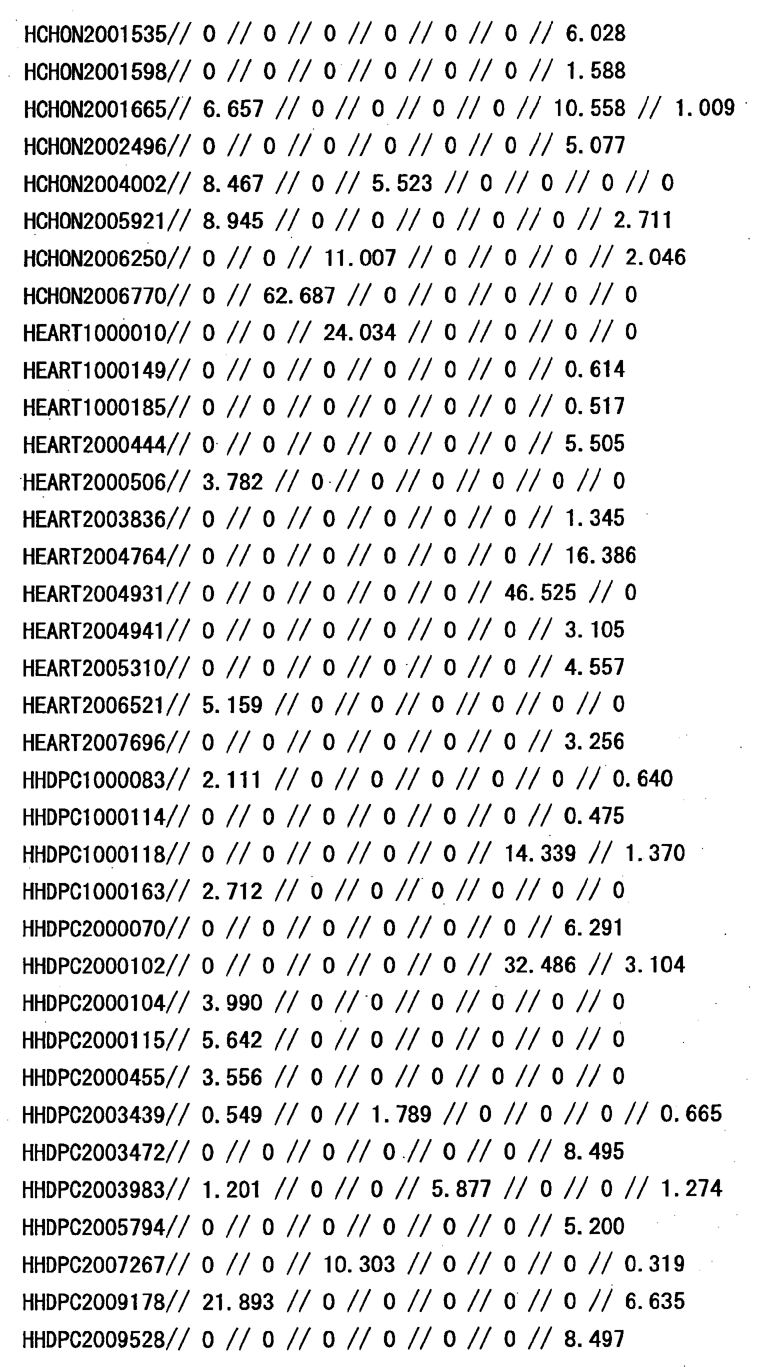

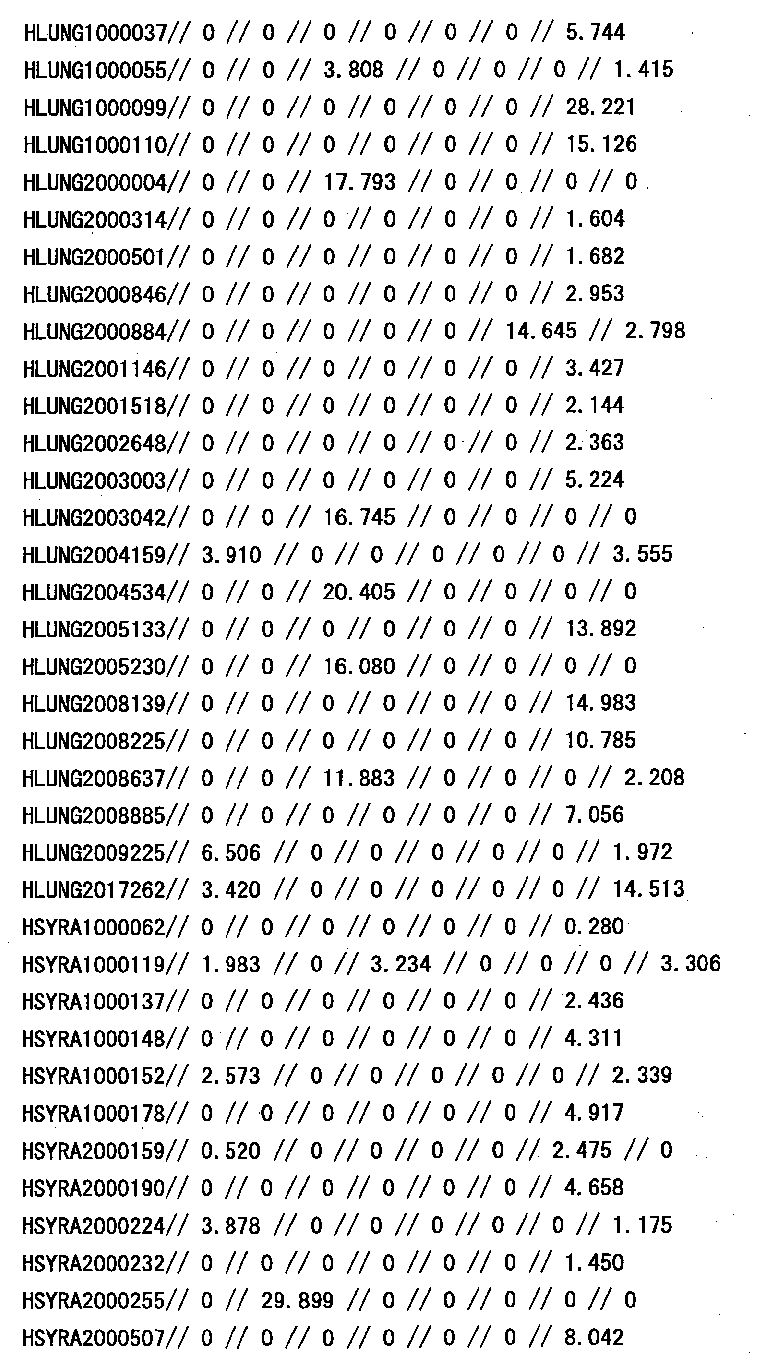

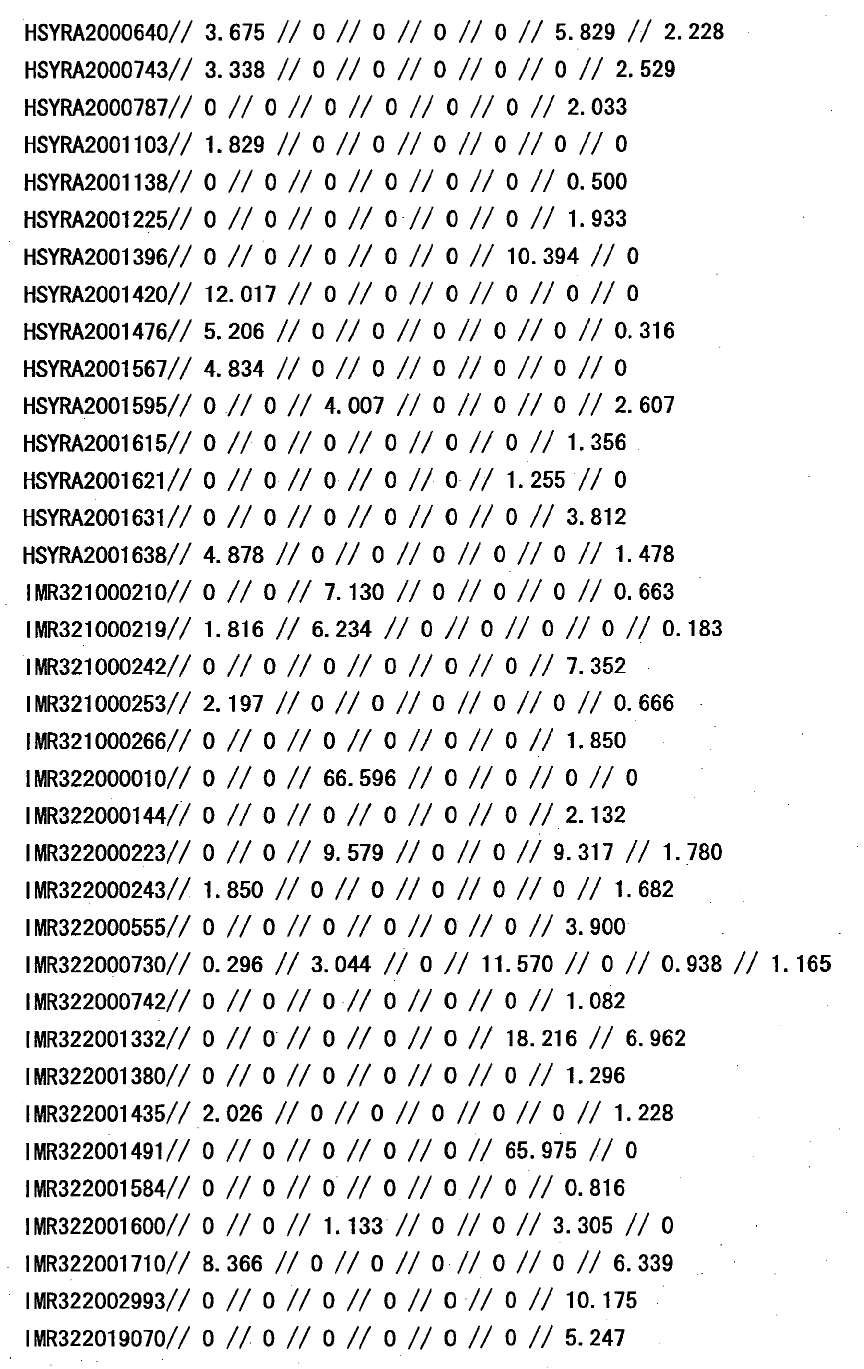

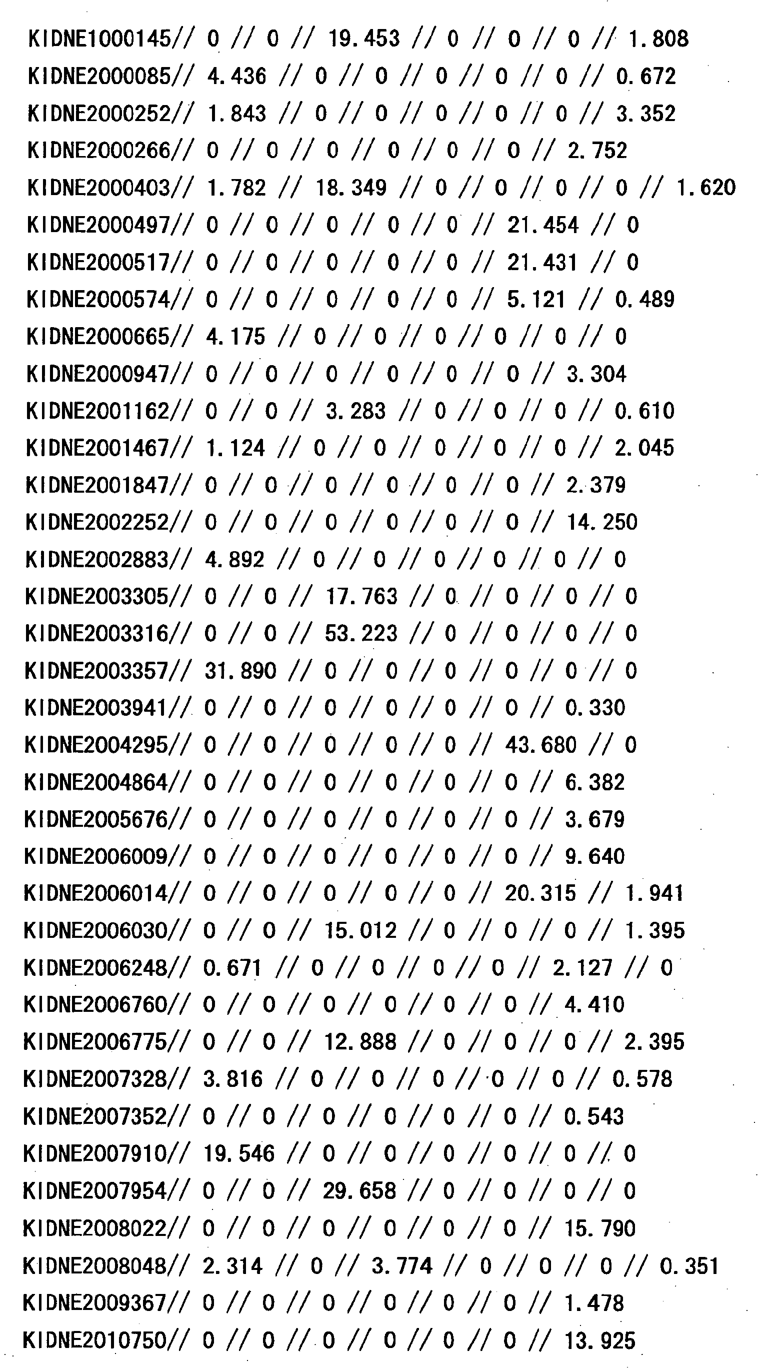

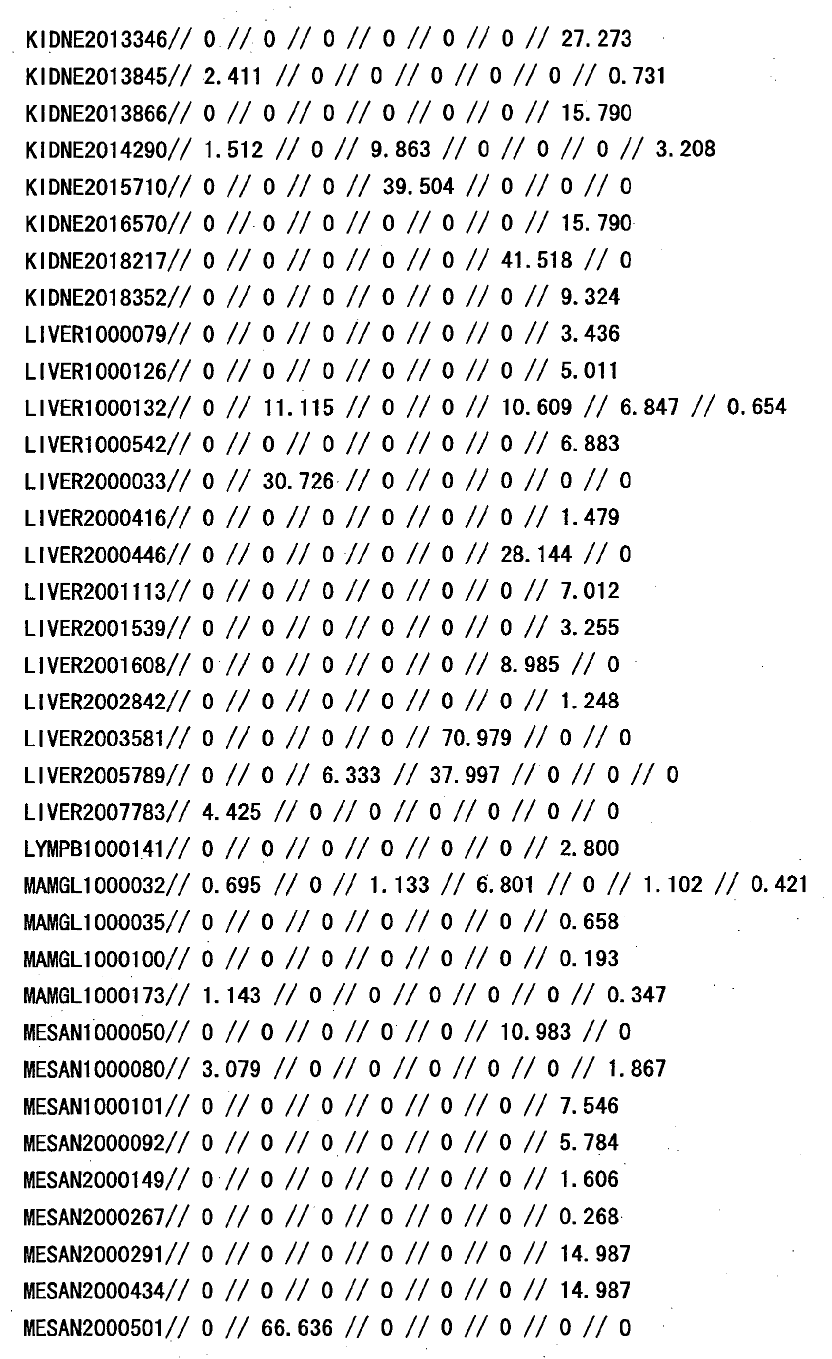

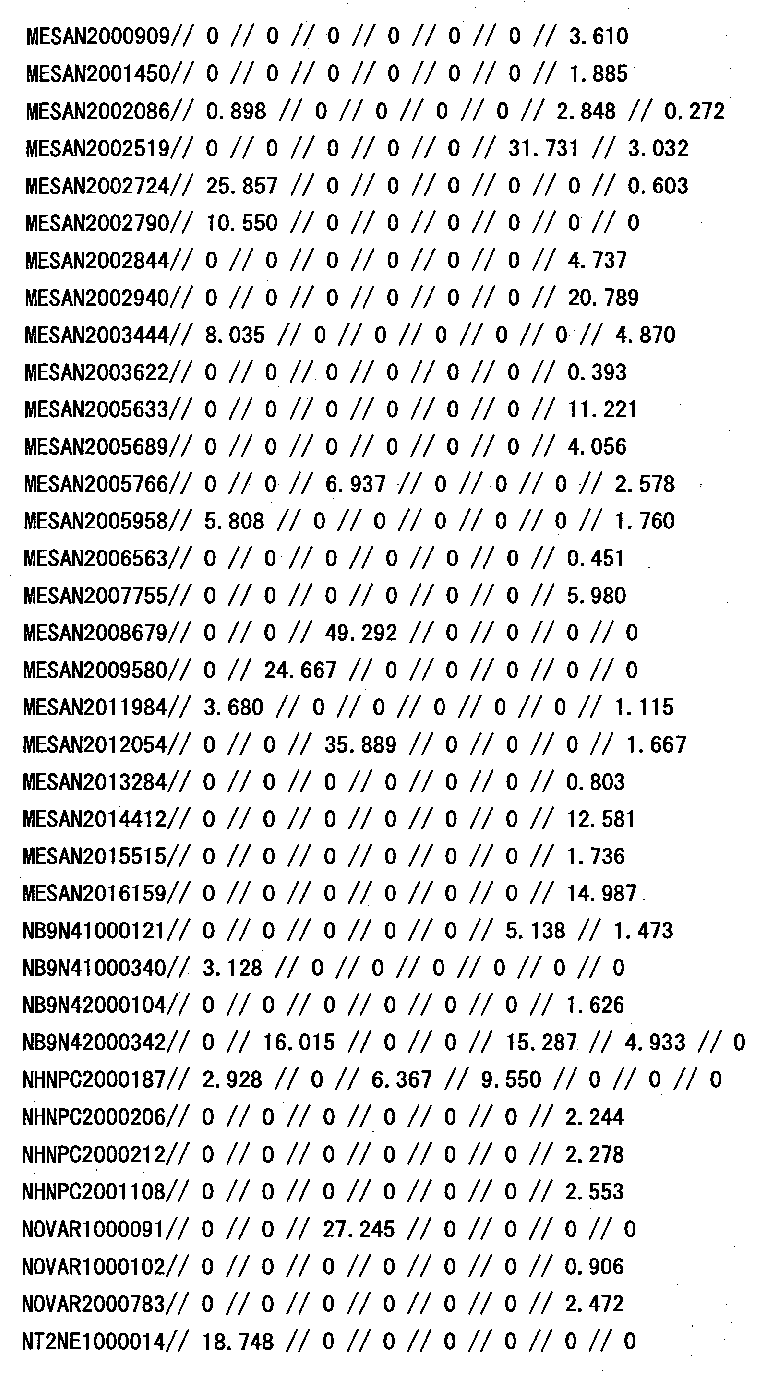

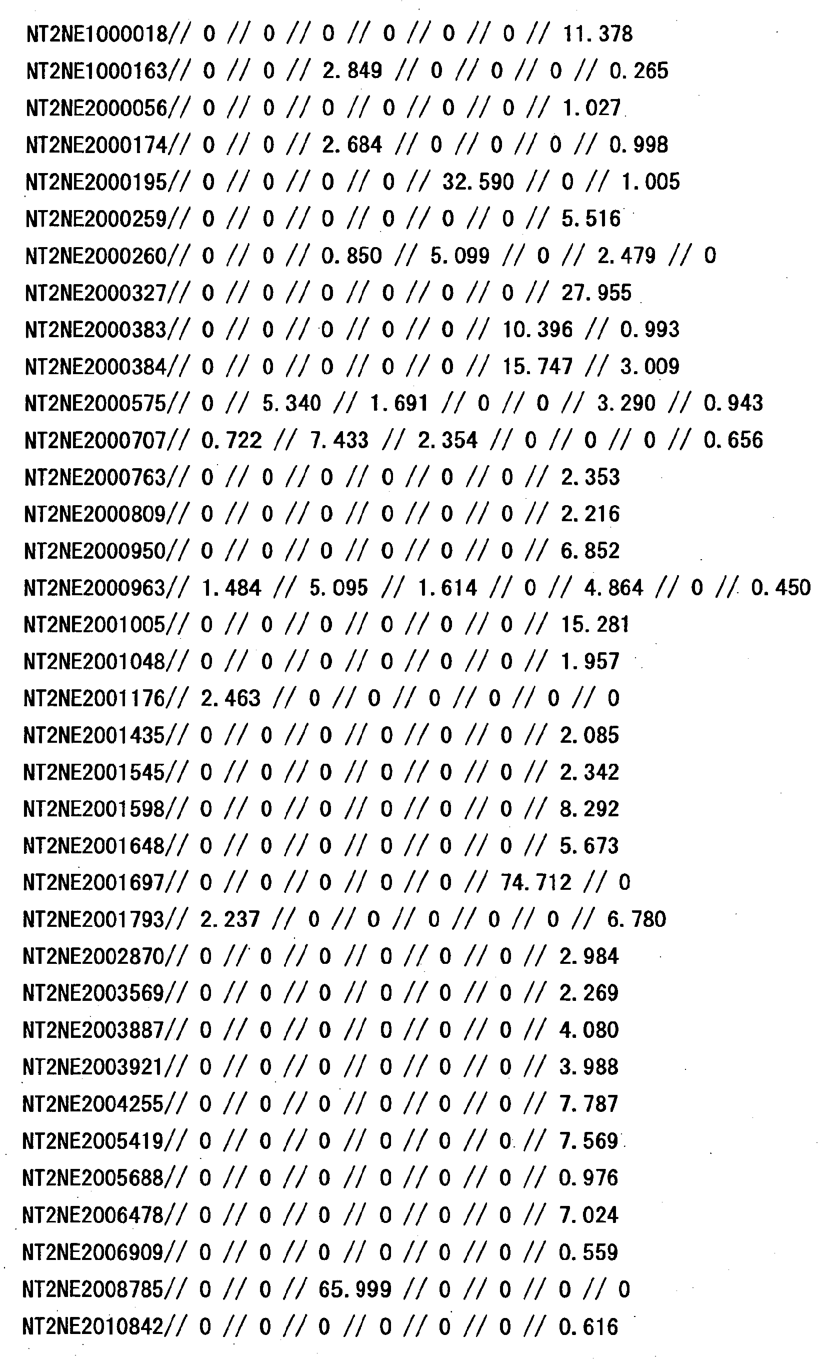

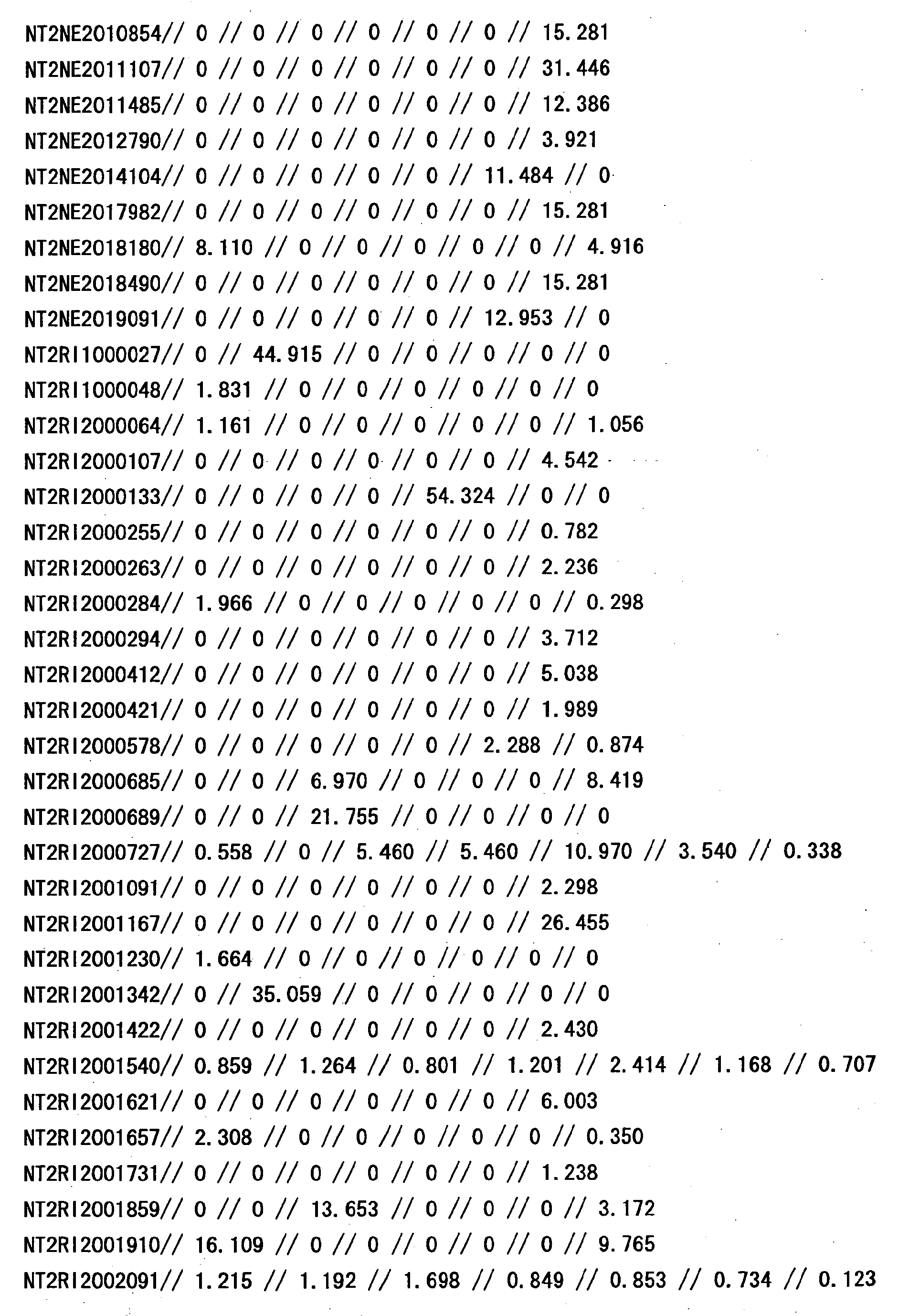

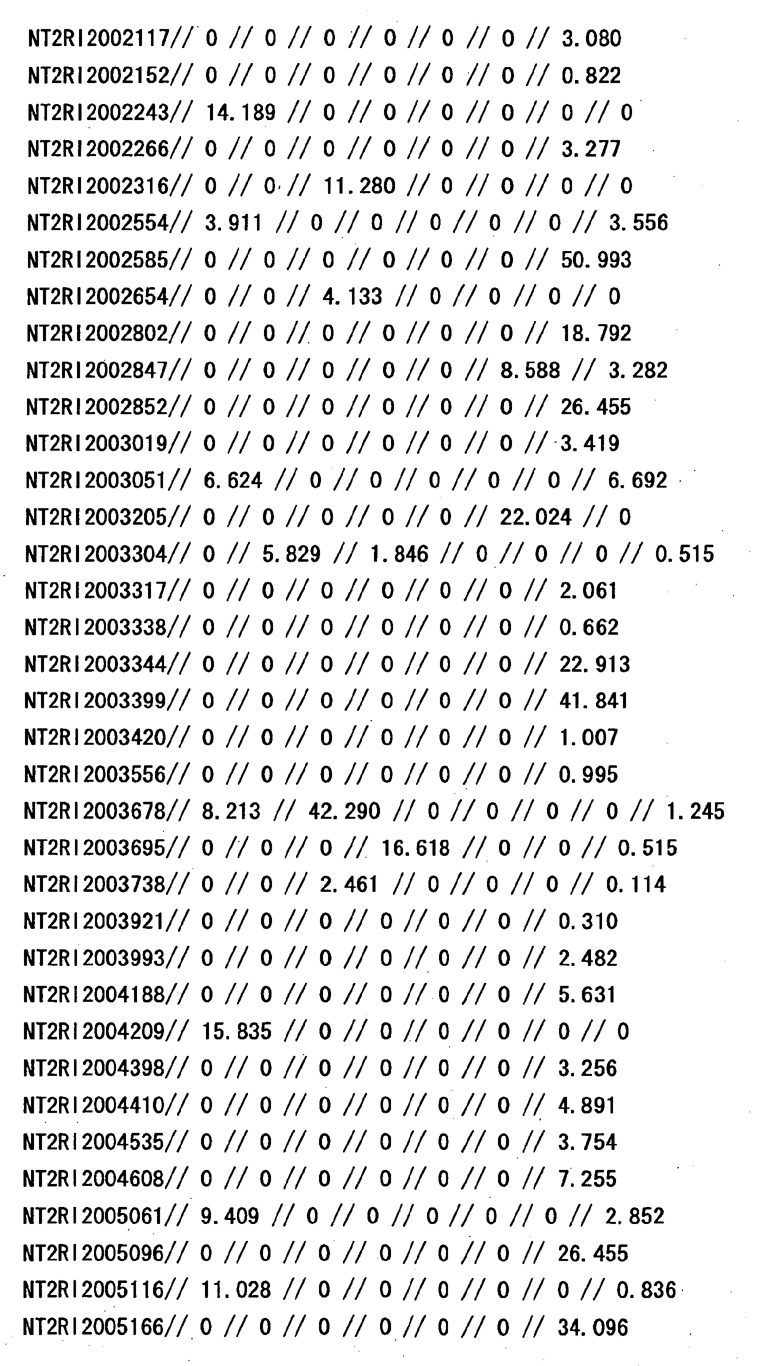

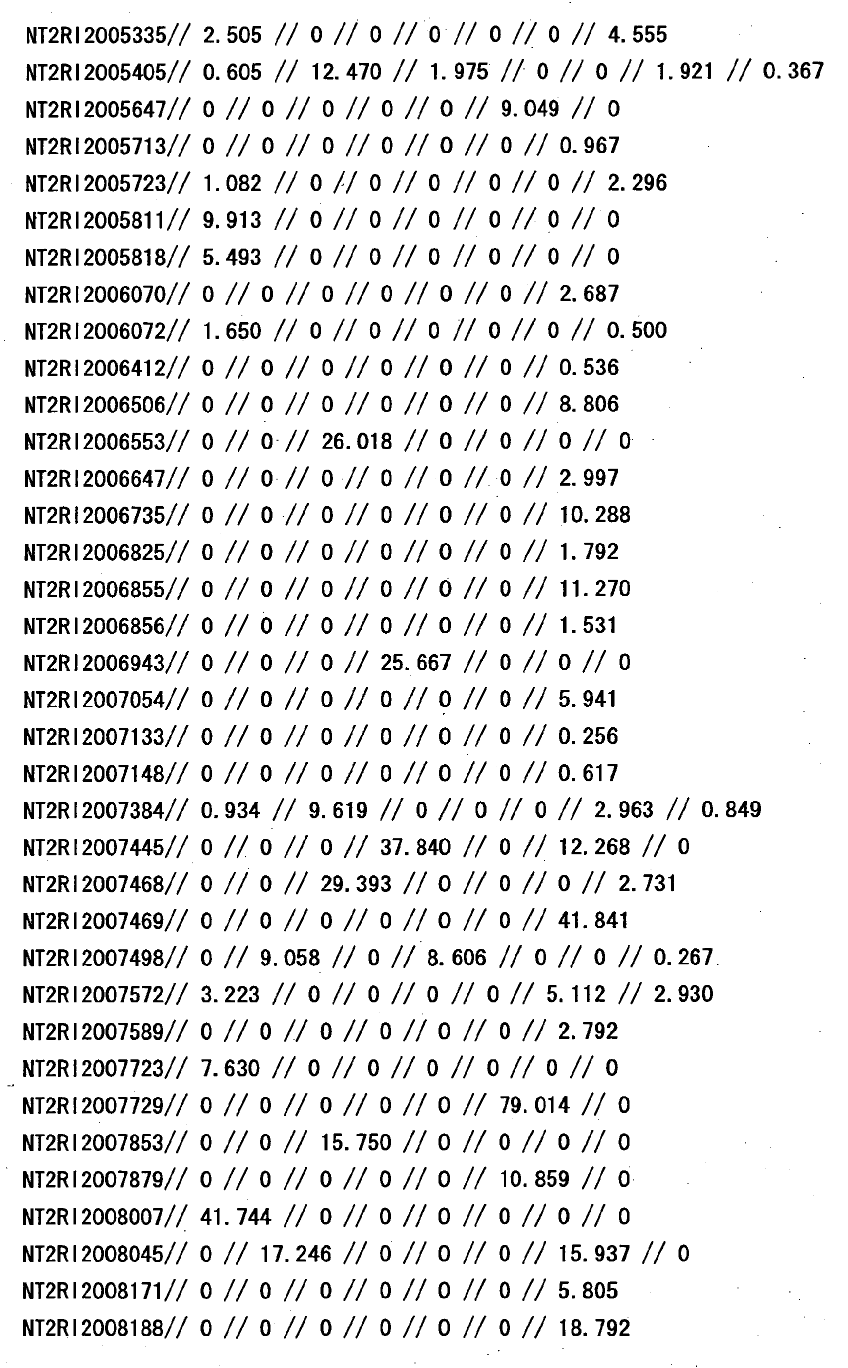

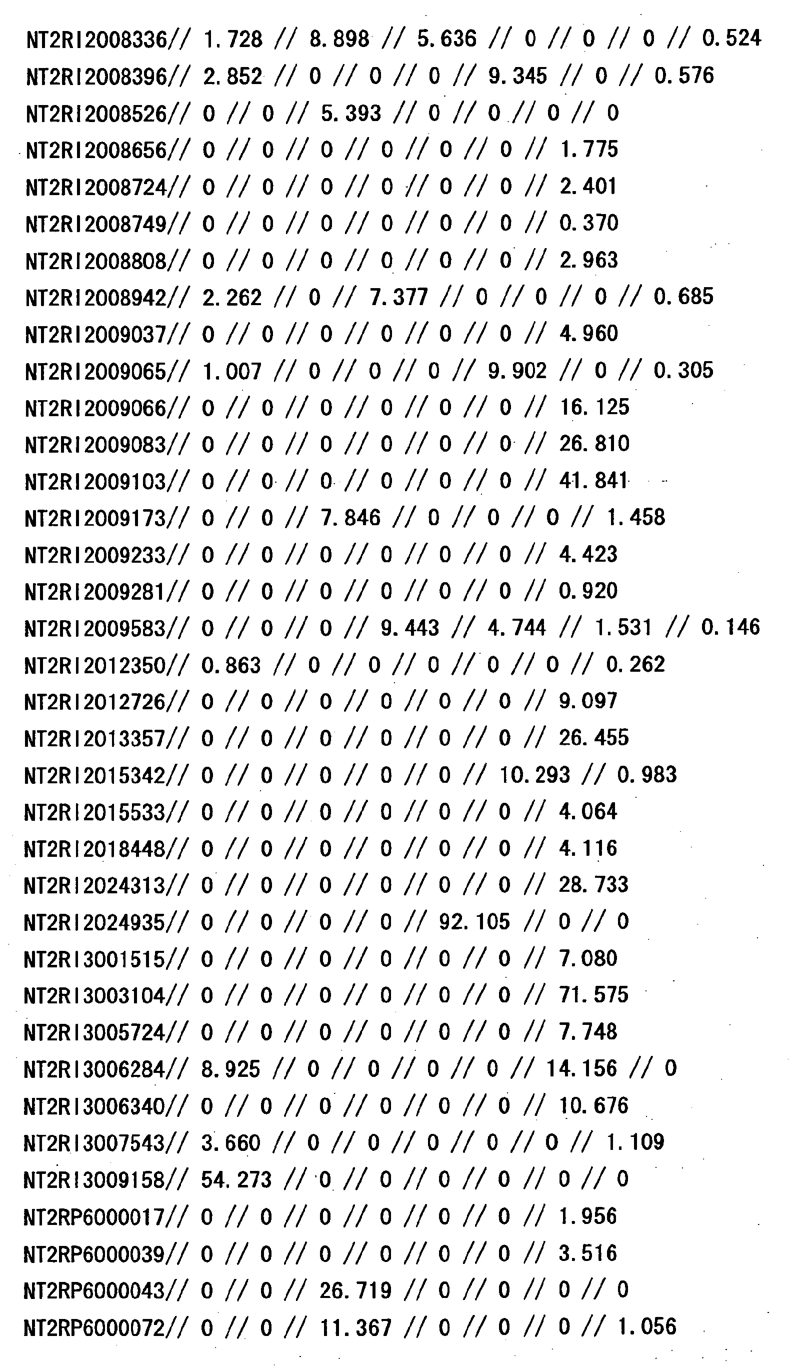

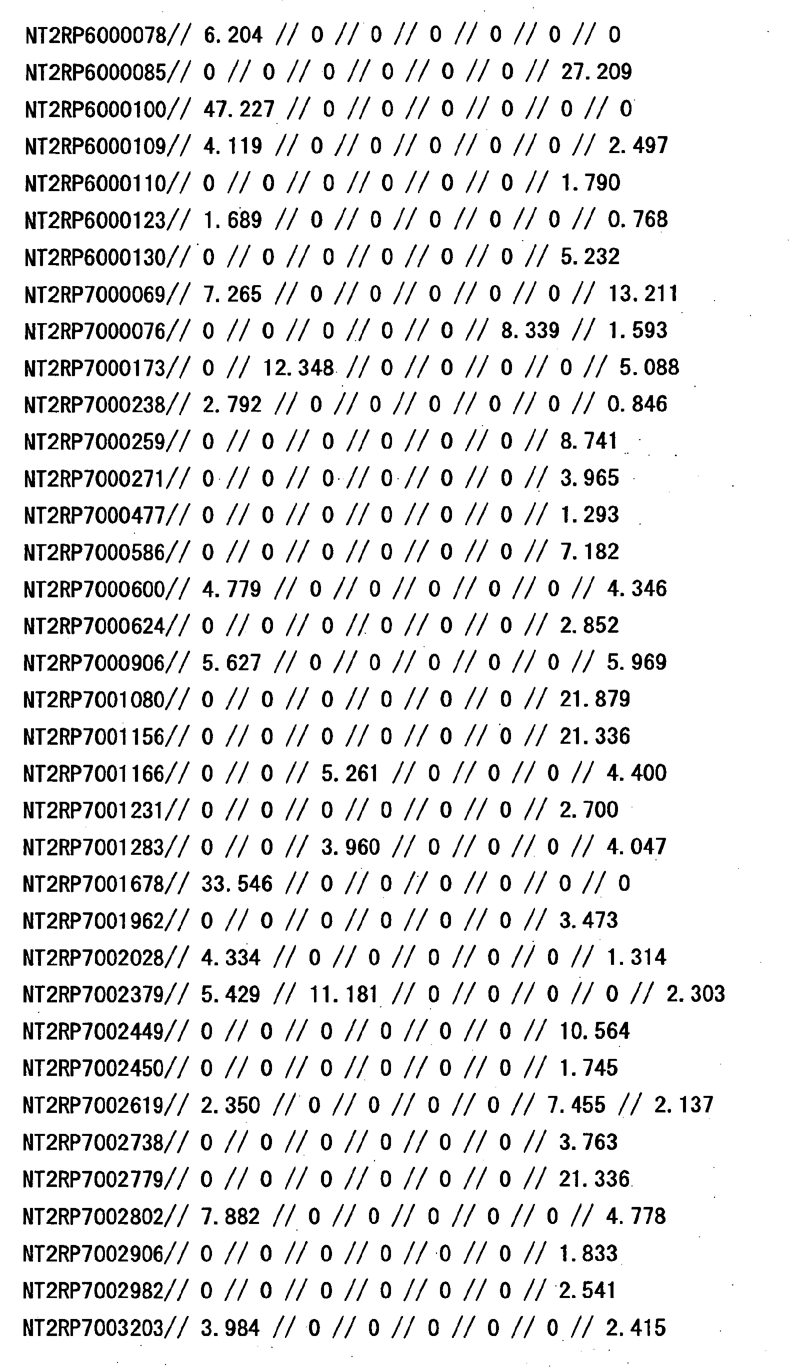

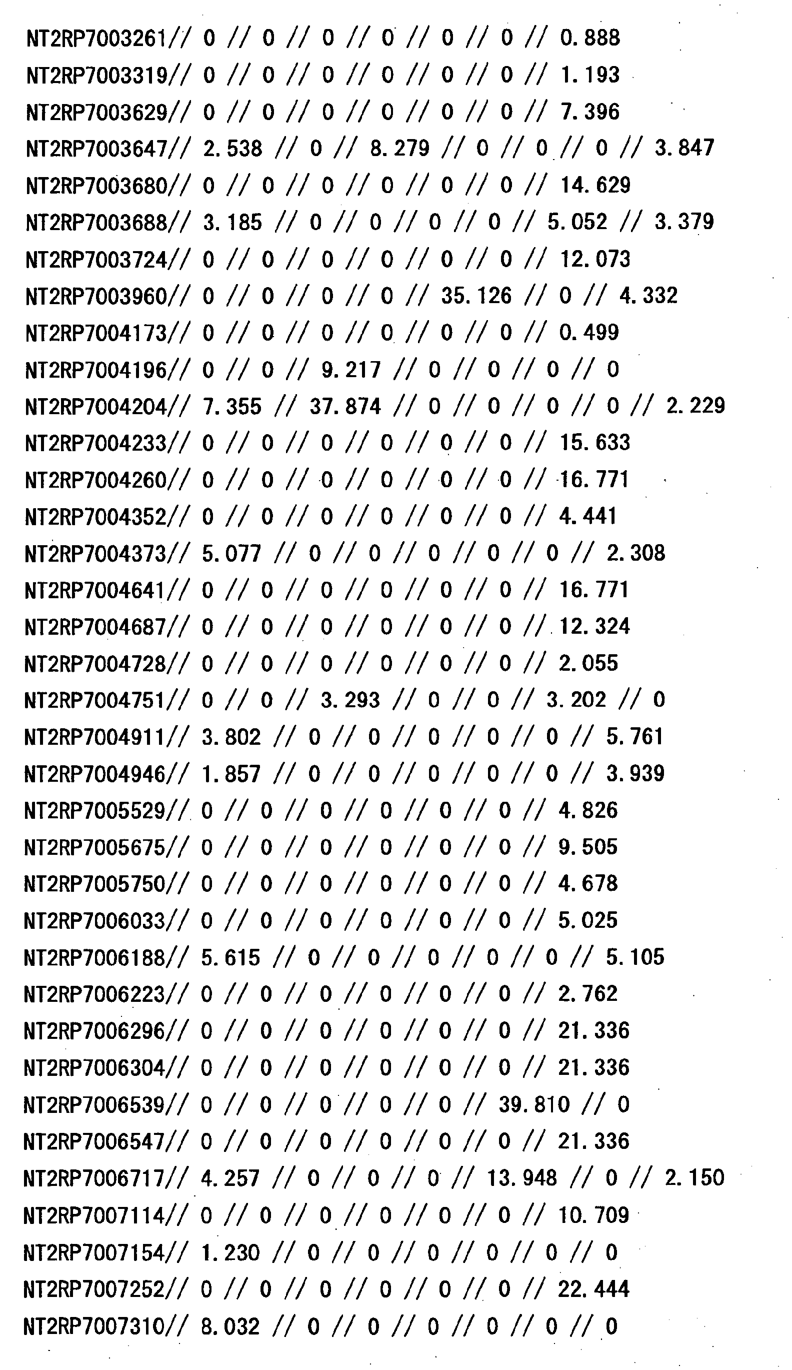

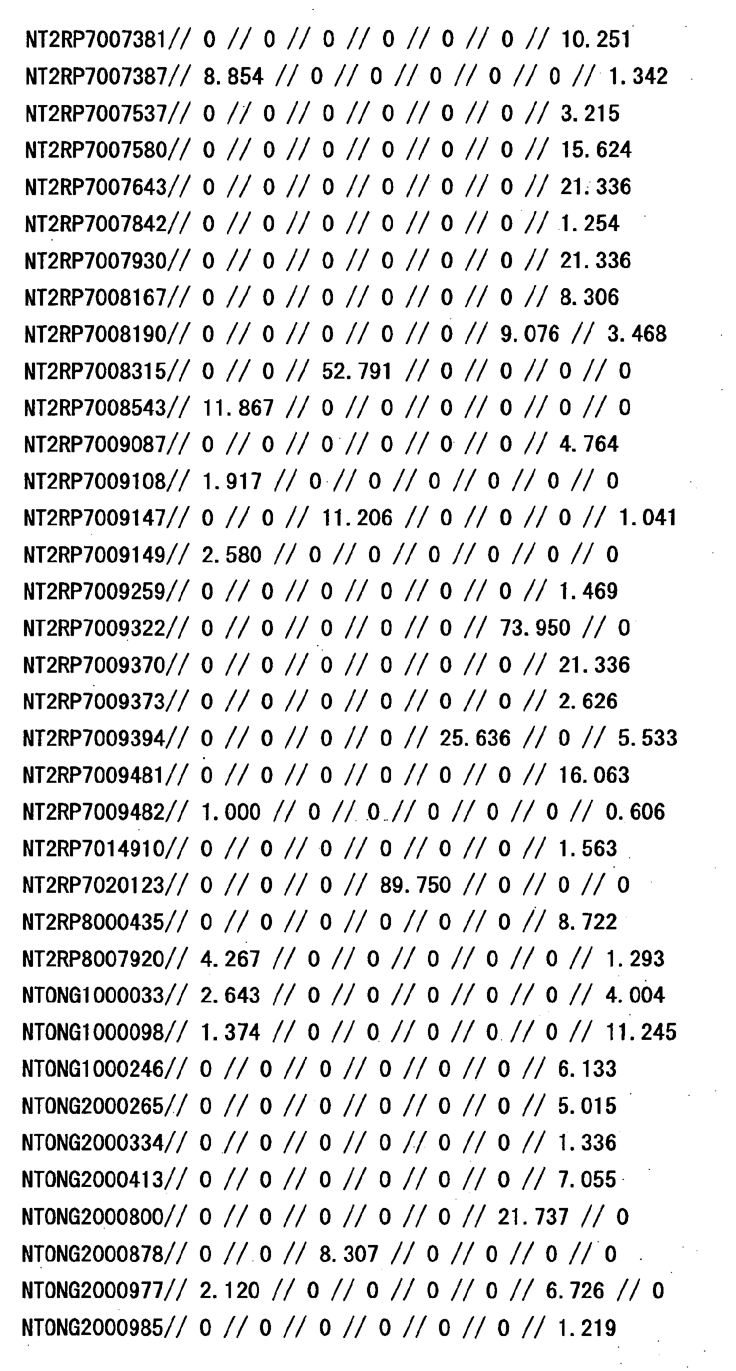

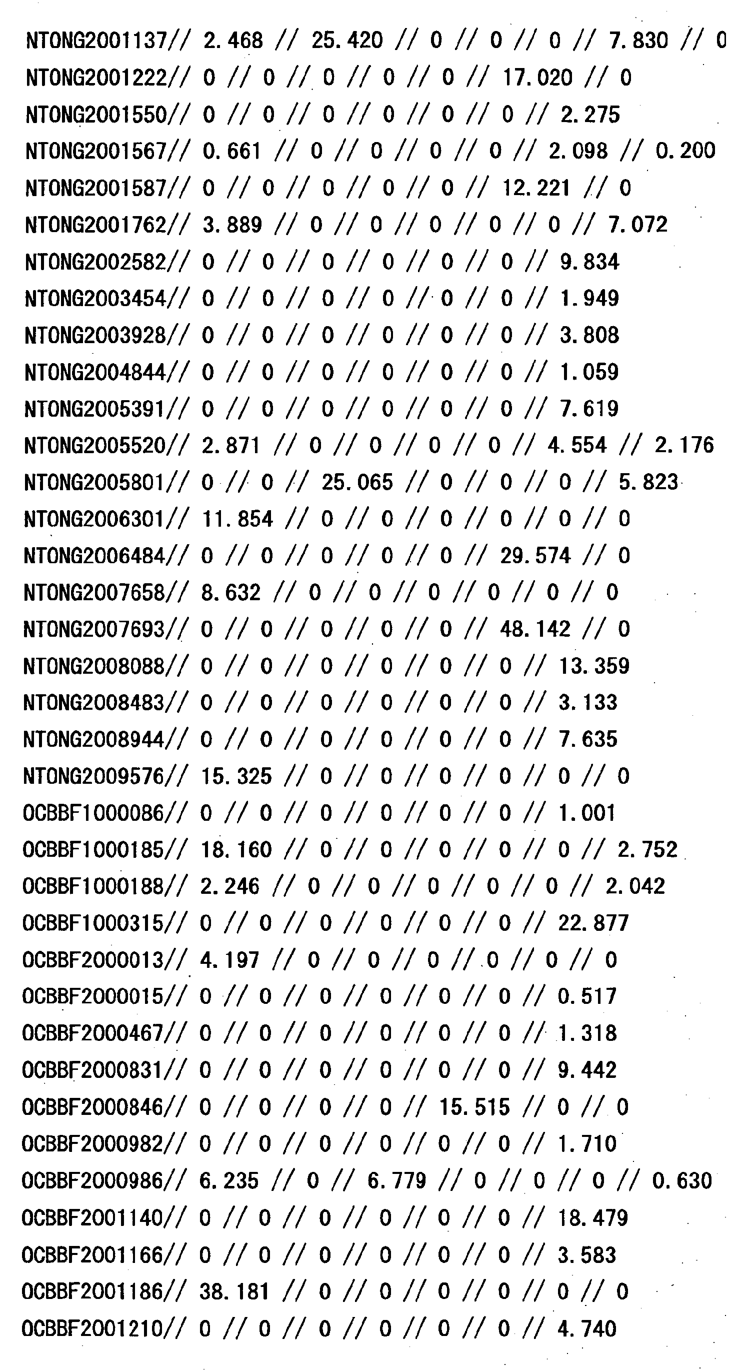

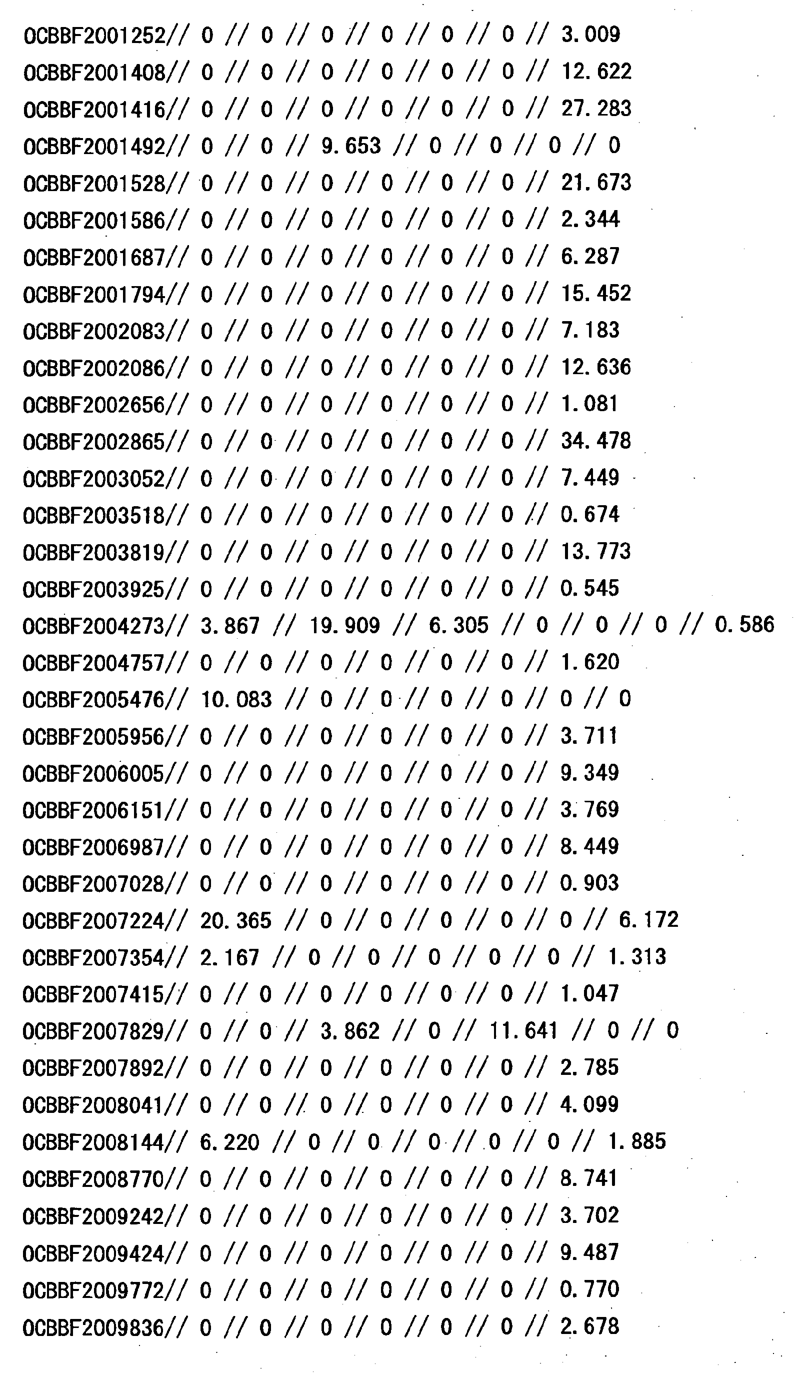

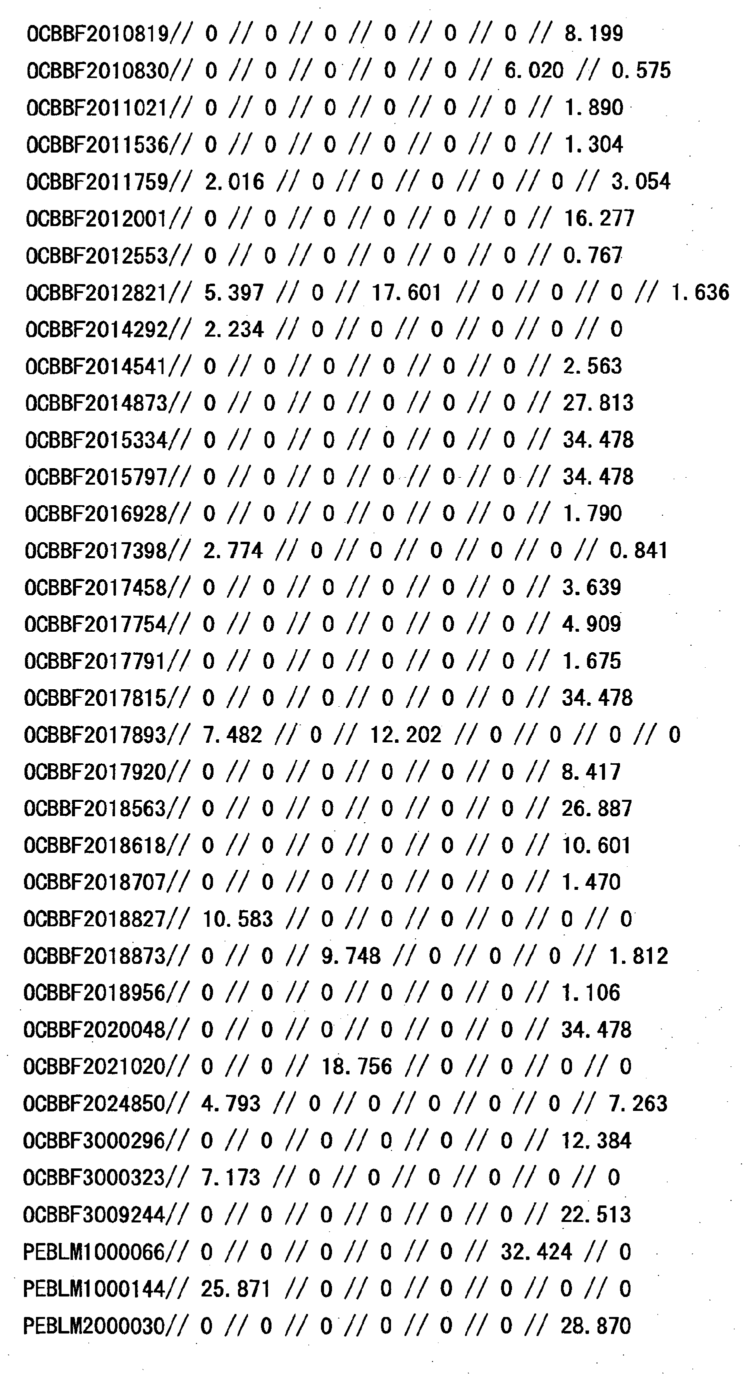

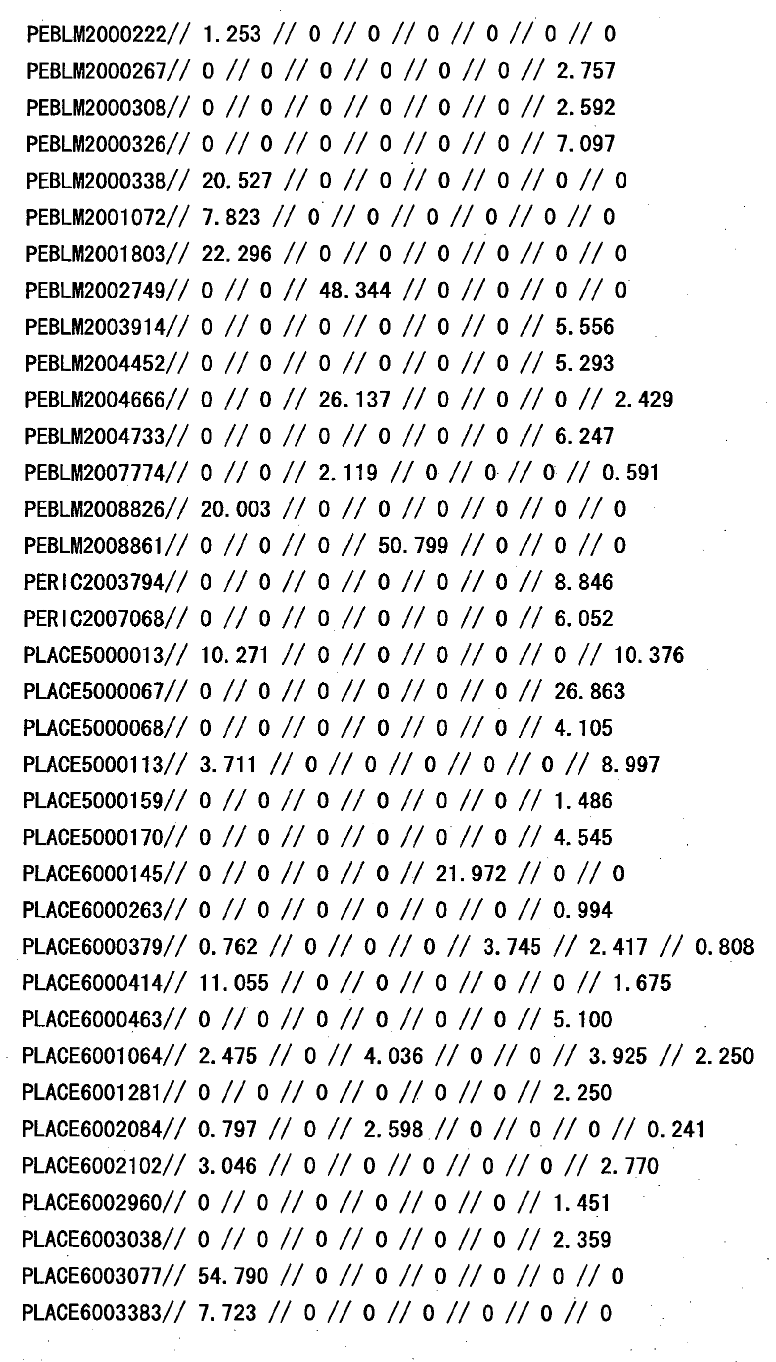

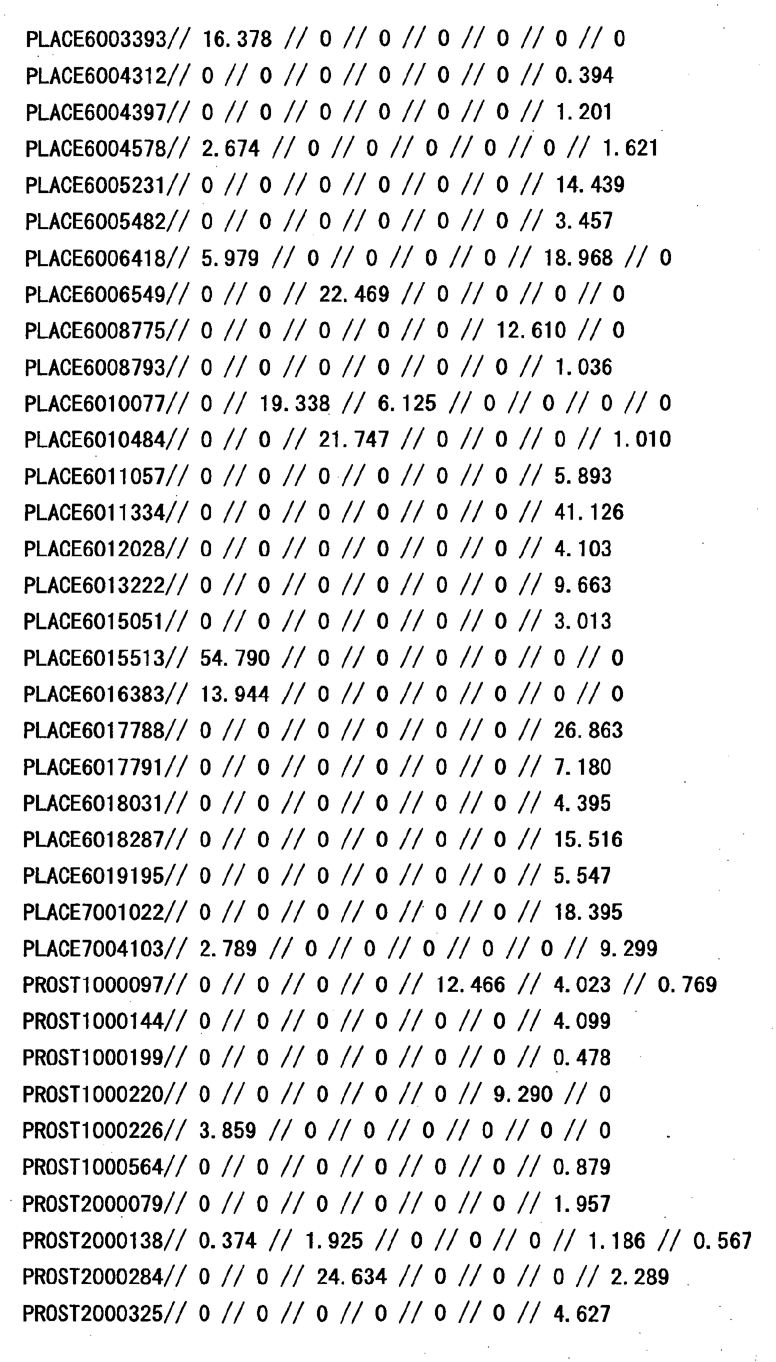

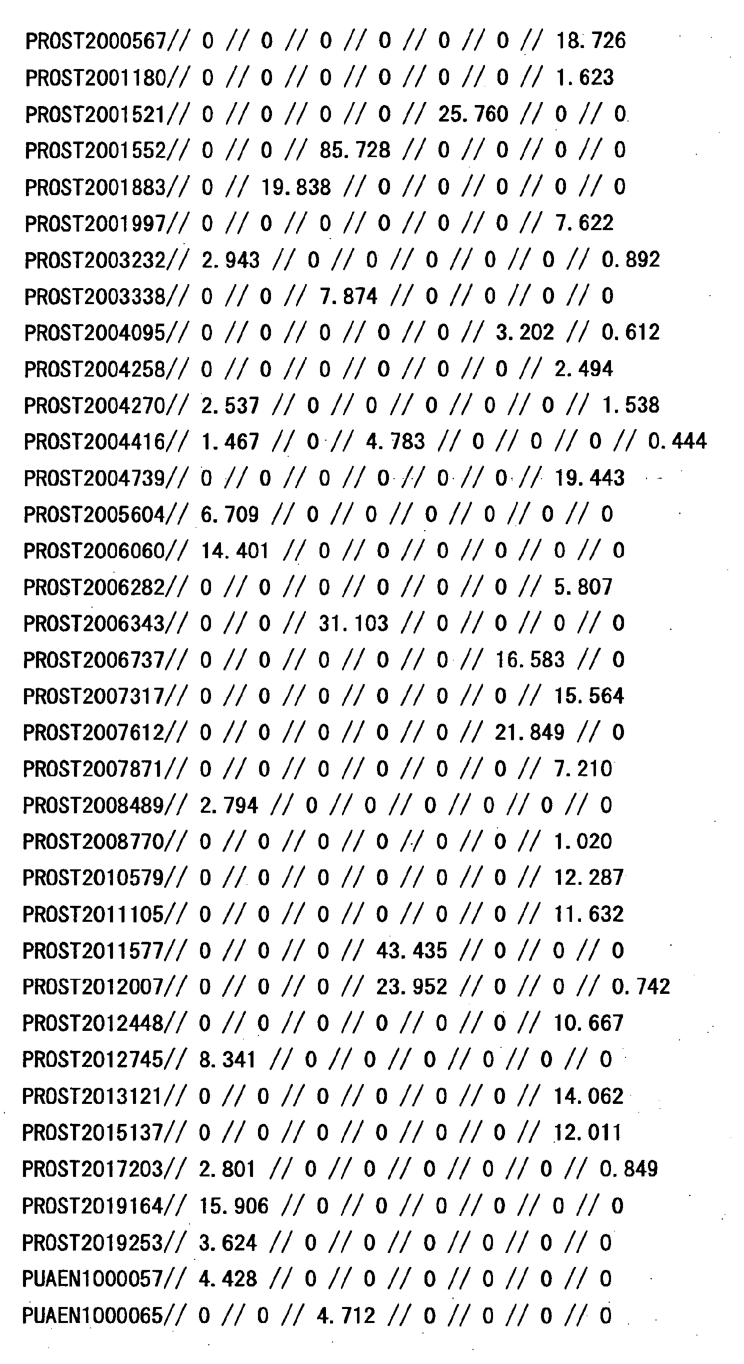

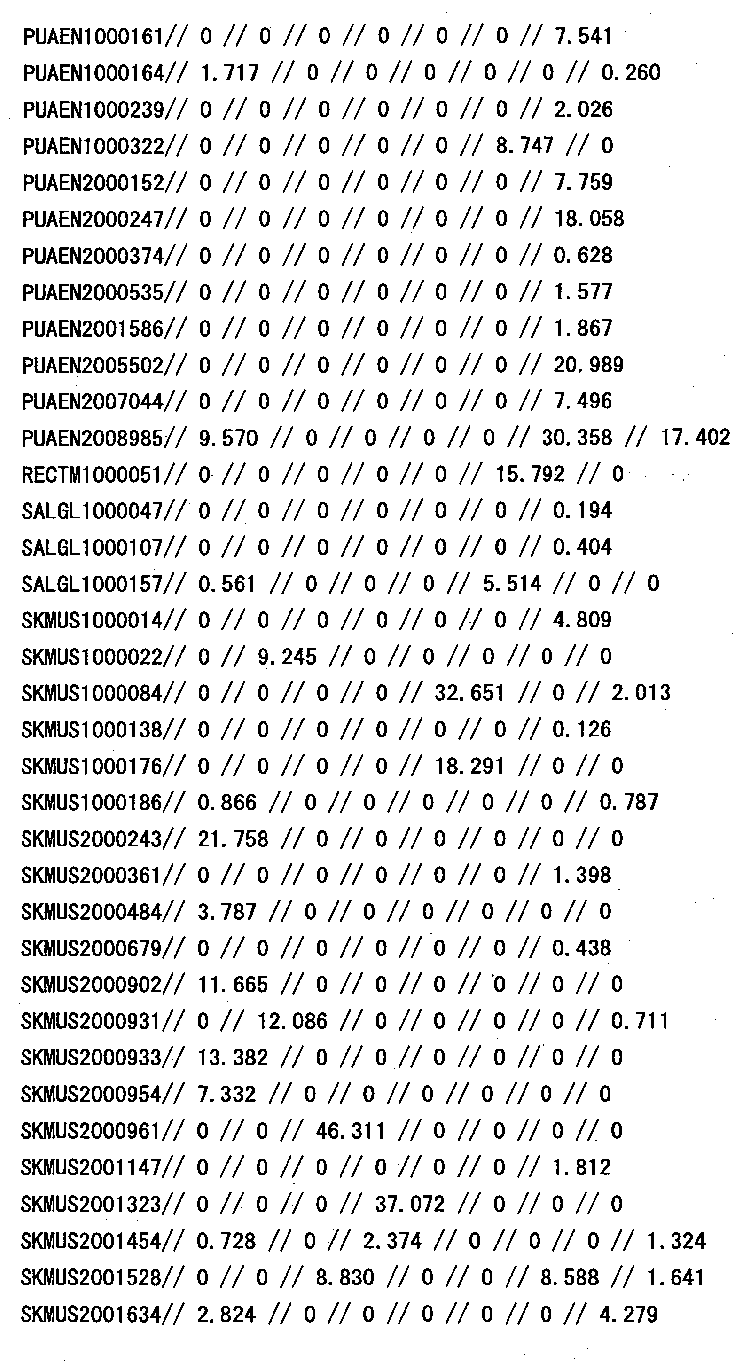

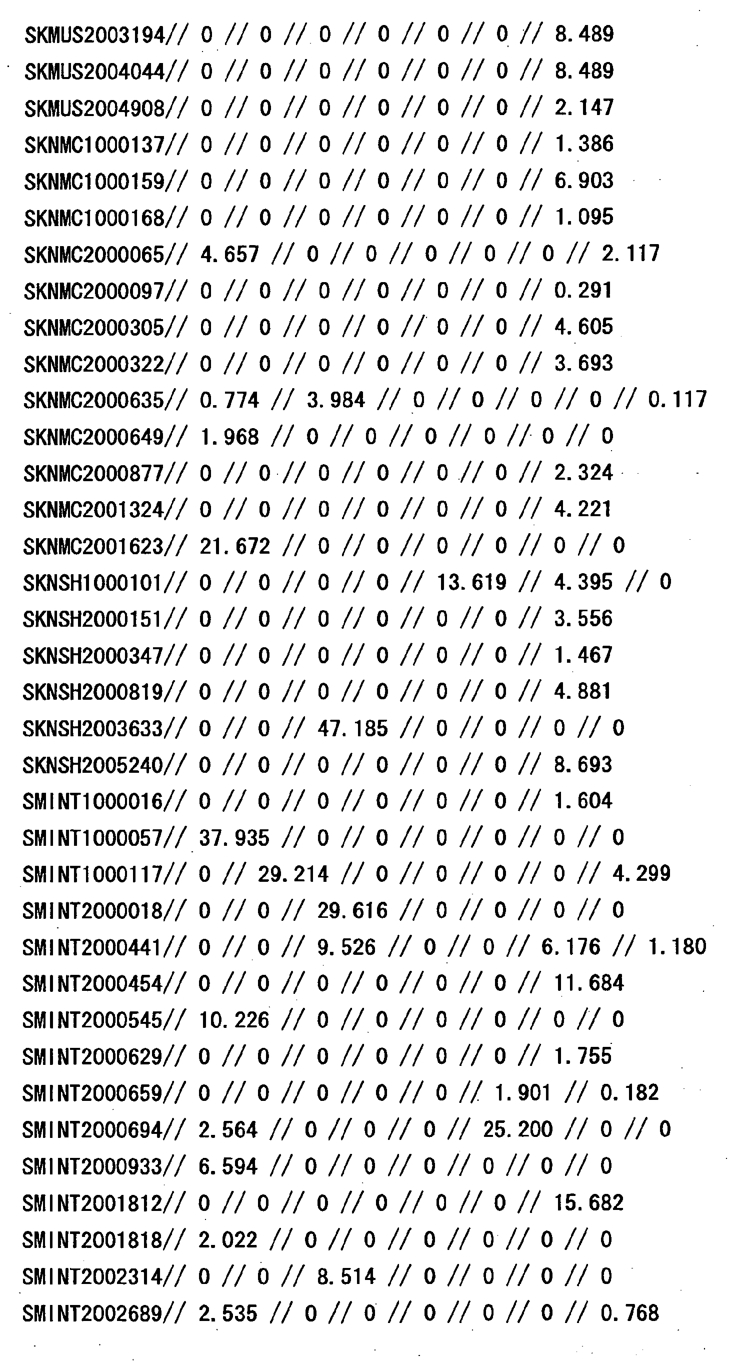

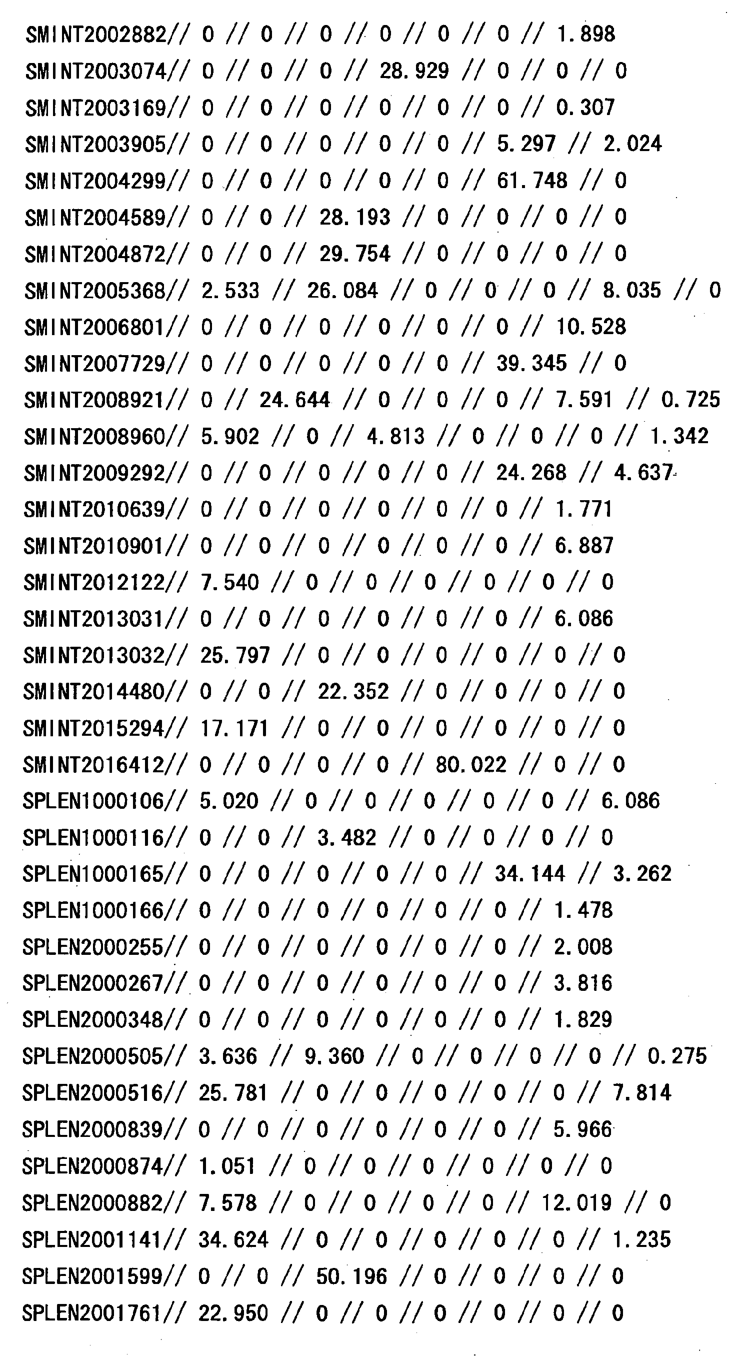

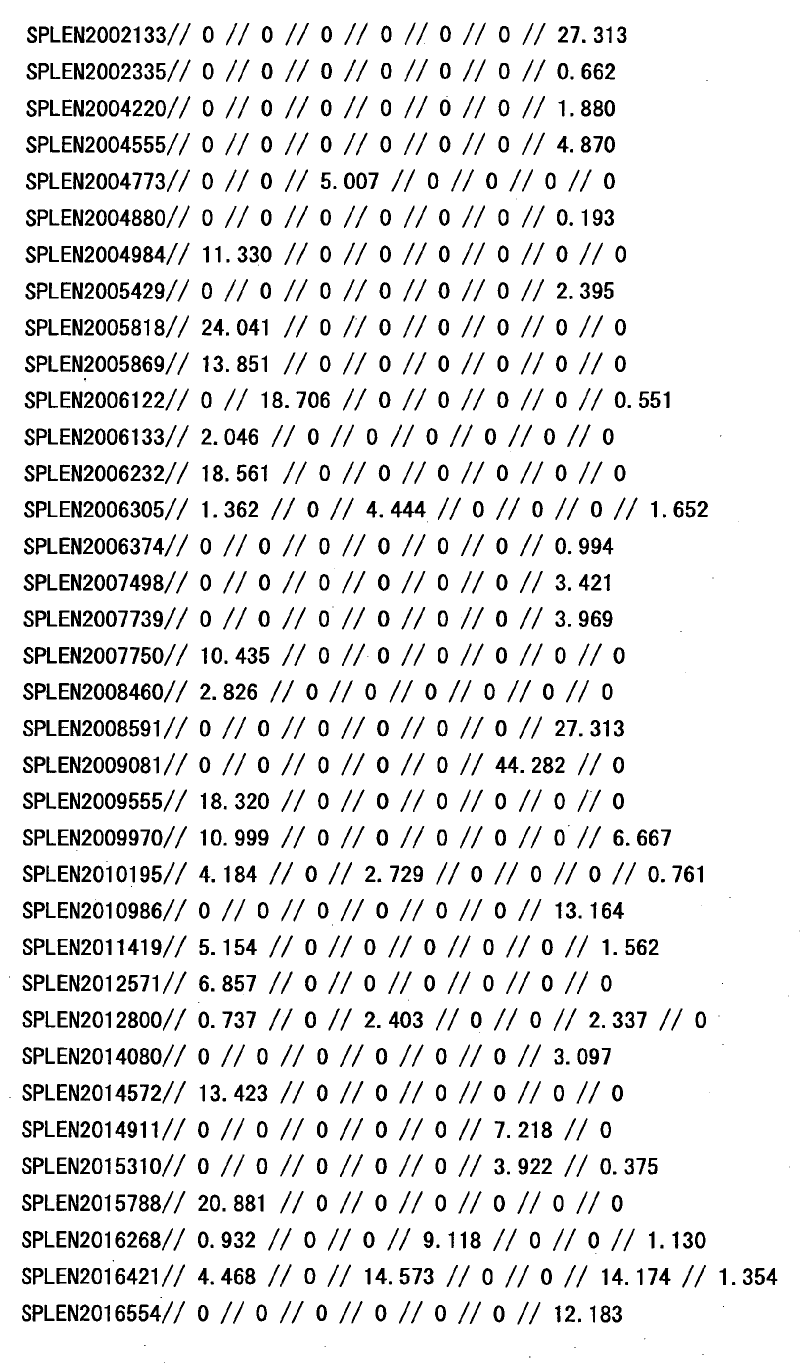

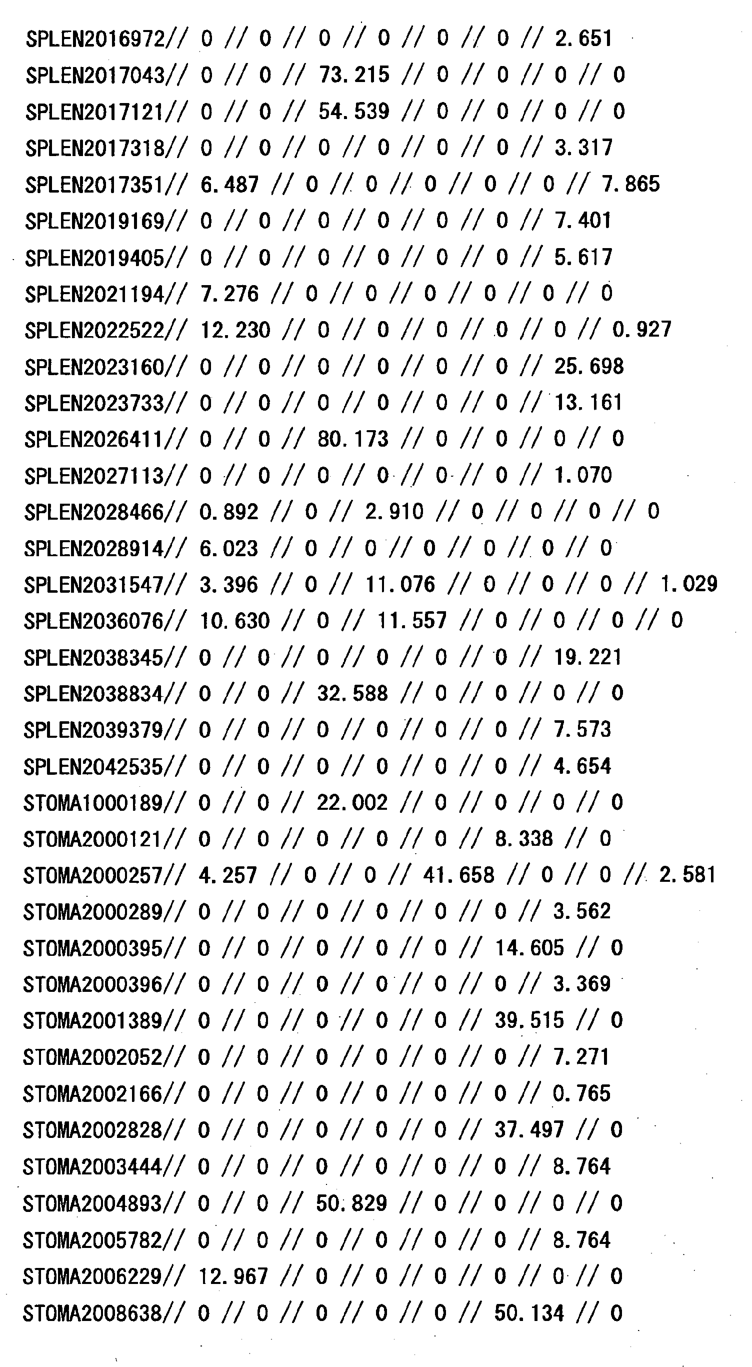

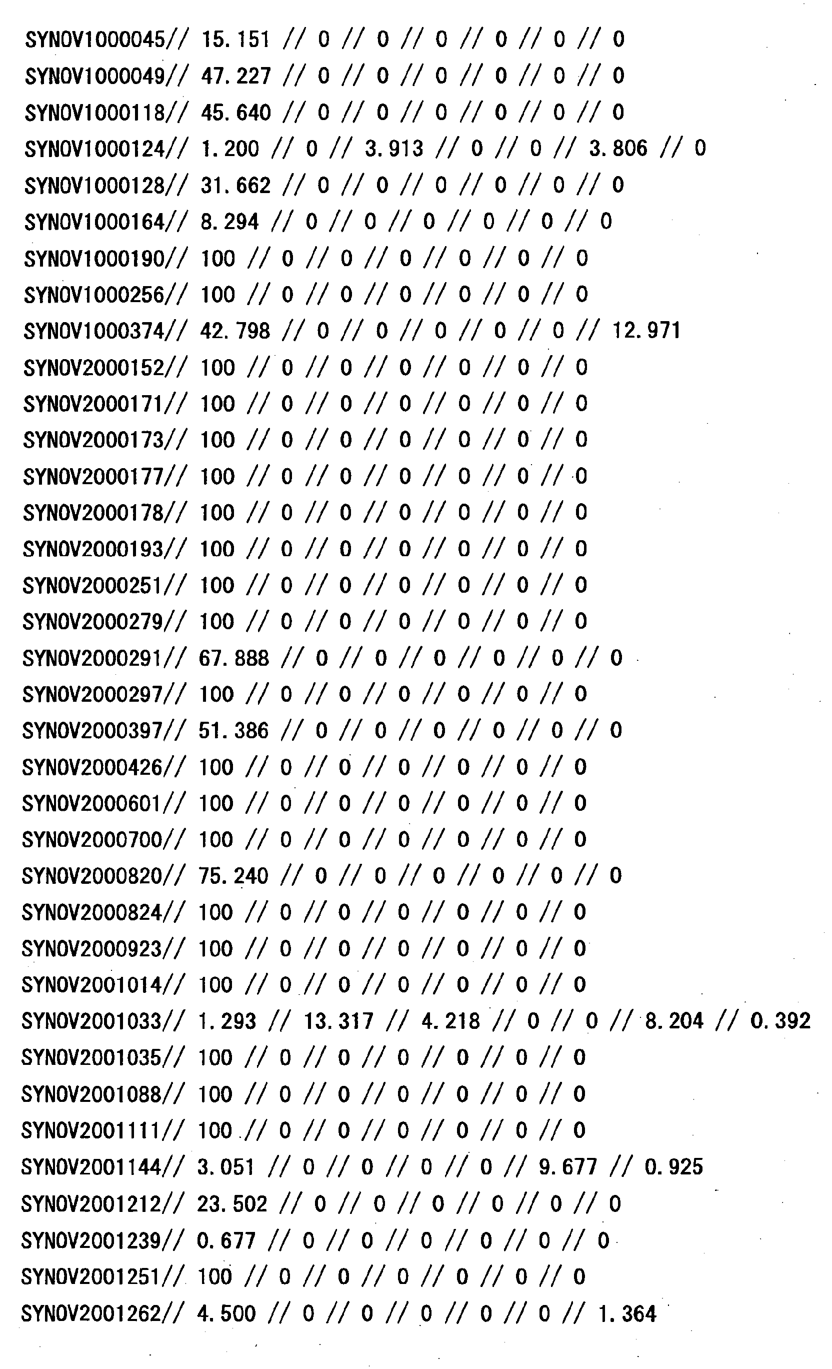

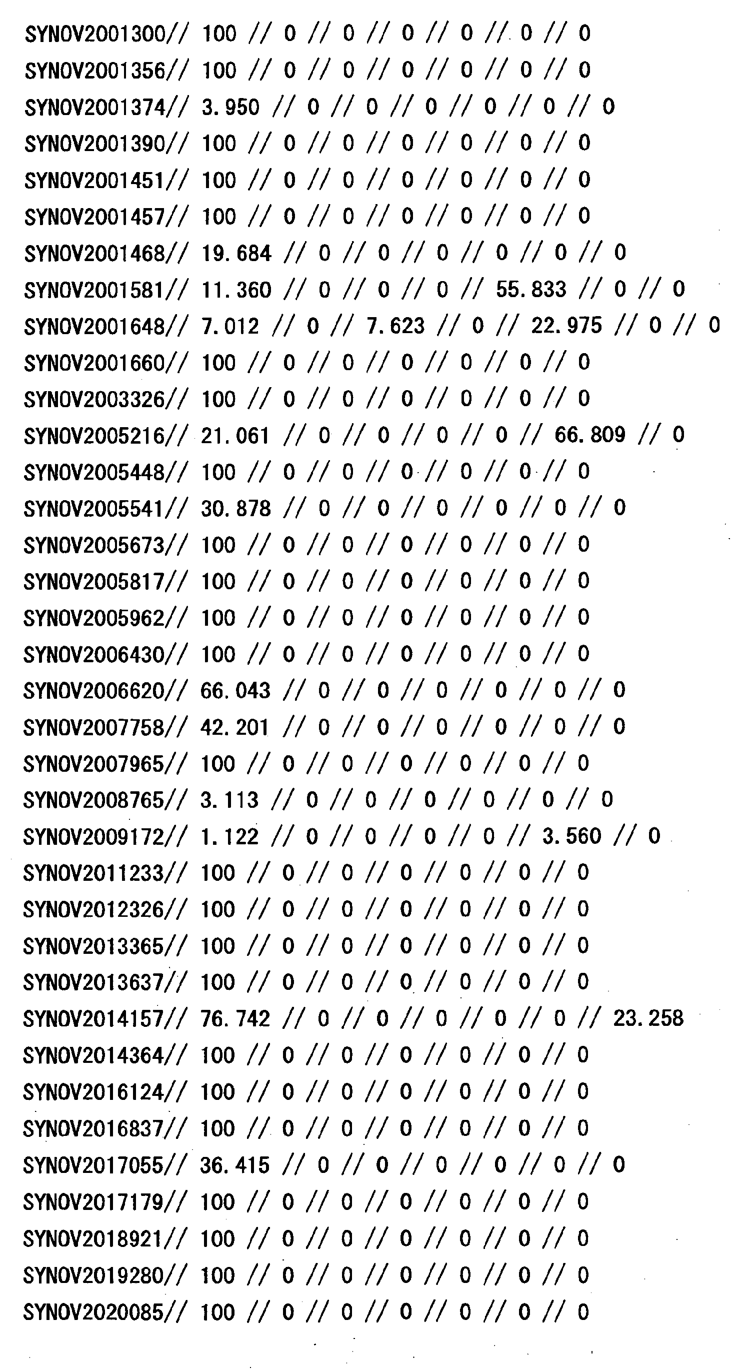

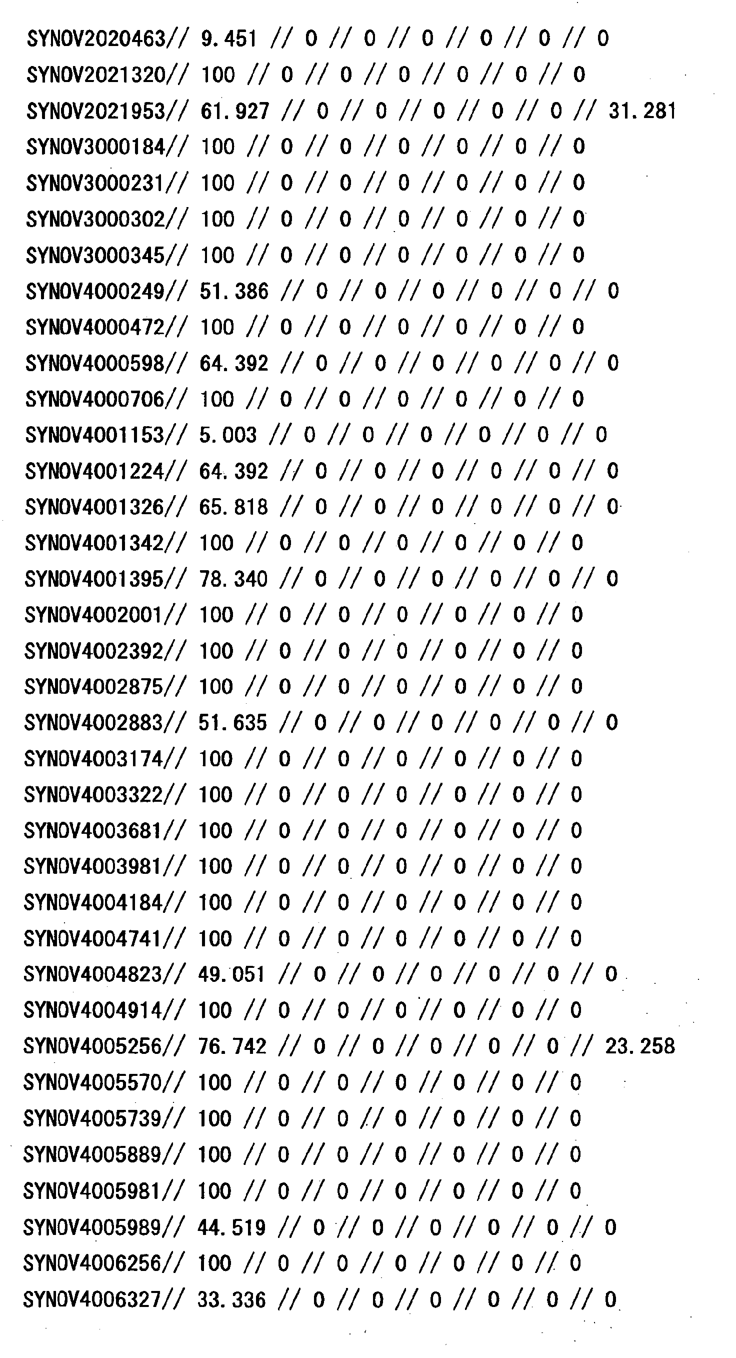

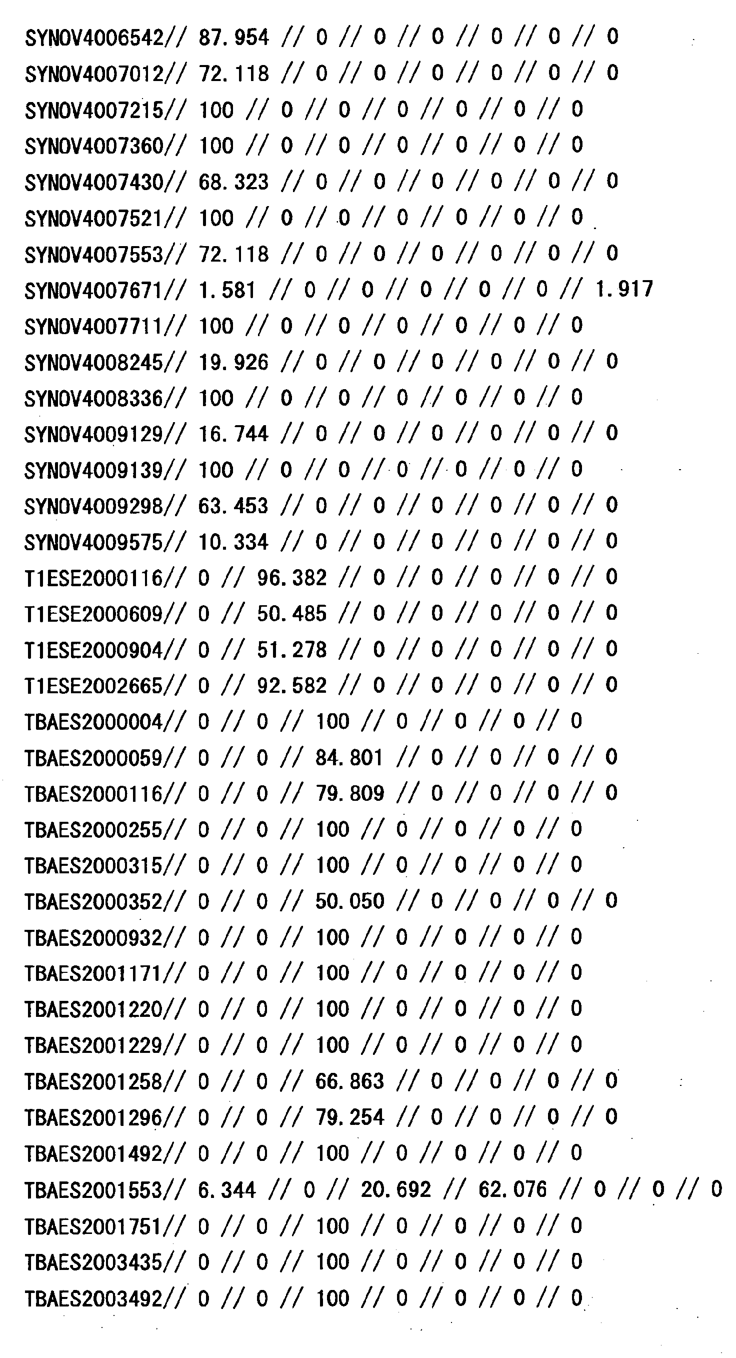

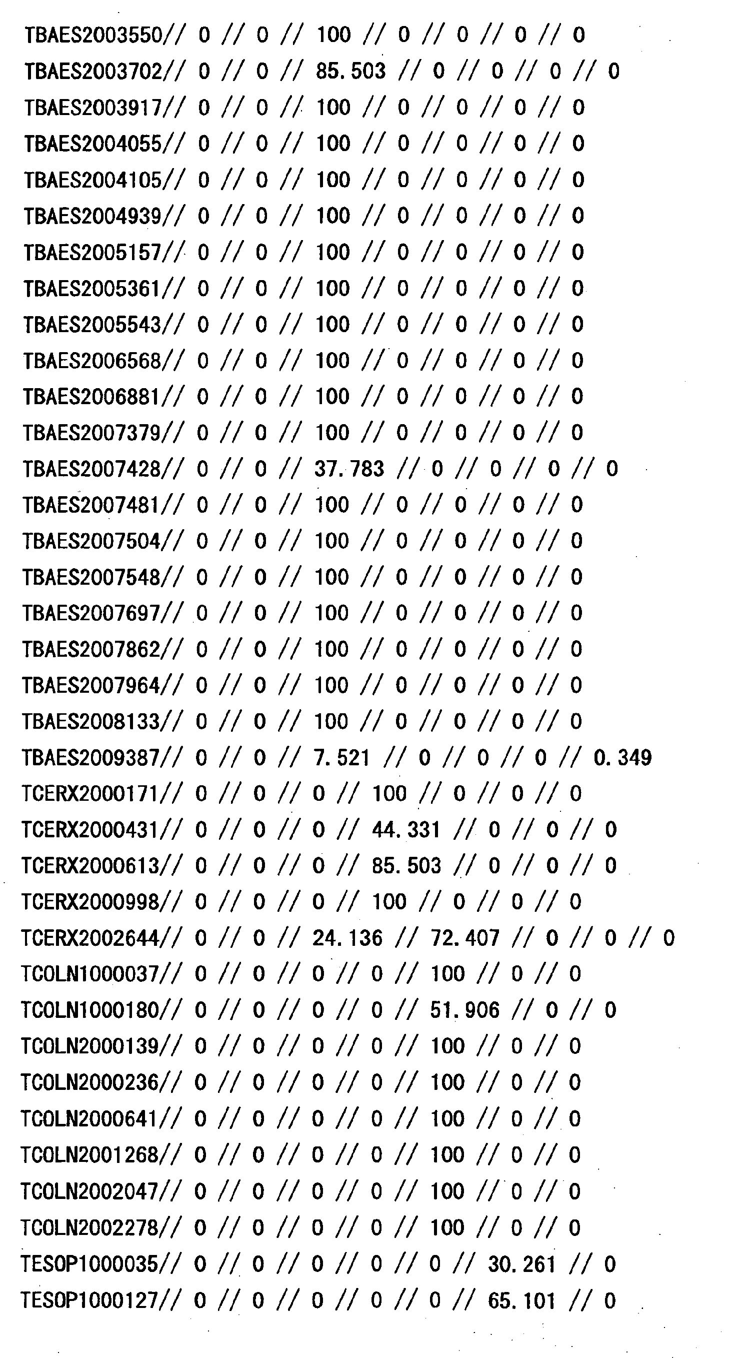

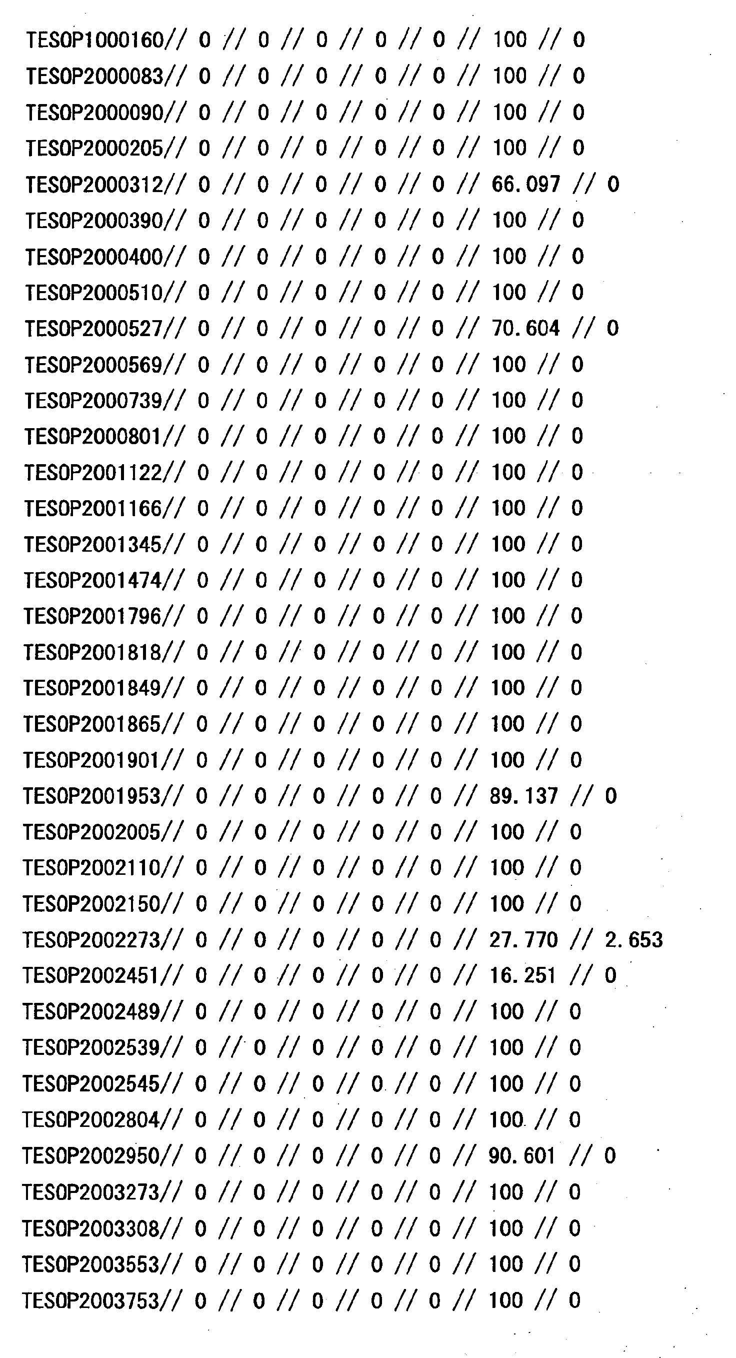

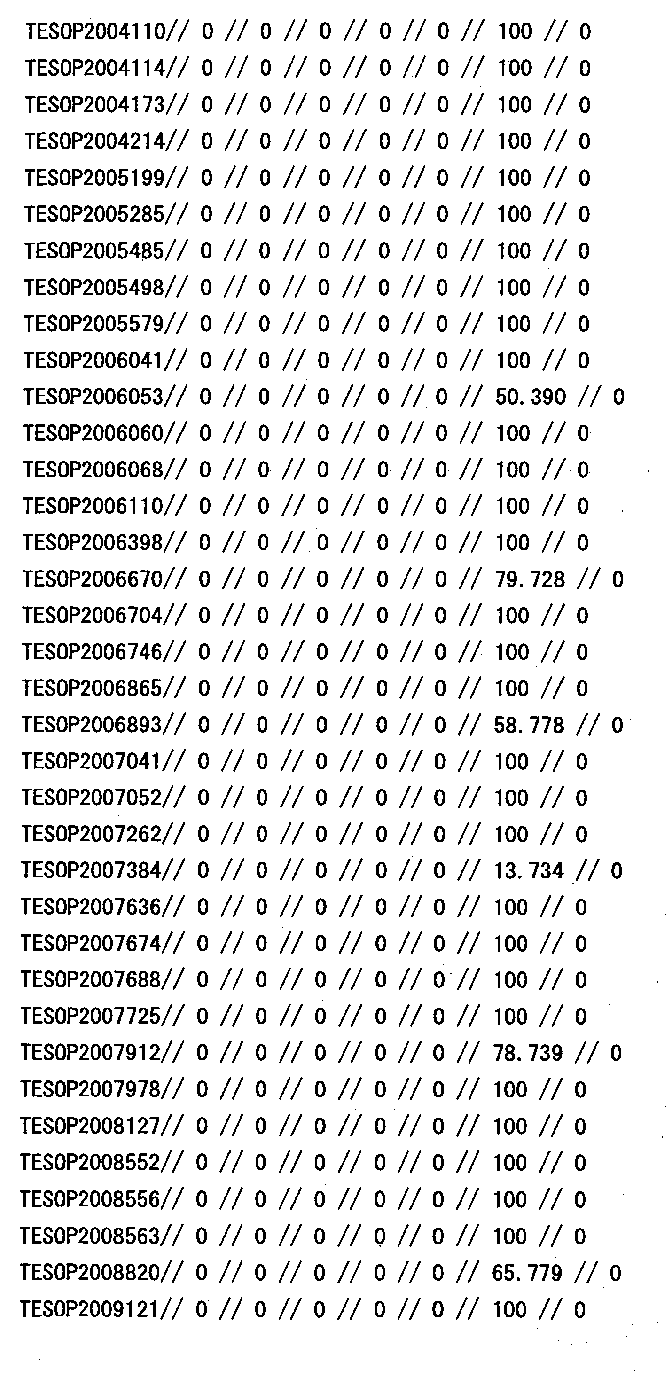

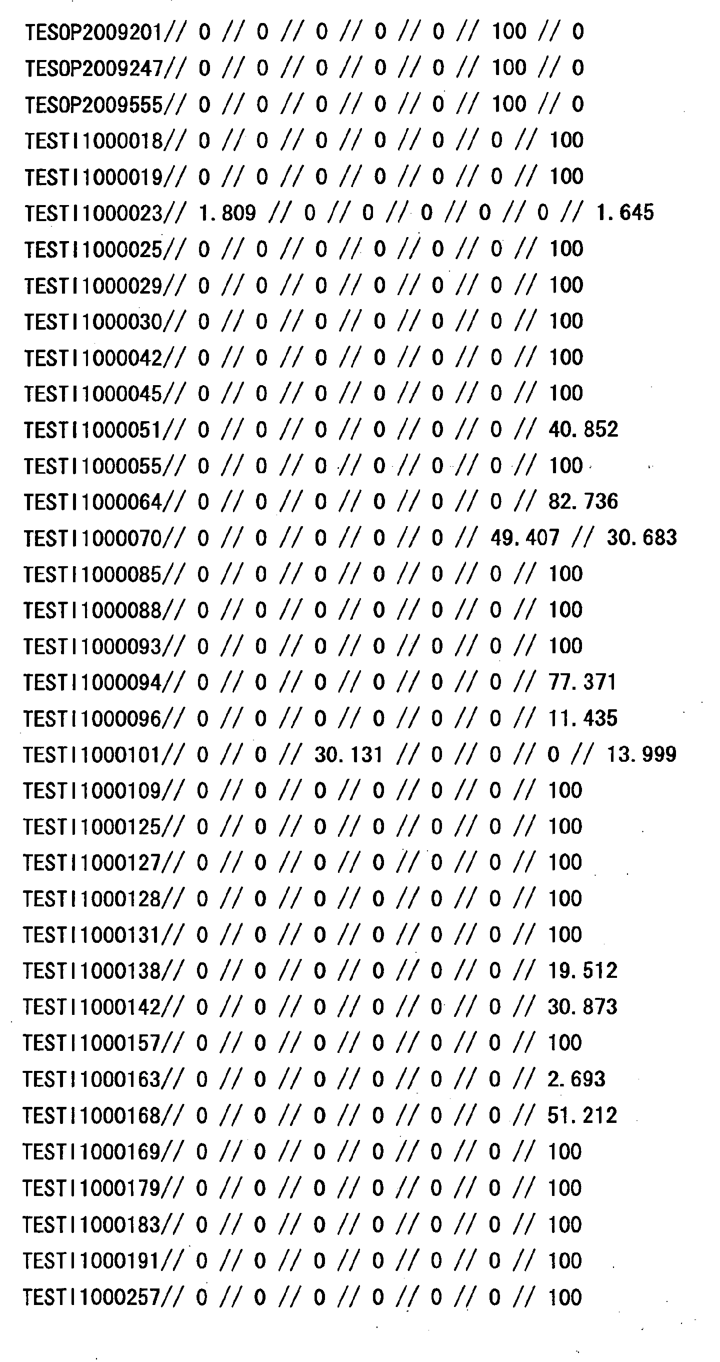

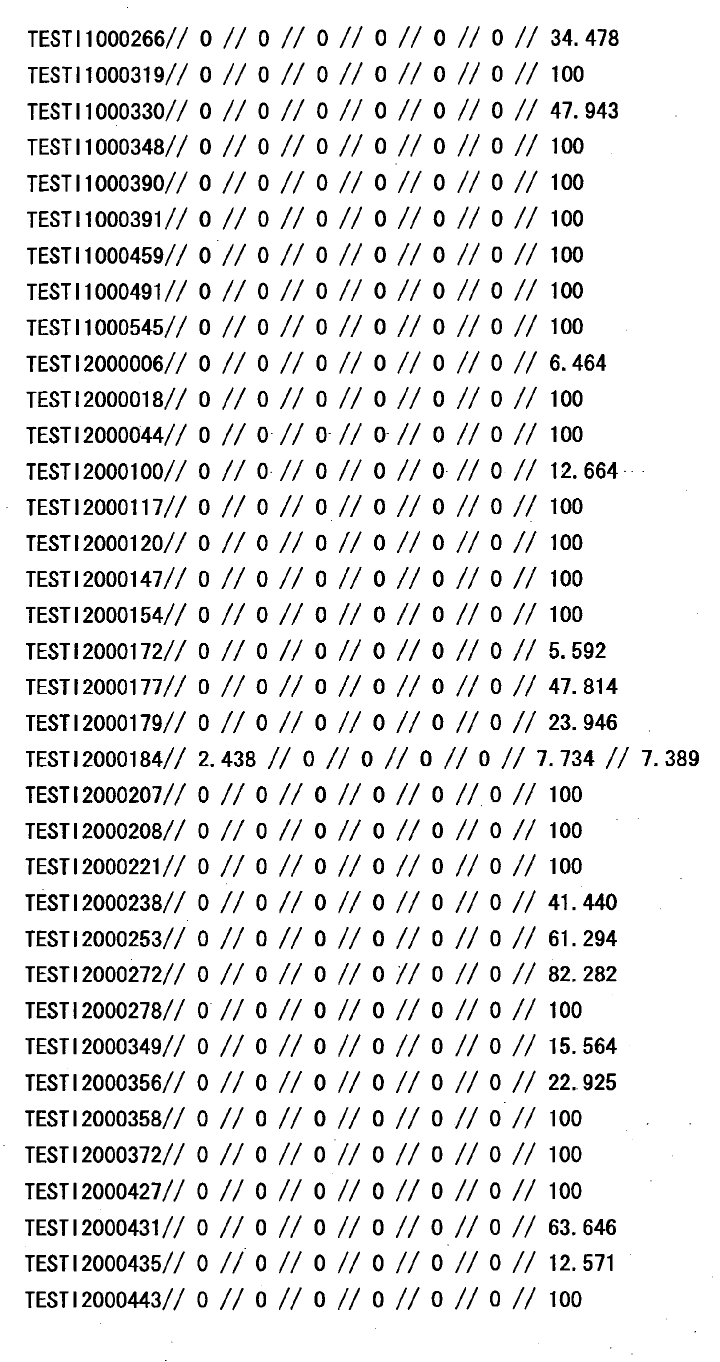

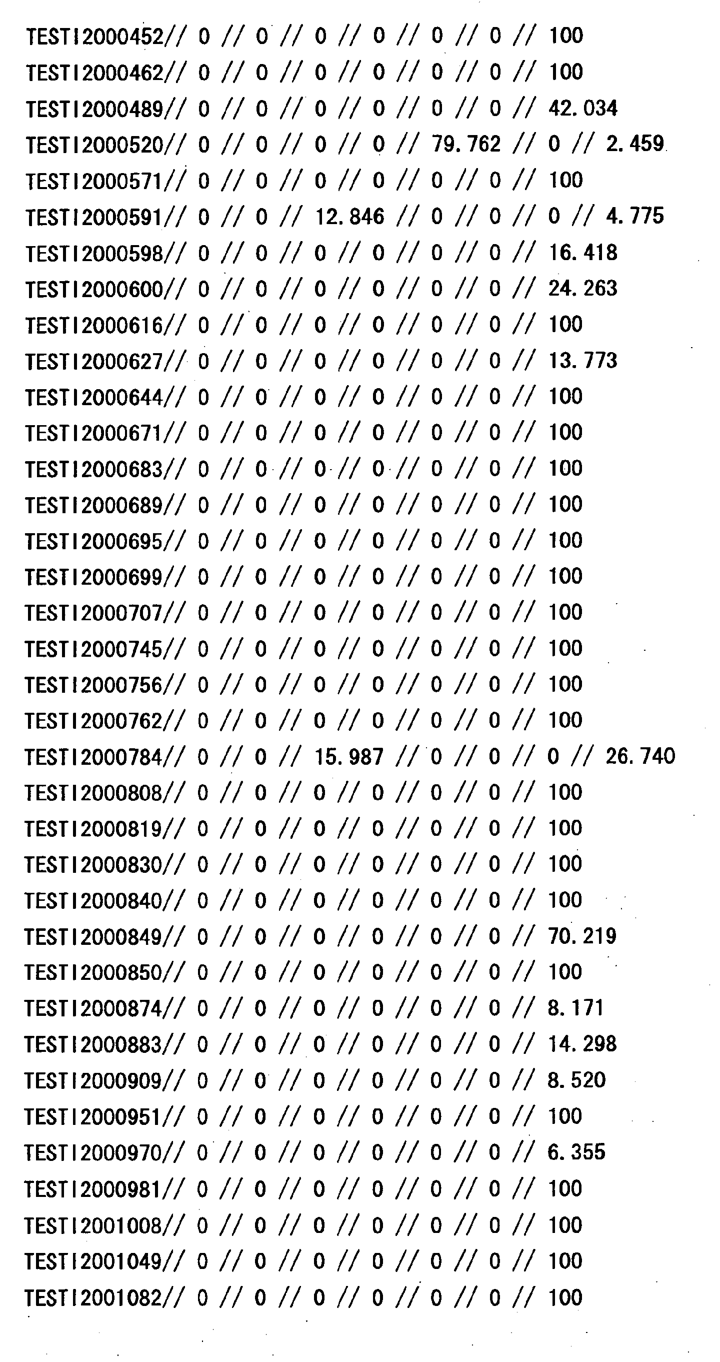

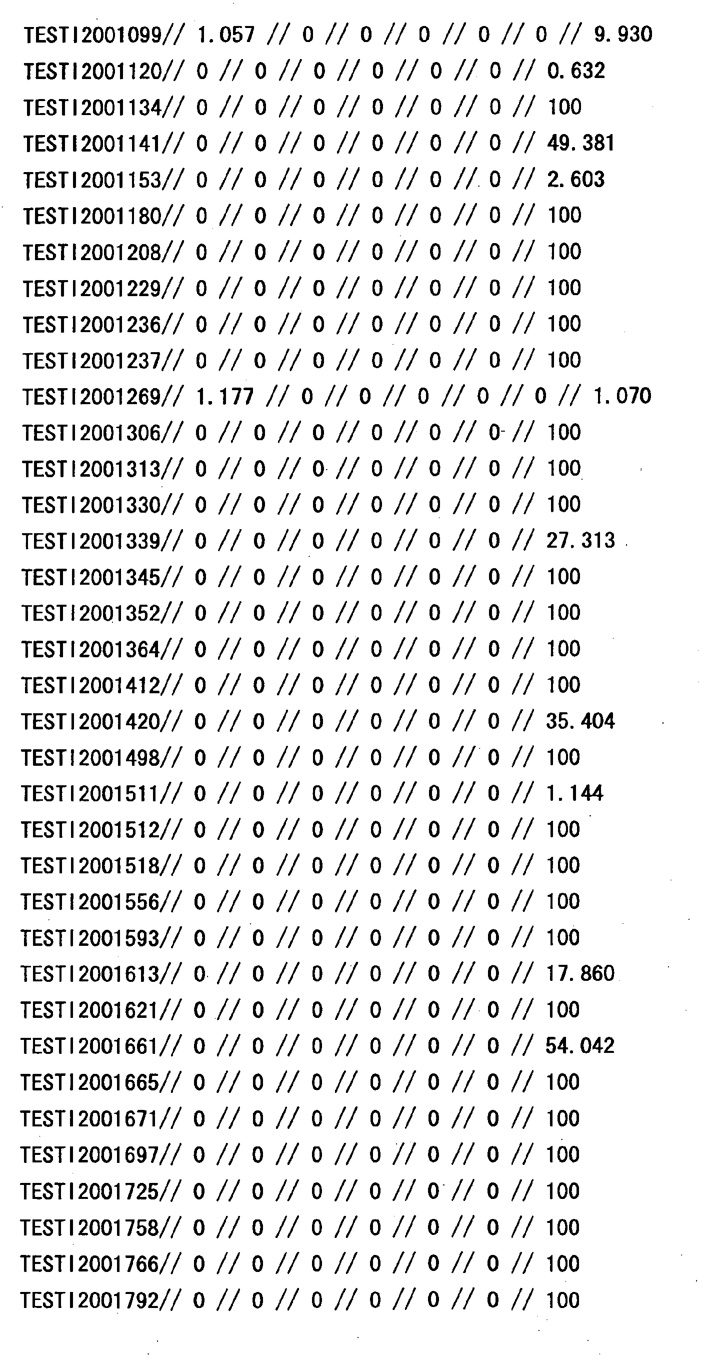

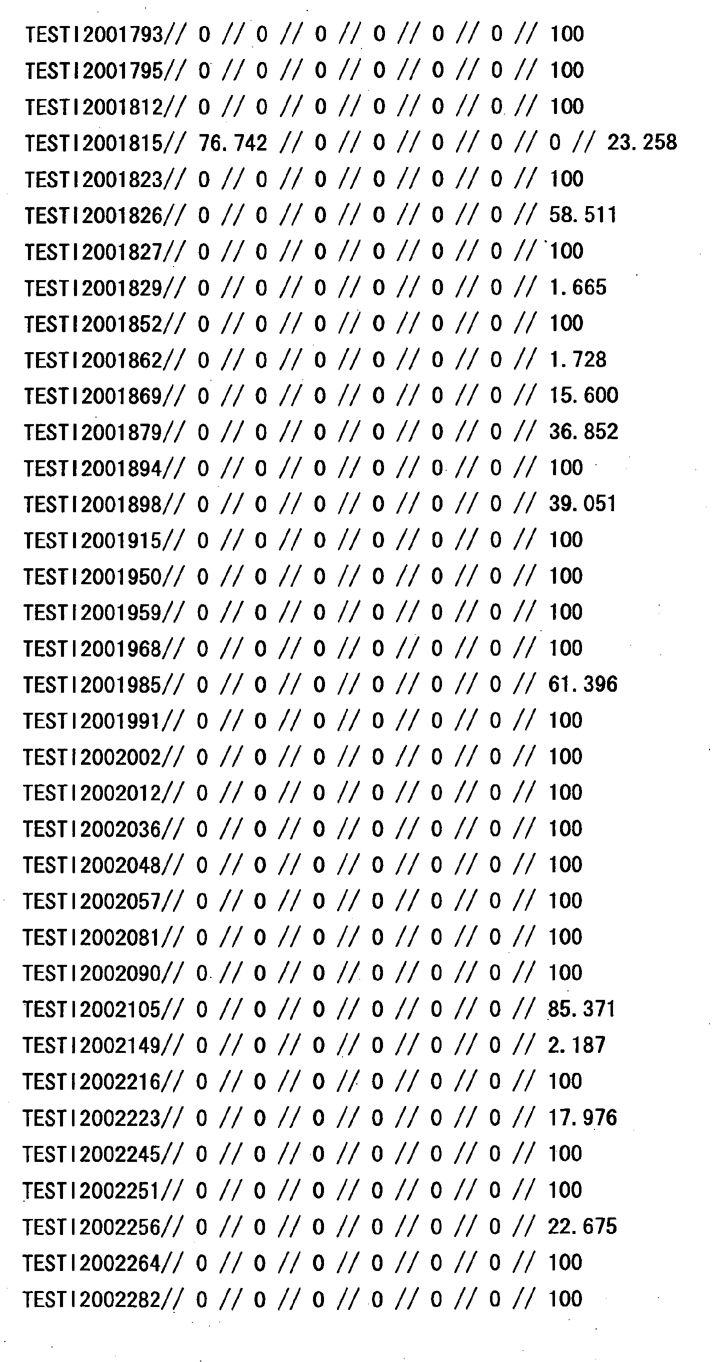

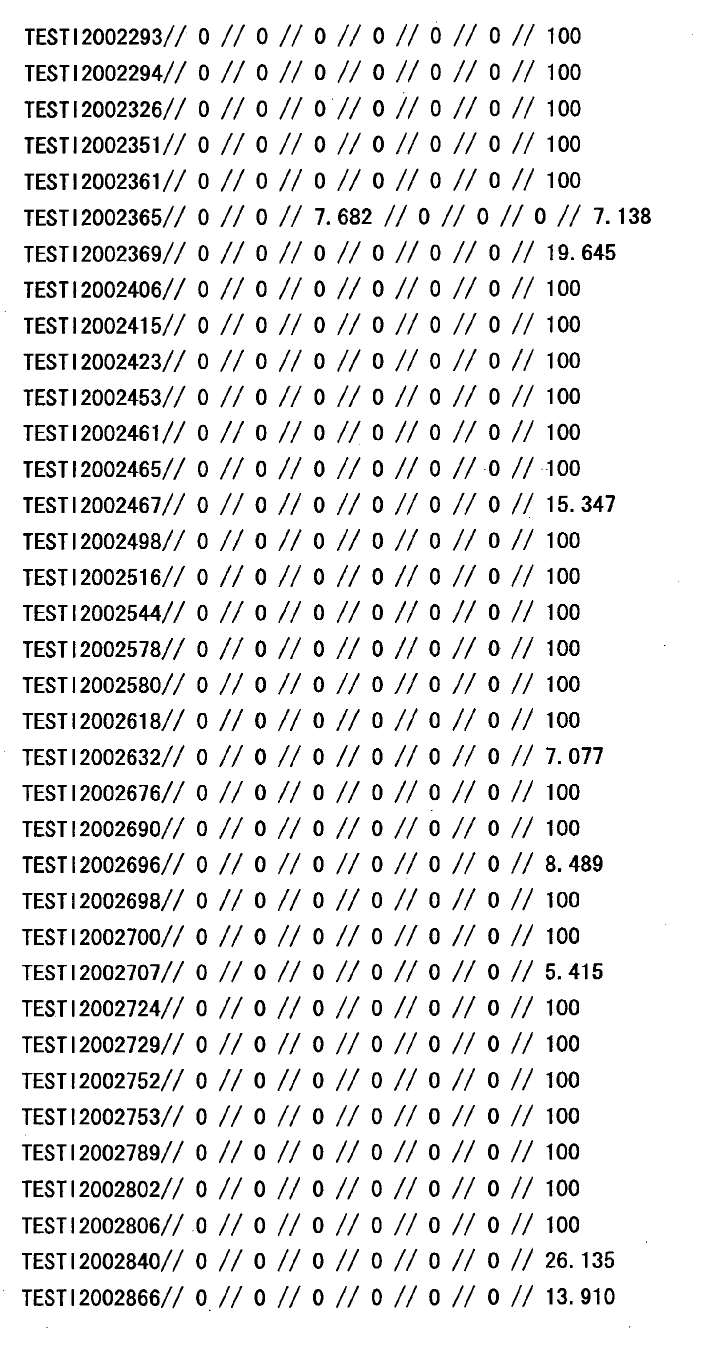

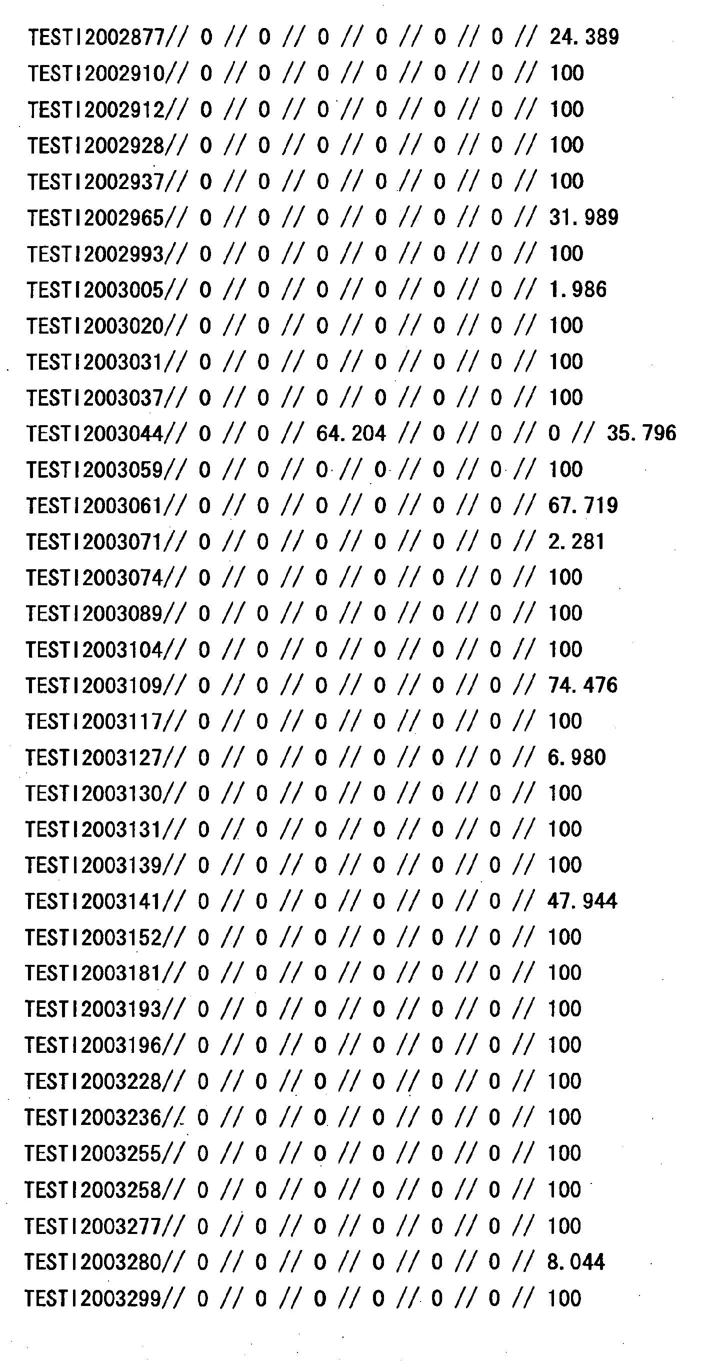

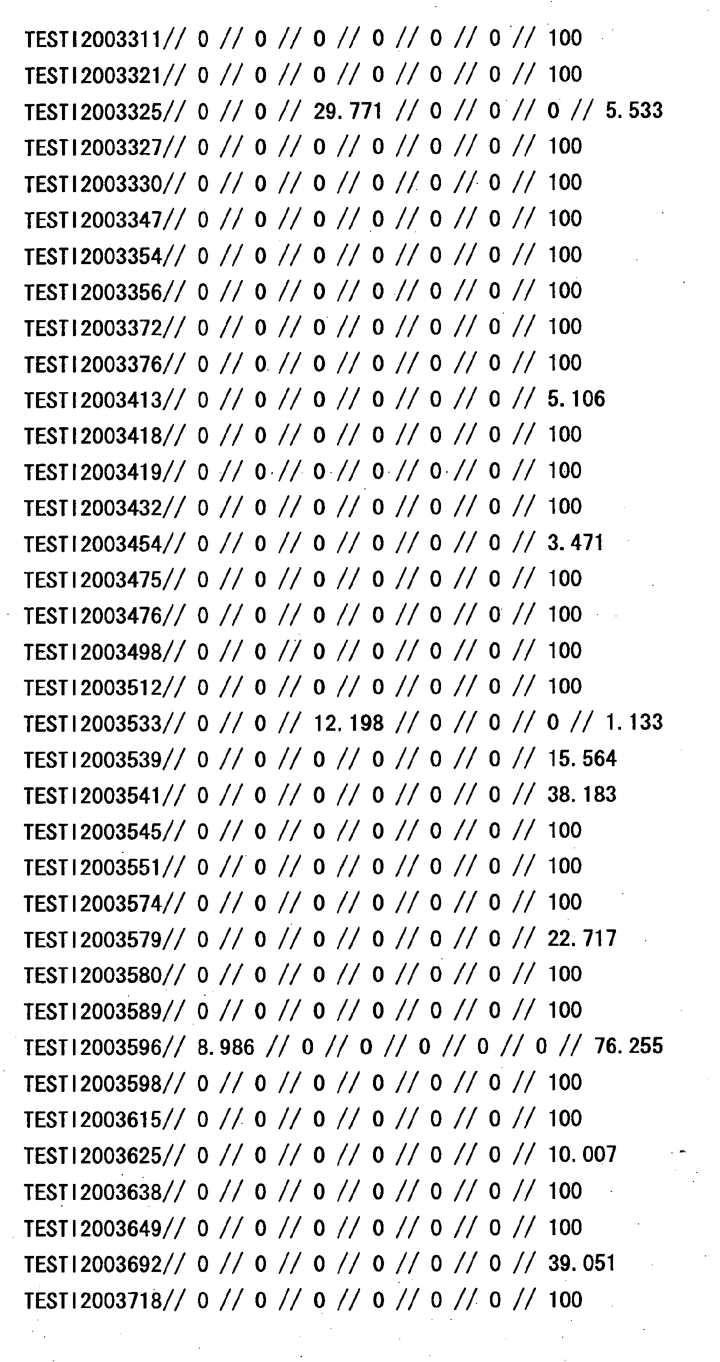

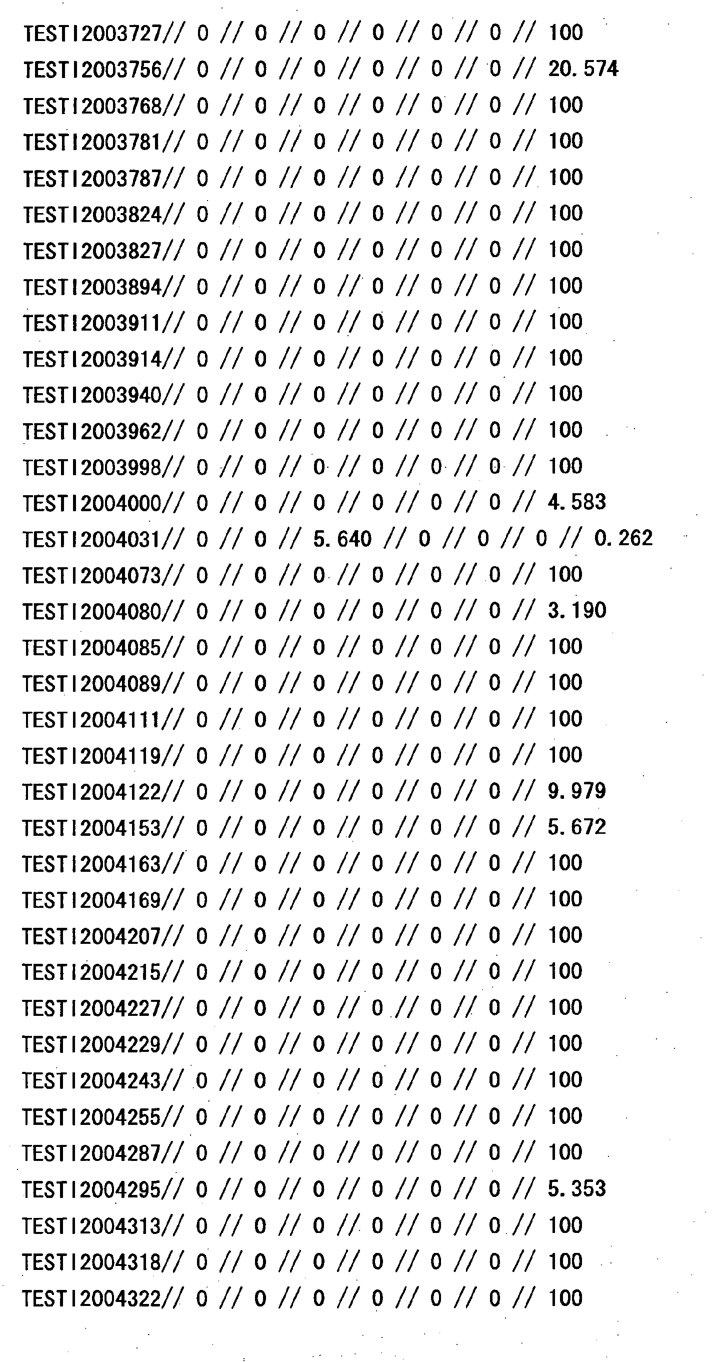

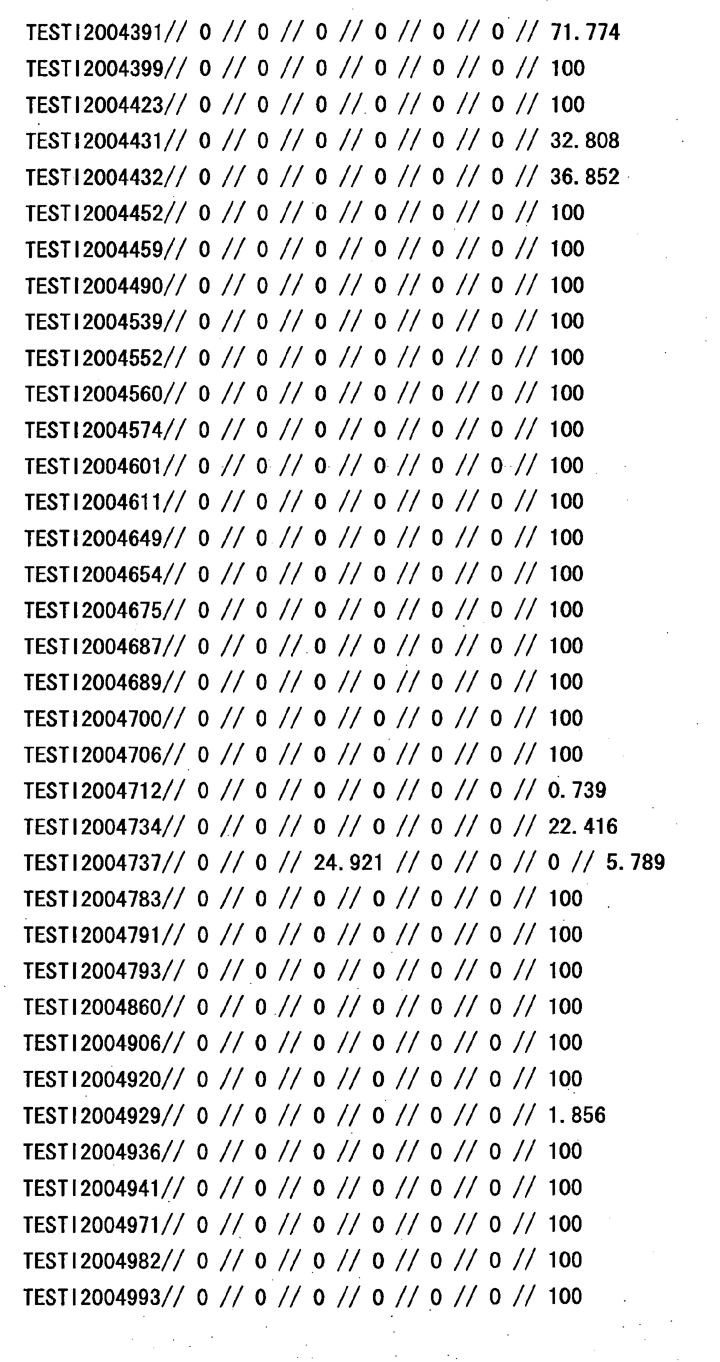

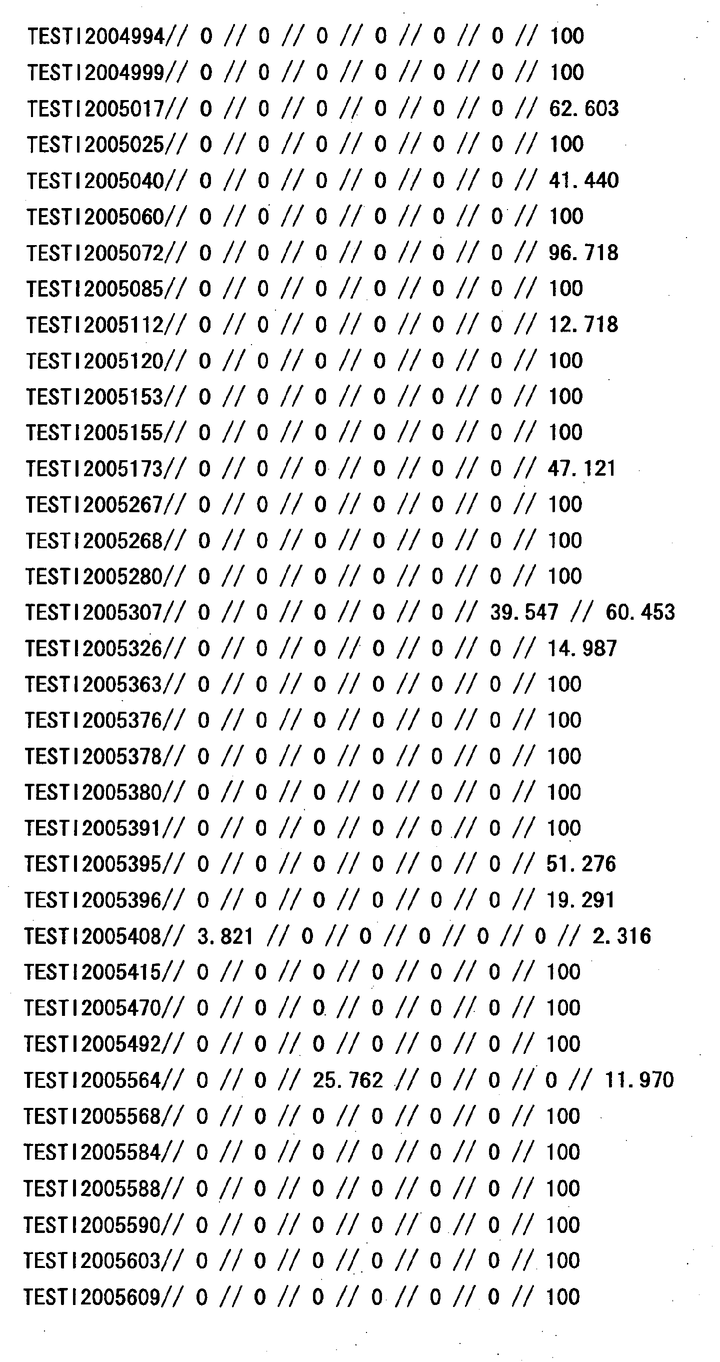

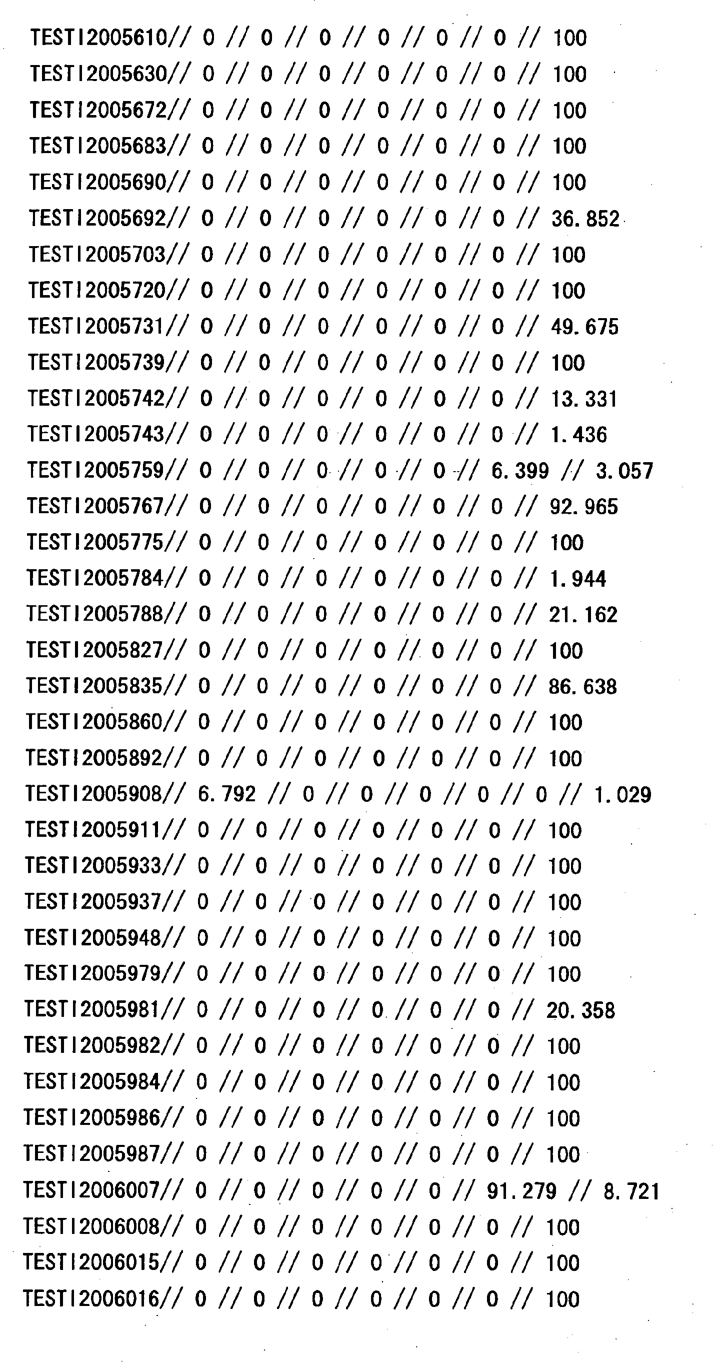

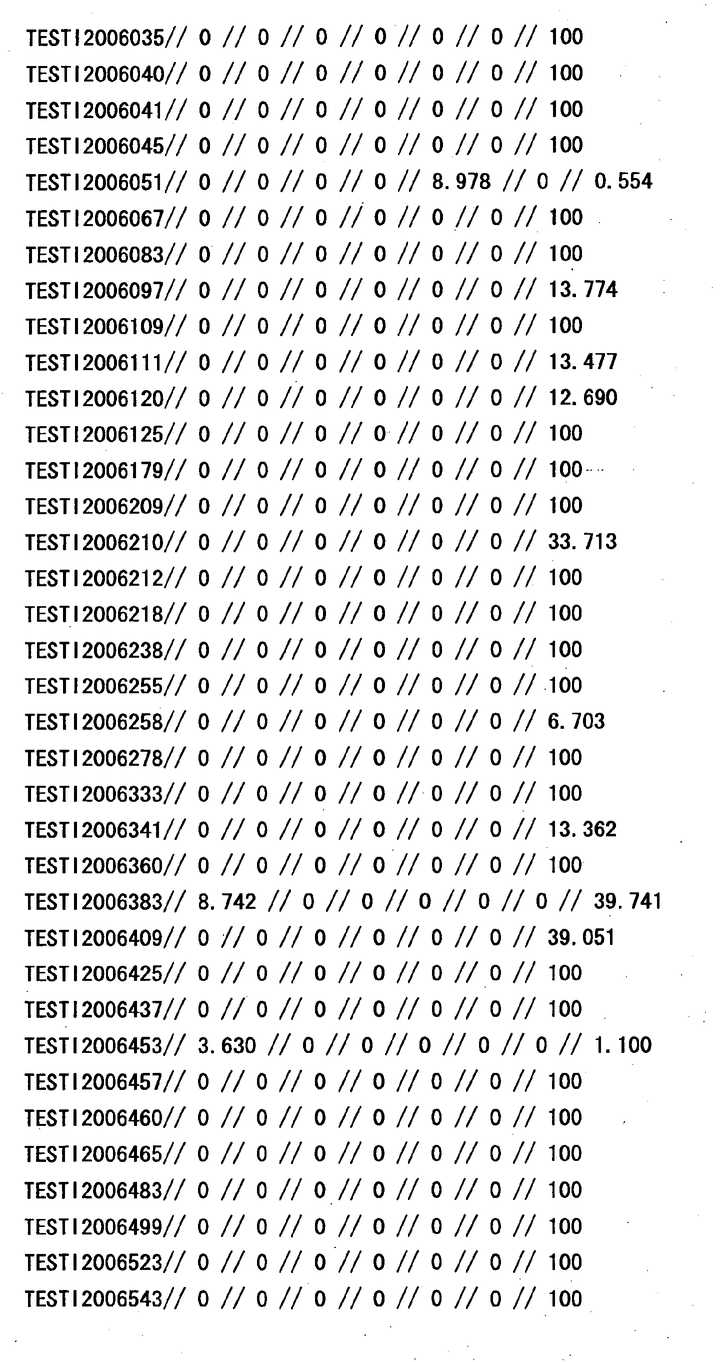

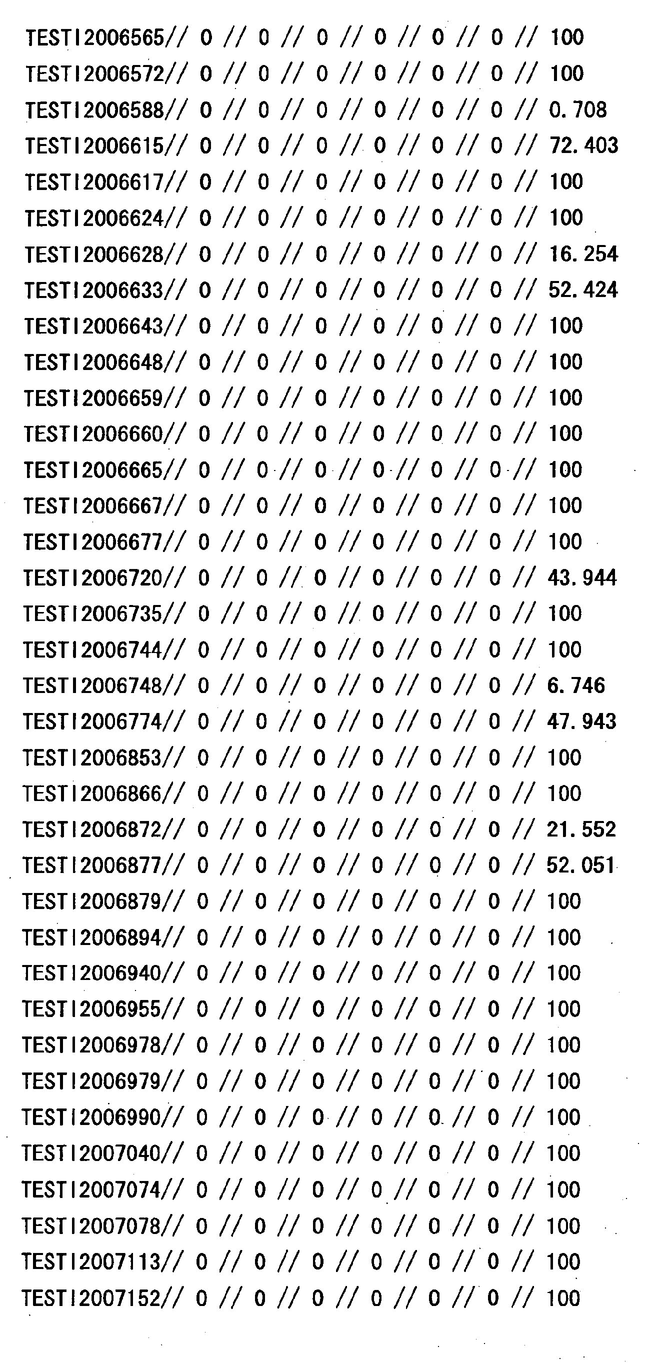

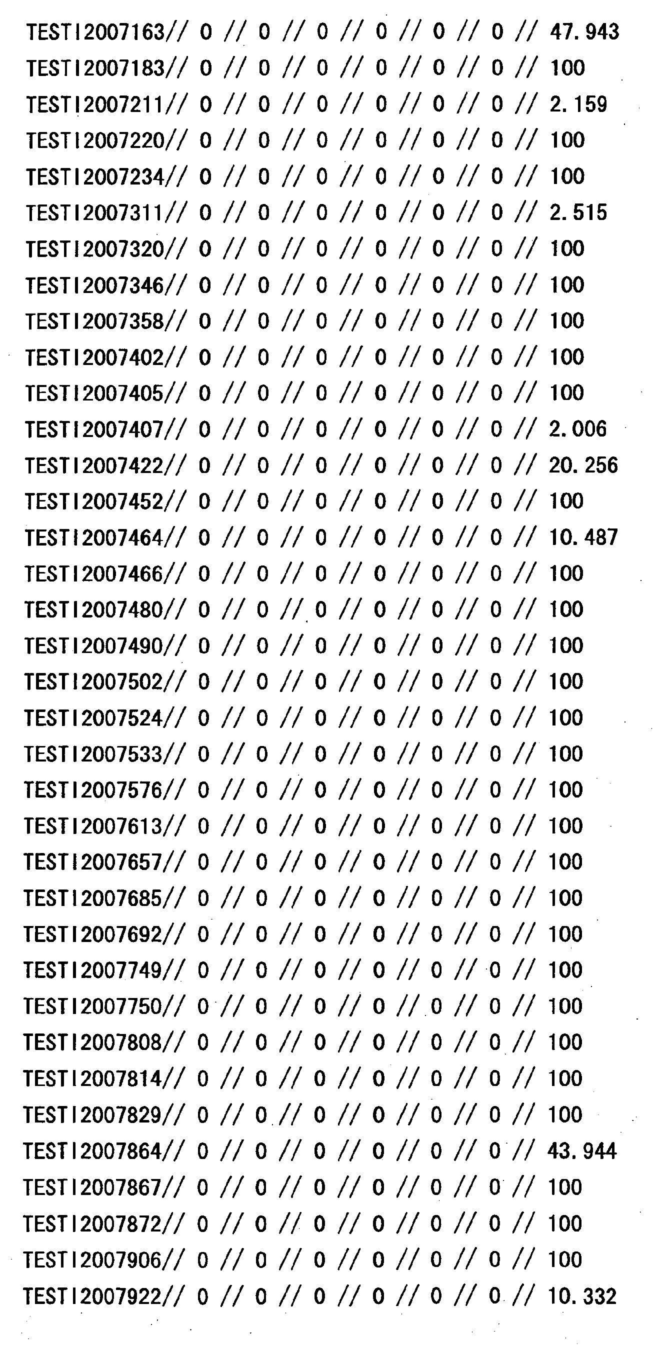

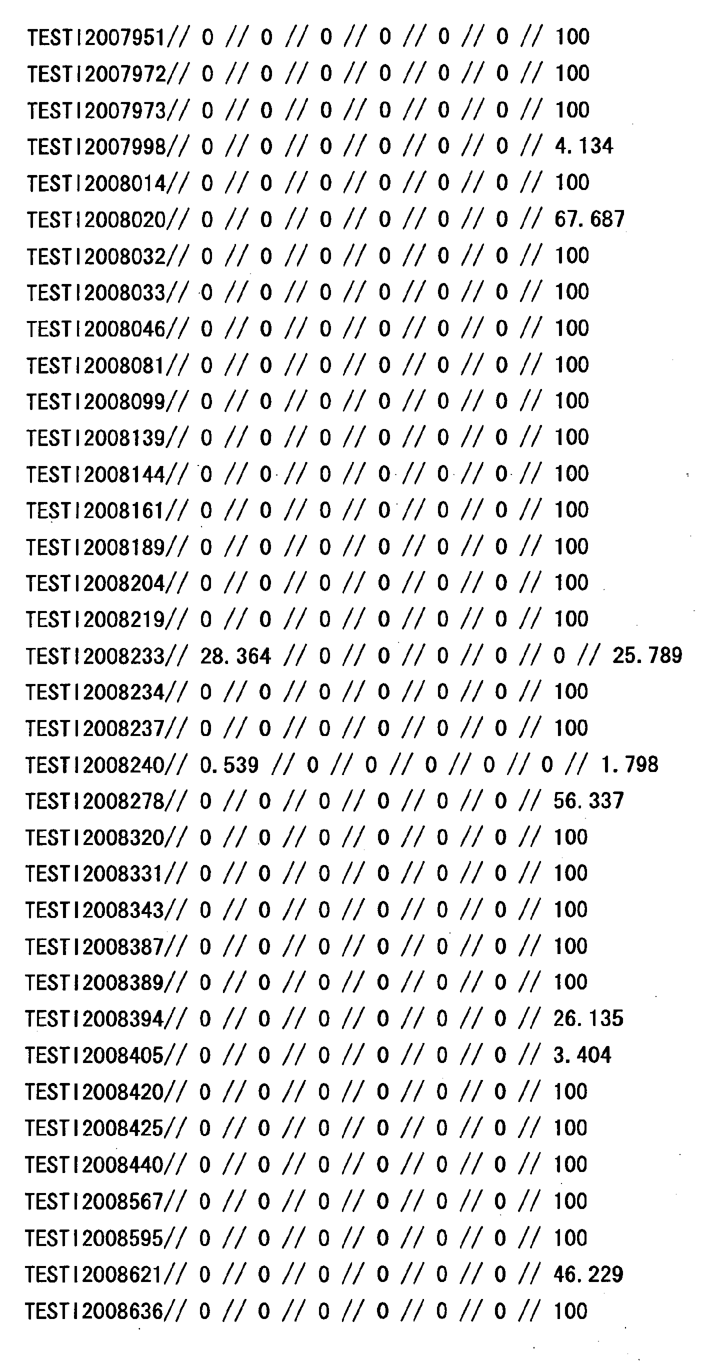

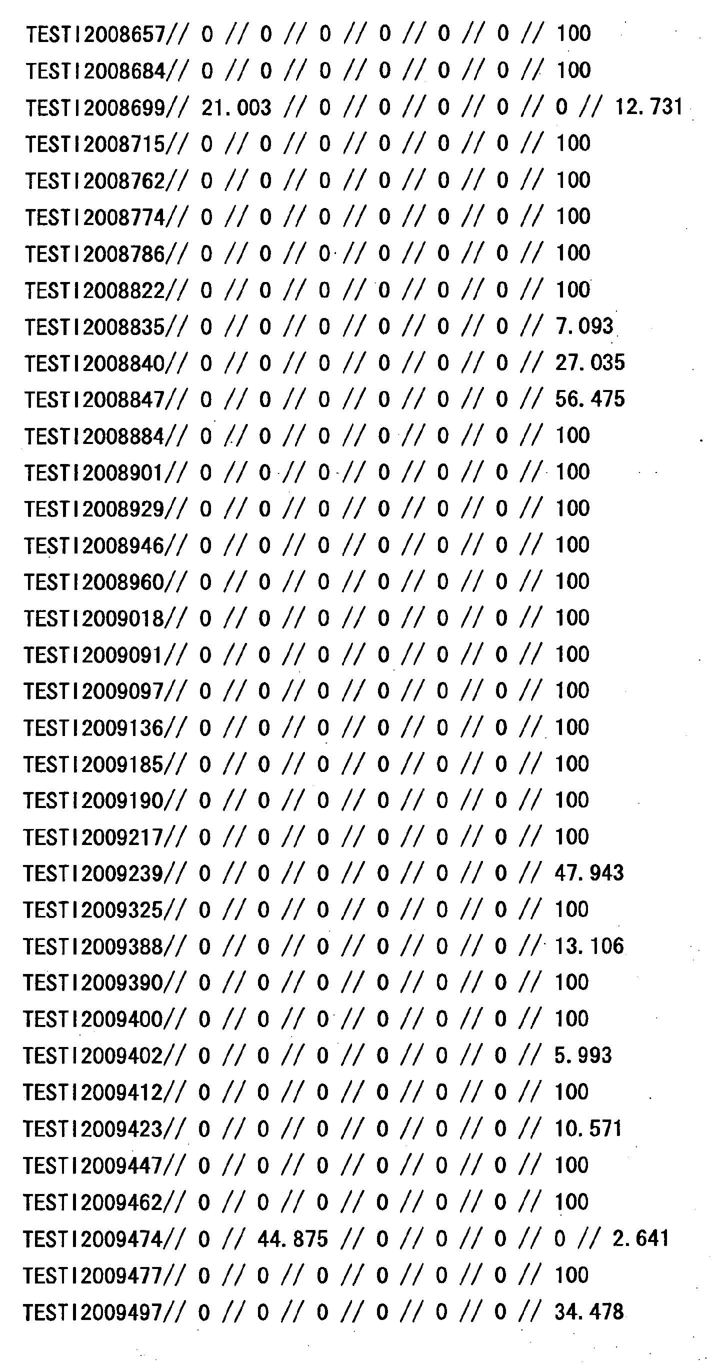

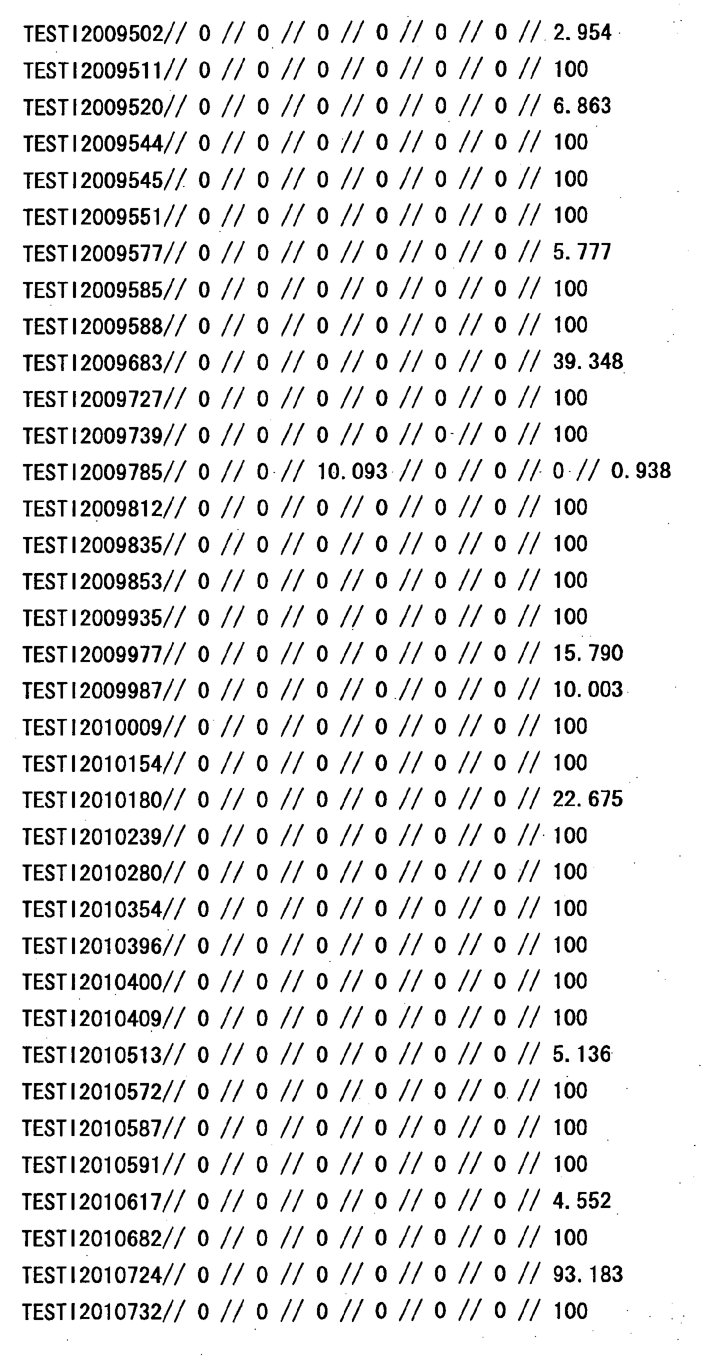

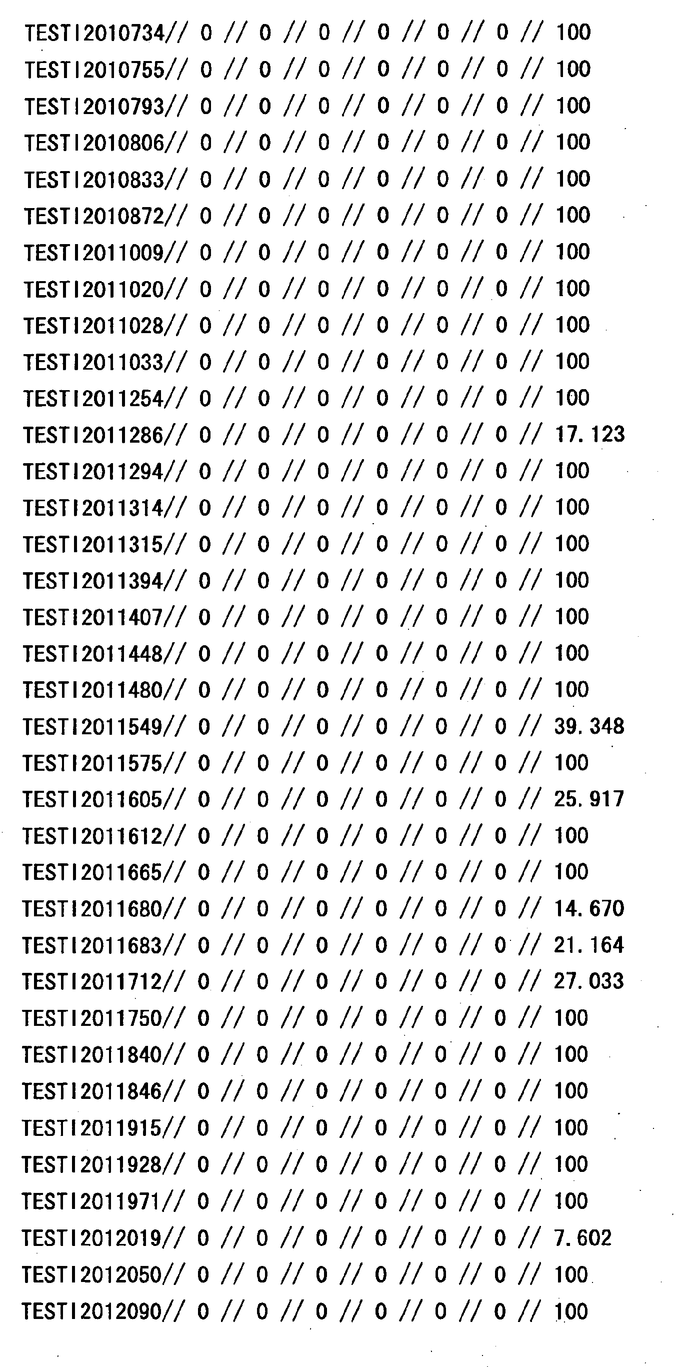

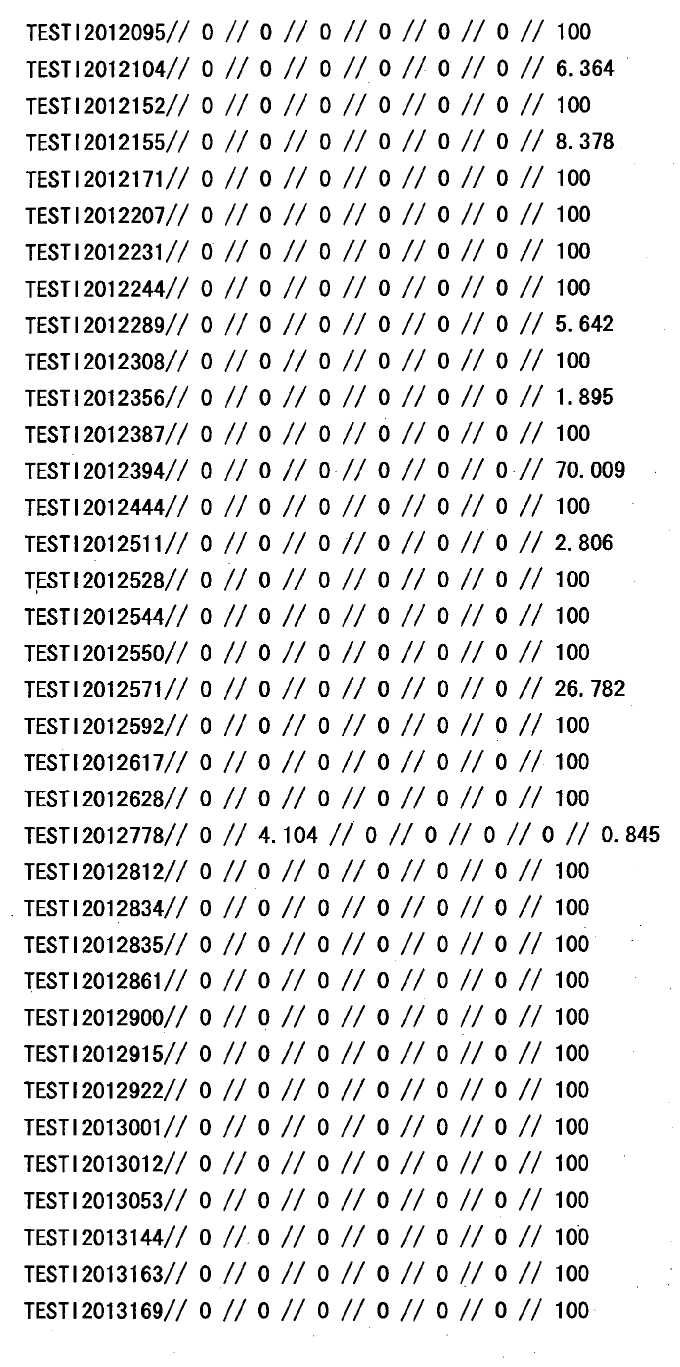

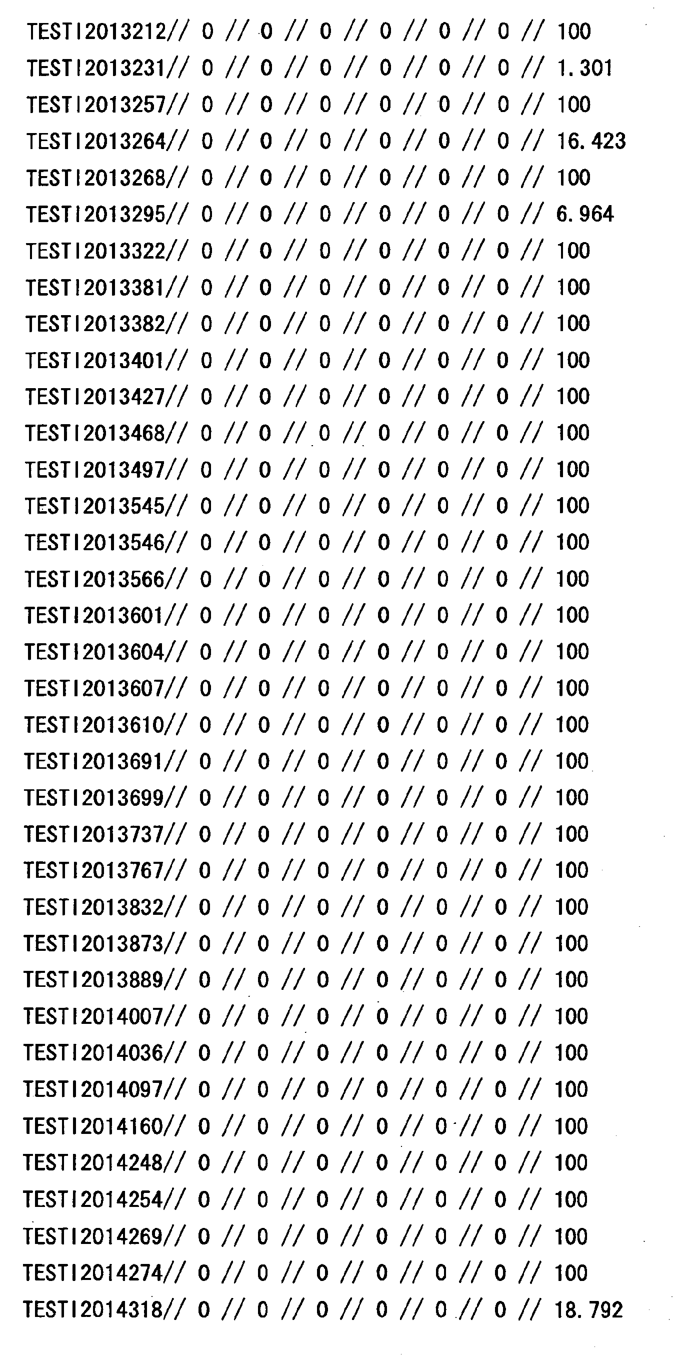

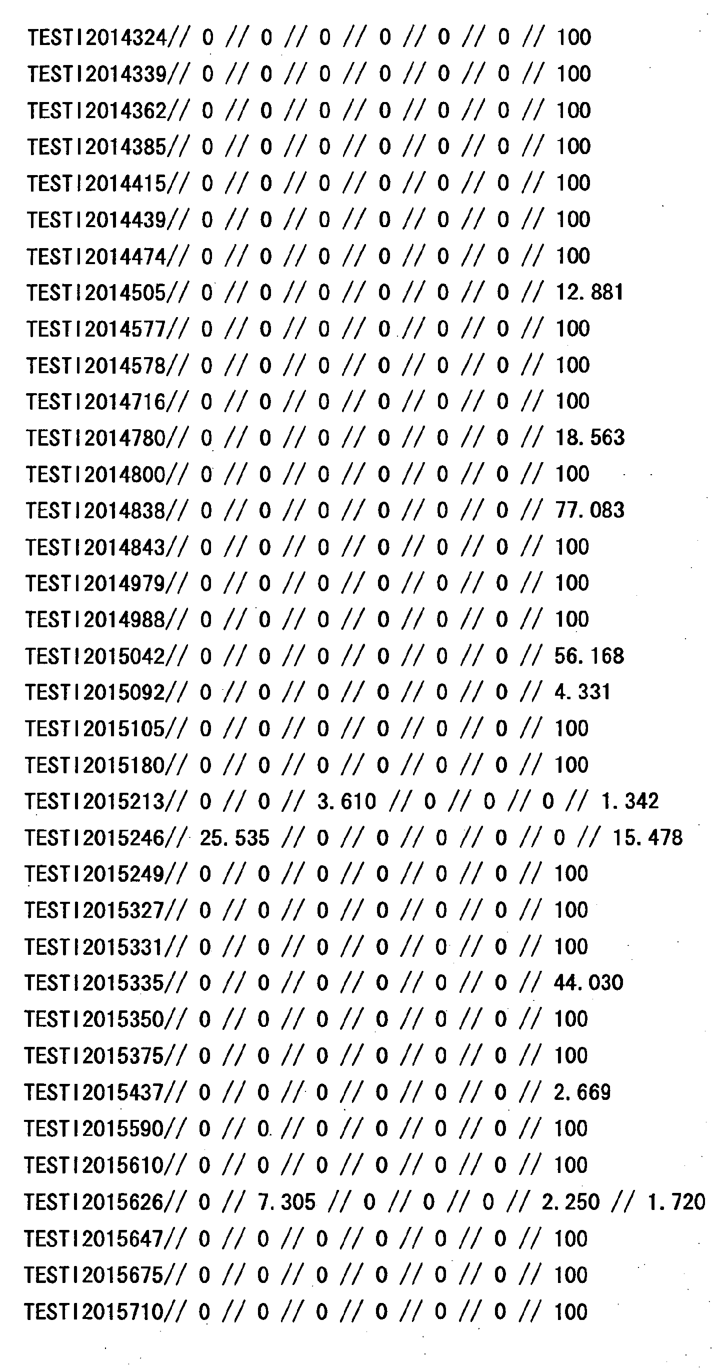

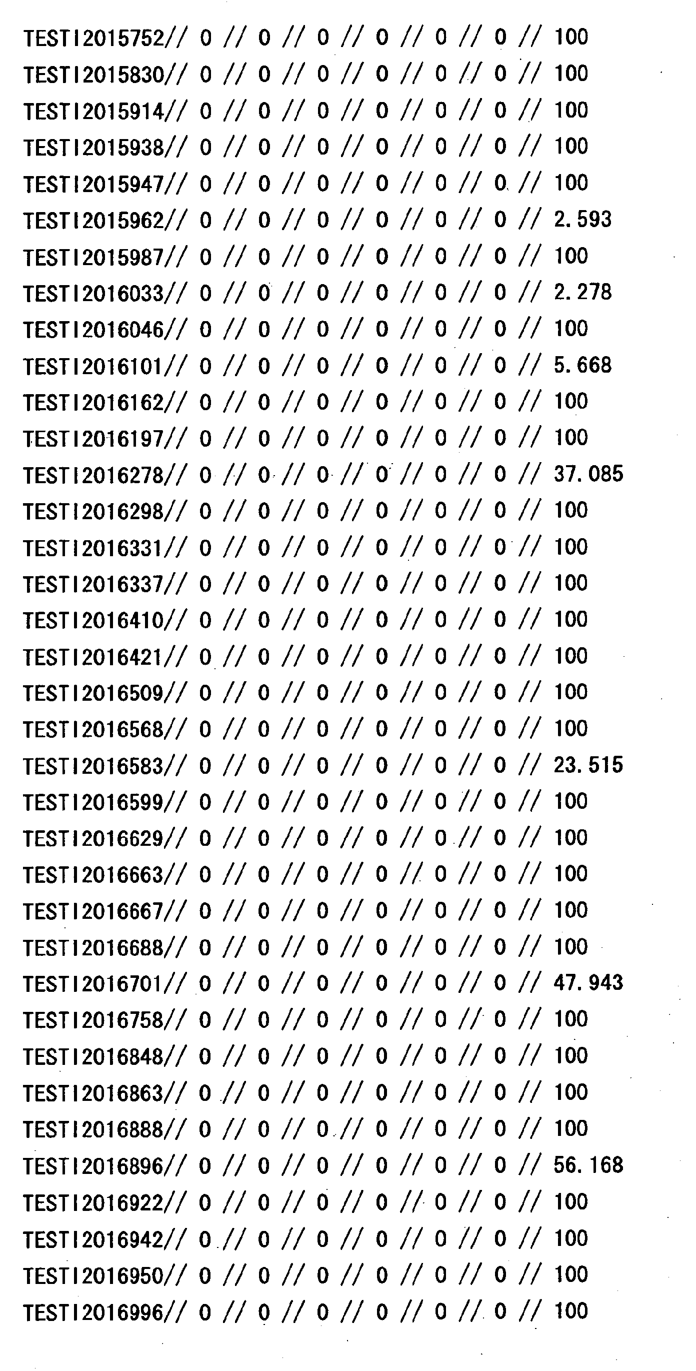

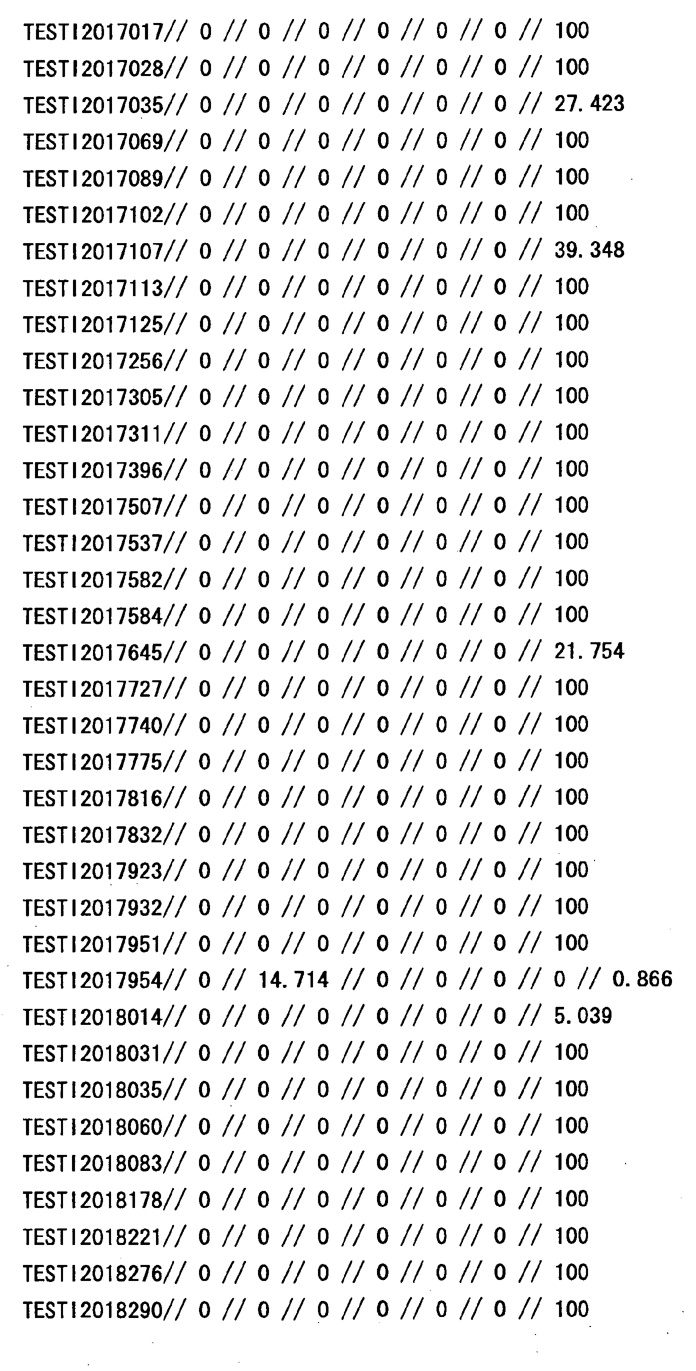

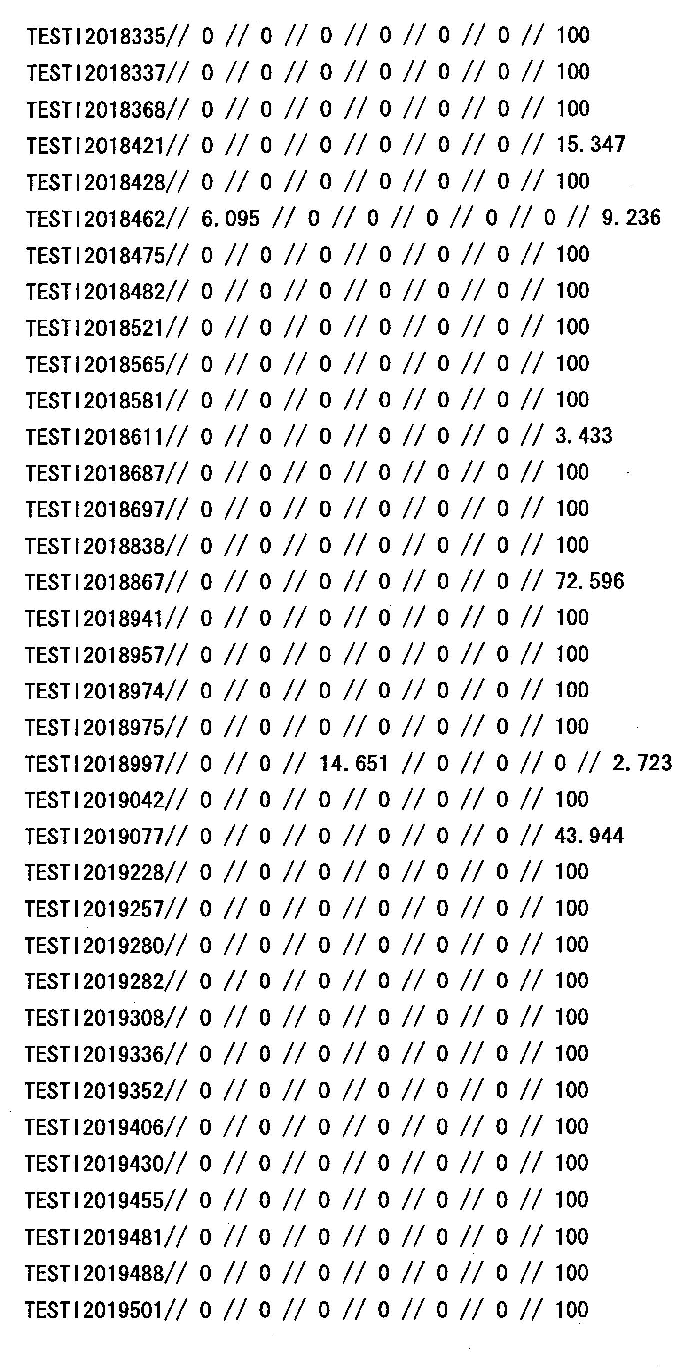

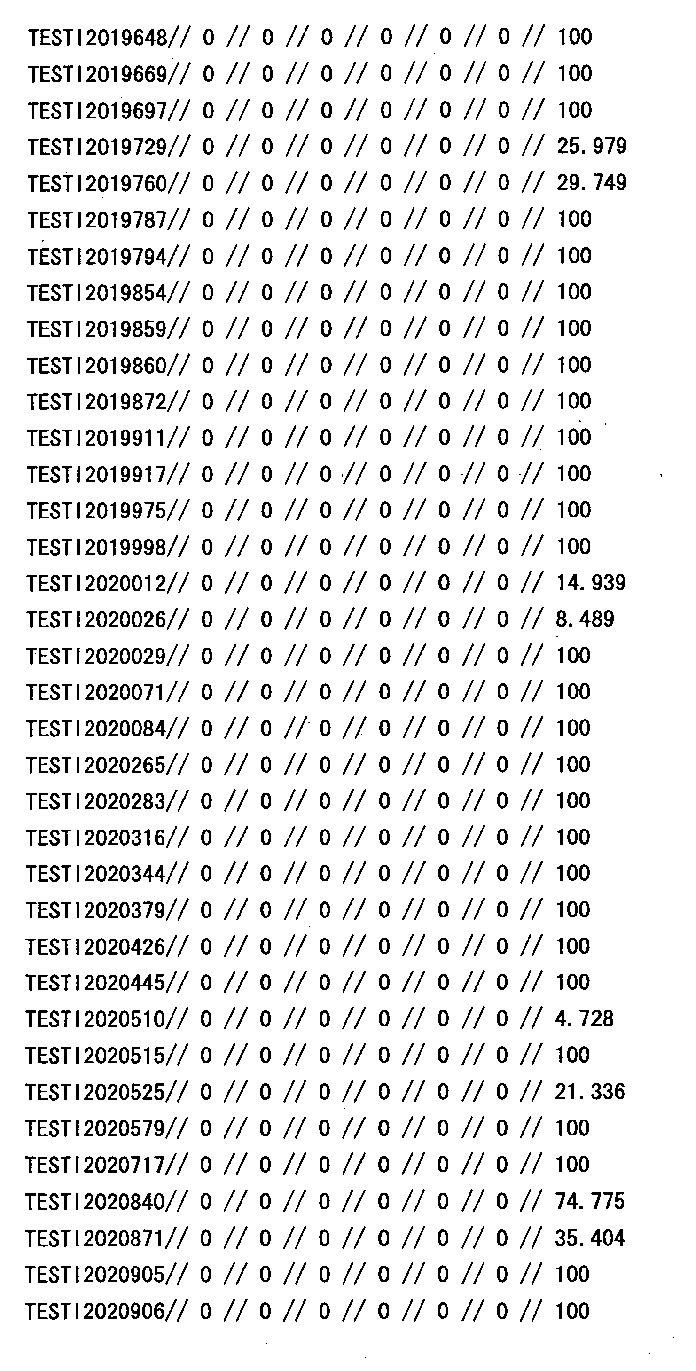

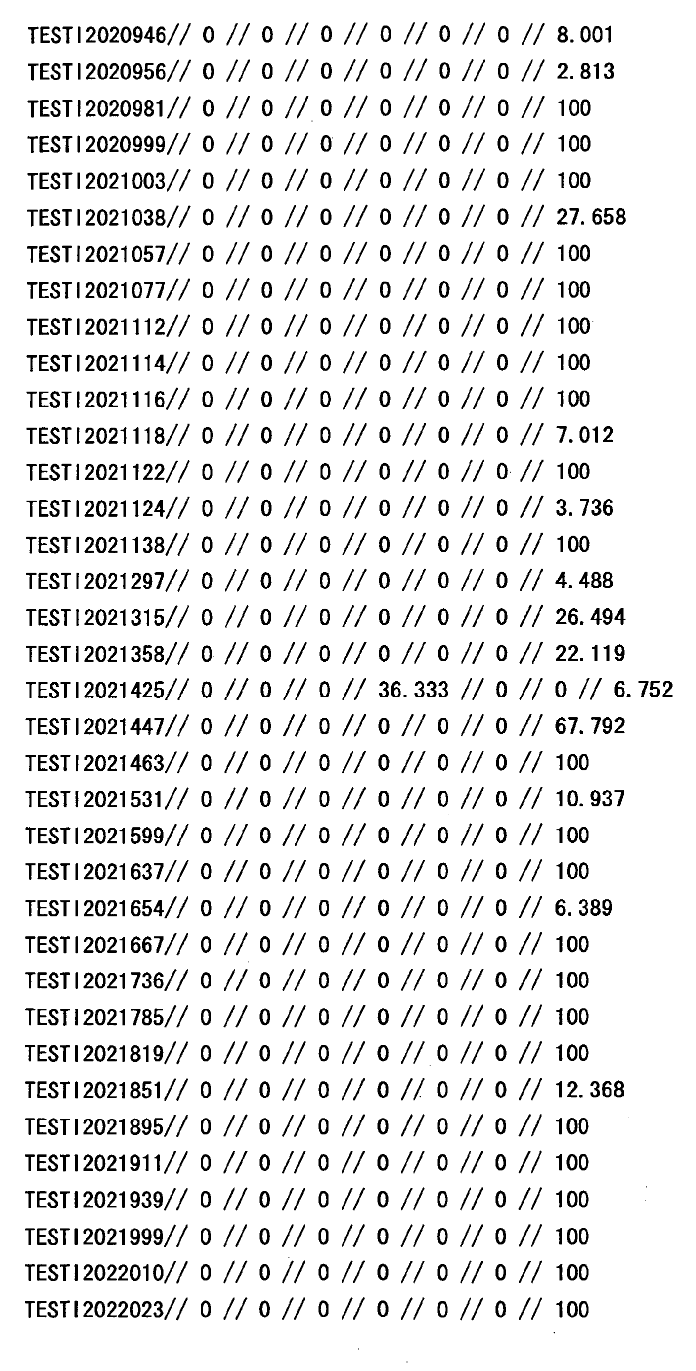

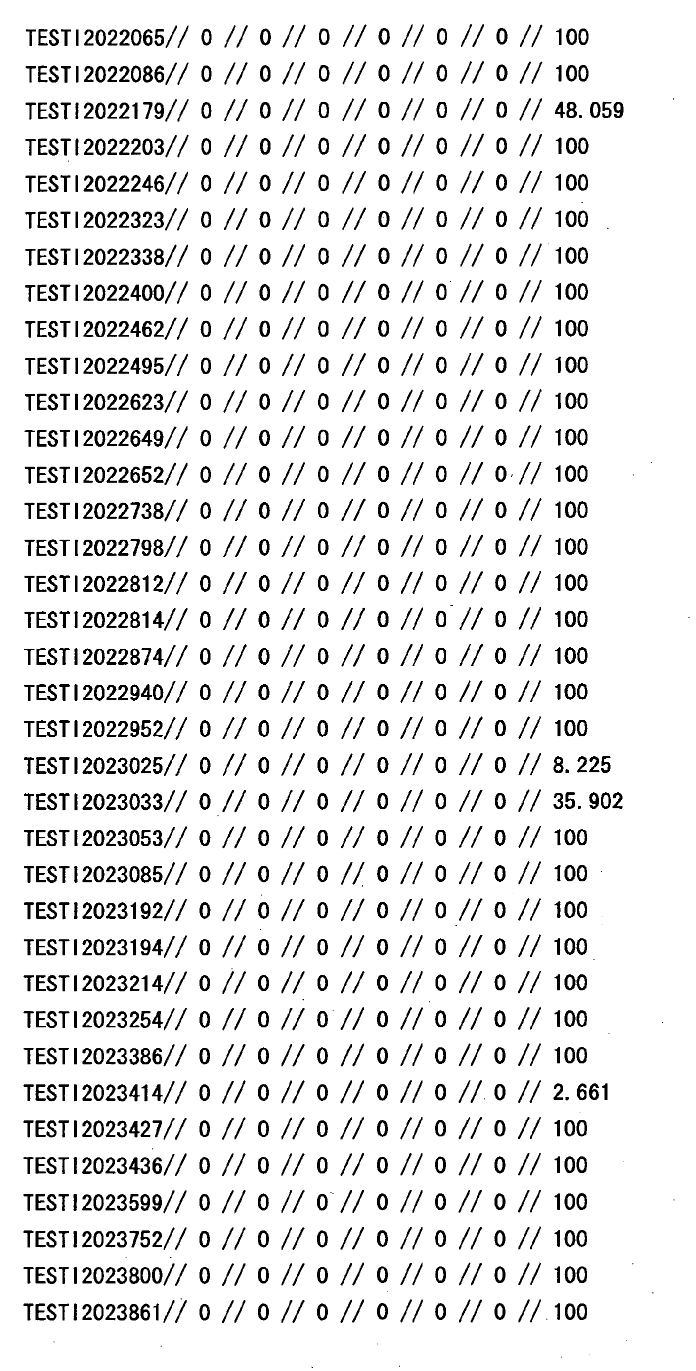

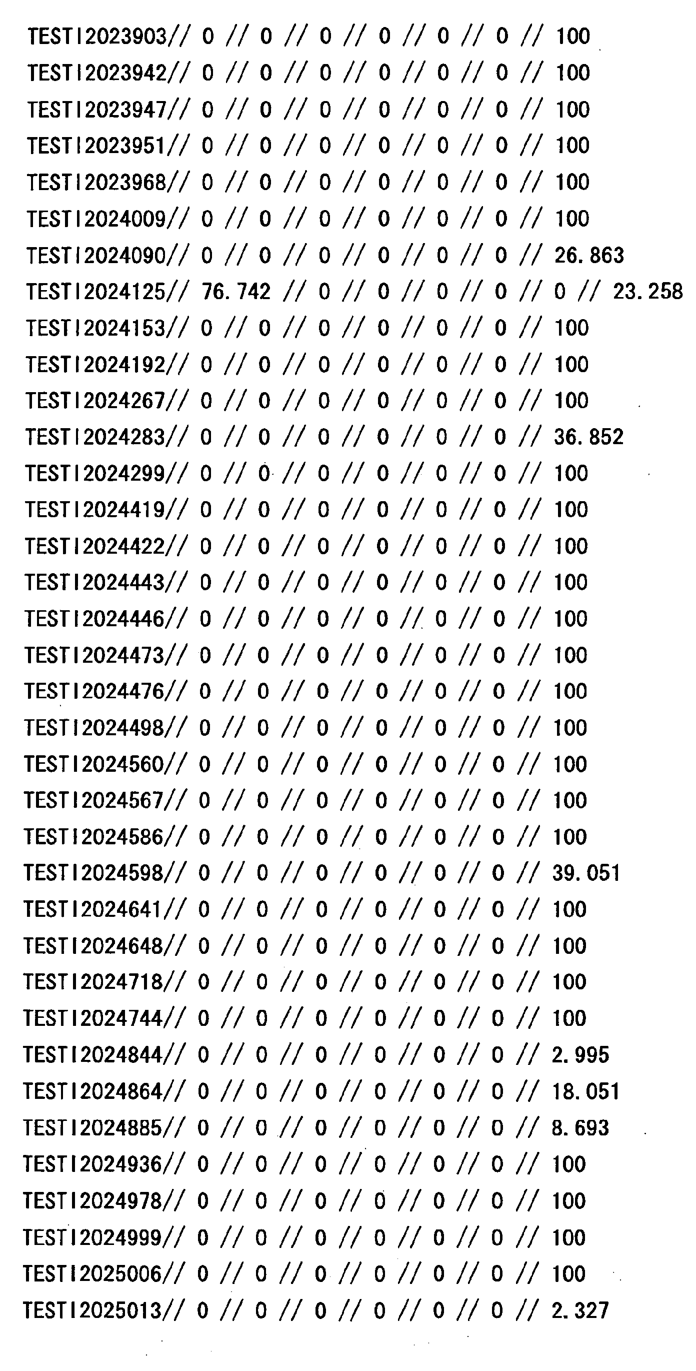

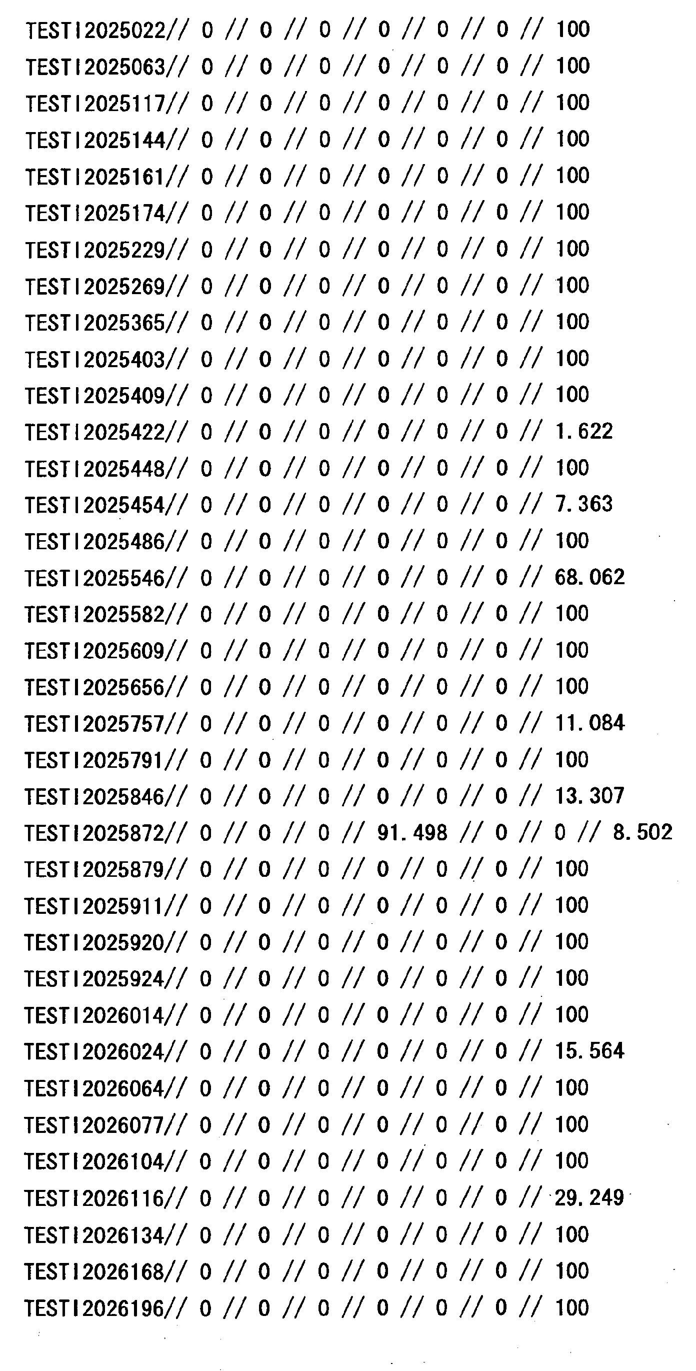

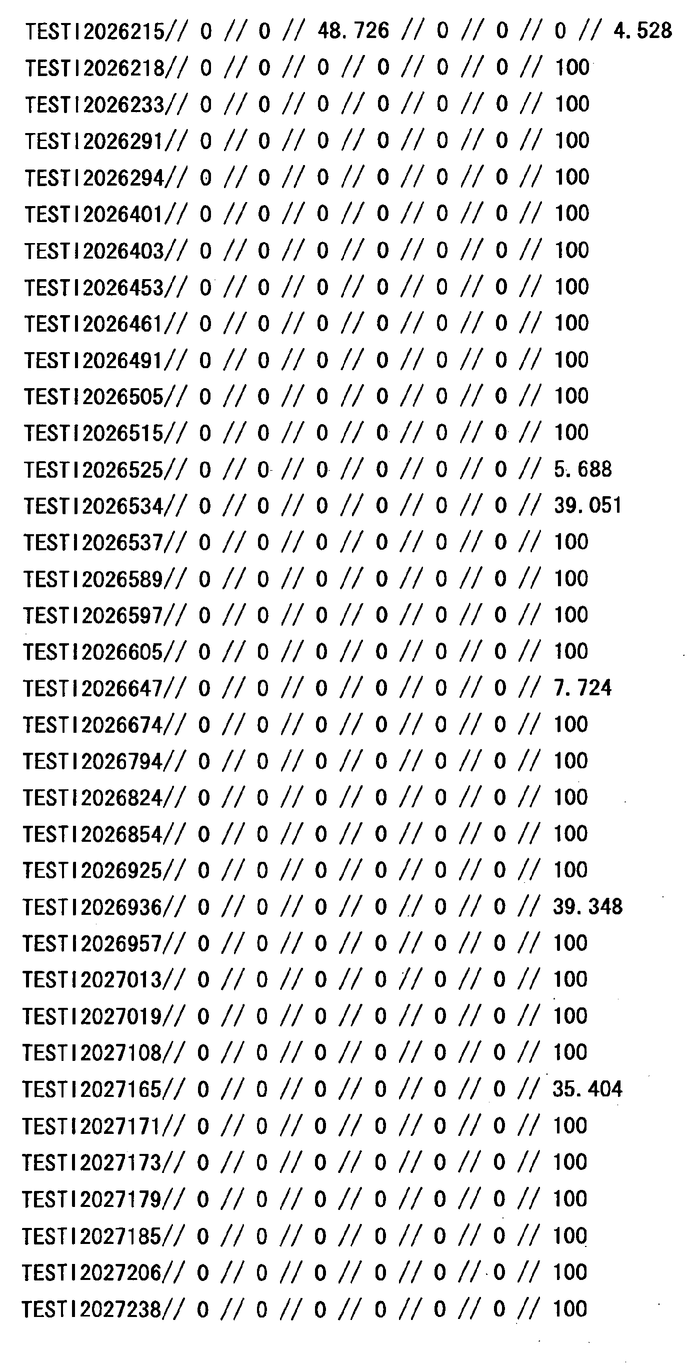

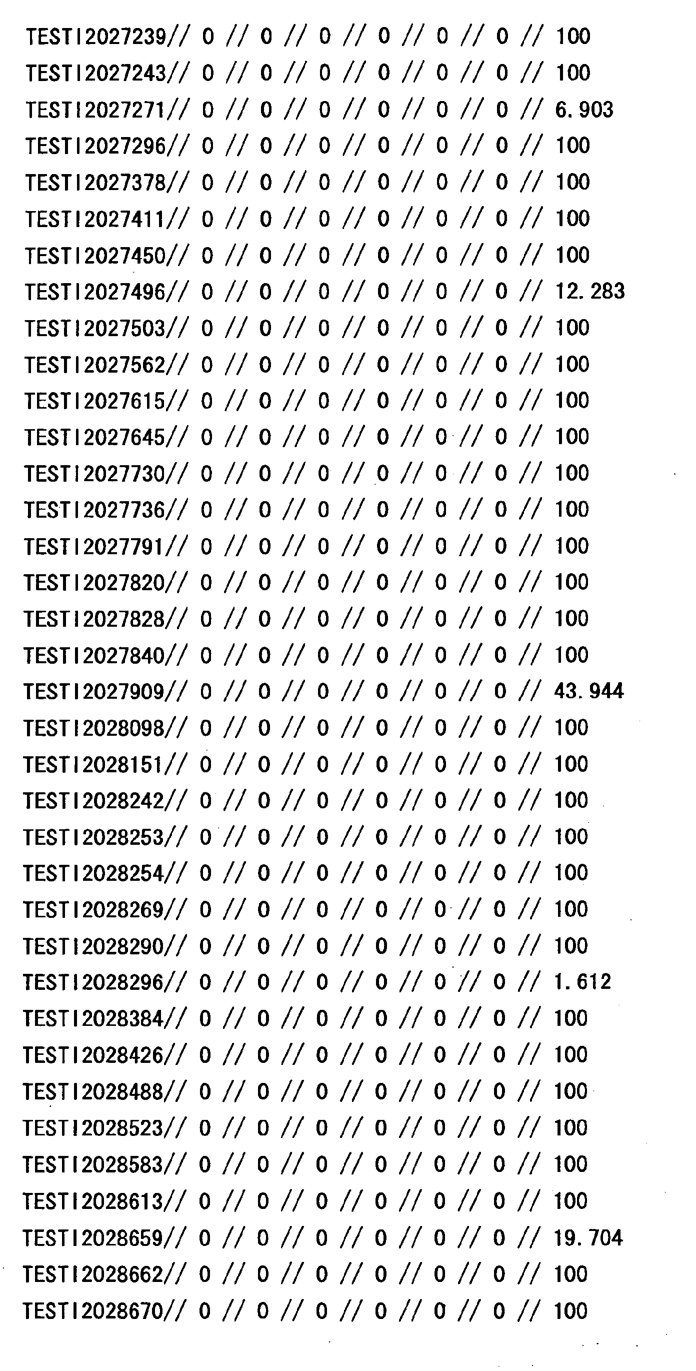

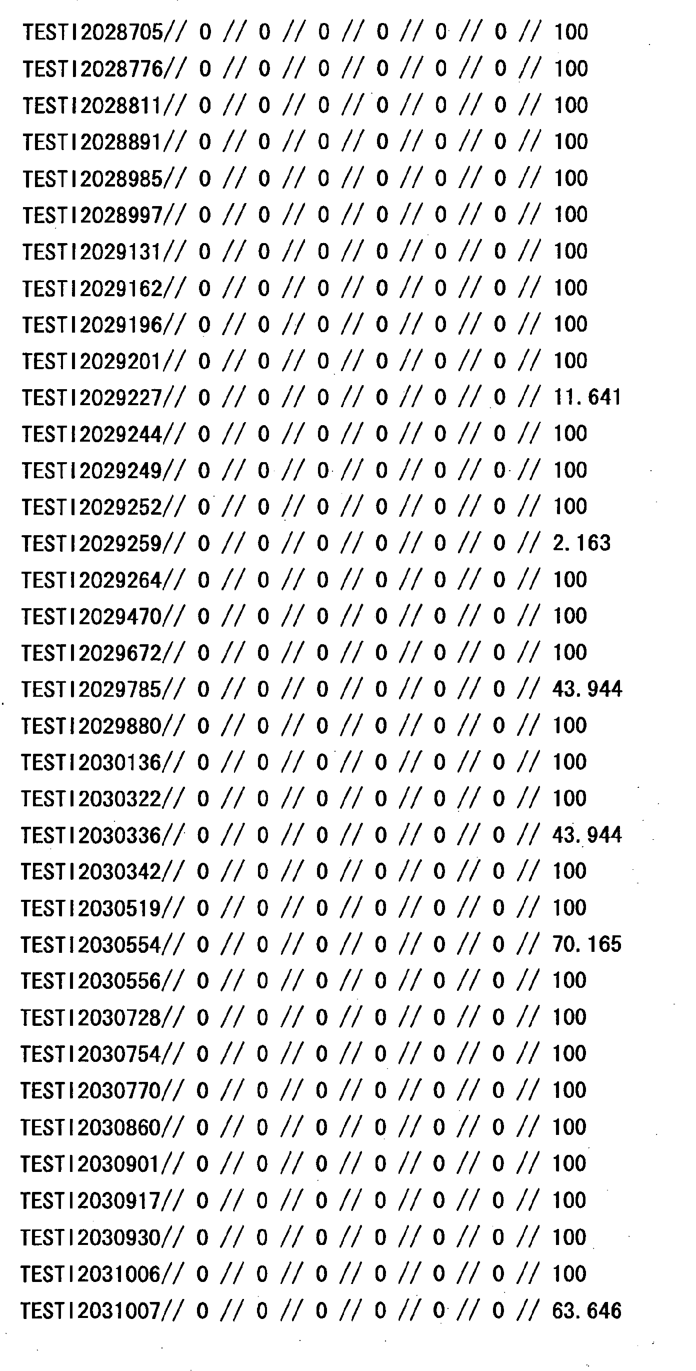

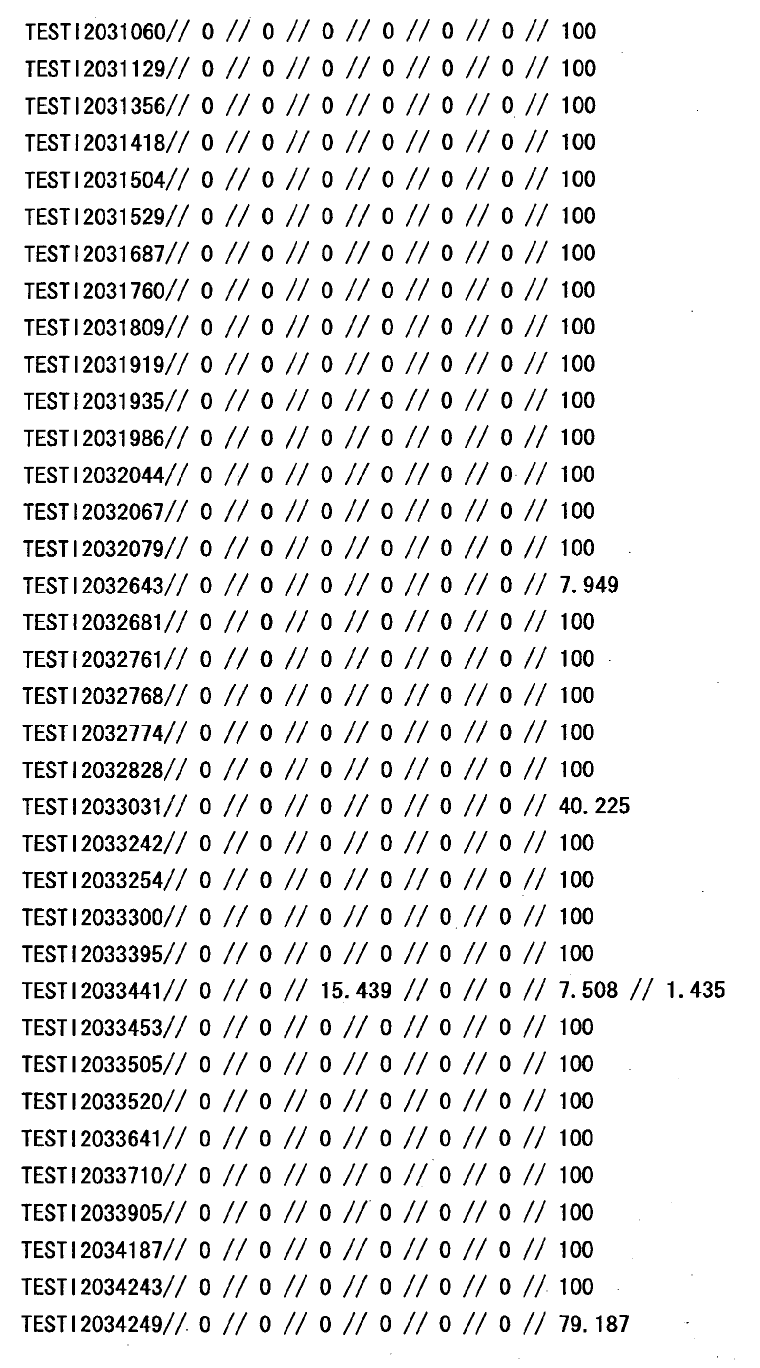

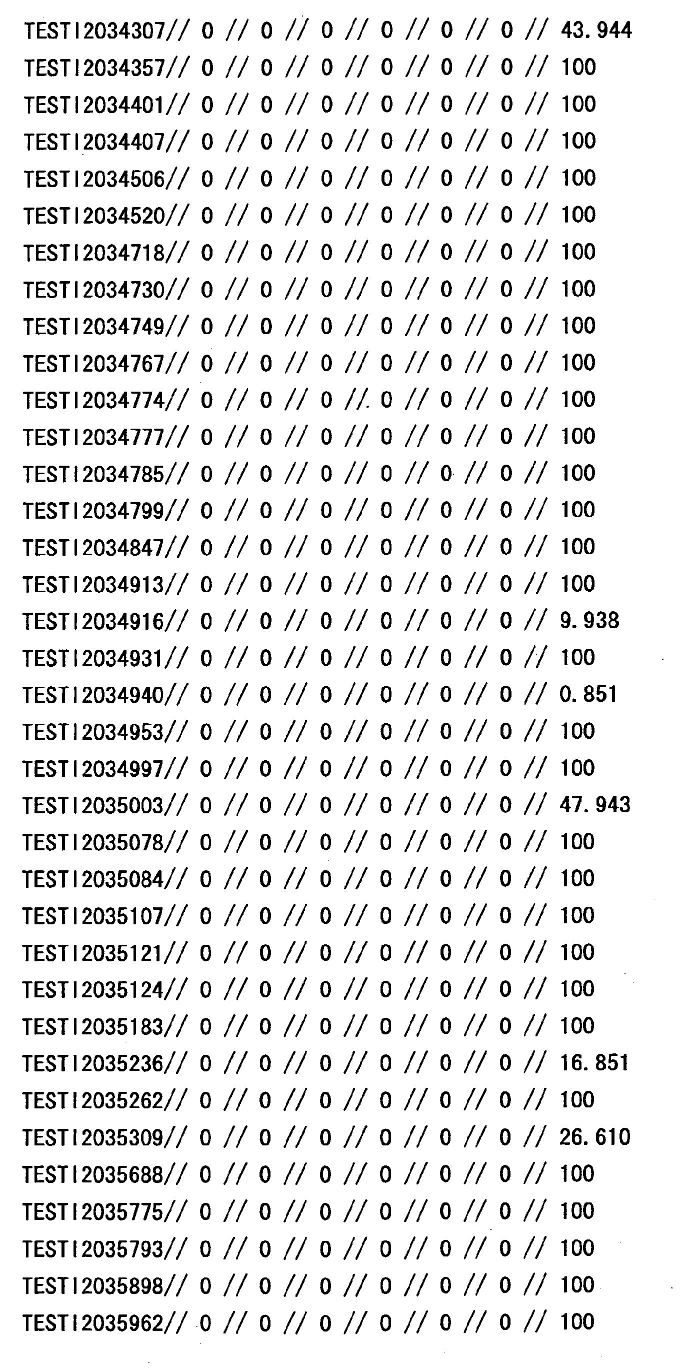

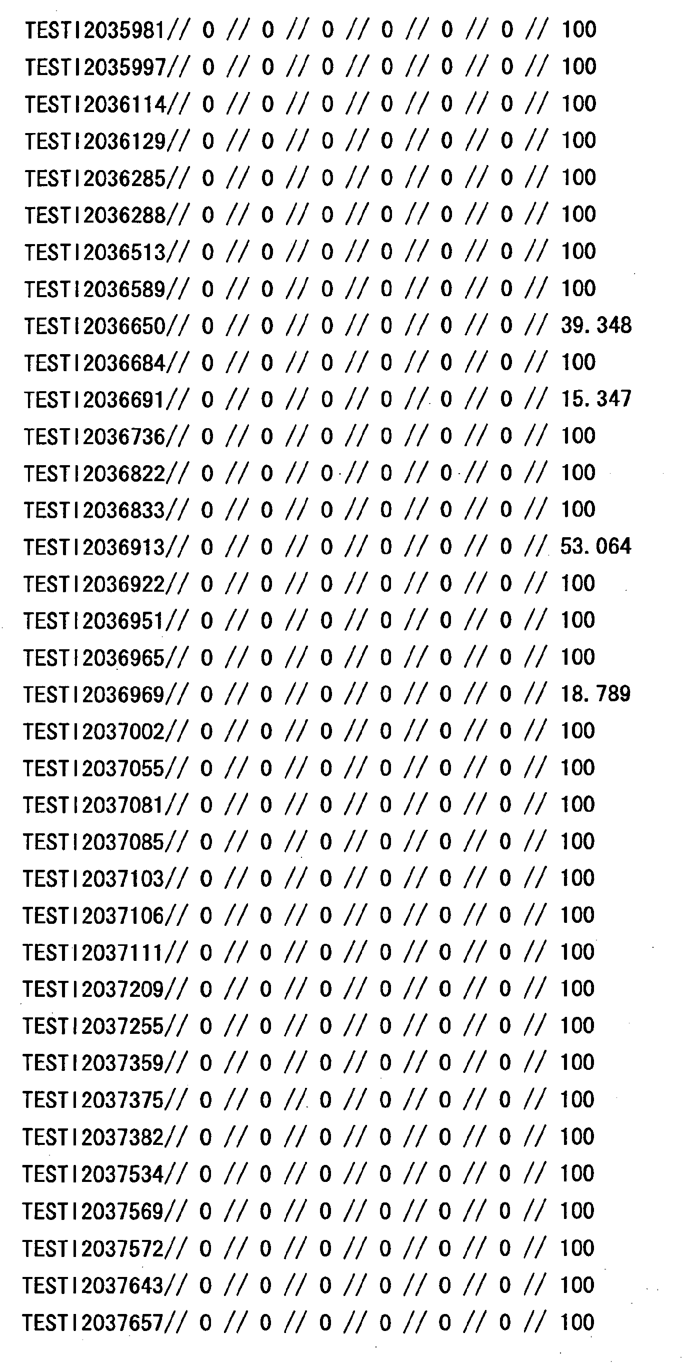

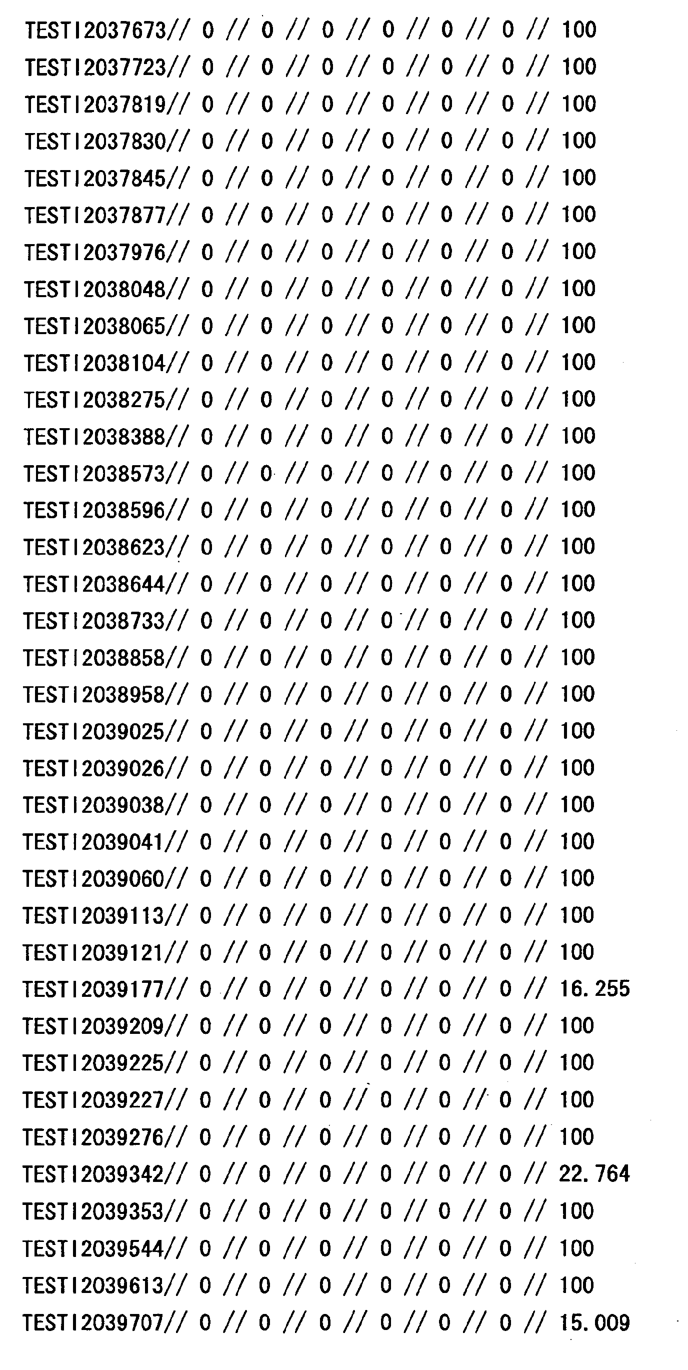

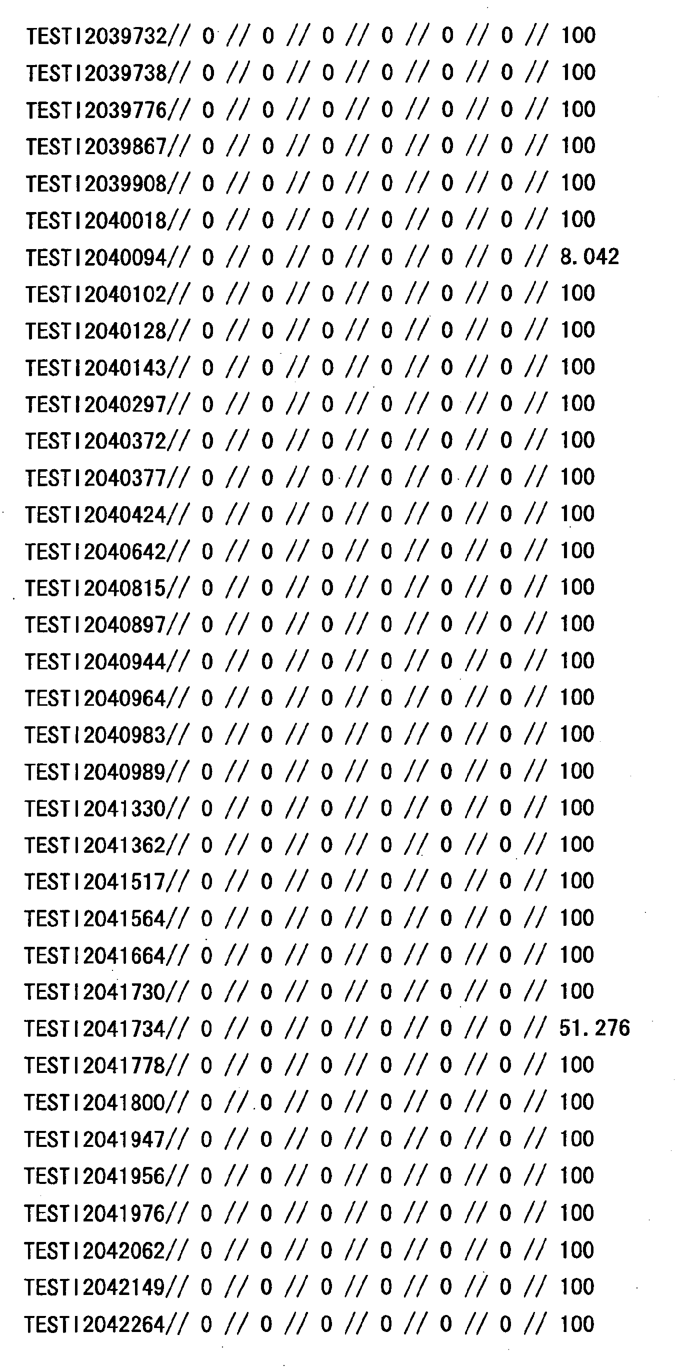

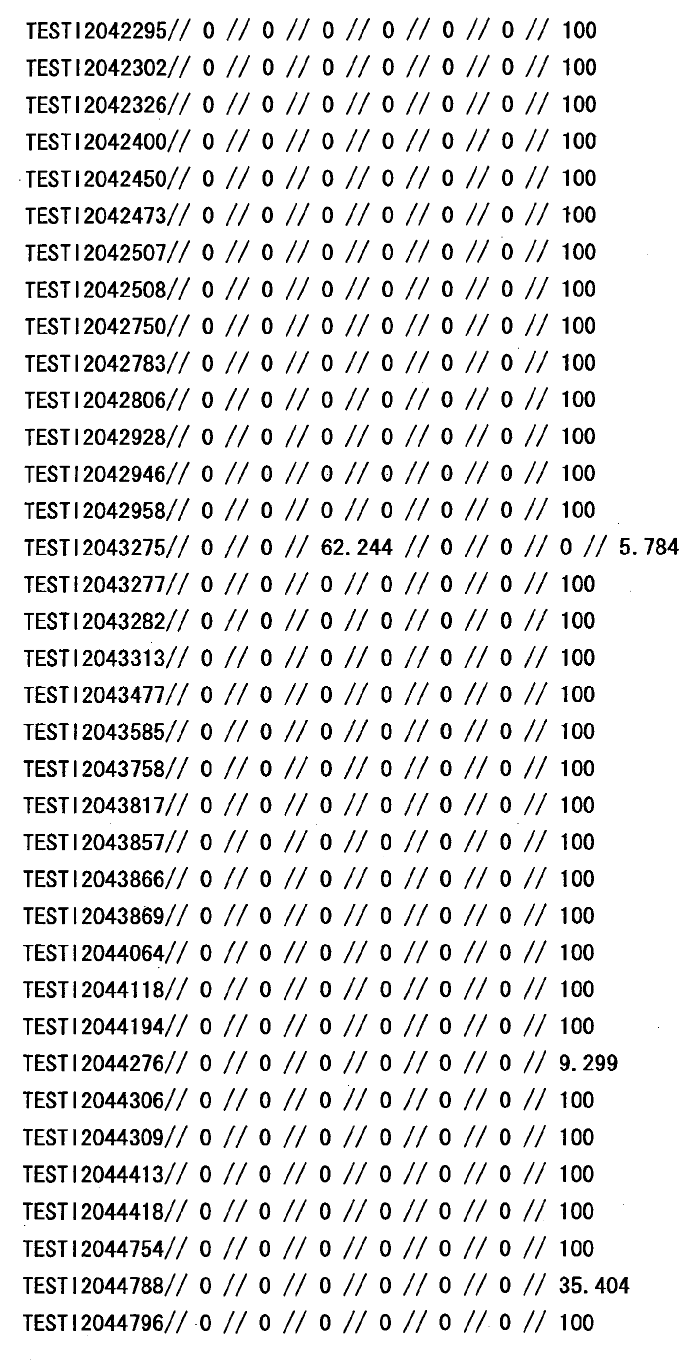

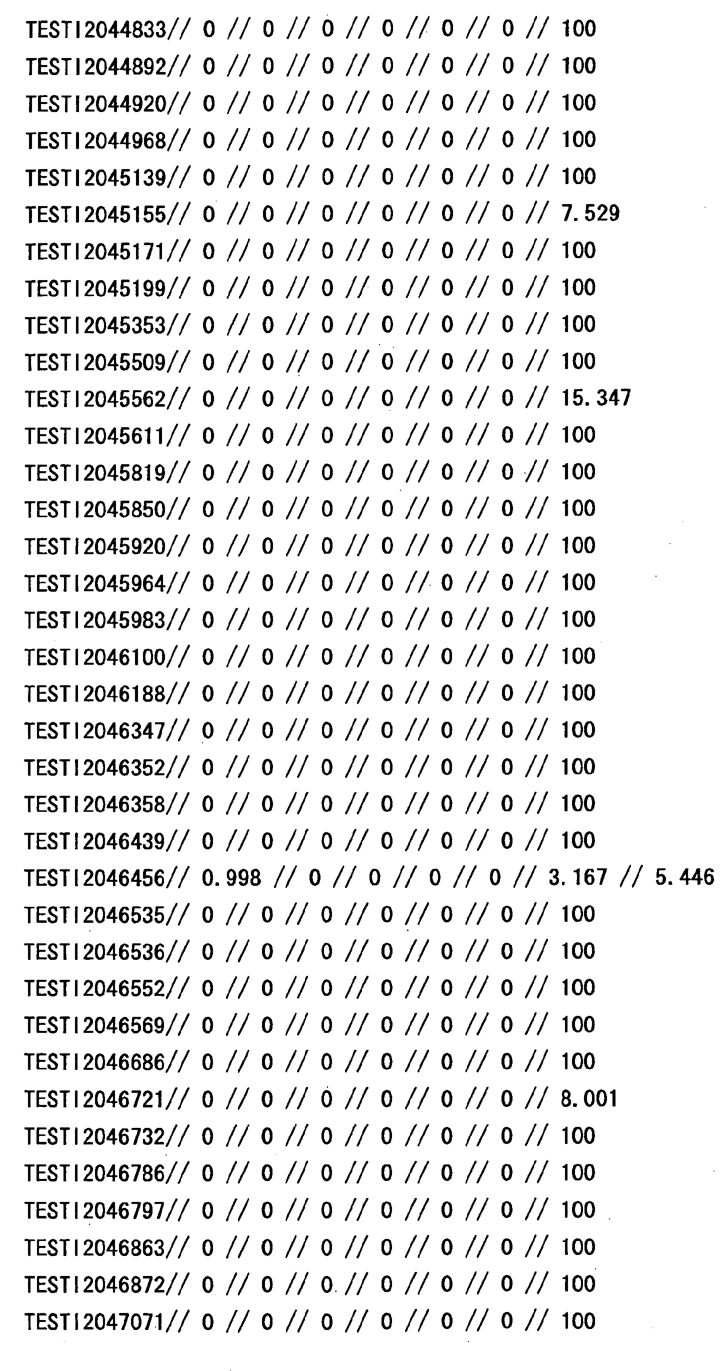

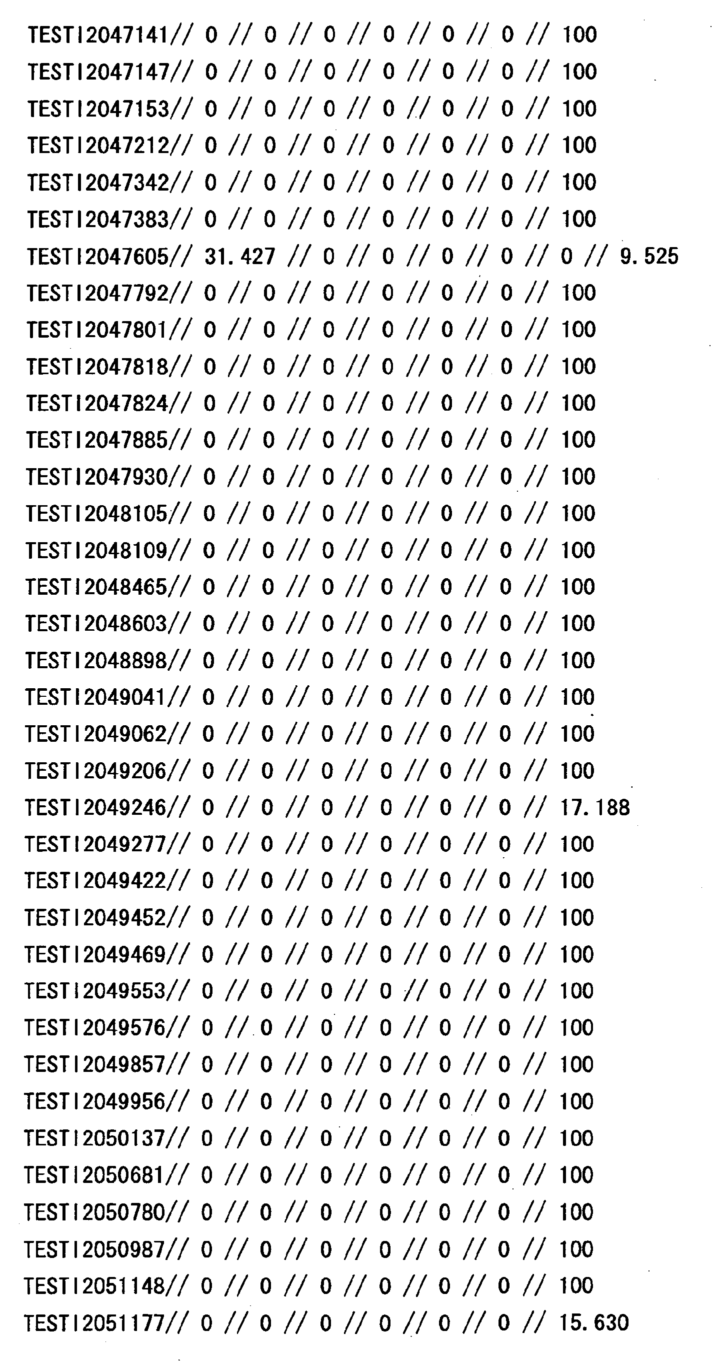

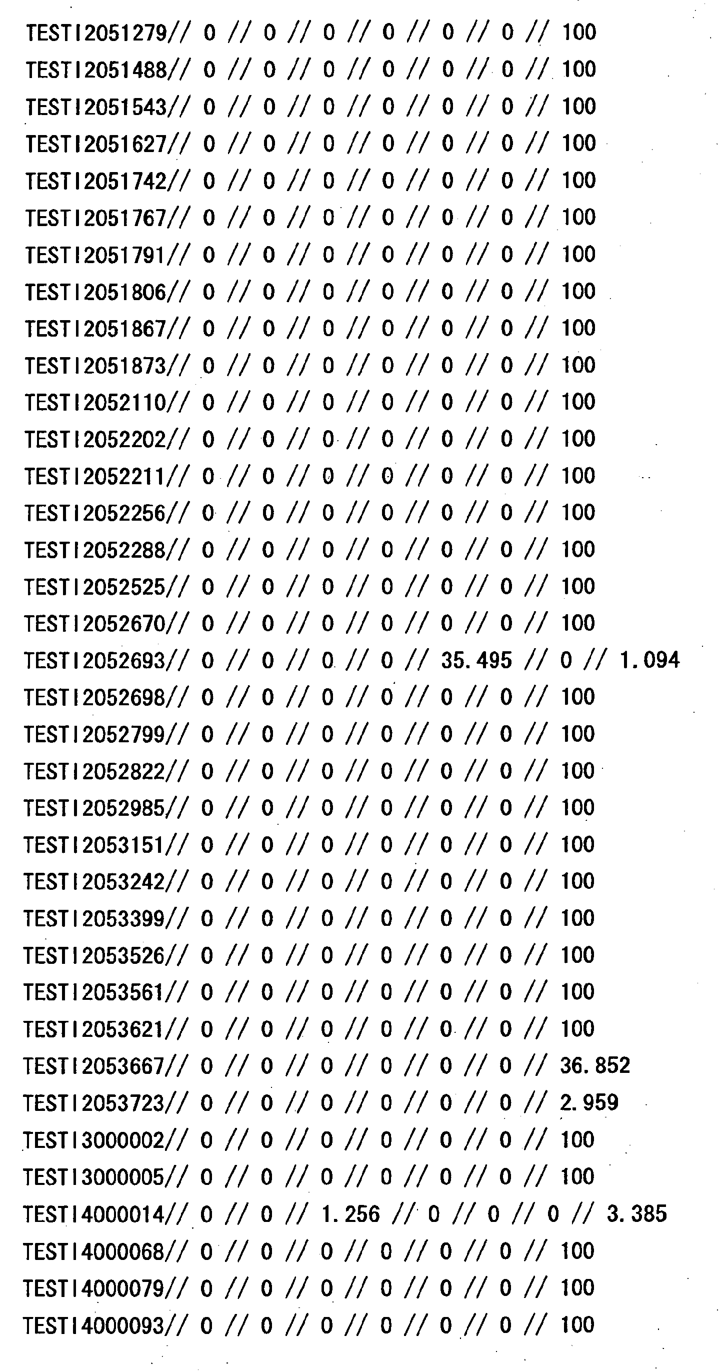

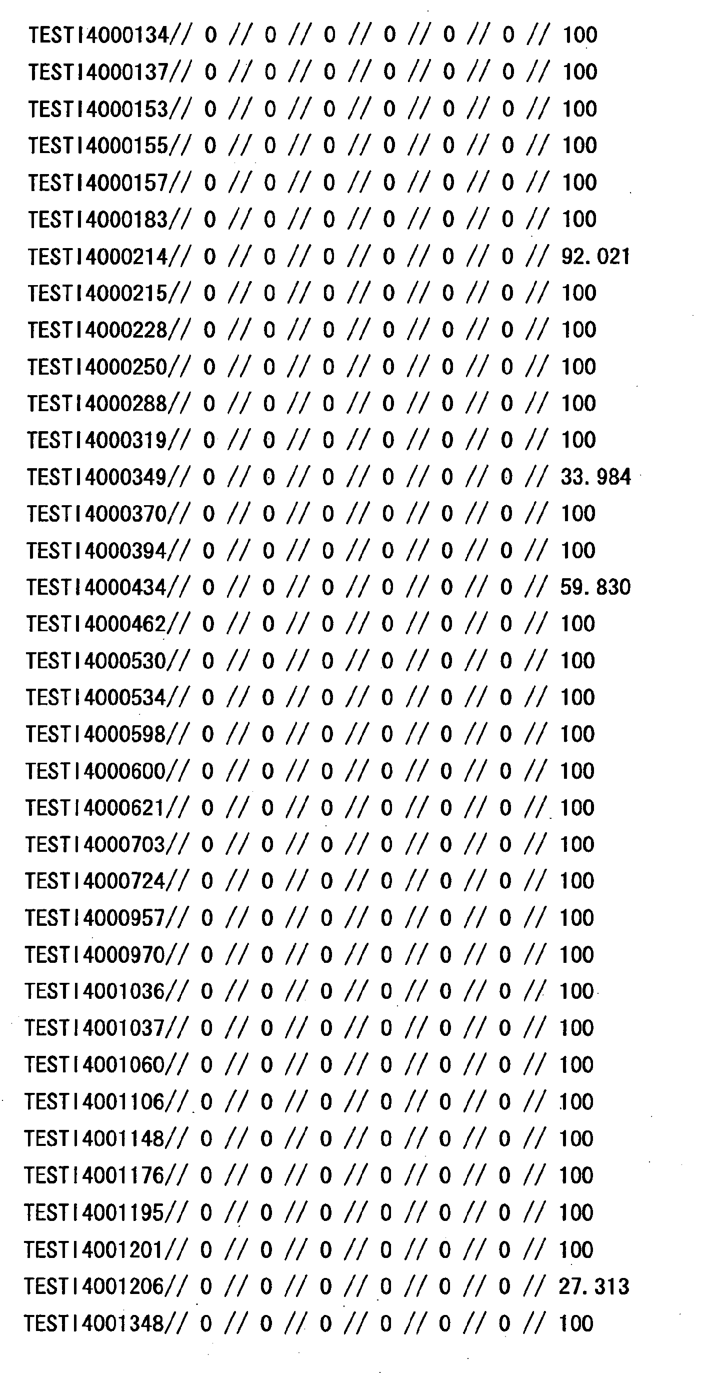

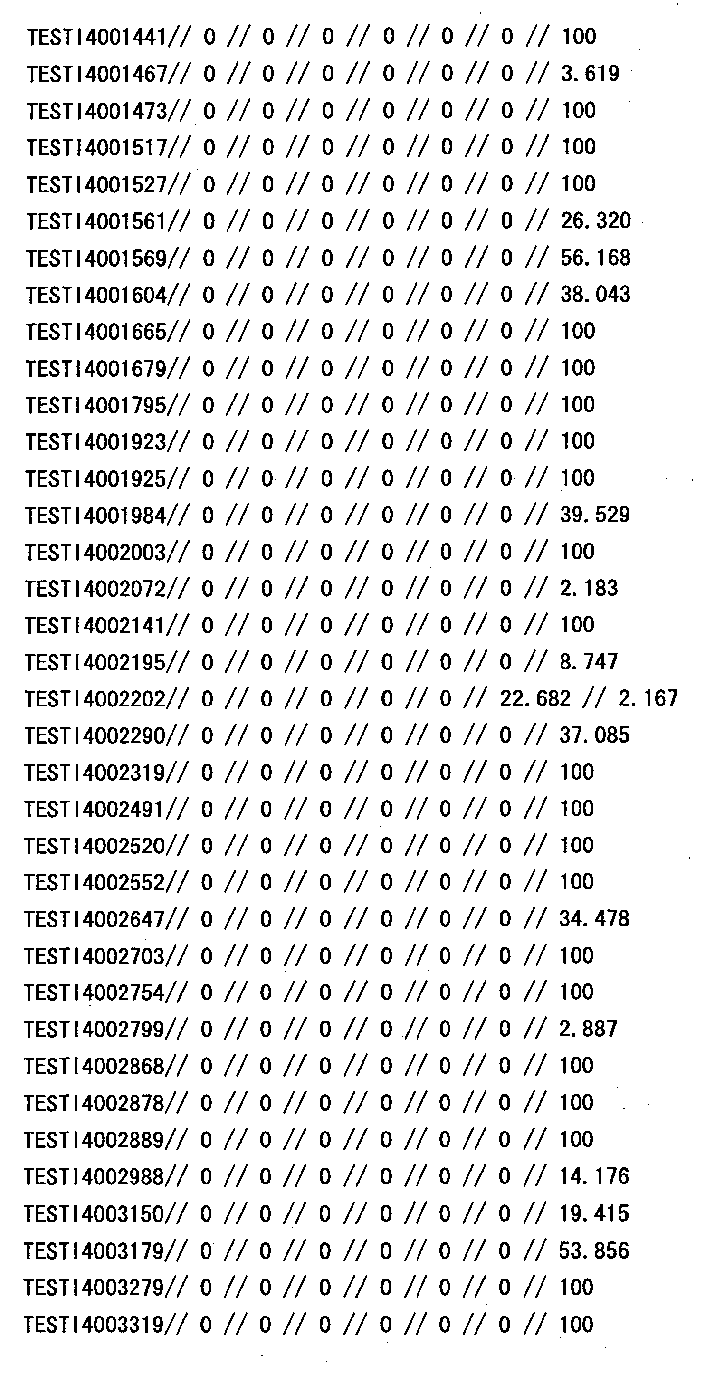

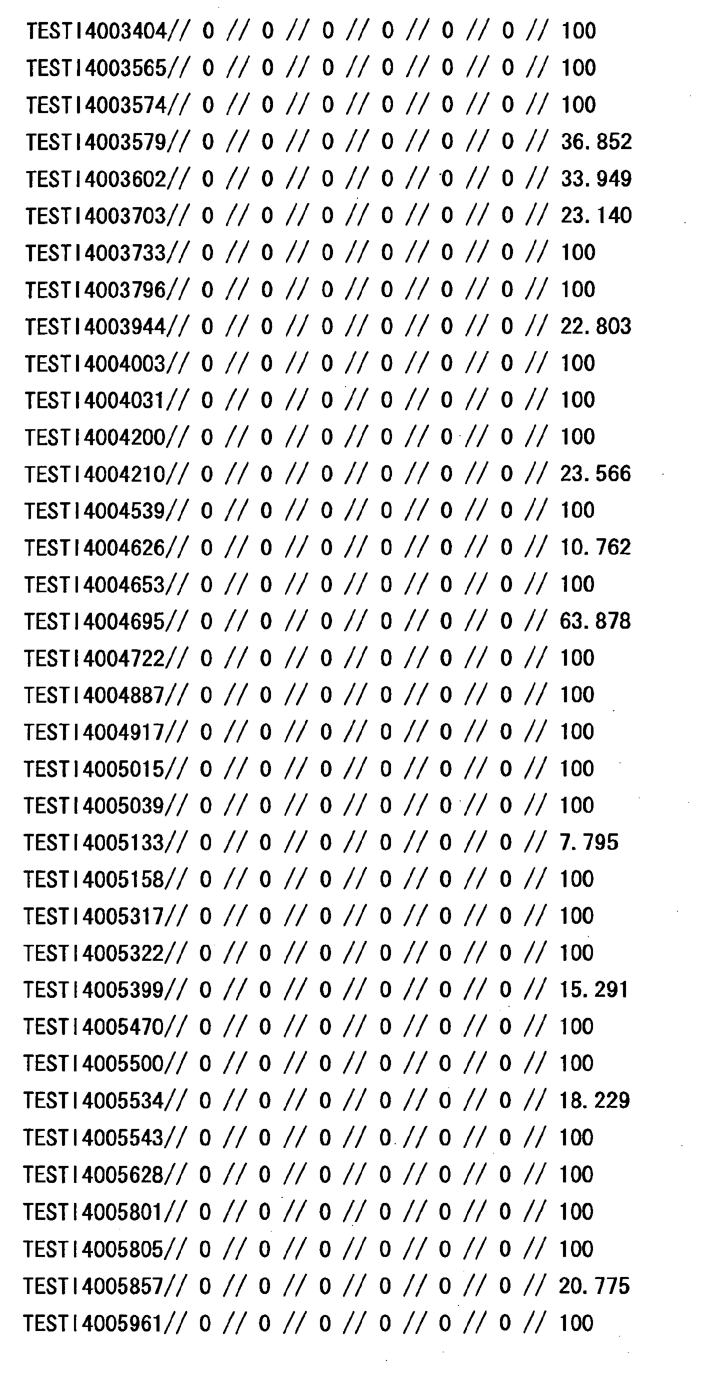

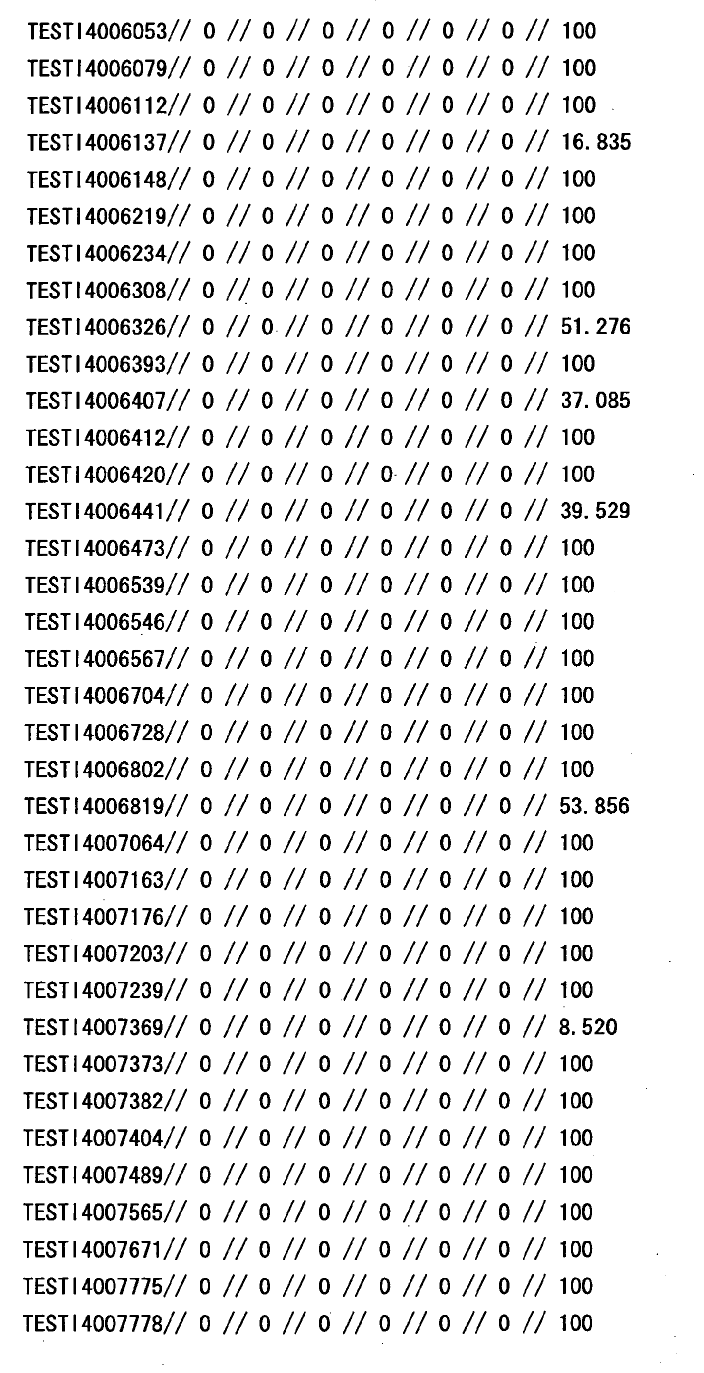

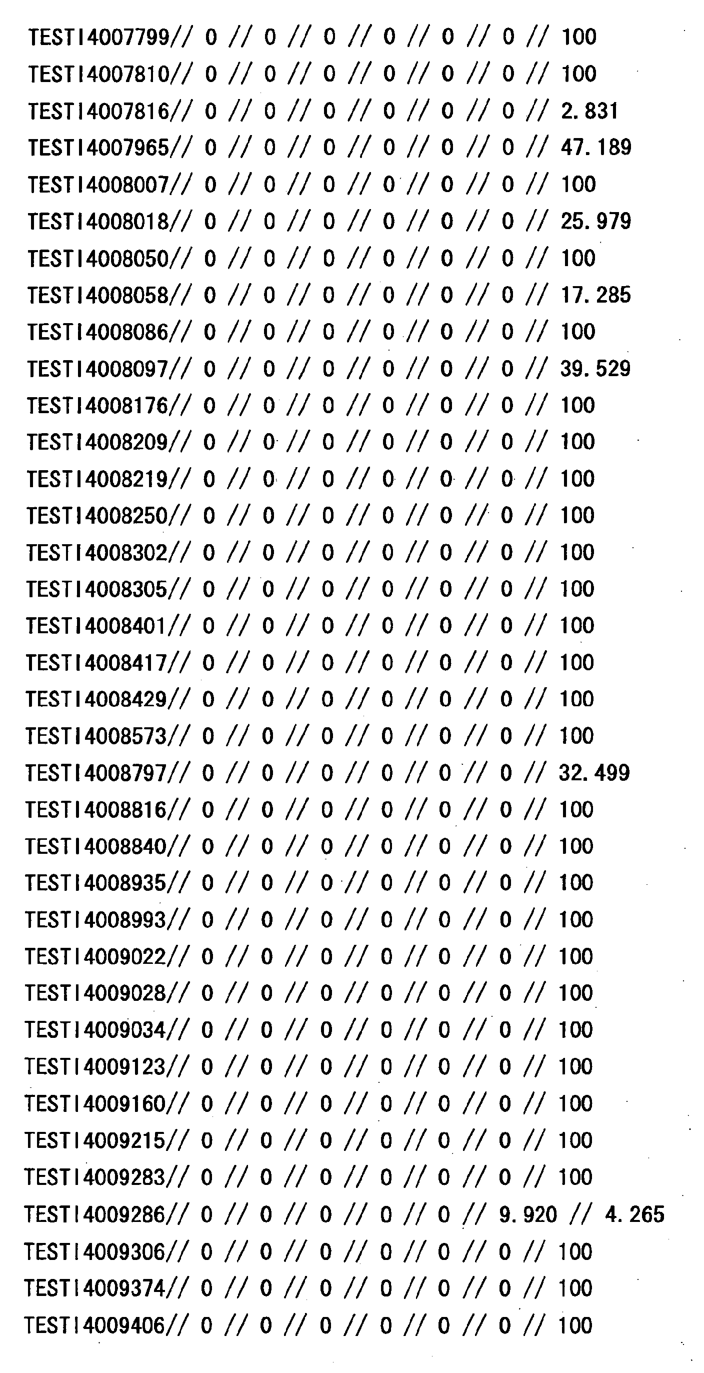

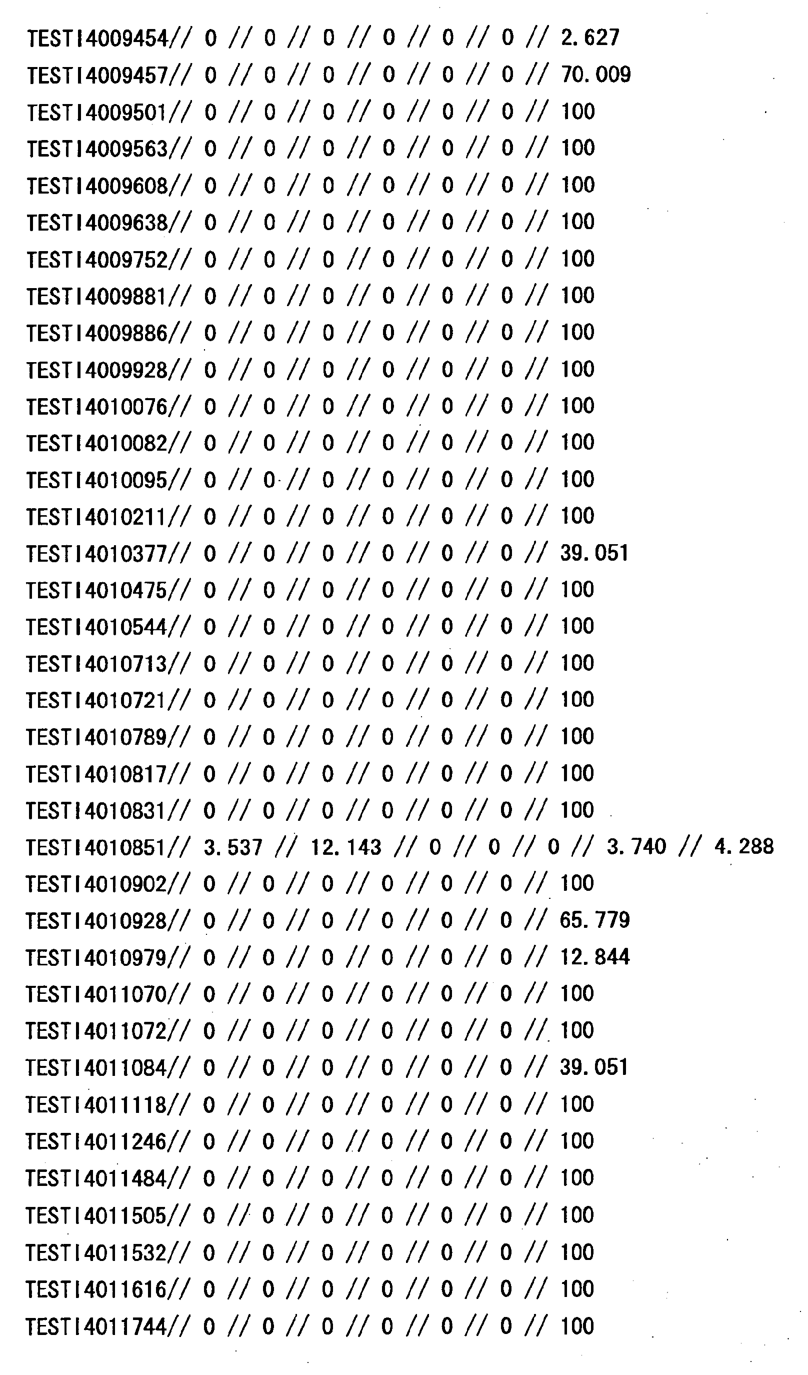

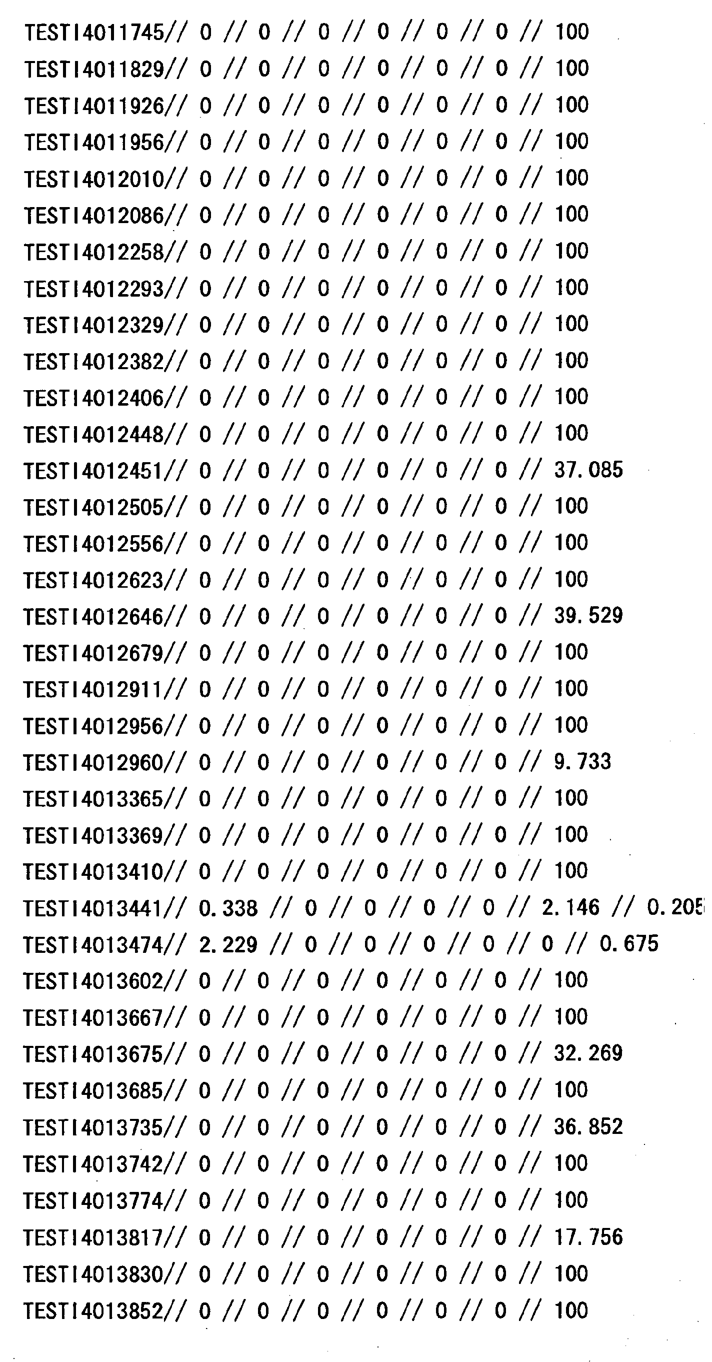

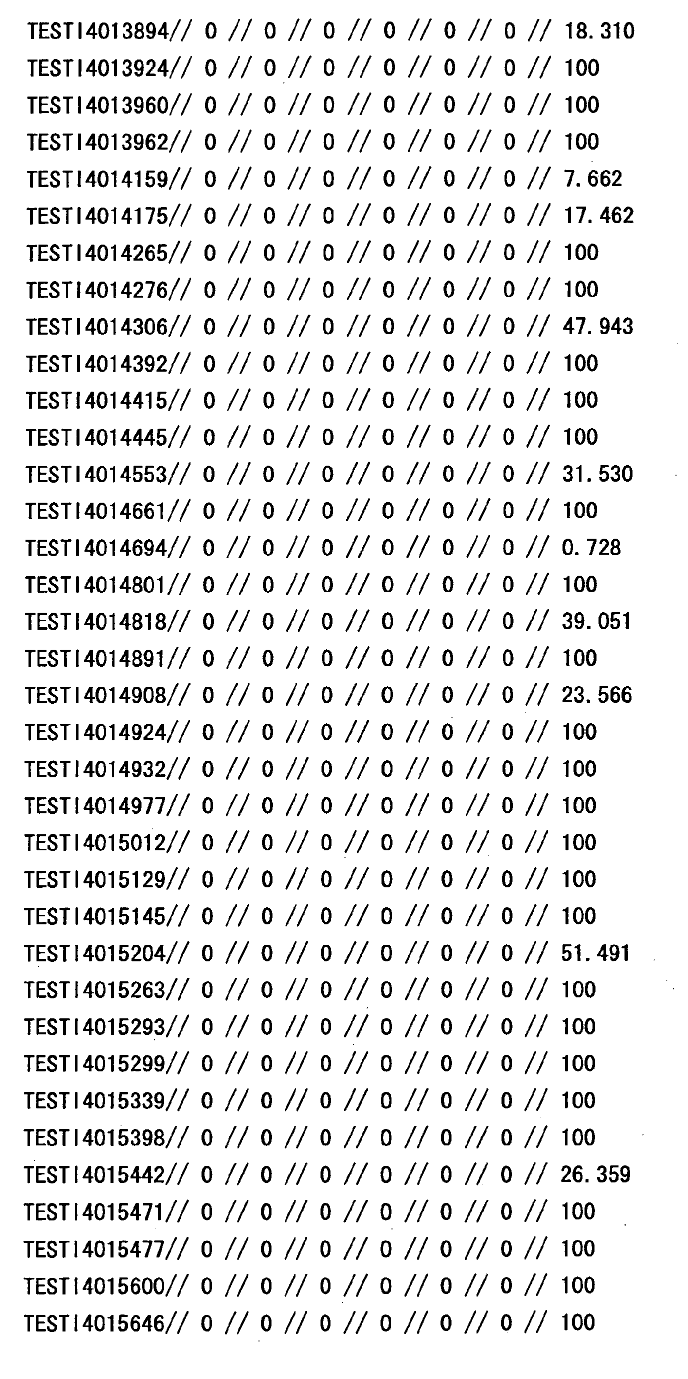

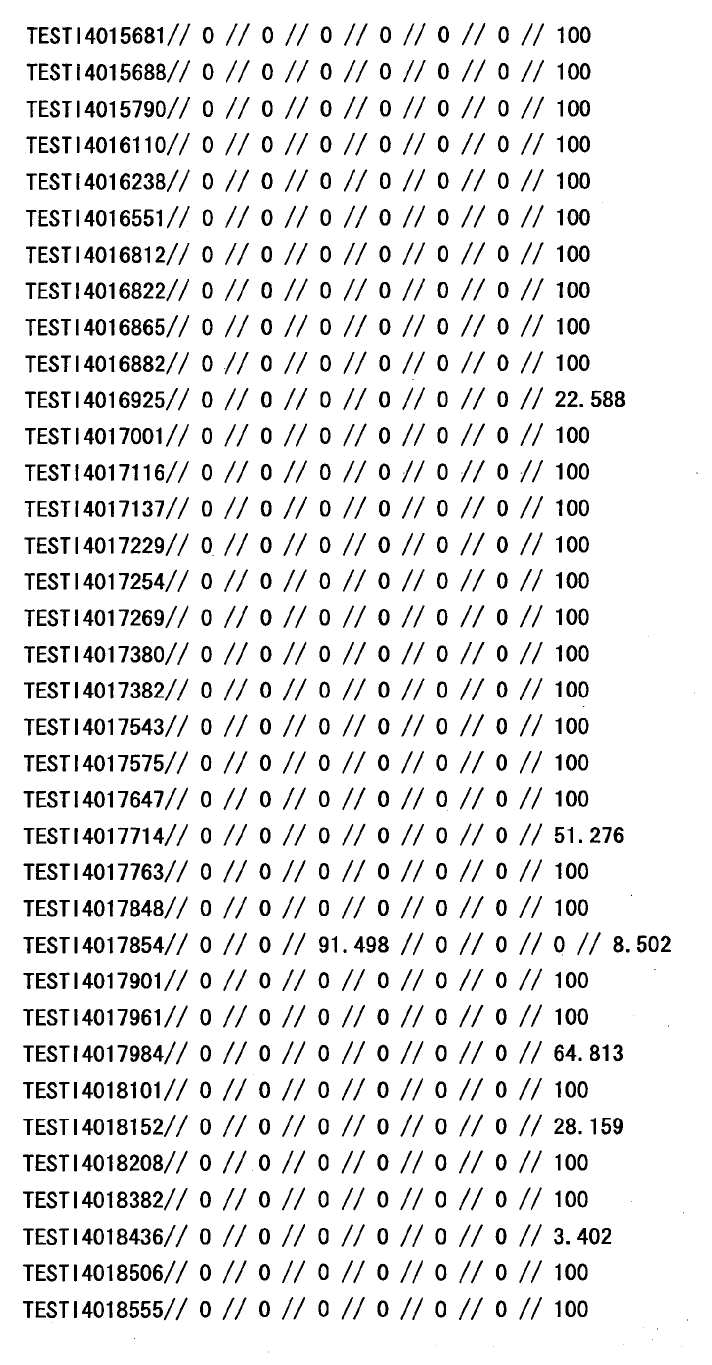

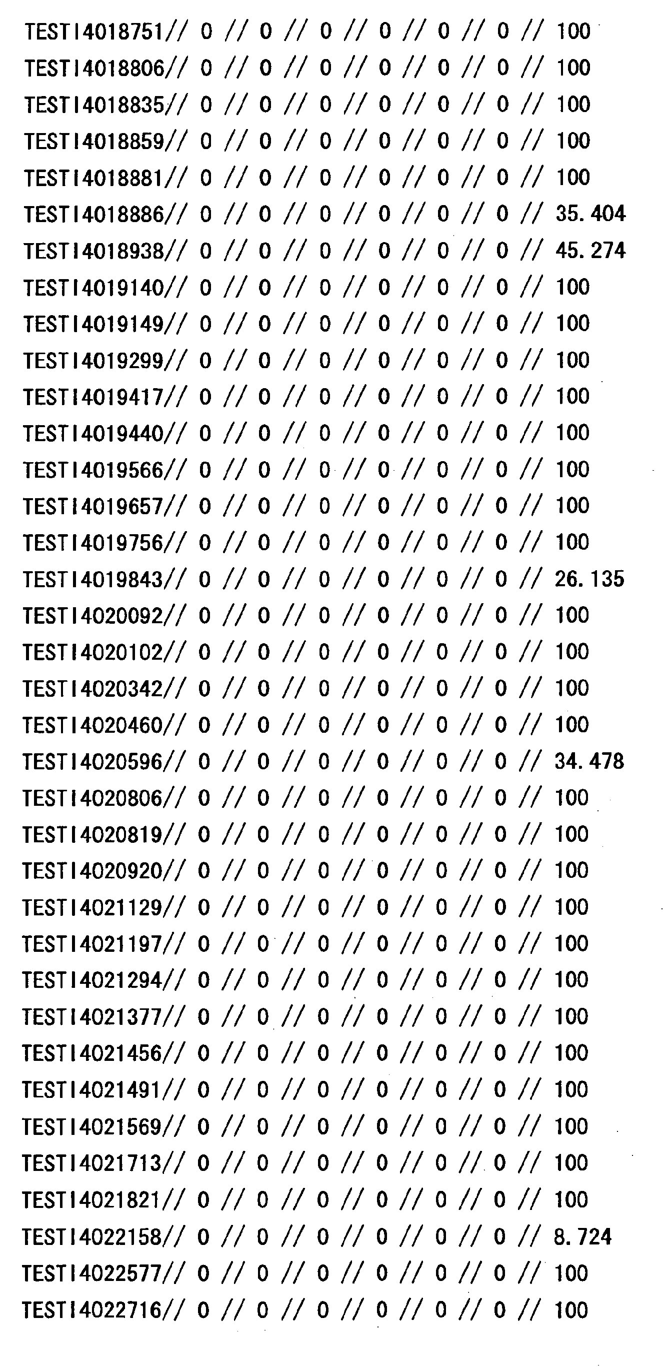

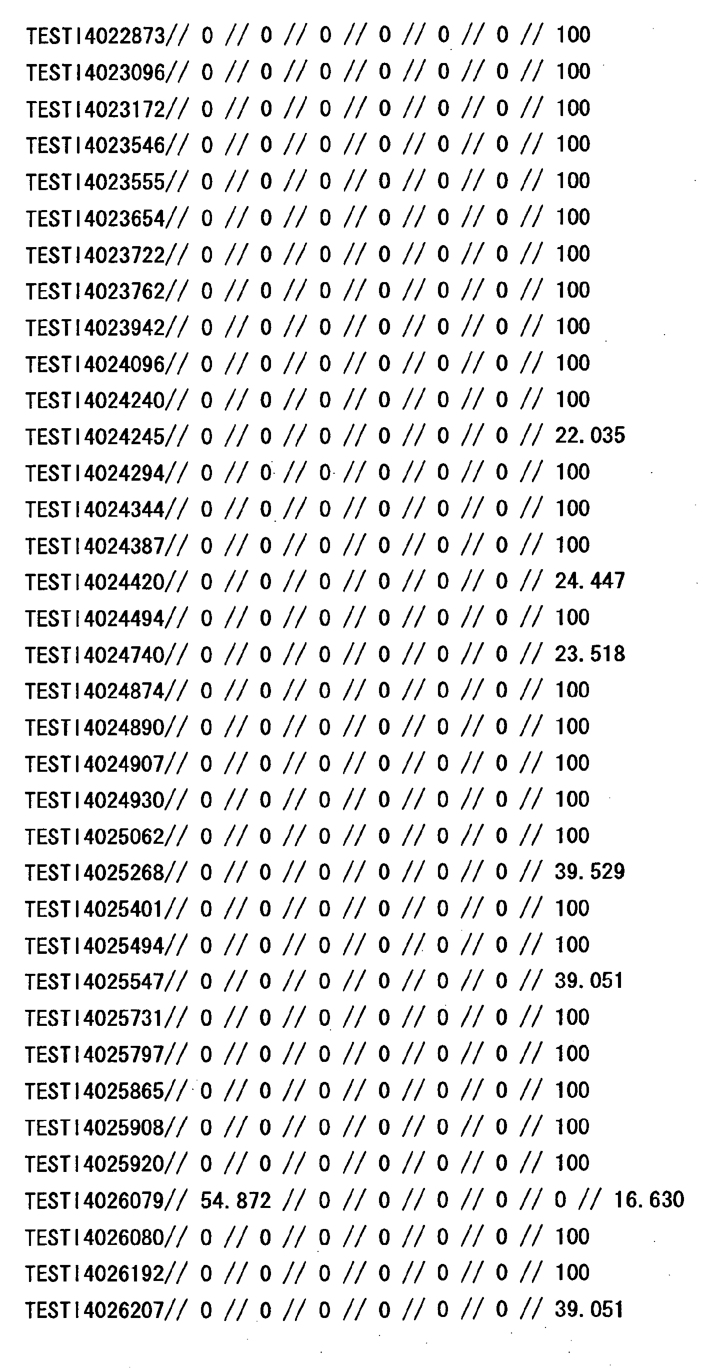

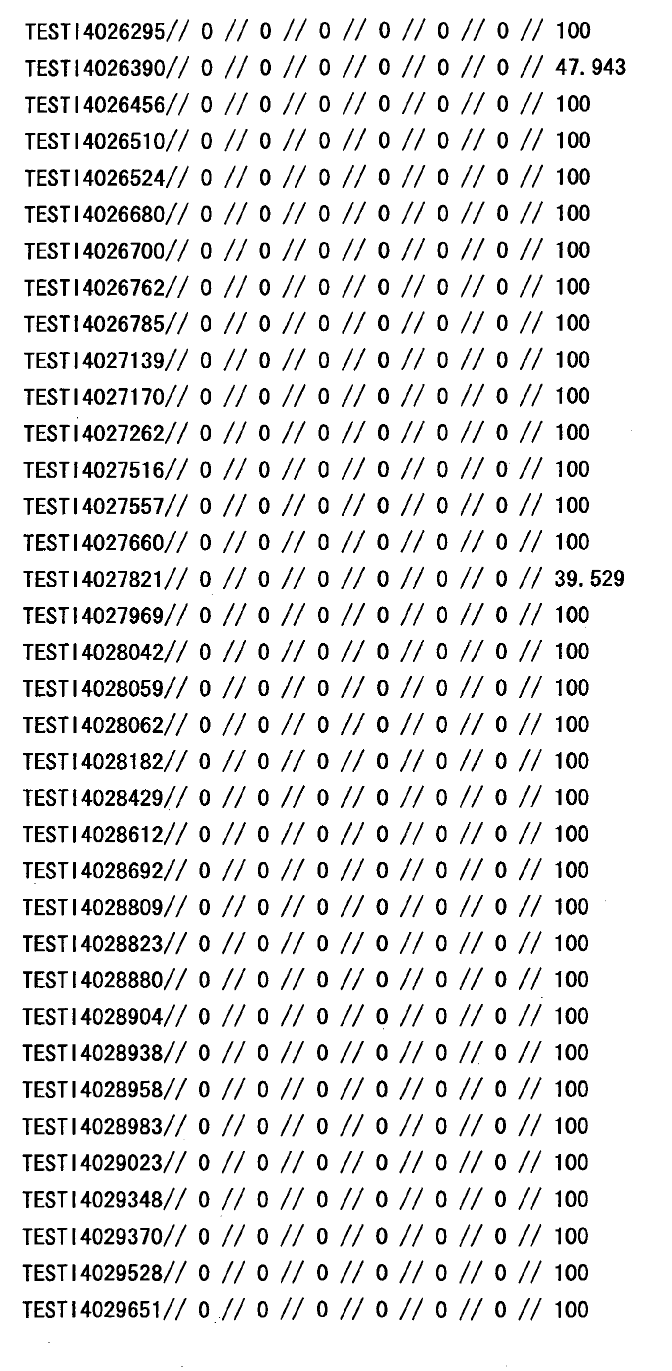

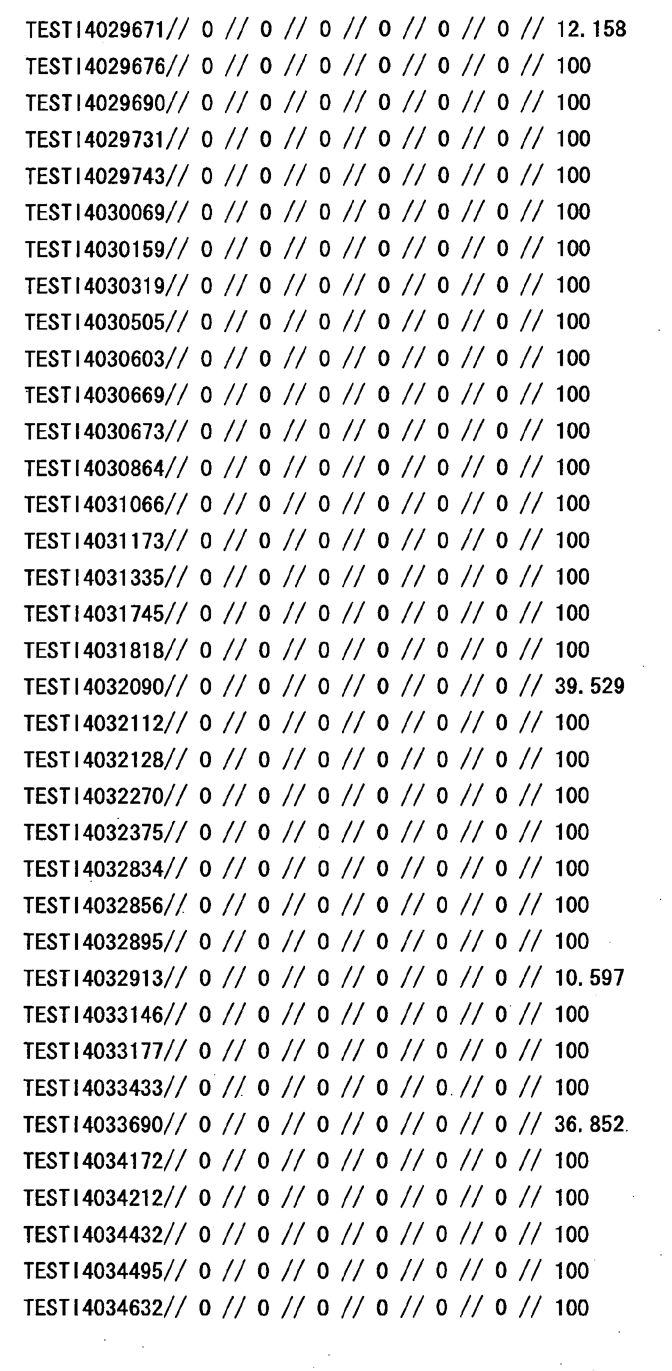

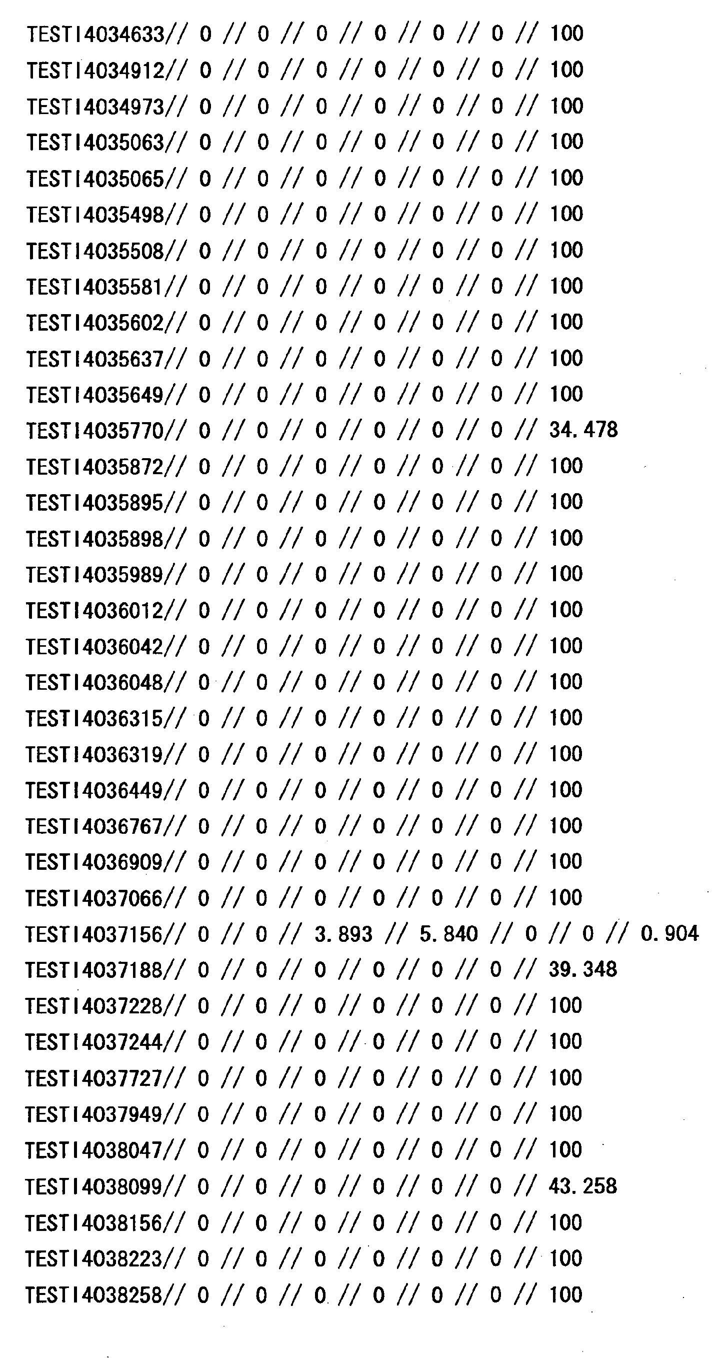

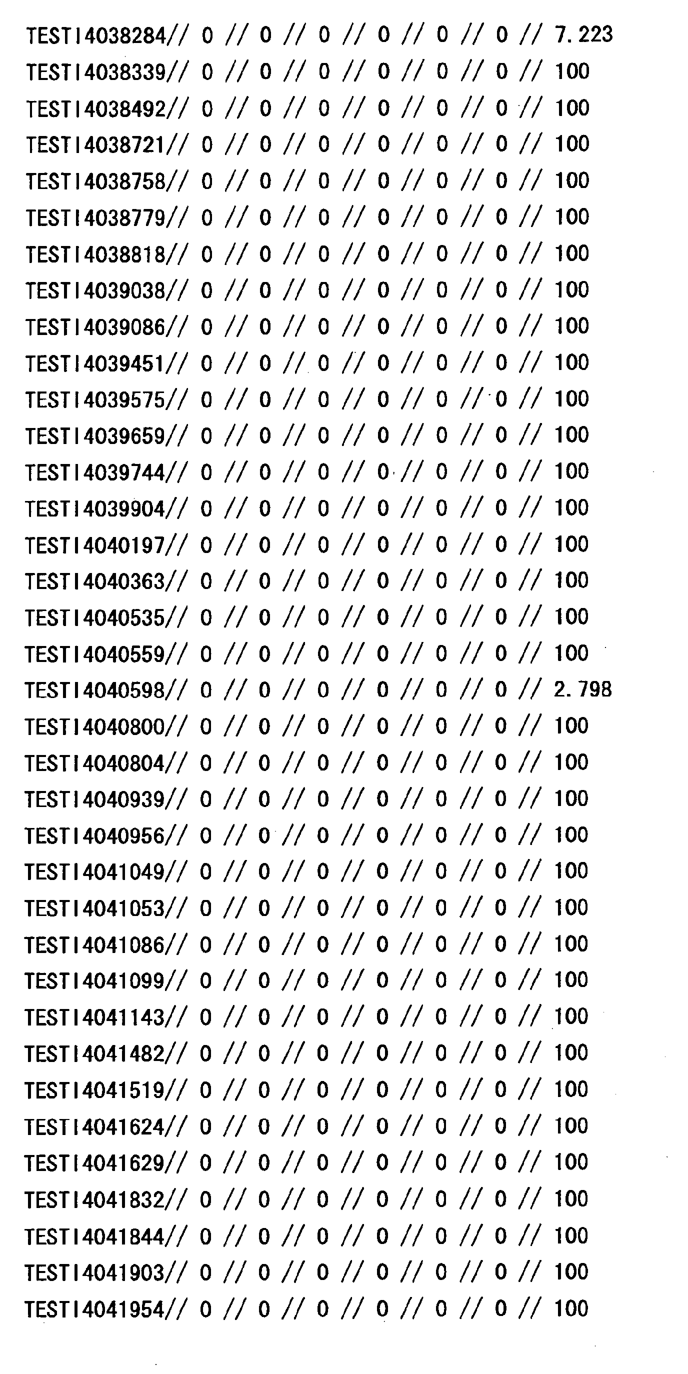

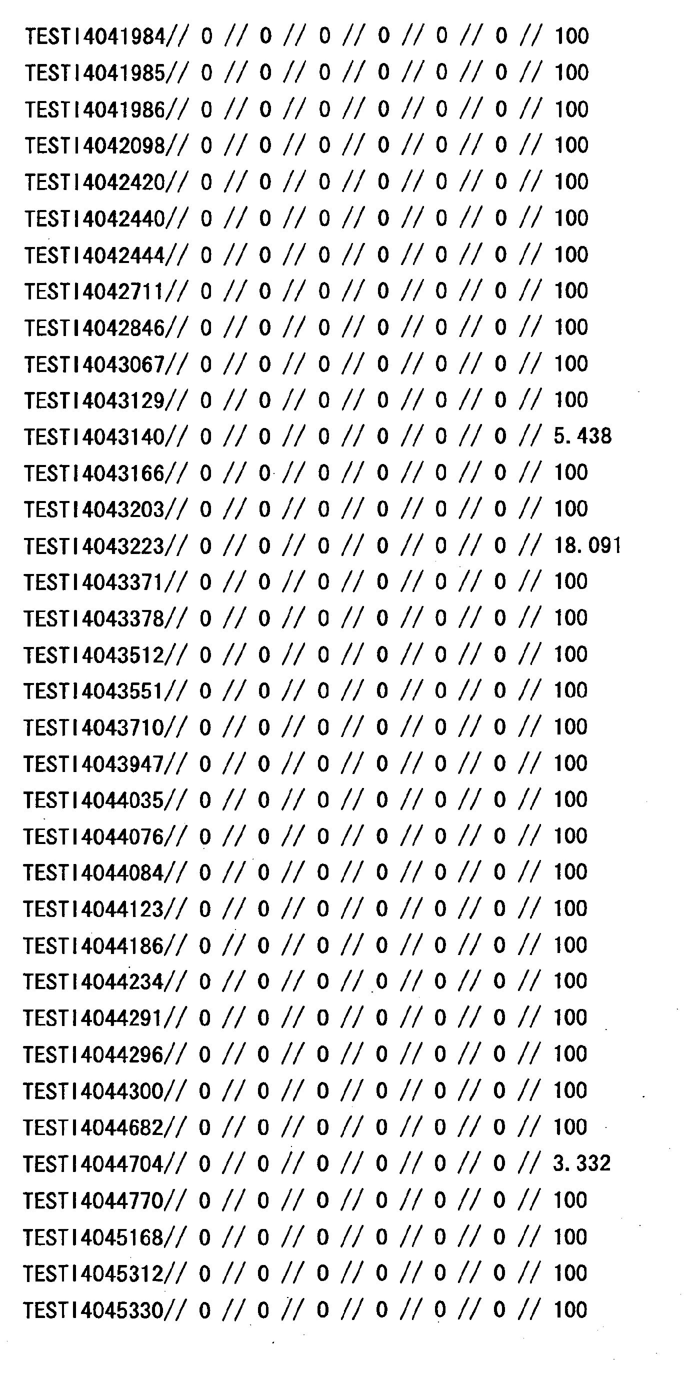

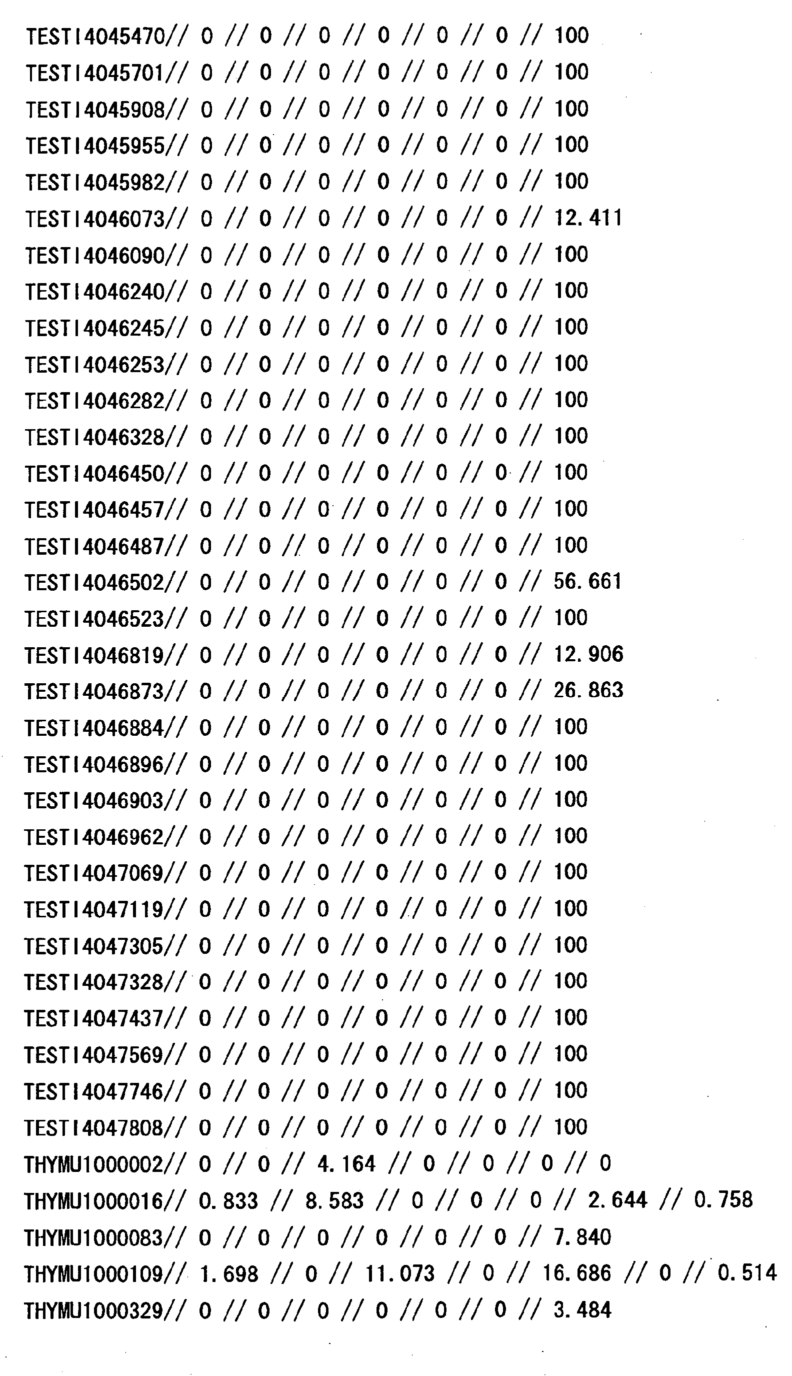

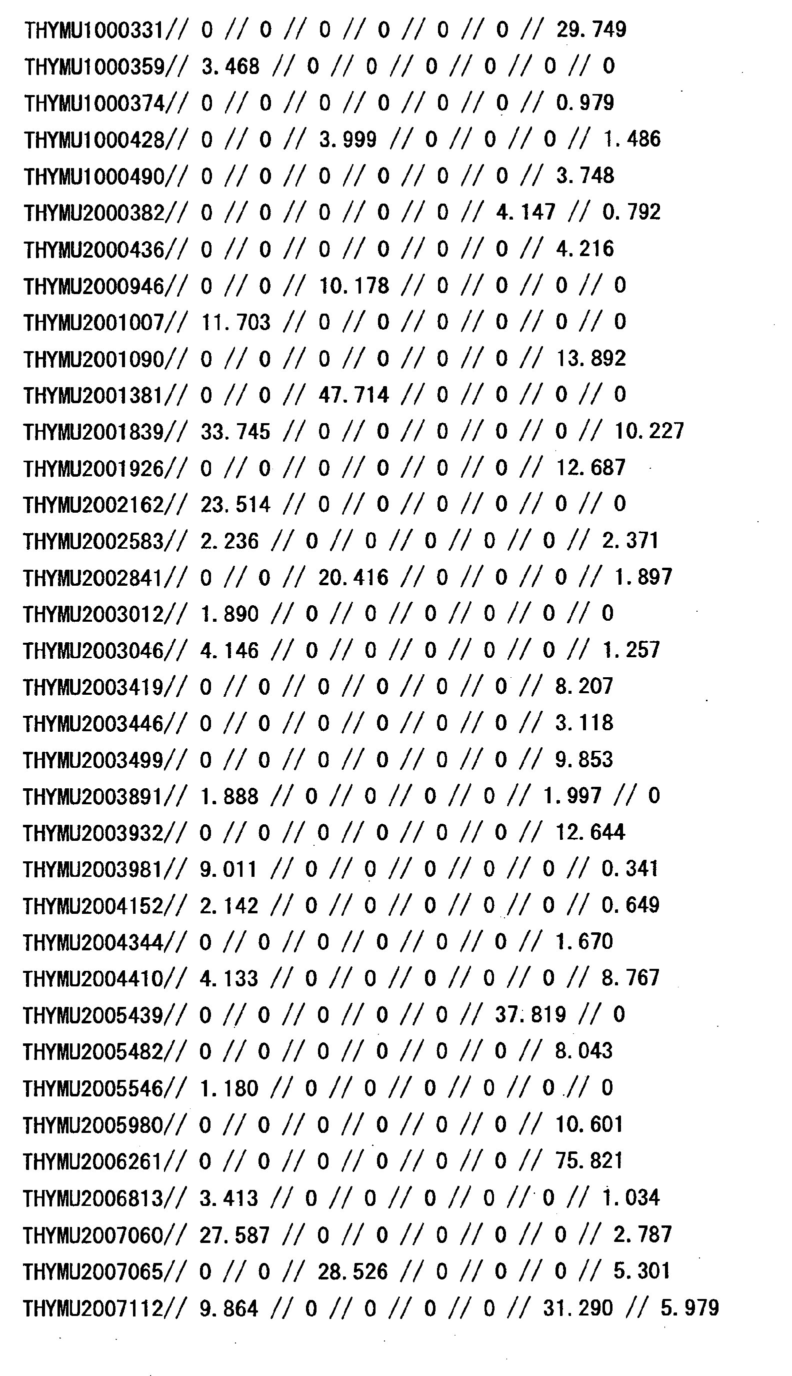

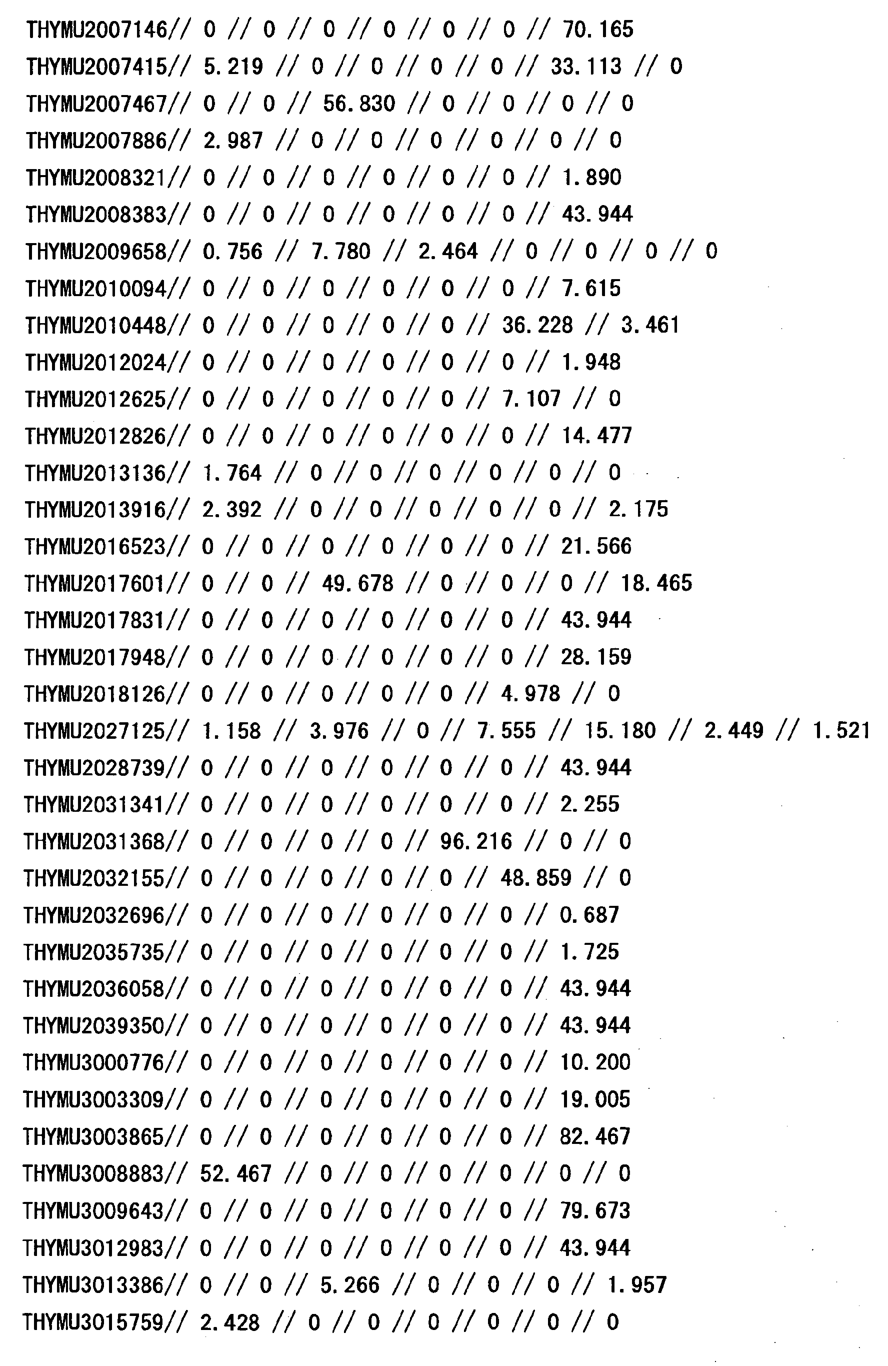

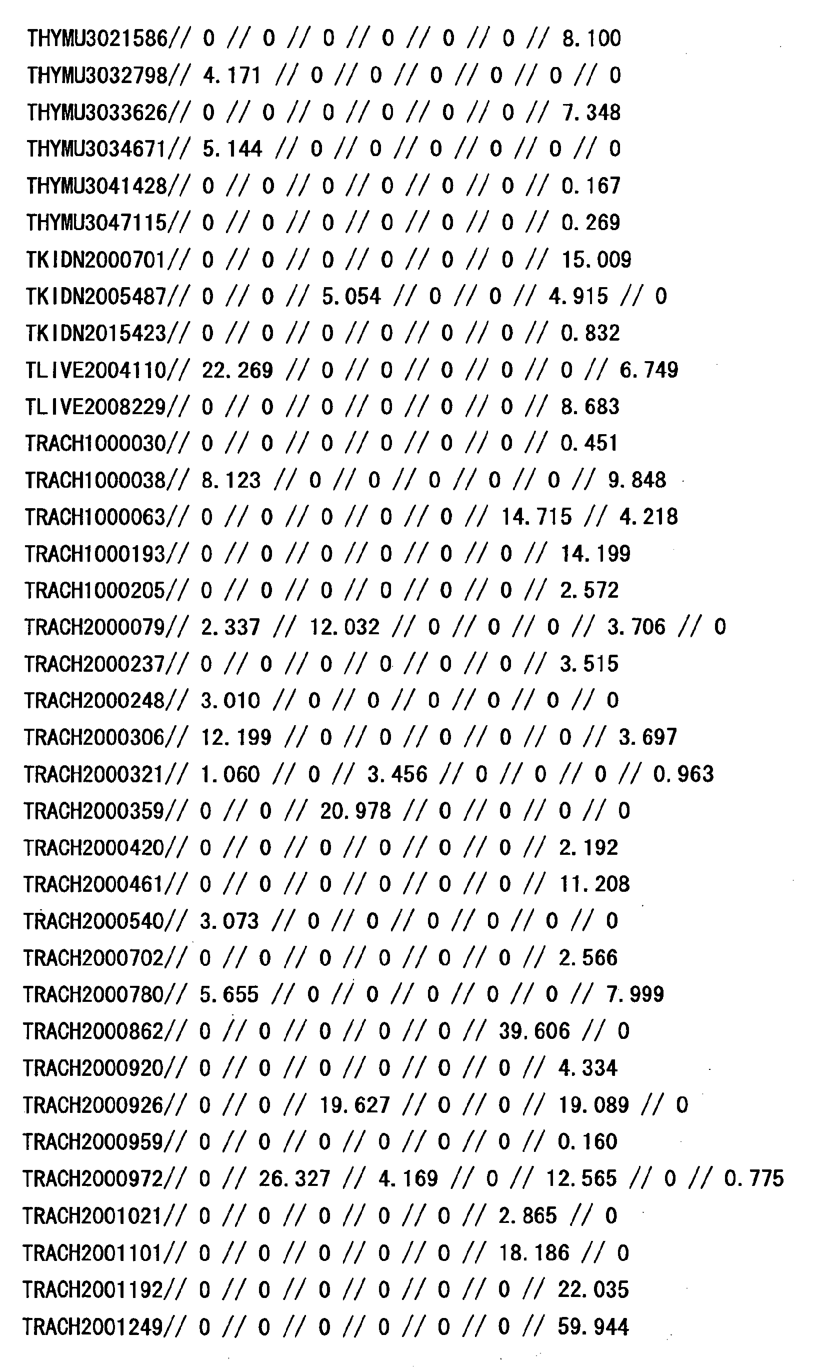

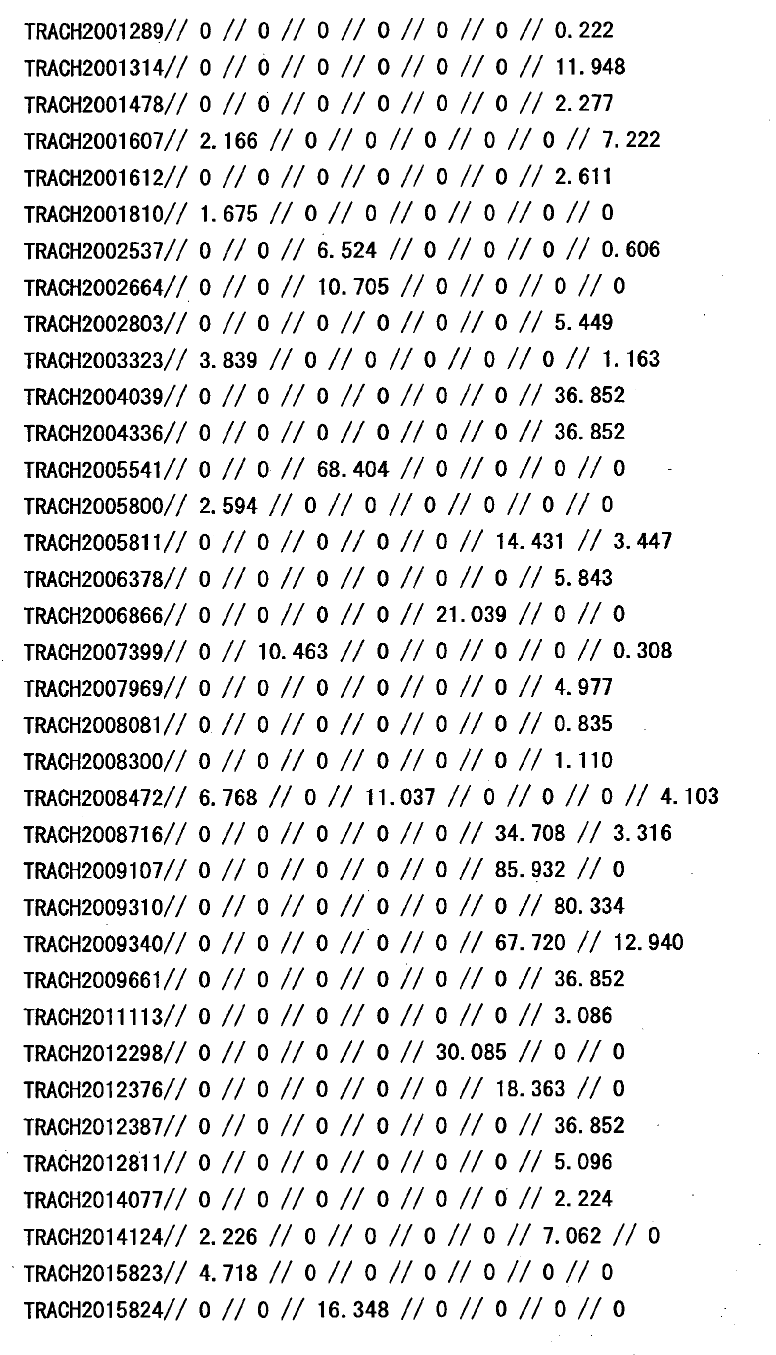

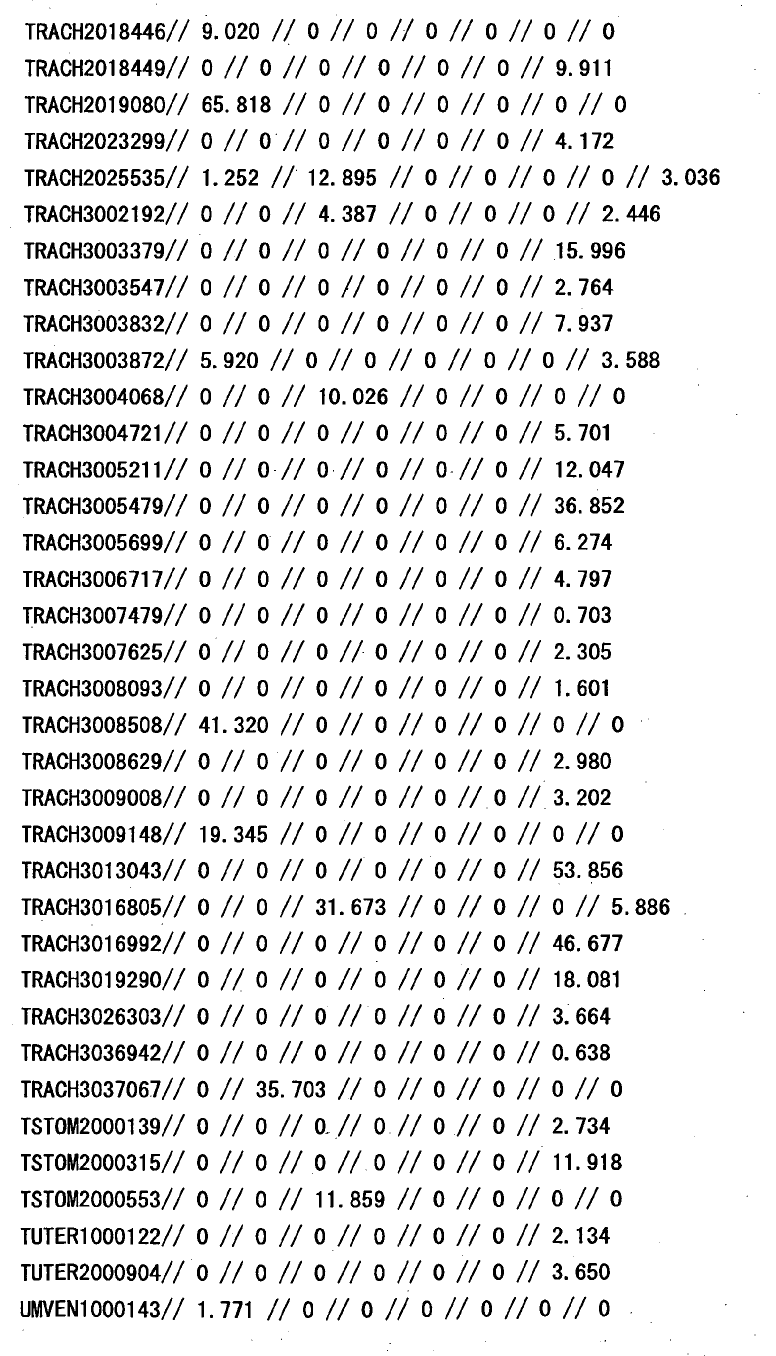

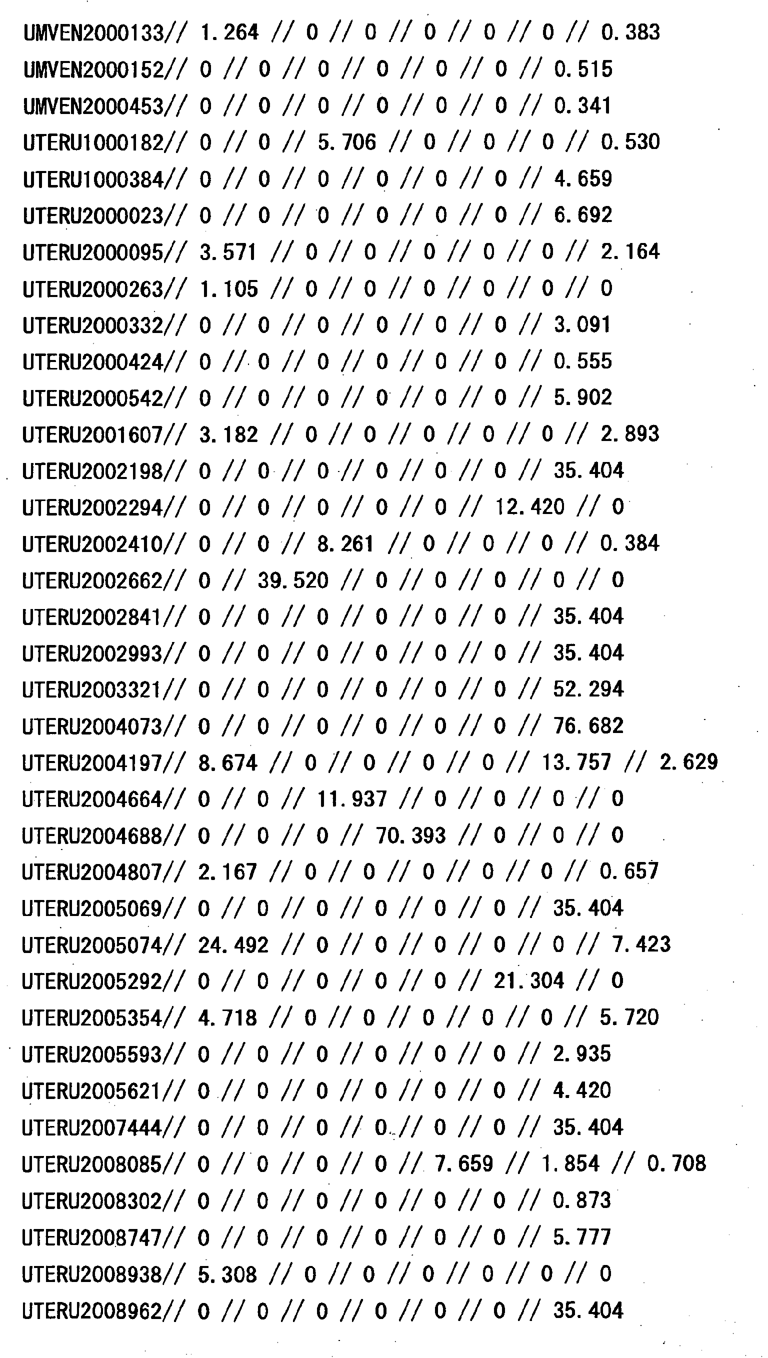

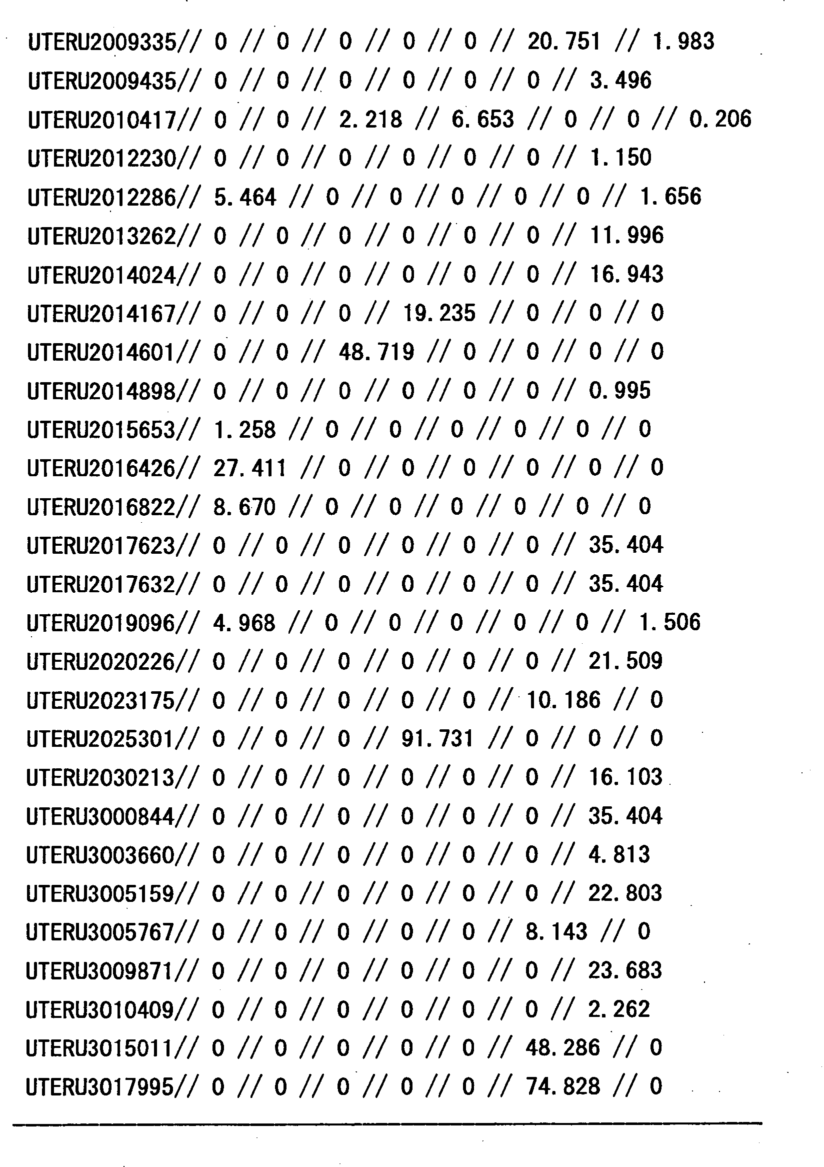

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000973, 3NB692001002, 3NB692001022, 3NB692001034, 3NB692001040, 3NB692001123, 3NB692001267, 3NB692001288, 3NB692001339, 3NB692001349, 3NB692001408, 3NB692001433, 3NB692001459, 3NB692001471, 3NB692001501, 3NB692001507, 3NB692001519, 3NB692001520, 3NB692001528, 3NB692001538, 3NB692001548, 3NB692001637, 3NB692001719, 3NB692001853, 3NB692001861, 3NB692002051, 3NB692002365, 3NB692002685, 3NB692002806, 3NB692003538, 3NB692004045, 3NB692004724, 3NB692005439, 3NB692006952, 3NB692008729, ACTVT2000380, ADIPS1000064,
ADIPS2000069, ADIPS2000088, ADIPS2000245, ADIPS2000425, ADRGL1000002, ADRGL1000033, ADRGL1000038, ADRGL1000067, ADRGL1000111, ADRGL1000-144, ADRGL1000147, ADRGL1000160, ADRGL1000165, ADRGL1000182, ADRGL2000056, ADRGL2000064, ADRGL2000074, ADRGL2000085, ADRGL2000142, ADRGL2000172, ADRGL2000248, ADRGL2000261, ADRGL2000323, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002260, ADRGL2002392, ADRGL2002477, ADRGL2002679, ADRGL2002753, ADRGL2002857, ADRGL2003017,
ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004676, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000095, ASTRO2000191, ASTRO2000278, ASTRO2000372, ASTRO2000381, ASTRO2000403, ASTRO2000417, ASTRO2000480, ASTRO2000482, ASTRO2000533, ASTRO2000653, ASTRO2000662, ASTRO2000725, ASTRO2000801, ASTRO2000914, ASTRO2001001, ASTRO2001008, ASTRO2001088, ASTRO2001107, ASTRO2001122, ASTRO2001227, ASTRO2001249, ASTRO2001480, ASTRO2002035, ASTRO2002064, ASTRO2002202, ASTRO2002571,
ASTRO2002632, ASTRO2002659, ASTRO2002693, ASTRO2002733, ASTRO2002743, ASTRO2003461, ASTRO2003574, ASTRO2003581, ASTRO2003740, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2004974, ASTRO2005008, ASTRO2005081, ASTRO2005096, ASTRO2005242, ASTRO2005273, ASTRO2005343, ASTRO2005413, ASTRO2005423, ASTRO2005553, ASTRO2005557, ASTRO2005575, ASTRO2005593, ASTRO2005726, ASTRO2005863, ASTRO2005896, ASTRO2005902, ASTRO2006356, ASTRO2006475, ASTRO2006732, ASTRO2006747, ASTRO2006920, ASTRO2006952, ASTRO2007221, ASTRO2007377, ASTRO2007515, ASTRO2007578-, ASTRO2007643, ASTRO2007666, ASTRO2007956, ASTRO2007962,
ASTRO2008040, ASTRO2008216, ASTRO2008239, ASTRO2008409, ASTRO2008425, ASTRO2008508, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010582, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013671, ASTRO2013802, ASTRO2014135, ASTRO2014174, ASTRO2014211, ASTRO2014363, ASTRO2014392, ASTRO2014498, ASTRO2014561, ASTRO2014863, ASTRO2014923, ASTRO2014961, ASTRO2015162, ASTRO2015185, ASTRO2015328, ASTRO2015987, ASTRO2016044, ASTRO2016148, ASTRO2016681,
ASTRO2016847, ASTRO2016892, ASTRO2017348, ASTRO2017627, ASTRO2018373, ASTRO2019039, ASTRO2019171, ASTRO3000154, ASTRO3000172, ASTRO3000177, ASTRO3000301, ASTRO3000474, ASTRO3000482, BEAST2000454, BGGI11000285, BGGI12000022, BGGI12000067, BGGI12000161, BGGI12000533, BGGI12000544, BGGI12000684, BGGI12000693, BGGI12001002, BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866,- BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987,
BLADE2002073, BLADE2002310, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004722, BLADE2004849, BLADE2005036, BLADE2005038, BLADE2005229, BLADE2005459, BLADE2005792, BLADE2006043, BLADE2006412, BLADE2006415, BLADE2006607, BLADE2006826, BLADE2006849, BLADE2006947, BLADE2007033, BLADE2007043, BLADE2007589, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479,
BLADE2009593, BNGH41000098, BNGH41000104, BNGH41000118, BNGH41000137, BNGH41000153, BNGH41000169, BNGH41000177, BNGH41000216, BNGH42000130, BNGH42000132, BNGH42000405, BNGH42000532, BNGH42000675, BNGH42000791, BNGH42000815, BNGH42000994, BNGH42001724, BNGH42002168, BNGH42002244, BNGH42002487, BNGH42002489, BNGH42003529, BNGH42003570, BNGH42003641, BNGH42004076, BNGH42004291, BNGH42004538, BNGH42005017, BNGH42005235, BNGH42005504, BNGH42006226, BNGH42006234, BNGH42007366, BNGH42007594, BNGH42007788, BNGH42008199, BNGH42008510, BNGH42008603, BNGH42008649, BNGH42008832, BNGH42008850, BNGH42008943, BNGH42009021, BRACE1000020, BRACE1000042, BRACE1000047, BRACE1000051, BRACE1000065, BRACE1000070,
BRACE1000084, BRACE1000087, BRACE1000092, BRACE1000093, BRACE1000101, BRACE1000114, BRACE1000124, BRACE1000132, BRACE1000166, BRACE1000169, BRACE1000186, BRACE1000187, BRACE1000239, BRACE1000258, BRACE1000451, BRACE1000475, BRACE1000572, BRACE2000061, BRACE2000078, BRACE2000100, BRACE2000109, BRACE2000141, BRACE2000148, BRACE2000232, BRACE2000245, BRACE2000256, BRACE2000266, BRACE2000267, BRACE2000280, BRACE2000300, BRACE2000307, BRACE2000331, BRACE2000332, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000640, BRACE2000650, BRACE2000677, BRACE2000698,
BRACE2000718, BRACE2000733, BRACE2000743, BRACE2000750, BRACE2000753, BRACE2000815, BRACE2000864, BRACE2000905, BRACE2000936, BRACE2000947, BRACE2000965, BRACE2000969, BRACE2000988, BRACE2001017, BRACE2001065, BRACE2001070, BRACE2001094, BRACE2001151, BRACE2001188, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001492, BRACE2001496, BRACE2001508, BRACE2001534, BRACE2001543, BRACE2001588, BRACE2001607, BRACE2001626, BRACE2001673, BRACE2001677, BRACE2001705, BRACE2001709, BRACE2001726,
BRACE2001733, BRACE2001737, BRACE2001742, BRACE2001777, BRACE2001779, BRACE2001834, BRACE2001855, BRACE2001859, BRACE2001865, BRACE2001872, BRACE2001881, BRACE2001898, BRACE2001923, BRACE2001942, BRACE2001944, BRACE2001954, BRACE2001971, BRACE2001972, BRACE2001973, BRACE2002026, BRACE2002034, BRACE2002081, BRACE2002139, BRACE2002142, BRACE2002151, BRACE2002157, BRACE2002176, BRACE2002206, BRACE2002227, BRACE2002260, BRACE2002297, BRACE2002304, BRACE2002394, BRACE2002409, BRACE2002431, BRACE2002444, BRACE2002458, BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623,
BRACE2002635, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002812, BRACE2002834, BRACE2002849, BRACE2002857, BRACE2002860, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003254, BRACE2003269, BRACE2003285, BRACE2003304, BRACE2003319, BRACE2003398, BRACE2003420, BRACE2003428, BRACE2003431, BRACE2003512, BRACE2003516, BRACE2003539, BRACE2003540, BRACE2003594, BRACE2003598, BRACE2003609, BRACE2003614, BRACE2003639, BRACE2003700, BRACE2003713,
BRACE2003766, BRACE2003785, BRACE2003788, BRACE2003800, BRACE2003802, BRACE2003825, BRACE2003845, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005099, BRACE2005118, BRACE2005124, BRACE2005138, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005298, BRACE2005328, BRACE2005329, BRACE2005337, BRACE2005348, BRACE2005363, BRACE2005395, BRACE2005408, BRACE2005448, BRACE2005450, BRACE2005453, BRACE2005457, BRACE2005460,
BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006075, BRACE2006084, BRACE2006089, BRACE2006102, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274, BRACE2006281, BRACE2006298, BRACE2006344,
BRACE2006354, BRACE2006363, BRACE2006378, BRACE2006380, BRACE2006393, BRACE2006413, BRACE2006417, BRACE2006453, BRACE2006459, BRACE2006488, BRACE2006534, BRACE2006537, BRACE2006540, BRACE2006564, BRACE2006587, BRACE2006598, BRACE2006636, BRACE2006685, BRACE2006703, BRACE2006743, BRACE2006753, BRACE2006783, BRACE2006821, BRACE2006833, BRACE2006871, BRACE2006909, BRACE2006911, BRACE2006924, BRACE2006935, BRACE2006937, BRACE2006944, BRACE2006951, BRACE2006982, BRACE2006986, BRACE2007013, BRACE2007068, BRACE2007087, BRACE2007138, BRACE2007174, BRACE2007197, BRACE2007201, BRACE2007203, BRACE2007232, BRACE2007254, BRACE2007262, BRACE2007274, BRACE2007281, BRACE2007295, BRACE2007358, BRACE2007396,
BRACE2007398, BRACE2007411, BRACE2007447, BRACE2007477, BRACE2007502, BRACE2007503,.BRACE2007522, BRACE2007527, BRACE2007538, BRACE2007563, BRACE2007577, BRACE2007621, BRACE2007640, BRACE2007641, BRACE2007646, BRACE2007663, BRACE2007671, BRACE2007682, BRACE2007685, BRACE2007727, BRACE2007749, BRACE2007760, BRACE2007761, BRACE2007764, BRACE2007767, BRACE2007768, BRACE2007769, BRACE2007784, BRACE2007786, BRACE2007798, BRACE2007799, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008097, BRACE2008102, BRACE2008114, BRACE2008144, BRACE2008172, BRACE2008216,
BRACE2008218, BRACE2008251, BRACE2008276, BRACE2008295, BRACE2008308, BRACE2008336, BRACE2008361, BRACE2008380, BRACE2008401, BRACE2008410, BRACE2008420, BRACE2008443, BRACE2008473, BRACE2008480, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009053, BRACE2009122, BRACE2009131, BRACE2009185, BRACE2009188, BRACE2009212,
BRACE2009221, BRACE2009235, BRACE2009238, BRACE2009255, BRACE2009270, BRACE2009272, BRACE2009274, BRACE2009275, BRACE2009293, BRACE2009311, BRACE2009318, BRACE2009323, BRACE2009366, BRACE2009421, BRACE2009428, BRACE2009434, BRACE2009437, BRACE2009483, BRACE2009533, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010684, BRACE2010753, BRACE2010813, BRACE2010828,
BRACE2010837, BRACE2010888, BRACE2010937, BRACE2010983, BRACE2011007, BRACE2011025, BRACE2011183, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268,
BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934,
BRACE2017985, BRACE2017992, BRACE2018337, BRACE2018443, BRACE2018448, BRACE2018568, BRACE2018700, BRACE2018727, BRACE2018759, BRACE2018762, BRACE2018782, BRACE2019004, BRACE2019044, BRACE2019147, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843, BRACE2019865, BRACE2019872, BRACE2019877, BRACE2020028, BRACE2020077, BRACE2020097, BRACE2020157, BRACE2020278,- BRACE2020358, BRACE2020467, BRACE2020584, BRACE2020652, BRACE2020709, BRACE2020742, BRACE2020756, BRACE2020863, BRACE2020982, BRACE2021148, BRACE2021245, BRACE2021254, BRACE2021264, BRACE2021344, BRACE2021433,
BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404, BRACE2023451, BRACE2023540, BRACE2023633, BRACE2023744, BRACE2023800, BRACE2023801, BRACE2024074, BRACE2024222, BRACE2024610, BRACE2024781, BRACE2024826, BRACE2025018, BRACE2025316, BRACE2025333, BRACE2025452, BRACE2025492, BRACE2025710, BRACE2025796, BRACE2025992, BRACE2026054, BRACE2026141,
BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879, BRACE2027896, BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044,
BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037310, BRACE2037431, BRACE2037692, BRACE2037847, BRACE2038114, BRACE2038271, BRACE2038329, BRACE2038492, BRACE2038551, BRACE2038618, BRACE2039009,
BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043248, BRACE2043349, BRACE2043774, BRACE2043942, BRACE2044057, BRACE2044263, BRACE2044286, BRACE2044304, BRACE2044473, BRACE2044510, BRACE2044640, BRACE2044816, BRACE2044946, BRACE2044949, BRACE2045300, BRACE2045428,
BRACE2045445, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413,
BRACE3003595, BRACE3003698, BRACE3003866, BRACE3003920, BRACE3004046, BRACE3004058, BRACE3004113, BRACE3004358, BRACE3004371, BRACE3004435, BRACE3004477, BRACE3004767, BRACE3004772, BRACE3004783, BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005760, BRACE3005870, BRACE3005903, BRACE3005989, BRACE3006113, BRACE3006185, BRACE3006226, BRACE3006462, BRACE3006463, BRACE3006872, BRACE3006917, BRACE3007084, BRACE3007085, BRACE3007258, BRACE3007322, BRACE3007472, BRACE3007480, BRACE3007559, BRACE3007625, BRACE3007642, BRACE3007649, BRACE3007767, BRACE3007869, BRACE3008036,
BRACE3008066, BRACE3008092, BRACE3008095, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009265, BRACE3009297, BRACE3009377, BRACE3009392, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780,
BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3020151, BRACE3020194, BRACE3020286, BRACE3020356, BRACE3020594, BRACE3020669, BRACE3020671, BRACE3020890, BRACE3021148, BRACE3021517,
BRACE3021805, BRACE3022051, BRACE3022303, BRACE3022312, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027478, BRACE3027480, BRACE3027931, BRACE3027976, BRACE3028214, BRACE3028241, BRACE3028360, BRACE3028998, BRACE3029005, BRACE3029021, BRACE3029205, BRACE3029447,
BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856,
BRACE3040863, BRACE3041059, BRACE3041162, BRACE3041827, BRACE3042046, BRACE3042210, BRACE3042326, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009482, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951,
BRALZ2015168, BRALZ2015811, BRALZ2016085, BRALZ2016498, BRALZ2017359, BRALZ2017531, BRALZ2017607, BRALZ2017620, BRALZ2017844, BRALZ2018342, BRALZ2018492, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000388, BRAMY2000411, BRAMY2000508, BRAMY2000562, BRAMY2000653, BRAMY2000814, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339,
BRAMY2002364, BRAMY2002369, BRAMY2002479, BRAMY2002493, BRAMY2002525, BRAMY2002584, BRAMY2002604, BRAMY2002621, BRAMY2002638, BRAMY2002739, BRAMY2002799, BRAMY2002853, BRAMY2002862, BRAMY2002956, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004521, BRAMY2004524, BRAMY2004537, BRAMY2004542, BRAMY2004756, BRAMY2004771, BRAMY2005064, BRAMY2005094, BRAMY2005151, BRAMY2005182, BRAMY2005244, BRAMY2005391,
BRAMY2005576, BRAMY2005648, BRAMY2005662, BRAMY2005708, BRAMY2005711, BRAMY2006092, BRAMY2006118, BRAMY2006149, BRAMY2006366, BRAMY2006375, BRAMY2006411, BRAMY2006459, BRAMY2006466, BRAMY2006659, BRAMY2006779, BRAMY2006809, BRAMY2006879, BRAMY2007043, BRAMY2007110, BRAMY2007185, BRAMY2007244, BRAMY2007249, BRAMY2007255, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008979, BRAMY2009023, BRAMY2009034, BRAMY2009105, BRAMY2009123, BRAMY2009192, BRAMY2009344, BRAMY2009349, BRAMY2009394, BRAMY2009489, BRAMY2009508,
BRAMY2009528, BRAMY2009557, BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011105, BRAMY2011178, BRAMY2011196, BRAMY2011216, BRAMY2011253, BRAMY2011446, BRAMY2011480, BRAMY2011639, BRAMY2011679, BRAMY2011811, BRAMY2011846, BRAMY2011849, BRAMY2011872, BRAMY2011961, BRAMY2012017, BRAMY2012091, BRAMY2012119, BRAMY2012162, BRAMY2012277, BRAMY2012340, BRAMY2012497, BRAMY2012517, BRAMY2012555, BRAMY2012568, BRAMY2012581, BRAMY2012691, BRAMY2012721,
BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013659, BRAMY2013722, BRAMY2013736, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015855, BRAMY2015890, BRAMY2015911, BRAMY2015925, BRAMY2016002, BRAMY2016009, BRAMY2016020, BRAMY2016070, BRAMY2016251, BRAMY2016327, BRAMY2016386, BRAMY2016706, BRAMY2016771,
BRAMY2016892, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018785, BRAMY2019055, BRAMY2019111, BRAMY2019277, BRAMY2019300, BRAMY2019509, BRAMY2019600, BRAMY2019710, BRAMY2019963, BRAMY2019985, BRAMY2019989, BRAMY2020050, BRAMY2020058, BRAMY2020106, BRAMY2020270, BRAMY2020355, BRAMY2020412, BRAMY2020445, BRAMY2020466, BRAMY2020520, BRAMY2020574, BRAMY2020634, BRAMY2021040, BRAMY2021063, BRAMY2021098, BRAMY2021139, BRAMY2021276, BRAMY2021310, BRAMY2021388, BRAMY2021498,
BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022168, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863, BRAMY2024004, BRAMY2024073, BRAMY2024312, BRAMY2024374, BRAMY2024390, BRAMY2024416, BRAMY2024449, BRAMY2024489,
BRAMY2024497, BRAMY2024514, BRAMY2024530, BRAMY2024535, BRAMY2024545, BRAMY2024576, BRAMY2024593, BRAMY2024711, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277,.BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027114, BRAMY2027140, BRAMY2027242, BRAMY2027396, BRAMY2027451, BRAMY2027461, BRAMY2027467, BRAMY2027587, BRAMY2027714, BRAMY2027717, BRAMY2027739,
BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028856, BRAMY2028914, BRAMY2029204, BRAMY2029602, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2031516, BRAMY2032014, BRAMY2032231, BRAMY2032242, BRAMY2032290, BRAMY2032311, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332,
BRAMY2033506, BRAMY2033594, BRAMY2033895, BRAMY2033914, BRAMY2034072, BRAMY2034185, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035923, BRAMY2036076, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629, BRAMY2037800, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2039287, BRAMY2039300, BRAMY2039314, BRAMY2039341, BRAMY2039630,
BRAMY2039695, BRAMY2039728, BRAMY2039872, BRAMY2040051, BRAMY2040159, BRAMY2040214, BRAMY2040478, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069, BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871,
BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002508, BRAMY3002620, BRAMY3002803, BRAMY3002805, BRAMY3002886, BRAMY3002991, BRAMY3003026, BRAMY3003109, BRAMY3003310, BRAMY3003566, BRAMY3003723, BRAMY3004126, BRAMY3004224, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761,
BRAMY3007078, BRAMY3007206, BRAMY3007311, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009556, BRAMY3009782, BRAMY3009811, BRAMY3009904, BRAMY3010008, BRAMY3010264, BRAMY3010321, BRAMY3010411, BRAMY3010492, BRAMY3010603, BRAMY3010654, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000045, BRAWH1000094,
BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000082, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000417, BRAWH2000443, BRAWH2000471, BRAWH2000476, BRAWH2000482, BRAWH2000488, BRAWH2000494, BRAWH2000500, BRAWH2000503, BRAWH2000522, BRAWH2000554, BRAWH2000588, BRAWH2000595, BRAWH2000633, BRAWH2000643, BRAWH2000651, BRAWH2000682, BRAWH2000752, BRAWH2000859, BRAWH2000866, BRAWH2000892,
BRAWH2000901, BRAWH2000926, BRAWH2000984, BRAWH2001019, BRAWH2001037, BRAWH2001052, BRAWH2001103, BRAWH2001129, BRAWH2001137, BRAWH2001141, BRAWH2001159, BRAWH2001186, BRAWH2001203, BRAWH2001238, BRAWH2001239, BRAWH2001255, BRAWH2001355, BRAWH2001378, BRAWH2001395, BRAWH2001412, BRAWH2001418, BRAWH2001424, BRAWH2001436, BRAWH2001438, BRAWH2001459, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2002047, BRAWH2002060, BRAWH2002062, BRAWH2002125, BRAWH2002191, BRAWH2002318, BRAWH2002333, BRAWH2002352, BRAWH2002383,
BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003135, BRAWH2003240, BRAWH2003307, BRAWH2003355, BRAWH2003366, BRAWH2003662, BRAWH2003689, BRAWH2003693, BRAWH2003832, BRAWH2003948, BRAWH2003964, BRAWH2004044, BRAWH2004068, BRAWH2004078, BRAWH2004217, BRAWH2004580, BRAWH2004731, BRAWH2004779, BRAWH2004842, BRAWH2004884, BRAWH2004923, BRAWH2005008, BRAWH2005074, BRAWH2005225, BRAWH2005315, BRAWH2005517, BRAWH2005524, BRAWH2005533, BRAWH2005578, BRAWH2005812, BRAWH2005974, BRAWH2005998, BRAWH2006083, BRAWH2006207,
BRAWH2006301, BRAWH2006526, BRAWH2006543, BRAWH2006622, BRAWH2006760, BRAWH2006960, BRAWH2006989, BRAWH2007069, BRAWH2007308, BRAWH2007406, BRAWH2007550, BRAWH2007570, BRAWH2007591, BRAWH2007658, BRAWH2007664, BRAWH2007800, BRAWH2007808, BRAWH2007825, BRAWH2007862, BRAWH2007890, BRAWH2008058, BRAWH2008128, BRAWH2008255, BRAWH2008292, BRAWH2008297, BRAWH2008380, BRAWH2008540, BRAWH2008577, BRAWH2008790, BRAWH2008903, BRAWH2008956, BRAWH2008993, BRAWH2009112, BRAWH2009227, BRAWH2009261, BRAWH2009263, BRAWH2009298, BRAWH2009304, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069,
BRAWH2010084, BRAWH2010136, BRAWH2010318, BRAWH2010329, BRAWH2010354, BRAWH2010536, BRAWH2010552, BRAWH2010584, BRAWH2010618, BRAWH2010619, BRAWH2010754, BRAWH2011066, BRAWH2011079, BRAWH2011096, BRAWH2011155, BRAWH2011166, BRAWH2011204, BRAWH2011286, BRAWH2011294, BRAWH2011343, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012161, BRAWH2012162, BRAWH2012164, BRAWH2012258, BRAWH2012277, BRAWH2012326, BRAWH2012538, BRAWH2012540, BRAWH2012619, BRAWH2012698, BRAWH2012749, BRAWH2012827, BRAWH2012866, BRAWH2012963, BRAWH2013047, BRAWH2013219, BRAWH2013294, BRAWH2013748, BRAWH2013866, BRAWH2013871,
BRAWH2013914, BRAWH2013941, BRAWH2013955, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016166, BRAWH2016209, BRAWH2016221, BRAWH2016223, BRAWH2016439, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542,
BRAWH2017635, BRAWH2017913, BRAWH2018206, BRAWH2018267, BRAWH2018282, BRAWH2018317, BRAWH2018506, BRAWH2018514, BRAWH2018527, BRAWH2018601, BRAWH2018745, BRAWH2018875, BRAWH2019053, BRAWH2019055, BRAWH2019198, BRAWH3000078, BRAWH3000088, BRAWH3000100, BRAWH3000166, BRAWH3000314, BRAWH3000329, BRAWH3000410, BRAWH3000446, BRAWH3000491, BRAWH3000604, BRAWH3000884, BRAWH3001053, BRAWH3001319, BRAWH3001326, BRAWH3001475, BRAWH3001636, BRAWH3001638, BRAWH3001712, BRAWH3001760, BRAWH3001783, BRAWH3001833, BRAWH3001879, BRAWH3001918, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003522, BRAWH3003598,
BRAWH3003727, BRAWH3003801, BRAWH3003975, BRAWH3004222, BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331,
BRAWH3011402, BRAWH3011577, BRAWH3011623, BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431,
BRAWH3022459, BRAWH3022542, BRAWH3022719, 8RAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3026349, BRAWH3026394, BRAWH3026529, BRAWH3027420, BRAWH3027440, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571,
BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639,
BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007426, BRCAN2007525, BRCAN2007700, BRCAN2008494, BRCAN2008528, BRCAN2008701,
BRCAN2008894, BRCAN2009156, BRCAN2009168, BRCAN2009203, BRCAN2009432, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490, BRCAN2018566,
BRCAN2018667, BRCAN2018748, BRCAN2018935, BRCAN2019002,. BRCAN2019387, BRCAN2019653, BRCAN2019773, BRCAN2019907, BRCAN2020331, BRCAN2020412, BRCAN2020467, BRCAN2020710, BRCAN2020880, BRCAN2021024, BRCAN2021028, BRCAN2021325, BRCAN2021452, BRCAN2021669, BRCAN2021718, BRCAN2022126, BRCAN2022191, BRCAN2022472, BRCAN2022474, BRCAN2023347, BRCAN2023724, BRCAN2024451, BRCAN2024563, BRCAN2024572, BRCAN2025009, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027513, BRCAN2027593, BRCAN2027970, BRCAN2028021, BRCAN2028036, BRCAN2028319, BRCAN2028338, BRCAN2028355, BRCAN2028378, BRCAN2028460,
BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000333, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005951, BRCOC2006164, BRCOC2006243, BRCOC2006270, BRCOC2006639, BRCOC2006942, BRCOC2007034, BRCOC2007593, BRCOC2007733, BRCOC2007769,
BRCOC2008064, BRCOC2008225, BRCOC2008642, BRCOC2008993, BRCOC2009052, BRCOC2009196, BRCOC2009380, BRCOC2009638, BRCOC2010115, BRCOC2010510, BRCOC2010730, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012551, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896, BRCOC2019903, BRCOC2019910, BRCOC2019934,
BRCOC2020122, BRCOC2020142, BRHIP1000058, BRHIP1000072, BRHIP1000108, BRHIP1000129, BRHIP1000258, BRHIP2000021, BRHIP2000079, BRHIP2000087, BRHIP2000210, BRHIP2000239, BRHIP2000245, BRHIP2000312, BRHIP2000359, BRHIP2000445, BRHIP2000506, BRHIP2000532, BRHIP2000621, BRHIP2000691, BRHIP2000819, BRHIP2000826, BRHIP2000920, BRHIP2001099, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003062, BRHIP2003242, BRHIP2003272, BRHIP2003748, BRHIP2003786, BRHIP2003828, BRHIP2004312, BRHIP2004355, BRHIP2004359, BRHIP2004814,
BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010487, BRHIP2010571, BRHIP2010593, BRHIP2010610, BRHIP2010723, BRHIP2010744, BRHIP2011080, BRHIP2011120, BRHIP2011199, BRHIP2011508, BRHIP2011576, BRHIP2011616,
BRHIP2011783, BRHIP2011838, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013972, BRHIP2014063, BRHIP2014285, BRHIP2014291, BRHIP2014373, BRHIP2014386, BRHIP2014387, BRHIP2014954, BRHIP2015224, BRHIP2015245, BRHIP2015356, BRHIP2015360, BRHIP2016005, BRHIP2016125, BRHIP2016788, BRHIP2016968, BRHIP2016990, BRHIP2017315, BRHIP2017385, BRHIP2017404, BRHIP2017542, BRHIP2017553, BRHIP2017642, BRHIP2017714, BRHIP2017780, BRHIP2017945, BRHIP2018014, BRHIP2018369, BRHIP2018612,
BRHIP2018650, BRHIP2018719, BRHIP2018805, BRHIP2018998, BRHIP2019007, BRHIP2019047, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020799, BRHIP2020842, BRHIP2020859, BRHIP2020930, BRHIP2021102, BRHIP2021289, BRHIP2021495, BRHIP2021615, BRHIP2021744, BRHIP2021929, BRHIP2022006, BRHIP2022054, BRHIP2022221, BRHIP2022228, BRHIP2022298, BRHIP2022467, BRHIP2022708, BRHIP2022986, BRHIP2023071, BRHIP2023229, BRHIP2023272, BRHIP2023292, BRHIP2023309, BRHIP2023369, BRHIP2023438, BRHIP2023695, BRHIP2023735, BRHIP2023773, BRHIP2023860,
BRHIP2024046, BRHIP2024165, BRHIP2024201, BRHIP2024347, BRHIP2024398, BRHIP2024549, BRHIP2024789, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027017, BRHIP2027054, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000017, BRHIP3000060, BRHIP3000070, BRHIP3000081, BRHIP3000131, BRHIP3000240, BRHIP3000377, BRHIP3000457, BRHIP3000475, BRHIP3000488,
BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001878, BRHIP3001964, BRHIP3002114, BRHIP3002124, BRHIP3002141, BRHIP3002363, BRHIP3002691, BRHIP3002920, BRHIP3002931, BRHIP3003063, BRHIP3003126, BRHIP3003340, BRHIP3003395, BRHIP3003484, BRHIP3003644, BRHIP3003688, BRHIP3003795, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968, BRHIP3005137, BRHIP3005231, BRHIP3005307, BRHIP3005574, BRHIP3005673, BRHIP3005768, BRHIP3005801, BRHIP3005944, BRHIP3006279, BRHIP3006390, BRHIP3006449, BRHIP3006683, BRHIP3006717,
BRHIP3006786, BRHIP3006950, BRHIP3007172, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008405, BRHIP3008565, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013698,
BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160,
BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000295, BRSSN2000312, BRSSN2000518, BRSSN2000561, BRSSN2000715, BRSSN2000832, BRSSN2001213, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086, BRSSN2003093, BRSSN2003820, BRSSN2003841, BRSSN2003937, BRSSN2004142, BRSSN2004303, BRSSN2004304, BRSSN2004496, BRSSN2004679, BRSSN2004686, BRSSN2004710, BRSSN2004719, BRSSN2005847, BRSSN2006133, BRSSN2006390,
BRSSN2006575, BRSSN2006611, BRSSN2006644, BRSSN2006892, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010019, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198, BRSSN2012254, BRSSN2012439, BRSSN2012622, BRSSN2012691, BRSSN2012838, BRSSN2013095, BRSSN2013544, BRSSN2013733, BRSSN2013753, BRSSN2013874, BRSSN2014112, BRSSN2014294, BRSSN2014424, BRSSN2014513,
BRSSN2014556, BRSSN2014573, BRSSN2014636, BRSSN2014685, BRSSN2015199, BRSSN2015369, BRSSN2015493, BRSSN2015554, BRSSN2015707, BRSSN2015773, BRSSN2015907, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000097, BRSTN2000058, BRSTN2000070, BRSTN2000220, BRSTN2000312, BRSTN2000536, BRSTN2000872, BRSTN2001067, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583,
BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010089, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451, BRSTN2013171, BRSTN2013354, BRSTN2013931, BRSTN2014490, BRSTN2014633, BRSTN2014751, BRSTN2014770, BRSTN2015015, BRSTN2015978, BRSTN2016066, BRSTN2016335, BRSTN2016892, BRSTN2016918, BRSTN2016992, BRSTN2017151, BRSTN2017237, BRSTN2017379, BRSTN2017761, BRSTN2017771,
BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639,
BRTHA2004642, BRTHA2004812, BRTHA2004821, BRTHA2004978, BRTHA2004992, BRTHA2005005, BRTHA2005013, BRTHA2005448, BRTHA2005579, BRTHA2005831, BRTHA2005864, BRTHA2005942, BRTHA2005956, BRTHA2006075, BRTHA2006146, BRTHA2006194, BRTHA2006232, BRTHA2006720, BRTHA2006735, BRTHA2006974, BRTHA2007060, BRTHA2007122, BRTHA2007422, BRTHA2007603, BRTHA2007665, BRTHA2007998, BRTHA2008316, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884,
BRTHA2010907, BRTHA2011062, BRTHA2011194, BRTHA2011321, BRTHA2011351, BRTHA2011500, BRTHA2011641, BRTHA2011691, BRTHA2012129, BRTHA2012183, BRTHA2012189, BRTHA2012351, BRTHA2012392, BRTHA2012466, BRTHA2012555, BRTHA2012562; BRTHA2012980, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663, BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543,
BRTHA2016552, BRTHA2016918, BRTHA2017178, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229,
BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000273, BRTHA3000296, BRTHA3000297, BRTHA3000325, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003023, BRTHA3003225, BRTHA3003339, BRTHA3003343,
BRTHA3003417, BRTHA3003449, BRTHA3003474, BRTHA3003704, BRTHA3004432, BRTHA3004475,. BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685, BRTHA3007769, BRTHA3007922, BRTHA3008143, BRTHA3008293, BRTHA3008310, BRTHA3008386, BRTHA3008520, BRTHA3008608, BRTHA3008694, BRTHA3008826, BRTHA3009037, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011229, BRTHA3011265, BRTHA3011510, BRTHA3011892, BRTHA3012265, BRTHA3013860, BRTHA3013882,
BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3021971, BRTHA3022641, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3026916, BRTHA3027171, BRTHA3027318, BRTHA3027638,
BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382, CHONS2001797, CHONS2001834, CHONS2002419, COLON1000030, COLON1000084, COLON1000135, COLON2000470, COLON2000557, COLON2000568, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282,
COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000094, CTONG1000097, CTONG1000113, CTONG1000137, CTONG1000165, CTONG1000175, CTONG1000180, CTONG1000181, CTONG1000220, CTONG1000277, CTONG1000283, CTONG1000288, CTONG1000302, CTONG1000341, CTONG1000467, CTONG1000476, CTONG1000488, CTONG1000508, CTONG1000540, CTONG2000031, CTONG2000034, CTONG2000042, CTONG2000218, CTONG2000279, CTONG2000303, CTONG2000330, CTONG2000395, CTONG2000411, CTONG2000465, CTONG2000480, CTONG2000561,
CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001413, CTONG2001428, CTONG2001513, CTONG2001524, CTONG2001533, CTONG2001598, CTONG2001681, CTONG2001724, CTONG2001749, CTONG2001820, CTONG2001877, CTONG2001911, CTONG2001932, CTONG2001955, CTONG2002066, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002903, CTONG2002965, CTONG2002999, CTONG2003028, CTONG2003094, CTONG2003100, CTONG2003115, CTONG2003245, CTONG2003348, CTONG2003350, CTONG2003372, CTONG2003375, CTONG2003427,
CTONG2003599, CTONG2003680, CTONG2003699, CTONG2003764, CTONG2003782, CTONG2003889, CTONG2003937, CTONG2004000, CTONG2004062, CTONG2004115, CTONG2004126, CTONG2004264, CTONG2004305, CTONG2004397, CTONG2004423, CTONG2004454, CTONG2004550, CTONG2004669, CTONG2004716, CTONG2004912, CTONG2004941, CTONG2004948, CTONG2005028, CTONG2005049, CTONG2005110, CTONG2005145, CTONG2005265, CTONG2005290, CTONG2005374, CTONG2005399, CTONG2005468, CTONG2005567, CTONG2005585, CTONG2005635, CTONG2005775, CTONG2005795, CTONG2005890, CTONG2005904, CTONG2006129, CTONG2006223, CTONG2006273, CTONG2006310, CTONG2006393, CTONG2006449, CTONG2006515, CTONG2006524, CTONG2006568, CTONG2006611, CTONG2006620, CTONG2006666,
CTONG2006691, CTONG2006709, CTONG2006836, CTONG2006942, CTONG2006964, CTONG2007009, CTONG2007091, CTONG2007168, CTONG2007175, CTONG2007259, CTONG2007272, CTONG2007293, CTONG2007297, CTONG2007400, CTONG2007417, CTONG2007441, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343, CTONG2008402, CTONG2008521, CTONG2008562, CTONG2008577, CTONG2008595, CTONG2008621, CTONG2008689, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395,
CTONG2009423, CTONG2009440, CTONG2009527, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009923, CTONG2009938, CTONG2009955, CTONG2009958, CTONG2010018, CTONG2010024, CTONG2010116, CTONG2010133, CTONG2010148, CTONG2010276, CTONG2010330, CTONG2010348, CTONG2010408, CTONG2010508, CTONG2010566, CTONG2010623, CTONG2010649, CTONG2010803, CTONG2011188, CTONG2011429, CTONG2011474, CTONG2011676, CTONG2011716, CTONG2011765, CTONG2011779, CTONG2011815, CTONG2011822, CTONG2011825, CTONG2011920, CTONG2011985, CTONG2012029, CTONG2012077,
CTONG2012123, CTONG2012158, CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012661, CTONG2012724, CTONG2012738, CTONG2012758, CTONG2012843, CTONG2012879, CTONG2012901, CTONG2012996, CTONG2013128, CTONG2013149, CTONG2013156, CTONG2013284, CTONG2013339, CTONG2013352, CTONG2013381, CTONG2013393, CTONG2013511, CTONG2013630, CTONG2013803, CTONG2013907, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014389, CTONG2014491, CTONG2014630, CTONG2014635, CTONG2014646, CTONG2014705,
CTONG2014946, CTONG2014954, CTONG2014959, CTONG2014966, CTONG2014995, CTONG2015204, CTONG2015316, CTONG2015345, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016056, CTONG2016217, CTONG2016355, CTONG2016499, CTONG2016505, CTONG2016559, CTONG2016658, CTONG2016775, CTONG2016797, CTONG2016824, CTONG2016846, CTONG2016904, CTONG2016953, CTONG2017021, CTONG2017292, CTONG2017409, CTONG2017444, CTONG2017458,- CTONG2017604, CTONG2017650, CTONG2017804, CTONG2017814, CTONG2017910, CTONG2017939, CTONG2017989, CTONG2018062, CTONG2018069, CTONG2018147, CTONG2018211, CTONG2018217,
CTONG2018279, CTONG2018334, CTONG2018343, CTONG2018379, CTONG2018413, CTONG2018483, CTONG2018629, CTONG2018653, CTONG2018655, CTONG2018735, CTONG2018900, CTONG2018976, CTONG2019029, CTONG2019063, CTONG2019144, CTONG2019232, CTONG2019248, CTONG2019590, CTONG2019652, CTONG2019704, CTONG2019822, CTONG2019833, CTONG2020031, CTONG2020108, CTONG2020127, CTONG2020171, CTONG2020374, CTONG2020378, CTONG2020411, CTONG2020445, CTONG2020806, CTONG2020831, CTONG2021024, CTONG2021132, CTONG2021168, CTONG2022153, CTONG2022601, CTONG2022835, CTONG2023021, CTONG2023512, CTONG2024031, CTONG2024206, CTONG2024614, CTONG2024749, CTONG2025496, CTONG2025513, CTONG2025516, CTONG2025610, CTONG2025900, CTONG2026513,
CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028208, CTONG2028486, CTONG2028518, CTONG2028687, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002552, CTONG3002588, CTONG3002674, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004317,
CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328, CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001788, D3OST2002182, D3OST2002223, D3OST2002262,
D3OST2002272, D3OST2002417, D3OST2002436, D3OST2002648, D3OST2003331, D3OST2003607, D3OST2003797, D3OST2004637, D3OST3000195, D3OST3000258, D3OST3000291, D3OST3000388, D3OST3000493, D6OST2000127, D6OST2000169, D6OST2000245, D6OST2000358, D6OST2000464, D6OST2000507, D9OST2000278, D9OST2000440, D9OST2000869, D9OST2001319, D9OST2001433, D9OST2002397, D9OST2002608, D9OST2002673, D9OST2003106, D9OST2003108, D9OST2003137, D9OST2003397, D9OST2003580, D9OST2003594, D9OST2003791, D9OST2003989, D9OST2004018, D9OST2004073, D9OST2004417, DFNES1000003, DFNES1000083, DFNES1000107, DFNES1000185, DFNES2000143, DFNES2000146, DFNES2000212, DFNES2000292, DFNES2000426, DFNES2000432, DFNES2000457, DFNES2000471,
DFNES2000913, DFNES2001091, DFNES2001096, DFNES2001108, DFNES2001177, DFNES2001404, DFNES2001564, DFNES2001565, DFNES2001647, DFNES2001800, DFNES2001829, DFNES2002220, DFNES2002509, DFNES2002550, DFNES2002588, DFNES2002817, DFNES2003081, DFNES2003255, DFNES2003742, DFNES2004354, DFNES2004383, DFNES2004608, DFNES2004684, DFNES2005266, DFNES2005527, DFNES2005540, DFNES2005690, DFNES2005766, DFNES2005779, DFNES2005875, DFNES2006346, DFNES2006944, DFNES2007299, DFNES2007332, DFNES2007641, DFNES2007757, DFNES2008160, DFNES2008280, DFNES2008881, DFNES2009482, DFNES2010502, DFNES2011192, DFNES2011221, DFNES2011239, DFNES2011245, ERLTF1000021, ERLTF2000324, ERLTF2000465, FCBBF1000023, FCBBF1000025,
FCBBF1000038, FCBBF1000053, FCBBF1000069, FCBBF1000115, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232, FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000367, FCBBF1000376, FCBBF1000377, FCBBF1000395, FCBBF1000397, FCBBF1000403, FCBBF1000412, FCBBF1000437, FCBBF1000449, FCBBF1000466, FCBBF1000476, FCBBF1000506, FCBBF1000509, FCBBF1000546, FCBBF1000550, FCBBF1000556, FCBBF1000581, FCBBF1000582, FCBBF1000598, FCBBF1000604, FCBBF1000618, FCBBF1000627,
FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566, FCBBF2000677, FCBBF2000678, FCBBF2000685, FCBBF2000733, FCBBF2000782, FCBBF2000808, FCBBF2000815, FCBBF2000825, FCBBF2000940, FCBBF2000951, FCBBF2001001, FCBBF2001088, FCBBF2001105, FCBBF2001116, FCBBF2001183, FCBBF2001188, FCBBF2001211, FCBBF2001238, FCBBF2001243, FCBBF2001291, FCBBF2001299, FCBBF2001309, FCBBF2001400, FCBBF2001427, FCBBF2001480, FCBBF2001529, FCBBF2001538, FCBBF2001594,
FCBBF2001672, FCBBF2001675, FCBBF2001718, FCBBF2001720, FCBBF2001817, FCBBF2001868, FCBBF2002044, FCBBF2002281, FCBBF2002370, FCBBF2002541, FCBBF2002626, FCBBF2002897, FCBBF2002898, FCBBF2002904, FCBBF2002928, FCBBF2002953, FCBBF2003083, FCBBF2003269, FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005733, FCBBF2005909, FCBBF2005966, FCBBF2006131, FCBBF2006380,
FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000115, FCBBF3000120, FCBBF3000168, FCBBF3000175, FCBBF3000227, FCBBF3000229, FCBBF3000233, FCBBF3000269, FCBBF3000361, FCBBF3000367, FCBBF3000462, FCBBF3000473, FCBBF3000501, FCBBF3000518, FCBBF3000550, FCBBF3000847, FCBBF3001018, FCBBF3001020, FCBBF3001235, FCBBF3001302, FCBBF3001594, FCBBF3001657, FCBBF3001855, FCBBF3001890, FCBBF3001912, FCBBF3001914, FCBBF3001918, FCBBF3001924, FCBBF3002163, FCBBF3002188, FCBBF3002190, FCBBF3002268, FCBBF3002475, FCBBF3002556, FCBBF3002592, FCBBF3002658, FCBBF3002782,
FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004323, FCBBF3004390, FCBBF3004409, FCBBF3004502, FCBBF3004715, FCBBF3004842, FCBBF3004847, FCBBF3004955, FCBBF3005160, FCBBF3005218, FCBBF3005320, FCBBF3005444, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007244, FCBBF3007248, FCBBF3007453, FCBBF3007462, FCBBF3007540, FCBBF3007604, FCBBF3007631, FCBBF3007829, FCBBF3007855, FCBBF3007859, FCBBF3007860, FCBBF3008073, FCBBF3008100, FCBBF3008153,
FCBBF3008251, FCBBF3008311, FCBBF3008362, FCBBF3008382, FCBBF3008475, FCBBF3008644, FCBBF3008689, FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010695, FCBBF3010729, FCBBF3010733, FCBBF3011003, FCBBF3011418, FCBBF3011523, FCBBF3011570, FCBBF3011592, FCBBF3011867, FCBBF3011889, FCBBF3012170, FCBBF3012182,
FCBBF3012332, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013589, FCBBF3013623, FCBBF3013800, FCBBF3013846, FCBBF3014229, FCBBF3014260, FCBBF3014355, FCBBF3014567, FCBBF3015100, FCBBF3015119, FCBBF3015131, FCBBF3015317, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017974, FCBBF3018067, FCBBF3018173, FCBBF3018453,
FCBBF3018591, FCBBF3018753, FCBBF3018826, FCBBF3018949, FCBBF3019085, FCBBF3019320, FCBBF3019437, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061; FCBBF3023078, FCBBF3023368, FCBBF3023372, FCBBF3023443, FCBBF3023599, FCBBF3023704, FCBBF3023887, FCBBF3023895, FCBBF3024096, FCBBF3024225, FCBBF3024266,
FCBBF3024364, FCBBF3024623, FCBBF3024793, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025905, FCBBF3026021, FCBBF3026048, FCBBF3026251, FCBBF3026308, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028244, FCBBF3028472, FCBBF3028528, FCBBF3028582, FCBBF3028671, FCBBF3028794, FCBBF4000017, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415,
FCBBF4000529, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000030, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000129, FEBRA2000220, FEBRA2000228, FEBRA2000253, FEBRA2000286, FEBRA2000297, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000454, FEBRA2000462, FEBRA2000468, FEBRA2000491, FEBRA2000504, FEBRA2000536, FEBRA2000538,
FEBRA2000575, FEBRA2000581, FEBRA2000656, FEBRA2000659, FEBRA2000680; FEBRA2000690, FEBRA2000727, FEBRA2000734, FEBRA2000757, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2001004, FEBRA2001012, FEBRA2001020, FEBRA2001093, FEBRA2001124, FEBRA2001146, FEBRA2001161, FEBRA2001175, FEBRA2001197, FEBRA2001217, FEBRA2001227, FEBRA2001245, FEBRA2001267, FEBRA2001294, FEBRA2001351, FEBRA2001383, FEBRA2001438, FEBRA2001440, FEBRA2001487, FEBRA2001561, FEBRA2001581,
FEBRA2001584, FEBRA2001590, FEBRA2001591, FEBRA2001610, FEBRA2001620, FEBRA2001659, FEBRA2001669, FEBRA2001698, FEBRA2001705, FEBRA2001706, FEBRA2001715, FEBRA2001745, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662, FEBRA2002682, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931,
FEBRA2002962, FEBRA2002986, FEBRA2003054, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003436, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003750, FEBRA2003822, FEBRA2003897, FEBRA2003907, FEBRA2003930, FEBRA2004026, FEBRA2004029, FEBRA2004042, FEBRA2004053, FEBRA2004056, FEBRA2004091, FEBRA2004110, FEBRA2004202, FEBRA2004219, FEBRA2004237, FEBRA2004268, FEBRA2004325, FEBRA2004329, FEBRA2004331, FEBRA2004443, FEBRA2004485, FEBRA2004490, FEBRA2004538, FEBRA2004592,
FEBRA2004620, FEBRA2004628, FEBRA2004651, FEBRA2004767, FEBRA2004813, FEBRA2004818, FEBRA2004840, FEBRA2004852, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005427, FEBRA2005458, FEBRA2005476, FEBRA2005572, FEBRA2005701, FEBRA2005726, FEBRA2005778, FEBRA2005787, FEBRA2005788, FEBRA2005824, FEBRA2005998, FEBRA2006055, FEBRA2006061, FEBRA2006150, FEBRA2006165, FEBRA2006255, FEBRA2006315, FEBRA2006346, FEBRA2006354, FEBRA2006357, FEBRA2006370, FEBRA2006379, FEBRA2006396, FEBRA2006409, FEBRA2006418, FEBRA2006427, FEBRA2006476, FEBRA2006485, FEBRA2006491,
FEBRA2006519, FEBRA2006521, FEBRA2006553, FEBRA2006627, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007247, FEBRA2007280, FEBRA2007414, FEBRA2007436, FEBRA2007449, FEBRA2007458, FEBRA2007534, FEBRA2007544, FEBRA2007551, FEBRA2007566, FEBRA2007589, FEBRA2007613, FEBRA2007714, FEBRA2007793, FEBRA2007818, FEBRA2007823, FEBRA2007880, FEBRA2007931, FEBRA2007957, FEBRA2008009, FEBRA2008086, FEBRA2008134, FEBRA2008135, FEBRA2008159, FEBRA2008165, FEBRA2008172, FEBRA2008201, FEBRA2008210, FEBRA2008255, FEBRA2008265, FEBRA2008266, FEBRA2008282, FEBRA2008287, FEBRA2008302,
FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009780, FEBRA2009846, FEBRA2010141, FEBRA2010299, FEBRA2010484, FEBRA2010719,
FEBRA2010746, FEBRA2010768, FEBRA2010802, FEBRA2011063, FEBRA2011090, FEBRA2011356, FEBRA2011392, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013699, FEBRA2013905, FEBRA2013951, FEBRA2014010, FEBRA2014122, FEBRA2014198, FEBRA2014213, FEBRA2014443, FEBRA2014578, FEBRA2014939, FEBRA2015076, FEBRA2015175, FEBRA2015292, FEBRA2015588, FEBRA2016112, FEBRA2016398, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333,
FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2019172, FEBRA2019242, FEBRA2019298, FEBRA2019389, FEBRA2019457, FEBRA2019519, FEBRA2019582, FEBRA2019637, FEBRA2019690, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021431, FEBRA2021497, FEBRA2021550, FEBRA2021571, FEBRA2021636, FEBRA2021864, FEBRA2021892, FEBRA2021908, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956,
FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023927, FEBRA2023989, FEBRA2023990, FEBRA2024019, FEBRA2024136, FEBRA2024343, FEBRA2024570, FEBRA2024693, FEBRA2024727, FEBRA2024744, FEBRA2024797, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477; FEBRA2028503, FEHRT2000325, FEHRT2000364, FEKID1000018, FELIV1000101, FELIV1000137, FELIV1000164, FELNG2000241, HCASM1000021,
HCASM1000050, HCASM1000061, HCASM1000130, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000142, HCHON1000176, HCHON1000191, HCHON2000028, HCHON2000038, HCHON2000062, HCHON2000100,
HCHON2000156, HCHON2000199, HCHON2000212, HCHON2000271, HCHON2000295, HCHON2000315, HCHON2000322, HCHON2000344, HCHON2000364, HCHON2000395, HCHON2000417, HCHON2000418, HCHON2000473, HCHON2000503, HCHON2000660, HCHON2000673, HCHON2000676, HCHON2000699, HCHON2000705, HCHON2000743, HCHON2000751, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001084, HCHON2001108, HCHON2001110, HCHON2001116, HCHON2001161, HCHON2001214; HCHON2001233, HCHON2001269, HCHON2001434, HCHON2001450, HCHON2001453, HCHON2001497, HCHON2001523, HCHON2001546, HCHON2001548, HCHON2001577, HCHON2001604, HCHON2001607,
HCHON2001646, HCHON2001712, HCHON2001768, HCHON2001801, HCHON2001853, HCHON2002123, HCHON2002194, HCHON2002241, HCHON2002247, HCHON2002354, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006722, HCHON2006786, HCHON2006841, HCHON2006871, HCHON2007046, HCHON2007482, HCHON2007650, HCHON2007674, HCHON2007774, HCHON2007881, HCHON2008112, HCHON2008239, HCHON2008444, HCHON2008582, HCHON2008672,
HCHON2008856, HCHON2008871, HCHON2008989, HCHON2009191, HCHON2009384, HCHON2009749, HCHON2009766, HCHON2009795, HCHON2010074, HCHON2010543, HEART1000074, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411, HEART2000418, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003852,
HEART2004570, HEART2004940, HEART2004980, HEART2005244, HEART2005581, HEART2005610, HEART2006131, HEART2006195, HEART2006310, HEART2006334, HEART2006487, HEART2006557, HEART2006787, HEART2006789, HEART2006909, HEART2007031, HEART2007231, HEART2007443, HEART2007767, HEART2008108, HEART2008111, HEART2008257, HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC2000055, HHDPC2000149, HHDPC2000258, HHDPC2000315, HHDPC2000456, HHDPC2000462, HHDPC2000572, HHDPC2000656, HHDPC2000692, HHDPC2001337, HHDPC2001432, HHDPC2001896, HHDPC2002963, HHDPC2003049, HHDPC2003113, HHDPC2003381,
HHDPC2004757, HHDPC2005185, HHDPC2005513, HHDPC2005742, HHDPC2006460, HHDPC2006862, HHDPC2006927, HHDPC2007302, HHDPC2007775, HHDPC2008123, HHDPC2008185, HHDPC2008279, HHDPC2008297, HHDPC2008414, HHDPC2008474, HHDPC2008816, HHDPC2008843, HHDPC2009114, HHDPC2009208, HHDPC2009272, HLUNG1000017, HLUNG1000024, HLUNG1000030, HLUNG1000034, HLUNG1000053, HLUNG1000064, HLUNG1000076, HLUNG1000084, HLUNG1000091, HLUNG1000104, HLUNG1000105, HLUNG1000151, HLUNG1000169, HLUNG2000014, HLUNG2000027, HLUNG2000063, HLUNG2000068, HLUNG2000116, HLUNG2000125, HLUNG2000130, HLUNG2000142, HLUNG2000174, HLUNG2000176, HLUNG2000217, HLUNG2000232, HLUNG2000255, HLUNG2000281, HLUNG2000315, HLUNG2000340, HLUNG2000362,
HLUNG2000363, HLUNG2000412, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001507, HLUNG2001633, HLUNG2001677, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002811, HLUNG2002916, HLUNG2002942, HLUNG2002949, HLUNG2002958, HLUNG2002982, HLUNG2003049, HLUNG2003061,
HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005203, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006370, HLUNG2006397, HLUNG2006570, HLUNG2006599, HLUNG2006614,
HLUNG2006671, HLUNG2006781, HLUNG2006812, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008153, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008833, HLUNG2008875, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049, HLUNG2012287, HLUNG2012315, HLUNG2012546, HLUNG2012600, HLUNG2012727, HLUNG2013097,
HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015184, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000131, HSYRA2000044, HSYRA2000135, HSYRA2000137, HSYRA2000221, HSYRA2000237, HSYRA2000248, HSYRA2000253, HSYRA2000332, HSYRA2000371, HSYRA2000424, HSYRA2000510, HSYRA2000605, HSYRA2000629, HSYRA2000706, HSYRA2000760, HSYRA2000792, HSYRA2000828, HSYRA2000832, HSYRA2000925, HSYRA2000927, HSYRA2001003, HSYRA2001105,
HSYRA2001142, HSYRA2001153, HSYRA2001232, HSYRA2001255, HSYRA2001332, HSYRA2001353, HSYRA2001484, HSYRA2001552, HSYRA2001574, HSYRA2001580, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000161, IMR321000165, IMR321000193, IMR321000207, IMR321000230, IMR322000072, IMR322000107, IMR322000121, IMR322000127, IMR322000152, IMR322000164, IMR322000178, IMR322000302, IMR322000316, IMR322000374, IMR322000379, IMR322000383, IMR322000418,
IMR322000518, IMR322000604, IMR322000609, IMR322000656, IMR322000672, IMR322000775, IMR322000791, IMR322000811, IMR322000819, IMR322000838, IMR322000859, IMR322000863, IMR322000917, IMR322000918, IMR322000919, IMR322000935, IMR322000953, IMR322000973, IMR322000984, IMR322001018, IMR322001049, IMR322001167, IMR322001185, IMR322001218, IMR322001245, IMR322001317, IMR322001318, IMR322001322, IMR322001438, IMR322001452, IMR322001534, IMR322001541, IMR322001665, IMR322001670, IMR322001724, IMR322001879, IMR322002035, IMR322002110, IMR322002470, IMR322003675, IMR322004768, IMR322005293, IMR322005833, IMR322006012, IMR322006222, IMR322006495, IMR322006520, IMR322006886, IMR322006947, IMR322007078,
IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000036, KIDNE1000064, KIDNE1000104, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000349, KIDNE2000375, KIDNE2000383,
KIDNE2000394, KIDNE2000422, KIDNE2000493, KIDNE2000510, KIDNE2000513, KIDNE2000519, KIDNE2000555, KIDNE2000624, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2001117, KIDNE2001140, KIDNE2001269, KIDNE2001361, KIDNE2001373, KIDNE2001636, KIDNE2001713, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882,
KIDNE2002980, KIDNE2002991, KIDNE2003185, KIDNE2003188, KIDNE2003233, KIDNE2003335, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004305, KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004879, KIDNE2004981; KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400,
KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376, KIDNE2006378, KIDNE2006465, KIDNE2006580, KIDNE2006652, KIDNE2006687, KIDNE2006733, KIDNE2006811, KIDNE2006820, KIDNE2006880, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007944, KIDNE2008069, KIDNE2008072, KIDNE2008116, KIDNE2008117, KIDNE2008218, KIDNE2008315, KIDNE2008362, KIDNE2008378,
KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191, KIDNE2009332, KIDNE2009426, KIDNE2009467, KIDNE2009490, KIDNE2009553, KIDNE2009605, KIDNE2009628, KIDNE2009647, KIDNE2009676, KIDNE2009723, KIDNE2010007, KIDNE2010049, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010762, KIDNE2010863, KIDNE2010973, KIDNE2010989, KIDNE2011200,
KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012784, KIDNE2012945, KIDNE2013045, KIDNE2013095, KIDNE2013158, KIDNE2013218, KIDNE2013263, KIDNE2013388, KIDNE2013413, KIDNE2013489, KIDNE2013639, KIDNE2013731, KIDNE2013734, KIDNE2013775, KIDNE2013801, KIDNE2014087, KIDNE2014112, KIDNE2014119, KIDNE2014170, KIDNE2014184, KIDNE2014268, KIDNE2014298, KIDNE2014320, KIDNE2014325, KIDNE2014415, KIDNE2014489, KIDNE2014496, KIDNE2014661, KIDNE2014717,
KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015987, KIDNE2016000, KIDNE2016036, KIDNE2016096, KIDNE2016101, KIDNE2016142, KIDNE2016188, KIDNE2016327, KIDNE2016371, KIDNE2016388, KIDNE2016464, KIDNE2016539, KIDNE2016918, KIDNE2017007, KIDNE2017040, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018254, KIDNE2018268, KIDNE2018269, KIDNE2018287, KIDNE2018294, KIDNE2018462, KIDNE2018493,
KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER2000003, LIVER2000037, LIVER2000158, LIVER2000216, LIVER2000237, LIVER2000247, LIVER2000360, LIVER2000515, LIVER2000592, LIVER2000626, LIVER2000682, LIVER2000769, LIVER2000775, LIVER2001051, LIVER2001076, LIVER2001099, LIVER2001164,
LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003234, LIVER2003511, LIVER2003524, LIVER2003568, LIVER2003800, LIVER2003854, LIVER2004074, LIVER2004392, LIVER2004514, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008465, LIVER2008473, LIVER2008580, LIVER2008706,
LIVER2008751, LIVER2008945, LIVER2008998, LIVER2009110, LIVER2009118, LIVER2009554, LYMPB1000158, LYMPB2000083, MAMGL1000017, MAMGL1000083, MAMGL1000096, MAMGL1000132; MAMGL1000151, MAMGL1000178, MAMGL1000182, MAMGL1000184, MESAN1000032, MESAN1000035, MESAN1000079, MESAN1000121, MESAN1000126, MESAN1000147, MESAN1000180, MESAN2000023, MESAN2000026, MESAN2000067, MESAN2000167, MESAN2000264, MESAN2000337, MESAN2000457, MESAN2000462, MESAN2000572, MESAN2000608, MESAN2000620, MESAN2000711, MESAN2000815, MESAN2000894, MESAN2001154, MESAN2001627, MESAN2001770, MESAN2001979, MESAN2001980, MESAN2002113, MESAN2002122, MESAN2002147, MESAN2002186, MESAN2002424, MESAN2002687, MESAN2002709, MESAN2002978,
MESAN2003035, MESAN2003037, MESAN2003039, MESAN2003058, MESAN2003101, MESAN2003190, MESAN2003322, MESAN2003490, MESAN2003529, MESAN2003646, MESAN2003662, MESAN2003709, MESAN2003851, MESAN2004138, MESAN2004288, MESAN2004575, MESAN2005284, MESAN2005303, MESAN2005371, MESAN2005724, MESAN2005808, MESAN2005811, MESAN2005957, MESAN2006022, MESAN2006043, MESAN2006328, MESAN2006401, MESAN2006580, MESAN2006599, MESAN2006743, MESAN2006953, MESAN2006971, MESAN2007032, MESAN2007440, MESAN2007597, MESAN2008222, MESAN2008334, MESAN2008415, MESAN2008460, MESAN2008536, MESAN2008729, MESAN2008821, MESAN2008926, MESAN2008936, MESAN2009019, MESAN2009081, MESAN2009105, MESAN2009311, MESAN2009418, MESAN2009503,
MESAN2009522, MESAN2010031, MESAN2010114, MESAN2010312, MESAN2010321, MESAN2010664, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014295, MESAN2014298, MESAN2014624, MESAN2015277, MESAN2015365, MESAN2015391, MESAN2015401, MESAN2015501, MESAN2015708, MESAN2016304, MESAN2016409, MESAN2016552, MESAN2016965, MESAN2017133, MESAN2017152, MESAN2017373, MESAN2017417, MESAN2018209, MESAN2018576, MESAN2018670, MESAN2018699, MESTC1000042, MESTC1000147,
MESTC2000150, MESTC2000153, MESTC2000170, NIESE2000698, NB9N41000011, NB9N41000047, NB9N41000135, NB9N41000142, NB9N41000146, NB9N42000122, NB9N42000196, NB9N42000281, NB9N42000314, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000606, NHNPC2000877, NHNPC2001223, NHNPC2001312,
NHNPC2001816, NHNPC2001931, NHNPC2002565, NHNPC2002749, NOVAR1000015, NOVAR2000038, NOVAR2000136, NOVAR2000352, NOVAR2000412, NOVAR2000710, NOVAR2000962, NOVAR2001108, NOVAR2001620, NOVAR2001783, NOVAR2001876, NOVAR2002039, NT2NE1000004, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000222, NT2NE2000284, NT2NE2000299, NT2NE2000353, NT2NE2000356, NT2NE2000369, NT2NE2000374, NT2NE2000386, NT2NE2000390, NT2NE2000392, NT2NE2000455, NT2NE2000470, NT2NE2000488, NT2NE2000517, NT2NE2000536,
NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864, NT2NE2000909, NT2NE2000913, NT2NE2000951, NT2NE2000980, NT2NE2001000, NT2NE2001021, NT2NE2001040, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001337, NT2NE2001364, NT2NE2001372, NT2NE2001397, NT2NE2001403, NT2NE2001428, NT2NE2001432, NT2NE2001442, NT2NE2001524, NT2NE2001530, NT2NE2001617, NT2NE2001623, NT2NE2001626, NT2NE2001634, NT2NE2001655, NT2NE2001660, NT2NE2001666, NT2NE2001677, NT2NE2001698, NT2NE2001723, NT2NE2001725,
NT2NE2001874, NT2NE2001889, NT2NE2001923, NT2NE2002100, NT2NE2002162, NT2NE2002186, NT2NE2002285, NT2NE2002311, NT2NE2002620, NT2NE2002651, NT2NE2002732, NT2NE2002768, NT2NE2002791, NT2NE2002856, NT2NE2003150, NT2NE2003185, NT2NE2003252, NT2NE2003280, NT2NE2003308, NT2NE2003309, NT2NE2003315, NT2NE2003393, NT2NE2003408, NT2NE2003457, NT2NE2003485, NT2NE2003540, NT2NE2003571, NT2NE2003705, NT2NE2003752, NT2NE2004378, NT2NE2004490, NT2NE2004519, NT2NE2004606, NT2NE2004716, NT2NE2004740, NT2NE2004787, NT2NE2004916, NT2NE2005035, NT2NE2005223, NT2NE2005276, NT2NE2005317, NT2NE2005323, NT2NE2005358, NT2NE2005371, NT2NE2005395, NT2NE2005441, NT2NE2005517, NT2NE2005537, NT2NE2005555, NT2NE2005588,
NT2NE2005676, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006458, NT2NE2006531, NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008803, NT2NE2008867, NT2NE2008961, NT2NE2008997, NT2NE2009138, NT2NE2009295,
NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2011033, NT2NE2011036, NT2NE2011119, NT2NE2011154, NT2NE2011221, NT2NE2011411, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012505, NT2NE2012533, NT2NE2012603, NT2NE2012747, NT2NE2012847, NT2NE2012928, NT2NE2013019, NT2NE2013081, NT2NE2013189, NT2NE2013217, NT2NE2013547, NT2NE2014013, NT2NE2014028, NT2NE2014113, NT2NE2014176, NT2NE2014221,
NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2015974, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2018165, NT2NE2018176, NT2NE2018273, NT2NE2018376, NT2NE2018472, NT2NE2018594, NT2NE2018739, NT2NE2018916, NT2NE2019056, NT2NE2019076,
NT2NE2019099, NT2RI1000016, NT2RI1000035, NT2RI1000127, NT2RI1000164, NT2RI1000172, NT2RI2000004, NT2RI2000007, NT2RI2000116, NT2RI2000117, NT2RI2000128, NT2RI2000167, NT2RI2000173, NT2RI2000187, NT2RI2000270, NT2RI2000276, NT2RI2000282, NT2RI2000313, NT2RI2000341, NT2RI2000344, NT2RI2000348, NT2RI2000480, NT2RI2000575, NT2RI2000588, NT2RI2000597, NT2RI2000669, NT2RI2000671, NT2RI2000688, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001178, NT2RI2001235, NT2RI2001244, NT2RI2001307, NT2RI2001385, NT2RI2001409, NT2RI2001410,
NT2RI2001425, NT2RI2001449, NT2RI2001450, NT2RI2001474, NT2RI2001519, NT2RI2001595, NT2RI2001624, NT2RI2001726, NT2RI2001769, NT2RI2001782, NT2RI2001836, NT2RI2001846, NT2RI2001866, NT2RI2001952, NT2RI2002022, NT2RI2002041, NT2RI2002120, NT2RI2002153, NT2RI2002179, NT2RI2002252, NT2RI2002260, NT2RI2002270, NT2RI2002334, NT2RI2002359, NT2RI2002391, NT2RI2002447, NT2RI2002481, NT2RI2002507, NT2RI2002517, NT2RI2002530, NT2RI2002540, NT2RI2002549, NT2RI2002564, NT2RI2002592, NT2RI2002602, NT2RI2002699, NT2RI2002729, NT2RI2002819, NT2RI2002822, NT2RI2002865, NT2RI2002923, NT2RI2002926, NT2RI2002958, NT2RI2002970, NT2RI2003011, NT2RI2003067, NT2RI2003184, NT2RI2003222; NT2RI2003301, NT2RI2003360,
NT2RI2003361, NT2RI2003383, NT2RI2003392, NT2RI2003407, NT2RI2003419, NT2RI2003481, NT2RI2003487, NT2RI2003655, NT2RI2003667, NT2RI2003751, NT2RI2003884, NT2RI2003973, NT2RI2004059, NT2RI2004078, NT2RI2004079, NT2RI2004093, NT2RI2004136, NT2RI2004157, NT2RI2004190, NT2RI2004230, NT2RI2004284, NT2RI2004290, NT2RI2004304, NT2RI2004312, NT2RI2004381, NT2RI2004442, NT2RI2004468, NT2RI2004606, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005069, NT2RI2005087, NT2RI2005115, NT2RI2005130, NT2RI2005150, NT2RI2005239, NT2RI2005246, NT2RI2005315, NT2RI2005353, NT2RI2005358, NT2RI2005368, NT2RI2005382,
NT2RI2005473, NT2RI2005520, NT2RI2005564, NT2RI2005579, NT2RI2005621, NT2RI2005628, NT2RI2005670, NT2RI2005710, NT2RI2005772, NT2RI2005814, NT2RI2005816, NT2RI2005851, NT2RI2005966, NT2RI2006071, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006565, NT2RI2006640, NT2RI2006659, NT2RI2006662, NT2RI2006667, NT2RI2006679, NT2RI2006682, NT2RI2006686, NT2RI2006703, NT2RI2006716, NT2RI2006762, NT2RI2006788, NT2RI2006838, NT2RI2006853, NT2RI2006973, NT2RI2007046, NT2RI2007048, NT2RI2007084, NT2RI2007096, NT2RI2007116, NT2RI2007214, NT2RI2007277, NT2RI2007303, NT2RI2007334, NT2RI2007377,
NT2RI2007386, NT2RI2007406, NT2RI2007439, NT2RI2007507, NT2RI2007593, NT2RI2007629, NT2RI2007751, NT2RI2007754, NT2RI2007827, NT2RI2007877, NT2RI2007884, NT2RI2007891, NT2RI2007956, NT2RI2007965, NT2RI2007987, NT2RI2008050, NT2RI2008141, NT2RI2008204, NT2RI2008221, NT2RI2008233, NT2RI2008455, NT2RI2008481, NT2RI2008502, NT2RI2008566, NT2RI2008598, NT2RI2008622, NT2RI2008714, NT2RI2008791, NT2RI2008801, NT2RI2008812, NT2RI2008841, NT2RI2008952, NT2RI2009005, NT2RI2009101, NT2RI2009144, NT2RI2009151, NT2RI2009194, NT2RI2009215, NT2RI2009239, NT2RI2009259, NT2RI2009269, NT2RI2009289, NT2RI2009301, NT2RI2009402, NT2RI2009406, NT2RI2009517, NT2RI2009585, NT2RI2009595, NT2RI2009855, NT2RI2010283,
NT2RI2010508, NT2RI2010795, NT2RI2011422, NT2RI2011450, NT2RI2011683, NT2RI2011803, NT2RI2012659, NT2RI2012990, NT2RI2013345, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018883, NT2RI2018950, NT2RI2019751, NT2RI2019826, NT2RI2020390, NT2RI2020703, NT2RI2021463, NT2RI2021625, NT2RI2022468, NT2RI2022584, NT2RI2023303, NT2RI2023671, NT2RI2024008, NT2RI2024460, NT2RI2024496, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027323, NT2RI2027396, NT2RI2027484, NT2RI2027975,
NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001445, NT2RI3001458, NT2RI3001573, NT2RI3001967, NT2RI3002303, NT2RI3002557, NT2RI3002842, NT2RI3003027, NT2RI3003031, NT2RI3003095, NT2RI3003162, NT2RI3003362, NT2RI3003382, NT2RI3003409, NT2RI3003738, NT2RI3003925, NT2RI3004133, NT2RI3004381, NT2RI3004510, NT2RI3005202, NT2RI3005403, NT2RI3005416, NT2RI3005861, NT2RI3005923, NT2RI3005928, NT2RI3006132, NT2RI3006171, NT2RI3006376, NT2RI3006666, NT2RI3006673, NT2RI3006796, NT2RI3007065, NT2RI3007095, NT2RI3007158; NT2RI3007167, NT2RI3007213, NT2RI3007291, NT2RI3007443,
NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009480, NT2RI3009524, NT2RP6000005, NT2RP6000008, NT2RP6000032, NT2RP6000033, NT2RP6000059, NT2RP6000060, NT2RP6000077, NT2RP6000086, NT2RP6000119, NT2RP6000127, NT2RP6000140, NT2RP6000150, NT2RP7000041, NT2RP7000086, NT2RP7000109, NT2RP7000112, NT2RP7000311, NT2RP7000359, NT2RP7000364, NT2RP7000380, NT2RP7000391, NT2RP7000425, NT2RP7000579, NT2RP7000812, NT2RP7000926, NT2RP7001074, NT2RP7001306, NT2RP7001319, NT2RP7001335, NT2RP7001364, NT2RP7001475, NT2RP7001532, NT2RP7001591, NT2RP7001602, NT2RP7001856, NT2RP7002064,
NT2RP7002151, NT2RP7002163, NT2RP7002243, NT2RP7002282, NT2RP7002376, NT2RP7002478, NT2RP7002554, NT2RP7002594, NT2RP7002603, NT2RP7002650, NT2RP7002829, NT2RP7002841, NT2RP7002875, NT2RP7002978, NT2RP7003025, NT2RP7003050, NT2RP7003055, NT2RP7003084, NT2RP7003091, NT2RP7003107, NT2RP7003134, NT2RP7003148, NT2RP7003439, NT2RP7003511, NT2RP7003632, NT2RP7004027, NT2RP7004080, NT2RP7004114, NT2RP7004123, NT2RP7004348, NT2RP7004358, NT2RP7004396, NT2RP7004428, NT2RP7004481, NT2RP7004541, NT2RP7004559, NT2RP7004656, NT2RP7004722, NT2RP7004766, NT2RP7004790, NT2RP7004884, NT2RP7004915, NT2RP7004925, NT2RP7004961, NT2RP7004964, NT2RP7004975, NT2RP7005118, NT2RP7005205, NT2RP7005219, NT2RP7005323,
NT2RP7005468, NT2RP7005493, NT2RP7005513, NT2RP7005629, NT2RP7005631, NT2RP7005669, NT2RP7005846, NT2RP7006075, NT2RP7006141, NT2RP7006160, NT2RP7006162, NT2RP7006263, NT2RP7006374, NT2RP7006395, NT2RP7006408, NT2RP7006457, NT2RP7006490, NT2RP7006601, NT2RP7006619, NT2RP7006621, NT2RP7006636, NT2RP7006701, NT2RP7006853, NT2RP7006886, NT2RP7006887, NT2RP7006980, NT2RP7006986, NT2RP7006995, NT2RP7007177, NT2RP7007221, NT2RP7007226, NT2RP7007269, NT2RP7007359, NT2RP7007406, NT2RP7007480, NT2RP7007504, NT2RP7007530, NT2RP7007594, NT2RP7007610, NT2RP7007617, NT2RP7007766, NT2RP7007925, NT2RP7007975, NT2RP7008013, NT2RP7008015, NT2RP7008133, NT2RP7008137, NT2RP7008142, NT2RP7008144, NT2RP7008161,
NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009097, NT2RP7009168, NT2RP7009215, NT2RP7009225, NT2RP7009236, NT2RP7009253, NT2RP7009267, NT2RP7009363, NT2RP7009397, NT2RP7009429, NT2RP7009439, NT2RP7009466, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010612, NT2RP7010817, NT2RP7011086, NT2RP7011132, NT2RP7011268, NT2RP7011318, NT2RP7011452,
NT2RP7011570, NT2RP7011721, NT2RP7011909, NT2RP7012291, NT2RP7012516, NT2RP7012776, NT2RP7013002, NT2RP7013374, NT2RP7013499, NT2RP7013573, NT2RP7013729, NT2RP7013764, NT2RP7013795, NT2RP7013999, NT2RP7014005,- NT2RP7014178, NT2RP7014348, NT2RP7014420, NT2RP7014583, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, .
NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000182, NTONG1000213, NTONG1000214, NTONG1000247, NTONG1000257, NTONG1000264, NTONG2000040,
NTONG2000107, NTONG2000231, NTONG2000363, NTONG2000492, NTONG2000499, NTONG2000531, NTONG2000583, NTONG2000858, NTONG2000876, NTONG2000966, NTONG2001014, NTONG2001079, NTONG2001157, NTONG2001173, NTONG2001220, NTONG2001362, NTONG2001428, NTONG2001532, NTONG2001612, NTONG2001619, NTONG2001642, NTONG2001657, NTONG2001676, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2004095, NTONG2004308, NTONG2004521, NTONG2004614, NTONG2004690, NTONG2004806, NTONG2004829, NTONG2004918, NTONG2004991, NTONG2005062, NTONG2005086, NTONG2005137, NTONG2005153,
NTONG2005265, NTONG2005277, NTONG2005363, NTONG2005373, NTONG2005480, NTONG2005497, NTONG2005577, NTONG2005657, NTONG2005822, NTONG2005897, NTONG2005969, NTONG2006099, NTONG2006187, NTONG2006324, NTONG2006354, NTONG2006440, NTONG2006501, NTONG2006646, NTONG2006709, NTONG2006783, NTONG2007020, NTONG2007028, NTONG2007034, NTONG2007249, NTONG2007517, NTONG2007522, NTONG2007756, NTONG2008093, NTONG2008143, NTONG2008365, NTONG2008522, NTONG2008672, NTONG2008862, NTONG2008939, NTONG2009060, NTONG2009068, NTONG2009229, NTONG2009233, NTONG2009468, OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114,
OCBBF1000118, OCBBF1000119, OCBBF1000122, OCBBF1000130, OCBBF1000138, OCBBF1000145, OCBBF1000146, OCBBF1000153, OCBBF1000175, OCBBF1000192, OCBBF1000254, OCBBF2000126, OCBBF2000178, OCBBF2000231, OCBBF2000277, OCBBF2000287, OCBBF2000323, OCBBF2000452, OCBBF2000522, OCBBF2000677, OCBBF2000703, OCBBF2000712, OCBBF2000719, OCBBF2000824, OCBBF2000904, OCBBF2000998, OCBBF2001075, OCBBF2001101, OCBBF2001124, OCBBF2001176, OCBBF2001196, OCBBF2001307, OCBBF2001323, OCBBF2001389, OCBBF2001402, OCBBF2001473, OCBBF2001494, OCBBF2001527, OCBBF2001639, OCBBF2001681; OCBBF2001706, OCBBF2001749, OCBBF2001910, OCBBF2001931, OCBBF2001961, OCBBF2001983, OCBBF2002015, OCBBF2002124, OCBBF2002161, OCBBF2002290,
OCBBF2002357, OCBBF2002376, OCBBF2002615, OCBBF2002626, OCBBF2002663, OCBBF2002805, OCBBF2002920, OCBBF2002980, OCBBF2003028, OCBBF2003091, OCBBF2003246, OCBBF2003327, OCBBF2003543, OCBBF2003593, OCBBF2003744, OCBBF2004038, OCBBF2004104, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2006030, OCBBF2006058,
OCBBF2006113, OCBBF2006148, OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006660, OCBBF2006764, OCBBF2006849, OCBBF2006859, OCBBF2006972, OCBBF2007039, OCBBF2007051, OCBBF2007068, OCBBF2007114, OCBBF2007121, OCBBF2007184, OCBBF2007194, OCBBF2007196, OCBBF2007238, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008005, OCBBF2008116, OCBBF2008138, OCBBF2008283, OCBBF2008330, OCBBF2008340, OCBBF2008366, OCBBF2008466, OCBBF2008483, OCBBF2008511, OCBBF2008520, OCBBF2008569,
OCBBF2008586, OCBBF2008640, OCBBF2008691, OCBBF2008701, OCBBF2008724, OCBBF2008760, OCBBF2008768, OCBBF2008775, OCBBF2008790, OCBBF2008814, OCBBF2008822, OCBBF2009095, OCBBF2009115, OCBBF2009301, OCBBF2009352, OCBBF2009391, OCBBF2009536, OCBBF2009571, OCBBF2009583, OCBBF2009603, OCBBF2009788, OCBBF2009920, OCBBF2009926, OCBBF2010040, OCBBF2010140, OCBBF2010281, OCBBF2010313, OCBBF2010404, OCBBF2010416, OCBBF2010420, OCBBF2010557, OCBBF2010604, OCBBF2010709, OCBBF2010792, OCBBF2010841, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283,
OCBBF2011311, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012028, OCBBF2012039, OCBBF2012095, OCBBF2012139, OCBBF2012191, OCBBF2012262, OCBBF2012320, OCBBF2012436, OCBBF2012525, OCBBF2012678, OCBBF2012704, OCBBF2012714, OCBBF2012755, OCBBF2012812, OCBBF2012881, OCBBF2012908, OCBBF2012936, OCBBF2012990, OCBBF2013011, OCBBF2013049, OCBBF2013091, OCBBF2013123, OCBBF2013127, OCBBF2013149, OCBBF2013208, OCBBF2013285, OCBBF2013319, OCBBF2013324, OCBBF2013366, OCBBF2013481, OCBBF2013507, OCBBF2013721, OCBBF2013789, OCBBF2013843, OCBBF2013883, OCBBF2013926,
OCBBF2014052, OCBBF2014064, OCBBF2014089, OCBBF2014322, OCBBF2014386, OCBBF2014576, OCBBF2014707, OCBBF2014745, OCBBF2014828, OCBBF2014880, OCBBF2014928, OCBBF2015031, OCBBF2015081, OCBBF2015115, OCBBF2015232, OCBBF2015233, OCBBF2015285, OCBBF2015335, OCBBF2015503, OCBBF2015506, OCBBF2015599, OCBBF2015645, OCBBF2015659, OCBBF2015751, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2017035, OCBBF2017055, OCBBF2017069, OCBBF2017306, OCBBF2017325, OCBBF2017489, OCBBF2017516, OCBBF2017665, OCBBF2017882, OCBBF2017888, OCBBF2018012, OCBBF2018014, OCBBF2018084, OCBBF2018167,
OCBBF2018206, OCBBF2018229, OCBBF2018234, OCBBF2018362, OCBBF2018380, OCBBF2018581, OCBBF2018663, OCBBF2018687, OCBBF2018828, OCBBF2018934, OCBBF2019108, OCBBF2019195, OCBBF2019327, OCBBF2019684, OCBBF2019761, OCBBF2019823, OCBBF2019950, OCBBF2020318, OCBBF2020343, OCBBF2020453, OCBBF2020741, OCBBF2020801, OCBBF2020825, OCBBF2020838, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025,
OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481, OCBBF2027511, OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906,
OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036743, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000372, OCBBF3000380, OCBBF3000743, OCBBF3000830, OCBBF3001076, OCBBF3001202, OCBBF3001333, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004487, OCBBF3004972, OCBBF3005330, OCBBF3005597, OCBBF3005843,
OCBBF3006802, OCBBF3006986, OCBBF3007078, OCBBF3007704, OCBBF3008230, OCBBF3008392, OCBBF3008835, OCBBF3009192, OCBBF3009279, PANCR1000012, PANCR1000021, PANCR1000081, PANCR1000086, PANCR1000091, PANCR1000185, PEBLM1000024, PEBLM1000029, PEBLM1000034, PEBLM1000062, PEBLM1000068, PEBLM1000071, PEBLM1000083, PEBLM1000147, PEBLM1000174, PEBLM1000180, PEBLM2000112, PEBLM2000126, PEBLM2000147, PEBLM2000170, PEBLM2000180, PEBLM2000213, PEBLM2000248, PEBLM2000270, PEBLM2000321, PEBLM2000333, PEBLM2000395, PEBLM2000479, PEBLM2000502, PEBLM2001312, PEBLM2001465, PEBLM2001488, PEBLM2002432, PEBLM2002455, PEBLM2002594, PEBLM2002887, PEBLM2003426, PEBLM2003765, PEBLM2003935, PEBLM2004015, PEBLM2004216,
PEBLM2004290, PEBLM2004497, PEBLM2005282, PEBLM2005615, PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006366, PEBLM2006709, PEBLM2006912, PEBLM2007112, PEBLM2007140, PEBLM2007188, PEBLM2007296, PEBLM2007437, PEBLM2007447, PEBLM2007598, PEBLM2007832, PEBLM2007834, PEBLM2008106, PEBLM2008576, PEBLM2008605, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766,
PERIC2003001, PERIC2003090, PERIC2003287, PERIC2003349, PERIC2003452, PERIC2003699, PERIC2003720, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000070, PLACE5000080, PLACE5000105, PLACE5000115, PLACE5000116, PLACE5000129, PLACE5000139, PLACE5000153, PLACE5000171,
PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000630, PLACE6000953, PLACE6001118, PLACE6001153, PLACE6001185, PLACE6001262, PLACE6001294, PLACE6001443, PLACE6001536, PLACE6001557, PLACE6001712, PLACE6001740, PLACE6001823, PLACE6001886, PLACE6001923, PLACE6001925, PLACE6001933, PLACE6002011,
PLACE6002016, PLACE6002056, PLACE6002157, PLACE6002312, PLACE6002323, PLACE6002345, PLACE6002419, PLACE6002462, PLACE6002647, PLACE6002668, PLACE6002692, PLACE6002699, PLACE6002710, PLACE6002871, PLACE6002949, PLACE6003004, PLACE6003094, PLACE6003109, PLACE6003204, PLACE6003218, PLACE6003372, PLACE6003399, PLACE6003539, PLACE6003621, PLACE6003705, PLACE6003740, PLACE6003745, PLACE6003771, PLACE6003796, PLACE6003839, PLACE6003850, PLACE6004005, PLACE6004101, PLACE6004219, PLACE6004336, PLACE6004368, PLACE6004380, PLACE6004396, PLACE6004454, PLACE6004464, PLACE6004491, PLACE6004663, PLACE6004687, PLACE6004738, PLACE6004931, PLACE6004966, PLACE6004993, PLACE6005007, PLACE6005029, PLACE6005108,
PLACE6005234, PLACE6005283, PLACE6005294, PLACE6005328, PLACE6005351, PLACE6005423, PLACE6005487, PLACE6005535, PLACE6005546, PLACE6005559, PLACE6005579, PLACE6005691, PLACE6005786, PLACE6006042, PLACE6006077, PLACE6006137, PLACE6006158, PLACE6006186, PLACE6006217, PLACE6006222, PLACE6006266, PLACE6006287, PLACE6006394, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444,
PLACE6008453, PLACE6008640, PLACE6008750, PLACE6008768, PLACE6008824, PLACE6008989, PLACE6009006, PLACE6009008, PLACE6009228, PLACE6009237, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011627, PLACE6011774, PLACE6011881, PLACE6011907, PLACE6011970, PLACE6011975, PLACE6012108, PLACE6012168, PLACE6012297, PLACE6012308, PLACE6012574, PLACE6012908, PLACE6012936,
PLACE6012942, PLACE6013232, PLACE6013288, PLACE6013386, PLACE6013400, PLACE6013650, PLACE6013672, PLACE6013883, PLACE6013884, PLACE6013885, PLACE6014043, PLACE6014064, PLACE6014516, PLACE6014882, PLACE6015211, PLACE6015322, PLACE6015445, PLACE6015460, PLACE6015591, PLACE6015729, PLACE6015731, PLACE6015939, PLACE6016030, PLACE6016093, PLACE6016105, PLACE6016160, PLACE6016210, PLACE6016254, PLACE6016942, PLACE6016984, PLACE6017002, PLACE6017431, PLACE6017564, PLACE6017578, PLACE6017626, PLACE6017701, PLACE6017714, PLACE6018107, PLACE6018187, PLACE6018235, PLACE6018441, PLACE6018487, PLACE6018497, PLACE6018769, PLACE6018834, PLACE6018843, PLACE6018863, PLACE6018938, PLACE6019385, PLACE6019542,
PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7005840, PLACE7006051, PLACE7006090, PLACE7006240, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000120,
PROST1000123, PROST1000136, PROST1000145, PROST1000152, PROST1000167, PROST1000184, PROST1000215, PROST1000217, PROST1000246, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000526, PROST1000527, PROST1000528, PROST1000536, PROST1000559, PROST1000575, PROST2000036, PROST2000053, PROST2000067, PROST2000105, PROST2000143, PROST2000162, PROST2000165, PROST2000176, PROST2000203, PROST2000206, PROST2000212, PROST2000241, PROST2000267, PROST2000273, PROST2000274, PROST2000277, PROST2000337, PROST2000432, PROST2000452,
PROST2000463, PROST2000470, PROST2000505, PROST2000572, PROST2000717, PROST2000760, PROST2000761, PROST2000835, PROST2000893, PROST2000961, PROST2001002, PROST2001116, PROST2001138, PROST2001213, PROST2001283, PROST2001414, PROST2001415, PROST2001465, PROST2001540, PROST2001599, PROST2001611, PROST2001636, PROST2001739, PROST2001796, PROST2001805, PROST2001823, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002651, PROST2002682, PROST2002685, PROST2002722, PROST2002736, PROST2002842, PROST2002897, PROST2002906, PROST2002927,
PROST2002960, PROST2003102, PROST2003117, PROST2003210, PROST2003302, PROST2003303, PROST2003324, PROST2003340, PROST2003396, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004742, PROST2004744, PROST2004817, PROST2005039, PROST2005067, PROST2005076, PROST2005093, PROST2005121, PROST2005131, PROST2005143, PROST2005228, PROST2005272, PROST2005285, PROST2005426,
PROST2005466, PROST2005630, PROST2005683, PROST2005788, PROST2005793, PROST2005880, PROST2005886, PROST2005904, PROST2005919, PROST2005943, PROST2006008, PROST2006020, PROST2006030, PROST2006196, PROST2006201, PROST2006260, PROST2006450, PROST2006491, PROST2006510, PROST2006536, PROST2006579, PROST2006688, PROST2006766, PROST2006782, PROST2006933, PROST2006988, PROST2007122, PROST2007200, PROST2007237, PROST2007248, PROST2007289, PROST2007328, PROST2007382, PROST2007389, PROST2007416, PROST2007528, PROST2007818, PROST2007950, PROST2007956, PROST2007974, PROST2007988, PROST2008079, PROST2008088, PROST2008243, PROST2008245, PROST2008268, PROST2008271, PROST2008360, PROST2008447, PROST2008468,
PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010318, PROST2010326, PROST2010382, PROST2010400, PROST2010545, PROST2010606, PROST2010782, PROST2010885, PROST2011012, PROST2011038, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353,
PROST2012400, PROST2012504, PROST2012542, PROST2012550, PROST2012740, PROST2012780, PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900, PROST2015924, PROST2015932, PROST2016195, PROST2016246, PROST2016351, PROST2016379, PROST2016444, PROST2016462, PROST2016499,
PROST2016512, PROST2016566, PROST2016582, PROST2016668, PROST2016684, PROST2016777, PROST2016829, PROST2016860, PROST2016918, PROST2016980, PROST2017098, PROST2017128, PROST2017367, PROST2017413, PROST2017441, PROST2017529, PROST2017578, PROST2017612, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000081, PUAEN1000085, PUAEN1000087, PUAEN1000121, PUAEN1000147, PUAEN1000175,
PUAEN1000275, PUAEN1000276, PUAEN1000288, PUAEN2000080, PUAEN2000175, PUAEN2000312, PUAEN2000394, PUAEN2000497, PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005588, PUAEN2005930, PUAEN2006029, PUAEN2006328, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515,
PUAEN2008815, PUAEN2008939, PUAEN2008980, PUAEN2009174, PUAEN2009348, PUAEN2009534, PUAEN2009655, PUAEN2009795, PUAEN2009852, PUAEN2010346, PUAEN2010824, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000065, SALGL1000171, SKMUS1000030, SKMUS1000064, SKMUS1000067, SKMUS1000102, SKMUS1000104, SKMUS1000110, SKMUS1000118, SKMUS1000124, SKMUS1000129, SKMUS1000177, SKMUS2000020, SKMUS2000061, SKMUS2000074, SKMUS2000078, SKMUS2000117, SKMUS2000137, SKMUS2000198, SKMUS2000271, SKMUS2000287, SKMUS2000317,
SKMUS2000330, SKMUS2000339, SKMUS2000343, SKMUS2000365, SKMUS2000380, SKMUS2000390, SKMUS2000416, SKMUS2000458, SKMUS2000467, SKMUS2000468, SKMUS2000515, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000873, SKMUS2000894, SKMUS2000898, SKMUS2000945, SKMUS2000973, SKMUS2000980, SKMUS2001008, SKMUS2001014, SKMUS2001129, SKMUS2001157, SKMUS2001164, SKMUS2001199, SKMUS2001201, SKMUS2001215, SKMUS2001247, SKMUS2001254, SKMUS2001271, SKMUS2001287, SKMUS2001364, SKMUS2001398, SKMUS2001402, SKMUS2001412, SKMUS2001492, SKMUS2001501, SKMUS2001543, SKMUS2001580, SKMUS2001585,
SKMUS2001608, SKMUS2001631, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003744, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232,
SKMUS2009479, SKMUS2009557, SKNMC1000110, SKNMC1000123, SKNMC1000124, SKNMC1000229, SKNMC1000251, SKNMC1000264, SKNMC2000224, SKNMC2000256, SKNMC2000356, SKNMC2000374, SKNMC2000583, SKNMC2000593, SKNMC2000612, SKNMC2000622, SKNMC2000698, SKNMC2000966, SKNMC2001113, SKNMC2001503, SKNMC2001555, SKNMC2001596, SKNMC2002402, SKNMC2003639, SKNMC2003924, SKNMC2003987, SKNMC2004457, SKNMC2004643, SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000086, SKNSH1000174, SKNSH1000301, SKNSH1000308, SKNSH1000416, SKNSH2000051, SKNSH2000101, SKNSH2000163, SKNSH2000250, SKNSH2000319, SKNSH2000482,
SKNSH2000516, SKNSH2000550, SKNSH2000716, SKNSH2000971, SKNSH2001222, SKNSH2001875, SKNSH2001931, SKNSH2002054, SKNSH2002325, SKNSH2002768, SKNSH2002866, SKNSH2003064, SKNSH2003174, SKNSH2003422, SKNSH2003466, SKNSH2003483, SKNSH2003528, SKNSH2004039, SKNSH2005061, SKNSH2005120, SKNSH2005194, SKNSH2005792, SKNSH2005816, SKNSH2005981, SKNSH2006234, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000039, SMINT1000042, SMINT1000048, SMINT1000051, SMINT1000054, SMINT1000071, SMINT1000075, SMINT1000078, SMINT1000091,
SMINT1000100, SMINT1000103, SMINT1000118, SMINT1000131, SMINT1000137, SMINT1000192, SMINT2000007, SMINT2000032, SMINT2000040, SMINT2000060, SMINT2000073, SMINT2000084, SMINT2000145, SMINT2000159, SMINT2000176, SMINT2000185, SMINT2000227, SMINT2000229, SMINT2000232, SMINT2000239, SMINT2000267, SMINT2000277, SMINT2000396, SMINT2000400, SMINT2000468, SMINT2000518, SMINT2000529, SMINT2000530, SMINT2000537, SMINT2000538, SMINT2000541, SMINT2000558, SMINT2000562, SMINT2000568, SMINT2000602, SMINT2000609, SMINT2000747, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535,
SMINT2001559, SMINT2001609, SMINT2001615, SMINT2001683, SMINT2001731, SMINT2001950, SMINT2002126, SMINT2002159, SMINT2002202, SMINT2002210, SMINT2002224, SMINT2002281, SMINT2002311, SMINT2002328, SMINT2002414, SMINT2002457, SMINT2002620, SMINT2002743, SMINT2002778, SMINT2002880, SMINT2002884, SMINT2002899, SMINT2002974, SMINT2002976, SMINT2003003, SMINT2003128, SMINT2003150, SMINT2003317, SMINT2003340, SMINT2003386, SMINT2003423, SMINT2003505, SMINT2003551, SMINT2003569, SMINT2003641, SMINT2003644, SMINT2003866, SMINT2004023, SMINT2004037, SMINT2004047, SMINT2004086, SMINT2004144, SMINT2004280, SMINT2004339, SMINT2004350, SMINT2004414, SMINT2004473, SMINT2004547, SMINT2004583, SMINT2004729,
SMINT2004781, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2005956, SMINT2006078, SMINT2006205, SMINT2006331, SMINT2006596, SMINT2006641, SMINT2006648, SMINT2006708, SMINT2006716, SMINT2007062, SMINT2007101, SMINT2007140, SMINT2007187, SMINT2007219, SMINT2007294, SMINT2007365, SMINT2007391, SMINT2007433, SMINT2007508, SMINT2007526, SMINT2007579, SMINT2007647, SMINT2007790, SMINT2007792, SMINT2007796, SMINT2007867, SMINT2007932, SMINT2008042, SMINT2008054, SMINT2008133, SMINT2008277, SMINT2008329,
SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010672, SMINT2010753, SMINT2010853, SMINT2010897, SMINT2010959,
SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014489, SMINT2014721, SMINT2015006, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016440, SMINT2016477,
SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMI,NT2018353, SMINT2018592, SMINT2018681, SMINT2019017, SMINT2019105, SMINT2019142, SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189,
SPLEN2000197, SPLEN2000205, SPLEN2000210, SPLEN2000222, SPLEN2000242, SPLEN2000243, SPLEN2000247, SPLEN2000258, SPLEN2000270, SPLEN2000307, SPLEN2000310, SPLEN2000341, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000698, SPLEN2001135, SPLEN2001157, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001626, SPLEN2001650, SPLEN2001689, SPLEN2001710, SPLEN2001781, SPLEN2001881, SPLEN2001912, SPLEN2001945,
SPLEN2002007, SPLEN2002053, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002463, SPLEN2002467, SPLEN2002477, SPLEN2002493, SPLEN2002662, SPLEN2002695, SPLEN2002707, SPLEN2002744, SPLEN2002917, SPLEN2002931, SPLEN2002957, SPLEN2003160, SPLEN2003204, SPLEN2003219, SPLEN2003297, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004343, SPLEN2004346, SPLEN2004368, SPLEN2004611, SPLEN2004662, SPLEN2004844, SPLEN2005009, SPLEN2005227, SPLEN2005272, SPLEN2005342, SPLEN2005416,
SPLEN2005450, SPLEN2005474, SPLEN2005560, SPLEN2005727, SPLEN2005783, SPLEN2005790, SPLEN2005806, SPLEN2005838, SPLEN2005927, SPLEN2005939, SPLEN2006143, SPLEN2006268, SPLEN2006283, SPLEN2006325, SPLEN2006389, SPLEN2006425, SPLEN2006671, SPLEN2006701, SPLEN2006785, SPLEN2006875, SPLEN2007182, SPLEN2007276, SPLEN2007314, SPLEN2007326, SPLEN2007350, SPLEN2007388, SPLEN2007619, SPLEN2007653, SPLEN2007689, SPLEN2007695, SPLEN2007794, SPLEN2007879, SPLEN2007926, SPLEN2007951, SPLEN2008007, SPLEN2008135, SPLEN2008164, SPLEN2008317, SPLEN2008496, SPLEN2008786, SPLEN2009088, SPLEN2009163, SPLEN2009315, SPLEN2009340, SPLEN2009512, SPLEN2009513, SPLEN2009541, SPLEN2009548, SPLEN2009581, SPLEN2009733,
SPLEN2009803, SPLEN2009884, SPLEN2009918, SPLEN2010004, SPLEN2010015, SPLEN2010112, SPLEN2010119, SPLEN2010146, SPLEN2010187, SPLEN2010350, SPLEN2010395, SPLEN2010415, SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011737, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453,
SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889, SPLEN2012961, SPLEN2013108, SPLEN2013195, SPLEN2013503, SPLEN2013644, SPLEN2013648, SPLEN2013670, SPLEN2013673, SPLEN2013690, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014645, SPLEN2014669, SPLEN2014711, SPLEN2014739, SPLEN2014860, SPLEN2014865, SPLEN2014919,
SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016602, SPLEN2016627, SPLEN2016678, SPLEN2016743, SPLEN2016773, SPLEN2016781, SPLEN2016840, SPLEN2016863,
SPLEN2016922, SPLEN2016932, SPLEN2016973, SPLEN2017104, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018157, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019311, SPLEN2019323, SPLEN2019349, SPLEN2019375, SPLEN2019379, SPLEN2019446,
SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920,
SPLEN2023024, SPLEN2023118, SPLEN2023423, SPLEN2023710, SPLEN2023791, SPLEN2023977, SPLEN2024070, SPLEN2024127, SPLEN2024232, SPLEN2024273, SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028994, SPLEN2029051, SPLEN2029176, SPLEN2029295, SPLEN2029380, SPLEN2029522,
SPLEN2029683, SPLEN2029727, SPLEN2029904, SPLEN2029912, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036103, SPLEN2036326, SPLEN2036475,
SPLEN2036501, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038407, SPLEN2038473, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041585, SPLEN2041645, SPLEN2041700, SPLEN2041720, SPLEN2041977, SPLEN2042051, SPLEN2042303, SPLEN2042521, SPLEN2042598, SPLEN2042714, SPLEN2042920, STOMA1000013, STOMA1000047, STOMA1000052, STOMA1000074, STOMA1000091, STOMA1000117, STOMA1000133,
STOMA1000175, STOMA1000186, STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000383, STOMA2000386, STOMA2000478, STOMA2000482, STOMA2000529, STOMA2000539, STOMA2000567, STOMA2000640, STOMA2000678, STOMA2000686, STOMA2000888, STOMA2001025, STOMA2002141, STOMA2002367, STOMA2002688, STOMA2003158, STOMA2003289, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004925, STOMA2005120, STOMA2005664, STOMA2005667, STOMA2005746, STOMA2006181, STOMA2006213, STOMA2006325,
STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008838, STOMA2009250, STOMA2009253, STOMA2009256, STOMA2009289, THYMU1000010, THYMU1000017, THYMU1000025, THYMU1000029, THYMU1000032, THYMU1000033, THYMU1000041, THYMU1000060, THYMU1000074, THYMU1000098, THYMU1000103, THYMU1000105, THYMU1000136, THYMU1000142, THYMU1000144, THYMU1000158, THYMU1000176, THYMU1000179, THYMU1000191, THYMU1000198, THYMU1000217, THYMU1000240, THYMU1000242, THYMU1000291, THYMU1000316, THYMU1000318, THYMU1000336, THYMU1000366, THYMU1000382, THYMU1000393, THYMU1000394, THYMU1000399, THYMU1000426,
THYMU1000440, THYMU1000459, THYMU1000473, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140, THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000684, THYMU2000702, THYMU2000767, THYMU2000775, THYMU2000800, THYMU2000879,
THYMU2000932, THYMU2000950, THYMU2000971, THYMU2000982, THYMU2000990, THYMU2001018, THYMU2001053, THYMU2001071, THYMU2001139, THYMU2001195, THYMU2001202, THYMU2001203, THYMU2001256, THYMU2001270, THYMU2001291, THYMU2001314, THYMU2001325, THYMU2001357, THYMU2001422, THYMU2001443, THYMU2001515, THYMU2001521, THYMU2001556, THYMU2001622, THYMU2001727, THYMU2001819, THYMU2001825, THYMU2001868, THYMU2001898, THYMU2001900, THYMU2001916, THYMU2001989, THYMU2002023, THYMU2002037, THYMU2002080, THYMU2002109, THYMU2002147, THYMU2002154, THYMU2002157, THYMU2002177, THYMU2002203, THYMU2002229, THYMU2002242, THYMU2002338, THYMU2002356, THYMU2002376, THYMU2002384, THYMU2002450, THYMU2002490, THYMU2002548,
THYMU2002695, THYMU2002745, THYMU2002756, THYMU2002789, THYMU2002792, THYMU2002815, THYMU2002852, THYMU2002910, THYMU2002950, THYMU2002983, THYMU2003069, THYMU2003107, THYMU2003124, THYMU2003133, THYMU2003166, THYMU2003232, THYMU2003242, THYMU2003249, THYMU2003270, THYMU2003282, THYMU2003287, THYMU2003336, THYMU2003366, THYMU2003397, THYMU2003440, THYMU2003470, THYMU2003479, THYMU2003542, THYMU2003632, THYMU2003650, THYMU2003700, THYMU2003760, THYMU2003803, THYMU2003855, THYMU2003907, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004356, THYMU2004452, THYMU2004508, THYMU2004512,
THYMU2004635, THYMU2004639, THYMU2004668, THYMU2004677, THYMU2004688, THYMU2004693, THYMU2004733, THYMU2004835, THYMU2004843, THYMU2004906, THYMU2004916, THYMU2004986, THYMU2005001, THYMU2005003, THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005480, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005997, THYMU2006001, THYMU2006048, THYMU2006098, THYMU2006160, THYMU2006170, THYMU2006252, THYMU2006277, THYMU2006303, THYMU2006324, THYMU2006350,
THYMU2006357, THYMU2006365, THYMU2006420, THYMU2006468, THYMU2006505, THYMU2006610, THYMU2006666, THYMU2006712, THYMU2006913, THYMU2006946, THYMU2006965, THYMU2007025, THYMU2007036, THYMU2007095, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226,
THYMU2008282, THYMU2008339, THYMU2008350, THYMU2008351, THYMU2008452, THYMU2008643, THYMU2008686, THYMU2008691, THYMU2008714, THYMU2008725, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010519, THYMU2010637, THYMU2010671, THYMU2010699, THYMU2010705,
THYMU2010779, THYMU2010816, THYMU2010831, THYMU2010848, THYMU2011053, THYMU2011063, THYMU2011072, THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012631, THYMU2012665, THYMU2012690, THYMU2012701, THYMU2012807,
THYMU2012882, THYMU2012891, THYMU2012902, THYMU2012919, THYMU2013023, THYMU2013047, THYMU2013051, THYMU2013089, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015321, THYMU2015439, THYMU2015441, THYMU2015479, THYMU2015762, THYMU2015775, THYMU2015825, THYMU2015918, THYMU2015943, THYMU2015984,
THYMU2016113, THYMU2016164, THYMU2016204, THYMU2016219, THYMU2016360, THYMU2016496, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017675, THYMU2017707, THYMU2017844, THYMU2017878, THYMU2018028, THYMU2018071, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019021, THYMU2019054, THYMU2019197, THYMU2019210, THYMU2019364, THYMU2019436, THYMU2019442, THYMU2019587,
THYMU2019599, THYMU2019686, THYMU2019813, THYMU2020198, THYMU2020289, THYMU2020416, THYMU2020491, THYMU2020499, THYMU2020560, THYMU2020667, THYMU2020830, THYMU2020883, THYMU2020959, THYMU2021361, THYMU2021509, THYMU2021597, THYMU2021684, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024071, THYMU2024121, THYMU2024185, THYMU2024207, THYMU2024616, THYMU2024684, THYMU2024748, THYMU2025042, THYMU2025189, THYMU2025319, THYMU2025325, THYMU2025406, THYMU2025557, THYMU2025572, THYMU2025707, THYMU2025849, THYMU2025909, THYMU2025939, THYMU2026182, THYMU2026350,
THYMU2026530, THYMU2026531, THYMU2026835, THYMU2026904, THYMU2027053, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031579, THYMU2031847, THYMU2031890, THYMU2031994, THYMU2032014, THYMU2032035,
THYMU2032080, THYMU2032150, THYMU2032358, THYMU2032437, THYMU2032601, THYMU2032655, THYMU2032715, THYMU2032732, THYMU2032825, THYMU2032976, THYMU2033053, THYMU2033059, THYMU2033070, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2034917, THYMU2035064, THYMU2035078, THYMU2035101, THYMU2035319, THYMU2035321, THYMU2035388, THYMU2035400, THYMU2035554, THYMU2035710, THYMU2036105, THYMU2036207, THYMU2036215, THYMU2036252, THYMU2036265, THYMU2036366, THYMU2036459, THYMU2036461, THYMU2036604, THYMU2036653,
THYMU2037081, THYMU2037208, THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427, THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360,
THYMU3000390, THYMU3000420, THYMU3000490, THYMU3000504, THYMU3000542, THYMU3000655, THYMU3000826, THYMU3000841, THYMU3001077, THYMU3001082, THYMU3001083, THYMU3001133, THYMU3001151, THYMU3001379, THYMU3001472, THYMU3001593, THYMU3001883, THYMU3001939, THYMU3001991, THYMU3002452, THYMU3002578, THYMU3002661, THYMU3003007, THYMU3003092, THYMU3003212, THYMU3003350, THYMU3003403, THYMU3003565, THYMU3003763, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008935, THYMU3009006, THYMU3009255, THYMU3009391, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701, THYMU3015042, THYMU3015457, THYMU3015571,
THYMU3015592, THYMU3015625, THYMU3015647, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642, THYMU3025683, THYMU3025772, THYMU3025865, THYMU3026000,
THYMU3026350, THYMU3026479, THYMU3026532, THYMU3026783, THYMU3026869, THYMU3027251, THYMU3027540, THYMU3027655, THYMU3027671, THYMU3028075, THYMU3028410, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033630, THYMU3033649, THYMU3033754, THYMU3033759, THYMU3034078, THYMU3034099, THYMU3034453, THYMU3034616, THYMU3034853, THYMU3034867, THYMU3034983, THYMU3036200, THYMU3036310, THYMU3036934, THYMU3036953, THYMU3037052, THYMU3037192,
THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482, THYMU3043597, THYMU3043688, THYMU3043779,
THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240,. TKIDN2000246, TKIDN2000319, TKIDN2000464, TKIDN2000521, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078, TKIDN2003366, TKIDN2003396, TKIDN2003413, TKIDN2003597, TKIDN2003601,
TKIDN2004185, TKIDN2004386, TKIDN2004458, TKIDN2004748, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161, TKIDN2015263, TKIDN2015285, TKIDN2015788, TKIDN2016309, TKIDN2016399, TKIDN2016846,
TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192, TLIVE2007528, TLIVE2007607, TLIVE2007736, TLIVE2007816, TLIVE2007919, TLIVE2008083, TLIVE2008213, TLIVE2008221,
TLIVE2008712, TLIVE2008797, TLIVE2009087, TLIVE2009163, TLIVE2009489, TLIVE2009541, TLUNG2000179, TOVAR2000476, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000106, TRACH1000125, TRACH1000140, TRACH1000144, TRACH1000181, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235, TRACH2000243, TRACH2000250, TRACH2000259, TRACH2000272, TRACH2000282, TRACH2000287, TRACH2000289, TRACH2000312,
TRACH2000336, TRACH2000393, TRACH2000411, TRACH2000472, TRACH2000496, TRACH2000497, TRACH2000502, TRACH2000522, TRACH2000559, TRACH2000660, TRACH2000665, TRACH2000675, TRACH2000677, TRACH2000703, TRACH2000767, TRACH2000775, TRACH2000807, TRACH2000873, TRACH2000894, TRACH2000898, TRACH2000923, TRACH2000944, TRACH2000981, TRACH2000986, TRACH2001037, TRACH2001060, TRACH2001085, TRACH2001109, TRACH2001121, TRACH2001147, TRACH2001154, TRACH2001188, TRACH2001275, TRACH2001335, TRACH2001339, TRACH2001341, TRACH2001347, TRACH2001395, TRACH2001400, TRACH2001403, TRACH2001416, TRACH2001428, TRACH2001432, TRACH2001443, TRACH2001463, TRACH2001474, TRACH2001523, TRACH2001525, TRACH2001532, TRACH2001549,
TRACH2001592, TRACH2001596, TRACH2001621, TRACH2001646, TRACH2001651, TRACH2001684, TRACH2001898, TRACH2001933, TRACH2001968, TRACH2001996, TRACH2002043, TRACH2002054, TRACH2002092, TRACH2002100, TRACH2002138, TRACH2002333, TRACH2002784, TRACH2002840, TRACH2002847, TRACH2002853, TRACH2002862, TRACH2002954, TRACH2002960, TRACH2002988, TRACH2003070, TRACH2003272, TRACH2003347, TRACH2003484, TRACH2003516, TRACH2003605, TRACH2003736, TRACH2003759, TRACH2003941, TRACH2004054, TRACH2004087, TRACH2004109, TRACH2004170, TRACH2004183, TRACH2004251, TRACH2004292, TRACH2004399, TRACH2004428, TRACH2004458, TRACH2004499, TRACH2004521, TRACH2004570, TRACH2004845, TRACH2004887, TRACH2004950, TRACH2005004,
TRACH2005017, TRACH2005066, TRACH2005159, TRACH2005314, TRACH2005444, TRACH2005666, TRACH2005698, TRACH2005710, TRACH2005720, TRACH2005769, TRACH2005796, TRACH2006015, TRACH2006049, TRACH2006097, TRACH2006194, TRACH2006327, TRACH2006331, TRACH2006387, TRACH2006528, TRACH2006670, TRACH2006762, TRACH2006870, TRACH2006918, TRACH2007059, TRACH2007324, TRACH2007358, TRACH2007483, TRACH2007542, TRACH2007577, TRACH2007674, TRACH2007676, TRACH2007688, TRACH2007733, TRACH2007754, TRACH2007816, TRACH2007894, TRACH2007988, TRACH2008112, TRACH2008113, TRACH2008127, TRACH2008130, TRACH2008278, TRACH2008478, TRACH2008540, TRACH2008583, TRACH2008597, TRACH2008820, TRACH2008891, TRACH2008921, TRACH2009006,
TRACH2009060, TRACH2009123, TRACH2009268, TRACH2009291, TRACH2009348, TRACH2009358, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587, TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011290, TRACH2011293, TRACH2011302, TRACH2011500, TRACH2011574, TRACH2011894, TRACH2011956, TRACH2012000, TRACH2012138, TRACH2012242, TRACH2012370, TRACH2012497, TRACH2012536, TRACH2012562, TRACH2012742, TRACH2012823, TRACH2012918,
TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045, TRACH2014046, TRACH2014082, TRACH2014268, TRACH2014284, TRACH2014337, TRACH2014356, TRACH2014371, TRACH2014442, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2014997, TRACH2015180, TRACH2015243, TRACH2015316, TRACH2015381, TRACH2015486, TRACH2015654, TRACH2015939, TRACH2015987, TRACH2016080, TRACH2016131, TRACH2016286, TRACH2016317, TRACH2016347,
TRACH2016380, TRACH2016384, TRACH2016410, TRACH2016481, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498, TRACH2017609, TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018512, TRACH2018646, TRACH2018663, TRACH2018718, TRACH2018835, TRACH2018882, TRACH2018914, TRACH2018950, TRACH2019024, TRACH2019046, TRACH2019151, TRACH2019154, TRACH2019176, TRACH2019248, TRACH2019309, TRACH2019425, TRACH2019473, TRACH2019602, TRACH2019661, TRACH2019672, TRACH2019673,
TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839, TRACH2023246, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025569, TRACH2025705, TRACH2025749, TRACH2025753, TRACH2025810, TRACH2025853, TRACH2025911,
TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000692, TRACH3000780, TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003586, TRACH3003683, TRACH3003780, TRACH3003805, TRACH3004113, TRACH3004288, TRACH3004303, TRACH3004412, TRACH3004424, TRACH3004456,
TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085, TRACH3005102, TRACH3005173, TRACH3005191, TRACH3005274, TRACH3005294, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006800, TRACH3006889, TRACH3006985, TRACH3007097, TRACH3007268, TRACH3007274, TRACH3007277, TRACH3007391, TRACH3007541, TRACH3007595, TRACH3007627, TRACH3007689,
TRACH3007766, TRACH3007866, TRACH3007995, TRACH3008042, TRACH3008061, TRACH3008201, TRACH3008535, TRACH3008632, TRACH3008688, TRACH3008697, TRACH3008713, TRACH3008887, TRACH3008902, TRACH3008990, TRACH3009001, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942,
TRACH3013072, TRACH3013426, TRACH3013454, TRACH3013558, TRACH3013684, TRACH3013693, TRACH3013700, TRACH3013766, TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019347,
TRACH3019370, TRACH3019447, TRACH3019598, TRACH3019621, TRACH3019662, TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823,
TRACH3025302, TRACH3025316, TRACH3025346, TRACH3025520, TRACH3026148, TRACH3026283, TRACH3026299, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660,
TRACH3031678, TRACH3031936, TRACH3032044, TRACH3032065, TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036997, TRACH3037063, TRACH3037267, TRACH3037288, TRACH3037505, TRACH3037573, TRACH3037696,
TRACH3037897, TRACH3038086, TRACH3038399, TSTOM1000135, TSTOM1000186, TSTOM2000235, TSTOM2000442, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682, TUTER1000014, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002323, TUTER2002356, TUTER2002729, UMVEN1000122, UMVEN1000156, UMVEN1000186, UMVEN2000046, UMVEN2000069, UMVEN2000121, UMVEN2000354, UTERU1000008, UTERU1000015, UTERU1000024, UTERU1000031, UTERU1000032, UTERU1000057,
UTERU1000065, UTERU1000077, UTERU1000093, UTERU1000096, UTERU1000106, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000047, UTERU2000074, UTERU2000099, UTERU2000154, UTERU2000197, UTERU2000218, UTERU2000238, UTERU2000243, UTERU2000260, UTERU2000300, UTERU2000329, UTERU2000338, UTERU2000349, UTERU2000377, UTERU2000393, UTERU2000418, UTERU2000465, UTERU2000485, UTERU2000517, UTERU2000524, UTERU2000537, UTERU2000539, UTERU2000541, UTERU2000546, UTERU2000550, UTERU2000569, UTERU2000607, UTERU2000629, UTERU2000649, UTERU2000663, UTERU2000696, UTERU2000794, UTERU2000830, UTERU2000844,
UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002332, UTERU2002473, UTERU2002547, UTERU2002693, UTERU2002733, UTERU2002736, UTERU2002737, UTERU2002826, UTERU2002964, UTERU2003035, UTERU2003057, UTERU2003126, UTERU2003135, UTERU2003399, UTERU2003411, UTERU2003456, UTERU2003577, UTERU2003704, UTERU2003926, UTERU2003973, UTERU2004015, UTERU2004037, UTERU2004039, UTERU2004061, UTERU2004163, UTERU2004299, UTERU2004461, UTERU2004520, UTERU2004564, UTERU2004698, UTERU2004861, UTERU2004929, UTERU2005004, UTERU2005050,
UTERU2005179, UTERU2005346, UTERU2005446, UTERU2005449, UTERU2005450, UTERU2005533, UTERU2005601, UTERU2005664, UTERU2005822, UTERU2005903, UTERU2005905, UTERU2006103, UTERU2006115, UTERU2006137, UTERU2006182, UTERU2006400, UTERU2006412, UTERU2006429, UTERU2006486, UTERU2006524, UTERU2006547, UTERU2006568, UTERU2006593, UTERU2006643, UTERU2006651, UTERU2006705, UTERU2006899, UTERU2007004, UTERU2007075, UTERU2007081, UTERU2007128, UTERU2007253, UTERU2007267, UTERU2007499, UTERU2007639, UTERU2007724, UTERU2007924, UTERU2007942, UTERU2008018, UTERU2008019, UTERU2008027, UTERU2008040, UTERU2008077, UTERU2008130, UTERU2008347, UTERU2008426, UTERU2008516, UTERU2008561, UTERU2008653, UTERU2008705,
UTERU2008707, UTERU2008785, UTERU2008845, UTERU2008901, UTERU2008930, UTERU2008939, UTERU2009094, UTERU2009120, UTERU2009131, UTERU2009147, UTERU2009206, UTERU2009283, UTERU2009414, UTERU2009483, UTERU2009510, UTERU2009538, UTERU2009540, UTERU2009776, UTERU2009904, UTERU2009951, UTERU2009972, UTERU2010115, UTERU2010124, UTERU2010164, UTERU2010226, UTERU2010231, UTERU2010304, UTERU2010320, UTERU2010431, UTERU2010525, UTERU2010651, UTERU2010724, UTERU2010747, UTERU2011195, UTERU2011199, UTERU2011220, UTERU2011261, UTERU2011287, UTERU2011410, UTERU2011574, UTERU2011621, UTERU2011657, UTERU2011741, UTERU2011806, UTERU2011811, UTERU2011897, UTERU2011906, UTERU2011962, UTERU2011968, UTERU2012031,
UTERU2012101, UTERU2012114, UTERU2012252, UTERU2012333, UTERU2012407, UTERU2012526, UTERU2012581, UTERU2012610, UTERU2012615, UTERU2012688, UTERU2012703, UTERU2012715, UTERU2012741, UTERU2012767, UTERU2012786, UTERU2012856, UTERU2012890, UTERU2012938, UTERU2012976, UTERU2013048, UTERU2013078, UTERU2013231, UTERU2013280, UTERU2013322, UTERU2013483, UTERU2013491, UTERU2013502, UTERU2013586, UTERU2013926, UTERU2013976, UTERU2014001, UTERU2014223, UTERU2014398, UTERU2014464, UTERU2014548, UTERU2014631, UTERU2014668, UTERU2014678, UTERU2014728, UTERU2014998, UTERU2015062, UTERU2015087, UTERU2015108, UTERU2015190, UTERU2015198, UTERU2015202, UTERU2015405, UTERU2015640, UTERU2015830, UTERU2015880,
UTERU2016147, UTERU2016157, UTERU2016464, UTERU2016669, UTERU2016757, UTERU2016761, UTERU2016799, UTERU2016896, UTERU2016902, UTERU2016979, UTERU2016981, UTERU2017123, UTERU2017303, UTERU2017421, UTERU2017492, UTERU2017613, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU2018784, UTERU2018789, UTERU2018811, UTERU2018867, UTERU2018881, UTERU2018884, UTERU2018955, UTERU2019005, UTERU2019038, UTERU2019163, UTERU2019257, UTERU2019453, UTERU2019491, UTERU2019534, UTERU2019681, UTERU2019706, UTERU2019710,
UTERU2019940, UTERU2019959, UTERU2019964, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023550, UTERU2023651, UTERU2023687, UTERU2023712, UTERU2023941, UTERU2024002, UTERU2024042, UTERU2024141, UTERU2024481, UTERU2024656, UTERU2024758, UTERU2024820, UTERU2024881, UTERU2024969, UTERU2025025, UTERU2025041, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591, UTERU2027616, UTERU2027941,
UTERU2028377, UTERU2028734, UTERU2029503, UTERU2029660, UTERU2029742, UTERU2029953, UTERU2030270, UTERU2030280, UTERU2031060, UTERU2031084, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034147, UTERU2034695, UTERU2035114, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036347, UTERU2036507, UTERU2036512, UTERU2036530, UTERU2036623,
UTERU2036690, UTERU2037224, UTERU2037361, UTERU2037423, UTERU2037577, UTERU2037674, UTERU2037791, UTERU2037843, UTERU2038171, UTERU2038251, UTERU3000226, UTERU3000298, UTERU3000402, UTERU3000645, UTERU3000665, UTERU3000670, UTERU3000727, UTERU3000738, UTERU3000828, UTERU3000899, UTERU3000959, UTERU3001029, UTERU3001053, UTERU3001059, UTERU3001158, UTERU3001240, UTERU3001394, UTERU3001542, UTERU3001558, UTERU3001571, UTERU3001585, UTERU3001632, UTERU3001652, UTERU3001766, UTERU3001988, UTERU3002209, UTERU3002218, UTERU3002383, UTERU3002600, UTERU3002620, UTERU3002667, UTERU3002701, UTERU3002731, UTERU3002768, UTERU3002786, UTERU3002993, UTERU3003116, UTERU3003135, UTERU3003178, UTERU3003465,
UTERU3003495, UTERU3003523, UTERU3003774, UTERU3003776, UTERU3004384, UTERU3004418, UTERU3004477, UTERU3004523, UTERU3004616, UTERU3004635, UTERU3004709, UTERU3004938, UTERU3004992, UTERU3005049, UTERU3005205, UTERU3005230, UTERU3005264, UTERU3005422, UTERU3005460, UTERU3005536, UTERU3005585, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007021, UTERU3007104, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690,
UTERU3009775, UTERU3009979, UTERU3009987, UTERU3010029, UTERU3010526, UTERU3010604, UTERU3010892, UTERU3010919, UTERU3011063, UTERU3011092, UTERU3011273, UTERU3011398, UTERU3011558, UTERU3011579, UTERU3011795, UTERU3011837, UTERU3012293, UTERU3012414, UTERU3012476, UTERU3012599, UTERU3012999, UTERU3013167, UTERU3013302, UTERU3013781, UTERU3014274, UTERU3014446, UTERU3014611, UTERU3014647, UTERU3014791, UTERU3014906, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3018154, UTERU3018172, UTERU3018255, UTERU3018616, UTERU3018711, UTERU3019078

Of the 17,176 genes that include the cDNAs of the present invention, genes whose expression levels were high enough to detect in each of the THYMU, TKIDN, TLIVE, TLUNG, TOVAR, TRACH, and TSTOM libraries were analyzed for expression frequency. The analysis results are described below. Each is a relative value, where the expression level over all tissues is taken as 100. The larger this value, the greater the level of expression.

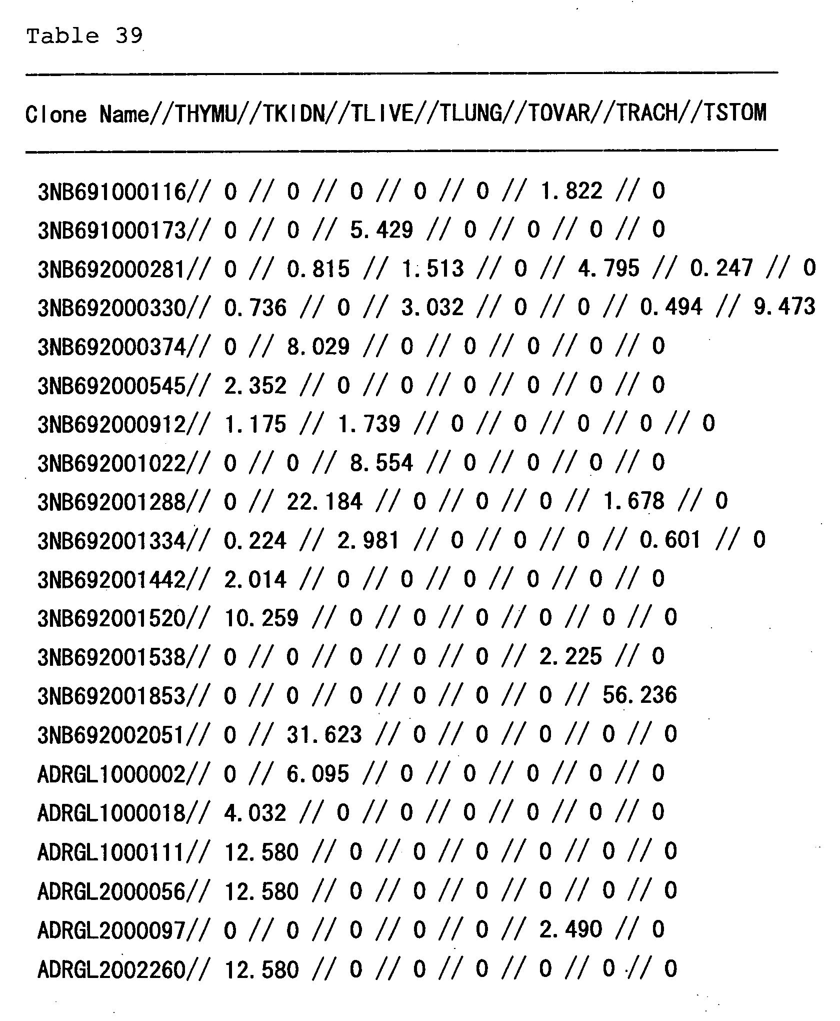

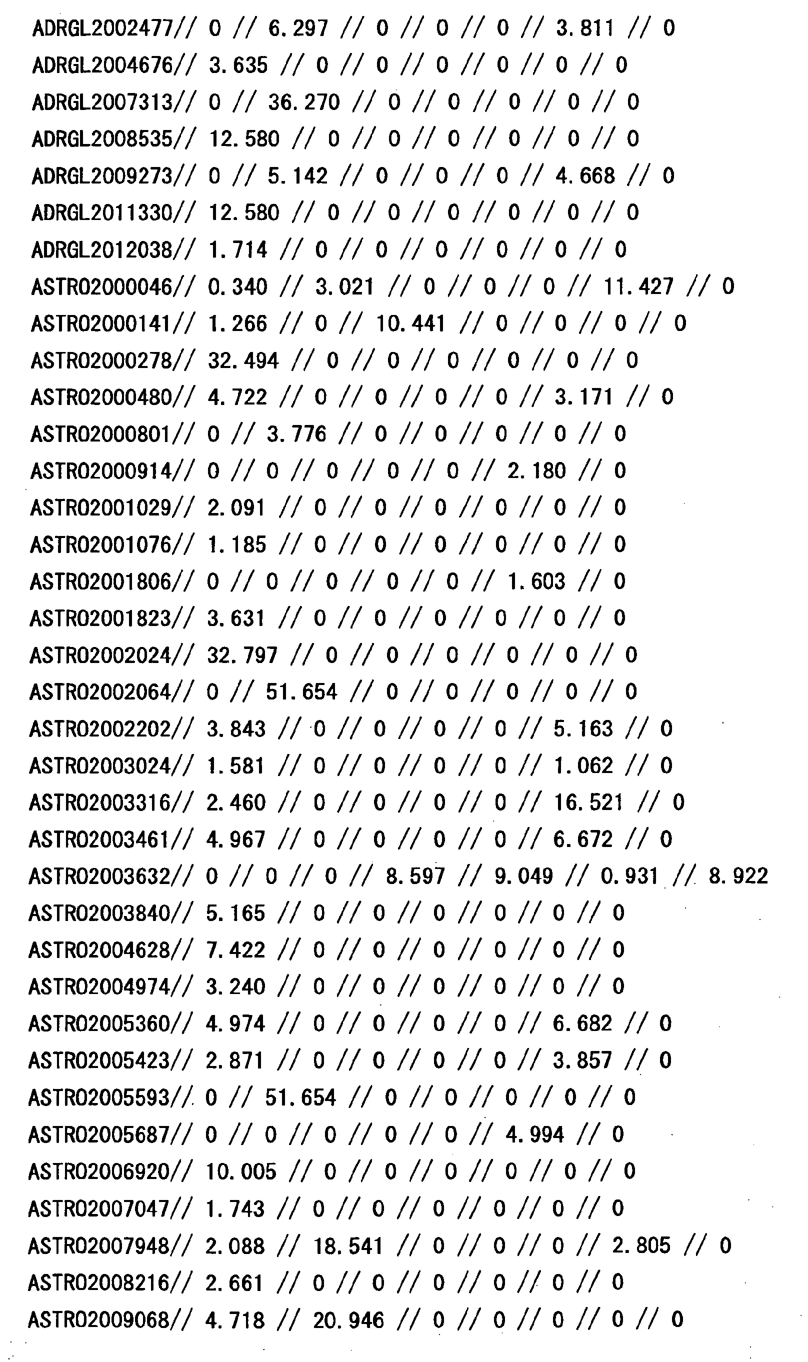

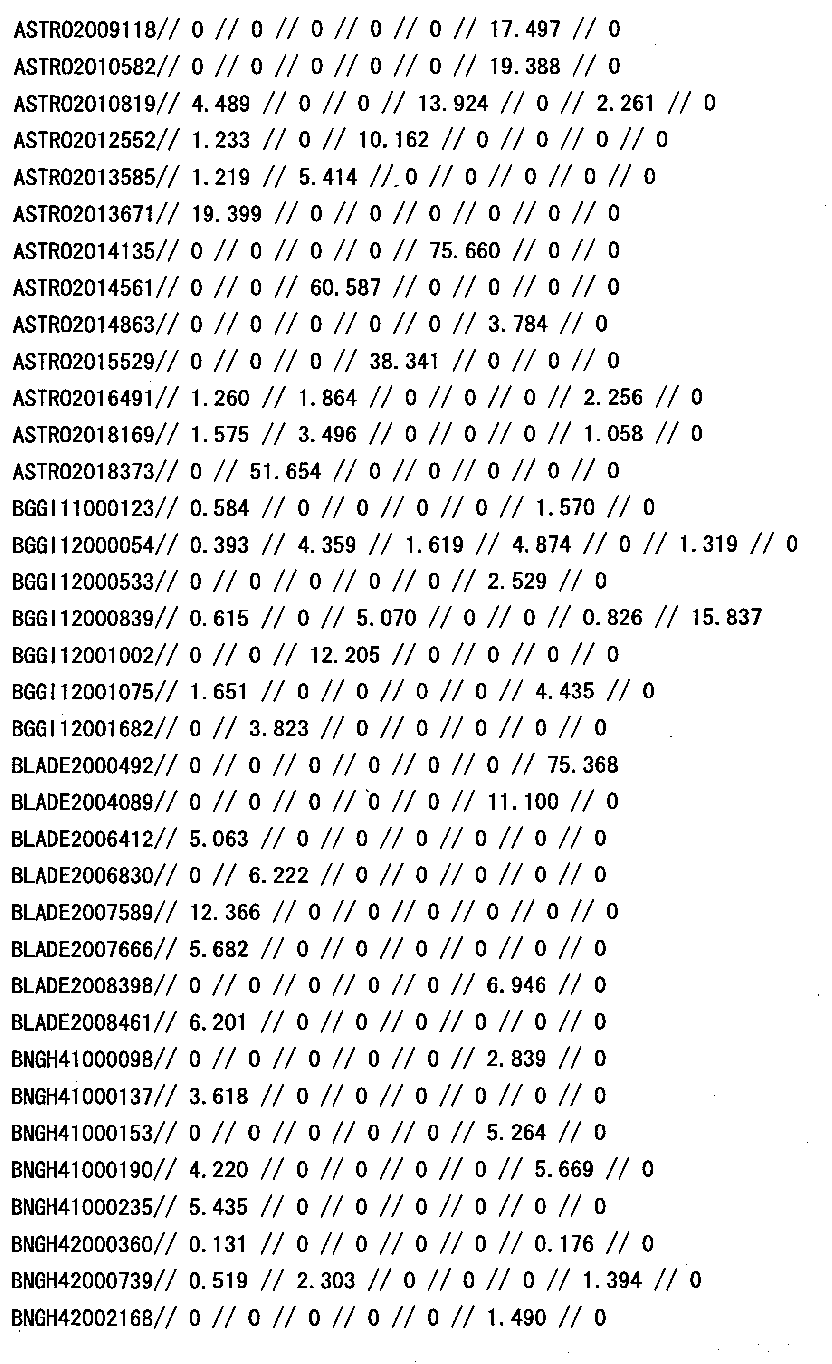

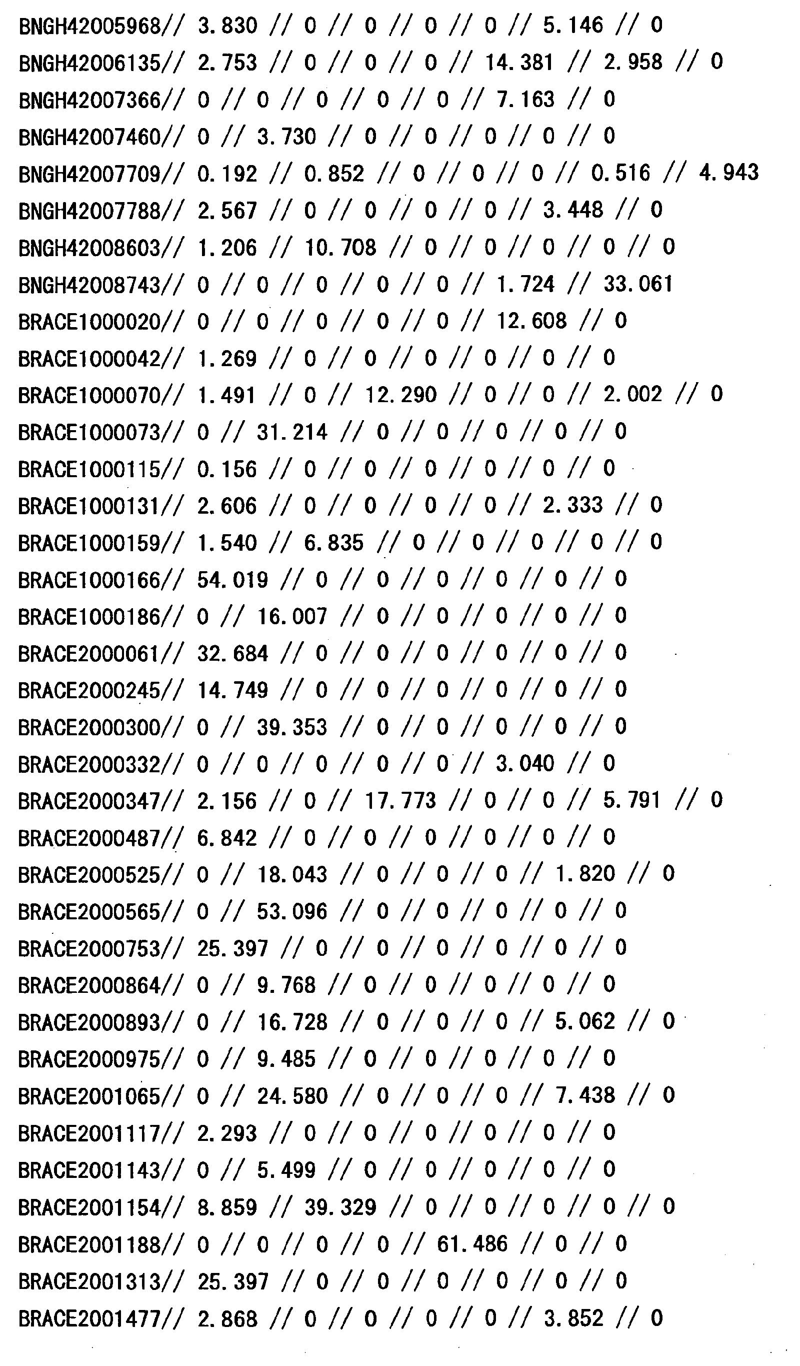

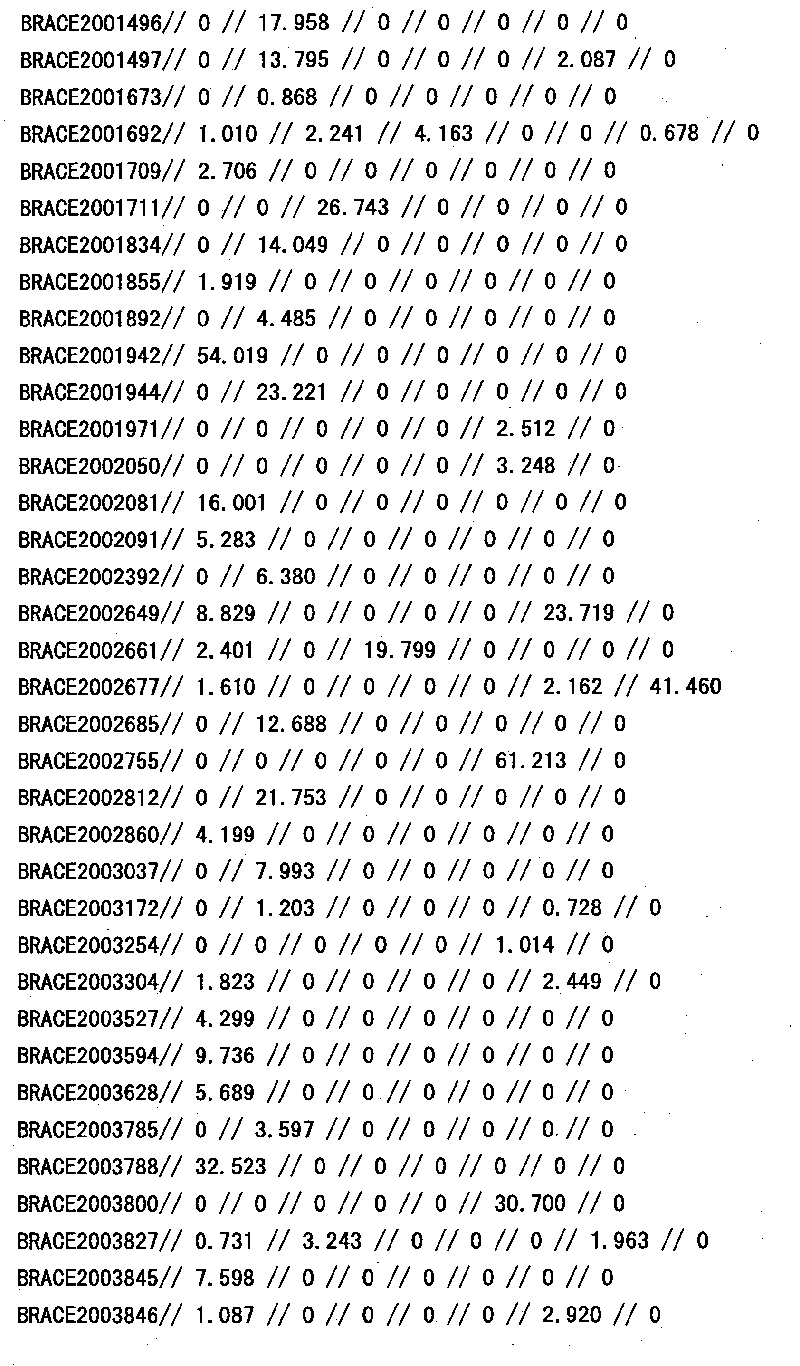

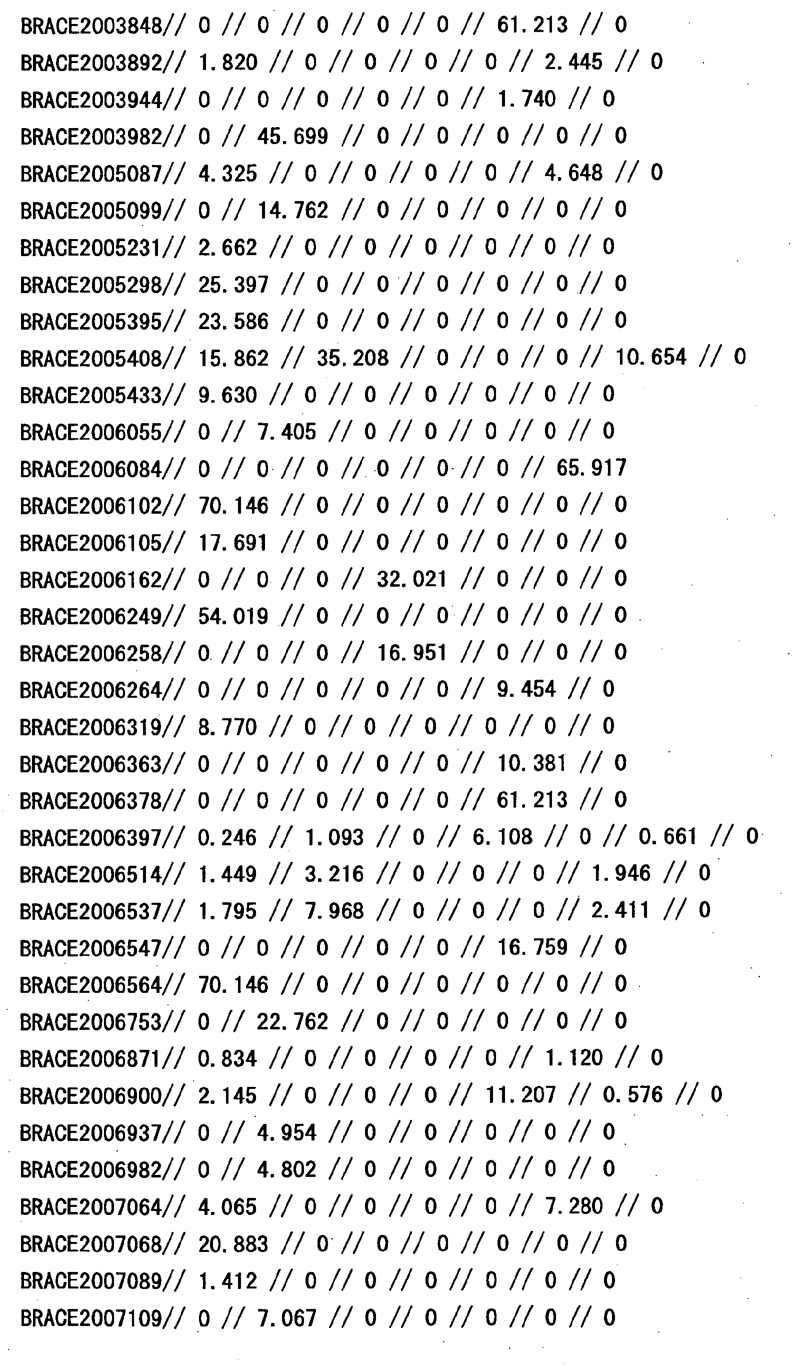

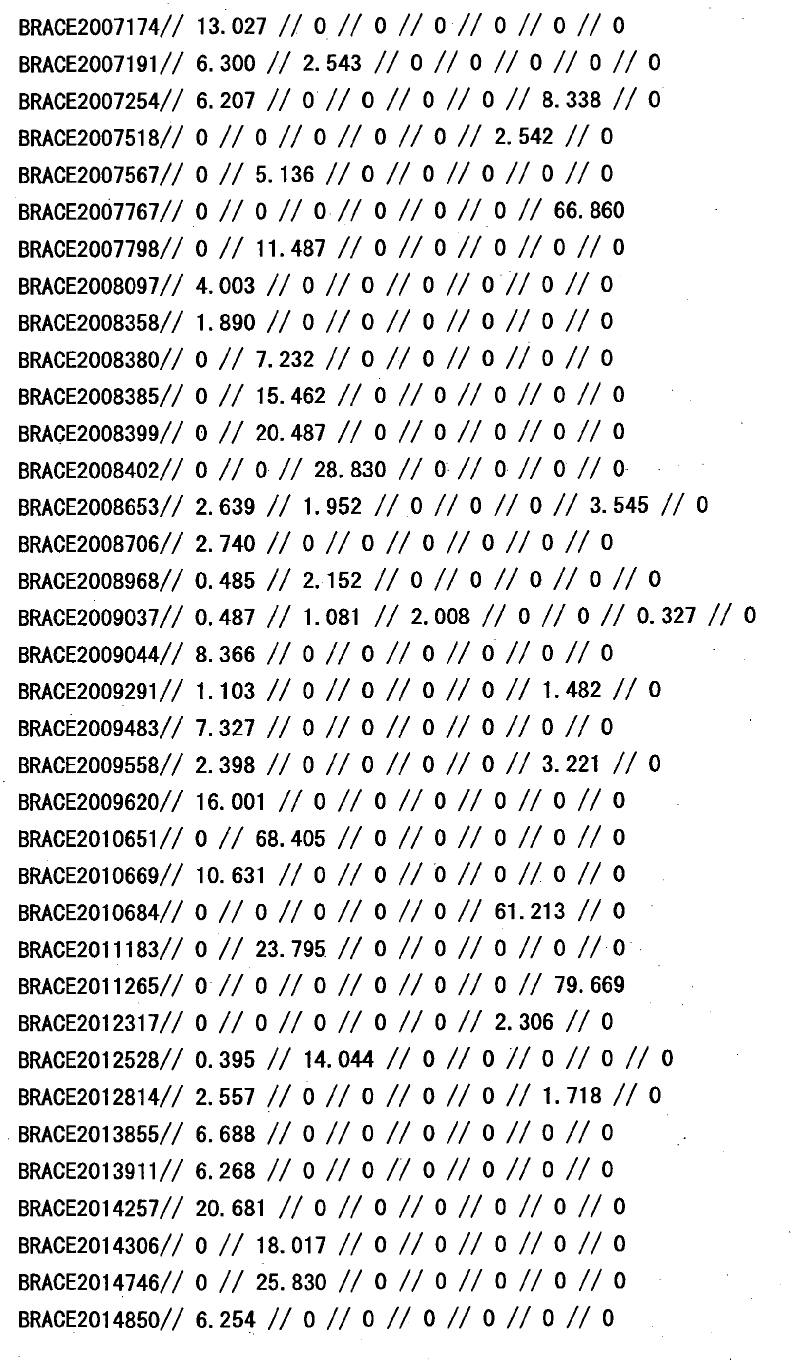

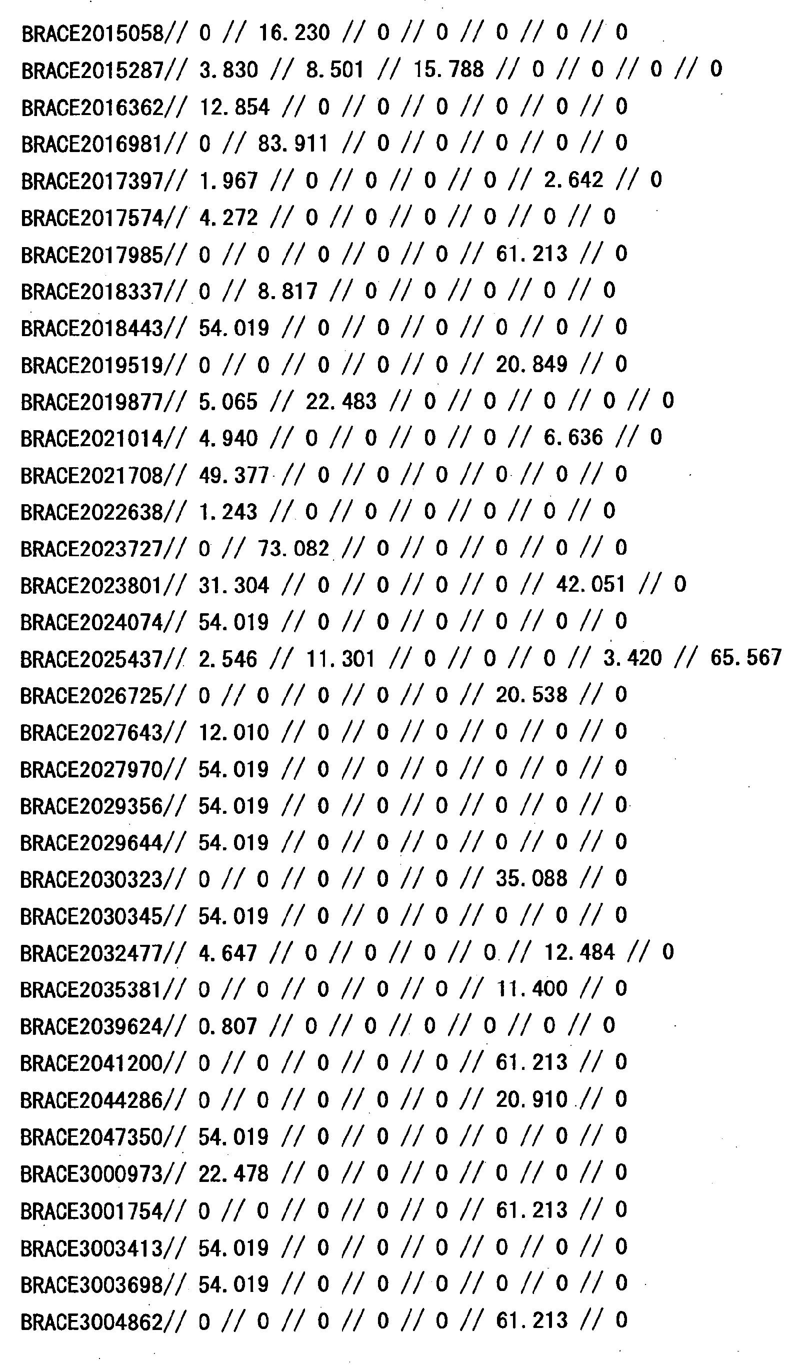

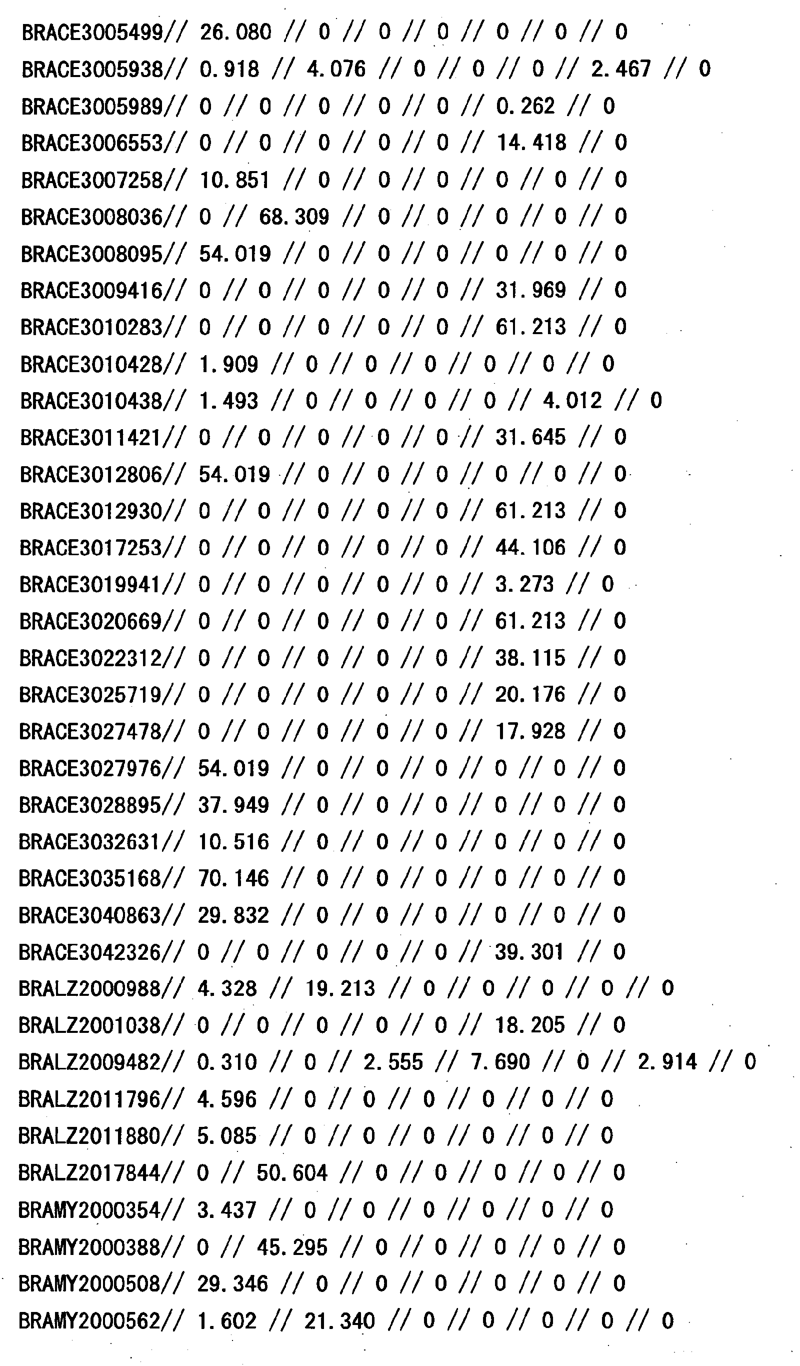

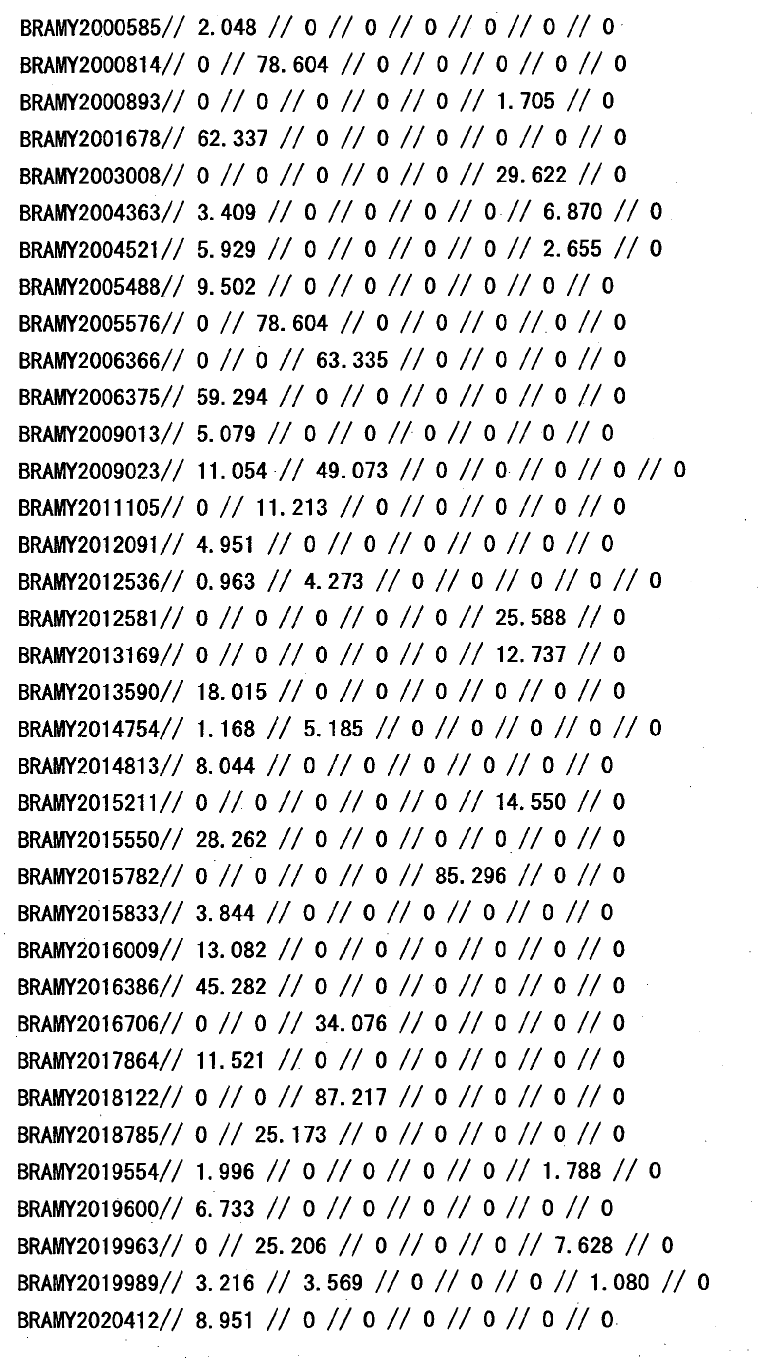

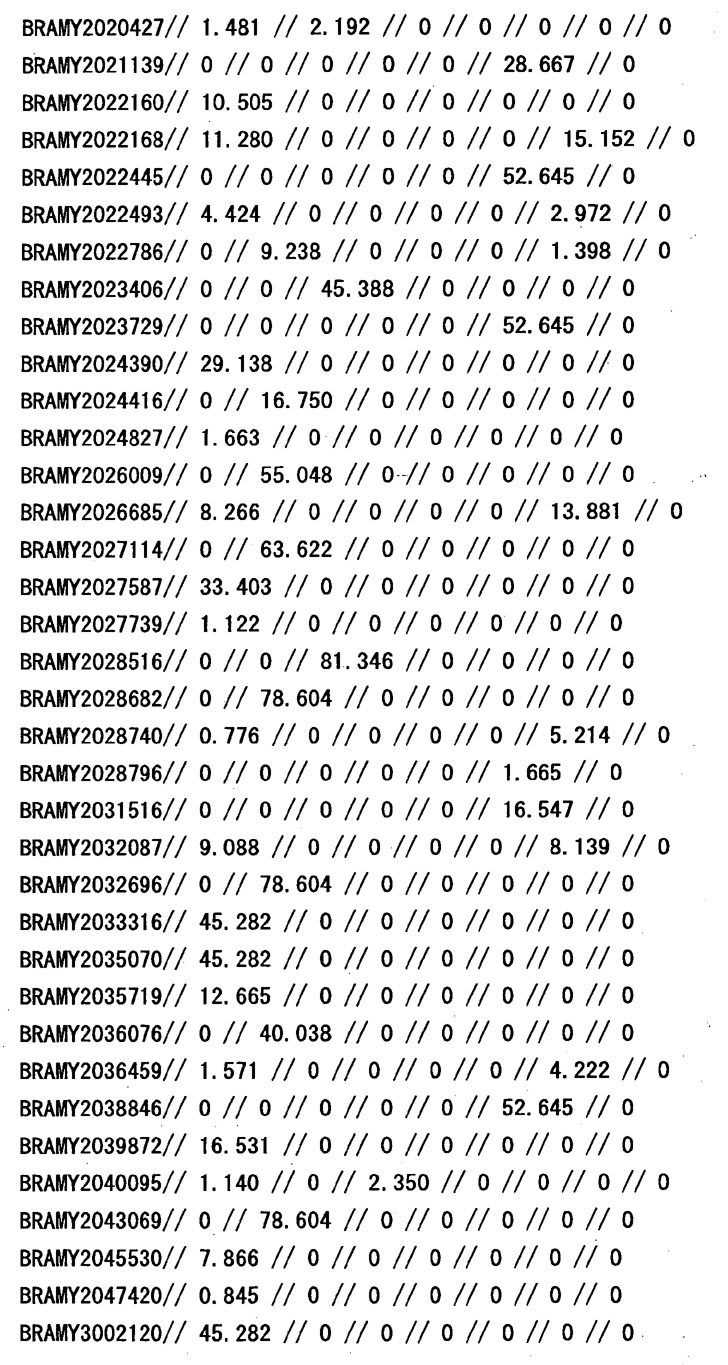

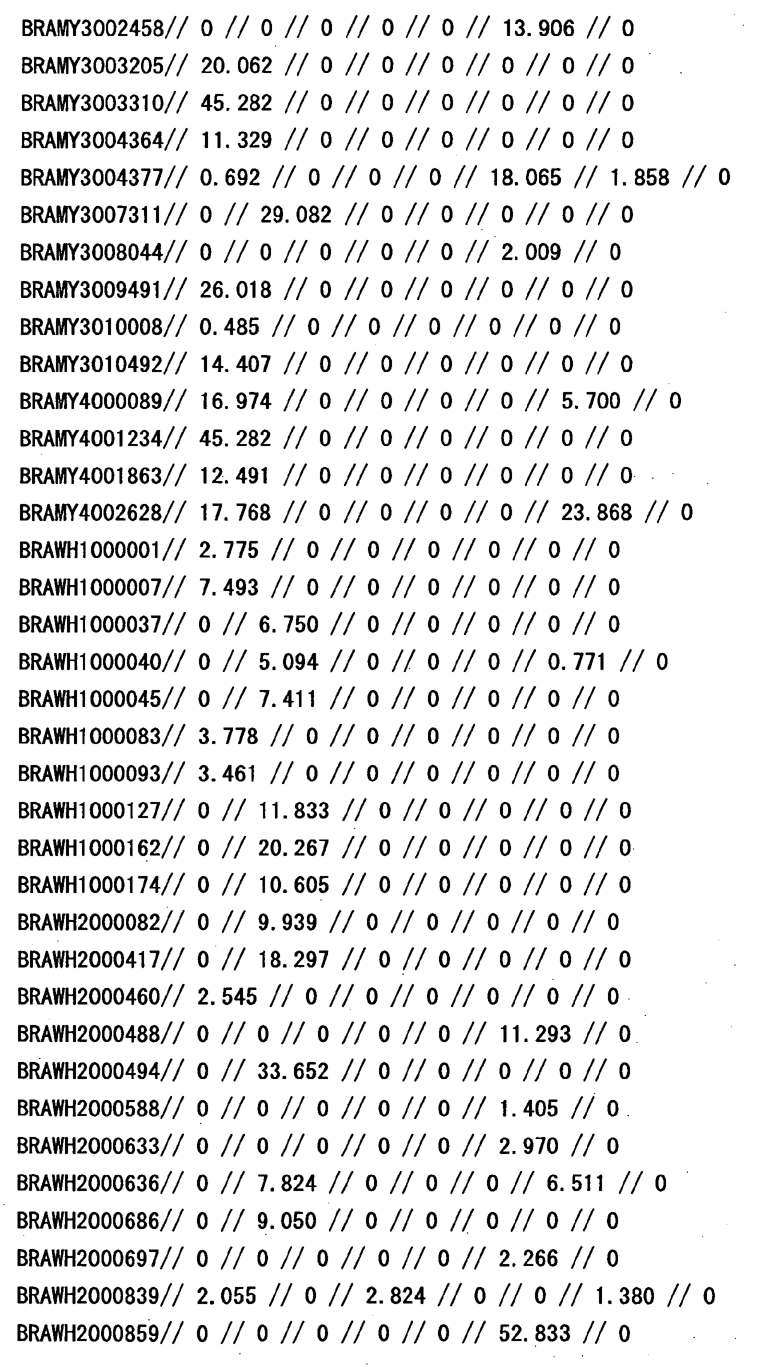

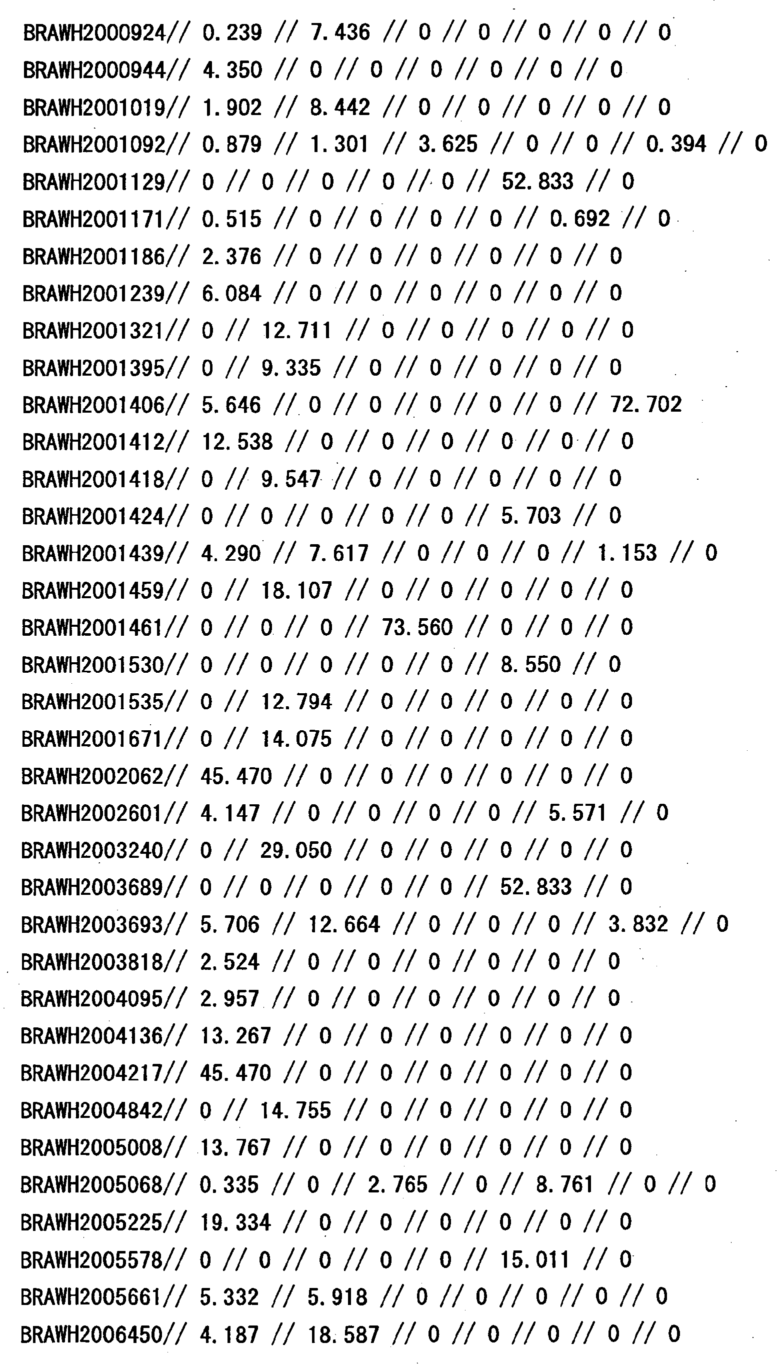

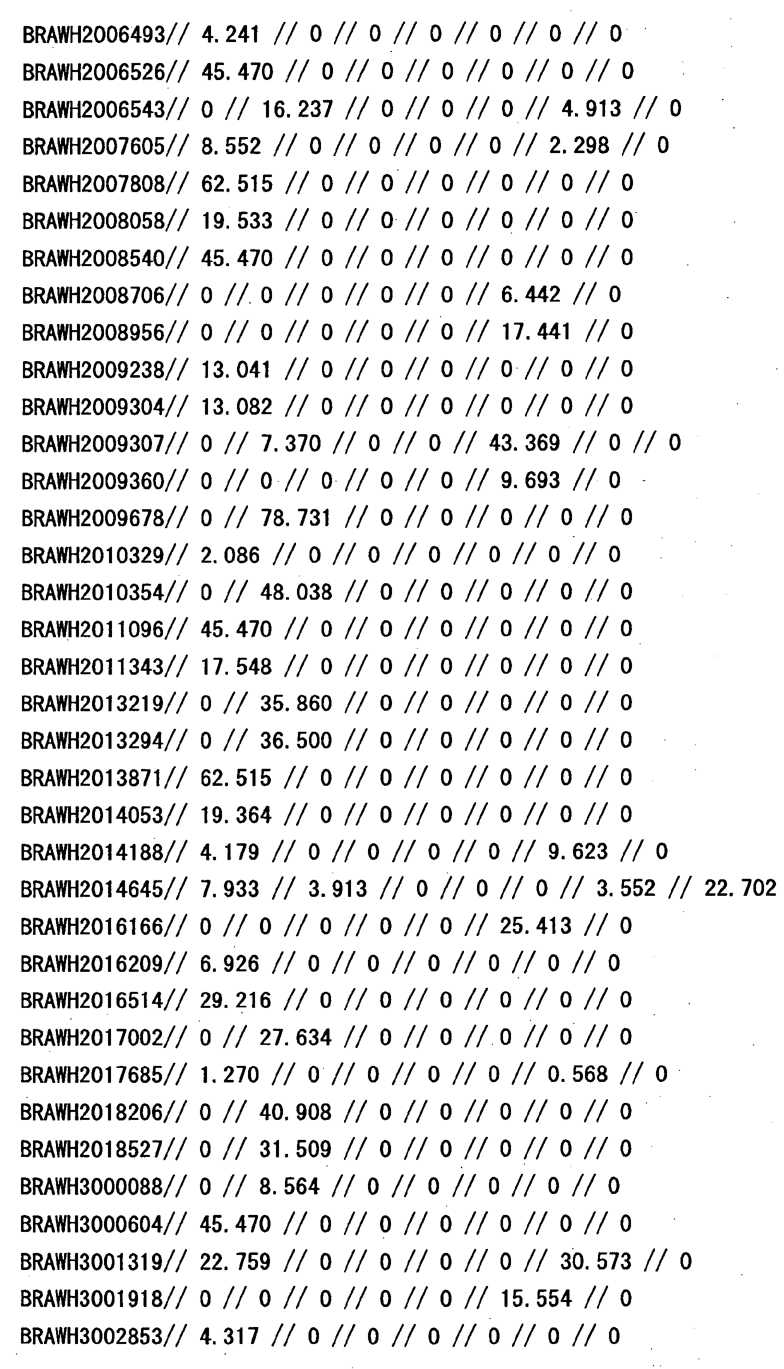

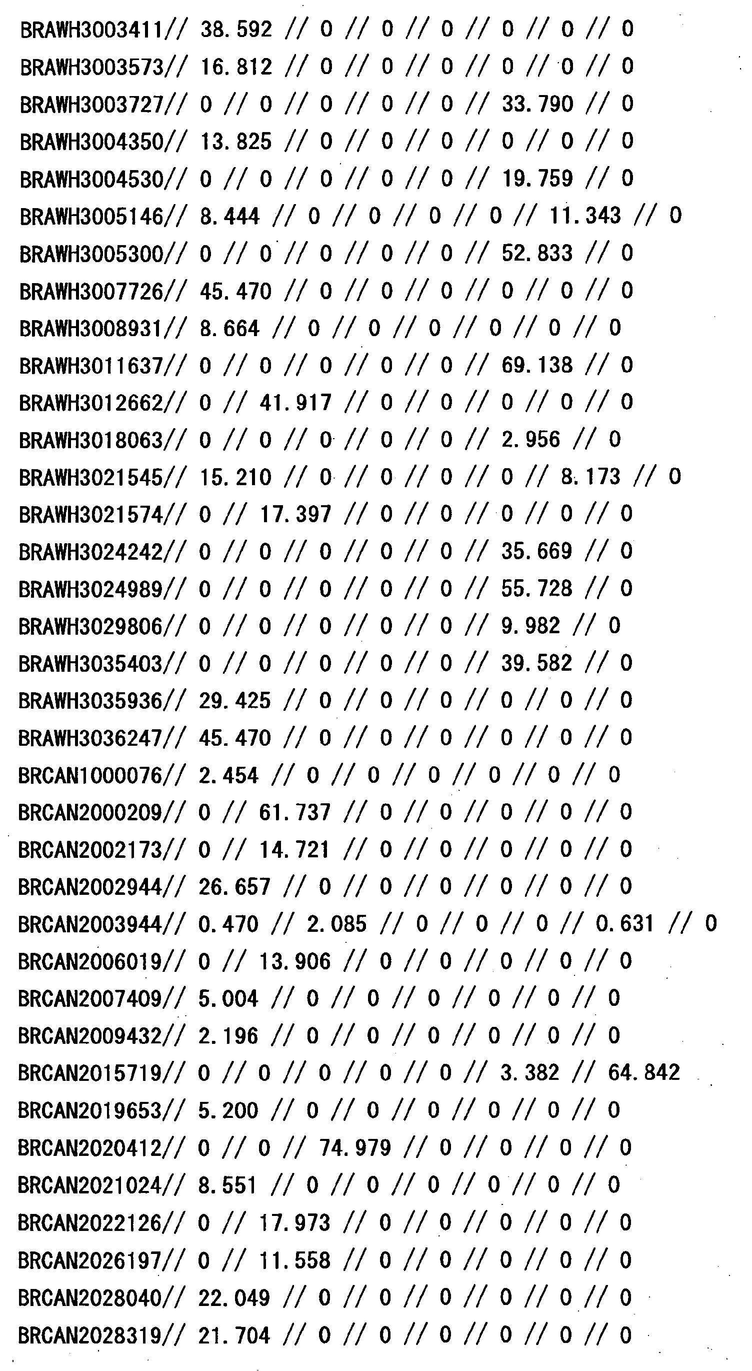

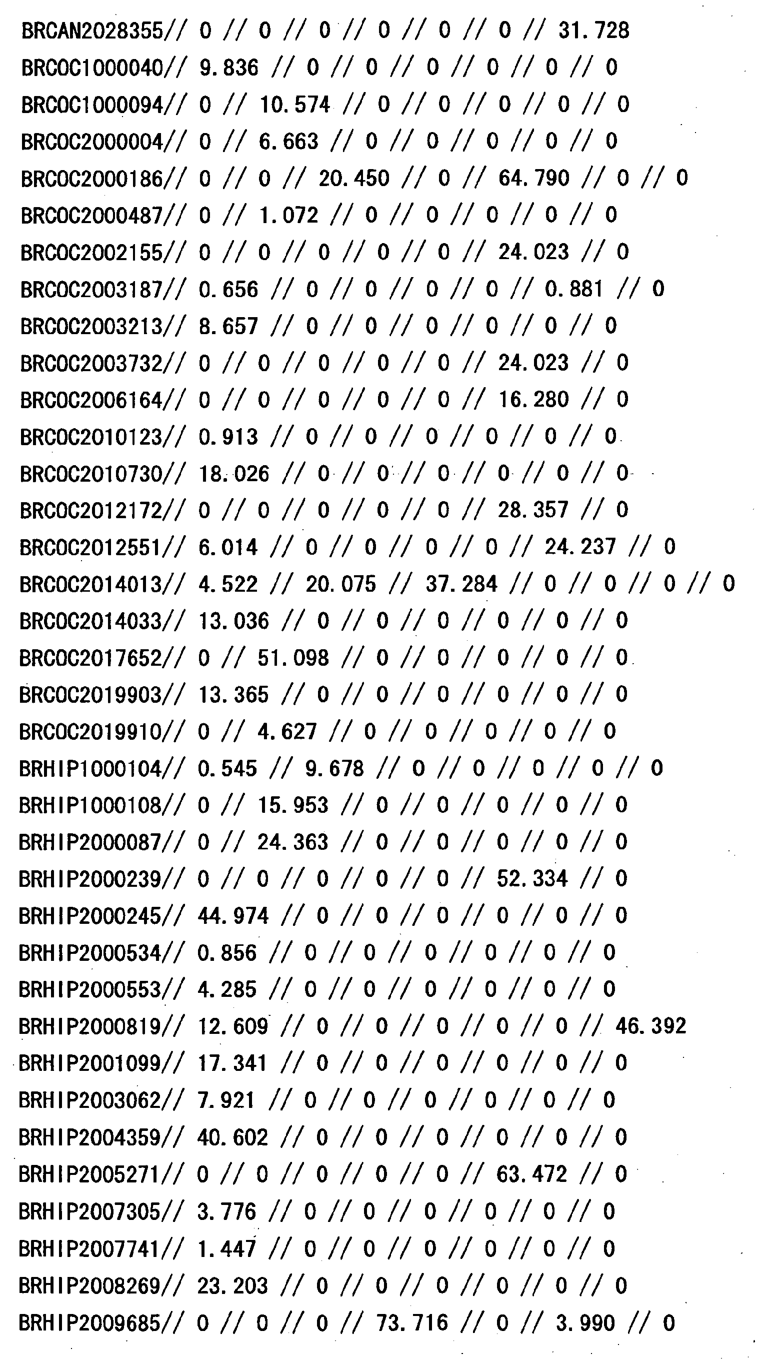

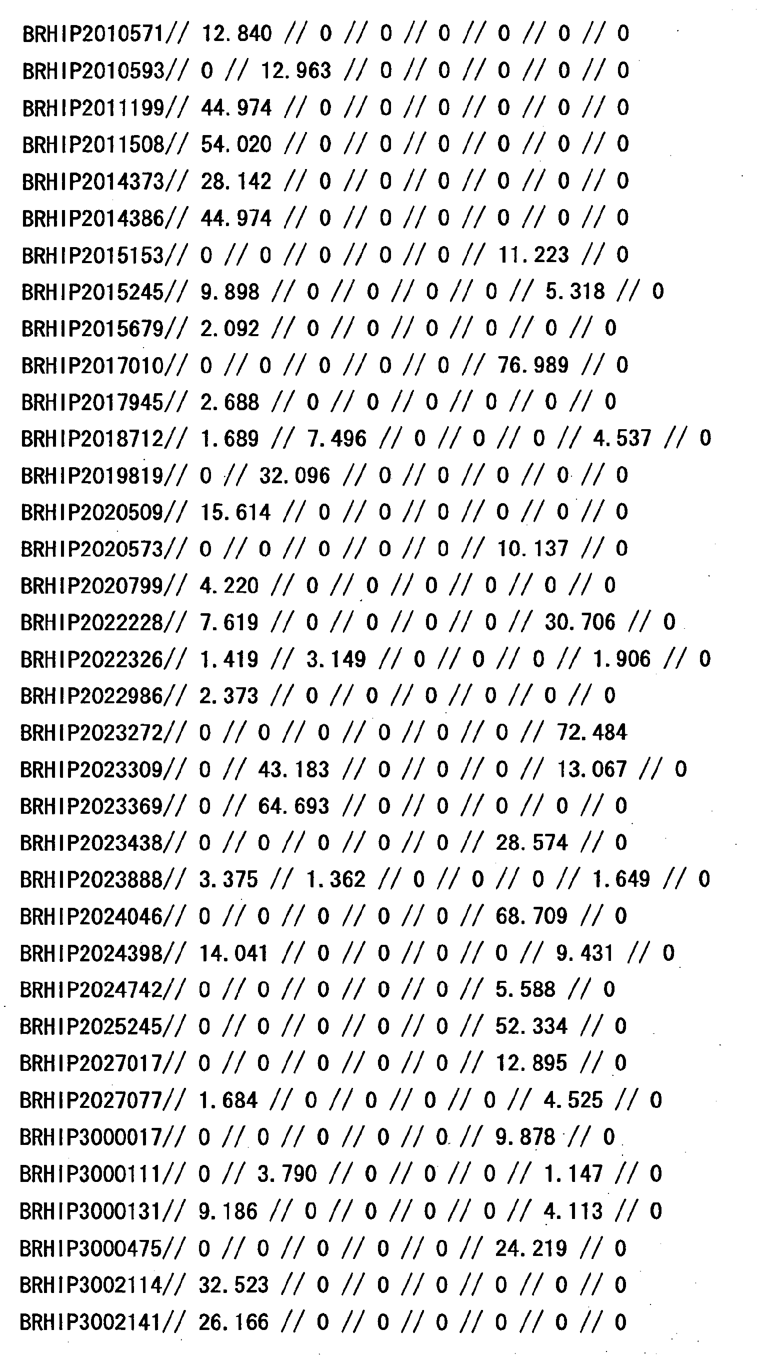

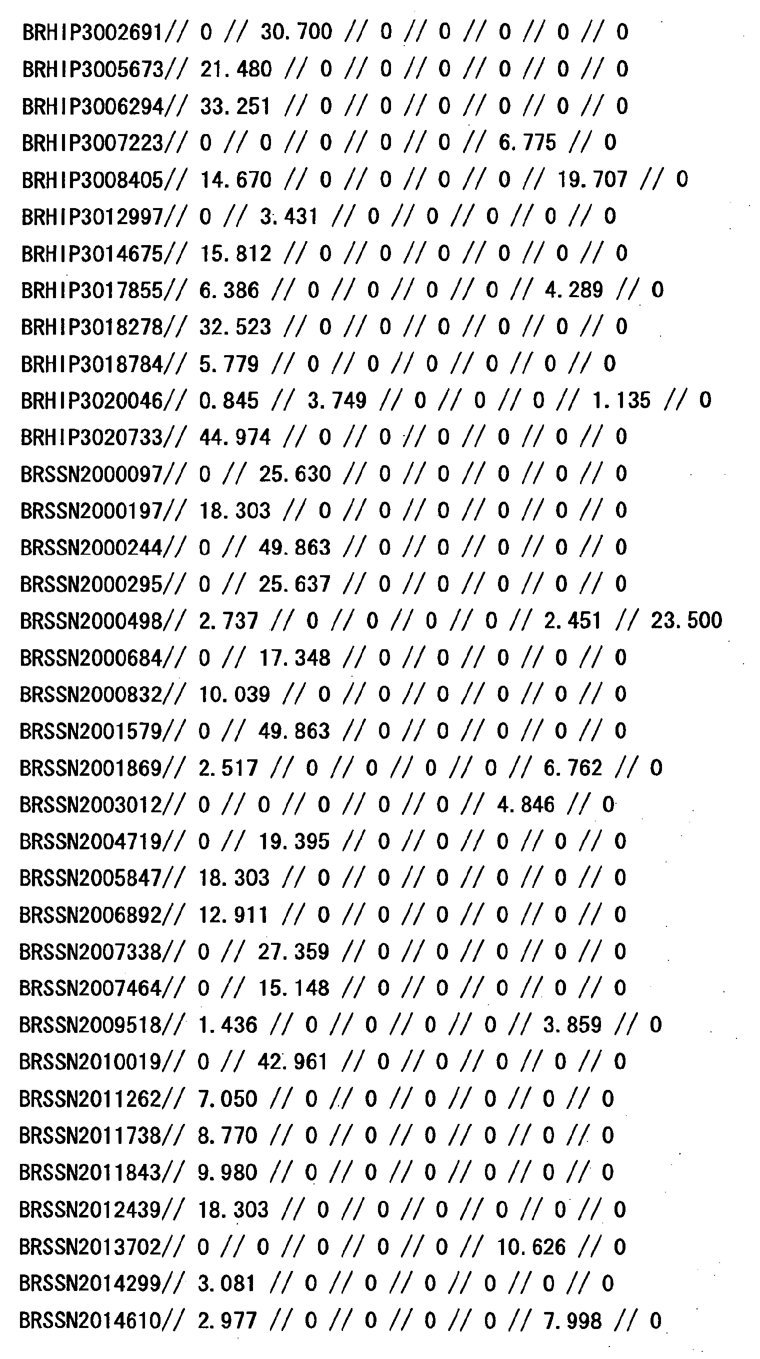

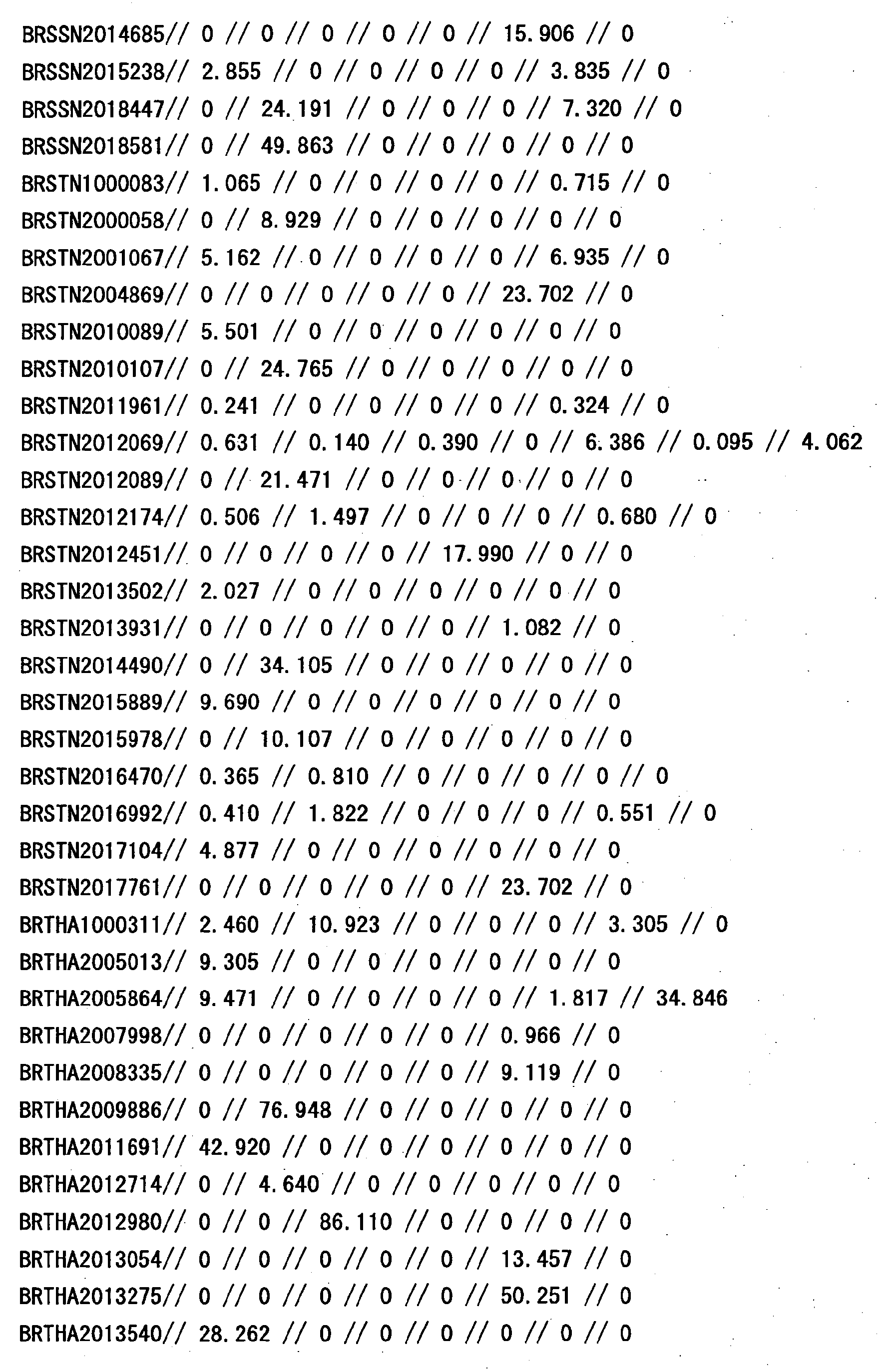

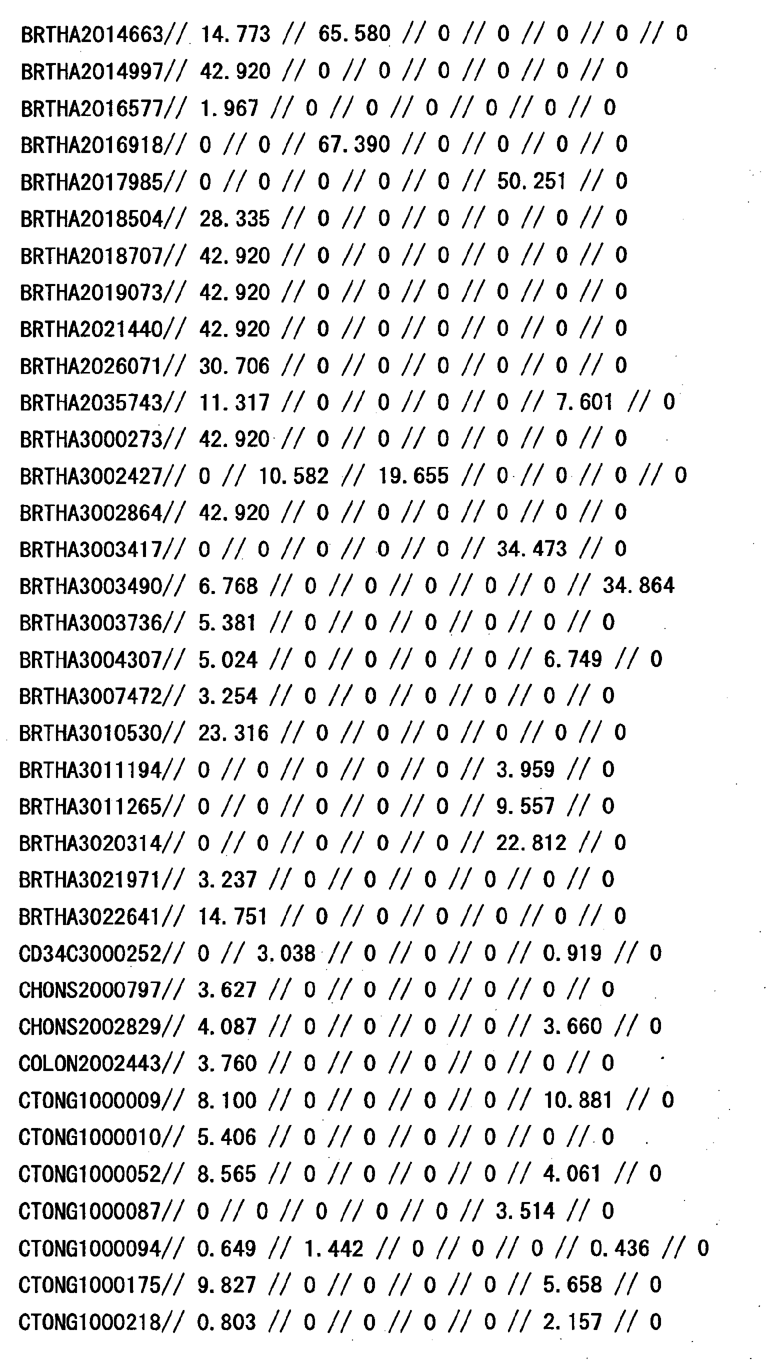

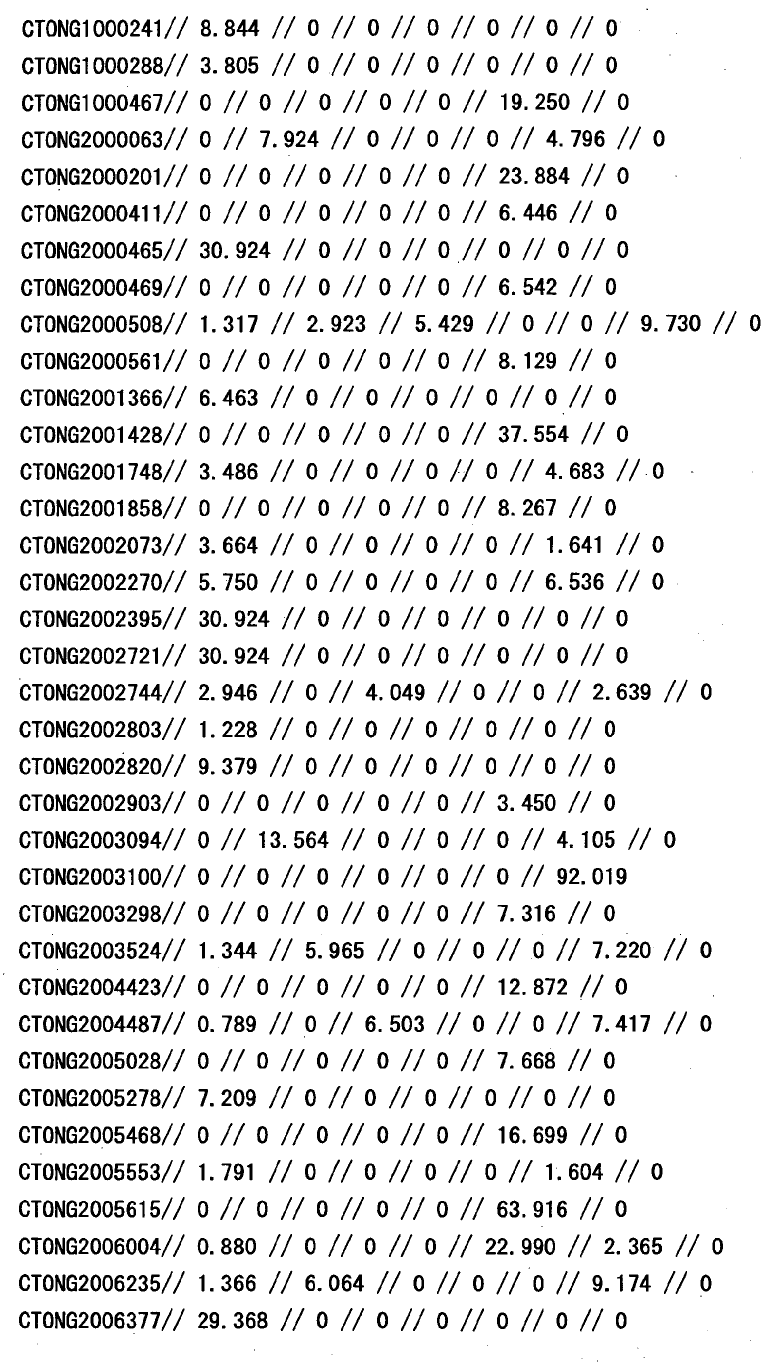

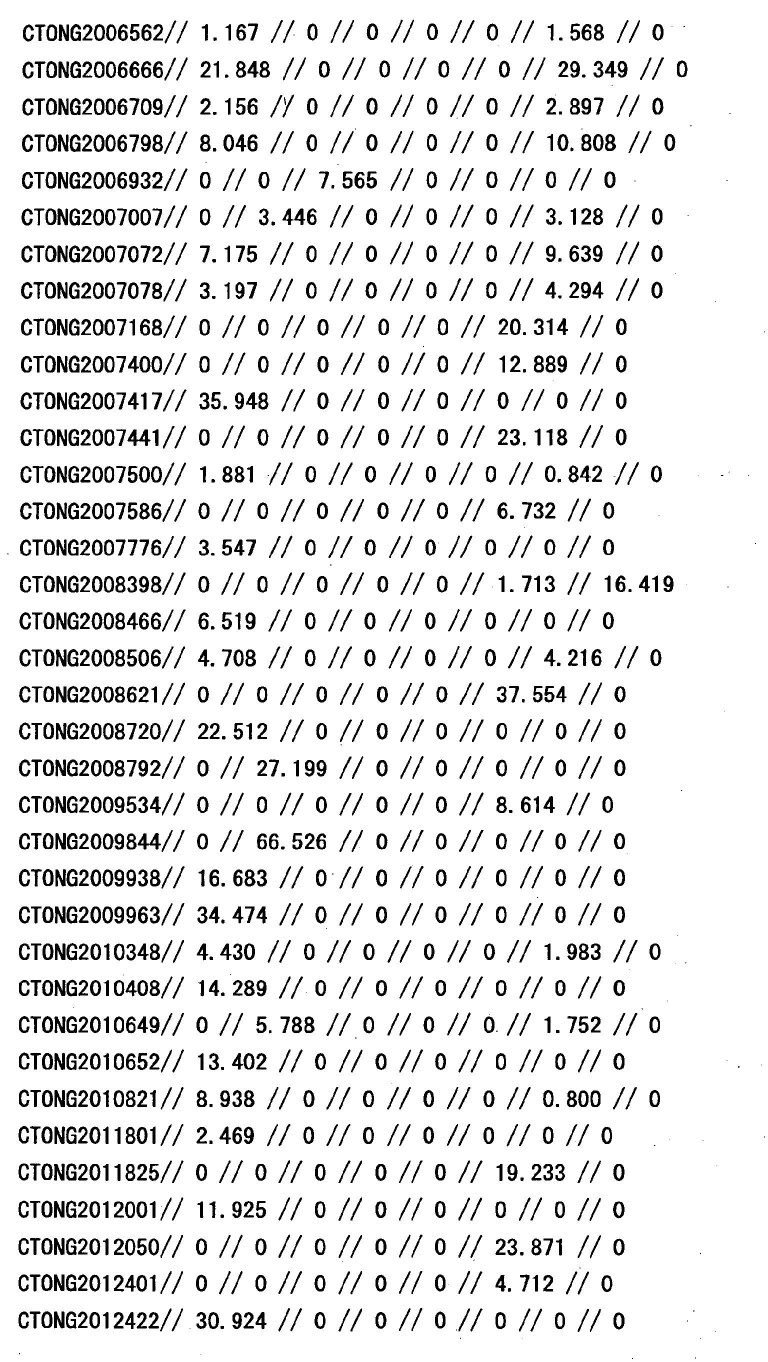

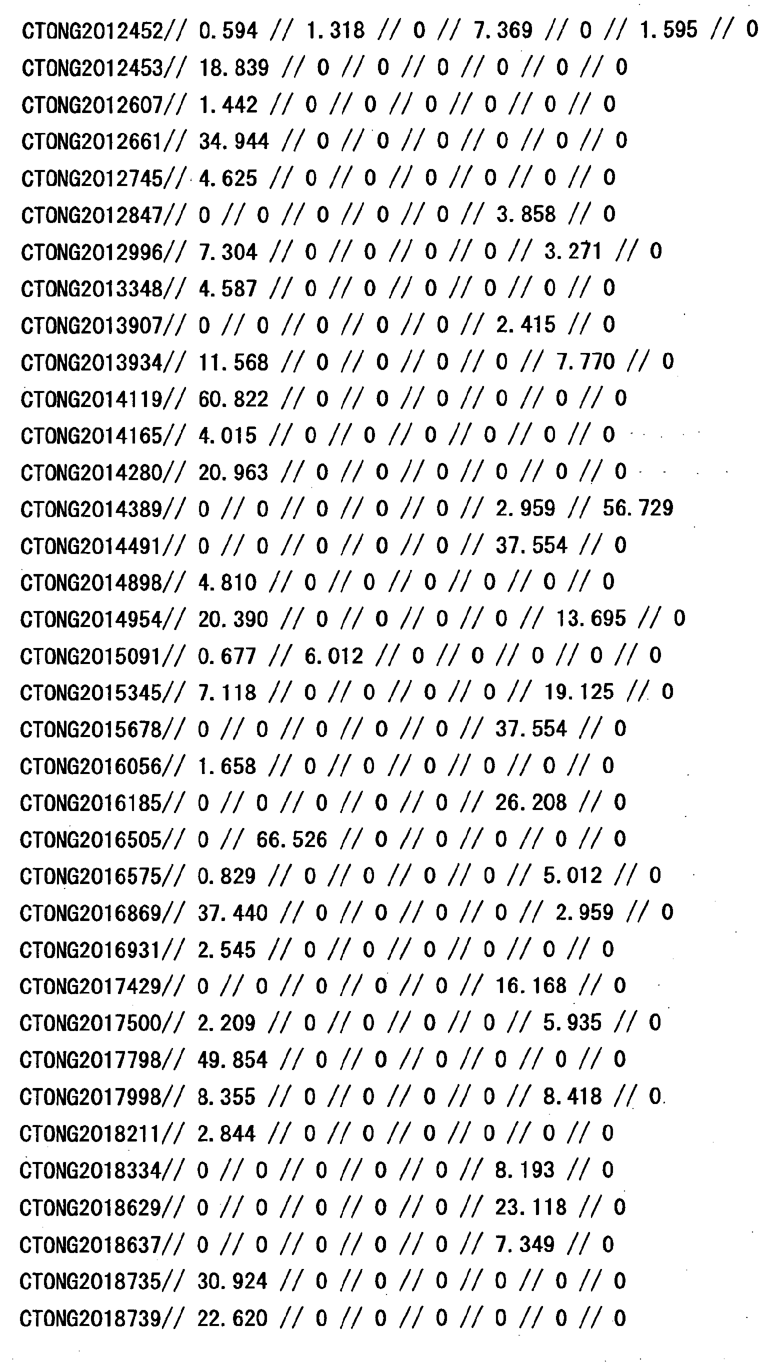

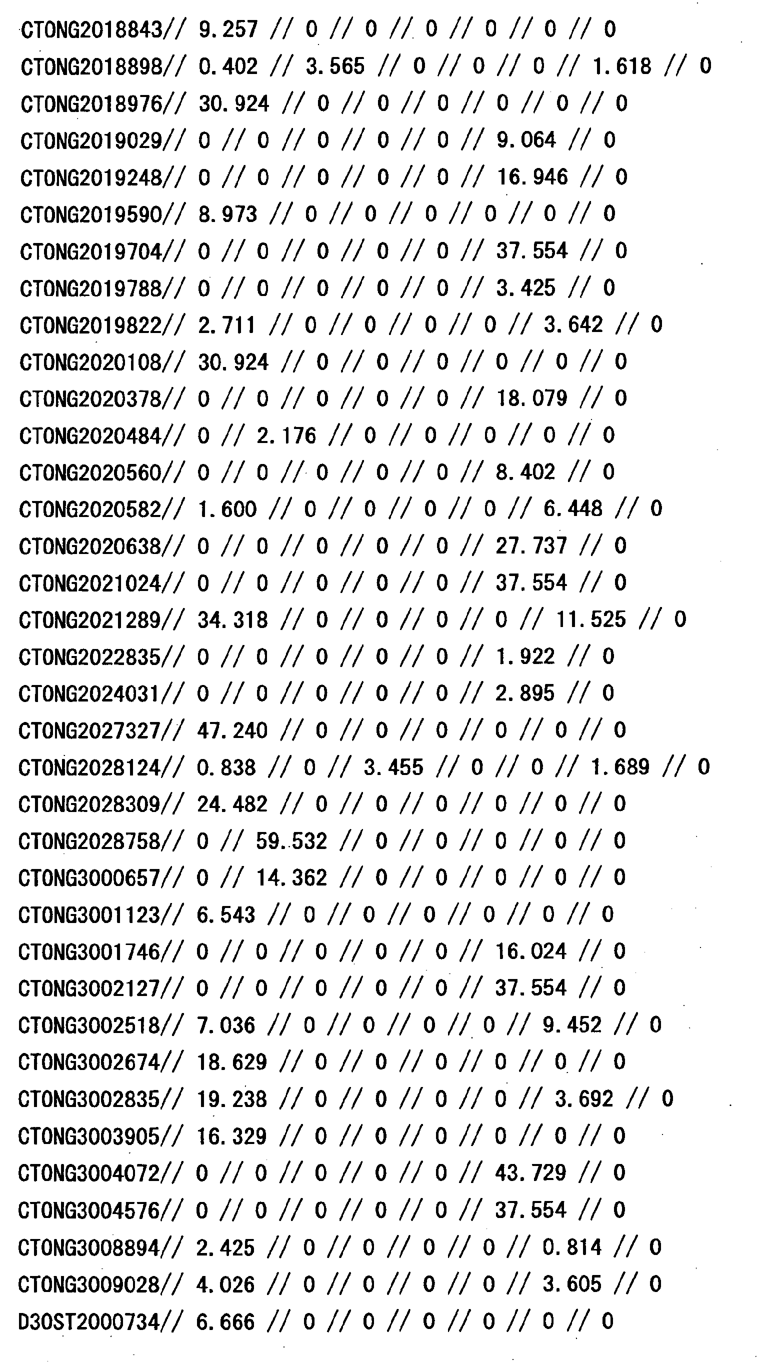

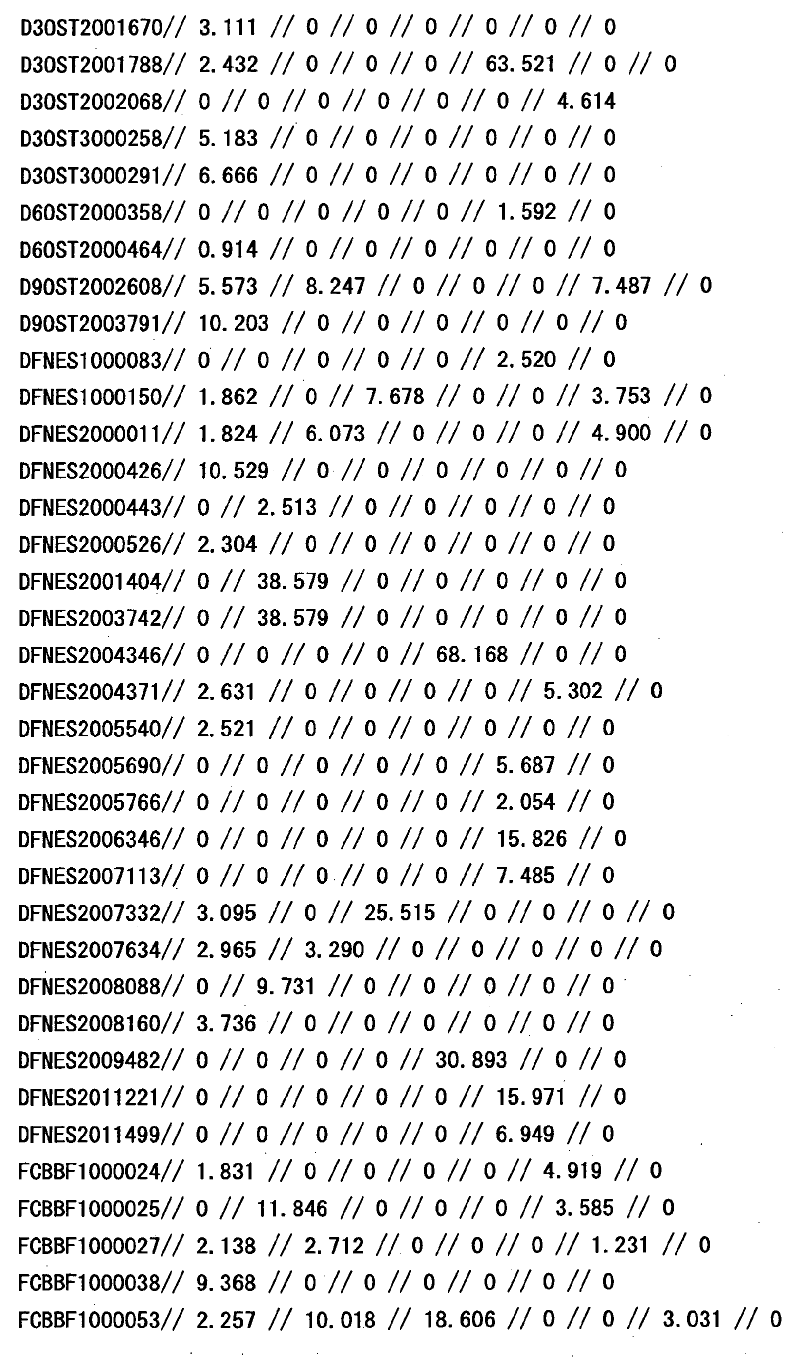

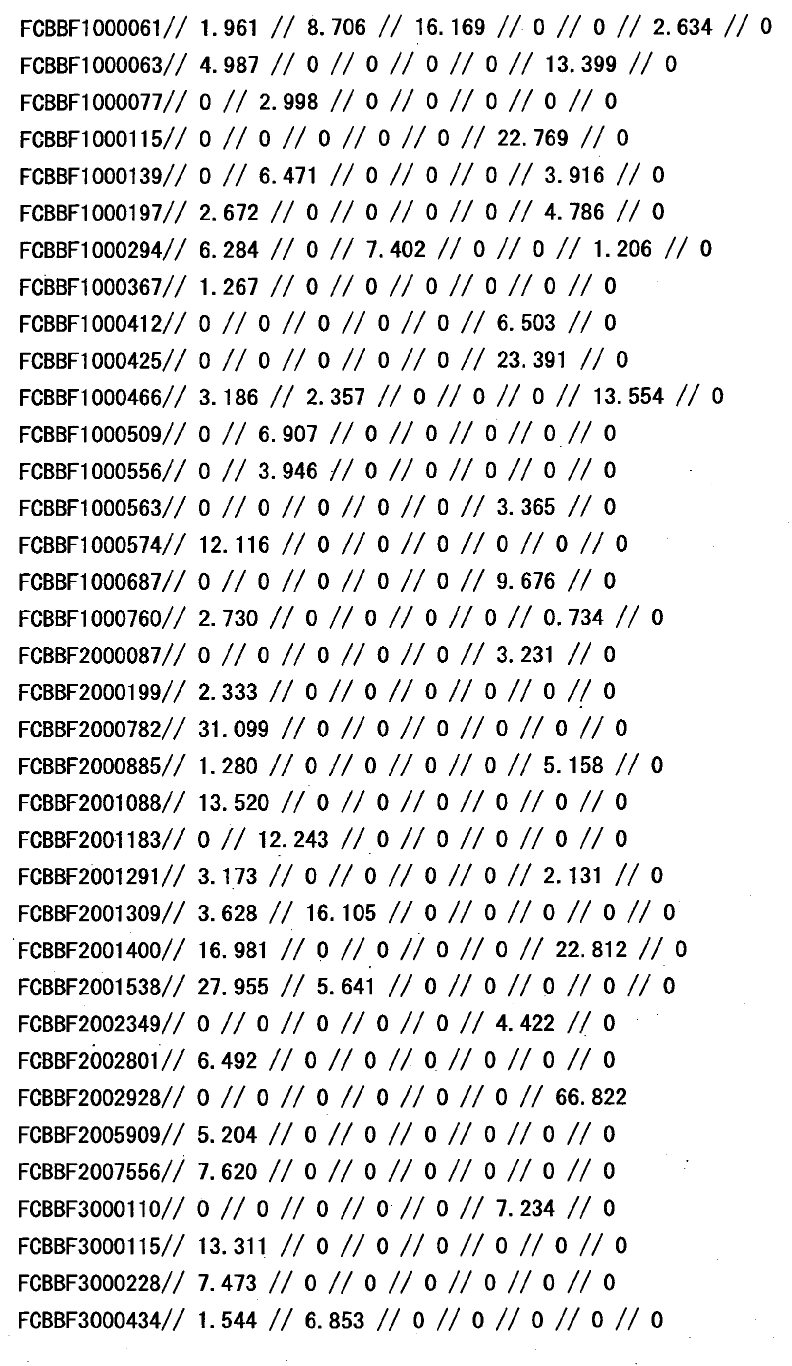

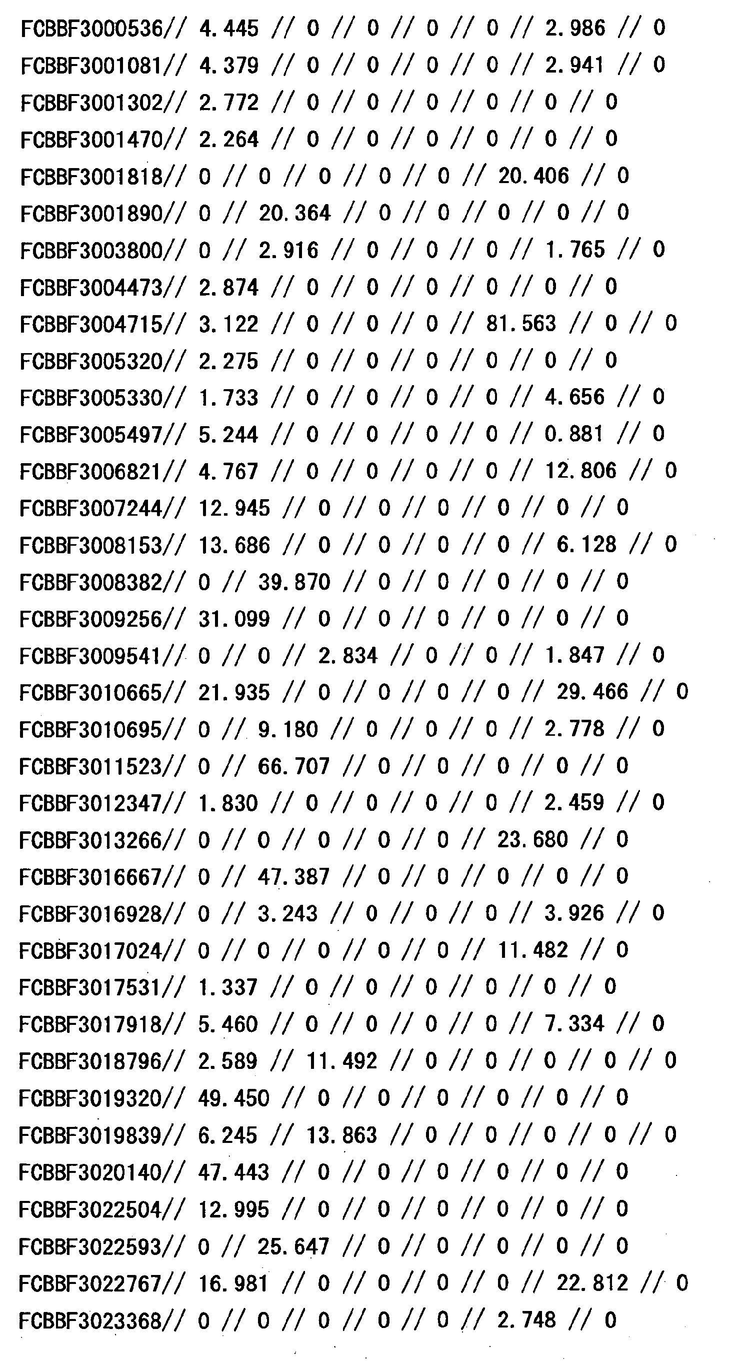

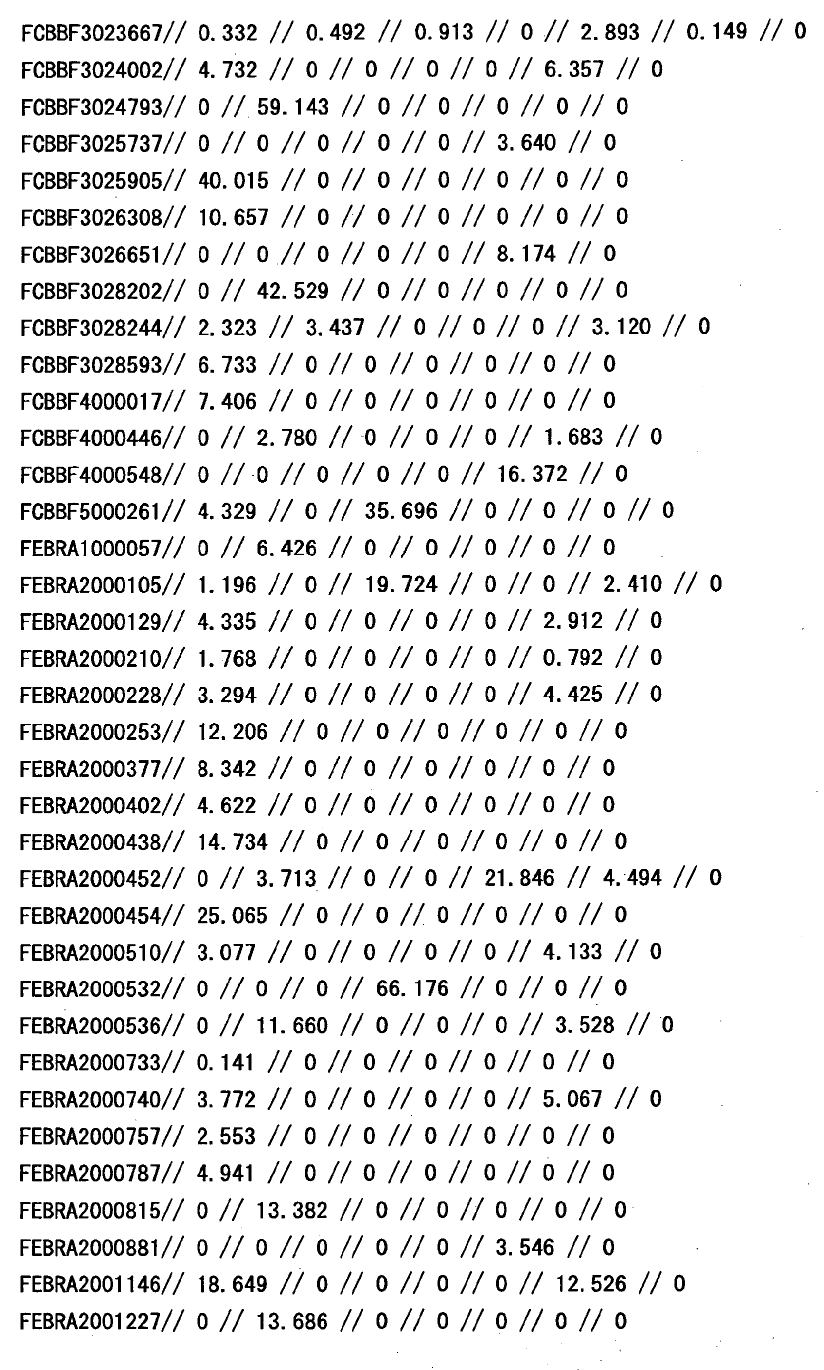

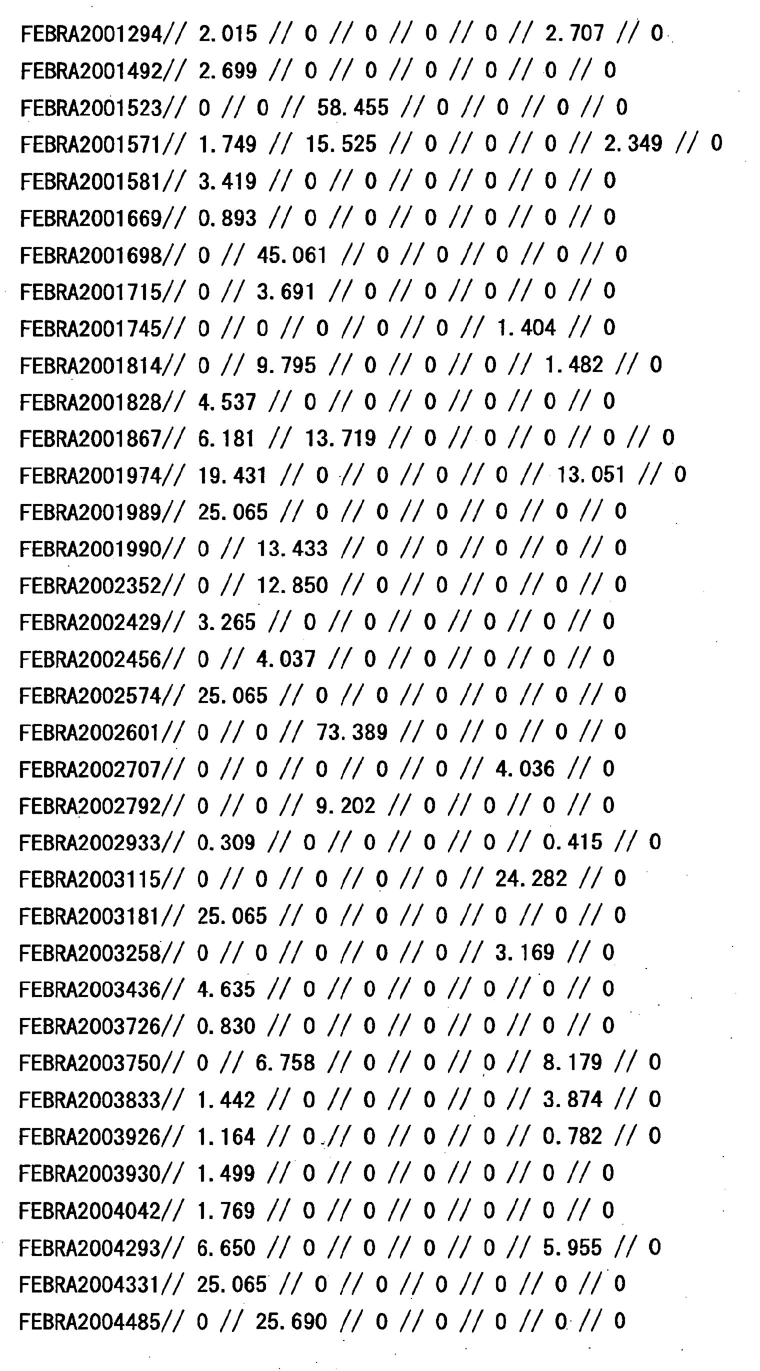

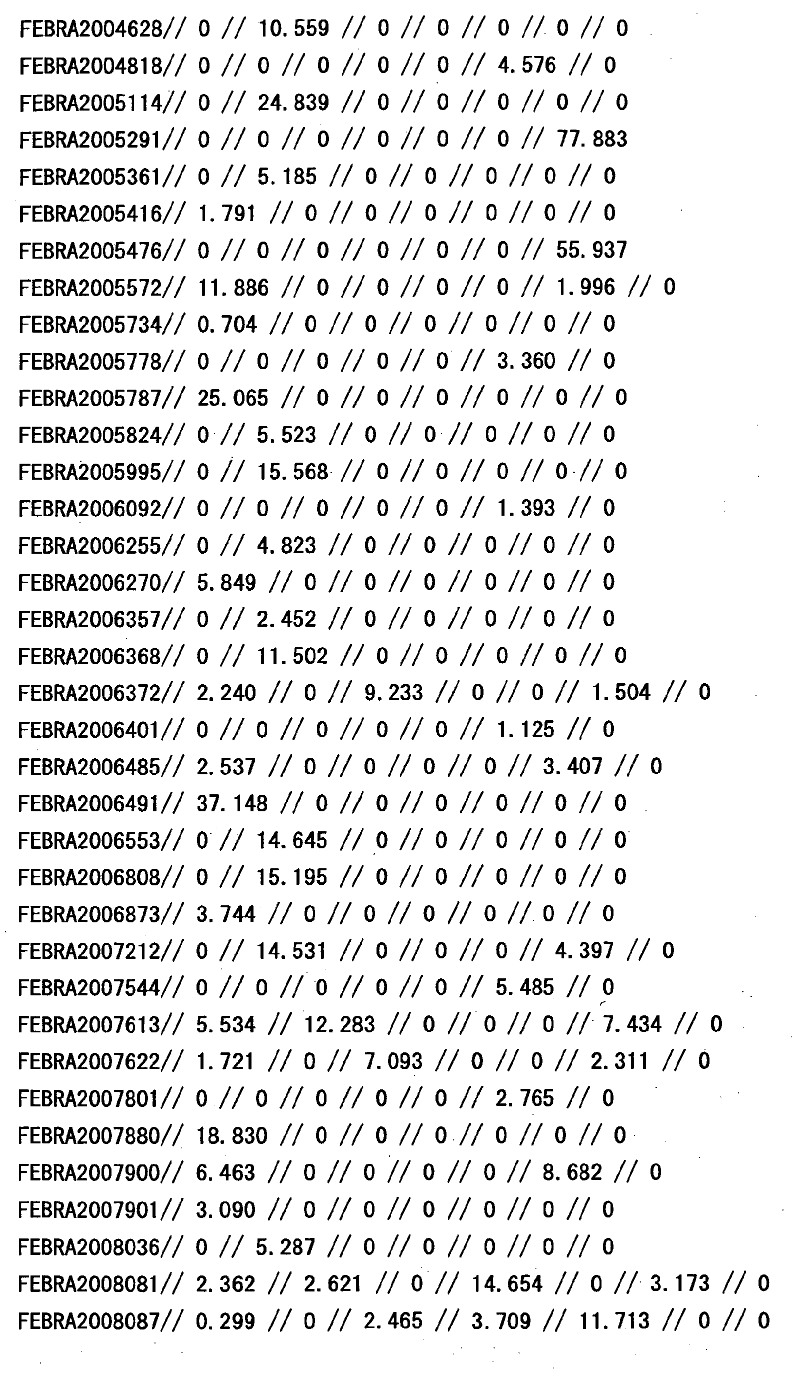

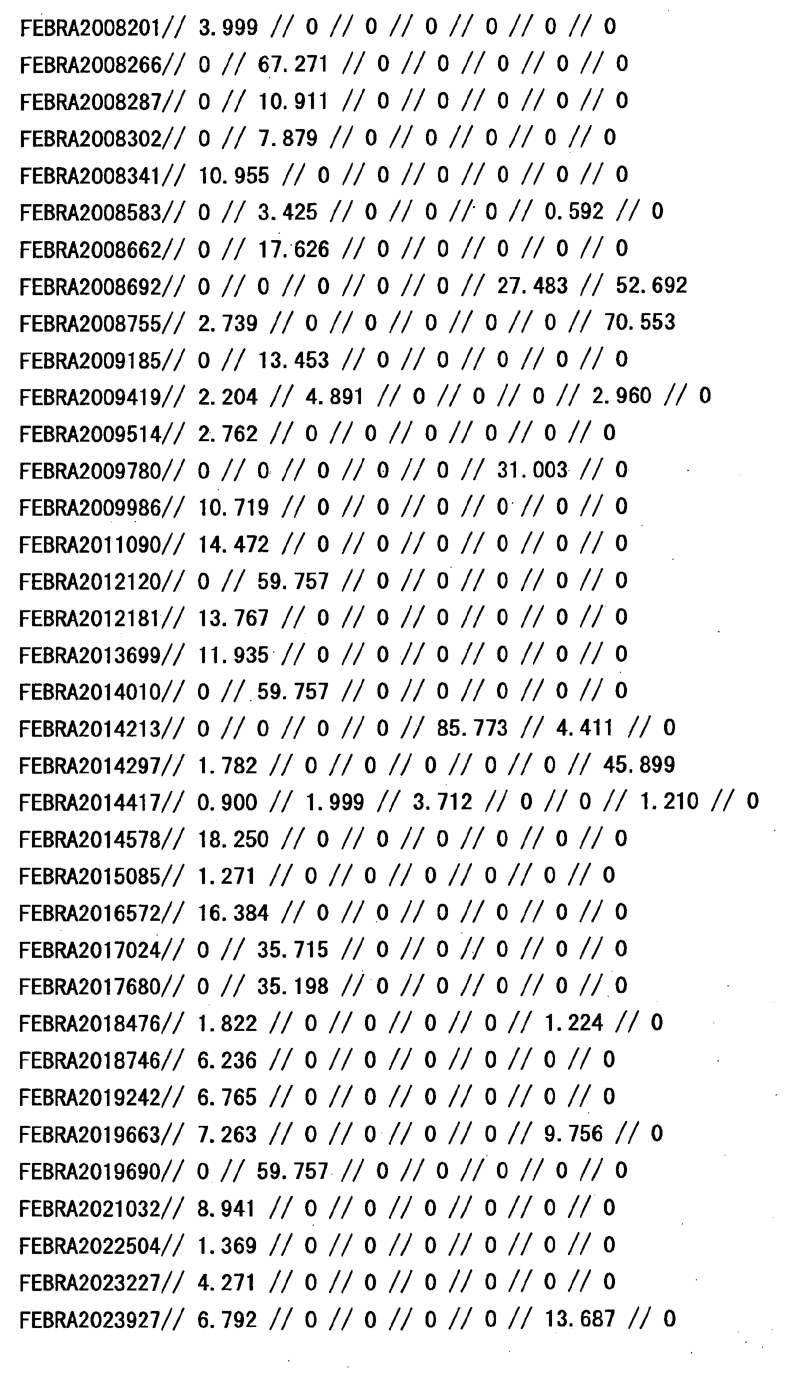

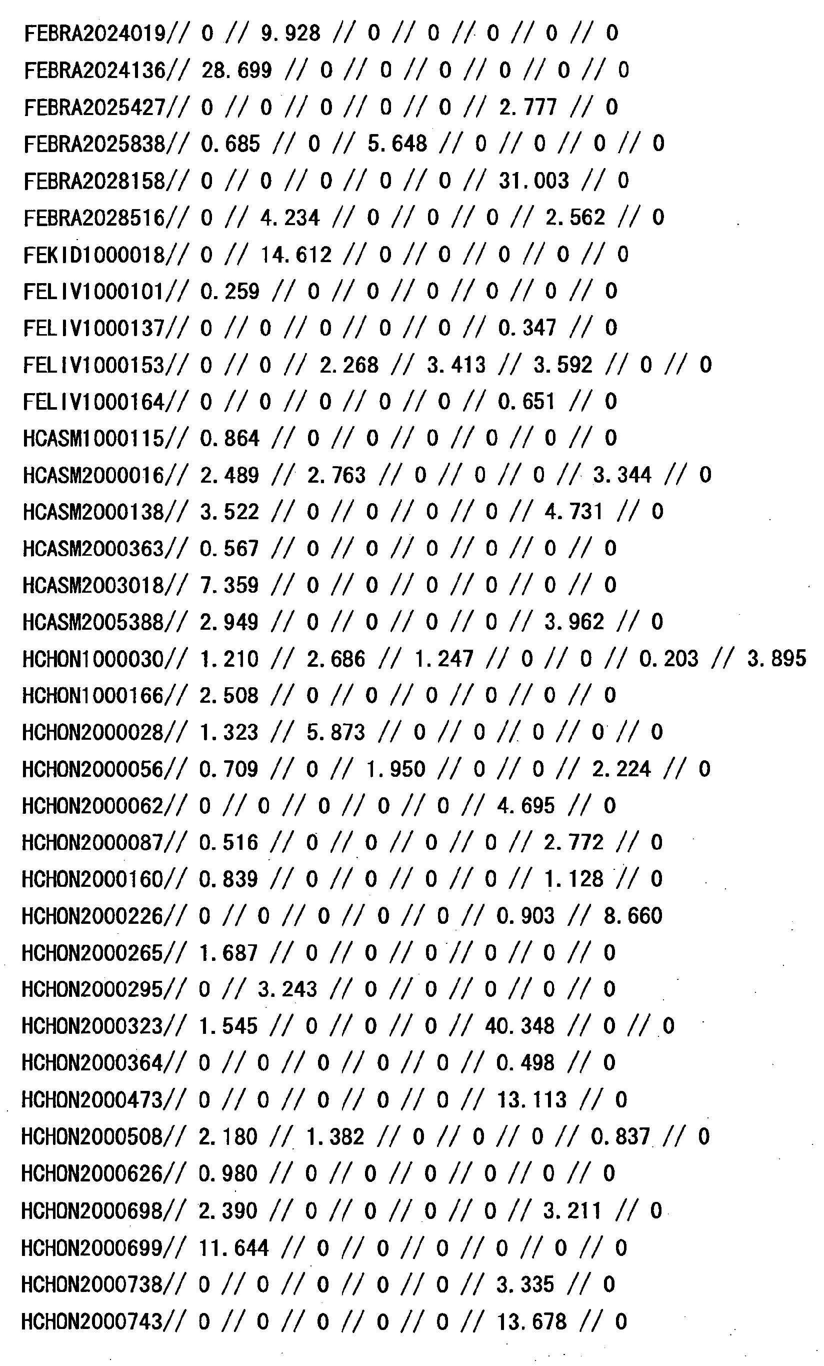

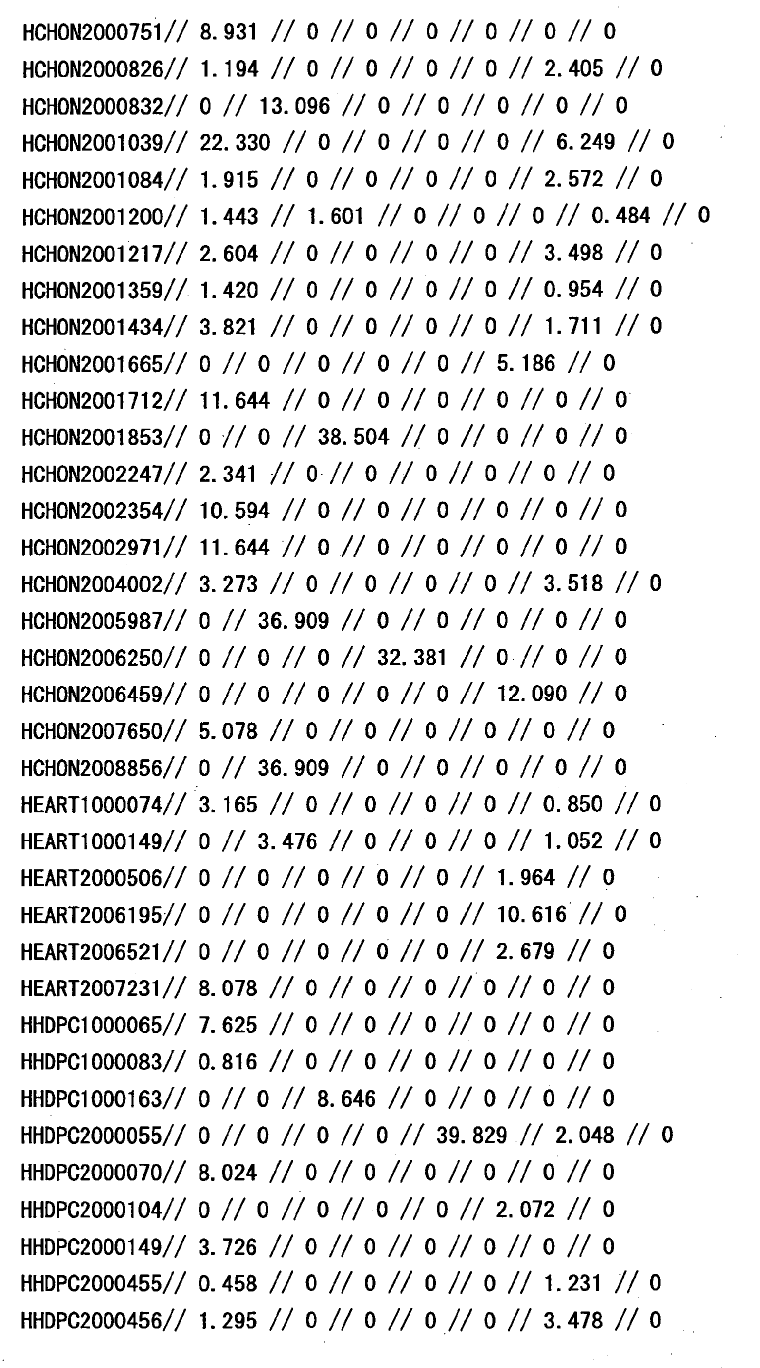

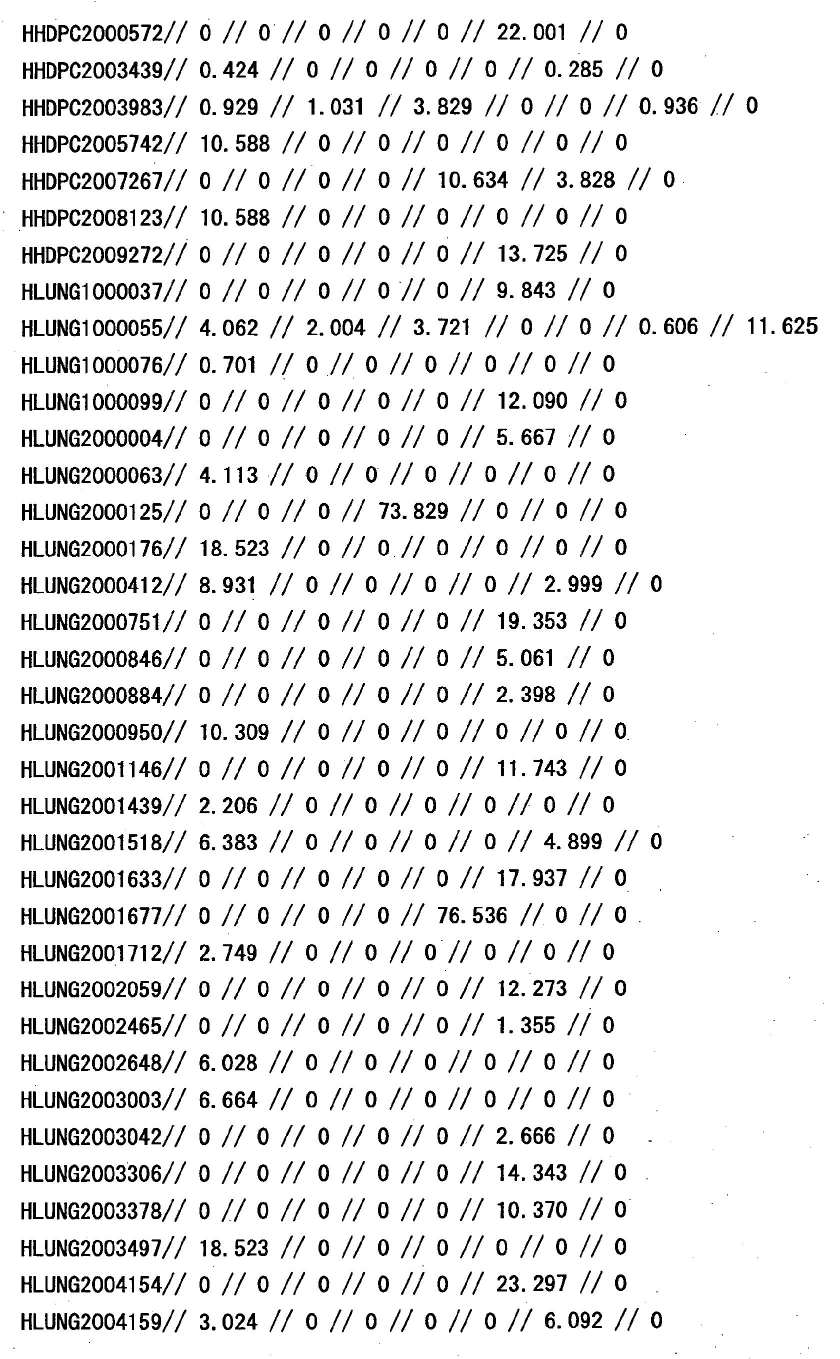

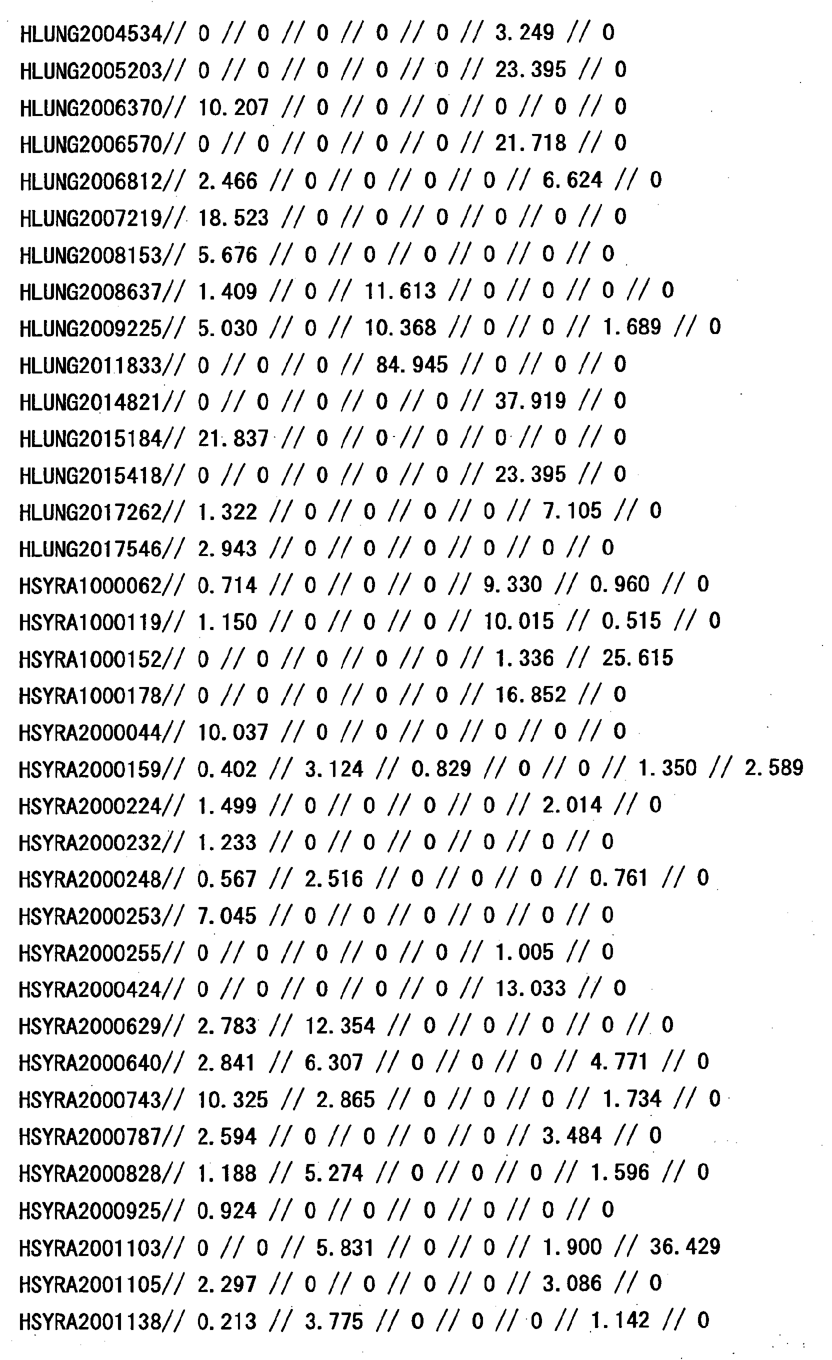

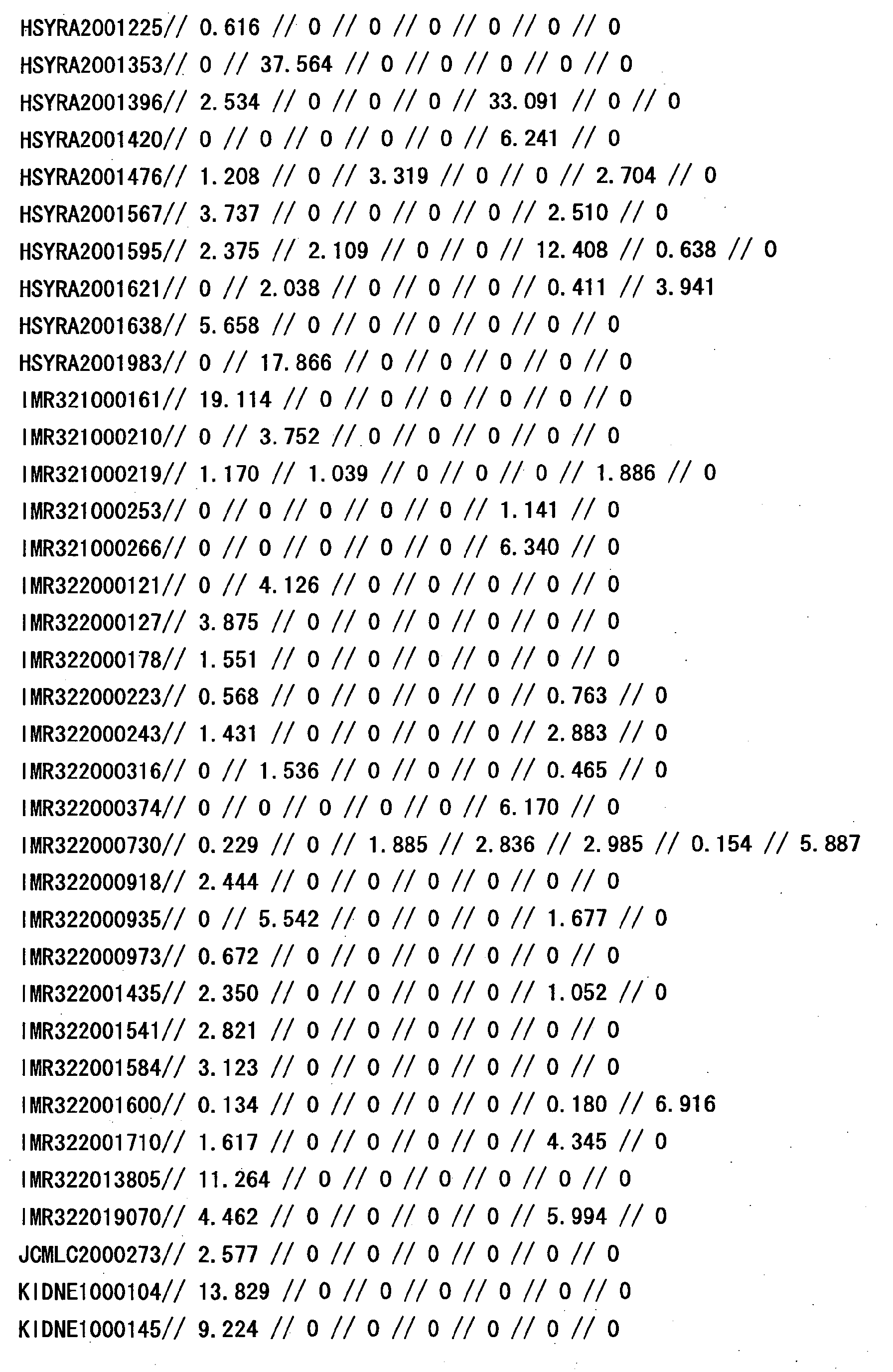

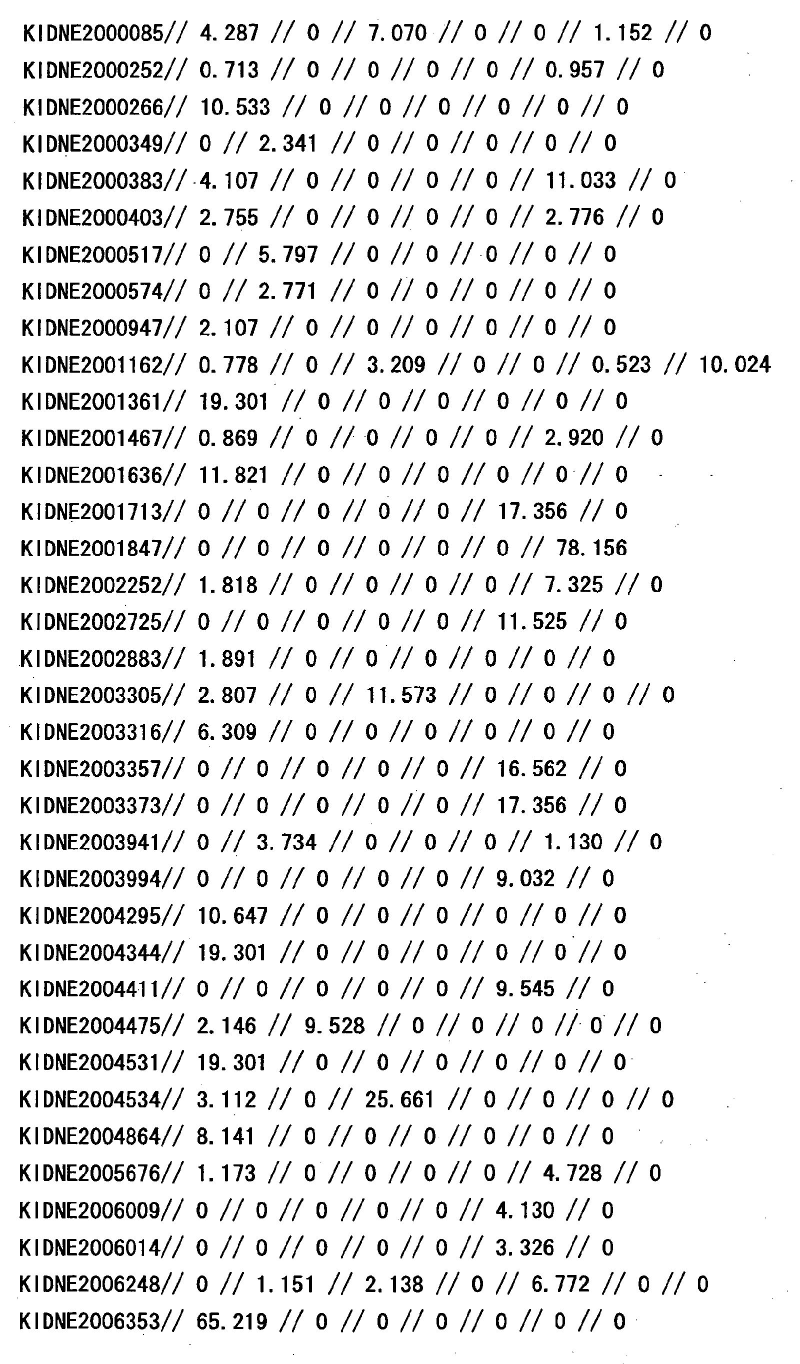

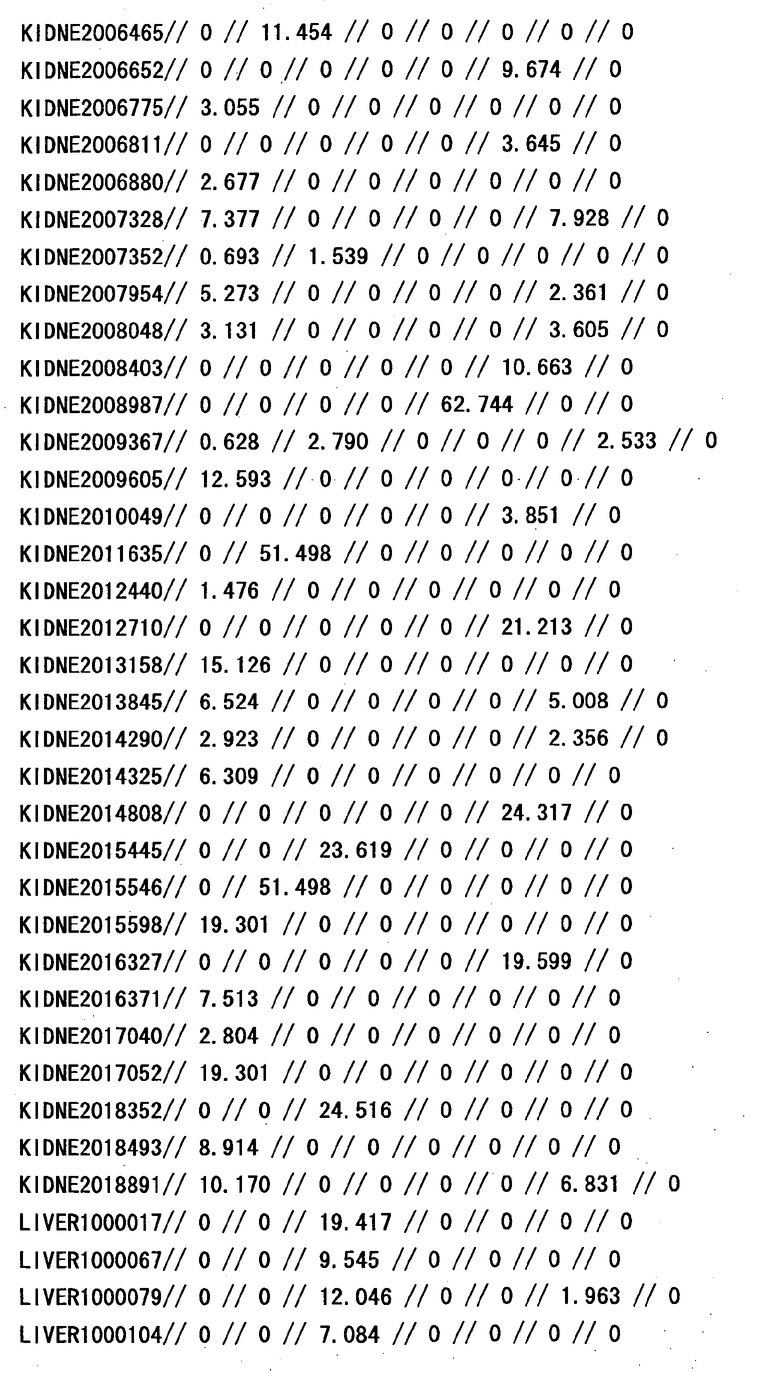

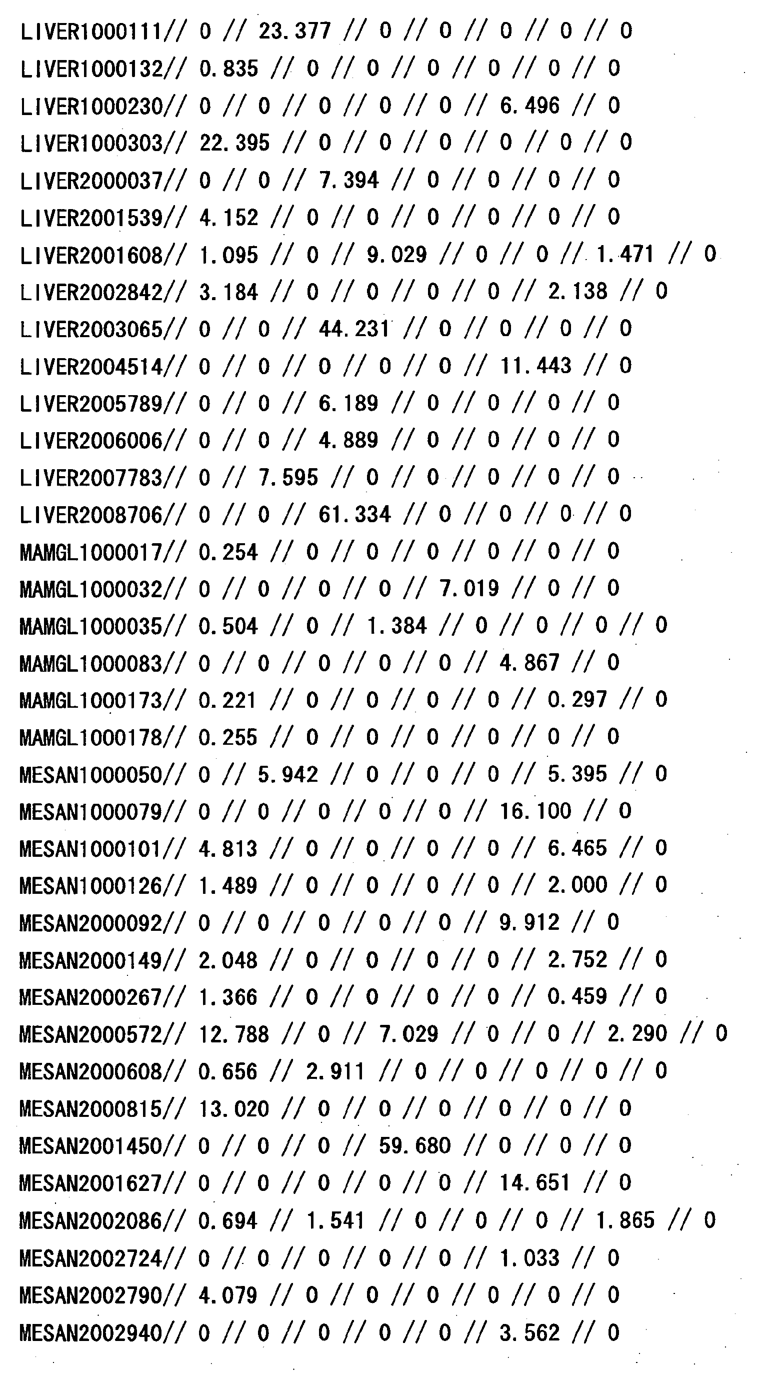

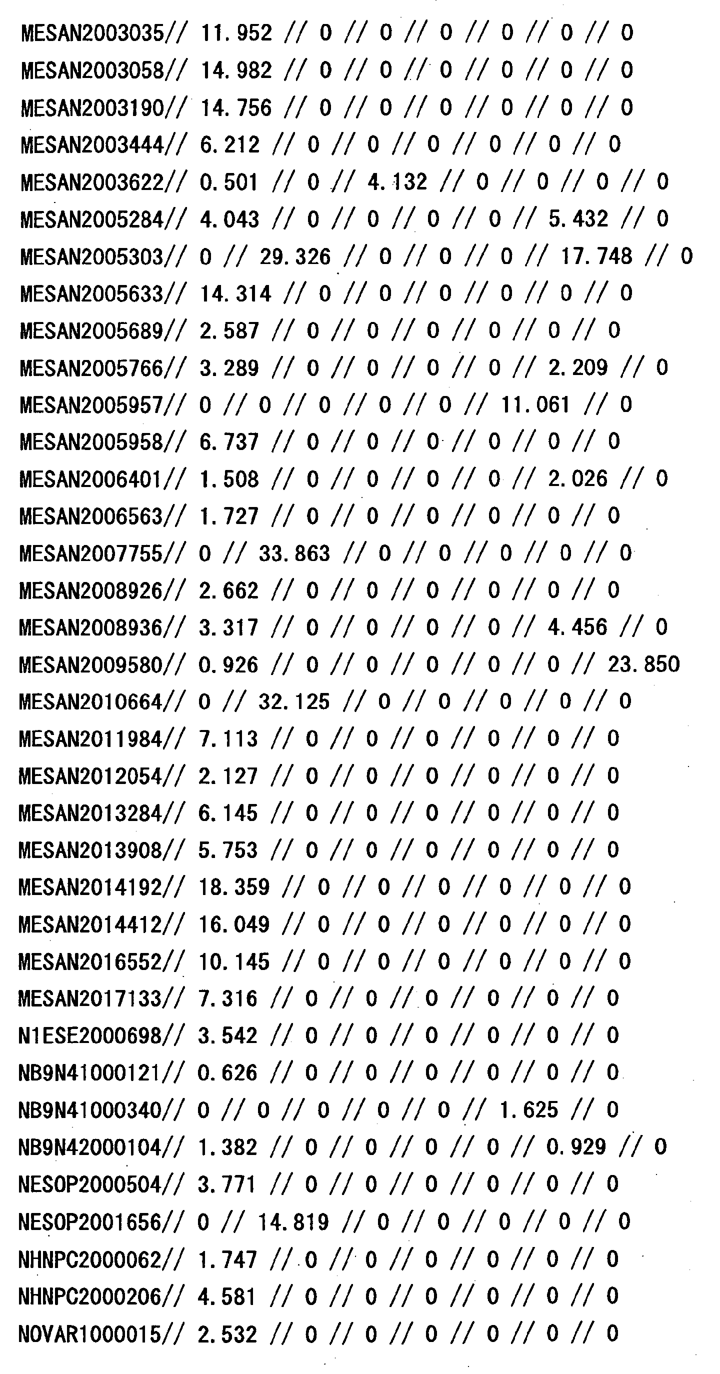

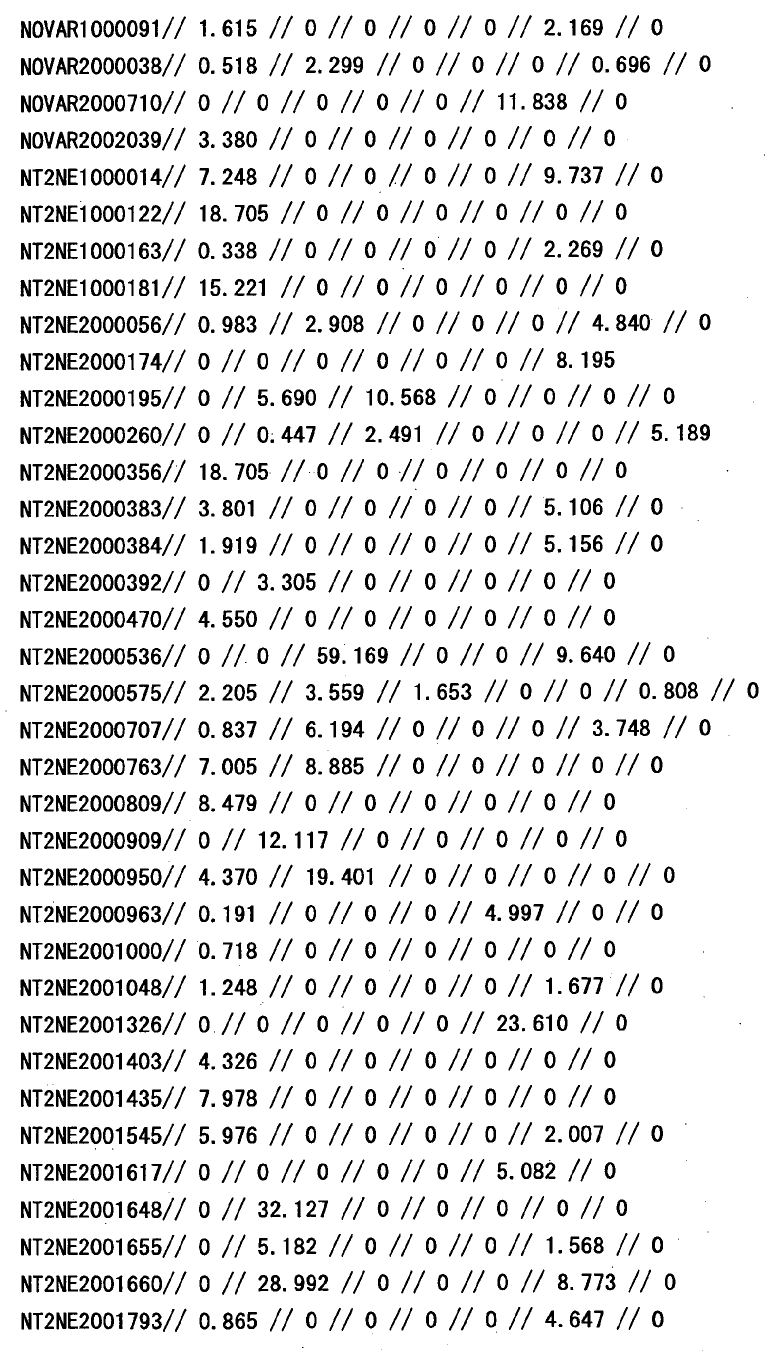

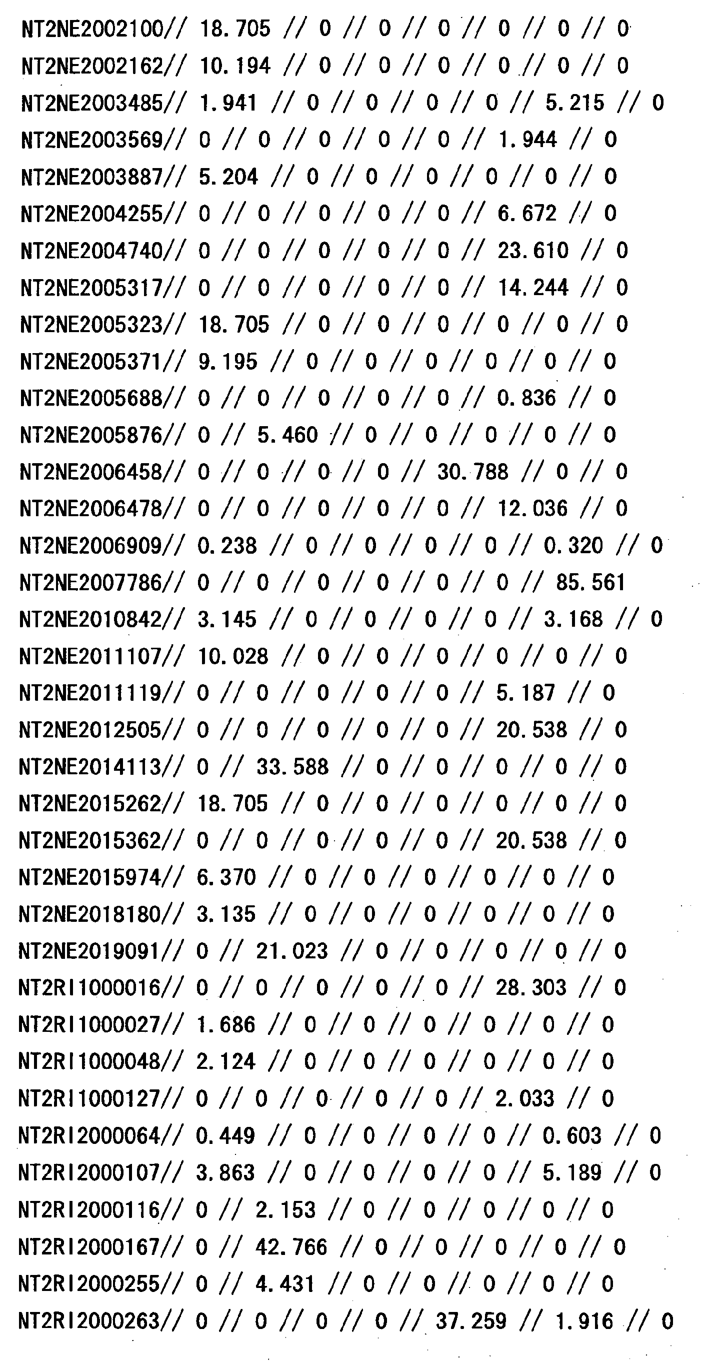

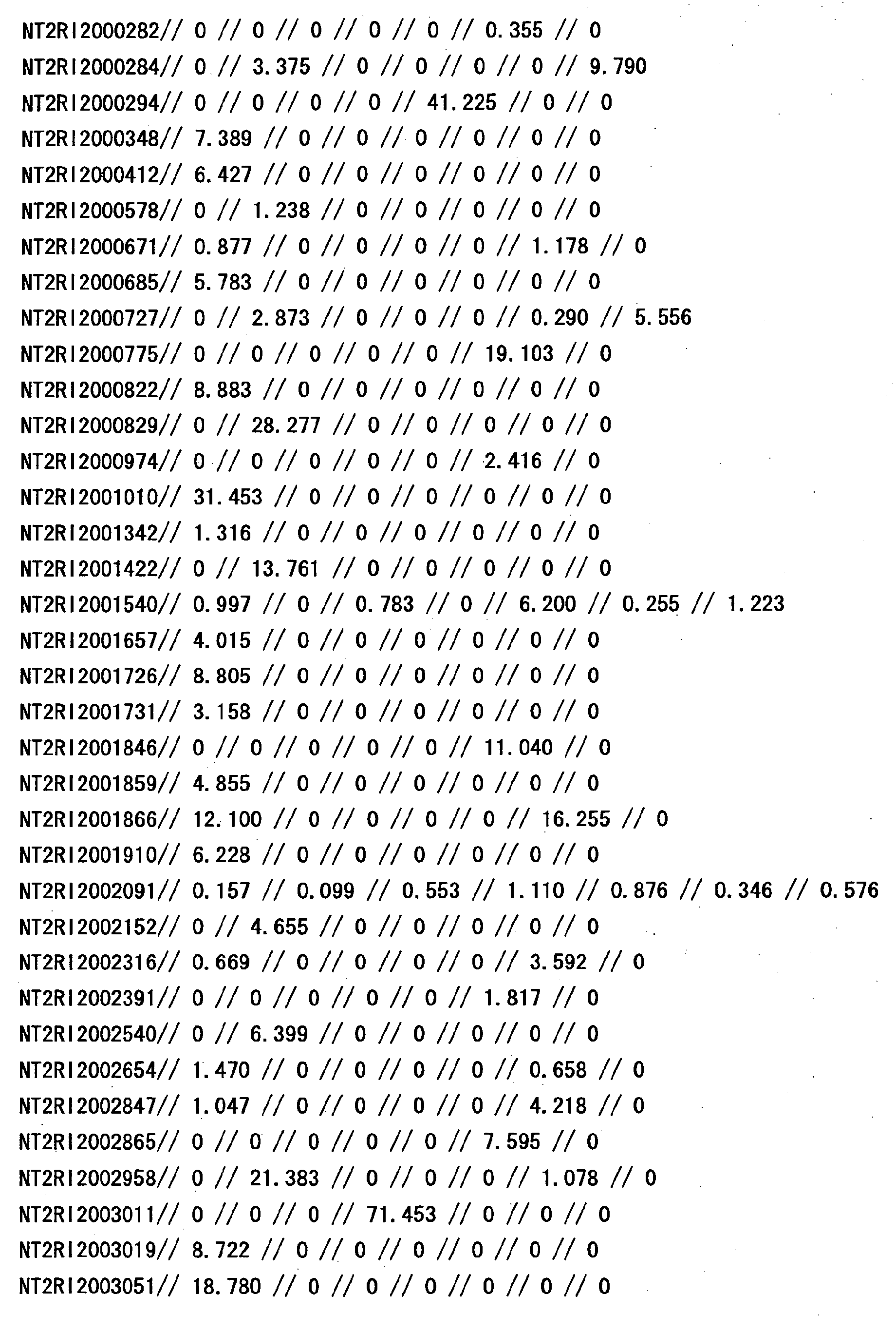

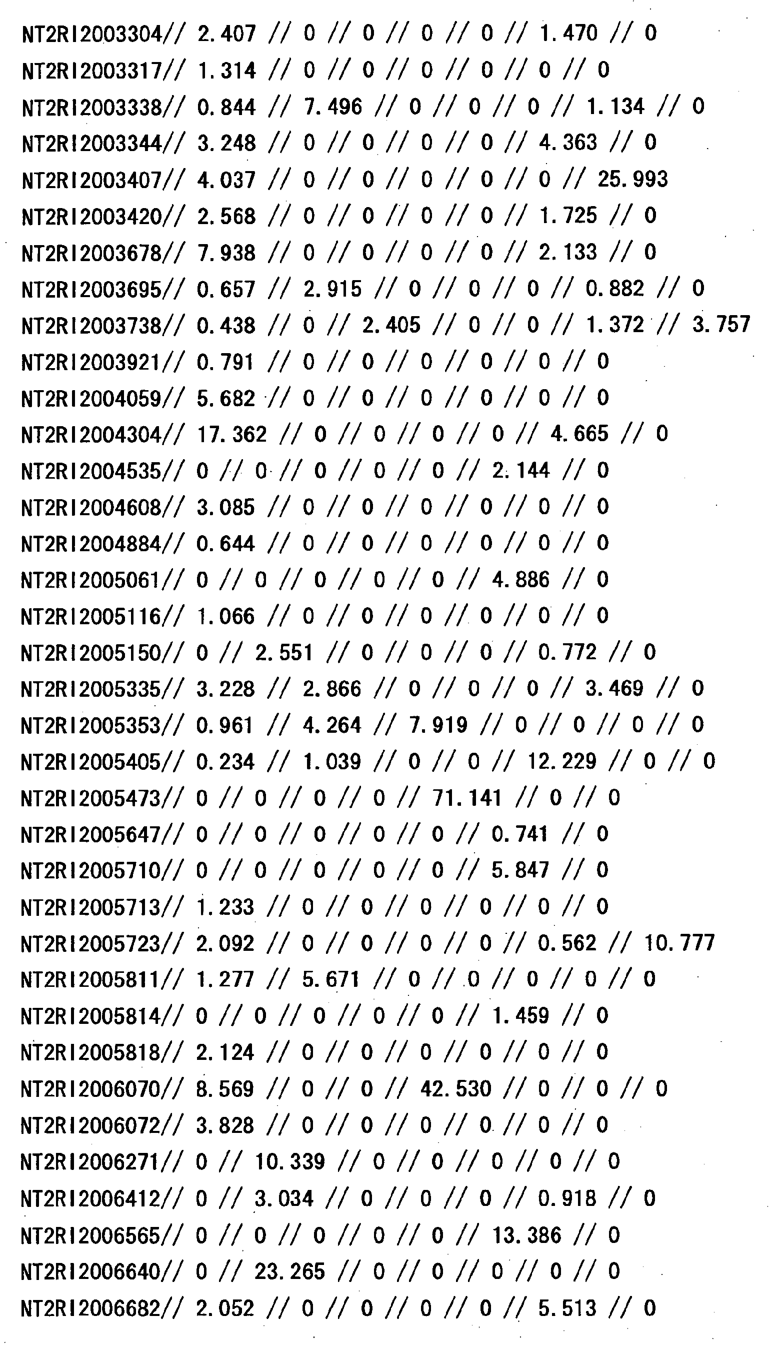

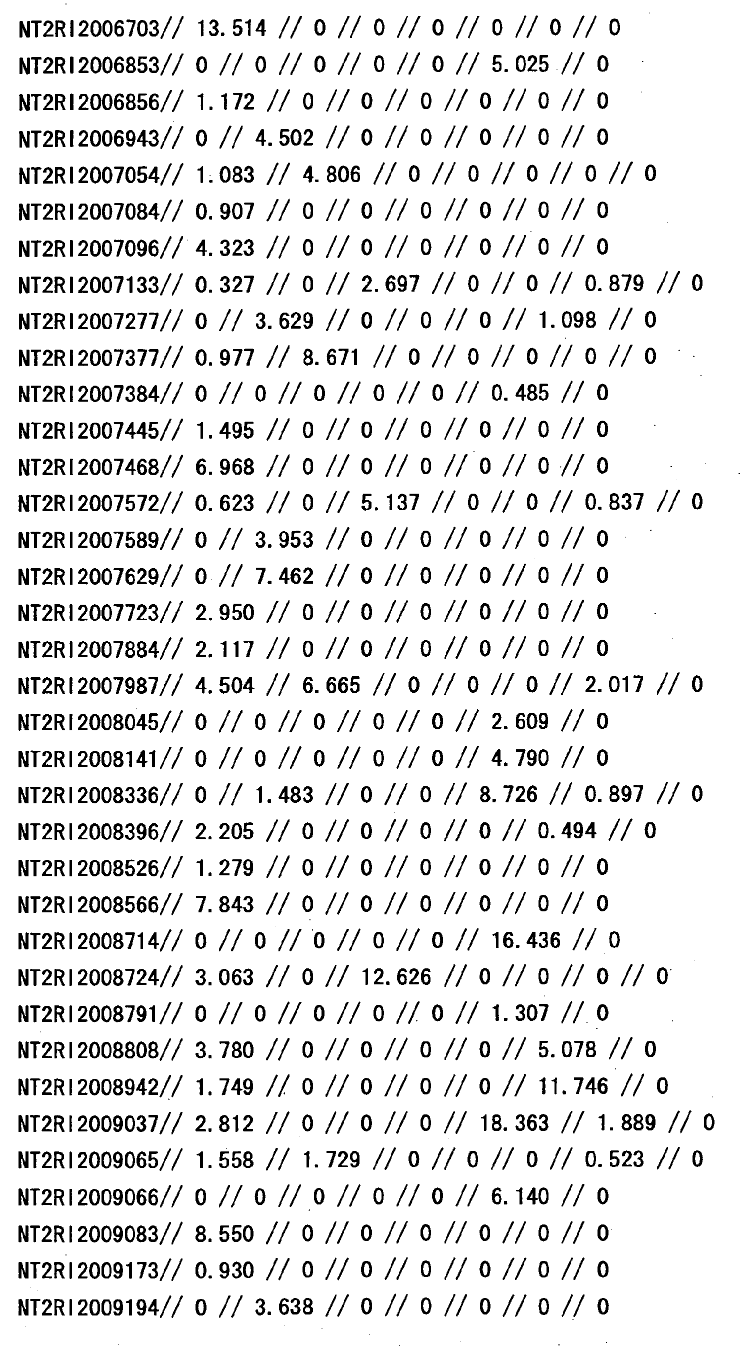

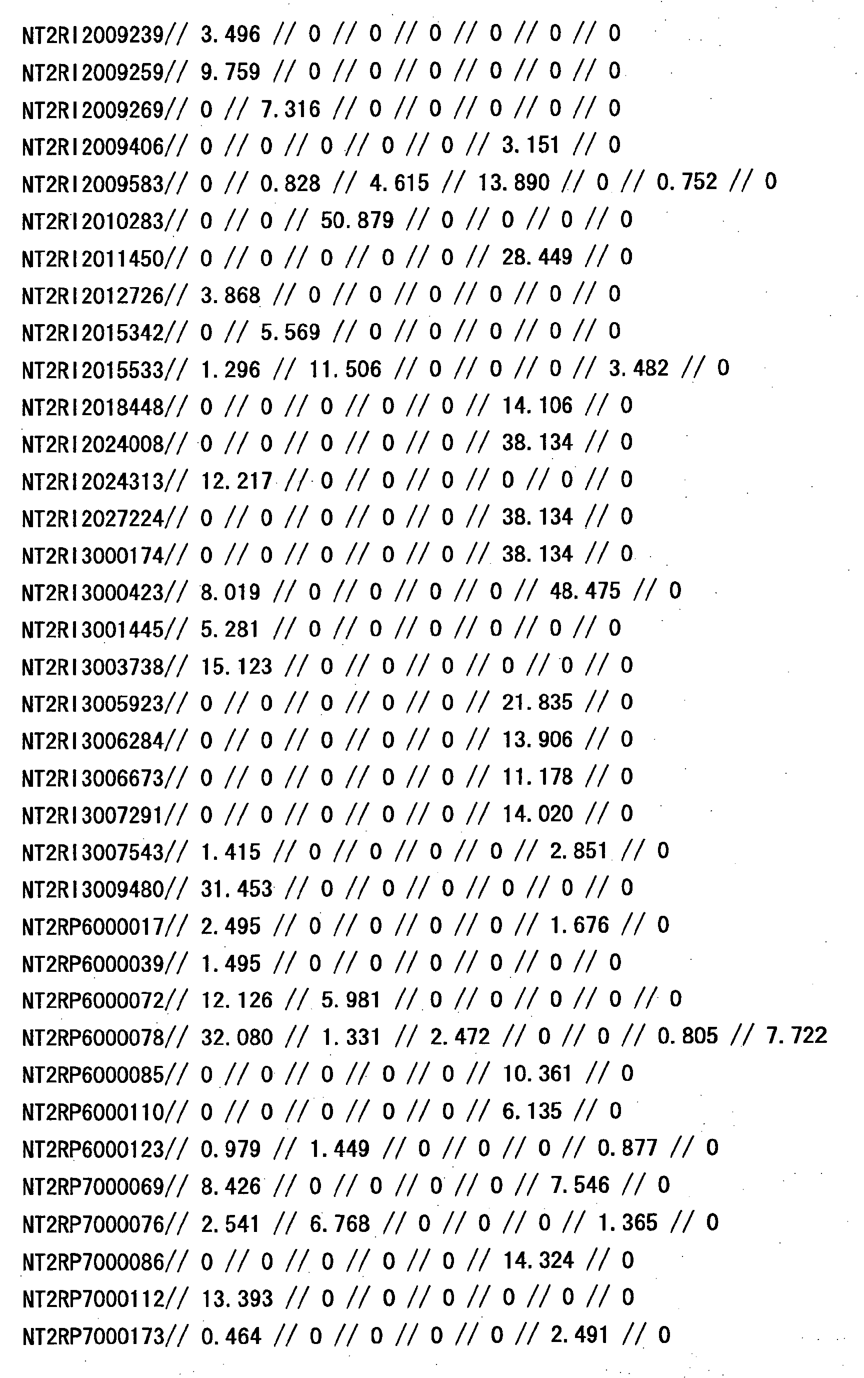

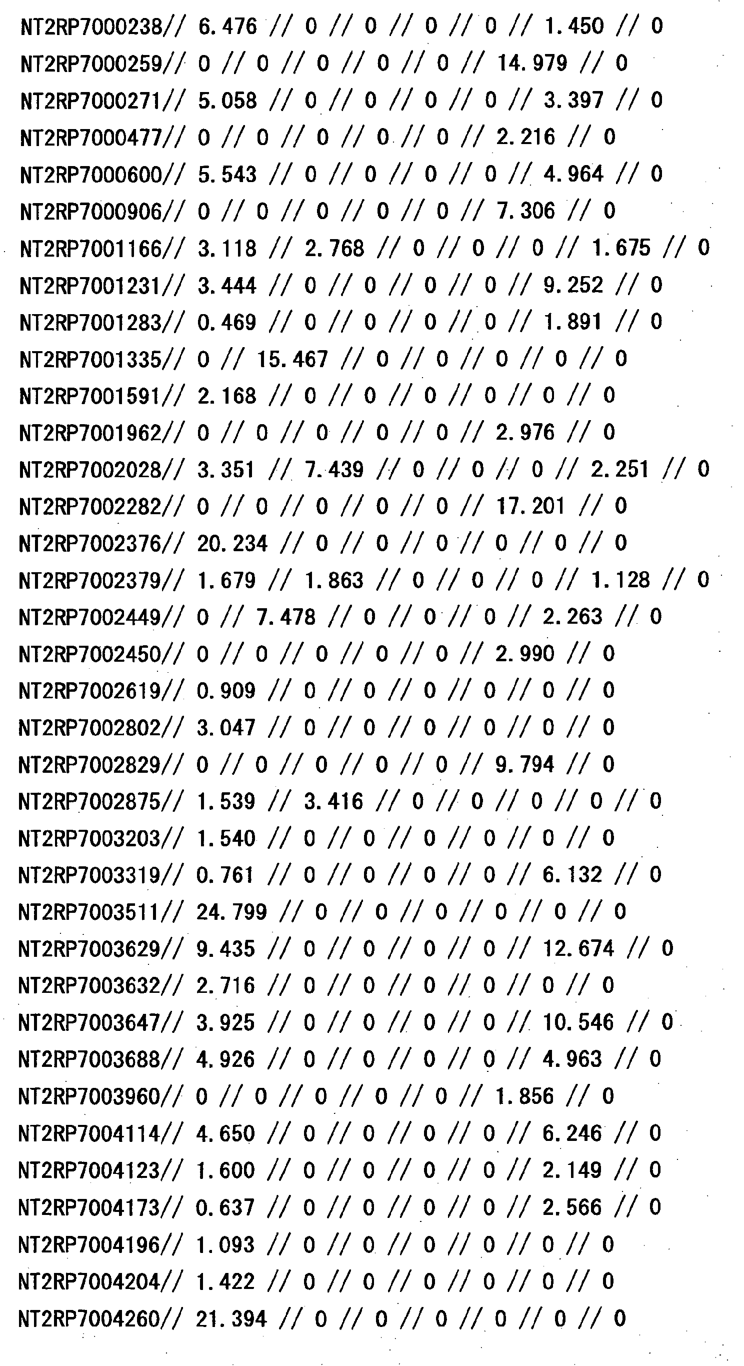

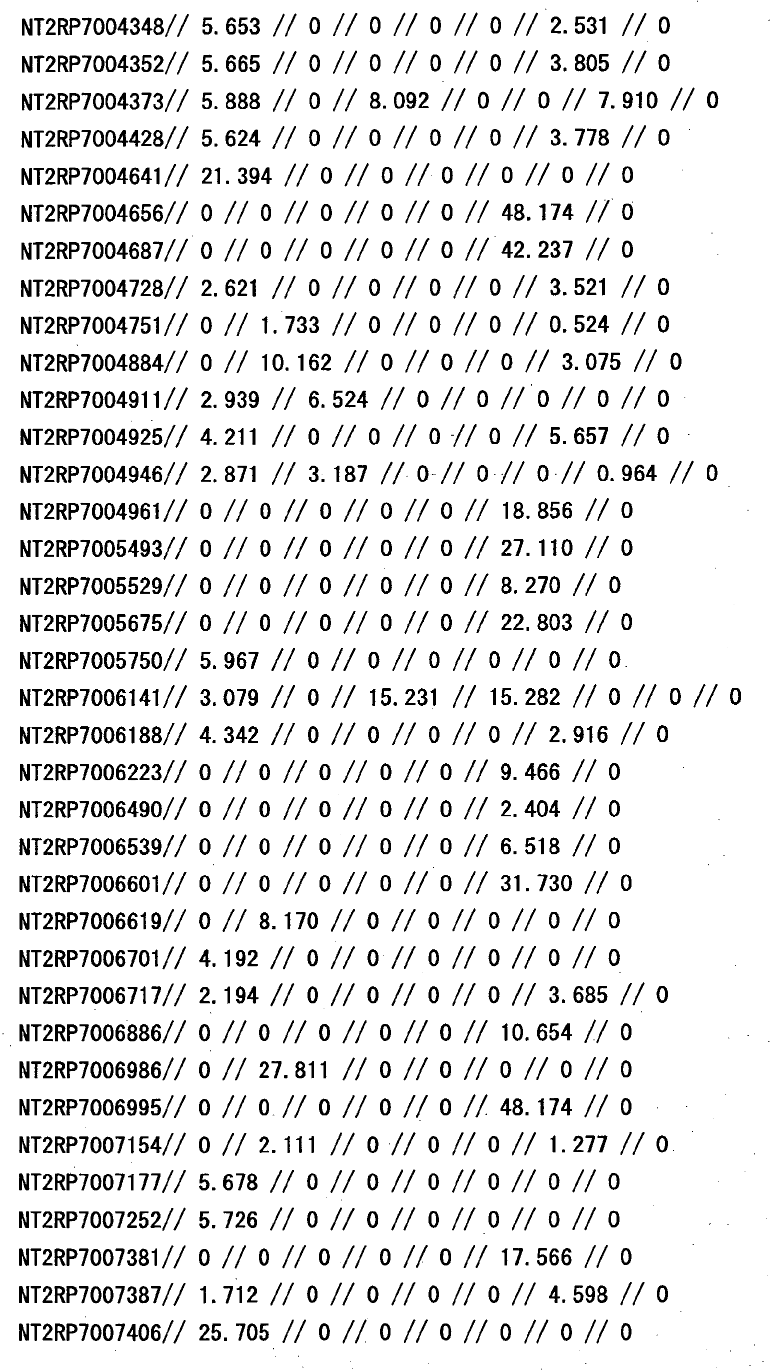

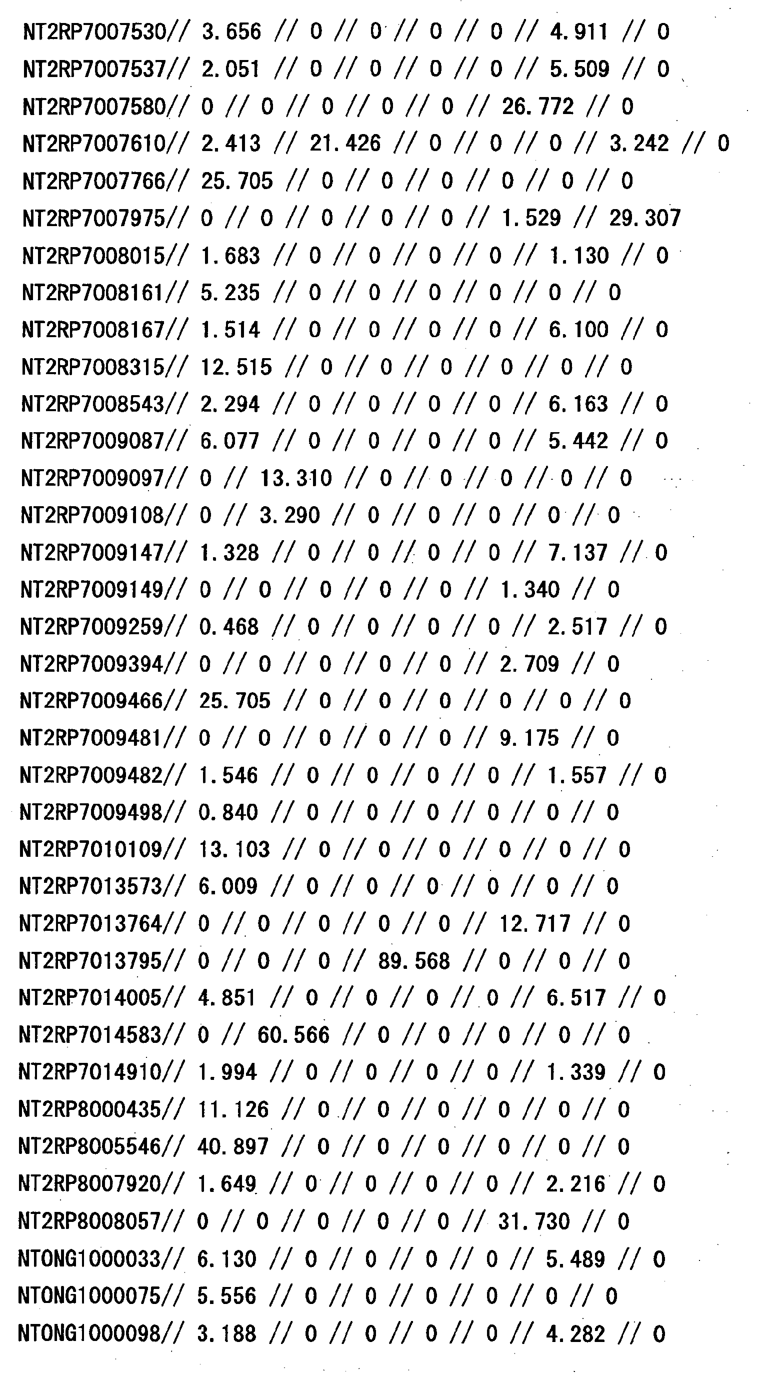

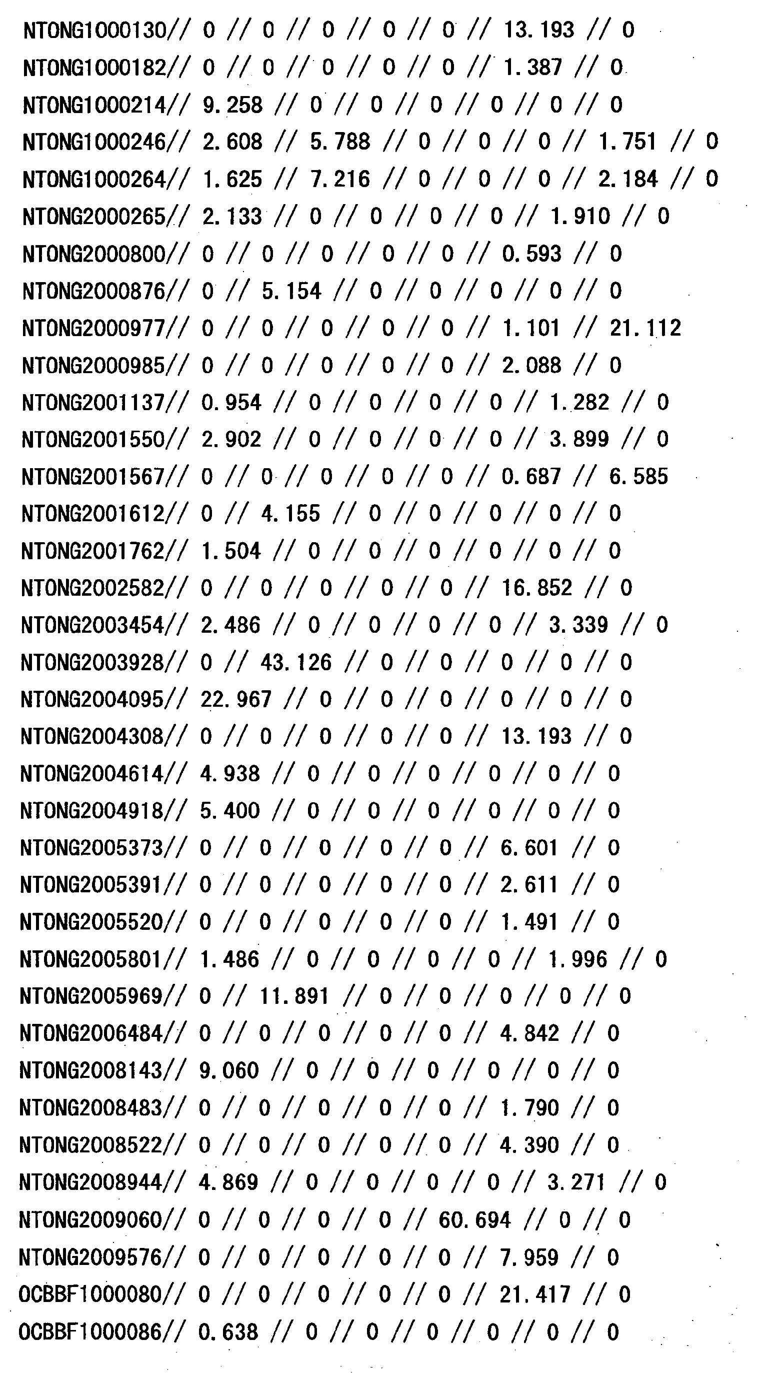

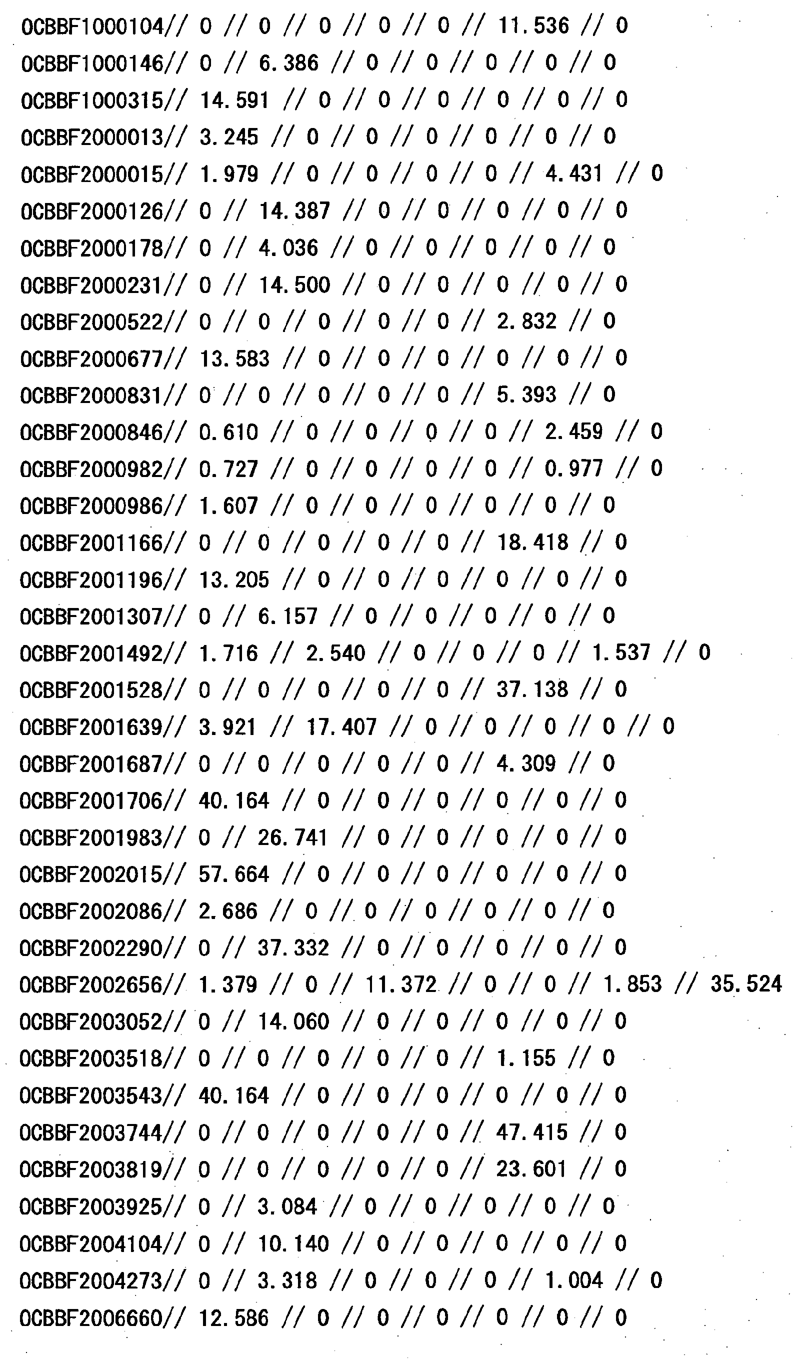

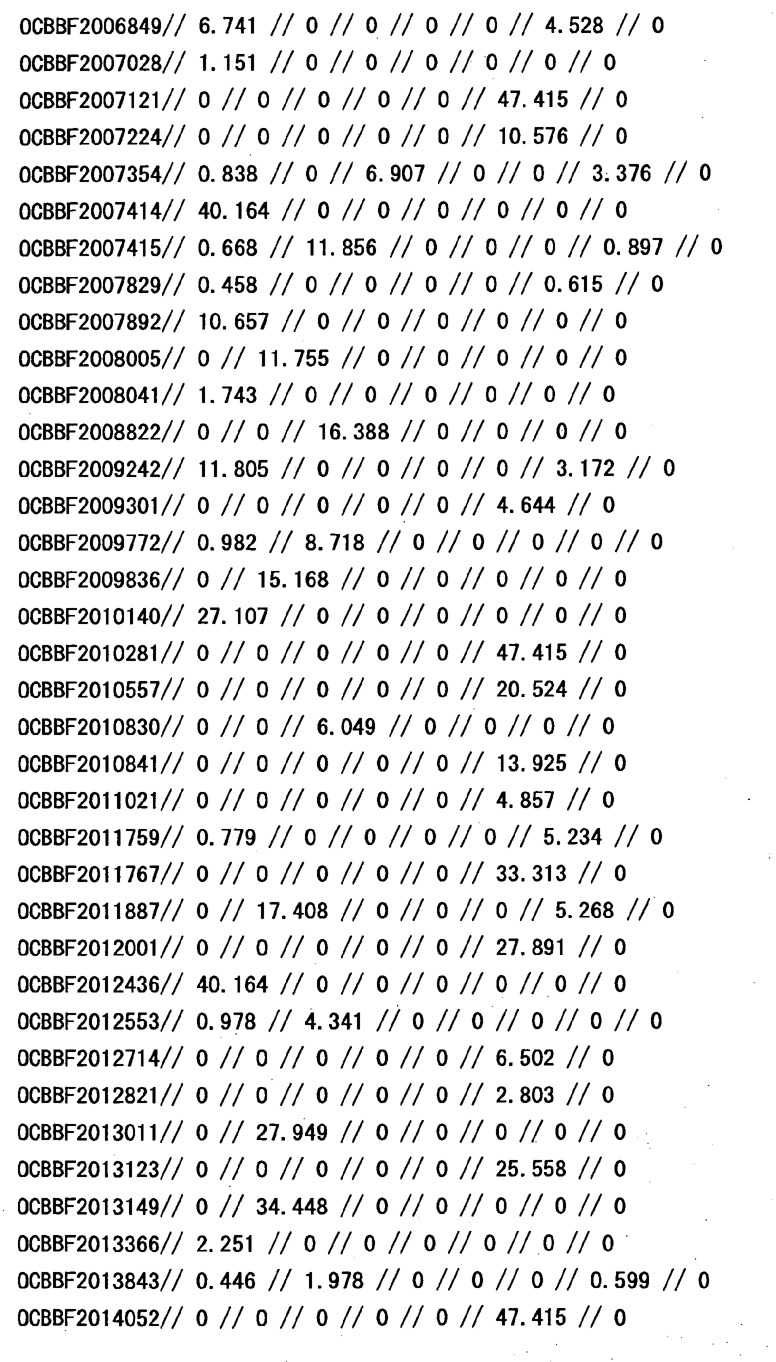

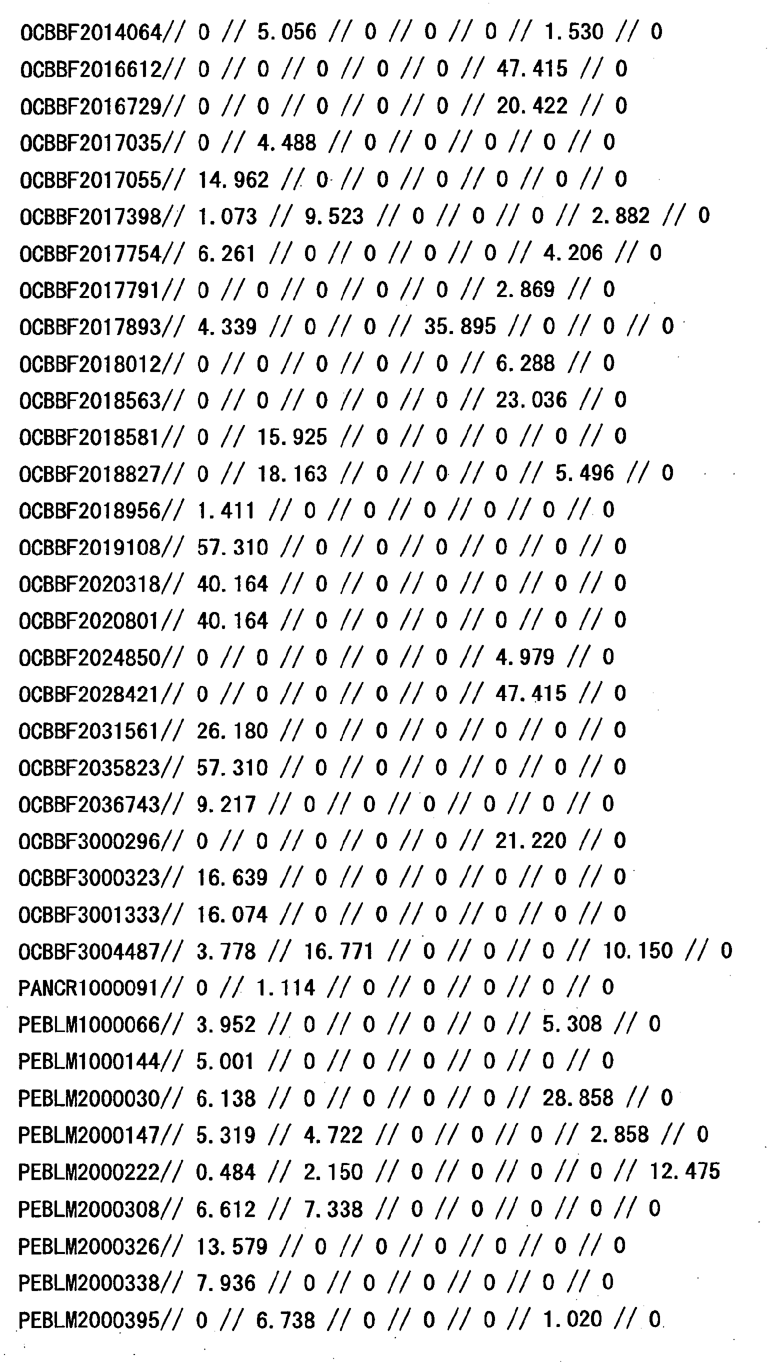

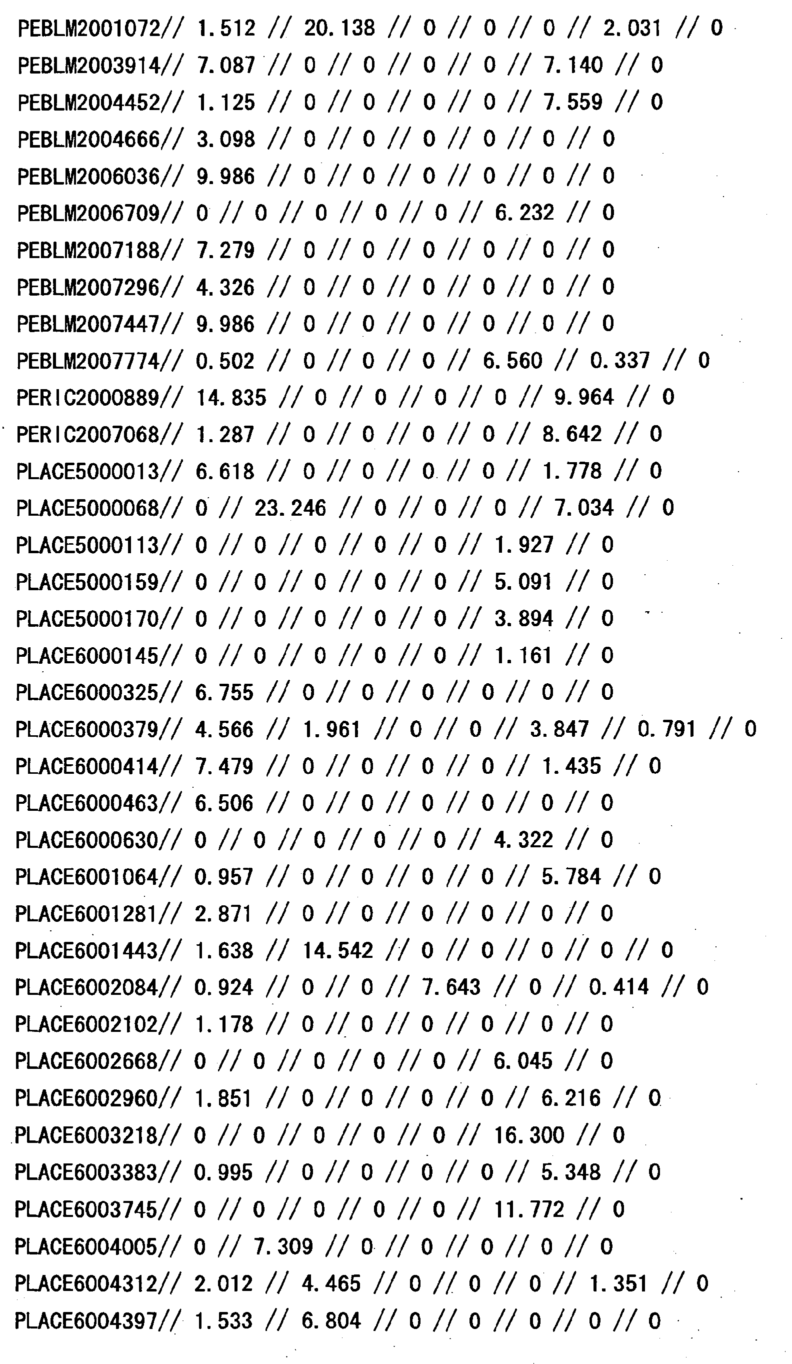

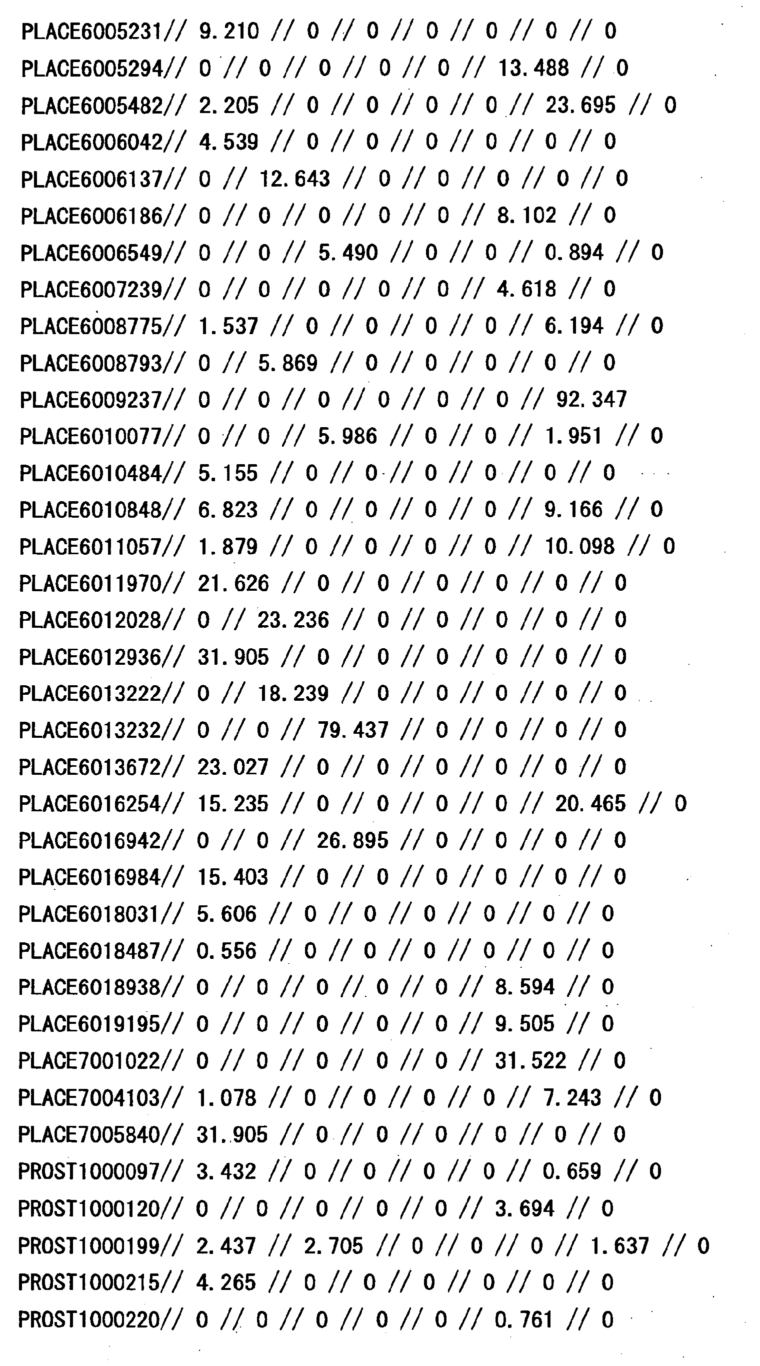

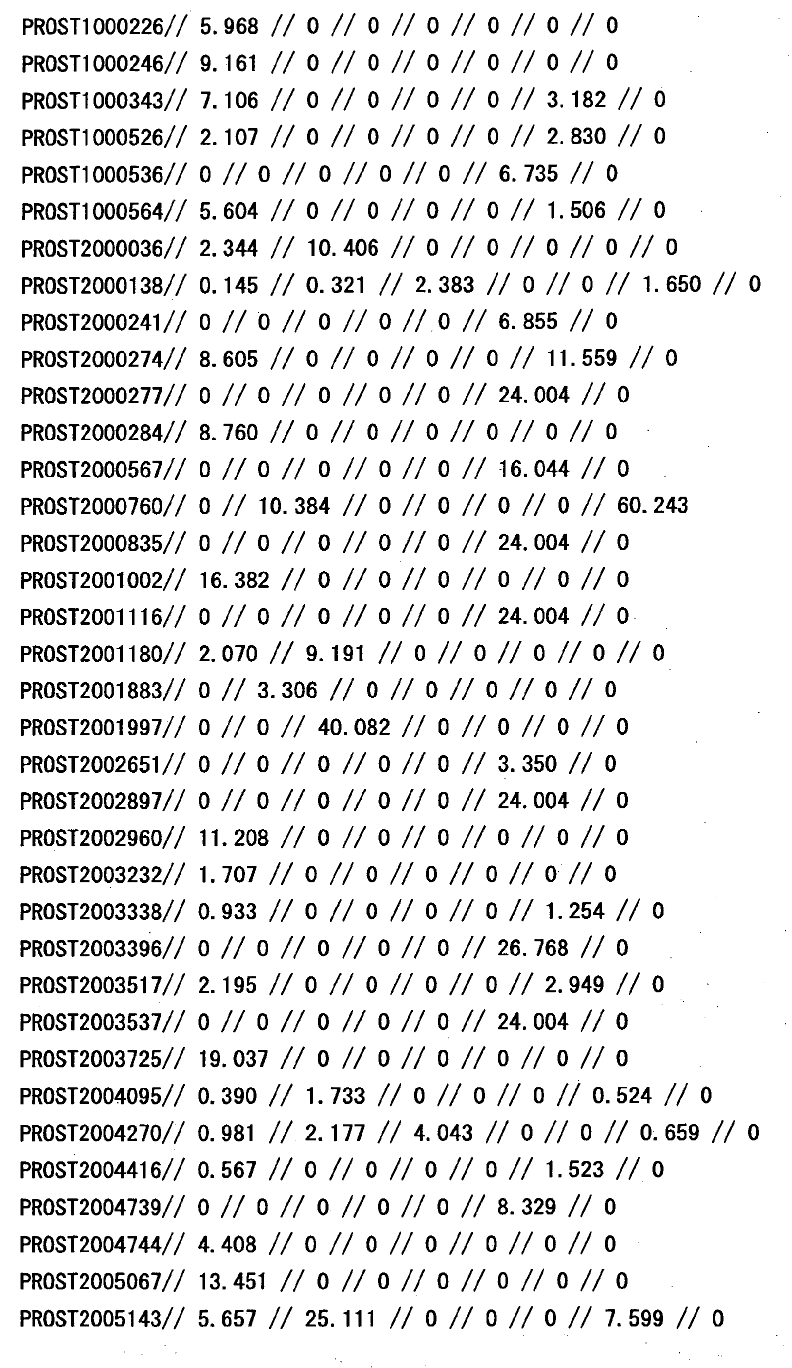

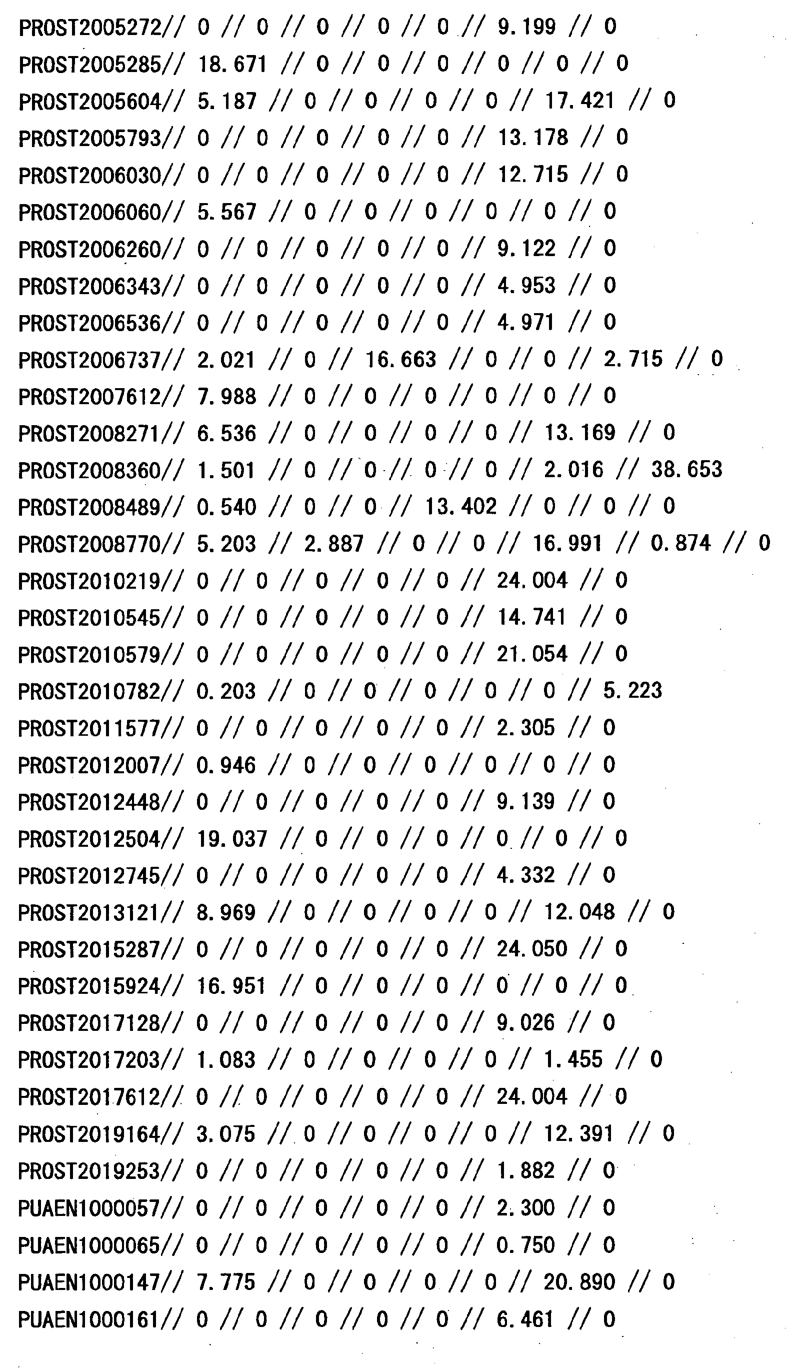

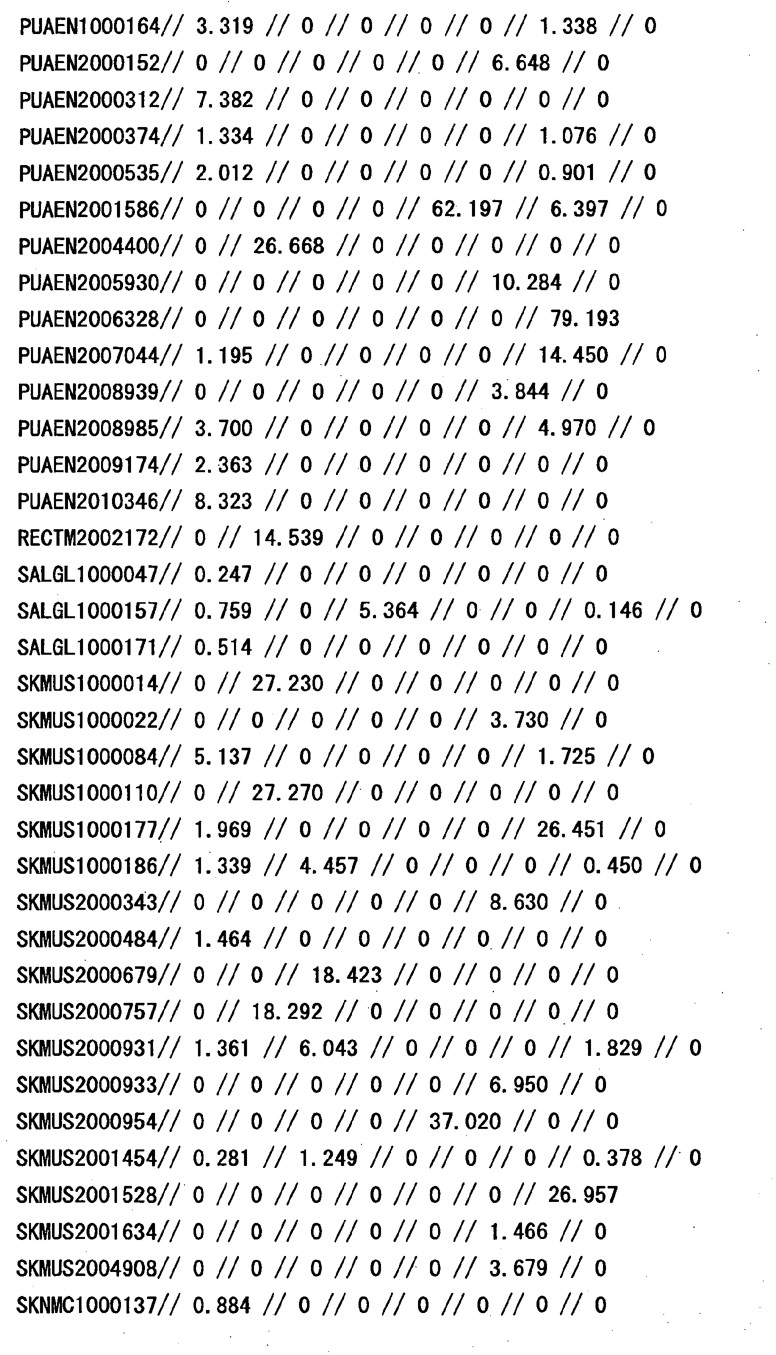

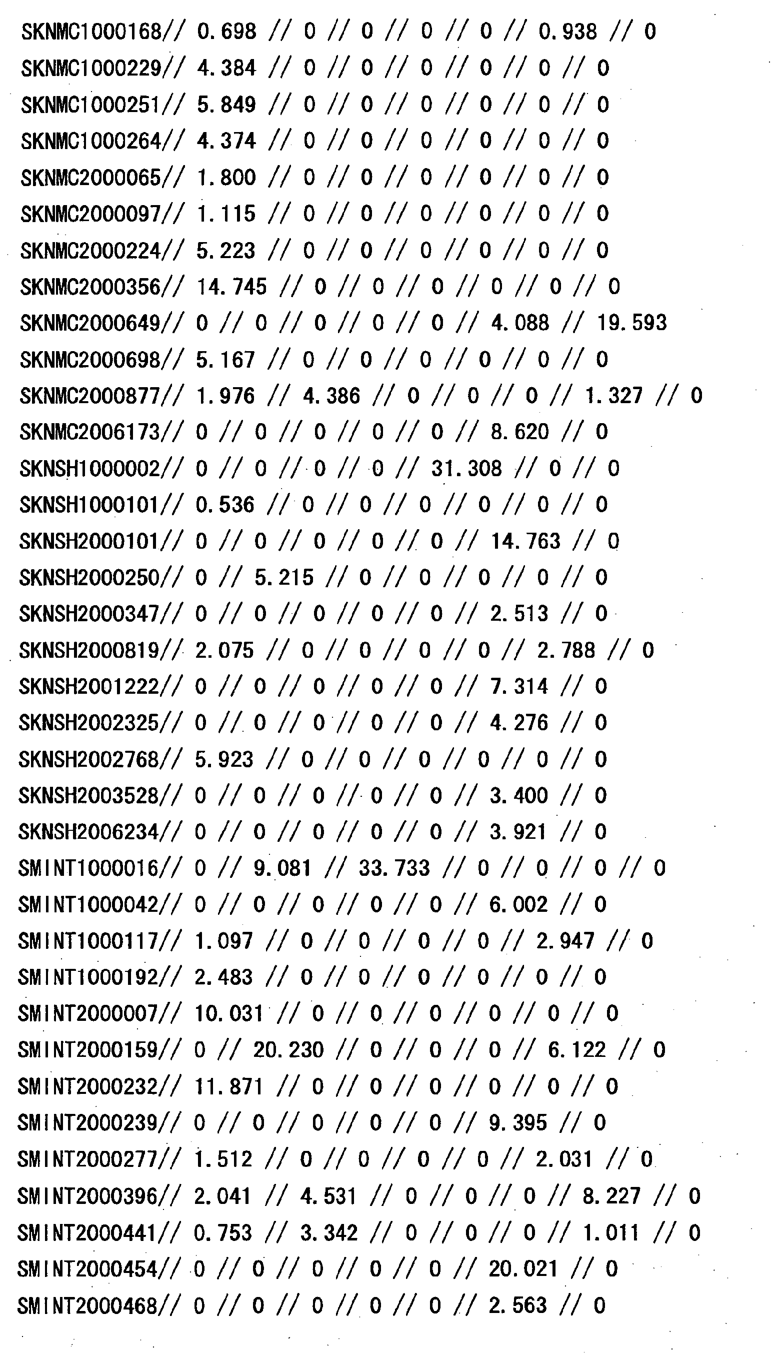

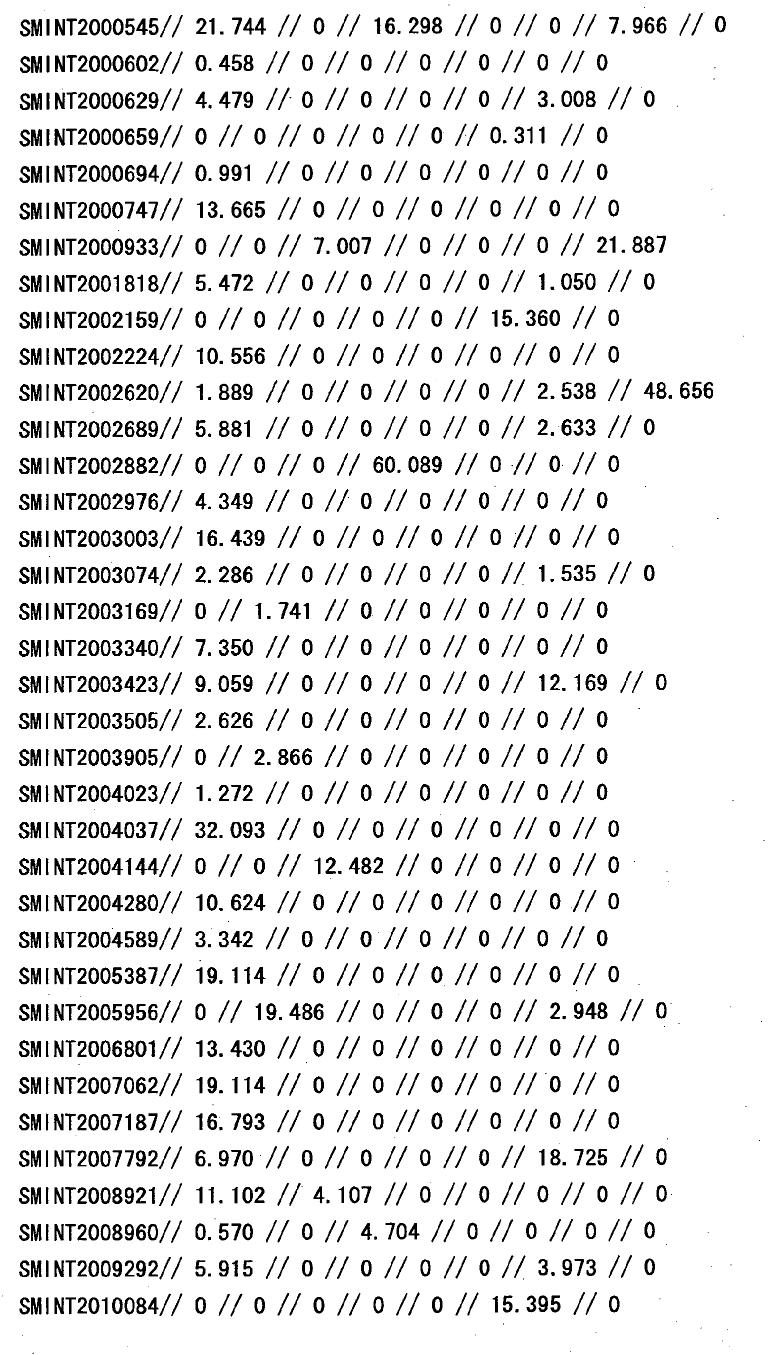

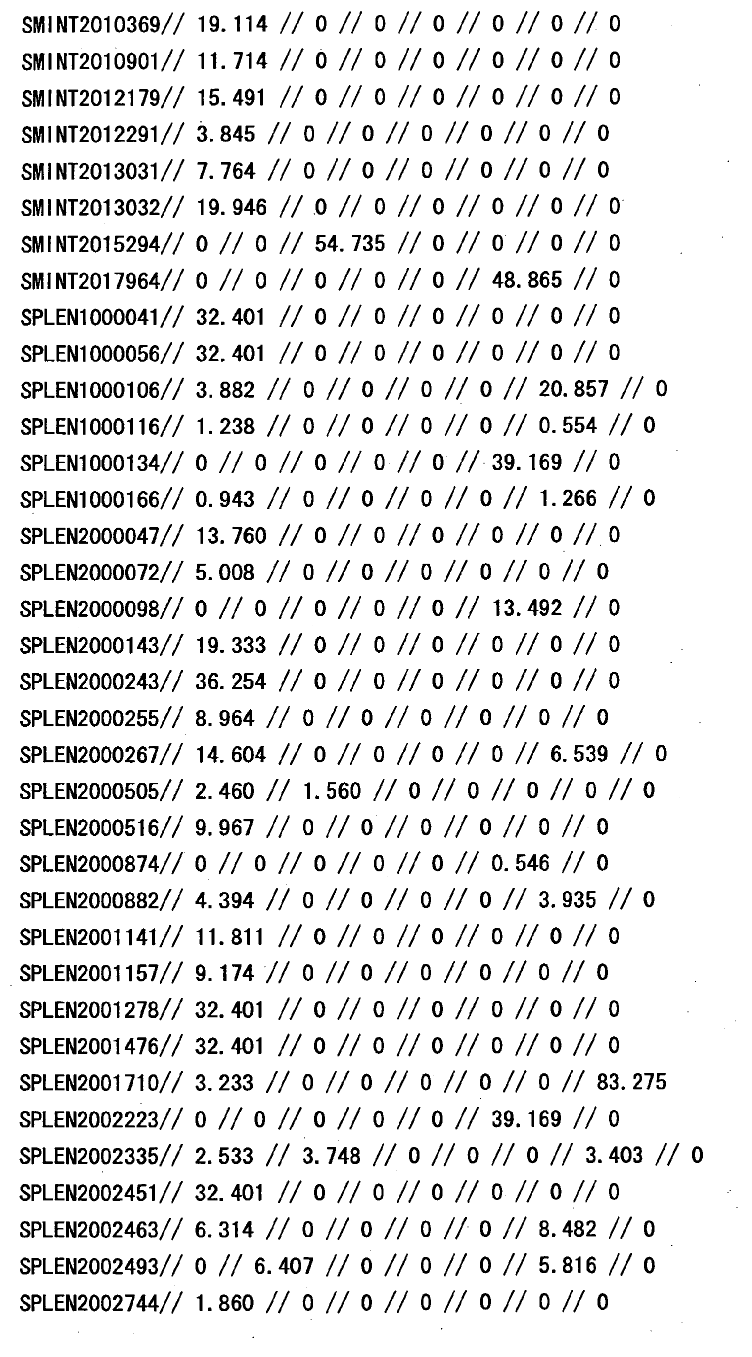

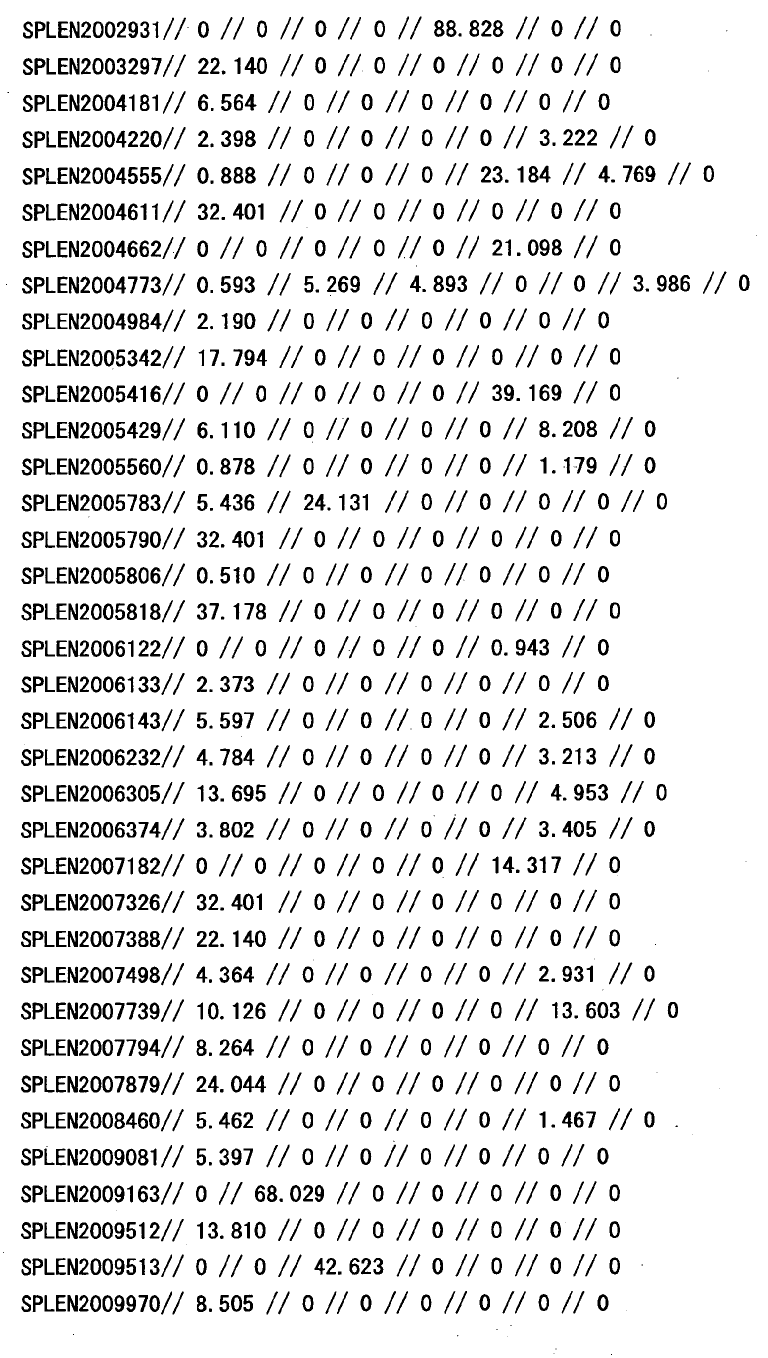

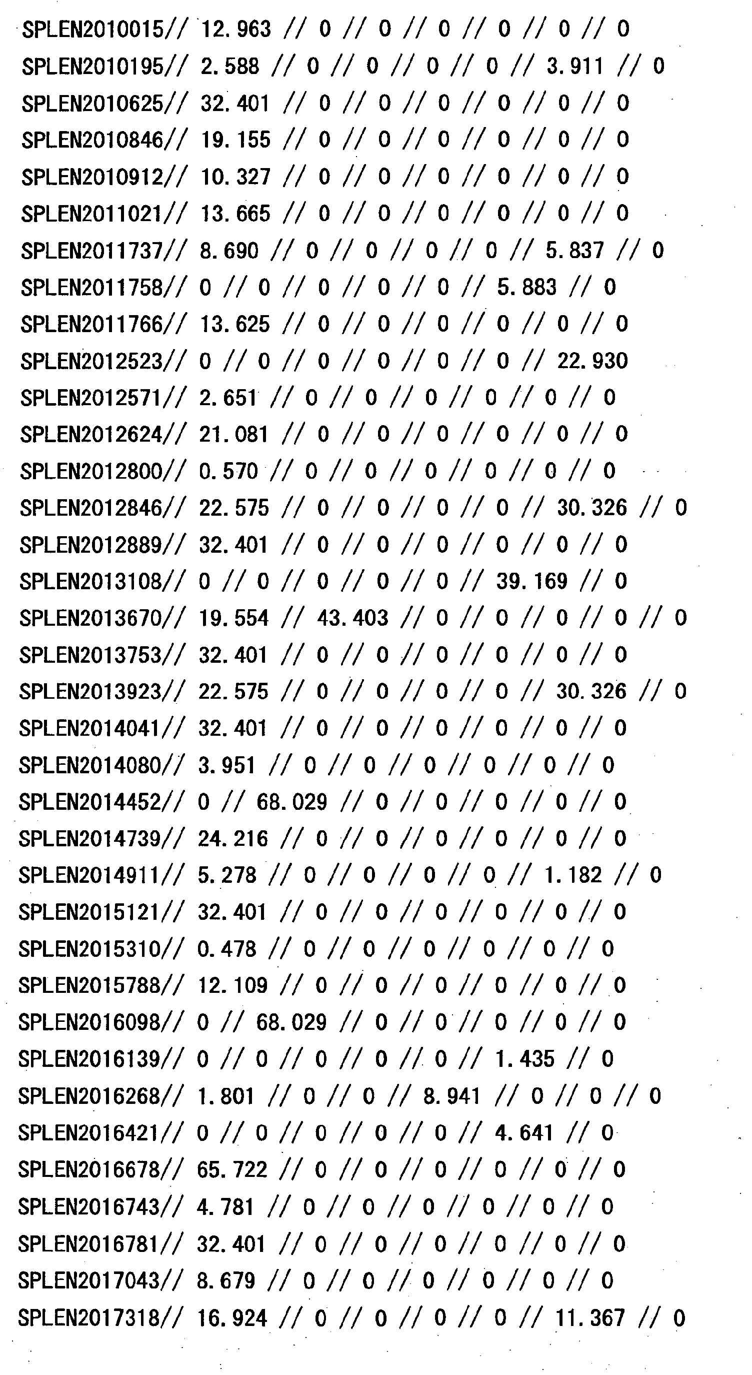

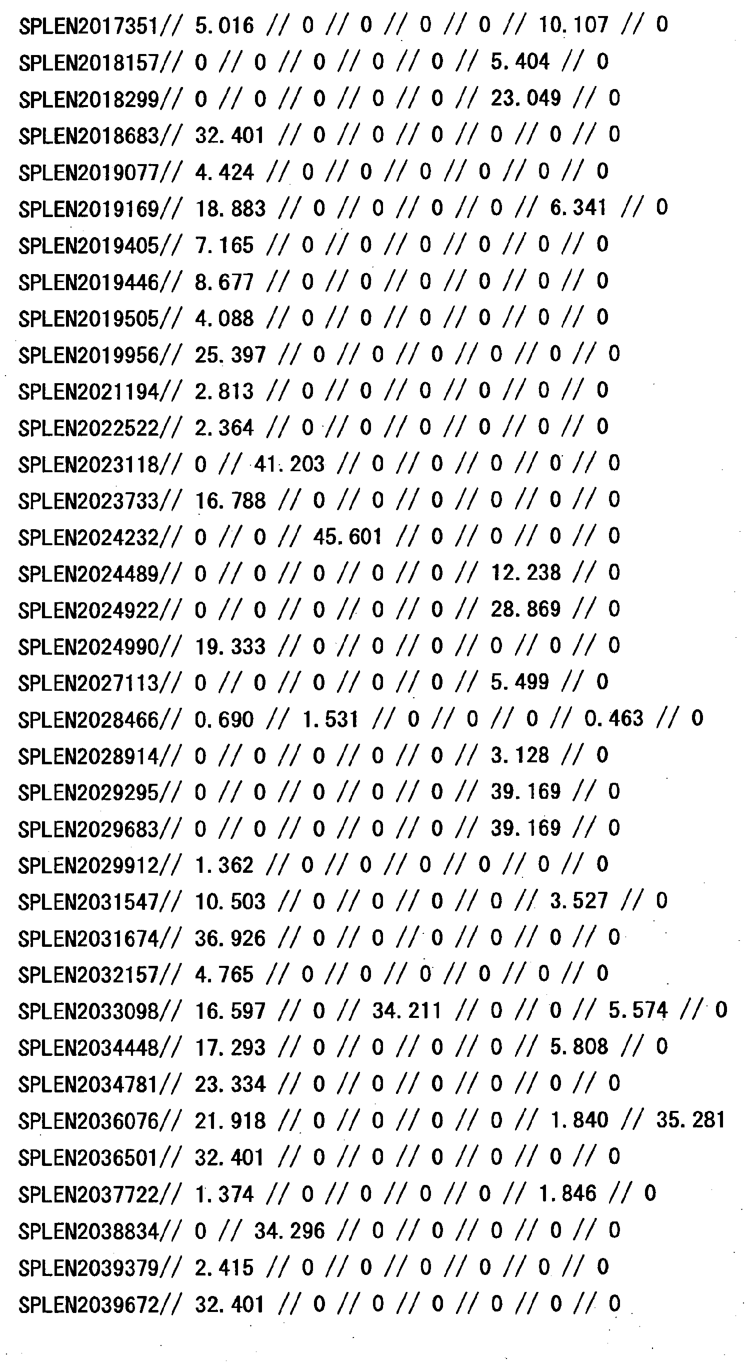

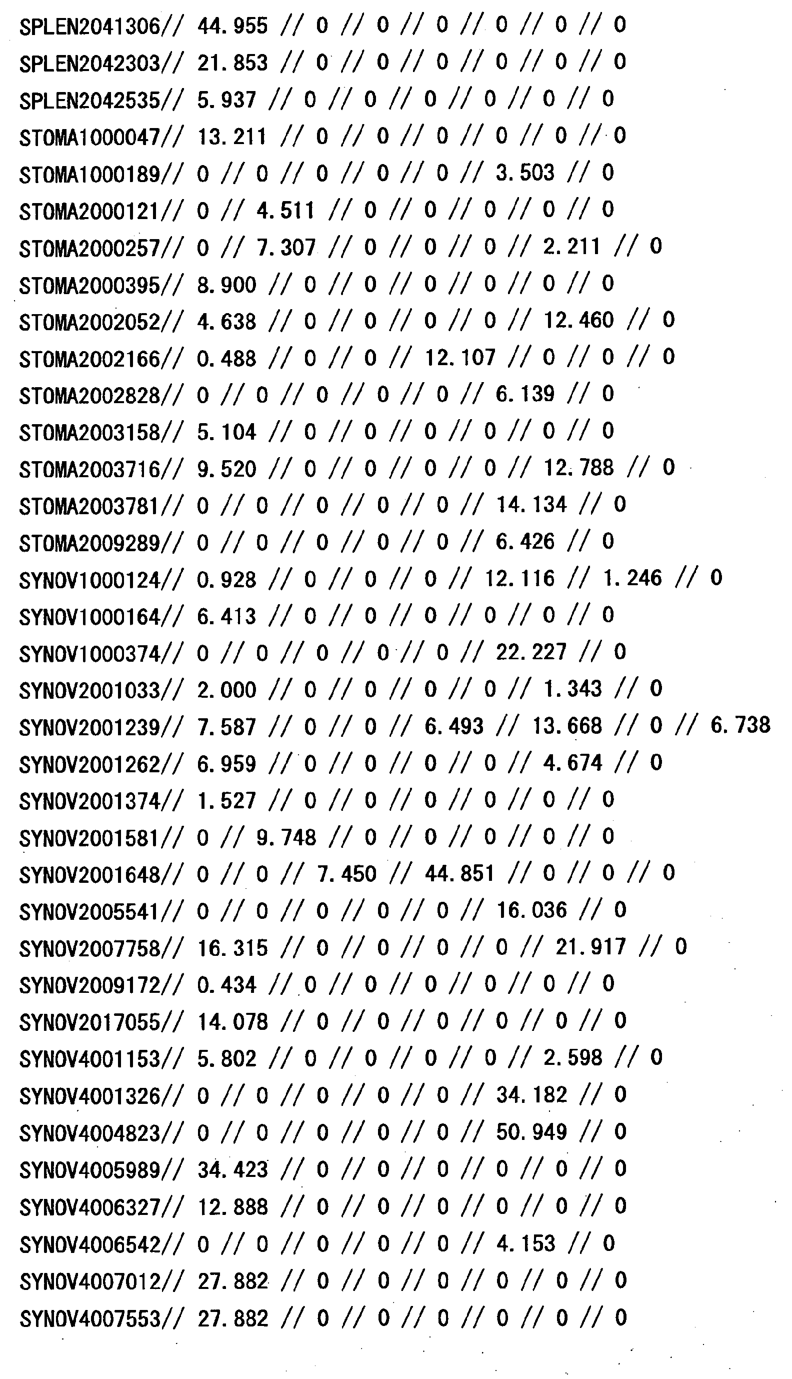

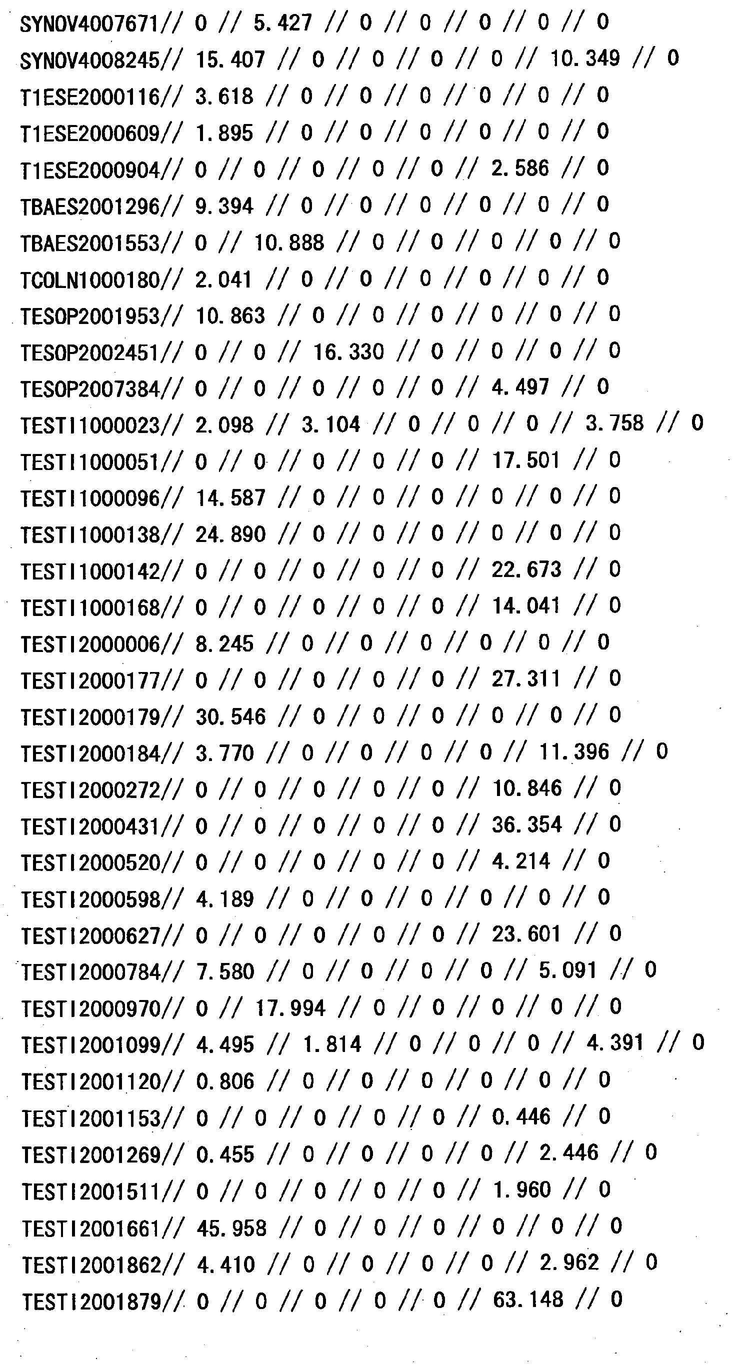

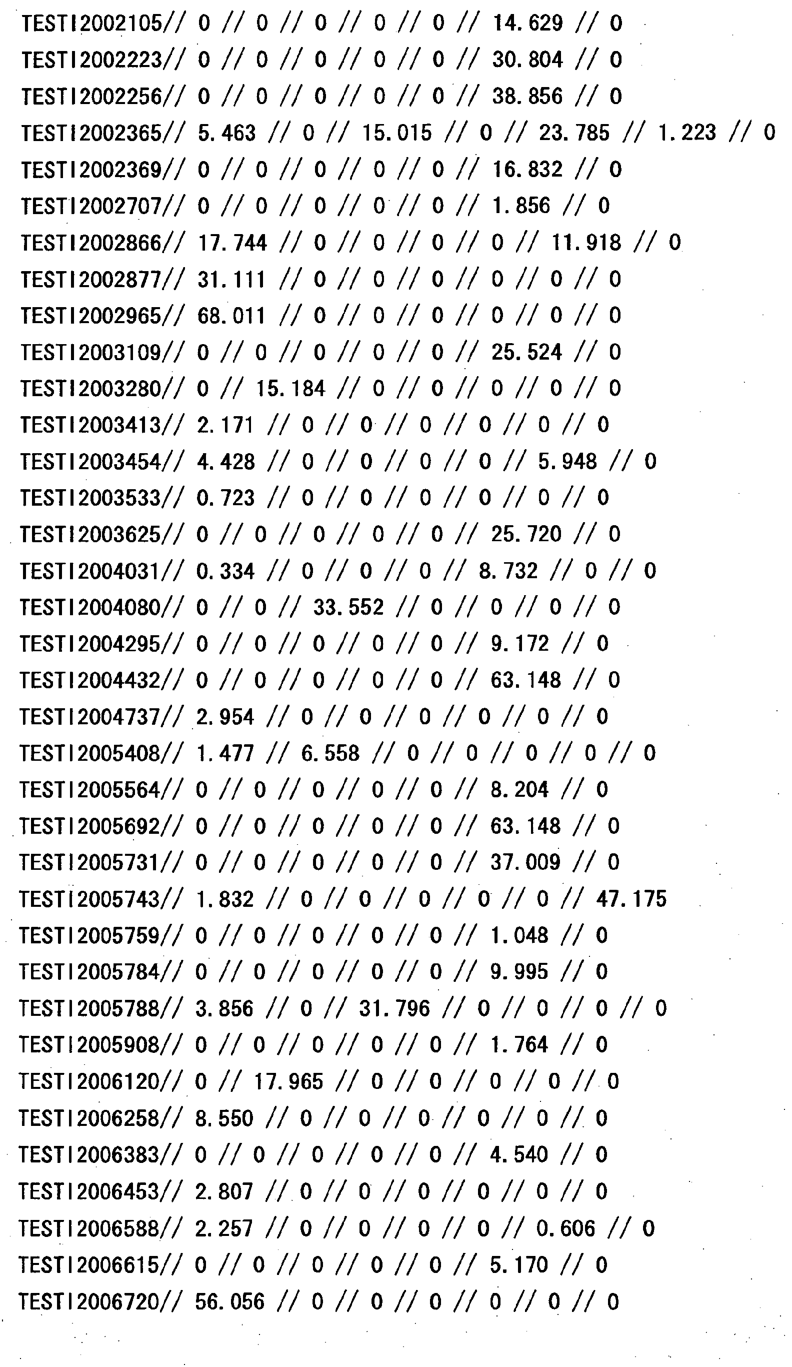

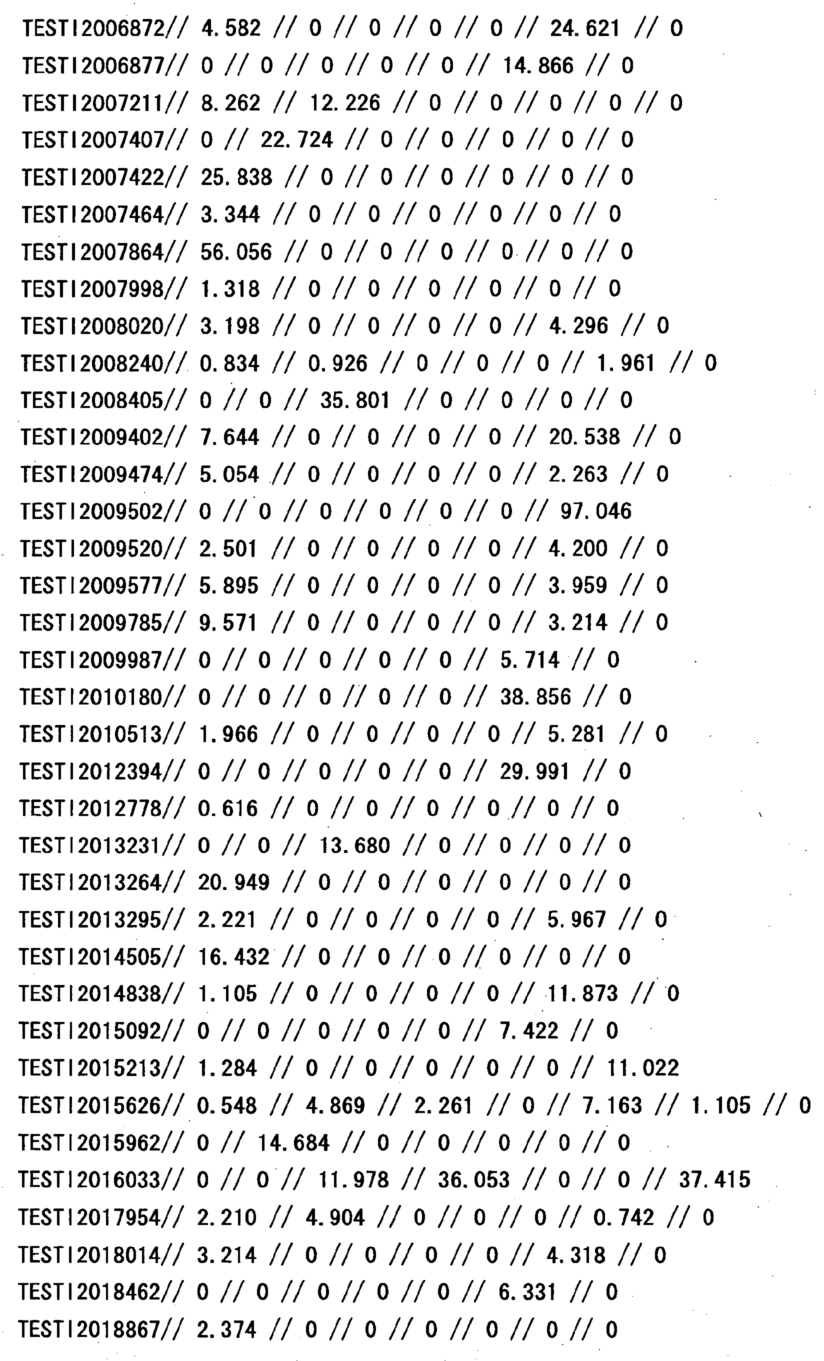

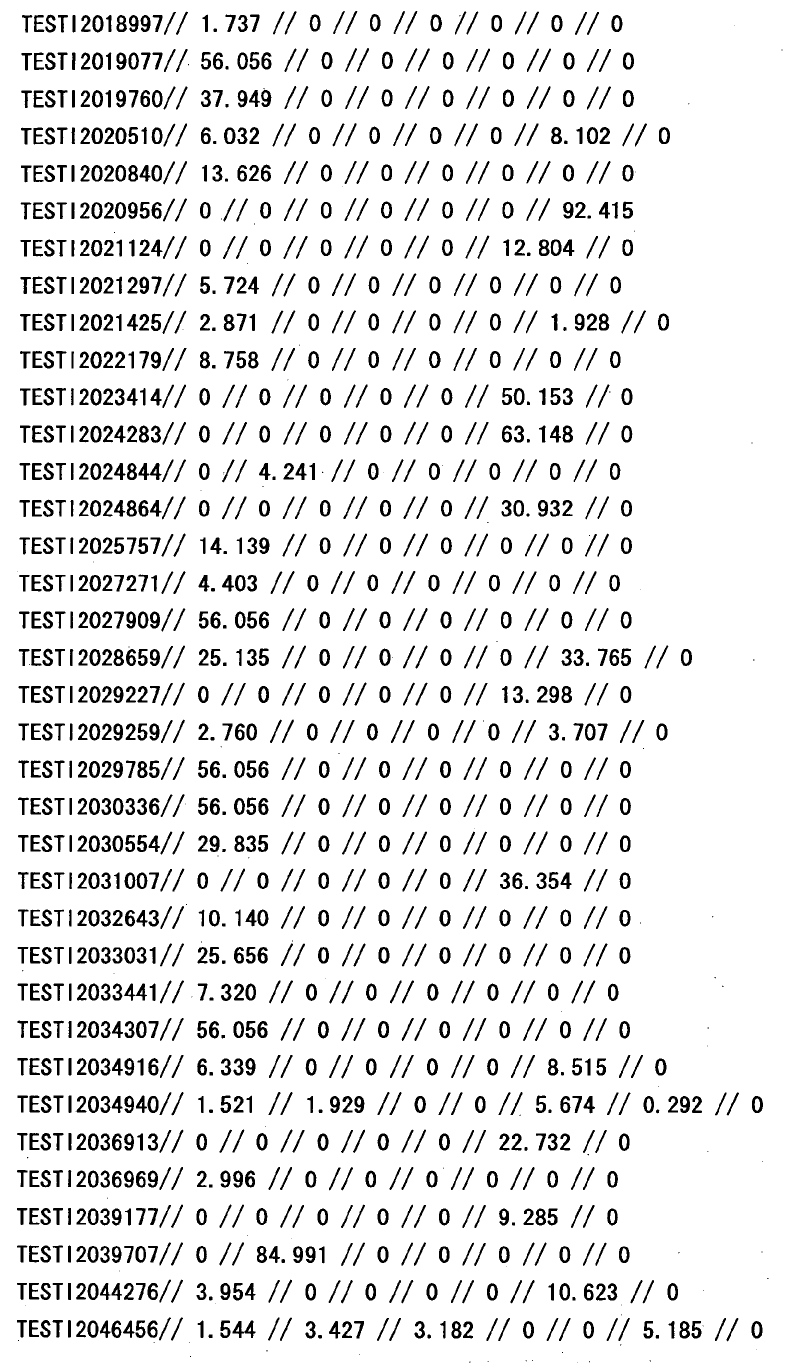

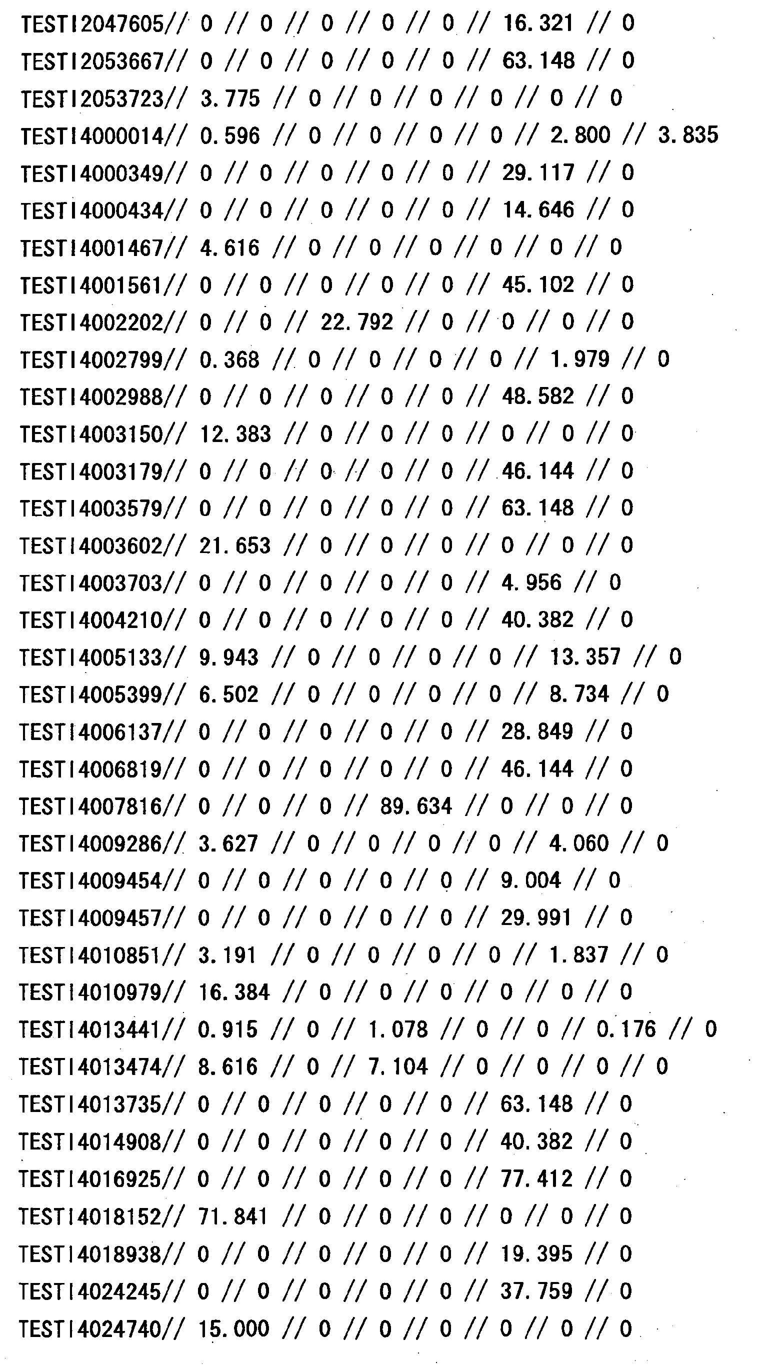

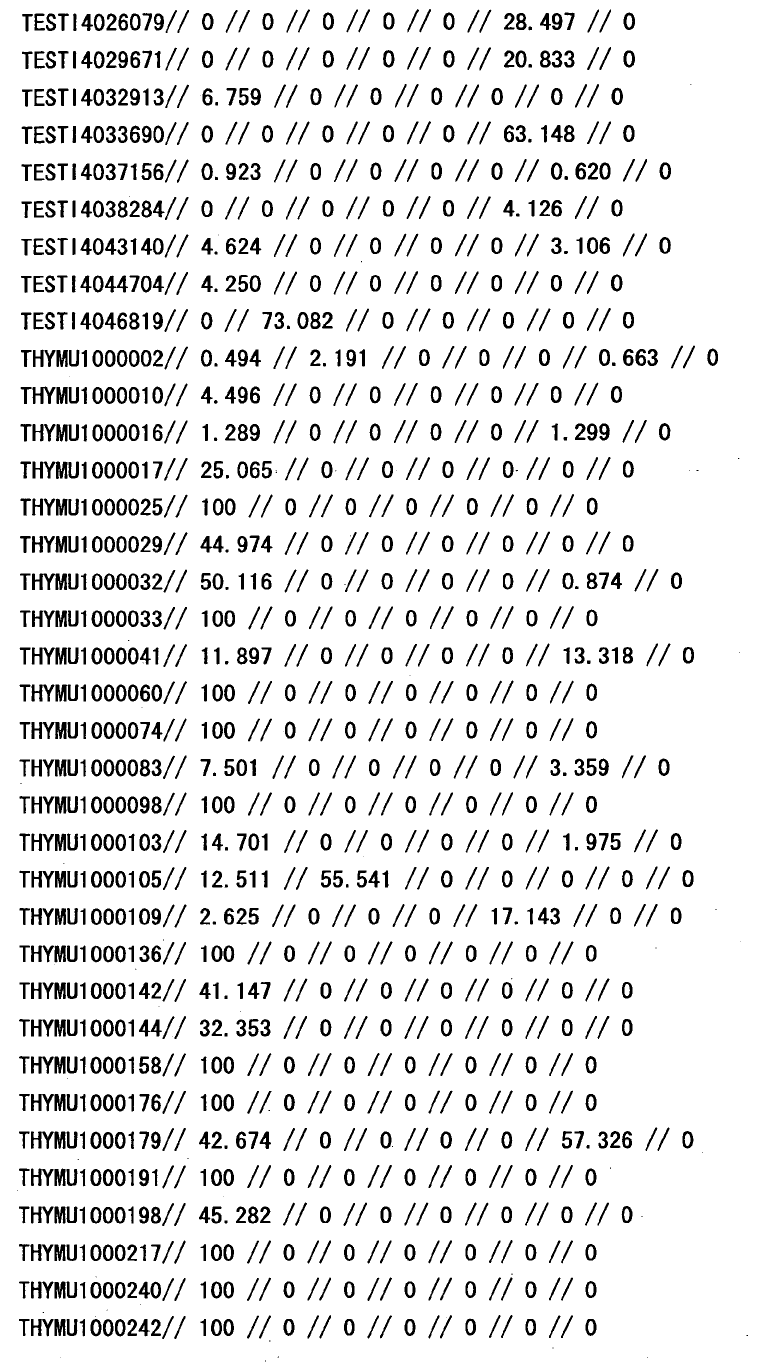

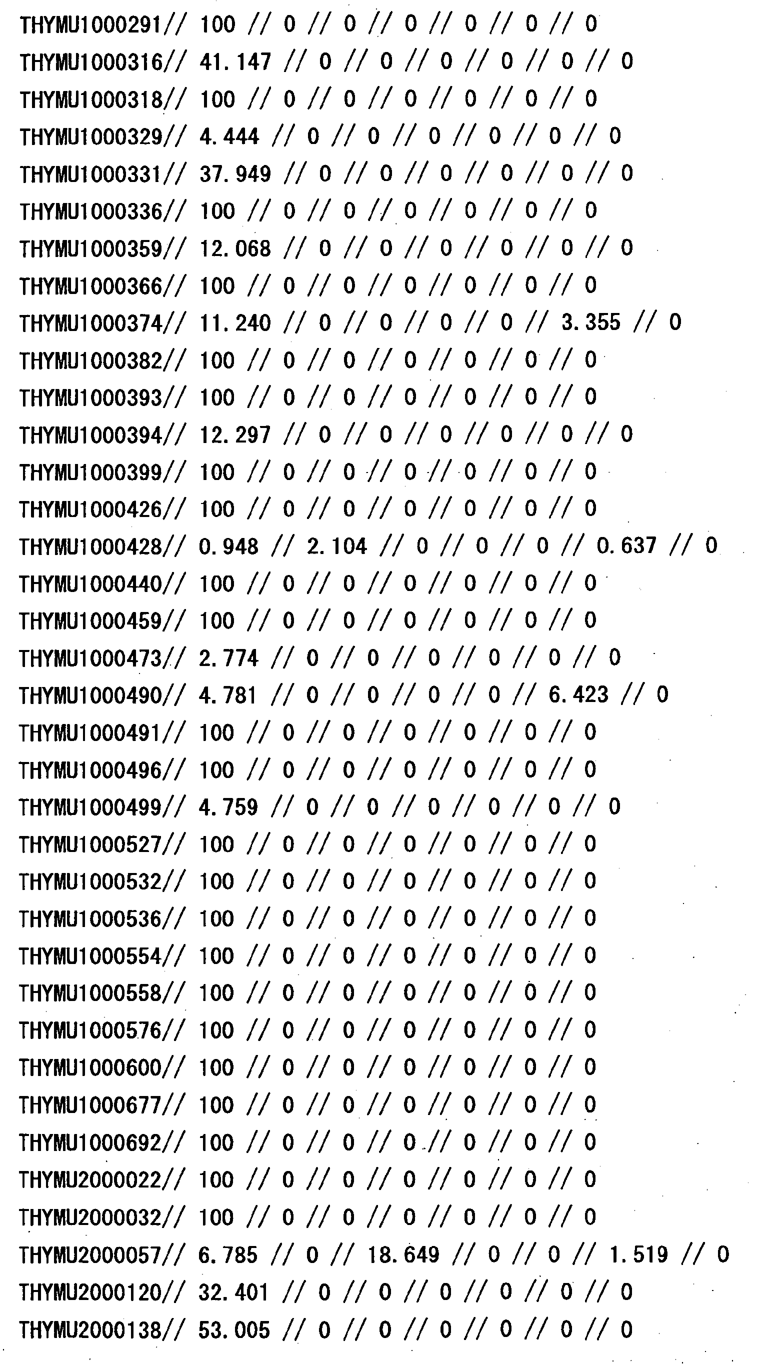

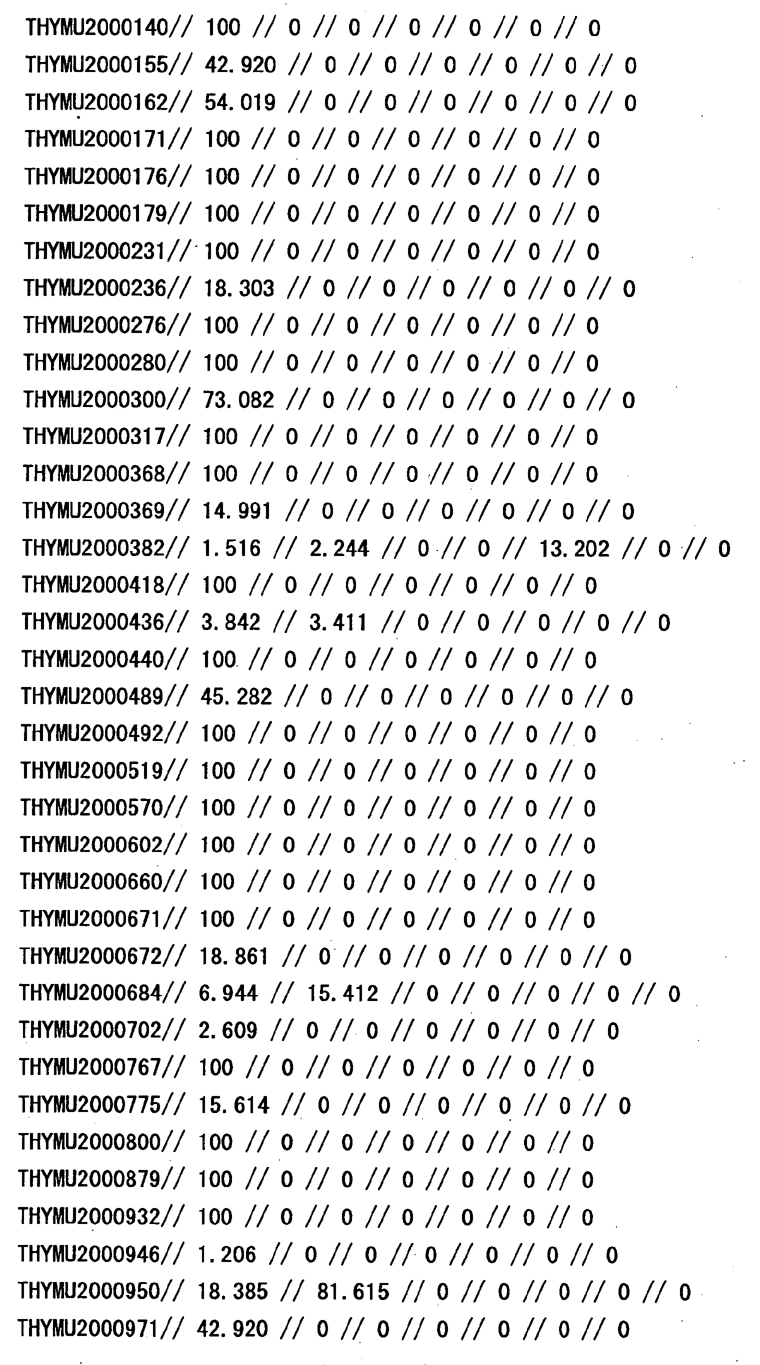

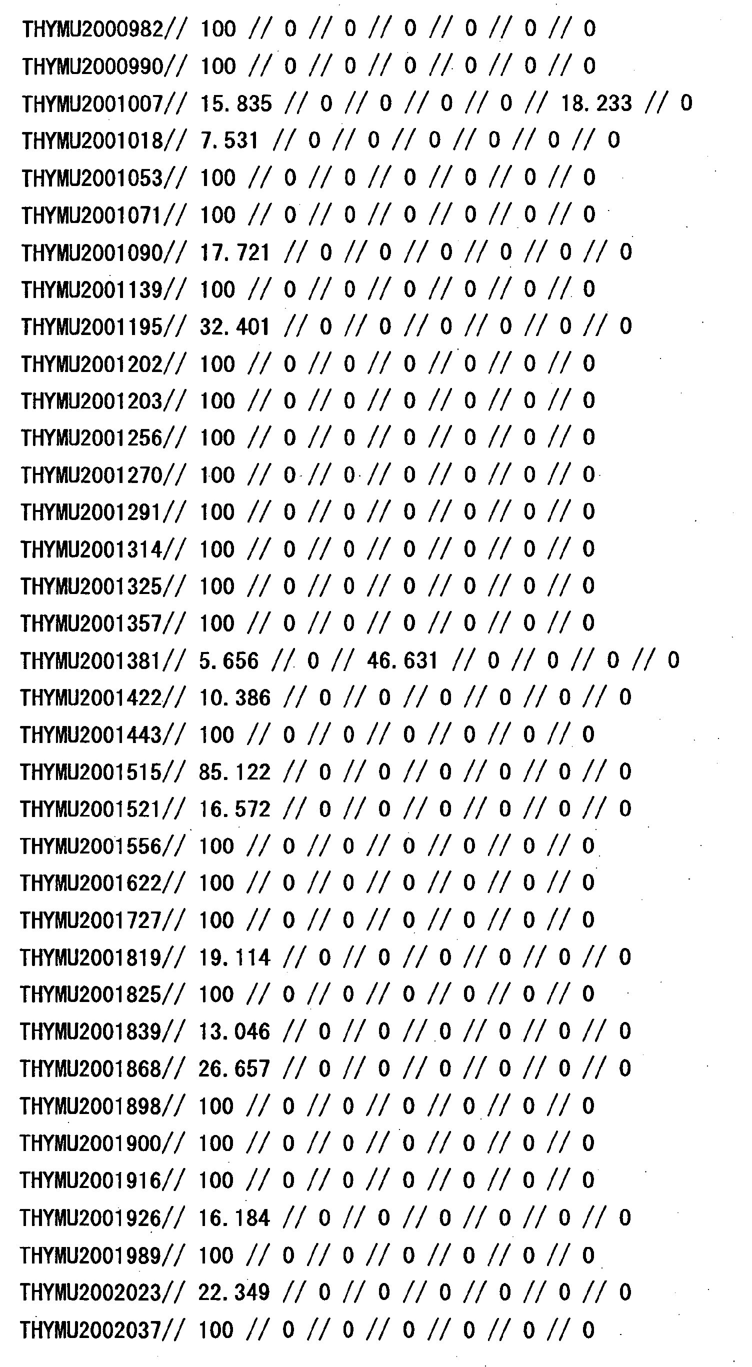

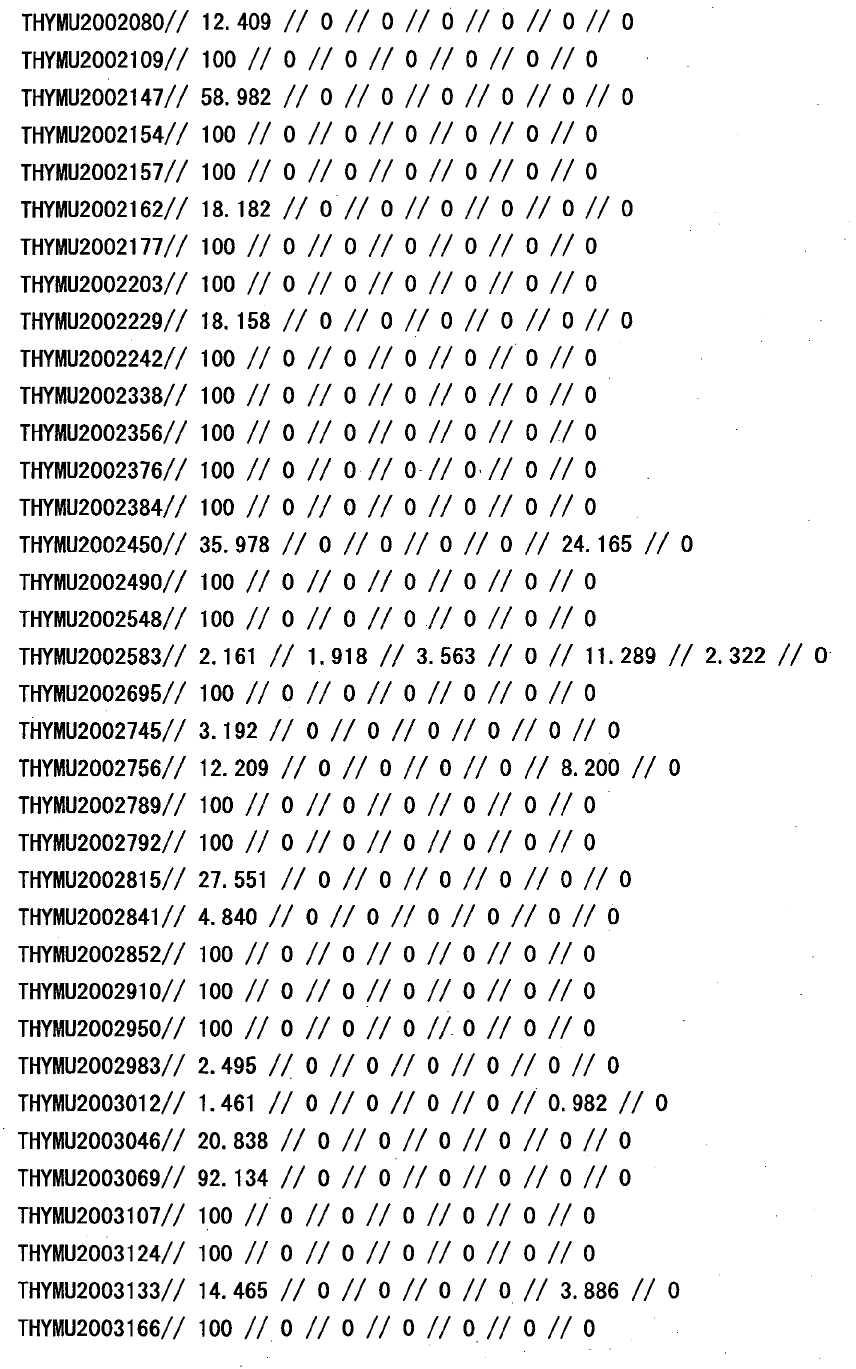

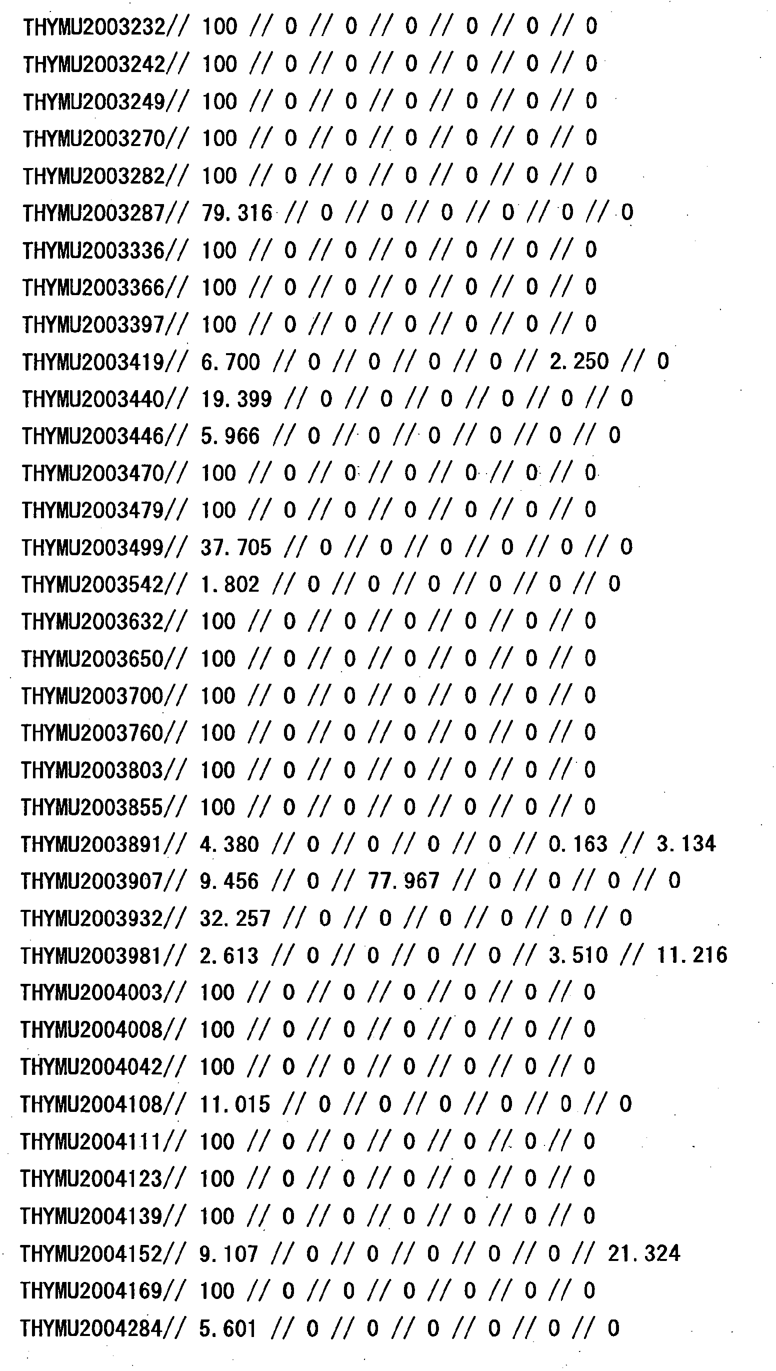

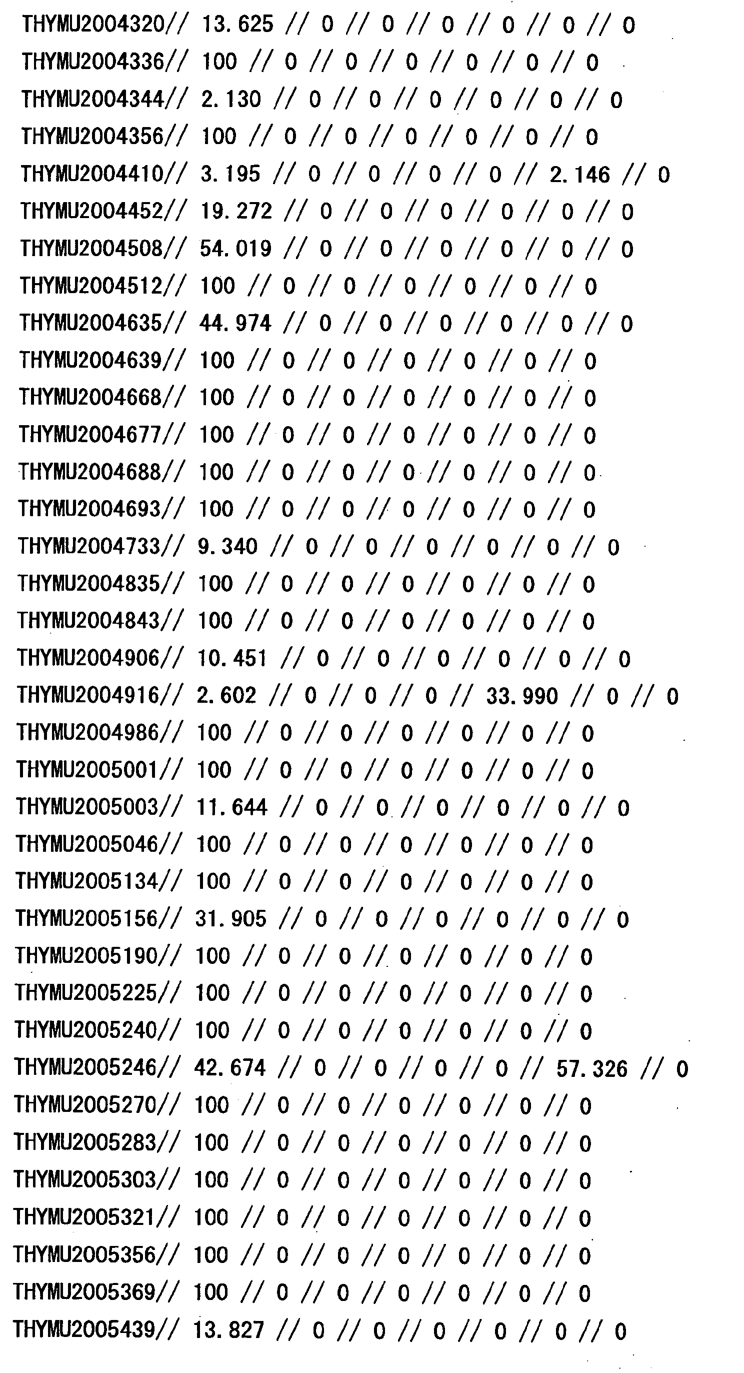

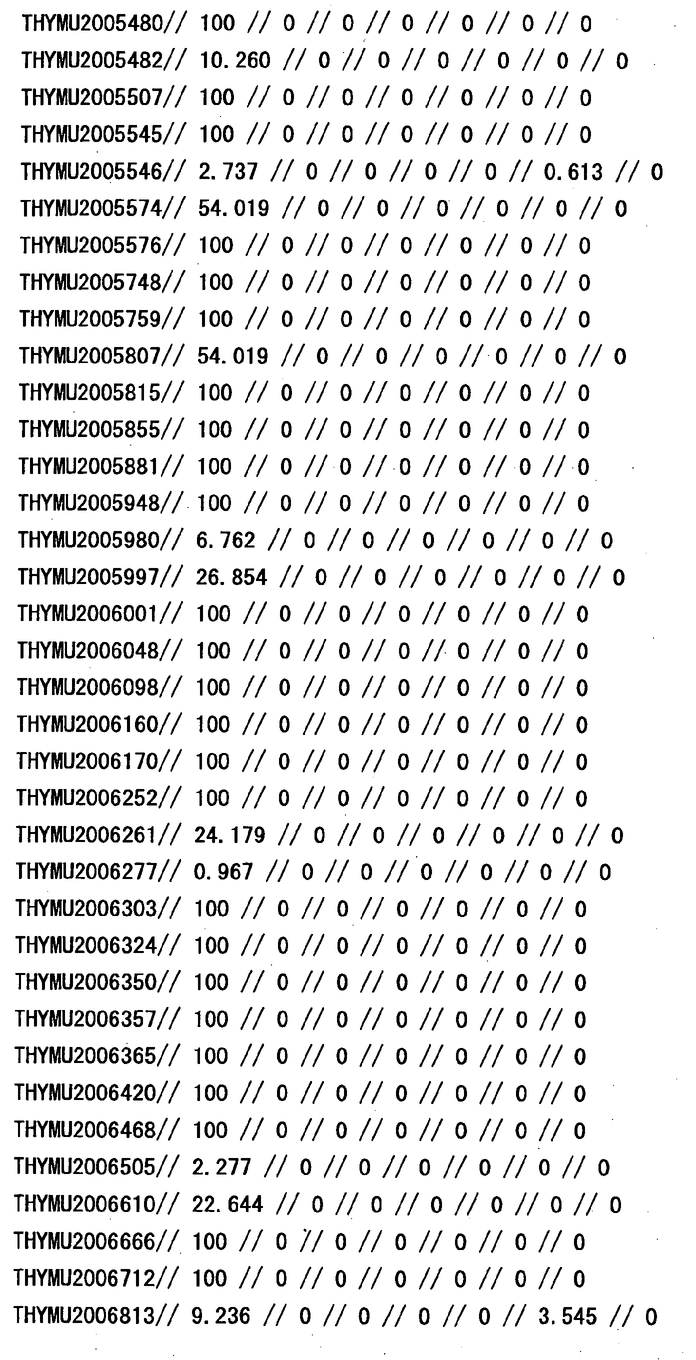

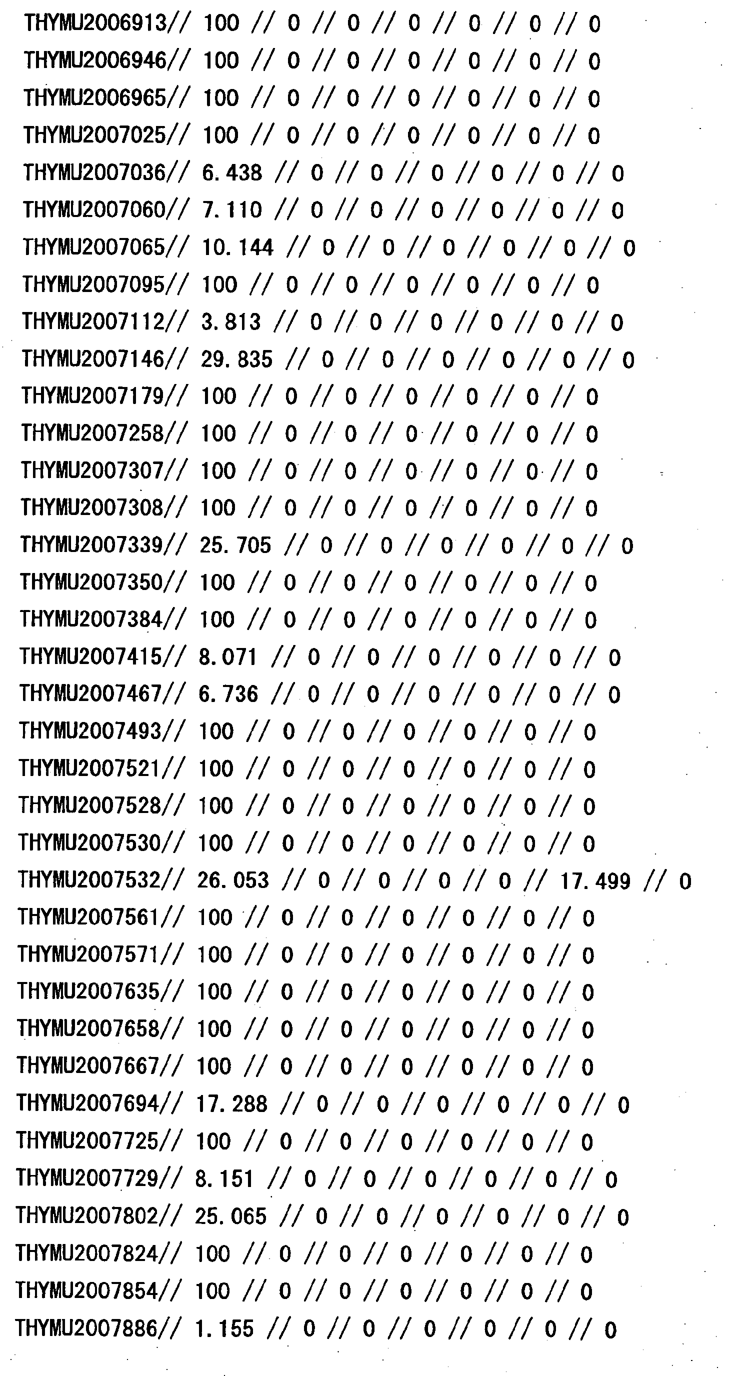

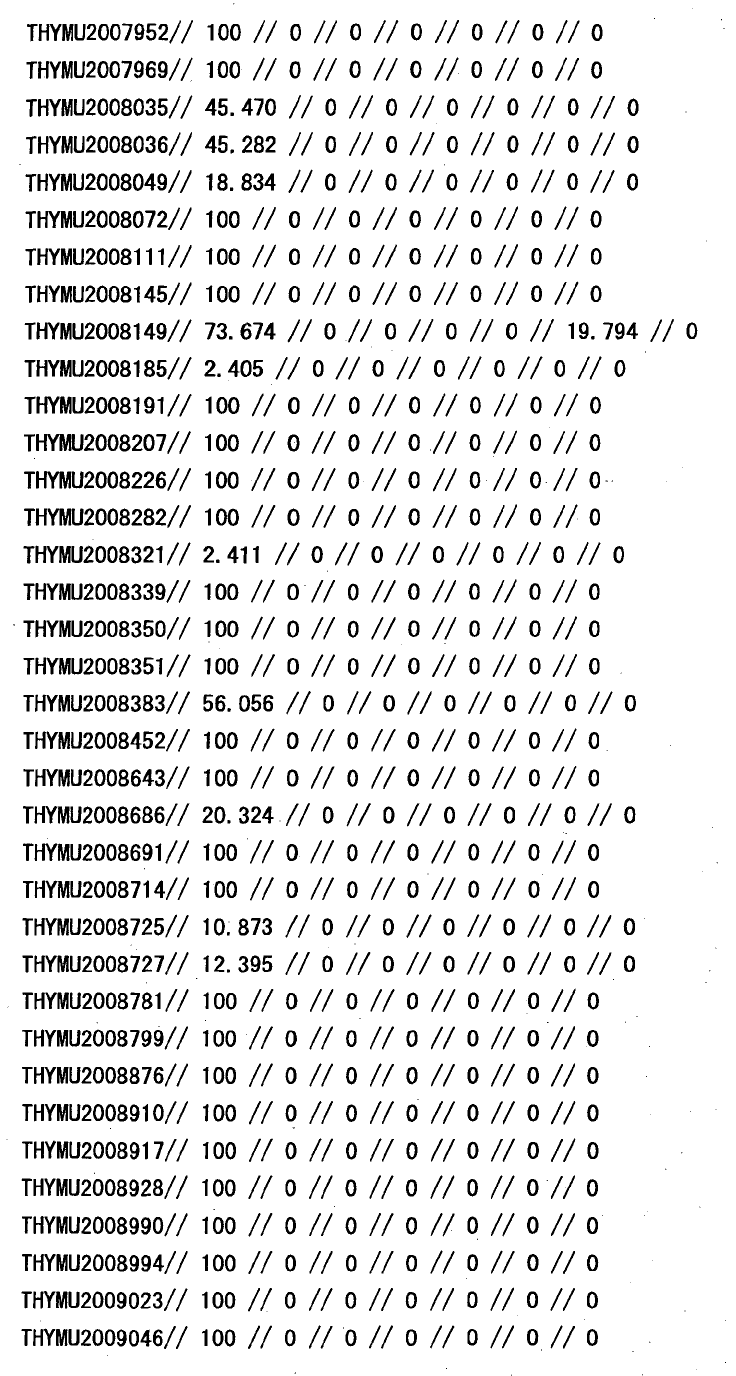

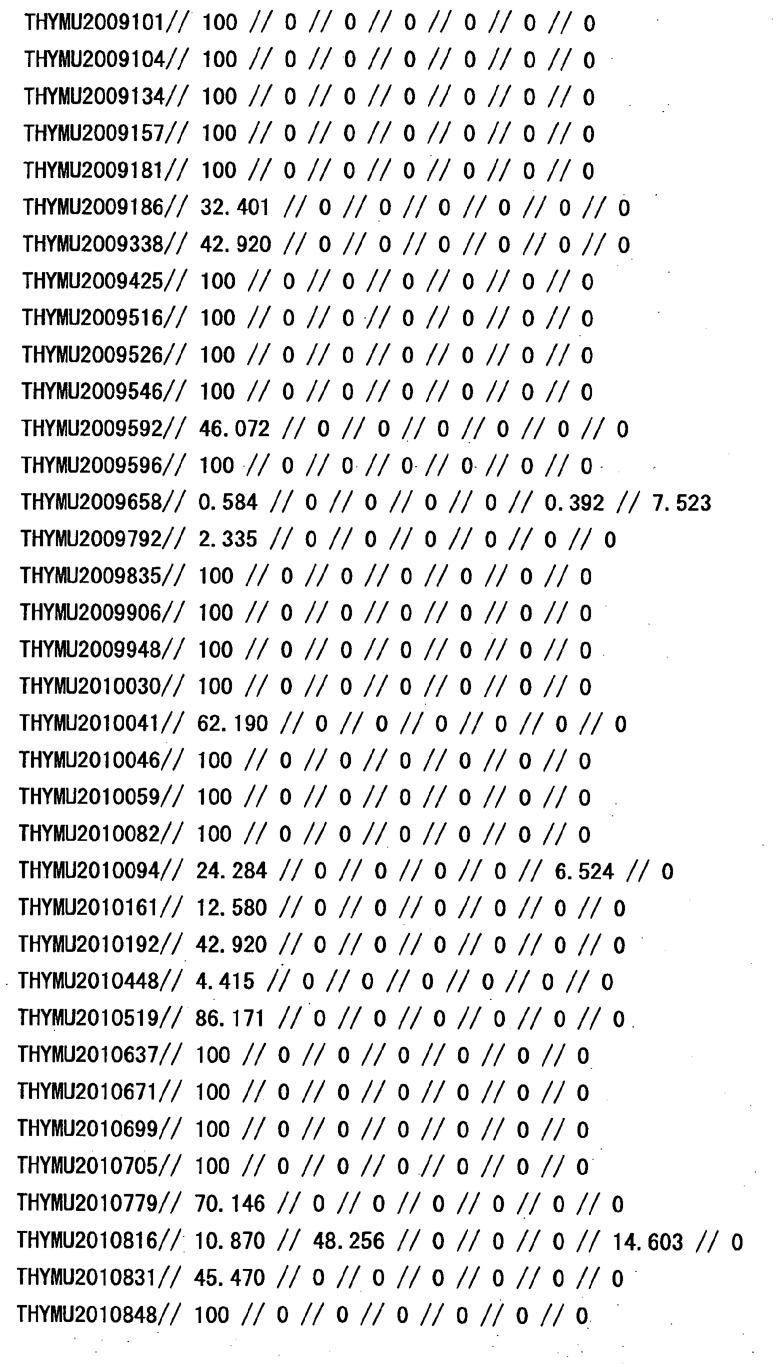

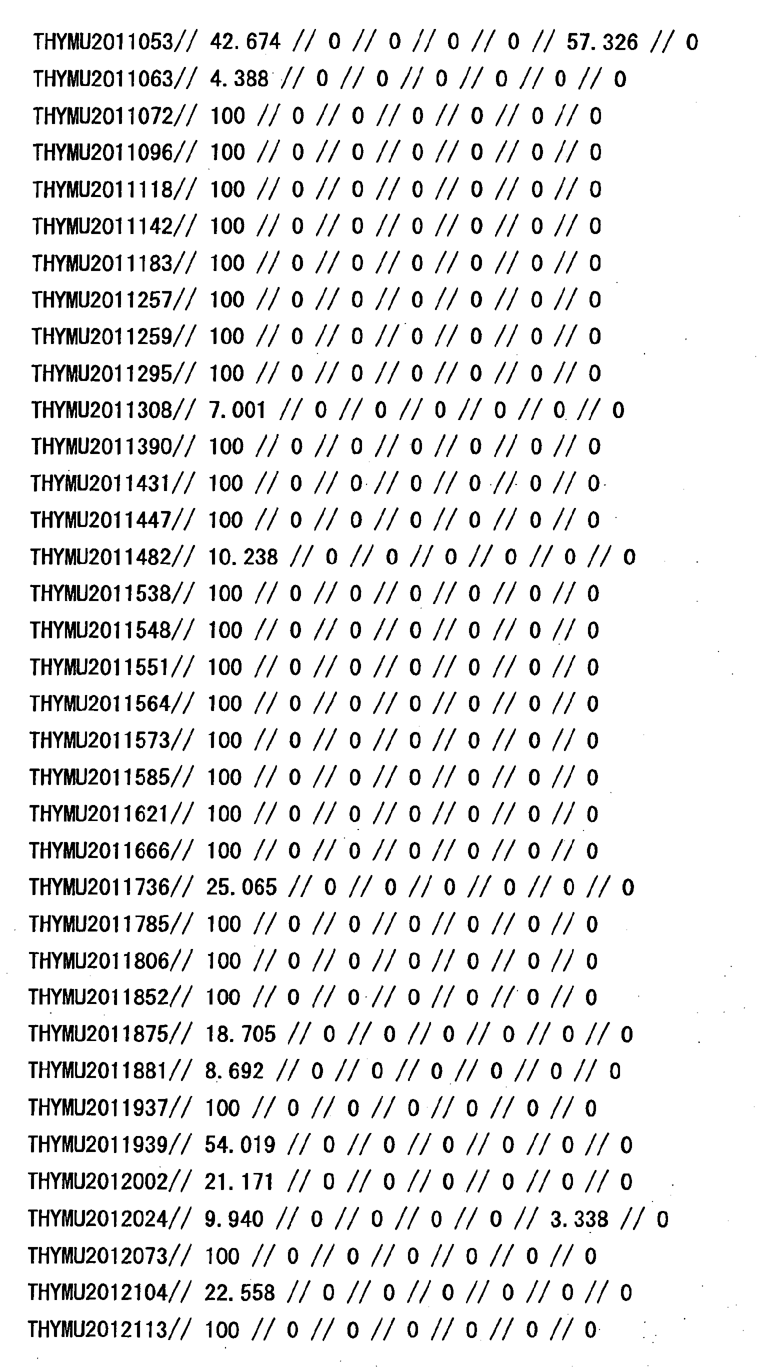

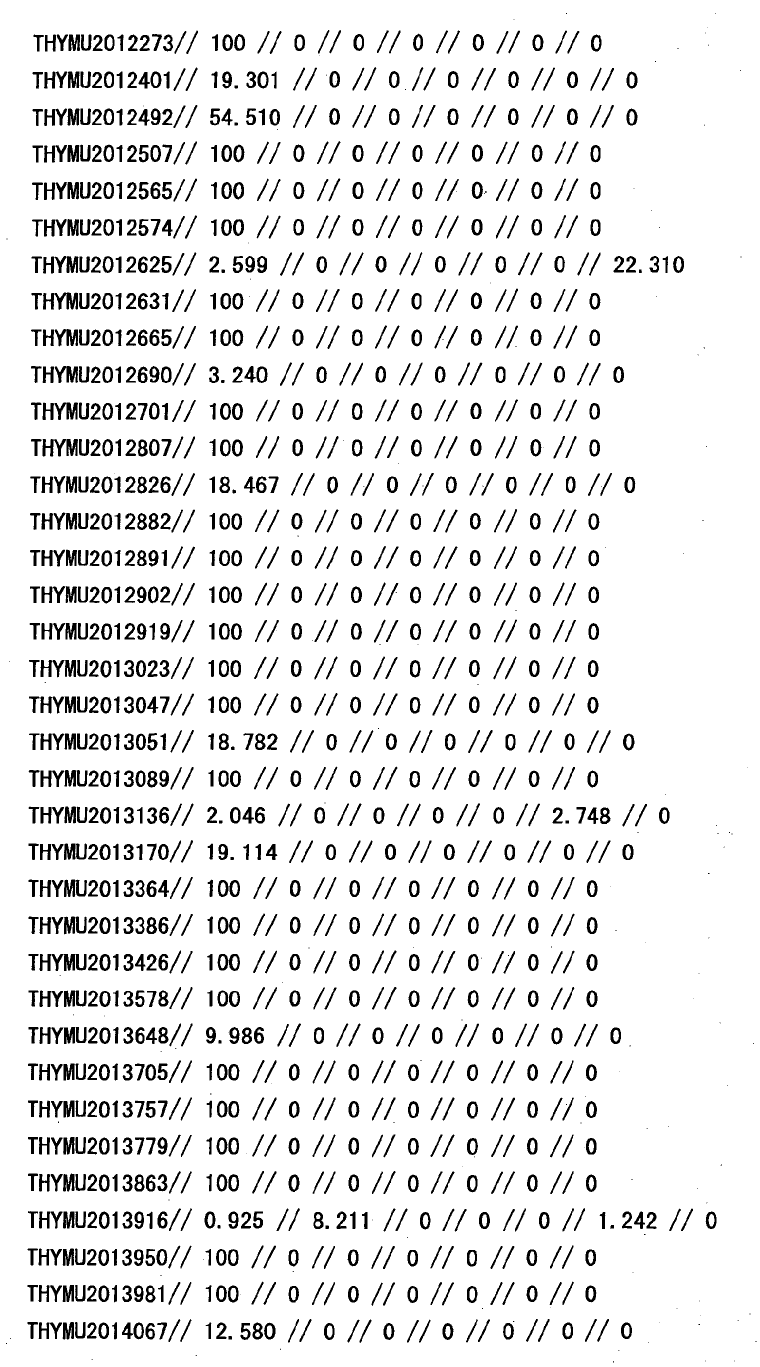

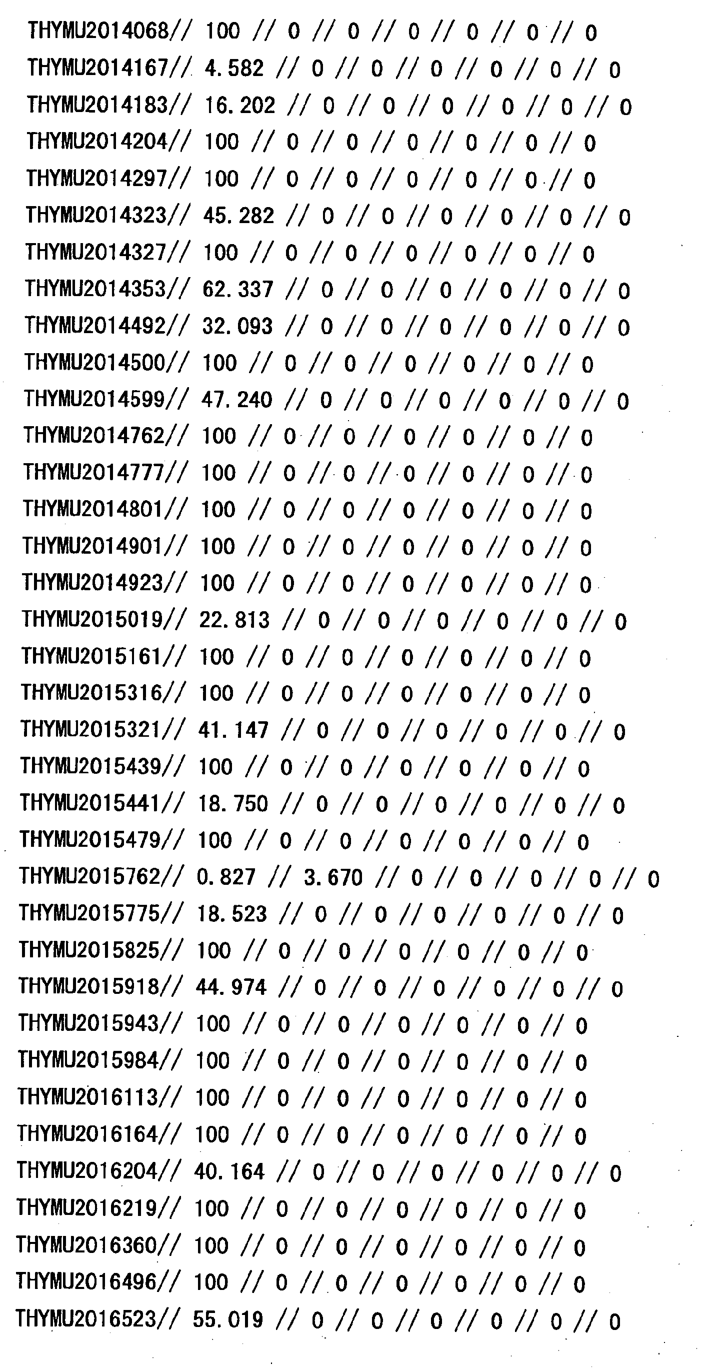

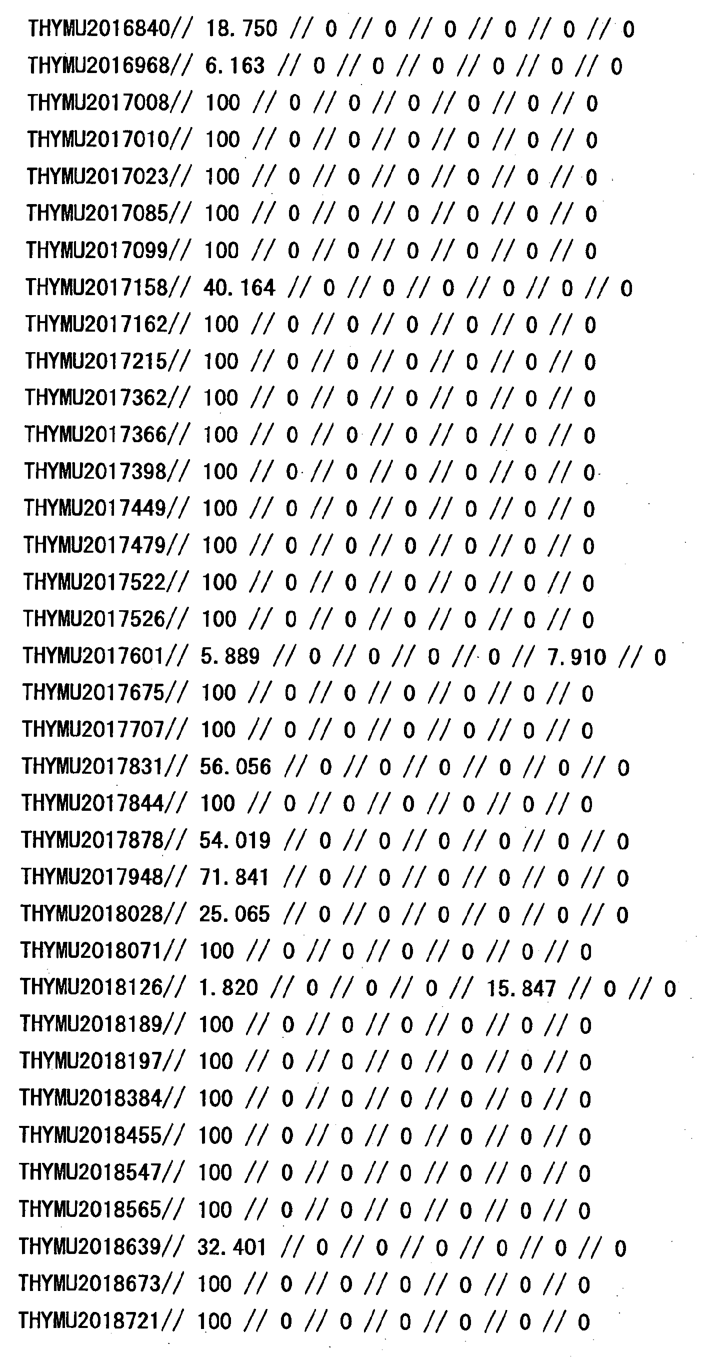

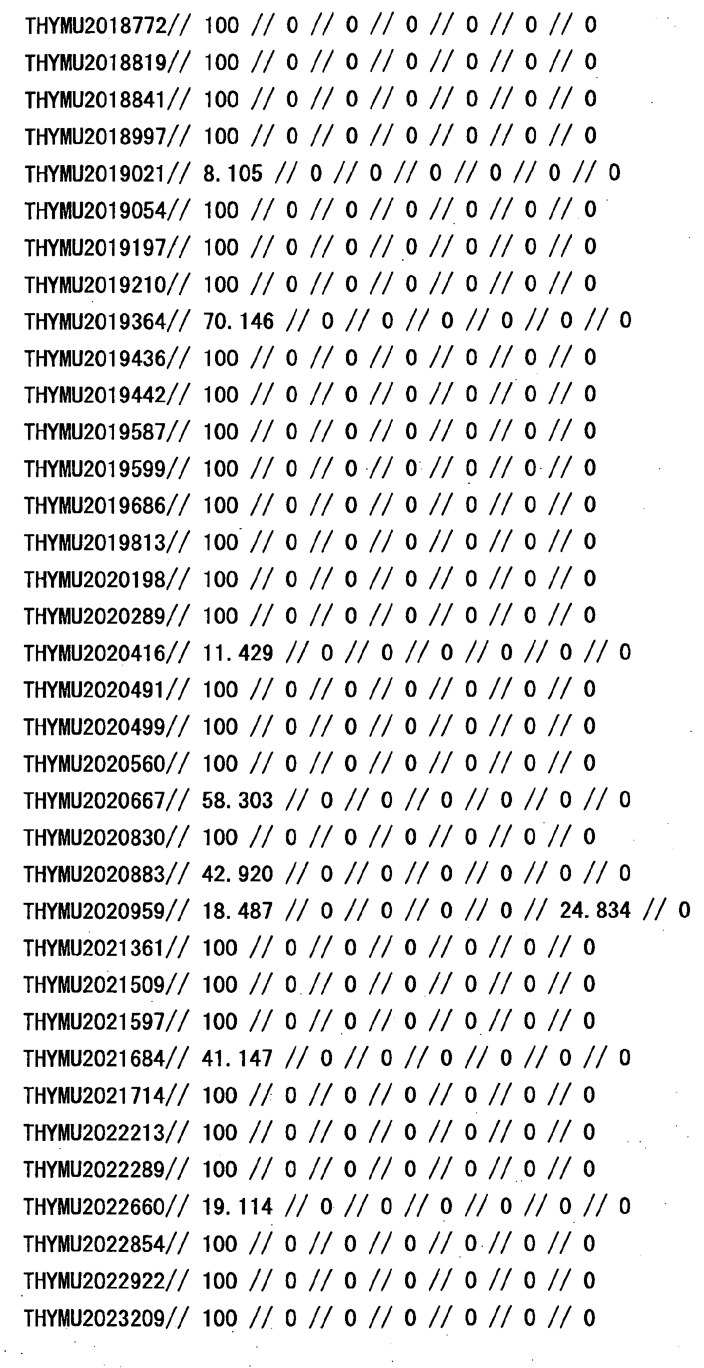

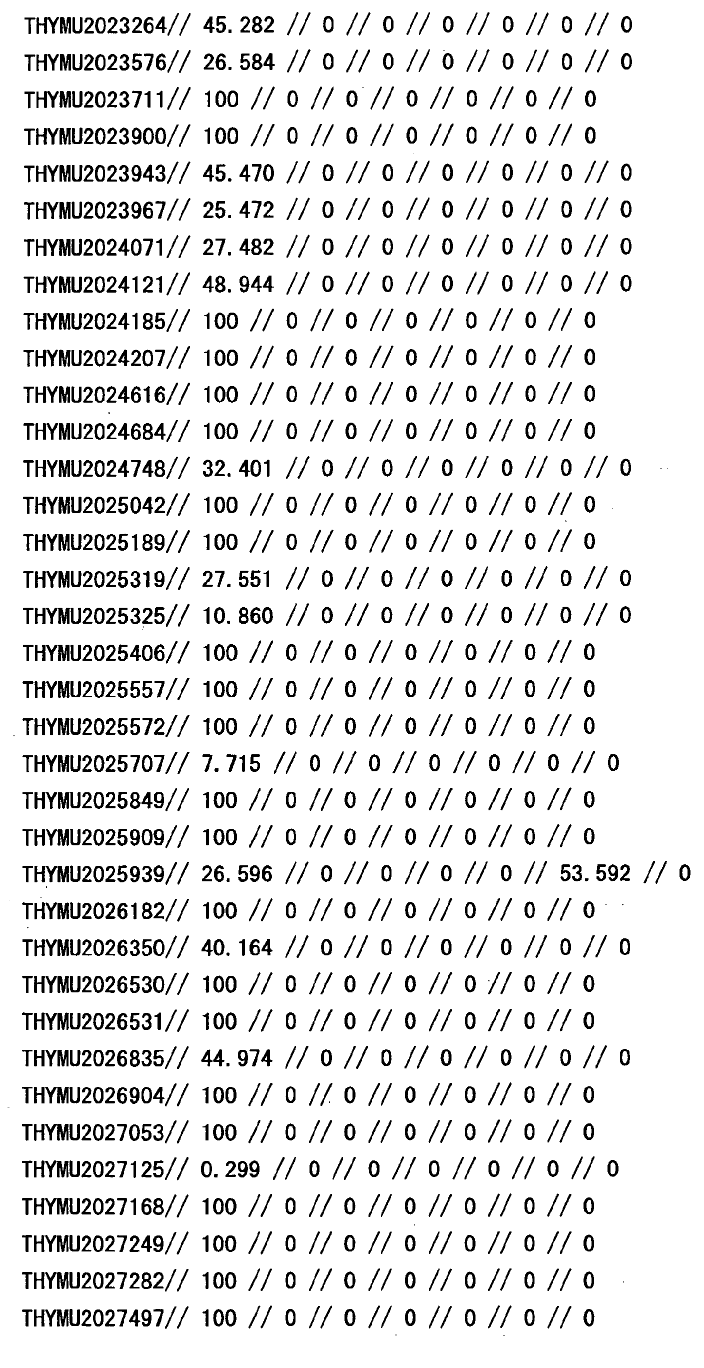

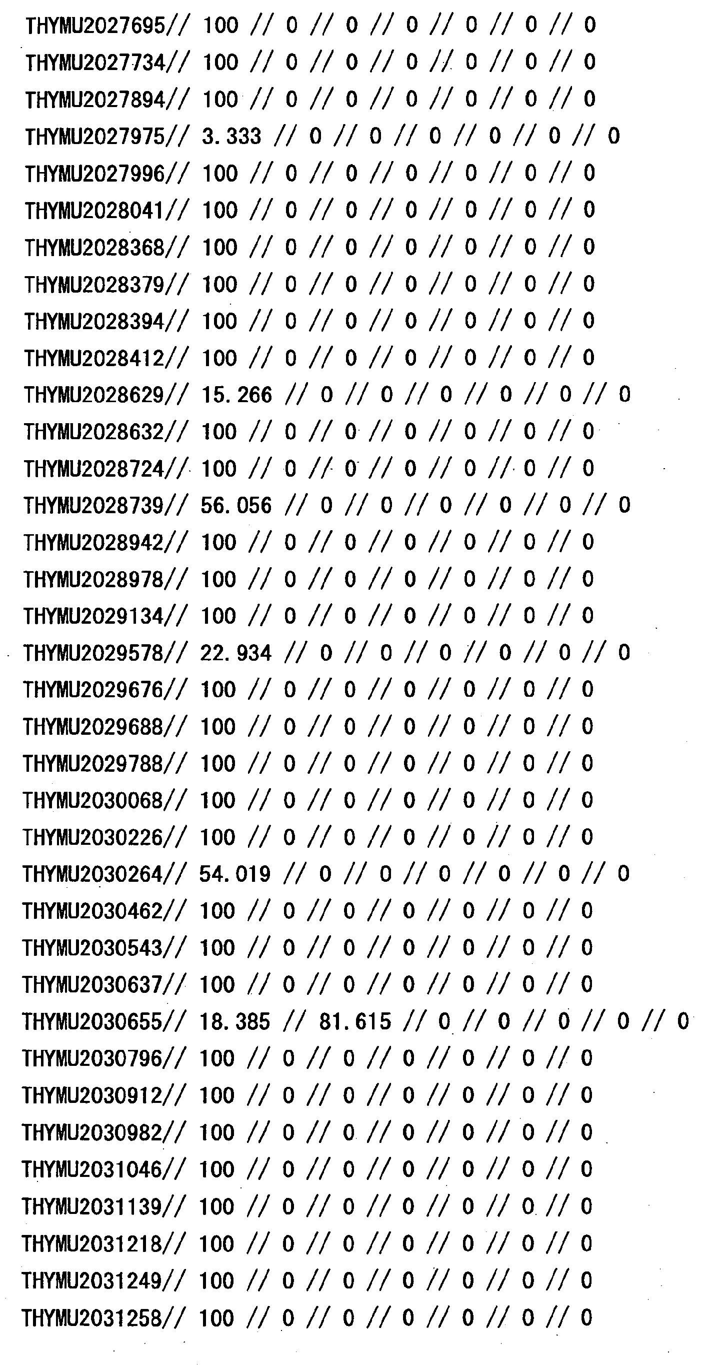

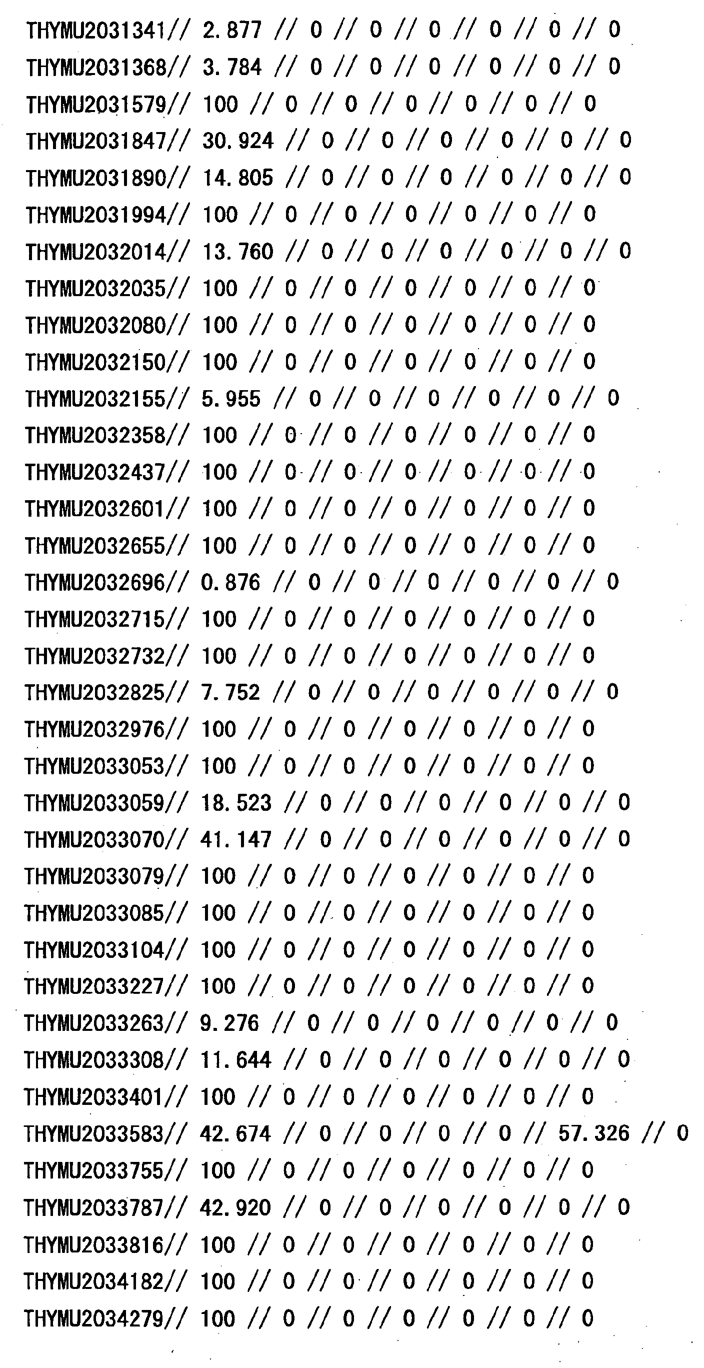

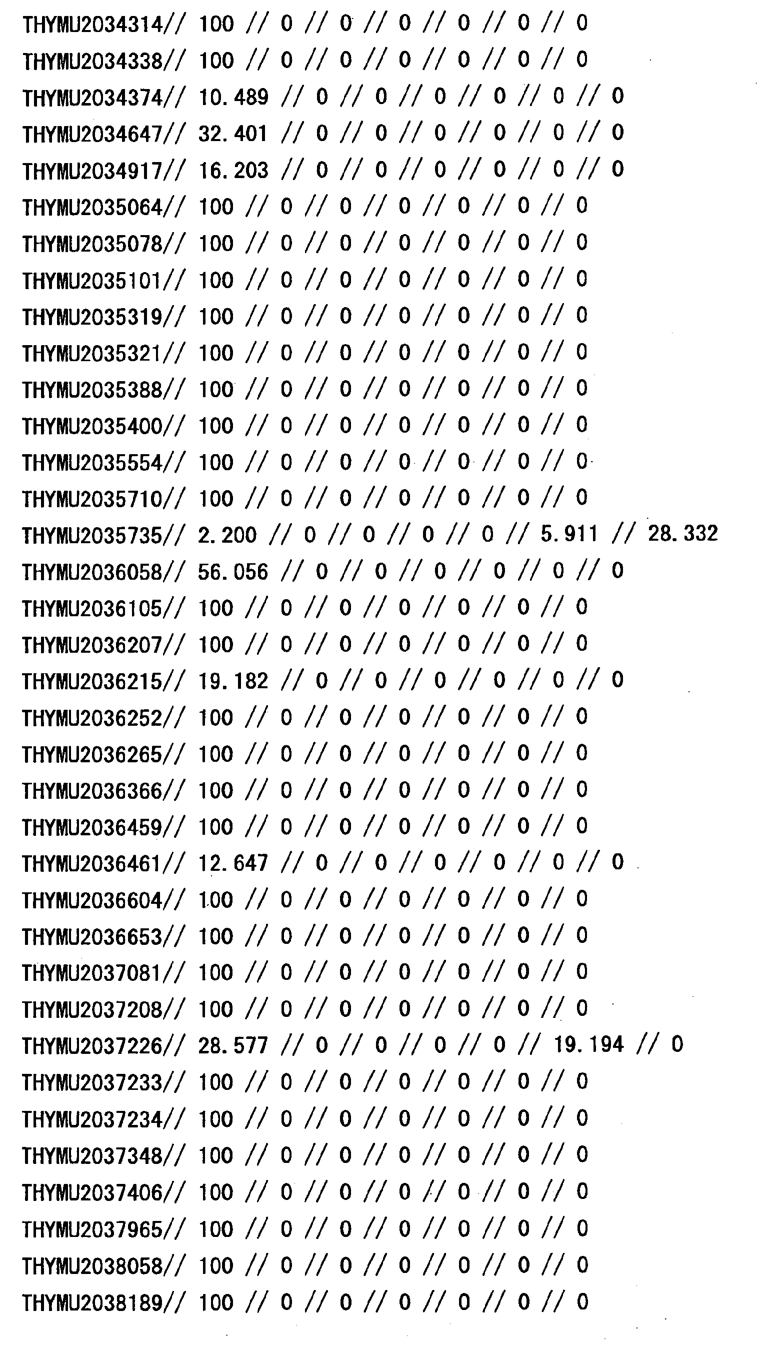

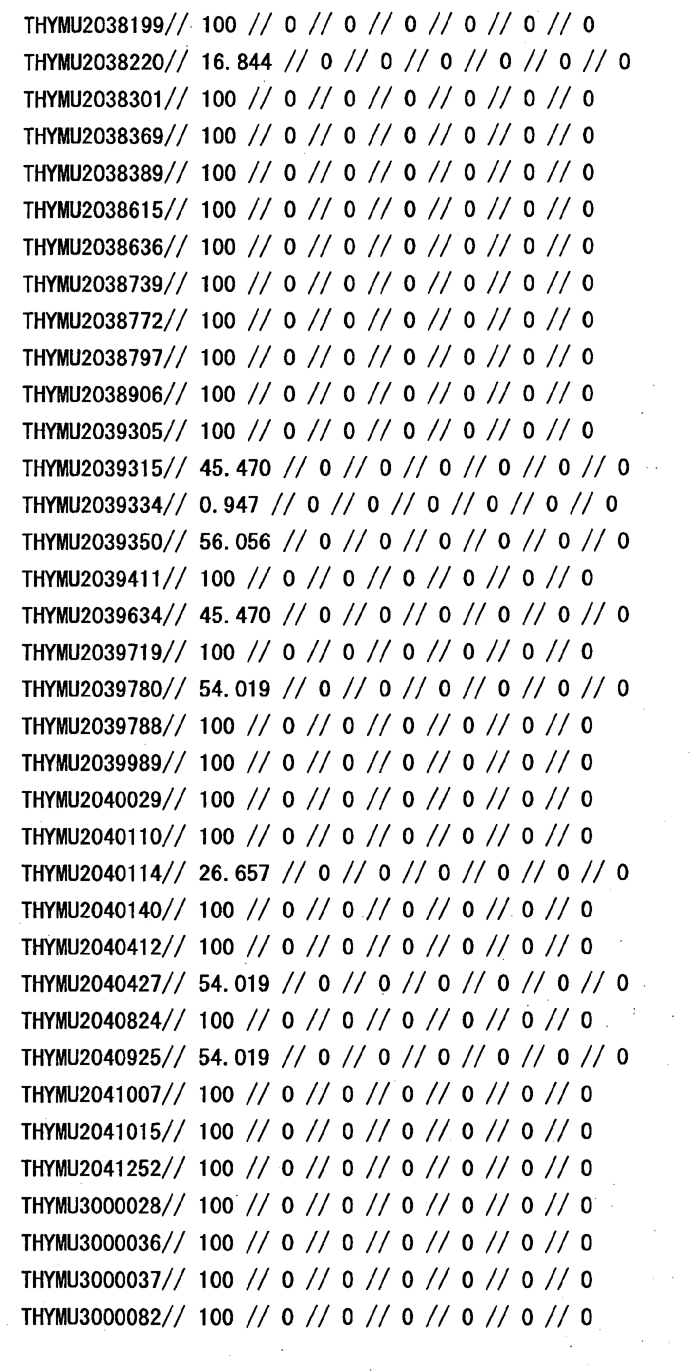

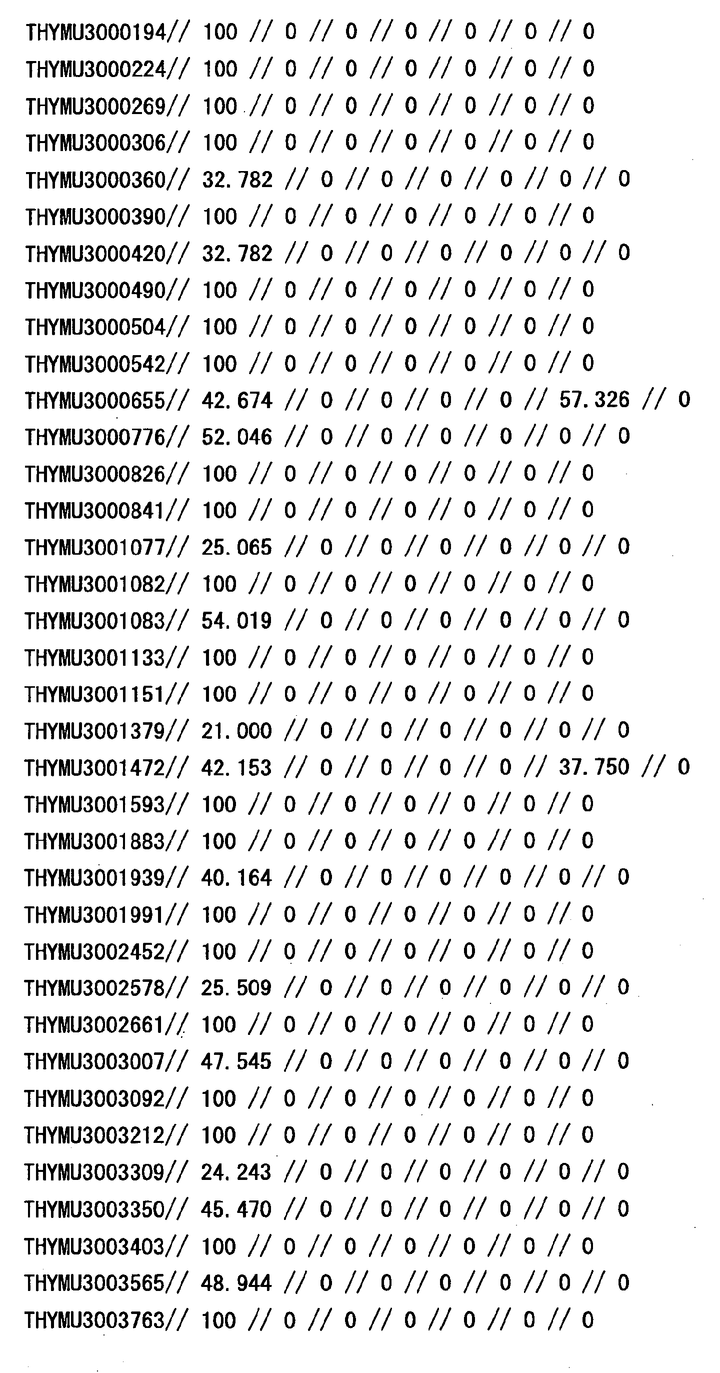

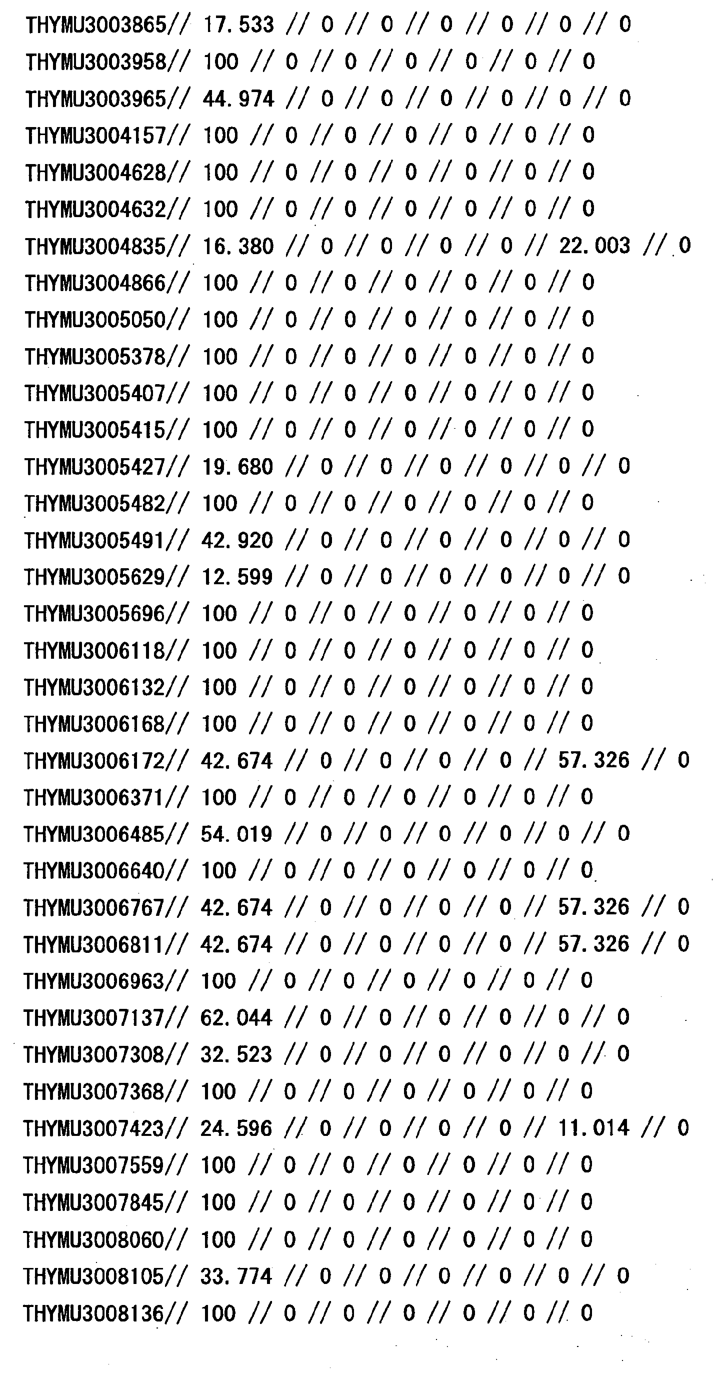

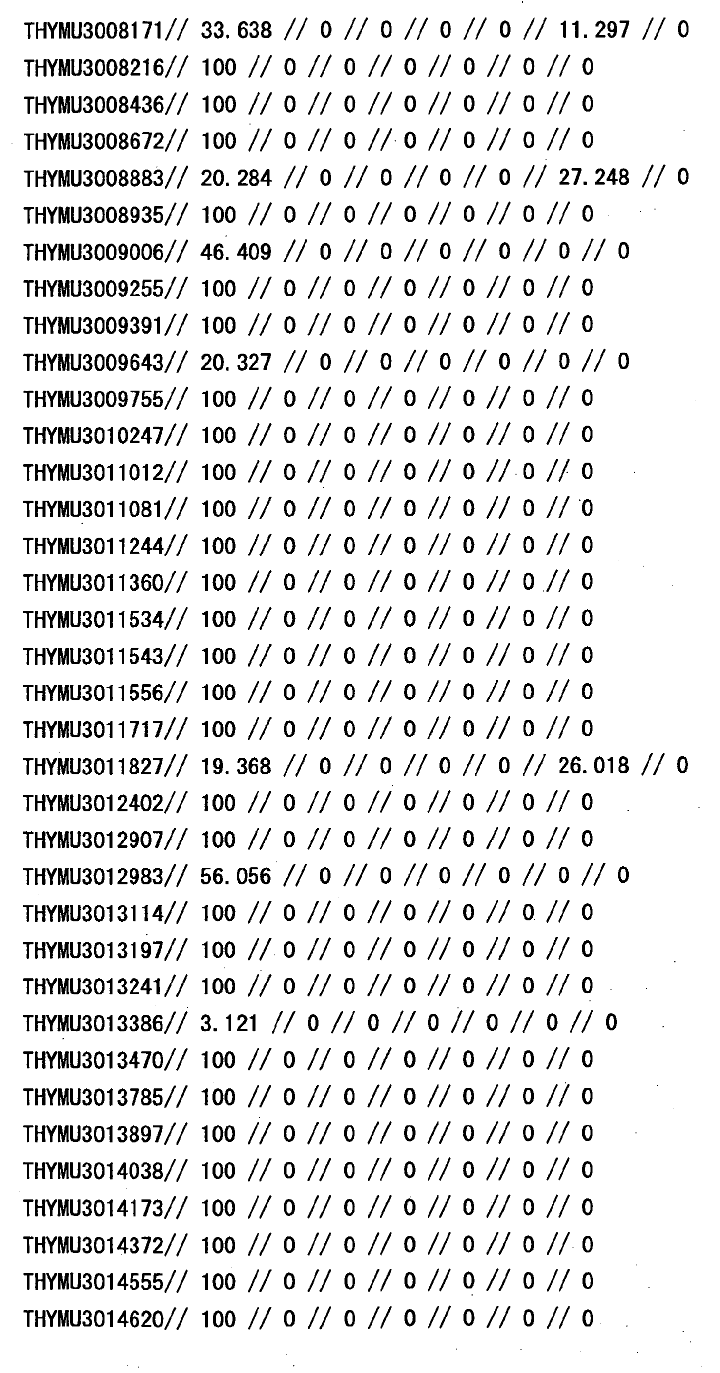

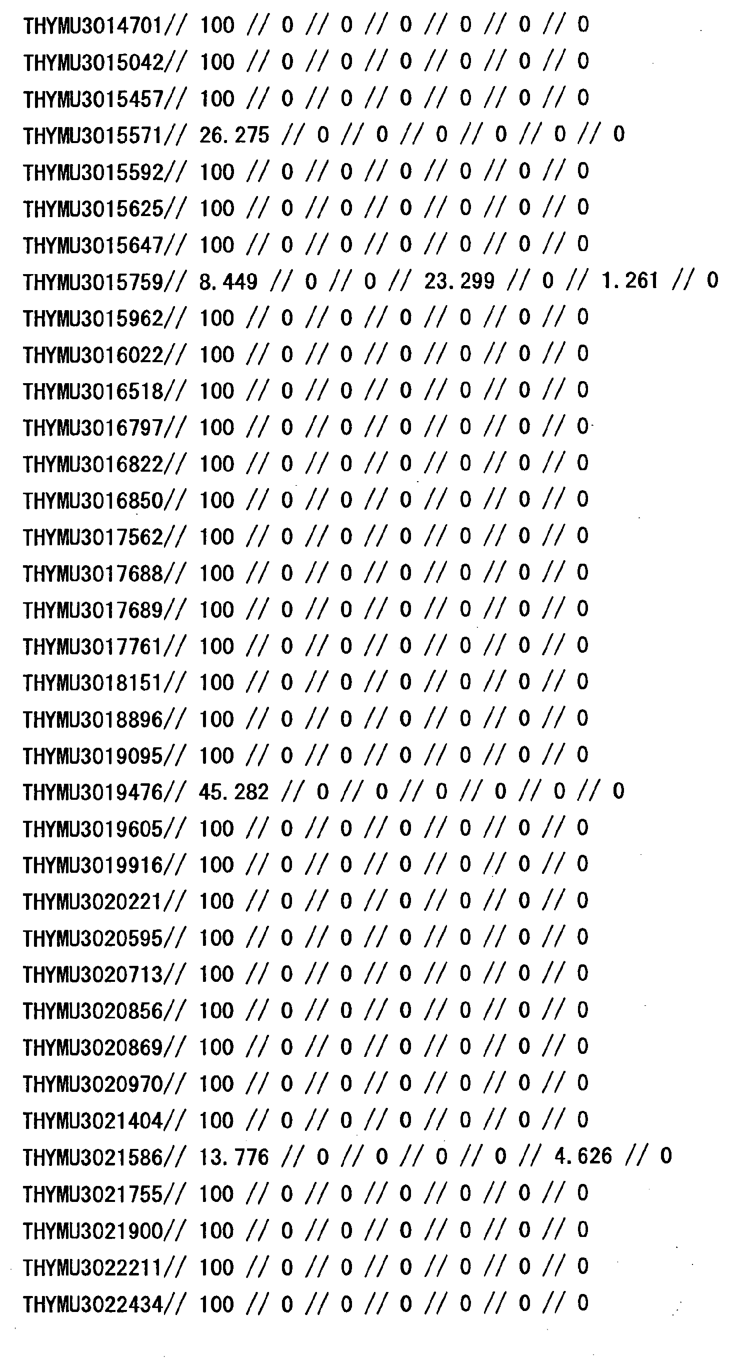

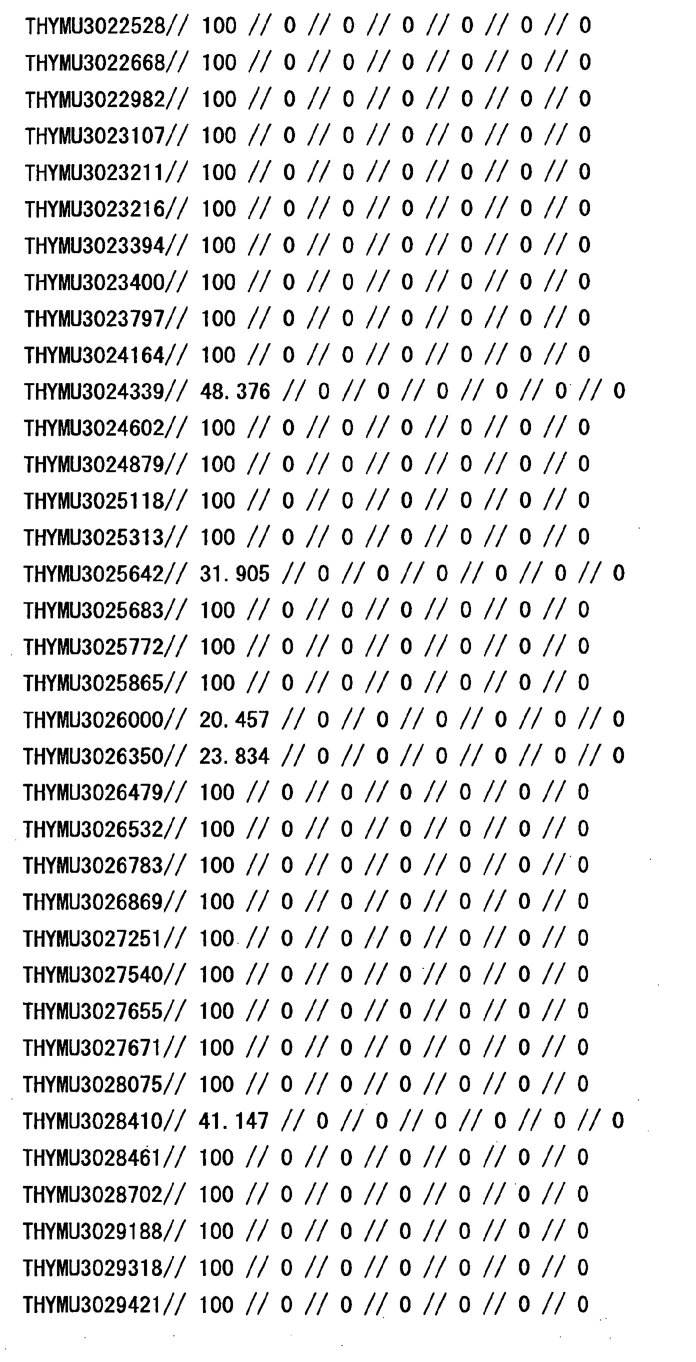

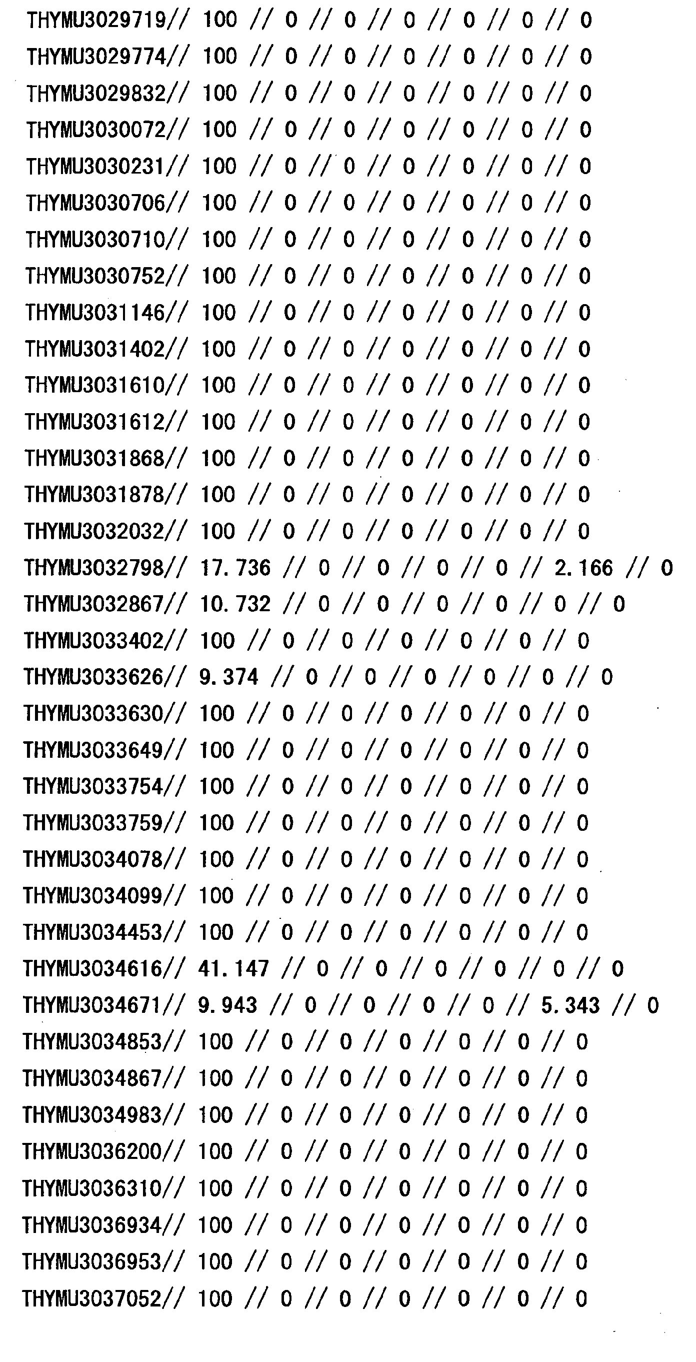

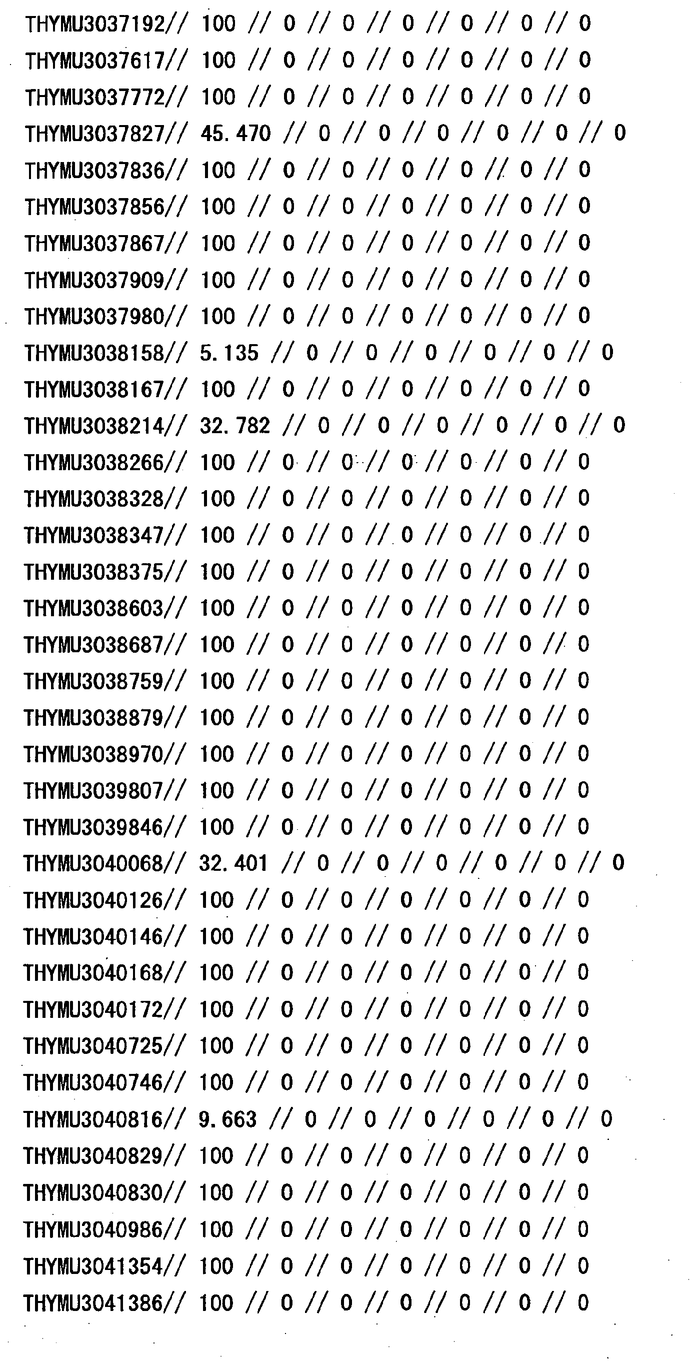

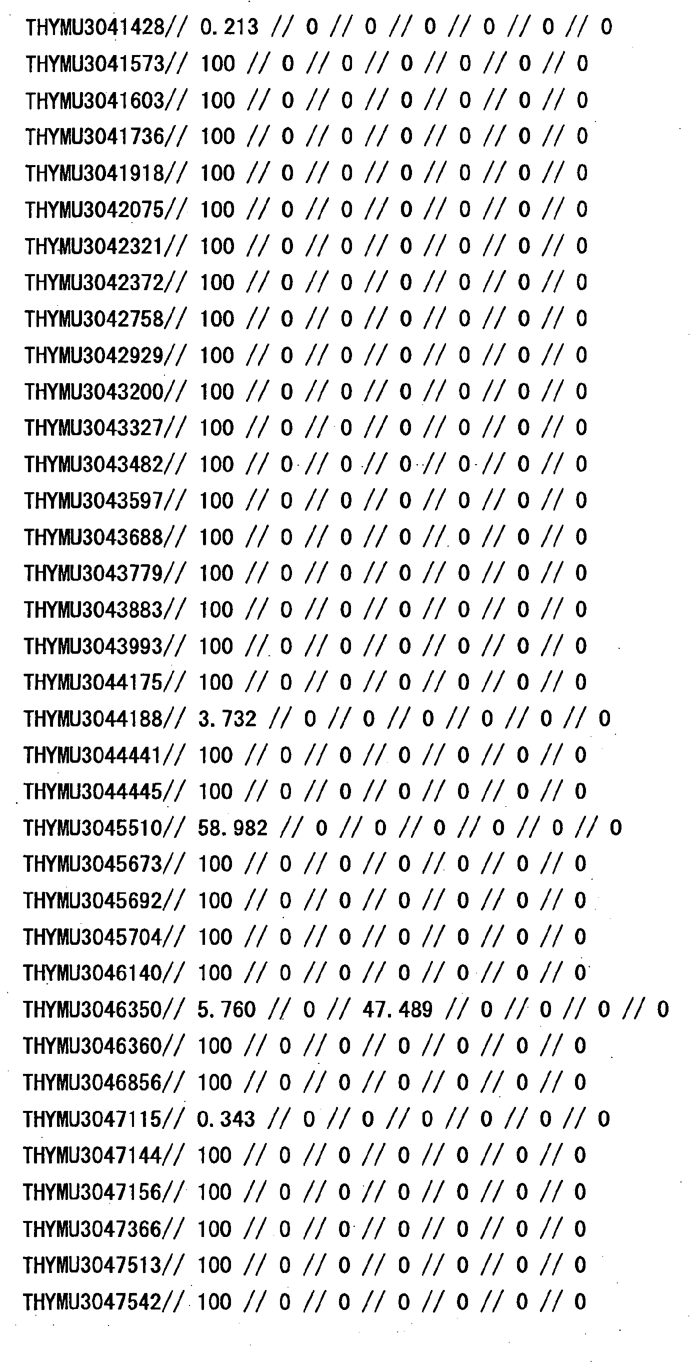

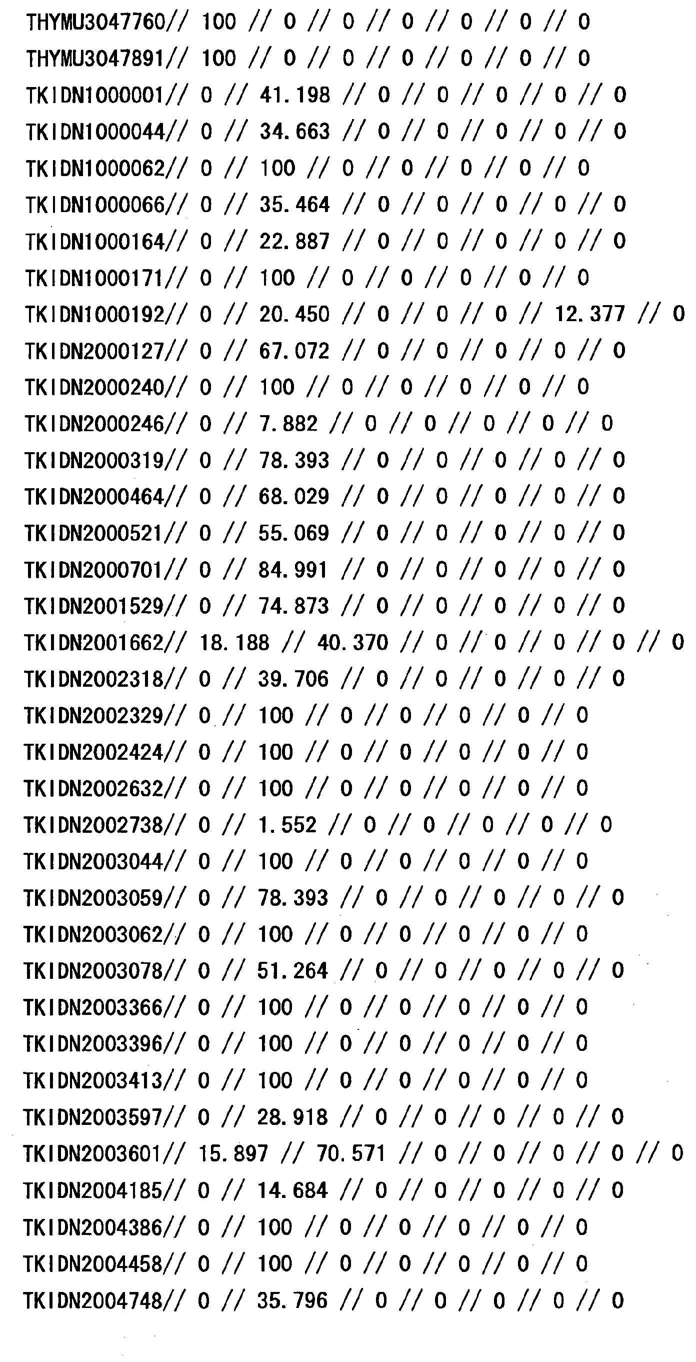

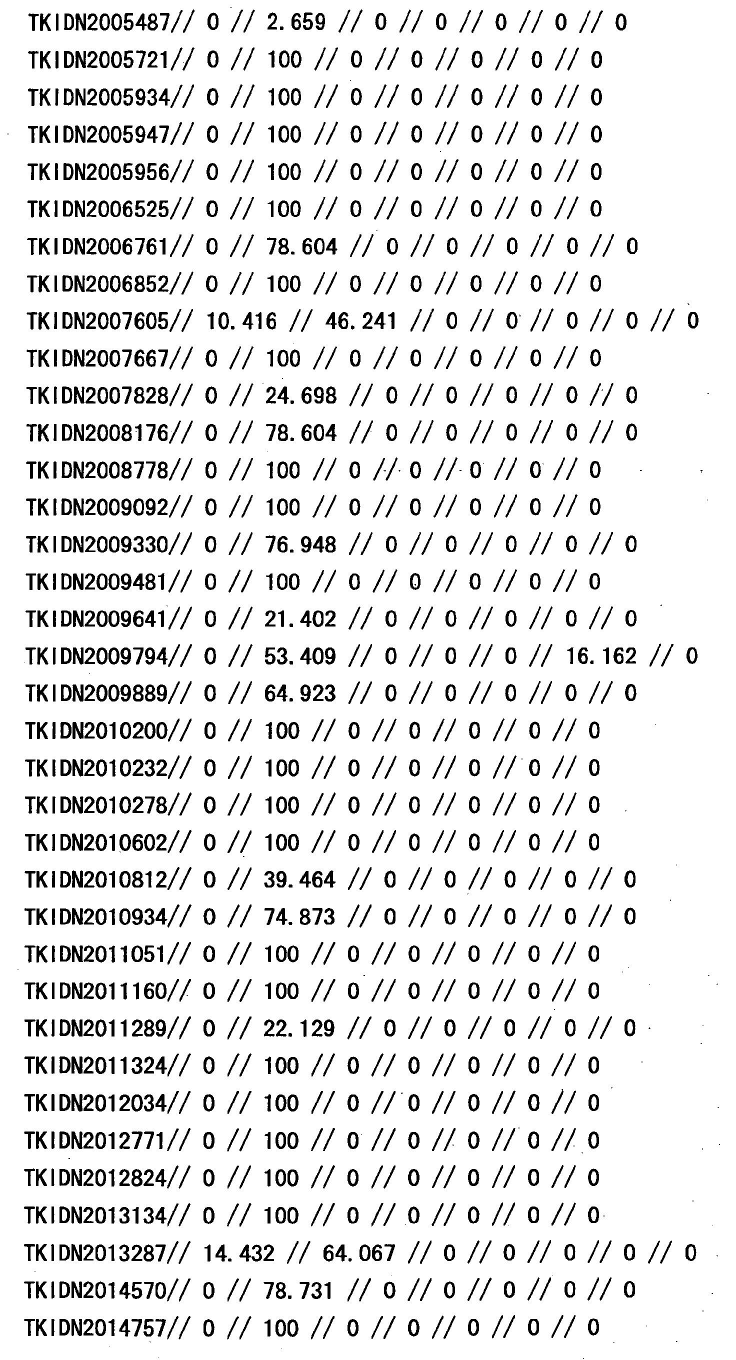

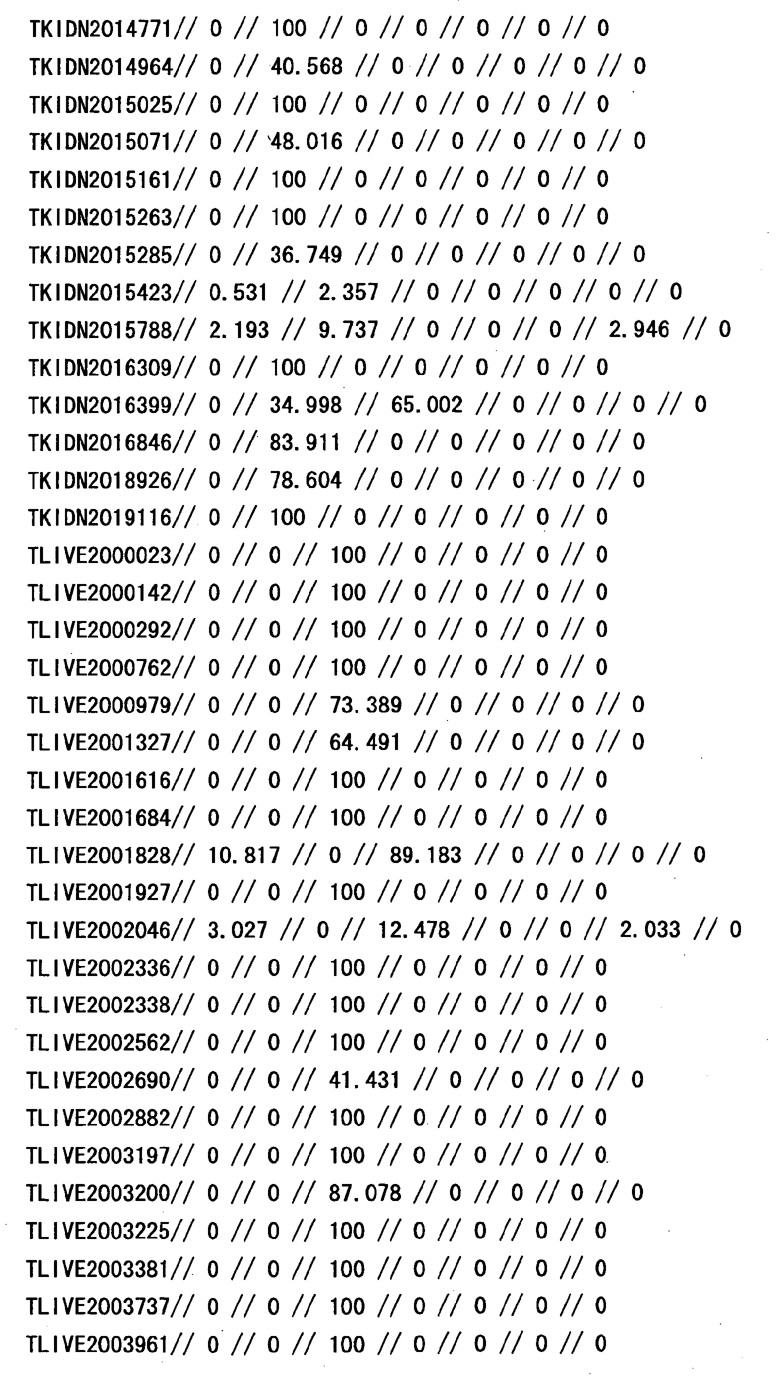

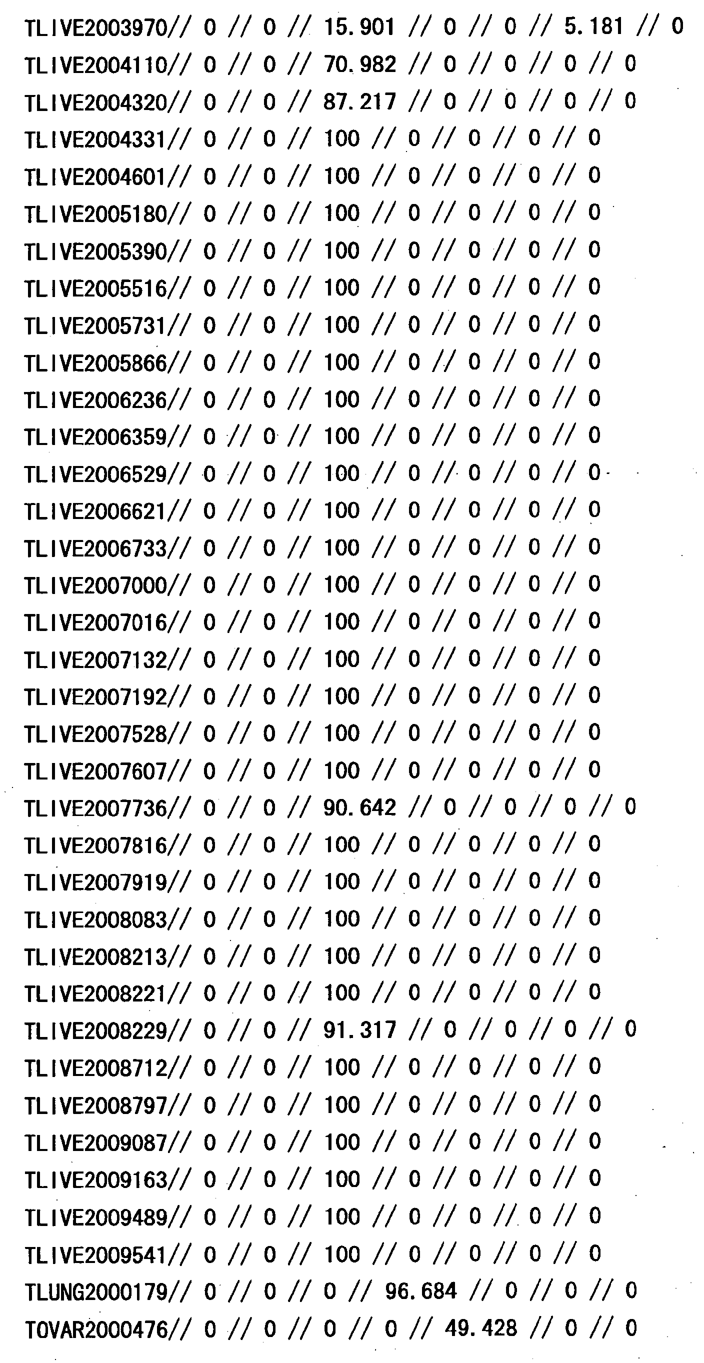

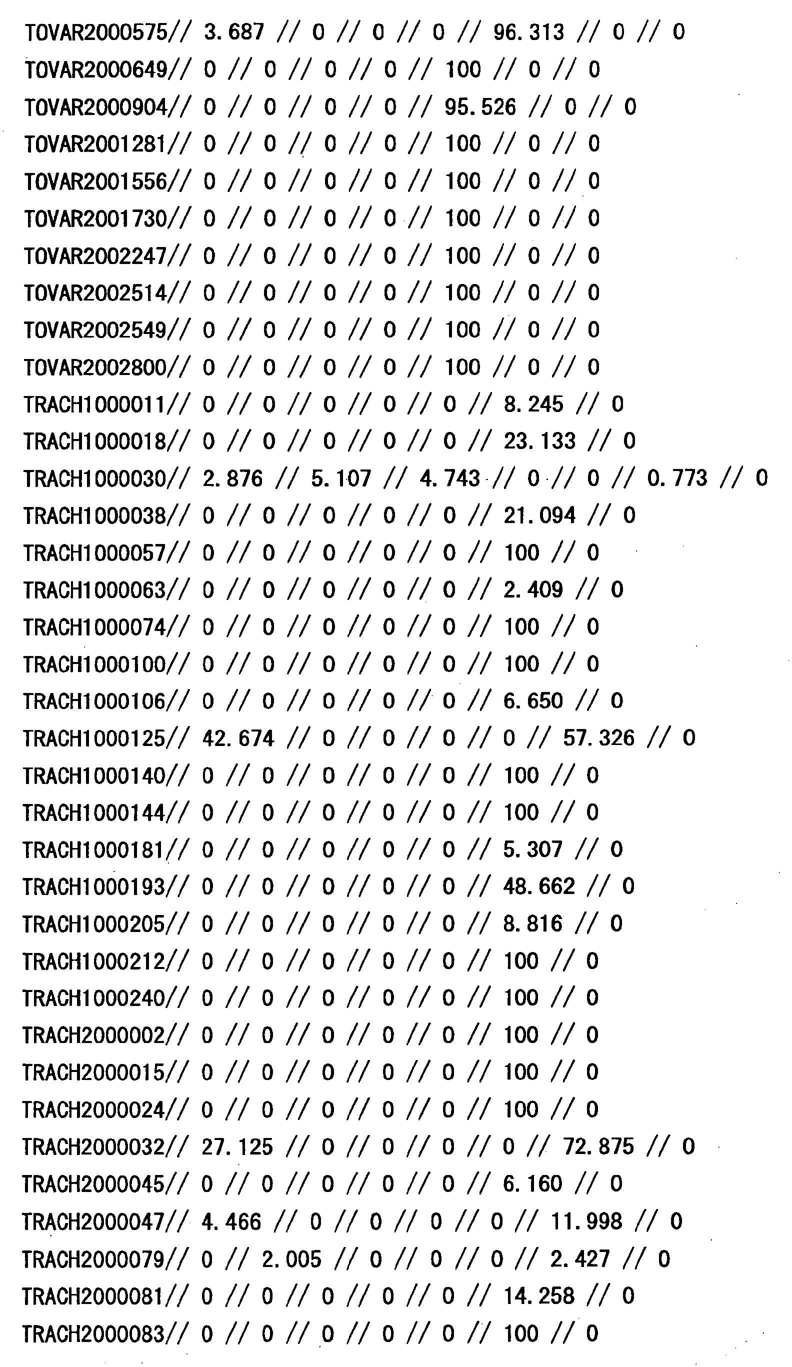

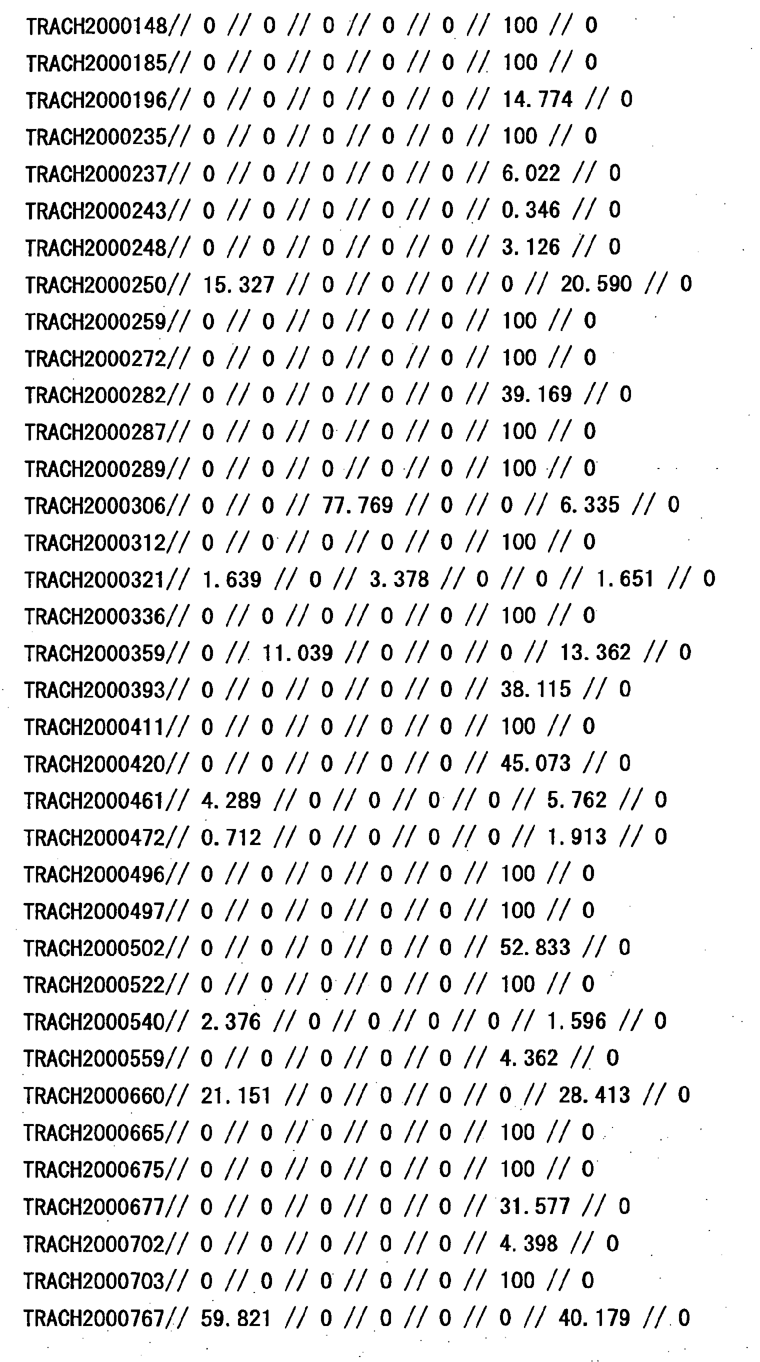

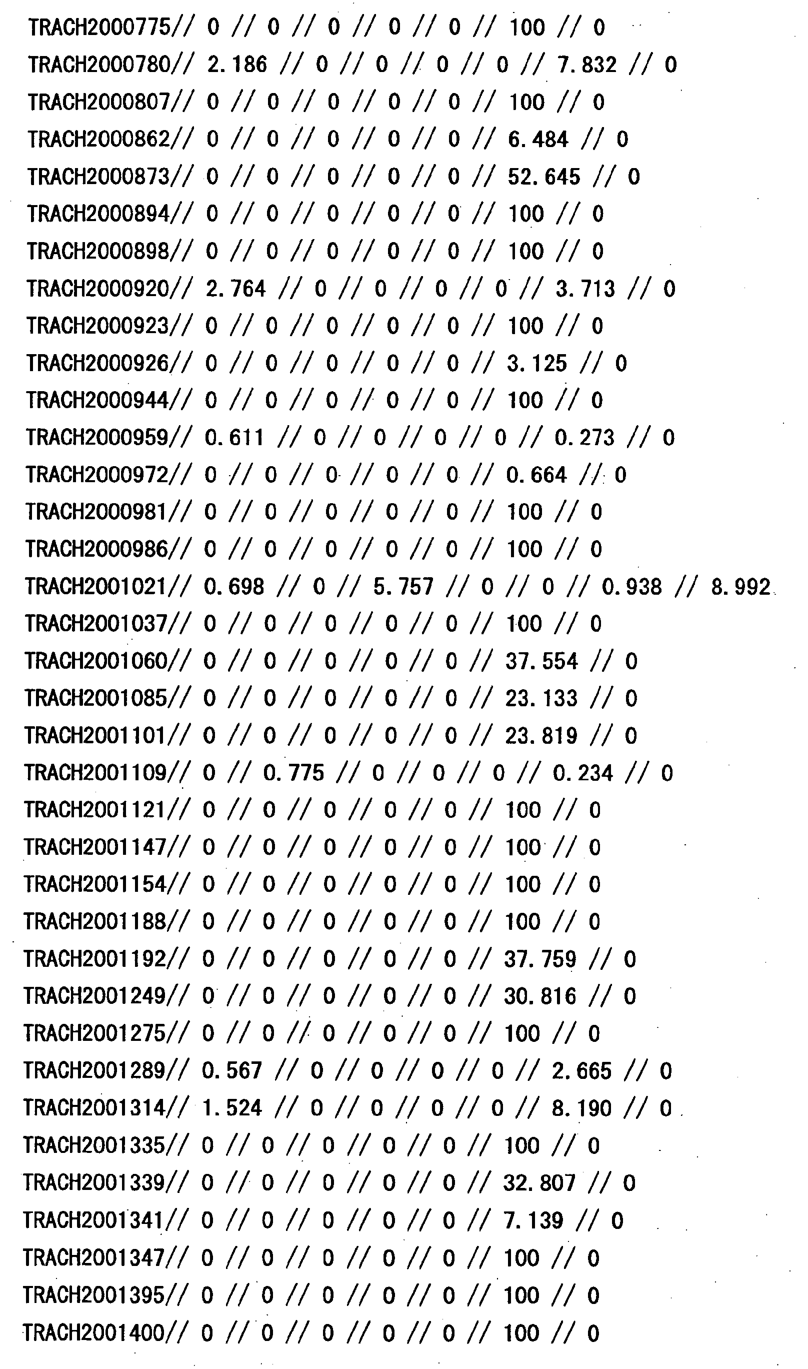

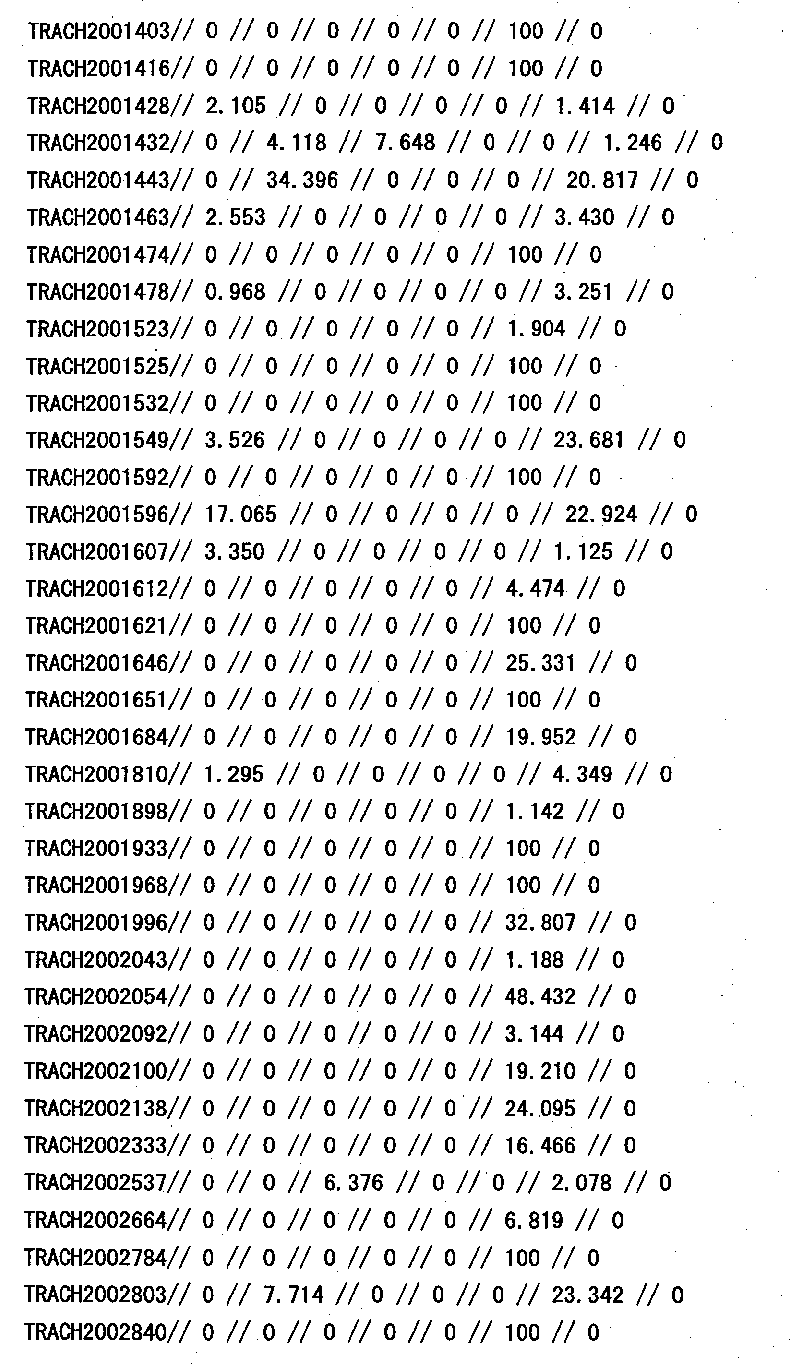

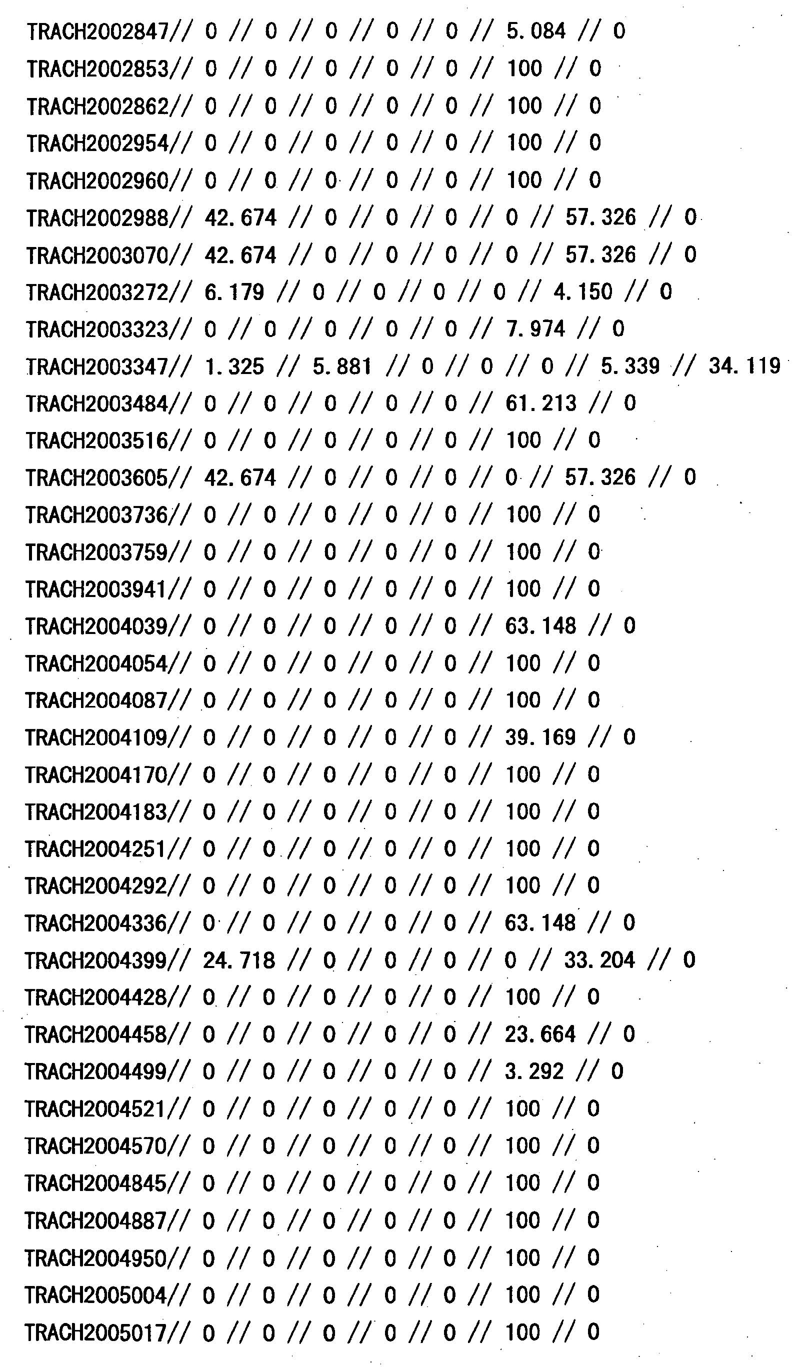

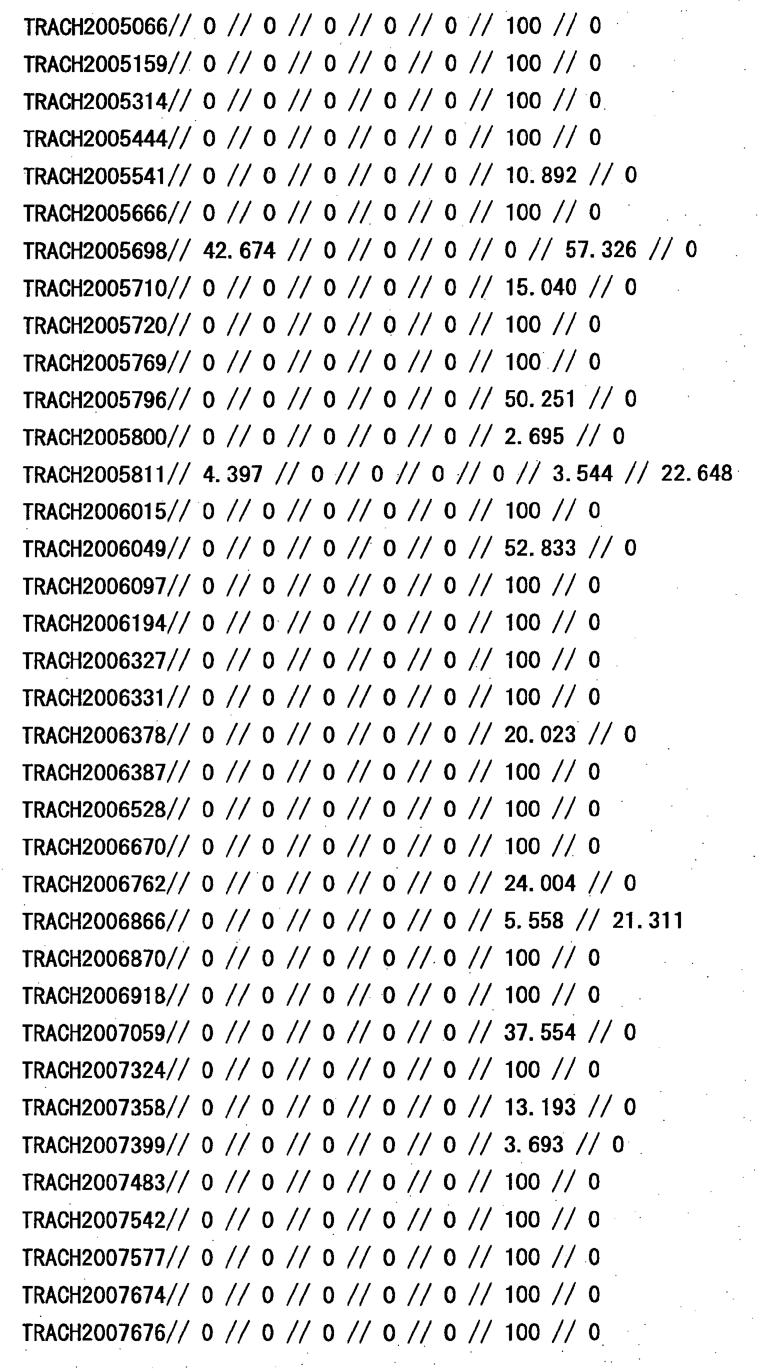

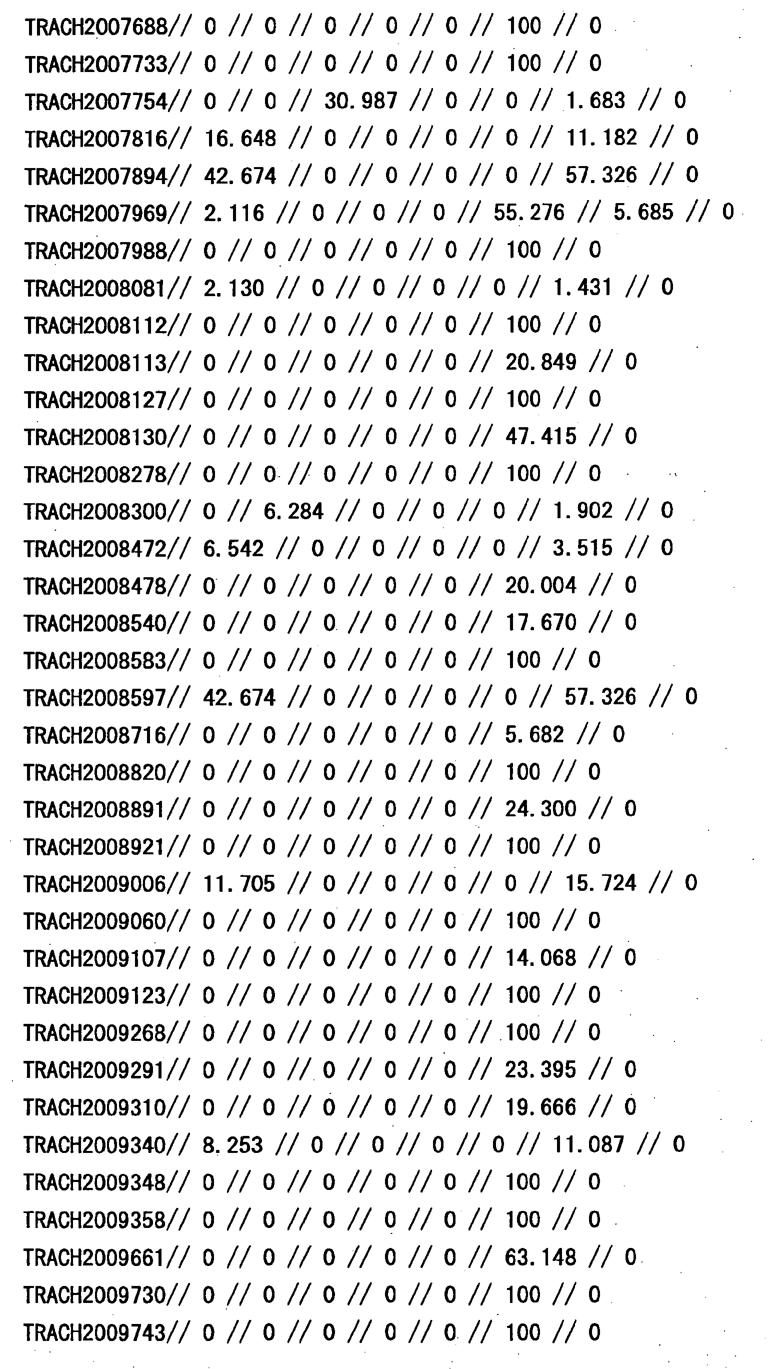

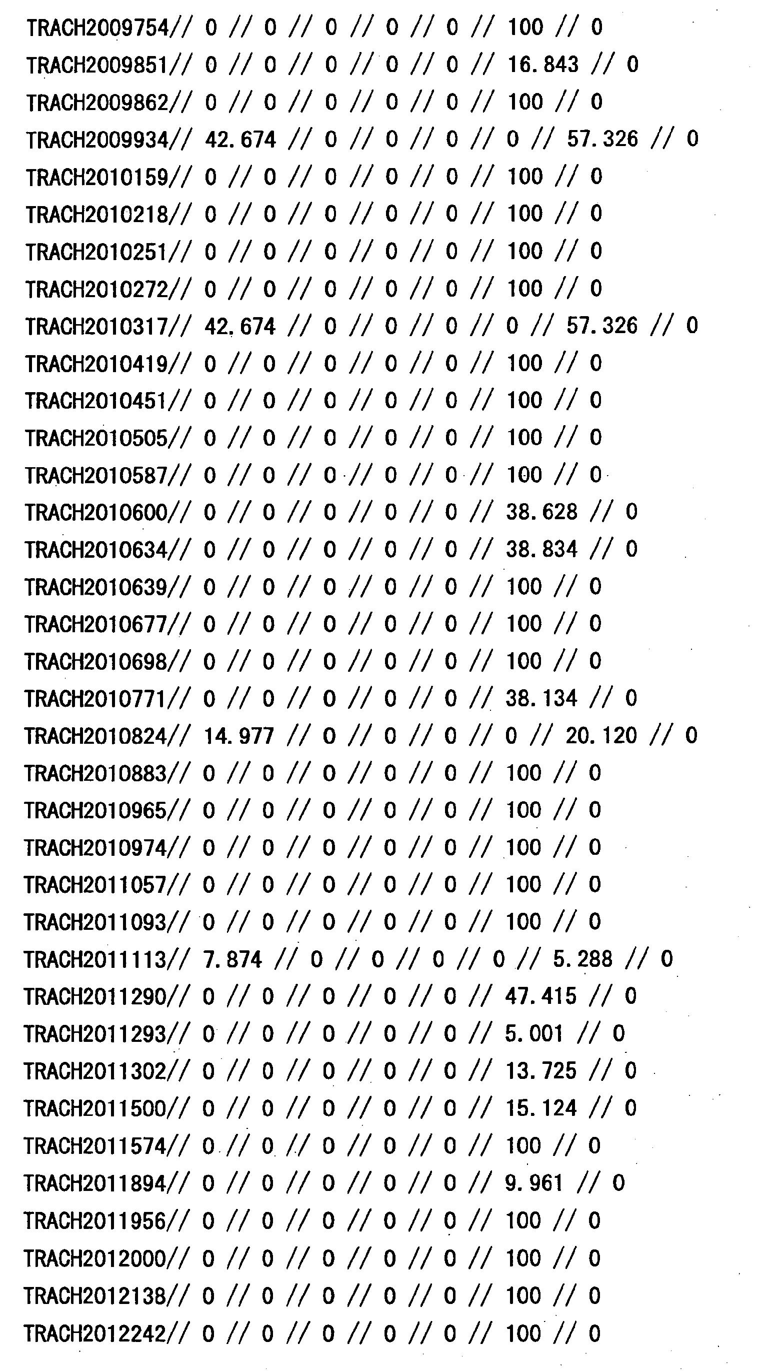

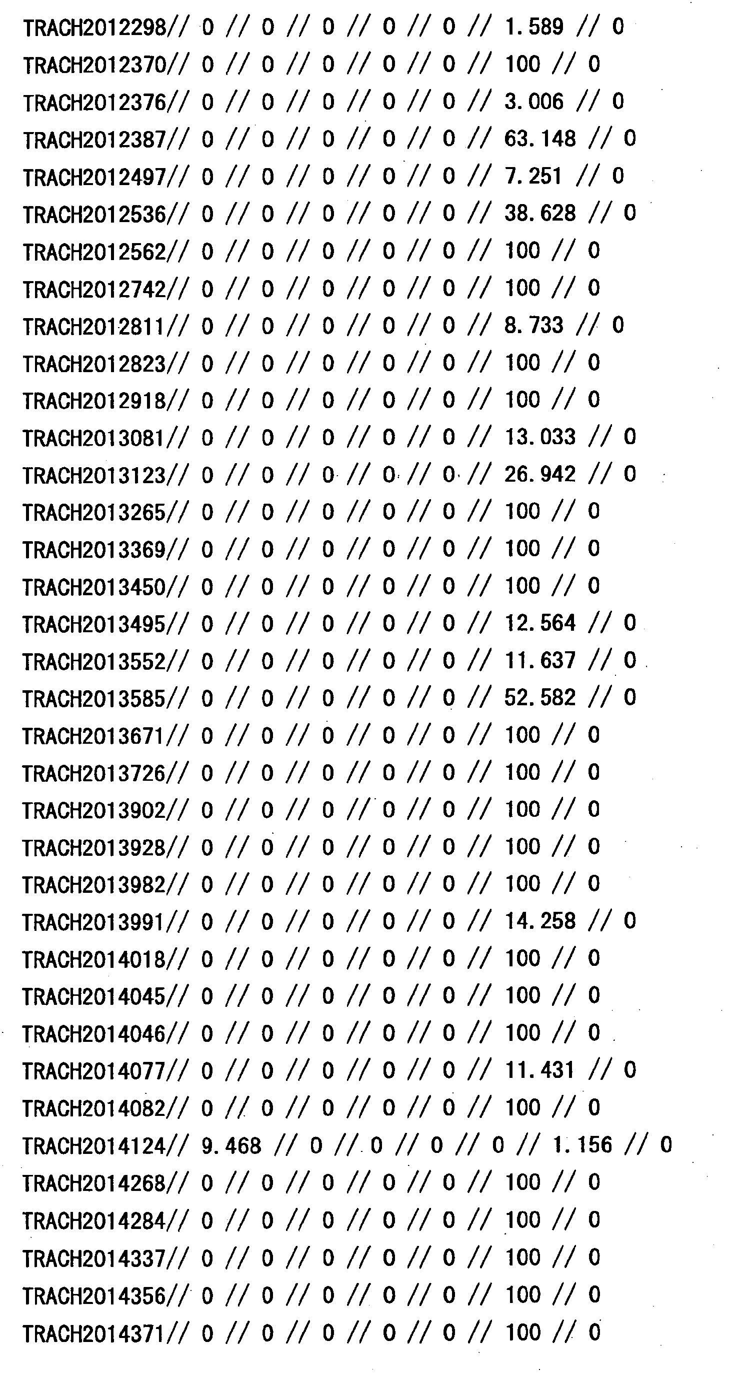

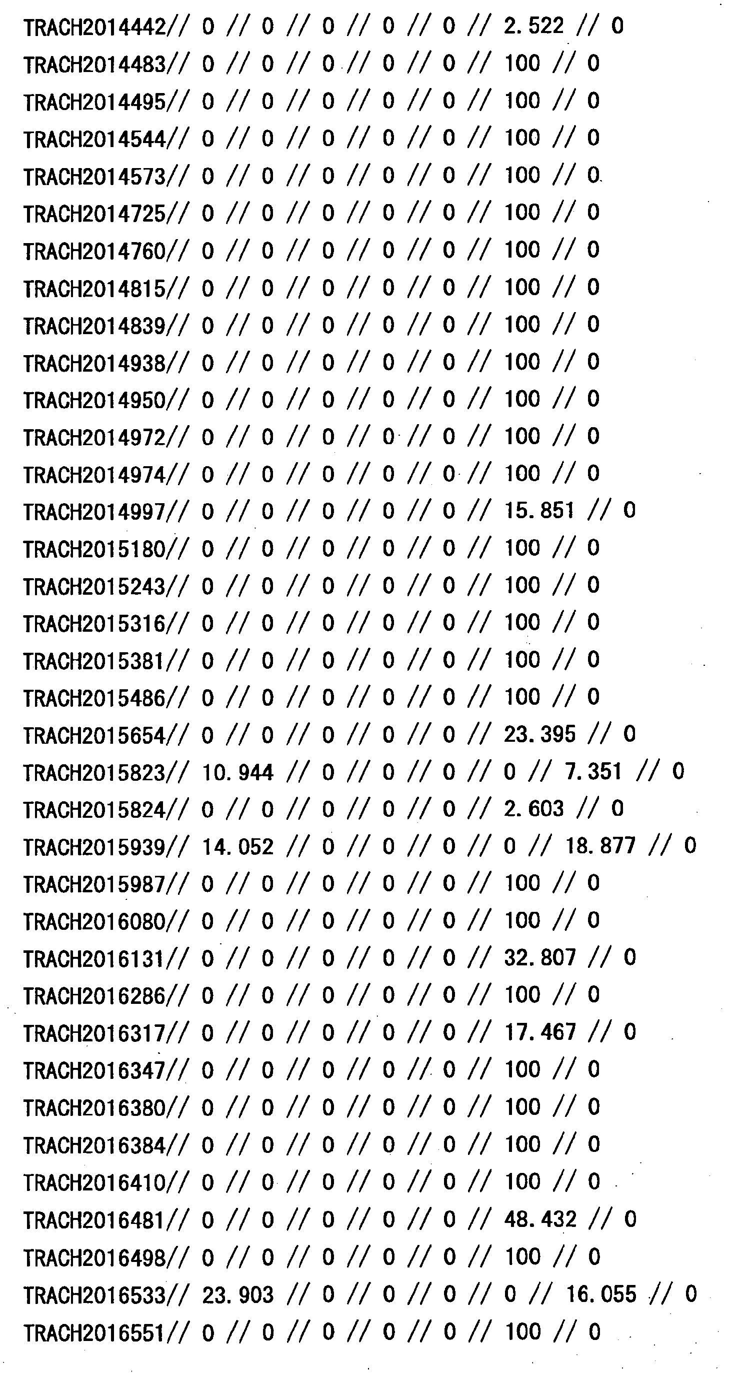

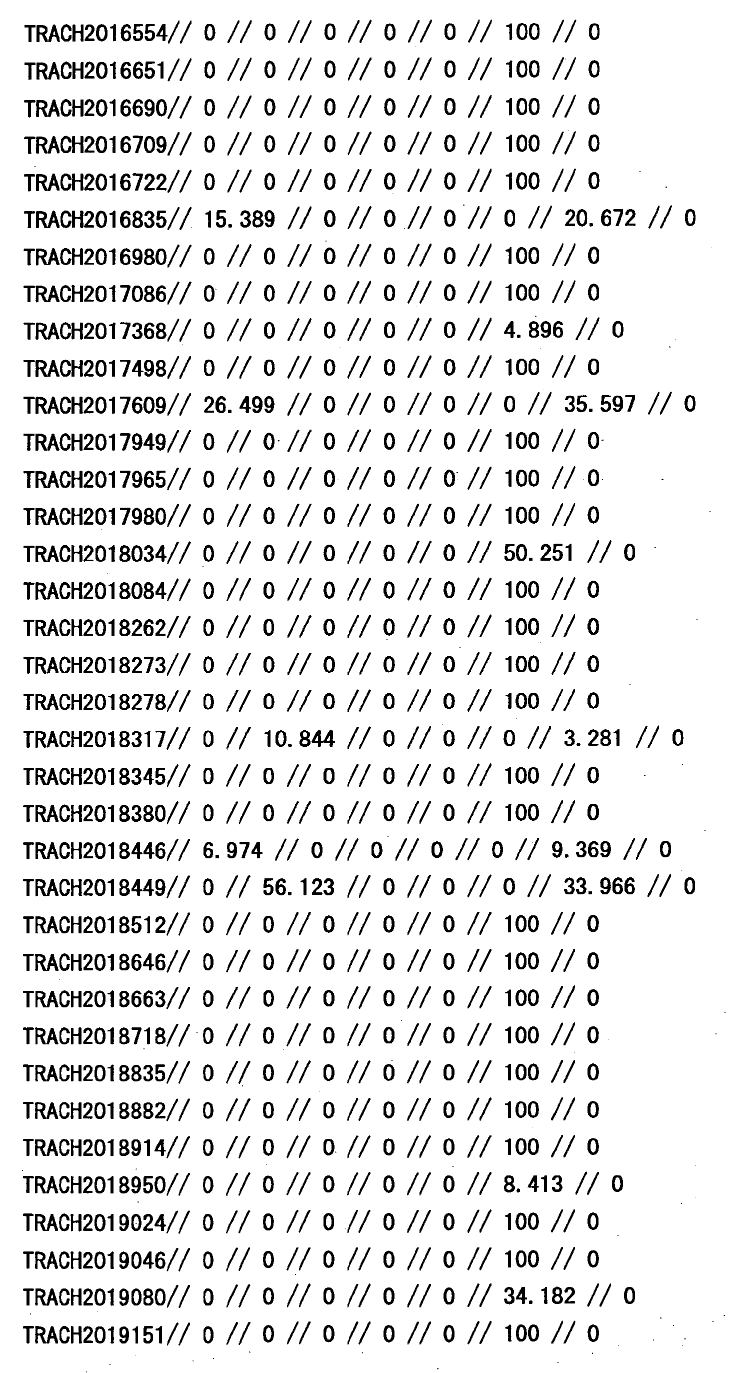

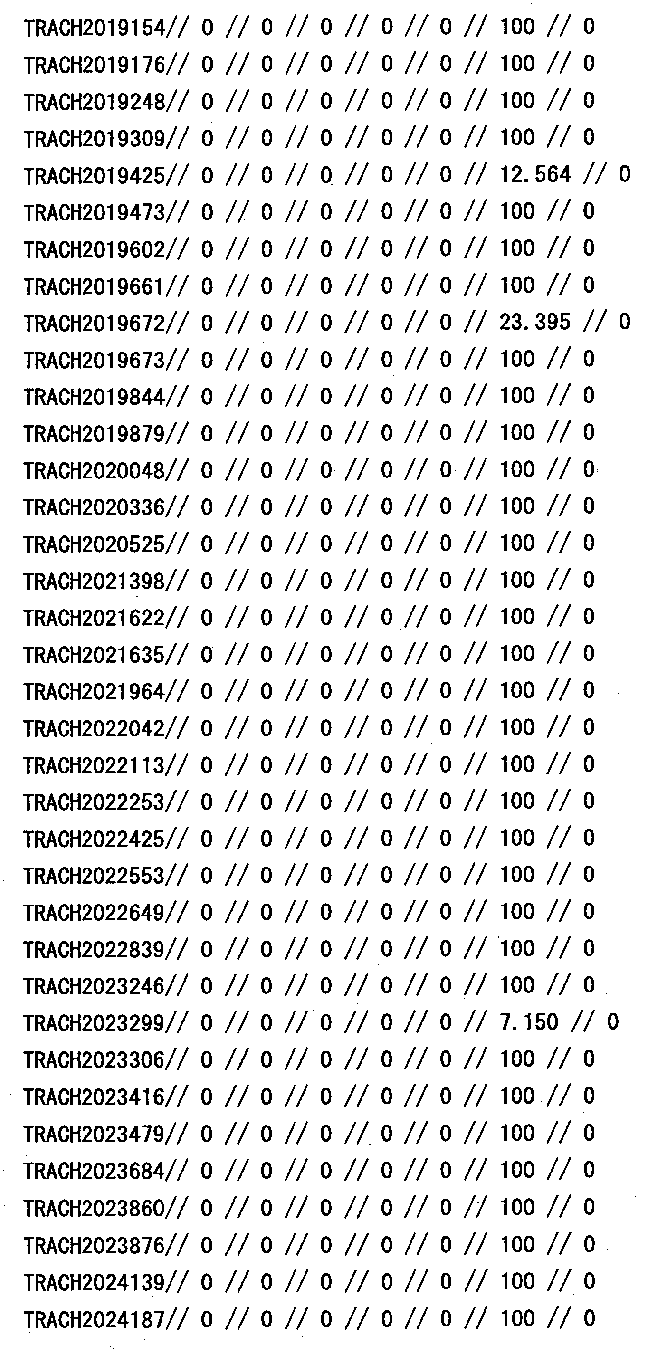

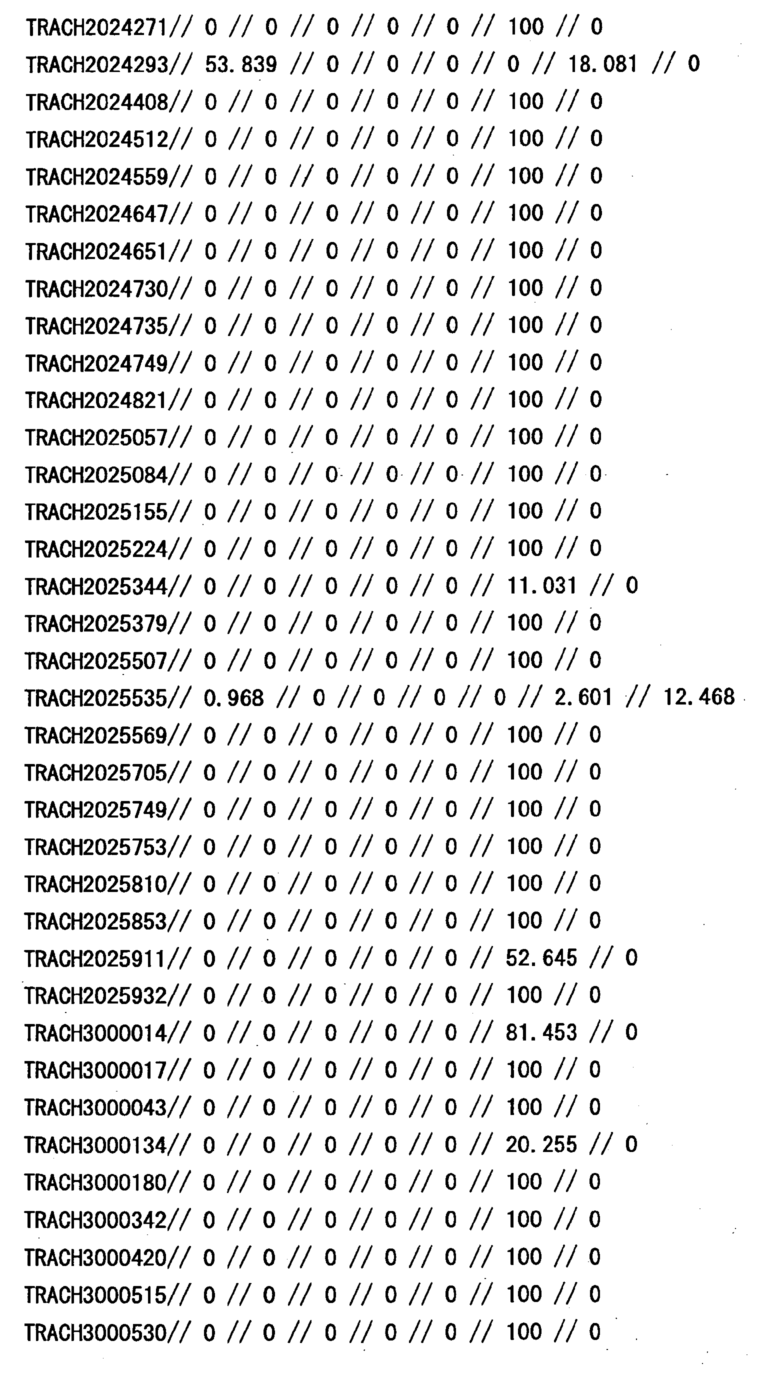

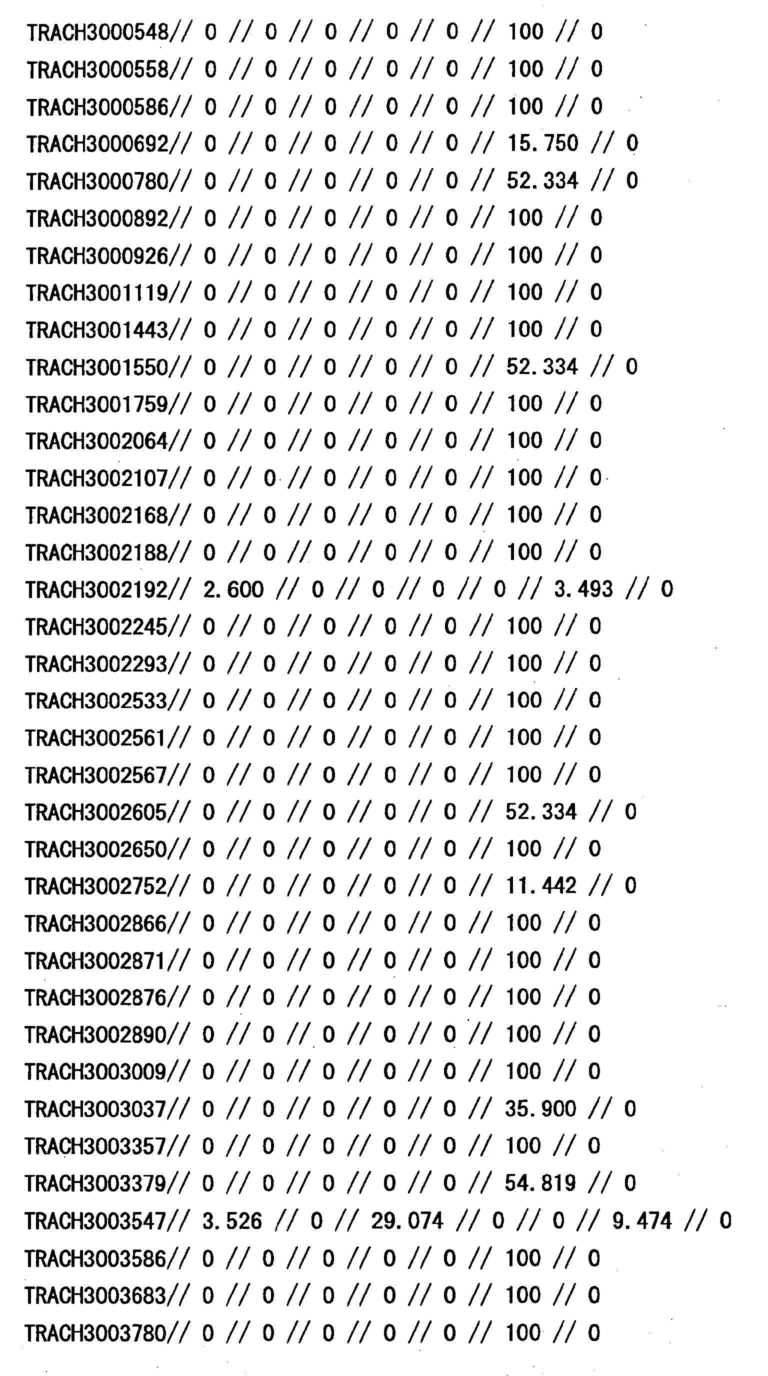

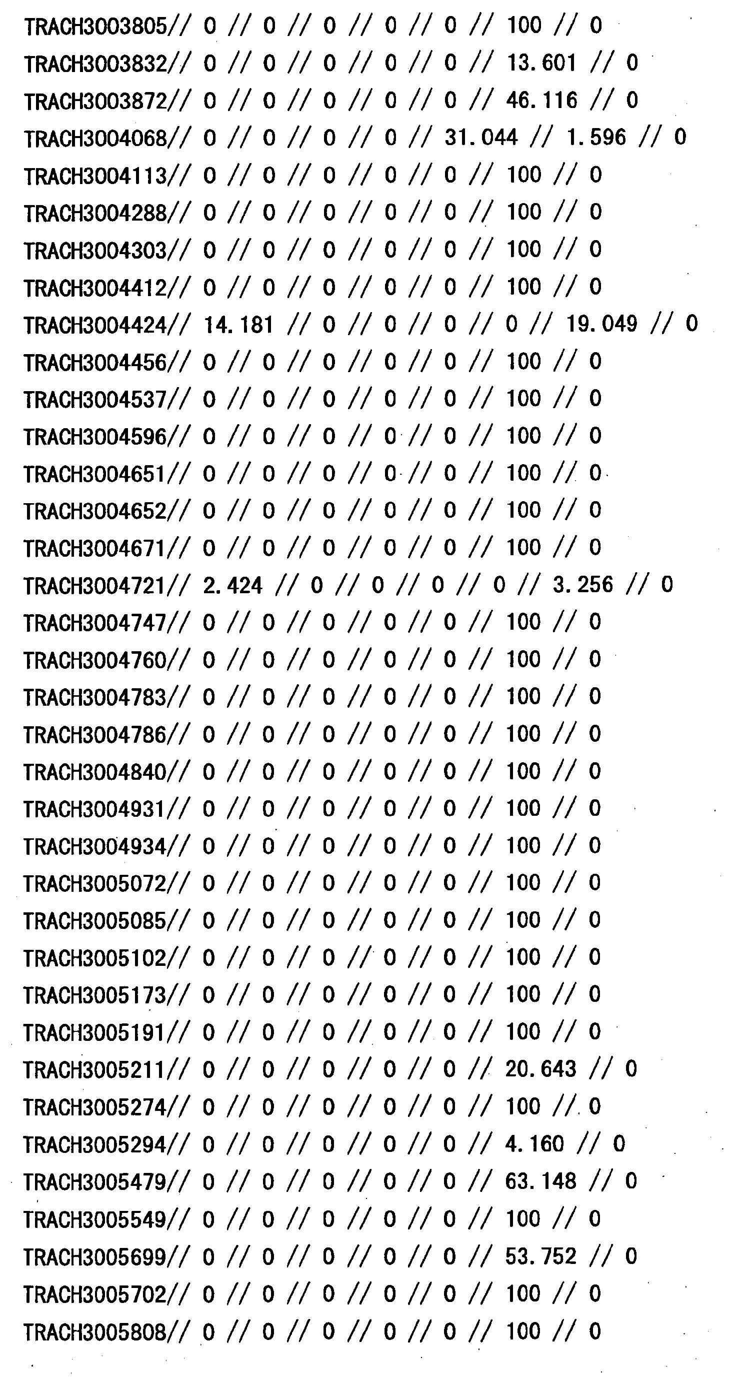

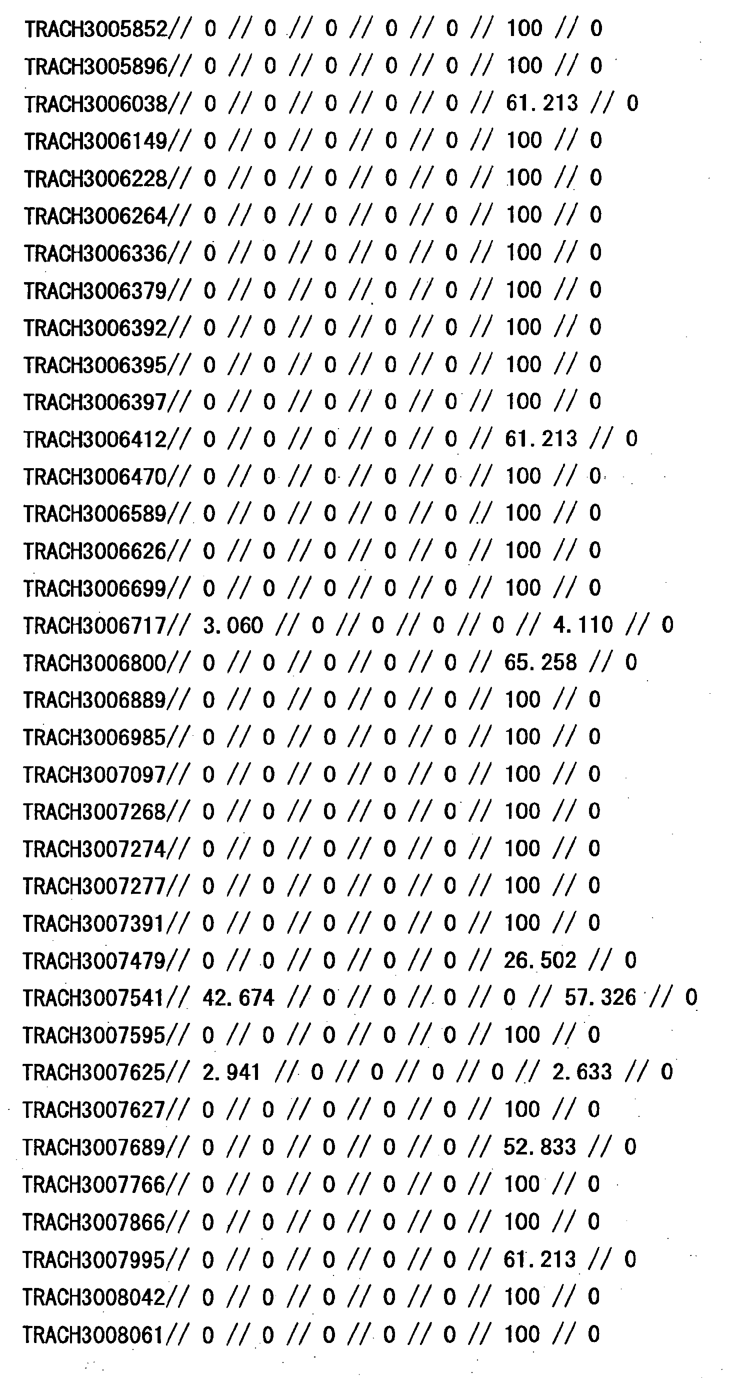

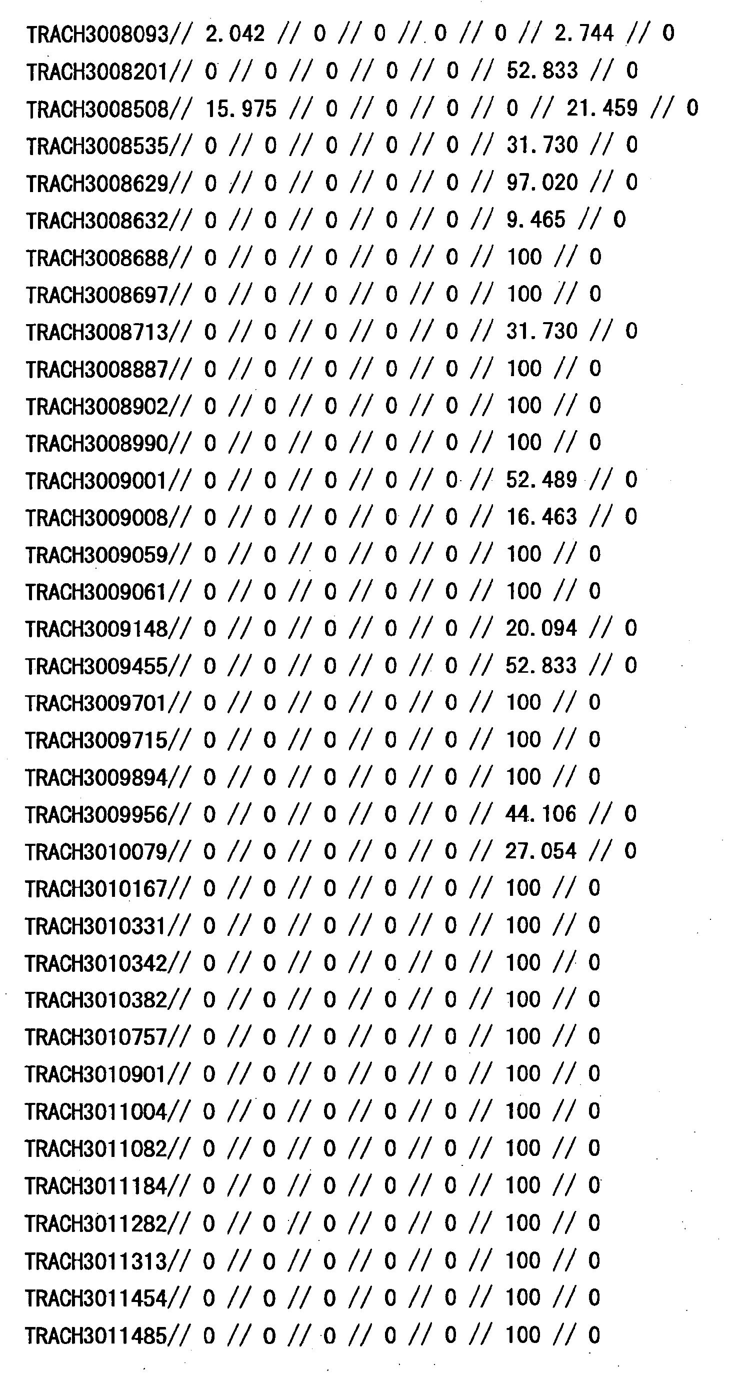

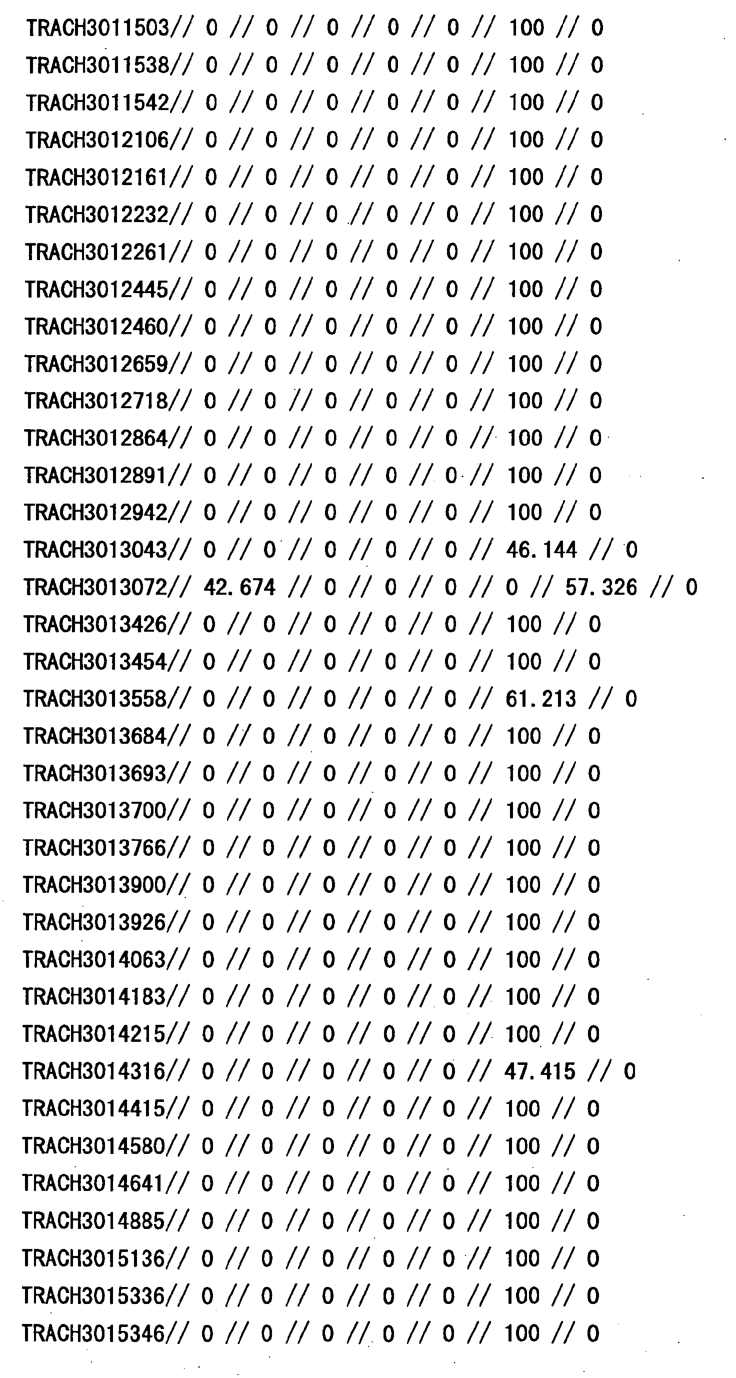

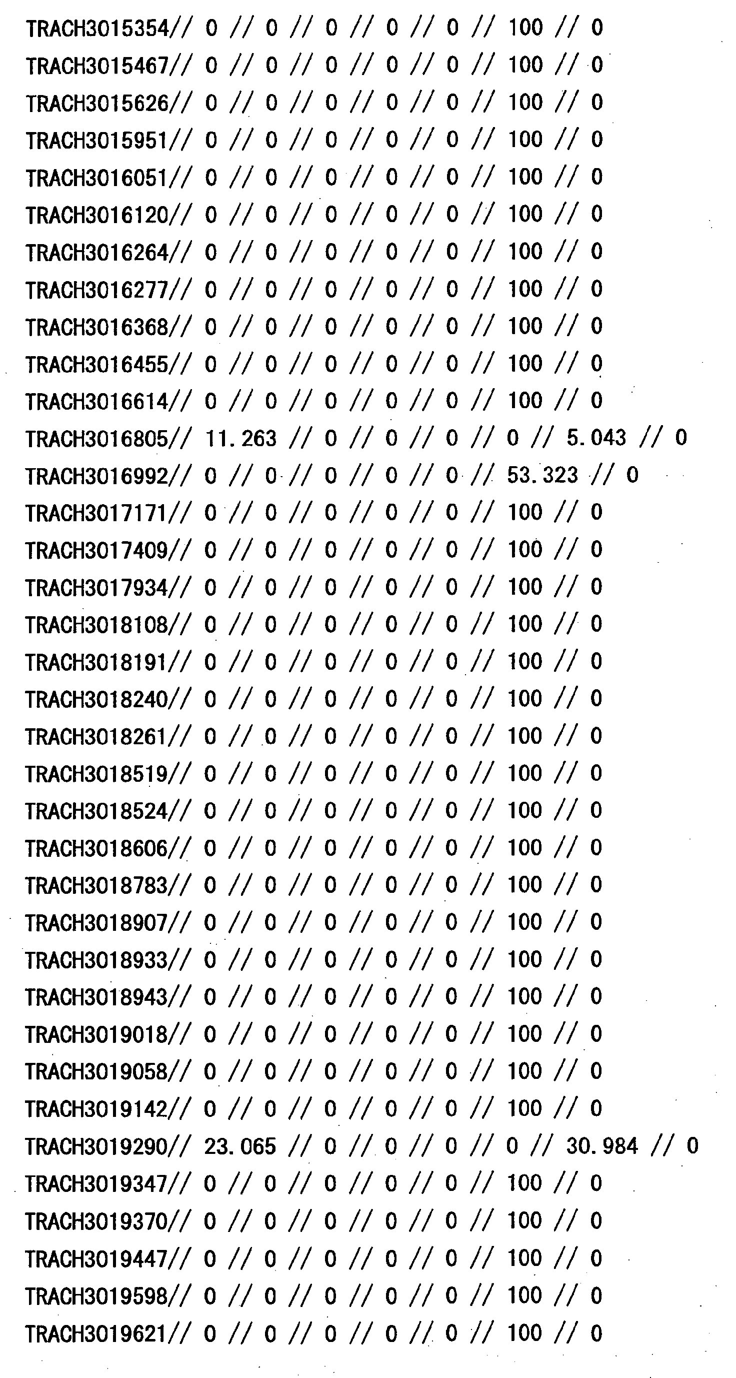

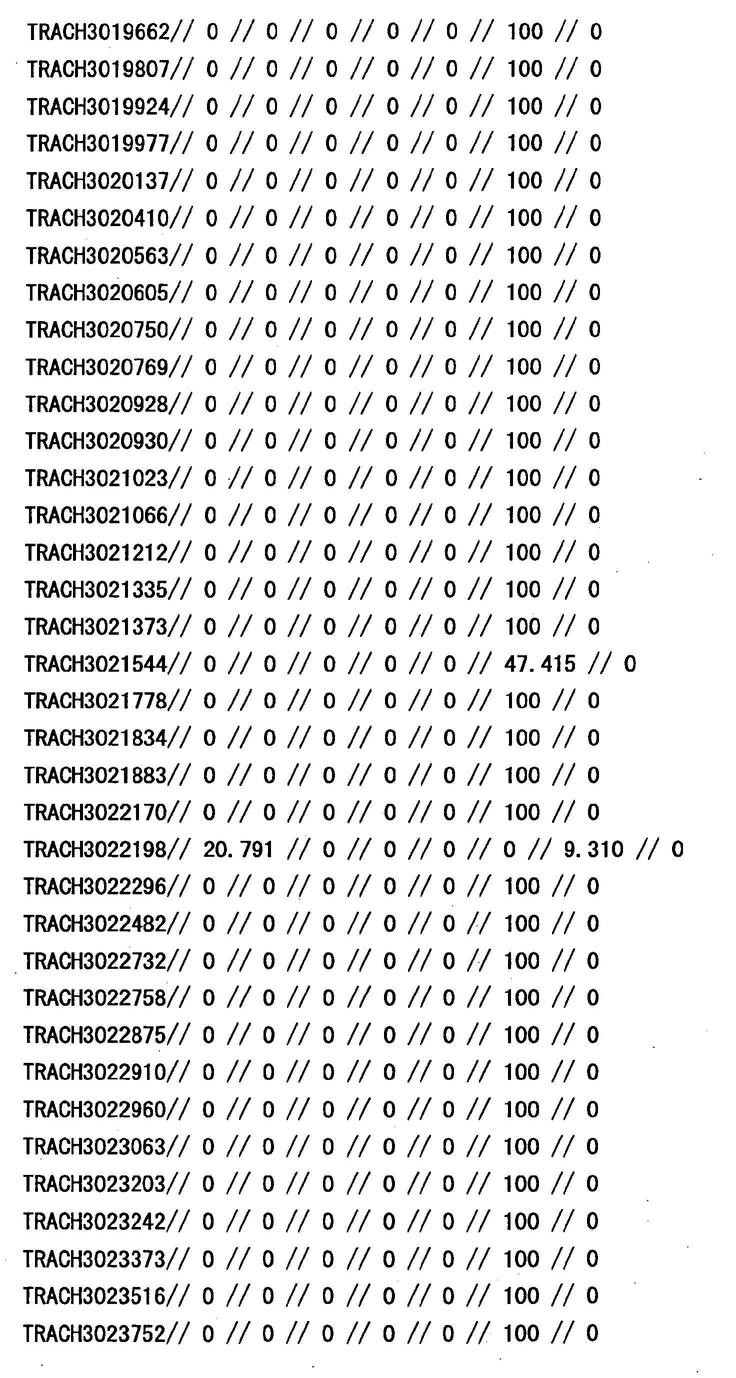

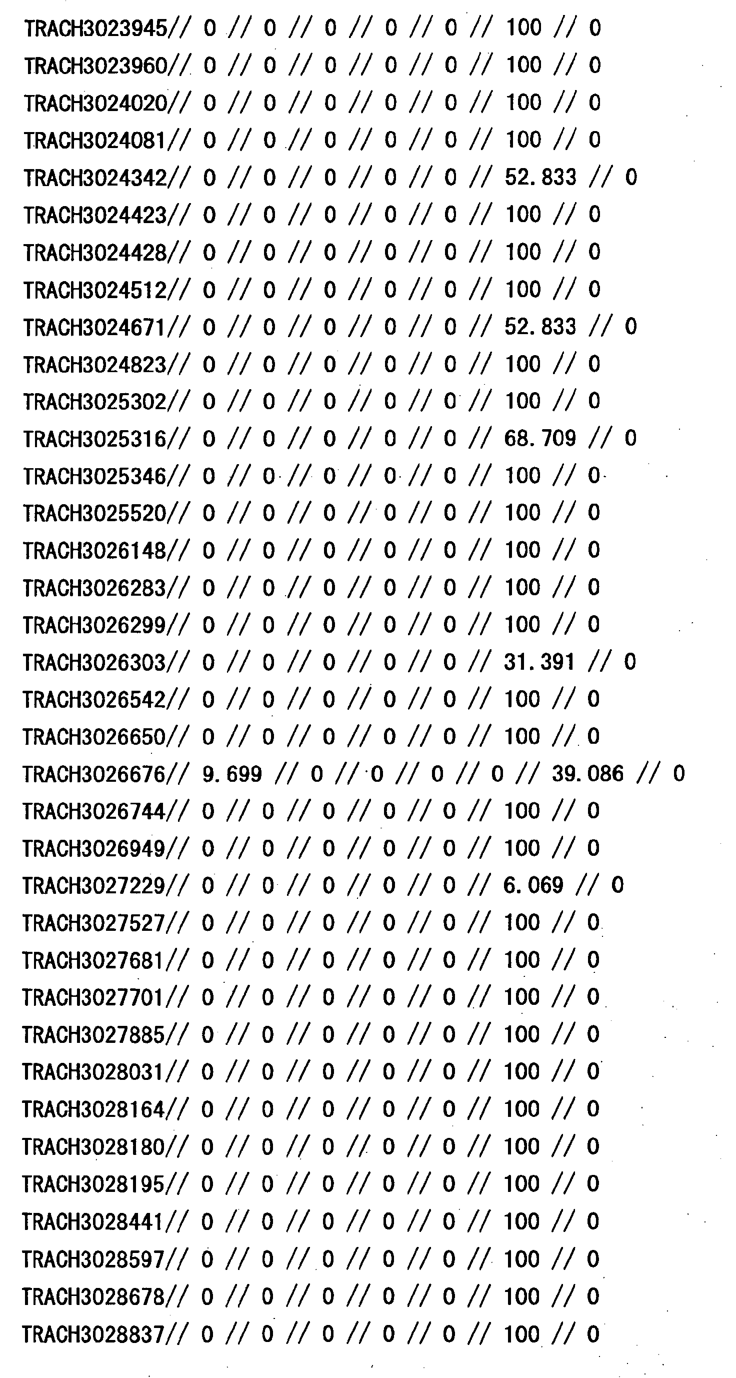

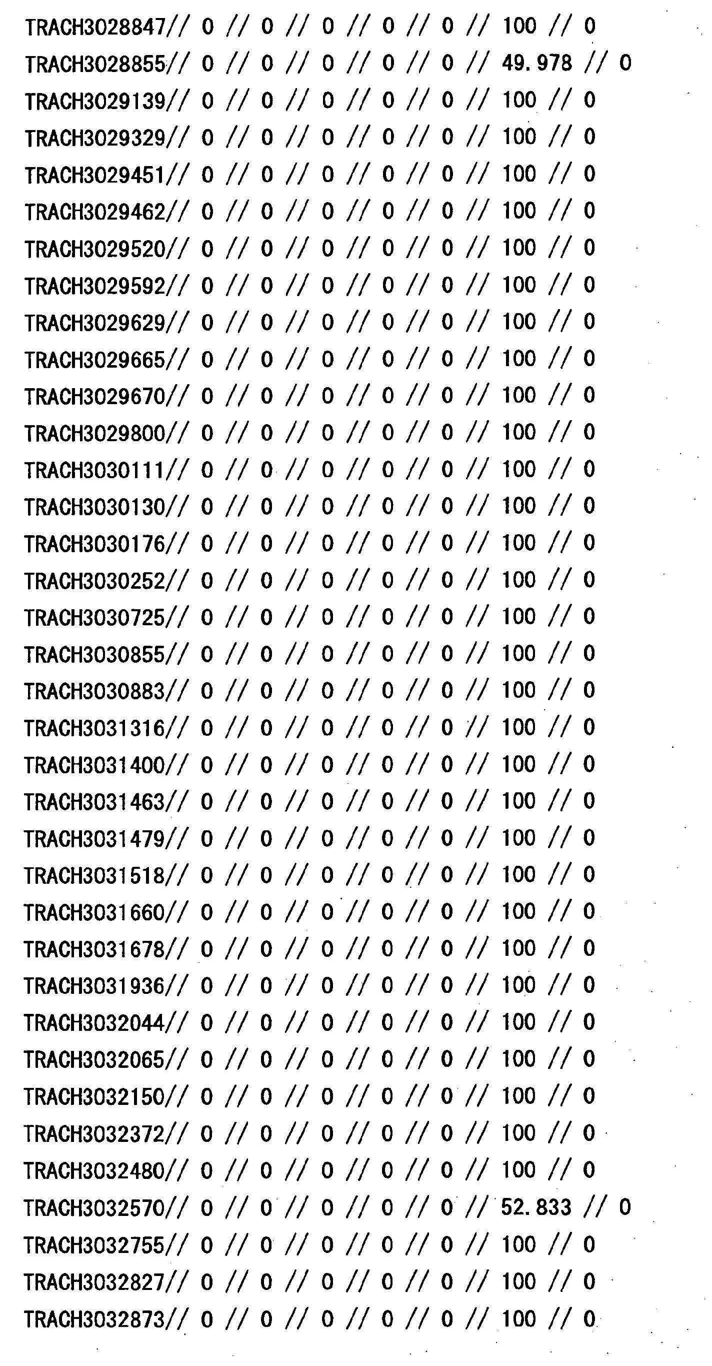

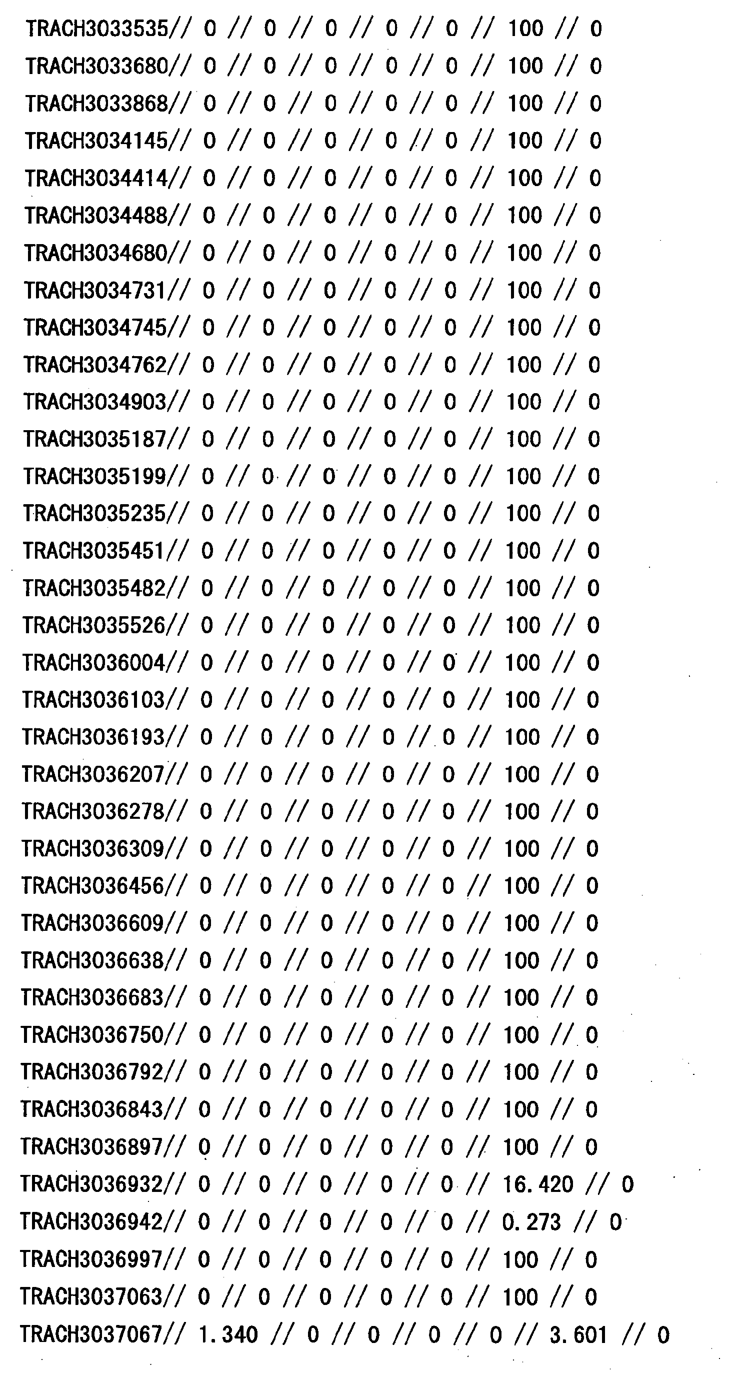

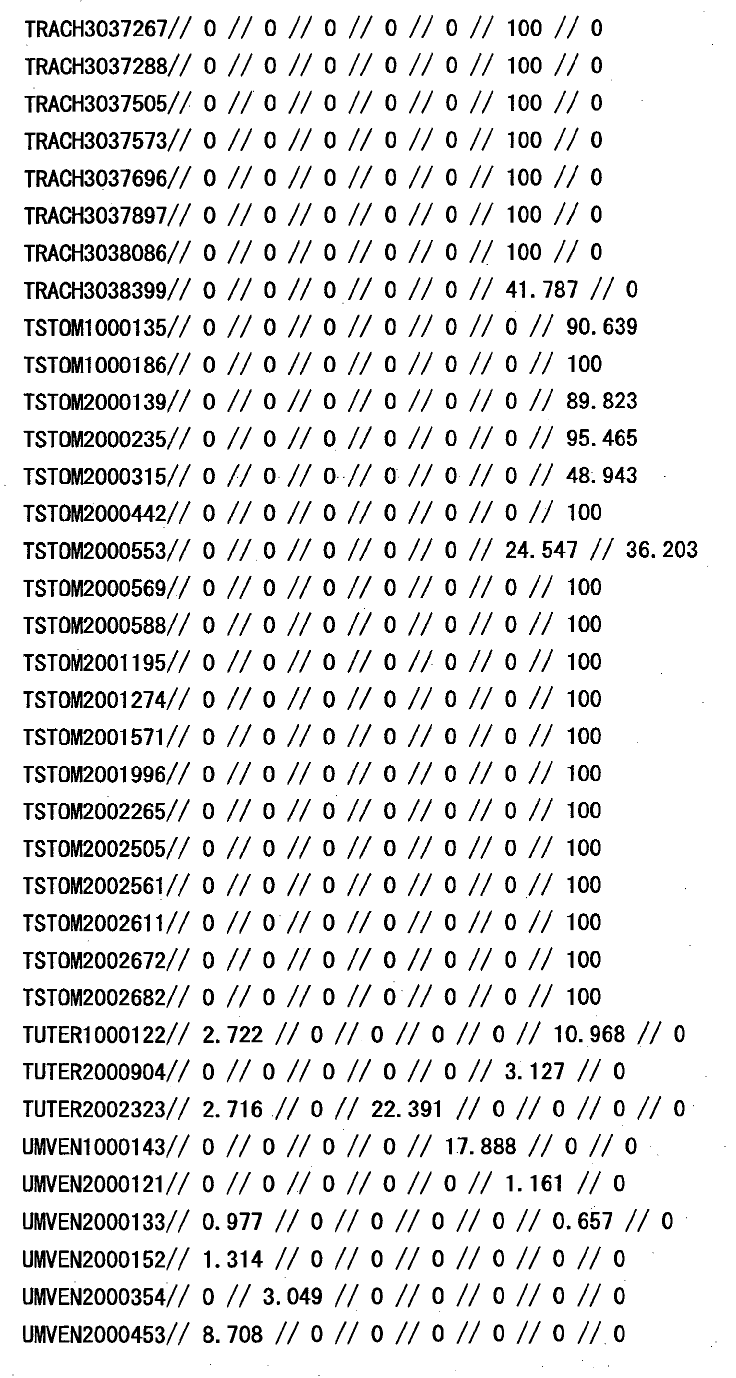

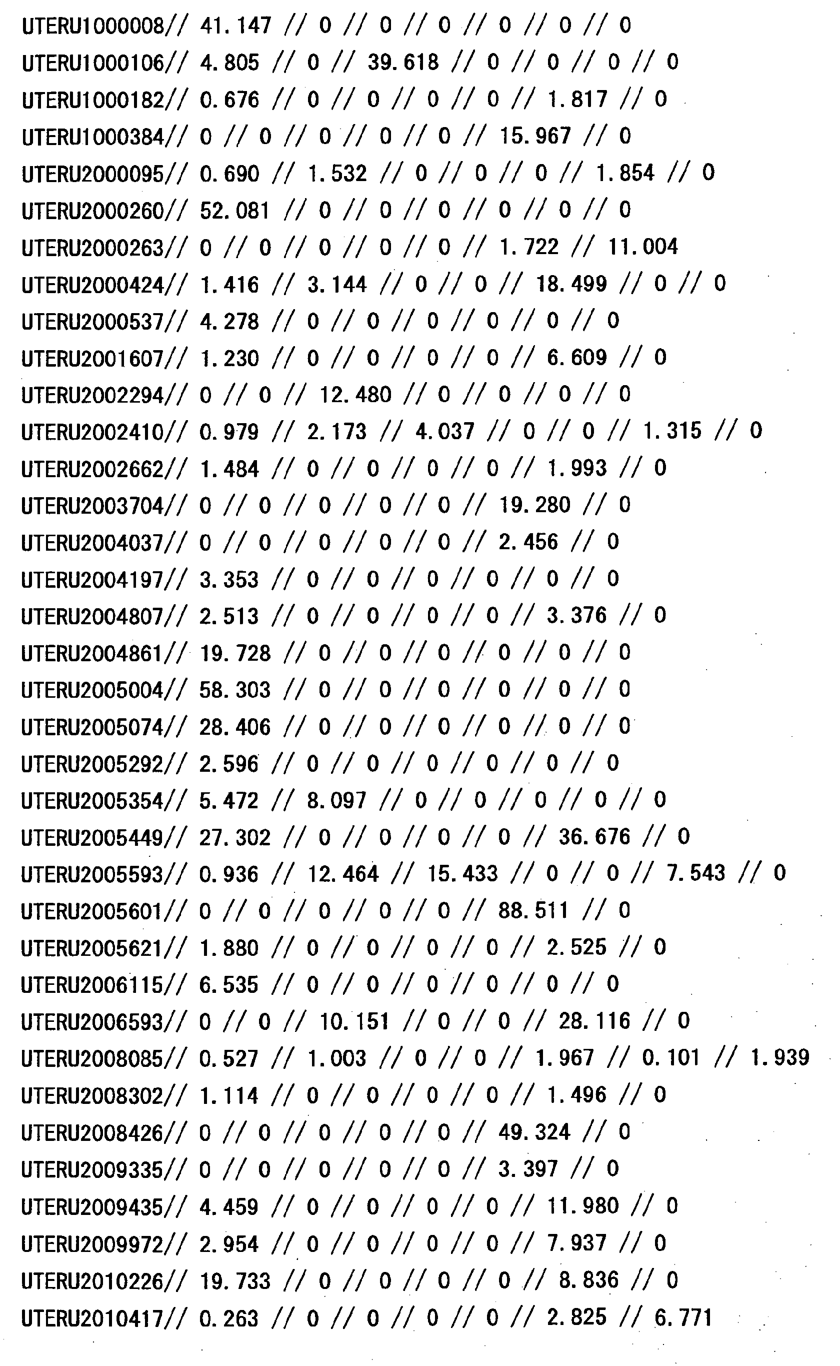

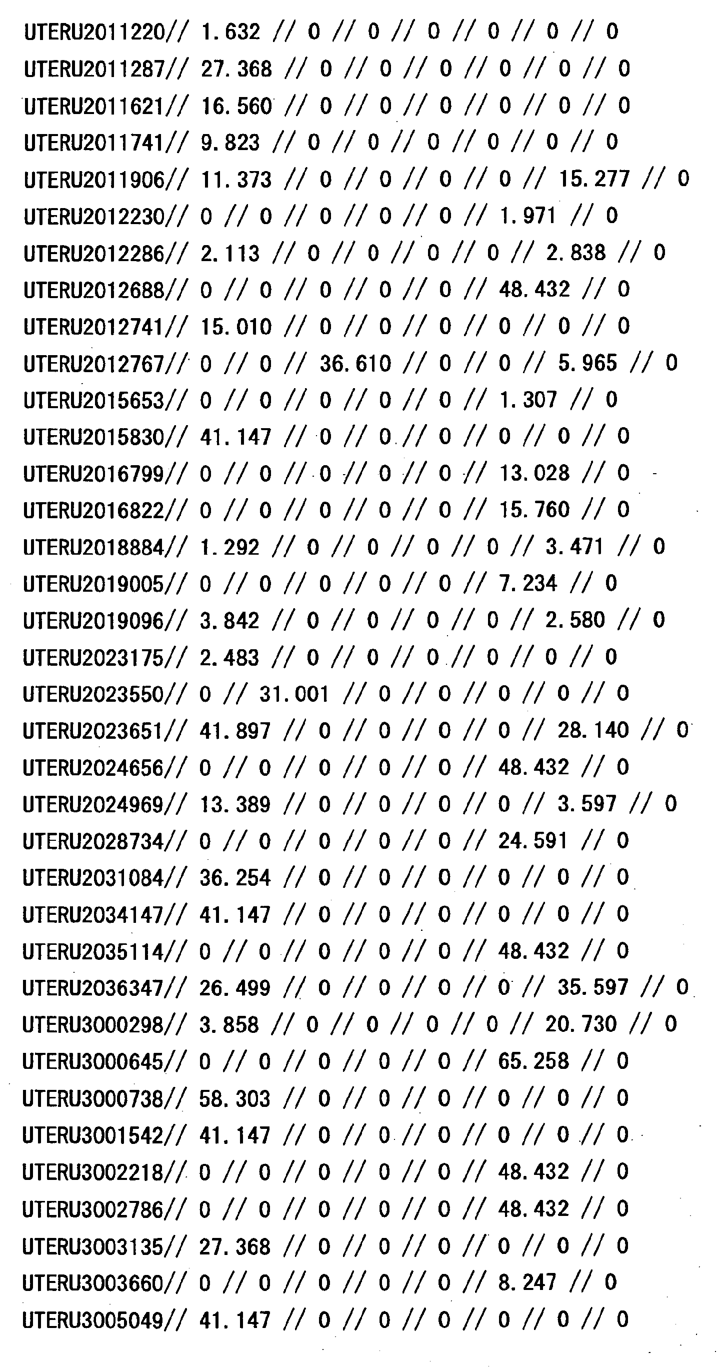

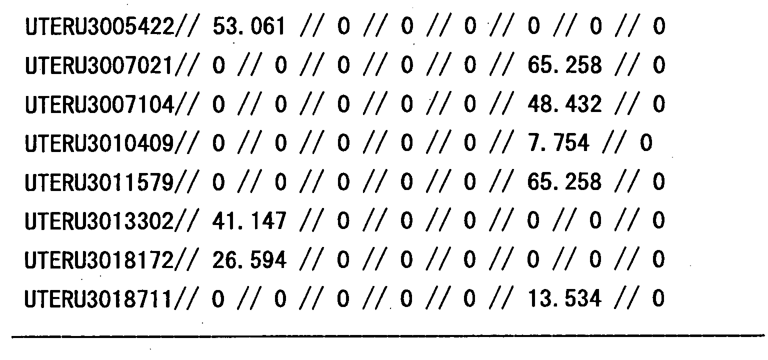

Genes whose expression levels were negligibly low in each of the above-described libraries are shown with their clone names below.
3NB691000018, 3NB691000085, 3NB691000113, 3NB691000129, 3NB691000191, 3NB692000012, 3NB692000029, 3NB692000102, 3NB692000154, 3NB692000276, 3NB692000305, 3NB692000429, 3NB692000484, 3NB692000529, 3NB692000973, 3NB692001002, 3NB692001034, 3NB692001040, 3NB692001123, 3NB692001267, 3NB692001339, 3NB692001349, 3NB692001366, 3NB692001408, 3NB692001433, 3NB692001459, 3NB692001471, 3NB692001496, 3NB692001501, 3NB692001507, 3NB692001511, 3NB692001519, 3NB692001528, 3NB692001548, 3NB692001557, 3NB692001637, 3NB692001719, 3NB692001861, 3NB692002365, 3NB692002685, 3NB692002806, 3NB692003538, 3NB692004045, 3NB692004724, 3NB692005439, 3NB692006952, 3NB692008178, 3NB692008729, ACTVT2000380, ADIPS1000064,
ADIPS2000069, ADIPS2000088, ADIPS2000245, ADIPS2000425, ADRGL1000033, ADRGL1000038, ADRGL1000065, ADRGL1000067, ADRGL1000144, ADRGL1000147, ADRGL1000160, ADRGL1000165, ADRGL1000182, ADRGL2000006, ADRGL2000042, ADRGL2000064, ADRGL2000074, ADRGL2000085, ADRGL2000117, ADRGL2000142, ADRGL2000172, ADRGL2000248, ADRGL2000261, ADRGL2000323, ADRGL2000328, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756, ADRGL2001830, ADRGL2001836, ADRGL2001854, ADRGL2002013, ADRGL2002029, ADRGL2002191, ADRGL2002392, ADRGL2002679, ADRGL2002753, ADRGL2002857, ADRGL2003017,
ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008966, ADRGL2009146, ADRGL2009324,
ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2012179, AHMSC1000138, ASTRO1000009, ASTRO1000018, ASTRO1000029, ASTRO1000096, ASTRO1000165, ASTRO2000011, ASTRO2000014, ASTRO2000095, ASTRO2000191, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008, ASTRO2001088, ASTRO2001107, ASTRO2001122, ASTRO2001227, ASTRO2001249, ASTRO2001480, ASTRO2002035, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003574,
ASTRO2003581, ASTRO2003740, ASTRO2003925, ASTRO2003960, ASTRO2004032, ASTRO2004051, ASTRO2004177, ASTRO2004217, ASTRO2004297, ASTRO2004584, ASTRO2004751, ASTRO2004877, ASTRO2005008, ASTRO2005081, ASTRO2005096, ASTRO2005242, ASTRO2005273, ASTRO2005343, ASTRO2005413, ASTRO2005553, ASTRO2005557, ASTRO2005575, ASTRO2005726, ASTRO2005863, ASTRO2005896, ASTRO2005902, ASTRO2006356, ASTRO2006475, ASTRO2006732, ASTRO2006747, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008508, ASTRO2008777, ASTRO2008895, ASTRO2008960, ASTRO2008972, ASTRO2009177, ASTRO2009333,
ASTRO2009458, ASTRO2009526, ASTRO2009879, ASTRO2010072, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2013124, ASTRO2013802, ASTRO2014174, ASTRO2014211, ASTRO2014363, ASTRO2014392, ASTRO2014498, ASTRO2014576, ASTRO2014923, ASTRO2014961, ASTRO2015162, ASTRO2015185, ASTRO2015328, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000193, BGGI11000285,
BGGI12000022, BGGI12000067, BGGI12000161, BGGI12000544, BGGI12000616, BGGI12000684, BGGI12000693, BGGI12001097, BGGI12001241, BGGI12001247, BGGI12001714, BLADE1000176, BLADE2000181, BLADE2000256, BLADE2000340, BLADE2000389, BLADE2000463, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004345, BLADE2004389, BLADE2004462, BLADE2004628, BLADE2004670, BLADE2004722, BLADE2004849, BLADE2005036,
BLADE2005038, BLADE2005229, BLADE2005459, BLADE2005792, BLADE2006043, BLADE2006415, BLADE2006607, BLADE2006826, BLADE2006849, BLADE2006947, BLADE2007033, BLADE2007043, BLADE2007722, BLADE2007735, BLADE2007744, BLADE2007799, BLADE2007923, BLADE2007935, BLADE2007958, BLADE2008281, BLADE2008454, BLADE2008479, BLADE2008539, BLADE2008809, BLADE2008995, BLADE2009452, BLADE2009479, BLADE2009593, BNGH41000104, BNGH41000118, BNGH41000169, BNGH41000177, BNGH41000198, BNGH41000216, BNGH42000130, BNGH42000132, BNGH42000405, BNGH42000532, BNGH42000675, BNGH42000791, BNGH42000815, BNGH42000994, BNGH42001247, BNGH42001406, BNGH42001576, BNGH42001724, BNGH42002244, BNGH42002387, BNGH42002487, BNGH42002489,
BNGH42003529, BNGH42003570, BNGH42003641, BNGH42004076, BNGH42004291, BNGH42004538, BNGH42004679, BNGH42005017, BNGH42005235, BNGH42005504, BNGH42006226, BNGH42006234, BNGH42007037, BNGH42007594, BNGH42007798, BNGH42008199, BNGH42008510, BNGH42008649, BNGH42008832, BNGH42008850, BNGH42008943, BNGH42009021, BNGH42009025, BRACE1000047, BRACE1000051, BRACE1000065, BRACE1000084, BRACE1000087, BRACE1000092, BRACE1000093, BRACE1000101, BRACE1000114, BRACE1000124, BRACE1000132, BRACE1000169, BRACE1000187, BRACE1000239, BRACE1000258, BRACE1000451, BRACE1000475, BRACE1000572, BRACE2000077, BRACE2000078, BRACE2000100, BRACE2000109, BRACE2000141, BRACE2000148, BRACE2000183, BRACE2000229, BRACE2000232,
BRACE2000256, BRACE2000266, BRACE2000267, BRACE2000280, BRACE2000307, BRACE2000331, BRACE2000351, BRACE2000368, BRACE2000392, BRACE2000441, BRACE2000489, BRACE2000505, BRACE2000526, BRACE2000531, BRACE2000545, BRACE2000553, BRACE2000577, BRACE2000640, BRACE2000650, BRACE2000677, BRACE2000698, BRACE2000718, BRACE2000733, BRACE2000743, BRACE2000750, BRACE2000815, BRACE2000885, BRACE2000905, BRACE2000936, BRACE2000947, BRACE2000965, BRACE2000969, BRACE2000988, BRACE2001017, BRACE2001070, BRACE2001094, BRACE2001107, BRACE2001151, BRACE2001191, BRACE2001197, BRACE2001236, BRACE2001239, BRACE2001269, BRACE2001312, BRACE2001330, BRACE2001340, BRACE2001352, BRACE2001353, BRACE2001374, BRACE2001375,
BRACE2001423, BRACE2001424, BRACE2001445, BRACE2001453, BRACE2001455, BRACE2001492, BRACE2001508, BRACE2001534, BRACE2001543, BRACE2001564, BRACE2001588, BRACE2001607, BRACE2001626, BRACE2001677, BRACE2001705, BRACE2001726, BRACE2001733, BRACE2001737, BRACE2001742, BRACE2001777, BRACE2001779, BRACE2001859, BRACE2001865, BRACE2001872, BRACE2001881, BRACE2001898, BRACE2001923, BRACE2001954, BRACE2001972, BRACE2001973, BRACE2002026, BRACE2002034, BRACE2002139, BRACE2002142, BRACE2002151, BRACE2002157, BRACE2002176, BRACE2002202, BRACE2002206, BRACE2002227, BRACE2002260, BRACE2002297, BRACE2002304, BRACE2002356, BRACE2002394, BRACE2002409, BRACE2002431, BRACE2002441, BRACE2002444, BRACE2002458,
BRACE2002467, BRACE2002468, BRACE2002478, BRACE2002495, BRACE2002582, BRACE2002585, BRACE2002589, BRACE2002590, BRACE2002606, BRACE2002613, BRACE2002614, BRACE2002617, BRACE2002623, BRACE2002635, BRACE2002676, BRACE2002682, BRACE2002695, BRACE2002707, BRACE2002736, BRACE2002752, BRACE2002762, BRACE2002772, BRACE2002792, BRACE2002796, BRACE2002803, BRACE2002807, BRACE2002834, BRACE2002849, BRACE2002857, BRACE2002861, BRACE2002884, BRACE2002896, BRACE2002948, BRACE2003078, BRACE2003097, BRACE2003110, BRACE2003168, BRACE2003199, BRACE2003210, BRACE2003217, BRACE2003269, BRACE2003285, BRACE2003319, BRACE2003398, BRACE2003420, BRACE2003428, BRACE2003431, BRACE2003512, BRACE2003516, BRACE2003539,
BRACE2003540,. BRACE2003598, BRACE2003609, BRACE2003614, BRACE2003639, BRACE2003700, BRACE2003713, BRACE2003766; BRACE2003802, BRACE2003825, BRACE2003847, BRACE2003873, BRACE2003885, BRACE2003887, BRACE2003904, BRACE2003905, BRACE2003928, BRACE2003954, BRACE2003987, BRACE2005034, BRACE2005047, BRACE2005063, BRACE2005083, BRACE2005090, BRACE2005118, BRACE2005124, BRACE2005138, BRACE2005151, BRACE2005160, BRACE2005169, BRACE2005189, BRACE2005193, BRACE2005196, BRACE2005198, BRACE2005216, BRACE2005243, BRACE2005251, BRACE2005253, BRACE2005290, BRACE2005328, BRACE2005329, BRACE2005337, BRACE2005348, BRACE2005363, BRACE2005434, BRACE2005448, BRACE2005450, BRACE2005453, BRACE2005457, BRACE2005460,
BRACE2005489, BRACE2005518, BRACE2005556, BRACE2005562, BRACE2005624, BRACE2005642, BRACE2005661, BRACE2005667, BRACE2005681, BRACE2005719, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006072, BRACE2006075, BRACE2006089, BRACE2006122, BRACE2006137, BRACE2006143, BRACE2006167, BRACE2006174, BRACE2006240, BRACE2006245, BRACE2006274, BRACE2006281, BRACE2006298, BRACE2006344, BRACE2006354, BRACE2006380, BRACE2006393, BRACE2006413, BRACE2006417, BRACE2006453,
BRACE2006459, BRACE2006488, BRACE2006534, BRACE2006540, BRACE2006587, BRACE2006598, BRACE2006636, BRACE2006685, BRACE2006703, BRACE2006743, BRACE2006783, BRACE2006821, BRACE2006833, BRACE2006859, BRACE2006909, BRACE2006911, BRACE2006924, BRACE2006935, BRACE2006944, BRACE2006951, BRACE2006986, BRACE2007013, BRACE2007087, BRACE2007138, BRACE2007197, BRACE2007201, BRACE2007203, BRACE2007232, BRACE2007262, BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007577, BRACE2007621, BRACE2007640, BRACE2007641, BRACE2007646,
BRACE2007663, BRACE2007671, BRACE2007682, BRACE2007685, BRACE2007708, BRACE2007727, BRACE2007749, BRACE2007760, BRACE2007761, BRACE2007764, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008, BRACE2008015, BRACE2008021, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008361, BRACE2008401, BRACE2008410, BRACE2008420, BRACE2008443, BRACE2008473, BRACE2008480, BRACE2008481, BRACE2008488, BRACE2008495,
BRACE2008500, BRACE2008553, BRACE2008594, BRACE2008604, BRACE2008615, BRACE2008622, BRACE2008629, BRACE2008655, BRACE2008656, BRACE2008708, BRACE2008710, BRACE2008754, BRACE2008797, BRACE2008847, BRACE2008857, BRACE2008861, BRACE2008867, BRACE2008881, BRACE2008883, BRACE2008917, BRACE2008919, BRACE2008941, BRACE2008948, BRACE2008960, BRACE2008999, BRACE2009014, BRACE2009016, BRACE2009031, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009293, BRACE2009307, BRACE2009311, BRACE2009318, BRACE2009323, BRACE2009366, BRACE2009421,
BRACE2009428, BRACE2009434, BRACE2009437, BRACE2009517, BRACE2009533, BRACE2009566, BRACE2009591, BRACE2009654, BRACE2009721, BRACE2009732, BRACE2009754, BRACE2009828, BRACE2009886, BRACE2009907, BRACE2009957, BRACE2010170, BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010753, BRACE-2010813, BRACE2010828, BRACE2010837, BRACE2010888, BRACE2010937, BRACE2010983, BRACE2011007, BRACE2011025, BRACE2011199, BRACE2011389, BRACE2011478, BRACE2011545, BRACE2011592, BRACE2011611, BRACE2011646, BRACE2011677, BRACE2011703, BRACE2011747, BRACE2011838,
BRACE2011892, BRACE2011904, BRACE2011947, BRACE2012061, BRACE2012062, BRACE2012185, BRACE2012222, BRACE2012232, BRACE2012291, BRACE2012510, BRACE2012625, BRACE2012653, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2014108, BRACE2014161, BRACE2014232, BRACE2014268, BRACE2014475, BRACE2014657, BRACE2014780, BRACE2014818, BRACE2014821, BRACE2014824, BRACE2014854, BRACE2015031, BRACE2015132, BRACE2015262, BRACE2015270, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315,
BRACE2016325, BRACE2016335, BRACE2016366, BRACE2016465, BRACE2016516, BRACE2016583, BRACE2016708, BRACE2016821, BRACE2016896, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017992, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019147, BRACE2019244, BRACE2019258, BRACE2019327, BRACE2019348, BRACE2019371, BRACE2019467, BRACE2019510, BRACE2019528, BRACE2019618, BRACE2019696, BRACE2019703, BRACE2019843, BRACE2019865, BRACE2019872, BRACE2020028,
BRACE2020077, BRACE2020097, BRACE2020157, BRACE2020278, BRACE2020358, BRACE2020467, BRACE2020584, BRACE2020652, BRACE2020709, BRACE2020742, BRACE2020756, BRACE2020863, BRACE2020982, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021721, BRACE2021932, BRACE2021936, BRACE2022030, BRACE2022158, BRACE2022196, BRACE2022270, BRACE2022328, BRACE2022333, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070, BRACE2023223, BRACE2023268, BRACE2023284, BRACE2023404,
BRACE2023451, BRACE2023540, BRACE2023633, BRACE2023718, BRACE2023744, BRACE2023800, BRACE2024222, BRACE2024610, BRACE2024627, BRACE2024781, BRACE2024826, BRACE2025018, BRACE2025316, BRACE2025333, BRACE2025452, BRACE2025492, BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026836, BRACE2026920, BRACE2026971, BRACE2027018, BRACE2027221, BRACE2027258, BRACE2027312, BRACE2027382, BRACE2027389, BRACE2027408, BRACE2027767, BRACE2027770, BRACE2027879, BRACE2027896, BRACE2028171, BRACE2028392, BRACE2028410, BRACE2028636, BRACE2028741, BRACE2028956, BRACE2028970, BRACE2029112, BRACE2029396,
BRACE2029581, BRACE2029849, BRACE2030039, BRACE2030096, BRACE2030326, BRACE2030341, BRACE2030736, BRACE2030883, BRACE2030884, BRACE2031154, BRACE2031232, BRACE2031527, BRACE2031531, BRACE2031553, BRACE2031899, BRACE2032044, 0BRACE2032090, BRACE2032134, BRACE2032148, BRACE2032182, BRACE2032329, BRACE2032385, BRACE2032436, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622, BRACE2034870, BRACE2035003, BRACE2035120, BRACE2035124, BRACE2035191, BRACE2035278, BRACE2035441, BRACE2035485, BRACE2036005, BRACE2036096, BRACE2036743, BRACE2036830, BRACE2036834, BRACE2036900,
BRACE2037132, BRACE2037294, BRACE2037295, BRACE2037299, BRACE2037310, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039734, BRACE2039823, BRACE2039832, BRACE2039893, BRACE2039986, BRACE2040138, BRACE2040325, BRACE2040514, BRACE2040640, BRACE2040987, BRACE2041009, BRACE2041095, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142,
BRACE2043248, BRACE2043349, BRACE2043381, BRACE2043774, BRACE2043942, BRACE2044057, BRACE2044105, BRACE2044263, BRACE2044304, BRACE2044473, BRACE2044510, BRACE2044640, BRACE2044816, BRACE2044946, BRACE2044949, BRACE2045300, BRACE2045428, BRACE2045445, BRACE2045596, BRACE2045772, BRACE2045947, BRACE2045954, BRACE2046251, BRACE2046295, BRACE2046976, BRACE2047011, BRACE2047232, BRACE2047377, BRACE2047385, BRACE2047492, BRACE2047558, BRACE2047573, BRACE2047835, BRACE2047893, BRACE2047975, BRACE3000071, BRACE3000404, BRACE3000520, BRACE3000697, BRACE3000840, BRACE3001002, BRACE3001058, BRACE3001066, BRACE3001113, BRACE3001217, BRACE3001299, BRACE3001384, BRACE.3001391, BRACE3001403, BRACE3001595,
BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003595, BRACE3003866, BRACE3003920, BRACE3004046, BRACE3004058, BRACE3004113, BRACE3004150, BRACE3004205, BRACE3004358, BRACE3004371. BRACE3004435, BRACE3004477, BRACE3004767, BRACE3004772, BRACE3004783, BRACE3004843, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005760, BRACE3005870, BRACE3005903, BRACE3006113, BRACE3006185, BRACE3006226, BRACE3006462, BRACE3006463, BRACE3006872,
BRACE3006917, BRACE3007058, BRACE3007084, BRACE3007085, BRACE3007322, BRACE3007472, BRACE3007480, BRACE3007559, BRACE3007625, BRACE3007642, BRACE3007649, BRACE3007767, BRACE3007869, BRACE3008066, BRACE3008092, BRACE3008137, BRACE3008255, BRACE3008337, BRACE3008384, BRACE3008720, BRACE3008772, BRACE3009044, BRACE3009075, BRACE3009090, BRACE3009127, BRACE3009197, BRACE3009237, BRACE3009265, BRACE3009297, BRACE3009377, BRACE3009392, BRACE3009539, BRACE3009574, BRACE3009701, BRACE3009708, BRACE3009724, BRACE3009993, BRACE3010076, BRACE3010079, BRACE3010086, BRACE3010221, BRACE3010315, BRACE3010397, BRACE3010416, BRACE3010435, BRACE3010702, BRACE3010862, BRACE3011271, BRACE3011447, BRACE3011505,
BRACE3011634, BRACE3011774, BRACE3012357, BRACE3012574, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714, BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3018083, BRACE3018308, BRACE3018963, BRACE3019055, BRACE3019071, BRACE3019084, BRACE3019570, BRACE3019611, BRACE3019817, BRACE3019828, BRACE3020151,
BRACE3020194, BRACE3020286, BRACE3020356, BRACE3020594, BRACE3020671, BRACE3020890, BRACE3021148, BRACE3021430, BRACE3021517, BRACE3021805, BRACE3022051, BRACE3022303, BRACE3022340, BRACE3022847, BRACE3023604, BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027480, BRACE3027931, BRACE3028214, BRACE3028241, BRACE3028360,
BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184, BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032771, BRACE3032980, BRACE3032983, BRACE3033525, BRACE3034159, BRACE3034183, BRACE3034389, BRACE3034964, BRACE3034993, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239,
BRACE3040504, BRACE3040644, BRACE3040856, BRACE3041059, BRACE3041162, BRACE3041827, BRACE3042046, BRACE3042210, BRACE3042409, BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2001279, BRALZ2001350, BRALZ2001445, BRALZ2001559, BRALZ2001743, BRALZ2001834, BRALZ2002174,
BRALZ2002418, BRALZ2002477, BRALZ2003119, BRALZ2003330, BRALZ2003453, BRALZ2003525, BRALZ2004397, BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009446, BRALZ2009486, BRALZ2010186, BRALZ2010842, BRALZ2011337, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085,
BRALZ2016498, BRALZ2017359, BRALZ2017531, BRALZ2017607, BRALZ2017620, BRALZ2018342, BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000411, BRAMY2000653, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604, BRAMY2002621,
BRAMY2002638, BRAMY2002739, BRAMY2002799, BRAMY2002853, BRAMY2002862, BRAMY2002956, BRAMY2003037, BRAMY2003117, BRAMY2003287, BRAMY20-03325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005648, BRAMY2005662, BRAMY2005708, BRAMY2005711, BRAMY2006092, BRAMY2006118, BRAMY2006149, BRAMY2006397,
BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563,. BRAMY2008583, BRAMY2008977, BRAMY2008979, BRAMY2009034, BRAMY2009105, BRAMY2009123, BRAMY2009192, BRAMY2009344, BRAMY2009349, BRAMY2009394, BRAMY2009489, BRAMY2009508, BRAMY2009528, BRAMY2009557, BRAMY2009612, BRAMY2009693, BRAMY2009934, BRAMY2010068, BRAMY2010135, BRAMY2010145,
BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012691, BRAMY2012721, BRAMY2012731, BRAMY2012776, BRAMY2012880, BRAMY2012881, BRAMY2013200, BRAMY2013216, BRAMY2013405,
BRAMY2013414, BRAMY2013572, BRAMY2013621, BRAMY2013659, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2015251, BRAMY2015311, BRAMY2015420, BRAMY2015503, BRAMY2015516, BRAMY2015551, BRAMY2015559, BRAMY2015688, BRAMY2015705, BRAMY2015855, BRAMY2015890, BRAMY2015911, BRAMY2015925, BRAMY2016002, BRAMY2016020, BRAMY2016070, BRAMY2016251, BRAMY2016325, BRAMY2016327, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2018027, BRAMY2018106, BRAMY2018126, BRAMY2018272, BRAMY2018273, BRAMY2018279,
BRAMY2018295, BRAMY2018308, BRAMY2018396, BRAMY2018467, BRAMY2018635, BRAMY2019055, BRAMY2019111, BRAMY2019277, BRAMY2019300, BRAMY2019468, BRAMY2019509, BRAMY2019643, BRAMY2019710, BRAMY2019985, BRAMY2020050, BRAMY2020058, BRAMY2020106, BRAMY2020270, BRAMY2020354;.BRAMY2020355, BRAMY2020445, BRAMY2020466, BRAMY2020520, BRAMY2020574, BRAMY2020634, BRAMY2020713, BRAMY2021040, BRAMY2021063, BRAMY2021098, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022263, BRAMY2022301, BRAMY2022320, BRAMY2022383, BRAMY2022454, BRAMY2022525, BRAMY2022532,
BRAMY2022723, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023482, BRAMY2023514, BRAMY2023719, BRAMY2023786, BRAMY2023852, BRAMY2023863, BRAMY2023939, BRAMY2024004, BRAMY2024073, BRAMY2024312, BRAMY2024374, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026091, BRAMY2026168,
BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027140, BRAMY2027242, BRAMY2027396, BRAMY2027451, BRAMY2027461, BRAMY2027467, BRAMY2027714, BRAMY2027717, BRAMY2027752, BRAMY2027782, BRAMY2028072, BRAMY2028183, BRAMY2028188, BRAMY2028269, BRAMY2028282, BRAMY2028472, BRAMY2028491, BRAMY2028565, BRAMY2028593, BRAMY2028783, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859,
BRAMY2030915, BRAMY2031317, BRAMY2031377, BRAMY2031442, BRAMY2032014, BRAMY2032231, BRAMY2032242, BRAMY2032290, BRAMY2032311, BRAMY2032317, BRAMY2032344, BRAMY2032589, BRAMY2032678, BRAMY2032777, BRAMY2032994, BRAMY2033003, BRAMY2033116, BRAMY2033240, BRAMY2033267, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035449, BRAMY2035545, BRAMY2035606, BRAMY2035718, BRAMY2035801, BRAMY2035869, BRAMY2035923, BRAMY2036079, BRAMY2036254, BRAMY2036266, BRAMY2036387, BRAMY2036396, BRAMY2036567, BRAMY2036615, BRAMY2036699, BRAMY2036913, BRAMY2036918, BRAMY2036968, BRAMY2037147,
BRAMY2037328, BRAMY2037609, BRAMY2037629, BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038887, BRAMY2038904, BRAMY2039287, BRAMY2039300, BRAMY2039314, BRAMY2039341, BRAMY2039630, BRAMY2039695, BRAMY2039728, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918,
BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002312, BRAMY300232.9, BRAMY3002508, BRAMY3002620, BRAMY3002803, BRAMY3002805, BRAMY3002886, BRAMY3002991, BRAMY3003026, BRAMY3003109,
BRAMY3003566, BRAMY3003723, BRAMY3004126, BRAMY3004224, BRAMY3004672, BRAMY3004800, BRAMY3004900, BRAMY3004919, BRAMY3005091, BRAMY3005184, BRAMY3005656, BRAMY3005912, BRAMY3005932, BRAMY3006032, BRAMY3006297, BRAMY3006761, BRAMY3007078, BRAMY3007206, BRAMY3007350, BRAMY3007449, BRAMY3007471, BRAMY3007609, BRAMY3007723, BRAMY3007968, BRAMY3008088, BRAMY3008096, BRAMY3008335, BRAMY3008466, BRAMY3008505, BRAMY3008650, BRAMY3008937, BRAMY3009158, BRAMY3009207, BRAMY3009556, BRAMY3009782, BRAMY3009811, BRAMY3009904, BRAMY3010264, BRAMY3010321, BRAMY3010411, BRAMY3010603, BRAMY3010654, BRAMY4000095, BRAMY4000229, BRAMY4000277, BRAMY4000915, BRAMY4000962, BRAMY4001173, BRAMY4001200, BRAMY4001449,
BRAMY4001652, BRAMY4001913, BRAMY4002575, BRASW1000053, BRASW1000125, BRAWH1000002, BRAWH1000094, BRAWH1000116, BRAWH1000130, BRAWH1000157, BRAWH1000164, BRAWH1000168, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256, BRAWH2000274, BRAWH2000323, BRAWH2000353, BRAWH2000370, BRAWH2000409, BRAWH2000443, BRAWH2000471, BRAWH2000476, BRAWH2000482, BRAWH2000500, BRAWH2000503, BRAWH2000522, BRAWH2000554, BRAWH2000595, BRAWH2000643, BRAWH2000651, BRAWH2000682, BRAWH2000752, BRAWH2000866, BRAWH2000892, BRAWH2000901, BRAWH2000926, BRAWH2000984,
BRAWH2001037, BRAWH2001052, BRAWH2001103, BRAWH2001137, BRAWH2001141, BRAWH2001159, BRAWH2001166, BRAWH2001203, BRAWH2001238, BRAWH2001255, BRAWH2001355, BRAWH2001378, BRAWH2001436, BRAWH2001438, BRAWH2001484, BRAWH2001492, BRAWH2001503, BRAWH2001519, BRAWH2001589, BRAWH2001662, BRAWH2001666, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002125, BRAWH2002191, BRAWH2002318, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, .
BRAWH2002967, BRAWH2002976, BRAWH2003000, BRAWH2003025, BRAWH2003135, BRAWH2003307, BRAWH2003355, BRAWH2003366, BRAWH2003662, BRAWH2003832, BRAWH2003948, BRAWH2003964, BRAWH2004044, BRAWH2004068, BRAWH2004078, BRAWH2004580, BRAWH2004731, BRAWH2004779, BRAWH2004884, BRAWH2004923, BRAWH2005074, BRAWH2005315, BRAWH2005517, BRAWH2005524, BRAWH200.5533, BRAWH2005812, BRAWH2005974, BRAWH2005998, BRAWH2006044, BRAWH2006083, BRAWH2006207, BRAWH2006301, BRAWH2006395, BRAWH2006405, BRAWH2006622, BRAWH2006760, BRAWH2006960, BRAWH2006989, BRAWH2006996, BRAWH2007069, BRAWH2007308, BRAWH2007406, BRAWH2007550, BRAWH2007570, BRAWH2007591, BRAWH2007658, BRAWH2007664, BRAWH2007800, BRAWH2007825, BRAWH2007862,
BRAWH2007890, BRAWH2008128, BRAWH2008219, BRAWH2008255, BRAWH2008292, BRAWH2008297, BRAWH2008366, BRAWH2008380, BRAWH2008577, BRAWH2008790, BRAWH2008903, BRAWH2008993, BRAWH2009112, BRAWH2009227, BRAWH2009261, BRAWH2009263, BRAWH2009298, BRAWH2009393, BRAWH2009485, BRAWH2009490, BRAWH2009558, BRAWH2009590, BRAWH2009626, BRAWH2009695, BRAWH2009907, BRAWH2010000, BRAWH2010069, BRAWH2010084, BRAWH2010136, BRAWH2010318, BRAWH2010364, BRAWH2010536, BRAWH2010552, BRAWH2010584, BRAWH2010618, BRAWH2010619, BRAWH2010754, BRAWH2010980, BRAWH2011066, BRAWH2011079, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812,
BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326,. BRAWH2012538, BRAWH2012540, BRAWH2012619, BRAWH2012698, BRAWH2012749, BRAWH2012827, BRAWH2012866, BRAWH2012963, BRAWH2013047, BRAWH2013748, BRAWH2013866, BRAWH2013914, BRAWH2013941, BRAWH2013955, BRAWH2014234, BRAWH2014379, BRAWH2014414, BRAWH2014473, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016221, BRAWH2016223,
BRAWH2016269, BRAWH2016305, BRAWH2016439, BRAWH2016446, BRAWH2016562, BRAWH2016655, BRAWH2016679, BRAWH2016702, BRAWH2016724, BRAWH2016785, BRAWH2016792, BRAWH2016800, BRAWH2017103, BRAWH2017250, BRAWH2017294, BRAWH2017304, BRAWH2017305, BRAWH2017379, BRAWH2017407, BRAWH2017433, BRAWH2017523, BRAWH2017534, BRAWH2017542, BRAWH2017635, BRAWH2017913, BRAWH2018267, BRAWH2018282, BRAWH2018317, BRAWH2018506, BRAWH2018514, BRAWH2018526, BRAWH2018601, BRAWH2018729, BRAWH2018745, BRAWH2018875, BRAWH2019053, BRAWH2019055, BRAWH2019198, BRAWH3000078, BRAWH3000100, BRAWH3000166, BRAWH3000314, BRAWH3000329, BRAWH3000410, BRAWH3000446, BRAWH3000491, BRAWH3000884, BRAWH3001053, BRAWH3001326, BRAWH3001475,
BRAWH3001636, BRAWH3001638, BRAWH3001712, BRAWH3001760, BRAWH3001783, BRAWH3001833, BRAWH3001879, BRAWH3001891, BRAWH3002433, BRAWH3002467, BRAWH3002513, BRAWH3002574, BRAWH3002600, BRAWH3002819, BRAWH3003019, BRAWH3003136, BRAWH3003244, BRAWH3003343, BRAWH3003522, BRAWH3003598, BRAWH3003801, BRAWH3003975, BRAWH3004222, BRAWH3004335, BRAWH3004453, BRAWH3004666, BRAWH3005037, BRAWH3005047, BRAWH3005132, BRAWH3005292, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007783, BRAWH3008167, BRAWH3008341, BRAWH3008559,
BRAWH3008697, BRAWH3008867, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623, BRAWH3011685, BRAWH3011907, BRAWH3011929, BRAWH3012005, BRAWH3012779, BRAWH3013009; BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016271, BRAWH3016715, BRAWH3017259, BRAWH3017260, BRAWH3017477, BRAWH3017537, BRAWH3017980, BRAWH3018369, BRAWH3018548, BRAWH3018969, BRAWH3019026, BRAWH3019529, BRAWH3019594, BRAWH3019629,
BRAWH3019820, BRAWH3020200, BRAWH3020318, BRAWH3020486, BRAWH3020884, BRAWH3020928, BRAWH3021012, BRAWH3021580, BRAWH3021641, BRAWH3021643, BRAWH3021724, BRAWH3022347, BRAWH3022431, BRAWH3022459, BRAWH3022542, BRAWH3022719, BRAWH3022866, BRAWH3022900, BRAWH3023156, BRAWH3023168, BRAWH3023172, BRAWH3023274, BRAWH3023421, BRAWH3024231, BRAWH3024469, BRAWH3024506, BRAWH3024613, BRAWH3026349, BRAWH3026394, BRAWH3026529, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538,
BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620, BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035904, BRAWH3035914, BRAWH3036077, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000105, BRCAN1000149, BRCAN1000168, BRCAN2000148, BRCAN2000149, BRCAN2000239, BRCAN2000346,
BRCAN2000364, BRCAN2000383, BRCAN2000418, BRCAN2000523, BRCAN2000541, BRCAN2000563, BRCAN2000577, BRCAN2000620, BRCAN2000639, BRCAN2000763, BRCAN2000832, BRCAN2000923, BRCAN2001223, BRCAN2001866, BRCAN2002416, BRCAN2002473, BRCAN2002562, BRCAN2002662, BRCAN2002826, BRCAN2002854, BRCAN2002856, BRCAN2002892, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119,
BRCAN2007144, BRCAN2007426, BRCAN2007525, BRCAN2007700,. BRCAN2008494, BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2011946, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015757, BRCAN2015886, BRCAN2016025, BRCAN2016046, BRCAN2016174, BRCAN2016433, BRCAN2016619, BRCAN2016762, BRCAN2016814,
BRCAN2017442, BRCAN2017905, BRCAN2018240, BRCAN2018269, BRCAN2018490, BRCAN2018566, BRCAN2018667, BRCAN2018748, BRCAN2018935, BRCAN2019002, BRCAN2019387, BRCAN2019773, BRCAN2019907, BRCAN2020234, BRCAN2020331, BRCAN2020467, BRCAN2020710, BRCAN2020880, BRCAN2021028, BRCAN2021325, BRCAN2021452, BRCAN2021669,.BRCAN2021718, BRCAN2022191, BRCAN2022472, BRCAN2022474, BRCAN2023347, BRCAN2023724, BRCAN2024451, BRCAN2024563, BRCAN2024572, BRCAN2025009, BRCAN2025093, BRCAN2025349, BRCAN2025368, BRCAN2025712, BRCAN2025982, BRCAN2026117, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036,
BRCAN2028338, BRCAN2028378, BRCAN2028460, BRCAN2028545, BRCAN2028634, BRCAN2028702, BRCOC1000087, BRCOC2000047, BRCOC2000152, BRCOC2000184, BRCOC2000333, BRCOC2000360, BRCOC2000404, BRCOC2000637, BRCOC2000670, BRCOC2000816, BRCOC2000850, BRCOC2001355, BRCOC2002085, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003513, BRCOC2003740, BRCOC2004036, BRCOC2004175, BRCOC2004354, BRCOC2004455, BRCOC2004950, BRCOC2005542, BRCOC2005903, BRCOC2005951, BRCOC2006189, BRCOC2006243, BRCOC2006270, BRCOC2006639, BRCOC2006942, BRCOC2007034, BRCOC2007593, BRCOC2007733, BRCOC2007769, BRCOC2008064, BRCOC2008225,
BRCOC2008642, BRCOC2008993, BRCOC2009052, BRCOC2009196, BRCOC2009380, BRCOC2009638, BRCOC2009937, BRCOC2010115, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012813, BRCOC2013448, BRCOC2013573, BRCOC2013675, BRCOC2013780, BRCOC2014400, BRCOC2014748, BRCOC2014833, BRCOC2015597, BRCOC2015824, BRCOC2015908, BRCOC2015944, BRCOC2016525, BRCOC2016661, BRCOC2017061, BRCOC2017856, BRCOC2018775, BRCOC2018877, BRCOC2019255, BRCOC2019549, BRCOC2019841, BRCOC2019896, BRCOC2019934, BRCOC2020122, BRCOC2020142, BRHIP1000058, BRHIP1000072, BRHIP1000129, BRHIP1000174, BRHIP1000258, BRHIP2000021,
BRHIP2000079, BRHIP2000163, BRHIP2000210, BRHIP2000312, BRHIP2000359, BRHIP2000445, BRHIP2000506, BRHIP2000532, BRHIP2000621, BRHIP2000691, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003242, BRHIP2003272, BRHIP2003748, BRHIP2003786, BRHIP2003828, BRHIP2003917, BRHIP2004312, BRHIP2004355, BRHIP2004814, BRHIP2004883, BRHIP2004902, BRHIP2005010, BRHIP2005236, BRHIP2005354, BRHIP2005600, BRHIP2005719, BRHIP2005724, BRHIP2005752, BRHIP2005932, BRHIP2006617, BRHIP2006782,
BRHIP2006800, BRHIP2006819, BRHIP2006921, BRHIP2007307, BRHIP2007616, BRHIP2007690, BRHIP2007928, BRHIP2007969, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2010217, BRHIP2010309, BRHIP2010444, BRHIP2010487, BRHIP2010610, BRHIP2010723, BRHIP2010744, BRHIP2011080, BRHIP2011120, BRHIP2011491, BRHIP2011576, BRHIP2011616, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447,
BRHIP2013510, BRHIP2013699, BRHIP2013723, BRHIP2013958, BRHIP2013972, BRHIP2014063, BRHIP2014228, BRHIP2014285, BRHIP2014291, BRHIP2014387, BRHIP2014954, BRHIP2015224, BRHIP2015356, BRHIP2015360, BRHIP2016005, BRHIP2016125, BRHIP2016788, BRHIP2016968, BRHIP2016990, BRHIP2017315, BRHIP2017385, BRHIP2017404, BRHIP2017542, BRHIP2017553, BRHIP2017642, BRHIP2017714, BRHIP2017780, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019149, BRHIP2019177, BRHIP2019186, BRHIP2019291, BRHIP2019494, BRHIP2019589, BRHIP2019641, BRHIP2019884, BRHIP2019972, BRHIP2020182, BRHIP2020622, BRHIP2020695, BRHIP2020743,
BRHIP2020842, BRHIP2020859, BRHIP2020930, BRHIP2021102, BRHIP2021289, BRHIP2021495, BRHIP2021615, BRHIP2021744, BRHIP2021762, BRHIP2021858, BRHIP2021870, BRHIP2021929, BRHIP2022006, BRHIP2022054, BRHIP2022221, BRHIP2022298, BRHIP2022467, BRHIP2022708, BRHIP2023071, BRHIP2023229, BRHIP2023292, BRHIP2023695, BRHIP2023735, BRHIP2023762, BRHIP2023773, BRHIP2023860, BRHIP2023869, BRHIP2024165, BRHIP2024201, BRHIP2024347, BRHIP2024549, BRHIP2024789, BRHIP2024911, BRHIP2024941, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027054, BRHIP2027563,
BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393, BRHIP2029529, BRHIP2029643, BRHIP2029663, BRHIP3000060, BRHIP3000070, BRHIP3000081, BRHIP3000240, BRHIP3000377, BRHIP3000457, BRHIP3000488, BRHIP3000526, BRHIP3000626, BRHIP3000859, BRHIP3000907, BRHIP3001076, BRHIP3001079, BRHIP3001141, BRHIP3001283, BRHIP3001338, BRHIP3001360, BRHIP3001481, BRHIP3001878, BRHIP3001964, BRHIP3002124, BRHIP3002363, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193,
BRHIP3004215, BRHIP3004416, BRHIP3004710, BRHIP3004725, BRHIP3004774, BRHIP3004786, BRHIP3004863, BRHIP3004968, BRHIP3005037, BRHIP3005137, BRHIP3005142, BRHIP3005231, BRHIP3005307, BRHIP3005574, BRHIP3005768, BRHIP3005801, BRHIP3005944, BRHIP3006279, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007586, BRHIP3007609, BRHIP3007640, BRHIP3007960, BRHIP3008082, BRHIP3008183, BRHIP3008314, BRHIP3008320, BRHIP3008344, BRHIP3008565, BRHIP3008598, BRHIP3008606, BRHIP3008691, BRHIP3008714, BRHIP3008997, BRHIP3009099, BRHIP3009140, BRHIP3009181,
BRHIP3009318, BRHIP300942.7, BRHIP3009448, BRHIP3009672, BRHIP3009753, BRHIP3010135, BRHIP3010289, BRHIP3010529, BRHIP3011082, BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271, BRHIP3013588, BRHIP3013698, BRHIP3013765, BRHIP3013897, BRHIP3014237, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3019643, BRHIP3019824, BRHIP3019880, BRHIP3019956, BRHIP3020155, BRHIP3020182, BRHIP3021019, BRHIP3021499, BRHIP3021534, BRHIP3021778,
BRHIP3021987, BRHIP3022656, BRHIP3023922, BRHIP3024118, BRHIP3024533, BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000175, BRSSN2000293, BRSSN2000312, BRSSN2000518, BRSSN2000561, BRSSN2000566, BRSSN2000715, BRSSN2001213, BRSSN2001275, BRSSN2001342, BRSSN2001426, BRSSN2001503, BRSSN2001758, BRSSN2001810, BRSSN2002857, BRSSN2002859, BRSSN2003086, BRSSN2003093,
BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2006133, BRSSN2006390, BRSSN2006575, BRSSN2006611, BRSSN2006644, BRSSN2007076, BRSSN2007168, BRSSN2007202, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010110, BRSSN2010587, BRSSN2010596, BRSSN2010830, BRSSN2011653, BRSSN2012081, BRSSN2012103, BRSSN2012157, BRSSN2012198, BRSSN2012254, BRSSN2012622, BRSSN2012691, BRSSN2012838, BRSSN2013095, BRSSN2013544, BRSSN2013696, BRSSN2013733,
BRSSN2013753, BRSSN2013874, BRSSN2014112, BRSSN2014294, BRSSN2014424, BRSSN2014513, BRSSN2014556, BRSSN2014573, BRSSN2014636, BRSSN2015199, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017422, BRSSN2017530, BRSSN2017682, BRSSN2017757, BRSSN2018218, BRSSN2018440, BRSSN2018731, BRSSN2018771, BRSSN2018925, BRSTN1000097, BRSTN2000070, BRSTN2000220, BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004987, BRSTN2005721, BRSTN2006306,
BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012284, BRSTN2012320, BRSTN2012380, BRSTN2013171, BRSTN2013354, BRSTN2014633, BRSTN2014751, BRSTN2014770, BRSTN2015015, BRSTN2016066, BRSTN2016335, BRSTN2016678, BRSTN2016892, BRSTN2016918, BRSTN2017151, BRSTN2017237, BRSTN2017379, BRSTN2017771, BRSTN2017995, BRSTN2018077, BRSTN2018083, BRSTN2018596, BRSTN2018692,
BRSTN2018712, BRSTN2019079, BRSTN2019129, BRTHA1000021, BRTHA1000063, BRTHA2000057, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565, BRTHA2002608, BRTHA2002702, BRTHA2002721, BRTHA2002726, BRTHA2002741, BRTHA2002808,. BRTHA2002896, BRTHA2002903, BRTHA2003030, BRTHA2003110, BRTHA2003116, BRTHA2003436, BRTHA2003759, BRTHA2003794, BRTHA2004361, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005,
BRTHA2005448, BRTHA2005579, BRTHA2005831, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2008316, BRTHA2008502, BRTHA2008513, BRTHA2008527, BRTHA2008535, BRTHA2008598, BRTHA2008669, BRTHA2008955, BRTHA2009311, BRTHA2009349, BRTHA2009486, BRTHA2009803, BRTHA2009846, BRTHA2009972, BRTHA2010008, BRTHA2010033, BRTHA2010073, BRTHA2010097, BRTHA2010191, BRTHA2010397, BRTHA2010608, BRTHA2010672, BRTHA2010884, BRTHA2010907, BRTHA2011062, BRTHA2011194, BRTHA2011321, BRTHA2011351, BRTHA2011500, BRTHA2011641, BRTHA2012129, BRTHA2012183,
BRTHA2012189, BRTHA2012351, BRTHA2012392, BRTHA2012466, BRTHA2012555, BRTHA2012562, BRTHA2013262, BRTHA2013426, BRTHA2013460, BRTHA2013515, BRTHA2013595, BRTHA2013610, BRTHA2013707, BRTHA2013942, BRTHA2014287, BRTHA2014334, BRTHA2014381, BRTHA2014578, BRTHA2014647, BRTHA2014792, BRTHA2014828, BRTHA2014909, BRTHA2014975, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016765, BRTHA2017178, BRTHA2017353, BRTHA2017364, BRTHA2017972, BRTHA2018011, BRTHA2018129, BRTHA2018165, BRTHA2018304, BRTHA2018344, BRTHA2018420, BRTHA2018443, BRTHA2018558, BRTHA2018591,
BRTHA2018624, BRTHA2018706, BRTHA2019014, BRTHA2019022, BRTHA2019048, BRTHA2019726, BRTHA2019743, BRTHA2020400, BRTHA2020566, BRTHA2020642, BRTHA2020695, BRTHA2020721, BRTHA2020781, BRTHA2020811, BRTHA2020910, BRTHA2021212, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874,
BRTHA2035448, BRTHA2036055, BRTHA2036295, BRTHA2036660, BRTHA2037247, BRTHA2038279, BRTHA2038345, BRTHA2038353, BRTHA2038396, BRTHA3000252, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002933, BRTHA3002955, BRTHA3003000, BRTHA3003023, BRTHA3003074, BRTHA3003225, BRTHA3003339, BRTHA3003343, BRTHA3003449, BRTHA3003474, BRTHA3003704, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007532, BRTHA3007662, BRTHA3007685,
BRTHA3007769, BRTHA3007922, BRTHA3008143, BRTHA3008293, BRTHA3008310, BRTHA3008386, BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010540, BRTHA3010717, BRTHA3011149, BRTHA3011187, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514,
BRTHA3018617, BRTHA3018623, BRTHA3018656, BRTHA3019048, BRTHA3019105, BRTHA3019183, BRTHA3020000, BRTHA3020369, BRTHA3021569, BRTHA3021708, BRTHA3021786, BRTHA3023403, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026507, BRTHA3026556, BRTHA3026916, BRTHA3027171, BRTHA3027318, BRTHA3027638, BRTHA3027820, BRTHA3027879, BRTHA3027957, BRTHA3028339, BRTHA3028505, BRTHA3028782, CD34C2000051, CD34C2000152, CD34C2000175, CD34C2000443, CD34C3000125, CD34C3000314, CD34C3000424, CD34C3000494, CERVX1000042, CERVX2000812, CERVX2000968, CERVX2002006, CERVX2002013, CERVX2002430, CERVX2002770, CHONS1000058,
CHONS2000172, CHONS2001287, CHONS2001382, CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002520, COLON2003043, COLON2003529, COLON2004318, COLON2004351, COLON2004478, COLON2004911; COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000020, CTONG1000022, CTONG1000040, CTONG1000056, CTONG1000062, CTONG1000086, CTONG1000088, CTONG1000093, CTONG1000097, CTONG1000113, CTONG1000137, CTONG1000165,
CTONG1000180, CTONG1000181, CTONG1000220, CTONG1000277, CTONG1000283, CTONG1000302, CTONG1000341, CTONG1000476, CTONG1000488, CTONG1000508, CTONG1000540, CTONG2000031, CTONG2000034, CTONG2000042, CTONG2000218, CTONG2000279, CTONG2000303, CTONG2000330, CTONG2000395, CTONG2000452, CTONG2000480, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001413, CTONG2001513, CTONG2001524, CTONG2001533, CTONG2001598, CTONG2001681, CTONG2001724, CTONG2001749, CTONG2001800, CTONG2001820, CTONG2001877, CTONG2001911, CTONG2001932, CTONG2001955, CTONG2002066, CTONG2002095, CTONG2002143, CTONG2002417,
CTONG2002418, CTONG2002453, CTONG2002520, CTONG2002558, CTONG2002590, CTONG2002709, CTONG2002766, CTONG2002816, CTONG2002832, CTONG2002841, CTONG2002965, CTONG2002999, CTONG2003028, CTONG2003115, CTONG2003189, CTONG2003245, CTONG2003293, CTONG2003348, CTONG2003350, CTONG2003361, CTONG2003372, CTONG2003375, CTONG2003427, CTONG2003517, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004454, CTONG2004550, CTONG2004669, CTONG2004716, CTONG2004912, CTONG2004941, CTONG2004948, CTONG2005049, CTONG2005110, CTONG2005145, CTONG2005265,
CTONG2005290, CTONG2005374, CTONG2005399, CTONG2005567, CTONG2005585, CTONG2005635, CTONG2005775, CTONG2005795, CTONG2005890, CTONG2005904, CTONG2005913, CTONG2006129, CTONG2006223, CTONG2006273, CTONG2006310, CTONG2006393, CTONG2006412, CTONG2006449, CTONG2006515, CTONG2006524, CTONG2006568, CTONG2006611, CTONG2006620, CTONG2006691, CTONG2006836, CTONG2006942, CTONG2006964, CTONG2007009, CTONG2007091, CTONG2007104, CTONG2007175, CTONG2007259, CTONG2007272, CTONG2007293, CTONG2007297, CTONG2007399, CTONG2007474, CTONG2007613, CTONG2007623, CTONG2007681, CTONG2007779, CTONG2007898, CTONG2007959, CTONG2007970, CTONG2008014, CTONG2008184, CTONG2008262, CTONG2008269, CTONG2008321, CTONG2008343,
CTONG2008402, CTONG2008405, CTONG2008518, CTONG2008521, CTONG2008562, CTONG2008577, CTONG2008595, CTONG2008689, CTONG2008721, CTONG2008773, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423, CTONG2009440, CTONG2009527, CTONG2009529, CTONG2009531, CTONG2009570, CTONG2009572, CTONG2009604, CTONG2009643, CTONG2009675, CTONG2009689, CTONG2009725, CTONG2009766, CTONG2009768, CTONG2009871, CTONG2009887, CTONG2009891, CTONG2009923, CTONG2009955, CTONG2009958, CTONG2010018, CTONG2010024, CTONG2010116, CTONG2010133, CTONG2010148, CTONG2010276, CTONG2010330, CTONG2010508, CTONG2010566,
CTONG2010623, CTONG2010803, CTONG2011188, CTONG2011429, CTONG2011474, CTONG2011676, CTONG2011716, CTONG2011765, CTONG2011779, CTONG2011815, CTONG2011822, CTONG2011920, CTONG2011985, CTONG2012029, CTONG2012077, CTONG2012101, CTONG2012123, CTONG2012158, CTONG2012230, CTONG2012425, CTONG2012447, CTONG2012473, CTONG2012476, CTONG2012533, CTONG2012554, CTONG2012564, CTONG2012724, CTONG2012738, CTONG2012758, CTONG2012843, CTONG2012879, CTONG2012901, CTONG2013128, CTONG2013149, CTONG2013156, CTONG2013178, CTONG2013222, CTONG2013284, CTONG2013339, CTONG2013352, CTONG2013381, CTONG2013393, CTONG2013511, CTONG2013630, CTONG2013803, CTONG2013985, CTONG2013986, CTONG2014032, CTONG2014058, CTONG2014191,
CTONG2014206, CTONG2014234, CTONG2014264, CTONG2014369, CTONG2014630, CTONG2014635, CTONG2014646, CTONG2014705, CTONG2014946, CTONG2014959, CTONG2014966, CTONG2014995, CTONG2015204, CTONG2015316, CTONG2015330, CTONG2015358, CTONG2015434, CTONG2015518, CTONG2015540, CTONG2015596, CTONG2015633, CTONG2015717, CTONG2015804, CTONG2015815, CTONG2015866, CTONG2015914, CTONG2015953, CTONG2016217, CTONG2016355, CTONG2016408, CTONG2016499, CTONG2016559, CTONG2016658, CTONG2016775, CTONG2016797, CTONG2016824, CTONG2016846, CTONG2016904, CTONG2016942, CTONG2016953, CTONG2017021, CTONG2017094, CTONG2017292, CTONG2017409, CTONG2017444, CTONG2017458, CTONG2017604, CTONG2017650, CTONG2017804, CTONG2017814,
CTONG2017910, CTONG2017939, CTONG2017989, CTONG2018062, CTONG2018069, CTONG2018147, CTONG2018217, CTONG2018279, CTONG2018343, CTONG2018379, CTONG2018383, CTONG2018413, CTONG2018483, CTONG2018632, CTONG2018652, CTONG2018653, CTONG2018655, CTONG2018808, CTONG2018900, CTONG2019063, CTONG2019144, CTONG2019232, CTONG2019400, CTONG2019652, CTONG2019833, CTONG2020031, CTONG2020127, CTONG2020171, CTONG2020374, CTONG2020411, CTONG2020445, CTONG2020522, CTONG2020806, CTONG2020831, CTONG2020974, CTONG2021132, CTONG2021168, CTONG2022153, CTONG2022601, CTONG2023021, CTONG2023449, CTONG2023512, CTONG2024206, CTONG2024614, CTONG2024749, CTONG2025496, CTONG2025513, CTONG2025516, CTONG2025610, CTONG2025900,
CTONG2026513, CTONG2026770, CTONG2026920, CTONG2026987, CTONG2027150, CTONG2027263, CTONG2027361, CTONG2027591, CTONG2027736, CTONG2027783, CTONG2027828, CTONG2027959, CTONG2028097, CTONG2028208, CTONG2028486, CTONG2028518, CTONG2028687, CTONG3000686, CTONG3000707, CTONG3000896, CTONG3001420, CTONG3001501, CTONG3001560, CTONG3001605, CTONG3002020, CTONG3002370, CTONG3002412, CTONG3002433, CTONG3002552, CTONG3002588, CTONG3002728, CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003972, CTONG3004317, CTONG3004397, CTONG3004550, CTONG3004607, CTONG3004712, CTONG3004726,
CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009239, CTONG3009287, CTONG3009328, CTONG3009385, D3OST1000066, D3OST1000109, D3OST1000238 D3OST1000267 D3OST10002 D3OST2000184, D3OST2000618 D3OST2000654 D3OST2001328 D3OST2001669 D3OST2002182 D3OST2002223, D3OST2002262 D3OST2002272 D3OST2002417 D3OST2002436 D3OST2002648 D3OST2003024, D3OST2003331,
D3OST2003607, D3OST2003797, D3OST2004637, D3OST3000169, D3OST3000195, D3OST3000388, D3OST3000493, D6OST2000127, D6OST2000169, D6OST2000245, D6OST2000507, D9OST2000031, D9OST2000278, D9OST2000440, D9OST2000869, D9OST2001319, D9OST2001433 D9OST2002397 D9OST2002673 D9OST2003106, D9OST2003108, D9OST2003137 D9OST2003397 D9OST2003580, D9OST2003594 D9OST2003762 D9OST2003989 D9OST2004018 D9OST2004073 D9OST2004417 DFNES1000003, DFNES1000107, DFNES1000185, DFNES2000143, DFNES2000146, DFNES2000153, DFNES2000212, DFNES2000268, DFNES2000292, DFNES2000335, DFNES2000432, DFNES2000457, DFNES2000471, DFNES2000913, DFNES2001091, DFNES2001096, DFNES2001108, DFNES2001177, DFNES2001564, DFNES2001565,
DFNES2001647, DFNES2001800, DFNES2001829, DFNES2002220, DFNES2002509, DFNES2002550, DFNES2002588, DFNES2002817, DFNES2002966, DFNES2003081, DFNES2003192, DFNES2003255, DFNES2004354, DFNES2004383, DFNES2004608, DFNES2004684, DFNES2005266, DFNES2005527, DFNES2005779, DFNES2005875, DFNES2006944, DFNES2007299, DFNES2007641, DFNES2007757, DFNES2008280, DFNES2008881, DFNES2010502, DFNES2011192, DFNES2011239, DFNES2011245, DFNES2011380, ERLTF1000021, ERLTF2000324, ERLTF2000465, FCBBF1000023, FCBBF1000069, FCBBF1000117, FCBBF1000118, FCBBF1000121, FCBBF1000125, FCBBF1000131, FCBBF1000155, FCBBF1000157, FCBBF1000171, FCBBF1000174, FCBBF1000182, FCBBF1000198, FCBBF1000206, FCBBF1000220, FCBBF1000232,
FCBBF1000233, FCBBF1000243, FCBBF1000270, FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000449, FCBBF1000476, FCBBF1000506, FCBBF1000546, FCBBF1000550, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000691, FCBBF1000732, FCBBF1000748, FCBBF2000038, FCBBF2000094, FCBBF2000105, FCBBF2000123, FCBBF2000195, FCBBF2000214, FCBBF2000232, FCBBF2000259, FCBBF2000276, FCBBF2000291, FCBBF2000362, FCBBF2000379, FCBBF2000473, FCBBF2000502, FCBBF2000566,
FCBBF2000576, FCBBF2000591, FCBBF2000677, FCBBF2000678, FCBBF2000685, FCBBF2000733, FCBBF2000808, FCBBF2000815, FCBBF2000825, FCBBF2000940, FCBBF2000951, FCBBF2001001, FCBBF2001105, FCBBF2001116, FCBBF2001188, FCBBF2001211, FCBBF2001238, FCBBF2001243, FCBBF2001299, FCBBF2001427, FCBBF2001480, FCBBF2001529, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002370, FCBBF2002541, FCBBF2002626, FCBBF2002897, FCBBF2002898, FCBBF2002904, FCBBF2002953, FCBBF2003083, FCBBF2003102, FCBBF2003269, FCBBF2003293, FCBBF2003297, FCBBF2003395, FCBBF2003528, FCBBF2003543, FCBBF2003549, FCBBF2003567, FCBBF2003636,
FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005966, FCBBF2006131, FCBBF2006380, FCBBF2006418, FCBBF2006452, FCBBF2006634, FCBBF2006781, FCBBF2007012, FCBBF2007186, FCBBF2007265, FCBBF2007302, FCBBF2007610, FCBBF2007633, FCBBF2007840, FCBBF3000102, FCBBF3000120, FCBBF3000168, FCBBF3000175, FCBBF3000227, FCBBF3000229, FCBBF3000233, FCBBF3000269, FCBBF3000361, FCBBF3000367, FCBBF3000388, FCBBF3000462, FCBBF3000473, FCBBF3000501, FCBBF3000518,
FCBBF3000550, FCBBF3000847, FCBBF3001018, FCBBF3001020, FCBBF3001235, FCBBF3001281, FCBBF3001377, FCBBF3001594, FCBBF3001632, FCBBF3001657, FCBBF3001855, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557, FCBBF3003787, FCBBF3003902, FCBBF3004021, FCBBF3004041, FCBBF3004261, FCBBF3004323, FCBBF3004390, FCBBF3004409, FCBBF3004502, FCBBF3004842, FCBBF3004847, FCBBF3004955, FCBBF3005160, FCBBF3005218, FCBBF3005444, FCBBF3005698,
FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007859, FCBBF3007860, FCBBF3008073, FCBBF3008100, FCBBF3008159, FCBBF3008251, FCBBF3008311, FCBBF3008362, FCBBF3008475, FCBBF3008556, FCBBF3008644, FCBBF3008689, FCBBF3008748, FCBBF3008870, FCBBF3008938, FCBBF3008944, FCBBF3009069, FCBBF3009101, FCBBF3009152, FCBBF3009317, FCBBF3009428, FCBBF3009464, FCBBF3009479, FCBBF3009526, FCBBF3009703, FCBBF3009717, FCBBF3009888, FCBBF3009949, FCBBF3009978, FCBBF3010008, FCBBF3010012,
FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010729, FCBBF3010733, FCBBF3011003, FCBBF3011418, FCBBF3011485, FCBBF3011570, FCBBF3011592, FCBBF3011867, FCBBF3011889, FCBBF3012170, FCBBF3012182, FCBBF3012288, FCBBF3012332, FCBBF3012443, FCBBF3012588, FCBBF3012667, FCBBF3012842, FCBBF3012901, FCBBF3013041, FCBBF3013058, FCBBF3013205, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809,
FCBBF3015870, FCBBF3016018, FCBBF3016134, FCBBF3016178, FCBBF3016451, FCBBF3016618, FCBBF3016622, FCBBF3016644, FCBBF3016987, FCBBF3017059, FCBBF3017071, FCBBF3017123, FCBBF3017233, FCBBF3017255, FCBBF3017288, FCBBF3017396, FCBBF3017535, FCBBF3017681, FCBBF3017729, FCBBF3017873, FCBBF3017974, FCBBF3018067, FCBBF3018173, FCBBF3018453, FCBBF3018591, FCBBF3018753, FCBBF3018826, FCBBF3018949, FCBBF3019085, FCBBF3019437, FCBBF3019455, FCBBF3019564, FCBBF3019569, FCBBF3019570, FCBBF3019663, FCBBF3019714, FCBBF3019716, FCBBF3019784, FCBBF3019867, FCBBF3019961, FCBBF3020030, FCBBF3020138, FCBBF3020163, FCBBF3020232, FCBBF3020599, FCBBF3021026, FCBBF3021191, FCBBF3021221, FCBBF3021501, FCBBF3021506,
FCBBF3021524, FCBBF3021807, FCBBF3021940, FCBBF3021985, FCBBF3022005, FCBBF3022291, FCBBF3022311, FCBBF3022566, FCBBF3022765, FCBBF3022894, FCBBF3023061, FCBBF3023078, FCBBF3023372, FCBBF3023443, FCBBF3023599, FCBBF3023704, FCBBF3023887, FCBBF3023895, FCBBF3024096, FCBBF3024225, FCBBF3024266, FCBBF3024364, FCBBF3024623, FCBBF3024911, FCBBF3025098, FCBBF3025252, FCBBF3025280, FCBBF3025285, FCBBF3025417, FCBBF3025528, FCBBF3025568, FCBBF3025650, FCBBF3025674, FCBBF3025730, FCBBF3026021, FCBBF3026048, FCBBF3026251, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102,
FCBBF3028143, FCBBF3028188, FCBBF3028472, FCBBF3028528, FCBBF3028582, FCBBF3028671, FCBBF3028794, FCBBF4000061, FCBBF4000076, FCBBF4000142, FCBBF4000173, FCBBF4000192, FCBBF4000193, FCBBF4000268, FCBBF4000282, FCBBF4000399, FCBBF4000415, FCBBF4000500, FCBBF4000529, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000022, FEBRA1000030, FEBRA1000088, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000282, FEBRA2000286, FEBRA2000297, FEBRA2000311, FEBRA2000321,
FEBRA2000333, FEBRA2000378, FEBRA2000391, FEBRA2000394, FEBRA2000397, FEBRA2000399, FEBRA2000404, FEBRA2000409, FEBRA2000415, FEBRA2000436, FEBRA2000462, FEBRA2000468, FEBRA2000491, FEBRA2000504, FEBRA2000538, FEBRA2000575, FEBRA2000581, FEBRA2000656, FEBRA2000659, FEBRA2000680, FEBRA2000690, FEBRA2000727, FEBRA2000734, FEBRA2000762, FEBRA2000771, FEBRA2000772, FEBRA2000782, FEBRA2000790, FEBRA2000793, FEBRA2000803, FEBRA2000805, FEBRA2000809, FEBRA2000856, FEBRA2000862, FEBRA2000877, FEBRA2000880, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133,
FEBRA2001161, FEBRA2001175, FEBRA2001197, FEBRA2001217, FEBRA2001245, FEBRA2001267, FEBRA2001334, FEBRA2001351, FEBRA2001383, FEBRA2001438, FEBRA2001440, FEBRA2001487, FEBRA2001561, FEBRA2001584, FEBRA2001590, FEBRA2001591, FEBRA2001610, FEBRA2001620, FEBRA2001659, FEBRA2001705, FEBRA2001706, FEBRA2001752, FEBRA2001772, FEBRA2001790, FEBRA2001914, FEBRA2002009, FEBRA2002027, FEBRA2002086, FEBRA2002109, FEBRA2002191, FEBRA2002260, FEBRA2002399, FEBRA2002410, FEBRA2002442, FEBRA2002508, FEBRA2002525, FEBRA2002527, FEBRA2002552, FEBRA2002611, FEBRA2002628, FEBRA2002662, FEBRA2002682, FEBRA2002727, FEBRA2002781, FEBRA2002783, FEBRA2002818, FEBRA2002858, FEBRA2002882, FEBRA2002897, FEBRA2002908,
FEBRA2002931, FEBRA2002962, FEBRA2002986, FEBRA2003054, FEBRA2003100, FEBRA2003128, FEBRA2003155, FEBRA2003163, FEBRA2003198, FEBRA2003253, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003822, FEBRA2003897, FEBRA2003907, FEBRA2004023, FEBRA2004026, FEBRA2004029, FEBRA2004053, FEBRA2004056, FEBRA2004091, FEBRA2004110, FEBRA2004191, FEBRA2004202, FEBRA2004219, FEBRA2004224, FEBRA2004237, FEBRA2004268, FEBRA2004325, FEBRA2004329, FEBRA2004412, FEBRA2004443, FEBRA2004490, FEBRA2004538, FEBRA2004592, FEBRA2004620, FEBRA2004651, FEBRA2004767, FEBRA2004813,
FEBRA2004840, FEBRA2004852, FEBRA2004867, FEBRA2004914, FEBRA2004933, FEBRA2004987, FEBRA2005014, FEBRA2005036, FEBRA2005079, FEBRA2005216, FEBRA2005377, FEBRA2005380, FEBRA2005427, FEBRA2005458, FEBRA2005701, FEBRA2005726, FEBRA2005752, FEBRA2005788, FEBRA2005998, FEBRA2006055, FEBRA2006061, FEBRA2006112, FEBRA2006150, FEBRA2006165, FEBRA2006315, FEBRA2006346, FEBRA2006354, FEBRA2006370, FEBRA2006379, FEBRA2006396, FEBRA2006409, FEBRA2006418, FEBRA2006427, FEBRA2006476, FEBRA2006519, FEBRA2006521, FEBRA2006627, FEBRA2006664, FEBRA2006667, FEBRA2006726, FEBRA2006736, FEBRA2006793, FEBRA2006817, FEBRA2006890, FEBRA2006942, FEBRA2006977, FEBRA2007017, FEBRA2007176, FEBRA2007200, FEBRA2007247,
FEBRA2007280, FEBRA2007414, FEBRA2007436, FEBRA2007449, FEBRA2007458, FEBRA2007534, FEBRA2007551, FEBRA2007566, FEBRA2007589, FEBRA2007620, FEBRA2007708, FEBRA2007714, FEBRA2007793, FEBRA2007818, FEBRA2007823, FEBRA2007931, FEBRA2007957, FEBRA2008009, FEBRA2008086, FEBRA2008134, FEBRA2008135, FEBRA2008159, FEBRA2008165, FEBRA2008172, FEBRA2008210, FEBRA2008255, FEBRA2008265, FEBRA2008282, FEBRA2008311, FEBRA2008360, FEBRA2008459, FEBRA2008468, FEBRA2008475, FEBRA2008481, FEBRA2008542, FEBRA2008660, FEBRA2008681, FEBRA2008741, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029, FEBRA2009074, FEBRA2009117,
FEBRA2009120, FEBRA2009162, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009541, FEBRA2009567, FEBRA2009588, FEBRA2009731, FEBRA2009754, FEBRA2009846, FEBRA2010141, FEBRA2010299, FEBRA2010484, FEBRA2010719, FEBRA2010746, FEBRA2010768, FEBRA2010802, FEBRA2011063, FEBRA2011356, FEBRA2011392, FEBRA2011414, FEBRA2011593, FEBRA2011665, FEBRA2012090, FEBRA2012195, FEBRA2012397, FEBRA2012418, FEBRA2012507, FEBRA2012625, FEBRA2013019, FEBRA2013069, FEBRA2013274, FEBRA2013465, FEBRA2013570, FEBRA2013905, FEBRA2013951, FEBRA2014122, FEBRA2014198, FEBRA2014443,
FEBRA2014939, FEBRA2015076, FEBRA2015175, FEBRA2015292, FEBRA2015588, FEBRA2016112, FEBRA2016398, FEBRA2016583, FEBRA2016621, FEBRA2016654, FEBRA2016739, FEBRA2017098, FEBRA2017138, FEBRA2017154, FEBRA2017200, FEBRA2017223, FEBRA2017333, FEBRA2017502, FEBRA2017533, FEBRA2017736, FEBRA2017780, FEBRA2017811, FEBRA2017850, FEBRA2018051, FEBRA2018203, FEBRA2018407, FEBRA2018433, FEBRA2019172, FEBRA2019298, FEBRA2019389, FEBRA2019457, FEBRA2019519, FEBRA2019582, FEBRA2019637, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021134, FEBRA2021171, FEBRA2021339, FEBRA2021431, FEBRA2021497, FEBRA2021550,
FEBRA2021571, FEBRA2021636, FEBRA2021864, FEBRA2021892, FEBRA2021908, FEBRA2021966, FEBRA2022013, FEBRA2022055, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023285, FEBRA2023351, FEBRA2023377, FEBRA2023399, FEBRA2023498, FEBRA2023550, FEBRA2023721, FEBRA2023989, FEBRA2023990, FEBRA2024150, FEBRA2024343, FEBRA2024570, FEBRA2024693, FEBRA2024727, FEBRA2024744, FEBRA2024797, FEBRA2024987, FEBRA2025249, FEBRA2025463, FEBRA2025477, FEBRA2026474, FEBRA2026582, FEBRA2026629, FEBRA2026769, FEBRA2026879, FEBRA2026890, FEBRA2026977, FEBRA2026984, FEBRA2027082, FEBRA2027297,
FEBRA2027352, FEBRA2027364, FEBRA2027609, FEBRA2028222, FEBRA2028366, FEBRA2028457, FEBRA2028477, FEBRA2028503, FEHRT2000325, FEHRT2000364, FELNG2000241, HCASM1000021, HCASM1000050, HCASM1000061, HCASM1000130, HCASM2000156, HCASM2000202, HCASM2000214, HCASM2000266, HCASM2000307, HCASM2000534, HCASM2000536, HCASM2000560, HCASM2000871, HCASM2001285, HCASM2001810, HCASM2001866, HCASM2001890, HCASM2002148, HCASM2002502, HCASM2002754, HCASM2002918, HCASM2003076, HCASM2003099, HCASM2003212, HCASM2003237, HCASM2003357, HCASM2003415, HCASM2005484, HCASM2006131, HCASM2006338, HCASM2006359, HCASM2006632, HCASM2007047, HCASM2007317, HCASM2007737, HCASM2007773, HCASM2008154, HCASM2008536, HCASM2009405, HCASM2009424, HCASM2009463, HCASM2009588, HCHON1000015, HCHON1000131, HCHON1000142, HCHON1000176, HCHON1000191, HCHON2000038, HCHON2000100, HCHON2000156, HCHON2000199, HCHON2000212, HCHON2000244, HCHON2000271, HCHON2000315, HCHON2000322, HCHON2000344, HCHON2000373, HCHON2000395, HCHON2000417, HCHON2000418, HCHON2000475, HCHON2000503, HCHON2000660, HCHON2000663, HCHON2000673, HCHON2000676, HCHON2000705, HCHON2000815, HCHON2000817, HCHON2000818, HCHON2000819, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001161, HCHON2001214, HCHON2001233, HCHON2001269, HCHON2001407, HCHON2001450,
HCHON2001453, HCHON2001497, HCHON2001505, HCHON2001519, HCHON2001523, HCHON2001535, HCHON2001546, HCHON2001548, HCHON2001577, HCHON2001598, HCHON2001604, HCHON2001607, HCHON2001646, HCHON2001768, HCHON2001801, HCHON2002123, HCHON2002194, HCHON2002241, HCHON2002496, HCHON2002676, HCHON2002770, HCHON2002964, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005921, HCHON2006714, HCHON2006722, HCHON2006770, HCHON2006786, HCHON2006841, HCHON2006871, HCHON2007046, HCHON2007482, HCHON2007674,
HCHON2007774, HCHON2007881, HCHON2008112, HCHON2008239, HCHON2008444, HCHON2008582, HCHON2008672, HCHON2008871, HCHON2008989, HCHON2009191, HCHON2009384, HCHON2009749, HCHON2009766, HCHON2009795, HCHON2010074, HCHON2010543, HEART1000010, HEART1000088, HEART1000099, HEART1000102, HEART1000118, HEART1000132, HEART1000139, HEART1000142, HEART1000151, HEART1000173, HEART1000185, HEART2000024, HEART2000035, HEART2000099, HEART2000298, HEART2000306, HEART2000309, HEART2000411, HEART2000418, HEART2000444, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531,
HEART2002598, HEART2002717, HEART2003027, HEART2003168, HEART2003432, HEART2003440, HEART2003781, HEART2003836, HEART2003852, HEART2004570, HEART2004764, HEART2004931, HEART2004940, HEART2004941, HEART2004980, HEART2005244, HEART2005310, HEART2005581, HEART2005610, HEART2006131, HEART2006310, HEART2006334, HEART2006487, HEART2006557, HEART2006787, HEART2006789, HEART2006909, HEART2007031, HEART2007443, HEART2007696, HEART2007767, HEART2008108, HEART2008111, HEART2008257, HEART2008509, HEART2008994, HEART2009000, HEART2009599, H-EART2009680, HEART2010391, HEART2010492, HEART2010495, HELAC2000158, HELAC2000301, HHDPC1000001, HHDPC1000114, HHDPC1000118, HHDPC2000102, HHDPC2000115, HHDPC2000258,
HHDPC2000315, HHDPC2000462, HHDPC2000656, HHDPC2000692, HHDPC2001337, HHDPC2001432, HHDPC2001896, HHDPC2002963, HHDPC2003049, HHDPC2003113, HHDPC2003381, HHDPC2003472, HHDPC2004757, HHDPC2005185, HHDPC2005513, HHDPC2005794, HHDPC2006460, HHDPC2006862, HHDPC2006927, HHDPC2007302, HHDPC2007775, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009528, HLUNG1000017, HLUNG1000024, HLUNG1000030, HLUNG1000034, HLUNG1000053, HLUNG1000064, HLUNG1000084, HLUNG1000091, HLUNG1000104, HLUNG1000105, HLUNG1000110, HLUNG1000151, HLUNG1000169, HLUNG2000014, HLUNG2000027, HLUNG2000068, HLUNG2000116, HLUNG2000130,
HLUNG2000142, HLUNG2000174, HLUNG2000217, HLUNG2000232, HLUNG2000255, HLUNG2000281, HLUNG2000314, HLUNG2000315, HLUNG2000340, HLUNG2000362, HLUNG2000363, HLUNG2000431, HLUNG2000480, HLUNG2000489, HLUNG2000501, HLUNG2000549, HLUNG2000557, HLUNG2000570, HLUNG2000761, HLUNG2000777, HLUNG2000870, HLUNG2000926, HLUNG2000955, HLUNG2000970, HLUNG2001013, HLUNG2001126, HLUNG2001144, HLUNG2001214, HLUNG2001459, HLUNG2001507, HLUNG2001814, HLUNG2001996, HLUNG2002006, HLUNG2002050, HLUNG2002085, HLUNG2002145, HLUNG2002295, HLUNG2002303, HLUNG2002334, HLUNG2002405, HLUNG2002429, HLUNG2002562, HLUNG2002659, HLUNG2002707, HLUNG2002811, HLUNG2002916, HLUNG2002942, HLUNG2002949, HLUNG2002958, HLUNG2002982,
HLUNG2003049, HLUNG2003061, HLUNG2003162, HLUNG2003246, HLUNG2003331, HLUNG2003335, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004170, HLUNG2004217, HLUNG2004273, HLUNG2004388, HLUNG2004416, HLUNG2004521, HLUNG2004684, HLUNG2004707, HLUNG2004774, HLUNG2004775, HLUNG2005012, HLUNG2005030, HLUNG2005045, HLUNG2005076, HLUNG2005133, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094, HLUNG2006397, HLUNG2006599, HLUNG2006614, HLUNG2006671, HLUNG2006781, HLUNG2006843,
HLUNG2006852, HLUNG2006935, HLUNG2007041, HLUNG2007163, HLUNG2007210, HLUNG2007245, HLUNG2007433, HLUNG2007931, HLUNG2008066, HLUNG2008139, HLUNG2008225, HLUNG2008235, HLUNG2008247, HLUNG2008333, HLUNG2008348, HLUNG2008384, HLUNG2008396, HLUNG2008439, HLUNG2008479, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2012049, HLUNG2012287, HLUNG2012315, HLUNG2012546, HLUNG2012600, HLUNG2012727, HLUNG2013097, HLUNG2013204, HLUNG2013304, HLUNG2013350, HLUNG2013622,
HLUNG2013643, HLUNG2013784, HLUNG2013851, HLUNG2014262, HLUNG2014288, HLUNG2014449, HLUNG2015548, HLUNG2015578, HLUNG2015617, HLUNG2016022, HLUNG2016862, HLUNG2017286, HLUNG2017307, HLUNG2017350, HLUNG2017806, HLUNG2018029, HLUNG2018282, HLUNG2018626, HLUNG2019058, HSYRA1000131, HSYRA1000137, HSYRA1000148, HSYRA2000135, HSYRA2000137, HSYRA2000190, HSYRA2000221, HSYRA2000237, HSYRA2000332, HSYRA2000371, HSYRA2000507, HSYRA2000510, HSYRA2000605, HSYRA2000706, HSYRA2000760, HSYRA2000792, HSYRA2000832, HSYRA2000927, HSYRA2001003, HSYRA2001142, HSYRA2001153, HSYRA2001232, HSYRA2001255, HSYRA2001332, HSYRA2001484, HSYRA2001552, HSYRA2001574, HSYRA2001580, HSYRA2001615, HSYRA2001631, HSYRA2002103,
HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007650, HSYRA2007667, HSYRA2008154, HSYRA2008714, HSYRA2009102, IMR321000155, IMR321000158, IMR321000165, IMR321000193, IMR321000207, IMR321000230, IMR321000242, IMR322000010, IMR322000072, IMR322000107, IMR322000144, IMR322000152, IMR322000164, IMR322000302, IMR322000379, IMR322000383, IMR322000418, IMR322000518, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917,
IMR322000919, IMR322000953, IMR322000984, IMR322001018, IMR322001049, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001534, IMR322001665, IMR322001670, IMR322001724, IMR322001879, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731,
IMR322015523, IMR322016146, IMR322017049, IMR322018117, IMR322018192, KIDNE1000008, KIDNE1000012, KIDNE1000015, KIDNE1000028, KIDNE1000036, KIDNE1000064, KIDNE1000121, KIDNE1000143, KIDNE1000152, KIDNE2000026, KIDNE2000041, KIDNE2000046, KIDNE2000051, KIDNE2000070, KIDNE2000167, KIDNE2000174, KIDNE2000192, KIDNE2000227, KIDNE2000244, KIDNE2000245, KIDNE2000287, KIDNE2000315, KIDNE2000317, KIDNE2000328, KIDNE2000330, KIDNE2000335, KIDNE2000375, KIDNE2000394, KIDNE2000422, KIDNE2000493, KIDNE2000497, KIDNE2000510, KIDNE2000513, KIDNE2000519, KIDNE2000555, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833,
KIDNE2000846, KIDNE2000867, KIDNE2000909, KIDNE2001117, KIDNE2001140, KIDNE2001269, KIDNE2001373, KIDNE2001802, KIDNE2001840, KIDNE2001857, KIDNE2001873, KIDNE2001897, KIDNE2001931, KIDNE2001979, KIDNE2002015, KIDNE2002162, KIDNE2002168, KIDNE2002191, KIDNE2002198, KIDNE2002262, KIDNE2002265, KIDNE2002438, KIDNE2002483, KIDNE2002768, KIDNE2002795, KIDNE2002798, KIDNE2002839, KIDNE2002845, KIDNE2002872, KIDNE2002882, KIDNE2002980, KIDNE2002991, KIDNE2003185, KIDNE2003188, KIDNE2003233, KIDNE2003335, KIDNE2003377, KIDNE2003507, KIDNE2003752, KIDNE2003764, KIDNE2003837, KIDNE2004034, KIDNE2004054, KIDNE2004084, KIDNE2004095, KIDNE2004115, KIDNE2004233, KIDNE2004262, KIDNE2004279, KIDNE2004294,
KIDNE2004305, KIDNE2004394, KIDNE2004519, KIDNE2004520, KIDNE2004562, KIDNE2004579, KIDNE2004626, KIDNE2004681, KIDNE2004828, KIDNE2004846, KIDNE2004879, KIDNE2004981, KIDNE2004985, KIDNE2004999, KIDNE2005038, KIDNE2005042, KIDNE2005062, KIDNE2005266, KIDNE2005296, KIDNE2005321, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006030, KIDNE2006039, KIDNE2006053, KIDNE2006062, KIDNE2006092, KIDNE2006145, KIDNE2006149, KIDNE2006210, KIDNE2006299, KIDNE2006334, KIDNE2006376, KIDNE2006378, KIDNE2006580, KIDNE2006687, KIDNE2006733, KIDNE2006760,
KIDNE2006820, KIDNE2007005, KIDNE2007040, KIDNE2007077, KIDNE2007186, KIDNE2007222, KIDNE2007356, KIDNE2007422, KIDNE2007569, KIDNE2007810, KIDNE2007811, KIDNE2007910, KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2009019, KIDNE2009109, KIDNE2009180, KIDNE2009191, KIDNE2009332, KIDNE2009426, KIDNE2009467, KIDNE2009490, KIDNE2009553, KIDNE2009628, KIDNE2009647, KIDNE2009676, KIDNE2009723,
KIDNE2010007, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011752, KIDNE2011782, KIDNE2011858, KIDNE2012009, KIDNE2012025, KIDNE2012188, KIDNE2012291, KIDNE2012316, KIDNE2012361, KIDNE2012453, KIDNE2012563, KIDNE2012601, KIDNE2012613, KIDNE2012745, KIDNE2012775, KIDNE2012784, KIDNE2012945, KIDNE2013045, KIDNE2013095, KIDNE2013218, KIDNE2013263, KIDNE2013346, KIDNE2013388, KIDNE2013413, KIDNE2013489, KIDNE2013639, KIDNE2013731, KIDNE2013734,
KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014890, KIDNE2014978, KIDNE2015073, KIDNE2015221, KIDNE2015244, KIDNE2015313, KIDNE2015433, KIDNE2015483, KIDNE2015515, KIDNE2015556, KIDNE2015660, KIDNE2015710, KIDNE2015987, KIDNE2016000, KIDNE2016036, KIDNE2016096, KIDNE2016101, KIDNE2016142, KIDNE2016188, KIDNE2016388, KIDNE2016464, KIDNE2016539, KIDNE2016570, KIDNE2016918, KIDNE2017007, KIDNE2017151, KIDNE2017153, KIDNE2017315, KIDNE2017332, KIDNE2017343, KIDNE2017454, KIDNE2017603, KIDNE2017956,
KIDNE2018071, KIDNE2018166, KIDNE2018167, KIDNE2018217, KIDNE2018254, KIDNE2018268, KIDNE2018269, KIDNE2018287, KIDNE2018294, KIDNE2018462, KIDNE2018617, KIDNE2018678, KIDNE2018727, KIDNE2018863, KIDNE2018989, KIDNE2018996, KIDNE2019074, KIDNE2019187, LIVER1000050, LIVER1000055, LIVER1000058, LIVER1000063, LIVER1000073, LIVER1000082, LIVER1000099, LIVER1000126, LIVER1000139, LIVER1000155, LIVER1000165, LIVER1000175, LIVER1000224, LIVER1000278, LIVER1000397, LIVER1000421, LIVER1000433, LIVER1000479, LIVER1000482, LIVER1000542, LIVER2000003, LIVER2000033, LIVER2000158, LIVER2000216, LIVER2000237, LIVER2000247, LIVER2000360, LIVER2000416, LIVER2000446, LIVER2000515, LIVER2000592, LIVER2000626,
LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003234, LIVER2003511, LIVER2003524, LIVER2003568, LIVER2003581, LIVER2003800, LIVER2003854, LIVER2004074, LIVER2004392, LIVER2004565, LIVER2005028, LIVER2005198, LIVER2005218, LIVER2005376, LIVER2005385, LIVER2005512, LIVER2005520, LIVER2005527, LIVER2005544, LIVER2005625, LIVER2005728, LIVER2005973, LIVER2005981, LIVER2006251, LIVER2006410, LIVER2006469, LIVER2006644, LIVER2006764, LIVER2007415, LIVER2007548, LIVER2007568,
LIVER2007721, LIVER2008053, LIVER2008465, LIVER2008473, LIVER2008580, LIVER2008751, LIVER2008945, LIVER2008998, LIVER2009110, LIVER2009118, LIVER2009554, LYMPB1000141, LYMPB1000158, LYMPB2000083, MAMGL1000096, MAMGL1000100, MAMGL1000132, MAMGL1000151, MAMGL1000182, MAMGL1000184, MESAN1000032, MESAN1000035, MESAN1000080, MESAN1000121, MESAN1000147, MESAN1000180, MESAN2000023, MESAN2000026, MESAN2000067, MESAN2000167, MESAN2000264, MESAN2000291, MESAN2000337, MESAN2000434, MESAN2000457, MESAN2000462, MESAN2000501, MESAN2000620, MESAN2000711, MESAN2000894, MESAN2000909, MESAN2001154, MESAN2001770, MESAN2001979, MESAN2001980, MESAN2002113, MESAN2002122, MESAN2002147, MESAN2002186, MESAN2002424,
MESAN2002519, MESAN2002687, MESAN2002709, MESAN2002844, MESAN2002978, MESAN2003037, MESAN2003039, MESAN2003101, MESAN2003322, MESAN2003490, MESAN2003529, MESAN2003646, MESAN2003662, MESAN2003709, MESAN2003851, MESAN2004138, MESAN2004288, MESAN2004575, MESAN2005371, MESAN2005724, MESAN2005808, MESAN2005811, MESAN2006022, MESAN2006043, MESAN2006328, MESAN2006580, MESAN2006599, MESAN2006743, MESAN2006953, MESAN2006971, MESAN2007032, MESAN2007440, MESAN2007597, MESAN2008222, MESAN2008334, MESAN2008415, MESAN2008460, MESAN2008536, MESAN2008679, MESAN2008729, MESAN2008821, MESAN2009019, MESAN2009081, MESAN2009105, MESAN2009311, MESAN2009418, MESAN2009503, MESAN2009522, MESAN2010031, MESAN2010114,
MESAN2010312, MESAN2010321, MESAN2010681, MESAN2011545, MESAN2011597, MESAN2011627, MESAN2011632, MESAN2011977, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013936, MESAN2014295, MESAN2014298, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016965, MESAN2017152, MESAN2017373, MESAN2017417, MESAN2018209, MESAN2018576, MESAN2018670, MESAN2018699, MESTC1000042, MESTC1000147, MESTC2000150, MESTC2000153, MESTC2000170, NB9N41000011, NB9N41000047, NB9N41000135,
NB9N41000142, NB9N41000146, NB9N42000122, NB9N42000196, NB9N42000281, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000744, NESOP2001144, NESOP2001433, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000187, NHNPC2000212, NHNPC2000606, NHNPC2000877, NHNPC2001108, NHNPC2001223, NHNPC2001312, NHNPC2001816, NHNPC2001931, NHNPC2002565, NHNPC2002749, NOVAR1000102, NOVAR2000136,
NOVAR2000352, NOVAR2000412, NOVAR2000783, NOVAR2000962, NOVAR2001108, NOVAR2001620, NOVAR2001783, NOVAR2001876, NT2NE1000004, NT2NE1000018, NT2NE1000023, NT2NE1000024, NT2NE1000059, NT2NE1000063, NT2NE1000073, NT2NE1000083, NT2NE1000120, NT2NE1000175, NT2NE1000185, NT2NE2000038, NT2NE2000054, NT2NE2000057, NT2NE2000064, NT2NE2000109, NT2NE2000137, NT2NE2000156, NT2NE2000222, NT2NE2000259, NT2NE2000284, NT2NE2000299, NT2NE2000327, NT2NE2000353, NT2NE2000369, NT2NE2000374, NT2NE2000386, NT2NE2000390, NT2NE2000455, NT2NE2000488, NT2NE2000517, NT2NE2000546, NT2NE2000548, NT2NE2000550, NT2NE2000586, NT2NE2000612, NT2NE2000636, NT2NE2000658, NT2NE2000706, NT2NE2000802, NT2NE2000808, NT2NE2000864,
NT2NE2000913, NT2NE2000951, NT2NE2000980, NT2NE2001005, NT2NE2001021, NT2NE2001040, NT2NE2001049, NT2NE2001142, NT2NE2001156, NT2NE2001176, NT2NE2001180, NT2NE2001181, NT2NE2001188, NT2NE2001191, NT2NE2001247, NT2NE2001252, NT2NE2001324, NT2NE2001337, NT2NE2001364, NT2NE2001372, NT2NE2001397, NT2NE2001428, NT2NE2001432, NT2NE2001442, NT2NE2001524, NT2NE2001530, NT2NE2001598, NT2NE2001623, NT2NE2001626, NT2NE2001634, NT2NE2001666, NT2NE2001677, NT2NE2001697, NT2NE2001698, NT2NE2001723, NT2NE2001725, NT2NE2001874, NT2NE2001889, NT2NE2001923, NT2NE2002186, NT2NE2002285, NT2NE2002311, NT2NE2002620, NT2NE2002651, NT2NE2002732, NT2NE2002768, NT2NE2002791, NT2NE2002856, NT2NE2002870, NT2NE2003150,
NT2NE2003185, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003921, NT2NE2004378, NT2NE2004490, NT2NE2004519, NT2NE2004606, NT2NE2004716, NT2NE2004787, NT2NE2004916, NT2NE2005035, NT2NE2005223, NT2NE2005276, NT2NE2005358, NT2NE2005395, NT2NE2005419, NT2NE2005441, NT2NE2005517, NT2NE2005537, NT2NE2005555, NT2NE2005588, NT2NE2005676, NT2NE2005720, NT2NE2005784, NT2NE2005804, NT2NE2005821, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006531,
NT2NE2006659, NT2NE2006784, NT2NE2006813, NT2NE2006894, NT2NE2006942, NT2NE2006958, NT2NE2007052, NT2NE2007220, NT2NE2007425, NT2NE2007725, NT2NE2007727, NT2NE2007877, NT2NE2007967, NT2NE2008017, NT2NE2008060, NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009295, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010854, NT2NE2011033, NT2NE2011036, NT2NE2011154, NT2NE2011221, NT2NE2011411, NT2NE2011485,
NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448, NT2NE2012457, NT2NE2012493, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015275, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387,
NT2NE2016419, NT2NE2016519, NT2NE.2016608, NT2NE2016766, NT2NE2016774, NT2NE2016971, NT2NE2017259, NT2NE2017332, NT2NE2017397, NT2NE2017480, NT2NE2017492, NT2NE2017508, NT2NE2017562, NT2NE2017590, NT2NE2017612, NT2NE2017721, NT2NE2017752, NT2NE2017792, NT2NE2017982, NT2NE2018165, NT2NE2018176, NT2NE2018273, NT2NE2018376, NT2NE2018472, NT2NE2018490, NT2NE2018594, NT2NE2018739, NT2NE2018916, NT2NE2019056, NT2NE2019076, NT2NE2019099, NT2RI1000035, NT2RI1000164, NT2RI1000172, NT2RI2000004, NT2RI2000007, NT2RI2000117, NT2RI2000128, NT2RI2000133, NT2RI2000173, NT2RI2000187, NT2RI2000270, NT2RI2000276, NT2RI2000313, NT2RI2000341, NT2RI2000344, NT2RI2000421, NT2RI2000480, NT2RI2000575, NT2RI2000588,
NT2RI2000597, NT2RI2000669, NT2RI2000688, NT2RI2000689, NT2RI2000704, NT2RI2000738, NT2RI2000841, NT2RI2000865, NT2RI2000932, NT2RI2000987, NT2RI2001017, NT2RI2001030, NT2RI2001054, NT2RI2001083, NT2RI2001091, NT2RI2001167, NT2RI2001178, NT2RI2001230, NT2RI2001235, NT2RI2001244, NT2RI2001307, NT2RI2001385, NT2RI2001409, NT2RI2001410, NT2RI2001425, NT2RI2001449, NT2RI2001450, NT2RI2001474, NT2RI2001519, NT2RI2001595, NT2RI2001621, NT2RI2001624, NT2RI2001769, NT2RI2001782, NT2RI2001836, NT2RI2001952, NT2RI2002022, NT2RI2002041, NT2RI2002117, NT2RI2002120, NT2RI2002153, NT2RI2002179, NT2RI2002243, NT2RI2002252, NT2RI2002260, NT2RI2002266, NT2RI2002270, NT2RI2002334, NT2RI2002359, NT2RI2002447,
NT2RI2002481, NT2RI2002507, NT2RI2002517, NT2RI2002530, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002822, NT2RI2002852, NT2RI2002923, NT2RI2002926, NT2RI2002970, NT2RI2003067, NT2RI2003184, NT2RI2003205, NT2RI2003222, NT2RI2003301, NT2RI2003360, NT2RI2003361, NT2RI2003383, NT2RI2003392, NT2RI2003399, NT2RI2003419, NT2RI2003481, NT2RI2003487, NT2RI2003556, NT2RI2003655, NT2RI2003667, NT2RI2003751, NT2RI2003884, NT2RI2003973, NT2RI2003993, NT2RI2004078, NT2RI2004079, NT2RI2004093, NT2RI2004136, NT2RI2004157, NT2RI2004188, NT2RI2004190, NT2RI2004209, NT2RI2004230, NT2RI2004284, NT2RI2004290,
NT2RI2004312, NT2RI2004381, NT2RI2004398, NT2RI2004410, NT2RI2004442, NT2RI2004468, NT2RI2004606, NT2RI2004618, NT2RI2004783, NT2RI2004818, NT2RI2004840, NT2RI2004916, NT2RI2004962, NT2RI2004984, NT2RI2004985, NT2RI2005048, NT2RI2005069, NT2RI2005087, NT2RI2005096, NT2RI2005115, NT2RI2005130, NT2RI2005166, NT2RI2005239, NT2RI2005246, NT2RI2005315, NT2RI2005358, NT2RI2005368, NT2RI2005382, NT2RI2005520, NT2RI2005564, NT2RI2005579, NT2RI2005621, NT2RI2005628, NT2RI2005670, NT2RI2005772, NT2RI2005816, NT2RI2005851, NT2RI2005966, NT2RI2006071, NT2RI2006081, NT2RI2006126, NT2RI2006127, NT2RI2006183, NT2RI2006257, NT2RI2006294, NT2RI2006345, NT2RI2006403, NT2RI2006445, NT2RI2006487, NT2RI2006506,
NT2RI2006553, NT2RI2006647, NT2RI2006659, NT2RI2006662, NT2RI2006667, NT2RI2006679, NT2RI2006686, NT2RI2006716, NT2RI2006735, NT2RI2006762, NT2RI2006788, NT2RI2006825, NT2RI2006838, NT2RI2006855, NT2RI2006973, NT2RI2007046, NT2RI2007048, NT2RI2007116, NT2RI2007148, NT2RI2007214, NT2RI2007303, NT2RI2007334, NT2RI2007386, NT2RI2007406, NT2RI2007439, NT2RI2007469, NT2RI2007498, NT2RI2007507, NT2RI2007593, NT2RI2007729, NT2RI2007751, NT2RI2007754, NT2RI2007827, NT2RI2007853, NT2RI2007877, NT2RI2007879, NT2RI2007891, NT2RI2007956, NT2RI2007965, NT2RI2008007, NT2RI2008050, NT2RI2008171, NT2RI2008188, NT2RI2008204, NT2RI2008221, NT2RI2008233, NT2RI2008455, NT2RI2008481, NT2RI2008502, NT2RI2008598,
NT2RI2008622, NT2RI2008656, NT2RI2008749, NT2RI2008801, NT2RI2008812, NT2RI2008841, NT2RI2008952, NT2RI2009005, NT2RI2009101, NT2RI2009103, NT2RI2009144, NT2RI2009151, NT2RI2009215, NT2RI2009233, NT2RI2009281, NT2RI2009289, NT2RI2009301, NT2RI2009402, NT2RI2009517, NT2RI2009585, NT2RI2009595, NT2RI2009855, NT2RI2010508, NT2RI2010795, NT2RI2011422, NT2RL2011683, NT2RI2011803, NT2RI2012350, NT2RI2012659, NT2RI2012990, NT2RI2013345, NT2RI2013357, NT2RI2013373, NT2RI2014247, NT2RI2014551, NT2RI2014683, NT2RI2014733, NT2RI2015239, NT2RI2016128, NT2RI2016134, NT2RI2017529, NT2RI2018311, NT2RI2018363, NT2RI2018883, NT2RI2018950, NT2RI2019751, NT2RI2019826, NT2RI2020390, NT2RI2020703, NT2RI2021463,
NT2RI2021625, NT2RI2022468, NT2RI2022584, NT2RI2023303, NT2RI2023671, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027323, NT2RI2027396, NT2RI2027484, NT2RI2027975, NT2RI2028213, NT2RI2028222, NT2RI2028537, NT2RI2028642, NT2RI3000213, NT2RI3000622, NT2RI3001132, NT2RI3001263, NT2RI3001279, NT2RI3001348, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003925, NT2RI3004133, NT2RI3004381,
NT2RI3004510, NT2RI3005202, NT2RI3005403, NT2RI3005416, NT2RI3005724, NT2RI3005861, NT2RI3005928, NT2RI3006132, NT2RI3006171, NT2RI3006340, NT2RI3006376, NT2RI3006666, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009524, NT2RP6000005, NT2RP6000008, NT2RP6000032, NT2RP6000033, NT2RP6000043, NT2RP6000059, NT2RP6000060, NT2RP6000077, NT2RP6000086, NT2RP6000100, NT2RP6000109, NT2RP6000119, NT2RP6000127, NT2RP6000130, NT2RP6000140, NT2RP6000150, NT2RP7000041,
NT2RP7000109, NT2RP7000311, NT2RP7000359, NT2RP7000364, NT2RP7000380, NT2RP7000391, NT2RP7000425, NT2RP7000579, NT2RP7000586, NT2RP7000624, NT2RP7000812, NT2RP7000926, NT2RP7001074, NT2RP7001080, NT2RP7001156, NT2RP7001306, NT2RP7001319, NT2RP7001364, NT2RP7001475, NT2RP7001532, NT2RP7001602, NT2RP7001678, NT2RP7001856, NT2RP7002064, NT2RP7002151, NT2RP7002163, NT2RP7002243, NT2RP7002478, NT2RP7002554, NT2RP7002594, NT2RP7002603, NT2RP7002650, NT2RP7002738, NT2RP7002779, NT2RP7002841, NT2RP7002906, NT2RP7002978, NT2RP7002982, NT2RP7003025, NT2RP7003050, NT2RP7003055, NT2RP7003084, NT2RP7003091, NT2RP7003107, NT2RP7003134, NT2RP7003148, NT2RP7003261, NT2RP7003439, NT2RP7003680, NT2RP7003724,
NT2RP7004027, NT2RP7004080, NT2RP7004233, NT2RP7004358, NT2RP7004396, NT2RP7004481, NT2RP7004541, NT2RP7004559, NT2RP7004722, NT2RP7004766, NT2RP7004790, NT2RP7004915, NT2RP7004964, NT2RP7004975, NT2RP7005118, NT2RP7005205, NT2RP7005219, NT2RP7005323, NT2RP7005468, NT2RP7005513, NT2RP7005629, NT2RP7005631, NT2RP7005669, NT2RP7005846, NT2RP7006033, NT2RP7006075, NT2RP7006160, NT2RP7006162, NT2RP7006263, NT2RP7006296, NT2RP7006304, NT2RP7006374, NT2RP7006395, NT2RP7006408, NT2RP7006457, NT2RP7006547, NT2RP7006621, NT2RP7006636, NT2RP7006853, NT2RP7006887, NT2RP7006980, NT2RP7007114, NT2RP7007221, NT2RP7007226, NT2RP7007269, NT2RP7007310, NT2RP7007359, NT2RP7007480, NT2RP7007504, NT2RP7007594,
NT2RP7007617, NT2RP7007643, NT2RP7007842, NT2RP7007925, NT2RP7007930, NT2RP7008013, NT2RP7008133, NT2RP7008137, NT2RP7008142, NT2RP7008144, NT2RP7008190, NT2RP7008360, NT2RP7008396, NT2RP7008406, NT2RP7008435, NT2RP7008441, NT2RP7008454, NT2RP7008487, NT2RP7008544, NT2RP7008550, NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009168, NT2RP7009215, NT2RP7009225, NT2RP7009236, NT2RP7009253, NT2RP7009267, NT2RP7009322, NT2RP7009363, NT2RP7009370, NT2RP7009373, NT2RP7009397, NT2RP7009429, NT2RP7009439, NT2RP7009502, NT2RP7009507, NT2RP7009577, NT2RP7009743, NT2RP7009867, NT2RP7010128, NT2RP7010235,
NT2RP7010275, NT2RP7010521, NT2RP7010599, NT2RP7010612, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291, NT2RP7012516, NT2RP7012776, NT2RP7013002, NT2RP7013374, NT2RP7013499, NT2RP7013729, NT2RP7013999, NT2RP7014178, NT2RP7014348, NT2RP7014420, NT2RP7014721, NT2RP7014743, NT2RP7014778, NT2RP7014906, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT-2RP7016911, NT2RP7017139, NT2RP7017284, NT2RP7017365, NT2RP7017474, NT2RP7017546, NT2RP7017567, NT2RP7017795, NT2RP7017971, NT2RP7018032, NT2RP7018126, NT2RP7018197,
NT2RP7018206, NT2RP7018340, NT2RP7018586, NT2RP7018802, NT2RP7018871, NT2RP7019064, NT2RP7019135, NT2RP7019273, NT2RP7019367, NT2RP7019401, NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8006452, NT2RP8006521, NT2RP8007416, NT2RP8007503, NT2RP8007715, NT2RP8009248, NTONG1000006, NTONG1000047, NTONG1000052, NTONG1000087, NTONG1000123, NTONG1000161, NTONG1000172, NTONG1000213, NTONG1000247, NTONG1000257, NTONG2000040, NTONG2000107,
NTONG2000231, NTONG2000334, NTONG2000363, NTONG2000413, NTONG2000492, NTONG2000499, NTONG2000531, NTONG2000583, NTONG2000858, NTONG2000878, NTONG2000966, NTONG2001014, NTONG2001079, NTONG2001157, NTONG2001173, NTONG2001220, NTONG2001222, NTONG2001362, NTONG2001428, NTONG2001532, NTONG2001587, NTONG2001619, NTONG2001642, NTONG2001657, NTONG2001676, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003839, NTONG2003852, NTONG2003897, NTONG2004521, NTONG2004690, NTONG2004806, NTONG2004829, NTONG2004844, NTONG2004991, NTONG2005062, NTONG2005086, NTONG2005137, NTONG2005153, NTONG2005265,
NTONG2005277, NTONG2005363, NTONG2005480, NTONG2005497, NTONG2005577, NTONG2005657, NTONG2005822, NTONG2005897, NTONG2006099, NTONG2006187, NTONG2006301, NTONG2006324, NTONG2006354, NTONG2006440, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008365, NTONG2008672, NTONG2008862, NTONG2008939, NTONG2009068, NTONG2009229, NTONG2009233, NTONG2009468, OCBBF1000016, OCBBF1000030, OCBBF1000042, OCBBF1000049, OCBBF1000054, OCBBF1000067, OCBBF1000085, OCBBF1000091, OCBBF1000114, OCBBF1000118, OCBBF1000119, OCBBF1000122, OCBBF1000130,
OCBBF1000138, OCBBF1000145, OCBBF1000153, OCBBF1000175, OCBBF1000185, OCBBF1000188, OCBBF1000192, OCBBF1000254, OCBBF2000277, OCBBF2000287, OCBBF2000323, OCBBF2000452, OCBBF2000467, OCBBF2000703, OCBBF2000712, OCBBF2000719, OCBBF2000824, OCBBF2000904, OCBBF2000998, OCBBF2001075, OCBBF2001101, OCBBF2001124, OCBBF2001140, OCBBF2001176, OCBBF2001186, OCBBF2001210, OCBBF2001252, OCBBF2001323, OCBBF2001389, OCBBF2001402, OCBBF2001408, OCBBF2001416, OCBBF2001473, OCBBF2001494, OCBBF2001527, OCBBF2001586, OCBBF2001681, OCBBF2001749, OCBBF2001794, OCBBF2001910, OCBBF2001931, OCBBF2001961, OCBBF2002083, OCBBF2002124, OCBBF2002161, OCBBF2002357, OCBBF2002376, OCBBF2002615, OCBBF2002626, OCBBF2002663,
OCBBF2002805, OCBBF2002865, OCBBF2002920, OCBBF2002980, OCBBF2003028, OCBBF2003091, OCBBF2003246, OCBBF2003327, OCBBF2003593, OCBBF2004038, OCBBF2004147, OCBBF2004168, OCBBF2004235, OCBBF2004259, OCBBF2004385, OCBBF2004418, OCBBF2004478, OCBBF2004482, OCBBF2004533, OCBBF2004567, OCBBF2004612, OCBBF2004647, OCBBF2004669, OCBBF2004757, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005476, OCBBF2005546, OCBBF2005843, OCBBF2005901, OCBBF2005956, OCBBF2006005, OCBBF2006030, OCBBF2006058, OCBBF2006113, OCBBF2006148, OCBBF2006151,
OCBBF2006154, OCBBF2006172, OCBBF2006214, OCBBF2006241, OCBBF2006313, OCBBF2006388, OCBBF2006438, OCBBF2006567, OCBBF2006624, OCBBF2006639, OCBBF2006764, OCBBF2006859, OCBBF2006972, OCBBF2006987, OCBBF2007039, OCBBF2007051, OCBBF2007068, OCBBF2007114, OCBBF2007184, OCBBF2007194, OCBBF2007196, OCBBF2007238, OCBBF2007428, OCBBF2007478, OCBBF2007485, OCBBF2007520, OCBBF2007555, OCBBF2007610, OCBBF2007622, OCBBF2007756, OCBBF2007762, OCBBF2007931, OCBBF2007946, OCBBF2008116, OCBBF2008138, OCBBF2008144, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724,
OCBBF2008760, OCBBF2008768, OCBBF2008770, OCBBF2008775, OCBBF2008790, OCBBF2008814, OCBBF2009095, OCBBF2009115, OCBBF2009352, OCBBF2009391, OCBBF2009424, OCBBF2009536, OCBBF2009571, OCBBF2009583, OCBBF2009603, OCBBF2009788, OCBBF2009920, OCBBF2009926, OCBBF2010040, OCBBF2010313, OCBBF2010404, OCBBF2010416, OCBBF2010420, OCBBF2010604, OCBBF2010709, OCBBF2010792, OCBBF2010819, OCBBF2010843, OCBBF2010858, OCBBF2010863, OCBBF2010871, OCBBF2010931, OCBBF2010945, OCBBF2010978, OCBBF2010989, OCBBF2011073, OCBBF2011137, OCBBF2011160, OCBBF2011177, OCBBF2011228, OCBBF2011232, OCBBF2011283, OCBBF2011311, OCBBF2011536, OCBBF2011625, OCBBF2011669, OCBBF2011685, OCBBF2011706, OCBBF2011722, OCBBF2011855,
OCBBF2011872, OCBBF2011897, OCBBF2011914, OCBBF2011960, OCBBF2011981, OCBBF2012028, OCBBF2012039, OCBBF2012095, OCBBF2012139, OCBBF2012191, OCBBF2012262, OCBBF2012320, OCBBF2012525, OCBBF2012678, OCBBF2012704, OCBBF2012755, OCBBF2012812, OCBBF2012881, OCBBF2012908, OCBBF2012936, OCBBF2012990, OCBBF2013049, OCBBF2013091, OCBBF2013127, OCBBF2013208, OCBBF2013285, OCBBF2013319, OCBBF2013324, OCBBF2013481, OCBBF2013507, OCBBF2013721, OCBBF2013789, OCBBF2013883, OCBBF2013926, OCBBF2014089, OCBBF2014292, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232,
OCBBF2015233, OCBBF2015285, OCBBF2015334, OCBBF2015335, OCBBF2015503, OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016689, OCBBF2016690, OCBBF2016841, OCBBF2016928, OCBBF2017069, OCBBF2017306, OCBBF2017325, OCBBF2017458, OCBBF2017489, OCBBF2017516, OCBBF2017665, OCBBF2017815, OCBBF2017882, OCBBF2017888, OCBBF2017920, OCBBF2018014, OCBBF2018084, OCBBF2018167, OCBBF2018206, OCBBF2018229, OCBBF2018234, OCBBF2018362, OCBBF2018380, OCBBF2018618, OCBBF2018663, OCBBF2018687, OCBBF2018707, OCBBF2018828, OCBBF2018873, OCBBF2018934, OCBBF2019195, OCBBF2019327,
OCBBF2019684, OCBBF2019761, OCBBF2019823, OCBBF2019950, OCBBF2020048, OCBBF2020343, OCBBF2020453, OCBBF2020741, OCBBF2020825, OCBBF2020838, OCBBF2021020, OCBBF2021214, OCBBF2021286, OCBBF2021323, OCBBF2021518, OCBBF2021788, OCBBF2021833, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026368, OCBBF2026645, OCBBF2026649, OCBBF2026690, OCBBF2026981, OCBBF2027148, OCBBF2027197, OCBBF2027354, OCBBF2027423, OCBBF2027478, OCBBF2027481,
OCBBF2027511, OCBBF2027661, OCBBF2027728, OCBBF2027782, OCBBF2027868, OCBBF2028055, OCBBF2028173, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035885, OCBBF2035916, OCBBF2036019, OCBBF2036031, OCBBF2036225, OCBBF2036476, OCBBF2036752, OCBBF2037068, OCBBF2037340,
OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000185, PEBLM1000024, PEBLM1000029, PEBLM1000034, PEBLM1000062,
PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000248, PEBLM2000267, PEBLM2000270, PEBLM2000321, PEBLM2000333, PEBLM2000479, PEBLM20.00502, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003935, PEBLM2004015, PEBLM2004216, PEBLM2004290, PEBLM2004497, PEBLM2004733, PEBLM2005282, PEBLM2005615, PEBLM2005632, PEBLM2005697, PEBLM2006031, PEBLM2006049, PEBLM2006113, PEBLM2006135, PEBLM2006215, PEBLM2006283, PEBLM2006298, PEBLM2006366, PEBLM2006912, PEBLM2007112, PEBLM2007140,
PEBLM2007437, PEBLM2007598, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287, PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117,
PERIC2005305, PERIC2005347, PERIC2005370, PERIC2005936, PERIC2006035, PERIC2006121, PERIC2006443, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000070, PLACE5000080, PLACE5000105, PLACE5000115, PLACE5000116, PLACE5000129, PLACE5000139, PLACE5000153, PLACE5000171, PLACE5000172, PLACE5000178, PLACE5000245, PLACE5000260, PLACE5000282, PLACE5000297, PLACE5000372, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000263,
PLACE6000296, PLACE6000314, PLACE6000348, PLACE6000371, PLACE6000424, PLACE6000426, PLACE6000429, PLACE6000450, PLACE6000523, PLACE6000550, PLACE6000552, PLACE6000555, PLACE6000619, PLACE6000953, PLACE6001118, PLACE6001153, PLACE6001185, PLACE6001262, PLACE6001294, PLACE6001536, PLACE6001557, PLACE6001712, PLACE6001740, PLACE6001823, PLACE6001886, PLACE6001923, PLACE6001925, PLACE6001933, PLACE6002011, PLACE6002016, PLACE6002056, PLACE6002157, PLACE6002312, PLACE6002323, PLACE6002345, PLACE6002419, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003372,
PLACE6003393, PLACE6003399, PLACE6003539, PLACE6003621, PLACE6003705, PLACE6003740, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004491, PLACE6004578, PLACE6004663, PLACE6004687, PLACE6004738, PLACE6004931, PLACE6004966, PLACE6004993, PLACE6005007, PLACE6005029, PLACE6005108, PLACE6005234, PLACE6005283, PLACE6005328, PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006217, PLACE6006222, PLACE6006266, PLACE6006287, PLACE6006394, PLACE6006418, PLACE6006474,
PLACE6006566, PLACE6006697, PLACE6006822, PLACE6006871, PLACE6006905, PLACE6006988, PLACE6007050, PLACE6007180, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453, PLACE6008640, PLACE6008750, PLACE6008768, PLACE6008824, PLACE6008989, PLACE6009006, PLACE6009008, PLACE6009228, PLACE6009338, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010485, PLACE6010568, PLACE6010701, PLACE6010704,
PLACE6010732, PLACE6010765, PLACE6010925, PLACE6010936, PLACE6010991, PLACE6011041, PLACE6011102, PLACE6011156, PLACE6011211, PLACE6011260, PLACE6011334, PLACE6011627, PLACE6011774, PLACE6011881, PLACE6011907, PLACE6011975, PLACE6012108, PLACE6012168, PLACE6012297, PLACE6012308, PLACE6012574, PLACE6012908, PLACE6012942, PLACE6013288, PLACE6013386, PLACE6013400, PLACE6013650, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015731, PLACE6015939, PLACE6016030, PLACE6016093, PLACE6016105, PLACE6016160, PLACE6016210, PLACE6016383,
PLACE6017002, PLACE6017431, PLACE6017564, PLACE6017578, PLACE6017626, PLACE6017701, PLACE6017714, PLACE6017788, PLACE6017791, PLACE6018107, PLACE6018187, PLACE6018235, PLACE6018287, PLACE6018441, PLACE6018497, PLACE6018769, PLACE6018834, PLACE6018843, PLACE6018863, PLACE6019385, PLACE6019542, PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001544, PLACE7001759, PLACE7001936, PLACE7002303, PLACE7002641, PLACE7003639, PLACE7003657, PLACE7003684, PLACE7003985, PLACE7004961, PLACE7005169, PLACE7005671, PLACE7006051, PLACE7006090, PLACE7006240,
PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110, PROST1000123, PROST1000136, PROST1000144, PROST1000145, PROST1000152, PROST1000167, PROST1000184, PROST1000217, PROST1000262, PROST1000272, PROST1000298, PROST1000322, PROST1000334, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000559, PROST1000575, PROST2000053, PROST2000067, PROST2000079, PROST2000105,
PROST2000143, PROST2000162, PROST2000165, PROST2000176, PROST2000203, PROST2000206, PROST2000212, PROST2000267, PROST2000273, PROST2000325, PROST2000337, PROST2000432, PROST2000452, PROST2000463, PROST2000470, PROST2000505, PROST2000572, PROST2000717, PROST2000761, PROST2000893, PROST2000961, PROST2001138, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001899, PROST2001998, PROST2002078, PROST2002101, PROST2002162, PROST2002212, PROST2002227, PROST2002338, PROST2002425, PROST2002488, PROST2002489, PROST2002527, PROST2002591,
PROST2002602, PROST2002633, PROST2002682, PROST2002685, PROST2002722, PROST2002736, PROST2002842, PROST2002906, PROST2002927, PROST2003102, PROST2003117, PROST2003210, PROST2003302, PROST2003303, PROST2003324, PROST2003340, PROST2003428, PROST2003472, PROST2003563, PROST2003577, PROST2003583, PROST2003586, PROST2003628, PROST2003635, PROST2003674, PROST2003676, PROST2003732, PROST2003775, PROST2003922, PROST2003930, PROST2004115, PROST2004142, PROST2004146, PROST2004161, PROST2004251, PROST2004258, PROST2004332, PROST2004481, PROST2004526, PROST2004570, PROST2004727, PROST2004742, PROST2004817, PROST2005039, PROST2005076, PROST2005093, PROST2005121, PROST2005131, PROST2005228, PROST2005426,
PROST2005466, PROST2005630, PROST2005683, PROST2005788, PROST2005880, PROST2005886, PROST2005904, PROST2005919, PROST2005943, PROST2006008, PROST2006020, PROST2006196, PROST2006201, PROST2006282, PROST2006450, PROST2006491, PROST2006510, PROST2006579, PROST2006688, PROST2006766, PROST2006782, PROST2006933, PROST2006988, PROST2007122, PROST2007200, PROST2007237, PROST2007248, PROST2007289, PROST2007317, PROST2007328, PROST2007382, PROST2007389, PROST2007416, PROST2007528, PROST2007818, PROST2007871, PROST2007950, PROST2007956, PROST2007974, PROST2007988, PROST2008079, PROST2008088, PROST2008243, PROST2008245, PROST2008268, PROST2008447, PROST2008468, PROST2008472, PROST2008516, PROST2008724,
PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010250, PROST2010318, PROST2010326, PROST2010382, PROST2010400, PROST2010606, PROST2010885, PROST2011012, PROST2011038, PROST2011105, PROST2011297, PROST2011410, PROST2011439, PROST2011482, PROST2011631, PROST2011660, PROST2012005, PROST2012016, PROST2012088, PROST2012140, PROST2012157, PROST2012190, PROST2012249, PROST2012353, PROST2012400, PROST2012542, PROST2012550, PROST2012740, PROST2012780,
PROST2012888, PROST2012890, PROST2013032, PROST2013053, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014916, PROST2014925, PROST2015124, PROST2015137, PROST2015198, PROST2015243, PROST2015246, PROST2015251, PROST2015332, PROST2015457, PROST2015464, PROST2015537, PROST2015545, PROST2015551, PROST2015636, PROST2015710, PROST2015756, PROST2015877, PROST2015900, PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829,
PROST2016860, PROST2016918, PROST2016980, PROST2017098, PROST2017367, PROST2017413, PROST2017441, PROST2017529, PROST2017578, PROST2017617, PROST2017692, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2018977, PROST2019296, PROST2019398, PROST2019487, PROST2019781, PUAEN1000039, PUAEN1000081, PUAEN1000085, PUAEN1000087, PUAEN1000121, PUAEN1000175, PUAEN1000239, PUAEN1000275, PUAEN1000276, PUAEN1000288, PUAEN1000322, PUAEN2000080, PUAEN2000175, PUAEN2000247, PUAEN2000394, PUAEN2000497,
PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004511, PUAEN2004525, PUAEN2004875, PUAEN2005110, PUAEN2005247, PUAEN2005502, PUAEN2005588, PUAEN2006029, PUAEN2006335, PUAEN2006578, PUAEN2006639, PUAEN2006761, PUAEN2007350, PUAEN2007785, PUAEN2007898, PUAEN2008123, PUAEN2008228, PUAEN2008314, PUAEN2008515, PUAEN2008815, PUAEN2008980, PUAEN2009348, PUAEN2009534, PUAEN2009655, PUAEN2009795, PUAEN2009852, PUAEN2010824, RECTM1000051, RECTM1000141,
RECTM2000220, RECTM2000349, RECTM2000433, RECTM2000510, RECTM2001307, RECTM2001347, RECTM2001519, RECTM2001666, RECTM2001691, RECTM2001749, RECTM2002602, RECTM2002632, SALGL1000005, SALGL1000024, SALGL1000028, SALGL1000065, SALGL1000107, SKMUS1000030, SKMUS1000064, SKMUS1000067, SKMUS1000102, SKMUS1000104, SKMUS1000118, SKMUS1000124, SKMUS1000129, SKMUS1000138, SKMUS1000176, SKMUS2000020, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000361, SKMUS2000365, SKMUS2000380, SKMUS2000390, SKMUS2000416, SKMUS2000458, SKMUS2000467, SKMUS2000468, SKMUS2000515, SKMUS2000690,
SKMUS2000701, SKMUS2000705, SKMUS2000710, SKMUS2000724, SKMUS2000726, SKMUS2000774, SKMUS2000780, SKMUS2000821, SKMUS2000847, SKMUS2000863, SKMUS2000873, SKMUS2000894, SKMUS2000898, SKMUS2000902, SKMUS2000945, SKMUS2000961, SKMUS2000973, SKMUS2000980, SKMUS2001008, SKMUS2001014, SKMUS2001129, SKMUS2001147, SKMUS2001157, SKMUS2001164, SKMUS2001199, SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001501, SKMUS2001543, SKMUS2001580, SKMUS2001585, SKMUS2001608, SKMUS2001631, SKMUS2001662, SKMUS2001663, SKMUS2001668, SKMUS2001671, SKMUS2001683, SKMUS2001693, SKMUS2001695,
SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667, SKMUS2004897, SKMUS2004903, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000110, SKNMC1000123, SKNMC1000124, SKNMC1000159, SKNMC2000256,
SKNMC2000305, SKNMC2000322, SKNMC2000374, SKNMC2000583, SKNMC2000593, SKNMC2000612, SKNMC2000622, SKNMC2000635, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643, SKNMC2004651, SKNMC2005772, SKNMC2006327, SKNMC2006998, SKNMC2007134, SKNMC2007502, SKNMC2007504, SKNMC2007961, SKNMC2009450, SKNSH1000086, SKNSH1000174, SKNSH1000301, SKNSH1000308, SKNSH1000416, SKNSH2000051, SKNSH2000151, SKNSH2000163, SKNSH2000319, SKNSH2000482, SKNSH2000516, SKNSH2000550, SKNSH2000716, SKNSH2000971, SKNSH2001875, SKNSH2001931, SKNSH2002054, SKNSH2002866, SKNSH2003064, SKNSH2003174,
SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006304, SKNSH2006432, SKNSH2006822, SKNSH2007142, SKNSH2007149, SKNSH2007429, SKNSH2007739, SKNSH2008043, SKNSH2008940, SKNSH2008969, SKNSH2009197, SKNSH2009357, SKNSH2009435, SKNSH2009991, SKNSH2010015, SMINT1000012, SMINT1000039, SMINT1000048, SMINT1000051, SMINT1000054, SMINT1000057, SMINT1000071, SMINT1000075, SMINT1000078, SMINT1000091, SMINT1000100, SMINT1000103, SMINT1000118, SMINT1000131, SMINT1000137, SMINT2000018, SMINT2000032, SMINT2000040, SMINT2000060, SMINT2000073, SMINT2000084, SMINT2000145, SMINT2000176,
SMINT2000185, SMINT2000227, SMINT2000229, SMINT2000267, SMINT2000400, SMINT2000518, SMINT2000529, SMINT2000530, SMINT2000537, SMINT2000538, SMINT2000541, SMINT2000558, SMINT2000562, SMINT2000568, SMINT2000609, SMINT2000811, SMINT2000824, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001950, SMINT2002126, SMINT2002202, SMINT2002210, SMINT2002281, SMINT2002311, SMINT2002314, SMINT2002328, SMINT2002414, SMINT2002457, SMINT2002743, SMINT2002778, SMINT2002880, SMINT2002884, SMINT2002899, SMINT2002974,
SMINT2003128, SMINT2003150, SMINT2003317, SMINT2003386, SMINT2003551, SMINT2003569, SMINT2003641, SMINT2003644, SMINT2003866, SMINT2004047, SMINT2004086, SMINT2004299, SMINT2004339, SMINT2004350, SMINT2004414, SMINT2004473, SMINT2004547, SMINT2004583, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005368, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716, SMINT2007101, SMINT2007140, SMINT2007219, SMINT2007294,
SMINT2007365, SMINT2007391, SMINT2007433, SMINT2007508, SMINT2007526, SMINT2007579, SMINT2007647, SMINT2007729, SMINT2007790, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2009119, SMINT2009121, SMINT2009212, SMINT2009216, SMINT2009233, SMINT2009259, SMINT2009272, SMINT2009363, SMINT2009415, SMINT2009468, SMINT2009505, SMINT2009529, SMINT2009832, SMINT2009895, SMINT2009902, SMINT2009973, SMINT2010015, SMINT2010068,
SMINT2010076, SMINT2010144, SMINT2010200, SMINT2010278, SMINT2010324, SMINT2010326, SMINT2010426, SMINT2010500, SMINT2010533, SMINT2010629, SMINT2010639, SMINT2010672, SMINT2010753, SMINT2010853, SMINT2010897, SMINT2010959, SMINT2010997, SMINT2011033, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012122, SMINT2012195, SMINT2012285, SMINT2012501, SMINT2012734, SMINT2012793, SMINT2013129, SMINT2013170, SMINT2013181, SMINT2013228, SMINT2013613, SMINT2013626, SMINT2013695, SMINT2013833, SMINT2013890, SMINT2014166, SMINT2014282, SMINT2014443, SMINT2014480, SMINT2014489, SMINT2014721,
SMINT2015006, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017974, SMINT2018343, SMINT2018353, SMINT2018592, SMINT2018681, SMINT2019017, SMINT2019105, SMINT2019142, SMINT2019153, SMINT2019200, SPLEN1000013, SPLEN1000049, SPLEN1000087, SPLEN1000089, SPLEN1000091, SPLEN1000139, SPLEN1000143, SPLEN1000144,
SPLEN1000145, SPLEN1000156, SPLEN1000163, SPLEN1000165, SPLEN1000178, SPLEN2000020, SPLEN2000064, SPLEN2000126, SPLEN2000134, SPLEN2000189, SPLEN2000197, SPLEN2000205, SPLEN2000210, SPLEN2000222, SPLEN2000242, SPLEN2000247, SPLEN2000258, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464, SPLEN2000496, SPLEN2000515, SPLEN2000537, SPLEN2000541, SPLEN2000607, SPLEN2000630, SPLEN2000695, SPLEN2000698, SPLEN2000839, SPLEN2001135, SPLEN2001176, SPLEN2001227, SPLEN2001245, SPLEN2001354, SPLEN2001367, SPLEN2001383, SPLEN2001503, SPLEN2001510,
SPLEN2001599, SPLEN2001626, SPLEN2001650, SPLEN2001689, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002272, SPLEN2002314, SPLEN2002343, SPLEN2002354, SPLEN2002385, SPLEN2002419, SPLEN2002462, SPLEN2002467, SPLEN2002477, SPLEN2002662, SPLEN2002695, SPLEN2002707, SPLEN2002917, SPLEN2002957, SPLEN2003160, SPLEN2003204, SPLEN2003219, SPLEN2003396, SPLEN2003678, SPLEN2003878, SPLEN2003918, SPLEN2003924, SPLEN2004060, SPLEN2004078, SPLEN2004124, SPLEN2004148, SPLEN2004343, SPLEN2004346, SPLEN2004368, SPLEN2004844, SPLEN2004880, SPLEN2005009, SPLEN2005227, SPLEN2005272, SPLEN2005450,
SPLEN2005474, SPLEN2005727, SPLEN2005838, SPLEN2005869, SPLEN2005927, SPLEN2005939, SPLEN2006268, SPLEN2006283, SPLEN2006325, SPLEN2006389, SPLEN2006425, SPLEN2006671, SPLEN2006701, SPLEN2006785, SPLEN2006875, SPLEN2007276, SPLEN2007314, SPLEN2007350, SPLEN2007619, SPLEN2007653, SPLEN2007689, SPLEN2007695, SPLEN2007750, SPLEN2007926, SPLEN2007951, SPLEN2008007, SPLEN2008135, SPLEN2008164, SPLEN2008317, SPLEN2008496, SPLEN2008591, SPLEN2008786, SPLEN2009088, SPLEN2009315, SPLEN2009340, SPLEN2009541, SPLEN2009548, SPLEN2009555, SPLEN2009581, SPLEN2009733, SPLEN2009803, SPLEN2009884, SPLEN2009918, SPLEN2010004, SPLEN2010112, SPLEN2010119, SPLEN2010146, SPLEN2010187, SPLEN2010350, SPLEN2010395,
SPLEN2010415, SPLEN2010447, SPLEN2010469, SPLEN2010510, SPLEN2010530, SPLEN2010534, SPLEN2010588, SPLEN2010800, SPLEN2010847, SPLEN2010862, SPLEN2010978, SPLEN2010986, SPLEN2011018, SPLEN2011025, SPLEN2011086, SPLEN2011139, SPLEN2011145, SPLEN2011248, SPLEN2011252, SPLEN2011419, SPLEN2011422, SPLEN2011672, SPLEN2011678, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012524, SPLEN2012525, SPLEN2012606, SPLEN2012611, SPLEN2012619, SPLEN2012725, SPLEN2012743, SPLEN2012756, SPLEN2012961, SPLEN2013195, SPLEN2013503, SPLEN2013644, SPLEN2013648, SPLEN2013673, SPLEN2013690, SPLEN2013714,
SPLEN2013821, SPLEN2013857, SPLEN2013860, SPLEN2013910, SPLEN2013936, SPLEN2014063, SPLEN2014073, SPLEN2014119, SPLEN2014136, SPLEN2014199, SPLEN2014210, SPLEN2014293, SPLEN2014318, SPLEN2014381, SPLEN2014454, SPLEN2014486, SPLEN2014543, SPLEN2014572, SPLEN2014645, SPLEN2014669, SPLEN2014711, SPLEN2014860, SPLEN2014865, SPLEN2014919, SPLEN2014920, SPLEN2014924, SPLEN2014937, SPLEN2014946, SPLEN2015031, SPLEN2015094, SPLEN2015129, SPLEN2015158, SPLEN2015166, SPLEN2015216, SPLEN2015258, SPLEN2015261, SPLEN2015267, SPLEN2015276, SPLEN2015415, SPLEN2015433, SPLEN2015593, SPLEN2015674, SPLEN2015679, SPLEN2015707, SPLEN2015730, SPLEN2015746, SPLEN2015815, SPLEN2015890, SPLEN2015891, SPLEN2015899,
SPLEN2016045, SPLEN2016053, SPLEN2016069, SPLEN2016135, SPLEN2016241, SPLEN2016250, SPLEN2016304, SPLEN2016356, SPLEN2016480, SPLEN2016531, SPLEN2016533, SPLEN2016541, SPLEN2016552, SPLEN2016554, SPLEN2016602, SPLEN2016627, SPLEN2016773, SPLEN2016840, SPLEN2016863, SPLEN2016922, SPLEN2016932, SPLEN2016972, SPLEN2016973, SPLEN2017104, SPLEN2017121, SPLEN2017147, SPLEN2017187, SPLEN2017189, SPLEN2017212, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017918, SPLEN2017999, SPLEN2018098, SPLEN2018112, SPLEN2018159, SPLEN2018180, SPLEN2018181, SPLEN2018280, SPLEN2018285, SPLEN2018302, SPLEN2018364,
SPLEN2018395, SPLEN2018643, SPLEN2018732, SPLEN2018749, SPLEN2018933, SPLEN2019008, SPLEN2019043, SPLEN2019092, SPLEN2019102, SPLEN2019131, SPLEN2019257, SPLEN2019311, SPLEN2019323, SPLEN2019349, SPLEN2019375, SPLEN2019379, SPLEN2019471, SPLEN2019480, SPLEN2019571, SPLEN2019575, SPLEN2019709, SPLEN2019774, SPLEN2019793, SPLEN2019811, SPLEN2019839, SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021273, SPLEN2021295, SPLEN2021298, SPLEN2021383, SPLEN2021417,
SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023160, SPLEN2023423, SPLEN2023710, SPLEN2023791, SPLEN2023977, SPLEN2024070, SPLEN2024127, SPLEN2024273, SPLEN2024383, SPLEN2024530, SPLEN2024571, SPLEN2024577, SPLEN2024617, SPLEN2024737, SPLEN2024956, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138,
SPLEN2028365, SPLEN2028417, SPLEN2028424, SPLEN2028593, SPLEN2028844, SPLEN2028994, SPLEN2029051, SPLEN2029176, SPLEN2029380, SPLEN2029522, SPLEN2029727, SPLEN2029904, SPLEN2030250, SPLEN2030335, SPLEN2030371, SPLEN2030397, SPLEN2030479, SPLEN2030495, SPLEN2030562, SPLEN2030763, SPLEN2030847, SPLEN2031004, SPLEN2031125, SPLEN2031424, SPLEN2031724, SPLEN2031780, SPLEN2032036, SPLEN2032112, SPLEN2032154, SPLEN2032321, SPLEN2032356, SPLEN2032677, SPLEN2032813, SPLEN2032924, SPLEN2033153, SPLEN2033333, SPLEN2033490, SPLEN2033539, SPLEN2033921, SPLEN2033996, SPLEN2034021, SPLEN2034081, SPLEN2034252, SPLEN2034551, SPLEN2034601, SPLEN2034678, SPLEN2034864, SPLEN2034934, SPLEN2035318, SPLEN2035615,
SPLEN2036103, SPLEN2036326, SPLEN2036475, SPLEN2036579, SPLEN2036608, SPLEN2036702, SPLEN2036712, SPLEN2036821, SPLEN2036932, SPLEN2036953, SPLEN2037077, SPLEN2037194, SPLEN2037319, SPLEN2037444, SPLEN2037580, SPLEN2037630, SPLEN2037678, SPLEN2037891, SPLEN2038055, SPLEN2038180, SPLEN2038345, SPLEN2038407, SPLEN2038473, SPLEN2039311, SPLEN2039697, SPLEN2039845, SPLEN2039936, SPLEN2040222, SPLEN2040237, SPLEN2040824, SPLEN2041304, SPLEN2041310, SPLEN2041585, SPLEN2041645, SPLEN2041700, SPLEN2041720, SPLEN2041977, SPLEN2042051, SPLEN2042521, SPLEN2042598, SPLEN2042714, SPLEN2042920, STOMA1000013, STOMA1000052, STOMA1000074, STOMA1000091, STOMA1000117, STOMA1000133, STOMA1000175, STOMA1000186,
STOMA1000190, STOMA1000191, STOMA2000032, STOMA2000072, STOMA2000088, STOMA2000104, STOMA2000148, STOMA2000168, STOMA2000183, STOMA2000188, STOMA2000279, STOMA2000289, STOMA2000383, STOMA2000386, STOMA2000396, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002141, STOMA2002367, STOMA2002688, STOMA2003289, STOMA2003444, STOMA2003477, STOMA2003646, STOMA2003894, STOMA2004194, STOMA2004294, STOMA2004663, STOMA2004668, STOMA2004852, STOMA2004873, STOMA2004884, STOMA2004893, STOMA2004925, STOMA2005120, STOMA2005664, STOMA2005667, STOMA2005746, STOMA2005782, STOMA2006181, STOMA2006213,
STOMA2006229, STOMA2006325, STOMA2006330, STOMA2006398, STOMA2006447, STOMA2006780, STOMA2006904, STOMA2007269, STOMA2007680, STOMA2007745, STOMA2008050, STOMA2008361, STOMA2008546, STOMA2008614, STOMA2008638, STOMA2008838, STOMA2009250, STOMA2009253, STOMA2009256, SYNOV1000045, SYNOV1000049, SYNOV1000118, SYNOV1000128, SYNOV1000190, SYNOV1000256, SYNOV2000152, SYNOV2000171, SYNOV2000173, SYNOV2000177, SYNOV2000178,-SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251, SYNOV2001300,
SYNOV2001356, SYNOV2001390, SYNOV2001451, SYNOV2001457, SYNOV2001468, SYNOV2001660, SYNOV2003326, SYNOV2005216, SYNOV2005448, SYNOV2005673, SYNOV2005817, SYNOV2005962, SYNOV2006430, SYNOV2006620, SYNOV2007965, SYNOV2008765, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000598, SYNOV4000706, SYNOV4001224, SYNOV4001342, SYNOV4001395, SYNOV4002001, SYNOV4002392, SYNOV4002875, SYNOV4002883, SYNOV4003174, SYNOV4003322, SYNOV4003681, SYNOV4003981,
SYNOV4004184, SYNOV4004741, SYNOV4004914, SYNOV4005256, SYNOV4005570, SYNOV4005739, SYNOV4005889, SYNOV4005981, SYNOV4006256, SYNOV4007215, SYNOV4007360, SYNOV4007430, SYNOV4007521, SYNOV4007711, SYNOV4008336, SYNOV4009129, SYNOV4009139, SYNOV4009298, SYNOV4009575, T1ESE2002665, TBAES2000004, TBAES2000059, TBAES2000116, TBAES2000255, TBAES2000315, TBAES2000352, TBAES2000932, TBAES2001171, TBAES2001220, TBAES2001229, TBAES2001258, TBAES2001492, TBAES2001751, TBAES2003435, TBAES2003492, TBAES2003550, TBAES2003702, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504,
TBAES2007548, TBAES2007697, TBAES2007862, TBAES2007964, TBAES2008133, TBAES2009387, TCERX2000171, TCERX2000431, TCERX2000613, TCERX2000998, TCERX2002644, TCOLN1000037, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474, TESOP2001796, TESOP2001818, TESOP2001849, TESOP2001865, TESOP2001901, TESOP2002005, TESOP2002110, TESOP2002150, TESOP2002273, TESOP2002489, TESOP2002539, TESOP2002545, TESOP2002804, TESOP2002950,
TESOP2003273, TESOP2003308, TESOP2003553, TESOP2003753, TESOP2004110, TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2006893, TESOP2007041, TESOP2007052, TESOP2007262, TESOP2007636, TESOP2007674, TESOP2007688, TESOP2007725, TESOP2007912, TESOP2007978, TESOP2008127, TESOP2008552, TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000055,
TESTI1000064, TESTI1000070, TESTI1000085, TESTI1000088, TESTI1000093, TESTI1000094, TESTI1000101, TESTI1000109, TESTI1000125, TESTI1000127, TESTI1000128, TESTI1000131, TESTI1000157, TESTI1000163, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000018, TESTI2000044, TESTI2000100, TESTI2000117, TESTI2000120, TESTI2000147, TESTI2000154, TESTI2000172, TESTI2000207, TESTI2000208, TESTI2000221, TESTI2000238, TESTI2000253, TESTI2000278, TESTI2000349, TESTI2000356, TESTI2000358, TESTI2000372, TESTI2000427, TESTI2000435, TESTI2000443, TESTI2000452,
TESTI2000462, TESTI2000489, TESTI2000571, TESTI2000591, TESTI2000600, TESTI2000616, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874, TESTI2000883, TESTI2000909, TESTI2000951, TESTI2000981, TESTI2001008, TESTI2001049, TESTI2001082, TESTI2001134, TESTI2001141, TESTI2001180, TESTI2001208, TESTI2001229, TESTI2001236, TESTI2001237, TESTI2001306, TESTI2001313, TESTI2001330, TESTI2001339, TESTI2001345, TESTI2001352, TESTI2001364, TESTI2001412, TESTI2001420, TESTI2001498, TESTI2001512, TESTI2001518, TESTI2001556,
TESTI2001593, TESTI2001613, TESTI2001621, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001829, TESTI2001852, TESTI2001869, TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002149, TESTI2002216, TESTI2002245, TESTI2002251, TESTI2002264, TESTI2002282, TESTI2002293, TESTI2002294, TESTI2002326, TESTI2002351, TESTI2002361, TESTI2002406, TESTI2002415, TESTI2002423, TESTI2002453,
TESTI2002461, TESTI2002465, TESTI2002467, TESTI2002498, TESTI2002516, TESTI2002544, TESTI2002578, TESTI2002580, TESTI2002618, TESTI2002632, TESTI2002676, TESTI2002690, TESTI2002696, TESTI2002698, TESTI2002700, TESTI2002724, TESTI2002729, TESTI2002752, TESTI2002753, TESTI2002789, TESTI2002802, TESTI2002806, TESTI2002840, TESTI2002910, TESTI2002912, TESTI2002928, TESTI2002937, TESTI2002993, TESTI2003005, TESTI2003020, TESTI2003031, TESTI2003037, TESTI2003044, TESTI2003059, TESTI2003061, TESTI2003071, TESTI2003074, TESTI2003089, TESTI2003104, TESTI2003117, TESTI2003127, TESTI2003130, TESTI2003131, TESTI2003139, TESTI2003141, TESTI2003152, TESTI2003181, TESTI2003193, TESTI2003196, TESTI2003228,
TESTI2003236, TESTI2003255, TESTI2003258, TESTI2003277, TESTI2003299, TESTI2003311, TESTI2003321, TESTI2003325, TESTI2003327, TESTI2003330, TESTI2003347, TESTI2003354, TESTI2003356, TESTI2003372, TESTI2003376, TESTI2003418, TESTI2003419, TESTI2003432, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998,
TESTI2004000, TESTI2004073, TESTI2004085, TESTI2004089, TESTI2004111, TESTI2004119, TESTI2004122, TESTI2004153, TESTI2004163, TESTI2004169, TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004929, TESTI2004936,
TESTI2004941, TESTI2004971, TESTI2004982, TESTI2004993, TESTI2004994, TESTI2004999, TESTI2005017, TESTI2005025, TESTI2005040, TESTI2005060, TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005415, TESTI2005470, TESTI2005492, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005703, TESTI2005720, TESTI2005739, TESTI2005742, TESTI2005767, TESTI2005775, TESTI2005827,
TESTI2005835, TESTI2005860, TESTI2005892, TESTI2005911, TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006051, TESTI2006067, TESTI2006083, TESTI2006097, TESTI2006109, TESTI2006111, TESTI2006125, TESTI2006179, TESTI2006209, TESTI2006210, TESTI2006212, TESTI2006218, TESTI2006238, TESTI2006255, TESTI2006278, TESTI2006333, TESTI2006341, TESTI2006360, TESTI2006409, TESTI2006425, TESTI2006437, TESTI2006457, TESTI2006460, TESTI2006465, TESTI2006483, TESTI2006499, TESTI2006523, TESTI2006543, TESTI2006565,
TESTI2006572, TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006735, TESTI2006744, TESTI2006748, TESTI2006774, TESTI2006853, TESTI2006866, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007452, TESTI2007466, TESTI2007480, TESTI2007490, TESTI2007502, TESTI2007524, TESTI2007533, TESTI2007576, TESTI2007613, TESTI2007657,
TESTI2007685, TESTI2007692, TESTI2007749, TESTI2007750, TESTI2007808, TESTI2007814, TESTI2007829, TESTI2007867, TESTI2007872, TESTI2007906, TESTI2007922, TESTI2007951, TESTI2007972, TESTI2007973, TESTI2008014, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008420, TESTI2008425, TESTI2008440, TESTI2008567, TESTI2008595, TESTI2008621, TESTI2008636, TESTI2008657, TESTI2008684, TESTI2008699, TESTI2008715, TESTI2008762, TESTI2008774, TESTI2008786,
TESTI2008822, TESTI2008835, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009412, TESTI2009423, TESTI2009447, TESTI2009462, TESTI2009477, TESTI2009497, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935, TESTI2009977, TESTI2010009, TESTI2010154, TESTI2010239, TESTI2010280, TESTI2010354, TESTI2010396, TESTI2010400, TESTI2010409, TESTI2010572,
TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011683, TESTI2011712, TESTI2011750, TESTI2011840, TESTI2011846, TESTI2011915, TESTI2011928, TESTI2011971, TESTI2012019, TESTI2012050, TESTI2012090, TESTI2012095, TESTI2012104, TESTI2012152, TESTI2012155, TESTI2012171, TESTI2012207, TESTI2012231, TESTI2012244,
TESTI2012289, TESTI2012308, TESTI2012356, TESTI2012387, TESTI2012444, TESTI2012511, TESTI2012528, TESTI2012544, TESTI2012550, TESTI2012571, TESTI2012592, TESTI2012617, TESTI2012628, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013257, TESTI2013268, TESTI2013322, TESTI2013381, TESTI2013382, TESTI2013401, TESTI2013427, TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889,
TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014577, TESTI2014578, TESTI2014716, TESTI2014780, TESTI2014800, TESTI2014843, TESTI2014979, TESTI2014988, TESTI2015042, TESTI2015105, TESTI2015180, TESTI2015246, TESTI2015249, TESTI2015327, TESTI2015331, TESTI2015335, TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015987, TESTI2016046, TESTI2016101, TESTI2016162, TESTI2016197,
TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102, TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951,
TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018941, TESTI2018957, TESTI2018974, TESTI2018975, TESTI2019042, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019787, TESTI2019794, TESTI2019854, TESTI2019859, TESTI2019860, TESTI2019872,
TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020515, TESTI2020525, TESTI2020579, TESTI2020717, TESTI2020871, TESTI2020905, TESTI2020906, TESTI2020946, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021315, TESTI2021358, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895,
TESTI2021911, TESTI2021939, TESTI2021999, TESTI2022010, TESTI2022023, TESTI2022065, TESTI2022086, TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874, TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267,
TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024885, TESTI2024936, TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656,TESTI2025791, TESTI2025846, TESTI2025872, TESTI2025879, TESTI2025911, TESTI2025920, TESTI2025924, TESTI2026014, TESTI2026024,
TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491, TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027165, TESTI2027171, TESTI2027173, TESTI2027179, TESTI2027185, TESTI2027206, TESTI2027238, TESTI2027239, TESTI2027243, TESTI2027296, TESTI2027378, TESTI2027411, TESTI2027450, TESTI2027496,
TESTI2027503, TESTI2027562, TESTI2027615, TESTI2027645, TESTI2027730, TESTI2027736, TESTI2027791, TESTI2027820, TESTI2027828, TESTI2027840, TESTI2028098, TESTI2028151, TESTI2028242, TESTI2028253, TESTI2028254, TESTI2028269, TESTI2028290, TESTI2028296, TESTI2028384, TESTI2028426, TESTI2028488, TESTI2028523, TESTI2028583, TESTI2028613, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029244, TESTI2029249, TESTI2029252, TESTI2029264, TESTI2029470, TESTI2029672, TESTI2029880, TESTI2030136, TESTI2030322, TESTI2030342, TESTI2030519, TESTI2030556, TESTI2030728, TESTI2030754,
TESTI2030770, TESTI2030860, TESTI2030901, TESTI2030917, TESTI2030930, TESTI2031006, TESTI2031060, TESTI2031129, TESTI2031356, TESTI2031418, TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032681, TESTI2032761, TESTI2032768, TESTI2032774, TESTI2032828, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034357, TESTI2034401, TEST.12034407, TESTI2034506, TESTI2034520, TESTI2034718, TESTI2034730, TESTI2034749, TESTI2034767, TESTI2034774, TESTI2034777,
TESTI2034785, TESTI2034799, TESTI2034847, TESTI2034913, TESTI2034931, TESTI2034953, TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375,
TESTI2037382, TESTI2037534, TESTI2037569, TESTI2037572, TESTI2037643, TESTI2037657, TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039732, TESTI2039738, TESTI2039776, TESTI2039867, TESTI2039908, TESTI2040018, TESTI2040094, TESTI2040102, TESTI2040128, TESTI2040143,
TESTI2040297, TESTI2040372, TESTI2040377, TESTI2040424, TESTI2040642, TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866,
TESTI2043869, TESTI2044064, TESTI2044118, TESTI2044194, TESTI2044306, TESTI2044309, TESTI2044413, TESTI2044418, TESTI2044754, TESTI2044788, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212,
TESTI2047342, TESTI2047383, TESTI2047792, TESTI2047801, TESTI2047818, TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698,
TESTI2052799, TESTI2052822, TESTI2052985, TESTI2053151, TESTI2053242, TESTI2053399, TESTI2053526, TESTI2053561, TESTI2053621, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000370, TESTI4000394, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473,
TESTI4001517, TESTI4001527, TESTI4001569, TESTI4001604, TESTI4001665, TESTI4001679, TESTI4001795, TESTI4001923, TESTI4001925, TESTI4001984, TESTI4002003, TESTI4002072, TESTI4002141, TESTI4002195, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4003279, TESTI4003319, TESTI4003404, TESTI4003565, TESTI4003574, TESTI4003733, TESTI4003796, TESTI4003944, TESTI4004003, TESTI4004031, TESTI4004200, TESTI4004539, TESTI4004626, TESTI4004653, TESTI4004695, TESTI4004722, TESTI4004887, TESTI4004917, TESTI4005015, TESTI4005039, TESTI4005158, TESTI4005317, TESTI4005322, TESTI4005470, TESTI4005500,
TESTI4005534, TESTI4005543, TESTI4005628, TESTI4005801, TESTI4005805, TESTI4005857, TESTI4005961, TESTI4006053, TESTI4006079, TESTI4006112, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007965, TESTI4008007, TESTI4008018, TESTI4008050, TESTI4008058, TESTI4008086, TESTI4008097,
TESTI4008176, TESTI4008209, TESTI4008219, TESTI4008250, TESTI4008302, TESTI4008305, TESTI4008401, TESTI4008417, TESTI4008429, TESTI4008573, TESTI4008797, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789, TESTI4010817, TESTI4010831, TESTI4010902, TESTI4010928, TESTI4011070, TESTI4011072, TESTI4011084,
TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013602, TESTI4013667, TESTI4013675, TESTI4013685, TESTI4013742, TESTI4013774, TESTI4013817, TESTI4013830, TESTI4013852, TESTI4013894, TESTI4013924, TESTI4013960, TESTI4013962, TESTI4014159, TESTI4014175, TESTI4014265, TESTI4014276, TESTI4014306, TESTI4014392,
TESTI4014415, TESTI4014445, TESTI4014553, TESTI4014661, TESTI4014694, TESTI4014801, TESTI4014818, TESTI4014891, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110; TESTI4016238, TESTI4016551, TESTI4016812, TESTI4016822, TESTI4016865, TESTI4016882, TESTI4017001, TESTI4017116, TESTI4017137, TESTI4017229, TESTI4017254, TESTI4017269, TESTI4017380, TESTI4017382, TESTI4017543, TESTI4017575, TESTI4017647, TESTI4017714, TESTI4017763, TESTI4017848, TESTI4017854,
TESTI4017901, TESTI4017961, TESTI4017984, TESTI4018101, TESTI4018208, TESTI4018382, TESTI4018436, TESTI4018506, TESTI4018555, TESTI4018751, TESTI4018806, TESTI4018835, TESTI4018859, TESTI4018881, TESTI4018886, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569, TESTI4021713, TESTI4021821, TESTI4022158, TESTI4022577, TESTI4022716, TESTI4022873, TESTI4023096, TESTI4023172, TESTI4023546, TESTI4023555, TESTI4023654,
TESTI4023722, TESTI4023762, TESTI4023942, TESTI4024096, TESTI4024240, TESTI4024294, TESTI4024344, TESTI4024387, TESTI4024420, TESTI4024494, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139, TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612,
TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856, TESTI4032895, TESTI4033146, TESTI4033177, TESTI4033433, TESTI4034172, TESTI4034212, TESTI4034432, TESTI4034495, TESTI4034632, TESTI4034633, TESTI4034912, TESTI4034973, TESTI4035063,
TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758, TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800,
TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043166, TESTI4043203, TESTI4043223, TESTI4043371, TESTI4043378, TESTI4043512, TESTI4043551, TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044770, TESTI4045168, TESTI4045312, TESTI4045330,
TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, TUTER1000014, TUTER1000137, TUTER2000057, TUTER2000283, TUTER2000425, TUTER2000916, TUTER2001286, TUTER2001341, TUTER2001387, TUTER2001433, TUTER2001461, TUTER2002028, TUTER2002074, TUTER2002158, TUTER2002228, TUTER2002356, TUTER2002729, UMVEN1000122, UMVEN1000156, UMVEN1000186,
UMVEN2000046, UMVEN2000069, UTERU1000015, UTERU1000024, UTERU1000031, UTERU1000032, UTERU1000057, UTERU1000065, UTERU1000077, UTERU1000093, UTERU1000096, UTERU1000109, UTERU1000131, UTERU1000138, UTERU1000148, UTERU1000160, UTERU1000183, UTERU1000187, UTERU1000192, UTERU1000249, UTERU1000337, UTERU1000339, UTERU2000023, UTERU2000047, UTERU2000074, UTERU2000099, UTERU2000154, UTERU2000197, UTERU2000218, UTERU2000238, UTERU2000243, UTERU2000300, UTERU2000329, UTERU2000332, UTERU2000338, UTERU2000349, UTERU2000377, UTERU2000393, UTERU2000418, UTERU2000465, UTERU2000485, UTERU2000517, UTERU2000524, UTERU2000539, UTERU2000541, UTERU2000542, UTERU2000546, UTERU2000550, UTERU2000569, UTERU2000607,
UTERU2000629, UTERU2000649, UTERU2000663, UTERU2000696, UTERU2000794, UTERU2000830, UTERU2000844, UTERU2000922, UTERU2000925, UTERU2001024, UTERU2001110, UTERU2001176, UTERU2001281, UTERU2001389, UTERU2001409, UTERU2001412, UTERU2001504, UTERU2001658, UTERU2001747, UTERU2001876, UTERU2002001, UTERU2002011, UTERU2002176, UTERU2002198, UTERU2002332, UTERU2002473, UTERU2002547, UTERU2002693, UTERU2002733, UTERU2002736, UTERU2002737, UTERU2002826, UTERU2002841, UTERU2002964, UTERU2002993, UTERU2003035, UTERU2003057, UTERU2003126, UTERU2003135, UTERU2003321, UTERU2003399, UTERU2003411, UTERU2003456, UTERU2003577, UTERU2003926, UTERU2003973, UTERU2004015, UTERU2004039, UTERU2004061, UTERU2004073,
UTERU2004163, UTERU2004299, UTERU2004461, UTERU2004520, UTERU2004564, UTERU2004664, UTERU2004688, UTERU2004698, UTERU2004929, UTERU2005050, UTERU2005069, UTERU2005179, UTERU2005346, UTERU2005446, UTERU2005450, UTERU2005533, UTERU2005664, UTERU2005822, UTERU2005903, UTERU2005905, UTERU2006103, UTERU2006137, UTERU2006182, UTERU2006400, UTERU2006412, UTERU2006429, UTERU2006486, UTERU2006524, UTERU2006547, UTERU2006568, UTERU2006643, UTERU2006651, UTERU2006705, UTERU2006899, UTERU2007004, UTERU2007075, UTERU2007081, UTERU2007128, UTERU2007253, UTERU2007267, UTERU2007444, UTERU2007499, UTERU2007639, UTERU2007724, UTERU2007924, UTERU2007942, UTERU2008018, UTERU2008019, UTERU2008027, UTERU2008040,
UTERU2008077, UTERU2008130, UTERU2008347, UTERU2008516, UTERU2008561, UTERU2008653, UTERU2008705, UTERU2008707, UTERU2008747, UTERU2008785, UTERU2008845, UTERU2008901, UTERU2008930, UTERU2008938, UTERU2008939, UTERU2008962, UTERU2009094, UTERU2009120, UTERU2009131, UTERU2009147, UTERU2009206, UTERU2009283', UTERU2009414, UTERU2009483, UTERU2009510, UTERU2009538, UTERU2009540, UTERU2009776, UTERU2009904, UTERU2009951, UTERU2010115, UTERU2010124, UTERU2010164, UTERU2010231, UTERU2010304, UTERU2010320, UTERU2010431, UTERU2010525, UTERU2010651, UTERU2010724, UTERU2010747, UTERU2011195, UTERU2011199, UTERU2011261, UTERU2011410, UTERU2011574, UTERU2011657, UTERU2011806, UTERU2011811, UTERU2011897,
UTERU2011962, UTERU2011968, UTERU2012031, UTERU2012101, UTERU2012114, UTERU2012252, UTERU2012333, UTERU2012407, UTERU2012526, UTERU2012581, UTERU2012610, UTERU2012615, UTERU2012703, UTERU2012715, UTERU2012786, UTERU2012856, UTERU2012890, UTERU2012938, UTERU2012976, UTERU2013048, UTERU2013078, UTERU2013231, UTERU2013262, UTERU2013280, UTERU2013322, UTERU2013483, UTERU2013491, UTERU2013502, UTERU2013586, UTERU2013926, UTERU2013976, UTERU2014001, UTERU2014024, UTERU2014167, UTERU2014223, UTERU2014398, UTERU2014464, UTERU2014548, UTERU2014601, UTERU2014631, UTERU2014668, UTERU2014678, UTERU2014728, UTERU2014898, UTERU2014998, UTERU2015062, UTERU2015087, UTERU2015108, UTERU2015190, UTERU2015198,
UTERU2015202, UTERU2015405, UTERU2015640, UTERU2015880, UTERU2016147, UTERU2016157, UTERU2016426, UTERU2016464, UTERU2016669, UTERU2016757, UTERU2016761, UTERU2016896, UTERU2016902, UTERU2016979, UTERU2016981, UTERU2017123, UTERU2017303, UTERU2017421, UTERU2017492, UTERU2017613, UTERU2017623, UTERU2017632, UTERU2017761, UTERU2017762, UTERU2017810, UTERU2017988, UTERU2018127, UTERU2018180, UTERU2018200, UTERU2018333, UTERU2018364, UTERU2018514, UTERU2018522, UTERU2018523, UTERU2018544, UTERU2018566, UTERU2018609, UTERU2018674, UTERU2018712, UTERU20187.84, UTERU2018789, UTERU2018811, UTERU2018867, UTERU2018881, UTERU2018955, UTERU2019038, UTERU2019163, UTERU2019257, UTERU2019453, UTERU2019491,
UTERU2019534, UTERU2019681, UTERU2019706, UTERU2019710, UTERU2019940, UTERU2019959, UTERU2019964, UTERU2020226, UTERU2020242, UTERU2020292, UTERU2020351, UTERU2020491, UTERU2020718, UTERU2021163, UTERU2021380, UTERU2021649, UTERU2021820, UTERU2022020, UTERU2022773, UTERU2022955, UTERU2022981, UTERU2023039, UTERU2023045, UTERU2023262, UTERU2023687, UTERU2023712, UTERU2023941, UTERU2024002, UTERU2024042, UTERU2024141, UTERU2024481, UTERU2024758, UTERU2024820, UTERU2024881, UTERU2025025, UTERU2025041, UTERU2025301, UTERU2025366, UTERU2025415, UTERU2025579, UTERU2025645, UTERU2025891, UTERU2026025, UTERU2026090, UTERU2026142, UTERU2026203, UTERU2026775, UTERU2027023, UTERU2027369, UTERU2027591,
UTERU2027616, UTERU2027941, UTERU2028377, UTERU2029503; UTERU2029660, UTERU2029742, UTERU2029953, UTERU2030213, UTERU2030270, UTERU2030280, UTERU2031060, UTERU2031268, UTERU2031295, UTERU2031521, UTERU2031611, UTERU2031703, UTERU2031834, UTERU2031851, UTERU2032075, UTERU2032220, UTERU2032279, UTERU2032726, UTERU2033172, UTERU2033375, UTERU2033382, UTERU2033420, UTERU2033530, UTERU2033577, UTERU2034053, UTERU2034695, UTERU2035187, UTERU2035231, UTERU2035306, UTERU2035323, UTERU2035328, UTERU2035331, UTERU2035452, UTERU2035469, UTERU2035503, UTERU2035745, UTERU2035908, UTERU2035926, UTERU2035978, UTERU2036089, UTERU2036507, UTERU2036512, UTERU2036530, UTERU2036623, UTERU2036690, UTERU2037224,
UTERU2037361, UTERU2037423, UTERU2037577, UTERU2037674, UTERU2037791, UTERU2037843, UTERU2038171, UTERU2038251, UTERU3000226, UTERU3000402, UTERU3000665, UTERU3000670, UTERU3000727, UTERU3000828, UTERU3000844, UTERU3000899, UTERU3000959, UTERU3001029, UTERU3001053, UTERU3001059, UTERU3001158, UTERU3001240, UTERU3001394, UTERU3001558, UTERU3001571, UTERU3001585, UTERU3001632, UTERU3001652, UTERU3001766, UTERU3001988, UTERU3002209, UTERU3002383, UTERU3002600, UTERU3002620, UTERU3002667, UTERU3002701, UTERU3002731, UTERU3002768, UTERU3002993, UTERU3003116, UTERU3003178, UTERU3003465, UTERU3003495, UTERU3003523, UTERU3003774, UTERU3003776, UTERU3004384, UTERU3004418, UTERU3004477, UTERU3004523,
UTERU3004616, UTERU3004635, UTERU3004709, UTERU3004938, UTERU3004992, UTERU3005159, UTERU3005205, UTERU3005230, UTERU3005264, UTERU3005460, UTERU3005536, UTERU3005585, UTERU3005767, UTERU3005907, UTERU3005970, UTERU3006008, UTERU3006228, UTERU3006308, UTERU3006538, UTERU3006640, UTERU3006687, UTERU3006720, UTERU3006798, UTERU3006884, UTERU3007108, UTERU3007134, UTERU3007419, UTERU3007640, UTERU3007731, UTERU3007913, UTERU3008280, UTERU3008463, UTERU3008660, UTERU3008671, UTERU3008722, UTERU3008917, UTERU3009259, UTERU3009490, UTERU3009517, UTERU3009690, UTERU3009775, UTERU3009871, UTERU3009979, UTERU3009987, UTERU3010029, UTERU3010526, UTERU3010604, UTERU3010892, UTERU3010919, UTERU3011063,
UTERU3011092, UTERU3011273, UTERU3011398, UTERU3011558, UTERU3011795, UTERU3011837, UTERU3012293, UTERU3012414, UTERU3012476, UTERU3012599, UTERU3012999, UTERU3013167, UTERU3013781, UTERU3014274, UTERU3014446, UTERU3014611, UTERU3014647, UTERU3014791, UTERU3014906, UTERU3015011, UTERU3015086, UTERU3015299, UTERU3015412, UTERU3015500, UTERU3015647, UTERU3015844, UTERU3016070, UTERU3016273, UTERU3016274, UTERU3016308, UTERU3016789, UTERU3017176, UTERU3017441, UTERU3017626, UTERU3017995, UTERU3018154, UTERU3018255, UTERU3018616, UTERU3019078

Of the 17,176 genes that include the cDNAs of the present invention, genes whose expression levels were high enough to detect in each of the TUTER, UMVEN, and UTERU libraries were analyzed for expression frequency. The analysis results are described below. Each is a relative value, where the expression level over all tissues is taken as 100. The larger this value, the greater the level of expression.

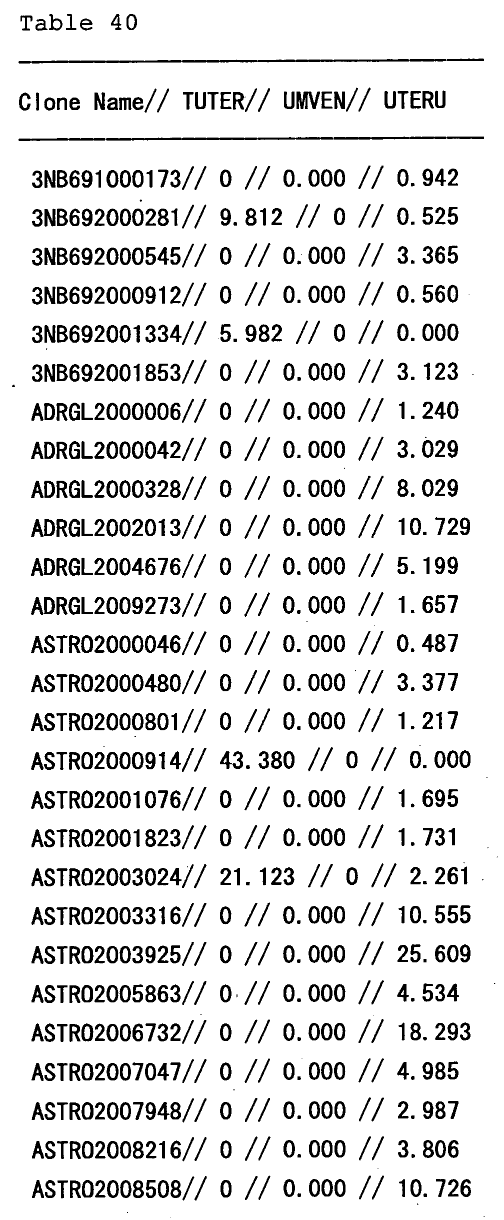

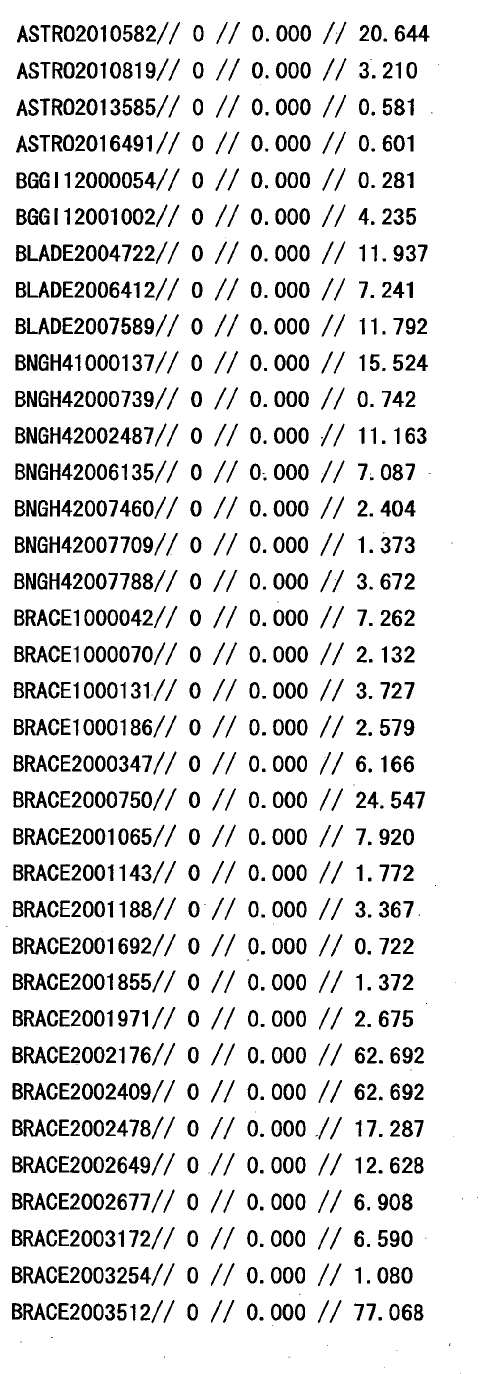

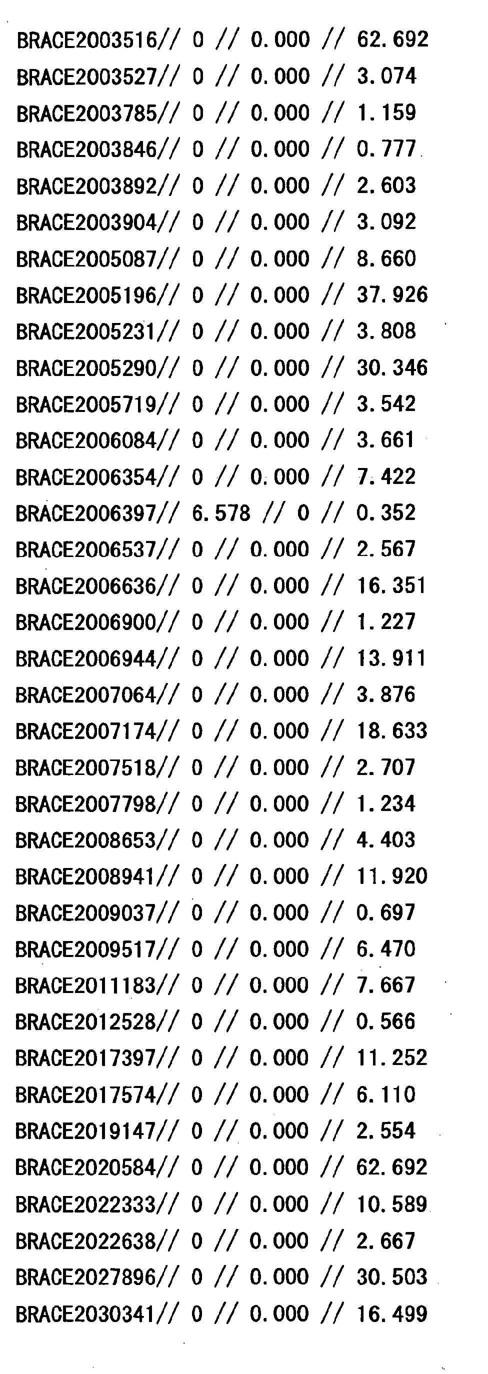

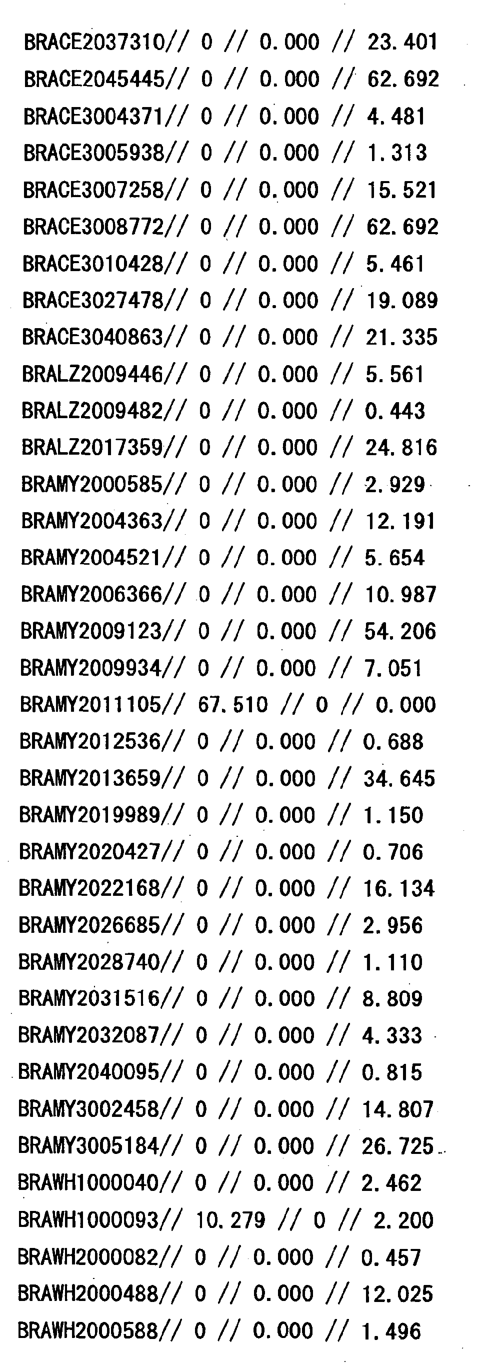

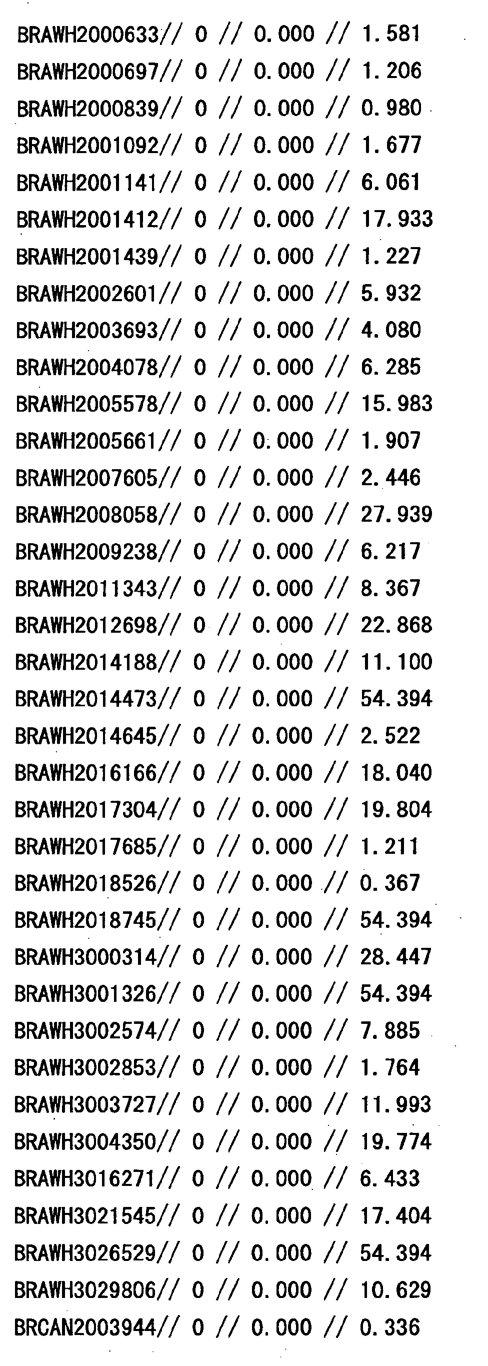

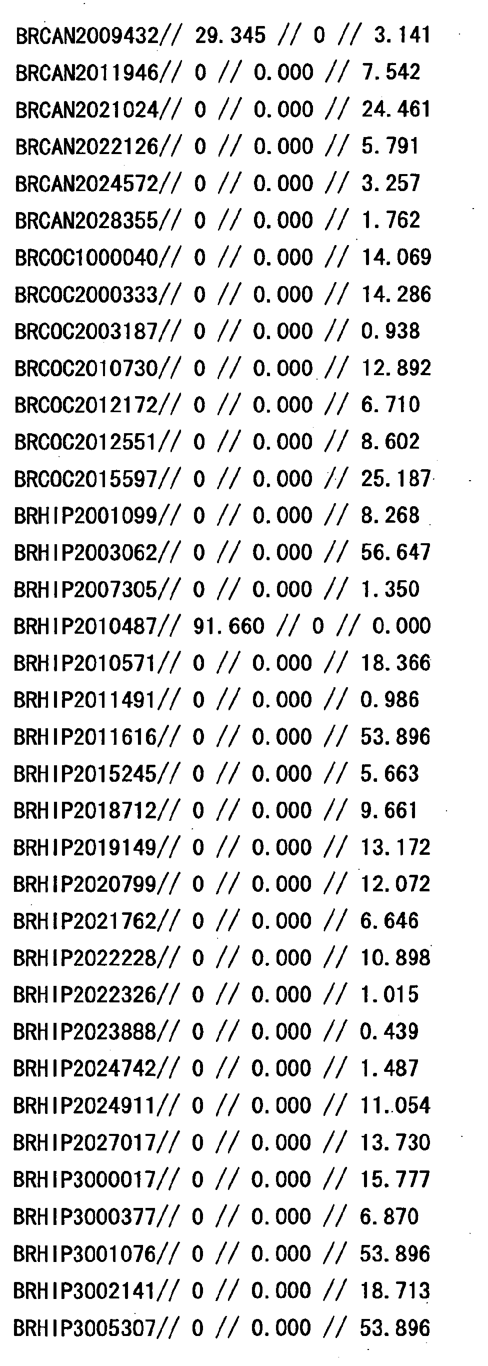

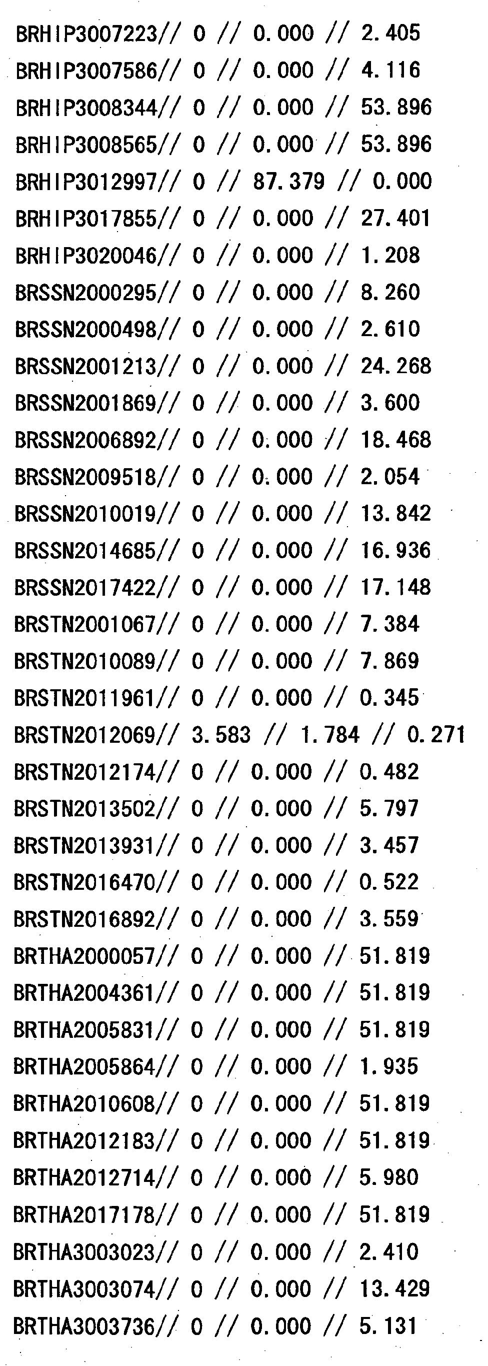

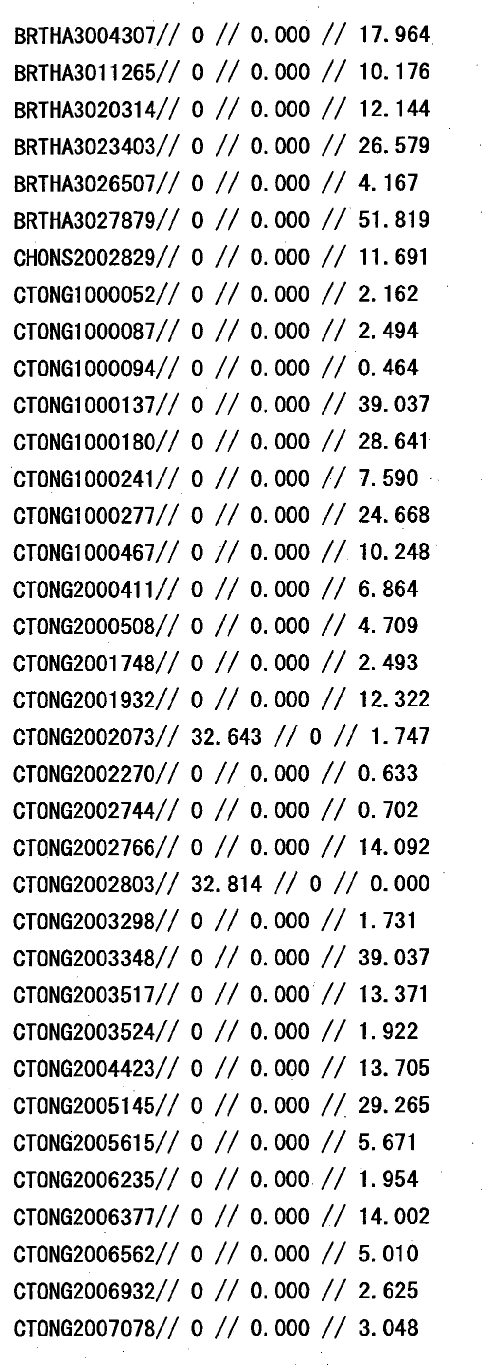

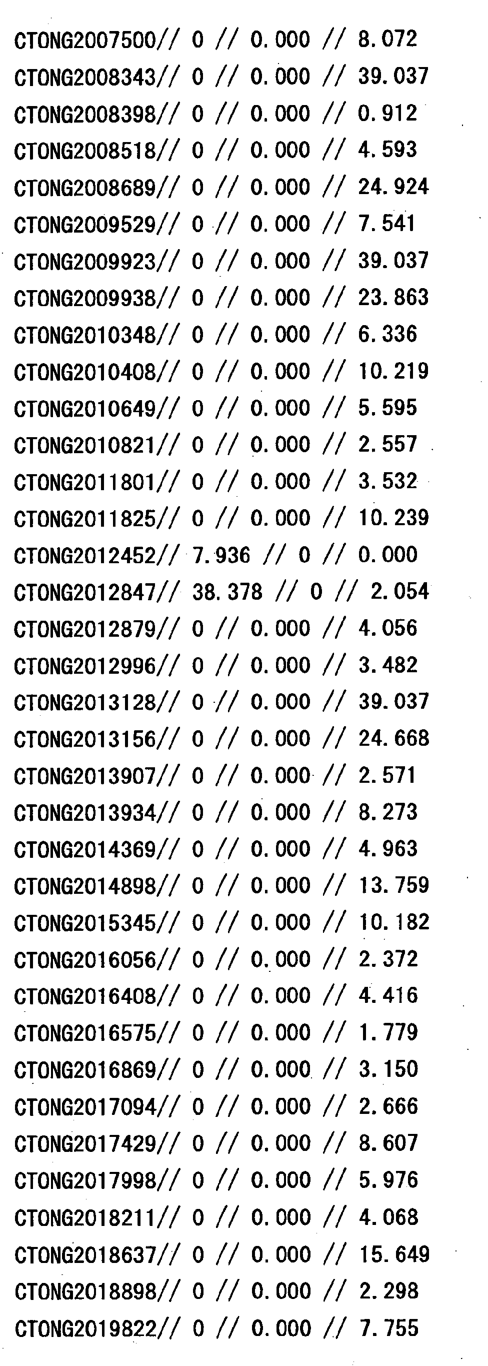

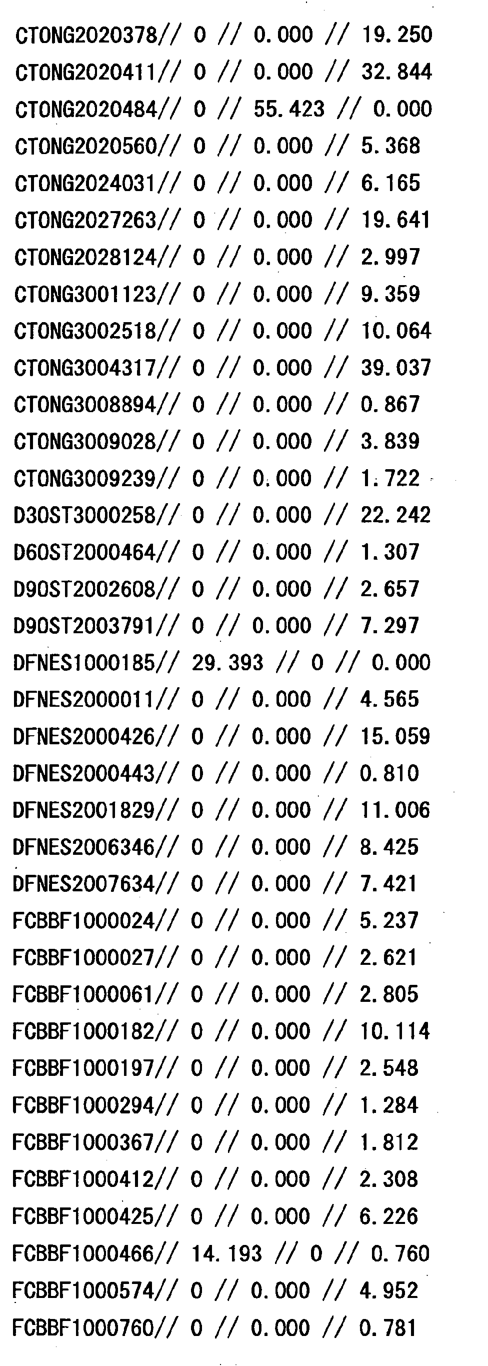

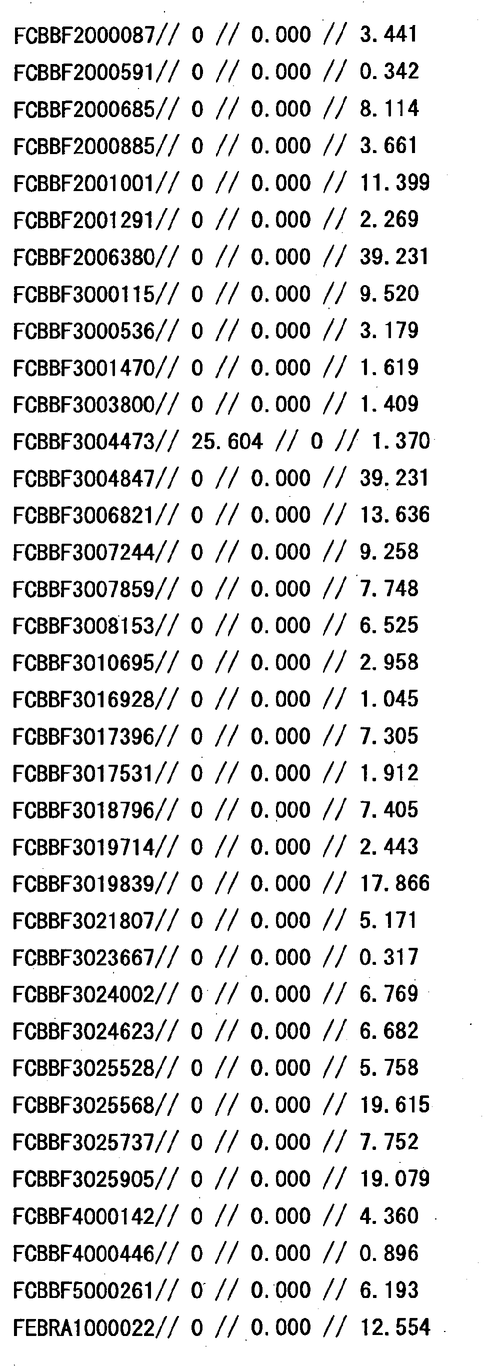

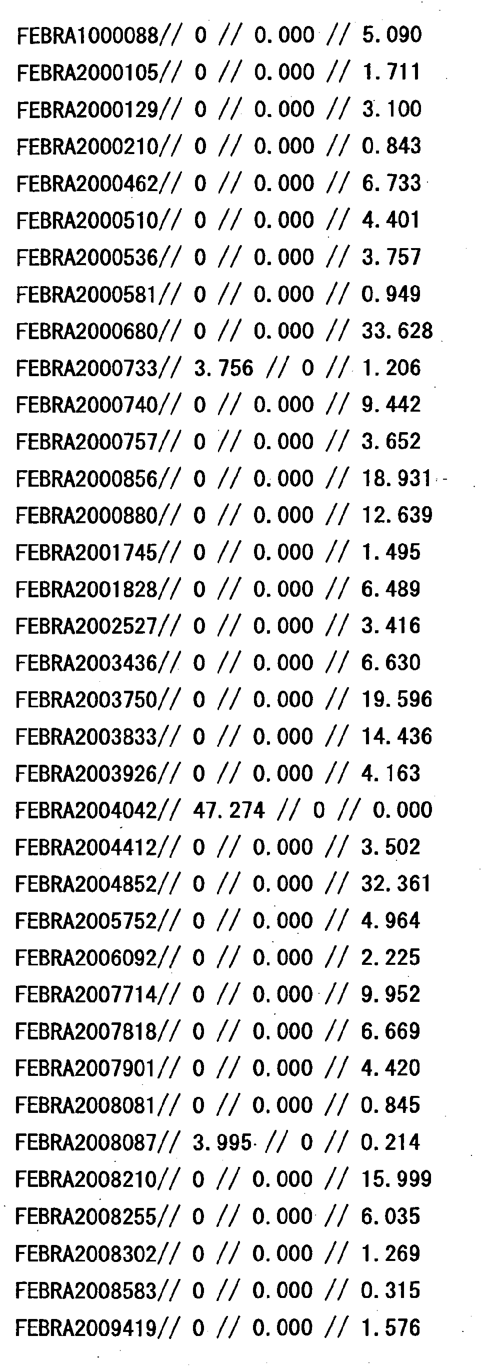

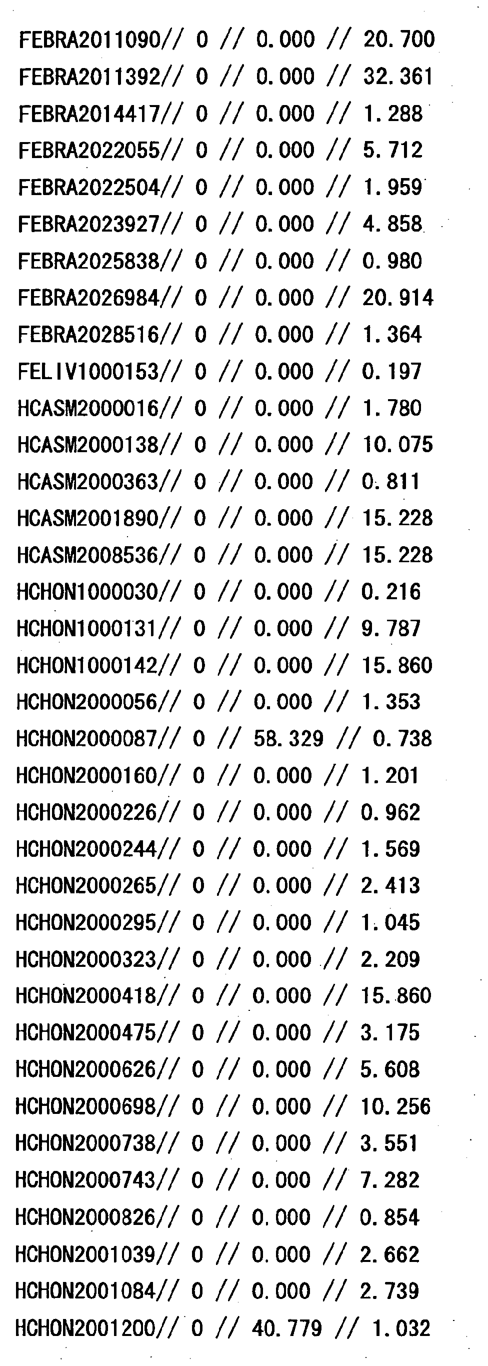

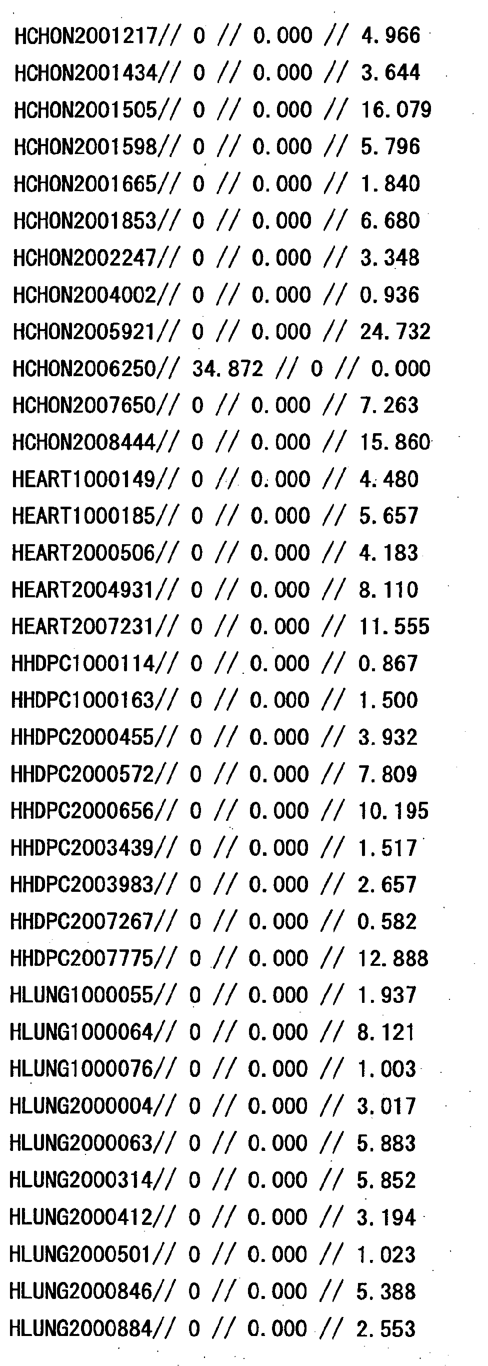

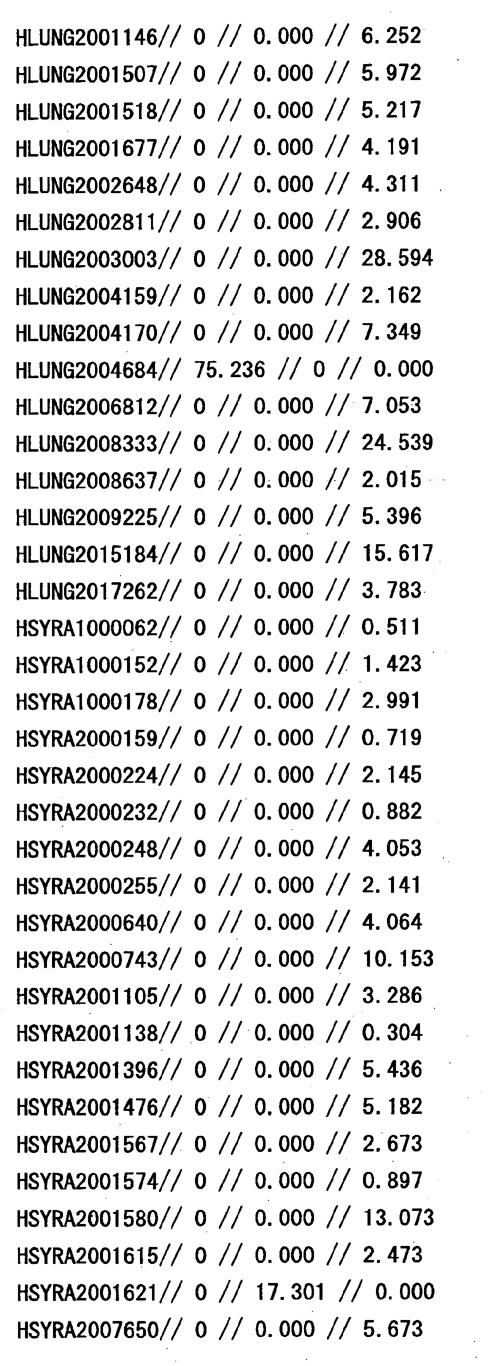

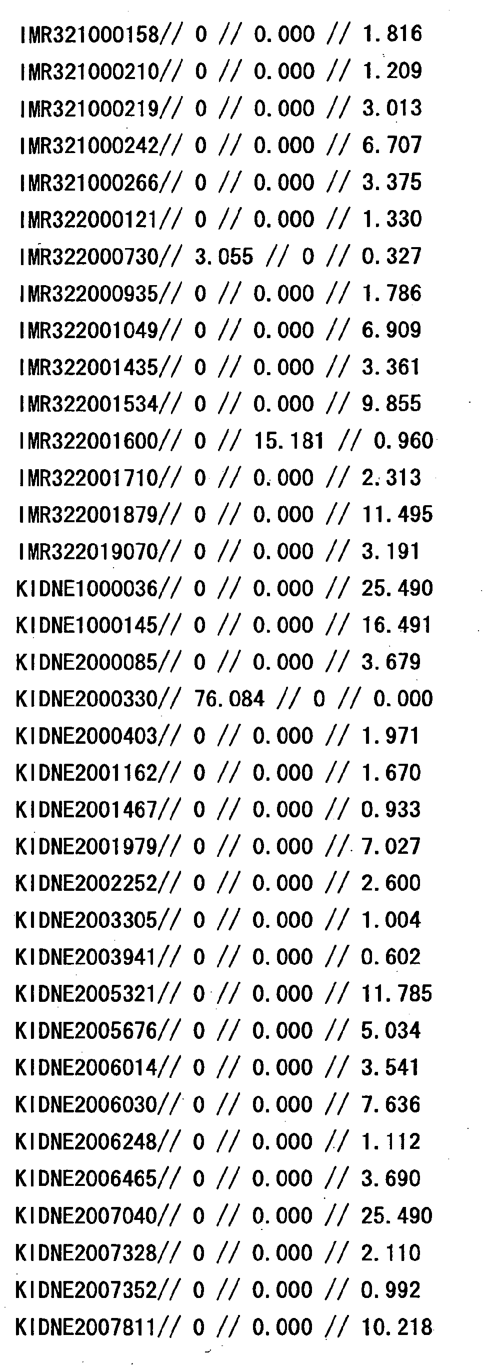

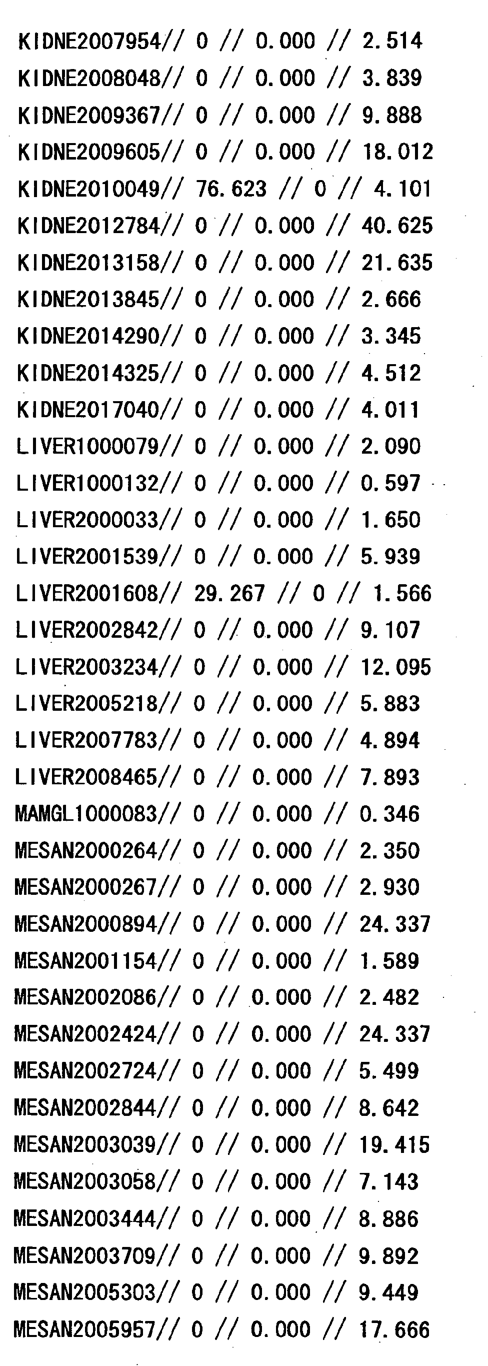

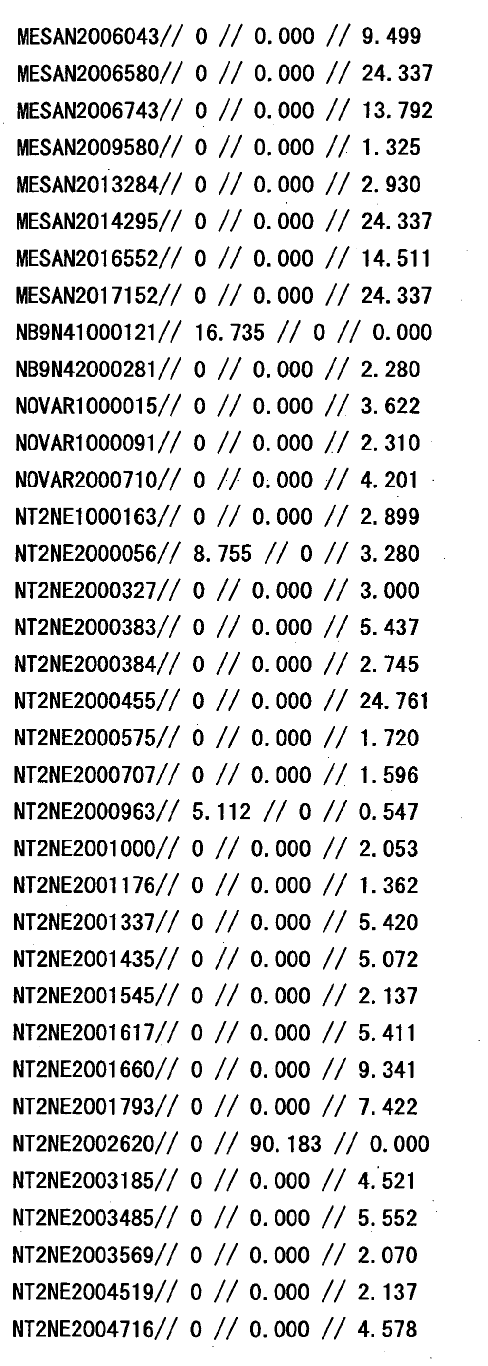

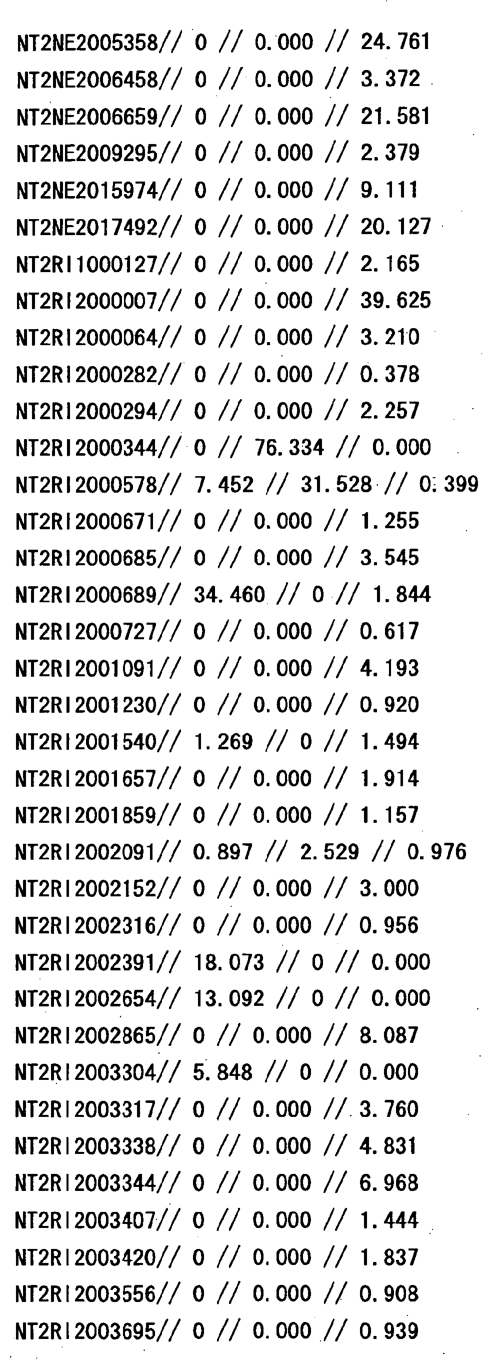

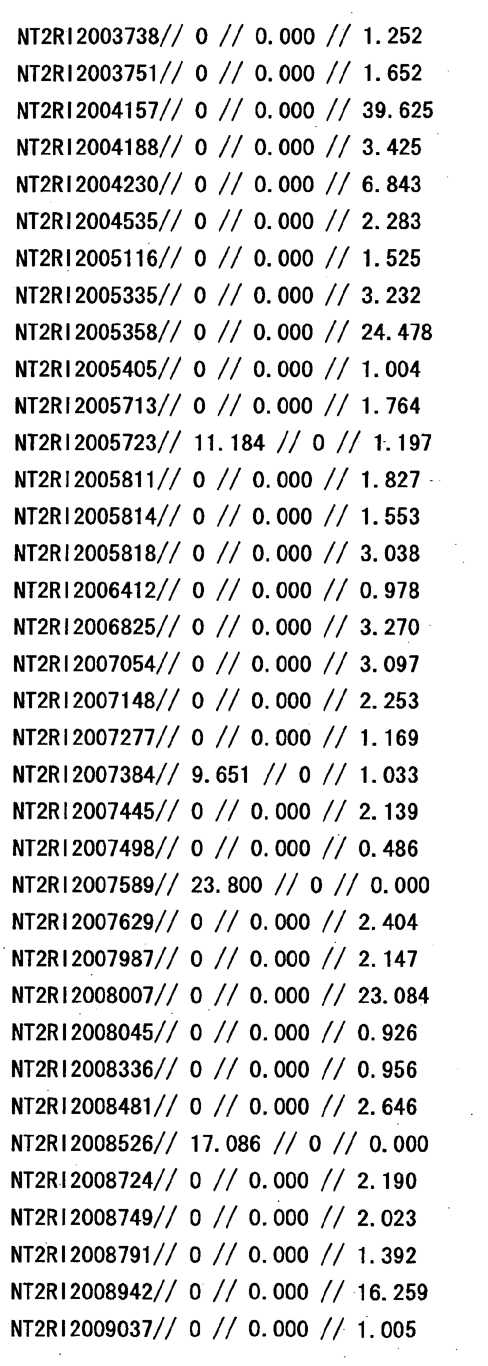

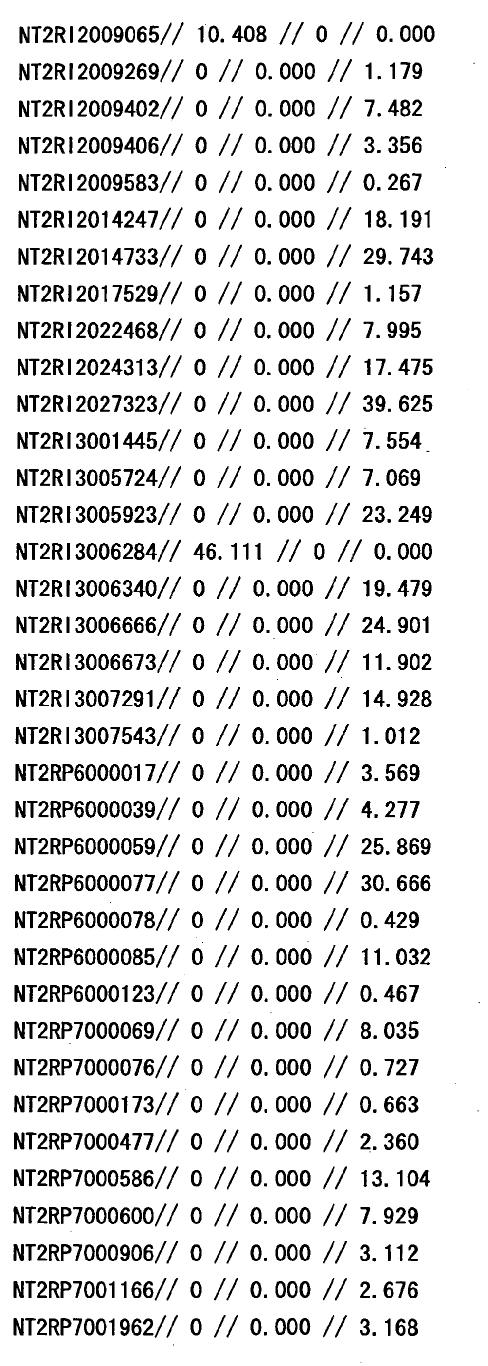

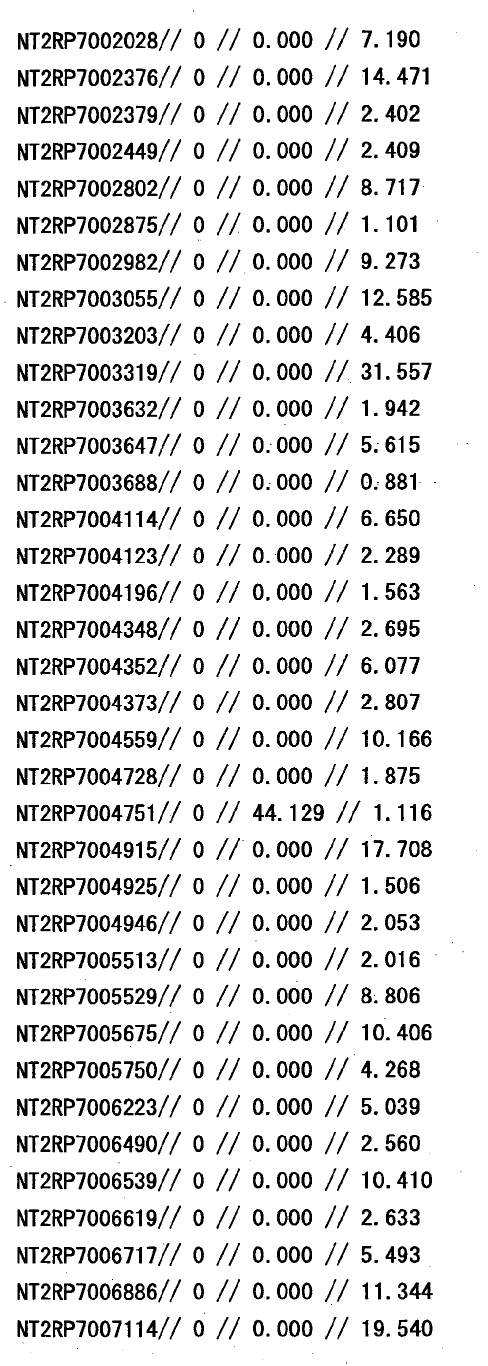

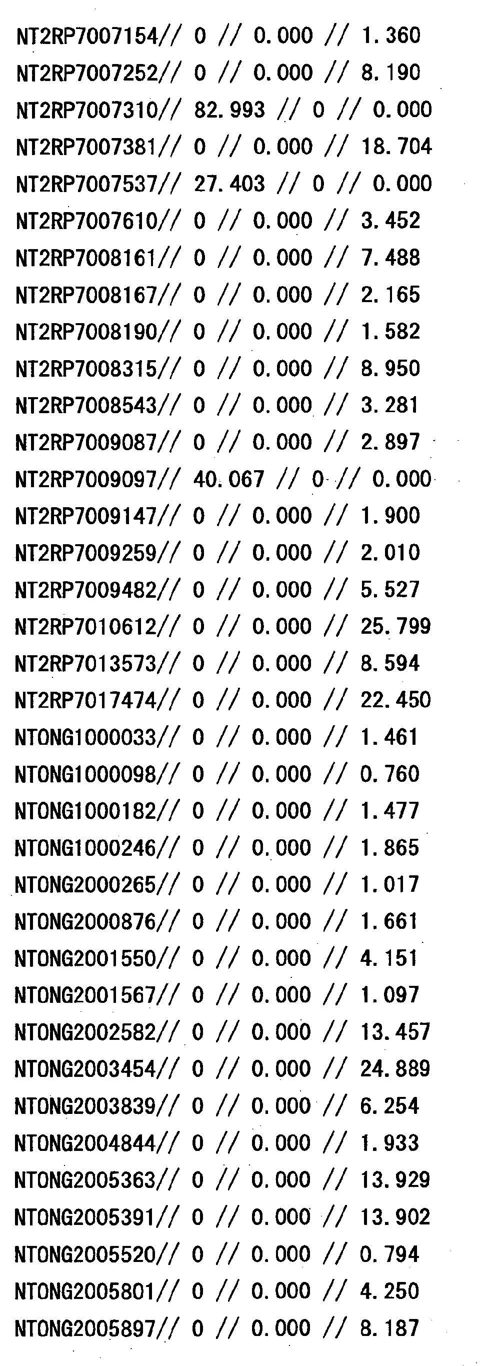

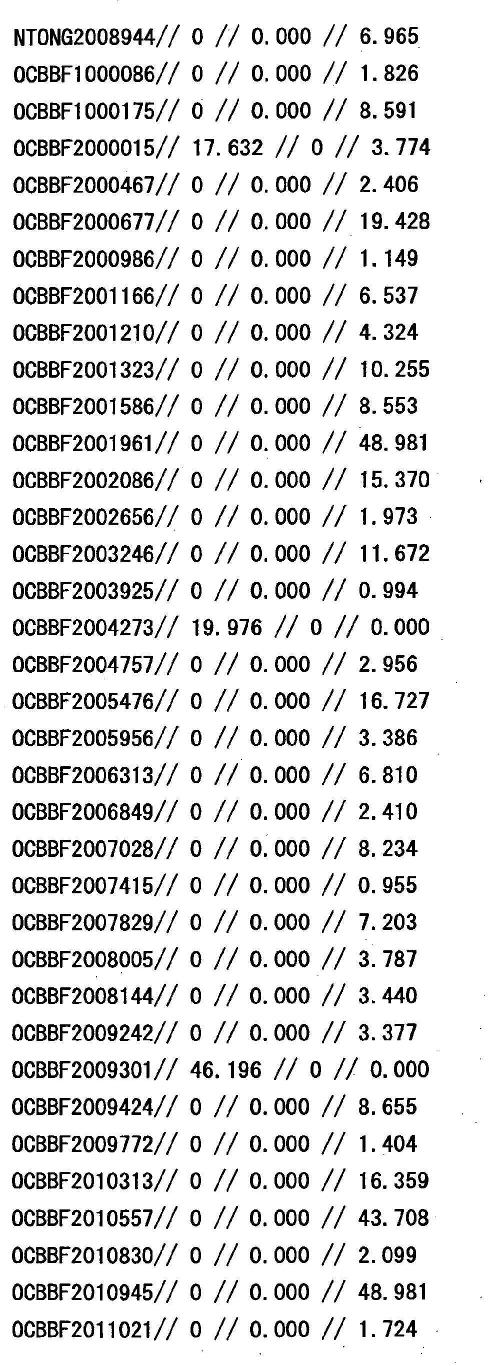

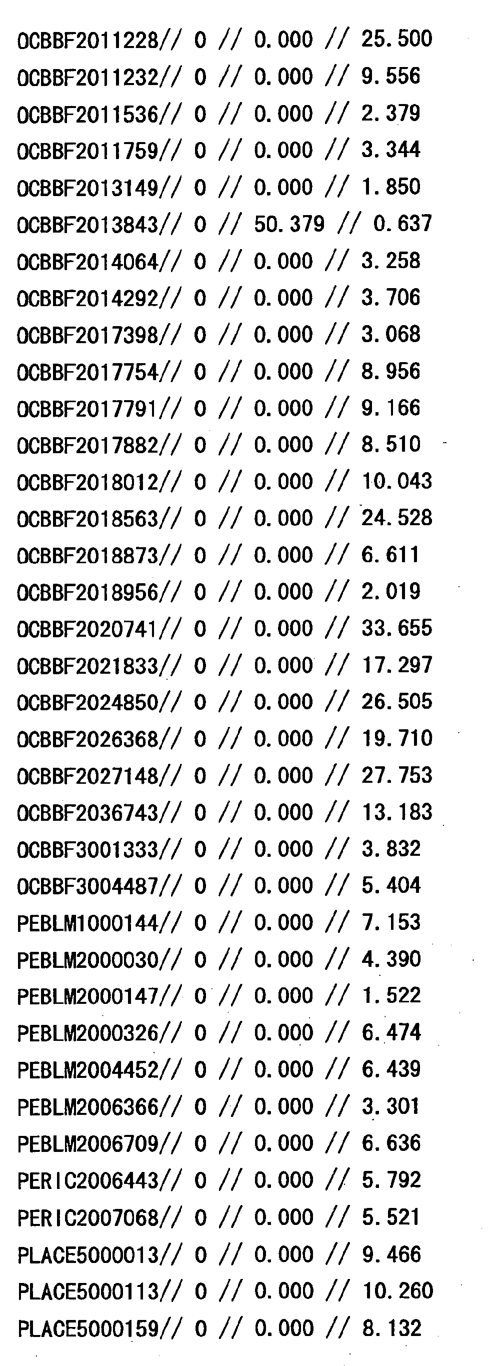

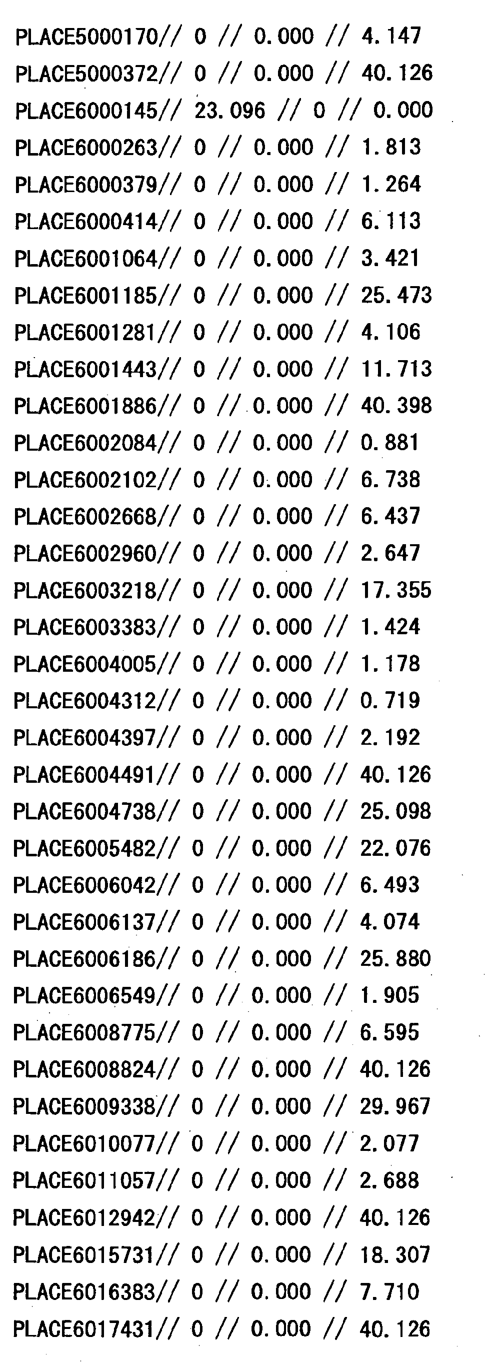

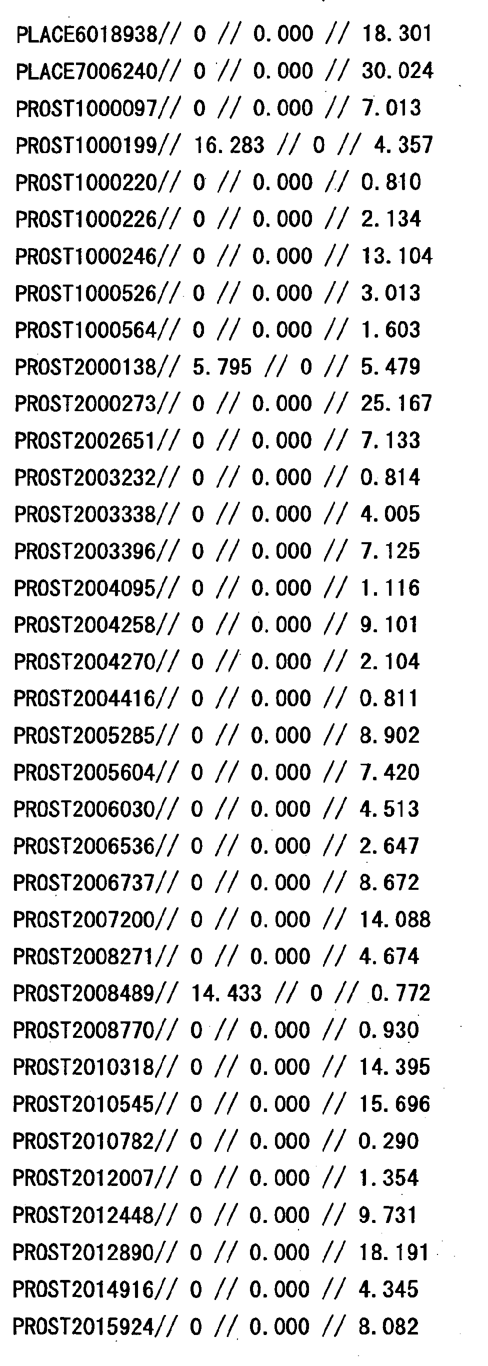

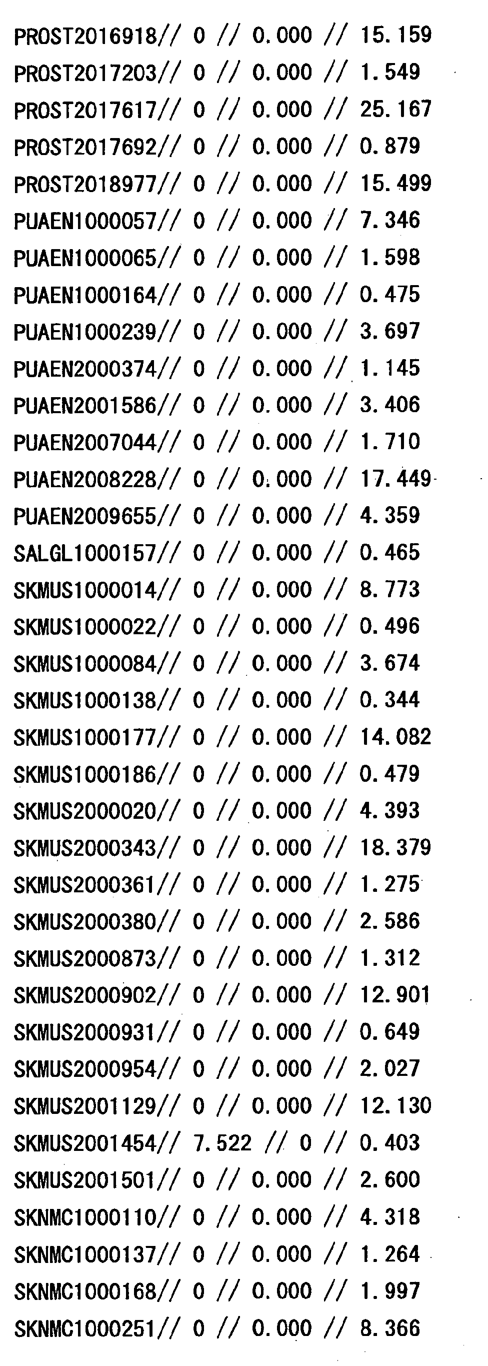

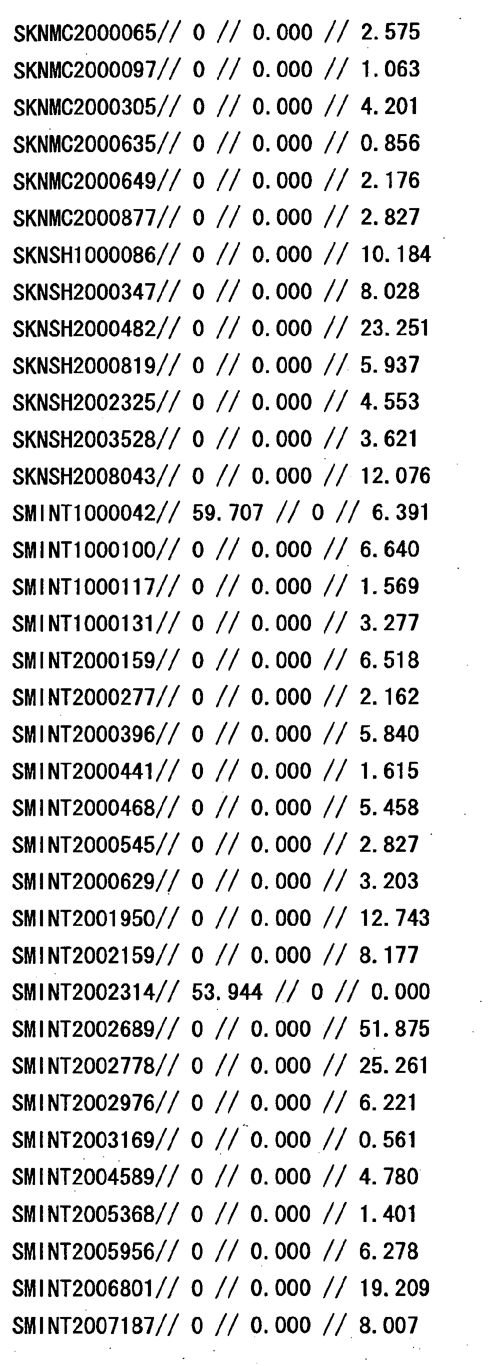

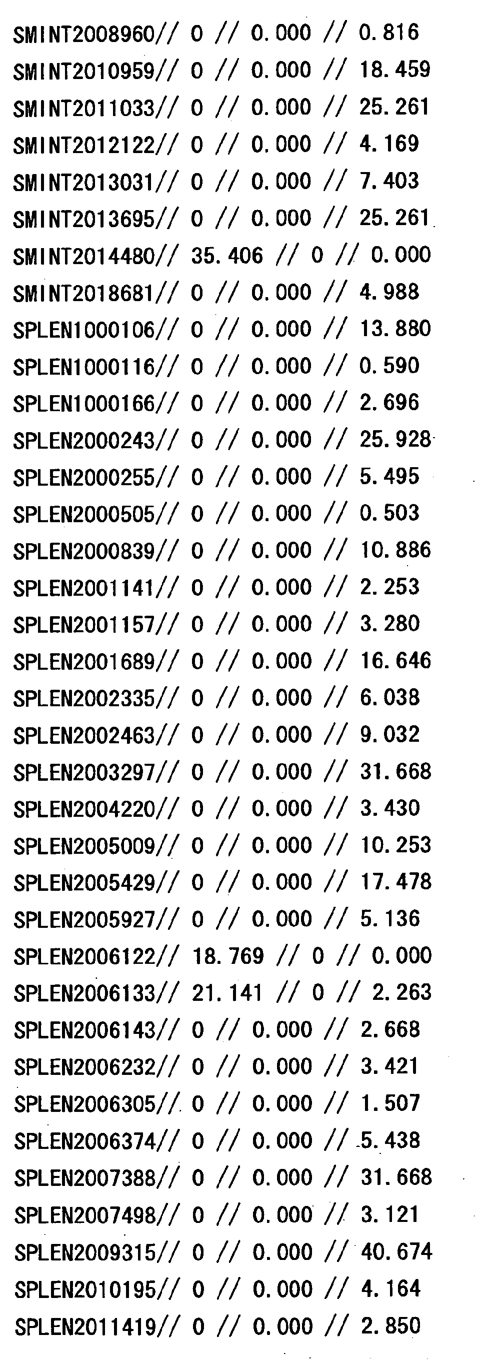

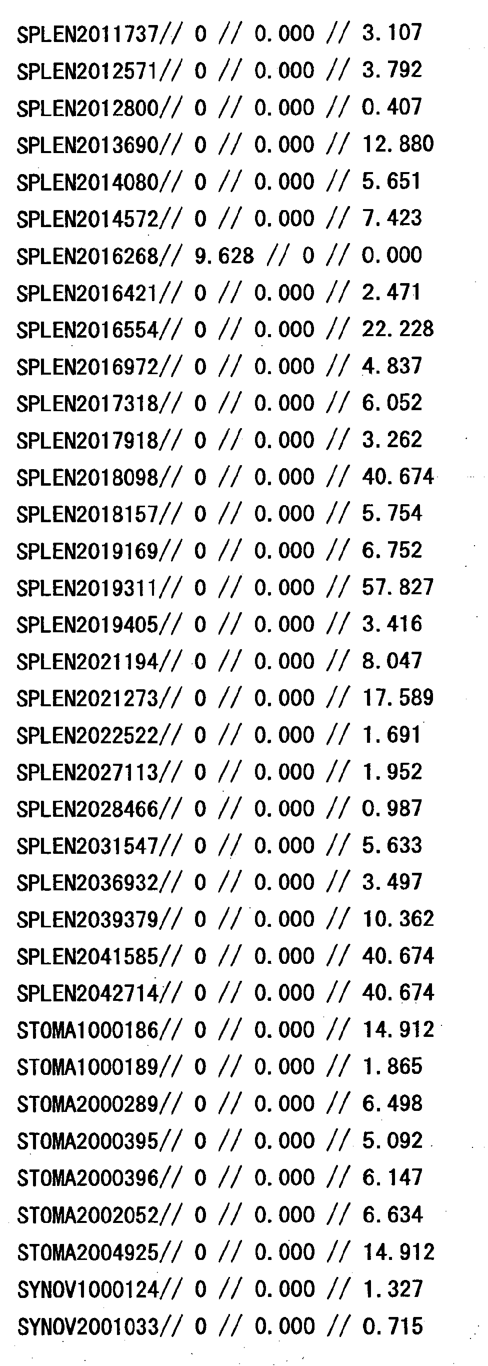

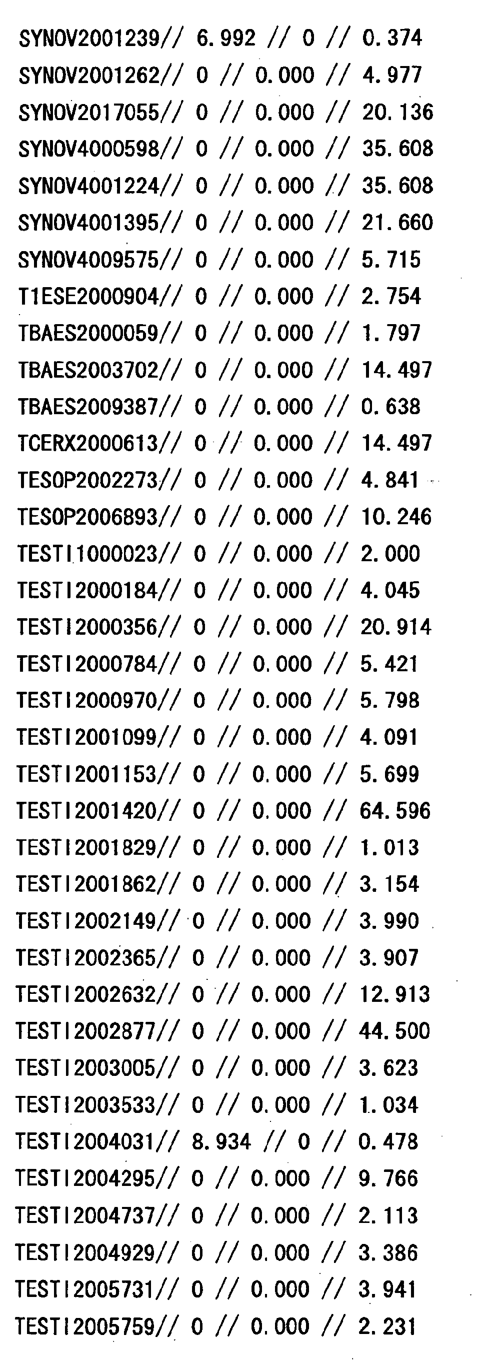

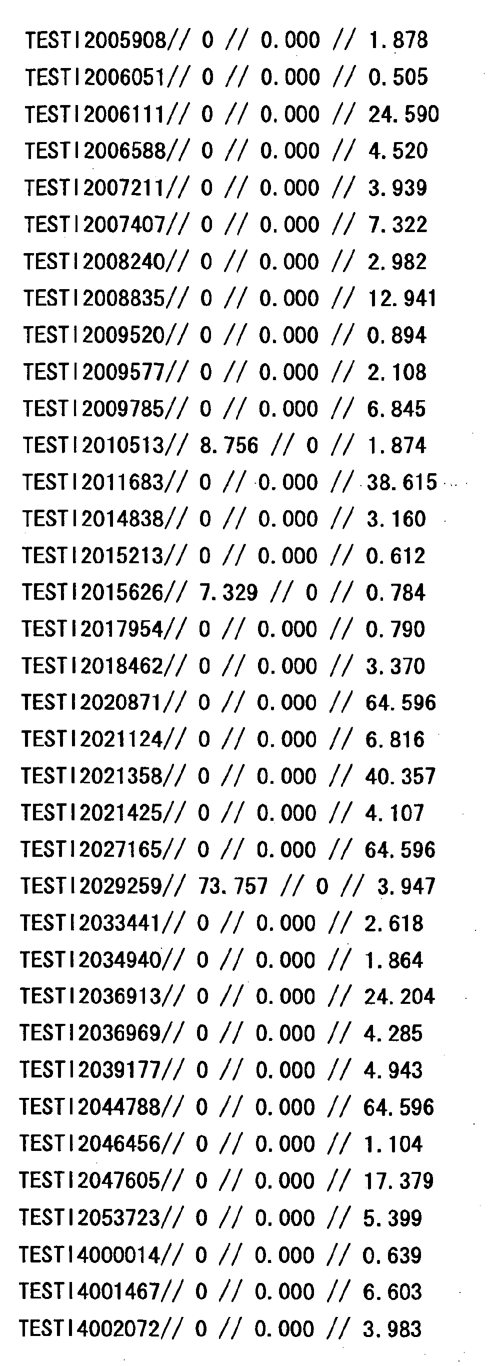

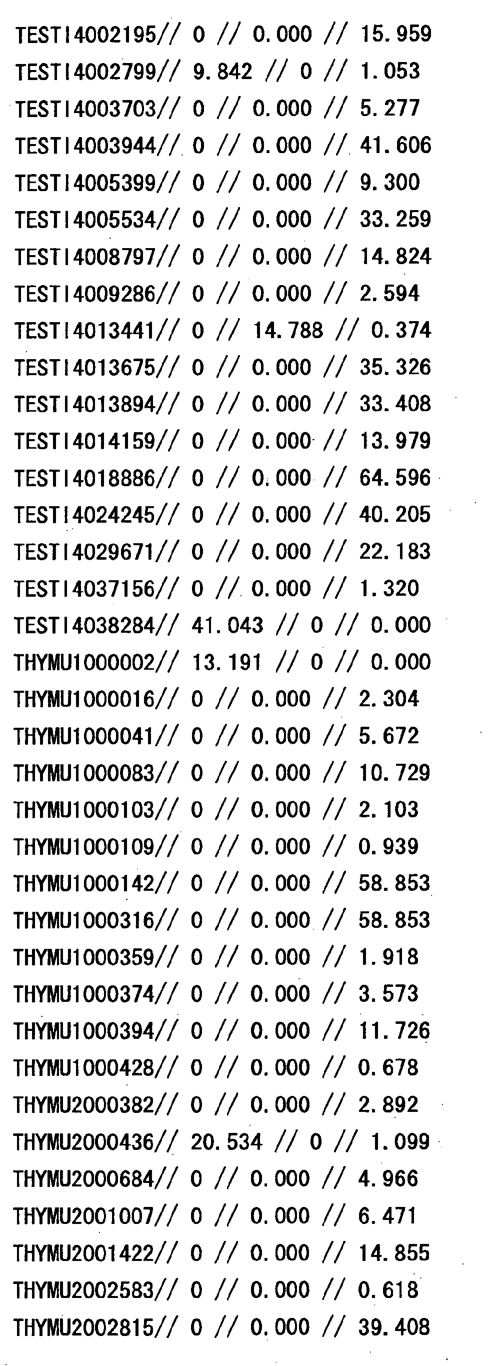

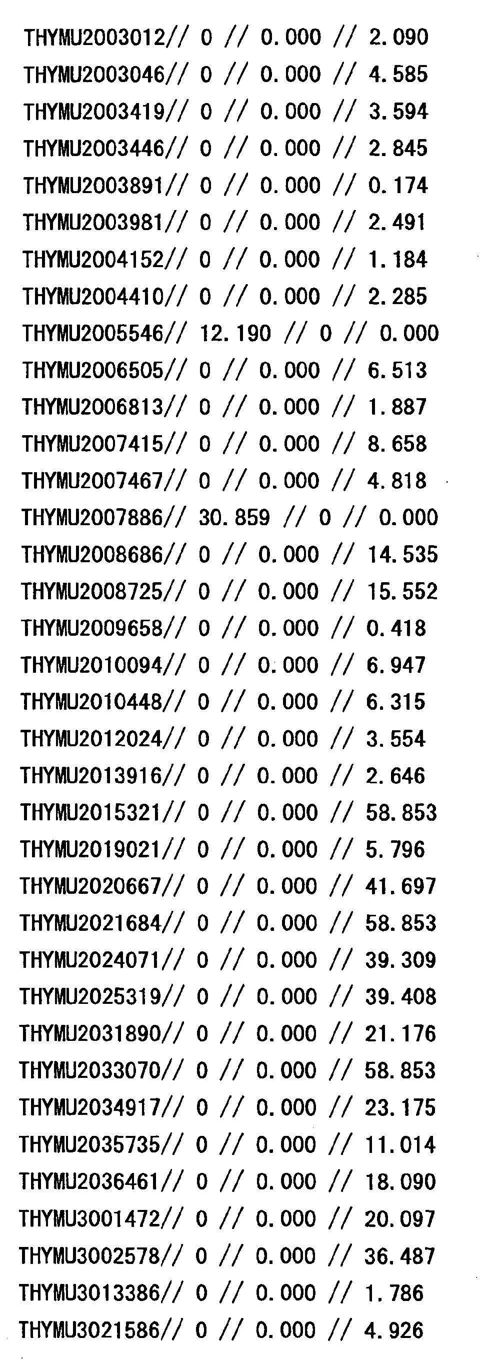

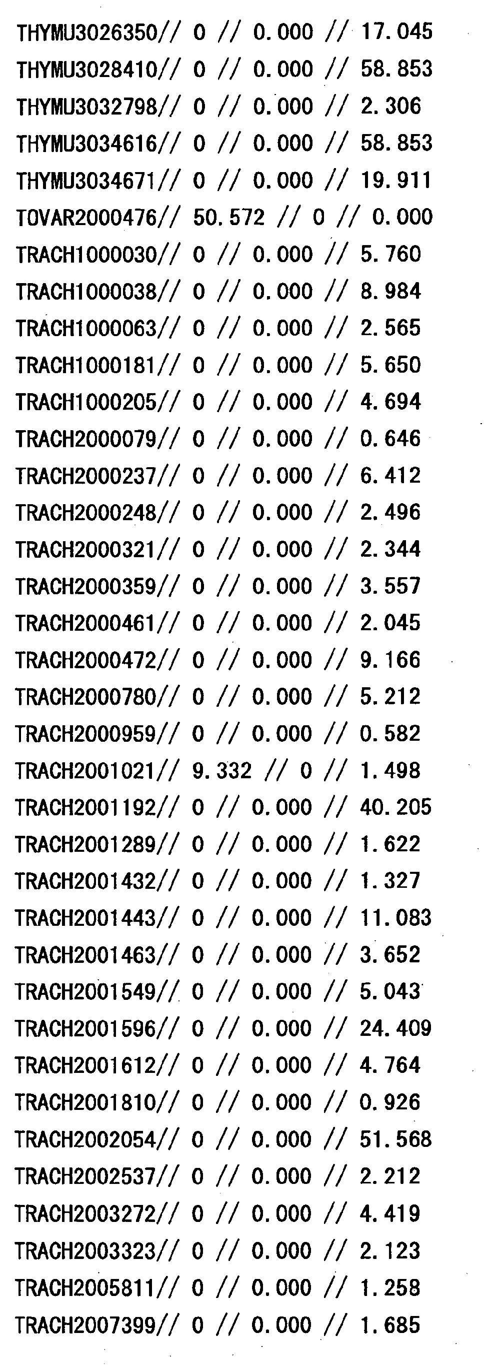

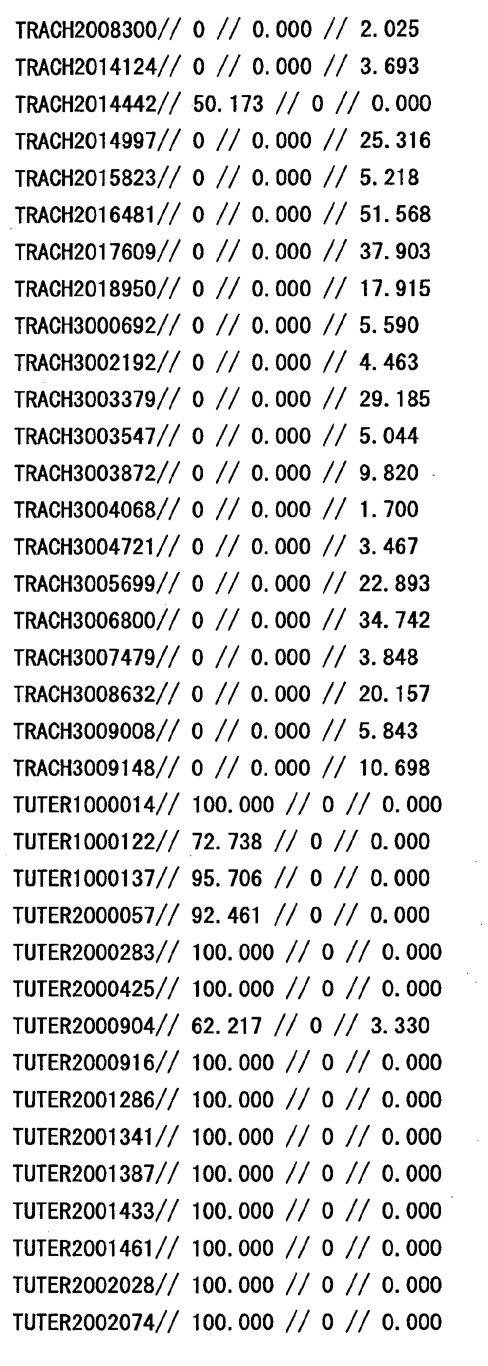

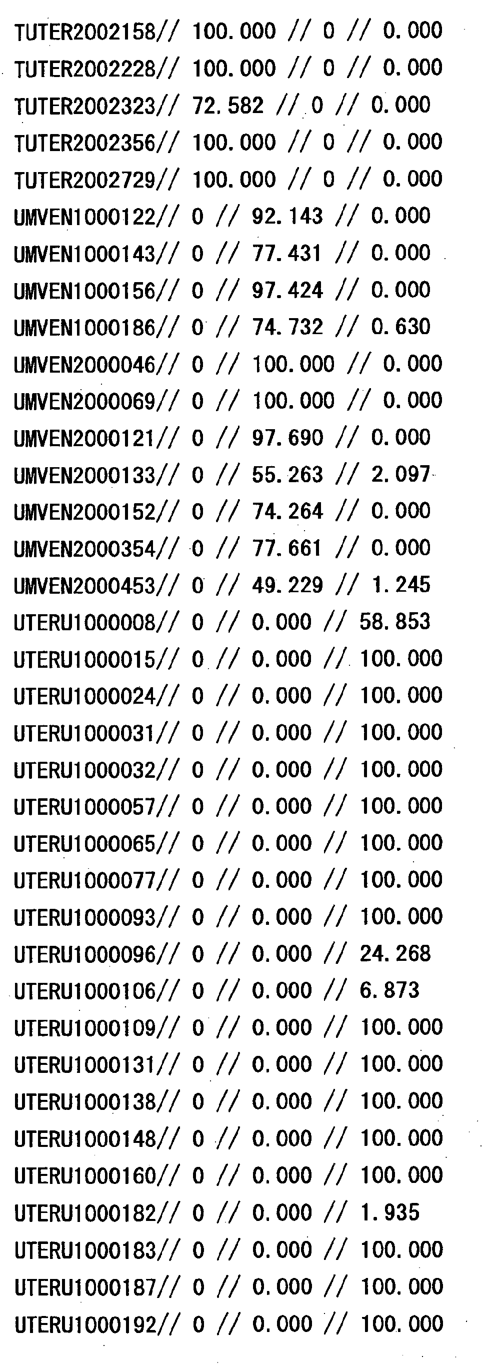

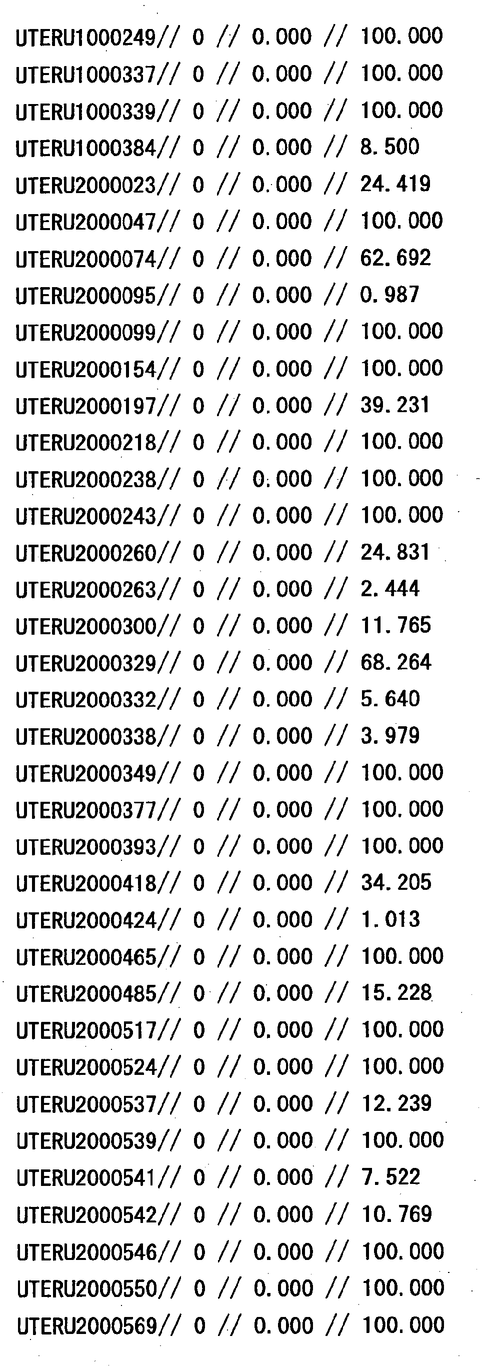

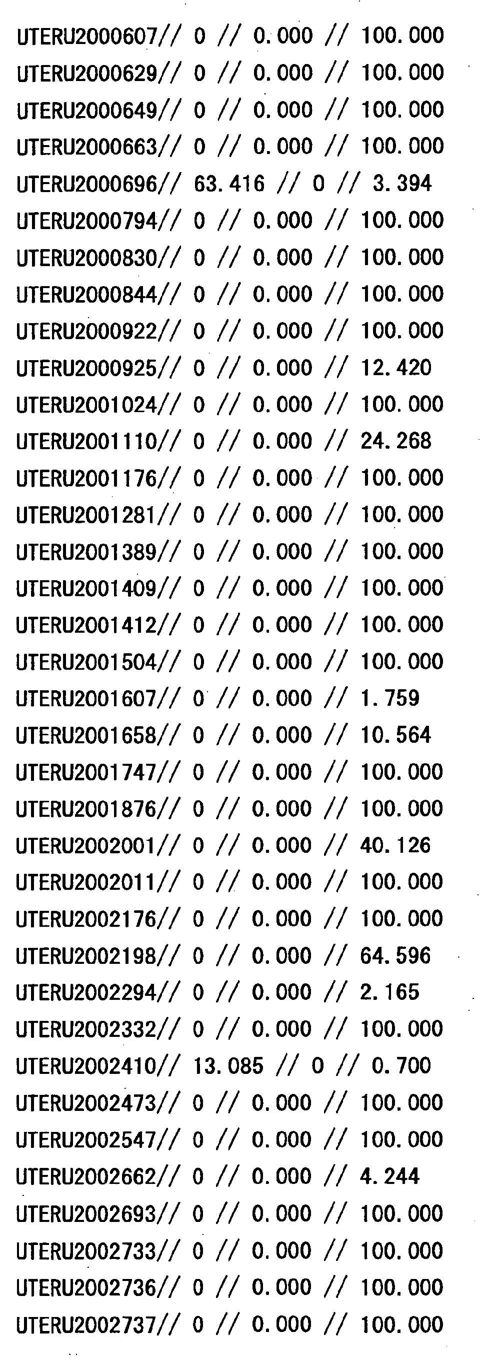

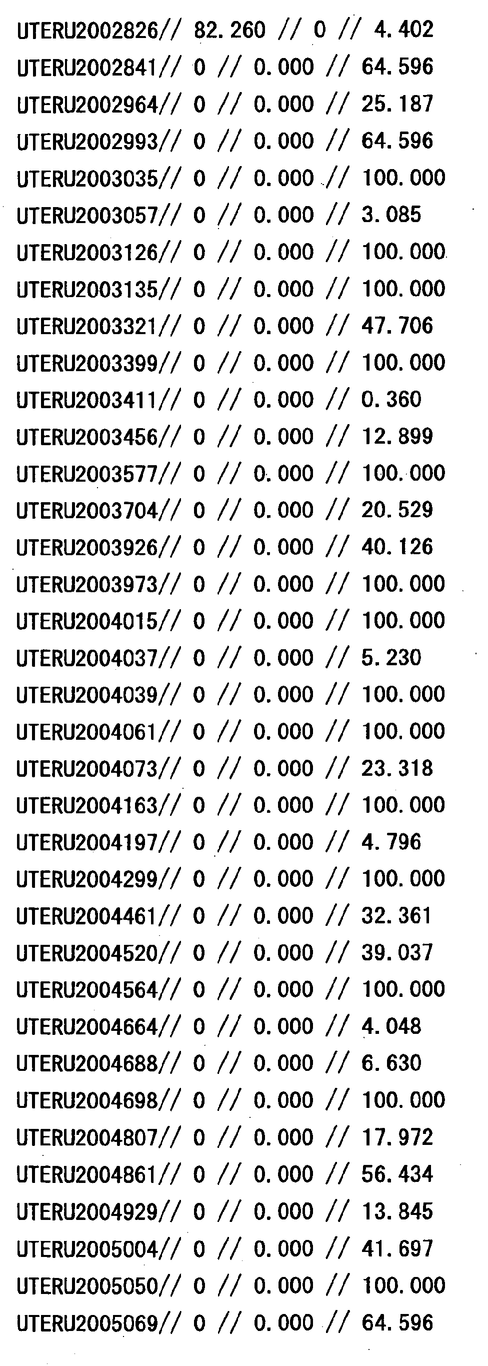

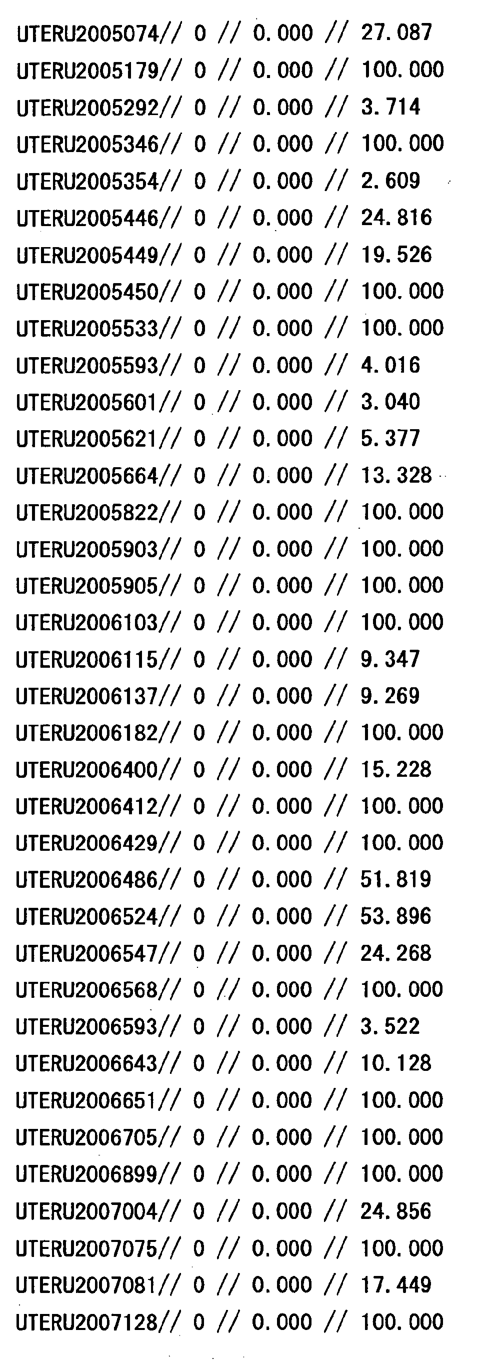

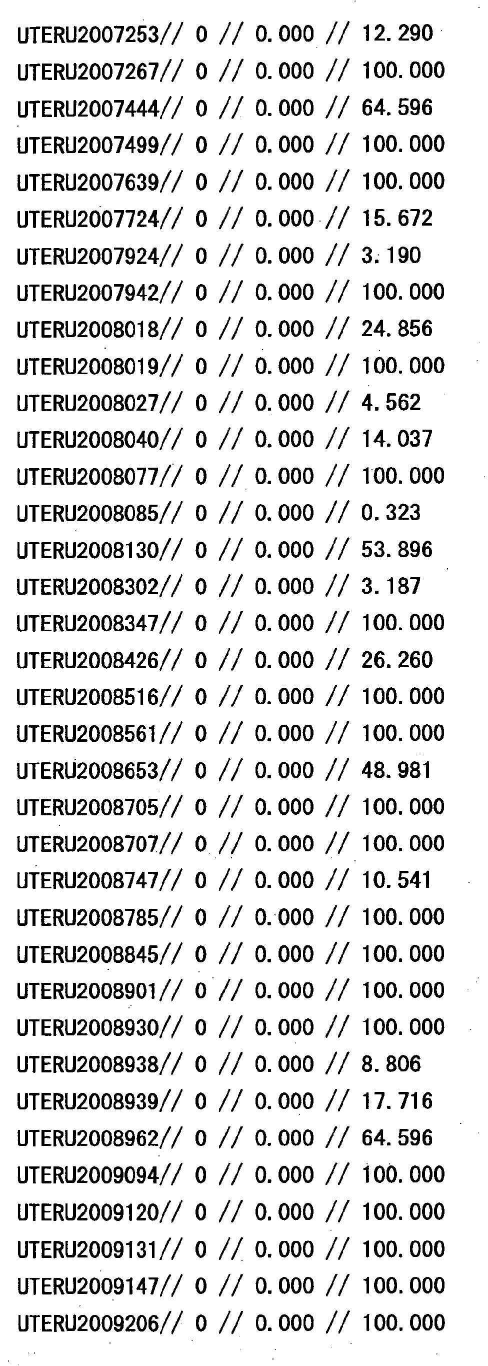

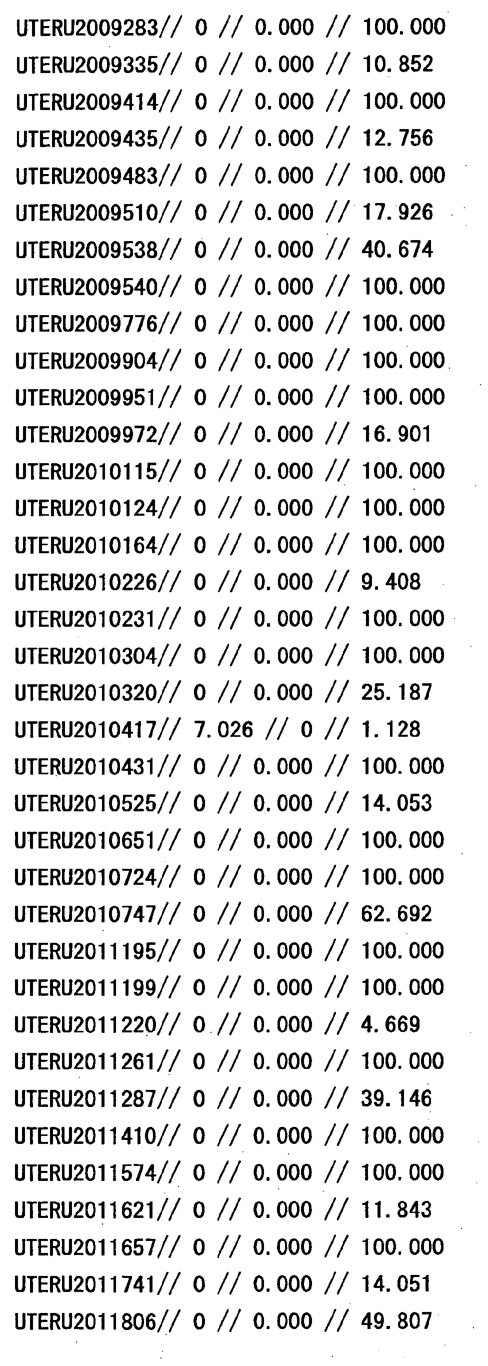

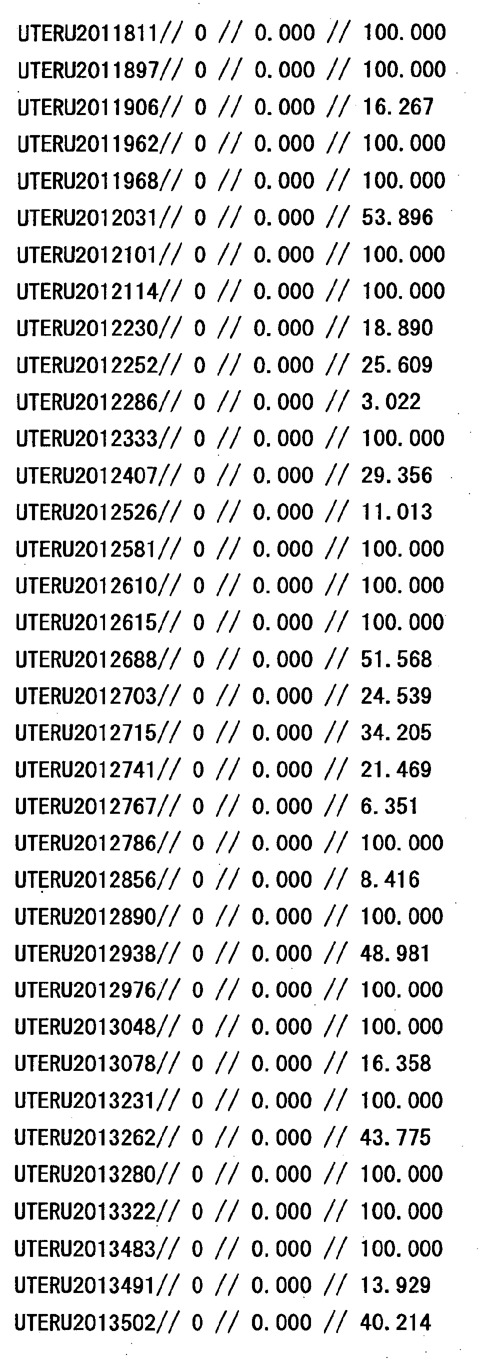

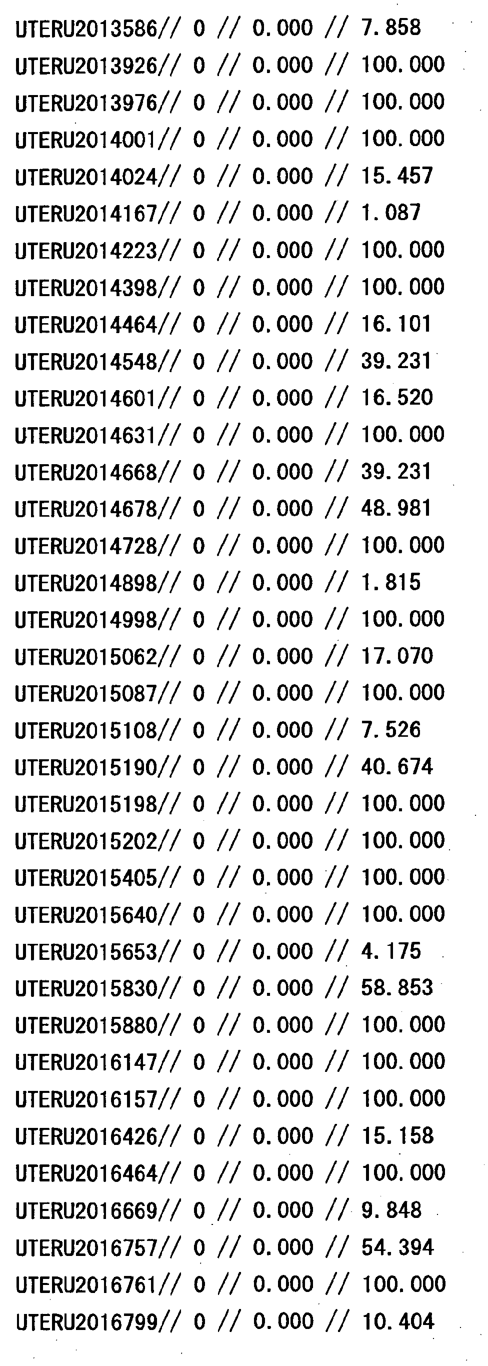

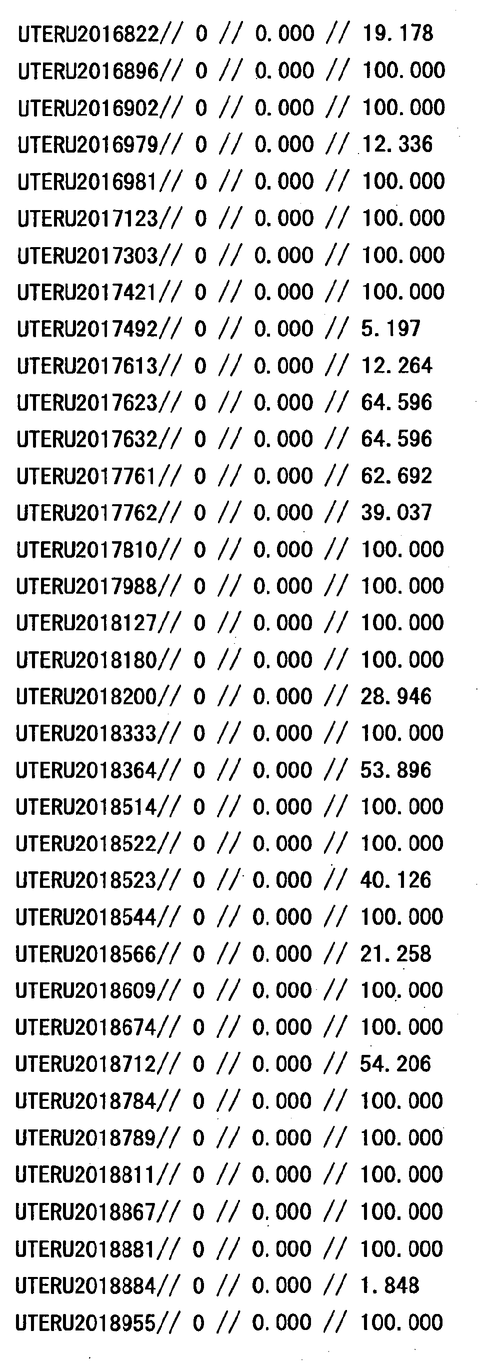

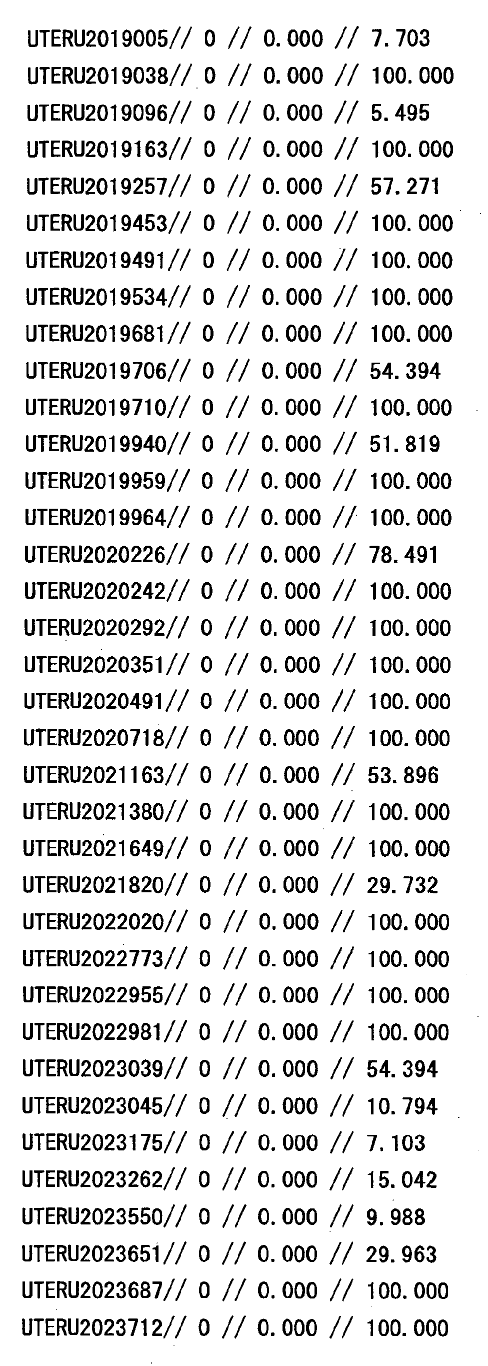

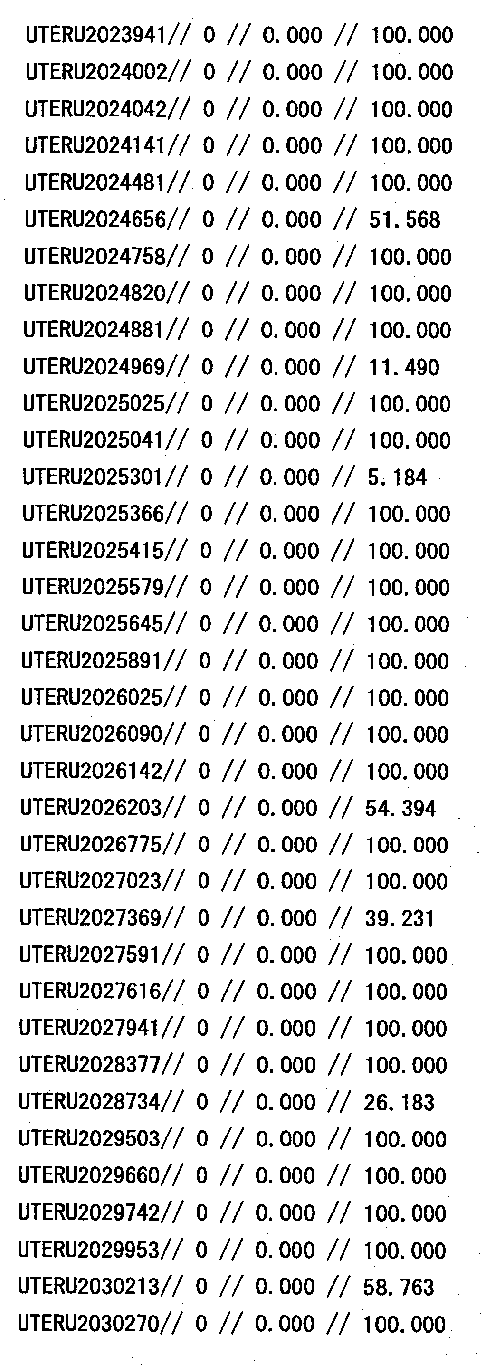

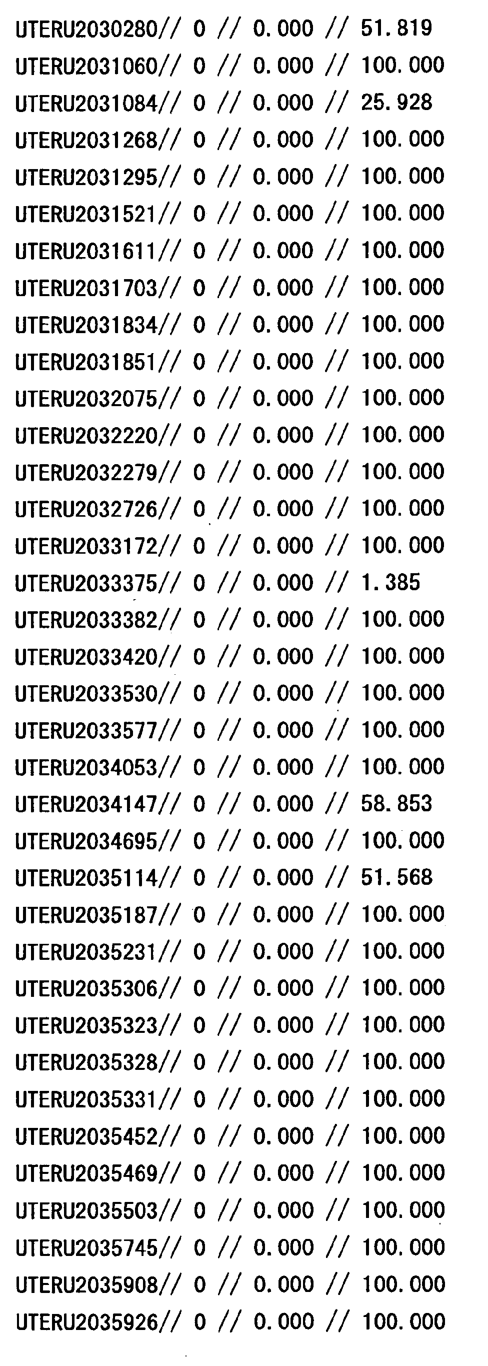

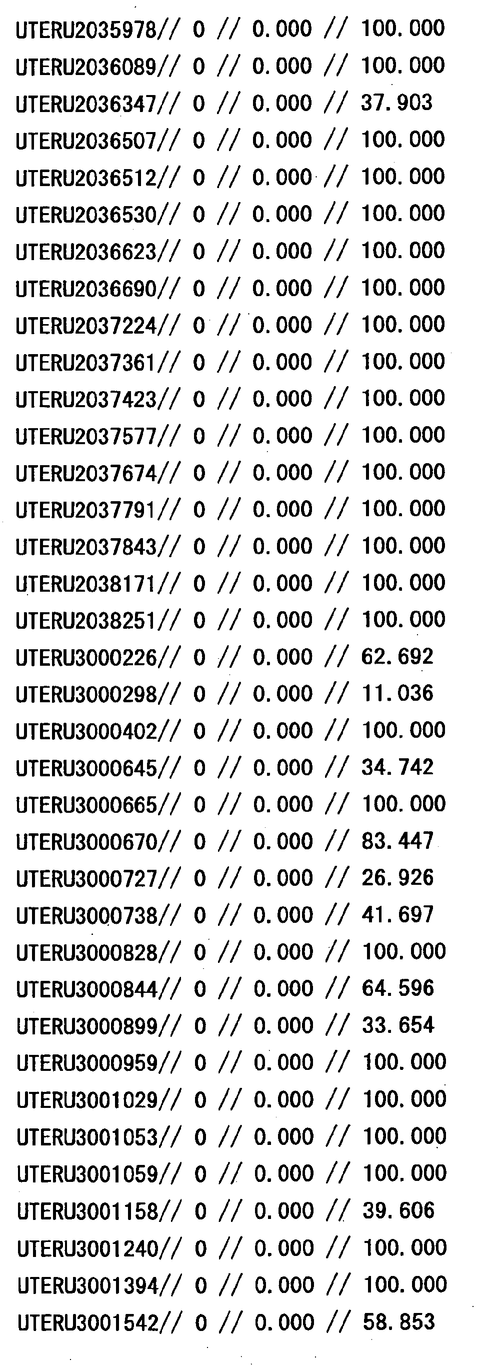

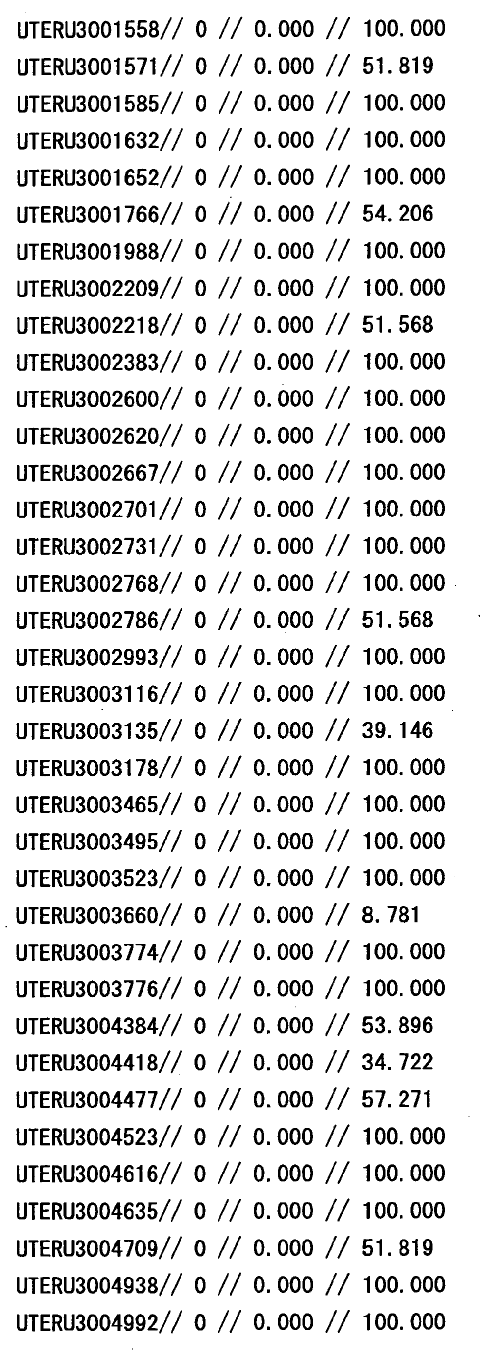

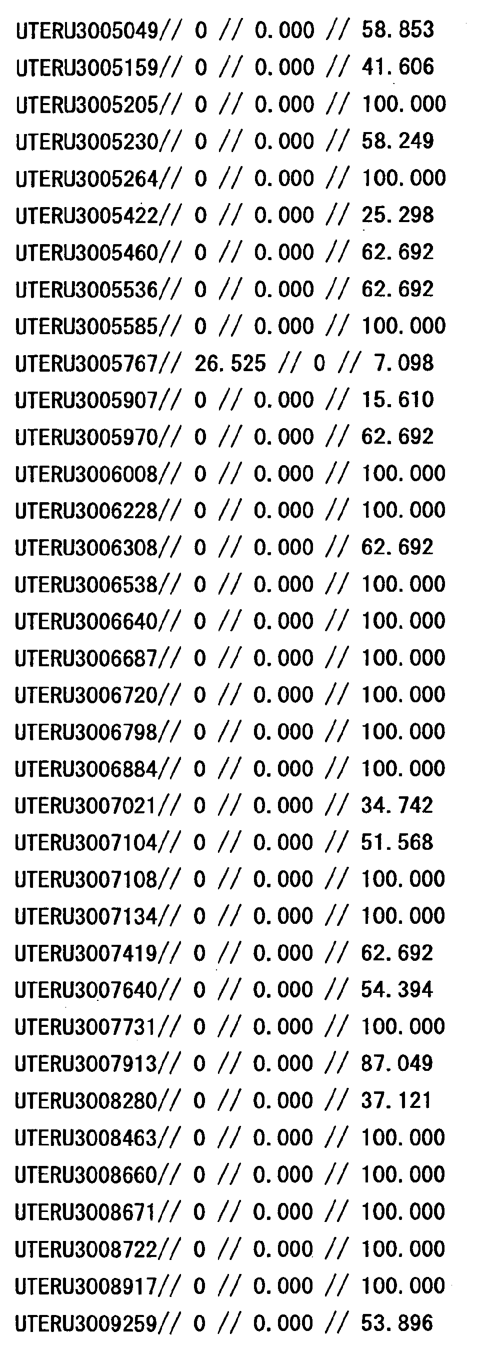

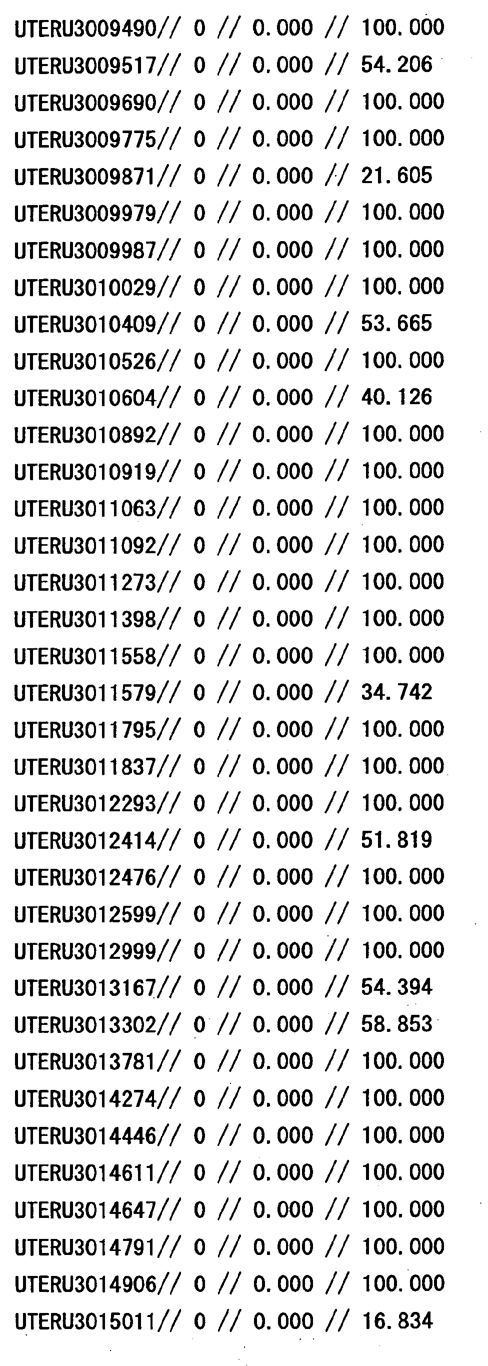

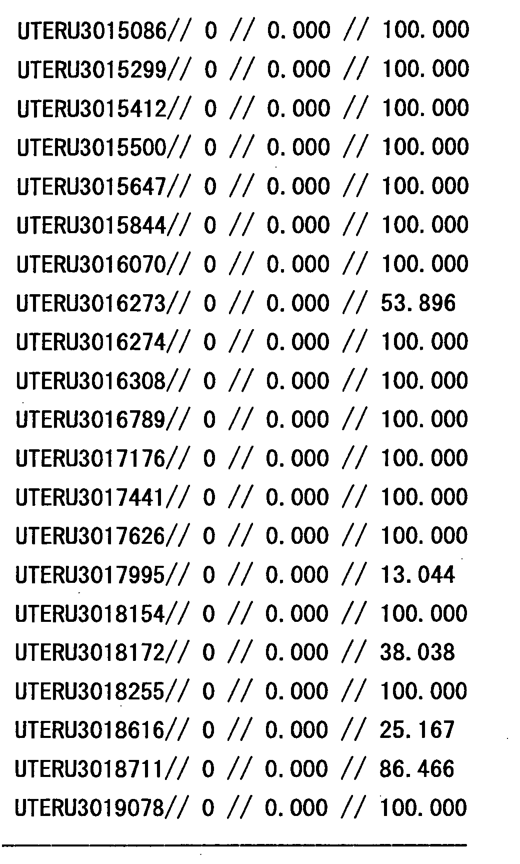

Genes whose expression levels were negligibly low in each of the above-described libraries are shown with their clone names below.
3NB691000018, 3NB691000085, 3NB691000113, 3NB691000116, 3NB691000129, 3NB691000191, 3NB692000012, 3NB692000029, 3NB692000102, 3NB692000154, 3NB692000276, 3NB692000305, 3NB692000330, 3NB692000374, 3NB692000429, 3NB692000484, 3NB692000529, 3NB692000973, 3NB692001002, 3NB692001022, 3NB692001034, 3NB692001040, 3NB692001123, 3NB692001267, 3NB692001288, 3NB692001339, 3NB692001349, 3NB692001366, 3NB692001408, 3NB692001433, 3NB692001442, 3NB692001459, 3NB692001471, 3NB692001496, 3NB692001501, 3NB692001507, 3NB692001511, 3NB692001519, 3NB692001520, 3NB692001528, 3NB692001538, 3NB692001548, 3NB692001557, 3NB692001637, 3NB692001719, 3NB692001861, 3NB692002051, 3NB692002365, 3NB692002685, 3NB692002806,
3NB692003538, 3NB692004045, 3NB692004724, 3NB692005439, 3NB692006952, 3NB692008178, 3NB692008729, ACTVT2000380, ADIPS1000064, ADIPS2000069, ADIPS2000088, ADIPS2000245, ADIPS2000425, ADRGL1000002, ADRGL1000018, ADRGL1000033, ADRGL1000038, ADRGL1000065, ADRGL1000067, ADRGL1000111, ADRGL1000144, ADRGL1000147, ADRGL1000160, ADRGL1000165, ADRGL1000182, ADRGL2000056, ADRGL2000064, ADRGL2000074, ADRGL2000085, ADRGL2000097, ADRGL2000117, ADRGL2000142, ADRGL2000172, ADRGL2000248, ADRGL2000261, ADRGL2000323, ADRGL2000353, ADRGL2000384, ADRGL2000428, ADRGL2000636, ADRGL2000644, ADRGL2000968, ADRGL2001119, ADRGL2001229, ADRGL2001287, ADRGL2001301, ADRGL2001354, ADRGL2001554, ADRGL2001651, ADRGL2001756,
ADRGL2001830, ADRGL2001836, ADRGL2001854, ADRGL2002029, ADRGL2002191, ADRGL2002260, ADRGL2002392, ADRGL2002477, ADRGL2002679, ADRGL2002753, ADRGL2002857, ADRGL2003017, ADRGL2003329, ADRGL2003552, ADRGL2003585, ADRGL2003638, ADRGL2003684, ADRGL2003773, ADRGL2003785, ADRGL2004031, ADRGL2004077, ADRGL2004451, ADRGL2004459, ADRGL2004708, ADRGL2004724, ADRGL2004749, ADRGL2004770, ADRGL2004777, ADRGL2004833, ADRGL2005336, ADRGL2005564, ADRGL2005756, ADRGL2005838, ADRGL2005926, ADRGL2005961, ADRGL2006034, ADRGL2006124, ADRGL2006177, ADRGL2006193, ADRGL2006377, ADRGL2006677, ADRGL2006732, ADRGL2006767, ADRGL2006817, ADRGL2006846, ADRGL2006886, ADRGL2007080, ADRGL2007125, ADRGL2007283, ADRGL2007313,
ADRGL2007357, ADRGL2007636, ADRGL2007651, ADRGL2007810, ADRGL2007877, ADRGL2007906, ADRGL2007974, ADRGL2008023, ADRGL2008331, ADRGL2008337, ADRGL2008535, ADRGL2008966, ADRGL2009146, ADRGL2009324, ADRGL2009489, ADRGL2009533, ADRGL2009691, ADRGL2009755, ADRGL2010152, ADRGL2010315, ADRGL2010594, ADRGL2010860, ADRGL2010974, ADRGL2011190, ADRGL2011330, ADRGL2012038, ADRGL2012179, AHMSC1000138, ASTRO1000009, ASTRO1000018, ASTRO1000029, ASTRO1000096, ASTRO1000165, ASTRO2000011, ASTRO2000014, ASTRO2000095, ASTRO2000141, ASTRO2000191, ASTRO2000278, ASTRO2000372, ASTRO2000381, ASTRO2000403, ASTRO2000417, ASTRO2000482, ASTRO2000533, ASTRO2000653, ASTRO2000662, ASTRO2000725, ASTRO2001001, ASTRO2001008,
ASTRO2001029, ASTRO2001088, ASTRO2001107, ASTRO2001122, ASTRO2001227, ASTRO2001249, ASTRO2001480, ASTRO2001806, ASTRO2002024, ASTRO2002035, ASTRO2002064, ASTRO2002202, ASTRO2002459, ASTRO2002571, ASTRO2002632, ASTRO2002659, ASTRO2002693, ASTRO2002720, ASTRO2002733, ASTRO2002743, ASTRO2002842, ASTRO2003212, ASTRO2003461, ASTRO2003574, ASTRO2003581, ASTRO2003632, ASTRO2003740, ASTRO2003840, ASTRO2003960, ASTRO2004032, ASTRO2004051, ASTRO2004177, ASTRO2004217, ASTRO2004297, ASTRO2004584, ASTRO2004628, ASTRO2004751, ASTRO2004877, ASTRO2004974, ASTRO2005008, ASTRO2005081, ASTRO2005096, ASTRO2005242, ASTRO2005273, ASTRO2005343, ASTRO2005360, ASTRO2005413, ASTRO2005423, ASTRO2005553, ASTRO2005557,
ASTRO2005575, ASTRO2005593, ASTRO2005687, ASTRO2005726, ASTRO2005896, ASTRO2005902, ASTRO2006356, ASTRO2006475, ASTRO2006747, ASTRO2006920, ASTRO2006952, ASTRO2007221, ASTRO2007377, ASTRO2007515, ASTRO2007578, ASTRO2007643, ASTRO2007666, ASTRO2007956, ASTRO2007962, ASTRO2008040, ASTRO2008239, ASTRO2008409, ASTRO2008425, ASTRO2008777, ASTRO2008895, ASTRO2008960, ASTRO2008972, ASTRO2009068, ASTRO2009118, ASTRO2009177, ASTRO2009333, ASTRO2009458, ASTRO2009526, ASTRO2009879, ASTRO2010072, ASTRO2010615, ASTRO2010799, ASTRO2010962, ASTRO2010996, ASTRO2011149, ASTRO2011422, ASTRO2011426, ASTRO2011437, ASTRO2011461, ASTRO2011834, ASTRO2011893, ASTRO2012552, ASTRO2013124, ASTRO2013671, ASTRO2013802,
ASTRO2014135, ASTRO2014174, ASTRO2014211, ASTRO2014363, ASTRO2014392, ASTRO2014498, ASTRO2014561, ASTRO2014576, ASTRO2014863, ASTRO2014923, ASTRO2014961, ASTRO2015162, ASTRO2015185, ASTRO2015328, ASTRO2015529, ASTRO2015987, ASTRO2016044, ASTRO2016114, ASTRO2016148, ASTRO2016681, ASTRO2016819, ASTRO2016847, ASTRO2016892, ASTRO2017348, ASTRO2017627, ASTRO2018169, ASTRO2018373, ASTRO2018540, ASTRO2019039, ASTRO2019171, ASTRO3000154, ASTRO3000172, ASTRO3000177, ASTRO3000301, ASTRO3000474, ASTRO3000482, BEAST2000454, BGGI11000123, BGGI11000193, BGGI11000285, BGGI12000022, BGGI12000067, BGGI12000161, BGGI12000533, BGGI12000544, BGGI12000616, BGGI12000684, BGGI12000693, BGGI12000839, BGGI12001075,
BGGI12001097, BGGI12001241, BGGI12001247, BGGI12001682, BGGI12001714, BLADE1000176, BLADE2000181, BLADE2000256, BLADE2000340, BLADE2000389, BLADE2000463, BLADE2000492, BLADE2000579, BLADE2000640, BLADE2000866, BLADE2000938, BLADE2001031, BLADE2001133, BLADE2001138, BLADE2001141, BLADE2001371, BLADE2001458, BLADE2001575, BLADE2001753, BLADE2001935, BLADE2001940, BLADE2001987, BLADE2002073, BLADE2002310, BLADE2002730, BLADE2002738, BLADE2002744, BLADE2002782, BLADE2002947, BLADE2002972, BLADE2003474, BLADE2003916, BLADE2004089, BLADE2004345, BLADE2004389, BLADE2004462, BLADE2004628, BLADE2004670, BLADE2004849, BLADE2005036, BLADE2005038, BLADE2005229, BLADE2005459, BLADE2005792, BLADE2006043,
BLADE2006415, BLADE2006607, BLADE2006826, BLADE2006830, BLADE2006849, BLADE2006947, BLADE2007033, BLADE2007043, BLADE2007666, BLADE2007722, BLADE2007735, BLADE2007744, BLADE2007799, BLADE2007923, BLADE2007935, BLADE2007958, BLADE2008281, BLADE2008398, BLADE2008454, BLADE2008461, BLADE2008479, BLADE2008539, BLADE2008809, BLADE2008995, BLADE2009452, BLADE2009479, BLADE2009593, BNGH41000098, BNGH41000104, BNGH41000118, BNGH41000153, BNGH41000169, BNGH41000177, BNGH41000190, BNGH41000198, BNGH41000216, BNGH41000235, BNGH42000130, BNGH42000132, BNGH42000360, BNGH42000405, BNGH42000532, BNGH42000675, BNGH42000791, BNGH42000815, BNGH42000994, BNGH42001247, BNGH42001406, BNGH42001576, BNGH42001724,
BNGH42002168, BNGH42002244, BNGH42002387, BNGH42002489, BNGH42003529, BNGH42003570, BNGH42003641, BNGH42004076, BNGH42004291, BNGH42004538, BNGH42004679, BNGH42005017, BNGH42005235, BNGH42005504, BNGH42005968, BNGH42006226, BNGH42006234, BNGH42007037, BNGH42007366, BNGH42007594, BNGH42007798, BNGH42008199, BNGH42008510, BNGH42008603, BNGH42008649, BNGH42008743, BNGH42008832, BNGH42008850, BNGH42008943, BNGH42009021, BNGH42009025, BRACE1000020, BRACE1000047, BRACE1000051, BRACE1000065, BRACE1000073, BRACE1000084, BRACE1000087, BRACE1000092, BRACE1000093, BRACE1000101, BRACE1000114, BRACE1000115, BRACE1000124, BRACE1000132, BRACE1000159, BRACE1000166, BRACE1000169, BRACE1000187, BRACE1000239,
BRACE1000258, BRACE1000451, BRACE1000475, BRACE1000572, BRACE2000061, BRACE2000077, BRACE2000078, BRACE2000100, BRACE2000109, BRACE2000141, BRACE2000148, BRACE2000183, BRACE2000229, BRACE2000232, BRACE2000245, BRACE2000256, BRACE2000266, BRACE2000267, BRACE2000280, BRACE2000300, BRACE2000307, BRACE2000331, BRACE2000332, BRACE2000351, BRACE2000368, BRACE2000392, BRACE2000441, BRACE2000487, BRACE2000489, BRACE2000505, BRACE2000525, BRACE2000526, BRACE2000531, BRACE2000545, BRACE2000553, BRACE2000565, BRACE2000577, BRACE2000640, BRACE2000650, BRACE2000677, BRACE2000698, BRACE2000718, BRACE2000733, BRACE2000743, BRACE2000753, BRACE2000815, BRACE2000864, BRACE2000885, BRACE2000893, BRACE2000905,
BRACE2000936, BRACE2000947, BRACE2000965, BRACE2000969, BRACE2000975, BRACE2000988, BRACE2001017, BRACE2001070, BRACE2001094, BRACE2001107, BRACE2001117, BRACE2001151, BRACE2001154, BRACE2001191, BRACE2001197, BRACE2001236, BRACE2001239, BRACE2001269, BRACE2001312, BRACE2001313, BRACE2001330, BRACE2001340, BRACE2001352, BRACE2001353, BRACE2001374, BRACE2001375, BRACE2001423, BRACE2001424, BRACE2001445, BRACE2001453, BRACE2001455, BRACE2001477, BRACE2001492, BRACE2001496, BRACE2001497, BRACE2001508, BRACE2001534, BRACE2001543, BRACE2001564, BRACE2001588, BRACE2001607, BRACE2001626, BRACE2001673, BRACE2001677, BRACE2001705, BRACE2001709, BRACE2001711, BRACE2001726, BRACE2001733, BRACE2001737,
BRACE2001742, BRACE2001777, BRACE2001779, BRACE2001834, BRACE2001859, BRACE2001865, BRACE2001872, BRACE2001881, BRACE2001892, BRACE2001898, BRACE2001923, BRACE2001942, BRACE2001944, BRACE2001954, BRACE2001972, BRACE2001973, BRACE2002026, BRACE2002034, BRACE2002050, BRACE2002081, BRACE2002091, BRACE2002139, BRACE2002142, BRACE2002151, BRACE2002157, BRACE2002202, BRACE2002206, BRACE2002227, BRACE2002260, BRACE2002297, BRACE2002304, BRACE2002356, BRACE2002392, BRACE2002394, BRACE2002431, BRACE2002441, BRACE2002444, BRACE2002458, BRACE2002467, BRACE2002468, BRACE2002495, BRACE2002582, BRACE2002585, BRACE2002589, BRACE2002590, BRACE2002606, BRACE2002613, BRACE2002614, BRACE2002617, BRACE2002623,
BRACE2002635, BRACE2002661, BRACE2002676, BRACE2002682, BRACE2002685, BRACE2002695, BRACE2002707, BRACE2002736, BRACE2002752, BRACE2002755, BRACE2002762, BRACE2002772, BRACE2002792, BRACE2002796, BRACE2002803, BRACE2002807, BRACE2002812, BRACE2002834, BRACE2002849, BRACE2002857, BRACE2002860, BRACE2002861, BRACE2002884, BRACE2002896, BRACE2002948, BRACE2003037, BRACE2003078, BRACE2003097, BRACE2003110, BRACE2003168, BRACE2003199, BRACE2003210, BRACE2003217, BRACE2003269, BRACE2003285, BRACE2003304, BRACE2003319, BRACE2003398, BRACE2003420, BRACE2003428, BRACE2003431, BRACE2003539, BRACE2003540, BRACE2003594, BRACE2003598, BRACE2003609, BRACE2003614, BRACE2003628, BRACE2003639, BRACE2003700,
BRACE2003713, BRACE2003766, BRACE2003788, BRACE2003800, BRACE2003802, BRACE2003825, BRACE2003827, BRACE2003845, BRACE2003847, BRACE2003848, BRACE2003873, BRACE2003885, BRACE2003887, BRACE2003905, BRACE2003928, BRACE2003944, BRACE2003954, BRACE2003982, BRACE2003987, BRACE2005034, BRACE2005047, BRACE2005063, BRACE2005083, BRACE2005090, BRACE2005099, BRACE2005118, BRACE2005124, BRACE2005138, BRACE2005151, BRACE2005160, BRACE2005169, BRACE2005189, BRACE2005193, BRACE2005198, BRACE2005216, BRACE2005243, BRACE2005251, BRACE2005253, BRACE2005298, BRACE2005328, BRACE2005329, BRACE2005337, BRACE2005348, BRACE2005363, BRACE2005395, BRACE2005408, BRACE2005433, BRACE2005434, BRACE2005448, BRACE2005450,
BRACE2005453, BRACE2005457, BRACE2005460, BRACE2005489, BRACE2005518, BRACE2005556, BRACE2005562, BRACE2005624, BRACE2005642, BRACE2005661, BRACE2005667, BRACE2005681, BRACE2005731, BRACE2005742, BRACE2005762, BRACE2005773, BRACE2005787, BRACE2005795, BRACE2005800, BRACE2005858, BRACE2005869, BRACE2005875, BRACE2005881, BRACE2005898, BRACE2005904, BRACE2005906, BRACE2005907, BRACE2005911, BRACE2005937, BRACE2005949, BRACE2005981, BRACE2005991, BRACE2006055, BRACE2006072, BRACE2006075, BRACE2006089, BRACE2006102, BRACE2006105, BRACE2006122, BRACE2006137, BRACE2006143, BRACE2006162, BRACE2006167, BRACE2006174, BRACE2006240, BRACE2006245, BRACE2006249, BRACE2006258, BRACE2006264, BRACE2006274,
BRACE2006281, BRACE2006298, BRACE2006319, BRACE2006344, BRACE2006363, BRACE2006378, BRACE2006380, BRACE2006393, BRACE2006413, BRACE2006417, BRACE2006453, BRACE2006459, BRACE2006488, BRACE2006514, BRACE2006534, BRACE2006540, BRACE2006547, BRACE2006564, BRACE2006587, BRACE2006598, BRACE2006685, BRACE2006703, BRACE2006743, BRACE2006753, BRACE2006783, BRACE2006821, BRACE2006833, BRACE2006859, BRACE2006871, BRACE2006909, BRACE2006911, BRACE2006924, BRACE2006935, BRACE2006937, BRACE2006951, BRACE2006982, BRACE2006986, BRACE2007013, BRACE2007068, BRACE2007087, BRACE2007089, BRACE2007109, BRACE2007138, BRACE2007191, BRACE2007197, BRACE2007201, BRACE2007203, BRACE2007232, BRACE2007254, BRACE2007262,
BRACE2007274, BRACE2007281, BRACE2007295, BRACE2007358, BRACE2007396, BRACE2007398, BRACE2007401, BRACE2007411, BRACE2007447, BRACE2007477, BRACE2007502, BRACE2007503, BRACE2007522, BRACE2007527, BRACE2007538, BRACE2007563, BRACE2007567, BRACE2007577, BRACE2007621, BRACE2007640, BRACE2007641, BRACE2007646, BRACE2007663, BRACE2007671, BRACE2007682, BRACE2007685, BRACE2007708, BRACE2007727, BRACE2007749, BRACE2007760, BRACE2007761, BRACE2007764, BRACE2007767, BRACE2007768, BRACE2007769, BRACE2007784, BRACE2007786, BRACE2007799, BRACE2007801, BRACE2007832, BRACE2007836, BRACE2007868, BRACE2007882, BRACE2007888, BRACE2007902, BRACE2007920, BRACE2007937, BRACE2007944, BRACE2007952, BRACE2008008,
BRACE2008015, BRACE2008021, BRACE2008097, BRACE2008102, BRACE2008114, BRACE2008144, BRACE2008172, BRACE2008216, BRACE2008218, BRACE2008251, BRACE2008276, BRACE2008295, BRACE2008308, BRACE2008336, BRACE2008358, BRACE2008361, BRACE2008380, BRACE2008385, BRACE2008399, BRACE2008401, BRACE2008402, BRACE2008410, BRACE2008420, BRACE2008443, BRACE2008473, BRACE2008480, BRACE2008481, BRACE2008488, BRACE2008495, BRACE2008500, BRACE2008553, BRACE2008594, BRACE2008604, BRACE2008615, BRACE2008622, BRACE2008629, BRACE2008655, BRACE2008656, BRACE2008706, BRACE2008708, BRACE2008710, BRACE2008754, BRACE2008797, BRACE2008847, BRACE2008857, BRACE2008861, BRACE2008867, BRACE2008881, BRACE2008883, BRACE2008917,
BRACE2008919, BRACE2008948, BRACE2008960, BRACE2008968, BRACE2008999, BRACE2009014, BRACE2009016, BRACE2009031, BRACE2009044, BRACE2009045, BRACE2009053, BRACE2009122, BRACE2009131, BRACE2009185, BRACE2009188, BRACE2009212, BRACE2009221, BRACE2009235, BRACE2009238, BRACE2009255, BRACE2009270, BRACE2009272, BRACE2009274, BRACE2009275, BRACE2009291, BRACE2009293, BRACE2009307, BRACE2009311, BRACE2009318, BRACE2009323, BRACE2009366, BRACE2009421, BRACE2009428, BRACE2009434, BRACE2009437, BRACE2009483, BRACE2009533, BRACE2009558, BRACE2009566, BRACE2009591, BRACE2009620, BRACE2009654, BRACE2009721, BRACE2009732, BRACE2009754, BRACE2009828, BRACE2009886, BRACE2009907, BRACE2009957, BRACE2010170,
BRACE2010171, BRACE2010203, BRACE2010208, BRACE2010336, BRACE2010348, BRACE2010440, BRACE2010444, BRACE2010477, BRACE2010489, BRACE2010535, BRACE2010550, BRACE2010598, BRACE2010632, BRACE2010642, BRACE2010651, BRACE2010669, BRACE2010684, BRACE2010753, BRACE2010813, BRACE2010828, BRACE2010837, BRACE2010888, BRACE2010937, BRACE2010983, BRACE2011007, BRACE2011025, BRACE2011199, BRACE2011265, BRACE2011389, BRACE2011478, BRACE2011545, BRACE2011592, BRACE2011611, BRACE2011646, BRACE2011677, BRACE2011703, BRACE2011747, BRACE2011838, BRACE2011892, BRACE2011904, BRACE2011947, BRACE2012061, BRACE2012062, BRACE2012185, BRACE2012222, BRACE2012232, BRACE2012291, BRACE2012317, BRACE2012510, BRACE2012625,
BRACE2012653, BRACE2012814, BRACE2012833, BRACE2012838, BRACE2012936, BRACE2012947, BRACE2013009, BRACE2013010, BRACE2013126, BRACE2013132, BRACE2013149, BRACE2013369, BRACE2013806, BRACE2013855, BRACE2013911, BRACE2014108, BRACE2014161, BRACE2014232, BRACE2014257, BRACE2014268, BRACE2014306, BRACE2014475, BRACE2014657, BRACE2014746, BRACE2014780, BRACE2014818, BRACE2014821, BRACE2014824, BRACE2014850, BRACE2014854, BRACE2015031, BRACE2015058, BRACE2015132, BRACE2015262, BRACE2015270, BRACE2015287, BRACE2015314, BRACE2015352, BRACE2015368, BRACE2015412, BRACE2015436, BRACE2015741, BRACE2015756, BRACE2015969, BRACE2015995, BRACE2016058, BRACE2016194, BRACE2016315, BRACE2016325, BRACE2016335,
BRACE2016362, BRACE2016366, BRACE2016465, BRACE2016516, BRACE2016583, BRACE2016708, BRACE2016821, BRACE2016896, BRACE2016981, BRACE2017108, BRACE2017124, BRACE2017298, BRACE2017359, BRACE2017410, BRACE2017438, BRACE2017580, BRACE2017587, BRACE2017720, BRACE2017844, BRACE2017872, BRACE2017929, BRACE2017934, BRACE2017985, BRACE2017992, BRACE2018337, BRACE2018443, BRACE2018448, BRACE2018568, BRACE2018700, BRACE2018727, BRACE2018736, BRACE2018759, BRACE2018762, BRACE2018782, BRACE2018847, BRACE2019004, BRACE2019044, BRACE2019244, BRACE2019258, BRACE2019327, BRACE2019348, BRACE2019371, BRACE2019467, BRACE2019510, BRACE2019519, BRACE2019528, BRACE2019618, BRACE2019696, BRACE2019703, BRACE2019843,
BRACE2019865, BRACE2019872, BRACE2019877, BRACE2020028, BRACE2020077, BRACE2020097, BRACE2020157, BRACE2020278, BRACE2020358, BRACE2020467, BRACE2020652, BRACE2020709, BRACE2020742, BRACE2020756, BRACE2020863, BRACE2020982, BRACE2021014, BRACE2021143, BRACE2021148, BRACE2021245, BRACE2021254, BRACE2021264, BRACE2021344, BRACE2021433, BRACE2021478, BRACE2021541, BRACE2021670, BRACE2021695, BRACE2021708, BRACE2021721, BRACE2021932, BRACE2021936, BRACE2022030, BRACE2022158, BRACE2022196, BRACE2022270, BRACE2022328, BRACE2022389, BRACE2022394, BRACE2022435, BRACE2022448, BRACE2022450, BRACE2022549, BRACE2022633, BRACE2022693, BRACE2022848, BRACE2022928, BRACE2022990, BRACE2023069, BRACE2023070,
BRACE2023223, BRACE2023268, BRACE2023284, BRACE2023404, BRACE2023451, BRACE2023540, BRACE2023633, BRACE2023718, BRACE2023727, BRACE2023744, BRACE2023800, BRACE2023801, BRACE2024074; BRACE2024222, BRACE2024610, BRACE2024627, BRACE2024781, BRACE2024826, BRACE2025018, BRACE2025316, BRACE2025333, BRACE2025437, BRACE2025452, BRACE2025492,. BRACE2025710, BRACE2025796, BRACE2025992, BRACE2026054, BRACE2026131, BRACE2026141, BRACE2026142, BRACE2026293, BRACE2026294, BRACE2026404, BRACE2026675, BRACE2026725, BRACE2026836, BRACE2026920, BRACE2026971, BRACE2027018, BRACE2027221, BRACE2027258, BRACE2027312, BRACE2027382, BRACE2027389, BRACE2027408, BRACE2027643, BRACE2027767, BRACE2027770, BRACE2027879,
BRACE2027970, BRACE2028171, BRACE2028392, BRACE2028410, BRACE2028636, BRACE2028741, BRACE2028956, BRACE2028970, BRACE2029112, BRACE2029356, BRACE2029396, BRACE2029581, BRACE2029644, BRACE2029849, BRACE2030039, BRACE2030096, BRACE2030323, BRACE2030326, BRACE2030345, BRACE2030736, BRACE2030883, BRACE2030884, BRACE2031154, BRACE2031232, BRACE2031527, BRACE2031531, BRACE2031553, BRACE2031899, BRACE2032044, BRACE2032090, BRACE2032134, BRACE2032148, BRACE2032182, BRACE2032329, BRACE2032385, BRACE2032436, BRACE2032477, BRACE2032502, BRACE2032538, BRACE2032584, BRACE2032823, BRACE2033128, BRACE2033394, BRACE2033478, BRACE2033720, BRACE2033786, BRACE2034434, BRACE2034523, BRACE2034616, BRACE2034622,
BRACE2034870, BRACE2035003, BRACE2035120, BRACE2035124, BRACE2035191, BRACE2035278, BRACE2035381, BRACE2035441, BRACE2035485, BRACE2036005, BRACE2036096, BRACE2036743, BRACE2036830, BRACE2036834, BRACE2036900, BRACE2037132, BRACE2037294, BRACE2037295, BRACE2037299, BRACE2037431, BRACE2037692, BRACE2037847, BRACE2038114, BRACE2038269, BRACE2038271, BRACE2038329, BRACE2038492, BRACE2038551, BRACE2038618, BRACE2039009, BRACE2039128, BRACE2039170, BRACE2039249, BRACE2039286, BRACE2039297, BRACE2039327, BRACE2039362, BRACE2039475, BRACE2039600, BRACE2039607, BRACE2039624, BRACE2039734, BRACE2039823, BRACE2039832, BRACE2039893, BRACE2039986, BRACE2040138, BRACE2040325, BRACE2040514, BRACE2040640,
BRACE2040987, BRACE2041009, BRACE2041095, BRACE2041200, BRACE2041264, BRACE2041890, BRACE2041898, BRACE2042394, BRACE2042398, BRACE2042550, BRACE2042620, BRACE2042628, BRACE2042873, BRACE2043036, BRACE2043105, BRACE2043142, BRACE2043248, BRACE2043349, BRACE2043381, BRACE2043774, BRACE2043942, BRACE2044057, BRACE2044105, BRACE2044263, BRACE2044286, BRACE2044304, BRACE2044473, BRACE2044510, BRACE2044640, BRACE2044816, BRACE2044946, BRACE2044949, BRACE2045300, BRACE2045428, BRACE2045596, BRACE2045772, BRACE2045947, BRACE2045954, BRACE2046251, BRACE2046295, BRACE2046976, BRACE2047011, BRACE2047232, BRACE2047350, BRACE2047377, BRACE2047385, BRACE2047492, BRACE2047558, BRACE2047573, BRACE2047835,
BRACE2047893, BRACE2047975, BRACE3000071, BRACE3000404, BRACE3000520, BRACE3000697, BRACE3000840, BRACE3000973, BRACE3001002, BRACE3001058, BRACE3001066, BRACE3001113, BRACE3001217, BRACE3001299, BRACE3001384, BRACE3001391, BRACE3001403, BRACE3001595, BRACE3001754, BRACE3001948, BRACE3001973, BRACE3002184, BRACE3002298, BRACE3002344, BRACE3002390, BRACE3002420, BRACE3002508, BRACE3002541, BRACE3002756, BRACE3003004, BRACE3003026, BRACE3003053, BRACE3003192, BRACE3003413, BRACE3003595, BRACE3003698, BRACE3003866, BRACE3003920, BRACE3004046, BRACE3004058, BRACE3004113, BRACE3004150, BRACE3004205, BRACE3004358, BRACE3004435, BRACE3004477, BRACE3004767, BRACE3004772, BRACE3004783, BRACE3004843,
BRACE3004862, BRACE3004880, BRACE3004887, BRACE3004981, BRACE3004996, BRACE3004998, BRACE3005103, BRACE3005127, BRACE3005145, BRACE3005217, BRACE3005225, BRACE3005430, BRACE3005499, BRACE3005760, BRACE3005870, BRACE3005903, BRACE3005989, BRACE3006113, BRACE3006185, BRACE3006226, BRACE3006462, BRACE3006463, BRACE3006553, BRACE3006872, BRACE3006917, BRACE3007058, BRACE3007084, BRACE3007085, BRACE3007322, BRACE3007472, BRACE3007480, BRACE3007559, BRACE3007625, BRACE3007642, BRACE3007649, BRACE3007767, BRACE3007869, BRACE3008036, BRACE3008066, BRACE3008092, BRACE3008095, BRACE3008137, BRACE3008255, BRACE3008337, BRACE3008384, BRACE3008720, BRACE3009044, BRACE3009075, BRACE3009090, BRACE3009127,
BRACE3009197, BRACE3009237, BRACE3009265, BRACE3009297, BRACE3009377, BRACE3009392, BRACE3009416, BRACE3009539, BRACE3009574, BRACE3009701, BRACE3009708, BRACE3009724, BRACE3009993, BRACE3010076, BRACE3010079, BRACE3010086, BRACE3010221, BRACE3010283, BRACE3010315, BRACE3010397, BRACE3010416, BRACE3010435, BRACE3010438, BRACE3010702, BRACE3010862, BRACE3011271, BRACE3011421, BRACE3011447, BRACE3011505, BRACE3011634, BRACE3011774, BRACE3012357, BRACE3012574, BRACE3012806, BRACE3012930, BRACE3013119, BRACE3013418, BRACE3013576, BRACE3013740, BRACE3013780, BRACE3013874, BRACE3013936, BRACE3013986, BRACE3014005, BRACE3014068, BRACE3014231, BRACE3014290, BRACE3014317, BRACE3014523, BRACE3014714,
BRACE3014807, BRACE3014942, BRACE3015090, BRACE3015121, BRACE3015262, BRACE3015521, BRACE3015808, BRACE3015829, BRACE3015894, BRACE3015898, BRACE3016020, BRACE3016122, BRACE3016167, BRACE3016170, BRACE3016580, BRACE3016788, BRACE3016810, BRACE3016862, BRACE3016884, BRACE3017083, BRACE3017253, BRACE3018083, BRACE3018308, BRACE3018963, BRACE3019055, BRACE3019071, BRACE3019084, BRACE3019570, BRACE3019611, BRACE3019817, BRACE3019828, BRACE3019941, BRACE3020151, BRACE3020194, BRACE3020286, BRACE3020356, BRACE3020594, BRACE3020669, BRACE3020671, BRACE3020890, BRACE3021148, BRACE3021430, BRACE3021517, BRACE3021805, BRACE3022051, BRACE3022303, BRACE3022312, BRACE3022340, BRACE3022847, BRACE3023604,
BRACE3023912, BRACE3024073, BRACE3024359, BRACE3024379, BRACE3024444, BRACE3024497, BRACE3024537, BRACE3024659, BRACE3024662, BRACE3024879, BRACE3025153, BRACE3025302, BRACE3025457, BRACE3025531, BRACE3025627, BRACE3025630, BRACE3025719, BRACE3026008, BRACE3026075, BRACE3026161, BRACE3026290, BRACE3026345, BRACE3026456, BRACE3026735, BRACE3026802, BRACE3026844, BRACE3026947, BRACE3026993, BRACE3027242, BRACE3027256, BRACE3027326, BRACE3027480, BRACE3027931, BRACE3027976, BRACE3028214, BRACE3028241, BRACE3028360, BRACE3028895, BRACE3028998, BRACE3029005, BRACE3029021, BRACE3029205, BRACE3029447, BRACE3030057, BRACE3030103, BRACE3030538, BRACE3030748, BRACE3030866, BRACE3031161, BRACE3031184,
BRACE3031185, BRACE3031315, BRACE3031372, BRACE3031579, BRACE3031728, BRACE3031838, BRACE3031843, BRACE3032427, BRACE3032455, BRACE3032537, BRACE3032538, BRACE3032631, BRACE3032771, BRACE3032980, BRACE3032983, BRACE3033525, BRACE3034159, BRACE3034183, BRACE3034389, BRACE3034964, BRACE3034993, BRACE3035168, BRACE3035227, BRACE3036156, BRACE3036271, BRACE3037612, BRACE3037637, BRACE3037803, BRACE3038012, BRACE3038030, BRACE3038569, BRACE3038570, BRACE3038687, BRACE3038760, BRACE3039288, BRACE3039358, BRACE3039378, BRACE3039454, BRACE3040012, BRACE3040239, BRACE3040504, BRACE3040644, BRACE3040856, BRACE3041059, BRACE3041162, BRACE3041827, BRACE3042046, BRACE3042210, BRACE3042326, BRACE3042409,
BRACE3042432, BRACE3042594, BRACE3043597, BRACE3044090, BRACE3044172, BRACE3044247, BRACE3044377, BRACE3044389, BRACE3044495, BRACE3045011, BRACE3045033, BRACE3045078, BRACE3045145, BRACE3045424, BRACE3045708, BRACE3045981, BRACE3046049, BRACE3046152, BRACE3046294, BRACE3046450, BRACE3046466, BRACE3046491, BRACE3046609, BRACE3046762, BRACE3046837, BRACE3046855, BRACE3046966, BRACE3047018, BRACE3047482, BRACE3047801, BRALZ1000047, BRALZ1000103, BRALZ2000189, BRALZ2000263, BRALZ2000988, BRALZ2001038, BRALZ2001279, BRALZ2001350, BRALZ2001445, BRALZ2001559, BRALZ2001743, BRALZ2001834, BRALZ2002174, BRALZ2002418, BRALZ2002477, BRALZ2003119, BRALZ2003330, BRALZ2003453, BRALZ2003525, BRALZ2004397,
BRALZ2005467, BRALZ2005471, BRALZ2005888, BRALZ2005950, BRALZ2005992, BRALZ2006288, BRALZ2006474, BRALZ2006560, BRALZ2006734, BRALZ2006800, BRALZ2006976, BRALZ2007376, BRALZ2007545, BRALZ2007576, BRALZ2007661, BRALZ2007790, BRALZ2007793, BRALZ2007952, BRALZ2008031, BRALZ2008166, BRALZ2008462, BRALZ2008617, BRALZ2008696, BRALZ2008763, BRALZ2008869, BRALZ2008930, BRALZ2009486, BRALZ2010186, BRALZ2010842, BRALZ2011337, BRALZ2011796, BRALZ2011880, BRALZ2012050, BRALZ2012183, BRALZ2012848, BRALZ2013621, BRALZ2013690, BRALZ2014054, BRALZ2014316, BRALZ2014484, BRALZ2014951, BRALZ2015168, BRALZ2015811, BRALZ2016085, BRALZ2016498, BRALZ2017531, BRALZ2017607, BRALZ2017620, BRALZ2017844, BRALZ2018342,
BRALZ2018492, BRALZ2018834, BRALZ2019047, BRAMY1000095, BRAMY1000098, BRAMY1000130, BRAMY1000157, BRAMY1000173, BRAMY2000021, BRAMY2000025, BRAMY2000052, BRAMY2000076, BRAMY2000086, BRAMY2000151, BRAMY2000181, BRAMY2000196, BRAMY2000231, BRAMY2000277, BRAMY2000354, BRAMY2000388, BRAMY2000411, BRAMY2000508, BRAMY2000562, BRAMY2000653, BRAMY2000814, BRAMY2000893, BRAMY2000946, BRAMY2000981, BRAMY2000987, BRAMY2001068, BRAMY2001114, BRAMY2001203, BRAMY2001282, BRAMY2001367, BRAMY2001427, BRAMY2001473, BRAMY2001678, BRAMY2002005, BRAMY2002044, BRAMY2002072, BRAMY2002158, BRAMY2002311, BRAMY2002339, BRAMY2002364, BRAMY2002369, BRAMY2002479, BRAMY2002493, BRAMY2002525, BRAMY2002584, BRAMY2002604,
BRAMY2002621, BRAMY2002638, BRAMY2002739, BRAMY2002799, BRAMY2002853, BRAMY2002862, BRAMY2002956, BRAMY2003008, BRAMY2003037, BRAMY2003117, BRAMY2003287, BRAMY2003325, BRAMY2003529, BRAMY2003538, BRAMY2003585, BRAMY2003634, BRAMY2003653, BRAMY2003681, BRAMY2003893, BRAMY2003897, BRAMY2003898, BRAMY2003904, BRAMY2003926, BRAMY2003929, BRAMY2004031, BRAMY2004058, BRAMY2004183, BRAMY2004335, BRAMY2004351, BRAMY2004352, BRAMY2004387, BRAMY2004492, BRAMY2004524, BRAMY2004537, BRAMY2004542, BRAMY2004756, BRAMY2004771, BRAMY2005052, BRAMY2005064, BRAMY2005094, BRAMY2005151, BRAMY2005182, BRAMY2005244, BRAMY2005391, BRAMY2005488, BRAMY2005576, BRAMY2005648, BRAMY2005662, BRAMY2005708, BRAMY2005711,
BRAMY2006092, BRAMY2006118, BRAMY2006149, BRAMY2006375, BRAMY2006397, BRAMY2006411, BRAMY2006459, BRAMY2006466, BRAMY2006659, BRAMY2006779, BRAMY2006809, BRAMY2006879, BRAMY2007043, BRAMY2007078, BRAMY2007110, BRAMY2007185, BRAMY2007244, BRAMY2007249, BRAMY2007255, BRAMY2007287, BRAMY2007308, BRAMY2007411, BRAMY2007458, BRAMY2007486, BRAMY2007546, BRAMY2007567, BRAMY2007594, BRAMY2007610, BRAMY2007613, BRAMY2007653, BRAMY2007753, BRAMY2007859, BRAMY2008333, BRAMY2008382, BRAMY2008563, BRAMY2008583, BRAMY2008977, BRAMY2008979, BRAMY2009013, BRAMY2009023, BRAMY2009034, BRAMY2009105, BRAMY2009192, BRAMY2009344, BRAMY2009349, BRAMY2009394, BRAMY2009489, BRAMY2009508, BRAMY2009528, BRAMY2009557,
BRAMY2009612, BRAMY2009693, BRAMY2010068, BRAMY2010135, BRAMY2010145, BRAMY2010171, BRAMY2010208, BRAMY2010239, BRAMY2010272, BRAMY2010290, BRAMY2010357, BRAMY2010366, BRAMY2010395, BRAMY2010441, BRAMY2010442, BRAMY2010464, BRAMY2010581, BRAMY2010798, BRAMY2010808, BRAMY2010919, BRAMY2011064, BRAMY2011178, BRAMY2011196, BRAMY2011216, BRAMY2011253, BRAMY2011446, BRAMY2011480, BRAMY2011639, BRAMY2011679, BRAMY2011811, BRAMY2011841, BRAMY2011846, BRAMY2011849, BRAMY2011872, BRAMY2011961, BRAMY2012017, BRAMY2012091, BRAMY2012119, BRAMY2012162, BRAMY2012163, BRAMY2012277, BRAMY2012340, BRAMY2012426, BRAMY2012497, BRAMY2012517, BRAMY2012555, BRAMY2012568, BRAMY2012581, BRAMY2012691, BRAMY2012721,
BRAMY2012731, BRAMY2012776, BRAMY2012880, BRAMY2012881, BRAMY2013169, BRAMY2013200, BRAMY2013216, BRAMY2013405, BRAMY2013414, BRAMY2013572, BRAMY2013590, BRAMY2013621, BRAMY2013722, BRAMY2013736, BRAMY2013756, BRAMY2013944, BRAMY2013975, BRAMY2014019, BRAMY2014205, BRAMY2014387, BRAMY2014462, BRAMY2014469, BRAMY2014541, BRAMY2014754, BRAMY2014813, BRAMY2015211, BRAMY2015251, BRAMY2015311, BRAMY2015420, BRAMY2015503, BRAMY2015516, BRAMY2015550, BRAMY2015551, BRAMY2015559, BRAMY2015688, BRAMY2015705, BRAMY2015782, BRAMY2015833, BRAMY2015855, BRAMY2015890, BRAMY2015911, BRAMY2015925, BRAMY2016002, BRAMY2016009, BRAMY2016020, BRAMY2016070, BRAMY2016251, BRAMY2016325, BRAMY2016327, BRAMY2016386,
BRAMY2016706, BRAMY2016771, BRAMY2016892, BRAMY2016938, BRAMY2016953, BRAMY2017014, BRAMY2017249, BRAMY2017348, BRAMY2017450, BRAMY2017455, BRAMY2017528, BRAMY2017864, BRAMY2018027, BRAMY2018106, BRAMY2018122, BRAMY2018126, BRAMY2018272, BRAMY2018273, BRAMY2018279, BRAMY2018295, BRAMY2018308, BRAMY2018396, BRAMY2018467, BRAMY2018635, BRAMY2018785, BRAMY2019055, BRAMY2019111, BRAMY2019277, BRAMY2019300, BRAMY2019468, BRAMY2019509, BRAMY2019554, BRAMY2019600, BRAMY2019643, BRAMY2019710, BRAMY2019963, BRAMY2019985, BRAMY2020050, BRAMY2020058, BRAMY2020106, BRAMY2020270, BRAMY2020354, BRAMY2020355, BRAMY2020412, BRAMY2020445, BRAMY2020466, BRAMY2020520, BRAMY2020574, BRAMY2020634, BRAMY2020713,
BRAMY2021040, BRAMY2021063, BRAMY2021098, BRAMY2021139, BRAMY2021142, BRAMY2021276, BRAMY2021310, BRAMY2021388, BRAMY2021498, BRAMY2021523, BRAMY2021710, BRAMY2021732, BRAMY2021746, BRAMY2021825, BRAMY2021867, BRAMY2021962, BRAMY2021982, BRAMY2022052, BRAMY2022160, BRAMY2022263, BRAMY2022301, BRAMY2022320, BRAMY2022383, BRAMY2022445, BRAMY2022454, BRAMY2022493, BRAMY2022525, BRAMY2022532, BRAMY2022723, BRAMY2022786, BRAMY2022796, BRAMY2022929, BRAMY2022980, BRAMY2022984, BRAMY2023060, BRAMY2023110, BRAMY2023115, BRAMY2023161, BRAMY2023172, BRAMY2023238, BRAMY2023327, BRAMY2023358, BRAMY2023406, BRAMY2023482, BRAMY2023514, BRAMY2023719, BRAMY2023729, BRAMY2023786, BRAMY2023852, BRAMY2023863,
BRAMY2023939, BRAMY2024004, BRAMY2024073, BRAMY2024312, BRAMY2024374, BRAMY2024390, BRAMY2024416, BRAMY2024449, BRAMY2024489, BRAMY2024497, BRAMY2024514, BRAMY2024530, BRAMY2024535, BRAMY2024545, BRAMY2024576, BRAMY2024593, BRAMY2024711, BRAMY2024715, BRAMY2024728, BRAMY2024827, BRAMY2024849, BRAMY2025024, BRAMY2025032, BRAMY2025121, BRAMY2025175, BRAMY2025218, BRAMY2025230, BRAMY2025272, BRAMY2025277, BRAMY2025495, BRAMY2025812, BRAMY2025996, BRAMY2026009, BRAMY2026091, BRAMY2026168, BRAMY2026300, BRAMY2026347, BRAMY2026395, BRAMY2026405, BRAMY2026533, BRAMY2026538, BRAMY2026713, BRAMY2026778, BRAMY2026824, BRAMY2026899, BRAMY2026902, BRAMY2026904, BRAMY2026976, BRAMY2027073, BRAMY2027114,
BRAMY2027140, BRAMY2027242, BRAMY2027396, BRAMY2027451, BRAMY2027461, BRAMY2027467, BRAMY2027587, BRAMY2027714, BRAMY2027717, BRAMY2027739, BRAMY2027752, BRAMY2027782, BRAMY2028072, BRAMY2028183, BRAMY2028188, BRAMY2028269, BRAMY2028282, BRAMY2028472, BRAMY2028491, BRAMY2028516, BRAMY2028565, BRAMY2028593, BRAMY2028682, BRAMY2028783, BRAMY2028796, BRAMY2028856, BRAMY2028914, BRAMY2029204, BRAMY2029602, BRAMY2029988, BRAMY2030098, BRAMY2030109, BRAMY2030206, BRAMY2030477, BRAMY2030545, BRAMY2030607, BRAMY2030702, BRAMY2030703, BRAMY2030768, BRAMY2030799, BRAMY2030859, BRAMY2030915, BRAMY2031317, BRAMY2031377, BRAMY2031442, BRAMY2032014, BRAMY2032231, BRAMY2032242, BRAMY2032290, BRAMY2032311,
BRAMY2032317, BRAMY2032344, BRAMY2032589, BRAMY2032678, BRAMY2032696, BRAMY2032777, BRAMY2032994, BRAMY2033003, BRAMY2033116, BRAMY2033240, BRAMY2033267, BRAMY2033316, BRAMY2033332, BRAMY2033506, BRAMY2033594, BRAMY2033895, BRAMY2033914, BRAMY2034072, BRAMY2034185, BRAMY2034187, BRAMY2034305, BRAMY2034561, BRAMY2034920, BRAMY2034993, BRAMY2035070, BRAMY2035449, BRAMY2035545, BRAMY2035606, BRAMY2035718, BRAMY2035719, BRAMY2035801, BRAMY2035869, BRAMY2035923, BRAMY2036076, BRAMY2036079, BRAMY2036254, BRAMY2036266, BRAMY2036387, BRAMY2036396, BRAMY2036459, BRAMY2036567, BRAMY2036615, BRAMY2036699, BRAMY2036913, BRAMY2036918, BRAMY2036968, BRAMY2037147, BRAMY2037328, BRAMY2037609, BRAMY2037629,
BRAMY2037800, BRAMY2037823, BRAMY2037971, BRAMY2038100, BRAMY2038128, BRAMY2038317, BRAMY2038475, BRAMY2038484, BRAMY2038516, BRAMY2038832, BRAMY2038846, BRAMY2038887, BRAMY2038904, BRAMY2039287, BRAMY2039300, BRAMY2039314, BRAMY2039341, BRAMY2039630, BRAMY2039695, BRAMY2039728, BRAMY2039872, BRAMY2040051, BRAMY2040159, BRAMY2040214, BRAMY2040478, BRAMY2040592, BRAMY2040915, BRAMY2041261, BRAMY2041347, BRAMY2041378, BRAMY2041384, BRAMY2041434, BRAMY2041507, BRAMY2041542, BRAMY2041738, BRAMY2042122, BRAMY2042131, BRAMY2042178, BRAMY2042195, BRAMY2042244, BRAMY2042549, BRAMY2042596, BRAMY2042612, BRAMY2042641, BRAMY2042711, BRAMY2042760, BRAMY2042804, BRAMY2042899, BRAMY2042918, BRAMY2043069,
BRAMY2043103, BRAMY2043314, BRAMY2043345, BRAMY2044246, BRAMY2044317, BRAMY2044349, BRAMY2044686, BRAMY2044838, BRAMY2045036, BRAMY2045299, BRAMY2045471, BRAMY2045530, BRAMY2046109, BRAMY2046478, BRAMY2046489, BRAMY2046537, BRAMY2046667, BRAMY2046742, BRAMY2046871, BRAMY2046984, BRAMY2046989, BRAMY2047169, BRAMY2047280, BRAMY2047420, BRAMY2047676, BRAMY2047692, BRAMY2047746, BRAMY2047751, BRAMY2047765, BRAMY2047884, BRAMY2047948, BRAMY3000206, BRAMY3000210, BRAMY3000213, BRAMY3000224, BRAMY3000254, BRAMY3000307, BRAMY3000692, BRAMY3001356, BRAMY3001401, BRAMY3001409, BRAMY3001794, BRAMY3002120, BRAMY3002312, BRAMY3002329, BRAMY3002508, BRAMY3002620, BRAMY3002803, BRAMY3002805, BRAMY3002886,
BRAMY3002991, BRAMY3003026, BRAMY3003109, BRAMY3003205, BRAMY3003310, BRAMY3003566, BRAMY3003723, BRAMY3004126, BRAMY3004224, BRAMY3004364, BRAMY3004377, BRAMY3004672, BRAMY3004800, BRAMY3004900, BRAMY3004919, BRAMY3005091, BRAMY3005656, BRAMY3005912, BRAMY3005932, BRAMY3006032, BRAMY3006297, BRAMY3006761, BRAMY3007078, BRAMY3007206, BRAMY3007311, BRAMY3007350, BRAMY3007449, BRAMY3007471, BRAMY3007609, BRAMY3007723, BRAMY3007968, BRAMY3008044, BRAMY3008088, BRAMY3008096, BRAMY3008335, BRAMY3008466, BRAMY3008505, BRAMY3008650, BRAMY3008937, BRAMY3009158, BRAMY3009207, BRAMY3009491, BRAMY3009556, BRAMY3009782, BRAMY3009811, BRAMY3009904, BRAMY3010008, BRAMY3010264, BRAMY3010321, BRAMY3010411,
BRAMY3010492, BRAMY3010603, BRAMY3010654, BRAMY4000089, BRAMY4000095, BRAMY4000229, BRAMY4000277, BRAMY4000915, BRAMY4000962, BRAMY4001173, BRAMY4001200, BRAMY4001234, BRAMY4001449, BRAMY4001652, BRAMY4001863, BRAMY4001913, BRAMY4002575, BRAMY4002628, BRASW1000053, BRASW1000125, BRAWH1000001, BRAWH1000002, BRAWH1000007, BRAWH1000037, BRAWH1000045, BRAWH1000083, BRAWH1000094, BRAWH1000116, BRAWH1000127, BRAWH1000130, BRAWH1000157, BRAWH1000162, BRAWH1000164, BRAWH1000168, BRAWH1000174, BRAWH1000180, BRAWH1000369, BRAWH2000034, BRAWH2000048, BRAWH2000071, BRAWH2000093, BRAWH2000109, BRAWH2000131, BRAWH2000142, BRAWH2000169, BRAWH2000177, BRAWH2000218, BRAWH2000232, BRAWH2000248, BRAWH2000256,
BRAWH2000274, BRAWH2000323, BRAWH2000353, BRAWH2000370, BRAWH2000409, BRAWH2000417, BRAWH2000443, BRAWH2000460, BRAWH2000471, BRAWH2000476, BRAWH2000482, BRAWH2000494, BRAWH2000500, BRAWH2000503, BRAWH2000522, BRAWH2000554, BRAWH2000595, BRAWH2000636, BRAWH2000643, BRAWH2000651, BRAWH2000682, BRAWH2000686, BRAWH2000752, BRAWH2000859, BRAWH2000866, BRAWH2000892, BRAWH2000901, BRAWH2000924, BRAWH2000926, BRAWH2000944, BRAWH2000984, BRAWH2001019, BRAWH2001037, BRAWH2001052, BRAWH2001103, BRAWH2001129, BRAWH2001137, BRAWH2001159, BRAWH2001166, BRAWH2001171, BRAWH2001186, BRAWH2001203, BRAWH2001238, BRAWH2001239, BRAWH2001255, BRAWH2001321, BRAWH2001355, BRAWH2001378, BRAWH2001395, BRAWH2001406,
BRAWH2001418, BRAWH2001424, BRAWH2001436, BRAWH2001438, BRAWH2001459, BRAWH2001461, BRAWH2001484, BRAWH2001492, BRAWH2001503, BRAWH2001519, BRAWH2001530, BRAWH2001535, BRAWH2001589, BRAWH2001662, BRAWH2001666, BRAWH2001671, BRAWH2001679, BRAWH2001686, BRAWH2001701, BRAWH2001842, BRAWH2001862, BRAWH2001873, BRAWH2001940, BRAWH2001973, BRAWH2002047, BRAWH2002060, BRAWH2002062, BRAWH2002125, BRAWH2002191, BRAWH2002318, BRAWH2002333, BRAWH2002352, BRAWH2002383, BRAWH2002549, BRAWH2002560, BRAWH2002623, BRAWH2002630, BRAWH2002635, BRAWH2002662, BRAWH2002679, BRAWH2002725, BRAWH2002761, BRAWH2002811, BRAWH2002861, BRAWH2002911, BRAWH2002963, BRAWH2002967, BRAWH2002976, BRAWH2003000, BRAWH2003025,
BRAWH2003135, BRAWH2003240, BRAWH2003307, BRAWH2003355, BRAWH2003366, BRAWH2003662, BRAWH2003689, BRAWH2003818, BRAWH2003832, BRAWH2003948, BRAWH2003964, BRAWH2004044, BRAWH2004068, BRAWH2004095, BRAWH2004136, BRAWH2004217, BRAWH2004580, BRAWH2004731, BRAWH2004779, BRAWH2004842, BRAWH2004884, BRAWH2004923, BRAWH2005008, BRAWH2005068, BRAWH2005074, BRAWH2005225, BRAWH2005315, BRAWH2005517, BRAWH2005524, BRAWH2005533, BRAWH2005812, BRAWH2005974, BRAWH2005998, BRAWH2006044, BRAWH2006083, BRAWH2006207, BRAWH2006301, BRAWH2006395, BRAWH2006405, BRAWH2006450, BRAWH2006493, BRAWH2006526, BRAWH2006543, BRAWH2006622, BRAWH2006760, BRAWH2006960, BRAWH2006989, BRAWH2006996, BRAWH2007069, BRAWH2007308,
BRAWH2007406, BRAWH2007550, BRAWH2007570, BRAWH2007591, BRAWH2007658, BRAWH2007664, BRAWH2007800, BRAWH2007808, BRAWH2007825, BRAWH2007862, BRAWH2007890, BRAWH2008128, BRAWH2008219, BRAWH2008255, BRAWH2008292, BRAWH2008297, BRAWH2008366, BRAWH2008380, BRAWH2008540, BRAWH2008577, BRAWH2008706, BRAWH2008790, BRAWH2008903, BRAWH2008956, BRAWH2008993, BRAWH2009112, BRAWH2009227, BRAWH2009261, BRAWH2009263, BRAWH2009298, BRAWH2009304, BRAWH2009307, BRAWH2009360, BRAWH2009393, BRAWH2009485, BRAWH2009490, BRAWH2009558, BRAWH2009590, BRAWH2009626, BRAWH2009678, BRAWH2009695, BRAWH2009907, BRAWH2010000, BRAWH2010069, BRAWH2010084, BRAWH2010136, BRAWH2010318, BRAWH2010329, BRAWH2010354, BRAWH2010364,
BRAWH2010536, BRAWH2010552, BRAWH2010584, BRAWH2010618, BRAWH2010619, BRAWH2010754, BRAWH2010980, BRAWH2011066, BRAWH2011079, BRAWH2011096, BRAWH2011155, BRAWH2011166, BRAWH2011204, BRAWH2011286, BRAWH2011294, BRAWH2011400, BRAWH2011762, BRAWH2011795, BRAWH2011796, BRAWH2011812, BRAWH2011823, BRAWH2011860, BRAWH2011920, BRAWH2011958, BRAWH2012054, BRAWH2012077, BRAWH2012161, BRAWH2012162, BRAWH2012164, BRAWH2012258, BRAWH2012277, BRAWH2012326, BRAWH2012538, BRAWH2012540, BRAWH2012619, BRAWH2012749, BRAWH2012827, BRAWH2012866, BRAWH2012963, BRAWH2013047, BRAWH2013219, BRAWH2013294, BRAWH2013748, BRAWH2013866, BRAWH2.013871, BRAWH2013914, BRAWH2013941, BRAWH2013955, BRAWH2014053, BRAWH2014234,
BRAWH2014379, BRAWH2014414, BRAWH2014511, BRAWH2014662, BRAWH2014717, BRAWH2014729, BRAWH2014876, BRAWH2014934, BRAWH2014954, BRAWH2015247, BRAWH2015349, BRAWH2015375, BRAWH2015385, BRAWH2015595, BRAWH2015728, BRAWH2015853, BRAWH2015866, BRAWH2016028, BRAWH2016106, BRAWH2016160, BRAWH2016209, BRAWH2016221, BRAWH2016223, BRAWH2016269, BRAWH2016305, BRAWH2016439, BRAWH2016446, BRAWH2016514, BRAWH2016562, BRAWH2016655, BRAWH2016679, BRAWH2016702, BRAWH2016724, BRAWH2016785, BRAWH2016792, BRAWH2016800, BRAWH2017002, BRAWH2017103, BRAWH2017250, BRAWH2017294, BRAWH2017305, BRAWH2017379, BRAWH2017407, BRAWH2017433, BRAWH2017523, BRAWH2017534, BRAWH2017542, BRAWH2017635, BRAWH2017913, BRAWH2018206,
BRAWH2018267, BRAWH2018282, BRAWH2018317, BRAWH2018506, BRAWH2018514, BRAWH2018527, BRAWH2018601, BRAWH2018729, BRAWH2018875, BRAWH2019053, BRAWH2019055, BRAWH2019198, BRAWH3000078, BRAWH3000088, BRAWH3000100, BRAWH3000166, BRAWH3000329, BRAWH3000410, BRAWH3000446, BRAWH3000491, BRAWH3000604, BRAWH3000884, BRAWH3001053, BRAWH3001319, BRAWH3001475, BRAWH3001636, BRAWH3001638, BRAWH3001712, BRAWH3001760, BRAWH3001783, BRAWH3001833, BRAWH3001879, BRAWH3001891, BRAWH3001918, BRAWH3002433, BRAWH3002467, BRAWH3002513, BRAWH3002600, BRAWH3002819, BRAWH3003019, BRAWH3003136, BRAWH3003244, BRAWH3003343, BRAWH3003411, BRAWH3003522, BRAWH3003573, BRAWH3003598, BRAWH3003801, BRAWH3003975, BRAWH3004222,
BRAWH3004335, BRAWH3004453, BRAWH3004530, BRAWH3004666, BRAWH3005037, BRAWH3005047, BRAWH3005132, BRAWH3005146, BRAWH3005292, BRAWH3005300, BRAWH3005422, BRAWH3005534, BRAWH3005886, BRAWH3005892, BRAWH3005896, BRAWH3005912, BRAWH3005981, BRAWH3006547, BRAWH3006548, BRAWH3006761, BRAWH3006792, BRAWH3007129, BRAWH3007221, BRAWH3007441, BRAWH3007455, BRAWH3007506, BRAWH3007726, BRAWH3007783, BRAWH3008167, BRAWH3008341, BRAWH3008559, BRAWH3008697, BRAWH3008867, BRAWH3008931, BRAWH3009017, BRAWH3009285, BRAWH3009297, BRAWH3009546, BRAWH3009961, BRAWH3010461, BRAWH3010560, BRAWH3010602, BRAWH3010657, BRAWH3010726, BRAWH3010833, BRAWH3011101, BRAWH3011331, BRAWH3011402, BRAWH3011577, BRAWH3011623,
BRAWH3011637, BRAWH3011685, BRAWH3011907, BRAWH3011929, BRAWH3012005, BRAWH3012662, BRAWH3012779, BRAWH3013009, BRAWH3013049, BRAWH3013197, BRAWH3013264, BRAWH3013508, BRAWH3014609, BRAWH3014639, BRAWH3015017, BRAWH3015175, BRAWH3015260, BRAWH3015610, BRAWH3015825, BRAWH3016123, BRAWH3016715, BRAWH3017259, BRAWH3017260, BRAWH3017477, BRAWH3017537, BRAWH3017980, BRAWH3018063, BRAWH3018369, BRAWH3018548, BRAWH3018969, BRAWH3019026, BRAWH3019529, BRAWH3019594, BRAWH3019629, BRAWH3019820, BRAWH3020200, BRAWH3020318, BRAWH3020486, BRAWH3020884, BRAWH3020928, BRAWH3021012, BRAWH3021574, BRAWH3021580, BRAWH3021641, BRAWH3021643, BRAWH3021724, BRAWH3022347, BRAWH3022431, BRAWH3022459, BRAWH3022542,
BRAWH3022719, BRAWH3022866, BRAWH3022900, BRAWH3023156, BRAWH3023168, BRAWH3023172, BRAWH3023274, BRAWH3023421, BRAWH3024231, BRAWH3024242, BRAWH3024469, BRAWH3024506, BRAWH3024613, BRAWH3024989, BRAWH3026349, BRAWH3026394, BRAWH3027420, BRAWH3027440, BRAWH3027533, BRAWH3027574, BRAWH3027607, BRAWH3027616, BRAWH3027675, BRAWH3027806, BRAWH3027880, BRAWH3027927, BRAWH3028202, BRAWH3028223, BRAWH3028306, BRAWH3028461, BRAWH3028645, BRAWH3028754, BRAWH3028796, BRAWH3029254, BRAWH3029313, BRAWH3029538, BRAWH3029556, BRAWH3030613, BRAWH3030772, BRAWH3030810, BRAWH3030892, BRAWH3030910, BRAWH3030950, BRAWH3031054, BRAWH3031710, BRAWH3031902, BRAWH3032298, BRAWH3032340, BRAWH3032571, BRAWH3032620,
BRAWH3032930, BRAWH3033117, BRAWH3033293, BRAWH3033448, BRAWH3033513, BRAWH3034097, BRAWH3034114, BRAWH3034134, BRAWH3034668, BRAWH3034743, BRAWH3034775, BRAWH3034890, BRAWH3035154, BRAWH3035403, BRAWH3035904, BRAWH3035914, BRAWH3035936, BRAWH3036077, BRAWH3036247, BRAWH3036270, BRAWH3036561, BRAWH3036738, BRAWH3036951, BRAWH3037265, BRAWH3037394, BRAWH3037533, BRAWH3037979, BRAWH3038055, BRAWH3038230, BRAWH3038252, BRAWH3038324, BRCAN1000065, BRCAN1000076, BRCAN1000105, BRCAN1000149, BRCAN1000168, BRCAN2000148, BRCAN2000149, BRCAN2000209, BRCAN2000239, BRCAN2000346, BRCAN2000364, BRCAN2000383, BRCAN2000418, BRCAN2000523, BRCAN2000541, BRCAN2000563, BRCAN2000577, BRCAN2000620, BRCAN2000639,
BRCAN2000763, BRCAN2000832, BRCAN2000923, BRCAN2001223, BRCAN2001866, BRCAN2002173, BRCAN2002416, BRCAN2002473, BRCAN2002562, BRCAN2002662, BRCAN2002826, BRCAN2002854, BRCAN2002856, BRCAN2002892, BRCAN2002944, BRCAN2002948, BRCAN2003070, BRCAN2003269, BRCAN2003389, BRCAN2003628, BRCAN2003703, BRCAN2003746, BRCAN2003814, BRCAN2003987, BRCAN2004173, BRCAN2004355, BRCAN2004371, BRCAN2004653, BRCAN2004699, BRCAN2005436, BRCAN2005449, BRCAN2006019, BRCAN2006051, BRCAN2006063, BRCAN2006117, BRCAN2006174, BRCAN2006290, BRCAN2006297, BRCAN2006323, BRCAN2006401, BRCAN2006411, BRCAN2006450, BRCAN2006955, BRCAN2007119, BRCAN2007144, BRCAN2007409, BRCAN2007426, BRCAN2007525, BRCAN2007700, BRCAN2008494,
BRCAN2008528, BRCAN2008701, BRCAN2008894, BRCAN2009156, BRCAN2009168, BRCAN2009203, BRCAN2010374, BRCAN2010376, BRCAN2010547, BRCAN2010581, BRCAN2010665, BRCAN2011254, BRCAN2011316, BRCAN2011602, BRCAN2012110, BRCAN2012355, BRCAN2012402, BRCAN2012408, BRCAN2012481, BRCAN2012613, BRCAN2012713, BRCAN2012929, BRCAN2013655, BRCAN2013660, BRCAN2014143, BRCAN2014229, BRCAN2014370, BRCAN2014602, BRCAN2014652, BRCAN2014788, BRCAN2014881, BRCAN2014952, BRCAN2015371, BRCAN2015402, BRCAN2015464, BRCAN2015719, BRCAN2015757, BRCAN2015886, BRCAN2016025, BRCAN2016046, BRCAN2016174, BRCAN2016433, BRCAN2016619, BRCAN2016762, BRCAN2016814, BRCAN2017442, BRCAN2017905, BRCAN2018240, BRCAN2018269, BRCAN2018490,
BRCAN2018566, BRCAN2018667, BRCAN2018748, BRCAN2018935, BRCAN2019002, BRCAN2019387, BRCAN2019653, BRCAN2019773, BRCAN2019907, BRCAN2020234, BRCAN2020331, BRCAN2020412, BRCAN2020467, BRCAN2020710, BRCAN2020880, BRCAN2021028, BRCAN2021325, BRCAN2021452, BRCAN2021669, BRCAN2021718, BRCAN2022191, BRCAN2022472, BRCAN2022474, BRCAN2023347, BRCAN2023724, BRCAN2024451, BRCAN2024563, BRCAN2025009, BRCAN2025093, BRCAN2025349, BRCAN2025368, BRCAN2025712, BRCAN2025982, BRCAN2026117, BRCAN2026197, BRCAN2026340, BRCAN2026711, BRCAN2027150, BRCAN2027310, BRCAN2027334, BRCAN2027355, BRCAN2027364, BRCAN2027513, BRCAN2027593, BRCAN2027970, BRCAN2028021, BRCAN2028036, BRCAN2028040, BRCAN2028319, BRCAN2028338,
BRCAN2028378, BRCAN2028460, BRCAN2028545, BRCAN2028634, BRCAN2028702, BRCOC1000087, BRCOC1000094, BRCOC2000004, BRCOC2000047, BRCOC2000152, BRCOC2000184, BRCOC2000186, BRCOC2000360, BRCOC2000404, BRCOC2000487, BRCOC2000637, BRCOC2000670, BRCOC2000816, BRCOC2000850, BRCOC2001355, BRCOC2002085, BRCOC2002155, BRCOC2002323, BRCOC2002659, BRCOC2002664, BRCOC2002686, BRCOC2002751, BRCOC2002756, BRCOC2002777, BRCOC2002835, BRCOC2003100, BRCOC2003125, BRCOC2003213, BRCOC2003513, BRCOC2003732, BRCOC2003740, BRCOC2004036, BRCOC2004175, BRCOC2004354, BRCOC2004455, BRCOC2004950, BRCOC2005542, BRCOC2005903, BRCOC2005951, BRCOC2006164, BRCOC2006189, BRCOC2006243, BRCOC2006270, BRCOC2006639, BRCOC2006942,
BRCOC2007034, BRCOC2007593, BRCOC2007733, BRCOC2007769, BRCOC2008064, BRCOC2008225, BRCOC2008642, BRCOC2008993, BRCOC2009052, BRCOC2009196, BRCOC2009380, BRCOC2009638, BRCOC2009937, BRCOC2010115, BRCOC2010123, BRCOC2010510, BRCOC2010980, BRCOC2010994, BRCOC2011010, BRCOC2011398, BRCOC2011418, BRCOC2011506, BRCOC2011769, BRCOC2011996, BRCOC2012229, BRCOC2012386, BRCOC2012813, BRCOC2013448, BRCOC2013573, BRCOC2013675, BRCOC2013780, BRCOC2014013, BRCOC2014033, BRCOC2014400, BRCOC2014748, BRCOC2014833, BRCOC2015824, BRCOC2015908, BRCOC2015944, BRCOC2016525, BRCOC2016661, BRCOC2017061, BRCOC2017652, BRCOC2017856, BRCOC2018775, BRCOC2018877, BRCOC2019255, BRCOC2019549, BRCOC2019841, BRCOC2019896,
BRCOC2019903, BRCOC2019910, BRCOC2019934, BRCOC2020122, BRCOC2020142, BRHIP1000058, BRHIP1000072, BRHIP1000104, BRHIP1000108, BRHIP1000129, BRHIP1000174, BRHIP1000258, BRHIP2000021, BRHIP2000079, BRHIP2000087, BRHIP2000163, BRHIP2000210, BRHIP2000239, BRHIP2000245, BRHIP2000312, BRHIP2000359, BRHIP2000445, BRHIP2000506, BRHIP2000532, BRHIP2000534, BRHIP2000553, BRHIP2000621, BRHIP2000691, BRHIP2000819, BRHIP2000826, BRHIP2000920, BRHIP2001074, BRHIP2001686, BRHIP2001782, BRHIP2001802, BRHIP2001805, BRHIP2001886, BRHIP2001927, BRHIP2002122, BRHIP2002143, BRHIP2002172, BRHIP2002339, BRHIP2002346, BRHIP2002510, BRHIP2002664, BRHIP2002722, BRHIP2002929, BRHIP2003048, BRHIP2003242, BRHIP2003272,
BRHIP2003748, BRHIP2003786, BRHIP2003828, BRHIP2003917, BRHIP2004312, BRHIP2004355, BRHIP2004359, BRHIP2004814, BRHIP2004883, BRHIP2004902, BRHIP2005010, BRHIP2005236, BRHIP2005271, BRHIP2005354, BRHIP2005600, BRHIP2005719, BRHIP2005724, BRHIP2005752, BRHIP2005932, BRHIP2006617, BRHIP2006782, BRHIP2006800, BRHIP2006819, BRHIP2006921, BRHIP2007307, BRHIP2007616, BRHIP2007690, BRHIP2007741, BRHIP2007928, BRHIP2007969, BRHIP2008269, BRHIP2008389, BRHIP2008607, BRHIP2008756, BRHIP2008909, BRHIP2009019, BRHIP2009177, BRHIP2009340, BRHIP2009351, BRHIP2009414, BRHIP2009474, BRHIP2009617, BRHIP2009685, BRHIP2010217, BRHIP2010309, BRHIP2010444, BRHIP2010593, BRHIP2010610, BRHIP2010723, BRHIP2010744,
BRHIP2011080, BRHIP2011120, BRHIP2011199, BRHIP2011508, BRHIP2011576, BRHIP2011783, BRHIP2011838, BRHIP2011891, BRHIP2011933, BRHIP2012054, BRHIP2012059, BRHIP2012141, BRHIP2012314, BRHIP2012360, BRHIP2012545, BRHIP2012580, BRHIP2012627, BRHIP2012642, BRHIP2012750, BRHIP2012922, BRHIP2012923, BRHIP2012972, BRHIP2013286, BRHIP2013447, BRHIP2013510, BRHIP2013699, BRHIP2013723, BRHIP2013958, BRHIP2013972, BRHIP2014063, BRHIP2014228, BRHIP2014285, BRHIP2014291, BRHIP2014373, BRHIP2014386, BRHIP2014387, BRHIP2014954, BRHIP2015153, BRHIP2015224; BRHIP2015356, BRHIP2015360, BRHIP2015679, BRHIP2016005, BRHIP2016125, BRHIP2016788, BRHIP2016968, BRHIP2016990, BRHIP2017010, BRHIP2017315, BRHIP2017385,
BRHIP2017404, BRHIP2017542, BRHIP2017553, BRHIP2017642, BRHIP2017714, BRHIP2017780, BRHIP2017945, BRHIP2018014, BRHIP2018369, BRHIP2018612, BRHIP2018635, BRHIP2018650, BRHIP2018719, BRHIP2018805, BRHIP2018998, BRHIP2019007, BRHIP2019047, BRHIP2019177, BRHIP2019186, BRHIP2019291, BRHIP2019494, BRHIP2019589, BRHIP2019641, BRHIP2019819, BRHIP2019884, BRHIP2019972, BRHIP2020182, BRHIP2020509, BRHIP2020573, BRHIP2020622, BRHIP2020695, BRHIP2020743, BRHIP2020842, BRHIP2020859, BRHIP2020930, BRHIP2021102, BRHIP2021289, BRHIP2021495, BRHIP2021615, BRHIP2021744, BRHIP2021858, BRHIP2021870, BRHIP2021929, BRHIP2022006, BRHIP2022054, BRHIP2022221, BRHIP2022298, BRHIP2022467, BRHIP2022708, BRHIP2022986,
BRHIP2023071, BRHIP2023229, BRHIP2023272, BRHIP2023292, BRHIP2023309, BRHIP2023369, BRHIP2023438, BRHIP2023695, BRHIP2023735, BRHIP2023762, BRHIP2023773, BRHIP2023860, BRHIP2023869, BRHIP2024046, BRHIP2024165, BRHIP2024201, BRHIP2024347, BRHIP2024398, BRHIP2024549, BRHIP2024789, BRHIP2024941, BRHIP2025245, BRHIP2025366, BRHIP2025448, BRHIP2025679, BRHIP2025739, BRHIP2025797, BRHIP2026021, BRHIP2026061, BRHIP2026155, BRHIP2026214, BRHIP2026288, BRHIP2026346, BRHIP2026557, BRHIP2026723, BRHIP2026877, BRHIP2027054, BRHIP2027077, BRHIP2027563, BRHIP2027685, BRHIP2027762, BRHIP2028187, BRHIP2028303, BRHIP2028330, BRHIP2028480, BRHIP2028583, BRHIP2028593, BRHIP2029176, BRHIP2029283, BRHIP2029393,
BRHIP2029529, BRHIP2029643, BRHIP2029663, BRHIP3000060, BRHIP3000070, BRHIP3000081, BRHIP3000111, BRHIP3000131, BRHIP3000240, BRHIP3000457, BRHIP3000475, BRHIP3000488, BRHIP3000526, BRHIP3000626, BRHIP3000859, BRHIP3000907, BRHIP3001079, BRHIP3001141, BRHIP3001283, BRHIP3001338, BRHIP3001360, BRHIP3001481, BRHIP3001878, BRHIP3001964, BRHIP3002114, BRHIP3002124, BRHIP3002363, BRHIP3002691, BRHIP3002920, BRHIP3002931, BRHIP3003063, BRHIP3003126, BRHIP3003306, BRHIP3003340, BRHIP3003395, BRHIP3003484, BRHIP3003644, BRHIP3003688, BRHIP3003795, BRHIP3003961, BRHIP3003984, BRHIP3004193, BRHIP3004215, BRHIP3004416, BRHIP3004710, BRHIP3004725, BRHIP3004774, BRHIP3004786, BRHIP3004863, BRHIP3004968,
BRHIP3005037, BRHIP3005137, BRHIP3005142, BRHIP3005231, BRHIP3005574, BRHIP3005673, BRHIP3005768, BRHIP3005801, BRHIP3005944, BRHIP3006279, BRHIP3006294, BRHIP3006390, BRHIP3006449, BRHIP3006683, BRHIP3006717, BRHIP3006786, BRHIP3006950, BRHIP3007172, BRHIP3007183, BRHIP3007195, BRHIP3007239, BRHIP3007291, BRHIP3007409, BRHIP3007424, BRHIP3007483, BRHIP3007609, BRHIP3007640, BRHIP3007960, BRHIP3008082, BRHIP3008183, BRHIP3008314, BRHIP3008320, BRHIP3008405, BRHIP3008598, BRHIP3008606, BRHIP3008691, BRHIP3008714, BRHIP3008997, BRHIP3009099, BRHIP3009140, BRHIP3009181, BRHIP3009318, BRHIP3009427, BRHIP3009448, BRHIP3009672, BRHIP3009753, BRHIP3010135, BRHIP3010289, BRHIP3010529, BRHIP3011082,
BRHIP3011241, BRHIP3011269, BRHIP3011460, BRHIP3011567, BRHIP3011831, BRHIP3012185, BRHIP3012289, BRHIP3012357, BRHIP3012736, BRHIP3012745, BRHIP3012868, BRHIP3013078, BRHIP3013271,.BRHIP3013588, BRHIP3013698, BRHIP3013765, BRHIP3013897, BRHIP3014237, BRHIP3014675, BRHIP3015620, BRHIP3015751, BRHIP3015854, BRHIP3016032, BRHIP3016213, BRHIP3016302, BRHIP3016421, BRHIP3017109, BRHIP3017146, BRHIP3017256, BRHIP3017325, BRHIP3017558, BRHIP3017637, BRHIP3018278, BRHIP3018784, BRHIP3019643, BRHIP3019824, BRHIP3019880, BRHIP3019956, BRHIP3020155, BRHIP3020182, BRHIP3020733, BRHIP3021019, BRHIP3021499, BRHIP3021534, BRHIP3021778, BRHIP3021987, BRHIP3022656, BRHIP3023922, BRHIP3024118, BRHIP3024533,
BRHIP3024703, BRHIP3024725, BRHIP3024820, BRHIP3025161, BRHIP3025702, BRHIP3025795, BRHIP3025844, BRHIP3026052, BRHIP3026097, BRHIP3026231, BRHIP3026335, BRHIP3026651, BRHIP3027105, BRHIP3027137, BRHIP3027160, BRHIP3027191, BRHIP3027501, BRHIP3027594, BRHIP3027651, BRHIP3027854, BRHIP3027947, BRHIP3028570, BRHIP3028742, BRSSN1000056, BRSSN1000059, BRSSN1000092, BRSSN1000153, BRSSN2000097, BRSSN2000175, BRSSN2000197, BRSSN2000244, BRSSN2000293, BRSSN2000312, BRSSN2000518, BRSSN2000561, BRSSN2000566, BRSSN2000684, BRSSN2000715, BRSSN2000832, BRSSN2001275, BRSSN2001342, BRSSN2001426, BRSSN2001503, BRSSN2001579, BRSSN2001758, BRSSN2001810, BRSSN2002857, BRSSN2002859, BRSSN2003012, BRSSN2003086,
BRSSN2003093, BRSSN2003820, BRSSN2003841, BRSSN2003937, BRSSN2004142, BRSSN2004303, BRSSN2004304, BRSSN2004496, BRSSN2004657, BRSSN2004679, BRSSN2004686, BRSSN2004710, BRSSN2004719, BRSSN2005847, BRSSN2006133, BRSSN2006390, BRSSN2006575, BRSSN2006611, BRSSN2006644, BRSSN2007076, BRSSN2007168, BRSSN2007202, BRSSN2007338, BRSSN2007464, BRSSN2007504, BRSSN2007819, BRSSN2008125, BRSSN2008419, BRSSN2008464, BRSSN2008549, BRSSN2008797, BRSSN2009119, BRSSN2009378, BRSSN2009389, BRSSN2009395, BRSSN2009627, BRSSN2009630, BRSSN2009702, BRSSN2010110, BRSSN2010587, BRSSN2010596, BRSSN2010830, BRSSN2011262, BRSSN2011653, BRSSN2011738, BRSSN2011843, BRSSN2012081, BRSSN2012103, BRSSN2012157, BRSSN2012198,
BRSSN2012254, BRSSN2012439, BRSSN2012622, BRSSN2012691, BRSSN2012838, BRSSN2013095, BRSSN2013544, BRSSN2013696, BRSSN2013702, BRSSN2013733, BRSSN2013753, BRSSN2013874, BRSSN2014112, BRSSN2014294, BRSSN2014299, BRSSN2014424, BRSSN2014513, BRSSN2014556, BRSSN2014573, BRSSN2014610, BRSSN2014636, BRSSN2015199, BRSSN2015238, BRSSN2015369, BRSSN2015493, BRSSN2015497, BRSSN2015554, BRSSN2015707, BRSSN2015773, BRSSN2015907, BRSSN2015982, BRSSN2016589, BRSSN2016768, BRSSN2016905, BRSSN2017297, BRSSN2017530, BRSSN2017682, BRSSN2017757, BRSSN2018218, BRSSN2018440, BRSSN2018447, BRSSN2018581, BRSSN2018731, BRSSN2018771, BRSSN2018925, BRSTN1000083, BRSTN1000097, BRSTN2000058, BRSTN2000070, BRSTN2000220,
BRSTN2000312, BRSTN2000367, BRSTN2000536, BRSTN2000872, BRSTN2001613, BRSTN2001801, BRSTN2002105, BRSTN2002400, BRSTN2002532, BRSTN2002691, BRSTN2003590, BRSTN2003835, BRSTN2004863, BRSTN2004869, BRSTN2004987, BRSTN2005721, BRSTN2006306, BRSTN2006466, BRSTN2006580, BRSTN2006583, BRSTN2006638, BRSTN2006865, BRSTN2007000, BRSTN2007118, BRSTN2007284, BRSTN2007765, BRSTN2008052, BRSTN2008283, BRSTN2008367, BRSTN2008418, BRSTN2008457, BRSTN2008475, BRSTN2008930, BRSTN2009247, BRSTN2010107, BRSTN2010363, BRSTN2010416, BRSTN2010500, BRSTN2010569, BRSTN2010750, BRSTN2010923, BRSTN2011211, BRSTN2011597, BRSTN2011688, BRSTN2011799, BRSTN2012089, BRSTN2012284, BRSTN2012320, BRSTN2012380, BRSTN2012451,
BRSTN2013171, BRSTN2013354, BRSTN2014490, BRSTN2014633, BRSTN2014751, BRSTN2014770, BRSTN2015015, BRSTN2015889, BRSTN2015978, BRSTN2016066, BRSTN2016335, BRSTN2016678, BRSTN2016918, BRSTN2016992, BRSTN2017104, BRSTN2017151, BRSTN2017237, BRSTN2017379, BRSTN2017761, BRSTN2017771, BRSTN2017995, BRSTN2018077, BRSTN2018083, BRSTN2018596, BRSTN2018692, BRSTN2018712, BRSTN2019079, BRSTN2019129, BRTHA1000021, BRTHA1000063, BRTHA1000311, BRTHA2000474, BRTHA2000514, BRTHA2000574, BRTHA2000855, BRTHA2000969, BRTHA2000972, BRTHA2001304, BRTHA2001370, BRTHA2001462, BRTHA2001717, BRTHA2001812, BRTHA2002091, BRTHA2002115, BRTHA2002133, BRTHA2002281, BRTHA2002376, BRTHA2002442, BRTHA2002493, BRTHA2002565,
BRTHA2002608, BRTHA2002702, BRTHA2002721, BRTHA2002726, BRTHA2002741, BRTHA2002808, BRTHA2002896, BRTHA2002903, BRTHA2003030, BRTHA2003110, BRTHA2003116, BRTHA2003436, BRTHA2003759, BRTHA2003794, BRTHA2004371, BRTHA2004582, BRTHA2004629, BRTHA2004639, BRTHA2004642, BRTHA2004812, BRTHA2004821, BRTHA2004978, BRTHA2004992, BRTHA2005005, BRTHA2005013, BRTHA2005448, BRTHA2005579, BRTHA2005942, BRTHA2005956, BRTHA2006075, BRTHA2006146, BRTHA2006194, BRTHA2006232, BRTHA2006720, BRTHA2006735, BRTHA2006974, BRTHA2006975, BRTHA2007060, BRTHA2007122, BRTHA2007422, BRTHA2007603, BRTHA2007665, BRTHA2007998, BRTHA2008316, BRTHA2008335, BRTHA2008502, BRTHA2008513, BRTHA2008527, BRTHA2008535, BRTHA2008598,
BRTHA2008669, BRTHA2008955, BRTHA2009311, BRTHA2009349, BRTHA2009486, BRTHA2009803, BRTHA2009846, BRTHA2009886, BRTHA2009972, BRTHA2010008, BRTHA2010033, BRTHA2010073, BRTHA2010097, BRTHA2010191, BRTHA2010397, BRTHA2010672, BRTHA2010884, BRTHA2010907, BRTHA2011062, BRTHA2011194, BRTHA2011321, BRTHA2011351, BRTHA2011500, BRTHA2011641, BRTHA2011691, BRTHA2012129, BRTHA2012189, BRTHA2012351, BRTHA2012392, BRTHA2012466, BRTHA2012555, BRTHA2012562, BRTHA2012980, BRTHA2013054, BRTHA2013262, BRTHA2013275, BRTHA2013426, BRTHA2013460, BRTHA2013515, BRTHA2013540, BRTHA2013595, BRTHA2013610, BRTHA2013707, BRTHA2013942, BRTHA2014287, BRTHA2014334, BRTHA2014381, BRTHA2014578, BRTHA2014647, BRTHA2014663,
BRTHA2014792, BRTHA2014828, BRTHA2014909, BRTHA2014975, BRTHA2014997, BRTHA2015406, BRTHA2015478, BRTHA2015696, BRTHA2015700, BRTHA2015853, BRTHA2015878, BRTHA2016179, BRTHA2016215, BRTHA2016267, BRTHA2016318, BRTHA2016496, BRTHA2016543, BRTHA2016552, BRTHA2016577, BRTHA2016765, BRTHA2016918, BRTHA2017353, BRTHA2017364, BRTHA2017972, BRTHA2017985, BRTHA2018011, BRTHA2018129, BRTHA2018165, BRTHA2018304, BRTHA2018344, BRTHA2018420, BRTHA2018443, BRTHA2018504, BRTHA2018558, BRTHA2018591, BRTHA2018624, BRTHA2018706, BRTHA2018707, BRTHA2019014, BRTHA2019022, BRTHA2019048, BRTHA2019073, BRTHA2019726, BRTHA2019743, BRTHA2020400, BRTHA2020566, BRTHA2020642, BRTHA2020695, BRTHA2020721, BRTHA2020781,
BRTHA2020811, BRTHA2020910, BRTHA2021212, BRTHA2021440, BRTHA2021450, BRTHA2022074, BRTHA2022914, BRTHA2022968, BRTHA2023402, BRTHA2023437, BRTHA2024177, BRTHA2024354, BRTHA2024712, BRTHA2025869, BRTHA2026071, BRTHA2026290, BRTHA2026311, BRTHA2027227, BRTHA2027229, BRTHA2027250, BRTHA2027546, BRTHA2028297, BRTHA2029969, BRTHA2030036, BRTHA2030213, BRTHA2031517, BRTHA2031800, BRTHA2031917, BRTHA2032763, BRTHA2032845, BRTHA2033122, BRTHA2033155, BRTHA2033264, BRTHA2033320, BRTHA2033469, BRTHA2033683, BRTHA2034281, BRTHA2034576, BRTHA2034874, BRTHA2035448, BRTHA2035743, BRTHA2036055, BRTHA2036295, BRTHA2036660, BRTHA2037247, BRTHA2038279, BRTHA2038345, BRTHA2038353; BRTHA2038396, BRTHA3000252,
BRTHA3000273, BRTHA3000296, BRTHA3000297, BRTHA3000325, BRTHA3000456, BRTHA3000633, BRTHA3000789, BRTHA3001221, BRTHA3001623, BRTHA3002265, BRTHA3002401, BRTHA3002411, BRTHA3002427, BRTHA3002864, BRTHA3002933, BRTHA3002955, BRTHA3003000, BRTHA3003225, BRTHA3003339, BRTHA3003343, BRTHA3003417, BRTHA3003449, BRTHA3003474, BRTHA3003490, BRTHA3003704, BRTHA3004432, BRTHA3004475, BRTHA3004502, BRTHA3004610, BRTHA3005046, BRTHA3005735, BRTHA3005988, BRTHA3006318, BRTHA3006593, BRTHA3006856, BRTHA3007113, BRTHA3007130, BRTHA3007148, BRTHA3007319, BRTHA3007469, BRTHA3007472, BRTHA3007532, BRTHA3007662, BRTHA3007685, BRTHA3007769, BRTHA3007922, BRTHA3008143, BRTHA3008293, BRTHA3008310, BRTHA3008386,
BRTHA3008520, BRTHA3008608, BRTHA3008694, BRTHA3008778, BRTHA3008826, BRTHA3009037, BRTHA3009090, BRTHA3009291, BRTHA3009858, BRTHA3010135, BRTHA3010366, BRTHA3010469, BRTHA3010530, BRTHA3010540, BRTHA3010717, BRTHA3011149, BRTHA3011187, BRTHA3011194, BRTHA3011229, BRTHA3011361, BRTHA3011510, BRTHA3011892, BRTHA3012265, BRTHA3013860, BRTHA3013882, BRTHA3013884, BRTHA3013981, BRTHA3014000, BRTHA3014105, BRTHA3014507, BRTHA3014547, BRTHA3014691, BRTHA3014835, BRTHA3014854, BRTHA3014908, BRTHA3014920, BRTHA3015815, BRTHA3016616, BRTHA3016835, BRTHA3016845, BRTHA3016917, BRTHA3017047, BRTHA3017589, BRTHA3017848, BRTHA3018409, BRTHA3018514, BRTHA3018617, BRTHA3018623, BRTHA3018656, BRTHA3019048,
BRTHA3019105, BRTHA3019183, BRTHA3020000, BRTHA3020369, BRTHA3021569, BRTHA3021708, BRTHA3021786, BRTHA3021971, BRTHA3022641, BRTHA3023523, BRTHA3023590, BRTHA3023929, BRTHA3024233, BRTHA3024600, BRTHA3025073, BRTHA3025789, BRTHA3025826, BRTHA3026161, BRTHA3026180, BRTHA3026556, BRTHA3026916, BRTHA3027171, BRTHA3027318, BRTHA3027638, BRTHA3027820, BRTHA3027957, BRTHA3028339, BRTHA3028505, BRTHA3028782, CD34C2000051, CD34C2000152, CD34C2000175, CD34C2000443, CD34C3000125, CD34C3000252, CD34C3000314, CD34C3000424, CD34C3000494, CERVX1000042, CERVX2000812, CERVX2000968, CERVX2002006, CERVX2002013, CERVX2002430, CERVX2002770, CHONS1000058, CHONS2000172, CHONS2000797, CHONS2001287, CHONS2001382,
CHONS2001797, CHONS2001834, CHONS2002419, COLON1000030, COLON1000084, COLON1000135, COLON2000470, COLON2000557, COLON2000568, COLON2001721, COLON2001829, COLON2001866, COLON2002443, COLON2002520, COLON2003043, COLON2003529, COLON2004318, COLON2004351, COLON2004478, COLON2004911, COLON2005126, COLON2005623, COLON2005735, COLON2005772, COLON2006263, COLON2006282, COLON2006417, COLON2007274, COLON2007733, COLON2008105, COLON2009337, COLON2009499, CORDB1000140, CORDB2000061, CORDB2000127, CORDB2000541, CTONG1000009, CTONG1000010, CTONG1000020, CTONG1000022, CTONG1000040, CTONG1000056, CTONG1000062, CTONG1000086, CTONG1000088, CTONG1000093, CTONG1000097, CTONG1000113, CTONG1000165, CTONG1000175,
CTONG1000181, CTONG1000218, CTONG1000220, CTONG1000283, CTONG1000288, CTONG1000302, CTONG1000341, CTONG1000476, CTONG1000488, CTONG1000508, CTONG1000540, CTONG2000031, CTONG2000034, CTONG2000042, CTONG2000063, CTONG2000201, CTONG2000218, CTONG2000279, CTONG2000303, CTONG2000330, CTONG2000395, CTONG2000452, CTONG2000465, CTONG2000469, CTONG2000480, CTONG2000561, CTONG2000589, CTONG2000766, CTONG2000771, CTONG2000819, CTONG2000827, CTONG2000846, CTONG2000855, CTONG2000977, CTONG2001139, CTONG2001226, CTONG2001320, CTONG2001366, CTONG2001413, CTONG2001428, CTONG2001513, CTONG2001524, CTONG2001533, CTONG2001598, CTONG2001681, CTONG2001724, CTONG2001749, CTONG2001800, CTONG2001820, CTONG2001858,
CTONG2001877, CTONG2001911, CTONG2001955, CTONG2002066, CTONG2002095, CTONG2002143, CTONG2002395, CTONG2002417, CTONG2002418, CTONG2002453, CTONG2002520, CTONG2002558, CTONG2002590, CTONG2002709, CTONG2002721, CTONG2002816, CTONG2002820, CTONG2002832, CTONG2002841, CTONG2002903, CTONG2002965, CTONG2002999, CTONG2003028, CTONG2003094, CTONG2003100, CTONG2003115, CTONG2003189, CTONG2003245, CTONG2003293, CTONG2003350, CTONG2003361, CTONG2003372, CTONG2003375, CTONG2003427, CTONG2003599, CTONG2003680, CTONG2003699, CTONG2003764, CTONG2003782, CTONG2003889, CTONG2003937, CTONG2003945, CTONG2004000, CTONG2004062, CTONG2004115, CTONG2004126, CTONG2004264, CTONG2004305, CTONG2004397, CTONG2004454,
CTONG2004487, CTONG2004550, CTONG2004669, CTONG2004716, CTONG2004912, CTONG2004941, CTONG2004948, CTONG2005028, CTONG2005049, CTONG2005110, CTONG2005265, CTONG2005278, CTONG2005290, CTONG2005374, CTONG2005399, CTONG2005468, CTONG2005553, CTONG2005567, CTONG2005585, CTONG2005635, CTONG2005775, CTONG2005795, CTONG2005890, CTONG2005904, CTONG2005913, CTONG2006004, CTONG2006129, CTONG2006223, CTONG2006273, CTONG2006310, CTONG2006393, CTONG2006412, CTONG2006449, CTONG2006515, CTONG2006524, CTONG2006568, CTONG2006611, CTONG2006620, CTONG2006666, CTONG2006691, CTONG2006709, CTONG2006798, CTONG2006836, CTONG2006942, CTONG2006964, CTONG2007007, CTONG2007009, CTONG2007072, CTONG2007091, CTONG2007104,
CTONG2007168, CTONG2007175, CTONG2007259, CTONG2007272, CTONG2007293, CTONG2007297, CTONG2007399, CTONG2007400, CTONG2007417, CTONG2007441, CTONG2007474, CTONG2007586, CTONG2007613, CTONG2007623, CTONG2007681, CTONG2007776, CTONG2007779, CTONG2007898, CTONG2007959, CTONG2007970, CTONG2008014, CTONG2008184, CTONG2008262, CTONG2008269, CTONG2008321, CTONG2008402, CTONG2008405, CTONG2008466, CTONG2008506, CTONG2008521, CTONG2008562, CTONG2008577, CTONG2008595, CTONG2008621, CTONG2008720, CTONG2008721, CTONG2008773, CTONG2008792, CTONG2008989, CTONG2009033, CTONG2009108, CTONG2009132, CTONG2009156, CTONG2009181, CTONG2009257, CTONG2009258, CTONG2009268, CTONG2009270, CTONG2009395, CTONG2009423,
CTONG2009440, CTONG2009527, CTONG2009531, CTONG2009534, CTONG2009570, CTONG2009572, CTONG2009604, CTONG2009643, CTONG2009675, CTONG2009689, CTONG2009725, CTONG2009766, CTONG2009768, CTONG2009844, CTONG2009871, CTONG2009887, CTONG2009891, CTONG2009955, CTONG2009958, CTONG2009963, CTONG2010018, CTONG2010024, CTONG2010116, CTONG2010133, CTONG2010148, CTONG2010276, CTONG2010330, CTONG2010508, CTONG2010566, CTONG2010623, CTONG2010652, CTONG2010803, CTONG2011188, CTONG2011429, CTONG2011474, CTONG2011676, CTONG2011716, CTONG2011765, CTONG2011779, CTONG2011815, CTONG2011822, CTONG2011920, CTONG2011985, CTONG2012001, CTONG2012029, CTONG2012050, CTONG2012077, CTONG2012101, CTONG2012123, CTONG2012158,
CTONG2012230, CTONG2012401, CTONG2012422, CTONG2012425, CTONG2012447, CTONG2012453, CTONG2012473, CTONG2012476, CTONG2012533, CTONG2012554, CTONG2012564, CTONG2012607, CTONG2012661, CTONG2012724, CTONG2012738, CTONG2012745, CTONG2012758, CTONG2012843, CTONG2012901, CTONG2013149, CTONG2013178, CTONG2013222, CTONG2013284, CTONG2013339, CTONG2013348, CTONG2013352, CTONG2013381, CTONG2013393, CTONG2013511, CTONG2013630, CTONG2013803, CTONG2013985, CTONG2013986, CTONG2014032, CTONG2014058, CTONG2014119, CTONG2014165, CTONG2014191, CTONG2014206, CTONG2014234, CTONG2014264, CTONG2014280, CTONG2014389, CTONG2014491, CTONG2014630, CTONG2014635, CTONG2014646, CTONG2014705, CTONG2014946, CTONG2014954,
CTONG2014959, CTONG2014966, CTONG2014995, CTONG2015091, CTONG2015204, CTONG2015316, CTONG2015330, CTONG2015358, CTONG2015434, CTONG2015518, CTONG2015540, CTONG2015596, CTONG2015633, CTONG2015678, CTONG2015717, CTONG2015804, CTONG2015815, CTONG2015866, CTONG2015914, CTONG2015953, CTONG2016185, CTONG2016217, CTONG2016355, CTONG2016499, CTONG2016505, CTONG2016559, CTONG2016658, CTONG2016775, CTONG2016797, CTONG2016824, CTONG2016846, CTONG2016904, CTONG2016931, CTONG2016942, CTONG2016953, CTONG2017021, CTONG2017292, CTONG2017409, CTONG2017444, CTONG2017458, CTONG2017500, CTONG2017604, CTONG2017650, CTONG2017798, CTONG2017804, CTONG2017814, CTONG2017910, CTONG2017939, CTONG2017989, CTONG2018062,
CTONG2018069, CTONG2018147, CTONG2018217, CTONG2018279, CTONG2018334, CTONG2018343, CTONG2018379, CTONG2018383, CTONG2018413, CTONG2018483, CTONG2018629, CTONG2018632, CTONG2018652, CTONG2018653, CTONG2018655, CTONG2018735, CTONG2018739, CTONG2018808, CTONG2018843, CTONG2018900, CTONG2018976, CTONG2019029, CTONG2019063, CTONG2019144, CTONG2019232, CTONG2019248, CTONG2019400, CTONG2019590, CTONG2019652, CTONG2019704, CTONG2019788, CTONG2019833, CTONG2020031, CTONG2020108, CTONG2020127, CTONG2020171, CTONG2020374, CTONG2020445, CTONG2020522, CTONG2020582, CTONG2020638, CTONG2020806, CTONG2020831, CTONG2020974, CTONG2021024, CTONG2021132, CTONG2021168, CTONG2021289, CTONG2022153, CTONG2022601,
CTONG2022835, CTONG2023021, CTONG2023449, CTONG2023512, CTONG2024206, CTONG2024614, CTONG2024749, CTONG2025496, CTONG2025513, CTONG2025516, CTONG2025610, CTONG2025900, CTONG2026513, CTONG2026770, CTONG2026920, CTONG2026987, CTONG2027150, CTONG2027327, CTONG2027361, CTONG2027591, CTONG2027736, CTONG2027783, CTONG2027828, CTONG2027959, CTONG2028097, CTONG2028208, CTONG2028309, CTONG2028486, CTONG2028518, CTONG2028687, CTONG2028758, CTONG3000657, CTONG3000686, CTONG3000707, CTONG3000896, CTONG3001420, CTONG3001501, CTONG3001560, CTONG3001605, CTONG3001746, CTONG3002020, CTONG3002127, CTONG3002370, CTONG3002412, CTONG3002433, CTONG3002552, CTONG3002588, CTONG3002674, CTONG3002728, CTONG3002835,
CTONG3002909, CTONG3002947, CTONG3002983, CTONG3003165, CTONG3003179, CTONG3003483, CTONG3003553, CTONG3003598, CTONG3003652, CTONG3003654, CTONG3003669, CTONG3003737, CTONG3003905, CTONG3003972, CTONG3004072, CTONG3004397, CTONG3004550, CTONG3004576, CTONG3004607, CTONG3004712, CTONG3004726, CTONG3005325, CTONG3005648, CTONG3005713, CTONG3005803, CTONG3005813, CTONG3006067, CTONG3006073, CTONG3006186, CTONG3006629, CTONG3006650, CTONG3006659, CTONG3007244, CTONG3007444, CTONG3007528, CTONG3007586, CTONG3007870, CTONG3008223, CTONG3008252, CTONG3008258, CTONG3008457, CTONG3008496, CTONG3008566, CTONG3008639, CTONG3008831, CTONG3008951, CTONG3008959, CTONG3009227, CTONG3009287, CTONG3009328,
CTONG3009385, D3OST1000066, D3OST1000109, D3OST1000238, D3OST1000267, D3OST1000270, D3OST2000184, D3OST2000618, D3OST2000654, D3OST2000734, D3OST2001328, D3OST2001669, D3OST2001670, D3OST2001788, D3OST2002068, D3OST2002182, D3OST2002223, D3OST2002262, D3OST2002272, D3OST2002417, D3OST2002436, D3OST2002648, D3OST2003024, D3OST2003331, D3OST2003607, D3OST2003797, D3OST2004637, D3OST3000169, D3OST3000195, D3OST3000291, D3OST3000388, D3OST3000493, D6OST2000127, D6OST2000169, D6OST2000245, D6OST2000358, D6OST2000507 D9OST2000031 D9OST2000278 D9OST2000440 D9OST2000869 D9OST2001319, D9OST2001433 D9OST2002397 D9OST2002673 D9OST2003106 D9OST2003108 D9OST2003137 D9OST2003397 D9OST2003580
D9OST2003594 D9OST2003762 D9OST2003989 D9OST2004018 D9OST2004073 D9OST2004417 DFNES1000003, DFNES1000083, DFNES1000107, DFNES1000150, DFNES2000143, DFNES2000146, DFNES2000153, DFNES2000212, DFNES2000268, DFNES2000292, DFNES2000335, DFNES2000432, DFNES2000457, DFNES2000471, DFNES2000526, DFNES2000913, DFNES2001091, DFNES2001096, DFNES2001108, DFNES2001177, DFNES2001404, DFNES2001564, DFNES2001565, DFNES2001647, DFNES2001800, DFNES2002220, DFNES2002509, DFNES2002550, DFNES2002588, DFNES2002817, DFNES2002966, DFNES2003081, DFNES2003192, DFNES2003255, DFNES2003742, DFNES2004346, DFNES2004354, DFNES2004371, DFNES2004383, DFNES2004608, DFNES2004684, DFNES2005266, DFNES2005527, DFNES2005540,
DFNES2005690, DFNES2005766, DFNES2005779, DFNES2005875, DFNES2006944, DFNES2007113, DFNES2007299, DFNES2007332, DFNES2007641, DFNES2007757, DFNES2008088, DFNES2008160, DFNES2008280, DFNES2008881, DFNES2009482, DFNES2010502, DFNES2011192, DFNES2011221, DFNES2011239, DFNES2011245, DFNES2011380, DFNES2011499, ERLTF1000021, ERLTF2000324, ERLTF2000465, FCBBF1000023, FCBBF1000025, FCBBF1000038, FCBBF1000053, FCBBF1000063, FCBBF1000069, FCBBF1000077, FCBBF1000115, FCBBF1000117, FCBBF1000118, FCBBF1000121, FCBBF1000125, FCBBF1000131, FCBBF1000139, FCBBF1000155, FCBBF1000157, FCBBF1000171, FCBBF1000174, FCBBF1000198, FCBBF1000206, FCBBF1000220, FCBBF1000232, FCBBF1000233, FCBBF1000243, FCBBF1000270,
FCBBF1000272, FCBBF1000280, FCBBF1000297, FCBBF1000305, FCBBF1000314, FCBBF1000322, FCBBF1000349, FCBBF1000374, FCBBF1000376, FCBBF1000377, FCBBF1000395, FCBBF1000397, FCBBF1000403, FCBBF1000437, FCBBF1000449, FCBBF1000476, FCBBF1000506, FCBBF1000509, FCBBF1000546, FCBBF1000550, FCBBF1000556, FCBBF1000563, FCBBF1000581, FCBBF1000582, FCBBF1000598, FCBBF1000604, FCBBF1000618, FCBBF1000627, FCBBF1000671, FCBBF1000675, FCBBF1000684, FCBBF1000686, FCBBF1000687, FCBBF1000691, FCBBF1000732, FCBBF1000748, FCBBF2000038, FCBBF2000094, FCBBF2000105, FCBBF2000123, FCBBF2000195, FCBBF2000199, FCBBF2000214, FCBBF2000232, FCBBF2000259, FCBBF2000276, FCBBF2000291, FCBBF2000362, FCBBF2000379, FCBBF2000473,
FCBBF2000502, FCBBF2000566, FCBBF2000576, FCBBF2000677, FCBBF2000678, FCBBF2000733, FCBBF2000782, FCBBF2000808, FCBBF2000815, FCBBF2000825, FCBBF2000940, FCBBF2000951, FCBBF2001088, FCBBF2001105, FCBBF2001116, FCBBF2001183, FCBBF2001188, FCBBF2001211, FCBBF2001238, FCBBF2001243, FCBBF2001299, FCBBF2001309, FCBBF2001400, FCBBF2001427, FCBBF2001480, FCBBF2001529, FCBBF2001538, FCBBF2001594, FCBBF2001672, FCBBF2001675, FCBBF2001718, FCBBF2001720, FCBBF2001817, FCBBF2001868, FCBBF2002044, FCBBF2002111, FCBBF2002281, FCBBF2002349, FCBBF2002370, FCBBF2002541, FCBBF2002626, FCBBF2002801, FCBBF2002897, FCBBF2002898, FCBBF2002904, FCBBF2002928, FCBBF2002953, FCBBF2003083, FCBBF2003102, FCBBF2003269,
FCBBF2003293, FCBBF2003297, FCBBF2003395, FCBBF2003528, FCBBF2003543, FCBBF2003549, FCBBF2003567, FCBBF2003636, FCBBF2003823, FCBBF2003895, FCBBF2004066, FCBBF2004090, FCBBF2004138, FCBBF2004217, FCBBF2004256, FCBBF2004373, FCBBF2004448, FCBBF2004671, FCBBF2004707, FCBBF2004788, FCBBF2005069, FCBBF2005122, FCBBF2005428, FCBBF2005439, FCBBF2005538, FCBBF2005637, FCBBF2005645, FCBBF2005658, FCBBF2005733, FCBBF2005909, FCBBF2005966, FCBBF2006131, FCBBF2006418, FCBBF2006452, FCBBF2006634, FCBBF2006781, FCBBF2007012, FCBBF2007186, FCBBF2007265, FCBBF2007302, FCBBF2007556, FCBBF2007610, FCBBF2007633, FCBBF2007840, FCBBF3000102, FCBBF3000110, FCBBF3000120, FCBBF3000168, FCBBF3000175, FCBBF3000227,
FCBBF3000228, FCBBF3000229, FCBBF3000233, FCBBF3000269, FCBBF3000361, FCBBF3000367, FCBBF3000388, FCBBF3000434, FCBBF3000462, FCBBF3000473, FCBBF3000501, FCBBF3000518, FCBBF3000550, FCBBF3000847, FCBBF3001018, FCBBF3001020, FCBBF3001081, FCBBF3001235, FCBBF3001281, FCBBF3001302, FCBBF3001377, FCBBF3001594, FCBBF3001632, FCBBF3001657, FCBBF3001818, FCBBF3001855, FCBBF3001890, FCBBF3001912, FCBBF3001914, FCBBF3001918, FCBBF3001924, FCBBF3001977, FCBBF3002163, FCBBF3002188, FCBBF3002190, FCBBF3002268, FCBBF3002475, FCBBF3002556, FCBBF3002592, FCBBF3002658, FCBBF3002782, FCBBF3002818, FCBBF3002925, FCBBF3003216, FCBBF3003305, FCBBF3003373, FCBBF3003435, FCBBF3003475, FCBBF3003481, FCBBF3003557,
FCBBF3003787, FCBBF3003902, FCBBF3004021, FCBBF3004041, FCBBF3004261, FCBBF3004323, FCBBF3004390, FCBBF3004409, FCBBF3004502, FCBBF3004715, FCBBF3004842, FCBBF3004955, FCBBF3005160, FCBBF3005218, FCBBF3005320, FCBBF3005330, FCBBF3005444, FCBBF3005497, FCBBF3005698, FCBBF3005729, FCBBF3006171, FCBBF3006249, FCBBF3006288, FCBBF3006399, FCBBF3006628, FCBBF3007077, FCBBF3007150, FCBBF3007152, FCBBF3007242, FCBBF3007248, FCBBF3007453, FCBBF3007462, FCBBF3007540, FCBBF3007597, FCBBF3007604, FCBBF3007631, FCBBF3007829, FCBBF3007855, FCBBF3007860, FCBBF3008073, FCBBF3008100, FCBBF3008159, FCBBF3008251, FCBBF3008311, FCBBF3008362, FCBBF3008382, FCBBF3008475, FCBBF3008556, FCBBF3008644, FCBBF3008689,
FCBBF3008748, FCBBF3008870, FCBBF3008938, FCBBF3008944, FCBBF3009069, FCBBF3009101, FCBBF3009152, FCBBF3009256, FCBBF3009317, FCBBF3009428, FCBBF3009464, FCBBF3009479, FCBBF3009526, FCBBF3009541, FCBBF3009703, FCBBF3009717, FCBBF3009888, FCBBF3009949, FCBBF3009978, FCBBF3010008, FCBBF3010012, FCBBF3010124, FCBBF3010130, FCBBF3010142, FCBBF3010149, FCBBF3010211, FCBBF3010361, FCBBF3010508, FCBBF3010544, FCBBF3010586, FCBBF3010601, FCBBF3010665, FCBBF3010729, FCBBF3010733, FCBBF3011003, FCBBF3011418, FCBBF3011485, FCBBF3011523, FCBBF3011570, FCBBF3011592, FCBBF3011867, FCBBF3011889, FCBBF3012170, FCBBF3012182, FCBBF3012288, FCBBF3012332, FCBBF3012347, FCBBF3012443, FCBBF3012588, FCBBF3012667,
FCBBF3012842, FCBBF3012901, FCBBF3013041, FCBBF3013058, FCBBF3013205, FCBBF3013266, FCBBF3013307, FCBBF3013341, FCBBF3013506, FCBBF3013589, FCBBF3013623, FCBBF3013800, FCBBF3013846, FCBBF3014229, FCBBF3014260, FCBBF3014355, FCBBF3014567, FCBBF3014770, FCBBF3015100, FCBBF3015119, FCBBF3015131, FCBBF3015317, FCBBF3015727, FCBBF3015758, FCBBF3015809, FCBBF3015870, FCBBF3016018, FCBBF3016134, FCBBF3016178, FCBBF3016451, FCBBF3016618, FCBBF3016622, FCBBF3016644, FCBBF3016667, FCBBF3016987, FCBBF3017024, FCBBF3017059, FCBBF3017071, FCBBF3017123, FCBBF3017233, FCBBF3017255, FCBBF3017288, FCBBF3017535, FCBBF3017681, FCBBF3017729, FCBBF3017873, FCBBF3017918, FCBBF3017974, FCBBF3018067, FCBBF3018173,
FCBBF3018453, FCBBF3018591, FCBBF3018753, FCBBF3018826, FCBBF3018949, FCBBF3019085, FCBBF3019320, FCBBF3019437, FCBBF3019455, FCBBF3019564, FCBBF3019569, FCBBF3019570, FCBBF3019663, FCBBF3019716, FCBBF3019784, FCBBF3019867, FCBBF3019961, FCBBF3020030, FCBBF3020138, FCBBF3020140, FCBBF3020163, FCBBF3020232, FCBBF3020599, FCBBF3021026, FCBBF3021191, FCBBF3021221, FCBBF3021501, FCBBF3021506, FCBBF3021524, FCBBF3021940, FCBBF3021985, FCBBF3022005, FCBBF3022291, FCBBF3022311, FCBBF3022504, FCBBF3022566, FCBBF3022593, FCBBF3022765, FCBBF3022767, FCBBF3022894, FCBBF3023061, FCBBF3023078, FCBBF3023368, FCBBF3023372, FCBBF3023443, FCBBF3023599, FCBBF3023704, FCBBF3023887, FCBBF3023895, FCBBF3024096,
FCBBF3024225, FCBBF3024266, FCBBF3024364, FCBBF3024793, FCBBF3024911, FCBBF3025098, FCBBF3025252, FCBBF3025280, FCBBF3025285, FCBBF3025417, FCBBF3025650, FCBBF3025674, FCBBF3025730, FCBBF3026021, FCBBF3026048, FCBBF3026251, FCBBF3026308, FCBBF3026651, FCBBF3026678, FCBBF3026692, FCBBF3027104, FCBBF3027199, FCBBF3027328, FCBBF3027559, FCBBF3027717, FCBBF3027755, FCBBF3027768, FCBBF3027854, FCBBF3027863, FCBBF3027903, FCBBF3028102, FCBBF3028143, FCBBF3028188, FCBBF3028202, FCBBF3028244, FCBBF3028472, FCBBF3028528, FCBBF3028582, FCBBF3028593, FCBBF3028671, FCBBF3028794, FCBBF4000017, FCBBF4000061, FCBBF4000076, FCBBF4000173, FCBBF4000192, FCBBF4000193, FCBBF4000268, FCBBF4000282, FCBBF4000399,
FCBBF4000415, FCBBF4000500, FCBBF4000529, FCBBF4000548, FCBBF5000041, FCBBF5000061, FCBBF5000165, FCBBF5000325, FCBBF5000353, FCBBF5000384, FCBBF5000483, FCBBF5000495, FEBRA1000005, FEBRA1000008, FEBRA1000030, FEBRA1000057, FEBRA1000138, FEBRA1000148, FEBRA1000149, FEBRA1000183, FEBRA1000188, FEBRA1000189, FEBRA1000190, FEBRA2000007, FEBRA2000021, FEBRA2000035, FEBRA2000053, FEBRA2000126, FEBRA2000220, FEBRA2000228, FEBRA2000253, FEBRA2000282, FEBRA2000286, FEBRA2000297, FEBRA2000311, FEBRA2000321, FEBRA2000333, FEBRA2000377, FEBRA2000378, FEBRA2000391, FEBRA2000394, FEBRA2000397, FEBRA2000399, FEBRA2000402, FEBRA2000404, FEBRA2000409, FEBRA2000415, FEBRA2000436, FEBRA2000438, FEBRA2000452,
FEBRA2000454, FEBRA2000468, FEBRA2000491, FEBRA2000504, FEBRA2000532, FEBRA2000538, FEBRA2000575, FEBRA2000656, FEBRA2000659, FEBRA2000690, FEBRA2000727, FEBRA2000734, FEBRA2000762, FEBRA2000771, FEBRA2000772, FEBRA2000782, FEBRA2000787, FEBRA2000790, FEBRA2000793, FEBRA2000803, FEBRA2000805, FEBRA2000809, FEBRA2000815, FEBRA2000862, FEBRA2000877, FEBRA2000881, FEBRA2000901, FEBRA2000909, FEBRA2000917, FEBRA2000928, FEBRA2000936, FEBRA2000937, FEBRA2000959, FEBRA2000972, FEBRA2001004, FEBRA2001012, FEBRA2001020, FEBRA2001093, FEBRA2001124, FEBRA2001133, FEBRA2001146, FEBRA2001161, FEBRA2001175, FEBRA2001197, FEBRA2001217, FEBRA2001227, FEBRA2001245, FEBRA2001267, FEBRA2001294, FEBRA2001334,
FEBRA2001351, FEBRA2001383, FEBRA2001438, FEBRA2001440, FEBRA2001487, FEBRA2001492, FEBRA2001523, FEBRA2001561, FEBRA2001571, FEBRA2001581, FEBRA2001584, FEBRA2001590, FEBRA2001591, FEBRA2001610,. FEBRA2001620, FEBRA2001659, FEBRA2001669, FEBRA2001698, FEBRA2001705, FEBRA2001706, FEBRA2001715, FEBRA2001752, FEBRA2001772, FEBRA2001790, FEBRA2001814, FEBRA2001867, FEBRA2001914, FEBRA2001974, FEBRA2001989, FEBRA2001990, FEBRA2002009, FEBRA2002027, FEBRA2002086, FEBRA2002109, FEBRA2002191, FEBRA2002260, FEBRA2002352, FEBRA2002399, FEBRA2002410, FEBRA2002429, FEBRA2002442, FEBRA2002456, FEBRA2002508, FEBRA2002525, FEBRA2002552, FEBRA2002574, FEBRA2002601, FEBRA2002611, FEBRA2002628, FEBRA2002662,
FEBRA2002682, FEBRA2002707, FEBRA2002727, FEBRA2002781, FEBRA2002783, FEBRA2002792, FEBRA2002818, FEBRA2002858, FEBRA2002882, FEBRA2002897, FEBRA2002908, FEBRA2002931, FEBRA2002933, FEBRA2002962, FEBRA2002986, FEBRA2003054, FEBRA2003100, FEBRA2003115, FEBRA2003128, FEBRA2003155, FEBRA2003163, FEBRA2003181, FEBRA2003198, FEBRA2003253, FEBRA2003258, FEBRA2003275, FEBRA2003308, FEBRA2003327, FEBRA2003429, FEBRA2003454, FEBRA2003468, FEBRA2003470, FEBRA2003520, FEBRA2003524, FEBRA2003541, FEBRA2003675, FEBRA2003707, FEBRA2003711, FEBRA2003726, FEBRA2003822, FEBRA2003897, FEBRA2003907, FEBRA2003930, FEBRA2004023, FEBRA2004026, FEBRA2004029, FEBRA2004053, FEBRA2004056, FEBRA2004091, FEBRA2004110,
FEBRA2004191, FEBRA2004202, FEBRA2004219, FEBRA2004224, FEBRA2004237, FEBRA2004268, FEBRA2004293, FEBRA2004325, FEBRA2004329, FEBRA2004331, FEBRA2004443, FEBRA2004485, FEBRA2004490, FEBRA2004538, FEBRA2004592, FEBRA2004620, FEBRA2004628, FEBRA2004651, FEBRA2004767, FEBRA2004813, FEBRA2004818, FEBRA2004840, FEBRA2004867, FEBRA2004914, FEBRA2004933, FEBRA2004987, FEBRA2005014, FEBRA2005036, FEBRA2005079, FEBRA2005114, FEBRA2005216, FEBRA2005291, FEBRA2005361, FEBRA2005377, FEBRA2005380, FEBRA2005416, FEBRA2005427, FEBRA2005458, FEBRA2005476, FEBRA2005572, FEBRA2005701, FEBRA2005726, FEBRA2005734, FEBRA2005778, FEBRA2005787, FEBRA2005788, FEBRA2005824, FEBRA2005995, FEBRA2005998, FEBRA2006055,
FEBRA2006061, FEBRA2006112, FEBRA2006150, FEBRA2006165, FEBRA2006255, FEBRA2006270, FEBRA2006315, FEBRA2006346, FEBRA2006354, FEBRA2006357, FEBRA2006368, FEBRA2006370, FEBRA2006372, FEBRA2006379, FEBRA2006396, FEBRA2006401, FEBRA2006409, FEBRA2006418, FEBRA2006427, FEBRA2006476, FEBRA2006485, FEBRA2006491, FEBRA2006519, FEBRA2006521, FEBRA2006553, FEBRA2006627, FEBRA2006664, FEBRA2006667, FEBRA2006726, FEBRA2006736, FEBRA2006793, FEBRA2006808, FEBRA2006817, FEBRA2006873, FEBRA2006890, FEBRA2006942, FEBRA2006977, FEBRA2007017, FEBRA2007176, FEBRA2007200, FEBRA2007212, FEBRA2007247, FEBRA2007280, FEBRA2007414, FEBRA2007436, FEBRA2007449, FEBRA2007458, FEBRA2007534, FEBRA2007544, FEBRA2007551,
FEBRA2007566, FEBRA2007589, FEBRA2007613, FEBRA2007620, FEBRA2007622, FEBRA2007708, FEBRA2007793, FEBRA2007801, FEBRA2007823, FEBRA2007880, FEBRA2007900, FEBRA2007931, FEBRA2007957, FEBRA2008009, FEBRA2008036, FEBRA2008086, FEBRA2008134, FEBRA2008135, FEBRA2008159, FEBRA2008165, FEBRA2008172, FEBRA2008201, FEBRA2008265, FEBRA2008266, FEBRA2008282, FEBRA2008287, FEBRA2008311, FEBRA2008341, FEBRA2008360, FEBRA2008459, FEBRA2008468, FEBRA2008475, FEBRA2008481, FEBRA2008542, FEBRA2008660, FEBRA2008662, FEBRA2008681, FEBRA2008692, FEBRA2008741, FEBRA2008755, FEBRA2008836, FEBRA2008859, FEBRA2008861, FEBRA2008866, FEBRA2008881, FEBRA2008928, FEBRA2008983, FEBRA2009016, FEBRA2009022, FEBRA2009029,
FEBRA2009074, FEBRA2009117, FEBRA2009120, FEBRA2009162, FEBRA2009185, FEBRA2009240, FEBRA2009265, FEBRA2009276, FEBRA2009289, FEBRA2009327, FEBRA2009328, FEBRA2009352, FEBRA2009362, FEBRA2009385, FEBRA2009416, FEBRA2009437, FEBRA2009478, FEBRA2009507, FEBRA2009514, FEBRA2009541, FEBRA2009567, FEBRA2009588, FEBRA2009731, FEBRA2009754, FEBRA2009780, FEBRA2009846, FEBRA2009986, FEBRA2010141, FEBRA2010299, FEBRA2010484, FEBRA2010719, FEBRA2010746, FEBRA2010768, FEBRA2010802, FEBRA2011063, FEBRA2011356, FEBRA2011414, FEBRA2011593, FEBRA2011665, FEBRA2012090, FEBRA2012120, FEBRA2012181, FEBRA2012195, FEBRA2012397, FEBRA2012418, FEBRA2012507, FEBRA2012625, FEBRA2013019, FEBRA2013069, FEBRA2013274,
FEBRA2013465, FEBRA2013570, FEBRA2013699, FEBRA2013905, FEBRA2013951, FEBRA2014010, FEBRA2014122, FEBRA2014198, FEBRA2014213, FEBRA2014297, FEBRA2014443, FEBRA2014578, FEBRA2014939, FEBRA2015076, FEBRA2015085, FEBRA2015175, FEBRA2015292, FEBRA2015588, FEBRA2016112, FEBRA2016398, FEBRA2016572, FEBRA2016583, FEBRA2016621, FEBRA2016654, FEBRA2016739, FEBRA2017024, FEBRA2017098, FEBRA2017138, FEBRA2017154, FEBRA2017200, FEBRA2017223, FEBRA2017333, FEBRA2017502, FEBRA2017533, FEBRA2017680, FEBRA2017736, FEBRA2017780, FEBRA2017811, FEBRA2017850, FEBRA2018051, FEBRA2018203, FEBRA2018407, FEBRA2018433, FEBRA2018476, FEBRA2018746, FEBRA2019172, FEBRA2019242, FEBRA2019298, FEBRA2019389, FEBRA2019457,
FEBRA2019519, FEBRA2019582, FEBRA2019637, FEBRA2019663, FEBRA2019690, FEBRA2019711, FEBRA2019867, FEBRA2020406, FEBRA2020566, FEBRA2020582, FEBRA2020668, FEBRA2020801, FEBRA2020845, FEBRA2020886, FEBRA2020977, FEBRA2021032, FEBRA2021134, FEBRA2021171, FEBRA2021339, FEBRA2021431, FEBRA2021497, FEBRA2021550, FEBRA2021571, FEBRA2021636, FEBRA2021864, FEBRA2021892, FEBRA2021908, FEBRA2021966, FEBRA2022013, FEBRA2022148, FEBRA2022204, FEBRA2022255, FEBRA2022322, FEBRA2022601, FEBRA2022696, FEBRA2022729, FEBRA2022787, FEBRA2022911, FEBRA2022956, FEBRA2022963, FEBRA2022972, FEBRA2023227, FEBRA2023285, FEBRA2023351, FEBRA2023377, FEBRA2023399, FEBRA2023498, FEBRA2023550, FEBRA2023721, FEBRA2023989,
FEBRA2023990, FEBRA2024019, FEBRA2024136, FEBRA2024150, FEBRA2024343, FEBRA2024570, FEBRA2024693, FEBRA2024727, FEBRA2024744, FEBRA2024797, FEBRA2024987, FEBRA2025249, FEBRA2025427, FEBRA2025463, FEBRA2025477, FEBRA2026474, FEBRA2026582, FEBRA2026629, FEBRA2026769, FEBRA2026879, FEBRA2026890, FEBRA2026977, FEBRA2027082, FEBRA2027297, FEBRA2027352, FEBRA2027364, FEBRA2027609, FEBRA2028158, FEBRA2028222, FEBRA2028366, FEBRA2028457, FEBRA2028477, FEBRA2028503, FEHRT2000325, FEHRT2000364, FEKID1000018, FELIV1000101, FELIV1000137, FELIV1000164, FELNG2000241, HCASM1000021, HCASM1000050, HCASM1000061, HCASM1000115, HCASM1000130, HCASM2000156, HCASM2000202, HCASM2000214, HCASM2000266, HCASM2000307,
HCASM2000534, HCASM2000536, HCASM2000560, HCASM2000871, HCASM2001285, HCASM2001810, HCASM2001866, HCASM2002148, HCASM2002502, HCASM2002754, HCASM2002918, HCASM2003018, HCASM2003076, HCASM2003099, HCASM2003212, HCASM2003237, HCASM2003357, HCASM2003415, HCASM2005388, HCASM2005484, HCASM2006131, HCASM2006338, HCASM2006359, HCASM2006632, HCASM2007047, HCASM2007317, HCASM2007737, HCASM2007773, HCASM2008154, HCASM2009405, HCASM2009424, HCASM2009463, HCASM2009588, HCHON1000015, HCHON1000166, HCHON1000176, HCHON1000191, HCHON2000028, HCHON2000038, HCHON2000062, HCHON2000100, HCHON2000156, HCHON2000199, HCHON2000212, HCHON2000271, HCHON2000315, HCHON2000322, HCHON2000344, HCHON2000364, HCHON2000373,
HCHON2000395, HCHON2000417, HCHON2000473, HCHON2000503, HCHON2000508, HCHON2000660, HCHON2000663, HCHON2000673, HCHON2000676, HCHON2000699, HCHON2000705, HCHON2000751, HCHON2000815, HCHON2000817, HCHON2000818, HCHON2000819, HCHON2000832, HCHON2000851, HCHON2000898, HCHON2000935, HCHON2000942, HCHON2000956, HCHON2000994, HCHON2001050, HCHON2001099, HCHON2001108, HCHON2001110, HCHON2001116, HCHON2001161, HCHON2001214, HCHON2001233, HCHON2001269, HCHON2001359, HCHON2001407, HCHON2001450, HCHON2001453, HCHON2001497, HCHON2001519, HCHON2001523, HCHON2001535, HCHON2001546, HCHON2001548, HCHON2001577, HCHON2001604, HCHON2001607, HCHON2001646, HCHON2001712, HCHON2001768, HCHON2001801, HCHON2002123,
HCHON2002194, HCHON2002241, HCHON2002354, HCHON2002496, HCHON2002676, HCHON2002770, HCHON2002964, HCHON2002971, HCHON2003131, HCHON2003134, HCHON2003327, HCHON2003513, HCHON2003532, HCHON2003642, HCHON2003659, HCHON2003676, HCHON2004007, HCHON2004279, HCHON2004359, HCHON2004531, HCHON2004776, HCHON2004858, HCHON2005048, HCHON2005118, HCHON2005166, HCHON2005802, HCHON2005987, HCHON2006459, HCHON2006714, HCHON2006722, HCHON2006770, HCHON2006786, HCHON2006841, HCHON2006871, HCHON2007046, HCHON2007482, HCHON2007674, HCHON2007774, HCHON2007881, HCHON2008112, HCHON2008239, HCHON2008582, HCHON2008672, HCHON2008856, HCHON2008871, HCHON2008989,. HCHON2009191, HCHON2009384, HCHON2009749, HCHON2009766,
HCHON2009795, HCHON2010074, HCHON2010543, HEART1000010, HEART1000074, HEART1000088, HEART1000099, HEART1000102, HEART1000118, HEART1000132, HEART1000139, HEART1000142, HEART1000151, HEART1000173, HEART2000024, HEART2000035, HEART2000099, HEART2000298, HEART2000306, HEART2000309, HEART2000411, HEART2000418, HEART2000444, HEART2000448, HEART2000492, HEART2000520, HEART2000541, HEART2000568, HEART2000611, HEART2000959, HEART2001680, HEART2001756, HEART2002129, HEART2002184, HEART2002220, HEART2002432, HEART2002531, HEART2002598, HEART2002717, HEART2003027, HEART2003168, HEART2003432, HEART2003440, HEART2003781, HEART2003836, HEART2003852, HEART2004570, HEART2004764, HEART2004940, HEART2004941,
HEART2004980, HEART2005244, HEART2005310, HEART2005581, HEART2005610, HEART2006131, HEART2006195, HEART2006310, HEART2006334, HEART2006487, HEART2006521, HEART2006557, HEART2006787, HEART2006789, HEART2006909, HEART2007031, HEART2007443, HEART2007696, HEART2007767, HEART2008108, HEART2008111, HEART2008257,. HEART2008509, HEART2008994, HEART2009000, HEART2009599, HEART2009680, HEART2010391, HEART2010492, HEART2010495, HELAC2000158, HELAC2000301, HHDPC1000001, HHDPC1000065, HHDPC1000083, HHDPC1000118, HHDPC2000055, HHDPC2000070, HHDPC2000102, HHDPC2000104, HHDPC2000115, HHDPC2000149, HHDPC2000258, HHDPC2000315, HHDPC2000456, HHDPC2000462, HHDPC2000692, HHDPC2001337, HHDPC2001432, HHDPC2001896,
HHDPC2002963, HHDPC2003049, HHDPC2003113, HHDPC2003381, HHDPC2003472, HHDPC2004757, HHDPC2005185, HHDPC2005513, HHDPC2005742, HHDPC2005794, HHDPC2006460, HHDPC2006862, HHDPC2006927, HHDPC2007302, HHDPC2008123, HHDPC2008185, HHDPC2008279, HHDPC2008297, HHDPC2008414, HHDPC2008474, HHDPC2008816, HHDPC2008843, HHDPC2009114, HHDPC2009178, HHDPC2009208, HHDPC2009272, HHDPC2009528, HLUNG1000017, HLUNG1000024, HLUNG1000030, HLUNG1000034, HLUNG1000037, HLUNG1000053, HLUNG1000084, HLUNG1000091, HLUNG1000099, HLUNG1000104, HLUNG1000105, HLUNG1000110, HLUNG1000151, HLUNG1000169, HLUNG2000014, HLUNG2000027, HLUNG2000068, HLUNG2000116, HLUNG2000125, HLUNG2000130, HLUNG2000142, HLUNG2000174, HLUNG2000176,
HLUNG2000217, HLUNG2000232, HLUNG2000255, HLUNG2000281, HLUNG2000315, HLUNG2000340, HLUNG2000362, HLUNG2000363, HLUNG2000431, HLUNG2000480, HLUNG2000489, HLUNG2000549, HLUNG2000557, HLUNG2000570, HLUNG2000751, HLUNG2000761, HLUNG2000777, HLUNG2000870, HLUNG2000926, HLUNG2000950, HLUNG2000955, HLUNG2000970, HLUNG2001013, HLUNG2001126, HLUNG2001144, HLUNG2001214, HLUNG2001439, HLUNG2001459, HLUNG2001633, HLUNG2001712, HLUNG2001814, HLUNG2001996, HLUNG2002006, HLUNG2002050, HLUNG2002059, HLUNG2002085, HLUNG2002145, HLUNG2002295, HLUNG2002303, HLUNG2002334, HLUNG2002405, HLUNG2002429, HLUNG2002465, HLUNG2002562, HLUNG2002659, HLUNG2002707, HLUNG2002916, HLUNG2002942, HLUNG2002949, HLUNG2002958,
HLUNG2002982, HLUNG2003042, HLUNG2003049, HLUNG2003061, HLUNG2003162, HLUNG2003246, HLUNG2003306, HLUNG2003331, HLUNG2003335, HLUNG2003378, HLUNG2003497, HLUNG2003691, HLUNG2003710, HLUNG2003714, HLUNG2003716, HLUNG2003778, HLUNG2003833, HLUNG2003872, HLUNG2003918, HLUNG2003991, HLUNG2004014, HLUNG2004037, HLUNG2004067, HLUNG2004154, HLUNG2004217, HLUNG2004273, HLUNG2004388, HLUNG2004416, HLUNG2004521, HLUNG2004534, HLUNG2004707, HLUNG2004774, HLUNG2004775, HLUNG2005012, HLUNG2005030, HLUNG2005045, HLUNG2005076, HLUNG2005133, HLUNG2005203, HLUNG2005230, HLUNG2005479, HLUNG2005524, HLUNG2005656, HLUNG2005738, HLUNG2005774, HLUNG2005813, HLUNG2005924, HLUNG2006026, HLUNG2006067, HLUNG2006094,
HLUNG2006370, HLUNG2006397, HLUNG2006570, HLUNG2006599, HLUNG2006614, HLUNG2006671, HLUNG2006781, HLUNG2006843, HLUNG2006852, HLUNG2006935, HLUNG2007041, HLUNG2007163, HLUNG2007210, HLUNG2007219, HLUNG2007245, HLUNG2007433, HLUNG2007931, HLUNG2008066, HLUNG2008139, HLUNG2008153, HLUNG2008225, HLUNG2008235, HLUNG2008247, HLUNG2008348, HLUNG2008384, HLUNG2008396, HLUNG2008439, HLUNG2008479, HLUNG2008480, HLUNG2008521, HLUNG2008833, HLUNG2008875, HLUNG2008885, HLUNG2008958, HLUNG2008959, HLUNG2009067, HLUNG2009215, HLUNG2009253, HLUNG2009303, HLUNG2009413, HLUNG2009729, HLUNG2010181, HLUNG2010464, HLUNG2010562, HLUNG2010845, HLUNG2011041, HLUNG2011298, HLUNG2011461, HLUNG2011833, HLUNG2012049,
HLUNG2012287, HLUNG2012315, HLUNG2012546, HLUNG2012600, HLUNG2012727, HLUNG2013097, HLUNG2013204, HLUNG2013304, HLUNG2013350, HLUNG2013622, HLUNG2013643, HLUNG2013784, HLUNG2013851, HLUNG2014262, HLUNG2014288, HLUNG2014449, HLUNG2014821, HLUNG2015418, HLUNG2015548, HLUNG2015578, HLUNG2015617, HLUNG2016022, HLUNG2016862, HLUNG2017286, HLUNG2017307, HLUNG2017350, HLUNG2017546, HLUNG2017806, HLUNG2018029, HLUNG2018282, HLUNG2018626, HLUNG2019058, HSYRA1000119, HSYRA1000131, HSYRA1000137, HSYRA1000148, HSYRA2000044, HSYRA2000135, HSYRA2000137, HSYRA2000190, HSYRA2000221, HSYRA2000237, HSYRA2000253, HSYRA2000332, HSYRA2000371, HSYRA2000424, HSYRA2000507, HSYRA2000510, HSYRA2000605, HSYRA2000629,
HSYRA2000706, HSYRA2000760, HSYRA2000787, HSYRA2000792, HSYRA2000828, HSYRA2000832, HSYRA2000925, HSYRA2000927, HSYRA2001003, HSYRA2001103, HSYRA2001142, HSYRA2001153, HSYRA2001225, HSYRA2001232, HSYRA2001255, HSYRA2001332, HSYRA2001353, HSYRA2001420, HSYRA2001484, HSYRA2001552, HSYRA2001595, HSYRA2001631, HSYRA2001638, HSYRA2001983, HSYRA2002103, HSYRA2002590, HSYRA2002741, HSYRA2003168, HSYRA2004550, HSYRA2004858, HSYRA2005456, HSYRA2005496, HSYRA2005628, HSYRA2005970, HSYRA2006049, HSYRA2006873, HSYRA2007241, HSYRA2007338, HSYRA2007667, HSYRA2008154, HSYRA2008714, HSYRA2009102, IMR321000155, IMR321000161, IMR321000165, IMR321000193, IMR321000207, IMR321000230, IMR321000253, IMR322000010,
IMR322000072, IMR322000107, IMR322000127, IMR322000144, IMR322000152, IMR322000164, IMR322000178, IMR322000223, IMR322000243, IMR322000302, IMR322000316, IMR322000374, IMR322000379, IMR322000383, IMR322000418, IMR322000518, IMR322000555, IMR322000604, IMR322000609, IMR322000656, IMR322000672, IMR322000742, IMR322000775, IMR322000791, IMR322000811, IMR322000819, IMR322000838, IMR322000859, IMR322000863, IMR322000917, IMR322000918, IMR322000919, IMR322000953, IMR322000973, IMR322000984, IMR322001018, IMR322001167, IMR322001185, IMR322001218, IMR322001245, IMR322001317, IMR322001318, IMR322001322, IMR322001332, IMR322001380, IMR322001438, IMR322001452, IMR322001491, IMR322001541, IMR322001584,
IMR322001665, IMR322001670, IMR322001724, IMR322002035, IMR322002110, IMR322002470, IMR322002993, IMR322003675, IMR322004768, IMR322005293, IMR322005833, IMR322006012, IMR322006222, IMR322006495, IMR322006520, IMR322006886, IMR322006947, IMR322007078, IMR322007225, IMR322007704, IMR322008651, IMR322009710, IMR322009807, IMR322010295, IMR322010953, IMR322011689, IMR322012314, IMR322012529, IMR322013053, IMR322013396, IMR322013731, IMR322013805, IMR322015523, IMR322016146, IMR322017049, IMR322018117, IMR322018192, JCMLC2000273, KIDNE1000008, KIDNE1000012, KIDNE1000015, KIDNE1000028, KIDNE1000064, KIDNE1000104, KIDNE1000121, KIDNE1000143, KIDNE1000152, KIDNE2000026, KIDNE2000041, KIDNE2000046,
KIDNE2000051, KIDNE2000070, KIDNE2000167, KIDNE2000174, KIDNE2000192, KIDNE2000227, KIDNE2000244, KIDNE2000245, KIDNE2000252, KIDNE2000266, KIDNE2000287, KIDNE2000315, KIDNE2000317, KIDNE2000328, KIDNE2000335, KIDNE2000349, KIDNE2000375, KIDNE2000383, KIDNE2000394, KIDNE2000422, KIDNE2000493, KIDNE2000497, KIDNE2000510, KIDNE2000513, KIDNE2000517, KIDNE2000519, KIDNE2000555, KIDNE2000574, KIDNE2000624, KIDNE2000665, KIDNE2000678, KIDNE2000717, KIDNE2000721, KIDNE2000722, KIDNE2000777, KIDNE2000801, KIDNE2000832, KIDNE2000833, KIDNE2000846, KIDNE2000867, KIDNE2000909, KIDNE2000947, KIDNE2001117, KIDNE2001140, KIDNE2001269, KIDNE2001361, KIDNE2001373, KIDNE2001636, KIDNE2001713, KIDNE2001802,
KIDNE2001840, KIDNE2001847, KIDNE2001857, KIDNE2001873, KIDNE2001897, KIDNE2001931, KIDNE2002015, KIDNE2002162, KIDNE2002168, KIDNE2002191, KIDNE2002198, KIDNE2002262, KIDNE2002265, KIDNE2002438, KIDNE2002483, KIDNE2002725, KIDNE2002768, KIDNE2002795, KIDNE2002798, KIDNE2002839, KIDNE2002845, KIDNE2002872, KIDNE2002882, KIDNE2002883, KIDNE2002980, KIDNE2002991, KIDNE2003185, KIDNE2003188, KIDNE2003233, KIDNE2003316, KIDNE2003335, KIDNE2003357, KIDNE2003373, KIDNE2003377, KIDNE2003507, KIDNE2003752, KIDNE2003764, KIDNE2003837, KIDNE2003994, KIDNE2004034, KIDNE2004054, KIDNE2004084, KIDNE2004095, KIDNE2004115, KIDNE2004233, KIDNE2004262, KIDNE2004279, KIDNE2004294, KIDNE2004295, KIDNE2004305,
KIDNE2004344, KIDNE2004394, KIDNE2004411, KIDNE2004475, KIDNE2004519, KIDNE2004520, KIDNE2004531, KIDNE2004534, KIDNE2004562, KIDNE2004579, KIDNE2004626, KIDNE2004681, KIDNE2004828, KIDNE2004846, KIDNE2004864, KIDNE2004879, KIDNE2004981, KIDNE2004985, KIDNE2004999, KIDNE2005038, KIDNE2005042, KIDNE2005062, KIDNE2005266, KIDNE2005296, KIDNE2005327, KIDNE2005336, KIDNE2005400, KIDNE2005424, KIDNE2005477, KIDNE2005526, KIDNE2005543, KIDNE2005603, KIDNE2005629, KIDNE2005781, KIDNE2005798, KIDNE2005854, KIDNE2005908, KIDNE2005937, KIDNE2006009, KIDNE2006039, KIDNE2006053, KIDNE2006062, KIDNE2006092, KIDNE2006145, KIDNE2006149, KIDNE2006210, KIDNE2006299, KIDNE2006334, KIDNE2006353, KIDNE2006376,
KIDNE2006378, KIDNE2006580, KIDNE2006652, KIDNE2006687, KIDNE2006733, KIDNE2006760, KIDNE2006775, KIDNE2006811, KIDNE2006820, KIDNE2006880, KIDNE2007005, KIDNE2007077, KIDNE2007186, KIDNE2007222, KIDNE2007356, KIDNE2007422, KIDNE2007569, KIDNE2007810, KIDNE2007910, KIDNE2007944, KIDNE2008022, KIDNE2008069, KIDNE2008072, KIDNE2008116, KIDNE2008117, KIDNE2008218, KIDNE2008315, KIDNE2008362, KIDNE2008378, KIDNE2008403, KIDNE2008404, KIDNE2008446, KIDNE2008473, KIDNE2008480, KIDNE2008649, KIDNE2008666, KIDNE2008697, KIDNE2008758, KIDNE2008788, KIDNE2008795, KIDNE2008824, KIDNE2008832, KIDNE2008869, KIDNE2008914, KIDNE2008975, KIDNE2008987, KIDNE2009019, KIDNE2009109, KIDNE2009180, KIDNE2009191,
KIDNE2009332, KIDNE2009426, KIDNE2009467, KIDNE2009490, KIDNE2009553, KIDNE2009628, KIDNE2009647, KIDNE2009676, KIDNE2009723, KIDNE2010007, KIDNE2010052, KIDNE2010084, KIDNE2010137, KIDNE2010151, KIDNE2010264, KIDNE2010265, KIDNE2010271, KIDNE2010419, KIDNE2010430, KIDNE2010574, KIDNE2010674, KIDNE2010739, KIDNE2010750, KIDNE2010762, KIDNE2010863, KIDNE2010973, KIDNE2010989, KIDNE2011200, KIDNE2011314, KIDNE2011508, KIDNE2011532, KIDNE2011635, KIDNE2011752, KIDNE2011782, KIDNE2011858, KIDNE2012009, KIDNE2012025, KIDNE2012188, KIDNE2012291, KIDNE2012316, KIDNE2012361, KIDNE2012440, KIDNE2012453, KIDNE2012563, KIDNE2012601, KIDNE2012613, KIDNE2012710, KIDNE2012745, KIDNE2012775, KIDNE2012945,
KIDNE2013045, KIDNE2013095, KIDNE2013218, KIDNE2013263, KIDNE2013346, KIDNE2013388, KIDNE2013413, KIDNE2013489, KIDNE2013639, KIDNE2013731, KIDNE2013734, KIDNE2013775, KIDNE2013801, KIDNE2013866, KIDNE2014087, KIDNE2014112, KIDNE2014119, KIDNE2014170, KIDNE2014184, KIDNE2014268, KIDNE2014298, KIDNE2014320, KIDNE2014415, KIDNE2014489, KIDNE2014496, KIDNE2014661, KIDNE2014717, KIDNE2014789, KIDNE2014808, KIDNE2014890, KIDNE2014978, KIDNE2015073, KIDNE2015221, KIDNE2015244, KIDNE2015313, KIDNE2015433, KIDNE2015445, KIDNE2015483, KIDNE2015515, KIDNE2015546, KIDNE2015556, KIDNE2015598, KIDNE2015660, KIDNE2015710, KIDNE2015987, KIDNE2016000, KIDNE2016036, KIDNE2016096, KIDNE2016101, KIDNE2016142,
KIDNE2016188, KIDNE2016327, KIDNE2016371, KIDNE2016388, KIDNE2016464, KIDNE2016539, KIDNE2016570, KIDNE2016918, KIDNE2017007, KIDNE2017052, KIDNE2017151, KIDNE2017153, KIDNE2017315, KIDNE2017332, KIDNE2017343, KIDNE2017454, KIDNE2017603, KIDNE2017956, KIDNE2018071, KIDNE2018166, KIDNE2018167, KIDNE2018217, KIDNE2018254, KIDNE2018268, KIDNE2018269, KIDNE2018287, KIDNE2018294, KIDNE2018352, KIDNE2018462, KIDNE2018493, KIDNE2018617, KIDNE2018678, KIDNE2018727, KIDNE2018863, KIDNE2018891, KIDNE2018989, KIDNE2018996, KIDNE2019074, KIDNE2019187, LIVER1000017, LIVER1000050, LIVER1000055, LIVER1000058, LIVER1000063, LIVER1000067, LIVER1000073, LIVER1000082, LIVER1000099, LIVER1000104, LIVER1000111,
LIVER1000126, LIVER1000139, LIVER1000155, LIVER1000165, LIVER1000175, LIVER1000224, LIVER1000230, LIVER1000278, LIVER1000303, LIVER1000397, LIVER1000421, LIVER1000433, LIVER1000479, LIVER1000482, LIVER1000542, LIVER2000003, LIVER2000037, LIVER2000158, LIVER2000216, LIVER2000237, LIVER2000247, LIVER2000360, LIVER2000416, LIVER2000446, LIVER2000515, LIVER2000592, LIVER2000626, LIVER2000682, LIVER2000769, LIVER2000775, LIVER2001051, LIVER2001076, LIVER2001099, LIVER2001113, LIVER2001164, LIVER2001191, LIVER2001265, LIVER2001329, LIVER2001389, LIVER2001461, LIVER2001835, LIVER2002638, LIVER2002644, LIVER2002660, LIVER2002702, LIVER2003008, LIVER2003065, LIVER2003511, LIVER2003524, LIVER2003568,
LIVER2003581, LIVER2003800, LIVER2003854, LIVER2004074, LIVER2004392, LIVER2004514, LIVER2004565, LIVER2005028, LIVER2005198, LIVER2005376, LIVER2005385, LIVER2005512, LIVER2005520, LIVER2005527, LIVER2005544, LIVER2005625, LIVER2005728, LIVER2005789, LIVER2005973, LIVER2005981, LIVER2006006, LIVER2006251, LIVER2006410, LIVER2006469, LIVER2006644, LIVER2006764, LIVER2007415, LIVER2007548, LIVER2007568, LIVER2007721, LIVER2008053, LIVER2008473, LIVER2008580, LIVER2008706, LIVER2008751, LIVER2008945, LIVER2008998, LIVER2009110, LIVER2009118, LIVER2009554, LYMPB1000141, LYMPB1000158, LYMPB2000083, MAMGL1000017, MAMGL1000032, MAMGL1000035, MAMGL1000096, MAMGL1000100, MAMGL1000132, MAMGL1000151,
MAMGL1000173, MAMGL1000178, MAMGL1000182, MAMGL1000184, MESAN1000032, MESAN1000035, MESAN1000050, MESAN1000079, MESAN1000080, MESAN1000101, MESAN1000121, MESAN1000126, MESAN1000147, MESAN1000180, MESAN2000023, MESAN2000026, MESAN2000067, MESAN2000092, MESAN2000149, MESAN2000167, MESAN2000291, MESAN2000337, MESAN2000434, MESAN2000457, MESAN2000462, MESAN2000501, MESAN2000572, MESAN2000608, MESAN2000620, MESAN2000711, MESAN2000815, MESAN2000909, MESAN2001450, MESAN2001627, MESAN2001770, MESAN2001979, MESAN2001980, MESAN2002113, MESAN2002122, MESAN2002147, MESAN2002186, MESAN2002519, MESAN2002687, MESAN2002709, MESAN2002790, MESAN2002940, MESAN2002978, MESAN2003035, MESAN2003037, MESAN2003101,
MESAN2003190, MESAN2003322, MESAN2003490, MESAN2003529, MESAN2003622, MESAN2003646, MESAN2003662, MESAN2003851, MESAN2004138, MESAN2004288, MESAN2004575, MESAN2005284, MESAN2005371, MESAN2005633, MESAN2005689, MESAN2005724, MESAN2005766, MESAN2005808, MESAN2005811, MESAN2005958, MESAN2006022, MESAN2006328, MESAN2006401, MESAN2006563, MESAN2006599, MESAN2006953, MESAN2006971, MESAN2007032, MESAN2007440, MESAN2007597, MESAN2007755, MESAN2008222, MESAN2008334, MESAN2008415, MESAN2008460, MESAN2008536, MESAN2008679, MESAN2008729, MESAN2008821, MESAN2008926, MESAN2008936, MESAN2009019, MESAN2009081, MESAN2009105, MESAN2009311, MESAN2009418, MESAN2009503, MESAN2009522, MESAN2010031, MESAN2010114,
MESAN2010312, MESAN2010321, MESAN2010664, MESAN2010681, MESAN2011545, MESAN2011597, MESAN2011627, MESAN2011632, MESAN2011977, MESAN2011984, MESAN2012054, MESAN2012113, MESAN2012586, MESAN2012708, MESAN2012735, MESAN2013019, MESAN2013022, MESAN2013183, MESAN2013211, MESAN2013283, MESAN2013611, MESAN2013674, MESAN2013845, MESAN2013908, MESAN2013936, MESAN2014192, MESAN2014298, MESAN2014412, MESAN2014624, MESAN2015277, MESAN2015365, MESAN2015391, MESAN2015401, MESAN2015501, MESAN2015515, MESAN2015708, MESAN2016159, MESAN2016304, MESAN2016409, MESAN2016965, MESAN2017133, MESAN2017373, MESAN2017417, MESAN2018209, MESAN2018576, MESAN2018670, MESAN2018699, MESTC1000042, MESTC1000147, MESTC2000150,
MESTC2000153, MESTC2000170, N1ESE2000698, NB9N41000011, NB9N41000047, NB9N41000135, NB9N41000142, NB9N41000146, NB9N41000340, NB9N42000104, NB9N42000122, NB9N42000196, NB9N42000314, NB9N42000342, NB9N42000430, NB9N42000495, NB9N42001300, NCRRP1000129, NESOP1000010, NESOP1000087, NESOP1000108, NESOP2000231, NESOP2000452, NESOP2000499, NESOP2000504, NESOP2000744, NESOP2001144, NESOP2001433, NESOP2001656, NESOP2001694, NESOP2001752, NESOP2002352, NESOP2002418, NESOP2002487, NESOP2002738, NESOP2002780, NETRP1000034, NETRP1000082, NETRP2000086, NETRP2000182, NETRP2000439, NHNPC1000034, NHNPC1000037, NHNPC1000084, NHNPC1000101, NHNPC1000124, NHNPC2000062, NHNPC2000187, NHNPC2000206, NHNPC2000212,
NHNPC2000606, NHNPC2000877, NHNPC2001108, NHNPC2001223, NHNPC2001312, NHNPC2001816, NHNPC2001931, NHNPC2002565, NHNPC2002749, NOVAR1000102, NOVAR2000038, NOVAR2000136, NOVAR2000352, NOVAR2000412, NOVAR2000783, NOVAR2000962, NOVAR2001108, NOVAR2001620, NOVAR2001783, NOVAR2001876, NOVAR2002039, NT2NE1000004, NT2NE1000014, NT2NE1000018, NT2NE1000023, NT2NE1000024, NT2NE1000059, NT2NE1000063, NT2NE1000073, NT2NE1000083, NT2NE1000120, NT2NE1000122, NT2NE1000175, NT2NE1000181, NT2NE1000185, NT2NE2000038, NT2NE2000054, NT2NE2000057, NT2NE2000064, NT2NE2000109, NT2NE2000137, NT2NE2000156, NT2NE2000174, NT2NE2000195, NT2NE2000222, NT2NE2000259, NT2NE2000260, NT2NE2000284, NT2NE2000299, NT2NE2000353,
NT2NE2000356, NT2NE2000369, NT2NE2000374, NT2NE2000386, NT2NE2000390, NT2NE2000392, NT2NE2000470, NT2NE2000488, NT2NE2000517, NT2NE2000536, NT2NE2000546, NT2NE2000548, NT2NE2000550, NT2NE2000586, NT2NE2000612, NT2NE2000636, NT2NE2000658, NT2NE2000706, NT2NE2000763, NT2NE2000802, NT2NE2000808, NT2NE2000809, NT2NE2000864, NT2NE2000909, NT2NE2000913, NT2NE2000950, NT2NE2000951, NT2NE2000980, NT2NE2001005, NT2NE2001021, NT2NE2001040, NT2NE2001048, NT2NE2001049, NT2NE2001142, NT2NE2001156, NT2NE2001180, NT2NE2001181, NT2NE2001188, NT2NE2001191, NT2NE2001247, NT2NE2001252, NT2NE2001324, NT2NE2001326, NT2NE2001364, NT2NE2001372, NT2NE2001397, NT2NE2001403, NT2NE2001428, NT2NE2001432, NT2NE2001442,
NT2NE2001524, NT2NE2001530, NT2NE2001598, NT2NE2001623, NT2NE2001626, NT2NE2001634, NT2NE2001648, NT2NE2001655, NT2NE2001666, NT2NE2001677, NT2NE2001697, NT2NE2001698, NT2NE2001723, NT2NE2001725, NT2NE2001874, NT2NE2001889, NT2NE2001923, NT2NE2002100, NT2NE2002162, NT2NE2002186, NT2NE2002285, NT2NE2002311, NT2NE2002651, NT2NE2002732, NT2NE2002768, NT2NE2002791, NT2NE2002856, NT2NE2002870, NT2NE2003150, NT2NE2003252, NT2NE2003280, NT2NE2003308, NT2NE2003309, NT2NE2003315, NT2NE2003393, NT2NE2003408, NT2NE2003457, NT2NE2003540, NT2NE2003571, NT2NE2003705, NT2NE2003752, NT2NE2003887, NT2NE2003921, NT2NE2004255, NT2NE2004378, NT2NE2004490, NT2NE2004606, NT2NE2004740, NT2NE2004787, NT2NE2004916,
NT2NE2005035, NT2NE2005223, NT2NE2005276, NT2NE2005317, NT2NE2005323, NT2NE2005371, NT2NE2005395, NT2NE2005419, NT2NE2005441, NT2NE2005517, NT2NE2005537, NT2NE2005555, NT2NE2005588, NT2NE2005676, NT2NE2005688, NT2NE2005720, NT2NE2005784, NT2NE2005804, NT2NE2005821, NT2NE2005876, NT2NE2005890, NT2NE2005921, NT2NE2005968, NT2NE2006075, NT2NE2006087, NT2NE2006103, NT2NE2006217, NT2NE2006288, NT2NE2006382, NT2NE2006387, NT2NE2006427, NT2NE2006432, NT2NE2006478, NT2NE2006531, NT2NE2006784, NT2NE2006813, NT2NE2006894, NT2NE2006909, NT2NE2006942, NT2NE2006958, NT2NE2007052, NT2NE2007220, NT2NE2007425, NT2NE2007725, NT2NE2007727, NT2NE2007786, NT2NE2007877, NT2NE2007967, NT2NE2008017, NT2NE2008060,
NT2NE2008077, NT2NE2008213, NT2NE2008260, NT2NE2008378, NT2NE2008607, NT2NE2008639, NT2NE2008727, NT2NE2008783, NT2NE2008785, NT2NE2008803, NT2NE2008867, NT2NE2008961, NT2NE2008997, NT2NE2009138, NT2NE2009426, NT2NE2009523, NT2NE2009608, NT2NE2009691, NT2NE2010056, NT2NE2010105, NT2NE2010150, NT2NE2010400, NT2NE2010632, NT2NE2010633, NT2NE2010654, NT2NE2010688, NT2NE2010781, NT2NE2010842, NT2NE2010854, NT2NE2011033, NT2NE2011036, NT2NE2011107, NT2NE2011119, NT2NE2011154, NT2NE2011221, NT2NE2011411, NT2NE2011485, NT2NE2011650, NT2NE2011684, NT2NE2011687, NT2NE2011691, NT2NE2011758, NT2NE2011823, NT2NE2011896, NT2NE2011998, NT2NE2012113, NT2NE2012199, NT2NE2012243, NT2NE2012361, NT2NE2012448,
NT2NE2012457, NT2NE2012493, NT2NE2012505, NT2NE2012533, NT2NE2012603, NT2NE2012747, NT2NE2012790, NT2NE2012847, NT2NE2012928, NT2NE2013019, NT2NE2013081, NT2NE2013189, NT2NE2013217, NT2NE2013547, NT2NE2014013, NT2NE2014028, NT2NE2014104, NT2NE2014113, NT2NE2014176, NT2NE2014221, NT2NE2014525, NT2NE2014650, NT2NE2014651, NT2NE2014681, NT2NE2014758, NT2NE2014869, NT2NE2014950, NT2NE2015061, NT2NE2015262, NT2NE2015275, NT2NE2015362, NT2NE2015480, NT2NE2015511, NT2NE2015565, NT2NE2015626, NT2NE2015747, NT2NE2015860, NT2NE2015885, NT2NE2015904, NT2NE2016041, NT2NE2016387, NT2NE2016419, NT2NE2016519, NT2NE2016608, NT2NE2016766, NT2NE2016774, NT2NE2016971, NT2NE2017259, NT2NE2017332, NT2NE2017397,
NT2NE2017480, NT2NE2017508, NT2NE2017562, NT2NE2017590, NT2NE2017612, NT2NE2017721, NT2NE2017752, NT2NE2017792, - NT2NE2017982, NT2NE2018165, NT2NE2018176, NT2NE2018180, NT2NE2018273, NT2NE2018376, NT2NE2018472, NT2NE2018490, NT2NE2018594, NT2NE2018739; NT2NE2018916, NT2NE2019056, NT2NE2019076, NT2NE2019091, NT2NE2019099, NT2RI1000016, NT2RI1000027, NT2RI1000035, NT2RI1000048, NT2RI1000164, NT2RI1000172, NT2RI2000004, NT2RI2000107, NT2RI2000116, NT2RI2000117, NT2RI2000128, NT2RI2000133, NT2RI2000167, NT2RI2000173, NT2RI2000187, NT2RI2000255, NT2RI2000263, NT2RI2000270, NT2RI2000276, NT2RI2000284, NT2RI2000313, NT2RI2000341, NT2RI2000348, NT2RI2000412, NT2RI2000421, NT2RI2000480, NT2RI2000575,
NT2RI2000588, NT2RI2000597, NT2RI2000669, NT2RI2000688, NT2RI2000704, NT2RI2000738, NT2RI2000775, NT2RI2000822, NT2RI2000829, NT2RI2000841, NT2RI2000865, NT2RI2000932, NT2RI2000974, NT2RI2000987, NT2RI2001010, NT2RI2001017, NT2RI2001030, NT2RI2001054, NT2RI2001083, NT2RI2001167, NT2RI2001178, NT2RI2001235, NT2RI2001244, NT2RI2001307, NT2RI2001342, NT2RI2001385, NT2RI2001409, NT2RI2001410, NT2RI2001422, NT2RI2001425, NT2RI2001449, NT2RI2001450, NT2RI2001474, NT2RI2001519, NT2RI2001595, NT2RI2001621, NT2RI2001624, NT2RI2001726, NT2RI2001731, NT2RI2001769, NT2RI2001782, NT2RI2001836, NT2RI2001846, NT2RI2001866, NT2RI2001910, NT2RI2001952, NT2RI2002022, NT2RI2002041, NT2RI2002117, NT2RI2002120,
NT2RI2002153, NT2RI2002179, NT2RI2002243, NT2RI2002252, NT2RI2002260, NT2RI2002266, NT2RI2002270, NT2RI2002334, NT2RI2002359, NT2RI2002447, NT2RI2002481, NT2RI2002507, NT2RI2002517, NT2RI2002530, NT2RI2002540, NT2RI2002549, NT2RI2002554, NT2RI2002564, NT2RI2002585, NT2RI2002592, NT2RI2002602, NT2RI2002699, NT2RI2002729, NT2RI2002802, NT2RI2002819, NT2RI2002822, NT2RI2002847, NT2RI2002852, NT2RI2002923, NT2RI2002926, NT2RI2002958, NT2RI2002970, NT2RI2003011, NT2RI2003019, NT2RI2003051, NT2RI2003067, NT2RI2003184, NT2RI2003205, NT2RI2003222, NT2RI2003301, NT2RI2003360, NT2RI2003361, NT2RI2003383, NT2RI2003392, NT2RI2003399, NT2RI2003419, NT2RI2003481, NT2RI2003487, NT2RI2003655, NT2RI2003667,
NT2RI2003678, NT2RI2003884, NT2RI2003921, NT2RI2003973, NT2RI2003993, NT2RI2004059, NT2RI2004078, NT2RI2004079, NT2RI2004093, NT2RI2004136, NT2RI2004190, NT2RI2004209, NT2RI2004284, NT2RI2004290, NT2RI2004304, NT2RI2004312, NT2RI2004381, NT2RI2004398, NT2RI2004410, NT2RI2004442, NT2RI2004468, NT2RI2004606, NT2RI2004608, NT2RI2004618, NT2RI2004783, NT2RI2004818, NT2RI2004840, NT2RI2004884, NT2RI2004916, NT2RI2004962, NT2RI2004984, NT2RI2004985, NT2RI2005048, NT2RI2005061, NT2RI2005069, NT2RI2005087, NT2RI2005096, NT2RI2005115, NT2RI2005130, NT2RI2005150, NT2RI2005166, NT2RI2005239,. NT2RI2005246, NT2RI2005315, NT2RI2005353, NT2RI2005368, NT2RI2005382, NT2RI2005473, NT2RI2005520, NT2RI2005564,
NT2RI2005579, NT2RI2005621, NT2RI2005628, NT2RI2005647, NT2RI2005670, NT2RI2005710, NT2RI2005772, NT2RI2005816, NT2RI2005851, NT2RI2005966, NT2RI2006070, NT2RI2006071, NT2RI2006072, NT2RI2006081, NT2RI2006126, NT2RI2006127, NT2RI2006183, NT2RI2006257, NT2RI2006271, NT2RI2006294, NT2RI2006345, NT2RI2006403, NT2RI2006445, NT2RI2006487, NT2RI2006506, NT2RI2006553, NT2RI2006565, NT2RI2006640, NT2RI2006647, NT2RI2006659, NT2RI2006662, NT2RI2006667, NT2RI2006679, NT2RI2006682, NT2RI2006686, NT2RI2006703, NT2RI2006716, NT2RI2006735, NT2RI2006762, NT2RI2006788, NT2RI2006838, NT2RI2006853, NT2RI2006855, NT2RI2006856, NT2RI2006943, NT2RI2006973, NT2RI2007046, NT2RI2007048, NT2RI2007084, NT2RI2007096,
NT2RI2007116, NT2RI2007133, NT2RI2007214, NT2RI2007303, NT2RI2007334, NT2RI2007377, NT2RI2007386, NT2RI2007406, NT2RI2007439, NT2RI2007468, NT2RI2007469, NT2RI2007507, NT2RI2007572, NT2RI2007593, NT2RI2007723, NT2RI2007729, NT2RI2007751, NT2RI2007754, NT2RI2007827, NT2RI2007853, NT2RI2007877, NT2RI2007879, NT2RI2007884, NT2RI2007891, NT2RI2007956, NT2RI2007965, NT2RI2008050, NT2RI2008141, NT2RI2008171, NT2RI2008188, NT2RI2008204, NT2RI2008221, NT2RI2008233, NT2RI2008396, NT2RI2008455, NT2RI2008502, NT2RI2008566, NT2RI2008598, NT2RI2008622, NT2RI2008656, NT2RI2008714, NT2RI2008801, NT2RI2008808, NT2RI2008812, NT2RI2008841, NT2RI2008952, NT2RI2009005, NT2RI2009066, NT2RI2009083, NT2RI2009101,
NT2RI2009103, NT2RI2009144, NT2RI2009151, NT2RI2009173, NT2RI2009194, NT2RI2009215, NT2RI2009233, NT2RI2009239, NT2RI2009259, NT2RI2009281, NT2RI2009289, NT2RI2009301, NT2RI2009517, NT2RI2009585, NT2RI2009595, NT2RI2009855, NT2RI2010283, NT2RI2010508, NT2RI2010795, NT2RI2011422, NT2RI2011450, NT2RI2011683, NT2RI2011803, NT2RI2012350, NT2RI2012659, NT2RI2012726, NT2RI2012990, NT2RI2013345, NT2RI2013357, NT2RI2013373, NT2RI2014551, NT2RI2014683, NT2RI2015239, NT2RI2015342, NT2RI2015533, NT2RI2016128, NT2RI2016134, NT2RI2018311, NT2RI2018363, NT2RI2018448, NT2RI2018883, NT2RI2018950, NT2RI2019751, NT2RI2019826, NT2RI2020390, NT2RI2020703, NT2RI2021463, NT2RI2021625, NT2RI2022584, NT2RI2023303,
NT2RI2023671, NT2RI2024008, NT2RI2024460, NT2RI2024496, NT2RI2024935, NT2RI2025075, NT2RI2025255, NT2RI2025564, NT2RI2025565, NT2RI2025909, NT2RI2025957, NT2RI2025976, NT2RI2026758, NT2RI2027081, NT2RI2027157, NT2RI2027224, NT2RI2027396, NT2RI2027484, NT2RI2027975, NT2RI2028213, NT2RI2028222, NT2RI2028537, NT2RI2028642, NT2RI3000174, NT2RI3000213, NT2RI3000423, NT2RI3000622, NT2RI3001132, NT2RI3001263, NT2RI3001279, NT2RI3001348, NT2RI3001458, NT2RI3001515, NT2RI3001573, NT2RI3001967, NT2RI3002303, NT2RI3002557, NT2RI3002842, NT2RI3003027, NT2RI3003031, NT2RI3003095, NT2RI3003104, NT2RI3003162, NT2RI3003362, NT2RI3003382, NT2RI3003409, NT2RI3003738, NT2RI3003925, NT2RI3004133, NT2RI3004381,
NT2RI3004510, NT2RI3005202, NT2RI3005403, NT2RI3005416, NT2RI3005861, NT2RI3005928, NT2RI3006132, NT2RI3006171, NT2RI3006376, NT2RI3006796, NT2RI3007065, NT2RI3007095, NT2RI3007158, NT2RI3007167, NT2RI3007213, NT2RI3007443, NT2RI3007684, NT2RI3007757, NT2RI3007878, NT2RI3007978, NT2RI3008055, NT2RI3008162, NT2RI3008179, NT2RI3008228, NT2RI3008442, NT2RI3008652, NT2RI3008697, NT2RI3008974, NT2RI3009158, NT2RI3009480, NT2RI3009524, NT2RP6000005, NT2RP6000008, NT2RP6000032, NT2RP6000033, NT2RP6000043, NT2RP6000060, NT2RP6000072, NT2RP6000086, NT2RP6000100, NT2RP6000109, NT2RP6000110, NT2RP6000119, NT2RP6000127, NT2RP6000130, NT2RP6000140, NT2RP6000150, NT2RP7000041, NT2RP7000086, NT2RP7000109,
NT2RP7000112, NT2RP7000238, NT2RP7000259, NT2RP7000271, NT2RP7000311, NT2RP7000359, NT2RP7000364, NT2RP7000380, NT2RP7000391, NT2RP7000425, NT2RP7000579, NT2RP7000624, NT2RP7000812, NT2RP7000926, NT2RP7001074, NT2RP7001080, NT2RP7001156, NT2RP7001231, NT2RP7001283, NT2RP7001306, NT2RP7001319, NT2RP7001335, NT2RP7001364, NT2RP7001475, NT2RP7001532, NT2RP7001591, NT2RP7001602, NT2RP7001678, NT2RP7001856, NT2RP7002064, NT2RP7002151, NT2RP7002163, NT2RP7002243, NT2RP7002282, NT2RP7002450, NT2RP7002478, NT2RP7002554, NT2RP7002594, NT2RP7002603, NT2RP7002619, NT2RP7002650, NT2RP7002738, NT2RP7002779, NT2RP7002829, NT2RP7002841, NT2RP7002906, NT2RP7002978, NT2RP7003025, NT2RP7003050, NT2RP7003084,
NT2RP7003091, NT2RP7003107, NT2RP7003134, NT2RP7003148, NT2RP7003261, NT2RP7003439, NT2RP7003511, NT2RP7003629, NT2RP7003680, NT2RP7003724, NT2RP7003960, NT2RP7004027, NT2RP7004080, NT2RP7004173, NT2RP7004204, NT2RP7004233, NT2RP7004260, NT2RP7004358, NT2RP7004396, NT2RP7004428, NT2RP7004481, NT2RP7004541, NT2RP7004641, NT2RP7004656, NT2RP7004687, NT2RP7004722, NT2RP7004766, NT2RP7004790, NT2RP7004884, NT2RP7004911, NT2RP7004961, NT2RP7004964, NT2RP7004975, NT2RP7005118, NT2RP7005205, NT2RP7005219, NT2RP7005323, NT2RP7005468, NT2RP7005493, NT2RP7005629, NT2RP7005631, NT2RP7005669, NT2RP7005846, NT2RP7006033, NT2RP7006075, NT2RP7006141, NT2RP7006160, NT2RP7006162, NT2RP7006188, NT2RP7006263,
NT2RP7006296, NT2RP7006304, NT2RP7006374, NT2RP7006395, NT2RP7006408, NT2RP7006457, NT2RP7006547, NT2RP7006601, NT2RP7006621, NT2RP7006636, NT2RP7006701, NT2RP7006853, NT2RP7006887, NT2RP7006980, NT2RP7006986, NT2RP7006995, NT2RP7007177, NT2RP7007221, NT2RP7007226, NT2RP7007269, NT2RP7007359, NT2RP7007387, NT2RP7007406, NT2RP7007480, NT2RP7007504, NT2RP7007530, NT2RP7007580, NT2RP7007594, NT2RP7007617, NT2RP7007643, NT2RP7007766, NT2RP7007842, NT2RP7007925, NT2RP7007930, NT2RP7007975, NT2RP7008013, NT2RP7008015, NT2RP7008133, NT2RP7008137, NT2RP7008142, NT2RP7008144, NT2RP7008360, NT2RP7008396, NT2RP7008406, NT2RP7008435, NT2RP7008441, NT2RP7008454, NT2RP7008487, NT2RP7008544, NT2RP7008550,
NT2RP7008557, NT2RP7008623, NT2RP7008657, NT2RP7008714, NT2RP7008720, NT2RP7008855, NT2RP7008863, NT2RP7009012, NT2RP7009019, NT2RP7009030, NT2RP7009108, NT2RP7009149, NT2RP7009168, NT2RP7009215, NT2RP7009225, NT2RP7009236, NT2RP7009253, NT2RP7009267, NT2RP7009322, NT2RP7009363; NT2RP7009370, NT2RP7009373, NT2RP7009394, NT2RP7009397, NT2RP7009429, NT2RP7009439, NT2RP7009466, NT2RP7009481, NT2RP7009498, NT2RP7009502, NT2RP7009507, NT2RP7009577, NT2RP7009743, NT2RP7009867, NT2RP7010109, NT2RP7010128, NT2RP7010235, NT2RP7010275, NT2RP7010521, NT2RP7010599, NT2RP7010817, NT2RP7011086, NT2RP7011132, NT2RP7011268, NT2RP7011318, NT2RP7011452, NT2RP7011570, NT2RP7011721, NT2RP7011909, NT2RP7012291,
NT2RP7012516, NT2RP7012776, NT2RP7013002, NT2RP7013374, NT2RP7013499, NT2RP7013729, NT2RP7013764, NT2RP7013795, NT2RP7013999, NT2RP7014005, NT2RP7014178, NT2RP7014348, NT2RP7014420, NT2RP7014583, NT2RP7014721, NT2RP7014743, NT2RP7014778, NT2RP7014906, NT2RP7014910, NT2RP7015080, NT2RP7015431, NT2RP7015503, NT2RP7015512, NT2RP7015676, NT2RP7015789, NT2RP7015996, NT2RP7016508, NT2RP7016574, NT2RP7016622, NT2RP7016911, NT2RP7017139, NT2RP7017284, NT2RP7017365, NT2RP7017546, NT2RP7017567, NT2RP7017795, NT2RP7017971, NT2RP7018032, NT2RP7018126, NT2RP7018197, NT2RP7018206, NT2RP7018340, NT2RP7018586, NT2RP7018802, NT2RP7018871, NT2RP7019064, NT2RP7019135, NT2RP7019273, NT2RP7019367, NT2RP7019401,
NT2RP7019445, NT2RP7019543, NT2RP7019682, NT2RP7019835, NT2RP7020112, NT2RP7020123, NT2RP7020343, NT2RP7020379, NT2RP8000137, NT2RP8000296, NT2RP8000435, NT2RP8000483, NT2RP8000521, NT2RP8000633, NT2RP8001363, NT2RP8001407, NT2RP8001584, NT2RP8001604, NT2RP8001605, NT2RP8003490, NT2RP8003657, NT2RP8003787, NT2RP8004306, NT2RP8005546, NT2RP8006452, NT2RP8006521, NT2RP8007416, NT2RP8007503, NT2RP8007715, NT2RP8007920, NT2RP8008057, NT2RP8009248, NTONG1000006, NTONG1000047, NTONG1000052, NTONG1000075, NTONG1000087, NTONG1000123, NTONG1000130, NTONG1000161, NTONG1000172, NTONG1000213, NTONG1000214, NTONG1000247, NTONG1000257, NTONG1000264, NTONG2000040, NTONG2000107, NTONG2000231, NTONG2000334,
NTONG2000363, NTONG2000413, NTONG2000492, NTONG2000499, NTONG2000531, NTONG2000583, NTONG2000800, NTONG2000858, NTONG2000878, NTONG2000966, NTONG2000977, NTONG2000985, NTONG2001014, NTONG2001079, NTONG2001137, NTONG2001157, NTONG2001173, NTONG2001220, NTONG2001222, NTONG2001362, NTONG2001428, NTONG2001532, NTONG2001587, NTONG2001612, NTONG2001619, NTONG2001642, NTONG2001657, NTONG2001676, NTONG2001762, NTONG2002278, NTONG2002807, NTONG2002948, NTONG2002970, NTONG2002985, NTONG2003158, NTONG2003210, NTONG2003316, NTONG2003409, NTONG2003515, NTONG2003805, NTONG2003852, NTONG2003897, NTONG2003928, NTONG2004095, NTONG2004308, NTONG2004521, NTONG2004614, NTONG2004690, NTONG2004806, NTONG2004829,
NTONG2004918, NTONG2004991, NTONG2005062, NTONG2005086, NTONG2005137, NTONG2005153, NTONG2005265, NTONG2005277, NTONG2005373, NTONG2005480, NTONG2005497, NTONG2005577, NTONG2005657, NTONG2005822, NTONG2005969, NTONG2006099, NTONG2006187, NTONG2006301, NTONG2006324, NTONG2006354, NTONG2006440, NTONG2006484, NTONG2006501, NTONG2006646, NTONG2006709, NTONG2006783, NTONG2007020, NTONG2007028, NTONG2007034, NTONG2007249, NTONG2007517, NTONG2007522, NTONG2007658, NTONG2007693, NTONG2007756, NTONG2008088, NTONG2008093, NTONG2008143, NTONG2008365, NTONG2008483, NTONG2008522, NTONG2008672, NTONG2008862, NTONG2008939, NTONG2009060, NTONG2009068, NTONG2009229, NTONG2009233, NTONG2009468, NTONG2009576,
OCBBF1000016, OCBBF1000030, OCBBF1000042, OCBBF1000049, OCBBF1000054, OCBBF1000067, OCBBF1000080, OCBBF1000085, OCBBF1000091, OCBBF1000104, OCBBF1000114, OCBBF1000118, OCBBF1000119, OCBBF1000122, OCBBF1000130, OCBBF1000138, OCBBF1000145, OCBBF1000146, OCBBF1000153, OCBBF1000185, OCBBF1000188, OCBBF1000192, OCBBF1000254, OCBBF1000315, OCBBF2000013, OCBBF2000126, OCBBF2000178, OCBBF2000231, OCBBF2000277, OCBBF2000287, OCBBF2000323, OCBBF2000452, OCBBF2000522, OCBBF2000703, OCBBF2000712, OCBBF2000719, OCBBF2000824, OCBBF2000831, OCBBF2000846, OCBBF2000904, OCBBF2000982, OCBBF2000998, OCBBF2001075, OCBBF2001101, OCBBF2001124, OCBBF2001140, OCBBF2001176, OCBBF2001186, OCBBF2001196, OCBBF2001252,
OCBBF2001307, OCBBF2001389, OCBBF2001402, OCBBF2001408, OCBBF2001416, OCBBF2001473, OCBBF2001492, OCBBF2001494, OCBBF2001527, OCBBF2001528, OCBBF2001639, OCBBF2001681, OCBBF2001687, OCBBF2001706, OCBBF2001749, OCBBF2001794, OCBBF2001910, OCBBF2001931, OCBBF2001983, OCBBF2002015, OCBBF2002083, OCBBF2002124, OCBBF2002161, OCBBF2002290, OCBBF2002357, OCBBF2002376, OCBBF2002615, OCBBF2002626, OCBBF2002663, OCBBF2002805, OCBBF2002865, OCBBF2002920, OCBBF2002980, OCBBF2003028, OCBBF2003052, OCBBF2003091, OCBBF2003327, OCBBF2003518, OCBBF2003543, OCBBF2003593, OCBBF2003744, OCBBF2003819, OCBBF2004038, OCBBF2004104, OCBBF2004147, OCBBF2004168, OCBBF2004235, OCBBF2004259, OCBBF2004385, OCBBF2004418,
OCBBF2004478, OCBBF2004482, OCBBF2004533, OCBBF2004567, OCBBF2004612, OCBBF2004647, OCBBF2004669, OCBBF2004826, OCBBF2004866, OCBBF2004883, OCBBF2004889, OCBBF2004930, OCBBF2004984, OCBBF2005066, OCBBF2005077, OCBBF2005161, OCBBF2005343, OCBBF2005349, OCBBF2005373, OCBBF2005420, OCBBF2005428, OCBBF2005433, OCBBF2005546, OCBBF2005843, OCBBF2005901, OCBBF2006005, OCBBF2006030, OCBBF2006058, OCBBF2006113, OCBBF2006148, OCBBF2006151, OCBBF2006154, OCBBF2006172, OCBBF2006214, OCBBF2006241, OCBBF2006388, OCBBF2006438, OCBBF2006567, OCBBF2006624, OCBBF2006639, OCBBF2006660, OCBBF2006764, OCBBF2006859, OCBBF2006972, OCBBF2006987, OCBBF2007039, OCBBF2007051, OCBBF2007068, OCBBF2007114, OCBBF2007121,
OCBBF2007184, OCBBF2007194, OCBBF2007196, OCBBF2007224, OCBBF2007238, OCBBF2007354, OCBBF2007414, OCBBF2007428, OCBBF2007478, OCBBF2007485, OCBBF2007520, OCBBF2007555, OCBBF2007610, OCBBF2007622, OCBBF2007756, OCBBF2007762, OCBBF2007892, OCBBF2007931, OCBBF2007946, OCBBF2008041, OCBBF2008116, OCBBF2008138, OCBBF2008283, OCBBF2008330, OCBBF2008340, OCBBF2008366, OCBBF2008466, OCBBF2008483, OCBBF2008511, OCBBF2008520, OCBBF2008569, OCBBF2008586, OCBBF2008640, OCBBF2008691, OCBBF2008701, OCBBF2008724, OCBBF2008760, OCBBF2008768, OCBBF2008770, OCBBF2008775, OCBBF2008790, OCBBF2008814, OCBBF2008822, OCBBF2009095, OCBBF2009115, OCBBF2009352, OCBBF2009391, OCBBF2009536, OCBBF2009571, OCBBF2009583,
OCBBF2009603, OCBBF2009788, OCBBF2009836, OCBBF2009920, OCBBF2009926, OCBBF2010040, OCBBF2010140, OCBBF2010281, OCBBF2010404, OCBBF2010416, OCBBF2010420, OCBBF2010604, OCBBF2010709, OCBBF2010792, OCBBF2010819, OCBBF2010841, OCBBF2010843, OCBBF2010858, OCBBF2010863, OCBBF2010871, OCBBF2010931, OCBBF2010978, OCBBF2010989, OCBBF2011073, OCBBF2011137, OCBBF2011160, OCBBF2011177, OCBBF2011283, OCBBF2011311, OCBBF2011625, OCBBF2011669, OCBBF2011685, OCBBF2011706, OCBBF2011722, OCBBF2011767, OCBBF2011855, OCBBF2011872, OCBBF2011887, OCBBF2011897, OCBBF2011914, OCBBF2011960, OCBBF2011981, OCBBF2012001, OCBBF2012028, OCBBF2012039, OCBBF2012095, OCBBF2012139, OCBBF2012191, OCBBF2012262, OCBBF2012320,
OCBBF2012436, OCBBF2012525, OCBBF2012553, OCBBF2012678, OCBBF2012704, OCBBF2012714, OCBBF2012755, OCBBF2012812, OCBBF2012821, OCBBF2012881, OCBBF2012908, OCBBF2012936, OCBBF2012990, OCBBF2013011, OCBBF2013049, OCBBF2013091, OCBBF2013123, OCBBF2013127, OCBBF2013208, OCBBF2013285, OCBBF2013319, OCBBF2013324, OCBBF2013366, OCBBF2013481, OCBBF2013507, OCBBF2013721, OCBBF2013789, OCBBF2013883, OCBBF2013926, OCBBF2014052, OCBBF2014089, OCBBF2014322, OCBBF2014386, OCBBF2014541, OCBBF2014576, OCBBF2014707, OCBBF2014745, OCBBF2014828, OCBBF2014873, OCBBF2014880, OCBBF2014928, OCBBF2015031, OCBBF2015081, OCBBF2015115, OCBBF2015232, OCBBF2015233, OCBBF2015285, OCBBF2015334, OCBBF2015335, OCBBF2015503,
OCBBF2015506, OCBBF2015599, OCBBF2015645, OCBBF2015659, OCBBF2015751, OCBBF2015797, OCBBF2015931, OCBBF2015980, OCBBF2016038, OCBBF2016405, OCBBF2016467, OCBBF2016590, OCBBF2016591, OCBBF2016612, OCBBF2016689, OCBBF2016690, OCBBF2016729, OCBBF2016841, OCBBF2016928, OCBBF2017035, OCBBF2017055, OCBBF2017069, OCBBF2017306, OCBBF2017325, OCBBF2017458, OCBBF2017489, OCBBF2017516, OCBBF2017665, OCBBF2017815, OCBBF2017888, OCBBF2017893, OCBBF2017920, OCBBF2018014, OCBBF2018084, OCBBF2018167, OCBBF2018206, OCBBF2018229, OCBBF2018234, OCBBF2018362, OCBBF2018380, OCBBF2018581, OCBBF2018618, OCBBF2018663, OCBBF2018687, OCBBF2018707, OCBBF2018827, OCBBF2018828, OCBBF2018934, OCBBF2019108, OCBBF2019195,
OCBBF2019327, OCBBF2019684, OCBBF2019761, OCBBF2019823, OCBBF2019950, OCBBF2020048, OCBBF2020318, OCBBF2020343, OCBBF2020453, OCBBF2020801, OCBBF2020825, OCBBF2020838, OCBBF2021020, OCBBF2021214, OCBBF2021286, OCBBF2021323, OCBBF2021518, OCBBF2021788, OCBBF2022351, OCBBF2022573, OCBBF2022574, OCBBF2023162, OCBBF2023598, OCBBF2023643, OCBBF2024463, OCBBF2024589, OCBBF2024719, OCBBF2024779, OCBBF2024781, OCBBF2025028, OCBBF2025115, OCBBF2025198, OCBBF2025451, OCBBF2025458, OCBBF2025527, OCBBF2025631, OCBBF2025730, OCBBF2026025, OCBBF2026035, OCBBF2026144, OCBBF2026645, OCBBF2026649, OCBBF2026690, OCBBF2026981, OCBBF2027197, OCBBF2027354, OCBBF2027423, OCBBF2027478, OCBBF2027481, OCBBF2027511,
OCBBF2027661, OCBBF2027728, OCBBF2027782, OCBBF2027868, OCBBF2028055, OCBBF2028173, OCBBF2028421, OCBBF2029471, OCBBF2029901, OCBBF2030116, OCBBF2030329, OCBBF2030354, OCBBF2030517, OCBBF2030574, OCBBF2030708, OCBBF2030927, OCBBF2030963, OCBBF2031167, OCBBF2031198, OCBBF2031366, OCBBF2031561, OCBBF2032152, OCBBF2032274, OCBBF2032539, OCBBF2032590, OCBBF2032599, OCBBF2032611, OCBBF2032671, OCBBF2033295, OCBBF2033413, OCBBF2033869, OCBBF2033948, OCBBF2034333, OCBBF2034529, OCBBF2034637, OCBBF2034906, OCBBF2035110, OCBBF2035214, OCBBF2035226, OCBBF2035390, OCBBF2035564, OCBBF2035658, OCBBF2035823, OCBBF2035885, OCBBF2035916, OCBBF2036019, OCBBF2036031, OCBBF2036225, OCBBF2036476, OCBBF2036752, OCBBF2037068, OCBBF2037340, OCBBF2037398, OCBBF2037464, OCBBF2037547, OCBBF2037598, OCBBF2037638, OCBBF2038238, OCBBF2038285, OCBBF2038317, OCBBF3000097, OCBBF3000213, OCBBF3000296, OCBBF3000323, OCBBF3000372, OCBBF3000380, OCBBF3000743, OCBBF3000830, OCBBF3001076, OCBBF3001202, OCBBF3001616, OCBBF3002553, OCBBF3002600, OCBBF3002654, OCBBF3003103, OCBBF3003209, OCBBF3003592, OCBBF3003745, OCBBF3003761, OCBBF3004264, OCBBF3004314, OCBBF3004972, OCBBF3005330, OCBBF3005597, OCBBF3005843, OCBBF3006802, OCBBF3006986, OCBBF3007078, OCBBF3007704, OCBBF3008230, OCBBF3008392, OCBBF3008835, OCBBF3009192, OCBBF3009244, OCBBF3009279, PANCR1000012, PANCR1000021, PANCR1000081, PANCR1000086, PANCR1000091,
PANCR1000185, PEBLM1000024, PEBLM1000029, PEBLM1000034, PEBLM1000062, PEBLM1000066, PEBLM1000068, PEBLM1000071, PEBLM1000083, PEBLM1000147, PEBLM1000174, PEBLM1000180, PEBLM2000112, PEBLM2000126, PEBLM2000170, PEBLM2000180, PEBLM2000213, PEBLM2000222, PEBLM2000248, PEBLM2000267, PEBLM2000270, PEBLM2000308, PEBLM2000321, PEBLM2000333, PEBLM2000338, PEBLM2000395, PEBLM2000479, PEBLM2000502, PEBLM2001072, PEBLM2001312, PEBLM2001465, PEBLM2001488, PEBLM2001803, PEBLM2002432, PEBLM2002455, PEBLM2002594, PEBLM2002749, PEBLM2002887, PEBLM2003426, PEBLM2003765, PEBLM2003914, PEBLM2003935, PEBLM2004015, PEBLM2004216, PEBLM2004290, PEBLM2004497, PEBLM2004666, PEBLM2004733, PEBLM2005282, PEBLM2005615,
PEBLM2005632, PEBLM2005697, PEBLM2006031, PEBLM2006036, PEBLM2006049, PEBLM2006113, PEBLM2006135, PEBLM2006215, PEBLM2006283, PEBLM2006298, PEBLM2006912, PEBLM2007112, PEBLM2007140, PEBLM2007188, PEBLM2007296, PEBLM2007437, PEBLM2007447, PEBLM2007598, PEBLM2007774, PEBLM2007832, PEBLM2007834, PEBLM2008106, PEBLM2008576, PEBLM2008605, PEBLM2008826, PEBLM2008861, PERIC1000025, PERIC1000147, PERIC2000180, PERIC2000214, PERIC2000386, PERIC2000387, PERIC2000422, PERIC2000473, PERIC2000478, PERIC2000889, PERIC2000914, PERIC2001227, PERIC2001228, PERIC2001379, PERIC2001504, PERIC2001703, PERIC2001764, PERIC2002243, PERIC2002272, PERIC2002452, PERIC2002766, PERIC2003001, PERIC2003090, PERIC2003287,
PERIC2003349, PERIC2003452, PERIC2003699, PERIC2003720, PERIC2003794, PERIC2003834, PERIC2003919, PERIC2003957, PERIC2004028, PERIC2004131, PERIC2004259, PERIC2004379, PERIC2004429, PERIC2004510, PERIC2004613, PERIC2004909, PERIC2005111, PERIC2005117, PERIC2005305, PERIC2005347, PERIC2005370, PERIC2005936, PERIC2006035, PERIC2006121, PERIC2006945, PERIC2006960, PERIC2007439, PERIC2007914, PERIC2008008, PERIC2008385, PERIC2009001, PERIC2009086, PERIC2009329, PERIC2009566, PLACE5000001, PLACE5000037, PLACE5000058, PLACE5000066, PLACE5000067, PLACE5000068, PLACE5000070, PLACE5000080, PLACE5000105, PLACE5000115, PLACE5000116, PLACE5000129, PLACE5000139, PLACE5000153, PLACE5000171, PLACE5000172,
PLACE5000178, PLACE5000245, PLACE5000260, PLACE5000282, PLACE5000297, PLACE5000492, PLACE5000522, PLACE5000527, PLACE6000012, PLACE6000044, PLACE6000055, PLACE6000070, PLACE6000137, PLACE6000191, PLACE6000205, PLACE6000231, PLACE6000296, PLACE6000314, PLACE6000325, PLACE6000348, PLACE6000371, PLACE6000424, PLACE6000426, PLACE6000429, PLACE6000450, PLACE6000463, PLACE6000523, PLACE6000550, PLACE6000552, PLACE6000555, PLACE6000619, PLACE6000630, PLACE6000953, PLACE6001118, PLACE6001153, PLACE6001262, PLACE6001294, PLACE6001536, PLACE6001557, PLACE6001712, PLACE6001740, PLACE6001823, PLACE6001923, PLACE6001925, PLACE6001933, PLACE6002011, PLACE6002016, PLACE6002056, PLACE6002157, PLACE6002312,
PLACE6002323, PLACE6002345, PLACE6002419, PLACE6002462, PLACE6002647, PLACE6002692, PLACE6002699, PLACE6002710, PLACE6002871, PLACE6002949, PLACE6003004, PLACE6003038, PLACE6003077, PLACE6003094, PLACE6003109, PLACE6003204, PLACE6003372, PLACE6003393, PLACE6003399, PLACE6003539, PLACE6003621, PLACE6003705, PLACE6003740, PLACE6003745, PLACE6003771, PLACE6003796, PLACE6003839, PLACE6003850, PLACE6004101, PLACE6004219, PLACE6004336, PLACE6004368, PLACE6004380, PLACE6004396, PLACE6004454, PLACE6004464, PLACE6004578, PLACE6004663, PLACE6004687, PLACE6004931, PLACE6004966, PLACE6004993, PLACE6005007, PLACE6005029, PLACE6005108, PLACE6005231, PLACE6005234, PLACE6005283, PLACE6005294, PLACE6005328,
PLACE6005351, PLACE6005423, PLACE6005487, PLACE6005535, PLACE6005546, PLACE6005559, PLACE6005579, PLACE6005691, PLACE6005786, PLACE6006077, PLACE6006158, PLACE6006217, PLACE6006222, PLACE6006266, PLACE6006287, PLACE6006394, PLACE6006418, PLACE6006474, PLACE6006566, PLACE6006697, PLACE6006822, PLACE6006871, PLACE6006905, PLACE6006988, PLACE6007050, PLACE6007180, PLACE6007239, PLACE6007242, PLACE6007309, PLACE6007373, PLACE6007380, PLACE6007433, PLACE6007436, PLACE6007482, PLACE6007687, PLACE6007787, PLACE6007798, PLACE6007807, PLACE6007925, PLACE6008036, PLACE6008094, PLACE6008126, PLACE6008285, PLACE6008444, PLACE6008453', PLACE6008640, PLACE6008750, PLACE6008768, PLACE6008793, PLACE6008989,
PLACE6009006, PLACE6009008, PLACE6009228, PLACE6009237, PLACE6009419, PLACE6009430, PLACE6009482, PLACE6009524, PLACE6009527, PLACE6009560, PLACE6009590, PLACE6009821, PLACE6009835, PLACE6009853, PLACE6010463, PLACE6010484, PLACE6010485, PLACE6010568, PLACE6010701, PLACE6010704, PLACE6010732, PLACE6010765, PLACE6010848, PLACE6010925, PLACE6010936, PLACE6010991, PLACE6011041, PLACE6011102, PLACE6011156, PLACE6011211, PLACE6011260, PLACE6011334, PLACE6011627, PLACE6011774, PLACE6011881, PLACE6011907, PLACE6011970, PLACE6011975, PLACE6012028, PLACE6012108, PLACE6012168, PLACE6012297, PLACE6012308, PLACE6012574, PLACE6012908, PLACE6012936, PLACE6013222, PLACE6013232, PLACE6013288, PLACE6013386,
PLACE6013400, PLACE6013650, PLACE6013672, PLACE6013883, PLACE6013884, PLACE6013885, PLACE6014043, PLACE6014064, PLACE6014516, PLACE6014882, PLACE6015051, PLACE6015211, PLACE6015322, PLACE6015445, PLACE6015460, PLACE6015513, PLACE6015591, PLACE6015729, PLACE6015939, PLACE6016030, PLACE6016093, PLACE6016105, PLACE6016160, PLACE6016210, PLACE6016254, PLACE6016942, PLACE6016984, PLACE6017002, PLACE6017564, PLACE6017578, PLACE6017626, PLACE6017701, PLACE6017714, PLACE6017788, PLACE6017791, PLACE6018031, PLACE6018107, PLACE6018187, PLACE6018235, PLACE6018287, PLACE6018441, PLACE6018487, PLACE6018497, PLACE6018769, PLACE6018834, PLACE6018843, PLACE6018863, PLACE6019195, PLACE6019385, PLACE6019542,
PLACE6019600, PLACE6019674, PLACE6019676, PLACE6019701, PLACE6019932, PLACE6020031, PLACE6020145, PLACE7000167, PLACE7000170, PLACE7000266, PLACE7000333, PLACE7000410, PLACE7000502, PLACE7000707, PLACE7001022, PLACE7001544, PLACE7001759, PLACE7001936, PLACE7002303, PLACE7002641, PLACE7003639, PLACE7003657, PLACE7003684, PLACE7003985, PLACE7004103, PLACE7004961, PLACE7005169, PLACE7005671, PLACE7005840, PLACE7006051, PLACE7006090, PLACE7006268, PLACE7006275, PLACE7006498, PLACE7006540, PLACE7007379, PLACE7007644, PLACE7007973, PLACE7008136, PLACE7008261, PLACE7008431, PLACE7008623, PLACE7008766, PLACE7009563, PROST1000033, PROST1000039, PROST1000065, PROST1000083, PROST1000085, PROST1000110,
PROST1000120, PROST1000123, PROST1000136, PROST1000144, PROST1000145, PROST1000152, PROST1000167, PROST1000184, PROST1000215, PROST1000217, PROST1000262, PROST1000272, PROST1000298, PROST1000322, PROST1000334, PROST1000343, PROST1000362, PROST1000377, PROST1000383, PROST1000412, PROST1000419, PROST1000451, PROST1000480, PROST1000485, PROST1000493, PROST1000512, PROST1000527, PROST1000528, PROST1000536, PROST1000559, PROST1000575, PROST2000036, PROST2000053, PROST20.00067, PROST2000079, PROST2000105, PROST2000143, PROST2000162, PROST2000165, PROST2000176, PROST2000203, PROST2000206, PROST2000212, PROST2000241, PROST2000267, PROST2000274, PROST2000277, PROST2000284, PROST2000325, PROST2000337,
PROST2000432, PROST2000452, PROST2000463, PROST2000470, PROST2000505, PROST2000567, PROST2000572, PROST2000717, PROST2000760, PROST2000761, PROST2000835, PROST2000893, PROST2000961, PROST2001002, PROST2001116, PROST2001138, PROST2001180, PROST2001213, PROST2001283, PROST2001414, PROST2001415, PROST2001465, PROST2001521, PROST2001540, PROST2001552, PROST2001599, PROST2001611, PROST2001636, PROST2001739, PROST2001796, PROST2001805, PROST2001823, PROST2001883, PROST2001899, PROST2001997, PROST2001998, PROST2002078, PROST2002101, PROST2002162, PROST2002212, PROST2002227, PROST2002338, PROST2002425, PROST2002488, PROST2002489, PROST2002527, PROST2002591, PROST2002602, PROST2002633, PROST2002682,
PROST2002685, PROST2002722, PROST2002736, PROST2002842, PROST2002897, PROST2002906, PROST2002927, PROST2002960, PROST2003102, PROST2003117, PROST2003210, PROST2003302, PROST2003303, PROST2003324, PROST2003340, PROST2003428, PROST2003472, PROST2003517, PROST2003537, PROST2003563, PROST2003577, PROST2003583, PROST2003586, PROST2003628, PROST2003635, PROST2003674, PROST2003676, PROST2003725, PROST2003732, PROST2003775, PROST2003922, PROST2003930, PROST2004115, PROST2004142, PROST2004146, PROST2004161, PROST2004251, PROST2004332, PROST2004481, PROST2004526, PROST2004570, PROST2004727, PROST2004739, PROST2004742, PROST2004744, PROST2004817, PROST2005039, PROST2005067, PROST2005076, PROST2005093,
PROST2005121, PROST2005131, PROST2005143, PROST2005228, PROST2005272, PROST2005426, PROST2005466, PROST2005630, PROST2005683, PROST2005788, PROST2005793, PROST2005880, PROST2005886, PROST2005904, PROST2005919, PROST2005943, PROST2006008, PROST2006020, PROST2006060, PROST2006196, PROST2006201, PROST2006260, PROST2006282, PROST2006343, PROST2006450, PROST2006491, PROST2006510, PROST2006579, PROST2006688, PROST2006766, PROST2006782, PROST2006933, PROST2006988, PROST2007122, PROST2007237, PROST2007248, PROST2007289, PROST2007317, PROST2007328, PROST2007382, PROST2007389, PROST2007416, PROST2007528, PROST2007612, PROST2007818, PROST2007871, PROST2007950, PROST2007956, PROST2007974, PROST2007988,
PROST2008079, PROST2008088, PROST2008243, PROST2008245, PROST2008268, PROST2008360, PROST2008447, PROST2008468, PROST2008472, PROST2008516, PROST2008724, PROST2008835, PROST2008993, PROST2009022, PROST2009208, PROST2009222, PROST2009254, PROST2009273, PROST2009320, PROST2009347, PROST2009365, PROST2009388, PROST2009400, PROST2009470, PROST2009483, PROST2009571, PROST2009731, PROST2009736, PROST2009795, PROST2009901, PROST2009909, PROST2010046, PROST2010219, PROST2010250, PROST2010326, PROST2010382, PROST2010400, PROST2010579, PROST2010606, PROST2010885, PROST2011012, PROST2011038, PROST2011105, PROST2011297, PROST2011410, PROST2011439, PROST2011482, PROST2011577, PROST2011631, PROST2011660,
PROST2012005, PROST2012016, PROST2012088, PROST2012140, PROST2012157, PROST2012190, PROST2012249, PROST2012353, PROST2012400, PROST2012504, PROST2012542, PROST2012550, PROST2012740, PROST2012745, PROST2012780, PROST2012888, PROST2013032, PROST2013053, PROST2013121, PROST2013260, PROST2013327, PROST2013531, PROST2013605, PROST2013627, PROST2013873, PROST2013880, PROST2014422, PROST2014585, PROST2014601, PROST2014659, PROST2014862, PROST2014925, PROST2015124, PROST2015137, PROST2015198, PROST2015243, PROST2015246, PROST2015251, PROST2015287, PROST2015332, PROST2015457, PROST2015464, PROST2015537, PROST2015545, PROST2015551, PROST2015636, PROST2015710, PROST2015756, PROST2015877, PROST2015900,
PROST2015932, PROST2016195, PROST2016246, PROST2016351, PROST2016379, PROST2016444, PROST2016462, PROST2016499, PROST2016512, PROST2016566, PROST2016582, PROST2016668, PROST2016684, PROST2016777, PROST2016829, PROST2016860, PROST2016980, PROST2017098, PROST2017128, PROST2017367, PROST2017413, PROST2017441, PROST2017529, PROST2017578, PROST2017612, PROST2017700, PROST2017701, PROST2017729, PROST2017836, PROST2017910, PROST2017972, PROST2018030, PROST2018090, PROST2018235, PROST2018437, PROST2018487, PROST2018511, PROST2018583, PROST2018588, PROST2018607, PROST2018788, PROST2018902, PROST2018922, PROST2019164, PROST2019253, PROST2019296, PROST2019398, PROST2019487, PROST2019781, PUAEN1000039,
PUAEN1000081, PUAEN1000085, PUAEN1000087, PUAEN1000121, PUAEN1000147, PUAEN1000161, PUAEN1000175, PUAEN1000275, PUAEN1000276, PUAEN1000288, PUAEN1000322, PUAEN2000080, PUAEN2000152, PUAEN2000175, PUAEN2000247, PUAEN2000312, PUAEN2000394, PUAEN2000497, PUAEN2000535, PUAEN2000594, PUAEN2000684, PUAEN2000942, PUAEN2001260, PUAEN2001345, PUAEN2001526, PUAEN2001882, PUAEN2002473, PUAEN2002489, PUAEN2002552, PUAEN2002568, PUAEN2002616, PUAEN2002758, PUAEN2003018, PUAEN2003079, PUAEN2003116, PUAEN2003408, PUAEN2003517, PUAEN2003954, PUAEN2004067, PUAEN2004083, PUAEN2004400, PUAEN2004511, PUAEN2004525, PUAEN2004875, PUAEN2005110, PUAEN2005247, PUAEN2005502, PUAEN2005588, PUAEN2005930, PUAEN2006029,
PUAEN2006328, PUAEN2006335, PUAEN2006578, PUAEN2006639, PUAEN2006761, PUAEN2007350, PUAEN2007785, PUAEN2007898, PUAEN2008123, PUAEN2008314, PUAEN2008515, PUAEN2008815, PUAEN2008939, PUAEN2008980, PUAEN2008985, PUAEN2009174, PUAEN2009348, PUAEN2009534, PUAEN2009795, PUAEN2009852, PUAEN2010346, PUAEN2010824, RECTM1000051, RECTM1000141, RECTM2000220, RECTM2000349, RECTM2000433, RECTM2000510, RECTM2001307, RECTM2001347, RECTM2001519, RECTM2001666, RECTM2001691, RECTM2001749, RECTM2002172, RECTM2002602, RECTM2002632, SALGL1000005, SALGL1000024, SALGL1000028, SALGL1000047, SALGL1000065, SALGL1000107, SALGL1000171, SKMUS1000030, SKMUS1000064, SKMUS1000067, SKMUS1000102, SKMUS1000104, SKMUS1000110,
SKMUS1000118, SKMUS1000124, SKMUS1000129, SKMUS1000176, SKMUS2000061, SKMUS2000074, SKMUS2000078, SKMUS2000117, SKMUS2000137, SKMUS2000198, SKMUS2000243, SKMUS2000271, SKMUS2000287, SKMUS2000317, SKMUS2000330, SKMUS2000339, SKMUS2000365, SKMUS2000390, SKMUS2000416, SKMUS2000458, SKMUS2000467, SKMUS2000468, SKMUS2000484, SKMUS2000515, SKMUS2000679, SKMUS2000690, SKMUS2000701, SKMUS2000705, SKMUS2000710, SKMUS2000724, SKMUS2000726, SKMUS2000757, SKMUS2000774, SKMUS2000780, SKMUS2000821, SKMUS2000847, SKMUS2000863, SKMUS2000894, SKMUS2000898, SKMUS2000933, SKMUS2000945, SKMUS2000961, SKMUS2000973, SKMUS2000980, SKMUS2001008, SKMUS2001014, SKMUS2001147, SKMUS2001157, SKMUS2001164, SKMUS2001199,
SKMUS2001201, SKMUS2001215, SKMUS2001247, SKMUS2001254, SKMUS2001271, SKMUS2001287, SKMUS2001323, SKMUS2001364, SKMUS2001398, SKMUS2001402, SKMUS2001412, SKMUS2001492, SKMUS2001528, SKMUS2001543, SKMUS2001580, SKMUS2001585, SKMUS2001608, SKMUS2001631, SKMUS2001634, SKMUS2001662, SKMUS2001663, SKMUS2001668, SKMUS2001671, SKMUS2001683, SKMUS2001693, SKMUS2001695, SKMUS2001703, SKMUS2001740, SKMUS2001742, SKMUS2001823, SKMUS2001850, SKMUS2002077, SKMUS2002084, SKMUS2002153, SKMUS2002475, SKMUS2002602, SKMUS2002634, SKMUS2002693, SKMUS2002821, SKMUS2002840, SKMUS2002920, SKMUS2003034, SKMUS2003074, SKMUS2003168, SKMUS2003194, SKMUS2003744, SKMUS2004044, SKMUS2004047, SKMUS2004483, SKMUS2004667,
SKMUS2004897, SKMUS2004903, SKMUS2004908, SKMUS2005428, SKMUS2006394, SKMUS2006481, SKMUS2006755, SKMUS2007315, SKMUS2007359, SKMUS2007568, SKMUS2007740, SKMUS2007816, SKMUS2007915, SKMUS2008338, SKMUS2008407, SKMUS2008474, SKMUS2008585, SKMUS2008607, SKMUS2009190, SKMUS2009232, SKMUS2009479, SKMUS2009557, SKNMC1000123, SKNMC1000124, SKNMC1000159, SKNMC1000229, SKNMC1000264, SKNMC2000224, SKNMC2000256, SKNMC2000322, SKNMC2000356, SKNMC2000374, SKNMC2000583, SKNMC2000593, SKNMC2000612, SKNMC2000622, SKNMC2000698, SKNMC2000966, SKNMC2001113, SKNMC2001324, SKNMC2001503, SKNMC2001555, SKNMC2001596, SKNMC2001623, SKNMC2002402, SKNMC2003639, SKNMC2003924, SKNMC2003987, SKNMC2004457, SKNMC2004643,
SKNMC2004651, SKNMC2005772, SKNMC2006173, SKNMC2006327, SKNMC2006998, SKNMC2007134, SKNMC2007502, SKNMC2007504, SKNMC2007961, SKNMC2009450, SKNSH1000002, SKNSH1000101, SKNSH1000174, SKNSH1000301, SKNSH1000308, SKNSH1000416, SKNSH2000051, SKNSH2000101, SKNSH2000151, SKNSH2000163, SKNSH2000250, SKNSH2000319, SKNSH2000516, SKNSH2000550, SKNSH2000716, SKNSH2000971, SKNSH2001222, SKNSH2001875, SKNSH2001931, SKNSH2002054, SKNSH2002768, SKNSH2002866, SKNSH2003064, SKNSH2003174, SKNSH2003422, SKNSH2003466, SKNSH2003483, SKNSH2003633, SKNSH2004039, SKNSH2005061, SKNSH2005120, SKNSH2005194, SKNSH2005240, SKNSH2005792, SKNSH2005816, SKNSH2005981, SKNSH2006234, SKNSH2006304, SKNSH2006432, SKNSH2006822,
SKNSH2007142, SKNSH2007149, SKNSH2007429, SKNSH2007739, SKNSH2008940, SKNSH2008969, SKNSH2009197, SKNSH2009357, SKNSH2009435, SKNSH2009991, SKNSH2010015, SMINT1000012, SMINT1000016, SMINT1000039, SMINT1000048, SMINT1000051, SMINT1000054, SMINT1000057, SMINT1000071, SMINT1000075, SMINT1000078, SMINT1000091, SMINT1000103, SMINT1000118, SMINT1000137, SMINT1000192, SMINT2000007, SMINT2000018, SMINT2000032, SMINT2000040, SMINT2000060, SMINT2000073, SMINT2000084, SMINT2000145, SMINT2000176, SMINT2000185, SMINT2000227, SMINT2000229, SMINT2000232, SMINT2000239, SMINT2000267, SMINT2000400, SMINT2000454, SMINT2000518, SMINT2000529, SMINT2000530, SMINT2000537, SMINT2000538, SMINT2000541, SMINT2000558,
SMINT2000562, SMINT2000568, SMINT2000602, SMINT2000609, SMINT2000659, SMINT2000694, SMINT2000747, SMINT2000811, SMINT2000824, SMINT2000933, SMINT2000984, SMINT2001114, SMINT2001158, SMINT2001183, SMINT2001199, SMINT2001222, SMINT2001348, SMINT2001397, SMINT2001458, SMINT2001461, SMINT2001535, SMINT2001559, SMINT2001609, SMINT2001615, SMINT2001683, SMINT2001731, SMINT2001812, SMINT2001818, SMINT2002126, SMINT2002202, SMINT2002210, SMINT2002224, SMINT2002281, SMINT2002311, SMINT2002328, SMINT2002414, SMINT2002457, SMINT2002620, SMINT2002743, SMINT2002880, SMINT2002882, SMINT2002884, SMINT2002899, SMINT2002974, SMINT2003003, SMINT2003074, SMINT2003128, SMINT2003150, SMINT2003317, SMINT2003340,
SMINT2003386, SMINT2003423, SMINT2003505, SMINT2003551, SMINT2003569, SMINT2003641, SMINT2003644, SMINT2003866, SMINT2003905, SMINT2004023, SMINT2004037, SMINT2004047, SMINT2004086, SMINT2004144, SMINT2004280, SMINT2004299, SMINT2004339, SMINT2004350, SMINT2004414, SMINT2004473, SMINT2004547, SMINT2004583, SMINT2004729, SMINT2004781, SMINT2004872, SMINT2004891, SMINT2004909, SMINT2004972, SMINT2004992, SMINT2005075, SMINT2005161, SMINT2005174, SMINT2005213, SMINT2005330, SMINT2005387, SMINT2005402, SMINT2005405, SMINT2005621, SMINT2005623, SMINT2005624, SMINT2005688, SMINT2005800, SMINT2006078, SMINT2006205, SMINT2006331, SMINT2006596, SMINT2006641, SMINT2006648, SMINT2006708, SMINT2006716,
SMINT2007062, SMINT2007101, SMINT2007140, SMINT2007219, SMINT2007294, SMINT2007365, SMINT2007391, SMINT2007433, SMINT2007508, SMINT2007526, SMINT2007579, SMINT2007647, SMINT2007729, SMINT2007790, SMINT2007792, SMINT2007796, SMINT2007867, SMINT2007932, SMINT2008042, SMINT2008054, SMINT2008133, SMINT2008277, SMINT2008329, SMINT2008455, SMINT2008491, SMINT2008531, SMINT2008545, SMINT2008558, SMINT2008590, SMINT2008625, SMINT2008672, SMINT2008690, SMINT2008715, SMINT2008720, SMINT2008817, SMINT2008844, SMINT2008869, SMINT2008917, SMINT2008921, SMINT2009119, SMINT2009121, SMINT2009212, SMINT2009216, SMINT2009233, SMINT2009259, SMINT2009272, SMINT2009292, SMINT2009363, SMINT2009415, SMINT2009468,
SMINT2009505, SMINT2009529, SMINT2009832, SMINT2009895, SMINT2009902, SMINT2009973, SMINT2010015, SMINT2010068, SMINT2010076, SMINT2010084, SMINT2010144, SMINT2010200, SMINT2010278, SMINT2010324, SMINT2010326, SMINT2010369, SMINT2010426, SMINT2010500, SMINT2010533, SMINT2010629, SMINT2010639, SMINT2010672, SMINT2010753, SMINT2010853, SMINT2010897, SMINT2010901, SMINT2010997, SMINT2011066, SMINT2011273, SMINT2011311, SMINT2011406, SMINT2011509, SMINT2011567, SMINT2011588, SMINT2011656, SMINT2011888, SMINT2011958, SMINT2012040, SMINT2012179, SMINT2012195, SMINT2012285, SMINT2012291, SMINT2012501, SMINT2012734, SMINT2012793, SMINT2013032, SMINT2013129, SMINT2013170, SMINT2013181, SMINT2013228,
SMINT2013613, SMINT2013626, SMINT2013833, SMINT2013890, SMINT2014166, SMINT2014282, SMINT2014443, SMINT2014489, SMINT2014721, SMINT2015006, SMINT2015294, SMINT2015306, SMINT2015326, SMINT2015353, SMINT2015454, SMINT2015518, SMINT2015745, SMINT2015787, SMINT2015810, SMINT2016122, SMINT2016146, SMINT2016286, SMINT2016313, SMINT2016396, SMINT2016412, SMINT2016440, SMINT2016477, SMINT2016544, SMINT2016857, SMINT2017134, SMINT2017319, SMINT2017324, SMINT2017410, SMINT2017431, SMINT2017432, SMINT2017436, SMINT2017599, SMINT2017659, SMINT2017736, SMINT2017781, SMINT2017855, SMINT2017884, SMINT2017964, SMINT2017974, SMINT2018343, SMINT2018353, SMINT2018592, SMINT2019017, SMINT2019105, SMINT2019142,
SMINT2019153, SMINT2019200, SPLEN1000013, SPLEN1000041, SPLEN1000049, SPLEN1000056, SPLEN1000087, SPLEN1000089, SPLEN1000091, SPLEN1000134, SPLEN1000139, SPLEN1000143, SPLEN1000144, SPLEN1000145, SPLEN1000156, SPLEN1000163, SPLEN1000165, SPLEN1000178, SPLEN2000020, SPLEN2000047, SPLEN2000064, SPLEN2000072, SPLEN2000098, SPLEN2000126, SPLEN2000134, SPLEN2000143, SPLEN2000189, SPLEN2000197, SPLEN2000205, SPLEN2000210, SPLEN2000222, SPLEN2000242, SPLEN2000247, SPLEN2000258, SPLEN2000267, SPLEN2000270, SPLEN2000307, SPLEN2000310, SPLEN2000341, SPLEN2000348, SPLEN2000350, SPLEN2000357, SPLEN2000364, SPLEN2000402, SPLEN2000414, SPLEN2000420, SPLEN2000443, SPLEN2000448, SPLEN2000450, SPLEN2000464,
SPLEN2000496, SPLEN2000515, SPLEN2000516, SPLEN2000537, SPLEN2000541, SPLEN2000607, SPLEN2000630, SPLEN2000695, SPLEN2000698, SPLEN2000874, SPLEN2000882, SPLEN2001135, SPLEN2001176, SPLEN2001227, SPLEN2001245, SPLEN2001278, SPLEN2001354, SPLEN2001367, SPLEN2001383, SPLEN2001476, SPLEN2001503, SPLEN2001510, SPLEN2001599, SPLEN2001626, SPLEN2001650, SPLEN2001710, SPLEN2001761, SPLEN2001781, SPLEN2001881, SPLEN2001912, SPLEN2001945, SPLEN2002007, SPLEN2002053, SPLEN2002133, SPLEN2002147, SPLEN2002166, SPLEN2002175, SPLEN2002223, SPLEN2002272, SPLEN2002314, SPLEN2002343, SPLEN2002354, SPLEN2002385, SPLEN2002419, SPLEN2002451, SPLEN2002462, SPLEN2002467, SPLEN2002477, SPLEN2002493, SPLEN2002662,
SPLEN2002695, SPLEN2002707, SPLEN2002744, SPLEN2002917, SPLEN2002931, SPLEN2002957, SPLEN2003160, SPLEN2003204, SPLEN2003219, SPLEN2003396, SPLEN2003678, SPLEN2003878, SPLEN2003918, SPLEN2003924, SPLEN2004060, SPLEN2004078, SPLEN2004124, SPLEN2004148, SPLEN2004181, SPLEN2004343, SPLEN2004346, SPLEN2004368, SPLEN2004555, SPLEN2004611, SPLEN2004662, SPLEN2004773, SPLEN2004844, SPLEN2004880, SPLEN2004984, SPLEN2005227, SPLEN2005272, SPLEN2005342, SPLEN2005416, SPLEN2005450, SPLEN2005474, SPLEN2005560, SPLEN2005727, SPLEN2005783, SPLEN2005790, SPLEN2005806, SPLEN2005818, SPLEN2005838, SPLEN2005869, SPLEN2005939, SPLEN2006268, SPLEN2006283, SPLEN2006325, SPLEN2006389, SPLEN2006425, SPLEN2006671,
SPLEN2006701, SPLEN2006785, SPLEN2006875, SPLEN2007182, SPLEN2007276, SPLEN2007314, SPLEN2007326, SPLEN2007350, SPLEN2007619, SPLEN2007653, SPLEN2007689, SPLEN2007695, SPLEN2007739, SPLEN2007750, SPLEN2007794, SPLEN2007879, SPLEN2007926, SPLEN2007951, SPLEN2008007, SPLEN2008135, SPLEN2008164, SPLEN2008317, SPLEN2008460, SPLEN2008496, SPLEN2008591, SPLEN2008786, SPLEN2009081, SPLEN2009088, SPLEN2009163, SPLEN2009340, SPLEN2009512, SPLEN2009513, SPLEN2009541, SPLEN2009548, SPLEN2009555, SPLEN2009581, SPLEN2009733, SPLEN2009803, SPLEN2009884, SPLEN2009918, SPLEN2009970, SPLEN2010004, SPLEN2010015, SPLEN2010112, SPLEN2010119, SPLEN2010146,. SPLEN2010187, SPLEN2010350, SPLEN2010395, SPLEN2010415,
SPLEN2010447, SPLEN2010469, SPLEN2010510, SPLEN2010530, SPLEN2010534, SPLEN2010588, SPLEN2010625, SPLEN2010800, SPLEN2010846, SPLEN2010847, SPLEN2010862, SPLEN2010912, SPLEN2010978, SPLEN2010986, SPLEN2011018, SPLEN2011021, SPLEN2011025, SPLEN2011086, SPLEN2011139, SPLEN2011145, SPLEN2011248, SPLEN2011252, SPLEN2011422, SPLEN2011672, SPLEN2011678, SPLEN2011758, SPLEN2011766, SPLEN2011805, SPLEN2011820, SPLEN2011830, SPLEN2011931, SPLEN2011981, SPLEN2012115, SPLEN2012175, SPLEN2012179, SPLEN2012185, SPLEN2012234, SPLEN2012453, SPLEN2012523, SPLEN2012524, SPLEN2012525, SPLEN2012606, SPLEN2012611, SPLEN2012619, SPLEN2012624, SPLEN2012725, SPLEN2012743, SPLEN2012756, SPLEN2012846, SPLEN2012889,
SPLEN2012961, SPLEN2013108, SPLEN2013195, SPLEN2013503, SPLEN2013644, SPLEN2013648, SPLEN2013670, SPLEN2013673, SPLEN2013714, SPLEN2013753, SPLEN2013821, SPLEN2013857, SPLEN2013860, SPLEN2013910, SPLEN2013923, SPLEN2013936, SPLEN2014041, SPLEN2014063, SPLEN2014073, SPLEN2014119, SPLEN2014136, SPLEN2014199, SPLEN2014210, SPLEN2014293, SPLEN2014318, SPLEN2014381, SPLEN2014452, SPLEN2014454, SPLEN2014486, SPLEN2014543, SPLEN2014645, SPLEN2014669, SPLEN2014711, SPLEN2014739, SPLEN2014860, SPLEN2014865, SPLEN2014911, SPLEN2014919, SPLEN2014920, SPLEN2014924, SPLEN2014937, SPLEN2014946, SPLEN2015031, SPLEN2015094, SPLEN2015121, SPLEN2015129, SPLEN2015158, SPLEN2015166, SPLEN2015216, SPLEN2015258,
SPLEN2015261, SPLEN2015267, SPLEN2015276, SPLEN2015310, SPLEN2015415, SPLEN2015433, SPLEN2015593, SPLEN2015674, SPLEN2015679, SPLEN2015707, SPLEN2015730, SPLEN2015746, SPLEN2015788, SPLEN2015815, SPLEN2015890, SPLEN2015891, SPLEN2015899, SPLEN2016045, SPLEN2016053, SPLEN2016069, SPLEN2016098, SPLEN2016135, SPLEN2016139, SPLEN2016241, SPLEN2016250, SPLEN2016304, SPLEN2016356, SPLEN2016480, SPLEN2016531, SPLEN2016533, SPLEN2016541, SPLEN2016552, SPLEN2016602, SPLEN2016627, SPLEN2016678, SPLEN2016743, SPLEN2016773, SPLEN2016781, SPLEN2016840, SPLEN2016863, SPLEN2016922, SPLEN2016932, SPLEN2016973, SPLEN2017043, SPLEN2017104, SPLEN2017121, SPLEN2017147, SPLEN2017187, SPLEN2017189, SPLEN2017212,
SPLEN2017351, SPLEN2017426, SPLEN2017452, SPLEN2017538, SPLEN2017592, SPLEN2017613, SPLEN2017620, SPLEN2017643, SPLEN2017687, SPLEN2017740, SPLEN2017781, SPLEN2017999, SPLEN2018112, SPLEN2018159, SPLEN2018180, SPLEN2018181, SPLEN2018280, SPLEN2018285, SPLEN2018299, SPLEN2018302, SPLEN2018364, SPLEN2018395, SPLEN2018643, SPLEN2018683, SPLEN2018732, SPLEN2018749, SPLEN2018933, SPLEN2019008, SPLEN2019043, SPLEN2019077, SPLEN2019092, SPLEN2019102, SPLEN2019131, SPLEN2019257, SPLEN2019323, SPLEN2019349, SPLEN2019375, SPLEN2019379, SPLEN2019446, SPLEN2019471, SPLEN2019480, SPLEN2019505, SPLEN2019571, SPLEN2019575, SPLEN2019709, SPLEN2019774, SPLEN2019793, SPLEN2019811, SPLEN2019839, SPLEN2019956,
SPLEN2019985, SPLEN2020007, SPLEN2020034, SPLEN2020153, SPLEN2020154, SPLEN2020166, SPLEN2020183, SPLEN2020223, SPLEN2020359, SPLEN2020417, SPLEN2020427, SPLEN2020467, SPLEN2020512, SPLEN2020608, SPLEN2020777, SPLEN2020805, SPLEN2021057, SPLEN2021122, SPLEN2021157, SPLEN2021231, SPLEN2021295, SPLEN2021298, SPLEN2021383, SPLEN2021417, SPLEN2021440, SPLEN2021458, SPLEN2021463, SPLEN2021560, SPLEN2021701, SPLEN2021795, SPLEN2021983, SPLEN2021991, SPLEN2022040, SPLEN2022162, SPLEN2022227, SPLEN2022591, SPLEN2022785, SPLEN2022920, SPLEN2023024, SPLEN2023118, SPLEN2023160, SPLEN2023423, SPLEN2023710, SPLEN2023733, SPLEN2023791, SPLEN2023977, SPLEN2024070, SPLEN2024127, SPLEN2024232, SPLEN2024273,
SPLEN2024383, SPLEN2024489, SPLEN2024530, SPLEN2024571, SPLEN2024577, SPLEN2024617, SPLEN2024737, SPLEN2024922, SPLEN2024956, SPLEN2024990, SPLEN2025012, SPLEN2025017, SPLEN2025039, SPLEN2025219, SPLEN2025491, SPLEN2025626, SPLEN2025820, SPLEN2026144, SPLEN2026411, SPLEN2026765, SPLEN2027157, SPLEN2027395, SPLEN2027488, SPLEN2027780, SPLEN2027852, SPLEN2027868, SPLEN2027995, SPLEN2028046, SPLEN2028066, SPLEN2028138, SPLEN2028365, SPLEN2028417, SPLEN2028424, SPLEN2028593, SPLEN2028844, SPLEN2028914, SPLEN2028994, SPLEN2029051, SPLEN2029176, SPLEN2029295, SPLEN2029380, SPLEN2029522, SPLEN2029683, SPLEN2029727, SPLEN2029904, SPLEN2029912, SPLEN2030250, SPLEN2030335, SPLEN2030371, SPLEN2030397,
SPLEN2030479, SPLEN2030495, SPLEN2030562, SPLEN2030763, SPLEN2030847, SPLEN2031004, SPLEN2031125, SPLEN2031424, SPLEN2031674, SPLEN2031724, SPLEN2031780, SPLEN2032036, SPLEN2032112, SPLEN2032154, SPLEN2032157, SPLEN2032321, SPLEN2032356, SPLEN2032677, SPLEN2032813, SPLEN2032924, SPLEN2033098, SPLEN2033153, SPLEN2033333, SPLEN2033490, SPLEN2033539, SPLEN2033921, SPLEN2033996, SPLEN2034021, SPLEN2034081, SPLEN2034252, SPLEN2034448, SPLEN2034551, SPLEN2034601, SPLEN2034678, SPLEN2034781, SPLEN2034864, SPLEN2034934, SPLEN2035318, SPLEN2035615, SPLEN2036076, SPLEN2036103, SPLEN2036326, SPLEN2036475, SPLEN2036501, SPLEN2036579, SPLEN2036608, SPLEN2036702, SPLEN2036712, SPLEN2036821, SPLEN2036953,
SPLEN2037077, SPLEN2037194, SPLEN2037319, SPLEN2037444, SPLEN2037580, SPLEN2037630, SPLEN2037678, SPLEN2037722, SPLEN2037891, SPLEN2038055, SPLEN2038180, SPLEN2038345, SPLEN2038407, SPLEN2038473, SPLEN2038834, SPLEN2039311, SPLEN2039672, SPLEN2039697, SPLEN2039845, SPLEN2039936, SPLEN2040222, SPLEN2040237, SPLEN2040824, SPLEN2041304, SPLEN2041306, SPLEN2041310, SPLEN2041645, SPLEN2041700, SPLEN2041720, SPLEN2041977, SPLEN2042051, SPLEN2042303, SPLEN2042521, SPLEN2042535, SPLEN2042598, SPLEN2042920, STOMA1000013, STOMA1000047, STOMA1000052, STOMA1000074, STOMA1000091, STOMA1000117, STOMA1000133, STOMA1000175, STOMA1000190, STOMA1000191, STOMA2000032, STOMA2000072, STOMA2000088, STOMA2000104,
STOMA2000121, STOMA2000148, STOMA2000168, STOMA2000183, STOMA2000188, STOMA2000257, STOMA2000279, STOMA2000383, STOMA2000386, STOMA2000478, STOMA2000482, STOMA2000529, STOMA2000539, STOMA2000567, STOMA2000640, STOMA2000678, STOMA2000686, STOMA2000888, STOMA2001025, STOMA2001389, STOMA2002141, STOMA2002166, STOMA2002367, STOMA2002688, STOMA2002828, STOMA2003158, STOMA2003289, STOMA2003444, STOMA2003477, STOMA2003646, STOMA2003716, STOMA2003781, STOMA2003894, STOMA2004194, STOMA2004294, STOMA2004663, STOMA2004668, STOMA2004852, STOMA2004873, STOMA2004884, STOMA2004893, STOMA2005120, STOMA2005664, STOMA2005667, STOMA2005746, STOMA2005782, STOMA2006181, STOMA2006213, STOMA2006229, STOMA2006325,
STOMA2006330, STOMA2006398, STOMA2006447, STOMA2006780, STOMA2006904, STOMA2007269,. STOMA2007680, STOMA2007745, STOMA2008050, STOMA2008361, STOMA2008546, STOMA2008614, STOMA2008638, STOMA2008838, STOMA2009250, STOMA2009253, STOMA2009256, STOMA2009289, SYNOV1000045, SYNOV1000049, SYNOV1000118, SYNOV1000128, SYNOV1000164, SYNOV1000190, SYNOV1000256, SYNOV1000374, SYNOV2000152, SYNOV2000171, SYNOV2000173, SYNOV2000177, SYNOV2000178, SYNOV2000193, SYNOV2000251, SYNOV2000279, SYNOV2000291, SYNOV2000297, SYNOV2000397, SYNOV2000426, SYNOV2000601, SYNOV2000700, SYNOV2000820, SYNOV2000824, SYNOV2000923, SYNOV2001014, SYNOV2001035, SYNOV2001088, SYNOV2001111, SYNOV2001144, SYNOV2001212, SYNOV2001251,
SYNOV2001300, SYNOV2001356, SYNOV2001374, SYNOV2001390, SYNOV2001451, SYNOV2001457, SYNOV2001468, SYNOV2001581, SYNOV2001648, SYNOV2001660, SYNOV2003326, SYNOV2005216, SYNOV2005448, SYNOV2005541, SYNOV2005673, SYNOV2005817, SYNOV2005962, SYNOV2006430, SYNOV2006620, SYNOV2007758, SYNOV2007965, SYNOV2008765, SYNOV2009172, SYNOV2011233, SYNOV2012326, SYNOV2013365, SYNOV2013637, SYNOV2014157, SYNOV2014364, SYNOV2016124, SYNOV2016837, SYNOV2017179, SYNOV2018921, SYNOV2019280, SYNOV2020085, SYNOV2020463, SYNOV2021320, SYNOV2021953, SYNOV3000184, SYNOV3000231, SYNOV3000302, SYNOV3000345, SYNOV4000249, SYNOV4000472, SYNOV4000706, SYNOV4001153, SYNOV4001326, SYNOV4001342, SYNOV4002001, SYNOV4002392,
SYNOV4002875, SYNOV4002883, SYNOV4003174, SYNOV4003322,. SYNOV4003681, SYNOV4003981, SYNOV4004184, SYNOV4004741, SYNOV4004823, SYNOV4004914, SYNOV4005256, SYNOV4005570, SYNOV4005739, SYNOV4005889, SYNOV4005981, SYNOV4005989, SYNOV4006256, SYNOV4006327, SYNOV4006542, SYNOV4007012, SYNOV4007215, SYNOV4007360, SYNOV4007430, SYNOV4007521, SYNOV4007553, SYNOV4007671, SYNOV4007711, SYNOV4008245, SYNOV4008336, SYNOV4009129, SYNOV4009139, SYNOV4009298, T1ESE2000116, T1ESE2000609, T1ESE2002665, TBAES2000004, TBAES2000116, TBAES2000255, TBAES2000315, TBAES2000352, TBAES2000932, TBAES2001171, TBAES2001220, TBAES2001229, TBAES2001258, TBAES2001296, TBAES2001492, TBAES2001553, TBAES2001751, TBAES2003435,
TBAES2003492, TBAES2003550, TBAES2003917, TBAES2004055, TBAES2004105, TBAES2004939, TBAES2005157, TBAES2005361, TBAES2005543, TBAES2006568, TBAES2006881, TBAES2007379, TBAES2007428, TBAES2007481, TBAES2007504, TBAES2007548, TBAES2007697, TBAES2007862, TBAES2007964, TBAES2008133, TCERX2000171, TCERX2000431, TCERX2000998, TCERX2002644, TCOLN1000037, TCOLN1000180, TCOLN2000139, TCOLN2000236, TCOLN2000641, TCOLN2001268, TCOLN2002047, TCOLN2002278, TESOP1000035, TESOP1000127, TESOP1000160, TESOP2000083, TESOP2000090, TESOP2000205, TESOP2000312, TESOP2000390, TESOP2000400, TESOP2000510, TESOP2000527, TESOP2000569, TESOP2000739, TESOP2000801, TESOP2001122, TESOP2001166, TESOP2001345, TESOP2001474,
TESOP2001796, TESOP2001818, TESOP2001849, TESOP2001865, TESOP2001901, TESOP2001953, TESOP2002005, TESOP2002110, TESOP2002150, TESOP2002451, TESOP2002489, TESOP2002539, TESOP2002545, TESOP2002804, TESOP2002950, TESOP2003273, TESOP2003308, TESOP2003553, TESOP2003753, TESOP2004110; TESOP2004114, TESOP2004173, TESOP2004214, TESOP2005199, TESOP2005285, TESOP2005485, TESOP2005498, TESOP2005579, TESOP2006041, TESOP2006053, TESOP2006060, TESOP2006068, TESOP2006110, TESOP2006398, TESOP2006670, TESOP2006704, TESOP2006746, TESOP2006865, TESOP2007041, TESOP2007052, TESOP2007262, TESOP2007384, TESOP2007636, TESOP2007674, TESOP2007688, TESOP2007725, TESOP2007912, TESOP2007978, TESOP2008127, TESOP2008552,
TESOP2008556, TESOP2008563, TESOP2008820, TESOP2009121, TESOP2009201, TESOP2009247, TESOP2009555, TESTI1000018, TESTI1000019, TESTI1000025, TESTI1000029, TESTI1000030, TESTI1000042, TESTI1000045, TESTI1000051, TESTI1000055, TESTI1000064, TESTI1000070, TESTI1000085, TESTI1000088, TESTI1000093, TESTI1000094, TESTI1000096, TESTI1000101, TESTI1000109, TESTI1000125, TESTI1000127, TESTI1000128, TESTI1000131, TESTI1000138, TESTI1000142, TESTI1000157, TESTI1000163, TESTI1000168, TESTI1000169, TESTI1000179, TESTI1000183, TESTI1000191, TESTI1000257, TESTI1000266, TESTI1000319, TESTI1000330, TESTI1000348, TESTI1000390, TESTI1000391, TESTI1000459, TESTI1000491, TESTI1000545, TESTI2000006, TESTI2000018,
TESTI2000044, TESTI2000100, TESTI2000117, TESTI2000120, TESTI2000147, TESTI2000154, TESTI2000172, TESTI2000177, TESTI2000179, TESTI2000207, TESTI2000208, TESTI2000221, TESTI2000238, TESTI2000253, TESTI2000272, TESTI2000278, TESTI2000349, TESTI2000358, TESTI2000372, TESTI2000427, TESTI2000431, TESTI2000435, TESTI2000443, TESTI2000452, TESTI2000462, TESTI2000489, TESTI2000520, TESTI2000571, TESTI2000591, TESTI2000598, TESTI2000600, TESTI2000616, TESTI2000627, TESTI2000644, TESTI2000671, TESTI2000683, TESTI2000689, TESTI2000695, TESTI2000699, TESTI2000707, TESTI2000745, TESTI2000756, TESTI2000762, TESTI2000808, TESTI2000819, TESTI2000830, TESTI2000840, TESTI2000849, TESTI2000850, TESTI2000874,
TESTI2000883, TESTI2000909, TESTI2000951, TESTI2000981, TESTI2001008, TESTI2001049, TESTI2001082, TESTI2001120, TESTI2001134, TESTI2001141, TESTI2001180, TESTI2001208, TESTI2001229, TESTI2001236, TESTI2001237, TESTI2001269, TESTI2001306, TESTI2001313, TESTI2001330, TESTI2001339, TESTI2001345, TESTI2001352, TESTI2001364, TESTI2001412, TESTI2001498, TESTI2001511, TESTI2001512, TESTI2001518, TESTI2001556, TESTI2001593, TESTI2001613, TESTI2001621, TESTI2001661, TESTI2001665, TESTI2001671, TESTI2001697, TESTI2001725, TESTI2001758, TESTI2001766, TESTI2001792, TESTI2001793, TESTI2001795, TESTI2001812, TESTI2001815, TESTI2001823, TESTI2001826, TESTI2001827, TESTI2001852, TESTI2001869, TESTI2001879,
TESTI2001894, TESTI2001898, TESTI2001915, TESTI2001950, TESTI2001959, TESTI2001968, TESTI2001985, TESTI2001991, TESTI2002002, TESTI2002012, TESTI2002036, TESTI2002048, TESTI2002057, TESTI2002081, TESTI2002090, TESTI2002105, TESTI2002216, TESTI2002223, TESTI2002245, TESTI2002251, TESTI2002256, TESTI2002264, TESTI2002282, TESTI2002293, TESTI2002294, TESTI2002326, TESTI2002351, TESTI2002361, TESTI2002369, TESTI2002406, TESTI2002415, TESTI2002423, TESTI2002453, TESTI2002461, TESTI2002465, TESTI2002467, TESTI2002498, TESTI2002516, TESTI2002544, TESTI2002578, TESTI2002580, TESTI2002618, TESTI2002676, TESTI2002690, TESTI2002696, TESTI2002698, TESTI2002700, TESTI2002707, TESTI2002724, TESTI2002729,
TESTI2002752, TESTI2002753, TESTI2002789, TESTI2002802, TESTI2002806, TESTI2002840, TESTI2002866, TESTI2002910, TESTI2002912, TESTI2002928, TESTI2002937, TESTI2002965, TESTI2002993, TESTI2003020, TESTI2003031, TESTI2003037, TESTI2003044, TESTI2003059, TESTI2003061, TESTI2003071, TESTI2003074, TESTI2003089, TESTI2003104, TESTI2003109, TESTI2003117, TESTI2003127, TESTI2003130, TESTI2003131, TESTI2003139, TESTI2003141, TESTI2003152, TESTI2003181, TESTI2003193, TESTI2003196, TESTI2003228, TESTI2003236, TESTI2003255, TESTI2003258, TESTI2003277, TESTI2003280, TESTI2003299, TESTI2003311, TESTI2003321, TESTI2003325, TESTI2003327, TESTI2003330, TESTI2003347, TESTI2003354, TESTI2003356, TESTI2003372,
TESTI2003376, TESTI2003413, TESTI2003418, TESTI2003419, TESTI2003432, TESTI2003454, TESTI2003475, TESTI2003476, TESTI2003498, TESTI2003512, TESTI2003539, TESTI2003541, TESTI2003545, TESTI2003551, TESTI2003574, TESTI2003579, TESTI2003580, TESTI2003589, TESTI2003596, TESTI2003598, TESTI2003615, TESTI2003625, TESTI2003638, TESTI2003649, TESTI2003692, TESTI2003718, TESTI2003727, TESTI2003756, TESTI2003768, TESTI2003781, TESTI2003787, TESTI2003824, TESTI2003827, TESTI2003894, TESTI2003911, TESTI2003914, TESTI2003940, TESTI2003962, TESTI2003998, TESTI2004000, TESTI2004073, TESTI2004080, TESTI2004085, TESTI2004089, TESTI2004111, TESTI2004119, TESTI2004122, TESTI2004153, TESTI2004163, TESTI2004169,
TESTI2004207, TESTI2004215, TESTI2004227, TESTI2004229, TESTI2004243, TESTI2004255, TESTI2004287, TESTI2004313, TESTI2004318, TESTI2004322, TESTI2004391, TESTI2004399, TESTI2004423, TESTI2004431, TESTI2004432, TESTI2004452, TESTI2004459, TESTI2004490, TESTI2004539, TESTI2004552, TESTI2004560, TESTI2004574, TESTI2004601, TESTI2004611, TESTI2004649, TESTI2004654, TESTI2004675, TESTI2004687, TESTI2004689, TESTI2004700, TESTI2004706, TESTI2004712, TESTI2004734, TESTI2004783, TESTI2004791, TESTI2004793, TESTI2004860, TESTI2004906, TESTI2004920, TESTI2004936, TESTI2004941, TESTI2004971, TESTI2004982, TESTI2004993, TESTI2004994, TESTI2004999, TESTI2005017, TESTI2005025, TESTI2005040, TESTI2005060,
TESTI2005072, TESTI2005085, TESTI2005112, TESTI2005120, TESTI2005153, TESTI2005155, TESTI2005173, TESTI2005267, TESTI2005268, TESTI2005280, TESTI2005307, TESTI2005326, TESTI2005363, TESTI2005376, TESTI2005378, TESTI2005380, TESTI2005391, TESTI2005395, TESTI2005396, TESTI2005408, TESTI2005415, TESTI2005470, TESTI2005492, TESTI2005564, TESTI2005568, TESTI2005584, TESTI2005588, TESTI2005590, TESTI2005603, TESTI2005609, TESTI2005610, TESTI2005630, TESTI2005672, TESTI2005683, TESTI2005690, TESTI2005692, TESTI2005703, TESTI2005720, TESTI2005739, TESTI2005742, TESTI2005743, TESTI2005767, TESTI2005775, TESTI2005784, TESTI2005788, TESTI2005827, TESTI2005835, TESTI2005860, TESTI2005892, TESTI2005911,
TESTI2005933, TESTI2005937, TESTI2005948, TESTI2005979, TESTI2005981, TESTI2005982, TESTI2005984, TESTI2005986, TESTI2005987, TESTI2006007, TESTI2006008, TESTI2006015, TESTI2006016, TESTI2006035, TESTI2006040, TESTI2006041, TESTI2006045, TESTI2006067, TESTI2006083, TESTI2006097, TESTI2006109, TESTI2006120, TESTI2006125, TESTI2006179, TESTI2006209, TESTI2006210, TESTI2006212, TESTI2006218, TESTI2006238, TESTI2006255, TESTI2006258, TESTI2006278, TESTI2006333, TESTI2006341, TESTI2006360, TESTI2006383, TESTI2006409, TESTI2006425, TESTI2006437, TESTI2006453, TESTI2006457, TESTI2006460, TESTI2006465, TESTI2006483,-TESTI2006499, TESTI2006523, TESTI2006543, TESTI2006565, . TESTI2006572, TESTI2006615,
TESTI2006617, TESTI2006624, TESTI2006628, TESTI2006633, TESTI2006643, TESTI2006648, TESTI2006659, TESTI2006660, TESTI2006665, TESTI2006667, TESTI2006677, TESTI2006720, TESTI2006735, TESTI2006744, TESTI2006748, TESTI2006774, TESTI2006853, TESTI2006866, TESTI2006872, TESTI2006877, TESTI2006879, TESTI2006894, TESTI2006940, TESTI2006955, TESTI2006978, TESTI2006979, TESTI2006990, TESTI2007040, TESTI2007074, TESTI2007078, TESTI2007113, TESTI2007152, TESTI2007163, TESTI2007183, TESTI2007220, TESTI2007234, TESTI2007311, TESTI2007320, TESTI2007346, TESTI2007358, TESTI2007402, TESTI2007405, TESTI2007422, TESTI2007452, TESTI2007464, TESTI2007466, TESTI2007480, TESTI2007490, TESTI2007502, TESTI2007524,
TESTI2007533, TESTI2007576, TESTI2007613, TESTI2007657, TESTI2007685, TESTI2007692, TESTI2007749, TESTI2007750, TESTI2007808, TESTI2007814, TESTI2007829, TESTI2007864, TESTI2007867, TESTI2007872, TESTI2007906, TESTI2007922, TESTI2007951, TESTI2007972, TESTI2007973, TESTI2007998, TESTI2008014, TESTI2008020, TESTI2008032, TESTI2008033, TESTI2008046, TESTI2008081, TESTI2008099, TESTI2008139, TESTI2008144, TESTI2008161, TESTI2008189, TESTI2008204, TESTI2008219, TESTI2008233, TESTI2008234, TESTI2008237, TESTI2008278, TESTI2008320, TESTI2008331, TESTI2008343, TESTI2008387, TESTI2008389, TESTI2008394, TESTI2008405, TESTI2008420, TESTI2008425, TESTI2008440, TESTI2008567, TESTI2008595, TESTI2008621,
TESTI2008636, TESTI2008657, TESTI2008684, TESTI2008699, TESTI2008715, TESTI2008762, TESTI2008774, TESTI2008786, TESTI2008822, TESTI2008840, TESTI2008847, TESTI2008884, TESTI2008901, TESTI2008929, TESTI2008946, TESTI2008960, TESTI2009018, TESTI2009091, TESTI2009097, TESTI2009136, TESTI2009185, TESTI2009190, TESTI2009217, TESTI2009239, TESTI2009325, TESTI2009388, TESTI2009390, TESTI2009400, TESTI2009402, TESTI2009412, TESTI2009423, TESTI2009447, TESTI2009462, TESTI2009474, TESTI2009477, TESTI2009497, TESTI2009502, TESTI2009511, TESTI2009544, TESTI2009545, TESTI2009551, TESTI2009585, TESTI2009588, TESTI2009683, TESTI2009727, TESTI2009739, TESTI2009812, TESTI2009835, TESTI2009853, TESTI2009935,
TESTI2009977, TESTI2009987, TESTI2010009, TESTI2010154, TESTI2010180, TESTI2010239, TESTI2010280, TESTI2010354, TESTI2010396, TESTI2010400, TESTI2010409, TESTI2010572, TESTI2010587, TESTI2010591, TESTI2010617, TESTI2010682, TESTI2010724, TESTI2010732, TESTI2010734, TESTI2010755, TESTI2010793, TESTI2010806, TESTI2010833, TESTI2010872, TESTI2011009, TESTI2011020, TESTI2011028, TESTI2011033, TESTI2011254, TESTI2011286, TESTI2011294, TESTI2011314, TESTI2011315, TESTI2011394, TESTI2011407, TESTI2011448, TESTI2011480, TESTI2011549, TESTI2011575, TESTI2011605, TESTI2011612, TESTI2011665, TESTI2011680, TESTI2011712, TESTI2011750, TESTI2011840, TESTI2011846, TESTI2011915, TESTI2011928, TESTI2011971,
TESTI2012019, TESTI2012050, TESTI2012090, TESTI2012095, TESTI2012104, TESTI2012152, TESTI2012155, TESTI2012171, TESTI2012207, TESTI2012231, TESTI2012244, TESTI2012289, TESTI2012308, TESTI2012356, TESTI2012387, TESTI2012394, TESTI2012444, TESTI2012511, TESTI2012528, TESTI2012544, TESTI2012550, TESTI2012571, TESTI2012592, TESTI2012617, TESTI2012628, TESTI2012778, TESTI2012812, TESTI2012834, TESTI2012835, TESTI2012861, TESTI2012900, TESTI2012915, TESTI2012922, TESTI2013001, TESTI2013012, TESTI2013053, TESTI2013144, TESTI2013163, TESTI2013169, TESTI2013212, TESTI2013231, TESTI2013257, TESTI2013264, TESTI2013268, TESTI2013295, TESTI2013322, TESTI2013381, TESTI2013382, TESTI2013401, TESTI2013427,
TESTI2013468, TESTI2013497, TESTI2013545, TESTI2013546, TESTI2013566, TESTI2013601, TESTI2013604, TESTI2013607, TESTI2013610, TESTI2013691, TESTI2013699, TESTI2013737, TESTI2013767, TESTI2013832, TESTI2013873, TESTI2013889, TESTI2014007, TESTI2014036, TESTI2014097, TESTI2014160, TESTI2014248, TESTI2014254, TESTI2014269, TESTI2014274, TESTI2014318, TESTI2014324, TESTI2014339, TESTI2014362, TESTI2014385, TESTI2014415, TESTI2014439, TESTI2014474, TESTI2014505, TESTI2014577, TESTI2014578, TESTI2014716, TESTI2014780, TESTI2014800, TESTI2014843, TESTI2014979, TESTI2014988, TESTI2015042, TESTI2015092, TESTI2015105, TESTI2015180, TESTI2015246, TESTI2015249, TESTI2015327, TESTI2015331, TESTI2015335,
TESTI2015350, TESTI2015375, TESTI2015437, TESTI2015590, TESTI2015610, TESTI2015647, TESTI2015675, TESTI2015710, TESTI2015752, TESTI2015830, TESTI2015914, TESTI2015938, TESTI2015947, TESTI2015962, TESTI2015987, TESTI2016033, TESTI2016046, TESTI2016101, TESTI2016162, TESTI2016197, TESTI2016278, TESTI2016298, TESTI2016331, TESTI2016337, TESTI2016410, TESTI2016421, TESTI2016509, TESTI2016568, TESTI2016583, TESTI2016599, TESTI2016629, TESTI2016663, TESTI2016667, TESTI2016688, TESTI2016701, TESTI2016758, TESTI2016848, TESTI2016863, TESTI2016888, TESTI2016896, TESTI2016922, TESTI2016942, TESTI2016950, TESTI2016996, TESTI2017017, TESTI2017028, TESTI2017035, TESTI2017069, TESTI2017089, TESTI2017102,
TESTI2017107, TESTI2017113, TESTI2017125, TESTI2017256, TESTI2017305, TESTI2017311, TESTI2017396, TESTI2017507, TESTI2017537, TESTI2017582, TESTI2017584, TESTI2017645, TESTI2017727, TESTI2017740, TESTI2017775, TESTI2017816, TESTI2017832, TESTI2017923, TESTI2017932, TESTI2017951, TESTI2018014, TESTI2018031, TESTI2018035, TESTI2018060, TESTI2018083, TESTI2018178, TESTI2018221, TESTI2018276, TESTI2018290, TESTI2018335, TESTI2018337, TESTI2018368, TESTI2018421, TESTI2018428, TESTI2018475, TESTI2018482, TESTI2018521, TESTI2018565, TESTI2018581, TESTI2018611, TESTI2018687, TESTI2018697, TESTI2018838, TESTI2018867, TESTI2018941, TESTI2018957, TESTI2018974, TESTI2018975, TESTI2018997, TESTI2019042,
TESTI2019077, TESTI2019228, TESTI2019257, TESTI2019280, TESTI2019282, TESTI2019308, TESTI2019336, TESTI2019352, TESTI2019406, TESTI2019430, TESTI2019455, TESTI2019481, TESTI2019488, TESTI2019501, TESTI2019648, TESTI2019669, TESTI2019697, TESTI2019729, TESTI2019760, TESTI2019787, TESTI2019794, TESTI2019854, TESTI2019859, TESTI2019860, TESTI2019872, TESTI2019911, TESTI2019917, TESTI2019975, TESTI2019998, TESTI2020012, TESTI2020026, TESTI2020029, TESTI2020071, TESTI2020084, TESTI2020265, TESTI2020283, TESTI2020316, TESTI2020344, TESTI2020379, TESTI2020426, TESTI2020445, TESTI2020510, TESTI2020515, TESTI2020525, TESTI2020579, TESTI2020717, TESTI2020840, TESTI2020905, TESTI2020906, TESTI2020946,
TESTI2020956, TESTI2020981, TESTI2020999, TESTI2021003, TESTI2021038, TESTI2021057, TESTI2021077, TESTI2021112, TESTI2021114, TESTI2021116, TESTI2021118, TESTI2021122, TESTI2021138, TESTI2021297, TESTI2021315, TESTI2021447, TESTI2021463, TESTI2021531, TESTI2021599, TESTI2021637, TESTI2021654, TESTI2021667, TESTI2021736, TESTI2021785, TESTI2021819, TESTI2021851, TESTI2021895, TESTI2021911, TESTI2021939, TESTI2021999, TESTI2022010, TESTI2022023, TESTI2022065, TESTI2022086, TESTI2022179,.TESTI2022203, TESTI2022246, TESTI2022323, TESTI2022338, TESTI2022400, TESTI2022462, TESTI2022495, TESTI2022623, TESTI2022649, TESTI2022652, TESTI2022738, TESTI2022798, TESTI2022812, TESTI2022814, TESTI2022874,
TESTI2022940, TESTI2022952, TESTI2023025, TESTI2023033, TESTI2023053, TESTI2023085, TESTI2023192, TESTI2023194, TESTI2023214, TESTI2023254, TESTI2023386, TESTI2023414, TESTI2023427, TESTI2023436, TESTI2023599, TESTI2023752, TESTI2023800, TESTI2023861, TESTI2023903, TESTI2023942, TESTI2023947, TESTI2023951, TESTI2023968, TESTI2024009, TESTI2024090, TESTI2024125, TESTI2024153, TESTI2024192, TESTI2024267, TESTI2024283, TESTI2024299, TESTI2024419, TESTI2024422, TESTI2024443, TESTI2024446, TESTI2024473, TESTI2024476, TESTI2024498, TESTI2024560, TESTI2024567, TESTI2024586, TESTI2024598, TESTI2024641, TESTI2024648, TESTI2024718, TESTI2024744, TESTI2024844, TESTI2024864, TESTI2024885, TESTI2024936,
TESTI2024978, TESTI2024999, TESTI2025006, TESTI2025013, TESTI2025022, TESTI2025063, TESTI2025117, TESTI2025144, TESTI2025161, TESTI2025174, TESTI2025229, TESTI2025269, TESTI2025365, TESTI2025403, TESTI2025409, TESTI2025422, TESTI2025448, TESTI2025454, TESTI2025486, TESTI2025546, TESTI2025582, TESTI2025609, TESTI2025656, TESTI2025757, TESTI2025791, TESTI2025846, TESTI2025872, TESTI2025879, TESTI2025911, TESTI2025920, TESTI2025924, TESTI2026014, TESTI2026024, TESTI2026064, TESTI2026077, TESTI2026104, TESTI2026116, TESTI2026134, TESTI2026168, TESTI2026196, TESTI2026215, TESTI2026218, TESTI2026233, TESTI2026291, TESTI2026294, TESTI2026401, TESTI2026403, TESTI2026453, TESTI2026461, TESTI2026491,
TESTI2026505, TESTI2026515, TESTI2026525, TESTI2026534, TESTI2026537, TESTI2026589, TESTI2026597, TESTI2026605, TESTI2026647, TESTI2026674, TESTI2026794, TESTI2026824, TESTI2026854, TESTI2026925, TESTI2026936, TESTI2026957, TESTI2027013, TESTI2027019, TESTI2027108, TESTI2027171, TESTI2027173, TESTI2027179, TESTI2027185, TESTI2027206, TESTI2027238, TESTI2027239, TESTI2027243, TESTI2027271, TESTI2027296, TESTI2027378, TESTI2027411, TESTI2027450, TESTI2027496, TESTI2027503, TESTI2027562, TESTI2027615, TESTI2027645, TESTI2027730, TESTI2027736, TESTI2027791, TESTI2027820, TESTI2027828, TESTI2027840, TESTI2027909, TESTI2028098, TESTI2028151, TESTI2028242, TESTI2028253, TESTI2028254, TESTI2028269,
TESTI2028290, TESTI2028296, TESTI2028384, TESTI2028426, TESTI2028488, TESTI2028523, TESTI2028583, TESTI2028613, TESTI2028659, TESTI2028662, TESTI2028670, TESTI2028705, TESTI2028776, TESTI2028811, TESTI2028891, TESTI2028985, TESTI2028997, TESTI2029131, TESTI2029162, TESTI2029196, TESTI2029201, TESTI2029227, TESTI2029244, TESTI2029249, TESTI2029252, TESTI2029264, TESTI2029470, TESTI2029672, TESTI2029785, TESTI2029880, TESTI2030136, TESTI2030322, TESTI2030336, TESTI2030342, TESTI2030519, TESTI2030554, TESTI2030556, TESTI2030728, TESTI2030754, TESTI2030770, TESTI2030860, TESTI2030901, TESTI2030917, TESTI2030930, TESTI2031006, TESTI2031007, TESTI2031060, TESTI2031129, TESTI2031356, TESTI2031418,
TESTI2031504, TESTI2031529, TESTI2031687, TESTI2031760, TESTI2031809, TESTI2031919, TESTI2031935, TESTI2031986, TESTI2032044, TESTI2032067, TESTI2032079, TESTI2032643, TESTI2032681, TESTI2032761, TESTI2032768, TESTI2032774, TESTI2032828, TESTI2033031, TESTI2033242, TESTI2033254, TESTI2033300, TESTI2033395, TESTI2033453, TESTI2033505, TESTI2033520, TESTI2033641, TESTI2033710, TESTI2033905, TESTI2034187, TESTI2034243, TESTI2034249, TESTI2034307, TESTI2034357, TESTI2034401, TESTI2034407, TESTI2034506, TESTI2034520, TESTI2034718, TESTI2034730, TESTI2034749, TESTI2034767, TESTI2034774, TESTI2034777, TESTI2034785, TESTI2034799, TESTI2034847, TESTI2034913, TESTI2034916, TESTI2034931, TESTI2034953,
TESTI2034997, TESTI2035003, TESTI2035078, TESTI2035084, TESTI2035107, TESTI2035121, TESTI2035124, TESTI2035183, TESTI2035236, TESTI2035262, TESTI2035309, TESTI2035688, TESTI2035775, TESTI2035793, TESTI2035898, TESTI2035962, TESTI2035981, TESTI2035997, TESTI2036114, TESTI2036129, TESTI2036285, TESTI2036288, TESTI2036513, TESTI2036589, TESTI2036650, TESTI2036684, TESTI2036691, TESTI2036736, TESTI2036822, TESTI2036833, TESTI2036922, TESTI2036951, TESTI2036965, TESTI2037002, TESTI2037055, TESTI2037081, TESTI2037085, TESTI2037103, TESTI2037106, TESTI2037111, TESTI2037209, TESTI2037255, TESTI2037359, TESTI2037375, TESTI2037382, TESTI2037534, TESTI2037569, TESTI2037572, TESTI2037643, TESTI2037657,
TESTI2037673, TESTI2037723, TESTI2037819, TESTI2037830, TESTI2037845, TESTI2037877, TESTI2037976, TESTI2038048, TESTI2038065, TESTI2038104, TESTI2038275, TESTI2038388, TESTI2038573, TESTI2038596, TESTI2038623, TESTI2038644, TESTI2038733, TESTI2038858, TESTI2038958, TESTI2039025, TESTI2039026, TESTI2039038, TESTI2039041, TESTI2039060, TESTI2039113, TESTI2039121, TESTI2039209, TESTI2039225, TESTI2039227, TESTI2039276, TESTI2039342, TESTI2039353, TESTI2039544, TESTI2039613, TESTI2039707, TESTI2039732, TESTI2039738, TESTI2039776, TESTI2039867, TESTI2039908, TESTI2040018, TESTI2040094, TESTI2040102, TESTI2040128, TESTI2040143, TESTI2040297, TESTI2040372, TESTI2040377, TESTI2040424, TESTI2040642,
TESTI2040815, TESTI2040897, TESTI2040944, TESTI2040964, TESTI2040983, TESTI2040989, TESTI2041330, TESTI2041362, TESTI2041517, TESTI2041564, TESTI2041664, TESTI2041730, TESTI2041734, TESTI2041778, TESTI2041800, TESTI2041947, TESTI2041956, TESTI2041976, TESTI2042062, TESTI2042149, TESTI2042264, TESTI2042295, TESTI2042302, TESTI2042326, TESTI2042400, TESTI2042450, TESTI2042473, TESTI2042507, TESTI2042508, TESTI2042750, TESTI2042783, TESTI2042806, TESTI2042928, TESTI2042946, TESTI2042958, TESTI2043275, TESTI2043277, TESTI2043282, TESTI2043313, TESTI2043477, TESTI2043585, TESTI2043758, TESTI2043817, TESTI2043857, TESTI2043866, TESTI2043869, TESTI2044064, TESTI2044118, TESTI2044194, TESTI2044276,
TESTI2044306, TESTI2044309, TESTI2044413, TESTI2044418, TESTI2044754, TESTI2044796, TESTI2044833, TESTI2044892, TESTI2044920, TESTI2044968, TESTI2045139, TESTI2045155, TESTI2045171, TESTI2045199, TESTI2045353, TESTI2045509, TESTI2045562, TESTI2045611, TESTI2045819, TESTI2045850, TESTI2045920, TESTI2045964, TESTI2045983, TESTI2046100, TESTI2046188, TESTI2046347, TESTI2046352, TESTI2046358, TESTI2046439, TESTI2046535, TESTI2046536, TESTI2046552, TESTI2046569, TESTI2046686, TESTI2046721, TESTI2046732, TESTI2046786, TESTI2046797, TESTI2046863, TESTI2046872, TESTI2047071, TESTI2047141, TESTI2047147, TESTI2047153, TESTI2047212, TESTI2047342, TESTI2047383, TESTI2047792, TESTI2047801, TESTI2047818,
TESTI2047824, TESTI2047885, TESTI2047930, TESTI2048105, TESTI2048109, TESTI2048465, TESTI2048603, TESTI2048898, TESTI2049041, TESTI2049062, TESTI2049206, TESTI2049246, TESTI2049277, TESTI2049422, TESTI2049452, TESTI2049469, TESTI2049553, TESTI2049576, TESTI2049857, TESTI2049956, TESTI2050137, TESTI2050681, TESTI2050780, TESTI2050987, TESTI2051148, TESTI2051177, TESTI2051279, TESTI2051488, TESTI2051543, TESTI2051627, TESTI2051742, TESTI2051767, TESTI2051791, TESTI2051806, TESTI2051867, TESTI2051873, TESTI2052110, TESTI2052202, TESTI2052211, TESTI2052256, TESTI2052288, TESTI2052525, TESTI2052670, TESTI2052693, TESTI2052698, TESTI2052799, TESTI2052822, TESTI2052985, TESTI2053151, TESTI2053242,
TESTI2053399, TESTI2053526, TESTI2053561, TESTI2053621, TESTI2053667, TESTI3000002, TESTI3000005, TESTI4000068, TESTI4000079, TESTI4000093, TESTI4000134, TESTI4000137, TESTI4000153, TESTI4000155, TESTI4000157, TESTI4000183, TESTI4000214, TESTI4000215, TESTI4000228, TESTI4000250, TESTI4000288, TESTI4000319, TESTI4000349, TESTI4000370, TESTI4000394, TESTI4000434, TESTI4000462, TESTI4000530, TESTI4000534, TESTI4000598, TESTI4000600, TESTI4000621, TESTI4000703, TESTI4000724, TESTI4000957, TESTI4000970, TESTI4001036, TESTI4001037, TESTI4001060, TESTI4001106, TESTI4001148, TESTI4001176, TESTI4001195, TESTI4001201, TESTI4001206, TESTI4001348, TESTI4001441, TESTI4001473, TESTI4001517, TESTI4001527,
TESTI4001561, TESTI4001569, TESTI4001604, TESTI4001665, TESTI4001679, TESTI4001795, TESTI4001923, TESTI4001925, TESTI4001984, TESTI4002003, TESTI4002141, TESTI4002202, TESTI4002290, TESTI4002319, TESTI4002491, TESTI4002520, TESTI4002552, TESTI4002647, TESTI4002703, TESTI4002754, TESTI4002868, TESTI4002878, TESTI4002889, TESTI4002988, TESTI4003150, TESTI4003179, TESTI4003279, TESTI4003319, TESTI4003404, TESTI4003565, TESTI4003574, TESTI4003579, TESTI4003602, TESTI4003733, TESTI4003796, TESTI4004003, TESTI4004031, TESTI4004200, TESTI4004210, TESTI4004539, TESTI4004626, TESTI4004653, TESTI4004695, TESTI4004722, TESTI4004887, TESTI4004917, TESTI4005015, TESTI4005039, TESTI4005133, TESTI4005158,
TESTI4005317, TESTI4005322, TESTI4005470, TESTI4005500, TESTI4005543, TESTI4005628, TESTI4005801, TESTI4005805, TESTI4005857, TESTI4005961, TESTI4006053, TESTI4006079, TESTI4006112, TESTI4006137, TESTI4006148, TESTI4006219, TESTI4006234, TESTI4006308, TESTI4006326, TESTI4006393, TESTI4006407, TESTI4006412, TESTI4006420, TESTI4006441, TESTI4006473, TESTI4006539, TESTI4006546, TESTI4006567, TESTI4006704, TESTI4006728, TESTI4006802, TESTI4006819, TESTI4007064, TESTI4007163, TESTI4007176, TESTI4007203, TESTI4007239, TESTI4007369, TESTI4007373, TESTI4007382, TESTI4007404, TESTI4007489, TESTI4007565, TESTI4007671, TESTI4007775, TESTI4007778, TESTI4007799, TESTI4007810, TESTI4007816, TESTI4007965,
TESTI4008007, TESTI4008018, TESTI4008050, TESTI4008058, TESTI4008086, TESTI4008097, TESTI4008176, TESTI4008209, TESTI4008219, TESTI4008250, TESTI4008302, TESTI4008305, TESTI4008401, TESTI4008417, TESTI4008429, TESTI4008573, TESTI4008816, TESTI4008840, TESTI4008935, TESTI4008993, TESTI4009022, TESTI4009028, TESTI4009034, TESTI4009123, TESTI4009160, TESTI4009215, TESTI4009283, TESTI4009306, TESTI4009374, TESTI4009406, TESTI4009454, TESTI4009457, TESTI4009501, TESTI4009563, TESTI4009608, TESTI4009638, TESTI4009752, TESTI4009881, TESTI4009886, TESTI4009928, TESTI4010076, TESTI4010082, TESTI4010095, TESTI4010211, TESTI4010377, TESTI4010475, TESTI4010544, TESTI4010713, TESTI4010721, TESTI4010789,
TESTI4010817, TESTI4010831, TESTI4010851, TESTI4010902, TESTI4010928, TESTI4010979, TESTI4011070, TESTI4011072, TESTI4011084, TESTI4011118, TESTI4011246, TESTI4011484, TESTI4011505, TESTI4011532, TESTI4011616, TESTI4011744, TESTI4011745, TESTI4011829, TESTI4011926, TESTI4011956, TESTI4012010, TESTI4012086, TESTI4012258, TESTI4012293, TESTI4012329, TESTI4012382, TESTI4012406, TESTI4012448, TESTI4012451, TESTI4012505, TESTI4012556, TESTI4012623, TESTI4012646, TESTI4012679, TESTI4012911, TESTI4012956, TESTI4012960, TESTI4013365, TESTI4013369, TESTI4013410, TESTI4013474, TESTI4013602, TESTI4013667, TESTI4013685, TESTI4013735, TESTI4013742, TESTI4013774, TESTI4013817, TESTI4013830, TESTI4013852,
TESTI4013924, TESTI4013960, TESTI4013962, TESTI4014175, TESTI4014265, TESTI4014276, TESTI4014306, TESTI4014392, TESTI4014415, TESTI4014445, TESTI4014553, TESTI4014661, TESTI4014694, TESTI4014801, TESTI4014818, TESTI4014891, TESTI4014908, TESTI4014924, TESTI4014932, TESTI4014977, TESTI4015012, TESTI4015129, TESTI4015145, TESTI4015204, TESTI4015263, TESTI4015293, TESTI4015299, TESTI4015339, TESTI4015398, TESTI4015442, TESTI4015471, TESTI4015477, TESTI4015600, TESTI4015646, TESTI4015681, TESTI4015688, TESTI4015790, TESTI4016110, TESTI4016238, TESTI4016551, TESTI4016812, TESTI4016822, TESTI4016865, TESTI4016882, TESTI4016925, TESTI4017001, TESTI4017116, TESTI4017137, TESTI4017229, TESTI4017254,
TESTI4017269, TESTI4017380, TESTI4017382, TESTI4017543, TESTI4017575, TESTI4017647, TESTI4017714, TESTI4017763, TESTI4017848, TESTI4017854, TESTI4017901, TESTI4017961, TESTI4017984, TESTI4018101, TESTI4018152, TESTI4018208, TESTI4018382, TESTI4018436, TESTI4018506, TESTI4018555, TESTI4018751, TESTI4018806, TESTI4018835, TESTI4018859, TESTI4018881, TESTI4018938, TESTI4019140, TESTI4019149, TESTI4019299, TESTI4019417, TESTI4019440, TESTI4019566, TESTI4019657, TESTI4019756, TESTI4019843, TESTI4020092, TESTI4020102, TESTI4020342, TESTI4020460, TESTI4020596, TESTI4020806, TESTI4020819, TESTI4020920, TESTI4021129, TESTI4021197, TESTI4021294, TESTI4021377, TESTI4021456, TESTI4021491, TESTI4021569,
TESTI4021713, TESTI4021821, TESTI4022158, TESTI4022577, TESTI4022716, TESTI4022873, TESTI4023096, TESTI4023172, TESTI4023546, TESTI4023555, TESTI4023654, TESTI4023722, TESTI4023762, TESTI4023942, TESTI4024096, TESTI4024240, TESTI4024294, TESTI4024344, TESTI4024387, TESTI4024420, TESTI4024494, TESTI4024740, TESTI4024874, TESTI4024890, TESTI4024907, TESTI4024930, TESTI4025062, TESTI4025268, TESTI4025401, TESTI4025494, TESTI4025547, TESTI4025731, TESTI4025797, TESTI4025865, TESTI4025908, TESTI4025920, TESTI4026079, TESTI4026080, TESTI4026192, TESTI4026207, TESTI4026295, TESTI4026390, TESTI4026456, TESTI4026510, TESTI4026524, TESTI4026680, TESTI4026700, TESTI4026762, TESTI4026785, TESTI4027139,
TESTI4027170, TESTI4027262, TESTI4027516, TESTI4027557, TESTI4027660, TESTI4027821, TESTI4027969, TESTI4028042, TESTI4028059, TESTI4028062, TESTI4028182, TESTI4028429, TESTI4028612, TESTI4028692, TESTI4028809, TESTI4028823, TESTI4028880, TESTI4028904, TESTI4028938, TESTI4028958, TESTI4028983, TESTI4029023, TESTI4029348, TESTI4029370, TESTI4029528, TESTI4029651, TESTI4029676, TESTI4029690, TESTI4029731, TESTI4029743, TESTI4030069, TESTI4030159, TESTI4030319, TESTI4030505, TESTI4030603, TESTI4030669, TESTI4030673, TESTI4030864, TESTI4031066, TESTI4031173, TESTI4031335, TESTI4031745, TESTI4031818, TESTI4032090, TESTI4032112, TESTI4032128, TESTI4032270, TESTI4032375, TESTI4032834, TESTI4032856,
TESTI4032895, TESTI4032913, TESTI4033146, TESTI4033177, TESTI4033433, TESTI4033690, TESTI4034172, TESTI4034212, TESTI4034432, TESTI4034495, TESTI4034632, TESTI4034633, TESTI4034912, TESTI4034973, TESTI4035063, TESTI4035065, TESTI4035498, TESTI4035508, TESTI4035581, TESTI4035602, TESTI4035637, TESTI4035649, TESTI4035770, TESTI4035872, TESTI4035895, TESTI4035898, TESTI4035989, TESTI4036012, TESTI4036042, TESTI4036048, TESTI4036315, TESTI4036319, TESTI4036449, TESTI4036767, TESTI4036909, TESTI4037066, TESTI4037188, TESTI4037228, TESTI4037244, TESTI4037727, TESTI4037949, TESTI4038047, TESTI4038099, TESTI4038156, TESTI4038223, TESTI4038258, TESTI4038339, TESTI4038492, TESTI4038721, TESTI4038758,
TESTI4038779, TESTI4038818, TESTI4039038, TESTI4039086, TESTI4039451, TESTI4039575, TESTI4039659, TESTI4039744, TESTI4039904, TESTI4040197, TESTI4040363, TESTI4040535, TESTI4040559, TESTI4040598, TESTI4040800, TESTI4040804, TESTI4040939, TESTI4040956, TESTI4041049, TESTI4041053, TESTI4041086, TESTI4041099, TESTI4041143, TESTI4041482, TESTI4041519, TESTI4041624, TESTI4041629, TESTI4041832, TESTI4041844, TESTI4041903, TESTI4041954, TESTI4041984, TESTI4041985, TESTI4041986, TESTI4042098, TESTI4042420, TESTI4042440, TESTI4042444, TESTI4042711, TESTI4042846, TESTI4043067, TESTI4043129, TESTI4043140, TESTI4043166, TESTI4043203, TESTI4043223, TESTI4043371, TESTI4043378, TESTI4043512, TESTI4043551,
TESTI4043710, TESTI4043947, TESTI4044035, TESTI4044076, TESTI4044084, TESTI4044123, TESTI4044186, TESTI4044234, TESTI4044291, TESTI4044296, TESTI4044300, TESTI4044682, TESTI4044704, TESTI4044770, TESTI4045168, TESTI4045312, TESTI4045330, TESTI4045470, TESTI4045701, TESTI4045908, TESTI4045955, TESTI4045982, TESTI4046073, TESTI4046090, TESTI4046240, TESTI4046245, TESTI4046253, TESTI4046282, TESTI4046328, TESTI4046450, TESTI4046457, TESTI4046487, TESTI4046502, TESTI4046523, TESTI4046819, TESTI4046873, TESTI4046884, TESTI4046896, TESTI4046903, TESTI4046962, TESTI4047069, TESTI4047119, TESTI4047305, TESTI4047328, TESTI4047437, TESTI4047569, TESTI4047746, TESTI4047808, THYMU1000010, THYMU1000017 ,
THYMU1000025, THYMU1000029, THYMU1000032, THYMU1000033, THYMU1000060, THYMU1000074, THYMU1000098, THYMU1000105, THYMU1000136, THYMU1000144, THYMU1000158, THYMU1000176, THYMU1000179, THYMU1000191, THYMU1000198, THYMU1000217, THYMU1000240, THYMU1000242, THYMU1000291, THYMU1000318, THYMU1000329, THYMU1000331, THYMU1000336, THYMU1000366, THYMU1000382, THYMU1000393, THYMU1000399, THYMU1000426, THYMU1000440, THYMU1000459, THYMU1000473, THYMU1000490, THYMU1000491, THYMU1000496, THYMU1000499, THYMU1000527, THYMU1000532, THYMU1000536, THYMU1000554, THYMU1000558, THYMU1000576, THYMU1000600, THYMU1000677, THYMU1000692, THYMU2000022, THYMU2000032, THYMU2000057, THYMU2000120, THYMU2000138, THYMU2000140,
THYMU2000155, THYMU2000162, THYMU2000171, THYMU2000176, THYMU2000179, THYMU2000231, THYMU2000236, THYMU2000276, THYMU2000280, THYMU2000300, THYMU2000317, THYMU2000368, THYMU2000369, THYMU2000418, THYMU2000440, THYMU2000489, THYMU2000492, THYMU2000519, THYMU2000570, THYMU2000602, THYMU2000660, THYMU2000671, THYMU2000672, THYMU2000702, THYMU2000767, THYMU2000775, THYMU2000800, THYMU2000879, THYMU2000932, THYMU2000946, THYMU2000950, THYMU2000971, THYMU2000982, THYMU2000990, THYMU2001018, THYMU2001053, THYMU2001071, THYMU2001090, THYMU2001139, THYMU2001195, THYMU2001202, THYMU2001203, THYMU2001256, THYMU2001270, THYMU2001291, THYMU2001314, THYMU2001325, THYMU2001357, THYMU2001381, THYMU2001443,
THYMU2001515, THYMU2001521, THYMU2001556, THYMU2001622, THYMU2001727, THYMU2001819, THYMU2001825, THYMU2001839, THYMU2001868, THYMU2001898, THYMU2001900, THYMU2001916, THYMU2001926, THYMU2001989, THYMU2002023, THYMU2002037, THYMU2002080, THYMU2002109, THYMU2002147, THYMU2002154, THYMU2002157, THYMU2002162, THYMU2002177, THYMU2002203, THYMU2002229, THYMU2002242, THYMU2002338, THYMU2002356, THYMU2002376, THYMU2002384, THYMU2002450, THYMU2002490, THYMU2002548, THYMU2002695, THYMU2002745, THYMU2002756, THYMU2002789, THYMU2002792, THYMU2002841, THYMU2002852, THYMU2002910, THYMU2002950, THYMU2002983, THYMU2003069, THYMU2003107, THYMU2003124, THYMU2003133, THYMU2003166, THYMU2003232, THYMU2003242,
THYMU2003249, THYMU2003270, THYMU2003282, THYMU2003287, THYMU2003336, THYMU2003366, THYMU2003397, THYMU2003440, THYMU2003470, THYMU2003479, THYMU2003499, THYMU2003542, THYMU2003632, THYMU2003650, THYMU2003700, THYMU2003760, THYMU2003803, THYMU2003855, THYMU2003907, THYMU2003932, THYMU2004003, THYMU2004008, THYMU2004042, THYMU2004108, THYMU2004111, THYMU2004123, THYMU2004139, THYMU2004169, THYMU2004284, THYMU2004320, THYMU2004336, THYMU2004344, THYMU2004356, THYMU2004452, THYMU2004508, THYMU2004512, THYMU2004635, THYMU2004639, THYMU2004668, THYMU2004677, THYMU2004688, THYMU2004693,THYMU2004733, THYMU2004835, THYMU2004843, THYMU2004906, THYMU2004916, THYMU2004986, THYMU2005001, THYMU2005003,
THYMU2005046, THYMU2005134, THYMU2005156, THYMU2005190, THYMU2005225, THYMU2005240, THYMU2005246, THYMU2005270, THYMU2005283, THYMU2005303, THYMU2005321, THYMU2005356, THYMU2005369, THYMU2005439, THYMU2005480, THYMU2005482, THYMU2005507, THYMU2005545, THYMU2005574, THYMU2005576, THYMU2005748, THYMU2005759, THYMU2005807, THYMU2005815, THYMU2005855, THYMU2005881, THYMU2005948, THYMU2005980, THYMU2005997, THYMU2006001, THYMU2006048, THYMU2006098, THYMU2006160, THYMU2006170, THYMU2006252, THYMU2006261, THYMU2006277, THYMU2006303, THYMU2006324, THYMU2006350, THYMU2006357, THYMU2006365, THYMU2006420, THYMU2006468, THYMU2006610, THYMU2006666, THYMU2006712, THYMU2006913, THYMU2006946, THYMU2006965,
THYMU2007025, THYMU2007036, THYMU2007060, THYMU2007065, THYMU2007095, THYMU2007112, THYMU2007146, THYMU2007179, THYMU2007258, THYMU2007307, THYMU2007308, THYMU2007339, THYMU2007350, THYMU2007384, THYMU2007493, THYMU2007521, THYMU2007528, THYMU2007530, THYMU2007532, THYMU2007561, THYMU2007571, THYMU2007635, THYMU2007658, THYMU2007667, THYMU2007694, THYMU2007725, THYMU2007729, THYMU2007802, THYMU2007824, THYMU2007854, THYMU2007952, THYMU2007969, THYMU2008035, THYMU2008036, THYMU2008049, THYMU2008072, THYMU2008111, THYMU2008145, THYMU2008149, THYMU2008185, THYMU2008191, THYMU2008207, THYMU2008226, THYMU2008282, THYMU2008321, THYMU2008339, THYMU2008350, THYMU2008351, THYMU2008383, THYMU2008452,
THYMU2008643, THYMU2008691, THYMU2008714, THYMU2008727, THYMU2008781, THYMU2008799, THYMU2008876, THYMU2008910, THYMU2008917, THYMU2008928, THYMU2008990, THYMU2008994, THYMU2009023, THYMU2009046, THYMU2009101, THYMU2009104, THYMU2009134, THYMU2009157, THYMU2009181, THYMU2009186, THYMU2009338, THYMU2009425, THYMU2009516, THYMU2009526, THYMU2009546, THYMU2009592, THYMU2009596, THYMU2009792, THYMU2009835, THYMU2009906, THYMU2009948, THYMU2010030, THYMU2010041, THYMU2010046, THYMU2010059, THYMU2010082, THYMU2010161, THYMU2010192, THYMU2010519, THYMU2010637, THYMU2010671, THYMU2010699, THYMU2010705, THYMU2010779, THYMU2010816, THYMU2010831, THYMU2010848, THYMU2011053, THYMU2011063, THYMU2011072,
THYMU2011096, THYMU2011118, THYMU2011142, THYMU2011183, THYMU2011257, THYMU2011259, THYMU2011295, THYMU2011308, THYMU2011390, THYMU2011431, THYMU2011447, THYMU2011482, THYMU2011538, THYMU2011548, THYMU2011551, THYMU2011564, THYMU2011573, THYMU2011585, THYMU2011621, THYMU2011666, THYMU2011736, THYMU2011785, THYMU2011806, THYMU2011852, THYMU2011875, THYMU2011881, THYMU2011937, THYMU2011939, THYMU2012002, THYMU2012073, THYMU2012104, THYMU2012113, THYMU2012273, THYMU2012401, THYMU2012492, THYMU2012507, THYMU2012565, THYMU2012574, THYMU2012625, THYMU2012631, THYMU2012665, THYMU2012690, THYMU2012701, THYMU2012807, THYMU2012826, THYMU2012882, THYMU2012891, THYMU2012902, THYMU2012919, THYMU2013023,
THYMU2013047, THYMU2013051, THYMU2013089, THYMU2013136, THYMU2013170, THYMU2013364, THYMU2013386, THYMU2013426, THYMU2013578, THYMU2013648, THYMU2013705, THYMU2013757, THYMU2013779, THYMU2013863, THYMU2013950, THYMU2013981, THYMU2014067, THYMU2014068, THYMU2014167, THYMU2014183, THYMU2014204, THYMU2014297, THYMU2014323, THYMU2014327, THYMU2014353, THYMU2014492, THYMU2014500, THYMU2014599, THYMU2014762, THYMU2014777, THYMU2014801, THYMU2014901, THYMU2014923, THYMU2015019, THYMU2015161, THYMU2015316, THYMU2015439, THYMU2015441, THYMU2015479, THYMU2015762, THYMU2015775, THYMU2015825, THYMU2015918, THYMU2015943, THYMU2015984, THYMU2016113, THYMU2016164, THYMU2016204, THYMU2016219, THYMU2016360,
THYMU2016496, THYMU2016523, THYMU2016840, THYMU2016968, THYMU2017008, THYMU2017010, THYMU2017023, THYMU2017085, THYMU2017099, THYMU2017158, THYMU2017162, THYMU2017215, THYMU2017362, THYMU2017366, THYMU2017398, THYMU2017449, THYMU2017479, THYMU2017522, THYMU2017526, THYMU2017601, THYMU2017675, THYMU2017707, THYMU2017831, THYMU2017844, THYMU2017878, THYMU2017948, THYMU2018028, THYMU2018071, THYMU2018126, THYMU2018189, THYMU2018197, THYMU2018384, THYMU2018455, THYMU2018547, THYMU2018565, THYMU2018639, THYMU2018673, THYMU2018721, THYMU2018772, THYMU2018819, THYMU2018841, THYMU2018997, THYMU2019054, THYMU2019197, THYMU2019210, THYMU2019364, THYMU2019436, THYMU2019442, THYMU2019587, THYMU2019599,
THYMU2019686, THYMU2019813, THYMU2020198, THYMU2020289, THYMU2020416, THYMU2020491, THYMU2020499, THYMU2020560, THYMU2020830, THYMU2020883, THYMU2020959, THYMU2021361, THYMU2021509, THYMU2021597, THYMU2021714, THYMU2022213, THYMU2022289, THYMU2022660, THYMU2022854, THYMU2022922, THYMU2023209, THYMU2023264, THYMU2023576, THYMU2023711, THYMU2023900, THYMU2023943, THYMU2023967, THYMU2024121, THYMU2024185, THYMU2024207, THYMU2024616, THYMU2024684, THYMU2024748, THYMU2025042, THYMU2025189, THYMU2025325, THYMU2025406, THYMU2025557, THYMU2025572, THYMU2025707, THYMU2025849, THYMU2025909, THYMU2025939, THYMU2026182, THYMU2026350, THYMU2026530, THYMU2026531, THYMU2026835, THYMU2026904, THYMU2027053,
THYMU2027125, THYMU2027168, THYMU2027249, THYMU2027282, THYMU2027497, THYMU2027695, THYMU2027734, THYMU2027894, THYMU2027975, THYMU2027996, THYMU2028041, THYMU2028368, THYMU2028379, THYMU2028394, THYMU2028412, THYMU2028629, THYMU2028632, THYMU2028724, THYMU2028739, THYMU2028942, THYMU2028978, THYMU2029134, THYMU2029578, THYMU2029676, THYMU2029688, THYMU2029788, THYMU2030068, THYMU2030226, THYMU2030264, THYMU2030462, THYMU2030543, THYMU2030637, THYMU2030655, THYMU2030796, THYMU2030912, THYMU2030982, THYMU2031046, THYMU2031139, THYMU2031218, THYMU2031249, THYMU2031258, THYMU2031341, THYMU2031368, THYMU2031579, THYMU2031847, THYMU2031994, THYMU2032014, THYMU2032035, THYMU2032080, THYMU2032150,
THYMU2032155, THYMU2032358, THYMU2032437, THYMU2032601, THYMU2032655, THYMU2032696, THYMU2032715, THYMU2032732, THYMU2032825, THYMU2032976, THYMU2033053, THYMU2033059, THYMU2033079, THYMU2033085, THYMU2033104, THYMU2033227, THYMU2033263, THYMU2033308, THYMU2033401, THYMU2033583, THYMU2033755, THYMU2033787, THYMU2033816, THYMU2034182, THYMU2034279, THYMU2034314, THYMU2034338, THYMU2034374, THYMU2034647, THYMU2035064, THYMU2035078, THYMU2035101, THYMU2035319, THYMU2035321, THYMU2035388, THYMU2035400, THYMU2035554, THYMU2035710, THYMU2036058, THYMU2036105, THYMU2036207, THYMU2036215, THYMU2036252, THYMU2036265, THYMU2036366, THYMU2036459, THYMU2036604, THYMU2036653, THYMU2037081, THYMU2037208,
THYMU2037226, THYMU2037233, THYMU2037234, THYMU2037348, THYMU2037406, THYMU2037965, THYMU2038058, THYMU2038189, THYMU2038199, THYMU2038220, THYMU2038301, THYMU2038369, THYMU2038389, THYMU2038615, THYMU2038636, THYMU2038739, THYMU2038772, THYMU2038797, THYMU2038906, THYMU2039305, THYMU2039315, THYMU2039334, THYMU2039350, THYMU2039411, THYMU2039634, THYMU2039719, THYMU2039780, THYMU2039788, THYMU2039989, THYMU2040029, THYMU2040110, THYMU2040114, THYMU2040140, THYMU2040412, THYMU2040427,. THYMU2040824, THYMU2040925, THYMU2041007, THYMU2041015, THYMU2041252, THYMU3000028, THYMU3000036, THYMU3000037, THYMU3000082, THYMU3000194, THYMU3000224, THYMU3000269, THYMU3000306, THYMU3000360, THYMU3000390,
THYMU3000420, THYMU3000490, THYMU3000504, THYMU3000542, THYMU3000655, THYMU3000776, THYMU3000826, THYMU3000841, THYMU3001077, THYMU3001082, THYMU3001083, THYMU3001133, THYMU3001151, THYMU3001379, THYMU3001593, THYMU3001883, THYMU3001939, THYMU3001991, THYMU3002452, THYMU3002661, THYMU3003007, THYMU3003092, THYMU3003212, THYMU3003309, THYMU3003350, THYMU3003403, THYMU3003565, THYMU3003763, THYMU3003865, THYMU3003958, THYMU3003965, THYMU3004157, THYMU3004628, THYMU3004632, THYMU3004835, THYMU3004866, THYMU3005050, THYMU3005378, THYMU3005407, THYMU3005415, THYMU3005427, THYMU3005482, THYMU3005491, THYMU3005629, THYMU3005696, THYMU3006118, THYMU3006132, THYMU3006168, THYMU3006172, THYMU3006371,
THYMU3006485, THYMU3006640, THYMU3006767, THYMU3006811, THYMU3006963, THYMU3007137, THYMU3007308, THYMU3007368, THYMU3007423, THYMU3007559, THYMU3007845, THYMU3008060, THYMU3008105, THYMU3008136, THYMU3008171, THYMU3008216, THYMU3008436, THYMU3008672, THYMU3008883, THYMU3008935, THYMU3009006, THYMU3009255, THYMU3009391, THYMU3009643, THYMU3009755, THYMU3010247, THYMU3011012, THYMU3011081, THYMU3011244, THYMU3011360, THYMU3011534, THYMU3011543, THYMU3011556, THYMU3011717, THYMU3011827, THYMU3012402, THYMU3012907, THYMU3012983, THYMU3013114, THYMU3013197, THYMU3013241, THYMU3013470, THYMU3013785, THYMU3013897, THYMU3014038, THYMU3014173, THYMU3014372, THYMU3014555, THYMU3014620, THYMU3014701,
THYMU3015042, THYMU3015457, THYMU3015571, THYMU3015592, THYMU3015625, THYMU3015647, THYMU3015759, THYMU3015962, THYMU3016022, THYMU3016518, THYMU3016797, THYMU3016822, THYMU3016850, THYMU3017562, THYMU3017688, THYMU3017689, THYMU3017761, THYMU3018151, THYMU3018896, THYMU3019095, THYMU3019476, THYMU3019605, THYMU3019916, THYMU3020221, THYMU3020595, THYMU3020713, THYMU3020856, THYMU3020869, THYMU3020970, THYMU3021404, THYMU3021755, THYMU3021900, THYMU3022211, THYMU3022434, THYMU3022528, THYMU3022668, THYMU3022982, THYMU3023107, THYMU3023211, THYMU3023216, THYMU3023394, THYMU3023400, THYMU3023797, THYMU3024164, THYMU3024339, THYMU3024602, THYMU3024879, THYMU3025118, THYMU3025313, THYMU3025642,
THYMU3025683, THYMU3025772, THYMU3025865, THYMU3026000, THYMU3026479, THYMU3026532, THYMU3026783, THYMU3026869, THYMU3027251, THYMU3027540, THYMU3027655, THYMU3027671, THYMU3028075, THYMU3028461, THYMU3028702, THYMU3029188, THYMU3029318, THYMU3029421, THYMU3029719, THYMU3029774, THYMU3029832, THYMU3030072, THYMU3030231, THYMU3030706, THYMU3030710, THYMU3030752, THYMU3031146, THYMU3031402, THYMU3031610, THYMU3031612, THYMU3031868, THYMU3031878, THYMU3032032, THYMU3032867, THYMU3033402, THYMU3033626, THYMU3033630, THYMU3033649, THYMU3033754, THYMU3033759, THYMU3034078, THYMU3034099, THYMU3034453, THYMU3034853, THYMU3034867, THYMU3034983, THYMU3036200, THYMU3036310, THYMU3036934, THYMU3036953,
THYMU3037052, THYMU3037192, THYMU3037617, THYMU3037772, THYMU3037827, THYMU3037836, THYMU3037856, THYMU3037867, THYMU3037909, THYMU3037980, THYMU3038158, THYMU3038167, THYMU3038214, THYMU3038266, THYMU3038328, THYMU3038347, THYMU3038375, THYMU3038603, THYMU3038687, THYMU3038759, THYMU3038879, THYMU3038970, THYMU3039807, THYMU3039846, THYMU3040068, THYMU3040126, THYMU3040146, THYMU3040168, THYMU3040172, THYMU3040725, THYMU3040746, THYMU3040816, THYMU3040829, THYMU3040830, THYMU3040986, THYMU3041354, THYMU3041386, THYMU3041428, THYMU3041573, THYMU3041603, THYMU3041736, THYMU3041918, THYMU3042075, THYMU3042321, THYMU3042372, THYMU3042758, THYMU3042929, THYMU3043200, THYMU3043327, THYMU3043482,
THYMU3043597, THYMU3043688, THYMU3043779, THYMU3043883, THYMU3043993, THYMU3044175, THYMU3044188, THYMU3044441, THYMU3044445, THYMU3045510, THYMU3045673, THYMU3045692, THYMU3045704, THYMU3046140, THYMU3046350, THYMU3046360, THYMU3046856, THYMU3047115, THYMU3047144, THYMU3047156, THYMU3047366, THYMU3047513, THYMU3047542, THYMU3047760, THYMU3047891, TKIDN1000001, TKIDN1000044, TKIDN1000062, TKIDN1000066, TKIDN1000164, TKIDN1000171, TKIDN1000192, TKIDN2000127, TKIDN2000240, TKIDN2000246, TKIDN2000319, TKIDN2000464, TKIDN2000521, TKIDN2000701, TKIDN2001529, TKIDN2001662, TKIDN2002318, TKIDN2002329, TKIDN2002424, TKIDN2002632, TKIDN2002738, TKIDN2003044, TKIDN2003059, TKIDN2003062, TKIDN2003078,
TKIDN2003366, TKIDN2003396, TKIDN2003413, TKIDN2003597, TKIDN2003601, TKIDN2004185, TKIDN2004386, TKIDN2004458, TKIDN2004748, TKIDN2005487, TKIDN2005721, TKIDN2005934, TKIDN2005947, TKIDN2005956, TKIDN2006525, TKIDN2006761, TKIDN2006852, TKIDN2007605, TKIDN2007667, TKIDN2007828, TKIDN2008176, TKIDN2008778, TKIDN2009092, TKIDN2009330, TKIDN2009481, TKIDN2009641, TKIDN2009794, TKIDN2009889, TKIDN2010200, TKIDN2010232, TKIDN2010278, TKIDN2010602, TKIDN2010812, TKIDN2010934, TKIDN2011051, TKIDN2011160, TKIDN2011289, TKIDN2011324, TKIDN2012034, TKIDN2012771, TKIDN2012824, TKIDN2013134, TKIDN2013287, TKIDN2014570, TKIDN2014757, TKIDN2014771, TKIDN2014964, TKIDN2015025, TKIDN2015071, TKIDN2015161,
TKIDN2015263, TKIDN2015285, TKIDN2015423, TKIDN2015788, TKIDN2016309, TKIDN2016399, TKIDN2016846, TKIDN2018926, TKIDN2019116, TLIVE2000023, TLIVE2000142, TLIVE2000292, TLIVE2000762, TLIVE2000979, TLIVE2001327, TLIVE2001616, TLIVE2001684, TLIVE2001828, TLIVE2001927, TLIVE2002046, TLIVE2002336, TLIVE2002338, TLIVE2002562, TLIVE2002690, TLIVE2002882, TLIVE2003197, TLIVE2003200, TLIVE2003225, TLIVE2003381, TLIVE2003737, TLIVE2003961, TLIVE2003970, TLIVE2004110, TLIVE2004320, TLIVE2004331, TLIVE2004601, TLIVE2005180, TLIVE2005390, TLIVE2005516, TLIVE2005731, TLIVE2005866, TLIVE2006236, TLIVE2006359, TLIVE2006529, TLIVE2006621, TLIVE2006733, TLIVE2007000, TLIVE2007016, TLIVE2007132, TLIVE2007192,
TLIVE2007528, TLIVE2007607, TLIVE2007736, TLIVE2007816, TLIVE2007919, TLIVE2008083, TLIVE2008213, TLIVE2008221, TLIVE2008229, TLIVE2008712, TLIVE2008797, TLIVE2009087, TLIVE2009163, TLIVE2009489, TLIVE2009541, TLUNG2000179, TOVAR2000575, TOVAR2000649, TOVAR2000904, TOVAR2001281, TOVAR2001556, TOVAR2001730, TOVAR2002247, TOVAR2002514, TOVAR2002549, TOVAR2002800, TRACH1000011, TRACH1000018, TRACH1000057, TRACH1000074, TRACH1000100, TRACH1000106, TRACH1000125, TRACH1000140, TRACH1000144, TRACH1000193, TRACH1000212, TRACH1000240, TRACH2000002, TRACH2000015, TRACH2000024, TRACH2000032, TRACH2000045, TRACH2000047, TRACH2000081, TRACH2000083, TRACH2000148, TRACH2000185, TRACH2000196, TRACH2000235,
TRACH2000243, TRACH2000250, TRACH2000259, TRACH2000272, TRACH2000282, TRACH2000287, TRACH2000289, TRACH2000306, TRACH2000312, TRACH2000336, TRACH2000393, TRACH2000411, TRACH2000420, TRACH2000496, TRACH2000497, TRACH2000502, TRACH2000522, TRACH2000540, TRACH2000559, TRACH2000660, TRACH2000665, TRACH2000675, TRACH2000677, TRACH2000702, TRACH2000703, TRACH2000767, TRACH2000775, TRACH2000807, TRACH2000862, TRACH2000873, TRACH2000894, TRACH2000898, TRACH2000920, TRACH2000923, TRACH2000926, TRACH2000944, TRACH2000972, TRACH2000981, TRACH2000986, TRACH2001037, TRACH2001060, TRACH2001085, TRACH2001101, TRACH2001109, TRACH2001121, TRACH2001147, TRACH2001154, TRACH2001188, TRACH2001249, TRACH2001275,
TRACH2001314, TRACH2001335, TRACH2001339, TRACH2001341, TRACH2001347, TRACH2001395, TRACH2001400, TRACH2001403, TRACH2001416, TRACH2001428, TRACH2001474, TRACH2001478, TRACH2001523, TRACH2001525, TRACH2001532, TRACH2001592, TRACH2001607, TRACH2001621, TRACH2001646, TRACH2001651, TRACH2001684, TRACH2001898, TRACH2001933, TRACH2001968, TRACH2001996, TRACH2002043, TRACH2002092, TRACH2002100, TRACH2002138, TRACH2002333, TRACH2002664, TRACH2002784, TRACH2002803, TRACH2002840, TRACH2002847, TRACH2002853, TRACH2002862, TRACH2002954, TRACH2002960, TRACH2002988, TRACH2003070, TRACH2003347, TRACH2003484, TRACH2003516, TRACH2003605, TRACH2003736, TRACH2003759, TRACH2003941, TRACH2004039, TRACH2004054,
TRACH2004087, TRACH2004109, TRACH2004170, TRACH2004183, TRACH2004251, TRACH2004292, TRACH2004336, TRACH2004399, TRACH2004428, TRACH2004458, TRACH2004499, TRACH2004521, TRACH2004570, TRACH2004845, TRACH2004887, TRACH2004950, TRACH2005004, TRACH2005017, TRACH2005066, TRACH2005159, TRACH2005314, TRACH2005444, TRACH2005541, TRACH2005666, TRACH2005698, TRACH2005710, TRACH2005720, TRACH2005769, TRACH2005796, TRACH2005800, TRACH2006015, TRACH2006049, TRACH2006097, TRACH2006194, TRACH2006327, TRACH2006331, TRACH2006378, TRACH2006387, TRACH2006528, TRACH2006670, TRACH2006762, TRACH2006866, TRACH2006870, TRACH2006918, TRACH2007059, TRACH2007324, TRACH2007358, TRACH2007483, TRACH2007542, TRACH2007577,
TRACH2007674, TRACH2007676, TRACH2007688, TRACH2007733, TRACH2007754, TRACH2007816, TRACH2007894, TRACH2007969, TRACH2007988, TRACH2008081, TRACH2008112, TRACH2008113, TRACH2008127, TRACH2008130, TRACH2008278, TRACH2008472, TRACH2008478, TRACH2008540, TRACH2008583, TRACH2008597, TRACH2008716, TRACH2008820, TRACH2008891, TRACH2008921, TRACH2009006, TRACH2009060, TRACH2009107, TRACH2009123, TRACH2009268, TRACH2009291, TRACH2009310, TRACH2009340, TRACH2009348, TRACH2009358, TRACH2009661, TRACH2009730, TRACH2009743, TRACH2009754, TRACH2009851, TRACH2009862, TRACH2009934, TRACH2010159, TRACH2010218, TRACH2010251, TRACH2010272, TRACH2010317, TRACH2010419, TRACH2010451, TRACH2010505, TRACH2010587,
TRACH2010600, TRACH2010634, TRACH2010639, TRACH2010677, TRACH2010698, TRACH2010771, TRACH2010824, TRACH2010883, TRACH2010965, TRACH2010974, TRACH2011057, TRACH2011093, TRACH2011113, TRACH2011290, TRACH2011293, TRACH2011302, TRACH2011500, TRACH2011574, TRACH2011894, TRACH2011956, TRACH2012000, TRACH2012138, TRACH2012242, TRACH2012298, TRACH2012370, TRACH2012376, TRACH2012387, TRACH2012497, TRACH2012536, TRACH2012562, TRACH2012742, TRACH2012811, TRACH2012823, TRACH2012918, TRACH2013081, TRACH2013123, TRACH2013265, TRACH2013369, TRACH2013450, TRACH2013495, TRACH2013552, TRACH2013585, TRACH2013671, TRACH2013726, TRACH2013902, TRACH2013928, TRACH2013982, TRACH2013991, TRACH2014018, TRACH2014045,
TRACH2014046, TRACH2014077, TRACH2014082, TRACH2014268, TRACH2014284, TRACH2014337, TRACH2014356, TRACH2014371, TRACH2014483, TRACH2014495, TRACH2014544, TRACH2014573, TRACH2014725, TRACH2014760, TRACH2014815, TRACH2014839, TRACH2014938, TRACH2014950, TRACH2014972, TRACH2014974, TRACH2015180, TRACH2015243, TRACH2015316, TRACH2015381, TRACH2015486, TRACH2015654, TRACH2015824, TRACH2015939, TRACH2015987, TRACH2016080, TRACH2016131, TRACH2016286, TRACH2016317, TRACH2016347, TRACH2016380, TRACH2016384, TRACH2016410, TRACH2016498, TRACH2016533, TRACH2016551, TRACH2016554, TRACH2016651, TRACH2016690, TRACH2016709, TRACH2016722, TRACH2016835, TRACH2016980, TRACH2017086, TRACH2017368, TRACH2017498,
TRACH2017949, TRACH2017965, TRACH2017980, TRACH2018034, TRACH2018084, TRACH2018262, TRACH2018273, TRACH2018278, TRACH2018317, TRACH2018345, TRACH2018380, TRACH2018446, TRACH2018449, TRACH2018512, TRACH2018646, TRACH2018663, TRACH2018718, TRACH2018835, TRACH2018882, TRACH2018914, TRACH2019024, TRACH2019046, TRACH2019080, TRACH2019151, TRACH2019154, TRACH2019176, TRACH2019248, TRACH2019309, TRACH2019425, TRACH2019473, TRACH2019602, TRACH2019661, TRACH2019672, TRACH2019673, TRACH2019844, TRACH2019879, TRACH2020048, TRACH2020336, TRACH2020525, TRACH2021398, TRACH2021622, TRACH2021635, TRACH2021964, TRACH2022042, TRACH2022113, TRACH2022253, TRACH2022425, TRACH2022553, TRACH2022649, TRACH2022839,
TRACH2023246, TRACH2023299, TRACH2023306, TRACH2023416, TRACH2023479, TRACH2023684, TRACH2023860, TRACH2023876, TRACH2024139, TRACH2024187, TRACH2024271, TRACH2024293, TRACH2024408, TRACH2024512, TRACH2024559, TRACH2024647, TRACH2024651, TRACH2024730, TRACH2024735, TRACH2024749, TRACH2024821, TRACH2025057, TRACH2025084, TRACH2025155, TRACH2025224, TRACH2025344, TRACH2025379, TRACH2025507, TRACH2025535, TRACH2025569, TRACH2025705, TRACH2025749, TRACH2025753, TRACH2025810, TRACH2025853, TRACH2025911, TRACH2025932, TRACH3000014, TRACH3000017, TRACH3000043, TRACH3000134, TRACH3000180, TRACH3000342, TRACH3000420, TRACH3000515, TRACH3000530, TRACH3000548, TRACH3000558, TRACH3000586, TRACH3000780,
TRACH3000892, TRACH3000926, TRACH3001119, TRACH3001443, TRACH3001550, TRACH3001759, TRACH3002064, TRACH3002107, TRACH3002168, TRACH3002188, TRACH3002245, TRACH3002293, TRACH3002533, TRACH3002561, TRACH3002567, TRACH3002605, TRACH3002650, TRACH3002752, TRACH3002866, TRACH3002871, TRACH3002876, TRACH3002890, TRACH3003009, TRACH3003037, TRACH3003357, TRACH3003586, TRACH3003683, TRACH3003780, TRACH3003805, TRACH3003832, TRACH3004113, TRACH3004288, TRACH3004303, TRACH3004412, TRACH3004424, TRACH3004456, TRACH3004537, TRACH3004596, TRACH3004651, TRACH3004652, TRACH3004671, TRACH3004747, TRACH3004760, TRACH3004783, TRACH3004786, TRACH3004840, TRACH3004931, TRACH3004934, TRACH3005072, TRACH3005085,
TRACH3005102, TRACH3005173, TRACH3005191, TRACH3005211, TRACH3005274, TRACH3005294, TRACH3005479, TRACH3005549, TRACH3005702, TRACH3005808, TRACH3005852, TRACH3005896, TRACH3006038, TRACH3006149, TRACH3006228, TRACH3006264, TRACH3006336, TRACH3006379, TRACH3006392, TRACH3006395, TRACH3006397, TRACH3006412, TRACH3006470, TRACH3006589, TRACH3006626, TRACH3006699, TRACH3006717, TRACH3006889, TRACH3006985, TRACH3007097, TRACH3007268, TRACH3007274, TRACH3007277, TRACH3007391, TRACH3007541, TRACH3007595, TRACH3007625, TRACH3007627, TRACH3007689, TRACH3007766, TRACH3007866, TRACH3007995, TRACH3008042, TRACH3008061, TRACH3008093, TRACH3008201, TRACH3008508, TRACH3008535, TRACH3008629, TRACH3008688,
TRACH3008697, TRACH3008713, TRACH3008887, TRACH3008902, TRACH3008990, TRACH3009001, TRACH3009059, TRACH3009061, TRACH3009455, TRACH3009701, TRACH3009715, TRACH3009894, TRACH3009956, TRACH3010079, TRACH3010167, TRACH3010331, TRACH3010342, TRACH3010382, TRACH3010757, TRACH3010901, TRACH3011004, TRACH3011082, TRACH3011184, TRACH3011282, TRACH3011313, TRACH3011454, TRACH3011485, TRACH3011503, TRACH3011538, TRACH3011542, TRACH3012106, TRACH3012161, TRACH3012232, TRACH3012261, TRACH3012445, TRACH3012460, TRACH3012659, TRACH3012718, TRACH3012864, TRACH3012891, TRACH3012942, TRACH3013043, TRACH3013072, TRACH3013426, TRACH3013454, TRACH3013558, TRACH3013684, TRACH3013693, TRACH3013700, TRACH3013766,
TRACH3013900, TRACH3013926, TRACH3014063, TRACH3014183, TRACH3014215, TRACH3014316, TRACH3014415, TRACH3014580, TRACH3014641, TRACH3014885, TRACH3015136, TRACH3015336, TRACH3015346, TRACH3015354, TRACH3015467, TRACH3015626, TRACH3015951, TRACH3016051, TRACH3016120, TRACH3016264, TRACH3016277, TRACH3016368, TRACH3016455, TRACH3016614, TRACH3016805, TRACH3016992, TRACH3017171, TRACH3017409, TRACH3017934, TRACH3018108, TRACH3018191, TRACH3018240, TRACH3018261, TRACH3018519, TRACH3018524, TRACH3018606, TRACH3018783, TRACH3018907, TRACH3018933, TRACH3018943, TRACH3019018, TRACH3019058, TRACH3019142, TRACH3019290, TRACH3019347, TRACH3019370, TRACH3019447, TRACH3019598, TRACH3019621, TRACH3019662,
TRACH3019807, TRACH3019924, TRACH3019977, TRACH3020137, TRACH3020410, TRACH3020563, TRACH3020605, TRACH3020750, TRACH3020769, TRACH3020928, TRACH3020930, TRACH3021023, TRACH3021066, TRACH3021212, TRACH3021335, TRACH3021373, TRACH3021544, TRACH3021778, TRACH3021834, TRACH3021883, TRACH3022170, TRACH3022198, TRACH3022296, TRACH3022482, TRACH3022732, TRACH3022758, TRACH3022875, TRACH3022910, TRACH3022960, TRACH3023063, TRACH3023203, TRACH3023242, TRACH3023373, TRACH3023516, TRACH3023752, TRACH3023945, TRACH3023960, TRACH3024020, TRACH3024081, TRACH3024342, TRACH3024423, TRACH3024428, TRACH3024512, TRACH3024671, TRACH3024823, TRACH3025302, TRACH3025316, TRACH3025346, TRACH3025520, TRACH3026148,
TRACH3026283, TRACH3026299, TRACH3026303, TRACH3026542, TRACH3026650, TRACH3026676, TRACH3026744, TRACH3026949, TRACH3027229, TRACH3027527, TRACH3027681, TRACH3027701, TRACH3027885, TRACH3028031, TRACH3028164, TRACH3028180, TRACH3028195, TRACH3028441, TRACH3028597, TRACH3028678, TRACH3028837, TRACH3028847, TRACH3028855, TRACH3029139, TRACH3029329, TRACH3029451, TRACH3029462, TRACH3029520, TRACH3029592, TRACH3029629, TRACH3029665, TRACH3029670, TRACH3029800, TRACH3030111, TRACH3030130, TRACH3030176, TRACH3030252, TRACH3030725, TRACH3030855, TRACH3030883, TRACH3031316, TRACH3031400, TRACH3031463, TRACH3031479, TRACH3031518, TRACH3031660, TRACH3031678, TRACH3031936, TRACH3032044, TRACH3032065,
TRACH3032150, TRACH3032372, TRACH3032480, TRACH3032570, TRACH3032755, TRACH3032827, TRACH3032873, TRACH3033535, TRACH3033680, TRACH3033868, TRACH3034145, TRACH3034414, TRACH3034488, TRACH3034680, TRACH3034731, TRACH3034745, TRACH3034762, TRACH3034903, TRACH3035187, TRACH3035199, TRACH3035235, TRACH3035451, TRACH3035482, TRACH3035526, TRACH3036004, TRACH3036103, TRACH3036193, TRACH3036207, TRACH3036278, TRACH3036309, TRACH3036456, TRACH3036609, TRACH3036638, TRACH3036683, TRACH3036750, TRACH3036792, TRACH3036843, TRACH3036897, TRACH3036932, TRACH3036942, TRACH3036997, TRACH3037063, TRACH3037067, TRACH3037267, TRACH3037288, TRACH3037505, TRACH3037573, TRACH3037696, TRACH3037897, TRACH3038086,
TRACH3038399, TSTOM1000135, TSTOM1000186, TSTOM2000139, TSTOM2000235, TSTOM2000315, TSTOM2000442, TSTOM2000553, TSTOM2000569, TSTOM2000588, TSTOM2001195, TSTOM2001274, TSTOM2001571, TSTOM2001996, TSTOM2002265, TSTOM2002505, TSTOM2002561, TSTOM2002611, TSTOM2002672, TSTOM2002682

### Homology search data

Homology search data for full-length nucleotide sequences and the deduced amino acid sequences are shown below.

Each piece of data is punctuated with a double slash mark (//) and shown in order: Sequence Name, Definition of hit data, P value, Length of sequence compared, Homology, and Accession Number of hit data. Sequences which did not show hit data in the homology search list only the Clone name.
3NB692004045// Sodium/bile acid cotransporter (Na(+)/bile acid cotransporter) (Na(+)/taurocholate transport protein) (Sodium/taurocholate cotransporting polypeptide).// 3.00E-50// 99aa// 35%
ADIPS2000069// Ig alpha-1 chain C region.// 0// 325aa// 92%
ADRGL2010315
ADRGL2010594
AHMSC1000138
ASTRO2008972// Tubuli n-tyrosine ligase (EC 6. 3. 2. 25) (TTL) . // 6. 00E-57// 108aa// 91%
ASTRO2015162// CTL2 gene [Homo sapiens]// 1.00E-124// 222aa// 87%
ASTRO2016114// Zinc finger protein 85 (Zinc finger protein HPF4) (HTF1).// 1.00E-141// 227aa// 70%
ASTRO3000154// Jumonji protein.// 7.00E-56// 105aa// 100%
BEAST2000981// g1-related zinc finger protein [Mus musculus]// 4.00E-55// 118aa// 45%
BLADE2000256// suppression of tumorigenicity 5 [Homo sapiens]// 1.00E-135// 255aa// 65%
BLADE2001031
BLADE2002310// SH3-domain binding protein 1 [Homo sapiens]// 0// 408aa// 85%
BLADE2002744
BLADE2004849
BLADE2006043
BLADE2007735
BLADE2007744
BLADE2007799// Hepatocellular carcinoma-associated antigen 66.// 0// 401aa// 97%
BLADE2008809
BRACE1000475// Delta3,5-delta2,4-dienoyl-CoA isomerase, mitochondrial precursor (EC 5.3.3.-).// 2.00E-74// 144aa// 93%
BRACE2002392// differential display and activated by p53: p53-regulated DDA3 [Mus musculus]// 6.00E-75// 143aa// 72%
BRACE2003628// transmembrane protein induced by tumor necrosis factor alpha [Homo sapiens]// 1.00E-131// 220aa// 65%
BRACE2005991
BRACE2010336
BRACE2012528// NDRG family, member 4: KIAA1180 protein [Homo sapiens]// 0// 342aa// 92%
BRACE2012625
BRACE2012833
BRACE2012838// Complexin 1 (Synaphin 2).// 1.00E-38// 86aa// 64%
BRACE2012936
BRACE2012947// Heat shock factor protein 1 (HSF 1) (Heat shock transcription factor 1) (HSTF 1 ) . // 3. OOE-26// 57aa// 96%
BRACE2013009// EH-domain containing protein 1 (Testilin) (hPAST1).// 1.00E-111// 204aa// 88%
BRACE2013126
BRACE2013132// Adenosine deaminase (EC 3.5.4.4) (Adenosine aminohydrolase).// 2.00E-11// 52aa// 23%
BRACE2016896// Lysyl-tRNA synthetase (EC 6.1.1.6) (Lysine-tRNA ligase) (LysRS).// 0// 337aa// 87%
BRACE2017359// Mus musculus suppressor of Ty 6 homolog (S. cerevisiae) (Supt6h)// 1.00E-82// 144aa// 100%
BRACE2017397// Cartilage matrix protein precursor (Matrilin-1).// 6.00E-36// 83aa// 40%
BRACE2017580// Glycerol-3-phosphate transporter (G-3-P transporter) (G-3-P permease).// 2.00E-15// 50aa// 40%
BRACE2017844
BRACE2017872// nuclear receptor-binding SET-domain protein 1 [Mus musculus]// 1.00E-154// 268aa// 85%
BRACE2017992
BRACE2019348// Zinc finger protein ZIC4 (Zinc finger protein of the cerebellum 4).// 4.00E-45// 82aa// 78%
BRACE2023633
BRACE2023744// Translationally controlled tumor protein (TCTP) (p23) (Histamine- releasing factor) (HRF).// 7.00E-62// 118aa// 88%
BRACE2025452
BRACE2026404
BRACE2027312
BRACE2027382// Opa-interacting protein OIP2 [Homo sapiens].// 1.00E-61// 101aa// 93%
BRACE2028956
BRACE2030039
BRACE2032584
BRACE2033128
BRACE2034434
BRACE2035120// phosphatidylinositol (4,5) bisphosphate 5-phosphatase, A [Homo sapiens].// 0// 582aa// 94%
BRACE2035191
BRACE2039362
BRACE2039607
BRACE2042541
BRACE2046976
BRACE2047232
BRACE2047975
BRACE3001403// Dipeptidyl peptidase IV like protein (Dipeptidyl aminopeptidase- related protein) (Dipeptidylpeptidase VI) (DPPX).// 1.00E-57// 107aa// 97%
BRACE3001973// Cadherin-related tumor suppressor homolog precursor (Fat protein homolog).// 1.00E-157// 344aa// 36%
BRACE3002264// Voltage-dependent L-type calcium channel alpha-1D subunit (Calcium channel, L type, alpha-1 polypeptide, isoform 2) (RAT brain class D) (RBD).// 5.00E-55// 193aa// 26%
BRACE3002344// Vegetatible incompatibility protein HET-E-1.// 7. 00E-19// 127aa// 24%
BRACE3002541
BRACE3002756// protein tyrosine phosphatase, receptor type, f polypeptide (PTPRF), interacting protein (liprin), alpha 2 [Homo sapiens]// 0// 427aa// 68%
BRACE3003866
BRACE3004046
BRACE3004371
BRACE3004767// Spectrin beta chain, brain (Spectrin, non-erythroid beta chain) (Fodrin beta chain).// 4.00E-43// 86aa// 52%
BRACE3004887
BRACE3004981
BRACE3005870
BRACE3005903
BRACE3006553
BRACE3007649
BRACE3007869
BRACE3009075
BRACE3009265
BRACE3009392// Neutral amino acid transporter A (SATT) (Alanine/serine/cysteine/ threonine transporter) (ASCT1).// 1.00E-143// 258aa// 88%
BRACE3009416// testis specific ankyrin-like protein 1 [Homo sapiens]// 1.00E-119// 209aa// 92%
BRACE3009539
BRACE3010702
BRACE3011447
BRACE3011774
BRACE3013418// ankyrin 1, isoform 2; ankyrin-1, erythrocytic: ankyrin-R [Homo sapiens]// 0// 969aa// 95%
BRACE3013874
BRACE3013986
BRACE3014523
BRACE3014714// 116 kDa U5 small nuclear ribonucleoprotein component (U5 snRNP- specific protein, 116 kDa) (U5-116 kDa).// 1.00E-120// 209aa// 99%
BRACE3015090
BRACE3015898
BRACE3016020// SBBI31 protein [Homo sapiens]// 7.00E-20// 47aa// 49%
BRACE3016167
BRACE3016580
BRACE3016788
BRACE3016810
BRACE3016862
BRACE3017253// LAO/AO transport system kinase (EC 2.7.-.-).// 2.00E-65// 130aa// 45%
BRACE3018083// junctophilin 3: junctophilin type3 [Homo sapiens]// 0// 331aa// 91%
BRACE3019570// SNAP-25-interacting protein [Rattus norvegicus]// 0// 785aa// 81%
BRACE3019611
BRACE3019817
BRACE3019941// Recessive polycystic kidney disease protein Tg737 homolog.// 0// 685aa// 99%
BRACE3020356
BRACE3020669// Eukaryotic translation initiation factor 3 subunit 9 (elF-3 eta) (elF3 p116) (elF3 p110) . // 5.00E-66// 118aa// 92%
BRACE3021430
BRACE3021517
BRACE3021805// sulfhydryl oxidase [Mus musculus]// 3.00E-51// 102aa// 47%
BRACE3022051
BRACE3022303// Pax transcription activation domain interacting protein [Mus musculus]// 1.00E-144// 246aa// 84%
BRACE3022312
BRACE3022340// SNAP-25-interacting protein [Rattus norvegicus]// 0// 745aa// 80%
BRACE3022847
BRACE3023604
BRACE3024379
BRACE3024444
BRACE3024497
BRACE3024537
BRACE3024879
BRACE3025627
BRACE3025719// ring finger protein 22, isoform alpha; brain expressed ring finger; tripartite motif protein TRIM3 [Homo sapiens]// 0// 430aa// 95%
BRACE3026161
BRACE3026290// Homo sapiens lethal giant larvae homolog 2 [Homo sapiens]// 0// 914aa// 95%
BRACE3026345// Insulin-like growth factor II precursor (IGF-II) (Somatomedin A).// 6.00E-87// 153aa// 85%
BRACE3026456
BRACE3026802// Homo sapiens tweety homolog 1 (Drosophila) (TTYH1), mRNA.// 2.00E-97// 190aa// 66%
BRACE3026844// Zinc finger protein 84 (Zinc finger protein HPF2).// 0// 315aa// 48%
BRACE3026947
BRACE3027256
BRACE3027931
BRACE3028360
BRACE3028895
BRACE3028998// D-beta-hydroxybutyrate dehydrogenase precursor (EC 1.1.1.30) (BDH) (3-hydroxybutyrate dehydrogenase).// 8.00E-30// 62aa// 100%
BRACE3029005
BRACE3029021
BRACE3029205
BRACE3029447
BRACE3030538// putative tumor suppressor [Homo sapiens]// 1.00E-35// 77aa// 45%
BRACE3031161
BRACE3031184
BRACE3031185
BRACE3031315// activated p21cdc42Hs kinase [Homo sapiens]// 0// 328aa// 73%
BRACE3031372
BRACE3031579
BRACE3031728
BRACE3031743// Homo sapiens bruno-like 4, RNA binding protein (Drosophila) (BRUNOL4) , mRNA. // 2. 00E-42/ / 91aa// 72%
BRACE3031843
BRACE3032385// Voltage-dependent L-type calcium channel alpha-1D subunit (Calcium channel, L type, alpha-1 polypeptide, isoform 2) (RAT brain class D) (RBD).// 2.00E-31// 124aa// 26%
BRACE3032537
BRACE3032538
BRACE3032631// F-box protein FBX13 [Mus musculus].// 0// 276aa// 94%
BRACE3032980
BRACE3033525
BRACE3034183// Zinc finger protein 84 (Zinc finger protein HPF2).// 1.00E-141// 216aa// 55%
BRACE3034389
BRACE3034964// Brain development-related molecule 1.// 1.00E-151// 259aa// 87%
BRACE3034993
BRACE3035168
BRACE3036156
BRACE3036271
BRACE3036283// Cyclin G-associated kinase (EC 2.7.1.-).// 1.00E-69// 138aa// 71%
BRACE3037612
BRACE3037637
BRACE3037803
BRACE3038012
BRACE3038030
BRACE3038570
BRACE3038760
BRACE3039288
BRACE3039358// integral membrane glycoprotein [Mus musculus]// 0// 581aa// 78%
BRACE3039378// Metabotropic glutamate receptor 4 precursor.// 0// 569aa// 96%
BRACE3039454
BRACE3040012
BRACE3040239// Deltex3 [Mus musculus]// 1.00E-158// 276aa// 79%
BRACE3040504
BRACE3040644// low density lipoprotein receptor-related protein 3 [Homo sapiens]// 0// 549aa// 89%
BRACE3040863
BRACE3041059// Ubiquitin carboxyl-terminal hydrolase 4 (EC 3.1.2.15) (Ubiquitin thiolesterase 4) (Ubiquitin-specific processing protease 4) (Deubiquitinating enzyme 4) (Ubiquitous nuclear protein homolog).// 6.00E-39// 82aa// 39%
BRACE3041162// zinc finger protein 14 (KOX 6); GI0T-4 for gonadotropin inducible transcription repressor-4 [Homo sapiens]// 1.00E-67// 110aa// 60%
BRACE3041827
BRACE3042046// Proto-oncogene DBL precursor [Contains: MCF2].// 9.00E-19// 190aa// 26%
BRACE3042210
BRACE3042326// Protein dpy-19.// 2.00E-59// 166aa// 28%
BRACE3042409// Putative fatty-acid-CoA ligase fadD26 (EC 6.2.1.-) (Acyl-CoA synthetase).// 5.00E-21// 135aa// 25%
BRACE3042432// Vasoactive intestinal polypeptide receptor 1 precursor (VIP-R-1) (Pituitary adenylate cyclase activating polypeptide type II receptor) (PACAP type II receptor) (PACAP-R-2).// 8.00E-67// 123aa// 98%
BRACE3042594
BRACE3043597// 60S ribosomal protein L26.// 3.00E-68// 128aa// 88%
BRACE3044090
BRACE3044172// FKBP-rapamycin associated protein (FRAP) (Rapamycin target protein).// 0// 709aa// 94%
BRACE3044247
BRACE3044377
BRACE3044495// GROS1-L protein [Homo sapiens]// 3.00E-55// 150aa// 44%
BRACE3045078
BRACE3045145
BRACE3045424
BRACE3045708
BRACE3045981
BRACE3046049
BRACE3046152// putative homeodomain transcription factor: putative homeodomain transcription factor 1 [Homo sapiens]// 0// 375aa// 93%
BRACE3046294
BRACE3046466// Crumbs protein homolog 1 precursor.// 1.00E-126// 284aa// 29%
BRACE3046491// Channel associated protein of synapse-110 (Chapsyn-110) (Synaptic density protein PSD-93) (Discs, large homolog 2).// 0// 686aa// 89% BRACE3046609// putative BTK-binding protein [Homo sapiens].// 1.00E-30// 195aa// 100%
BRACE3046837
BRACE3046855
BRACE3046966
BRACE3047018
BRACE3047482// tripartite motif-containing 9 [Homo sapiens]// 0// 448aa// 60%
BRACE3047801
BRACE3048483
BRACE3048565
BRACE3048615
BRACE3048677
BRACE3048756
BRACE3048904
BRACE3048905
BRACE3049186
BRACE3049714// NYD-TSPG protein [Homo sapiens]// 4.00E-47// 119aa// 32%
BRACE3050270
BRACE3050504
BRACE3051144
BRACE3051621// Polycystin precursor (Autosomal dominant polycystic kidney disease protein 1).// 0// 387aa// 83%
BRACE3051627
BRACE3051722
BRACE3051819// Myosin heavy chain, cardiac muscle alpha isoform (MyHC-alpha).// 0// 513aa// 55%
BRACE3051879
BRACE3052321// spectrin SH3 domain binding protein 1: eps8 binding protein: interactor protein AbIBP4: Abl-interactor protein 1 long [Homo sapiens]// 0// 388aa// 73%
BRACE3052410// IDN3 protein [Homo sapiens]// 0// 532aa// 94%
BRACE3052486
BRACE3052595// Nim2 [Rattus norvegicus]// 1.00E-80// 145aa// 62%
BRALZ2003119
BRALZ2007661
BRALZ2008930
BRALZ2010842// Mitochondrial carnitine/acylcarnitine carrier protein (Carnitine/acylcarnitine translocase) (CAC).// 4.00E-22// 73aa// 31%
BRALZ2011337
BRALZ2013621// Heterogeneous nuclear ribonucleoprotein K (hnRNP K) (DC-stretch binding protein) (CSBP) (Transformation upregulated nuclear protein) (TUNP).// 5.00E-37// 87aa// 65%
BRALZ2013690
BRALZ2014054// cenexin 2 [Rattus norvegicus].// 4.00E-50// 96aa// 87%
BRAMY2015516
BRAMY2021098
BRAMY2022320
BRAMY2023939
BRAMY2025495
BRAMY2031516
BRAMY2033895
BRAMY2035801
BRAMY2036254
BRAMY2036266
BRAMY2037609
BRAMY2039630
BRAMY2040915// LISCH protein [Homo sapiens]// 1.00E-121// 234aa// 68%
BRAMY2041347
BRAMY2041384// Annexin VI (Lipocortin VI) (P68) (P70) (Protein III) (Chromobindin 20) (67 kDa calelectrin) (Calphobindin-II) (CPB-II).// 0// 471aa// 96%
BRAMY2041507
BRAMY2044686
BRAMY2046489
BRAMY2046537// lipoma HMGIC fusion partner [Homo sapiens]// 5.00E-14// 53aa// 28%
BRAMY3000692// Zinc finger protein 91 (Zinc finger protein HTF10) (HPF7).// 3.00E-30// 56aa// 72%
BRAMY3001409
BRAMY3002329
BRAMY3002508
BRAMY3002886// ancient conserved domain protein 4 [Homo sapiens]// 1.00E-125// 252aa// 49%
BRAMY3004126// Putative G protein-coupled receptor GPR17 (R12).// 0// 322aa// 94%
BRAMY3004364
BRAMY3005184// ankyrin 3, epithelial [Mus musculus]// 0// 769aa// 82%
BRAMY3005656
BRAMY3005912
BRAMY3007078// Peregrin (Bromodomain and PHD finger-containing protein 1) (BR140 protein).// 5.00E-29// 62aa// 45%
BRAMY3007449
BRAMY3007471// gene trap locus F3b; transcript expressed during hematopoiesis 2 [Mus musculus]// 3.00E-56// 104aa// 92%
BRAMY3008436
BRAMY3009158
BRAMY3009491// Phosphatidylinositol 4-kinase alpha (EC 2.7.1.67) (P)4-kinase) (Ptdlns-4-kinase) (P14K-alpha).// 1.00E-129// 222aa// 97%
BRAMY3009556// TGFB1-induced anti-apoptotic factor 1 (12 kDa TGF-beta1-induced antiapoptotic factor).// 4.00E-56// 104aa// 90%
BRAMY3009904
BRAMY3010321// MRIP-1 protein [Homo sapiens]// 2.00E-18// 56aa// 31%
BRAMY3010603
BRAMY3010654
BRAMY3010902
BRAMY3011501// Heterogenous nuclear ribonucleoprotein U (hnRNP U) (Scaffold attachment factor A) (SAF-A).// 0// 469aa// 79%
BRAMY3011581// DNA topoisomerase III alpha (EC 5.99.1.2).// 0// 410aa// 90%
BRAMY3011865
BRAMY3014027// Zinc finger protein 44 (Zinc finger protein KOX7) (Gonadotropin inducible transcription repressor-2) (GI0T-2).// 0// 339aa// 56%
BRAMY3014555
BRAMY3014613// SH3-domain binding protein 1 [Homo sapiens]// 0// 420aa// 86%
BRAMY3015086// E4L1_HUMAN Band 4.1-like protein 1.// 0// 477aa// 94%
BRAMY3015547// Rho guanine nucleotide exchange factor 10 [Homo sapiens]// 0// 461aa// 44%
BRAMY3015549// Homo sapiens cell adhesion molecule with homology to L1CAM (close homolog of L1) (CHL1)// 0// 1076aa// 95%
BRAMY3016829
BRAMY3017827
BRAMY3017920// Active breakpoint cluster region-related protein.// 1.00E-161// 279aa// 98%
BRAMY3017965
BRAMY3018121
BRAMY3018248// ATP-binding cassette, sub-family B, member 8, mitochondrial precursor (Mitochondrial ATP-binding cassette 1) (M-ABC1).// 3.00E-27// 57aa// 100%
BRAMY3018340
BRAMY3018754// junction-mediating and regulatory protein: p300 transcriptional cofactor JMY [Mus musculus]// 7.00E-72// 160aa// 35%
BRAMY4000915// Ankyrin 1 (Erythrocyte ankyrin).// 2.00E-68// 190aa// 27%
BRAMY4000962// putative RNA binding protein [Homo sapiens]// 2.00E-16// 65aa// 29%
BRAMY4001234
BRAMY4001652// Ankyrin 1 (Erythrocyte ankyrin).// 2.00E-58// 197aa// 29%
BRAMY4001863// Mus musculus enabled homolog (Drosophila) (Enah), mRNA// 1.00E-84// 161aa// 81%
BRAMY4001913
BRAMY4002575
BRAMY4002628
BRAWH2000256// prestin [Rattus norvegicus]// 1.00E-121// 223aa// 93%
BRAWH2002333// Calcium/calmodulin-dependent 3',5'-cyclic nucleotide phosphod i esterase 1 B (EC 3. 1. 4. 17) (Cam-PDE 1B) (63 kDa Cam-PDE) . // 1.00E-175// 302aa// 90%
BRAWH2004078
BRAWH2010364
BRAWH2010619
BRAWH2011796// S-100 protein, alpha chain.// 6.00E-30// 65aa// 69%
BRAWH2011812// VPS10 domain receptor protein SORCS [Mus musculus]// 0// 966aa// 95%
BRAWH2011958
BRAWH2012054
BRAWH2012866// Synaptotagmin I (Sytl) (p65).// 0// 335aa// 79%
BRAWH2013955
BRAWH2014053// AFG3-like protein 2 (EC 3.4.24.-) (Paraplegin-like protein).// 4.00E-18// 42aa// 89%
BRAWH2016209// autocrine motility factor receptor [Mus musculus]// 2.00E-28// 89aa// 28%
BRAWH2016223
BRAWH2016305
BRAWH2016514
BRAWH2016562
BRAWH2016785
BRAWH3000446// Kruppel-like zinc finger protein GLIS2 [Homo sapiens]// 0// 300aa// 84%
BRAWH3000884
BRAWH3001053
BRAWH3001638// SUMO-1-specific protease 1 (EC 3.4.22.-) (Sentrin-specific protease SENP6) (Protease FKSG6).// 0// 1009aa// 90%
BRAWH3001783// protocadherin 15 [Homo sapiens]// 0// 688aa// 92%
BRAWH3001833
BRAWH3003244
BRAWH3003573// 45 kDa calcium-binding protein precursor (Stroma cell-derived factor 4) (SDF-4).// 9.00E-69// 121aa// 93%
BRAWH3003975
BRAWH3004335// heparan sulfate D-glucosaminyl 3-0-sulfotransferase 1 precursor; heparin-glucosamine 3-0-sulfotransferase [Homo sapiens]// 5.00E-72// 118aa// 49%
BRAWH3004350// Spindlin (Ovarian cancer-related protein).// 3.00E-96// 170aa// 77%
BRAWH3005037
BRAWH3005886// Zinc-finger protein cer-d4.// 1.00E-160// 273aa// 78%
BRAWH3005892
BRAWH3005896
BRAWH3008167// CUB and Sushi multiple domains 1 [Homo sapiens]// 0// 727aa// 96%
BRAWH3008559
BRAWH3008867
BRAWH3009961// Nim2 [Rattus norvegicus]// 2.00E-95// 174aa// 69%
BRAWH3010461// secretory carrier membrane protein 5 [Mus musculus]// 1.00E-107// 186aa// 79%
BRAWH3010602// Gamma-soluble NSF attachment protein (SNAP-gamma) (N-ethylmaleimide- sensitive factor attachment protein, gamma).// 1.00E-113// 203aa// 90%
BRAWH3010657
BRAWH3010726// phosphatidylinositol transfer protein, membrane-associated: Drosophila retinal degeneration B [Homo sapiens]// 0// 509aa// 96%
BRAWH3010833
BRAWH3011101
BRAWH3011331// heparan sulfate 6-0-sulfotransferase 3 [Mus musculus]// 1.00E-130// 219aa// 87%
BRAWH3011402
BRAWH3011577// Zinc finger protein 175 (Zinc finger protein OTK18).// 1.00E-30// 65aa// 51%
BRAWH3011623// Heterogeneous nuclear ribonucleoproteins C1/C2 (hnRNP C1 / hnRNP C2).// 2.00E-97// 192aa// 74%
BRAWH3011685
BRAWH3011907
BRAWH3011929
BRAWH3012005
BRAWH3012662// Mus musculus tweety homolog 1 (Drosophila) (Ttyh1), mRNA.// 3.00E-15// 42aa// 37%
BRAWH3012779
BRAWH3013009// zinc finger protein 35 [Mus musculus]// 1.00E-136// 280aa// 49%
BRAWH3013049
BRAWH3013264// SWI/SNF related, matrix associated, actin dependent regulator of chromatin, subfamily a, member 3 [Mus musculus]// 3.00E-20// 75aa// 27%
BRAWH3013508
BRAWH3014609// Insulin-like growth factor binding protein complex acid labile chain precursor (ALS).// 1.00E-25// 79aa// 30%
BRAWH3014639
BRAWH3015017// axonemal dyne in light chain p33.// 3.00E-22// 34aa// 84%
BRAWH3015175// Myosin heavy chain, muscle.// 6.00E-08// 68aa// 21%
BRAWH3015610
BRAWH3015825
BRAWH3016123
BRAWH3016715
BRAWH3017180// Diacylglycerol kinase, zeta (EC 2.7.1.107) (Diglyceride kinase) (DGK- zeta) (DAG kinase zeta).// 0// 837aa// 90%
BRAWH3017259// protein tyrosine phosphatase, receptor type, f polypeptide (PTPRF), interacting protein (liprin), alpha 2 [Homo sapiens]// 0// 1068aa// 86%
BRAWH3017260
BRAWH3017477// AE-binding protein 2 [Mus musculus]// 1.00E-162// 273aa// 92%
BRAWH3017980
BRAWH3018063// tulip 1 [Rattus norvegicus]// 0// 444aa// 86%
BRAWH3018369
BRAWH3018548// Vinculin (Metavinculin).// 0// 877aa// 90%
BRAWH3018969
BRAWH3019026// Echinoderm microtubule-associated protein-like 1 (EMAP-1) (HuEMAP-1).// 0// 612aa// 95%
BRAWH3019529
BRAWH3019594
BRAWH3019820
BRAWH3020200
BRAWH3020318
BRAWH3020884
BRAWH3020928// mitogen-activated protein kinase phosphatase x [Homo sapiens]// 2.00E-35// 66aa// 100%
BRAWH3021012// AP1 gamma subunit binding protein 1; gamma-synergin [Homo sapiens]// 1.00E-172// 302aa// 92%
BRAWH3021574
BRAWH3021580// Restin (Cytoplasmic linker protein-170 alpha-2) (CLIP-170) (Reed- Sternberg intermediate filament associated protein).// 0// 340aa// 72%
BRAWH3021641
BRAWH3021643
BRAWH3021724// kyphoscoliosis [Mus musculus]// 1.00E-136// 248aa// 68%
BRAWH3022347
BRAWH3022431
BRAWH3022459
BRAWH3022542
BRAWH3022651// ubiquitin-protein ligase e3 componen n-recognin [Mus musculus]// 1.00E-24// 82aa// 30%
BRAWH3022719
BRAWH3022900
BRAWH3023156// Gamma-aminobutyric-acid receptor beta-1 subunit precursor (GABA(A) receptor).// 4.00E-62// 115aa// 100%
BRAWH3023168
BRAWH3023172// Mothers against decapentaplegic homolog interacting protein (Madh-interacting protein) (Smad anchor for receptor activation) (Receptor activation anchor) (hSARA) (Novel serine protease) (NSP).//1.00E-109// 198aa// 56%
BRAWH3023274
BRAWH3023415// alpha 1,2-mannosidase [Homo sapiens]// 5.00E-57// 105aa// 100%
BRAWH3023421// Protein-arginine deiminase type II (EC 3.5.3.15) (Peptidylarginine deiminase II) (PAD-H19).// 1.00E-106// 180aa// 99%
BRAWH3024186// LAR protein precursor (Leukocyte antigen related) (EC 3.1.3.48).// 0// 862aa// 90%
BRAWH3024231// Tetratricopeptide repeat protein 4.// 1.00E-151// 261aa// 94%
BRAWH3024242
BRAWH3024506// Probable leucyl-tRNA synthetase (EG 6.1.1.4) (Leucine-tRNA ligase) (LeuRS).// 2.00E-61// 128aa// 38%
BRAWH3024989
BRAWH3025157
BRAWH3026349// Sorting nexin 7.// 2.00E-94// 160aa// 49%
BRAWH3026938// semaF cytoplasmic domain associated protein 3: semaphorin cytoplasmic domain-associated protein 3A [Mus musculus]// 0// 354aa// 45%
BRAWH3027420
BRAWH3027440// Probable kinesin light chain 3 (KLC 3).// 1.00E-159// 283aa// 85%
BRAWH3027533// rap2 interacting protein x [Homo sapiens].// 1.00E-117// 390aa// 64%
BRAWH3027574// vascular Rab-GAP/TBC-containing [Homo sapiens]// 0// 427aa// 45%
BRAWH3027607
BRAWH3027616
BRAWH3027675
BRAWH3027806// Echinoderm microtubule-associated protein-like 2 (EMAP-2) (HuEMAP-2).// 2.00E-92// 211aa// 34%
BRAWH3027880
BRAWH3028202
BRAWH3028223
BRAWH3028461
BRAWH3028754
BRAWH3028796// makorin, ring finger protein, 1 [Homo sapiens]// 0// 362aa// 86%
BRAWH3029313
BRAWH3029385// Dynamin 2 (EC 3.6.1.50).// 1.00E-131// 237aa// 88%
BRAWH3029538// myelin-associated oligodendrocyte basic protein [Homo sapiens]// 5.00E-39// 184aa// 99%
BRAWH3029806// Adenylate cyclase, type II (EC 4.6.1.1) (ATP pyrophosphate-lyase) (Adenylyl cyclase).// 0// 758aa// 90%
BRAWH3030772
BRAWH3030810
BRAWH3030910// Sec23-interacting protein p125 [Homo sapiens]// 5.00E-92// 180aa// 51%
BRAWH3031054
BRAWH3031342// Zinc finger protein 91 (Zinc finger protein HTF10) (HPF7).// 0// 301aa// 59%
BRAWH3031710// serologically defined colon cancer antigen 33 [Homo sapiens]// 0// 653aa// 96%
BRAWH3032298// Homo sapiens tenascin R (restrictin, janusin) (TNR), mRNA// 0// 472aa// 36%
BRAWH3032340// Rho-specific guanine nucleotide exchange factor p114 [Homo sapiens]// 0// 824aa// 81%
BRAWH3032571// Chromodomain helicase-DNA-binding protein 4 (CHD-4) (Mi-2 autoantigen 218 kDa protein) (Mi2-beta).// 0// 714aa// 69%
BRAWH3033117
BRAWH3033293// synaptopodin [Homo sapiens]// 0// 580aa// 85%
BRAWH3033448
BRAWH3033513// 3-ketoacyl-CoA thiolase, peroxisomal precursor (EC 2.3.1.16) (Beta- ketothiolase) (Acetyl-CoA acyltransferase) (Peroxisomal 3-oxoacyl- CoA thiolase).// 1.00E-60// 114aa// 87%
BRAWH3034097
BRAWH3034114
BRAWH3034134
BRAWH3034668// Ubiquitin carboxyl-terminal hydrolase 4 (EC 3.1.2.15) (Ubiquitin thiolesterase 4) (Ubiquitin-specific processing protease 4) (Deubiquitinating enzyme 4) (Ubiquitous nuclear protein homolog).// 0// 624aa// 90%
BRAWH3034743
BRAWH3034775// E1B-55kDa-associated protein 5 [Homo sapiens]// 1.00E-164// 283aa// 93%
BRAWH3034890// CAGH32 [Homo sapiens].// 9.00E-29// 131aa// 94%
BRAWH3035403
BRAWH3035904
BRAWH3035914
BRAWH3035936// Zinc finger protein 208.// 0// 354aa// 51%
BRAWH3036077
BRAWH3036247// Zinc finger protein 228.// 2.00E-54// 96aa// 39%
BRAWH3036270// Hb2E [Homo sapiens].// 1.00E-109// 233aa// 97%
BRAWH3036334// Zinc finger protein 91 (Zinc finger protein HTF10) (HPF7).// 0// 436aa// 60%
BRAWH3036561
BRAWH3037265
BRAWH3037394
BRAWH3037428// RAB37, member of RAS oncogene family: GTPase Rab37 [Mus musculus]// 1.00E-76// 140aa// 88%
BRAWH3037533
BRAWH3037979// Ubiquitin carboxyl-terminal hydrolase 24 (EG 3.1.2.15) (Ubiquitin thiolesterase 24) (Ubiquitin-specific processing protease 24) (Deubiquitinating enzyme 24) (Fragment).// 0// 415aa// 97%
BRAWH3038055// synaptic nuclei expressed gene 2 [Homo sapiens]// 0// 551aa// 47%
BRAWH3038230
BRAWH3038252// Formin 1 isoform IV (Limb deformity protein).// 0// 411aa// 89%
BRAWH3038324// Reticulon protein 3 (Neuroendocrine-specific protein-like 2) (NSP-like protein II) (NSPLII).// 1.00E-101// 189aa// 99%
BRAWH3038827// leucine-zipper-like transcriptional regulator, 1; Leucine-zipper-like regulator-1 [Homo sapiens]// 0// 530aa// 96%
BRAWH3039258
BRAWH3039623
BRAWH3040297
BRAWH3040695// Inner nuclear membrane protein Manl.// 1.00E-33// 66aa// 47%
BRAWH3040711
BRAWH3040900// Homo sapiens cadherin, EGF LAG seven-pass G-type receptor 2 (flamingo homolog, Drosophila) (CELSR2) mRNA// 1.00E-49// 97aa// 82%
BRAWH3041492// erythrocyte membrane protein band 4.1 (elliptocytosis 1, RH-linked); Erythrocyte surface protein band 4.1 [Homo sapiens]// 1.00E-66// 129aa// 64%
BRAWH3041556// C-1-tetrahydrofolate synthase, cytoplasmic (C1-THF synthase) [Includes: Methylenetetrahydrofolate dehydrogenase (EC 1.5.1.5);
Methenyltetrahydrofolate cyclohydrolase (EC 3.5.4.9); Formyltetrahydrofolate synthetase (EC 6.3.4.3)].// 0// 536aa// 58%
BRAWH3041928
BRAWH3042132
BRAWH3042438// Diacylglycerol kinase, alpha (EC 2.7.1.107) (Diglyceride kinase) (DGK- alpha) (DAG kinase alpha) (80 kDa diacylglycerol kinase).// 5.00E-56// 103aa// 100%
BRAWH3042447// p53 inducible protein [Homo sapiens].// 0// 694aa// 86%
BRAWH3042568// ventral anterior homeobox containing gene 1 [Mus musculus]// 5.00E-76// 137aa// 95%
BRAWH3042772// Zinc transporter 3 (ZnT-3).// 3.00E-71// 135aa// 87%
BRAWH3042787
BRAWH3042820
BRAWH3042996
BRAWH3043034// Mus musculus neuregulin 1 (Nrgl)// 0// 924aa// 92%
BRAWH3043295// Inorganic pyrophosphatase (EC 3.6.1.1) (Pyrophosphate phosphohydrolase) (PPase).// 7.00E-53// 95aa// 98%
BRAWH3043498
BRAWH3043623// Cadherin-related tumor suppressor homolog precursor (Fat protein homolog).// 8.00E-24// 74aa// 36%
BRAWH3043944
BRAWH3044122// Munc13-1 [Rattus norvegicus]// 0// 888aa// 98%
BRAWH3044151// ADAM-TS 9 precursor (EC 3.4.24.-) (A disintegrin and metalloproteinase with thrombospondin motifs 9) (ADAMTS-9) (ADAM-TS9).// 7.00E-45// 118aa// 31%
BRAWH3044487
BRAWH3044585
BRAWH3044676
BRAWH3044985
BRAWH3045118
BRAWH3045229
BRAWH3045625
BRAWH3046196
BRAWH3046209
BRAWH3046424// Histone deacetylase 6 (HD6).// 0// 530aa// 93%
BRAWH3046802
BRAWH3046959
BRAWH3047063
BRAWH3047539
BRAWH3047565// TOLLIP protein [Homo sapiens]// 1.00E-101// 177aa// 86%
BRAWH3047644// Rho guanine nucleotide exchange factor 4, isoform b: APC-stimulated guanine nucleotide exchange factor [Homo sapiens]// 3.00E-84// 142aa// 100%
BRAWH3047692// Lon protease homolog 1, mitochondrial precursor (EC 3.4.21.-).// 1.00E-164// 297aa// 51%
BRAWH3047946
BRAWH3048374// CUB and Sushi multiple domains 1 [Homo sapiens]// 0// 715aa// 96%
BRAWH3048548
BRAWH3048724
BRAWH3049068
BRAWH3049544// Polypeptide N-acetylgalactosaminyltransferase (EC 2.4.1.41) (Protein- UDP acetylgalactosaminyltransferase) (UDP-GaINAc:polypeptide, N-acetylgalactosaminyltransferase) (GaINAc-T1).// 1.00E-119// 230aa// 43%
BRAWH3049726
BRAWH3049858
BRCAN2000923// Putative ADP-ribosylation factor 2.// 2.00E-29// 180aa// 32%
BRCAN2002662
BRCAN2002892// Ras-related protein Rab-7.// 7.00E-47// 96aa// 50%
BRCAN2003269// Multidrug resistance protein 3 (P-glycoprotein 3).// 4.00E-93// 171aa// 55%
BRCAN2003814// Variant-surface-glycoprotein phospholipase C (EG 3.1.4.47) (VSG lipase) (Glycosylphosphatidylinositol-specific phospholipase C) (GPI-PLC).// 3.00E-18// 78aa// 26%
BRCAN2006051// AFG3-like protein 2 (EG 3.4.24.-) (Paraplegin-like protein).// 3.00E-50// 97aa// 66%
BRCAN2006955
BRCAN2007525
BRCAN2008701
BRCAN2009168
BRCAN2010547
BRCAN2010581
BRCAN2010665// Channel associated protein of synapse-110 (Chapsyn-110) (Discs, large homolog 2).// 0// 422aa// 96%
BRCAN2015402// Cytochrome P450 2G1 (EC 1.14.14.1) (CYPIIG1) (P450-NMB) (Olfactive).// 3.00E-73// 129aa// 88%
BRCAN2015757
BRCAN2018269
BRCAN2018667
BRCAN2019653
BRCAN2019907// vascular Rab-GAP/TBC-containing [Homo sapiens]// 8.00E-18// 44aa// 57%
BRCAN2019953
BRCAN2020234// Quiescence-specific protein precursor (P20K) (CH21 protein).// 2.00E-06// 70aa// 27%
BRCAN2020331// G-rich sequence factor-1 (GRSF-1).// 0// 360aa// 80%
BRCAN2020412// phosphatidylinositol glycan, class S [Homo sapiens]// 6.00E-89// 195aa// 99%
BRCAN2020467// Nuclear movement protein NUDC.// 5.00E-12// 130aa// 32%
BRCAN2020880
BRCAN2020972
BRCAN2021325// Carboxypeptidase H precursor (EC 3.4.17.10) (CPH) (Carboxypeptidase E) (CPE) (Enkephalin convertase) (Prohormone processing carboxypeptidase).// 0// 389aa// 100%
BRCAN2021452
BRCAN2021718
BRCAN2022126// glutamate receptor delta-1 subunit [Rattus norvegicus]// 0// 949aa// 94%
BRCAN2025093
BRCAN2027593
BRCAN2028702
BRCOC2001355
BRCOC2002777
BRCOC2006164
BRCOC2006639
BRCOC2006942
BRCOC2009638
BRCOC2010115
BRCOC2012386// Zinc finger protein 184.// 2.00E-85// 166aa// 42%
BRHIP2006819
BRHIP2006921
BRHIP2008756// Homo sapiens pescadillo homolog 1, containing BRCT domain (zebrafish) (PES1), mRNA// 0// 352aa// 78%
BRHIP2009177
BRHIP2011199
BRHIP2013958
BRHIP2015153
BRHIP2016125
BRHIP2017714
BRHIP2020930
BRHIP2021929
BRHIP2023735// Zinc-finger protein neuro-d4.// 3.00E-46// 87aa// 87%
BRHIP2024941
BRHIP2026346// lymphocyte specific formin related protein: formin-related gene in leukocytes [Mus musculus]// 1.00E-114// 216aa// 47%
BRHIP2027077// FIt3 interacting zinc finger protein 1 [Mus musculus]// 4.00E-14// 54aa// 56%
BRHIP2027563// host cell factor homolog [Homo sapiens]// 2.00E-95// 166aa// 47%
BRHIP2029529// TFIIH basal transcription factor complex p62 subunit// 0// 414aa// 95%
BRHIP2029643
BRHIP2029663
BRHIP3000626
BRHIP3000859
BRHIP3001076// murine retrovirus integration site 1 homolog: inositol 1,4,5-triphosphate-associated cGMP kinase substrate; JAW1-related protein [Homo sapiens]// 0// 482aa// 80%
BRHIP3001141
BRHIP3001338
BRHIP3001360
BRHIP3001481// Protein-tyrosine phosphatase, non-receptor type 7 (EC 3.1.3.48) (Protein-tyrosine phosphatase LC-PTP) (Hematopoietic protein-tyrosine phosphatase) (HEPTP).// 0// 359aa// 99%
BRHIP3001573
BRHIP3001878// peptide transporter 3 [Homo sapiens]// 7.00E-74// 131aa// 86%
BRHIP3002000
BRHIP3002114// rTS beta protein [Homo sapiens]// 1.00E-148// 248aa// 94%
BRHIP3002124
BRHIP3002141
BRHIP3002363
BRHIP3002691
BRHIP3002920
BRHIP3002931
BRHIP3003063// Alpha-2 catenin (Alpha N-catenin) (Neural alpha-catenin).// 0// 867aa// 95%
BRHIP3003126// Homo sapiens ubiquitin-protein isopeptide ligase (E3) (KIAA0010),mRNA.// 2.00E-88// 195aa// 37%
BRHIP3003306// tulip 1 [Rattus norvegicus]// 1.00E-148// 272aa// 49%
BRHIP3003340// Actin, alpha skeletal muscle 2.// 3.00E-30// 63aa// 96%
BRHIP3003395
BRHIP3003688
BRHIP3003795// cytochrome P450 retinoid metabolizing protein [Homo sapiens]// 1.00E-28// 97aa// 51%
BRHIP3003845
BRHIP3003961
BRHIP3003984// IkappaB kinase complex-associated protein (IKK complex-associated protein) (p150).// 0// 930aa// 94%
BRHIP3004215// 80 kda MCM3-associated protein (GANP protein).// 0// 792aa// 87%
BRHIP3004710
BRHIP3004725// DNA-binding protein SATB1 (Special AT-rich sequence binding protein 1).// 0// 711aa// 93%
BRHIP3004774
BRHIP3004786
BRHIP3005037// Metastasis-associated protein MTA1.// 0// 339aa// 66%
BRHIP3005142
BRHIP3005231
BRHIP3005307// neuropathy target esterase [Homo sapiens]// 1.00E-176// 308aa// 63%
BRHIP3005673// Glutathione S-transferase Mu 5 (EC 2.5.1.18) (GSTM5-5) (GST class-Mu 5).// 5.00E-58// 111aa// 82%
BRHIP3005801// VAMP (vesicle-associated membrane protein)-associated protein B and C: VAMP-associated protein C: VAMP-associated protein B: VAMP-associated 33 kDa protein [Homo sapiens]// 4.00E-63// 118aa// 99%
BRHIP3005944// Antigen WC1.1 precursor.// 1.00E-50// 100aa// 39%
BRHIP3006279
BRHIP3006294// Cdc42 GTPase-activating protein [Mus musculus]// 5.00E-77// 144aa// 60%
BRHIP3006449// catenin (cadherin-associated protein), delta 2 (neural plakophilin-related arm-repeat protein); catenin (cadherin-associated protein), delta 2 [Homo sapiens]// 0// 756aa// 95%
BRHIP3006786// peptidylprolyl isomerase (cyclophilin)-like 2; cyclophilin-like protein CyP-60 [Homo sapiens]// 1.00E-123// 210aa// 99%
BRHIP3006950
BRHIP3007172
BRHIP3007195// Potential phospholipid-transporting ATPase IB (EC 3.6.3.13).// 0// 1049aa// 91%
BRHIP3007223// Ubiquitin carboxyl-terminal hydrolase 64E (EC 3.1.2.15) (Ubiquitin thiolesterase 64E) (Ubiquitin-specific processing protease 64E) (Deubiquitinating enzyme 64E).// 1.00E-139// 272aa// 47%
BRHIP3007291
BRHIP3007409
BRHIP3007424// WNT-14 protein precursor.// 4.00E-83// 143aa// 58%
BRHIP3007609// Homo sapiens Smcy homolog, Y chromosome (mouse) (SMCY)// 0// 571aa// 83%
BRHIP3007960
BRHIP3008082
BRHIP3008320// Rattus norvegicus potassium channel subunit (Slack) (Slack)// 0// 1063aa// 90%
BRHIP3008714
BRHIP3009672
BRHIP3009753
BRHIP3010289// Attractin precursor (Mahogany homolog) (DPPT-L).// 0// 738aa// 58%
BRHIP3010916
BRHIP3011082// Homo sapiens ubiquitin-protein isopeptide ligase (E3) (KIAA0010)// 0// 415aa// 99%
BRH I P3011269// Prenylcysteine lyase precursor (EC 4. 4.1. 18) . // 7.00E-80// 154aa// 39%
BRHIP3011460// Rho guanine exchange factor 16: putative neuroblastoma protein [Homo sapiens]// 1.00E-120// 227aa// 52%
BRHIP3011567// Dihydropyridine-sensitive L-type, channel beta-1-B2 subunit (Beta-1 isoform A).// 1.00E-173// 310aa// 83%
BRHIP3011831
BRHIP3012185
BRHIP3012289
BRHIP3012357// cerebral cell adhesion molecule [Homo sapiens]// 2.00E-61// 105aa// 55%
BRHIP3012736
BRHIP3012997
BRHIP3013078// BAI1-associated protein 2, isoform 2 [Homo sapiens]// 2.00E-40// 77aa// 100%
BRHIP3013588
BRHIP3013698
BRHIP3014675
BRHIP3015854
BRHIP3016032
BRHIP3016421
BRHIP3017109// Socs-5 [Mus musculus]// 1.00E-69// 118aa// 84%
BRHIP3017146// solute carrier family 30 (zinc transporter), member 3// 6.00E-96// 92aa// 97%
BRHIP3017256
BRHIP3017558// Monocarboxylate transporter 3 (MCT 3) (Retinal epithelial membrane protein).// 2.00E-60// 137aa// 30%
BRHIP3017855// nuclear pore complex interacting protein [Homo sapiens]// 4.00E-58// 107aa// 82%
BRHIP3018784
BRHIP3019643// Homo sapiens gamma tubulin ring complex protein (76p gene) (76P), mRNA// 1.00E-104// 187aa// 93%
BRHIP3019824
BRHIP3019880
BRHIP3019956
BRHIP3020046// ubiquitin-protein ligase e3 componen n-recognin [Mus musculus]// 3.00E-96// 167aa// 47%
BRHIP3020155
BRHIP3020733
BRHIP3021019// Protein-tyrosine phosphatase, non-receptor type 5 (EC 3.1.3.48) (Protein-tyrosine phosphatase striatum-enriched) (STEP) (Neural-specific protein-tyrosine phosphatase) (Fragment).// 0// 440aa// 81%
BRHIP3021499
BRHIP3021987
BRHIP3022656
BRHIP3023922// 5-methyltetrahydrofolate-homocysteine methyltransferase (EC 2.1.1.13) (Methionine synthase, vitamin-B12 dependent) (MS).// 4.00E-72// 134aa// 95%
BRHIP3024703
BRHIP3024820
BRHIP3025795// Monocarboxylate transporter 4 (MCT 4) (MCT 3).// 0// 392aa// 84%
BRHIP3025844// germ cell-specific gene 1 [Mus musculus]// 9.00E-14// 43aa// 47%
BRHIP3026231
BRHIP3026651
BRHIP3027160
BRHIP3027191// 150 kDa oxygen-regulated protein precursor (Orp150).// 0// 904aa// 90%
BRHIP3027651// Bromodomain-containing protein 1 (BR140-like protein).// 2.00E-59// 149aa// 31%
BRHIP3027947
BRHIP3028246// PHD finger protein 2 [Mus musculus]// 2.00E-67// 125aa// 42%
BRHIP3028570
BRHIP3028742// vanilloid receptor subtype 1 [Homo sapiens]// 1.00E-55// 108aa// 50%
BRHIP3029409// secreted frizzled-related protein 1; secreted apoptosis-related protein 2 [Homo sapiens]// 2.00E-43// 87aa// 65%
BRHIP3029530
BRHIP3029670
BRHIP3029866
BRHIP3030230// Neuronal pentraxin I precursor (NP-I) (NP1).// 0// 386aa// 93%
BRHIP3031733
BRHIP3031890
BRHIP3032148// brain-enriched guanylate kinase-associated [Rattus norvegicus]// 1.00E-16// 49aa// 40%
BRHIP3032311
BRHIP3032374// neuropathy target esterase [Homo sapiens]// 0// 369aa// 62%
BRHIP3033481
BRHIP3033557// N-methyl-D-aspartate receptor splice variant NR3A-2// 0// 852aa// 93%
BRHIP3033734
BRHIP3033806
BRHIP3035006// Synthase [Homo sapiens]// 1.00E-161// 276aa// 95%
BRHIP3035222
BRHIP3035754
BRHIP3036371
BRHIP3036715// HLA class I histocompatibility antigen, alpha chain H precursor (HLA-AR) (HLA-12.4).// 2.00E-60// 108aa// 67%
BRHIP3036936// astrotactin 2 [Mus musculus]// 0// 858aa// 96%
BRHIP3037543// cyclin G associated kinase [Homo sapiens]// 4.00E-40// 84aa// 78%
BRHIP3037810
BRHIP3038030
BRHIP3038735
BRHIP3039430
BRHIP3039509// Amiloride-sensitive sodium channel delta-subunit (Epithelial Na+ channel delta subunit) (Delta ENaC) (Nonvoltage-gated sodium channel 1 delta subunit) (SCNED) (Delta NaCH).// 0// 588aa// 92%
BRHIP3039592
BRHIP3040878
BRHIP3041587// Myosin light chain kinase, skeletal muscle (EC 2.7.1.117) (MLCK).// 2.00E-24// 76aa// 61%
BRHIP3042817
BRHIP3043012
BRSSN2004303
BRSSN2004710// cAMP-dependent 3' , 5' -cyclic phosphodiesterase 4B (EC 3. 1. 4. 17) (DPDE4) (PDE32).// 1.00E-59// 108aa// 88%
BRSSN2008464
BRSSN2011843
BRSSN2012157
BRSSN2012198
BRSSN2013696
BRSSN2015497// putative RNA binding protein [Homo sapiens]// 4.00E-16// 65aa// 29%
BRSSN2018218
BRSTN2000312
BRSTN2006466// Glutamate decarboxylase, 65 kDa isoform (EC 4.1.1.15) (GAD-65) (65 kDa glutamic acid decarboxylase).// 2.00E-77// 140aa// 80%
BRSTN2006638// synaptotagmin interacting protein 1 [Rattus norvegicus]// 2.00E-74// 147aa// 45%
BRSTN2008475// growth arrest-specific 11: growth arrest specific 11 [Homo sapiens]// 6.00E-36// 71aa// 98%
BRSTN2009247
BRSTN2010089// tumor differentially expressed 1, like: membrane protein TMS-2 [Mus musculus]// 0// 413aa// 91%
BRSTN2010416
BRSTN2011688
BRSTN2011899
BRSTN2011961// Protein disulfide isomerase precursor (PDI) (EC 5.3.4.1) (Prolyl 4- hydroxylase beta subunit) (Cellular thyroid hormone binding protein) (P55).// 3.00E-90// 160aa// 86%.
BRSTN2012069// Elongation factor 1-alpha 1 (EF-1-alpha-1) (Elongation factor 1 A-1) (eEF1 A-1) (Elongation factor Tu) (EF-Tu). // 0// 441aa// 95%
BRSTN2015699
BRSTN2015788
BRSTN2016892// BUP protein [Homo sapiens]// 1.00E-56// 105aa// 100%
BRSTN2016918// Glial fibrillary acidic protein, astrocyte (GFAP).// 0// 367aa// 86%
BRSTN2016992// DRR1 protein (TU3A protein).// 4.00E-62// 118aa// 81%
BRSTN2017104
BRSTN2017151// COP9 (constitutive photomorphogenic), subunit 7a (Arabidopsis); COP9 complex S7a [Mus musculus]// 7.00E-24// 54aa// 98%
BRSTN2017184
BRSTN2018712
BRTHA2000969
BRTHA2001304
BRTHA2001953
BRTHA2002091
BRTHA2003759
BRTHA2005448// calpain 12 [Mus musculus]// 0// 300aa// 87%
BRTHA2006720
BRTHA2008502
BRTHA2008598
BRTHA2010672// neutral protease large subunit [Homo sapiens].// 3.00E-39// 117aa// 71%
BRTHA2012189
BRTHA2014647
BRTHA2018304
BRTHA2019726// Ig gamma-1 chain C region.// 0// 314aa// 95%
BRTHA2019743
BRTHA2020400// Ig lambda chain V-IV region Bau.// 2.00E-45// 82aa// 78%
BRTHA2020566
BRTHA2020642// DRR1 protein (TU3A protein).// 5.00E-19// 120aa// 50%
BRTHA2020695
BRTHA2020721// Ig gamma-1 chain C region.// 0// 315aa// 95%
BRTHA2020781
BRTHA2020910// Tubulin beta-4 chain (Tubulin beta-III).// 1.00E-163// 283aa// 93%
BRTHA2021212
BRTHA2021440
BRTHA2021450
BRTHA2022074
BRTHA2022914
BRTHA2022968// NG-CAM related cell adhesion molecule precursor (NR-CAM) (BRAVO).// 7.00E-45// 95aa// 54%
BRTHA2023402
BRTHA2023437
BRTHA2024177
BRTHA2024354
BRTHA2024712// T-box transcription factor TBX2 (T-box protein 2).// 6.00E-65// 123aa// 83%
BRTHA2025869// Neurofilament triplet L protein (68 kDa neurofilament protein) (Neurofilament light polypeptide) (NF-L).// 1.00E-157// 306aa// 80%
BRTHA2026071// TH1 drosophila homolog [Homo sapiens]// 7.00E-21// 45aa// 100%
BRTHA2026290// Ral guanine nucleotide dissociation stimulator-like 2 (RaIGDS-like factor) (RAS-associated protein RAB2L).// 3.00E-17// 116aa// 24%
BRTHA2026311// Protein disulfide isomerase A6 precursor (EG 5.3.4.1) (Protein disulfide isomerase P5).// 0// 379aa// 87%
BRTHA2027227
BRTHA2027229
BRTHA2027250// Synaptotagmin B (Synaptic vesicle protein 0-P65-B).// 6.00E-46// 99aa// 35%
BRTHA2028297
BRTHA2029969
BRTHA2030036
BRTHA2030213
BRTHA2031517
BRTHA2031917// Ciliary neurotrophic factor receptor alpha precursor (CNTFR alpha).// 1.00E-107// 181aa// 88%
BRTHA2032763
BRTHA2033122
BRTHA2033155// Beta-1, 4 N-acetylgalactosaminyltransferase (EC 2.4.1.92) ((N-acetylneuraminyl)-galactosylglucosylceramide) (GM2/GD2 synthase) (GaINAc-T).// 2.00E-43// 95aa// 65%
BRTHA2033320
BRTHA2033469
BRTHA2033683// Urea transporter, erythrocyte.// 1.00E-153// 259aa// 93%
BRTHA2034281
BRTHA2034576
BRTHA2035743// activated p21cdc42Hs kinase [Homo sapiens]// 0// 417aa// 73%
BRTHA2036055
BRTHA2036295
BRTHA2037247
BRTHA2038279
BRTHA2038345
BRTHA2038353
BRTHA3000456// Zinc finger protein HZF10.// 1.00E-134// 212aa// 57%
BRTHA3002411
BRTHA3003225
BRTHA3003417
BRTHA3003736// TFIIH basal transcription factor complex helicase XPB subunit (EC 3.6.1.-) (Basic transcription factor 2 89 kDa subunit) (BTF2-p89) (TFIIH 89 kDa subunit) (DNA-repair protein complementing XP-B cells) (Xeroderma pigmentosum group B complementing protein) (DNA excision repair protein ERCC-3).// 0// 520aa// 96%
BRTHA3005988
BRTHA3006593
BRTHA3007469
BRTHA3007662
BRTHA3009858
BRTHA3010135// LAR protein precursor (Leukocyte antigen related) (EC 3.1.3.48).// 0// 422aa// 100%
BRTHA3010212// Zinc finger protein 91 (Zinc finger protein HTF10) (HPF7).// 0// 397aa// 69%
BRTHA3010469// NMN adenylyltransferase; nicotinamide mononucleotide adenylyl transferase [Homo sapiens]// 2.00E-30// 61aa// 50%
BRTHA3010530// ubiquitin-protein ligase e3 componen n-recognin [Mus musculus]// 1.00E-161// 264aa// 85%
BRTHA3010540
BRTHA3010717// secretory carrier membrane protein 5 [Mus musculus]// 1.00E-107// 186aa// 79%
BRTHA3011187// hect domain and RLD 2 [Homo sapiens]// 7.00E-17// 51aa// 34%
BRTHA3011194// Chloride channel protein 3 (CIC-3).// 0// 622aa// 89%
BRTHA3011229
BRTHA3011265// Spindlin (30000 Mr metaphase complex) (SSEC P).// 5.00E-69// 120aa// 91%
BRTHA3011306
BRTHA3011361
BRTHA3011510
BRTHA3011892
BRTHA3011998// rhomboid (veinlet, Drosophila)-like; Rhomboid, drosophila, homolog of [Homo sapiens]// 9.00E-31// 110aa// 51%
BRTHA3012265// pendrin [Homo sapiens].// 8.00E-44// 300aa// 34%
BRTHA3013860
BRTHA3013882
BRTHA3014000// rapa-1 [Homo sapiens]// 0// 1059aa// 90%
BRTHA3014105
BRTHA3014507
BRTHA3014547// Ankyrin 1 (Erythrocyte ankyrin) (Ankyrin R) (Ankyrins 2.1 and 2.2).// 3.00E-85// 205aa// 34%
BRTHA3014835
BRTHA3014854
BRTHA3014920
BRTHA3016616
BRTHA3017791
BRTHA3018409// synaptotagmin-like 4; granuphilin-a: granuphilin-b: granuphilin// 2.00E-56// 160aa// 41%
BRTHA3018623
BRTHA3019183// Ca<2+>dependent activator protein for secretion: Ca2+- dependent activator protein for secretion [Mus musculus]// 4.00E-67// 122aa// 96%
BRTHA3020369
BRTHA3020771
BRTHA3021569
BRTHA3021708// FERM, RhoGEF, and pleckstrin domain protein 1; chondrocyte-derived ezrin-like protein [Homo sapiens]// 2.00E-66// 122aa// 64%
BRTHA3021786
BRTHA3021971// oxidation resistance 1; oxidation resistance 1; hypothetical protein FLJ10125 [Homo sapiens]// 1.00E-76// 136aa// 50%
BRTHA3022641
BRTHA3023403// phospholipase C, epsilon [Homo sapiens]// 0// 689aa// 90%
BRTHA3023590
BRTHA3023929
BRTHA3024600
BRTHA3025073// Actin cross-linking family protein 7 (Macrophin) (Trabeculin-alpha) (620 kDa actin-binding protein) (ABP620).// 0// 685aa// 97%
BRTHA3026161
BRTHA3026180
BRTHA3026556
BRTHA3026916// Ral guanine nucleotide dissociation stimulator (RaIGEF) (RaIGDS).// 0// 728aa// 85%
BRTHA3027171
BRTHA3027318
BRTHA3027638
BRTHA3027820
BRTHA3027879
BRTHA3027957// megacaryocytic acute leukemia protein [Homo sapiens]// 3.00E-36// 95aa// 41%
BRTHA3028339// YY1-associated factor 2 [Homo sapiens]// 4.00E-26// 55aa// 63%
BRTHA3028505
CERVX2000812
CERVX2000968// F-actin binding protein b-Nexilin [Rattus norvegicus].// 1.00E-112// 312aa// 92%
CHONS2000797// T-box transcription factor TBX15 (T-box protein 15) (MmTBx8).// 0// 451 aa// 90%
CHONS2001287// Insulin-like growth factor binding protein 3 precursor (IGFBP-3) (IBP-3) (IGF-binding protein 3).// 1.00E-117// 201aa// 77%
CHONS2001797
CHONS2001834// tumor endothelial marker 7 precursor [Homo sapiens]// 1.00E-147// 246aa// 98%
CHONS2002419
CHONS2002829// adipocyte enhancer binding protein 1 precursor [Homo sapiens]// 0// 542aa// 77%
COLON2001829// Dopamine- and cAMP-regulated neuronal phosphoprotein (DARPP-32).// 6.00E-84// 157aa// 77%
COLON2001866
COLON2004351// Probable beta-1,3-galactosyltransferase 8 (EC 2.4.1.-) (UDP-galactose:beta-N- acetylglucosamine beta-1,3-galactosyltransferase 8) // 4.00E-46// 93aa// 47%
COLON2004911// Oxygen-regulated protein 1 (Retinitis pigmentosa RP1 protein) (Retinitis pigmentosa 1 protein).// 2.00E-31// 110aa// 48%
COLON2005623// 2-19 protein precursor.// 3.00E-68// 121aa// 54%
COLON2005735// Blood group RH(CE) polypeptide (Rhesus C/E antigens) (RH30A) (RHIXB) (RH polypeptide 1) (RHPI).// 0// 353aa// 95%
CTONG2001932// bromodomain adjacent to zinc finger domain, 2B [Homo sapiens]// 0// 385aa// 66%
CTONG2003517
CTONG2006235// ubiquitin-protein ligase e3 componen n-recognin [Mus musculus]// 1.00E-24// 82aa// 30%
CTONG2008989// Gap junction beta-5 protein (Connexin 31.1) (Cx31.1).// 7.00E-74// 123aa// 57%
CTONG2009033
CTONG2009570// rab11 binding protein [Bos taurus].// 0// 400aa// 90%
CTONG2010330// Homo sapiens phospholipase A2, group IVB (cytosolic) (PLA2G4B)// 1.00E-122// 222aa// 45%
CTONG2010633
CTONG2011801// Zinc finger protein 91 (Zinc finger protein HTF10) (HPF7).// 3.00E-62// 104aa// 50%
CTONG2012123// Mus musculus enabled homolog (Drosophila) (Enah), mRNA// 1.00E-77// 155aa// 74%
CTONG2014206// oxidation resistance 1; oxidation resistance 1: hypothetical protein FLJ10125 [Homo sapiens]// 1.00E-127// 221aa// 82%
CTONG2014959
CTONG2020582// Acetyl-coenzyme A synthetase (EG 6.2.1.1) (Acetate-CoA ligase) (Acyl- activating enzyme).// 1.00E-157// 269aa// 54%
CTONG2026987
CTONG2027150
CTONG2027591// Mus musculus pecanex homolog (Drosophila) (Pcnx), mRNA.// 0// 347aa// 81%
CTONG2027783
CTONG2027959
CTONG3001605// putative tumor suppressor [Homo sapiens]// 1.00E-35// 77aa// 45%
CTONG3002518
CTONG3002588
CTONG3003669// high-glucose-regulated protein 8 [Homo sapiens]// 1.00E-101// 170aa// 79%
CTONG3008223
D9OST2003106// Homo sapiens SMART/HDAC1 associated repressor protein (SHARP), mRNA// 0// 486aa// 82%
D9OST2003989
D9OST2004417// 60S ribosomal protein L13 (Breast basic conserved protein 1).// 1.00E-100// 185aa// 88%
DFNES2001829
DFNES2011221
ERLTF2001452
ERLTF2001835
ERLTF2002178// Kelch-like protein X.// 1.00E-138// 251aa// 50%
ERLTF2002369
FCBBF3001018// Hydroxymethylglutaryl-CoA lyase (EC 4.1.3.4) (HMG-CoA lyase) (HL) (3- hydroxy-3-methylglutarate-CoA lyase).// 5.00E-88// 152aa// 73%
FCBBF3002188
FCBBF3005160
FCBBF3012443
FCBBF3020030// Transcription intermediary factor 1-alpha (TIF1-alpha).// 0// 423aa// 80%
FCBBF3021191// Protein phosphatase 2C gamma isoform (EC 3.1.3.16) (PP2C-gamma) (Protein phosphatase magnesium-dependent 1 gamma) (Protein phosphatase 1C).// 0// 441aa// 80%
FCBBF3024911// WHSC1L1 protein isoform short: Wolf-Hirschhorn syndrome candidate 1-like 1 protein [Homo sapiens]// 1.00E-124// 217aa// 91%
FCBBF5000384// SWI/SNF related, matrix associated, actin dependent regulator of chromatin, subfamily d, member 3; Rsc6p [Homo sapiens]// 0// 394aa// 84%
FEBRA2000805
FEBRA2002260
FEBRA2012625
FEBRA2013069
FEBRA2013570// 2-oxoisovalerate dehydrogenase alpha subunit, mitochondrial precursor (EC 1.2.4.4) (Branched-chain alpha-keto acid dehydrogenase component alpha chain (E1)) (BCKDH E1-alpha).// 0// 391aa// 95%
FEBRA2017736
FEBRA2017811
FEBRA2023498// Leucine-rich repeat protein LRRC3 precursor.// 2.00E-63// 116aa// 86%
FEBRA2026582// Homo sapiens protein (peptidyl-prolyl cis/trans isomerase) NIMA-interacting, 4 (parvulin) (PIN4)// 7.00E-25// 79aa// 100%
FEBRA2026977
FEBRA2028222
FEBRA2028457// Nucleolin (Protein C23).// 0// 360aa// 82%
FEHRT2001482
FEHRT2002708// solute carrier family 22 (organic cation transporter)-like 2 [Mus musculus].// 6.00E-42// 90aa// 44%
FEKID2001001
FEKID2001201
FEKID2002231
FEKID2002493// WNT-14 protein precursor.// 1.00E-100// 174aa// 61%
FEKID2002637// Serine/threonine protein phosphatase PP1-alpha 1 catalytic subunit (EC 3.1.3.16) (PP-1A).// 0// 297aa// 99%
FELNG2000720// CD166 antigen precursor (Activated leukocyte-cell adhesion molecule) (ALCAM).// 1.00E-123// 219aa// 91%
FELNG2001613
FELNG2001706// transcriptional co-activator with PDZ-binding motif (TAZ); transcriptional co-activator with PDZ-binding motif (TAZ); DKFZP58611419 protein [Homo sapiens]// 2.00E-21// 45aa// 97%
FELNG2001953// JAK binding protein [Homo sapiens]// 6.00E-96// 174aa// 82%
HCASM2007773
HCASM2008154// RNA-binding protein EWS (EWS oncogene) (Ewing sarcoma breakpoint region 1 protein).//1.00E-104// 219aa// 48%
HCHON2009766
HEART2002531
HHDPC2008185// jerky [Mus musculus]// 0// 397aa// 75%
HLUNG2012600
HSYRA2004550// Potential phospholipid-transporting ATPase IIB (EC 3.6.3.13).// 0// 433aa// 92%
HSYRA2007338
JCMLC1000159// Interferon-gamma receptor alpha chain precursor (CDw119).// 4.00E-65// 122aa// 78%
JCMLC2000273// Integrin alpha-M precursor (Cell surface glycoprotein MAC-1 alpha subunit) (CR-3 alpha chain) (CD11b) (Leukocyte adhesion receptor M01) (Neutrophil adherence receptor).// 0// 536aa// 96%
JCMLC2002095// P-selectin glycoprotein ligand 1 precursor (PSGL-1) (Selectin P ligand) (CD162 antigen).// 1.00E-21// 48aa// 72%
JCMLC2002751// Von Willebrand factor precursor (vWF).// 2.00E-42// 83aa// 37% KIDNE2004531// Lag protein [Mus musculus]// 1.00E-109// 197aa// 78%
KIDNE2010049// Glycerol kinase 2 (EC 2.7.1.30) (ATP:glycerol 3-phosphotransferase 2) (Glycerokinase 2) (GK 2).// 8.00E-87// 176aa// 34%
KIDNE2014496
KIDNE2015987// Uromodulin precursor (Tamm-Horsfall urinary glycoprotein) (THP).// 0// 433aa// 86%
KIDNE2016464
KIDNE2017153
KIDNE2018268
LIVER2008465
LYMPB1000158
LYMPB2001387
LYMPB2002236// Gene terminal protein (Membrane protein LMP-2A/LMP-2B).// 3.00E-75// 153aa// 55%
LYMPB2002344
LYMPB2002458// Tenascin precursor (TN) (Hexabrachion) (Cytotactin) (Neuronectin) (GMEM) (JI) (Miotendinous antigen) (Glioma-associated-extracellular matrix antigen) (GP 150-225).// 1.00E-115// 193aa// 45%
LYMPB2002478
MESAN2007032
MESAN2009156
MESAN2014624// tumor differentially expressed 1, like: membrane protein TMS-2 [Mus musculus]// 2.00E-71// 124aa// 52%
MESAN2016304
MESAN2017133
MESTC2000170
N1ESE2000698// WD-repeat protein 1 (Actin interacting protein 1) (NORI-1).// 0// 558aa// 92%
NETRP2000439
NETRP2000961// Cytosolic purine 5'-nucleotidase (EC 3.1.3.5).// 0// 368aa// 100%
NETRP2002082
NETRP2003103
NETRP2003268
NETRP2003448// Ras-related protein RAL-B.// 4.00E-97// 189aa// 83%
NETRP2003539
NETRP2004017// nuclear transcription factor Y, gamma: CCAAT-binding transcription factor subunit C: transactivator HSM-1: histone H1 transcription factor large subunit 2A [Homo sapiens]// 6.00E-90// 193aa// 59%
NETRP2004090
NETRP2004434
NETRP2005282
NETRP2005849
NETRP2005972
NETRP2006468
NETRP2007945
NETRP2008488
NETRP2008582// Adipophilin (Adipose differentiation-related protein) (ADRP).// 1.00E-19// 100aa// 44%
NOVAR2000783
NT2NE2011107
NT2NE2016041
NT2RI2004818// 1-phosphatidylinositol-4,5-bisphosphate phosphodiesterase beta 1 (EG 3.1.4.11) (PLC-beta-1) (Phospholipase C-beta-1) (PLC-I) (PLC-154). // 0// 883aa// 91%
NT2RI2009233
NT2RI2010795
NT2RI2012542
NT2RI2015533
NT2RI2023671
NT2RI2028537
NT2RI3001573// F-box protein FBL10 [Mus musculus].// 3.00E-78// 223aa// 62%
NT2RI3001967// Ankyrin homolog precursor.// 6.00E-13// 68aa// 30%
NT2RI3005861
NT2RI3005923// Cadherin-related tumor suppressor precursor (Fat protein).// 1.00E-116// 370aa// 28%
NT2RI3007095// Mus musculus neuregulin 1 (Nrg1), mRNA.// 0// 861aa// 96%
NT2RI3008179// thyroid hormone receptor-associated protein, 240 kDa subunit [Homo sapiens]// 0// 562aa// 43%
NT2RI3009480// Transcription factor Sp3 (SPR-2) (Fragment).// 1.00E-43// 78aa// 69%
NT2RI3009524// Crumbs protein homolog 1 precursor.// 1.00E-178// 375aa// 31%
NT2RP7003439
NT2RP7007387
NT2RP7014178
NT2RP7014778
NT2RP7016508// YN21_CAEEL Putative ATP-dependent RNA helicase T26G10.1 in chromosome III// 1.00E-157// 388aa// 69%
NT2RP7017139
NT2RP7019682
NT2RP7020343
NT2RP8000633
NT2RP8001363// signal peptide, CUB domain, EGF-like 1 [Mus musculus]// 0// 357aa// 87%
NT2RP8001407
NT2RP8001584// alpha integrin binding protein 63 [Homo sapiens]// 1.00E-17// 74aa// 30%
NT2RP8001604// CUB and Sushi multiple domains 1 [Homo sapiens]// 0// 754aa// 65%
NT2RP8001605
NT2RP8003490// LIM/homeobox protein LMX1A (LMX-1) (LIM-homeobox protein 1).// 0// 344aa// 90%
NT2RP8003657
NT2RP8003787// vanilloid receptor-related osmotically activated channel;
0SM9-like transient receptor potential channel 4: transient receptor potential channel 12: transient receptor potential, drosophila, homolog of, 12 [Homo sapiens]// 1.00E-118// 221aa// 44%
NT2RP8005546// schlafen 3 [Mus musculus]// 1.00E-34// 92aa// 29%
NT2RP8006452// Flightless-I protein.// 1.00E-14// 79aa// 30%
NT2RP8006521
NT2RP8007416
NT2RP8007503
NT2RP8007920
NT2RP8008057// cartilage intermediate layer protein [Homo sapiens]// 0// 564aa// 49%
NT2RP8009119
NT2RP8009248
NTONG2002278
NTONG2003805// Zinc finger protein 45 (BRC1744).// 0// 438aa// 98%
NTONG2004829
NTONG2008483// zinc finger protein 106 [Homo sapiens]// 0// 607aa// 72%
NTONG2009468
OCBBF2000831// AMBP protein precursor [Contains: A!pha-1-microglobulin: Inter-alpha- trypsin inhibitor light chain (ITI-LC) (Bikunin) (HI-30)].// 9.00E-23// 54aa// 36%
OCBBF2003518// Myosin heavy chain, non-muscle (Zipper protein) (Myosin II).// 3.00E-14// 118aa// 21%
OCBBF2004478// Homo sapiens cadherin, EGF LAG seven-pass G-type receptor 2 (flamingo homolog, Drosophila) (CELSR2), mRNA// 4.00E-96// 177aa// 80%
OCBBF2007039// ADAM-TS 7 precursor (EC 3.4.24.-) (A disintegrin and metalloproteinase with thrombospondin motifs 7) (ADAMTS-7) (ADAM-TS7).// 1.00E-143// 293aa// 36%
OCBBF2009536// Mus musculus amiloride-sensitive cation channel 1, neuronal (degenerin) (Accn1)// 0// 471aa// 95%
OCBBF2014745
OCBBF2016928// zinc finger protein [Homo sapiens]// 0// 449aa// 88%
OCBBF2018229
OCBBF2018618// Adenosine A1 receptor.// 1.00E-155// 273aa// 83%
OCBBF2019761
OCBBF2024589// Dihydropyrimidinase related protein-1 (DRP-1) (Collapsin response mediator protein 1) (CRMP-1).// 0// 528aa// 92%
OCBBF2024779
OCBBF2025631
OCBBF2030927// matrilin 4 isoform 2 precursor [Homo sapiens]// 2.00E-39// 138aa// 28%
OCBBF2036019// NADH-ubiquinone oxidoreductase 20 kDa subunit, mitochondrial precursor (EC 1.6. 5. 3) (EC 1.6. 99. 3) (Complex I-20KD) (CI-20KD) (PSST subunit).// 7.00E-36// 76aa// 97%
OCBBF3000743
OCBBF3000830
OCBBF3001076
OCBBF3001202// suppression of tumorigenicity 5 [Homo sapiens]// 0// 532aa// 87%
OCBBF3001333// ubiquitin UBF-fl [Homo sapiens]// 1.00E-166// 279aa// 58%
OCBBF3001616
OCBBF3003745
OCBBF3004487// Probable ATP-dependent RNA helicase p47.// 1.00E-140// 245aa// 100%
OCBBF3004908
OCBBF3005330// FYVE finger-containing phosphoinositide kinase (EC 2.7.1.68) (1- phosphatidylinositol-4-phosphate kinase) (PIPSK) (Ptdlns(4)P-5-kinase) (p235).// 0// 878aa// 86%
OCBBF3005843
OCBBF3006986// CDC4-like protein (Beige-like protein) (Fragment).// 1.00E-112// 299aa// 33%
OCBBF3007078
OCBBF3007704
OCBBF3008392// bromodomain adjacent to zinc finger domain, 2A; TTF-I interacting peptide 5 [Homo sapiens]// 0// 504aa// 92%
OCBBF3008835
OCBBF3009244// pantothenate kinase 1 beta [Mus musculus]// 1.00E-154// 267aa// 89%
OCBBF3019269// Homo sapiens Dvl-binding protein IDAX (inhibition of the DvI and Axin complex) (IDAX)// 1.00E-100// 168aa// 84%
OCBBF3020263// Zinc finger protein 135.// 4.00E-90// 144aa// 59%
OCBBF3020414
OCBBF3021086
OCBBF3021166
OCBBF3021361// rapa-1 [Homo sapiens]// 3.00E-50// 330aa// 50%
OCBBF3021502
OCBBF3021515
OCBBF3022123
OCBBF3022166// Nuclear receptor co-repressor 2 (N-CoR2) (Silencing mediator of retinoic acid and thyroid hormone receptor) (SMRT) (SMRTe) (Thyroid-, retinoic-acid-receptor-associated co-repressor) (T3 receptor- associating factor) (TRAC) (CTG26) . // 2. 00E-38// 75aa// 92%
OCBBF3022576
OCBBF3022827// putative Rab5 GDP/GTP exchange factor homologue [Homo sapiens]// 0// 384aa// 90%
OCBBF3023175// LPS-responsive beige-like anchor [Mus musculus]// 0// 999aa// 63%
OCBBF3023543
OCBBF3023913// Mus musculus protein phosphatase 1, regulatory (inhibitor) subunit 1C (Ppp1r1c)// 1.00E-154// 302aa// 44%
OCBBF3023993
OCBBF3025127
OCBBF3025131
OCBBF3025475// Renal sodium/dicarboxylate cotransporter (Na(+)/dicarboxylate cotransporter).// 1.00E-155// 279aa// 50%
OCBBF3025503// Eyes absent homolog 1.// 0// 423aa// 89%
OCBBF3025630// Brefeldin A-inhibited guanine nucleotide-exchange protein 1 (Brefeldin A-inhibited GEP 1) (p200 ARF-GEP1) (p200 ARF guanine nucleotide exchange factor).// 1.00E-132// 235aa// 96%
OCBBF3025887
OCBBF3025901
OCBBF3026088
OCBBF3026361
OCBBF3026979// Protein kinase C-binding protein NELL1 precursor (NEL-like protein 1) (Nel-related protein 1).// 0// 795aa// 94%
OCBBF3027969// myosin IIIA [Homo sapiens]// 3.00E-84// 187aa// 36%
OCBBF3028001// Rattus norvegicus potassium channel subunit (Slack) (Slack), mRNA// 0// 407aa// 76%
PEBLM2001803
PEBLM2003935
PEBLM2005615// C-C chemokine receptor type 7 precursor (C-C CKR-7) (CC-CKR-7) (CCR-7) (MIP-3 beta receptor) (EBV-induced G protein-coupled receptor 1) (EB11) (BLR2).// 0// 351aa// 92%
PEBLM2006298
PERIC2003349
PLACE5000492// 1-phosphatidylinositol-4,5-bisphosphate phosphodiesterase delta 1 (EC 3.1.4.11) (PLC-delta-1) (Phospholipase C-delta-1) (PLC-III).// 0// 505aa// 91%
PLACE5000522
PLACE5000527
PLACE6000012// cation-chloride cotransporter-interacting protein [Homo sapiens]// 3.00E-19// 54aa// 54%
PLACE6000055// neuronal differentiation related protein [Mus musculus]// 0// 416aa// 90%
PLACE6001933// Epidermal growth factor receptor precursor (EC 2.7.1.112).// 1.00E-40// 79aa// 98%
PLACE6003004// rTS beta protein [Homo sapiens]// 0// 388aa// 93%
PLACE6008315// similar to ALPHA-ACTININ, SARCOMERIC (F-ACTIN CROSS LINKING
PROTEIN) (D. melanogaster) [Homo sapiens].// 1.00E-76// 121aa// 100%
PLACE6010925// NY-REN-50 antigen [Homo sapiens]// 0// 345aa// 98%
PLACE6010936// orphan G protein-coupled receptor GPR26 [Rattus norvegicus].// 1.00E-62// 112aa// 63%
PLACE6016030// Wilms tumor 1-associating protein (WT1-associated protein).// 1.00E-73// 138aa// 91%
PLACE6019542
PLACE6019600// Ras-related protein Rab-12 (Fragment).// 1.00E-45// 92aa// 85%
PLACE6019674
PLACE7000266// titin [Homo sapiens]// 2.00E-36// 218aa// 24%
PLACE7000707
PLACE7001759// Arylacetamide deacetylase (EG 3. 1. 1. -) (AADAC) . // 8.00E-41 // 97aa// 36%
PLACE7002303// Pituitary homeobox 2 (RIEG bicoid-related homeobox transcription factor) (Solurshin) (ALL1 responsive protein ARP1).// 1.00E-145// 256aa// 80%
PLACE7003639
PLACE7003684
PLACE7003985// Estradiol 17 beta-dehydrogenase 1 (EC 1.1.1.62) (17-beta-HSD 1) (Placental 17-beta-hydroxysteroid dehydrogenase) (20 alpha- hydroxysteroid dehydrogenase) (20-alpha-HSD) (E2DH).// 1.00E-164// 293aa// 89%
PLACE7004103// Vigilin (High density lipoprotein-binding protein) (HDL-binding protein).// 0// 701aa// 91%
PLACE7004961// Dynamin-1 (EC 3.6.1.50) (D100) (Dynamin, brain) (B-dynamin).// 2.00E-18// 49aa// 68%
PLACE7005169// zinc finger protein 292 [Mus musculus]// 0// 953aa// 76%
PLACE7005671
PLACE7005840
PLACE7006090// Maltase-glucoamylase, intestinal [Includes: Maltase (EC 3.2.1.20) (Alpha-glucosidase); Glucoamylase (EC 3.2.1.3) (Glucan 1,4-alpha-glucosidase)].// 9.00E-59// 107aa// 86%
PLACE7006240
PLACE7006268// Histone acetyltransferase type B catalytic subunit (EC 2.3.1.48).// 1.00E-67// 124aa// 100%
PLACE7006498// Guanine nucleotide-binding protein, alpha-12 subunit (G alpha 12).// 1.00E-160// 277aa// 99%
PLACE7006540
PLACE7007379// carboxypeptidase Z precursor [Homo sapiens]// 1.00E-149// 245aa// 99%
PLACE7007973
PLACE7008136
PLACE7008766
PLACE7009563// Melanoma-associated antigen 11 (MAGE-11 antigen).// 1.00E-162// 284aa// 89%
PLACE7009757// Transcription factor Sp3 (SPR-2) (Fragment).//3.00E-44// 79aa// 70%
PLACE7009936// Active breakpoint cluster region-related protein.// 1.00E-114// 204aa// 99%
PLACE7010567
PLACE7011269
PLACE7011559// Death-associated protein kinase 1 (EC 2.7.1.-) (DAP kinase 1).// 1. 00E-153// 263aa// 88%
PLACE7012111// ADAM 12 precursor (EC 3.4.24.-) (A disintegrin and metalloproteinase domain 12) (Meltrin alpha).// 0// 662aa// 96%
PLACE7012127// AAA-ATPase TOB3 [Homo sapiens]// 9.00E-64// 118aa// 91%
PLACE7013060
PLACE7014247// Phosphatidylinositol 3-kinase catalytic subunit, gamma isoform (EC 2. 7. 1. 137) (P13-kinase P110 subunit gamma) (Ptd I ns-3-k i nase P110) (P13K) (P13Kgamma).// 4.00E-50// 93aa// 100%
PLACE7014396
PLACE7015238
PLACE7015647
PLACE7016214
PLACE7016321
PLACE7016454
PLACE7016526// Lon protease homolog, mitochondrial precursor (EC 3.4.21.-) (Lon protease-like protein) (LONP) (LONHs).// 0// 865aa// 90%
PLACE7018304
PLACE7018349
PLACE7018452
PLACE7018479
PLACE7018512
PROST2002078// RNA-binding protein with multiple splicing homolog (RBP-MS) (HEart, RRM Expressed Sequence) (Hermes).// 7.00E-90// 161aa// 77%
PROST2007444
PROST2016566// erythroblast macrophage protein [Mus musculus]// 6.00E-82// 144aa// 91%
PROST2017578
PROST2017729
PROST2017749
PROST2017910
PUAEN2000594// Polyadenylate-binding protein 1 (Poly(A)-binding protein 1) (PABP 1) (PABP1).// 5.00E-60// 117aa// 92%
PUAEN2000684// Mus musculus soc-2 (suppressor of clear) homolog// 5.00E-28// 107aa// 26%
PUAEN2006639// Vegetatible incompatibility protein HET-E-1.// 1.00E-46// 89aa// 36%
RECTM2001519
SKMUS2008585// protein phosphatase [Homo sapiens]// 1.00E-112// 198aa// 100%
SKMUS2009479
SKMUS2009557
SKNMC2003639
SKNSH2007306// Sperm-specific antigen 2 (Cleavage signal-1 protein) (CS-1).// 1.00E-102// 194aa// 85%
SMINT2003641
SMINT2009292
SMINT2009895
SMINT2010753// Probable kinesin light chain 3 (KLC 3).// 0// 528aa// 85%
SMINT2011406// Interferon-regulated resistance GTP-binding protein MxB (P78-related protein).// 1.00E-90// 165aa// 100%
SMINT2011509// Homo sapiens polymerase (DNA directed), lambda (POLL), mRNA// 1.00E-145// 254aa// 95%
SMINT2012040
SMINT2012179// LCAT-like lysophospholipase [Homo sapiens]// 1.00E-123// 205aa// 99%
SMINT2014166
SMINT2014721// Peroxisomal carnitine octanoyltransferase (EC 2.3.1.-) (COT).// 0// 611aa// 95%
SMINT2017964
SMINT2019105
SPLEN2001227
SPLEN2007689// Neutrophil cytosol factor 1 (NCF-1) (Neutrophil NADPH oxidase factor 1) (47 kDa neutrophil oxidase factor) (p47-phox) (NCF-47K) (47 kDa autosomal chronic granulomatous disease protein).// 2.00E-75// 135aa// 99%
SPLEN2011252
SPLEN2012571// Zinc finger protein 202.// 1.00E-77// 203aa// 32%
SPLEN2017999
SPLEN2019092
SPLEN2019480
SPLEN2021231
SPLEN2021991
SPLEN2022785
SPLEN2022920
SPLEN2024571
SPLEN2025012// Ig alpha-1 chain C region.// 0// 318aa// 92%
SPLEN2027852
SPLEN2028417// Homeobox protein HLX1 (Homeobox protein HB24).// 0// 364aa// 74%
SPLEN2028593
SPLEN2031004
SPLEN2032677
SPLEN2033996// pan-hematopoietic expression: tumor-suppressing STF cDNA 6:
TSSC6 tumor-suppressing STF cDNA 6: tumor suppressing subtransferable candidate 6 [Homo sapiens]// 2.00E-63// 111aa// 100%
SPLEN2034551// LIS1-interacting protein NUDE1, rat homolog [Homo sapiens]// 2.00E-71// 136aa// 100%
SPLEN2034601
SPLEN2034934// shroom [Mus musculus]// 2.00E-49// 90aa// 68%
SPLEN2035615
SPLEN2036608
SPLEN2037077
SPLEN2042051
STOMA2003894
STOMA2004663// T-cell receptor alpha chain V region RL-5 precursor.// 9.00E-36// 75aa// 65%
SYNOV2003326// Glucocorticoid-induced leucine zipper protein (Delta sleep-inducing peptide immunoreactor) (DSIP-immunoreactive peptide) (DIP protein) (hDIP) (TSC-22-like protein) (TSC-22R).// 3.00E-65// 125aa// 93%
SYNOV2017179// EBP50-PDZ interactor of 64 kD [Homo sapiens]// 0// 485aa// 94%
SYNOV30003455// upregulated by 1,25-dihydroxyvitamin D-3 [Homo sapiens]// 5.00E-65// 142aa// 36%
SYNOV4000598
SYNOV4003174// unconventional myosin from rat 4 for myosin I heavy chain [Rattus norvegicus]// 0// 650aa// 97%
SYNOV4004210
SYNOV4009139// hyaluronoglucosaminidase 2: lysosomal hyaluronidase: PH-20 homolog: hyaluronidase 2 [Homo sapiens]// 0// 429aa// 90%
SYNOV4009575
T1ESE2000609// SON protein (SON3).// 1.00E-177// 315aa// 86%
T1ESE2000904// zinc finger protein 304 [Homo sapiens]// 0// 325aa// 49%
T1ESE2002665// Bumetanide-sensitive sodium-(potassium)-chloride cotransporter 1 (Basolateral Na-K-Cl symporter).// 6.00E-60// 129aa// 31%
TBAES2003917// NG28 protein [Mus musculus]// 0// 479aa// 58%
TBAES2005361
TBAES2007428// N-acetylglucosamine-1-phosphodiester alpha-N- acetylglucosaminidase [Homo sapiens]// 0// 427aa// 82%
TBAES2007548
TBAES2007862
TESOP2002005
TESOP2003308
TESOP2004110
TESOP2008556
TESTI1000459
TESTI2001364// lactate dehydrogenase A -like [Homo sapiens]// 1.00E-113// 204aa// 81%
TESTI2001915// actin filament associated protein [Homo sapiens]// 1.00E-96// 183aa// 81%
TESTI2003768// Chloride channel protein 3 (CIC-3).// 2.00E-23// 47aa// 90%
TESTI2004452
TESTI2004601// NYD-TSPG protein [Homo sapiens]// 1.00E-45// 116aa// 35%
TESTI2004971
TESTI2005112// NADH-ubiquinone oxidoreductase 30 kDa subunit, mitochondrial precursor (EC 1. 6. 5. 3) (EC 1. 6. 99. 3) (Complex I-30KD) (CI-30KD).// 1.00E-110// 189aa// 100%
TESTI2005153
TESTI2005564
TESTI2006543
TESTI2007490// 2-hydroxyacylsphingosine 1-beta-galactosyltransferase precursor (EC 2.4.1.45) (UDP-galactose-ceramide galactosyltransferase) (Ceramide UDP-galactosyltransferase) (Cerebroside synthase).// 0// 510aa// 94%
TESTI2008636
TESTI 2009497// GPI-anchored protein p137 (p137GP1).// 0// 455aa// 76%
TESTI 2009739// Myosin heavy chain, smooth muscle isoform (SMMHC).// 3.00E-14// 104aa// 20%
TESTI2011020
TESTI2011033
TESTI2018335
TESTI2018687
TESTI2018867
TESTI2021112
TESTI2021654// thymidylate kinase family LPS-inducible member: thymidylate kinase homologue [Mus musculus]// 9.00E-19// 196aa// 74%
TESTI2022323// Olfactory receptor 2H1 (Hs6M1-16) (Olfactory receptor 6-2) (OR6-2) (OLFR42A-9004.14/9026.2).// 1.00E-174// 303aa// 95%
TESTI2023053
TESTI2023903// ubiquilin 1 [Homo sapiens]// 2.00E-53// 156aa// 34%
TESTI2024267
TESTI2026024// early development regulator 2 (homolog of polyhomeotic 2); polyhomeotic 2 protein [Mus musculus]// 1.00E-116// 198aa// 89%
TESTI2026284
TESTI2028613
TESTI2030519
TESTI2030901// glutathione S-transferase theta 1 [Homo sapiens]// 4.00E-18// 68aa// 57%
TESTI2033905
TESTI2034913// Keratin, type II cytoskeletal 8 (Cytokerat i n 8) (K8) (CK 8) . // 1.00E-64// 137aa// 69%
TESTI2035962
TESTI2036285// ubiquitin C [Rattus norvegicus]// 1.00E-78// 166aa// 91%
TESTI2036822
TESTI2037085
TESTI2037209
TESTI2037572
TESTI2037657// Integral membrane glycoprotein gp210 precursor.// 1.00E-147// 260aa// 48%
TESTI2037877
TESTI2038733
TESTI2039342
TESTI2039732
TESTI2039738
TESTI2040372
TESTI2040377// Mitogen-activated protein kinase kinase kinase 1 (EC 2.7.1.-) (MAPK/ERK kinase kinase 1) (MEK kinase 1) (MEKK 1).// 1.00E-13// 56aa// 28%
TESTI2041362
TESTI2041976
TESTI2046188
TESTI2049041
TESTI2049062// peroxisomal 2,4-dienoyl-CoA reductase [Homo sapiens]// 6.00E-51// 106aa// 63%
TESTI2051742
TESTI2052110
TESTI2052202
TESTI2052670// Basement membrane-specific heparan sulfate proteoglycan core protein precursor (HSPG) (Per lecan) (PLC).// 2.00E-44// 139aa// 32%
TESTI2052799
TESTI4000370
TESTI4000534
TESTI4000600
TESTI4000621// Snf2-related CBP activator protein [Homo sapiens]// 0// 1397aa// 62%
TESTI4000703
TESTI4000957
TESTI4001037
TESTI4001348
TESTI4001517// Keratin, type I cytoskeletal 18 (Cytokeratin 18) (K18) (CK 18).// 7.00E-52// 103aa// 78%
TESTI4001569
TESTI4001679// RING finger protein 4.// 2.00E-92// 165aa// 86%
TESTI4002003
TESTI4002072
TESTI4002141
TESTI4002195
TESTI4002520
TESTI4002774// oxysterol binding protein 2 [Mus musculus]// 3.00E-48// 125aa// 35%
TESTI4002799// DNA-directed RNA polymerase I 135 kDa polypeptide (EC 2.7.7.6) (RNA polymerase I subunit 2) (RPA135).// 0// 1001aa// 88%
TESTI4002868// DNA-binding protein RFX5 (Regulatory factor X subunit 5).// 5.00E-40// 76aa// 47%
TESTI4002889
TESTI4003179
TESTI4003279
TESTI4003319
TESTI4003404
TESTI4003565
TESTI4003574
TESTI4003579// FH1/FH2 domains-containing protein (Formin homolog overexpressed in spleen) (FHOS).// 1.00E-111// 207aa// 53%
TESTI4003602
TESTI4003703// retinoblastoma-associated protein RAP140 [Homo sapiens]// 5.00E-46// 101aa// 40%
TESTI4003733
TESTI4003796// Zinc finger protein 189.// 1.00E-79// 148aa// 45%
TESTI4003944// Zinc finger protein 91 (Zinc finger protein HTF10) (HPF7).// 8.00E-32// 162aa// 23%
TESTI4004031
TESTI4004210
TESTI4004539
TESTI4004653// CTL2 gene [Homo sapiens]// 0// 292aa// 51%
TESTI4004695
TESTI4004917//Dyne in beta chain, ciliary.// 0// 560aa// 73%
TESTI4005013
TESTI4005322// Zinc finger protein 85 (Zinc finger protein HPF4) (HTF1).// 0// 339aa// 69%
TESTI4005399
TESTI4005470// Transcriptional repressor CTCF.// 1.00E-155// 249aa// 68%
TESTI4005653// SON DNA binding protein: SON DNA-binding protein: SON DNA-binding protein, KIAA1019; NRE-binding protein [Homo sapiens]// 0// 1122aa// 77%
TESTI4006441
TESTI4006539
TESTI4007565
TESTI4007671// Mus musculus quiescin Q6 (Qscn6), mRNA.// 1.00E-111// 207aa// 44%
TESTI4007965// Adapter-related protein complex 1 gamma 1 subunit (Gamma-adaptin) (Golgi adaptor HA1/AP1 adaptin gamma subunit) (Clathrin assembly protein complex 1 gamma large chain).// 0// 766aa// 93%
TESTI4008086
TESTI4008305
TESTI4009501
TESTI4010544
TESTI4010721
TESTI4010902// Echinoderm microtubule-associated protein-like 4 (EMAP-4) (Restrictedly overexpressed proliferation-associated protein) (Ropp 120).// 1.00E-56// 135aa// 35%
TESTI4010979
TESTI4011616
TESTI4011744
TESTI4011926
TESTI4012258
TESTI4012382
TESTI4012623
TESTI4012956
TESTI4012960
TESTI4013474// PTPL1-associated RhoGAP 1 [Homo sapiens]// 1.00E-133// 283aa// 33%
TESTI4013742// antigen identified by monoclonal antibody 2A8 [Mus musculus]// 0// 640aa// 70%
TESTI4013774
TESTI4013960
TESTI4013962
TESTI4014262
TESTI4014415
TESTI4014891
TESTI4014908// dedicator of cyto-kinesis 2 [Mus musculus]// 0// 825aa// 92%
TESTI4014932// Integral membrane glycoprotein gp210 precursor.// 0// 580aa// 40%
TESTI4014977
TESTI4015129
TESTI4015339
TESTI4016848
TESTI4017229
TESTI4017382// Copine VII.// 1.00E-152// 250aa// 72%
TESTI 4017647// homolog-like: Jerky, mouse, homolog-like [Homo sapiens]// 2.00E-07// 67aa// 37%
TESTI4017854
TESTI4018436// Ubiquitin fusion degradation protein 1 homolog (UB fusion protein 1).// 5.00E-70// 125aa// 100%
TESTI4018506// tomosyn [Rattus norvegicus]// 0// 689aa// 85%
TESTI4019149
TESTI4020342// H326 [Homo sapiens]// 0// 370aa// 61%
TESTI4020596// calpain 5 [Homo sapiens]// 0// 629aa// 98%
TESTI4020819// complement-c1q tumor necrosis factor-related protein 6 [Homo sapiens]// 2.00E-74// 135aa// 87%
TESTI4021129
TESTI4021197// Regulator of G-protein signaling 3 (RGS3) (RGP3).// 0// 479aa// 92%
TESTI4021377
TESTI4021569// ATP-binding cassette, sub-family B, member 8, mitochondrial precursor (Mitochondrial ATP-binding cassette 1) (M-ABC1).// 0// 343aa// 77%
TESTI4021713// synaptonemal complex protein 2 [Homo sapiens]// 1.00E-46// 108aa// 40%
TESTI4021821
TESTI4022158// transmembrane receptor Unc5H2 [Rattus norvegicus]// 0// 812aa// 87%
TESTI4023096// solute carrier family 12 (potassium/chloride transporters), member 6: potassium/chloride transporter 3 [Homo sapiens]// 0// 901aa// 89%
TESTI4023172
TESTI4023654
TESTI4024240
TESTI4024245
TESTI4024294// WW domain binding protein 2 [Mus musculus]// 3.00E-30// 68aa// 48%
TESTI4024494// zinc-binding protein Rbcc728 [Homo sapiens]// 1.00E-111// 190aa// 46%
TESTI4025062
TESTI4025401
TESTI4025908
TESTI4026080// family member, chromosome-associated protein C [Homo sapiens]// 0// 1137aa// 88%
TESTI4026680
TESTI4027139
TESTI4027170
TESTI4027262
TESTI4027660
TESTI4028042// NYD-SP11 protein [Homo sapiens]// 0// 610aa// 90%
TESTI4028182// 116 kDa U5 small nuclear ribonucleoprotein component (U5 snRNP- specific protein, 116 kDa) (U5-116 kDa).// 0// 642aa// 95%
TESTI4029023
TESTI4029297
TESTI4029651
TESTI4029676
TESTI4029731
TESTI4029743
TESTI4030319
TESTI4030673
TESTI4030864
TESTI4031066
TESTI4031173// Contactin 2 precursor (Axonin-1) (Axonal glycoprotein TAG-1) (Transient axonal glycoprotein 1) (TAX-1).// 1.00E-29// 93aa// 30%
TESTI4031818
TESTI4032128// Amyloid-like protein 2 precursor (Amyloid protein homolog) (APPH) (CDEI-box binding protein) (CDEBP).// 0// 564aa// 84%
TESTI4032270
TESTI4032375
TESTI4032834// trinucleotide repeat containing 11 (THR-associated protein, 230 kDa subunit); thyroid hormone receptor-associated protein, 230 kDa subunit [Homo sapiens]// 0// 359aa// 66%
TESTI4032856
TESTI4032913// Ankyrin 2 (Brain ankyrin) (Ankyrin B) (Ankyrin, nonerythroid).// 1.00E-30// 106aa// 30%
TESTI4033177// Dual specificity protein phosphatase 8 (EC 3.1.3.48) (EC 3.1.3.16) (Dual specificity protein phosphatase hVH-5).// 2.00E-57// 110aa// 92%
TESTI4034633
TESTI4034973
TESTI4035770
TESTI4035872
TESTI4035898
TESTI4035989
TESTI4036012
TESTI4036048// Sorting nexin 1.// 0// 457aa// 90%
TESTI4037228
TESTI4037949// Kelch-like protein X.// 1.00E-129// 241aa// 44%
TESTI4038047
TESTI4038758
TESTI4039451// B29 protein [Homo sapiens]// 3.00E-34// 73aa// 38%
TESTI4039575// Telomeric repeat binding factor 1.// 2.00E-12// 62aa// 100%
TESTI4039744
TESTI4039904// Zinc finger protein 85 (Zinc finger protein HPF4) (HTF1).// 0// 298aa// 72%
TESTI4040197
TESTI4040559// Cyclic-nucleotide-gated olfactory channel (Cyclic-nucleotide-gated cation channel 2) (CNG channel 2) (CNG-2) (CNG2) (Aorta CNG channel) (RACNG).// 0// 600aa// 90%
TESTI4040598
TESTI4040804
TESTI4041049// leucine-rich neuronal protein [Homo sapiens]// 2.00E-72// 136aa// 90%
TESTI4041482// Rattus norvegicus SEC15 homolog (S. cerevisiae) (Sec15), mRNA// 0// 700aa// 88%
TESTI4041832
TESTI4041984// latent transforming growth factor beta binding protein 4 [Homo sapiens]// 0// 732aa// 86%
TESTI4042420
TESTI4042846
TESTI4043067
TESTI4043166// lymphocyte specific formin related protein: formin-related gene in leukocytes [Mus musculus]// 1.00E-137// 257aa// 60%
TESTI4043223
TESTI4043371
TESTI4043378
TESTI4044076
TESTI4044291
TESTI4044770
TESTI4045168
TESTI4045330
TESTI4045470
TESTI4046073// Rho-GTPase-activating protein 6 (Rho-type GTPase-activating protein RhoGAPX-1).// 1.00E-75// 177aa// 36%
TESTI4046090
TESTI4046245
TESTI4046328
TESTI4046450
TESTI4046873
TESTI4046962
TESTI4047119// AIM-1 protein [Homo sapiens]// 1.00E-38// 82aa// 35%
TESTI4047305
TESTI4047328// otogelin [Mus musculus]// 0// 1009aa// 65%
TESTI4047437
TESTI4047569
TESTI4047746
TESTI4047808
TESTI4048232
TESTI4048296
TESTI4048545
TESTI4048619
TESTI4049110
TESTI4049552
TESTI4049562
TESTI4049677
TESTI4049786// Hexokinase, type I (EC 2.7.1.1) (HK I) (Brain form hexokinase).// 0// 892aa// 99%
TESTI4049863
TESTI4049899// deleted in malignant brain tumors 1 isoform c precursor [Homo sapiens]// 6.00E-44// 79aa// 45%
TESTI4050293
TESTI4050954
TESTI4051015// Aquaporin 7 (Aquaporin-7 like) (Aquaporin adipose) (AQPap).// 7.00E-17// 45aa// 86%
TESTI4051054
TESTI4051388
TESTI4051424// M-protein, striated muscle.// 0// 511aa// 43%
TESTI4051504
TESTI4051747
TESTI4051858
TESTI4051865// Tight junction protein Z0-3 (Zonula occludens 3 protein) (Zona occludens 3 protein) (Tight junction protein 3).// 0// 825aa// 88%
TESTI4051952
TESTI4052132
TESTI4052217
TESTI4052219// Potential phospholipid-transporting ATPase IH (EC 3.6.3.13).// 0// 670aa// 92%
TESTI4052430
TESTI4052598// C-type lectin-like receptor-1 [Homo sapiens]// 7.00E-29// 67aa// 33%
TESTI4052775// Krueppel-related zinc finger protein 1 (HKR1 protein) (Fragment).// 1.00E-29// 78aa// 32%
THYMU2008207
THYMU2038199
THYMU3000390
THYMU3000776
THYMU3001082
THYMU3001593
THYMU3001776
THYMU3002825// alpha 1,2-mannosidase [Homo sapiens]// 1.00E-169// 282aa// 100%
THYMU3002887
THYMU3003007
THYMU3003350
THYMU3003958
THYMU3004628
THYMU3004632
THYMU3007308
THYMU3007559
THYMU3008105// Zinc finger protein 74.// 0// 490aa// 90%
THYMU3008935
THYMU3009643
THYMU3009755
THYMU3011012
THYMU3011244
THYMU3011360
THYMU3011534
THYMU3011556
THYMU3011717// exocyst component protein 70 kDa homolog (S. cerevisiae)// 1.00E-25// 62aa// 100%
THYMU3012402// import in alpha-1 subunit (Karyopherin alpha-1 subunit) (SRP1-beta) (RAG cohort protein 2) (Nucleoprotein interactor 1) (NPI-1).// 0// 322aa// 95%
THYMU3012907
THYMU3012983// zinc finger protein 14 (KOX 6): GIOT-4 for gonadotropin inducible transcription repressor-4 [Homo sapiens]// 1.00E-156// 265aa// 54%
THYMU3013114
THYMU3013197
THYMU3013241
THYMU3013470
THYMU3013785// novel SH2-containing protein 3 [Homo sapiens]// 0// 457aa// 90%
THYMU3013897
THYMU3014038
THYMU3014173// HLA class I histocompatibility antigen, alpha chain E*0101/E*0102 precursor.// 8.00E-26// 60aa// 57%
THYMU3014372// DNA replication licensing factor MCM2 (Nuclear protein BM28).// 0// 743aa// 96%
THYMU3014620// F-box protein Lilina [Homo sapiens].// 1.00E-123// 239aa// 100%
THYMU3014701
THYMU3015042
THYMU3015457
THYMU3015571// 10 kDa heat shock protein, mitochondrial (Hsp10) (10 kDa chaperonin) (CPN10).// 5.00E-18// 44aa// 100%
THYMU3015647// serpentine receptor (secretin receptor superfamily member with s: serpentine receptor: secretin receptor [Mus musculus]// 4.00E-57// 151aa// 30%
THYMU3016518// lg gamma-1 chain C region.// 0// 304aa// 94%
THYMU3016822// erythroblast macrophage protein [Mus musculus]// 1.00E-42// 80aa// 97%
THYMU3017761
THYMU3018151
THYMU3018896
THYMU3019095
THYMU3019476
THYMU3019605
THYMU3019916
THYMU3020221// Ig gamma-1 chain C region.// 0// 315aa// 95%
THYMU3020869// BMAL1 protein (Brain and muscle ARNT-like 1) (Member of PAS protein 3) (MOP3) (BHLH-PAS protein JAP3).// 1.00E-45// 89aa// 83%
THYMU3020970
THYMU3021404// differentiation-associated Na-dependent inorganic phosphate cotr: differentiation-associated Na-dependent inorganic phosphate cotransporter [Homo sapiens]// 0// 417aa// 75%
THYMU3021586// Sterol regulatory element binding protein-1 (SREBP-1) (Sterol regulatory element-binding transcription factor 1).// 0// 774aa// 86%
THYMU3021755// solute carrier family 4, sodium bicarbonate cotransporter, member 8 [Homo sapiens]// 1.00E-169// 294aa// 93%
THYMU3021900
THYMU3022211
THYMU3022434// Monocytic leukemia zinc finger protein (Zinc finger protein 220).// 0// 537aa// 81%
THYMU3022528
THYMU3022668
THYMU3022982
THYMU3023107
THYMU3023394
THYMU3023400// amino acid transporter 2 [Homo sapiens]// 4.00E-19// 71aa// 25%
THYMU3023797
THYMU3024164
THYMU3024339
THYMU3025118// Cell surface glycoprotein MUC18 precursor (Melanoma-associated antigen MUC18) (Melanoma-associated antigen A32) (S-endo 1 endothelial-associated antigen) (CD146 antigen) (Melanoma adhesion molecule).// 0// 493aa// 95%
THYMU3025313
THYMU3025642
THYMU3025683// Ras interaction/interference protein 1.// 8.00E-36// 111aa// 30%
THYMU3025772
THYMU3026000// zinc finger protein interacting with K protein 1 [Mus musculus]// 0// 335aa// 70%
THYMU3026306// alpha 1,2-mannosidase [Homo sapiens]// 1.00E-169// 282aa// 100%
THYMU3026350
THYMU3026479// secretory pathway component Sec31B-1 [Homo sapiens]// 0// 385aa// 95%
THYMU3026532// Integrin beta-2 precursor (Cell surface adhesion glycoproteins LFA- 1/CR3/p150,95 beta-subunit) (CD18) (Complement receptor C3 beta-subunit).// 0// 701aa// 93%
THYMU3026783
THYMU3026869
THYMU3027251
THYMU3027540
THYMU3027655
THYMU3027671// extra spindle poles, S. cerevisiae, homolog of [Homo sapiens]// 1.00E-176// 306aa// 91%
THYMU3028075
THYMU3028461
THYMU3028702// chromosome condensation-related SMC-associated protein 1; chromosome condensation-related SMC-associated protein 1: KIAA0159 gene product [Homo sapiens]// 0// 1278aa// 91%
THYMU3029188
THYMU3029318
THYMU3029421
THYMU3029719// AAA-ATPase TOB3 [Homo sapiens]// 6.00E-52// 105aa// 85%
THYMU3029774
THYMU3029795
THYMU3030072// Zinc finger protein 84 (Zinc finger protein HPF2).// 0// 365aa// 51%
THYMU3030706
THYMU3030752
THYMU3031146
THYMU3031402
THYMU3031612
THYMU3031868
THYMU3031878
THYMU3032032// Natural killer cell receptor BY55 precursor (CD160 antigen).// 2.00E-20// 46aa// 95%
THYMU3032798// Focal adhesion kinase 2 (EC 2.7.1.112) (FADK 2) (Proline-rich tyrosine kinase 2) (Cell adhesion kinase beta) (CAK beta).// 0// 512aa// 85%
THYMU3032867
THYMU3033626
THYMU3033630
THYMU3033649// T-cell surface glycoprotein CD3 zeta chain precursor (T-cell receptor T3 zeta chain).// 2.00E-57// 110aa// 94%
THYMU3033754// basement membrane-induced gene [Homo sapiens]// 6.00E-25// 89aa// 32%
THYMU3033759// Eukaryotic translation initiation factor 4 gamma (elF-4-gamma) (elF- 4G) (elF4G) (P220).// 0// 522aa// 68%
THYMU3034099
THYMU3034453
THYMU3034616
THYMU3034671// histone deacetylase 7A, isoform b: histone deacetylase 7A: histone.deacetylase 7 [Homo sapiens]// 0// 412aa// 96%
THYMU3034853
THYMU3034867
THYMU3034983
THYMU3036200
THYMU3036310
THYMU3036934
THYMU3036953// mosaic serine protease [Homo sapiens]// 3.00E-13// 100aa// 39%
THYMU3037052// HIV TAT specific factor 1; cofactor required for Tat activation of HIV-1 transcription [Homo sapiens]// 1.00E-31// 80aa// 45%
THYMU3037192// potassium large conductance calcium-activated channel, subfamily M, alpha member 1 [Mus musculus]// 8.00E-94// 166aa// 100%
THYMU3037617
THYMU3037772// lg gamma-1 chain C region.// 0// 313aa// 94%
THYMU3037827
THYMU3037856
THYMU3037867
THYMU3037909
THYMU3037980
THYMU3038158// Acetylcholinesterase collagenic tail peptide precursor (AChE Q subunit) (Acetylcholinesterase-associated collagen).// 1.00E-138// 237aa// 79%
THYMU3038167
THYMU3038214
THYMU3038266
THYMU3038347// tumor stroma and activated macrophage protein DLM-1 [Homo sapiens]// 3.00E-93// 159aa// 100%
THYMU3038375// interrupting locus; SCL interrupting locus [Homo sapiens]// 0// 573aa// 96%
THYMU3038603// WW domain binding protein 2 [Mus musculus]// 3.00E-48// 113aa// 98%
THYMU3038687
THYMU3038759
THYMU3038879
THYMU3038970
THYMU3039807
THYMU3039846
THYMU3040068
THYMU3040126
THYMU3040146
THYMU3040168
THYMU3040172// T-cell differentiation antigen CD6 precursor (T12) (TP120).// 0// 563aa// 84%
THYMU3040725
THYMU3040746// Ig gamma-2 chain C region.// 1.00E-166// 282aa// 88%
THYMU3040816// VAMP (vesicle-associated membrane protein)-associated protein B and C; VAMP-associated protein C: VAMP-associated protein B; VAMP-associated 33 kDa protein [Homo sapiens]// 1.00E-45// 91aa// 100%
THYMU3040829// Cold-inducible RNA-binding protein (Glycine-rich RNA-binding protein CIRP) (A18 hnRNP) . // 7.00E-34// 70aa// 100%
THYMU3040830// AD-012 protein [Homo sapiens]// 1.00E-28// 84aa// 97%
THYMU3041354
THYMU3041386
THYMU3041428// Homo sapiens prp28, U5 snRNP 100 kd protein [Homo sapiens]// 0// 599aa// 87%
THYMU3041573
THYMU3041603// gamma-tubulin complex protein 2 [Homo sapiens]// 1.00E-27// 59aa// 100%
THYMU3041736
THYMU3041918
THYMU3042075
THYMU3042321
THYMU3042758
THYMU3043200// Splicing factor 3A subunit 3 (Spliceosome associated protein 61) (SAP 61) (SF3a60).// 0// 479aa// 95%
THYMU3043327
THYMU3043482
THYMU3043688
THYMU3043779// Phospholipid scramblase 4 (PL scramblase 4) (Ca2+ dependent phospholipid scramblase 4).// 6.00E-69// 123aa// 93%
THYMU3043883
THYMU3043993
THYMU3044075
THYMU3044188
THYMU3044441
THYMU3044445
THYMU3045510// dedicator of cyto-kinesis 2 [Mus musculus]// 0// 321aa// 85%
THYMU3045673
THYMU3045692
THYMU3045704
THYMU3046140
THYMU3046360// F-box only protein 3.// 0// 393aa// 95%
THYMU3047115// Sarcoplasmic/endoplasmic reticulum calcium ATPase 1 (EC 3. 6. 3. 8) (Calcium pump 1) (SERCA1 ) - (SR Ca (2+) -ATPase 1) (Calcium-transporting ATPase sarcoplasmic reticulum type, fast twitch skeletal muscle isoform) (Endoplasmic reticulum class 1/2 Ca(2+) ATPase).// 0// 826aa// 95%
THYMU3047144
THYMU3047156
THYMU3047513
THYMU3047542
THYMU3047760// cleft lip and palate associated transmembrane protein 1 [Homo sapiens]// 3.00E-46// 80aa// 59%
THYMU3047891// oxidoreductase UCPA [Homo sapiens]// 8.00E-59// 111aa// 84%
TKIDN2000319
TKIDN2003396// Homo sapiens paternally expressed 10 (PEG10), mRNA// 3.00E-17// 310aa// 22%
TKIDN2010602
TKIDN2011051.
TKIDN2011160// ADAM-TS 3 precursor (EC 3.4.24.-) (A disintegrin and metalloproteinase with thrombospondin motifs 3) (ADAMTS-3) (ADAM-TS3) (Fragment).// 1.00E-114// 176aa// 64%
TLIVE2000142
TLIVE2001616// Zinc finger protein 84 (Zinc finger protein HPF2).// 1.00E-177// 285aa// 50%
TLIVE2007736// solute carrier family 9 (sodium/hydrogen exchanger), isoform 3 regulatory factor 1 [Homo sapiens]// 2.00E-95// 172aa// 85%
TLIVE2008797
TLUNG2000654// Keratin, type II cytoskeletal 7 (Cytokeratin 7) (K7) (CK 7).// 0// 361aa// 79%
TLUNG2001445// lg alpha-1 chain C region.// 0// 326aa// 92%
TLUNG2001600// lg gamma-4 chain C region.// 0// 311aa// 95%
TLUNG2001810
TLUNG2002055
TRACH2011057// D-type cyclin-interacting protein 1; MAID protein [Homo sapiens]// 3.00E-89// 161aa// 89%
TRACH2013585
TRACH2019080
TRACH2022113
TRACH2024730// SH3-domain binding protein 4 [Homo sapiens]// 1.00E-178// 320aa// 47%
TRACH3002188
TRACH3002293
TRACH3002752// ubiquitin-protein ligase e3 componen n-recognin [Mus musculus]// 1.00E-24// 82aa// 30%
TRACH3002890
TRACH3003037// Putative Rho/Rac guanine nucleotide exchange factor (Rho/Rac GEF) (Faciogenital dysplasia protein homolog).// 3.00E-75// 175aa// 30%
TRACH3003357// SH3 domain protein D19: SH3 domain containing protein D 19; SH3 domain-containing protein [Mus musculus]// 0// 321aa// 76%
TRACH3003458// Zinc finger protein 208.// 0// 436aa// 62%
TRACH3003872
TRACH3004113
TRACH3004288
TRACH3004412// clusterin-like 1 (retinal): unknown prepropeptide specific to rod photoreceptor [Homo sapiens]// 0// 445aa// 95%
TRACH3004424// Zinc finger protein 91 (Zinc finger protein HTF10) (HPF7).// 0// 559aa// 59%
TRACH3004596
TRACH3004747// organic anion transporter OATP-E [Homo sapiens]// 1.00E-129// 251aa// 40%
TRACH3005173// Breakpoint cluster region protein (EC 2.7.1.-).// 9.00E-85// 157aa// 83%
TRACH3005191// androgen-induced prostate proliferative shutoff associated protein [Homo sapiens]// 0// 516aa// 87%
TRACH3005274// Glycosyltransferase ALG11 (EC 2.4.1.-).// 1.00E-47// 104aa// 37%
TRACH3005699// mucin 16: CA125 ovarian cancer antigen [Homo sapiens]// 0// 1037aa// 90%
TRACH3006379// Keratin, type II cytoskeletal 7 (Cytokeratin 7) (K7) (CK 7).// 1.00E-106// 212aa// 74%
TRACH3006397
TRACH3006717
TRACH3006800// mucin 16: CA125 ovarian cancer antigen [Homo sapiens]// 0// 901aa// 90%
TRACH3007274
TRACH3007625// Hemolysin III (HLY-III).// 8.00E-06// 60aa// 25%
TRACH3007689
TRACH3007995
TRACH3008042// Membrane component, chromosome 17, surface marker 2 (Ovarian carcinoma antigen CA125) (1A1-3B) (Next to BRCA1 gene 1 protein).// 0// 327aa// 100%
TRACH3008508// GC-rich sequence DNA-binding factor homolog.// 1.00E-109// 198aa// 85%
TRACH3008632
TRACH3009008// Thioredoxin reductase (EC 1.6.4.5).// 0// 456aa// 95%
TRACH3009061//ATP-bind ing cassette, sub-family A, member 1 (ATP-binding cassette transporter 1) (ATP-binding cassette 1) (ABC-1).// 1.00E-138// 265aa// 41%
TRACH3009701// dyne in, axonemal, heavy polypeptide 9, isoform 2// 1.00E-165// 329aa// 36%
TRACH3010079// Homo sapiens COP9 constitutive photomorphogenic homolog subunit 5 (Arabidopsis) (COPS5)// 1.00E-143// 246aa// 91%
TRACH3010167// Transcription factor Sp3 (SPR-2) (Fragment).// 1.00E-40// 72aa// 67%
TRACH3010342// Nuclear receptor ROR-gamma (Nuclear receptor RZR-gamma).// 4.00E-46// 86aa// 100%
TRACH3010382
TRACH3011082// Ig kappa chain V-III region HAH precursor.// 3.00E-51// 100aa// 77%
TRACH3011184// Ig kappa chain V-III region CLL precursor (Rheumatoid factor).// 5.00E-53// 101aa// 78%
TRACH3011282// Allograft inflammatory factor-1 (AIF-1) (Ionized calcium-binding adapter molecule 1).// 5.00E-46// 94aa// 56%
TRACH3011313// Branched-chain amino acid aminotransferase, cytosolic (EC 2.6.1.42) (BCAT(C)) (ECA39 protein).// 0// 320aa// 88%
TRACH3011454
TRACH3011503// UDP-N-acetylhexosamine pyrophosphorylase (Antigen X) (AGX) (Sperm- associated antigen 2) [Includes: UDP-N-acetylgalactosamine pyrophosphorylase (EC 2.7.7.-) (AGX-1): UDP-N-acetylglucosamine pyrophosphorylase (EC 2.7.7.23) (AGX-2)].// 1.00E-109// 197aa// 55%
TRACH3011538
TRACH3012106// erythrocyte protein band 4.1-like 3 [Mus musculus]// 0// 610aa// 74%
TRACH3012460
TRACH3012659// Ig heavy chain V-III region VH26 precursor.// 4.00E-51// 95aa// 86%
TRACH3012718
TRACH3012864
TRACH3012891// ATPase, Class I, type 8B, member 1; benign recurrent intrahepatic cholestasis; progressive familial intrahepatic cholestasis 1, Byler disease: familial intrahepatic cholestasis 1, (progressive, Byler disease and benign recurrent); ATPase ,Class I, type 8B, member 1 [Homo sapiens]// 6.00E-42// 85aa// 84%
TRACH3013043
TRACH3013072
TRACH3013684
TRACH3013900
TRACH3014063
TRACH3014183
TRACH3014580
TRACH3015136// Polypeptide N-acetylgalactosaminyltransferase (EC 2.4.1.41) (Protein- UDP acetylgalactosaminyltransferase) (UDP-GaINAc:polypeptide, N-acetylgalactosaminyltransferase) (GaINAc-T1).// 3.00E-67// 117aa// 56%
TRACH3015346
TRACH3015354
TRACH3015467
TRACH3015951// Zinc finger protein 151 (Myc-interacting zinc finger protein) (Miz-1 protein).// 4.00E-53// 156aa// 26%
TRACH3016264
TRACH3016368
TRACH3016455// calpain 10, isoform e; calcium-activated neutral protease [Homo sapiens]// 1.00E-162// 267aa// 91%
TRACH3016805// Ankyrin 2 (Brain ankyrin) (Ankyrin B) (Ankyrin, nonerythroid).// 1.00E-30// 106aa// 30%
TRACH3016885
TRACH3016992
TRACH3017409
TRACH3018108// 51 kDa FK506-binding protein (FKBP51) (Peptidyl-prolyl cis-trans isomerase) (EC 5.2.1.8) (PPiase) (Rotamase).// 0// 407aa// 89%
TRACH3018191
TRACH3018240
TRACH3018261// 1,2-alpha-mannosidase IC [Homo sapiens]// 4.00E-76// 139aa// 100%
TRACH3018519// Coatomer beta' subunit (Beta'-coat protein) (Beta'-COP) (p102).// 1.00E-22// 47aa// 100%
TRACH3018524// Protein-tyrosine phosphatase beta precursor (EC 3.1.3.48) (R-PTP- beta).// 0// 1251aa// 96%
TRACH3018606// SH3/ankyrin domain gene 3 [Rattus norvegicus]// 8.00E-26// 80aa// 68%
TRACH3018783
TRACH3018907// Ig alpha-1 chain C region.// 0// 325aa// 92%
TRACH3018943
TRACH3019058// Ig delta chain C region.// 1.00E-78// 137aa// 99%
TRACH3019370// ATP-binding cassette, sub-family A, member 1 (ATP-binding cassette transporter 1) (ATP-binding cassette 1) (ABC-1).// 1.00E-122// 264aa// 34%
TRACH3019621// UDP-N-acetylglucosamine-dolichyl-phosphate N-acetylglucosaminephosphotransferase (EC 2.7.8.15) (GPT) (G1PT) (N-acetylglucosamine-1-phosphate transferase) (GIcNAc-1-P transferase).// 0// 367aa// 89%
TRACH3019807// Antigen LY-9 precursor.// 4.00E-47// 103aa// 48%
TRACH3020137
TRACH3020563
TRACH3020605
TRACH3020769// Myosin heavy chain, nonmuscle type B (Cellular myosin heavy chain, type B) (Nonmuscle myosin heavy chain-B) (NMMHC-B).//2.00E-31// 159aa// 21%
TRACH3020930// lg gamma-1 chain C region.// 1.00E-166// 284aa// 80%
TRACH3021023// lg delta chain C region.// 0// 354aa// 92%
TRACH3021335
TRACH3021373// autoantigen [Homo sapiens]// 0// 836aa// 86%
TRACH3021544// ubiquitin-specific processing protease [Homo sapiens]// 1.00E-127// 276aa// 44%
TRACH3021778// Nucleolin (Protein C23).// 6.00E-50// 100aa// 85%
TRACH3021834
TRACH3021883// Neuronal PAS domain protein 2 (Neuronal PAS2) (Member of PAS protein 4) (MOP4) . // 2.00E-73// 140aa// 75%
TRACH3022109// rec [Homo sapiens]// 1.00E-107// 173aa// 59%
TRACH3022198
TRACH3022296// DnaJ homolog subfamily B member 1 (Heat shock 40 kDa protein 1) (Heat shock protein 40) (HSP40).// 9.00E-32// 63aa// 56%
TRACH3022732
TRACH3022758// dual oxidase 1 [Homo sapiens]// 0// 546aa// 97%
TRACH3022960// Dynein beta chain, ciliary.// 0// 386aa// 35%
TRACH3023063
TRACH3023203
TRACH3023242
TRACH3023373// Colorectal mutant cancer protein (MCC protein).// 0// 638aa// 84%
TRACH3023516// 65 kDa FK506-binding protein precursor (EC 5.2.1.8) (FKBP65) (FKBPRP) (Peptidyl-prolyl cis-trans isomerase) (PPiase) (Rotamase) (Immunophilin FKBP65).// 0// 333aa// 60%
TRACH3023752
TRACH3023945
TRACH3023960// dynein, axonemal, heavy polypeptide 8 [Homo sapiens]// 0// 551aa// 69%
TRACH3024020// calcium/calmodulin-dependent protein kinase kinase 1, alpha [Rattus norvegicus]// 1.00E-119// 208aa// 91%
TRACH3024081// Potential phospholipid-transporting ATPase ID (EC 3.6.3.13) (Fragment).// 5.00E-79// 136aa// 72%
TRACH3024342
TRACH3024423
TRACH3024428
TRACH3024512
TRACH3024671// squamous cell carcinoma antigen recognized by T cell [Homo sapiens]// 0// 602aa// 96%
TRACH3024823
TRACH3025316// import in alpha-6 subunit (Karyopherin alpha-5 subunit) (Importin alpha S2).// 1.00E-165// 288aa// 92%
TRACH3025346// Organic cation/carnitine transporter 2 (Solute carrier family 22, member 5) (High-affinity sodium-dependent carnitine cotransporter).// 1.00E-108// 195aa// 88%
TRACH3025520
TRACH3026283// ataxin 2: olivopontocerebellar ataxia 2, autosomal dominant [Homo sapiens]// 1.00E-100// 179aa// 69%
TRACH3026299// lg delta chain C region.// 0// 353aa// 92%
TRACH3026303
TRACH3026542
TRACH3026650// Actin cross-linking family protein 7 (Macrophin) (Trabeculin-alpha) (620 kDa actin-binding protein) (ABP620).// 0// 351aa// 82%
TRACH3026676
TRACH3026949
TRACH3027229// Homo sapiens diacylglycerol 0-acyltransferase homolog 2 (mouse) (DGAT2), mRNA// 1.00E-64// 252aa// 48%
TRACH3027681
TRACH3027701// phosphatidylinositol 4-kinase type II [Homo sapiens]// 1.00E-158// 265aa// 68%
TRACH3028164
TRACH3028180// Zinc finger protein 29 (Zfp-29).// 0// 530aa// 86%
TRACH3028441// Ig delta chain C region.// 0// 355aa// 92%
TRACH3028597// pallidin [Homo sapiens]// 4.00E-21// 48aa// 100%
TRACH3028837// Smoothelin.// 0// 564aa// 60%
TRACH3028855
TRACH3029139
TRACH3029329
TRACH3029462// Actin cross-linking family protein 7 (Macrophin) (Trabeculin-alpha) (620 kDa actin-binding protein) (ABP620).// 0// 735aa// 59%
TRACH3029520
TRACH3029592
TRACH3029670// Ig delta chain C region.// 0// 355aa// 92%
TRACH3030176// CDC4-like protein (Beige-like protein) (Fragment).// 1.00E-123// 278aa// 37%
TRACH3030855// Placental thrombin inhibitor (Cytoplasmic antiproteinase) (CAP) (Protease inhibitor 6).// 1.00E-157// 272aa// 94%
TRACH3031316// Ig kappa chain V-IV region B17 precursor.// 2.00E-57// 109aa// 81%
TRACH3031660// cAMP-dependent protein kinase type II-beta regulatory chain.// 0// 384aa// 92%
TRACH3031678// Natural resistance-associated macrophage protein 2 (NRAMP 2) (Divalent metal transporter 1) (DMT1).// 0// 358aa// 91%
TRACH3032044
TRACH3032150// Cysteine sulfinic acid decarboxylase (EC 4.1.1.29) (Sulfinoalanine decarboxylase) (Cysteine-sulfinate decarboxylase).// 1.00E-126// 205aa// 60%
TRACH3032480
TRACH3032570// Homo sapiens syndecan binding protein (syntenin) (SDCBP), mRNA// 1.00E-167// 296aa// 99%
TRACH3033535
TRACH3034680// Ig delta chain C region.// 1.00E-150// 293aa// 63%
TRACH3035451
TRACH3036004
TRACH3036103// Ig heavy chain V-III region VH26 precursor.// 3.00E-48// 88aa// 75%
TRACH3036278
TRACH3036750// Zinc finger protein 135.// 3.00E-94// 148aa// 48%
TRACH3036792
TRACH3036897
TRACH3036932
TRACH3037505// lg delta chain C region.// 0// 354aa// 92%
TRACH3038399// Eukaryotic translation initiation factor 2-alpha kinase 3 precursor (EC 2.7.1.-) (PRKR-like endoplasmic reticulum kinase) (Pancreatic eIF2-alpha kinase) (HsPEK).// 0// 912aa// 94%
TSTOM2000235
TSTOM2001571// Casein kinase I, delta isoform (EC 2.7.1.-) (CKI-delta).//-1.00E-144// 248aa// 93%
TSTOM2002611// thiamin pyrophosphokinase [Mus musculus]// 6.00E-64// 119aa// 65%
TSTOM2002682
TUTER1000014
TUTER2001433// lg alpha-1 chain C region.// 0// 320aa// 92%
UTERU2000300
UTERU2014998
UTERU2016464
UTERU2016669
UTERU2020226
UTERU2022955
UTERU2023941
UTERU2024042// Casein kinase I , gamma 2 isoform (EG 2.7.1.-) (CKI-gamma 2).// 9.00E-46// 88aa// 73%
UTERU2027369
UTERU2028377
UTERU2029660
UTERU2035926
UTERU2037423// Zinc finger protein 135.// 4.00E-90// 144aa// 59%
UTERU3000670// shroom [Mus musculus]// 0// 1134aa// 62%
UTERU3001029// Echinoderm microtubule-associated protein-like 4 (EMAP-4) (Restrictedly overexpressed proliferation-associated protein) (Ropp 120).// 0// 388aa// 58%
UTERU3001394
UTERU3001946// mucin 16: CA125 ovarian cancer antigen [Homo sapiens]// 0// 613aa// 89%
UTERU3004635// Neuroblast differentiation associated protein AHNAK (Desmoyokin) (Fragments).// 0// 1453aa// 78%
UTERU3005264
UTERU3005422
UTERU3006538
UTERU3006720
UTERU3007108
UTERU3009775// PAPIN [Rattus norvegicus]// 2.00E-28// 95aa// 90%
UTERU3010029
UTERU3010409// Aortic preferentially expressed protein 1 (APEG-1).// 3.00E-61// 113aa// 100%
UTERU3010604// Indoleamine 2,3-dioxygenase (EG 1.13.11.42) (IDO) (Indoleamine- pyrrole 2,3-dioxygenase).// 2.00E-38// 72aa// 55%
UTERU3010892// adaptor-related protein complex 3, delta 1 subunit: adaptin, delta [Homo sapiens]// 3.00E-48// 91aa// 100%
UTERU3010919// Serine/threonine-protein kinase NEK1 (EC 2.7.1.-) (NimA-related protein kinase 1).// 2.00E-58// 106aa// 43%
UTERU3011092
UTERU3011398// Collagen alpha 2(VI) chain precursor.// 1.00E-103// 184aa// 92%
UTERU3011558
UTERU3011579
UTERU3011837// NG-CAM related cell adhesion molecule precursor (NR-CAM) (BRAVO).// 1. 00E-47// 126aa// 61%
UTERU3012293// zinc finger protein 14 (KOX 6): GIOT-4 for gonadotropin inducible transcription repressor-4 [Homo sapiens]// 1.00E-160// 278aa// 48% UTERU3012414// Guanine nucleotide-binding protein, alpha-12 subunit (G alpha 12).// 1. 00E-116// 211aa// 89%
UTERU3012476
UTERU3012599
UTERU3012999// Homo sapiens CD27-binding (Siva) protein (SIVA), transcript variant 1, mRNA// 1.00E-56// 104aa// 100%
UTERU3013167
UTERU3013302// nuclear protein [Homo sapiens]// 0// 515aa// 92%
UTERU3014274
UTERU3014647// Sorting nexin 7.// 4.00E-26// 48aa// 44%
UTERU3014906
UTERU3015011// Paxillin.// 1.00E-168// 262aa// 93%
UTERU3015299// NADH-ubiquinone oxidoreductase 20 kDa subunit, mitochondrial precursor (EG 1. 6. 5. 3) (EG 1. 6. 99. 3) (Comp I ex I-20KD) (CI-20KD) (PSST subunit).// 1.00E-51// 97aa// 98%
UTERU3015647// Embigin precursor (Teratocarcinoma glycoprotein GP-70).// 8.00E-48// 93aa// 78%
UTERU3015844
UTERU3016070// Zinc finger protein 29 (Zfp-29).// 1.00E-94// 196aa// 39%
UTERU3016273
UTERU3016274
UTERU3016308// Smoothelin.// 0// 405aa// 65%
UTERU3017441// plakophilin 4 [Homo sapiens]// 0// 922aa// 88%
UTERU3017626// potassium voltage-gated channel, subfamily G, member 1; potassium channel KH2 [Homo sapiens]// 1.00E-119// 219aa// 87%
UTERU3017995// p47 [Homo sapiens]// 3.00E-55// 113aa// 87%
UTERU3018172
UTERU3018255
UTERU3019708// Regulator of nonsense transcripts 1 (Nonsense mRNA reducing factor 1) (NORF1) (Up-frameshift suppressor 1 homolog).// 0// 336aa// 90%
UTERU3020090
UTERU3021231// five SH3 domains [Mus musculus]// 3.00E-59// 101aa// 78%
UTERU3021850// ADAM-TS 7 precursor (EC 3.4.24.-) (A disintegrin and metalloproteinase with thrombospondin motifs 7) (ADAMTS-7) (ADAM-TS7).// 2.00E-80// 163aa// 39%
UTERU3022168// membrane bound C2 domain containing protein [Rattus norvegicus]// 0// 810aa// 77%
UTERU3022588// Cyclic-AMP-dependent transcription factor ATF-2 (Activating transcription factor 2) (cAMP response element binding protein CRE- BP1) (HB16).// 0// 415aa// 85%
UTERU3022922
UTERU3023141
UTERU3023413
ADRGL2011190// cGMP-dependent 3',5'-cyclic phosphodiesterase (EC 3.1.4.17) (Cyclic GMP stimulated phosphodiesterase) (CGS-PDE).// 0// 570aa// 97%// 000408
BRACE3002184// Zinc finger protein 85 (Zinc finger protein HPF4) (HTF1).// 0// 435aa// 76%// Q03923
BRACE3026993// plectin 1, intermediate filament binding protein; 500kD [Homo sapiens]// 4.00E-09// 107aa// 21%// NM_000445
BRACE3046450// Interleukin-16 precursor (IL-16) (Lymphocyte chemoattractant factor) (LCF).// 0// 530aa// 83%// Q14005
BRAMY3008096
BRAMY3016953
BRAWH3013197// Metabotropic glutamate receptor 4 precursor.// 0// 675aa// 91%// Q14833
BRAWH3028645// CL1BA protein [Rattus norvegicus]// 2.00E-61// 126aa// 38%// NM_022962
BRAWH3046240// Probable G protein-coupled receptor GPR35.// 1.00E-157// 273aa// 88%// Q9HC97
BRCAN2019772// Orphan nuclear receptor NR2E1 (Nuclear receptor TLX) (Tailless homolog) (Tll) (hTll) .// 0// 347aa// 92%// Q9Y466
BRHIP3030064// Homo sapiens renal tumor antigen (RAGE), mRNA// 8.00E-49// 92aa// 98%// NM_014226
BRHIP3038037
BRTHA3004432
BRTHA3024233// Probable low-affinity copper uptake protein 2 (hCTR2) (Copper transporter 2).// 4.00E-70// 128aa// 90%// 015432
CTONG2002832// Zinc finger protein 264.// 0// 430aa// 72%// 043296
CTONG2003764// Protein kinase C, delta type (EC 2.7.1.-) (NPKC-delta).// 2.00E-91// 163aa// 91%// Q05655
PLACE7013963// Nik related kinase [Mus musculus]// 0// 447aa// 46%// NM_013724
PROST2010326// G protein-coupled receptor 56; EGF-TM7-like [Homo sapiens]// 0// 463aa// 94%// NM_005682
TBAES2004105// ADAM-TS 6 precursor (EC 3.4.24.-) (A disintegrin and metalloproteinase with thrombospondin motifs 6) (ADAMTS-6) (ADAM-TS6).// 1.00E-57// 110aa// 41%// Q9UKP5
TBAES2007379// CEGP1 protein [Homo sapiens]// 0// 545aa// 67%// NM_020974
TBAES2007481// Cyclin I.// 5.00E-51// 111aa// 44%// Q9Z2V9
TBAES2008133// semaF cytoplasmic domain associated protein 3: semaphorin cytoplasmic domain-associated protein 3A [Mus musculus]// 0// 422aa// 54%// NM_018884
TESTI2043585// Olfactory receptor 5V1 (Hs6M1-21).// 1.00E-79// 141aa// 47%// Q9UGF6
TESTI2046536// Sperm histone P2 precursor (Protamine P2) (HP2, HP3, and HP4) (Basic proteins HPS1, HPS2, HPI2, and HPI1).// 1.00E-10// 33aa// 64%// P04554
TESTI4002988// Zinc finger protein 208.// 0// 311aa// 52%// 043345
TESTI4005158// Amiloride-sensitive sodium channel delta-subunit (Epithelial Na+ channel delta subunit) (Delta ENaC) (Nonvoltage-gated sodium channel 1 delta subunit) (SCNED) (Delta NaCH).// 0// 592aa// 87%// P51172
TESTI4005500// Zinc finger protein 184.// 0// 340aa// 47%// Q99676
TESTI4052089
THYMU3024602// Cell surface glycoprotein EMR1 precursor (EMR1 hormone receptor).// 0// 795aa// 86%// Q14246
THYMU3044175// Putative adenosylhomocysteinase (EC 3.3.1.1) (S-adenosyl-L-homocysteine hydrolase) (AdoHcyase).// 1.00E-161// 266aa// 93%// 043865
THYMU3046350// Sodium- and chloride-dependent betaine transporter (Na+/Cl-betaine/GABA transporter) (BGT-1).// 0// 440aa// 88%// P48065
TLIVE2007192
TRACH1000193// Orphan nuclear receptor HMR (Early response protein NAK1) (TR3 orphan receptor).// 0// 503aa// 84%// P22736
TRACH3019290// Zinc finger protein 93 (Zinc finger protein HTF34) (Fragment).// 0// 387aa// 73%// P35789
TRACH3021066// Receptor protein-tyrosine kinase ERBB-2 precursor (EC 2.7.1.112) (p185erbB2) (NEU proto-oncogene) (C-erbB-2) (Tyrosine kinase-type cell surface receptor HER2) (MLN 19).// 0// 360aa// 88%// P04626
UTERU2017492// Calcipressin 1 (Down syndrome critical region protein 1) (Myocyte- enriched calcineurin interacting protein 1) (MCIP1) (Adapt78).// 2.00E-83// 150aa// 88%// P53805
UTERU2025415
UTERU2036507// ADAM-TS 6 precursor (EC 3.4.24.-) (A disintegrin and metalloproteinase with thrombospondin motifs 6) (ADAMTS-6) (ADAM-TS6).// 6.00E-48// 104aa// 37%// Q9UKP5
UTERU3020583

## Claims

1. A polynucleotide selected from the group consisting of :
(a) a polynucleotide comprising a protein-coding region of the nucleotide sequence according to any one of SEQ ID NOs: 1-1956 and SEG ID NOs: 3913-3951;
(b) a polynucleotide comprising the nucleotide sequence encoding a polypeptide that comprises the amino acid sequence of any one of SEQ ID NOs: 1957-3912 and SEG ID NOs:3952-3990;
(c) a polynucleotide comprising a nucleotide sequence encoding a polypeptide, which comprises the amino acid sequence selected from SEQ ID NOs : SEQ ID NOs: 1957-3912 and SEQ ID NOs: 3952-3990 wherein one or more amino acids have been substituted, deleted, inserted, and/or added, and which is functionally equivalent to the polypeptide comprising the selected amino acid sequence as described above;
(d) a polynucleotide which hybridizes to a polynucleotide comprising the nucleotide sequence selected from SEG ID NOs: 1-1956 and SEQ ID NOs: 3913-3951, and which comprises the nucleotide sequence encoding a polypeptide functionally equivalent to a polypeptide encoded by the selected nucleotide sequence as described above;
(e) a polynucleotide comprising a nucleotide sequence encoding a partial amino acid sequence of a polypeptide encoded by the polynucleotides according to any one of (a)-(d);
(f) a polynucleotide comprising a nucleotide sequence having at least 70% identity to the nucleotide sequence of any one of SEQ ID NOs 1-1956 and SEQ ID NOs: 3913-3951; and
(g) a polynucleotide comprising a nucleotide sequence having at least 90% identity to the nucleotide sequence of any one of SEQ ID NOs: 1-1959 and SEQ ID NOs: 3913-3951.

2. A polypeptide encoded by the polynucleotide according to claim 1, or a partial peptide thereof.

3. An antibody which binds to the polypeptide or the peptide according to claim 2.

4. An immunoassay method for the polypeptide or the peptide according to claim 2, which comprises the steps of:
(a) contacting the polypeptide or the peptide according to claim 2 with the antibody according to claim 3; and
(b) observing the binding between the two.

5. A vector comprising the polynucleotide according to claim 1.

6. A transformant comprising the polynucleotide according to claim 1 or the vector according to claim 5.

7. A transformant which comprises the polynucleotide according to claim 1 or vector according to claim 5 in an expressible manner.

8. A method for producing the polypeptide or the peptide according to claim 2, which comprises the steps of:
(a) culturing the transformant according to claim 7; and
(b) recovering the expression product.

9. An oligonucleotide comprising 15 or more nucleotides, which comprises the nucleotide sequence according to any one of SEQ ID NOs: 1-1956 and SEQ ID NOs: 3913-3951, or a nucleotide sequence complementary to the complementary strand thereof.

10. A primer for synthesizing a polynucleotide, which comprises the oligonucleotide according to claim 9.

11. A probe for detecting a polynucleotide, which comprises the oligonucleotide according to claim 9.

12. A polynucleotide according to any one of:
(a) an antisense polynucleotide comprising a nucleotide sequence complementary to the transcript of the polynucleotide according to claim 1;
(b) a polynucleotide with the ribozyme activity for specifically cleaving the transcript of the polynucleotide according to claim 1; and
(c) a polynucleotide which downregulates the expression of the polynucleotide of claim 1 due to RNAi activity in a host cell.

13. A method for detecting the polynucleotide according to claim 1, which comprises the steps of:
(a) incubating a target polynucleotide with the oligonucleotide according to claim 9 under conditions ensuring hybridization; and
(b) detecting the hybridization between the target polynucleotide and the oligonucleotide according to claim 9.

14. A database of polynucleotides and/or polypeptides, which comprises information on at least one of the nucleotide sequences of SEQ ID NOs: 1-1956 and SEQ ID NOs: 3913-3951 and/or on at least one of the amino acid sequences of SEQ ID NOs: 1957-3912 and SEQ ID NOs: 3952-3990.
